# EUROPEAN PATENT APPLICATION

(11) **EP 4 803 148 A2**
(43) Date of publication of application: **09.09.2026**
(21) Application number: 26192608.3
(22) Date of filing: 25.03.2022
(51) Int. Cl.: A61P 35/00

(54) **HETEROARYL DERIVATIVES AS PD1/PD-L1 INHIBITORS FOR THE TREATMENT OF HBV AND CANCER**

(30) Priority: 29.03.2021 US 202163167440 P; 20.12.2021 US 202163291666 P
(62) Document number(s) of the earlier application(s) in accordance with Art. 76 EPC: 22779250.4 / 4 313 968
(71) Applicant: Arbutus Biopharma Corporation, Vancouver, BC V7Y 1B3 (CA)
(72) Inventor: AHUJA, Vijay, Princeton, 08540 (US); COLE, Andrew G., Cranbury, 08512 (US); DORSEY, Bruce D., Ambler, 19002 (US); FAN, Yi, Doylestown, 18901 (US); HEFFERNAN, Gavin D., Florence, 08518 (US); KAKARLA, Ramesh, San Diego, 92131 (US); KULTGEN, Steven G., Warminster, 18974 (US); NGUYEN, Duyan, Ambler, 19002 (US); OZTURK, Seyma, Southampton, 18966 (US); QUINTERO, Jorge, Cherry Hill, 08003 (US); SOFIA, Michael J., Doylestown, 18902 (US)
(74) Representative: Grünecker Patent- und Rechtsanwälte PartG mbB

(57) **Abstract**

**(EN)** The present disclosure provides substituted 1-ary 1-1'-heteroarvl compounds, substituted 1,1 '-biheteroaryl compounds, analogues thereof, and compositions comprising the same. In one aspect, the compounds provided can be used to treat, ameliorate, and/or prevent hepatitis B virus (HBV) and/or hepatitis D virus (HDV) infections in a patient. In another aspect, the compounds provided can be used to treat, ameliorate, and/or prevent cancer in a patient. Compounds provided are of formula (I): (I) wherein: A is (AA) or (BB)

## Description

### CROSS-REFERENCE TO RELATED APPLICATIONS

The present application claims priority under 35 U.S.C. § 119(e) to U.S. Provisional Application No. 63/167,440, filed March 29, 2021, and U.S. Provisional Application No. 63/291,666, filed December 20, 2021, all of which are hereby incorporated by reference herein in their entireties.

### BACKGROUND

Hepatitis B virus (HBV) is a noncytopathic, liver tropic DNA virus belonging to *Hepadnaviridae* family. HBV infection is one of the world's most prevalent diseases, being listed by National Institute of Allergy and Infectious Diseases (NIAID) as a High Priority Area of Interest. Although most individuals resolve the infection following acute symptoms, approximately 30% of cases become chronic. 350-400 million people worldwide are estimated to have chronic hepatitis B, leading to 0.5-1 million deaths per year, due largely to the development of hepatocellular carcinoma, cirrhosis and/or other complications.

A limited number of drugs are currently approved for the management of chronic hepatitis B, including two formulations of alpha-interferon (standard and pegylated) and five nucleoside/nucleotide analogues (lamivudine, adefovir, entecavir, telbivudine, and tenofovir) that inhibit HBV DNA polymerase. At present, the first-line treatment choices are entecavir, tenofovir and/or peg-interferon alfa-2a. However, peg-interferon alfa-2a achieves desirable serological milestones in only one third of treated patients, and is frequently associated with severe side effects. Entecavir and tenofovir are potent HBV inhibitors, but require long-term or possibly lifetime administration to continuously suppress HBV replication, and may eventually fail due to emergence of drug-resistant viruses. There is thus a pressing need for the introduction of novel, safe and effective therapies for chronic hepatitis B.

Hepatitis D virus (HDV) is a small circular enveloped RNA virus that can propagate only in the presence of HBV. In particular, HDV requires the HBV surface antigen protein to propagate itself. Infection with both HBV and HDV results in more severe complications compared to infection with HBV alone. These complications include a greater likelihood of experiencing liver failure in acute infections and a rapid progression to liver cirrhosis, with an increased chance of developing liver cancer in chronic infections. In combination with hepatitis B virus, hepatitis D has the highest mortality rate of all the hepatitis infections. The routes of transmission of HDV are similar to those for HBV. Infection is largely restricted to persons at high risk of HBV infection, particularly injecting drug users and persons receiving clotting factor concentrates.

Currently, there is no effective antiviral therapy available for the treatment of acute or chronic type D hepatitis. Interferon-alfa, given weekly for 12 to 18 months, is the only licensed treatment for hepatitis D. Response to this therapy is limited-in only about onequarter of patients is serum HDV RNA undetectable 6 months post therapy.

There is a need in the art for the identification of novel compounds that can be used to treat, ameliorate, and/or prevent HBV infection in a subject. In certain embodiments, the novel compounds can be used in patients that are HBV infected, patients who are at risk of becoming HBV infected, and/or patients that are infected with drug-resistant HBV. In other embodiments, the HBV-infected subject is further HDV-infected. The present disclosure addresses this need.

### BRIEF SUMMARY

The present disclosure provides certain compounds of formula (I), or a salt, solvate, geometric isomer, stereoisomer, or tautomer thereof, or any mixtures thereof, wherein the substituents in (I) are defined elsewhere herein:

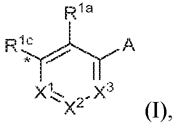

The present disclosure further provides pharmaceutical compositions comprising at least one compound of the present disclosure and at least one pharmaceutically acceptable carrier. In certain embodiments, the pharmaceutical composition further comprises at least one additional agent that treats, ameliorates, and/or prevents hepatitis virus infection.

The present disclosure further provides methods of treating, ameliorating, and/or preventing hepatitis virus infection in a subject. In certain embodiments, the method comprises administering to the subject in need thereof a therapeutically effective amount of at least one compound of the present disclosure and/or at least one pharmaceutical composition of the present disclosure. In certain embodiments, the subject is infected with hepatitis B virus (HBV). In certain embodiments, the subject is infected with hepatitis D virus (HDV). In certain embodiments, the subject is infected with HBV and HDV.

The present disclosure further provides methods of treating, ameliorating, and/or preventing cancer in a subject. In certain embodiments, the method comprises administering to the subject in need thereof a therapeutically effective amount of at least one compound of the present disclosure and/or at least one pharmaceutically acceptable composition of the present disclosure. In certain embodiments, the cancer is amenable to treatment by inhibiting PD-1, PD-L1, or the PD-1/PD-L1 interaction. In certain embodiments, the cancer is at least one of pancreatic cancer, bladder cancer, colorectal cancer, breast cancer, prostate cancer, renal cancer, hepatocellular cancer, lung cancer, ovarian cancer, cervical cancer, gastric cancer, esophageal cancer, head and neck cancer, melanoma, neuroendocrine cancer, CNS cancer, brain cancer, bone cancer, soft tissue sarcoma, non-small cell lung cancer, small-cell lung cancer, or colon cancer. In certain embodiments, the cancer is at least one of acute lymphocytic leukemia (ALL), acute myeloid leukemia (AML), chronic lymphocytic leukemia (CLL), small lymphocytic lymphoma (SLL), myelodysplastic syndrome (MDS), myeloproliferative disease (MPD), chronic myeloid leukemia (CML), multiple myeloma (MM), non-Hodgkin's lymphoma (NHL), mantle cell lymphoma (MCL), follicular lymphoma, Waldestrom's macroglobulinemia (WM), T-cell lymphoma, B-cell lymphoma and diffuse large B-cell lymphoma (DLBCL).

### DETAILED DESCRIPTION

The present disclosure relates, in certain aspects, to the discovery of certain substituted 1-aryl-1'-heteroaryl or substituted 1,1'-biheteroaryl compounds. In one aspect, the compounds of the present disclosure are useful to treat, ameliorate, and/or prevent hepatitis B virus (HBV) infection and/or hepatitis B virus-hepatitis D virus (HBV-HDV) infection and related conditions in a subject. In certain embodiments, these compounds are administered along with at least one additional agent useful for treating, ameliorating, and/or preventing the viral infection. In other embodiments, the subject is infected with HBV. In yet other embodiments, the HBV-infected subject is further infected with HDV. In another aspect, the compounds of the disclosure are useful to treat, ameliorate and/or prevent cancer and related conditions in a subject.

### Definitions

As used herein, each of the following terms has the meaning associated with it in this section. Unless defined otherwise, all technical and scientific terms used herein generally have the same meaning as commonly understood by one of ordinary skill in the art to which this disclosure belongs. Generally, the nomenclature used herein and the laboratory procedures in animal pharmacology, pharmaceutical science, separation science and organic chemistry are those well-known and commonly employed in the art. It should be understood that the order of steps or order for performing certain actions is immaterial, so long as the present teachings remain operable. Moreover, two or more steps or actions can be conducted simultaneously or not.

As used herein, the articles "a" and "an" refer to one or to more than one (*i.e.,* to at least one) of the grammatical object of the article. By way of example, "an element" means one element or more than one element.

As used herein, the term "alkenyl," employed alone or in combination with other terms, means, unless otherwise stated, a stable monounsaturated or diunsaturated straight chain or branched chain hydrocarbon group having the stated number of carbon atoms. Examples include vinyl, propenyl (or allyl), crotyl, isopentenyl, butadienyl, 1,3-pentadienyl, 1,4-pentadienyl, and the higher homologs and isomers. A functional group representing an alkene is exemplified by -CH₂-CH=CH₂.

As used herein, the term "alkoxy" employed alone or in combination with other terms means, unless otherwise stated, an alkyl group having the designated number of carbon atoms, as defined elsewhere herein, connected to the rest of the molecule via an oxygen atom, such as, for example, methoxy, ethoxy, 1-propoxy, 2-propoxy (or isopropoxy) and the higher homologs and isomers. A specific example is (C₁-C₃)alkoxy, such as, but not limited to, ethoxy and methoxy.

As used herein, the term "alkyl" by itself or as part of another substituent means, unless otherwise stated, a straight or branched chain hydrocarbon having the number of carbon atoms designated (*i.e.,* C₁-C₁₀ means one to ten carbon atoms) and includes straight, branched chain, or cyclic substituent groups. Examples include methyl, ethyl, propyl, isopropyl, butyl, isobutyl, *tert*-butyl, pentyl, neopentyl, hexyl, and cyclopropylmethyl. A specific embodiment is (C₁-C₆) alkyl, such as, but not limited to, ethyl, methyl, isopropyl, isobutyl, *n*-pentyl, *n*-hexyl and cyclopropylmethyl.

As used herein, the term "alkynyl" employed alone or in combination with other terms means, unless otherwise stated, a stable straight chain or branched chain hydrocarbon group with a triple carbon-carbon bond, having the stated number of carbon atoms. Non-limiting examples include ethynyl and propynyl, and the higher homologs and isomers. The term "propargylic" refers to a group exemplified by -CH₂-C≡CH. The term "homopropargylic" refers to a group exemplified by -CH₂CH₂-C≡CH.

As used herein, the term "aromatic" refers to a carbocycle or heterocycle with one or more polyunsaturated rings and having aromatic character, *i.e.,* having (4n+2) delocalized π (pi) electrons, where 'n' is an integer.

As used herein, the term "aryl" employed alone or in combination with other terms means, unless otherwise stated, a carbocyclic aromatic system containing one or more rings (typically one, two or three rings) wherein such rings may be attached together in a pendent manner, such as a biphenyl, or may be fused, such as naphthalene. Examples include phenyl, anthracyl and naphthyl. Aryl groups also include, for example, phenyl or naphthyl rings fused with one or more saturated or partially saturated carbon rings (e.g., bicyclo[4.2.0]octa-1,3,5-trienyl, or indanyl), which can be substituted at one or more carbon atoms of the aromatic and/or saturated or partially saturated rings.

As used herein, the term "aryl-(C₁-C₆)alkyl" refers to a functional group wherein a one to six carbon alkylene chain is attached to an aryl group, *e.g*., -CH₂CH₂-phenyl or -CH₂-phenyl (or benzyl). Specific examples are aryl-CH₂- and aryl-CH(CH₃)-. The term "substituted aryl-(C₁-C₆)alkyl" refers to an aryl-(C₁-C₆)alkyl functional group in which the aryl group is substituted. A specific example is substituted aryl(CH₂)-. Similarly, the term "heteroaryl-(C₁-C₆)alkyl" refers to a functional group wherein a one to three carbon alkylene chain is attached to a heteroaryl group, *e.g*., -CH₂CH₂-pyridyl. A specific example is heteroaryl-(CH₂)-. The term "substituted heteroaryl-(C₁-C₆)alkyl" refers to a heteroaryl-(C₁-C₆)alkyl functional group in which the heteroaryl group is substituted. A specific example is substituted heteroaryl-(CH₂)-.

In one aspect, the terms "co-administered" and "co-administration" as relating to a subject refer to administering to the subject a compound and/or composition of the disclosure along with a compound and/or composition that may also treat or prevent a disease or disorder contemplated herein. In certain embodiments, the co-administered compounds and/or compositions are administered separately, or in any kind of combination as part of a single therapeutic approach. The co-administered compound and/or composition may be formulated in any kind of combinations as mixtures of solids and liquids under a variety of solid, gel, and liquid formulations, and as a solution.

As used herein, the term "cycloalkyl" by itself or as part of another substituent refers to, unless otherwise stated, a cyclic chain hydrocarbon having the number of carbon atoms designated (*i.e.,* C₃-C₆ refers to a cyclic group comprising a ring group consisting of three to six carbon atoms) and includes straight, branched chain or cyclic substituent groups. Examples of (C₃-C₆)cycloalkyl groups are cyclopropyl, cyclobutyl, cyclopentyl and cyclohexyl. Cycloalkyl rings can be optionally substituted. Non-limiting examples of cycloalkyl groups include: cyclopropyl, 2-methyl-cyclopropyl, cyclopropenyl, cyclobutyl, 2,3-dihydroxycyclobutyl, cyclobutenyl, cyclopentyl, cyclopentenyl, cyclopentadienyl, cyclohexyl, cyclohexenyl, cycloheptyl, cyclooctanyl, decalinyl, 2,5-dimethylcyclopentyl, 3,5-dichlorocyclohexyl, 4-hydroxycyclohexyl, 3,3,5-trimethylcyclohex-1-yl, octahydropentalenyl, octahydro-1*H*-indenyl, 3a,4,5,6,7,7a-hexahydro-3*H*-inden-4-yl, decahydroazulenyl; bicyclo[6.2.0]decanyl, decahydronaphthalenyl, and dodecahydro-1*H-*fluorenyl. The term "cycloalkyl" also includes bicyclic hydrocarbon rings, non-limiting examples of which include, bicyclo-[2.1.1]hexanyl, bicyclo[2.2.1]heptanyl, bicyclo[3.1.1]heptanyl, 1,3-dimethyl[2.2.1] heptan-2-yl, bicyclo[2.2.2]octanyl, and bicyclo[3.3.3]undecanyl.

As used herein, the term "DCM" refers to dichloromethane.

As used herein, a "disease" is a state of health of a subject wherein the subject cannot maintain homeostasis, and wherein if the disease is not ameliorated then the subject's health continues to deteriorate.

As used herein, a "disorder" in a subject is a state of health in which the subject is able to maintain homeostasis, but in which the subject's state of health is less favorable than it would be in the absence of the disorder. Left untreated, a disorder does not necessarily cause a further decrease in the subject's state of health.

As used herein, the term "halide" refers to a halogen atom bearing a negative charge. The halide anions are fluoride (F⁻), chloride (Cl⁻), bromide (Br⁻), and iodide (I⁻).

As used herein, the term "halo" or "halogen" alone or as part of another substituent refers to, unless otherwise stated, a fluorine, chlorine, bromine, or iodine atom.

As used herein, the term "Hepatitis B virus" (or HBV) refers to a virus species of the genus Orthohepadnavirus, which is a part of the Hepadnaviridae family of viruses, and that is capable of causing liver inflammation in humans.

As used herein, the term "Hepatitis D virus" (or HDV) refers to a virus species of the genus Deltaviridae, which is capable of causing liver inflammation in humans. The HDV particle comprises an envelope, which is provided by HBV and surrounds the RNA genome and the HDV antigen. The HDV genome is a single, negative stranded, circular RNA molecule nearly 1.7 kb in length. The genome contains several sense and antisense open reading frames (ORFs), only one of which is functional and conserved. The RNA genome is replicated through an RNA intermediate, the antigenome. The genomic RNA and its complement, the antigenome, can function as ribozymes to carry out self-cleavage and selfligation reactions. A third RNA present in the infected cell, also complementary to the genome, but 800 bp long and polyadenylated, is the mRNA for the synthesis of the delta antigen (HDAg).

As used herein, the term "heteroalkenyl" by itself or in combination with another term refers to, unless otherwise stated, a stable straight or branched chain monounsaturated or diunsaturated hydrocarbon group consisting of the stated number of carbon atoms and one or two heteroatoms selected from the group consisting of O, N, and S, and wherein the nitrogen and sulfur atoms may optionally be oxidized and the nitrogen heteroatom may optionally be quaternized. Up to two heteroatoms may be placed consecutively. Examples include - CH=CH-O-CH₃, -CH=CH-CH₂-OH, -CH₂-CH=N-OCH₃, -CH=CH-N(CH₃)-CH₃, and -CH₂-CH=CH-CH₂-SH.

As used herein, the term "heteroalkyl" by itself or in combination with another term refers to, unless otherwise stated, a stable straight or branched chain alkyl group consisting of the stated number of carbon atoms and one or two heteroatoms selected from the group consisting of O, N, and S, and wherein the nitrogen and sulfur atoms may be optionally oxidized and the nitrogen heteroatom may be optionally quaternized. The heteroatom(s) may be placed at any position of the heteroalkyl group, including between the rest of the heteroalkyl group and the fragment to which it is attached, as well as attached to the most distal carbon atom in the heteroalkyl group. Examples include: -OCH₂CH₂CH₃, - CH₂CH₂CH₂OH, -CH₂CH₂NHCH₃, -CH₂SCH₂CH₃, and -CH₂CH₂S(=O)CH₃. Up to two heteroatoms may be consecutive, such as, for example, -CH₂NH-OCH₃, or -CH₂CH₂SSCH₃.

As used herein, the term "heteroaryl" or "heteroaromatic" refers to a heterocycle having aromatic character. A polycyclic heteroaryl may include one or more rings that are partially saturated. Examples include tetrahydroquinoline and 2,3-dihydrobenzofuryl.

As used herein, the term "heterocycle" or "heterocyclyl" or "heterocyclic" by itself or as part of another substituent refers to, unless otherwise stated, an unsubstituted or substituted, stable, mono- or multi-cyclic heterocyclic ring system that comprises carbon atoms and at least one heteroatom selected from the group consisting of N, O, and S, and wherein the nitrogen and sulfur heteroatoms may be optionally oxidized, and the nitrogen atom may be optionally quaternized. The heterocyclic system may be attached, unless otherwise stated, at any heteroatom or carbon atom that affords a stable structure. A heterocycle may be aromatic or non-aromatic in nature. In certain embodiments, the heterocycle is a heteroaryl.

Examples of non-aromatic heterocycles include monocyclic groups such as aziridine, oxirane, thiirane, azetidine, oxetane, thietane, pyrrolidine, pyrroline, imidazoline, pyrazolidine, dioxolane, sulfolane, 2,3-dihydrofuran, 2,5-dihydrofuran, tetrahydrofuran, thiophane, piperidine, 1,2,3,6-tetrahydropyridine, 1,4-dihydropyridine, piperazine, morpholine, thiomorpholine, pyran, 2,3-dihydropyran, tetrahydropyran, 1,4-dioxane, 1,3-dioxane, homopiperazine, homopiperidine, 1,3-dioxepane, 4,7-dihydro-1,3-dioxepin and hexamethyleneoxide.

Examples of heteroaryl groups include pyridyl, pyrazinyl, pyrimidinyl (such as, but not limited to, 2- and 4-pyrimidinyl), pyridazinyl, thienyl, furyl, pyrrolyl, imidazolyl, thiazolyl, oxazolyl, pyrazolyl, isothiazolyl, 1,2,3-triazolyl, 1,2,4-triazolyl, 1,3,4-triazolyl, tetrazolyl, 1,2,3-thiadiazolyl, 1,2,3-oxadiazolyl, 1,3,4-thiadiazolyl and 1,3,4-oxadiazolyl.

Examples of polycyclic heterocycles include indolyl (such as, but not limited to, 3-, 4-, 5-, 6- and 7-indolyl), indolinyl, quinolyl, tetrahydroquinolyl, isoquinolyl (such as, but not limited to, 1- and 5-isoquinolyl), 1,2,3,4-tetrahydroisoquinolyl, cinnolinyl, quinoxalinyl (such as, but not limited to, 2- and 5-quinoxalinyl), quinazolinyl, phthalazinyl, 1,8-naphthyridinyl, 1,4-benzodioxanyl, coumarin, dihydrocoumarin, 1,5-naphthyridinyl, benzofuryl (such as, but not limited to, 3-, 4-, 5-, 6- and 7-benzofuryl), 2,3-dihydrobenzofuryl, 1,2-benzisoxazolyl, benzothienyl (such as, but not limited to, 3-, 4-, 5-, 6-, and 7-benzothienyl), benzoxazolyl, benzothiazolyl (such as, but not limited to, 2-benzothiazolyl and 5-benzothiazolyl), purinyl, benzimidazolyl, benztriazolyl, thioxanthinyl, carbazolyl, carbolinyl, acridinyl, pyrrolizidinyl, and quinolizidinyl.

The aforementioned listing of heterocyclyl and heteroaryl moieties is intended to be representative and not limiting.

As described herein, the term "PD-L1 inhibitor" includes any compound that is capable of inhibiting the expression and/or function of the protein Programmed Death-Ligand 1 (PD-L1) either directly or indirectly. PD-L1, also known as cluster of differentiation 274 (CD274) or B7 homolog 1 (B7-H1), is a type 1 transmembrane protein that plays a major role in suppressing the adaptive arm of immune system during pregnancy, tissue allograft transplants, autoimmune disease, and hepatitis. PD-L1 binds to its receptor, the inhibitory checkpoint molecule PD-1 (which is found on activated T cells, B cells, and myeloid cells) so as to modulate activation or inhibition of the adaptive arm of immune system. In certain embodiments, the PD-L1 inhibitor inhibits the expression and/or function of PD-L1 by at least 5%, at least 10%, at least 20%, at least 50%, at least 75%, or at least 90%.

As used herein, the term "pharmaceutical composition" or "composition" refers to a mixture of at least one compound useful within the disclosure with a pharmaceutically acceptable carrier. The pharmaceutical composition facilitates administration of the compound to a subject.

As used herein, the term "pharmaceutically acceptable" refers to a material, such as a carrier or diluent, which does not abrogate the biological activity or properties of the compound useful within the disclosure, and is relatively non-toxic, *i.e.,* the material may be administered to a subject without causing undesirable biological effects or interacting in a deleterious manner with any of the components of the composition in which it is contained.

As used herein, the term "pharmaceutically acceptable carrier" means a pharmaceutically acceptable material, composition or carrier, such as a liquid or solid filler, stabilizer, dispersing agent, suspending agent, diluent, excipient, thickening agent, solvent or encapsulating material, involved in carrying or transporting a compound useful within the disclosure within or to the subject such that it may perform its intended function. Typically, such constructs are carried or transported from one organ, or portion of the body, to another organ, or portion of the body. Each carrier must be "acceptable" in the sense of being compatible with the other ingredients of the formulation, including the compound useful within the disclosure, and not injurious to the subject. Some examples of materials that may serve as pharmaceutically acceptable carriers include: sugars, such as lactose, glucose and sucrose; starches, such as corn starch and potato starch; cellulose, and its derivatives, such as sodium carboxymethyl cellulose, ethyl cellulose and cellulose acetate; powdered tragacanth; malt; gelatin; talc; excipients, such as cocoa butter and suppository waxes; oils, such as peanut oil, cottonseed oil, safflower oil, sesame oil, olive oil, corn oil and soybean oil; glycols, such as propylene glycol; polyols, such as glycerin, sorbitol, mannitol and polyethylene glycol; esters, such as ethyl oleate and ethyl laurate; agar; buffering agents, such as magnesium hydroxide and aluminum hydroxide; surface active agents; alginic acid; pyrogen-free water; isotonic saline; Ringer's solution; ethyl alcohol; phosphate buffer solutions; and other non-toxic compatible substances employed in pharmaceutical formulations. As used herein, "pharmaceutically acceptable carrier" also includes any and all coatings, antibacterial and antifungal agents, and absorption delaying agents, and the like that are compatible with the activity of the compound useful within the disclosure, and are physiologically acceptable to the subject. Supplementary active compounds may also be incorporated into the compositions. The "pharmaceutically acceptable carrier" may further include a pharmaceutically acceptable salt of the compound useful within the disclosure. Other additional ingredients that may be included in the pharmaceutical compositions used in the practice of the disclosure are known in the art and described, for example in Remington's Pharmaceutical Sciences (Genaro, Ed., Mack Publishing Co., 1985, Easton, PA), which is incorporated herein by reference.

As used herein, the language "pharmaceutically acceptable salt" refers to a salt of the administered compound prepared from pharmaceutically acceptable non-toxic acids and/or bases, including inorganic acids, inorganic bases, organic acids, inorganic bases, solvates (including hydrates) and clathrates thereof.

As used herein, a "pharmaceutically effective amount," "therapeutically effective amount," or "effective amount" of a compound is that amount of compound that is sufficient to provide a beneficial effect to the subject to which the compound is administered.

The term "prevent," "preventing," or "prevention" as used herein means avoiding or delaying the onset of symptoms associated with a disease or condition in a subject that has not developed such symptoms at the time the administering of an agent or compound commences. Disease, condition and disorder are used interchangeably herein.

By the term "specifically bind" or "specifically binds" as used herein is meant that a first molecule preferentially binds to a second molecule (*e.g*., a particular receptor or enzyme), but does not necessarily bind only to that second molecule.

As used herein, the terms "subject" and "individual" and "patient" can be used interchangeably and may refer to a human or non-human mammal or a bird. Non-human mammals include, for example, livestock and pets, such as ovine, bovine, porcine, canine, feline and murine mammals. In certain embodiments, the subject is human.

As used herein, the term "substituted" refers to that an atom or group of atoms has replaced hydrogen as the substituent attached to another group.

As used herein, the term "substituted alkyl," "substituted cycloalkyl," "substituted alkenyl," or "substituted alkynyl" refers to alkyl, cycloalkyl, alkenyl or alkynyl, as defined elsewhere herein, substituted by one, two or three substituents independently selected from the group consisting of halogen, -OH, alkoxy, tetrahydro-2-H-pyranyl, -NH₂, -NH(C₁-C₆ alkyl), -N(C₁-C₆ alkyl)₂, 1-methyl-imidazol-2-yl, pyridin-2-yl, pyridin-3-yl, pyridin-4-yl, - C(=O)OH, -C(=O)O(C₁-C₆)alkyl, trifluoromethyl, -C≡N, -C(=O)NH₂, -C(=O)NH(C₁-C₆)alkyl, -C(=O)N((C₁-C₆)alkyl)₂, -SO₂NH₂, -SO₂NH(C₁-C₆ alkyl), -SO₂N(C₁-C₆ alkyl)₂, - C(=NH)NH₂, and -NO₂, in certain embodiments containing one or two substituents independently selected from halogen, -OH, alkoxy, -NH₂, trifluoromethyl, -N(CH₃)₂, and - C(=O)OH, in certain embodiments independently selected from halogen, alkoxy and -OH. Examples of substituted alkyls include, but are not limited to, 2,2-difluoropropyl, 2-carboxycyclopentyl and 3-chloropropyl.

For aryl, aryl-(C₁-C₃)alkyl and heterocyclyl groups, the term "substituted" as applied to the rings of these groups refers to any level of substitution, namely mono-, di-, tri-, tetra-, or penta-substitution, where such substitution is permitted. The substituents are independently selected, and substitution may be at any chemically accessible position. In certain embodiments, the substituents vary in number between one and four. In other embodiments, the substituents vary in number between one and three. In yet another embodiments, the substituents vary in number between one and two. In yet other embodiments, the substituents are independently selected from the group consisting of C₁-C₆ alkyl, -OH, C₁-C₆ alkoxy, halo, amino, acetamido and nitro. As used herein, where a substituent is an alkyl or alkoxy group, the carbon chain may be branched, straight or cyclic.

In certain embodiments, each occurrence of alkyl or cycloalkyl is independently optionally substituted with at least one substituent selected from the group consisting of C₁-C₆ alkyl, halo, -OR, phenyl (thus yielding, in non-limiting examples, optionally substituted phenyl-(C₁-C₃ alkyl), such as, but not limited to, benzyl or substituted benzyl) and -N(R)(R), wherein each occurrence of R is independently H, C₁-C₆ alkyl or C₃-C₈ cycloalkyl. In other embodiments, each occurrence of aryl or heteroaryl is independently optionally substituted with at least one substituent selected from the group consisting of C₁-C₆ alkyl, C₁-C₆ haloalkyl, C₁-C₆ haloalkoxy, halo, -CN, -OR, -N(R)(R), -NO₂, -S(=O)₂N(R)(R), acyl, and C₁-C₆ alkoxycarbonyl, wherein each occurrence of R is independently H, C₁-C₆ alkyl or C₃-C₈ cycloalkyl. In yet other embodiments, each occurrence of aryl or heteroaryl is independently optionally substituted with at least one substituent selected from the group consisting of C₁-C₆ alkyl, C₁-C₆ haloalkyl, C₁-C₆ haloalkoxy, halo, -CN, -OR, -N(R)(R), and C₁-C₆ alkoxycarbonyl, wherein each occurrence of R is independently H, C₁-C₆ alkyl or C₃-C₈ cycloalkyl.

Unless otherwise noted, when two substituents are taken together to form a ring having a specified number of ring atoms (*e.g.,* R² and R³ taken together with the nitrogen to which they are attached to form a ring having from 3 to 7 ring members), the ring can have carbon atoms and optionally one or more (*e.g.,* 1 to 3) additional heteroatoms independently selected from nitrogen, oxygen, or sulfur. The ring can be saturated or partially saturated, and can be optionally substituted with one or more substituents, non-limiting examples including a carbonyl (C=O).

Whenever a term or either of their prefix roots appear in a name of a substituent the name is to be interpreted as including those limitations provided herein. For example, whenever the term "alkyl" or "aryl" or either of their prefix roots appear in a name of a substituent (e.g., arylalkyl, alkylamino) the name is to be interpreted as including those limitations given elsewhere herein for "alkyl" and "aryl" respectively.

In certain embodiments, substituents of compounds are disclosed in groups or in ranges. It is specifically intended that the description include each and every individual subcombination of the members of such groups and ranges. For example, the term "C₁₋₆ alkyl" is specifically intended to individually disclose C₁, C₂, C₃, C₄, C₅, C₆, C₁-C₆, C₁-C₅, C₁-C₄, C₁-C₃, C₁-C₂, C₂-C₆, C₂-C₅, C₂-C₄, C₂-C₃, C₃-C₆, C₃-C₅, C₃-C₄, C₄-C₆, C₄-C₅, and C₅-C₆ alkyl.

The terms "treat," "treating," and "treatment," as used herein, means reducing the frequency or severity with which symptoms of a disease or condition are experienced by a subject by virtue of administering an agent or compound to the subject.

Ranges: throughout this disclosure, various aspects of the disclosure can be presented in a range format. It should be understood that the description in range format is merely for convenience and brevity and should not be construed as an inflexible limitation on the scope of the disclosure. Accordingly, the description of a range should be considered to have specifically disclosed all the possible sub-ranges as well as individual numerical values within that range. For example, description of a range such as from 1 to 6 should be considered to have specifically disclosed sub-ranges such as from 1 to 3, from 1 to 4, from 1 to 5, from 2 to 4, from 2 to 6, from 3 to 6 etc., as well as individual and partial numbers within that range, for example, 1, 2, 2.7, 3, 4, 5, 5.3, and 6. This applies regardless of the breadth of the range.

### Compounds

Programmed death-ligand 1 (PD-L1), which is also known as cluster of differentiation 274 (CD274) or B7 homolog 1 (B7-H1), is a human transmembrane protein that plays a major role in suppressing the immune system as needed. Generally, the presence of a foreign antigen in the body triggers proliferation of antigen-specific CD8+ T cells in the lymph nodes. However, binding of PD-L1 to the receptor programmed cell death protein 1 (PD-1) or B7.1 membrane protein (both of which are found on activated T cells, B cells, and myeloid cells), transmits an inhibitory signal, which reduces proliferation of the CD8+ T cells in the lymph nodes. Such interaction effectively suppresses the immune response and avoids detection and destruction of the antigens.

In certain embodiments, small-molecule immunomodulators targeting the PD-1/PD-L1 signaling pathway are used to treat, ameliorate, and/or prevent hepatitis B virus (HBV) infection and related conditions in a subject. In other embodiments, inhibition of PDL-1 enhances the immune response to at least one HBV antigen.

The disclosure includes a compound of formula (I), or a salt, solvate, prodrug, stereoisomer (such as, in a non-limiting example, an enantiomer or diastereoisomer, and any mixtures thereof, such as, in a non-limiting example, mixtures in any proportion of enantiomers and/or diastereoisomers thereof), tautomer, and/or geometric isomer, and any mixtures thereof. It should be noted that the absolute stereochemistry of the chiral center(s) represented in any structure depicted herein and/or compound named herein is merely illustrative and non-limiting.

In certain embodiments, the compound of formula (I) is: 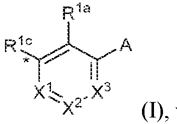 wherein: 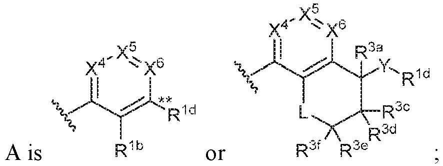
Y is NR^{3b} or O;
L is selected from the group consisting of -C(R^{3g})(R^{3h})- -(C(R^{3g})(R^{3h}))-(C(R³ⁱ)(R^{3j}))-, and a bond;
X¹ is selected from the group consisting of CR^{'1} and N;
X² is selected from the group consisting of CR^{'2} and N;
X³ is selected from the group consisting of CR^{'3} and N;
X⁴ is selected from the group consisting of CR^{"1} and N;
X⁵ is selected from the group consisting of CR^{"2} and N;
X⁶ is selected from the group consisting of CR^{"3} and N;
   wherein one to four of X¹, X², X³, X⁴, X⁵, and X⁶ is N;
R^{'1}, R^{'2}, R^{'3}, R^{"1}, R^{"2}, and R^{"3} are each independently selected from the group consisting of H, halogen, C₁-C₃ alkyl, C₃-C₈ cycloalkyl, C₁-C₃ alkoxy, cyano, C₁-C₃ haloalkyl, and C₁-C₃ haloalkoxy;
R^{1a} and R^{1b} are each independently selected from the group consisting of H, halogen, C₁-C₃ alkyl, C₃-C₈ cycloalkyl, C₁-C₃ alkoxy, cyano, C₁-C₃ haloalkyl, and C₁-C₃ haloalkoxy;
R^{1c} and R^{1d} are each independently selected from the group consisting of 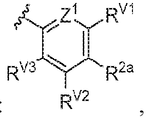 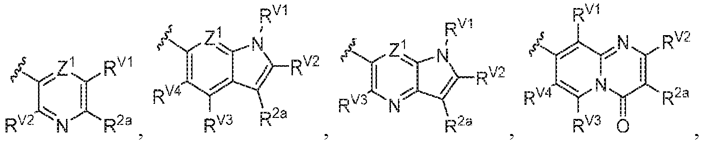 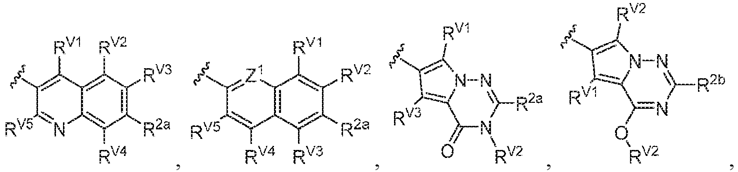 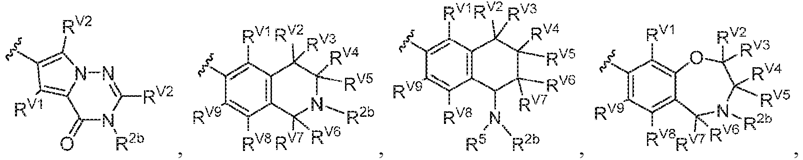 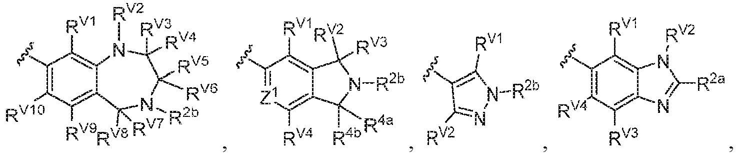 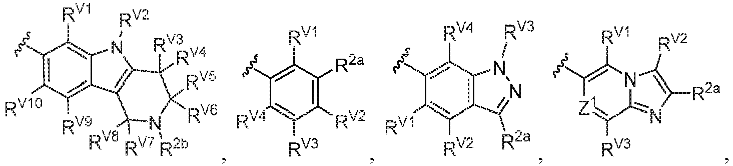 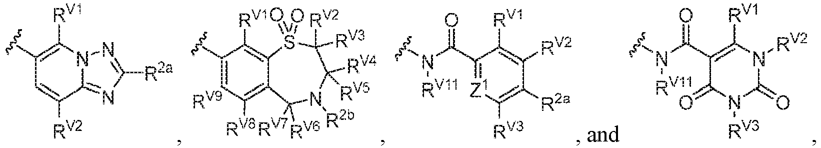 wherein each occurrence of Z¹ is CR^{V10} or N;
R^{2a} is selected from the group consisting of C(O)OR¹, -C(=O)NR⁶R⁶, optionally substituted heterocyclyl, -(CH₂)₁₋₃(optionally substituted heterocyclyl), optionally substituted C₁-C₆ alkoxy, optionally substituted C₁-C₆ aminoalkyl, C(=O)(optionally substituted C₁-C₆ alkyl), and optionally substituted C₁-C₆ hydroxyalkyl,
   wherein each optional substituent in R^{2a} is independently selected from the group consisting of optionally substituted heterocyclyl, optionally substituted C₁-C₆ alkyl, optionally substituted C₁-C₆ hydroxyalkyl, optionally substituted cycloalkyl, and optionally substituted -(CH₂)₁₋₂(heterocyclyl),
   wherein two optional substituents in R^{2a} may combine with the atoms to which they bound to form an optionally substituted C₂-C₈ heterocyclyl or optionally substituted C₃-C₈ cycloalkyl,
   wherein each occurrence of R^{I} is independently H, C₁-C₆ alkyl, or C₃-C₈ cycloalkyl, wherein the alkyl or cycloalkyl is independently optionally substituted with at least one of halogen, -OH, C₁-C₆ alkoxy, -NH₂, -NH(C₁-C₆ alkyl), and -N(C₁-C₆ alkyl)(C₁-C₆ alkyl),
   wherein each occurrence of R⁶ is independently H, C₁-C₆ alkyl, or C₃-C₈ cycloalkyl, wherein the alkyl or cycloalkyl is independently optionally substituted with at least one of halogen, -OH, C₁-C₆ alkoxy, -NH₂, -NH(C₁-C₆ alkyl), and -N(C₁-C₆ alkyl)(C₁-C₆ alkyl),
R^{2b} is selected from the group consisting of H, -(CH₂)₁₋₃C(=O)OR^{I}, -(CH₂)₁₋₃C(=O)NR^{I}R^{I}, optionally substituted heterocyclyl, -(CH₂)₁₋₂(optionally substituted heterocyclyl), optionally substituted C₁-C₆ alkyl, optionally substituted C₁-C₆ haloalkyl, optionally substituted C₁-C₆ alkoxy, optionally substituted C₁-C₆ aminoalkyl, and optionally substituted C₁-C₆ hydroxyalkyl,
   wherein each optional substituent in R^{2b} is independently selected from the group consisting of optionally substituted C₁-C₆ alkyl, optionally substituted heterocyclyl, optionally substituted cycloalkyl, and optionally substituted -(CH₂)₁₋₃(heterocyclyl);
R^{3a}, R^{3b}, R^{3c}, R^{3d}, R^{3e}, R^{3f}, R^{3g}, R^{3h}, R³ⁱ, and R^{3j} are each independently selected from the group consisting of H, halogen, C₁-C₃ alkyl, C₃-C₈ cycloalkyl, C₁-C₃ alkoxy, C₁-C₃ haloalkyl, and C₁-C₃ haloalkoxy;
R⁵ is selected from the group consisting of H, C₁-C₃ alkyl, C₃-C₈ cycloalkyl, C₁-C₃ alkoxy, C₁-C₃ haloalkyl, and C₁-C₃ haloalkoxy,
   wherein R⁵ and R^{2b} may combine with the nitrogen atom to which they are bound to form a C₃-C₁₂ heterocyclyl;
each occurrence of R^{4a} and R^{4b} is independently selected from the group consisting of H, C₁-C₃ alkyl, C₃-C₈ cycloalkyl, C₁-C₃ alkoxy, C₁-C₃ haloalkyl, and C₁-C₃ haloalkoxy,
   wherein R^{4a} and R^{4b} may combine with the carbon atom to which they are bound to form a carbonyl (C=O);
each occurrence of R^{V1}, R^{V2}, R^{V3}, R^{V4}, R^{V5}, R^{V6}, R^{V7}, R^{V8}, R^{V9}, and R^{V10} is independently selected from the group consisting of H, C₁-C₃ alkyl, C₃-C₈ cycloalkyl, C₁-C₃ alkoxy, cyano, halogen, C₁-C₃ haloalkyl, and C₁-C₃ haloalkoxy; and
R^{V11} is selected from the group consisting of H, C₁-C₃ alkyl, C₃-C₈ cycloalkyl, C₁-C₃ alkoxy, C₁-C₃ haloalkyl, and C₁-C₃ haloalkoxy.

In certain embodiments, if R^{1c} and R^{1d} are identical, then at least one of the following applies:
(a) if X¹ is N, then Z¹ in R^{1c}, if present, is CR^{V10}, and;
(b) if X⁶ is N, then Z¹ in R^{1d}, if present, is CR^{V10}.

In certain embodiments, at least one of the following applies:
(a) if X¹ is N, then Z¹ in R¹⁰, if present, is CR^{V10}, and;
(b) if X⁶ is N, then Z¹ in R^{1d}, if present, is CR^{V10}.

In certain embodiments, if X³ is N, then X⁴ is CR^{"1}. In certain embodiments, if X⁴ is N, then X³ is CR^{'3}.

In certain embodiments, if X¹ is N, then R^{1c} is not a heteroaryl group with a ring nitrogen atom adjacent to the * bond.

In certain embodiments, if X⁶ is N, then R^{1d} is not a heteroaryl group with a ring nitrogen atom adjacent to the ** bond.

In certain embodiments, the compound of formula (I) is:

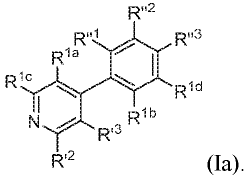

In certain embodiments, the compound of formula (I) is:

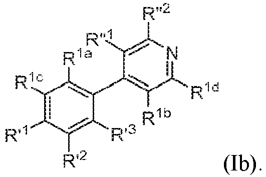

In certain embodiments, the compound of formula (I) is:

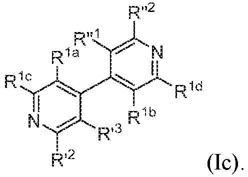

In certain embodiments, the compound of formula (I) is:

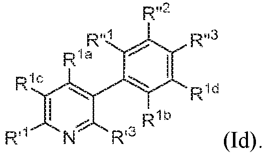

In certain embodiments, the compound of formula (I) is:

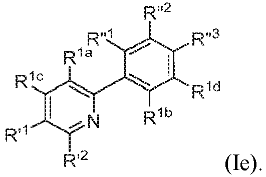

In certain embodiments, the compound of formula (I) is

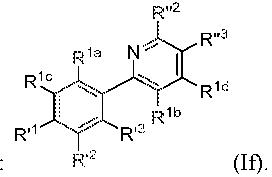

In certain embodiments, the compound of formula (I) is

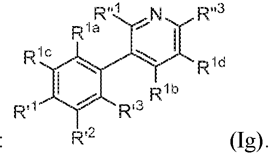

In certain embodiments, the compound of formula (I) is:

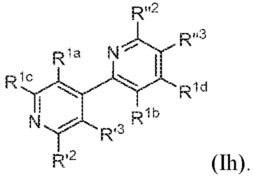

In certain embodiments, the compound of formula (I) is:

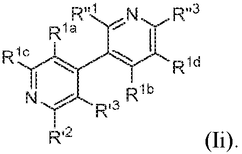

In certain embodiments, the compound of formula (I) is:

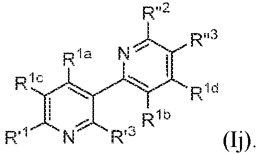

In certain embodiments, the compound of formula (I) is:

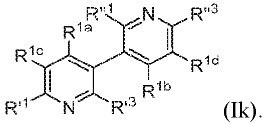

In certain embodiments, the compound of formula (I) is:

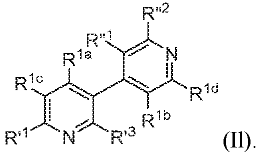

In certain embodiments, the compound of formula (I) is: 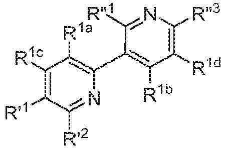 (Im).

In certain embodiments, the compound of formula (I) is:

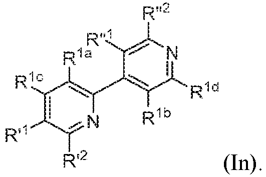

In certain embodiments, the compound of formula (I) is:

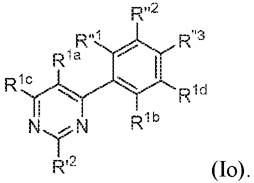

In certain embodiments, the compound of formula (I) is:

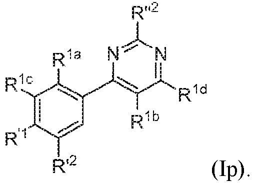

In certain embodiments, the compound of formula (I) is 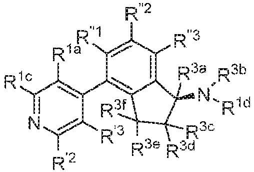 (Iq).

In certain embodiments, the compound of formula (I) is 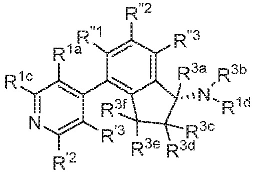 (Ir).

In certain embodiments, the compound of formula (I) is: 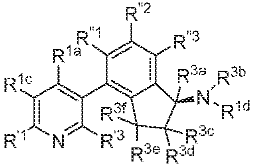 (Is).

In certain embodiments, the compound of formula (I) is: 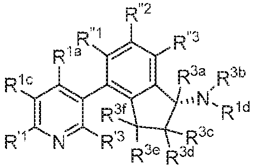 (It).

In certain embodiments, the compound of formula (I) is: 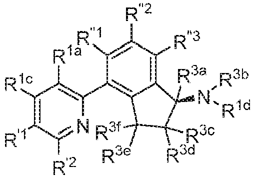 (Iu).

In certain embodiments, the compound of formula (I) is: 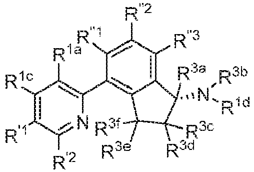 (Iv).

In certain embodiments, the compound of formula (I) is: 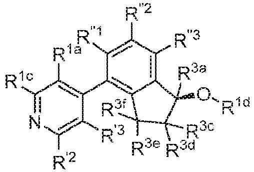 (Iw).

In certain embodiments, the compound of formula (I) is: 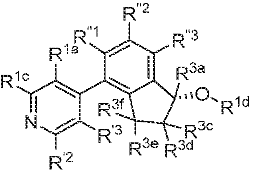 (Ix).

In certain embodiments, the compound of formula (I) is: 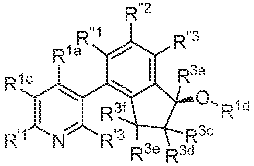 (Iy).

In certain embodiments, the compound of formula (I) is: 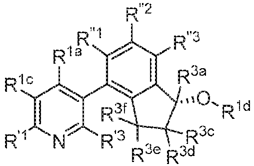 (Iz).

In certain embodiments, the compound of formula (I) is: 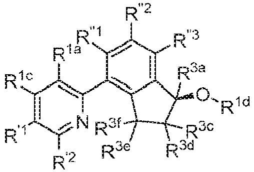 (Iaa).

In certain embodiments, the compound of formula (I) is: 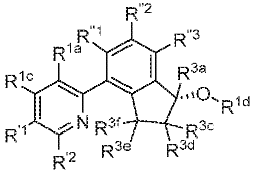 (Iab).

In certain embodiments, the compound of formula (I) is: 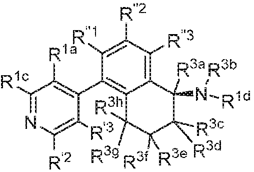 (Iac).

In certain embodiments, the compound of formula (I) is: 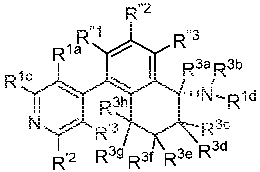 (Iad).

In certain embodiments, the compound of formula (I) is: 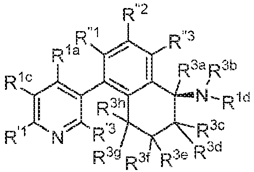 (Iae).

In certain embodiments, the compound of formula (I) is: 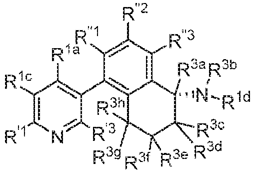 (Iaf).

In certain embodiments, the compound of formula (I) is: 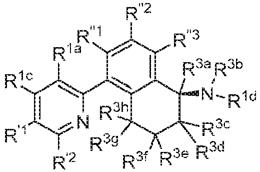 (Iag).

In certain embodiments, the compound of formula (I) is: 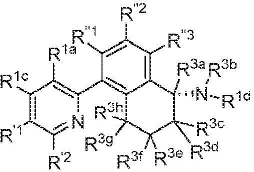 (Iah).

In certain embodiments, the compound of formula (I) is: 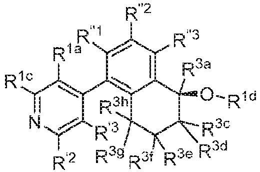 (Iai).

In certain embodiments, the compound of formula (I) is: 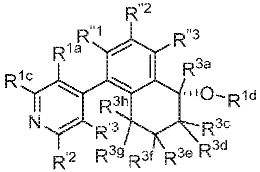 (Iaj).

In certain embodiments, the compound of formula (I) is: 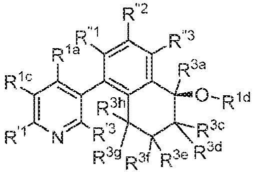 (Iak).

In certain embodiments, the compound of formula (I) is: 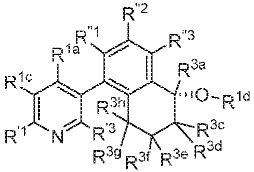 (Ial).

In certain embodiments, the compound of formula (I) is: 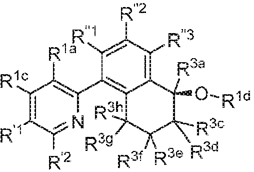 (Iam).

In certain embodiments, the compound of formula (I) is: 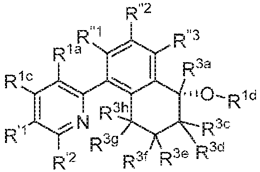 (Ian).

In certain embodiments, each of R^{'2}, R^{'3}, R^{"1}, R^{"2}, and R^{"3} are H, and X¹ is N. In certain embodiments, each of R^{'1}, R^{'3}, R^{"1}, R^{"2}, and R^{"3} are H, and X² is N. In certain embodiments, each of R^{'1}, R^{'2}, R^{"1}, R^{"2}, and R^{"3} are H, and X³ is N. In certain embodiments, each of R^{'1}, R^{'2}, R^{'3}, R^{"2}, and R^{"3} are H, and X⁴ is N. In certain embodiments, each of R^{'1}, R^{'2}, R^{'3}, R^{"1}, and R^{"3} are H, and X⁵ is N. In certain embodiments, each of R^{'1}, R^{'2}, R^{'3}, R^{"1}, and R^{"2} are H, and X⁶ is N. In certain embodiments, each of R^{'2}, R^{'3}, R^{"2}, and R^{"3} are H, and X¹ and X⁴ are N. In certain embodiments, each of R^{'2}, R^{'3}, R^{"1}, and R^{"3} are H, and X¹ and X⁵ are N. In certain embodiments, each of R^{'2}, R^{'3}, R^{"1}, and R^{"2} are H, and X¹ and X⁶ are N. In certain embodiments, each of R^{'1}, R^{'3}, R^{"2}, and R^{"3} are H, and X² and X⁴ are N. In certain embodiments, each of R^{'1}, R^{'3}, R^{"1}, and R^{"3} are H, and X² and X⁵ are N. In certain embodiments, each of R^{'1}, R^{'3}, R^{"1}, and R^{"2} are H, and X² and X⁶ are N. In certain embodiments, each of R^{'1}, R^{'2}, R^{"1}, and R^{"3} are H, and X³ and X⁵ are N. In certain embodiments, each of R^{'1}, R^{'2}, R^{"1}, and R^{"2} are H, and X³ and X⁶ are N. In certain embodiments, each of R^{'2}, R^{'1}, R^{"2}, and R^{"3} are H, and X¹ and X³ are N. In certain embodiments, each of R^{'1}, R^{'2}, R^{'3}, and R^{"2} are H, and X⁴ and X⁶ are N.

In certain embodiments, L is a bond and each of R^{3a}, R^{3c}, R^{3a}, R^{3e}, and R^{3f} is H. In certain embodiments, L is a bond, Y is NR^{3b}, and each of R^{3a}, R^{3b}, R^{3c}, R^{3d}, R^{3e}, and R^{3f} is H. In certain embodiments, L is -C(R^{3g})(R^{3h})- and each of R^{3a}, R^{3c}, R^{3d}, R^{3e}, R^{3f}, R^{3g}, and R^{3h} is H. In certain embodiments, L is -C(R^{3g})(R^{3h})-, Y is NR^{3b}, and each of R^{3a}, R^{3b}, R^{3c}, R^{3d}, R^{3e}, R^{3f}, R^{3g}, and R^{3h} is H. In certain embodiments, L is -(C(R^{3g})(R^{3h}))(C(R³ⁱ)(R^{3j}))- and each of R^{3a}, R^{3c}, R^{3d}, R^{3e}, R^{3f}, R^{3g}, R^{3h}, R³ⁱ, and R^{3j} is H. In certain embodiments, L is -C(R^{3g})(R^{3h})-, Y is NR^{3b}, and each of R^{3a}, R^{3b}, R^{3c}, R^{3d}, R^{3e}, R^{3f}, R^{3g}, R^{3h}, R³ⁱ, and R^{3j} are H.

In certain embodiments, R^{3a} is H. In certain embodiments, R^{3a} is a halogen. In certain embodiments, R^{3a} is C₁-C₃ alkyl. In certain embodiments, R^{3a} is C₃-C₈ cycloalkyl. In certain embodiments, R^{3a} is C₁-C₃ alkoxy. In certain embodiments, R^{3a} is C₁-C₃ haloalkyl. In certain embodiments, R^{3a} is C₁-C₃ haloalkoxy.

In certain embodiments, R^{3b} is H. In certain embodiments, R^{3b} is a halogen. In certain embodiments, R^{3b} is C₁-C₃ alkyl. In certain embodiments, R^{3b} is C₃-C₈ cycloalkyl. In certain embodiments, R^{3b} is C₁-C₃ alkoxy. In certain embodiments, R^{3b} is C₁-C₃ haloalkyl. In certain embodiments, R^{3b} is C₁-C₃ haloalkoxy.

In certain embodiments, R^{3c} is H. In certain embodiments, R^{3c} is a halogen. In certain embodiments, R^{3c} is C₁-C₃ alkyl. In certain embodiments, R^{3c} is C₃-C₈ cycloalkyl. In certain embodiments, R^{3c} is C₁-C₃ alkoxy. In certain embodiments, R^{3c} is C₁-C₃ haloalkyl. In certain embodiments, R^{3c} is C₁-C₃ haloalkoxy.

In certain embodiments, R^{3d} is H. In certain embodiments, R^{3d} is a halogen. In certain embodiments, R^{3d} is C₁-C₃ alkyl. In certain embodiments, R^{3d} is C₃-C₈ cycloalkyl. In certain embodiments, R^{3d} is C₁-C₃ alkoxy. In certain embodiments, R^{3d} is C₁-C₃ haloalkyl. In certain embodiments, R^{3d} is C₁-C₃ haloalkoxy.

In certain embodiments, R^{3e} is H. In certain embodiments, R^{3e} is a halogen. In certain embodiments, R^{3e} is C₁-C₃ alkyl. In certain embodiments, R^{3e} is C₃-C₈ cycloalkyl. In certain embodiments, R^{3e} is C₁-C₃ alkoxy. In certain embodiments, R^{3e} is C₁-C₃ haloalkyl. In certain embodiments, R^{3e} is C₁-C₃ haloalkoxy.

In certain embodiments, R^{3f} is H. In certain embodiments, R^{3f} is a halogen. In certain embodiments, R^{3f} is C₁-C₃ alkyl. In certain embodiments, R^{3f} is C₃-C₈ cycloalkyl. In certain embodiments, R^{3f} is C₁-C₃ alkoxy. In certain embodiments, R^{3f} is C₁-C₃ haloalkyl. In certain embodiments, R^{3f} is C₁-C₃ haloalkoxy.

In certain embodiments, R^{3g} is H. In certain embodiments, R^{3g} is a halogen. In certain embodiments, R^{3g} is C₁-C₃ alkyl. In certain embodiments, R^{3g} is C₃-C₈ cycloalkyl. In certain embodiments, R^{3g} is C₁-C₃ alkoxy. In certain embodiments, R^{3g} is C₁-C₃ haloalkyl. In certain embodiments, R^{3g} is C₁-C₃ haloalkoxy.

In certain embodiments, R^{3h} is H. In certain embodiments, R^{3h} is a halogen. In certain embodiments, R^{3h} is C₁-C₃ alkyl. In certain embodiments, R^{3h} is C₃-C₈ cycloalkyl. In certain embodiments, R^{3h} is C₁-C₃ alkoxy. In certain embodiments, R^{3h} is C₁-C₃ haloalkyl. In certain embodiments, R^{3h} is C₁-C₃ haloalkoxy.

In certain embodiments, R³ⁱ is H. In certain embodiments, R³ⁱ is a halogen. In certain embodiments, R³ⁱ is C₁-C₃ alkyl. In certain embodiments, R³ⁱ is C₃-C₈ cycloalkyl. In certain embodiments, R³ⁱ is C₁-C₃ alkoxy. In certain embodiments, R³ⁱ is C₁-C₃ haloalkyl. In certain embodiments, R³ⁱ is C₁-C₃ haloalkoxy.

In certain embodiments, R^{3j} is H. In certain embodiments, R^{3j} is a halogen. In certain embodiments, R^{3j} is C₁-C₃ alkyl. In certain embodiments, R^{3j} is C₃-C₈ cycloalkyl. In certain embodiments, R^{3j} is C₁-C₃ alkoxy. In certain embodiments, R^{3j} is C₁-C₃ haloalkyl. In certain embodiments, R^{3j} is C₁-C₃ haloalkoxy.

In certain embodiments, R^{1a} is H. In certain embodiments, R^{1a} is methyl. In certain embodiments, R^{1a} is F. In certain embodiments, R^{1a} is Cl. In certain embodiments, R^{1a} is CF₃. In certain embodiments, R^{1a} is CN.

In certain embodiments, R^{1b} is H. In certain embodiments, R^{1b} is methyl. In certain embodiments, R^{1b} is F. In certain embodiments, R^{1b} is Cl. In certain embodiments, R^{1b} is CF₃. In certain embodiments, R^{1b} is CN.

In certain embodiments, R^{1a} and R^{1b} are identical.

In certain embodiments, R^{2a} is -(CH₂)₀₋₂NH(CH₂)₀₋₂(optionally substituted 5-oxopyrrolidin-2-yl). In certain embodiments, R^{2a} is -(CH₂)₀₋₂N(C₁-C₆ alkyl)(CH₂)₀₋₂(optionally substituted 5-oxopyrrolidin-2-yl). In certain embodiments, R^{2a} is -(CH₂)₀₋₂NH(CH₂)₀₋₂(C₁-C₆ alkyl). In certain embodiments, R^{2a} is -(CH₂)₀₋₂N(C₁-C₆ alkyl)₂. In certain embodiments, R^{2a} is -(CH₂)₀₋₂NH(CH₂)₀₋₂(C₁-C₆ haloalkyl). In certain embodiments, R^{2a} is -(CH₂)₀₋₂N(C₁-C₆ alkyl)(C₁-C₆ haloalkyl). In certain embodiments, R^{2a} is -(CH₂)₀₋₂NH(CH₂)₀₋₂(C₁-C₆ hydroxyalkyl). In certain embodiments, R^{2a} is -(CH₂)₀₋₂N(C₁-C₆ alkyl)(CH₂)₀₋₂(C₁-C₆ hydroxyalkyl). In certain embodiments, R^{2a} is -(CH₂)₀₋₂NH(CH₂)₀₋₂C(=O)O(C₁-C₆ alkyl). In certain embodiments, R^{2a} is -(CH₂)₀₋₂N(C₁-C₆ alkyl)(CH₂)₀₋₂C(=O)O(C₁-C₆ alkyl). In certain embodiments, R^{2a} is -(CH₂)₀₋₂NH(CH₂)₀₋₂N(C₁-C₆ alkyl)C(=O)(C₁-C₆ alkyl). In certain embodiments, R^{2a} is -(CH₂)₀₋₂NH(CH₂)₀₋₂NHC(=O)(C₁₋C₆ alkyl). In certain embodiments, R^{2a} is -(CH₂)₀₋₂N(C₁-C₆ alkyl)(CH₂)₀₋₂N(C₁-C₆ alkyl)C(=O)(C₁-C₆ alkyl). In certain embodiments, R^{2a} is -(CH₂)₀₋₂N(C₁-C₆ alkyl)(CH₂)₀₋₂NHC(=O)(C₁-C₆ alkyl). In certain embodiments, R^{2a} is -(CH₂)₀₋₂NH(CH₂)₀₋₂(optionally substituted oxetanyl). In certain embodiments, R^{2a} is -(CH₂)₀₋₂N(C₁-C₆ alkyl)(CH₂)₀₋₂(optionally substituted oxetanyl). In certain embodiments, R^{2a} is -(CH₂)₀₋₂NH(CH₂)₀₋₂(optionally substituted oxolanyl). In certain embodiments, R^{2a} is -(CH₂)₀₋₂N(C₁-C₆ alkyl)(CH₂)₀₋₂(optionally substituted oxolanyl). In certain embodiments, R^{2a} is -(CH₂)₀₋₂NH(CH₂)₀₋₂(optionally substituted oxanyl). In certain embodiments, R^{2a} is -(CH₂)₀₋₂N(C₁-C₆ alkyl)(CH₂)₀₋₂(optionally substituted oxanyl). In certain embodiments, R^{2a} is -(CH₂)₀₋₂NH(CH₂)₀₋₂(optionally substituted cyclobutyl). In certain embodiments, R^{2a} is -(CH₂)₀₋₂N(C₁-C₆ alkyl)(CH₂)₀₋₂(optionally substituted cyclobutyl). In certain embodiments, R^{2a} is - (CH₂)₀₋₂NH(CH₂)₀₋₂(optionally substituted cyclohexyl). In certain embodiments, R^{2a} is - (CH₂)₀₋₂N(C₁-C₆ alkyl)(CH₂)₀₋₂(optionally substituted cyclohexyl). In certain embodiments, R^{2a} is -(CH₂)₀₋₂NH(CH₂)₀₋₂(optionally substituted C₅-C₆ cycloalkylsulfonyl). In certain embodiments, R^{2a} is -(CH₂)₀₋₂N(C₁-C₆ alkyl)(CH₂)₀₋₂(optionally substituted C₅-C₆ cycloalkylsulfonyl). In certain embodiments, R^{2a} is -(CH₂)₀₋₂NH(CH₂)₀₋₂(optionally substituted azetidinyl). In certain embodiments, R^{2a} is -(CH₂)₀₋₂N(C₁-C₆ alkyl)(CH₂)₀₋₂(optionally substituted azetidinyl). In certain embodiments, R^{2a} is -(CH₂)₀₋₂NH(CH₂)₀₋₂(optionally substituted pyrrolidinyl). In certain embodiments, R^{2a} is -(CH₂)₀₋₂N(C₁-C₆ alkyl)(CH₂)₀₋₂(optionally substituted pyrrolidinyl). In certain embodiments, R^{2a} is -(CH₂)₀₋₂NH(CH₂)₀₋₂(optionally substituted piperidinyl). In certain embodiments, R^{2a} is -(CH₂)₀₋₂N(C₁-C₆ alkyl)(CH₂)₀₋₂(optionally substituted piperidinyl). In certain embodiments, R^{2a} is - (CH₂)₀₋₂(optionally substituted guanidinyl). In certain embodiments, R^{2a} is -(CH₂)₀₋₂(optionally substituted azetidinyl). In certain embodiments, R^{2a} is -(CH₂)₀₋₂NH(C(CH₂)₂₋₅)C(=O)O(C₁-C₆ alkyl). In certain embodiments, R^{2a} is -(CH₂)₀₋₂N(C₁-C₆ alkyl)(C(CH₂)₂₋₅)C(=O)O(C₁-C₆ alkyl). In certain embodiments, R^{2a} is -(CH₂)₀₋₂NH(CH(C₁-C₃ alkyl))C(=O)O(C₁-C₆ alkyl). In certain embodiments, R^{2a} is -(CH₂)₀₋₂N(C₁-C₆ alkyl)(CH(C₁₋C₃ alkyl))C(=O)O(C₁-C₆ alkyl). In certain embodiments, R^{2a} is -(CH₂)₀₋₂NH(CH₂)₀₋₁(C₅-C₉ bridged cycloalkyl)(CH₂)₀₋₁C(=O)O(C₁-C₆ alkyl). In certain embodiments, R^{2a} is -(CH₂)₀₋₂N(C₁-C₆ alkyl)(CH₂)₀₋₁(C₅-C₉ bridged cycloalkyl)(CH₂)₀₋₁C(=O)O(C₁-C₆ alkyl). In certain embodiments, R^{2a} is -(CH₂)₀₋₂NH(CH₂)₀₋₁(CHOH)(CH₂)₀₋₁C(=O)O(C₁-C₆ alkyl). In certain embodiments, R^{2a} is -(CH₂)₀₋₂NH(CH₂)₀₋₁(CHO(C₁-C₆ alkyl))(CH₂)₀₋₁C(=O)O(C₁-C₆ alkyl). In certain embodiments, R^{2a} is -(CH₂)₀₋₂N(C₁-C₆ alkyl)(CH₂)₀₋₁(CHOH)(CH₂)₀₋₁C(=O)O(C₁-C₆ alkyl). In certain embodiments, R^{2a} is -(CH₂)₀₋₂N(C₁-C₆ alkyl)(CH₂)₀₋₁(CHO(C₁-C₆ alkyl))(CH₂)₀₋₁C(=O)O(C₁-C₆ alkyl). In certain embodiments, R^{2a} is -(CH₂)₀₋₂(optionally substituted azetidinyl). In certain embodiments, R^{2a} is -(CH₂)₀₋₂(C₄-C₁₀ bridged heterocycloalkyl). In certain embodiments, R^{2a} is -(CH₂)₀₋₂(optionally substituted pyrrolidinyl). In certain embodiments, R^{2a} is -(CH₂)₀₋₂(optionally substituted piperidinyl). In certain embodiments, R^{2a} is -(CH₂)₀₋₂(optionally substituted morpholinyl). In certain embodiments, R^{2a} is -(CH₂)₀₋₂(optionally substituted piperazinyl). In certain embodiments, R^{2a} is -(CH₂)₀₋₂(optionally substituted piperazin-2-onyl). In certain embodiments, R^{2a} is - C(CH₃)N(C₁-C₆ alkyl)₂. In certain embodiments, R^{2a} is -(CH₂)₀₋₂N(C₁-C₆ alkyl)(CH₂)₀₋₂(CHF₂). In certain embodiments, R^{2a} is -(CH₂)₀₋₂NH(CH₂)₀₋₂(CHF₂). In certain embodiments, R^{2a} is -(CH₂)₀₋₂N(C₁-C₆ alkyl)(CH₂)₀₋₄(CH₂F). In certain embodiments, R^{2a} is -(CH₂)₀₋₂NH(CH₂)₀₋₄(CH₂F). In certain embodiments, R^{2a} is -(CH₂)₀₋₂NH(CH₂)₀₋₂N(C₁-C₆ alkyl)C(=O)CH₃. In certain embodiments, R^{2a} is -(CH₂)₀₋₂NH(CH₂)₀₋₂NHC(=O)CH₃. In certain embodiments, R^{2a} is -(CH₂)₀₋₂N(C₁-C₆ alkyl)(CH₂)₀₋₂N(C₁-C₆ alkyl)C(=O)CH₃. In certain embodiments, R^{2a} is -(CH₂)₀₋₂N(C₁-C₆ alkyl)(CH₂)₀₋₂NHC(=O)CH₃. In certain embodiments, R^{2a} is -(CH₂)₀₋₂N(C₁-C₆ alkyl)C((C(=O)OC₁-C₆ alkyl)((CH₂)₀₋₂O(C₁-C₆ alkyl))). In certain embodiments, R^{2a} is -(CH₂)₀₋₂NHC((C(=O)OC₁-C₆ alkyl)((CH₂)₀₋₂O(C₁-C₆ alkyl))). In certain embodiments, R^{2a} is -(CH₂)₀₋₂N(C₁-C₆ alkyl)C((C(=O)OH((CH₂)₀-₂O(C₁-C₆ alkyl))). In certain embodiments, R^{2a} is -(CH₂)₀₋₂N(C₁-C₆ alkyl)C((C(=O)OC₁-C₆ alkyl)((CH₂)₀₋₂OH)). In certain embodiments, R^{2a} is -(CH₂)₀₋₂NHC((C(=O)OH)((CH₂)₀₋₂O(C₁-C₆ alkyl))). In certain embodiments, R^{2a} is -(CH₂)₀₋₂N(C₁-C₆ alkyl)C((C(=O)OH((CH₂)₀₋₂OH)). In certain embodiments, R^{2a} is -(CH₂)₀₋₂NHC((C(=O)OC₁-C₆ alkyl)((CH₂)₀₋₂OH)). In certain embodiments, R^{2a} is -(CH₂)₀₋₂NHC((C(=O)OH((CH₂)₀₋₂OH)). In certain embodiments, R^{2a} is -(CH₂)₀₋₂N(C₁-C₆ alkyl)(CH₂)₁₋₄N(C₁-C₆ alkyl)C(=O)Me. In certain embodiments, R^{2a} is -(CH₂)₀₋₂N(C₁-C₆ alkyl)(CH₂)₁₋₄NHC(=O)Me. In certain embodiments, R^{2a} is -(CH₂)₀₋₂NH(CH₂)₁₋₄N(C₁-C₆ alkyl)C(=O)Me. In certain embodiments, R^{2a} is -(CH₂)₀₋₂NH(CH₂)₁₋₄NHC(=O)Me. In certain embodiments, R^{2a} is -(CH₂)₀₋₂N(C₁-C₆ alkyl)(CH₂)₁₋₅C(=O)N(C₁-C₆ alkyl)(C₁-C₆ alkyl). In certain embodiments, R^{2a} is -(CH₂)₀₋₂NH(CH₂)₁₋₅C(=O)N(C₁-C₆ alkyl)(C₁-C₆ alkyl). In certain embodiments, R^{2a} is -(CH₂)₀₋₂N(C₁-C₆ alkyl)(CH₂)₁₋₅C(=O)NH(C₁-C₆ alkyl). In certain embodiments, R^{2a} is -(CH₂)₀₋₂N(C₁-C₆ alkyl)(CH₂)₁₋₅C(=O)NH₂. In certain embodiments, R^{2a} is -(CH₂)₀₋₂NH(CH₂)₁₋₅C(=O)NH(C₁-C₆ alkyl). In certain embodiments, R^{2a} is -(CH₂)₀₋₂NH(CH₂)₁₋₅C(=O)NH₂. In certain embodiments, R^{2a} is -(CH₂)₀₋₂N(C₁-C₆ alkyl)(CH₂)₀₋₂(optionally substituted cyclopropyl). In certain embodiments, R^{2a} is -(CH₂)₀₋₂NH(CH₂)₀₋₂(optionally substituted cyclopropyl). In certain embodiments, R^{2a} is -(CH₂)₀₋₂N(C₁-C₆ alkyl)(CH₂)₀₋₂(optionally substituted butanolid-2-yl). In certain embodiments, R^{2a} is -(CH₂)₀₋₂NH(CH₂)₀₋₂(optionally substituted butanolide-2-yl). In certain embodiments, R^{2a} is -(CH₂)₀₋₂N(C₁-C₆ alkyl)(CH₂)₀₋₂(optionally substituted tetrahydro-4-thiopyranyl). In certain embodiments, R^{2a} is -(CH₂)₀₋₂NH(CH₂)₀₋₂(optionally substituted oxazolyl), In certain embodiments, R^{2a} is -(CH₂)₀₋₂NH(CH₂)₀₋₂(optionally substituted tetrahydro-4-thiopyranyl). In certain embodiments, R^{2a} is -CH(CH₃)NH(CH₂)₀₋₂(optionally substituted piperidinyl). In certain embodiments, R^{2a} is -CH(CH₃)N(C₁-C₆ alkyl)(CH₂)₀₋₂(optionally substituted piperidinyl). In certain embodiments, R^{2a} is -(CH₂)₀₋₂N(C₁-C₆ alkyl)(CH₂)₀₋₂(optionally substituted piperazinyl). In certain embodiments, R^{2a} is -(CH₂)₀₋₂NH(CH₂)₀₋₂(optionally substituted piperazinyl). In certain embodiments, R^{2a} is C(=O)OH.

In certain embodiments, R^{2b} is -(CH₂)₀₋₂(optionally substituted 5-oxopyrrolidin-2-yl). In certain embodiments, R^{2b} is -(CH₂)₀₋₂(optionally substituted piperidinyl). In certain embodiments, R^{2b} is -(CH₂)₀₋₂(C₁-C₆ alkyl). In certain embodiments, R^{2b} is -(CH₂)₀₋₂(C₁-C₆ haloalkyl). In certain embodiments, R^{2b} is -(CH₂)₀₋₂(C₁-C₆ hydroxyalkyl). In certain embodiments, R^{2b} is -(CH₂)₀₋₂(optionally substituted cyclopropyl). In certain embodiments, R^{2b} is -(CH₂)₀₋₂(optionally substituted cyclobutyl). In certain embodiments, R^{2b} is -(CH₂)₀₋₂C(=O)O(C₁-C₆ alkyl). In certain embodiments, R^{2b} is -(CH₂)₀₋₂(optionally substituted azetidinyl). In certain embodiments, R^{2b} is -(CH₂)₀₋₂C(=O)OH. In certain embodiments, R^{2b} is -(CH₂)₀₋₂N(C₁-C₆ alkyl)CH₃. In certain embodiments, R^{2b} is -(CH₂)₀₋₂NHCH₃. In certain embodiments, R^{2b} is -(CH₂)₀₋₂N(C₁-C₆ alkyl)(CH₂)₀₋₂C(=O)O(C₁-C₆ alkyl). In certain embodiments, R^{2b} is -(CH₂)₀₋₂NH(CH₂)₀₋₂C(=O)O(C₁-C₆ alkyl). In certain embodiments, R^{2b} is -(CH₂)₀₋₂N(C₁-C₆ alkyl)(CH₂)₀₋₂C(=O)OH. In certain embodiments, R^{2b} is -(CH₂)₀₋₂NH(CH₂)₀₋₂C(=O)OH. In certain embodiments, R^{2b} is -(CH₂)₀₋₂N(C₁-C₆ alkyl)(CH₂)₀₋₂(optionally substituted 5-oxopyrrolidin-2-yl). In certain embodiments, R^{2b} is -(CH₂)₀₋₂NH(CH₂)₀₋₂(optionally substituted 5-oxopyrrolidin-2-yl). In certain embodiments, R^{2b} is - (CH₂)₀₋₂C(=O)N(C₁-C₆ alkyl)(C₁-C₆ alky). In certain embodiments, R^{2b} is -(CH₂)₀₋₂C(=O)NH(C₁-C₆ alkyl). In certain embodiments, R^{2b} is -(CH₂)₀₋₂C(=O)NH₂. In certain embodiments, R^{2b} is -(CH₂)₀₋₂CH(OC₁-C₆ alkyl)CH₂F. In certain embodiments, R^{2b} is - (CH₂)₀₋₂CH(OH)CH₂F.

In certain embodiments, R^{2a} is 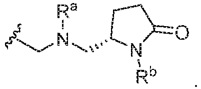 In certain embodiments, R^{2a} is 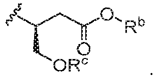 In certain embodiments, R^{2a} is 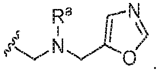 In certain embodiments, R^{2a} is OR^{c}. In certain embodiments, R^{2a} is 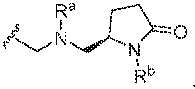 In certain embodiments, 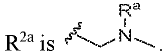 In certain embodiments, R^{2a} is 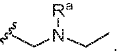 In certain embodiments, R^{2a} is 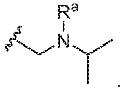 In certain embodiments, R^{2a} is 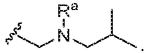 In certain embodiments, R^{2a} is 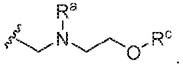 In certain embodiments, R^{2a} is 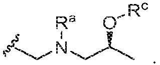 In certain embodiments, R^{2a} is 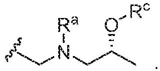 In certain embodiments, R^{2a} is 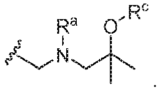 In certain embodiments, R^{2a} is 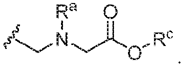 In certain embodiments, R^{2a} is 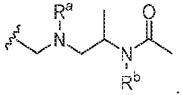 In certain embodiments, R^{2a} is 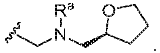 In certain embodiments, R^{2a} is 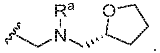 In certain embodiments, R^{2a} is 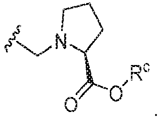 In certain embodiments, R^{2a} is 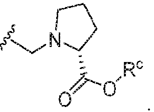 In certain embodiments, R^{2a} is 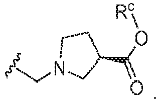 In certain embodiments, R^{2a} is 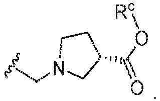 In certain embodiments, R^{2a} is 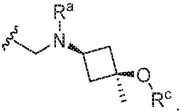 In certain embodiments, R^{2a} is 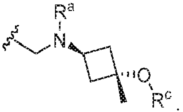 In certain embodiments, R^{2a} is 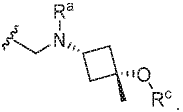 In certain embodiments, R^{2a} is 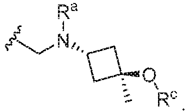 In certain embodiments, R^{2a} is 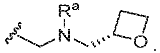 In certain embodiments, R^{2a} is 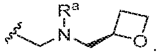 In certain embodiments, R^{2a} is 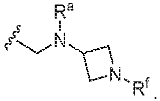 In certain embodiments, R^{2a} is 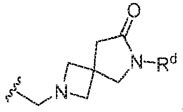 . In certain embodiments, R^{2a} is 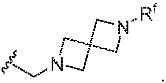 In certain embodiments, R^{2a} is 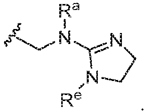 In certain embodiments, R^{2a} is 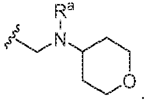 In certain embodiments, R^{2a} is 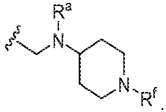 In certain embodiments, R^{2a} is 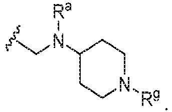 In certain embodiments, R^{2a} is 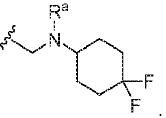 In certain embodiments, R^{2a} is 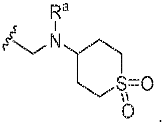 In certain embodiments, R^{2a} is 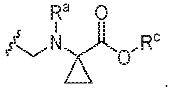 In certain embodiments, R^{2a} is 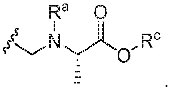 In certain embodiments, R^{2a} is 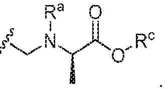 In certain embodiments, R^{2a} is 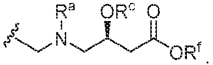 In certain embodiments, R^{2a} is 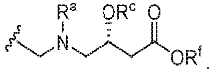 In certain embodiments, R^{2a} is 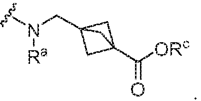 In certain embodiments, R^{2a} is 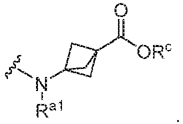 In certain embodiments, R^{2a} is 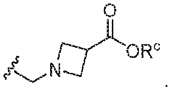 In certain embodiments, R^{2a} is 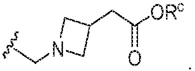 In certain embodiments, R^{2a} is 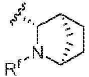 . In certain embodiments, R^{2a} is 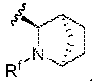 In certain embodiments, R^{2a} is 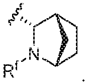 In certain embodiments, R^{2a} is 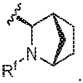 In certain embodiments, R^{2a} is 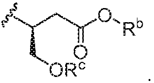 In certain embodiments, R^{2a} is 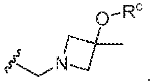 In certain embodiments, R^{2a} is 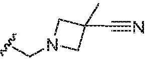 . In certain embodiments, R^{2a} is 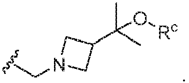 In certain embodiments, R^{2a} is 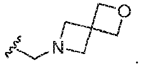 In certain embodiments, R^{2a} is 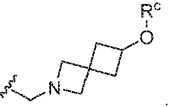 In certain embodiments, R^{2a} is 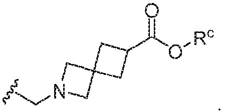 In certain embodiments, R^{2a} is 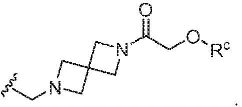 In certain embodiments, R^{2a} is 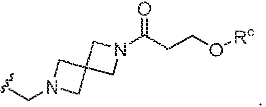 In certain embodiments, R^{2a} is 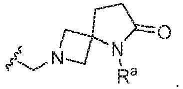 In certain embodiments, R^{2a} is 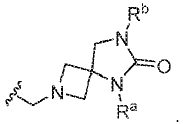 In certain embodiments, R^{2a} is 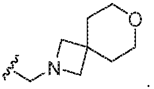 In certain embodiments, R^{2a} is 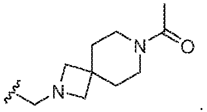 In certain embodiments, R^{2a} is 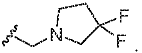 In certain embodiments, R^{2a} is 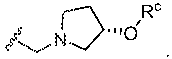 In certain embodiments, R^{2a} is 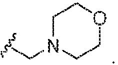 In certain embodiments, R^{2a} is 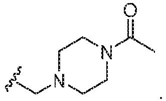 In certain embodiments, R^{2a} is 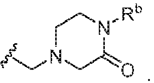 In certain embodiments, R^{2a} is 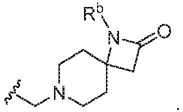 In certain embodiments, R^{2a} is 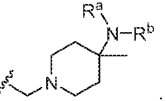 In certain embodiments, R^{2a} is 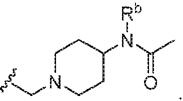 In certain embodiments, R^{2a} is 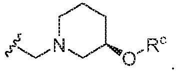 In certain embodiments, R^{2a} is 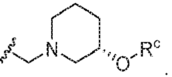 In certain embodiments, R^{2a} is 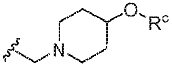 . In certain embodiments, R^{2a} is 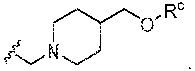 In certain embodiments, R^{2a} is 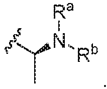 In certain embodiments, R^{2a} is 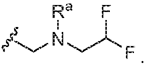 In certain embodiments, R^{2a} is 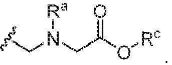 In certain embodiments, R^{2a} is 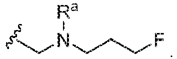 In certain embodiments, R^{2a} is 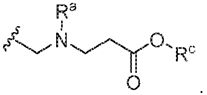 In certain embodiments, R^{2a} is 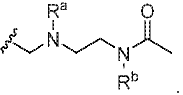 In certain embodiments, R^{2a} is 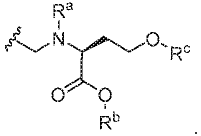 In certain embodiments, R^{2a} is 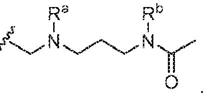 In certain embodiments, R^{2a} is 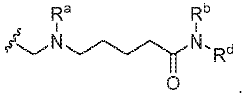 In certain embodiments, R^{2a} is 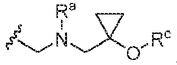 . In certain embodiments, R^{2a} is 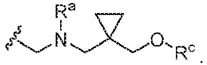 In certain embodiments, R^{2a} is 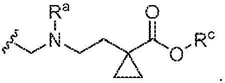 In certain embodiments, R^{2a} is 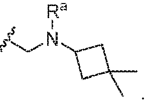 In certain embodiments, R^{2a} is 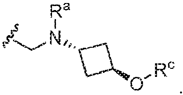 In certain embodiments, R^{2a} is 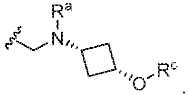 In certain embodiments, R^{2a} is 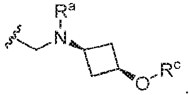 In certain embodiments, R^{2a} is 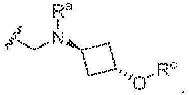 In certain embodiments, R^{2a} is 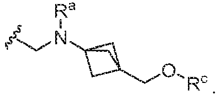 In certain embodiments, R^{2a} is 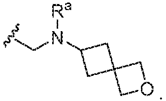 In certain embodiments, R^{2a} is 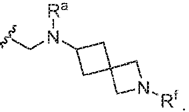 In certain embodiments, R^{2a} is 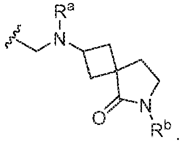 In certain embodiments, R^{2a} is 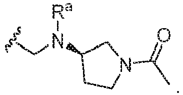 In certain embodiments, R^{2a} is 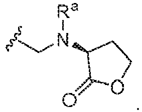 In certain embodiments, R^{2a} is 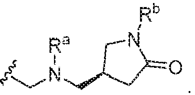 In certain embodiments, R^{2a} is 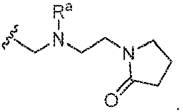 In certain embodiments, R^{2a} is 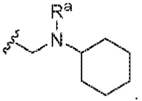 In certain embodiments, R^{2a} is 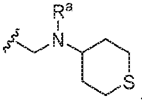 In certain embodiments, R^{2a} is 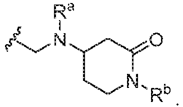 In certain embodiments, R^{2a} is 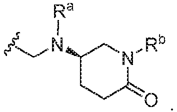 In certain embodiments, R^{2a} is 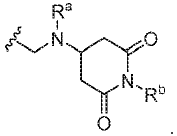 In certain embodiments, R^{2a} is 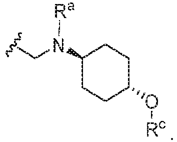 In certain embodiments, R^{2a} is 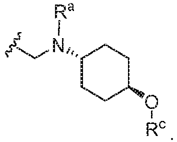 In certain embodiments, R^{2a} is 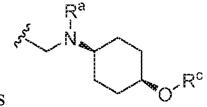 . In certain embodiments, R^{2a} is 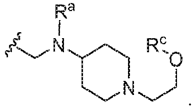 In certain embodiments, R^{2a} is 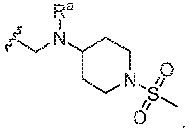 In certain embodiments, R^{2a} is 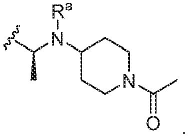 In certain embodiments, R^{2a} is 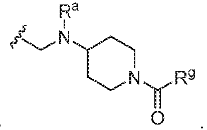 In certain embodiments, R^{2a} is 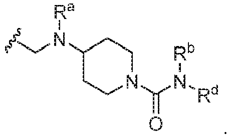 In certain embodiments, R^{2a} is 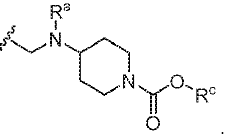 In certain embodiments, R^{2a} is 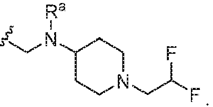 In certain embodiments, R^{2a} is 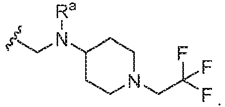 In certain embodiments, R^{2a} is 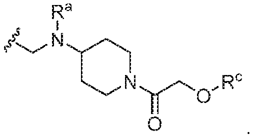 In certain embodiments, R^{2a} is 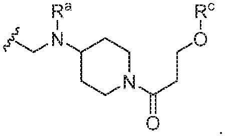 In certain embodiments, R^{2a} is 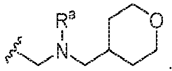 In certain embodiments, R^{2a} is

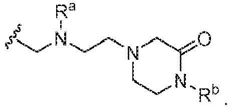

In certain embodiments, R³ is H. In certain embodiments, R³ is C₁-C₃ alkyl. In certain embodiments, R³ is C₃-C₈ cycloalkyl. In certain embodiments, R³ is C₁-C₃ alkoxy. In certain embodiments, R³ is C₁-C₃ haloalkyl. In certain embodiments, R³ is C₁-C₃ haloalkoxy. In certain embodiments, R³ is -C(=O)C₁-C₃ alkyl. In certain embodiments, R³ is H. In certain embodiments, R³ is methyl. In certain embodiments, R³ is ethyl. In certain embodiments, R³ is isopropyl. In certain embodiments, R³ is -C(=O)CH₃.

In certain embodiments, R^{b} is H. In certain embodiments, R^{d} is C₁-C₃ alkyl. In certain embodiments, R^{b} is C₃-C₈ cycloalkyl. In certain embodiments, R^{d} is C₁-C₃ alkoxy. In certain embodiments, R^{d} is C₁-C₃ haloalkyl. In certain embodiments, R^{d} is C₁-C₃ haloalkoxy. In certain embodiments, R^{d} is -C(=O)C₁-C₃ alkyl. In certain embodiments, R^{b} is H. In certain embodiments, R^{b} is methyl. In certain embodiments, R^{d} is ethyl. In certain embodiments, R^{b} is isopropyl. In certain embodiments, R^{d} is -C(=O)CH₃.

In certain embodiments, R^{c} is H. In certain embodiments, R^{c} is C₁-C₃ alkyl. In certain embodiments, R^{c} is C₃-C₈ cycloalkyl. In certain embodiments, R^{c} is C₁-C₃ alkoxy. In certain embodiments, R^{c} is C₁-C₃ haloalkyl. In certain embodiments, R^{c} is C₁-C₃ haloalkoxy. In certain embodiments, R^{c} is -C(=O)C₁-C₃ alkyl. In certain embodiments, R^{c} is H. In certain embodiments, R^{c} is methyl. In certain embodiments, R^{c} is ethyl. In certain embodiments, R^{c} is isopropyl. In certain embodiments, R^{c} is -C(=O)CH₃.

In certain embodiments, R^{d} is H. In certain embodiments, R^{d} is C₁-C₃ alkyl. In certain embodiments, R^{d} is C₃-C₈ cycloalkyl. In certain embodiments, R^{d} is C₁-C₃ alkoxy. In certain embodiments, R^{d} is C₁-C₃ haloalkyl. In certain embodiments, R^{d} is C₁-C₃ haloalkoxy. In certain embodiments, R^{d} is -C(=O)C₁-C₃ alkyl. In certain embodiments, R^{d} is H. In certain embodiments, R^{d} is methyl. In certain embodiments, R^{d} is ethyl. In certain embodiments, R^{d} is isopropyl. In certain embodiments, R^{d} is -C(=O)CH₃.

In certain embodiments, R^{e} is H. In certain embodiments, R^{e} is C₁-C₃ alkyl. In certain embodiments, R^{e} is C₃-C₈ cycloalkyl. In certain embodiments, R^{e} is C₁-C₃ alkoxy. In certain embodiments, R^{e} is C₁-C₃ haloalkyl. In certain embodiments, R^{e} is C₁-C₃ haloalkoxy. In certain embodiments, R^{e} is -C(=O)C₁-C₃ alkyl. In certain embodiments, R^{e} is H. In certain embodiments, R^{e} is methyl. In certain embodiments, R^{e} is ethyl. In certain embodiments, R^{e} is isopropyl. In certain embodiments, R^{e} is -C(=O)CH₃.

In certain embodiments, R^{f} is H. In certain embodiments, R^{f} is C₁-C₃ alkyl. In certain embodiments, R^{f} is C₃-C₈ cycloalkyl. In certain embodiments, R^{f} is C₁-C₃ alkoxy. In certain embodiments, R^{f} is C₁-C₃ haloalkyl. In certain embodiments, R^{f} is C₁-C₃ haloalkoxy. In certain embodiments, R^{f} is -C(=O)C₁-C₃ alkyl. In certain embodiments, R^{f} is H. In certain embodiments, R^{f} is methyl. In certain embodiments, R^{f} is ethyl. In certain embodiments, R^{f} is isopropyl. In certain embodiments, R^{f} is -C(=O)CH₃.

In certain embodiments, R^{g} is optionally substituted C₂-C₆ heteroaryl. In certain embodiments, R^{g} is optionally substituted phenyl. In certain embodiments, R^{g} is phenyl. In certain embodiments, R^{g} is 2-furyl. In certain embodiments, R^{g} is 2-pyridyl. In certain embodiments, R^{g} is 3-pyridyl. In certain embodiments, R^{g} is 4-pyridyl. In certain embodiments, R^{g} is 3-chloro-2-pyridyl.

In certain embodiments, R^{2b} is 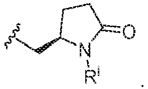 In certain embodiments, R^{2b} is 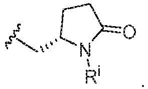 In certain embodiments, R^{2b} is methyl. In certain embodiments, R^{2b} is 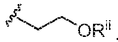 In certain embodiments, R^{2b} is 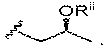 In certain embodiments, R^{2b} is 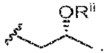 In certain embodiments, R^{2b} is 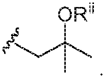 In certain embodiments, R^{2b} is 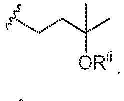 certain embodiments, R^{2b} is 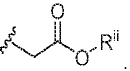 In certain embodiments, R^{2b} is 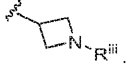 In certain embodiments, R^{2b} is 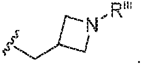 In certain embodiments, R^{2b} is 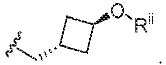 In certain embodiments, R^{2b} is 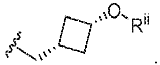 In certain embodiments, R^{2b} is 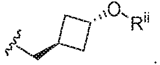 In certain embodiments, R^{2b} is 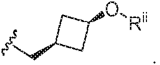 In certain embodiments, R^{2b} is 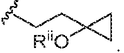 In certain embodiments, R^{2b} is 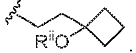 In certain embodiments, R^{2b} is 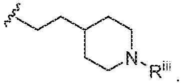 In certain embodiments, R^{2b} is 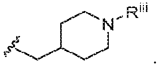 In certain embodiments, R^{2b} is 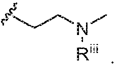 In certain embodiments, R^{2b} is 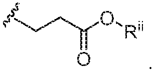 In certain embodiments, R^{2b} is 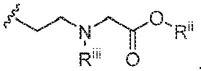 In certain embodiments, R^{2b} is 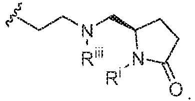 In certain embodiments, R^{2b} is 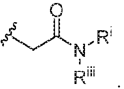 In certain embodiments, R^{2b} is 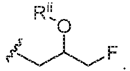 In certain embodiments, R^{2b} is 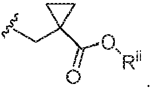 In certain embodiments, R^{2b} is 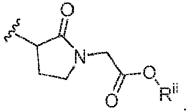 In certain embodiments, R^{2b} is

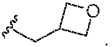

In certain embodiments, Rⁱ is H. In certain embodiments, Rⁱ is C₁-C₃ alkyl. In certain embodiments, Rⁱ is C₃-C₈ cycloalkyl. In certain embodiments, Rⁱ is C₁-C₃ alkoxy. In certain embodiments, Rⁱ is C₁-C₆ haloalkyl. In certain embodiments, Rⁱ is C₁-C₃ haloalkoxy. In certain embodiments, Rⁱ is -C(=O)C₁-C₃ alkyl. In certain embodiments, Rⁱ is methyl. In certain embodiments, Rⁱ is -C(=O)CH₃.

In certain embodiments, Rⁱⁱ is H. In certain embodiments, Rⁱⁱ is C₁-C₃ alkyl. In certain embodiments, Rⁱⁱ is C₃-C₈ cycloalkyl. In certain embodiments, Rⁱⁱ is C₁-C₃ alkoxy. In certain embodiments, Rⁱⁱ is C₁-C₆ haloalkyl. In certain embodiments, Rⁱⁱ is C₁-C₃ haloalkoxy. In certain embodiments, Rⁱⁱ is -C(=O)C₁-C₃ alkyl. In certain embodiments, Rⁱⁱ is methyl. In certain embodiments, Rⁱⁱ is -C(=O)CH₃.

In certain embodiments, Rⁱⁱⁱ is H. In certain embodiments, Rⁱⁱⁱ is C₁-C₃ alkyl. In certain embodiments, Rⁱⁱⁱ is C₃-C₈ cycloalkyl. In certain embodiments, Rⁱⁱⁱ is C₁-C₃ alkoxy. In certain embodiments, Rⁱⁱⁱ is C₁-C₆ haloalkyl. In certain embodiments, Rⁱⁱⁱ is C₁-C₃ haloalkoxy. In certain embodiments, Rⁱⁱⁱ is -C(=O)C₁-C₃ alkyl. In certain embodiments, Rⁱⁱⁱ is methyl. In certain embodiments, Rⁱⁱⁱ is -C(=O)CH₃.

In certain embodiments, R^{1c} is 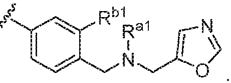 In certain embodiments, R^{1c} is 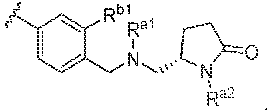 In certain embodiments, R^{1c} is 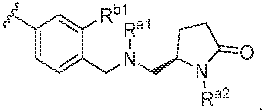 In certain embodiments, R^{1c} is 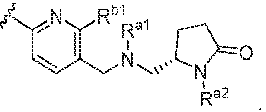 In certain embodiments, R^{1c} is 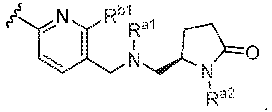 In certain embodiments, R^{1c} is 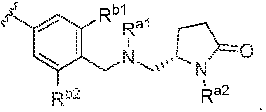 In certain embodiments, R^{1c} is 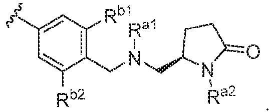 In certain embodiments, R^{1c} is 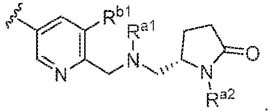 In certain embodiments, R^{1c} is 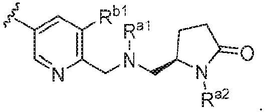 In certain embodiments, R^{1c} is 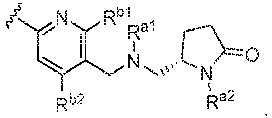 In certain embodiments, R^{1c} is 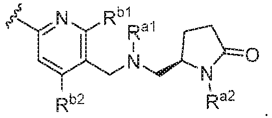 In certain embodiments, R^{1c} is 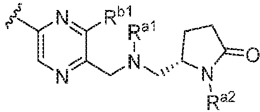 . In certain embodiments, R^{1c} is 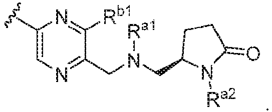 In certain embodiments, R^{1c} is 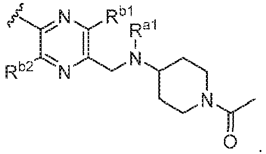 In certain embodiments, R^{1c} is 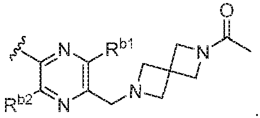 In certain embodiments, R^{1c} is 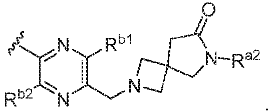 In certain embodiments, R^{1c} is 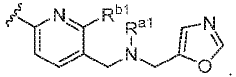 In certain embodiments, R^{1c} is 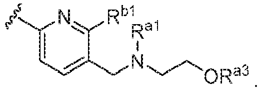 In certain embodiments, R^{1c} is 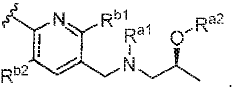 In certain embodiments, R^{1c} is 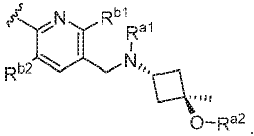 In certain embodiments, R^{1c} is 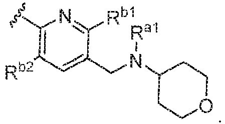 In certain embodiments, R^{1c} is 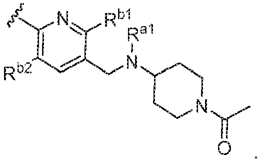 In certain embodiments, R^{1c} is 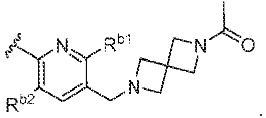 In certain embodiments, R^{1c} is 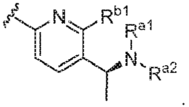 In certain embodiments, R^{1c} is 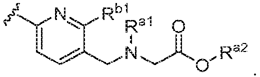 In certain embodiments, R^{1c} is 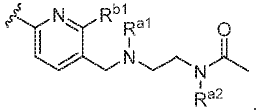 In certain embodiments, R^{1c} is 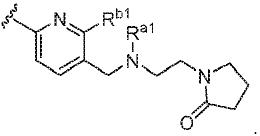 In certain embodiments, R^{1c} is 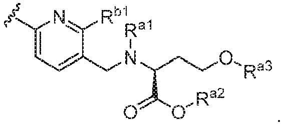 In certain embodiments, R^{1c} is 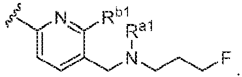 In certain embodiments, R^{1c} is 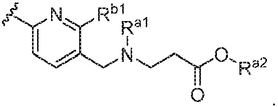 In certain embodiments, R^{1c} is 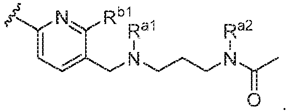 In certain embodiments, R^{1c} is 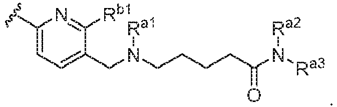 In certain embodiments, R^{1c} is 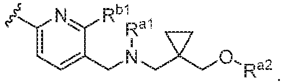 In certain embodiments, R^{1c} is 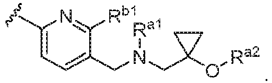 In certain embodiments, R^{1c} is 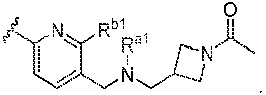 In certain embodiments, R^{1c} is 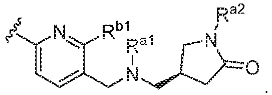 In certain embodiments, R^{1c} is 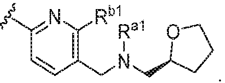 In certain embodiments, R^{1c} is 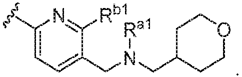 In certain embodiments, R^{1c} is 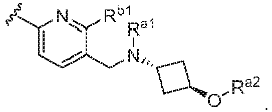 In certain embodiments, R^{1c} is 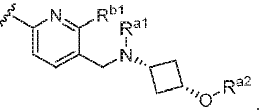 In certain embodiments, R^{1c} is 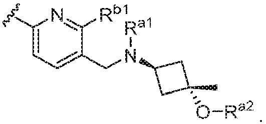 In certain embodiments, R^{1c} is 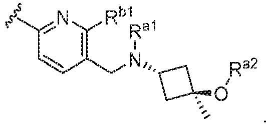 In certain embodiments, R^{1c} is 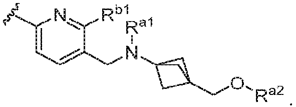 In certain embodiments, R^{1c} is 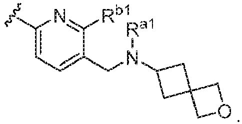 In certain embodiments, R^{1c} is 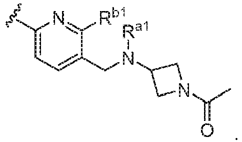 In certain embodiments, R^{1c} is 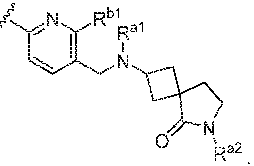 In certain embodiments, R^{1c} is 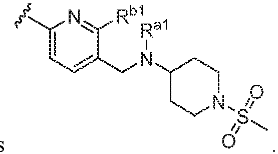 In certain embodiments, R^{1c} is 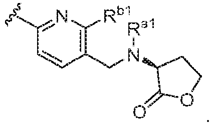 In certain embodiments, R^{1c} is 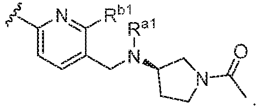 In certain embodiments, R^{1c} is 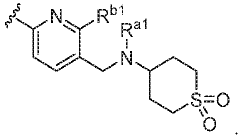 In certain embodiments, R^{1c} is 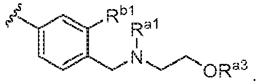 In certain embodiments, R^{1c} is 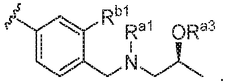 In certain embodiments, R^{1c} is 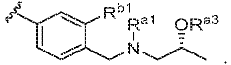 In certain embodiments, R^{1c} is 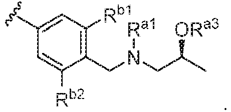 In certain embodiments, R^{1c} is 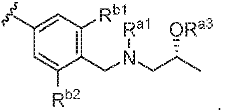 In certain embodiments, R^{1c} is 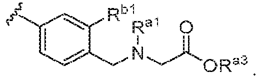 In certain embodiments, R^{1c} is 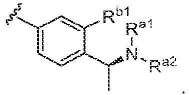 In certain embodiments, R^{1c} is 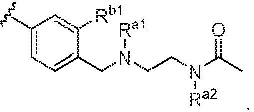 In certain embodiments, R^{1c} is 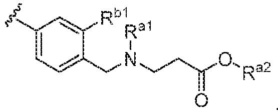 In certain embodiments, R^{1c} is 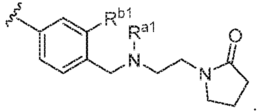 In certain embodiments, R^{1c} is 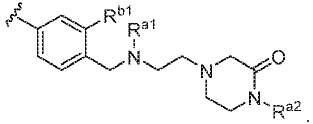 In certain embodiments, R^{1c} is 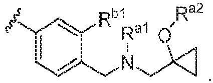 In certain embodiments, R^{1c} is 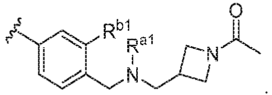 In certain embodiments, R^{1c} is 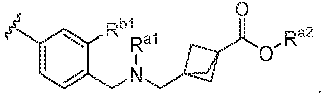 In certain embodiments, R^{1c} is 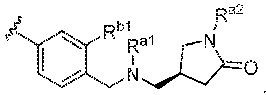 In certain embodiments, R^{1c} is 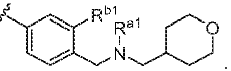 In certain embodiments, R^{1c} is 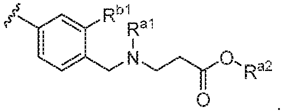 In certain embodiments, R^{1c} is 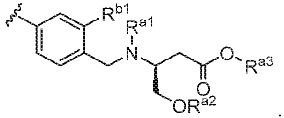 In certain embodiments, R^{1c} is 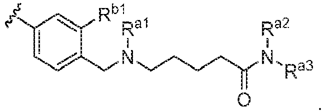 In certain embodiments, R^{1c} is 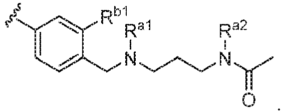 In certain embodiments, R^{1c} is 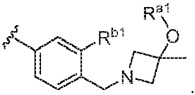 In certain embodiments, R^{1c} is 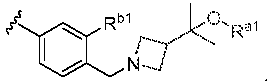 In certain embodiments, R^{1c} is 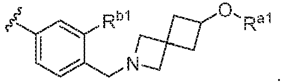 In certain embodiments, R^{1c} is 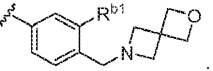 In certain embodiments, R^{1c} is 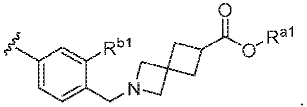 In certain embodiments, R^{1c} is 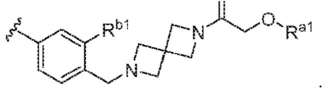 In certain embodiments, R^{1c} is 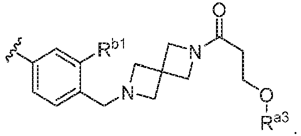 In certain embodiments, R^{1c} is 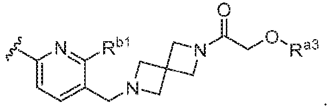 In certain embodiments, R^{1c} is 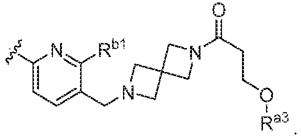 In certain embodiments, R^{1c} is 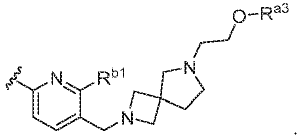 In certain embodiments, R^{1c} is 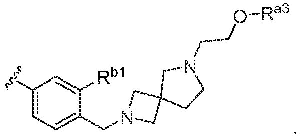 In certain embodiments, R^{1c} is 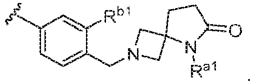 In certain embodiments, R^{1c} is 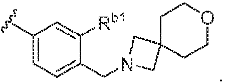 In certain embodiments, R^{1c} is 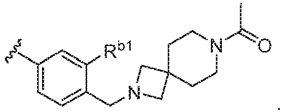 In certain embodiments, R^{1c} is 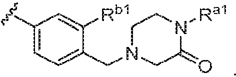 In certain embodiments, R^{1c} is 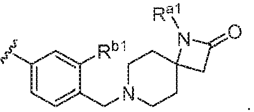 In certain embodiments, R^{1c} is 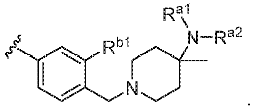 In certain embodiments, R^{1c} is 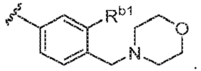 In certain embodiments, R^{1c} is 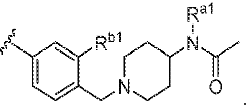 In certain embodiments, R^{1c} is 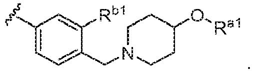 In certain embodiments, R^{1c} is 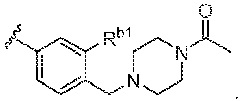 In certain embodiments, R^{1c} is 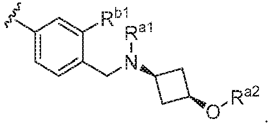 In certain embodiments, R^{1c} is 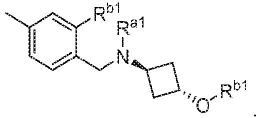 In certain embodiments, R^{1c} is 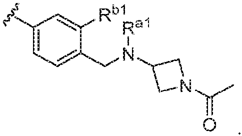 In certain embodiments, R^{1c} is 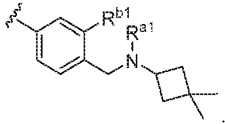 In certain embodiments, R^{1c} is 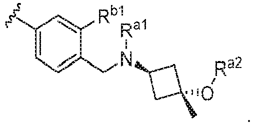 In certain embodiments, R^{1c} is 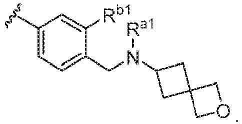 In certain embodiments, R^{1c} is 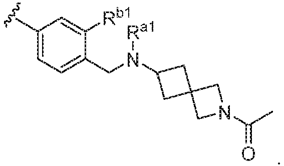 In certain embodiments, R^{1c} is 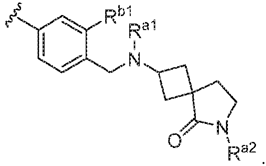 In certain embodiments, R^{1c} is 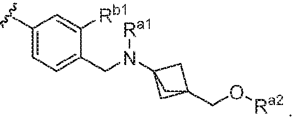 In certain embodiments, R^{1c} is 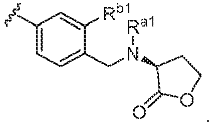 In certain embodiments, R^{1c} is 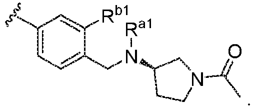 In certain embodiments, R^{1c} is 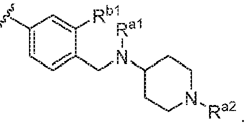 In certain embodiments, R^{1c} is 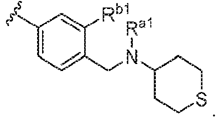 In certain embodiments, R^{1c} is 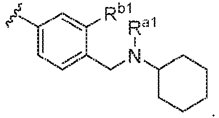 In certain embodiments, R^{1c} is 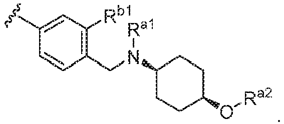 In certain embodiments, R^{1c} is 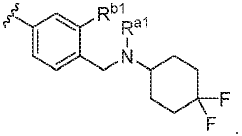 In certain embodiments, R^{1c} is 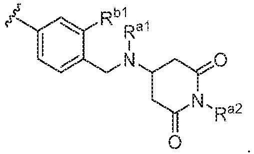 In certain embodiments, R^{1c} is 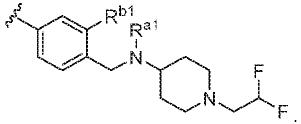 In certain embodiments, R^{1c} is 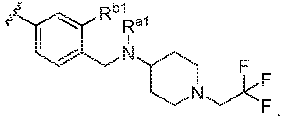 In certain embodiments, R^{1c} is 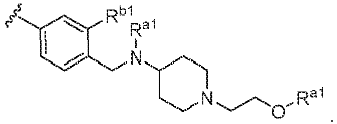 In certain embodiments, R^{1c} is 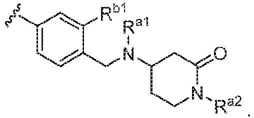 In certain embodiments, R^{1c} is 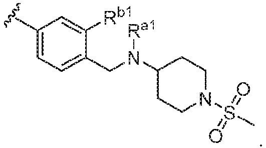 In certain embodiments, R^{1c} is 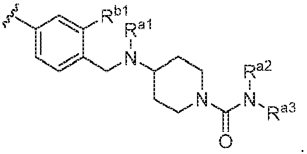 In certain embodiments, R^{1c} is 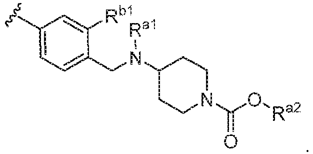 In certain embodiments, R^{1c} is 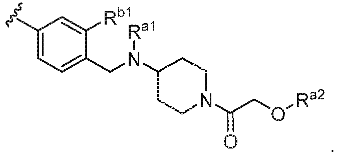 In certain embodiments, R^{1c} is 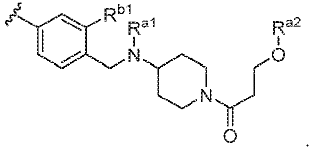 In certain embodiments, R^{1c} is 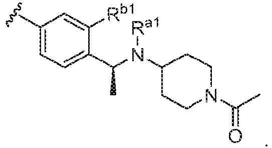 In certain embodiments, R^{1c} is 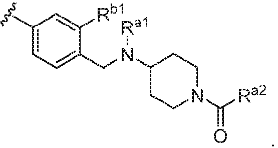 In certain embodiments, R^{1c} is 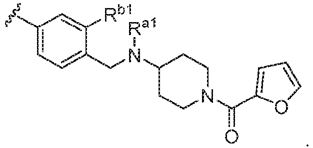 In certain embodiments, R^{1c} is 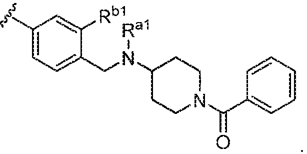 In certain embodiments, R^{1c} is 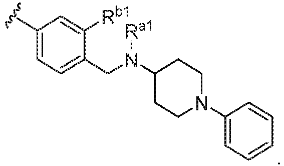 In certain embodiments, R^{1c} is 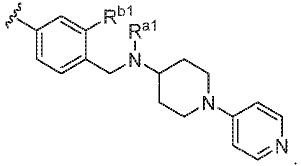 In certain embodiments, R^{1c} is 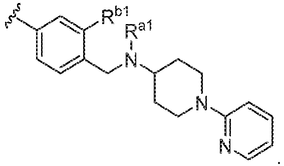 In certain embodiments, R^{1c} is 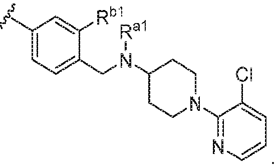 In certain embodiments, R^{1c} is 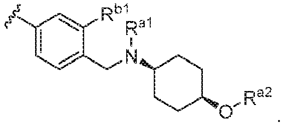 In certain embodiments, R^{1c} is 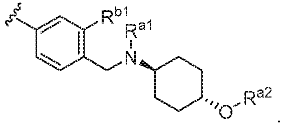 In certain embodiments, R^{1c} is 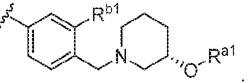 In certain embodiments, R^{1c} is 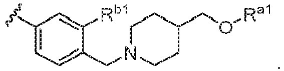 In certain embodiments, R^{1c} is 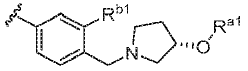 . In certain embodiments, R^{1c} is 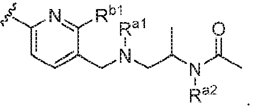 In certain embodiments, R^{1c} is 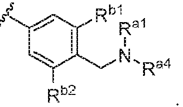 In certain embodiments, R^{1c} is 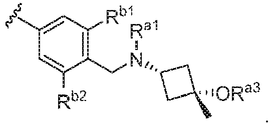 In certain embodiments, R^{1c} is 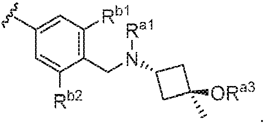 In certain embodiments, R^{1c} is 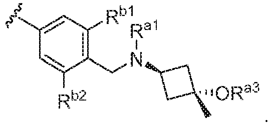 In certain embodiments, R^{1c} is 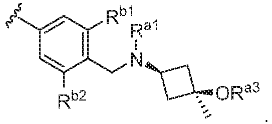 In certain embodiments, R^{1c} is 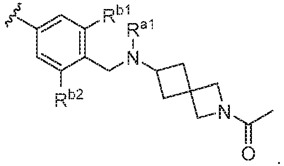 In certain embodiments, R^{1c} is 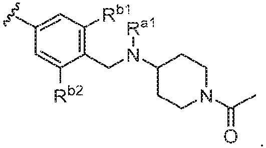 In certain embodiments, R^{1c} is 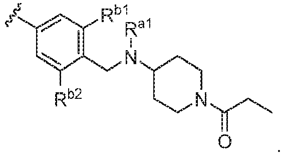 In certain embodiments, R^{1c} is 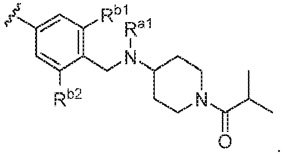 In certain embodiments, R^{1c} is 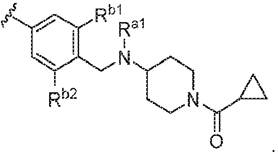 In certain embodiments, R^{1c} is 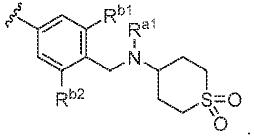 In certain embodiments, R^{1c} is 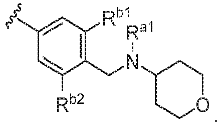 In certain embodiments, R^{1c} is 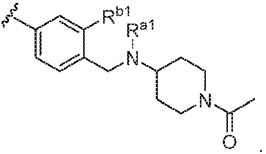 In certain embodiments, R^{1c} is 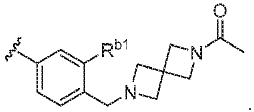 In certain embodiments, R^{1c} is 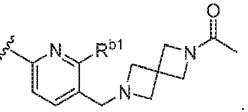 In certain embodiments, R^{1c} is 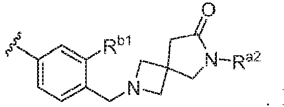 In certain embodiments, R^{1c} is 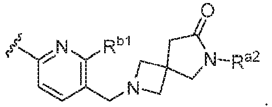 In certain embodiments, R^{1c} is 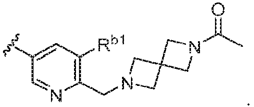 In certain embodiments, R^{1c} is 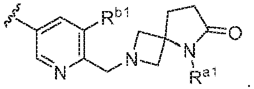 In certain embodiments, R^{1c} is 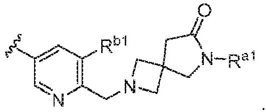 In certain embodiments, R^{1c} is 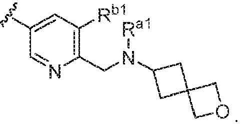 In certain embodiments, R^{1c} is 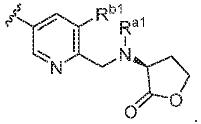 In certain embodiments, R^{1c} is 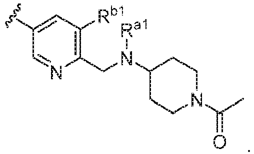 In certain embodiments, R^{1c} is 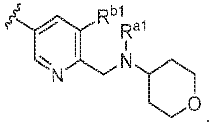 In certain embodiments, R^{1c} is 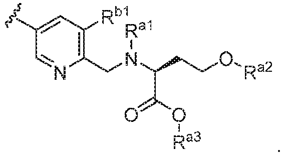 In certain embodiments, R^{1c} is 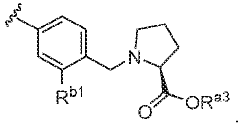 In certain embodiments, R^{1c} is 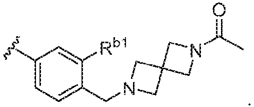 In certain embodiments, R^{1c} is 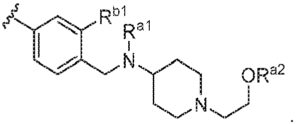 In certain embodiments, R^{1c} is 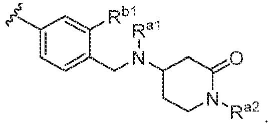 In certain embodiments, R^{1c} is 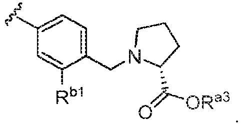 In certain embodiments, R^{1c} is 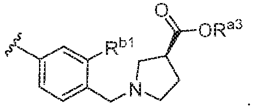 In certain embodiments, R^{1c} is 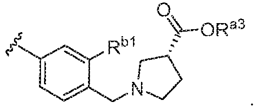 In certain embodiments, R^{1c} is 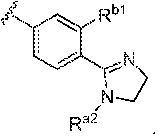 In certain embodiments, R^{1c} is 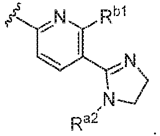 In certain embodiments, R^{1c} is 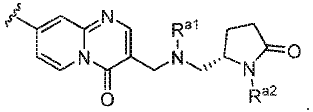 In certain embodiments, R^{1c} is 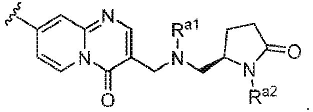 In certain embodiments, R^{1c} is 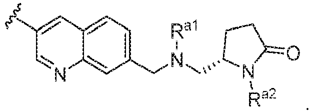 In certain embodiments, R^{1c} is 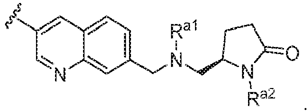 In certain embodiments, R^{1c} is 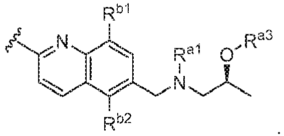 In certain embodiments, R^{1c} is 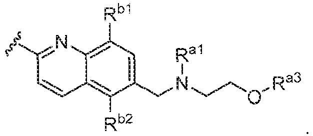 In certain embodiments, R^{1c} is 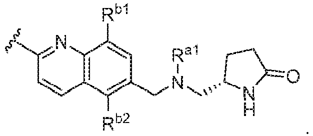 In certain embodiments, R^{1c} is 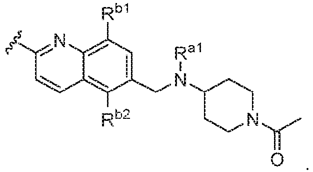 In certain embodiments, R^{1c} is 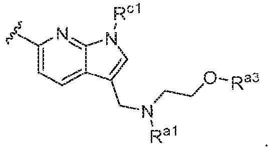 In certain embodiments, R^{1c} is 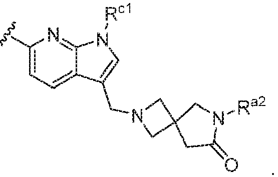 In certain embodiments, R^{1c} is 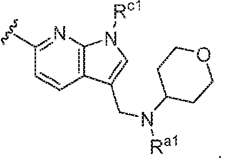 In certain embodiments, R^{1c} is 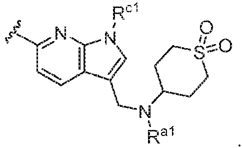 In certain embodiments, R^{1c} is 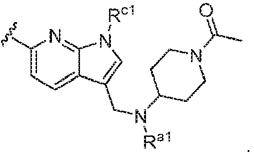 In certain embodiments, R^{1c} is 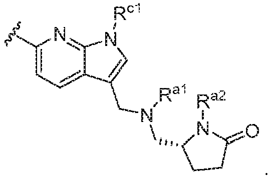 In certain embodiments, R^{1c} is 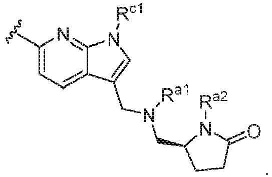 In certain embodiments, R^{1c} is 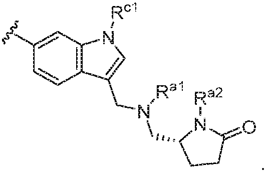 In certain embodiments, R^{1c} is 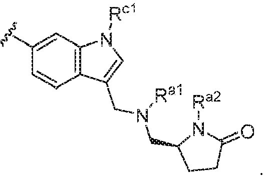 In certain embodiments, R^{1c} is 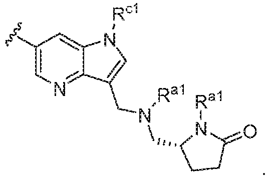 In certain embodiments, R^{1c} is 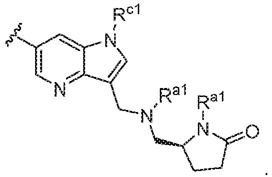 In certain embodiments, R^{1c} is 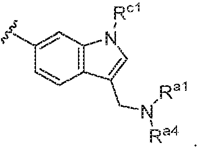 In certain embodiments, R^{1c} is 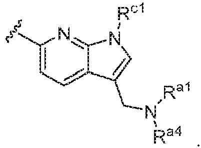 In certain embodiments, R^{1c} is 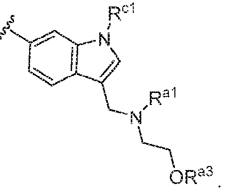 In certain embodiments, R^{1c} is 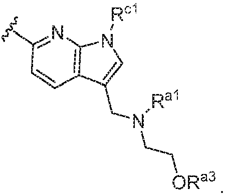 In certain embodiments, R^{1c} is 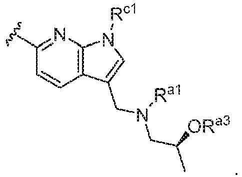 In certain embodiments, R^{1c} is 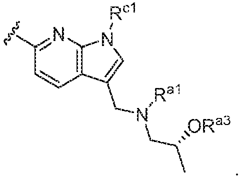 In certain embodiments, R^{1c} is 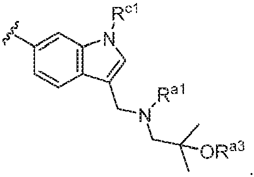 In certain embodiments, R^{1c} is 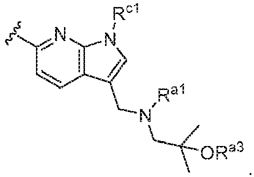 In certain embodiments, R^{1c} is 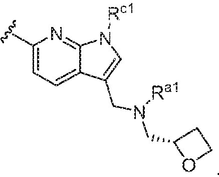 In certain embodiments, R^{1c} is 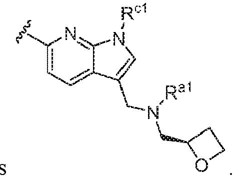 In certain embodiments, R^{1c} is 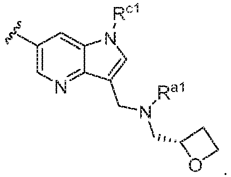 In certain embodiments, R^{1c} is 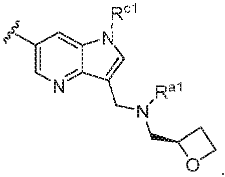 In certain embodiments, R^{1c} is 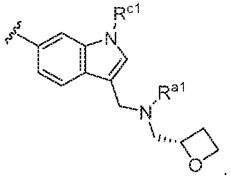 In certain embodiments, R^{1c} is 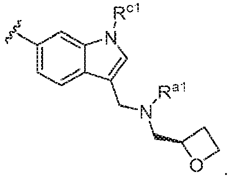 In certain embodiments, R^{1c} is 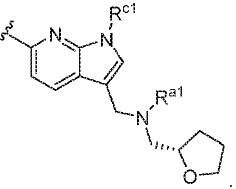 In certain embodiments, R^{1c} is 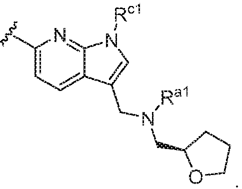 In certain embodiments, R^{1c} is 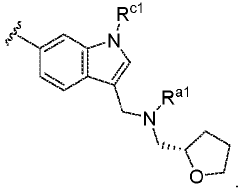 In certain embodiments, R^{1c} is 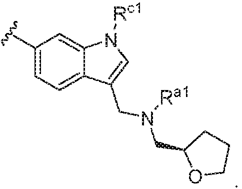 In certain embodiments, R^{1c} is 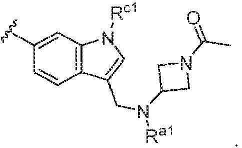 In certain embodiments, R^{1c} is 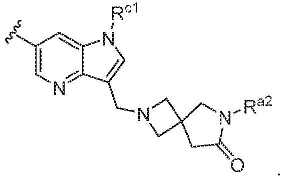 In certain embodiments, R^{1c} is 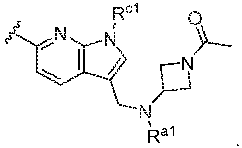 In certain embodiments, R^{1c} is 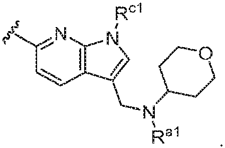 In certain embodiments, R^{1c} is 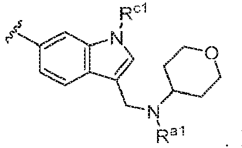 In certain embodiments, R^{1c} is 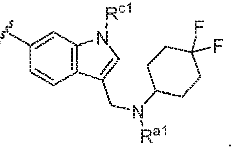 In certain embodiments, R^{1c} is 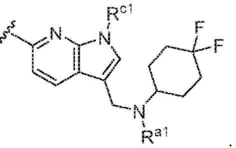 In certain embodiments, R^{1c} is 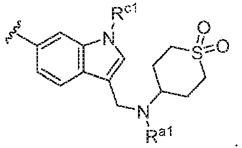 In certain embodiments, R^{1c} is 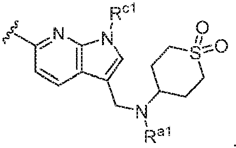 In certain embodiments, R^{1c} is 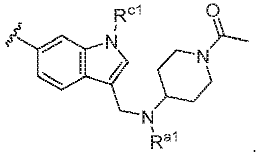 In certain embodiments, R^{1c} is 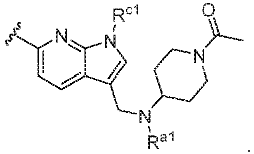 In certain embodiments, R^{1c} is 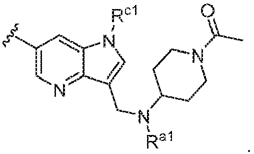 In certain embodiments, R^{1c} is 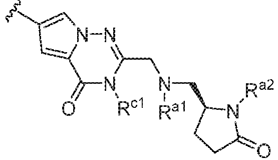 . In certain embodiments, R^{1c} is 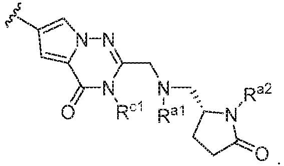 In certain embodiments, R^{1c} is 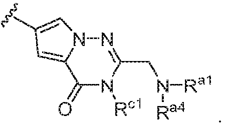 In certain embodiments, R^{1c} is 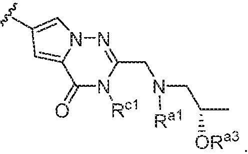 In certain embodiments, R^{1c} is 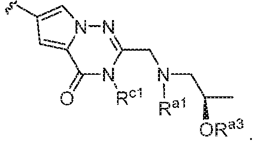 In certain embodiments, R^{1c} is 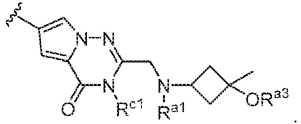 In certain embodiments, R^{1c} is 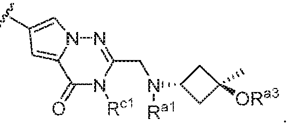 In certain embodiments, R^{1c} 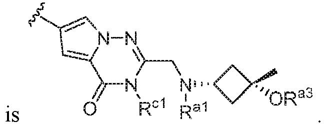 In certain embodiments, R^{1c} is 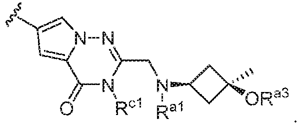 In certain embodiments, R^{1c} is 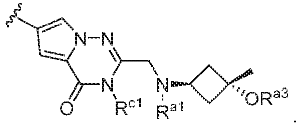 In certain embodiments, R^{1c} is 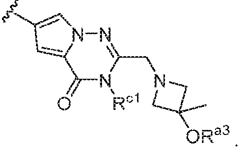 In certain embodiments, R^{1c} is 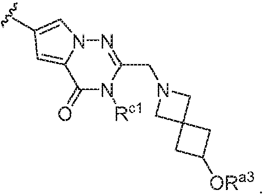 In certain embodiments, R^{1c} is 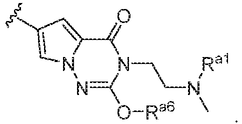 In certain embodiments, R^{1c} is 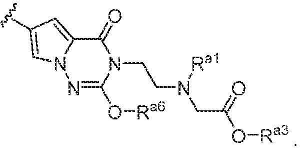 In certain embodiments, R^{1c} is 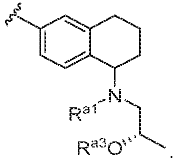 In certain embodiments, R^{1c} is 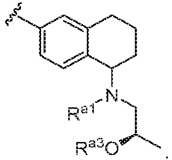 In certain embodiments, R^{1c} is 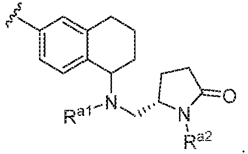 In certain embodiments, R^{1c} is 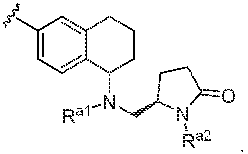 In certain embodiments, R^{1c} is 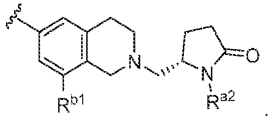 In certain embodiments, R^{1c} is 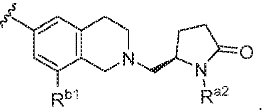 In certain embodiments, R^{1c} is 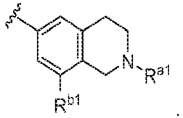 In certain embodiments, R^{1c} is 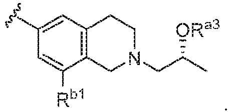 In certain embodiments, R^{1c} is 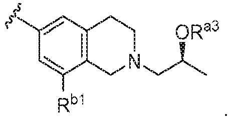 In certain embodiments, R^{1c} is 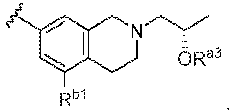 In certain embodiments, R^{1c} is 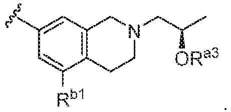 In certain embodiments, R^{1c} is 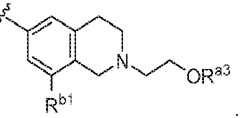 In certain embodiments, R^{1c} is 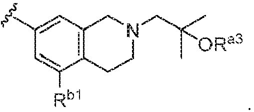 In certain embodiments, R^{1c} is 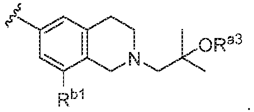 In certain embodiments, R^{1c} is 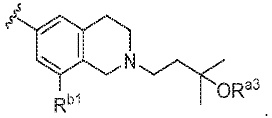 In certain embodiments, R^{1c} is 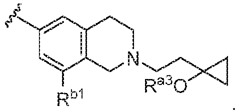 In certain embodiments, R^{1c} is 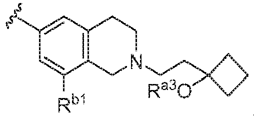 . In certain embodiments, R^{1c} is 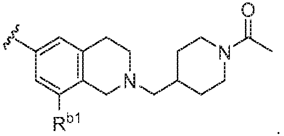 In certain embodiments, R^{1c} is 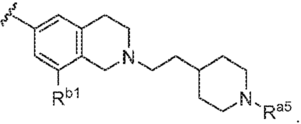 In certain embodiments, R^{1c} is 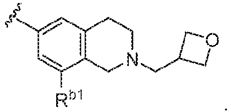 In certain embodiments, R^{1c} is 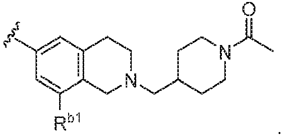 In certain embodiments, R^{1c} is 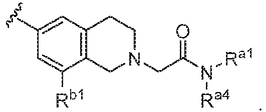 In certain embodiments, R^{1c} is 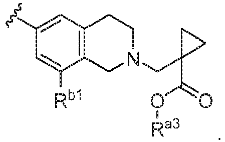 In certain embodiments, R^{1c} is 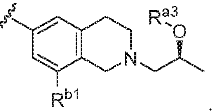 In certain embodiments, R^{1c} is 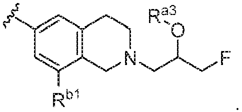 In certain embodiments, R^{1c} is 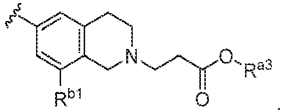 In certain embodiments, R^{1c} is 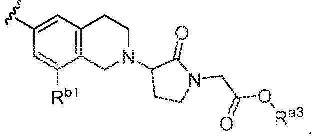 In certain embodiments, R^{1c} is 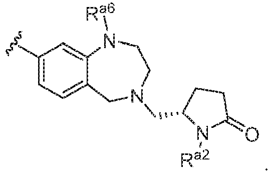 In certain embodiments, R^{1c} is 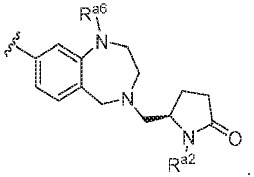 In certain embodiments, R^{1c} is 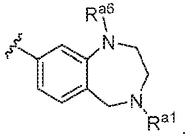 In certain embodiments, R^{1c} is 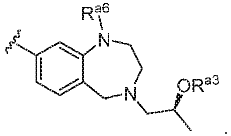 In certain embodiments, R^{1c} is 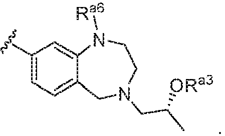 In certain embodiments, R^{1c} is 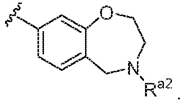 In certain embodiments, R^{1c} is 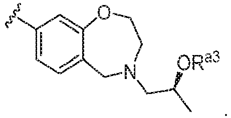 In certain embodiments, R^{1c} is 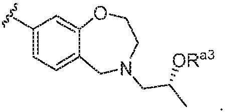 In certain embodiments, R^{1c} is 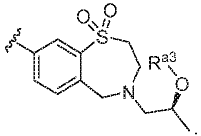 In certain embodiments, R^{1c} is 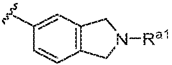 . In certain embodiments, R^{1c} is 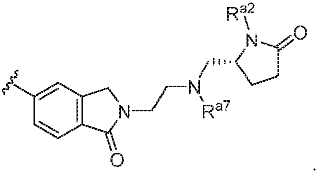 In certain embodiments, R^{1c} is 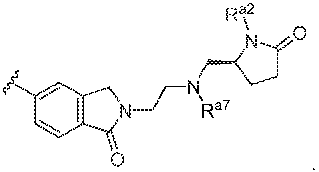 In certain embodiments, R^{1c} is 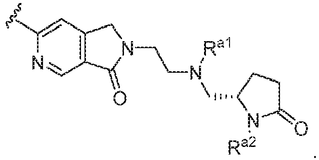 In certain embodiments, R^{1c} is 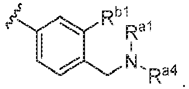 In certain embodiments, R^{1c} is 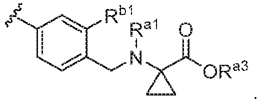 In certain embodiments, R^{1c} is 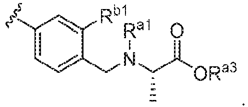 In certain embodiments, R^{1c} is 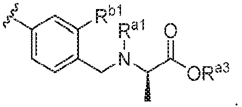 In certain embodiments, R^{1c} is 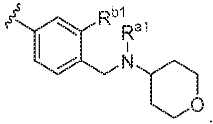 In certain embodiments, R^{1c} is 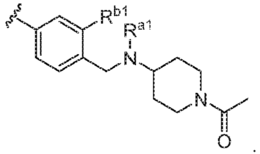 In certain embodiments, R^{1c} is 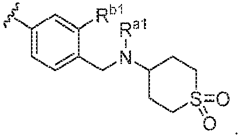 In certain embodiments, R^{1c} is 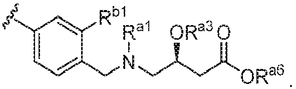 In certain embodiments, R^{1c} is 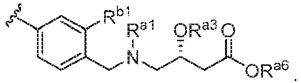 In certain embodiments, R^{1c} is 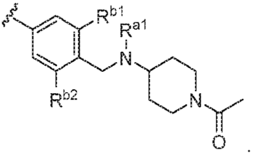 In certain embodiments, R^{1c} is 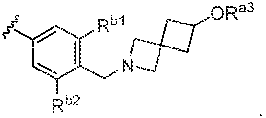 In certain embodiments, R^{1c} is 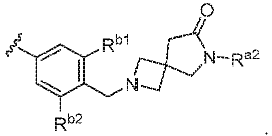 In certain embodiments, R^{1c} is 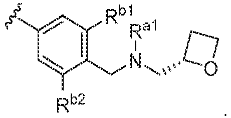 In certain embodiments, R^{1c} is 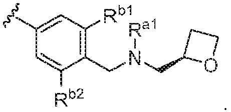 In certain embodiments, R^{1c} is 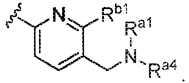 In certain embodiments, R^{1c} is 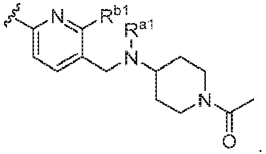 In certain embodiments, R^{1c} is 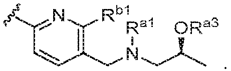 In certain embodiments, R^{1c} is 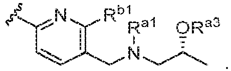 In certain embodiments, R^{1c} is 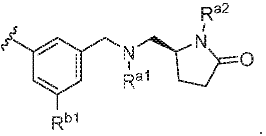 In certain embodiments, R^{1c} is 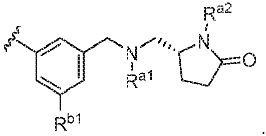 In certain embodiments, R^{1c} is 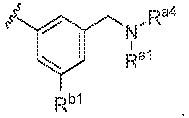 In certain embodiments, R^{1c} is 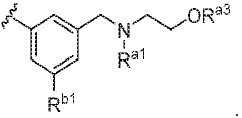 In certain embodiments, R^{1c} is 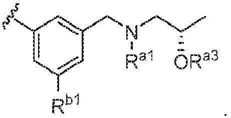 In certain embodiments, R^{1c} is 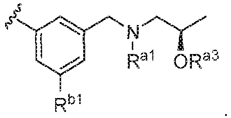 In certain embodiments, R^{1c} is 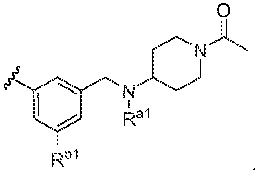 In certain embodiments, R^{1c} is 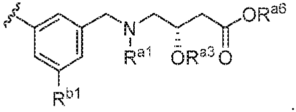 In certain embodiments, R^{1c} is 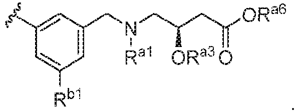 In certain embodiments, R^{1c} is 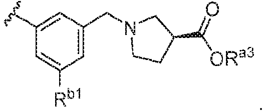 In certain embodiments, R^{1c} is 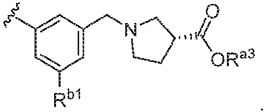 In certain embodiments, R^{1c} is 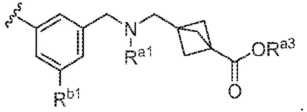 In certain embodiments, R^{1c} is 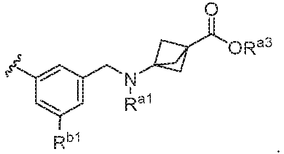 In certain embodiments, R^{1c} is 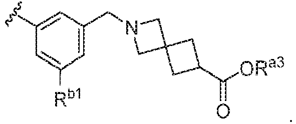 In certain embodiments, R^{1c} is 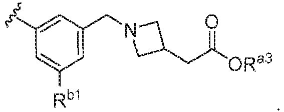 In certain embodiments, R^{1c} is 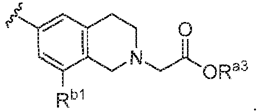 In certain embodiments, R^{1c} is 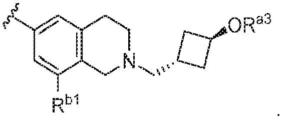 In certain embodiments, R^{1c} is 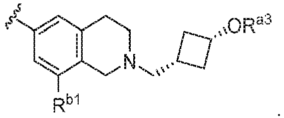 In certain embodiments, R^{1c} is 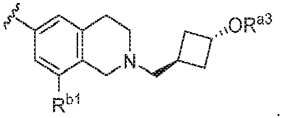 In certain embodiments, R^{1c} is 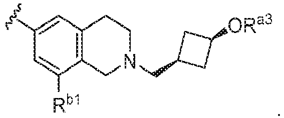 In certain embodiments, R^{1c} is 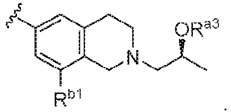 In certain embodiments, R^{1c} is 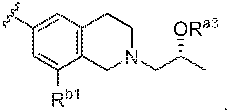 In certain embodiments, R^{1c} is 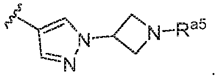 In certain embodiments, R^{1c} is 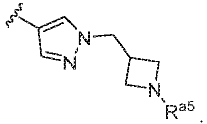 In certain embodiments, R^{1c} is 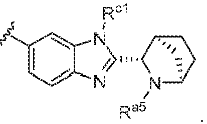 In certain embodiments, R^{1c} is 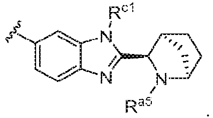 In certain embodiments, R^{1c} is 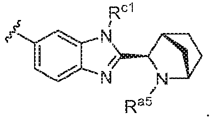 In certain embodiments, R^{1c} is 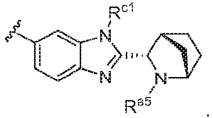 In certain embodiments, R^{1c} is 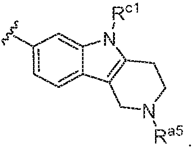 In certain embodiments, R^{1c} is 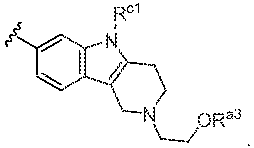 In certain embodiments, R^{1c} is 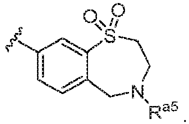 In certain embodiments, R^{1c} is 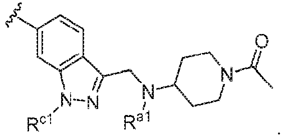 In certain embodiments, R^{1c} is 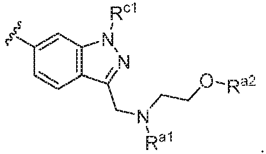 In certain embodiments, R^{1c} is 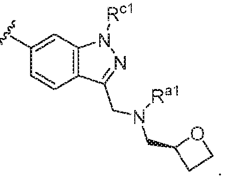 In certain embodiments, R^{1c} is 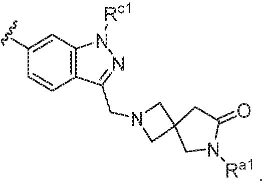 In certain embodiments, R^{1c} is 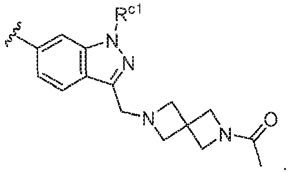 In certain embodiments, R^{1c} is 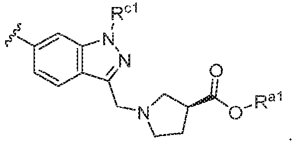 In certain embodiments, R^{1c} is 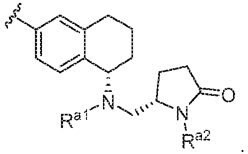 In certain embodiments, R^{1c} is 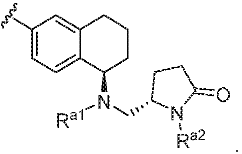 In certain embodiments, R^{1c} is 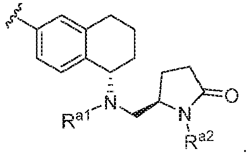 I In certain embodiments, R^{1c} is 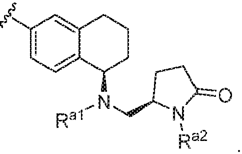 In certain embodiments, R^{1c} is 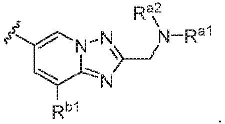 In certain embodiments, R^{1c} is 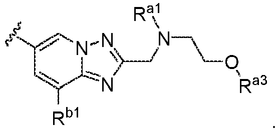 In certain embodiments, R^{1c} is 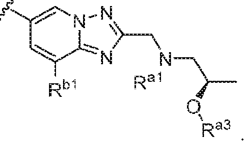 In certain embodiments, R^{1c} is 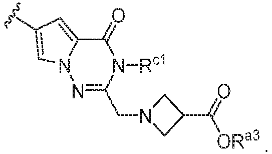 In certain embodiments, R^{1c} is 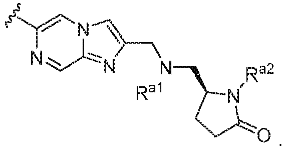 In certain embodiments, R^{1c} is 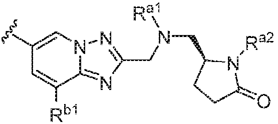 . In certain embodiments, R^{1c} is 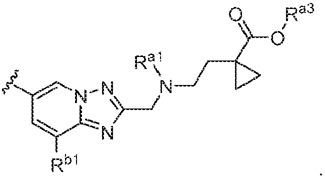 In certain embodiments, R^{1c} is 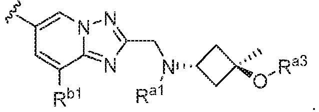 In certain embodiments, R^{1c} is 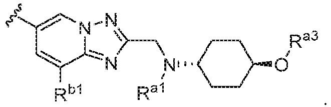 In certain embodiments, R^{1c} is 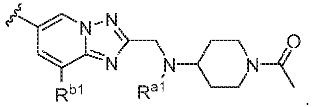 In certain embodiments, R^{1c} is 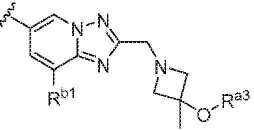 . In certain embodiments, R^{1c} is 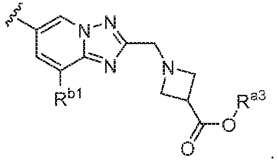 In certain embodiments, R^{1c} is 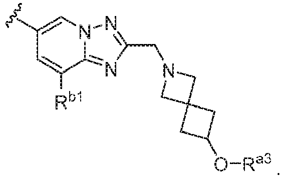 In certain embodiments, R^{1c} is 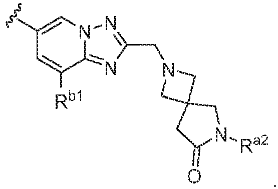 In certain embodiments, R^{1c} is 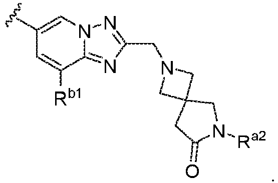 In certain embodiments, R^{1c} is 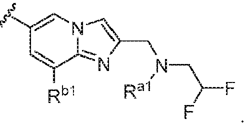 In certain embodiments, R^{1c} is 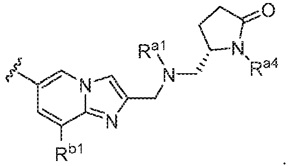 In certain embodiments, R^{1c} is 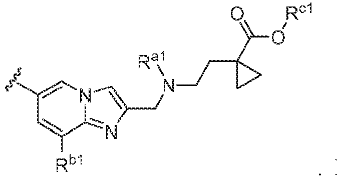 In certain embodiments, R^{1c} is 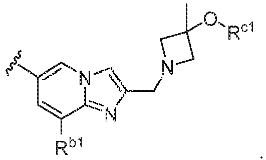 In certain embodiments, R^{1c} is 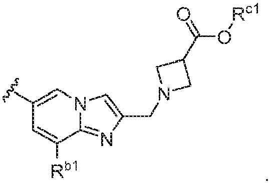 In certain embodiments, R^{1c} is 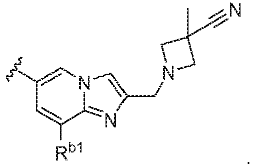 In certain embodiments, R^{1c} is 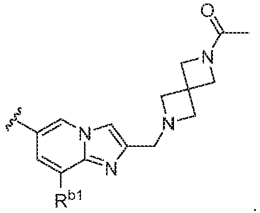 In certain embodiments, R^{1c} is 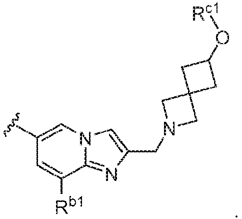 In
certain embodiments, R^{1c} is 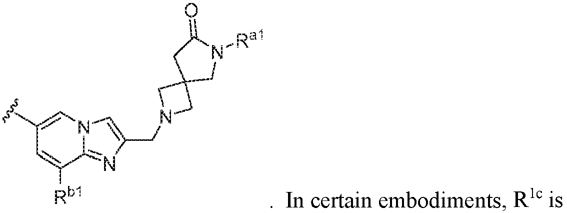 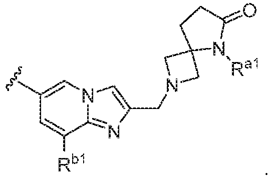 In certain embodiments, R^{1c} is 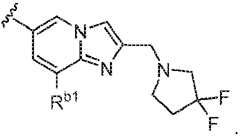 In certain embodiments, R^{1c} is 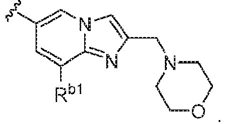 In certain embodiments, R^{1c} is 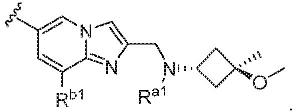 In certain embodiments, R^{1c} is 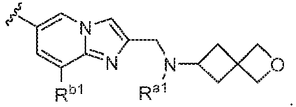 In certain embodiments, R^{1c} is 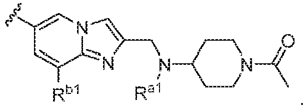 In certain embodiments, R^{1c} is 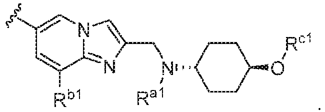 In certain embodiments, R^{1c} is 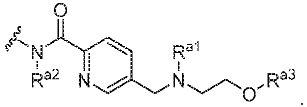 In certain embodiments, R^{1c} is 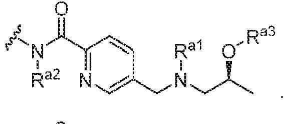 In certain embodiments, R^{1c} is 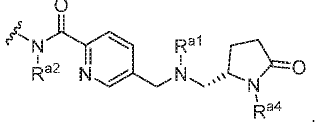 In certain embodiments, R^{1c} is 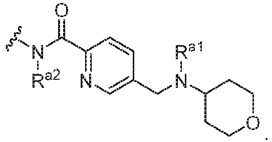 In certain embodiments, R^{1c} is 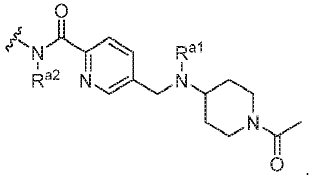 In certain embodiments, R^{1c} is 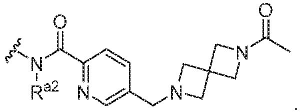 In certain embodiments, R^{1c} is 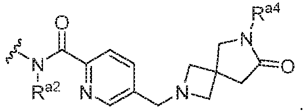 In certain embodiments, R^{1c} is 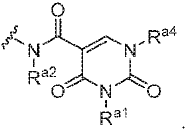 In certain embodiments, R^{1c} is 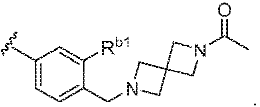 In certain embodiments, R^{1c} is 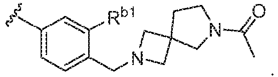 In certain embodiments, R^{1c} is 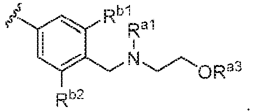 In certain embodiments, R^{1c} is 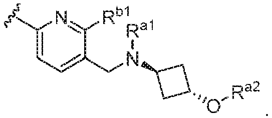 In certain embodiments, R^{1c} is 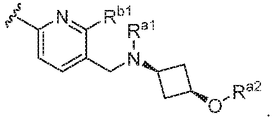 In certain embodiments, R^{1c} is 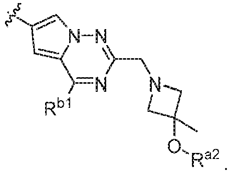 In certain embodiments, R^{1c} is 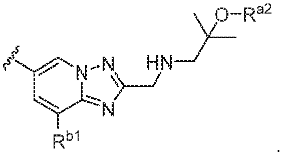 In certain embodiments, R^{1c} is 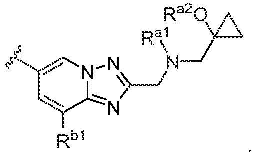 In certain embodiments, R^{1c} is 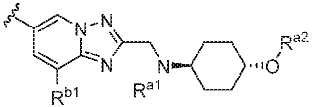 . In certain embodiments, R^{1c} is 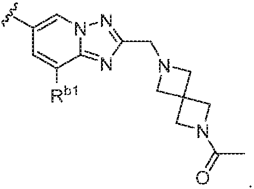 In certain embodiments, R^{1c} is 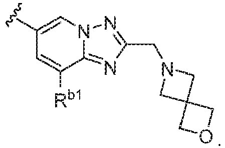 In certain embodiments, R^{1c} is 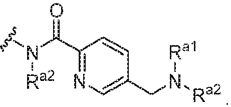 In certain embodiments, R^{1c} is 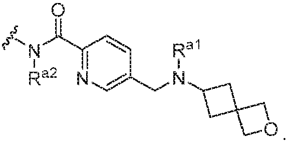 In certain embodiments, R^{1c} is 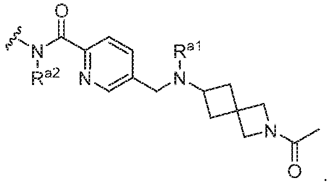 In certain embodiments, R^{1d} is 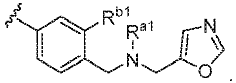 In certain embodiments, R^{1d} is 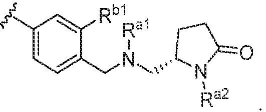 In certain embodiments, R^{1d} is 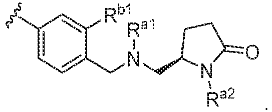 In certain embodiments, R^{1d} is 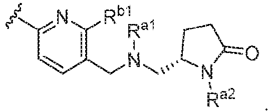 In certain embodiments, R^{1d} is 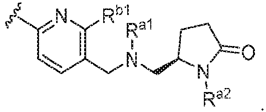 In certain embodiments, R^{1d} is 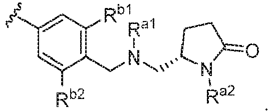 In certain embodiments, R^{1d} is 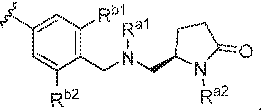 In certain embodiments, R^{1d} is 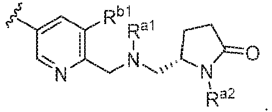 In certain embodiments, R^{1d} is 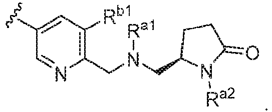 In certain embodiments, R^{1d} is 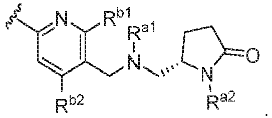 In certain embodiments, R^{1d} is 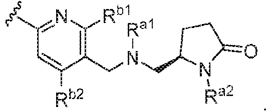 In certain embodiments, R^{1d} is 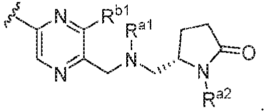 In certain embodiments, R^{1d} is 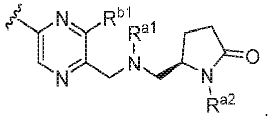 In certain embodiments, R^{1d} is 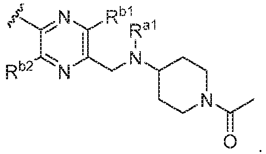 In certain embodiments, R^{1d} is 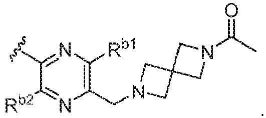 In certain embodiments, R^{1d} is 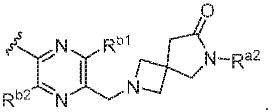 In certain embodiments, R^{1d} is 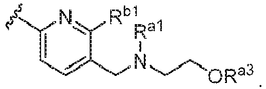 In certain embodiments, R^{1d} is 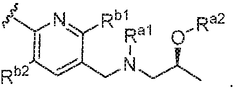 In certain embodiments, R^{1d} is 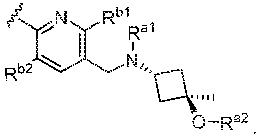 In certain embodiments, R^{1d} is 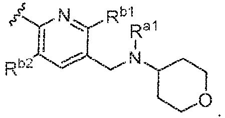 In certain embodiments, R^{1d} is 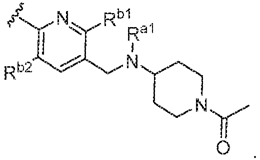 In certain embodiments, R^{1d} is 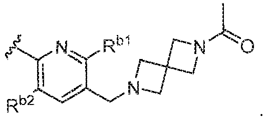 In certain embodiments, R^{1d} is 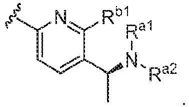 In certain embodiments, R^{1d} is 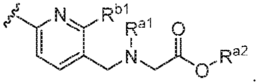 In certain embodiments, R^{1d} is 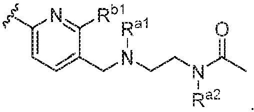 In certain embodiments, R^{1d} is 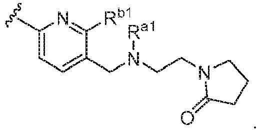 In certain embodiments, R^{1d} is 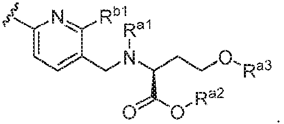 In certain embodiments, R^{1d} is 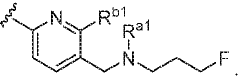 In certain embodiments, R^{1d} is 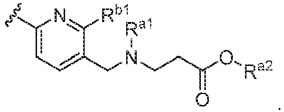 In certain embodiments, R^{1d} is 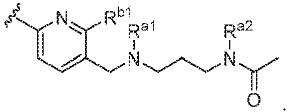 In certain embodiments, R^{1d} is 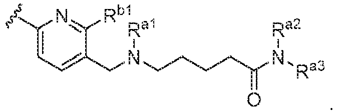 In certain embodiments, R^{1d} is 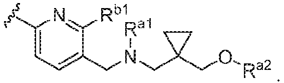 In certain embodiments, R^{1d} is 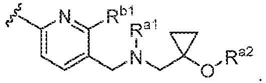 In certain embodiments, R^{1d} is 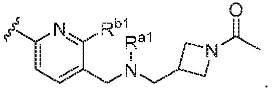 In certain embodiments, R^{1d} is 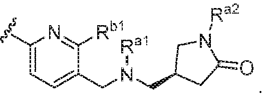 In certain embodiments, R^{1d} is 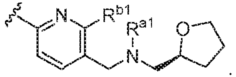 In certain embodiments, R^{1d} is 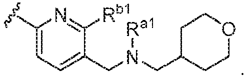 In certain embodiments, R^{1d} is 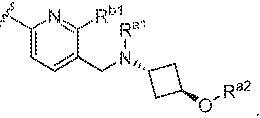 In certain embodiments, R^{1d} is 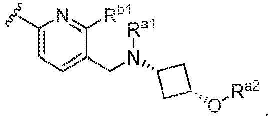 In certain embodiments, R^{1d} is 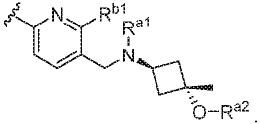 In certain embodiments, R^{1d} is 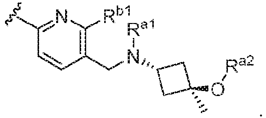 In certain embodiments, R^{1d} is 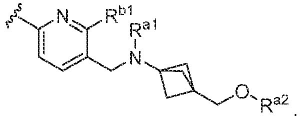 In certain embodiments, R^{1d} is 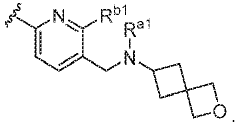 In certain embodiments, R^{1d} is 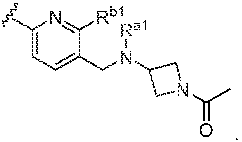 In certain embodiments, R^{1d} is 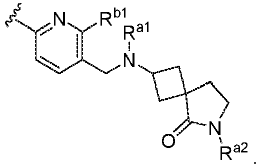 I In certain embodiments, R^{1d} is 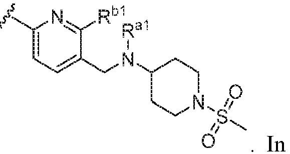 I certain embodiments, R^{1d} is 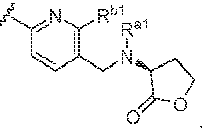 In certain embodiments, R^{1d} is 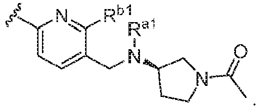 In certain embodiments, R^{1d} is 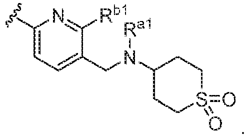 In certain embodiments, R^{1d} is 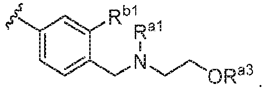 In certain embodiments, R^{1d} is 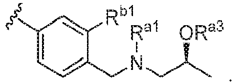 In certain embodiments, R^{1d} is 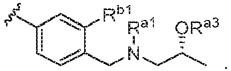 In certain embodiments, R^{1d} is 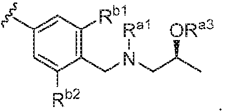 In certain embodiments, R^{1d} is 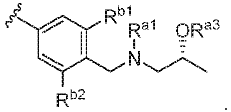 In certain embodiments, R^{1d} is 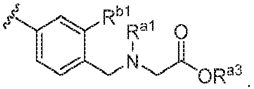 In certain embodiments, R^{1d} is 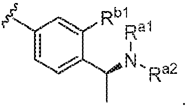 In certain embodiments, R^{1d} is 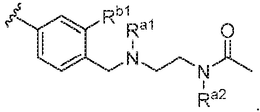 In certain embodiments, R^{1d} is 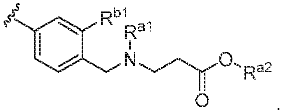 In certain embodiments, R^{1d} is 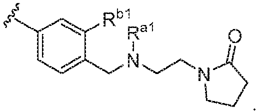 In certain embodiments, R^{1d} is 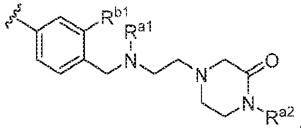 In certain embodiments, R^{1d} is 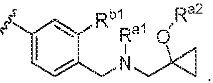 In certain embodiments, R^{1d} is 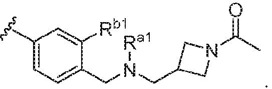 In certain embodiments, R^{1d} is 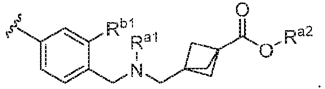 In certain embodiments, R^{1d} is 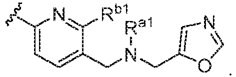 In certain embodiments, R^{1d} is 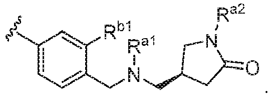 In certain embodiments, R^{1d} is 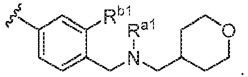 In certain embodiments, R^{1d} is 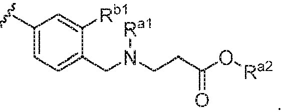 In certain embodiments, R^{1d} is 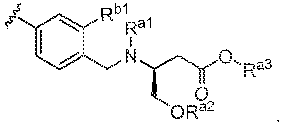 In certain embodiments, R^{1d} is 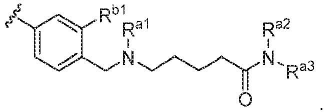 In certain embodiments, R^{1d} is 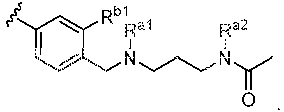 In certain embodiments, R^{1d} is 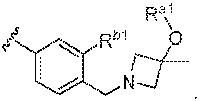 In certain embodiments, R^{1d} is 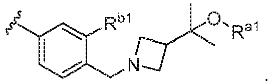 In certain embodiments, R^{1d} is 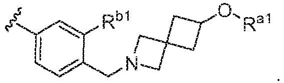 In certain embodiments, R^{1d} is 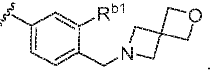 In certain embodiments, R^{1d} is 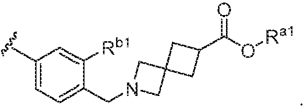 In certain embodiments, R^{1d} is 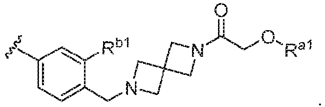 In certain embodiments, R^{1d} is 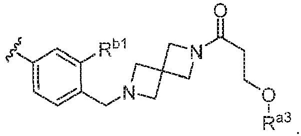 In certain embodiments, R^{1d} is 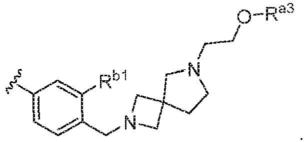 In certain embodiments, R^{1d} is 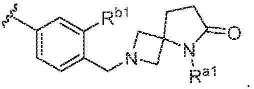 In certain embodiments, R^{1d} is 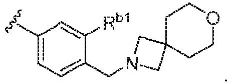 In certain embodiments, R^{1d} is 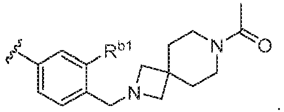 In certain embodiments, R^{1d} is 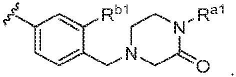 In certain embodiments, R^{1d} is 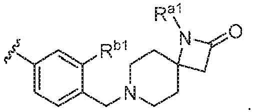 In certain embodiments, R^{1d} is 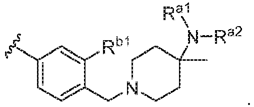 In certain embodiments, R^{1d} is 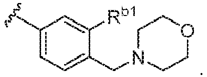 In certain embodiments, R^{1d} is 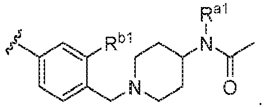 In certain embodiments, R^{1d} is 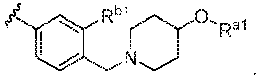 In certain embodiments, R^{1d} is 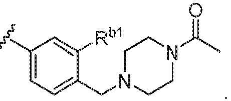 In certain embodiments, R^{1d} is 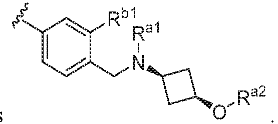 In certain embodiments, R^{1d} is 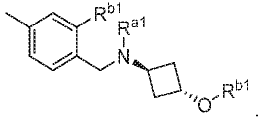 In certain embodiments, R^{1d} is 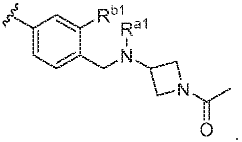 In certain embodiments, R^{1d} is 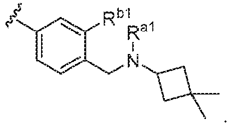 In certain embodiments, R^{1d} is 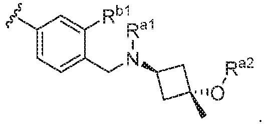 In certain embodiments, R^{1d} is 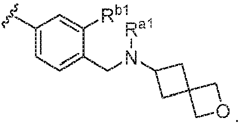 In certain embodiments, R^{1d} is 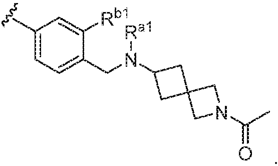 In certain embodiments, R^{1d} is 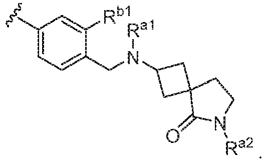 In certain embodiments, R^{1d} is 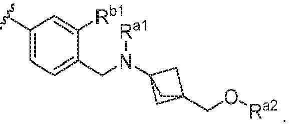 In certain embodiments, R^{1d} is 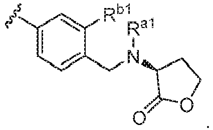 In certain embodiments, R^{1d} is 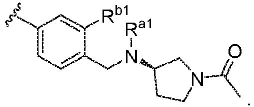 In certain embodiments, R^{1d} is 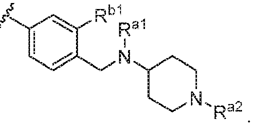 In certain embodiments, R^{1d} is 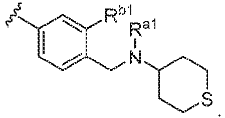 In certain embodiments, R^{1d} is 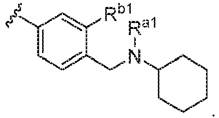 In certain embodiments, R^{1d} is 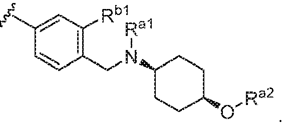 In certain embodiments, R^{1d} is 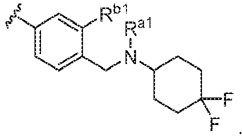 In certain embodiments, R^{1d} is 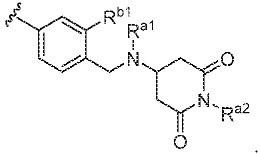 In certain embodiments, R^{1d} is 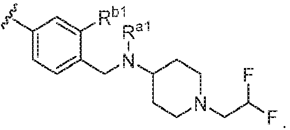 In certain embodiments, R^{1d} is 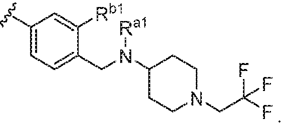 In certain embodiments, R^{1d} is 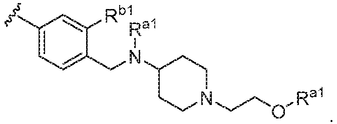 In certain embodiments, R^{1d} is 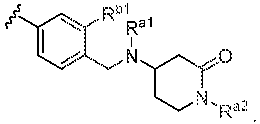 In certain embodiments, R^{1d} is 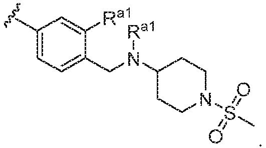 In certain embodiments, R^{1d} is 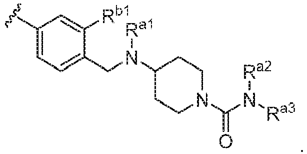 In certain embodiments, R^{1d} is 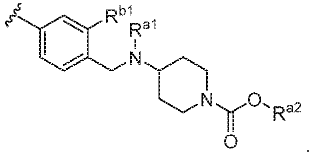 In certain embodiments, R^{1d} is 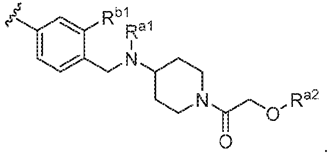 In certain embodiments, R^{1d} is 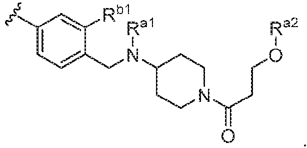 In certain embodiments, R^{1d} is 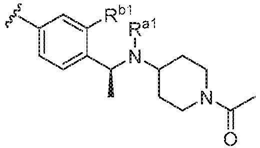 . In certain embodiments, R^{1d} is 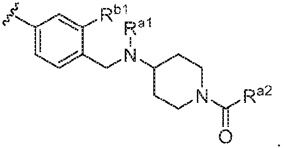 In certain embodiments, R^{1d} is 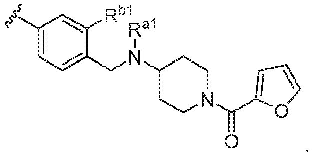 In certain embodiments, R^{1d} is 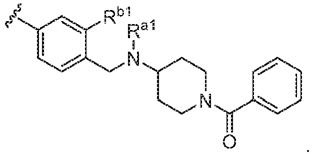 In certain embodiments, R^{1d} is 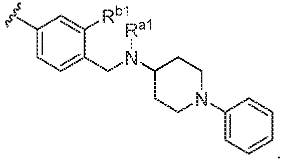 In certain embodiments, R^{1d} is 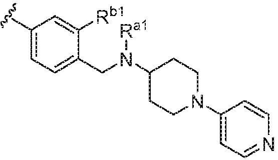 . In certain embodiments, R^{1d} is 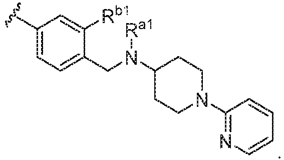 In certain embodiments, R^{1d} is 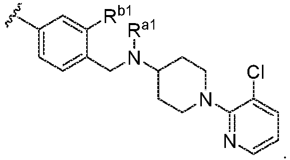 In certain embodiments, R^{1d} is 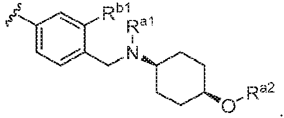 In certain embodiments, R^{1d} is 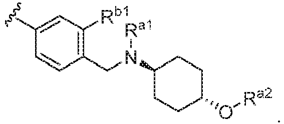 In certain embodiments, R^{1d} is 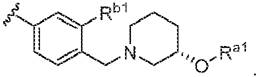 In certain embodiments, R^{1d} is 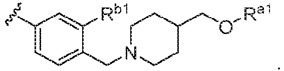 In certain embodiments, R^{1d} is 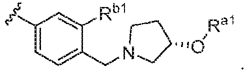 In certain embodiments, R^{1d} is 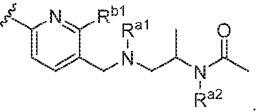 In certain embodiments, R^{1d} is 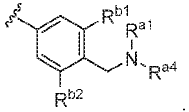 In certain embodiments, R^{1d} is 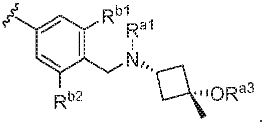 In certain embodiments, R^{1d} is 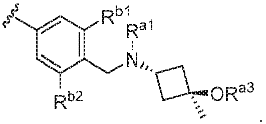 In certain embodiments, R^{1d} is 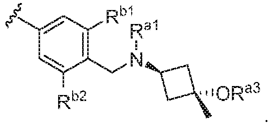 In certain embodiments, R^{1d} is 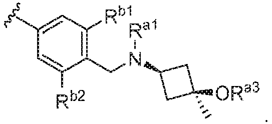 In certain embodiments, R^{1d} is 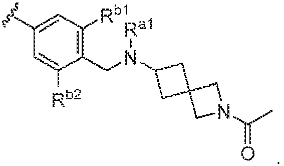 In certain embodiments, R^{1d} is 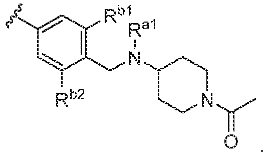 In certain embodiments, R^{1d} is 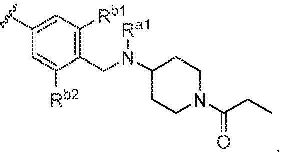 In certain embodiments, R^{1d} is 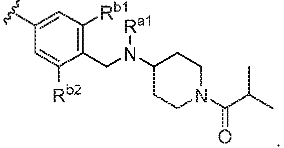 In certain embodiments, R^{1d} is 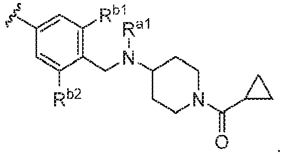 In certain embodiments, R^{1d} is 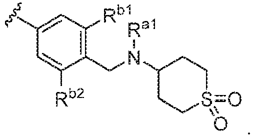 In certain embodiments, R^{1d} is 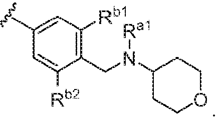 In certain embodiments, R^{1d} is 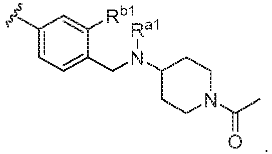 In certain embodiments, R^{1d} is 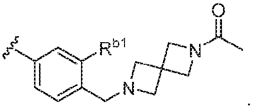 In certain embodiments, R^{1d} is 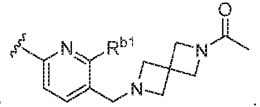 In certain embodiments, R^{1d} is 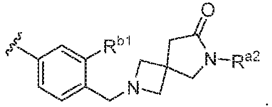 In certain embodiments, R^{1d} is 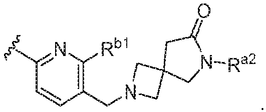 In certain embodiments, R^{1d} is 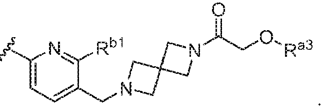 In certain embodiments, R^{1d} is 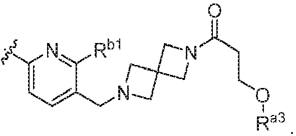 In certain embodiments, R^{1d} is 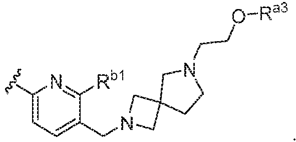 In certain embodiments, R^{1d} is 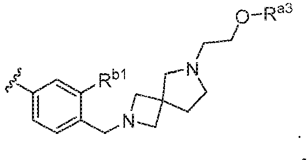 In certain embodiments, R^{1d} is 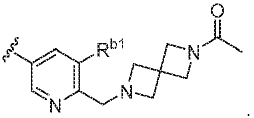 In certain embodiments, R^{1d} is 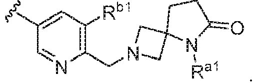 In certain embodiments, R^{1d} is 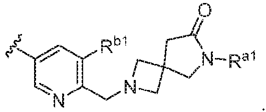 In certain embodiments, R^{1d} is 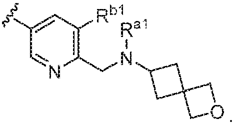 In certain embodiments, R^{1d} is 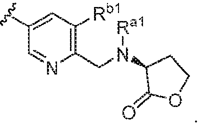 In certain embodiments, R^{1d} is 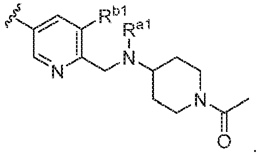 In certain embodiments, R^{1d} is 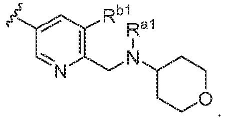 In certain embodiments, R^{1d} is 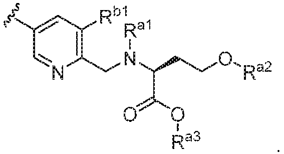 In certain embodiments, R^{1d} is 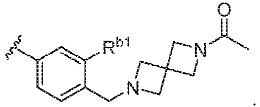 In certain embodiments, R^{1d} is 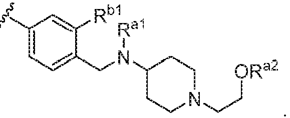 In certain embodiments, R^{1d} is 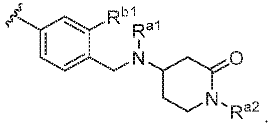 In certain embodiments, R^{1d} is 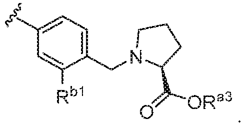 In certain embodiments, R^{1d} is 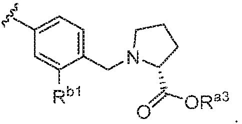 In certain embodiments, R^{1d} is 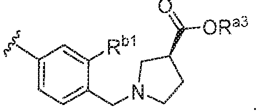 In certain embodiments, R^{1d} is 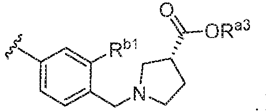 In certain embodiments, R^{1d} is 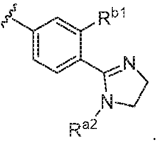 In certain embodiments, R^{1d} is 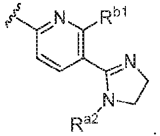 In certain embodiments, R^{1d} is 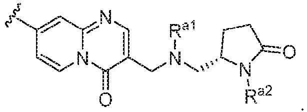 In certain embodiments, R^{1d} is 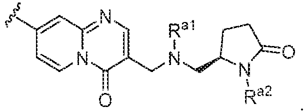 In certain embodiments, R^{1d} is 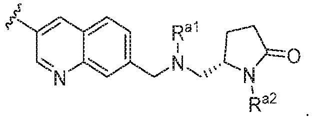 In certain embodiments, R^{1d} is 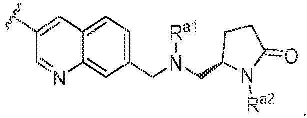 In certain embodiments, R^{1d} is 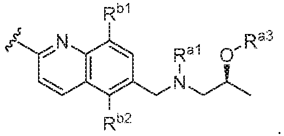 In certain embodiments, R^{1d} is 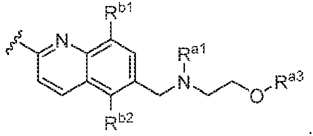 In certain embodiments, R^{1d} is 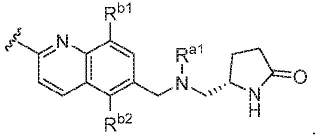 In certain embodiments, R^{1d} is 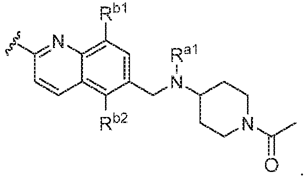 In certain embodiments, R^{1d} is 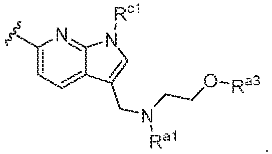 In certain embodiments, R^{1d} is 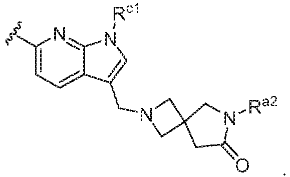 In certain embodiments, R^{1d} is 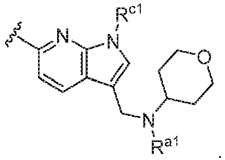 In certain embodiments, R^{1d} is 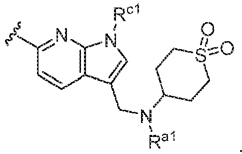 In certain embodiments, R^{1d} is 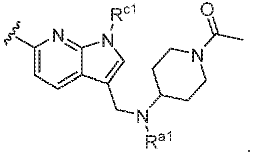 In certain embodiments, R^{1d} is 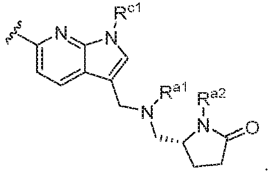 In certain embodiments, R^{1d} is 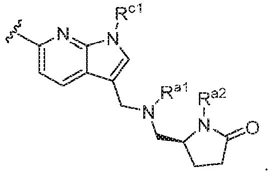 In certain embodiments, R^{1d} is 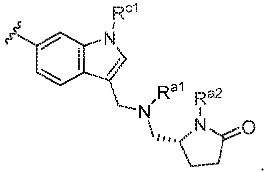 In certain embodiments, R^{1d} is 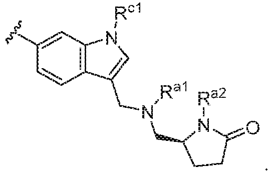 In certain embodiments, R^{1d} is 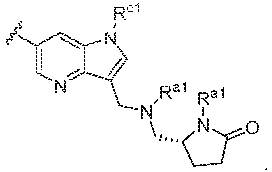 In certain embodiments, R^{1d} is 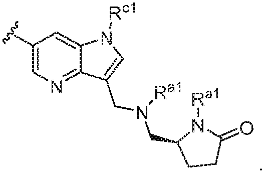 In certain embodiments, R^{1d} is 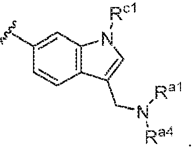 In certain embodiments, R^{1d} is 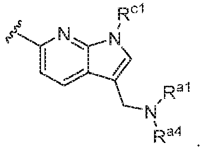 In certain embodiments, R^{1d} is 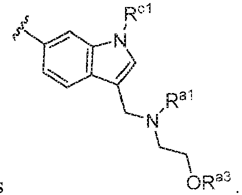 In certain embodiments, R^{1d} is 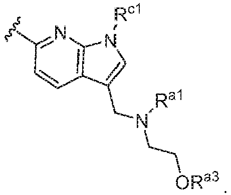 In certain embodiments, R^{1d} is 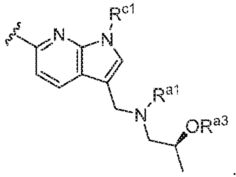 In certain embodiments, R^{1d} is 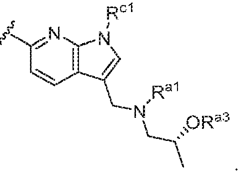 In certain embodiments, R^{1d} is 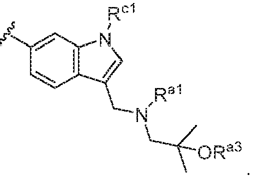 In certain embodiments, R^{1d} is 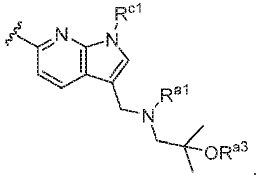 In certain embodiments, R^{1d} is 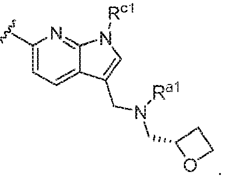 In certain embodiments, R^{1d} is 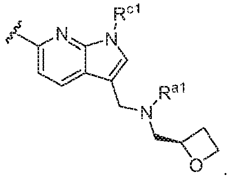 In certain embodiments, R^{1d} is 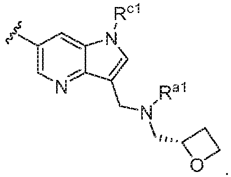 In certain embodiments, R^{1d} is 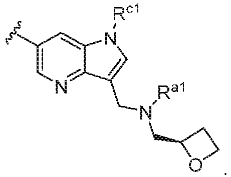 In certain embodiments, R^{1d} is 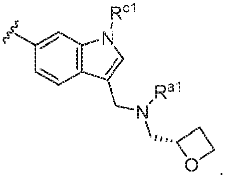 In certain embodiments, R^{1d} is 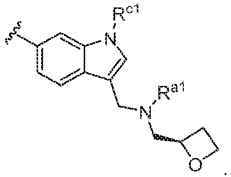 In certain embodiments, R^{1d} is 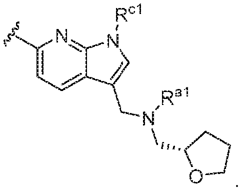 In certain embodiments, R^{1d} is 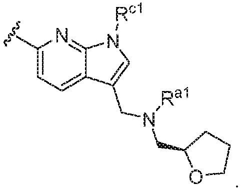 In certain embodiments, R^{1d} is 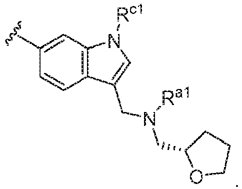 In certain embodiments, R^{1d} is 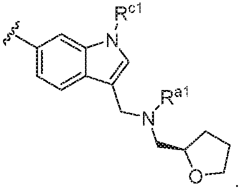 In certain embodiments, R^{1d} is 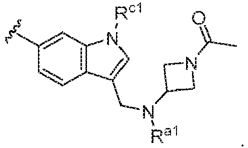 In certain embodiments, R^{1d} is 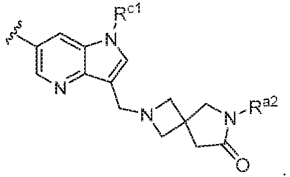 In certain embodiments, R^{1d} is 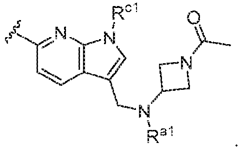 In certain embodiments, R^{1d} is 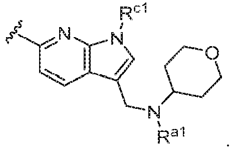 In certain embodiments, R^{1d} is 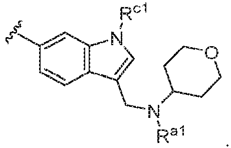 In certain embodiments, R^{1d} is 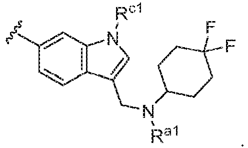 In certain embodiments, R^{1d} is 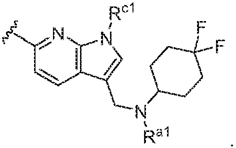 In certain embodiments, R^{1d} is 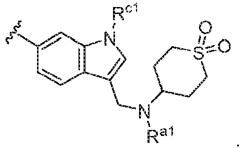 In certain embodiments, R^{1d} is 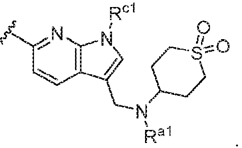 In certain embodiments, R^{1d} is 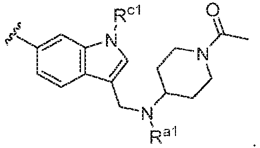 In certain embodiments, R^{1d} is 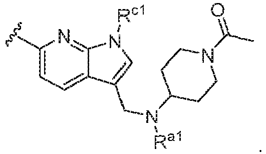 In certain embodiments, R^{1d} is 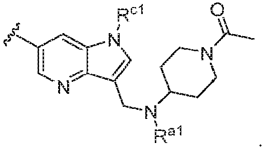 In certain embodiments, R^{1d} is 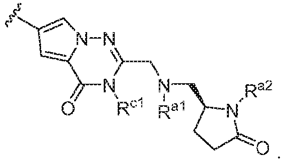 In certain embodiments, R^{1d} is 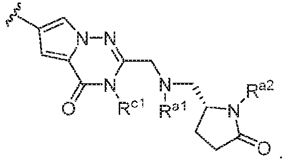 In certain embodiments, R^{1d} is 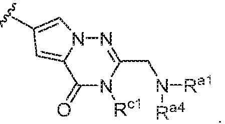 In certain embodiments, R^{1d} is 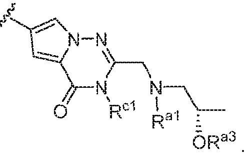 In certain embodiments, R^{1d} is 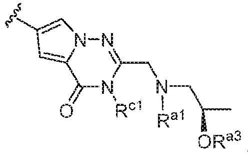 In certain embodiments, R^{1d} is 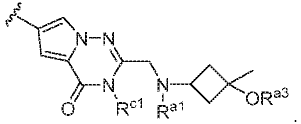 In certain embodiments, R^{1d} is 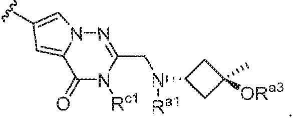 In certain embodiments, R^{1d} is 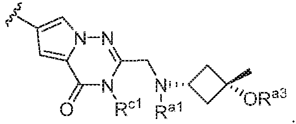 In certain embodiments, R^{1d} is 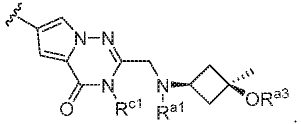 In certain embodiments, R^{1d} is 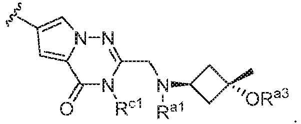 In certain embodiments, R^{1d} is 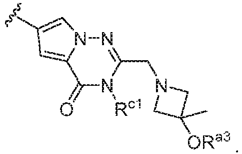 In certain embodiments, R^{1d} is 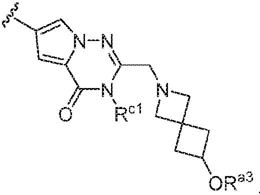 In certain embodiments, R^{1d} is 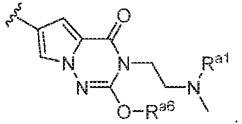 In certain embodiments, R^{1d} is 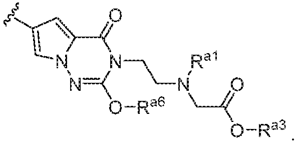 In certain embodiments, R^{1d} is 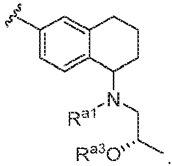 In certain embodiments, R^{1d} is 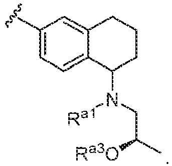 In certain embodiments, R^{1d} is 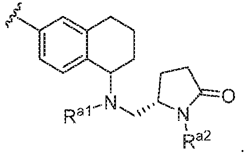 In certain embodiments, R^{1d} is 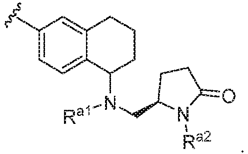 In certain embodiments, R^{1d} is 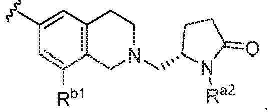 In certain embodiments, R^{1d} is 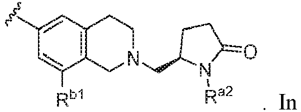 certain embodiments, R^{1d} is 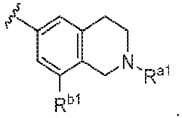 In certain embodiments, R^{1d} is 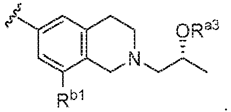 In certain embodiments, R^{1d} is 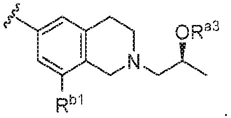 In certain embodiments, R^{1d} is 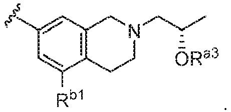 In certain embodiments, R^{1d} is 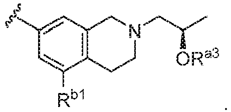 In certain embodiments, R^{1d} is 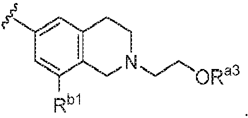 In certain embodiments, R^{1d} is 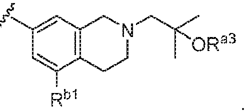 In certain embodiments, R^{1d} is 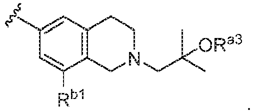 In certain embodiments, R^{1d} is 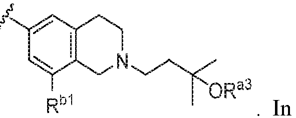 certain embodiments, R^{1d} is 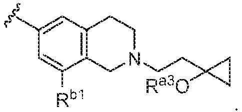 In certain embodiments, R^{1d} is 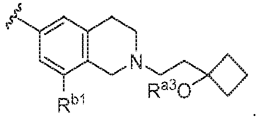 In certain embodiments, R^{1d} is 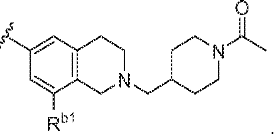 In certain embodiments, R^{1d} is 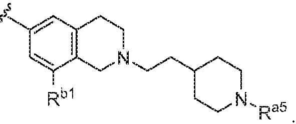 In certain embodiments, R^{1d} is 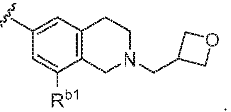 In certain embodiments, R^{1d} is 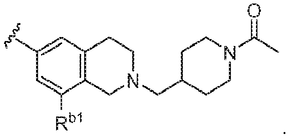 In certain embodiments, R^{1d} is 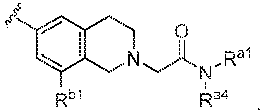 In certain embodiments, R^{1d} is 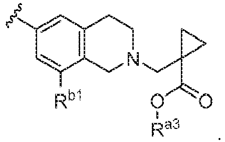 In certain embodiments, R^{1d} is 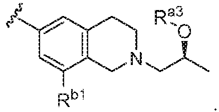 In certain embodiments, R^{1d} is 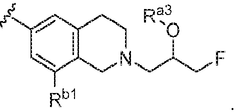 In certain embodiments, R^{1d} is 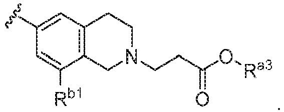 In certain embodiments, R^{1d} is 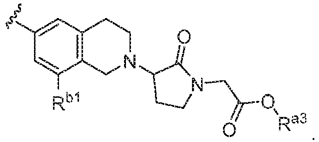 In certain embodiments, R^{1d} is 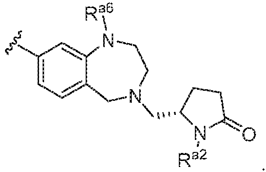 In certain embodiments, R^{1d} is 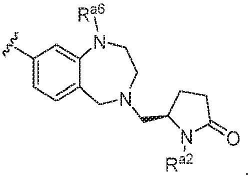 In certain embodiments, R^{1d} is 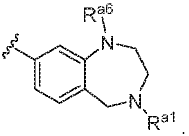 In certain embodiments, R^{1d} is 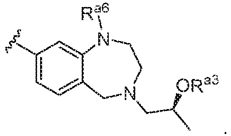 In certain embodiments, R^{1d} is 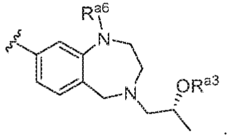 In certain embodiments, R^{1d} is 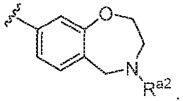 In certain embodiments, R^{1d} is 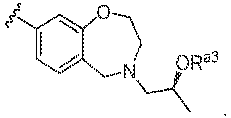 In certain embodiments, R^{1d} is 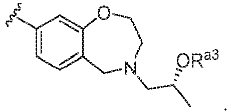 In certain embodiments, R^{1d} is 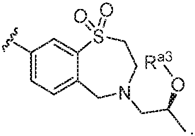 In certain embodiments, R^{1d} is 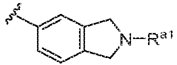 . In certain embodiments, R^{1d} is 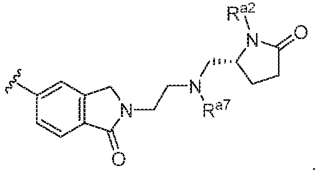 In certain embodiments, R^{1d} is 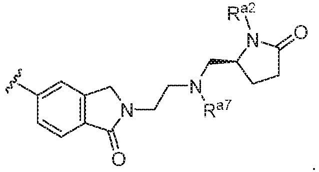 In certain embodiments, R^{1d} is 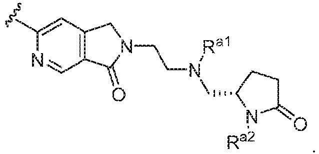 In certain embodiments, R^{1d} is 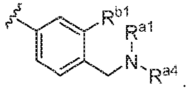 In certain embodiments, R^{1d} is 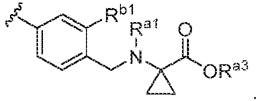 In certain embodiments, R^{1d} is 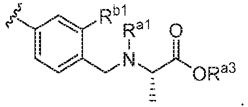 In certain embodiments, R^{1d} is 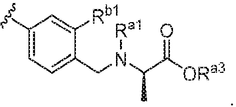 In certain embodiments, R^{1d} is 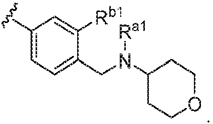 In certain embodiments, R^{1d} is 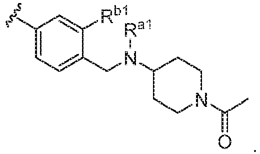 In certain embodiments, R^{1d} is 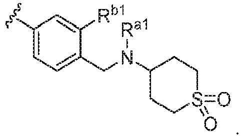 In certain embodiments, R^{1d} is 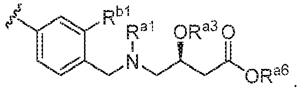 In certain embodiments, R^{1d} is 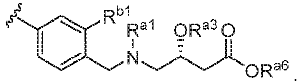 In certain embodiments, R^{1d} is 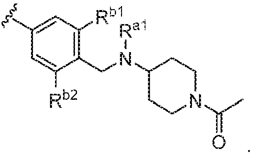 In certain embodiments, R^{1d} is 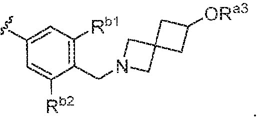 In certain embodiments, R^{1d} is 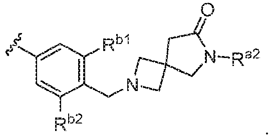 In certain embodiments, R^{1d} is 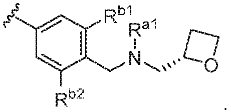 In certain embodiments, R^{1d} is 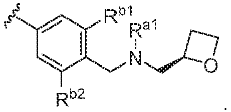 In certain embodiments, R^{1d} is 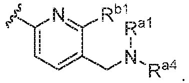 In certain embodiments, R^{1d} is 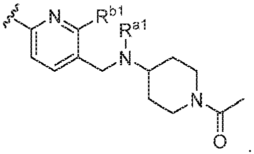 In certain embodiments, R^{1d} is 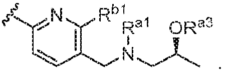 In certain embodiments, R^{1d} is 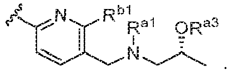 In certain embodiments, R^{1d} is 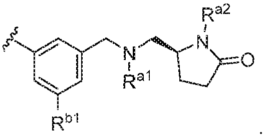 In certain embodiments, R^{1d} is 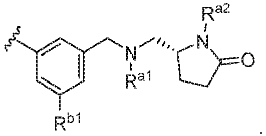 In certain embodiments, R^{1d} is 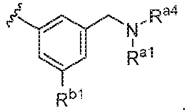 In certain embodiments, R^{1d} is 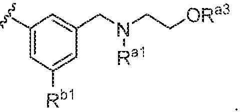 In certain embodiments, R^{1d} is 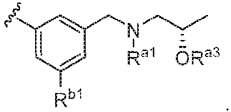 In certain embodiments, R^{1d} is 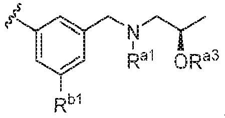 In certain embodiments, R^{1d} is 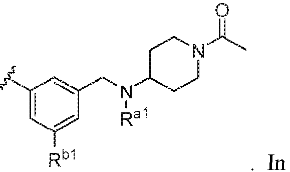 certain embodiments, R^{1d} is 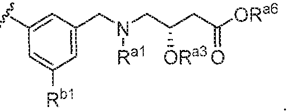 In certain embodiments, R^{1d} is 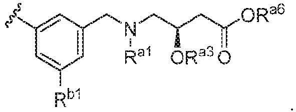 In certain embodiments, R^{1d} is 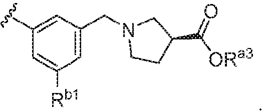 In certain embodiments, R^{1d} is 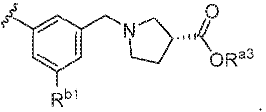 In certain embodiments, R^{1d} is 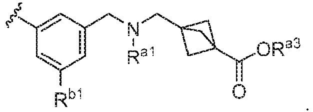 In certain embodiments, R^{1d} is 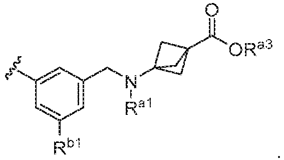 In certain embodiments, R^{1d} is 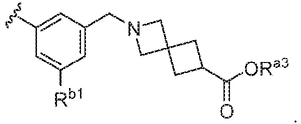 In certain embodiments, R^{1d} is 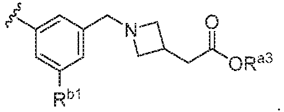 In certain embodiments, R^{1d} is 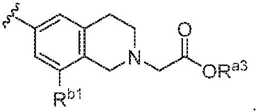 In certain embodiments, R^{1d} is 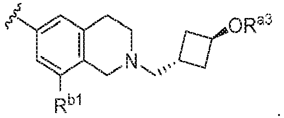 In certain embodiments, R^{1d} is 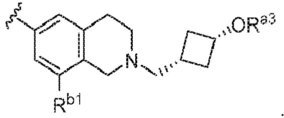 In certain embodiments, R^{1d} is 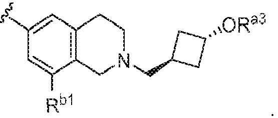 In certain embodiments, R^{1d} is 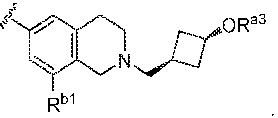 In certain embodiments, R^{1d} is 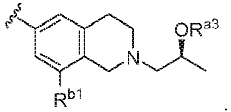 In certain embodiments, R^{1d} is 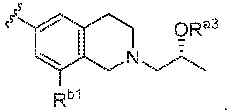 In certain embodiments, R^{1d} is 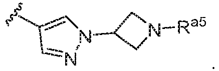 In certain embodiments, R^{1d} is 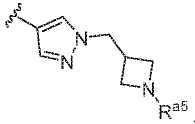 In certain embodiments, R^{1d} is 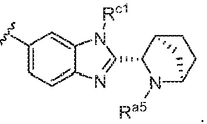 In certain embodiments, R^{1d} is 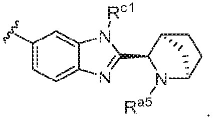 In certain embodiments, R^{1d} is 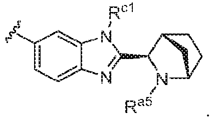 In certain embodiments, R^{1d} is 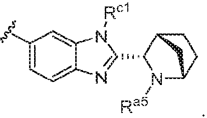 In certain embodiments, R^{1d} is 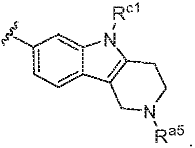 In certain embodiments, R^{1d} is 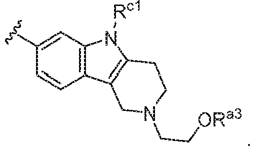 In certain embodiments, R^{1d} is 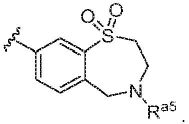 In certain embodiments, R^{1d} is 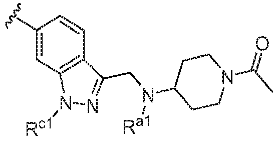 . In certain embodiments, R^{1d} is 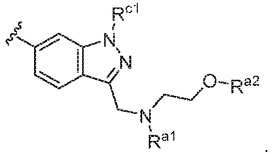 In certain embodiments, R^{1d} is 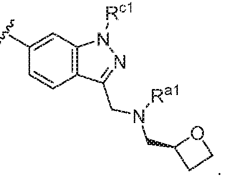 In certain embodiments, R^{1d} is 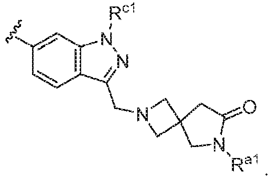 In certain embodiments, R^{1d} is 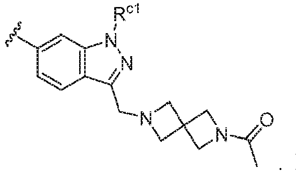 In certain embodiments, R^{1d} is 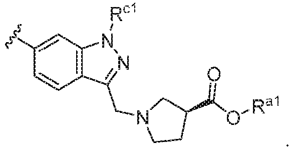 In certain embodiments, R^{1d} is 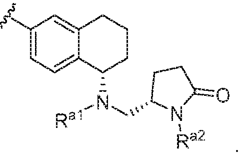 In certain embodiments, R^{1d} is 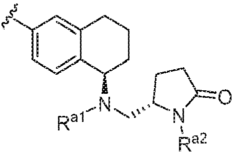 . In certain embodiments, R^{1d} is 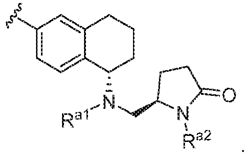 In certain embodiments, R^{1d} is 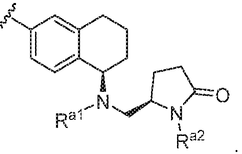 In certain embodiments, R^{1d} is 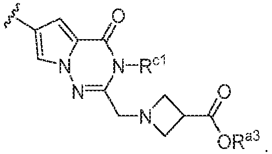 In certain embodiments, R^{1d} is 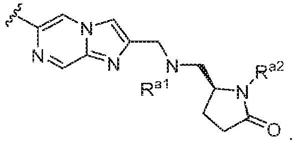 In certain embodiments, R^{1d} is 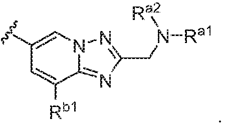 In certain embodiments, R^{1d} is 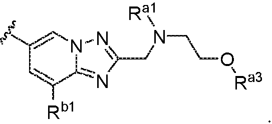 In certain embodiments, R^{1d} is 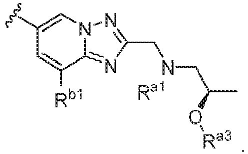 In certain embodiments, R^{1d} is 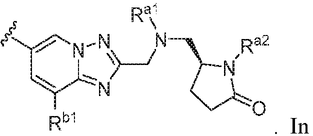 certain embodiments, R^{1d} is 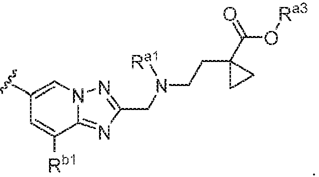 In certain embodiments, R^{1d} is 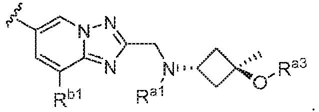 In certain embodiments, R^{1d} is 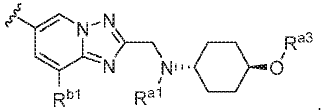 In certain embodiments, R^{1d} is 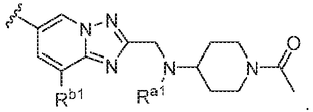 In certain embodiments, R^{1d} is 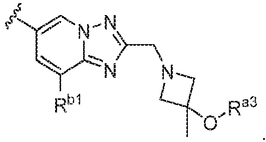 In certain embodiments, R^{1d} is 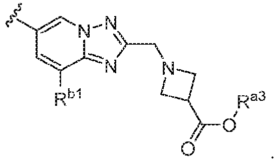 certain embodiments, R^{1d} is 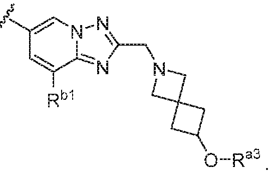 In certain embodiments, R^{1d} is 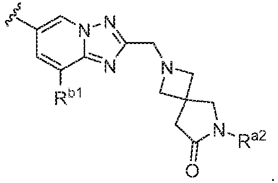 In certain embodiments, R^{1d} is 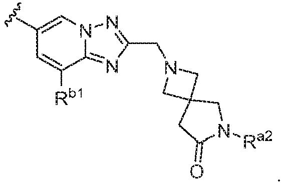 In certain embodiments, R^{1d} is 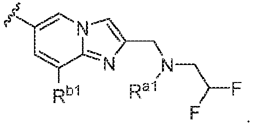 In certain embodiments, R^{1d} is 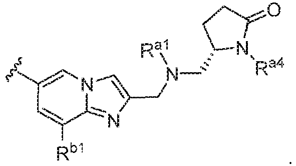 In certain embodiments, R^{1d} is 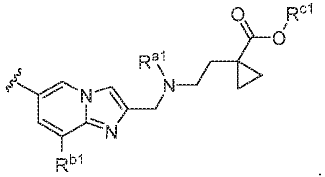 In certain embodiments, R^{1d} is 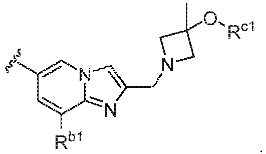 In certain embodiments, R^{1d} is 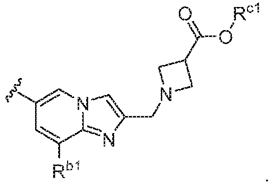 In certain embodiments, R^{1d} is 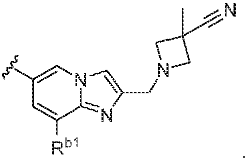 In certain embodiments, R^{1d} is 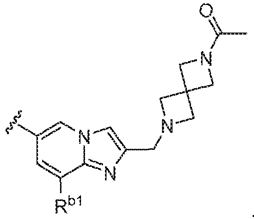 In certain embodiments, R^{1d} is 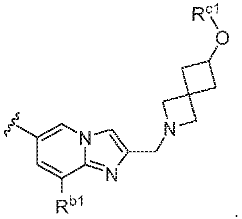 In certain embodiments, R^{1d} is 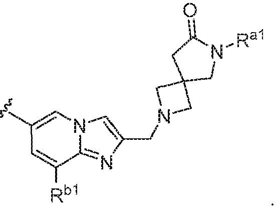 In certain embodiments, R^{1d} is 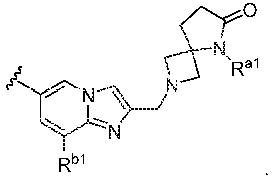 In certain embodiments, R^{1d} is 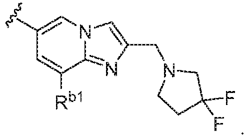 In certain embodiments, R^{1d} is 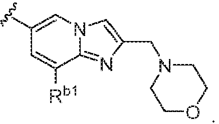 In certain embodiments, R^{1d} is 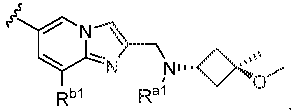 In certain embodiments, R^{1d} is 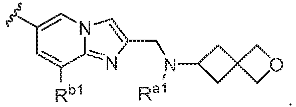 In certain embodiments, R^{1d} is 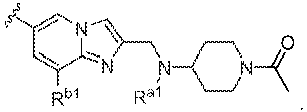 In certain embodiments, R^{1d} is 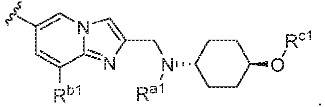 In certain embodiments, R^{1d} is 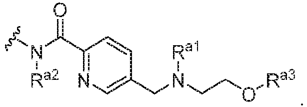 In certain embodiments, R^{1d} is 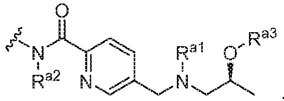 In certain embodiments, R^{1d} is 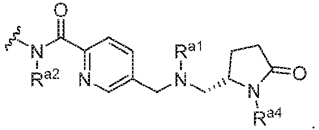 In certain embodiments, R^{1d} is 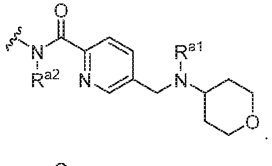 In certain embodiments, R^{1d} is 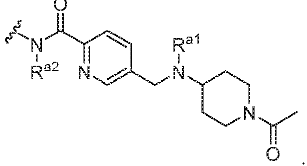 In certain embodiments, R^{1d} is 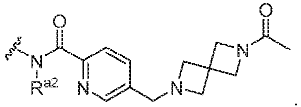 In certain embodiments, R^{1d} is 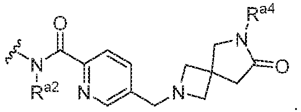 In certain embodiments, R^{1d} is 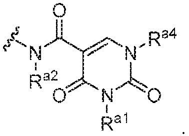 In certain embodiments, R^{1d} is 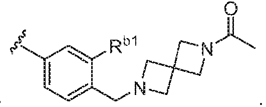 In certain embodiments, R^{1d} is 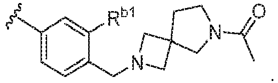 In certain embodiments, R^{1d} is 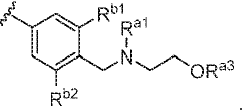 In certain embodiments, R^{1d} is 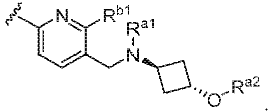 In certain embodiments, R^{1d} is 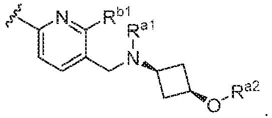 In certain embodiments, R^{1d} is 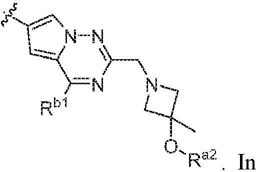 certain embodiments, R^{1d} is 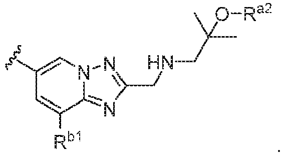 In certain embodiments, R^{1d} is 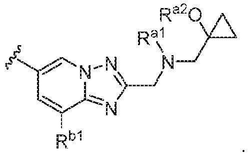 In certain embodiments, R^{1d} is 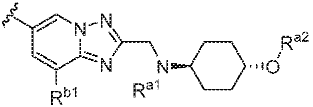 . In certain embodiments, R^{1d} is 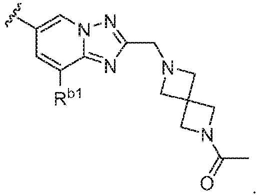 In certain embodiments, R^{1d} is 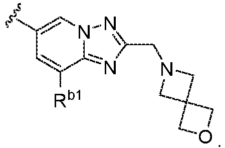 In certain embodiments, R¹⁴ is 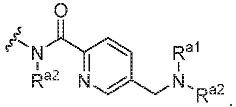 In certain embodiments, R^{1d} is 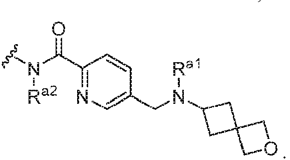 In certain embodiments, R^{1d} is

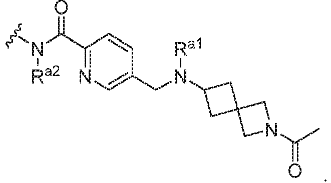

In certain embodiments, R^{a1} is H. In certain embodiments, R^{a1} is C₁-C₆ alkyl. In certain embodiments, R^{a1} is C₃-C₈ cycloalkyl. In certain embodiments, R^{a1} is C₁-C₆ alkoxy. In certain embodiments, R^{a1} is C₁-C₆ haloalkyl. In certain embodiments, R^{a1} is C₁-C₆ haloalkoxy. In certain embodiments, R^{a1} is methyl. In certain embodiments, R^{a1} is ethyl. In certain embodiments, R^{a1} is isopropyl. In certain embodiments, R^{a1} is 2-methylpropyl.

In certain embodiments, R^{a2} is H. In certain embodiments, R^{a2} is C₁-C₆ alkyl. In certain embodiments, R^{a2} is C₃-C₈ cycloalkyl. In certain embodiments, R^{a2} is C₁-C₆ alkoxy. In certain embodiments, R^{a2} is C₁-C₆ haloalkyl. In certain embodiments, R^{a2} is C₁-C₆ haloalkoxy. In certain embodiments, R^{a2} is methyl. In certain embodiments, R³² is ethyl. In certain embodiments, R^{a2} is isopropyl. In certain embodiments, R^{a2} is 2-methylpropyl.

In certain embodiments, R^{a3} is H. In certain embodiments, R^{a3} is C₁-C₆ alkyl. In certain embodiments, R^{a3} is C₃-C₈ cycloalkyl. In certain embodiments, R^{a3} is C₁-C₆ alkoxy. In certain embodiments, R^{a3} is C₁-C₆ haloalkyl. In certain embodiments, R^{a3} is C₁-C₆ haloalkoxy. In certain embodiments, R^{a3} is methyl. In certain embodiments, R^{a3} is ethyl. In certain embodiments, R^{a3} is isopropyl. In certain embodiments, R^{a3} is 2-methylpropyl.

In certain embodiments, R^{a4} is H. In certain embodiments, R^{a4} is C₁-C₆ alkyl. In certain embodiments, R^{a4} is C₃-C₈ cycloalkyl. In certain embodiments, R^{a4} is C₁-C₆ alkoxy. In certain embodiments, R^{a4} is C₁-C₆ haloalkyl. In certain embodiments, R^{a4} is C₁-C₆ haloalkoxy. In certain embodiments, R^{a4} is methyl. In certain embodiments, R^{a4} is ethyl. In certain embodiments, R^{a4} is isopropyl. In certain embodiments, R^{a4} is 2-methylpropyl.

In certain embodiments, R^{a5} is H. In certain embodiments, R^{a5} is C₁-C₆ alkyl. In certain embodiments, R^{a5} is C₃-C₈ cycloalkyl. In certain embodiments, R^{a5} is C₁-C₆ alkoxy. In certain embodiments, R^{a5} is C₁-C₆ haloalkyl. In certain embodiments, R^{a5} is C₁-C₆ haloalkoxy. In certain embodiments, R^{a5} is methyl. In certain embodiments, R^{a5} is ethyl. In certain embodiments, R^{a5} is isopropyl. In certain embodiments, R^{a5} is 2-methylpropyl.

In certain embodiments, R^{a6} is H. In certain embodiments, R^{a6} is C₁-C₆ alkyl. In certain embodiments, R^{a6} is C₃-C₈ cycloalkyl. In certain embodiments, R^{a6} is C₁-C₆ alkoxy. In certain embodiments, R^{a6} is C₁-C₆ haloalkyl. In certain embodiments, R^{a6} is C₁-C₆ haloalkoxy. In certain embodiments, R^{a6} is methyl. In certain embodiments, R³⁶ is ethyl. In certain embodiments, R^{a6} is isopropyl. In certain embodiments, R^{a6} is 2-methylpropyl.

In certain embodiments, R^{a7} is H. In certain embodiments, R^{a7} is C₁-C₆ alkyl. In certain embodiments, R^{a7} is C₃-C₈ cycloalkyl. In certain embodiments, R^{a7} is C₁-C₆ alkoxy. In certain embodiments, R^{a7} is C₁-C₆ haloalkyl. In certain embodiments, R^{a7} is C₁-C₆ haloalkoxy. In certain embodiments, R^{a7} is methyl. In certain embodiments, R^{a7} is ethyl. In certain embodiments, R^{a7} is isopropyl. In certain embodiments, R^{a7} is 2-methylpropyl.

In certain embodiments, R^{b1} is H. In certain embodiments, R^{b1} is C₁-C₆ alkyl. In certain embodiments, R^{b1} is C₃-C₈ cycloalkyl. In certain embodiments, R^{b1} is C₁-C₆ alkoxy. In certain embodiments, R^{b1} is cyano. In certain embodiments, R^{b1} is halogen. In certain embodiments, R^{b1} is C₁-C₆ haloalkyl. In certain embodiments, R^{b1} is C₁-C₆ haloalkoxy. In certain embodiments, R^{b1} is methyl. In certain embodiments, R^{b1} is methoxy. In certain embodiments, R^{b1} is chloro. In certain embodiments, R^{b1} is fluoro. In certain embodiments, R^{b1} is ethoxy. In certain embodiments, R^{b1} is trifluoromethyl. In certain embodiments, R^{b1} is difluoromethoxy.

In certain embodiments, R^{b2} is H. In certain embodiments, R^{b2} is C₁-C₆ alkyl. In certain embodiments, R^{b2} is C₃-C₈ cycloalkyl. In certain embodiments, R^{b2} is C₁-C₆ alkoxy. In certain embodiments, R^{b2} is cyano. In certain embodiments, R^{b2} is halogen. In certain embodiments, R^{b2} is C₁-C₆ haloalkyl. In certain embodiments, R^{b2} is C₁-C₆ haloalkoxy. In certain embodiments, R^{b2} is methyl. In certain embodiments, R^{b2} is methoxy. In certain embodiments, R^{d2} is chloro. In certain embodiments, R^{b2} is fluoro. In certain embodiments, R^{b2} is ethoxy. In certain embodiments, R^{b2} is trifluoromethyl. In certain embodiments, R^{b2} is difluoromethoxy.

In certain embodiments, R^{c1} is H. In certain embodiments, R^{c1} is C₁-C₆ alkyl. In certain embodiments, R^{c1} is C₃-C₈ cycloalkyl. In certain embodiments, R^{c1} is C₁-C₆ alkoxy. In certain embodiments, R^{c1} is C₁-C₆ haloalkyl. In certain embodiments, R^{c1} is C₁-C₆ haloalkoxy. In certain embodiments, R^{c1} is methyl.

In certain embodiments, R^{1c} is 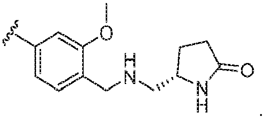 In certain embodiments, R^{1c} is 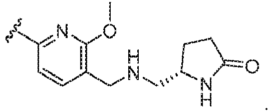 In certain embodiments, R^{1c} is 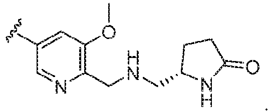 In certain embodiments, R^{1c} is 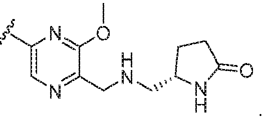 In certain embodiments, R^{1c} is 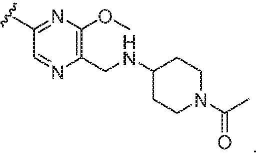 In certain embodiments, R^{1c} is 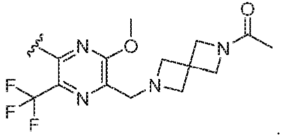 In certain embodiments, R^{1c} is 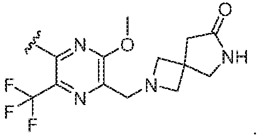 In certain embodiments, R^{1c} is 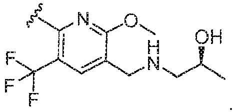 In certain embodiments, R^{1c} is 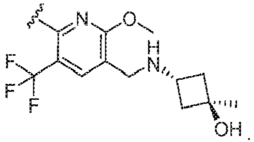 In certain embodiments, R^{1c} is 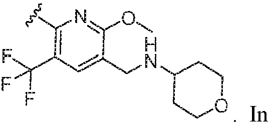 certain embodiments, R^{1c} is 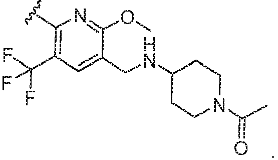 In certain embodiments, R^{1c} is 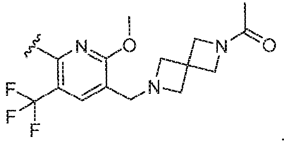 In certain embodiments, R^{1c} is 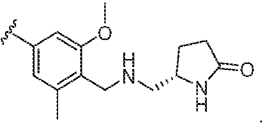 In certain embodiments, R^{1c} is 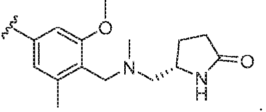 In certain embodiments, R^{1c} is 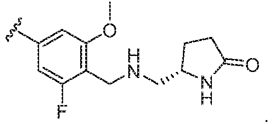 In certain embodiments, R^{1c} is 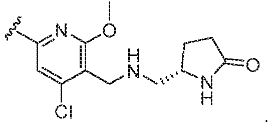 In certain embodiments, R^{1c} is 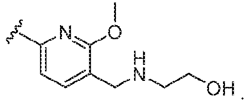 In certain embodiments, R^{1c} is 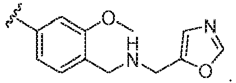 In certain embodiments, R^{1c} is 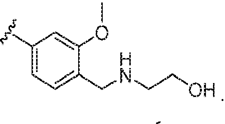 In certain embodiments, R^{1c} is 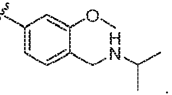 In certain embodiments, R^{1c} is 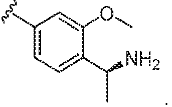 In certain embodiments, R^{1c} is 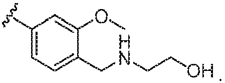 In certain embodiments, R^{1c} is 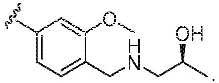 In certain embodiments, R^{1c} is 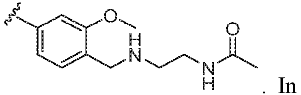 certain embodiments, R^{1c} is 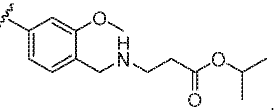 In certain embodiments, R^{1c} is 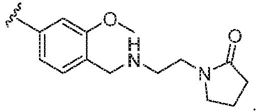 In certain embodiments, R^{1c} is 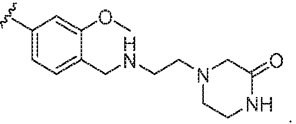 In certain embodiments, R^{1c} is 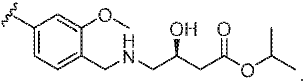 In certain embodiments, R^{1c} is 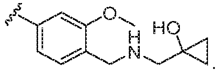 In certain embodiments, R^{1c} is 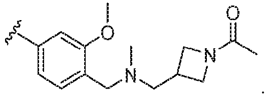 In certain embodiments, R^{1c} is 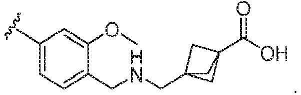 In certain embodiments, R^{1c} is 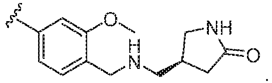 In certain embodiments, R^{1c} is 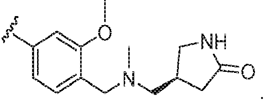 In certain embodiments, R^{1c} is 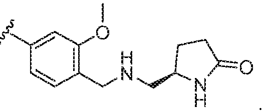 In certain embodiments, R^{1c} is 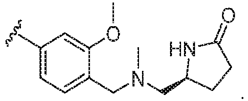 In certain embodiments, R^{1c} is 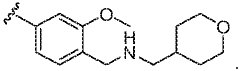 In certain embodiments, R^{1c} is 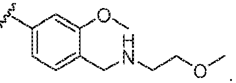 In certain embodiments, R^{1c} is 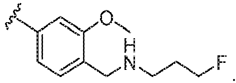 In certain embodiments, R^{1c} is 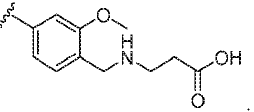 In certain embodiments, R^{1c} is 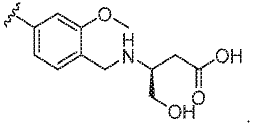 In certain embodiments, R^{1c} is 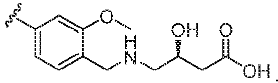 In certain embodiments, R^{1c} is 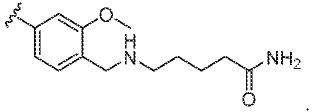 In certain embodiments, R^{1c} is 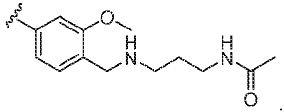 In certain embodiments, R^{1c} is 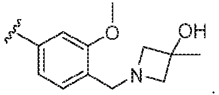 In certain embodiments, R^{1c} is 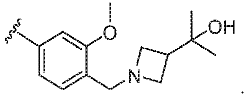 In certain embodiments, R^{1c} is 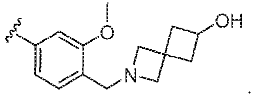 In certain embodiments, R^{1c} is 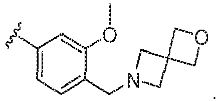 In certain embodiments, R^{1c} is 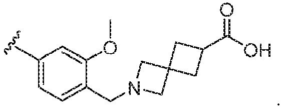 In certain embodiments, R^{1c} is 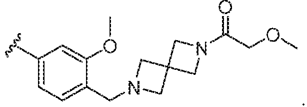 In certain embodiments, R^{1c} is 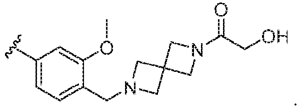 In certain embodiments, R^{1c} is 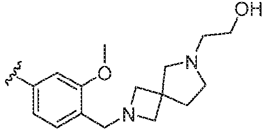 . In certain embodiments, R^{1c} is 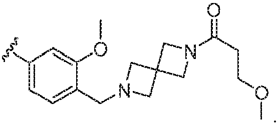 In certain embodiments, R^{1c} is 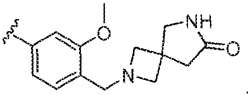 In certain embodiments, R^{1c} is 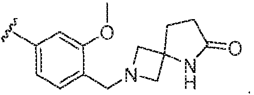 In certain embodiments, R^{1c} is 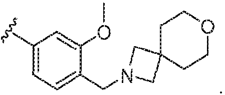 In certain embodiments, R^{1c} is 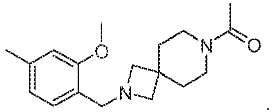 In certain embodiments, R^{1c} is 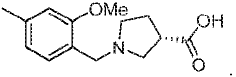 In certain embodiments, R^{1c} is 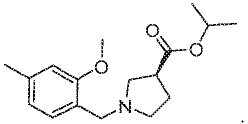 In certain embodiments, R^{1c} is 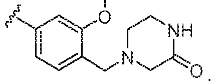 In certain embodiments, R^{1c} is 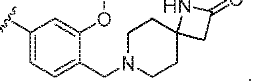 In certain embodiments, R^{1c} is 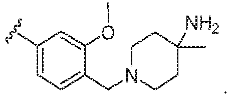 In certain embodiments, R^{1c} is 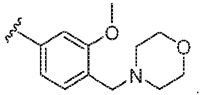 In certain embodiments, R^{1c} is 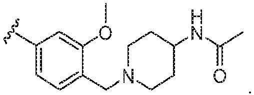 In certain embodiments, R^{1c} is 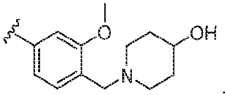 In certain embodiments, R^{1c} is 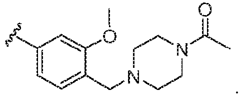 In certain embodiments, R^{1c} is 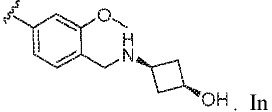 certain embodiments, R^{1c} is 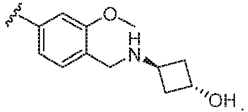 In certain embodiments, R^{1c} is 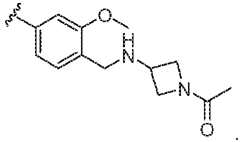 In certain embodiments, R^{1c} is 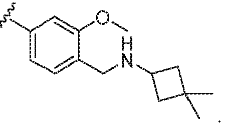 In certain embodiments, R^{1c} is 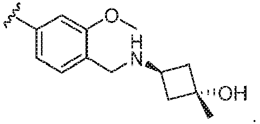 In certain embodiments, R^{1c} is 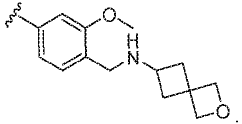 In certain embodiments, R^{1c} is 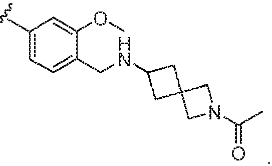 In certain embodiments, R^{1c} is 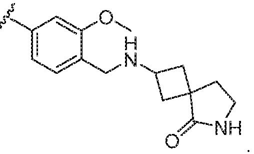 In certain embodiments, R^{1c} is 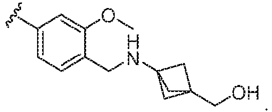 In certain embodiments, R^{1c} is 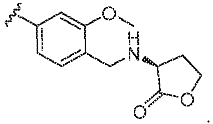 In certain embodiments, R^{1c} is 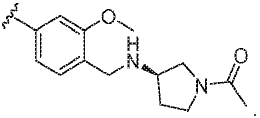 In certain embodiments, R^{1c} is 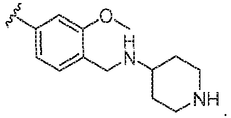 In certain embodiments, R^{1c} is 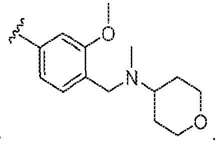 In certain embodiments, R^{1c} is 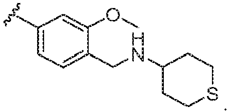 In certain embodiments, R^{1c} is 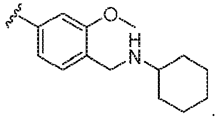 In certain embodiments, R^{1c} is 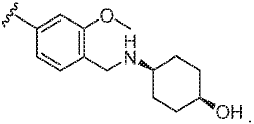 In certain embodiments, R^{1c} is 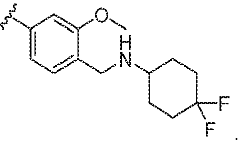 In certain embodiments, R^{1c} is 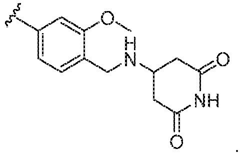 In certain embodiments, R^{1c} is 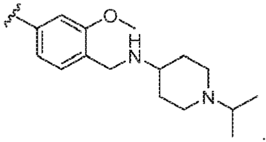 In certain embodiments, R^{1c} is 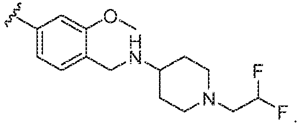 In certain embodiments, R^{1c} is 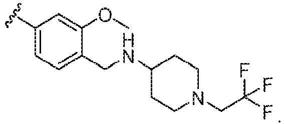 In certain embodiments R^{1c} is 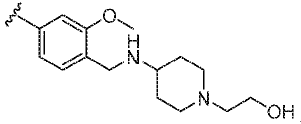 In certain embodiments, R^{1c} is 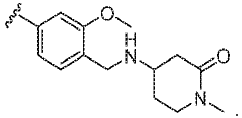 In certain embodiments, R^{1c} is 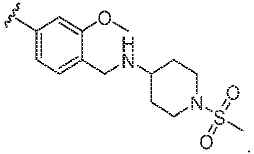 In certain embodiments, R^{1c} is 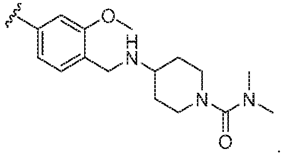 In certain embodiments, R^{1c} is 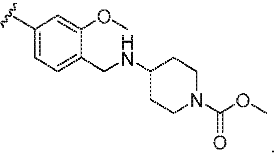 In certain embodiments, R^{1c} is 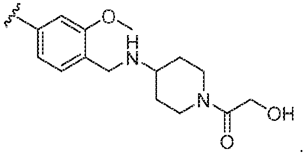 In certain embodiments, R^{1c} is 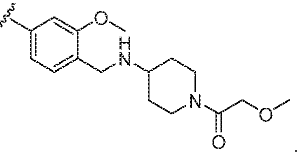 In certain embodiments, R^{1c} is 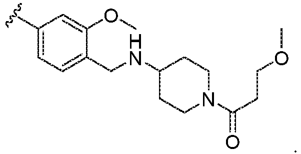 In certain embodiments, R^{1c} is 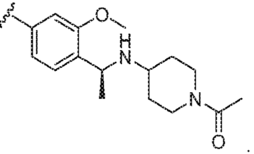 In certain embodiments, R^{1c} is 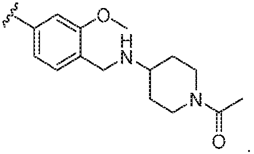 In certain embodiments, R^{1c} is 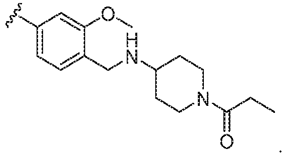 In certain embodiments, R^{1c} is 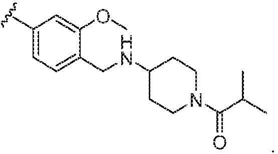 In certain embodiments, R^{1c} is 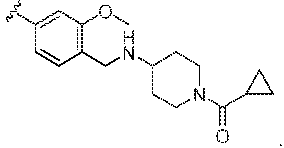 In certain embodiments, R^{1c} is 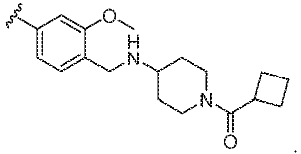 In certain embodiments, R^{1c} is 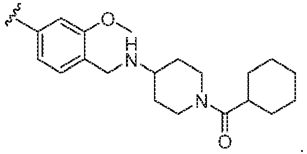 In certain embodiments, R^{1c} is 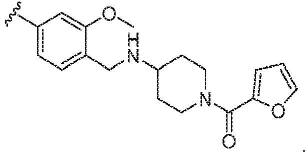 In certain embodiments, R^{1c} is 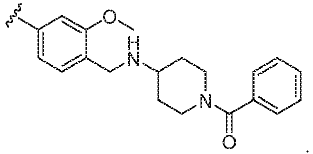 In certain embodiments, R^{1c} is 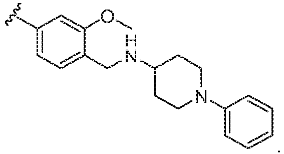 In certain embodiments, R^{1c} is 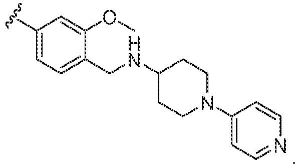 In certain embodiments, R^{1c} is 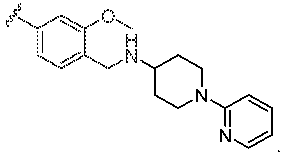 In certain embodiments, R^{1c} is 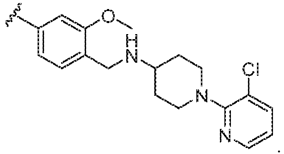 In certain embodiments, R^{1c} is 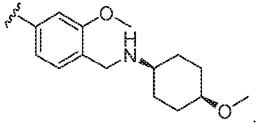 In certain embodiments, R^{1c} is 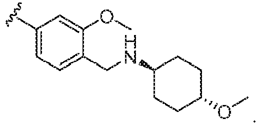 In certain embodiments, R^{1c} is 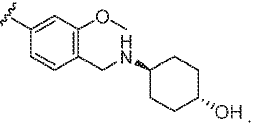 In certain embodiments, R^{1c} is 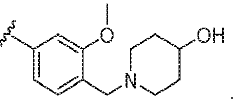 In certain embodiments, R^{1c} is 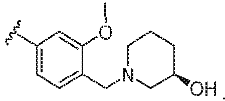 In certain embodiments, R^{1c} is 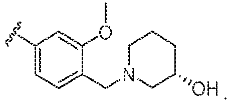 In certain embodiments, R^{1c} is 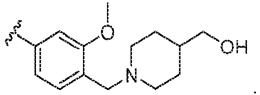 In certain embodiments, R^{1c} is 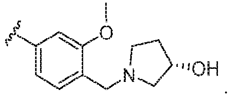 In certain embodiments, R^{1c} is 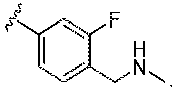 In certain embodiments, R^{1c} is 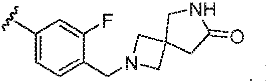 In certain embodiments, R^{1c} is 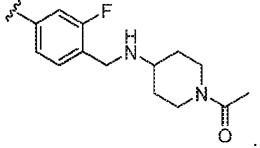 In certain embodiments, R^{1c} is 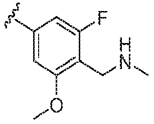 . In certain embodiments, R^{1c} is 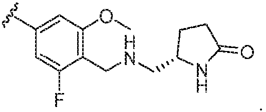 In certain embodiments, R^{1c} is 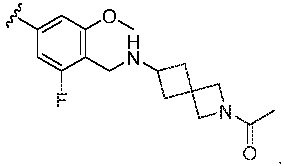 In certain embodiments, R^{1c} is 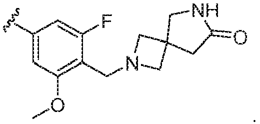 In certain embodiments, R^{1c} is 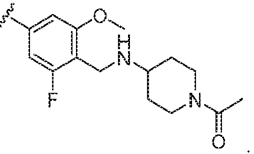 In certain embodiments, R^{1c} is 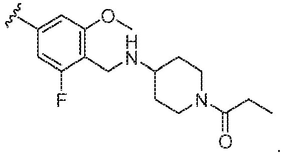 In certain embodiments, R^{1c} is 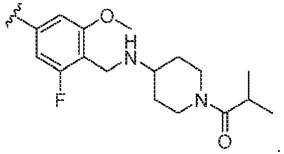 In certain embodiments, R^{1c} is 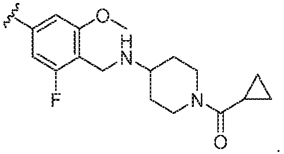 In certain embodiments, R^{1c} is 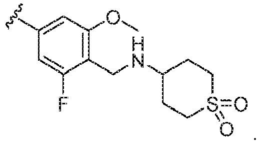 In certain embodiments, R^{1c} is 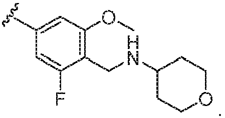 In certain embodiments, R^{1c} is 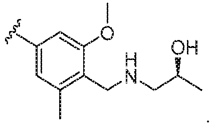 In certain embodiments, R^{1c} is 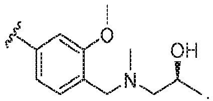 In certain embodiments, R^{1c} is 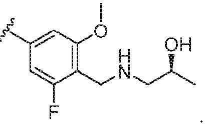 In certain embodiments, R^{1c} is 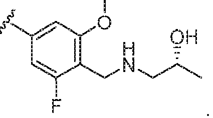 In certain embodiments, R^{1c} is 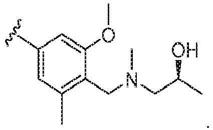 In certain embodiments, R^{1c} is 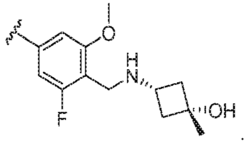 In certain embodiments, R^{1c} is 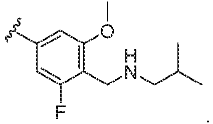 In certain embodiments, R^{1c} is 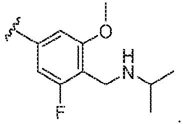 In certain embodiments, R^{1c} is 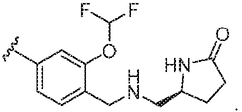 In certain embodiments, R^{1c} is 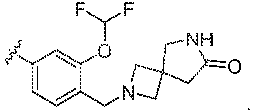 In certain embodiments, R^{1c} is 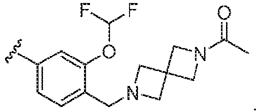 In certain embodiments, R^{1c} is 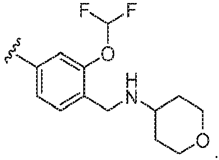 In certain embodiments R^{1c} is 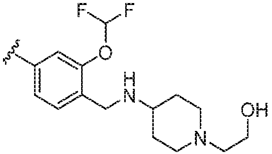 . In certain embodiments R^{1c} is 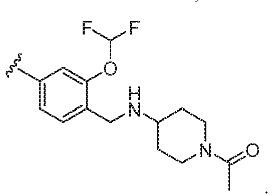 In certain embodiments, R^{1c} is 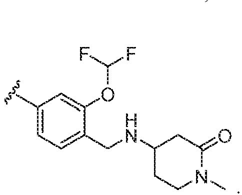 In certain embodiments, R^{1c} is 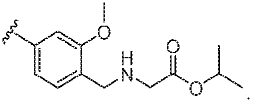 In certain embodiments, R^{1c} is 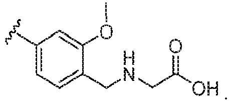 In certain embodiments, R^{1c} is 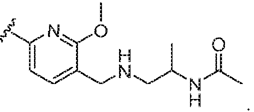 In certain embodiments, R^{1c} is 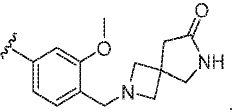 In certain embodiments, R^{1c} is 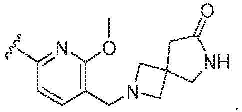 In certain embodiments, R^{1c} is 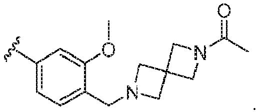 In certain embodiments, R^{1c} is 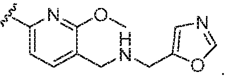 In certain embodiments, R^{1c} is 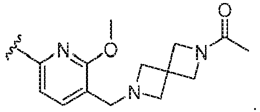 In certain embodiments, R^{1c} is 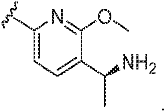 In certain embodiments, R^{1c} is 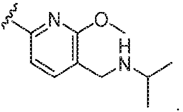 In certain embodiments, R^{1c} is 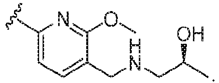 In certain embodiments, R^{1c} is 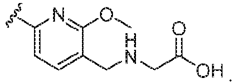 In certain embodiments, R^{1c} is 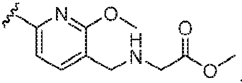 In certain embodiments, R^{1c} is 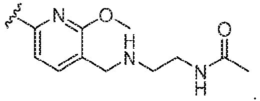 In certain embodiments, R^{1c} is 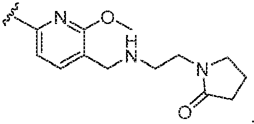 In certain embodiments, R^{1c} is 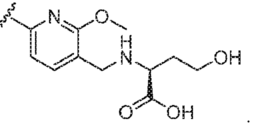 In certain embodiments, R^{1c} is 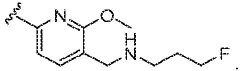 In certain embodiments, R^{1c} is 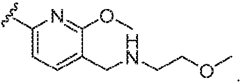 In certain embodiments, R^{1c} is 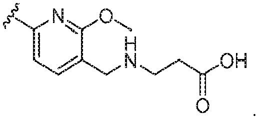 In certain embodiments, R^{1c} is 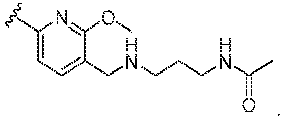 In certain embodiments, R^{1c} is 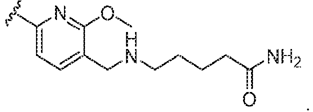 In certain embodiments, R^{1c} is 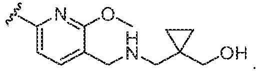 In certain embodiments, R^{1c} is 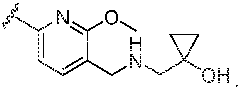 In certain embodiments, R^{1c} is 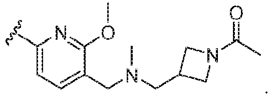 In certain embodiments, R^{1c} is 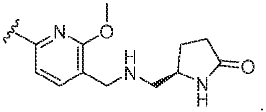 In certain embodiments, R^{1c} is 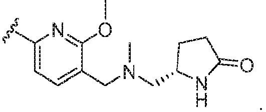 In certain embodiments, R^{1c} is 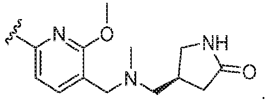 In certain embodiments, R^{1c} is 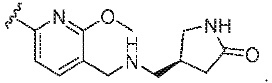 In certain embodiments, R^{1c} is 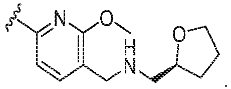 In certain embodiments, R^{1c} is 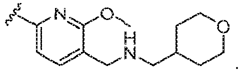 In certain embodiments, R^{1c} is 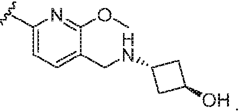 In certain embodiments, R^{1c} is 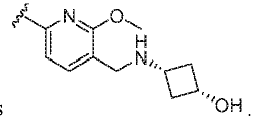 In certain embodiments, R^{1c} is 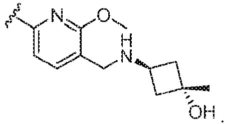 In certain embodiments, R^{1c} is 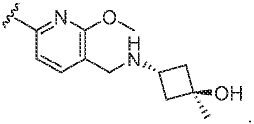 In certain embodiments, R^{1c} is 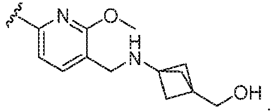 In certain embodiments, R^{1c} is 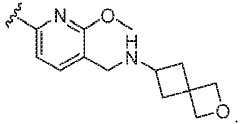 In certain embodiments, R^{1c} is 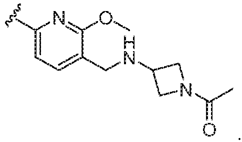 In certain embodiments, R^{1c} is 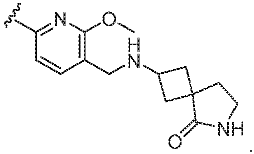 In certain embodiments, R^{1c} is 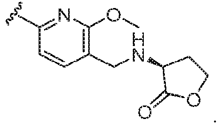 In certain embodiments, R^{1c} is 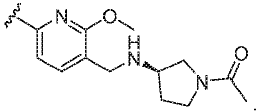 In certain embodiments, R^{1c} is 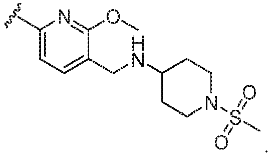 In certain embodiments, R^{1c} is 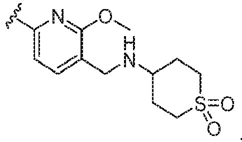 In certain embodiments, R^{1c} is 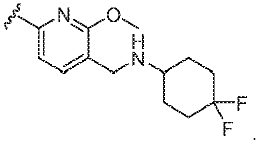 In certain embodiments, R^{1c} is 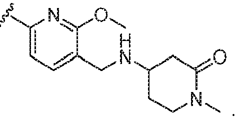 In certain embodiments, R^{1c} is 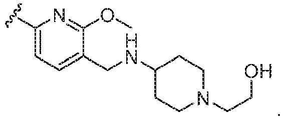 In certain embodiments, R^{1c} is 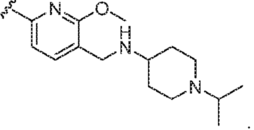 In certain embodiments, R^{1c} is 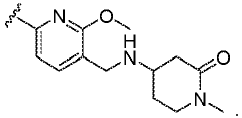 In certain embodiments, R^{1c} is 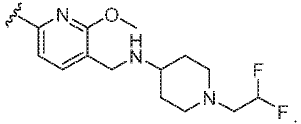 In certain embodiments, R^{1c} is 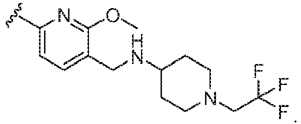 In certain embodiments, R^{1c} is 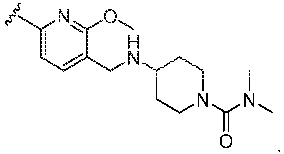 In certain embodiments, R^{1c} is 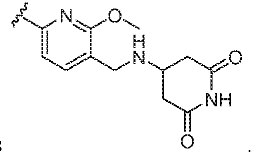 In certain embodiments, R^{1c} is 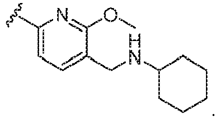 In certain embodiments, R^{1c} is 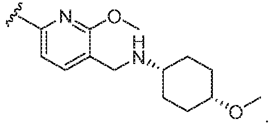 In certain embodiments, R^{1c} is 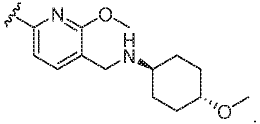 In certain embodiments, R^{1c} is 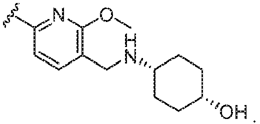 In certain embodiments, R^{1c} is 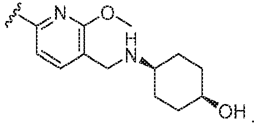 In certain embodiments, R^{1c} is 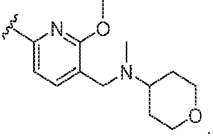 In certain embodiments, R^{1c} is 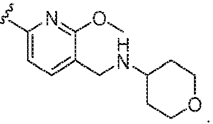 In certain embodiments, R^{1c} is 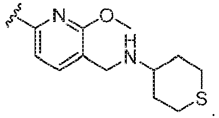 In certain embodiments, R^{1c} is 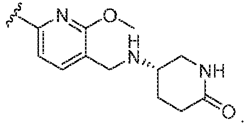 In certain embodiments, R^{1c} is 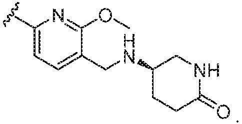 In certain embodiments, R^{1c} is 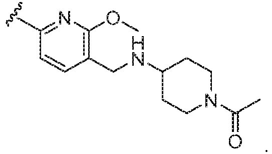 In certain embodiments, R^{1c} is 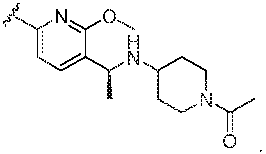 In certain embodiments, R^{1c} is 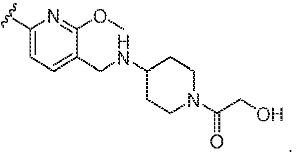 In certain embodiments, R^{1c} is 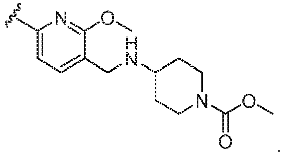 In certain embodiments, R^{1c} is 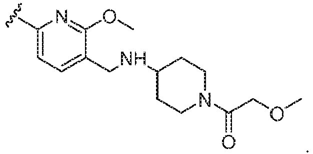 In certain embodiments, R^{1c} is 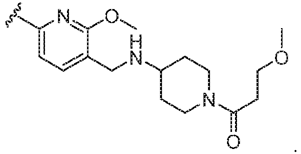 In certain embodiments, R^{1c} is 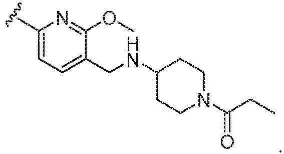 In certain embodiments, R^{1c} is 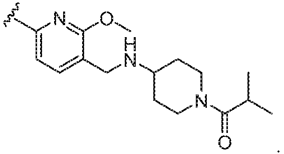 In certain embodiments, R^{1c} is 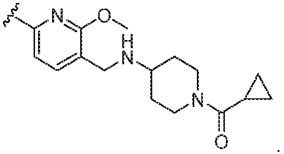 In certain embodiments, R^{1c} is 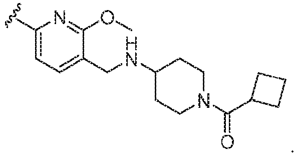 In certain embodiments, R^{1c} is 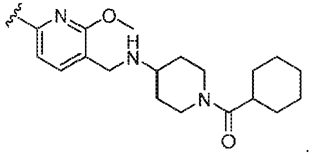 In certain embodiments, R^{1c} is 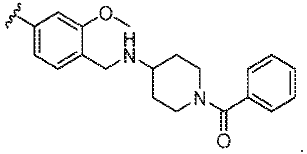 In certain embodiments, R^{1c} is 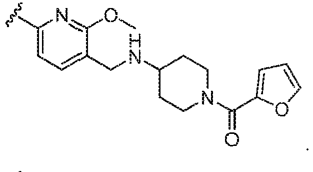 In certain embodiments, R^{1c} is 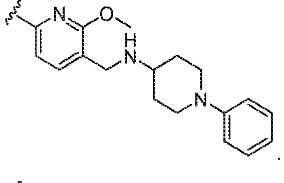 In certain embodiments, R^{1c} is 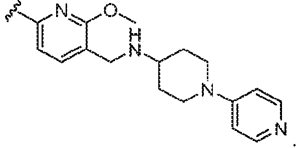 In certain embodiments, R^{1c} is 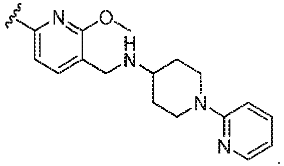 In certain embodiments, R^{1c} is 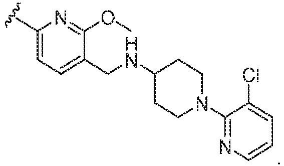 In certain embodiments, R^{1c} is 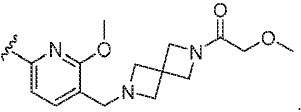 In certain embodiments, R^{1c} is 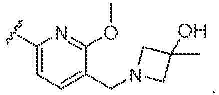 In certain embodiments, R^{1c} is 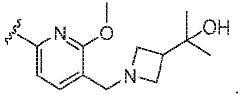 In certain embodiments, R^{1c} is 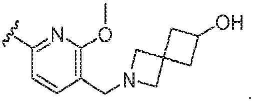 In certain embodiments, R^{1c} is 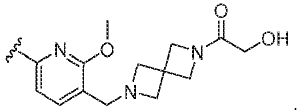 In certain embodiments, R^{1c} is 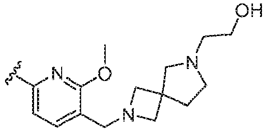 . In certain embodiments, R^{1c} is 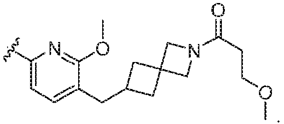 In certain embodiments, R^{1c} is 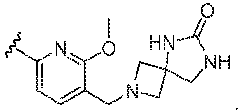 In certain embodiments, R^{1c} is 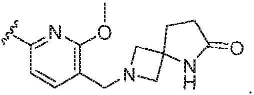 In certain embodiments, R^{1c} is 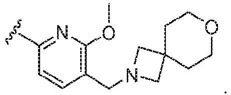 In certain embodiments, R^{1c} is 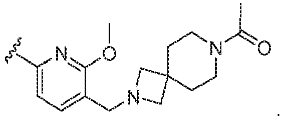 In certain embodiments, R^{1c} is 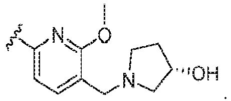 In certain embodiments, R^{1c} is 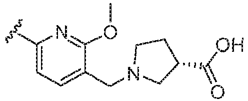 . In certain embodiments, R^{1c} is 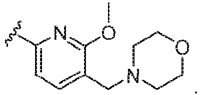 In certain embodiments, R^{1c} is 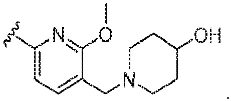 In certain embodiments, R^{1c} is 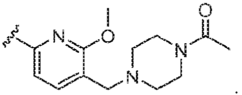 In certain embodiments, R^{1c} is 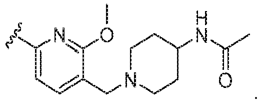 In certain embodiments, R^{1c} is 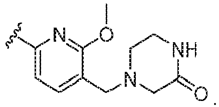 In certain embodiments, R^{1c} is 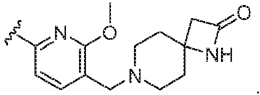 In certain embodiments, R^{1c} is 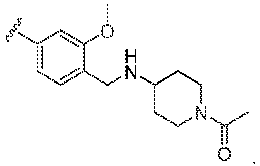 In certain embodiments, R^{1c} is 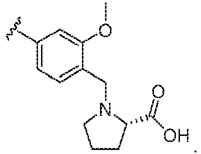 In certain embodiments, R^{1c} is 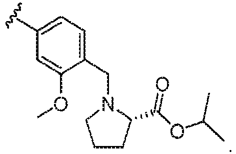 In certain embodiments, R^{1c} is 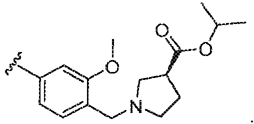 In certain embodiments, R^{1c} is 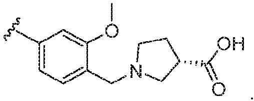 In certain embodiments, R^{1c} is 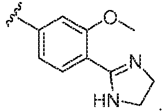 In certain embodiments, R^{1c} is 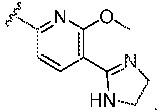 In certain embodiments, R^{1c} is 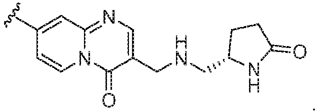 In certain embodiments, R^{1c} is 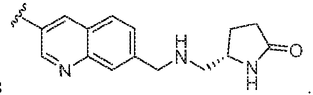 In certain embodiments, R^{1c} is 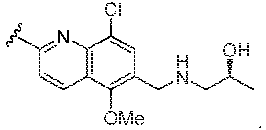 In certain embodiments, R^{1c} is 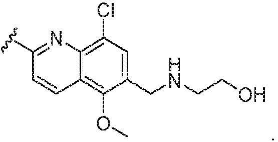 In certain embodiments, R^{1c} is 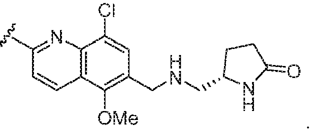 In certain embodiments, R^{1c} is 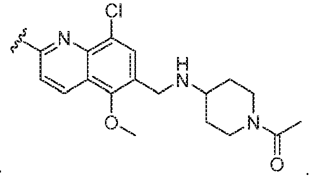 In certain embodiments, R^{1c} is 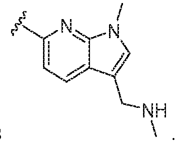 In certain embodiments, R^{1c} is 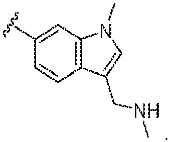 In certain embodiments, R^{1c} is 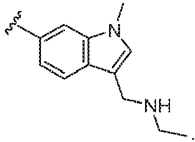 In certain embodiments, R^{1c} is 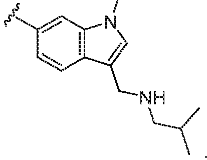 In certain embodiments, R^{1c} is 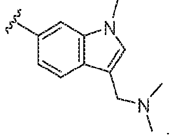 In certain embodiments, R^{1c} is 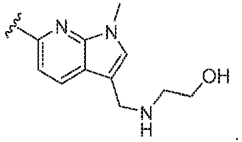 In certain embodiments, R^{1c} is 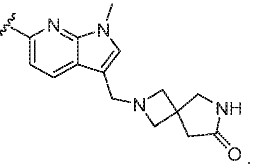 In certain embodiments, R^{1c} is 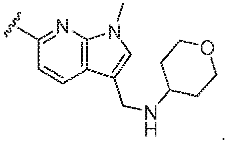 In certain embodiments, R^{1c} is 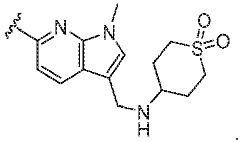 In certain embodiments, R^{1c} is 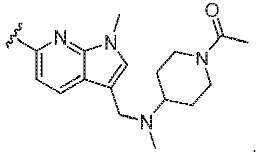 In certain embodiments, R^{1c} is 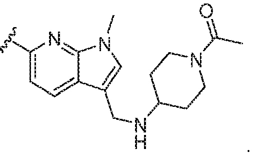 In certain embodiments, R^{1c} is 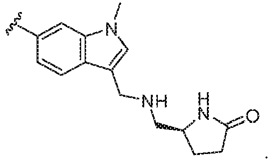 In certain embodiments, R^{1c} is 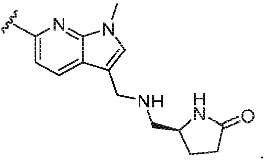 In certain embodiments, R^{1c} is 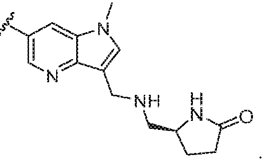 In certain embodiments, R^{1c} is 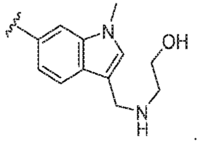 In certain embodiments, R^{1c} is 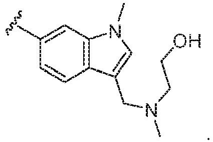 In certain embodiments, R^{1c} is 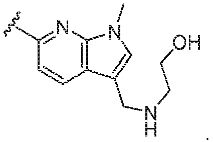 In certain embodiments, R^{1c} is 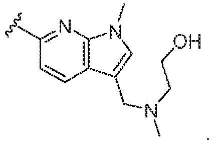 In certain embodiments, R^{1c} is 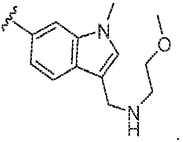 In certain embodiments, R^{1c} is 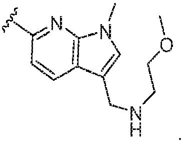 In certain embodiments, R^{1c} is 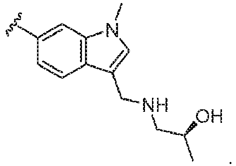 In certain embodiments, R^{1c} is 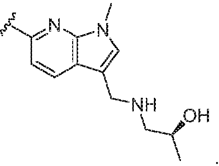 In certain embodiments, R^{1c} is 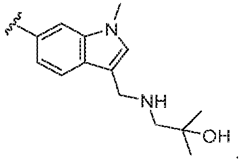 In certain embodiments, R^{1c} is 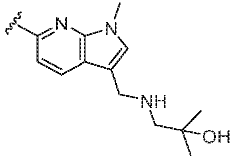 . In certain embodiments, R^{1c} is 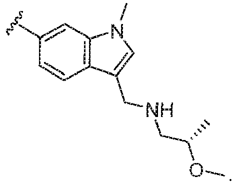 In certain embodiments, R^{1c} is 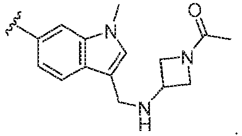 In certain embodiments, R^{1c} is 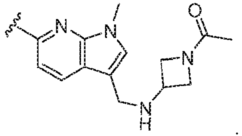 In certain embodiments, R^{1c} is 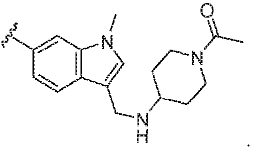 In certain embodiments, R^{1c} is 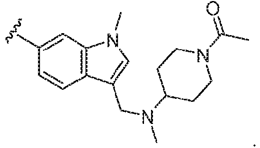 In certain embodiments, R^{1c} is 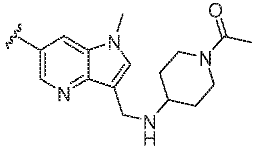 In certain embodiments R^{1c} is 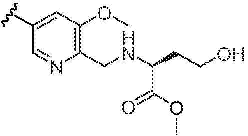 In certain embodiments, R^{1c} is 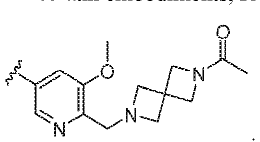 In certain embodiments, R^{1c} is 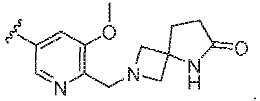 In certain embodiments, R^{1c} is 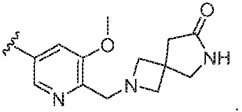 In certain embodiments, R^{1c} is 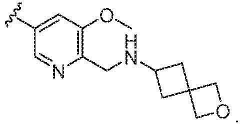 In certain embodiments, R^{1c} is 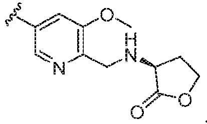 In certain embodiments, R^{1c} is 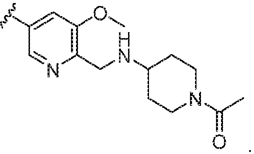 In certain embodiments, R^{1c} is 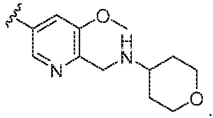 In certain embodiments, R^{1c} is 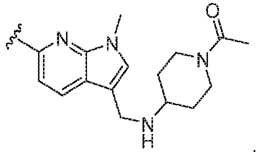 In certain embodiments, R^{1c} is 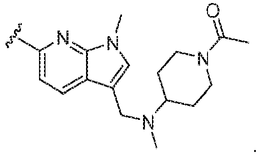 In certain embodiments, R^{1c} is 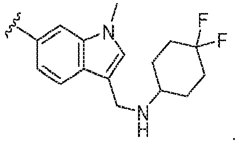 In certain embodiments, R^{1c} is 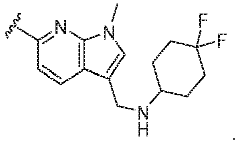 In certain embodiments, R^{1c} is 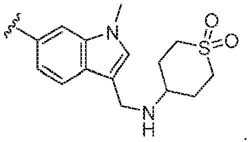 In certain embodiments, R^{1c} is 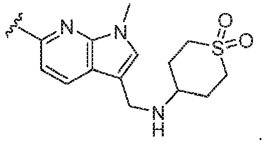 In certain embodiments, R^{1c} is 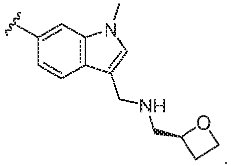 In certain embodiments, R^{1c} is 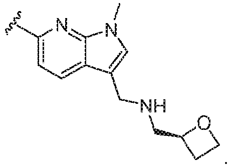 In certain embodiments, R^{1c} is 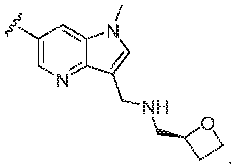 In certain embodiments, R^{1c} is 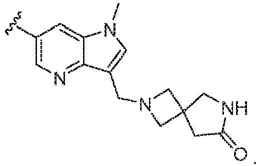 In certain embodiments, R^{1c} is 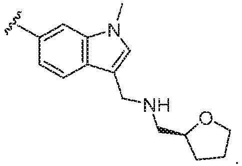 In certain embodiments, R^{1c} is 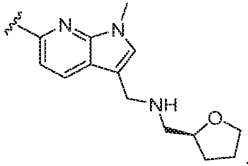 In certain embodiments, R^{1c} is 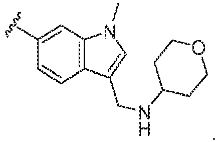 In certain embodiments, R^{1c} is 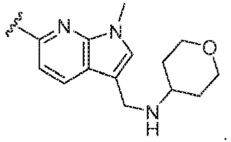 In certain embodiments, R^{1c} is 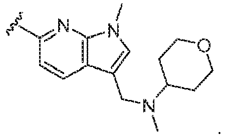 In certain embodiments, R^{1c} is 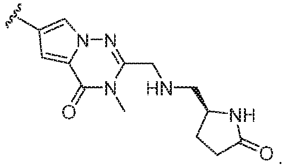 In certain embodiments, R^{1c} is 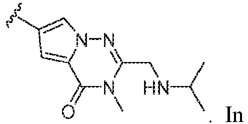 certain embodiments, R^{1c} is 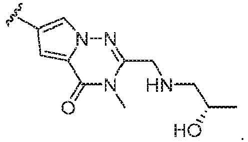 In certain embodiments, R^{1c} is 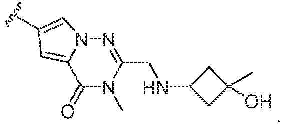 In certain embodiments, R^{1c} is 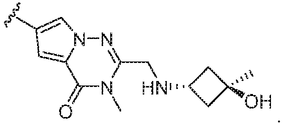 In certain embodiments, R^{1c} is 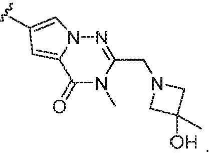 In certain embodiments, R^{1c} is 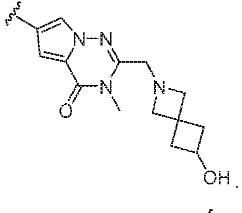 In certain embodiments, R^{1c} is 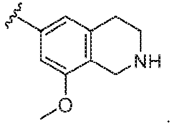 In certain embodiments, R^{1c} is 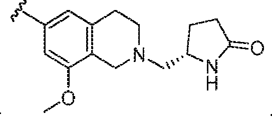 In certain embodiments, R^{1c} is 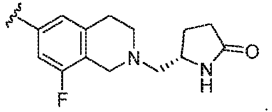 In certain embodiments, R^{1c} is 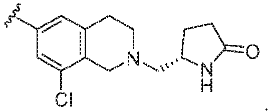 In certain embodiments, R^{1c} is 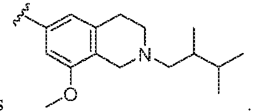 In certain embodiments, R^{1c} is 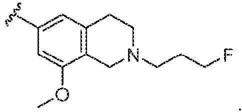 In certain embodiments, R^{1c} is 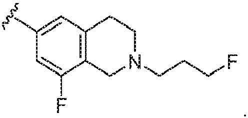 In certain embodiments, R^{1c} is 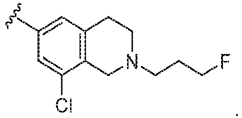 In certain embodiments, R^{1c} is 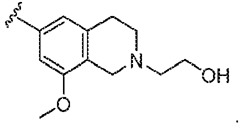 In certain embodiments, R^{1c} is 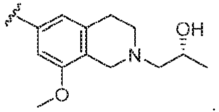 In certain embodiments, R^{1c} is 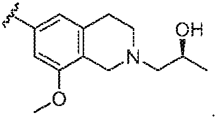 In certain embodiments, R^{1c} is 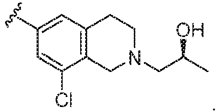 In certain embodiments, R^{1c} is 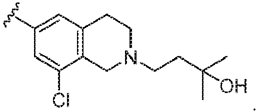 In certain embodiments, R^{1c} is 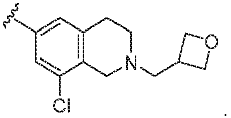 In certain embodiments, R^{1c} is 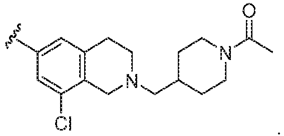 In certain embodiments, R^{1c} is 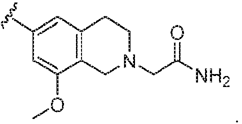 In certain embodiments, R^{1c} is 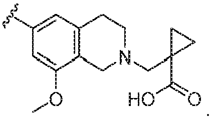 In certain embodiments, R^{1c} is 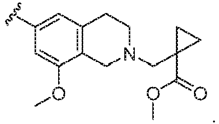 In certain embodiments, R^{1c} is 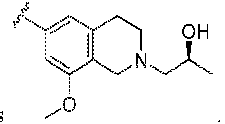 In certain embodiments, R^{1c} is 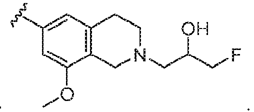 In certain embodiments, R^{1c} is 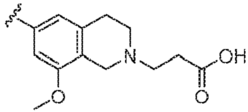 . In certain embodiments, R^{1c} is 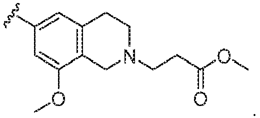 In certain embodiments, R^{1c} is 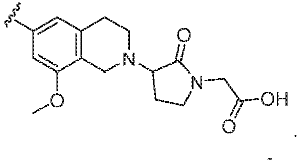 In certain embodiments, R^{1c} is 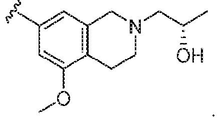 In certain embodiments, R^{1c} is 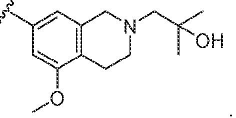 In certain embodiments, R^{1c} is 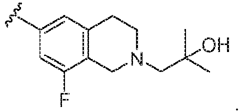 In certain embodiments, R^{1c} is 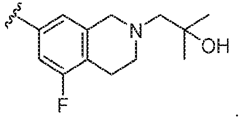 In certain embodiments, R^{1c} is 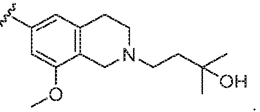 In certain embodiments, R^{1c} is 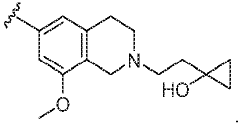 In certain embodiments, R^{1c} is 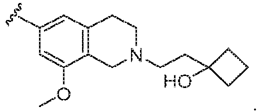 In certain embodiments, R^{1c} is 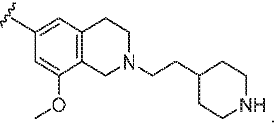 In certain embodiments, R^{1c} is 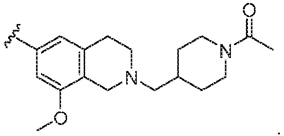 In certain embodiments, R^{1c} is 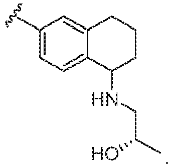 In certain embodiments, R^{1c} is 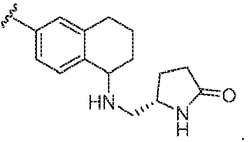 In certain embodiments, R^{1c} is 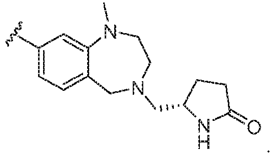 In certain embodiments, R^{1c} is 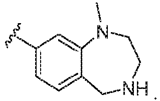 In certain embodiments, R^{1c} is 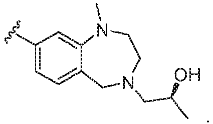 In certain embodiments, R^{1c} is 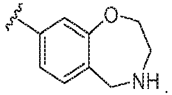 In certain embodiments, R^{1c} is 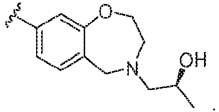 In certain embodiments, R^{1c} is 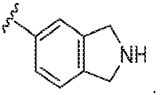 In certain embodiments, R^{1c} is 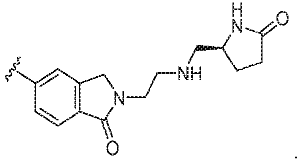 In certain embodiments, R^{1c} is 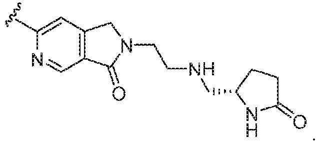 In certain embodiments, R^{1c} is 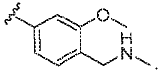 In certain embodiments, R^{1c} is 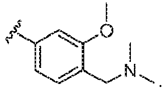 In certain embodiments, R^{1c} is 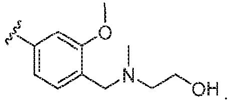 In certain embodiments, R^{1c} is 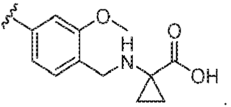 In certain embodiments, R^{1c} is 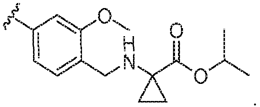 In certain embodiments, R^{1c} is 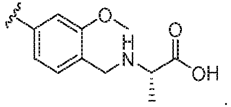 In certain embodiments, R^{1c} is 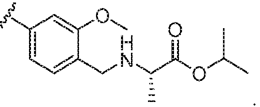 In certain embodiments, R^{1c} is 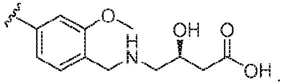 In certain embodiments, R^{1c} is 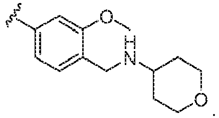 In certain embodiments, R^{1c} is 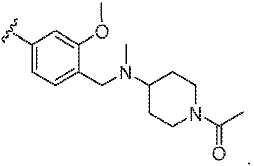 In certain embodiments, R^{1c} is 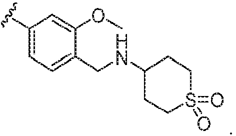 In certain embodiments, R^{1c} is 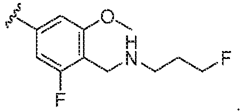 In certain embodiments, R^{1c} is 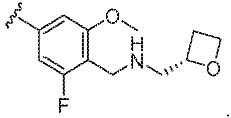 In certain embodiments, R^{1c} is 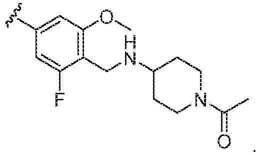 In certain embodiments, R^{1c} is 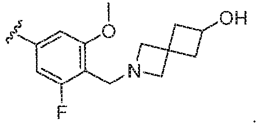 In certain embodiments, R^{1c} is 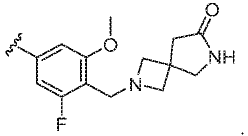 In certain embodiments, R^{1c} is 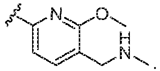 In certain embodiments, R^{1c} is 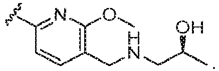 In certain embodiments, R^{1c} is 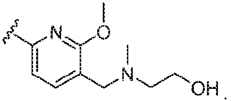 In certain embodiments, R^{1c} is 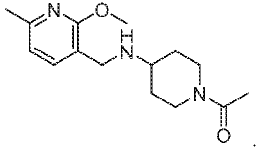 In certain embodiments, R^{1c} is 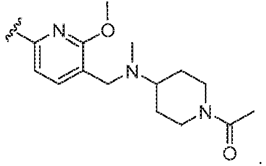 In certain embodiments, R^{1c} is 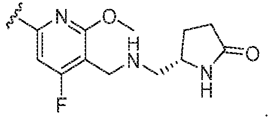 In certain embodiments, R^{1c} is 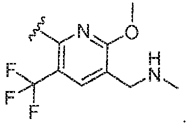 In certain embodiments, R^{1c} is 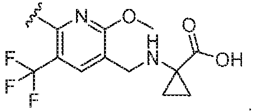 In certain embodiments, R^{1c} is 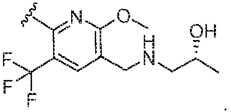 In certain embodiments, R^{1c} is 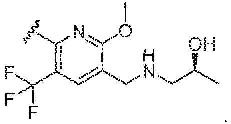 In certain embodiments, R^{1c} is 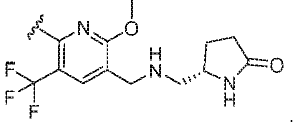 In certain embodiments, R^{1c} is 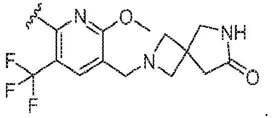 In certain embodiments, R^{1c} is 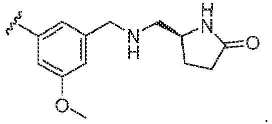 In certain embodiments, R^{1c} is 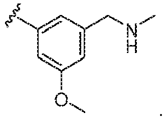 In certain embodiments, R^{1c} is 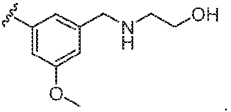 In certain embodiments, R^{1c} is 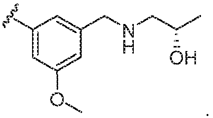 In certain embodiments, R^{1c} is 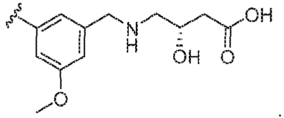 In certain embodiments, R^{1c} is 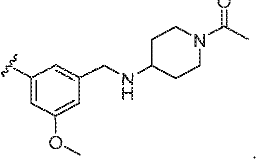 In certain embodiments, R^{1c} is 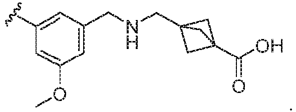 In certain embodiments, R^{1c} is 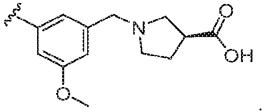 In certain embodiments, R^{1c} is 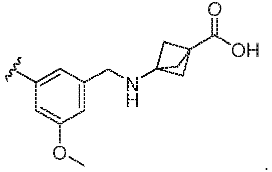 In certain embodiments, R^{1c} is 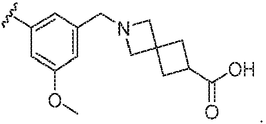 In certain embodiments, R^{1c} is 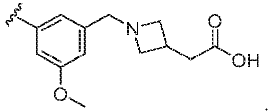 In certain embodiments, R^{1c} is 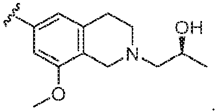 In certain embodiments, R^{1c} is 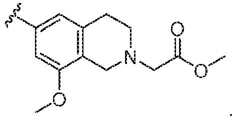 In certain embodiments, R^{1c} is 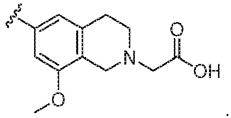 In certain embodiments, R^{1c} is 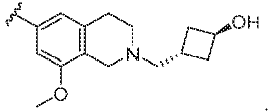 In certain embodiments, R^{1c} is 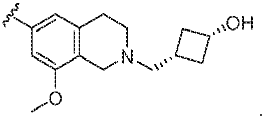 In certain embodiments, R^{1c} is 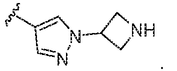 In certain embodiments, R^{1c} is 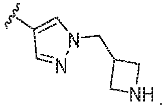 In certain embodiments, R^{1c} is 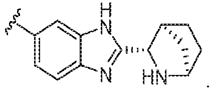 In certain embodiments, R^{1c} is 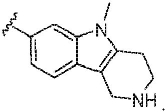 In certain embodiments, R^{1c} is 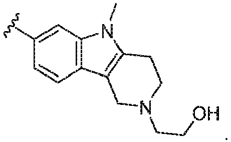 In certain embodiments, R^{1c} is 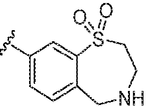 . In certain embodiments, R^{1c} is 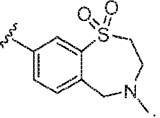 In certain embodiments, R^{1c} is 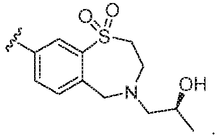 In certain embodiments, R^{1c} is 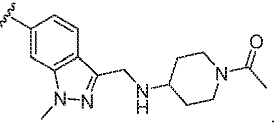 In certain embodiments, R^{1c} is 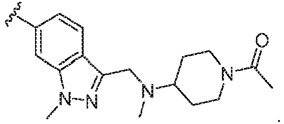 In certain embodiments, R^{1c} is 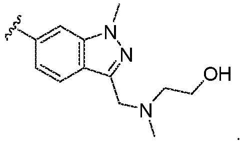 In certain embodiments, R^{1c} is 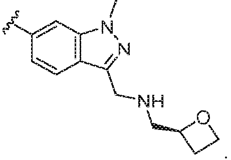 In certain embodiments, R^{1c} is 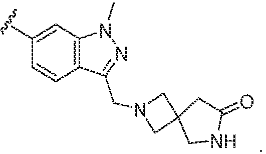 In certain embodiments, R^{1c} is 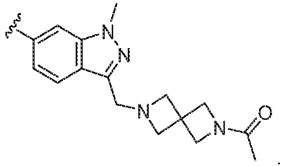 In certain embodiments, R^{1c} is 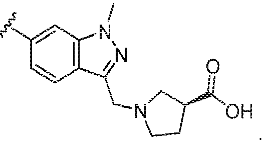 In certain embodiments, R^{1c} is 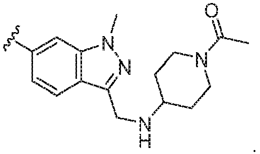 In certain embodiments, R^{1c} is 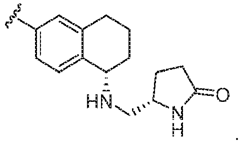 In certain embodiments, R^{1c} is 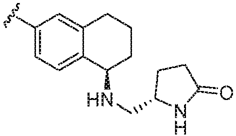 . In certain embodiments, R^{1c} is 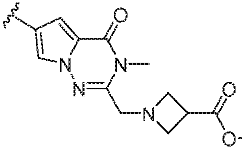 In certain embodiments, R^{1c} is 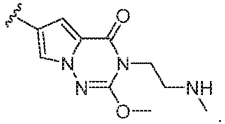 In certain embodiments, R^{1c} is 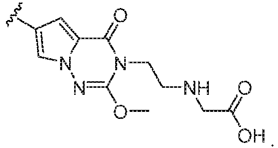 In certain embodiments, R^{1c} is 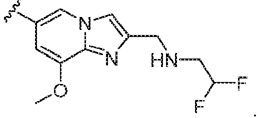 In certain embodiments, R^{1c} is 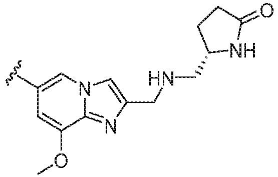 . In certain embodiments, R^{1c} is 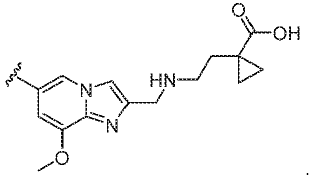 In certain embodiments, R^{1c} is 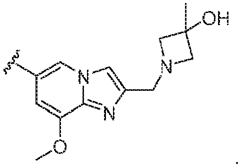 In certain embodiments, R^{1c} is 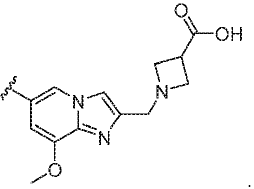 In certain embodiments, R^{1c} is 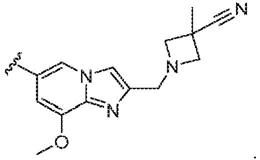 In certain embodiments, R^{1c} is 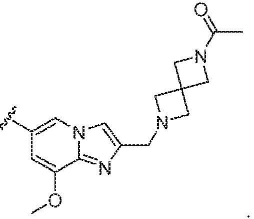 In certain embodiments, R^{1c} is 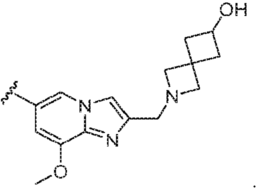 In certain embodiments, R^{1c} is 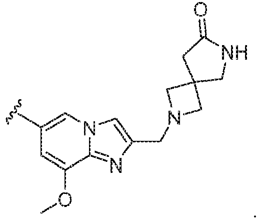 In certain embodiments, R^{1c} is 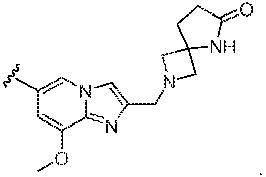 In certain embodiments, R^{1c} is 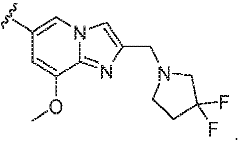 In certain embodiments, R^{1c} is 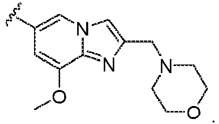 In certain embodiments, R^{1c} is 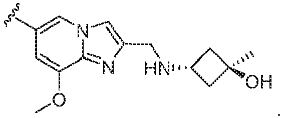 In certain embodiments, R^{1c} is 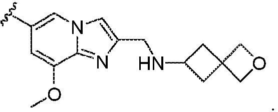 In certain embodiments, R^{1c} is 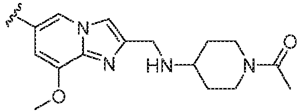 . In certain embodiments, R^{1c} is 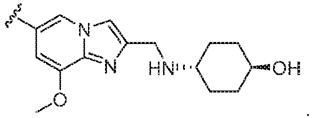 In certain embodiments, R^{1c} is 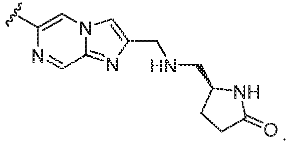 In certain embodiments, R^{1c} is 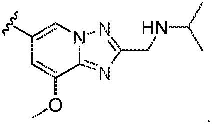 In certain embodiments, R^{1c} is 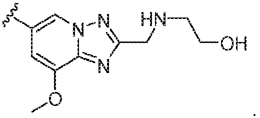 In certain embodiments, R^{1c} is 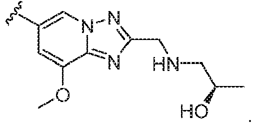 In certain embodiments, R^{1c} is 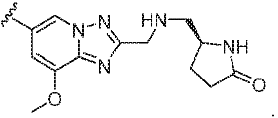 In certain embodiments, R^{1c} is 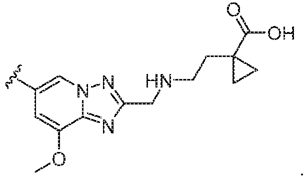 In certain embodiments, R^{1c} is 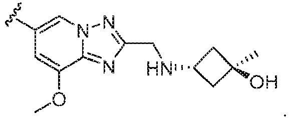 In certain embodiments, R^{1c} is 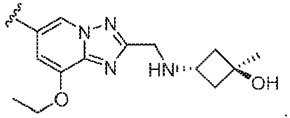 In certain embodiments, R^{1c} is 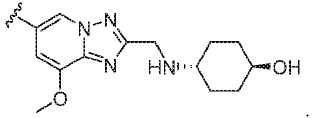 In certain embodiments, R^{1c} is 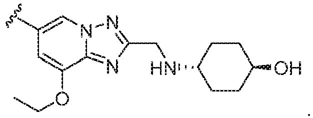 In certain embodiments, R^{1c} is 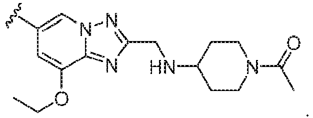 In certain embodiments, R^{1c} is 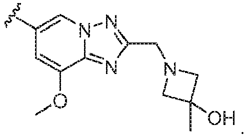 In certain embodiments, R^{1c} is 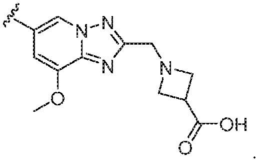 In certain embodiments, R^{1c} is 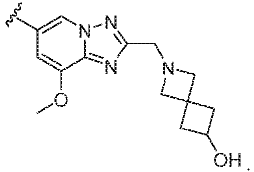 In certain embodiments, R^{1c} is 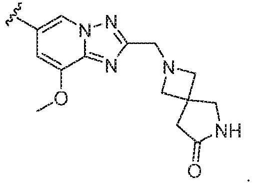 In certain embodiments, R^{1c} is 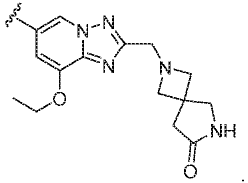 In certain embodiments, R^{1c} is 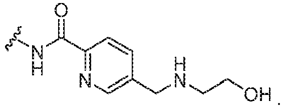 In certain embodiments, R^{1c} is 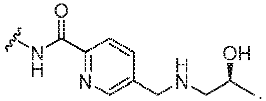 In certain embodiments, R^{1c} is 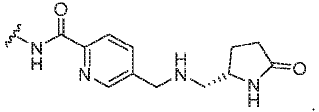 In certain embodiments, R^{1c} is 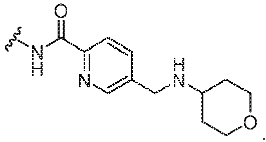 In certain embodiments, R^{1c} is 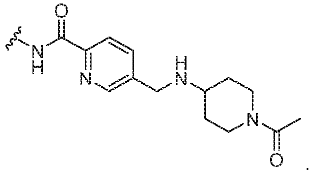 In certain embodiments, R^{1c} is 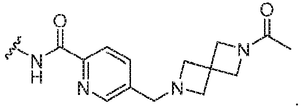 In certain embodiments, R^{1c} is 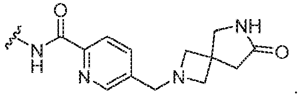 In certain embodiments, R^{1c} is 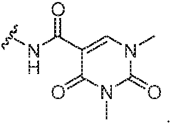 In certain embodiments, R^{1d} is 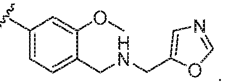 In certain embodiments, R^{1c} is 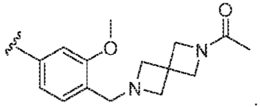 In certain embodiments, R^{1c} is 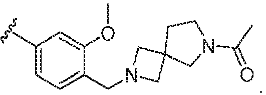 In certain embodiments, R^{1c} is 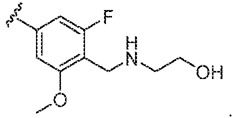 In certain embodiments, R^{1c} is 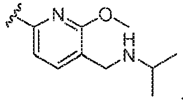 In certain embodiments, R^{1c} is 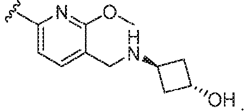 In certain embodiments, R^{1c} is 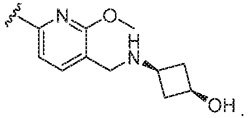 In certain embodiments, R^{1c} is 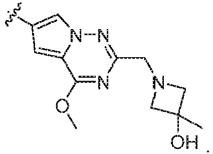 In certain embodiments, R^{1c} is 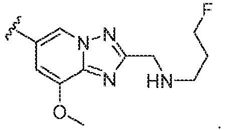 In certain embodiments, R^{1c} is 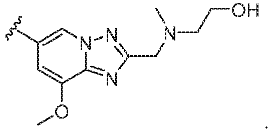 In certain embodiments, R^{1c} is 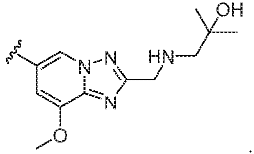 In certain embodiments, R^{1c} is 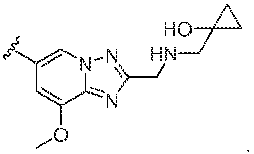 In certain embodiments, R^{1c} is 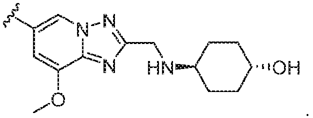 In certain embodiments, R^{1c} is 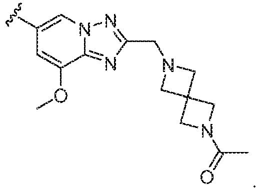 In certain embodiments, R^{1c} is 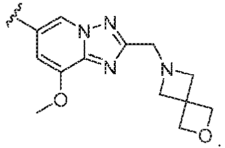 In certain embodiments, R^{1c} is 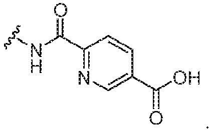 In certain embodiments, R^{1c} is 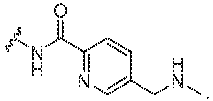 In certain embodiments, R^{1c} is 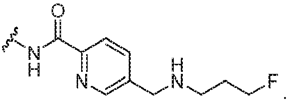 In certain embodiments, R^{1c} is 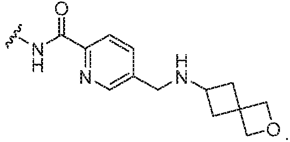 In certain embodiments, R^{1c} is 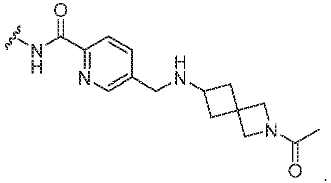 In certain embodiments, R^{1d} is 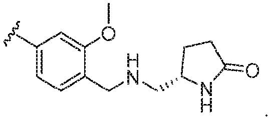 In certain embodiments, R^{1d} is 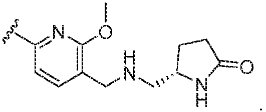 In certain embodiments, R^{1d} is 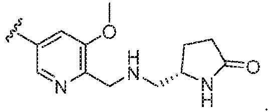 In certain embodiments, R^{1d} is 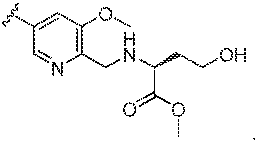 In certain embodiments, R^{1d} is 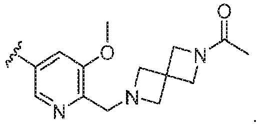 In certain embodiments, R^{1d} is 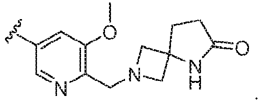 In certain embodiments, R^{1d} is 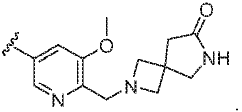 In certain embodiments, R^{1d} is 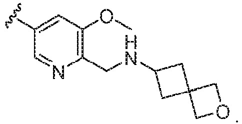 In certain embodiments, R^{1d} is 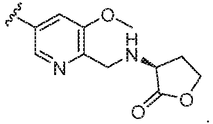 In certain embodiments, R^{1d} is 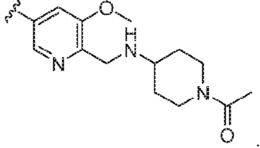 In certain embodiments, R^{1d} is 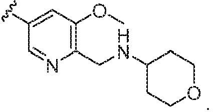 In certain embodiments, R^{1d} is 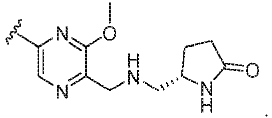 In certain embodiments, R^{1d} is 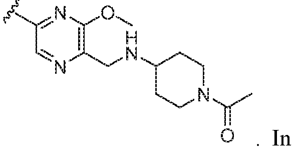 certain embodiments, R^{1d} is 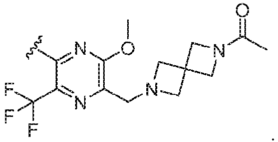 In certain embodiments, R^{1d} is 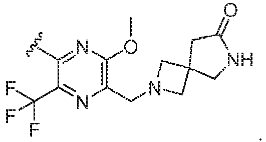 In certain embodiments, R^{1d} is 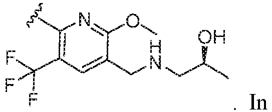 certain embodiments, R^{1d} is 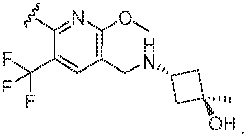 In certain embodiments, R^{1d} is 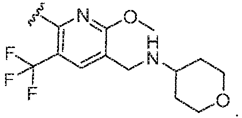 In certain embodiments, R^{1d} is 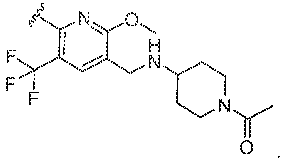 In certain embodiments, R^{1d} is 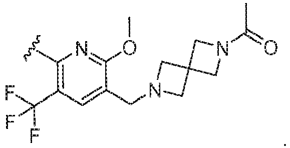 In certain embodiments, R^{1d} is 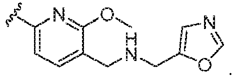 In certain embodiments, R^{1d} is 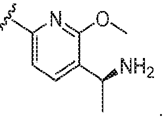 In certain embodiments, R^{1d} is 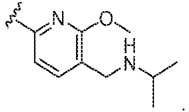 In certain embodiments, R^{1d} is 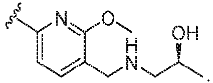 In certain embodiments, R^{1d} is 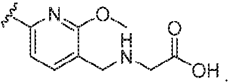 In certain embodiments, R^{1d} is 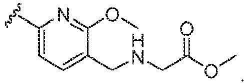 In certain embodiments, R^{1d} is 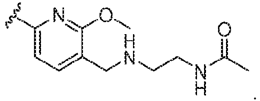 In certain embodiments, R^{1d} is 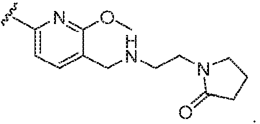 In certain embodiments, R^{1d} is 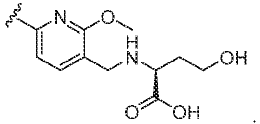 In certain embodiments, R^{1d} is 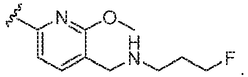 In certain embodiments, R^{1d} is 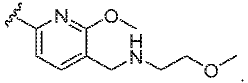 In certain embodiments, R^{1d} is 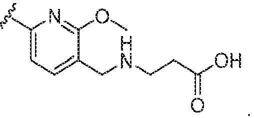 In certain embodiments, R^{1d} is 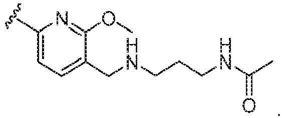 In certain embodiments, R^{1d} is 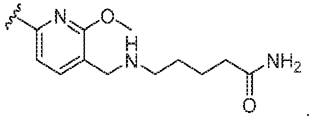 In certain embodiments, R^{1d} is 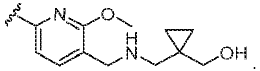 In certain embodiments, R^{1d} is 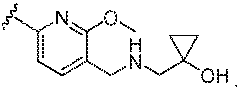 In certain embodiments, R^{1d} is 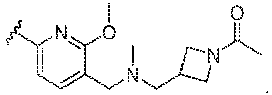 In certain embodiments, R^{1d} is 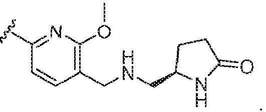 In certain embodiments, R^{1d} is 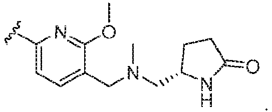 In certain embodiments, R^{1d} is 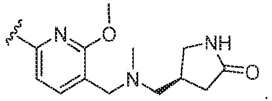 In certain embodiments, R^{1d} is 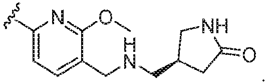 In certain embodiments, R^{1d} is 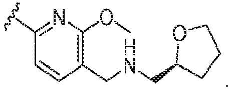 In certain embodiments, R^{1d} is 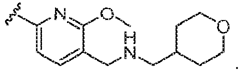 In certain embodiments, R^{1d} is 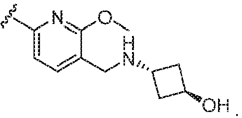 In certain embodiments, R^{1d} is 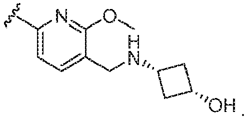 In certain embodiments, R^{1d} is 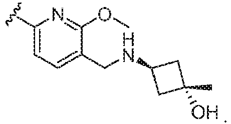 In certain embodiments, R^{1d} is 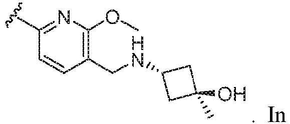 certain embodiments, R^{1d} is 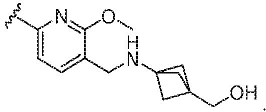 In certain embodiments, R^{1d} is 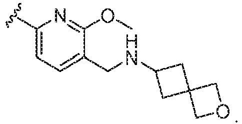 In certain embodiments, R^{1d} is 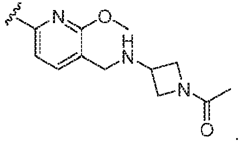 In certain embodiments, R^{1d} is 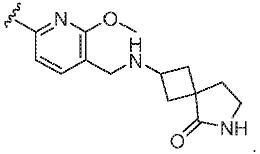 In certain embodiments, R^{1d} is 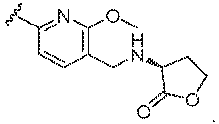 In certain embodiments, R^{1d} is 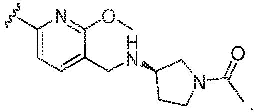 In certain embodiments, R^{1d} is 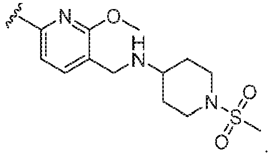 In certain embodiments, R^{1d} is 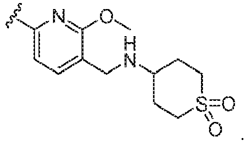 In certain embodiments, R^{1d} is 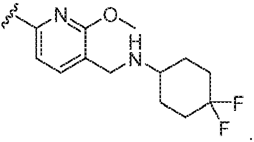 In certain embodiments, R^{1d} is 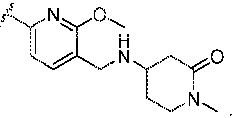 In certain embodiments, R^{1d} is 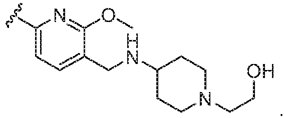 In certain embodiments, R^{1d} is 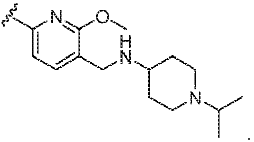 In certain embodiments, R^{1d} is 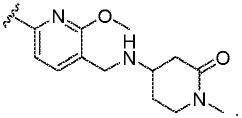 In certain embodiments, R^{1d} is 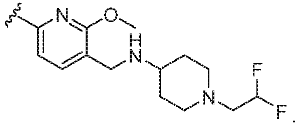 In certain embodiments, R^{1d} is 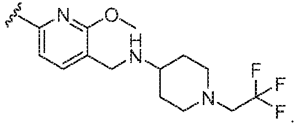 In certain embodiments, R^{1d} is 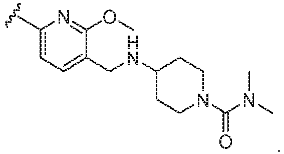 In certain embodiments, R^{1d} is 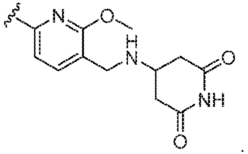 In certain embodiments, R^{1d} is 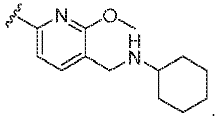 In certain embodiments, R^{1d} is 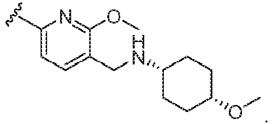 In certain embodiments, R^{1d} is 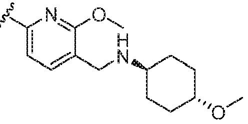 In certain embodiments, R^{1d} is 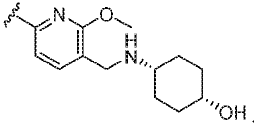 In certain embodiments, R^{1d} is 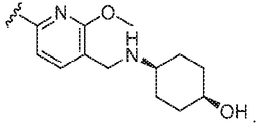 In certain embodiments, R^{1d} is 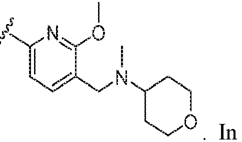 certain embodiments, R^{1d} is 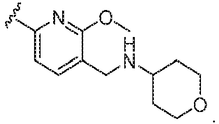 In certain embodiments, R^{1d} is 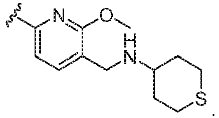 In certain embodiments, R^{1d} is 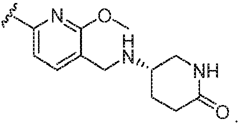 In certain embodiments, R^{1d} is 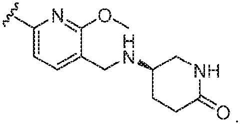 In certain embodiments, R^{1d} is 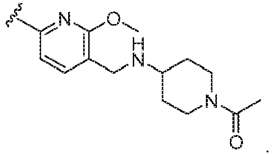 In certain embodiments, R^{1d} is 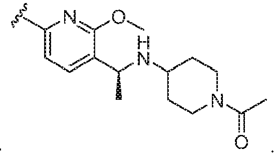 In certain embodiments, R^{1d} is 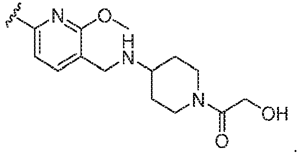 In certain embodiments, R^{1d} is 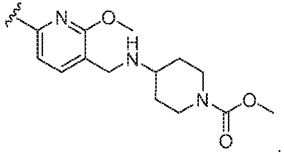 In certain embodiments, R^{1d} is 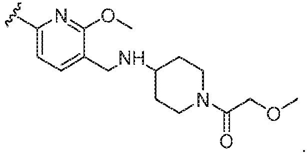 In certain embodiments, R^{1d} is 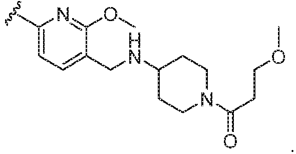 In certain embodiments, R^{1d} is 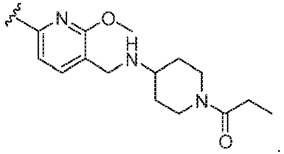 In certain embodiments, R^{1d} is 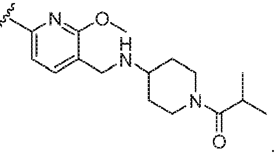 In certain embodiments, R^{1d} is 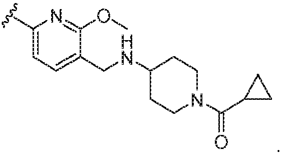 In certain embodiments, R^{1d} is 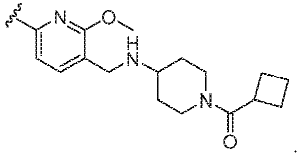 In certain embodiments, R^{1d} is 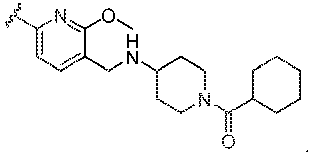 In certain embodiments, R^{1d} is 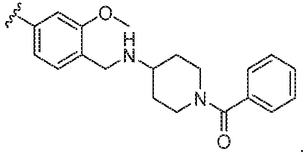 In certain embodiments, R^{1d} is 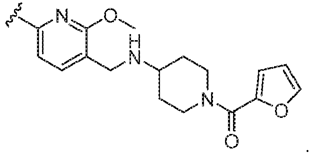 In certain embodiments, R^{1d} is 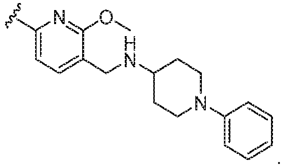 In certain embodiments, R^{1d} is 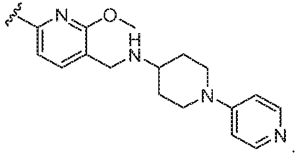 In certain embodiments, R^{1d} is 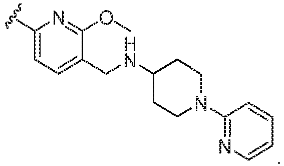 In certain embodiments, R^{1d} is 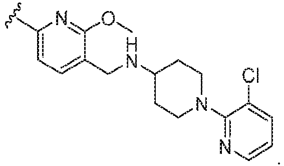 In certain embodiments, R^{1d} is 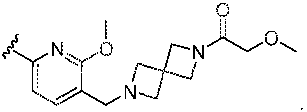 In certain embodiments, R^{1d} is 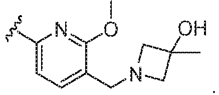 In certain embodiments, R^{1d} is 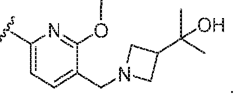 In certain embodiments, R^{1d} is 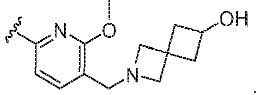 In certain embodiments, R^{1d} is 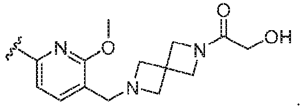 In certain embodiments, R^{1d} is 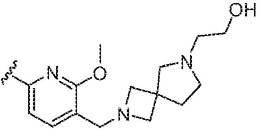 . In certain embodiments, R^{1d} is 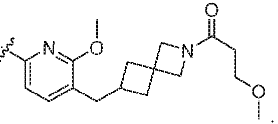 In certain embodiments, R^{1d} is 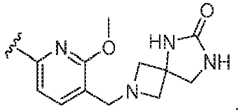 In certain embodiments, R^{1d} is 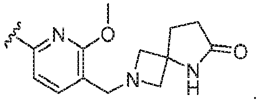 In certain embodiments, R^{1d} is 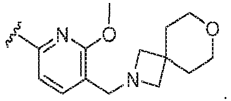 In certain embodiments, R^{1d} is 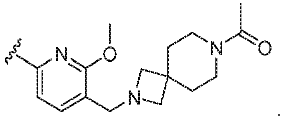 In certain embodiments, R^{1d} is 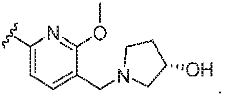 In certain embodiments, R^{1d} is 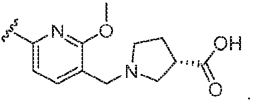 In certain embodiments, R^{1d} is 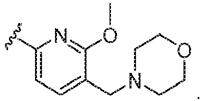 In certain embodiments, R^{1d} is 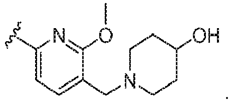 In certain embodiments, R^{1d} is 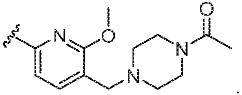 In certain embodiments, R^{1d} is 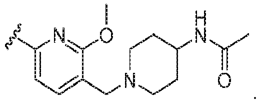 In certain embodiments, R^{1d} is 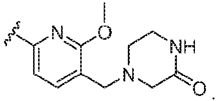 In certain embodiments, R^{1d} is 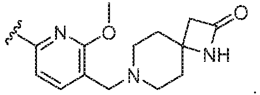 In certain embodiments, R^{1d} is 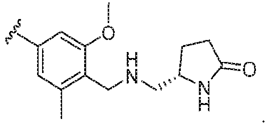 In certain embodiments, R^{1d} is 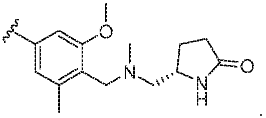 In certain embodiments, R^{1d} is 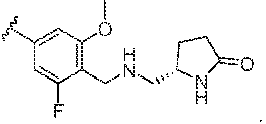 In certain embodiments, R^{1d} is 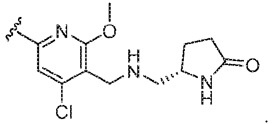 In certain embodiments, R^{1d} is 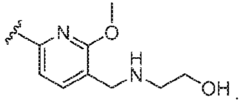 In certain embodiments, R^{1d} is 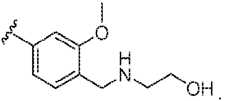 In certain embodiments, R^{1d} is 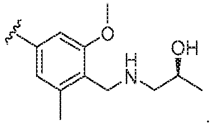 In certain embodiments, R^{1d} is 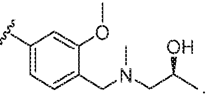 In certain embodiments, R^{1d} is 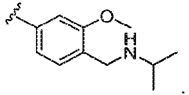 In certain embodiments, R^{1d} is 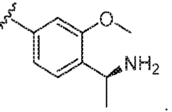 In certain embodiments, R^{1d} is 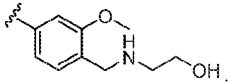 In certain embodiments, R^{1d} is 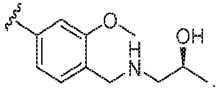 In certain embodiments, R^{1d} is 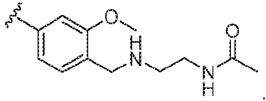 In certain embodiments, R^{1d} is 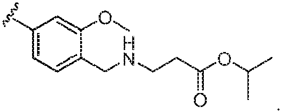 In certain embodiments, R^{1d} is 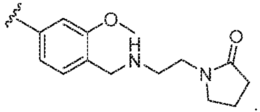 In certain embodiments, R^{1d} is 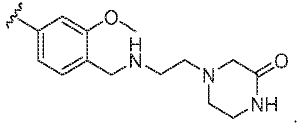 In certain embodiments, R^{1d} is 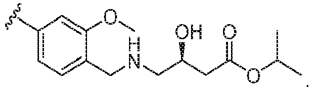 In certain embodiments, R^{1d} is 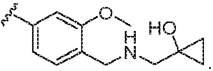 In certain embodiments, R^{1d} is 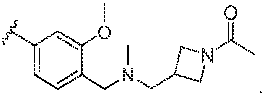 In certain embodiments, R^{1d} is 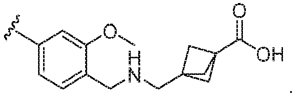 In certain embodiments, R^{1d} is 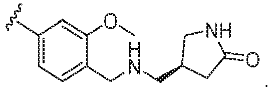 In certain embodiments, R^{1d} is 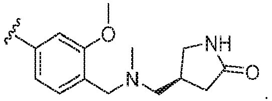 In certain embodiments, R^{1d} is 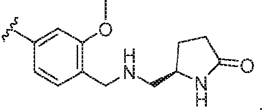 In certain embodiments, R^{1d} is 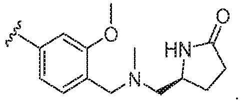 In certain embodiments, R^{1d} is 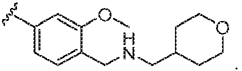 In certain embodiments, R^{1d} is 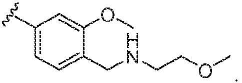 In certain embodiments, R^{1d} is 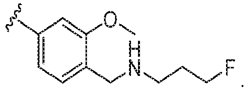 In certain embodiments, R^{1d} is 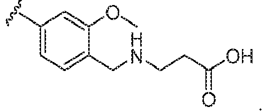 In certain embodiments, R^{1d} is 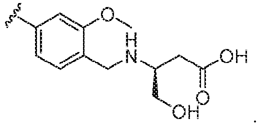 In certain embodiments, R^{1d} is 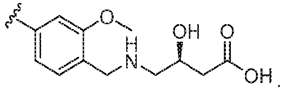 In certain embodiments, R^{1d} is 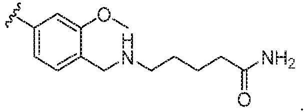 In certain embodiments, R^{1d} is 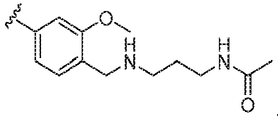 In certain embodiments, R^{1d} is 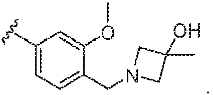 In certain embodiments, R^{1d} is 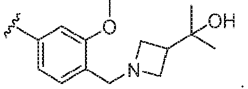 In certain embodiments, R^{1d} is 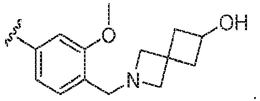 In certain embodiments, R^{1d} is 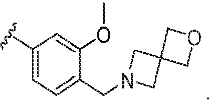 In certain embodiments, R^{1d} is 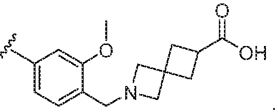 In certain embodiments, R^{1d} is 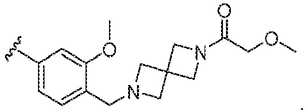 In certain embodiments, R^{1d} is 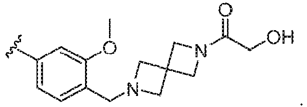 In certain embodiments, R^{1d} is 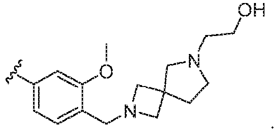 In certain embodiments, R^{1d} is 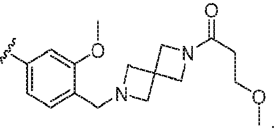 In certain embodiments, R^{1d} is 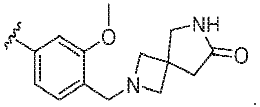 In certain embodiments, R^{1d} is 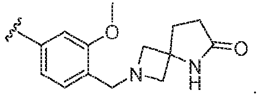 In certain embodiments, R^{1d} is 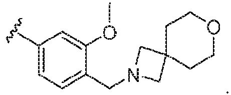 In certain embodiments, R^{1d} is 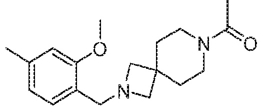 . In certain embodiments, R^{1d} is 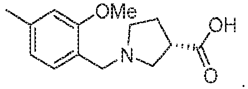 In certain embodiments, R^{1d} is 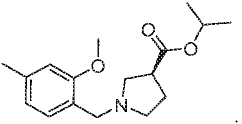 In certain embodiments, R^{1d} is 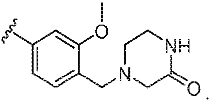 In certain embodiments, R^{1d} is 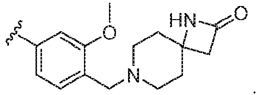 In certain embodiments, R^{1d} is 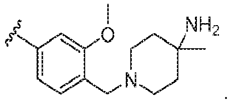 In certain embodiments, R^{1d} is 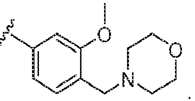 In certain embodiments, R^{1d} is 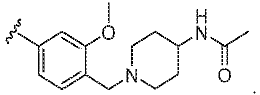 In certain embodiments, R^{1d} is 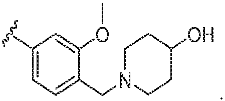 In certain embodiments, R^{1d} is 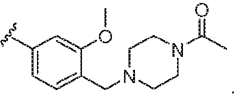 In certain embodiments, R^{1d} is 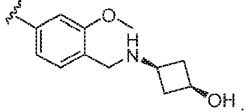 In certain embodiments, R^{1d} is 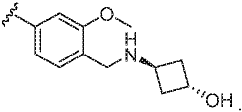 In certain embodiments, R^{1d} is 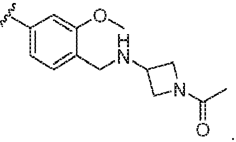 In certain embodiments, R^{1d} is 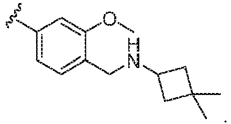 In certain embodiments, R^{1d} is 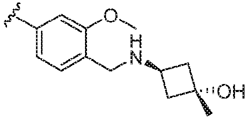 . In certain embodiments, R^{1d} is 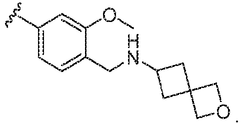 In certain embodiments, R^{1d} is 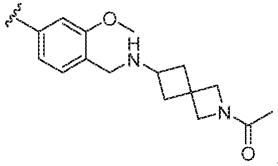 . In certain embodiments, R^{1d} is 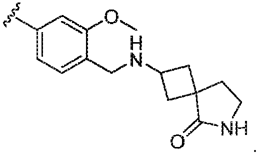 In certain embodiments, R^{1d} is 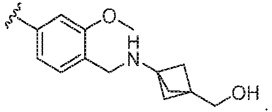 In certain embodiments, R^{1d} is 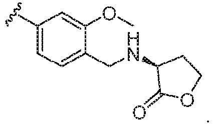 In certain embodiments, R^{1d} is 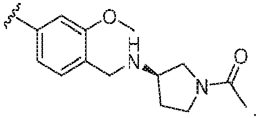 In certain embodiments, R^{1d} is 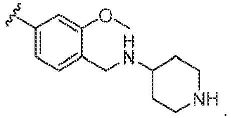 In certain embodiments, R^{1d} is 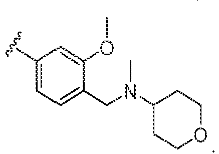 In certain embodiments, R^{1d} is 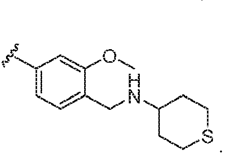 In certain embodiments, R^{1d} is 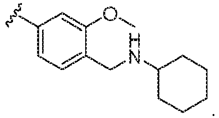 In certain embodiments, R^{1d} is 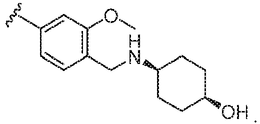 In certain embodiments, R^{1d} is 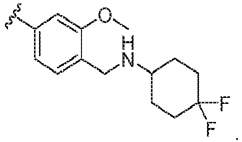 In certain embodiments, R^{1d} is 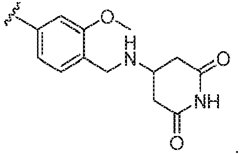 In certain embodiments, R^{1d} is 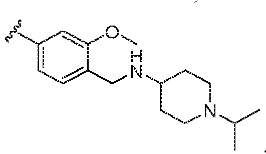 In certain embodiments, R^{1d} is 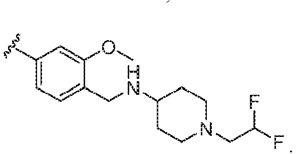 In certain embodiments, R^{1d} is 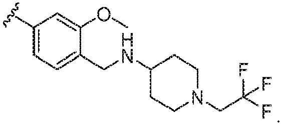 In certain embodiments, R^{1d} is 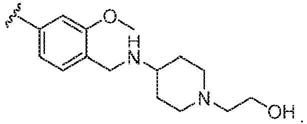 In certain embodiments, R^{1d} is 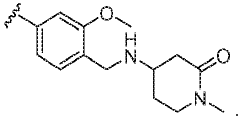 In certain embodiments, R^{1d} is 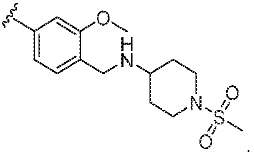 In certain embodiments, R^{1d} is 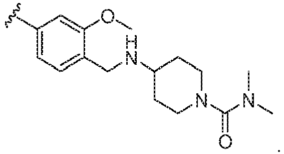 In certain embodiments, R^{1d} is 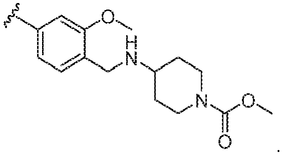 In certain embodiments, R^{1d} is 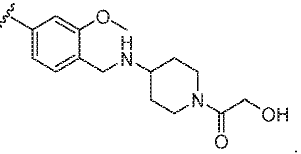 In certain embodiments, R^{1d} is 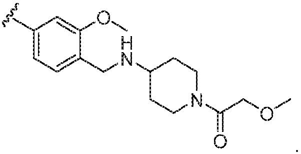 In certain embodiments, R^{1d} is 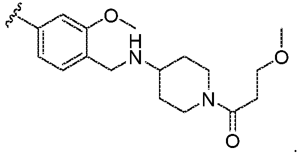 In certain embodiments, R^{1d} is 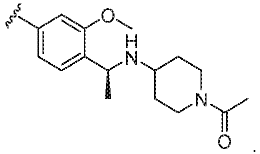 In certain embodiments, R^{1d} is 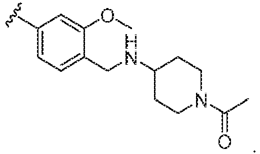 In certain embodiments, R^{1d} is 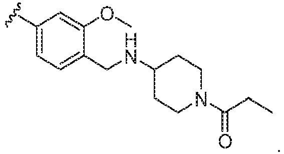 In certain embodiments, R^{1d} is 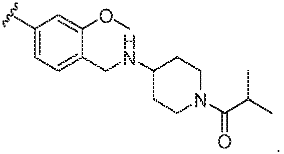 In certain embodiments, R^{1d} is 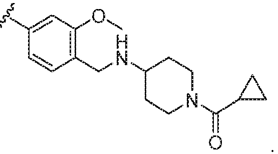 In certain embodiments, R^{1d} is 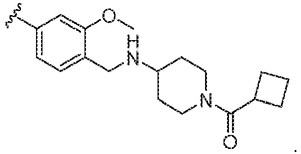 In certain embodiments, R^{1d} is 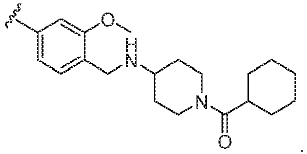 In certain embodiments, R^{1d} is 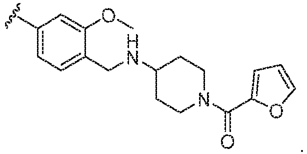 In certain embodiments, R^{1d} is 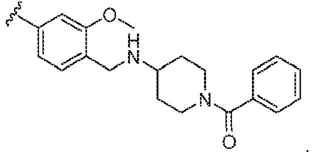 In certain embodiments, R^{1d} is 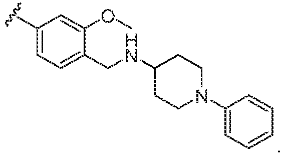 In certain embodiments, R^{1d} is 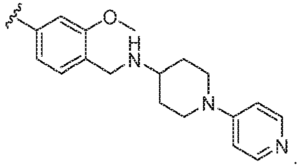 In certain embodiments, R^{1d} is 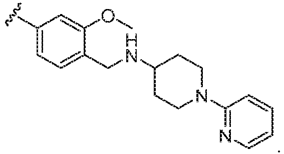 In certain embodiments, R^{1d} is 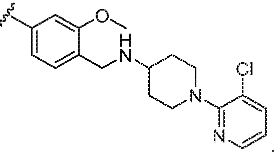 In certain embodiments, R^{1d} is 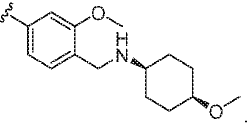 In certain embodiments, R^{1d} is 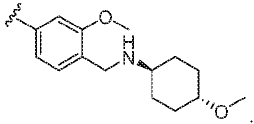 In certain embodiments, R^{1d} is 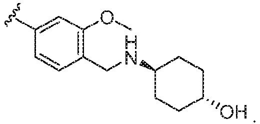 In certain embodiments, R^{1d} is 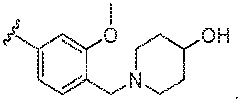 In certain embodiments, R^{1d} is 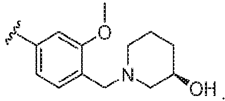 In certain embodiments, R^{1d} is 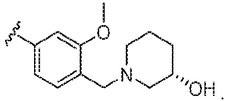 In certain embodiments, R^{1d} is 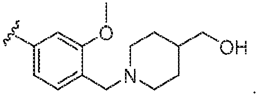 In certain embodiments, R^{1d} is 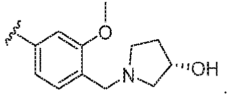 In certain embodiments, R^{1d} is 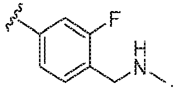 In certain embodiments, R^{1d} is 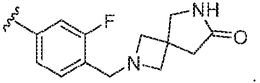 In certain embodiments, R^{1d} is 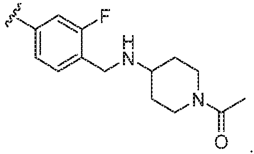 In certain embodiments, R^{1d} is 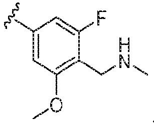 In certain embodiments, R^{1d} is 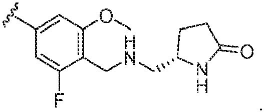 In certain embodiments, R^{1d} is 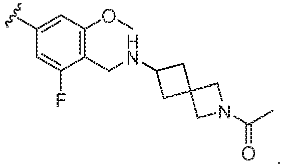 In certain embodiments, R^{1d} is 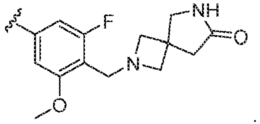 In certain embodiments, R^{1d} is 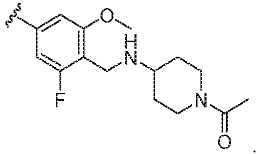 In certain embodiments, R^{1d} is 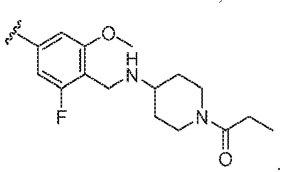 In certain embodiments, R^{1d} is 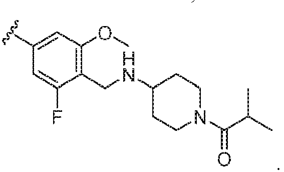 In certain embodiments, R^{1d} is 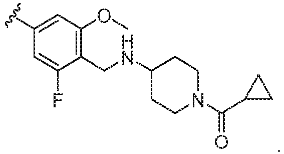 In certain embodiments, R^{1d} is 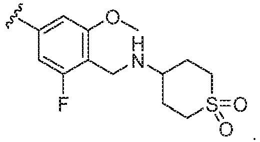 In certain embodiments, R^{1d} is 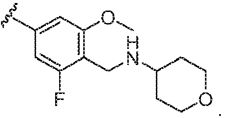 In certain embodiments, R^{1d} is 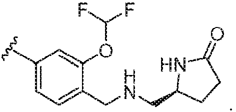 In certain embodiments, R^{1d} is 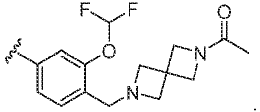 In certain embodiments, R^{1d} is 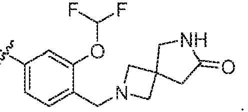 In certain embodiments, R^{1d} is 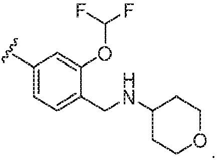 In certain embodiments, R^{1d} is 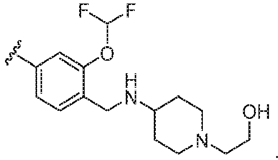 In certain embodiments, R^{1d} is 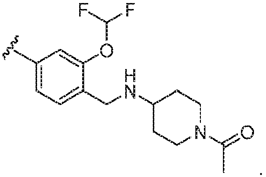 In certain embodiments, R^{1d} is 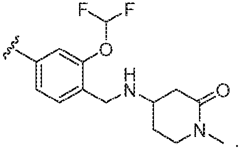 In certain embodiments, R^{1d} is 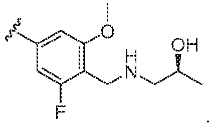 In certain embodiments, R^{1d} is 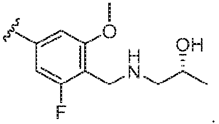 In certain embodiments, R^{1d} is 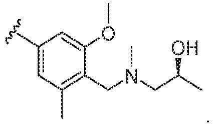 In certain embodiments, R^{1d} is 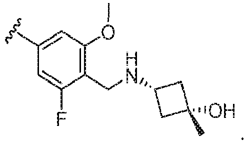 In certain embodiments, R^{1d} is 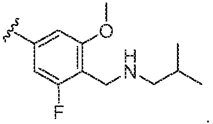 In certain embodiments, R^{1d} is 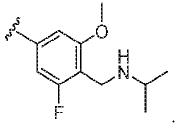 In certain embodiments, R^{1d} is 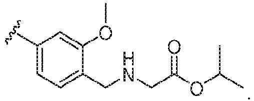 In certain embodiments, R^{1d} is 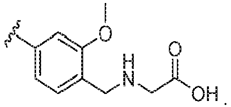 In certain embodiments, R^{1d} is 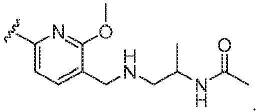 In certain embodiments, R^{1d} is 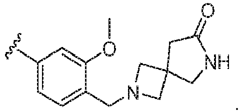 In certain embodiments, R^{1d} is 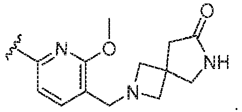 In certain embodiments, R^{1d} is 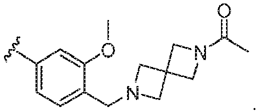 In certain embodiments, R^{1d} is 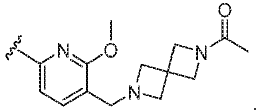 In certain embodiments, R^{1d} is 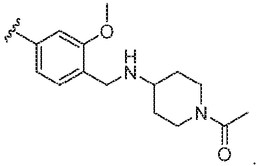 In certain embodiments, R^{1d} is 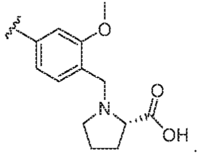 In certain embodiments, R^{1d} is 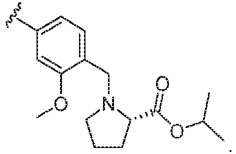 In certain embodiments, R^{1d} is 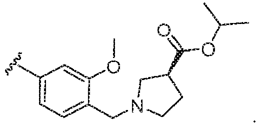 In certain embodiments, R^{1d} is 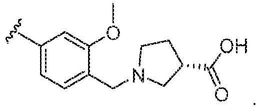 In certain embodiments, R^{1d} is 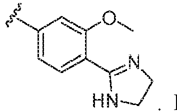 In certain embodiments, R^{1d} is 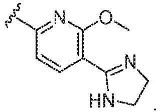 In certain embodiments, R^{1d} is 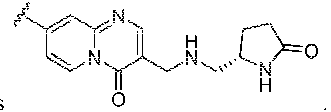 In certain embodiments, R^{1d} is 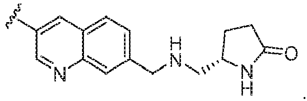 In certain embodiments, R^{1d} is 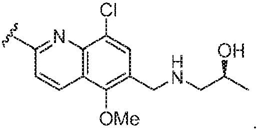 In certain embodiments, R^{1d} is 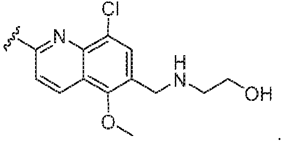 In certain embodiments, R^{1d} is 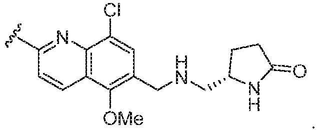 In certain embodiments, R^{1d} is 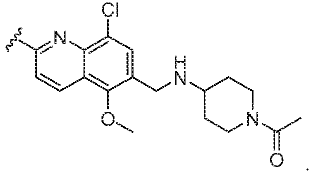 In certain embodiments, R^{1d} is 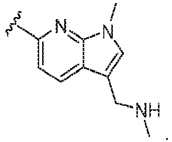 In certain embodiments, R^{1d} is 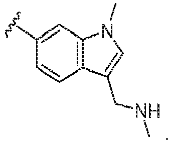 In certain embodiments, R^{1d} is 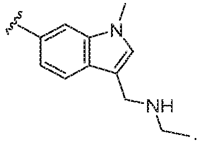 In certain embodiments, R^{1d} is 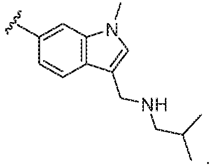 In certain embodiments, R^{1d} is 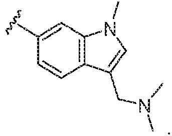 In certain embodiments, R^{1d} is 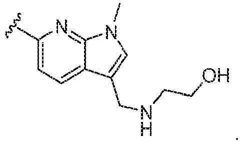 In certain embodiments, R^{1d} is 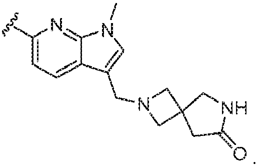 In certain embodiments, R^{1d} is 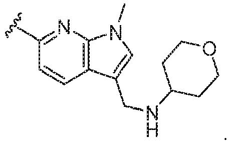 In certain embodiments, R^{1d} is 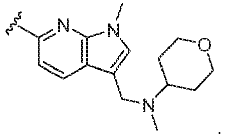 In certain embodiments, R^{1d} is 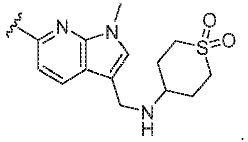 In certain embodiments, R^{1d} is 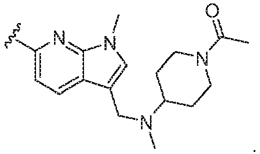 In certain embodiments, R^{1d} is 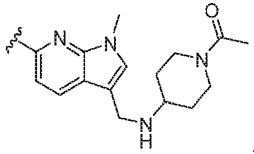 In certain embodiments, R^{1d} is 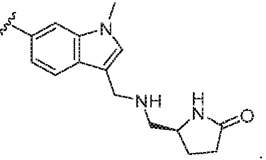 In certain embodiments, R^{1d} is 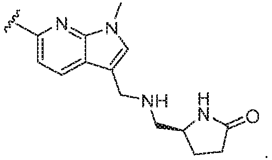 In certain embodiments, R^{1d} is 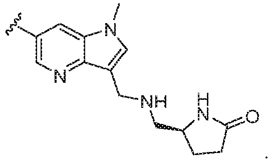 In certain embodiments, R^{1d} is 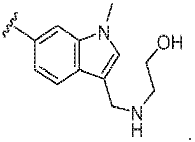 In certain embodiments, R^{1d} is 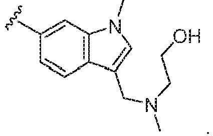 In certain embodiments, R^{1d} is 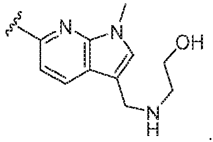 In certain embodiments, R^{1d} is 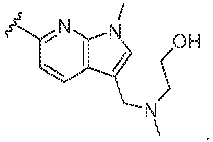 In certain embodiments, R^{1d} is 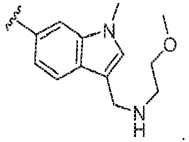 In certain embodiments, R^{1d} is 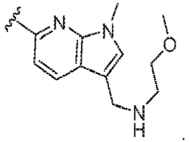 In certain embodiments, R^{1d} is 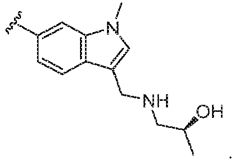 In certain embodiments, R^{1d} is 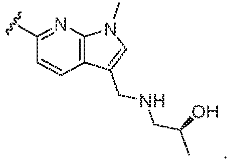 In certain embodiments, R^{1d} is 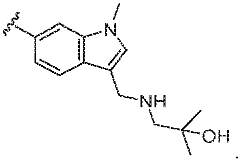 In certain embodiments, R^{1d} is 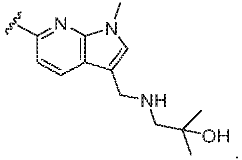 In certain embodiments, R^{1d} is 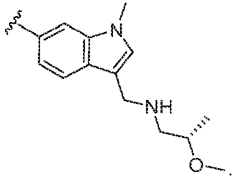 In certain embodiments, R^{1d} is 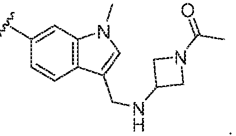 In certain embodiments, R^{1d} is 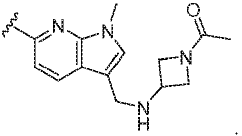 In certain embodiments, R^{1d} is 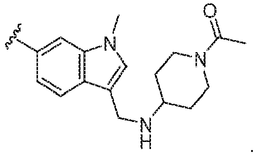 In certain embodiments, R^{1d} is 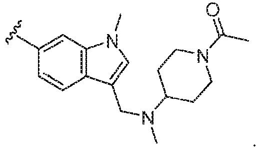 In certain embodiments, R^{1d} is 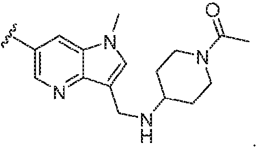 In certain embodiments, R^{1d} is 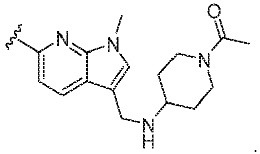 In certain embodiments, R^{1d} is 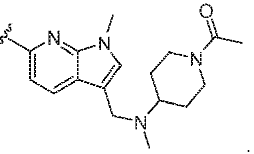 In certain embodiments, R^{1d} is 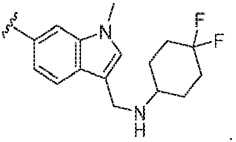 In certain embodiments, R^{1d} is 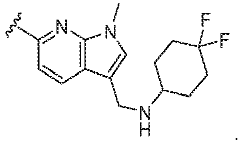 In certain embodiments, R^{1d} is 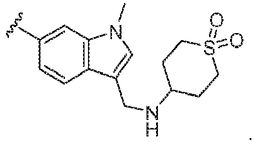 In certain embodiments, R^{1d} is 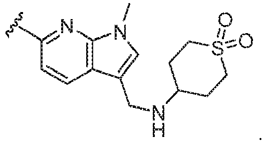 In certain embodiments, R^{1d} is 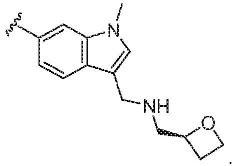 In certain embodiments, R^{1d} is 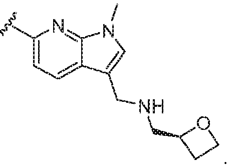 In certain embodiments, R^{1d} is 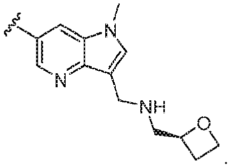 In certain embodiments, R^{1d} is 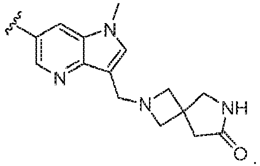 In certain embodiments, R^{1d} is 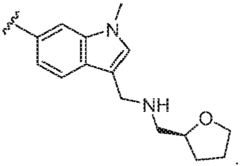 In certain embodiments, R^{1d} is 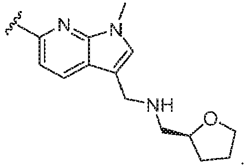 In certain embodiments, R^{1d} is 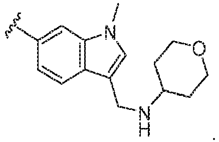 In certain embodiments, R^{1d} is 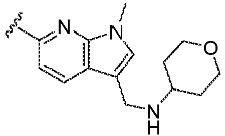 . In certain embodiments, R^{1d} is 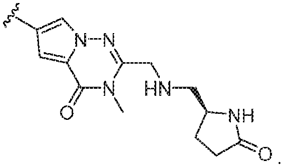 In certain embodiments, R^{1d} is 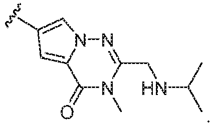 In certain embodiments, R^{1d} is 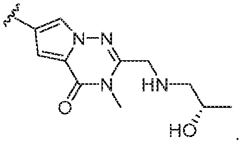 In certain embodiments, R^{1d} is 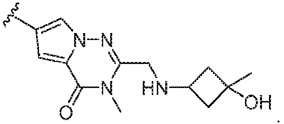 In certain embodiments, R^{1d} is 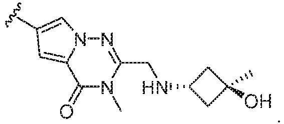 In certain embodiments, R^{1d} is 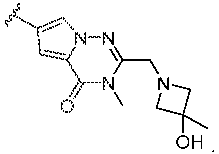 In certain embodiments, R^{1d} is 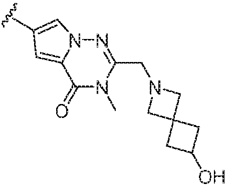 . In certain embodiments, R^{1d} is 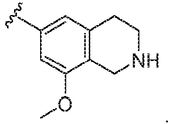 In certain embodiments, R^{1d} is 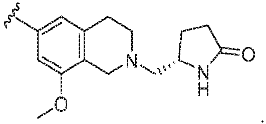 In certain embodiments, R^{1d} is 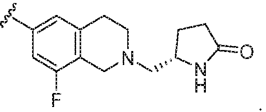 In certain embodiments, R^{1d} is 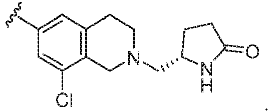 In certain embodiments, R^{1d} is 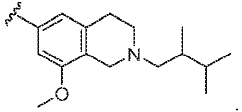 In certain embodiments, R^{1d} is 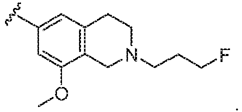 In certain embodiments, R^{1d} is 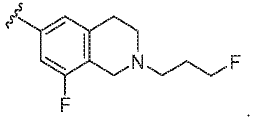 In certain embodiments, R^{1d} is 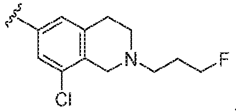 In certain embodiments, R^{1d} is 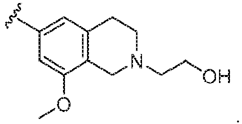 In certain embodiments, R^{1d} is 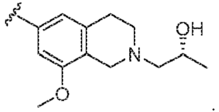 In certain embodiments, R^{1d} is 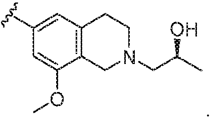 In certain embodiments, R^{1d} is 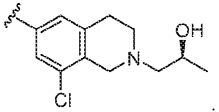 In certain embodiments, R^{1d} is 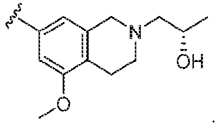 In certain embodiments, R^{1d} is 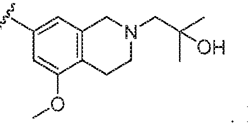 In certain embodiments, R^{1d} is 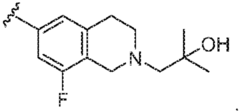 In certain embodiments, R^{1d} is 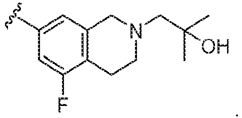 In certain embodiments, R^{1d} is 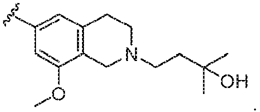 In certain embodiments, R^{1d} is 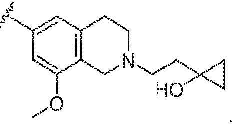 In certain embodiments, R^{1d} is 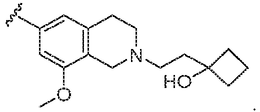 In certain embodiments, R^{1d} is 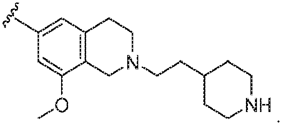 In certain embodiments, R^{1d} is 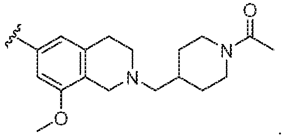 In certain embodiments, R^{1d} is 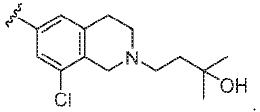 In certain embodiments, R^{1d} is 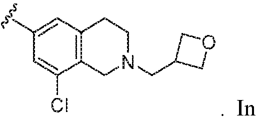 certain embodiments, R^{1d} is 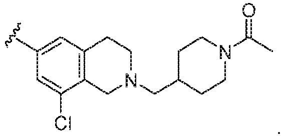 In certain embodiments, R^{1d} is 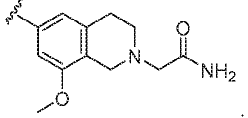 In certain embodiments, R^{1d} is 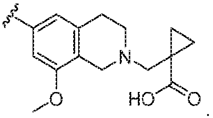 In certain embodiments, R^{1d} is 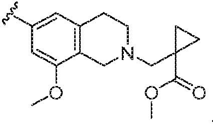 In certain embodiments, R^{1d} is 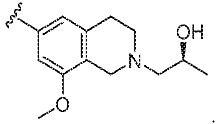 In certain embodiments, R^{1d} is 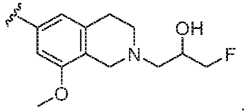 In certain embodiments, R^{1d} is 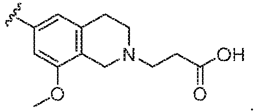 In certain embodiments, R^{1d} is 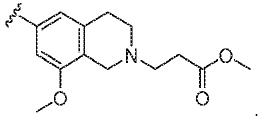 In certain embodiments, R^{1d} is 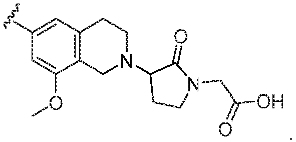 In certain embodiments, R^{1d} is 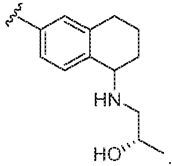 In certain embodiments, R^{1d} is 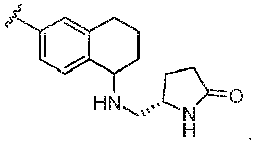 In certain embodiments, R^{1d} is 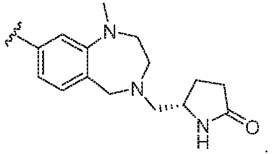 In certain embodiments, R^{1d} is 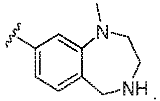 In certain embodiments, R^{1d} is 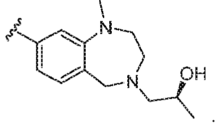 In certain embodiments, R^{1d} is 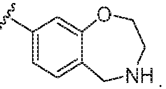 In certain embodiments, R^{1d} is 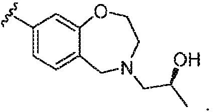 In certain embodiments, R^{1d} is 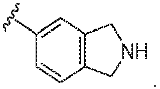 In certain embodiments, R^{1d} is 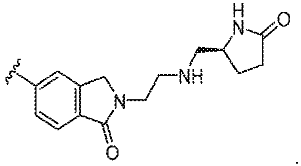 **In** certain embodiments, R^{1d} is 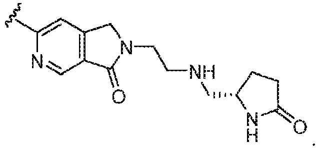 In certain embodiments, R^{1d} is 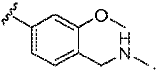 In certain embodiments, R^{1d} is 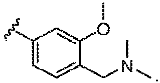 In certain embodiments, R^{1d} is 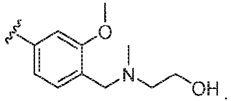 In certain embodiments, R^{1d} is 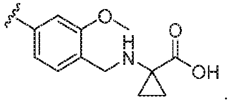 In certain embodiments, R^{1d} is 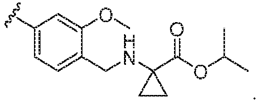 In certain embodiments, R^{1d} is 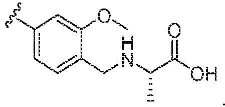 In certain embodiments, R^{1d} is 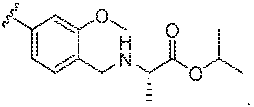 In certain embodiments, R^{1d} is 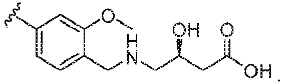 In certain embodiments, R^{1d} is 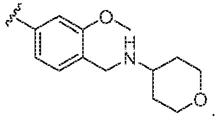 In certain embodiments, R^{1d} is 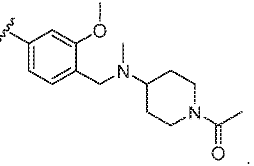 In certain embodiments, R^{1d} is 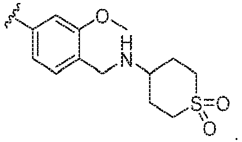 In certain embodiments, R^{1d} is 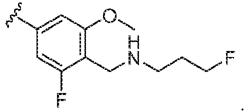 In certain embodiments, R^{1d} is 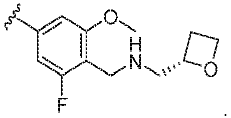 In certain embodiments, R^{1d} is 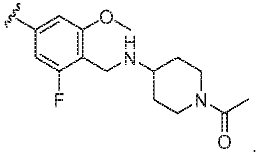 In certain embodiments, R^{1d} is 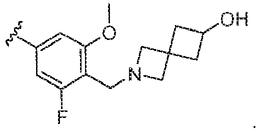 In certain embodiments, R^{1d} is 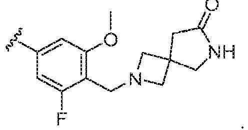 In certain embodiments, R^{1d} is 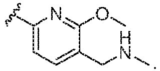 In certain embodiments, R^{1d} is 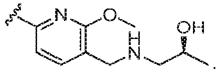 In certain embodiments, R^{1d} is 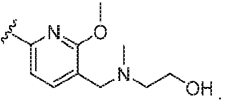 In certain embodiments, R^{1d} is 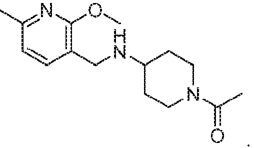 In certain embodiments, R^{1d} is 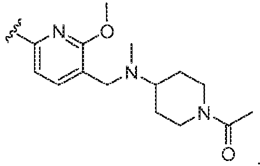 In certain embodiments, R^{1d} is 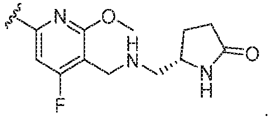 In certain embodiments, R^{1d} is 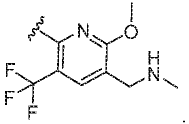 In certain embodiments, R^{1d} is 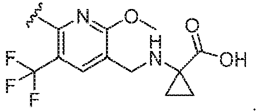 In certain embodiments, R^{1d} is 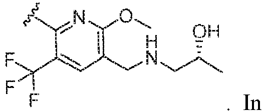 certain embodiments, R^{1d} is 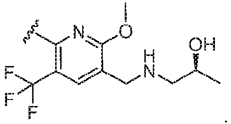 In certain embodiments, R^{1d} is 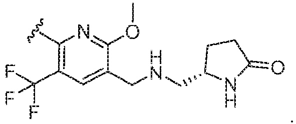 In certain embodiments, R^{1d} is 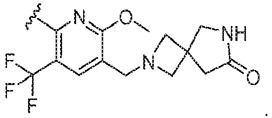 In certain embodiments, R^{1d} is 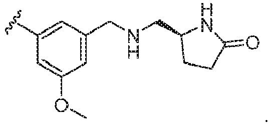 In certain embodiments, R^{1d} is 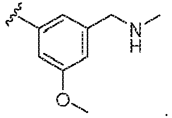 In certain embodiments, R^{1d} is 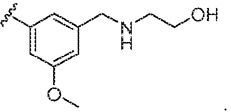 In certain embodiments, R^{1d} is 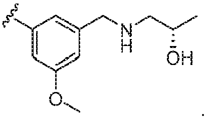 In certain embodiments, R^{1d} is 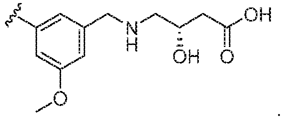 In certain embodiments, R^{1d} is 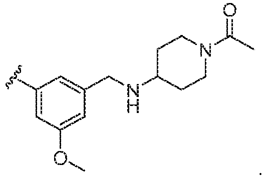 In certain embodiments, R^{1d} is 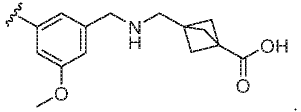 In certain embodiments, R^{1d} is 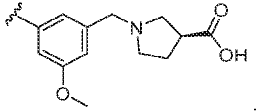 In certain embodiments, R^{1d} is 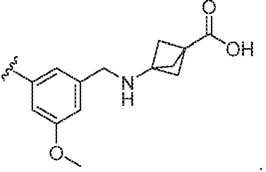 In certain embodiments, R^{1d} is 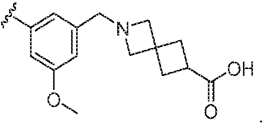 In certain embodiments, R^{1d} is 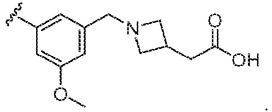 In certain embodiments, R^{1d} is 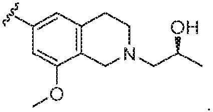 In certain embodiments, R^{1d} is 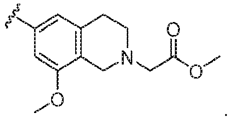 In certain embodiments, R^{1d} is 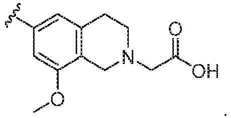 In certain embodiments, R^{1d} is 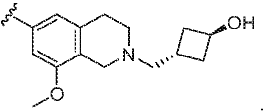 In certain embodiments, R^{1d} is 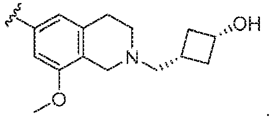 In certain embodiments, R^{1d} is 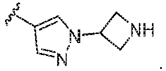 In certain embodiments, R^{1d} is 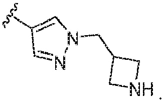 In certain embodiments, R^{1d} is 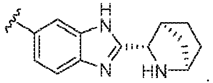 In certain embodiments, R^{1d} is 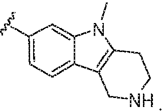 In certain embodiments, R^{1d} is 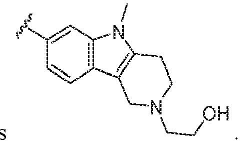 In certain embodiments, R^{1d} is 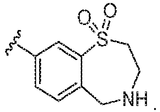 In certain embodiments, R^{1d} is 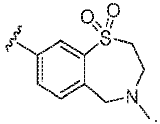 In certain embodiments, R^{1d} is 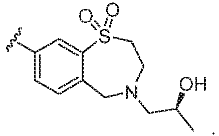 In certain embodiments, R^{1d} is 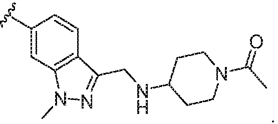 In certain embodiments, R^{1d} is 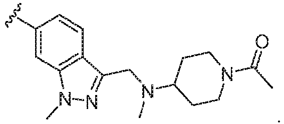 In certain embodiments, R^{1d} is 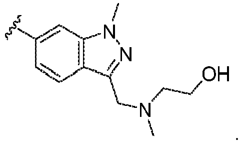 In certain embodiments, R^{1d} is 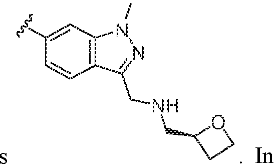 certain embodiments, R^{1d} is 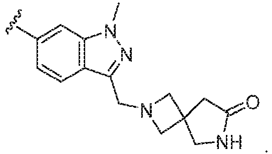 In certain embodiments, R^{1d} is 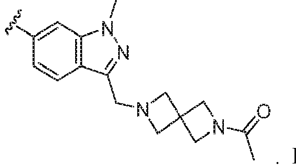 In certain embodiments, R^{1d} is 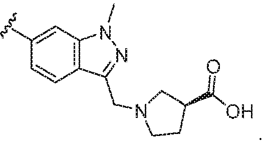 In certain embodiments, R^{1d} is 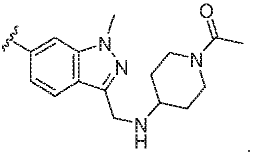 In certain embodiments, R^{1d} is 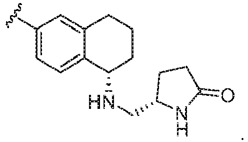 In certain embodiments, R^{1d} is 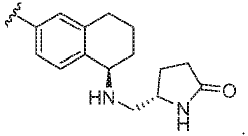 In certain embodiments, R^{1d} is 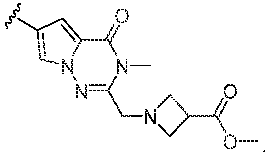 In certain embodiments, R^{1d} is 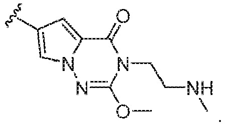 In certain embodiments, R^{1d} is 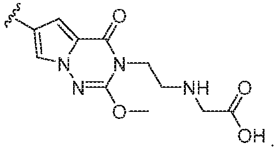 In certain embodiments, R^{1d} is 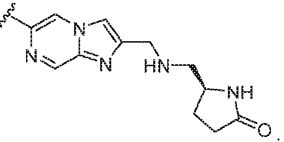 In certain embodiments, R^{1d} is 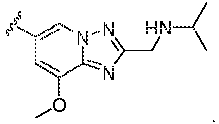 In certain embodiments, R^{1d} is 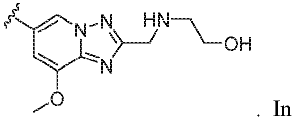 certain embodiments, R^{1d} is 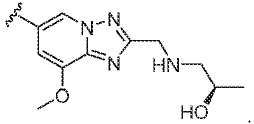 In certain embodiments, R^{1d} is 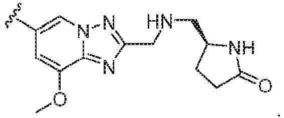 In certain embodiments, R^{1d} is 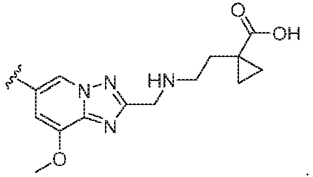 In certain embodiments, R^{1d} is 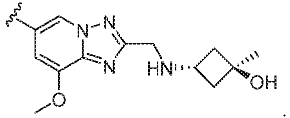 In certain embodiments, R^{1d} is 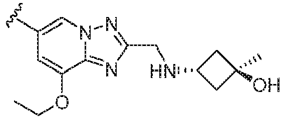 . In certain embodiments, R^{1d} is 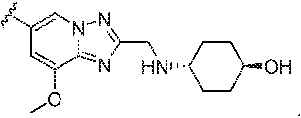 In certain embodiments, R^{1d} is 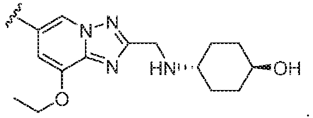 In certain embodiments, R^{1d} is 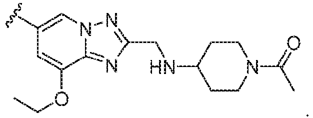 In certain embodiments, R^{1d} is 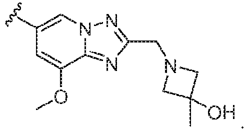 In certain embodiments, R^{1d} is 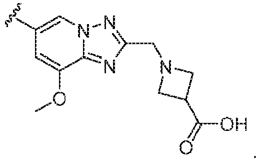 In certain embodiments, R^{1d} is 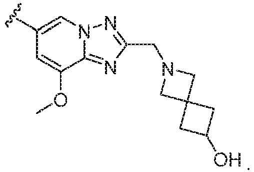 In certain embodiments, R^{1d} is 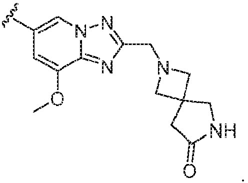 In certain embodiments, R^{1d} is 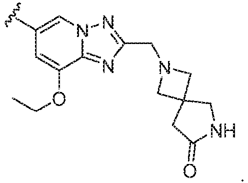 In certain embodiments, R^{1d} is 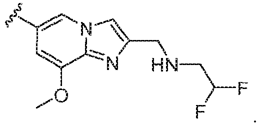 In certain embodiments, R^{1d} is 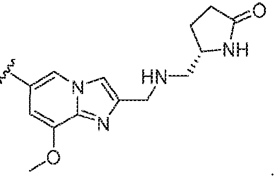 In certain embodiments, R^{1d} is 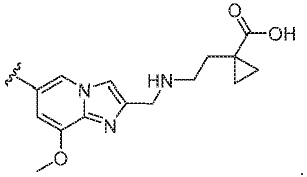 In certain embodiments, R^{1d} is 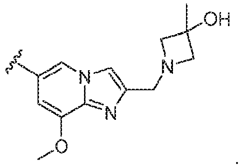 In certain embodiments, R^{1d} is 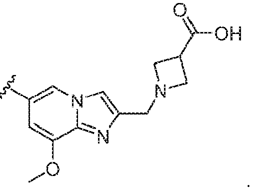 In certain embodiments, R^{1d} is 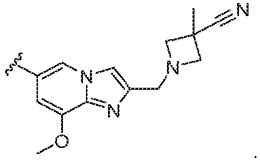 In certain embodiments, R^{1d} is 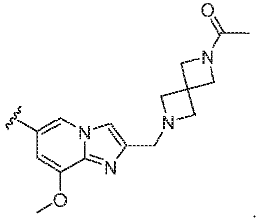 In certain embodiments, R^{1d} is 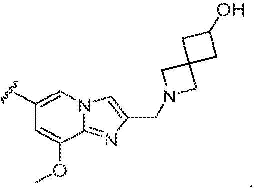 In certain embodiments, R^{1d} is 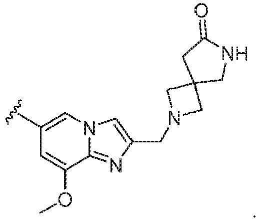 In certain embodiments, R^{1d} is 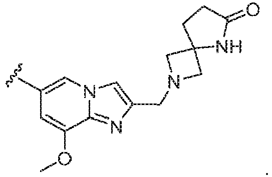 In certain embodiments, R^{1d} is 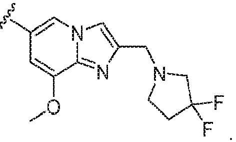 In certain embodiments, R^{1d} is 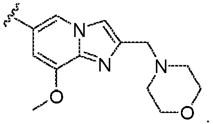 In certain embodiments, R^{1d} is 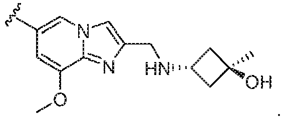 In certain embodiments, R^{1d} is 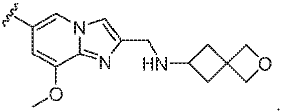 In certain embodiments, R^{1d} is 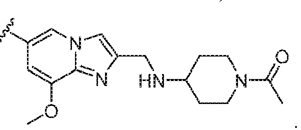 In certain embodiments, R^{1d} is 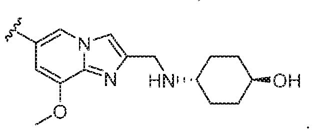 In certain embodiments, R^{1d} is 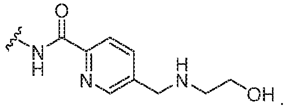 In certain embodiments, R^{1d} is 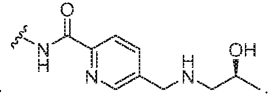 In certain embodiments, R^{1d} is 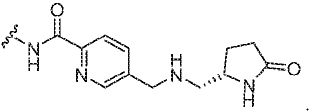 In certain embodiments, R^{1d} is 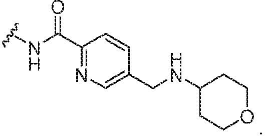 In certain embodiments, R^{1d} is 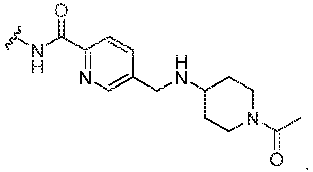 In certain embodiments, R^{1d} is 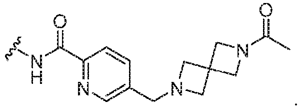 In certain embodiments, R^{1d} is 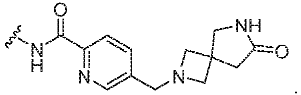 In certain embodiments, R^{1d} is 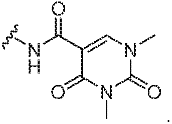 In certain embodiments, R^{1d} is 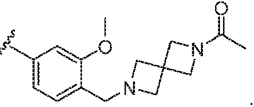 In certain embodiments, R^{1d} is 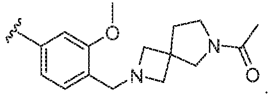 In certain embodiments, R^{1d} is 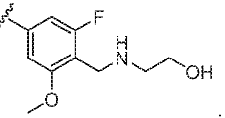 In certain embodiments, R^{1d} is 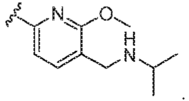 In certain embodiments, R^{1d} is 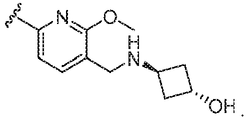 In certain embodiments, R^{1d} is 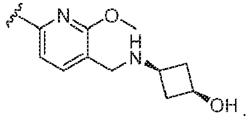 In certain embodiments, R^{1d} is 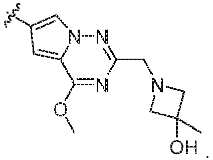 In certain embodiments, R^{1d} is 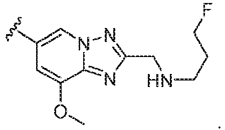 In certain embodiments, R^{1d} is 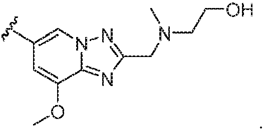 In certain embodiments, R^{1d} is 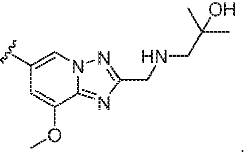 In certain embodiments, R^{1d} is 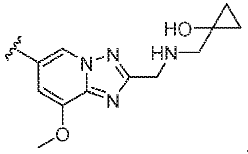 In certain embodiments, R^{1d} is 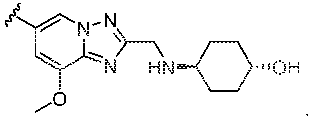 In certain embodiments, R^{1d} is 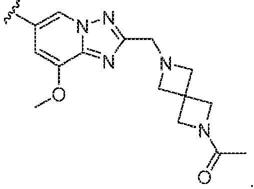 In certain embodiments, R^{1d} is 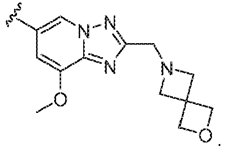 In certain embodiments, R^{1d} is 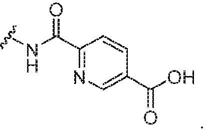 In certain embodiments, R^{1d} is 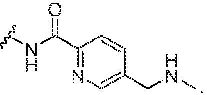 In certain embodiments, R^{1d} is 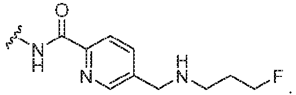 In certain embodiments, R^{1d} is 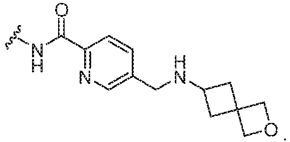 In certain embodiments, R^{1d} is

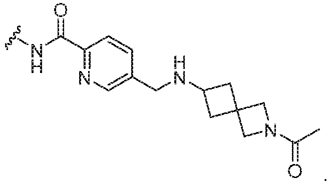

In certain embodiments, the compound is selected from the group consisting of:
8-(2-chloro-3-(3-chloro-2-(3-methoxy-4-(((((S)-5-oxopyrrolidin-2-yl)methyl)amino)methyl)phenyl)pyridin-4-yl)phenyl)-3-(((((S)-5-oxopyrrolidin-2-yl)methyl)amino)methyl)-4H-pyrido[1,2-a]pyrimidin-4-one;
8-(2-chloro-3-(3-chloro-2-(3-methoxy-4-(((((S)-5-oxopyrrolidin-2-yl)methyl)amino)methyl)phenyl)pyridin-4-yl)phenyl)-3-(((((R)-5-oxopyrrolidin-2-yl)methyl)amino)methyl)-4H-pyrido[1,2-a]pyrimidin-4-one;
8-(2-chloro-3-(3-chloro-2-(3-methoxy-4-(((((R)-5-oxopyrrolidin-2-yl)methyl)amino)methyl)phenyl)pyridin-4-yl)phenyl)-3-(((((S)-5-oxopyrrolidin-2-yl)methyl)amino)methyl)-4H-pyrido[1,2-a]pyrimidin-4-one;
8-(2-chloro-3-(3-chloro-2-(3-methoxy-4-(((((R)-5-oxopyrrolidin-2-yl)methyl)amino)methyl)phenyl)pyridin-4-yl)phenyl)-3-(((((R)-5-oxopyrrolidin-2-yl)methyl)amino)methyl)-4H-pyrido[1,2-a]pyrimidin-4-one;
8-(3-chloro-4-(2-chloro-3-(6-methoxy-5-(((((S)-5-oxopyrrolidin-2-yl)methyl)amino)methyl)pyridin-2-yl)phenyl)pyridin-2-yl)-3-(((((S)-5-oxopyrrolidin-2-yl)methyl)amino)methyl)-4H-pyrido[1,2-a]pyrimidin-4-one;
8-(3-chloro-4-(2-chloro-3-(6-methoxy-5-(((((S)-5-oxopyrrolidin-2-yl)methyl)amino)methyl)pyridin-2-yl)phenyl)pyridin-2-yl)-3-(((((R)-5-oxopyrrolidin-2-yl)methyl)amino)methyl)-4H-pyrido[1,2-a]pyrimidin-4-one;
8-(3-chloro-4-(2-chloro-3-(6-methoxy-5-(((((R)-5-oxopyrrolidin-2-yl)methyl)amino)methyl)pyridin-2-yl)phenyl)pyridin-2-yl)-3-(((((S)-5-oxopyrrolidin-2-yl)methyl)amino)methyl)-4H-pyrido[1,2-a]pyrimidin-4-one;
8-(3-chloro-4-(2-chloro-3-(6-methoxy-5-(((((R)-5-oxopyrrolidin-2-yl)methyl)amino)methyl)pyridin-2-yl)phenyl)pyridin-2-yl)-3-(((((R)-5-oxopyrrolidin-2-yl)methyl)amino)methyl)-4H-pyrido[1,2-a]pyrimidin-4-one;
(S)-5-((((6-(2-chloro-3-(3-chloro-2-(1-methyl-2,3,4,5-tetrahydro-1H-benzo[e][1,4]diazepin-8-yl)pyridin-4-yl)phenyl)-2-methoxypyridin-3-yl)methyl)amino)methyl)pyrrolidin-2-one;
(R)-5-((((6-(2-chloro-3-(3-chloro-2-(1-methyl-2,3,4,5-tetrahydro-1H-benzo[e][1,4]diazepin-8-yl)pyridin-4-yl)phenyl)-2-methoxypyridin-3-yl)methyl)amino)methyl)pyrrolidin-2-one;
(5S,5'S)-5,5'-(((((3,3'-dichloro-[4,4'-bipyridine]-2,2'-diyl)bis(2-methoxy-4,1-phenylene))bis(methylene))bis(azanediyl))bis(methylene))bis(pyrrolidin-2-one);
(5S,5'R)-5,5'-(((((3,3'-dichloro-[4,4'-bipyridine]-2,2'-diyl)bis(2-methoxy-4,1-phenylene))bis(methylene))bis(azanediyl))bis(methylene))bis(pyrrolidin-2-one);
(5R,5'S)-5,5'-(((((3,3'-dichloro-[4,4'-bipyridine]-2,2'-diyl)bis(2-methoxy-4,1-phenylene))bis(methylene))bis(azanediyl))bis(methylene))bis(pyrrolidin-2-one);
(5R,5'R)-5,5'-(((((3,3'-dichloro-[4,4'-bipyridine]-2,2'-diyl)bis(2-methoxy-4,1-phenylene))bis(methylene))bis(azanediyl))bis(methylene))bis(pyrrolidin-2-one);
N-(1-(((6-(2-chloro-3-(3-chloro-2-(1-methyl-2,3,4,5-tetrahydro-1H-benzo[e][1,4]diazepin-8-yl)pyridin-4-yl)phenyl)-2-methoxypyridin-3-yl)methyl)amino)propan-2-yl)acetamide;
(S)-N-(1-(((6-(2-chloro-3-(3-chloro-2-(1-methyl-2,3,4,5-tetrahydro-1H-benzo[e][1,4]diazepin-8-yl)pyridin-4-yl)phenyl)-2-methoxypyridin-3-yl)methyl)amino)propan-2-yl)acetamide;
(R)-N-(1-(((6-(2-chloro-3-(3-chloro-2-(1-methyl-2,3,4,5-tetrahydro-1H-benzo[e][1,4]diazepin-8-yl)pyridin-4-yl)phenyl)-2-methoxypyridin-3-yl)methyl)amino)propan-2-yl)acetamide;
(S)-5-((((6-(2-chloro-3-(3-chloro-2-(8-methoxy-2-(((S)-5-oxopyrrolidin-2-yl)methyl)-1,2,3,4-tetrahydroisoquinolin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(8-methoxy-2-(((R)-5-oxopyrrolidin-2-yl)methyl)-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(8-methoxy-2-(((S)-5-oxopyrrolidin-2-yl)methyl)-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(8-methoxy-2-(((R)-5-oxopyrrolidin-2-yl)methyl)-1,2,3,4-tetrahydroisoquinolin-6-yl)pyridin-4-yl)phenyl)-2-methoxypyridin-3-yl)methyl)amino)methyl)pyrrolidin-2-one
(S)-5-((((6-(2-chloro-3-(3-chloro-2-(8-methoxy-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(8-methoxy-1,2,3,4-tetrahydroisoquinolin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(3-methoxy-4-(((((S)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
(S)-5-((((6-(2-chloro-3-(3-chloro-2-(3-methoxy-4-(((((R)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
(R)-5-((((6-(2-chloro-3-(3-chloro-2-(3-methoxy-4-(((((S)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
(R)-5-((((6-(2-chloro-3-(3-chloro-2-(3-methoxy-4-(((((R)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
(S)-5-((((6-(2-chloro-3-(3-chloro-2-(1-methyl-2,3,4,5-tetrahydro-1H-benzo[e][1,4]diazepin-8-yl)pyridin-4-yl)phenyl)-1-methyl-1H-pyrrolo[2,3-b]pyridin-3-yl)methyl)amino)methyl)pyrrolidin-2-one;
(R)-5-((((6-(2-chloro-3-(3-chloro-2-(1-methyl-2,3,4,5-tetrahydro-1H-benzo[e][1,4]diazepin-8-yl)pyridin-4-yl)phenyl)-1-methyl-1H-pyrrolo[2,3-b]pyridin-3-yl)methyl)amino)methyl)pyrrolidin-2-one;
(S)-5-((((6-(2-chloro-3-(3-chloro-2-(3-fluoro-5-methoxy-4-(((((S)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
(S)-5-((((6-(2-chloro-3-(3-chloro-2-(3-fluoro-5-methoxy-4-(((((R)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
(R)-5-((((6-(2-chloro-3-(3-chloro-2-(3-fluoro-5-methoxy-4-(((((S)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
(R)-5-((((6-(2-chloro-3-(3-chloro-2-(3-fluoro-5-methoxy-4-(((((R)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
(S)-5-((((6-(2-chloro-3-(3-chloro-2-(1-methyl-3-(((((S)-5-oxopyrrolidin-2-yl)methyl)amino)methyl)-1H-indol-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(1-methyl-3-(((((R)-5-oxopyrrolidin-2-yl)methyl)amino)methyl)-1H-indol-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(1-methyl-3-(((((S)-5-oxopyrrolidin-2-yl)methyl)amino)methyl)-1H-indol-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(1-methyl-3-(((((R)-5-oxopyrrolidin-2-yl)methyl)amino)methyl)-1H-indol-6-yl)pyridin-4-yl)phenyl)-2-methoxypyridin-3-yl)methyl)amino)methyl)pyrrolidin-2-one;
(5S,5'S)-5,5'-(((((3,3'-dichloro-[4,4'-bipyridine]-2,2'-diyl)bis(2-fluoro-6-methoxy-4,1-phenylene))bis(methylene))bis(azanediyl))bis(methylene))bis(pyrrolidin-2-one);
(5S,5'R)-5,5'-(((((3,3'-dichloro-[4,4'-bipyridine]-2,2'-diyl)bis(2-fluoro-6-methoxy-4,1-phenylene))bis(methylene))bis(azanediyl))bis(methylene))bis(pyrrolidin-2-one);
(5R,5'S)-5,5'-(((((3,3'-dichloro-[4,4'-bipyridine]-2,2'-diyl)bis(2-fluoro-6-methoxy-4,1-phenylene))bis(methylene))bis(azanediyl))bis(methylene))bis(pyrrolidin-2-one);
(5R,5'R)-5,5'-(((((3,3'-dichloro-[4,4'-bipyridine]-2,2'-diyl)bis(2-fluoro-6-methoxy-4,1-phenylene))bis(methylene))bis(azanediyl))bis(methylene))bis(pyrrolidin-2-one);
(S)-5-((((6-(2-chloro-3-(3-chloro-2-(1-methyl-3-(((((S)-5-oxopyrrolidin-2-yl)methyl)amino)methyl)-1H-pyrrolo[3,2-b]pyridin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(1-methyl-3-(((((R)-5-oxopyrrolidin-2-yl)methyl)amino)methyl)-1H-pyrrolo[3,2-b]pyridin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(1-methyl-3-(((((S)-5-oxopyrrolidin-2-yl)methyl)amino)methyl)-1H-pyrrolo[3,2-b]pyridin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(1-methyl-3-(((((R)-5-oxopyrrolidin-2-yl)methyl)amino)methyl)-1H-pyrrolo[3,2-b]pyridin-6-yl)pyridin-4-yl)phenyl)-2-methoxypyridin-3-yl)methyl)amino)methyl)pyrrolidin-2-one;
(S)-5-(((4-(3,3'-dichloro-2'-(1-methyl-3-(((((S)-5-oxopyrrolidin-2-yl)methyl)amino)methyl)-1H-indol-6-yl)-[4,4'-bipyridin]-2-yl)-2-methoxybenzyl)amino)methyl)pyrrolidin-2-one;
(S)-5-(((4-(3,3'-dichloro-2'-(1-methyl-3-(((((R)-5-oxopyrrolidin-2-yl)methyl)amino)methyl)-1H-indol-6-yl)-[4,4'-bipyridin]-2-yl)-2-methoxybenzyl)amino)methyl)pyrrolidin-2-one;
(R)-5-(((4-(3,3'-dichloro-2'-(1-methyl-3-(((((S)-5-oxopyrrolidin-2-yl)methyl)amino)methyl)-1H-indol-6-yl)-[4,4'-bipyridin]-2-yl)-2-methoxybenzyl)amino)methyl)pyrrolidin-2-one;
(R)-5-(((4-(3,3'-dichloro-2'-(1-methyl-3-(((((R)-5-oxopyrrolidin-2-yl)methyl)amino)methyl)-1H-indol-6-yl)-[4,4'-bipyridin]-2-yl)-2-methoxybenzyl)amino)methyl)pyrrolidin-2-one;
(5S,5'S)-5,5'-(((((3,3'-dichloro-[4,4'-bipyridine]-2,2'-diyl)bis(1-methyl-1H-indole-6,3-diyl))bis(methylene))bis(azanediyl))bis(methylene))bis(pyrrolidin-2-one);
(5S,5'R)-5,5'-(((((3,3'-dichloro-[4,4'-bipyridine]-2,2'-diyl)bis(1-methyl-1H-indole-6,3-diyl))bis(methylene))bis(azanediyl))bis(methylene))bis(pyrrolidin-2-one);
(5R,5'S)-5,5'-(((((3,3'-dichloro-[4,4'-bipyridine]-2,2'-diyl)bis(1-methyl-1H-indole-6,3-diyl))bis(methylene))bis(azanediyl))bis(methylene))bis(pyrrolidin-2-one);
(5R,5'R)-5,5'-(((((3,3'-dichloro-[4,4'-bipyridine]-2,2'-diyl)bis(1-methyl-1H-indole-6,3-diyl))bis(methylene))bis(azanediyl))bis(methylene))bis(pyrrolidin-2-one);
(S)-5-(((4-(3,3'-dichloro-2'-(1-methyl-3-(((((S)-5-oxopyrrolidin-2-yl)methyl)amino)methyl)-1H-pyrrolo[3,2-b]pyridin-6-yl)-[4,4'-bipyridin]-2-yl)-2-methoxybenzyl)amino)methyl)pyrrolidin-2-one;
(S)-5-(((4-(3,3'-dichloro-2'-(1-methyl-3-(((((R)-5-oxopyrrolidin-2-yl)methyl)amino)methyl)-1H-pyrrolo[3,2-b]pyridin-6-yl)-[4,4'-bipyridin]-2-yl)-2-methoxybenzyl)amino)methyl)pyrrolidin-2-one;
(R)-5-(((4-(3,3'-dichloro-2'-(1-methyl-3-(((((S)-5-oxopyrrolidin-2-yl)methyl)amino)methyl)-1H-pyrrolo[3,2-b]pyridin-6-yl)-[4,4'-bipyridin]-2-yl)-2-methoxybenzyl)amino)methyl)pyrrolidin-2-one;
(R)-5-(((4-(3,3'-dichloro-2'-(1-methyl-3-(((((R)-5-oxopyrrolidin-2-yl)methyl)amino)methyl)-1H-pyrrolo[3,2-b]pyridin-6-yl)-[4,4'-bipyridin]-2-yl)-2-methoxybenzyl)amino)methyl)pyrrolidin-2-one;
(S)-5-((((6-(2-chloro-3-(3-chloro-2-(3-methoxy-4-(((((S)-5-oxopyrrolidin-2-yl)methyl)amino)methyl)phenyl)pyridin-4-yl)phenyl)-1-methyl-1H-pyrrolo[2,3-b]pyridin-3-yl)methyl)amino)methyl)pyrrolidin-2-one;
(S)-5-((((6-(2-chloro-3-(3-chloro-2-(3-methoxy-4-(((((R)-5-oxopyrrolidin-2-yl)methyl)amino)methyl)phenyl)pyridin-4-yl)phenyl)-1-methyl-1H-pyrrolo[2,3-b]pyridin-3-yl)methyl)amino)methyl)pyrrolidin-2-one;
(R)-5-((((6-(2-chloro-3-(3-chloro-2-(3-methoxy-4-(((((S)-5-oxopyrrolidin-2-yl)methyl)amino)methyl)phenyl)pyridin-4-yl)phenyl)-1-methyl-1H-pyrrolo[2,3-b]pyridin-3-yl)methyl)amino)methyl)pyrrolidin-2-one;
(R)-5-((((6-(2-chloro-3-(3-chloro-2-(3-methoxy-4-(((((R)-5-oxopyrrolidin-2-yl)methyl)amino)methyl)phenyl)pyridin-4-yl)phenyl)-1-methyl-1H-pyrrolo[2,3-b]pyridin-3-yl)methyl)amino)methyl)pyrrolidin-2-one;
(S)-5-((6-(2-chloro-3-(3-chloro-2-(3-methoxy-4-(((((S)-5-oxopyrrolidin-2-yl)methyl)amino)methyl)phenyl)pyridin-4-yl)phenyl)-8-methoxy-3,4-dihydroisoquinolin-2(1H)-yl)methyl)pyrrolidin-2-one;
(S)-5-((6-(2-chloro-3-(3-chloro-2-(3-methoxy-4-(((((R)-5-oxopyrrolidin-2-yl)methyl)amino)methyl)phenyl)pyridin-4-yl)phenyl)-8-methoxy-3,4-dihydroisoquinolin-2(1H)-yl)methyl)pyrrolidin-2-one;
(R)-5-((6-(2-chloro-3-(3-chloro-2-(3-methoxy-4-(((((S)-5-oxopyrrolidin-2-yl)methyl)amino)methyl)phenyl)pyridin-4-yl)phenyl)-8-methoxy-3,4-dihydroisoquinolin-2(1H)-yl)methyl)pyrrolidin-2-one;
(R)-5-((6-(2-chloro-3-(3-chloro-2-(3-methoxy-4-(((((R)-5-oxopyrrolidin-2-yl)methyl)amino)methyl)phenyl)pyridin-4-yl)phenyl)-8-methoxy-3,4-dihydroisoquinolin-2(1H)-yl)methyl)pyrrolidin-2-one;
(S)-5-((((6-(2-chloro-3-(3-chloro-2-(1-methyl-4-(((S)-5-oxopyrrolidin-2-yl)methyl)-2,3,4,5-tetrahydro-1H-benzo[e][1,4]diazepin-8-yl)pyridin-4-yl)phenyl)-2-methoxypyridin-3-yl)methyl)amino)methyl)pyrrolidin-2-one;
(S)-5-((((6-(2-chloro-3-(3-chloro-2-(1-methyl-4-(((R)-5-oxopyrrolidin-2-yl)methyl)-2,3,4,5-tetrahydro-1H-benzo[e][1,4]diazepin-8-yl)pyridin-4-yl)phenyl)-2-methoxypyridin-3-yl)methyl)amino)methyl)pyrrolidin-2-one;
(R)-5-((((6-(2-chloro-3-(3-chloro-2-(1-methyl-4-(((S)-5-oxopyrrolidin-2-yl)methyl)-2,3,4,5-tetrahydro-1H-benzo[e][1,4]diazepin-8-yl)pyridin-4-yl)phenyl)-2-methoxypyridin-3-yl)methyl)amino)methyl)pyrrolidin-2-one;
(R)-5-((((6-(2-chloro-3-(3-chloro-2-(1-methyl-4-(((R)-5-oxopyrrolidin-2-yl)methyl)-2,3,4,5-tetrahydro-1H-benzo[e][1,4]diazepin-8-yl)pyridin-4-yl)phenyl)-2-methoxypyridin-3-yl)methyl)amino)methyl)pyrrolidin-2-one;
(S)-5-((((5-(2-chloro-3-(3-chloro-2-(3-methoxy-4-(((((S)-5-oxopyrrolidin-2-yl)methyl)amino)methyl)phenyl)pyridin-4-yl)phenyl)-3-methoxypyrazin-2-yl)methyl)amino)methyl)pyrrolidin-2-one;
(S)-5-((((5-(2-chloro-3-(3-chloro-2-(3-methoxy-4-(((((R)-5-oxopyrrolidin-2-yl)methyl)amino)methyl)phenyl)pyridin-4-yl)phenyl)-3-methoxypyrazin-2-yl)methyl)amino)methyl)pyrrolidin-2-one;
(R)-5-((((5-(2-chloro-3-(3-chloro-2-(3-methoxy-4-(((((S)-5-oxopyrrolidin-2-yl)methyl)amino)methyl)phenyl)pyridin-4-yl)phenyl)-3-methoxypyrazin-2-yl)methyl)amino)methyl)pyrrolidin-2-one;
(R)-5-((((5-(2-chloro-3-(3-chloro-2-(3-methoxy-4-(((((R)-5-oxopyrrolidin-2-yl)methyl)amino)methyl)phenyl)pyridin-4-yl)phenyl)-3-methoxypyrazin-2-yl)methyl)amino)methyl)pyrrolidin-2-one;
(S)-5-((((6-(3-(2-(3-(((1-acetylpiperidin-4-yl)amino)methyl)-1-methyl-1H-pyrrolo[3,2-b]pyridin-6-yl)-3-chloropyridin-4-yl)-2-chlorophenyl)-2-methoxypyridin-3-yl)methyl)amino)methyl)pyrrolidin-2-one;
(R)-5-((((6-(3-(2-(3-(((1-acetylpiperidin-4-yl)amino)methyl)-1-methyl-1H-pyrrolo[3,2-b]pyridin-6-yl)-3-chloropyridin-4-yl)-2-chlorophenyl)-2-methoxypyridin-3-yl)methyl)amino)methyl)pyrrolidin-2-one;
(S)-5-((((6-(2-chloro-3-(3-chloro-2-(1-methyl-3-(((((S)-oxetan-2-yl)methyl)amino)methyl)-1H-pyrrolo[3,2-b]pyridin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(1-methyl-3-(((((R)-oxetan-2-yl)methyl)amino)methyl)-1H-pyrrolo[3,2-b]pyridin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(1-methyl-3-(((((S)-oxetan-2-yl)methyl)amino)methyl)-1H-pyrrolo[3,2-b]pyridin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(1-methyl-3-(((((R)-oxetan-2-yl)methyl)amino)methyl)-1H-pyrrolo[3,2-b]pyridin-6-yl)pyridin-4-yl)phenyl)-2-methoxypyridin-3-yl)methyl)amino)methyl)pyrrolidin-2-one;
(S)-5-((((6-(2-chloro-3-(3-chloro-5'-methoxy-6'-(((((S)-5-oxopyrrolidin-2-yl)methyl)amino)methyl)-[2,3'-bipyridin]-4-yl)phenyl)-2-methoxypyridin-3-yl)methyl)amino)methyl)pyrrolidin-2-one;
(S)-5-((((6-(2-chloro-3-(3-chloro-5'-methoxy-6'-(((((R)-5-oxopyrrolidin-2-yl)methyl)amino)methyl)-[2,3'-bipyridin]-4-yl)phenyl)-2-methoxypyridin-3-yl)methyl)amino)methyl)pyrrolidin-2-one;
(R)-5-((((6-(2-chloro-3-(3-chloro-5'-methoxy-6'-(((((S)-5-oxopyrrolidin-2-yl)methyl)amino)methyl)-[2,3'-bipyridin]-4-yl)phenyl)-2-methoxypyridin-3-yl)methyl)amino)methyl)pyrrolidin-2-one;
(R)-5-((((6-(2-chloro-3-(3-chloro-5'-methoxy-6'-(((((R)-5-oxopyrrolidin-2-yl)methyl)amino)methyl)-[2,3'-bipyridin]-4-yl)phenyl)-2-methoxypyridin-3-yl)methyl)amino)methyl)pyrrolidin-2-one;
(S)-5-((((6-(2-chloro-3-(3-chloro-2-(4-((S)-2-hydroxypropyl)-1-methyl-2,3,4,5-tetrahydro-1H-benzo[e][1,4]diazepin-8-yl)pyridin-4-yl)phenyl)-2-methoxypyridin-3-yl)methyl)amino)methyl)pyrrolidin-2-one;
(S)-5-((((6-(2-chloro-3-(3-chloro-2-(4-((R)-2-hydroxypropyl)-1-methyl-2,3,4,5-tetrahydro-1H-benzo[e][1,4]diazepin-8-yl)pyridin-4-yl)phenyl)-2-methoxypyridin-3-yl)methyl)amino)methyl)pyrrolidin-2-one;
(R)-5-((((6-(2-chloro-3-(3-chloro-2-(4-((S)-2-hydroxypropyl)-1-methyl-2,3,4,5-tetrahydro-1H-benzo[e][1,4]diazepin-8-yl)pyridin-4-yl)phenyl)-2-methoxypyridin-3-yl)methyl)amino)methyl)pyrrolidin-2-one;
(R)-5-((((6-(2-chloro-3-(3-chloro-2-(4-((R)-2-hydroxypropyl)-1-methyl-2,3,4,5-tetrahydro-1H-benzo[e][1,4]diazepin-8-yl)pyridin-4-yl)phenyl)-2-methoxypyridin-3-yl)methyl)amino)methyl)pyrrolidin-2-one;
(S)-5-((((6-(2-chloro-3-(3-chloro-2-(4-((S)-2-hydroxypropyl)-2,3,4,5-tetrahydrobenzo[f][1,4]oxazepin-8-yl)pyridin-4-yl)phenyl)-2-methoxypyridin-3-yl)methyl)amino)methyl)pyrrolidin-2-one;
(S)-5-((((6-(2-chloro-3-(3-chloro-2-(4-((R)-2-hydroxypropyl)-2,3,4,5-tetrahydrobenzo[f][1,4]oxazepin-8-yl)pyridin-4-yl)phenyl)-2-methoxypyridin-3-yl)methyl)amino)methyl)pyrrolidin-2-one;
(R)-5-((((6-(2-chloro-3-(3-chloro-2-(4-((S)-2-hydroxypropyl)-2,3,4,5-tetrahydrobenzo[f][1,4]oxazepin-8-yl)pyridin-4-yl)phenyl)-2-methoxypyridin-3-yl)methyl)amino)methyl)pyrrolidin-2-one;
(R)-5-((((6-(2-chloro-3-(3-chloro-2-(4-((R)-2-hydroxypropyl)-2,3,4,5-tetrahydrobenzo[f][1,4]oxazepin-8-yl)pyridin-4-yl)phenyl)-2-methoxypyridin-3-yl)methyl)amino)methyl)pyrrolidin-2-one;
(S)-5-((((6-(2-chloro-3-(3-chloro-2-(2,3,4,5-tetrahydrobenzo[f][1,4]oxazepin-8-yl)pyridin-4-yl)phenyl)-2-methoxypyridin-3-yl)methyl)amino)methyl)pyrrolidin-2-one;
(R)-5-((((6-(2-chloro-3-(3-chloro-2-(2,3,4,5-tetrahydrobenzo[f][1,4]oxazepin-8-yl)pyridin-4-yl)phenyl)-2-methoxypyridin-3-yl)methyl)amino)methyl)pyrrolidin-2-one;
(S)-5-((((6-(2-chloro-3-(3-chloro-2-(2-((R)-2-hydroxypropyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(2-((S)-2-hydroxypropyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-((R)-2-hydroxypropyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-((S)-2-hydroxypropyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(S)-5-((((6-(3-(2-(3-(((1-acetylpiperidin-4-yl)amino)methyl)-1-methyl-1H-indol-6-yl)-3-chloropyridin-4-yl)-2-chlorophenyl)-2-methoxypyridin-3-yl)methyl)amino)methyl)pyrrolidin-2-one;
(R)-5-((((6-(3-(2-(3-(((1-acetylpiperidin-4-yl)amino)methyl)-1-methyl-1H-indol-6-yl)-3-chloropyridin-4-yl)-2-chlorophenyl)-2-methoxypyridin-3-yl)methyl)amino)methyl)pyrrolidin-2-one;
(S)-5-((((6-(2-chloro-3-(3-chloro-2-(3-(((2-hydroxyethyl)amino)methyl)-1-methyl-1H-indol-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(3-(((2-hydroxyethyl)amino)methyl)-1-methyl-1H-indol-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(3-(((2-methoxyethyl)amino)methyl)-1-methyl-1H-indol-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(3-(((2-methoxyethyl)amino)methyl)-1-methyl-1H-indol-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(1-methyl-3-((methylamino)methyl)-1H-indol-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(1-methyl-3-((methylamino)methyl)-1H-indol-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(3-((ethylamino)methyl)-1-methyl-1H-indol-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(3-((ethylamino)methyl)-1-methyl-1H-indol-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(3-((dimethylamino)methyl)-1-methyl-1H-indol-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(3-((dimethylamino)methyl)-1-methyl-1H-indol-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(1-methyl-3-(((((S)-oxetan-2-yl)methyl)amino)methyl)-1H-indol-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(1-methyl-3-(((((R)-oxetan-2-yl)methyl)amino)methyl)-1H-indol-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(1-methyl-3-(((((S)-oxetan-2-yl)methyl)amino)methyl)-1H-indol-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(1-methyl-3-(((((R)-oxetan-2-yl)methyl)amino)methyl)-1H-indol-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(3-((((S)-2-hydroxypropyl)amino)methyl)-1-methyl-1H-indol-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(3-((((R)-2-hydroxypropyl)amino)methyl)-1-methyl-1H-indol-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(3-((((S)-2-hydroxypropyl)amino)methyl)-1-methyl-1H-indol-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(3-((((R)-2-hydroxypropyl)amino)methyl)-1-methyl-1H-indol-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(3-(((2-hydroxy-2-methylpropyl)amino)methyl)-1-methyl-1H-indol-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(3-(((2-hydroxy-2-methylpropyl)amino)methyl)-1-methyl-1H-indol-6-yl)pyridin-4-yl)phenyl)-2-methoxypyridin-3-yl)methyl)amino)methyl)pyrrolidin-2-one;
(S)-5-((6-(3-chloro-4-(2-chloro-3-(4-chloro-6-methoxy-5-(((((S)-5-oxopyrrolidin-2-yl)methyl)amino)methyl)pyridin-2-yl)phenyl)pyridin-2-yl)-8-methoxy-3,4-dihydroisoquinolin-2(1H)-yl)methyl)pyrrolidin-2-one;
(S)-5-((6-(3-chloro-4-(2-chloro-3-(4-chloro-6-methoxy-5-(((((R)-5-oxopyrrolidin-2-yl)methyl)amino)methyl)pyridin-2-yl)phenyl)pyridin-2-yl)-8-methoxy-3,4-dihydroisoquinolin-2(1H)-yl)methyl)pyrrolidin-2-one;
(R)-5-((6-(3-chloro-4-(2-chloro-3-(4-chloro-6-methoxy-5-(((((S)-5-oxopyrrolidin-2-yl)methyl)amino)methyl)pyridin-2-yl)phenyl)pyridin-2-yl)-8-methoxy-3,4-dihydroisoquinolin-2(1H)-yl)methyl)pyrrolidin-2-one;
(R)-5-((6-(3-chloro-4-(2-chloro-3-(4-chloro-6-methoxy-5-(((((R)-5-oxopyrrolidin-2-yl)methyl)amino)methyl)pyridin-2-yl)phenyl)pyridin-2-yl)-8-methoxy-3,4-dihydroisoquinolin-2(1H)-yl)methyl)pyrrolidin-2-one;
(S)-5-((((6-(2-chloro-3-(3-chloro-2-(3-((isobutylamino)methyl)-1-methyl-1H-indol-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(3-((isobutylamino)methyl)-1-methyl-1H-indol-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(3-(((2-hydroxyethyl)(methyl)amino)methyl)-1-methyl-1H-indol-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(3-(((2-hydroxyethyl)(methyl)amino)methyl)-1-methyl-1H-indol-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(3-((((S)-2-methoxypropyl)amino)methyl)-1-methyl-1H-indol-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(3-((((R)-2-methoxypropyl)amino)methyl)-1-methyl-1H-indol-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(3-((((S)-2-methoxypropyl)amino)methyl)-1-methyl-1H-indol-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(3-((((R)-2-methoxypropyl)amino)methyl)-1-methyl-1H-indol-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(1-methyl-3-(((((S)-tetrahydrofuran-2-yl)methyl)amino)methyl)-1H-indol-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(1-methyl-3-(((((R)-tetrahydrofuran-2-yl)methyl)amino)methyl)-1H-indol-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(1-methyl-3-(((((S)-tetrahydrofuran-2-yl)methyl)amino)methyl)-1H-indol-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(1-methyl-3-(((((R)-tetrahydrofuran-2-yl)methyl)amino)methyl)-1H-indol-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(1-methyl-3-(((tetrahydro-2H-pyran-4-yl)amino)methyl)-1H-indol-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(1-methyl-3-(((tetrahydro-2H-pyran-4-yl)amino)methyl)-1H-indol-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(3-(((1,1-dioxidotetrahydro-2H-thiopyran-4-yl)amino)methyl)- 1-methyl-1H-indol-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(3-(((1,1-dioxidotetrahydro-2H-thiopyran-4-yl)amino)methyl)- 1-methyl-1H-indol-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(2-((S)-2-hydroxypropyl)-5-methoxy-1,2,3,4-tetrahydroisoquinolin-7-yl)pyridin-4-yl)phenyl)-2-methoxypyridin-3-yl)methyl)amino)methyl)pyrrolidin-2-one;
(S)-5-((((6-(2-chloro-3-(3-chloro-2-(2-((R)-2-hydroxypropyl)-5-methoxy-1,2,3,4-tetrahydroisoquinolin-7-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-((S)-2-hydroxypropyl)-5-methoxy-1,2,3,4-tetrahydroisoquinolin-7-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-((R)-2-hydroxypropyl)-5-methoxy-1,2,3,4-tetrahydroisoquinolin-7-yl)pyridin-4-yl)phenyl)-2-methoxypyridin-3-yl)methyl)amino)methyl)pyrrolidin-2-one;
(S)-5-((((6-(2-chloro-3-(3-chloro-2-(2-(2-hydroxy-2-methylpropyl)-5-methoxy-1,2,3,4-tetrahydroisoquinolin-7-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-(2-hydroxy-2-methylpropyl)-5-methoxy-1,2,3,4-tetrahydroisoquinolin-7-yl)pyridin-4-yl)phenyl)-2-methoxypyridin-3-yl)methyl)amino)methyl)pyrrolidin-2-one;
(S)-5-((((6-(2-chloro-3-(3-chloro-2-(8-fluoro-2-(2-hydroxy-2-methylpropyl)-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(8-fluoro-2-(2-hydroxy-2-methylpropyl)-1,2,3,4-tetrahydroisoquinolin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(5-fluoro-2-(2-hydroxy-2-methylpropyl)-1,2,3,4-tetrahydroisoquinolin-7-yl)pyridin-4-yl)phenyl)-2-methoxypyridin-3-yl)methyl)amino)methyl)pyrrolidin-2-one;
(R)-5-((((6-(2-chloro-3-(3-chloro-2-(5-fluoro-2-(2-hydroxy-2-methylpropyl)-1,2,3,4-tetrahydroisoquinolin-7-yl)pyridin-4-yl)phenyl)-2-methoxypyridin-3-yl)methyl)amino)methyl)pyrrolidin-2-one;
(S)-6-(3-chloro-4-(2-chloro-3-(6-methoxy-5-((((5-oxopyrrolidin-2-yl)methyl)amino)methyl)pyridin-2-yl)phenyl)pyridin-2-yl)-2-((6-hydroxy-2-azaspiro[3.3]heptan-2-yl)methyl)-3-methylpyrrolo[2,1-f][1,2,4]triazin-4(3H)-one;
(R)-6-(3-chloro-4-(2-chloro-3-(6-methoxy-5-((((5-oxopyrrolidin-2-yl)methyl)amino)methyl)pyridin-2-yl)phenyl)pyridin-2-yl)-2-((6-hydroxy-2-azaspiro[3.3]heptan-2-yl)methyl)-3-methylpyrrolo[2,1-f][1,2,4]triazin-4(3H)-one;
(S)-5-((((6-(2-chloro-3-(3-chloro-2-(2-(3-hydroxy-3-methylbutyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-(3-hydroxy-3-methylbutyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(2-((S)-2-hydroxypropyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(2-((R)-2-hydroxypropyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-((S)-2-hydroxypropyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-((R)-2-hydroxypropyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(2-(2-(1-hydroxycyclobutyl)ethyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-(2-(1-hydroxycyclobutyl)ethyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(7-(((((S)-5-oxopyrrolidin-2-yl)methyl)amino)methyl)quinolin-3-yl)pyridin-4-yl)phenyl)-2-methoxypyridin-3-yl)methyl)amino)methyl)pyrrolidin-2-one;
(S)-5-((((6-(2-chloro-3-(3-chloro-2-(7-(((((R)-5-oxopyrrolidin-2-yl)methyl)amino)methyl)quinolin-3-yl)pyridin-4-yl)phenyl)-2-methoxypyridin-3-yl)methyl)amino)methyl)pyrrolidin-2-one;
(R)-5-((((6-(2-chloro-3-(3-chloro-2-(7-(((((S)-5-oxopyrrolidin-2-yl)methyl)amino)methyl)quinolin-3-yl)pyridin-4-yl)phenyl)-2-methoxypyridin-3-yl)methyl)amino)methyl)pyrrolidin-2-one;
(R)-5-((((6-(2-chloro-3-(3-chloro-2-(7-(((((R)-5-oxopyrrolidin-2-yl)methyl)amino)methyl)quinolin-3-yl)pyridin-4-yl)phenyl)-2-methoxypyridin-3-yl)methyl)amino)methyl)pyrrolidin-2-one;
(5S)-5-((((6-(2-chloro-3-(3-chloro-2-(2-(2-hydroxy-3-methylbutyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(SR)-5-((((6-(2-chloro-3-(3-chloro-2-(2-(2-hydroxy-3-methylbutyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(2-(2-hydroxyethyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-(2-hydroxyethyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(S)-5-((((6-(3-(2-(3-(((1-acetylazetidin-3-yl)amino)methyl)-1-methyl-1H-indol-6-yl)-3-chloropyridin-4-yl)-2-chlorophenyl)-2-methoxypyridin-3-yl)methyl)amino)methyl)pyrrolidin-2-one;
(R)-5-((((6-(3-(2-(3-(((1-acetylazetidin-3-yl)amino)methyl)-1-methyl-1H-indol-6-yl)-3-chloropyridin-4-yl)-2-chlorophenyl)-2-methoxypyridin-3-yl)methyl)amino)methyl)pyrrolidin-2-one;
(S)-5-((((6-(3-(2-(3-(((1-acetylpiperidin-4-yl)(methyl)amino)methyl)-1-methyl-1H-indol-6-yl)-3-chloropyridin-4-yl)-2-chlorophenyl)-2-methoxypyridin-3-yl)methyl)amino)methyl)pyrrolidin-2-one;
(R)-5-((((6-(3-(2-(3-(((1-acetylpiperidin-4-yl)(methyl)amino)methyl)-1-methyl-1H-indol-6-yl)-3-chloropyridin-4-yl)-2-chlorophenyl)-2-methoxypyridin-3-yl)methyl)amino)methyl)pyrrolidin-2-one;
(S)-5-(((4-(4-(3-(3-(((1-acetylpiperidin-4-yl)amino)methyl)-1-methyl-1H-pyrrolo[2,3-b]pyridin-6-yl)-2-chlorophenyl)-3-chloropyridin-2-yl)-2-methoxybenzyl)amino)methyl)pyrrolidin-2-one;
(R)-5-(((4-(4-(3-(3-(((1-acetylpiperidin-4-yl)amino)methyl)-1-methyl-1H-pyrrolo[2,3-b]pyridin-6-yl)-2-chlorophenyl)-3-chloropyridin-2-yl)-2-methoxybenzyl)amino)methyl)pyrrolidin-2-one;
(S)-5-(((4-(3-chloro-4-(2-chloro-3-(1-methyl-3-(((((S)-oxetan-2-yl)methyl)amino)methyl)-1H-pyrrolo[2,3-b]pyridin-6-yl)phenyl)pyridin-2-yl)-2-methoxybenzyl)amino)methyl)pyrrolidin-2-one;
(S)-5-(((4-(3-chloro-4-(2-chloro-3-(1-methyl-3-(((((R)-oxetan-2-yl)methyl)amino)methyl)-1H-pyrrolo[2,3-b]pyridin-6-yl)phenyl)pyridin-2-yl)-2-methoxybenzyl)amino)methyl)pyrrolidin-2-one;
(R)-5-(((4-(3-chloro-4-(2-chloro-3-(1-methyl-3-(((((S)-oxetan-2-yl)methyl)amino)methyl)-1H-pyrrolo[2,3-b]pyridin-6-yl)phenyl)pyridin-2-yl)-2-methoxybenzyl)amino)methyl)pyrrolidin-2-one;
(R)-5-(((4-(3-chloro-4-(2-chloro-3-(1-methyl-3-(((((R)-oxetan-2-yl)methyl)amino)methyl)-1H-pyrrolo[2,3-b]pyridin-6-yl)phenyl)pyridin-2-yl)-2-methoxybenzyl)amino)methyl)pyrrolidin-2-one;
(S)-5-(((4-(3-chloro-4-(2-chloro-3-(1-methyl-3-(((tetrahydro-2H-pyran-4-yl)amino)methyl)-1H-pyrrolo[2,3-b]pyridin-6-yl)phenyl)pyridin-2-yl)-2-methoxybenzyl)amino)methyl)pyrrolidin-2-one;
(R)-5-(((4-(3-chloro-4-(2-chloro-3-(1-methyl-3-(((tetrahydro-2H-pyran-4-yl)amino)methyl)-1H-pyrrolo[2,3-b]pyridin-6-yl)phenyl)pyridin-2-yl)-2-methoxybenzyl)amino)methyl)pyrrolidin-2-one;
(S)-5-(((4-(3-chloro-4-(2-chloro-3-(3-(((1,1-dioxidotetrahydro-2H-thiopyran-4-yl)amino)methyl)-1-methyl-1H-pyrrolo[2,3-b]pyridin-6-yl)phenyl)pyridin-2-yl)-2-methoxybenzyl)amino)methyl)pyrrolidin-2-one;
(R)-5-(((4-(3-chloro-4-(2-chloro-3-(3-(((1,1-dioxidotetrahydro-2H-thiopyran-4-yl)amino)methyl)-1-methyl-1H-pyrrolo[2,3-b]pyridin-6-yl)phenyl)pyridin-2-yl)-2-methoxybenzyl)amino)methyl)pyrrolidin-2-one;
(S)-5-((((6-(2-chloro-3-(3-chloro-2-(8-fluoro-2-(((S)-5-oxopyrrolidin-2-yl)methyl)-1,2,3,4-tetrahydroisoquinolin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(8-fluoro-2-(((R)-5-oxopyrrolidin-2-yl)methyl)-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(8-fluoro-2-(((S)-5-oxopyrrolidin-2-yl)methyl)-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(8-fluoro-2-(((R)-5-oxopyrrolidin-2-yl)methyl)-1,2,3,4-tetrahydroisoquinolin-6-yl)pyridin-4-yl)phenyl)-2-methoxypyridin-3-yl)methyl)amino)methyl)pyrrolidin-2-one;
(S)-5-((((6-(3-(2-(2-((1-acetylpiperidin-4-yl)methyl)-8-methoxy-1,2,3,4-tetrahydroisoquinolin-6-yl)-3-chloropyridin-4-yl)-2-chlorophenyl)-2-methoxypyridin-3-yl)methyl)amino)methyl)pyrrolidin-2-one;
(R)-5-((((6-(3-(2-(2-((1-acetylpiperidin-4-yl)methyl)-8-methoxy-1,2,3,4-tetrahydroisoquinolin-6-yl)-3-chloropyridin-4-yl)-2-chlorophenyl)-2-methoxypyridin-3-yl)methyl)amino)methyl)pyrrolidin-2-one;
(S)-5-((((6-(2-chloro-3-(3-chloro-2-(2-(3-fluoropropyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-(3-fluoropropyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(8-methoxy-2-(2-(piperidin-4-yl)ethyl)-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(8-methoxy-2-(2-(piperidin-4-yl)ethyl)-1,2,3,4-tetrahydroisoquinolin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(8-fluoro-2-(3-fluoropropyl)-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(8-fluoro-2-(3-fluoropropyl)-1,2,3,4-tetrahydroisoquinolin-6-yl)pyridin-4-yl)phenyl)-2-methoxypyridin-3-yl)methyl)amino)methyl)pyrrolidin-2-one;
5-(3-chloro-4-(2-chloro-3-(6-methoxy-5-(((((S)-5-oxopyrrolidin-2-yl)methyl)amino)methyl)pyridin-2-yl)phenyl)pyridin-2-yl)-2-(2-((((S)-5-oxopyrrolidin-2-yl)methyl)amino)ethyl)isoindolin-1-one;
5-(3-chloro-4-(2-chloro-3-(6-methoxy-5-(((((S)-5-oxopyrrolidin-2-yl)methyl)amino)methyl)pyridin-2-yl)phenyl)pyridin-2-yl)-2-(2-((((R)-5-oxopyrrolidin-2-yl)methyl)amino)ethyl)isoindolin-1-one;
5-(3-chloro-4-(2-chloro-3-(6-methoxy-5-(((((R)-5-oxopyrrolidin-2-yl)methyl)amino)methyl)pyridin-2-yl)phenyl)pyridin-2-yl)-2-(2-((((S)-5-oxopyrrolidin-2-yl)methyl)amino)ethyl)isoindolin-1-one;
5-(3-chloro-4-(2-chloro-3-(6-methoxy-5-(((((R)-5-oxopyrrolidin-2-yl)methyl)amino)methyl)pyridin-2-yl)phenyl)pyridin-2-yl)-2-(2-((((R)-5-oxopyrrolidin-2-yl)methyl)amino)ethyl)isoindolin-1-one;
(S)-5-((((6-(2-chloro-3-(3-chloro-2-(8-chloro-2-(((S)-5-oxopyrrolidin-2-yl)methyl)-1,2,3,4-tetrahydroisoquinolin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(8-chloro-2-(((R)-5-oxopyrrolidin-2-yl)methyl)-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(8-chloro-2-(((S)-5-oxopyrrolidin-2-yl)methyl)-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(8-chloro-2-(((R)-5-oxopyrrolidin-2-yl)methyl)-1,2,3,4-tetrahydroisoquinolin-6-yl)pyridin-4-yl)phenyl)-2-methoxypyridin-3-yl)methyl)amino)methyl)pyrrolidin-2-one;
isopropyl (4-(3-chloro-4-(2-chloro-3-(6-methoxy-5-(((((S)-5-oxopyrrolidin-2-yl)methyl)amino)methyl)pyridin-2-yl)phenyl)pyridin-2-yl)-2-methoxybenzyl)-L-prolinate;
isopropyl (4-(3-chloro-4-(2-chloro-3-(6-methoxy-5-(((((S)-5-oxopyrrolidin-2-yl)methyl)amino)methyl)pyridin-2-yl)phenyl)pyridin-2-yl)-2-methoxybenzyl)-D-prolinate;
isopropyl (4-(3-chloro-4-(2-chloro-3-(6-methoxy-5-(((((R)-5-oxopyrrolidin-2-yl)methyl)amino)methyl)pyridin-2-yl)phenyl)pyridin-2-yl)-2-methoxybenzyl)-L-prolinate;
isopropyl (4-(3-chloro-4-(2-chloro-3-(6-methoxy-5-(((((R)-5-oxopyrrolidin-2-yl)methyl)amino)methyl)pyridin-2-yl)phenyl)pyridin-2-yl)-2-methoxybenzyl)-D-prolinate;
(S)-5-((((6-(2-chloro-3-(3-chloro-2-(8-chloro-2-((S)-2-hydroxypropyl)-1,2,3,4-tetrahydroisoquinolin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(8-chloro-2-((R)-2-hydroxypropyl)-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(8-chloro-2-((S)-2-hydroxypropyl)-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(8-chloro-2-((R)-2-hydroxypropyl)-1,2,3,4-tetrahydroisoquinolin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(4-(((2-hydroxyethyl)amino)methyl)-3-methoxyphenyl)pyridin-4-yl)phenyl)-2-methoxypyridin-3-yl)methyl)amino)methyl)pyrrolidin-2-one;
(R)-5-((((6-(2-chloro-3-(3-chloro-2-(4-(((2-hydroxyethyl)amino)methyl)-3-methoxyphenyl)pyridin-4-yl)phenyl)-2-methoxypyridin-3-yl)methyl)amino)methyl)pyrrolidin-2-one;
(S)-5-((((4-chloro-6-(2-chloro-3-(3-chloro-2-(4-((((S)-2-hydroxypropyl)(methyl)amino)methyl)-3-methoxyphenyl)pyridin-4-yl)phenyl)-2-methoxypyridin-3-yl)methyl)amino)methyl)pyrrolidin-2-one;
(S)-5-((((4-chloro-6-(2-chloro-3-(3-chloro-2-(4-((((R)-2-hydroxypropyl)(methyl)amino)methyl)-3-methoxyphenyl)pyridin-4-yl)phenyl)-2-methoxypyridin-3-yl)methyl)amino)methyl)pyrrolidin-2-one;
(R)-5-((((4-chloro-6-(2-chloro-3-(3-chloro-2-(4-((((S)-2-hydroxypropyl)(methyl)amino)methyl)-3-methoxyphenyl)pyridin-4-yl)phenyl)-2-methoxypyridin-3-yl)methyl)amino)methyl)pyrrolidin-2-one;
(R)-5-((((4-chloro-6-(2-chloro-3-(3-chloro-2-(4-((((R)-2-hydroxypropyl)(methyl)amino)methyl)-3-methoxyphenyl)pyridin-4-yl)phenyl)-2-methoxypyridin-3-yl)methyl)amino)methyl)pyrrolidin-2-one;
(S)-5-((((6-(2-chloro-3-(3-chloro-2-(8-chloro-2-(3-fluoropropyl)-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(8-chloro-2-(3-fluoropropyl)-1,2,3,4-tetrahydroisoquinolin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(3-(((4,4-difluorocyclohexyl)amino)methyl)-1-methyl-1H-indol-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(3-(((4,4-difluorocyclohexyl)amino)methyl)-1-methyl-1H-indol-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(2-(2-(1-hydroxycyclopropyl)ethyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-(2-(1-hydroxycyclopropyl)ethyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(4-((((S)-2-hydroxypropyl)(methyl)amino)methyl)-3-methoxyphenyl)pyridin-4-yl)phenyl)-2-methoxypyridin-3-yl)methyl)amino)methyl)pyrrolidin-2-one;
(S)-5-((((6-(2-chloro-3-(3-chloro-2-(4-((((R)-2-hydroxypropyl)(methyl)amino)methyl)-3-methoxyphenyl)pyridin-4-yl)phenyl)-2-methoxypyridin-3-yl)methyl)amino)methyl)pyrrolidin-2-one;
(R)-5-((((6-(2-chloro-3-(3-chloro-2-(4-((((S)-2-hydroxypropyl)(methyl)amino)methyl)-3-methoxyphenyl)pyridin-4-yl)phenyl)-2-methoxypyridin-3-yl)methyl)amino)methyl)pyrrolidin-2-one;
(R)-5-((((6-(2-chloro-3-(3-chloro-2-(4-((((R)-2-hydroxypropyl)(methyl)amino)methyl)-3-methoxyphenyl)pyridin-4-yl)phenyl)-2-methoxypyridin-3-yl)methyl)amino)methyl)pyrrolidin-2-one;
isopropyl (S)-1-(4-(3-chloro-4-(2-chloro-3-(6-methoxy-5-(((((S)-5-oxopyrrolidin-2-yl)methyl)amino)methyl)pyridin-2-yl)phenyl)pyridin-2-yl)-2-methoxybenzyl)pyrrolidine-3-carboxylate;
isopropyl (S)-1-(4-(3-chloro-4-(2-chloro-3-(6-methoxy-5-(((((R)-5-oxopyrrolidin-2-yl)methyl)amino)methyl)pyridin-2-yl)phenyl)pyridin-2-yl)-2-methoxybenzyl)pyrrolidine-3-carboxylate;
isopropyl (R)-1-(4-(3-chloro-4-(2-chloro-3-(6-methoxy-5-(((((S)-5-oxopyrrolidin-2-yl)methyl)amino)methyl)pyridin-2-yl)phenyl)pyridin-2-yl)-2-methoxybenzyl)pyrrolidine-3-carboxylate;
isopropyl (R)-1-(4-(3-chloro-4-(2-chloro-3-(6-methoxy-5-(((((R)-5-oxopyrrolidin-2-yl)methyl)amino)methyl)pyridin-2-yl)phenyl)pyridin-2-yl)-2-methoxybenzyl)pyrrolidine-3-carboxylate;
(S)-1-(4-(3-chloro-4-(2-chloro-3-(6-methoxy-5-(((((S)-5-oxopyrrolidin-2-yl)methyl)amino)methyl)pyridin-2-yl)phenyl)pyridin-2-yl)-2-methoxybenzyl)pyrrolidine-3-carboxylic acid;
(S)-1-(4-(3-chloro-4-(2-chloro-3-(6-methoxy-5-(((((R)-5-oxopyrrolidin-2-yl)methyl)amino)methyl)pyridin-2-yl)phenyl)pyridin-2-yl)-2-methoxybenzyl)pyrrolidine-3-carboxylic acid;
(R)-1-(4-(3-chloro-4-(2-chloro-3-(6-methoxy-5-(((((S)-5-oxopyrrolidin-2-yl)methyl)amino)methyl)pyridin-2-yl)phenyl)pyridin-2-yl)-2-methoxybenzyl)pyrrolidine-3-carboxylic acid;
(R)-1-(4-(3-chloro-4-(2-chloro-3-(6-methoxy-5-(((((R)-5-oxopyrrolidin-2-yl)methyl)amino)methyl)pyridin-2-yl)phenyl)pyridin-2-yl)-2-methoxybenzyl)pyrrolidine-3-carboxylic acid;
(4-(3-chloro-4-(2-chloro-3-(6-methoxy-5-(((((S)-5-oxopyrrolidin-2-yl)methyl)amino)methyl)pyridin-2-yl)phenyl)pyridin-2-yl)-2-methoxybenzyl)-L-proline;
(4-(3-chloro-4-(2-chloro-3-(6-methoxy-5-(((((S)-5-oxopyrrolidin-2-yl)methyl)amino)methyl)pyridin-2-yl)phenyl)pyridin-2-yl)-2-methoxybenzyl)-D-proline;
(4-(3-chloro-4-(2-chloro-3-(6-methoxy-5-(((((R)-5-oxopyrrolidin-2-yl)methyl)amino)methyl)pyridin-2-yl)phenyl)pyridin-2-yl)-2-methoxybenzyl)-L-proline;
(4-(3-chloro-4-(2-chloro-3-(6-methoxy-5-(((((R)-5-oxopyrrolidin-2-yl)methyl)amino)methyl)pyridin-2-yl)phenyl)pyridin-2-yl)-2-methoxybenzyl)-D-proline;
(S)-5-((((6-(2-chloro-3-(3-chloro-2-(3-fluoro-4-((((1r,3r)-3-hydroxy-3-methylcyclobutyl)amino)methyl)-5-methoxyphenyl)pyridin-4-yl)phenyl)-2-methoxypyridin-3-yl)methyl)amino)methyl)pyrrolidin-2-one;
(R)-5-((((6-(2-chloro-3-(3-chloro-2-(3-fluoro-4-((((1r,3r)-3-hydroxy-3-methylcyclobutyl)amino)methyl)-5-methoxyphenyl)pyridin-4-yl)phenyl)-2-methoxypyridin-3-yl)methyl)amino)methyl)pyrrolidin-2-one;
(R)-5-((((6-(2-chloro-3-(3-chloro-2-(3-fluoro-4-((((1r,3s)-3-hydroxy-3-methylcyclobutyl)amino)methyl)-5-methoxyphenyl)pyridin-4-yl)phenyl)-2-methoxypyridin-3-yl)methyl)amino)methyl)pyrrolidin-2-one;
(R)-5-((((6-(2-chloro-3-(3-chloro-2-(3-fluoro-4-((((1s,3r)-3-hydroxy-3-methylcyclobutyl)amino)methyl)-5-methoxyphenyl)pyridin-4-yl)phenyl)-2-methoxypyridin-3-yl)methyl)amino)methyl)pyrrolidin-2-one;
(S)-5-((((6-(2-chloro-3-(3-chloro-2-(3-fluoro-4-((((1s,3r)-3-hydroxy-3-methylcyclobutyl)amino)methyl)-5-methoxyphenyl)pyridin-4-yl)phenyl)-2-methoxypyridin-3-yl)methyl)amino)methyl)pyrrolidin-2-one;
(S)-5-((((6-(2-chloro-3-(3-chloro-2-(3-fluoro-4-((((1r,3s)-3-hydroxy-3-methylcyclobutyl)amino)methyl)-5-methoxyphenyl)pyridin-4-yl)phenyl)-2-methoxypyridin-3-yl)methyl)amino)methyl)pyrrolidin-2-one;
(R)-5-((((6-(2-chloro-3-(3-chloro-2-(3-fluoro-4-((((1s,3s)-3-hydroxy-3-methylcyclobutyl)amino)methyl)-5-methoxyphenyl)pyridin-4-yl)phenyl)-2-methoxypyridin-3-yl)methyl)amino)methyl)pyrrolidin-2-one;
(S)-5-((((6-(2-chloro-3-(3-chloro-2-(3-fluoro-4-((((1s,3s)-3-hydroxy-3-methylcyclobutyl)amino)methyl)-5-methoxyphenyl)pyridin-4-yl)phenyl)-2-methoxypyridin-3-yl)methyl)amino)methyl)pyrrolidin-2-one;
(S)-5-((((6-(2-chloro-3-(3-chloro-2-(3-fluoro-4-((((S)-2-hydroxypropyl)amino)methyl)-5-methoxyphenyl)pyridin-4-yl)phenyl)-2-methoxypyridin-3-yl)methyl)amino)methyl)pyrrolidin-2-one;
(R)-5-((((6-(2-chloro-3-(3-chloro-2-(3-fluoro-4-((((S)-2-hydroxypropyl)amino)methyl)-5-methoxyphenyl)pyridin-4-yl)phenyl)-2-methoxypyridin-3-yl)methyl)amino)methyl)pyrrolidin-2-one;
(R)-5-((((6-(2-chloro-3-(3-chloro-2-(3-fluoro-4-((((R)-2-hydroxypropyl)amino)methyl)-5-methoxyphenyl)pyridin-4-yl)phenyl)-2-methoxypyridin-3-yl)methyl)amino)methyl)pyrrolidin-2-one;
(S)-5-((((6-(2-chloro-3-(3-chloro-2-(3-fluoro-4-((((R)-2-hydroxypropyl)amino)methyl)-5-methoxyphenyl)pyridin-4-yl)phenyl)-2-methoxypyridin-3-yl)methyl)amino)methyl)pyrrolidin-2-one;
(S)-5-((((6-(2-chloro-3-(3-chloro-2-(3-fluoro-4-((isopropylamino)methyl)-5-methoxyphenyl)pyridin-4-yl)phenyl)-2-methoxypyridin-3-yl)methyl)amino)methyl)pyrrolidin-2-one;
(R)-5-((((6-(2-chloro-3-(3-chloro-2-(3-fluoro-4-((isopropylamino)methyl)-5-methoxyphenyl)pyridin-4-yl)phenyl)-2-methoxypyridin-3-yl)methyl)amino)methyl)pyrrolidin-2-one;
(S)-5-((((6-(2-chloro-3-(3-chloro-2-(3-fluoro-4-((isobutylamino)methyl)-5-methoxyphenyl)pyridin-4-yl)phenyl)-2-methoxypyridin-3-yl)methyl)amino)methyl)pyrrolidin-2-one;
(R)-5-((((6-(2-chloro-3-(3-chloro-2-(3-fluoro-4-((isobutylamino)methyl)-5-methoxyphenyl)pyridin-4-yl)phenyl)-2-methoxypyridin-3-yl)methyl)amino)methyl)pyrrolidin-2-one;
2-(((6-(2-chloro-3-(3-chloro-2-(2-(3-fluoropropyl)-8-methoxy-1,2,3,4-tetrahydroisoquinolin-6-yl)pyridin-4-yl)phenyl)-2-methoxypyridin-3-yl)methyl)amino)ethan-1-ol;
2-(((6-(2-chloro-3-(3-chloro-2-(8-methoxy-2-(2-(piperidin-4-yl)ethyl)-1,2,3,4-tetrahydroisoquinolin-6-yl)pyridin-4-yl)phenyl)-2-methoxypyridin-3-yl)methyl)amino)ethan-1-ol;
(S)-5-((((4-chloro-6-(2-chloro-3-(3-chloro-2-(2-((S)-2-hydroxypropyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(S)-5-((((4-chloro-6-(2-chloro-3-(3-chloro-2-(2-((R)-2-hydroxypropyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((4-chloro-6-(2-chloro-3-(3-chloro-2-(2-((S)-2-hydroxypropyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((4-chloro-6-(2-chloro-3-(3-chloro-2-(2-((R)-2-hydroxypropyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
6-(3-chloro-4-(2-chloro-3-(4-chloro-6-methoxy-5-(((((S)-5-oxopyrrolidin-2-yl)methyl)amino)methyl)pyridin-2-yl)phenyl)pyridin-2-yl)-2-((((1r,3r)-3-hydroxy-3-methylcyclobutyl)amino)methyl)-3-methylpyrrolo[2,1-f][1,2,4]triazin-4(3H)-one;
6-(3-chloro-4-(2-chloro-3-(4-chloro-6-methoxy-5-(((((R)-5-oxopyrrolidin-2-yl)methyl)amino)methyl)pyridin-2-yl)phenyl)pyridin-2-yl)-2-((((1r,3s)-3-hydroxy-3-methylcyclobutyl)amino)methyl)-3-methylpyrrolo[2,1-f][1,2,4]triazin-4(3H)-one;
6-(3-chloro-4-(2-chloro-3-(4-chloro-6-methoxy-5-(((((R)-5-oxopyrrolidin-2-yl)methyl)amino)methyl)pyridin-2-yl)phenyl)pyridin-2-yl)-2-((((1s,3r)-3-hydroxy-3-methylcyclobutyl)amino)methyl)-3-methylpyrrolo[2,1-f][1,2,4]triazin-4(3H)-one;
6-(3-chloro-4-(2-chloro-3-(4-chloro-6-methoxy-5-(((((S)-5-oxopyrrolidin-2-yl)methyl)amino)methyl)pyridin-2-yl)phenyl)pyridin-2-yl)-2-((((1s,3r)-3-hydroxy-3-methylcyclobutyl)amino)methyl)-3-methylpyrrolo[2,1-f][1,2,4]triazin-4(3H)-one;
6-(3-chloro-4-(2-chloro-3-(4-chloro-6-methoxy-5-(((((S)-5-oxopyrrolidin-2-yl)methyl)amino)methyl)pyridin-2-yl)phenyl)pyridin-2-yl)-2-((((1r,3s)-3-hydroxy-3-methylcyclobutyl)amino)methyl)-3-methylpyrrolo[2,1-f][1,2,4]triazin-4(3H)-one;
6-(3-chloro-4-(2-chloro-3-(4-chloro-6-methoxy-5-(((((R)-5-oxopyrrolidin-2-yl)methyl)amino)methyl)pyridin-2-yl)phenyl)pyridin-2-yl)-2-((((1s,3s)-3-hydroxy-3-methylcyclobutyl)amino)methyl)-3-methylpyrrolo[2,1-f][1,2,4]triazin-4(3H)-one;
6-(3-chloro-4-(2-chloro-3-(4-chloro-6-methoxy-5-(((((R)-5-oxopyrrolidin-2-yl)methyl)amino)methyl)pyridin-2-yl)phenyl)pyridin-2-yl)-2-((((1r,3r)-3-hydroxy-3-methylcyclobutyl)amino)methyl)-3-methylpyrrolo[2,1-f][1,2,4]triazin-4(3H)-one;
6-(3-chloro-4-(2-chloro-3-(4-chloro-6-methoxy-5-(((((S)-5-oxopyrrolidin-2-yl)methyl)amino)methyl)pyridin-2-yl)phenyl)pyridin-2-yl)-2-((((1s,3s)-3-hydroxy-3-methylcyclobutyl)amino)methyl)-3-methylpyrrolo[2,1-f][1,2,4]triazin-4(3H)-one;
(S)-2-(4-(3-chloro-4-(2-chloro-3-(6-methoxy-5-((((5-oxopyrrolidin-2-yl)methyl)amino)methyl)pyridin-2-yl)phenyl)pyridin-2-yl)-2-methoxybenzyl)-2,6-diazaspiro[3.4]octan-7-one;
(R)-2-(4-(3-chloro-4-(2-chloro-3-(6-methoxy-5-((((5-oxopyrrolidin-2-yl)methyl)amino)methyl)pyridin-2-yl)phenyl)pyridin-2-yl)-2-methoxybenzyl)-2,6-diazaspiro[3.4]octan-7-one;
2-(4-(3-chloro-4-(2-chloro-3-(6-methoxy-5-((7-oxo-2,6-diazaspiro[3.4]octan-2-yl)methyl)pyridin-2-yl)phenyl)pyridin-2-yl)-2-methoxybenzyl)-2,6-diazaspiro[3.4]octan-7-one;
1-(6-((6-(3-(2-(4-((6-acetyl-2,6-diazaspiro[3.3]heptan-2-yl)methyl)-3-methoxyphenyl)-3-chloropyridin-4-yl)-2-chlorophenyl)-2-methoxypyridin-3-yl)methyl)-2,6-diazaspiro[3.3]heptan-2-yl)ethan-1-one;
6-(3-chloro-4-(2-chloro-3-(6-methoxy-5-(((((S)-5-oxopyrrolidin-2-yl)methyl)amino)methyl)pyridin-2-yl)phenyl)pyridin-2-yl)-2-((((S)-2-hydroxypropyl)amino)methyl)-3-methylpyrrolo[2,1-f][1,2,4]triazin-4(3H)-one;
6-(3-chloro-4-(2-chloro-3-(6-methoxy-5-(((((S)-5-oxopyrrolidin-2-yl)methyl)amino)methyl)pyridin-2-yl)phenyl)pyridin-2-yl)-2-((((R)-2-hydroxypropyl)amino)methyl)-3-methylpyrrolo[2,1-f][1,2,4]triazin-4(3H)-one;
6-(3-chloro-4-(2-chloro-3-(6-methoxy-5-(((((R)-5-oxopyrrolidin-2-yl)methyl)amino)methyl)pyridin-2-yl)phenyl)pyridin-2-yl)-2-((((S)-2-hydroxypropyl)amino)methyl)-3-methylpyrrolo[2,1-f][1,2,4]triazin-4(3H)-one;
6-(3-chloro-4-(2-chloro-3-(6-methoxy-5-(((((R)-5-oxopyrrolidin-2-yl)methyl)amino)methyl)pyridin-2-yl)phenyl)pyridin-2-yl)-2-((((R)-2-hydroxypropyl)amino)methyl)-3-methylpyrrolo[2,1-f][1,2,4]triazin-4(3H)-one;
(S)-5-(((4-(4-(3-(3-(((1-acetylpiperidin-4-yl)(methyl)amino)methyl)-1-methyl-1H-pyrrolo[2,3-b]pyridin-6-yl)-2-chlorophenyl)-3-chloropyridin-2-yl)-2-methoxybenzyl)amino)methyl)pyrrolidin-2-one;
(R)-5-(((4-(4-(3-(3-(((1-acetylpiperidin-4-yl)(methyl)amino)methyl)-1-methyl-1H-pyrrolo[2,3-b]pyridin-6-yl)-2-chlorophenyl)-3-chloropyridin-2-yl)-2-methoxybenzyl)amino)methyl)pyrrolidin-2-one;
(S)-5-(((4-(3-chloro-4-(2-chloro-3-(1-methyl-3-(((((S)-tetrahydrofuran-2-yl)methyl)amino)methyl)-1H-pyrrolo[2,3-b]pyridin-6-yl)phenyl)pyridin-2-yl)-2-methoxybenzyl)amino)methyl)pyrrolidin-2-one;
(S)-5-(((4-(3-chloro-4-(2-chloro-3-(1-methyl-3-(((((R)-tetrahydrofuran-2-yl)methyl)amino)methyl)-1H-pyrrolo[2,3-b]pyridin-6-yl)phenyl)pyridin-2-yl)-2-methoxybenzyl)amino)methyl)pyrrolidin-2-one;
(R)-5-(((4-(3-chloro-4-(2-chloro-3-(1-methyl-3-(((((S)-tetrahydrofuran-2-yl)methyl)amino)methyl)-1H-pyrrolo[2,3-b]pyridin-6-yl)phenyl)pyridin-2-yl)-2-methoxybenzyl)amino)methyl)pyrrolidin-2-one;
(R)-5-(((4-(3-chloro-4-(2-chloro-3-(1-methyl-3-(((((R)-tetrahydrofuran-2-yl)methyl)amino)methyl)-1H-pyrrolo[2,3-b]pyridin-6-yl)phenyl)pyridin-2-yl)-2-methoxybenzyl)amino)methyl)pyrrolidin-2-one;
(S)-5-(((4-(3-chloro-4-(2-chloro-3-(1-methyl-3-((methylamino)methyl)-1H-pyrrolo[2,3-b]pyridin-6-yl)phenyl)pyridin-2-yl)-2-methoxybenzyl)amino)methyl)pyrrolidin-2-one;
(R)-5-(((4-(3-chloro-4-(2-chloro-3-(1-methyl-3-((methylamino)methyl)-1H-pyrrolo[2,3-b]pyridin-6-yl)phenyl)pyridin-2-yl)-2-methoxybenzyl)amino)methyl)pyrrolidin-2-one;
(S)-5-(((4-(4-(3-(3-(((1-acetylazetidin-3-yl)amino)methyl)-1-methyl-1H-pyrrolo[2,3-b]pyridin-6-yl)-2-chlorophenyl)-3-chloropyridin-2-yl)-2-methoxybenzyl)amino)methyl)pyrrolidin-2-one;
(R)-5-(((4-(4-(3-(3-(((1-acetylazetidin-3-yl)amino)methyl)-1-methyl-1H-pyrrolo[2,3-b]pyridin-6-yl)-2-chlorophenyl)-3-chloropyridin-2-yl)-2-methoxybenzyl)amino)methyl)pyrrolidin-2-one;
(S)-5-(((4-(3-chloro-4-(2-chloro-3-(3-(((4,4-difluorocyclohexyl)amino)methyl)-1-methyl-**1H-pyrrolo[2,3-b]pyridin-6-yl)phenyl)pyridin-2-yl)-2-**methoxybenzyl)amino)methyl)pyrrolidin-2-one;
(R)-5-(((4-(3-chloro-4-(2-chloro-3-(3-(((4,4-difluorocyclohexyl)amino)methyl)-1-methyl-**1H-pyrrolo[2,3-b]pyridin-6-yl)phenyl)pyridin-2-yl)-2-**methoxybenzyl)amino)methyl)pyrrolidin-2-one;
(S)-5-(((4-(3-chloro-4-(2-chloro-3-(3-(((2-hydroxyethyl)amino)methyl)-1-methyl-1H-pyrrolo[2,3-b]pyridin-6-yl)phenyl)pyridin-2-yl)-2-methoxybenzyl)amino)methyl)pyrrolidin-2-one;
(R)-5-(((4-(3-chloro-4-(2-chloro-3-(3-(((2-hydroxyethyl)amino)methyl)-1-methyl-1H-pyrrolo[2,3-b]pyridin-6-yl)phenyl)pyridin-2-yl)-2-methoxybenzyl)amino)methyl)pyrrolidin-2-one;
(S)-5-(((4-(3-chloro-4-(2-chloro-3-(3-((((S)-2-hydroxypropyl)amino)methyl)-1-methyl-**1H-pyrrolo[2,3-b]pyridin-6-yl)phenyl)pyridin-2-yl)-2-**methoxybenzyl)amino)methyl)pyrrolidin-2-one;
(S)-5-(((4-(3-chloro-4-(2-chloro-3-(3-((((R)-2-hydroxypropyl)amino)methyl)-1-methyl-**1H-pyrrolo[2,3-b]pyridin-6-yl)phenyl)pyridin-2-yl)-2-**methoxybenzyl)amino)methyl)pyrrolidin-2-one;
(R)-5-(((4-(3-chloro-4-(2-chloro-3-(3-((((S)-2-hydroxypropyl)amino)methyl)-1-methyl-**1H-pyrrolo[2,3-b]pyridin-6-yl)phenyl)pyridin-2-yl)-2-**methoxybenzyl)amino)methyl)pyrrolidin-2-one;
(R)-5-(((4-(3-chloro-4-(2-chloro-3-(3-((((R)-2-hydroxypropyl)amino)methyl)-1-methyl-**1H-pyrrolo[2,3-b]pyridin-6-yl)phenyl)pyridin-2-yl)-2-**methoxybenzyl)amino)methyl)pyrrolidin-2-one;
(S)-5-(((4-(3-chloro-4-(2-chloro-3-(3-(((2-hydroxy-2-methylpropyl)amino)methyl)-1-methyl-1H-pyrrolo[2,3-b]pyridin-6-yl)phenyl)pyridin-2-yl)-2-methoxybenzyl)amino)methyl)pyrrolidin-2-one;
(R)-5-(((4-(3-chloro-4-(2-chloro-3-(3-(((2-hydroxy-2-methylpropyl)amino)methyl)-1-methyl-1H-pyrrolo[2,3-b]pyridin-6-yl)phenyl)pyridin-2-yl)-2-methoxybenzyl)amino)methyl)pyrrolidin-2-one;
(S)-5-(((4-(3-chloro-4-(2-chloro-3-(3-(((2-methoxyethyl)amino)methyl)-1-methyl-1H-pyrrolo[2,3-b]pyridin-6-yl)phenyl)pyridin-2-yl)-2-methoxybenzyl)amino)methyl)pyrrolidin-2-one;
(R)-5-(((4-(3-chloro-4-(2-chloro-3-(3-(((2-methoxyethyl)amino)methyl)-1-methyl-1H-pyrrolo[2,3-b]pyridin-6-yl)phenyl)pyridin-2-yl)-2-methoxybenzyl)amino)methyl)pyrrolidin-2-one;
(S)-5-(((4-(3-chloro-4-(2-chloro-3-(3-(((2-hydroxyethyl)(methyl)amino)methyl)-1-methyl-1H-pyrrolo[2,3-b]pyridin-6-yl)phenyl)pyridin-2-yl)-2-methoxybenzyl)amino)methyl)pyrrolidin-2-one;
(R)-5-(((4-(3-chloro-4-(2-chloro-3-(3-(((2-hydroxyethyl)(methyl)amino)methyl)-1-methyl-1H-pyrrolo[2,3-b]pyridin-6-yl)phenyl)pyridin-2-yl)-2-methoxybenzyl)amino)methyl)pyrrolidin-2-one;
(S)-6-(3-chloro-4-(2-chloro-3-(6-methoxy-5-((((5-oxopyrrolidin-2-yl)methyl)amino)methyl)pyridin-2-yl)phenyl)pyridin-2-yl)-2-(((3-hydroxy-3-methylcyclobutyl)amino)methyl)-3-methylpyrrolo[2,1-f][1,2,4]triazin-4(3H)-one;
(R)-6-(3-chloro-4-(2-chloro-3-(6-methoxy-5-((((5-oxopyrrolidin-2-yl)methyl)amino)methyl)pyridin-2-yl)phenyl)pyridin-2-yl)-2-(((3-hydroxy-3-methylcyclobutyl)amino)methyl)-3-methylpyrrolo[2,1-f][1,2,4]triazin-4(3H)-one;
6-(3-chloro-4-(2-chloro-3-(6-methoxy-5-(((((S)-5-oxopyrrolidin-2-yl)methyl)amino)methyl)pyridin-2-yl)phenyl)pyridin-2-yl)-3-methyl-2-(((((S)-5-oxopyrrolidin-2-yl)methyl)amino)methyl)pyrrolo[2,1-f][1,2,4]triazin-4(3H)-one;
6-(3-chloro-4-(2-chloro-3-(6-methoxy-5-(((((S)-5-oxopyrrolidin-2-yl)methyl)amino)methyl)pyridin-2-yl)phenyl)pyridin-2-yl)-3-methyl-2-(((((R)-5-oxopyrrolidin-2-yl)methyl)amino)methyl)pyrrolo[2,1-f][1,2,4]triazin-4(3H)-one;
6-(3-chloro-4-(2-chloro-3-(6-methoxy-5-(((((R)-5-oxopyrrolidin-2-yl)methyl)amino)methyl)pyridin-2-yl)phenyl)pyridin-2-yl)-3-methyl-2-(((((S)-5-oxopyrrolidin-2-yl)methyl)amino)methyl)pyrrolo[2,1-f][1,2,4]triazin-4(3H)-one;
6-(3-chloro-4-(2-chloro-3-(6-methoxy-5-(((((R)-5-oxopyrrolidin-2-yl)methyl)amino)methyl)pyridin-2-yl)phenyl)pyridin-2-yl)-3-methyl-2-(((((R)-5-oxopyrrolidin-2-yl)methyl)amino)methyl)pyrrolo[2,1-f][1,2,4]triazin-4(3H)-one;
(S)-5-((((4-chloro-6-(2-chloro-3-(3-chloro-2-(2-((R)-2-hydroxypropyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(S)-5-((((4-chloro-6-(2-chloro-3-(3-chloro-2-(2-((S)-2-hydroxypropyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((4-chloro-6-(2-chloro-3-(3-chloro-2-(2-((R)-2-hydroxypropyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((4-chloro-6-(2-chloro-3-(3-chloro-2-(2-((S)-2-hydroxypropyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(S)-(4-(3-chloro-4-(2-chloro-3-(4-chloro-6-methoxy-5-((((5-oxopyrrolidin-2-yl)methyl)amino)methyl)pyridin-2-yl)phenyl)pyridin-2-yl)-2-methoxybenzyl)glycine;
(R)-(4-(3-chloro-4-(2-chloro-3-(4-chloro-6-methoxy-5-((((5-oxopyrrolidin-2-yl)methyl)amino)methyl)pyridin-2-yl)phenyl)pyridin-2-yl)-2-methoxybenzyl)glycine;
3-chloro-4-(2-chloro-3-(5-(4,5-dihydro-1H-imidazol-2-yl)-6-methoxypyridin-2-yl)phenyl)-2-(4-(4,5-dihydro-1H-imidazol-2-yl)-3-methoxyphenyl)pyridine;
(S)-(4-(3-chloro-4-(2-chloro-3-(6-methoxy-5-((((5-oxopyrrolidin-2-yl)methyl)amino)methyl)pyridin-2-yl)phenyl)pyridin-2-yl)-2-methoxybenzyl)glycine;
(R)-(4-(3-chloro-4-(2-chloro-3-(6-methoxy-5-((((5-oxopyrrolidin-2-yl)methyl)amino)methyl)pyridin-2-yl)phenyl)pyridin-2-yl)-2-methoxybenzyl)glycine;
isopropyl (S)-(4-(3-chloro-4-(2-chloro-3-(6-methoxy-5-((((5-oxopyrrolidin-2-yl)methyl)amino)methyl)pyridin-2-yl)phenyl)pyridin-2-yl)-2-methoxybenzyl)glycinate;
isopropyl (R)-(4-(3-chloro-4-(2-chloro-3-(6-methoxy-5-((((5-oxopyrrolidin-2-yl)methyl)amino)methyl)pyridin-2-yl)phenyl)pyridin-2-yl)-2-methoxybenzyl)glycinate;
(S)-5-((((6-(3-(2-(4-(((1-acetylpiperidin-4-yl)amino)methyl)-3-methoxyphenyl)-3-chloropyridin-4-yl)-2-chlorophenyl)-2-methoxypyridin-3-yl)methyl)amino)methyl)pyrrolidin-2-one;
(R)-5-((((6-(3-(2-(4-(((1-acetylpiperidin-4-yl)amino)methyl)-3-methoxyphenyl)-3-chloropyridin-4-yl)-2-chlorophenyl)-2-methoxypyridin-3-yl)methyl)amino)methyl)pyrrolidin-2-one;
(S)-6-(3-chloro-4-(2-chloro-3-(6-methoxy-5-((((5-oxopyrrolidin-2-yl)methyl)amino)methyl)pyridin-2-yl)phenyl)pyridin-2-yl)-2-((3-hydroxy-3-methylazetidin-1-yl)methyl)-3-methylpyrrolo[2,1-f][1,2,4]triazin-4(3H)-one;
(R)-6-(3-chloro-4-(2-chloro-3-(6-methoxy-5-((((5-oxopyrrolidin-2-yl)methyl)amino)methyl)pyridin-2-yl)phenyl)pyridin-2-yl)-2-((3-hydroxy-3-methylazetidin-1-yl)methyl)-3-methylpyrrolo[2,1-f][1,2,4]triazin-4(3H)-one;
(S)-6-(3-chloro-4-(2-chloro-3-(6-methoxy-5-((((5-oxopyrrolidin-2-yl)methyl)amino)methyl)pyridin-2-yl)phenyl)pyridin-2-yl)-2-((isopropylamino)methyl)-3-methylpyrrolo[2,1-f][1,2,4]triazin-4(3H)-one;
(R)-6-(3-chloro-4-(2-chloro-3-(6-methoxy-5-((((5-oxopyrrolidin-2-yl)methyl)amino)methyl)pyridin-2-yl)phenyl)pyridin-2-yl)-2-((isopropylamino)methyl)-3-methylpyrrolo[2,1-f][1,2,4]triazin-4(3H)-one;
(5S)-5-((((6-(2-chloro-3-(3-chloro-2-(5-((((S)-5-oxopyrrolidin-2-yl)methyl)amino)-5,6,7,8-tetrahydronaphthalen-2-yl)pyridin-4-yl)phenyl)-2-methoxypyridin-3-yl)methyl)amino)methyl)pyrrolidin-2-one;
(5S)-5-((((6-(2-chloro-3-(3-chloro-2-(5-((((R)-5-oxopyrrolidin-2-yl)methyl)amino)-5,6,7,8-tetrahydronaphthalen-2-yl)pyridin-4-yl)phenyl)-2-methoxypyridin-3-yl)methyl)amino)methyl)pyrrolidin-2-one;
(5R)-5-((((6-(2-chloro-3-(3-chloro-2-(5-((((S)-5-oxopyrrolidin-2-yl)methyl)amino)-5,6,7,8-tetrahydronaphthalen-2-yl)pyridin-4-yl)phenyl)-2-methoxypyridin-3-yl)methyl)amino)methyl)pyrrolidin-2-one;
(5R)-5-((((6-(2-chloro-3-(3-chloro-2-(5-((((R)-5-oxopyrrolidin-2-yl)methyl)amino)-5,6,7,8-tetrahydronaphthalen-2-yl)pyridin-4-yl)phenyl)-2-methoxypyridin-3-yl)methyl)amino)methyl)pyrrolidin-2-one;
(5 S)-5-((((6-(2-chloro-3-(3-chloro-2-(5-(((S)-2-hydroxypropyl)amino)-5,6,7, 8-tetrahydronaphthalen-2-yl)pyridin-4-yl)phenyl)-2-methoxypyridin-3-yl)methyl)amino)methyl)pyrrolidin-2-one;
(5 S)-5-((((6-(2-chloro-3-(3-chloro-2-(5-(((R)-2-hydroxypropyl)amino)-5,6,7, 8-tetrahydronaphthalen-2-yl)pyridin-4-yl)phenyl)-2-methoxypyridin-3-yl)methyl)amino)methyl)pyrrolidin-2-one;
(5R)-5-((((6-(2-chloro-3-(3-chloro-2-(5-(((S)-2-hydroxypropyl)amino)-5,6,7,8-tetrahydronaphthalen-2-yl)pyridin-4-yl)phenyl)-2-methoxypyridin-3-yl)methyl)amino)methyl)pyrrolidin-2-one;
(5R)-5-((((6-(2-chloro-3-(3-chloro-2-(5-(((R)-2-hydroxypropyl)amino)-5,6,7,8-tetrahydronaphthalen-2-yl)pyridin-4-yl)phenyl)-2-methoxypyridin-3-yl)methyl)amino)methyl)pyrrolidin-2-one;
(S)-5-((((6-(2-chloro-3-(3-chloro-2-(4-((((S)-2-hydroxypropyl)amino)methyl)-3-methoxy-5-methylphenyl)pyridin-4-yl)phenyl)-2-methoxypyridin-3-yl)methyl)amino)methyl)pyrrolidin-2-one;
(S)-5-((((6-(2-chloro-3-(3-chloro-2-(4-((((R)-2-hydroxypropyl)amino)methyl)-3-methoxy-5-methylphenyl)pyridin-4-yl)phenyl)-2-methoxypyridin-3-yl)methyl)amino)methyl)pyrrolidin-2-one;
(R)-5-((((6-(2-chloro-3-(3-chloro-2-(4-((((S)-2-hydroxypropyl)amino)methyl)-3-methoxy-5-methylphenyl)pyridin-4-yl)phenyl)-2-methoxypyridin-3-yl)methyl)amino)methyl)pyrrolidin-2-one;
(R)-5-((((6-(2-chloro-3-(3-chloro-2-(4-((((R)-2-hydroxypropyl)amino)methyl)-3-methoxy-5-methylphenyl)pyridin-4-yl)phenyl)-2-methoxypyridin-3-yl)methyl)amino)methyl)pyrrolidin-2-one;
(S)-5-((((6-(2-chloro-3-(3-chloro-2-(3-methoxy-5-methyl-4-(((((S)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
(S)-5-((((6-(2-chloro-3-(3-chloro-2-(3-methoxy-5-methyl-4-(((((R)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
(R)-5-((((6-(2-chloro-3-(3-chloro-2-(3-methoxy-5-methyl-4-(((((S)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
(R)-5-((((6-(2-chloro-3-(3-chloro-2-(3-methoxy-5-methyl-4-(((((R)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
(S)-5-((((6-(2-chloro-3-(3-chloro-2-(isoindolin-5-yl)pyridin-4-yl)phenyl)-2-methoxypyridin-3-yl)methyl)amino)methyl)pyrrolidin-2-one;
(R)-5-((((6-(2-chloro-3-(3-chloro-2-(isoindolin-5-yl)pyridin-4-yl)phenyl)-2-methoxypyridin-3-yl)methyl)amino)methyl)pyrrolidin-2-one;
(S)-5-((((6-(2-chloro-3-(3-chloro-2-(3-methoxy-5-methyl-4-((methyl(((S)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
(S)-5-((((6-(2-chloro-3-(3-chloro-2-(3-methoxy-5-methyl-4-((methyl(((R)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
(R)-5-((((6-(2-chloro-3-(3-chloro-2-(3-methoxy-5-methyl-4-((methyl(((S)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
(R)-5-((((6-(2-chloro-3-(3-chloro-2-(3-methoxy-5-methyl-4-((methyl(((R)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
(S)-5-((((6-(2-chloro-3-(3-chloro-2-(4-((((S)-2-hydroxypropyl)(methyl)amino)methyl)-3-methoxy-5-methylphenyl)pyridin-4-yl)phenyl)-2-methoxypyridin-3-yl)methyl)amino)methyl)pyrrolidin-2-one;
(S)-5-((((6-(2-chloro-3-(3-chloro-2-(4-((((R)-2-hydroxypropyl)(methyl)amino)methyl)-3-methoxy-5-methylphenyl)pyridin-4-yl)phenyl)-2-methoxypyridin-3-yl)methyl)amino)methyl)pyrrolidin-2-one;
(R)-5-((((6-(2-chloro-3-(3-chloro-2-(4-((((S)-2-hydroxypropyl)(methyl)amino)methyl)-3-methoxy-5-methylphenyl)pyridin-4-yl)phenyl)-2-methoxypyridin-3-yl)methyl)amino)methyl)pyrrolidin-2-one;
(R)-5-((((6-(2-chloro-3-(3-chloro-2-(4-((((R)-2-hydroxypropyl)(methyl)amino)methyl)-3-methoxy-5-methylphenyl)pyridin-4-yl)phenyl)-2-methoxypyridin-3-yl)methyl)amino)methyl)pyrrolidin-2-one;
(S)-5-((((6-(3-(2-(1-(azetidin-3-yl)-1H-pyrazol-4-yl)-3-chloropyridin-4-yl)-2-chlorophenyl)-2-methoxypyridin-3-yl)methyl)amino)methyl)pyrrolidin-2-one;
(R)-5-((((6-(3-(2-(1-(azetidin-3-yl)-1H-pyrazol-4-yl)-3-chloropyridin-4-yl)-2-chlorophenyl)-2-methoxypyridin-3-yl)methyl)amino)methyl)pyrrolidin-2-one;
(S)-5-((((6-(3-(2-(1-(azetidin-3-ylmethyl)-1H-pyrazol-4-yl)-3-chloropyridin-4-yl)-2-chlorophenyl)-2-methoxypyridin-3-yl)methyl)amino)methyl)pyrrolidin-2-one;
(R)-5-((((6-(3-(2-(1-(azetidin-3-ylmethyl)-1H-pyrazol-4-yl)-3-chloropyridin-4-yl)-2-chlorophenyl)-2-methoxypyridin-3-yl)methyl)amino)methyl)pyrrolidin-2-one;
(S)-1-((4-(3-chloro-4-(2-chloro-3-(6-methoxy-5-((((5-oxopyrrolidin-2-yl)methyl)amino)methyl)pyridin-2-yl)phenyl)pyridin-2-yl)-2-methoxybenzyl)amino)cyclopropane-1-carboxylic acid;
(R)-1-((4-(3-chloro-4-(2-chloro-3-(6-methoxy-5-((((5-oxopyrrolidin-2-yl)methyl)amino)methyl)pyridin-2-yl)phenyl)pyridin-2-yl)-2-methoxybenzyl)amino)cyclopropane-1-carboxylic acid;
(S)-5-((((6-(2-chloro-3-(3-chloro-2-(3-methoxy-4-(((((S)-oxetan-2-yl)methyl)amino)methyl)phenyl)pyridin-4-yl)phenyl)-2-methoxypyridin-3-yl)methyl)amino)methyl)pyrrolidin-2-one;
(S)-5-((((6-(2-chloro-3-(3-chloro-2-(3-methoxy-4-(((((R)-oxetan-2-yl)methyl)amino)methyl)phenyl)pyridin-4-yl)phenyl)-2-methoxypyridin-3-yl)methyl)amino)methyl)pyrrolidin-2-one;
(R)-5-((((6-(2-chloro-3-(3-chloro-2-(3-methoxy-4-(((((S)-oxetan-2-yl)methyl)amino)methyl)phenyl)pyridin-4-yl)phenyl)-2-methoxypyridin-3-yl)methyl)amino)methyl)pyrrolidin-2-one;
(R)-5-((((6-(2-chloro-3-(3-chloro-2-(3-methoxy-4-(((((R)-oxetan-2-yl)methyl)amino)methyl)phenyl)pyridin-4-yl)phenyl)-2-methoxypyridin-3-yl)methyl)amino)methyl)pyrrolidin-2-one;
isopropyl (S)-(4-(3-chloro-4-(2-chloro-3-(4-chloro-6-methoxy-5-((((5-oxopyrrolidin-2-yl)methyl)amino)methyl)pyridin-2-yl)phenyl)pyridin-2-yl)-2-methoxybenzyl)glycinate;
isopropyl (R)-(4-(3-chloro-4-(2-chloro-3-(4-chloro-6-methoxy-5-((((5-oxopyrrolidin-2-yl)methyl)amino)methyl)pyridin-2-yl)phenyl)pyridin-2-yl)-2-methoxybenzyl)glycinate;
(S)-5-((((4-chloro-6-(2-chloro-3-(3-chloro-2-(2-(2-hydroxyethyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((4-chloro-6-(2-chloro-3-(3-chloro-2-(2-(2-hydroxyethyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
isopropyl (4-(3-chloro-4-(2-chloro-3-(6-methoxy-5-(((((S)-5-oxopyrrolidin-2-yl)methyl)amino)methyl)pyridin-2-yl)phenyl)pyridin-2-yl)-2-methoxybenzyl)-L-alaninate;
isopropyl (4-(3-chloro-4-(2-chloro-3-(6-methoxy-5-(((((S)-5-oxopyrrolidin-2-yl)methyl)amino)methyl)pyridin-2-yl)phenyl)pyridin-2-yl)-2-methoxybenzyl)-D-alaninate;
isopropyl (4-(3-chloro-4-(2-chloro-3-(6-methoxy-5-(((((R)-5-oxopyrrolidin-2-yl)methyl)amino)methyl)pyridin-2-yl)phenyl)pyridin-2-yl)-2-methoxybenzyl)-L-alaninate;
isopropyl (4-(3-chloro-4-(2-chloro-3-(6-methoxy-5-(((((R)-5-oxopyrrolidin-2-yl)methyl)amino)methyl)pyridin-2-yl)phenyl)pyridin-2-yl)-2-methoxybenzyl)-D-alaninate;
(S)-5-((((6-(2-chloro-3-(3-chloro-2-(4-(((1,1-dioxidotetrahydro-2H-thiopyran-4-yl)amino)methyl)-3-methoxyphenyl)pyridin-4-yl)phenyl)-2-methoxypyridin-3-yl)methyl)amino)methyl)pyrrolidin-2-one;
(R)-5-((((6-(2-chloro-3-(3-chloro-2-(4-(((1,1-dioxidotetrahydro-2H-thiopyran-4-yl)amino)methyl)-3-methoxyphenyl)pyridin-4-yl)phenyl)-2-methoxypyridin-3-yl)methyl)amino)methyl)pyrrolidin-2-one;
(S)-5-((((6-(2-chloro-3-(3-chloro-2-(3-methoxy-4-(((tetrahydro-2H-pyran-4-yl)amino)methyl)phenyl)pyridin-4-yl)phenyl)-2-methoxypyridin-3-yl)methyl)amino)methyl)pyrrolidin-2-one;
(R)-5-((((6-(2-chloro-3-(3-chloro-2-(3-methoxy-4-(((tetrahydro-2H-pyran-4-yl)amino)methyl)phenyl)pyridin-4-yl)phenyl)-2-methoxypyridin-3-yl)methyl)amino)methyl)pyrrolidin-2-one;
(4-(3-chloro-4-(2-chloro-3-(6-methoxy-5-(((((S)-5-oxopyrrolidin-2-yl)methyl)amino)methyl)pyridin-2-yl)phenyl)pyridin-2-yl)-2-methoxybenzyl)-L-alanine;
(4-(3-chloro-4-(2-chloro-3-(6-methoxy-5-(((((S)-5-oxopyrrolidin-2-yl)methyl)amino)methyl)pyridin-2-yl)phenyl)pyridin-2-yl)-2-methoxybenzyl)-D-alanine;
(4-(3-chloro-4-(2-chloro-3-(6-methoxy-5-(((((R)-5-oxopyrrolidin-2-yl)methyl)amino)methyl)pyridin-2-yl)phenyl)pyridin-2-yl)-2-methoxybenzyl)-L-alanine;
(4-(3-chloro-4-(2-chloro-3-(6-methoxy-5-(((((R)-5-oxopyrrolidin-2-yl)methyl)amino)methyl)pyridin-2-yl)phenyl)pyridin-2-yl)-2-methoxybenzyl)-D-alanine;
(S)-5-((((6-(2-chloro-3-(3-chloro-2-(2-((R)-2-hydroxypropyl)-8-methoxy-1,2,3,4-tetrahydroisoquinolin-6-yl)pyridin-4-yl)phenyl)-4-fluoro-2-methoxypyridin-3-yl)methyl)amino)methyl)pyrrolidin-2-one;
(S)-5-((((6-(2-chloro-3-(3-chloro-2-(2-((S)-2-hydroxypropyl)-8-methoxy-1,2,3,4-tetrahydroisoquinolin-6-yl)pyridin-4-yl)phenyl)-4-fluoro-2-methoxypyridin-3-yl)methyl)amino)methyl)pyrrolidin-2-one;
(R)-5-((((6-(2-chloro-3-(3-chloro-2-(2-((R)-2-hydroxypropyl)-8-methoxy-1,2,3,4-tetrahydroisoquinolin-6-yl)pyridin-4-yl)phenyl)-4-fluoro-2-methoxypyridin-3-yl)methyl)amino)methyl)pyrrolidin-2-one;
(R)-5-((((6-(2-chloro-3-(3-chloro-2-(2-((S)-2-hydroxypropyl)-8-methoxy-1,2,3,4-tetrahydroisoquinolin-6-yl)pyridin-4-yl)phenyl)-4-fluoro-2-methoxypyridin-3-yl)methyl)amino)methyl)pyrrolidin-2-one;
(S)-5-((((6-(2-chloro-3-(3-chloro-2-(2-(2-hydroxyethyl)-8-methoxy-1,2,3,4-tetrahydroisoquinolin-6-yl)pyridin-4-yl)phenyl)-4-fluoro-2-methoxypyridin-3-yl)methyl)amino)methyl)pyrrolidin-2-one;
(S)-5-((((6-(2-chloro-3-(3-chloro-2-(2-(2-hydroxyethyl)-8-methoxy-1,2,3,4-tetrahydroisoquinolin-6-yl)pyridin-4-yl)phenyl)-4-fluoro-2-methoxypyridin-3-yl)methyl)amino)methyl)pyrrolidin-2-one;
(S)-5-((((6-(2-chloro-3-(3-chloro-2-(2-(((1r,3r)-3-hydroxycyclobutyl)methyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(2-(((1s,3s)-3-hydroxycyclobutyl)methyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(2-(((1s,3r)-3-hydroxycyclobutyl)methyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-(((1s,3s)-3-hydroxycyclobutyl)methyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(2-(((1r,3r)-3-hydroxycyclobutyl)methyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-(((1s,3r)-3-hydroxycyclobutyl)methyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-(((1r,3s)-3-hydroxycyclobutyl)methyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-(((1r,3r)-3-hydroxycyclobutyl)methyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
isopropyl (S)-1-((4-(3-chloro-4-(2-chloro-3-(6-methoxy-5-((((5-oxopyrrolidin-2-yl)methyl)amino)methyl)pyridin-2-yl)phenyl)pyridin-2-yl)-2-methoxybenzyl)amino)cyclopropane-1-carboxylate;
isopropyl (R)-1-((4-(3-chloro-4-(2-chloro-3-(6-methoxy-5-((((5-oxopyrrolidin-2-yl)methyl)amino)methyl)pyridin-2-yl)phenyl)pyridin-2-yl)-2-methoxybenzyl)amino)cyclopropane-1-carboxylate;
1-(6-(2-chloro-3-(3-chloro-2-(3-methoxy-4-((methylamino)methyl)phenyl)pyridin-4-yl)phenyl)-2-methoxypyridin-3-yl)-N-methylmethanamine;
1-(6-(2-chloro-3-(3-chloro-2-(4-((dimethylamino)methyl)-3-methoxyphenyl)pyridin-4-yl)phenyl)-2-methoxypyridin-3-yl)-N,N-dimethylmethanamine;
2-(((6-(2-chloro-3-(3-chloro-2-(4-(((2-hydroxyethyl)(methyl)amino)methyl)-3-methoxyphenyl)pyridin-4-yl)phenyl)-2-methoxypyridin-3-yl)methyl)(methyl)amino)ethan-1-ol;
1-(4-(((6-(3-(2-(4-(((1-acetylpiperidin-4-yl)amino)methyl)-3-methoxyphenyl)-3-chloropyridin-4-yl)-2-chlorophenyl)-2-methoxypyridin-3-yl)methyl)amino)piperidin-1-yl)ethan-1-one;
1-(4-(((6-(3-(2-(4-(((1-acetylpiperidin-4-yl)(methyl)amino)methyl)-3-methoxyphenyl)-3-chloropyridin-4-yl)-2-chlorophenyl)-2-methoxypyridin-3-yl)methyl)(methyl)amino)piperidin-1-yl)ethan-1-one;
(S)-5-((((6-(2-chloro-3-(3-chloro-2-(5-methyl-2,3,4,5-tetrahydro-1H-pyrido[4,3-b]indol-7-yl)pyridin-4-yl)phenyl)-2-methoxypyridin-3-yl)methyl)amino)methyl)pyrrolidin-2-one;
(S)-5-((((6-(2-chloro-3-(3-chloro-2-(5-methyl-2,3,4,5-tetrahydro-1H-pyrido[4,3-b]indol-7-yl)pyridin-4-yl)phenyl)-2-methoxypyridin-3-yl)methyl)amino)methyl)pyrrolidin-2-one;
5-(3-chloro-4-(2-chloro-3-(5-((((S)-2-hydroxypropyl)amino)methyl)-6-methoxypyridin-2-yl)phenyl)pyridin-2-yl)-2-(2-((((S)-5-oxopyrrolidin-2-yl)methyl)amino)ethyl)isoindolin-1-one;
5-(3-chloro-4-(2-chloro-3-(5-((((S)-2-hydroxypropyl)amino)methyl)-6-methoxypyridin-2-yl)phenyl)pyridin-2-yl)-2-(2-((((R)-5-oxopyrrolidin-2-yl)methyl)amino)ethyl)isoindolin-1-one;
5-(3-chloro-4-(2-chloro-3-(5-((((R)-2-hydroxypropyl)amino)methyl)-6-methoxypyridin-2-yl)phenyl)pyridin-2-yl)-2-(2-((((S)-5-oxopyrrolidin-2-yl)methyl)amino)ethyl)isoindolin-1-one;
5-(3-chloro-4-(2-chloro-3-(5-((((R)-2-hydroxypropyl)amino)methyl)-6-methoxypyridin-2-yl)phenyl)pyridin-2-yl)-2-(2-((((R)-5-oxopyrrolidin-2-yl)methyl)amino)ethyl)isoindolin-1-one;
(S)-5-(4-(3-(5-(((1-acetylpiperidin-4-yl)amino)methyl)-6-methoxypyridin-2-yl)-2-chlorophenyl)-3-chloropyridin-2-yl)-2-(2-(((5-oxopyrrolidin-2-yl)methyl)amino)ethyl)isoindolin-1-one;
(R)-5-(4-(3-(5-(((1-acetylpiperidin-4-yl)amino)methyl)-6-methoxypyridin-2-yl)-2-chlorophenyl)-3-chloropyridin-2-yl)-2-(2-(((5-oxopyrrolidin-2-yl)methyl)amino)ethyl)isoindolin-1-one;
(S)-5-(4-(3-(5-((6-acetyl-2,6-diazaspiro[3.3]heptan-2-yl)methyl)-6-methoxypyridin-2-yl)-2-chlorophenyl)-3-chloropyridin-2-yl)-2-(2-(((5-oxopyrrolidin-2-yl)methyl)amino)ethyl)isoindolin-1-one;
(R)-5-(4-(3-(5-((6-acetyl-2,6-diazaspiro[3.3]heptan-2-yl)methyl)-6-methoxypyridin-2-yl)-2-chlorophenyl)-3-chloropyridin-2-yl)-2-(2-(((5-oxopyrrolidin-2-yl)methyl)amino)ethyl)isoindolin-1-one;
(S)-2-(4-(3-chloro-4-(2-chloro-3-(6-methoxy-5-((((5-oxopyrrolidin-2-yl)methyl)amino)methyl)pyridin-2-yl)phenyl)pyridin-2-yl)-2-fluoro-6-methoxybenzyl)-2,6-diazaspiro[3.4]octan-7-one;
(R)-2-(4-(3-chloro-4-(2-chloro-3-(6-methoxy-5-((((5-oxopyrrolidin-2-yl)methyl)amino)methyl)pyridin-2-yl)phenyl)pyridin-2-yl)-2-fluoro-6-methoxybenzyl)-2,6-diazaspiro[3.4]octan-7-one;
(S)-5-((((6-(2-chloro-3-(3-chloro-2-(3-fluoro-4-((6-hydroxy-2-azaspiro[3.3]heptan-2-yl)methyl)-5-methoxyphenyl)pyridin-4-yl)phenyl)-2-methoxypyridin-3-yl)methyl)amino)methyl)pyrrolidin-2-one;
(R)-5-((((6-(2-chloro-3-(3-chloro-2-(3-fluoro-4-((6-hydroxy-2-azaspiro[3.3]heptan-2-yl)methyl)-5-methoxyphenyl)pyridin-4-yl)phenyl)-2-methoxypyridin-3-yl)methyl)amino)methyl)pyrrolidin-2-one;
(S)-5-((((6-(2-chloro-3-(3-chloro-2-(3-fluoro-4-(((3-fluoropropyl)amino)methyl)-5-methoxyphenyl)pyridin-4-yl)phenyl)-2-methoxypyridin-3-yl)methyl)amino)methyl)pyrrolidin-2-one;
(R)-5-((((6-(2-chloro-3-(3-chloro-2-(3-fluoro-4-(((3-fluoropropyl)amino)methyl)-5-methoxyphenyl)pyridin-4-yl)phenyl)-2-methoxypyridin-3-yl)methyl)amino)methyl)pyrrolidin-2-one;
(S)-5-((((6-(3-(2-(4-(((1-acetylpiperidin-4-yl)amino)methyl)-3-fluoro-5-methoxyphenyl)-3-chloropyridin-4-yl)-2-chlorophenyl)-2-methoxypyridin-3-yl)methyl)amino)methyl)pyrrolidin-2-one;
(R)-5-((((6-(3-(2-(4-(((1-acetylpiperidin-4-yl)amino)methyl)-3-fluoro-5-methoxyphenyl)-3-chloropyridin-4-yl)-2-chlorophenyl)-2-methoxypyridin-3-yl)methyl)amino)methyl)pyrrolidin-2-one;
methyl (S)-2-(6-(3-chloro-4-(2-chloro-3-(6-methoxy-5-((((5-oxopyrrolidin-2-yl)methyl)amino)methyl)pyridin-2-yl)phenyl)pyridin-2-yl)-8-methoxy-3,4-dihydroisoquinolin-2(1H)-yl)acetate;
methyl (R)-2-(6-(3-chloro-4-(2-chloro-3-(6-methoxy-5-((((5-oxopyrrolidin-2-yl)methyl)amino)methyl)pyridin-2-yl)phenyl)pyridin-2-yl)-8-methoxy-3,4-dihydroisoquinolin-2(1H)-yl)acetate;
(S)-2-(6-(3-chloro-4-(2-chloro-3-(6-methoxy-5-((((5-oxopyrrolidin-2-yl)methyl)amino)methyl)pyridin-2-yl)phenyl)pyridin-2-yl)-8-methoxy-3,4-dihydroisoquinolin-2(1H)-yl)acetic acid;
(R)-2-(6-(3-chloro-4-(2-chloro-3-(6-methoxy-5-((((5-oxopyrrolidin-2-yl)methyl)amino)methyl)pyridin-2-yl)phenyl)pyridin-2-yl)-8-methoxy-3,4-dihydroisoquinolin-2(1H)-yl)acetic acid;
(S)-5-((((6-(3-(2-(2-((1R,3S,4S)-2-azabicyclo[2.2.1]heptan-3-yl)-1H-benzo[d]imidazol-6-yl)-3-chloropyridin-4-yl)-2-chlorophenyl)-2-methoxypyridin-3-yl)methyl)amino)methyl)pyrrolidin-2-one;
(R)-5-((((6-(3-(2-(2-((1R,3S,4S)-2-azabicyclo[2.2.1]heptan-3-yl)-1H-benzo[d]imidazol-6-yl)-3-chloropyridin-4-yl)-2-chlorophenyl)-2-methoxypyridin-3-yl)methyl)amino)methyl)pyrrolidin-2-one;
(S)-5-((((6-(3-(2-(2-((1R,3R,4S)-2-azabicyclo[2.2.1]heptan-3-yl)-1H-benzo[d]imidazol-6-yl)-3-chloropyridin-4-yl)-2-chlorophenyl)-2-methoxypyridin-3-yl)methyl)amino)methyl)pyrrolidin-2-one;
(R)-5-((((6-(3-(2-(2-((1R,3R,4S)-2-azabicyclo[2.2.1]heptan-3-yl)-1H-benzo[d]imidazol-6-yl)-3-chloropyridin-4-yl)-2-chlorophenyl)-2-methoxypyridin-3-yl)methyl)amino)methyl)pyrrolidin-2-one;
(S)-5-((((6-(3-(2-(2-((1S,3S,4R)-2-azabicyclo[2.2.1]heptan-3-yl)-1H-benzo[d]imidazol-6-yl)-3-chloropyridin-4-yl)-2-chlorophenyl)-2-methoxypyridin-3-yl)methyl)amino)methyl)pyrrolidin-2-one;
(R)-5-((((6-(3-(2-(2-((1S,3 S,4R)-2-azabicyclo [2.2.1]heptan-3-yl)-1H-benzo [d]imidazol-6-yl)-3-chloropyridin-4-yl)-2-chlorophenyl)-2-methoxypyridin-3-yl)methyl)amino)methyl)pyrrolidin-2-one;
(S)-5-((((6-(3-(2-(2-((1S,3R,4R)-2-azabicyclo[2.2.1]heptan-3-yl)-1H-benzo[d]imidazol-6-yl)-3-chloropyridin-4-yl)-2-chlorophenyl)-2-methoxypyridin-3-yl)methyl)amino)methyl)pyrrolidin-2-one;
(R)-5-((((6-(3-(2-(2-((1S,3R,4R)-2-azabicyclo[2.2.1]heptan-3-yl)-1H-benzo[d]imidazol-6-yl)-3-chloropyridin-4-yl)-2-chlorophenyl)-2-methoxypyridin-3-yl)methyl)amino)methyl)pyrrolidin-2-one;
(S)-5-((((6-(2-chloro-3-(3-chloro-2-(3-methoxy-5-(((((S)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
(S)-5-((((6-(2-chloro-3-(3-chloro-2-(3-methoxy-5-(((((R)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
(R)-5-((((6-(2-chloro-3-(3-chloro-2-(3-methoxy-5-(((((S)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
(R)-5-((((6-(2-chloro-3-(3-chloro-2-(3-methoxy-5-(((((R)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
1-(4-(((6-(3-(2-(3-(((1-acetylpiperidin-4-yl)amino)methyl)-5-methoxyphenyl)-3-chloropyridin-4-yl)-2-chlorophenyl)-2-methoxypyridin-3-yl)methyl)amino)piperidin-1-yl)ethan-1-one;
1-(6-(2-chloro-3-(3-chloro-2-(3-methoxy-5-((methylamino)methyl)phenyl)pyridin-4-yl)phenyl)-2-methoxypyridin-3-yl)-N-methylmethanamine;
chloro-3-(3-chloro-2-(3-(((2-hydroxyethyl)amino)methyl)-5-methoxyphenyl)pyridin-4-yl)phenyl)-2-methoxypyridin-3-yl)methyl)amino)ethan-1-ol;
(S)-1-(((6-(2-chloro-3-(3-chloro-2-(3-((((S)-2-hydroxypropyl)amino)methyl)-5-methoxyphenyl)pyridin-4-yl)phenyl)-2-methoxypyridin-3-yl)methyl)amino)propan-2-ol;
(S)-1-(((6-(2-chloro-3-(3-chloro-2-(3-((((R)-2-hydroxypropyl)amino)methyl)-5-methoxyphenyl)pyridin-4-yl)phenyl)-2-methoxypyridin-3-yl)methyl)amino)propan-2-ol;
(R)-1-(((6-(2-chloro-3-(3-chloro-2-(3-((((S)-2-hydroxypropyl)amino)methyl)-5-methoxyphenyl)pyridin-4-yl)phenyl)-2-methoxypyridin-3-yl)methyl)amino)propan-2-ol;
(R)-1-(((6-(2-chloro-3-(3-chloro-2-(3-((((R)-2-hydroxypropyl)amino)methyl)-5-methoxyphenyl)pyridin-4-yl)phenyl)-2-methoxypyridin-3-yl)methyl)amino)propan-2-ol;
(S)-5-((((3'-chloro-2'-((S)-1-((5-((((S)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)methyl)pyrrolidin-2-one;
(S)-5-((((3'-chloro-2'-((S)-1-((5-((((R)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)methyl)pyrrolidin-2-one;
(S)-5-((((3'-chloro-2'-((R)-1-((5-((((S)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)methyl)pyrrolidin-2-one;
(R)-5-((((3'-chloro-2'-((S)-1-((5-((((S)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)methyl)pyrrolidin-2-one;
(S)-5-((((3'-chloro-2'-((R)-1-((5-((((R)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)methyl)pyrrolidin-2-one;
(R)-5-((((3'-chloro-2'-((S)-1-((5-((((R)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)methyl)pyrrolidin-2-one;
(R)-5-((((3'-chloro-2'-((R)-1-((5-((((S)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)methyl)pyrrolidin-2-one;
(R)-5-((((3'-chloro-2'-((R)-1-((5-((((R)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)methyl)pyrrolidin-2-one;
1-(((6-(((S)-4-(3'-chloro-6-methoxy-5-(((((S)-5-oxopyrrolidin-2-yl)methyl)amino)methyl)-[2,4'-bipyridin]-2'-yl)-2,3-dihydro-1H-inden-1-yl)oxy)-2-methoxy-5-(trifluoromethyl)pyridin-3-yl)methyl)amino)cyclopropane-1-carboxylic acid;
1-(((6-(((S)-4-(3'-chloro-6-methoxy-5-(((((R)-5-oxopyrrolidin-2-yl)methyl)amino)methyl)-[2,4'-bipyridin]-2'-yl)-2,3-dihydro-1H-inden-1-yl)oxy)-2-methoxy-5-(trifluoromethyl)pyridin-3-yl)methyl)amino)cyclopropane-1-carboxylic acid;
1-(((6-(((R)-4-(3'-chloro-6-methoxy-5-(((((S)-5-oxopyrrolidin-2-yl)methyl)amino)methyl)-[2,4'-bipyridin]-2'-yl)-2,3-dihydro-1H-inden-1-yl)oxy)-2-methoxy-5-(trifluoromethyl)pyridin-3-yl)methyl)amino)cyclopropane-1-carboxylic acid;
1-(((6-(((R)-4-(3'-chloro-6-methoxy-5-(((((R)-5-oxopyrrolidin-2-yl)methyl)amino)methyl)-[2,4'-bipyridin]-2'-yl)-2,3-dihydro-1H-inden-1-yl)oxy)-2-methoxy-5-(trifluoromethyl)pyridin-3-yl)methyl)amino)cyclopropane-1-carboxylic acid;
methyl 1-(((6-(((S)-4-(3'-chloro-6-methoxy-5-(((((S)-5-oxopyrrolidin-2-yl)methyl)amino)methyl)-[2,4'-bipyridin]-2'-yl)-2,3-dihydro-1H-inden-1-yl)amino)-2-methoxy-5-(trifluoromethyl)pyridin-3-yl)methyl)amino)cyclopropane-1-carboxylate;
methyl 1-(((6-(((S)-4-(3'-chloro-6-methoxy-5-(((((R)-5-oxopyrrolidin-2-yl)methyl)amino)methyl)-[2,4'-bipyridin]-2'-yl)-2,3-dihydro-1H-inden-1-yl)amino)-2-methoxy-5-(trifluoromethyl)pyridin-3-yl)methyl)amino)cyclopropane-1-carboxylate;
methyl 1-(((6-(((R)-4-(3'-chloro-6-methoxy-5-(((((S)-5-oxopyrrolidin-2-yl)methyl)amino)methyl)-[2,4'-bipyridin]-2'-yl)-2,3-dihydro-1H-inden-1-yl)amino)-2-methoxy-5-(trifluoromethyl)pyridin-3-yl)methyl)amino)cyclopropane-1-carboxylate;
methyl 1-(((6-(((R)-4-(3'-chloro-6-methoxy-5-(((((R)-5-oxopyrrolidin-2-yl)methyl)amino)methyl)-[2,4'-bipyridin]-2'-yl)-2,3-dihydro-1H-inden-1-yl)amino)-2-methoxy-5-(trifluoromethyl)pyridin-3-yl)methyl)amino)cyclopropane-1-carboxylate;
(S)-5-((((6-(2-chloro-3-(3-chloro-2-(2-(2-hydroxyethyl)-5-methyl-2,3,4,5-tetrahydro-1H-pyrido[4,3-b]indol-7-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-(2-hydroxyethyl)-5-methyl-2,3,4,5-tetrahydro-1H-pyrido[4,3-b]indol-7-yl)pyridin-4-yl)phenyl)-2-methoxypyridin-3-yl)methyl)amino)methyl)pyrrolidin-2-one;
1-(6-(2-chloro-3-(3-chloro-2-(isoindolin-5-yl)pyridin-4-yl)phenyl)-2-methoxypyridin-3-yl)-N-methylmethanamine;
2-(((6-(2-chloro-3-(3-chloro-2-(isoindolin-5-yl)pyridin-4-yl)phenyl)-2-methoxypyridin-3-yl)methyl)amino)ethan-1-ol;
(S)-1-(((6-(2-chloro-3-(3-chloro-2-(isoindolin-5-yl)pyridin-4-yl)phenyl)-2-methoxypyridin-3-yl)methyl)amino)propan-2-ol;
(R)-1-(((6-(2-chloro-3-(3-chloro-2-(isoindolin-5-yl)pyridin-4-yl)phenyl)-2-methoxypyridin-3-yl)methyl)amino)propan-2-ol;
1-(4-(((6-(2-chloro-3-(3-chloro-2-(isoindolin-5-yl)pyridin-4-yl)phenyl)-2-methoxypyridin-3-yl)methyl)amino)piperidin-1-yl)ethan-1-one;
1-(4-(((6-(2-chloro-3-(3-chloro-2-(isoindolin-5-yl)pyridin-4-yl)phenyl)-2-methoxypyridin-3-yl)methyl)(methyl)amino)piperidin-1-yl)ethan-1-one;
1-(((6-(((S)-4-(3'-chloro-6-methoxy-5-(((((S)-5-oxopyrrolidin-2-yl)methyl)amino)methyl)-[2,4'-bipyridin]-2'-yl)-2,3-dihydro-1H-inden-1-yl)amino)-2-methoxy-5-(trifluoromethyl)pyridin-3-yl)methyl)amino)cyclopropane-1-carboxylic acid;
1-(((6-(((S)-4-(3'-chloro-6-methoxy-5-(((((R)-5-oxopyrrolidin-2-yl)methyl)amino)methyl)-[2,4'-bipyridin]-2'-yl)-2,3-dihydro-1H-inden-1-yl)amino)-2-methoxy-5-(trifluoromethyl)pyridin-3-yl)methyl)amino)cyclopropane-1-carboxylic acid;
1-(((6-(((R)-4-(3'-chloro-6-methoxy-5-(((((S)-5-oxopyrrolidin-2-yl)methyl)amino)methyl)-[2,4'-bipyridin]-2'-yl)-2,3-dihydro-1H-inden-1-yl)amino)-2-methoxy-5-(trifluoromethyl)pyridin-3-yl)methyl)amino)cyclopropane-1-carboxylic acid;
1-(((6-(((R)-4-(3'-chloro-6-methoxy-5-(((((R)-5-oxopyrrolidin-2-yl)methyl)amino)methyl)-[2,4'-bipyridin]-2'-yl)-2,3-dihydro-1H-inden-1-yl)amino)-2-methoxy-5-(trifluoromethyl)pyridin-3-yl)methyl)amino)cyclopropane-1-carboxylic acid;
(S)-5-((((3'-chloro-2'-((S)-1-((5-((((R)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)methyl)pyrrolidin-2-one;
(S)-5-((((3'-chloro-2'-((S)-1-((5-((((S)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)methyl)pyrrolidin-2-one;
(S)-5-((((3'-chloro-2'-((R)-1-((5-((((S)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)methyl)pyrrolidin-2-one;
(S)-5-((((3'-chloro-2'-((R)-1-((5-((((R)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)methyl)pyrrolidin-2-one;
(R)-5-((((3'-chloro-2'-((R)-1-((5-((((R)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)methyl)pyrrolidin-2-one;
(R)-5-((((3'-chloro-2'-((S)-1-((5-((((S)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)methyl)pyrrolidin-2-one;
(R)-5-((((3'-chloro-2'-((S)-1-((5-((((R)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)methyl)pyrrolidin-2-one;
(R)-5-((((3'-chloro-2'-((R)-1-((5-((((S)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)methyl)pyrrolidin-2-one;
(S)-5-((((3'-chloro-6-methoxy-2'-((S)-1-((6-methoxy-5-(((((S)-5-oxopyrrolidin-2-yl)methyl)amino)methyl)-3-(trifluoromethyl)pyridin-2-yl)oxy)-2,3-dihydro-1H-inden-4-yl)-[2,4'-bipyridin]-5-yl)methyl)amino)methyl)pyrrolidin-2-one;
(S)-5-((((3'-chloro-6-methoxy-2'-((S)-1-((6-methoxy-5-(((((R)-5-oxopyrrolidin-2-yl)methyl)amino)methyl)-3-(trifluoromethyl)pyridin-2-yl)oxy)-2,3-dihydro-1H-inden-4-yl)-[2,4'-bipyridin]-5-yl)methyl)amino)methyl)pyrrolidin-2-one;
(S)-5-((((3'-chloro-6-methoxy-2'-((R)-1-((6-methoxy-5-(((((R)-5-oxopyrrolidin-2-yl)methyl)amino)methyl)-3-(trifluoromethyl)pyridin-2-yl)oxy)-2,3-dihydro-1H-inden-4-yl)-[2,4'-bipyridin]-5-yl)methyl)amino)methyl)pyrrolidin-2-one;
(S)-5-((((3'-chloro-6-methoxy-2'-((R)-1-((6-methoxy-5-(((((R)-5-oxopyrrolidin-2-yl)methyl)amino)methyl)-3-(trifluoromethyl)pyridin-2-yl)oxy)-2,3-dihydro-1H-inden-4-yl)-[2,4'-bipyridin]-5-yl)methyl)amino)methyl)pyrrolidin-2-one;
(R)-5-((((3'-chloro-6-methoxy-2'-((R)-1-((6-methoxy-5-(((((R)-5-oxopyrrolidin-2-yl)methyl)amino)methyl)-3-(trifluoromethyl)pyridin-2-yl)oxy)-2,3-dihydro-1H-inden-4-yl)-[2,4'-bipyridin]-5-yl)methyl)amino)methyl)pyrrolidin-2-one;
(R)-5-((((3'-chloro-6-methoxy-2'-((S)-1-((6-methoxy-5-(((((S)-5-oxopyrrolidin-2-yl)methyl)amino)methyl)-3-(trifluoromethyl)pyridin-2-yl)oxy)-2,3-dihydro-1H-inden-4-yl)-[2,4'-bipyridin]-5-yl)methyl)amino)methyl)pyrrolidin-2-one;
(R)-5-((((3'-chloro-6-methoxy-2'-((S)-1-((6-methoxy-5-(((((R)-5-oxopyrrolidin-2-yl)methyl)amino)methyl)-3-(trifluoromethyl)pyridin-2-yl)oxy)-2,3-dihydro-1H-inden-4-yl)-[2,4'-bipyridin]-5-yl)methyl)amino)methyl)pyrrolidin-2-one;
(R)-5-((((3'-chloro-6-methoxy-2'-((R)-1-((6-methoxy-5-(((((S)-5-oxopyrrolidin-2-yl)methyl)amino)methyl)-3-(trifluoromethyl)pyridin-2-yl)oxy)-2,3-dihydro-1H-inden-4-yl)-[2,4'-bipyridin]-5-yl)methyl)amino)methyl)pyrrolidin-2-one;
(S)-5-((((6-(3-(2-(3-(((1-acetylpiperidin-4-yl)amino)methyl)-1-methyl-1H-indazol-6-yl)-3-chloropyridin-4-yl)-2-chlorophenyl)-2-methoxypyridin-3-yl)methyl)amino)methyl)pyrrolidin-2-one;
(R)-5-((((6-(3-(2-(3-(((1-acetylpiperidin-4-yl)amino)methyl)-1-methyl-1H-indazol-6-yl)-3-chloropyridin-4-yl)-2-chlorophenyl)-2-methoxypyridin-3-yl)methyl)amino)methyl)pyrrolidin-2-one;
(S)-5-((((6-(3-(2-(3-(((1-acetylpiperidin-4-yl)(methyl)amino)methyl)-1-methyl-1H-indazol-6-yl)-3-chloropyridin-4-yl)-2-chlorophenyl)-2-methoxypyridin-3-yl)methyl)amino)methyl)pyrrolidin-2-one;
(R)-5-((((6-(3-(2-(3-(((1-acetylpiperidin-4-yl)(methyl)amino)methyl)-1-methyl-1H-indazol-6-yl)-3-chloropyridin-4-yl)-2-chlorophenyl)-2-methoxypyridin-3-yl)methyl)amino)methyl)pyrrolidin-2-one;
(S)-4-((3-(3-chloro-4-(2-chloro-3-(6-methoxy-5-((methylamino)methyl)pyridin-2-yl)phenyl)pyridin-2-yl)-5-methoxybenzyl)amino)-3-hydroxybutanoic acid;
(R)-4-((3-(3-chloro-4-(2-chloro-3-(6-methoxy-5-((methylamino)methyl)pyridin-2-yl)phenyl)pyridin-2-yl)-5-methoxybenzyl)amino)-3-hydroxybutanoic acid;
3-((3-(3-chloro-4-(2-chloro-3-(6-methoxy-5-((methylamino)methyl)pyridin-2-yl)phenyl)pyridin-2-yl)-5-methoxybenzyl)amino)propanoic acid;
(S)-1-(3-(3-chloro-4-(2-chloro-3-(6-methoxy-5-((methylamino)methyl)pyridin-2-yl)phenyl)pyridin-2-yl)-5-methoxybenzyl)pyrrolidine-3-carboxylic acid;
(R)-1-(3-(3-chloro-4-(2-chloro-3-(6-methoxy-5-((methylamino)methyl)pyridin-2-yl)phenyl)pyridin-2-yl)-5-methoxybenzyl)pyrrolidine-3-carboxylic acid;
2-(1-(3-(3-chloro-4-(2-chloro-3-(6-methoxy-5-((methylamino)methyl)pyridin-2-yl)phenyl)pyridin-2-yl)-5-methoxybenzyl)azetidin-3-yl)acetic acid;
2-(3-(3-chloro-4-(2-chloro-3-(6-methoxy-5-((methylamino)methyl)pyridin-2-yl)phenyl)pyridin-2-yl)-5-methoxybenzyl)-2-azaspiro[3.3]heptane-6-carboxylic acid;
3-((3-(3-chloro-4-(2-chloro-3-(6-methoxy-5-((methylamino)methyl)pyridin-2-yl)phenyl)pyridin-2-yl)-5-methoxybenzyl)amino)bicyclo[1.1.1]pentane-1-carboxylic acid;
3-(((3-(3-chloro-4-(2-chloro-3-(6-methoxy-5-((methylamino)methyl)pyridin-2-yl)phenyl)pyridin-2-yl)-5-methoxybenzyl)amino)methyl)bicyclo[1.1.1]pentane-1-carboxylic acid;
(S)-5-((((6-(2-chloro-3-(3-chloro-2-(2-((S)-2-hydroxypropyl)-8-methoxy-1,2,3,4-tetrahydroisoquinolin-6-yl)pyridin-4-yl)phenyl)-4-fluoro-2-methoxypyridin-3-yl)methyl)amino)methyl)pyrrolidin-2-one;
(S)-5-((((6-(2-chloro-3-(3-chloro-2-(2-((R)-2-hydroxypropyl)-8-methoxy-1,2,3,4-tetrahydroisoquinolin-6-yl)pyridin-4-yl)phenyl)-4-fluoro-2-methoxypyridin-3-yl)methyl)amino)methyl)pyrrolidin-2-one;
(R)-5-((((6-(2-chloro-3-(3-chloro-2-(2-((S)-2-hydroxypropyl)-8-methoxy-1,2,3,4-tetrahydroisoquinolin-6-yl)pyridin-4-yl)phenyl)-4-fluoro-2-methoxypyridin-3-yl)methyl)amino)methyl)pyrrolidin-2-one;
(R)-5-((((6-(2-chloro-3-(3-chloro-2-(2-((R)-2-hydroxypropyl)-8-methoxy-1,2,3,4-tetrahydroisoquinolin-6-yl)pyridin-4-yl)phenyl)-4-fluoro-2-methoxypyridin-3-yl)methyl)amino)methyl)pyrrolidin-2-one;
(S)-5-((((6-(2-chloro-3-(3-chloro-2-(1,1-dioxido-2,3,4,5-tetrahydrobenzo[f][1,4]thiazepin-8-yl)pyridin-4-yl)phenyl)-2-methoxypyridin-3-yl)methyl)amino)methyl)pyrrolidin-2-one;
(R)-5-((((6-(2-chloro-3-(3-chloro-2-(1,1-dioxido-2,3,4,5-tetrahydrobenzo[f][1,4]thiazepin-8-yl)pyridin-4-yl)phenyl)-2-methoxypyridin-3-yl)methyl)amino)methyl)pyrrolidin-2-one;
5-(3-chloro-4-(2-chloro-3-(4-fluoro-6-methoxy-5-(((((S)-5-oxopyrrolidin-2-yl)methyl)amino)methyl)pyridin-2-yl)phenyl)pyridin-2-yl)-2-(2-((((S)-5-oxopyrrolidin-2-yl)methyl)amino)ethyl)isoindolin-1-one;
5-(3-chloro-4-(2-chloro-3-(4-fluoro-6-methoxy-5-(((((S)-5-oxopyrrolidin-2-yl)methyl)amino)methyl)pyridin-2-yl)phenyl)pyridin-2-yl)-2-(2-((((R)-5-oxopyrrolidin-2-yl)methyl)amino)ethyl)isoindolin-1-one;
5-(3-chloro-4-(2-chloro-3-(4-fluoro-6-methoxy-5-(((((R)-5-oxopyrrolidin-2-yl)methyl)amino)methyl)pyridin-2-yl)phenyl)pyridin-2-yl)-2-(2-((((S)-5-oxopyrrolidin-2-yl)methyl)amino)ethyl)isoindolin-1-one;
5-(3-chloro-4-(2-chloro-3-(4-fluoro-6-methoxy-5-(((((R)-5-oxopyrrolidin-2-yl)methyl)amino)methyl)pyridin-2-yl)phenyl)pyridin-2-yl)-2-(2-((((R)-5-oxopyrrolidin-2-yl)methyl)amino)ethyl)isoindolin-1-one;
(S)-5-((((6-(2-chloro-3-(3-chloro-2-(4-(4,5-dihydro-1H-imidazol-2-yl)-3-methoxyphenyl)pyridin-4-yl)phenyl)-2-methoxypyridin-3-yl)methyl)amino)methyl)pyrrolidin-2-one;
(R)-5-((((6-(2-chloro-3-(3-chloro-2-(4-(4,5-dihydro-1H-imidazol-2-yl)-3-methoxyphenyl)pyridin-4-yl)phenyl)-2-methoxypyridin-3-yl)methyl)amino)methyl)pyrrolidin-2-one;
(S)-4-((4-(3-chloro-4-(2-chloro-3-(5-(4,5-dihydro-1H-imidazol-2-yl)-6-methoxypyridin-2-yl)phenyl)pyridin-2-yl)-2-methoxybenzyl)amino)-3-hydroxybutanoic acid;
(R)-4-((4-(3-chloro-4-(2-chloro-3-(5-(4,5-dihydro-1H-imidazol-2-yl)-6-methoxypyridin-2-yl)phenyl)pyridin-2-yl)-2-methoxybenzyl)amino)-3-hydroxybutanoic acid;
(S)-5-((((6-(2-chloro-3-(3-chloro-2-(3-fluoro-5-methoxy-4-(((((S)-oxetan-2-yl)methyl)amino)methyl)phenyl)pyridin-4-yl)phenyl)-2-methoxypyridin-3-yl)methyl)amino)methyl)pyrrolidin-2-one;
(S)-5-((((6-(2-chloro-3-(3-chloro-2-(3-fluoro-5-methoxy-4-(((((R)-oxetan-2-yl)methyl)amino)methyl)phenyl)pyridin-4-yl)phenyl)-2-methoxypyridin-3-yl)methyl)amino)methyl)pyrrolidin-2-one;
(R)-5-((((6-(2-chloro-3-(3-chloro-2-(3-fluoro-5-methoxy-4-(((((S)-oxetan-2-yl)methyl)amino)methyl)phenyl)pyridin-4-yl)phenyl)-2-methoxypyridin-3-yl)methyl)amino)methyl)pyrrolidin-2-one;
(S)-5-((((6-(2-chloro-3-(3-chloro-2-(3-fluoro-5-methoxy-4-(((((S)-oxetan-2-yl)methyl)amino)methyl)phenyl)pyridin-4-yl)phenyl)-2-methoxypyridin-3-yl)methyl)amino)methyl)pyrrolidin-2-one;
methyl (S)-1-((6-(3-chloro-4-(2-chloro-3-(6-methoxy-5-((((5-oxopyrrolidin-2-yl)methyl)amino)methyl)pyridin-2-yl)phenyl)pyridin-2-yl)-3-methyl-4-oxo-3,4-dihydropyrrolo[2,1-f][1,2,4]triazin-2-yl)methyl)azetidine-3-carboxylate;
methyl (R)-1-((6-(3-chloro-4-(2-chloro-3-(6-methoxy-5-((((5-oxopyrrolidin-2-yl)methyl)amino)methyl)pyridin-2-yl)phenyl)pyridin-2-yl)-3-methyl-4-oxo-3,4-dihydropyrrolo[2,1-f][1,2,4]triazin-2-yl)methyl)azetidine-3-carboxylate;
(S)-5-((((6-(2-chloro-3-(3-chloro-2-(1-methyl-3-(((((S)-5-oxopyrrolidin-2-yl)methyl)amino)methyl)-1H-indazol-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(1-methyl-3-(((((S)-5-oxopyrrolidin-2-yl)methyl)amino)methyl)-1H-indazol-6-yl)pyridin-4-yl)phenyl)-2-methoxypyridin-3-yl)methyl)amino)methyl)pyrrolidin-2-one;
(S)-5-((((6-(2-chloro-3-(3-chloro-2-((S)-5-((((S)-5-oxopyrrolidin-2-yl)methyl)amino)-5,6,7,8-tetrahydronaphthalen-2-yl)pyridin-4-yl)phenyl)-2-methoxypyridin-3-yl)methyl)amino)methyl)pyrrolidin-2-one;
(S)-5-((((6-(2-chloro-3-(3-chloro-2-((S)-5-((((R)-5-oxopyrrolidin-2-yl)methyl)amino)-5,6,7,8-tetrahydronaphthalen-2-yl)pyridin-4-yl)phenyl)-2-methoxypyridin-3-yl)methyl)amino)methyl)pyrrolidin-2-one;
(S)-5-((((6-(2-chloro-3-(3-chloro-2-((R)-5-((((S)-5-oxopyrrolidin-2-yl)methyl)amino)-5,6,7,8-tetrahydronaphthalen-2-yl)pyridin-4-yl)phenyl)-2-methoxypyridin-3-yl)methyl)amino)methyl)pyrrolidin-2-one;
(S)-5-((((6-(2-chloro-3-(3-chloro-2-((R)-5-((((R)-5-oxopyrrolidin-2-yl)methyl)amino)-5,6,7,8-tetrahydronaphthalen-2-yl)pyridin-4-yl)phenyl)-2-methoxypyridin-3-yl)methyl)amino)methyl)pyrrolidin-2-one;
(R)-5-((((6-(2-chloro-3-(3-chloro-2-((R)-5-((((R)-5-oxopyrrolidin-2-yl)methyl)amino)-5,6,7,8-tetrahydronaphthalen-2-yl)pyridin-4-yl)phenyl)-2-methoxypyridin-3-yl)methyl)amino)methyl)pyrrolidin-2-one;
(R)-5-((((6-(2-chloro-3-(3-chloro-2-((R)-5-((((S)-5-oxopyrrolidin-2-yl)methyl)amino)-5,6,7,8-tetrahydronaphthalen-2-yl)pyridin-4-yl)phenyl)-2-methoxypyridin-3-yl)methyl)amino)methyl)pyrrolidin-2-one;
(R)-5-((((6-(2-chloro-3-(3-chloro-2-((R)-5-((((R)-5-oxopyrrolidin-2-yl)methyl)amino)-5,6,7,8-tetrahydronaphthalen-2-yl)pyridin-4-yl)phenyl)-2-methoxypyridin-3-yl)methyl)amino)methyl)pyrrolidin-2-one;
(R)-5-((((6-(2-chloro-3-(3-chloro-2-((S)-5-((((S)-5-oxopyrrolidin-2-yl)methyl)amino)-5,6,7,8-tetrahydronaphthalen-2-yl)pyridin-4-yl)phenyl)-2-methoxypyridin-3-yl)methyl)amino)methyl)pyrrolidin-2-one;
1-(((6-(((S)-5-(3'-chloro-6-methoxy-5-(((((S)-5-oxopyrrolidin-2-yl)methyl)amino)methyl)-[2,4'-bipyridin]-2'-yl)-1,2,3,4-tetrahydronaphthalen-1-yl)amino)-2-methoxy-5-(trifluoromethyl)pyridin-3-yl)methyl)amino)cyclopropane-1-carboxylic acid;
1-(((6-(((S)-5-(3'-chloro-6-methoxy-5-(((((R)-5-oxopyrrolidin-2-yl)methyl)amino)methyl)-[2,4'-bipyridin]-2'-yl)-1,2,3,4-tetrahydronaphthalen-1-yl)amino)-2-methoxy-5-(trifluoromethyl)pyridin-3-yl)methyl)amino)cyclopropane-1-carboxylic acid;
1-(((6-(((R)-5-(3'-chloro-6-methoxy-5-(((((S)-5-oxopyrrolidin-2-yl)methyl)amino)methyl)-[2,4'-bipyridin]-2'-yl)-1,2,3,4-tetrahydronaphthalen-1-yl)amino)-2-methoxy-5-(trifluoromethyl)pyridin-3-yl)methyl)amino)cyclopropane-1-carboxylic acid;
1-(((6-(((R)-5-(3'-chloro-6-methoxy-5-(((((R)-5-oxopyrrolidin-2-yl)methyl)amino)methyl)-[2,4'-bipyridin]-2'-yl)-1,2,3,4-tetrahydronaphthalen-1-yl)amino)-2-methoxy-5-(trifluoromethyl)pyridin-3-yl)methyl)amino)cyclopropane-1-carboxylic acid;
(S)-5-((((3'-chloro-2'-((S)-5-((5-((((S)-2-hydroxypropyl)amino)methyl)-6-methoxy-3-(trifluoromethyl)pyridin-2-yl)amino)-5,6,7,8-tetrahydronaphthalen-1-yl)-6-methoxy-[2,4'-bipyridin]-5-yl)methyl)amino)methyl)pyrrolidin-2-one;
(S)-5-((((3'-chloro-2'-((S)-5-((5-((((R)-2-hydroxypropyl)amino)methyl)-6-methoxy-3-(trifluoromethyl)pyridin-2-yl)amino)-5,6,7,8-tetrahydronaphthalen- 1-yl)-6-methoxy-[2,4'-bipyridin]-5-yl)methyl)amino)methyl)pyrrolidin-2-one;
(S)-5-((((3'-chloro-2'-((R)-5-((5-((((S)-2-hydroxypropyl)amino)methyl)-6-methoxy-3-(trifluoromethyl)pyridin-2-yl)amino)-5,6,7,8-tetrahydronaphthalen- 1-yl)-6-methoxy-[2,4'-bipyridin]-5-yl)methyl)amino)methyl)pyrrolidin-2-one;
(S)-5-((((3'-chloro-2'-((R)-5-((5-((((R)-2-hydroxypropyl)amino)methyl)-6-methoxy-3-(trifluoromethyl)pyridin-2-yl)amino)-5,6,7,8-tetrahydronaphthalen- 1-yl)-6-methoxy-[2,4'-bipyridin]-5-yl)methyl)amino)methyl)pyrrolidin-2-one;
(R)-5-((((3'-chloro-2'-((R)-5-((5-((((R)-2-hydroxypropyl)amino)methyl)-6-methoxy-3-(trifluoromethyl)pyridin-2-yl)amino)-5,6,7,8-tetrahydronaphthalen- 1-yl)-6-methoxy-[2,4'-bipyridin]-5-yl)methyl)amino)methyl)pyrrolidin-2-one;
(R)-5-((((3'-chloro-2'-((R)-5-((5-((((S)-2-hydroxypropyl)amino)methyl)-6-methoxy-3-(trifluoromethyl)pyridin-2-yl)amino)-5,6,7,8-tetrahydronaphthalen- 1-yl)-6-methoxy-[2,4'-bipyridin]-5-yl)methyl)amino)methyl)pyrrolidin-2-one;
(R)-5-((((3'-chloro-2'-((S)-5-((5-((((R)-2-hydroxypropyl)amino)methyl)-6-methoxy-3-(trifluoromethyl)pyridin-2-yl)amino)-5,6,7,8-tetrahydronaphthalen- 1-yl)-6-methoxy-[2,4'-bipyridin]-5-yl)methyl)amino)methyl)pyrrolidin-2-one;
(R)-5-((((3'-chloro-2'-((S)-5-((5-((((S)-2-hydroxypropyl)amino)methyl)-6-methoxy-3-(trifluoromethyl)pyridin-2-yl)amino)-5,6,7,8-tetrahydronaphthalen- 1-yl)-6-methoxy-[2,4'-bipyridin]-5-yl)methyl)amino)methyl)pyrrolidin-2-one;
(S)-5-((((3'-chloro-6-methoxy-2'-((S)-5-((6-methoxy-5-(((((S)-5-oxopyrrolidin-2-yl)methyl)amino)methyl)-3-(trifluoromethyl)pyridin-2-yl)amino)-5,6,7,8-tetrahydronaphthalen-1-yl)-[2,4'-bipyridin]-5-yl)methyl)amino)methyl)pyrrolidin-2-one;
(S)-5-((((3'-chloro-6-methoxy-2'-((S)-5-((6-methoxy-5-(((((R)-5-oxopyrrolidin-2-yl)methyl)amino)methyl)-3-(trifluoromethyl)pyridin-2-yl)amino)-5,6,7,8-tetrahydronaphthalen-1-yl)-[2,4'-bipyridin]-5-yl)methyl)amino)methyl)pyrrolidin-2-one;
(S)-5-((((3'-chloro-6-methoxy-2'-((R)-5-((6-methoxy-5-(((((S)-5-oxopyrrolidin-2-yl)methyl)amino)methyl)-3-(trifluoromethyl)pyridin-2-yl)amino)-5,6,7,8-tetrahydronaphthalen-1-yl)-[2,4'-bipyridin]-5-yl)methyl)amino)methyl)pyrrolidin-2-one;
(S)-5-((((3'-chloro-6-methoxy-2'-((R)-5-((6-methoxy-5-(((((R)-5-oxopyrrolidin-2-yl)methyl)amino)methyl)-3-(trifluoromethyl)pyridin-2-yl)amino)-5,6,7,8-tetrahydronaphthalen-1-yl)-[2,4'-bipyridin]-5-yl)methyl)amino)methyl)pyrrolidin-2-one;
(R)-5-((((3'-chloro-6-methoxy-2'-((R)-5-((6-methoxy-5-(((((R)-5-oxopyrrolidin-2-yl)methyl)amino)methyl)-3-(trifluoromethyl)pyridin-2-yl)amino)-5,6,7,8-tetrahydronaphthalen-1-yl)-[2,4'-bipyridin]-5-yl)methyl)amino)methyl)pyrrolidin-2-one;
(R)-5-((((3'-chloro-6-methoxy-2'-((R)-5-((6-methoxy-5-(((((S)-5-oxopyrrolidin-2-yl)methyl)amino)methyl)-3-(trifluoromethyl)pyridin-2-yl)amino)-5,6,7,8-tetrahydronaphthalen-1-yl)-[2,4'-bipyridin]-5-yl)methyl)amino)methyl)pyrrolidin-2-one;
(R)-5-((((3'-chloro-6-methoxy-2'-((S)-5-((6-methoxy-5-(((((R)-5-oxopyrrolidin-2-yl)methyl)amino)methyl)-3-(trifluoromethyl)pyridin-2-yl)amino)-5,6,7,8-tetrahydronaphthalen-1-yl)-[2,4'-bipyridin]-5-yl)methyl)amino)methyl)pyrrolidin-2-one;
(R)-5-((((3'-chloro-6-methoxy-2'-((S)-5-((6-methoxy-5-(((((S)-5-oxopyrrolidin-2-yl)methyl)amino)methyl)-3-(trifluoromethyl)pyridin-2-yl)amino)-5,6,7,8-tetrahydronaphthalen-1-yl)-[2,4'-bipyridin]-5-yl)methyl)amino)methyl)pyrrolidin-2-one;
2-((6-(((S)-5-(3'-chloro-6-methoxy-5-(((((S)-5-oxopyrrolidin-2-yl)methyl)amino)methyl)-[2,4'-bipyridin]-2'-yl)-1,2,3,4-tetrahydronaphthalen-1-yl)amino)-2-methoxy-5-(trifluoromethyl)pyridin-3-yl)methyl)-2,6-diazaspiro[3.4]octan-7-one;
2-((6-(((S)-5-(3'-chloro-6-methoxy-5-(((((R)-5-oxopyrrolidin-2-yl)methyl)amino)methyl)-[2,4'-bipyridin]-2'-yl)-1,2,3,4-tetrahydronaphthalen-1-yl)amino)-2-methoxy-5-(trifluoromethyl)pyridin-3-yl)methyl)-2,6-diazaspiro[3.4]octan-7-one;
2-((6-(((R)-5-(3'-chloro-6-methoxy-5-(((((S)-5-oxopyrrolidin-2-yl)methyl)amino)methyl)-[2,4'-bipyridin]-2'-yl)-1,2,3,4-tetrahydronaphthalen-1-yl)amino)-2-methoxy-5-(trifluoromethyl)pyridin-3-yl)methyl)-2,6-diazaspiro[3.4]octan-7-one;
2-((6-(((R)-5-(3'-chloro-6-methoxy-5-(((((R)-5-oxopyrrolidin-2-yl)methyl)amino)methyl)-[2,4'-bipyridin]-2'-yl)-1,2,3,4-tetrahydronaphthalen-1-yl)amino)-2-methoxy-5-(trifluoromethyl)pyridin-3-yl)methyl)-2,6-diazaspiro[3.4]octan-7-one;
(S)-N-(5-(3'-chloro-6-methoxy-5-((methylamino)methyl)-[2,4'-bipyridin]-2'-yl)-1,2,3,4-tetrahydronaphthalen-1-yl)-6-methoxy-5-((methylamino)methyl)-3-(trifluoromethyl)pyridin-2-amine;
(R)-N-(5-(3'-chloro-6-methoxy-5-((methylamino)methyl)-[2,4'-bipyridin]-2'-yl)-1,2,3,4-tetrahydronaphthalen-1-yl)-6-methoxy-5-((methylamino)methyl)-3-(trifluoromethyl)pyridin-2-amine;
1-(((6-(((S)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)cyclopropane-1-carboxylic acid;
1-(((6-(((S)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)cyclopropane-1-carboxylic acid;
1-(((6-(((R)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)cyclopropane-1-carboxylic acid;
1-(((6-(((R)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)cyclopropane-1-carboxylic acid;
(S)-5-(((4-(3-chloro-4-((S)-5-((5-((((S)-2-hydroxypropyl)amino)methyl)-6-methoxy-3-(trifluoromethyl)pyridin-2-yl)amino)-5,6,7,8-tetrahydronaphthalen-1-yl)pyridin-2-yl)-2-methoxybenzyl)amino)methyl)pyrrolidin-2-one;
(S)-5-(((4-(3-chloro-4-((S)-5-((5-((((R)-2-hydroxypropyl)amino)methyl)-6-methoxy-3-(trifluoromethyl)pyridin-2-yl)amino)-5,6,7,8-tetrahydronaphthalen-1-yl)pyridin-2-yl)-2-methoxybenzyl)amino)methyl)pyrrolidin-2-one;
(S)-5-(((4-(3-chloro-4-((R)-5-((5-((((S)-2-hydroxypropyl)amino)methyl)-6-methoxy-3-(trifluoromethyl)pyridin-2-yl)amino)-5,6,7,8-tetrahydronaphthalen-1-yl)pyridin-2-yl)-2-methoxybenzyl)amino)methyl)pyrrolidin-2-one;
(S)-5-(((4-(3-chloro-4-((R)-5-((5-((((R)-2-hydroxypropyl)amino)methyl)-6-methoxy-3-(trifluoromethyl)pyridin-2-yl)amino)-5,6,7,8-tetrahydronaphthalen-1-yl)pyridin-2-yl)-2-methoxybenzyl)amino)methyl)pyrrolidin-2-one;
(R)-5-(((4-(3-chloro-4-((R)-5-((5-((((R)-2-hydroxypropyl)amino)methyl)-6-methoxy-3-(trifluoromethyl)pyridin-2-yl)amino)-5,6,7,8-tetrahydronaphthalen-1-yl)pyridin-2-yl)-2-methoxybenzyl)amino)methyl)pyrrolidin-2-one;
(R)-5-(((4-(3-chloro-4-((R)-5-((5-((((S)-2-hydroxypropyl)amino)methyl)-6-methoxy-3-(trifluoromethyl)pyridin-2-yl)amino)-5,6,7,8-tetrahydronaphthalen-1-yl)pyridin-2-yl)-2-methoxybenzyl)amino)methyl)pyrrolidin-2-one;
(R)-5-(((4-(3-chloro-4-((S)-5-((5-((((R)-2-hydroxypropyl)amino)methyl)-6-methoxy-3-(trifluoromethyl)pyridin-2-yl)amino)-5,6,7,8-tetrahydronaphthalen-1-yl)pyridin-2-yl)-2-methoxybenzyl)amino)methyl)pyrrolidin-2-one;
(R)-5-(((4-(3-chloro-4-((S)-5-((5-((((S)-2-hydroxypropyl)amino)methyl)-6-methoxy-3-(trifluoromethyl)pyridin-2-yl)amino)-5,6,7,8-tetrahydronaphthalen-1-yl)pyridin-2-yl)-2-methoxybenzyl)amino)methyl)pyrrolidin-2-one;
(S)-5-((((6-(((S)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)methyl)pyrrolidin-2-one;
(S)-5-((((6-(((S)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)methyl)pyrrolidin-2-one;
(S)-5-((((6-(((R)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)methyl)pyrrolidin-2-one;
(S)-5-((((6-(((R)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)methyl)pyrrolidin-2-one;
(R)-5-((((6-(((R)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)methyl)pyrrolidin-2-one;
(R)-5-((((6-(((R)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)methyl)pyrrolidin-2-one;
(R)-5-((((6-(((S)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)methyl)pyrrolidin-2-one;
(R)-5-((((6-(((S)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)methyl)pyrrolidin-2-one;
2-((6-(((S)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)-2,6-diazaspiro| 3.4] octan-7-one;
2-((6-(((S)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)-2,6-diazaspiro[3.4]octan-7-one;
2-((6-(((R)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)-2,6-diazaspiro[3.4]octan-7-one;
2-((6-(((R)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)-2,6-diazaspiro[3.4]octan-7-one;
(S)-N-(5-(3-chloro-2-(3-methoxy-4-((methylamino)methyl)phenyl)pyridin-4-yl)-1,2,3,4-tetrahydronaphthalen-1-yl)-6-methoxy-5-((methylamino)methyl)-3-(trifluoromethyl)pyridin-2-amine;
(R)-N-(5-(3-chloro-2-(3-methoxy-4-((methylamino)methyl)phenyl)pyridin-4-yl)-1,2,3,4-tetrahydronaphthalen-1-yl)-6-methoxy-5-((methylamino)methyl)-3-(trifluoromethyl)pyridin-2-amine;
1-(((6-(((S)-5-(3'-chloro-6-methoxy-5-(((((S)-5-oxopyrrolidin-2-yl)methyl)amino)methyl)-[2,4'-bipyridin]-2'-yl)-1,2,3,4-tetrahydronaphthalen-1-yl)oxy)-2-methoxy-5-(trifluoromethyl)pyridin-3-yl)methyl)amino)cyclopropane-1-carboxylic acid;
1-(((6-(((S)-5-(3'-chloro-6-methoxy-5-(((((R)-5-oxopyrrolidin-2-yl)methyl)amino)methyl)-[2,4'-bipyridin]-2'-yl)-1,2,3,4-tetrahydronaphthalen-1-yl)oxy)-2-methoxy-5-(trifluoromethyl)pyridin-3-yl)methyl)amino)cyclopropane-1-carboxylic acid;
1-(((6-(((R)-5-(3'-chloro-6-methoxy-5-(((((S)-5-oxopyrrolidin-2-yl)methyl)amino)methyl)-[2,4'-bipyridin]-2'-yl)-1,2,3,4-tetrahydronaphthalen-1-yl)oxy)-2-methoxy-5-(trifluoromethyl)pyridin-3-yl)methyl)amino)cyclopropane-1-carboxylic acid;
1-(((6-(((R)-5-(3'-chloro-6-methoxy-5-(((((R)-5-oxopyrrolidin-2-yl)methyl)amino)methyl)-[2,4'-bipyridin]-2'-yl)-1,2,3,4-tetrahydronaphthalen-1-yl)oxy)-2-methoxy-5-(trifluoromethyl)pyridin-3-yl)methyl)amino)cyclopropane-1-carboxylic acid;
(S)-5-((((3'-chloro-2'-((S)-5-((5-((((S)-2-hydroxypropyl)amino)methyl)-6-methoxy-3-(trifluoromethyl)pyridin-2-yl)oxy)-5,6,7,8-tetrahydronaphthalen-1-yl)-6-methoxy-[2,4'-bipyridin]-5-yl)methyl)amino)methyl)pyrrolidin-2-one;
(S)-5-((((3'-chloro-2'-((S)-5-((5-((((R)-2-hydroxypropyl)amino)methyl)-6-methoxy-3-(trifluoromethyl)pyridin-2-yl)oxy)-5,6,7,8-tetrahydronaphthalen-1-yl)-6-methoxy-[2,4'-bipyridin]-5-yl)methyl)amino)methyl)pyrrolidin-2-one;
(S)-5-((((3'-chloro-2'-((R)-5-((5-((((S)-2-hydroxypropyl)amino)methyl)-6-methoxy-3-(trifluoromethyl)pyridin-2-yl)oxy)-5,6,7,8-tetrahydronaphthalen-1-yl)-6-methoxy-[2,4'-bipyridin]-5-yl)methyl)amino)methyl)pyrrolidin-2-one;
(S)-5-((((3'-chloro-2'-((R)-5-((5-((((R)-2-hydroxypropyl)amino)methyl)-6-methoxy-3-(trifluoromethyl)pyridin-2-yl)oxy)-5,6,7,8-tetrahydronaphthalen-1-yl)-6-methoxy-[2,4'-bipyridin]-5-yl)methyl)amino)methyl)pyrrolidin-2-one;
(R)-5-((((3'-chloro-2'-((R)-5-((5-((((R)-2-hydroxypropyl)amino)methyl)-6-methoxy-3-(trifluoromethyl)pyridin-2-yl)oxy)-5,6,7,8-tetrahydronaphthalen-1-yl)-6-methoxy-[2,4'-bipyridin]-5-yl)methyl)amino)methyl)pyrrolidin-2-one;
(R)-5-((((3'-chloro-2'-((R)-5-((5-((((S)-2-hydroxypropyl)amino)methyl)-6-methoxy-3-(trifluoromethyl)pyridin-2-yl)oxy)-5,6,7,8-tetrahydronaphthalen-1-yl)-6-methoxy-[2,4'-bipyridin]-5-yl)methyl)amino)methyl)pyrrolidin-2-one;
(R)-5-((((3'-chloro-2'-((S)-5-((5-((((R)-2-hydroxypropyl)amino)methyl)-6-methoxy-3-(trifluoromethyl)pyridin-2-yl)oxy)-5,6,7,8-tetrahydronaphthalen-1-yl)-6-methoxy-[2,4'-bipyridin]-5-yl)methyl)amino)methyl)pyrrolidin-2-one;
(R)-5-((((3'-chloro-2'-((S)-5-((5-((((S)-2-hydroxypropyl)amino)methyl)-6-methoxy-3-(trifluoromethyl)pyridin-2-yl)oxy)-5,6,7,8-tetrahydronaphthalen-1-yl)-6-methoxy-[2,4'-bipyridin]-5-yl)methyl)amino)methyl)pyrrolidin-2-one;
(S)-5-((((3'-chloro-6-methoxy-2'-((S)-5-((6-methoxy-5-(((((S)-5-oxopyrrolidin-2-yl)methyl)amino)methyl)-3-(trifluoromethyl)pyridin-2-yl)oxy)-5,6,7,8-tetrahydronaphthalen-1-yl)-[2,4'-bipyridin]-5-yl)methyl)amino)methyl)pyrrolidin-2-one;
(S)-5-((((3'-chloro-6-methoxy-2'-((S)-5-((6-methoxy-5-(((((R)-5-oxopyrrolidin-2-yl)methyl)amino)methyl)-3-(trifluoromethyl)pyridin-2-yl)oxy)-5,6,7,8-tetrahydronaphthalen-1-yl)-[2,4'-bipyridin]-5-yl)methyl)amino)methyl)pyrrolidin-2-one;
(S)-5-((((3'-chloro-6-methoxy-2'-((R)-5-((6-methoxy-5-(((((S)-5-oxopyrrolidin-2-yl)methyl)amino)methyl)-3-(trifluoromethyl)pyridin-2-yl)oxy)-5,6,7,8-tetrahydronaphthalen-1-yl)-[2,4'-bipyridin]-5-yl)methyl)amino)methyl)pyrrolidin-2-one;
(S)-5-((((3'-chloro-6-methoxy-2'-((R)-5-((6-methoxy-5-(((((R)-5-oxopyrrolidin-2-yl)methyl)amino)methyl)-3-(trifluoromethyl)pyridin-2-yl)oxy)-5,6,7,8-tetrahydronaphthalen-1-yl)-[2,4'-bipyridin]-5-yl)methyl)amino)methyl)pyrrolidin-2-one;
(R)-5-((((3'-chloro-6-methoxy-2'-((R)-5-((6-methoxy-5-(((((R)-5-oxopyrrolidin-2-yl)methyl)amino)methyl)-3-(trifluoromethyl)pyridin-2-yl)oxy)-5,6,7,8-tetrahydronaphthalen-1-yl)-[2,4'-bipyridin]-5-yl)methyl)amino)methyl)pyrrolidin-2-one;
(R)-5-((((3'-chloro-6-methoxy-2'-((R)-5-((6-methoxy-5-(((((S)-5-oxopyrrolidin-2-yl)methyl)amino)methyl)-3-(trifluoromethyl)pyridin-2-yl)oxy)-5,6,7,8-tetrahydronaphthalen-1-yl)-[2,4'-bipyridin]-5-yl)methyl)amino)methyl)pyrrolidin-2-one;
(R)-5-((((3'-chloro-6-methoxy-2'-((S)-5-((6-methoxy-5-(((((R)-5-oxopyrrolidin-2-yl)methyl)amino)methyl)-3-(trifluoromethyl)pyridin-2-yl)oxy)-5,6,7,8-tetrahydronaphthalen-1-yl)-[2,4'-bipyridin]-5-yl)methyl)amino)methyl)pyrrolidin-2-one;
(R)-5-((((3'-chloro-6-methoxy-2'-((S)-5-((6-methoxy-5-(((((S)-5-oxopyrrolidin-2-yl)methyl)amino)methyl)-3-(trifluoromethyl)pyridin-2-yl)oxy)-5,6,7,8-tetrahydronaphthalen-1-yl)-[2,4'-bipyridin]-5-yl)methyl)amino)methyl)pyrrolidin-2-one;
2-((6-(((S)-5-(3'-chloro-6-methoxy-5-(((((S)-5-oxopyrrolidin-2-yl)methyl)amino)methyl)-[2,4'-bipyridin]-2'-yl)-1,2,3,4-tetrahydronaphthalen-1-yl)oxy)-2-methoxy-5-(trifluoromethyl)pyridin-3-yl)methyl)-2,6-diazaspiro[3.4]octan-7-one;
2-((6-(((S)-5-(3'-chloro-6-methoxy-5-(((((R)-5-oxopyrrolidin-2-yl)methyl)amino)methyl)-[2,4'-bipyridin]-2'-yl)-1,2,3,4-tetrahydronaphthalen-1-yl)oxy)-2-methoxy-5-(trifluoromethyl)pyridin-3-yl)methyl)-2,6-diazaspiro[3.4]octan-7-one;
2-((6-(((S)-5-(3'-chloro-6-methoxy-5-(((((S)-5-oxopyrrolidin-2-yl)methyl)amino)methyl)-[2,4'-bipyridin]-2'-yl)-1,2,3,4-tetrahydronaphthalen-1-yl)oxy)-2-methoxy-5-(trifluoromethyl)pyridin-3-yl)methyl)-2,6-diazaspiro[3.4]octan-7-one;
2-((6-(((R)-5-(3'-chloro-6-methoxy-5-(((((R)-5-oxopyrrolidin-2-yl)methyl)amino)methyl)-[2,4'-bipyridin]-2'-yl)-1,2,3,4-tetrahydronaphthalen-1-yl)oxy)-2-methoxy-5-(trifluoromethyl)pyridin-3-yl)methyl)-2,6-diazaspiro[3.4]octan-7-one;
(S)-1-(3'-chloro-6-methoxy-2'-(5-((6-methoxy-5-((methylamino)methyl)-3-(trifluoromethyl)pyridin-2-yl)oxy)-5,6,7,8-tetrahydronaphthalen-l-yl)-[2,4'-bipyridin]-5-yl)-N-methylmethanamine;
(R)-1-(3'-chloro-6-methoxy-2'-(5-((6-methoxy-5-((methylamino)methyl)-3-(trifluoromethyl)pyridin-2-yl)oxy)-5,6,7,8-tetrahydronaphthalen-1-yl)-[2,4'-bipyridin]-5-yl)-N-methylmethanamine;
1-(((6-(((S)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)oxy)-2-methoxy-5-(trifluoromethyl)pyridin-3-yl)methyl)amino)cyclopropane-1-carboxylic acid;
1-(((6-(((S)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)oxy)-2-methoxy-5-(trifluoromethyl)pyridin-3-yl)methyl)amino)cyclopropane-1-carboxylic acid;
1-(((6-(((R)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)oxy)-2-methoxy-5-(trifluoromethyl)pyridin-3-yl)methyl)amino)cyclopropane-1-carboxylic acid;
1-(((6-(((R)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)oxy)-2-methoxy-5-(trifluoromethyl)pyridin-3-yl)methyl)amino)cyclopropane-1-carboxylic acid;
(S)-5-(((4-(3-chloro-4-((S)-5-((5-((((S)-2-hydroxypropyl)amino)methyl)-6-methoxy-3-(trifluoromethyl)pyridin-2-yl)oxy)-5,6,7,8-tetrahydronaphthalen-1-yl)pyridin-2-yl)-2-methoxybenzyl)amino)methyl)pyrrolidin-2-one;
(S)-5-(((4-(3-chloro-4-((S)-5-((5-((((R)-2-hydroxypropyl)amino)methyl)-6-methoxy-3-(trifluoromethyl)pyridin-2-yl)oxy)-5,6,7,8-tetrahydronaphthalen-1-yl)pyridin-2-yl)-2-methoxybenzyl)amino)methyl)pyrrolidin-2-one;
(S)-5-(((4-(3-chloro-4-((R)-5-((5-((((S)-2-hydroxypropyl)amino)methyl)-6-methoxy-3-(trifluoromethyl)pyridin-2-yl)oxy)-5,6,7,8-tetrahydronaphthalen-1-yl)pyridin-2-yl)-2-methoxybenzyl)amino)methyl)pyrrolidin-2-one;
(S)-5-(((4-(3-chloro-4-((R)-5-((5-((((R)-2-hydroxypropyl)amino)methyl)-6-methoxy-3-(trifluoromethyl)pyridin-2-yl)oxy)-5,6,7,8-tetrahydronaphthalen-1-yl)pyridin-2-yl)-2-methoxybenzyl)amino)methyl)pyrrolidin-2-one;
(R)-5-(((4-(3-chloro-4-((R)-5-((5-((((R)-2-hydroxypropyl)amino)methyl)-6-methoxy-3-(trifluoromethyl)pyridin-2-yl)oxy)-5,6,7,8-tetrahydronaphthalen-1-yl)pyridin-2-yl)-2-methoxybenzyl)amino)methyl)pyrrolidin-2-one;
(R)-5-(((4-(3-chloro-4-((R)-5-((5-((((S)-2-hydroxypropyl)amino)methyl)-6-methoxy-3-(trifluoromethyl)pyridin-2-yl)oxy)-5,6,7,8-tetrahydronaphthalen-1-yl)pyridin-2-yl)-2-methoxybenzyl)amino)methyl)pyrrolidin-2-one;
(R)-5-(((4-(3-chloro-4-((S)-5-((5-((((R)-2-hydroxypropyl)amino)methyl)-6-methoxy-3-(trifluoromethyl)pyridin-2-yl)oxy)-5,6,7,8-tetrahydronaphthalen-1-yl)pyridin-2-yl)-2-methoxybenzyl)amino)methyl)pyrrolidin-2-one;
(R)-5-(((4-(3-chloro-4-((S)-5-((5-((((S)-2-hydroxypropyl)amino)methyl)-6-methoxy-3-(trifluoromethyl)pyridin-2-yl)oxy)-5,6,7,8-tetrahydronaphthalen-1-yl)pyridin-2-yl)-2-methoxybenzyl)amino)methyl)pyrrolidin-2-one;
(S)-5-((((6-(((S)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)oxy)-2-methoxy-5-(trifluoromethyl)pyridin-3-yl)methyl)amino)methyl)pyrrolidin-2-one;
(S)-5-((((6-(((S)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)oxy)-2-methoxy-5-(trifluoromethyl)pyridin-3-yl)methyl)amino)methyl)pyrrolidin-2-one;
(S)-5-((((6-(((R)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)oxy)-2-methoxy-5-(trifluoromethyl)pyridin-3-yl)methyl)amino)methyl)pyrrolidin-2-one;
(S)-5-((((6-(((R)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)oxy)-2-methoxy-5-(trifluoromethyl)pyridin-3-yl)methyl)amino)methyl)pyrrolidin-2-one;
(R)-5-((((6-(((R)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)oxy)-2-methoxy-5-(trifluoromethyl)pyridin-3-yl)methyl)amino)methyl)pyrrolidin-2-one;
(R)-5-((((6-(((R)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)oxy)-2-methoxy-5-(trifluoromethyl)pyridin-3-yl)methyl)amino)methyl)pyrrolidin-2-one;
(R)-5-((((6-(((S)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)oxy)-2-methoxy-5-(trifluoromethyl)pyridin-3-yl)methyl)amino)methyl)pyrrolidin-2-one;
(R)-5-((((6-(((S)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)oxy)-2-methoxy-5-(trifluoromethyl)pyridin-3-yl)methyl)amino)methyl)pyrrolidin-2-one;
2-((6-(((S)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)oxy)-2-methoxy-5-(trifluoromethyl)pyridin-3-yl)methyl)-2,6-diazaspiro[3.4]octan-7-one;
2-((6-(((S)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)oxy)-2-methoxy-5-(trifluoromethyl)pyridin-3-yl)methyl)-2,6-diazaspiro[3.4]octan-7-one;
2-((6-(((R)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)oxy)-2-methoxy-5-(trifluoromethyl)pyridin-3-yl)methyl)-2,6-diazaspiro[3.4]octan-7-one;
2-((6-(((R)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)oxy)-2-methoxy-5-(trifluoromethyl)pyridin-3-yl)methyl)-2,6-diazaspiro[3.4]octan-7-one;
(S)-1-(6-((5-(3-chloro-2-(3-methoxy-4-((methylamino)methyl)phenyl)pyridin-4-yl)-1,2,3,4-tetrahydronaphthalen-1-yl)oxy)-2-methoxy-5-(trifluoromethyl)pyridin-3-yl)-N-methylmethanamine;
(R)-1-(6-((5-(3-chloro-2-(3-methoxy-4-((methylamino)methyl)phenyl)pyridin-4-yl)-1,2,3,4-tetrahydronaphthalen-1-yl)oxy)-2-methoxy-5-(trifluoromethyl)pyridin-3-yl)-N-methylmethanamine;
(S)-5-(((4-(3-chloro-4-(2-chloro-3-(8-chloro-6-((((S)-2-hydroxypropyl)amino)methyl)-5-methoxyquinolin-2-yl)phenyl)pyridin-2-yl)-2-methoxybenzyl)amino)methyl)pyrrolidin-2-one;
(S)-5-(((4-(3-chloro-4-(2-chloro-3-(8-chloro-6-((((R)-2-hydroxypropyl)amino)methyl)-5-methoxyquinolin-2-yl)phenyl)pyridin-2-yl)-2-methoxybenzyl)amino)methyl)pyrrolidin-2-one;
(R)-5-(((4-(3-chloro-4-(2-chloro-3-(8-chloro-6-((((S)-2-hydroxypropyl)amino)methyl)-5-methoxyquinolin-2-yl)phenyl)pyridin-2-yl)-2-methoxybenzyl)amino)methyl)pyrrolidin-2-one;
(R)-5-(((4-(3-chloro-4-(2-chloro-3-(8-chloro-6-((((R)-2-hydroxypropyl)amino)methyl)-5-methoxyquinolin-2-yl)phenyl)pyridin-2-yl)-2-methoxybenzyl)amino)methyl)pyrrolidin-2-one;
(S)-5-((((8-chloro-2-(2-chloro-3-(3-chloro-2-(3-methoxy-4-(((((S)-5-oxopyrrolidin-2-yl)methyl)amino)methyl)phenyl)pyridin-4-yl)phenyl)-5-methoxyquinolin-6-yl)methyl)amino)methyl)pyrrolidin-2-one;
(S)-5-((((8-chloro-2-(2-chloro-3-(3-chloro-2-(3-methoxy-4-(((((R)-5-oxopyrrolidin-2-yl)methyl)amino)methyl)phenyl)pyridin-4-yl)phenyl)-5-methoxyquinolin-6-yl)methyl)amino)methyl)pyrrolidin-2-one;
(R)-5-((((8-chloro-2-(2-chloro-3-(3-chloro-2-(3-methoxy-4-(((((S)-5-oxopyrrolidin-2-yl)methyl)amino)methyl)phenyl)pyridin-4-yl)phenyl)-5-methoxyquinolin-6-yl)methyl)amino)methyl)pyrrolidin-2-one;
(R)-5-((((8-chloro-2-(2-chloro-3-(3-chloro-2-(3-methoxy-4-(((((R)-5-oxopyrrolidin-2-yl)methyl)amino)methyl)phenyl)pyridin-4-yl)phenyl)-5-methoxyquinolin-6-yl)methyl)amino)methyl)pyrrolidin-2-one;
(S)-5-((((6-(2-chloro-3-(3-chloro-2-(8-chloro-2-(3-hydroxy-3-methylbutyl)-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(8-chloro-2-(3-hydroxy-3-methylbutyl)-1,2,3,4-tetrahydroisoquinolin-6-yl)pyridin-4-yl)phenyl)-2-methoxypyridin-3-yl)methyl)amino)methyl)pyrrolidin-2-one;
isopropyl (S)-4-((4-(3-chloro-4-(2-chloro-3-(5-(4,5-dihydro-1H-imidazol-2-yl)-6-methoxypyridin-2-yl)phenyl)pyridin-2-yl)-2-methoxybenzyl)amino)-3-hydroxybutanoate;
isopropyl (R)-4-((4-(3-chloro-4-(2-chloro-3-(5-(4,5-dihydro-1H-imidazol-2-yl)-6-methoxypyridin-2-yl)phenyl)pyridin-2-yl)-2-methoxybenzyl)amino)-3-hydroxybutanoate;
(S)-5-((((6-(3-(2-(4-(((2-acetyl-2-azaspiro[3.3]heptan-6-yl)amino)methyl)-3-fluoro-5-methoxyphenyl)-3-chloropyridin-4-yl)-2-chlorophenyl)-2-methoxypyridin-3-yl)methyl)amino)methyl)pyrrolidin-2-one;
(R)-5-((((6-(3-(2-(4-(((2-acetyl-2-azaspiro[3.3]heptan-6-yl)amino)methyl)-3-fluoro-5-methoxyphenyl)-3-chloropyridin-4-yl)-2-chlorophenyl)-2-methoxypyridin-3-yl)methyl)amino)methyl)pyrrolidin-2-one;
N-(4-(3-chloro-4-(2-chloro-3-(6-methoxy-5-((methylamino)methyl)pyridin-2-yl)phenyl)pyridin-2-yl)-2-methoxybenzyl)propan-2-amine;
1-(4-((4-(3-chloro-4-(2-chloro-3-(6-methoxy-5-((methylamino)methyl)pyridin-2-yl)phenyl)pyridin-2-yl)-2-methoxybenzyl)amino)piperidin-1-yl)ethan-1-one;
2-(4-(3-chloro-4-(2-chloro-3-(6-methoxy-5-((methylamino)methyl)pyridin-2-yl)phenyl)pyridin-2-yl)-2-methoxybenzyl)-2,6-diazaspiro[3.4]octan-7-one;
(S)-1-(((6-(2-chloro-3-(3-chloro-2-(2-((S)-2-hydroxypropyl)-8-methoxy-1,2,3,4-tetrahydroisoquinolin-6-yl)pyridin-4-yl)phenyl)-2-methoxypyridin-3-yl)methyl)amino)propan-2-ol;
(S)-1-(((6-(2-chloro-3-(3-chloro-2-(2-((R)-2-hydroxypropyl)-8-methoxy-1,2,3,4-tetrahydroisoquinolin-6-yl)pyridin-4-yl)phenyl)-2-methoxypyridin-3-yl)methyl)amino)propan-2-ol;
(R)-1-(((6-(2-chloro-3-(3-chloro-2-(2-((S)-2-hydroxypropyl)-8-methoxy-1,2,3,4-tetrahydroisoquinolin-6-yl)pyridin-4-yl)phenyl)-2-methoxypyridin-3-yl)methyl)amino)propan-2-ol;
(R)-1-(((6-(2-chloro-3-(3-chloro-2-(2-((R)-2-hydroxypropyl)-8-methoxy-1,2,3,4-tetrahydroisoquinolin-6-yl)pyridin-4-yl)phenyl)-2-methoxypyridin-3-yl)methyl)amino)propan-2-ol;
(1r,3r)-3-(((6-(2-chloro-3-(3-chloro-2-(2-((S)-2-hydroxypropyl)-8-methoxy-1,2,3,4-tetrahydroisoquinolin-6-yl)pyridin-4-yl)phenyl)-2-methoxypyridin-3-yl)methyl)amino)-1-methylcyclobutan-1-ol;
(1r,3r)-3-(((6-(2-chloro-3-(3-chloro-2-(2-((R)-2-hydroxypropyl)-8-methoxy-1,2,3,4-tetrahydroisoquinolin-6-yl)pyridin-4-yl)phenyl)-2-methoxypyridin-3-yl)methyl)amino)-1-methylcyclobutan-1-ol;
(1r,3s)-3-(((6-(2-chloro-3-(3-chloro-2-(2-((S)-2-hydroxypropyl)-8-methoxy-1,2,3,4-tetrahydroisoquinolin-6-yl)pyridin-4-yl)phenyl)-2-methoxypyridin-3-yl)methyl)amino)-1-methylcyclobutan-1-ol;
(1s,3s)-3-(((6-(2-chloro-3-(3-chloro-2-(2-((S)-2-hydroxypropyl)-8-methoxy-1,2,3,4-tetrahydroisoquinolin-6-yl)pyridin-4-yl)phenyl)-2-methoxypyridin-3-yl)methyl)amino)-1-methylcyclobutan-1-ol;
(1s,3s)-3-(((6-(2-chloro-3-(3-chloro-2-(2-((R)-2-hydroxypropyl)-8-methoxy-1,2,3,4-tetrahydroisoquinolin-6-yl)pyridin-4-yl)phenyl)-2-methoxypyridin-3-yl)methyl)amino)-1-methylcyclobutan-1-ol;
(1s,3r)-3-(((6-(2-chloro-3-(3-chloro-2-(2-((R)-2-hydroxypropyl)-8-methoxy-1,2,3,4-tetrahydroisoquinolin-6-yl)pyridin-4-yl)phenyl)-2-methoxypyridin-3-yl)methyl)amino)-1-methylcyclobutan-1-ol;
(1s,3r)-3-(((6-(2-chloro-3-(3-chloro-2-(2-((S)-2-hydroxypropyl)-8-methoxy-1,2,3,4-tetrahydroisoquinolin-6-yl)pyridin-4-yl)phenyl)-2-methoxypyridin-3-yl)methyl)amino)-1-methylcyclobutan-1-ol;
(1r,3s)-3-(((6-(2-chloro-3-(3-chloro-2-(2-((R)-2-hydroxypropyl)-8-methoxy-1,2,3,4-tetrahydroisoquinolin-6-yl)pyridin-4-yl)phenyl)-2-methoxypyridin-3-yl)methyl)amino)-1-methylcyclobutan-1-ol;
(S)-1-(6-(3-chloro-4-(2-chloro-3-(6-methoxy-5-(((((S)-tetrahydrofuran-2-yl)methyl)amino)methyl)pyridin-2-yl)phenyl)pyridin-2-yl)-8-methoxy-3,4-dihydroisoquinolin-2(1H)-yl)propan-2-ol;
(S)-1-(6-(3-chloro-4-(2-chloro-3-(6-methoxy-5-(((((R)-tetrahydrofuran-2-yl)methyl)amino)methyl)pyridin-2-yl)phenyl)pyridin-2-yl)-8-methoxy-3,4-dihydroisoquinolin-2(1H)-yl)propan-2-ol;
(R)-1-(6-(3-chloro-4-(2-chloro-3-(6-methoxy-5-(((((S)-tetrahydrofuran-2-yl)methyl)amino)methyl)pyridin-2-yl)phenyl)pyridin-2-yl)-8-methoxy-3,4-dihydroisoquinolin-2(1H)-yl)propan-2-ol;
(R)-1-(6-(3-chloro-4-(2-chloro-3-(6-methoxy-5-(((((R)-tetrahydrofuran-2-yl)methyl)amino)methyl)pyridin-2-yl)phenyl)pyridin-2-yl)-8-methoxy-3,4-dihydroisoquinolin-2(1H)-yl)propan-2-ol;
(S)-1-((6-(2-chloro-3-(3-chloro-2-(2-(2-hydroxypropyl)-8-methoxy-1,2,3,4-tetrahydroisoquinolin-6-yl)pyridin-4-yl)phenyl)-2-methoxypyridin-3-yl)methyl)-3-methylazetidin-3-ol;
(R)-1-((6-(2-chloro-3-(3-chloro-2-(2-(2-hydroxypropyl)-8-methoxy-1,2,3,4-tetrahydroisoquinolin-6-yl)pyridin-4-yl)phenyl)-2-methoxypyridin-3-yl)methyl)-3-methylazetidin-3-ol;
methyl (S)-((6-(2-chloro-3-(3-chloro-2-(2-(2-hydroxypropyl)-8-methoxy-1,2,3,4-tetrahydroisoquinolin-6-yl)pyridin-4-yl)phenyl)-2-methoxypyridin-3-yl)methyl)glycinate;
methyl (R)-((6-(2-chloro-3-(3-chloro-2-(2-(2-hydroxypropyl)-8-methoxy-1,2,3,4-tetrahydroisoquinolin-6-yl)pyridin-4-yl)phenyl)-2-methoxypyridin-3-yl)methyl)glycinate;
(S)-2-((6-(2-chloro-3-(3-chloro-2-(3-methoxy-4-((((5-oxopyrrolidin-2-yl)methyl)amino)methyl)phenyl)pyridin-4-yl)phenyl)-1-methyl-1H-pyrrolo[2,3-b]pyridin-3-yl)methyl)-2,6-diazaspiro[3.4]octan-7-one;
(R)-2-((6-(2-chloro-3-(3-chloro-2-(3-methoxy-4-((((5-oxopyrrolidin-2-yl)methyl)amino)methyl)phenyl)pyridin-4-yl)phenyl)-1-methyl-1H-pyrrolo[2,3-b]pyridin-3-yl)methyl)-2,6-diazaspiro[3.4]octan-7-one;
(S)-4-((4-(3-chloro-4-(2-chloro-3-(6-methoxy-5-((methylamino)methyl)pyridin-2-yl)phenyl)pyridin-2-yl)-2-methoxybenzyl)amino)-3-hydroxybutanoic acid;
(R)-4-((4-(3-chloro-4-(2-chloro-3-(6-methoxy-5-((methylamino)methyl)pyridin-2-yl)phenyl)pyridin-2-yl)-2-methoxybenzyl)amino)-3-hydroxybutanoic acid;
3-((4-(3-chloro-4-(2-chloro-3-(6-methoxy-5-((methylamino)methyl)pyridin-2-yl)phenyl)pyridin-2-yl)-2-methoxybenzyl)amino)propanoic acid;
6-(3-chloro-4-(2-chloro-3-(5-((6-hydroxy-2-azaspiro[3.3]heptan-2-yl)methyl)-6-methoxypyridin-2-yl)phenyl)pyridin-2-yl)-2-((3-hydroxy-3-methylazetidin-1-yl)methyl)-3-methylpyrrolo[2,1-f][1,2,4]triazin-4(3H)-one;
(S)-5-((((3'-chloro-6-methoxy-2'-((S)-1-((6-methoxy-5-(((((S)-5-oxopyrrolidin-2-yl)methyl)amino)methyl)-3-(trifluoromethyl)pyridin-2-yl)amino)-2,3-dihydro-1H-inden-4-yl)-[2,4'-bipyridin]-5-yl)methyl)amino)methyl)pyrrolidin-2-one;
(R)-5-((((3'-chloro-6-methoxy-2'-((R)-1-((6-methoxy-5-(((((R)-5-oxopyrrolidin-2-yl)methyl)amino)methyl)-3-(trifluoromethyl)pyridin-2-yl)amino)-2,3-dihydro-1H-inden-4-yl)-[2,4'-bipyridin]-5-yl)methyl)amino)methyl)pyrrolidin-2-one;
(R)-5-((((3'-chloro-6-methoxy-2'-((R)-1-((6-methoxy-5-(((((S)-5-oxopyrrolidin-2-yl)methyl)amino)methyl)-3-(trifluoromethyl)pyridin-2-yl)amino)-2,3-dihydro-1H-inden-4-yl)-[2,4'-bipyridin]-5-yl)methyl)amino)methyl)pyrrolidin-2-one;
(R)-5-((((3'-chloro-6-methoxy-2'-((S)-1-((6-methoxy-5-(((((S)-5-oxopyrrolidin-2-yl)methyl)amino)methyl)-3-(trifluoromethyl)pyridin-2-yl)amino)-2,3-dihydro-1H-inden-4-yl)-[2,4'-bipyridin]-5-yl)methyl)amino)methyl)pyrrolidin-2-one;
(R)-5-((((3'-chloro-6-methoxy-2'-((S)-1-((6-methoxy-5-(((((R)-5-oxopyrrolidin-2-yl)methyl)amino)methyl)-3-(trifluoromethyl)pyridin-2-yl)amino)-2,3-dihydro-1H-inden-4-yl)-[2,4'-bipyridin]-5-yl)methyl)amino)methyl)pyrrolidin-2-one;
(S)-5-((((3'-chloro-6-methoxy-2'-((S)-1-((6-methoxy-5-(((((R)-5-oxopyrrolidin-2-yl)methyl)amino)methyl)-3-(trifluoromethyl)pyridin-2-yl)amino)-2,3-dihydro-1H-inden-4-yl)-[2,4'-bipyridin]-5-yl)methyl)amino)methyl)pyrrolidin-2-one;
(S)-5-((((3'-chloro-6-methoxy-2'-((R)-1-((6-methoxy-5-(((((R)-5-oxopyrrolidin-2-yl)methyl)amino)methyl)-3-(trifluoromethyl)pyridin-2-yl)amino)-2,3-dihydro-1H-inden-4-yl)-[2,4'-bipyridin]-5-yl)methyl)amino)methyl)pyrrolidin-2-one;
(S)-5-((((3'-chloro-6-methoxy-2'-((R)-1-((6-methoxy-5-(((((S)-5-oxopyrrolidin-2-yl)methyl)amino)methyl)-3-(trifluoromethyl)pyridin-2-yl)amino)-2,3-dihydro-1H-inden-4-yl)-[2,4'-bipyridin]-5-yl)methyl)amino)methyl)pyrrolidin-2-one;
(S)-N-(4-(3'-chloro-6-methoxy-5-((methylamino)methyl)-[2,4'-bipyridin]-2'-yl)-2,3-dihydro-1H-inden-1-yl)-6-methoxy-5-((methylamino)methyl)-3-(trifluoromethyl)pyridin-2-amine;
(R)-N-(4-(3'-chloro-6-methoxy-5-((methylamino)methyl)-[2,4'-bipyridin]-2'-yl)-2,3-dihydro-1H-inden-1-yl)-6-methoxy-5-((methylamino)methyl)-3-(trifluoromethyl)pyridin-2-amine;
(1S,3r)-3-(((3'-chloro-2'-((S)-1-((5-((((1r,3S)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1R,3r)-3-(((3'-chloro-2'-((R)-1-((5-((((1r,3R)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1R,3r)-3-(((3'-chloro-2'-((R)-1-((5-((((1r,3S)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1R,3r)-3-(((3'-chloro-2'-((R)-1-((5-((((1s,3R)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1R,3r)-3-(((3'-chloro-2'-((R)-1-((5-((((1s,3S)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1R,3r)-3-(((3'-chloro-2'-((S)-1-((5-((((1r,3R)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1R,3r)-3-(((3'-chloro-2'-((S)-1-((5-((((1r,3S)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1R,3r)-3-(((3'-chloro-2'-((S)-1-((5-((((1s,3R)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1R,3r)-3-(((3'-chloro-2'-((S)-1-((5-((((1s,3S)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1R,3s)-3-(((3'-chloro-2'-((R)-1-((5-((((1r,3R)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1R,3s)-3-(((3'-chloro-2'-((R)-1-((5-((((1r,3S)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1R,3s)-3-(((3'-chloro-2'-((R)-1-((5-((((1s,3R)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1R,3s)-3-(((3'-chloro-2'-((R)-1-((5-((((1s,3S)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1R,3r)-3-(((3'-chloro-2'-((R)-1-((5-((((1r,3R)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1R,3s)-3-(((3'-chloro-2'-((S)-1-((5-((((1r,3S)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1R,3s)-3-(((3'-chloro-2'-((S)-1-((5-((((1s,3R)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1R,3s)-3-(((3'-chloro-2'-((S)-1-((5-((((1s,3S)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1S,3r)-3-(((3'-chloro-2'-((R)-1-((5-((((1r,3R)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1S,3r)-3-(((3'-chloro-2'-((R)-1-((5-((((1r,3S)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1S,3r)-3-(((3'-chloro-2'-((R)-1-((5-((((1s,3R)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1S,3r)-3-(((3'-chloro-2'-((R)-1-((5-((((1s,3S)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1S,3r)-3-(((3'-chloro-2'-((S)-1-((5-((((1r,3R)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1S,3r)-3-(((3'-chloro-2'-((S)-1-((5-((((1s,3R)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1S,3r)-3-(((3'-chloro-2'-((S)-1-((5-((((1s,3S)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1S,3s)-3-(((3'-chloro-2'-((R)-1-((5-((((1r,3R)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1S,3s)-3-(((3'-chloro-2'-((R)-1-((5-((((1r,3S)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1S,3s)-3-(((3'-chloro-2'-((R)-1-((5-((((1s,3R)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1S,3s)-3-(((3'-chloro-2'-((R)-1-((5-((((1s,3S)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1S,3r)-3-(((3'-chloro-2'-((R)-1-((5-((((1r,3R)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1S,3s)-3-(((3'-chloro-2'-((S)-1-((5-((((1r,3S)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1S,3s)-3-(((3'-chloro-2'-((S)-1-((5-((((1s,3R)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1S,3s)-3-(((3'-chloro-2'-((S)-1-((5-((((1s,3S)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(S)-1-(4-(3-chloro-4-(2-chloro-3-(6-methoxy-5-((methylamino)methyl)pyridin-2-yl)phenyl)pyridin-2-yl)-2-methoxybenzyl)pyrrolidine-3-carboxylic acid;
(R)-1-(4-(3-chloro-4-(2-chloro-3-(6-methoxy-5-((methylamino)methyl)pyridin-2-yl)phenyl)pyridin-2-yl)-2-methoxybenzyl)pyrrolidine-3-carboxylic acid;
2-(4-(3-chloro-4-(2-chloro-3-(6-methoxy-5-((methylamino)methyl)pyridin-2-yl)phenyl)pyridin-2-yl)-2-methoxybenzyl)-2-azaspiro[3.3]heptane-6-carboxylic acid;
3-(((4-(3-chloro-4-(2-chloro-3-(6-methoxy-5-((methylamino)methyl)pyridin-2-yl)phenyl)pyridin-2-yl)-2-methoxybenzyl)amino)methyl)bicyclo[1.1.1]pentane-1-carboxylic acid;
(S)-((6-(2-chloro-3-(3-chloro-2-(2-(2-hydroxypropyl)-8-methoxy-1,2,3,4-tetrahydroisoquinolin-6-yl)pyridin-4-yl)phenyl)-2-methoxypyridin-3-yl)methyl)glycine;
(R)-((6-(2-chloro-3-(3-chloro-2-(2-(2-hydroxypropyl)-8-methoxy-1,2,3,4-tetrahydroisoquinolin-6-yl)pyridin-4-yl)phenyl)-2-methoxypyridin-3-yl)methyl)glycine;
2-((6-(2-chloro-3-(3-chloro-2-(isoindolin-5-yl)pyridin-4-yl)phenyl)-2-methoxypyridin-3-yl)methyl)-2,6-diazaspiro[3.4]octan-7-one;
6-(3-chloro-4-(2-chloro-3-(5-((3-hydroxy-3-methylazetidin-1-yl)methyl)-6-methoxypyridin-2-yl)phenyl)pyridin-2-yl)-2-((3-hydroxy-3-methylazetidin-1-yl)methyl)-3-methylpyrrolo[2,1-f][1,2,4]triazin-4(3H)-one;
6-(3-chloro-4-(2-chloro-3-(5-((((1r,3r)-3-hydroxy-3-methylcyclobutyl)amino)methyl)-6-methoxypyridin-2-yl)phenyl)pyridin-2-yl)-2-((3-hydroxy-3-methylazetidin-1-yl)methyl)-3-methylpyrrolo[2,1-f][1,2,4 |triazin-4(3H)-one;
6-(3-chloro-4-(2-chloro-3-(5-((((1r,3s)-3-hydroxy-3-methylcyclobutyl)amino)methyl)-6-methoxypyridin-2-yl)phenyl)pyridin-2-yl)-2-((3-hydroxy-3-methylazetidin-1-yl)methyl)-3-methylpyrrolo[2,1-f][1,2,4]triazin-4(3H)-one;
6-(3-chloro-4-(2-chloro-3-(5-((((1s,3r)-3-hydroxy-3-methylcyclobutyl)amino)methyl)-6-methoxypyridin-2-yl)phenyl)pyridin-2-yl)-2-((3-hydroxy-3-methylazetidin-1-yl)methyl)-3-methylpyrrolo[2,1-f][1,2,4 |triazin-4(3H)-one;
6-(3-chloro-4-(2-chloro-3-(5-((((1s,3s)-3-hydroxy-3-methylcyclobutyl)amino)methyl)-6-methoxypyridin-2-yl)phenyl)pyridin-2-yl)-2-((3-hydroxy-3-methylazetidin-1-yl)methyl)-3-methylpyrrolo[2,1-f][1,2,4]triazin-4(3H)-one;
6-(3-chloro-4-(2-chloro-3-(5-((isopropylamino)methyl)-6-methoxypyridin-2-yl)phenyl)pyridin-2-yl)-2-((3-hydroxy-3-methylazetidin-1-yl)methyl)-3-methylpyrrolo[2,1-f][1,2,4]triazin-4(3H)-one;
6-(3-chloro-4-(2-chloro-3-(5-((((1r,4r)-4-hydroxycyclohexyl)amino)methyl)-6-methoxypyridin-2-yl)phenyl)pyridin-2-yl)-2-((3-hydroxy-3-methylazetidin-1-yl)methyl)-3-methylpyrrolo[2,1-f][1,2,4]triazin-4(3H)-one;
6-(3-chloro-4-(2-chloro-3-(5-((((1r,4s)-4-hydroxycyclohexyl)amino)methyl)-6-methoxypyridin-2-yl)phenyl)pyridin-2-yl)-2-((3-hydroxy-3-methylazetidin-1-yl)methyl)-3-methylpyrrolo[2,1-f][1,2,4]triazin-4(3H)-one;
6-(3-chloro-4-(2-chloro-3-(5-((((1s,4r)-4-hydroxycyclohexyl)amino)methyl)-6-methoxypyridin-2-yl)phenyl)pyridin-2-yl)-2-((3-hydroxy-3-methylazetidin-1-yl)methyl)-3-methylpyrrolo[2, I-f] [1,2,4]triazin-4(3H)-one;
6-(3-chloro-4-(2-chloro-3-(5-((((1s,4s)-4-hydroxycyclohexyl)amino)methyl)-6-methoxypyridin-2-yl)phenyl)pyridin-2-yl)-2-((3-hydroxy-3-methylazetidin-1-yl)methyl)-3-methylpyrrolo[2,1-f][1,2,4]triazin-4(3H)-one;
(S)-5-((((6-(2-chloro-3-(3-chloro-2-(1-methyl-3-(((((S)-oxetan-2-yl)methyl)amino)methyl)-1H-indazol-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(1-methyl-3-(((((R)-oxetan-2-yl)methyl)amino)methyl)-1H-indazol-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(1-methyl-3-(((((S)-oxetan-2-yl)methyl)amino)methyl)-1H-indazol-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(1-methyl-3-(((((R)-oxetan-2-yl)methyl)amino)methyl)-1H-indazol-6-yl)pyridin-4-yl)phenyl)-2-methoxypyridin-3-yl)methyl)amino)methyl)pyrrolidin-2-one;
(S)-5-((((8-chloro-2-(2-chloro-3-(3-chloro-2-(3-fluoro-5-methoxy-4-(((((S)-5-oxopyrrolidin-2-yl)methyl)amino)methyl)phenyl)pyridin-4-yl)phenyl)-5-methoxyquinolin-6-yl)methyl)amino)methyl)pyrrolidin-2-one;
(S)-5-((((8-chloro-2-(2-chloro-3-(3-chloro-2-(3-fluoro-5-methoxy-4-(((((R)-5-oxopyrrolidin-2-yl)methyl)amino)methyl)phenyl)pyridin-4-yl)phenyl)-5-methoxyquinolin-6-yl)methyl)amino)methyl)pyrrolidin-2-one;
(R)-5-((((8-chloro-2-(2-chloro-3-(3-chloro-2-(3-fluoro-5-methoxy-4-(((((S)-5-oxopyrrolidin-2-yl)methyl)amino)methyl)phenyl)pyridin-4-yl)phenyl)-5-methoxyquinolin-6-yl)methyl)amino)methyl)pyrrolidin-2-one;
(R)-5-((((8-chloro-2-(2-chloro-3-(3-chloro-2-(3-fluoro-5-methoxy-4-(((((R)-5-oxopyrrolidin-2-yl)methyl)amino)methyl)phenyl)pyridin-4-yl)phenyl)-5-methoxyquinolin-6-yl)methyl)amino)methyl)pyrrolidin-2-one;
(S)-5-((((6-(3-(2-(4-(((2-acetyl-2-azaspiro[3.3]heptan-6-yl)amino)methyl)-3-methoxyphenyl)-3-chloropyridin-4-yl)-2-chlorophenyl)-2-methoxypyridin-3-yl)methyl)amino)methyl)pyrrolidin-2-one;
(R)-5-((((6-(3-(2-(4-(((2-acetyl-2-azaspiro[3.3]heptan-6-yl)amino)methyl)-3-methoxyphenyl)-3-chloropyridin-4-yl)-2-chlorophenyl)-2-methoxypyridin-3-yl)methyl)amino)methyl)pyrrolidin-2-one;
(S)-5-(((4-(3-chloro-4-(2-chloro-3-(8-chloro-6-(((2-hydroxyethyl)amino)methyl)-5-methoxyquinolin-2-yl)phenyl)pyridin-2-yl)-2-methoxybenzyl)amino)methyl)pyrrolidin-2-one;
(R)-5-(((4-(3-chloro-4-(2-chloro-3-(8-chloro-6-(((2-hydroxyethyl)amino)methyl)-5-methoxyquinolin-2-yl)phenyl)pyridin-2-yl)-2-methoxybenzyl)amino)methyl)pyrrolidin-2-one;
(S)-1-(4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)pyrrolidine-3-carboxylic acid;
(R)-1-(4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)pyrrolidine-3-carboxylic acid;
1-(4-(((6-(2-chloro-3-(3-chloro-2-(3-methoxy-4-((methylamino)methyl)phenyl)pyridin-4-yl)phenyl)-2-methoxypyridin-3-yl)methyl)amino)piperidin-1-yl)ethan-1-one;
(S)-5-(((4-(4-(3-(6-(((1-acetylpiperidin-4-yl)amino)methyl)-8-chloro-5-methoxyquinolin-2-yl)-2-chlorophenyl)-3-chloropyridin-2-yl)-2-methoxybenzyl)amino)methyl)pyrrolidin-2-one;
(R)-5-(((4-(4-(3-(6-(((1-acetylpiperidin-4-yl)amino)methyl)-8-chloro-5-methoxyquinolin-2-yl)-2-chlorophenyl)-3-chloropyridin-2-yl)-2-methoxybenzyl)amino)methyl)pyrrolidin-2-one;
5-(3-chloro-4-(2-chloro-3'-fluoro-5'-methoxy-4'-(((((S)-5-oxopyrrolidin-2-yl)methyl)amino)methyl)-[1,1'-biphenyl]-3-yl)pyridin-2-yl)-2-(2-((((S)-5-oxopyrrolidin-2-yl)methyl)amino)ethyl)isoindolin-1-one;
5-(3-chloro-4-(2-chloro-3'-fluoro-5'-methoxy-4'-(((((S)-5-oxopyrrolidin-2-yl)methyl)amino)methyl)-[1,1'-biphenyl]-3-yl)pyridin-2-yl)-2-(2-((((R)-5-oxopyrrolidin-2-yl)methyl)amino)ethyl)isoindolin-1-one;
5-(3-chloro-4-(2-chloro-3'-fluoro-5'-methoxy-4'-(((((R)-5-oxopyrrolidin-2-yl)methyl)amino)methyl)-[1,1'-biphenyl]-3-yl)pyridin-2-yl)-2-(2-((((S)-5-oxopyrrolidin-2-yl)methyl)amino)ethyl)isoindolin-1-one;
5-(3-chloro-4-(2-chloro-3'-fluoro-5'-methoxy-4'-(((((R)-5-oxopyrrolidin-2-yl)methyl)amino)methyl)-[1,1'-biphenyl]-3-yl)pyridin-2-yl)-2-(2-((((R)-5-oxopyrrolidin-2-yl)methyl)amino)ethyl)isoindolin-1-one;
2-(4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)-2-azaspiro[3.3]heptane-6-carboxylic acid;
2-(4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)-2,6-diazaspiro[3.4]octan-7-one;
isopropyl (S)-1-(4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)pyrrolidine-3-carboxylate;
isopropyl (R)-1-(4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)pyrrolidine-3-carboxylate;
1-(4-((4-(4-(3-(3-(((1-acetylpiperidin-4-yl)amino)methyl)-1-methyl-1H-pyrrolo[2,3-b]pyridin-6-yl)-2-chlorophenyl)-3-chloropyridin-2-yl)-2-methoxybenzyl)amino)piperidin-1-yl)ethan-1-one;
(S)-5-((((6-(2-chloro-3-(3-chloro-2-(3-(((2-hydroxyethyl)(methyl)amino)methyl)-1-methyl-1H-indazol-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(3-(((2-hydroxyethyl)(methyl)amino)methyl)-1-methyl-1H-indazol-6-yl)pyridin-4-yl)phenyl)-2-methoxypyridin-3-yl)methyl)amino)methyl)pyrrolidin-2-one;
(S)-1-(6-((6-((4-(5-((6-acetyl-2,6-diazaspiro[3.3]heptan-2-yl)methyl)-3'-chloro-6-methoxy-[2,4'-bipyridin]-2'-yl)-2,3-dihydro-1H-inden-1-yl)oxy)-2-methoxy-5-(trifluoromethyl)pyridin-3-yl)methyl)-2,6-diazaspiro[3.3]heptan-2-yl)ethan-1-one;
(R)-1-(6-((6-((4-(5-((6-acetyl-2,6-diazaspiro[3.3]heptan-2-yl)methyl)-3'-chloro-6-methoxy-[2,4'-bipyridin]-2'-yl)-2,3-dihydro-1H-inden-1-yl)oxy)-2-methoxy-5-(trifluoromethyl)pyridin-3-yl)methyl)-2,6-diazaspiro[3.3]heptan-2-yl)ethan-1-one;
1-(6-((6-(2-chloro-3-(3-chloro-2-(2-(3-fluoropropyl)-8-methoxy-1,2,3,4-tetrahydroisoquinolin-6-yl)pyridin-4-yl)phenyl)-2-methoxypyridin-3-yl)methyl)-2,6-diazaspiro[3.3]heptan-2-yl)ethan-1-one;
2-((6-(2-chloro-3-(3-chloro-2-(2-(3-fluoropropyl)-8-methoxy-1,2,3,4-tetrahydroisoquinolin-6-yl)pyridin-4-yl)phenyl)-2-methoxypyridin-3-yl)methyl)-2,6-diazaspiro[3.4]octan-7-one;
N-((6-(2-chloro-3-(3-chloro-2-(2-(3-fluoropropyl)-8-methoxy-1,2,3,4-tetrahydroisoquinolin-6-yl)pyridin-4-yl)phenyl)-2-methoxypyridin-3-yl)methyl)-2-oxaspiro[3.3]heptan-6-amine;
(1-((((6-(2-chloro-3-(3-chloro-2-(2-(3-fluoropropyl)-8-methoxy-1,2,3,4-tetrahydroisoquinolin-6-yl)pyridin-4-yl)phenyl)-2-methoxypyridin-3-yl)methyl)amino)methyl)cyclopropyl)methanol;
(S)-1-(((3'-chloro-2'-((S)-1-((5-((((S)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)propan-2-ol;
(S)-1-(((3'-chloro-2'-((R)-1-((5-((((R)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)propan-2-ol;
(S)-1-(((3'-chloro-2'-((R)-1-((5-((((S)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)propan-2-ol;
(S)-1-(((3'-chloro-2'-((S)-1-((5-((((R)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)propan-2-ol;
(R)-1-(((3'-chloro-2'-((R)-1-((5-((((R)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)propan-2-ol;
(R)-1-(((3'-chloro-2'-((R)-1-((5-((((S)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)propan-2-ol;
(R)-1-(((3'-chloro-2'-((S)-1-((5-((((R)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)propan-2-ol;
(R)-1-(((3'-chloro-2'-((S)-1-((5-((((S)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)propan-2-ol;
(S)-1-(4-(((6-((4-(5-(((1-acetylpiperidin-4-yl)amino)methyl)-3'-chloro-6-methoxy-[2,4'-bipyridin]-2'-yl)-2,3-dihydro-1H-inden- 1-yl)oxy)-2-methoxy-5-(trifluoromethyl)pyridin-3-yl)methyl)amino)piperidin-1-yl)ethan-1-one;
(R)-1-(4-(((6-((4-(5-(((1-acetylpiperidin-4-yl)amino)methyl)-3'-chloro-6-methoxy-[2,4'-bipyridin]-2'-yl)-2,3-dihydro-1H-inden- 1-yl)oxy)-2-methoxy-5-(trifluoromethyl)pyridin-3-yl)methyl)amino)piperidin-1-yl)ethan-1-one;
2-((6-(2-chloro-3-(3-chloro-2-((R)-5-((((S)-5-oxopyrrolidin-2-yl)methyl)amino)-5,6,7, 8-tetrahydronaphthalen-2-yl)pyridin-4-yl)phenyl)-2-methoxypyridin-3-yl)methyl)-2,6-diazaspiro[3.4]octan-7-one;
2-((6-(2-chloro-3-(3-chloro-2-((S)-5-((((S)-5-oxopyrrolidin-2-yl)methyl)amino)-5,6,7,8-tetrahydronaphthalen-2-yl)pyridin-4-yl)phenyl)-2-methoxypyridin-3-yl)methyl)-2,6-diazaspiro[3.4]octan-7-one;
2-((6-(2-chloro-3-(3-chloro-2-((R)-5-((((R)-5-oxopyrrolidin-2-yl)methyl)amino)-5,6,7,8-tetrahydronaphthalen-2-yl)pyridin-4-yl)phenyl)-2-methoxypyridin-3-yl)methyl)-2,6-diazaspiro[3.4]octan-7-one;
2-((6-(2-chloro-3-(3-chloro-2-((S)-5-((((R)-5-oxopyrrolidin-2-yl)methyl)amino)-5,6,7,8-tetrahydronaphthalen-2-yl)pyridin-4-yl)phenyl)-2-methoxypyridin-3-yl)methyl)-2,6-diazaspiro[3.4]octan-7-one;
(S)-5-((((R)-6-(3-chloro-4-(2-chloro-3-(5-((((S)-2-hydroxypropyl)amino)methyl)-6-methoxypyridin-2-yl)phenyl)pyridin-2-yl)-1,2,3,4-tetrahydronaphthalen-1-yl)amino)methyl)pyrrolidin-2-one;
(S)-5-((((R)-6-(3-chloro-4-(2-chloro-3-(5-((((R)-2-hydroxypropyl)amino)methyl)-6-methoxypyridin-2-yl)phenyl)pyridin-2-yl)-1,2,3,4-tetrahydronaphthalen-1-yl)amino)methyl)pyrrolidin-2-one;
(S)-5-((((S)-6-(3-chloro-4-(2-chloro-3-(5-((((R)-2-hydroxypropyl)amino)methyl)-6-methoxypyridin-2-yl)phenyl)pyridin-2-yl)-1,2,3,4-tetrahydronaphthalen-1-yl)amino)methyl)pyrrolidin-2-one;
(S)-5-((((S)-6-(3-chloro-4-(2-chloro-3-(5-((((S)-2-hydroxypropyl)amino)methyl)-6-methoxypyridin-2-yl)phenyl)pyridin-2-yl)-1,2,3,4-tetrahydronaphthalen-1-yl)amino)methyl)pyrrolidin-2-one;
(R)-5-((((R)-6-(3-chloro-4-(2-chloro-3-(5-((((R)-2-hydroxypropyl)amino)methyl)-6-methoxypyridin-2-yl)phenyl)pyridin-2-yl)-1,2,3,4-tetrahydronaphthalen-1-yl)amino)methyl)pyrrolidin-2-one;
(R)-5-((((R)-6-(3-chloro-4-(2-chloro-3-(5-((((S)-2-hydroxypropyl)amino)methyl)-6-methoxypyridin-2-yl)phenyl)pyridin-2-yl)-1,2,3,4-tetrahydronaphthalen-1-yl)amino)methyl)pyrrolidin-2-one;
(R)-5-((((S)-6-(3-chloro-4-(2-chloro-3-(5-((((R)-2-hydroxypropyl)amino)methyl)-6-methoxypyridin-2-yl)phenyl)pyridin-2-yl)-1,2,3,4-tetrahydronaphthalen-1-yl)amino)methyl)pyrrolidin-2-one;
(R)-5-((((S)-6-(3-chloro-4-(2-chloro-3-(5-((((S)-2-hydroxypropyl)amino)methyl)-6-methoxypyridin-2-yl)phenyl)pyridin-2-yl)-1,2,3,4-tetrahydronaphthalen-1-yl)amino)methyl)pyrrolidin-2-one;
(S)-5-((((R)-6-(3-chloro-4-(2-chloro-3-(6-methoxy-5-((((S)-2-oxotetrahydrofuran-3-yl)amino)methyl)pyridin-2-yl)phenyl)pyridin-2-yl)-1,2,3,4-tetrahydronaphthalen-1-yl)amino)methyl)pyrrolidin-2-one;
(S)-5-((((S)-6-(3-chloro-4-(2-chloro-3-(6-methoxy-5-((((S)-2-oxotetrahydrofuran-3-yl)amino)methyl)pyridin-2-yl)phenyl)pyridin-2-yl)-1,2,3,4-tetrahydronaphthalen-1-yl)amino)methyl)pyrrolidin-2-one;
(S)-5-((((S)-6-(3-chloro-4-(2-chloro-3-(6-methoxy-5-((((R)-2-oxotetrahydrofuran-3-yl)amino)methyl)pyridin-2-yl)phenyl)pyridin-2-yl)-1,2,3,4-tetrahydronaphthalen-1-yl)amino)methyl)pyrrolidin-2-one;
(S)-5-((((R)-6-(3-chloro-4-(2-chloro-3-(6-methoxy-5-((((R)-2-oxotetrahydrofuran-3-yl)amino)methyl)pyridin-2-yl)phenyl)pyridin-2-yl)-1,2,3,4-tetrahydronaphthalen-1-yl)amino)methyl)pyrrolidin-2-one;
(R)-5-((((R)-6-(3-chloro-4-(2-chloro-3-(6-methoxy-5-((((S)-2-oxotetrahydrofuran-3-yl)amino)methyl)pyridin-2-yl)phenyl)pyridin-2-yl)-1,2,3,4-tetrahydronaphthalen-1-yl)amino)methyl)pyrrolidin-2-one;
(R)-5-((((S)-6-(3-chloro-4-(2-chloro-3-(6-methoxy-5-((((S)-2-oxotetrahydrofuran-3-yl)amino)methyl)pyridin-2-yl)phenyl)pyridin-2-yl)-1,2,3,4-tetrahydronaphthalen-1-yl)amino)methyl)pyrrolidin-2-one;
(R)-5-((((S)-6-(3-chloro-4-(2-chloro-3-(6-methoxy-5-((((R)-2-oxotetrahydrofuran-3-yl)amino)methyl)pyridin-2-yl)phenyl)pyridin-2-yl)-1,2,3,4-tetrahydronaphthalen-1-yl)amino)methyl)pyrrolidin-2-one;
(S)-5-((((S)-6-(3-chloro-4-(2-chloro-3-(6-methoxy-5-((((S)-2-oxotetrahydrofuran-3-yl)amino)methyl)pyridin-2-yl)phenyl)pyridin-2-yl)-1,2,3,4-tetrahydronaphthalen-1-yl)amino)methyl)pyrrolidin-2-one;
(S)-5-((((6-(2-chloro-3-(3-chloro-2-(8-methoxy-2-(((((S)-5-oxopyrrolidin-2-yl)methyl)amino)methyl)-[1,2,4]triazolo[1,5-a]pyridin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(8-methoxy-2-(((((R)-5-oxopyrrolidin-2-yl)methyl)amino)methyl)-[1,2,4]triazolo[1,5-a]pyridin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(8-methoxy-2-(((((S)-5-oxopyrrolidin-2-yl)methyl)amino)methyl)-[1,2,4]triazolo[1,5-a]pyridin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(8-methoxy-2-(((((R)-5-oxopyrrolidin-2-yl)methyl)amino)methyl)-[1,2,4]triazolo[1,5-a]pyridin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(2-((3-hydroxy-3-methylazetidin-1-yl)methyl)-8-methoxy-[1,2,4]triazolo[1,5-a]pyridin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-((3-hydroxy-3-methylazetidin-1-yl)methyl)-8-methoxy-[1,2,4]triazolo[1,5-a]pyridin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(2-((((1r,4r)-4-hydroxycyclohexyl)amino)methyl)-8-methoxy-[1,2,4]triazolo[1,5-a]pyridin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(2-((((1s,4s)-4-hydroxycyclohexyl)amino)methyl)-8-methoxy-[1,2,4]triazolo[1,5-a]pyridin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(2-((((1s,4r)-4-hydroxycyclohexyl)amino)methyl)-8-methoxy-[1,2,4]triazolo[1,5-a]pyridin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(2-((((1r,4s)-4-hydroxycyclohexyl)amino)methyl)-8-methoxy-[1,2,4]triazolo[1,5-a]pyridin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-((((1r,4s)-4-hydroxycyclohexyl)amino)methyl)-8-methoxy-[1,2,4]triazolo[1,5-a]pyridin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-((((1s,4s)-4-hydroxycyclohexyl)amino)methyl)-8-methoxy-[1,2,4]triazolo[1,5-a]pyridin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-((((1s,4r)-4-hydroxycyclohexyl)amino)methyl)-8-methoxy-[1,2,4]triazolo[1,5-a]pyridin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-((((1r,4r)-4-hydroxycyclohexyl)amino)methyl)-8-methoxy-[1,2,4]triazolo[1,5-a]pyridin-6-yl)pyridin-4-yl)phenyl)-2-methoxypyridin-3-yl)methyl)amino)methyl)pyrrolidin-2-one;
(S)-1-((6-(3-chloro-4-(2-chloro-3-(6-methoxy-5-((((5-oxopyrrolidin-2-yl)methyl)amino)methyl)pyridin-2-yl)phenyl)pyridin-2-yl)-8-methoxy-[1,2,4]triazolo[1,5-a]pyridin-2-yl)methyl)azetidine-3-carboxylic acid;
(R)-1-((6-(3-chloro-4-(2-chloro-3-(6-methoxy-5-((((5-oxopyrrolidin-2-yl)methyl)amino)methyl)pyridin-2-yl)phenyl)pyridin-2-yl)-8-methoxy-[1,2,4]triazolo[1,5-a]pyridin-2-yl)methyl)azetidine-3-carboxylic acid;
(S)-1-(2-(((6-(3-chloro-4-(2-chloro-3-(6-methoxy-5-((((5-oxopyrrolidin-2-yl)methyl)amino)methyl)pyridin-2-yl)phenyl)pyridin-2-yl)-8-methoxy-[1,2,4]triazolo[1,5-a]pyridin-2-yl)methyl)amino)ethyl)cyclopropane-1-carboxylic acid;
(R)-1-(2-(((6-(3-chloro-4-(2-chloro-3-(6-methoxy-5-((((5-oxopyrrolidin-2-yl)methyl)amino)methyl)pyridin-2-yl)phenyl)pyridin-2-yl)-8-methoxy-[1,2,4]triazolo[1,5-a]pyridin-2-yl)methyl)amino)ethyl)cyclopropane-1-carboxylic acid;
1-(4-(((6-(3-(2-(3-(((1-acetylpiperidin-4-yl)amino)methyl)-1-methyl-1H-indazol-6-yl)-3-chloropyridin-4-yl)-2-chlorophenyl)-2-methoxypyridin-3-yl)methyl)amino)piperidin-1-yl)ethan-1-one;
1-(4-((4-(4-(3-(3-(((1-acetylpiperidin-4-yl)(methyl)amino)methyl)-1-methyl-1H-pyrrolo[2,3-b]pyridin-6-yl)-2-chlorophenyl)-3-chloropyridin-2-yl)-2-methoxybenzyl)amino)piperidin-1-yl)ethan-1-one;
((6-(2-chloro-3-(3-chloro-2-((R)-5-((((S)-5-oxopyrrolidin-2-yl)methyl)amino)-5,6,7, 8-tetrahydronaphthalen-2-yl)pyridin-4-yl)phenyl)-2-methoxypyridin-3-yl)methyl)-L-homoserine;
((6-(2-chloro-3-(3-chloro-2-((R)-5-((((R)-5-oxopyrrolidin-2-yl)methyl)amino)-5,6,7,8-tetrahydronaphthalen-2-yl)pyridin-4-yl)phenyl)-2-methoxypyridin-3-yl)methyl)-L-homoserine;
((6-(2-chloro-3-(3-chloro-2-((S)-5-((((S)-5-oxopyrrolidin-2-yl)methyl)amino)-5,6,7,8-tetrahydronaphthalen-2-yl)pyridin-4-yl)phenyl)-2-methoxypyridin-3-yl)methyl)-L-homoserine;
((6-(2-chloro-3-(3-chloro-2-((S)-5-((((R)-5-oxopyrrolidin-2-yl)methyl)amino)-5,6,7,8-tetrahydronaphthalen-2-yl)pyridin-4-yl)phenyl)-2-methoxypyridin-3-yl)methyl)-L-homoserine;
((6-(2-chloro-3-(3-chloro-2-((R)-5-((((R)-5-oxopyrrolidin-2-yl)methyl)amino)-5,6,7,8-tetrahydronaphthalen-2-yl)pyridin-4-yl)phenyl)-2-methoxypyridin-3-yl)methyl)-D-homoserine;
((6-(2-chloro-3-(3-chloro-2-((S)-5-((((S)-5-oxopyrrolidin-2-yl)methyl)amino)-5,6,7,8-tetrahydronaphthalen-2-yl)pyridin-4-yl)phenyl)-2-methoxypyridin-3-yl)methyl)-D-homoserine;
((6-(2-chloro-3-(3-chloro-2-((S)-5-((((R)-5-oxopyrrolidin-2-yl)methyl)amino)-5,6,7,8-tetrahydronaphthalen-2-yl)pyridin-4-yl)phenyl)-2-methoxypyridin-3-yl)methyl)-D-homoserine;
((6-(2-chloro-3-(3-chloro-2-((R)-5-((((S)-5-oxopyrrolidin-2-yl)methyl)amino)-5,6,7, 8-tetrahydronaphthalen-2-yl)pyridin-4-yl)phenyl)-2-methoxypyridin-3-yl)methyl)-D-homoserine;
(5S,5'S)-5,5'-(((3,3'-dichloro-[4,4'-bipyridine]-2,2'-diyl)bis(8-methoxy-3,4-dihydroisoquinoline-6,2(1H)-diyl))bis(methylene))bis(pyrrolidin-2-one);
(5S,5'R)-5,5'-(((3,3'-dichloro-[4,4'-bipyridine]-2,2'-diyl)bis(8-methoxy-3,4-dihydroisoquinoline-6,2(1H)-diyl))bis(methylene))bis(pyrrolidin-2-one);
(5R,5'S)-5,5'-(((3,3'-dichloro-[4,4'-bipyridine]-2,2'-diyl)bis(8-methoxy-3,4-dihydroisoquinoline-6,2(1H)-diyl))bis(methylene))bis(pyrrolidin-2-one);
(5R,5'R)-5,5'-(((3,3'-dichloro-[4,4'-bipyridine]-2,2'-diyl)bis(8-methoxy-3,4-dihydroisoquinoline-6,2(1H)-diyl))bis(methylene))bis(pyrrolidin-2-one);
methyl ((3-chloro-4-(2-chloro-3-(6-methoxy-5-(((((S)-5-oxopyrrolidin-2-yl)methyl)amino)methyl)pyridin-2-yl)phenyl)-5'-methoxy-[2,3'-bipyridin]-6'-yl)methyl)-L-homoserinate;
methyl ((3-chloro-4-(2-chloro-3-(6-methoxy-5-(((((R)-5-oxopyrrolidin-2-yl)methyl)amino)methyl)pyridin-2-yl)phenyl)-5'-methoxy-[2,3'-bipyridin]-6'-yl)methyl)-L-homoserinate;
methyl ((3-chloro-4-(2-chloro-3-(6-methoxy-5-(((((R)-5-oxopyrrolidin-2-yl)methyl)amino)methyl)pyridin-2-yl)phenyl)-5'-methoxy-[2,3'-bipyridin]-6'-yl)methyl)-D-homoserinate;
methyl ((3-chloro-4-(2-chloro-3-(6-methoxy-5-(((((S)-5-oxopyrrolidin-2-yl)methyl)amino)methyl)pyridin-2-yl)phenyl)-5'-methoxy-[2,3'-bipyridin]-6'-yl)methyl)-D-homoserinate;
(S)-5-((((6-(2-chloro-3-(3-chloro-5'-methoxy-6'-((((S)-2-oxotetrahydrofuran-3-yl)amino)methyl)-[2,3'-bipyridin]-4-yl)phenyl)-2-methoxypyridin-3-yl)methyl)amino)methyl)pyrrolidin-2-one;
(S)-5-((((6-(2-chloro-3-(3-chloro-5'-methoxy-6'-((((R)-2-oxotetrahydrofuran-3-yl)amino)methyl)-[2,3'-bipyridin]-4-yl)phenyl)-2-methoxypyridin-3-yl)methyl)amino)methyl)pyrrolidin-2-one;
(R)-5-((((6-(2-chloro-3-(3-chloro-5'-methoxy-6'-((((S)-2-oxotetrahydrofuran-3-yl)amino)methyl)-[2,3'-bipyridin]-4-yl)phenyl)-2-methoxypyridin-3-yl)methyl)amino)methyl)pyrrolidin-2-one;
(R)-5-((((6-(2-chloro-3-(3-chloro-5'-methoxy-6'-((((R)-2-oxotetrahydrofuran-3-yl)amino)methyl)-[2,3'-bipyridin]-4-yl)phenyl)-2-methoxypyridin-3-yl)methyl)amino)methyl)pyrrolidin-2-one;
(S)-5-((((6-(((S)-4-(3-chloro-2-(3-methoxy-4-(((((S)-5-oxopyrrolidin-2-yl)methyl)amino)methyl)phenyl)pyridin-4-yl)-2,3-dihydro-1H-inden-1-yl)oxy)-2-methoxy-5-(trifluoromethyl)pyridin-3-yl)methyl)amino)methyl)pyrrolidin-2-one;
(S)-5-((((6-(((S)-4-(3-chloro-2-(3-methoxy-4-(((((R)-5-oxopyrrolidin-2-yl)methyl)amino)methyl)phenyl)pyridin-4-yl)-2,3-dihydro-1H-inden-1-yl)oxy)-2-methoxy-5-(trifluoromethyl)pyridin-3-yl)methyl)amino)methyl)pyrrolidin-2-one;
(S)-5-((((6-(((R)-4-(3-chloro-2-(3-methoxy-4-(((((S)-5-oxopyrrolidin-2-yl)methyl)amino)methyl)phenyl)pyridin-4-yl)-2,3-dihydro-1H-inden-1-yl)oxy)-2-methoxy-5-(trifluoromethyl)pyridin-3-yl)methyl)amino)methyl)pyrrolidin-2-one;
(S)-5-((((6-(((S)-4-(3-chloro-2-(3-methoxy-4-(((((R)-5-oxopyrrolidin-2-yl)methyl)amino)methyl)phenyl)pyridin-4-yl)-2,3-dihydro-1H-inden-1-yl)oxy)-2-methoxy-5-(trifluoromethyl)pyridin-3-yl)methyl)amino)methyl)pyrrolidin-2-one;
(R)-5-((((6-(((S)-4-(3-chloro-2-(3-methoxy-4-(((((S)-5-oxopyrrolidin-2-yl)methyl)amino)methyl)phenyl)pyridin-4-yl)-2,3-dihydro-1H-inden-1-yl)oxy)-2-methoxy-5-(trifluoromethyl)pyridin-3-yl)methyl)amino)methyl)pyrrolidin-2-one;
(R)-5-((((6-(((S)-4-(3-chloro-2-(3-methoxy-4-(((((R)-5-oxopyrrolidin-2-yl)methyl)amino)methyl)phenyl)pyridin-4-yl)-2,3-dihydro-1H-inden-1-yl)oxy)-2-methoxy-5-(trifluoromethyl)pyridin-3-yl)methyl)amino)methyl)pyrrolidin-2-one;
(R)-5-((((6-(((R)-4-(3-chloro-2-(3-methoxy-4-(((((S)-5-oxopyrrolidin-2-yl)methyl)amino)methyl)phenyl)pyridin-4-yl)-2,3-dihydro-1H-inden-1-yl)oxy)-2-methoxy-5-(trifluoromethyl)pyridin-3-yl)methyl)amino)methyl)pyrrolidin-2-one;
(R)-5-((((6-(((S)-4-(3-chloro-2-(3-methoxy-4-(((((R)-5-oxopyrrolidin-2-yl)methyl)amino)methyl)phenyl)pyridin-4-yl)-2,3-dihydro-1H-inden-1-yl)oxy)-2-methoxy-5-(trifluoromethyl)pyridin-3-yl)methyl)amino)methyl)pyrrolidin-2-one;
(S)-1-(((6-(((S)-4-(3-chloro-2-(4-((((S)-2-hydroxypropyl)amino)methyl)-3-methoxyphenyl)pyridin-4-yl)-2,3-dihydro-1H-inden-1-yl)oxy)-2-methoxy-5-(trifluoromethyl)pyridin-3-yl)methyl)amino)propan-2-ol;
(S)-1-(((6-(((S)-4-(3-chloro-2-(4-((((R)-2-hydroxypropyl)amino)methyl)-3-methoxyphenyl)pyridin-4-yl)-2,3-dihydro-1H-inden-1-yl)oxy)-2-methoxy-5-(trifluoromethyl)pyridin-3-yl)methyl)amino)propan-2-ol;
(S)-1-(((6-(((R)-4-(3-chloro-2-(4-((((S)-2-hydroxypropyl)amino)methyl)-3-methoxyphenyl)pyridin-4-yl)-2,3-dihydro-1H-inden-1-yl)oxy)-2-methoxy-5-(trifluoromethyl)pyridin-3-yl)methyl)amino)propan-2-ol;
(S)-1-(((6-(((R)-4-(3-chloro-2-(4-((((R)-2-hydroxypropyl)amino)methyl)-3-methoxyphenyl)pyridin-4-yl)-2,3-dihydro-1H-inden-1-yl)oxy)-2-methoxy-5-(trifluoromethyl)pyridin-3-yl)methyl)amino)propan-2-ol;
(R)-1-(((6-(((S)-4-(3-chloro-2-(4-((((S)-2-hydroxypropyl)amino)methyl)-3-methoxyphenyl)pyridin-4-yl)-2,3-dihydro-1H-inden-1-yl)oxy)-2-methoxy-5-(trifluoromethyl)pyridin-3-yl)methyl)amino)propan-2-ol;
(R)-1-(((6-(((S)-4-(3-chloro-2-(4-((((R)-2-hydroxypropyl)amino)methyl)-3-methoxyphenyl)pyridin-4-yl)-2,3-dihydro-1H-inden-1-yl)oxy)-2-methoxy-5-(trifluoromethyl)pyridin-3-yl)methyl)amino)propan-2-ol;
(R)-1-(((6-(((R)-4-(3-chloro-2-(4-((((S)-2-hydroxypropyl)amino)methyl)-3-methoxyphenyl)pyridin-4-yl)-2,3-dihydro-1H-inden-1-yl)oxy)-2-methoxy-5-(trifluoromethyl)pyridin-3-yl)methyl)amino)propan-2-ol;
(R)-1-(((6-(((R)-4-(3-chloro-2-(4-((((R)-2-hydroxypropyl)amino)methyl)-3-methoxyphenyl)pyridin-4-yl)-2,3-dihydro-1H-inden-1-yl)oxy)-2-methoxy-5-(trifluoromethyl)pyridin-3-yl)methyl)amino)propan-2-ol;
1-(4-(((6-(3-(2-(4-(((1-acetylpiperidin-4-yl)amino)methyl)-3-methoxyphenyl)-3-methylpyridin-4-yl)-2-chlorophenyl)-2-methoxypyridin-3-yl)methyl)amino)piperidin-1-yl)ethan-1-one;
methyl (S)-3-(6-(3-chloro-4-(2-chloro-3-(5-(((2-hydroxypropyl)amino)methyl)-6-methoxypyridin-2-yl)phenyl)pyridin-2-yl)-8-methoxy-3,4-dihydroisoquinolin-2(1H)-yl)propanoate;
methyl (R)-3-(6-(3-chloro-4-(2-chloro-3-(5-(((2-hydroxypropyl)amino)methyl)-6-methoxypyridin-2-yl)phenyl)pyridin-2-yl)-8-methoxy-3,4-dihydroisoquinolin-2(1H)-yl)propanoate;
methyl 3-(6-(3-chloro-4-(2-chloro-3-(6-methoxy-5-((7-oxo-2,6-diazaspiro[3.4]octan-2-yl)methyl)pyridin-2-yl)phenyl)pyridin-2-yl)-8-methoxy-3,4-dihydroisoquinolin-2(1H)-yl)propanoate;
(S)-1-(((6-(2-chloro-3-(2-(4-((((S)-2-hydroxypropyl)amino)methyl)-3-methoxyphenyl)-3-methylpyridin-4-yl)phenyl)-2-methoxypyridin-3-yl)methyl)amino)propan-2-ol;
(S)-1-(((6-(2-chloro-3-(2-(4-((((R)-2-hydroxypropyl)amino)methyl)-3-methoxyphenyl)-3-methylpyridin-4-yl)phenyl)-2-methoxypyridin-3-yl)methyl)amino)propan-2-ol;
(R)-1-(((6-(2-chloro-3-(2-(4-((((S)-2-hydroxypropyl)amino)methyl)-3-methoxyphenyl)-3-methylpyridin-4-yl)phenyl)-2-methoxypyridin-3-yl)methyl)amino)propan-2-ol;
(R)-1-(((6-(2-chloro-3-(2-(4-((((R)-2-hydroxypropyl)amino)methyl)-3-methoxyphenyl)-3-methylpyridin-4-yl)phenyl)-2-methoxypyridin-3-yl)methyl)amino)propan-2-ol;
(S)-2-(6-(3-chloro-4-(2-chloro-3-(5-(((2-hydroxypropyl)amino)methyl)-6-methoxypyridin-2-yl)phenyl)pyridin-2-yl)-8-methoxy-3,4-dihydroisoquinolin-2(1H)-yl)acetamide;
(R)-2-(6-(3-chloro-4-(2-chloro-3-(5-(((2-hydroxypropyl)amino)methyl)-6-methoxypyridin-2-yl)phenyl)pyridin-2-yl)-8-methoxy-3,4-dihydroisoquinolin-2(1H)-yl)acetamide;
1-(6-(2-chloro-3-(3-chloro-2-(1-methyl-3-((methylamino)methyl)-1H-indol-6-yl)pyridin-4-yl)phenyl)-2-methoxypyridin-3-yl)-N-methylmethanamine;
1-(4-(((6-(3-(2-(3-(((1-acetylpiperidin-4-yl)amino)methyl)-1-methyl-1H-indol-6-yl)-3-chloropyridin-4-yl)-2-chlorophenyl)-2-methoxypyridin-3-yl)methyl)amino)piperidin-1-yl)ethan-1-one;
isopropyl (S)-1-(4-(3-chloro-4-(2-chloro-3-(6-methoxy-5-((methylamino)methyl)pyridin-2-yl)phenyl)pyridin-2-yl)-2-methoxybenzyl)pyrrolidine-3-carboxylate;
isopropyl (R)-1-(4-(3-chloro-4-(2-chloro-3-(6-methoxy-5-((methylamino)methyl)pyridin-2-yl)phenyl)pyridin-2-yl)-2-methoxybenzyl)pyrrolidine-3-carboxylate;
isopropyl 3-((4-(3-chloro-4-(2-chloro-3-(6-methoxy-5-((methylamino)methyl)pyridin-2-yl)phenyl)pyridin-2-yl)-2-methoxybenzyl)amino)propanoate;
isopropyl (S)-4-((4-(3-chloro-4-(2-chloro-3-(6-methoxy-5-((methylamino)methyl)pyridin-2-yl)phenyl)pyridin-2-yl)-2-methoxybenzyl)amino)-3-hydroxybutanoate;
isopropyl (R)-4-((4-(3-chloro-4-(2-chloro-3-(6-methoxy-5-((methylamino)methyl)pyridin-2-yl)phenyl)pyridin-2-yl)-2-methoxybenzyl)amino)-3-hydroxybutanoate;
isopropyl 2-(4-(3-chloro-4-(2-chloro-3-(6-methoxy-5-((methylamino)methyl)pyridin-2-yl)phenyl)pyridin-2-yl)-2-methoxybenzyl)-2-azaspiro[3.3]heptane-6-carboxylate;
1-(4-(((6-(3-(2-(4-(((1-acetylpiperidin-4-yl)amino)methyl)-3-fluorophenyl)-3-chloropyridin-4-yl)-2-chlorophenyl)-2-methoxypyridin-3-yl)methyl)amino)piperidin-1-yl)ethan-1-one;
1-(6-(2-chloro-3-(3-chloro-2-(3-fluoro-4-((methylamino)methyl)phenyl)pyridin-4-yl)phenyl)-2-methoxypyridin-3-yl)-N-methylmethanamine;
2-((6-(2-chloro-3-(3-chloro-2-(3-fluoro-4-((7-oxo-2,6-diazaspiro[3.4]octan-2-yl)methyl)phenyl)pyridin-4-yl)phenyl)-2-methoxypyridin-3-yl)methyl)-2,6-diazaspiro[3.4]octan-7-one;
1-(4-(((6-(3-(2-(4-(((1-acetylpiperidin-4-yl)amino)methyl)-3-fluoro-5-methoxyphenyl)-3-chloropyridin-4-yl)-2-chlorophenyl)-2-methoxypyridin-3-yl)methyl)amino)piperidin-1-yl)ethan-1-one;
1-(6-(2-chloro-3-(3-chloro-2-(3-fluoro-5-methoxy-4-((methylamino)methyl)phenyl)pyridin-4-yl)phenyl)-2-methoxypyridin-3-yl)-N-methylmethanamine;
2-(4-(3-chloro-4-(2-chloro-3-(6-methoxy-5-((7-oxo-2,6-diazaspiro[3.4]octan-2-yl)methyl)pyridin-2-yl)phenyl)pyridin-2-yl)-2-fluoro-6-methoxybenzyl)-2,6-diazaspiro[3.4]octan-7-one;
(S)-2-((6-(3-chloro-4-(2-chloro-3-(6-methoxy-5-((((5-oxopyrrolidin-2-yl)methyl)amino)methyl)pyridin-2-yl)phenyl)pyridin-2-yl)-1-methyl-1H-indazol-3-yl)methyl)-2,6-diazaspiro[3.4]octan-7-one;
(R)-2-((6-(3-chloro-4-(2-chloro-3-(6-methoxy-5-((((5-oxopyrrolidin-2-yl)methyl)amino)methyl)pyridin-2-yl)phenyl)pyridin-2-yl)-1-methyl-1H-indazol-3-yl)methyl)-2,6-diazaspiro[3.4]octan-7-one;
(S)-5-((((6-(3-(2-(2-((1-acetylpiperidin-4-yl)methyl)-8-chloro-1,2,3,4-tetrahydroisoquinolin-6-yl)-3-chloropyridin-4-yl)-2-chlorophenyl)-2-methoxypyridin-3-yl)methyl)amino)methyl)pyrrolidin-2-one;
(R)-5-((((6-(3-(2-(2-((1-acetylpiperidin-4-yl)methyl)-8-chloro-1,2,3,4-tetrahydroisoquinolin-6-yl)-3-chloropyridin-4-yl)-2-chlorophenyl)-2-methoxypyridin-3-yl)methyl)amino)methyl)pyrrolidin-2-one;
6-(2-chloro-3-(3-chloro-2-(3-methoxy-4-(((((S)-5-oxopyrrolidin-2-yl)methyl)amino)methyl)phenyl)pyridin-4-yl)phenyl)-2-(2-((((S)-5-oxopyrrolidin-2-yl)methyl)amino)ethyl)-1,2-dihydro-3H-pyrrolo[3,4-c]pyridin-3-one;
6-(2-chloro-3-(3-chloro-2-(3-methoxy-4-(((((S)-5-oxopyrrolidin-2-yl)methyl)amino)methyl)phenyl)pyridin-4-yl)phenyl)-2-(2-((((R)-5-oxopyrrolidin-2-yl)methyl)amino)ethyl)-1,2-dihydro-3H-pyrrolo[3,4-c]pyridin-3-one;
6-(2-chloro-3-(3-chloro-2-(3-methoxy-4-(((((R)-5-oxopyrrolidin-2-yl)methyl)amino)methyl)phenyl)pyridin-4-yl)phenyl)-2-(2-((((S)-5-oxopyrrolidin-2-yl)methyl)amino)ethyl)-1,2-dihydro-3H-pyrrolo[3,4-c]pyridin-3-one;
6-(2-chloro-3-(3-chloro-2-(3-methoxy-4-(((((R)-5-oxopyrrolidin-2-yl)methyl)amino)methyl)phenyl)pyridin-4-yl)phenyl)-2-(2-((((R)-5-oxopyrrolidin-2-yl)methyl)amino)ethyl)-1,2-dihydro-3H-pyrrolo[3,4-c]pyridin-3-one;
(S)-5-((((6-(2-chloro-3-(3-chloro-2-(3-methoxy-4-(((((S)-5-oxopyrrolidin-2-yl)methyl)amino)methyl)phenyl)pyridin-4-yl)phenyl)imidazo[1,2-a]pyrazin-2-yl)methyl)amino)methyl)pyrrolidin-2-one;
(S)-5-((((6-(2-chloro-3-(3-chloro-2-(3-methoxy-4-(((((R)-5-oxopyrrolidin-2-yl)methyl)amino)methyl)phenyl)pyridin-4-yl)phenyl)imidazo[1,2-a]pyrazin-2-yl)methyl)amino)methyl)pyrrolidin-2-one;
(R)-5-((((6-(2-chloro-3-(3-chloro-2-(3-methoxy-4-(((((S)-5-oxopyrrolidin-2-yl)methyl)amino)methyl)phenyl)pyridin-4-yl)phenyl)imidazo[1,2-a]pyrazin-2-yl)methyl)amino)methyl)pyrrolidin-2-one;
(R)-5-((((6-(2-chloro-3-(3-chloro-2-(3-methoxy-4-(((((R)-5-oxopyrrolidin-2-yl)methyl)amino)methyl)phenyl)pyridin-4-yl)phenyl)imidazo[1,2-a]pyrazin-2-yl)methyl)amino)methyl)pyrrolidin-2-one;
(S)-2-(4-(3-chloro-4-(1-((6-methoxy-5-((7-oxo-2,6-diazaspiro[3.4]octan-2-yl)methyl)-3-(trifluoromethyl)pyridin-2-yl)oxy)-2,3-dihydro-1H-inden-4-yl)pyridin-2-yl)-2-methoxybenzyl)-2,6-diazaspiro[3.4]octan-7-one;
(R)-2-(4-(3-chloro-4-(1-((6-methoxy-5-((7-oxo-2,6-diazaspiro[3.4]octan-2-yl)methyl)-3-(trifluoromethyl)pyridin-2-yl)oxy)-2,3-dihydro-1H-inden-4-yl)pyridin-2-yl)-2-methoxybenzyl)-2,6-diazaspiro[3.4]octan-7-one;
(S)-3-(6-(3-chloro-4-(2-chloro-3-(5-(((2-hydroxypropyl)amino)methyl)-6-methoxypyridin-2-yl)phenyl)pyridin-2-yl)-8-methoxy-3,4-dihydroisoquinolin-2(1H)-yl)propanoic acid;
(R)-3-(6-(3-chloro-4-(2-chloro-3-(5-(((2-hydroxypropyl)amino)methyl)-6-methoxypyridin-2-yl)phenyl)pyridin-2-yl)-8-methoxy-3,4-dihydroisoquinolin-2(1H)-yl)propanoic acid;
3-(6-(3-chloro-4-(2-chloro-3-(6-methoxy-5-((7-oxo-2,6-diazaspiro[3.4]octan-2-yl)methyl)pyridin-2-yl)phenyl)pyridin-2-yl)-8-methoxy-3,4-dihydroisoquinolin-2(1H)-yl)propanoic acid;
methyl 1-((6-(3-chloro-4-(2-chloro-3-(6-methoxy-5-((7-oxo-2,6-diazaspiro[3.4]octan-2-yl)methyl)pyridin-2-yl)phenyl)pyridin-2-yl)-8-methoxy-3,4-dihydroisoquinolin-2(1H)-yl)methyl)cyclopropane-1-carboxylate;
2-((6-(2-chloro-3-(3-chloro-2-(2-(3-fluoro-2-hydroxypropyl)-8-methoxy-1,2,3,4-tetrahydroisoquinolin-6-yl)pyridin-4-yl)phenyl)-2-methoxypyridin-3-yl)methyl)-2,6-diazaspiro[3.4]octan-7-one;
(S)-2-((6-(2-chloro-3-(3-chloro-2-(2-(3-fluoro-2-hydroxypropyl)-8-methoxy-1,2,3,4-tetrahydroisoquinolin-6-yl)pyridin-4-yl)phenyl)-2-methoxypyridin-3-yl)methyl)-2,6-diazaspiro[3.4]octan-7-one;
(R)-2-((6-(2-chloro-3-(3-chloro-2-(2-(3-fluoro-2-hydroxypropyl)-8-methoxy-1,2,3,4-tetrahydroisoquinolin-6-yl)pyridin-4-yl)phenyl)-2-methoxypyridin-3-yl)methyl)-2,6-diazaspiro[3.4]octan-7-one;
1-((6-(3-chloro-4-(2-chloro-3-(6-methoxy-5-((7-oxo-2,6-diazaspiro[3.4]octan-2-yl)methyl)pyridin-2-yl)phenyl)pyridin-2-yl)-8-methoxy-3,4-dihydroisoquinolin-2(1H)-yl)methyl)cyclopropane-1-carboxylic acid;
N-((6-(2-chloro-3-(3-chloro-2-(3-methoxy-4-(((tetrahydro-2H-pyran-4-yl)amino)methyl)phenyl)pyridin-4-yl)phenyl)-2-methoxypyridin-3-yl)methyl)tetrahydro-2H-pyran-4-amine;
4-(((6-(2-chloro-3-(3-chloro-2-(4-(((1,1-dioxidotetrahydro-2H-thiopyran-4-yl)amino)methyl)-3-methoxyphenyl)pyridin-4-yl)phenyl)-2-methoxypyridin-3-yl)methyl)amino)tetrahydro-2H-thiopyran 1,1-dioxide;
1-(3-(((6-(3-(2-(4-(((1-acetylazetidin-3-yl)amino)methyl)-3-methoxyphenyl)-3-chloropyridin-4-yl)-2-chlorophenyl)-2-methoxypyridin-3-yl)methyl)amino)azetidin-1-yl)ethan-1-one;
(S)-1-(((6-(2-chloro-3-(3-chloro-2-(4-((((S)-2-hydroxypropyl)amino)methyl)-3-methoxyphenyl)pyridin-4-yl)phenyl)-2-methoxypyridin-3-yl)methyl)amino)propan-2-ol;
(R)-1-(((6-(2-chloro-3-(3-chloro-2-(4-((((S)-2-hydroxypropyl)amino)methyl)-3-methoxyphenyl)pyridin-4-yl)phenyl)-2-methoxypyridin-3-yl)methyl)amino)propan-2-ol;
(S)-1-(((6-(2-chloro-3-(3-chloro-2-(4-((((R)-2-hydroxypropyl)amino)methyl)-3-methoxyphenyl)pyridin-4-yl)phenyl)-2-methoxypyridin-3-yl)methyl)amino)propan-2-ol;
(R)-1-(((6-(2-chloro-3-(3-chloro-2-(4-((((R)-2-hydroxypropyl)amino)methyl)-3-methoxyphenyl)pyridin-4-yl)phenyl)-2-methoxypyridin-3-yl)methyl)amino)propan-2-ol;
2-((4-(3-chloro-4-(2-chloro-3-(6-methoxy-5-(((5-oxo-6-azaspiro[3.4]octan-2-yl)amino)methyl)pyridin-2-yl)phenyl)pyridin-2-yl)-2-methoxybenzyl)amino)-6-azaspiro[3.4]octan-5-one;
1-(2-(((6-(2-chloro-3-(3-chloro-2-(3-methoxy-4-(((2-(2-oxopyrrolidin-1-yl)ethyl)amino)methyl)phenyl)pyridin-4-yl)phenyl)-2-methoxypyridin-3-yl)methyl)amino)ethyl)pyrrolidin-2-one;
(S)-2-((3'-chloro-6-methoxy-2'-(1-((6-methoxy-5-((7-oxo-2,6-diazaspiro[3.4]octan-2-yl)methyl)-3-(trifluoromethyl)pyrazin-2-yl)amino)-2,3-dihydro-1H-inden-4-yl)-[2,4'-bipyridin]-5-yl)methyl)-2,6-diazaspiro[3.4]octan-7-one;
(R)-2-((3'-chloro-6-methoxy-2'-(1-((6-methoxy-5-((7-oxo-2,6-diazaspiro[3.4]octan-2-yl)methyl)-3-(trifluoromethyl)pyrazin-2-yl)amino)-2,3-dihydro-1H-inden-4-yl)-[2,4'-bipyridin]-5-yl)methyl)-2,6-diazaspiro[3.4]octan-7-one;
(S)-1-(6-((5-((4-(5-((6-acetyl-2,6-diazaspiro[3.3]heptan-2-yl)methyl)-3'-chloro-6-methoxy-[2,4'-bipyridin]-2'-yl)-2,3-dihydro-1H-inden-1-yl)amino)-3-methoxy-6-(trifluoromethyl)pyrazin-2-yl)methyl)-2,6-diazaspiro[3.3]heptan-2-yl)ethan-1-one;
(R)-1-(6-((5-((4-(5-((6-acetyl-2,6-diazaspiro[3.3]heptan-2-yl)methyl)-3'-chloro-6-methoxy-[2,4'-bipyridin]-2'-yl)-2,3-dihydro-1H-inden-1-yl)amino)-3-methoxy-6-(trifluoromethyl)pyrazin-2-yl)methyl)-2,6-diazaspiro[3.3]heptan-2-yl)ethan-1-one;
(S)-2-(4-(3-chloro-4-(1-((6-methoxy-5-((7-oxo-2,6-diazaspiro[3.4]octan-2-yl)methyl)-3-(trifluoromethyl)pyridin-2-yl)oxy)-2,3-dihydro-1H-inden-4-yl)pyridin-2-yl)-2-fluoro-6-methoxybenzyl)-2,6-diazaspiro[3.4]octan-7-one;
(R)-2-(4-(3-chloro-4-(1-((6-methoxy-5-((7-oxo-2,6-diazaspiro[3.4]octan-2-yl)methyl)-3-(trifluoromethyl)pyridin-2-yl)oxy)-2,3-dihydro-1H-inden-4-yl)pyridin-2-yl)-2-fluoro-6-methoxybenzyl)-2,6-diazaspiro[3.4]octan-7-one;
(S)-5-((((6-(2-chloro-3-(3-chloro-2-(8-chloro-2-(oxetan-3-ylmethyl)-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(8-chloro-2-(oxetan-3-ylmethyl)-1,2,3,4-tetrahydroisoquinolin-6-yl)pyridin-4-yl)phenyl)-2-methoxypyridin-3-yl)methyl)amino)methyl)pyrrolidin-2-one;
(S)-5-((((6-(3-(2-(3-((6-acetyl-2,6-diazaspiro[3.3]heptan-2-yl)methyl)-1-methyl-1H-indazol-6-yl)-3-chloropyridin-4-yl)-2-chlorophenyl)-2-methoxypyridin-3-yl)methyl)amino)methyl)pyrrolidin-2-one;
(R)-5-((((6-(3-(2-(3-((6-acetyl-2,6-diazaspiro[3.3]heptan-2-yl)methyl)-1-methyl-1H-indazol-6-yl)-3-chloropyridin-4-yl)-2-chlorophenyl)-2-methoxypyridin-3-yl)methyl)amino)methyl)pyrrolidin-2-one;
(S)-1-((6-(3-chloro-4-(2-chloro-3-(6-methoxy-5-(((((S)-5-oxopyrrolidin-2-yl)methyl)amino)methyl)pyridin-2-yl)phenyl)pyridin-2-yl)-1-methyl-1H-indazol-3-yl)methyl)pyrrolidine-3-carboxylic acid;
(S)-1-((6-(3-chloro-4-(2-chloro-3-(6-methoxy-5-(((((R)-5-oxopyrrolidin-2-yl)methyl)amino)methyl)pyridin-2-yl)phenyl)pyridin-2-yl)-1-methyl-1H-indazol-3-yl)methyl)pyrrolidine-3-carboxylic acid;
(R)-1-((6-(3-chloro-4-(2-chloro-3-(6-methoxy-5-(((((S)-5-oxopyrrolidin-2-yl)methyl)amino)methyl)pyridin-2-yl)phenyl)pyridin-2-yl)-1-methyl-1H-indazol-3-yl)methyl)pyrrolidine-3-carboxylic acid;
(R)-1-((6-(3-chloro-4-(2-chloro-3-(6-methoxy-5-(((((R)-5-oxopyrrolidin-2-yl)methyl)amino)methyl)pyridin-2-yl)phenyl)pyridin-2-yl)-1-methyl-1H-indazol-3-yl)methyl)pyrrolidine-3-carboxylic acid;
(S)-5-((((6-(2-chloro-3-(3-chloro-2-(8-methoxy-2-(((((S)-5-oxopyrrolidin-2-yl)methyl)amino)methyl)imidazo[1,2-a]pyridin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(8-methoxy-2-(((((R)-5-oxopyrrolidin-2-yl)methyl)amino)methyl)imidazo[1,2-a]pyridin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(8-methoxy-2-(((((S)-5-oxopyrrolidin-2-yl)methyl)amino)methyl)imidazo[1,2-a]pyridin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(8-methoxy-2-(((((R)-5-oxopyrrolidin-2-yl)methyl)amino)methyl)imidazo[1,2-a]pyridin-6-yl)pyridin-4-yl)phenyl)-2-methoxypyridin-3-yl)methyl)amino)methyl)pyrrolidin-2-one;
1-(4-(((5-(3-(2-(4-(((1-acetylpiperidin-4-yl)amino)methyl)-3-methoxyphenyl)-3-chloropyridin-4-yl)-2-chlorophenyl)-3-methoxypyrazin-2-yl)methyl)amino)piperidin-1-yl)ethan-1-one;
(S)-5-((((6-(2-chloro-3-(3-chloro-2-(2-((((1r,3r)-3-hydroxy-3-methylcyclobutyl)amino)methyl)-8-methoxyimidazo[1,2-a]pyridin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(2-((((1s,3s)-3-hydroxy-3-methylcyclobutyl)amino)methyl)-8-methoxyimidazo[1,2-a]pyridin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(2-((((1s,3r)-3-hydroxy-3-methylcyclobutyl)amino)methyl)-8-methoxyimidazo[1,2-a]pyridin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(2-((((1r,3s)-3-hydroxy-3-methylcyclobutyl)amino)methyl)-8-methoxyimidazo[1,2-a]pyridin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-((((1r,3s)-3-hydroxy-3-methylcyclobutyl)amino)methyl)-8-methoxyimidazo[1,2-a]pyridin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-((((1s,3s)-3-hydroxy-3-methylcyclobutyl)amino)methyl)-8-methoxyimidazo[1,2-a]pyridin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-((((1s,3r)-3-hydroxy-3-methylcyclobutyl)amino)methyl)-8-methoxyimidazo[1,2-a]pyridin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-((((1r,3r)-3-hydroxy-3-methylcyclobutyl)amino)methyl)-8-methoxyimidazo[1,2-a]pyridin-6-yl)pyridin-4-yl)phenyl)-2-methoxypyridin-3-yl)methyl)amino)methyl)pyrrolidin-2-one;
1,1'-(((((3,3'-dichloro-[4,4'-bipyridine]-2,2'-diyl)bis(2-fluoro-6-methoxy-4,1-phenylene))bis(methylene))bis(azanediyl))bis(piperidine-4,1-diyl))bis(ethan-1-one);
(S)-2-((6-(3-chloro-4-(2-chloro-3-(6-methoxy-5-((((5-oxopyrrolidin-2-yl)methyl)amino)methyl)pyridin-2-yl)phenyl)pyridin-2-yl)-8-methoxyimidazo[1,2-a]pyridin-2-yl)methyl)-2,6-diazaspiro[3.4]octan-7-one;
(R)-2-((6-(3-chloro-4-(2-chloro-3-(6-methoxy-5-((((5-oxopyrrolidin-2-yl)methyl)amino)methyl)pyridin-2-yl)phenyl)pyridin-2-yl)-8-methoxyimidazo[1,2-a]pyridin-2-yl)methyl)-2,6-diazaspiro[3.4]octan-7-one;
1-(4-(((6-(3-(2-(4-(((1-acetylpiperidin-4-yl)(methyl)amino)methyl)-3-methoxyphenyl)-3-chloropyridin-4-yl)-2-chlorophenyl)-2-methoxypyridin-3-yl)methyl)amino)piperidin-1-yl)ethan-1-one;
(4-(((6-(2-chloro-3-(3-chloro-2-(4-(((1-(cyclopropanecarbonyl)piperidin-4-yl)amino)methyl)-3-fluoro-5-methoxyphenyl)pyridin-4-yl)phenyl)-2-methoxypyridin-3-yl)methyl)amino)piperidin-1-yl)(cyclopropyl)methanone;
(S)-5-((((6-(2-chloro-3-(3-chloro-2-(2-((3-hydroxy-3-methylazetidin-1-yl)methyl)-8-methoxyimidazo[1,2-a]pyridin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-((3-hydroxy-3-methylazetidin-1-yl)methyl)-8-methoxyimidazo[1,2-a]pyridin-6-yl)pyridin-4-yl)phenyl)-2-methoxypyridin-3-yl)methyl)amino)methyl)pyrrolidin-2-one;
(S)-5-((((6-(3-(2-(2-(((1-acetylpiperidin-4-yl)amino)methyl)-8-methoxyimidazo[1,2-a]pyridin-6-yl)-3-chloropyridin-4-yl)-2-chlorophenyl)-2-methoxypyridin-3-yl)methyl)amino)methyl)pyrrolidin-2-one;
(R)-5-((((6-(3-(2-(2-(((1-acetylpiperidin-4-yl)amino)methyl)-8-methoxyimidazo[1,2-a]pyridin-6-yl)-3-chloropyridin-4-yl)-2-chlorophenyl)-2-methoxypyridin-3-yl)methyl)amino)methyl)pyrrolidin-2-one;
(S)-5-((((6-(3-(2-(2-((6-acetyl-2,6-diazaspiro[3.3]heptan-2-yl)methyl)-8-methoxyimidazo[1,2-a]pyridin-6-yl)-3-chloropyridin-4-yl)-2-chlorophenyl)-2-methoxypyridin-3-yl)methyl)amino)methyl)pyrrolidin-2-one;
(R)-5-((((6-(3-(2-(2-((6-acetyl-2,6-diazaspiro[3.3]heptan-2-yl)methyl)-8-methoxyimidazo[1,2-a]pyridin-6-yl)-3-chloropyridin-4-yl)-2-chlorophenyl)-2-methoxypyridin-3-yl)methyl)amino)methyl)pyrrolidin-2-one;
(S)-2-((6-(3-chloro-4-(2-chloro-3-(6-methoxy-5-((((5-oxopyrrolidin-2-yl)methyl)amino)methyl)pyridin-2-yl)phenyl)pyridin-2-yl)-8-methoxyimidazo[1,2-a]pyridin-2-yl)methyl)-2,5-diazaspiro[3.4]octan-6-one;
(R)-2-((6-(3-chloro-4-(2-chloro-3-(6-methoxy-5-((((5-oxopyrrolidin-2-yl)methyl)amino)methyl)pyridin-2-yl)phenyl)pyridin-2-yl)-8-methoxyimidazo[1,2-a]pyridin-2-yl)methyl)-2,5-diazaspiro[3.4]octan-6-one;
(S)-5-((((6-(3-(2-(2-(((2-oxaspiro[3.3]heptan-6-yl)amino)methyl)-8-methoxyimidazo[1,2-a]pyridin-6-yl)-3-chloropyridin-4-yl)-2-chlorophenyl)-2-methoxypyridin-3-yl)methyl)amino)methyl)pyrrolidin-2-one;
(R)-5-((((6-(3-(2-(2-(((2-oxaspiro[3.3]heptan-6-yl)amino)methyl)-8-methoxyimidazo[1,2-a]pyridin-6-yl)-3-chloropyridin-4-yl)-2-chlorophenyl)-2-methoxypyridin-3-yl)methyl)amino)methyl)pyrrolidin-2-one;
N-(1-((6-(3-(2-(4-((4-acetamidopiperidin-1-yl)methyl)-3-methoxyphenyl)-3-chloropyridin-4-yl)-2-chlorophenyl)-2-methoxypyridin-3-yl)methyl)piperidin-4-yl)acetamide;
2,2'-(((3,3'-dichloro-[4,4'-bipyridine]-2,2'-diyl)bis(2-fluoro-6-methoxy-4,1-phenylene))bis(methylene))bis(2,6-diazaspiro[3.4]octan-7-one);
2-(4-(4-(2-chloro-3-(6-methoxy-5-((7-oxo-2,6-diazaspiro[3.4]octan-2-yl)methyl)pyridin-2-yl)phenyl)-3-fluoropyridin-2-yl)-2-methoxybenzyl)-2,6-diazaspiro[3.4]octan-7-one;
N-(1-(4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)piperidin-4-yl)acetamide;
N-((6-(2-chloro-3-(3-chloro-2-(4-(((1-isopropylpiperidin-4-yl)amino)methyl)-3-methoxyphenyl)pyridin-4-yl)phenyl)-2-methoxypyridin-3-yl)methyl)-1-isopropylpiperidin-4-amine;
N-((6-(2-chloro-3-(3-chloro-2-(4-(((4,4-difluorocyclohexyl)amino)methyl)-3-methoxyphenyl)pyridin-4-yl)phenyl)-2-methoxypyridin-3-yl)methyl)-4,4-difluorocyclohexan-1-amine;
2-(4-(((6-(2-chloro-3-(3-chloro-2-(4-(((1-(2-hydroxyethyl)piperidin-4-yl)amino)methyl)-3-methoxyphenyl)pyridin-4-yl)phenyl)-2-methoxypyridin-3-yl)methyl)amino)piperidin-1-yl)ethan-1-ol;
N-(2-(((6-(3-(2-(4-(((2-acetamidoethyl)amino)methyl)-3-methoxyphenyl)-3-chloropyridin-4-yl)-2-chlorophenyl)-2-methoxypyridin-3-yl)methyl)amino)ethyl)acetamide;
1-(6-(2-chloro-3-(3-chloro-2-(3-methoxy-4-((((tetrahydro-2H-pyran-4-yl)methyl)amino)methyl)phenyl)pyridin-4-yl)phenyl)-2-methoxypyridin-3-yl)-N-((tetrahydro-2H-pyran-4-yl)methyl)methanamine;
1-(4-(((6-(2-chloro-3-(3-chloro-2-(3-fluoro-5-methoxy-4-(((1-propionylpiperidin-4-yl)amino)methyl)phenyl)pyridin-4-yl)phenyl)-2-methoxypyridin-3-yl)methyl)amino)piperidin-1-yl)propan-1-one;
2-((6-(3-(2-(4-(((1-acetylpiperidin-4-yl)amino)methyl)-3-methoxyphenyl)-3-chloropyridin-4-yl)-2-chlorophenyl)-2-methoxypyridin-3-yl)methyl)-2,6-diazaspiro[3.4]octan-7-one;
1-(4-((4-(4-(3-(5-(((1-acetylpiperidin-4-yl)(methyl)amino)methyl)-6-methoxypyridin-2-yl)-2-chlorophenyl)-3-chloropyridin-2-yl)-2-methoxybenzyl)amino)piperidin-1-yl)ethan-1-one;
1-(6-(2-chloro-3-(2-(3-methoxy-4-((methylamino)methyl)phenyl)-3-methylpyridin-4-yl)phenyl)-2-methoxypyridin-3-yl)-N-methylmethanamine;
(S)-5-((((6-(3-(2-(4-((S)-1-aminoethyl)-3-methoxyphenyl)-3-chloropyridin-4-yl)-2-chlorophenyl)-2-methoxypyridin-3-yl)methyl)amino)methyl)pyrrolidin-2-one;
(R)-5-((((6-(3-(2-(4-((S)-1-aminoethyl)-3-methoxyphenyl)-3-chloropyridin-4-yl)-2-chlorophenyl)-2-methoxypyridin-3-yl)methyl)amino)methyl)pyrrolidin-2-one;
(S)-5-((((6-(3-(2-(4-((R)-1-aminoethyl)-3-methoxyphenyl)-3-chloropyridin-4-yl)-2-chlorophenyl)-2-methoxypyridin-3-yl)methyl)amino)methyl)pyrrolidin-2-one;
(R)-5-((((6-(3-(2-(4-((R)-1-aminoethyl)-3-methoxyphenyl)-3-chloropyridin-4-yl)-2-chlorophenyl)-2-methoxypyridin-3-yl)methyl)amino)methyl)pyrrolidin-2-one;
(S)-2-((3'-chloro-6-methoxy-2'-(1-((6-methoxy-5-((7-oxo-2,6-diazaspiro[3.4]octan-2-yl)methyl)-3-(trifluoromethyl)pyridin-2-yl)oxy)-2,3-dihydro-1H-inden-4-yl)-[2,4'-bipyridin]-5-yl)methyl)-2,6-diazaspiro[3.4]octan-7-one;
(R)-2-((3'-chloro-6-methoxy-2'-(1-((6-methoxy-5-((7-oxo-2,6-diazaspiro[3.4]octan-2-yl)methyl)-3-(trifluoromethyl)pyridin-2-yl)oxy)-2,3-dihydro-1H-inden-4-yl)-[2,4'-bipyridin]-5-yl)methyl)-2,6-diazaspiro[3.4]octan-7-one;
1-(4-(((6-(3-(2-(4-(((1-acetylpiperidin-4-yl)amino)methyl)-3-methoxyphenyl)-3-fluoropyridin-4-yl)-2-chlorophenyl)-2-methoxypyridin-3-yl)methyl)amino)piperidin-1-yl)ethan-1-one;
(S)-4-((4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)amino)dihydrofuran-2(3H)-one;
(R)-4-((4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)amino)dihydrofuran-2(3H)-one;
methyl (S)-3-((4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)amino)-4-hydroxybutanoate;
methyl (R)-3-((4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)amino)-4-hydroxybutanoate;
(S)-3-((4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)amino)dihydrofuran-2(3H)-one;
(R)-3-((4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)amino)dihydrofuran-2(3H)-one;
N-(1-((6-(3-(2-(4-(((1-acetylpiperidin-4-yl)amino)methyl)-3-methoxyphenyl)-3-chloropyridin-4-yl)-2-chlorophenyl)-2-methoxypyridin-3-yl)methyl)piperidin-4-yl)acetamide;
(S)-3-((4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)amino)-4-hydroxybutanoic acid;
(R)-3-((4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)amino)-4-hydroxybutanoic acid;
(4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)-L-homoserine;
(4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)-D-homoserine;
1-(4-(((6-(2-chloro-3-(3-chloro-2-(3-methoxy-4-(((1-propionylpiperidin-4-yl)amino)methyl)phenyl)pyridin-4-yl)phenyl)-2-methoxypyridin-3-yl)methyl)amino)piperidin-1-yl)propan-1-one;
(4-(((6-(2-chloro-3-(3-chloro-2-(4-(((1-(cyclopropanecarbonyl)piperidin-4-yl)amino)methyl)-3-methoxyphenyl)pyridin-4-yl)phenyl)-2-methoxypyridin-3-yl)methyl)amino)piperidin-1-yl)(cyclopropyl)methanone;
1-(4-(((6-(2-chloro-3-(3-chloro-2-(4-(((1-isobutyrylpiperldin-4-yl)amino)methyl)-3-methoxyphenyl)pyridin-4-yl)phenyl)-2-methoxypyridin-3-yl)methyl)amino)piperidin-1-yl)-2-methylpropan-1-one;
1-(4-(((6-(2-chloro-3-(3-chloro-2-(3-fluoro-4-(((1-isobutyrylpiperidin-4-yl)amino)methyl)-5-methoxyphenyl)pyridin-4-yl)phenyl)-2-methoxypyridin-3-yl)methyl)amino)piperidin-1-yl)-2-methylpropan-1-one;
N-((6-(2-chloro-3-(3-chloro-2-(3-fluoro-5-methoxy-4-(((tetrahydro-2H-pyran-4-yl)amino)methyl)phenyl)pyridin-4-yl)phenyl)-2-methoxypyridin-3-yl)methyl)tetrahydro-2H-pyran-4-amine;
4-(((6-(2-chloro-3-(3-chloro-2-(4-(((1,1-dioxidotetrahydro-2H-thiopyran-4-yl)amino)methyl)-3-fluoro-5-methoxyphenyl)pyridin-4-yl)phenyl)-2-methoxypyridin-3-yl)methyl)amino)tetrahydro-2H-thiopyran 1,1-dioxide;
(S)-1-((6-(3-chloro-4-(2-chloro-3-(6-methoxy-5-((((5-oxopyrrolidin-2-yl)methyl)amino)methyl)pyridin-2-yl)phenyl)pyridin-2-yl)-8-methoxyimidazo[1,2-a]pyridin-2-yl)methyl)azetidine-3-carboxylic acid;
(R)-1-((6-(3-chloro-4-(2-chloro-3-(6-methoxy-5-((((5-oxopyrrolidin-2-yl)methyl)amino)methyl)pyridin-2-yl)phenyl)pyridin-2-yl)-8-methoxyimidazo[1,2-a]pyridin-2-yl)methyl)azetidine-3-carboxylic acid;
(S)-1-(2-(((6-(3-chloro-4-(2-chloro-3-(6-methoxy-5-((((5-oxopyrrolidin-2-yl)methyl)amino)methyl)pyridin-2-yl)phenyl)pyridin-2-yl)-8-methoxyimidazo[1,2-a]pyridin-2-yl)methyl)amino)ethyl)cyclopropane-1-carboxylic acid;
(R)-1-(2-(((6-(3-chloro-4-(2-chloro-3-(6-methoxy-5-((((5-oxopyrrolidin-2-yl)methyl)amino)methyl)pyridin-2-yl)phenyl)pyridin-2-yl)-8-methoxyimidazo[1,2-a]pyridin-2-yl)methyl)amino)ethyl)cyclopropane-1-carboxylic acid;
(4-(((6-(2-chloro-3-(3-chloro-2-(4-(((1-(cyclobutanecarbonyl)piperidin-4-yl)amino)methyl)-3-methoxyphenyl)pyridin-4-yl)phenyl)-2-methoxypyridin-3-yl)methyl)amino)piperidin-1-yl)(cyclobutyl)methanone;
7-(4-(3-chloro-4-(2-chloro-3-(6-methoxy-5-((2-oxo-1,7-diazaspiro[3.5]nonan-7-yl)methyl)pyridin-2-yl)phenyl)pyridin-2-yl)-2-methoxybenzyl)-1,7-diazaspiro[3.5]nonan-2-one;
(R)-5-((((6-(2-chloro-3-(3-chloro-2-(3-methoxy-4-(((((R)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
(R)-5-((((6-(2-chloro-3-(3-chloro-2-(3-methoxy-4-(((((S)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
(S)-5-((((6-(2-chloro-3-(3-chloro-2-(3-methoxy-4-(((((R)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
(S)-5-((((6-(2-chloro-3-(3-chloro-2-(3-methoxy-4-(((((S)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
N-(1-((6-(2-chloro-3-(3-chloro-2-(3-methoxy-4-((7-oxo-2,6-diazaspiro[3.4]octan-2-yl)methyl)phenyl)pyridin-4-yl)phenyl)-2-methoxypyridin-3-yl)methyl)piperidin-4-yl)acetamide;
(R)-N-(1-((6-(2-chloro-3-(3-chloro-2-(3-methoxy-4-((((5-oxopyrrolidin-2-yl)methyl)amino)methyl)phenyl)pyridin-4-yl)phenyl)-2-methoxypyridin-3-yl)methyl)piperidin-4-yl)acetamide;
(S)-N-(1-((6-(2-chloro-3-(3-chloro-2-(3-methoxy-4-((((5-oxopyrrolidin-2-yl)methyl)amino)methyl)phenyl)pyridin-4-yl)phenyl)-2-methoxypyridin-3-yl)methyl)piperidin-4-yl)acetamide;
(S)-N-(1-((6-(2-chloro-3-(3-chloro-2-(3-methoxy-4-((((5-oxopyrrolidin-2-yl)methyl)amino)methyl)phenyl)pyridin-4-yl)phenyl)-2-methoxypyridin-3-yl)methyl)piperidin-4-yl)acetamide;
(R)-N-(1-((6-(2-chloro-3-(3-chloro-2-(3-methoxy-4-((((5-oxopyrrolidin-2-yl)methyl)amino)methyl)phenyl)pyridin-4-yl)phenyl)-2-methoxypyridin-3-yl)methyl)piperidin-4-yl)acetamide;
N-(3-(((6-(3-(2-(4-(((3-acetamidopropyl)amino)methyl)-3-methoxyphenyl)-3-chloropyridin-4-yl)-2-chlorophenyl)-2-methoxypyridin-3-yl)methyl)amino)propyl)acetamide;
4-(((6-(2-chloro-3-(3-chloro-2-(4-(((2,6-dioxopiperidin-4-yl)amino)methyl)-3-methoxyphenyl)pyridin-4-yl)phenyl)-2-methoxypyridin-3-yl)methyl)amino)piperidine-2,6-dione;
1-(4-(((6-(2-chloro-3-(3-chloro-2-(4-((((1r,4r)-4-hydroxycyclohexyl)amino)methyl)-3-methoxyphenyl)pyridin-4-yl)phenyl)-2-methoxypyridin-3-yl)methyl)amino)piperidin-1-yl)ethan-1-one;
1-(4-(((6-(2-chloro-3-(3-chloro-2-(4-((((1r,4s)-4-hydroxycyclohexyl)amino)methyl)-3-methoxyphenyl)pyridin-4-yl)phenyl)-2-methoxypyridin-3-yl)methyl)amino)piperidin-1-yl)ethan-1-one;
1-(4-(((6-(2-chloro-3-(3-chloro-2-(4-((((1s,4r)-4-hydroxycyclohexyl)amino)methyl)-3-methoxyphenyl)pyridin-4-yl)phenyl)-2-methoxypyridin-3-yl)methyl)amino)piperidin-1-yl)ethan-1-one;
1-(4-(((6-(2-chloro-3-(3-chloro-2-(4-((((1s,4s)-4-hydroxycyclohexyl)amino)methyl)-3-methoxyphenyl)pyridin-4-yl)phenyl)-2-methoxypyridin-3-yl)methyl)amino)piperidin-1-yl)ethan-1-one;
1-(4-(((6-(2-chloro-3-(3-chloro-2-(4-((6-hydroxy-2-azaspiro[3.3]heptan-2-yl)methyl)-3-methoxyphenyl)pyridin-4-yl)phenyl)-2-methoxypyridin-3-yl)methyl)amino)piperidin-1-yl)ethan-1-one;
1-(2-methoxy-4-(4-(3-(6-methoxy-5-((methylamino)methyl)pyridin-2-yl)-2-methylphenyl)-3-methylpyridin-2-yl)phenyl)-N-methylmethanamine;
1-(4-(((6-(3-(2-(4-(((1-acetylpiperidin-4-yl)amino)methyl)-3-methoxyphenyl)-3-methylpyridin-4-yl)-2-methylphenyl)-2-methoxypyridin-3-yl)methyl)amino)piperidin-1-yl)ethan-1-one;
(1r,4r)-4-(((6-(2-chloro-3-(3-chloro-2-(4-((((1r,4r)-4-hydroxycyclohexyl)amino)methyl)-3-methoxyphenyl)pyridin-4-yl)phenyl)-2-methoxypyridin-3-yl)methyl)amino)cyclohexan-1-ol;
(1r,4r)-4-(((6-(2-chloro-3-(3-chloro-2-(4-((((1r,4s)-4-hydroxycyclohexyl)amino)methyl)-3-methoxyphenyl)pyridin-4-yl)phenyl)-2-methoxypyridin-3-yl)methyl)amino)cyclohexan-1-ol;
(1r,4r)-4-(((6-(2-chloro-3-(3-chloro-2-(4-((((1s,4r)-4-hydroxycyclohexyl)amino)methyl)-3-methoxyphenyl)pyridin-4-yl)phenyl)-2-methoxypyridin-3-yl)methyl)amino)cyclohexan-1-ol;
(1r,4r)-4-(((6-(2-chloro-3-(3-chloro-2-(4-((((1s,4s)-4-hydroxycyclohexyl)amino)methyl)-3-methoxyphenyl)pyridin-4-yl)phenyl)-2-methoxypyridin-3-yl)methyl)amino)cyclohexan-1-ol;
(1r,4s)-4-(((6-(2-chloro-3-(3-chloro-2-(4-((((1r,4r)-4-hydroxycyclohexyl)amino)methyl)-3-methoxyphenyl)pyridin-4-yl)phenyl)-2-methoxypyridin-3-yl)methyl)amino)cyclohexan-1-ol;
(1r,4s)-4-(((6-(2-chloro-3-(3-chloro-2-(4-((((1r,4s)-4-hydroxycyclohexyl)amino)methyl)-3-methoxyphenyl)pyridin-4-yl)phenyl)-2-methoxypyridin-3-yl)methyl)amino)cyclohexan-1-ol;
(1r,4s)-4-(((6-(2-chloro-3-(3-chloro-2-(4-((((1s,4r)-4-hydroxycyclohexyl)amino)methyl)-3-methoxyphenyl)pyridin-4-yl)phenyl)-2-methoxypyridin-3-yl)methyl)amino)cyclohexan-1-ol;
(1r,4s)-4-(((6-(2-chloro-3-(3-chloro-2-(4-((((1s,4s)-4-hydroxycyclohexyl)amino)methyl)-3-methoxyphenyl)pyridin-4-yl)phenyl)-2-methoxypyridin-3-yl)methyl)amino)cyclohexan-1-ol;
(1s,4r)-4-(((6-(2-chloro-3-(3-chloro-2-(4-((((1r,4r)-4-hydroxycyclohexyl)amino)methyl)-3-methoxyphenyl)pyridin-4-yl)phenyl)-2-methoxypyridin-3-yl)methyl)amino)cyclohexan-1-ol;
(1s,4r)-4-(((6-(2-chloro-3-(3-chloro-2-(4-((((1r,4s)-4-hydroxycyclohexyl)amino)methyl)-3-methoxyphenyl)pyridin-4-yl)phenyl)-2-methoxypyridin-3-yl)methyl)amino)cyclohexan-1-ol;
(1s,4r)-4-(((6-(2-chloro-3-(3-chloro-2-(4-((((1s,4r)-4-hydroxycyclohexyl)amino)methyl)-3-methoxyphenyl)pyridin-4-yl)phenyl)-2-methoxypyridin-3-yl)methyl)amino)cyclohexan-1-ol;
(1s,4r)-4-(((6-(2-chloro-3-(3-chloro-2-(4-((((1s,4s)-4-hydroxycyclohexyl)amino)methyl)-3-methoxyphenyl)pyridin-4-yl)phenyl)-2-methoxypyridin-3-yl)methyl)amino)cyclohexan-1-ol;
(1s,4s)-4-(((6-(2-chloro-3-(3-chloro-2-(4-((((1r,4r)-4-hydroxycyclohexyl)amino)methyl)-3-methoxyphenyl)pyridin-4-yl)phenyl)-2-methoxypyridin-3-yl)methyl)amino)cyclohexan-1-ol;
(1s,4s)-4-(((6-(2-chloro-3-(3-chloro-2-(4-((((1r,4s)-4-hydroxycyclohexyl)amino)methyl)-3-methoxyphenyl)pyridin-4-yl)phenyl)-2-methoxypyridin-3-yl)methyl)amino)cyclohexan-1-ol;
(1s,4s)-4-(((6-(2-chloro-3-(3-chloro-2-(4-((((1s,4r)-4-hydroxycyclohexyl)amino)methyl)-3-methoxyphenyl)pyridin-4-yl)phenyl)-2-methoxypyridin-3-yl)methyl)amino)cyclohexan-1-ol;
(1s,4s)-4-(((6-(2-chloro-3-(3-chloro-2-(4-((((1s,4s)-4-hydroxycyclohexyl)amino)methyl)-3-methoxyphenyl)pyridin-4-yl)phenyl)-2-methoxypyridin-3-yl)methyl)amino)cyclohexan-1-ol;
N-(4-(4-(3-(5-(((2-oxaspiro[3.3]heptan-6-yl)amino)methyl)-6-methoxypyridin-2-yl)-2-chlorophenyl)-3-chloropyridin-2-yl)-2-methoxybenzyl)-2-oxaspiro[3.3]heptan-6-amine;
(S)-2-(4-(3-chloro-4-(1-((6-methoxy-5-((7-oxo-2,6-diazaspiro[3.4]octan-2-yl)methyl)-3-(trifluoromethyl)pyrazin-2-yl)amino)-2,3-dihydro-1H-inden-4-yl)pyridin-2-yl)-2-methoxybenzyl)-2,6-diazaspiro[3.4]octan-7-one;
(R)-2-(4-(3-chloro-4-(1-((6-methoxy-5-((7-oxo-2,6-diazaspiro[3.4]octan-2-yl)methyl)-3-(trifluoromethyl)pyrazin-2-yl)amino)-2,3-dihydro-1H-inden-4-yl)pyridin-2-yl)-2-methoxybenzyl)-2,6-diazaspiro[3.4]octan-7-one;
(S)-1-(6-((5-((4-(2-(4-((6-acetyl-2,6-diazaspiro[3.3]heptan-2-yl)methyl)-3-methoxyphenyl)-3-chloropyridin-4-yl)-2,3-dihydro-1H-inden-1-yl)amino)-3-methoxy-6-(trifluoromethyl)pyrazin-2-yl)methyl)-2,6-diazaspiro[3.3]heptan-2-yl)ethan-1-one;
(R)-1-(6-((5-((4-(2-(4-((6-acetyl-2,6-diazaspiro[3.3]heptan-2-yl)methyl)-3-methoxyphenyl)-3-chloropyridin-4-yl)-2,3-dihydro-1H-inden-1-yl)amino)-3-methoxy-6-(trifluoromethyl)pyrazin-2-yl)methyl)-2,6-diazaspiro[3.3]heptan-2-yl)ethan-1-one;
2-(4-(4-(2-chloro-3-(6-methoxy-5-((7-oxo-2,6-diazaspiro[3.4]octan-2-yl)methyl)pyridin-2-yl)phenyl)-3-methylpyridin-2-yl)-2-methoxybenzyl)-2,6-diazaspiro[3.4]octan-7-one;
1-(6-((6-(3-(2-(4-((6-acetyl-2,6-diazaspiro[3.3]heptan-2-yl)methyl)-3-methoxyphenyl)-3-methylpyridin-4-yl)-2-chlorophenyl)-2-methoxypyridin-3-yl)methyl)-2,6-diazaspiro[3.3]heptan-2-yl)ethan-1-one;
(S)-5-((((6-(2-chloro-3-(3-chloro-2-(2-((((1r,3r)-3-hydroxy-3-methylcyclobutyl)amino)methyl)-8-methoxy-[1,2,4]triazolo[1,5-a]pyridin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-((((1r,3r)-3-hydroxy-3-methylcyclobutyl)amino)methyl)-8-methoxy-[1,2,4]triazolo[1,5-a]pyridin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-((((1r,3s)-3-hydroxy-3-methylcyclobutyl)amino)methyl)-8-methoxy-[1,2,4]triazolo[1,5-a]pyridin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-((((1s,3r)-3-hydroxy-3-methylcyclobutyl)amino)methyl)-8-methoxy-[1,2,4]triazolo[1,5-a]pyridin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-((((1s,3s)-3-hydroxy-3-methylcyclobutyl)amino)methyl)-8-methoxy-[1,2,4]triazolo[1,5-a]pyridin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(2-((((1r,3s)-3-hydroxy-3-methylcyclobutyl)amino)methyl)-8-methoxy-[1,2,4]triazolo[1,5-a]pyridin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(2-((((1s,3r)-3-hydroxy-3-methylcyclobutyl)amino)methyl)-8-methoxy-[1,2,4]triazolo[1,5-a]pyridin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(2-((((1s,3s)-3-hydroxy-3-methylcyclobutyl)amino)methyl)-8-methoxy-[1,2,4]triazolo[1,5-a]pyridin-6-yl)pyridin-4-yl)phenyl)-2-methoxypyridin-3-yl)methyl)amino)methyl)pyrrolidin-2-one;
1-(4-((4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)amino)piperidin-1-yl)-2-methylpropan-1-one;
N-((6-(2-chloro-3-(3-chloro-2-(3-methoxy-4-(((1-(methylsulfonyl)piperidin-4-yl)amino)methyl)phenyl)pyridin-4-yl)phenyl)-2-methoxypyridin-3-yl)methyl)-1-(methylsulfonyl)piperidin-4-amine;
5-(((6-(3-(2-(4-(((5-amino-5-oxopentyl)amino)methyl)-3-methoxyphenyl)-3-chloropyridin-4-yl)-2-chlorophenyl)-2-methoxypyridin-3-yl)methyl)amino)pentanamide;
1-((R)-3-(((6-(3-(2-(4-((((R)-1-acetylpyrrolidin-3-yl)amino)methyl)-3-methoxyphenyl)-3-chloropyridin-4-yl)-2-chlorophenyl)-2-methoxypyridin-3-yl)methyl)amino)pyrrolidin-1-yl)ethan-1-one;
1-((S)-3-(((6-(3-(2-(4-((((R)-1-acetylpyrrolidin-3-yl)amino)methyl)-3-methoxyphenyl)-3-chloropyridin-4-yl)-2-chlorophenyl)-2-methoxypyridin-3-yl)methyl)amino)pyrrolidin-1-yl)ethan-1-one;
1-((R)-3-(((6-(3-(2-(4-((((S)-1-acetylpyrrolidin-3-yl)amino)methyl)-3-methoxyphenyl)-3-chloropyridin-4-yl)-2-chlorophenyl)-2-methoxypyridin-3-yl)methyl)amino)pyrrolidin-1-yl)ethan-1-one;
1-((S)-3-(((6-(3-(2-(4-((((S)-1-acetylpyrrolidin-3-yl)amino)methyl)-3-methoxyphenyl)-3-chloropyridin-4-yl)-2-chlorophenyl)-2-methoxypyridin-3-yl)methyl)amino)pyrrolidin-1-yl)ethan-1-one;
1-(4-(((6-(3-(6'-(((1-acetylpiperidin-4-yl)amino)methyl)-3-chloro-5'-methoxy-[2,3'-bipyridin] -4-yl)-2-chlorophenyl)-2-methoxypyridin-3 -yl)methyl)amino)piperidin-1-yl)ethan-1-one;
2-((3-chloro-4-(2-chloro-3-(6-methoxy-5-((7-oxo-2,6-diazaspiro[3.4]octan-2-yl)methyl)pyridin-2-yl)phenyl)-5'-methoxy-[2,3'-bipyridin]-6'-yl)methyl)-2,6-diazaspiro[3.4]octan-7-one;
(S)-N-(1-((6-(3-chloro-4-(2-chloro-3-(6-methoxy-5-((((5-oxopyrrolidin-2-yl)methyl)amino)methyl)pyridin-2-yl)phenyl)pyridin-2-yl)-1-methyl-1H-pyrrolo[3,2-b]pyridin-3-yl)methyl)piperidin-4-yl)acetamide;
(R)-N-(1-((6-(3-chloro-4-(2-chloro-3-(6-methoxy-5-((((5-oxopyrrolidin-2-yl)methyl)amino)methyl)pyridin-2-yl)phenyl)pyridin-2-yl)-1-methyl-1H-pyrrolo[3,2-b]pyridin-3-yl)methyl)piperidin-4-yl)acetamide;
(S)-2-((6-(3-chloro-4-(2-chloro-3-(6-methoxy-5-((((5-oxopyrrolidin-2-yl)methyl)amino)methyl)pyridin-2-yl)phenyl)pyridin-2-yl)-1-methyl-1H-pyrrolo[3,2-b]pyridin-3-yl)methyl)-2,6-diazaspiro[3.4]octan-7-one;
(R)-2-((6-(3-chloro-4-(2-chloro-3-(6-methoxy-5-((((5-oxopyrrolidin-2-yl)methyl)amino)methyl)pyridin-2-yl)phenyl)pyridin-2-yl)-1-methyl-1H-pyrrolo[3,2-b]pyridin-3-yl)methyl)-2,6-diazaspiro[3.4]octan-7-one;
1-(4-(((6-(3-(2-(4-(((2-oxaspiro[3.3]heptan-6-yl)amino)methyl)-3-methoxyphenyl)-3-chloropyridin-4-yl)-2-chlorophenyl)-2-methoxypyridin-3-yl)methyl)amino)piperidin-1-yl)ethan-1-one;
1-(4-(((6-(3-(2-(4-((2-oxa-6-azaspiro[3.3]heptan-6-yl)methyl)-3-methoxyphenyl)-3-chloropyridin-4-yl)-2-chlorophenyl)-2-methoxypyridin-3-yl)methyl)amino)piperidin-1-yl)ethan-1-one;
1-(4-(((6-(2-chloro-3-(3-chloro-2-(4-(((3,3-dimethylcyclobutyl)amino)methyl)-3-methoxyphenyl)pyridin-4-yl)phenyl)-2-methoxypyridin-3-yl)methyl)amino)piperidin-1-yl)ethan-1-one;
1-(4-(((6-(3-(2-(4-(((1-acetylpiperidin-4-yl)amino)methyl)-3-methoxyphenyl)-3-chloropyridin-4-yl)-2-chlorophenyl)-2-methoxypyridin-3-yl)methyl)amino)piperidin-1-yl)-2-methylpropan-1-one;
1-(4-(((6-(2-chloro-3-(3-chloro-2-(3-methoxy-4-(((1-(methylsulfonyl)piperidin-4-yl)amino)methyl)phenyl)pyridin-4-yl)phenyl)-2-methoxypyridin-3-yl)methyl)amino)piperidin-1-yl)ethan-1-one;
(S)-5-((((6-(2-chloro-3-(3-chloro-2-(2-((((1r,4r)-4-hydroxycyclohexyl)amino)methyl)-8-methoxyimidazo[1,2-a]pyridin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-((((1r,4r)-4-hydroxycyclohexyl)amino)methyl)-8-methoxyimidazo[1,2-a]pyridin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-((((1r,4s)-4-hydroxycyclohexyl)amino)methyl)-8-methoxyimidazo[1,2-a]pyridin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-((((1s,4r)-4-hydroxycyclohexyl)amino)methyl)-8-methoxyimidazo[1,2-a]pyridin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-((((1s,4s)-4-hydroxycyclohexyl)amino)methyl)-8-methoxyimidazo[1,2-a]pyridin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(2-((((1r,4s)-4-hydroxycyclohexyl)amino)methyl)-8-methoxyimidazo[1,2-a]pyridin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(2-((((1s,4r)-4-hydroxycyclohexyl)amino)methyl)-8-methoxyimidazo[1,2-a]pyridin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(2-((((1s,4s)-4-hydroxycyclohexyl)amino)methyl)-8-methoxyimidazo[1,2-a]pyridin-6-yl)pyridin-4-yl)phenyl)-2-methoxypyridin-3-yl)methyl)amino)methyl)pyrrolidin-2-one;
(1r,3r)-3-(((6-(2-chloro-3-(3-chloro-2-(4-((((1r,3r)-3-hydroxy-3-methylcyclobutyl)amino)methyl)-3-methoxyphenyl)pyridin-4-yl)phenyl)-2-methoxypyridin-3-yl)methyl)amino)-1-methylcyclobutan-1-ol;
(1r,3r)-3-(((6-(2-chloro-3-(3-chloro-2-(4-((((1r,3s)-3-hydroxy-3-methylcyclobutyl)amino)methyl)-3-methoxyphenyl)pyridin-4-yl)phenyl)-2-methoxypyridin-3-yl)methyl)amino)-1-methylcyclobutan-1-ol;
(1r,3r)-3-(((6-(2-chloro-3-(3-chloro-2-(4-((((1s,3r)-3-hydroxy-3-methylcyclobutyl)amino)methyl)-3-methoxyphenyl)pyridin-4-yl)phenyl)-2-methoxypyridin-3-yl)methyl)amino)-1-methylcyclobutan-1-ol;
(1r,3r)-3-(((6-(2-chloro-3-(3-chloro-2-(4-((((1s,3s)-3-hydroxy-3-methylcyclobutyl)amino)methyl)-3-methoxyphenyl)pyridin-4-yl)phenyl)-2-methoxypyridin-3-yl)methyl)amino)-1-methylcyclobutan-1-ol;
(1r,3s)-3-(((6-(2-chloro-3-(3-chloro-2-(4-((((1r,3r)-3-hydroxy-3-methylcyclobutyl)amino)methyl)-3-methoxyphenyl)pyridin-4-yl)phenyl)-2-methoxypyridin-3-yl)methyl)amino)-1-methylcyclobutan-1-ol;
(1r,3s)-3-(((6-(2-chloro-3-(3-chloro-2-(4-((((1r,3s)-3-hydroxy-3-methylcyclobutyl)amino)methyl)-3-methoxyphenyl)pyridin-4-yl)phenyl)-2-methoxypyridin-3-yl)methyl)amino)-1-methylcyclobutan-1-ol;
(1r,3s)-3-(((6-(2-chloro-3-(3-chloro-2-(4-((((1s,3r)-3-hydroxy-3-methylcyclobutyl)amino)methyl)-3-methoxyphenyl)pyridin-4-yl)phenyl)-2-methoxypyridin-3-yl)methyl)amino)-1-methylcyclobutan-1-ol;
(1r,3s)-3-(((6-(2-chloro-3-(3-chloro-2-(4-((((1s,3s)-3-hydroxy-3-methylcyclobutyl)amino)methyl)-3-methoxyphenyl)pyridin-4-yl)phenyl)-2-methoxypyridin-3-yl)methyl)amino)-1-methylcyclobutan-1-ol;
(1s,3r)-3-(((6-(2-chloro-3-(3-chloro-2-(4-((((1r,3r)-3-hydroxy-3-methylcyclobutyl)amino)methyl)-3-methoxyphenyl)pyridin-4-yl)phenyl)-2-methoxypyridin-3-yl)methyl)amino)-1-methylcyclobutan-1-ol;
(1s,3r)-3-(((6-(2-chloro-3-(3-chloro-2-(4-((((1r,3s)-3-hydroxy-3-methylcyclobutyl)amino)methyl)-3-methoxyphenyl)pyridin-4-yl)phenyl)-2-methoxypyridin-3-yl)methyl)amino)-1-methylcyclobutan-1-ol;
(1s,3r)-3-(((6-(2-chloro-3-(3-chloro-2-(4-((((1s,3r)-3-hydroxy-3-methylcyclobutyl)amino)methyl)-3-methoxyphenyl)pyridin-4-yl)phenyl)-2-methoxypyridin-3-yl)methyl)amino)-1-methylcyclobutan-1-ol;
(1s,3r)-3-(((6-(2-chloro-3-(3-chloro-2-(4-((((1s,3s)-3-hydroxy-3-methylcyclobutyl)amino)methyl)-3-methoxyphenyl)pyridin-4-yl)phenyl)-2-methoxypyridin-3-yl)methyl)amino)-1-methylcyclobutan-1-ol;
(1s,3s)-3-(((6-(2-chloro-3-(3-chloro-2-(4-((((1r,3r)-3-hydroxy-3-methylcyclobutyl)amino)methyl)-3-methoxyphenyl)pyridin-4-yl)phenyl)-2-methoxypyridin-3-yl)methyl)amino)-1-methylcyclobutan-1-ol;
(1s,3s)-3-(((6-(2-chloro-3-(3-chloro-2-(4-((((1r,3s)-3-hydroxy-3-methylcyclobutyl)amino)methyl)-3-methoxyphenyl)pyridin-4-yl)phenyl)-2-methoxypyridin-3-yl)methyl)amino)-1-methylcyclobutan-1-ol;
(1s,3s)-3-(((6-(2-chloro-3-(3-chloro-2-(4-((((1s,3r)-3-hydroxy-3-methylcyclobutyl)amino)methyl)-3-methoxyphenyl)pyridin-4-yl)phenyl)-2-methoxypyridin-3-yl)methyl)amino)-1-methylcyclobutan-1-ol;
(1s,3s)-3-(((6-(2-chloro-3-(3-chloro-2-(4-((((1s,3s)-3-hydroxy-3-methylcyclobutyl)amino)methyl)-3-methoxyphenyl)pyridin-4-yl)phenyl)-2-methoxypyridin-3-yl)methyl)amino)-1-methylcyclobutan-1-ol;
2-(4-(3-chloro-4-(2-chloro-3-(5-((6-hydroxy-2-azaspiro[3.3]heptan-2-yl)methyl)-6-methoxypyridin-2-yl)phenyl)pyridin-2-yl)-2-methoxybenzyl)-2-azaspiro[3.3]heptan-6-ol;
6-(4-(4-(3-(5-((2-oxa-6-azaspiro[3.3]heptan-6-yl)methyl)-6-methoxypyridin-2-yl)-2-chlorophenyl)-3-chloropyridin-2-yl)-2-methoxybenzyl)-2-oxa-6-azaspiro[3.3]heptane;
(S)-1-((6-(3-(2-(4-(((1-acetylpiperidin-4-yl)amino)methyl)-3-methoxyphenyl)-3-chloropyridin-4-yl)-2-chlorophenyl)-2-methoxypyridin-3-yl)methyl)pyrrolidine-3-carboxylic acid;
(R)-1-((6-(3-(2-(4-(((1-acetylpiperidin-4-yl)amino)methyl)-3-methoxyphenyl)-3-chloropyridin-4-yl)-2-chlorophenyl)-2-methoxypyridin-3-yl)methyl)pyrrolidine-3-carboxylic acid;
3-(((6-(3-(2-(4-(((1-acetylpiperidin-4-yl)amino)methyl)-3-methoxyphenyl)-3-chloropyridin-4-yl)-2-chlorophenyl)-2-methoxypyridin-3-yl)methyl)amino)propanoic acid;
3-((4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)amino)propanoic acid;
1-(4-((4-(3-chloro-4-(2-chloro-3-(6-methoxy-5-(((tetrahydro-2H-pyran-4-yl)amino)methyl)pyridin-2-yl)phenyl)pyridin-2-yl)-2-methoxybenzyl)amino)piperidin-1-yl)ethan-1-one;
1-(4-(((6-(2-chloro-3-(3-chloro-2-(3-methoxy-4-(((tetrahydro-2H-pyran-4-yl)amino)methyl)phenyl)pyridin-4-yl)phenyl)-2-methoxypyridin-3-yl)methyl)amino)piperidin-1-yl)ethan-1-one;
(S)-1-(6-(3-(2-(4-((S)-1-aminoethyl)-3-methoxyphenyl)-3-chloropyridin-4-yl)-2-chlorophenyl)-2-methoxypyridin-3-yl)ethan-1-amine;
(R)-1-(6-(3-(2-(4-((S)-1-aminoethyl)-3-methoxyphenyl)-3-chloropyridin-4-yl)-2-chlorophenyl)-2-methoxypyridin-3-yl)ethan-1-amine;
(S)-1-(6-(3-(2-(4-((R)-1-aminoethyl)-3-methoxyphenyl)-3-chloropyridin-4-yl)-2-chlorophenyl)-2-methoxypyridin-3-yl)ethan-1-amine;
(R)-1-(6-(3-(2-(4-((R)-1-aminoethyl)-3-methoxyphenyl)-3-chloropyridin-4-yl)-2-chlorophenyl)-2-methoxypyridin-3-yl)ethan-1-amine;
1-(4-(((6-(3-(2-(4-(((1-acetylpiperidin-4-yl)amino)methyl)-3-(difluoromethoxy)phenyl)-3-chloropyridin-4-yl)-2-chlorophenyl)-2-methoxypyridin-3-yl)methyl)amino)piperidin-1-yl)ethan-1-one;
1-(6-(3-(3-chloro-2-(3-methoxy-4-((methylamino)methyl)phenyl)pyridin-4-yl)-2-methylphenyl)-2-methoxypyridin-3-yl)-N-methylmethanamine;
1-((6-(2-chloro-3-(3-chloro-2-(4-((3-hydroxy-3-methylazetidin-1-yl)methyl)-3-methoxyphenyl)pyridin-4-yl)phenyl)-2-methoxypyridin-3-yl)methyl)-3-methylazetidin-3-ol;
1-(4-(((6-(2-chloro-3-(3-chloro-2-(3-methoxy-4-(((1-(2-methoxyacetyl)piperidin-4-yl)amino)methyl)phenyl)pyridin-4-yl)phenyl)-2-methoxypyridin-3-yl)methyl)amino)piperidin-1-yl)-2-methoxyethan-1-one;
2-(4-(3-chloro-4-(2-chloro-3-(6-methoxy-5-((6-oxo-2,5-diazaspiro[3.4]octan-2-yl)methyl)pyridin-2-yl)phenyl)pyridin-2-yl)-2-methoxybenzyl)-2,5-diazaspiro[3.4]octan-6-one;
1-(4-(((6-(3-(2-(4-(((1-acetylpiperidin-4-yl)amino)methyl)-3-methoxyphenyl)-3-chloropyridin-4-yl)-2-methylphenyl)-2-methoxypyridin-3-yl)methyl)amino)piperidin-1-yl)ethan-1-one;
N-(1-(4-(3-chloro-4-(2-chloro-3-(6-methoxy-5-((7-oxo-2,6-diazaspiro[3.4]octan-2-yl)methyl)pyridin-2-yl)phenyl)pyridin-2-yl)-2-methoxybenzyl)piperidin-4-yl)acetamide;
N-(1-(4-(3-chloro-4-(2-chloro-3-(6-methoxy-5-((6-oxo-2,5-diazaspiro[3.4]octan-2-yl)methyl)pyridin-2-yl)phenyl)pyridin-2-yl)-2-methoxybenzyl)piperidin-4-yl)acetamide;
N-(1-(4-(4-(3-(5-((6-acetyl-2,6-diazaspiro[3.3]heptan-2-yl)methyl)-6-methoxypyridin-2-yl)-2-chlorophenyl)-3-chloropyridin-2-yl)-2-methoxybenzyl)piperidin-4-yl)acetamide;
(S)-N-(1-(4-(3-chloro-4-(2-chloro-3-(6-methoxy-5-((((5-oxopyrrolidin-2-yl)methyl)amino)methyl)pyridin-2-yl)phenyl)pyridin-2-yl)-2-methoxybenzyl)piperidin-4-yl)acetamide;
(R)-N-(1-(4-(3-chloro-4-(2-chloro-3-(6-methoxy-5-((((5-oxopyrrolidin-2-yl)methyl)amino)methyl)pyridin-2-yl)phenyl)pyridin-2-yl)-2-methoxybenzyl)piperidin-4-yl)acetamide;
2-(4-(((1-acetylpiperidin-4-yl)amino)methyl)-3-methoxyphenyl)-4-(3-(5-(((1-acetylpiperidin-4-yl)amino)methyl)-6-methoxypyridin-2-yl)-2-chlorophenyl)nicotinonitrile;
(S)-4-((((6-(2-chloro-3-(3-chloro-2-(3-methoxy-4-(((((S)-5-oxopyrrolidin-3-yl)methyl)amino)methyl)phenyl)pyridin-4-yl)phenyl)-2-methoxypyridin-3-yl)methyl)amino)methyl)pyrrolidin-2-one;
(S)-4-((((6-(2-chloro-3-(3-chloro-2-(3-methoxy-4-(((((R)-5-oxopyrrolidin-3-yl)methyl)amino)methyl)phenyl)pyridin-4-yl)phenyl)-2-methoxypyridin-3-yl)methyl)amino)methyl)pyrrolidin-2-one;
(R)-4-((((6-(2-chloro-3-(3-chloro-2-(3-methoxy-4-(((((S)-5-oxopyrrolidin-3-yl)methyl)amino)methyl)phenyl)pyridin-4-yl)phenyl)-2-methoxypyridin-3-yl)methyl)amino)methyl)pyrrolidin-2-one;
(R)-4-((((6-(2-chloro-3-(3-chloro-2-(3-methoxy-4-(((((R)-5-oxopyrrolidin-3-yl)methyl)amino)methyl)phenyl)pyridin-4-yl)phenyl)-2-methoxypyridin-3-yl)methyl)amino)methyl)pyrrolidin-2-one;
1-(4-(((6-(3-(2-(4-((4-amino-4-methylpiperidin-1-yl)methyl)-3-methoxyphenyl)-3-chloropyridin-4-yl)-2-chlorophenyl)-2-methoxypyridin-3-yl)methyl)amino)piperidin-1-yl)ethan-1-one;
(S)-4-(((4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)amino)methyl)pyrrolidin-2-one;
(R)-4-(((4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)amino)methyl)pyrrolidin-2-one;
2-(4-(3-chloro-4-(3-(6-methoxy-5-((7-oxo-2,6-diazaspiro[3.4]octan-2-yl)methyl)pyridin-2-yl)-2-methylphenyl)pyridin-2-yl)-2-methoxybenzyl)-2,6-diazaspiro[3.4]octan-7-one;
1-(6-((6-(3-(2-(4-((6-acetyl-2,6-diazaspiro[3.3]heptan-2-yl)methyl)-3-methoxyphenyl)-3-chloropyridin-4-yl)-2-methylphenyl)-2-methoxypyridin-3-yl)methyl)-2,6-diazaspiro[3.3]heptan-2-yl)ethan-1-one;
(1s,4s)-4-(((6-(2-chloro-3-(3-chloro-2-(4-((((1s,4s)-4-hydroxycyclohexyl)amino)methyl)-3-methoxyphenyl)pyridin-4-yl)phenyl)-2-methoxypyridin-3-yl)methyl)amino)cyclohexan-1-ol;
(1r,4r)-4-(((6-(2-chloro-3-(3-chloro-2-(4-((((1r,4r)-4-hydroxycyclohexyl)amino)methyl)-3-methoxyphenyl)pyridin-4-yl)phenyl)-2-methoxypyridin-3-yl)methyl)amino)cyclohexan-1-ol;
(1r,4r)-4-(((6-(2-chloro-3-(3-chloro-2-(4-((((1r,4s)-4-hydroxycyclohexyl)amino)methyl)-3-methoxyphenyl)pyridin-4-yl)phenyl)-2-methoxypyridin-3-yl)methyl)amino)cyclohexan-1-ol;
(1r,4r)-4-(((6-(2-chloro-3-(3-chloro-2-(4-((((1s,4r)-4-hydroxycyclohexyl)amino)methyl)-3-methoxyphenyl)pyridin-4-yl)phenyl)-2-methoxypyridin-3-yl)methyl)amino)cyclohexan-1-ol;
(1r,4r)-4-(((6-(2-chloro-3-(3-chloro-2-(4-((((1s,4s)-4-hydroxycyclohexyl)amino)methyl)-3-methoxyphenyl)pyridin-4-yl)phenyl)-2-methoxypyridin-3-yl)methyl)amino)cyclohexan-1-ol;
(1r,4s)-4-(((6-(2-chloro-3-(3-chloro-2-(4-((((1r,4r)-4-hydroxycyclohexyl)amino)methyl)-3-methoxyphenyl)pyridin-4-yl)phenyl)-2-methoxypyridin-3-yl)methyl)amino)cyclohexan-1-ol;
(1r,4s)-4-(((6-(2-chloro-3-(3-chloro-2-(4-((((1r,4s)-4-hydroxycyclohexyl)amino)methyl)-3-methoxyphenyl)pyridin-4-yl)phenyl)-2-methoxypyridin-3-yl)methyl)amino)cyclohexan-1-ol;
(1r,4s)-4-(((6-(2-chloro-3-(3-chloro-2-(4-((((1s,4r)-4-hydroxycyclohexyl)amino)methyl)-3-methoxyphenyl)pyridin-4-yl)phenyl)-2-methoxypyridin-3-yl)methyl)amino)cyclohexan-1-ol;
(1r,4s)-4-(((6-(2-chloro-3-(3-chloro-2-(4-((((1s,4s)-4-hydroxycyclohexyl)amino)methyl)-3-methoxyphenyl)pyridin-4-yl)phenyl)-2-methoxypyridin-3-yl)methyl)amino)cyclohexan-1-ol;
(1s,4r)-4-(((6-(2-chloro-3-(3-chloro-2-(4-((((1r,4r)-4-hydroxycyclohexyl)amino)methyl)-3-methoxyphenyl)pyridin-4-yl)phenyl)-2-methoxypyridin-3-yl)methyl)amino)cyclohexan-1-ol;
(1s,4r)-4-(((6-(2-chloro-3-(3-chloro-2-(4-((((1r,4s)-4-hydroxycyclohexyl)amino)methyl)-3-methoxyphenyl)pyridin-4-yl)phenyl)-2-methoxypyridin-3-yl)methyl)amino)cyclohexan-1-ol;
(1s,4r)-4-(((6-(2-chloro-3-(3-chloro-2-(4-((((1s,4r)-4-hydroxycyclohexyl)amino)methyl)-3-methoxyphenyl)pyridin-4-yl)phenyl)-2-methoxypyridin-3-yl)methyl)amino)cyclohexan-1-ol;
(1s,4r)-4-(((6-(2-chloro-3-(3-chloro-2-(4-((((1s,4s)-4-hydroxycyclohexyl)amino)methyl)-3-methoxyphenyl)pyridin-4-yl)phenyl)-2-methoxypyridin-3-yl)methyl)amino)cyclohexan-1-ol;
(1s,4s)-4-(((6-(2-chloro-3-(3-chloro-2-(4-((((1r,4r)-4-hydroxycyclohexyl)amino)methyl)-3-methoxyphenyl)pyridin-4-yl)phenyl)-2-methoxypyridin-3-yl)methyl)amino)cyclohexan-1-ol;
(1s,4s)-4-(((6-(2-chloro-3-(3-chloro-2-(4-((((1r,4s)-4-hydroxycyclohexyl)amino)methyl)-3-methoxyphenyl)pyridin-4-yl)phenyl)-2-methoxypyridin-3-yl)methyl)amino)cyclohexan-1-ol;
(1s,4s)-4-(((6-(2-chloro-3-(3-chloro-2-(4-((((1s,4r)-4-hydroxycyclohexyl)amino)methyl)-3-methoxyphenyl)pyridin-4-yl)phenyl)-2-methoxypyridin-3-yl)methyl)amino)cyclohexan-1-ol;
4-((6-(2-chloro-3-(3-chloro-2-(3-methoxy-4-(morpholinomethyl)phenyl)pyridin-4-yl)phenyl)-2-methoxypyridin-3-yl)methyl)morpholine;
1-((6-(2-chloro-3-(3-chloro-2-(4-((4-hydroxypiperidin-1-yl)methyl)-3-methoxyphenyl)pyridin-4-yl)phenyl)-2-methoxypyridin-3-yl)methyl)piperidin-4-ol;
1-(4-((4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)amino)piperidin-1-yl)-2-methoxyethan-1-one;
(S)-5-((((6-(2-chloro-3-(2-(3-methoxy-4-(((((S)-5-oxopyrrolidin-2-yl)methyl)amino)methyl)phenyl)-3-methylpyridin-4-yl)phenyl)-2-methoxypyridin-3-yl)methyl)amino)methyl)pyrrolidin-2-one;
(S)-5-((((6-(2-chloro-3-(2-(3-methoxy-4-(((((R)-5-oxopyrrolidin-2-yl)methyl)amino)methyl)phenyl)-3-methylpyridin-4-yl)phenyl)-2-methoxypyridin-3-yl)methyl)amino)methyl)pyrrolidin-2-one;
(R)-5-((((6-(2-chloro-3-(2-(3-methoxy-4-(((((S)-5-oxopyrrolidin-2-yl)methyl)amino)methyl)phenyl)-3-methylpyridin-4-yl)phenyl)-2-methoxypyridin-3-yl)methyl)amino)methyl)pyrrolidin-2-one;
(R)-5-((((6-(2-chloro-3-(2-(3-methoxy-4-(((((R)-5-oxopyrrolidin-2-yl)methyl)amino)methyl)phenyl)-3-methylpyridin-4-yl)phenyl)-2-methoxypyridin-3-yl)methyl)amino)methyl)pyrrolidin-2-one;
2-(2-methoxy-4-(4-(3-(6-methoxy-5-((7-oxo-2,6-diazaspiro[3.4]octan-2-yl)methyl)pyridin-2-yl)-2-methylphenyl)-3-methylpyridin-2-yl)benzyl)-2,6-diazaspiro[3.4]octan-7-one;
1-(6-((6-(3-(2-(4-((6-acetyl-2,6-diazaspiro[3.3]heptan-2-yl)methyl)-3-methoxyphenyl)-3-methylpyridin-4-yl)-2-methylphenyl)-2-methoxypyridin-3-yl)methyl)-2,6-diazaspiro[3.3]heptan-2-yl)ethan-1-one;
1-(4-(((6-(2-chloro-3-(3-chloro-2-(3-methoxy-4-((piperidin-4-ylamino)methyl)phenyl)pyridin-4-yl)phenyl)-2-methoxypyridin-3-yl)methyl)amino)piperidin-1-yl)ethan-1-one;
4-(((6-(2-chloro-3-(3-chloro-2-(3-methoxy-4-(((1-methyl-2-oxopiperidin-4-yl)amino)methyl)phenyl)pyridin-4-yl)phenyl)-2-methoxypyridin-3-yl)methyl)amino)-1-methylpiperidin-2-one;
4-(((6-(2-chloro-3-(3-chloro-2-(4-(((1-(dimethylcarbamoyl)piperidin-4-yl)amino)methyl)-3-methoxyphenyl)pyridin-4-yl)phenyl)-2-methoxypyridin-3-yl)methyl)amino)-N,N-dimethylpiperidine-1-carboxamide;
N-((6-(2-chloro-3-(3-chloro-2-(4-(((1-(2,2-difluoroethyl)piperidin-4-yl)amino)methyl)-3-methoxyphenyl)pyridin-4-yl)phenyl)-2-methoxypyridin-3-yl)methyl)-1-(2,2-difluoroethyl)piperidin-4-amine;
(4-(((6-(2-chloro-3-(3-chloro-2-(4-(((1-(furan-2-carbonyl)piperidin-4-yl)amino)methyl)-3-methoxyphenyl)pyridin-4-yl)phenyl)-2-methoxypyridin-3-yl)methyl)amino)piperidin-1-yl)(furan-2-yl)methanone;
(4-(((6-(3-(2-(4-(((1-benzoylpiperidin-4-yl)amino)methyl)-3-methoxyphenyl)-3-chloropyridin-4-yl)-2-chlorophenyl)-2-methoxypyridin-3-yl)methyl)amino)piperidin-1-yl)(phenyl)methanone;
N-((6-(2-chloro-3-(3-chloro-2-(3-methoxy-4-(((1-phenylpiperidin-4-yl)amino)methyl)phenyl)pyridin-4-yl)phenyl)-2-methoxypyridin-3-yl)methyl)-1-phenylpiperidin-4-amine;
N-((6-(2-chloro-3-(3-chloro-2-(3-methoxy-4-(((1-(pyridin-4-yl)piperidin-4-yl)amino)methyl)phenyl)pyridin-4-yl)phenyl)-2-methoxypyridin-3-yl)methyl)-1-(pyridin-4-yl)piperidin-4-amine;
N-(2-methoxy-4-(4-(3-(6-methoxy-5-(((tetrahydro-2H-pyran-4-yl)amino)methyl)pyridin-2-yl)-2-methylphenyl)-3-methylpyridin-2-yl)benzyl)tetrahydro-2H-pyran-4-amine;
N-((6-(3-(3-chloro-2-(3-methoxy-4-(((tetrahydro-2H-pyran-4-yl)amino)methyl)phenyl)pyridin-4-yl)-2-methylphenyl)-2-methoxypyridin-3-yl)methyl)tetrahydro-2H-pyran-4-amine;
1-(4-(((6-(2-chloro-3-(3-chloro-2-(3-methoxy-4-((methyl(tetrahydro-2H-pyran-4-yl)amino)methyl)phenyl)pyridin-4-yl)phenyl)-2-methoxypyridin-3-yl)methyl)amino)piperidin-1-yl)ethan-1-one;
1-(6-((6-(3-(6'-((6-acetyl-2,6-diazaspiro[3.3]heptan-2-yl)methyl)-3-chloro-5'-methoxy-[2,3'-bipyridin]-4-yl)-2-chlorophenyl)-2-methoxypyridin-3-yl)methyl)-2,6-diazaspiro[3.3]heptan-2-yl)ethan-1-one;
N-((4-(3-(5-(((2-oxaspiro[3.3]heptan-6-yl)amino)methyl)-6-methoxypyridin-2-yl)-2-chlorophenyl)-3-chloro-5'-methoxy-[2,3'-bipyridin]-6'-yl)methyl)-2-oxaspiro[3.3]heptan-6-amine;
2-((3-chloro-4-(2-chloro-3-(6-methoxy-5-((6-oxo-2,5-diazaspiro[3.4]octan-2-yl)methyl)pyridin-2-yl)phenyl)-5'-methoxy-[2,3'-bipyridin]-6'-yl)methyl)-2,5-diazaspiro[3.4]octan-6-one;
N-((6-(2-chloro-3-(3-chloro-5'-methoxy-6'-(((tetrahydro-2H-pyran-4-yl)amino)methyl)-[2,3'-bipyridin]-4-yl)phenyl)-2-methoxypyridin-3-yl)methyl)tetrahydro-2H-pyran-4-amine;
N-(1-((6-(2-chloro-3-(3-chloro-2-(3-methoxy-4-(((tetrahydro-2H-pyran-4-yl)amino)methyl)phenyl)pyridin-4-yl)phenyl)-2-methoxypyridin-3-yl)methyl)piperidin-4-yl)acetamide;
4-(2-chloro-3-(6-methoxy-5-((7-oxo-2,6-diazaspiro[3.4]octan-2-yl)methyl)pyridin-2-yl)phenyl)-2-(3-methoxy-4-((7-oxo-2,6-diazaspiro[3.4]octan-2-yl)methyl)phenyl)nicotinonitrile;
4-(2-chloro-3-(6-methoxy-5-(((tetrahydro-2H-pyran-4-yl)amino)methyl)pyridin-2-yl)phenyl)-2-(3-methoxy-4-(((tetrahydro-2H-pyran-4-yl)amino)methyl)phenyl)nicotinonitrile;
(S)-1-((6-(2-chloro-3-(3-chloro-2-(4-(((S)-3-hydroxypyrrolidin-1-yl)methyl)-3-methoxyphenyl)pyridin-4-yl)phenyl)-2-methoxypyridin-3-yl)methyl)pyrrolidin-3-ol;
(S)-1-((6-(2-chloro-3-(3-chloro-2-(4-(((R)-3-hydroxypyrrolidin-1-yl)methyl)-3-methoxyphenyl)pyridin-4-yl)phenyl)-2-methoxypyridin-3-yl)methyl)pyrrolidin-3-ol;
(R)-1-((6-(2-chloro-3-(3-chloro-2-(4-(((S)-3-hydroxypyrrolidin-1-yl)methyl)-3-methoxyphenyl)pyridin-4-yl)phenyl)-2-methoxypyridin-3-yl)methyl)pyrrolidin-3-ol;
(R)-1-((6-(2-chloro-3-(3-chloro-2-(4-(((R)-3-hydroxypyrrolidin-1-yl)methyl)-3-methoxyphenyl)pyridin-4-yl)phenyl)-2-methoxypyridin-3-yl)methyl)pyrrolidin-3-ol;
1-(4-((4-(3-chloro-4-(2-chloro-3-(6-methoxy-5-((methyl(tetrahydro-2H-pyran-4-yl)amino)methyl)pyridin-2-yl)phenyl)pyridin-2-yl)-2-methoxybenzyl)amino)piperidin-1-yl)ethan-1-one;
N-((6-(2-chloro-3-(3-chloro-2-(3-methoxy-4-(((1-(2,2,2-trifluoroethyl)piperidin-4-yl)amino)methyl)phenyl)pyridin-4-yl)phenyl)-2-methoxypyridin-3-yl)methyl)-1-(2,2,2-trifluoroethyl)piperidin-4-amine;
2-(4-(3-chloro-4-(2-fluoro-3-(6-methoxy-5-((7-oxo-2,6-diazaspiro[3.4]octan-2-yl)methyl)pyridin-2-yl)phenyl)pyridin-2-yl)-2-methoxybenzyl)-2,6-diazaspiro[3.4]octan-7-one;
1-(4-(((6-(3-(2-(4-(((1-acetylpiperidin-4-yl)amino)methyl)-3-methoxyphenyl)-3-chloropyridin-4-yl)-2-fluorophenyl)-2-methoxypyridin-3-yl)methyl)amino)piperidin-1-yl)ethan-1-one;
N-((6-(3-(3-chloro-2-(3-methoxy-4-(((tetrahydro-2H-pyran-4-yl)amino)methyl)phenyl)pyridin-4-yl)-2-fluorophenyl)-2-methoxypyridin-3-yl)methyl)tetrahydro-2H-pyran-4-amine;
N-(4-(3-chloro-4-(2-chloro-3-(6-methoxy-5-(((tetrahydro-2H-pyran-4-yl)amino)methyl)pyridin-2-yl)phenyl)pyridin-2-yl)-2-methoxybenzyl)-N-methyltetrahydro-2H-pyran-4-amine;
1-(4-((4-(3-chloro-4-(2-chloro-3-(6-methoxy-5-(((tetrahydro-2H-pyran-4-yl)amino)methyl)pyridin-2-yl)phenyl)pyridin-2-yl)-2-methoxybenzyl)(methyl)amino)piperidin-1-yl)ethan-1-one;
1-(4-(((6-(2-chloro-3-(3-chloro-2-(4-(((1-(2-hydroxyacetyl)piperidin-4-yl)amino)methyl)-3-methoxyphenyl)pyridin-4-yl)phenyl)-2-methoxypyridin-3-yl)methyl)amino)piperidin-1-yl)-2-hydroxyethan-1-one;
N-((6-(2-chloro-3-(3-chloro-2-(3-methoxy-4-(((1-(pyridin-2-yl)piperidin-4-yl)amino)methyl)phenyl)pyridin-4-yl)phenyl)-2-methoxypyridin-3-yl)methyl)-1-(pyridin-2-yl)piperidin-4-amine;
N-((6-(2-chloro-3-(3-chloro-2-(4-(((1-(3-chloropyridin-2-yl)piperidin-4-yl)amino)methyl)-3-methoxyphenyl)pyridin-4-yl)phenyl)-2-methoxypyridin-3-yl)methyl)-1-(3-chloropyridin-2-yl)piperidin-4-amine;
N-((6-(2-chloro-3-(3-chloro-2-(3-methoxy-4-(((2-methoxyethyl)amino)methyl)phenyl)pyridin-4-yl)phenyl)-2-methoxypyridin-3-yl)methyl)-2-methoxyethan-1-amine;
3-chloro-4-(2-chloro-3-(6-methoxy-5-((4-methoxypiperidin-1-yl)methyl)pyridin-2-yl)phenyl)-2-(3-methoxy-4-((4-methoxypiperidin-1-yl)methyl)phenyl)pyridine;
(1s,4s)-N-((6-(2-chloro-3-(3-chloro-2-(3-methoxy-4-((((1s,4s)-4-methoxycyclohexyl)amino)methyl)phenyl)pyridin-4-yl)phenyl)-2-methoxypyridin-3-yl)methyl)-4-methoxycyclohexan-1-amine;
(1r,4r)-N-((6-(2-chloro-3-(3-chloro-2-(3-methoxy-4-((((1r,4r)-4-methoxycyclohexyl)amino)methyl)phenyl)pyridin-4-yl)phenyl)-2-methoxypyridin-3-yl)methyl)-4-methoxycyclohexan-1-amine;
(1r,4r)-N-((6-(2-chloro-3-(3-chloro-2-(3-methoxy-4-((((1r,4s)-4-methoxycyclohexyl)amino)methyl)phenyl)pyridin-4-yl)phenyl)-2-methoxypyridin-3-yl)methyl)-4-methoxycyclohexan-1-amine;
(1r,4r)-N-((6-(2-chloro-3-(3-chloro-2-(3-methoxy-4-((((1s,4r)-4-methoxycyclohexyl)amino)methyl)phenyl)pyridin-4-yl)phenyl)-2-methoxypyridin-3-yl)methyl)-4-methoxycyclohexan-1-amine;
(1r,4r)-N-((6-(2-chloro-3-(3-chloro-2-(3-methoxy-4-((((1s,4s)-4-methoxycyclohexyl)amino)methyl)phenyl)pyridin-4-yl)phenyl)-2-methoxypyridin-3-yl)methyl)-4-methoxycyclohexan-1-amine;
(1r,4s)-N-((6-(2-chloro-3-(3-chloro-2-(3-methoxy-4-((((1r,4r)-4-methoxycyclohexyl)amino)methyl)phenyl)pyridin-4-yl)phenyl)-2-methoxypyridin-3-yl)methyl)-4-methoxycyclohexan-1-amine;
(1r,4s)-N-((6-(2-chloro-3-(3-chloro-2-(3-methoxy-4-((((1r,4s)-4-methoxycyclohexyl)amino)methyl)phenyl)pyridin-4-yl)phenyl)-2-methoxypyridin-3-yl)methyl)-4-methoxycyclohexan-1-amine;
(1r,4s)-N-((6-(2-chloro-3-(3-chloro-2-(3-methoxy-4-((((1s,4r)-4-methoxycyclohexyl)amino)methyl)phenyl)pyridin-4-yl)phenyl)-2-methoxypyridin-3-yl)methyl)-4-methoxycyclohexan-1-amine;
(1r,4s)-N-((6-(2-chloro-3-(3-chloro-2-(3-methoxy-4-((((1s,4s)-4-methoxycyclohexyl)amino)methyl)phenyl)pyridin-4-yl)phenyl)-2-methoxypyridin-3-yl)methyl)-4-methoxycyclohexan-1-amine;
(1s,4r)-N-((6-(2-chloro-3-(3-chloro-2-(3-methoxy-4-((((1r,4r)-4-methoxycyclohexyl)amino)methyl)phenyl)pyridin-4-yl)phenyl)-2-methoxypyridin-3-yl)methyl)-4-methoxycyclohexan-1-amine;
(1s,4r)-N-((6-(2-chloro-3-(3-chloro-2-(3-methoxy-4-((((1r,4s)-4-methoxycyclohexyl)amino)methyl)phenyl)pyridin-4-yl)phenyl)-2-methoxypyridin-3-yl)methyl)-4-methoxycyclohexan-1-amine;
(1s,4r)-N-((6-(2-chloro-3-(3-chloro-2-(3-methoxy-4-((((1s,4r)-4-methoxycyclohexyl)amino)methyl)phenyl)pyridin-4-yl)phenyl)-2-methoxypyridin-3-yl)methyl)-4-methoxycyclohexan-1-amine;
(1s,4r)-N-((6-(2-chloro-3-(3-chloro-2-(3-methoxy-4-((((1s,4s)-4-methoxycyclohexyl)amino)methyl)phenyl)pyridin-4-yl)phenyl)-2-methoxypyridin-3-yl)methyl)-4-methoxycyclohexan-1-amine;
(1s,4s)-N-((6-(2-chloro-3-(3-chloro-2-(3-methoxy-4-((((1r,4r)-4-methoxycyclohexyl)amino)methyl)phenyl)pyridin-4-yl)phenyl)-2-methoxypyridin-3-yl)methyl)-4-methoxycyclohexan-1-amine;
(1s,4s)-N-((6-(2-chloro-3-(3-chloro-2-(3-methoxy-4-((((1r,4s)-4-methoxycyclohexyl)amino)methyl)phenyl)pyridin-4-yl)phenyl)-2-methoxypyridin-3-yl)methyl)-4-methoxycyclohexan-1-amine;
(1s,4s)-N-((6-(2-chloro-3-(3-chloro-2-(3-methoxy-4-((((1s,4r)-4-methoxycyclohexyl)amino)methyl)phenyl)pyridin-4-yl)phenyl)-2-methoxypyridin-3-yl)methyl)-4-methoxycyclohexan-1-amine;
1-(4-(((6-(3-(2-(4-(((1-acetylpiperidin-4-yl)amino)methyl)-3-methoxyphenyl)-3-fluoropyridin-4-yl)-2-fluorophenyl)-2-methoxypyridin-3-yl)methyl)amino)piperidin-1-yl)ethan-1-one;
(1r,4r)-N-((6-(2-chloro-3-(3-chloro-2-(3-methoxy-4-((((1r,4r)-4-methoxycyclohexyl)amino)methyl)phenyl)pyridin-4-yl)phenyl)-2-methoxypyridin-3-yl)methyl)-4-methoxycyclohexan-1-amine;
(1r,4r)-N-((6-(2-chloro-3-(3-chloro-2-(3-methoxy-4-((((1r,4s)-4-methoxycyclohexyl)amino)methyl)phenyl)pyridin-4-yl)phenyl)-2-methoxypyridin-3-yl)methyl)-4-methoxycyclohexan-1-amine;
(1r,4r)-N-((6-(2-chloro-3-(3-chloro-2-(3-methoxy-4-((((1s,4r)-4-methoxycyclohexyl)amino)methyl)phenyl)pyridin-4-yl)phenyl)-2-methoxypyridin-3-yl)methyl)-4-methoxycyclohexan-1-amine;
(1r,4r)-N-((6-(2-chloro-3-(3-chloro-2-(3-methoxy-4-((((1s,4s)-4-methoxycyclohexyl)amino)methyl)phenyl)pyridin-4-yl)phenyl)-2-methoxypyridin-3-yl)methyl)-4-methoxycyclohexan-1-amine;
(1r,4s)-N-((6-(2-chloro-3-(3-chloro-2-(3-methoxy-4-((((1r,4r)-4-methoxycyclohexyl)amino)methyl)phenyl)pyridin-4-yl)phenyl)-2-methoxypyridin-3-yl)methyl)-4-methoxycyclohexan-1-amine;
(1r,4s)-N-((6-(2-chloro-3-(3-chloro-2-(3-methoxy-4-((((1r,4s)-4-methoxycyclohexyl)amino)methyl)phenyl)pyridin-4-yl)phenyl)-2-methoxypyridin-3-yl)methyl)-4-methoxycyclohexan-1-amine;
(1r,4s)-N-((6-(2-chloro-3-(3-chloro-2-(3-methoxy-4-((((1s,4r)-4-methoxycyclohexyl)amino)methyl)phenyl)pyridin-4-yl)phenyl)-2-methoxypyridin-3-yl)methyl)-4-methoxycyclohexan-1-amine;
(1r,4s)-N-((6-(2-chloro-3-(3-chloro-2-(3-methoxy-4-((((1s,4s)-4-methoxycyclohexyl)amino)methyl)phenyl)pyridin-4-yl)phenyl)-2-methoxypyridin-3-yl)methyl)-4-methoxycyclohexan-1-amine;
(1s,4r)-N-((6-(2-chloro-3-(3-chloro-2-(3-methoxy-4-((((1r,4r)-4-methoxycyclohexyl)amino)methyl)phenyl)pyridin-4-yl)phenyl)-2-methoxypyridin-3-yl)methyl)-4-methoxycyclohexan-1-amine;
(1s,4r)-N-((6-(2-chloro-3-(3-chloro-2-(3-methoxy-4-((((1r,4s)-4-methoxycyclohexyl)amino)methyl)phenyl)pyridin-4-yl)phenyl)-2-methoxypyridin-3-yl)methyl)-4-methoxycyclohexan-1-amine;
(1s,4r)-N-((6-(2-chloro-3-(3-chloro-2-(3-methoxy-4-((((1s,4r)-4-methoxycyclohexyl)amino)methyl)phenyl)pyridin-4-yl)phenyl)-2-methoxypyridin-3-yl)methyl)-4-methoxycyclohexan-1-amine;
(1s,4r)-N-((6-(2-chloro-3-(3-chloro-2-(3-methoxy-4-((((1s,4s)-4-methoxycyclohexyl)amino)methyl)phenyl)pyridin-4-yl)phenyl)-2-methoxypyridin-3-yl)methyl)-4-methoxycyclohexan-1-amine;
(1s,4s)-N-((6-(2-chloro-3-(3-chloro-2-(3-methoxy-4-((((1r,4r)-4-methoxycyclohexyl)amino)methyl)phenyl)pyridin-4-yl)phenyl)-2-methoxypyridin-3-yl)methyl)-4-methoxycyclohexan-1-amine;
(1s,4s)-N-((6-(2-chloro-3-(3-chloro-2-(3-methoxy-4-((((1r,4s)-4-methoxycyclohexyl)amino)methyl)phenyl)pyridin-4-yl)phenyl)-2-methoxypyridin-3-yl)methyl)-4-methoxycyclohexan-1-amine;
(1s,4s)-N-((6-(2-chloro-3-(3-chloro-2-(3-methoxy-4-((((1s,4r)-4-methoxycyclohexyl)amino)methyl)phenyl)pyridin-4-yl)phenyl)-2-methoxypyridin-3-yl)methyl)-4-methoxycyclohexan-1-amine;
(1s,4s)-N-((6-(2-chloro-3-(3-chloro-2-(3-methoxy-4-((((1s,4s)-4-methoxycyclohexyl)amino)methyl)phenyl)pyridin-4-yl)phenyl)-2-methoxypyridin-3-yl)methyl)-4-methoxycyclohexan-1-amine;
2-(4-(3-fluoro-4-(2-fluoro-3-(6-methoxy-5-((7-oxo-2,6-diazaspiro[3.4]octan-2-yl)methyl)pyridin-2-yl)phenyl)pyridin-2-yl)-2-methoxybenzyl)-2,6-diazaspiro[3.4]octan-7-one;
N-((6-(2-fluoro-3-(3-fluoro-2-(3-methoxy-4-(((tetrahydro-2H-pyran-4-yl)amino)methyl)phenyl)pyridin-4-yl)phenyl)-2-methoxypyridin-3-yl)methyl)tetrahydro-2H-pyran-4-amine;
(1r,4r)-4-(((6-(2-chloro-3-(3-chloro-2-(2-((((1r,4r)-4-hydroxycyclohexyl)amino)methyl)-8-methoxyimidazo[1,2-a]pyridin-6-yl)pyridin-4-yl)phenyl)-2-methoxypyridin-3-yl)methyl)amino)cyclohexan-1-ol;
(1r,4r)-4-(((6-(2-chloro-3-(3-chloro-2-(2-((((1r,4s)-4-hydroxycyclohexyl)amino)methyl)-8-methoxyimidazo[1,2-a]pyridin-6-yl)pyridin-4-yl)phenyl)-2-methoxypyridin-3-yl)methyl)amino)cyclohexan-1-ol;
(1r,4r)-4-(((6-(2-chloro-3-(3-chloro-2-(2-((((1s,4r)-4-hydroxycyclohexyl)amino)methyl)-8-methoxyimidazo[1,2-a]pyridin-6-yl)pyridin-4-yl)phenyl)-2-methoxypyridin-3-yl)methyl)amino)cyclohexan-1-ol;
(1r,4r)-4-(((6-(2-chloro-3-(3-chloro-2-(2-((((1s,4s)-4-hydroxycyclohexyl)amino)methyl)-8-methoxyimidazo[1,2-a]pyridin-6-yl)pyridin-4-yl)phenyl)-2-methoxypyridin-3-yl)methyl)amino)cyclohexan-1-ol;
(1r,4s)-4-(((6-(2-chloro-3-(3-chloro-2-(2-((((1r,4r)-4-hydroxycyclohexyl)amino)methyl)-8-methoxyimidazo[1,2-a]pyridin-6-yl)pyridin-4-yl)phenyl)-2-methoxypyridin-3-yl)methyl)amino)cyclohexan-1-ol;
(1r,4s)-4-(((6-(2-chloro-3-(3-chloro-2-(2-((((1r,4s)-4-hydroxycyclohexyl)amino)methyl)-8-methoxyimidazo[1,2-a]pyridin-6-yl)pyridin-4-yl)phenyl)-2-methoxypyridin-3-yl)methyl)amino)cyclohexan-1-ol;
(1r,4s)-4-(((6-(2-chloro-3-(3-chloro-2-(2-((((1s,4r)-4-hydroxycyclohexyl)amino)methyl)-8-methoxyimidazo[1,2-a]pyridin-6-yl)pyridin-4-yl)phenyl)-2-methoxypyridin-3-yl)methyl)amino)cyclohexan-1-ol;
(1r,4s)-4-(((6-(2-chloro-3-(3-chloro-2-(2-((((1s,4s)-4-hydroxycyclohexyl)amino)methyl)-8-methoxyimidazo[1,2-a]pyridin-6-yl)pyridin-4-yl)phenyl)-2-methoxypyridin-3-yl)methyl)amino)cyclohexan-1-ol;
(1s,4r)-4-(((6-(2-chloro-3-(3-chloro-2-(2-((((1r,4r)-4-hydroxycyclohexyl)amino)methyl)-8-methoxyimidazo[1,2-a]pyridin-6-yl)pyridin-4-yl)phenyl)-2-methoxypyridin-3-yl)methyl)amino)cyclohexan-1-ol;
(1s,4r)-4-(((6-(2-chloro-3-(3-chloro-2-(2-((((1r,4s)-4-hydroxycyclohexyl)amino)methyl)-8-methoxyimidazo[1,2-a]pyridin-6-yl)pyridin-4-yl)phenyl)-2-methoxypyridin-3-yl)methyl)amino)cyclohexan-1-ol;
(1s,4r)-4-(((6-(2-chloro-3-(3-chloro-2-(2-((((1s,4r)-4-hydroxycyclohexyl)amino)methyl)-8-methoxyimidazo[1,2-a]pyridin-6-yl)pyridin-4-yl)phenyl)-2-methoxypyridin-3-yl)methyl)amino)cyclohexan-1-ol;
(1s,4r)-4-(((6-(2-chloro-3-(3-chloro-2-(2-((((1s,4s)-4-hydroxycyclohexyl)amino)methyl)-8-methoxyimidazo[1,2-a]pyridin-6-yl)pyridin-4-yl)phenyl)-2-methoxypyridin-3-yl)methyl)amino)cyclohexan-1-ol;
(1s,4s)-4-(((6-(2-chloro-3-(3-chloro-2-(2-((((1r,4r)-4-hydroxycyclohexyl)amino)methyl)-8-methoxyimidazo[1,2-a]pyridin-6-yl)pyridin-4-yl)phenyl)-2-methoxypyridin-3-yl)methyl)amino)cyclohexan-1-ol;
(1s,4s)-4-(((6-(2-chloro-3-(3-chloro-2-(2-((((1r,4s)-4-hydroxycyclohexyl)amino)methyl)-8-methoxyimidazo[1,2-a]pyridin-6-yl)pyridin-4-yl)phenyl)-2-methoxypyridin-3-yl)methyl)amino)cyclohexan-1-ol;
(1s,4s)-4-(((6-(2-chloro-3-(3-chloro-2-(2-((((1s,4r)-4-hydroxycyclohexyl)amino)methyl)-8-methoxyimidazo[1,2-a]pyridin-6-yl)pyridin-4-yl)phenyl)-2-methoxypyridin-3-yl)methyl)amino)cyclohexan-1-ol;
(1s,4s)-4-(((6-(2-chloro-3-(3-chloro-2-(2-((((1s,4s)-4-hydroxycyclohexyl)amino)methyl)-8-methoxyimidazo[1,2-a]pyridin-6-yl)pyridin-4-yl)phenyl)-2-methoxypyridin-3-yl)methyl)amino)cyclohexan-1-ol;
2-((6-(3-chloro-4-(2-chloro-3-(6-methoxy-5-((6-oxo-2,5-diazaspiro[3.4]octan-2-yl)methyl)pyridin-2-yl)phenyl)pyridin-2-yl)-8-methoxyimidazo[1,2-a]pyridin-2-yl)methyl)-2,5-diazaspiro[3.4]octan-6-one;
1-((6-(2-chloro-3-(3-chloro-2-(2-((3-hydroxy-3-methylazetidin-1-yl)methyl)-8-methoxyimidazo[1,2-a]pyridin-6-yl)pyridin-4-yl)phenyl)-2-methoxypyridin-3-yl)methyl)-3-methylazetidin-3-ol;
(4-(((6-(2-chloro-3-(3-chloro-2-(4-(((1-(cyclohexanecarbonyl)piperidin-4-yl)amino)methyl)-3-methoxyphenyl)pyridin-4-yl)phenyl)-2-methoxypyridin-3-yl)methyl)amino)piperidin-1-yl)(cyclohexyl)methanone;
4-((6-(2-chloro-3-(3-chloro-2-(3-methoxy-4-((3-oxopiperazin-1-yl)methyl)phenyl)pyridin-4-yl)phenyl)-2-methoxypyridin-3-yl)methyl)piperazin-2-one;
1-(4-(((6-(2-chloro-3-(3-chloro-2-(3-methoxy-4-(morpholinomethyl)phenyl)pyridin-4-yl)phenyl)-2-methoxypyridin-3-yl)methyl)amino)piperidin-1-yl)ethan-1-one;
1-(4-(((6-(2-chloro-3-(3-chloro-2-(4-((4-hydroxypiperidin-1-yl)methyl)-3-methoxyphenyl)pyridin-4-yl)phenyl)-2-methoxypyridin-3-yl)methyl)amino)piperidin-1-yl)ethan-1-one;
(S)-(2-(6-(3-chloro-4-(2-chloro-3-(6-methoxy-5-((((5-oxopyrrolidin-2-yl)methyl)amino)methyl)pyridin-2-yl)phenyl)pyridin-2-yl)-2-methoxy-4-oxopyrrolo[2,1-f][1,2,4]triazin-3(4H)-yl)ethyl)glycine;
(R)-(2-(6-(3-chloro-4-(2-chloro-3-(6-methoxy-5-((((5-oxopyrrolidin-2-yl)methyl)amino)methyl)pyridin-2-yl)phenyl)pyridin-2-yl)-2-methoxy-4-oxopyrrolo[2,1-f][1,2,4]triazin-3(4H)-yl)ethyl)glycine;
N-(1-((6-(3-(2-(4-((4-acetamidopiperidin-1-yl)methyl)-3-methoxyphenyl)-3-methylpyridin-4-yl)-2-methylphenyl)-2-methoxypyridin-3-yl)methyl)piperidin-4-yl)acetamide;
N-((6-(2-chloro-3-(2-(3-methoxy-4-(((tetrahydro-2H-pyran-4-yl)amino)methyl)phenyl)-3-methylpyridin-4-yl)phenyl)-2-methoxypyridin-3-yl)methyl)tetrahydro-2H-pyran-4-amine;
(1r,4r)-4-(((6-(2-chloro-3-(2-(4-((((1r,4r)-4-hydroxycyclohexyl)amino)methyl)-3-methoxyphenyl)-3-methylpyridin-4-yl)phenyl)-2-methoxypyridin-3-yl)methyl)amino)cyclohexan-1-ol;
(1r,4r)-4-(((6-(2-chloro-3-(2-(4-((((1r,4s)-4-hydroxycyclohexyl)amino)methyl)-3-methoxyphenyl)-3-methylpyridin-4-yl)phenyl)-2-methoxypyridin-3-yl)methyl)amino)cyclohexan-1-ol;
(1r,4r)-4-(((6-(2-chloro-3-(2-(4-((((1s,4r)-4-hydroxycyclohexyl)amino)methyl)-3-methoxyphenyl)-3-methylpyridin-4-yl)phenyl)-2-methoxypyridin-3-yl)methyl)amino)cyclohexan-1-ol;
(1r,4r)-4-(((6-(2-chloro-3-(2-(4-((((1s,4s)-4-hydroxycyclohexyl)amino)methyl)-3-methoxyphenyl)-3-methylpyridin-4-yl)phenyl)-2-methoxypyridin-3-yl)methyl)amino)cyclohexan-1-ol;
(1r,4s)-4-(((6-(2-chloro-3-(2-(4-((((1r,4r)-4-hydroxycyclohexyl)amino)methyl)-3-methoxyphenyl)-3-methylpyridin-4-yl)phenyl)-2-methoxypyridin-3-yl)methyl)amino)cyclohexan-1-ol;
(1r,4s)-4-(((6-(2-chloro-3-(2-(4-((((1r,4s)-4-hydroxycyclohexyl)amino)methyl)-3-methoxyphenyl)-3-methylpyridin-4-yl)phenyl)-2-methoxypyridin-3-yl)methyl)amino)cyclohexan-1-ol;
(1r,4s)-4-(((6-(2-chloro-3-(2-(4-((((1s,4r)-4-hydroxycyclohexyl)amino)methyl)-3-methoxyphenyl)-3-methylpyridin-4-yl)phenyl)-2-methoxypyridin-3-yl)methyl)amino)cyclohexan-1-ol;
(1r,4s)-4-(((6-(2-chloro-3-(2-(4-((((1s,4s)-4-hydroxycyclohexyl)amino)methyl)-3-methoxyphenyl)-3-methylpyridin-4-yl)phenyl)-2-methoxypyridin-3-yl)methyl)amino)cyclohexan-1-ol;
(1s,4r)-4-(((6-(2-chloro-3-(2-(4-((((1r,4r)-4-hydroxycyclohexyl)amino)methyl)-3-methoxyphenyl)-3-methylpyridin-4-yl)phenyl)-2-methoxypyridin-3-yl)methyl)amino)cyclohexan-1-ol;
(1s,4r)-4-(((6-(2-chloro-3-(2-(4-((((1r,4s)-4-hydroxycyclohexyl)amino)methyl)-3-methoxyphenyl)-3-methylpyridin-4-yl)phenyl)-2-methoxypyridin-3-yl)methyl)amino)cyclohexan-1-ol;
(1s,4r)-4-(((6-(2-chloro-3-(2-(4-((((1s,4r)-4-hydroxycyclohexyl)amino)methyl)-3-methoxyphenyl)-3-methylpyridin-4-yl)phenyl)-2-methoxypyridin-3-yl)methyl)amino)cyclohexan-1-ol;
(1s,4r)-4-(((6-(2-chloro-3-(2-(4-((((1s,4s)-4-hydroxycyclohexyl)amino)methyl)-3-methoxyphenyl)-3-methylpyridin-4-yl)phenyl)-2-methoxypyridin-3-yl)methyl)amino)cyclohexan-1-ol;
(1s,4s)-4-(((6-(2-chloro-3-(2-(4-((((1r,4r)-4-hydroxycyclohexyl)amino)methyl)-3-methoxyphenyl)-3-methylpyridin-4-yl)phenyl)-2-methoxypyridin-3-yl)methyl)amino)cyclohexan-1-ol;
(1s,4s)-4-(((6-(2-chloro-3-(2-(4-((((1r,4s)-4-hydroxycyclohexyl)amino)methyl)-3-methoxyphenyl)-3-methylpyridin-4-yl)phenyl)-2-methoxypyridin-3-yl)methyl)amino)cyclohexan-1-ol;
(1s,4s)-4-(((6-(2-chloro-3-(2-(4-((((1s,4r)-4-hydroxycyclohexyl)amino)methyl)-3-methoxyphenyl)-3-methylpyridin-4-yl)phenyl)-2-methoxypyridin-3-yl)methyl)amino)cyclohexan-1-ol;
(1s,4s)-4-(((6-(2-chloro-3-(2-(4-((((1s,4s)-4-hydroxycyclohexyl)amino)methyl)-3-methoxyphenyl)-3-methylpyridin-4-yl)phenyl)-2-methoxypyridin-3-yl)methyl)amino)cyclohexan-1-ol;
6-(4-(4-(3-(5-((2-oxa-6-azaspiro[3.3]heptan-6-yl)methyl)-6-methoxypyridin-2-yl)-2-chlorophenyl)-3-methylpyridin-2-yl)-2-methoxybenzyl)-2-oxa-6-azaspiro[3.3]heptane;
methyl 4-(((6-(2-chloro-3-(3-chloro-2-(3-methoxy-4-(((1-(methoxycarbonyl)piperidin-4-yl)amino)methyl)phenyl)pyridin-4-yl)phenyl)-2-methoxypyridin-3-yl)methyl)amino)piperidine-1-carboxylate;
N-(1-((6-(3-(2-(4-((4-acetamidopiperidin-1-yl)methyl)-3-methoxyphenyl)-3-methylpyridin-4-yl)-2-chlorophenyl)-2-methoxypyridin-3-yl)methyl)piperidin-4-yl)acetamide;
N-(1-((6-(3-(2-(4-((4-acetamidopiperidin-1-yl)methyl)-3-methoxyphenyl)-3-chloropyridin-4-yl)-2-methylphenyl)-2-methoxypyridin-3-yl)methyl)piperidin-4-yl)acetamide;
N-((6-(2-chloro-3-(3-chloro-2-(3-methoxy-4-(((tetrahydro-2H-pyran-4-yl)amino)methyl)phenyl)pyridin-4-yl)phenyl)-2-methoxypyridin-3-yl)methyl)-N-methyltetrahydro-2H-pyran-4-amine;
1-(4-(((6-(2-chloro-3-(3-chloro-2-(3-methoxy-4-(((tetrahydro-2H-pyran-4-yl)amino)methyl)phenyl)pyridin-4-yl)phenyl)-2-methoxypyridin-3-yl)methyl)(methyl)amino)piperidin-1-yl)ethan-1-one;
2-(4-(4-(3-(5-((7-oxa-2-azaspiro[3.5]nonan-2-yl)methyl)-6-methoxypyridin-2-yl)-2-chlorophenyl)-3-chloropyridin-2-yl)-2-methoxybenzyl)-7-oxa-2-azaspiro[3.5]nonane;
2-(1-((6-(2-chloro-3-(3-chloro-2-(4-((3-(2-hydroxypropan-2-yl)azetidin-1-yl)methyl)-3-methoxyphenyl)pyridin-4-yl)phenyl)-2-methoxypyridin-3-yl)methyl)azetidin-3-yl)propan-2-ol;
(S)-5-((((6-(2-chloro-3-(3-chloro-2-(3-methoxy-4-((methyl(((S)-5-oxopyrrolidin-2-yl)methyl)amino)methyl)phenyl)pyridin-4-yl)phenyl)-2-methoxypyridin-3-yl)methyl)(methyl)amino)methyl)pyrrolidin-2-one;
(S)-5-((((6-(2-chloro-3-(3-chloro-2-(3-methoxy-4-((methyl(((R)-5-oxopyrrolidin-2-yl)methyl)amino)methyl)phenyl)pyridin-4-yl)phenyl)-2-methoxypyridin-3-yl)methyl)(methyl)amino)methyl)pyrrolidin-2-one;
(R)-5-((((6-(2-chloro-3-(3-chloro-2-(3-methoxy-4-((methyl(((S)-5-oxopyrrolidin-2-yl)methyl)amino)methyl)phenyl)pyridin-4-yl)phenyl)-2-methoxypyridin-3-yl)methyl)(methyl)amino)methyl)pyrrolidin-2-one;
(R)-5-((((6-(2-chloro-3-(3-chloro-2-(3-methoxy-4-((methyl(((R)-5-oxopyrrolidin-2-yl)methyl)amino)methyl)phenyl)pyridin-4-yl)phenyl)-2-methoxypyridin-3-yl)methyl)(methyl)amino)methyl)pyrrolidin-2-one;
(R)-4-((((6-(2-chloro-3-(3-chloro-2-(3-methoxy-4-((methyl(((R)-5-oxopyrrolidin-3-yl)methyl)amino)methyl)phenyl)pyridin-4-yl)phenyl)-2-methoxypyridin-3-yl)methyl)(methyl)amino)methyl)pyrrolidin-2-one;
(S)-4-((((6-(2-chloro-3-(3-chloro-2-(3-methoxy-4-((methyl(((R)-5-oxopyrrolidin-3-yl)methyl)amino)methyl)phenyl)pyridin-4-yl)phenyl)-2-methoxypyridin-3-yl)methyl)(methyl)amino)methyl)pyrrolidin-2-one;
(R)-4-((((6-(2-chloro-3-(3-chloro-2-(3-methoxy-4-((methyl(((S)-5-oxopyrrolidin-3-yl)methyl)amino)methyl)phenyl)pyridin-4-yl)phenyl)-2-methoxypyridin-3-yl)methyl)(methyl)amino)methyl)pyrrolidin-2-one;
(S)-4-((((6-(2-chloro-3-(3-chloro-2-(3-methoxy-4-((methyl(((S)-5-oxopyrrolidin-3-yl)methyl)amino)methyl)phenyl)pyridin-4-yl)phenyl)-2-methoxypyridin-3-yl)methyl)(methyl)amino)methyl)pyrrolidin-2-one;
2-((6-(3-chloro-4-(2-chloro-3-(5-((6-hydroxy-2-azaspiro[3.3]heptan-2-yl)methyl)-6-methoxypyridin-2-yl)phenyl)pyridin-2-yl)-8-methoxyimidazo[1,2-a]pyridin-2-yl)methyl)-2-azaspiro[3.3]heptan-6-ol;
1-(4-(((6-(2-chloro-3-(3-chloro-2-(8-methoxy-2-(morpholinomethyl)imidazo[1,2-a]pyridin-6-yl)pyridin-4-yl)phenyl)-2-methoxypyridin-3-yl)methyl)amino)piperidin-1-yl)ethan-1-one;
1-(4-(((6-(2-chloro-3-(3-chloro-2-(2-((3,3-difluoropyrrolidin-1-yl)methyl)-8-methoxyimidazo[1,2-a]pyridin-6-yl)pyridin-4-yl)phenyl)-2-methoxypyridin-3-yl)methyl)amino)piperidin-1-yl)ethan-1-one;
1-((6-(4-(3-(5-(((1-acetylpiperidin-4-yl)amino)methyl)-6-methoxypyridin-2-yl)-2-chlorophenyl)-3-chloropyridin-2-yl)-8-methoxyimidazo[1,2-a]pyridin-2-yl)methyl)-3-methylazetidine-3-carbonitrile;
1-(4-(((6-(2-chloro-3-(3-chloro-2-(2-(((2,2-difluoroethyl)amino)methyl)-8-methoxyimidazo[1,2-a]pyridin-6-yl)pyridin-4-yl)phenyl)-2-methoxypyridin-3-yl)methyl)amino)piperidin-1-yl)ethan-1-one;
4-(2-((4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)amino)ethyl)piperazin-2-one;
2-(3-(6-(4-(3-(5-(((1-acetylpiperidin-4-yl)amino)methyl)-6-methoxypyridin-2-yl)-2-chlorophenyl)-3-chloropyridin-2-yl)-8-methoxy-3,4-dihydroisoquinolin-2(1H)-yl)-2-oxopyrrolidin-1-yl)acetic acid;
1-(4-(((6-(3-(2-(4-(((1-acetylpiperidin-4-yl)amino)methyl)-3-methoxyphenyl)-3-chloropyridin-4-yl)-2-chlorophenyl)-2-methoxypyridin-3-yl)methyl)amino)piperidin-1-yl)-2-methoxyethan-1-one;
1-(4-(((6-(3-(2-(4-((6-acetyl-2,6-diazaspiro[3.3]heptan-2-yl)methyl)-3-methoxyphenyl)-3-chloropyridin-4-yl)-2-chlorophenyl)-2-methoxypyridin-3-yl)methyl)amino)piperidin-1-yl)ethan-1-one;
1-(4-((4-(4-(3-(5-((6-acetyl-2,6-diazaspiro[3.3]heptan-2-yl)methyl)-6-methoxypyridin-2-yl)-2-chlorophenyl)-3-chloropyridin-2-yl)-2-methoxybenzyl)amino)piperidin-1-yl)ethan-1-one;
N-((6-(2-chloro-3-(3-chloro-2-(3-methoxy-4-(((tetrahydro-2H-thiopyran-4-yl)amino)methyl)phenyl)pyridin-4-yl)phenyl)-2-methoxypyridin-3-yl)methyl)tetrahydro-2H-thiopyran-4-amine;
1-(2-((6-(3-(2-(4-((7-acetyl-2,7-diazaspiro[3.5]nonan-2-yl)methyl)-3-methoxyphenyl)-3-chloropyridin-4-yl)-2-chlorophenyl)-2-methoxypyridin-3-yl)methyl)-2,7-diazaspiro[3.5]nonan-7-yl)ethan-1-one;
(S)-5-((((6-(((S)-4-(5-(((1-acetylpiperidin-4-yl)amino)methyl)-3'-chloro-6-methoxy-[2,4'-bipyridin]-2'-yl)-2,3-dihydro-1H-inden-1-yl)amino)-2-methoxy-5-(trifluoromethyl)pyridin-3-yl)methyl)amino)methyl)pyrrolidin-2-one;
(S)-5-((((6-(((R)-4-(5-(((1-acetylpiperidin-4-yl)amino)methyl)-3'-chloro-6-methoxy-[2,4'-bipyridin]-2'-yl)-2,3-dihydro-1H-inden-1-yl)amino)-2-methoxy-5-(trifluoromethyl)pyridin-3-yl)methyl)amino)methyl)pyrrolidin-2-one;
(R)-5-((((6-(((S)-4-(5-(((1-acetylpiperidin-4-yl)amino)methyl)-3'-chloro-6-methoxy-[2,4'-bipyridin]-2'-yl)-2,3-dihydro-1H-inden-1-yl)amino)-2-methoxy-5-(trifluoromethyl)pyridin-3-yl)methyl)amino)methyl)pyrrolidin-2-one;
(R)-5-((((6-(((R)-4-(5-(((1-acetylpiperidin-4-yl)amino)methyl)-3'-chloro-6-methoxy-[2,4'-bipyridin]-2'-yl)-2,3-dihydro-1H-inden-1-yl)amino)-2-methoxy-5-(trifluoromethyl)pyridin-3-yl)methyl)amino)methyl)pyrrolidin-2-one;
1-(4-((4-(3-chloro-4-(2-chloro-3-(5-((4-hydroxypiperidin-1-yl)methyl)-6-methoxypyridin-2-yl)phenyl)pyridin-2-yl)-2-methoxybenzyl)amino)piperidin-1-yl)ethan-1-one;
1-(4-((4-(3-chloro-4-(2-chloro-3-(6-methoxy-5-((((1r,4r)-4-methoxycyclohexyl)amino)methyl)pyridin-2-yl)phenyl)pyridin-2-yl)-2-methoxybenzyl)amino)piperidin-1-yl)ethan-1-one;
1-(4-((4-(3-chloro-4-(2-chloro-3-(6-methoxy-5-((((1s,4r)-4-methoxycyclohexyl)amino)methyl)pyridin-2-yl)phenyl)pyridin-2-yl)-2-methoxybenzyl)amino)piperidin-1-yl)ethan-1-one;
1-(4-((4-(3-chloro-4-(2-chloro-3-(6-methoxy-5-((((1r,4s)-4-methoxycyclohexyl)amino)methyl)pyridin-2-yl)phenyl)pyridin-2-yl)-2-methoxybenzyl)amino)piperidin-1-yl)ethan-1-one;
1-(4-((4-(3-chloro-4-(2-chloro-3-(6-methoxy-5-((((1s,4s)-4-methoxycyclohexyl)amino)methyl)pyridin-2-yl)phenyl)pyridin-2-yl)-2-methoxybenzyl)amino)piperidin-1-yl)ethan-1-one;
(R)-1-(4-(((6-(2-chloro-3-(3-chloro-2-(4-((3-hydroxypiperidin-1-yl)methyl)-3-methoxyphenyl)pyridin-4-yl)phenyl)-2-methoxypyridin-3-yl)methyl)amino)piperidin-1-yl)ethan-1-one;
(S)-1-(4-(((6-(2-chloro-3-(3-chloro-2-(4-((3-hydroxypiperidin-1-yl)methyl)-3-methoxyphenyl)pyridin-4-yl)phenyl)-2-methoxypyridin-3-yl)methyl)amino)piperidin-1-yl)ethan-1-one;
2-(4-(3-chloro-4-(2-chloro-3-(6-methoxy-5-(((1-(2-methoxyacetyl)piperidin-4-yl)amino)methyl)pyridin-2-yl)phenyl)pyridin-2-yl)-2-methoxybenzyl)-2,6-diazaspiro[3.4]octan-7-one;
1-(4-(((6-(3-(2-(4-((6-acetyl-2,6-diazaspiro[3.3]heptan-2-yl)methyl)-3-methoxyphenyl)-3-chloropyridin-4-yl)-2-chlorophenyl)-2-methoxypyridin-3-yl)methyl)amino)piperidin-1-yl)-2-methoxyethan-1-one;
1-(6-(4-(3-chloro-4-(2-chloro-3-(6-methoxy-5-(((tetrahydro-2H-pyran-4-yl)amino)methyl)pyridin-2-yl)phenyl)pyridin-2-yl)-2-methoxybenzyl)-2,6-diazaspiro[3.3]heptan-2-yl)ethan-1-one;
2-((6-(3-(2-(4-((6-acetyl-2,6-diazaspiro[3.3]heptan-2-yl)methyl)-3-methoxyphenyl)-3-chloropyridin-4-yl)-2-chlorophenyl)-2-methoxypyridin-3-yl)methyl)-2,6-diazaspiro[3.4]octan-7-one;
2-((6-(3-(2-(4-((2,6-diazaspiro[3.3]heptan-2-yl)methyl)-3-methoxyphenyl)-3-chloropyridin-4-yl)-2-chlorophenyl)-2-methoxypyridin-3-yl)methyl)-2,6-diazaspiro[3.4]octan-7-one;
2-((6-(2-chloro-3-(3-chloro-2-(3-methoxy-4-(((tetrahydro-2H-pyran-4-yl)amino)methyl)phenyl)pyridin-4-yl)phenyl)-2-methoxypyridin-3-yl)methyl)-2,6-diazaspiro[3.4]octan-7-one;
2-((6-(2-chloro-3-(3-chloro-2-(4-((((1r,3r)-3-hydroxy-3-methylcyclobutyl)amino)methyl)-3-methoxyphenyl)pyridin-4-yl)phenyl)-2-methoxypyridin-3-yl)methyl)-2,6-diazaspiro[3.4]octan-7-one;
2-((6-(2-chloro-3-(3-chloro-2-(4-((((1s,3r)-3-hydroxy-3-methylcyclobutyl)amino)methyl)-3-methoxyphenyl)pyridin-4-yl)phenyl)-2-methoxypyridin-3-yl)methyl)-2,6-diazaspiro[3.4]octan-7-one;
2-((6-(2-chloro-3-(3-chloro-2-(4-((((1r,3s)-3-hydroxy-3-methylcyclobutyl)amino)methyl)-3-methoxyphenyl)pyridin-4-yl)phenyl)-2-methoxypyridin-3-yl)methyl)-2,6-diazaspiro[3.4]octan-7-one;
2-((6-(2-chloro-3-(3-chloro-2-(4-((((1s,3s)-3-hydroxy-3-methylcyclobutyl)amino)methyl)-3-methoxyphenyl)pyridin-4-yl)phenyl)-2-methoxypyridin-3-yl)methyl)-2,6-diazaspiro[3.4]octan-7-one;
2-((6-(2-chloro-3-(3-chloro-2-(4-((6-hydroxy-2-azaspiro[3.3]heptan-2-yl)methyl)-3-methoxyphenyl)pyridin-4-yl)phenyl)-2-methoxypyridin-3-yl)methyl)-2,6-diazaspiro[3.4]octan-7-one;
2-((6-(2-chloro-3-(3-chloro-2-(4-((3-hydroxy-3-methylazetidin-1-yl)methyl)-3-methoxyphenyl)pyridin-4-yl)phenyl)-2-methoxypyridin-3-yl)methyl)-2,6-diazaspiro[3.4]octan-7-one;
1-(3-((((6-(3-(2-(4-((((1-acetylazetidin-3-yl)methyl)(methyl)amino)methyl)-3-methoxyphenyl)-3-chloropyridin-4-yl)-2-chlorophenyl)-2-methoxypyridin-3-yl)methyl)(methyl)amino)methyl)azetidin-1-yl)ethan-1-one;
1-(4-(((4-(4'-(((1-acetylpiperidin-4-yl)amino)methyl)-2-chloro-3'-methoxy-[1,1'-biphenyl]-3-yl)-3-chloro-5'-methoxy-[2,3'-bipyridin]-6'-yl)methyl)amino)piperidin-1-yl)ethan-1-one;
2-((2'-chloro-3'-(3-chloro-5'-methoxy-6'-((7-oxo-2,6-diazaspiro[3.4]octan-2-yl)methyl)-[2,3'-bipyridin]-4-yl)-3-methoxy-[1,1'-biphenyl]-4-yl)methyl)-2,6-diazaspiro[3.4]octan-7-one;
2-((6-(2-chloro-3-(3-chloro-2-(4-((((1r,4r)-4-hydroxycyclohexyl)amino)methyl)-3-methoxyphenyl)pyridin-4-yl)phenyl)-2-methoxypyridin-3-yl)methyl)-2,6-diazaspiro[3.4]octan-7-one;
2-((6-(2-chloro-3-(3-chloro-2-(4-((((1s,4r)-4-hydroxycyclohexyl)amino)methyl)-3-methoxyphenyl)pyridin-4-yl)phenyl)-2-methoxypyridin-3-yl)methyl)-2,6-diazaspiro[3.4]octan-7-one;
2-((6-(2-chloro-3-(3-chloro-2-(4-((((1r,4s)-4-hydroxycyclohexyl)amino)methyl)-3-methoxyphenyl)pyridin-4-yl)phenyl)-2-methoxypyridin-3-yl)methyl)-2,6-diazaspiro[3.4]octan-7-one;
2-((6-(2-chloro-3-(3-chloro-2-(4-((((1s,4s)-4-hydroxycyclohexyl)amino)methyl)-3-methoxyphenyl)pyridin-4-yl)phenyl)-2-methoxypyridin-3-yl)methyl)-2,6-diazaspiro[3.4]octan-7-one;
1-((((6-(2-chloro-3-(3-chloro-2-(4-((((1-hydroxycyclopropyl)methyl)amino)methyl)-3-methoxyphenyl)pyridin-4-yl)phenyl)-2-methoxypyridin-3-yl)methyl)amino)methyl)cyclopropan-1-ol;
N-((6-(2-chloro-3-(3-chloro-2-(4-(((2,2-difluoroethyl)amino)methyl)-3-methoxyphenyl)pyridin-4-yl)phenyl)-2-methoxypyridin-3-yl)methyl)-2,2-difluoroethan-1-amine;
2-(4-(3-chloro-4-(2-chloro-3-(6-methoxy-5-(((tetrahydro-2H-pyran-4-yl)amino)methyl)pyridin-2-yl)phenyl)pyridin-2-yl)-2-methoxybenzyl)-2,6-diazaspiro[3.4]octan-7-one;
(S)-1-(4-(((6-(2-chloro-3-(3-chloro-2-(4-((3-hydroxypiperidin-1-yl)methyl)-3-methoxyphenyl)pyridin-4-yl)phenyl)-2-methoxypyridin-3-yl)methyl)amino)piperidin-1-yl)ethan-1-one;
(R)-1-(4-(((6-(2-chloro-3-(3-chloro-2-(4-((3-hydroxypiperidin-1-yl)methyl)-3-methoxyphenyl)pyridin-4-yl)phenyl)-2-methoxypyridin-3-yl)methyl)amino)piperidin-1-yl)ethan-1-one;
1-(4-(((6-(2-chloro-3-(3-chloro-2-(4-((4-(hydroxymethyl)piperidin-1-yl)methyl)-3-methoxyphenyl)pyridin-4-yl)phenyl)-2-methoxypyridin-3-yl)methyl)amino)piperidin-1-yl)ethan-1-one;
1-(4-(((6-(2-chloro-3-(3-chloro-2-(3-methoxy-4-((4-(methoxymethyl)piperidin-1-yl)methyl)phenyl)pyridin-4-yl)phenyl)-2-methoxypyridin-3-yl)methyl)amino)piperidin-1-yl)ethan-1-one;
1-(4-(((6-(2-chloro-3-(3-chloro-2-(4-((4-hydroxypiperidin-1-yl)methyl)-3-methoxyphenyl)pyridin-4-yl)phenyl)-2-methoxypyridin-3-yl)methyl)amino)piperidin-1-yl)-2-methoxyethan-1-one;
1-(6-((6-(2-chloro-3-(3-chloro-2-(3-methoxy-4-(((tetrahydro-2H-pyran-4-yl)amino)methyl)phenyl)pyridin-4-yl)phenyl)-2-methoxypyridin-3-yl)methyl)-2,6-diazaspiro[3.3]heptan-2-yl)ethan-1-one;
1-(4-(((6-(2-chloro-3-(3-chloro-2-(4-((((1-hydroxycyclopropyl)methyl)amino)methyl)-3-methoxyphenyl)pyridin-4-yl)phenyl)-2-methoxypyridin-3-yl)methyl)amino)piperidin-1-yl)ethan-1-one;
1-(4-(((6-(2-chloro-3-(3-chloro-2-(4-(((2,2-difluoroethyl)amino)methyl)-3-methoxyphenyl)pyridin-4-yl)phenyl)-2-methoxypyridin-3-yl)methyl)amino)piperidin-1-yl)ethan-1-one;
N-((6-(2-chloro-3-(3-chloro-2-(4-((isopropylamino)methyl)-3-methoxyphenyl)pyridin-4-yl)phenyl)-2-methoxypyridin-3-yl)methyl)propan-2-amine;
2-(4-(3-chloro-4-(2-chloro-3-(5-((((1r,4r)-4-hydroxycyclohexyl)amino)methyl)-6-methoxypyridin-2-yl)phenyl)pyridin-2-yl)-2-methoxybenzyl)-2,6-diazaspiro[3.4]octan-7-one;
2-(4-(3-chloro-4-(2-chloro-3-(5-((((1s,4r)-4-hydroxycyclohexyl)amino)methyl)-6-methoxypyridin-2-yl)phenyl)pyridin-2-yl)-2-methoxybenzyl)-2,6-diazaspiro[3.4]octan-7-one;
2-(4-(3-chloro-4-(2-chloro-3-(5-((((1r,4s)-4-hydroxycyclohexyl)amino)methyl)-6-methoxypyridin-2-yl)phenyl)pyridin-2-yl)-2-methoxybenzyl)-2,6-diazaspiro[3.4]octan-7-one;
2-(4-(3-chloro-4-(2-chloro-3-(5-((((1s,4s)-4-hydroxycyclohexyl)amino)methyl)-6-methoxypyridin-2-yl)phenyl)pyridin-2-yl)-2-methoxybenzyl)-2,6-diazaspiro[3.4]octan-7-one;
2-(4-(3-chloro-4-(2-chloro-3-(5-((((1s,4s)-4-hydroxycyclohexyl)amino)methyl)-6-methoxypyridin-2-yl)phenyl)pyridin-2-yl)-2-methoxybenzyl)-2,6-diazaspiro[3.4]octan-7-one;
2-(4-(3-chloro-4-(2-chloro-3-(5-((((1s,4r)-4-hydroxycyclohexyl)amino)methyl)-6-methoxypyridin-2-yl)phenyl)pyridin-2-yl)-2-methoxybenzyl)-2,6-diazaspiro[3.4]octan-7-one;
2-(4-(3-chloro-4-(2-chloro-3-(5-((((1r,4s)-4-hydroxycyclohexyl)amino)methyl)-6-methoxypyridin-2-yl)phenyl)pyridin-2-yl)-2-methoxybenzyl)-2,6-diazaspiro[3.4]octan-7-one;
2-(4-(3-chloro-4-(2-chloro-3-(5-((((1r,4r)-4-hydroxycyclohexyl)amino)methyl)-6-methoxypyridin-2-yl)phenyl)pyridin-2-yl)-2-methoxybenzyl)-2,6-diazaspiro[3.4]octan-7-one;
2-(4-(3-chloro-4-(2-chloro-3-(5-((((1r,3r)-3-hydroxy-3-methylcyclobutyl)amino)methyl)-6-methoxypyridin-2-yl)phenyl)pyridin-2-yl)-2-methoxybenzyl)-2,6-diazaspiro[3.4]octan-7-one;
2-(4-(3-chloro-4-(2-chloro-3-(5-((((1s,3r)-3-hydroxy-3-methylcyclobutyl)amino)methyl)-6-methoxypyridin-2-yl)phenyl)pyridin-2-yl)-2-methoxybenzyl)-2,6-diazaspiro[3.4]octan-7-one;
2-(4-(3-chloro-4-(2-chloro-3-(5-((((1r,3s)-3-hydroxy-3-methylcyclobutyl)amino)methyl)-6-methoxypyridin-2-yl)phenyl)pyridin-2-yl)-2-methoxybenzyl)-2,6-diazaspiro[3.4]octan-7-one;
2-(4-(3-chloro-4-(2-chloro-3-(5-((((1s,3s)-3-hydroxy-3-methylcyclobutyl)amino)methyl)-6-methoxypyridin-2-yl)phenyl)pyridin-2-yl)-2-methoxybenzyl)-2,6-diazaspiro[3.4]octan-7-one;
2-(4-(3-chloro-4-(2-chloro-3-(5-((3-hydroxy-3-methylazetidin-1-yl)methyl)-6-methoxypyridin-2-yl)phenyl)pyridin-2-yl)-2-methoxybenzyl)-2,6-diazaspiro[3.4]octan-7-one;
2-(4-(4-(3-(5-((6-acetyl-2,6-diazaspiro[3.3]heptan-2-yl)methyl)-6-methoxypyridin-2-yl)-2-chlorophenyl)-3-chloropyridin-2-yl)-2-methoxybenzyl)-2,6-diazaspiro[3.4]octan-7-one;
2-(4-(4-(3-(5-((2,6-diazaspiro[3.3]heptan-2-yl)methyl)-6-methoxypyridin-2-yl)-2-chlorophenyl)-3-chloropyridin-2-yl)-2-methoxybenzyl)-2,6-diazaspiro[3.4]octan-7-one;
2-(4-(3-chloro-4-(2-chloro-3-(5-((6-hydroxy-2-azaspiro[3.3]heptan-2-yl)methyl)-6-methoxypyridin-2-yl)phenyl)pyridin-2-yl)-2-methoxybenzyl)-2,6-diazaspiro[3.4]octan-7-one;
1-(4-(((6-(3-(6-(4-(((1-acetylpiperidin-4-yl)amino)methyl)-3-methoxyphenyl)-5-methylpyrimidin-4-yl)-2-chlorophenyl)-2-methoxypyridin-3-yl)methyl)amino)piperidin-1-yl)ethan-1-one;
(S)-5-((((6-(2-chloro-3-(6-(3-methoxy-4-(((((S)-5-oxopyrrolidin-2-yl)methyl)amino)methyl)phenyl)-5-methylpyrimidin-4-yl)phenyl)-2-methoxypyridin-3-yl)methyl)amino)methyl)pyrrolidin-2-one;
(S)-5-((((6-(2-chloro-3-(6-(3-methoxy-4-(((((R)-5-oxopyrrolidin-2-yl)methyl)amino)methyl)phenyl)-5-methylpyrimidin-4-yl)phenyl)-2-methoxypyridin-3-yl)methyl)amino)methyl)pyrrolidin-2-one;
(R)-5-((((6-(2-chloro-3-(6-(3-methoxy-4-(((((S)-5-oxopyrrolidin-2-yl)methyl)amino)methyl)phenyl)-5-methylpyrimidin-4-yl)phenyl)-2-methoxypyridin-3-yl)methyl)amino)methyl)pyrrolidin-2-one;
(R)-5-((((6-(2-chloro-3-(6-(3-methoxy-4-(((((R)-5-oxopyrrolidin-2-yl)methyl)amino)methyl)phenyl)-5-methylpyrimidin-4-yl)phenyl)-2-methoxypyridin-3-yl)methyl)amino)methyl)pyrrolidin-2-one;
N-((6-(2-chloro-3-(3-chloro-2-(4-((cyclohexylamino)methyl)-3-methoxyphenyl)pyridin-4-yl)phenyl)-2-methoxypyridin-3-yl)methyl)cyclohexanamine;
2-((6-(3-(2-(4-(((1-acetylpiperidin-4-yl)amino)methyl)-3-methoxyphenyl)-3-chloropyridin-4-yl)-2-chlorophenyl)-2-methoxypyridin-3-yl)methyl)-2,6-diazaspiro[3.4]octan-5-one;
2-((6-(3-(2-(4-(((1-acetylpiperidin-4-yl)amino)methyl)-3-methoxyphenyl)-3-chloropyridin-4-yl)-2-chlorophenyl)-2-methoxypyridin-3-yl)methyl)-2,5,7-triazaspiro[3.4]octan-6-one;
(S)-5-((((2'-((S)-1-((5-(((1-acetylpiperidin-4-yl)amino)methyl)-6-methoxy-3-(trifluoromethyl)pyridin-2-yl)oxy)-2,3-dihydro-1H-inden-4-yl)-3'-chloro-6-methoxy-[2,4'-bipyridin]-5-yl)methyl)amino)methyl)pyrrolidin-2-one;
(S)-5-((((2'-((R)-1-((5-(((1-acetylpiperidin-4-yl)amino)methyl)-6-methoxy-3-(trifluoromethyl)pyridin-2-yl)oxy)-2,3-dihydro-1H-inden-4-yl)-3'-chloro-6-methoxy-[2,4'-bipyridin]-5-yl)methyl)amino)methyl)pyrrolidin-2-one;
(R)-5-((((2'-((S)-1-((5-(((1-acetylpiperidin-4-yl)amino)methyl)-6-methoxy-3-(trifluoromethyl)pyridin-2-yl)oxy)-2,3-dihydro-1H-inden-4-yl)-3'-chloro-6-methoxy-[2,4'-bipyridin]-5-yl)methyl)amino)methyl)pyrrolidin-2-one;
(R)-5-((((2'-((R)-1-((5-(((1-acetylpiperidin-4-yl)amino)methyl)-6-methoxy-3-(trifluoromethyl)pyridin-2-yl)oxy)-2,3-dihydro-1H-inden-4-yl)-3'-chloro-6-methoxy-[2,4'-bipyridin]-5-yl)methyl)amino)methyl)pyrrolidin-2-one;
1-(4-(((6-(3-(2-(4-(((1-acetylpiperidin-4-yl)amino)methyl)-3-methoxyphenyl)-3-chloropyridin-4-yl)-2-(trifluoromethyl)phenyl)-2-methoxypyridin-3-yl)methyl)amino)piperidin-1-yl)ethan-1-one;
N-((6-(3-(3-chloro-2-(3-methoxy-4-(((tetrahydro-2H-pyran-4-yl)amino)methyl)phenyl)pyridin-4-yl)-2-(trifluoromethyl)phenyl)-2-methoxypyridin-3-yl)methyl)tetrahydro-2H-pyran-4-amine;
N-((6-(3-(2-(4-((7-oxa-2-azaspiro[3.5]nonan-2-yl)methyl)-3-methoxyphenyl)-3-chloropyridin-4-yl)-2-chlorophenyl)-2-methoxypyridin-3-yl)methyl)tetrahydro-2H-pyran-4-amine;N-(4-(4-(3-(5-((7-oxa-2-azaspiro[3.5]nonan-2-yl)methyl)-6-methoxypyridin-2-yl)-2-chlorophenyl)-3-chloropyridin-2-yl)-2-methoxybenzyl)tetrahydro-2H-pyran-4-amine;
(S)-5-((((6-(3-chloro-2-((S)-1-((6-methoxy-5-(((((S)-5-oxopyrrolidin-2-yl)methyl)amino)methyl)-3-(trifluoromethyl)pyrazin-2-yl)amino)-2,3-dihydro-1H-inden-4-yl)pyridin-4-yl)-1-methyl-1H-pyrrolo[2,3-b]pyridin-3-yl)methyl)amino)methyl)pyrrolidin-2-one;
(R)-5-((((6-(3-chloro-2-((R)-1-((6-methoxy-5-(((((R)-5-oxopyrrolidin-2-yl)methyl)amino)methyl)-3-(trifluoromethyl)pyrazin-2-yl)amino)-2,3-dihydro-1H-inden-4-yl)pyridin-4-yl)-1-methyl-1H-pyrrolo[2,3-b]pyridin-3-yl)methyl)amino)methyl)pyrrolidin-2-one;
(R)-5-((((6-(3-chloro-2-((R)-1-((6-methoxy-5-(((((S)-5-oxopyrrolidin-2-yl)methyl)amino)methyl)-3-(trifluoromethyl)pyrazin-2-yl)amino)-2,3-dihydro-1H-inden-4-yl)pyridin-4-yl)-1-methyl-1H-pyrrolo[2,3-b]pyridin-3-yl)methyl)amino)methyl)pyrrolidin-2-one;
(R)-5-((((6-(3-chloro-2-((S)-1-((6-methoxy-5-(((((R)-5-oxopyrrolidin-2-yl)methyl)amino)methyl)-3-(trifluoromethyl)pyrazin-2-yl)amino)-2,3-dihydro-1H-inden-4-yl)pyridin-4-yl)-1-methyl-1H-pyrrolo[2,3-b]pyridin-3-yl)methyl)amino)methyl)pyrrolidin-2-one;
(R)-5-((((6-(3-chloro-2-((S)-1-((6-methoxy-5-(((((S)-5-oxopyrrolidin-2-yl)methyl)amino)methyl)-3-(trifluoromethyl)pyrazin-2-yl)amino)-2,3-dihydro-1H-inden-4-yl)pyridin-4-yl)-1-methyl-1H-pyrrolo[2,3-b]pyridin-3-yl)methyl)amino)methyl)pyrrolidin-2-one;
(S)-5-((((6-(3-chloro-2-((R)-1-((6-methoxy-5-(((((R)-5-oxopyrrolidin-2-yl)methyl)amino)methyl)-3-(trifluoromethyl)pyrazin-2-yl)amino)-2,3-dihydro-1H-inden-4-yl)pyridin-4-yl)-1-methyl-1H-pyrrolo[2,3-b]pyridin-3-yl)methyl)amino)methyl)pyrrolidin-2-one;
(S)-5-((((6-(3-chloro-2-((R)-1-((6-methoxy-5-(((((S)-5-oxopyrrolidin-2-yl)methyl)amino)methyl)-3-(trifluoromethyl)pyrazin-2-yl)amino)-2,3-dihydro-1H-inden-4-yl)pyridin-4-yl)-1-methyl-1H-pyrrolo[2,3-b]pyridin-3-yl)methyl)amino)methyl)pyrrolidin-2-one;
(S)-5-((((6-(3-chloro-2-((S)-1-((6-methoxy-5-(((((R)-5-oxopyrrolidin-2-yl)methyl)amino)methyl)-3-(trifluoromethyl)pyrazin-2-yl)amino)-2,3-dihydro-1H-inden-4-yl)pyridin-4-yl)-1-methyl-1H-pyrrolo[2,3-b]pyridin-3-yl)methyl)amino)methyl)pyrrolidin-2-one;
1-(4-(((6-(3-(2-(4-(((1-acetylpiperidin-4-yl)amino)methyl)-3-methoxyphenyl)-3-chloropyridin-4-yl)-2-chlorophenyl)-2-methoxypyridin-3-yl)methyl)amino)piperidin-1-yl)-2-hydroxyethan-1-one;
N-((6-(2-chloro-3-(3-chloro-2-(4-(((3-fluoropropyl)amino)methyl)-3-methoxyphenyl)pyridin-4-yl)phenyl)-2-methoxypyridin-3-yl)methyl)-3-fluoropropan-1-amine;
2-(4-(6-(2-chloro-3-(6-methoxy-5-((7-oxo-2,6-diazaspiro[3.4]octan-2-yl)methyl)pyridin-2-yl)phenyl)-5-methylpyrimidin-4-yl)-2-methoxybenzyl)-2,6-diazaspiro[3.4]octan-7-one;
1-(1-((6-(3-(2-(4-((4-acetylpiperidin-1-yl)methyl)-3-methoxyphenyl)-3-chloropyridin-4-yl)-2-chlorophenyl)-2-methoxypyridin-3-yl)methyl)piperidin-4-yl)ethan-1-one;
1-(4-(((6-(2-chloro-3-(3-chloro-2-(4-(((3-fluoropropyl)amino)methyl)-3-methoxyphenyl)pyridin-4-yl)phenyl)-2-methoxypyridin-3-yl)methyl)amino)piperidin-1-yl)ethan-1-one;
1-(6-((6-(3-(6-(4-((6-acetyl-2,6-diazaspiro[3.3]heptan-2-yl)methyl)-3-methoxyphenyl)-5-methylpyrimidin-4-yl)-2-chlorophenyl)-2-methoxypyridin-3-yl)methyl)-2,6-diazaspiro[3.3]heptan-2-yl)ethan-1-one;
1-(4-(((6-(2-chloro-3-(3-chloro-2-(4-((((1r,4r)-4-hydroxycyclohexyl)amino)methyl)-3-methoxyphenyl)pyridin-4-yl)phenyl)-2-methoxypyridin-3-yl)methyl)amino)piperidin-1-yl)-2-hydroxyethan-1-one;
1-(4-(((6-(2-chloro-3-(3-chloro-2-(4-((((1s,4r)-4-hydroxycyclohexyl)amino)methyl)-3-methoxyphenyl)pyridin-4-yl)phenyl)-2-methoxypyridin-3-yl)methyl)amino)piperidin-1-yl)-2-hydroxyethan-1-one;
1-(4-(((6-(2-chloro-3-(3-chloro-2-(4-((((1r,4s)-4-hydroxycyclohexyl)amino)methyl)-3-methoxyphenyl)pyridin-4-yl)phenyl)-2-methoxypyridin-3-yl)methyl)amino)piperidin-1-yl)-2-hydroxyethan-1-one;
1-(4-(((6-(2-chloro-3-(3-chloro-2-(4-((((1s,4s)-4-hydroxycyclohexyl)amino)methyl)-3-methoxyphenyl)pyridin-4-yl)phenyl)-2-methoxypyridin-3-yl)methyl)amino)piperidin-1-yl)-2-hydroxyethan-1-one;
1-(4-(((6-(3-(6-(4-(((1-acetylpiperidin-4-yl)amino)methyl)-3-methoxyphenyl)-5-chloropyrimidin-4-yl)-2-chlorophenyl)-2-methoxypyridin-3-yl)methyl)amino)piperidin-1-yl)ethan-1-one;
(R)-5-(((6-(3-(2-(4-(((1-acetylpiperidin-4-yl)amino)methyl)-3-methoxyphenyl)-3-chloropyridin-4-yl)-2-chlorophenyl)-2-methoxypyridin-3-yl)methyl)amino)piperidin-2-one;
(S)-5-(((6-(3-(2-(4-(((1-acetylpiperidin-4-yl)amino)methyl)-3-methoxyphenyl)-3-chloropyridin-4-yl)-2-chlorophenyl)-2-methoxypyridin-3-yl)methyl)amino)piperidin-2-one;
(S)-5-((((6-(2-chloro-3-(5-chloro-6-(3-methoxy-4-(((((S)-5-oxopyrrolidin-2-yl)methyl)amino)methyl)phenyl)pyrimidin-4-yl)phenyl)-2-methoxypyridin-3-yl)methyl)amino)methyl)pyrrolidin-2-one;
(S)-5-((((6-(2-chloro-3-(5-chloro-6-(3-methoxy-4-(((((R)-5-oxopyrrolidin-2-yl)methyl)amino)methyl)phenyl)pyrimidin-4-yl)phenyl)-2-methoxypyridin-3-yl)methyl)amino)methyl)pyrrolidin-2-one;
(R)-5-((((6-(2-chloro-3-(5-chloro-6-(3-methoxy-4-(((((S)-5-oxopyrrolidin-2-yl)methyl)amino)methyl)phenyl)pyrimidin-4-yl)phenyl)-2-methoxypyridin-3-yl)methyl)amino)methyl)pyrrolidin-2-one;
(R)-5-((((6-(2-chloro-3-(5-chloro-6-(3-methoxy-4-(((((R)-5-oxopyrrolidin-2-yl)methyl)amino)methyl)phenyl)pyrimidin-4-yl)phenyl)-2-methoxypyridin-3-yl)methyl)amino)methyl)pyrrolidin-2-one;
(S)-5-((((6-(((S)-4-(5-(((1-acetylpiperidin-4-yl)amino)methyl)-3'-chloro-6-methoxy-[2,4'-bipyridin]-2'-yl)-2,3-dihydro-1H-inden-1-yl)oxy)-2-methoxy-5-(trifluoromethyl)pyridin-3-yl)methyl)amino)methyl)pyrrolidin-2-one;
(S)-5-((((6-(((R)-4-(5-(((1-acetylpiperidin-4-yl)amino)methyl)-3'-chloro-6-methoxy-[2,4'-bipyridin]-2'-yl)-2,3-dihydro-1H-inden-1-yl)oxy)-2-methoxy-5-(trifluoromethyl)pyridin-3-yl)methyl)amino)methyl)pyrrolidin-2-one;
(R)-5-((((6-(((S)-4-(5-(((1-acetylpiperidin-4-yl)amino)methyl)-3'-chloro-6-methoxy-[2,4'-bipyridin]-2'-yl)-2,3-dihydro-1H-inden-1-yl)oxy)-2-methoxy-5-(trifluoromethyl)pyridin-3-yl)methyl)amino)methyl)pyrrolidin-2-one;
(R)-5-((((6-(((R)-4-(5-(((1-acetylpiperidin-4-yl)amino)methyl)-3'-chloro-6-methoxy-[2,4'-bipyridin]-2'-yl)-2,3-dihydro-1H-inden-1-yl)oxy)-2-methoxy-5-(trifluoromethyl)pyridin-3-yl)methyl)amino)methyl)pyrrolidin-2-one;
(S)-5-((((6-(3-chloro-2-((S)-1-((6-methoxy-5-(((((S)-5-oxopyrrolidin-2-yl)methyl)amino)methyl)-3-(trifluoromethyl)pyridin-2-yl)oxy)-2,3-dihydro-1H-inden-4-yl)pyridin-4-yl)-1-methyl-1H-pyrrolo[2,3-b]pyridin-3-yl)methyl)amino)methyl)pyrrolidin-2-one;
(R)-5-((((6-(3-chloro-2-((R)-1-((6-methoxy-5-(((((R)-5-oxopyrrolidin-2-yl)methyl)amino)methyl)-3-(trifluoromethyl)pyridin-2-yl)oxy)-2,3-dihydro-1H-inden-4-yl)pyridin-4-yl)-1-methyl-1H-pyrrolo[2,3-b]pyridin-3-yl)methyl)amino)methyl)pyrrolidin-2-one;
(R)-5-((((6-(3-chloro-2-((R)-1-((6-methoxy-5-(((((S)-5-oxopyrrolidin-2-yl)methyl)amino)methyl)-3-(trifluoromethyl)pyridin-2-yl)oxy)-2,3-dihydro-1H-inden-4-yl)pyridin-4-yl)-1-methyl-1H-pyrrolo[2,3-b]pyridin-3-yl)methyl)amino)methyl)pyrrolidin-2-one;
(R)-5-((((6-(3-chloro-2-((S)-1-((6-methoxy-5-(((((R)-5-oxopyrrolidin-2-yl)methyl)amino)methyl)-3-(trifluoromethyl)pyridin-2-yl)oxy)-2,3-dihydro-1H-inden-4-yl)pyridin-4-yl)-1-methyl-1H-pyrrolo[2,3-b]pyridin-3-yl)methyl)amino)methyl)pyrrolidin-2-one;
(R)-5-((((6-(3-chloro-2-((S)-1-((6-methoxy-5-(((((S)-5-oxopyrrolidin-2-yl)methyl)amino)methyl)-3-(trifluoromethyl)pyridin-2-yl)oxy)-2,3-dihydro-1H-inden-4-yl)pyridin-4-yl)-1-methyl-1H-pyrrolo[2,3-b]pyridin-3-yl)methyl)amino)methyl)pyrrolidin-2-one;
(S)-5-((((6-(3-chloro-2-((R)-1-((6-methoxy-5-(((((R)-5-oxopyrrolidin-2-yl)methyl)amino)methyl)-3-(trifluoromethyl)pyridin-2-yl)oxy)-2,3-dihydro-1H-inden-4-yl)pyridin-4-yl)-1-methyl-1H-pyrrolo[2,3-b]pyridin-3-yl)methyl)amino)methyl)pyrrolidin-2-one;
(S)-5-((((6-(3-chloro-2-((R)-1-((6-methoxy-5-(((((S)-5-oxopyrrolidin-2-yl)methyl)amino)methyl)-3-(trifluoromethyl)pyridin-2-yl)oxy)-2,3-dihydro-1H-inden-4-yl)pyridin-4-yl)-1-methyl-1H-pyrrolo[2,3-b]pyridin-3-yl)methyl)amino)methyl)pyrrolidin-2-one;
(S)-5-((((6-(3-chloro-2-((S)-1-((6-methoxy-5-(((((R)-5-oxopyrrolidin-2-yl)methyl)amino)methyl)-3-(trifluoromethyl)pyridin-2-yl)oxy)-2,3-dihydro-1H-inden-4-yl)pyridin-4-yl)-1-methyl-1H-pyrrolo[2,3-b]pyridin-3-yl)methyl)amino)methyl)pyrrolidin-2-one;
1-(4-(((6-(2-chloro-3-(3-chloro-2-(3-methoxy-4-(((1-(3-methoxypropanoyl)piperidin-4-yl)amino)methyl)phenyl)pyridin-4-yl)phenyl)-2-methoxypyridin-3-yl)methyl)amino)piperidin-1-yl)-3-methoxypropan-1-one;
2-((6-(3-(6'-(((1-acetylpiperidin-4-yl)amino)methyl)-3-chloro-5'-methoxy-[2,3'-bipyridin]-4-yl)-2-chlorophenyl)-2-methoxypyridin-3-yl)methyl)-2,6-diazaspiro[3.4]octan-7-one;
2-((6-(2-chloro-3-(3-chloro-5'-methoxy-6'-(((tetrahydro-2H-pyran-4-yl)amino)methyl)-[2,3'-bipyridin]-4-yl)phenyl)-2-methoxypyridin-3-yl)methyl)-2,6-diazaspiro[3.4]octan-7-one;
2-((6-(2-chloro-3-(3-chloro-6'-((((1r,4r)-4-hydroxycyclohexyl)amino)methyl)-5'-methoxy-[2,3'-bipyridin]-4-yl)phenyl)-2-methoxypyridin-3-yl)methyl)-2,6-diazaspiro[3.4]octan-7-one;
2-((6-(2-chloro-3-(3-chloro-6'-((((1s,4r)-4-hydroxycyclohexyl)amino)methyl)-5'-methoxy-[2,3'-bipyridin]-4-yl)phenyl)-2-methoxypyridin-3-yl)methyl)-2,6-diazaspiro[3.4]octan-7-one;
2-((6-(2-chloro-3-(3-chloro-6'-((((1r,4s)-4-hydroxycyclohexyl)amino)methyl)-5'-methoxy-[2,3'-bipyridin]-4-yl)phenyl)-2-methoxypyridin-3-yl)methyl)-2,6-diazaspiro[3.4]octan-7-one;
2-((6-(2-chloro-3-(3-chloro-6'-((((1s,4s)-4-hydroxycyclohexyl)amino)methyl)-5'-methoxy-[2,3'-bipyridin]-4-yl)phenyl)-2-methoxypyridin-3-yl)methyl)-2,6-diazaspiro[3.4]octan-7-one;
(S)-2-((6-(2-chloro-3-(3-chloro-5'-methoxy-6'-((((5-oxopyrrolidin-2-yl)methyl)amino)methyl)-[2,3'-bipyridin]-4-yl)phenyl)-2-methoxypyridin-3-yl)methyl)-2,6-diazaspiro[3.4]octan-7-one;
(R)-2-((6-(2-chloro-3-(3-chloro-5'-methoxy-6'-((((5-oxopyrrolidin-2-yl)methyl)amino)methyl)-[2,3'-bipyridin]-4-yl)phenyl)-2-methoxypyridin-3-yl)methyl)-2,6-diazaspiro[3.4]octan-7-one;
(S)-2-((2'-(1-((5-(((1-acetylpiperidin-4-yl)amino)methyl)-6-methoxy-3-(trifluoromethyl)pyridin-2-yl)oxy)-2,3-dihydro-1H-inden-4-yl)-3'-chloro-6-methoxy-[2,4'-bipyridin]-5-yl)methyl)-2,6-diazaspiro[3.4]octan-7-one;
(R)-2-((2'-(1-((5-(((1-acetylpiperidin-4-yl)amino)methyl)-6-methoxy-3-(trifluoromethyl)pyridin-2-yl)oxy)-2,3-dihydro-1H-inden-4-yl)-3'-chloro-6-methoxy-[2,4'-bipyridin]-5-yl)methyl)-2,6-diazaspiro[3.4]octan-7-one;
(S)-2-((3'-chloro-6-methoxy-2'-(1-((6-methoxy-5-(((tetrahydro-2H-pyran-4-yl)amino)methyl)-3-(trifluoromethyl)pyridin-2-yl)oxy)-2,3-dihydro-1H-inden-4-yl)-[2,4'-bipyridin]-5-yl)methyl)-2,6-diazaspiro[3.4]octan-7-one;
(R)-2-((3'-chloro-6-methoxy-2'-(1-((6-methoxy-5-(((tetrahydro-2H-pyran-4-yl)amino)methyl)-3-(trifluoromethyl)pyridin-2-yl)oxy)-2,3-dihydro-1H-inden-4-yl)-[2,4'-bipyridin]-5-yl)methyl)-2,6-diazaspiro[3.4]octan-7-one;
2-((3'-chloro-2'-((S)-1-((5-((((S)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)-2,6-diazaspiro[3.4]octan-7-one;
2-((3'-chloro-2'-((S)-1-((5-((((R)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)-2,6-diazaspiro[3.4]octan-7-one;
2-((3'-chloro-2'-((R)-1-((5-((((S)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)-2,6-diazaspiro[3.4]octan-7-one;
2-((3'-chloro-2'-((R)-1-((5-((((R)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)-2,6-diazaspiro[3.4]octan-7-one;
2-(4-(5-chloro-6-(2-chloro-3-(6-methoxy-5-((7-oxo-2,6-diazaspiro[3.4]octan-2-yl)methyl)pyridin-2-yl)phenyl)pyrimidin-4-yl)-2-methoxybenzyl)-2,6-diazaspiro[3.4]octan-7-one;
(S)-6-(3-chloro-4-(2-chloro-3-(6-methoxy-5-((((5-oxopyrrolidin-2-yl)methyl)amino)methyl)pyridin-2-yl)phenyl)pyridin-2-yl)-2-methoxy-3-(2-(methylamino)ethyl)pyrrolo[2,1-f][1,2,4]triazin-4(3H)-one;
(R)-6-(3-chloro-4-(2-chloro-3-(6-methoxy-5-((((5-oxopyrrolidin-2-yl)methyl)amino)methyl)pyridin-2-yl)phenyl)pyridin-2-yl)-2-methoxy-3-(2-(methylamino)ethyl)pyrrolo[2,1-f][1,2,4]triazin-4(3H)-one;
1-(4-((4-(3-chloro-4-(2-chloro-3-(1-methyl-3-(((tetrahydro-2H-pyran-4-yl)amino)methyl)-1H-pyrrolo[2,3-b]pyridin-6-yl)phenyl)pyridin-2-yl)-2-methoxybenzyl)amino)piperidin-1-yl)ethan-1-one;
1-(4-((4-(3-chloro-4-(2-chloro-3-(1-methyl-3-((((tetrahydro-2H-pyran-4-yl)methyl)amino)methyl)-1H-pyrrolo[2,3-b]pyridin-6-yl)phenyl)pyridin-2-yl)-2-methoxybenzyl)amino)piperidin-1-yl)ethan-1-one;
1-(4-((4-(3-chloro-4-(2-chloro-3-(3-(((1,1-dioxidotetrahydro-2H-thiopyran-4-yl)amino)methyl)-1-methyl-1H-pyrrolo [2,3-b]pyridin-6-yl)phenyl)pyridin-2-yl)-2-methoxybenzyl)amino)piperidin-1-yl)ethan-1-one;
1-(4-((4-(3-chloro-4-(2-chloro-3-(3-(((2-hydroxyethyl)amino)methyl)-1-methyl-1H-pyrrolo[2,3-b]pyridin-6-yl)phenyl)pyridin-2-yl)-2-methoxybenzyl)amino)piperidin-1-yl)ethan-1-one;
1-(4-(((6-(2-chloro-3-(3-chloro-2-(4-(((2-hydroxyethyl)amino)methyl)-3-methoxyphenyl)pyridin-4-yl)phenyl)-1-methyl-1H-pyrrolo[2,3-b]pyridin-3-yl)methyl)amino)piperidin-1-yl)ethan-1-one;
(S)-5-((((6-(2-chloro-3-(3-chloro-2-(4-methyl-1,1-dioxido-2,3,4,5-tetrahydrobenzo[f][1,4]thiazepin-8-yl)pyridin-4-yl)phenyl)-2-methoxypyridin-3-yl)methyl)amino)methyl)pyrrolidin-2-one;
(R)-5-((((6-(2-chloro-3-(3-chloro-2-(4-methyl-1,1-dioxido-2,3,4,5-tetrahydrobenzo[f][1,4]thiazepin-8-yl)pyridin-4-yl)phenyl)-2-methoxypyridin-3-yl)methyl)amino)methyl)pyrrolidin-2-one;
1-(4-((6-(3-(6-(4-((4-acetylpiperazin-1-yl)methyl)-3-methoxyphenyl)-5-chloropyrimidin-4-yl)-2-chlorophenyl)-2-methoxypyridin-3-yl)methyl)piperazin-1-yl)ethan-1-one;
N-((6-(2-chloro-3-(3-chloro-2-(3-methoxy-4-(((tetrahydro-2H-pyran-4-yl)amino)methyl)phenyl)pyridin-4-yl)phenyl)-1-methyl-1H-pyrrolo[2,3-b]pyridin-3-yl)methyl)tetrahydro-2H-pyran-4-amine;
1-(4-(((6-(3-(2-(4-((6-acetyl-2,6-diazaspiro[3.3]heptan-2-yl)methyl)-3-methoxyphenyl)-3-chloropyridin-4-yl)-2-chlorophenyl)-1-methyl-1H-pyrrolo[2,3-b]pyridin-3-yl)methyl)amino)piperidin-1-yl)ethan-1-one;
1-(4-(((5-(3-(2-(4-(((1-acetylpiperidin-4-yl)amino)methyl)-3-methoxyphenyl)-3-chloropyridin-4-yl)-2-chlorophenyl)-3-methoxypyridin-2-yl)methyl)amino)piperidin-1-yl)ethan-1-one;
2-(4-(3-chloro-4-(2-chloro-3-(5-methoxy-6-((7-oxo-2,6-diazaspiro[3.4]octan-2-yl)methyl)pyridin-3-yl)phenyl)pyridin-2-yl)-2-methoxybenzyl)-2,6-diazaspiro[3.4]octan-7-one;
1-(4-(((5-(3-(6'-(((1-acetylpiperidin-4-yl)amino)methyl)-3-chloro-5'-methoxy-[2,3'-bipyridin]-4-yl)-2-chlorophenyl)-3-methoxypyridin-2-yl)methyl)amino)piperidin-1-yl)ethan-1-one;
2-(4-(5-chloro-6-(2-chloro-3-(5-((6-hydroxy-2-azaspiro[3.3]heptan-2-yl)methyl)-6-methoxypyridin-2-yl)phenyl)pyrimidin-4-yl)-2-methoxybenzyl)-2-azaspiro[3.3]heptan-6-ol;
2-((5-(2-chloro-3-(3-chloro-5'-methoxy-6'-((7-oxo-2,6-diazaspiro[3.4]octan-2-yl)methyl)-[2,3'-bipyridin]-4-yl)phenyl)-3-methoxypyridin-2-yl)methyl)-2,6-diazaspiro[3.4]octan-7-one;
N-((5-(2-chloro-3-(3-chloro-5'-methoxy-6'-(((tetrahydro-2H-pyran-4-yl)amino)methyl)-[2,3'-bipyridin]-4-yl)phenyl)-3-methoxypyridin-2-yl)methyl)tetrahydro-2H-pyran-4-amine;
2-(4-(3-chloro-4-(2-chloro-3-(6-methoxy-5-((6-methyl-7-oxo-2,6-diazaspiro[3.4]octan-2-yl)methyl)pyridin-2-yl)phenyl)pyridin-2-yl)-2-methoxybenzyl)-6-methyl-2,6-diazaspiro[3.4]octan-7-one;
1-(4-(((6-(3-(5-(((1-acetylpiperidin-4-yl)amino)methyl)-3'-chloro-6-methoxy-[2,4'-bipyridin]-2'-yl)-2-chlorophenyl)-2-methoxypyridin-3-yl)methyl)amino)piperidin-1-yl)ethan-1-one;
2-((6-(2-chloro-3-(3'-chloro-6-methoxy-5-((7-oxo-2,6-diazaspiro[3.4]octan-2-yl)methyl)-[2,4'-bipyridin]-2'-yl)phenyl)-2-methoxypyridin-3-yl)methyl)-2,6-diazaspiro[3.4]octan-7-one;
N-((3'-chloro-2'-(2-chloro-3-(6-methoxy-5-(((tetrahydro-2H-pyran-4-yl)amino)methyl)pyridin-2-yl)phenyl)-6-methoxy-[2,4'-bipyridin]-5-yl)methyl)tetrahydro-2H-pyran-4-amine;
(S)-5-((((3'-chloro-2'-(2-chloro-3-(6-methoxy-5-(((((S)-5-oxopyrrolidin-2-yl)methyl)amino)methyl)pyridin-2-yl)phenyl)-6-methoxy-[2,4'-bipyridin]-5-yl)methyl)amino)methyl)pyrrolidin-2-one;
(S)-5-((((3'-chloro-2'-(2-chloro-3-(6-methoxy-5-(((((R)-5-oxopyrrolidin-2-yl)methyl)amino)methyl)pyridin-2-yl)phenyl)-6-methoxy-[2,4'-bipyridin]-5-yl)methyl)amino)methyl)pyrrolidin-2-one;
(R)-5-((((3'-chloro-2'-(2-chloro-3-(6-methoxy-5-(((((S)-5-oxopyrrolidin-2-yl)methyl)amino)methyl)pyridin-2-yl)phenyl)-6-methoxy-[2,4'-bipyridin]-5-yl)methyl)amino)methyl)pyrrolidin-2-one;
(R)-5-((((3'-chloro-2'-(2-chloro-3-(6-methoxy-5-(((((R)-5-oxopyrrolidin-2-yl)methyl)amino)methyl)pyridin-2-yl)phenyl)-6-methoxy-[2,4'-bipyridin]-5-yl)methyl)amino)methyl)pyrrolidin-2-one;
2-(4-(3-chloro-4-(3-(6-methoxy-5-((7-oxo-2,6-diazaspiro[3.4]octan-2-yl)methyl)pyridin-2-yl)-2-(trifluoromethyl)phenyl)pyridin-2-yl)-2-methoxybenzyl)-2,6-diazaspiro[3.4]octan-7-one;
1-(6-((6-(3-(2-(4-((6-acetyl-2,6-diazaspiro[3.3]heptan-2-yl)methyl)-3-methoxyphenyl)-3-chloropyridin-4-yl)-2-(trifluoromethyl)phenyl)-2-methoxypyridin-3-yl)methyl)-2,6-diazaspiro[3.3]heptan-2-yl)ethan-1-one;
6-(4-(4-(3-(5-((2-oxa-6-azaspiro[3.3]heptan-6-yl)methyl)-6-methoxypyridin-2-yl)-2-(trifluoromethyl)phenyl)-3-chloropyridin-2-yl)-2-methoxybenzyl)-2-oxa-6-azaspiro[3.3]heptane;
2-(4-(4-(3-(3-(((1-acetylpiperidin-4-yl)amino)methyl)-1-methyl-1H-pyrrolo[2,3-b]pyridin-6-yl)-2-chlorophenyl)-3-chloropyridin-2-yl)-2-methoxybenzyl)-2,6-diazaspiro[3.4]octan-7-one;
(S)-1-(((6-(3-(3-chloro-2-(4-((((S)-2-hydroxypropyl)amino)methyl)-3-methoxyphenyl)pyridin-4-yl)-2-(trifluoromethyl)phenyl)-2-methoxypyridin-3-yl)methyl)amino)propan-2-ol;
(S)-1-(((6-(3-(3-chloro-2-(4-((((R)-2-hydroxypropyl)amino)methyl)-3-methoxyphenyl)pyridin-4-yl)-2-(trifluoromethyl)phenyl)-2-methoxypyridin-3-yl)methyl)amino)propan-2-ol;
(R)-1-(((6-(3-(3-chloro-2-(4-((((S)-2-hydroxypropyl)amino)methyl)-3-methoxyphenyl)pyridin-4-yl)-2-(trifluoromethyl)phenyl)-2-methoxypyridin-3-yl)methyl)amino)propan-2-ol;
(R)-1-(((6-(3-(3-chloro-2-(4-((((R)-2-hydroxypropyl)amino)methyl)-3-methoxyphenyl)pyridin-4-yl)-2-(trifluoromethyl)phenyl)-2-methoxypyridin-3-yl)methyl)amino)propan-2-ol;
2-(((6-(2-chloro-3-(5-chloro-6-(4-(((2-hydroxyethyl)amino)methyl)-3-methoxyphenyl)pyrimidin-4-yl)phenyl)-2-methoxypyridin-3-yl)methyl)amino)ethan-1-ol;
5-((((6-(2-chloro-3-(3-chloro-2-(1,1-dioxido-4-(((S)-5-oxopyrrolidin-2-yl)methyl)-2,3,4,5-tetrahydrobenzo[f][1,4]thiazepin-8-yl)pyridin-4-yl)phenyl)-2-methoxypyridin-3-yl)methyl)amino)methyl)pyrrolidin-2-one;
5-((((6-(2-chloro-3-(3-chloro-2-(1,1-dioxido-4-(((R)-5-oxopyrrolidin-2-yl)methyl)-2,3,4,5-tetrahydrobenzo[f][1,4]thiazepin-8-yl)pyridin-4-yl)phenyl)-2-methoxypyridin-3-yl)methyl)amino)methyl)pyrrolidin-2-one;
(S)-5-((((6-(2-chloro-3-(3-chloro-2-(1,1-dioxido-4-(((S)-5-oxopyrrolidin-2-yl)methyl)-2,3,4,5-tetrahydrobenzo[f][1,4]thiazepin-8-yl)pyridin-4-yl)phenyl)-2-methoxypyridin-3-yl)methyl)amino)methyl)pyrrolidin-2-one;
(S)-5-((((6-(2-chloro-3-(3-chloro-2-(1,1-dioxido-4-(((R)-5-oxopyrrolidin-2-yl)methyl)-2,3,4,5-tetrahydrobenzo[f][1,4]thiazepin-8-yl)pyridin-4-yl)phenyl)-2-methoxypyridin-3-yl)methyl)amino)methyl)pyrrolidin-2-one;
(R)-5-((((6-(2-chloro-3-(3-chloro-2-(1,1-dioxido-4-(((S)-5-oxopyrrolidin-2-yl)methyl)-2,3,4,5-tetrahydrobenzo[f][1,4]thiazepin-8-yl)pyridin-4-yl)phenyl)-2-methoxypyridin-3-yl)methyl)amino)methyl)pyrrolidin-2-one;
(R)-5-((((6-(2-chloro-3-(3-chloro-2-(1,1-dioxido-4-(((R)-5-oxopyrrolidin-2-yl)methyl)-2,3,4,5-tetrahydrobenzo[f][1,4]thiazepin-8-yl)pyridin-4-yl)phenyl)-2-methoxypyridin-3-yl)methyl)amino)methyl)pyrrolidin-2-one;
1-(4-(((6-(2-chloro-3-(3-chloro-2-(8-ethoxy-2-((((1r,4r)-4-hydroxycyclohexyl)amino)methyl)-[1,2,4]triazolo[1,5-a]pyridin-6-yl)pyridin-4-yl)phenyl)-2-methoxypyridin-3-yl)methyl)amino)piperidin-1-yl)ethan-1-one;
1-(4-(((6-(2-chloro-3-(3-chloro-2-(8-ethoxy-2-((((1s,4r)-4-hydroxycyclohexyl)amino)methyl)-[1,2,4]triazolo[1,5-a]pyridin-6-yl)pyridin-4-yl)phenyl)-2-methoxypyridin-3-yl)methyl)amino)piperidin-1-yl)ethan-1-one;
1-(4-(((6-(2-chloro-3-(3-chloro-2-(8-ethoxy-2-((((1r,4s)-4-hydroxycyclohexyl)amino)methyl)-[1,2,4]triazolo[1,5-a]pyridin-6-yl)pyridin-4-yl)phenyl)-2-methoxypyridin-3-yl)methyl)amino)piperidin-1-yl)ethan-1-one;
1-(4-(((6-(2-chloro-3-(3-chloro-2-(8-ethoxy-2-((((1s,4s)-4-hydroxycyclohexyl)amino)methyl)-[1,2,4]triazolo[1,5-a]pyridin-6-yl)pyridin-4-yl)phenyl)-2-methoxypyridin-3-yl)methyl)amino)piperidin-1-yl)ethan-1-one;
1-(4-(((6-(2-chloro-3-(3-chloro-2-(8-ethoxy-2-((((1r,3r)-3-hydroxy-3-methylcyclobutyl)amino)methyl)-[1,2,4]triazolo[1,5-a]pyridin-6-yl)pyridin-4-yl)phenyl)-2-methoxypyridin-3-yl)methyl)amino)piperidin-1-yl)ethan-1-one;
1-(4-(((6-(2-chloro-3-(3-chloro-2-(8-ethoxy-2-((((1s,3r)-3-hydroxy-3-methylcyclobutyl)amino)methyl)-[1,2,4]triazolo[1,5-a]pyridin-6-yl)pyridin-4-yl)phenyl)-2-methoxypyridin-3-yl)methyl)amino)piperidin-1-yl)ethan-1-one;
1-(4-(((6-(2-chloro-3-(3-chloro-2-(8-ethoxy-2-((((1r,3s)-3-hydroxy-3-methylcyclobutyl)amino)methyl)-[1,2,4]triazolo[1,5-a]pyridin-6-yl)pyridin-4-yl)phenyl)-2-methoxypyridin-3-yl)methyl)amino)piperidin-1-yl)ethan-1-one;
1-(4-(((6-(2-chloro-3-(3-chloro-2-(8-ethoxy-2-((((1s,3s)-3-hydroxy-3-methylcyclobutyl)amino)methyl)-[1,2,4]triazolo[1,5-a]pyridin-6-yl)pyridin-4-yl)phenyl)-2-methoxypyridin-3-yl)methyl)amino)piperidin-1-yl)ethan-1-one;
1-(4-(((6-(4-(3-(5-(((1-acetylpiperidin-4-yl)amino)methyl)-6-methoxypyridin-2-yl)-2-chlorophenyl)-3-chloropyridin-2-yl)-8-ethoxy-[1,2,4]triazolo[1,5-a]pyridin-2-yl)methyl)amino)piperidin-1-yl)ethan-1-one;
2-((6-(4-(3-(5-(((1-acetylpiperidin-4-yl)amino)methyl)-6-methoxypyridin-2-yl)-2-chlorophenyl)-3-chloropyridin-2-yl)-8-ethoxy-[1,2,4]triazolo[1,5-a]pyridin-2-yl)methyl)-2,6-diazaspiro[3.4]octan-7-one;
N-((6-(3-(3-chloro-2-(4-(((3-fluoropropyl)amino)methyl)-3-methoxyphenyl)pyridin-4-yl)-2-(trifluoromethyl)phenyl)-2-methoxypyridin-3-yl)methyl)-3-fluoropropan-1-amine;
(1r,4r)-4-(((6-(3-(3-chloro-2-(4-((((1r,4r)-4-hydroxycyclohexyl)amino)methyl)-3-methoxyphenyl)pyridin-4-yl)-2-(trifluoromethyl)phenyl)-2-methoxypyridin-3-yl)methyl)amino)cyclohexan-1-ol;
(1r,4r)-4-(((6-(3-(3-chloro-2-(4-((((1r,4s)-4-hydroxycyclohexyl)amino)methyl)-3-methoxyphenyl)pyridin-4-yl)-2-(trifluoromethyl)phenyl)-2-methoxypyridin-3-yl)methyl)amino)cyclohexan-1-ol;
(1r,4r)-4-(((6-(3-(3-chloro-2-(4-((((1s,4r)-4-hydroxycyclohexyl)amino)methyl)-3-methoxyphenyl)pyridin-4-yl)-2-(trifluoromethyl)phenyl)-2-methoxypyridin-3-yl)methyl)amino)cyclohexan-1-ol;
(1r,4r)-4-(((6-(3-(3-chloro-2-(4-((((1s,4s)-4-hydroxycyclohexyl)amino)methyl)-3-methoxyphenyl)pyridin-4-yl)-2-(trifluoromethyl)phenyl)-2-methoxypyridin-3-yl)methyl)amino)cyclohexan-1-ol;
(1r,4s)-4-(((6-(3-(3-chloro-2-(4-((((1r,4r)-4-hydroxycyclohexyl)amino)methyl)-3-methoxyphenyl)pyridin-4-yl)-2-(trifluoromethyl)phenyl)-2-methoxypyridin-3-yl)methyl)amino)cyclohexan-1-ol;
(1r,4s)-4-(((6-(3-(3-chloro-2-(4-((((1r,4s)-4-hydroxycyclohexyl)amino)methyl)-3-methoxyphenyl)pyridin-4-yl)-2-(trifluoromethyl)phenyl)-2-methoxypyridin-3-yl)methyl)amino)cyclohexan-1-ol;
(1r,4s)-4-(((6-(3-(3-chloro-2-(4-((((1s,4r)-4-hydroxycyclohexyl)amino)methyl)-3-methoxyphenyl)pyridin-4-yl)-2-(trifluoromethyl)phenyl)-2-methoxypyridin-3-yl)methyl)amino)cyclohexan-1-ol;
(1r,4s)-4-(((6-(3-(3-chloro-2-(4-((((1s,4s)-4-hydroxycyclohexyl)amino)methyl)-3-methoxyphenyl)pyridin-4-yl)-2-(trifluoromethyl)phenyl)-2-methoxypyridin-3-yl)methyl)amino)cyclohexan-1-ol;
(1s,4r)-4-(((6-(3-(3-chloro-2-(4-((((1r,4r)-4-hydroxycyclohexyl)amino)methyl)-3-methoxyphenyl)pyridin-4-yl)-2-(trifluoromethyl)phenyl)-2-methoxypyridin-3-yl)methyl)amino)cyclohexan-1-ol;
(1s,4r)-4-(((6-(3-(3-chloro-2-(4-((((1r,4s)-4-hydroxycyclohexyl)amino)methyl)-3-methoxyphenyl)pyridin-4-yl)-2-(trifluoromethyl)phenyl)-2-methoxypyridin-3-yl)methyl)amino)cyclohexan-1-ol;
(1s,4r)-4-(((6-(3-(3-chloro-2-(4-((((1s,4r)-4-hydroxycyclohexyl)amino)methyl)-3-methoxyphenyl)pyridin-4-yl)-2-(trifluoromethyl)phenyl)-2-methoxypyridin-3-yl)methyl)amino)cyclohexan-1-ol;
(1s,4r)-4-(((6-(3-(3-chloro-2-(4-((((1s,4s)-4-hydroxycyclohexyl)amino)methyl)-3-methoxyphenyl)pyridin-4-yl)-2-(trifluoromethyl)phenyl)-2-methoxypyridin-3-yl)methyl)amino)cyclohexan-1-ol;
(1s,4s)-4-(((6-(3-(3-chloro-2-(4-((((1r,4r)-4-hydroxycyclohexyl)amino)methyl)-3-methoxyphenyl)pyridin-4-yl)-2-(trifluoromethyl)phenyl)-2-methoxypyridin-3-yl)methyl)amino)cyclohexan-1-ol;
(1s,4s)-4-(((6-(3-(3-chloro-2-(4-((((1r,4s)-4-hydroxycyclohexyl)amino)methyl)-3-methoxyphenyl)pyridin-4-yl)-2-(trifluoromethyl)phenyl)-2-methoxypyridin-3-yl)methyl)amino)cyclohexan-1-ol;
(1s,4s)-4-(((6-(3-(3-chloro-2-(4-((((1s,4r)-4-hydroxycyclohexyl)amino)methyl)-3-methoxyphenyl)pyridin-4-yl)-2-(trifluoromethyl)phenyl)-2-methoxypyridin-3-yl)methyl)amino)cyclohexan-1-ol;
(1s,4s)-4-(((6-(3-(3-chloro-2-(4-((((1s,4s)-4-hydroxycyclohexyl)amino)methyl)-3-methoxyphenyl)pyridin-4-yl)-2-(trifluoromethyl)phenyl)-2-methoxypyridin-3-yl)methyl)amino)cyclohexan-1-ol;
1-(6-((6-(3-(6-(4-((6-acetyl-2,6-diazaspiro[3.3]heptan-2-yl)methyl)-3-methoxyphenyl)-5-chloropyrimidin-4-yl)-2-chlorophenyl)-2-methoxypyridin-3-yl)methyl)-2,6-diazaspiro[3.3]heptan-2-yl)ethan-1-one;
N-(3-(3-chloro-2-(3-methoxy-4-((7-oxo-2,6-diazaspiro[3.4]octan-2-yl)methyl)phenyl)pyridin-4-yl)-2-methylphenyl)-5-((7-oxo-2,6-diazaspiro[3.4]octan-2-yl)methyl)picolinamide;
(3-(((6-(2-chloro-3-(3-chloro-2-(4-(((3-(hydroxymethyl)bicyclo[1.1.1]pentan-1-yl)amino)methyl)-3-methoxyphenyl)pyridin-4-yl)phenyl)-2-methoxypyridin-3-yl)methyl)amino)bicyclo[1.1.1]pentan-1-yl)methanol;
(1s,3s)-3-(((6-(2-chloro-3-(5-chloro-6-(4-((((1s,3s)-3-hydroxycyclobutyl)amino)methyl)-3-methoxyphenyl)pyrimidin-4-yl)phenyl)-2-methoxypyridin-3-yl)methyl)amino)cyclobutan-1-ol;
(1r,3r)-3-(((6-(2-chloro-3-(5-chloro-6-(4-((((1r,3r)-3-hydroxycyclobutyl)amino)methyl)-3-methoxyphenyl)pyrimidin-4-yl)phenyl)-2-methoxypyridin-3-yl)methyl)amino)cyclobutan-1-ol;
(1r,3r)-3-(((6-(2-chloro-3-(5-chloro-6-(4-((((1r,3s)-3-hydroxycyclobutyl)amino)methyl)-3-methoxyphenyl)pyrimidin-4-yl)phenyl)-2-methoxypyridin-3-yl)methyl)amino)cyclobutan-1-ol;
(1r,3r)-3-(((6-(2-chloro-3-(5-chloro-6-(4-((((1s,3r)-3-hydroxycyclobutyl)amino)methyl)-3-methoxyphenyl)pyrimidin-4-yl)phenyl)-2-methoxypyridin-3-yl)methyl)amino)cyclobutan-1-ol;
(1r,3r)-3-(((6-(2-chloro-3-(5-chloro-6-(4-((((1s,3s)-3-hydroxycyclobutyl)amino)methyl)-3-methoxyphenyl)pyrimidin-4-yl)phenyl)-2-methoxypyridin-3-yl)methyl)amino)cyclobutan-1-ol;
(1r,3s)-3-(((6-(2-chloro-3-(5-chloro-6-(4-((((1r,3r)-3-hydroxycyclobutyl)amino)methyl)-3-methoxyphenyl)pyrimidin-4-yl)phenyl)-2-methoxypyridin-3-yl)methyl)amino)cyclobutan-1-ol;
(1r,3s)-3-(((6-(2-chloro-3-(5-chloro-6-(4-((((1r,3s)-3-hydroxycyclobutyl)amino)methyl)-3-methoxyphenyl)pyrimidin-4-yl)phenyl)-2-methoxypyridin-3-yl)methyl)amino)cyclobutan-1-ol;
(1r,3s)-3-(((6-(2-chloro-3-(5-chloro-6-(4-((((1s,3r)-3-hydroxycyclobutyl)amino)methyl)-3-methoxyphenyl)pyrimidin-4-yl)phenyl)-2-methoxypyridin-3-yl)methyl)amino)cyclobutan-1-ol;
(1r,3s)-3-(((6-(2-chloro-3-(5-chloro-6-(4-((((1s,3s)-3-hydroxycyclobutyl)amino)methyl)-3-methoxyphenyl)pyrimidin-4-yl)phenyl)-2-methoxypyridin-3-yl)methyl)amino)cyclobutan-1-ol;
(1s,3r)-3-(((6-(2-chloro-3-(5-chloro-6-(4-((((1r,3r)-3-hydroxycyclobutyl)amino)methyl)-3-methoxyphenyl)pyrimidin-4-yl)phenyl)-2-methoxypyridin-3-yl)methyl)amino)cyclobutan-1-ol;
(1s,3r)-3-(((6-(2-chloro-3-(5-chloro-6-(4-((((1r,3s)-3-hydroxycyclobutyl)amino)methyl)-3-methoxyphenyl)pyrimidin-4-yl)phenyl)-2-methoxypyridin-3-yl)methyl)amino)cyclobutan-1-ol;
(1s,3r)-3-(((6-(2-chloro-3-(5-chloro-6-(4-((((1s,3r)-3-hydroxycyclobutyl)amino)methyl)-3-methoxyphenyl)pyrimidin-4-yl)phenyl)-2-methoxypyridin-3-yl)methyl)amino)cyclobutan-1-ol;
(1s,3r)-3-(((6-(2-chloro-3-(5-chloro-6-(4-((((1s,3s)-3-hydroxycyclobutyl)amino)methyl)-3-methoxyphenyl)pyrimidin-4-yl)phenyl)-2-methoxypyridin-3-yl)methyl)amino)cyclobutan-1-ol;
(1s,3s)-3-(((6-(2-chloro-3-(5-chloro-6-(4-((((1r,3r)-3-hydroxycyclobutyl)amino)methyl)-3-methoxyphenyl)pyrimidin-4-yl)phenyl)-2-methoxypyridin-3-yl)methyl)amino)cyclobutan-1-ol;
(1s,3s)-3-(((6-(2-chloro-3-(5-chloro-6-(4-((((1r,3s)-3-hydroxycyclobutyl)amino)methyl)-3-methoxyphenyl)pyrimidin-4-yl)phenyl)-2-methoxypyridin-3-yl)methyl)amino)cyclobutan-1-ol;
(1s,3s)-3-(((6-(2-chloro-3-(5-chloro-6-(4-((((1s,3r)-3-hydroxycyclobutyl)amino)methyl)-3-methoxyphenyl)pyrimidin-4-yl)phenyl)-2-methoxypyridin-3-yl)methyl)amino)cyclobutan-1-ol;
1-(6-((6-(2-chloro-3-(3-chloro-2-(3-methoxy-4-((6-(2-methoxyacetyl)-2,6-diazaspiro[3.3]heptan-2-yl)methyl)phenyl)pyridin-4-yl)phenyl)-2-methoxypyridin-3-yl)methyl)-2,6-diazaspiro[3 .3]heptan-2-yl)-2-methoxyethan-1-one;
1-(4-(((6-(2-chloro-3-(3-chloro-2-(2-((((1r,4r)-4-hydroxycyclohexyl)amino)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one;
1-(4-(((6-(2-chloro-3-(3-chloro-2-(2-((((1s,4r)-4-hydroxycyclohexyl)amino)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one;
1-(4-(((6-(2-chloro-3-(3-chloro-2-(2-((((1r,4s)-4-hydroxycyclohexyl)amino)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one;
1-(4-(((6-(2-chloro-3-(3-chloro-2-(2-((((1s,4s)-4-hydroxycyclohexyl)amino)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one;
1-(4-(((6-(2-chloro-3-(3-chloro-2-(2-((((1r,3r)-3-hydroxy-3-methylcyclobutyl)amino)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one;
1-(4-(((6-(2-chloro-3-(3-chloro-2-(2-((((1s,3r)-3-hydroxy-3-methylcyclobutyl)amino)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one;
1-(4-(((6-(2-chloro-3-(3-chloro-2-(2-((((1r,3s)-3-hydroxy-3-methylcyclobutyl)amino)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one;
1-(4-(((6-(2-chloro-3-(3-chloro-2-(2-((((1s,3s)-3-hydroxy-3-methylcyclobutyl)amino)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one;
2-((6-(2-chloro-3-(3-chloro-2-(3-(difluoromethoxy)-4-((7-oxo-2,6-diazaspiro[3.4]octan-2-yl)methyl)phenyl)pyridin-4-yl)phenyl)-2-methoxypyridin-3-yl)methyl)-2,6-diazaspiro[3.4]octan-7-one;
1-(6-((6-(3-(2-(4-((6-acetyl-2,6-diazaspiro[3.3]heptan-2-yl)methyl)-3-(difluoromethoxy)phenyl)-3-chloropyridin-4-yl)-2-chlorophenyl)-2-methoxypyridin-3-yl)methyl)-2,6-diazaspiro[3.3]heptan-2-yl)ethan-1-one;
1-(4-(((S)-1-(6-(3-(2-(4-((S)-1-((1-acetylpiperidin-4-yl)amino)ethyl)-3-methoxyphenyl)-3-chloropyridin-4-yl)-2-chlorophenyl)-2-methoxypyridin-3-yl)ethyl)amino)piperidin-1-yl)ethan-1-one;
1-(4-(((S)-1-(6-(3-(2-(4-((R)-1-((1-acetylpiperidin-4-yl)amino)ethyl)-3-methoxyphenyl)-3-chloropyridin-4-yl)-2-chlorophenyl)-2-methoxypyridin-3-yl)ethyl)amino)piperidin-1-yl)ethan-1-one;
1-(4-(((R)-1-(6-(3-(2-(4-((S)-1-((1-acetylpiperidin-4-yl)amino)ethyl)-3-methoxyphenyl)-3-chloropyridin-4-yl)-2-chlorophenyl)-2-methoxypyridin-3-yl)ethyl)amino)piperidin-1-yl)ethan-1-one;
1-(4-(((R)-1-(6-(3-(2-(4-((R)-1-((1-acetylpiperidin-4-yl)amino)ethyl)-3-methoxyphenyl)-3-chloropyridin-4-yl)-2-chlorophenyl)-2-methoxypyridin-3-yl)ethyl)amino)piperidin-1-yl)ethan-1-one;
1-(4-(((6-(2-chloro-3-(3-chloro-2-(3-methoxy-4-(((tetrahydro-2H-pyran-4-yl)amino)methyl)phenyl)pyridin-4-yl)phenyl)-1-methyl-1H-pyrrolo[2,3-b]pyridin-3-yl)methyl)(methyl)amino)piperidin-1-yl)ethan-1-one;
1-(4-(((6-(3-(2-(4-((6-acetyl-2,6-diazaspiro[3.3]heptan-2-yl)methyl)-3-methoxyphenyl)-3-chloropyridin-4-yl)-2-chlorophenyl)-1-methyl-1H-pyrrolo[2,3-b]pyridin-3-yl)methyl)(methyl)amino)piperidin-1-yl)ethan-1-one;
1-(4-(((6-(3-(3-chloro-2-(3-methoxy-4-(((1-(2-methoxyacetyl)piperidin-4-yl)amino)methyl)phenyl)pyridin-4-yl)-2-(trifluoromethyl)phenyl)-2-methoxypyridin-3-yl)methyl)amino)piperidin-1-yl)-2-methoxyethan-1-one;
2-(4-(4-(3-(3-(((1-acetylpiperidin-4-yl)(methyl)amino)methyl)-1-methyl-1H-pyrrolo[2,3-b]pyridin-6-yl)-2-chlorophenyl)-3-chloropyridin-2-yl)-2-methoxybenzyl)-2,6-diazaspiro[3.4]octan-7-one;
N-(4-(4-(3-(5-(((2-oxaspiro[3.3]heptan-6-yl)amino)methyl)-6-methoxypyridin-2-yl)-2-(trifluoromethyl)phenyl)-3-chloropyridin-2-yl)-2-methoxybenzyl)-2-oxaspiro[3.3]heptan-6-amine;
2-(4-(3-chloro-4-(2-chloro-3-(1-methyl-3-(((tetrahydro-2H-pyran-4-yl)amino)methyl)-1H-pyrrolo[2,3-b]pyridin-6-yl)phenyl)pyridin-2-yl)-2-methoxybenzyl)-2,6-diazaspiro[3.4]octan-7-one;
2-((4-(3-chloro-4-(2-chloro-3-(1-methyl-3-(((tetrahydro-2H-pyran-4-yl)amino)methyl)-1H-pyrrolo[2,3-b]pyridin-6-yl)phenyl)pyridin-2-yl)-2-methoxybenzyl)amino)ethan-1-ol;
1-(4-(((6-(3-(2-(4-(((1-acetylpiperidin-4-yl)amino)methyl)-3-methoxyphenyl)-3-chloropyridin-4-yl)-2-chlorophenyl)-2-methoxypyridin-3-yl)methyl)amino)piperidin-1-yl)-3-methoxypropan-1-one;
(S)-5-((((3'-chloro-6-methoxy-2'-((S)-1-((6-methoxy-5-(((tetrahydro-2H-pyran-4-yl)amino)methyl)-3-(trifluoromethyl)pyridin-2-yl)oxy)-2,3-dihydro-1H-inden-4-yl)-[2,4'-bipyridin]-5-yl)methyl)amino)methyl)pyrrolidin-2-one;
(S)-5-((((3'-chloro-6-methoxy-2'-((R)-1-((6-methoxy-5-(((tetrahydro-2H-pyran-4-yl)amino)methyl)-3-(trifluoromethyl)pyridin-2-yl)oxy)-2,3-dihydro-1H-inden-4-yl)-[2,4'-bipyridin]-5-yl)methyl)amino)methyl)pyrrolidin-2-one;
(R)-5-((((3'-chloro-6-methoxy-2'-((S)-1-((6-methoxy-5-(((tetrahydro-2H-pyran-4-yl)amino)methyl)-3-(trifluoromethyl)pyridin-2-yl)oxy)-2,3-dihydro-1H-inden-4-yl)-[2,4'-bipyridin]-5-yl)methyl)amino)methyl)pyrrolidin-2-one;
(R)-5-((((3'-chloro-6-methoxy-2'-((R)-1-((6-methoxy-5-(((tetrahydro-2H-pyran-4-yl)amino)methyl)-3-(trifluoromethyl)pyridin-2-yl)oxy)-2,3-dihydro-1H-inden-4-yl)-[2,4'-bipyridin]-5-yl)methyl)amino)methyl)pyrrolidin-2-one;
1-(6-(4-(3-chloro-4-(2-chloro-3-(1-methyl-3-(((tetrahydro-2H-pyran-4-yl)amino)methyl)-1H-pyrrolo[2,3-b]pyridin-6-yl)phenyl)pyridin-2-yl)-2-methoxybenzyl)-2,6-diazaspiro[3.3]heptan-2-yl)ethan-1-one;
(S)-5-((((6-(3-(3-chloro-2-(3-methoxy-4-(((((S)-5-oxopyrrolidin-2-yl)methyl)amino)methyl)phenyl)pyridin-4-yl)-2-(trifluoromethyl)phenyl)-2-methoxypyridin-3-yl)methyl)amino)methyl)pyrrolidin-2-one;
(S)-5-((((6-(3-(3-chloro-2-(3-methoxy-4-(((((R)-5-oxopyrrolidin-2-yl)methyl)amino)methyl)phenyl)pyridin-4-yl)-2-(trifluoromethyl)phenyl)-2-methoxypyridin-3-yl)methyl)amino)methyl)pyrrolidin-2-one;
(R)-5-((((6-(3-(3-chloro-2-(3-methoxy-4-(((((S)-5-oxopyrrolidin-2-yl)methyl)amino)methyl)phenyl)pyridin-4-yl)-2-(trifluoromethyl)phenyl)-2-methoxypyridin-3-yl)methyl)amino)methyl)pyrrolidin-2-one;
(R)-5-((((6-(3-(3-chloro-2-(3-methoxy-4-(((((R)-5-oxopyrrolidin-2-yl)methyl)amino)methyl)phenyl)pyridin-4-yl)-2-(trifluoromethyl)phenyl)-2-methoxypyridin-3-yl)methyl)amino)methyl)pyrrolidin-2-one;
(S)-5-((((6-(2-chloro-3-(3-chloro-2-(4-((S)-2-hydroxypropyl)-1,1-dioxido-2,3,4,5-tetrahydrobenzo[f][1,4]thiazepin-8-yl)pyridin-4-yl)phenyl)-2-methoxypyridin-3-yl)methyl)amino)methyl)pyrrolidin-2-one;
(S)-5-((((6-(2-chloro-3-(3-chloro-2-(4-((R)-2-hydroxypropyl)-1,1-dioxido-2,3,4,5-tetrahydrobenzo[f][1,4]thiazepin-8-yl)pyridin-4-yl)phenyl)-2-methoxypyridin-3-yl)methyl)amino)methyl)pyrrolidin-2-one;
(R)-5-((((6-(2-chloro-3-(3-chloro-2-(4-((S)-2-hydroxypropyl)-1,1-dioxido-2,3,4,5-tetrahydrobenzo[f][1,4]thiazepin-8-yl)pyridin-4-yl)phenyl)-2-methoxypyridin-3-yl)methyl)amino)methyl)pyrrolidin-2-one;
(R)-5-((((6-(2-chloro-3-(3-chloro-2-(4-((R)-2-hydroxypropyl)-1,1-dioxido-2,3,4,5-tetrahydrobenzo[f][1,4]thiazepin-8-yl)pyridin-4-yl)phenyl)-2-methoxypyridin-3-yl)methyl)amino)methyl)pyrrolidin-2-one;
2-(((6-(3-(3-chloro-2-(4-(((2-hydroxyethyl)amino)methyl)-3-methoxyphenyl)pyridin-4-yl)-2-(trifluoromethyl)phenyl)-2-methoxypyridin-3-yl)methyl)amino)ethan-1-ol;
1-(6-(3-(3-chloro-2-(3-methoxy-4-((methylamino)methyl)phenyl)pyridin-4-yl)-2-(trifluoromethyl)phenyl)-2-methoxypyridin-3-yl)-N-methylmethanamine;
N-((6-(3-(3-chloro-2-(3-methoxy-4-(((2-methoxyethyl)amino)methyl)phenyl)pyridin-4-yl)-2-(trifluoromethyl)phenyl)-2-methoxypyridin-3-yl)methyl)-2-methoxyethan-1-amine;
2-(2-(4-(((1-acetylpiperidin-4-yl)amino)methyl)-3-methoxyphenyl)-3-chloropyridin-4-yl)-6-(5-(((1-acetylpiperidin-4-yl)amino)methyl)-6-methoxypyridin-2-yl)benzonitrile;
1-(4-(((6-(2-chloro-3-(3-chloro-2-(4-((4-hydroxypiperidin-1-yl)methyl)-3-methoxyphenyl)pyridin-4-yl)phenyl)-2-methoxypyridin-3-yl)methyl)amino)piperidin-1-yl)-3-methoxypropan-1-one;
(S)-5-((((6-(2-chloro-3-(3-chloro-2-(3-(difluoromethoxy)-4-(((((S)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
(S)-5-((((6-(2-chloro-3-(3-chloro-2-(3-(difluoromethoxy)-4-(((((R)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
(R)-5-((((6-(2-chloro-3-(3-chloro-2-(3-(difluoromethoxy)-4-(((((S)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
(R)-5-((((6-(2-chloro-3-(3-chloro-2-(3-(difluoromethoxy)-4-(((((R)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
N-((6-(2-chloro-3-(3-chloro-2-(3-(difluoromethoxy)-4-(((tetrahydro-2H-pyran-4-yl)amino)methyl)phenyl)pyridin-4-yl)phenyl)-2-methoxypyridin-3-yl)methyl)tetrahydro-2H-pyran-4-amine;
4-(((6-(2-chloro-3-(3-chloro-2-(3-(difluoromethoxy)-4-(((1-methyl-2-oxopiperidin-4-yl)amino)methyl)phenyl)pyridin-4-yl)phenyl)-2-methoxypyridin-3-yl)methyl)amino)-1-methylpiperidin-2-one;
2-(4-(((6-(2-chloro-3-(3-chloro-2-(3-(difluoromethoxy)-4-(((1-(2-hydroxyethyl)piperidin-4-yl)amino)methyl)phenyl)pyridin-4-yl)phenyl)-2-methoxypyridin-3-yl)methyl)amino)piperidin-1-yl)ethan-1-ol;
1-(4-(((6-(3-(4-(4-(((1-acetylpiperidin-4-yl)amino)methyl)-3-methoxyphenyl)-3-chloropyridin-2-yl)-2-chlorophenyl)-2-methoxypyridin-3-yl)methyl)amino)piperidin-1-yl)ethan-1-one;
2-(4-(3-chloro-2-(2-chloro-3-(6-methoxy-5-((7-oxo-2,6-diazaspiro[3.4]octan-2-yl)methyl)pyridin-2-yl)phenyl)pyridin-4-yl)-2-methoxybenzyl)-2,6-diazaspiro[3.4]octan-7-one;
N-(4-(3-chloro-2-(2-chloro-3-(6-methoxy-5-(((tetrahydro-2H-pyran-4-yl)amino)methyl)pyridin-2-yl)phenyl)pyridin-4-yl)-2-methoxybenzyl)tetrahydro-2H-pyran-4-amine;
(S)-5-(((4-(3-chloro-2-(2-chloro-3-(6-methoxy-5-(((((S)-5-oxopyrrolidin-2-yl)methyl)amino)methyl)pyridin-2-yl)phenyl)pyridin-4-yl)-2-methoxybenzyl)amino)methyl)pyrrolidin-2-one;
(S)-5-(((4-(3-chloro-2-(2-chloro-3-(6-methoxy-5-(((((R)-5-oxopyrrolidin-2-yl)methyl)amino)methyl)pyridin-2-yl)phenyl)pyridin-4-yl)-2-methoxybenzyl)amino)methyl)pyrrolidin-2-one;
(R)-5-(((4-(3-chloro-2-(2-chloro-3-(6-methoxy-5-(((((S)-5-oxopyrrolidin-2-yl)methyl)amino)methyl)pyridin-2-yl)phenyl)pyridin-4-yl)-2-methoxybenzyl)amino)methyl)pyrrolidin-2-one;
(R)-5-(((4-(3-chloro-2-(2-chloro-3-(6-methoxy-5-(((((R)-5-oxopyrrolidin-2-yl)methyl)amino)methyl)pyridin-2-yl)phenyl)pyridin-4-yl)-2-methoxybenzyl)amino)methyl)pyrrolidin-2-one;
2-(4-(3-chloro-4-(2-chloro-3-(6-methoxy-5-(((1-(3-methoxypropanoyl)piperidin-4-yl)amino)methyl)pyridin-2-yl)phenyl)pyridin-2-yl)-2-methoxybenzyl)-2,6-diazaspiro[3.4]octan-7-one;
N-((6-(2-chloro-3-(5-chloro-6-(3-methoxy-4-(((tetrahydro-2H-pyran-4-yl)amino)methyl)phenyl)pyrimidin-4-yl)phenyl)-2-methoxypyridin-3-yl)methyl)tetrahydro-2H-pyran-4-amine;
1-(4-(((6-(3-(5'-(((1-acetylpiperidin-4-yl)amino)methyl)-3-chloro-6'-methoxy-[2,2'-bipyridin]-4-yl)-2-chlorophenyl)-2-methoxypyridin-3-yl)methyl)amino)piperidin-1-yl)ethan-1-one;
2-((6-(2-chloro-3-(3-chloro-6'-methoxy-5'-((7-oxo-2,6-diazaspiro[3.4]octan-2-yl)methyl)-[2,2'-bipyridin]-4-yl)phenyl)-2-methoxypyridin-3-yl)methyl)-2,6-diazaspiro[3.4]octan-7-one;
N-((6-(2-chloro-3-(3-chloro-6'-methoxy-5'-(((tetrahydro-2H-pyran-4-yl)amino)methyl)-[2,2'-bipyridin]-4-yl)phenyl)-2-methoxypyridin-3-yl)methyl)tetrahydro-2H-pyran-4-amine;
(S)-5-((((6-(2-chloro-3-(3-chloro-6'-methoxy-5'-(((((S)-5-oxopyrrolidin-2-yl)methyl)amino)methyl)-[2,2'-bipyridin]-4-yl)phenyl)-2-methoxypyridin-3-yl)methyl)amino)methyl)pyrrolidin-2-one;
(S)-5-((((6-(2-chloro-3-(3-chloro-6'-methoxy-5'-(((((R)-5-oxopyrrolidin-2-yl)methyl)amino)methyl)-[2,2'-bipyridin]-4-yl)phenyl)-2-methoxypyridin-3-yl)methyl)amino)methyl)pyrrolidin-2-one;
(R)-5-((((6-(2-chloro-3-(3-chloro-6'-methoxy-5'-(((((S)-5-oxopyrrolidin-2-yl)methyl)amino)methyl)-[2,2'-bipyridin]-4-yl)phenyl)-2-methoxypyridin-3-yl)methyl)amino)methyl)pyrrolidin-2-one;
(R)-5-((((6-(2-chloro-3-(3-chloro-6'-methoxy-5'-(((((R)-5-oxopyrrolidin-2-yl)methyl)amino)methyl)-[2,2'-bipyridin]-4-yl)phenyl)-2-methoxypyridin-3-yl)methyl)amino)methyl)pyrrolidin-2-one;
1-(4-(((6-(3-(2-(4-((6-acetyl-2,6-diazaspiro[3.3]heptan-2-yl)methyl)-3-methoxyphenyl)-3-chloropyridin-4-yl)-2-chlorophenyl)-2-methoxypyridin-3-yl)methyl)amino)piperidin-1-yl)-3-methoxypropan-1-one;
(S)-1-(4-(((6-(2-chloro-3-(3-chloro-2-(4-(((2-hydroxypropyl)amino)methyl)-3-methoxyphenyl)pyridin-4-yl)phenyl)-2-methoxypyridin-3-yl)methyl)amino)piperidin-1-yl)-3-methoxypropan-1-one;
(R)-1-(4-(((6-(2-chloro-3-(3-chloro-2-(4-(((2-hydroxypropyl)amino)methyl)-3-methoxyphenyl)pyridin-4-yl)phenyl)-2-methoxypyridin-3-yl)methyl)amino)piperidin-1-yl)-3-methoxypropan-1-one;
(S)-5-((((2'-((S)-1-((5-(((1-acetylpiperidin-4-yl)amino)methyl)-6-methoxy-3-(trifluoromethyl)pyridin-2-yl)amino)-2,3-dihydro-1H-inden-4-yl)-3'-chloro-6-methoxy-[2,4'-bipyridin]-5-yl)methyl)amino)methyl)pyrrolidin-2-one;
(S)-5-((((2'-((R)-1-((5-(((1-acetylpiperidin-4-yl)amino)methyl)-6-methoxy-3-(trifluoromethyl)pyridin-2-yl)amino)-2,3-dihydro-1H-inden-4-yl)-3'-chloro-6-methoxy-[2,4'-bipyridin]-5-yl)methyl)amino)methyl)pyrrolidin-2-one;
(R)-5-((((2'-((S)-1-((5-(((1-acetylpiperidin-4-yl)amino)methyl)-6-methoxy-3-(trifluoromethyl)pyridin-2-yl)amino)-2,3-dihydro-1H-inden-4-yl)-3'-chloro-6-methoxy-[2,4'-bipyridin]-5-yl)methyl)amino)methyl)pyrrolidin-2-one;
(R)-5-((((2'-((R)-1-((5-(((1-acetylpiperidin-4-yl)amino)methyl)-6-methoxy-3-(trifluoromethyl)pyridin-2-yl)amino)-2,3-dihydro-1H-inden-4-yl)-3'-chloro-6-methoxy-[2,4'-bipyridin]-5-yl)methyl)amino)methyl)pyrrolidin-2-one;
(S)-5-((((3'-chloro-6-methoxy-2'-((S)-1-((6-methoxy-5-(((tetrahydro-2H-pyran-4-yl)amino)methyl)-3-(trifluoromethyl)pyridin-2-yl)amino)-2,3-dihydro-1H-inden-4-yl)-[2,4'-bipyridin]-5-yl)methyl)amino)methyl)pyrrolidin-2-one;
(S)-5-((((3'-chloro-6-methoxy-2'-((R)-1-((6-methoxy-5-(((tetrahydro-2H-pyran-4-yl)amino)methyl)-3-(trifluoromethyl)pyridin-2-yl)amino)-2,3-dihydro-1H-inden-4-yl)-[2,4'-bipyridin]-5-yl)methyl)amino)methyl)pyrrolidin-2-one;
(R)-5-((((3'-chloro-6-methoxy-2'-((S)-1-((6-methoxy-5-(((tetrahydro-2H-pyran-4-yl)amino)methyl)-3-(trifluoromethyl)pyridin-2-yl)amino)-2,3-dihydro-1H-inden-4-yl)-[2,4'-bipyridin]-5-yl)methyl)amino)methyl)pyrrolidin-2-one;
(R)-5-((((3'-chloro-6-methoxy-2'-((R)-1-((6-methoxy-5-(((tetrahydro-2H-pyran-4-yl)amino)methyl)-3-(trifluoromethyl)pyridin-2-yl)amino)-2,3-dihydro-1H-inden-4-yl)-[2,4'-bipyridin]-5-yl)methyl)amino)methyl)pyrrolidin-2-one;
N-((6-(2-chloro-3-(3-chloro-2-(3-methoxy-4-(((tetrahydro-2H-pyran-4-yl)amino)methyl)phenyl)pyridin-4-yl)phenyl)-1-methyl-1H-pyrrolo[2,3-b]pyridin-3-yl)methyl)-N-methyltetrahydro-2H-pyran-4-amine;
2-(2-(4-((6-acetyl-2,6-diazaspiro[3.3]heptan-2-yl)methyl)-3-methoxyphenyl)-3-chloropyridin-4-yl)-6-(5-((6-acetyl-2,6-diazaspiro[3.3]heptan-2-yl)methyl)-6-methoxypyridin-2-yl)benzonitrile;
1-(4-(((6-(2-chloro-3-(3-chloro-2-(2-((3-hydroxy-3-methylazetidin-1-yl)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one;
(R)-1-(4-(((6-(2-chloro-3-(3-chloro-2-(2-(((2-hydroxypropyl)amino)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one;
(S)-1-(4-(((6-(2-chloro-3-(3-chloro-2-(2-(((2-hydroxypropyl)amino)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one;
1-(4-(((6-(3-(5-(((1-acetylpiperidin-4-yl)amino)methyl)-4'-chloro-6-methoxy-[2,3'-bipyridin]-5'-yl)-2-chlorophenyl)-2-methoxypyridin-3-yl)methyl)amino)piperidin-1-yl)ethan-1-one;
2-((6-(2-chloro-3-(4'-chloro-6-methoxy-5-((7-oxo-2,6-diazaspiro[3.4]octan-2-yl)methyl)-[2,3'-bipyridin]-5'-yl)phenyl)-2-methoxypyridin-3-yl)methyl)-2,6-diazaspiro[3.4]octan-7-one;
1-(4-(((6-(3-(5-(4-(((1-acetylpiperidin-4-yl)amino)methyl)-3-methoxyphenyl)-4-chloropyridin-3-yl)-2-chlorophenyl)-2-methoxypyridin-3-yl)methyl)amino)piperidin-1-yl)ethan-1-one;
2-(4-(4-chloro-5-(2-chloro-3-(6-methoxy-5-((7-oxo-2,6-diazaspiro[3.4]octan-2-yl)methyl)pyridin-2-yl)phenyl)pyridin-3-yl)-2-methoxybenzyl)-2,6-diazaspiro[3.4]octan-7-one;
1-(6-(2-chloro-3-(5-chloro-6-(3-methoxy-4-(((oxazol-5-ylmethyl)amino)methyl)phenyl)pyrimidin-4-yl)phenyl)-2-methoxypyridin-3-yl)-N-(oxazol-5-ylmethyl)methanamine;
1-(4-(((6-(2-chloro-3-(3-chloro-2-(2-((isopropylamino)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one;
5-(((1-acetylpiperidin-4-yl)amino)methyl)-N-(3-(2-(4-(((1-acetylpiperidin-4-yl)amino)methyl)-3-methoxyphenyl)-3-chloropyridin-4-yl)-2-methylphenyl)picolinamide;
N-(3-(3-chloro-2-(3-methoxy-4-(((tetrahydro-2H-pyran-4-yl)amino)methyl)phenyl)pyridin-4-yl)-2-methylphenyl)-5-(((tetrahydro-2H-pyran-4-yl)amino)methyl)picolinamide;
N-(3-(3-chloro-2-(3-methoxy-4-(((((S)-5-oxopyrrolidin-2-yl)methyl)amino)methyl)phenyl)pyridin-4-yl)-2-methylphenyl)-5-(((((S)-5-oxopyrrolidin-2-yl)methyl)amino)methyl)picolinamide;
N-(3-(3-chloro-2-(3-methoxy-4-(((((S)-5-oxopyrrolidin-2-yl)methyl)amino)methyl)phenyl)pyridin-4-yl)-2-methylphenyl)-5-(((((R)-5-oxopyrrolidin-2-yl)methyl)amino)methyl)picolinamide;
N-(3-(3-chloro-2-(3-methoxy-4-(((((R)-5-oxopyrrolidin-2-yl)methyl)amino)methyl)phenyl)pyridin-4-yl)-2-methylphenyl)-5-(((((S)-5-oxopyrrolidin-2-yl)methyl)amino)methyl)picolinamide;
N-(3-(3-chloro-2-(3-methoxy-4-(((((R)-5-oxopyrrolidin-2-yl)methyl)amino)methyl)phenyl)pyridin-4-yl)-2-methylphenyl)-5-(((((R)-5-oxopyrrolidin-2-yl)methyl)amino)methyl)picolinamide;
N-(3-(3-chloro-2-(4-(((2-hydroxyethyl)amino)methyl)-3-methoxyphenyl)pyridin-4-yl)-2-methylphenyl)-5-(((2-hydroxyethyl)amino)methyl)picolinamide;
5-((6-acetyl-2,6-diazaspiro[3.3]heptan-2-yl)methyl)-N-(3-(2-(4-((6-acetyl-2,6-diazaspiro[3.3]heptan-2-yl)methyl)-3-methoxyphenyl)-3-chloropyridin-4-yl)-2-methylphenyl)picolinamide;
N-(3-(3-chloro-2-(4-((((S)-2-hydroxypropyl)amino)methyl)-3-methoxyphenyl)pyridin-4-yl)-2-methylphenyl)-5-((((S)-2-hydroxypropyl)amino)methyl)picolinamide;
N-(3-(3-chloro-2-(4-((((S)-2-hydroxypropyl)amino)methyl)-3-methoxyphenyl)pyridin-4-yl)-2-methylphenyl)-5-((((R)-2-hydroxypropyl)amino)methyl)picolinamide;
N-(3-(3-chloro-2-(4-((((R)-2-hydroxypropyl)amino)methyl)-3-methoxyphenyl)pyridin-4-yl)-2-methylphenyl)-5-((((S)-2-hydroxypropyl)amino)methyl)picolinamide;
N-(3-(3-chloro-2-(4-((((R)-2-hydroxypropyl)amino)methyl)-3-methoxyphenyl)pyridin-4-yl)-2-methylphenyl)-5-((((R)-2-hydroxypropyl)amino)methyl)picolinamide;
1-(6-(((6-(3-(2-(4-(((2-acetyl-2-azaspiro[3.3]heptan-6-yl)amino)methyl)-3-methoxyphenyl)-3-chloropyridin-4-yl)-2-(trifluoromethyl)phenyl)-2-methoxypyridin-3-yl)methyl)amino)-2-azaspiro[3.3]heptan-2-yl)ethan-1-one;
2-(3-chloro-2-(3-methoxy-4-(((tetrahydro-2H-pyran-4-yl)amino)methyl)phenyl)pyridin-4-yl)-6-(6-methoxy-5-(((tetrahydro-2H-pyran-4-yl)amino)methyl)pyridin-2-yl)benzonitrile;
2-(3-chloro-2-(3-methoxy-4-((7-oxo-2,6-diazaspiro[3.4]octan-2-yl)methyl)phenyl)pyridin-4-yl)-6-(6-methoxy-5-((7-oxo-2,6-diazaspiro[3.4]octan-2-yl)methyl)pyridin-2-yl)benzonitrile;
1-(4-(((6-(2-chloro-3-(3-chloro-2-(2-(((2-hydroxyethyl)amino)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one;
1-(4-((6-(3-(2-(4-((4-acetylpiperazin-1-yl)methyl)-3-methoxyphenyl)-3-chloropyridin-4-yl)-2-chlorophenyl)-2-methoxypyridin-3-yl)methyl)piperazin-1-yl)ethan-1-one;
N-(3-(2-(4-(((1-acetylpiperidin-4-yl)amino)methyl)-3-methoxyphenyl)-3-chloropyridin-4-yl)-2-chlorophenyl)-1,3-dimethyl-2,4-dioxo-1,2,3,4-tetrahydropyrimidine-5-carboxamide;
(S)-N-(2-chloro-3-(3-chloro-2-(3-methoxy-4-((((5-oxopyrrolidin-2-yl)methyl)amino)methyl)phenyl)pyridin-4-yl)phenyl)-1,3-dimethyl-2,4-dioxo-1,2,3,4-tetrahydropyrimidine-5-carboxamide;
(R)-N-(2-chloro-3-(3-chloro-2-(3-methoxy-4-((((5-oxopyrrolidin-2-yl)methyl)amino)methyl)phenyl)pyridin-4-yl)phenyl)-1,3-dimethyl-2,4-dioxo-1,2,3,4-tetrahydropyrimidine-5-carboxamide;
N-(2-chloro-3-(3-chloro-2-(3-methoxy-4-((7-oxo-2,6-diazaspiro[3.4]octan-2-yl)methyl)phenyl)pyridin-4-yl)phenyl)-1,3-dimethyl-2,4-dioxo-1,2,3,4-tetrahydropyrimidine-5-carboxamide;
2-((6-(4-(3-(5-(((1-acetylpiperidin-4-yl)amino)methyl)-6-methoxypyridin-2-yl)-2-chlorophenyl)-3-chloropyridin-2-yl)-8-methoxy-[1,2,4]triazolo[1,5-a]pyridin-2-yl)methyl)-2,6-diazaspiro[3.4]octan-7-one;
1-(4-(((6-(2-chloro-3-(3-chloro-2-(2-((6-hydroxy-2-azaspiro[3.3]heptan-2-yl)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one;
1-(4-(((6-(2-chloro-3-(3-chloro-2-(2-(((2-hydroxyethyl)amino)methyl)-4-methoxypyrrolo[2,1-f][1,2,4]triazin-6-yl)pyridin-4-yl)phenyl)-2-methoxypyridin-3-yl)methyl)amino)piperidin-1-yl)ethan-1-one;
2-((6-(3-(2-(4-((6-acetyl-2,6-diazaspiro[3.3]heptan-2-yl)methyl)-3-(difluoromethoxy)phenyl)-3-chloropyridin-4-yl)-2-chlorophenyl)-2-methoxypyridin-3-yl)methyl)-2,6-diazaspiro[3.4]octan-7-one;
1-(6-(4-(3-chloro-4-(2-chloro-3-(6-methoxy-5-(((tetrahydro-2H-pyran-4-yl)amino)methyl)pyridin-2-yl)phenyl)pyridin-2-yl)-2-(difluoromethoxy)benzyl)-2,6-diazaspiro[3.3]heptan-2-yl)ethan-1-one;
1-(4-(((6-(3-(2-(4-((6-acetyl-2,6-diazaspiro[3.3]heptan-2-yl)methyl)-3-(difluoromethoxy)phenyl)-3-chloropyridin-4-yl)-2-chlorophenyl)-2-methoxypyridin-3-yl)methyl)amino)piperidin-1-yl)ethan-1-one;
2-((6-(3-(2-(4-(((1-acetylpiperidin-4-yl)amino)methyl)-3-(difluoromethoxy)phenyl)-3-chloropyridin-4-yl)-2-chlorophenyl)-2-methoxypyridin-3-yl)methyl)-2,6-diazaspiro[3.4]octan-7-one;
(S)-5-((((6-(((S)-4-(3'-chloro-6-methoxy-5-(((tetrahydro-2H-pyran-4-yl)amino)methyl)-[2,4'-bipyridin]-2'-yl)-2,3-dihydro-1H-inden-1-yl)amino)-2-methoxy-5-(trifluoromethyl)pyridin-3-yl)methyl)amino)methyl)pyrrolidin-2-one;
(S)-5-((((6-(((R)-4-(3'-chloro-6-methoxy-5-(((tetrahydro-2H-pyran-4-yl)amino)methyl)-[2,4'-bipyridin]-2'-yl)-2,3-dihydro-1H-inden-1-yl)amino)-2-methoxy-5-(trifluoromethyl)pyridin-3-yl)methyl)amino)methyl)pyrrolidin-2-one;
(R)-5-((((6-(((S)-4-(3'-chloro-6-methoxy-5-(((tetrahydro-2H-pyran-4-yl)amino)methyl)-[2,4'-bipyridin]-2'-yl)-2,3-dihydro-1H-inden-1-yl)amino)-2-methoxy-5-(trifluoromethyl)pyridin-3-yl)methyl)amino)methyl)pyrrolidin-2-one;
(R)-5-((((6-(((R)-4-(3'-chloro-6-methoxy-5-(((tetrahydro-2H-pyran-4-yl)amino)methyl)-[2,4'-bipyridin]-2'-yl)-2,3-dihydro-1H-inden-1-yl)amino)-2-methoxy-5-(trifluoromethyl)pyridin-3-yl)methyl)amino)methyl)pyrrolidin-2-one;
1-(6-((6-(2-chloro-3-(3-chloro-2-(4-((6-(2-hydroxyacetyl)-2,6-diazaspiro[3.3]heptan-2-yl)methyl)-3-methoxyphenyl)pyridin-4-yl)phenyl)-2-methoxypyridin-3-yl)methyl)-2,6-diazaspiro[3.3]heptan-2-yl)-2-hydroxyethan-1-one;
1-(4-((4-(5-(((1-acetylpiperidin-4-yl)amino)methyl)-3',3"-dichloro-6-methoxy-[2,4':2',4"-terpyridin]-2"-yl)-2-methoxybenzyl)amino)piperidin-1-yl)ethan-1-one;
2-(4-(3',3"-dichloro-6-methoxy-5-((7-oxo-2,6-diazaspiro[3.4]octan-2-yl)methyl)-[2,4':2',4"-terpyridin]-2"-yl)-2-methoxybenzyl)-2,6-diazaspiro[3.4]octan-7-one;
2-(2-((6-(2-chloro-3-(3-chloro-2-(4-((6-(2-hydroxyethyl)-2,6-diazaspiro[3.4]octan-2-yl)methyl)-3-methoxyphenyl)pyridin-4-yl)phenyl)-2-methoxypyridin-3-yl)methyl)-2,6-diazaspiro[3.4]octan-6-yl)ethan-1-ol;
1-(2-((6-(3-(2-(4-((6-acetyl-2,6-diazaspiro[3.4]octan-2-yl)methyl)-3-methoxyphenyl)-3-chloropyridin-4-yl)-2-chlorophenyl)-2-methoxypyridin-3-yl)methyl)-2,6-diazaspiro[3.4]octan-6-yl)ethan-1-one;
N-(2-chloro-3-(3-chloro-2-(3-methoxy-4-((7-oxo-2,6-diazaspiro[3.4]octan-2-yl)methyl)phenyl)pyridin-4-yl)phenyl)-5-((7-oxo-2,6-diazaspiro[3.4]octan-2-yl)methyl)picolinamide;
5-(((1-acetylpiperidin-4-yl)amino)methyl)-N-(3-(2-(4-(((1-acetylpiperidin-4-yl)amino)methyl)-3-methoxyphenyl)-3-chloropyridin-4-yl)-2-chlorophenyl)picolinamide;
N-(2-chloro-3-(3-chloro-2-(3-methoxy-4-(((tetrahydro-2H-pyran-4-yl)amino)methyl)phenyl)pyridin-4-yl)phenyl)-5-(((tetrahydro-2H-pyran-4-yl)amino)methyl)picolinamide;
N-(2-chloro-3-(3-chloro-2-(3-methoxy-4-(((((S)-5-oxopyrrolidin-2-yl)methyl)amino)methyl)phenyl)pyridin-4-yl)phenyl)-5-(((((S)-5-oxopyrrolidin-2-yl)methyl)amino)methyl)picolinamide;
N-(2-chloro-3-(3-chloro-2-(3-methoxy-4-(((((S)-5-oxopyrrolidin-2-yl)methyl)amino)methyl)phenyl)pyridin-4-yl)phenyl)-5-(((((R)-5-oxopyrrolidin-2-yl)methyl)amino)methyl)picolinamide;
N-(2-chloro-3-(3-chloro-2-(3-methoxy-4-(((((R)-5-oxopyrrolidin-2-yl)methyl)amino)methyl)phenyl)pyridin-4-yl)phenyl)-5-(((((S)-5-oxopyrrolidin-2-yl)methyl)amino)methyl)picolinamide;
N-(2-chloro-3-(3-chloro-2-(3-methoxy-4-(((((R)-5-oxopyrrolidin-2-yl)methyl)amino)methyl)phenyl)pyridin-4-yl)phenyl)-5-(((((R)-5-oxopyrrolidin-2-yl)methyl)amino)methyl)picolinamide;
N-(2-chloro-3-(3-chloro-2-(4-(((2-hydroxyethyl)amino)methyl)-3-methoxyphenyl)pyridin-4-yl)phenyl)-5-(((2-hydroxyethyl)amino)methyl)picolinamide;
5-((6-acetyl-2,6-diazaspiro[3.3]heptan-2-yl)methyl)-N-(3-(2-(4-((6-acetyl-2,6-diazaspiro[3.3]heptan-2-yl)methyl)-3-methoxyphenyl)-3-chloropyridin-4-yl)-2-chlorophenyl)picolinamide;
N-(2-chloro-3-(3-chloro-2-(4-((((S)-2-hydroxypropyl)amino)methyl)-3-methoxyphenyl)pyridin-4-yl)phenyl)-5-((((S)-2-hydroxypropyl)amino)methyl)picolinamide;
N-(2-chloro-3-(3-chloro-2-(4-((((S)-2-hydroxypropyl)amino)methyl)-3-methoxyphenyl)pyridin-4-yl)phenyl)-5-((((R)-2-hydroxypropyl)amino)methyl)picolinamide;
N-(2-chloro-3-(3-chloro-2-(4-((((R)-2-hydroxypropyl)amino)methyl)-3-methoxyphenyl)pyridin-4-yl)phenyl)-5-((((S)-2-hydroxypropyl)amino)methyl)picolinamide;
N-(2-chloro-3-(3-chloro-2-(4-((((R)-2-hydroxypropyl)amino)methyl)-3-methoxyphenyl)pyridin-4-yl)phenyl)-5-((((R)-2-hydroxypropyl)amino)methyl)picolinamide;
1-(6-((6-(2-chloro-3-(3-chloro-2-(3-methoxy-4-((6-(3-methoxypropanoyl)-2,6-diazaspiro[3.3]heptan-2-yl)methyl)phenyl)pyridin-4-yl)phenyl)-2-methoxypyridin-3-yl)methyl)-2,6-diazaspiro[3.3]heptan-2-yl)-3-methoxypropan-1-one;
2-((2'-(3-(5-(((1-acetylpiperidin-4-yl)amino)methyl)-6-methoxypyridin-2-yl)-2-chlorophenyl)-3'-chloro-6-methoxy-[2,4'-bipyridin]-5-yl)methyl)-2,6-diazaspiro[3.4]octan-7-one;
2-((3'-chloro-2'-(2-chloro-3-(5-((4-hydroxypiperidin-1-yl)methyl)-6-methoxypyridin-2-yl)phenyl)-6-methoxy-[2,4'-bipyridin]-5-yl)methyl)-2,6-diazaspiro[3.4]octan-7-one;
2-((6-(3-(5-(((1-acetylpiperidin-4-yl)amino)methyl)-3'-chloro-6-methoxy-[2,4'-bipyridin]-2'-yl)-2-chlorophenyl)-2-methoxypyridin-3-yl)methyl)-2,6-diazaspiro[3.4]octan-7-one;
2-(4-(3-chloro-4-(2-chloro-3-(6-methoxy-5-(((tetrahydro-2H-pyran-4-yl)amino)methyl)pyridin-2-yl)phenyl)pyridin-2-yl)-2-(difluoromethoxy)benzyl)-2,6-diazaspiro[3.4]octan-7-one;
2-(4-(4-(3-(5-(((1-acetylpiperidin-4-yl)amino)methyl)-6-methoxypyridin-2-yl)-2-chlorophenyl)-3-chloropyridin-2-yl)-2-(difluoromethoxy)benzyl)-2,6-diazaspiro[3.4]octan-7-one;
2-((6-(2-chloro-3-(3-chloro-2-(3-(difluoromethoxy)-4-(((tetrahydro-2H-pyran-4-yl)amino)methyl)phenyl)pyridin-4-yl)phenyl)-2-methoxypyridin-3-yl)methyl)-2,6-diazaspiro[3.4]octan-7-one;
(S)-5-((((6-(((S)-4-(3'-chloro-6-methoxy-5-(((tetrahydro-2H-pyran-4-yl)amino)methyl)-[2,4'-bipyridin]-2'-yl)-2,3-dihydro-1H-inden-1-yl)oxy)-2-methoxy-5-(trifluoromethyl)pyridin-3-yl)methyl)amino)methyl)pyrrolidin-2-one;
(R)-5-((((6-(((S)-4-(3'-chloro-6-methoxy-5-(((tetrahydro-2H-pyran-4-yl)amino)methyl)-[2,4'-bipyridin]-2'-yl)-2,3-dihydro-1H-inden-1-yl)oxy)-2-methoxy-5-(trifluoromethyl)pyridin-3-yl)methyl)amino)methyl)pyrrolidin-2-one;
(S)-5-((((6-(((R)-4-(3'-chloro-6-methoxy-5-(((tetrahydro-2H-pyran-4-yl)amino)methyl)-[2,4'-bipyridin]-2'-yl)-2,3-dihydro-1H-inden-1-yl)oxy)-2-methoxy-5-(trifluoromethyl)pyridin-3-yl)methyl)amino)methyl)pyrrolidin-2-one;
(R)-5-((((6-(((R)-4-(3'-chloro-6-methoxy-5-(((tetrahydro-2H-pyran-4-yl)amino)methyl)-[2,4'-bipyridin]-2'-yl)-2,3-dihydro-1H-inden-1-yl)oxy)-2-methoxy-5-(trifluoromethyl)pyridin-3-yl)methyl)amino)methyl)pyrrolidin-2-one;
2-(4-(3-chloro-4-(2-chloro-3-(6-methoxy-5-((6-(2-methoxyacetyl)-2,6-diazaspiro[3.3]heptan-2-yl)methyl)pyridin-2-yl)phenyl)pyridin-2-yl)-2-methoxybenzyl)-2,6-diazaspiro[3.4]octan-7-one;
2-(4-(4-(3-(5-((6-acetyl-2,6-diazaspiro[3.3]heptan-2-yl)methyl)-6-methoxypyridin-2-yl)-2-chlorophenyl)-3-chloropyridin-2-yl)-2-(difluoromethoxy)benzyl)-2,6-diazaspiro[3.4]octan-7-one;
1-(6-((6-(2-chloro-3-(3-chloro-2-(3-(difluoromethoxy)-4-(((tetrahydro-2H-pyran-4-yl)amino)methyl)phenyl)pyridin-4-yl)phenyl)-2-methoxypyridin-3-yl)methyl)-2,6-diazaspiro[3.3]heptan-2-yl)ethan-1-one;
1-(4-((4-(4-(3-(5-((6-acetyl-2,6-diazaspiro[3.3]heptan-2-yl)methyl)-6-methoxypyridin-2-yl)-2-chlorophenyl)-3-chloropyridin-2-yl)-2-(difluoromethoxy)benzyl)amino)piperidin-1-yl)ethan-1-one;
1-(6-((6-(3-(2-(4-((6-acetyl-2,6-diazaspiro[3.3]heptan-2-yl)methyl)-3-methoxyphenyl)-3-chloropyridin-4-yl)-2-chlorophenyl)-2-methoxypyridin-3-yl)methyl)-2,6-diazaspiro[3.3]heptan-2-yl)-2-methoxyethan-1-one;
1-(2-((6-(2-chloro-3-(3-chloro-2-(4-((4-hydroxypiperidin-1-yl)methyl)-3-methoxyphenyl)pyridin-4-yl)phenyl)-2-methoxypyridin-3-yl)methyl)-2,6-diazaspiro[3.4]octan-6-yl)ethan-1-one;
1-(2-(4-(3-chloro-4-(2-chloro-3-(5-((4-hydroxypiperidin-1-yl)methyl)-6-methoxypyridin-2-yl)phenyl)pyridin-2-yl)-2-methoxybenzyl)-2,6-diazaspiro[3.4]octan-6-yl)ethan-1-one;
N-(2-chloro-3-(3-chloro-2-(3-methoxy-4-((methylamino)methyl)phenyl)pyridin-4-yl)phenyl)-5-((methylamino)methyl)picolinamide;
5-(((2-oxaspiro[3.3]heptan-6-yl)amino)methyl)-N-(3-(2-(4-(((2-oxaspiro[3.3]heptan-6-yl)amino)methyl)-3-methoxyphenyl)-3-chloropyridin-4-yl)-2-chlorophenyl)picolinamide;
N-(3-(3-chloro-2-(3-methoxy-4-((methylamino)methyl)phenyl)pyridin-4-yl)-2-methylphenyl)-5-((methylamino)methyl)picolinamide;
(S)-5-((((6-(2-chloro-3-(3-chloro-2-(2-((3-hydroxy-3-methylazetidin-1-yl)methyl)-4-methoxypyrrolo[2,1-f][1,2,4]triazin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-((3-hydroxy-3-methylazetidin-1-yl)methyl)-4-methoxypyrrolo[2,1-f][1,2,4]triazin-6-yl)pyridin-4-yl)phenyl)-2-methoxypyridin-3-yl)methyl)amino)methyl)pyrrolidin-2-one;
1-(4-(((6-(3-(2-(2-((2-oxa-6-azaspiro[3.3]heptan-6-yl)methyl)-8-methoxy-[1,2,4]triazolo[1,5-a]pyridin-6-yl)-3-chloropyridin-4-yl)-2-chlorophenyl)-2-methoxypyridin-3-yl)methyl)amino)piperidin-1-yl)ethan-1-one;
1-(4-(((6-(2-chloro-3-(3-chloro-2-(2-((((1-hydroxycyclopropyl)methyl)amino)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one;
N-(3-(2-(4-(((1-acetylpiperidin-4-yl)amino)methyl)-3-methoxyphenyl)-3-chloropyridin-4-yl)-2-methylphenyl)-5-(((2-hydroxyethyl)amino)methyl)picolinamide;
5-((2-oxa-6-azaspiro[3.3]heptan-6-yl)methyl)-N-(3-(2-(4-((2-oxa-6-azaspiro[3.3]heptan-6-yl)methyl)-3-methoxyphenyl)-3-chloropyridin-4-yl)-2-chlorophenyl)picolinamide;
N-(2-chloro-3-(3-chloro-2-(4-(((3-fluoropropyl)amino)methyl)-3-methoxyphenyl)pyridin-4-yl)phenyl)-5-(((3-fluoropropyl)amino)methyl)picolinamide;
N-(3-(3-chloro-2-(3-methoxy-4-(((tetrahydro-2H-pyran-4-yl)amino)methyl)phenyl)pyridin-4-yl)-2-methylphenyl)-5-(((2-hydroxyethyl)amino)methyl)picolinamide;
1-(((6-(((S)-5-(3'-chloro-6-methoxy-5-(((((S)-5-oxopyrrolidin-2-yl)methyl)amino)methyl)-[2,4'-bipyridin]-2'-yl)-1,2,3,4-tetrahydronaphthalen-1-yl)amino)-2-methoxy-5-(trifluoromethyl)pyridin-3-yl)methyl)amino)cyclopropane-1-carboxylic acid;
1-(((6-(((R)-5-(3'-chloro-6-methoxy-5-(((((S)-5-oxopyrrolidin-2-yl)methyl)amino)methyl)-[2,4'-bipyridin]-2'-yl)-1,2,3,4-tetrahydronaphthalen-1-yl)amino)-2-methoxy-5-(trifluoromethyl)pyridin-3-yl)methyl)amino)cyclopropane-1-carboxylic acid;
1-(((6-(((S)-5-(3'-chloro-6-methoxy-5-(((((R)-5-oxopyrrolidin-2-yl)methyl)amino)methyl)-[2,4'-bipyridin]-2'-yl)-1,2,3,4-tetrahydronaphthalen-1-yl)amino)-2-methoxy-5-(trifluoromethyl)pyridin-3-yl)methyl)amino)cyclopropane-1-carboxylic acid;
1-(((6-(((R)-5-(3'-chloro-6-methoxy-5-(((((R)-5-oxopyrrolidin-2-yl)methyl)amino)methyl)-[2,4'-bipyridin]-2'-yl)-1,2,3,4-tetrahydronaphthalen-1-yl)amino)-2-methoxy-5-(trifluoromethyl)pyridin-3-yl)methyl)amino)cyclopropane-1-carboxylic acid;
1-(((6-(((S)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)cyclopropane-1-carboxylic acid;
1-(((6-(((R)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)cyclopropane-1-carboxylic acid;
1-(((6-(((S)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)cyclopropane-1-carboxylic acid;
1-(((6-(((R)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)cyclopropane-1-carboxylic acid;
5-(((2-acetyl-2-azaspiro[3.3]heptan-6-yl)amino)methyl)-N-(3-(2-(4-(((2-acetyl-2-azaspiro[3.3]heptan-6-yl)amino)methyl)-3-methoxyphenyl)-3-chloropyridin-4-yl)-2-chlorophenyl)picolinamide;
(S)-5-((((3'-chloro-2'-((S)-5-((5-((((S)-2-hydroxypropyl)amino)methyl)-6-methoxy-3-(trifluoromethyl)pyridin-2-yl)amino)-5,6,7,8-tetrahydronaphthalen-1-yl)-6-methoxy-[2,4'-bipyridin]-5-yl)methyl)amino)methyl)pyrrolidin-2-one;
(S)-5-((((3'-chloro-2'-((R)-5-((5-((((S)-2-hydroxypropyl)amino)methyl)-6-methoxy-3-(trifluoromethyl)pyridin-2-yl)amino)-5,6,7,8-tetrahydronaphthalen-1-yl)-6-methoxy-[2,4'-bipyridin]-5-yl)methyl)amino)methyl)pyrrolidin-2-one;
(S)-5-((((3'-chloro-2'-((S)-5-((5-((((R)-2-hydroxypropyl)amino)methyl)-6-methoxy-3-(trifluoromethyl)pyridin-2-yl)amino)-5,6,7,8-tetrahydronaphthalen-1-yl)-6-methoxy-[2,4'-bipyridin]-5-yl)methyl)amino)methyl)pyrrolidin-2-one;
(S)-5-((((3'-chloro-2'-((R)-5-((5-((((R)-2-hydroxypropyl)amino)methyl)-6-methoxy-3-(trifluoromethyl)pyridin-2-yl)amino)-5,6,7,8-tetrahydronaphthalen-1-yl)-6-methoxy-[2,4'-bipyridin]-5-yl)methyl)amino)methyl)pyrrolidin-2-one;
(R)-5-((((3'-chloro-2'-((S)-5-((5-((((S)-2-hydroxypropyl)amino)methyl)-6-methoxy-3-(trifluoromethyl)pyridin-2-yl)amino)-5,6,7,8-tetrahydronaphthalen-1-yl)-6-methoxy-[2,4'-bipyridin]-5-yl)methyl)amino)methyl)pyrrolidin-2-one;
(R)-5-((((3'-chloro-2'-((R)-5-((5-((((S)-2-hydroxypropyl)amino)methyl)-6-methoxy-3-(trifluoromethyl)pyridin-2-yl)amino)-5,6,7,8-tetrahydronaphthalen-1-yl)-6-methoxy-[2,4'-bipyridin]-5-yl)methyl)amino)methyl)pyrrolidin-2-one;
(R)-5-((((3'-chloro-2'-((S)-5-((5-((((R)-2-hydroxypropyl)amino)methyl)-6-methoxy-3-(trifluoromethyl)pyridin-2-yl)amino)-5,6,7,8-tetrahydronaphthalen-1-yl)-6-methoxy-[2,4'-bipyridin]-5-yl)methyl)amino)methyl)pyrrolidin-2-one;
(R)-5-((((3'-chloro-2'-((R)-5-((5-((((R)-2-hydroxypropyl)amino)methyl)-6-methoxy-3-(trifluoromethyl)pyridin-2-yl)amino)-5,6,7,8-tetrahydronaphthalen-1-yl)-6-methoxy-[2,4'-bipyridin]-5-yl)methyl)amino)methyl)pyrrolidin-2-one;
(S)-N-(2-chloro-3-(5-chloro-6-(3-methoxy-4-((((5-oxopyrrolidin-2-yl)methyl)amino)methyl)phenyl)pyrimidin-4-yl)phenyl)-1,3-dimethyl-2,4-dioxo-1,2,3,4-tetrahydropyrimidine-5-carboxamide;
(R)-N-(2-chloro-3-(5-chloro-6-(3-methoxy-4-((((5-oxopyrrolidin-2-yl)methyl)amino)methyl)phenyl)pyrimidin-4-yl)phenyl)-1,3-dimethyl-2,4-dioxo-1,2,3,4-tetrahydropyrimidine-5-carboxamide;
(S)-5-((((3'-chloro-6-methoxy-2'-((S)-5-((6-methoxy-5-(((((S)-5-oxopyrrolidin-2-yl)methyl)amino)methyl)-3-(trifluoromethyl)pyridin-2-yl)amino)-5,6,7,8-tetrahydronaphthalen-1-yl)-[2,4'-bipyridin]-5-yl)methyl)amino)methyl)pyrrolidin-2-one;
(S)-5-((((3'-chloro-6-methoxy-2'-((R)-5-((6-methoxy-5-(((((S)-5-oxopyrrolidin-2-yl)methyl)amino)methyl)-3-(trifluoromethyl)pyridin-2-yl)amino)-5,6,7,8-tetrahydronaphthalen-1-yl)-[2,4'-bipyridin]-5-yl)methyl)amino)methyl)pyrrolidin-2-one;
(S)-5-((((3'-chloro-6-methoxy-2'-((S)-5-((6-methoxy-5-(((((R)-5-oxopyrrolidin-2-yl)methyl)amino)methyl)-3-(trifluoromethyl)pyridin-2-yl)amino)-5,6,7,8-tetrahydronaphthalen-1-yl)-[2,4'-bipyridin]-5-yl)methyl)amino)methyl)pyrrolidin-2-one;
(S)-5-((((3'-chloro-6-methoxy-2'-((R)-5-((6-methoxy-5-(((((R)-5-oxopyrrolidin-2-yl)methyl)amino)methyl)-3-(trifluoromethyl)pyridin-2-yl)amino)-5,6,7,8-tetrahydronaphthalen-1-yl)-[2,4'-bipyridin]-5-yl)methyl)amino)methyl)pyrrolidin-2-one;
(R)-5-((((3'-chloro-6-methoxy-2'-((S)-5-((6-methoxy-5-(((((S)-5-oxopyrrolidin-2-yl)methyl)amino)methyl)-3-(trifluoromethyl)pyridin-2-yl)amino)-5,6,7,8-tetrahydronaphthalen-1-yl)-[2,4'-bipyridin]-5-yl)methyl)amino)methyl)pyrrolidin-2-one;
(R)-5-((((3'-chloro-6-methoxy-2'-((R)-5-((6-methoxy-5-(((((S)-5-oxopyrrolidin-2-yl)methyl)amino)methyl)-3-(trifluoromethyl)pyridin-2-yl)amino)-5,6,7,8-tetrahydronaphthalen-1-yl)-[2,4'-bipyridin]-5-yl)methyl)amino)methyl)pyrrolidin-2-one;
(R)-5-((((3'-chloro-6-methoxy-2'-((S)-5-((6-methoxy-5-(((((R)-5-oxopyrrolidin-2-yl)methyl)amino)methyl)-3-(trifluoromethyl)pyridin-2-yl)amino)-5,6,7,8-tetrahydronaphthalen-1-yl)-[2,4'-bipyridin]-5-yl)methyl)amino)methyl)pyrrolidin-2-one;
(R)-5-((((3'-chloro-6-methoxy-2'-((R)-5-((6-methoxy-5-(((((R)-5-oxopyrrolidin-2-yl)methyl)amino)methyl)-3-(trifluoromethyl)pyridin-2-yl)amino)-5,6,7,8-tetrahydronaphthalen-1-yl)-[2,4'-bipyridin]-5-yl)methyl)amino)methyl)pyrrolidin-2-one;
(S)-5-((((6-(((S)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)methyl)pyrrolidin-2-one;
(S)-5-((((6-(((R)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)methyl)pyrrolidin-2-one;
(S)-5-((((6-(((S)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)methyl)pyrrolidin-2-one;
(S)-5-((((6-(((R)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)methyl)pyrrolidin-2-one;
(R)-5-((((6-(((S)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)methyl)pyrrolidin-2-one;
(R)-5-((((6-(((R)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)methyl)pyrrolidin-2-one;
(R)-5-((((6-(((S)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)methyl)pyrrolidin-2-one;
(R)-5-((((6-(((R)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)methyl)pyrrolidin-2-one;
2-(4-(5-chloro-6-(2-chloro-3-(5-(((2-hydroxyethyl)amino)methyl)-6-methoxypyridin-2-yl)phenyl)pyrimidin-4-yl)-2-methoxybenzyl)-2,6-diazaspiro[3.4]octan-7-one;
(S)-N-(2-chloro-3-(4'-chloro-6-methoxy-5-((((5-oxopyrrolidin-2-yl)methyl)amino)methyl)-[2,3'-bipyridin]-5'-yl)phenyl)-1,3-dimethyl-2,4-dioxo-1,2,3,4-tetrahydropyrimidine-5-carboxamide;
(R)-N-(2-chloro-3-(4'-chloro-6-methoxy-5-((((5-oxopyrrolidin-2-yl)methyl)amino)methyl)-[2,3'-bipyridin]-5'-yl)phenyl)-1,3-dimethyl-2,4-dioxo-1,2,3,4-tetrahydropyrimidine-5-carboxamide;
1-(4-(((6-(2-chloro-3-(3-chloro-2-(2-(((3-fluoropropyl)amino)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one;
1-(4-(((6-(2-chloro-3-(3-chloro-2-(2-(((2-hydroxy-2-methylpropyl)amino)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one;
1-(4-(((6-(2-chloro-3-(3-chloro-2-(2-(((2-hydroxyethyl)(methyl)amino)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one;
(S)-5-((((6-(2-chloro-3-(5-methoxy-6-(3-methoxy-4-(((((S)-5-oxopyrrolidin-2-yl)methyl)amino)methyl)phenyl)pyrimidin-4-yl)phenyl)-2-methoxypyridin-3-yl)methyl)amino)methyl)pyrrolidin-2-one;
(S)-5-((((6-(2-chloro-3-(5-methoxy-6-(3-methoxy-4-(((((R)-5-oxopyrrolidin-2-yl)methyl)amino)methyl)phenyl)pyrimidin-4-yl)phenyl)-2-methoxypyridin-3-yl)methyl)amino)methyl)pyrrolidin-2-one;
(R)-5-((((6-(2-chloro-3-(5-methoxy-6-(3-methoxy-4-(((((S)-5-oxopyrrolidin-2-yl)methyl)amino)methyl)phenyl)pyrimidin-4-yl)phenyl)-2-methoxypyridin-3-yl)methyl)amino)methyl)pyrrolidin-2-one;
(R)-5-((((6-(2-chloro-3-(5-methoxy-6-(3-methoxy-4-(((((R)-5-oxopyrrolidin-2-yl)methyl)amino)methyl)phenyl)pyrimidin-4-yl)phenyl)-2-methoxypyridin-3-yl)methyl)amino)methyl)pyrrolidin-2-one;
1-(4-(((6-(3-(6-(4-(((1-acetylpiperidin-4-yl)amino)methyl)-3-methoxyphenyl)-5-methoxypyrimidin-4-yl)-2-chlorophenyl)-2-methoxypyridin-3-yl)methyl)amino)piperidin-1-yl)ethan-1-one;
(S)-N-(2-chloro-3-(3'-chloro-6-methoxy-5-((((5-oxopyrrolidin-2-yl)methyl)amino)methyl)-[2,4'-bipyridin]-2'-yl)phenyl)-1,3-dimethyl-2,4-dioxo-1,2,3,4-tetrahydropyrimidine-5-carboxamide;
(R)-N-(2-chloro-3-(3'-chloro-6-methoxy-5-((((5-oxopyrrolidin-2-yl)methyl)amino)methyl)-[2,4'-bipyridin]-2'-yl)phenyl)-1,3-dimethyl-2,4-dioxo-1,2,3,4-tetrahydropyrimidine-5-carboxamide;
N-(2-chloro-3-(3'-chloro-6-methoxy-5-((7-oxo-2,6-diazaspiro[3.4]octan-2-yl)methyl)-[2,4'-bipyridin]-2'-yl)phenyl)-1,3-dimethyl-2,4-dioxo-1,2,3,4-tetrahydropyrimidine-5-carboxamide;
2-((6-(((S)-5-(3'-chloro-6-methoxy-5-(((((S)-5-oxopyrrolidin-2-yl)methyl)amino)methyl)-[2,4'-bipyridin]-2'-yl)-1,2,3,4-tetrahydronaphthalen-1-yl)amino)-2-methoxy-5-(trifluoromethyl)pyridin-3-yl)methyl)-2,6-diazaspiro[3.4]octan-7-one;
2-((6-(((R)-5-(3'-chloro-6-methoxy-5-(((((S)-5-oxopyrrolidin-2-yl)methyl)amino)methyl)-[2,4'-bipyridin]-2'-yl)-1,2,3,4-tetrahydronaphthalen-1-yl)amino)-2-methoxy-5-(trifluoromethyl)pyridin-3-yl)methyl)-2,6-diazaspiro[3.4]octan-7-one;
2-((6-(((S)-5-(3'-chloro-6-methoxy-5-(((((R)-5-oxopyrrolidin-2-yl)methyl)amino)methyl)-[2,4'-bipyridin]-2'-yl)-1,2,3,4-tetrahydronaphthalen-1-yl)amino)-2-methoxy-5-(trifluoromethyl)pyridin-3-yl)methyl)-2,6-diazaspiro[3.4]octan-7-one;
2-((6-(((R)-5-(3'-chloro-6-methoxy-5-(((((R)-5-oxopyrrolidin-2-yl)methyl)amino)methyl)-[2,4'-bipyridin]-2'-yl)-1,2,3,4-tetrahydronaphthalen-1-yl)amino)-2-methoxy-5-(trifluoromethyl)pyridin-3-yl)methyl)-2,6-diazaspiro[3.4]octan-7-one;
N-(3-(5-(((1-acetylpiperidin-4-yl)amino)methyl)-3'-chloro-6-methoxy-[2,4'-bipyridin]-2'-yl)-2-chlorophenyl)-1,3-dimethyl-2,4-dioxo-1,2,3,4-tetrahydropyrimidine-5-carboxamide;
N-(2-chloro-3-(4'-chloro-6-methoxy-5-((7-oxo-2,6-diazaspiro[3.4]octan-2-yl)methyl)-[2,3'-bipyridin]-5'-yl)phenyl)-1,3-dimethyl-2,4-dioxo-1,2,3,4-tetrahydropyrimidine-5-carboxamide;
N-(3-(5-(((1-acetylpiperidin-4-yl)amino)methyl)-4'-chloro-6-methoxy-[2,3'-bipyridin]-5'-yl)-2-chlorophenyl)-1,3-dimethyl-2,4-dioxo-1,2,3,4-tetrahydropyrimidine-5-carboxamide;
2-((6-(((S)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)-2,6-diazaspiro[3.4]octan-7-one;
2-((6-(((R)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)-2,6-diazaspiro[3.4]octan-7-one;
2-((6-(((S)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)-2,6-diazaspiro[3.4]octan-7-one;
2-((6-(((R)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)-2,6-diazaspiro[3.4]octan-7-one;
N-(3-(3-chloro-2-(3-(difluoromethoxy)-4-(((((S)-5-oxopyrrolidin-2-yl)methyl)amino)methyl)phenyl)pyridin-4-yl)-2-methylphenyl)-5-(((((S)-5-oxopyrrolidin-2-yl)methyl)amino)methyl)picolinamide;
N-(3-(3-chloro-2-(3-(difluoromethoxy)-4-(((((R)-5-oxopyrrolidin-2-yl)methyl)amino)methyl)phenyl)pyridin-4-yl)-2-methylphenyl)-5-(((((S)-5-oxopyrrolidin-2-yl)methyl)amino)methyl)picolinamide;
N-(3-(3-chloro-2-(3-(difluoromethoxy)-4-(((((S)-5-oxopyrrolidin-2-yl)methyl)amino)methyl)phenyl)pyridin-4-yl)-2-methylphenyl)-5-(((((R)-5-oxopyrrolidin-2-yl)methyl)amino)methyl)picolinamide;
N-(3-(3-chloro-2-(3-(difluoromethoxy)-4-(((((R)-5-oxopyrrolidin-2-yl)methyl)amino)methyl)phenyl)pyridin-4-yl)-2-methylphenyl)-5-(((((R)-5-oxopyrrolidin-2-yl)methyl)amino)methyl)picolinamide;
N-(3-(3-chloro-2-(3-fluoro-5-methoxy-4-(((((S)-5-oxopyrrolidin-2-yl)methyl)amino)methyl)phenyl)pyridin-4-yl)-2-methylphenyl)-5-(((((S)-5-oxopyrrolidin-2-yl)methyl)amino)methyl)picolinamide;
N-(3-(3-chloro-2-(3-fluoro-5-methoxy-4-(((((R)-5-oxopyrrolidin-2-yl)methyl)amino)methyl)phenyl)pyridin-4-yl)-2-methylphenyl)-5-(((((S)-5-oxopyrrolidin-2-yl)methyl)amino)methyl)picolinamide;
N-(3-(3-chloro-2-(3-fluoro-5-methoxy-4-(((((S)-5-oxopyrrolidin-2-yl)methyl)amino)methyl)phenyl)pyridin-4-yl)-2-methylphenyl)-5-(((((R)-5-oxopyrrolidin-2-yl)methyl)amino)methyl)picolinamide;
N-(3-(3-chloro-2-(3-fluoro-5-methoxy-4-(((((R)-5-oxopyrrolidin-2-yl)methyl)amino)methyl)phenyl)pyridin-4-yl)-2-methylphenyl)-5-(((((R)-5-oxopyrrolidin-2-yl)methyl)amino)methyl)picolinamide;
2-(4-(6-(2-chloro-3-(6-methoxy-5-((7-oxo-2,6-diazaspiro[3.4]octan-2-yl)methyl)pyridin-2-yl)phenyl)-5-methoxypyrimidin-4-yl)-2-methoxybenzyl)-2,6-diazaspiro[3.4]octan-7-one;
2-((4-(6-(2-chloro-3-(5-(((2-hydroxyethyl)amino)methyl)-6-methoxypyridin-2-yl)phenyl)-5-methoxypyrimidin-4-yl)-2-methoxybenzyl)amino)ethan-1-ol;
(S)-N-(2-chloro-3-(4'-chloro-5-(((2-hydroxypropyl)amino)methyl)-6-methoxy-[2,3'-bipyridin]-5'-yl)phenyl)-1,3-dimethyl-2,4-dioxo-1,2,3,4-tetrahydropyrimidine-5-carboxamide;
(R)-N-(2-chloro-3-(4'-chloro-5-(((2-hydroxypropyl)amino)methyl)-6-methoxy-[2,3'-bipyridin]-5'-yl)phenyl)-1,3-dimethyl-2,4-dioxo-1,2,3,4-tetrahydropyrimidine-5-carboxamide;
N-(3-(5-((6-acetyl-2,6-diazaspiro[3.3]heptan-2-yl)methyl)-4'-chloro-6-methoxy-[2,3'-bipyridin]-5'-yl)-2-chlorophenyl)-1,3-dimethyl-2,4-dioxo-1,2,3,4-tetrahydropyrimidine-5-carboxamide;
N-(3-(5-((6-acetyl-2,6-diazaspiro[3.3]heptan-2-yl)methyl)-3'-chloro-6-methoxy-[2,4'-bipyridin]-2'-yl)-2-chlorophenyl)-1,3-dimethyl-2,4-dioxo-1,2,3,4-tetrahydropyrimidine-5-carboxamide;
(S)-N-(5-(3'-chloro-6-methoxy-5-((methylamino)methyl)-[2,4'-bipyridin]-2'-yl)-1,2,3,4-tetrahydronaphthalen-1-yl)-6-methoxy-5-((methylamino)methyl)-3-(trifluoromethyl)pyridin-2-amine;
(R)-N-(5-(3'-chloro-6-methoxy-5-((methylamino)methyl)-[2,4'-bipyridin]-2'-yl)-1,2,3,4-tetrahydronaphthalen-1-yl)-6-methoxy-5-((methylamino)methyl)-3-(trifluoromethyl)pyridin-2-amine;
N-(3-(2-(4-(((1-acetylpiperidin-4-yl)amino)methyl)-3-methoxyphenyl)-3-chloropyridin-4-yl)-2-chlorophenyl)-5-(((2-hydroxyethyl)amino)methyl)picolinamide;
N-((6-(2-chloro-3-(5-methoxy-6-(3-methoxy-4-(((tetrahydro-2H-pyran-4-yl)amino)methyl)phenyl)pyrimidin-4-yl)phenyl)-2-methoxypyridin-3-yl)methyl)tetrahydro-2H-pyran-4-amine;
(S)-N-(3-(3-chloro-2-(3-methoxy-4-((((5-oxopyrrolidin-2-yl)methyl)amino)methyl)phenyl)pyridin-4-yl)-2-methylphenyl)-5-(((2-hydroxyethyl)amino)methyl)picolinamide;
(R)-N-(3-(3-chloro-2-(3-methoxy-4-((((5-oxopyrrolidin-2-yl)methyl)amino)methyl)phenyl)pyridin-4-yl)-2-methylphenyl)-5-(((2-hydroxyethyl)amino)methyl)picolinamide;
(S)-5-(((4-(3-chloro-4-((S)-5-((5-((((S)-2-hydroxypropyl)amino)methyl)-6-methoxy-3-(trifluoromethyl)pyridin-2-yl)amino)-5,6,7,8-tetrahydronaphthalen-1-yl)pyridin-2-yl)-2-methoxybenzyl)amino)methyl)pyrrolidin-2-one;
(S)-5-(((4-(3-chloro-4-((R)-5-((5-((((S)-2-hydroxypropyl)amino)methyl)-6-methoxy-3-(trifluoromethyl)pyridin-2-yl)amino)-5,6,7,8-tetrahydronaphthalen-1-yl)pyridin-2-yl)-2-methoxybenzyl)amino)methyl)pyrrolidin-2-one;
(S)-5-(((4-(3-chloro-4-((S)-5-((5-((((R)-2-hydroxypropyl)amino)methyl)-6-methoxy-3-(trifluoromethyl)pyridin-2-yl)amino)-5,6,7,8-tetrahydronaphthalen-1-yl)pyridin-2-yl)-2-methoxybenzyl)amino)methyl)pyrrolidin-2-one;
(S)-5-(((4-(3-chloro-4-((R)-5-((5-((((R)-2-hydroxypropyl)amino)methyl)-6-methoxy-3-(trifluoromethyl)pyridin-2-yl)amino)-5,6,7,8-tetrahydronaphthalen-1-yl)pyridin-2-yl)-2-methoxybenzyl)amino)methyl)pyrrolidin-2-one;
(R)-5-(((4-(3-chloro-4-((S)-5-((5-((((S)-2-hydroxypropyl)amino)methyl)-6-methoxy-3-(trifluoromethyl)pyridin-2-yl)amino)-5,6,7,8-tetrahydronaphthalen-1-yl)pyridin-2-yl)-2-methoxybenzyl)amino)methyl)pyrrolidin-2-one;
(R)-5-(((4-(3-chloro-4-((R)-5-((5-((((S)-2-hydroxypropyl)amino)methyl)-6-methoxy-3-(trifluoromethyl)pyridin-2-yl)amino)-5,6,7,8-tetrahydronaphthalen-1-yl)pyridin-2-yl)-2-methoxybenzyl)amino)methyl)pyrrolidin-2-one;
(R)-5-(((4-(3-chloro-4-((S)-5-((5-((((R)-2-hydroxypropyl)amino)methyl)-6-methoxy-3-(trifluoromethyl)pyridin-2-yl)amino)-5,6,7,8-tetrahydronaphthalen-1-yl)pyridin-2-yl)-2-methoxybenzyl)amino)methyl)pyrrolidin-2-one;
(R)-5-(((4-(3-chloro-4-((R)-5-((5-((((R)-2-hydroxypropyl)amino)methyl)-6-methoxy-3-(trifluoromethyl)pyridin-2-yl)amino)-5,6,7,8-tetrahydronaphthalen-1-yl)pyridin-2-yl)-2-methoxybenzyl)amino)methyl)pyrrolidin-2-one;
2-(4-(5-chloro-6-(2-chloro-3-(5-((((1s,3s)-3-hydroxycyclobutyl)amino)methyl)-6-methoxypyridin-2-yl)phenyl)pyrimidin-4-yl)-2-methoxybenzyl)-2,6-diazaspiro[3.4]octan-7-one;
2-(4-(5-chloro-6-(2-chloro-3-(5-((((1s,3r)-3-hydroxycyclobutyl)amino)methyl)-6-methoxypyridin-2-yl)phenyl)pyrimidin-4-yl)-2-methoxybenzyl)-2,6-diazaspiro[3.4]octan-7-one;
2-(4-(5-chloro-6-(2-chloro-3-(5-((((1r,3s)-3-hydroxycyclobutyl)amino)methyl)-6-methoxypyridin-2-yl)phenyl)pyrimidin-4-yl)-2-methoxybenzyl)-2,6-diazaspiro[3.4]octan-7-one;
2-(4-(5-chloro-6-(2-chloro-3-(5-((((1r,3r)-3-hydroxycyclobutyl)amino)methyl)-6-methoxypyridin-2-yl)phenyl)pyrimidin-4-yl)-2-methoxybenzyl)-2,6-diazaspiro[3.4]octan-7-one;
(S)-N-(2-chloro-3-(3-chloro-6'-methoxy-5'-((((5-oxopyrrolidin-2-yl)methyl)amino)methyl)-[2,2'-bipyridin]-4-yl)phenyl)-1,3-dimethyl-2,4-dioxo-1,2,3,4-tetrahydropyrimidine-5-carboxamide;
(R)-N-(2-chloro-3-(3-chloro-6'-methoxy-5'-((((5-oxopyrrolidin-2-yl)methyl)amino)methyl)-[2,2'-bipyridin]-4-yl)phenyl)-1,3-dimethyl-2,4-dioxo-1,2,3,4-tetrahydropyrimidine-5-carboxamide;
(S)-N-(2-chloro-3-(3-chloro-5'-(((2-hydroxypropyl)amino)methyl)-6'-methoxy-[2,2'-bipyridin]-4-yl)phenyl)-1,3-dimethyl-2,4-dioxo-1,2,3,4-tetrahydropyrimidine-5-carboxamide;
(R)-N-(2-chloro-3-(3-chloro-5'-(((2-hydroxypropyl)amino)methyl)-6'-methoxy-[2,2'-bipyridin]-4-yl)phenyl)-1,3-dimethyl-2,4-dioxo-1,2,3,4-tetrahydropyrimidine-5-carboxamide;
(S)-N-(5-(3-chloro-2-(3-methoxy-4-((methylamino)methyl)phenyl)pyridin-4-yl)-1,2,3,4-tetrahydronaphthalen-1-yl)-6-methoxy-5-((methylamino)methyl)-3-(trifluoromethyl)pyridin-2-amine;
(R)-N-(5-(3-chloro-2-(3-methoxy-4-((methylamino)methyl)phenyl)pyridin-4-yl)-1,2,3,4-tetrahydronaphthalen-1-yl)-6-methoxy-5-((methylamino)methyl)-3-(trifluoromethyl)pyridin-2-amine;
(S)-2-(4-(5-chloro-6-(2-chloro-3-(6-methoxy-5-((((5-oxopyrrolidin-2-yl)methyl)amino)methyl)pyridin-2-yl)phenyl)pyrimidin-4-yl)-2-methoxybenzyl)-2,6-diazaspiro[3.4]octan-7-one;
(R)-2-(4-(5-chloro-6-(2-chloro-3-(6-methoxy-5-((((5-oxopyrrolidin-2-yl)methyl)amino)methyl)pyridin-2-yl)phenyl)pyrimidin-4-yl)-2-methoxybenzyl)-2,6-diazaspiro[3.4]octan-7-one;
2-(4-(6-(2-chloro-3-(6-methoxy-5-((6-oxo-2,5-diazaspiro[3.4]octan-2-yl)methyl)pyridin-2-yl)phenyl)-5-methoxypyrimidin-4-yl)-2-methoxybenzyl)-2,5-diazaspiro[3.4]octan-6-one;
N-(3-(3-chloro-2-(3-methoxy-4-((7-oxo-2,6-diazaspiro[3.4]octan-2-yl)methyl)phenyl)pyridin-4-yl)-2-methylphenyl)-5-(((2-hydroxyethyl)amino)methyl)picolinamide;
(S)-N-(2-chloro-3-(3'-chloro-5-(((2-hydroxypropyl)amino)methyl)-6-methoxy-[2,4'-bipyridin]-2'-yl)phenyl)-1,3-dimethyl-2,4-dioxo-1,2,3,4-tetrahydropyrimidine-5-carboxamide;
(R)-N-(2-chloro-3-(3'-chloro-5-(((2-hydroxypropyl)amino)methyl)-6-methoxy-[2,4'-bipyridin]-2'-yl)phenyl)-1,3-dimethyl-2,4-dioxo-1,2,3,4-tetrahydropyrimidine-5-carboxamide;
(S)-N-(2-chloro-3-(5-chloro-6-(4-(((2-hydroxypropyl)amino)methyl)-3-methoxyphenyl)pyrimidin-4-yl)phenyl)-1,3-dimethyl-2,4-dioxo-1,2,3,4-tetrahydropyrimidine-5-carboxamide;
(R)-N-(2-chloro-3-(5-chloro-6-(4-(((2-hydroxypropyl)amino)methyl)-3-methoxyphenyl)pyrimidin-4-yl)phenyl)-1,3-dimethyl-2,4-dioxo-1,2,3,4-tetrahydropyrimidine-5-carboxamide;
(S)-N-(3-(2-(4-(((1-acetylpiperidin-4-yl)amino)methyl)-3-methoxyphenyl)-3-chloropyridin-4-yl)-2-methylphenyl)-5-((((5-oxopyrrolidin-2-yl)methyl)amino)methyl)picolinamide;
(R)-N-(3-(2-(4-(((1-acetylpiperidin-4-yl)amino)methyl)-3-methoxyphenyl)-3-chloropyridin-4-yl)-2-methylphenyl)-5-((((5-oxopyrrolidin-2-yl)methyl)amino)methyl)picolinamide;
(S)-N-(3-(3-chloro-2-(3-methoxy-4-(((tetrahydro-2H-pyran-4-yl)amino)methyl)phenyl)pyridin-4-yl)-2-methylphenyl)-5-((((5-oxopyrrolidin-2-yl)methyl)amino)methyl)picolinamide;
(R)-N-(3-(3-chloro-2-(3-methoxy-4-(((tetrahydro-2H-pyran-4-yl)amino)methyl)phenyl)pyridin-4-yl)-2-methylphenyl)-5-((((5-oxopyrrolidin-2-yl)methyl)amino)methyl)picolinamide;
(S)-N-(3-(3-chloro-2-(4-(((3-fluoropropyl)amino)methyl)-3-methoxyphenyl)pyridin-4-yl)-2-methylphenyl)-5-((((5-oxopyrrolidin-2-yl)methyl)amino)methyl)picolinamide;
(R)-N-(3-(3-chloro-2-(4-(((3-fluoropropyl)amino)methyl)-3-methoxyphenyl)pyridin-4-yl)-2-methylphenyl)-5-((((5-oxopyrrolidin-2-yl)methyl)amino)methyl)picolinamide;
(S)-N-(3-(3-chloro-2-(4-(((2-hydroxyethyl)amino)methyl)-3-methoxyphenyl)pyridin-4-yl)-2-methylphenyl)-5-((((5-oxopyrrolidin-2-yl)methyl)amino)methyl)picolinamide;
(R)-N-(3-(3-chloro-2-(4-(((2-hydroxyethyl)amino)methyl)-3-methoxyphenyl)pyridin-4-yl)-2-methylphenyl)-5-((((5-oxopyrrolidin-2-yl)methyl)amino)methyl)picolinamide;
(S)-N-(2-chloro-3-(3-chloro-2-(3-methoxy-4-((((5-oxopyrrolidin-2-yl)methyl)amino)methyl)phenyl)pyridin-4-yl)phenyl)-5-((methylamino)methyl)picolinamide;
(R)-N-(2-chloro-3-(3-chloro-2-(3-methoxy-4-((((5-oxopyrrolidin-2-yl)methyl)amino)methyl)phenyl)pyridin-4-yl)phenyl)-5-((methylamino)methyl)picolinamide;
(S)-6-((2-chloro-3-(3-chloro-2-(3-methoxy-4-((((5-oxopyrrolidin-2-yl)methyl)amino)methyl)phenyl)pyridin-4-yl)phenyl)carbamoyl)nicotinic acid;
(R)-6-((2-chloro-3-(3-chloro-2-(3-methoxy-4-((((5-oxopyrrolidin-2-yl)methyl)amino)methyl)phenyl)pyridin-4-yl)phenyl)carbamoyl)nicotinic acid;
2-((6-(3-chloro-4-(2-chloro-3-(5-((isopropylamino)methyl)-6-methoxypyridin-2-yl)phenyl)pyridin-2-yl)-8-methoxy-[1,2,4]triazolo[1,5-a]pyridin-2-yl)methyl)-2-azaspiro[3.3]heptan-6-ol;
N-(2-chloro-3-(3-chloro-2-(3-methoxy-4-((7-oxo-2,6-diazaspiro[3.4]octan-2-yl)methyl)phenyl)pyridin-4-yl)phenyl)-5-(((2-hydroxyethyl)amino)methyl)picolinamide;
(S)-N-(2-chloro-3-(3-chloro-2-(3-methoxy-4-((((5-oxopyrrolidin-2-yl)methyl)amino)methyl)phenyl)pyridin-4-yl)phenyl)-5-(((2-hydroxyethyl)amino)methyl)picolinamide;
(R)-N-(2-chloro-3-(3-chloro-2-(3-methoxy-4-((((5-oxopyrrolidin-2-yl)methyl)amino)methyl)phenyl)pyridin-4-yl)phenyl)-5-(((2-hydroxyethyl)amino)methyl)picolinamide;
(S)-N-(3-(3-chloro-2-(3-methoxy-4-((((5-oxopyrrolidin-2-yl)methyl)amino)methyl)phenyl)pyridin-4-yl)-2-methylphenyl)-5-((methylamino)methyl)picolinamide;
(R)-N-(3-(3-chloro-2-(3-methoxy-4-((((5-oxopyrrolidin-2-yl)methyl)amino)methyl)phenyl)pyridin-4-yl)-2-methylphenyl)-5-((methylamino)methyl)picolinamide;
N-(3-(2-(4-((6-acetyl-2,6-diazaspiro[3.3]heptan-2-yl)methyl)-3-methoxyphenyl)-3-chloropyridin-4-yl)-2-chlorophenyl)-5-(((2-hydroxyethyl)amino)methyl)picolinamide;
(1r,4r)-4-(((6-(3-chloro-4-(2-chloro-3-(5-((isopropylamino)methyl)-6-methoxypyridin-2-yl)phenyl)pyridin-2-yl)-8-methoxy-[1,2,4]triazolo[1,5-a]pyridin-2-yl)methyl)amino)cyclohexan-1-ol;
(1s,4r)-4-(((6-(3-chloro-4-(2-chloro-3-(5-((isopropylamino)methyl)-6-methoxypyridin-2-yl)phenyl)pyridin-2-yl)-8-methoxy-[1,2,4]triazolo[1,5-a]pyridin-2-yl)methyl)amino)cyclohexan-1-ol;
(1r,4s)-4-(((6-(3-chloro-4-(2-chloro-3-(5-((isopropylamino)methyl)-6-methoxypyridin-2-yl)phenyl)pyridin-2-yl)-8-methoxy-[1,2,4]triazolo[1,5-a]pyridin-2-yl)methyl)amino)cyclohexan-1-ol;
(1s,4s)-4-(((6-(3-chloro-4-(2-chloro-3-(5-((isopropylamino)methyl)-6-methoxypyridin-2-yl)phenyl)pyridin-2-yl)-8-methoxy-[1,2,4]triazolo[1,5-a]pyridin-2-yl)methyl)amino)cyclohexan-1-ol;
1-((3'-chloro-2'-(2-chloro-3-(5-((4-hydroxypiperidin-1-yl)methyl)-6-methoxypyridin-2-yl)phenyl)-6-methoxy-[2,4'-bipyridin]-5-yl)methyl)piperidin-4-ol;
1-(2-((6-(3-(5-((6-acetyl-2,6-diazaspiro[3.4]octan-2-yl)methyl)-3'-chloro-6-methoxy-[2,4'-bipyridin]-2'-yl)-2-chlorophenyl)-2-methoxypyridin-3-yl)methyl)-2,6-diazaspiro[3.4]octan-6-yl)ethan-1-one;
1-(4-(((6-(2-chloro-3-(3'-chloro-5-((4-hydroxypiperidin-1-yl)methyl)-6-methoxy-[2,4'-bipyridin]-2'-yl)phenyl)-2-methoxypyridin-3-yl)methyl)amino)piperidin-1-yl)ethan-1-one;
N-(3-(3-chloro-2-(3-fluoro-4-(((2-hydroxyethyl)amino)methyl)-5-methoxyphenyl)pyridin-4-yl)-2-methylphenyl)-5-(((2-hydroxyethyl)amino)methyl)picolinamide;
N-((6-(2-chloro-3-(3-chloro-2-(2-((isopropylamino)methyl)-8-methoxy-[1,2,4]triazolo[1,5-a]pyridin-6-yl)pyridin-4-yl)phenyl)-2-methoxypyridin-3-yl)methyl)propan-2-amine;
(S)-5-((((6-(3-chloro-4-(2-chloro-3-(5-((isopropylamino)methyl)-6-methoxypyridin-2-yl)phenyl)pyridin-2-yl)-8-methoxy-[1,2,4]triazolo[1,5-a]pyridin-2-yl)methyl)amino)methyl)pyrrolidin-2-one;
(R)-5-((((6-(3-chloro-4-(2-chloro-3-(5-((isopropylamino)methyl)-6-methoxypyridin-2-yl)phenyl)pyridin-2-yl)-8-methoxy-[1,2,4]triazolo[1,5-a]pyridin-2-yl)methyl)amino)methyl)pyrrolidin-2-one;
1-(4-(((6-(3-chloro-4-(2-chloro-3-(5-((isopropylamino)methyl)-6-methoxypyridin-2-yl)phenyl)pyridin-2-yl)-8-methoxy-[1,2,4]triazolo[1,5-a]pyridin-2-yl)methyl)amino)piperidin-1-yl)ethan-1-one;
N-(3-(3'-chloro-6-methoxy-5-(((tetrahydro-2H-pyran-4-yl)amino)methyl)-[2,4'-bipyridin]-2'-yl)-2-methylphenyl)-5-(((2-hydroxyethyl)amino)methyl)picolinamide;
1-(6-((6-(3-chloro-4-(2-chloro-3-(5-((isopropylamino)methyl)-6-methoxypyridin-2-yl)phenyl)pyridin-2-yl)-8-methoxy-[1,2,4]triazolo[1,5-a]pyridin-2-yl)methyl)-2,6-diazaspiro[3.3]heptan-2-yl)ethan-1-one;
N-(2-chloro-3-(3-chloro-2-(3-methoxy-4-(((tetrahydro-2H-pyran-4-yl)amino)methyl)phenyl)pyridin-4-yl)phenyl)-5-(((2-hydroxyethyl)amino)methyl)picolinamide;
N-(3-(5-(((1-acetylpiperidin-4-yl)amino)methyl)-3'-chloro-6-methoxy-[2,4'-bipyridin]-2'-yl)-2-methylphenyl)-5-(((2-hydroxyethyl)amino)methyl)picolinamide;
(S)-N-(3-(3-chloro-2-(3-(difluoromethoxy)-4-((((5-oxopyrrolidin-2-yl)methyl)amino)methyl)phenyl)pyridin-4-yl)-2-methylphenyl)-5-(((2-hydroxyethyl)amino)methyl)picolinamide;
(R)-N-(3-(3-chloro-2-(3-(difluoromethoxy)-4-((((5-oxopyrrolidin-2-yl)methyl)amino)methyl)phenyl)pyridin-4-yl)-2-methylphenyl)-5-(((2-hydroxyethyl)amino)methyl)picolinamide;
(S)-N-(3-(3'-chloro-6-methoxy-5-((((5-oxopyrrolidin-2-yl)methyl)amino)methyl)-[2,4'-bipyridin]-2'-yl)-2-methylphenyl)-5-(((2-hydroxyethyl)amino)methyl)picolinamide;
(R)-N-(3-(3'-chloro-6-methoxy-5-((((5-oxopyrrolidin-2-yl)methyl)amino)methyl)-[2,4'-bipyridin]-2'-yl)-2-methylphenyl)-5-(((2-hydroxyethyl)amino)methyl)picolinamide;
(R)-1-((3'-chloro-2'-(2-chloro-3-(5-(((R)-3-hydroxypyrrolidin-1-yl)methyl)-6-methoxypyridin-2-yl)phenyl)-6-methoxy-[2,4'-bipyridin]-5-yl)methyl)pyrrolidin-3-ol;
(R)-1-((3'-chloro-2'-(2-chloro-3-(5-(((S)-3-hydroxypyrrolidin-1-yl)methyl)-6-methoxypyridin-2-yl)phenyl)-6-methoxy-[2,4'-bipyridin]-5-yl)methyl)pyrrolidin-3-ol;
(S)-1-((3'-chloro-2'-(2-chloro-3-(5-(((R)-3-hydroxypyrrolidin-1-yl)methyl)-6-methoxypyridin-2-yl)phenyl)-6-methoxy-[2,4'-bipyridin]-5-yl)methyl)pyrrolidin-3-ol;
(S)-1-((3'-chloro-2'-(2-chloro-3-(5-(((S)-3-hydroxypyrrolidin-1-yl)methyl)-6-methoxypyridin-2-yl)phenyl)-6-methoxy-[2,4'-bipyridin]-5-yl)methyl)pyrrolidin-3-ol; and
1-(4-(((3'-chloro-2'-(2-chloro-3-(5-((4-hydroxypiperidin-1-yl)methyl)-6-methoxypyridin-2-yl)phenyl)-6-methoxy-[2,4'-bipyridin]-5-yl)methyl)amino)piperidin-1-yl)ethan-1-one.

The compounds of the disclosure may possess one or more stereocenters, and each stereocenter may exist independently in either the (*R*) or (*S*) configuration. In certain embodiments, compounds described herein are present in optically active or racemic forms. The compounds described herein encompass racemic, optically active, regioisomeric and stereoisomeric forms, or combinations thereof that possess the therapeutically useful properties described herein. Preparation of optically active forms is achieved in any suitable manner, including by way of non-limiting example, by resolution of the racemic form with recrystallization techniques, synthesis from optically active starting materials, chiral synthesis, or chromatographic separation using a chiral stationary phase. A compound illustrated herein by the racemic formula further represents either of the two enantiomers or mixtures thereof, or in the case where two or more chiral center are present, all diastereomers or mixtures thereof.

In certain embodiments, the compounds of the disclosure exist as tautomers. All tautomers are included within the scope of the compounds recited herein.

Compounds described herein also include isotopically labeled compounds wherein one or more atoms is replaced by an atom having the same atomic number, but an atomic mass or mass number different from the atomic mass or mass number usually found in nature. Examples of isotopes suitable for inclusion in the compounds described herein include and are not limited to ²H, ³H, ¹¹C, ¹³C, ¹⁴C, ³⁶Cl, ¹⁸F, ¹²³I, ¹²⁵I, ¹³N, ¹⁵N, ¹⁵O, ¹⁷O, ¹⁸O, ³²P, and ³⁵S. In certain embodiments, substitution with heavier isotopes such as deuterium affords greater chemical stability. Isotopically labeled compounds are prepared by any suitable method or by processes using an appropriate isotopically labeled reagent in place of the non-labeled reagent otherwise employed.

In certain embodiments, the compounds described herein are labeled by other means, including, but not limited to, the use of chromophores or fluorescent moieties, bioluminescent labels, or chemiluminescent labels.

In all of the embodiments provided herein, examples of suitable optional substituents are not intended to limit the scope of the claimed disclosure. The compounds of the disclosure may contain any of the substituents, or combinations of substituents, provided herein.

### Salts

The compounds described herein may form salts with acids or bases, and such salts are included in the present disclosure. The term "salts" embraces addition salts of free acids or bases that are useful within the methods of the disclosure. The term "pharmaceutically acceptable salt" refers to salts that possess toxicity profiles within a range that affords utility in pharmaceutical applications. In certain embodiments, the salts are pharmaceutically acceptable salts. Pharmaceutically unacceptable salts may nonetheless possess properties such as high crystallinity, which have utility in the practice of the present disclosure, such as for example utility in process of synthesis, purification or formulation of compounds useful within the methods of the disclosure.

Suitable pharmaceutically acceptable acid addition salts may be prepared from an inorganic acid or from an organic acid. Examples of inorganic acids include sulfate, hydrogen sulfate, hydrochloric, hydrobromic, hydriodic, nitric, carbonic, sulfuric, and phosphoric acids (including hydrogen phosphate and dihydrogen phosphate). Appropriate organic acids may be selected from aliphatic, cycloaliphatic, aromatic, araliphatic, heterocyclic, carboxylic and sulfonic classes of organic acids, examples of which include formic, acetic, propionic, succinic, glycolic, gluconic, lactic, malic, tartaric, citric, ascorbic, glucuronic, maleic, fumaric, pyruvic, aspartic, glutamic, benzoic, anthranilic, 4-hydroxybenzoic, phenylacetic, mandelic, embonic (or pamoic), methanesulfonic, ethanesulfonic, benzene sulfonic, pantothenic, sulfanilic, 2-hydroxyethanesulfonic, trifluoromethanesulfonic, p-toluenesulfonic, cyclohexylaminosulfonic, stearic, alginic, β-hydroxybutyric, salicylic, galactaric, galacturonic acid, glycerophosphonic acids and saccharin (*e.g.*, saccharinate, saccharate). Salts may be comprised of a fraction of one, one or more than one molar equivalent of acid or base with respect to any compound of the disclosure.

Suitable pharmaceutically acceptable base addition salts of compounds of the disclosure include, for example, ammonium salts and metallic salts including alkali metal, alkaline earth metal and transition metal salts such as, for example, calcium, magnesium, potassium, sodium and zinc salts. Pharmaceutically acceptable base addition salts also include organic salts made from basic amines such as, for example, N,N'-dibenzylethylenediamine, chloroprocaine, choline, diethanolamine, ethylenediamine, meglumine (or N-methylglucamine) and procaine. All of these salts may be prepared from the corresponding compound by reacting, for example, the appropriate acid or base with the compound.

### Combination Therapies

In one aspect, the compounds of the disclosure are useful within the methods of the disclosure in combination with one or more additional agents useful for treating HBV and/or HDV infections. These additional agents may comprise compounds or compositions identified herein, or compounds (*e.g.*, commercially available compounds) known to treat, prevent, or reduce the symptoms of HBV and/or HDV infections.

Non-limiting examples of one or more additional agents useful for treating HBV and/or HDV infections include: (a) reverse transcriptase inhibitors; (b) capsid inhibitors; (c) cccDNA formation inhibitors; (d) RNA destabilizers; (e) oligomeric nucleotides targeted against the HBV genome; (f) immunostimulators, such as checkpoint inhibitors (*e.g.,* PD-L1 inhibitors); (g) GalNAc-siRNA conjugates targeted against an HBV gene transcript; and (h) therapeutic vaccines.

### (a) Reverse Transcriptase Inhibitors

In certain embodiments, the reverse transcriptase inhibitor is a reverse-transcriptase inhibitor (NARTI or NRTI). In other embodiments, the reverse transcriptase inhibitor is a nucleotide analog reverse-transcriptase inhibitor (NtARTI or NtRTI).

Reported reverse transcriptase inhibitors include, but are not limited to, entecavir, clevudine, telbivudine, lamivudine, adefovir, and tenofovir, tenofovir disoproxil, tenofovir alafenamide, adefovir dipovoxil, (1*R*,2*R*,3*R*,5*R*)-3-(6-amino-9*H*-9-purinyl)-2-fluoro-5-(hydroxymethyl)-4-methylenecyclopentan-1-ol (described in U.S. Patent No. 8,816,074, incorporated herein in its entirety by reference), emtricitabine, abacavir, elvucitabine, ganciclovir, lobucavir, famciclovir, penciclovir, and amdoxovir.

Reported reverse transcriptase inhibitors further include, but are not limited to, entecavir, lamivudine, and (1R,2R,3R,5R)-3-(6-amino-9H-9-purinyl)-2-fluoro-5-(hydroxymethyl)-4-methylenecyclopentan-1-ol.

Reported reverse transcriptase inhibitors further include, but are not limited to, a covalently bound phosphoramidate or phosphonamidate moiety of the above-mentioned reverse transcriptase inhibitors, or as described in for example U.S. Patent No. 8,816,074, US Patent Application Publications No. US 2011/0245484 A1, and US 2008/0286230A1, all of which incorporated herein in their entireties by reference.

Reported reverse transcriptase inhibitors further include, but are not limited to, nucleotide analogs that comprise a phosphoramidate moiety, such as, for example, methyl ((((1*R*,3*R*,4*R*,5*R*)-3-(6-amino-9*H*-purin-9-yl)-4-fluoro-5-hydroxy-2-methylenecyclopentyl) methoxy)(phenoxy) phosphoryl)-(D or L)-alaninate and methyl ((((1*R*,2*R*,3*R*,4*R*)-3-fluoro-2-hydroxy-5-methylene-4-(6-oxo-1,6-dihydro-9*H*-purin-9-yl)cyclopentyl)methoxy)(phenoxy) phosphoryl)-(D or L)-alaninate. Also included are the individual diastereomers thereof, which include, for example, methyl ((*R*)-(((1*R*,3*R*,4*R*,5*R*)-3-(6-amino-9*H*-purin-9-yl)-4-fluoro-5-hydroxy-2-methylenecyclopentyl)methoxy)(phenoxy)phosphoryl)-(D or L)-alaninate and methyl ((*S*)-(((1*R*,3*R*,4*R*,5*R*)-3-(6-amino-9*H*-purin-9-yl)-4-fluoro-5-hydroxy-2-methylenecyclopentyl) methoxy)(phenoxy)phosphoryl)-(D or L)-alaninate.

Reported reverse transcriptase inhibitors further include, but are not limited to, compounds comprising a phosphonamidate moiety, such as, for example, tenofovir alafenamide, as well as those described in U.S. Patent Application Publication No. US 2008/0286230 A1, incorporated herein in its entirety by reference. Methods for preparing stereoselective phosphoramidate or phosphonamidate containing actives are described in, for example, U.S. Patent No. 8,816,074, as well as U.S. Patent Application Publications No. US 2011/0245484 A1 and US 2008/0286230 A1, all of which incorporated herein in their entireties by reference.

### (b) Capsid Inhibitors

As described herein, the term "capsid inhibitor" includes compounds that are capable of inhibiting the expression and/or function of a capsid protein either directly or indirectly. For example, a capsid inhibitor may include, but is not limited to, any compound that inhibits capsid assembly, induces formation of non-capsid polymers, promotes excess capsid assembly or misdirected capsid assembly, affects capsid stabilization, and/or inhibits encapsidation of RNA (pgRNA). Capsid inhibitors also include any compound that inhibits capsid function in a downstream event(s) within the replication process (*e*.*g*., viral DNA synthesis, transport of relaxed circular DNA (rcDNA) into the nucleus, covalently closed circular DNA (cccDNA) formation, virus maturation, budding and/or release, and the like). For example, in certain embodiments, the inhibitor detectably inhibits the expression level or biological activity of the capsid protein as measured, *e*.*g*., using an assay described herein. In certain embodiments, the inhibitor inhibits the level of rcDNA and downstream products of viral life cycle by at least 5%, at least 10%, at least 20%, at least 50%, at least 75%, or at least 90%.

Reported capsid inhibitors include, but are not limited to, compounds described in International Patent Applications Publication Nos WO 2013006394, WO 2014106019, and WO2014089296, all of which incorporated herein in their entireties by reference.

Reported capsid inhibitors also include, but are not limited to, the following compounds and pharmaceutically acceptable salts and/or solvates thereof: Bay-41-4109 (see Int'l Patent Application Publication No. WO 2013144129), AT-61 (see Int'l Patent Application Publication No. WO 1998033501; and King, et al., 1998, Antimicrob. Agents Chemother. 42(12):3179-3186), DVR-01 and DVR-23 (see Int'l Patent Application Publication No. WO 2013006394; and Campagna, et al., 2013, J. Virol. 87(12):6931, all of which incorporated herein in their entireties by reference.

In addition, reported capsid inhibitors include, but are not limited to, those generally and specifically described in U.S. Patent Application Publication Nos. US 2015/0225355, US 2015/0132258, US 2016/0083383, US 2016/0052921, US 2019/0225593, and Int'l Patent Application Publication Nos. WO 2013096744, WO 2014165128, WO 2014033170, WO 2014033167, WO 2014033176, WO 2014131847, WO 2014161888, WO 2014184350, WO 2014184365, WO 2015059212, WO 2015011281, WO 2015118057, WO 2015109130, WO 2015073774, WO 2015180631, WO 2015138895, WO 2016089990, WO 2017015451, WO 2016183266, WO 2017011552, WO 2017048950, WO2017048954, WO 2017048962, WO 2017064156, WO 2018052967, WO 2018172852, WO 2020023710, WO2020123674, and are incorporated herein in their entirety by reference.

### (c) cccDNA Formation Inhibitors

Covalently closed circular DNA (cccDNA) is generated in the cell nucleus from viral rcDNA and serves as the transcription template for viral mRNAs. As described herein, the term "cccDNA formation inhibitor" includes compounds that are capable of inhibiting the formation and/or stability of cccDNA either directly or indirectly. For example, a cccDNA formation inhibitor may include, but is not limited to, any compound that inhibits capsid disassembly, rcDNA entry into the nucleus, and/or the conversion of rcDNA into cccDNA. For example, in certain embodiments, the inhibitor detectably inhibits the formation and/or stability of the cccDNA as measured, *e*.*g*., using an assay described herein. In certain embodiments, the inhibitor inhibits the formation and/or stability of cccDNA by at least 5%, at least 10%, at least 20%, at least 50%, at least 75%, or at least 90%.

Reported cccDNA formation inhibitors include, but are not limited to, compounds described in Int'l Patent Application Publication No. WO 2013130703, and are incorporated herein in their entirety by reference.

In addition, reported cccDNA formation inhibitors include, but are not limited to, those generally and specifically described in U.S. Patent Application Publication No. US 2015/0038515 A1, and are incorporated herein in their entirety by reference.

### (d) RNA Destabilizer

As used herein, the term "RNA destabilizer" refers to a molecule, or a salt or solvate thereof, that reduces the total amount of HBV RNA in mammalian cell culture or in a live human subject. In a non-limiting example, an RNA destabilizer reduces the amount of the RNA transcript(s) encoding one or more of the following HBV proteins: surface antigen, core protein, RNA polymerase, and e antigen. In certain embodiments, the RNA destabilizer reduces the total amount of HBV RNA in mammalian cell culture or in a live human subject by at least 5%, at least 10%, at least 20%, at least 50%, at least 75%, or at least 90%.

Reported RNA destabilizers include compounds described in U.S. Patent No. 8,921,381, as well as compounds described in U.S. Patent Application Publication Nos. US 2015/0087659 and US 2013/0303552, all of which are incorporated herein in their entireties by reference.

In addition, reported RNA destabilizers include, but are not limited to, those generally and specifically described in Int'l Patent Application Publication Nos. WO 2015113990, WO 2015173164, US 2016/0122344, WO 2016107832, WO 2016023877, WO 2016128335, WO 2016177655, WO 2016071215, WO 2017013046, WO 2017016921, WO 2017016960, WO 2017017042, WO 2017017043, WO 2017102648, WO 2017108630, WO 2017114812, WO 2017140821, WO 2018085619, and are incorporated herein in their entirety by reference.

### (e) Oligomeric Nucleotides Targeted Against the HBV Genome

Reported oligomeric nucleotides targeted against the HBV genome include, but are not limited to, Arrowhead-ARC-520 (see U.S. Patent No. 8,809,293; and Wooddell et al., 2013, Molecular Therapy 21(5):973-985, all of which incorporated herein in their entireties by reference).

In certain embodiments, the oligomeric nucleotides can be designed to target one or more genes and/or transcripts of the HBV genome. Oligomeric nucleotide targeted to the HBV genome also include, but are not limited to, isolated, double stranded, siRNA molecules, that each include a sense strand and an antisense strand that is hybridized to the sense strand. In certain embodiments, the siRNA target one or more genes and/or transcripts of the HBV genome.

### (f) Immunostimulators

### Checkpoint Inhibitors

As described herein, the term "checkpoint inhibitor" includes any compound that is capable of inhibiting immune checkpoint molecules that are regulators of the immune system (*e.g*., stimulate or inhibit immune system activity). For example, some checkpoint inhibitors block inhibitory checkpoint molecules, thereby stimulating immune system function, such as stimulation of T cell activity against cancer cells. A non-limiting example of a checkpoint inhibitor is a PD-L1 inhibitor.

As described herein, the term "PD-L1 inhibitor" includes any compound that is capable of inhibiting the expression and/or function of the protein Programmed Death-Ligand 1 (PD-L1) either directly or indirectly. PD-L1, also known as cluster of differentiation 274 (CD274) or B7 homolog 1 (B7-H1), is a type 1 transmembrane protein that plays a major role in suppressing the adaptive arm of immune system during pregnancy, tissue allograft transplants, autoimmune disease, and hepatitis. PD-L1 binds to its receptor, the inhibitory checkpoint molecule PD-1 (which is found on activated T cells, B cells, and myeloid cells) so as to modulate activation or inhibition of the adaptive arm of immune system. In certain embodiments, the PD-L1 inhibitor inhibits the expression and/or function of PD-L1 by at least 5%, at least 10%, at least 20%, at least 50%, at least 75%, or at least 90%.

Reported PD-L1 inhibitors include, but are not limited to, compounds recited in one of the following patent application publications: US 2018/0057455; US 2018/0057486; WO 2017/106634; WO 2018/026971; WO 2018/045142; WO 2018/118848; WO 2018/119221; WO 2018/119236; WO 2018/119266; WO 2018/119286; WO 2018/121560; WO 2019/076343; WO 2019/087214; and are incorporated herein in their entirety by reference.

### (g) GalNAc-siRNA Conjugates Targeted Against an HBV Gene Transcript

"GalNAc" is the abbreviation for N-acetylgalactosamine, and "siRNA" is the abbreviation for small interfering RNA. An siRNA that targets an HBV gene transcript is covalently bonded to GalNAc in a GalNAc-siRNA conjugate useful in the practice of the present disclosure. While not wishing to be bound by theory, it is believed that GalNAc binds to asialoglycoprotein receptors on hepatocytes thereby facilitating the targeting of the siRNA to the hepatocytes that are infected with HBV. The siRNA enter the infected hepatocytes and stimulate destruction of HBV gene transcripts by the phenomenon of RNA interference.

Examples of GalNAc-siRNA conjugates useful in the practice of this aspect of the present disclosure are set forth in published international application PCT/CA2017/050447 (PCT Application Publication number WO/2017/177326, published on October 19, 2017) which is hereby incorporated by reference in its entirety.

### (h) Therapeutic Vaccines

In certain embodiments, administration of a therapeutic vaccine is useful in the practice of the present disclosure for the treatment of a viral disease in a subject. In certain embodiments, the viral disease is a hepatitis virus. In certain embodiments, the hepatitis virus is at least one selected from the group consisting of hepatitis B virus (HBV) and hepatitis D virus (HDV). In certain embodiments, the subject is a human.

A synergistic effect may be calculated, for example, using suitable methods such as, for example, the Sigmoid-Eₘₐₓ equation (Holford & Scheiner, 1981, Clin. Pharmacokinet. 6:429-453), the equation of Loewe additivity (Loewe & Muischnek, 1926, Arch. Exp. Pathol Pharmacol. 114: 313-326) and the median-effect equation (Chou & Talalay, 1984, Adv. Enzyme Regul. 22:27-55). Each equation referred to elsewhere herein may be applied to experimental data to generate a corresponding graph to aid in assessing the effects of the drug combination. The corresponding graphs associated with the equations referred to elsewhere herein are the concentration-effect curve, isobologram curve and combination index curve, respectively.

### Synthesis

The present disclosure further provides methods of preparing the compounds of the present disclosure. Compounds of the present teachings can be prepared in accordance with the procedures outlined herein, from commercially available starting materials, compounds known in the literature, or readily prepared intermediates, by employing standard synthetic methods and procedures known to those skilled in the art. Standard synthetic methods and procedures for the preparation of organic molecules and functional group transformations and manipulations can be readily obtained from the relevant scientific literature or from standard textbooks in the field.

It is appreciated that where typical or preferred process conditions (*i.e.,* reaction temperatures, times, mole ratios of reactants, solvents, pressures, and so forth) are given, other process conditions can also be used unless otherwise stated. Optimum reaction conditions can vary with the particular reactants or solvent used, but such conditions can be determined by one skilled in the art by routine optimization procedures. Those skilled in the art of organic synthesis will recognize that the nature and order of the synthetic steps presented can be varied for the purpose of optimizing the formation of the compounds described herein.

The processes described herein can be monitored according to any suitable method known in the art. For example, product formation can be monitored by spectroscopic means, such as nuclear magnetic resonance spectroscopy (*e.g*., ¹H or ¹³C), infrared spectroscopy, spectrophotometry (*e.g.*, UV-visible), mass spectrometry, or by chromatography such as high pressure liquid chromatograpy (HPLC), gas chromatography (GC), gel-permeation chromatography (GPC), or thin layer chromatography (TLC).

Preparation of the compounds can involve protection and deprotection of various chemical groups. The need for protection and deprotection and the selection of appropriate protecting groups can be readily determined by one skilled in the art. The chemistry of protecting groups can be found, for example, in Greene, et al., Protective Groups in Organic Synthesis, 2d. Ed. (Wiley & Sons, 1991), the entire disclosure of which is incorporated by reference herein for all purposes.

The reactions or the processes described herein can be carried out in suitable solvents that can be readily selected by one skilled in the art of organic synthesis. Suitable solvents typically are substantially nonreactive with the reactants, intermediates, and/or products at the temperatures at which the reactions are carried out, *i.e.,* temperatures that can range from the solvent's freezing temperature to the solvent's boiling temperature. A given reaction can be carried out in one solvent or a mixture of more than one solvent. Depending on the particular reaction step, suitable solvents for a particular reaction step can be selected.

A compound of formula **(I)** can be prepared, for example, according to the synthetic methods outlined in Schemes **1-55,** wherein Ar₁ and Ar₂ are each independently selected from the group consisting of optionally substituted C₆-C₁₀ aryl and optionally substituted C₄-C₁₀ heteroaryl; R¹, R², R³, R⁴, and R⁷ are each independently selected from the group consisting of H, optionally substituted C₁-C₆ alkyl, optionally substituted C₃-C₈ cycloalkyl, optionally substituted C₁-C₃ alkoxy, optionally substituted C₁-C₃ haloalkyl, and optionally substituted C₁-C₃ haloalkoxy, or may comprise any substituent otherwise disclosed in any embodiment of the present disclosure; Z is N or C(R^{V10}); and R^{1a}, R^{1d} , and Y are defined within the scope of the present disclosure.

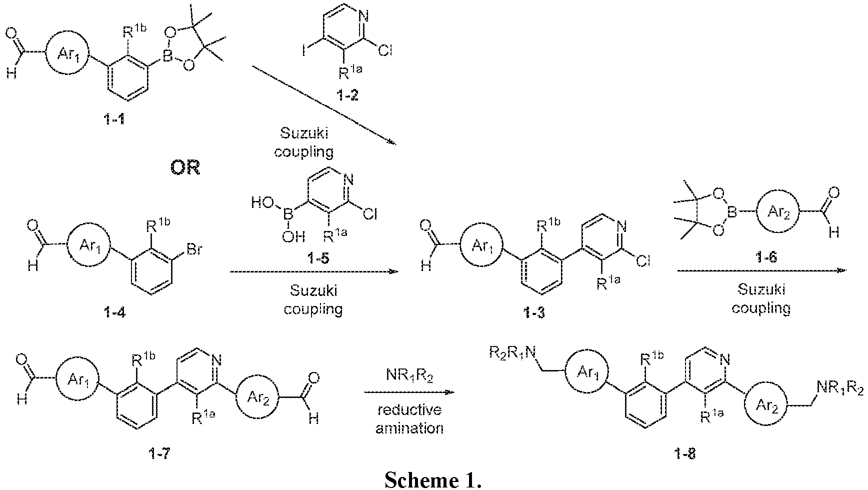

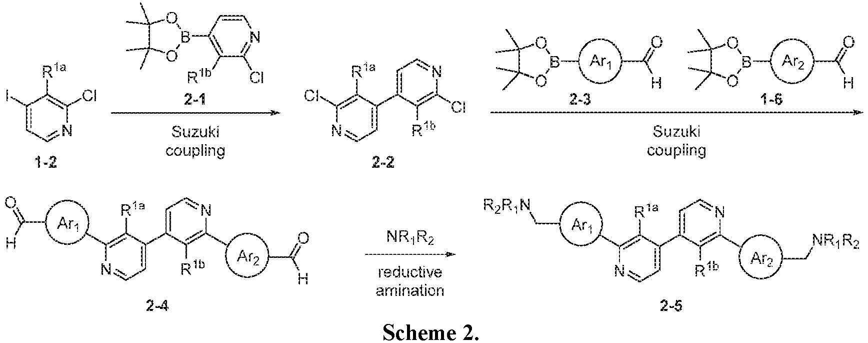

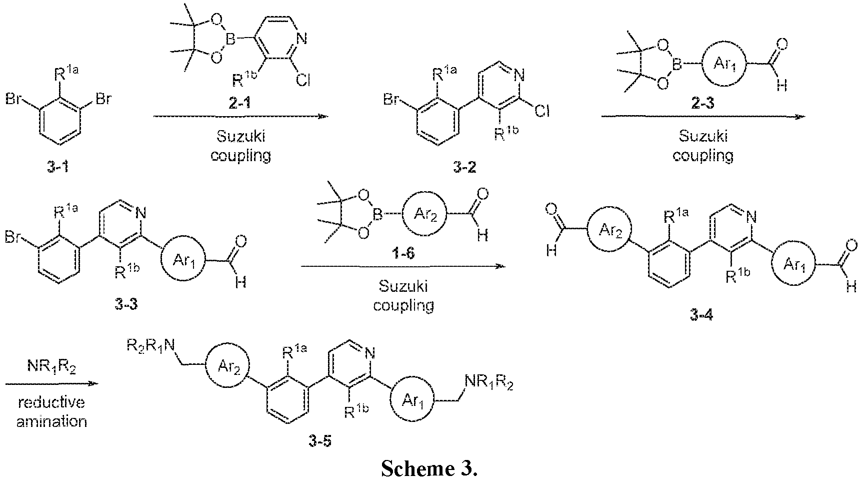

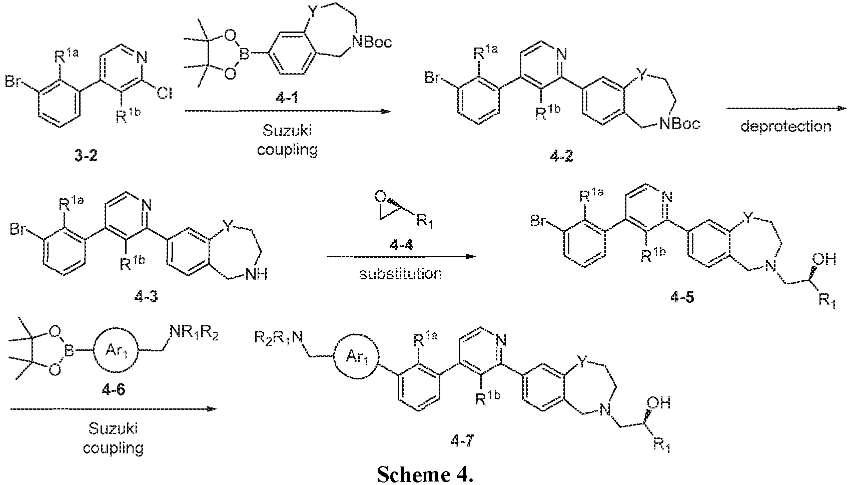

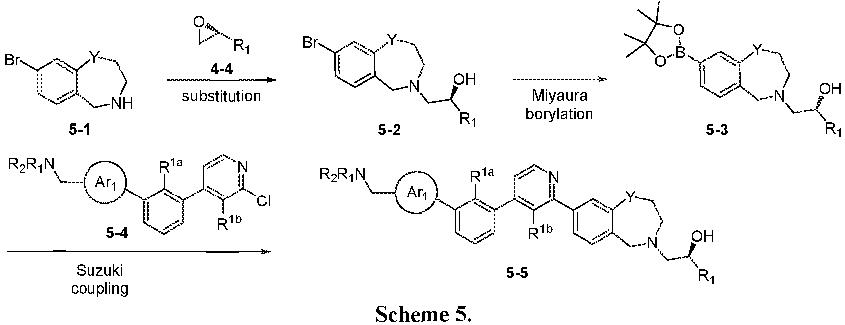

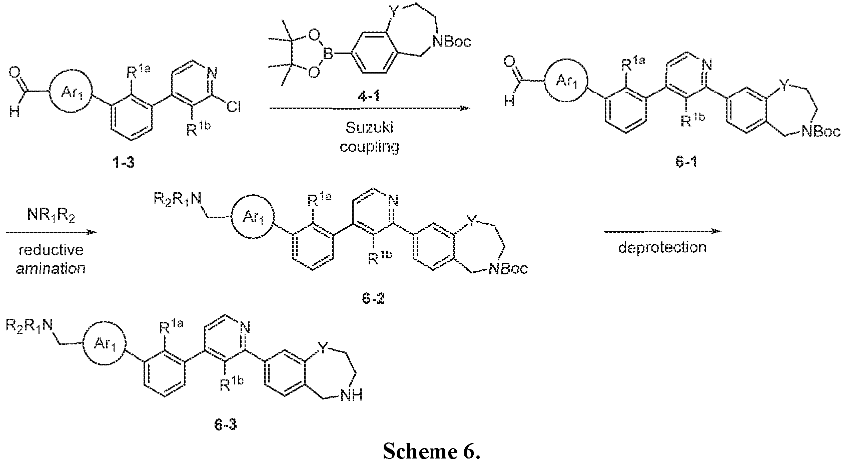

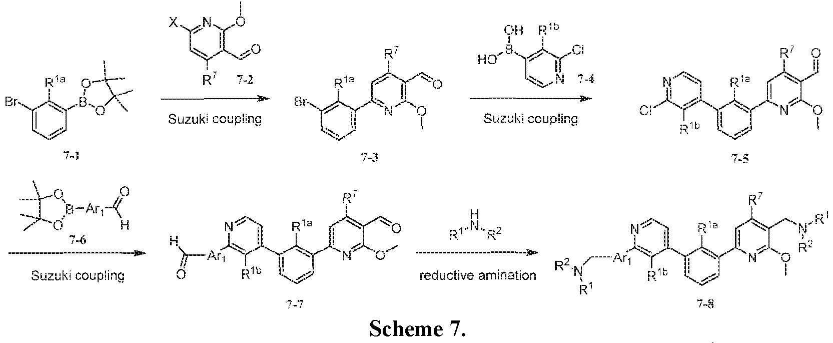

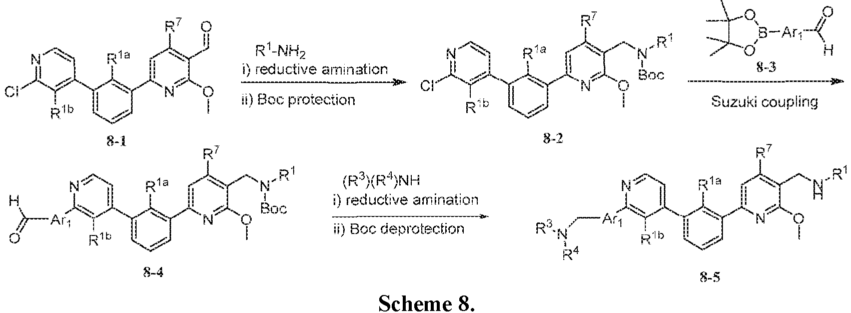

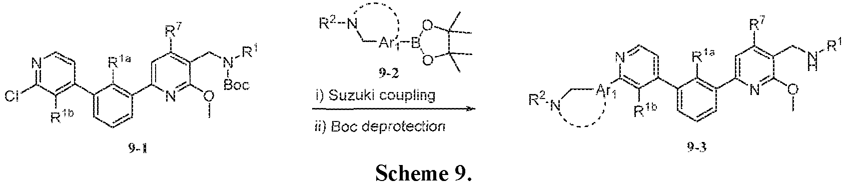

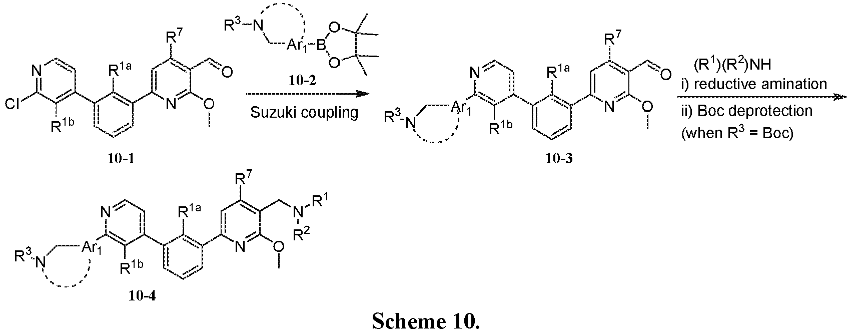

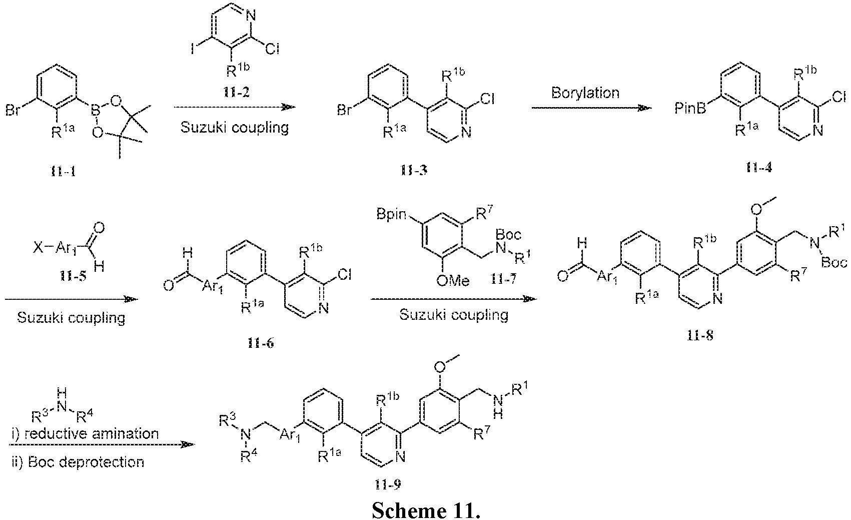

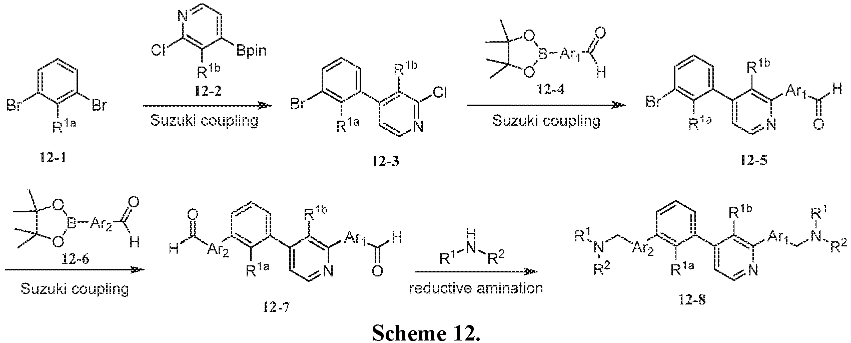

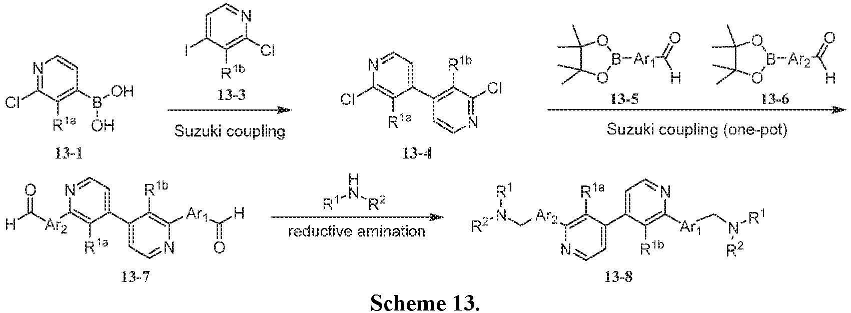

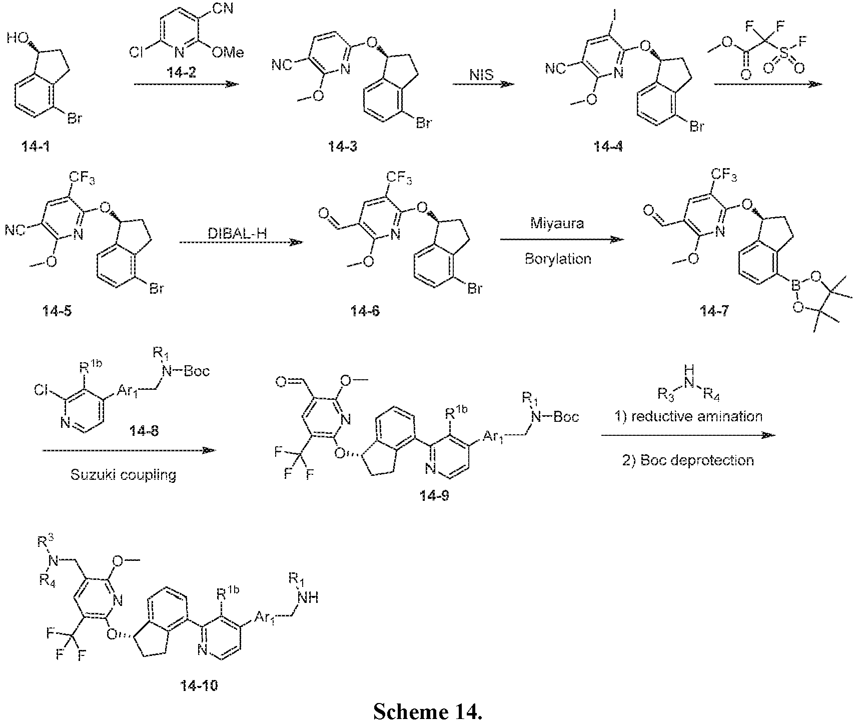

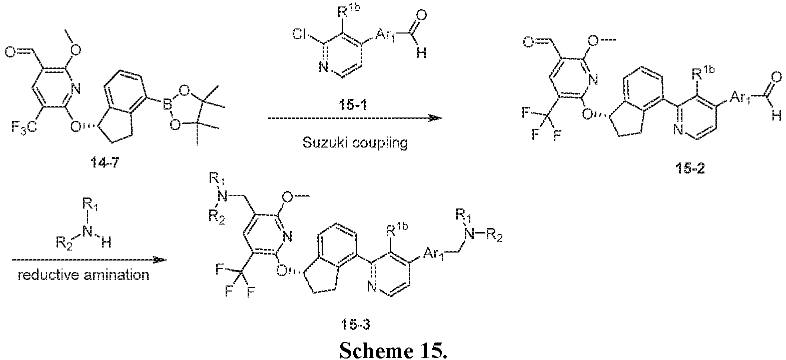

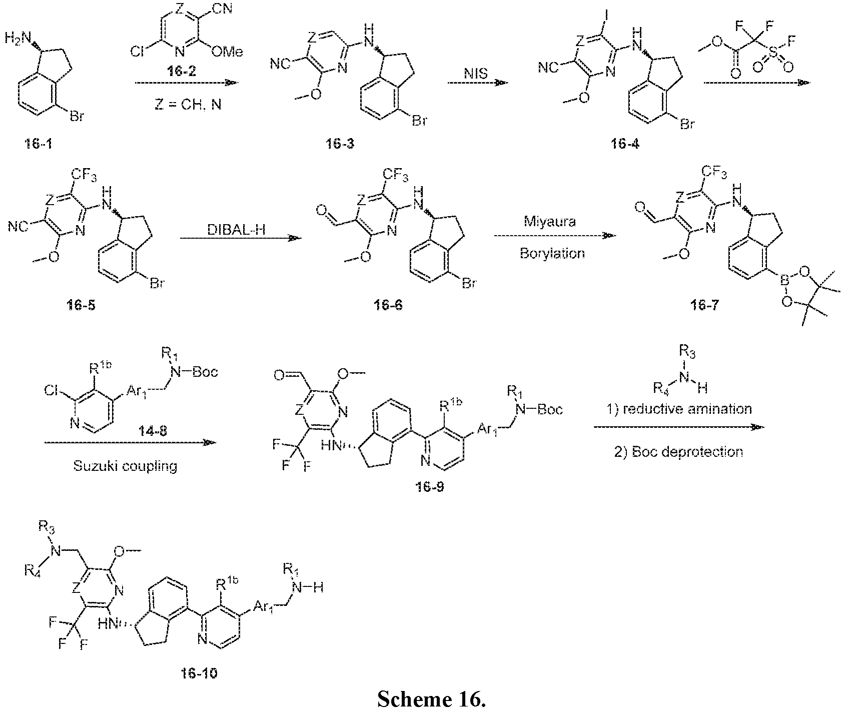

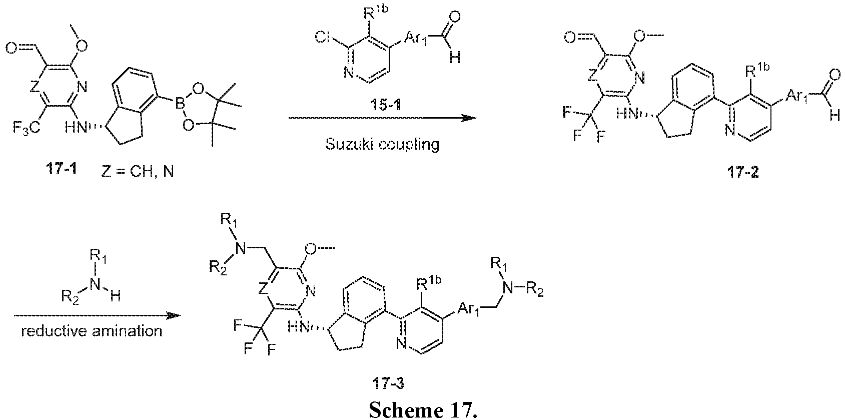

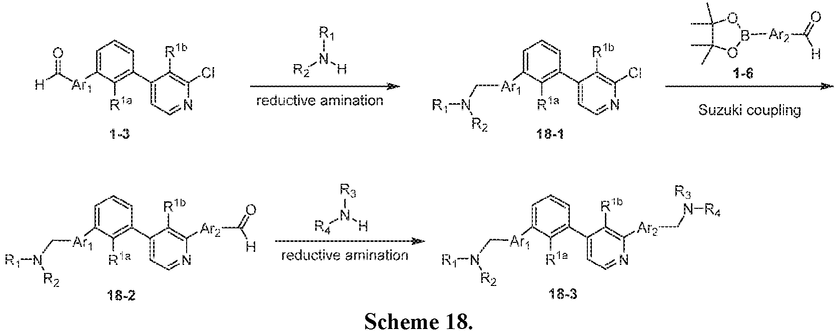

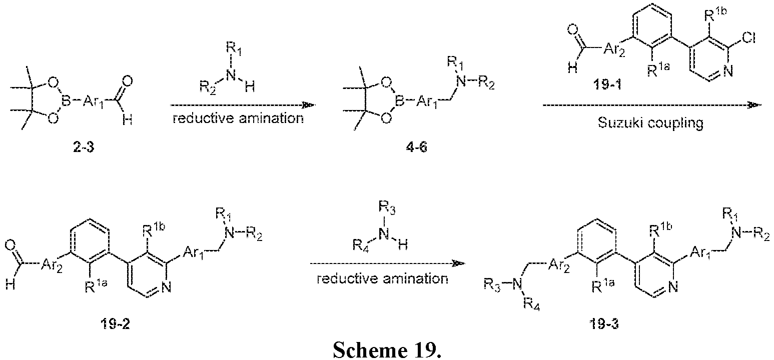

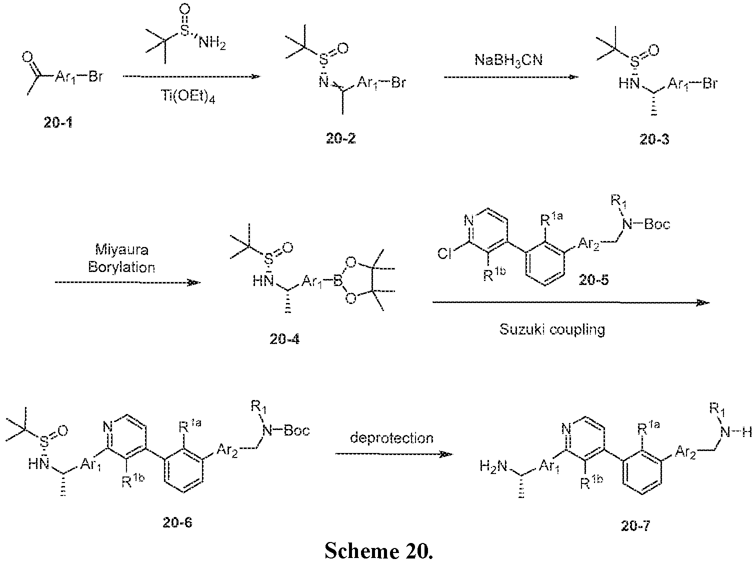

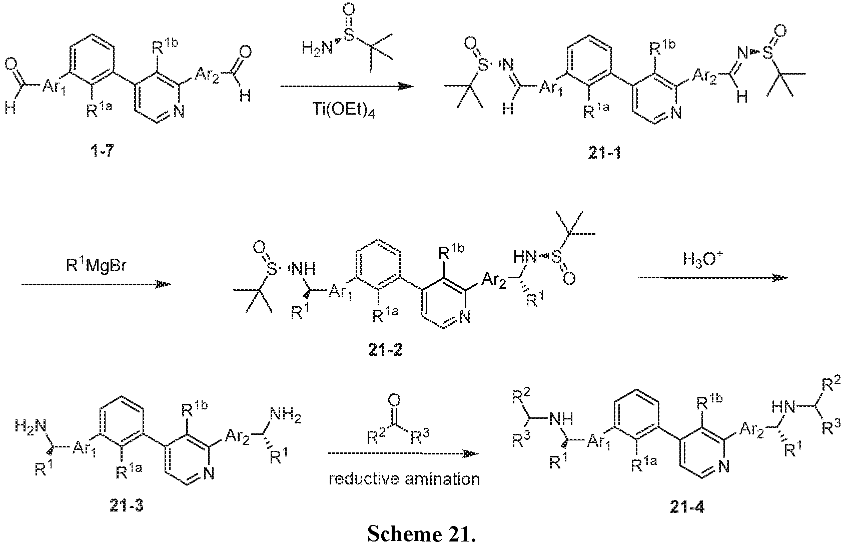

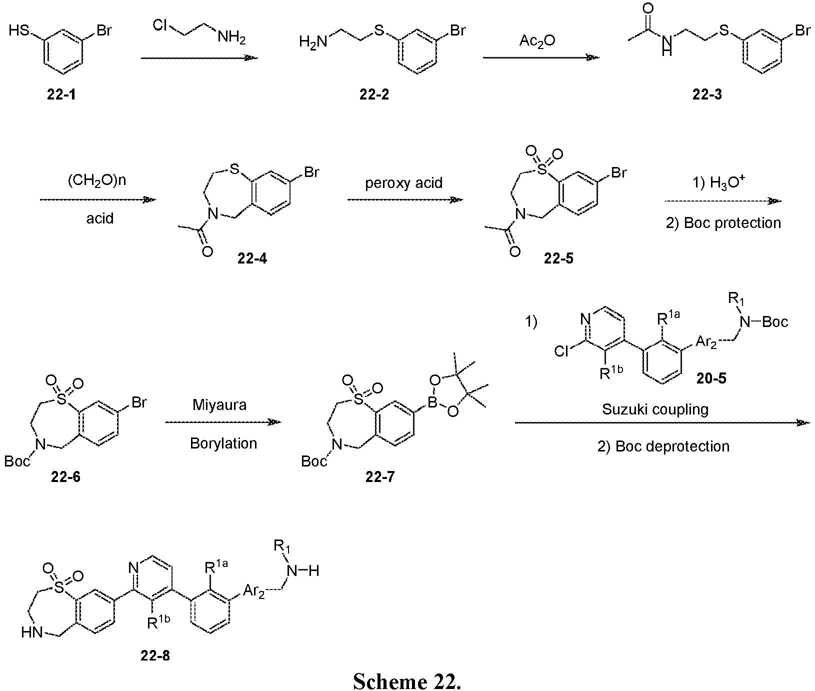

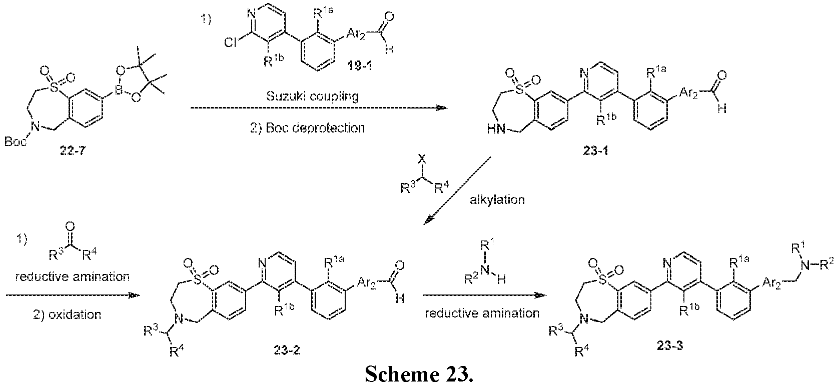

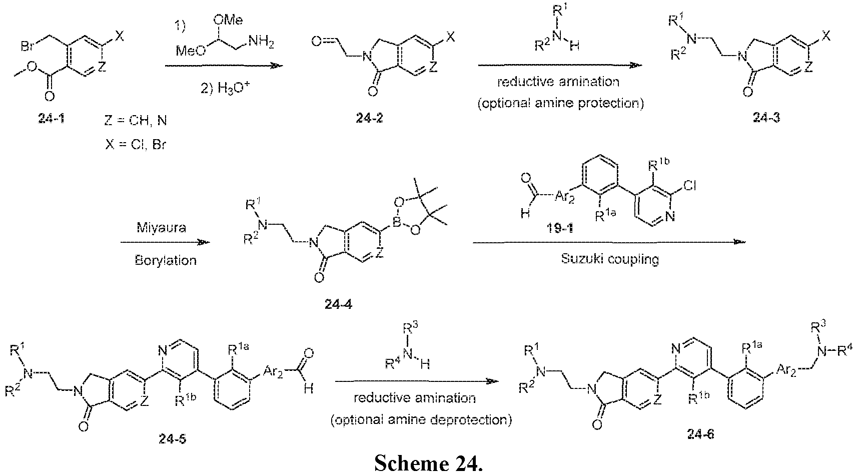

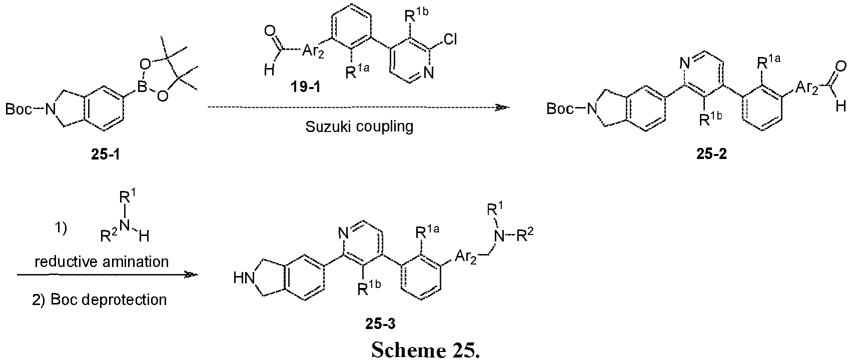

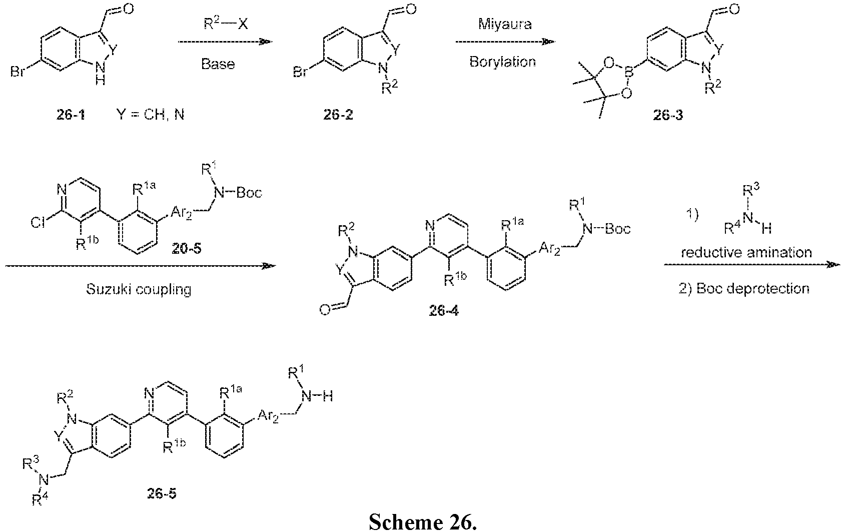

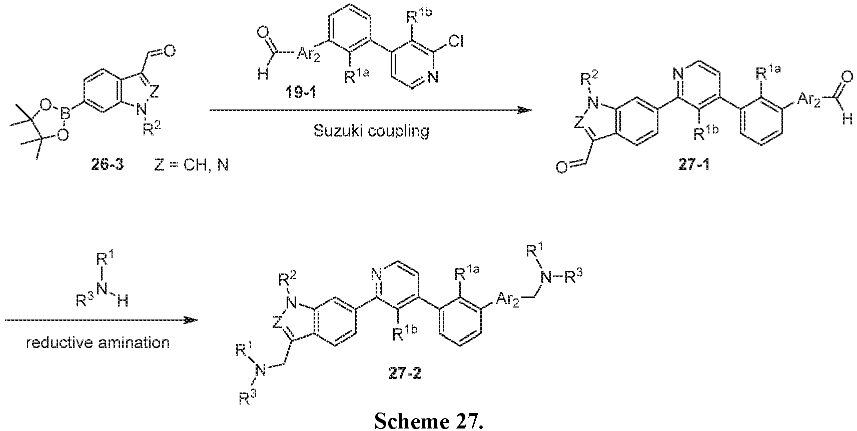

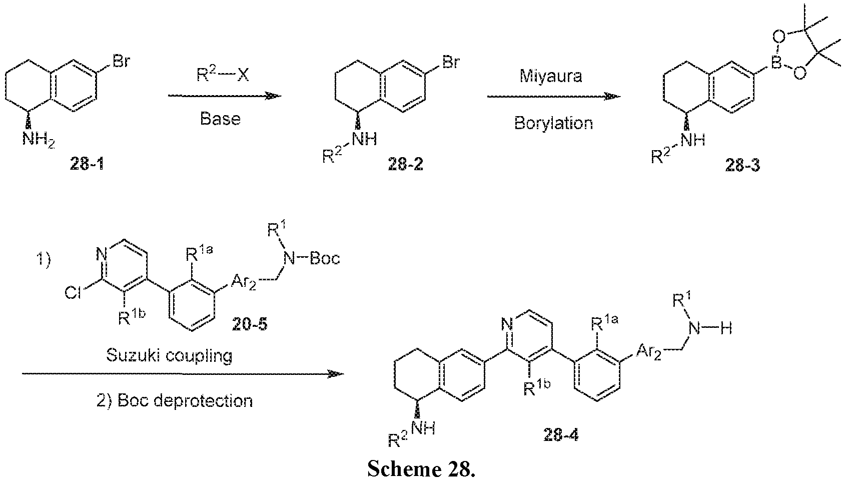

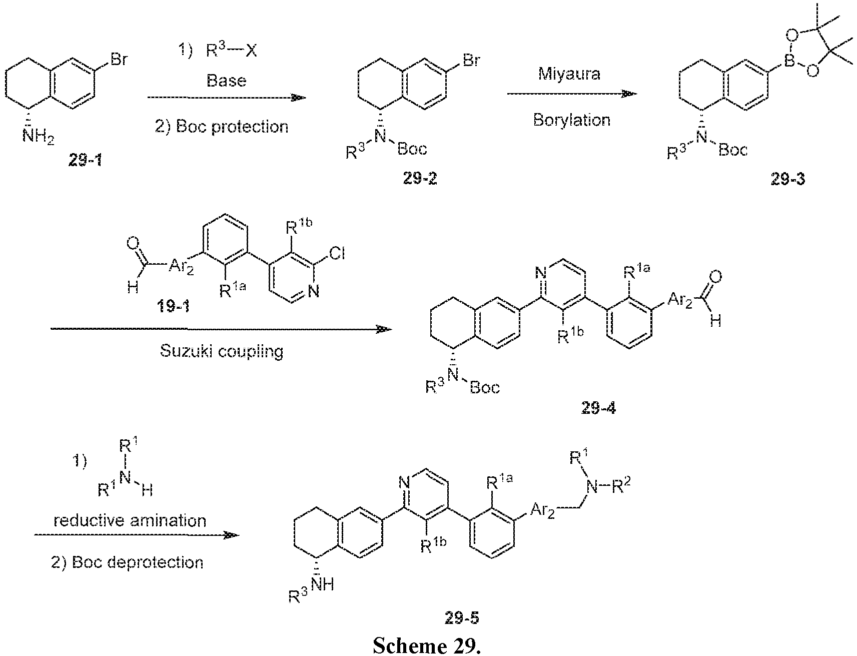

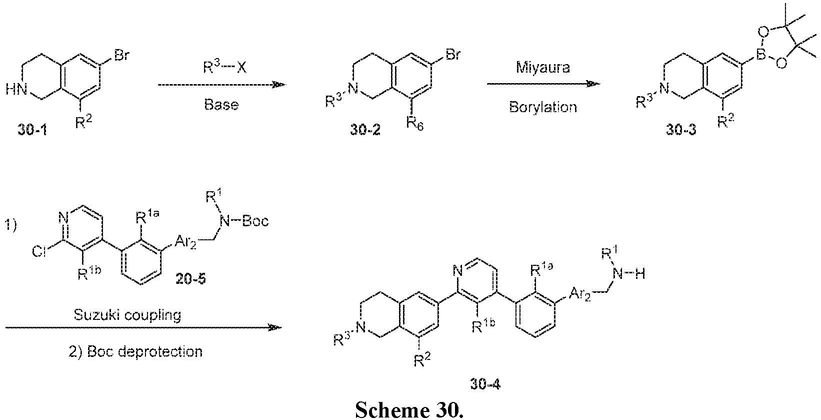

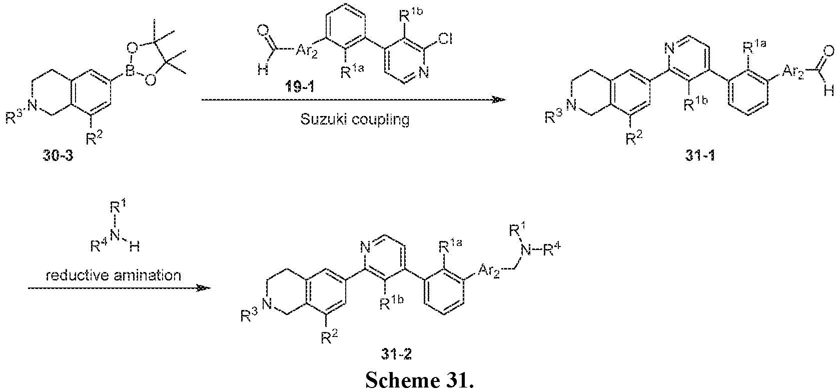

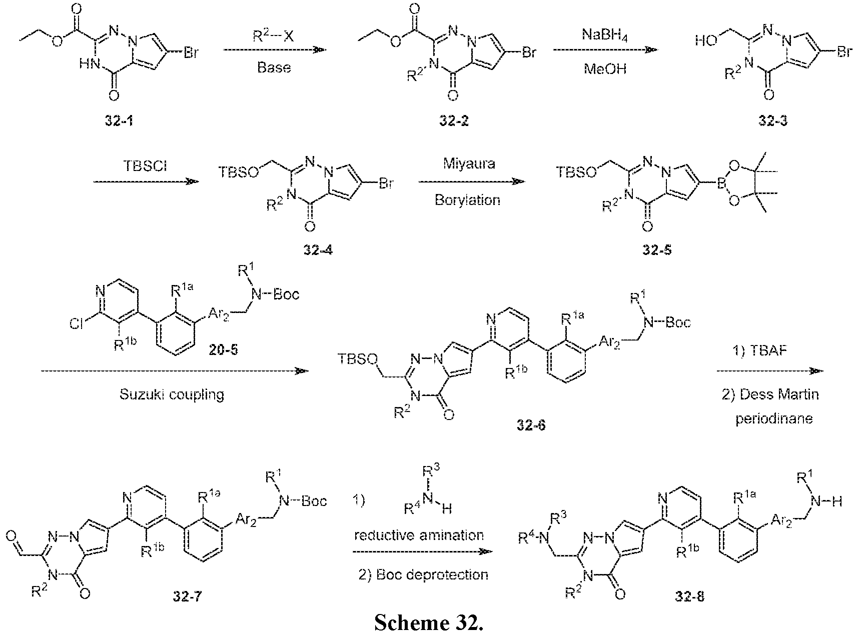

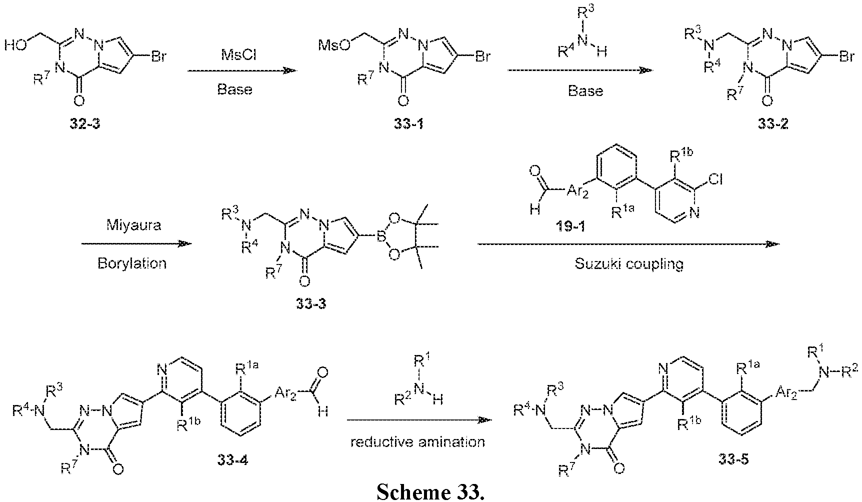

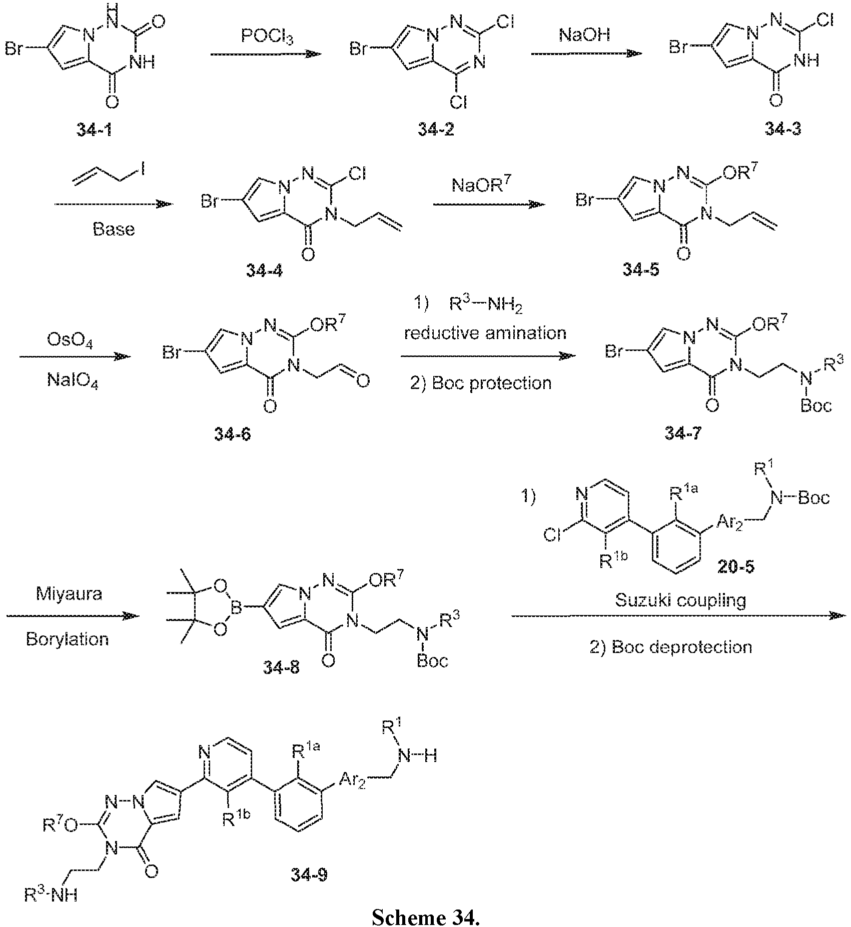

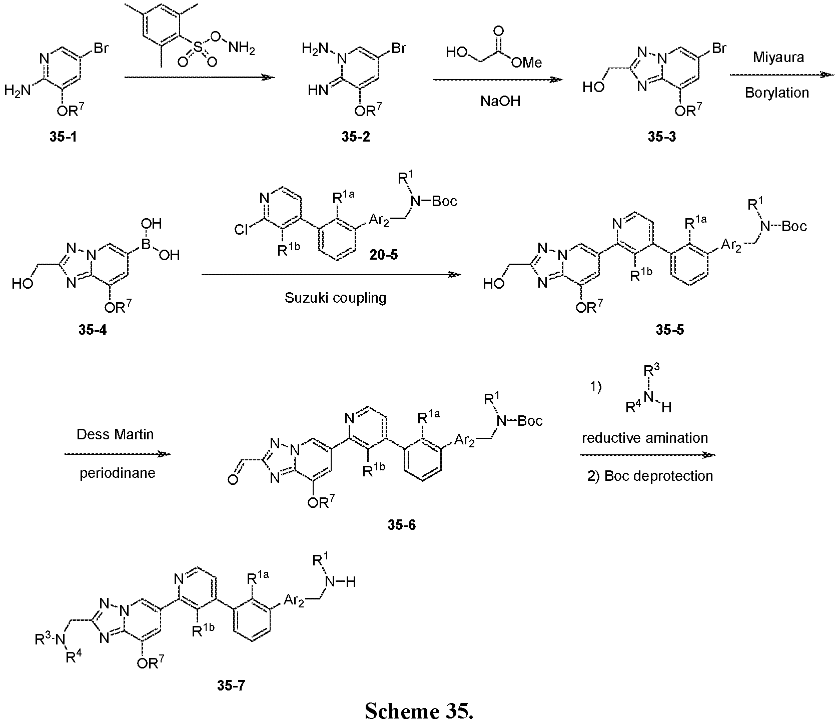

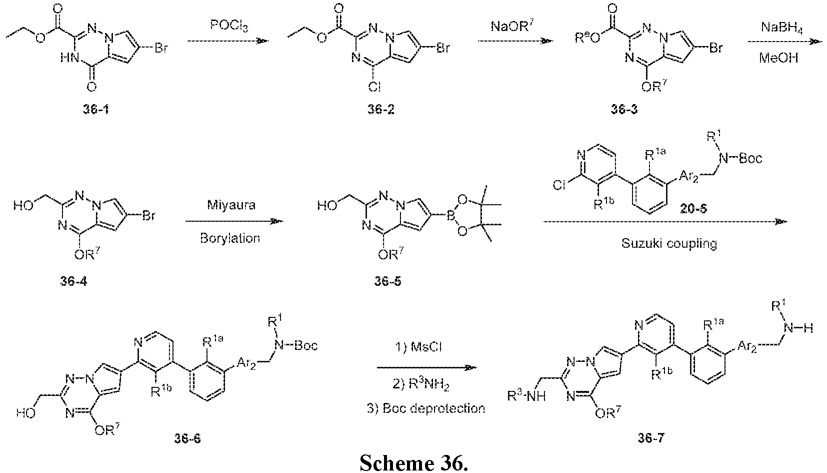

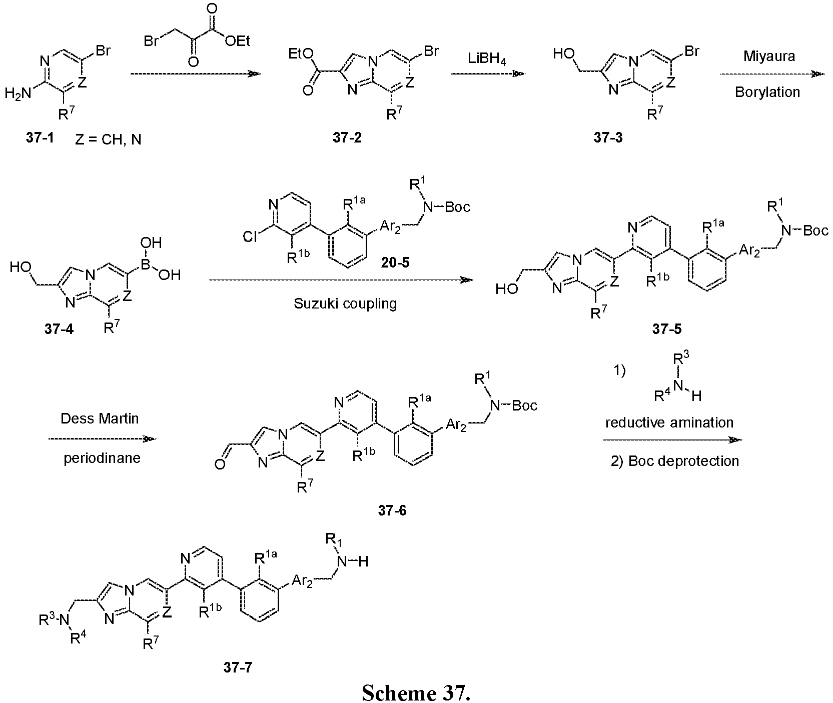

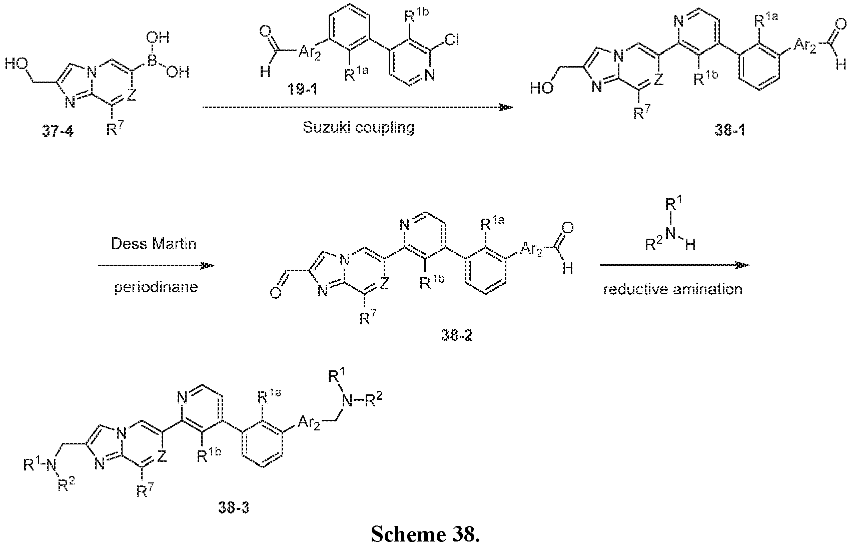

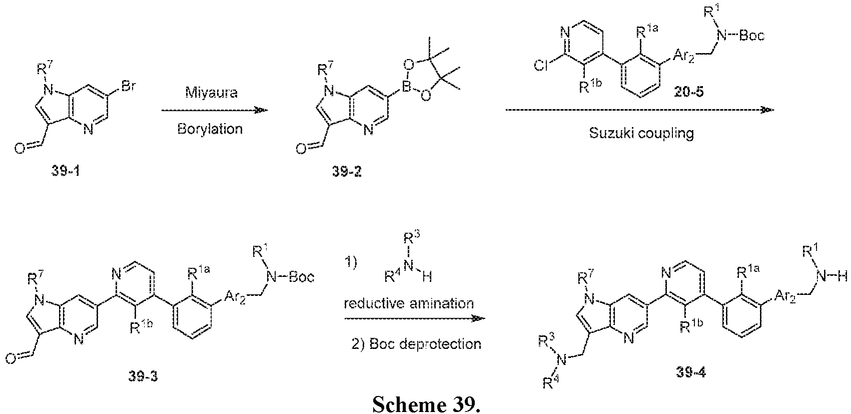

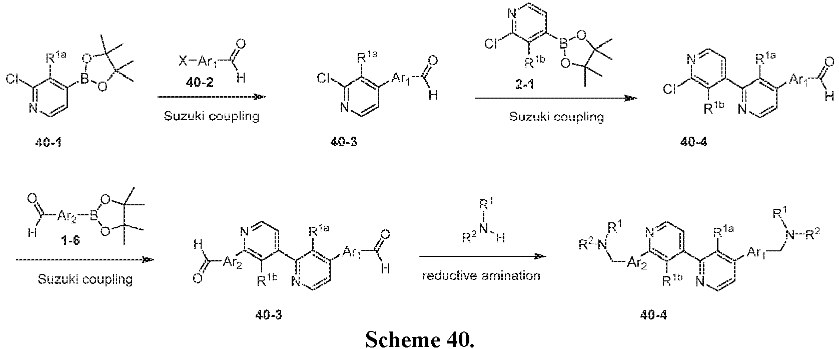

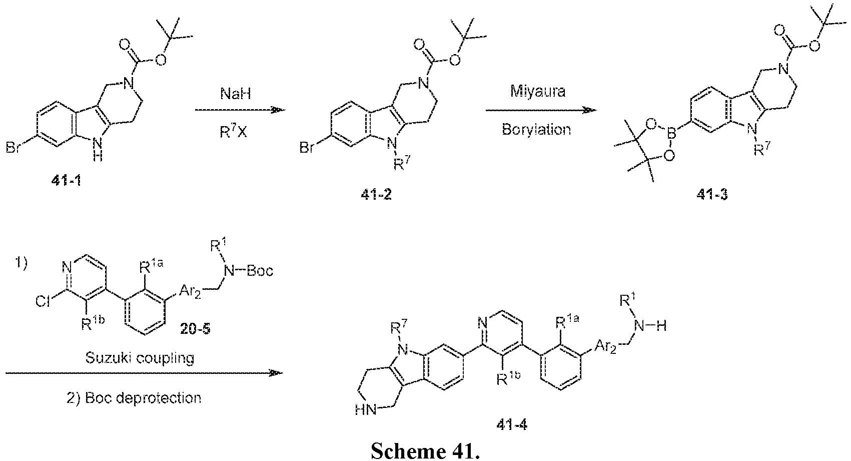

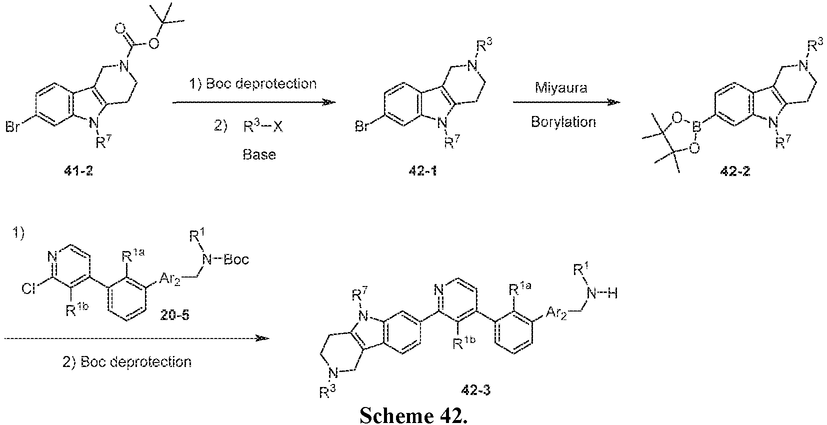

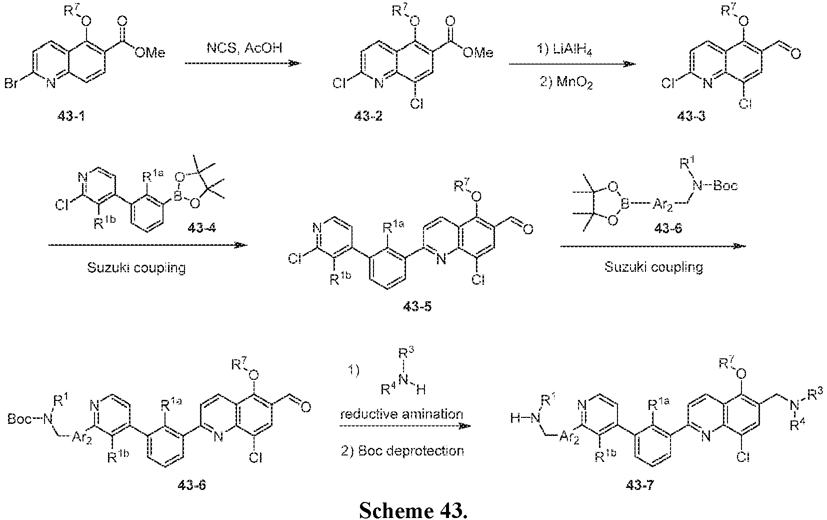

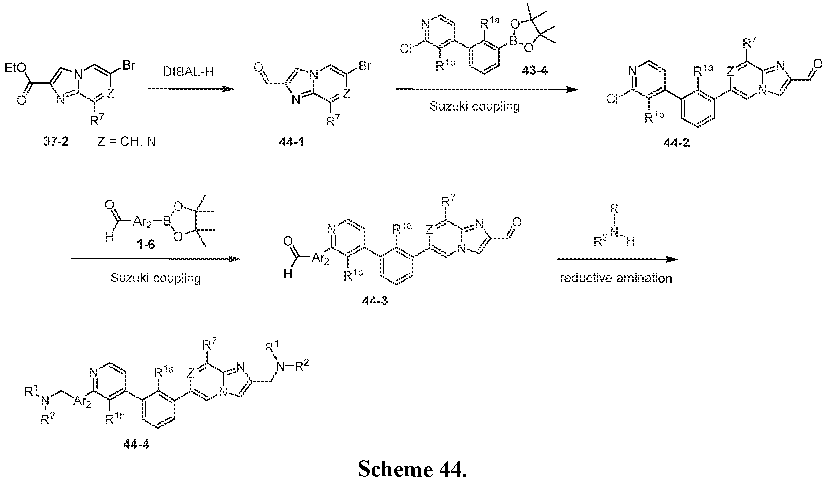

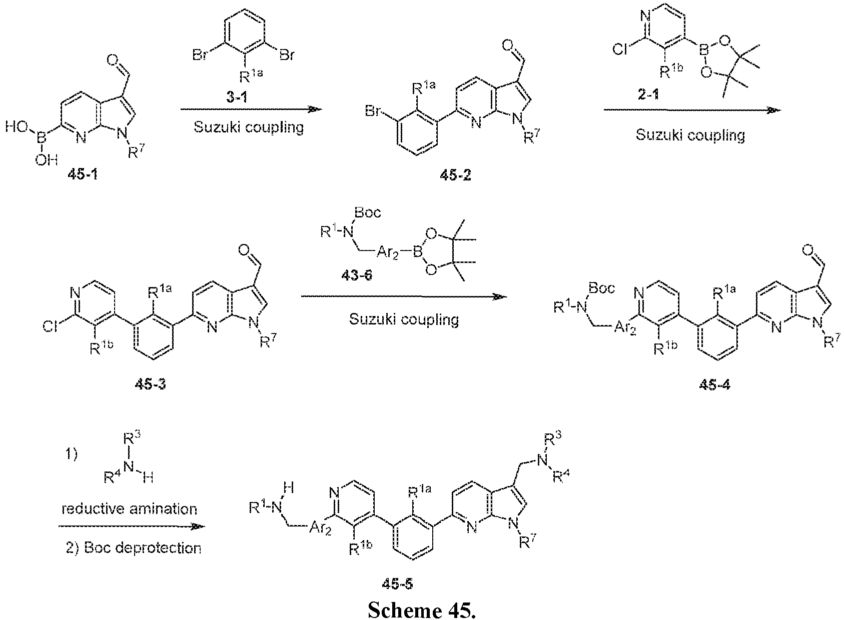

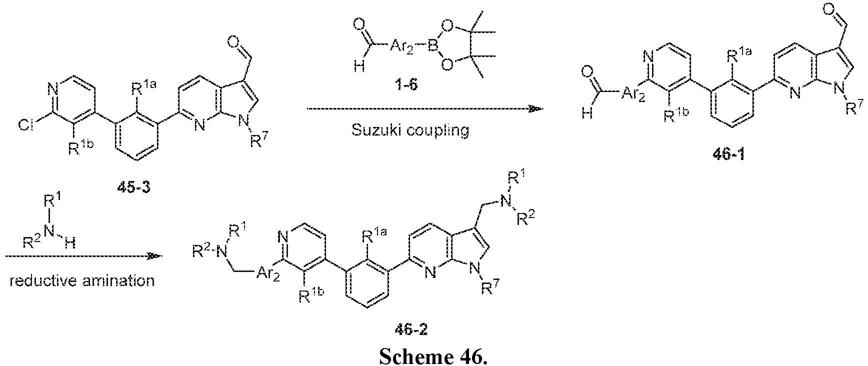

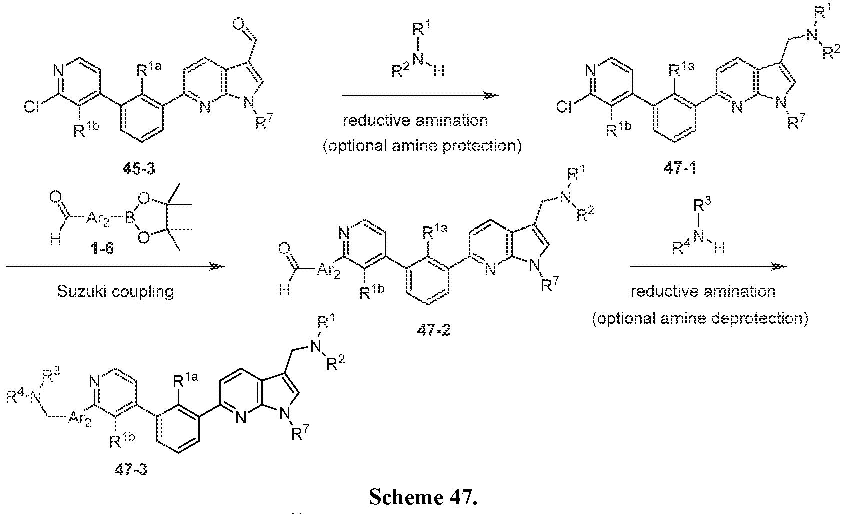

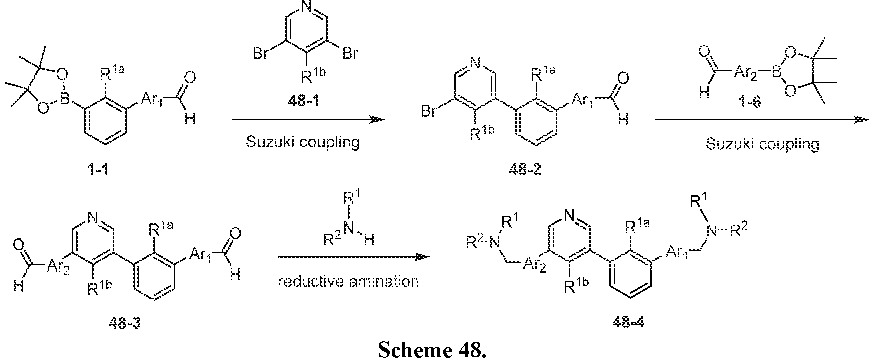

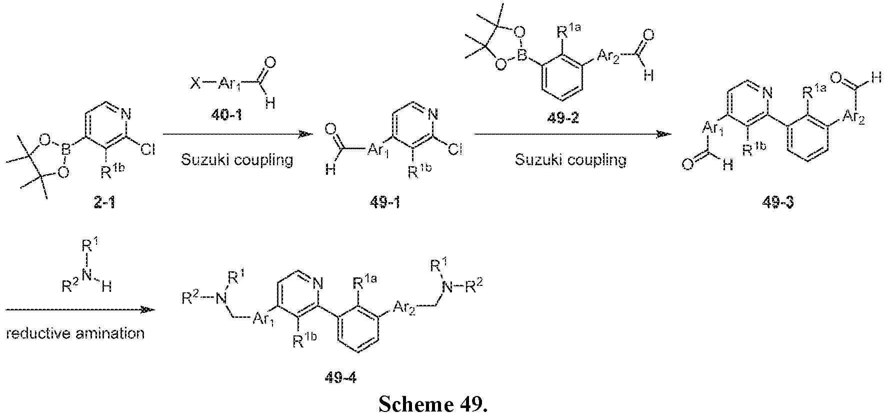

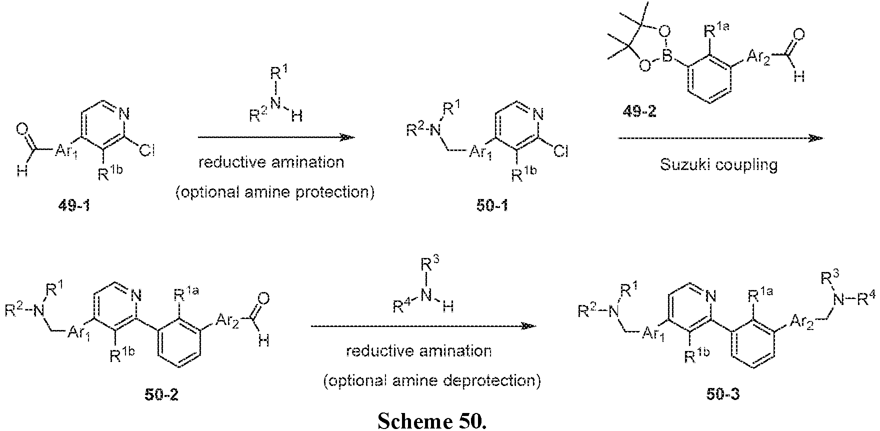

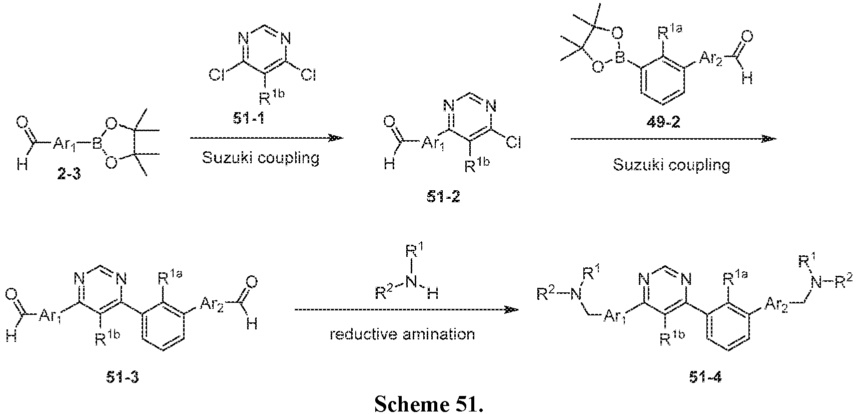

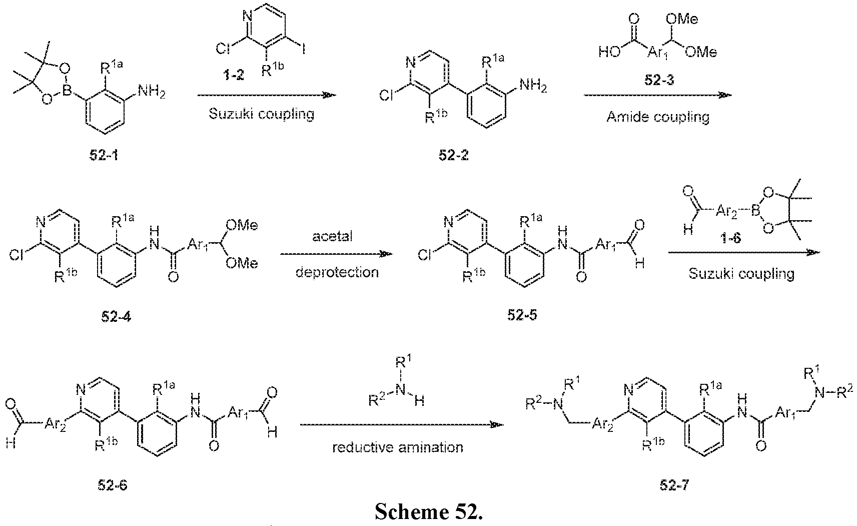

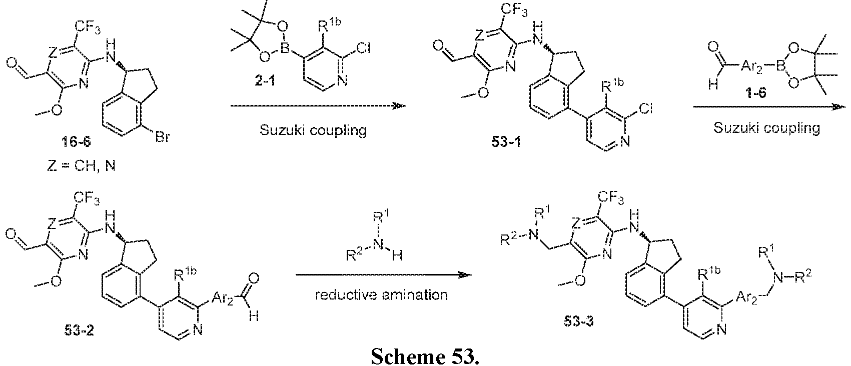

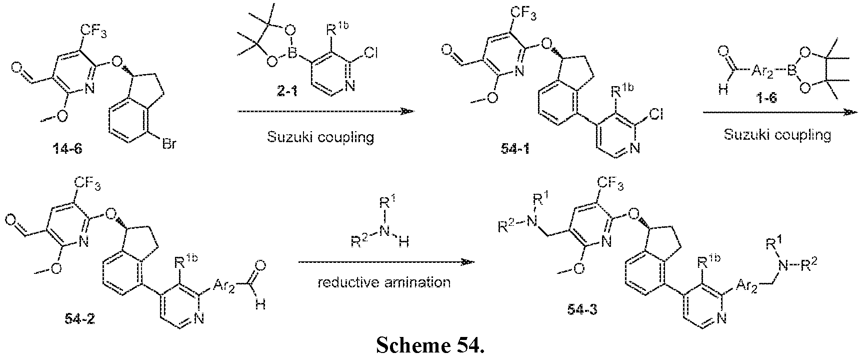

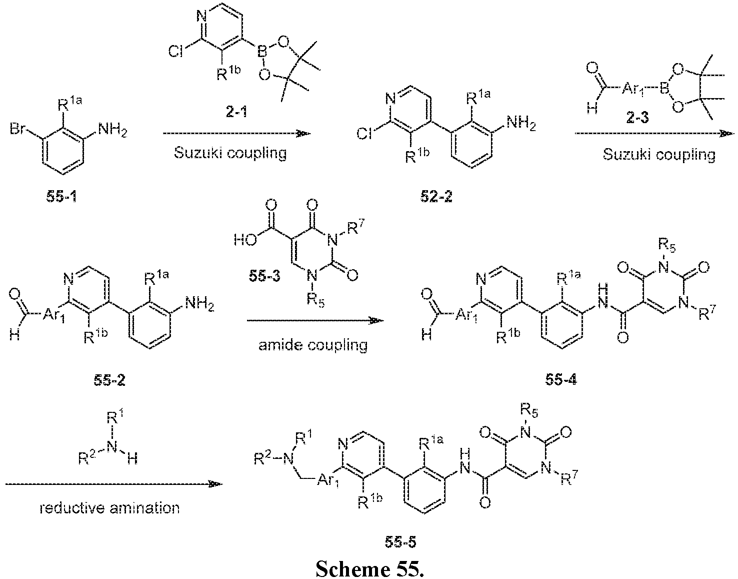

### Methods

The disclosure provides a method of treating, ameliorating, and/or preventing hepatitis virus infection in a subject. In certain embodiments, the infection comprises hepatitis B virus (HBV) and/or hepatitis D virus (HDV) infection. In other embodiments, the infection comprises hepatitis B virus (HBV) infection. In yet other embodiments, the infection comprises hepatitis D virus (HDV) infection. In yet other embodiments, the method comprises administering to the subject in need thereof a therapeutically effective amount of at least one compound of the disclosure. In yet other embodiments, the compound of the disclosure is the only antiviral agent administered to the subject. In yet other embodiments, the at least one compound is administered to the subject in a pharmaceutically acceptable composition. In yet other embodiments, the subject is further administered at least one additional agent useful for treating the hepatitis virus infection. In yet other embodiments, the at least one additional agent comprises at least one selected from the group consisting of reverse transcriptase inhibitors; capsid inhibitors; cccDNA formation inhibitors; RNA destabilizers; oligomeric nucleotides targeted against the HBV genome; immunostimulators; GalNAc-siRNA conjugates targeted against an HBV gene transcript; and therapeutic vaccines. In yet other embodiments, the subject is co-administered the at least one compound and the at least one additional agent. In yet other embodiments, the at least one compound and the at least one additional agent are coformulated.

The disclosure further provides a method of treating, ameliorating, and/or preventing cancer in a subject. In certain embodiments, the method comprises administering to the subject in need thereof a therapeutically effective amount of at least one compound of the disclosure. In other embodiments, the compound of the disclosure is the only anticancer agent administered to the subject. In yet other embodiments, the at least one compound is administered to the subject in a pharmaceutically acceptable composition. In yet other embodiments, the subject is further administered at least one additional agent or therapy useful for treating, ameliorating, or preventing the cancer. In yet other embodiments, the additional anticancer agent or therapy comprises nivolumab, pembrolizumab, atezolizumab, ipilimumab, chemotherapy, radiation therapy, and/or resection therapy. In yet other embodiments, the additional anticancer agent or therapy comprises rituxan, doxorubicin, gemcitabine, nivolumab, pembrolizumab, and/or ipilimumab.

In certain embodiments, the cancer is amenable to treatment by inhibiting PD-1, PD-L1 or the PD-1/PD-L1 interaction. In other embodiments, the cancer is at least one of pancreatic cancer, bladder cancer, colorectal cancer, breast cancer, prostate cancer, renal cancer, hepatocellular cancer, lung cancer, ovarian cancer, cervical cancer, gastric cancer, esophageal cancer, head and neck cancer, melanoma, neuroendocrine cancer, CNS cancer, brain cancer, bone cancer, soft tissue sarcoma, non-small cell lung cancer, small-cell lung cancer, or colon cancer. In yet other embodiments, the cancer is at least one of lymphoma, multiple myeloma, or leukemia. In yet other embodiments, the cancer is at least one of acute lymphocytic leukemia (ALL), acute myeloid leukemia (AML), chronic lymphocytic leukemia (CLL), small lymphocytic lymphoma (SLL), myelodysplastic syndrome (MDS), myeloproliferative disease (MPD), chronic myeloid leukemia (CML), multiple myeloma (MM), non-Hodgkin's lymphoma (NHL), mantle cell lymphoma (MCL), follicular lymphoma, Waldestrom's macroglobulinemia (WM), T-cell lymphoma, B-cell lymphoma and diffuse large B-cell lymphoma (DLBCL).

In certain embodiments, the subject is a mammal. In other embodiments, the mammal is a human.

### Pharmaceutical Compositions and Formulations

The disclosure provides pharmaceutical compositions comprising at least one compound of the disclosure or a salt or solvate thereof, which are useful to practice methods of the disclosure. Such a pharmaceutical composition may consist of at least one compound of the disclosure or a salt or solvate thereof, in a form suitable for administration to a subject, or the pharmaceutical composition may comprise at least one compound of the disclosure or a salt or solvate thereof, and one or more pharmaceutically acceptable carriers, one or more additional ingredients, or some combination of these. At least one compound of the disclosure may be present in the pharmaceutical composition in the form of a physiologically acceptable salt, such as in combination with a physiologically acceptable cation or anion, as is well known in the art.

In certain embodiments, the pharmaceutical compositions useful for practicing the method of the disclosure may be administered to deliver a dose of between 1 ng/kg/day and 100 mg/kg/day. In other embodiments, the pharmaceutical compositions useful for practicing the disclosure may be administered to deliver a dose of between 1 ng/kg/day and 1,000 mg/kg/day.

The relative amounts of the active ingredient, the pharmaceutically acceptable carrier, and any additional ingredients in a pharmaceutical composition of the disclosure will vary, depending upon the identity, size, and condition of the subject treated and further depending upon the route by which the composition is to be administered. By way of example, the composition may comprise between 0.1% and 100% (w/w) active ingredient.

Pharmaceutical compositions that are useful in the methods of the disclosure may be suitably developed for nasal, inhalational, oral, rectal, vaginal, pleural, peritoneal, parenteral, topical, transdermal, pulmonary, intranasal, buccal, ophthalmic, epidural, intrathecal, intravenous or another route of administration. A composition useful within the methods of the disclosure may be directly administered to the brain, the brainstem, or any other part of the central nervous system of a mammal or bird. Other contemplated formulations include projected nanoparticles, microspheres, liposomal preparations, coated particles, polymer conjugates, resealed erythrocytes containing the active ingredient, and immunologically-based formulations.

In certain embodiments, the compositions of the disclosure are part of a pharmaceutical matrix, which allows for manipulation of insoluble materials and improvement of the bioavailability thereof, development of controlled or sustained release products, and generation of homogeneous compositions. By way of example, a pharmaceutical matrix may be prepared using hot melt extrusion, solid solutions, solid dispersions, size reduction technologies, molecular complexes (e.g., cyclodextrins, and others), microparticulate, and particle and formulation coating processes. Amorphous or crystalline phases may be used in such processes.

The route(s) of administration will be readily apparent to the skilled artisan and will depend upon any number of factors including the type and severity of the disease being treated, the type and age of the veterinary or human patient being treated, and the like.

The formulations of the pharmaceutical compositions described herein may be prepared by any method known or hereafter developed in the art of pharmacology and pharmaceutics. In general, such preparatory methods include the step of bringing the active ingredient into association with a carrier or one or more other accessory ingredients, and then, if necessary or desirable, shaping or packaging the product into a desired single-dose or multi-dose unit.

As used herein, a "unit dose" is a discrete amount of the pharmaceutical composition comprising a predetermined amount of the active ingredient. The amount of the active ingredient is generally equal to the dosage of the active ingredient that would be administered to a subject or a convenient fraction of such a dosage such as, for example, one-half or one-third of such a dosage. The unit dosage form may be for a single daily dose or one of multiple daily doses (*e.g.*, about 1 to 4 or more times per day). When multiple daily doses are used, the unit dosage form may be the same or different for each dose.

Although the descriptions of pharmaceutical compositions provided herein are principally directed to pharmaceutical compositions suitable for ethical administration to humans, it will be understood by the skilled artisan that such compositions are generally suitable for administration to animals of all sorts. Modification of pharmaceutical compositions suitable for administration to humans in order to render the compositions suitable for administration to various animals is well understood, and the ordinarily skilled veterinary pharmacologist can design and perform such modification with merely ordinary, if any, experimentation. Subjects to which administration of the pharmaceutical compositions of the disclosure is contemplated include, but are not limited to, humans and other primates, mammals including commercially relevant mammals such as cattle, pigs, horses, sheep, cats, and dogs.

In certain embodiments, the compositions of the disclosure are formulated using one or more pharmaceutically acceptable excipients or carriers. In certain embodiments, the pharmaceutical compositions of the disclosure comprise a therapeutically effective amount of at least one compound of the disclosure and a pharmaceutically acceptable carrier. Pharmaceutically acceptable carriers, which are useful, include, but are not limited to, glycerol, water, saline, ethanol, recombinant human albumin (*e.g*., RECOMBUMIN^{®}), solubilized gelatins (*e.g.*, GELOFUSINE^{®}), and other pharmaceutically acceptable salt solutions such as phosphates and salts of organic acids. Examples of these and other pharmaceutically acceptable carriers are described in Remington's Pharmaceutical Sciences (1991, Mack Publication Co., New Jersey).

The carrier may be a solvent or dispersion medium containing, for example, water, ethanol, polyol (for example, glycerol, propylene glycol, and liquid polyethylene glycol, and the like), recombinant human albumin, solubilized gelatins, suitable mixtures thereof, and vegetable oils. The proper fluidity may be maintained, for example, by the use of a coating such as lecithin, by the maintenance of the required particle size in the case of dispersion and by the use of surfactants. Prevention of the action of microorganisms may be achieved by various antibacterial and antifungal agents, for example, parabens, chlorobutanol, phenol, ascorbic acid, thimerosal, and the like. In many cases, isotonic agents, for example, sugars, sodium chloride, or polyalcohols such as mannitol and sorbitol, are included in the composition. Prolonged absorption of the injectable compositions may be brought about by including in the composition an agent that delays absorption, for example, aluminum monostearate or gelatin.

Formulations may be employed in admixtures with conventional excipients, *i.e.,* pharmaceutically acceptable organic or inorganic carrier substances suitable for oral, parenteral, nasal, inhalational, intravenous, subcutaneous, transdermal enteral, or any other suitable mode of administration, known to the art. The pharmaceutical preparations may be sterilized and if desired mixed with auxiliary agents, *e.g.*, lubricants, preservatives, stabilizers, wetting agents, emulsifiers, salts for influencing osmotic pressure buffers, coloring, flavoring and/or fragrance-conferring substances and the like. They may also be combined where desired with other active agents, *e.g.,* other analgesic, anxiolytics or hypnotic agents. As used herein, "additional ingredients" include, but are not limited to, one or more ingredients that may be used as a pharmaceutical carrier.

The composition of the disclosure may comprise a preservative from about 0.005% to 2.0% by total weight of the composition. The preservative is used to prevent spoilage in the case of exposure to contaminants in the environment. Examples of preservatives useful in accordance with the disclosure include but are not limited to those selected from the group consisting of benzyl alcohol, sorbic acid, parabens, imidurea and combinations thereof. One such preservative is a combination of about 0.5% to 2.0% benzyl alcohol and 0.05% to 0.5% sorbic acid. The composition may include an antioxidant and a chelating agent which inhibit the degradation of the compound. Antioxidants for some compounds are BHT, BHA, alpha-tocopherol and ascorbic acid in the exemplary range of about 0.01% to 0.3%, or BHT in the range of 0.03% to 0.1% by weight by total weight of the composition. The chelating agent may be present in an amount of from 0.01% to 0.5% by weight by total weight of the composition. Exemplary chelating agents include edetate salts (*e.g*. disodium edetate) and citric acid in the weight range of about 0.01% to 0.20%, or in the range of 0.02% to 0.10% by weight by total weight of the composition. The chelating agent is useful for chelating metal ions in the composition that may be detrimental to the shelf life of the formulation. While BHT and disodium edetate are exemplary antioxidant and chelating agent, respectively, for some compounds, other suitable and equivalent antioxidants and chelating agents may be substituted therefore as would be known to those skilled in the art.

Liquid suspensions may be prepared using conventional methods to achieve suspension of the active ingredient in an aqueous or oily vehicle. Aqueous vehicles include, for example, water, and isotonic saline. Oily vehicles include, for example, almond oil, oily esters, ethyl alcohol, vegetable oils such as arachis, olive, sesame, or coconut oil, fractionated vegetable oils, and mineral oils such as liquid paraffin. Liquid suspensions may further comprise one or more additional ingredients including, but not limited to, suspending agents, dispersing or wetting agents, emulsifying agents, demulcents, preservatives, buffers, salts, flavorings, coloring agents, and sweetening agents. Oily suspensions may further comprise a thickening agent. Known suspending agents include, but are not limited to, sorbitol syrup, hydrogenated edible fats, sodium alginate, polyvinylpyrrolidone, gum tragacanth, gum acacia, and cellulose derivatives such as sodium carboxymethylcellulose, methylcellulose, hydroxypropylmethyl cellulose. Known dispersing or wetting agents include, but are not limited to, naturally-occurring phosphatides such as lecithin, condensation products of an alkylene oxide with a fatty acid, with a long chain aliphatic alcohol, with a partial ester derived from a fatty acid and a hexitol, or with a partial ester derived from a fatty acid and a hexitol anhydride (*e.g*., polyoxyethylene stearate, heptadecaethyleneoxycetanol, polyoxyethylene sorbitol monooleate, and polyoxyethylene sorbitan monooleate, respectively). Known emulsifying agents include, but are not limited to, lecithin, acacia, and ionic or non ionic surfactants. Known preservatives include, but are not limited to, methyl, ethyl, or n-propyl para-hydroxybenzoates, ascorbic acid, and sorbic acid. Known sweetening agents include, for example, glycerol, propylene glycol, sorbitol, sucrose, and saccharin.

Liquid solutions of the active ingredient in aqueous or oily solvents may be prepared in substantially the same manner as liquid suspensions, the primary difference being that the active ingredient is dissolved, rather than suspended in the solvent. As used herein, an "oily" liquid is one which comprises a carbon-containing liquid molecule and which exhibits a less polar character than water. Liquid solutions of the pharmaceutical composition of the disclosure may comprise each of the components described with regard to liquid suspensions, it being understood that suspending agents will not necessarily aid dissolution of the active ingredient in the solvent. Aqueous solvents include, for example, water, and isotonic saline. Oily solvents include, for example, almond oil, oily esters, ethyl alcohol, vegetable oils such as arachis, olive, sesame, or coconut oil, fractionated vegetable oils, and mineral oils such as liquid paraffin.

Powdered and granular formulations of a pharmaceutical preparation of the disclosure may be prepared using known methods. Such formulations may be administered directly to a subject, used, for example, to form tablets, to fill capsules, or to prepare an aqueous or oily suspension or solution by addition of an aqueous or oily vehicle thereto. Each of these formulations may further comprise one or more of dispersing or wetting agent, a suspending agent, ionic and non-ionic surfactants, and a preservative. Additional excipients, such as fillers and sweetening, flavoring, or coloring agents, may also be included in these formulations.

A pharmaceutical composition of the disclosure may also be prepared, packaged, or sold in the form of oil-in-water emulsion or a water-in-oil emulsion. The oily phase may be a vegetable oil such as olive or arachis oil, a mineral oil such as liquid paraffin, or a combination of these. Such compositions may further comprise one or more emulsifying agents such as naturally occurring gums such as gum acacia or gum tragacanth, naturally-occurring phosphatides such as soybean or lecithin phosphatide, esters or partial esters derived from combinations of fatty acids and hexitol anhydrides such as sorbitan monooleate, and condensation products of such partial esters with ethylene oxide such as polyoxyethylene sorbitan monooleate. These emulsions may also contain additional ingredients including, for example, sweetening or flavoring agents.

Methods for impregnating or coating a material with a chemical composition are known in the art, and include, but are not limited to methods of depositing or binding a chemical composition onto a surface, methods of incorporating a chemical composition into the structure of a material during the synthesis of the material (*i*.*e*., such as with a physiologically degradable material), and methods of absorbing an aqueous or oily solution or suspension into an absorbent material, with or without subsequent drying. Methods for mixing components include physical milling, the use of pellets in solid and suspension formulations and mixing in a transdermal patch, as known to those skilled in the art.

### Administration/Dosing

The regimen of administration may affect what constitutes an effective amount. The therapeutic formulations may be administered to the patient either prior to or after the onset of a disease or disorder. Further, several divided dosages, as well as staggered dosages may be administered daily or sequentially, or the dose may be continuously infused, or may be a bolus injection. Further, the dosages of the therapeutic formulations may be proportionally increased or decreased as indicated by the exigencies of the therapeutic or prophylactic situation.

Administration of the compositions of the present disclosure to a patient, such as a mammal, such as a human, may be carried out using known procedures, at dosages and for periods of time effective to treat a disease or disorder contemplated herein. An effective amount of the therapeutic compound necessary to achieve a therapeutic effect may vary according to factors such as the activity of the particular compound employed; the time of administration; the rate of excretion of the compound; the duration of the treatment; other drugs, compounds or materials used in combination with the compound; the state of the disease or disorder, age, sex, weight, condition, general health and prior medical history of the patient being treated, and like factors well-known in the medical arts. Dosage regimens may be adjusted to provide the optimum therapeutic response. For example, several divided doses may be administered daily or the dose may be proportionally reduced as indicated by the exigencies of the therapeutic situation. A non-limiting example of an effective dose range for a therapeutic compound of the disclosure is from about 0.01 mg/kg to 100 mg/kg of body weight/per day. One of ordinary skill in the art would be able to study the relevant factors and make the determination regarding the effective amount of the therapeutic compound without undue experimentation.

The compound may be administered to an animal as frequently as several times daily, or it may be administered less frequently, such as once a day, once a week, once every two weeks, once a month, or even less frequently, such as once every several months or even once a year or less. It is understood that the amount of compound dosed per day may be administered, in non-limiting examples, every day, every other day, every 2 days, every 3 days, every 4 days, or every 5 days. For example, with every other day administration, a 5 mg per day dose may be initiated on Monday with a first subsequent 5 mg per day dose administered on Wednesday, a second subsequent 5 mg per day dose administered on Friday, and so on. The frequency of the dose is readily apparent to the skilled artisan and depends upon a number of factors, such as, but not limited to, type and severity of the disease being treated, and type and age of the animal.

Actual dosage levels of the active ingredients in the pharmaceutical compositions of this disclosure may be varied so as to obtain an amount of the active ingredient that is effective to achieve the desired therapeutic response for a particular patient, composition, and mode of administration, without being toxic to the patient.

A medical doctor, *e.g.,* physician or veterinarian, having ordinary skill in the art may readily determine and prescribe the effective amount of the pharmaceutical composition required. For example, the physician or veterinarian could start doses of the compounds of the disclosure employed in the pharmaceutical composition at levels lower than that required in order to achieve the desired therapeutic effect and gradually increase the dosage until the desired effect is achieved.

In particular embodiments, it is especially advantageous to formulate the compound in dosage unit form for ease of administration and uniformity of dosage. Dosage unit form as used herein refers to physically discrete units suited as unitary dosages for the patients to be treated; each unit containing a predetermined quantity of therapeutic compound calculated to produce the desired therapeutic effect in association with the required pharmaceutical vehicle. The dosage unit forms of the disclosure are dictated by and directly dependent on (a) the unique characteristics of the therapeutic compound and the particular therapeutic effect to be achieved, and (b) the limitations inherent in the art of compounding/formulating such a therapeutic compound for the treatment of a disease or disorder in a patient.

In certain embodiments, the compositions of the disclosure are administered to the patient in dosages that range from one to five times per day or more. In other embodiments, the compositions of the disclosure are administered to the patient in range of dosages that include, but are not limited to, once every day, every two days, every three days to once a week, and once every two weeks. It will be readily apparent to one skilled in the art that the frequency of administration of the various combination compositions of the disclosure will vary from subject to subject depending on many factors including, but not limited to, age, disease or disorder to be treated, gender, overall health, and other factors. Thus, the disclosure should not be construed to be limited to any particular dosage regime and the precise dosage and composition to be administered to any patient will be determined by the attending physician taking all other factors about the patient into account.

Compounds of the disclosure for administration may be in the range of from about 1 µg to about 7,500 mg, about 20 µg to about 7,000 mg, about 40 µg to about 6,500 mg, about 80 µg to about 6,000 mg, about 100 µg to about 5,500 mg, about 200 µg to about 5,000 mg, about 400 µg to about 4,000 mg, about 800 µg to about 3,000 mg, about 1 mg to about 2,500 mg, about 2 mg to about 2,000 mg, about 5 mg to about 1,000 mg, about 10 mg to about 750 mg, about 20 mg to about 600 mg, about 30 mg to about 500 mg, about 40 mg to about 400 mg, about 50 mg to about 300 mg, about 60 mg to about 250 mg, about 70 mg to about 200 mg, about 80 mg to about 150 mg, and any and all whole or partial increments there-in-between.

In some embodiments, the dose of a compound of the disclosure is from about 0.5 µg and about 5,000 mg. In some embodiments, a dose of a compound of the disclosure used in compositions described herein is less than about 5,000 mg, or less than about 4,000 mg, or less than about 3,000 mg, or less than about 2,000 mg, or less than about 1,000 mg, or less than about 800 mg, or less than about 600 mg, or less than about 500 mg, or less than about 200 mg, or less than about 50 mg. Similarly, in some embodiments, a dose of a second compound as described herein is less than about 1,000 mg, or less than about 800 mg, or less than about 600 mg, or less than about 500 mg, or less than about 400 mg, or less than about 300 mg, or less than about 200 mg, or less than about 100 mg, or less than about 50 mg, or less than about 40 mg, or less than about 30 mg, or less than about 25 mg, or less than about 20 mg, or less than about 15 mg, or less than about 10 mg, or less than about 5 mg, or less than about 2 mg, or less than about 1 mg, or less than about 0.5 mg, and any and all whole or partial increments thereof.

In certain embodiments, the present disclosure is directed to a packaged pharmaceutical composition comprising a container holding a therapeutically effective amount of a compound of the disclosure, alone or in combination with a second pharmaceutical agent; and instructions for using the compound to treat, prevent, or reduce one or more symptoms of a disease or disorder in a patient.

The term "container" includes any receptacle for holding the pharmaceutical composition or for managing stability or water uptake. For example, in certain embodiments, the container is the packaging that contains the pharmaceutical composition, such as liquid (solution and suspension), semisolid, lyophilized solid, solution and powder or lyophilized formulation present in dual chambers. In other embodiments, the container is not the packaging that contains the pharmaceutical composition, *i.e.,* the container is a receptacle, such as a box or vial that contains the packaged pharmaceutical composition or unpackaged pharmaceutical composition and the instructions for use of the pharmaceutical composition. Moreover, packaging techniques are well known in the art. It should be understood that the instructions for use of the pharmaceutical composition may be contained on the packaging containing the pharmaceutical composition, and as such the instructions form an increased functional relationship to the packaged product. However, it should be understood that the instructions may contain information pertaining to the compound's ability to perform its intended function, *e.g*., treating, preventing, or reducing a disease or disorder in a patient.

### Administration

Routes of administration of any of the compositions of the disclosure include inhalational, oral, nasal, rectal, parenteral, sublingual, transdermal, transmucosal (*e.g*., sublingual, lingual, (trans)buccal, (trans)urethral, vaginal (*e.g*., trans- and perivaginally), (intra)nasal, and (trans)rectal), intravesical, intrapulmonary, intraduodenal, intragastrical, intrathecal, epidural, intrapleural, intraperitoneal, subcutaneous, intramuscular, intradermal, intra-arterial, intravenous, intrabronchial, inhalation, and topical administration.

Suitable compositions and dosage forms include, for example, tablets, capsules, caplets, pills, gel caps, troches, emulsions, dispersions, suspensions, solutions, syrups, granules, beads, transdermal patches, gels, powders, pellets, magmas, lozenges, creams, pastes, plasters, lotions, discs, suppositories, liquid sprays for nasal or oral administration, dry powder or aerosolized formulations for inhalation, compositions and formulations for intravesical administration and the like. It should be understood that the formulations and compositions that would be useful in the present disclosure are not limited to the particular formulations and compositions that are described herein.

### Oral Administration

For oral application, particularly suitable are tablets, dragees, liquids, drops, capsules, caplets and gelcaps. Other formulations suitable for oral administration include, but are not limited to, a powdered or granular formulation, an aqueous or oily suspension, an aqueous or oily solution, a paste, a gel, toothpaste, a mouthwash, a coating, an oral rinse, or an emulsion. The compositions intended for oral use may be prepared according to any method known in the art and such compositions may contain one or more agents selected from the group consisting of inert, non-toxic, generally recognized as safe (GRAS) pharmaceutically excipients which are suitable for the manufacture of tablets. Such excipients include, for example an inert diluent such as lactose; granulating and disintegrating agents such as cornstarch; binding agents such as starch; and lubricating agents such as magnesium stearate.

Tablets may be non-coated or they may be coated using known methods to achieve delayed disintegration in the gastrointestinal tract of a subject, thereby providing sustained release and absorption of the active ingredient. By way of example, a material such as glyceryl monostearate or glyceryl distearate may be used to coat tablets. Further by way of example, tablets may be coated using methods described in U.S. Patents Nos. 4,256,108; 4,160,452; and 4,265,874 to form osmotically controlled release tablets. Tablets may further comprise a sweetening agent, a flavoring agent, a coloring agent, a preservative, or some combination of these in order to provide for pharmaceutically elegant and palatable preparation. Hard capsules comprising the active ingredient may be made using a physiologically degradable composition, such as gelatin. The capsules comprise the active ingredient, and may further comprise additional ingredients including, for example, an inert solid diluent such as calcium carbonate, calcium phosphate, or kaolin.

Hard capsules comprising the active ingredient may be made using a physiologically degradable composition, such as gelatin. Such hard capsules comprise the active ingredient, and may further comprise additional ingredients including, for example, an inert solid diluent such as calcium carbonate, calcium phosphate, or kaolin.

Soft gelatin capsules comprising the active ingredient may be made using a physiologically degradable composition, such as gelatin from animal-derived collagen or from a hypromellose, a modified form of cellulose, and manufactured using optional mixtures of gelatin, water and plasticizers such as sorbitol or glycerol. Such soft capsules comprise the active ingredient, which may be mixed with water or an oil medium such as peanut oil, liquid paraffin, or olive oil.

For oral administration, the compounds of the disclosure may be in the form of tablets or capsules prepared by conventional means with pharmaceutically acceptable excipients such as binding agents; fillers; lubricants; disintegrates; or wetting agents. If desired, the tablets may be coated using suitable methods and coating materials such as OPADRY^{®} film coating systems available from Colorcon, West Point, Pa. (*e.g*., OPADRY^{®} OY Type, OYC Type, Organic Enteric OY-P Type, Aqueous Enteric OY-A Type, OY-PM Type and OPADRY^{®} White, 32K18400). It is understood that similar type of film coating or polymeric products from other companies may be used.

A tablet comprising the active ingredient may, for example, be made by compressing or molding the active ingredient, optionally with one or more additional ingredients. Compressed tablets may be prepared by compressing, in a suitable device, the active ingredient in a free-flowing form such as a powder or granular preparation, optionally mixed with one or more of a binder, a lubricant, an excipient, a surface active agent, and a dispersing agent. Molded tablets may be made by molding, in a suitable device, a mixture of the active ingredient, a pharmaceutically acceptable carrier, and at least sufficient liquid to moisten the mixture. Pharmaceutically acceptable excipients used in the manufacture of tablets include, but are not limited to, inert diluents, granulating and disintegrating agents, binding agents, and lubricating agents. Known dispersing agents include, but are not limited to, potato starch and sodium starch glycolate. Known surface-active agents include, but are not limited to, sodium lauryl sulphate. Known diluents include, but are not limited to, calcium carbonate, sodium carbonate, lactose, microcrystalline cellulose, calcium phosphate, calcium hydrogen phosphate, and sodium phosphate. Known granulating and disintegrating agents include, but are not limited to, corn starch and alginic acid. Known binding agents include, but are not limited to, gelatin, acacia, pre-gelatinized maize starch, polyvinylpyrrolidone, and hydroxypropyl methylcellulose. Known lubricating agents include, but are not limited to, magnesium stearate, stearic acid, silica, and talc.

Granulating techniques are well known in the pharmaceutical art for modifying starting powders or other particulate materials of an active ingredient. The powders are typically mixed with a binder material into larger permanent free-flowing agglomerates or granules referred to as a "granulation." For example, solvent-using "wet" granulation processes are generally characterized in that the powders are combined with a binder material and moistened with water or an organic solvent under conditions resulting in the formation of a wet granulated mass from which the solvent must then be evaporated.

Melt granulation generally consists in the use of materials that are solid or semi-solid at room temperature (*i.e.,* having a relatively low softening or melting point range) to promote granulation of powdered or other materials, essentially in the absence of added water or other liquid solvents. The low melting solids, when heated to a temperature in the melting point range, liquefy to act as a binder or granulating medium. The liquefied solid spreads itself over the surface of powdered materials with which it is contacted, and on cooling, forms a solid granulated mass in which the initial materials are bound together. The resulting melt granulation may then be provided to a tablet press or be encapsulated for preparing the oral dosage form. Melt granulation improves the dissolution rate and bioavailability of an active (*i.e.,* drug) by forming a solid dispersion or solid solution.

U.S. Patent No. 5,169,645 discloses directly compressible wax-containing granules having improved flow properties. The granules are obtained when waxes are admixed in the melt with certain flow improving additives, followed by cooling and granulation of the admixture. In certain embodiments, only the wax itself melts in the melt combination of the wax(es) and additives(s), and in other cases both the wax(es) and the additives(s) will melt.

The present disclosure also includes a multi-layer tablet comprising a layer providing for the delayed release of one or more compounds useful within the methods of the disclosure, and a further layer providing for the immediate release of one or more compounds useful within the methods of the disclosure. Using a wax/pH-sensitive polymer mix, a gastric insoluble composition may be obtained in which the active ingredient is entrapped, ensuring its delayed release.

Liquid preparation for oral administration may be in the form of solutions, syrups or suspensions. The liquid preparations may be prepared by conventional means with pharmaceutically acceptable additives such as suspending agents (*e.g.*, sorbitol syrup, methyl cellulose or hydrogenated edible fats); emulsifying agent (*e.g.*, lecithin or acacia); nonaqueous vehicles (*e.g.,* almond oil, oily esters or ethyl alcohol); and preservatives (*e.g.,* methyl or propyl para-hydroxy benzoates or sorbic acid). Liquid formulations of a pharmaceutical composition of the disclosure which are suitable for oral administration may be prepared, packaged, and sold either in liquid form or in the form of a dry product intended for reconstitution with water or another suitable vehicle prior to use.

### Parenteral Administration

As used herein, "parenteral administration" of a pharmaceutical composition includes any route of administration characterized by physical breaching of a tissue of a subject and administration of the pharmaceutical composition through the breach in the tissue. Parenteral administration thus includes, but is not limited to, administration of a pharmaceutical composition by injection of the composition, by application of the composition through a surgical incision, by application of the composition through a tissue-penetrating non-surgical wound, and the like. In particular, parenteral administration is contemplated to include, but is not limited to, subcutaneous, intravenous, intraperitoneal, intramuscular, intrasternal injection, and kidney dialytic infusion techniques.

Formulations of a pharmaceutical composition suitable for parenteral administration comprise the active ingredient combined with a pharmaceutically acceptable carrier, such as sterile water or sterile isotonic saline. Such formulations may be prepared, packaged, or sold in a form suitable for bolus administration or for continuous administration. Injectable formulations may be prepared, packaged, or sold in unit dosage form, such as in ampules or in multidose containers containing a preservative. Injectable formulations may also be prepared, packaged, or sold in devices such as patient-controlled analgesia (PCA) devices. Formulations for parenteral administration include, but are not limited to, suspensions, solutions, emulsions in oily or aqueous vehicles, pastes, and implantable sustained-release or biodegradable formulations. Such formulations may further comprise one or more additional ingredients including, but not limited to, suspending, stabilizing, or dispersing agents. In one embodiment of a formulation for parenteral administration, the active ingredient is provided in dry (*i.e.,* powder or granular) form for reconstitution with a suitable vehicle (*e.g.,* sterile pyrogen-free water) prior to parenteral administration of the reconstituted composition.

The pharmaceutical compositions may be prepared, packaged, or sold in the form of a sterile injectable aqueous or oily suspension or solution. This suspension or solution may be formulated according to the known art, and may comprise, in addition to the active ingredient, additional ingredients such as the dispersing agents, wetting agents, or suspending agents described herein. Such sterile injectable formulations may be prepared using a non-toxic parenterally acceptable diluent or solvent, such as water or 1,3-butanediol, for example. Other acceptable diluents and solvents include, but are not limited to, Ringer's solution, isotonic sodium chloride solution, and fixed oils such as synthetic mono- or di-glycerides. Other parentally-administrable formulations which are useful include those which comprise the active ingredient in microcrystalline form in a recombinant human albumin, a fluidized gelatin, in a liposomal preparation, or as a component of a biodegradable polymer system. Compositions for sustained release or implantation may comprise pharmaceutically acceptable polymeric or hydrophobic materials such as an emulsion, an ion exchange resin, a sparingly soluble polymer, or a sparingly soluble salt.

### Topical Administration

An obstacle for topical administration of pharmaceuticals is the stratum corneum layer of the epidermis. The stratum corneum is a highly resistant layer comprised of protein, cholesterol, sphingolipids, free fatty acids and various other lipids, and includes cornified and living cells. One of the factors that limit the penetration rate (flux) of a compound through the stratum corneum is the amount of the active substance that can be loaded or applied onto the skin surface. The greater the amount of active substance which is applied per unit of area of the skin, the greater the concentration gradient between the skin surface and the lower layers of the skin, and in turn the greater the diffusion force of the active substance through the skin. Therefore, a formulation containing a greater concentration of the active substance is more likely to result in penetration of the active substance through the skin, and more of it, and at a more consistent rate, than a formulation having a lesser concentration, all other things being equal.

Formulations suitable for topical administration include, but are not limited to, liquid or semi-liquid preparations such as liniments, lotions, oil-in-water or water-in-oil emulsions such as creams, ointments or pastes, and solutions or suspensions. Topically administrable formulations may, for example, comprise from about 1% to about 10% (w/w) active ingredient, although the concentration of the active ingredient may be as high as the solubility limit of the active ingredient in the solvent. Formulations for topical administration may further comprise one or more of the additional ingredients described herein.

Enhancers of permeation may be used. These materials increase the rate of penetration of drugs across the skin. Typical enhancers in the art include ethanol, glycerol monolaurate, PGML (polyethylene glycol monolaurate), dimethylsulfoxide, and the like. Other enhancers include oleic acid, oleyl alcohol, ethoxydiglycol, laurocapram, alkanecarboxylic acids, dimethylsulfoxide, polar lipids, or N-methyl-2-pyrrolidone.

One acceptable vehicle for topical delivery of some of the compositions of the disclosure may contain liposomes. The composition of the liposomes and their use are known in the art *(i.e.,* U.S. Patent No. 6,323,219).

In alternative embodiments, the topically active pharmaceutical composition may be optionally combined with other ingredients such as adjuvants, anti-oxidants, chelating agents, surfactants, foaming agents, wetting agents, emulsifying agents, viscosifiers, buffering agents, preservatives, and the like. In other embodiments, a permeation or penetration enhancer is included in the composition and is effective in improving the percutaneous penetration of the active ingredient into and through the stratum corneum with respect to a composition lacking the permeation enhancer. Various permeation enhancers, including oleic acid, oleyl alcohol, ethoxydiglycol, laurocapram, alkanecarboxylic acids, dimethylsulfoxide, polar lipids, or N-methyl-2-pyrrolidone, are known to those of skill in the art. In another aspect, the composition may further comprise a hydrotropic agent, which functions to increase disorder in the structure of the stratum corneum, and thus allows increased transport across the stratum corneum. Various hydrotropic agents such as isopropyl alcohol, propylene glycol, or sodium xylene sulfonate, are known to those of skill in the art.

The topically active pharmaceutical composition should be applied in an amount effective to affect desired changes. As used herein "amount effective" shall mean an amount sufficient to cover the region of skin surface where a change is desired. An active compound should be present in the amount of from about 0.0001% to about 15% by weight volume of the composition. For example, it should be present in an amount from about 0.0005% to about 5% of the composition; for example, it should be present in an amount of from about 0.001% to about 1% of the composition. Such compounds may be synthetically-or naturally derived.

### Buccal Administration

A pharmaceutical composition of the disclosure may be prepared, packaged, or sold in a formulation suitable for buccal administration. Such formulations may, for example, be in the form of tablets or lozenges made using conventional methods, and may contain, for example, 0.1 to 20% (w/w) of the active ingredient, the balance comprising an orally dissolvable or degradable composition and, optionally, one or more of the additional ingredients described herein. Alternately, formulations suitable for buccal administration may comprise a powder or an aerosolized or atomized solution or suspension comprising the active ingredient. Such powdered, aerosolized, or aerosolized formulations, when dispersed, may have an average particle or droplet size in the range from about 0.1 to about 200 nanometers, and may further comprise one or more of the additional ingredients described herein. The examples of formulations described herein are not exhaustive and it is understood that the disclosure includes additional modifications of these and other formulations not described herein, but which are known to those of skill in the art.

### Rectal Administration

A pharmaceutical composition of the disclosure may be prepared, packaged, or sold in a formulation suitable for rectal administration. Such a composition may be in the form of, for example, a suppository, a retention enema preparation, and a solution for rectal or colonic irrigation.

Suppository formulations may be made by combining the active ingredient with a non-irritating pharmaceutically acceptable excipient which is solid at ordinary room temperature (*i.e.,* about 20°C) and which is liquid at the rectal temperature of the subject (*i.e.,* about 37°C in a healthy human). Suitable pharmaceutically acceptable excipients include, but are not limited to, cocoa butter, polyethylene glycols, and various glycerides. Suppository formulations may further comprise various additional ingredients including, but not limited to, antioxidants, and preservatives.

Retention enema preparations or solutions for rectal or colonic irrigation may be made by combining the active ingredient with a pharmaceutically acceptable liquid carrier. As is well known in the art, enema preparations may be administered using, and may be packaged within, a delivery device adapted to the rectal anatomy of the subject. Enema preparations may further comprise various additional ingredients including, but not limited to, antioxidants, and preservatives.

### Additional Administration Forms

Additional dosage forms of this disclosure include dosage forms as described in U.S. Patents Nos. 6,340,475, 6,488,962, 6,451,808, 5,972,389, 5,582,837, and 5,007,790. Additional dosage forms of this disclosure also include dosage forms as described in U.S. Patent Applications Nos. 20030147952, 20030104062, 20030104053, 20030044466, 20030039688, and 20020051820. Additional dosage forms of this disclosure also include dosage forms as described in PCT Applications Nos. WO 03/35041, WO 03/35040, WO 03/35029, WO 03/35177, WO 03/35039, WO 02/96404, WO 02/32416, WO 01/97783, WO 01/56544, WO 01/32217, WO 98/55107, WO 98/11879, WO 97/47285, WO 93/18755, and WO 90/11757.

### Controlled Release Formulations and Drug Delivery Systems:

In certain embodiments, the compositions and/or formulations of the present disclosure may be, but are not limited to, short-term, rapid-offset, as well as controlled, for example, sustained release, delayed release and pulsatile release formulations.

The term sustained release is used in its conventional sense to refer to a drug formulation that provides for gradual release of a drug over an extended period of time, and that may, although not necessarily, result in substantially constant blood levels of a drug over an extended time period. The period of time may be as long as a month or more and should be a release which is longer that the same amount of agent administered in bolus form.

For sustained release, the compounds may be formulated with a suitable polymer or hydrophobic material which provides sustained release properties to the compounds. As such, the compounds for use the method of the disclosure may be administered in the form of microparticles, for example, by injection or in the form of wafers or discs by implantation. In certain embodiments of the disclosure, the compounds useful within the disclosure are administered to a subject, alone or in combination with another pharmaceutical agent, using a sustained release formulation.

The term delayed release is used herein in its conventional sense to refer to a drug formulation that provides for an initial release of the drug after some delay following drug administration and that may, although not necessarily, include a delay of from about 10 minutes up to about 12 hours. The term pulsatile release is used herein in its conventional sense to refer to a drug formulation that provides release of the drug in such a way as to produce pulsed plasma profiles of the drug after drug administration. The term immediate release is used in its conventional sense to refer to a drug formulation that provides for release of the drug immediately after drug administration.

As used herein, short-term refers to any period of time up to and including about 8 hours, about 7 hours, about 6 hours, about 5 hours, about 4 hours, about 3 hours, about 2 hours, about 1 hour, about 40 minutes, about 20 minutes, or about 10 minutes and any or all whole or partial increments thereof after drug administration after drug administration.

As used herein, rapid-offset refers to any period of time up to and including about 8 hours, about 7 hours, about 6 hours, about 5 hours, about 4 hours, about 3 hours, about 2 hours, about 1 hour, about 40 minutes, about 20 minutes, or about 10 minutes, and any and all whole or partial increments thereof after drug administration.

Those skilled in the art will recognize, or be able to ascertain using no more than routine experimentation, numerous equivalents to the specific procedures, embodiments, claims, and examples described herein. Such equivalents were considered to be within the scope of this disclosure and covered by the claims appended hereto. For example, it should be understood, that modifications in reaction conditions, including but not limited to reaction times, reaction size/volume, and experimental reagents, such as solvents, catalysts, pressures, atmospheric conditions, *e.g*., nitrogen atmosphere, and reducing/oxidizing agents, with art-recognized alternatives and using no more than routine experimentation, are within the scope of the present application.

It is to be understood that, wherever values and ranges are provided herein, the description in range format is merely for convenience and brevity and should not be construed as an inflexible limitation on the scope of the disclosure. Accordingly, all values and ranges encompassed by these values and ranges are meant to be encompassed within the scope of the present disclosure. Moreover, all values that fall within these ranges, as well as the upper or lower limits of a range of values, are also contemplated by the present application. The description of a range should be considered to have specifically disclosed all the possible sub-ranges as well as individual numerical values within that range and, when appropriate, partial integers of the numerical values within ranges. For example, description of a range such as from 1 to 6 should be considered to have specifically disclosed sub-ranges such as from 1 to 3, from 1 to 4, from 1 to 5, from 2 to 4, from 2 to 6, from 3 to 6 etc., as well as individual numbers within that range, for example, 1, 2, 2.7, 3, 4, 5, 5.3, and 6. This applies regardless of the breadth of the range.

The following examples further illustrate aspects of the present disclosure. However, they are in no way a limitation of the teachings or disclosure of the present disclosure as set forth herein.

### EXAMPLES

The disclosure is now described with reference to the following Examples. These Examples are provided for the purpose of illustration only, and the disclosure is not limited to these Examples, but rather encompasses all variations that are evident as a result of the teachings provided herein.

### LCMS Methods

**LCMS Method A:** Waters Acquity UPLC system employing a Waters Acquity UPLC BEH C18, 1.7 µm, 50 x 2.1 mm column with an aqueous acetonitrile based solvent gradient of 2-98% CH₃CN/H₂O (0.05 % TFA) over 9.5 mins. Flow rate = 0.8 mL/min.

**LCMS Method B:** Waters Acquity UPLC system employing a Waters Acquity UPLC BEH C18, 1.7 µm, 50 x 2.1 mm column with an aqueous acetonitrile based solvent gradient of 2-98% CH₃CN/H₂O (0.05 % TFA) over 1.0 mins. Flow rate = 0.8 mL/min.

**LCMS Method C:** Shimadzu UFLC system employing an ACE UltraCore Super PhenylHexyl, 2.5 µm, 50 x 2.1 mm column with an aqueous acetonitrile based solvent gradient of 5-100% CH₃CN/H₂O (0.05 % Formic acid) over 5.0 mins. Flow rate = 1.0 mL/min.

**LCMS Method D:** Waters Acquity UPLC system employing a Waters Acquity UPLC BEH C18, 1.7 µm, 50 x 2.1 mm column with an aqueous acetonitrile based solvent gradient of 2-98% CH₃CN/H₂O (0.05 % TFA) over 5.0 mins. Flow rate = 0.8 mL/min.

### General Procedures

### Suzuki Coupling Procedure A

A reaction vial equipped with a stir bar was charged with aryl halide (1.0 equivalent), aryl boronic ester/acid (1.0 equivalent), potassium carbonate (3.0 equivalent), Pd(PPh₃)₄ (0.1 equivalent) and dioxane/water (6:1, 0.3 M). The mixture was degassed for 20 minutes, capped with TFE lined silicone septa cap and stirred at 95 °C for 1 hour in a heating block. Upon cooling down to rt, water was added and the reaction mixture was extracted into EtOAc three times. The combined organic layers were dried over anhydrous sodium sulfate, filtered and concentrated *in vacuo.* The crude was purified on silica gel column using EtOAc in hexanes (0 to 60% gradient) as eluent.

### Suzuki Coupling Procedure B

A reaction vial equipped with a stir bar was charged with aryl halide (1.0 equivalent), aryl boronic ester/acid (1.0 equivalent), potassium carbonate (3.0 equivalent), Pd(PPh₃)₄ (0.1 equivalent) and dioxane/water (6:1, 0.3 M). The reaction mixture was degassed for 20 minutes, capped with TFE lined silicone septa cap and stirred at 95 °C for 2 hours in a heating block. After cooling down to rt, the mixture was directly purified on silica gel column using MeOH in CH₂Cl₂ (0 to 5% gradient) as eluent.

### Reductive Amination Procedure A

A mixture of bis-aldehyde (1.0 equivalent), amine (4.0 equivalent) and acetic acid (2.0 equivalent) in MeOH/THF (1:1) was stirred for 2 hours at rt. NaBH₃CN (4.0 equivalent) was added slowly in portions, the mixture was stirred for additional 1 hour or more (monitored by LCMS), quenched with water and extracted into DCM three times. The combined organic layers were dried over anhydrous magnesium sulfate, filtered and concentrated *in vacuo.* The crude was purified by reversed phase chromatography (5 to 100% acetonitrile gradient in water, with 0.05% formic acid) and pure combined fractions were lyophilized to afford product as formic acid salt.

### Reductive Amination Procedure B

A mixture of aldehyde (1.0 equivalent), amine (2.0 equivalent) and acetic acid (1.0 equivalent) in MeOH/THF (1:1) was stirred for 2 hours at rt. NaBH₃CN (2.0 equivalent) was added slowly in portions, the mixture was stirred for additional 1 hour or more (monitored by LCMS), quenched with water and extracted into DCM three times. The combined organic layers were dried over anhydrous magnesium sulfate, filtered, concentrated *in vacuo* and used in the next step without purification.

### General Boc Deprotection Procedure

A solution of a Reductive Amination or Suzuki Coupling product was stirred in CH₂Cl₂/TFA (1:1) for 30 minutes and concentrated to dryness under vacuum. The crude was purified by reversed phase chromatography (5 to 100% acetonitrile gradient in water, with 0.05% formic acid) and pure combined fractions were lyophilized to afford product as formic acid salt.

### Example 1: 3-Chloro-4-(2-chloro-3-(5-(4,5-dihydro-1H-imidazol-2-yl)-6-methoxypyridin-2-yl)phenyl)-2-(4-(4,5-dihydro-1H-imidazol-2-yl)-3-methoxyphenyl)pyridine (106)

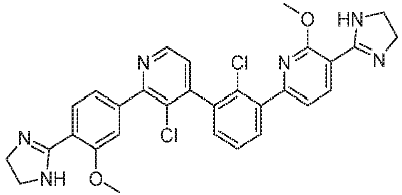

### (a) 6-(2-Chloro-3-(2,3-dichloropyridin-4-yl)phenyl)-2-methoxynicotinaldehyde

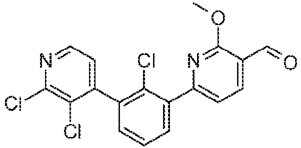

To a mixture of 6-(2-chloro-3-(4,4,5,5-tetramethyl-1,3,2-dioxaborolan-2-yl)phenyl)-2-methoxynicotinaldehyde (5.00 g, 13.4 mmol) and 2,3-dichloro-4-iodo-pyridine (4.40 g, 16.1 mmol) in dioxane/H₂O (6:1 (*v*/*v*), 70 mL) was added potassium carbonate (5.55 g, 40.1 mmol) and [1,1'-bis(di-*tert*-butylphosphino)ferrocene]dichloropalladium(II) (546 mg, 0.67 mmol). The mixture was stirred at 95 °C for 1 h under N₂. The reaction mixture was added to water (30 mL) and extracted with ethyl acetate (2 x 100 ml). The combined organic layers were dried over anhydrous sodium sulfate, filtered and concentrated under reduced pressure. The resulting residue was purified by normal phase SiO₂ chromatography (0-10% Ethyl acetate/petroleum ether) to afford 6-(2-chloro-3-(2,3-dichloropyridin-4-yl)phenyl)-2-methoxynicotinaldehyde (3.2 g, 61% yield) as a white solid. ¹H NMR (400 MHz, DMSO-*d*₆): δ 10.30 (s, 1H), 8.53 (d, 1H), 8.23 (d, 1H), 7.82 (m, 1H), 7.66 (t, 1H), 7.61 (d, 1H), 7.57 (m, 1H), 7.49 (d, 1H) 4.07 (s, 1H).

### (b) 6-(2-Chloro-3-(3-chloro-2-(4-formyl-3-methoxyphenyl)pyridin-4-yl)phenyl)-2-methoxynicotinaldehyde

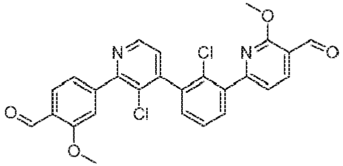

To a mixture of 6-(2-chloro-3-(2,3-dichloropyridin-4-yl)phenyl)-2-methoxynicotinaldehyde (1.50 g, 3.81 mmol) and 2-methoxy-4-(4,4,5,5-tetramethyl-1,3,2-dioxaborolan-2-yl)benzaldehyde (1.30 g, 4.95 mmol) in THF/H₂O (5: 1 *v*/*v*, 24 mL) was added potassium phosphate (2.43 g, 11.4 mmol) and[1,1'-bis(di-*tert-*butylphosphino)ferrocene]dichloropalladium(II) (248 mg, 0.38 mmol). The mixture was stirred at 80 °C for 1 h under N₂. The mixture was filtered, and the filtrate was concentrated under reduced pressure. The residue was purified by flash chromatography (SiO₂, 0-50% Ethyl acetate/petroleum ether) to afford 6-(2-chloro-3-(3-chloro-2-(4-formyl-3-methoxyphenyl)pyridin-4-yl)phenyl)-2-methoxynicotinaldehyde (1.17 g, 40% yield) as a white solid . ¹H NMR (400 MHz, DMSO-*d*₆): δ 10.43 (s, 1H), 10.31 (s, 1H), 8.78 (d, 1H), 8.23 (d, 1H), 7.84-7.80 (m, 2H), 7.68-7.59 (m, 3H), 7.51-7.49 (m, 2H), 7.41 (d, 1H), 4.07 (s, 3H), 3.99 (s, 3H).

### (c) 3-Chloro-4-(2-chloro-3-(5-(4,5-dihydro-1H-imidazol-2-yl)-6-methoxypyridin-2-yl)phenyl)-2-(4-(4,5-dihydro-1H-imidazol-2-yl)-3-methoxyphenyl)pyridine

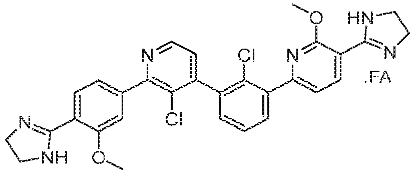

To a mixture of 6-(2-chloro-3-(3-chloro-2-(4-formyl-3-methoxyphenyl)pyridin-4-yl)phenyl)-2-methoxynicotinaldehyde (200 mg, 0.41 mmol) and ethane-1,2-diamine (0.47 mL, 7.04 mmol) in DMA (3 mL) was added potassium carbonate (560 mg, 4.05 mmol) and iodine (617 mg, 2.43 mmol). The mixture was stirred at RT for 12 h under a nitrogen atmosphere. An additional portion of ethane-1,2-diamine (2.66 mL, 39.78 mmol) was added to the mixture, then the mixture was stirred at RT for 12 h under N₂. The reaction mixture was added to water (10 mL), and then extracted with ethyl acetate (2 x 20 ml). The combined organic layers were dried over anhydrous sodium sulfate, filtered and concentrated under reduced pressure. The resulting residue was purified by reverse phase HPLC to afford 3-chloro-4-(2-chloro-3-(5-(4,5-dihydro-1H-imidazol-2-yl)-6-methoxypyridin-2-yl)phenyl)-2-(4-(4,5-dihydro-1H-imidazol-2-yl)-3-methoxyphenyl)pyridine as formic acid salt (2 mg, 0.73% yield) as a white solid. MS: m/z found 573 [M+H]⁺; ¹H NMR (400 MHz, Methanol-*d*₄): δ 8.62 (d, 1H), 8.45 (s, 1H), 8.20 (d, 1H), 7.79 (d, 1H), 7.71 (m, 1H), 7.53-7.51 (m, 2H), 7.47-7.41 (m, 4H), 4.08 (s, 3H), 4.00-3.95 (m, 11H).

### Example 2: 2-((6-(2-chloro-3-(3-chloro-2-(3-methoxy-4-((7-oxo-2,6-diazaspiro[3.4]octan-2-yl)methyl)phenyl)pyridin-4-yl)phenyl)-2-methoxypyridin-3-yl)methyl)-2,6-diazaspiro[3.4]octan-7-one (89)

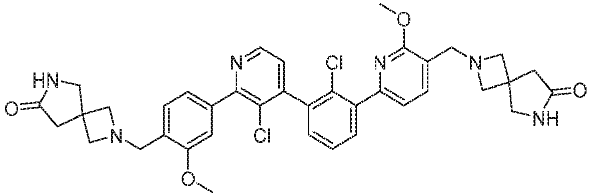

To a mixture of 6-(2-chloro-3-(3-chloro-2-(4-formyl-3-methoxyphenyl)pyridin-4-yl)phenyl)-2-methoxynicotinaldehyde (200 mg, 0.41 mmol) and 2,6-diazaspiro[3.4]octan-7-one (204 mg, 1.62 mmol) in methylene chloride (10 mL) was added sodium triacetoxyborohydride (344 mg, 1.62 mmol) and sodium acetate (133 mg, 1.62 mmol). The mixture was stirred at 20 °C for 3 h under N₂. The mixture was concentrated. The residue was purified by reverse phase HPLC to afford 2-((6-(2-chloro-3-(3-chloro-2-(3-methoxy-4-((7-oxo-2,6-diazaspiro[3.4]octan-2-yl)methyl)phenyl)pyridin-4-yl)phenyl)-2-methoxypyridin-3-yl)methyl)-2,6-diazaspiro[3.4]octan-7-one as a formic acid salt (37.6 mg, 12% yield) as a white solid. LCMS: m/z found 713.1 [M+H]+, RT = 2.54 min, (Method A); ¹H NMR (400 MHz, Methanol-*d*₄): δ 8.67 (d, 1H), 8.34 (s, 1H), 7.78 (d, 1H), 7.72 (m, 1H), 7.59-7.55 (m,1H), 7.51 (d, 1H), 7.49 (d, 1H), 7.44 (m, 1H), 7.42-7.36 (m, 2H), 7.30 (d, 1H), 4.32 (s, 2H), 4.07 (s, 4H), 4.04 (s, 3H), 4.02 (s, 3H), 3.99 (s, 2H), 3.72 (s, 4H), 3.72 (s, 2H), 3.64 (d, 2H), 2.71 (s, 2H), 2.65 (s, 2H).

### Example 3: 1-(6-((6-(3-(2-(4-((6-acetyl-2,6-diazaspiro[3.3]heptan-2-yl)methyl)-3-methoxyphenyl)-3-chloropyridin-4-yl)-2-chlorophenyl)-2-methoxypyridin-3-yl)methyl)-2,6-diazaspiro[3.3]heptan-2-yl)ethenone (90)

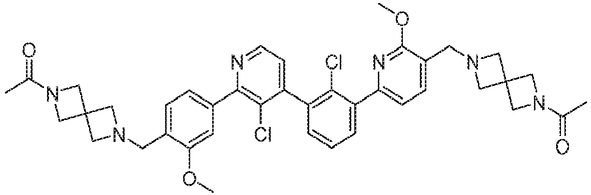

To a mixture of 6-(2-chloro-3-(3-chloro-2-(4-formyl-3-methoxyphenyl)pyridin-4-yl)phenyl)-2-methoxynicotinaldehyde (90 mg, 0.18mmol ) and 1-(2,6-diazaspiro[3.3]heptan-2-yl)ethanone trifluoroacetate (120 mg, 0.47 mmol) in methylene chloride (10 mL) was added sodium triacetoxyborohydride (154 mg, 0.73 mmol) and sodium acetate (60 mg, 0.73 mmol). The mixture was stirred at 20 °C for 5 h under N₂. The mixture was concentrated. The resulting residue was purified by reverse phase HPLC to afford 1-(6-((6-(3-(2-(4-((6-acetyl-2,6-diazaspiro[3.3]heptan-2-yl)methyl)-3-methoxyphenyl)-3-chloropyridin-4-yl)-2-chlorophenyl)-2-methoxypyridin-3-yl)methyl)-2,6-diazaspiro[3.3]heptan-2-yl)ethanone as formic acid salt (16.2 mg, 12%) as a white solid. LCMS: m/z found 741.0 [M+H]⁺, RT = 2.76 min, (Method A); ¹H NMR (400 MHz, Methanol-*d*₄): δ 8.65-8.64 (m, 1H), 8.39 (s, 1H), 7.75 (d, 1H), 7.77 (m, 1H), 7.56 (m, 1H), 7.48-7.44 (m, 2H), 7.42 (m, 1H), 7.37 (s, 1H), 7.34 (m, 1H), 7.28 (m, 1H), 4.35 (m, 4H), 4.29 (s, 2H), 4.17 (s, 4H), 4.12 (s, 2H), 4.08 (s, 2H), 4.03 (s, 3H), 3.97 (s, 5H), 3.81 (s, 4H), 1.84 (s, 6H).

### Example 4: (5S)-5-[[[3-[3-Chloro-4-[2-chloro-3-[6-methoxy-5-[[[(2S)-5-oxopyrrolidin-2-yl]methylamino]methyl]-2-pyridyl]phenyl]-2-pyridyl]-7-quinolyl]methylamino]methyl]pyrrolidin-2-one (52)

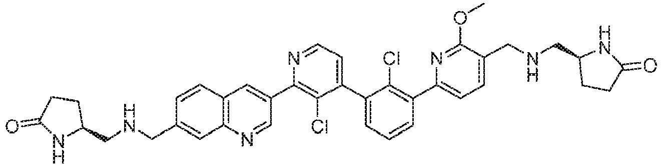

Following Suzuki Coupling Procedure A from 3-(4,4,5,5-tetramethyl-1,3,2-dioxaborolan-2-yl)quinoline-7-carbaldehyde (30 mg, 0.11 mmol) and 6-[2-chloro-3-(2,3-dichloro-4-pyridyl)phenyl]-2-methoxy-pyridine-3-carbaldehyde (42 mg, 0.11 mmol) afforded 3-[3-chloro-4-[2-chloro-3-(5-formyl-6-methoxy-2-pyridyl)phenyl]-2-pyridyl]quinoline-7-carbaldehyde (40 mg, 73%). MS: m/z found 514.1 [M+H]⁺. Following Reductive Amination Procedure A from 3-[3-chloro-4-[2-chloro-3-(5-formyl-6-methoxy-2-pyridyl)phenyl]-2-pyridyl]quinoline-7-carbaldehyde (30 mg, 0.06 mmol) and (5S)-5-(aminomethyl)pyrrolidin-2-one (27 mg, 0.23 mmol) gave (5S)-5-[[[3-[3-chloro-4-[2-chloro-3-[6-methoxy-5-[[[(2S)-5-oxopyrrolidin-2-yl]methylamino]methyl]-2-pyridyl]phenyl]-2-pyridyl]-7-quinolyl]methylamino]methyl]pyrrolidin-2-one as formic acid salt (42 mg, 91%). MS: m/z found 710.2 [M+H]⁺. ¹H NMR (400 MHz, Methanol-*d*₄): δ 9.19 (d, 1H), 8.79 - 8.70 (m, 2H), 8.49 (s, 2H), 8.13 - 8.02 (m, 2H), 7.80 - 7.69 (m, 3H), 7.57 (t, 1H), 7.52 (d, 1H), 7.46 (dd, 1H), 7.28 (d, 1H), 4.17 - 4.06 (m, 2H), 4.03 (s, 3H), 3.96 - 3.83 (m, 4H), 2.86 - 2.70 (m, 4H), 2.42 - 2.22 (m, 6H), 1.91 - 1.78 (m, 2H).

### (a) 6-[2-Chloro-3-[3-chloro-2-(4-formyl-3-methoxy-phenyl)-4-pyridyl]phenyl]-2-methoxy-pyridine-3-carbaldehyde

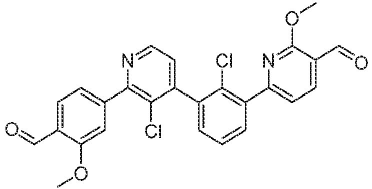

This intermediate was prepared following Suzuki Coupling Procedure A from 6-[2-chloro-3-(2,3-dichloro-4-pyridyl)phenyl]-2-methoxy-pyridine-3-carbaldehyde (300 mg, 0.76 mmol) and 2-methoxy-4-(4,4,5,5-tetramethyl-1,3,2-dioxaborolan-2-yl)benzaldehyde (200 mg, 0.76 mmol). 81% yield. MS: m/z found 493.1 [M+H]⁺.

### Example 5: 1-[4-[3-Chloro-4-[2-chloro-3-[6-methoxy-5-(methylaminomethyl)-2-pyridyl]phenyl]-2-pyridyl]-2-methoxy-phenyl]-N-methyl-methanamine (136)

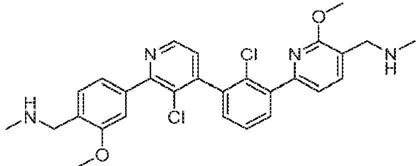

1-[4-[3-Chloro-4-[2-chloro-3-[6-methoxy-5-(methylaminomethyl)-2-pyridyl]phenyl]-2-pyridyl]-2-methoxy-phenyl]-N-methyl-methanamine was prepared as the formic acid salt, following Reductive Amination Procedure A from 6-[2-chloro-3-[3-chloro-2-(4-formyl-3-methoxy-phenyl)-4-pyridyl]phenyl]-2-methoxy-pyridine-3-carbaldehyde (40 mg, 0.08 mmol) and methanamine solution (33% wt in ethanol, 0.32 mmol). 59% yield. MS: m/z found 523.2 [M+H]⁺. ¹H NMR (400 MHz, Methanol-*d*₄): δ 8.66 (d, 1H), 8.53 (s, 2H), 7.87 (d, 1H), 7.73 (dd, 1H), 7.58 (t, 1H), 7.51 (d, 1H), 7.48 (d, 1H), 7.45 (dd, 1H), 7.39 (d, 1H), 7.35 (d, 2H), 4.26 (s, 2H), 4.21 (s, 2H), 4.08 (s, 3H), 3.99 (s, 3H), 2.74 (s, 6H).

### Example 6: 1-[4-[3-Chloro-4-[2-chloro-3-[5-[(dimethylamino)methyl]-6-methoxy-2-pyridyl]phenyl]-2-pyridyl]-2-methoxy-phenyl]-N,N-dimethyl-methanamine (137)

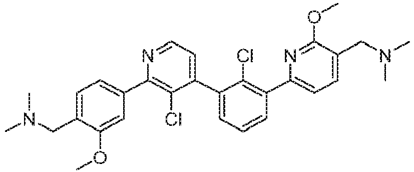

1-[4-[3-Chloro-4-[2-chloro-3-[5-[(dimethylamino)methyl]-6-methoxy-2-pyridyl]phenyl]-2-pyridyl]-2-methoxy-phenyl]-N,N-dimethyl-methanamine was prepared as the formic acid salt, following Reductive Amination Procedure A from 6-[2-chloro-3-[3-chloro-2-(4-formyl-3-methoxy-phenyl)-4-pyridyl]phenyl]-2-methoxy-pyridine-3-carbaldehyde (40 mg, 0.08 mmol) and N-methylmethanamine (2.0 M solution in ethanol, 0.32 mmol). 67% yield. MS: m/z found 551.2 [M+H]⁺. ¹H NMR (400 MHz, Methanol-*d*₄): δ 8.67 (dd, 1H), 8.53 (s, 2H), 7.82 (d1H), 7.74 (dd, 1H), 7.57 (t, 1H), 7.52 (d, 1H), 7.48 (d, 1H), 7.44 (dd, 1H), 7.41 (d, 1H), 7.37 (dd, 1H), 7.33 (d, 1H), 4.27 (s, 2H), 4.05 (d, 3H), 3.99 (s, 3H), 3.97 (s, 2H), 2.80 (s, 6H), 2.61 (s, 6H).

### Example 7: 2-[[4-[3-Chloro-4-[2-chloro-3-[5-[[2-hydroxyethyl(methyl)amino]methyl]-6-methoxy-2-pyridyl]phenyl]-2-pyridyl]-2-methoxy-phenyl]methyl-methyl-amino]ethanol (138)

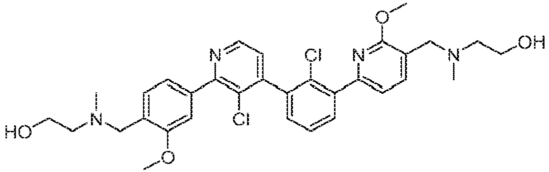

2-[[4-[3-Chloro-4-[2-chloro-3-[5-[[2-hydroxyethyl(methyl)amino]methyl]-6-methoxy-2-pyridyl]phenyl]-2-pyridyl]-2-methoxy-phenyl]methyl-methyl-amino]ethanol was prepared as the formic acid salt, following Reductive Amination Procedure A from 6-[2-chloro-3-[3-chloro-2-(4-formyl-3-methoxy-phenyl)-4-pyridyl]phenyl]-2-methoxy-pyridine-3-carbaldehyde (40 mg, 0.08 mmol) and 2-(methylamino)ethanol (24 mg, 0.32 mmol). 70% yield. MS: m/z found 611.2 [M+H]⁺. ¹H NMR (400 MHz, Methanol-*d*₄): δ 8.66 (d, 1H), 8.53 (s, 2H), 7.86 (d1H), 7.73 (dd, 1H), 7.61 - 7.51 (m, 2H), 7.48 (d, 1H), 7.43 (dd, 1H), 7.40 (d, 1H), 7.36 (dd, 1H), 7.31 (d, 1H), 4.32 (s, 2H), 4.03 (s, 3H), 4.00 - 3.93 (m, 5H), 3.89 (t, 2H), 3.81 (t2H), 3.19 (t, 2H), 2.92 (t, 2H), 2.77 (s, 3H), 2.55 (s, 3H).

### Example 8: 1-[4-[[4-[4-[3-[5-[[(1-Acetyl-4-piperidyl)amino]methyl]-6-methoxy-2-pyridyl]-2-chloro-phenyl]-3-chloro-2-pyridyl]-2-methoxy-phenyl]methylamino]-1-piperidyl]ethanone (139)

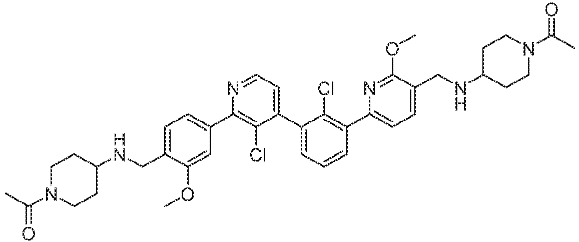

1-[4-[[4-[4-[3-[5-[[(1-Acetyl-4-piperidyl)amino]methyl]-6-methoxy-2-pyridyl]-2-chloro-phenyl]-3-chloro-2-pyridyl]-2-methoxy-phenyl]methylamino]-1-piperidyl]ethanone was prepared as the formic acid salt, following Reductive Amination Procedure A from 6-[2-chloro-3-[3-chloro-2-(4-formyl-3-methoxy-phenyl)-4-pyridyl]phenyl]-2-methoxy-pyridine-3-carbaldehyde (30 mg, 0.06 mmol) and 1-(4-amino-1-piperidyl)ethanone (34 mg, 0.24 mmol). 77% yield. MS: m/z found 745.3 [M+H]⁺. ¹H NMR (400 MHz, Methanol-*d*₄): δ 8.67 (dd, 1H), 8.50 - 8.43 (m, 2H), 7.91 - 7.84 (m, 1H), 7.76 - 7.68 (m, 1H), 7.61 - 7.51 (m, 2H), 7.50 - 7.42 (m, 2H), 7.39 (s, 1H), 7.34 (td, 2H), 4.65 (t, 2H), 4.31 (s, 2H), 4.19 (s, 2H), 4.12 - 4.01 (m, 5H), 3.99 (s, 3H), 3.20 (t, 2H), 2.70 (t, 2H), 2.30 - 2.17 (m, 3H), 2.17 - 2.06 (m, 9H), 1.71 - 1.37 (m, 4H).

### Example 9: 1-[4-[[4-[4-[3-[5-[[(1-Acetyl-4-piperidyl)-methyl-amino]methyl]-6-methoxy-2-pyridyl]-2-chloro-phenyl]-3-chloro-2-pyridyl]-2-methoxy-phenyl]methyl-methyl-amino]-1-piperidyl]ethanone (140)

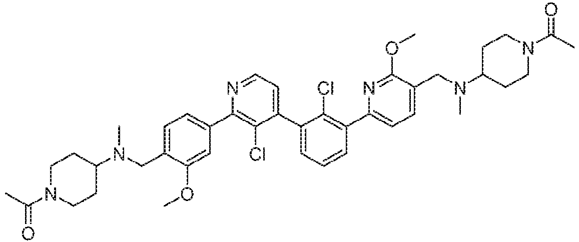

1-[4-[[4-[4-[3-[5-[[(1-Acetyl-4-piperidyl)-methyl-amino]methyl]-6-methoxy-2-pyridyl]-2-chloro-phenyl]-3-chloro-2-pyridyl]-2-methoxy-phenyl]methyl-methyl-amino]-1-piperidyl]ethanone was prepared as the formic acid salt following Reductive Amination Procedure A from 6-[2-chloro-3-[3-chloro-2-(4-formyl-3-methoxy-phenyl)-4-pyridyl]phenyl]-2-methoxy-pyridine-3-carbaldehyde (30 mg, 0.06 mmol) and 1-[4-(methylamino)-1-piperidyl]ethanone (38 mg, 0.24 mmol). 30% yield. MS: m/z found 773.3 [M+H]⁺. ¹H NMR (400 MHz, Methanol-*d*₄): δ 8.66 (d, 1H), 8.49 - 8.42 (m, 2H), 7.83 (d, 1H), 7.73 (d, 1H), 7.60 - 7.51 (m, 2H), 7.50 - 7.45 (m, 1H), 7.45 - 7.33 (m, 3H), 7.32 - 7.25 (m, 1H), 4.74 (d, 1H), 4.65 (d, 1H), 4.31 - 4.04 (m, 4H), 4.02 (s, 3H), 3.98 (s, 3H), 3.91 - 3.84 (m, 2H), 3.24 - 3.10 (m, 2H), 2.76 - 2.59 (m, 5H), 2.46 (s, 3H), 2.24 - 2.11 (m, 10H), 2.09 - 1.98 (m, 2H), 1.92 - 1.52 (m, 4H).

### Example 10: 8-(3-chloro-4-(2-chloro-3-(6-methoxy-5-(((((S)-5-oxopyrrolidin-2-yl)methyl)-amino)methyl)-pyridin-2-yl)phenyl)pyridin-2-yl)-3-(((((S)-5-oxopyrrolidin-2-yl)methyl)amino)methyl)-4H-pyrido[1,2-a]pyrimidin-4-one (2)

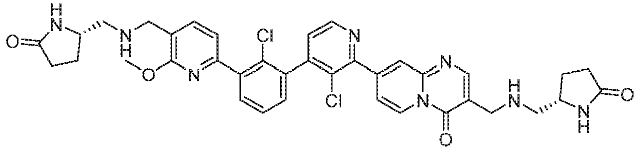

### (a) 6-(2-chloro-3-(2,3-dichloropyridin-4-yl)phenyl)-2-methoxynicotinaldehyde

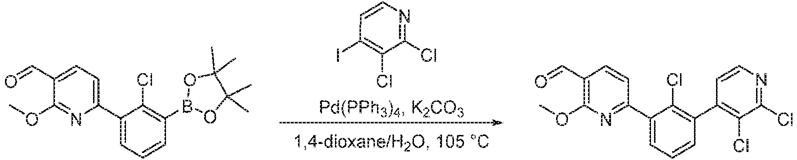

Pd(PPh₃)₄ (309 mg, 0.27 mmol), potassium carbonate (554 mg, 4.01 mmol), 2,3-dichloro-4-iodo-pyridine (476 mg, 1.74 mmol), and 6-[2-chloro-3-(4,4,5,5-tetramethyl-1,3,2-dioxaborolan-2-yl)phenyl]-2-methoxy-pyridine-3-carbaldehyde (500 mg, 1.34 mmol) were suspended in 1,4-dioxane/water (4:1, 15 mL), then the solution was heated at 105 °C for 20 minutes. Reaction was cooled to rt, diluted with 15 mL water, and extracted with EtOAc (3 x 10 mL). The combined organics were concentrated under reduced pressure and crude sample was purified by normal phase SiO₂ chromatography (0-30 % EtOAc/hexane) to afford 6-[2-chloro-3-(2,3-dichloro-4-pyridyl)phenyl]-2-methoxy-pyridine-3-carbaldehyde (373 mg, 71 % yield) as a tan solid. MS: *m*/*z* found 393, 395 [M+H] ⁺.

### (b) 4-Oxo-8-(4,4,5,5-tetramethyl-1,3,2-dioxaborolan-2-yl)-4H-pyrido[1,2-a]pyrimidine-3-carbaldehyde

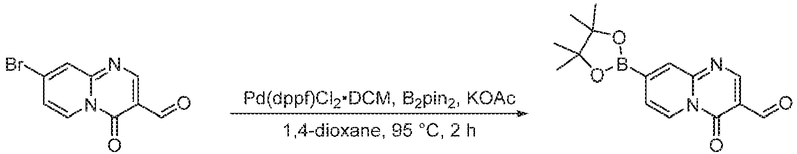

8-Bromo-4-oxo-pyrido[1,2-a]pyrimidine-3-carbaldehyde (500 mg, 1.78 mmol), 4,4,5,5-tetramethyl-2-(4,4,5,5-tetramethyl-1,3,2-dioxaborolan-2-yl)-1,3,2-dioxaborolane (587 mg, 2.31 mmol), 1,1'-bis(diphenylphosphino)-ferrocene-palladium(II) dichloride dichloromethane complex (145 mg, 0.18 mmol), and potassium acetate (488 mg, 4.98 mmol) were suspended in 10 mL 1,4-dioxane in a sealed tube. Nitrogen gas was bubbled through the reaction solution for 2 minutes, then the vessel was sealed and heated at 95 °C for 2 hours. Reaction was cooled to rt and the crude mixture was filtered through celite. The celite plug was further washed with 10 mL 1,4-dioxane and 30 mL EtOAc. The insoluble solid (desired product) was subsequently removed from the celite plug by washing with methanol (30 mL). The methanolic solution was then concentrated under reduced pressure and the crude oil was triturated with EtOAc/hexane. The precipitate was filtered to afford 4-oxo-8-(4,4,5,5-tetramethyl-1,3,2-dioxaborolan-2-yl)pyrido[1,2-a]pyrimidine-3-carbaldehyde (551 mg, crude) as a tan solid. MS: *m*/*z* found 301 [M+H] ⁺. Material was used for the next step without further purification.

### (c) 8-(3-Chloro-4-(2-chloro-3-(5-formyl-6-methoxypyridin-2-yl)phenyl)pyridin-2-yl)-4-oxo-4H-pyrido[1,2-a]pyrimidine-3-carbaldehyde

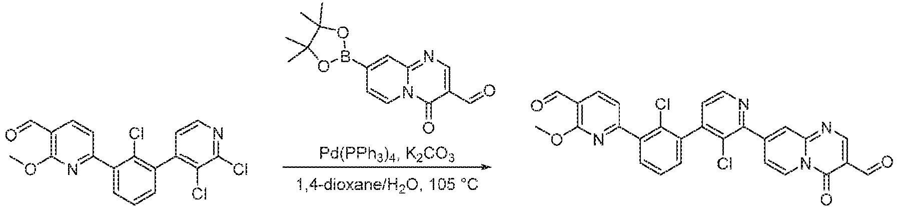

Pd(PPh₃)₄ (41 mg, 0.04 mmol), potassium carbonate (74 mg, 0.53 mmol), 6-[2-chloro-3-(2,3-dichloro-4-pyridyl)phenyl]-2-methoxy-pyridine-3-carbaldehyde (70 mg, 0.18 mmol), and 4-oxo-8-(4,4,5,5-tetramethyl-1,3,2-dioxaborolan-2-yl)pyrido[1,2-a]pyrimidine-3-carbaldehyde (69 mg, 0.23 mmol) were suspended in 1,4-dioxane/water (4:1, 3 mL), then the solution was heated at 105 °C for 20 minutes. Reaction was cooled to rt, diluted with 4 mL water, and extracted with EtOAc (3 x 3 mL). The combined organics were concentrated under reduced pressure and crude sample was passed through a short SiO₂ column (5-100 % EtOAc/hexane) to remove some impurities. The clean fractions were concentrated under reduced pressure to afford 8-[3-chloro-4-[2-chloro-3-(5-formyl-6-methoxy-2-pyridyl)phenyl]-2-pyridyl]-4-oxo-pyrido[1,2-a]pyrimidine-3-carbaldehyde (38 mg, crude) as a yellow foam. MS: *m*/*z* found 531, 533 [M+H] ⁺. Material was used for the next step without further purification.

### (d) 8-(3-Chloro-4-(2-chloro-3-(6-methoxy-5-(((((S)-5-oxopyrrolidin-2-yl)methyl)-amino)methyl)-pyridin-2-yl)phenyl)pyridin-2-yl)-3-(((((S)-5-oxopyrrolidin-2-yl)methyl)amino)methyl)-4H-pyrido[1,2-a]pyrimidin-4-one (2)

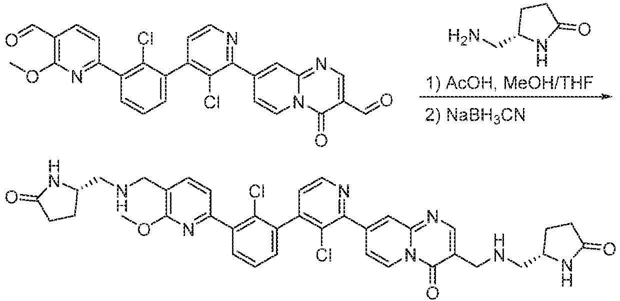

8-[3-Chloro-4-[2-chloro-3-(5-formyl-6-methoxy-2-pyridyl)phenyl]-2-pyridyl]-4-oxo-pyrido[1,2-a]pyrimidine-3-carbaldehyde (38 mg, 0.01 mmol, crude), (5S)-5-(aminomethyl)pyrrolidin-2-one (5 mg, 0.04 mmol), and acetic acid (2 mg, 0.03 mmol) were dissolved in MeOH/THF (1:1, 1 mL), then the solution was stirred at 25 °C for 2 hours. NaBH₃CN (2 mg, 0.03 mmol) was added and the resulting mixture was stirred at 25 °C for 1 hour. Reaction was subsequently diluted with water (2 mL) and extracted with EtOAc (3 x 2 mL). The combined organics were further washed with brine, dried over sodium sulfate, filtered, and concentrated under reduced pressure. Crude sample was purified by reverse phase HPLC to afford 8-[3-chloro-4-[2-chloro-3-[6-methoxy-5-[[[(2S)-5-oxopyrrolidin-2-yl]methylamino]methyl]-2-pyridyl]phenyl]-2-pyridyl]-3-[[[(2S)-5-oxopyrrolidin-2-yl]methylamino]methyl]pyrido[1,2-a]pyrimidin-4-one (6 mg, 11 % yield) as a tan foam. MS: *m*/*z* found 727.4, 729.4 [M+H] ⁺, retention time = 1.97 min. (Method C). ¹H NMR (400 MHz, Chloroform-d) δ 9.16 - 9.09 (m, 1H), 8.73 (d, 1H), 8.35 (s, 1H), 8.18 - 8.13 (m, 1H), 7.74 (dd, 1H), 7.67 - 7.57 (m, 2H), 7.50 (t, 1H), 7.39 (d, 1H), 7.31 (dd, 1H), 7.27 (s, 1H), 6.20 (s, 1H), 6.03 (s, 1H), 4.03 (d, 3H), 3.87 (d, 2H), 3.82 (s, 2H), 3.78 (s, 1H), 2.81 (dt, 2H), 2.62 - 2.51 (m, 2H), 2.41 - 2.33 (m, 4H), 2.24 (m, 2H), 1.82 - 1.74 (m, 2H), 0.85 (d, 1H).

### Example 11: (S)-5-((((6-(2-Chloro-3-(3-chloro-2-(3-methoxy-4-(((((S)-5-oxopyrrolidin-2-yl)methyl)amino)-methyl)phenyl)-pyridin-4-yl)phenyl)-2-methoxypyridin-3-yl)methyl)amino)methyl)pyrrolidin-2-one (8)

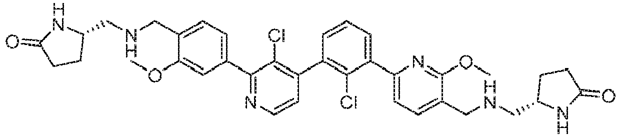

### (a) 6-(2-Chloro-3-(3-chloro-2-(4-formyl-3-methoxyphenyl)pyridin-4-yl)phenyl)-2-methoxynicotinaldehyde

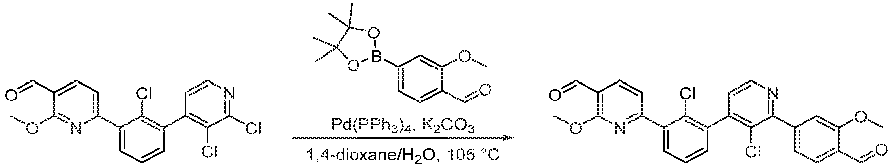

Pd(PPh₃)₄ (23 mg, 0.02 mmol), potassium carbonate (42 mg, 0.30 mmol), 6-[2-chloro-3-(2,3-dichloro-4-pyridyl)phenyl]-2-methoxy-pyridine-3-carbaldehyde (40 mg, 0.10 mmol), and 2-methoxy-4-(4,4,5,5-tetramethyl-1,3,2-dioxaborolan-2-yl)benzaldehyde (35 mg, 0.13 mmol) were suspended in 1,4-dioxane/water (4:1, 2 mL), then the solution was heated at 105 °C for 20 minutes. Reaction was cooled to rt, diluted with 3 mL water, and extracted with EtOAc (3 x 2 mL). The combined organics were concentrated under reduced pressure and crude sample was purified by normal phase SiO₂ chromatography (5-90 % EtOAc/hexane) to afford 6-[2-chloro-3-[3-chloro-2-(4-formyl-3-methoxy-phenyl)-4-pyridyl]phenyl]-2-methoxy-pyridine-3-carbaldehyde (37 mg, 73 % yield) as a yellow foam. MS: *m*/*z* found 493, 495 [M+H]⁺.

### (b) (S)-5-((((6-(2-Chloro-3-(3-chloro-2-(3-methoxy-4-(((((S)-5-oxopyrrolidin-2-yl)methyl)amino)-methyl)phenyl)-pyridin-4-yl)phenyl)-2-methoxypyridin-3-yl)methyl)amino)methyl)pyrrolidin-2-one

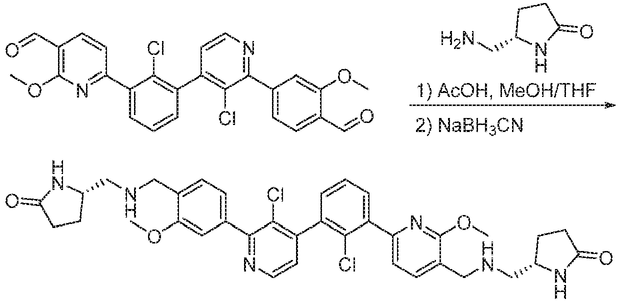

6-[2-Chloro-3-[3-chloro-2-(4-formyl-3-methoxy-phenyl)-4-pyridyl]phenyl]-2-methoxy-pyridine-3-carbaldehyde (37 mg, 0.04 mmol), (5S)-5-(aminomethyl)pyrrolidin-2-one (15 mg, 0.13 mmol), and acetic acid (5 mg, 0.09 mmol) were dissolved in MeOH/THF (1:1, 2 mL), then the solution was stirred at 25 °C for 2 hours. NaBH₃CN (8 mg, 0.13 mmol) was added and the resulting mixture was stirred at 25 °C for 1 hour. Reaction was subsequently diluted with water (2 mL) and extracted with EtOAc (3 x 2 mL). The combined organics were further washed with brine, dried over sodium sulfate, filtered, and concentrated under reduced pressure. Crude sample was purified by reverse phase HPLC to afford (5S)-5-[[[4-[3-chloro-4-[2-chloro-3-[6-methoxy-5-[[[(2S)-5-oxopyrrolidin-2-yl]methylamino]methyl]-2-pyridyl]phenyl]-2-pyridyl]-2-methoxy-phenyl]-methylamino]-methyl]pyrrolidin-2-one (27 mg, 86 % yield) as a white solid (formic acid salt). LCMS: *m*/*z* found 689.4, 691 [M+H]⁺, retention time = 2.04 min. (Method C). ¹H NMR (400 MHz, Methanol-*d*₄) δ 8.65 (dd, 1H), 7.84 (d, 1H), 7.71 (dt, 1H), 7.60 - 7.49 (m, 2H), 7.49 - 7.40 (m, 2H), 7.38 (d, 1H), 7.37 - 7.29 (m, 2H), 4.27 (d, 2H), 4.10 (d, 2H), 4.05 (d, 3H), 3.99 (d, 5H), 3.18 - 3.06 (m, 2H), 3.06 - 2.92 (m, 2H), 2.45 - 2.28 (m, 6H), 1.94 - 1.81 (m, 2H).

### Example 12: (S)-5-((((6-(2-chloro-3-(3-chloro-2-(3-fluoro-5-methoxy-4-(((((S)-5-oxopyrrolidin-2-yl)-methyl)-amino)-methyl)-phenyl)-pyridin-4-yl)-phenyl)-2-methoxypyridin-3-yl)methyl)-amino)methyl)-pyrrolidin-2-one (10)

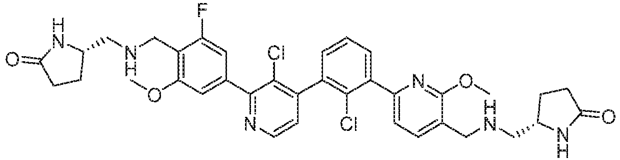

### (a) 6-(2-Chloro-3-(3-chloro-2-(3-fluoro-4-formyl-5-methoxyphenyl)-pyridin-4-yl)-phenyl)-2-methoxynicotinaldehyde

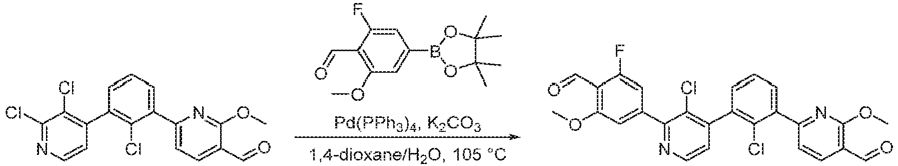

Pd(PPh₃)₄ (23 mg, 0.02 mmol), potassium carbonate (42 mg, 0.30 mmol), 6-[2-chloro-3-(2,3-dichloro-4-pyridyl)phenyl]-2-methoxy-pyridine-3-carbaldehyde (40 mg, 0.10 mmol), and 2-fluoro-6-methoxy-4-(4,4,5,5-tetramethyl-1,3,2-dioxaborolan-2-yl)benzaldehyde (37 mg, 0.13 mmol) were suspended in 1,4-dioxane/water (4:1, 2 mL), then the solution was heated at 105 °C for 20 minutes. Reaction was cooled to rt, diluted with 3 mL water, and extracted with EtOAc (3 x 2 mL). The combined organics were concentrated under reduced pressure and crude sample was purified by normal phase SiO₂ chromatography (5-90 % EtOAc/hexane) to afford 6-[2-chloro-3-[3-chloro-2-(3-fluoro-4-formyl-5-methoxyphenyl)-4-pyridyl]phenyl]-2-methoxy-pyridine-3-carbaldehyde (51 mg, 73 % yield) as a yellow foam. MS: *m*/*z* found 511, 513 [M+H]⁺.

### (b) (S)-5-((((6-(2-chloro-3-(3-chloro-2-(3-fluoro-5-methoxy-4-(((((S)-5-oxopyrrolidin-2-yl)-methyl)-amino)-methyl)-phenyl)-pyridin-4-yl)-phenyl)-2-methoxypyridin-3-yl)methyl)-amino)methyl)-pyrrolidin-2-one

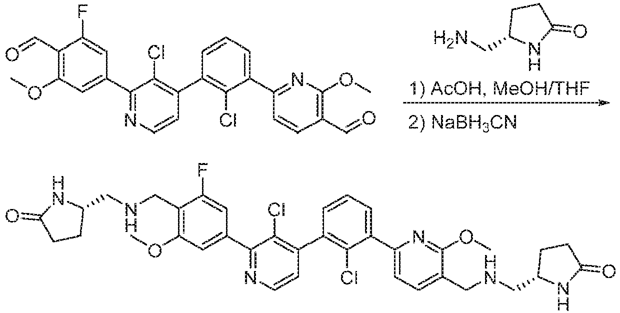

6-[2-Chloro-3-[3-chloro-2-(3-fluoro-4-formyl-5-methoxy-phenyl)-4-pyridyl]phenyl]-2-methoxy-pyridine-3-carbaldehyde (51 mg, 0.06 mmol), (5S)-5-(aminomethyl)pyrrolidin-2-one (20 mg, 0.18 mmol), and acetic acid (7 mg, 0.12 mmol) were dissolved in MeOH/THF (1:1, 2 mL), then the solution was stirred at 25 °C for 2 h. Sodium cyanoborohydride (11 mg, 0.18 mmol) was added and the resulting mixture was stirred at 25 °C for 1 hour. Reaction was subsequently diluted with water (2 mL) and extracted with EtOAc (3 x 2 mL). The combined organics were further washed with brine, dried over sodium sulfate, filtered, and concentrated under reduced pressure. Crude sample was purified by reverse phase HPLC to afford (5S)-5-[[[6-[2-chloro-3-[3-chloro-2-[3-fluoro-5-methoxy-4-[[[(2S)-5-oxopyrrolidin-2-yl]methylamino]methyl]phenyl]-4-pyridyl]phenyl]-2-methoxy-3-pyridyl]methylamino]methyl]pyrrolidin-2-one (21 mg, 49 % yield) as a white solid (formic acid salt). MS: *m*/*z* found 707.3, 709 [M+H] ⁺, LC retention time = 2.03 min. (method C). ¹H NMR (400 MHz, Methanol-*d*₄) δ 8.66 (d, 1H), 8.38 (s, 2H), 7.82 (d, 1H), 7.72 (dd, 1H), 7.56 (t, 1H), 7.48 (d, 1H), 7.42 (dd, 1H), 7.30 (d, 1H), 7.22 (s, 1H), 7.16 (d, 1H), 4.18 (s, 2H), 4.07 (d, 2H), 4.05 (s, 3H), 3.99 (s, 3H), 3.94 (s, 2H), 2.96 (m, 4H), 2.40 - 2.30 (m, 6H), 1.91 - 1.80 (m, 2H).

### Example 13: (S)-5-((((6-(2-Chloro-3-(3-chloro-2-(1-methyl-3-(((((S)-5-oxopyrrolidin-2-yl)methyl)-amino)-methyl)-1H-indol-6-yl)pyridin-4-yl)phenyl)-2-methoxypyridin-3-yl)methyl)amino)methyl)pyrrolidin-2-one (11)

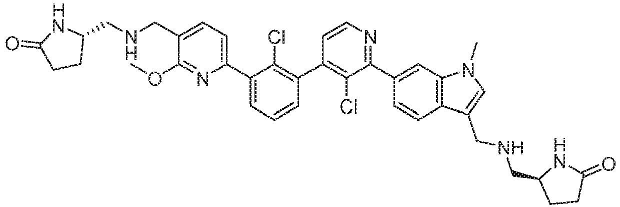

Compound was prepared in the same manner as described for compound 8, utilizing intermediate 6-(2-chloro-3-(2,3-dichloropyridin-4-yl)phenyl)-2-methoxynicotinaldehyde. 1-methyl-6-(4,4,5,5-tetramethyl-1,3,2-dioxaborolan-2-yl)-1H-indole-3-carbaldehyde was used in place of 2-methoxy-4-(4,4,5,5-tetramethyl-1,3,2-dioxaborolan-2-yl)benzaldehyde. Product was obtained as a white solid (formic acid salt). LCMS: *m*/*z* found 712.4, 714 [M+H] ⁺, retention time = 2.14 min. (Method C). ¹H NMR (400 MHz, Methanol-*d*₄) δ 8.64 (d, 1H), 8.47 (s, 2H), 7.83 (d, 1H), 7.77 (t, 2H), 7.71 (dd, 1H), 7.55 (t, 1H), 7.52 - 7.46 (m, 2H), 7.45 - 7.40 (m, 2H), 7.27 (d, 1H), 4.40 (s, 2H), 4.03 (s, 3H), 3.93 - 3.82 (m, 6H), 3.11 (d, 2H), 2.84 - 2.70 (m, 2H), 2.42 - 2.23 (m, 6H), 1.92 - 1.76 (m, 2H).

### Example 14: (S)-5-((((6-(2-Chloro-3-(3-chloro-2-(1-methyl-3-(((((S)-5-oxopyrrolidin-2-yl)methyl)-amino)methyl)-1H-pyrrolo[3,2-b]pyridin-6-yl)pyridin-4-yl)phenyl)-2-methoxypyridin-3-yl)methyl)amino)methyl)pyrrolidin-2-one (13)

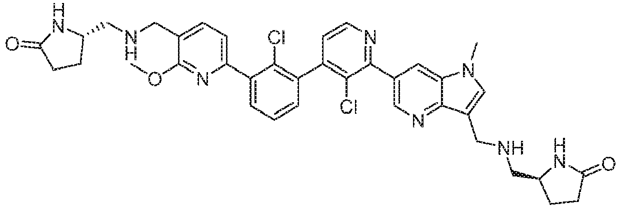

Compound was prepared in the same manner as described for compound 8, utilizing intermediate 6-(2-chloro-3-(2,3-dichloropyridin-4-yl)phenyl)-2-methoxynicotinaldehyde. 1-methyl-6-(4,4,5,5-tetramethyl-1,3,2-dioxaborolan-2-yl)-1H-pyrrolo[3,2-b]pyridine-3-carbaldehyde was used in place of 2-methoxy-4-(4,4,5,5-tetramethyl-1,3,2-dioxaborolan-2-yl)benzaldehyde. Product was obtained as a white solid. LCMS: *m*/*z* found 713.3, 715 [M+H]⁺, retention time = 1.97 min. (Method C). ¹H NMR (400 MHz, Methanol-*d*₄) δ 8.79 (d, 1H), 8.71 (d, 1H), 8.43 (s, 1H), 8.32 (d, 1H), 7.84 (s, 1H), 7.81 (d, 1H), 7.72 (dd, 1H), 7.57 (t, 1H), 7.49 (d, 1H), 7.45 (dd, 1H), 7.30 (d, 1H), 4.56 - 4.42 (m, 2H), 4.03 (d, 6H), 3.96 (s, 4H), 3.26 - 3.11 (m, 2H), 2.96 - 2.83 (m, 2H), 2.42 - 2.29 (m, 6H), 1.94 - 1.78 (m, 2H).

### Example 15: 8-(2-Chloro-3-(3-chloro-2-(3-methoxy-4-(((((S)-5-oxopyrrolidin-2-yl)methyl)-amino)methyl)-phenyl)pyridin-4-yl)phenyl)-3-(((((S)-5-oxopyrrolidin-2-yl)methyl)amino)methyl)-4H-pyrido[1,2-a]pyrimidin-4-one (1)

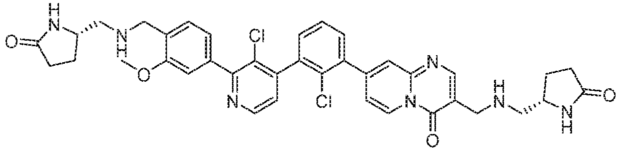

### (a) 8-(2-Chloro-3-(2,3-dichloropyridin-4-yl)phenyl)-4-oxo-4H-pyrido[1,2-a] pyrimidine-3-carbaldehyde

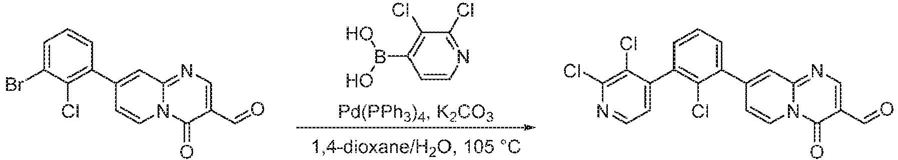

Pd(PPh₃)₄ (120 mg, 0.10 mmol), potassium carbonate, (216 mg, 1.56 mmol), (2,3-dichloro-4-pyridyl)boronic acid (100 mg, 0.52 mmol), and 8-(3-bromo-2-chloro-phenyl)-4-oxo-pyrido[1,2-a]pyrimidine-3-carbaldehyde (265 mg, 0.73 mmol) were suspended in 1,4-1,4-1,4-1,4-dioxane/water (4:1, 5 mL), then the solution was heated at 105 °C for 20 minutes. Reaction was cooled to rt, diluted with 10 ml water, and extracted with EtOAc (3 x 5 mL). The combined organics were concentrated under reduced pressure and crude sample was purified by flash chromatography (SiO₂, 5-90 % EtOAc/hexane) to afford 8-[2-chloro-3-(2,3-dichloro-4-pyridyl)phenyl]-4-oxo-pyrido[1,2-a]pyrimidine-3-carbaldehyde (74 mg, 33 % yield) as a yellow foam. MS: *m*/*z* found 430, 432 [M+H] ⁺.

### (b) 8-(2-Chloro-3-(3-chloro-2-(4-formyl-3-methoxyphenyl)pyridin-4-yl)phenyl)-4-oxo-4H-pyrido[1,2-a]pyrimidine-3-carbaldehyde

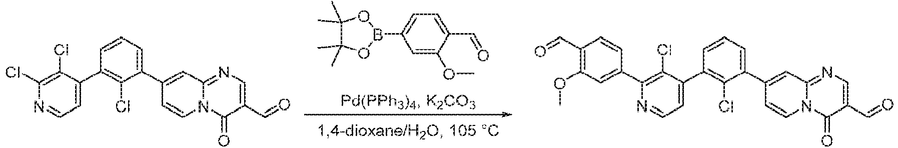

Pd(PPh₃)₄ (19 mg, 0.02 mmol), potassium carbonate (34 mg, 0.24 mmol), 2-methoxy-4-(4,4,5,5-tetramethyl-1,3,2-dioxaborolan-2-yl)benzaldehyde (25 mg, 0.10 mmol), and 8-[2-chloro-3-(2,3-dichloro-4-pyridyl)phenyl]-4-oxo-pyrido[1,2-a]pyrimidine-3-carbaldehyde (35 mg, 0.08 mmol) were suspended in 1,4-dioxane/water (4:1, 1 mL), then the solution was heated at 105 °C for 20 minutes. Reaction was cooled to rt, diluted with 3 ml water, and extracted with EtOAc (3 x 2 mL). The combined organics were concentrated under reduced pressure and crude sample was purified by normal phase SiO₂ chromatography (5-100 % EtOAc/hexane) to afford 8-[2-chloro-3-[3-chloro-2-(4-formyl-3-methoxy-phenyl)-4-pyridyl]phenyl]-4-oxo-pyrido[1,2-a]pyrimidine-3-carbaldehyde (43 mg, crude) as a yellow foam. MS: *m*/*z* found 530, 532 [M+H]⁺. Material was used for the next step without further purification.

### (c) 8-(2-Chloro-3-(3-chloro-2-(3-methoxy-4-(((((S)-5-oxopyrrolidin-2-yl)methyl)-amino)methyl)-phenyl)pyridin-4-yl)phenyl)-3-(((((S)-5-oxopyrrolidin-2-yl)methyl)amino)methyl)-4H-pyrido[1,2-a]pyrimidin-4-one

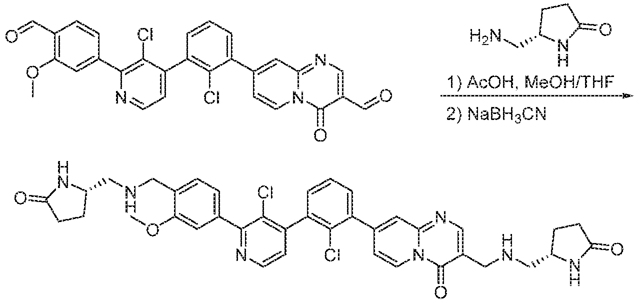

8-[2-Chloro-3-[3-chloro-2-(4-formyl-3-methoxy-phenyl)-4-pyridyl]phenyl]-4-oxo-pyrido[1,2-a]pyrimidine-3-carbaldehyde (43 mg, 0.08 mmol), (5S)-5-(aminomethyl)pyrrolidin-2-one (28 mg, 0.24 mmol), and acetic acid (10 mg, 0.16 mmol) were dissolved in MeOH/THF (1:1, 1 mL), then the solution was stirred at 25 °C for 2 hours. NaBH₃CN (10 mg, 0.16 mmol) was added and the resulting mixture was stirred at 25 °C for 1 hour. Reaction was subsequently diluted with water (2 mL) and extracted with EtOAc (3 x 2 mL). The combined organics were further washed with brine, dried over sodium sulfate, filtered, and concentrated under reduced pressure. Crude sample was purified by reverse phase HPLC to afford 8-[2-chloro-3-[3-chloro-2-[3-methoxy-4-[[[(2S)-5-oxopyrrolidin-2-yl]methylamino]methyl]phenyl]-4-pyridyl]phenyl]-3-[[[(2S)-5-oxopyrrolidin-2-yl]methylamino]methyl]pyrido[1,2-a]pyrimidin-4-one (7 mg, 12 % yield) as a white foam (formic acid salt). MS: *m*/*z* found 726.4, 728.4 [M+H] ⁺, LC retention time = 1.97 min. (Method C). ¹H NMR (400 MHz, Chloroform-d) δ 9.09 (d, 1H), 8.67 (d, 1H), 8.33 (s, 1H), 7.76 (d, 1H), 7.59 - 7.50 (m, 2H), 7.43 (dd, 1H), 7.36 (dd, 1H), 7.34 - 7.29 (m, 2H), 7.25 (s, 2H), 6.19 (s, 1H), 6.01 (s, 1H), 3.91 (d, 3H), 3.88 - 3.70 (m, 6H), 2.79 (ddd, 2H), 2.54 (ddd, 2H), 2.39 - 2.32 (m, 4H), 2.30 - 2.15 (m, 2H), 1.75 (dt, 2H).

### Example 16: (S)-5-((6-(3-Chloro-4-(2-chloro-3-(4-chloro-6-methoxy-5-(((((S)-5-oxopyrrolidin-2-yl)methyl)amino)methyl)pyridin-2-yl)phenyl)pyridin-2-yl)-8-methoxy-3,4-dihydroisoquinolin-2(1H)-yl)methyl)pyrrolidin-2-one (37)

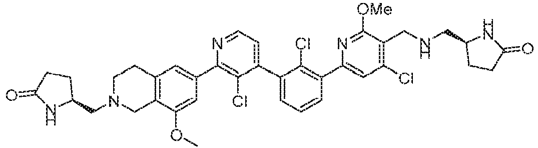

### (a) 2-chloro-6-methoxypyridin-4-amine

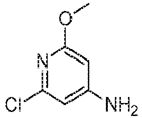

A solution of 2,6-dichloropyridin-4-amine (150 g, 0.92 mol) and sodium methoxide (60 g, 1.1 mol) in NMP (600 mL) was stirred at 140 °C for 12 h. The mixture was added to water (2 L) and extracted with EtOAc (2 x 3 L). The combined organic phase was dried over anhydrous sodium sulfate, filtered and concentrated under reduced pressure. The residue was purified by flash SiO₂ chromatography (0-20 % EtOAc/petroleum ether) to afford 2-chloro-6-methoxypyridin-4-amine (77.4 g) as a yellow solid. ¹H NMR (400 MHz, CDCl₃): δ 6.16 (s, 1H), 5.76 (s, 1H), 4.20-4.02 (m, 2H), 3.82 (s, 3H).

### (b) 6-Chloro-3-iodo-2-methoxypyridin-4-amine

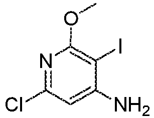

A mixture of 2-chloro-6-methoxypyridin-4-amine (50 g, 315 mmol) and N-Iodosuccinimide (74.5 g, 331 mmol) in DMF (300 mL) was stirred at rt for 1 h. The mixture was diluted with water (200 mL) and extracted with EtOAc (300 mL). The combined organic phase was dried over anhydrous sodium sulfate, filtered and concentrated. The residue was purified by flash SiO₂ chromatography (0-20 % EtOAc/petroleum ether) to afford 6-chloro-3-iodo-2-methoxy-pyridin-4-amine (90 g) as a yellow solid. ¹H NMR (400 MHz, CDCl₃): δ 6.51-6.41 (m, 2H), 6.36 (s, 1H), 3.79 (s, 3H).

### (c) 4,6-Dichloro-3-iodo-2-methoxypyridine

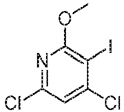

To a solution of copper chloride (5.67 g, 42.2 mmol) and *tert-butyl* nitrite (5.44 g, 52.7 mmol) in CH₃CN (80 mL) was added 6-chloro-3-iodo-2-methoxypyridin-4-amine (10 g, 35.2 mmol) in CH₃CN (40 mL) dropwise at 0 °C. The mixture was stirred at rt for 12 h. The reaction mixture was then diluted with water (50 mL) and extracted with EtOAc (2 x 100 ml). The combined organic phase was dried with anhydrous sodium sulfate, filtered and concentrated. The residue was purified by flash SiO₂ chromatography (0-3% EtOAc/petroleum ether) to afford 4,6-dichloro-3-iodo-2-methoxypyridine (9 g, 84% yield) as a yellow solid. ¹H NMR (400 MHz, DMSO-*d*₆): δ 7.45 (s, 1H), 3.92 (s, 3H).

### (d) 4,6-Dichloro-2-methoxynicotinaldehyde

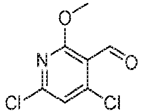

To a solution of 4,6-dichloro-3-iodo-2-methoxypyridine (4.5 g, 14.8 mmol) in THF (60 mL) was added n-BuLi (11.85 mL, 2.5 mol/L) at -70°C. The mixture was stirred at -70 °C for 0.5 h, followed by addition of ethyl formate (2.19 g, 29.6 mmol). The reaction was stirred at -70 °C for 0.5 h. The mixture was combined with another batch at the same scale. The reaction mixture was diluted with water (50 mL) and extracted with EtOAc (2 x 200 ml). The combined organic layers were dried over anhydrous sodium sulfate, filtered and concentrated under reduced pressure. The residue was purified by flash SiO₂ chromatography (0-5% EtOAc/petroleum ether) to afford 4,6-dichloro -2-methoxynicotinaldehyde (5 g, 82% yield) as a white solid. NMR (400 MHz, DMSO-*d*₆): δ 10.32 (s, 1H), 6.98 (s, 1H), 4.01 (s, 3H).

### (f) 6-(3-Bromo-2-chlorophenyl)-4-chloro-2-methoxynicotinaldehyde

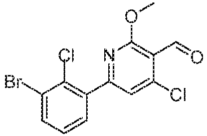

To a mixture of 4,6-dichloro-2-methoxynicotinaldehyde (4.9 g, 23.8 mmol), 2-(3-bromo-2-chlorophenyl)-4,4,5,5-tetramethyl-1,3,2-dioxaborolane (7.17 g, 22.6 mmol) in dioxane/ H₂O (5:1, 60 mL) was added cesium carbonate (23.3 g, 71.4 mmol) and tetrakis(triphenylphosphine)palladium (1.37 g, 1.19 mmol). The mixture was stirred at 100 °C for 2 h under a nitrogen atmosphere. The reaction mixture was diluted with water (50 mL) and extracted with EtOAc (2 x 200 ml). The combined organic phase was dried with anhydrous sodium sulfate, filtered and concentrated. The residue was triturated with EtOAc/petroleum ether (1/5, 60mL) at rt for 0.5 h to afford 6-(3-bromo-2-chlorophenyl)-4-chloro-2-methoxy nicotinaldehyde (5.3 g, 62% yield) as a white solid. ¹H NMR (400 MHz, DMSO-*d*₆): δ 10.36 (s, 1H), 7.94 (d, 1H), 7.67 (d, 1H), 7.56 (s, 1H), 7.45 (t, 1H), 4.02 (s, 3H).

### (g) (S)-5-((((6-(3-Bromo-2-chlorophenyl)-4-chloro-2-methoxypyridin-3-yl)methyl)amino)methyl)pyrrolidin-2-one

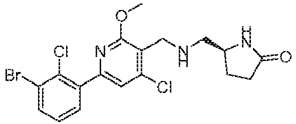

To a mixture of 6-(3-bromo-2-chlorophenyl)-4-chloro-2-methoxynicotinaldehyde (13.8 g, 38.2 mmol), (S)-5-(aminomethyl)pyrrolidin-2-one, HCl (11.5 g, 76.4 mmol) in methylene chloride / trimethyl orthoformate (15:1 (*v*/*v*)*,* 160 mL) was added sodium acetate (12.5 g, 153 mmol). The mixture was stirred at rt for 1 h under a nitrogen atmosphere. Sodium triacetoxyborohydride (24.3 g, 115 mmol) was added and the mixture was stirred at rt for 0.5 h under N₂. The mixture was concentrated to afford (S)-5-((((6-(3-bromo-2-chlorophenyl)-4-chloro-2- methoxypyridin-3-yl)methyl)amino)methyl)pyrrolidin-2-one (17.5 g, crude, m/z: 458 [M+H]⁺). The crude product was used directly without further purification.

### (h) (S)-tert-Butyl ((6-(3-bromo-2-chlorophenyl)-4-chloro-2-methoxypyridin -3-yl)methyl) ((5-oxopyrrolidin-2-yl)methyl)carbamate

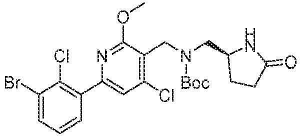

To a mixture of (S)-5-((((6-(3-bromo-2-chlorophenyl)-4-chloro-2-methoxypyridin -3-yl)methyl)amino)methyl)pyrrolidin-2-one (17.5 g, 38.1 mmol) and *tert*-butoxycarbonyl *tert-*butyl carbonate (26.6 g, 122 mmol) in methylene chloride (150 mL) was added TEA (13.3 mL, 95.3 mmol). The mixture was stirred at rt for 1 h under N₂. The mixture was filtered, and the filtrate was concentrated. The residue was purified by flash chromatography (SiO₂, 0-80% EtOAc/petroleum ether) to afford (S)-tert-butyl ((6-(3-bromo-2-chlorophenyl) -4-chloro-2-methoxypyridin-3-yl)methyl)((5-oxopyrrolidin-2-yl)methyl)carbamate (15 g, 70%) as a white solid. ¹H NMR (400 MHz, DMSO-*d*₆): δ 7.95 (dd, 1H), 7.86 (s, 1H), 7.66 (s, 1H), 7.49-7.45 (m, 2H), 4.74-4.58 (m, 2H), 3.82 (s, 3H), 3.82-3.77 (s, 1H), 3.33 (s, 1H), 3.12-3.00 (m, 1H), 2.19-2.08 (m, 3H), 1.81-1.74 (m, 1H), 1.47-1.33 (m, 9H).

### (j) (S)-tert-Butyl ((4-chloro-6-(2-chloro-3-(2,3-dichloropyridin-4-yl)phenyl) -2-methoxy pyridin-3-yl)methyl)((5-oxopyrrolidin-2-yl)methyl)carbamate

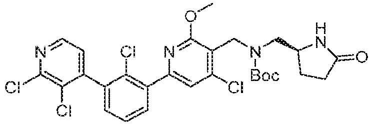

To a mixture of (S)-tert-butyl ((6-(3-bromo-2-chlorophenyl)-4-chloro-2- methoxy pyridin-3-yl) methyl)((5-oxopyrrolidin-2-yl)methyl)carbamate (8 g, 14.3 mmol), potassium carbonate (5.93 g, 42.9 mmol), [1,1-bis(diphenylphosphino)ferrocene] palladium(II) chloride dichloromethane complex (584 mg, 0.72 mmol) in dioxane/ H₂O (6:1, 140 mL), (2,3-dichloropyridin-4-yl)boronic acid (3.02 g, 15.7 mmol) was added. The mixture was stirred at 95 °C for 2 h under N₂. The reaction mixture was diluted with water (100 mL) and extracted with EtOAc (2 x 200 ml). The combined organic layers were dried over anhydrous sodium sulfate, filtered and concentrated. The residue was purified by flash chromatography (SiO₂, 0-3% EtOAc/petroleum ether) to afford (S)-tert-butyl ((4-chloro-6-(2-chloro-3-(2,3-dichloropyridin-4-yl)phenyl)-2-methoxypyridin-3-yl)methyl)((5-oxopyrrolidin-2-yl)methyl)carbamate (8.5 g) as a yellow solid. ¹H NMR (400 MHz, DMSO-*d*₆): δ 8.57 (d, 1H), 7.82 (s, 2H), 7.69-7.63 (m, 2H), 7.58 (dd, 1H), 7.46 (s, 1H), 4.73-4.62 (m, 2H), 3.98 (s, 3H), 3.81-3.80 (m, 1H), 3.32 (s, 1H), 3.13 (s, 1H), 2.19-2.07 (m, 3H), 1.73 (s, 1H), 1.46-1.32 (m, 9H).

### (k) (S)-5-((6-Bromo-8-methoxy-3,4-dihydroisoquinolin-2(1H)-yl)methyl)pyrrolidin-2-one

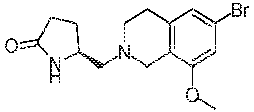

To a mixture of 6-bromo-8-methoxy-1,2,3,4-tetrahydroisoquinoline, HCl (500 mg, 1.79 mmol) in MeCN (100 mL) was added (5S)-5-(bromomethyl)pyrrolidin-2-one (415 mg, 2.33 mmol) and potassium carbonate (744 mg, 5.38 mmol). The mixture was stirred at 100 °C for 12 h under. To this mixture, (5S)-5-(bromomethyl)pyrrolidin-2-one (63.9 mg, 0.36 mmol) was added and the mixture stirred for another 12 h at 100 °C. The reaction mixture was diluted with water (100 mL) and extracted with EtOAc (200 ml). The combined organic layers were dried over anhydrous sodium sulfate, filtered and concentrated under reduced pressure. The residue was purified by flash chromatography (SiO₂, 0-10% THF/EtOAc) to afford (S)-5-((6-bromo-8-methoxy-3,4-dihydroisoquinolin-2(1H)-yl) methyl)pyrrolidin-2-one (0.6 g, 98%yield, m/z: 339, 341 [M+H]⁺) as a yellow solid.

### (l) (S)-5-((8-Methoxy-6-(4,4,5,5-tetramethyl-1,3,2-dioxaborolan-2-yl)-3,4-dihydroisoquinolin-2(1H)-yl)methyl)pyrrolidin-2-one

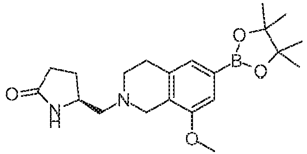

To a mixture of (S)-5-((6-bromo-8-methoxy-3,4-dihydroisoquinolin-2(1H)-yl)methyl) pyrrolidin-2-one (460 mg, 1.36 mmol), 4,4,4',4',5,5,5',5'-octamethyl- 2,2'-bi(1,3,2-dioxa borolane) (1.03 g, 4.07 mmol) in dioxane (8 mL) was added potassium acetate (266 mg, 2.71 mmol) and [1,1'-bis(di-*tert*-butylphosphino)ferrocene] dichloropalladium(II) (111 mg, 0.14 mmol). The mixture was stirred at 130 °C for 3 h under N₂. The mixture was filtered and washed with EtOAc (20 mL) and concentrated. The residue was purified by flash chromatography (SiO₂, 0-100% EtOAc / Petroleum ether gradient in THF (20%)) to afford (S)-5-((8-methoxy-6- (4,4,5,5 -tetramethyl-1,3,2-dioxaborolan-2-yl) -3,4-dihydroisoquinolin - 2(1H)-yl)methyl) pyrrolidin-2-one (0.49 g, 65.48% yield, m/z: 387 [M+H]⁺, observed).

### (m) tert-Butyl((4-chloro-6-(2-chloro-3-(3-chloro-2-(8-methoxy-2-(((S)-5-oxopyrrolidin-2-yl)methyl)-1,2,3,4-tetrahydroisoquinolin-6-yl)pyridin-4-yl)phenyl)-2-methoxypyridin-3-yl)methyl)(((S)-5-oxopyrrolidin-2-yl)methyl)carbamate

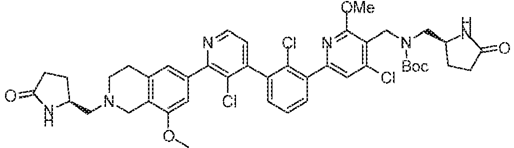

To a mixture of (S)-5-((8-methoxy-6-(4,4,5,5-tetramethyl-1,3,2-dioxaborolan-2-yl) - 3,4-dihydroisoquinolin-2(1H)-yl)methyl)pyrrolidin-2-one (250 mg, 0.65 mmol), (S)*-tert-*butyl ((4-chloro-6-(2-chloro-3-(2,3-dichloropyridin-4-yl)phenyl) -2-methoxy pyridin-3-yl)methyl)((5-oxopyrrolidin-2-yl)methyl)carbamate (324 mg, 0.52 mmol) in dioxane/ H₂O (6:1, 7 mL) was added potassium carbonate (268 mg, 1.94 mmol) and [1,1-bis(diphenylphosphino)ferrocene]palladium(II) chloride dichloromethane complex (52.8 mg, 0.06 mmol). The mixture was stirred at 110 °C for 0.5 h under N₂. The reaction mixture was diluted with water (10 mL) and extracted with EtOAc (2 x 30 mL). The combined organic layer was dried over anhydrous sodium sulfate, filtered and concentrated. The residue was purified by flash chromatography (SiO₂, 0-100% EtOAc/petroleum ether) to afford *tert*-butyl-((4-chloro-6-(2-chloro-3-(3-chloro-2 -(8-methoxy-2-(((S) -5-oxopyrrolidin-2-yl) methyl)-1,2,3,4-tetrahydroisoquinolin-6-yl) pyridin-4-yl)phenyl) -2-methoxypyridin-3-yl)methyl)(((S)-5-oxopyrrolidin-2-yl)methyl) carbamate (250 mg, 45% yield, m/z: 849 [M+H]⁺, observed).

### (n) (S)-5-((6-(3-Chloro-4-(2-chloro-3-(4-chloro-6-methoxy-5-(((((S)-5-oxopyrrolidin-2-yl)methyl)amino)methyl)pyridin-2-yl)phenyl)pyridin-2-yl)-8-methoxy-3,4-dihydroisoquinolin-2(1H)-yl)methyl)pyrrolidin-2-one

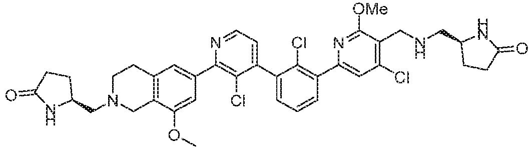

To a solution of *tert-butyl* ((4-chloro-6-(2-chloro-3-(3-chloro-2-(8-methoxy-2-(((S) - 5-oxopyrrolidin-2-yl)methyl)-1,2,3,4-tetrahydroisoquinolin-6-yl)pyridin-4-yl)phenyl)-2-methoxypyridin-3-yl)methyl)(((S)-5-oxopyrrolidin-2-yl)methyl)carbamate (240 mg, 0.28 mmol) in methylene chloride (10 mL) was added TFA (5 mL, 67.53 mmol). The mixture was stirred at rt for 0.5 h. The mixture was concentrated and the residue was purified by reverse phase HPLC to afford (S)-5-((6-(3-chloro-4-(2-chloro-3-(4-chloro-6-methoxy -5-(((((S)-5 - oxopyrrolidin-2-yl)methyl)amino)methyl)pyridin-2-yl)phenyl)pyridin-2-yl)-8-methoxy-3,4-dihydroisoquinolin-2(1H)-yl)methyl)pyrrolidin-2-one (74.8 mg, 32% yield, m/z: 749 [M+H]⁺ observed) as a white solid. ¹H NMR (400 MHz, DMSO-*d*₆): δ8.74 (d, 1H), 7.77-7.75 (m, 3H), 7.66-7.54 (m, 4H), 7.19 (s, 2H), 4.63 (m, 1H), 4.43-4.14 (m, 4H), 4.02 (s, 3H), 3.92 (s, 3H), 3.89-3.70 (m, 3H), 3.27-3.10 (m, 2H), 2.27-2.17 (m, 6H), 1.82-1.79 (m, 2H).

### Example 17: (S)-5-((((6-(2-Chloro-3-(3-chloro-2-(8-fluoro-2-(((S)-5-oxopyrrolidin-2-yl)methyl)-1,2,3,4-tetrahydroisoquinolin-6-yl)pyridin-4-yl)phenyl)-2-methoxypyridin-3-yl)methyl)amino)methyl)pyrrolidin-2-one (61)

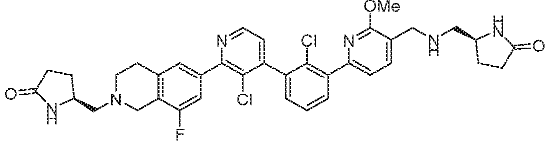

### (a) (S)-5-((6-Bromo-8-fluoro-3,4-dihydroisoquinolin-2(1H)-yl)methyl)pyrrolidin-2-one

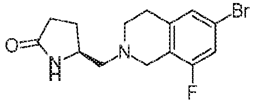

To a mixture of 6-bromo-8-fluoro-1,2,3,4-tetrahydroisoquinoline (0.35 g, 1.52 mmol) and (S)-5-(bromomethyl)pyrrolidin-2-one (0.35 g, 1.98 mmol) in MeCN (10 mL) was added potassium carbonate (0.63 g, 4.56 mmol). The mixture was stirred at 100 °C for 12 h. The mixture was filtered, and the filtrate was concentrated. The residue was purified by flash chromatography (SiO₂, 50-100% ethyl acetate/petroleum ether to 10% MeOH/ethyl acetate) to afford (5S)-5-[(6-bromo-8-fluoro -3,4-dihydro-1H -isoquinolin-2-yl)methyl]pyrrolidin-2-one as a yellow solid (0.32 g, 61% yield). ¹H NMR (400 MHz, Methanol-*d*₄): δ 7.17 (s, 1H), 7.12 (d, 1H), 4.03-3.97 (m, 1H), 3.76-3.58 (m, 2H), 2.95-2.92 (m, 2H), 2.89-2.84 (m, 1H), 2.78-2.72 (m, 1H), 2.70-2.58 (m, 2H), 2.42-2.26 (m, 3H), 1.91-1.83 (m, 1H).

### (b) (S)-5-((8-Fluoro-6-(4,4,5,5-tetramethyl-1,3,2-dioxaborolan-2-yl)-3,4-dihydroisoquinolin-2(1H)-yl)methyl)pyrrolidin-2-one

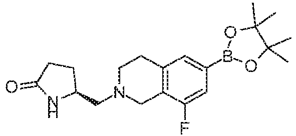

To a mixture of (S)-5-((6-bromo-8-fluoro-3,4-dihydroisoquinolin-2(1H)-yl)methyl) pyrrolidin-2-one (0.27 g, 0.84 mmol) and bis(pinacolato)diboron (0.64 g, 2.52 mmol) in dioxane (4 mL) was added potassium acetate (0.25 g, 2.52 mmol) and 1,1'-bis(diphenylphosphino)ferrocene-palladium(II) dichloride dichloromethane complex (0.07 g, 0.08 mmol). The mixture was stirred at 130 °C for 12 h. The solvent was evaporated, and the residue was purified by flash chromatography (SiO₂, 50-100% ethyl acetate/petroleum ether to 10% MeOH/ethyl acetate) to afford (S)-5-((8-fluoro-6- (4,4,5,5-tetramethyl-1,3,2-dioxaborolan-2-yl)-3,4-dihydroisoquinolin-2(1H)-yl)methyl) pyrrolidin-2-one (0.3 g, 95% yield). ¹H NMR (400 MHz, Methanol-*d*₄): δ 7.35 (s, 1H), 7.18 (d, 1H), 4.04-4.01 (m, 1H), 3.83-3.71 (m, 2H), 2.98-2.88 (m, 3H), 2.82-2.78 (m, 1H), 2.76-2.61 (m, 2H), 2.40-2.29 (m, 3H), 1.92-1.82 (m, 1H), 1.36 (m, 12H).

### (c) (S)-tert-Butyl((6-(3-bromo-2-chlorophenyl)-2-methoxypyridin-3-yl)methyl) ((5-oxopyrrolidin-2-yl)methyl)carbamate

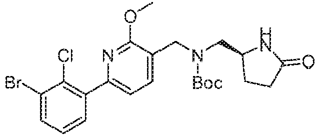

A mixture of 6-(3-bromo-2-chlorophenyl)-2-methoxynicotinaldehyde (20 g, 61 mmol), (5S)-5-(aminomethyl)pyrrolidin-2-one, HCl (14 g, 92 mmol) and sodium acetate (13 g, 153 mmol) in methylene chloride/trimethoxymethane (5:1, 300 mL) was stirred at rt for 4 h. Sodium triacetyloxyboranuide (26g, 122 mmol) was added to the mixture and stirred at rt for 2 h under N₂. To this solution was added di-tert-butyl decarbonate (42 mL, 183 mmol) and triethylamine (30 mL, 214 mmol) in methylene chloride (80 mL). The mixture was stirred at rt for 1 h under N₂. The reaction mixture was filtered, and the filtrate was concentrated under reduced pressure. The residue was purified by flash chromatography (SiO₂, 0-20% ethyl acetate/petroleum ether) to afford (S)-tert-butyl ((6-(3-bromo-2-chlorophenyl)-2- methoxypyridin-3-yl)methyl)((5-oxopyrrolidin-2-yl)methyl)carbamate as a yellow solid (20 g, 62% yield, 526 [M+H]⁺ observed).

### (d) (S)-tert-Butyl((6-(2-chloro-3-(2,3-dichloropyridin-4-yl)phenyl)-2-methoxypyridine -3-yl)methyl)((5-oxopyrrolidin-2-yl)methyl)carbamate

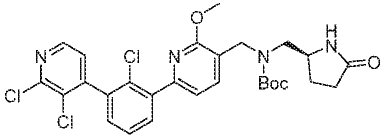

To a mixture of (S)-*tert*-butyl ((6-(3-bromo-2-chlorophenyl)-2-methoxypyridin-3-yl) methyl)((5-oxopyrrolidin-2-yl)methyl)carbamate (12 g, 23 mmol), 1,1'-bis(diphenylphosphino)ferrocene-palladium(II) dichloride dichloromethane complex (0.93 g, 1.14 mmol) and potassium carbonate (9.5 g, 68 mmol) in dioxane/H₂O mixture (6:1, 140 mL), (2,3-dichloro-4-pyridyl)boronic acid (3.5 g, 18 mmol) in dioxane (150 mL) was added. The mixture was stirred at 95 °C for 1 h under a nitrogen atmosphere. The mixture was diluted with water (50 mL) and extracted with EtOAc (2 x 200mL). The combined organic layers were dried with anhydrous sodium sulfate, filtered and concentrated. The residue was purified by flash chromatography (SiO₂, 0-15% EtOAc/petroleum ether) to afford (S)-*tert-*butyl ((6-(2-chloro-3-(2,3-dichloropyridin-4-yl) phenyl)-2-methoxypyridin-3-yl)methyl)((5-oxopyrrolidin-2-yl)methyl)carbamate as a yellow solid (7 g, m/z: 591 [M+H]⁺ observed). ¹H NMR (400 MHz, DMSO-*d*₆): δ. 8.51 (d, 1H), 7.77-7.73 (m, 2H), 7.65-7.56 (m, 2H), 7.50-7.44 (m, 2H), 7.31-7.29 (m, 1H), 4.47-4.35 (m, 2H), 3.94 (s, 3H), 3.78-3.76 (m, 1H), 3.33-3.27 (m, 2H), 2.22-2.04 (m, 3H), 1.73-1.72 (m, 1H), 1.44-1.30 (m, 9H).

### (e) tert-Butyl ((6-(2-chloro-3-(3-chloro-2-(8-fluoro-2-(((S)-5-oxopyrrolidin-2-yl)methyl) -1,2,3,4-tetrahydroisoquinolin-6-yl)pyridin-4-yl)phenyl)-2-methoxypyridin-3-yl)methyl)(((S)-5-oxopyrrolidin-2-yl)methyl)carbamate

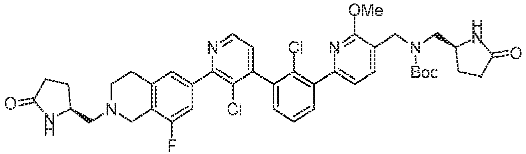

To a mixture of (S)-5-((8-fluoro-6-(4,4,5,5-tetramethyl-1,3,2-dioxaborolan -2-yl)-3,4 - dihydroisoquinolin-2(1H)-yl)methyl)pyrrolidin-2-one (0.24 g, 0.63 mmol) and (S)*-tert-*butyl((6-(2-chloro-3-(2,3-dichloropyridin-4-yl)phenyl)-2-methoxy pyridin-3-yl)methyl)((5-oxopyrrolidin-2-yl)methyl)carbamate (0.25 g, 0.42 mmol) in dioxane/H₂O mixture (25:1, 5.2 mL) was added 1,1'-bis(diphenylphosphino)ferrocene -palladium(II) dichloride dichloromethane complex (0.02 g, 0.02 mmol) and potassium carbonate (0.18 g, 1.27 mmol). The mixture was stirred at 110 °C for 2.5 h. To the mixture was added water (15 mL). The mixture was extracted with ethyl acetate (2 x 15 mL). The combined organic phase was washed with brine (2 x 15 mL), dried over anhydrous sodium sulfate, filtered and concentrated. The residue was purified by flash chromatography (SiO₂, 50-100% ethyl acetate/petroleum ether, then to 10% MeOH/ethyl acetate) to afford *tert-butyl* ((6-(2-chloro-3-(3-chloro-2-(8-fluoro-2-(((S)-5- oxopyrrolidin-2-yl)methyl)-1,2,3,4-tetrahydroisoquinolin-6-yl)pyridin-4-yl)phenyl)-2-methoxypyridin-3-yl)methyl)(((S)-5-oxopyrrolidin-2-yl)methyl)carbamate (50 mg, 6% yield, m/z: 803 [M+H]⁺ observed).

### (f) (S)-5-((((6-(2-Chloro-3-(3-chloro-2-(8-fluoro-2-(((S)-5-oxopyrrolidin-2-yl)methyl)-1,2,3,4-tetrahydroisoquinolin-6-yl)pyridin-4-yl)phenyl)-2-methoxypyridin-3-yl)methyl)amino)methyl)pyrrolidin-2-one

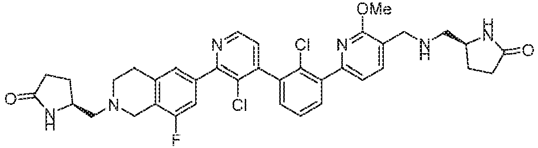

To a mixture of *tert*-butyl((6-(2-chloro-3-(3-chloro-2-(8-fluoro-2-(((S) -5-oxopyrrolidin -2-yl)methyl)-1,2,3,4-tetrahydroisoquinolin-6-yl)pyridin-4-yl)phenyl)-2-methoxypyridin-3-yl)methyl)(((S)-5-oxopyrrolidin-2-yl)methyl)carbamate (0.04 g, 0.05 mmol) in CH₂Cl₂ (0.5 mL) was added trifluoroacetic acid (0.8 mL). The mixture was stirred at rt for 0.5 h. The mixture was concentrated. The residue was purified by reverse phase HPLC to afford (S)-5-((((6-(2-chloro-3-(3-chloro-2-(8-fluoro-2-(((S)-5-oxopyrrolidin-2-yl)methyl)-1,2,3,4-tetrahydroisoquinolin-6-yl)pyridin-4-yl)phenyl)-2-methoxypyridin-3-yl)methyl)amino)methyl)pyrrolidin-2-one as a yellow solid (8.1 mg, 18% yield, m/z: 703 [M+H]+ observed). ¹H NMR (400 MHz, DMSO-*d*₆): δ 8.71 (d, 1H), 7.85 (d, 1H), 7.75-7.65 (m, 3H), 7.60 (t, 1H), 7.54-7.48 (m, 2H), 7.32-7.28 (m, 3H), 3.93 (s, 3H), 3.84-3.63 (m, 6H), 2.93 (m, 2H), 2.77 (m, 2H), 2.61-2.56 (m, 4H), 2.23-2.08 (m, 6H), 1.79-1.67 (m, 2H).

### Example 18: (S)-5-((((6-(2-Chloro-3-(3-chloro-2-(8-fluoro-2-(3-fluoropropyl)-1,2,3,4-tetrahydroisoquinolin-6-yl)pyridin-4-yl)phenyl)-2-methoxypyridin-3-yl)methyl)amino)methyl)pyrrolidin-2-one (65)

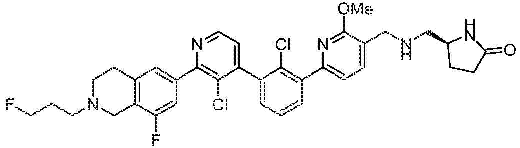

### (a) 6-Bromo-8-fluoro-2-(3-fluoropropyl)-1,2,3,4-tetrahydroisoquinoline

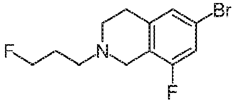

To a mixture of 6-bromo-8-fluoro-1,2,3,4-tetrahydroisoquinoline (0.29 g, 1.26 mmol) and 1-bromo-3-fluoro-propane (0.27 g, 1.89 mmol) in MeCN (10 mL) was added potassium carbonate (0.52 g, 3.78 mmol). The mixture was stirred at 100 °C for 12 h. The mixture was filtered, and the filtrate was concentrated. The residue was purified by flash chromatography (SiO₂, 0-10% ethyl acetate/petroleum ether) to afford 6-bromo-8-fluoro-2-(3-fluoropropyl)-1,2,3,4-tetrahydroisoquinoline as a yellow oil (0.23 g, 48% yield). ¹H NMR (400 MHz, CDCl₃): δ 7.00 (s, 1H), 6.95 (d, 1H), 4.54 (t, 1H), 4.42 (t, *J =* 6 Hz, 1H), 3.50 (s, 2H), 2.82-2.80 (m, 2H), 2.66-2.60 (m, 4H), 1.97-1.86 (m, 2H).

### (b) 8-Fluoro-2-(3-fluoropropyl)-6-(4,4,5,5-tetramethyl-1,3,2-dioxaborolan-2-yl)-1,2,3,4-tetrahydroisoquinoline

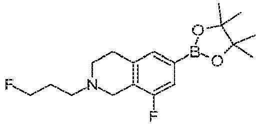

To a mixture of 6-bromo-8-fluoro-2-(3-fluoropropyl)-1,2,3,4-tetrahydroisoquinoline (0.21 g, 0.72 mmol) and bis(pinacolato)diboron (0.55 g, 2.17 mmol) in dioxane (4 mL) was added 1,1'-bis(diphenylphosphino)ferrocene-palladium(II)dichloride dichloromethane complex (0.03 g, 0.036 mmol) and potassium acetate (0.21 g, 2.17 mmol). The mixture was stirred at 120 °C for 12 h. The solvent was evaporated, and the residue was purified by flash chromatography (SiO₂, 0-10% ethyl acetate/petroleum ether) to afford 8-fluoro-2-(3-fluoropropyl)-6-(4,4,5,5-tetramethyl-1,3,2-dioxaborolan-2-yl)-1,2,3,4-tetrahydroisoquinoline as a yellow oil (0.2 g, 81% yield). ¹H NMR (400 MHz, CDCl₃): δ 7.28 (s, 1H), 7.19-7.16 (m, 1H), 4.55 (t, 1H), 4.43 (t, 1H), 3.60 (s, 2H), 2.85 (m, 2H), 2.67-2.63 (m, 4H), 1.98-1.91 (m, 2H), 1.27 (s, 12H).

### (c) (S)-tert-Butyl ((6-(2-chloro-3-(3-chloro-2-(8-fluoro-2-(3-fluoropropyl)-1,2,3,4-tetrahydroisoquinolin-6-yl)pyridin-4-yl)phenyl)-2-methoxypyridin-3-yl)methyl)((5-oxopyrrolidin-2-yl)methyl)carbamate

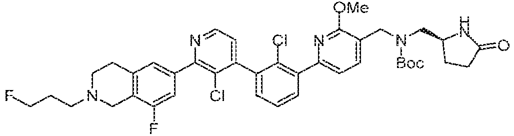

To a mixture of 8-fluoro-2-(3-fluoropropyl)-6-(4,4,5,5-tetramethyl -1,3,2-dioxaborolan-2-yl) -1,2,3,4-tetrahydroisoquinoline (0.18 g, 0.55 mmol) and (S)-tert-butyl ((6-(2-chloro-3- (2,3-dichloropyridin-4-yl)phenyl) -2-methoxypyridin-3-yl)methyl)((5-oxopyrrolidin-2-yl) methyl)carbamate (0.18 g, 0.3 mmol) in dioxane/H₂O mixture (40:3, 4.3 mL) was added 1,1'-bis(diphenylphosphino)ferrocene-palladium(II) dichloride dichloromethane complex (0.025 g, 0.03 mmol) and potassium carbonate (0.13 g, 0.91 mmol). The mixture was stirred at 110 °C for 4 h. The mixture was diluted with water (50 mL) and extracted with ethyl acetate (2 x 50 mL). The combined organic phase was dried with anhydrous sodium sulfate, filtered and concentrated. The residue was purified by flash chromatography (SiO₂, 0-100% ethyl acetate/petroleum ether) to afford (S)-*tert*-butyl ((6-(2-chloro-3-(3-chloro-2-(8-fluoro-2-(3-fluoropropyl)-1,2,3,4-tetrahydroisoquinolin-6-yl)pyridin-4-yl)phenyl)-2-methoxypyridin-3-yl)methyl)((5-oxopyrrolidin-2-yl)methyl)carbamate as a yellow solid (0.14 g, 40% yield, m/z: 766 [M+H]⁺ observed).

### (d) (S)-5-((((6-(2-Chloro-3-(3-chloro-2-(8-fluoro-2-(3-fluoropropyl)-1,2,3,4-tetrahydroisoquinolin-6-yl)pyridin-4-yl)phenyl)-2-methoxypyridin-3-yl)methyl)amino)methyl)pyrrolidin-2-one

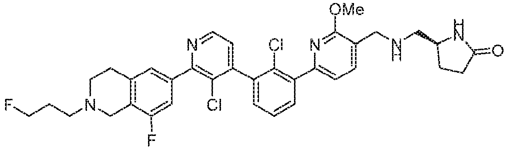

To a mixture of (S)-*tert*-butyl ((6-(2-chloro-3-(3-chloro-2-(8-fluoro-2-(3-fluoropropyl) -1,2,3,4-tetrahydroisoquinolin-6-yl)pyridin-4-yl)phenyl)-2-methoxypyridin-3-yl)methyl)((5-oxopyrrolidin-2-yl)methyl)carbamate (0.13 g, 0.17 mmol) in CH₂Cl₂ (0.5 mL) was added trifluoroacetic acid (2 mL, 27 mmol). The mixture was stirred at rt for 0.5 h. The mixture was concentrated, and the residue was purified by reverse phase HPLC to afford (S)-5-((((6-(2-chloro-3-(3-chloro-2-(8-fluoro-2-(3-fluoropropyl)-1,2,3,4-tetrahydroisoquinolin-6-yl)pyridin-4-yl)phenyl)-2-methoxypyridin-3-yl)methyl)amino)methyl)pyrrolidin-2- one (25.1 mg, 19% yield, m/z: 666 [M+H]⁺ observed). ¹H NMR (400 MHz, DMSO): δ 8.71 (d, 1H), 7.85 (d, 1H), 7.74-7.69 (m, 2H), 7.60 (t, 1H), 7.53-7.48 (m, 2H), 7.32-7.28 (m, 3H), 4.60 (t, 1H), 4.48 (t, 1H), 3.93 (s, 3H), 3.78-3.70 (m, 2H), 3.67-3.64 (m, 3H), 2.92-2.90 (m, 2H), 2.74-2.71 (m, 2H), 2.64 (t, 2H), 2.58 (d, 2H), 2.16-2.09 (m, 3H), 1.99-1.89 (m, 2H), 1.72-1.67 (m, 1H).

### Example 19: (S)-tert-Butyl((6-(2-chloro-3-(3-chloro-2-(8-chloro-2-(3-fluoropropyl)-1,2,3,4-tetra hydroisoquinolin-6-yl)pyridin-4-yl)phenyl)-2-methoxypyridin-3-yl)methyl) ((5-oxopyrrolidin-2-yl) methyl)carbamate (72)

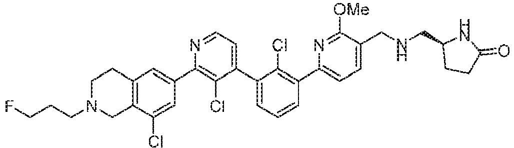

### (a) N-(4-Bromo-2-chlorobenzyl)-2,2-dimethoxyethanamine

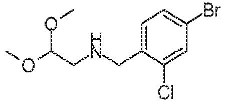

To a mixture of 4-bromo-2-chloro-benzaldehyde (34 g, 155 mmol) and 2,2-dimethoxy ethanamine (16.28 g, 155 mmol) in ethyl alcohol (500 mL) was added sodium cyanoborohydride (7.04 g, 186 mmol). The mixture was stirred at rt for 12 h. To the mixture was added aqueous ammonium chloride solution (200 mL). The mixture was extracted with ethyl acetate (2 x 100 mL). The organic phase was dried with anhydrous sodium sulfate, filtered and concentrated. The residue was purified by flash SiO₂ chromatography (0-100% ethyl acetate/petroleum ether) to afford N-[(4-bromo-2-chloro -phenyl)methyl]-2,2-dimethoxy-ethanamine as a white solid. (42 g, 307 [M+H]⁺ observed).

### (b) N-(4-Bromo-2-chlorobenzyl)-N-(2,2-dimethoxyethyl)benzenesulfonamide

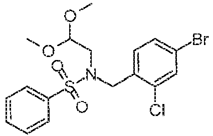

To a mixture of N-[(4-bromo-2-chloro-phenyl)methyl]-2,2-dimethoxy-ethanamine (46 g, 149 mmol) and triethylamine (37 mL, 268 mmol) in CH₂Cl₂ (350 mL) was added 4-dimethylaminopyridine (0.92 g, 7.45 mmol) and benzenesulfonyl chloride (31.6 g, 179 mmol). The mixture was stirred at rt for 16 h. The mixture was diluted with water (500 mL) and was extracted with ethyl acetate (2 x 250 mL). The combined organic phase was washed with brine (2 x 250 mL), dried with anhydrous sodium sulfate, filtered and concentrated. The residue was purified by flash chromatography (SiO₂, 0-100% ethyl acetate/petroleum ether) to afford N-[(4-bromo-2-chloro-phenyl)methyl] -N-(2,2-dimethoxyethyl)benzenesulfonamide as a white solid (57 g, 85% yield). ¹H NMR (400 MHz, CDCl₃): δ: 7.81-7.80 (m, 2H), 7.61-7.57 (m, 1H), 7.53-7.49 (m, 2H), 7.45 (m, 1H), 7.35 (s, 2H), 4.50 (s, 2H), 4.34 (t, *J* = 5.2 Hz, 1H), 3.29-3.21 (m, 7H).

### (c) 6-Bromo-8-chloroisoquinoline

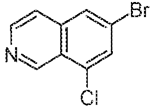

To a mixture of aluminum chloride (28 g, 212 mmol) in CH₂Cl₂ (150 mL) was added N-[(4-bromo-2-chloro-phenyl)methyl]-N-(2,2-dimethoxyethyl)benzenesulfonamide (19 g, 42.3 mmol) in CH₂Cl₂ (150 mL) in one portion at -5 °C. The mixture was stirred at rt for 2 h. The mixture was quenched with 1 N aqueous HCl solution (200 mL) at -10 °C and extracted with CH₂Cl₂ (400 mL). The organic phases were washed with brine (300 mL), dried over anhydrous sodium sulfate, filtered and concentrated in vacuo. The residue was purified by flash SiO₂ chromatography (0-15% ethyl acetate/petroleum ether) to afford 6-bromo-8-chloro-isoquinoline as a yellow solid (5.5 g, 18% yield, 243 [M+H]⁺ observed).

### (d) 6-Bromo-8-chloro-1,2,3,4-tetrahydroisoquinoline

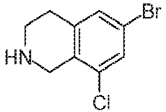

To a mixture of 6-bromo-8-chloro-isoquinoline (2 g, 8.25 mmol) in glacial acetic acid (50 mL) was added sodium cyanoborohydride (1.09 g, 28.9 mmol) in portions under N₂. The mixture was stirred at rt for 3 h. To the mixture was dropwise added saturated aqueous sodium bicarbonate solution (50 mL) and sodium carbonate solution (50 mL). The mixture was extracted with ethyl acetate (2 x 50 mL). The combined organic phase was washed with brine (2 x 25 mL), dried with anhydrous sodium sulfate, filtered and concentrated to afford 6-bromo-8-chloro-1,2,3,4-tetrahydroisoquinoline (1.5 g, crude, 60% yield). The material was used without any purification. ¹H NMR (400 MHz, DMSO-*d*₆): δ 7.47 (d, 1H), 7.32 (s, 1H), 3.75 (s, 2H), 2.90-2.83 (m, 2H), 2.71-2.69 (m, 2H).

### (e) 6-Bromo-8-chloro-2-(3-fluoropropyl)-1,2,3,4-tetrahydroisoquinoline

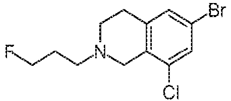

To a mixture of 6-bromo-8-chloro-1,2,3,4-tetrahydroisoquinoline (0.4 g, 1.62 mmol) and 1-bromo-3-fluoro-propane (0.46 g, 3.25 mmol) in acetonitrile (25 mL) was added potassium carbonate (0.67 g, 4.87 mmol). The mixture was stirred at 100 °C for 12 h. The mixture was filtered, and the filtrate was concentrated. The residue was purified by flash SiO₂ chromatography (0-50% EtOAc/petroleum ether) to afford 6-bromo-8-chloro-2- (3-fluoropropyl)-3,4-dihydro-1H-isoquinoline (0.26 g, 44% yield, 308 [M+H]⁺ observed).

### (f) 8-Chloro-2-(3-fluoropropyl)-6-(4,4,5,5-tetramethyl-1,3,2-dioxaborolan-2-yl)-1,2,3,4-tetrahydroisoquinoline

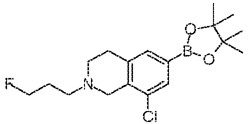

To a mixture of 6-bromo-8-chloro-2-(3-fluoropropyl)-3,4-dihydro-1H-isoquinoline (0.22 g, 0.72 mmol) and bis(pinacolato)diboron (0.55 g, 2.15 mmol) in dioxane (10 mL) was added potassium acetate (0.21 g, 2.15 mmol) and 1,1'-bis(diphenylphosphino) ferrocene-palladium(II) dichloride dichloromethane complex (0.06 g, 0.07 mmol). The mixture was stirred at 120 °C for 12 h. The mixture was concentrated, and the residue was purified by flash chromatography (SiO₂, 0-15% ethyl acetate/petroleum ether) to afford 8-chloro-2-(3-fluoropropyl)-6-(4,4,5,5-tetramethyl-1,3,2-dioxaborolan-2-yl)-3,4-dihydro-1H-isoquinoline as a yellow oil (0.1 g, 39% yield). ¹H NMR (400 MHz, CDCl₃): δ 7.53 (s, 1H), 7.39 (s, 1H), 4.55 (t, *J =* 1.6 Hz, 1H), 4.45-4.42 (m, 1H), 3.64-3.52 (m, 2H), 2.92-2.85 (m, 2H), 2.65 (m, 4H), 1.97-1.85 (m, 2H), 1.27 (s, 12H).

### (g) tert-Butyl N-[[6-[2-chloro-3-[3-chloro-2-[8-chloro-2-(3-fluoropropyl)-3,4-dihydro -1H-isoquinolin-6-yl]-4-pyridyl]phenyl]-2-methoxy-3-pyridyl]methyl]-N-[[(2S)-5-oxopyrrolidin-2-yl]methyl]carbamate

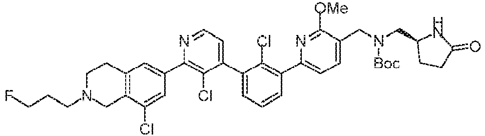

To a mixture of 8-chloro-2-(3-fluoropropyl)-6-(4,4,5,5-tetramethyl -1,3,2-dioxaborolan -2-yl)-3,4-dihydro-1H-isoquinoline (0.07 g, 0.2 mmol) and *tert-butyl* N-[[6-[2-chloro-3- (2,3-dichloro-4-pyridyl)phenyl]-2-methoxy-3-pyridyl]methyl]-N-[[(2S)-5-oxopyrrolidin-2-yl]methyl]carbamate (0.09 g, 0.15 mmol) in dioxane /H₂O mixture (10:1, 1.1 mL) was added potassium carbonate (0.06 g, 0.45 mmol) and 1,1'-bis(diphenylphosphino)ferrocene -palladium(II) dichloride dichloromethane complex (0.02 g, 0.02 mmol). The mixture was stirred at 110 °C for 5 h. The solvent was evaporated and the residue was purified by prep-TLC (silica gel, EtOAc: MeOH = 3:1) to afford *tert-butyl* N-[[6-[2-chloro-3-[3-chloro -2-[8-chloro-2-(3-fluoropropyl)-3,4 -dihydro-1H-isoquinolin-6-yl]-4-pyridyl]phenyl] -2-methoxy-3-pyridyl]methyl]-N-[[(2S)-5-oxopyrrolidin-2-yl]methyl]carbamate as a yellow oil (0.04 g, 23% yield, 781 [M+H]⁺ observed).

### (h) (S)-tert-Butyl ((6-(2-chloro-3-(3-chloro-2-(8-chloro-2-(3-fluoropropyl)-1,2,3,4 - tetrahydroisoquinolin-6-yl)pyridin-4-yl)phenyl)-2-methoxypyridin-3-yl)methyl)((5-oxopyrrolidin-2-yl)methyl)carbamate

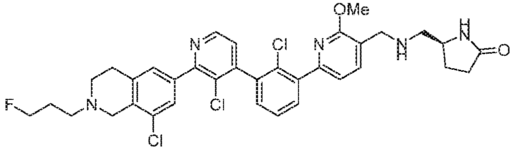

To a mixture of *tert*-butyl N-[[6-[2-chloro-3-[3-chloro-2-[8-chloro-2-(3-fluoropropyl) -3,4-dihydro-1H-isoquinolin-6-yl]-4-pyridyl]phenyl]-2-methoxy-3-pyridyl]methyl]-N-[[(2S)-5-oxopyrrolidin-2-yl]methyl]carbamate (0.06 g, 0.77 mmol) in CH₂Cl₂ (0.2 mL) was added trifluoroacetic acid (0.75 mL, 10 mmol). The mixture was stirred at rt for 0.5 h. The mixture was concentrated, and the residue was purified by reverse phase HPLC to afford (5S)-5-[[[6-[2-chloro-3-[3-chloro-2-[8-chloro-2-(3-fluoropropyl)-3,4-dihydro-1H-isoquinolin-6-yl]-4-pyridyl]phenyl]-2-methoxy-3-pyridyl]methylamino]methyl]pyrrolidin-2-one (2.5 mg, 5% yield, 682 [M+H]⁺ observed). ¹H NMR (400 MHz, Methanol-*d*₄): δ 8.63 (d, 1H), 7.83 (d, 1H), 7.71 (dd, 1H), 7.58-7.54 (m, 2H), 7.46-7.42 (m, 3H), 7.32 (d, 1H), 4.62 (t, 1H), 4.50 (t, 1H), 4.12-4.11 (m, 2H), 4.06 (s, 3H), 3.95 (m, 1H), 3.88 (s, 2H), 3.07-2.87 (m, 8H), 2.40-2.33 (m, 3H), 2.15-2.05 (m, 2H), 1.89-1.83 (m, 1H).

### Example 20: (S)-5-((((6-(2-Chloro-3-(3-chloro-2-(8-chloro-2-(((S)-5-oxopyrrolidin-2-yl)methyl)-1,2,3,4-tetrahydroisoquinolin-6-yl)pyridin-4-yl)phenyl)-2-methoxypyridin-3-yl)methyl)amino)methyl)pyrrolidin-2-one (67)

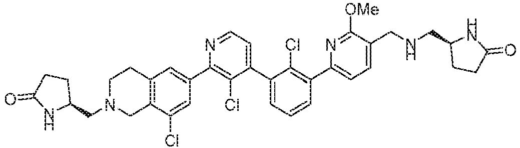

### (a) (S)-5-((6-Bromo-8-chloro-3,4-dihydroisoquinolin-2(1H)-yl)methyl)pyrrolidin-2-one

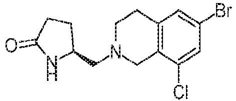

To a mixture of 6-bromo-8-chloro-1,2,3,4-tetrahydroisoquinoline (0.5 g, 2 mmol) and (5S)-5-(bromomethyl)pyrrolidin-2-one (0.47 g, 2.64 mmol) in MeCN (25 mL) was added potassium carbonate (0.84 g, 6.08 mmol). The mixture was stirred at 100 °C for 12 h. The mixture was filtered, and the filtrate was concentrated. The residue was purified by flash chromatography (SiO₂, 50-100% ethyl acetate/petroleum ether to 10% MeOH/ethyl acetate) to afford (S)-5-((6-bromo-8-chloro-3,4-dihydro isoquinolin-2(1H)-yl)methyl)pyrrolidin-2-one as a yellow solid (420 mg, 60% yield). ¹H NMR (400 MHz, DMSO) : δ 7.61 (s, 1H), 7.52 (d, 1H), 7.38 (s, 1H), 3.80-3.78 (m, 1H), 3.60-3.53 (m, 2H), 2.85-2.82 (m, 2H), 2.72-2.68 (m, 2H), 2.60-2.53 (m, 2H), 2.19-2.08 (m, 3H), 1.74-1.69 (m, 1H).

### (b) (S)-5-((8-Chloro-6-(4,4,5,5-tetramethyl-1,3,2-dioxaborolan-2-yl)-3,4-dihydroisoquinolin-2(1H)-yl)methyl)pyrrolidin-2-one

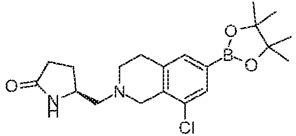

To a mixture of (S)-5-((6-bromo-8-chloro-3,4-dihydroisoquinolin-2(1H)-yl)methyl) pyrrolidin-2-one (0.37 mg, 1.08 mmol) and bis(pinacolato)diboron (0.82 g, 3.23 mmol) in dioxane (10 mL) was added potassium acetate (0.32 g, 3.23 mmol) and 1,1'-bis(diphenyl phosphino)ferrocene-palladium(II) dichloride dichloromethane complex (0.09 g, 0.11 mmol). The mixture was stirred at 120 °C for 12 h. The solvent was evaporated, and the residue was purified by flash chromatography (SiO₂, 50-100% ethyl acetate/petroleum ether) to afford (S)-5-((8-chloro-6-(4,4,5,5-tetramethyl -1,3,2- dioxaborolan-2-yl)-3,4-dihydroisoquinolin-2(1H)-yl)methyl)pyrrolidin-2-one as a yellow oil (0.28 g, 66% yield). ¹H NMR (400 MHz, Methanol-*d*₄): δ 7.38 (s, 1H), 7.33 (s, 1H), 3.90 (m, 1H), 3.69-3.58 (m, 2H), 2.83-2.79 (m, 4H), 2.61-2.52 (m, 2H), 2.26-2.20 (m, 3H), 1.79-1.74 (m, 1H), 1.23 (s, 12H).

### (c) tert-Butyl ((6-(2-chloro-3-(3-chloro-2-(8-chloro-2-(((S)-5-oxopyrrolidin-2-yl)methyl) -1,2,3,4-tetrahydroisoquinolin-6-yl)pyridin-4-yl)phenyl)-2-methoxypyridin-3-yl)methyl)(((S)-5-oxopyrrolidin-2-yl)methyl)carbamate

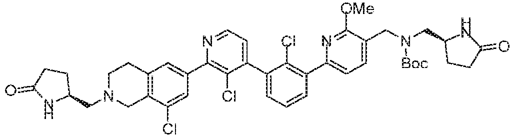

To a mixture of (S)-5-((8-chloro-6-(4,4,5,5-tetramethyl-1,3,2-dioxaborolan -2-yl)-3,4-dihydroisoquinolin-2(1H)-yl)methyl)pyrrolidin-2-one (0.24 g, 0.61 mmol) and (S)-tert-butyl ((6-(2-chloro-3-(2,3-dichloropyridin-4-yl)phenyl)-2-methoxypyridin-3-yl) methyl)((5-oxopyrrolidin-2-yl)methyl)carbamate (0.18 g, 0.3 mmol) in dioxane/H₂O mixture (25:2, 5.4 mL) was added 1,1'-bis(diphenylphosphino) ferrocene-palladium(II) dichloride dichloromethane complex (0.025 g, 0.03 mmol) and potassium carbonate (0.13 g, 0.91 mmol). The mixture was stirred at 110 °C for 3 h. The mixture was diluted with water (10 mL) and brine (10 mL). The mixture was extracted with ethyl acetate/THF mixture (1:1, 20 mL). The organic phase was dried with anhydrous sodium sulfate, filtered and concentrated. The residue was purified by flash chromatography (SiO₂, 50-100% ethyl acetate/petroleum ether to 10% MeOH/ethyl acetate) to afford *tert-butyl* ((6-(2-chloro-3-(3-chloro-2-(8-chloro-2-(((S) -5-oxopyrrolidin-2-yl)methyl)-1,2,3,4- tetrahydroisoquinolin-6-yl)pyridin-4-yl)phenyl)-2-methoxypyridin-3-yl)methyl)(((S)-5-oxopyrrolidin-2-yl)methyl)carbamate (0.16 g, 39% yield, m/z: 819 [M=H]⁺).

### (d) (S)-5-((((6-(2-Chloro-3-(3-chloro-2-(8-chloro-2-(((S)-5-oxopyrrolidin-2-yl)methyl)-1,2,3,4-tetrahydroisoquinolin-6-yl)pyridin-4-yl)phenyl)-2-methoxypyridin-3-yl)methyl)amino)methyl)pyrrolidin-2-one

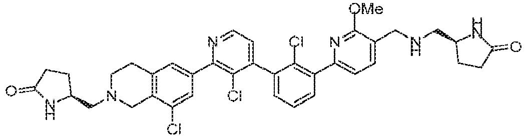

To a mixture of *tert*-butyl ((6-(2-chloro-3-(3-chloro-2-(8-chloro-2-(((S)-5-oxo pyrrolidin-2-yl)methyl)-1,2,3,4-tetrahydroisoquinolin-6-yl)pyridin-4-yl)phenyl)-2-methoxypyridin-3-yl)methyl)(((S)-5-oxopyrrolidin-2-yl)methyl)carbamate (0.15 g, 0.18 mmol) in CH₂Cl₂ (0.5 mL) was added trifluoroacetic acid (2 mL). The mixture was stirred at rt for 0.5 h. The mixture was concentrated. The residue was purified by reverse phase HPLC to afford (S)-5-((((6-(2-chloro-3-(3-chloro-2-(8-chloro-2-(((S)-5-oxopyrrolidin-2-yl)methyl)-1,2,3,4-tetrahydroisoquinolin-6-yl)pyridin-4-yl)phenyl)-2-methoxypyridin-3-yl)methyl)amino)methyl)pyrrolidin-2-one (9.3 mg, 6% yield, m/z: 719 [M+H]⁺ observed). ¹H NMR (400 MHz, DMSO-*d*₆): δ 8.72 (d, 1H), 7.84 (d, 1H), 7.74-7.46 (m, 7H), 7.28 (d, 1H), 3.92 (s, 3H), 3.84-3.81 (m, 1H), 3.72-3.62 (m, 5H), 2.93 (m, 2H), 2.78-2.73 (m, 2H), 2.61-2.56 (m, 4H), 2.23-2.08 (m, 6H), 1.76-1.66 (m, 2H).

### Example 21: (S)-5-((((6-(2-Chloro-3-(3-chloro-2-(8-chloro-2-((S)-2-hydroxypropyl)-1,2,3,4-tetrahydroisoquinolin-6-yl)pyridin-4-yl)phenyl)-2-methoxypyridin-3-yl)methyl)amino)methyl)pyrrolidin-2-one (69)

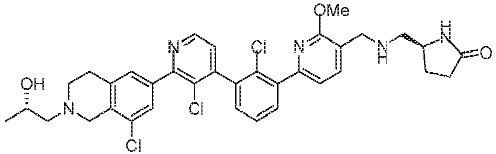

### (a) (S)-1-(6-Bromo-8-chloro-3,4-dihydroisoquinolin-2(1H)-yl)propan-2-ol:

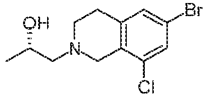

To a solution of 6-bromo-8-chloro-1,2,3,4-tetrahydroisoquinoline (400 mg, 1.62 mmol) and (2S)-2-methyloxirane (1.14 mL, 16.2 mmol) in EtOH (40 mL) was added triethylamine (0.67 mL, 4.87 mmol). The mixture was stirred at 50 °C for 3 h. The reaction mixture was concentrated under reduced pressure. The residue was purified by flash SiO₂ chromatography (10-20% EtOAc/petroleum ether) to give (S)-1-(6-bromo-8-chloro-3,4-dihydroisoquinolin-2(1H)-yl)propan-2-ol (349 mg, 71% yield) as a yellow oil. ¹H NMR (400 MHz, Methanol-*d*₄) δ 7.36 (s, 1 H), 7.20 (s, 1 H), 3.99-3.96 (m, 1 H), 3.76 (d1 H), 3.55 (d, 1 H), 2.93-2.88 (m, 3 H), 2.67-2.65 (m, 1 H), 2.57-2.47 (m, 2 H), 1.19 (d, 3 H).

### (b) (S)-1-(8-Chloro-6-(4,4,5,5-tetramethyl-1,3,2-dioxaborolan-2-yl)-3,4-dihydroisoquinolin-2(1H)-yl)propan-2-ol:

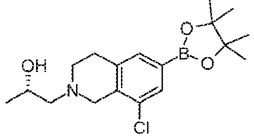

To a mixture of (S)-1-(6-bromo-8-chloro-3,4-dihydroisoquinolin-2(1H)-yl)propan-2-ol (279 mg, 0.92 mmol), 4,4,4',4',5,5,5',5'-octamethyl-2,2'-bi(1,3,2-dioxaborolane) (698 mg, 2.75 mmol) and potassium acetate (270 mg, 2.75 mmol) in dioxane (8 mL), 1,1'-bis(diphenyl phosphino)ferrocene-palladium(II) dichloride dichloromethane complex (74.8 mg, 0.09 mmol) was added. The mixture was stirred at 110 °C for 12 h under a nitrogen atmosphere. The mixture was concentrated. The residue was purified by flash chromatography to afford (SiO₂, 10-15% EtOAc/petroleum ether) to afford (S)-1-(8-chloro-6-(4,4,5,5-tetramethyl-1,3,2-dioxaborolan-2-yl)-3,4-dihydroisoquinolin-2(1H)-yl)propan-2-ol (180 mg, 56% yield, m/z: 352 [M+H]⁺).

### (c) tert-Butyl ((6-(2-chloro-3-(3-chloro-2-(8-chloro-2-((S)-2-hydroxypropyl)-1,2,3,4-tetrahydroisoquinolin-6-yl)pyridin-4-yl)phenyl)-2-methoxypyridin-3-yl)methyl)(((S)-5-oxopyrrolidin-2-yl)methyl)carbamate

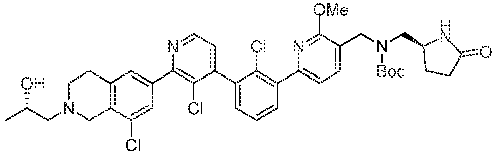

A mixture of (S)-1-(8-chloro-6-(4,4,5,5-tetramethyl-1,3,2-dioxaborolan-2-yl) -3,4-dihydroisoquinolin-2(1H)-yl)propan-2-ol (115 mg, 328 umol), (S)-*tert*-butyl ((6-(2-chloro-3-(2,3-dichloropyridin-4-yl)phenyl)-2-methoxypyridin-3-yl)methyl)((5-oxopyrrolidin-2-yl)methyl)carbamate (97 mg, 164 umol), potassium carbonate (68.0 mg, 492 umol) in H₂O (0.3 mL) and dioxane (3 mL) was degassed. To this mixture was added 1,1'-bis(diphenylphosphino)ferrocene-palladium(II) dichloride dichloromethane complex (13.4 mg, 16.4 umol). The mixture was stirred at 110 °C for 12 h under a nitrogen atmosphere. The mixture was concentrated and the residue was purified by prep-TLC (SiO₂, 10% MeOH/methylene chloride) to afford *tert-butyl* ((6-(2-chloro-3-(3-chloro-2-(8-chloro-2-((S)-2-hydroxypropyl)-1,2,3,4-tetrahydroisoquinolin-6-yl)pyridin-4-yl)phenyl)-2-methoxypyridin-3-yl)methyl)(((S)-5-oxopyrrolidin-2-yl)methyl)carbamate (60 mg, 44% yield, m/z: 780 [M+H]⁺ observed).

### (d) (S)-5-((((6-(2-Chloro-3-(3-chloro-2-(8-chloro-2-((S)-2-hydroxypropyl)-1,2,3,4-tetrahydroisoquinolin-6-yl)pyridin-4-yl)phenyl)-2-methoxypyridin-3-yl)methyl)amino)methyl)pyrrolidin-2-one

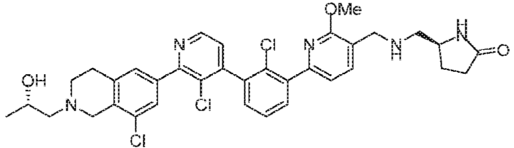

To a solution of *tert*-butyl((6-(2-chloro-3-(3-chloro-2-(8-chloro-2-((S) -2-hydroxypropyl)-1,2,3,4-tetrahydroisoquinolin-6-yl)pyridin-4-yl)phenyl)-2-methoxypyridin-3-yl)methyl)(((S)-5-oxopyrrolidin-2-yl)methyl)carbamate (90 mg, 0.12 mmol) in methylene chloride (10 mL) was added trifluoroacetic acid (6.5 mL, 87.8 mmol). The mixture was stirred at 25 °C for 30 min. The mixture was concentrated and the residue was purified by reverse phase HPLC to give (S)-5-((((6-(2-chloro-3-(3-chloro-2- (8-chloro-2-((S)-2-hydroxypropyl)-1,2,3,4-tetrahydroisoquinolin-6-yl)pyridin-4-yl)phenyl)-2-methoxypyridin-3-yl)methyl)amino)methyl)pyrrolidin-2-one (11.6 mg, 14% yield, m/z: 680 [M+H]⁺) as a white solid. ¹H NMR (400 MHz, DMSO-*d*₆) δ 8.71 (d, 1 H), 7.89 (d, 1 H), 7.74-7.69 (m, , 2 H), 7.63-7.50 (m, 4 H), 7.46 (s, 1 H), 7.32 (d, 1 H), 3.95-3.87 (m, 6 H), 3.72 (s, 3 H), 2.94-2.92 (m, 2 H), 2.80-2.67 (m, 4 H), 2.59-2.56 (m, 2 H), 2.18-2.09 (m, 3 H), 1.76-1.72 (m, 1 H), 1.10 (d, 3 H).

### Example 22: (S)-5-((((4-Chloro-6-(2-chloro-3-(3-chloro-2-(2-((S)-2-hydroxypropyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one (86)

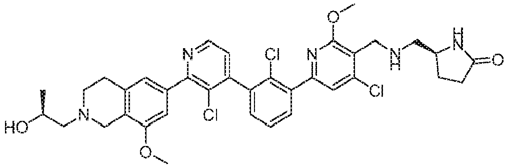

### (a) (S)-1-(6-Bromo-8-methoxy-3,4-dihydroisoquinolin-2(1H)-yl)propan-2-ol

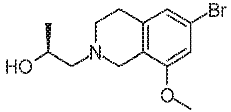

To a mixture of 6-bromo-8-methoxy-1,2,3,4-tetrahydroisoquinoline, HCl (0.25 g, 0.9 mmol) in EtOH (4 mL) was added N-ethyl-N-isopropylpropan-2-amine (0.94 mL, 5.38 mmol) and (2S)-2-methyloxirane (0.38 mL, 5.38 mmol). The mixture was stirred at rt for 12 h. The mixture was concentrated to afford (S)-1-(6-bromo-8-methoxy-3,4-dihydroisoquinolin-2(1H)-yl)propan-2-ol (0.35 g, crude, m/z: 300 [M+H]⁺).

### (b) (S)-1-(8-Methoxy-6-(4,4,5,5-tetramethyl-1,3,2-dioxaborolan-2-yl)-3,4-dihydroisoquinolin-2(1H)-yl)propan-2-ol

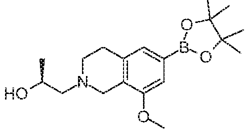

To a mixture of (S)-1-(6-bromo-8-methoxy-3,4-dihydroisoquinolin-2(1H)-yl)propan-2-ol (0.3 g, 1 mmol) and bis(pinacolato)diboron (0.63 g, 2.5 mmol) in dioxane (6 mL) was added potassium acetate (0.2 g, 2 mmol) and 1,1'-bis(diphenylphosphino)ferrocene-palladium(II) dichloride dichloromethane complex (0.04 g, 0.05 mmol). The mixture was stirred at 115 °C for 3 h. The mixture was concentrated, and the residue was purified by flash chromatography (SiO₂, 0-15% ethyl acetate/petroleum ether to 20% MeOH/ethyl acetate) to afford (S)-1-(8-methoxy-6-(4,4,5,5-tetramethyl-1,3,2- dioxaborolan-2-yl)-3,4-dihydroisoquinolin-2(1H)-yl)propan-2-ol (0.17 g, 45% yield, m/z: 348 [M+H]⁺ observed). ¹H NMR (400 MHz, Methanol-*d*₄): δ 7.23 (s, 1H), 7.17 (s, 1H), 4.26-4.20 (m, 1H), 4.18-4.10 (m, 2H), 3.89 (s, 3H), 3.30-3.23 (m, 2H), 3.10-3.07 (m, 2H), 3.03-2.95 (m, 2H), 1.37 (s, 12H), 1.27-1.25 (m, 3H).

### (c) tert-Butyl ((4-chloro-6-(2-chloro-3-(3-chloro-2-(2-((S)-2-hydroxypropyl) -8-methoxy -1,2,3,4-tetrahydroisoquinolin-6-yl)pyridin-4-yl)phenyl)-2-methoxypyridin-3-yl)methyl)(((S)-5-oxopyrrolidin-2-yl)methyl)carbamate

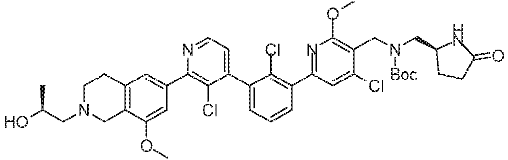

To a mixture of (S)-*tert*-butyl ((4-chloro-6-(2-chloro-3-(2,3-dichloropyridin-4-yl) phenyl)-2-methoxypyridin-3-yl)methyl)((5-oxopyrrolidin-2-yl)methyl)carbamate (0.17 g, 0.27 mmol) and (S)-1-(8-methoxy-6-(4,4,5,5-tetramethyl-1,3,2-dioxaborolane-2-yl) -3,4-dihydroisoquinolin-2(1H)-yl)propan-2-ol (0.095 g, 0.27 mmol) in dioxane/H₂O mixture (8:1, 4.5 mL) was added potassium carbonate (0.11 g, 0.82 mmol) and 1,1'-bis(diphenylphosphino)ferrocene-palladium(II) dichloride dichloromethane complex (0.022 g, 0.03 mmol). The mixture was stirred at 110 °C for 5 h. The mixture was diluted with water (5 mL) and brine (5 mL). The mixture was extracted with ethyl acetate/THF mixture (1:1, 10 mL). The organic phase was dried with anhydrous sodium sulfate, filtered and concentrated. The residue was purified by prep-TLC (silica gel, ethyl acetate: MeOH = 2:1) to afford *tert-butyl* ((4-chloro-6-(2-chloro-3-(3-chloro-2-(2-((S)-2-hydroxypropyl) -8-methoxy-1,2,3,4-tetrahydroisoquinolin-6-yl)pyridin-4-yl)phenyl)-2-methoxypyridin-3-yl)methyl)(((S)-5-oxopyrrolidin-2-yl)methyl)carbamate as a yellow solid (0.13 g, 29% yield, m/z: 810 [M+H]⁺).

### (d) (S)-5-((((4-Chloro-6-(2-chloro-3-(3-chloro-2-(2-((S)-2-hydroxypropyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one

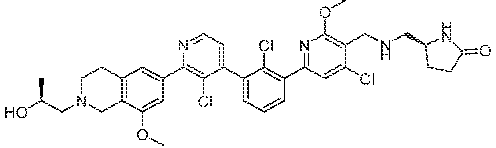

To a mixture of *tert*-butyl ((4-chloro-6-(2-chloro-3-(3-chloro-2-(2-((S)-2-hydroxypropyl) -8-methoxy-1,2,3,4-tetrahydroisoquinolin-6-yl)pyridin-4-yl)phenyl)-2-methoxypyridin-3-yl)methyl)(((S)-5-oxopyrrolidin-2-yl)methyl)carbamate (0.13 g, 0.15 mmol) in CH₂Cl₂ (0.3 mL) was added trifluoroacetic acid (1.5 mL). The mixture was stirred at rt for 10 min. The mixture was concentrated and the residue was purified by reverse phase HPLC to afford (S)-5-((((4-chloro-6-(2-chloro-3-(3-chloro-2-(2-((S)-2-hydroxypropyl)-8-methoxy-1,2,3,4-tetrahydroisoquinolin-6-yl)pyridin-4-yl)phenyl)-2-methoxypyridin-3-yl)methyl)amino) methyl)pyrrolidin-2-one (26.4 mg, 22% yield, m/z: 710 [M+H]⁺ observed). ¹H NMR (400 MHz, DMSO): δ 8.70 (d, 1H), 7.75 (dd, 1H), 7.65-7.59 (m, 2H), 7.54-7.53 (m, 1H), 7.49 (d, 1H), 7.40 (s, 1H), 7.06 (s, 2H), 3.96 (s, 3H), 3.92-3.87 (m, 3H), 3.82 (s, 3H), 3.66-3.57 (m, 3H), 2.87-2.86 (m, 2H), 2.78-2.77 (m, 2H), 2.59-2.55 (m, 2H), 2.45-2.43 (m, 2H), 2.16-2.08 (m, 3H), 1.72-1.64 (m, 1H), 1.10 (d, 3H).

### Example 23: (S)-5-((((4-Chloro-6-(2-chloro-3-(3-chloro-2-(2-((R)-2-hydroxypropyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one (104)

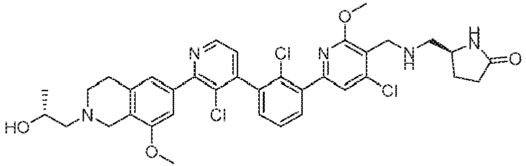

### (a) (R)-1-(6-Bromo-8-methoxy-3,4-dihydroisoquinolin-2(1H)-yl)propan-2-ol

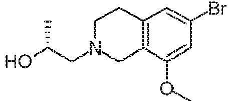

To a mixture of 6-bromo-8-methoxy-1,2,3,4-tetrahydroisoquinoline, HCl (0.5 g, 1.8 mmol) in EtOH (6 mL) was added N-ethyl-N-isopropylpropan-2-amine (1.9 mL, 10.8 mmol) and (2R)-2-methyloxirane (1 mL, 14.4 mmol). The mixture was stirred at room temperature for 12 h. The mixture was concentrated to afford (R)-1-(6-bromo-8-methoxy-3,4-dihydroisoquinolin -2(1H)-yl)propan-2-ol (0.6 g, crude, m/z: 300 [M+H]⁺).

### (b) (R)-1-(8-Methoxy-6-(4,4,5,5-tetramethyl-1,3,2-dioxaborolan-2-yl)-3,4-dihydroisoquinolin-2(1H)-yl)propan-2-ol

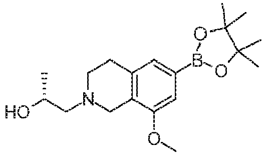

To a mixture of (R)-1-(6-bromo-8-methoxy-3,4-dihydroisoquinolin-2(1H)-yl)propan-2-ol (0.6 g, 2 mmol) and bis(pinacolato)diboron (1.02 g, 4 mmol) in dioxane (15 mL) was added 1,1'-bis(diphenylphosphino)ferrocene-palladium(II) dichloride dichloromethane complex (0.065 g, 0.08 mmol) and potassium acetate (0.39 g, 4 mmol). The mixture was stirred at 115 °C for 12 h. The mixture was concentrated, and the residue was purified by flash chromatography (SiO₂, 0-100% 30% THF in ethyl acetate/petroleum ether to 5% MeOH/ethyl acetate) to afford (R)-1-(8-methoxy-6-(4,4,5,5-tetramethyl-1,3,2- dioxaborolan-2-yl)-3,4-dihydroisoquinolin-2(1H)-yl)propan-2-ol (0.45 g, 55% yield). ¹H NMR (400 MHz, Methanol-*d*₄): δ 7.20 (s, 1H), 7.14 (s, 1H), 4.17-4.12 (m, 1H), 3.87 (s, 3H), 3.24-2.90 (m, 6H), 2.82-2.80 (m, 2H), 1.36 (s, 12H), 1.24-1.22 (m, 3H).

### (c) tert-Butyl ((4-chloro-6-(2-chloro-3-(3-chloro-2-(2-((R)-2-hydroxypropyl)-8-methoxy -1,2,3,4-tetrahydroisoquinolin-6-yl)pyridin-4-yl)phenyl)-2-methoxypyridin-3-yl)methyl)(((S)-5-oxopyrrolidin-2-yl)methyl)carbamate

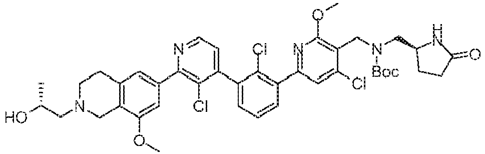

To a mixture of (R)-1-(8-methoxy-6-(4,4,5,5-tetramethyl-1,3,2-dioxaborolan-2-yl) - 3,4-dihydroisoquinolin-2(1H)-yl)propan-2-ol (0.16 g, 0.46 mmol) and (S)-tert-butyl ((4-chloro-6-(2-chloro-3-(2,3-dichloropyridin-4-yl)phenyl)-2-methoxypyridin-3-yl)methyl)((5-oxopyrrolidin-2-yl)methyl)carbamate (0.13 g, 0.21 mmol) in dioxane/H₂O mixture (10:1, 3.3 mL) was added 1,1'-bis(diphenylphosphino)ferrocene-palladium(II) dichloride dichloromethane complex (0.017 g, 0.021 mmol) and potassium carbonate (0.086 g, 0.62 mmol). The mixture was stirred at 110 °C for 9 h. The mixture was diluted with water (5 mL) and brine (5 mL). The mixture was extracted with ethyl acetate/THF mixture (1:1, 10 mL). The organic phase was dried with anhydrous sodium sulfate, filtered and concentrated. The residue was purified by prep-TLC (silica gel, ethyl acetate: MeOH = 2:1) to afford *tert-butyl* ((4-chloro-6-(2-chloro-3-(3-chloro-2-(2-((R)-2-hydroxypropyl) -8-methoxy-1,2,3,4-tetrahydroisoquinolin-6-yl)pyridin-4-yl)phenyl)-2-methoxypyridin-3-yl)methyl)(((S)-5-oxopyrrolidin-2-yl)methyl)carbamate as a brown solid (0.06 g, 29% yield, m/z: 810 [M+H]⁺).

### (d) (S)-5-((((4-Chloro-6-(2-chloro-3-(3-chloro-2-(2-((R)-2-hydroxypropyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one

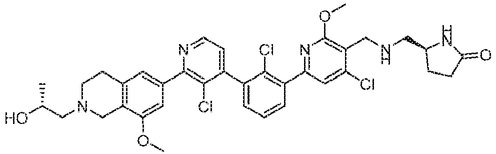

To a mixture of *tert-butyl* ((4-chloro-6-(2-chloro-3-(3-chloro-2-(2-((R)-2-hydroxypropyl)- 8-methoxy-1,2,3,4-tetrahydroisoquinolin-6-yl)pyridin-4-yl)phenyl)-2-methoxypyridin-3-yl)methyl)(((S)-5-oxopyrrolidin-2-yl)methyl)carbamate (0.055 g, 0.068 mmol) in CH₂Cl₂ (0.3 mL) was added trifluoroacetic acid (1 mL). The mixture was stirred at rt for 10 min. The mixture was concentrated, and the residue was purified by reverse phase HPLC to afford (S)-5-((((4-chloro-6-(2-chloro-3-(3-chloro-2-(2-((R)-2-hydroxypropyl)-8-methoxy-1,2,3,4-tetrahydroisoquinolin-6-yl)pyridin-4-yl)phenyl)-2-methoxypyridin-3-yl)methyl)amino) methyl)pyrrolidin-2-one (14.3 mg, 26% yield, m/z: 710 [M+H]⁺). ¹H NMR (400 MHz, DMSO-*d*₆): δ 8.70 (d, 1H), 7.76-7.74 (m, 1H), 7.67 (m, 1H), 7.63-7.59 (m, 1H), 7.54-7.53 (m, 1H), 7.49 (d, 1H), 7.40 (s, 1H), 7.05 (s, 2H), 3.96 (s, 3H), 3.86-3.82 (m, 6H), 3.58-3.52 (m, 3H), 2.86 (m, 2H), 2.72-2.67 (m, 4H), 2.45-2.33 (m, 2H), 2.09 (m, 3H), 1.67 (m, 1H), 1.09 (d, 3H).

### Example 24: (S)-5-((((4-Chloro-6-(2-chloro-3-(3-chloro-2-(2-(2-hydroxyethyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one (124)

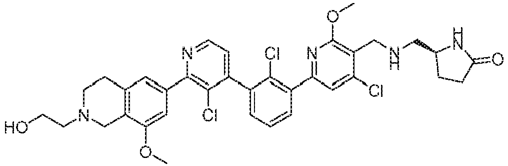

### (a) 6-Bromo-2-(2-((tert-butyldimethylsilyl)oxy)ethyl)-8-methoxy-1,2,3,4-tetrahydroisoquinoline

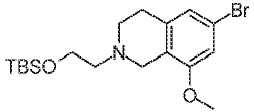

To a mixture of 6-bromo-8-methoxy-1,2,3,4-tetrahydroisoquinoline (0.4 g, 1.65 mmol) and 2-bromoethoxy-tert-butyl-dimethyl-silane (0.59 g, 2.48 mmol) in MeCN (8 mL) was added triethylamine (1.4 mL, 9.91 mmol). The mixture was stirred at 100 °C for 12 h. The mixture was concentrated, and the residue was added water (5 mL) and brine (5 mL). The mixture was extracted with ethyl acetate/THF mixture (1:1, 20 mL). The organic phase was dried with anhydrous sodium sulfate, filtered and concentrated. The residue was purified by flash chromatography (SiO₂, 0-30% ethyl acetate/petroleum ether) to afford 6-bromo-2-(2-((tert-butyldimethylsilyl)oxy)ethyl)- 8-methoxy -1,2,3,4-tetrahydroisoquinoline (0.45 g, 62% yield). ¹H NMR (400 MHz, CDCl₃): δ 6.80 (s, 1H), 6.70 (s, 1H), 3.79-3.76 (m, 2H), 3.71 (s, 3H), 3.50 (m, 2H), 2.76-2.65 (m, 6H), 0.84 (s, 9H), 0.00 (s, 6H).

### (b) 2-(2-((tert-Butyldimethylsilyl)oxy)ethyl)-8-methoxy-6-(4,4,5,5-tetramethyl-1,3,2-dioxaborolan-2-yl)-1,2,3,4-tetrahydroisoquinoline

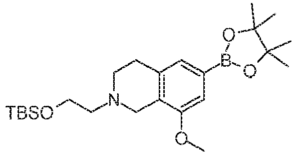

To a mixture of 6-bromo-2-(2-((*tert*-butyldimethylsilyl)oxy)ethyl)-8- methoxy-1,2,3,4-tetrahydroisoquinoline (0.42 g, 1.05 mmol) and bis(pinacolato)diboron (0.45 g, 1.78 mmol) in dioxane (5 mL) was added potassium acetate (0.21 g, 2.1 mmol) and 1,1'-bis(diphenylphosphino)ferrocene-palladium(II) dichloride dichloromethane complex (0.04 g, 0.05 mmol). The mixture was stirred at 110 °C for 12 h. The residue was purified by flash SiO₂ chromatography (0-30% 30% THF in ethyl acetate/petroleum ether) to afford 2-(2-((tert-butyldimethylsilyl)oxy)ethyl)-8-methoxy-6-(4,4,5,5-tetramethyl -1,3,2-dioxaborolan-2-yl)-1,2,3,4-tetrahydroisoquinoline (0.4 g, 78% yield). ¹H NMR (400 MHz, CDCl₃): δ 7.13 (s, 1H), 6.98 (s, 1H), 3.85-3.71 (m, 5H), 3.67-3.58 (m, 2H), 2.83 (m, 2H), 2.75-2.70 (m, 4H), 1.27 (s, 12H), 0.83 (s, 9H), 0.00 (s, 6H).

### (c) (S)-tert-Butyl ((6-(3-(2-(2-(2-((tert-butyldimethylsilyl)oxy)ethyl)-8-methoxy-1,2,3,4- tetrahydroisoquinolin-6-yl)-3-chloropyridin-4-yl)-2-chlorophenyl)-4-chloro-2-methoxypyridin-3-yl)methyl)((5-oxopyrrolidin-2-yl)methyl)carbamate

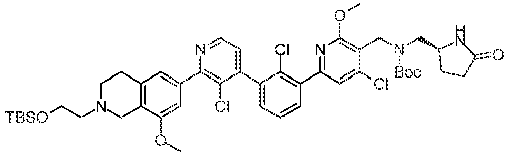

To a mixture of 2-(2-((*tert*-butyldimethylsilyl)oxy)ethyl)-8-methoxy-6-(4,4,5,5-tetramethyl -1,3,2-dioxaborolan-2-yl)-1,2,3,4-tetrahydroisoquinoline (0.21 g, 0.46 mmol) and (S)-tert-butyl ((4-chloro-6-(2-chloro-3-(2,3-dichloropyridin-4-yl)phenyl) -2-methoxypyridin-3-yl)methyl)((5-oxopyrrolidin-2-yl)methyl)carbamate (0.16 g, 0.26 mmol) in dioxane/H₂O mixture (10:1, 4.4 mL) was added 1,1'-bis(diphenylphosphino) ferrocene-palladium(II) dichloride dichloromethane complex (0.01 g, 0.015 mmol) and potassium carbonate (0.11 g, 0.77 mmol). The mixture was stirred at 110 °C for 8 h. The mixture was diluted with water (5 mL) and brine (5 mL). The mixture was extracted with ethyl acetate/THF mixture (1:1, 10 mL). The organic phase was dried with anhydrous sodium sulfate, filtered and concentrated. The residue was purified by prep-TLC (silica gel, ethyl acetate: MeOH =5:1) to afford (S)-*tert*-butyl ((6-(3-(2-(2-(2-((*tert*-butyldimethylsilyl) oxy)ethyl)-8-methoxy-1,2,3,4-tetrahydroisoquinolin-6-yl)-3-chloropyridin-4-yl)-2-chlorophenyl)-4-chloro-2-methoxypyridin-3-yl)methyl)((5-oxopyrrolidin-2-yl)methyl)carbamate as a yellow solid (0.1 g, 23% yield, m/z: 912 [M+H]⁺ observed).

### (d) (S)-5-((((4-Chloro-6-(2-chloro-3-(3-chloro-2-(2-(2-hydroxyethyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one

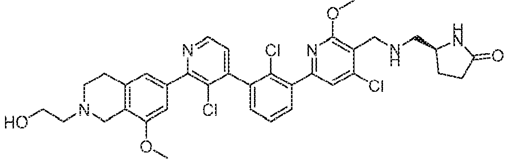

To a mixture of (S)-*tert*-butyl ((6-(3-(2-(2-(2-((*tert*-butyldimethylsilyl)oxy)ethyl) -8-methoxy-1,2,3,4-tetrahydroisoquinolin-6-yl)-3-chloropyridin-4-yl)-2-chlorophenyl)-4-chloro-2-methoxypyridin-3-yl)methyl)((5-oxopyrrolidin-2-yl)methyl)carbamate (0.095 g, 0.1 mmol) in CH₂Cl₂ (0.5 mL) was added trifluoroacetic acid (2.5 mL). The mixture was stirred at rt for 8 h. The mixture was concentrated, and the residue was purified by reverse phase HPLC to afford (S)-5-((((4-chloro-6-(2-chloro-3-(3-chloro-2-(2-(2-hydroxyethyl) -8-methoxy-1,2,3,4-tetrahydroisoquinolin-6-yl)pyridin-4-yl)phenyl)-2-methoxypyridin-3-yl)methyl)amino)methyl)pyrrolidin-2-one (28.1 mg, 35% yield, m/z: 696 [M+H]⁺ observed). ¹H NMR (400 MHz, DMSO-*d*₆): δ 8.72 (d, 1H), 7.76 (dd, 1H), 7.65-7.61 (m, 2H), 7.56-7.52 (m, 2H), 7.44 (s, 1H), 7.13 (d, 2H), 4.15-3.92 (m, 7H), 3.86 (s, 3H), 3.76-3.68 (m, 3H), 3.30-3.20 (m, 2H), 3.09-3.04 (m, 2H), 2.68-2.67 (m, 2H), 2.53-2.52 (m, 2H), 2.18-2.09 (m, 3H), 1.75-1.67 (m, 1H).

### Example 25: (S)-5-((((6-(2-Chloro-3-(3-chloro-2-(2-((S)-2-hydroxypropyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one (50)

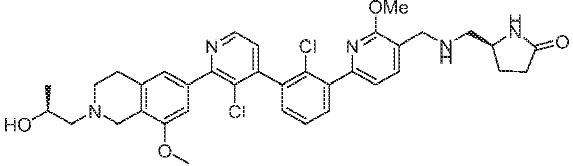

### (a) (S)-1-(6-Bromo-8-methoxy-3,4-dihydroisoquinolin-2(1H)-yl)propan-2-ol

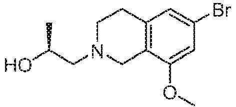

To a solution of 6-bromo-8-methoxy-1,2,3,4-tetrahydroisoquinoline hydrochloride salt (0.15 g, 0.54 mmol) and (S)-2-methyloxirane (0.63 g, 10.77 mmol) in ethanol (7 mL) was added N,N-diisopropylethylamine (0.19 mL, 1.08 mmol). The mixture was stirred at 85 °C for 1 h, then concentrated under reduced pressure. The residue was purified by flash chromatography (SiO₂, eluting with a linear gradient of 0-5% MeOH/methylene chloride) to afford (S)-1-(6-bromo-8-methoxy-3,4-dihydroisoquinolin-2(1H)-yl)propan-2-ol (0.094 g, 58%). LCMS: m/z found 300 [M+H]⁺, RT = 0.59 min (Method B).

### (b) (S)-1-(8-Methoxy-6-(4,4,5,5-tetramethyl-1,3,2-dioxaborolan-2-yl)-3,4-dihydroisoquinolin-2(1H)-yl)propan-2-ol

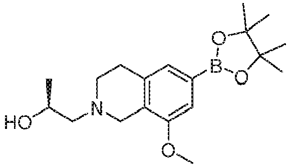

A pressure vessel was charged with (S)-1-(6-bromo-8-methoxy-3,4-dihydroisoquinolin-2(1H)-yl)propan-2-ol (0.10 g, 0.32 mmol), bis(pinacolato)diboron (0.09 g, 0.35 mmol), potassium acetate (0.09g, 0.89 mmol), and Pd(dppf)₂Cl₂-CH₂Cl₂ (0.03 g, 0.03 mmol). The vessel was purged with nitrogen for 5 min, then 7 mL of dry dioxane was added, and the mixture was sparged with nitrogen for 5 min. The mixture was stirred at 90 °C for 1 h and allowed to cool to room temperature. The mixture was filtered through CELITE^{®} and the filter cake was washed with ethyl acetate (3 x 20 mL). The filtrate was concentrated under reduced pressure and the residue was purified by flash chromatography (SiO₂, eluting with a linear gradient of 0-10% methano/methylene chloride) to provide (S)-1-(8-methoxy-6-(4,4,5,5-tetramethyl-1,3,2-dioxaborolan-2-yl)-3,4-dihydroisoquinolin-2(1H)-yl)propan-2-ol (0.08 g, 73% yield). LCMS: m/z found 348 [M+H]⁺, RT = 0.70 min (Method B).

### (c) tert-Butyl ((6-(2-chloro-3-(3-chloro-2-(2-((S)-2-hydroxypropyl)-8-methoxy-1,2,3,4-tetrahydroisoquinolin-6-yl)pyridin-4-yl)phenyl)-2-methoxypyridin-3-yl)methyl)(((S)-5-oxopyrrolidin-2-yl)methyl)carbamate

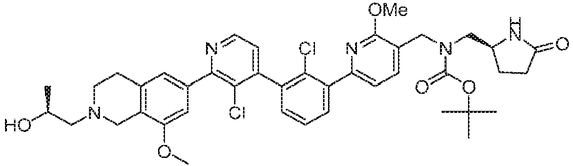

A pressure vessel was charged with (S)-1-(8-methoxy-6-(4,4,5,5-tetramethyl-1,3,2-dioxaborolan-2-yl)-3,4-dihydroisoquinolin-2(1H)-yl)propan-2-ol (0.08 g, 0.23 mmol), *tert-*butyl (S)-((6-(2-chloro-3-(2,3-dichloropyridin-4-yl)phenyl)-2-methoxypyridin-3-yl)methyl)((5-oxopyrrolidin-2-yl)methyl)carbamate (0.14 g, 0.23 mmol), cesium carbonate (0.23 g 0.69 mmol), and Pd(PPh₃)₄ (0.03 g, 0.02 mmol) and the vessel was flushed with nitrogen. Dioxane was added (6 mL), and the mixture was sparged with nitrogen, then 0.75 mL of water was added. The mixture was stirred at 90 °C for 1 hr. The mixture was allowed to cool to room temperature, filtered through CELITE^{®} and the filter cake was washed with ethyl acetate (3 x 15 mL). The filtrate was concentrated under reduced pressure and the residue was purified by flash chromatography (SiO₂, eluting with a linear gradient of 0-5% methanol/methylene chloride) to provide *tert*-butyl ((6-(2-chloro-3-(3-chloro-2-(2-((S)-2-hydroxypropyl)-8-methoxy-1,2,3,4-tetrahydroisoquinolin-6-yl)pyridin-4-yl)phenyl)-2-methoxypyridin-3-yl)methyl)(((S)-5-oxopyrrolidin-2-yl)methyl)carbamate (0.09 g, 50% yield). LCMS: m/z found 776 [M+H]⁺, RT = 0.83 min (Method B).

### (d) (S)-5-((((6-(2-Chloro-3-(3-chloro-2-(2-((S)-2-hydroxypropyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one

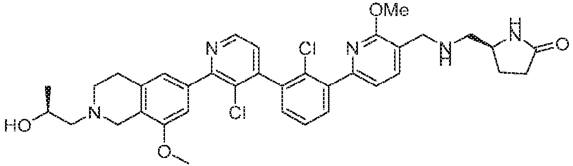

A scintillation vial was charged with *tert-butyl* ((6-(2-chloro-3-(3-chloro-2-(2-((S)-2-hydroxypropyl)-8-methoxy-1,2,3,4-tetrahydroisoquinolin-6-yl)pyridin-4-yl)phenyl)-2-methoxypyridin-3-yl)methyl)(((S)-5-oxopyrrolidin-2-yl)methyl)carbamate (0.10 g, 0.12 mmol), and 5 ml of 1:1 (v/v) TFA/methylene chloride was added, and the mixture was stirred at room temp for 10 min. The mixture was then concentrated, and the residue was purified by semi-preparative HPLC to provide 59 mg (66%) of (S)-5-((((6-(2-chloro-3-(3-chloro-2-(2-((S)-2-hydroxypropyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one bis formic acid salt. LCMS: m/z found 676 [M+H]⁺, RT = 1.68 min (Method D). ¹H NMR (400 MHz, Methanol-*d*₄) δ 8.61 (d, 1H), 7.77 (d, 1H), 7.68 (d, 1H), 7.53 (t, 1H), 7.46 - 7.35 (m, 2H), 7.26 (d, 1H), 7.14 (s, 2H), 4.32 (s, 2H), 4.25 (m, 2H), 4.04 - 3.95 (m, 5H), 3.89 (d, 2H), 3.49 (s, 3H), 3.21 - 3.04 (m, 4H), 2.93 - 2.79 (m, 2H), 2.40 - 2.24 (m, 3H), 1.87 - 1.75 (m, 1H), 1.24 (d, 3H).

### Example 26: (S)-5-((((6-(2-Chloro-3-(3-chloro-2-(2-(3-hydroxy-3-methylbutyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one (49)

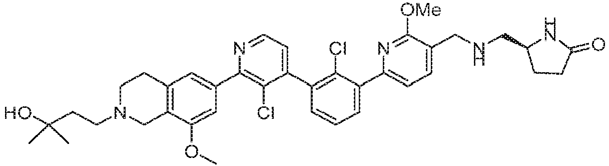

(S)-5-((((6-(2-Chloro-3-(3-chloro-2-(2-(3-hydroxy-3-methylbutyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one was prepared in a similar fashion to compound 50, from 4-bromo-2-methylbutan-2-ol and 6-bromo-8-methoxy-1,2,3,4-tetrahydroisoquinoline hydrochloride salt. LCMS: m/z found 704 [M+H]⁺, RT = 1.77 min (method D). ¹H NMR (400 MHz, Methanol-*d*₄) δ 8.60 (d, 1H), 7.77 (d, 1H), 7.68 (d, 1H), 7.53 (t, 1H), 7.45 - 7.35 (m, 2H), 7.26 (d, 1H), 7.13 (s, 2H), 4.24 (s, 2H), 4.04 - 3.95 (m, 5H), 3.90 (s, 3H), 3.42 (s, 2H), 3.35 - 3.24 (m, 3H), 3.15 (t, 2H), 2.93 - 2.78 (m, 2H), 2.40 - 2.22 (m, 3H), 1.97 - 1.88 (m, 2H), 1.89 - 1.75 (m, 1H), 1.27 (s, 6H).

### Example 27: (S)-5-((((6-(2-Chloro-3-(3-chloro-2-(2-(2-(1-hydroxycyclobutyl)ethyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one (51)

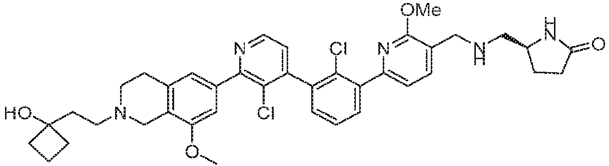

(S)-5-((((6-(2-Chloro-3-(3-chloro-2-(2-(2-(1-hydroxycyclobutyl)ethyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one was prepared in a similar fashion to compound 50, from 1-(2-bromoethyl)cyclobutan-1-ol and 6-bromo-8-methoxy-1,2,3,4-tetrahydroisoquinoline hydrochloride salt. LCMS: m/z found 716 [M+H]⁺, RT = 1.84 min (Method D). ¹H NMR (400 MHz, Methanol-*d*₄) δ 8.60 (d, 1H), 7.78 (dd, 1H), 7.68 (s, 1H), 7.53 (d, 1H), 7.45 - 7.35 (m, 2H), 7.27 (d, 1H), 7.14 (s, 2H), 4.28 (s, 2H), 4.07 - 3.93 (m, 5H), 3.90 (s, 4H), 3.45 (t, 2H), 3.35 - 3.25 (m, 2H), 3.16 (t, 2H), 2.96 - 2.82 (m, 2H), 2.41 - 2.23 (m, 3H), 2.15 - 2.04 (m, 7H), 1.90 - 1.69 (m, 1H), 1.67 - 1.52 (m, 1H).

### Example 28: (5S)-5-((((6-(2-Chloro-3-(3-chloro-2-(2-(2-hydroxy-3-methylbutyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one (53)

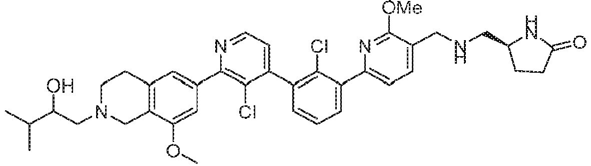

(5 S)-5 -((((6-(2-Chloro-3 -(3 -chloro-2-(2-(2-hydroxy-3 -methylbutyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one was prepared in a similar fashion to compound 50 from 1-bromo-3-methylbutan-2-ol and 6-bromo-8-methoxy-1,2,3,4-tetrahydroisoquinoline hydrochloride salt. LCMS: m/z found 704 [M+H]⁺, RT = 1.84 min, (Method D). ¹H NMR (400 MHz, Methanol-*d*₄) δ 8.60 (d, 1H), 8.44 (s, 1H), 7.75 (d, 1H), 7.68 (dd, 1H), 7.52 (t, 1H), 7.46 - 7.34 (m, 2H), 7.24 (d, 1H), 7.13 (s, 2H), 4.27 (q, 2H), 4.00 (s, 3H), 3.91 - 3.86 (m, 5H), 3.83 - 3.73 (m, 1H), 3.48 - 3.36 (m, 2H), 3.24 - 3.06 (m, 5H), 2.85 - 2.70 (m, 2H), 2.39 - 2.20 (m, 3H), 1.85 - 1.74 (m, 1H), 1.77 - 1.64 (m, 1H), 0.97 (d, 6H).

### Example 29: (S)-5-((((6-(2-Chloro-3-(3-chloro-2-(2-(2-hydroxyethyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one (54)

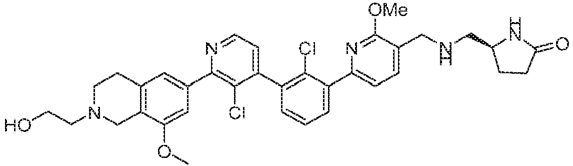

(S)-5-((((6-(2-Chloro-3-(3-chloro-2-(2-(2-hydroxyethyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one was prepared in a similar fashion to compound 50, from 2-bromoethanol and 6-bromo-8-methoxy-1,2,3,4-tetrahydroisoquinoline hydrochloride salt. LCMS: m/z found 662 [M+H]⁺, RT = 1.60 min (Method D). ¹H NMR (400 MHz, Methanol-*d*₄) δ 8.61 (d, 1H), 7.79 (d, 1H), 7.68 (dd, 1H), 7.53 (t, 1H), 7.45 - 7.35 (m, 2H), 7.27 (d, 1H), 7.14 (s, 2H), 4.87 (s, 1H), 4.32 (s, 2H), 4.11 - 3.98 (m, 5H), 3.98 - 3.87 (m, 6H), 3.49 (t, 2H), 3.31 (d, 1H), 3.18 (t, 2H), 3.00 - 2.86 (m, 2H), 2.41 - 2.23 (m, 3H), 1.91 - 1.77 (m, 1H).

### Example 30: (S)-5-((((6-(2-Chloro-3-(3-chloro-2-(8-methoxy-2-(2-(piperidin-4-yl)ethyl)-1,2,3,4-tetrahydroisoquinolin-6-yl)pyridin-4-yl)phenyl)-2-methoxypyridin-3-yl)methyl)amino)methyl)pyrrolidin-2-one (64)

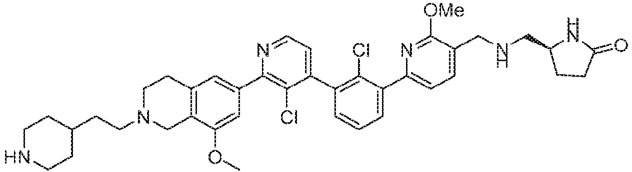

(S)-5-((((6-(2-Chloro-3-(3-chloro-2-(8-methoxy-2-(2-(piperidin-4-yl)ethyl)-1,2,3,4-tetrahydroisoquinolin-6-yl)pyridin-4-yl)phenyl)-2-methoxypyridin-3-yl)methyl)amino)methyl)pyrrolidin-2-one was prepared in a similar fashion to compound 50 using *tert*-butyl 4-(2-bromoethyl)piperidine-1-carboxylate and 6-bromo-8-methoxy-1,2,3,4-tetrahydroisoquinoline hydrochloride salt. LCMS: m/z found 729 [M+H]⁺, RT = 1.58 min, (Method D). ¹H NMR (400 MHz, Methanol-*d*₄) δ 8.59 (d, 1H), 8.41 (s, 1H), 7.79 (d, 1H), 7.69 (d, 1H), 7.53 (t, 1H), 7.45 - 7.35 (m, 2H), 7.27 (d, 1H), 7.10 (s, 2H), 4.09 - 3.95 (m, 7H), 3.88 (d, 4H), 3.36 (s, 2H), 3.19 - 3.10 (m, 4H), 3.01 - 2.92 (m, 6H), 2.39 - 2.24 (m, 3H), 1.99 (d, 2H), 1.81 - 1.64 (m, 4H), 1.45 (t, 2H).

### Example 31: 2-(((6-(2-Chloro-3-(3-chloro-2-(8-methoxy-2-(2-(piperidin-4-yl)ethyl)-1,2,3,4-tetrahydroisoquinolin-6-yl)pyridin-4-yl)phenyl)-2-methoxypyridin-3-yl)methyl)amino)ethan-1-ol (85)

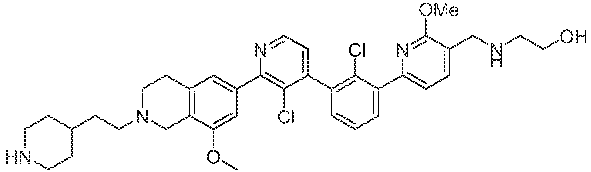

2-(((6-(2-Chloro-3-(3-chloro-2-(8-methoxy-2-(2-(piperidin-4-yl)ethyl)-1,2,3,4-tetrahydroisoquinolin-6-yl)pyridin-4-yl)phenyl)-2-methoxypyridin-3-yl)methyl)amino)ethan-1-ol was prepared in a similar fashion to compound **50** using *tert*-butyl 4-(2-bromoethyl)piperidine-1-carboxylate and 6-bromo-8-methoxy-1,2,3,4-tetrahydroisoquinoline hydrochloride salt in the initial transformation, and 2-aminoethanol in the final transformation. LCMS: m/z found 676 [M+H] ⁺at 1.58 min, (Method D). ¹H NMR (400 MHz, Methanol-*d*₄) δ 8.57 (d, 1H), 8.50 (s, 2H), 7.78 (d, 1H), 7.68 (d, 1H), 7.52 (t, 1H), 7.42 - 7.35 (m, 2H), 7.28 (d, 1H), 7.02 (d, 2H), 4.04 (d, 4H), 3.85 (s, 3H), 3.74 (t, 2H), 3.64 (s, 2H), 3.35 (d, 2H), 2.98 - 2.90 (m, 5H), 2.79 (t, 2H), 2.65 (t, 2H), 1.97 (d, 3H), 1.71 - 1.52 (m, 3H), 1.41 (t, 2H).

### Example 32: (S)-5-((((6-(2-Chloro-3-(3-chloro-2-(2-(2-(1-hydroxycyclopropyl)ethyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one (74)

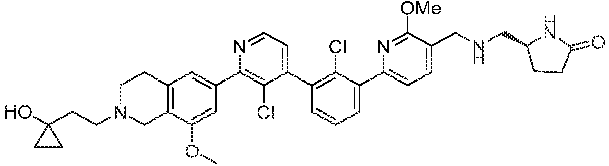

(S)-5-((((6-(2-Chloro-3-(3-chloro-2-(2-(2-(1-hydroxycyclopropyl)ethyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one was prepared in a similar fashion to compound 50, using 1-(2-bromoethyl)cyclopropan-1-ol and 6-bromo-8-methoxy-1,2,3,4-tetrahydroisoquinoline-HCl. LCMS: m/z found 702 [M+H]⁺, RT = 1.82 min, (Method D). ¹H NMR (400 MHz, Methanol-*d*₄) δ 8.57 (d, 1H), 8.49 (s, 1H), 7.75 - 7.63 (m, 2H), 7.51 (t, 1H), 7.43 - 7.33 (m, 2H), 7.22 (d, 1H), 7.06 - 6.99 (m, 2H), 3.99 (s, 2H), 3.92 - 3.76 (m, 6H), 3.68 (s, 2H), 3.00 - 2.87 (m, 4H), 2.86 - 2.75 (m, 4H), 2.76 - 2.61 (m, 2H), 2.52 (q, 2H), 2.37 - 2.18 (m, 3H), 1.85 - 1.72 (m, 1H), 1.02 (t, 3H).

### Example 33: (S)-5-((((6-(2-Chloro-3-(3-chloro-2-(5-fluoro-2-(2-hydroxy-2-methylpropyl)-1,2,3,4-tetrahydroisoquinolin-7-yl)pyridin-4-yl)phenyl)-2-methoxypyridin-3-yl)methyl)amino)methyl)pyrrolidin-2-one (47)

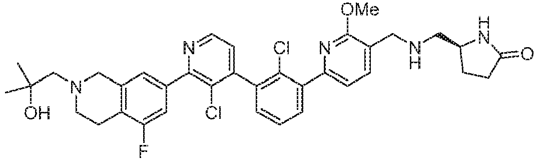

(S)-5-((((6-(2-Chloro-3-(3-chloro-2-(5-fluoro-2-(2-hydroxy-2-methylpropyl)-1,2,3,4-tetrahydroisoquinolin-7-yl)pyridin-4-yl)phenyl)-2-methoxypyridin-3-yl)methyl)amino)methyl)pyrrolidin-2-one was prepared in a similar fashion to compound 50, using 2,2-dimethyloxirane and 7-bromo-5-fluoro-1,2,3,4-tetrahydroisoquinoline-HCl, in EtOH at 70 °C in first step. LCMS: m/z found 678 [M+H]⁺, RT = 1.73 min (Method D). ¹H NMR (400 MHz, Methanol-*d*₄) δ 8.60 (d, 1H), 7.74-7.19 (m, 8H), 4.01 (s, 3H), 3.85 -3.82 (m, 4H), 2.95-2.85 (m, 4H), 2.71-2.65 (m, 2H), 2.53 (s, 2H), 2.35-2.24 (m, 3H), 1.84-1.76 (m, 1H),1.23 (s, 6H) and 0.09 (d, 1H).

### Example 34: (S)-5-((((6-(2-Chloro-3-(3-chloro-2-(8-fluoro-2-(2-hydroxy-2-methylpropyl)-1,2,3,4-tetrahydroisoquinolin-6-yl)pyridin-4-yl)phenyl)-2-methoxypyridin-3-yl)methyl)amino)methyl)pyrrolidin-2-one (46)

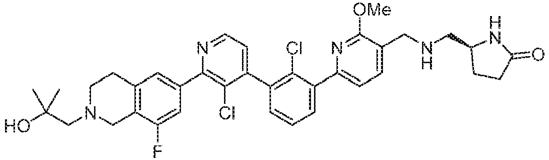

(S)-5-((((6-(2-Chloro-3-(3-chloro-2-(8-fluoro-2-(2-hydroxy-2-methylpropyl)-1,2,3,4-tetrahydroisoquinolin-6-yl)pyridin-4-yl)phenyl)-2-methoxypyridin-3-yl)methyl)amino)methyl)pyrrolidin-2-one was prepared in a similar fashion to compound 50, using 2,2-dimethyloxirane and 6-bromo-8-fluoro-1,2,3,4-tetrahydroisoquinoline-HCl, in EtOH at 70 °C in first step. LCMS: m/z found 678 [M+H]⁺, RT = 1.70 min (Method D). ¹H NMR (400 MHz, Methanol-*d*₄) δ 8.60 (dd, 1H), 7.74-7.16 (m, 8H), 4.01 (s, 3H), 3.85 -3.80 (m, 5H), 3.00-2.94 (m, 4H), 2.75-2.65 (m, 2H), 2.56 (s, 2H), 2.35-2.25 (m, 3H), 1.85-1.76 (m, 1H) and 1.24 (s, 6H).

### Example 35: (S)-5-((((6-(2-Chloro-3-(3-chloro-2-(2-(2-hydroxy-2-methylpropyl)-5-methoxy-1,2,3,4-tetrahydroisoquinolin-7-yl)pyridin-4-yl)phenyl)-2-methoxypyridin-3-yl)methyl)amino)methyl)pyrrolidin-2-one (45)

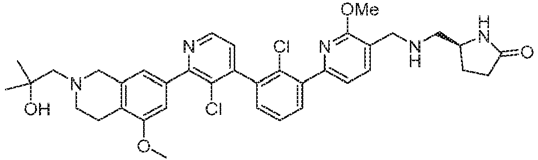

(S)-5-((((6-(2-Chloro-3-(3-chloro-2-(2-(2-hydroxy-2-methylpropyl)-5-methoxy-1,2,3,4-tetrahydroisoquinolin-7-yl)pyridin-4-yl)phenyl)-2-methoxypyridin-3-yl)methyl)amino)methyl)pyrrolidin-2-one was prepared in a similar fashion to compound 50, using 2,2-dimethyloxirane and 7-bromo-5-methoxy-1,2,3,4-tetrahydroisoquinoline-HCl, in EtOH at 70 °C in first step. LCMS: m/z found 690 [M+H]⁺, RT = 1.76 min (Method D). ¹H NMR (400 MHz, Methanol-*d*₄) δ 8.59 (dd, 1H), 8.53 (s, 1H), 7.76-7.23 (m, 6H), 7.03 (s,1H), 6.95 (s, 1H), 4.12 (m, 1H), 4.01 (s, 3H), 3.87 -3.81 (m, 7H), 3.00-2.95 (m, 2H), 2.82-2.80 (m, 2H), 2.75-2.68 (m, 2H), 2.56 (s, 2H), 2.35-2.25 (m, 3H), 1.84-1.75 (m, 1H) and 1.24 (s, 6H).

### Example 36: (S)-5-((((6-(2-Chloro-3-(3-chloro-2-(2-((S)-2-hydroxypropyl)-5-methoxy-1,2,3,4-tetrahydroisoquinolin-7-yl)pyridin-4-yl)phenyl)-2-methoxypyridin-3-yl)methyl)amino)methyl)pyrrolidin-2-one (44)

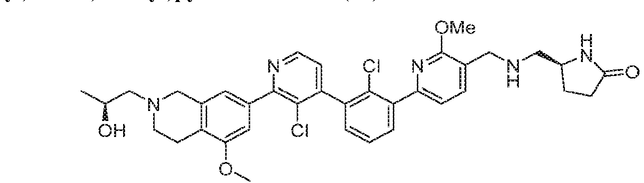

(S)-5-((((6-(2-Chloro-3-(3-chloro-2-(2-((S)-2-hydroxypropyl)-5-methoxy-1,2,3,4-tetrahydroisoquinolin-7-yl)pyridin-4-yl)phenyl)-2-methoxypyridin-3-yl)methyl)amino)methyl)pyrrolidin-2-one was prepared in a similar fashion to compound 50, using (S)-2-methyloxirane and 7-bromo-5-methoxy-1,2,3,4-tetrahydroisoquinoline-HCl, in EtOH at 70 °C in first step. LCMS: m/z found 676 [M+H]⁺, RT =1.71 min (Method D). ¹H NMR (400 MHz, Methanol-*d*₄) δ 8.60 (dd, 1H), 8.59 (s, 1H), 7.76-7.35 (m, 5H), 7.24 (dd, 1H), 7.08 (s,1H), 7.02 (s, 1H), 4.12 (m, 1H), 4.01 -3.81 (m, 11H), 3.10-3.03 (m, 2H), 2.92-2.87 (m, 3H), 2.76-2.65 (m, 5H), 2.35-2.23 (m, 3H), 1.84-1.77 (m, 1H) and 1.20 (dd, 1H).

### Example 37: 6-(3-Chloro-4-(2-chloro-3-(6-methoxy-5-(((((S)-5-oxopyrrolidin-2-yl)methyl)amino)methyl)pyridin-2-yl)phenyl)pyridin-2-yl)-2-((((1r,3r)-3-hydroxy-3-methylcyclobutyl)amino)methyl)-3-methylpyrrolo[2,1-f][1,2,4]triazin-4(3H)-one (102)

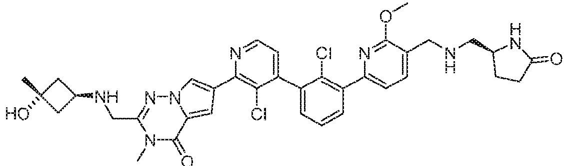

### (a) 1-(4-Bromo-1H-pyrrol-2-yl)-2,2,2-trichloroethanone

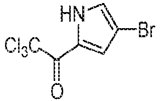

To a cooled solution (0 °C) of 2,2,2-trichloro-1-(1H-pyrrol-2-yl)ethanone (20 g, 94 mmol) in ACN (100 mL) was added NBS (16.8 g, 94 mmol) in portions. The reaction was stirred at 25 °C for 1.5 h. The reaction mixture was quenched by the slow addition of water (15 mL, 15.0 mL/g) and cooled to 0 °C. The solids were allowed to desaturate for an additional 1 h. The solids were filtered, washed with water (15 mL, 5.0 mL/g), and dried in vacuo to afford 1-(4-bromo-1H-pyrrol-2-yl)-2,2,2-trichloroethanone as a black solid (25 g, 91% yield). ¹H NMR (400 MHz, DMSO-*d*₆) δ = 12.84 (s, 1H), 7.54 (dd, 1H), 7.33 (dd, 1H).

### (b) 4-Bromo-1H-pyrrole-2-carboxamide

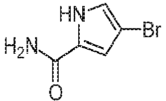

To a mixture of 1-(4-bromo-1H-pyrrol-2-yl)-2,2,2-trichloroethanone (25 g, 85.8 mmol) in ACN (125 mL) was added 30% of NH₃.H₂O (75.00 mL). The reaction was stirred at 25 °C for 12 h. The reaction mixture was concentrated under reduced pressure to give 4-bromo-1H-pyrrole-2-carboxamide as a black solid (15 g, 92% yield). The material was used without any purification.

### (c) 1-Amino-4-bromo-1H-pyrrole-2-carboxamide

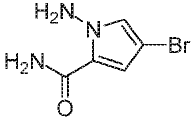

To a mixture of NaH (1.59 g, 39.7 mmol) suspended in DMF (15 mL) at 0° C, 4-bromo-1H-pyrrole-2-carboxamide (5 g, 26.4 mmol) was added slowly. After stirring at 0° C for 1 h, a solution of O-(2,4-dinitrophenyl)hydroxylamine (8.43 g, 42.3 mmol) in DMF (3 mL) was added dropwise for 30 min. The resulting reaction mixture was stirred at 0° C for 1.5 h. The reaction was terminated by slowly adding saturated sodium thiosulfate aqueous solution (120 mL). The resulting mixture was extracted with ethyl acetate (3 x 80 mL). The organic layer was washed with 10% lithium chloride aqueous solution (3 x 60 mL), dried with sodium sulfate, and filtered. The filtrate was concentrated, and the residue was purified by flash chromatography (SiO₂, 0-100% EtOAc/petroleum ether) to afford product 1-amino-4-bromo-1H-pyrrole-2-carboxamide (3 g, 55% yield). ¹H NMR (400 MHz, DMSO-*d*₆) δ = 7.93 (br, 1H), 7.27 - 7.25 (m, 1H), 7.00 (d, 1H), 6.74 (d, 1H).

### (d) ethyl 2-((4-bromo-2-carbamoyl-1H-pyrrol-1-yl)amino)-2-oxoacetate

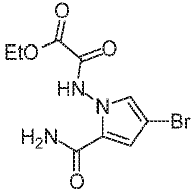

To the mixture 1-amino-4-bromo-1H-pyrrole-2-carboxamide (3 g, 12.5 mmol) and pyridine (0.99 g, 12.5 mmol) in THF (10 mL) was added ethyl-2-chloro-2-oxoacetate (1.71 g, 12.5 mmol). The resulting reaction mixture was stirred 25 °C for 3 h. The mixture was concentrated under reduce pressure. The residue was suspended in methylene chloride (10 mL) and the solid was collected by filtration to afford ethyl 2-((4-bromo-2-carbamoyl-1H-pyrrol-1-yl) amino)-2-oxoacetate as a yellow solid (2.5 g, 65% yield). ¹H NMR (400 MHz, DMSO-*d*₆) δ = 12.16 (s, 1H), 7.59 (br, 1H), 7.20 (d, 1H), 7.08 (br, 1H), 6.94 (d, 1H), 4.31 (q, 2H), 1.31 (t, 3H).

### (e) ethyl 6-bromo-4-oxo-3,4-dihydropyrrolo[2,1-f][1,2,4]triazine-2-carboxylate

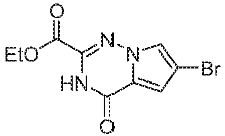

A mixture of ethyl 2-((4-bromo-2-carbamoyl-1H-pyrrol-1-yl)amino)-2-oxoacetate (2.1 g, 6.9 mmol), chlorotrimethylsilane (16.6 g, 152 mmol) and TEA (21.2 mL, 152 mmol) in DCE (210 mL) was heated at 110 °C for 1 h. The reaction mixture was filtered, and the filtrate was concentrated. The reaction mixture was diluted with water (80 mL) and extracted with EtOAc (3 x 60 ml). The combined organic layers were washed with brine (50 mL), dried over anhydrous sodium sulfate, filtered and concentrated under reduced pressure. The residue was purified by flash SiO₂ chromatography (0-100% EtOAc/petroleum ether) to afford ethyl 6-bromo-4-oxo-3,4-dihydropyrrolo [2,1-f][1,2,4]triazine-2-carboxylate as a yellow solid (1.1 g). ¹H NMR (400 MHz, DMSO-*d*₆) δ = 12.31 (br, 1H), 8.05 (s, 1H), 7.13 (s, 1H), 4.39 (q, 2H), 1.35 (t, 3H).

### (f) ethyl 6-bromo-3-methyl-4-oxo-3,4-dihydropyrrolo[2,1-f][1,2,4]triazine-2-carboxylate

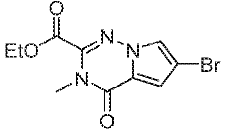

To a mixture of ethyl 6-bromo-4-oxo-3,4-dihydropyrrolo[2,1-f][1,2,4]triazine-2-carboxylate (1.5 g, 5.24 mmol) and potassium carbonate (0.72 g, 5.24 mmol) in DMF (20 mL), iodomethane (0.74 g, 5.24 mmol) was added dropwise. The mixture was stirred at 25 °C for 1 h. The reaction mixture was diluted with water (30 mL) and saturated ammonium chloride (50 mL) aqueous solution. The aqueous layer was extracted with EtOAc (3 x20 mL). The combined organic layers were washed with brine (20 mL), dried over anhydrous sodium sulfate, filtered and concentrated under reduced pressure. The residue was purified by flash chromatography (SiO₂, 0-100% EtOAc/petroleum ether) to afford ethyl 6-bromo-3-methyl-4-oxo-3,4-dihydropyrrolo[2,1-f][1,2,4]triazine-2-carboxylate as a yellow solid (1.02 g, 64% yield). ¹H NMR (400 MHz, DMSO-*d*₆) δ = 7.95 (d, 1H), 7.09 (d, 1H), 4.40 (q, 2H), 3.42 (s, 3H), 1.32 (t, 3H).

### (g) 6-Bromo-2-(hydroxymethyl)-3-methylpyrrolo[2,1-f][1,2,4]triazin-4(3H)-one

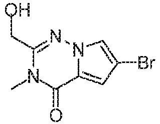

To a mixture ethyl 6-bromo-3-methyl-4-oxo-3,4-dihydropyrrolo[2,1-f][1,2,4]triazine-2-carboxylate (0.5 g, 1.67 mmol) in THF/ MeOH (10:11, 21 mL) was added sodium borohydride (0.19 g, 5 mmol) at 0 °C. The mixture was stirred at 20 °C for 2 h. The reaction mixture was quenched by addition of water (20 mL), and then extracted with EtOAc (3 x 30 ml). The combined organic layers were washed with brine (20 mL), dried over anhydrous sodium sulfate, filtered and concentrated under reduced pressure. The residue was purified by flash SiO₂ chromatography (0-100% EtOAc/petroleum ether) to afford 6-bromo-2-(hydroxymethyl)-3-methylpyrrolo[2,1-f][1,2,4]triazin-4(3H)- one as a yellow solid (0.35 g, 81% yield). ¹H NMR (400 MHz, DMSO-*d*₆) δ7.83 (d, 1H), 7.00 (d, 1H), 5.88 (t, 1H), 4.48 (d, 2H), 3.50 (s, 3H).

### (h) 6-Bromo-2-(((tert-butyldimethylsilyl)oxy)methyl)-3-methylpyrrolo[2,1-f][1,2,4]triazin-4(3H)-one

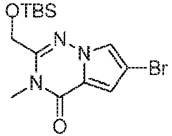

To a solution of 6-bromo-2-(hydroxymethyl)-3-methylpyrrolo[2,1-f][1,2,4]triazin-4(3H)-one (3 g, 11.6 mmol) in DMF (45 mL) was added tert-butylchlorodimethylsilane (2.14 mL, 17.4 mmol) and imidazole (1.58 g, 23.3 mmol). The mixture was stirred at rt for 3 h. The reaction mixture was partitioned between H₂O (80 mL) and EtOAC (60 mL). The organic phase was separated, washed with brine (30 mL x 2), dried over anhydrous sodium sulfate, filtered and concentrated under reduced pressure. The residue was purified by flash chromatography (SiO₂, 0-50% EtOAc/petroleum ether) to afford 6-bromo-2-(((tertbutyldimethylsilyl)oxy)methyl)-3-methylpyrrolo[2,1-f] [1,2,4]triazin-4(3H)-one as a yellow solid (3.98 g, 92% yield). ¹H NMR (400 MHz, CDCl₃): δ 7.29 (d, 1H), 7.01 (d, 1H), 4.63 (s, 2H), 3.62 (s, 3H), 0.91 (s, 9H), 0.13 (s, 6H).

### (i) 2-(((tert-Butyldimethylsilyl)oxy)methyl)-3-methyl-6-(4,4,5,5-tetramethyl-1,3,2-dioxaborolan-2-yl)pyrrolo[2,1-f][1,2,4]triazin-4(3H)-one

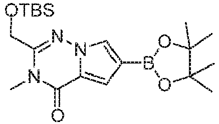

To a mixture of 6-bromo-2-(((*tert*-butyldimethylsilyl)oxy)methyl)-3-methylpyrrolo[2,1-f] [1,2,4]triazin-4(3H)-one (3.88 g, 10.4 mmol), 4,4,4',4',5,5,5',5'-octamethyl-2,2'-bi(1,3,2- dioxaborolane) (5.29 g, 20.8 mmol), potassium acetate (2.05 g, 20.84 mmol) in dioxane (80 mL) was added 1,1'-bis(diphenylphosphino)ferrocene-palladium(II) dichloride dichloromethane complex (851 mg, 1.04 mmol). The mixture was stirred at 110 °C for 12 h. The reaction mixture was partitioned between H₂O (50 mL) and EtOAC (70 mL). The organic phase was separated, washed with brine (40 mL), dried over anhydrous sodium sulfate, filtered and concentrated under reduced pressure. The residue was purified by flash chromatography (SiO₂, 0-10% EtOAc/petroleum ether) to afford 2-(((*tert*-butyldimethylsilyl)oxy)methyl)-3-methyl-6-(4,4,5,5-tetramethyl-1,3,2-dioxaborolan-2-yl)pyrrolo[2,1-f][1,2,4]triazin-4(3H)-one as a yellow solid (2.4 g, 54.9% yield) . ¹H NMR (400 MHz, CDCl₃): δ 7.63 (d, 1H), 7.33 (d, 1H), 4.64 (s, 2H), 3.60 (s, 3H), 1.34 (s, 12H), 0.90 (s, 9H), 0.12 (s, 6H).

### (j) (S)-tert-Butyl ((6-(3-(2-(2-(((tert-butyldimethylsilyl)oxy)methyl)-3-methyl-4- oxo-3,4-dihydropyrrolo[2,1-f][1,2,4]triazin-6-yl)-3-chloropyridin-4-yl)-2-chlorophenyl)-2-methoxypyridin-3-yl)methyl)((5-oxopyrrolidin-2-yl)methyl)carbamate

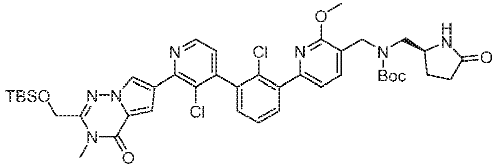

To the mixture of 2-(((tert-butyldimethylsilyl)oxy)methyl)-3-methyl-6-(4,4,5,5-tetra methyl-1,3,2-dioxaborolan-2-yl)pyrrolo[2,1-f][1,2,4]triazin-4(3H)-one (200 mg, 0.48 mmol) and (S)-tert-butyl ((6-(2-chloro-3-(2,3-dichloropyridin-4-yl)phenyl)-2-methoxy pyridin-3-yl)methyl)((5-oxopyrrolidin-2-yl)methyl)carbamate (282 mg, 0.48 umol) in THF/ H₂O (7 mL, 6/1) was added potassium carbonate (198 mg, 1.43 mmol) and tetrakis(triphenylphosphine)palladium(0) (55.1 mg, 0.48 mmol). The reaction was stirred for 12 h at 80 °C. The mixture was diluted with H₂O (30 mL) and extracted with EtOAc (120 mL). The organic layer was separated, dried over sodium sulfate and concentrated under vacuum. The residue was purified by flash chromatography (SiO₂, 0-100% EtOAc/petroleum ether to 0- 100% MeOH/EtOAc) to afford (S)-*tert*-butyl ((6-(3-(2-(2-(((*tert-*butyldimethylsilyl)oxy)methyl)-3-methyl-4-oxo-3,4-dihydropyrrolo[2,1-f][1,2,4]triazin-6-yl)-3-chloropyridin-4-yl)-2-chlorophenyl)-2-methoxypyridin-3-yl)methyl)((5-oxopyrrolidin-2-yl)methyl)carbamate. (1.2 g, m/z: 848 [M+H]⁺).

### (k) (S)-tert-Butyl ((6-(2-chloro-3-(3-chloro-2-(2-(hydroxymethyl)-3-methyl-4-oxo-3,4-dihydropyrrolo[2,1-f][1,2,4]triazin-6-yl)pyridin-4-yl)phenyl)-2-methoxypyridin-3-yl)methyl)((5-oxopyrrolidin-2-yl)methyl)carbamate

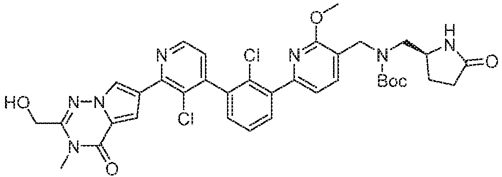

To a mixture of (S)-*tert*-butyl ((6-(3-(2-(2-(((*tert*-butyldimethylsilyl)oxy)methyl)-3-methyl-4-oxo-3,4-dihydropyrrolo[2,1-f][1,2,4]triazin-6-yl)-3-chloropyridin-4-yl)-2-chloro phenyl)-2-methoxypyridin-3-yl)methyl)((5-oxopyrrolidin-2-yl)methyl)carbamate (1.1 g, 1.30 mmol) in THF (5 mL) was added TBAF (1.94 mL,1.94 mmol, 1M in THF). The mixture was stirred for 12 h at rt. The mixture was concentrated and the residue was purified by flash chromatography (SiO₂, 0-100% EtOAc/petroleum ether, then 0 - 100% MeOH/EtOAc) to afford (S)-*tert*-butyl ((6-(2-chloro-3-(3-chloro-2-(2-(hydroxy methyl)-3-methyl-4-oxo-3,4-dihydropyrrolo[2,1-f][1,2,4]triazin-6-yl)pyridin-4-yl)phenyl)-2-methoxypyridin-3-yl)methyl)((5-oxopyrrolidin-2-yl)methyl)carbamate (660 mg, 52% yield. m/z: 734 [M+H]⁺). ¹H NMR (400 MHz, Methanol-*d*₄): δ 8.62 (d, 1H), 8.21 (d, 1H), 8.11 (s, 1H), 7.89-7.87 (m, 1H), 7.71 (dd, 1H), 7.64 (s, 1H), 7.71 (t, 1H), 7.58-7.54 (m, 2H), 7.40 (dd, 1H), 7.31 (d, 1H), 7.29 (d, 1H), 6.94 (s, 1H), 4.66 (s, 2H), 4.52 (s, 2H), 4.03 (s, 3 H), 4.02-3.98 (m, 1H), 3.65 (s, 3H), 3.46-3.40 (m, 2H), 2.40-2.28 (m, 3H), 1.85-1.83 (m, 1H), 1.42 (s, 19H).

### (l) (S)-tert-Butyl ((6-(2-chloro-3-(3-chloro-2-(2-formyl-3-methyl-4-oxo-3,4-dihydropyrrolo[2,1-f][1,2,4]triazin-6-yl)pyridin-4-yl)phenyl)-2-methoxypyridin-3-yl)methyl)((5-oxopyrrolidin-2-yl)methyl)carbamate

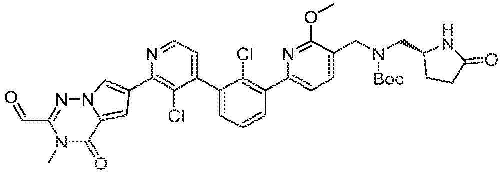

To a mixture of (S)-tert-butyl ((6-(2-chloro-3-(3-chloro-2-(2-(hydroxymethyl) -3-methyl-4-oxo-3,4-dihydropyrrolo[2,1-f][1,2,4]triazin-6-yl)pyridin-4-yl)phenyl)-2-methoxypyridin-3-yl)methyl)((5-oxopyrrolidin-2-yl)methyl)carbamate (540 mg, 0.74 mmol) in methylene chloride (10 mL) was added Dess-Martin periodinane (624 mg, 1.47 mmol). The mixture was stirred for 1 h at rt. The solvent was evaporated and the residue was purified by flash chromatography (SiO₂, 0-100% EtOAc/petroleum ether) to afford (S)-tert-butyl ((6-(2-chloro-3-(3-chloro-2-(2-formyl-3-methyl-4-oxo-3,4- dihydropyrrolo [2,1-f][1,2,4]triazin-6-yl)pyridin-4-yl)phenyl)-2-methoxypyridin-3-yl)methyl)((5-oxopyrrolidin-2-yl)methyl)carbamate as a yellow solid (420 mg, 78% yield).

### (m) tert-Butyl ((6-(2-chloro-3-(3-chloro-2-(2-((((1r,3r)-3-hydroxy-3-methylcyclobutyl) amino)methyl)-3-methyl-4-oxo-3,4-dihydropyrrolo[2,1-f][1,2,4]triazin-6-yl)pyridin-4-yl)phenyl)-2-methoxypyridin-3-yl)methyl)(((S)-5-oxopyrrolidin-2-yl)methyl)carbamate

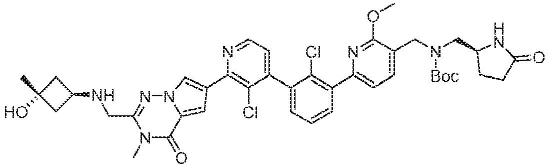

To a mixture of (S)-*tert*-butyl ((6-(2-chloro-3-(3-chloro-2-(2-formyl-3-methyl-4-oxo-3,4-dihydropyrrolo[2,1-f][1,2,4]triazin-6-yl)pyridin-4-yl)phenyl)-2-methoxypyridin-3-yl)methyl)((5-oxopyrrolidin-2-yl)methyl)carbamate (150 mg, 0.2 mmol) in methylene chloride (5 mL) was added sodium acetate (50.4 mg, 0.61 mmol) and 3-amino-1-methyl-cyclobutanol, HCl (31.1 mg, 0.23 mmol). The mixture was stirred at rt for 12 h. To the mixture was added sodium cyanoborohydride (38.6 mg, 0.61 mmol) and the mixture stirred for another 1 h. The residue was purified by prep-TLC (20% MeOH/EtOAc) to afford *tert-*butyl ((6-(2-chloro-3-(3-chloro-2-(2-((((1r,3r)-3-hydroxy-3-methylcyclobutyl)amino) methyl)-3-methyl-4-oxo-3,4-dihydropyrrolo[2,1-f][1,2,4]triazin-6-yl)pyridin-4-yl)phenyl)-2-methoxypyridin-3-yl)methyl)(((S)-5-oxopyrrolidin-2-yl)methyl)carbamate as a white solid (100 mg, 49% yield). m/z: 717 [M+H]⁺).

### (n) 6-(3-Chloro-4-(2-chloro-3-(6-methoxy-5-(((((S)-5-oxopyrrolidin-2-yl)methyl)amino)methyl)pyridin-2-yl)phenyl)pyridin-2-yl)-2-((((1r,3r)-3-hydroxy-3-methylcyclobutyl)amino)methyl)-3-methylpyrrolo[2,1-f][1,2,4]triazin-4(3H)-one

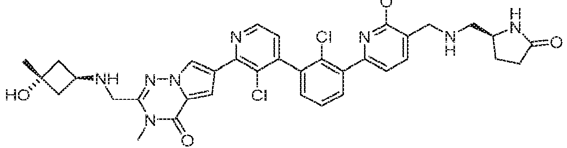

To a mixture of *tert*-butyl ((6-(2-chloro-3-(3-chloro-2-(2-((((1r,3r)-3-hydroxy-3-methylcyclobutyl)amino)methyl)-3-methyl-4-oxo-3,4-dihydropyrrolo[2,1-f][1,2,4]triazin-6-yl)pyridin-4-yl)phenyl)-2-methoxypyridin-3-yl)methyl)(((S)-5-oxopyrrolidin-2-yl)methyl)carbamate (100 mg, 0.12 mmol) in methylene chloride (1 mL) was added 2,2,2-trifluoroacetic acid (2 mL, 27.0 mmol) and stirred for 0.5 h at rt. The mixture was concentrated under reduced pressure and the residue was purified by reverse phase HPLC to afford 6-(3-chloro-4-(2-chloro-3- (6-methoxy-5-(((((S)-5-oxopyrrolidin-2-yl)methyl)amino)methyl)pyridin-2-yl)phenyl)pyridin-2-yl)-2-((((1r,3r)-3-hydroxy-3-methylcyclobutyl)amino)methyl)-3-methylpyrrolo[2,1-f][1,2,4]triazin-4(3H)-one (22.9 mg, 24% yield). m/z: 717 [M+H]⁺. ¹H NMR (400 MHz, DMSO-*d*₆): δ 8.67 (d, 1H), 8.22 (d, 1H), 8.11 (s, 1H), 7.89-7.87 (m, 1H), 7.71 (dd, 1H), 7.64 (s, 1H), 7.60 (t, 1H), 7.53 (d, 1H), 7.47 (dd, 1H), 7.38 (d, 1H), 7.33-7.32 (m, 1H), 4.72 (s, 1H), 3.95 (s, 4H), 3.76-3.73 (m, 2H), 3.51 (s, 3H), 3.26-3.25 (m, 5H), 2.16-2.08 (m, 5H), 1.76-1.71 (m, 2H), 1.23 (s, 3H).

### Example 38: 6-(3-Chloro-4-(2-chloro-3-(6-methoxy-5-(((((S)-5-oxopyrrolidin-2-yl)methyl)amino)methyl)pyridin-2-yl)phenyl)pyridin-2-yl)-2-((((S)-2-hydroxypropyl)amino)methyl)-3-methylpyrrolo[2,1-f][1,2,4]triazin-4(3H)-one (91)

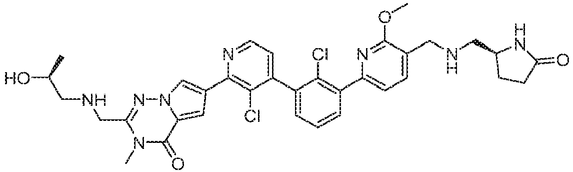

### (a) tert-Butyl ((6-(2-chloro-3-(3-chloro-2-(2-((((S)-2-hydroxypropyl)amino)methyl) - 3-methyl-4-oxo-3,4-dihydropyrrolo[2,1-f][1,2,4]triazin-6-yl)pyridin-4-yl)phenyl)-2-methoxypyridin-3-yl)methyl)(((S)-5-oxopyrrolidin-2-yl)methyl)carbamate

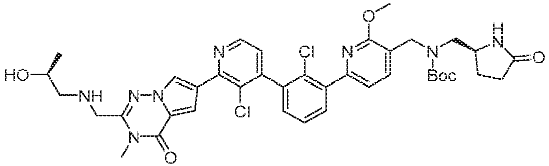

To a mixture of (S)-*tert*-butyl ((6-(2-chloro-3-(3-chloro-2-(2-formyl-3-methyl-4-oxo-3,4-di hydropyrrolo[2,1-f][1,2,4]triazin-6-yl)pyridin-4-yl)phenyl)-2-methoxypyridin-3-yl)methyl)((5-oxopyrrolidin-2-yl)methyl)carbamate (30 mg, 0.04 mmol) in methylene chloride (2 mL) was added sodium acetate (10.1 mg, 0.12 mmol) and (S)-1-aminopropan-2-ol (4.61 mg, 0.06 mmol). The mixture was stirred at rt for 11 h. To the mixture was added sodium cyanoborohydride (7.72 mg, 0.12 mmol) and stirred for another 1 h. The mixture was purified by prep-TLC (20% MeOH/EtOAc) to afford *tert-butyl* ((6-(2-chloro-3- (3-chloro-2-(2-((((S)-2-hydroxypropyl)amino)methyl)-3-methyl-4-oxo-3,4-dihydropyrrolo [2,1-f][1,2,4]triazin-6-yl)pyridin-4-yl)phenyl)-2-methoxypyridin-3-yl)methyl)(((S)-5-oxopyrrolidin-2-yl)methyl)carbamate as a white solid (17 mg, 52% yield).

### (b) 6-(3-Chloro-4-(2-chloro-3-(6-methoxy-5-(((((S)-5-oxopyrrolidin-2-yl)methyl)amino)methyl)pyridin-2-yl)phenyl)pyridin-2-yl)-2-((((S)-2-hydroxypropyl)amino)methyl)-3-methylpyrrolo[2,1-f][1,2,4]triazin-4(3H)-one

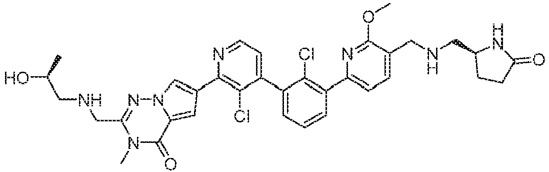

To a mixture of *tert*-butyl ((6-(2-chloro-3-(3-chloro-2-(2-((((S)-2-hydroxypropyl)amino) methyl)-3-methyl-4-oxo-3,4-dihydropyrrolo[2,1-f][1,2,4]triazin-6-yl)pyridin-4-yl)phenyl)-2-methoxypyridin-3-yl)methyl)(((S)-5-oxopyrrolidin-2-yl)methyl)carbamate (17 mg, 21.5 umol) in methylene chloride (0.3 mL) was added 2,2,2-trifluoroacetic acid (0.3 mL, 4.05 mmol). The mixture was stirred for 0.5 h at rt. The mixture was concentrated under reduced pressure directly. The residue was purified by reverse phase HPLC to afford 6-(3-chloro -4-(2-chloro-3-(6-methoxy-5-(((((S)-5-oxopyrrolidin-2-yl)methyl) amino)methyl)pyridin- 2-yl)phenyl)pyridin-2-yl)-2-((((S)-2-hydroxypropyl)amino)methyl)-3-methylpyrrolo[2,1-f][1,2,4]triazin-4(3H)-one (7 mg, 9.42 umol, 43.8% yield). m/z: 691 [M+H]⁺. ¹H NMR (400 MHz, DMSO): δ 8.69 (d, 1H), 8.24 (d, 1H), 8.15 (s, 2H), 7.85 (d, 1H), 7.73 (d, 1H), 7.69 (s, 1H), 7.60 (t, 1H), 7.55 (d, 1H), 7.48 (d, 1H), 7.40 (d, 1H), 7.30 (d, 1H), 3.93 (s, 3H), 3.87-3.82 (m, 3H), 3.75-3.64 (m, 6H), 3.55 (s, 4H), 2.16-2.08 (m, 3H), 1.72-1.70 (m, 1H), 1.05 (d, 3H).

### Example 39: 6-(3-Chloro-4-(2-chloro-3-(6-methoxy-5-(((((S)-5-oxopyrrolidin-2-yl)methyl)amino)methyl)pyridin-2-yl)phenyl)pyridin-2-yl)-3-methyl-2-(((((S)-5-oxopyrrolidin-2-yl)methyl)amino)methyl)pyrrolo[2,1-f][1,2,4]triazin-4(3H)-one (103)

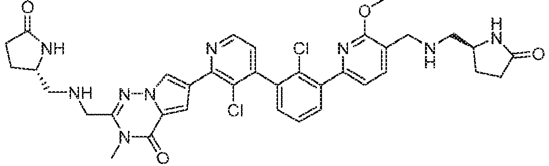

### (a) tert-Butyl ((6-(2-chloro-3-(3-chloro-2-(3-methyl-4-oxo-2-(((((S)-5-oxopyrrolidin-2-yl) methyl)amino)methyl)-3,4-dihydropyrrolo[2,1-f][1,2,4]triazin-6-yl)pyridin-4-yl)phenyl)-2-methoxypyridin-3-yl)methyl)(((S)-5-oxopyrrolidin-2-yl)methyl)carbamate

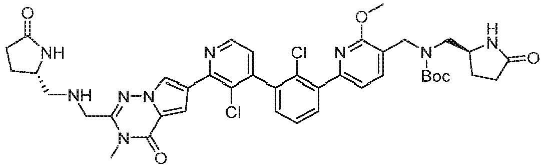

To a mixture of (S)-*tert*-butyl ((6-(2-chloro-3-(3-chloro-2-(2-formyl-3-methyl-4-oxo-3,4- dihydropyrrolo[2,1-f][1,2,4]triazin-6-yl)pyridin-4-yl)phenyl)-2-methoxypyridin-3-yl)methyl)((5-oxopyrrolidin-2-yl)methyl)carbamate (150 mg, 0.2 mmol) in methylene chloride (2 mL) was added sodium acetate (50.4 mg, 0.61 mmol) and (S)-5-(aminomethyl)pyrrolidin-2-one, HCl (34.0 mg, 0.23 mmol). The mixture was stirred at rt for 12 h. To the mixture was added sodium cyanoborohydride (38.6 mg, 0.61 mmol) and stirring continued for 1 h. The residue was purified by prep-TLC (20% MeOH/EtOAc) to afford *tert*-butyl ((6-(2-chloro-3-(3-chloro-2- (3-methyl-4-oxo-2-(((((S)-5-oxopyrrolidin-2-yl)methyl)amino)methyl)-3,4-dihydropyrrolo[2,1-f][1,2,4]triazin-6-yl)pyridin-4-yl)phenyl)-2-methoxypyridin-3-yl)methyl)(((S)-5-oxopyrrolidin-2-yl)methyl)carbamate as a white solid (70 mg, 30% yield, m/z: 830 [M+H]⁺)

### (b) 6-(3-Chloro-4-(2-chloro-3-(6-methoxy-5-(((((S)-5-oxopyrrolidin-2-yl)methyl)amino)methyl)pyridin-2-yl)phenyl)pyridin-2-yl)-3-methyl-2-(((((S)-5-oxopyrrolidin-2-yl)methyl)amino)methyl)pyrrolo[2,1-f][1,2,4]triazin-4(3H)-one

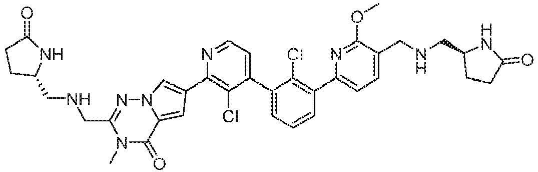

To a mixture of *tert*-butyl ((6-(2-chloro-3-(3-chloro-2-(3-methyl-4-oxo-2-(((((S)-5-oxo pyrrolidin-2-yl)methyl)amino)methyl)-3,4-dihydropyrrolo[2,1-f][1,2,4]triazin-6-yl)pyridin-4-yl)phenyl)-2-methoxypyridin-3-yl)methyl)(((S)-5-oxopyrrolidin-2-yl)methyl)carbamate (70 mg, 0.08 mmol) in methylene chloride (1 mL) was added 2,2,2-trifluoroacetic acid (2 mL g, 27.0 mmol). The mixture was stirred for 0.5 h at rt. The reaction mixture was concentrated under vacuum. The residue was purified by reverse phase HPLC to afford 6-(3-chloro-4-(2-chloro -3-(6-methoxy-5-(((((S)-5-oxopyrrolidin-2-yl)methyl) amino)methyl)pyridin-2-yl) phenyl)pyridin-2-yl)-3-methyl-2-(((((S)-5-oxopyrrolidin-2-yl) methyl)amino)methyl)pyrrolo[2,1-f][1,2,4]triazin-4(3H)-one (20.1 mg, 30% yield). m/z: 730 [M+H]⁺). ¹H NMR (400 MHz, DMSO-*d*₆): δ 8.66 (d, 1H), 8.21 (d, 1H), 8.12 (s, 1H), 7.82 (d, 1H), 7.71 (dd, 1H), 7.67 (s, 1H), 7.64 (s, 1H), 7.57 (t, 1H), 7.52 (d, 1H), 7.45 (dd, 1H), 7.37 (d, 1H), 7.27 (d, 1H), 3.91 (s, 3H), 3.79 (s, 2H), 3.72 (s, 2H), 3.65-3.57 (m, 2H), 3.52 (s, 3H), 2.60 (d, 2H), 2.56 (d, 2H), 2.13-2.04 (m, 6H), 1.69-1.66 (m, 2H).

### Example 40: 6-[3-Chloro-4-[2-chloro-3-[6-methoxy-5-[[[(2S)-5-oxopyrrolidin-2-yl]methylamino]methyl]-2-pyridyl]phenyl]-2-pyridyl]-2-[(isopropylamino)methyl]-3-methyl-pyrrolo[2,1-f] [1,2,4]triazin-4-one (111)

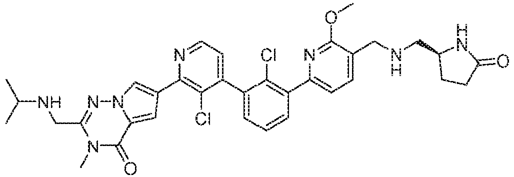

### (a) tert-Butyl N-[[6-[2-chloro-3-[2-chloro-3-[2-[(isopropylamino)methyl]-3-methyl-4-oxo- pyrrolo [2,1-f][1,2,4]triazin-6-yl]phenyl]phenyl]-2-methoxy-3-pyridyl]methyl]-N-[[(2S)-5-oxo pyrrolidin-2-yl]methyl]carbamate

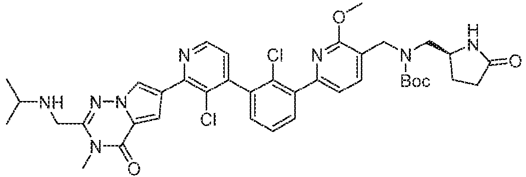

To a solution of *tert-butyl* N-[[6-[2-chloro-3-[2-chloro-3-[2-(hydroxymethyl)-3-methyl -4-oxo-pyrrolo[2,1-f][1,2,4]triazin-6-yl]phenyl]phenyl]-2-methoxy-3-pyridyl]methyl]-N-[[(2S)-5-oxopyrrolidin-2-yl]methyl]carbamate (20 mg, 0.03 mmol) in methylene chloride (0.4 mL) was added mesyl chloride (2.53 uL, 0.03 mmol) and triethylamine (11 uL, 0.08 mmol). The reaction was stirred at room temperature for 1 h. The solvent was evaporated, and the residue was taken up in DMF (0.4 mL) followed by the addition of propan-2-amine (22 uL, 0.27 mmol). The reaction was stirred at room temperature for 12 h. The solvent was evaporated, and the residue was suspended in water. The precipitate formed was collected to afford *tert*-butyl N-[[6-[2-chloro-3-[2-chloro -3-[2-[(isopropylamino)methyl]-3-methyl-4-oxo-pyrrolo[2,1-f][1,2,4]triazin-6-yl]phenyl] phenyl]-2-methoxy-3-pyridyl]methyl]-N-[[(2S)-5-oxopyrrolidin-2-yl]methyl]carbamate (14 mg, 75.8%). The material was used without any purification. m/z: [M+H]⁺ 776.1

### (b) 6-[3-Chloro-4-[2-chloro-3-[6-methoxy-5-[[[(2S)-5-oxopyrrolidin-2-yl]methylamino]methyl]-2-pyridyl]phenyl]-2-pyridyl]-2-[(isopropylamino)methyl]-3-methyl-pyrrolo [2,1-f] [1,2,4]triazin-4-one

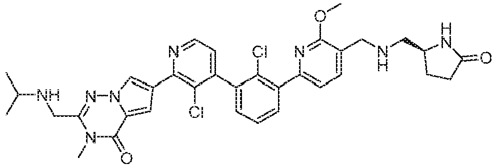

To a solution of *tert*-butyl N-[[6-[2-chloro-3-[3-chloro-2-[2-[(isopropylamino)methyl] -3-methyl-4-oxo-pyrrolo[2,1-f][1,2,4]triazin-6-yl]-4-pyridyl]phenyl]-2-methoxy-3-pyridyl]methyl]-N-[[(2S)-5-oxopyrrolidin-2-yl]methyl]carbamate (14 mg, 0.02 mmol) in methylene chloride (0.4 mL) was added TFA (69 uL, 0.90 mmol). The reaction was stirred at rt for 30 min. The solvent was evaporated and the residue was purified by reverse phase HPLC (acetonitrile: water) to afford 6-[3-chloro-4-[2-chloro-3-[6-methoxy-5-[[[(2S)-5-oxopyrrolidin-2-yl]methylamino]methyl]-2-pyridyl]phenyl]-2-pyridyl]-2-[(isopropylamino)methyl]-3-methyl-pyrrolo[2,1-f][1,2,4]triazin-4-one (8 mg, 65.6%). m/z: [M+H]⁺ = 677.1. ¹H NMR (400 MHz, methanol-*d*₄) δ 8.59 (d, 1H), 8.51 (s, 1H), 8.20 (d, 1H), 7.75 (d, 1H), 7.72 - 7.66 (m, 2H), 7.53 (t, 1H), 7.37 (dd, 1H), 7.27 (dd, 2H), 4.09 (s, 2H), 4.02 (s, 3H), 3.92 - 3.81 (m, 3H), 3.54 (s, 3H), 3.16 (p, 1H), 2.81 - 2.68 (m, 2H), 2.41 - 2.22 (m, 3H), 1.89 - 1.74 (m, 1H), 1.25 (d, 6H).

### Example 41: 6-[3-Chloro-4-[2-chloro-3-[6-methoxy-5-[[[(2S)-5-oxopyrrolidin-2-yl]methylamino]methyl]-2-pyridyl]phenyl]-2-pyridyl]-2-[(6-hydroxy-2-azaspiro[3.3]heptan-2-yl)methyl]-3-methyl-pyrrolo[2,1-f][1,2,4]triazin-4-one (48)

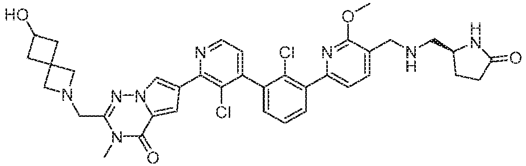

### (a) tert-Butyl N-[[6-[2-chloro-3-[3-chloro-2-[2-[(6-hydroxy-2-azaspiro [3.3]heptan-2-yl) methyl] -3-methyl-4-oxo-pyrrolo[2,1-f] [1,2,4]triazin-6-yl]-4-pyridyl] phenyl]-2-methoxy-3-pyridyl]methyl]-N-[[(2S)-5-oxopyrrolidin-2-yl]methyl]carbamate

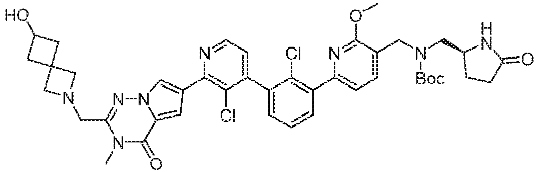

To a solution of *tert*-butyl N-[[6-[2-chloro-3-[3-chloro-2-[2-(hydroxymethyl) -3-methyl-4-oxo-pyrrolo[2,1-f][1,2,4]triazin-6-yl]-4-pyridyl]phenyl]-2-methoxy-3-pyridyl]methyl]-N-[[(2S)-5-oxopyrrolidin-2-yl]methyl]carbamate (16 mg, 0.02 mmol) in methylene chloride (0.3 mL) was added mesyl chloride (3 uL, 0.04 mmol) and TEA (15 uL, 0.11 mmol). The reaction was stirred for 1 h at room temperature. The solvent was evaporated, and the residue was suspended in DMF (0.5 mL). To this suspension, potassium carbonate (8 mg, 0.07 mmol) and 2-azaspiro[3.3]heptan-6-ol, HCL (9 mg, 0.07 mmol) were added and the reaction was stirred at room temperature for 72 h. The solvent was evaporated, and the residue was suspended in water. The precipitate formed was collected to afford *tert-*butyl N-[[6-[2-chloro-3-[3-chloro-2-[2-[(6- hydroxy-2-azaspiro[3.3]heptan-2-yl)methyl]-3-methyl-4-oxo-pyrrolo[2,1-f][1,2,4]triazin-6-yl]-4-pyridyl]phenyl]-2-methoxy-3-pyridyl]methyl]-N-[[(2S)-5-oxopyrrolidin-2-yl]methyl]carbamate (13 mg, 88%). The material was used as is for the next step. m/z: [M+H]⁺ = 829.2

### (b) 6-[3-Chloro-4-[2-chloro-3-[6-methoxy-5-[[[(2S)-5-oxopyrrolidin-2-yl]methylamino]methyl]-2-pyridyl]phenyl]-2-pyridyl]-2-[(6-hydroxy-2-azaspiro[3.3]heptan-2-yl)methyl]-3-methyl-pyrrolo[2,1-f][1,2,4]triazin-4-one

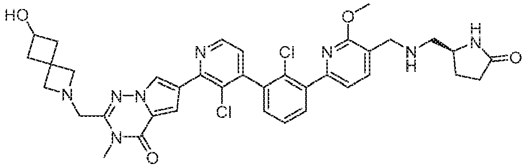

To a solution of *tert*-butyl N-[[6-[2-chloro-3-[3-chloro-2-[2-[(6-hydroxy-2-azaspiro [3.3]heptan-2-yl)methyl]-3-methyl-4-oxo-pyrrolo[2,1-f][1,2,4]triazin-6-yl]-4-pyridyl]phenyl]-2-methoxy-3-pyridyl]methyl]-N-[[(2S)-5-oxopyrrolidin-2-yl]methyl]carbamate ( 15 mg , 0.02 mmol) in methylene chloride (0.3 mL) was added TFA (69 uL, 0.90 mmol). The reaction was stirred at rt for 1 h. The solvent was evaporated and the residue was purified by reverse phase HPLC (acetonitrile:water) to afford 6-[3-chloro-4-[2-chloro-3-[6-methoxy-5-[[[(2S)-5- oxopyrrolidin-2-yl]methylamino] methyl]-2-pyridyl]phenyl]-2-pyridyl]-2-[(6-hydroxy-2-azaspiro[3.3]heptan-2-yl)methyl]-3-methyl-pyrrolo[2,1-f][1,2,4]triazin-4-one (5 mg, 37.9 %). m/z: [M+H]⁺ = 729.3. ¹H NMR (400 MHz, Methanol-*d*₄) δ 8.60 (d, 1H), 8.22 (s, 1H), 7.84 (d, 1H), 7.71 (d, 2H), 7.56 (t, 1H), 7.40 (d, 1H), 7.31 (dd, 2H), 4.22 (s, 2H), 4.14 (s, 3H), 4.07 (s, 3H), 3.98 (s, 2H), 3.90 (d, 4H), 3.49 (s, 3H), 3.03 (s, 2H), 2.60 (s, 3H), 2.36 (s, 2H), 2.14 (d, 2H), 1.87 (s, 1H).

### Example 42: 6-[3-Chloro-4-[2-chloro-3-[6-methoxy-5-[[[(2S)-5-oxopyrrolidin-2-yl]methylamino] methyl]-2-pyridyl]phenyl]-2-pyridyl]-2-[(3-hydroxy-3-methyl-azetidin-1-yl)methyl]-3-methyl-pyrrolo[2,1-f][1,2,4]triazin-4-one (110)

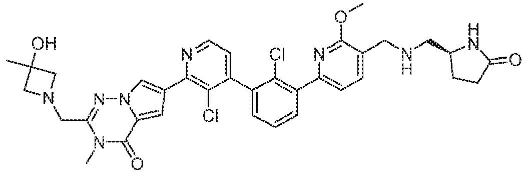

### (a) tert-Butyl-N-[[6-[2-chloro-3-[3-chloro-2-[2-[(3-hydroxy-3-methyl-azetidin-1-yl)methyl]-3-methyl-4-oxo-pyrrolo[2,1-f][1,2,4]triazin-6-yl]-4-pyridyl]phenyl]-2-methoxy-3-pyridyl]methyl]-N-[[(2S)-5-oxopyrrolidin-2-yl]methyl]carbamate

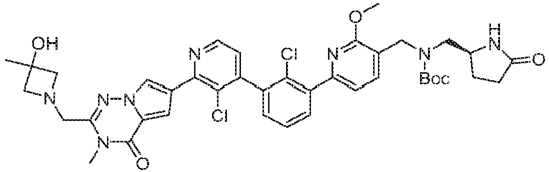

To a solution of *tert*-butyl N-[[6-[2-chloro-3-[3-chloro-2-[2-(hydroxymethyl)-3-methyl -4-oxo-pyrrolo[2,1-f][1,2,4]triazin-6-yl]-4-pyridyl]phenyl]-2-methoxy-3-pyridyl]methyl]-N-[[(2S)-5-oxopyrrolidin-2-yl]methyl]carbamate (23 mg, 0.03 mmol) in methylene chloride (0.4 mL) was added mesyl chloride (3 uL, 0.04 mmol) and triethylamine (13 uL, 0.09 mmol). The reaction was stirred at rt for 1h. The solvent was evaporated, and the residue was taken up in DMF (0.4 mL). To this mixture, potassium carbonate (12 mg, 0.09 mmol) and 3-methylazetidin-3-ol, HCl (11 mg, 0.09 mmol) were added and the reaction was stirred at rt 12 h. The solvent was evaporated, and the residue was suspended in water. The precipitate formed was collected to afford *tert*-butyl N-[[6-[2-chloro-3-[3-chloro-2-[2-[(3-hydroxy-3 -methyl-azetidin-1-yl)methyl]-3-methyl-4-oxo-pyrrolo[2,1-f][1,2,4]triazin-6-yl]-4-pyridyl] phenyl]-2-methoxy-3-pyridyl]methyl]-N-[[(2S)-5-oxopyrrolidin-2-yl]methyl]carbamate (13 mg, 51.7%). The material was sed as is without any purification. [M+H]⁺ = 804.2

### (b) 6-[3-Chloro-4-[2-chloro-3-[6-methoxy-5-[[[(2S)-5-oxopyrrolidin-2-yl]methylamino] methyl]-2-pyridyl]phenyl]-2-pyridyl]-2-[(3-hydroxy-3-methyl-azetidin-1-yl)methyl]-3-methyl-pyrrolo[2,1-f][1,2,4]triazin-4-one

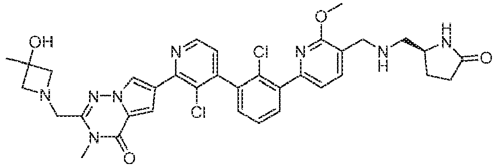

To a solution of *tert*-butyl N-[[6-[2-chloro-3-[3-chloro-2-[2-[(3-hydroxy-3-methyl - azetidin-1-yl)methyl]-3-methyl-4-oxo-pyrrolo[2,1-f][1,2,4]triazin-6-yl]-4-pyridyl]phenyl]-2-methoxy-3-pyridyl]methyl]-N-[[(2S)-5-oxopyrrolidin-2-yl]methyl]carbamate (13 mg, 0.02 mmol) in methylene chloride (0.4 mL) was added TFA (61 uL, 0.81 mmol). The reaction was stirred at rt for 30 min. The solvent was evaporated and the residue was purified by reverse phase HPLC (acetonitrile:water) to afford 6-[3-chloro-4-[2-chloro-3-[6-methoxy-5-[[[(2S)-5 - oxopyrrolidin-2-yl]methylamino]methyl]-2-pyridyl]phenyl]-2-pyridyl]-2-[(3-hydroxy-3-methyl-azetidin-1-yl)methyl]-3-methyl-pyrrolo[2,1-f][1,2,4]triazin-4-one (10 mg, 53.6%). m/z: [M+H]⁺ = 704.4. ¹H NMR (400 MHz, Methanol-*d*₄) δ 8.59 (d, 1H), 8.45 (s, 1H), 8.18 (d, 1H), 7.79 (d, 1H), 7.70 (dd, 1H), 7.66 (d, 1H), 7.54 (t, 1H), 7.38 (dd, 1H), 7.33 - 7.24 (m, 2H), 4.04 (s, 3H), 4.02 (d, 2H), 3.93 (q, 1H), 3.87 (s, 2H), 3.57 (s, 3H), 3.56 - 3.52 (m, 2H), 2.89 (h, 2H), 2.42 - 2.26 (m, 3H), 1.84 (m, 1H), 1.49 (s, 3H).

### Example 43: 6-(3-Chloro-4-(2-chloro-3-(4-chloro-6-methoxy-5-(((((S)-5-oxopyrrolidin-2-yl)methyl)amino)methyl)pyridin-2-yl)phenyl)pyridin-2-yl)-2-((((1r,3r)-3-hydroxy-3-methylcyclobutyl)amino)methyl)-3-methylpyrrolo[2,1-f][1,2,4]triazin-4(3H)-one (87)

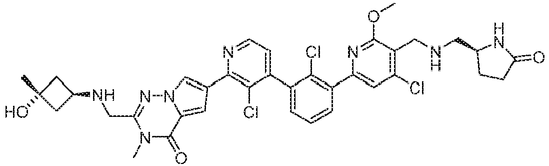

### (a) (S)-tert-Butyl((6-(3-(2-(2-(((tert-butyldimethylsilyl)oxy)methyl)-3-methyl-4-oxo-3,4-dihydropyrrolo[2,1-f][1,2,4]triazin-6-yl)-3-chloropyridin-4-yl)-2-chlorophenyl)-4-chloro-2-methoxypyridin-3-yl)methyl)((5-oxopyrrolidin-2-yl)methyl)carbamate

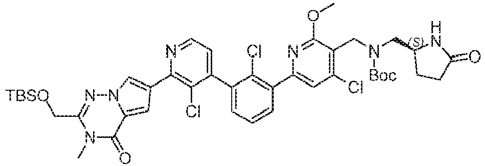

To a mixture of (S)-*tert*-butyl ((4-chloro-6-(2-chloro-3-(2,3-dichloropyridin-4-yl)phenyl) -2-methoxypyridin-3-yl)methyl)((5-oxopyrrolidin-2-yl)methyl)carbamate (200 mg, 319 umol) and 2-(((*tert*-butyldimethylsilyl)oxy)methyl)-3-methyl-6-(4,4,5,5-tetramethyl-1,3,2-dioxaborolan-2-yl)pyrrolo[2,1-f][1,2,4]triazin-4(3H)-one (201 mg, 479 umol) in THF/ H₂O (6:1, 7 mL) was added potassium carbonate (132 mg, 958 umol) and tetrakis(triphenylphosphine)platinum (36.9 mg, 31.9 umol). The mixture was stirred at 80 °C for 2 h under N₂. The mixture was filtered, and the filtrate was concentrated. The residue was purified by flash chromatography (SiO₂, 0-100 % EtOAc/petroleum ether) to afford (S)*-tert-*butyl((6-(3-(2-(2-(((*tert*-butyldimethylsilyl)oxy)methyl) -3-methyl-4-oxo-3,4-dihydropyrrolo[2,1-f][1,2,4]triazin-6-yl)-3-chloropyridin-4-yl)-2-chlorophenyl)-4-chloro-2-methoxypyridin-3-yl)methyl)((5-oxopyrrolidin-2-yl)methyl)carbamate (200 mg, 70% yield, 882 [M+H]⁺).

### (b) (S)-tert-Butyl((4-chloro-6-(2-chloro-3-(3-chloro-2-(2-(hydroxymethyl)-3-methyl-4-oxo-3,4-dihydropyrrolo[2,1-f][1,2,4]triazin-6-yl)pyridin-4-yl)phenyl)-2-methoxypyridin-3-yl)methyl)((5-oxopyrrolidin-2-yl)methyl)carbamate

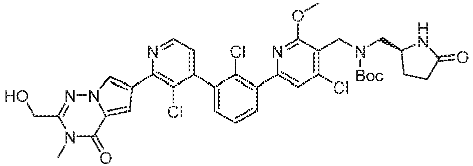

To a mixture of (S)-tert-butyl((6-(3-(2-(2-(((tert-butyldimethylsilyl)oxy)methyl) -3-methyl-4-oxo-3,4-dihydropyrrolo[2,1-f][1,2,4]triazin-6-yl)-3-chloropyridin-4-yl)-2-chlorophenyl)-4-chloro-2-methoxypyridin-3-yl)methyl)((5-oxopyrrolidin-2-yl)methyl) carbamate (180 mg, 204 umol) in THF (10 mL) was added tetrabutylammonium fluoride (1 mol/L in THF, 0.31 mL) The mixture was stirred at rt for 4 h under N₂. The mixture was combined with another batch at the 100 mg scale. The mixture was concentrated and the residue was purified by prep-TLC (SiO₂, EtOAc: MeOH = 9:1) to afford (S)-tert-butyl((4-chloro-6-(2-chloro-3-(3-chloro-2-(2-(hydroxymethyl)-3-methyl-4-oxo-3,4-dihydropyrrolo[2,1-f][1,2,4]triazin-6-yl)pyridin-4-yl)phenyl)-2-methoxypyridin-3-yl)methyl)((5-oxopyrrolidin-2-yl)methyl)carbamate (155 mg, 768 [M+H]⁺, observed).

### (c) (S)-tert-Butyl((4-chloro-6-(2-chloro-3-(3-chloro-2-(2-formyl-3-methyl-4-oxo-3,4-dihydropyrrolo[2,1-f][1,2,4]triazin-6-yl)pyridin-4-yl)phenyl)-2-methoxypyridin-3-yl)methyl)((5-oxopyrrolidin-2-yl)methyl)carbamate

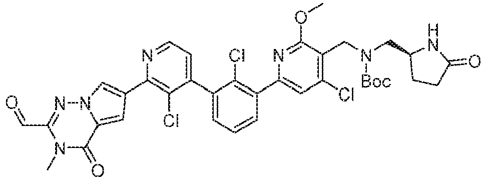

To a solution of (S)-*tert*-butyl((4-chloro-6-(2-chloro-3-(3-chloro-2-(2-(hydroxymethyl) -3-methyl-4-oxo-3,4-dihydropyrrolo[2,1-f][1,2,4]triazin-6-yl)pyridin-4-yl)phenyl)-2- methoxypyridin-3-yl)methyl)((5-oxopyrrolidin-2-yl)methyl)carbamate (150 mg, 195 umol) in methylene chloride (5 mL) was added Dess-Martin periodinane (165 mg, 390 umol). The mixture was stirred at rt for 30 min under a nitrogen atmosphere. The mixture was filtered, and the filtrate was concentrated. The residue was purified by flash chromatography (SiO₂, 0-100 % EtOAc/petroleum ether) to afford (S)-*tert*-butyl((4-chloro-6-(2-chloro-3-(3-chloro-2-(2-formyl-3-methyl-4-oxo-3,4-dihydropyrrolo[2,1-f][1,2,4]triazin-6-yl)pyridin-4-yl)phenyl)-2-methoxypyridin-3-yl)methyl)((5-oxopyrrolidin-2-yl)methyl)carbamate (85 mg, 57% yield, 766 [M+H]⁺, observed).

### (d) tert-Butyl((4-chloro-6-(2-chloro-3-(3-chloro-2-(2-((((1r,3r)-3-hydroxy-3-methylcyclobutyl)amino)methyl)-3-methyl-4-oxo-3,4-dihydropyrrolo[2,1-f][1,2,4]triazin-6-yl)pyridin-4-yl)phenyl)-2-methoxypyridin-3-yl)methyl)(((S)-5-oxopyrrolidin-2-yl)methyl)carbamate

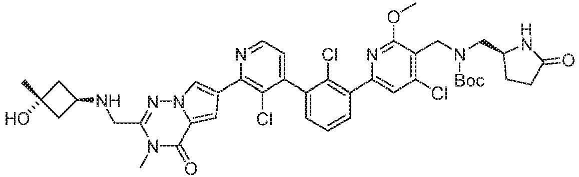

To a mixture of (S)-*tert*-butyl((4-chloro-6-(2-chloro-3-(3-chloro-2-(2-formyl- 3-methyl-4-oxo-3,4-dihydropyrrolo[2,1-f][1,2,4]triazin-6-yl)pyridin-4-yl)phenyl)-2-methoxypyridin-3-yl)methyl)((5-oxopyrrolidin-2-yl)methyl)carbamate (80 mg, 104 umol) and (1r,3r)-3-amino-1-methylcyclobutanol, HCl (28.7 mg, 209 umol) in MeOH (5 mL) was added sodium acetate (42.8 mg, 521 umol). After stirring the mixture for 5 h, sodium cyanoborohydride (13.1 mg, 209 umol) was added. The mixture was stirred at rt for 0.5 h under N₂. The mixture was concentrated, and the residue was purified by prep-TLC (SiO₂, EtOAc: MeOH = 4:1) to afford *tert*-butyl((4-chloro-6-(2-chloro-3- (3-chloro-2- (2-((((1r,3r)-3-hydroxy-3-methylcyclobutyl)amino)methyl)-3-methyl-4-oxo-3,4-dihydropyrrolo[2,1-f][1,2,4]triazin-6-yl)pyridin-4-yl)phenyl)-2-methoxypyridin-3-yl)methyl)(((S)-5-oxopyrrolidin-2-yl)methyl)carbamate (75 mg, 57% yield, 851 [M+H]⁺).

### (e) 6-(3-Chloro-4-(2-chloro-3-(4-chloro-6-methoxy-5-(((((S)-5-oxopyrrolidin-2-yl)methyl)amino)methyl)pyridin-2-yl)phenyl)pyridin-2-yl)-2-((((1r,3r)-3-hydroxy-3-methylcyclobutyl)amino)methyl)-3-methylpyrrolo[2,1-f][1,2,4]triazin-4(3H)-one

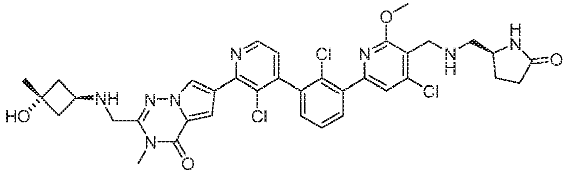

To a solution of *tert*-butyl((4-chloro-6-(2-chloro-3-(3-chloro-2-(2-((((1r,3r)-3-hydroxy -3-methylcyclobutyl)amino)methyl)-3-methyl-4-oxo-3,4-dihydropyrrolo[2,1-f][1,2,4]triazin-6-yl)pyridin-4-yl)phenyl)-2-methoxypyridin-3-yl)methyl)(((S)-5-oxopyrrolidin-2-yl)methyl)carbamate (70 mg, 0.08 mmol) in methylene chloride (3 mL) was added TFA (3 mL, 40.5 mmol). The mixture was stirred at rt for 30 min under a nitrogen atmosphere. The mixture was concentrated and the residue was purified by reverse phase HPLC to afford 6-(3-chloro-4-(2-chloro-3- (4-chloro-6 -methoxy-5-(((((S)-5-oxopyrrolidin-2-yl)methyl)amino)methyl)pyridin-2-yl)phenyl)pyridin-2-yl)-2-((((1r,3r)-3-hydroxy-3-methylcyclobutyl)amino)methyl)-3-methylpyrrolo[2,1-f][1,2,4]triazin-4(3H)-one (15 mg, 23% yield, m/z: 690 [M+H]⁺ observed) as a white solid. ¹H NMR (400 MHz, DMSO-*d*₆): δ 8.66 (d, 1H), 8.21 (d, 1H), 7.73 (dd, 1H), 7.63 (s, 1H), 7.59 (t, 1H), 7.53 (d, 1H), 7.49 (dd, 1H), 7.39 (s, 1H), 7.36 (d, 1H), 4.67 (s, 1H), 3.94 (s, 3H), 3.87 (s, 2H), 3.71 (s, 2H), 3.61-3.58 (m, 1H), 3.52 (s, 3H), 3.40-3.36 (m, 1H), 2.65 (d, 2H), 2.13-2.04 (m, 5H), 1.71-1.64 (m, 3H), 1.23 (s, 3H).

### Example 44: 5-(3-Chloro-4-(2-chloro-3-(6-methoxy-5-(((((S)-5-oxopyrrolidin-2-yl)methyl)amino)methyl)pyridin-2-yl)phenyl)pyridin-2-yl)-2-(2-((((S)-5-oxopyrrolidin-2-yl)methyl)amino)ethyl)isoindolin-1-one (66)

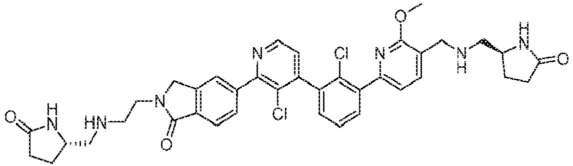

### (a) 5-Bromo-2-(2,2-dimethoxyethyl)isoindolin-1-one

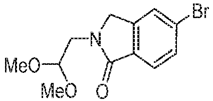

To a mixture of methyl 4-bromo-2-(bromomethyl)benzoate (3 g, 9.74 mmol) in MeOH (60 mL) was added 2,2-dimethoxyethanamine (5.31 mL, 48.7 mmol) in one portion under a nitrogen atmosphere. The mixture was stirred at room temperature for 2 h. The mixture was concentrated. To the residue was added water (30 mL) and saturated aqueous brine solution (30 mL). The mixture was extracted with ethyl acetate/THF mixture (1:1, 100 mL). The organic phase was dried with anhydrous sodium sulfate, filtered and concentrated. The residue was purified by flash chromatography (SiO₂, 0-50% EtOAc/petroleum ether) to afford 5-bromo-2-(2,2-dimethoxyethyl)isoindolin-1-one as a white solid (2.7 g, 92%).

### (b) 2-(5-Bromo-1-oxoisoindolin-2-yl)acetaldehyde

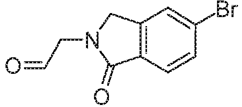

To a mixture of 5-bromo-2-(2,2-dimethoxyethyl)isoindolin-1-one (1.3 g, 4.33 mmol) in methylene chloride (30 mL) was added TFA (6.50 mL, 87.8 mmol) and H₂O (1 mL) in one portion. The mixture was stirred at room temperature for 2 h. The mixture was concentrated under vacuum to afford 2-(5-bromo-1-oxoisoindolin-2-yl)acetaldehyde as trifluoroacetic acid salt as a yellow oil (1.8 g) which was used into the next step without further purification.

### (c) (S)-tert-Butyl (2-(5-bromo-1-oxoisoindolin-2-yl)ethyl)((5-oxopyrrolidin -2-yl)methyl)carbamate

To a mixture of 2-(5-bromo-1-oxoisoindolin-2-yl)acetaldehyde (1.8 g, 7.08 mmol) in methylene chloride / MeOH (1/1, 40 mL) was added TEA (9.86 mL, 70.8 mmol) followed by (S)-5-(aminomethyl)pyrrolidin-2-one, HCl (1.07 g, 7.08 mmol) and sodium cyanoborohydride (1.34 g, 21.2 mmol) . The mixture was stirred at room temperature for 12 h. To this mixture of was added di-tert-butyldicarbonate (3.10 g, 14.2 mmol) and stirred for 2 h at room temperature. The mixture was concentrated under vacuum and diluted with H₂O (30 mL), extracted with THF/EtOAc(1/3, 100 mL) and dried over anhydrous sodium sulfate and filtered. The filtrate was concentrated, and the residue was purified by flash chromatography (SiO₂, 0-100% EtOAc/petroleum ether to 0- 20% MeOH/EtOAc) to afford (S)-*tert*-butyl (2-(5-bromo-1-oxoisoindolin-2-yl)ethyl)((5-oxopyrrolidin-2-yl)methyl) carbamate s a light-yellow oil (1.5 g, 44%).

### (d) (S)-tert-Butyl (2-(1-oxo-5-(4,4,5,5-tetramethyl-1,3,2-dioxaborolan-2-yl) isoindolin-2-yl)ethyl)((5-oxopyrrolidin-2-yl)methyl)carbamate

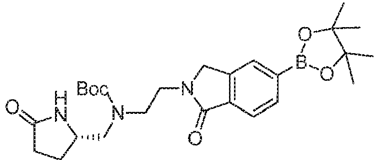

To a mixture of (S)-*tert*-butyl(2-(5-bromo-1-oxoisoindolin-2-yl)ethyl) ((5-oxopyrrolidin -2-yl)methyl)carbamate (1 g, 2.21 mmol) and 4,4,4',4',5,5,5',5'-octamethyl-2,2'-bi(1,3,2-dio xaborolane) (1.68 g, 6.63 mmol) in dioxane (20 mL) was added potassium acetate (434 mg, 4.42 mmol), followed by 1,1'-bis(diphenylphosphino)ferrocene-palladium(II) dichloride dichloromethane complex (181 mg, 0.22 mmol). The reaction was stirred for 3 h at 130 °C. After cooling, the mixture was filtered and washed with EtOAc (100 ml). The filtrate was concentrated under reduced pressure. The residue was purified by flash chromatography (SiO₂, 0-100% EtOAc/petroleum ether, then 0- 20% MeOH/EtOAc) to afford (S)-*tert*-butyl (2-(1-oxo-5-(4,4,5,5-tetramethyl-1,3,2-dioxaborolan-2-yl)isoindolin-2-yl) ethyl)((5-oxopyrrolidin-2-yl)methyl)carbamate as a orange solid (410 mg, 37% yield). ¹H NMR (400 MHz, Methanol-*d*₄): δ 7.93 (s, 1H), 7.90-7.84 (m, 1H), 7.81-7.73 (m, 1H), 4.61 (d, 2 H), 3.98 (s, 1 H), 3.82 (s, 2 H), 3.68-3.57 (m, 2 H), 3.37 (s, 2 H), 2.40-2.24 (m, 3 H), 1.84 (br, 1 H), 1.39 (s, 12 H), 1.22 (s, 9 H).

### (e) 5-(3-Chloro-4-(2-chloro-3-(6-methoxy-5-(((((S)-5-oxopyrrolidin-2-yl)methyl)amino)methyl)pyridin-2-yl)phenyl)pyridin-2-yl)-2-(2-((((S)-5-oxopyrrolidin-2-yl)methyl)amino)ethyl)isoindolin-1-one

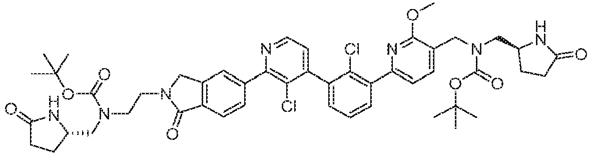

To a mixture of (S)-*tert*-butyl (2-(1-oxo-5-(4,4,5,5-tetramethyl-1,3,2-dioxaborolan-2-yl) isoindolin-2-yl)ethyl)((5-oxopyrrolidin-2-yl)methyl)carbamate (10 mg, 20 umol) and (S)-*tert*-butyl ((6-(2-chloro-3-(2,3-dichloropyridin-4-yl)phenyl)-2-methoxypyridin-3-yl) methyl)((5-oxopyrrolidin-2-yl)methyl)carbamate (12 mg, 20 umol) in dioxane/H₂O (100/1, 2.02 mL) was added potassium phosphate (4 mg, 20 umol) and di*tert-*butyl(cyclopentyl)phosphane;dichloropalladium;iron (1.3 mg, 2.0 umol). The reaction was stirred for 3 h at 110 °C. After cooling, the mixture combined with another batch at 50 mg scale was added H₂O (10 mL) and extracted with EtOAc (20 mL). The organic layer was concentrated under vacuum. The residue was purified by prep-TLC (20% MeOH/EtOAc) to afford *tert*-butyl N-[[6-[3-[2-[2-[2-[tert-butoxycarbonyl -[[(2S)-5-oxopyrrolidin-2-yl]methyl]amino]ethyl]-1-oxo-isoindolin-5-yl]-3-chloro-4-pyridyl]-2-chloro-phenyl]-2-methoxy-3-pyridyl]methyl]-N-[[(2S)-5-oxopyrrolidin-2-yl]methyl] carbamate as a white solid (32 mg, 28%).

### (f) 5-(3-Chloro-4-(2-chloro-3-(6-methoxy-5-(((((S)-5-oxopyrrolidin-2-yl)methyl)amino)methyl)pyridin-2-yl)phenyl)pyridin-2-yl)-2-(2-((((S)-5-oxopyrrolidin-2-yl)methyl)amino)ethyl)isoindolin-1-one

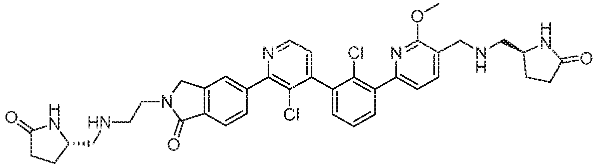

To a mixture of *tert*-butyl N-[[6-[3-[2-[2-[2-[*tert*-butoxycarbonyl-[[(2S)-5-oxopyrrolidin -2-yl]methyl]amino]ethyl]-1-oxo-isoindolin-5-yl]-3-chloro-4-pyridyl]-2-chloro-phenyl]-2-methoxy-3-pyridyl]methyl]-N-[[(2S)-5-oxopyrrolidin-2-yl]methyl]carbamate (50 mg, 54 umol) in methylene chloride (0.3 mL) was added TFA (3.00 mL, 40.5 mmol) and stirred for 0.5 h at room temperature. The reaction was concentrated under reduced pressure and the residue was purified by reverse phase HPLC to afford 5-(3-chloro-4-(2-chloro-3-(6-methoxy-5- (((((S)-5-oxopy rrolidin-2-yl)methyl)amino)methyl)pyridin-2-yl)phenyl)pyridin-2-yl)-2-(2-((((S)-5-oxopyrrolidin-2-yl)methyl)amino)ethyl)isoindolin-1-one as a white solid. m/z: 728 [M+H]⁺. ¹H NMR (400 MHz, DMSO-*d*₆): δ 8.75 (d, 1 H), 8.16 (s, 2 H), 7.90 (s, 1 H), 7.85 (d, 1 H), 7.80 (s, 2 H), 7.75-7.73 (m, 1 H), 7.68 (d, 2 H), 7.61 (t, 1 H), 7.57 (d, 1 H), 7.52 (dd, 1 H), 7.29 (d, 1 H), 4.62 (s, 2 H), 3.93 (s, 3 H), 3.73 (d, 2 H), 3.69-3.57 (m, 6 H), 2.86-2.83 (m, 3 H), 2.15-2.05 (m, 7 H), 1.72-1.66 (m, 2 H).

### Example 45: (S)-5-((((6-(3-(2-(3-(((1-acetylpiperidin-4-yl)amino)-methyl)-1-methyl-1H-pyrrolo[3,2-b]-pyridin-6-yl)-3-chloropyridin-4-yl)-2-chlorophenyl)-2-methoxypyridin-3-yl)methyl)amino)methyl)pyrrolidin-2-one (21)

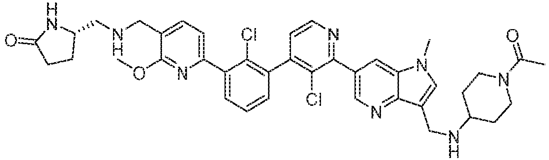

### (a) (S)-5-((((6-(3-bromo-2-chlorophenyl)-2-methoxypyridin-3-yl)methyl)amino)methyl)pyrrolidin-2-one

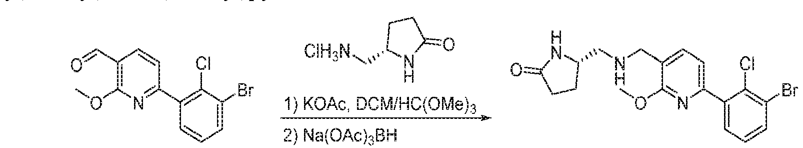

A mixture of 6-(3-bromo-2-chlorophenyl)-2-methoxynicotinaldehyde (20 g, 61 mmol), (5S)-5-(aminomethyl)-pyrrolidin-2-one hydrochloride (14 g, 92 mmol), and sodium acetate (13 g, 153 mmol) in methylene chloride/trimethoxymethane mixture (5:1 *(v*/*v), 300* mL) was stirred at rt for 4 hours. Sodium triacetoxyborohydride (26 g, 122 mmol) was added portion-wise and the reaction was stirred at room temperature for 2 h under N₂. Reaction was subsequently diluted with water (500 mL) and extracted with methylene chloride (3 x 300 mL). The combined organics were further washed with brine (300 mL), dried over sodium sulfate, filtered and concentrated under reduced pressure to afford (S)-5-((((6-(3-bromo-2-chlorophenyl)-2-methoxypyridin-3-yl)methyl-)-amino)-methyl)-pyrrolidin-2-one (26 g, crude, *m*/*z*: 426 [M+H]⁺ observed) as a clear oil. The crude product was used directly without further purification.

### (b) (S)-tert-Butyl ((6-(3-bromo-2-chlorophenyl)-2-methoxypyridin-3-yl)methyl)((5-oxopyrrolidin-2-yl)methyl)carbamate

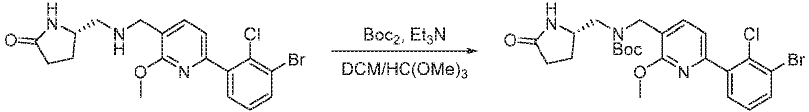

To a solution of (S)-5-((((6-(3-bromo-2-chlorophenyl)-2-methoxypyridin-3-yl)methyl)amino)methyl)pyrrolidin-2-one in DCM/trimethoxymethane mixture (5:1, 300 mL) was added di-tert-butyl dicarbonate (42 mL, 183 mmol) and triethylamine (30 mL, 214 mmol) in DCM (80 mL). The mixture was stirred at rt for 1 hour under N₂. Reaction mixture was filtered to give the filtrate. The filtrate was concentrated under reduced pressure and the crude residue was purified by normal phase SiO₂ chromatography (0-20 % ethyl acetate/petroleum ether) to afford (S)-*tert*-butyl ((6-(3-bromo-2-chlorophenyl)-2-methoxypyridin-3-yl)methyl)((5-oxopyrrolidin-2-yl)methyl)carbamate as a yellow solid (20 g, 62 % yield, *m*/*z*: 526 [M+H]⁺ observed).

### (c) (S)-tert-Butyl ((6-(2-chloro-3-(2,3-dichloropyridin-4-yl)phenyl)-2-methoxypyridin-3-yl)methyl)((5-oxopyrrolidin-2-yl)methyl)carbamate

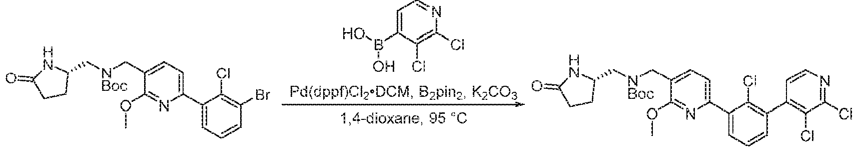

A mixture of (S)-tert-butyl ((6-(3-bromo-2-chlorophenyl)-2-methoxypyridin-3-yl)methyl)((5-oxopyrrolidin-2-yl)methyl)carbamate (12 g, 23 mmol), 1,1'-bis(diphenylphosphino)ferrocene-palladium(II) dichloride dichloromethane complex (0.93 g, 1.14 mmol) and potassium carbonate (9.5 g, 68 mmol) in 1,4-dioxane/H₂O mixture (6:1, 140 mL) was degassed and purged with N₂ for 3 times, then (2,3-dichloro-4-pyridyl)boronic acid (3.5 g, 18 mmol) in 1,4-dioxane (150 mL) was added dropwise to the mixture over 2 hours at 95 °C. The mixture was further stirred at 95 °C for 1 hour under N₂. Reaction was diluted with water (250 mL) and extracted with EtOAc (2 x 200 mL). The combined organics were dried with anhydrous sodium sulfate, filtered, and concentrated under reduced pressure. The crude residue was purified by normal phase SiO₂ chromatography (0-15 % EtOAc/petroleum ether) to afford (S)-tert-butyl ((6-(2-chloro-3-(2,3-dichloropyridin-4-yl)phenyl)-2-methoxypyridin-3-yl)methyl)((5-oxopyrrolidin-2-yl)methyl)carbamate as a yellow solid (7 g, *m*/*z*: 591 [M+H]⁺ observed). ¹H NMR (400 MHz, DMSO-d₆): δ. 8.51 (d, 1H), 7.77-7.73 (m, 2H), 7.65-7.56 (m, 2H), 7.50-7.44 (m, 2H), 7.31-7.29 (m, 1H), 4.47-4.35 (m, 2H), 3.94 (s, 3H), 3.78-3.76 (m, 1H), 3.33-3.27 (m, 2H), 2.22-2.04 (m, 3H), 1.73-1.72 (m, 1H), 1.44-1.30 (m, 9H).

### (d) tert-Butyl (S)-((6-(2-chloro-3-(3-chloro-2-(3-formyl-1-methyl-1H-pyrrolo[3,2-b]pyridin-6-yl)pyridin-4-yl)phenyl)-2-methoxypyridin-3-yl)methyl)((5-oxopyrrolidin-2-yl)methyl)carbamate

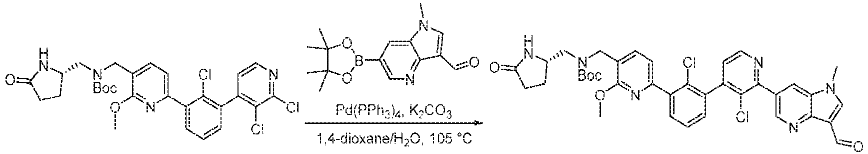

Pd(PPh₃)₄ (31 mg, 0.03 mmol), potassium carbonate (56 mg, 0.41 mmol), tert-butyl N-[[6-[2-chloro-3-(2,3-dichloro-4-pyridyl)phenyl]-2-methoxy-3-pyridyl]methyl]-N-[[(2S)-5-oxopyrrolidin-2-yl]methyl]carbamate (80 mg, 0.14 mmol), and 1-methyl-6-(4,4,5,5-tetramethyl-1,3,2-dioxaborolan-2-yl)pyrrolo[3,2-b]pyridine-3-carbaldehyde (58 mg, 0.20 mmol) were suspended in 1,4-dioxane/water (4:1 (*v*/*v*)*,* 2 mL), then the solution was heated at 105 °C for 20 minutes. Reaction was cooled to room temperature, diluted with 4 mL water, and extracted with EtOAc (3 x 3 mL). The combined organics were concentrated under reduced pressure and crude sample was purified by normal phase SiO₂ chromatography (0-6 % MeOH/DCM) to afford *tert*-butyl N-[[6-[2-chloro-3-[3-chloro-2-(3-formyl-1-methyl-pyrrolo[3,2-b]pyridin-6-yl)-4-pyridyl]phenyl]-2-methoxy-3-pyridyl]methyl]-N-[[(2S)-5-oxopyrrolidin-2-yl]methyl]carbamate (37 mg, 39 % yield) as a yellow foam. MS: *m*/*z* found 715.3, 717 [M+H] ⁺.

### (e) tert-Butyl N-[[6-[3-[2-[3-[[(1-acetyl-4-piperidyl)amino]methyl]-1-methyl-pyrrolo[3,2-b]pyridin-6-yl]-3-chloro-4-pyridyl]-2-chloro-phenyl]-2-methoxy-3-pyridyl]methyl]-N-[[(2S)-5-oxopyrrolidin-2-yl]methyl] carbamate

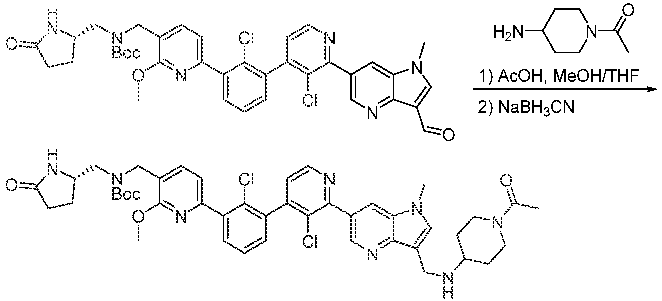

*tert*-Butyl N-[[6-[2-chloro-3-[3-chloro-2-(3-formyl-1-methyl-pyrrolo[3,2-b]pyridin-6-yl)-4-pyridyl]phenyl]-2-methoxy-3-pyridyl]methyl]-N-[[(2S)-5-oxopyrrolidin-2-yl]methyl]carbamate (18 mg, 0.03 mmol), 1-(4-amino-1-piperidyl)ethanone (5 mg, 0.04 mmol), and acetic acid (2 mg, 0.03 mmol) were dissolved in MeOH/THF (1:1, 1 mL), then the solution was stirred at 25 °C for 2 hours. NaBH₃CN (3 mg, 0.05 mmol) was added and the resulting mixture was stirred at 25 °C for 1 hour. Reaction was subsequently diluted with water (2 mL) and extracted with EtOAc (3 x 2 mL). The combined organics were further washed with brine (3 mL), dried over sodium sulfate, filtered and concentrated under reduced pressure to afford *tert*-butyl N-[[6-[3-[2-[3-[[(1-acetyl-4-piperidyl)amino]methyl]-1-methyl-pyrrolo[3,2-b]pyridin-6-yl]-3-chloro-4-pyridyl]-2-chloro-phenyl]-2-methoxy-3-pyridyl]methyl]-N-[[(2S)-5-oxopyrrolidin-2-yl]methyl]carbamate (21 mg, crude) as a yellow oil. MS: *m*/*z* found 841.4, 843 [M+H] ⁺. Material was used for the next step without further purification.

### (f) (S)-5-((((6-(3-(2-(3-(((1-Acetylpiperidin-4-yl)amino)methyl)-1-methyl-1H-pyrrolo[3,2-b]pyridin-6-yl)-3-chloropyridin-4-yl)-2-chlorophenyl)-2-methoxypyridin-3-yl)methyl)amino)methyl)pyrrolidin-2-one

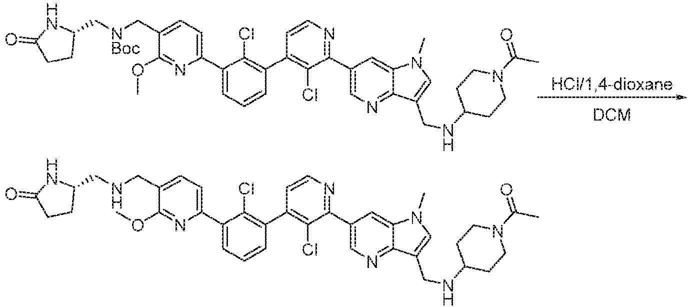

*tert-Butyl* N-[[6-[3-[2-[3-[[(1-acetyl-4-piperidyl)amino]methyl]-1-methyl-pyrrolo[3,2-b]pyridin-6-yl]-3-chloro-4-pyridyl]-2-chloro-phenyl]-2-methoxy-3-pyridyl]methyl]-N-[[(2S)-5-oxopyrrolidin-2-yl]methyl]carbamate (21 mg, 0.02 mmol, crude) was dissolved in 1 mL DCM and 4M HCl solution in 1,4-dioxane (25 µL, 0.10 mmol) was added. Reaction was stirred at rt for 20 minutes and solvent was removed under reduced pressure. Crude sample was purified by reverse phase HPLC to afford (5S)-5-[[[6-[3-[2-[3-[[(1-acetyl-4-piperidyl)amino]methyl]-1-methyl-pyrrolo[3,2-b]pyridin-6-yl]-3-chloro-4-pyridyl]-2-chloro-phenyl]-2-methoxy-3-pyridyl]methylamino]methyl]pyrrolidin-2-one (12 mg, 65 % yield) as a white solid (formic acid salt). MS: *m*/*z* found 741.4, 743.3 [M+H] ⁺, LC retention time = 2.02 min. (Method C). ¹H NMR (400 MHz, Methanol-*d*₄) δ 8.77 (d, 1H), 8.71 (d, 1H), 8.53 (s, 1H), 8.30 (d, 1H), 7.80 (s, 1H), 7.75 (d, 1H), 7.72 (dd, 1H), 7.56 (t, 1H), 7.49 (d, 1H), 7.43 (dd, 1H), 7.26 (d, 1H), 4.61 (d, 1H), 4.44 (s, 2H), 4.02 (s, 4H), 3.95 (s, 3H), 3.85 (d, 3H), 3.33 (s, 1H), 3.17 (t, 1H), 2.77 - 2.63 (m, 3H), 2.37 - 2.19 (m, 5H), 2.12 (s, 3H), 1.87 - 1.76 (m, 1H), 1.63 - 1.50 (m, 1H), 1.52 - 1.39 (m, 1H).

### Example 46: (S)-5-((((6-(2-Chloro-3-(3-chloro-2-(1-methyl-3-(((((S)-oxetan-2-yl)methyl)amino)methyl)-1H-pyrrolo[3,2-b]pyridin-6-yl)pyridin-4-yl)phenyl)-2-methoxypyridin-3-yl)-methyl)-amino)-methyl)-pyrrolidin-2-one (22)

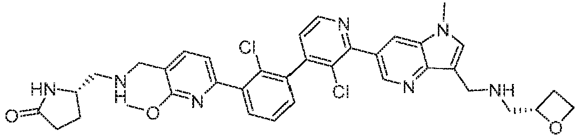

(S)-5-((((6-(2-Chloro-3-(3-chloro-2-(1-methyl-3-(((((S)-oxetan-2-yl)methyl)amino)methyl)-1H-pyrrolo [3,2-b]pyridin-6-yl)pyridin-4-yl)phenyl)-2-methoxypyridin-3-yl)-methyl)-amino)-methyl)-pyrrolidin-2-one was prepared in the same manner as described for compound **21,** utilizing intermediate tert-butyl (S)-((6-(2-chloro-3-(3-chloro-2-(3-formyl-1-methyl-1H-pyrrolo[3,2-b]pyridin-6-yl)pyridin-4-yl)phenyl)-2-methoxypyridin-3-yl)methyl)((5-oxopyrrolidin-2-yl)methyl)carbamate. (S)-oxetan-2-ylmethanamine was used in place of 1-(4-aminopiperidin-1-yl)ethan-1-one. Product was obtained as a yellow solid. MS: *m*/*z* found 686.3, 688 [M+H]⁺, LC retention time = 2.01 min. (Method C). ¹H NMR (400 MHz, Methanol-*d*₄) δ 8.80 (d, 1H), 8.71 (d, 1H), 8.50 (s, 1H), 8.33 (d, 1H), 7.90 (s, 1H), 7.76 (d, 1H), 7.72 (dd, 1H), 7.56 (t, 1H), 7.49 (d, 1H), 7.44 (dd, 1H), 7.27 (d, 1H), 4.64 (s, 2H), 4.53 (s, 1H), 4.02 (s, 3H), 3.97 (s, 3H), 3.87 (dd, 3H), 3.62 (q, 1H), 3.57 - 3.42 (m, 2H), 3.38 (d, 1H), 2.84 - 2.68 (m, 2H), 2.39 - 2.15 (m, 4H), 2.04 (s, 1H), 1.82 (m, 1H).

### Example 47: (S)-5-((((6-(2-Chloro-3-(3-chloro-5'-methoxy-6'-(((((S)-5-oxopyrrolidin-2-yl)-methyl)-amino)-methyl)-[2,3'-bipyridin]-4-yl)phenyl)-2-methoxypyridin-3-yl)methyl)amino)methyl)pyrrolidin-2-one (23)

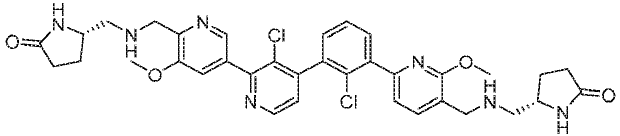

(S)-5-((((6-(2-Chloro-3-(3-chloro-5'-methoxy-6'-(((((S)-5-oxopyrrolidin-2-yl)-methyl)-amino)-methyl)-[2,3'-bipyridin]-4-yl)phenyl)-2-methoxypyridin-3-yl)methyl)amino)methyl)pyrrolidin-2-one was prepared in the same manner as described for compound **21,** utilizing intermediate (S)-*tert*-butyl ((6-(2-chloro-3-(2,3-dichloropyridin-4-yl)phenyl)-2-methoxypyridin-3-yl)methyl)((5-oxopyrrolidin-2-yl)methyl)carbamate. 3-methoxy-5-(4,4,5,5-tetramethyl-1,3,2-dioxaborolan-2-yl)picolinaldehyde was used in place of 1-methyl-6-(4,4,5,5-tetramethyl-1,3,2-dioxaborolan-2-yl)pyrrolo[3,2-b]pyridine-3-carbaldehyde. Product was obtained as a light yellow solid (formic acid salt). MS: *m*/*z* found 690.3, 693 [M+H]⁺, LC retention time = 1.98 min. (Method C). ¹H NMR (400 MHz, Methanol-*d*₄) δ 8.70 (dd, 1H), 8.52 (d, 1H), 8.42 (s, 2H), 7.80 (d, 2H), 7.75 - 7.67 (m, 1H), 7.60 - 7.51 (m, 1H), 7.50 (dd, 1H), 7.43 (d, 1H), 7.32 - 7.19 (m, 1H), 4.32 (d, 2H), 4.04 (d, 3H), 4.03 - 3.95 (m, 6H), 3.91 (s, 1H), 3.17 - 2.99 (m, 2H), 2.99 - 2.74 (m, 2H), 2.34 (t, 6H), 2.01 - 1.75 (m, 2H).

### Example 48: (S)-5-((((6-(2-chloro-3-(3-chloro-2-(4-((S)-2-hydroxypropyl)-2,3,4,5-tetrahydrobenzo[f][1,4]-oxazepin-8-yl)pyridin-4-yl)phenyl)-2-methoxypyridin-3-yl)methyl)amino)methyl)pyrrolidin-2-one (25)

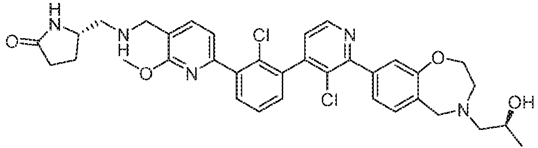

### (a) (S)-1-(8-(4,4,5,5-Tetramethyl-1,3,2-dioxaborolan-2-yl)-2,3-dihydrobenzo[f][1,4]oxazepin-4(5H)-yl)propan-2-ol

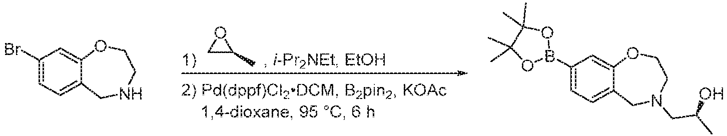

(2S)-2-Methyloxirane (127 mg, 2.19 mmol), 8-bromo-2,3,4,5-tetrahydro-1,4-benzoxazepine (50 mg, 0.22 mmol), and DIEA (38 µL, 0.22 mmol) were suspended in 2 mL EtOH and the mixture was stirred at 85 °C for 1 h. Reaction was concentrated and the residue was dissolved in 2 mL EtOAc. The organic solution was washed with water (2 x 2 mL), brine (2 x 2 mL), dried over sodium sulfate, filtered, and concentrated to afford (46 mg, crude) as a clear oil. MS: *m*/*z* found 286, 288 [M+H] ⁺. Material was used for the next step without further purification.

(2S)-1-(8-Bromo-3,5-dihydro-2H-1,4-benzoxazepin-4-yl)propan-2-ol (46 mg, 0.16 mmol, crude), 4,4,5,5-tetramethyl-2-(4,4,5,5-tetramethyl-1,3,2-dioxaborolan-2-yl)-1,3,2-dioxaborolane (53 mg, 0.21 mmol), 1,1'-bis(diphenylphosphino)-ferrocene-palladium(II) dichloride dichloromethane complex (13 mg, 0.02 mmol) , and potassium acetate (47 mg, 0.48 mmol) were suspended in 2 mL 1,4-dioxane in a sealed tube. Nitrogen gas was bubbled through the reaction solution for 2 minutes, then the vessel was sealed and heated at 95 °C for 6 hours. Reaction was cooled to rt and the mixture was filtered through celite. The filtrate was concentrated under reduced pressure and the crude oil was purified by normal phase SiO₂ chromatography (0-5 % MeOH/DCM) to afford (2S)-1-[8-(4,4,5,5-tetramethyl-1,3,2-dioxaborolan-2-yl)-3,5-dihydro-2H-1,4-benzoxazepin-4-yl]propan-2-ol (25 mg, 47 % yield) as a brown oil. MS: *m*/*z* found 334 [M+H] ⁺.

### (b) tert-Butyl ((6-(2-chloro-3-(3-chloro-2-(4-((S)-2-hydroxypropyl)-2,3,4,5-tetrahydrobenzo[f][1,4]oxazepin-8-yl)pyridin-4-yl)phenyl)-2-methoxypyridin-3-yl)methyl)(((S)-5-oxopyrrolidin-2-yl)methyl)carbamate

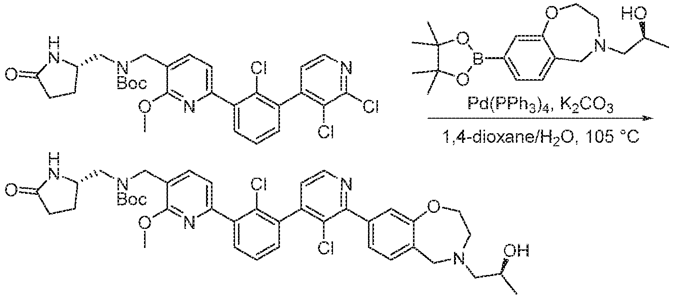

Pd(PPh₃)₄ (17 mg, 0.02 mmol), potassium carbonate (31 mg, 0.23 mmol), tert-butyl N-[[6-[2-chloro-3-(2,3-dichloro-4-pyridyl)phenyl]-2-methoxy-3-pyridyl]methyl]-N-[[(2S)-5-oxopyrrolidin-2-yl]methyl]carbamate (53 mg, 0.09 mmol), and (2S)-1-[8-(4,4,5,5-tetramethyl-1,3,2-dioxaborolan-2-yl)-3,5-dihydro-2H-1,4-benzoxazepin-4-yl]propan-2-ol (25 mg, 0.08 mmol) were suspended in 1,4-dioxane/water (4:1, 1 mL), then the solution was heated at 105 °C for 20 minutes. Reaction was cooled to rt, diluted with 3 mL water, and extracted with EtOAc (3 x 2 mL). The combined organics were concentrated under reduced pressure and crude sample was purified by flash chromatography (SiO₂, 0-6 % MeOH/DCM) to afford *tert*-butyl N-[[6-[2-chloro-3-[3-chloro-2-[4-[(2S)-2-hydroxypropyl]-3,5-dihydro-2H-1,4-benzoxazepin-8-yl]-4-pyridyl]phenyl]-2-methoxy-3-pyridyl]methyl]-N-[[(2S)-5-oxopyrrolidin-2-yl]methyl]carbamate (15 mg, 27 % yield) as a white foam. MS: *m*/*z* found 762.3, 764 [M+H] ⁺.

### (c) (S)-5-((((6-(2-Chloro-3-(3-chloro-2-(4-((S)-2-hydroxypropyl)-2,3,4,5-tetrahydrobenzo[f][1,4]-oxazepin-8-yl)pyridin-4-yl)phenyl)-2-methoxypyridin-3-yl)methyl)amino)methyl)pyrrolidin-2-one

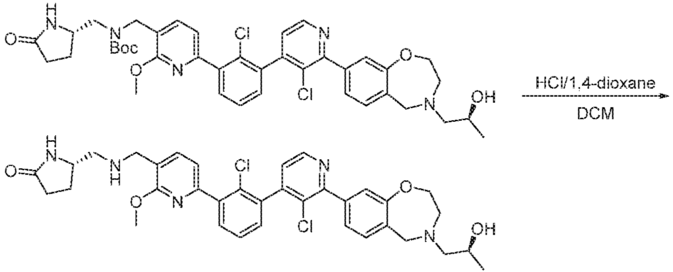

*tert*-Butyl N-[[6-[2-chloro-3-[3-chloro-2-[4-[(2S)-2-hydroxypropyl]-3,5-dihydro-2H-1,4-benzoxazepin-8-yl]-4-pyridyl]phenyl]-2-methoxy-3-pyridyl]methyl]-N-[[(2S)-5-oxopyrrolidin-2-yl]methyl]carbamate (13 mg, 0.02 mmol) was dissolved in 0.5 mL DCM and 4M HCl solution in 1,4-dioxane (17 µL, 0.07 mmol) was added. Reaction was stirred at rt for 20 minutes and solvent was removed under reduced pressure. Crude sample was purified by reverse phase HPLC to afford (5S)-5-[[[6-[2-chloro-3-[3-chloro-2-[4-[(2S)-2-hydroxypropyl]-3,5-dihydro-2H-1,4-benzoxazepin-8-yl]-4-pyridyl]phenyl]-2-methoxy-3-pyridyl]methylamino]methyl]pyrrolidin-2-one (10 mg, 94 % yield) as a white solid (formic acid salt). MS: *m*/*z* found 662.2, 664 [M+H] ⁺, LC retention time = 2.01 min. (Method C). ¹H NMR (400 MHz, Methanol-*d*₄) δ 8.61 (d, 1H), 8.40 (s, 1H), 7.80 (d, 1H), 7.71 (dd, 1H), 7.55 (t, 1H), 7.44 - 7.40 (m, 2H), 7.38 (d, 2H), 7.33 (d, 1H), 7.30 (d, 1H), 4.18 (dd, 2H), 4.14 (s, 2H), 4.03 (d, 6H), 3.92 (s, 1H), 3.33 (d, 2H), 2.97 - 2.84 (m, 2H), 2.71 (dd, 1H), 2.60 (dd, 1H), 2.41 - 2.26 (m, 3H), 1.92 - 1.77 (m, 1H), 1.16 (d, 3H).

### Example 49: (S)-5-((((6-(2-Chloro-3-(3-chloro-2-(2,3,4,5-tetrahydrobenzo[f][1,4]oxazepin-8-yl)pyridin-4-yl)phenyl)-2-methoxypyridin-3-yl)methyl)amino)methyl)pyrrolidin-2-one (26)

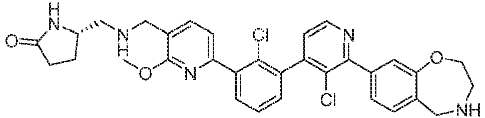

S)-5-((((6-(2-Chloro-3-(3-chloro-2-(2,3,4,5-tetrahydrobenzo[f][1,4]oxazepin-8-yl)pyridin-4-yl)phenyl)-2-methoxypyridin-3-yl)methyl)amino)methyl)pyrrolidin-2-one was prepared in the same manner as described for compound **25,** utilizing intermediate tert-butyl N-[[6-[2-chloro-3-(2,3-dichloro-4-pyridyl)phenyl]-2-methoxy-3-pyridyl]methyl]-N-[[(2S)-5-oxopyrrolidin-2-yl]methyl]carbamate. Tert-butyl 8-(4,4,5,5-tetramethyl-1,3,2-dioxaborolan-2-yl)-2,3-dihydrobenzo[f][1,4]oxazepine-4(5H)-carboxylate was used in place of (S)-1-(8-(4,4,5,5-tetramethyl-1,3,2-dioxaborolan-2-yl)-2,3-dihydrobenzo[f][1,4]oxazepin-4(5H)-yl)propan-2-ol. Product was obtained as a white solid. MS: *m*/*z* found 604.3, 606.1 [M+H]⁺ , LC retention time = 1.98 min. (Method C). ¹H NMR (400 MHz, Methanol-*d*₄) δ 8.64 (d, 1H), 8.43 (s, 2H), 7.80 (d, 1H), 7.71 (dd, 1H), 7.55 (t, 1H), 7.52 - 7.38 (m, 5H), 7.29 (d, 1H), 4.38 (s, 2H), 4.31 - 4.23 (m, 2H), 4.04 (s, 3H), 4.00 (d, 2H), 3.92 (d, 1H), 3.55 (dd, 2H), 2.94 - 2.80 (m, 2H), 2.41 - 2.22 (m, 3H), 1.92 - 1.78 (m, 1H).

### Example 50: (5S)-5-((((6-(2-Chloro-3-(3-chloro-2-(5-((((S)-5-oxopyrrolidin-2-yl)methyl)amino)-5,6,7,8-tetrahydronaphthalen-2-yl)pyridin-4-yl)phenyl)-2-methoxypyridin-3-yl)methyl)amino)methyl)pyrrolidin-2-one (diastereomeric mixture) (112)

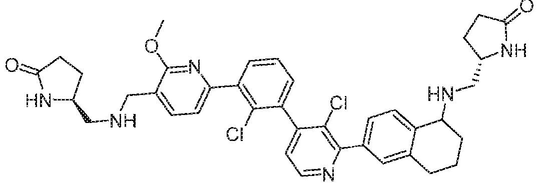

### (a) tert-Butyl (S)-((6-(2-chloro-3-(3-chloro-2-(5-oxo-5,6,7,8-tetrahydronaphthalen-2-yl)pyridin-4-yl)phenyl)-2-methoxypyridin-3-yl)methyl)((5-oxopyrrolidin-2-yl)methyl)carbamate

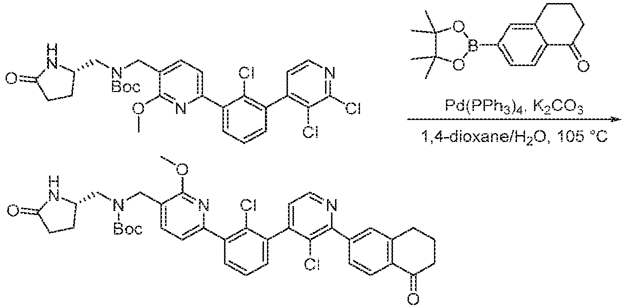

*tert*-Butyl N-[[6-[2-chloro-3-(2,3-dichloro-4-pyridyl)phenyl]-2-methoxy-3-pyridyl]methyl]-N-[[(2S)-5-oxopyrrolidin-2-yl]methyl]carbamate (200 mg, 0.34 mmol), Pd(PPh₃)₄ (78 mg, 0.07 mmol), potassium carbonate (140 mg, 1.01 mmol), and 6-(4,4,5,5-tetramethyl-1,3,2-dioxaborolan-2-yl)tetralin-1-one (138 mg, 0.51 mmol) were suspended in 1,4-dioxane/water (4:1, 4 mL), then the solution was heated at 105 °C for 20 minutes. Reaction was cooled to rt, diluted with 10 mL water, and extracted with EtOAc (3 x 5 mL). The combined organics were concentrated under reduced pressure and crude sample was purified by normal phase SiO₂ chromatography (0-4% MeOH/DCM) to afford tert-butyl N-[2-[3-[3-[5-[[tert-butoxycarbonyl-[[(2S)-5-oxopyrrolidin-2-yl]methyl]amino]methyl]-6-methoxy-2-pyridyl]-2-chloro-phenyl]-2-chloro-phenyl]-5,6,7,8-tetrahydroquinolin-5-yl]-N-[[(2S)-5-oxopyrrolidin-2-yl]methyl]carbamate (226 mg, 56 %) as a yellow foam. MS: *m*/*z* found 701, 703 [M+H] ⁺.

### (b) tert-Butyl ((6-(2-chloro-3-(3-chloro-2-(5-((((S)-5-oxopyrrolidin-2-yl)methyl)amino)-5,6,7,8-tetrahydronaphthalen-2-yl)pyridin-4-yl)phenyl)-2-methoxypyridin-3-yl)methyl)(((S)-5-oxopyrrolidin-2-yl)methyl)carbamate

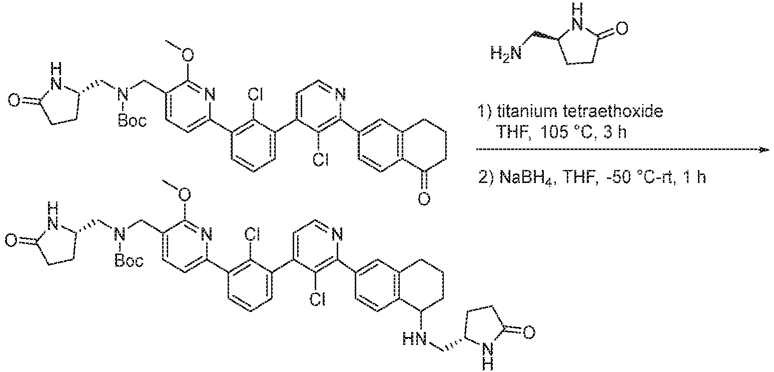

*tert*-Butyl N-[[6-[2-chloro-3-[3-chloro-2-(1-oxotetralin-6-yl)-4-pyridyl]phenyl]-2-methoxy-3-pyridyl]methyl]-N-[[(2S)-5-oxopyrrolidin-2-yl]methyl]carbamate (65 mg, 0.09 mmol), (5S)-5-(aminomethyl)pyrrolidin-2-one (21 mg, 0.19 mmol), and tetraethoxytitanium (63 mg, 0.28 mmol) were suspended in 1 mL anhydrous THF. Reaction mixture was heated at 105 °C for 3 hours in a sealed tube. Reaction was cooled to -50 °C and sodium borohydride (7 mg, 0.19 mmol) was added under a nitrogen stream, then the mixture was warmed to rt over 1 hour. Reaction was subsequently added drop-wise to an ice water solution (5 mL) and 5 mL EtOAc was also added. The resulting slurry was filtered through celite. The filtrate was extracted with EtOAc (3 x 5 mL) and the combined organics were further washed with brine (5 mL), dried over sodium sulfate, filtered, and concentrated under reduced pressure to afford tert-butyl N-[[6-[2-chloro-3-[2-chloro-3-[5-[[(2S)-2-hydroxypropyl]amino]-5,6,7,8-tetrahydroquinolin-2-yl]phenyl]phenyl]-2-methoxy-3-pyridyl]methyl]-N-[[(2S)-5-oxopyrrolidin-2-yl]methyl]carbamate (21 mg, crude, racemic mixture) as a yellow foam. MS: *m*/*z* found 799, 801 [M+H] ⁺. Material was used for the next step without further purification.

### (c) (5S)-5-((((6-(2-Chloro-3-(3-chloro-2-(5-((((S)-5-oxopyrrolidin-2-yl)methyl)amino)-5,6,7,8-tetrahydronaphthalen-2-yl)pyridin-4-yl)phenyl)-2-methoxypyridin-3-yl)methyl)amino)methyl)pyrrolidin-2-one

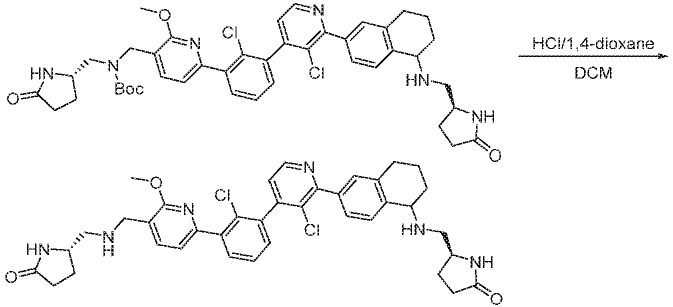

*tert*-Butyl N-[[6-[2-chloro-3-[3-chloro-2-[1-[[(2S)-5-oxopyrrolidin-2-yl]methylamino]tetralin-6-yl]-4-pyridyl]phenyl]-2-methoxy-3-pyridyl]methyl]-N-[[(2S)-5-oxopyrrolidin-2-yl]methyl]carbamate (34 mg, 0.04 mmol) was dissolved in 1 mL DCM and 4 M HCl solution in 1,4-dioxane (39 µL, 0.16 mmol) was added. Reaction was stirred at rt for 20 minutes and solvent was removed under reduced pressure. Crude sample was purified by reverse phase HPLC to afford (5S)-5-[[[6-[2-chloro-3-[3-chloro-2-[1-[[(2S)-5-oxopyrrolidin-2-yl]methylamino]tetralin-6-yl]-4-pyridyl]phenyl]-2-methoxy-3-pyridyl]methylamino]methyl]pyrrolidin-2-one (21 mg, 75 % yield, diastereomeric mixture) as the formic acid salt. LCMS: *m*/*z* found 699, 701 [M+H]⁺, retention time = 2.03 min. (Method C). ¹H NMR (400 MHz, Methanol-*d*₄) δ 8.60 (d, 1H), 7.76 (d, 1H), 7.70 (dd, 1H), 7.54 (t, 2H), 7.48 (d, 1H), 7.44 - 7.36 (m, 3H), 7.26 (d, 1H), 4.02 (s, 4H), 3.96 - 3.78 (m, 4H), 2.82 (m, 6H), 2.39 - 2.25 (m, 6H), 2.01 (s, 3H), 1.92 - 1.75 (m, 3H).

### Example 51: (5S)-5-((((6-(2-Chloro-3-(3-chloro-2-(5-(((S)-2-hydroxypropyl)amino)-5,6,7,8-tetrahydronaphthalen-2-yl)pyridin-4-yl)phenyl)-2-methoxypyridin-3-yl)methyl)amino)methyl)pyrrolidin-2-one (diastereomeric mixture) (113)

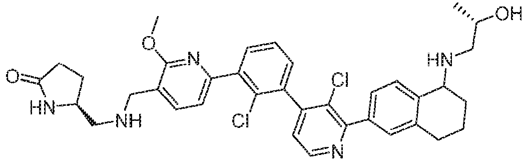

Compound was prepared in the same manner as described for compound 112, utilizing intermediate tert-butyl (S)-((6-(2-chloro-3-(3-chloro-2-(5-oxo-5,6,7,8-tetrahydronaphthalen-2-yl)pyridin-4-yl)phenyl)-2-methoxypyridin-3-yl)methyl)((5-oxopyrrolidin-2-yl)methyl)carbamate. (S)-1-aminopropan-2-ol was used in place of (S)-5-(aminomethyl)pyrrolidin-2-one. LCMS: *m*/*z* found 660, 662 [M+H]⁺, retention time = 2.12 min. (Method C). ¹H NMR (400 MHz, Methanol-*d*₄) δ 8.63 (d, 1H), 8.49 (s, 1H), 7.76 (d, 1H), 7.70 (dd, 1H), 7.58 (d, 2H), 7.54 (t, 2H), 7.45 (d, 1H), 7.40 (dd, 1H), 7.26 (d, 1H), 4.59 (s, 1H), 4.02 (s, 4H), 3.88 (dd, 3H), 3.18 - 3.05 (m, 1H), 3.02 - 2.84 (m, 3H), 2.82 - 2.69 (m, 2H), 2.38 - 2.25 (m, 3H), 2.20 (s, 2H), 2.02 (d, 1H), 1.97 - 1.77 (m, 2H), 1.25 (dd, 3H).

### Example 52: (S)-5-((((6-(2-Chloro-3-(3-chloro-2-(4-((((S)-2-hydroxypropyl)amino)methyl)-3-methoxy-5-methylphenyl)pyridin-4-yl)phenyl)-2-methoxypyridin-3-yl)methyl)amino)methyl)pyrrolidin-2-one (114)

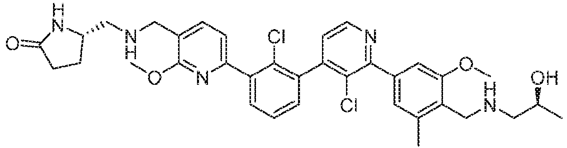

(S)-5-((((6-(2-Chloro-3-(3-chloro-2-(4-((((S)-2-hydroxypropyl)amino)methyl)-3-methoxy-5-methylphenyl)pyridin-4-yl)phenyl)-2-methoxypyridin-3-yl)methyl)amino)methyl)pyrrolidin-2-one was prepared in the same manner as described for compound 21 utilizing intermediate (S)-tert-butyl ((6-(2-chloro-3-(2,3-dichloropyridin-4-yl)phenyl)-2-methoxypyridin-3-yl)methyl)((5-oxopyrrolidin-2-yl)methyl)carbamate. 2-methoxy-6-methyl-4-(4,4,5,5-tetramethyl-1,3,2-dioxaborolan-2-yl)-benzaldehyde was used in place of 1-methyl-6-(4,4,5,5-tetramethyl-1,3,2-dioxaborolan-2-yl)pyrrolo[3,2-b]pyridine-3-carbaldehyde. Product was obtained as a white solid (formic acid salt). LCMS: *m*/*z* found 664, 666 [M+H]⁺, retention time = 2.07 min. (Method C). ¹H NMR (400 MHz, Methanol-*d*₄) δ 8.64 (d, 1H), 8.49 (s, 1H), 7.76 (d, 1H), 7.73 - 7.68 (m, 1H), 7.55 (t, 1H), 7.46 (d, 1H), 7.43 - 7.37 (m, 1H), 7.26 (d, 1H), 7.22 (s, 2H), 4.44 - 4.30 (m, 2H), 4.10 (d, 1H), 4.02 (s, 3H), 3.97 (s, 3H), 3.88 (dd, 3H), 3.16 (dd, 1H), 2.90 (dd, 1H), 2.84 - 2.71 (m, 2H), 2.51 (s, 3H), 2.39 - 2.24 (m, 3H), 1.82 (dd, 1H), 1.25 (d, 3H).

### Example 53: (S)-5-((((6-(2-Chloro-3-(3-chloro-2-(3-methoxy-5-methyl-4-(((((S)-5-oxopyrrolidin-2-yl)methyl)amino)methyl)phenyl)pyridin-4-yl)phenyl)-2-methoxypyridin-3-yl)methyl)amino)methyl)pyrrolidin-2-one (115)

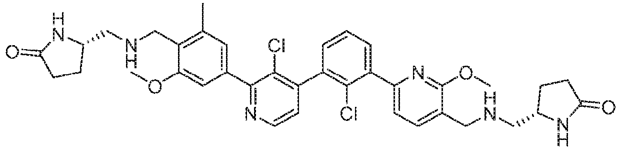

(S)-5-((((6-(2-Chloro-3-(3-chloro-2-(3-methoxy-5-methyl-4-(((((S)-5-oxopyrrolidin-2-yl)methyl)amino)methyl)phenyl)pyridin-4-yl)phenyl)-2-methoxypyridin-3-yl)methyl)amino)methyl)pyrrolidin-2-one was prepared in the same manner as described for compound 21, utilizing intermediate (S)-tert-butyl ((6-(2-chloro-3-(2,3-dichloropyridin-4-yl)phenyl)-2-methoxypyridin-3-yl)methyl)((5-oxopyrrolidin-2-yl)methyl)carbamate. 2-methoxy-6-methyl-4-(4,4,5,5-tetramethyl-1,3,2-dioxaborolan-2-yl)-benzaldehyde was used in place of 1-methyl-6-(4,4,5,5-tetramethyl-1,3,2-dioxaborolan-2-yl)pyrrolo[3,2-b]pyridine-3-carbaldehyde. Product was obtained as a white solid (formic acid salt). MS: *m*/*z* found 703, 705 [M+H] ⁺, LC retention time = 2.04 min. (Method C). ¹H NMR (400 MHz, Methanol-*d*₄) δ 8.63 (d, 1H), 8.46 (s, 1H), 7.78 (d, 1H), 7.71 (dd, 1H), 7.55 (t, 1H), 7.45 (d, 1H), 7.41 (dd, 1H), 7.27 (d, 1H), 7.20 (s, 2H), 4.23 (s, 2H), 4.03 (s, 3H), 4.02 - 3.94 (m, 6H), 3.90 (d, 1H), 3.08 (dd, 2H), 2.82 (h, 2H), 2.49 (s, 3H), 2.43 - 2.28 (m, 6H), 1.95 - 1.78 (m, 2H).

### Example 54: (S)-5-((((6-(2-Chloro-3-(3-chloro-2-(3-methoxy-5-methyl-4-((methyl(((S)-5-oxopyrrolidin-2-yl)methyl)amino)methyl)phenyl)pyridin-4-yl)phenyl)-2-methoxypyridin-3-yl)methyl)amino)methyl)pyrrolidin-2-one (117)

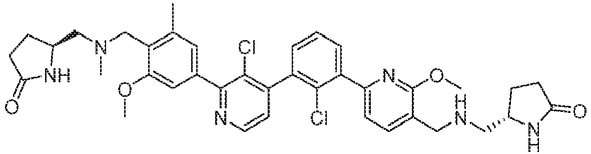

(S)-5-((((6-(2-Chloro-3-(3-chloro-2-(3-methoxy-5-methyl-4-((methyl(((S)-5-oxopyrrolidin-2-yl)methyl)amino)methyl)phenyl)pyridin-4-yl)phenyl)-2-methoxypyridin-3-yl)methyl)amino)methyl)pyrrolidin-2-one was prepared in the same manner as described for compound **21** utilizing intermediate (S)-tert-butyl ((6-(2-chloro-3-(2,3-dichloropyridin-4-yl)phenyl)-2-methoxypyridin-3-yl)methyl)((5-oxopyrrolidin-2-yl)methyl)carbamate. 2-methoxy-6-methyl-4-(4,4,5,5-tetramethyl-1,3,2-dioxaborolan-2-yl)-benzaldehyde was used in place of 1-methyl-6-(4,4,5,5-tetramethyl-1,3,2-dioxaborolan-2-yl)pyrrolo[3,2-b]pyridine-3-carbaldehyde. Product was obtained as a white solid (formic acid salt). MS: *m*/*z* found 717, 719 [M+H] ⁺, LC retention time = 2.07 min. (Method C). ¹H NMR (400 MHz, Methanol-*d*₄) δ 8.61 (d, 1H), 8.42 (s, 1H), 7.79 (d, 1H), 7.71 (d, 1H), 7.55 (t, 1H), 7.42 (t, 2H), 7.29 (d, 1H), 7.14 (s, 2H), 4.04 (s, 3H), 4.00 (d, 2H), 3.97 - 3.85 (m, 6H), 3.75 (d, 1H), 2.88 (t, 2H), 2.65 (d, 2H), 2.48 (s, 3H), 2.43 - 2.23 (m, 9H), 1.92 - 1.77 (m, 1H), 1.73 (s, 1H).

### Example 55: (S)-5-((((6-(2-Chloro-3-(3-chloro-2-(4-((((S)-2-hydroxypropyl)(methyl)amino)methyl)-3-methoxy-5-methylphenyl)pyridin-4-yl)phenyl)-2-methoxypyridin-3-yl)methyl)amino)methyl)pyrrolidin-2-one (118)

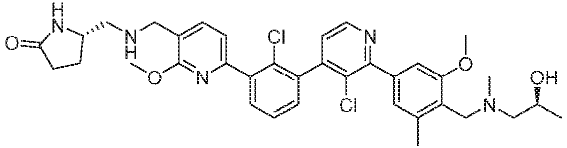

(S)-5-((((6-(2-Chloro-3-(3-chloro-2-(4-((((S)-2-hydroxypropyl)(methyl)amino)methyl)-3-methoxy-5-methylphenyl)pyridin-4-yl)phenyl)-2-methoxypyridin-3-yl)methyl)amino)methyl)pyrrolidin-2-one was prepared in the same manner as described for compound 21 utilizing intermediate (S)-tert-butyl ((6-(2-chloro-3-(2,3-dichloropyridin-4-yl)phenyl)-2-methoxypyridin-3-yl)methyl)((5-oxopyrrolidin-2-yl)methyl)carbamate. 2-methoxy-6-methyl-4-(4,4,5,5-tetramethyl-1,3,2-dioxaborolan-2-yl)-benzaldehyde was used in place of 1-methyl-6-(4,4,5,5-tetramethyl-1,3,2-dioxaborolan-2-yl)pyrrolo[3,2-b]pyridine-3-carbaldehyde. Product was obtained as a white solid (formic acid salt). MS: *m*/*z* found 678, 680 [M+H] ⁺, LC retention time = 2.10 min. (Method C). ¹H NMR (400 MHz, Methanol-*d*₄) δ 8.63 (d, 1H), 8.53 (s, 1H), 7.74 (d, 1H), 7.70 (d, 1H), 7.54 (t, 1H), 7.47 - 7.43 (m, 1H), 7.40 (d, 1H), 7.24 (d, 1H), 7.20 (s, 2H), 4.16 (s, 2H), 4.01 (t, 3H), 3.94 (s, 3H), 3.89 - 3.77 (m, 3H), 2.69 (dd, 5H), 2.50 (d, 3H), 2.30 (m, 3H), 1.80 (d, 1H), 1.21 (d, 3H).

### Example 56: (5S)-5-[[[6-[3-[2-[3-[[(1-Acetyl-4-piperidyl)amino]methyl]-1-methyl-indol-6-yl]-3-chloro-4-pyridyl]-2-chloro-phenyl]-2-methoxy-3-pyridyl]methylamino]methyl]pyrrolidin-2-one (28)

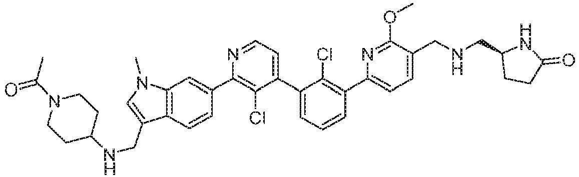

### (a) tert-Butyl N-[[6-[2-chloro-3-[3-chloro-2-(3-formyl-1-methyl-indol-6-yl)-4-pyridyl]phenyl]-2-methoxy-3-pyridyl]methyl]-N-[[(2S)-5-oxopyrrolidin-2-yl]methyl]carbamate

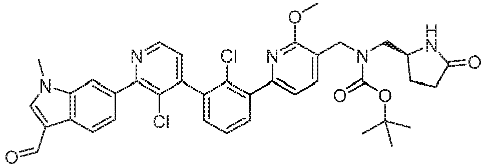

*tert*-Butyl N-[[6-[2-chloro-3-[3-chloro-2-(3-formyl-1-methyl-indol-6-yl)-4-pyridyl]phenyl]-2-methoxy-3-pyridyl]methyl]-N-[[(2S)-5-oxopyrrolidin-2-yl]methyl]carbamate was prepared by following Suzuki Coupling Procedure B from *tert-*butyl N-[[6-[2-chloro-3-(2,3-dichloro-4-pyridyl)phenyl]-2-methoxy-3-pyridyl]methyl]-N-[[(2S)-5-oxopyrrolidin-2-yl]methyl]carbamate (300 mg, 0.51 mmol) and 1-methyl-6-(4,4,5,5-tetramethyl-1,3,2-dioxaborolan-2-yl)indole-3-carbaldehyde (144 mg, 0.51 mmol). 80% yield. MS: m/z found 714.2 [M+H]⁺.

### (b) (5S)-5-[[[6-[3-[2-[3-[[(1-Acetyl-4-piperidyl)amino]methyl]-1-methyl-indol-6-yl]-3-chloro-4-pyridyl]-2-chloro-phenyl]-2-methoxy-3-pyridyl]methylamino]methyl]pyrrolidin-2-one

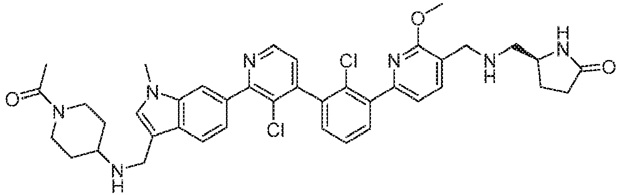

Utilizing reductive Amination Procedure B from starting materials *tert*-butyl N-[[6-[2-chloro-3-[3-chloro-2-(3-formyl-1-methyl-indol-6-yl)-4-pyridyl]phenyl]-2-methoxy-3-pyridyl]methyl]-N-[[(2S)-5-oxopyrrolidin-2-yl]methyl]carbamate (25 mg, 0.03 mmol) and 1-(4-amino-1-piperidyl)ethanone (10 mg, 0.07 mmol) provided *tert*-butyl N-[[6-[3-[2-[3-[[(1-acetyl-4-piperidyl)amino]methyl]-1-methyl-indol-6-yl]-3-chloro-4-pyridyl]-2-chloro-phenyl]-2-methoxy-3-pyridyl]methyl]-N-[[(2S)-5-oxopyrrolidin-2-yl]methyl]carbamate as crude, 75% LCMS yield. MS: m/z found 840.3 [M+H]⁺. The crude was deprotected and purified by General Boc Deprotection Procedure to afford the final product as formic acid salt. 96% LCMS yield for the deprotection step. MS: m/z found 740.3 [M+H]⁺. ¹H NMR (400 MHz, Methanol-*d*₄): δ 8.64 (d, 1H), 8.54 (s, 2H), 7.82 (d, 1H), 7.78 - 7.73 (m, 2H), 7.71 (dd, 1H), 7.55 (t, 1H), 7.52 - 7.47 (m, 2H), 7.45 - 7.40 (m, 2H), 7.26 (d, 1H), 4.63 (d, 1H), 4.39 (s, 2H), 4.09 - 4.00 (m, 4H), 3.90 (s, 3H), 3.88 - 3.78 (m, 3H), 3.40 - 3.32 (m, 1H), 3.24 - 3.11 (m, 1H), 2.79 - 2.62 (m, 3H), 2.39 - 2.17 (m, 5H), 2.13 (s, 3H), 1.82 (m, 1H), 1.66 - 1.40 (m, 2H).

### Example 57: (5S)-5-[[[6-[2-Chloro-3-[3-chloro-2-[3-[(2-hydroxyethylamino)methyl]-1-methyl-indol-6-yl]-4-pyridyl] phenyl]-2-methoxy-3-pyridyl]methylamino]methyl]pyrrolidin-2-one (29)

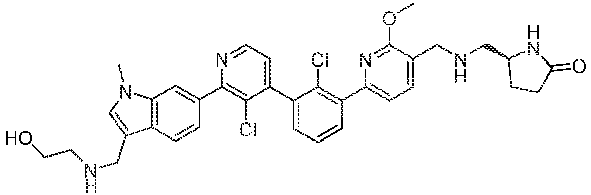

Utilizing reductive Amination Procedure B from *tert*-butyl N-[[6-[2-chloro-3-[3-chloro-2-(3-formyl-1-methyl-indol-6-yl)-4-pyridyl]phenyl]-2-methoxy-3-pyridyl]methyl]-N-[[(2S)-5-oxopyrrolidin-2-yl]methyl]carbamate (20 mg, 0.03 mmol) and 2-aminoethanol (4 mg, 0.06 mmol) provided *tert*-butyl N-[[6-[2-chloro-3-[3-chloro-2-[3-[(2-hydroxyethylamino)methyl]-1-methyl-indol-6-yl]-4-pyridyl]phenyl]-2-methoxy-3-pyridyl]methyl]-N-[[(2S)-5-oxopyrrolidin-2-yl]methyl]carbamate as crude, 73% LCMS yield. MS: m/z found 759.3 [M+H]⁺. The crude was deprotected and purified by General Boc Deprotection Procedure to afford the final product as formic acid salt. 97% LCMS yield for the deprotection step. MS: m/z found 659.2 [M+H]⁺. ¹H NMR (400 MHz, Methanol-*d*₄): δ 8.64 (d, 1H), 8.55 (s, 2H), 7.82 (d, 1H), 7.78 - 7.73 (m, 2H), 7.71 (dd, 1H), 7.55 (t, 1H), 7.51 - 7.46 (m, 2H), 7.47 - 7.39 (m, 2H), 7.26 (d, 1H), 4.38 (s, 2H), 4.02 (s, 3H), 3.90 (s, 3H), 3.88 - 3.81 (m, 3H), 3.81 - 3.77 (m, 2H), 3.09 (t, 2H), 2.77 - 2.62 (m, 2H), 2.39 - 2.21 (m, 3H), 1.88 - 1.75 (m, 1H).

### Example 58: (5S)-5-[[[6-[2-Chloro-3-[3-chloro-2-[3-[(2-methoxyethylamino)methyl]-1-methyl-indol-6-yl]-4-pyridyl] phenyl]-2-methoxy-3-pyridyl]methylamino]methyl]pyrrolidin-2-one (30)

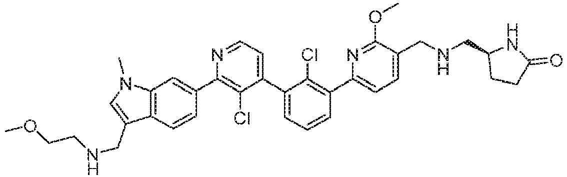

Utilizing Reductive Amination Procedure B from *tert*-butyl N-[[6-[2-chloro-3-[3-chloro-2-(3-formyl-1-methyl-indol-6-yl)-4-pyridyl]phenyl]-2-methoxy-3-pyridyl]methyl]-N-[[(2S)-5-oxopyrrolidin-2-yl]methyl]carbamate (20 mg, 0.03 mmol) and 2-methoxyethanamine (4 mg , 0.06 mmol) gave *tert*-butyl N-[[6-[2-chloro-3-[3-chloro-2-[3-[(2-methoxyethylamino)methyl]-1-methyl-indol-6-yl]-4-pyridyl]phenyl]-2-methoxy-3-pyridyl]methyl]-N-[[(2S)-5-oxopyrrolidin-2-yl]methyl]carbamate as crude, 74% LCMS yield. MS: m/z found 773.3 [M+H]⁺. The crude was deprotected and purified using the General Boc Deprotection Procedure to afford the final product as formic acid salt. 98% LCMS yield for the deprotection step. MS: m/z found 673.2 [M+H]⁺. ¹H NMR (400 MHz, Methanol-*d*₄): δ 8.64 (d, 1H), 8.55 (s, 2H), 7.81 (d, 1H), 7.79 - 7.73 (m, 2H), 7.71 (dd, 1H), 7.55 (t, 1H), 7.51 - 7.46 (m, 2H), 7.45 - 7.39 (m, 2H), 7.26 (d, 1H), 4.36 (s, 2H), 4.02 (s, 3H), 3.90 (s, 3H), 3.88 - 3.77 (m, 3H), 3.67 - 3.56 (m, 2H), 3.40 (s, 3H), 3.16 (t, 2H), 2.76 - 2.63 (m, 2H), 2.39 - 2.22 (m, 3H), 1.83 (td, 1H).

### Example 59: (5S)-5-[[[6-[2-Chloro-3-[3-chloro-2-[1-methyl-3-(methylaminomethyl)indol-6-yl]-4-pyridyl] phenyl]-2-methoxy-3-pyridyl]methylamino]methyl]pyrrolidin-2-one (31)

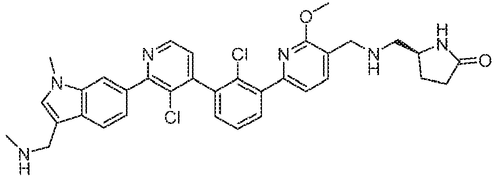

Utilizing Reductive Amination Procedure B from *tert*-butyl N-[[6-[2-chloro-3-[3-chloro-2-(3-formyl-1-methyl-indol-6-yl)-4-pyridyl]phenyl]-2-methoxy-3-pyridyl]methyl]-N-[[(2S)-5-oxopyrrolidin-2-yl]methyl]carbamate (20 mg, 0.03 mmol) and methanamine solution (33% wt in ethanol, 0.06 mmol) gave *tert*-butyl N-[[6-[2-chloro-3-[3-chloro-2-[1-methyl-3-(methylaminomethyl)indol-6-yl]-4-pyridyl]phenyl]-2-methoxy-3-pyridyl]methyl]-N-[[(2S)-5-oxopyrrolidin-2-yl]methyl]carbamate as crude, 73% LCMS yield. MS: m/z found 729.3 [M+H]⁺. The crude was deprotected and purified by General Boc Deprotection Procedure to afford the final product as formic acid salt. 96% LCMS yield for the deprotection step. MS: m/z found 629.2 [M+H]⁺. ¹H NMR (400 MHz, Methanol-*d*₄): δ 8.64 (d, 1H), 8.54 (s, 2H), 7.83 (d, 1H), 7.79 (d, *J* = 1.3 Hz, 1H), 7.75 (d, 1H), 7.71 (dd, 1H), 7.59 - 7.48 (m, 3H), 7.47 - 7.40 (m, 2H), 7.26 (d, 1H), 4.41 (s, 2H), 4.02 (s, 3H), 3.91 (s, 3H), 3.89 - 3.75 (m, 3H), 2.77 - 2.62 (m, 5H), 2.39 - 2.23 (m, 3H), 1.89 - 1.74 (m, 1H).

### Example 60: (5S)-5-[[[6-[2-Chloro-3-[3-chloro-2-[3-(ethylaminomethyl)-1-methyl-indol-6-yl]-4-pyridyl]phenyl]-2-methoxy-3-pyridyl]methylamino]methyl]pyrrolidin-2-one (32)

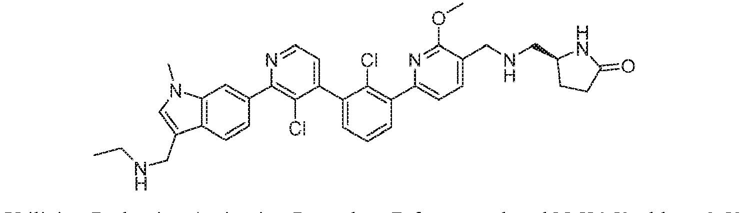

Utilizing Reductive Amination Procedure B from *tert*-butyl N-[[6-[2-chloro-3-[3-chloro-2-(3-formyl-1-methyl-indol-6-yl)-4-pyridyl]phenyl]-2-methoxy-3-pyridyl]methyl]-N-[[(2S)-5-oxopyrrolidin-2-yl]methyl]carbamate (20 mg, 0.03 mmol) and ethanamine solution (2.0 M in THF, 0.06 mmol) provided *tert*-butyl N-[[6-[2-chloro-3-[3-chloro-2-[3-(ethylaminomethyl)-1-methyl-indol-6-yl]-4-pyridyl]phenyl]-2-methoxy-3-pyridyl]methyl]-N-[[(2S)-5-oxopyrrolidin-2-yl]methyl]carbamate as crude, 72% LCMS yield. MS: m/z found 743.3 [M+H]⁺. The crude was deprotected and purified by General Boc Deprotection Procedure to afford the final product as formic acid salt. 95% LCMS yield for the deprotection step. MS: m/z found 643.2 [M+H]⁺. ¹H NMR (400 MHz, Methanol-*d*₄): δ 8.64 (d, 1H), 8.55 (s, 2H), 7.83 (d, 1H), 7.79 - 7.77 (m, 1H), 7.75 (d, 1H), 7.71 (dd, 1H), 7.56 (t, 1H), 7.53 - 7.48 (m, 2H), 7.46 - 7.40 (m, 2H), 7.26 (d, 1H), 4.40 (s, 2H), 4.02 (s, 3H), 3.91 (s, 3H), 3.89 - 3.77 (m, 3H), 3.12 (q, 2H), 2.77 - 2.62 (m, 2H), 2.39 - 2.22 (m, 3H), 1.89 - 1.75 (m, 1H), 1.33 (t, 3H).

### Example 61: (5S)-5-[[[6-[2-Chloro-3-[3-chloro-2-[3-[(dimethylamino)methyl]-1-methyl-indol-6-yl]-4-pyridyl]phenyl]-2-methoxy-3-pyridyl]methylamino]methyl]pyrrolidin-2-one (33)

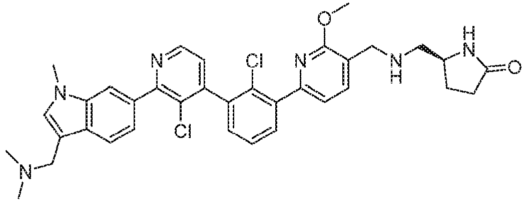

Utilizing Reductive Amination Procedure B from *tert*-butyl N-[[6-[2-chloro-3-[3-chloro-2-(3-formyl-1-methyl-indol-6-yl)-4-pyridyl]phenyl]-2-methoxy-3-pyridyl]methyl]-N-[[(2S)-5-oxopyrrolidin-2-yl]methyl]carbamate (20 mg, 0.03 mmol) and N-methylmethanamine solution (2.0 M in ethanol, 0.06 mmol gave *tert*-butyl N-[[6-[2-chloro-3-[3-chloro-2-[3-[(dimethylamino)methyl]-1-methyl-indol-6-yl]-4-pyridyl]phenyl]-2-methoxy-3-pyridyl]methyl]-N-[[(2S)-5-oxopyrrolidin-2-yl]methyl]carbamate as crude, 77% LCMS yield. MS: m/z found 743.3 [M+H]⁺. The crude was deprotected and purified by General Boc Deprotection Procedure to afford the final product as formic acid salt. 96% LCMS yield for the deprotection step. MS: m/z found 643.2 [M+H]⁺. ¹H NMR (400 MHz, Methanol-*d*₄): δ 8.64 (d, 1H), 8.54 (s, 2H), 7.81 (d, 1H), 7.77 (d, 1H), 7.75 (d, 1H), 7.71 (dd, 1H), 7.55 (t, 1H), 7.51 - 7.47 (m, 2H), 7.45 - 7.38 (m, 2H), 7.26 (d, 1H), 4.24 (s, 2H), 4.02 (s, 3H), 3.91 (s, 3H), 3.89 - 3.78 (m, 3H), 2.77 - 2.61 (m, 2H), 2.68 (s, 6H), 2.37 - 2.24 (m, 3H), 1.87 - 1.76 (m, 1H).

### Example 62: (5S)-5-[[[6-[2-Chloro-3-[3-chloro-2-[1-methyl-3-[[[(2S)-oxetan-2-yl]methylamino]methyl]indol-6-yl]-4-pyridyl]phenyl]-2-methoxy-3-pyridyl]methylamino]methyl]pyrrolidin-2-one (34)

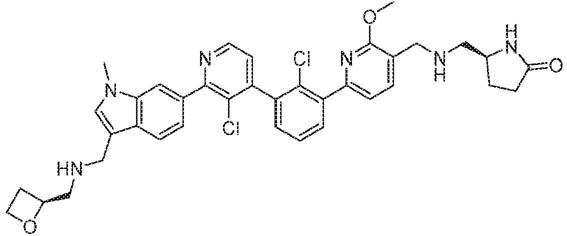

Utilizing Reductive Amination Procedure B from *tert*-butyl N-[[6-[2-chloro-3-[3-chloro-2-(3-formyl-1-methyl-indol-6-yl)-4-pyridyl]phenyl]-2-methoxy-3-pyridyl]methyl]-N-[[(2S)-5-oxopyrrolidin-2-yl]methyl]carbamate (20 mg, 0.03 mmol) and [(2S)-oxetan-2-yl]methanamine (5 mg, 0.06 mmol) gave *tert*-butyl N-[[6-[2-chloro-3-[3-chloro-2-[1-methyl-3-[[[(2S)-oxetan-2-yl]methylamino]methyl]indol-6-yl]-4-pyridyl]phenyl]-2-methoxy-3-pyridyl]methyl]-N-[[(2S)-5-oxopyrrolidin-2-yl]methyl]carbamate as crude, 73% LCMS yield. MS: m/z found 785.3 [M+H]⁺. Following the General Boc Deprotection Procedure from the crude afforded the final product as formic acid salt. 96% LCMS yield for the deprotection step. MS: m/z found 685.2 [M+H]⁺. ¹H NMR (400 MHz, Methanol-*d*₄): δ 8.63 (d, 1H), 8.55 (s, 2H), 7.79 (d, 1H), 7.75 (d, 1H), 7.73 (s, 1H), 7.71 (dd, 1H), 7.55 (t, 1H), 7.48 - 7.38 (m, 4H), 7.26 (d, 1H), 5.03 (dd, 1H), 4.75 - 4.66 (m, 1H), 4.57 (dt, 1H), 4.21 (s, 2H), 4.02 (s, 3H), 3.88 (s, 3H), 3.86 - 3.76 (m, 3H), 3.21 (dd, 1H), 3.01 (dd, 1H), 2.82 - 2.62 (m, 3H), 2.54 - 2.42 (m, 1H), 2.37 - 2.23 (m, 3H), 1.89 - 1.75 (m, 1H).

### Example 63: (5S)-5-[[[6-[2-Chloro-3-[3-chloro-2-[3-[[[(2S)-2-hydroxypropyl]amino]methyl]-1-methyl-indol-6-yl]-4-pyridyl]phenyl]-2-methoxy-3-pyridyl]methylamino]methyl]pyrrolidin-2-one (35)

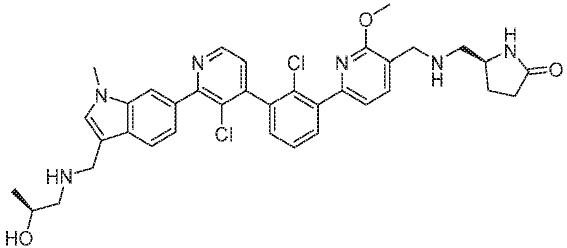

Utilizing Reductive Amination Procedure B from *tert*-butyl N-[[6-[2-chloro-3-[3-chloro-2-(3-formyl-1-methyl-indol-6-yl)-4-pyridyl]phenyl]-2-methoxy-3-pyridyl]methyl]-N-[[(2S)-5-oxopyrrolidin-2-yl]methyl]carbamate (20 mg, 0.03 mmol) and (2S)-1-aminopropan-2-ol (4 mg, 0.06 mmol) gave *tert*-butyl N-[[6-[2-chloro-3-[3-chloro-2-[3-[[[(2S)-2-hydroxypropyl]amino]methyl]-1-methyl-indol-6-yl]-4-pyridyl]phenyl]-2-methoxy-3-pyridyl]methyl]-N-[[(2S)-5-oxopyrrolidin-2-yl]methyl]carbamate (83.1% LCMS yield) as crude, MS: m/z found 773.3 [M+H]⁺, which was deprotected and purified by General Boc Deprotection Procedure to afford the final product as formic acid salt. 96% LCMS yield. MS: m/z found 673.2 [M+H]⁺. ¹H NMR (400 MHz, Methanol-*d*₄): δ 8.64 (d, 1H), 8.55 (s, 2H), 7.82 (d, 1H), 7.77 (t, 1H), 7.75 (d, 1H), 7.71 (dd, 1H), 7.55 (t, 1H), 7.52 - 7.47 (m, 2H), 7.45 - 7.40 (m, 2H), 7.26 (d, 1H), 4.39 (s, 2H), 4.07 - 3.99 (m, 4H), 3.90 (s, 3H), 3.87 - 3.78 (m, 3H), 3.03 (dd, 1H), 2.84 (dd, 1H), 2.76 - 2.63 (m, 2H), 2.38 - 2.20 (m, 3H), 1.83 (td, 1H), 1.20 (d, 3H).

### Example 64: (5S)-5-[[[6-[2-Chloro-3-[3-chloro-2-[3-[[(2-hydroxy-2-methylpropyl)amino]methyl]-1-methyl-indol-6-yl]-4-pyridyl]phenyl]-2-methoxy-3-pyridyl]methylamino]methyl]pyrrolidin-2-one (36)

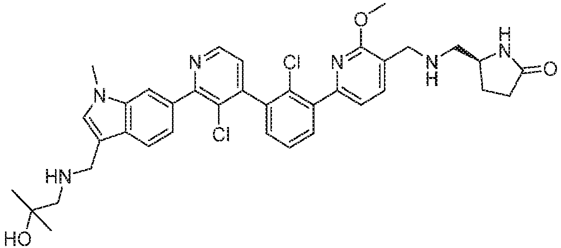

Utilizing Reductive Amination Procedure B from *tert*-butyl N-[[6-[2-chloro-3-[3-chloro-2-(3-formyl-1-methyl-indol-6-yl)-4-pyridyl]phenyl]-2-methoxy-3-pyridyl]methyl]-N-[[(2S)-5-oxopyrrolidin-2-yl]methyl]carbamate (25 mg, 0.03 mmol) and 1-amino-2-methyl-propan-2-ol (6 mg, 0.07 mmol) gave *tert*-butyl N-[[6-[2-chloro-3-[3-chloro-2-[3-[[(2-hydroxy-2-methyl-propyl)amino]methyl]-1-methyl-indol-6-yl]-4-pyridyl]phenyl]-2-methoxy-3-pyridyl]methyl]-N-[[(2S)-5-oxopyrrolidin-2-yl]methyl]carbamate (90% LCMS yield) as crude, MS: m/z found 787.3 [M+H]⁺, which was deprotected and purified by General Boc Deprotection Procedure to afford the final product as formic acid salt. 97% LCMS yield. MS: m/z found 687.3 [M+H]⁺. ¹H NMR (400 MHz, Methanol-*d*₄): δ 8.64 (d, 1H), 8.54 (s, 2H), 7.84 (d, 1H), 7.79 - 7.78 (m, 1H), 7.75 (d, 1H), 7.71 (dd, 1H), 7.59 - 7.53 (m, 2H), 7.51 (dd, 1H), 7.46 - 7.40 (m, 2H), 7.26 (d, 1H), 4.45 (s, 2H), 4.02 (s, 3H), 3.91 (s, 3H), 3.89 - 3.79 (m, 3H), 2.96 (s, 2H), 2.78 - 2.62 (m, 2H), 2.37 - 2.22 (m, 3H), 1.88 - 1.74 (m, 1H), 1.25 (s, 6H).

### Example 65: (5S)-5-[[[6-[2-Chloro-3-[3-chloro-2-[3-[(isobutylamino)methyl]-1-methyl-indol-6-yl]-4-pyridyl]phenyl]-2-methoxy-3-pyridyl]methylamino]methyl]pyrrolidin-2-one (38)

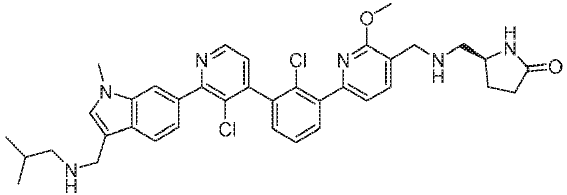

Utilizing Reductive Amination Procedure B from *tert*-butyl N-[[6-[2-chloro-3-[3-chloro-2-(3-formyl-1-methyl-indol-6-yl)-4-pyridyl]phenyl]-2-methoxy-3-pyridyl]methyl]-N-[[(2S)-5-oxopyrrolidin-2-yl]methyl]carbamate (25 mg, 0.03 mmol) and 2-methylpropan-1-amine (5 mg, 0.07 mmol) gave *tert*-butyl N-[[6-[2-chloro-3-[3-chloro-2-[3-[(isobutylamino)methyl]-1-methyl-indol-6-yl]-4-pyridyl]phenyl]-2-methoxy-3-pyridyl]methyl]-N-[[(2S)-5-oxopyrrolidin-2-yl]methyl]carbamate (74% LCMS yield) as crude, MS: m/z found 771.3 [M+H]⁺, which was deprotected and purified by General Boc Deprotection Procedure to afford the final product as formic acid salt. 96% LCMS yield. MS: m/z found 671.3 [M+H]⁺. ¹H NMR (400 MHz, Methanol-*d*₄): δ 8.64 (d1H), 8.54 (s, 2H), 7.87 - 7.81 (m, 1H), 7.78 (d, 1H), 7.75 (d, 1H), 7.71 (dd, 1H), 7.59 - 7.53 (m, 2H), 7.51 (dd, 1H), 7.46 - 7.40 (m, 2H), 7.26 (d, 1H), 4.42 (s, 2H), 4.02 (s, 3H), 3.91 (s, 3H), 3.89 - 3.77 (m, 3H), 2.89 (d, 2H), 2.76 - 2.63 (m, 2H), 2.38 - 2.23 (m, 3H), 2.06 - 1.95 (m, 1H), 1.87 - 1.75 (m, 1H), 1.02 (d, 6H).

### Example 66: (5S)-5-[[[6-[2-Chloro-3-[3-chloro-2-[3-[[2-hydroxyethyl(methyl)amino]methyl]-1-methyl-indol-6-yl]-4-pyridyl]phenyl]-2-methoxy-3-pyridyl]methylamino]methyl]pyrrolidin-2-one (39)

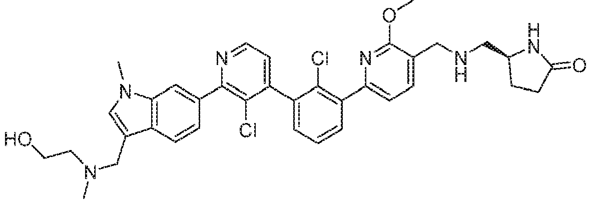

Utilizing Reductive Amination Procedure B from *tert*-butyl N-[[6-[2-chloro-3-[3-chloro-2-(3-formyl-1-methyl-indol-6-yl)-4-pyridyl]phenyl]-2-methoxy-3-pyridyl]methyl]-N-[[(2S)-5-oxopyrrolidin-2-yl]methyl]carbamate (25 mg, 0.03 mmol) and 2-(methylamino)ethanol (5 mg, 0.07 mmol) gave *tert*-butyl N-[[6-[2-chloro-3-[3-chloro-2-[3-[[2-hydroxyethyl(methyl)amino]methyl]-1-methyl-indol-6-yl]-4-pyridyl]phenyl]-2-methoxy-3-pyridyl]methyl]-N-[[(2S)-5-oxopyrrolidin-2-yl]methyl]carbamate (81% LCMS yield) as crude, MS: m/z found 773.3 [M+H]⁺, which was deprotected and purified by General Boc Deprotection Procedure to afford the final product as formic acid salt. 97% LCMS yield. MS: m/z found 673.2 [M+H]⁺. ¹H NMR (400 MHz, Methanol-*d*₄): δ 8.64 (d, 1H), 8.54 (s, 2H), 7.83 (d, 1H), 7.78 (d, 1H), 7.75 (d, 1H), 7.71 (dd, 1H), 7.55 (t, 1H), 7.53 - 7.47 (m, 2H), 7.45 - 7.39 (m, 2H), 7.26 (d, 1H), 4.37 (s, 2H), 4.02 (s, 3H), 3.91 (s, 3H), 3.85 (dd, 5H), 3.10 (t, 2H), 2.76 - 2.63 (m, 5H), 2.37 - 2.22 (m, 3H), 1.87 - 1.75 (m, 1H).

### Example 67: (5S)-5-[[[6-[2-Chloro-3-[3-chloro-2-[3-[[[(2S)-2-methoxypropyl]amino]methyl]-1-methyl-indol-6-yl]-4-pyridyl]phenyl]-2-methoxy-3-pyridyl]methylamino]methyl]pyrrolidin-2-one (40)

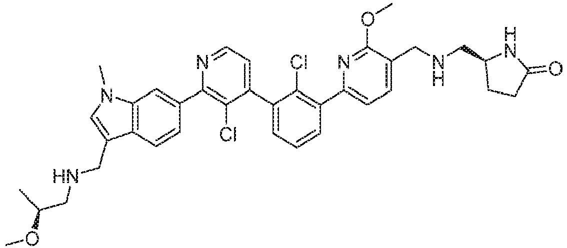

Utilizing Reductive Amination Procedure B from *tert*-butyl N-[[6-[2-chloro-3-[3-chloro-2-(3-formyl-1-methyl-indol-6-yl)-4-pyridyl]phenyl]-2-methoxy-3-pyridyl]methyl]-N-[[(2S)-5-oxopyrrolidin-2-yl]methyl]carbamate (25 mg, 0.03 mmol) and (2S)-2-methoxypropan-1-amine (6 mg, 0.07 mmol) gave *tert*-butyl N-[[6-[2-chloro-3-[3-chloro-2-[3-[[[(2S)-2-methoxypropyl]amino]methyl]-1-methyl-indol-6-yl]-4-pyridyl]phenyl]-2-methoxy-3-pyridyl]methyl]-N-[[(2S)-5-oxopyrrolidin-2-yl]methyl]carbamate (62% LCMS yield) as crude, MS: m/z found 787.3 [M+H]⁺, which was deprotected and purified by General Boc Deprotection Procedure to afford the final product as formic acid salt. 96% LCMS yield. MS: m/z found 687.3 [M+H]⁺. ¹H NMR (400 MHz, Methanol-*d*₄): δ 8.63 (d, 1H), 8.55 (s, 2H), 7.80 (d, 1H), 7.76 - 7.68 (m, 3H), 7.55 (t, 1H), 7.50 - 7.38 (m, 4H), 7.26 (d, 1H), 4.29 (s, 2H), 4.02 (s, 3H), 3.89 (s, 3H), 3.87 - 3.78 (m, 3H), 3.62 (ddd, 1H), 3.36 (s, 3H), 2.98 (dd, 1H), 2.86 (dd, , 1H), 2.76 - 2.63 (m, 2H), 2.37 - 2.22 (m, 3H), 1.89 - 1.75 (m, 1H), 1.16 (d, 3H).

### Example 68: (5S)-5-[[[6-[2-Chloro-3-[3-chloro-2-[1-methyl-3-[[[(2S)-tetrahydrofuran-2-yl]methylamino] methyl]indol-6-yl]-4-pyridyl]phenyl]-2-methoxy-3-pyridyl]methylamino]methyl]pyrrolidin-2-one (41)

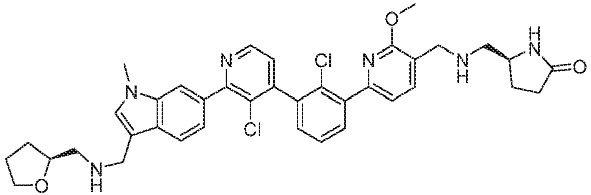

Reductive Amination Procedure B from *tert*-butyl N-[[6-[2-chloro-3-[3-chloro-2-(3-formyl-1-methyl-indol-6-yl)-4-pyridyl]phenyl]-2-methoxy-3-pyridyl]methyl]-N-[[(2S)-5-oxopyrrolidin-2-yl]methyl]carbamate (25 mg, 0.03 mmol) and [(2S)-tetrahydrofuran-2-yl]methanamine (7 mg, 0.07 mmol) gave *tert*-butyl N-[[6-[2-chloro-3-[3-chloro-2-[1-methyl-3-[[[(2S)-tetrahydrofuran-2-yl]methylamino]methyl]indol-6-yl]-4-pyridyl]phenyl]-2-methoxy-3-pyridyl]methyl]-N-[[(2S)-5-oxopyrrolidin-2-yl]methyl]carbamate (78% LCMS yield) as crude, MS: m/z found 799.3 [M+H]⁺, which was deprotected and purified by General Boc Deprotection Procedure to afford the final product as formic acid salt. 96% LCMS yield. MS: m/z found 699.3 [M+H]⁺. ¹H NMR (400 MHz, Methanol-*d*₄): δ 8.63 (d, 1H), 8.55 (s, 2H), 7.85 - 7.79 (m, 1H), 7.78 - 7.68 (m, 3H), 7.55 (t, 1H), 7.51 - 7.46 (m, 2H), 7.45 - 7.39 (m, 2H), 7.26 (d, 1H), 4.43 - 4.29 (m, 2H), 4.14 (dtd, 1H), 4.02 (s, 3H), 3.93 - 3.76 (m, 8H), 3.09 (dd, 1H), 2.93 (dd, 1H), 2.77 - 2.63 (m, 2H), 2.38 - 2.21 (m, 3H), 2.15 - 2.02 (m, 1H), 1.99 - 1.89 (m, 2H), 1.82 (m, 1H), 1.58 (m, 1H).

### Example 69: (5S)-5-[[[6-[2-Chloro-3-[3-chloro-2-[1-methyl-3-[(tetrahydropyran-4-ylamino)methyl]indol-6-yl]-4-pyridyl]phenyl]-2-methoxy-3-pyridyl]methylamino]methyl]pyrrolidin-2-one (42)

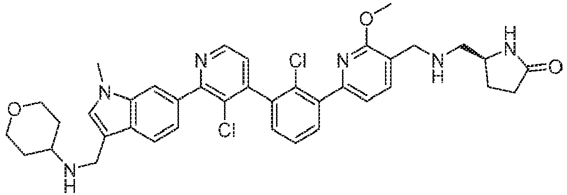

Reductive Amination Procedure B from *tert*-butyl N-[[6-[2-chloro-3-[3-chloro-2-(3-formyl-1-methyl-indol-6-yl)-4-pyridyl]phenyl]-2-methoxy-3-pyridyl]methyl]-N-[[(2S)-5-oxopyrrolidin-2-yl]methyl]carbamate (25 mg, 0.03 mmol) and tetrahydropyran-4-amine (7 mg, 0.07 mmol) gave *tert*-butyl N-[[6-[2-chloro-3-[3-chloro-2-[1-methyl-3-[(tetrahydropyran-4-ylamino)methyl]indol-6-yl]-4-pyridyl]phenyl]-2-methoxy-3-pyridyl]methyl]-N-[[(2S)-5-oxopyrrolidin-2-yl]methyl]carbamate (75% LCMS yield) as crude, MS: m/z found 799.3 [M+H]⁺, which was deprotected and purified by General Boc Deprotection Procedure to afford the final product as formic acid salt. 97% LCMS yield. MS: *m*/*z* found 699.3 [M+H]⁺. ¹H NMR (400 MHz, Methanol-*d*₄): δ 8.63 (d, 1H), 8.54 (s, 2H), 7.80 (d, 1H), 7.75 (dd, 2H), 7.71 (dd, 1H), 7.55 (t, 1H), 7.49 - 7.38 (m, 4H), 7.26 (d, 1H), 4.30 (s, 2H), 4.05 - 3.97 (m, 5H), 3.89 (s, 3H), 3.88 - 3.78 (m, 3H), 3.44 (td, 2H), 3.26 - 3.15 (m, 1H), 2.76 - 2.63 (m, 2H), 2.37 - 2.23 (m, 3H), 2.12 - 2.00 (m, 2H), 1.88 - 1.76 (m, 1H), 1.63 (m, 2H).

### Example 70: (5S)-5-[[[6-[2-Chloro-3-[3-chloro-2-[3-[[(1,1-dioxothian-4-yl)amino]methyl]-1-methyl-indol-6-yl]-4-pyridyl]phenyl]-2-methoxy-3-pyridyl]methylamino]methyl]pyrrolidin-2-one (43)

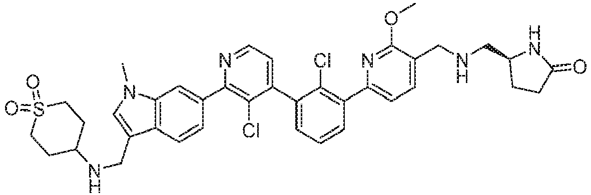

Reductive Amination Procedure B from *tert*-butyl N-[[6-[2-chloro-3-[3-chloro-2-(3-formyl-1-methyl-indol-6-yl)-4-pyridyl]phenyl]-2-methoxy-3-pyridyl]methyl]-N-[[(2S)-5-oxopyrrolidin-2-yl]methyl]carbamate (25 mg, 0.03 mmol) and 1,1-dioxothian-4-amine (10 mg, 0.07 mmol) gave *tert*-butyl N-[[6-[2-chloro-3-[3-chloro-2-[3-[[(1,1-dioxothian-4-yl)amino]methyl]-1-methyl-indol-6-yl]-4-pyridyl]phenyl]-2-methoxy-3-pyridyl]methyl]-N-[[(2S)-5-oxopyrrolidin-2-yl]methyl]carbamate (84% LCMS yield) as crude, MS: m/z found 847.3 [M+H]⁺, which was deprotected and purified by General Boc Deprotection Procedure to afford the final product as formic acid salt. 98% LCMS yield. MS: m/z found 747.2 [M+H]⁺. ¹H NMR (400 MHz, Methanol-*d*₄): δ 8.63 (d, 1H), 8.55 (s, 2H), 7.79 (d, 1H), 7.76 (d, 1H), 7.73 (d, 1H), 7.71 (dd 1H), 7.55 (t, 1H), 7.48 - 7.37 (m, 4H), 7.27 (d, 1H), 4.21 (s, 2H), 4.03 (s, 3H), 3.94 - 3.78 (m, 6H), 3.25 - 3.07 (m, 5H), 2.82 - 2.67 (m, 2H), 2.46 - 2.37 (m, 2H), 2.37 - 2.23 (m, 3H), 2.19 - 2.08 (m, 2H), 1.88 - 1.76 (m, 1H).

### Example 71: (5S)-5-[[[6-[3-[2-[3-[[(1-Acetylazetidin-3-yl)amino]methyl]-1-methyl-indol-6-yl]-3-chloro-4-pyridyl]-2-chloro-phenyl]-2-methoxy-3-pyridyl]methylamino]methyl]pyrrolidin-2-one (55)

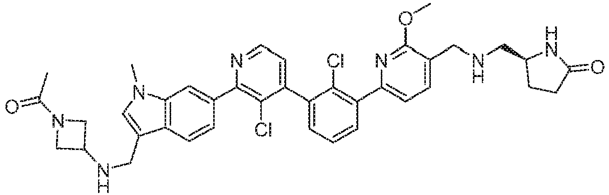

Reductive Amination Procedure B from *tert*-butyl N-[[6-[2-chloro-3-[3-chloro-2-(3-formyl-1-methyl-indol-6-yl)-4-pyridyl]phenyl]-2-methoxy-3-pyridyl]methyl]-N-[[(2S)-5-oxopyrrolidin-2-yl]methyl]carbamate (30 mg, 0.04 mmol) and 1-(3-aminoazetidin-1-yl)ethanone (10 mg, 0.08 mmol) gave *tert*-butyl N-[[6-[3-[2-[3-[[(1-acetylazetidin-3-yl)amino]methyl]-1-methyl-indol-6-yl]-3-chloro-4-pyridyl]-2-chloro-phenyl]-2-methoxy-3-pyridyl]methyl]-N-[[(2S)-5-oxopyrrolidin-2-yl]methyl]carbamate (88% LCMS yield) as crude, MS: m/z found 812.3 [M+H]⁺, which was deprotected and purified by General Boc Deprotection Procedure to afford the final product as formic acid salt. 84% LCMS yield. MS: m/z found 712.3 [M+H]⁺. ¹H NMR (400 MHz, Methanol-*d*₄): δ 8.62 (d, 1H), 8.54 (s, 2H), 7.79 - 7.77 (m, 1H), 7.77 - 7.74 (m, 1H), 7.71 (td, 2H), 7.55 (t, 1H), 7.45 - 7.38 (m, 3H), 7.31 (s, 1H), 7.27 (d, 1H), 4.31 (dd, 1H), 4.09 (dd, 1H), 4.03 (s, 3H), 4.02 (s, 2H), 3.92 (dd, 1H), 3.90 - 3.87 (m, 2H), 3.87 - 3.83 (m, 4H), 3.79 (m, 1H), 3.70 (dd, *J* = 10.2, 5.1 Hz, 1H), 2.82 - 2.68 (m, 2H), 2.38 - 2.24 (m, 3H), 1.90 - 1.77 (m, 4H).

### Example 72: (5S)-5-[[[6-[3-[2-[3-[[(1-acetyl-4-piperidyl)-methyl-amino]methyl]-1-methyl-indol-6-yl]-3-chloro-4-pyridyl]-2-chloro-phenyl]-2-methoxy-3-pyridyl]methylamino]methyl]pyrrolidin-2-one (56)

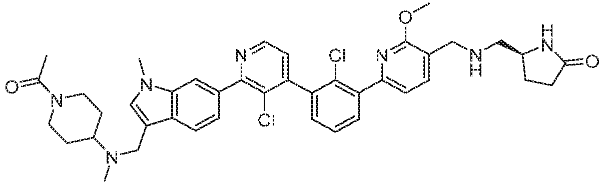

Reductive Amination Procedure B from *tert*-butyl N-[[6-[2-chloro-3-[3-chloro-2-(3-formyl-1-methyl-indol-6-yl)-4-pyridyl]phenyl]-2-methoxy-3-pyridyl]methyl]-N-[[(2S)-5-oxopyrrolidin-2-yl]methyl]carbamate (40 mg, 0.06 mmol) and 1-[4-(methylamino)-1-piperidyl]ethanone (17 mg, 0.11 mmol) gave *tert*-butyl N-[[6-[3-[2-[3-[[(1-acetyl-4-piperidyl)-methyl-amino]methyl]-1-methyl-indol-6-yl]-3-chloro-4-pyridyl]-2-chloro-phenyl]-2-methoxy-3-pyridyl]methyl]-N-[[(2S)-5-oxopyrrolidin-2-yl]methyl]carbamate (83% LCMS yield) as crude, MS: m/z found 854.4 [M+H]⁺, which was deprotected and purified by General Boc Deprotection Procedure to afford the final product as formic acid salt. 97% LCMS yield. MS: m/z found 754.3 [M+H]⁺. ¹H NMR (400 MHz, Methanol-*d*₄): δ 8.64 (d, 1H), 8.55 (s, 2H), 7.81 (d, 1H), 7.79 - 7.73 (m, 2H), 7.71 (dd, 1H), 7.55 (t, 1H), 7.52 - 7.46 (m, 2H), 7.45 - 7.40 (m, 2H), 7.26 (d, 1H), 4.70 (d, 1H), 4.34 (s, 2H), 4.15 - 3.95 (m, 5H), 3.91 (s, 3H), 3.89 - 3.79 (m, 3H), 3.15 (t, 1H), 2.79 - 2.69 (m, 2H), 2.63 (s, 4H), 2.38 - 2.22 (m, 3H), 2.21 - 2.09 (m, 5H), 1.88 - 1.75 (m, 2H), 1.72 - 1.59 (m, 1H).

### Example 73: (5S)-5-[[[6-[2-Chloro-3-[3-chloro-2-[3-[[(4,4-difluorocyclohexyl)amino]methyl]-1-methyl-indol-6-yl]-4-pyridyl]phenyl]-2-methoxy-3-pyridyl]methylamino]methyl]pyrrolidin-2-one (73)

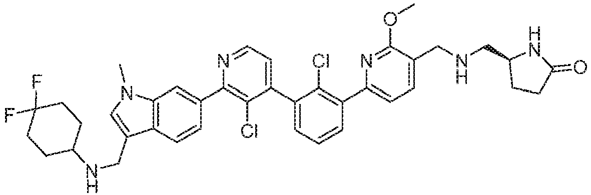

Reductive Amination Procedure B from *tert*-butyl N-[[6-[2-chloro-3-[3-chloro-2-(3-formyl-1-methyl-indol-6-yl)-4-pyridyl]phenyl]-2-methoxy-3-pyridyl]methyl]-N-[[(2S)-5-oxopyrrolidin-2-yl]methyl]carbamate (20 mg, 0.03 mmol) and 4,4-difluorocyclohexanamine (8 mg, 0.06 mmol) gave *tert*-butyl N-[[6-[2-chloro-3-[3-chloro-2-[3-[[(4,4-difluorocyclohexyl)amino]methyl]-1-methyl-indol-6-yl]-4-pyridyl]phenyl]-2-methoxy-3-pyridyl]methyl]-N-[[(2S)-5-oxopyrrolidin-2-yl]methyl]carbamate (60% LCMS yield) as crude, MS: m/z found 833.3 [M+H]⁺, which was deprotected by General Boc Deprotection Procedure and purified to afford the final product as formic acid salt. 97% LCMS yield. MS: m/z found 733.3 [M+H]⁺. ¹H NMR (400 MHz, Methanol-d4): δ 8.64 (d, 1H), 8.54 (s, 2H), 7.83 (d, 1H), 7.79 - 7.74 (m, 2H), 7.71 (dd, 1H), 7.59 - 7.47 (m, 3H), 7.46 - 7.37 (m, 2H), 7.26 (d, 1H), 4.46 (s, 2H), 4.03 (s, 3H), 3.91 (s, 3H), 3.89 - 3.78 (m, 3H), 3.38 - 3.19 (m, 1H), 2.79 - 2.64 (m, 2H), 2.38 - 2.25 (m, 5H), 2.25 - 2.13 (m, 2H), 2.02 - 1.67 (m, 5H).

### Example 74: (5S)-5-[[[6-[2-Chloro-3-(3-chloro-2-isoindolin-5-yl-4-pyridyl)phenyl]-2-methoxy-3-pyridyl]methylamino]methyl]pyrrolidin-2-one (116)

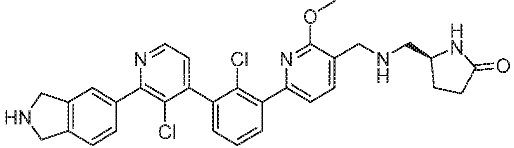

Following Suzuki Coupling Procedure B from *tert*-butyl 5-(4,4,5,5-tetramethyl-1,3,2-dioxaborolan-2-yl)isoindoline-2-carboxylate (30 mg, 0.09 mmol) and *tert*-butyl N-[[6-[2-chloro-3-(2,3-dichloro-4-pyridyl)phenyl]-2-methoxy-3-pyridyl]methyl]-N-[[(2S)-5-oxopyrrolidin-2-yl]methyl]carbamate (51 mg, 0.09 mmol) provided *tert*-butyl 5-[4-[3-[5-[[*tert*-butoxycarbonyl-[[(2S)-5-oxopyrrolidin-2-yl]methyl]amino]methyl]-6-methoxy-2-pyridyl]-2-chloro-phenyl]-3-chloro-2-pyridyl]isoindoline-2-carboxylate (55 mg, 81.7%). MS: m/z found 774.3 [M+H]⁺. The Boc deprotection of the Suzuki product and purification afforded the final product as formic acid salt. 98% yield for the deprotection step but isolated yield for formic acid salt form is 21%. MS: m/z found 574.2 [M+H]⁺. ¹H NMR (400 MHz, Methanol-*d*₄): δ 8.65 (d, 1H), 8.51 (br s, 2H), 7.80 - 7.75 (m, 1H), 7.75 - 7.69 (m, 3H), 7.55 (t, 2H), 7.47 (d, 1H), 7.41 (d, 1H), 7.27 (d, 1H), 5.50 (s, 1H), 4.68 (s, 4H), 4.03 (s, 3H), 3.96 - 3.82 (m, 3H), 2.85 - 2.70 (m, 2H), 2.39 - 2.23 (m, 3H), 1.89 - 1.76 (m, 1H).

### Example 75: (5S)-5-[[[6-[3-[2-[1-(Azetidin-3-yl)pyrazol-4-yl]-3-chloro-4-pyridyl]-2-chloro-phenyl]-2-methoxy-3-pyridyl]methylamino]methyl]pyrrolidin-2-one (119)

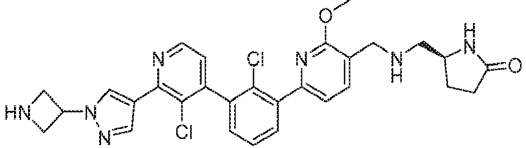

Following Suzuki Coupling Procedure B from *tert*-butyl 3-[4-(4,4,5,5-tetramethyl-1,3,2-dioxaborolan-2-yl)pyrazol-1-yl]azetidine-1-carboxylate (30 mg, 0.09 mmol) and *tert-*butyl N-[[6-[2-chloro-3-(2,3-dichloro-4-pyridyl)phenyl]-2-methoxy-3-pyridyl]methyl]-N-[[(2S)-5-oxopyrrolidin-2-yl]methyl]carbamate (51 mg, 0.09 mmol) provided *tert*-butyl 3-[4-[4-[3-[5-[[tert-butoxycarbonyl-[[(2S)-5-oxopyrrolidin-2-yl]methyl]amino]methyl]-6-methoxy-2-pyridyl]-2-chloro-phenyl]-3-chloro-2-pyridyl]pyrazol-1-yl]azetidine-1-carboxylate (55 mg, 82%). MS: m/z found 778.3 [M+H]⁺. The Boc deprotection of the Suzuki product and purification afforded the final product as formic acid salt. 97% yield for the deprotection step but isolated yield for formic acid salt form is 56%. MS: m/z found 578.2 [M+H]⁺. ¹H NMR (400 MHz, Methanol-*d*₄): δ 8.58 (d, 1H), 8.50 (d, 2H), 8.40 (s, 1H), 7.78 (d, 1H), 7.71 (dd, 1H), 7.54 (t, 1H), 7.37 (dd, 1H), 7.30 - 7.24 (m, 2H), 5.52 (p, 1H), 4.63 - 4.45 (m, 4H), 4.04 (s, 3H), 4.02 - 3.83 (m, 3H), 2.93 - 2.72 (m, 2H), 2.42 - 2.23 (m, 3H), 1.93 - 1.77 (m, 1H).

### Example 76: (5S)-5-[[[6-[3-[2-[1-(Azetidin-3-ylmethyl)pyrazol-4-yl]-3-chloro-4-pyridyl]-2-chloro-phenyl]-2-methoxy-3-pyridyl]methylamino]methyl]pyrrolidin-2-one (120)

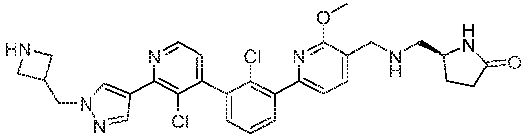

Following Suzuki Coupling Procedure B from *tert*-butyl 3-[[4-(4,4,5,5-tetramethyl-1,3,2-dioxaborolan-2-yl)pyrazol-1-yl]methyl]azetidine-1-carboxylate (30 mg, 0.08 mmol) and *tert*-butyl N-[[6-[2-chloro-3-(2,3-dichloro-4-pyridyl)phenyl]-2-methoxy-3-pyridyl]methyl]-N-[[(2S)-5-oxopyrrolidin-2-yl]methyl]carbamate (49 mg, 0.08 mmol) provided *tert*-butyl 3-[[4-[4-[3-[5-[[*tert*-butoxycarbonyl-[[(2S)-5-oxopyrrolidin-2-yl]methyl]amino]methyl]-6-methoxy-2-pyridyl]-2-chloro-phenyl]-3-chloro-2-pyridyl]pyrazol-1-yl]methyl]azetidine-1-carboxylate (56 mg, 84.0%). MS: m/z found 792.3 [M+H]⁺. The Boc deprotection of the Suzuki product and purification afforded the final product as formic acid salt. 98% yield for the deprotection step but isolated yield for formic acid salt form is 67%. MS: m/z found 592.2 [M+H]⁺. ¹H NMR (400 MHz, Methanol-*d*₄): δ 8.57 (d, 1H), 8.46 (d, 3H), 8.26 (s, 1H), 7.80 (d, 1H), 7.71 (dd, 1H), 7.55 (t, 1H), 7.38 (dd, 1H), 7.32 - 7.22 (m, 2H), 4.55 - 4.43 (m, 2H), 4.19 - 4.05 (m, 4H), 4.05 (s, 3H), 4.01 (d, 2H), 3.94 - 3.87 (m, 1H), 3.49 (p, 1H), 2.96 - 2.79 (m, 2H), 2.43 - 2.27 (m, 3H), 1.94 - 1.77 (m, 1H).

### Example 77: (S)-5-((((4-Chloro-6-(2-chloro-3-(3-chloro-2-(4-((((S)-2-hydroxypropyl)(methyl)amino)methyl)-3-methoxyphenyl)pyridin-4-yl)phenyl)-2-methoxypyridin-3-yl)methyl)amino)methyl)pyrrolidin-2-one (71)

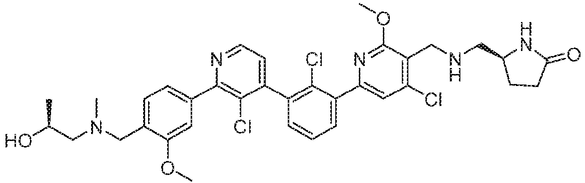

### (a) 2-Methoxy-4-(4,4,5,5-tetramethyl-1,3,2-dioxaborolan-2-yl)benzaldehyde

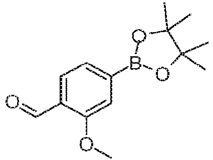

To a mixture of 4-bromo-2-methoxy-benzaldehyde (5.0 g, 23.2 mmol) and bis(pinacolato)diboron (11.8 g, 46.50 mmol) in dioxane (50 mL) was added potassium acetate (6.85 g, 69.7 mmol) and [1,1'-bis(di-*tert-*butylphosphino)ferrocene]dichloropalladium(II) (1.9 g, 2.33 mmol). The mixture was stirred at 95 °C for 5 h under nitrogen atmosphere. The mixture was filtered. The concentrated filtrate was purified by normal phase SiO₂ chromatography (8-50% ethyl acetate/petroleum ether) to afford 2-methoxy-4-(4,4,5,5-tetramethyl-1,3,2-dioxaborolan-2-yl)benzaldehyde (4.4 g, 72%) as a yellow solid. ¹H NMR (400 MHz, DMSO-d₆): δ 8.40 (s, 1H), 7.70 (d, *J* = 7.6 Hz, 1H), 7.38-7.36 (m, 2H), 3.95 (s, 3H), 1.33 (s, 12H).

### (b) (S)-tert-Butyl((4-chloro-6-(2-chloro-3-(3-chloro-2-(4-formyl-3-methoxyphenyl)pyridin-4-yl)phenyl)-2-methoxypyridin-3-yl)methyl)((5-oxopyrrolidin-2-yl)methyl)carbamate

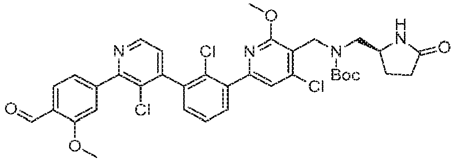

To a mixture of (S)-*tert*-butyl ((4-chloro-6-(2-chloro-3-(2,3-dichloropyridin-4-yl)phenyl)-2-methoxypyridin-3-yl)methyl)((5-oxopyrrolidin-2-yl)methyl)carbamate (as described for compound 37 (500 mg, 0.80 mmol) and 2-methoxy-4-(4,4,5,5-tetramethyl-1,3,2-dioxaborolan-2-yl)benzaldehyde (314 mg, 1.20 mmol) in THF/H₂O (6:1 v/v, 28 mL) was added potassium phosphate (508 mg, 2.39 mmol) and [1,1'-bis(di-tert-butylphosphino)ferrocene] dichloropalladium(II) (52 mg, 0.08 mmol). The mixture was stirred at 80 °C for 1 h under nitrogen atmosphere. The reaction mixture was diluted with water (20 mL) and the mixture was extracted with ethyl acetate (2 x 50 mL). The combined organics were dried over sodium sulfate, filtered and concentrated. The crude material was purified by flash chromatography (SiO₂, 0-3% ethyl acetate/petroleum ether) to afford (S)-*tert*-butyl ((4-chloro-6-(2-chloro-3-(3-chloro-2-(4-formyl-3-methoxyphenyl)pyridin-4-yl)phenyl)-2-methoxypyridin-3-yl)methyl)((5-oxopyrrolidin-2-yl)methyl)carbamate (360 mg) as a yellow solid. MS: m/z found 725 [M+H]⁺.

### (c) tert-Butyl((4-chloro-6-(2-chloro-3-(3-chloro-2-(4-((((S)-2-hydroxypropyl)(methyl)amino)methyl)-3-methoxyphenyl)pyridin-4-yl)phenyl)-2-methoxypyridin-3-yl)methyl)(((S)-5-oxopyrrolidin-2-yl)methyl)carbamate

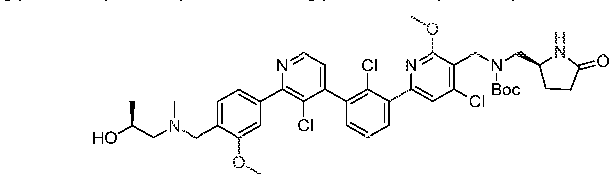

To a mixture of (S)-*tert*-butyl ((4-chloro-6-(2-chloro-3-(3-chloro-2-(4-formyl-3-methoxyphenyl)pyridin-4-yl)phenyl)-2-methoxypyridin-3-yl)methyl)((5-oxopyrrolidin-2-yl)methyl)carbamate (200 mg, 0.28 mmol) and (S)-1-(methylamino)propan-2-ol (49.1 mg, 0.55 mmol) in methylene chloride (10 mL) was added sodium acetate (67.8 mg, 0.83 mmol). The mixture was stirred at RT for 12 h under N₂. Sodium cyanoborohydride (51.9 mg, 0.83 mmol) was added and the mixture was stirred at RT for 0.5 h under N₂. The mixture was concentrated, and the resulting crude material was purified by preparative TLC (SiO₂, 4:1 ethyl acetate:MeOH) to afford *tert*-butyl((4-chloro-6-(2-chloro-3-(3-chloro-2-(4-((((S)-2-hydroxypropyl)(methyl)amino)methyl)-3-methoxyphenyl)pyridin-4-yl)phenyl)-2-methoxypyridin-3-yl)methyl)(((S)-5-oxopyrrolidin-2-yl)methyl)carbamate (110 mg, 50%) as a white solid. MS: m/z found 798 [M+H]⁺.

### (d) (S)-5-((((4-Chloro-6-(2-chloro-3-(3-chloro-2-(4-((((S)-2-hydroxypropyl)(methyl)amino)methyl)-3-methoxyphenyl)pyridin-4-yl)phenyl)-2-methoxypyridin-3-yl)methyl)amino)methyl)pyrrolidin-2-one

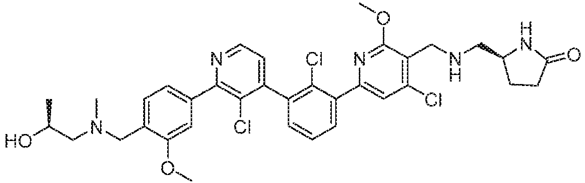

To a solution of tert-butyl((4-chloro-6-(2-chloro-3-(3-chloro-2-(4-((((S)-2-hydroxypropyl)(methyl)amino)methyl)-3-methoxyphenyl)pyridin-4-yl)phenyl)-2-methoxypyridin-3-yl)methyl)(((S)-5-oxopyrrolidin-2-yl)methyl)carbamate (100 mg, 0.12 mmol) in methylene chloride (5 mL) was added TFA (5 mL, 67.5 mmol). The mixture was stirred at RT for 20 min under N₂. The mixture was concentrated. The crude product was purified by reverse phase HPLC to afford (S)-5-((((4-chloro-6-(2-chloro-3-(3-chloro-2-(4-((((S)-2-hydroxypropyl)(methyl)amino)methyl)-3-methoxyphenyl)pyridin-4-yl)phenyl)-2-methoxypyridin-3-yl)methyl)amino)methyl)pyrrolidin-2-one as formic acid salt (14.5 mg, 15% yield) as a white solid. LCMS: m/z found 700.1 [M+H]⁺, RT = 2.98 min, (Method A); ¹H NMR (400 MHz, MeOH-d₄): δ 8.60 (d, *J* = 4.8 Hz, 1H), 7.75 (dd, *J*₁ = 1.6 Hz, *J*₂ = 7.6 Hz, 1H), 7.60-7.55 (m, 2H), 7.51-7.44 (m, 3H), 7.42-7.38 (m, 2H), 4.60-4.53 (m, 1H), 4.42-4.41 (m, 1H), 4.23-4.20 (m, 1H), 4.10-4.07 (m, 5H), 4.02 (s, 3H), 3.88-3.87 (m, 1H), 3.24-3.21 (m, 1H), 3.10-3.04 (m, 1H), 2.88 (s, 3H), 2.79-2.76 (m, 2H), 2.38-2.27 (m, 3H), 1.86-1.81 (m, 1H), 1.26 (d, *J =* 6 Hz, 3H).

### Example 78: (S)-(4-(3-chloro-4-(2-chloro-3-(4-chloro-6-methoxy-5-((((5-oxopyrrolidin-2-yl)methyl)amino)methyl)pyridin-2-yl)phenyl)pyridin-2-yl)-2-methoxybenzyl)glycine (105)

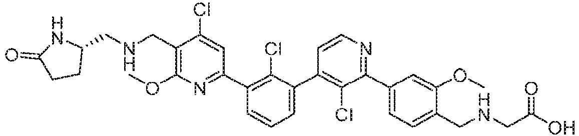

### (a) (S)-2-((4-(4-(3-(5-(((tert-Butoxycarbonyl)((5-oxopyrrolidin-2-yl)methyl)amino)methyl)-4-chloro-6-methoxypyridin-2-yl)-2-chlorophenyl)-3-chloropyridin-2-yl)-2-methoxybenzyl)amino)acetic acid

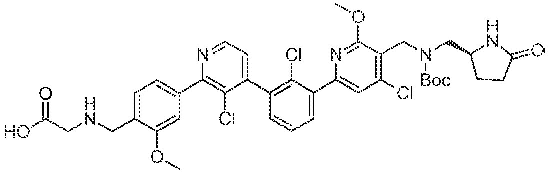

To a mixture of (S)-tert-butyl ((4-chloro-6-(2-chloro-3-(3-chloro-2-(4-formyl-3-methoxyphenyl)pyridin-4-yl)phenyl)-2-methoxypyridin-3-yl)methyl)((5-oxopyrrolidin-2-yl)methyl)carbamate (75 mg, 0.1 mmol) and glycine (15 mg, 0.21 mmol) in methylene chloride (2 mL) was added sodium acetate (25 mg, 0.31 mmol) in one portion under N₂ atmosphere. The mixture was stirred at RT for 3 h. Sodium cyanoborohydride (20 mg, 0.31 mmol) was added. The mixture was stirred at RT for 12 h. Methanol (2 mL) was added, and the mixture was stirred at RT for 1 h. The mixture was concentrated. The residue was purified by preparative TLC (silica gel, 3:1 ethyl acetate: MeOH ) to afford (S)-2-((4-(4-(3-(5-(((*tert-*butoxycarbonyl)((5-oxopyrrolidin-2-yl)methyl)amino)methyl)-4-chloro-6-methoxypyridin-2-yl)-2-chlorophenyl)-3-chloropyridin-2-yl)-2-methoxybenzyl)amino)acetic acid as a yellow solid (15 mg, 16% yield). MS: m/z found 784 [M+H]+.

### (b) (S)-2-((4-(3-Chloro-4-(2-chloro-3-(4-chloro-6-methoxy-5-((((5-oxopyrrolidin-2-yl)methyl)amino)methyl)pyridin-2-yl)phenyl)pyridin-2-yl)-2-methoxybenzyl)amino)acetic acid

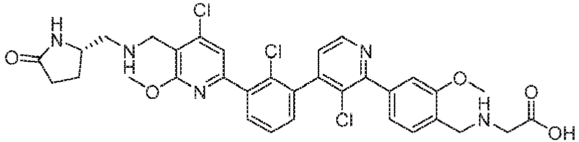

To a mixture of (S)-2-((4-(4-(3-(5-(((*tert*-butoxycarbonyl)((5-oxopyrrolidin-2-yl)methyl)amino)methyl)-4-chloro-6-methoxypyridin-2-yl)-2-chlorophenyl)-3-chloropyridin-2-yl)-2-methoxybenzyl)amino)acetic acid (14 mg, 0.018 mmol) in methylene chloride (0.1 mL) was added trifluoroacetic acid (0.7 mL) in one portion. The mixture was stirred at RT for 5 min. The mixture was concentrated. The residue was purified by reverse phase HPLC to afford (S)-2-((4-(3-chloro-4-(2-chloro-3-(4-chloro-6-methoxy-5-((((5-oxopyrrolidin-2-yl)methyl)amino)methyl)pyridin-2-yl)phenyl)pyridin-2-yl)-2-methoxybenzyl)amino)acetic acid as formic salt (3.1 mg, 23% yield) as a white solid. MS: m/z found 684 [M+H]+; ¹H NMR (400 MHz, Methanol-d4): δ 8.55 (d, 1H), 7.63 (m, 1H), 7.48 (t, 1H), 7.41 (d, 1H), 7.37-7.35 (m, 2H), 7.30-7.29 (m, 2H), 7.24 (m, 1H), 4.22 (s, 2H), 4.07 (s, 2H), 3.97 (s, 3H), 3.90 (s, 3H), 3.82-3.79 (m, 1H), 3.42 (s, 2H), 2.83-2.73 (m, 2H), 2.30-2.19 (m, 3H), 1.79-1.70 (m, 1H).

### Example 79: (S)-Isopropyl 2-((4-(3-chloro-4-(2-chloro-3-(4-chloro-6-methoxy-5-((((5-oxopyrrolidin-2-yl)methyl)amino)methyl)pyridin-2-yl)phenyl)pyridin-2-yl)-2-methoxybenzyl)amino)acetate (123)

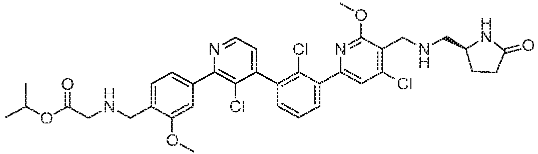

(S)-Isopropyl 2-((4-(3-chloro-4-(2-chloro-3-(4-chloro-6-methoxy-5-((((5-oxopyrrolidin-2-yl)methyl)amino)methyl)pyridin-2-yl)phenyl)pyridin-2-yl)-2-methoxybenzyl)amino)acetate was prepared in a similar fashion to compound 105, using (S)-*tert-butyl* ((4-chloro-6-(2-chloro-3-(3-chloro-2-(4-formyl-3-methoxyphenyl)pyridin-4-yl)phenyl)-2-methoxypyridin-3-yl)methyl)((5-oxopyrrolidin-2-yl)methyl)carbamate and isopropyl 2-aminoacetate. LCMS: m/z found 728.0 [M+H]+, RT = 3.32 min, (Method A); ¹H NMR (400 MHz, Methanol-d4): δ 8.64 (d, 1H), 7.74 (m, 1H), 7.58 (t, 1H), 7.47-7.44 (m, 2H), 7.41-7.37 (m, 2H), 7.29-7.27 (m, 2H), 5.08-5.02 (m, 1H), 4.06-4.03 (m, 5H), 3.94 (s, 3H), 3.88-3.83 (m, 3H), 3.37 (s, 2H), 2.73-2.68 (m, 2H), 2.37-2.26 (m, 3H), 1.87-1.80 (m, 1H), 1.27 (d, 6H).

### Example 80: (S)-5-((((6-(2-Chloro-3-(3-chloro-2-(4-((((S)-2-hydroxypropyl)(methyl)amino)methyl)-3-methoxyphenyl)pyridin-4-yl)phenyl)-2-methoxypyridin-3-yl)methyl)amino)methyl)pyrrolidin-2-one (75)

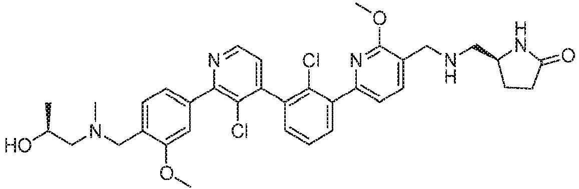

### (a) (S)-tert-Butyl ((6-(2-chloro-3-(3-chloro-2-(4-formyl-3-methoxyphenyl)pyridin-4-yl)phenyl)-2-methoxypyridin-3-yl)methyl)((5-oxopyrrolidin-2-yl)methyl)carbamate

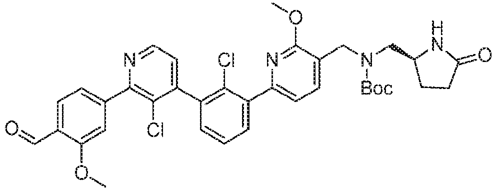

To a mixture of *tert*-butyl(S)-tert-butyl((6-(2-chloro-3-(2,3-dichloropyridin-4-yl)phenyl)-2-methoxypyridin-3-yl)methyl)((5-oxopyrrolidin-2-yl)methyl)carbamate (2.00 g, 3.38 mmol), 2-methoxy-4-(4,4,5,5-tetramethyl-1,3,2-dioxaborolan-2-yl)benzaldehyde) (1.51 g, 5.74 mmol) in THF (40 mL) and H₂O (6 mL) was added potassium phosphate (2.20 g, 10 mmol) and [1,1'-bis(di-*tert*-butylphosphino)ferrocene]dichloropalladium(II) (0.22 g, 0.33 mmol). The mixture was stirred at 80 °C for 2 h under N₂. The mixture was filtered and concentrated. The residue was purified by flash chromatography (SiO₂, 0-8% ethyl acetate/petroleum ether) to afford (S)-tert-butyl ((6-(2-chloro-3-(3-chloro-2-(4-formyl-3-methoxyphenyl)pyridin-4-yl)phenyl)-2-methoxypyridin-3-yl)methyl)((5-oxopyrrolidin-2-yl)methyl)carbamate (1.80 g, 2.60 mmol) as a yellow solid.

### (b) tert-Butyl ((6-(2-chloro-3-(3-chloro-2-(4-((((S)-2-hydroxypropyl)(methyl)amino)methyl)-3-methoxyphenyl)pyridin-4-yl)phenyl)-2-methoxypyridin-3-yl)methyl)(((S)-5-oxopyrrolidin-2-yl)methyl)carbamate

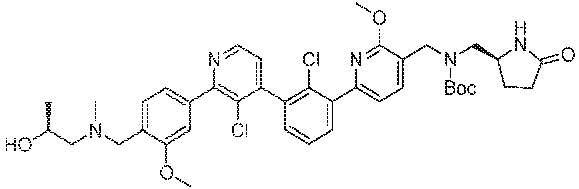

To a mixture of (S)-*tert*-butyl ((6-(2-chloro-3-(3-chloro-2-(4-formyl-3-methoxyphenyl)pyridin-4-yl)phenyl)-2-methoxypyridin-3-yl)methyl)((5-oxopyrrolidin-2-yl)methyl)carbamate (200 mg, 0.29 mmol) and (2S)-1-(methylamino)propan-2-ol (75 mg, 0.87 mmol) in methylene chloride (10 mL) was added sodium acetate (71 mg, 0.87 mmol) and sodium triacetoxyborohydride (54.5mg, 0.87 mmol). The mixture was stirred at 20 °C for 4 h under N₂. The filtrate was concentrated. The residue was purified by preparative TLC (SiO₂, 25% MeOH/ethyl acetate) to provide *tert*-butyl ((6-(2-chloro-3-(3-chloro-2-(4-((((S)-2-hydroxypropyl)(methyl)amino)methyl)-3-methoxyphenyl)pyridin-4-yl)phenyl)-2-methoxypyridin-3-yl)methyl)(((S)-5-oxopyrrolidin-2-yl)methyl)carbamate (130 mg, 59% yield) as a white solid. MS: m/z: 764 [M+H]⁺ observed.

### (c) (S)-5-((((6-(2-Chloro-3-(3-chloro-2-(4-((((S)-2-hydroxypropyl)(methyl)amino)methyl)-3-methoxyphenyl)pyridin-4-yl)phenyl)-2-methoxypyridin-3-yl)methyl)amino)methyl)pyrrolidin-2-one

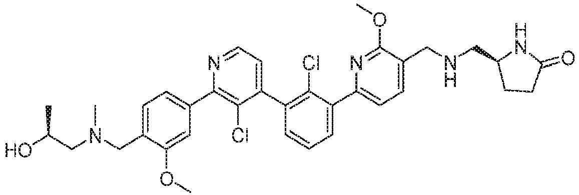

To a solution of *tert-butyl* ((6-(2-chloro-3-(3-chloro-2-(4-((((S)-2-hydroxypropyl)(methyl)amino)methyl)-3-methoxyphenyl)pyridin-4-yl)phenyl)-2-methoxypyridin-3-yl)methyl)(((S)-5-oxopyrrolidin-2-yl)methyl)carbamate (115 mg, 0.15 mmol) in methylene chloride (9 mL) was added trifluoroacetic acid (2 mL, 27 mmol). The mixture was stirred at 20 °C for 20 min under N₂. The mixture was concentrated and the resulting residue was purified by reverse phase HPLC to afford (S)-5-((((6-(2-chloro-3-(3-chloro-2-(4-((((S)-2-hydroxypropyl)(methyl)amino)methyl)-3-methoxyphenyl)pyridin-4-yl)phenyl)-2-methoxypyridin-3-yl)methyl)amino)methyl)pyrrolidin-2-one as formic acid salt (6.4 mg, 5.7% yield) as a white solid. LCMS: m/z found 664.0 [M+H]+, RT = 2.79 min, (Method A); ¹H NMR (400 MHz, Methanol-d₄): δ 8.66 (d, *J =* 4.8 Hz, 1H), 7.76 (d, *J =* 7.6 Hz, 1H), 7.71 (m, 1H), 7.58-7.53 (m, 2H), 7.48 (d, 1H), 7.43-7.42 (m, 2H), 7.37 (m, 1H), 7.27 (d, 1H), 4.52-4.51 (m, 2H), 4.23-4.21 (m, 1H), 4.03 (s, 3H), 4.00 (s, 3H), 3.98 (m, 2H), 3.90-3.89 (m, 1H), 3.18-3.04 (m, 2H), 2.86-2.81 (m, 5H), 2.38-2.23 (m, 3H), 1.86-1.81 (m, 1H), 1.23 (d, 3H).

### Example 81: (S)-2-((4-(3-chloro-4-(2-chloro-3-(6-methoxy-5-((((5-oxopyrrolidin-2-yl)methyl)amino)methyl)pyridin-2-yl)phenyl)pyridin-2-yl)-2-methoxybenzyl)amino)acetic acid (107)

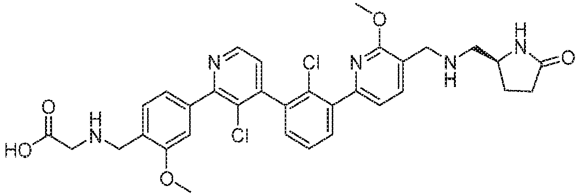

(S)-2-((4-(3-Chloro-4-(2-chloro-3-(6-methoxy-5-((((5-oxopyrrolidin-2-yl)methyl)amino)methyl)pyridin-2-yl)phenyl)pyridin-2-yl)-2-methoxybenzyl)amino)acetic acid formic acid salt was prepared in a similar fashion to compound 105 using (S)-tert-butyl ((6-(2-chloro-3-(3-chloro-2-(4-formyl-3-methoxyphenyl)pyridin-4-yl)phenyl)-2-methoxypyridin-3-yl)methyl)((5-oxopyrrolidin-2-yl)methyl)carbamate and glycine. LCMS: m/z found 650.1 [M+H]+, RT = 2.67 min, (Method A); ¹H NMR (400 MHz, Methanol-d4): δ 8.67 (d, 1H), 7.85 (d, 1H), 7.74 (m, 1H), 7.59 (t, 1H), 7.52 (d, 1H), 7.48-7.44 (m, 2H), 7.40 (s, 1H), 7.35-7.30 (m, 2H), 4.34 (s, 2H), 4.13 (m, 2H), 4.08 (s, 3H), 4.01 (s, 3H), 3.99-3.97 (m, 1H), 3.54 (s, 2H), 3.06-2.97 (m, 2H), 2.45-2.32 (m, 3H), 1.92-1.85 (m, 1H).

### Example 82: (S)-Isopropyl 2-((4-(3-chloro-4-(2-chloro-3-(6-methoxy-5-((((5-oxopyrrolidin-2-yl)methyl)amino)methyl)pyridin-2-yl)phenyl)pyridin-2-yl)-2-methoxybenzyl)amino)acetate (108)

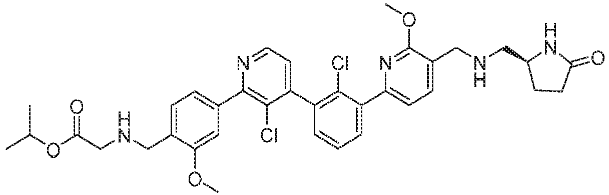

(S)-Isopropyl 2-((4-(3-chloro-4-(2-chloro-3-(6-methoxy-5-((((5-oxopyrrolidin-2-yl)methyl)amino)methyl)pyridin-2-yl)phenyl)pyridin-2-yl)-2-methoxybenzyl)amino)acetate was prepared in a similar fashion to compound **105** using (S)-tert-butyl ((6-(2-chloro-3-(3-chloro-2-(4-formyl-3-methoxyphenyl)pyridin-4-yl)phenyl)-2-methoxypyridin-3-yl)methyl)((5-oxopyrrolidin-2-yl)methyl)carbamate and isopropyl 2-aminoacetate hydrochloride. MS: m/z: 692 [M+H]⁺ observed; ¹H NMR (400 MHz, Methanol-d₄): δ8.54 (d, 1H), 7.68 (d, 1H), 7.61 (m, 1H), 7.46 (t,1H), 7.36-7.31 (m, 3H), 7.24-7.17 (m, 3H), 5.02-4.96 (m, 1H), 4.00 (s, 2H), 3.92 (s, 3H), 3.86 (s, 3H), 3.84 (m, 2H), 3.79-3.77 (m, 1H), 3.53 (s, 2H), 2.76-2.68 (m, 2H), 2.28-2.18 (m, 3H), 1.77-1.72 (m, 1H), 1.18 (d, 6H).

### Example 83: (S)-1-((4-(3-Chloro-4-(2-chloro-3-(6-methoxy-5-((((5-oxopyrrolidin-2-yl)methyl)amino)methyl)pyridin-2-yl)phenyl)pyridin-2-yl)-2-methoxybenzyl)amino)cyclopropanecarboxylic acid (121)

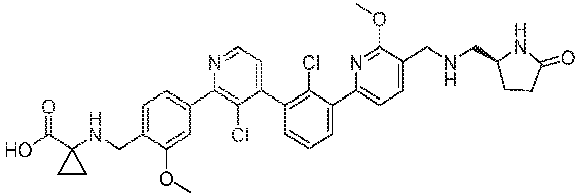

(S)-1-((4-(3-Chloro-4-(2-chloro-3-(6-methoxy-5-((((5-oxopyrrolidin-2-yl)methyl)amino)methyl)pyridin-2-yl)phenyl)pyridin-2-yl)-2-methoxybenzyl)amino)cyclopropanecarboxylic acid as formic acid salt was prepared in a similar fashion to compound 105 using (S)-tert-butyl ((6-(2-chloro-3-(3-chloro-2-(4-formyl-3-methoxyphenyl)pyridin-4-yl)phenyl)-2-methoxypyridin-3-yl)methyl)((5-oxopyrrolidin-2-yl)methyl)carbamate and 1-aminocyclopropanecarboxylic acid. LCMS: m/z found 676.0 [M+H]+, RT = 2.67 min, (Method A); ¹H NMR (400 MHz, Methanol-d₄): δ8.66 (d, 1H), 7.90 (d, 1H), 7.72 (m, 1H), 7.57 (t, 1H), 7.53 (m, 1H), 7.47-7.43 (m, 2H), 7.38-7.36 (m, 2H), 7.34-7.32 (m, 1H), 4.53 (s, 2H), 4.38-4.31 (m, 2H), 4.09 (s, 3H), 4.08-4.05 (m, 1H), 3.98 (s, 3H), 3.26-3.21 (m, 2H), 2.45-2.36 (m, 3H), 1.94-1.89 (m, 1H), 1.61-1.58 (m, 2H), 1.44-1.40 (m, 2H).

### Example 84: (S)-5-((((6-(2-Chloro-3-(3-chloro-2-(4-(((1,1-dioxidotetrahydro-2H-thiopyran-4-yl)amino)methyl)-3-methoxyphenyl)pyridin-4-yl)phenyl)-2-methoxypyridin-3-yl)methyl)amino)methyl)pyrrolidin-2-one (126)

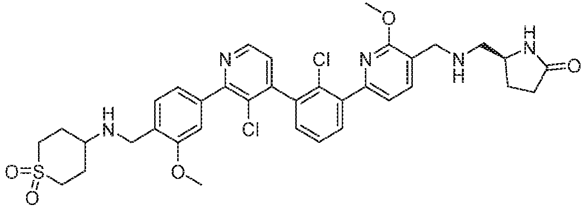

(S)-5-((((6-(2-Chloro-3-(3-chloro-2-(4-(((1,1-dioxidotetrahydro-2H-thiopyran-4-yl)amino)methyl)-3-methoxyphenyl)pyridin-4-yl)phenyl)-2-methoxypyridin-3-yl)methyl)amino)methyl)pyrrolidin-2-one was prepared in a similar fashion to compound **105** using (S)-tert-butyl ((6-(2-chloro-3-(3-chloro-2-(4-formyl-3-methoxyphenyl)pyridin-4-yl)phenyl)-2-methoxypyridin-3-yl)methyl)((5-oxopyrrolidin-2-yl)methyl)carbamate and 4-aminotetrahydro-2H-thiopyran 1,1-dioxide hydrochloride. LCMS: m/z found 723.9 [M+H]+, RT = 2.66 min, (Method A); ¹H NMR (400 MHz, Methanol-d₄): δ 8.66 (d, 1H), 7.90 (d, 1H), 7.73-7.71 (m, 1H), 7.60-7.53 (m, 3H), 7.48-7.46 (m, 2H), 7.40-7.35 (m, 3H), 4.37-4.30 (m, 4H), 4.09 (s, 3H), 4.04-4.02 (m, 1H), 4.00 (s, 3H), 3.58-3.55 (m, 1H), 3.28-3.12 (m, 5H), 2.60 (m, 2H), 2.43 -2.27 (m, 6H), 1.94-1.89 (m, 1H).

### Example 85: (S)-5-((((6-(2-Chloro-3-(3-chloro-2-(3-methoxy-4-(((tetrahydro-2H-pyran-4-yl)amino)methyl)phenyl)pyridin-4-yl)phenyl)-2-methoxypyridin-3-yl)methyl)amino)methyl)pyrrolidin-2-one (127)

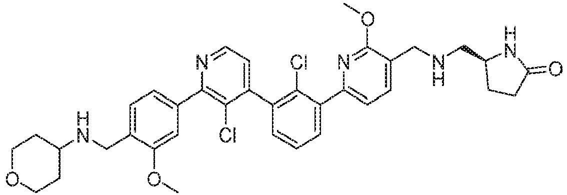

(S)-5-((((6-(2-Chloro-3-(3-chloro-2-(3-methoxy-4-(((tetrahydro-2H-pyran-4-yl)amino)methyl)phenyl)pyridin-4-yl)phenyl)-2-methoxypyridin-3-yl)methyl)amino)methyl)pyrrolidin-2-one was prepared in a similar fashion to compound **105** using (S)-*tert*-butyl ((6-(2-chloro-3-(3-chloro-2-(4-formyl-3-methoxyphenyl)pyridin-4-yl)phenyl)-2-methoxypyridin-3-yl)methyl)((5-oxopyrrolidin-2-yl)methyl)carbamate and tetrahydropyran-4-amine. LCMS: m/z found 676.1 [M+H]+, RT = 2.77 min, (Method A); ¹H NMR (400 MHz, Methanol-d₄): δ 8.66 (d,1H), 8.40 (s, 1H), 7.78 (m, 1H), 7.72-7.70 (m, 1H), 7.57 - 7.52 (m, 2H), 7.47 (d, 1H), 7.42-7.39 (m, 2H), 7.35 (d, 1H), 7.28- 7.25 (m, 1H), 4.31 (s, 2H), 4.07-3.91 (m, 11H), 3.46 (t, 3H), 2.84 (m, 2H), 2.36-2.30 (m, 3H), 2.13-2.10 (m, 2H), 1.84-1.72 (m, 3H).

### Example 86: (S)-5-((((6-(2-Chloro-3-(3-chloro-2-(4-(((2-hydroxyethyl)amino)methyl)-3-methoxyphenyl)pyridin-4-yl)phenyl)-2-methoxypyridin-3-yl)methyl)amino)methyl)pyrrolidin-2-one (70)

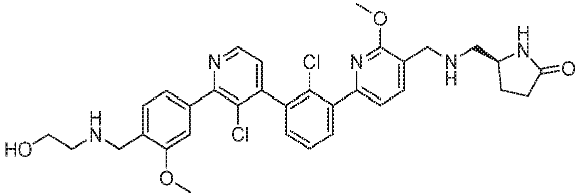

### (a) (S)-tert-butyl ((6-(2-Chloro-3-(3-chloro-2-(4-(((2-hydroxyethyl)amino)methyl)-3-methoxyphenyl)pyridin-4-yl)phenyl)-2-methoxypyridin-3-yl)methyl)((5-oxopyrrolidin-2-yl)methyl)carbamate

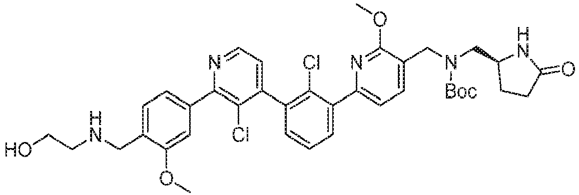

A solution of (S)-*tert*-butyl ((6-(2-chloro-3-(3-chloro-2-(4-formyl-3-methoxyphenyl)pyridin-4-yl)phenyl)-2-methoxypyridin-3-yl)methyl)((5-oxopyrrolidin-2-yl)methyl)carbamate (250 mg, 0.36 mmol) and 2-aminoethanol (33.1 mg, 0.54 mmol) in methylene chloride (4 mL) and 1, 1, 1,3,3,3-hexafluoro-2-propanol (1 mL) was stirred at 15 °C for 2 h. Sodium cyanoborohydride (45 mg, 0.72 mmol) was added to the mixture. The mixture was stirred at 15 °C for 2 hr. The mixture was concentrated and the resulting residue was purified by reverse phase HPLC to afford (S)-tert-butyl ((6-(2-chloro-3-(3-chloro-2-(4-(((2-hydroxyethyl)amino)methyl)-3-methoxyphenyl)pyridin-4-yl)phenyl)-2-methoxypyridin-3-yl)methyl)((5-oxopyrrolidin-2-yl)methyl)carbamate (200 mg, 75% yield) as a yellow solid. MS: m/z found 736 [M+H]+.

### (c) (S)-5-((((6-(2-Chloro-3-(3-chloro-2-(4-(((2-hydroxyethyl)amino)methyl)-3-methoxyphenyl)pyridin-4-yl)phenyl)-2-methoxypyridin-3-yl)methyl)amino)methyl)pyrrolidin-2-one

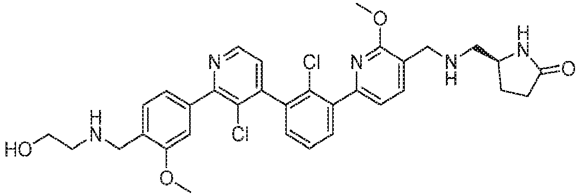

To a solution of (S)-*tert*-butyl ((6-(2-chloro-3-(3-chloro-2-(4-(((2-hydroxyethyl)amino)methyl)-3-methoxyphenyl)pyridin-4-yl)phenyl)-2-methoxypyridin-3-yl)methyl)((5-oxopyrrolidin-2-yl)methyl)carbamate (200 mg, 0.27 mmol) in DCM (5 mL) was added trifluoroacetic acid (0.9 mL, 12.2 mmol). The mixture was stirred at 15 °C for 0.5 hr. The reaction mixture was concentrated and purified by reverse phase HPLC to give Compound (S)-5-((((6-(2-chloro-3-(3-chloro-2-(4-(((2-hydroxyethyl)amino)methyl)-3-methoxyphenyl)pyridin-4-yl)phenyl)-2-methoxypyridin-3-yl)methyl)amino)methyl)pyrrolidin-2-one as formic acid salt (57 mg, 33% yield) as a white solid. LCMS: m/z found 636.1 [M+H]+, RT = 2.53 min, (Method A); ¹H NMR (400 MHz, Methanol-d₄) δ 8.68 (d, 1 H), 8.45 (s, 1H), 7.82 (d, 1 H), 7.74 (m, 1 H), 7.60-7.53 (m, 2 H), 7.49 (d, 1 H), 7.45-7.42 (m, 2 H), 7.37 (d, 1 H), 7.30 (d, 1 H), 4.35 (s, 2 H), 4.06 (s, 3 H), 4.02-3.93 (m, 6 H), 3.86 (t, 2 H), 3.18 (t, 2 H), 2.95-2.85 (m, 2 H), 2.43-2.31 (m, 3 H), 1.89-1.82 (m, 1 H).

### Example 87: (S)-Isopropyl 1-(4-(3-chloro-4-(2-chloro-3-(6-methoxy-5-(((((S)-5-oxopyrrolidin-2-yl)methyl)amino)methyl)pyridin-2-yl)phenyl)pyridin-2-yl)-2-methoxybenzyl)pyrrolidine-2-carboxylate (68)

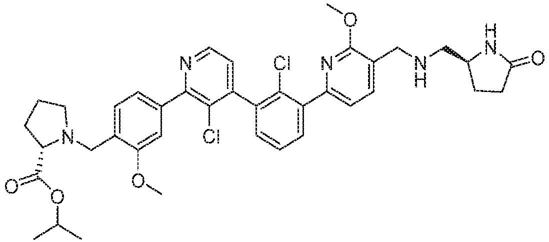

### (a) (S)-Isopropyl 1-(4-(4-(3-(5-(((tert-butoxycarbonyl)(((S)-5-oxopyrrolidin-2-yl)methyl)amino)methyl)-6-methoxypyridin-2-yl)-2-chlorophenyl)-3-chloropyridin-2-yl)-2-methoxybenzyl)pyrrolidine-2-carboxylate

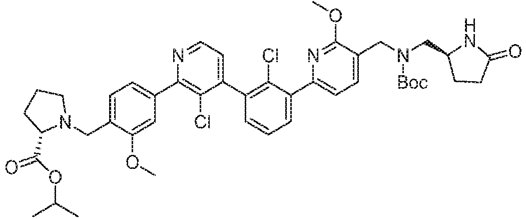

To a mixture of (S)-*tert*-butyl ((6-(2-chloro-3-(3-chloro-2-(4-formyl-3-methoxyphenyl)pyridin-4-yl)phenyl)-2-methoxypyridin-3-yl)methyl)((5-oxopyrrolidin-2-yl)methyl)carbamate (0.20 g, 0.29 mmol) and (S)-isopropyl pyrrolidine-2-carboxylate hydrochloride (0.08 g, 0.43 mmol) in methylene chloride (3 mL) was added sodium acetate (95 mg, 1.16 mmol) and sodium triacetoxyborohydride (0.18 g, 0.87 mmol) in one portion under N₂ atmosphere. The mixture was stirred at RT for 2 h. The mixture was purified by preparative TLC (silica gel, ethyl acetate: MeOH = 5:1) to afford (S)-isopropyl 1-(4-(4-(3-(5-(((tert-butoxycarbonyl)(((S)-5-oxopyrrolidin-2-yl)methyl)amino)methyl)-6-methoxypyridin-2-yl)-2-chlorophenyl)-3-chloropyridin-2-yl)-2-methoxybenzyl)pyrrolidine-2-carboxylate as a light yellow solid (70 mg, 21% yield). MS: m/z found 832 [M+H]+.

### (b) (S)-Isopropyl 1-(4-(3-chloro-4-(2-chloro-3-(6-methoxy-5-(((((S)-5-oxopyrrolidin-2-yl)methyl)amino)methyl)pyridin-2-yl)phenyl)pyridin-2-yl)-2-methoxybenzyl)pyrrolidine-2-carboxylate

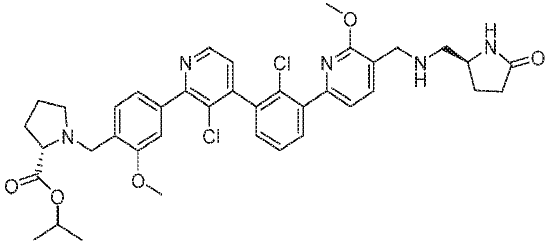

To a mixture of (S)-isopropyl 1-(4-(4-(3-(5-(((*tert*-butoxycarbonyl)(((S)-5-oxopyrrolidin-2-yl)methyl)amino)methyl)-6-methoxypyridin-2-yl)-2-chlorophenyl)-3-chloropyridin-2-yl)-2-methoxybenzyl)pyrrolidine-2-carboxylate (70 mg, 0.08 mmol) in methylene chloride (0.3 mL) was added trifluoroacetic acid (1.2 mL) in one portion. The mixture was stirred at RT for 10 min. The mixture was concentrated. The crude product was purified by reverse phase HPLC to afford (S)-isopropyl 1-(4-(3-chloro-4-(2-chloro-3-(6-methoxy-5-(((((S)-5-oxopyrrolidin-2-yl)methyl)amino)methyl)pyridin-2-yl)phenyl)pyridin-2-yl)-2-methoxybenzyl)pyrrolidine-2-carboxylate as formic salt (25.5 mg, 37% yield) as a white solid. LCMS: m/z found 732.0 [M+H]+, RT = 3.36 min, (Method A); ¹H NMR (400 MHz, Methanol-d₄): δ 8.66 (d, 1H), 8.35 (s, 1H), 7.84 (d, 1H), 7.74 (m, 1H), 7.58 (t, 1H), 7.52-7.44 (m, 3H), 7.35-7.32 (m, 3H), 5.07-4.88 (m, 1H), 4.27 (s, 2H), 4.14-4.06 (m, 5H), 3.96-3.94 (m, 4H), 3.84-3.81 (m, 1H), 3.39-3.37 (m, 1H), 3.03-2.94 (m, 3H), 2.44-2.34 (m, 4H), 2.03-1.87 (m, 4H), 1.28-1.25 (m, 6H).

### Example 88: (S)-Isopropyl 1-(4-(3-chloro-4-(2-chloro-3-(6-methoxy-5-(((((S)-5-oxopyrrolidin-2-yl)methyl)amino)methyl)pyridin-2-yl)phenyl)pyridin-2-yl)-2-methoxybenzyl)pyrrolidine-3-carboxylate (76)

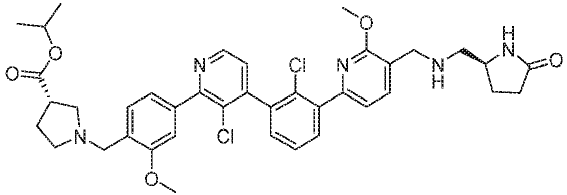

### (a) (S)-Isopropyl pyrrolidine-3-carboxylate

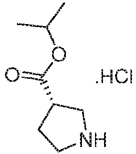

To a mixture of (3S)-pyrrolidine-3-carboxylic acid (0.3 g, 2.61 mmol) in propan-2-ol (3 mL) was added thionyl chloride (0.28 mL, 3.91 mmol) in one portion under N₂ atmosphere. The mixture was stirred at 95 °C for 24 h. The mixture was concentrated to afford (S)-isopropyl pyrrolidine-3-carboxylate as a hydrochloride salt as a yellow gum (0.5 g, 99% yield). ¹H NMR (400 MHz, Methanol-d₄): δ 4.98-4.89 (m, 1H), 3.48-3.40 (m, 2H), 3.28-3.22 (m, 3H), 2.30-2.08 (m, 2H), 1.22-1.16 (m, 6H).

### (b) Isopropyl (S)-1-(4-(4-(3-(5-(((tert-butoxycarbonyl)(((S)-5-oxopyrrolidin-2-yl)methyl)amino)methyl)-6-methoxypyridin-2-yl)-2-chlorophenyl)-3-chloropyridin-2-yl)-2-methoxybenzyl)pyrrolidine-3-carboxylate

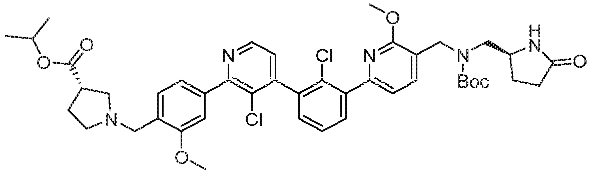

To a mixture of (S)-*tert*-butyl ((6-(2-chloro-3-(3-chloro-2-(4-formyl-3-methoxyphenyl)pyridin-4-yl)phenyl)-2-methoxypyridin-3-yl)methyl)((5-oxopyrrolidin-2-yl)methyl)carbamate (0.25 g, 0.36 mmol) and (S)-isopropyl pyrrolidine-3-carboxylate hydrochloride (0.14 g, 0.72 mmol) in methylene chloride (3 mL) was added sodium acetate (90 mg, 1.08 mmol) and sodium triacetoxyborohydride (0.23 g, 1.08 mmol) in one portion under N₂ atmosphere. The mixture was stirred at RT for 0.5 h. The mixture was purified by preparative TLC (silica gel, ethyl acetate: MeOH = 3:1) to afford (S)-isopropyl 1-(4-(4-(3-(5-(((*tert*-butoxycarbonyl)(((S)-5-oxopyrrolidin-2-yl)methyl)amino)methyl)-6-methoxypyridin-2-yl)-2-chlorophenyl)-3-chloropyridin-2-yl)-2-methoxybenzyl)pyrrolidine-3-carboxylate as a light yellow solid (140 mg, 40% yield). MS: m/z found 832 [M+H]+.

### (c) (S)-Isopropyl 1-(4-(3-chloro-4-(2-chloro-3-(6-methoxy-5-(((((S)-5-oxopyrrolidin-2-yl)methyl)amino)methyl)pyridin-2-yl)phenyl)pyridin-2-yl)-2-methoxybenzyl)pyrrolidine-3-carboxylate

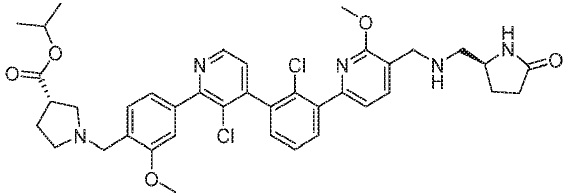

To a mixture of (S)-isopropyl 1-(4-(4-(3-(5-(((*tert*-butoxycarbonyl)(((S)-5-oxopyrrolidin-2-yl)methyl)amino)methyl)-6-methoxypyridin-2-yl)-2-chlorophenyl)-3-chloropyridin-2-yl)-2-methoxybenzyl)pyrrolidine-3-carboxylate (0.135 g, 0.16 mmol) in methylene chloride (0.5 mL) was added trifluoroacetic acid (1.5 mL) in one portion. The mixture was stirred at RT for 10 min. The mixture was concentrated. The residue was purified by reverse phase HPLC to afford (S)-isopropyl 1-(4-(3-chloro-4-(2-chloro-3-(6-methoxy-5-(((((S)-5-oxopyrrolidin-2-yl)methyl)amino)methyl)pyridin-2-yl)phenyl)pyridin-2-yl)-2-methoxybenzyl)pyrrolidine-3-carboxylate as formic salt as a white solid (59.1 mg, 46% yield). LCMS: m/z found 732.1 [M+H]+, RT = 3.24 min, (Method A); ¹H NMR (400 MHz, Methanol-d₄): δ 8.67 (d, 1H), 8.46 (s, 1H), 7.81-7.79 (m, 1H), 7.73 (m, 1H), 7.60-7.54 (m, 2H), 7.49 (d, 1H), 7.45-7.37 (m, 3H), 7.30-7.28 (m, 1H), 5.10-5.00 (m, 1H), 4.35-4.33 (m, 2H), 4.05 (s, 3H), 4.00-3.92 (m, 6H), 3.51-3.46 (m, 2H), 3.30 (m, 3H), 2.86-2.78 (m, 2H), 2.42-2.23 (m, 5H), 1.89-1.81 (m, 1H), 1.29-1.25 (m, 6H).

### Example 89: (S)-1-(4-(3-chloro-4-(2-chloro-3-(6-methoxy-5-(((((S)-5-oxopyrrolidin-2-yl)methyl)amino)methyl)pyridin-2-yl)phenyl)pyridin-2-yl)-2-methoxybenzyl)pyrrolidine-3-carboxylic acid (77)

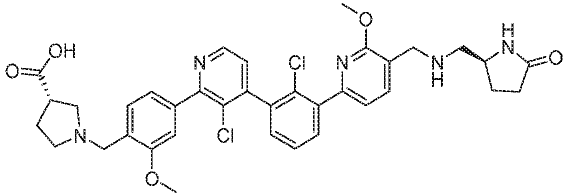

### (a) (S)-1-(4-(4-(3-(5-(((tert-Butoxycarbonyl)(((S)-5-oxopyrrolidin-2-yl)methyl)amino)methyl)-6-methoxypyridin-2-yl)-2-chlorophenyl)-3-chloropyridin-2-yl)-2-methoxybenzyl)pyrrolidine-3-carboxylic acid

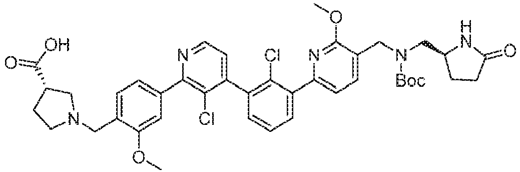

To a mixture of (S)-*tert*-butyl ((6-(2-chloro-3-(3-chloro-2-(4-formyl-3-methoxyphenyl)pyridin-4-yl)phenyl)-2-methoxypyridin-3-yl)methyl)((5-oxopyrrolidin-2-yl)methyl)carbamate (200 mg, 0.36 mmol) and (S)-pyrrolidine-3-carboxylic acid (66.6 mg, 0.58 mmol) in methylene chloride (10 mL) was added sodium acetate (71.2 mg, 0.86 mmol), sodium triacetoxyborohydride (184 mg, 0.87 mmol), and the mixture was stirred at RT for 5 h under N₂. The mixture was concentrated and the crude product was purified by prep-TLC (SiO₂, 3:1 ethyl acetate:MeOH) to afford (S)-1-(4-(4-(3-(5-(((tert-butoxycarbonyl)(((S)-5-oxopyrrolidin-2-yl)methyl)amino)methyl)-6-methoxypyridin-2-yl)-2-chlorophenyl)-3-chloropyridin-2-yl)-2-methoxybenzyl)pyrrolidine-3-carboxylic acid (75 mg, 32%) as a white solid. MS: m/z found 790 [M+H]⁺.

### (b) (S)-1-(4-(3-Chloro-4-(2-chloro-3-(6-methoxy-5-(((((S)-5-oxopyrrolidin-2-yl)methyl)amino)methyl)pyridin-2-yl)phenyl)pyridin-2-yl)-2-methoxybenzyl)pyrrolidine-3-carboxylic acid

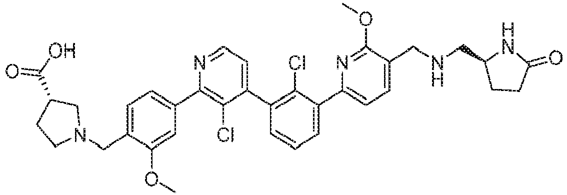

To a solution of (S)-1-(4-(4-(3-(5-(((tert-butoxycarbonyl)(((S)-5-oxopyrrolidin-2-yl)methyl)amino)methyl)-6-methoxypyridin-2-yl)-2-chlorophenyl)-3-chloropyridin-2-yl)-2-methoxybenzyl)pyrrolidine-3-carboxylic acid (70 mg, 0.09 mmol) in methylene chloride (4 mL) was added TFA (4 mL, 54 mmol), and the mixture was stirred at RT for 20 min under N₂. The mixture was concentrated and the crude product was purified by reverse phase HPLC to afford (S)-1-(4-(3-chloro-4-(2-chloro-3-(6-methoxy-5-(((((S)-5-oxopyrrolidin-2-yl)methyl)amino)methyl)pyridin-2-yl)phenyl)pyridin-2-yl)-2-methoxybenzyl)pyrrolidine-3-carboxylic acid as formic acid salt (15 mg, 23%) as a white solid. LCMS: m/z found 690.0 [M+H]⁺, RT = 2.69 min, (Method A); ¹H NMR (400 MHz, Methanol-d₄): δ 8.68 (d, 1H), 8.36 (s, 1H), 7.85 (d, 1H), 7.74 (m, 1H), 7.63-7.56 (m, 2H), 7.48 (d, 1H), 7.46-7.43 (m, 2H), 7.39 (m, 1H), 7.33 (d, 1H), 4.50 (d, 2H), 4.12 (d, 2H), 4.08 (s, 3H), 4.02 (s, 3H), 3.99-3.97 (m, 1H), 3.66-3.62 (m, 1H), 3.49-3.46 (m, 3H), 3.16-3.15 (m, 1H), 3.03-3.00 (m, 2H), 2.42-2.29 (m, 5H), 1.92-1.85 (m, 1H).

### Example 90: (S)-1-(4-(3-Chloro-4-(2-chloro-3-(6-methoxy-5-(((((S)-5-oxopyrrolidin-2-yl)methyl)amino)methyl)pyridin-2-yl)phenyl)pyridin-2-yl)-2-methoxybenzyl)pyrrolidine-2-carboxylic acid (78)

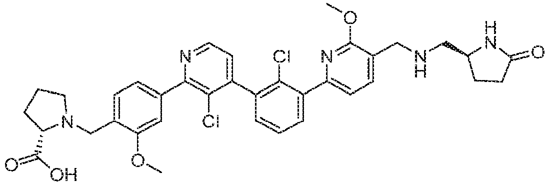

(S)-1-(4-(3-Chloro-4-(2-chloro-3-(6-methoxy-5-(((((S)-5-oxopyrrolidin-2-yl)methyl)amino)methyl)pyridin-2-yl)phenyl)pyridin-2-yl)-2-methoxybenzyl)pyrrolidine-2-carboxylic acid formic acid salt was prepared in a similar fashion to compound 77, using (S)-*tert-butyl* ((6-(2-chloro-3-(3-chloro-2-(4-formyl-3-methoxyphenyl)pyridin-4-yl)phenyl)-2-methoxypyridin-3-yl)methyl)((5-oxopyrrolidin-2-yl)methyl)carbamate and (S)-pyrrolidine-2-LCMS: m/z found 690.3 [M+H]+, RT = 2.77 min, (Method A); ¹H NMR (400 MHz, Methanol-d₄): δ 8.66 (d, 1H), 7.86 (m, 1H), 7.71 (m, 1H), 7.59-7.52 (m, 2H), 7.45-7.44 (m, 2H), 7.44 (s, 1H), 7.35 (d, 2H), 4.53 (m, 1H), 4.42 (m, 1H), 4.24 (s, 2H), 4.08 (s, 3H), 4.00 (s, 3H), 3.98-3.95 (m, 1H), 3.63-3.58 (m, 1H), 3.23-3.13 (m, 3H), 2.48-2.38 (m, 5H), 2.25-2.21 (m, 2H), 1.93-1.88 (m, 2H).

### Example 91: (S)-2-(4-(3-Chloro-4-(2-chloro-3-(6-methoxy-5-((((5-oxopyrrolidin-2-yl)methyl)amino)methyl)pyridin-2-yl)phenyl)pyridin-2-yl)-2-methoxybenzyl)-2,6-diazaspiro[3.4]octan-7-one (88)

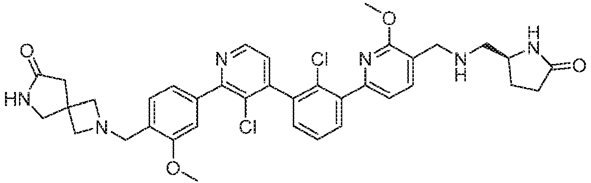

(S)-2-(4-(3-Chloro-4-(2-chloro-3-(6-methoxy-5-((((5-oxopyrrolidin-2-yl)methyl)amino)methyl)pyridin-2-yl)phenyl)pyridin-2-yl)-2-methoxybenzyl)-2,6-diazaspiro[3.4]octan-7-one as formic acid salt was prepared in a similar fashion to compound **68,** using (S)-*tert*-butyl ((6-(2-chloro-3-(3-chloro-2-(4-formyl-3-methoxyphenyl)pyridin-4-yl)phenyl)-2-methoxypyridin-3-yl)methyl)((5-oxopyrrolidin-2-yl)methyl)carbamate and 2,6-diazaspiro[3.4]octan-7-one. LCMS: m/z found 700.1 [M+H]+, RT = 2.55 min, (Method A); ¹H NMR (400 MHz, Methanol-d₄): δ 8.67 (d, 1H), 8.41 (s, 1H), 7.83 (d, 1H), 7.74 (m, 1H), 7.58 (t, 1H), 7.52 (d, 1H), 7.48 (d, 1H), 7.44 (m, 1H), 7.39 (d, 1H), 7.36 (m, 1H), 7.31 (d, 1H), 4.36 (s, 2H), 4.12 (s, 4H), 4.07 (s, 3H), 4.07-4.02 (m, 2H), 3.99 (s, 3H), 3.96-3.94 (m, 1H), 3.68 (s, 2H), 2.97-2.91 (m, 2H), 2.72 (s, 2H), 2.41-2.33 (m, 3H), 1.90-1.86 (m, 1H).

### Example 92: (S)-5-((((6-(3-(2-(4-(((1-Acetylpiperidin-4-yl)amino)methyl)-3-methoxyphenyl)-3-chloropyridin-4-yl)-2-chlorophenyl)-2-methoxypyridin-3-yl)methyl)amino)methyl)pyrrolidin-2-one (109)

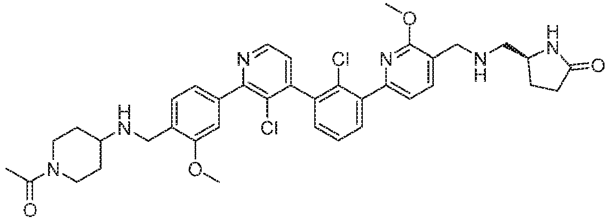

(S)-5-((((6-(3-(2-(4-(((1-Acetylpiperidin-4-yl)amino)methyl)-3-methoxyphenyl)-3-chloropyridin-4-yl)-2-chlorophenyl)-2-methoxypyridin-3-yl)methyl)amino)methyl)pyrrolidin-2-one as formic acid salt was prepared in a similar fashion to compound **68**, using (S)-*tert*-butyl ((6-(2-chloro-3-(3-chloro-2-(4-formyl-3-methoxyphenyl)pyridin-4-yl)phenyl)-2-methoxypyridin-3-yl)methyl)((5-oxopyrrolidin-2-yl)methyl)carbamate and 1-acetyl-4-aminopiperidine. LCMS: m/z found 718.3 [M+H]+, RT = 2.60 min, (Method A); ¹H NMR (400 MHz, Methanol-d₄): δ 8.56 (d, 1H), 8.27 (m, 1H), 7.70 (m, 1H), 7.62 (m, 1H), 7.48-7.43 (m, 2H), 7.38 (d, 1H), 7.34-7.30 (m, 2H), 7.26 (m, 1H), 7.19 (m, 1H), 4.59-4.56 (m, 1H), 4.23 (s, 2H), 3.95-3.82 (m, 10H), 3.38-3.22 (m, 1H), 3.14-3.11 (m, 1H), 2.79-2.76 (m, 2H), 2.60-2.57 (m, 1H), 2.29-2.11 (m, 5H), 2.04 (s, 3H), 1.57-1.54 (m, 1H), 1.46-1.42 (m, 2H).

### Example 93: (S)-5-((((6-(2-Chloro-3-(3-chloro-2-(3-methoxy-4-((((S)-oxetan-2-ylmethyl)amino)methyl)phenyl)pyridin-4-yl)phenyl)-2-methoxypyridin-3-yl)methyl)amino)methyl)pyrrolidin-2-one (122)

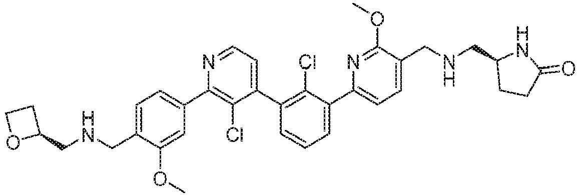

(S)-5-((((6-(2-Chloro-3-(3-chloro-2-(3-methoxy-4-((((S)-oxetan-2-ylmethyl)amino)methyl)phenyl)pyridin-4-yl)phenyl)-2-methoxypyridin-3-yl)methyl)amino)methyl)pyrrolidin-2-one as formic acid salt was prepared in a similar fashion to compound **68** using (S)-*tert*-butyl ((6-(2-chloro-3-(3-chloro-2-(4-formyl-3-methoxyphenyl)pyridin-4-yl)phenyl)-2-methoxypyridin-3-yl)methyl)((5-oxopyrrolidin-2-yl)methyl)carbamate and (S)-oxetan-2-ylmethanamine. LCMS: m/z found 662.1 [M+H]+, RT = 2.69 min, (Method A); ¹H NMR (400 MHz, Methanol-d₄): δ 8.66 (d, 1H), 7.90 (d, 1H), 7.72 (m, 1H), 7.58 (t, 1H), 7.52 (d, 1H), 7.47-7.42 (m, 2H), 7.39-7.34 (m, 3H), 5.14-5.09 (m, 1H), 4.75-4.71 (m, 1H), 4.67-4.63 (m, 1H), 4.35-4.30 (m, 4H), 4.09 (s, 3H), 4.06-4.05 (m, 1H), 3.99 (s, 3H), 3.52-3.47 (m, 1H), 3.26-3.24 (m, 3H), 2.86-2.83 (m, 1H), 2.54-2.36 (m, 4H), 1.95-1.89 (m, 1H).

### Example 94: (S)-Isopropyl 2-((4-(3-chloro-4-(2-chloro-3-(6-methoxy-5-(((((S)-5-oxopyrrolidin-2-yl)methyl)amino)methyl)pyridin-2-yl)phenyl)pyridin-2-yl)-2-methoxybenzyl)amino)propanoate (125)

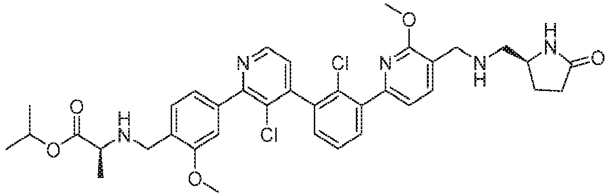

(S)-Isopropyl 2-((4-(3-chloro-4-(2-chloro-3-(6-methoxy-5-(((((S)-5-oxopyrrolidin-2-yl)methyl)amino)methyl)pyridin-2-yl)phenyl)pyridin-2-yl)-2-methoxybenzyl)amino)propanoate as formic acid salt was prepared in a similar fashion to compound **68** using (S)-*tert*-butyl ((6-(2-chloro-3-(3-chloro-2-(4-formyl-3-methoxyphenyl)pyridin-4-yl)phenyl)-2-methoxypyridin-3-yl)methyl)((5-oxopyrrolidin-2-yl)methyl)carbamate and (S)-isopropyl 2-aminopropanoate hydrochloride. LCMS: m/z found 706.2 [M+H]+, RT = 3.21 min, (Method A); ¹H NMR (400 MHz, Methanol-d₄) : δ 8.57 (d, 1H), 7.81 (d, 1H), 7.63 (m, 1H), 7.49 (t, 1H), 7.43 (d, 1H), 7.42-7.41 (m, 2H), 7.30-7.25 (m, 3H), 5.07-5.04 (m, 1H), 4.26 (m, 4H), 4.04-3.95 (m, 5H), 3.90 (s, 3H), 3.20-3.16 (m, 2H), 2.34-2.27 (m, 3H), 1.82 (m, 1H), 1.51 (d, 3H), 1.24-1.22 (m, 6H).

### Example 95: (S)-5-((((6-(2-Chloro-3-(3-chloro-2-(3-fluoro-4-((((1r,3r)-3-hydroxy-3-methylcyclobutyl)amino)methyl)-5-methoxyphenyl)pyridin-4-yl)phenyl)-2-methoxypyridin-3-yl)methyl)amino)methyl)pyrrolidin-2-one (79)

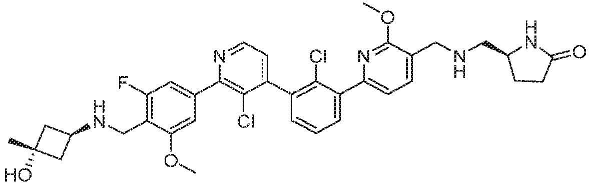

### (a) (S)-tert-Butyl ((6-(2-chloro-3-(3-chloro-2-(3-fluoro-4-formyl-5-methoxyphenyl)pyridin-4-yl)phenyl)-2-methoxypyridin-3-yl)methyl)((5-oxopyrrolidin-2-yl)methyl)carbamate

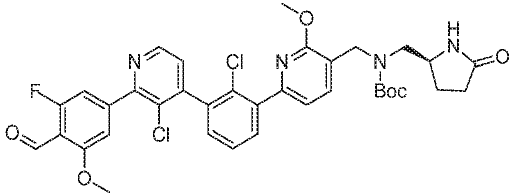

A mixture of *tert-butyl* N-[[6-[2-chloro-3-(2,3-dichloro-4-pyridyl)phenyl]-2-methoxy-3-pyridyl]methyl]-N-[[(2S)-5-oxopyrrolidin-2-yl]methyl]carbamate (0.46 g, 0.78 mmol), 2-fluoro-6-methoxy-4-(4,4,5,5-tetramethyl-1,3,2-dioxaborolan-2-yl)benzaldehyde (as described for compound **91** (0.44 g, 1.6 mmol), [1,1'-bis(di-*tert-*butylphosphino)ferrocene]dichloropalladium(II) (0.05 g, 0.08 mmol) and potassium phosphate (0.50 g, 2.4 mmol) in H₂O/THF (1:5, 12 mL) was stirred at 85 °C for 4 h under N₂ atmosphere. The reaction mixture was filtered and diluted with H₂O (2 x 50 mL) and extracted with Ethyl acetate (2 x 100 mL). The combined organic layers were washed with H₂O (2 x 100 mL), dried over anhydrous sodium sulfate, filtered and concentrated. The resulting residue was purified by flash chromatography (SiO₂, 0-80% ethyl acetate/petroleum ether) to afford (S)-*tert*-butyl ((6-(2-chloro-3-(3-chloro-2-(3-fluoro-4-formyl-5-methoxyphenyl)pyridin-4-yl)phenyl)-2-methoxypyridin-3-yl)methyl)((5-oxopyrrolidin-2-yl)methyl)carbamate (0.80 g) as a brown solid.

### (b) tert-Butyl ((6-(2-chloro-3-(3-chloro-2-(3-fluoro-4-((((1r,3r)-3-hydroxy-3-methylcyclobutyl)amino)methyl)-5-methoxyphenyl)pyridin-4-yl)phenyl)-2-methoxypyridin-3-yl)methyl)(((S)-5-oxopyrrolidin-2-yl)methyl)carbamate

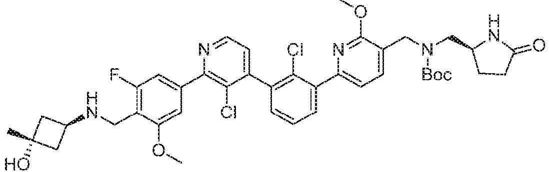

To a mixture of *tert-butyl* N-[[6-[2-chloro-3-[3-chloro-2-(3-fluoro-4-formyl-5-methoxy-phenyl)-4-pyridyl]phenyl]-2-methoxy-3-pyridyl]methyl]-N-[[(2S)-5-oxopyrrolidin-2-yl]methyl]carbamate (0.25 g, 0.35 mmol) and 3-amino-1-methyl-cyclobutanol hydrochloride (0.06 g, 0.40 mmol) in methylene chloride/MeOH (1:2, 6 mL) was added sodium acetate (86 mg, 1.00 mmol) and 4 Å molecular sieves (50 mg, 0.35 mmol). The mixture was stirred at RT for 12 h. Sodium triacetoxyborohydride (0.2 g, 0.9 mmol) was added to the mixture. After stirring for 1 h at RT, the crude reaction mixture was carried forward.

### (c) (S)-5-((((6-(2-Chloro-3-(3-chloro-2-(3-fluoro-4-((((1r,3r)-3-hydroxy-3-methylcyclobutyl)amino)methyl)-5-methoxyphenyl)pyridin-4-yl)phenyl)-2-methoxypyridin-3-yl)methyl)amino)methyl)pyrrolidin-2-one

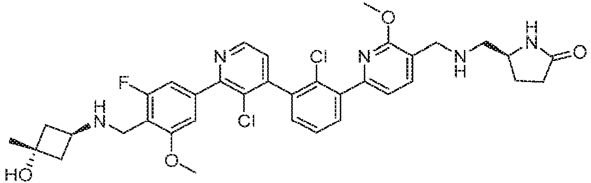

To the above solution of N-[[6-[2-chloro-3-[3-chloro-2-[3-fluoro-4-[[(3-hydroxy-3-methyl-cyclobutyl)amino]methyl]-5-methoxy-phenyl]-4-pyridyl]phenyl]-2-methoxy-3-pyridyl]methyl]-N-[[(2S)-5-oxopyrrolidin-2-yl]methyl]carbamate in methylene chloride (4 mL) was added trifluoroacetic acid (10 mL). The mixture was stirred at RT for 0.5 hr. The reaction mixture was filtered to give a residue. The residue was purified by reverse phase HPLC to afford (S)-5-((((6-(2-chloro-3-(3-chloro-2-(3-fluoro-4-((((1r,3r)-3-hydroxy-3-methylcyclobutyl)amino)methyl)-5-methoxyphenyl)pyridin-4-yl)phenyl)-2-methoxypyridin-3-yl)methyl)amino)methyl)pyrrolidin-2-one (31.9 mg, 12%). LCMS: m/z found 695.4 [M+H]+, RT = 2.77 min, (Method A); ¹H NMR (400 MHz, Methanol-d₄): δ 8.69 (d, 1H), 8.39 (s, 1H), 7.86 (d, 1H), 7.74 (m, 1H), 7.59 (t, 1H), 7.51 (d, 1H), 7.45 (m, 1H), 7.33-7.28 (m, 2H), 7.22 (m, 1H), 4.25 (s, 2H), 4.12 (br s, 2H), 4.08 (s, 3H), 4.04 (s, 3H), 4.00-3.94 (m, 2H), 3.06-2.96 (m, 2H), 2.45-2.38 (m, 5H), 2.26-2.21 (m, 2H), 1.94-1.85 (m, 1H), 1.41 (s, 3H).

### Example 96: (S)-5-((((6-(2-Chloro-3-(3-chloro-2-(3-fluoro-4-((((S)-2-hydroxypropyl)amino)methyl)-5-methoxyphenyl)pyridin-4-yl)phenyl)-2-methoxypyridin-3-yl)methyl)amino)methyl)pyrrolidin-2-one (80)

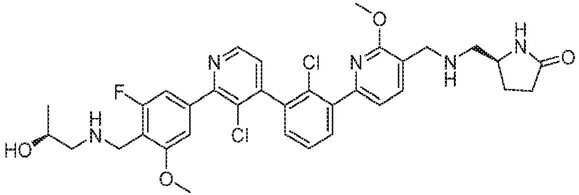

(S)-5-((((6-(2-Chloro-3-(3-chloro-2-(3-fluoro-4-((((S)-2-hydroxypropyl)amino)methyl)-5-methoxyphenyl)pyridin-4-yl)phenyl)-2-methoxypyridin-3-yl)methyl)amino)methyl)pyrrolidin-2-one as a formic acid salt was prepared in a similar fashion to compound 79, using *tert-butyl* ((6-(2-chloro-3-(3-chloro-2-(3-fluoro-4-((((S)-2-hydroxypropyl)amino)methyl)-5-methoxyphenyl)pyridin-4-yl)phenyl)-2-methoxypyridin-3-yl)methyl)(((S)-5-oxopyrrolidin-2-yl)methyl)carbamate (0.3 g,0.42 mmol) and (S)-1-aminopropan-2-ol. LCMS: m/z found 668.0 [M+H]+, RT = 2.57 min, (Method A); ¹H NMR (400 MHz, Methanol-d₄): δ. 8.66 (d, 1H), 8.40 (s, 2H), 7.82 (d, 1H), 7.70 (d, 1H), 7.55 (t, 1H), 7.48 (d, 1H), 7.41 (m, 1H), 7.29 (d, 1H), 7.25 (s, 1H), 7.19 (d, 1H), 4.35 (s, 2H), 4.07-3.95 (m, 10H), 3.10 (m, 1H), 2.96-2.85 (m, 3H), 2.40-2.31 (m, 3H), 1.90-1.81 (m, 1H), 1.22 (d, *J* = 6.4 Hz, 3H).

### Example 97: (S)-5-((((6-(2-Chloro-3-(3-chloro-2-(3-fluoro-4-((((R)-2-hydroxypropyl)amino)methyl)-5-methoxyphenyl)pyridin-4-yl)phenyl)-2-methoxypyridin-3-yl)methyl)amino)methyl)pyrrolidin-2-one (81)

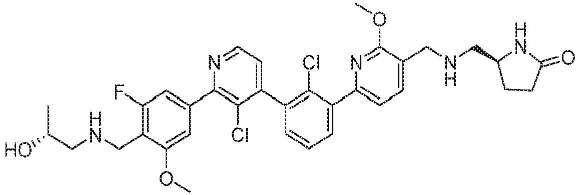

### (a) tert-Butyl ((6-(2-chloro-3-(3-chloro-2-(3-fluoro-4-((((R)-2-hydroxypropyl)amino)methyl)-5-methoxyphenyl)pyridin-4-yl)phenyl)-2-methoxypyridin-3-yl)methyl)(((S)-5-oxopyrrolidin-2-yl)methyl)carbamate

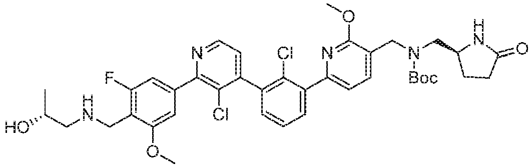

A mixture of (S)-*tert*-butyl ((6-(2-chloro-3-(3-chloro-2-(3-fluoro-4-formyl-5-methoxyphenyl)pyridin-4-yl)phenyl)-2-methoxypyridin-3-yl)methyl)((5-oxopyrrolidin-2-yl)methyl)carbamate (0.25 g, 0.35 mmol), (R)-1-aminopropan-2-ol hydrochloride (51 mg, 0.46 mmol) and sodium acetate (87 mg, 1 mmol) in methylene chloride/ trimethoxymethane mixture (5:1, 12 mL) was stirred at RT for 12 h under N₂. Sodium triacetoxyborohydride (0.15 g, 0.70 mmol) was added. The mixture was stirred at RT for 1 h under N₂. The mixture was filtered, and the filtrate was concentrated. The resulting residue was purified by preparative TLC (SiO₂, 2:1 ethyl acetate: MeOH) to afford *tert-butyl* ((6-(2-chloro-3-(3-chloro-2-(3-fluoro-4-((((R)-2-hydroxypropyl)amino)methyl)-5-methoxyphenyl)pyridin-4-yl)phenyl)-2-methoxypyridin-3-yl)methyl)(((S)-5-oxopyrrolidin-2-yl)methyl)carbamate as a yellow solid (0.25 g, 77% yield).

### (b) (S)-5-((((6-(2-Chloro-3-(3-chloro-2-(3-fluoro-4-((((R)-2-hydroxypropyl)amino)methyl)-5-methoxyphenyl)pyridin-4-yl)phenyl)-2-methoxypyridin-3-yl)methyl)amino)methyl)pyrrolidin-2-one

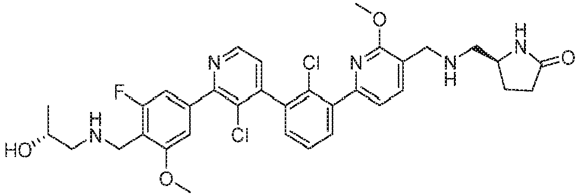

A mixture of *tert-butyl* ((6-(2-chloro-3-(3-chloro-2-(3-fluoro-4-((((R)-2-hydroxypropyl)amino)methyl)-5-methoxyphenyl)pyridin-4-yl)phenyl)-2-methoxypyridin-3-yl)methyl)(((S)-5-oxopyrrolidin-2-yl)methyl)carbamate (0.18 g, 0.23 mmol) in methylene chloride/ trifluoroacetic acid mixture (4:5, 9 mL) was stirred at RT for 0.5 h under N₂. The mixture was concentrated. The resulting residue was purified by reverse phase HPLC to afford (S)-5-((((6-(2-chloro-3-(3-chloro-2-(3-fluoro-4-((((R)-2-hydroxypropyl)amino)methyl)-5-methoxyphenyl)pyridin-4-yl)phenyl)-2-methoxypyridin-3-yl)methyl)amino)methyl)pyrrolidin-2-one as a formate salt as a white solid (52.5 mg, 31% yield). LCMS: m/z found 668.1 [M+H]+, RT = 2.65 min, (Method A); ¹H NMR (400 MHz, Methanol-d₄): δ 7.69 (d, 1H), 8.43 (br s, 2H), 7.84 (d, 1H), 7.74 (m, 1H), 7.58 (t, 1H), 7.51 (d, 1H), 7.44 (m, 1H), 7.32-7.29 (m, 2H), 7.22 (m, 1H), 4.39 (s, 2H), 4.13-4.04 (m, 9H), 4.01-3.95 (m, 1H), 3.13 (m, 1H), 2.98-2.89 (m, 3H), 2.41-2.33 (m, 3H), 1.93-1.84 (m, 1H), 1.26 (d, 3H).

### Example 98: (S)-5-((((6-(2-chloro-3-(3-chloro-2-(3-fluoro-4-((isopropylamino)methyl)-5-methoxyphenyl)pyridin-4-yl)phenyl)-2-methoxypyridin-3-yl)methyl)amino)methyl)pyrrolidin-2-one (82)

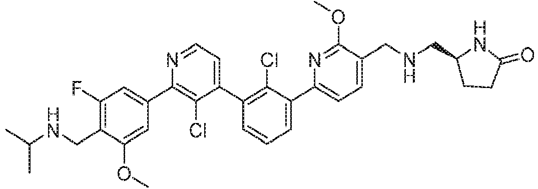

(S)-5-((((6-(2-Chloro-3-(3-chloro-2-(3-fluoro-4-((isopropylamino)methyl)-5-methoxyphenyl)pyridin-4-yl)phenyl)-2-methoxypyridin-3-yl)methyl)amino)methyl)pyrrolidin-2-one as a formic acid salt was prepared in a similar fashion to compound 81 using *tert-butyl* ((6-(2-chloro-3-(3-chloro-2-(3-fluoro-4-((((S)-2-hydroxypropyl)amino)methyl)-5-methoxyphenyl)pyridin-4-yl)phenyl)-2-methoxypyridin-3-yl)methyl)(((S)-5-oxopyrrolidin-2-yl)methyl)carbamate and propan-2-amine. LCMS: m/z found 652.1 [M+H]+, RT = 2.78 min, (Method A); ¹H NMR (400 MHz, Methanol-d₄): δ 8.70 (d, 1H), 8.46 (s, 1H), 7.88 (s, 1H), 7.75 (d, 1H), 7.59 (t, 1H), 7.51 (d, 1H), 7.45 (d, 1H), 7.34-7.29 (m, 2H), 7.23 (d, 1H), 4.35 (s, 2H), 4.15-4.04 (m, 9H), 3.57-3.50 (m, 1H), 3.05 (m, 2H), 2.38 (m, 3H), 1.89 (m, 1H), 1.45 (d, 6H).

### Example 99: (S)-5-((((6-(2-Chloro-3-(3-chloro-2-(3-fluoro-4-((isobutylamino)methyl)-5-methoxyphenyl)pyridin-4-yl)phenyl)-2-methoxypyridin-3-yl)methyl)amino)methyl)pyrrolidin-2-one (83)

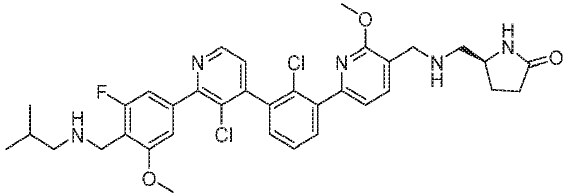

(S)-5-((((6-(2-Chloro-3-(3-chloro-2-(3-fluoro-4-((isobutylamino)methyl)-5-methoxyphenyl)pyridin-4-yl)phenyl)-2-methoxypyridin-3-yl)methyl)amino)methyl)pyrrolidin-2-one as a formic acid salt was prepared in a similar fashion to compound **81** using *tert-butyl* ((6-(2-chloro-3-(3-chloro-2-(3-fluoro-4-((((S)-2-hydroxypropyl)amino)methyl)-5-methoxyphenyl)pyridin-4-yl)phenyl)-2-methoxypyridin-3-yl)methyl)(((S)-5-oxopyrrolidin-2-yl)methyl)carbamate and 2-methylpropan-1-amine. LCMS: m/z found 666.1 [M+H]+, RT = 3.06min, (Method A); ¹H NMR (400 MHz, Methanol-d₄): δ 8.67 (d, 1H), 8.35 (s, 1H), 7.86-7.74 (m, 1H), 7.72 (m, 1H), 7.56 (t, 1H), 7.49 (d, 1H), 7.42 (m, 1H), 7.30-7.27 (m, 2H), 7.21 (d, 1H), 4.34 (s, 2H), 4.03 (m, 8H), 3.93 (br s, 1H), 2.95-2.92 (m, 4H), 2.37-2.31 (m, 3H), 2.14-2.07 (m, 1H), 1.85 (br s, 1H), 1.06 (d, 6H).

### Example 100: (S)-5-((((5-(2-Chloro-3-(3-chloro-2-(3-methoxy-4-(((((S)-5-oxopyrrolidin-2-yl)-methyl)-amino)-methyl)phenyl)pyridin-4-yl)phenyl)-3-methoxypyrazin-2-yl)methyl)amino)methyl)pyrrolidin-2-one (20)

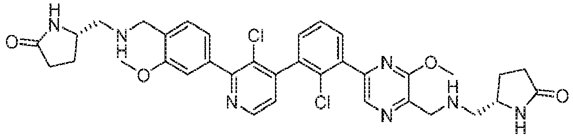

### (a) (S)-5-((((5-Bromo-3-methoxypyrazin-2-yl)methyl)amino)methyl)pyrrolidin-2-one

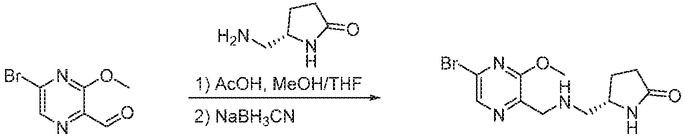

5-Bromo-3-methoxy-pyrazine-2-carbaldehyde (200 mg, 0.92 mmol), (5S)-5-(aminomethyl)pyrrolidin-2-one (137 mg, 1.20 mmol), and acetic acid (66 mg, 1.11 mmol) were dissolved in MeOH/THF (1:1, 5 mL), then the solution was stirred at 25 °C for 2 hours. NaBH₃CN (87 mg, 1.38 mmol) was added and the resulting mixture was stirred at 25 °C for 1 hour. Reaction was subsequently diluted with water (10 mL) and extracted with DCM (3 x 5 mL). The combined organics were further washed with brine (5 mL), dried over sodium sulfate, filtered, and concentrated. Crude sample was purified by flash chromatography (SiO₂, 5-20 % MeOH/DCM) to afford (5S)-5-[[(5-bromo-3-methoxy-pyrazin-2-yl)methylamino]methyl]pyrrolidin-2-one (205 mg, 71 % yield) . MS: *m*/*z* found 315, 317 [M+H]⁺.

### (b) tert-Butyl (S)-((5-bromo-3-methoxypyrazin-2-yl)methyl)((5-oxopyrrolidin-2-yl)methyl)carbamate

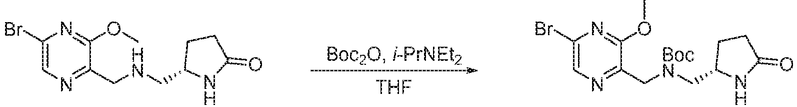

(5S)-5-[[(5-Bromo-3-methoxy-pyrazin-2-yl)methylamino]-methyl]pyrrolidin-2-one (162 mg, 0.44 mmol), *N*,*N*-diisopropylethylamine (0.3 mL, 1.75 mmol), and di-*tert*-butyl dicarbonate (0.19 g, 0.87 mmol) were dissolved in 6 mL THF, then the mixture was stirred at room temperature for 12 h. Reaction was diluted with EtOAc (10 mL) and the resulting solution was washed with saturated aqueous NaHCO₃ (5 mL × 2), followed by brine (5 mL × 2). The organic layer was concentrated under reduced pressure and crude sample was purified by flash chromatography (SiO₂, 0-5 % MeOH/DCM) to afford *tert*-butyl N-[(5-bromo-3-methoxy-pyrazin-2-yl)methyl]-N-[[(2S)-5-oxopyrrolidin-2-yl]methyl]carbamate (175 mg, 96 % yield) . MS: *m*/*z* found 415, 417 [M+H] ⁺.

### (c) tert-Butyl (S)-((3-methoxy-5-(4,4,5,5-tetramethyl-1,3,2-dioxaborolan-2-yl)pyrazin-2-yl)methyl)((5-oxopyrrolidin-2-yl)-methyl)-carbamate

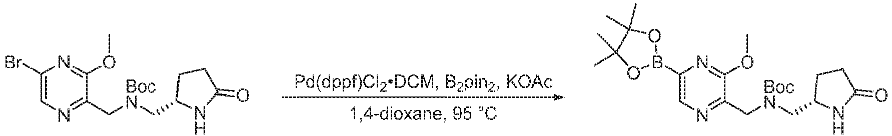

*tert-Butyl* N-[(5-bromo-3-methoxy-pyrazin-2-yl)methyl]-N-[[(2S)-5-oxopyrrolidin-2-yl]methyl]carbamate (156 mg, 0.38 mmol), 4,4,5,5-tetramethyl-2-(4,4,5,5-tetramethyl-1,3,2-dioxaborolan-2-yl)-1,3,2-dioxaborolane (0.13 g, 0.53 mmol), 1,1'-bis(diphenylphosphino)-ferrocene-palladium(II) dichloride dichloromethane complex (31 mg, 0.04 mmol), and potassium acetate (0.11 g, 1.13 mmol) were suspended in 4 mL 1,4-dioxane in a sealed tube. Nitrogen gas was bubbled through the reaction solution for 2 minutes, then the vessel was sealed and heated at 95 °C for 6 h. The mixture was allowed to cool to room temperature and filtered through CELITE^{®}. The filtrate was concentrated under reduced pressure and the crude oil was dissolved in 2 mL EtOAc. Hexane was added drop-wise with vigorous stirring until the dark solid precipitated out of solution. The solid was filtered and the filtrate was concentrated under reduced pressure to afford tert-butyl N-[[3-methoxy-5-(4,4,5,5-tetramethyl-1,3,2-dioxaborolan-2-yl)pyrazin-2-yl]methyl]-N-[[(2S)-5-oxopyrrolidin-2-yl]methyl]carbamate (0.21 g, crude) as a brown oil. MS: *m*/*z* found 381 [M+H] ⁺ (boronic acid fragment).

### (d) tert-Butyl (S)-((5-(2-chloro-3-(3-chloro-2-(4-formyl-3-methoxyphenyl)pyridin-4-yl)phenyl)-3-methoxypyrazin-2-yl)methyl)((5-oxopyrrolidin-2-yl)methyl)carbamate

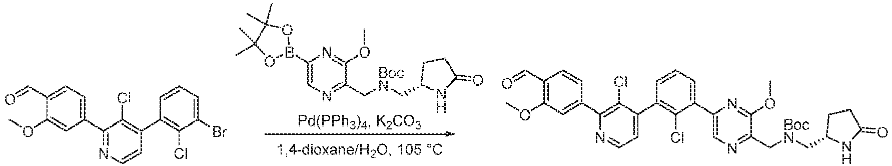

Pd(PPh₃)₄ (12 mg, 0.01 mmol), potassium carbonate (21 mg, 0.15 mmol), 4-[4-(3-bromo-2-chloro-phenyl)-3-chloro-2-pyridyl]-2-methoxy-benzaldehyde (22 mg, 0.05 mmol), and tert-butyl N-[[3-methoxy-5-(4,4,5,5-tetramethyl-1,3,2-dioxaborolan-2-yl)pyrazin-2-yl]methyl]-N-[[(2S)-5-oxopyrrolidin-2-yl]methyl]carbamate (30 mg, 0.07 mmol) were suspended in 1,4-dioxane/water (4:1, 1 mL), then the solution was heated at 105 °C for 20 minutes. Reaction was cooled to rt, diluted with 3 mL water, and extracted with EtOAc (3 x 2 mL). The combined organics were concentrated under reduced pressure and crude sample was purified by flash chromatography (SiO₂, 0-4 % MeOH/DCM) to afford *tert-butyl* N-[[5-[2-chloro-3-[3-chloro-2-(4-formyl-3-methoxy-phenyl)-4-pyridyl]phenyl]-3-methoxy-pyrazin-2-yl]methyl]-N-[[(2S)-5-oxopyrrolidin-2-yl]methyl]carbamate (18 mg, 52 % yield) as a yellow foam. MS: *m*/*z* found 692.3, 694.3 [M+H] ⁺.

### (e) tert-Butyl ((5-(2-chloro-3-(3-chloro-2-(3-methoxy-4-(((((S)-5-oxopyrrolidin-2-yl)methyl)amino)methyl)phenyl)pyridin-4-yl)phenyl)-3-methoxypyrazin-2-yl)methyl)(((S)-5-oxopyrrolidin-2-yl)methyl)carbamate

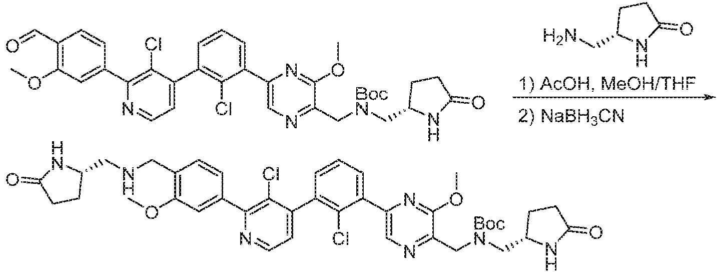

*tert-Butyl* N-[[5-[2-chloro-3-[3-chloro-2-(4-formyl-3-methoxy-phenyl)-4-pyridyl]phenyl]-3-methoxy-pyrazin-2-yl]methyl]-N-[[(2S)-5-oxopyrrolidin-2-yl]methyl]-carbamate (18 mg, 0.03 mmol), (5S)-5-(aminomethyl)pyrrolidin-2-one (4 mg, 0.04 mmol), and acetic acid (2 mg, 0.03 mmol) were dissolved in MeOH/THF (1:1, 1 mL), then the solution was stirred at 25 °C for 2 hours. NaBH₃CN (3 mg, 0.05 mmol) was added and the resulting mixture was stirred at 25 °C for 1 hour. Reaction was subsequently diluted with water (2 mL) and extracted with EtOAc (3 x 3 mL). The combined organics were further washed with brine (3 mL), dried over sodium sulfate, filtered and concentrated under reduced pressure to afford tert-butyl N-[[5-[2-chloro-3-[3-chloro-2-[3-methoxy-4-[[[(2S)-5-oxopyrrolidin-2-yl]methylamino]methyl]phenyl]-4-pyridyl]phenyl]-3-methoxy-pyrazin-2-yl]methyl]-N-[[(2S)-5-oxopyrrolidin-2-yl]methyl]carbamate (20 mg, crude). MS: *m*/*z* found 790.4, 792 [M+H] ⁺. Material was used for the next step without further purification.

### (f) (S)-5-((((5-(2-Chloro-3-(3-chloro-2-(3-methoxy-4-(((((S)-5-oxopyrrolidin-2-yl)-methyl)-amino)-methyl)phenyl)pyridin-4-yl)phenyl)-3-methoxypyrazin-2-yl)methyl)amino)methyl)pyrrolidin-2-one

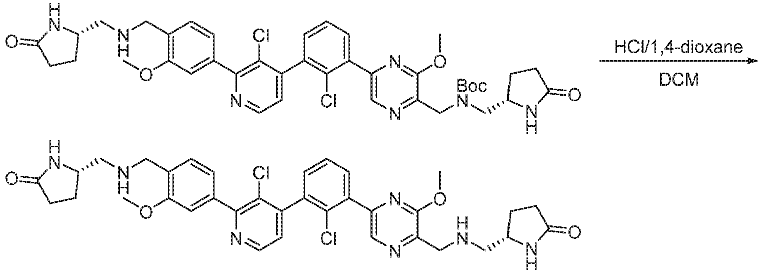

*tert-Butyl* N-[[5-[2-chloro-3-[3-chloro-2-[3-methoxy-4-[[[(2S)-5-oxopyrrolidin-2-yl]methylamino]-methyl]phenyl]-4-pyridyl]phenyl]-3-methoxy-pyrazin-2-yl]methyl]-N-[[(2S)-5-oxopyrrolidin-2-yl]methyl]carbamate (20 mg, 0.03 mmol, crude) was dissolved in 1 mL DCM and 4 M HCl solution in 1,4-dioxane (25 µL, 0.10 mmol) was added. Reaction was stirred at rt for 20 minutes and solvent was removed under reduced pressure. Crude sample was purified by reverse phase HPLC to afford (5S)-5-[[[4-[3-chloro-4-[2-chloro-3-[6-methoxy-5-[[[(2S)-5-oxopyrrolidin-2-yl]methylamino]methyl]pyrazin-2-yl]phenyl]-2-pyridyl]-2-methoxy-phenyl]methylamino]methyl]pyrrolidin-2-one (8 mg, 47 % yield) as a light yellow solid (formic acid salt). LCMS: *m*/*z* found 690.5, 692 [M+H]⁺, retention time = 1.90 min (Method C). ¹H NMR (400 MHz, Methanol-d₄) δ 8.66 (d, 1H), 8.45 (s, 1H), 8.41 (s, 2H), 7.77 (dd, 1H), 7.61 (t, 1H), 7.52 - 7.46 (m, 3H), 7.37 (d, 1H), 7.33 (dd, 1H), 4.25 - 4.18 (m, 2H), 4.15 (d, 2H), 4.08 (s, 3H), 3.98 (s, 5H), 3.10 - 3.01 (m, 2H), 2.93 (m, 2H), 2.44 - 2.27 (m, 6H), 1.95 - 1.78 (m, 2H).

### Example 101: (S)-5-((((6-(2-chloro-3-(3-chloro-2-(1-methyl-2,3,4,5-tetrahydro-1H-benzo[e][1,4]diazepin-8-yl)pyridin-4-yl)phenyl)-2-methoxypyridin-3-yl)methyl)amino)methyl)pyrrolidin-2-one (3)

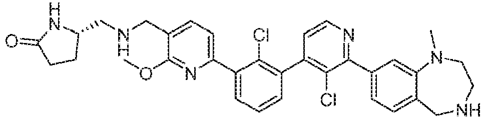

### (a) tert-Butyl 8-chloro-1-methyl-1,2,3,5-tetrahydro-4H-benzo[e][1,4]diazepine-4-carboxylate

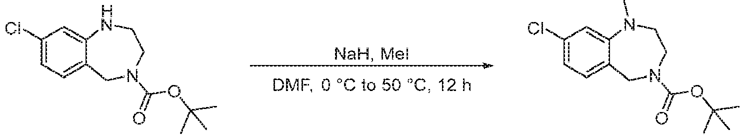

Sodium hydride (55 mg, 60 % in oil dispersion, 1.38 mmol) was suspended in 6 mL anhydrous DMF and the solution was cooled to 0 °C. A solution of tert-butyl 8-chloro-1,2,3,5-tetrahydro-1,4-benzodiazepine-4-carboxylate (300 mg, 1.06 mmol) in 3 mL DMF was slowly added over 2 minutes. The resulting mixture allowed to warm to room temperature and stirred for 30 minutes, then cooled back to 0 °C. Iodomethane (92 µL, 1.49 mmol) was added drop-wise, then the reaction mixture was warmed to 50 °C and stirred for 12 h. The mixture was diluted with 20 mL of water and extracted with EtOAc (3 x 20 mL). The combined organics were concentrated under reduced pressure and the residue was purified by flash chromatography (SiO₂, 0-40 % EtOAc/hexane) to afford *tert*-butyl 8-chloro-1-methyl-3,5-dihydro-2H-1,4-benzodiazepine-4-carboxylate (321 mg, 82 % yield) . MS: *m*/*z* found 297.1, 299.2 [M+H]⁺.

### (b) tert-Butyl 1-methyl-8-(4,4,5,5-tetramethyl-1,3,2-dioxaborolan-2-yl)-1,2,3,5-tetrahydro-4H-benzo[e][1,4]diazepine-4-carboxylate

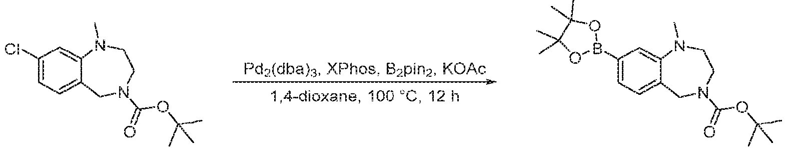

*tert-Butyl* 8-chloro-1-methyl-3,5-dihydro-2H-1,4-benzodiazepine-4-carboxylate (100 mg, 0.34 mmol), 4,4,5,5-tetramethyl-2-(4,4,5,5-tetramethyl-1,3,2-dioxaborolan-2-yl)-1,3,2-dioxaborolane (128 mg, 0.51 mmol), Pd₂(dba)₃ (31 mg, 0.03 mmol), Xphos (30 mg), and potassium acetate (92 mg, 0.94 mmol) were suspended in 3 mL 1,4-dioxane in a sealed tube. Nitrogen gas was bubbled through the reaction solution for 2 minutes, then the vessel was sealed and heated at 100 °C for 12 h. The mixture was cooled to room temperature and the mixture was filtered through CELITE^{®}. The filtrate was concentrated under reduced pressure and the residue was purified by flash chromatography (SiO₂, 0-20 % EtOAc/hexane) to afford *tert*-butyl 1-methyl-8-(4,4,5,5-tetramethyl-1,3,2-dioxaborolan-2-yl)-3,5-dihydro-2H-1,4-benzodiazepine-4-carboxylate (71 mg, 43 % yield). MS: *m*/*z* found 389.2 [M+H] ⁺.

### (c) tert-Butyl 8-(3-chloro-4-(2-chloro-3-(5-formyl-6-methoxypyridin-2-yl)phenyl)pyridin-2-yl)-1-methyl-1,2,3,5-tetrahydro-4H-benzo[e][1,4] diazepine-4-carboxylate

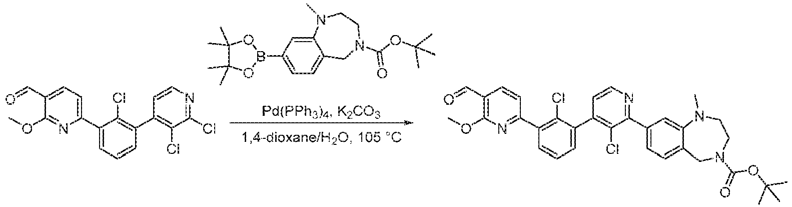

Pd(PPh₃)₄ (41 mg, 0.04 mmol), potassium carbonate (74 mg, 0.53 mmol), 6-[2-chloro-3-(2,3-dichloro-4-pyridyl)phenyl]-2-methoxy-pyridine-3-carbaldehyde (70 mg, 0.18 mmol), and tert-butyl 1-methyl-8-(4,4,5,5-tetramethyl-1,3,2-dioxaborolan-2-yl)-3,5-dihydro-2H-1,4-benzodiazepine-4-carboxylate (90 mg, 0.23 mmol) were suspended in 1,4-dioxane/water (4:1, 3 mL), then the solution was heated at 105 °C for 20 minutes. Reaction was cooled to rt, diluted with 5 mL water, and extracted with EtOAc (3 x 3 mL). The combined organics were concentrated under reduced pressure and crude sample was purified by flash chromatography (SiO₂, 5-60 % EtOAc/hexane) to afford *tert*-butyl 8-[3-chloro-4-[2-chloro-3-(5-formyl-6-methoxy-2-pyridyl)phenyl]-2-pyridyl]-1-methyl-3,5-dihydro-2H-1,4-benzodiazepine-4-carboxylate (88 mg, 79 % yield) as a yellow foam. MS: *m*/*z* found 619.3, 621 [M+H]⁺.

### (d) tert-Butyl (S)-8-(3-chloro-4-(2-chloro-3-(6-methoxy-5-((((5-oxopyrrolidin-2-yl)methyl)amino)methyl)pyridin-2-yl)phenyl)pyridin-2-yl)-1-methyl-1,2,3,5-tetrahydro-4H-benzo[e][1,4]diazepine-4-carboxylate

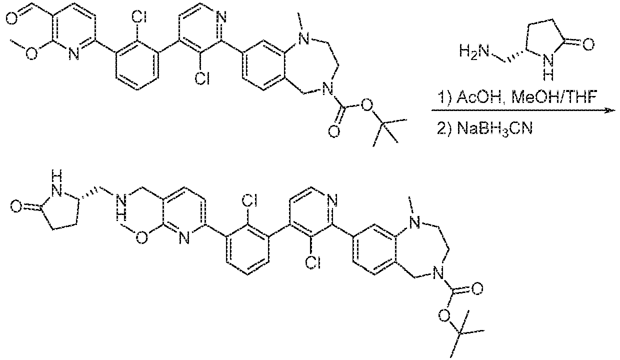

*tert-Butyl* 8-[3-chloro-4-[2-chloro-3-(5-formyl-6-methoxy-2-pyridyl)phenyl]-2-pyridyl]-1-methyl-3,5-dihydro-2H-1,4-benzodiazepine-4-carboxylate (40 mg, 0.06 mmol), (5S)-5-(aminomethyl)pyrrolidin-2-one (11 mg, 0.10 mmol), and acetic acid (4 mg, 0.06 mmol) were dissolved in MeOH/THF (1:1 (*v*/*v*), 2 mL), then the solution was stirred at 25 °C for 2 h. Sodium cyanoborohydride (8.11 mg, 0.13 mmol) was added and the resulting mixture was stirred at 25 °C for 1 h. Reaction was subsequently diluted with water (3 mL) and extracted with methylene chloride (3 x 2 mL). The combined organics were further washed with brine (3 mL), dried over sodium sulfate, filtered and concentrated under reduced pressure to afford *tert*-butyl (S)-8-(3-chloro-4-(2-chloro-3-(6-methoxy-5-((((5-oxopyrrolidin-2-yl)methyl)amino)methyl)pyridin-2-yl)phenyl)pyridin-2-yl)-1-methyl-1,2,3,5-tetrahydro-4H-benzo[e][1,4]diazepine-4-carboxylate (41 mg, crude) as a white foam. MS: *m*/*z* found 717.5, 719 [M+H]⁺. Material was used for the next step without further purification.

### (e) (S)-5-((((6-(2-Chloro-3-(3-chloro-2-(1-methyl-2,3,4,5-tetrahydro-1H-benzo[e][1,4]diazepin-8-yl)pyridin-4-yl)phenyl)-2-methoxypyridin-3-yl)methyl)amino)methyl)pyrrolidin-2-one

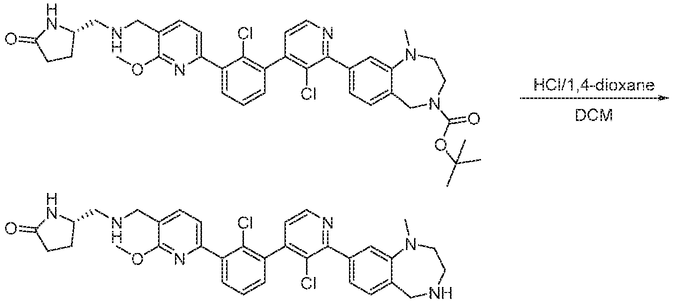

*tert-Butyl* 8-[3-chloro-4-[2-chloro-3-[6-methoxy-5-[[[(2S)-5-oxopyrrolidin-2-yl]methylamino]methyl]-2-pyridyl]phenyl]-2-pyridyl]-1-methyl-3,5-dihydro-2H-1,4-benzodiazepine-4-carboxylate (41 mg, 0.06 mmol) was dissolved in 1 mL of methylene chloride and 4 M HCl solution in 1,4-dioxane (57 µL, 0.23 mmol) was added. Reaction was stirred at rt for 20 minutes and solvent was removed under reduced pressure. Crude sample was purified by reverse phase HPLC to afford (5S)-5-[[[6-[2-chloro-3-[3-chloro-2-(1-methyl-2,3,4,5-tetrahydro-1,4-benzodiazepin-8-yl)-4-pyridyl]phenyl]-2-methoxy-3-pyridyl]methylamino]methyl]pyrrolidin-2-one (26 mg, 73 % yield) (formic acid salt). LCMS: *m*/*z* found 617.3. 619.4 [M+H]⁺, retention time = 2.08 min. (Method C). ¹H NMR (400 MHz, Methanol-d₄) δ 8.64 (d, 1H), 8.50 (s, 1H), 7.77 (d, 1H), 7.71 (dd, 1H), 7.55 (t, 1H), 7.48 - 7.39 (m, 3H), 7.38 (d, 1H), 7.31 (dd, 1H), 7.27 (d, 1H), 4.33 (s, 2H), 4.03 (s, 3H), 3.98 - 3.83 (m, 3H), 3.37 (q, 2H), 3.28 (d, 2H), 3.02 (s, 3H), 2.86 - 2.75 (m, 2H), 2.41 - 2.24 (m, 3H), 1.83 (tt, 1H).

### Example 102: N-(1-(((6-(2-Chloro-3-(3-chloro-2-(1-methyl-2,3,4,5-tetrahydro-1H-benzo[e][1,4]diazepin-8-yl)pyridin-4-yl)phenyl)-2-methoxypyridin-3-yl)methyl)amino)propan-2-yl)acetamide (5)

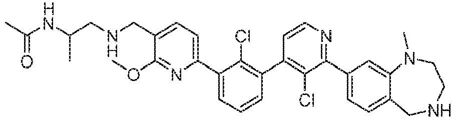

Compound was prepared in the same manner as described for compound **3,** utilizing intermediate *tert-butyl* 8-(3-chloro-4-(2-chloro-3-(5-formyl-6-methoxypyridin-2-yl)phenyl)pyridin-2-yl)-1-methyl-1,2,3,5-tetrahydro-4H-benzo[e][1,4]diazepine-4-carboxylate. N-(1-aminopropany-2-yl)acetamide was used in place of (S)-5-(aminomethyl)pyrrolidin-2-one. LCMS: *m*/*z* found 619.3, 621.3 [M+H] ⁺, retention time = 2.13 min. (Method C). ¹H NMR (400 MHz, Methanol-d₄) δ 8.64 (dd, 1H), 8.51 (s, 3H), 7.83 - 7.78 (m, 1H), 7.71 (dt, 1H), 7.56 (td, 1H), 7.48 - 7.40 (m, 3H), 7.37 (d, 1H), 7.31 (ddd, 2H), 4.32 (s, 2H), 4.19 (s, 1H), 4.16 - 4.02 (m, 5H), 3.37 (d, 1H), 3.32 - 3.25 (m, 3H), 3.02 (d, 3H), 3.00 - 2.94 (m, 1H), 2.90 (dd, 1H), 1.97 (d, 3H), 1.21 (dd, 3H).

### Example 103: (S)-5-((((6-(2-Chloro-3-(3-chloro-2-(8-methoxy-2-(((S)-5-oxopyrrolidin-2-yl)methyl)-1,2,3,4-tetrahydroisoquinolin-6-yl)pyridin-4-yl)phenyl)-2-methoxypyridin-3-yl)methyl)amino)methyl)pyrrolidin-2-one (6)

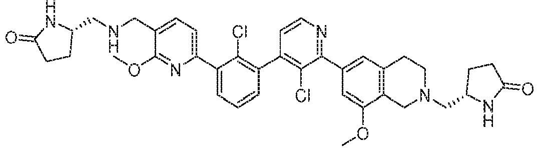

### (a) (S)-5-((6-Bromo-8-methoxy-3,4-dihydroisoquinolin-2(1H)-yl)methyl)pyrrolidin-2-one

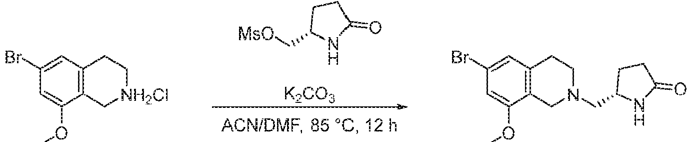

6-bromo-8-methoxy-1,2,3,4-tetrahydroisoquinoline hydrochloride salt (100 mg, 0.36 mmol), [(2S)-5-oxopyrrolidin-2-yl]methyl methanesulfonate (90 mg, 0.47 mmol), and potassium carbonate (149 mg, 1.08 mmol) were suspended in 3 mL acetonitrile/0.3 mL DMF. The mixture was then heated at 85 °C for 12 h. Reaction was cooled to rt, diluted with water (6 mL), and extracted with EtOAc (3 x 5 ml). The combined organic extracts were washed with water (5 mL), brine (5 mL), dried over sodium sulfate, filtered and concentrated under reduced pressure to afford (5S)-5-[(6-bromo-8-methoxy-3,4-dihydro-1H-isoquinolin-2-yl)methyl]pyrrolidin-2-one (118 mg, crude) as a light brown oil. MS: *m*/*z* found 339, 341 [M+H] ⁺. Material was used for the next step without further purification.

### (b) (S)-5-((8-Methoxy-6-(4,4,5,5-tetramethyl-1,3,2-dioxaborolan-2-yl)-3,4-dihydroisoquinolin-2(1H)-yl)methyl)pyrrolidin-2-one

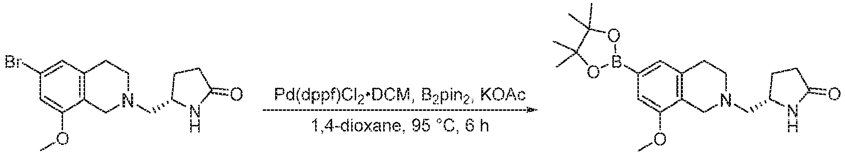

(5S)-5-[(6-Bromo-8-methoxy-3,4-dihydro-1H-isoquinolin-2-yl)methyl]pyrrolidin-2-one (50 mg, 0.15 mmol), 4,4,5,5-tetramethyl-2-(4,4,5,5-tetramethyl-1,3,2-dioxaborolan-2-yl)-1,3,2-dioxaborolane (52 mg, 0.21 mmol), 1,1'-bis(diphenylphosphino)-ferrocene-palladium(II) dichloride dichloromethane complex (12 mg, 0.01 mmol), and potassium acetate (40 mg, 0.41 mmol) were suspended in 2 mL of 1,4-dioxane in a sealed tube. Nitrogen gas was bubbled through the reaction solution for 2 minutes, then the vessel was sealed and heated at 95 °C for 6 hours. Reaction was cooled to rt and the mixture was filtered through CELITE^{®}. The filtrate was concentrated under reduced pressure and the crude oil was dissolved in 2 mL of EtOAc. Hexane was added drop-wise with vigorous stirring until the dark solid precipitated out of solution. The solid was filtered and the filtrate was concentrated under reduced pressure to afford (5S)-5-[[8-methoxy-6-(4,4,5,5-tetramethyl-1,3,2-dioxaborolan-2-yl)-3,4-dihydro-1H-isoquinolin-2-yl]methyl]pyrrolidin-2-one (56 mg, crude) as a light brown oil. MS: *m*/*z* found 387 [M+H] ⁺. Material was used for the next step without further purification.

### (c) (S)-6-(2-Chloro-3-(3-chloro-2-(8-methoxy-2-((5-oxopyrrolidin-2-yl)methyl)-1,2,3,4-tetrahydroisoquinolin-6-yl)pyridin-4-yl)phenyl)-2-methoxynicotinaldehyde

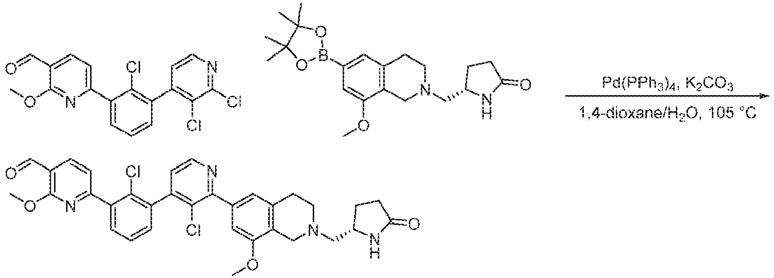

Pd(PPh₃)₄ (33 mg, 0.03 mmol), potassium carbonate (60 mg, 0.43 mmol), 6-[2-chloro-3-(2,3-dichloro-4-pyridyl)phenyl]-2-methoxy-pyridine-3-carbaldehyde (74 mg, 0.19 mmol), and (5S)-5-[[8-methoxy-6-(4,4,5,5-tetramethyl-1,3,2-dioxaborolan-2-yl)-3,4-dihydro-1H-isoquinolin-2-yl]methyl]pyrrolidin-2-one (56 mg, 0.14 mmol) were suspended in 1,4-dioxane/water (4:1, 3 mL), then the solution was heated at 105 °C for 20 minutes. The mixture was allowed to cool to room temperature, diluted with 3 mL water, and extracted with EtOAc (3 x 2 mL). The combined organic extracts were concentrated under reduced pressure and crude sample was purified by flash chromatography (SiO₂, 0-6 % MeOH/methylene chloride) to afford 6-[2-chloro-3-[3-chloro-2-[8-methoxy-2-[[(2S)-5-oxopyrrolidin-2-yl]methyl]-3,4-dihydro-1H-isoquinolin-6-yl]-4-pyridyl]phenyl]-2-methoxy-pyridine-3-carbaldehyde (25 mg, 28 % yield). MS: *m*/*z* found 617, 619 [M+H] ⁺.

### (d) (S)-5-((((6-(2-Chloro-3-(3-chloro-2-(8-methoxy-2-(((S)-5-oxopyrrolidin-2-yl)methyl)-1,2,3,4-tetrahydroisoquinolin-6-yl)pyridin-4-yl)phenyl)-2-methoxypyridin-3-yl)methyl)amino)methyl)pyrrolidin-2-one

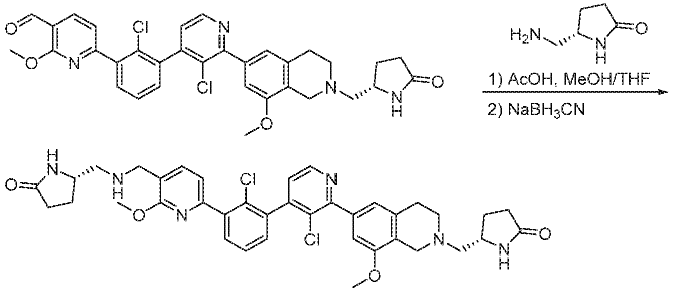

6-[2-Chloro-3-[3-chloro-2-[8-methoxy-2-[[(2S)-5-oxopyrrolidin-2-yl]methyl]-3,4-dihydro-1H-isoquinolin-6-yl]-4-pyridyl]phenyl]-2-methoxy-pyridine-3-carbaldehyde (25 mg, 0.04 mmol), (5S)-5-(aminomethyl)pyrrolidin-2-one (14 mg, 0.12 mmol), and acetic acid (5 mg, 0.08 mmol) were dissolved in MeOH/THF (1:1, 1 mL), then the solution was stirred at 25 °C for 2 hours. Sodium cyanoborohydride (8 mg, 0.12 mmol) was added and the resulting mixture was stirred at 25 °C for 1 h. The mixture was subsequently diluted with water (2 mL) and extracted with EtOAc (3 x 2 mL). The combined organics were further washed with brine, dried over sodium sulfate, filtered, and concentrated under reduced pressure. The residue was purified by reverse phase HPLC to afford (5S)-5-[[[6-[2-chloro-3-[3-chloro-2-[8-methoxy-2-[[(2S)-5-oxopyrrolidin-2-yl]methyl]-3,4-dihydro-1H-isoquinolin-6-yl]-4-pyridyl]phenyl]-2-methoxy-3-pyridyl]-methylamino]-methyl]-pyrrolidin-2-one (8 mg, 27 % yield). LCMS: *m*/*z* found 715.3, 717.3 [M+H]⁺, retention time = 2.01 min. (Method C). ¹H NMR (400 MHz, Methanol-*d*₄) δ 8.60 (d, 1H), 8.43 (s, 1H), 7.79 (d, 1H), 7.70 (dd, 1H), 7.55 (t, 1H), 7.44 - 7.38 (m, 2H), 7.28 (d, 1H), 7.08 - 7.00 (m, 2H), 4.04 (s, 4H), 3.97 (d, 2H), 3.87 (s, 4H), 3.77 (d, 1H), 3.64 (d, 1H), 2.98 (t, 2H), 2.95 - 2.59 (m, 6H), 2.33 (tt, 6H), 1.91 - 1.79 (m, 2H).

### Example 104: (S)-5-((((6-(2-Chloro-3-(3-chloro-2-(8-methoxy-1,2,3,4-tetrahydroisoquinolin-6-yl)pyridin-4-yl)phenyl)-2-methoxypyridin-3-yl)methyl)amino)methyl)pyrrolidin-2-one (7)

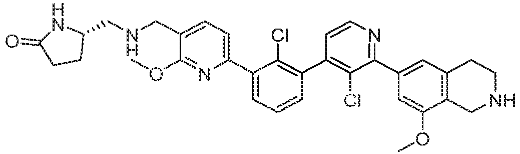

### (a) tert-Butyl 6-(3-chloro-4-(2-chloro-3-(5-formyl-6-methoxypyridin-2-yl)phenyl)pyridin-2-yl)-8-methoxy-3,4-dihydroisoquinoline-2(1H)-carboxylate

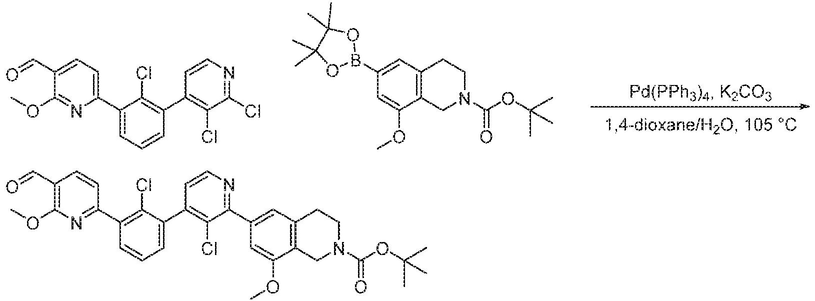

Pd(PPh₃)₄ (23 mg, 0.02 mmol), potassium carbonate, (42 mg, 0.30 mmol), 6-[2-chloro-3-(2,3-dichloro-4-pyridyl)phenyl]-2-methoxy-pyridine-3-carbaldehyde (40 mg, 0.10 mmol), and tert-butyl 8-methoxy-6-(4,4,5,5-tetramethyl-1,3,2-dioxaborolan-2-yl)-3,4-dihydro-1H-isoquinoline-2-carboxylate (47 mg, 0.12 mmol) were suspended in 1,4-dioxane/water (4:1, 3 mL), then the solution was heated at 105 °C for 20 minutes. Reaction was cooled to rt, diluted with 5 mL water, and extracted with EtOAc (3 x 3 mL). The combined organics were concentrated under reduced pressure and crude sample was purified by flash chromatography (SiO₂, 0-6 % MeOH/DCM) to afford *tert-butyl* 6-[3-chloro-4-[2-chloro-3-(5-formyl-6-methoxy-2-pyridyl)phenyl]-2-pyridyl]-8-methoxy-3,4-dihydro-1H-isoquinoline-2-carboxylate (44 mg, 70 % yield). MS: *m*/*z* found 620.2, 622 [M+H] ⁺.

### (b) tert-Butyl (S)-6-(3-chloro-4-(2-chloro-3-(6-methoxy-5-((((5-oxopyrrolidin-2-yl)methyl)amino)methyl)pyridin-2-yl)phenyl)pyridin-2-yl)-8-methoxy-3,4-dihydroisoquinoline-2(1H)-carboxylate

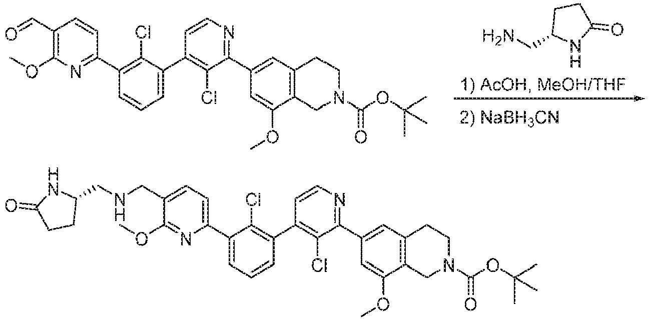

*tert-Butyl* 6-[3-chloro-4-[2-chloro-3-(5-formyl-6-methoxy-2-pyridyl)phenyl]-2-pyridyl]-8-methoxy-3,4-dihydro-1H-isoquinoline-2-carboxylate (44 mg, 0.07 mmol), (5S)-5-(aminomethyl)pyrrolidin-2-one (24 mg, 0.21 mmol), and acetic acid (9 mg, 0.14 mmol) were dissolved in MeOH/THF (1:1, 2 mL), then the solution was stirred at 25 °C for 2 h. Sodium cyanoborohydride (13 mg, 0.21 mmol) was added and the resulting mixture was stirred at 25 °C for 1 h. The mixture was subsequently diluted with water (2 mL) and extracted with methylene chloride (3 x 2 mL). The combined organic extracts were further washed with brine (3 mL), dried over sodium sulfate, filtered and concentrated under reduced pressure to afford *tert-butyl* 6-[3-chloro-4-[2-chloro-3-[6-methoxy-5-[[[(2S)-5-oxopyrrolidin-2-yl]methylamino]-methyl]-2-pyridyl]phenyl]-2-pyridyl]-8-methoxy-3,4-dihydro-1H-isoquinoline-2-carboxylate (50 mg, crude). MS: *m*/*z* found 719 [M+H]⁺.

### (c) (S)-5-((((6-(2-Chloro-3-(3-chloro-2-(8-methoxy-1,2,3,4-tetrahydroisoquinolin-6-yl)pyridin-4-yl)phenyl)-2-methoxypyridin-3-yl)methyl)amino)methyl)pyrrolidin-2-one

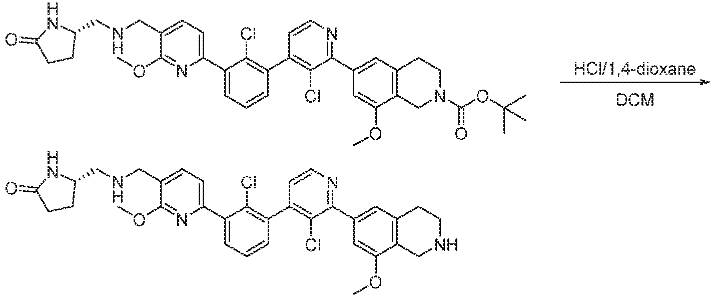

*tert-Butyl* 6-[3-chloro-4-[2-chloro-3-[6-methoxy-5-[[[(2S)-5-oxopyrrolidin-2-yl]methylamino]methyl]-2-pyridyl]phenyl]-2-pyridyl]-8-methoxy-3,4-dihydro-1H-isoquinoline-2-carboxylate (50 mg, 0.07 mmol, crude) was dissolved in 1 mL of methylene chloride and 4 M HCl solution in 1,4-dioxane (70 µL, 10 mg, 0.28 mmol) was added. The mixture was stirred at room temperature for 20 min and solvent was removed under reduced pressure. The residue was purified by reverse phase HPLC to afford (5S)-5-[[[6-[2-chloro-3-[3-chloro-2-(8-methoxy-1,2,3,4-tetrahydroisoquinolin-6-yl)-4-pyridyl]phenyl]-2-methoxy-3-pyridyl]methylamino]methyl]pyrrolidin-2-one (11 mg, 26 % yield) (formic acid salt). LCMS: *m*/*z* found 618.25, 620 [M+H]⁺, retention time = 2.12 min. (Method C). ¹H NMR (400 MHz, Methanol-d₄) δ 8.63 (dd, 1H), 8.49 (s, 1H), 7.76 (d, 1H), 7.71 (dt, 1H), 7.59 - 7.51 (m, 1H), 7.45 (dd, 1H), 7.44 - 7.37 (m, 1H), 7.30 - 7.21 (m, 1H), 7.17 (d, 2H), 4.30 (s, 2H), 4.02 (d, 3H), 3.92 (d, 3H), 3.87 (dd, 3H), 3.49 (t, 2H), 3.15 (t, 2H), 2.82 - 2.69 (m, 2H), 2.39 - 2.23 (m, 3H), 1.82 (dt, 1H).

### Example 105: (S)-5-((((6-(2-Chloro-3-(3-chloro-2-(1-methyl-4-(((S)-5-oxopyrrolidin-2-yl)methyl)-2,3,4,5-tetrahydro-1H-benzo[e][1,4]diazepin-8-yl)-pyridin-4-yl)phenyl)-2-methoxypyridin-3-yl)methyl)amino)methyl)pyrrolidin-2-one (19)

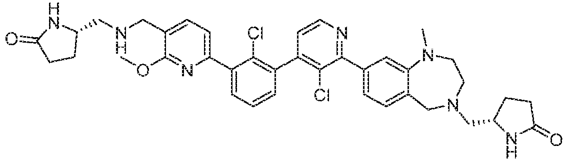

Compound was prepared in a similar manner as described for compound 6 utilizing intermediate 6-(2-chloro-3-(2,3-dichloropyridin-4-yl)phenyl)-2-methoxynicotinaldehyde. (S)-5-((1-methyl-8-(4,4,5,5-tetramethyl-1,3,2-dioxaborolan-2-yl)-1,2,3,5-tetrahydro-4H-benzo[e][1,4]diazepin-4-yl)methyl)pyrrolidin-2-one was used in place of (S)-5-((8-methoxy-6-(4,4,5,5-tetramethyl-1,3,2-dioxaborolan-2-yl)-3,4-dihydroisoquinolin-2(1H)-yl)methyl)pyrrolidin-2-one. LCMS: *m*/*z* found 714.4, 716 [M+H]⁺, retention time = 2.08 min. (Method C). ¹H NMR (400 MHz, Methanol-d₄) δ 8.60 (d, 1H), 8.42 (s, 1H), 7.81 (d, 1H), 7.70 (dd, 1H), 7.55 (t, 1H), 7.45 - 7.38 (m, 2H), 7.30 (dd, 2H), 7.25 - 7.13 (m, 2H), 4.04 (d, 4H), 3.96 (d, 4H), 3.04 (t, 4H), 2.93 (d, 5H), 2.56 (h, 2H), 2.35 (td, 5H), 2.29 - 2.20 (m, 1H), 1.90 - 1.70 (m, 2H).

### Example 106: (S)-5-((((6-(2-Chloro-3-(3-chloro-2-(2-((R)-2-hydroxypropyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one (27)

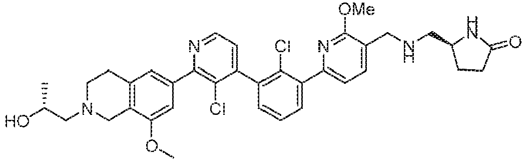

### (a) (R)-1-(6-Bromo-8-methoxy-3,4-dihydroisoquinolin-2(1H)-yl)propan-2-ol

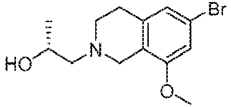

To a solution of 6-bromo-8-methoxy-1,2,3,4-tetrahydroisoquinoline hydrochloride salt (0.25 g, 0.90 mmol) and (R)-2-methyloxirane (0.52 g, 8.97 mmol) in ethanol (7 mL) was added N,N-diisopropylethylamine (0.01 g, 0.01 mmol). The mixture was stirred at 85 °C for 1 h, then concentrated under reduced pressure. The residue was purified by flash chromatography using a gradient (SiO₂, 0-5% MeOH/methylene chloride) to provide (R)-1-(6-bromo-8-methoxy-3,4-dihydroisoquinolin-2(1H)-yl)propan-2-ol (0.18 g, 67% yield). LCMS: m/z found 300 [M+H]⁺, RT = 0.58 min, (Method B).

### (b) (R)-1-(8-Methoxy-6-(4,4,5,5-tetramethyl-1,3,2-dioxaborolan-2-yl)-3,4-dihydroisoquinolin-2(1H)-yl)propan-2-ol:

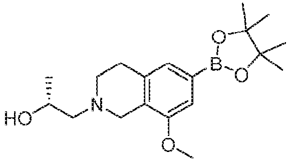

A pressure vessel was charged with (R)-1-(6-bromo-8-methoxy-3,4-dihydroisoquinolin-2(1H)-yl)propan-2-ol (0.18 g, 0.60 mmol), bis(pinacolato)diboron (0.17 g, 0.66 mmol), potassium acetate (0.16g, 1.68 mmol), and Pd(dppf)₂Cl₂-CH₂Cl₂ (0.05 g, 0.06 mmol). The flask was purged with nitrogen for 5 min, then 7 mL of dry dioxane was added, and the reaction was sparged with nitrogen for a further 5 min. The vessel was sealed, and the mixture was stirred at 90 °C for 1 hr. The mixture was then allowed to cool to room temperature, filtered through CELITE^{®} and the filter cake was washed with ethyl acetate (3 x 20 mL). The filtrate was concentrated under reduced pressure and the residue was purified by flash chromatography using a gradient (SiO₂, 0-10% methano/methylene chloride) to provide (R)-1-(8-methoxy-6-(4,4,5,5-tetramethyl-1,3,2-dioxaborolan-2-yl)-3,4-dihydroisoquinolin-2(1H)-yl)propan-2-ol (0.15 g, 75% yield). LCMS: m/z found 348 [M+H]⁺, RT = 0.68 min (Method B).

### (c) (R)-6-(2-Chloro-3-(3-chloro-2-(2-(2-hydroxypropyl)-8-methoxy-1,2,3,4-tetrahydroisoquinolin-6-yl)pyridin-4-yl)phenyl)-2-methoxynicotinaldehyde:

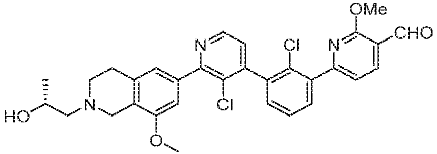

A pressure vessel was charged with (R)-1-(8-methoxy-6-(4,4,5,5-tetramethyl-1,3,2-dioxaborolan-2-yl)-3,4-dihydroisoquinolin-2(1H)-yl)propan-2-ol (0.15 g, 0.43 mmol), 6-(2-chloro-3-(2,3-dichloropyridin-4-yl)phenyl)-2-methoxynicotinaldehyde (0.17 g, 0.43 mmol), Cs₂CO₃ (0.42 g 1.30 mmol), and Pd(PPh₃)₄ (0.05 g, 0.04 mmol). The vessel was then purged with nitrogen. Dioxane was added (6 ml), and the reaction was sparged with nitrogen, then 0.75 mL of water added. The vessel was sealed and the mixture was stirred at 90 °C for 1 h. The mixture was allowed to cool to room temperature, filtered through CELITE^{®} and the filter cake was washed with ethyl acetate (3 x 15 mL). The filtrate was concentrated under reduced pressure and the residue was purified by flash chromatography (SiO₂, eluting with a linear gradient of 0-10% methanol/methylene chloride) to provide (R)-6-(2-chloro-3-(3-chloro-2-(2-(2-hydroxypropyl)-8-methoxy-1,2,3,4-tetrahydroisoquinolin-6-yl)pyridin-4-yl)phenyl)-2-methoxynicotinaldehyde (0.22 g, 90% yield). LCMS: m/z found 578 [M+H]⁺, RT = 0.81 min (Method B).

### (d) (S)-5-((((6-(2-Chloro-3-(3-chloro-2-(2-((R)-2-hydroxypropyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one

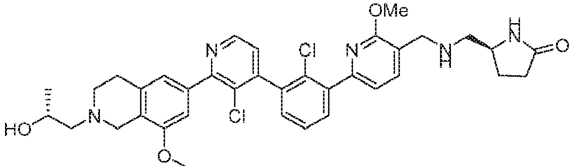

To a mixture of (R)-6-(2-chloro-3-(3-chloro-2-(2-(2-hydroxypropyl)-8-methoxy-1,2,3,4-tetrahydroisoquinolin-6-yl)pyridin-4-yl)phenyl)-2-methoxynicotinaldehyde (0.22 g, 0.39 mmol, 1 eq) and (S)-5-(aminomethyl)pyrrolidin-2-one (0.09 g, 0.77 mmol) in 1:1 (v/v) THF/MeOH (6 mL) was added acetic acid (0.05 g, 0.77 mmol) and 4 Å molecular sieves (1 g). The mixture was then stirred at room temperature for 2 h. Sodium cyanoborohydride (0.06 g, 0.97 mmol) was added and the mixture was stirred at room temperature for 10 min. The reaction was quenched with water (2 mL), then diluted with 50 mL of ethyl acetate, and washed with saturated brine, then concentrated under reduced pressure. The residue was purified by semi-preparative HPLC to provide 55 mg (21%) of (S)-5-((((6-(2-chloro-3-(3-chloro-2-(2-((R)-2-hydroxypropyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one bis formic acid salt. LCMS: m/z found 676 [M+H]⁺, RT = 1.64 min, Method D. ¹H NMR (400 MHz, Methanol-d₄) δ 8.60 (d, 1H), 8.40 (s, 1H), 7.76 (d, 1H), 7.68 (dd, 1H), 7.52 (t, 1H), 7.45 - 7.34 (m, 2H), 7.25 (d, 1H), 7.13 (s, 2H), 4.34 - 4.17 (m, 3H), 4.01 (s, 3H), 3.98 - 3.86 (m, 6H), 3.49 - 3.39 (m, 2H), 3.20 - 3.00 (m, 4H), 2.89 - 2.78 (m, 2H), 2.40 - 2.21 (m, 3H), 1.89 - 1.75 (m, 1H), 1.23 (d, 3H).

### Example 107: (S)-5-((((6-(3-(2-(2-((1-Acetylpiperidin-4-yl)methyl)-8-methoxy-1,2,3,4-tetrahydroisoquinolin-6-yl)-3-chloropyridin-4-yl)-2-chlorophenyl)-2-methoxypyridin-3-yl)methyl)amino)methyl)pyrrolidin-2-one (62)

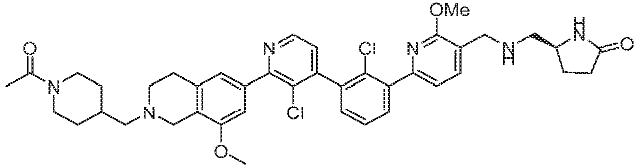

(S)-5-((((6-(3-(2-(2-((1-Acetylpiperidin-4-yl)methyl)-8-methoxy-1,2,3,4-tetrahydroisoquinolin-6-yl)-3-chloropyridin-4-yl)-2-chlorophenyl)-2-methoxypyridin-3-yl)methyl)amino)methyl)pyrrolidin-2-one, compound **62** was prepared in a similar fashion to compound **27** using 1-(4-(bromomethyl) piperidin-1-yl)ethan-1-one and 6-bromo-8-methoxy-1,2,3,4-tetrahydroisoquinoline-hydrochloride salt. LCMS: m/z found 757 [M+H]⁺, RT = 1.74 min (Method D). ¹H NMR (400 MHz, Methanol-d₄) δ 8.58 (d, 1H), 8.43 (s, 1H), 7.75 (d, 1H), 7.68 (dd, 1H), 7.52 (t, 1H), 7.43 - 7.34 (m, 2H), 7.25 (d, 1H), 7.04 (d, 2H), 4.51 (d, 1H), 4.00 (s, 3H), 3.99 - 3.81 (m, 7H), 3.78 (s, 2H), 3.14 (t, 1H), 2.97 (dd, 4H), 2.85 - 2.71 (m, 2H), 2.70 - 2.59 (m, 3H), 2.39 - 2.20 (m, 3H), 2.13 - 1.96 (m, 4H), 1.93 - 1.79 (m, 3H), 1.28 - 1.10 (m, 2H).

### Example 108: (S)-5-((((6-(2-Chloro-3-(3-chloro-2-(2-(3-fluoropropyl)-8-methoxy-1,2,3,4-tetrahydroisoquinolin-6-yl)pyridin-4-yl)phenyl)-2-methoxypyridin-3-yl)methyl) amino)methyl)pyrrolidin-2-one (63)

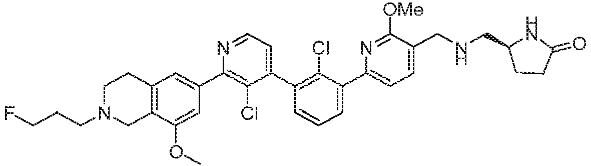

(S)-5-((((6-(2-chloro-3-(3-chloro-2-(2-(3-fluoropropyl)-8-methoxy-1,2,3,4-tetrahydroisoquinolin-6-yl)pyridin-4-yl)phenyl)-2-methoxypyridin-3-yl)methyl) amino)methyl)pyrrolidin-2-one was prepared in a similar fashion to compound 27 using 1-bromo-3-fluoropropane and 6-bromo-8-methoxy-1,2,3,4-tetrahydroisoquinoline-hydrochloride salt. LCMS: m/z found 678 [M+H]⁺RT = 1.74 min (Method D). ¹H NMR (400 MHz, Methanol-d₄) δ 8.58 (d, 1H), 8.48 (s, 1H), 7.69 (dd, 2H), 7.51 (t, 1H), 7.43 - 7.34 (m, 2H), 7.23 (d, 1H), 7.08 - 7.01 (m, 2H), 4.58 (t, 1H), 4.46 (t, 1H), 4.00 (s, 3H), 3.94 - 3.79 (m, 6H), 3.75 (s, 2H), 3.00 (t, 2H), 2.90 (t, 2H), 2.86 - 2.77 (m, 2H), 2.78 - 2.64 (m, 2H), 2.39 - 2.19 (m, 3H), 2.13 - 1.96 (m, 2H), 1.86 - 1.73 (m, 1H).

### Example 109: 2-(((6-(2-Chloro-3-(3-chloro-2-(2-(3-fluoropropyl)-8-methoxy-1,2,3,4-tetrahydroisoquinolin-6-yl)pyridin-4-yl)phenyl)-2-methoxypyridin-3-yl)methyl)amino)ethan-1-ol (84)

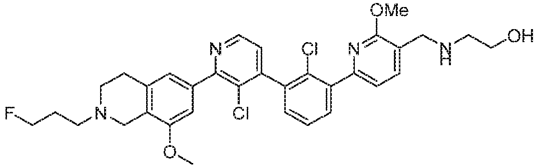

2-(((6-(2-Chloro-3-(3-chloro-2-(2-(3-fluoropropyl)-8-methoxy-1,2,3,4-tetrahydroisoquinolin-6-yl)pyridin-4-yl)phenyl)-2-methoxypyridin-3-yl)methyl)amino)ethan-1-ol was prepared in a similar fashion to compound 27 using 1-bromo-3-fluoropropane and 6-bromo-8-methoxy-1,2,3,4-tetrahydroisoquinoline-hydrochloride salt. LCMS: m/z found 625 [M+H]⁺, RT = 1.72 min (Method D). ¹H NMR (400 MHz, Methanol-d₄) δ 8.57 (d, 1H), 8.48 (s, 1H), 7.82 (d, 1H), 7.68 (dd, 1H), 7.53 (t, 1H), 7.43 - 7.36 (m, 2H), 7.30 (d, 1H), 7.03 (d, 2H), 4.57 (t, 1H), 4.45 (t, 1H), 4.17 (s, 2H), 4.04 (s, 3H), 3.85 (s, 3H), 3.82 - 3.74 (m, 2H), 3.71 (s, 2H), 3.10 - 3.02 (m, 2H), 2.98 (t, J = 5.9 Hz, 2H), 2.86 (t, 2H), 2.83 - 2.74 (m, 2H), 2.11 - 1.93 (m, 2H).

### Example 110: (S)-5-((((6-(2-Chloro-3-(3-chloro-2-(2-(((1r,3r)-3-hydroxycyclobutyl)methyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one (133)

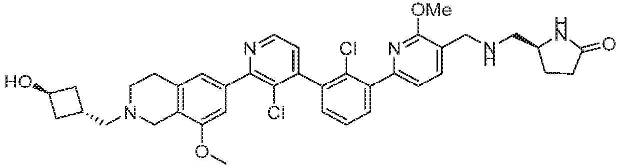

(S)-5-((((6-(2-Chloro-3-(3-chloro-2-(2-(((1r,3r)-3-hydroxycyclobutyl)methyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one was prepared in a similar fashion to compound 27 using (1r,3r)-3-(bromomethyl)cyclobutan-1-ol and 6-bromo-8-methoxy-1,2,3,4-tetrahydroisoquinoline-hydrochloride salt. LCMS: m/z found 702 [M+H]⁺, RT =1.74 min (Method D). ¹H NMR (400 MHz, Methanol-*d*₄) δ 8.57 (d, 1H), 8.49 (s, 1H), 7.75 - 7.63 (m, 2H), 7.51 (t, 1H), 7.42 - 7.33 (m, 2H), 7.22 (d, 1H), 7.03 (d, 2H), 4.31 (t1H), 3.99 (s, 3H), 3.83 (d, 6H), 3.75 (d, 2H), 2.98 (t, 2H), 2.90 (d, 2H), 2.81 (d, 2H), 2.76 - 2.61 (m, 3H), 2.35 - 2.20 (m, 3H), 2.18 - 2.09 (m, 4H), 1.84 - 1.73 (m, 1H).

### Example 111: (S)-5-((((6-(2-Chloro-3-(3-chloro-2-(2-(((1s,3s)-3-hydroxycyclobutyl)methyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one (134)

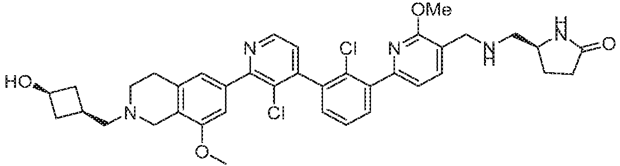

(S)-5-((((6-(2-Chloro-3-(3-chloro-2-(2-(((1s,3s)-3-hydroxycyclobutyl)methyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one was prepared in a similar fashion to compound 27, using (1s,3s)-3-(bromomethyl)cyclobutan-1-ol and 6-bromo-8-methoxy-1,2,3,4-tetrahydroisoquinoline-hydrochloride salt. LCMS: m/z found 702 [M+H] ⁺at 1.74 min (Method D). ¹H NMR (400 MHz, Methanol-d₄) δ 8.57 (d, 1H), 8.49 (s, 1H), 7.75 - 7.63 (m, 2H), 7.51 (t, 1H), 7.42 - 7.33 (m, 2H), 7.22 (d, 1H), 7.04 (d, 2H), 4.14 - 4.02 (m, 1H), 3.99 (s, 3H), 3.84 (d, 6H), 3.77 (s, 2H), 2.96 (dd, 4H), 2.82 (d, 2H), 2.77 - 2.62 (m, 2H), 2.56 - 2.45 (m, 2H), 2.38 - 2.18 (m, 3H), 2.13 (q, 1H), 1.85 - 1.72 (m, 1H), 1.70 - 1.58 (m, 2H).

### Example 112: Methyl (S)-2-(6-(3-chloro-4-(2-chloro-3-(6-methoxy-5-((((5-oxopyrrolidin-2-yl)methyl)amino)methyl)pyridin-2-yl)phenyl)pyridin-2-yl)-8-methoxy-3,4-dihydroisoquinolin-2(1H)-yl)acetate (150)

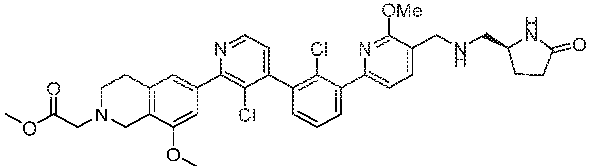

Methyl (S)-2-(6-(3-chloro-4-(2-chloro-3-(6-methoxy-5-((((5-oxopyrrolidin-2-yl)methyl)amino)methyl)pyridin-2-yl)phenyl)pyridin-2-yl)-8-methoxy-3,4-dihydroisoquinolin-2(1H)-yl)acetate was prepared in a similar fashion to compound 27, using methyl 2-bromoacetate and 6-bromo-8-methoxy-1,2,3,4-tetrahydroisoquinoline-hydrochloride salt. LCMS: m/z found 690 [M+H]⁺, RT = 1.75 min (Method D). ¹H NMR (400 MHz, Methanol-d₄) δ 8.56 (d, 1H), 7.75 - 7.63 (m, 2H), 7.51 (t, 1H), 7.42 - 7.32 (m, 2H), 7.22 (d, 1H), 7.02 (d, 2H), 3.99 (s, 3H), 3.86 - 3.74 (m, 6H), 3.73 (d, 5H), 3.47 (s, 2H), 2.96 (t, 2H), 2.86 (t, 2H), 2.74 - 2.60 (m, 2H), 2.38 - 2.17 (m, 3H), 1.84 - 1.72 (m, 1H).

### Example 113: (S)-2-(6-(3-Chloro-4-(2-chloro-3-(6-methoxy-5-((((5-oxopyrrolidin-2-yl)methyl)amino)methyl)pyridin-2-yl)phenyl)pyridin-2-yl)-8-methoxy-3,4-dihydroisoquinolin-2(1H)-yl)acetic acid (151)

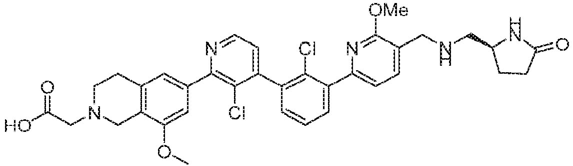

To a solution of methyl (S)-2-(6-(3-chloro-4-(2-chloro-3-(6-methoxy-5-((((5-oxopyrrolidin-2-yl)methyl)amino)methyl)pyridin-2-yl)phenyl)pyridin-2-yl)-8-methoxy-3,4-dihydroisoquinolin-2(1H)-yl)acetate (0.03 g, 0.04 mmol) in dioxane (1.5 mL), was added a solution of lithium hydroxide monohydrate (0.003 g, 0.07 mmol) in water (0.5 mL). The mixture was stirred for 5 minutes at room temperature, then purified directly by semi-preparative HPLC to provide 22 mg (92%) of (S)-2-(6-(3-chloro-4-(2-chloro-3-(6-methoxy-5-((((5-oxopyrrolidin-2-yl)methyl)amino)methyl)pyridin-2-yl)phenyl)pyridin-2-yl)-8-methoxy-3,4-dihydroisoquinolin-2(1H)-yl)acetic acid bis formic acid salt. LCMS: m/z found 676 [M+H]⁺, RT = 1.63 min (Method D). ¹H NMR (400 MHz, Methanol-d₄) δ 8.61 (d, 1H), 8.46 (s, 1H), 7.78 - 7.65 (m, 2H), 7.53 (t, 1H), 7.46 - 7.35 (m, 2H), 7.24 (d, 1H), 7.15 (d, 2H), 4.41 (s, 2H), 4.00 (s, 3H), 3.95 - 3.79 (m, 6H), 3.79 (s, 2H), 3.58 (t, 2H), 3.22 (t, 2H), 2.83 - 2.68 (m, 2H), 2.41 - 2.20 (m, 3H), 1.88 - 1.74 (m, 1H).

### Example 114: (5S)-5-[[[4-[4-[3-[3-[[(1-Acetyl-4-piperidyl)amino]methyl]-1-methyl-pyrrolo[2,3-b]pyridin-6-yl]-2-chloro-phenyl]-3-chloro-2-pyridyl]-2-methoxyphenyl]methylamino] methyl]pyrrolidin-2-one (57)

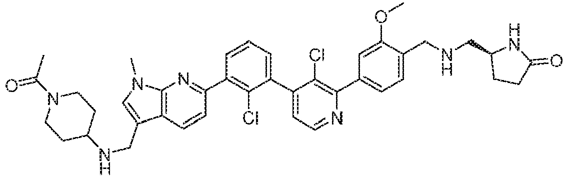

### (a) tert-Butyl N-[[4-[3-chloro-4-[2-chloro-3-(3-formyl-1-methyl-pyrrolo[2,3-b]pyridin-6-yl)phenyl]-2-pyridyl]-2-methoxy-phenyl]methyl]-N-[[(2S)-5-oxopyrrolidin-2-yl] methyl] carbamate

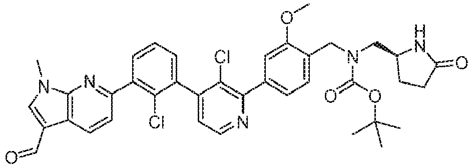

*tert-Butyl* N-[[4-[3-chloro-4-[2-chloro-3-(3-formyl-1-methyl-pyrrolo[2,3-b]pyridin-6-yl)phenyl]-2-pyridyl]-2-methoxy-phenyl]methyl]-N-[[(2S)-5-oxopyrrolidin-2-yl]methyl]carbamate was prepared by following Suzuki Coupling Procedure B from *tert-*butyl N-[[4-[4-(3-bromo-2-chloro-phenyl)-3-chloro-2-pyridyl]-2-methoxy-phenyl]methyl]-N-[[(2S)-5-oxopyrrolidin-2-yl]methyl]carbamate (315 mg, 0.50 mmol) and (3-formyl-1-methyl-pyrrolo[2,3-b]pyridin-6-yl)boronic acid (101 mg, 0.50 mmol). 320 mg, 90% yield. MS: m/z found 714.2 [M+H]⁺.

### (b) (5S)-5-[[[4-[4-[3-[3-[[(1-Acetyl-4-piperidyl)amino]methyl]-1-methyl-pyrrolo[2,3-b]pyridin-6-yl]-2-chloro-phenyl]-3-chloro-2-pyridyl]-2-methoxy-phenyl]methylamino] methyl]pyrrolidin-2-one

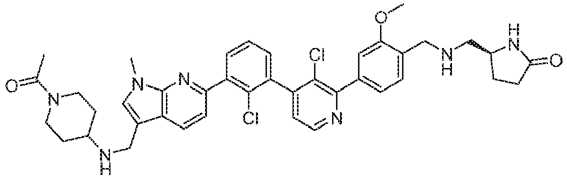

Reductive Amination Procedure B from *tert-butyl* N-[[4-[3-chloro-4-[2-chloro-3-(3-formyl-1-methyl-pyrrolo[2,3-b]pyridin-6-yl)phenyl]-2-pyridyl]-2-methoxy-phenyl]methyl]-N-[[(2S)-5-oxopyrrolidin-2-yl]methyl]carbamate (30 mg, 0.04 mmol) and 1-(4-amino-1-piperidyl)ethanone (12 mg, 0.08 mmol) gave *tert-butyl* N-[[4-[4-[3-[3-[[(1-acetyl-4-piperidyl)amino]methyl]-1-methyl-pyrrolo[2,3-b]pyridin-6-yl]-2-chloro-phenyl]-3-chloro-2-pyridyl]-2-methoxy-phenyl]methyl]-N-[[(2S)-5-oxopyrrolidin-2-yl]methyl]carbamate (88% LCMS yield) as crude, MS: m/z found 840.3 [M+H]⁺, which was deprotected by General Boc Deprotection Procedure and purified to afford the final product as formic acid salt. 98% yield. MS: m/z found 740.3 [M+H]⁺. ¹H NMR (400 MHz, Methanol-d₄): δ 8.64 (d, 1H), 8.52 (s, 2H), 8.24 (d, 1H), 7.71 (dd, 1H), 7.64 (s, 1H), 7.59 (t, 1H), 7.48 - 7.41 (m, 4H), 7.33 - 7.27 (m, 2H), 4.63 (d, 1H), 4.39 (s, 2H), 4.09 - 3.84 (m, 11H), 3.19 (t, 1H), 2.85 - 2.74 (m, 2H), 2.70 (t, 1H), 2.37 - 2.27 (m, 3H), 2.22 (t, 2H), 2.13 (s, 3H), 1.86 - 1.76 (m, 1H), 1.57 (m, 1H), 1.46 (m, 1H).

### Example 115: (5S)-5-[[[4-[3-Chloro-4-[2-chloro-3-[1-methyl-3-[[[(2S)-oxetan-2-yl]methylamino]methyl]pyrrolo[2,3-b]pyridin-6-yl]phenyl]-2-pyridyl]-2-methoxyphenyl]methylamino]methyl]pyrrolidin-2-one (58)

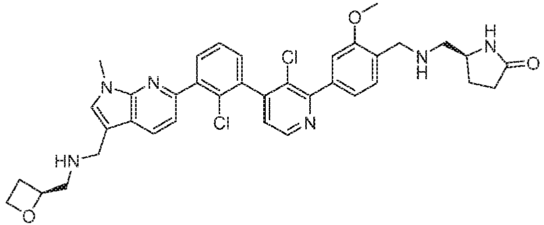

Reductive Amination Procedure B from *tert*-butyl N-[[4-[3-chloro-4-[2-chloro-3-(3-formyl-1-methyl-pyrrolo[2,3-b]pyridin-6-yl)phenyl]-2-pyridyl]-2-methoxy-phenyl]methyl]-N-[[(2S)-5-oxopyrrolidin-2-yl]methyl]carbamate (30 mg, 0.04 mmol) and [(2S)-oxetan-2-yl]methanamine (7 mg, 0.08 mmol) gave *tert-butyl* N-[[4-[3-chloro-4-[2-chloro-3-[1-methyl-3-[[[(2S)-oxetan-2-yl]methylamino]methyl]pyrrolo[2,3-b]pyridin-6-yl]phenyl]-2-pyridyl]-2-methoxy-phenyl]methyl]-N-[[(2S)-5-oxopyrrolidin-2-yl]methyl]carbamate (88% LCMS yield) as crude, MS: m/z found 785.3 [M+H]⁺, which was deprotected by General Boc Deprotection Procedure and purified to afford the final product as formic acid salt. 97% yield. MS: m/z found 685.2 [M+H]⁺. ¹H NMR (400 MHz, Methanol-d₄): δ 8.64 (d, 1H), 8.53 (s, 2H), 8.24 (d, 1H), 7.71 (dd, 1H), 7.63 - 7.54 (m, 2H), 7.48 - 7.39 (m, 4H), 7.33 - 7.25 (m, 2H), 5.06 (m, 1H), 4.77 - 4.66 (m, 1H), 4.61 (dt, 1H), 4.40 - 4.28 (m, 2H), 4.02 - 3.89 (m, 8H), 3.87 (t, 1H), 3.39 - 3.32 (m, 1H), 3.14 (dd, 1H), 2.86 - 2.70 (m, 3H), 2.57 - 2.43 (m, 1H), 2.38 - 2.24 (m, 3H), 1.87 - 1.75 (m, 1H).

### Example 116: (5S)-5-[[[4-[3-Chloro-4-[2-chloro-3-[1-methyl-3-[(tetrahydropyran-4-ylamino)methyl]pyrrolo[2,3-b]pyridin-6-yl]phenyl]-2-pyridyl]-2-methoxyphenyl]methylamino]methyl]pyrrolidin-2-one (59)

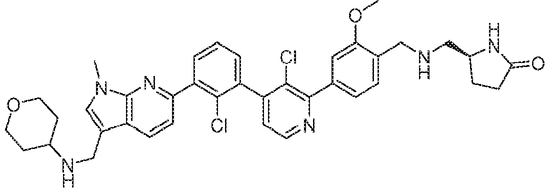

Reductive Amination Procedure B from *tert*-butyl N-[[4-[3-chloro-4-[2-chloro-3-(3-formyl-1-methyl-pyrrolo[2,3-b]pyridin-6-yl)phenyl]-2-pyridyl]-2-methoxy-phenyl]methyl]-N-[[(2S)-5-oxopyrrolidin-2-yl]methyl]carbamate (30 mg, 0.04 mmol) and tetrahydropyran-4-amine (9 mg, 0.08 mmol) gave *tert-butyl* N-[[4-[3-chloro-4-[2-chloro-3-[1-methyl-3-[(tetrahydropyran-4-ylamino)methyl]pyrrolo[2,3-b]pyridin-6-yl]phenyl]-2-pyridyl]-2-methoxy-phenyl]methyl]-N-[[(2S)-5-oxopyrrolidin-2-yl]methyl]carbamate (89% LCMS yield) as crude, MS: m/z found 799.3 [M+H]⁺, which was deprotected by General Boc Deprotection Procedure and purified to afford the final product as formic acid salt. 97% yield. MS: m/z found 699.3 [M+H]⁺. ¹H NMR (400 MHz, Methanol-d₄): δ 8.64 (d, 1H), 8.52 (s, 2H), 8.25 (d, 1H), 7.72 (dd, 1H), 7.67 (s, 1H), 7.60 (t, 1H), 7.50 - 7.41 (m, 4H), 7.33 - 7.26 (m, 2H), 4.44 (s, 2H), 4.06 (dd, 2H), 4.02 - 3.96 (m, 2H), 3.96 - 3.93 (m, 6H), 3.88 (t, 1H), 3.53 - 3.35 (m, 3H), 2.85 - 2.73 (m, 2H), 2.39 - 2.26 (m, 3H), 2.13 (d, 2H), 1.86 - 1.78 (m, 1H), 1.70 (m, 2H).

### Example 117: (5S)-5-[[[4-[3-Chloro-4-[2-chloro-3-[3-[[(1,1-dioxothian-4-yl)amino]methyl]-1-methyl-pyrrolo[2,3-b]pyridin-6-yl]phenyl]-2-pyridyl]-2-methoxyphenyl]methylamino]methyl]pyrrolidin-2-one (60)

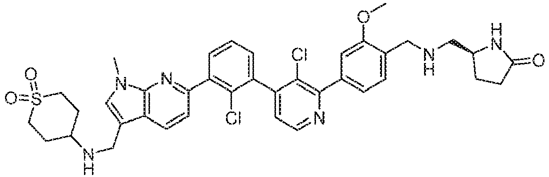

Reductive Amination Procedure B from *tert*-butyl N-[[4-[3-chloro-4-[2-chloro-3-(3-formyl-1-methyl-pyrrolo[2,3-b]pyridin-6-yl)phenyl]-2-pyridyl]-2-methoxy-phenyl]methyl]-N-[[(2S)-5-oxopyrrolidin-2-yl]methyl]carbamate (30 mg, 0.04 mmol) and 1,1-dioxothian-4-amine (9 mg, 0.08 mmol) gave *tert-butyl* N-[[4-[3-chloro-4-[2-chloro-3-[3-[[(1,1-dioxothian-4-yl)amino]methyl]-1-methyl-pyrrolo[2,3-b]pyridin-6-yl]phenyl]-2-pyridyl]-2-methoxyphenyl]methyl]-N-[[(2S)-5-oxopyrrolidin-2-yl]methyl]carbamate (87% LCMS yield) as crude, MS: m/z found 847.3 [M+H]⁺, which was deprotected by General Boc Deprotection Procedure and purified to afford the final product as formic acid salt. 98% yield. MS: m/z found 747.2 [M+H]⁺. ¹H NMR (400 MHz, Methanol-d₄): δ 8.65 (d, 1H), 8.50 (s, 2H), 8.20 (d, 1H), 7.71 (dd, 1H), 7.58 (t, 1H), 7.50 (s, 1H), 7.48 - 7.43 (m, 3H), 7.39 (d, 1H), 7.35 - 7.28 (m, 2H), 4.15 (s, 2H), 4.12 - 4.01 (m, 2H), 3.96 (s, 3H), 3.94 - 3.90 (m, 1H), 3.90 (s, 3H), 3.24 - 3.06 (m, 5H), 2.97 - 2.83 (m, 2H), 2.43 - 2.27 (m, 5H), 2.19 - 2.05 (m, 2H), 1.89 - 1.78 (m, 1H).

### Example 118: (5S)-5-[[[4-[4-[3-[3-[[(1-Acetyl-4-piperidyl)-methyl-amino]methyl]-1-methyl-pyrrolo[2,3-b]pyridin-6-yl]-2-chloro-phenyl]-3-chloro-2-pyridyl]-2-methoxyphenyl]methylamino] methyl]pyrrolidin-2-one (92)

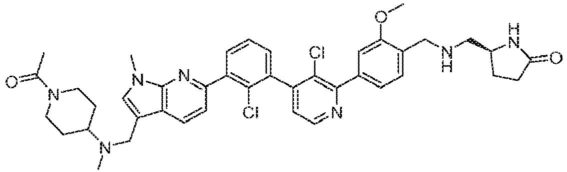

Reductive Amination Procedure B from *tert*-butyl N-[[4-[3-chloro-4-[2-chloro-3-(3-formyl-1-methyl-pyrrolo[2,3-b]pyridin-6-yl)phenyl]-2-pyridyl]-2-methoxy-phenyl]methyl]-N-[[(2S)-5-oxopyrrolidin-2-yl]methyl]carbamate (30 mg, 0.04 mmol) and 1-[4-(methylamino)-1-piperidyl]ethanone (13 mg, 0.08 mmol) gave *tert-butyl* N-[[4-[4-[3-[3-[[(1-acetyl-4-piperidyl)-methyl-amino]methyl]-1-methyl-pyrrolo[2,3-b]pyridin-6-yl]-2-chlorophenyl]-3-chloro-2-pyridyl]-2-methoxy-phenyl]methyl]-N-[[(2S)-5-oxopyrrolidin-2-yl]methyl]carbamate (61% LCMS yield) as crude, MS: m/z found 854.4 [M+H]⁺, which was deprotected by General Boc Deprotection Procedure and purified to afford the final product as formic acid salt. 96% LCMS yield. MS: m/z found 754.3 [M+H]⁺. ¹H NMR (400 MHz, Methanol-d₄): δ 8.64 (d, 1H), 8.51 (s, 2H), 8.20 (d, 1H), 7.72 (dd, 1H), 7.62 - 7.54 (m, 2H), 7.49 - 7.39 (m, 4H), 7.32 (s, 1H), 7.31 - 7.26 (m, 1H), 4.67 (d, 1H), 4.17 (s, 2H), 4.09 - 3.98 (m, 3H), 3.98 - 3.85 (m, 2H), 3.95 (s, 3H), 3.92 (s, 3H), 3.14 (t, 2H), 2.89 - 2.76 (m, 2H), 2.68 - 2.58 (m, 1H), 2.51 (s, 3H), 2.40 - 2.23 (m, 3H), 2.13 (s, 3H), 2.11 - 2.05 (m, 2H), 1.86 - 1.55 (m, 2H).

### Example 119: (5S)-5-[[[4-[3-Chloro-4-[2-chloro-3-[1-methyl-3-[[[(2S)-tetrahydrofuran-2-yl]methylamino]methyl]pyrrolo[2,3-b]pyridin-6-yl]phenyl]-2-pyridyl]-2-methoxyphenyl]methylamino]methyl]pyrrolidin-2-one (93)

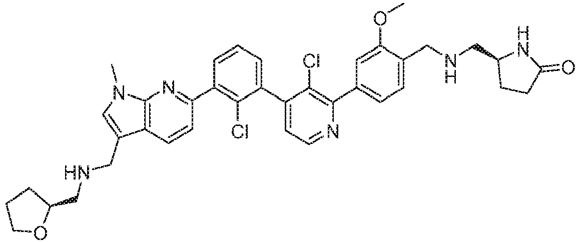

Reductive Amination Procedure B from *tert*-butyl N-[[4-[3-chloro-4-[2-chloro-3-(3-formyl-1-methyl-pyrrolo[2,3-b]pyridin-6-yl)phenyl]-2-pyridyl]-2-methoxy-phenyl]methyl]-N-[[(2S)-5-oxopyrrolidin-2-yl]methyl]carbamate (30 mg, 0.04 mmol) and [(2S)-tetrahydrofuran-2-yl]methanamine (9 mg, 0.08 mmol) gave *tert-butyl* N-[[4-[3-chloro-4-[2-chloro-3-[1-methyl-3-[[[(2S)-tetrahydrofuran-2-yl]methylamino]methyl]pyrrolo[2,3-b]pyridin-6-yl]phenyl]-2-pyridyl]-2-methoxy-phenyl]methyl]-N-[[(2S)-5-oxopyrrolidin-2-yl]methyl]carbamate (77% LCMS yield) as crude, MS: m/z found 799.3 [M+H]⁺, which was deprotected by General Boc Deprotection Procedure and purified to afford the final product as formic acid salt. 97% LCMS yield. MS: m/z found 699.3 [M+H]⁺. ¹H NMR (400 MHz, Methanol-*d*₄): δ 8.64 (d, 1H), 8.54 (s, 2H), 8.23 (d, 1H), 7.71 (dd, 1H), 7.62 (s, 1H), 7.59 (t, 1H), 7.46 (dt, 3H), 7.41 (d, 1H), 7.32 - 7.24 (m, 2H), 4.44 - 4.31 (m, 2H), 4.21 - 4.09 (m, 1H), 3.97 - 3.76 (m, 11H), 3.11 (m, 1H), 2.94 (m, 1H), 2.78 - 2.63 (m, 2H), 2.38 - 2.23 (m, 3H), 2.10 (m, 1H), 2.00 - 1.88 (m, 2H), 1.85 - 1.73 (m , 1H), 1.67 - 1.51 (m, 1H).

### Example 120: (5S)-5-[[[4-[3-Chloro-4-[2-chloro-3-[1-methyl-3-(methylaminomethyl)pyrrolo[2,3-b]pyridin-6-yl]phenyl]-2-pyridyl]-2-methoxyphenyl]methylamino]methyl]pyrrolidin-2-one (94)

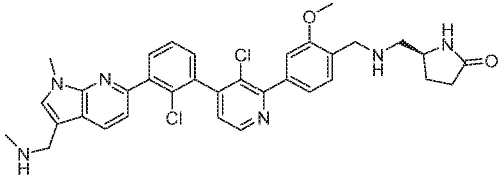

Reductive Amination Procedure B from *tert*-butyl N-[[4-[3-chloro-4-[2-chloro-3-(3-formyl-1-methyl-pyrrolo[2,3-b]pyridin-6-yl)phenyl]-2-pyridyl]-2-methoxy-phenyl]methyl]-N-[[(2S)-5-oxopyrrolidin-2-yl]methyl]carbamate (30 mg, 0.04 mmol) and methanamine solution (33% wt in ethanol, 0.08 mmol) gave *tert*-butyl N-[[4-[3-chloro-4-[2-chloro-3-[1-methyl-3-(methylaminomethyl)pyrrolo[2,3-b]pyridin-6-yl]phenyl]-2-pyridyl]-2-methoxyphenyl]methyl]-N-[[(2S)-5-oxopyrrolidin-2-yl]methyl]carbamate (78% LCMS yield) as crude, MS: m/z found 729.3 [M+H]⁺, which was deprotected and purified by General Boc Deprotection Procedure to afford the final product as formic acid salt. 97% LCMS yield. MS: m/z found 629.2 [M+H]⁺. ¹H NMR (400 MHz, Methanol-d₄): δ 8.64 (d, 1H), 8.52 (s, 2H), 8.25 (d, 1H), 7.71 (dd, 1H), 7.67 (s, 1H), 7.59 (t, 1H), 7.50 - 7.39 (m, 4H), 7.32 - 7.24 (m, 2H), 4.41 (s, 2H), 4.01 - 3.95 (m, 2H), 3.94 (s, 6H), 3.87 (m, 1H), 2.83 - 2.75 (m, 2H), 2.37 - 2.23 (m, 3H), 1.86 - 1.74 (m, 1H).

### Example 121: (5S)-5-[[[4-[4-[3-[3-[[(1-Acetylazetidin-3-yl)amino]methyl]-1-methyl-pyrrolo[2,3-b]pyridin-6-yl]-2-chloro-phenyl]-3-chloro-2-pyridyl]-2-methoxyphenyl]methylamino]methyl]pyrrolidin-2-one (95)

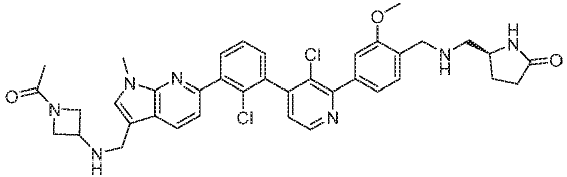

Reductive Amination Procedure B from *tert*-butyl N-[[4-[3-chloro-4-[2-chloro-3-(3-formyl-1-methyl-pyrrolo[2,3-b]pyridin-6-yl)phenyl]-2-pyridyl]-2-methoxy-phenyl]methyl]-N-[[(2S)-5-oxopyrrolidin-2-yl]methyl]carbamate (30 mg, 0.04 mmol) and 1-(3-aminoazetidin-1-yl)ethanone (10 mg, 0.08 mmol) gave *tert-butyl* N-[[4-[4-[3-[3-[[(1-acetylazetidin-3-yl)amino]methyl]-1-methyl-pyrrolo[2,3-b]pyridin-6-yl]-2-chloro-phenyl]-3-chloro-2-pyridyl]-2-methoxy-phenyl]methyl]-N-[[(2S)-5-oxopyrrolidin-2-yl]methyl]carbamate (73% LCMS yield) as crude, MS: m/z found 812.3 [M+H]⁺, which was deprotected and purified by General Boc Deprotection Procedure to afford the final product as formic acid salt. 96% LCMS yield. MS: m/z found 712.3 [M+H]⁺. ¹H NMR (400 MHz, Methanol-d₄): δ 8.65 (d, 1H), 8.49 (s, 2H), 8.19 (d, 1H), 7.71 (dd, 1H), 7.58 (t, 1H), 7.50 - 7.41 (m, 4H), 7.38 (d, 1H), 7.36 - 7.29 (m, 2H), 4.34 (t, 1H), 4.14 - 4.04 (m, 3H), 4.01 - 3.90 (m, 7H), 3.89 (s, 3H), 3.81 - 3.72 (m, 1H), 3.72 - 3.65 (m, 1H), 3.00 - 2.87 (m, 2H), 2.41 - 2.28 (m, 3H), 1.92 - 1.76 (m, 1H), 1.84 (s, 3H).

### Example 122: (5S)-5-[[[4-[3-Chloro-4-[2-chloro-3-[3-[[(4,4-difluorocyclohexyl)amino]methyl]-1-methyl-pyrrolo[2,3-b]pyridin-6-yl]phenyl]-2-pyridyl]-2-methoxy-phenyl]methylamino]methyl] pyrrolidin-2-one (96)

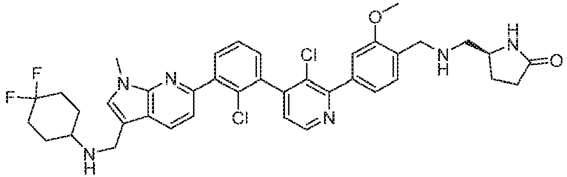

Reductive Amination Procedure B from *tert-butyl* N-[[4-[3-chloro-4-[2-chloro-3-(3-formyl-1-methyl-pyrrolo[2,3-b]pyridin-6-yl)phenyl]-2-pyridyl]-2-methoxy-phenyl]methyl]-N-[[(2S)-5-oxopyrrolidin-2-yl]methyl]carbamate (30 mg, 0.04 mmol) and 4,4-difluorocyclohexanamine (10 mg, 0.08 mmol) gave *tert-butyl* N-[[4-[3-chloro-4-[2-chloro-3-[3-[[(4,4-difluorocyclohexyl)amino]methyl]-1-methyl-pyrrolo[2,3-b]pyridin-6-yl]phenyl]-2-pyridyl]-2-methoxy-phenyl]methyl]-N-[[(2S)-5-oxopyrrolidin-2-yl]methyl]carbamate (74% LCMS yield) as crude, MS: m/z found 833.3 [M+H]⁺, which was deprotected and purified by General Boc Deprotection Procedure to afford the final product as formic acid salt. 97% LCMS yield. MS: m/z found 733.3 [M+H]⁺. ¹H NMR (400 MHz, Methanol-d₄): δ 8.64 (d, 1H), 8.53 (s, 2H), 8.22 (d, 1H), 7.71 (dd, 1H), 7.62 - 7.55 (m, 2H), 7.48 - 7.38 (m, 4H), 7.33 - 7.24 (m, 2H), 4.31 (s, 2H), 3.98 - 3.89 (m, 8H), 3.89 - 3.80 (m, 1H), 3.19 - 3.06 (m, 1H), 2.80 - 2.67 (m, 2H), 2.37 - 2.25 (m, 3H), 2.24 - 2.10 (m, 4H), 2.00 - 1.76 (m, 3H), 1.73 - 1.57 (m, 2H).

### Example 123: (5S)-5-[[[4-[3-Chloro-4-[2-chloro-3-[3-[(2-hydroxyethylamino)methyl]-1-methyl-pyrrolo[2,3-b]pyridin-6-yl]phenyl]-2-pyridyl]-2-methoxyphenyl]methylamino]methyl]pyrrolidin-2-one (97)

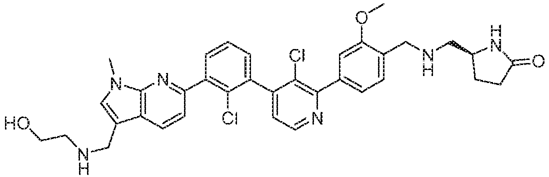

Reductive Amination Procedure B from *tert*-butyl N-[[4-[3-chloro-4-[2-chloro-3-(3-formyl-1-methyl-pyrrolo[2,3-b]pyridin-6-yl)phenyl]-2-pyridyl]-2-methoxy-phenyl]methyl]-N-[[(2S)-5-oxopyrrolidin-2-yl]methyl]carbamate (30 mg, 0.04 mmol) and 2-aminoethanol (5 mg, 0.08 mmol) gave *tert-butyl* N-[[4-[3-chloro-4-[2-chloro-3-[3-[(2-hydroxyethylamino)methyl]-1-methyl-pyrrolo[2,3-b]pyridin-6-yl]phenyl]-2-pyridyl]-2-methoxy-phenyl]methyl]-N-[[(2S)-5-oxopyrrolidin-2-yl]methyl]carbamate (88% LCMS yield) as crude, MS: m/z found 759.3 [M+H]⁺, which was deprotected and purified by General Boc Deprotection Procedure to afford the final product as formic acid salt. 97% yield. MS: m/z found 659.2 [M+H]⁺. ¹H NMR (400 MHz, Methanol-d₄): δ 8.64 (d, 1H), 8.53 (s, 2H), 8.25 (d, 1H), 7.71 (dd, 1H), 7.67 (s, 1H), 7.59 (t, 1H), 7.49 - 7.45 (m, 3H), 7.42 (d, 1H), 7.31 - 7.24 (m, 2H), 4.44 (s, 2H), 3.98 - 3.89 (m, 8H), 3.90 - 3.84 (m, 1H), 3.83 - 3.79 (m, 2H), 3.18 - 3.08 (m, 2H), 2.80 - 2.67 (m, 2H), 2.40 - 2.24 (m, 3H), 1.85 - 1.75 (m, 1H).

### Example 124: (5S)-5-[[[4-[3-Chloro-4-[2-chloro-3-[3-[[[(2S)-2-hydroxypropyl]amino]methyl]-1-methyl-pyrrolo[2,3-b]pyridin-6-yl]phenyl]-2-pyridyl]-2-methoxy-phenyl]methylamino]methyl]pyrrolidin-2-one (98)

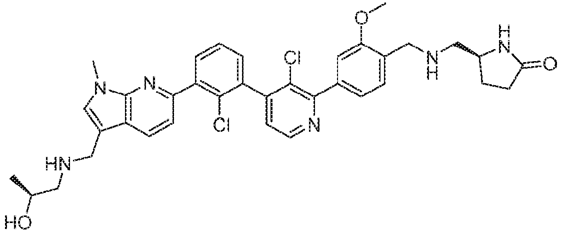

Reductive Amination Procedure B from *tert*-butyl N-[[4-[3-chloro-4-[2-chloro-3-(3-formyl-1-methyl-pyrrolo[2,3-b]pyridin-6-yl)phenyl]-2-pyridyl]-2-methoxy-phenyl]methyl]-N-[[(2S)-5-oxopyrrolidin-2-yl]methyl]carbamate (30 mg, 0.04 mmol) and (2S)-1-aminopropan-2-ol (6 mg, 0.08 mmol) gave *tert-butyl* N-[[4-[3-chloro-4-[2-chloro-3-[3-[[[(2S)-2-hydroxypropyl]amino]methyl]-1-methyl-pyrrolo[2,3-b]pyridin-6-yl]phenyl]-2-pyridyl]-2-methoxy-phenyl]methyl]-N-[[(2S)-5-oxopyrrolidin-2-yl]methyl]carbamate (86% LCMS yield) as crude, MS: m/z found 773.3 [M+H]⁺, which was deprotected and purified by General Boc Deprotection Procedure to afford the final product as formic acid salt. 98% LCMS yield. MS: m/z found 673.2 [M+H]⁺. ¹H NMR (400 MHz, Methanol-d₄): δ 8.64 (d, 1H), 8.54 (s, 2H), 8.24 (d, 1H), 7.71 (dd, 1H), 7.65 (s, 1H), 7.59 (t, 1H), 7.51 - 7.44 (m, 3H), 7.42 (d, 1H), 7.31 - 7.23 (m, 2H), 4.40 (s, 2H), 4.03 (dd, 1H), 3.97 - 3.89 (m, 8H), 3.89 - 3.82 (m, 1H), 3.09 - 3.00 (m, 1H), 2.85 (dd, 1H), 2.76 - 2.67 (m, 2H), 2.38 - 2.22 (m, 3H), 1.89 - 1.73 (m, 1H), 1.21 (d, 3H).

### Example 125: (5S)-5-[[[4-[3-Chloro-4-[2-chloro-3-[3-[[(2-hydroxy-2-methylpropyl)amino]methyl]-1-methyl-pyrrolo[2,3-b]pyridin-6-yl]phenyl]-2-pyridyl]-2-methoxy-phenyl]methylamino]methyl]pyrrolidin-2-one (99)

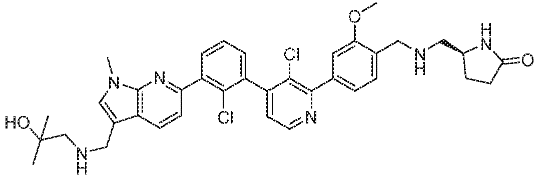

Reductive Amination Procedure B from *tert*-butyl N-[[4-[3-chloro-4-[2-chloro-3-(3-formyl-1-methyl-pyrrolo[2,3-b]pyridin-6-yl)phenyl]-2-pyridyl]-2-methoxy-phenyl]methyl]-N-[[(2S)-5-oxopyrrolidin-2-yl]methyl]carbamate (30 mg, 0.04 mmol) and 1-amino-2-methyl-propan-2-ol (7 mg, 0.08 mmol) gave *tert*-butyl N-[[4-[3-chloro-4-[2-chloro-3-[3-[[(2-hydroxy-2-methyl-propyl)amino]methyl]-1-methyl-pyrrolo[2,3-b]pyridin-6-yl]phenyl]-2-pyridyl]-2-methoxy-phenyl]methyl]-N-[[(2S)-5-oxopyrrolidin-2-yl]methyl]carbamate (87% LCMS yield) as crude, MS: m/z found 787.3 [M+H]⁺, which was deprotected and purified by General Boc Deprotection Procedure to afford the final product as formic acid salt. 96% LCMS yield. MS: m/z found 687.3 [M+H]⁺. ¹H NMR (400 MHz, Methanol-d₄): δ 8.64 (d, 1H), 8.54 (s, 2H), 8.25 (d, 1H), 7.75 - 7.68 (m, 1H), 7.64 (s, 1H), 7.59 (t, 1H), 7.48 - 7.44 (m, 3H), 7.41 (d, 1H), 7.31 - 7.22 (m, 2H), 4.37 (s, 2H), 3.93 (s, 6H), 3.90 (d, 2H), 3.87 - 3.81 (m, 1H), 2.90 (s, 2H), 2.77 - 2.67 (m, 2H), 2.38 - 2.22 (m, 3H), 1.79 (dd, 1H), 1.25 (s, 6H).

### Example 126: (5S)-5-[[[4-[3-Chloro-4-[2-chloro-3-[3-[(2-methoxyethylamino)methyl]-1-methyl-pyrrolo[2,3-b]pyridin-6-yl]phenyl]-2-pyridyl]-2-methoxyphenyl]methylamino]methyl]pyrrolidin-2-one (100)

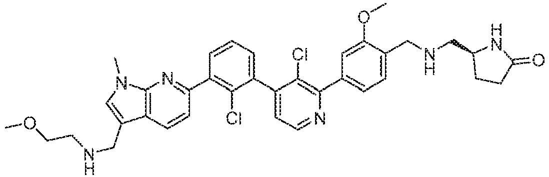

Reductive Amination Procedure B from *tert*-butyl N-[[4-[3-chloro-4-[2-chloro-3-(3-formyl-1-methyl-pyrrolo[2,3-b]pyridin-6-yl)phenyl]-2-pyridyl]-2-methoxy-phenyl]methyl]-N-[[(2S)-5-oxopyrrolidin-2-yl]methyl]carbamate (30 mg, 0.04 mmol) and 2-methoxyethanamine (6 mg, 0.08 mmol) gave *tert*-butyl N-[[4-[3-chloro-4-[2-chloro-3-[3-[(2-methoxyethylamino)methyl]-1-methyl-pyrrolo[2,3-b]pyridin-6-yl]phenyl]-2-pyridyl]-2-methoxy-phenyl]methyl]-N-[[(2S)-5-oxopyrrolidin-2-yl]methyl]carbamate (83% LCMS yield) as crude, MS: m/z found 773.3 [M+H]⁺, which was deprotected and purified by General Boc Deprotection Procedure to afford the final product as formic acid salt. 96% LCMS yield. MS: m/z found 673.2 [M+H]⁺. ¹H NMR (400 MHz, Methanol-d₄): δ 8.64 (d, 1H), 8.54 (s, 2H), 8.24 (d, 1H), 7.71 (dd, 1H), 7.65 (s, 1H), 7.59 (t, 1H), 7.50 - 7.44 (m, 3H), 7.42 (d, 1H), 7.31 - 7.20 (m, 2H), 4.40 (s, 2H), 3.97 - 3.90 (m, 8H), 3.90 - 3.83 (m, 1H), 3.68 - 3.60 (m, 2H), 3.41 (s, 3H), 3.20 (t, 2H), 2.80 - 2.67 (m, 2H), 2.38 - 2.22 (m, 3H), 1.87 - 1.74 (m, 1H).

### Example 127: (5S)-5-[[[4-[3-Chloro-4-[2-chloro-3-[3-[[2-hydroxyethyl(methyl)amino]methyl]-1-methyl-pyrrolo[2,3-b]pyridin-6-yl]phenyl]-2-pyridyl]-2-methoxy-phenyl]methylamino]methyl] pyrrolidin-2-one (101)

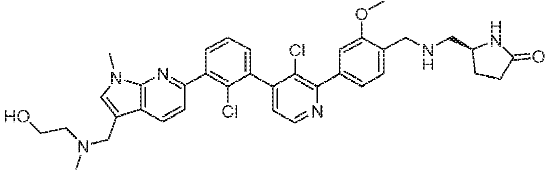

Reductive Amination Procedure B from *tert*-butyl N-[[4-[3-chloro-4-[2-chloro-3-(3-formyl-1-methyl-pyrrolo[2,3-b]pyridin-6-yl)phenyl]-2-pyridyl]-2-methoxy-phenyl]methyl]-N-[[(2S)-5-oxopyrrolidin-2-yl]methyl]carbamate (30 mg, 0.04 mmol) and 2-(methylamino)ethanol (6 mg, 0.08 mmol) gave *tert-butyl* N-[[4-[3-chloro-4-[2-chloro-3-[3-[[2-hydroxyethyl(methyl)amino] methyl]-1-methyl-pyrrolo[2,3-b]pyridin-6-yl]phenyl]-2-pyridyl]-2-methoxy-phenyl]methyl]-N-[[(2S)-5-oxopyrrolidin-2-yl]methyl]carbamate (80% LCMS yield) as crude, MS: m/z found 773.3 [M+H]⁺, which was deprotected and purified by General Boc Deprotection Procedure to afford the final product as formic acid salt. 97% LCMS yield. MS: m/z found 673.2 [M+H]⁺. ¹H NMR (400 MHz, Methanol-d₄): δ 8.64 (d, 1H), 8.54 (s, 2H), 8.23 (d, 1H), 7.71 (dd, 1H), 7.63 - 7.55 (m, 2H), 7.48 - 7.38 (m, 4H), 7.32 - 7.20 (m, 2H), 4.21 (s, 2H), 3.96 - 3.89 (m, 8H), 3.89 - 3.84 (m, 1H), 3.82 (t, 2H), 3.02 - 2.91 (m, 2H), 2.77 - 2.68 (m, 2H), 2.61 (s, 3H), 2.37 - 2.22 (m, 3H), 1.80 (m, 1H).

### Example 128: (S)-5-((((6-(2-chloro-3-(3-chloro-2-(3-methoxy-4-(((((S)-5-oxopyrrolidin-2-yl)-methyl)-amino)-methyl)-phenyl)pyridin-4-yl)phenyl)-1-methyl-1H-pyrrolo[2,3-b]pyridin-3-yl)methyl)amino)methyl)pyrrolidin-2-one (17)

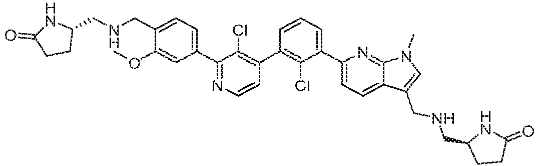

### (a) 4-(4-(3-Bromo-2-chlorophenyl)-3-chloropyridin-2-yl)-2-methoxybenzaldehyde

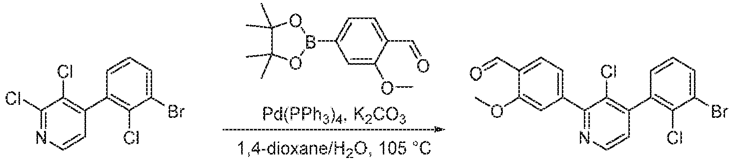

Pd(PPh₃)₄ (121 mg, 0.10 mmol), potassium carbonate (0.22 g, 1.56 mmol), 2-methoxy-4-(4,4,5,5-tetramethyl-1,3,2-dioxaborolan-2-yl)benzaldehyde (0.14 g, 0.52 mmol), and 4-(3-bromo-2-chloro-phenyl)-2,3-dichloro-pyridine (0.18 g, 0.52 mmol) were suspended in 1,4-dioxane/water (4:1, 4 mL), then the solution was heated at 105 °C for 20 minutes. Reaction was cooled to rt, diluted with 6 ml water, and extracted with EtOAc (3 x 4 mL). The combined organic extracts were concentrated under reduced pressure and crude sample was purified by flash chromatography (SiO₂, 0-60 % EtOAc/hexane) to afford 4-[4-(3-bromo-2-chloro-phenyl)-3-chloro-2-pyridyl]-2-methoxy-benzaldehyde (155 mg, 68 % yield) as a yellow foam. MS: *m*/*z* found 437, 439 [M+H]⁺.

### (b) 6-(2-Chloro-3-(3-chloro-2-(4-formyl-3-methoxyphenyl)pyridin-4-yl)phenyl)-1-methyl-1H-pyrrolo[2,3-b]pyridine-3-carbaldehyde

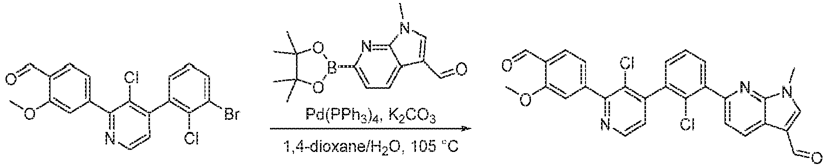

Pd(PPh₃)₄ (28 mg, 0.02 mmol), potassium carbonate (51 mg, 0.37 mmol), 4-[4-(3-bromo-2-chloro-phenyl)-3-chloro-2-pyridyl]-2-methoxy-benzaldehyde (53 mg, 0.12 mmol), and 1-methyl-6-(4,4,5,5-tetramethyl-1,3,2-dioxaborolan-2-yl)pyrrolo[2,3-b]pyridine-3-carbaldehyde (35 mg, 0.12 mmol) were suspended in 1,4-dioxane/water (4:1, 2 mL), then the solution was heated at 105 °C for 20 minutes. Reaction was cooled to rt, diluted with 3 mL water, and extracted with EtOAc (3 x 2 mL). The combined organics were concentrated under reduced pressure and crude sample was purified by flash chromatography (SiO₂, 0-10 % MeOH/methylene chloride) to afford 6-[2-chloro-3-[3-chloro-2-(4-formyl-3-methoxyphenyl)-4-pyridyl]phenyl]-1-methyl-pyrrolo[2,3-b]pyridine-3-carbaldehyde (25 mg, 40 % yield) as a yellow foam. MS: *m*/*z* found 516, 518 [M+H] ⁺.

### (c) (S)-5-((((6-(2-Chloro-3-(3-chloro-2-(3-methoxy-4-(((((S)-5-oxopyrrolidin-2-yl)-methyl)-amino)-methyl)-phenyl)-pyridin-4-yl)phenyl)-1-methyl-1H-pyrrolo[2,3-b]pyridin-3-yl)methyl)amino)methyl)pyrrolidin-2-one

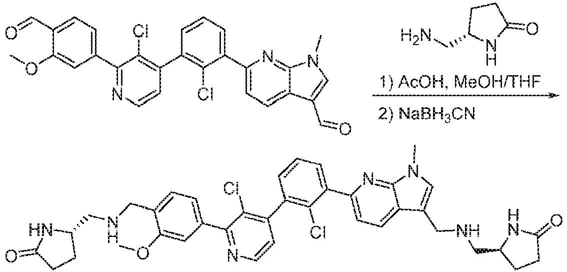

6-[2-chloro-3-[3-chloro-2-(4-formyl-3-methoxy-phenyl)-4-pyridyl]phenyl]-1-methyl-pyrrolo[2,3-b]pyridine-3-carbaldehyde (25 mg, 0.03 mmol), (5S)-5-(aminomethyl)pyrrolidin-2-one (10 mg, 0.09 mmol), and acetic acid (4 mg, 0.06 mmol) were dissolved in MeOH/THF (1:1, 1 mL), then the solution was stirred at 25 °C for 2 hours. Sodium cyanoborohydride (6 mg, 0.09 mmol) was added and the resulting mixture was stirred at 25 °C for 1 hour. Reaction was subsequently diluted with water (2 mL) and extracted with EtOAc (3 x 2 mL). The combined organics were further washed with brine, dried over sodium sulfate, filtered, and concentrated under reduced pressure. The residue was purified by reverse phase HPLC to afford (5S)-5-[[[4-[3-chloro-4-[2-chloro-3-[1-methyl-3-[[[(2S)-5-oxopyrrolidin-2-yl]methylamino]methyl]pyrrolo[2,3-b]pyridin-6-yl]phenyl]-2-pyridyl]-2-methoxyphenyl]methylamino]methyl]pyrrolidin-2-one (9 mg, 43 % yield) as a white solid (formic acid salt). LCMS: *m*/*z* found 712.35, 714 [M+H] ⁺, retention time = 2.05 min (Method C). ¹H NMR (400 MHz, Methanol-d₄) δ 8.65 (d, 1H), 8.48 (s, 1H), 8.23 (d, 1H), 7.71 (dd, 1H), 7.62 - 7.54 (m, 2H), 7.49 - 7.41 (m, 4H), 7.34 (d, 1H), 7.31 (dd, 1H), 4.35 - 4.23 (m, 2H), 4.17 - 4.06 (m, 2H), 3.96 (s, 3H), 3.91 (s, 5H), 3.07 - 2.88 (m, 4H), 2.41 - 2.29 (m, 6H), 1.91 - 1.78 (m, 2H).

### Example 129: (S)-5-((6-(2-chloro-3-(3-chloro-2-(3-methoxy-4-(((((S)-5-oxopyrrolidin-2-yl)methyl)amino)methyl)phenyl)pyridin-4-yl)phenyl)-8-methoxy-3,4-dihydroisoquinolin-2(1H)-yl)methyl)pyrrolidin-2-one (18)

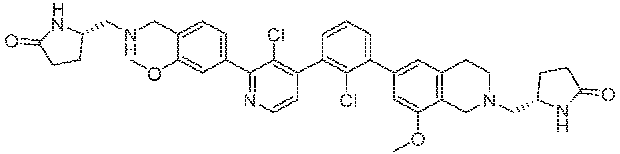

Compound was prepared in the same manner as described for compound 17 utilizing intermediate 4-(4-(3-bromo-2-chlorophenyl)-3-chloropyridin-2-yl)-2-methoxybenzaldehyde. (S)-5-((8-methoxy-6-(4,4,5,5-tetramethyl-1,3,2-dioxaborolan-2-yl)-3,4-dihydroisoquinolin-2(1H)-yl)methyl)pyrrolidin-2-one was used in place of 1-methyl-6-(4,4,5,5-tetramethyl-1,3,2-dioxaborolan-2-yl)pyrrolo[2,3-b]pyridine-3-carbaldehyde. Product was obtained as a yellow solid (formic acid salt). LCMS: *m*/*z* found 714.3, 716.3 [M+H]⁺, retention time = 2.10 min (Method C). ¹H NMR (400 MHz, Methanol-d₄) δ 8.65 - 8.59 (m, 1H), 8.52 (s, 1H), 7.49 (d, 2H), 7.42 (dd, 2H), 7.34 (dd, 1H), 7.32 - 7.23 (m, 2H), 6.80 (s, 2H), 4.07 - 3.96 (m, 3H), 3.94 (s, 3H), 3.88 (t, 1H), 3.83 (d, 3H), 3.74 (d, 1H), 3.60 (d, 1H), 2.94 (d, 2H), 2.91 - 2.65 (m, 5H), 2.61 (dd, 1H), 2.34 (qd, 6H), 1.81 (dd, 2H).

### Example 130: (S)-5-((((6-(2-Chloro-3-(3-chloro-2-(1-methyl-2,3,4,5-tetrahydro-1H-benzo[e][1,4]diazepin-8-yl)pyridin-4-yl)phenyl)-1-methyl-1H-pyrrolo[2,3-b]pyridin-3-yl)methyl)amino)methyl)pyrrolidin-2-one (9)

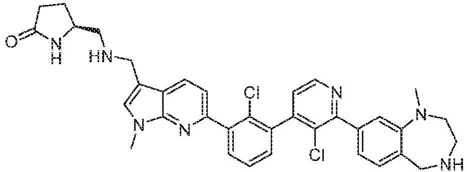

### (a) 4-(3-Bromo-2-chlorophenyl)-2,3-dichloropyridine

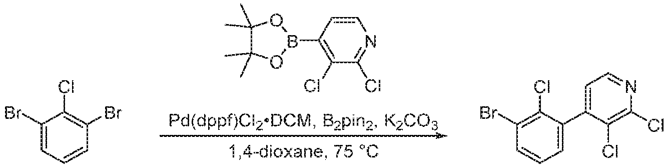

1,3-Dibromo-2-chloro-benzene (0.55 g, 2.04 mmol), 2,3-dichloro-4-(4,4,5,5-tetramethyl-1,3,2-dioxaborolan-2-yl)-pyridine (0.40 g, 1.46 mmol), 1,1'-bis(diphenylphosphino)-ferrocene-palladium(II) dichloride dichloromethane complex (0.12 g, 0.15 mmol), and potassium carbonate (0.56 g, 4.09 mmol) were suspended in 10 mL 1,4-dioxane in a sealed tube. Nitrogen gas was bubbled through the reaction solution for 2 minutes, then the vessel was sealed and heated at 75 °C for 12 h. The mixture was allowed to cool to room temperature and filtered through CELITE^{®}. The filtrate was concentrated under reduced pressure and the crude oil was purified by flash chromatography (SiO₂, 0-20 % EtOAc/hexane) to afford 4-(3-bromo-2-chloro-phenyl)-2,3-dichloro-pyridine (225 mg, 46 % yield) . MS: *m*/*z* found 337.8, 339.8 [M+H]⁺.

### (b) tert-Butyl 8-(4-(3-bromo-2-chlorophenyl)-3-chloropyridin-2-yl)-1-methyl-1,2,3,5-tetrahydro-4H-benzo[e][1,4]diazepine-4-carboxylate

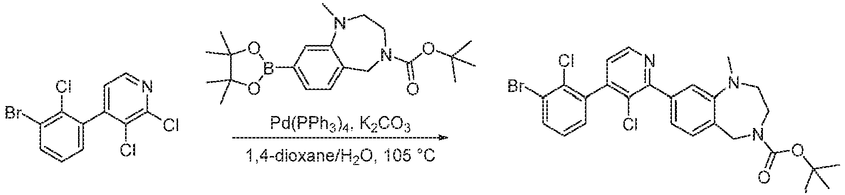

Pd(PPh₃)₄ (26 mg, 0.02 mmol), potassium carbonate (46 mg, 0.33 mmol), 4-(3-bromo-2-chloro-phenyl)-2,3-dichloro-pyridine (37 mg, 0.11 mmol), and tert-butyl 1-methyl-8-(4,4,5,5-tetramethyl-1,3,2-dioxaborolan-2-yl)-3,5-dihydro-2H-1,4-benzodiazepine-4-carboxylate (43 mg, 0.11 mmol) were suspended in 1,4-dioxane/water (4:1, 3 mL), then the solution was heated at 105 °C for 20 min. The mixture was allowed to cool to room temperature, diluted with 3 mL water, and extracted with EtOAc (3 x 2 mL). The combined organic extracts were concentrated under reduced pressure and crude sample was purified by flash chromatography (SiO₂, 5-40 % EtOAc/hexane) to afford *tert*-butyl 8-[4-(3-bromo-2-chloro-phenyl)-3-chloro-2-pyridyl]-1-methyl-3,5-dihydro-2H-1,4-benzodiazepine-4-carboxylate (18 mg, 29 % yield). MS: *m*/*z* found 564.3 [M+H] ⁺.

### (c) tert-Butyl 8-(3-chloro-4-(2-chloro-3-(3-formyl-1-methyl-1H-pyrrolo[2,3-b]pyridin-6-yl)phenyl)pyridin-2-yl)-1-methyl-1,2,3,5-tetrahydro-4H-benzo[e][1,4]diazepine-4-carboxylate

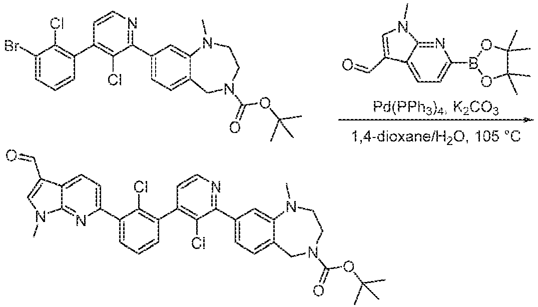

Pd(PPh₃)₄ (7 mg, 0.01 mmol), potassium carbonate (13 mg, 0.10 mmol), 1-methyl-6-(4,4,5,5-tetramethyl-1,3,2-dioxaborolan-2-yl)pyrrolo[2,3-b]pyridine-3-carbaldehyde (9 mg, 0.03 mmol), and *tert-*butyl 8-[4-(3-bromo-2-chloro-phenyl)-3-chloro-2-pyridyl]-1-methyl-3,5-dihydro-2H-1,4-benzodiazepine-4-carboxylate (18 mg, 0.03 mmol) were suspended in 1,4-dioxane/water (4:1, 1 mL), then the solution was heated at 105 °C for 20 min. The mixture was allowed to cool to room temperature, diluted with 3 mL water, and extracted with EtOAc (3 x 2 mL). The combined organic extracts were concentrated under reduced pressure and crude sample was purified by flash chromatography (SiO₂, 5-80 % EtOAc/hexane) to afford *tert-*butyl 8-[3-chloro-4-[2-chloro-3-(3-formyl-1-methyl-pyrrolo[2,3-b]pyridin-6-yl)phenyl]-2-pyridyl]-1-methyl-3,5-dihydro-2H-1,4-benzodiazepine-4-carboxylate (17 mg, 61 % yield). MS: *m*/*z* found 642, 644 [M+H] ⁺.

### (d) tert-Butyl (S)-8-(3-chloro-4-(2-chloro-3-(1-methyl-3-((((5-oxopyrrolidin-2-yl)methyl)amino)methyl)-1H-pyrrolo[2,3-b]pyridin-6-yl)phenyl)pyridin-2-yl)-1-methyl-1,2,3,5-tetrahydro-4H-benzo[e][1,4]diazepine-4-carboxylate

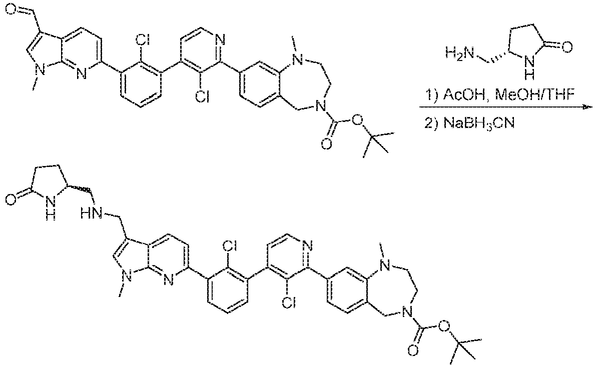

*tert-*Butyl 8-[3-chloro-4-[2-chloro-3-(3-formyl-1-methyl-pyrrolo[2,3-b]pyridin-6-yl)phenyl]-2-pyridyl]-1-methyl-3,5-dihydro-2H-1,4-benzodiazepine-4-carboxylate (17 mg, 0.03 mmol), (5S)-5-(aminomethyl)pyrrolidin-2-one (6 mg, 0.05 mmol), and acetic acid (3 mg, 0.05 mmol) were dissolved in MeOH/THF (1:1, 2 mL), then the solution was stirred at 25 °C for 2 hours. Sodium cyanoborohydride (5 mg, 0.08 mmol) was added and the resulting mixture was stirred at 25 °C for 1 hour. The mixture was subsequently diluted with water (2 mL) and extracted with methylene chloride (3 x2 mL). The combined organics were further washed with brine (3 mL), dried over sodium sulfate, filtered and concentrated under reduced pressure to afford *tert-*butyl 8-[3-chloro-4-[2-chloro-3-[1-methyl-3-[[[(2S)-5-oxopyrrolidin-2-yl]methylamino]methyl]pyrrolo[2,3-b]pyridin-6-yl]phenyl]-2-pyridyl]-1-methyl-3,5-dihydro-2H-1,4-benzodiazepine-4-carboxylate (19 mg, crude) as a yellow foam. MS: *m*/*z* found 740.4, 742 [M+H]⁺.

### (e) (S)-5-((((6-(2-Chloro-3-(3-chloro-2-(1-methyl-2,3,4,5-tetrahydro-1H-benzo[e][1,4]-diazepin-8-yl)-pyridin-4-yl)phenyl)-1-methyl-1H-pyrrolo[2,3-b]pyridin-3-yl)-methyl)-amino)-methyl)-pyrrolidin-2-one

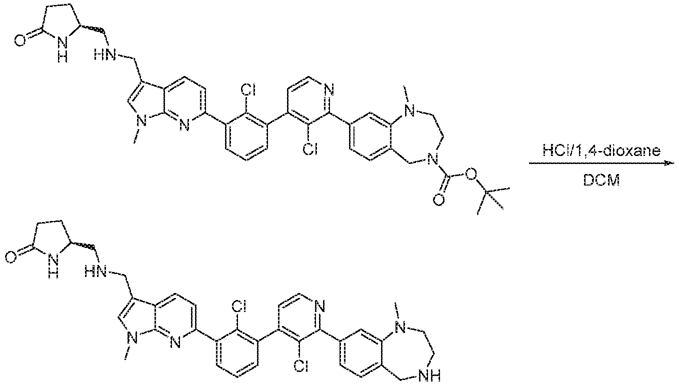

*tert-*Butyl 8-[3-chloro-4-[2-chloro-3-[1-methyl-3-[[[(2S)-5-oxopyrrolidin-2-yl]methylamino]-methyl]-pyrrolo[2,3-b]pyridin-6-yl]phenyl]-2-pyridyl]-1-methyl-3,5-dihydro-2H-1,4-benzodiazepine-4-carboxylate (19 mg, 0.03 mmol) was dissolved in 0.5 mL DCM and 4 M HCl solution in 1,4-dioxane (26 µL, 0.10 mmol) was added. The mixture was stirred at room temperature for 20 minutes and solvent was removed under reduced pressure. The residue was purified by reverse phase HPLC to afford (5S)-5-[[[6-[2-chloro-3-[3-chloro-2-(1-methyl-2,3,4,5-tetrahydro-1,4-benzodiazepin-8-yl)-4-pyridyl]phenyl]-1-methyl-pyrrolo[2,3-b]pyridin-3-yl]methylamino]methyl]pyrrolidin-2-one (8 mg, 48 % yield) (formic acid salt). LCMS: *m*/*z* found 640, 642 [M+H] ⁺, retention time = 2.17 min (Method C). ¹H NMR (400 MHz, Methanol-*d*₄) δ 8.65 (d, 1H), 8.49 (s, 2H), 8.22 (d, 1H), 7.71 (dd, 1H), 7.58 (t, 1H), 7.54 (s, 1H), 7.49 - 7.43 (m, 3H), 7.41 (d, 1H), 7.38 (d, 1H), 7.32 (dd, 1H), 4.33 (s, 2H), 4.22 (d, 2H), 3.91 (s, 4H), 3.40 - 3.35 (m, 2H), 3.03 (s, 3H), 2.94 (t, 2H), 2.38 - 2.27 (m, 3H), 1.89 - 1.78 (m, 1H).

### Example 131: (S)-5-((((6-(2-Chloro-3-(3-chloro-2-(4-((S)-2-hydroxypropyl)-1-methyl-2,3,4,5-tetrahydro-1H-benzo[e] [1,4]-diazepin-8-yl)-pyridin-4-yl)-phenyl)-2-methoxypyridin-3-yl)-methyl)-amino)-methyl)-pyrrolidin-2-one (24)

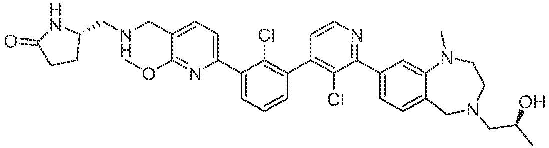

### (a) tert-Butyl 8-(4-(3-bromo-2-chlorophenyl)-3-chloropyridin-2-yl)-1-methyl-1,2,3,5-tetrahydro-4H-benzo[e][1,4]diazepine-4-carboxylate

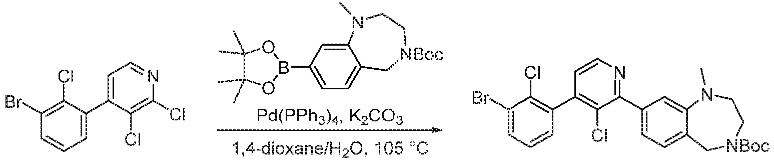

Pd(PPh₃)₄ (45 mg, 0.04 mmol), potassium carbonate (80 mg, 0.58 mmol), *tert*-butyl 1-methyl-8-(4,4,5,5-tetramethyl-1,3,2-dioxaborolan-2-yl)-3,5-dihydro-2H-1,4-benzodiazepine-4-carboxylate (75 mg, 0.19 mmol), and 4-(3-bromo-2-chloro-phenyl)-2,3-dichloro-pyridine (65 mg, 0.19 mmol) were suspended in 1,4-dioxane/water (4:1, 2 mL), then the solution was heated at 105 °C for 20 minutes. The mixture was allowed to cool to room temperature, diluted with 3 mL of water, and extracted with EtOAc (3 x 3 mL). The combined organic extracts were concentrated under reduced pressure and crude sample was purified by flash chromatography (SiO₂, 0-40 % EtOAc/hexane) to afford *tert-*butyl 8-[4-(3-bromo-2-chlorophenyl)-3-chloro-2-pyridyl]-1-methyl-3,5-dihydro-2H-1,4-benzodiazepine-4-carboxylate (75 mg, 69 % yield). MS: *m*/*z* found 564.1 [M+H] ⁺.

### (b) 8-(4-(3-Bromo-2-chlorophenyl)-3-chloropyridin-2-yl)-1-methyl-2,3,4,5-tetrahydro-1H-benzo-[e][1,4]diazepine

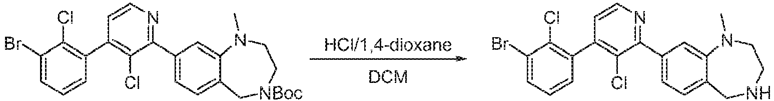

*tert-*Butyl 8-[4-(3-bromo-2-chloro-phenyl)-3-chloro-2-pyridyl]-1-methyl-3,5-dihydro-2H-1,4-benzodiazepine-4-carboxylate (75 mg, 0.13 mmol) was dissolved in 2 mL methylene chloride and a 4 M HCl solution in 1,4-dioxane (133 µL, 0.53 mmol) solution was added. The mixture was stirred at room temperature for 3 hours, then the precipitate was filtered and washed with methylene chloride (5 mL). The solid was further dried under vacuum to afford 8-[4-(3-bromo-2-chloro-phenyl)-3-chloro-2-pyridyl]-1-methyl-2,3,4,5-tetrahydro-1,4-benzodiazepine (51 mg, 82 % yield) (HCl salt). MS: *m*/*z* found 464 [M+H] ⁺.

### (c) (S)-1-(8-(4-(3-Bromo-2-chlorophenyl)-3-chloropyridin-2-yl)-1-methyl-1,2,3,5-tetrahydro-4H-benzo[e][1,4]diazepin-4-yl)propan-2-ol

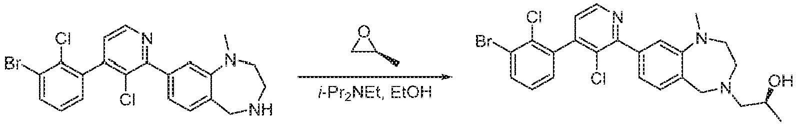

8-[4-(3-Bromo-2-chloro-phenyl)-3-chloro-2-pyridyl]-1-methyl-2,3,4,5-tetrahydro-1,4-benzodiazepine hydrochloride (25 mg, 0.05 mmol), (2S)-2-methyloxirane (29 mg, 0.50 mmol), and DIEA (26 µL, 0.15 mmol) were suspended in 1 mL of EtOH and the mixture was stirred at 85 °C for 1 hour. Reaction was concentrated and the residue was dissolved in 2 mL EtOAc. The organic solution was washed with water (2 x 2 mL), brine (2 x 2 mL), dried over sodium sulfate, filtered and concentrated to afford (2S)-1-[8-[4-(3-bromo-2-chloro-phenyl)-3-chloro-2-pyridyl]-1-methyl-3,5-dihydro-2H-1,4-benzodiazepin-4-yl]propan-2-ol (20 mg, crude) as a light yellow oil. MS: *m*/*z* found 522 [M+H] ⁺. Material was used for the next step without further purification.

### (d) tert-Butyl (S)-((6-chloro-2-methoxypyridin-3-yl)methyl)((5-oxopyrrolidin-2-yl)methyl)carbamate

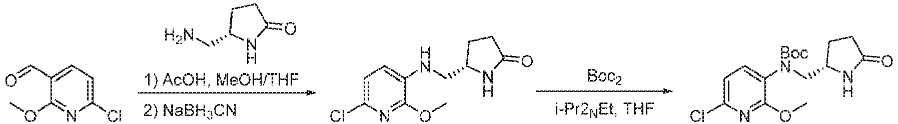

6-Chloro-2-methoxy-pyridine-3-carbaldehyde (400 mg, 2.33 mmol), (5S)-5-(aminomethyl)pyrrolidin-2-one (0.35 g, 3.03 mmol), and acetic acid (0.17 g, 2.80 mmol) were dissolved in MeOH/THF (1:1, 10 mL), then the solution was stirred at 25 °C for 2 hours. Sodium cyanoborohydride (0.22 g, 3.50 mmol) was added and the resulting mixture was stirred at 25 °C for 1 hour. Reaction was subsequently diluted with water (20 mL) and extracted with methylene chloride (3 x 10 mL). The combined organics were further washed with brine (20 mL), dried over sodium sulfate, filtered and concentrated under reduced pressure to afford (5S)-5-[[(6-chloro-2-methoxy-3-pyridyl)methylamino]methyl]pyrrolidin-2-one (0.63 g, crude) as a yellow oil. MS: *m*/*z* found 270 [M+H]⁺.

(5S)-5-[[(6-chloro-2-methoxy-3-pyridyl)methylamino]methyl]pyrrolidin-2-one (628 mg, 2.33 mmol, crude), DIEA (1619 µL, 9.31 mmol), and di-*tert*-butyl dicarbonate (1016 mg, 4.66 mmol) were dissolved in 10 mL THF, then the mixture was stirred at rt for 12 hours. Reaction was diluted with EtOAc (15 mL) and the resulting solution was washed with saturated aqueous NaHCO₃ (10 mL × 2), followed by brine (10 mL × 2). The organic layer was concentrated under reduced pressure and crude sample was purified by flash chromatography (SiO₂, 0-5 % MeOH/DCM) to afford *tert-*butyl N-[(6-chloro-2-methoxy-3-pyridyl)methyl]-N-[[(2S)-5-oxopyrrolidin-2-yl]methyl]carbamate (788 mg, 92 % yield). MS: *m*/*z* found 370 [M+H] ⁺.

### (e) tert-Butyl (S)-((2-methoxy-6-(4,4,5,5-tetramethyl-1,3,2-dioxaborolan-2-yl)pyridin-3-yl)methyl)((5-oxopyrrolidin-2-yl)methyl)carbamate

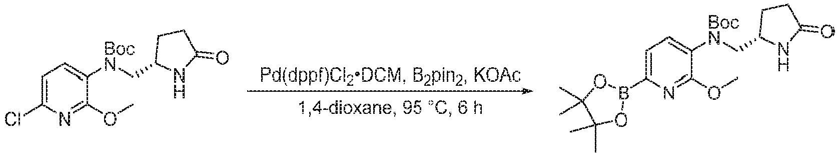

*tert-*Butyl N-[(6-chloro-2-methoxy-3-pyridyl)methyl]-N-[[(2S)-5-oxopyrrolidin-2-yl]methyl]carbamate (0.35 g, 0.94 mmol), 4,4,5,5-tetramethyl-2-(4,4,5,5-tetramethyl-1,3,2-dioxaborolan-2-yl)-1,3,2-dioxaborolane (0.34 g, 1.32 mmol), 1,1'-bis(diphenylphosphino)-ferrocene-palladium(II) dichloride dichloromethane complex (77 mg, 0.09 mmol), and potassium acetate (0.28 g, 2.82 mmol) were suspended in 10 mL 1,4-dioxane in a sealed tube. Nitrogen gas was bubbled through the reaction solution for 2 minutes, then the vessel was sealed and heated at 95 °C for 6 h. Reaction was cooled to rt and the mixture was filtered through celite. The filtrate was concentrated under reduced pressure and the crude oil was dissolved in 3 mL EtOAc. Hexane was added drop-wise with vigorous stirring until the dark solid precipitated out of solution. The solid was filtered and the filtrate was concentrated under reduced pressure to afford *tert-*butyl N-[[2-methoxy-6-(4,4,5,5-tetramethyl-1,3,2-dioxaborolan-2-yl)-3-pyridyl]methyl]-N-[[(2S)-5-oxopyrrolidin-2-yl]methyl]carbamate (434 mg, crude). The material was used for the next step without further purification.

### (f) tert-Butyl ((6-(2-chloro-3-(3-chloro-2-(4-((S)-2-hydroxypropyl)-1-methyl-2,3,4,5-tetrahydro-1H-benzo[e][1,4]diazepin-8-yl)pyridin-4-yl)phenyl)-2-methoxypyridin-3-yl)methyl)(((S)-5-oxopyrrolidin-2-yl)methyl)carbamate

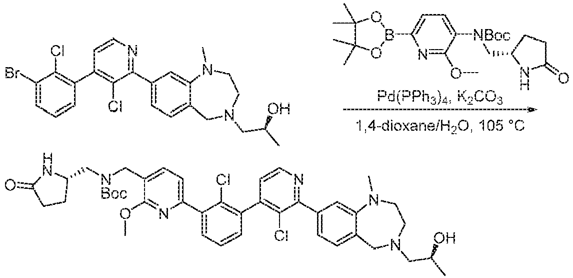

Pd(PPh₃)₄ (9 mg, 0.01 mmol), potassium carbonate (16 mg, 0.11 mmol), (2S)-1-[8-[4-(3-bromo-2-chloro-phenyl)-3-chloro-2-pyridyl]-1-methyl-3,5-dihydro-2H-1,4-benzodiazepin-4-yl]propan-2-ol (20 mg, 0.04 mmol), and tert-butyl N-[[2-methoxy-6-(4,4,5,5-tetramethyl-1,3,2-dioxaborolan-2-yl)-3-pyridyl]methyl]-N-[[(2S)-5-oxopyrrolidin-2-yl]methyl]carbamate (26 mg, 0.06 mmol) were suspended in 1,4-dioxane/water (4:1 (*v*/*v*), 1 mL), then the solution was heated at 105 °C for 20 minutes. The mixture was allowed to cool to room temperature, diluted with 3 mL of water, and extracted with EtOAc (3 x 2 mL). The combined organics were concentrated under reduced pressure and crude sample was purified by flash chromatography (SiO₂, 0-8 % MeOH/DCM) to afford *tert-*butyl N-[[6-[2-chloro-3-[3-chloro-2-[4-[(2S)-2-hydroxypropyl]-1-methyl-3,5-dihydro-2H-1,4-benzodiazepin-8-yl]-4-pyridyl]phenyl]-2-methoxy-3-pyridyl]methyl]-N-[[(2S)-5-oxopyrrolidin-2-yl]methyl]carbamate (13 mg, 45 % yield). MS: *m*/*z* found 775.4, 777 [M+H] ⁺.

### (g) (S)-5-((((6-(2-Chloro-3-(3-chloro-2-(4-((S)-2-hydroxypropyl)-1-methyl-2,3,4,5-tetrahydro-1H-benzo[e][1,4]-diazepin-8-yl)-pyridin-4-yl)-phenyl)-2-methoxypyridin-3-yl)-methyl)-amino)-methyl)-pyrrolidin-2-one

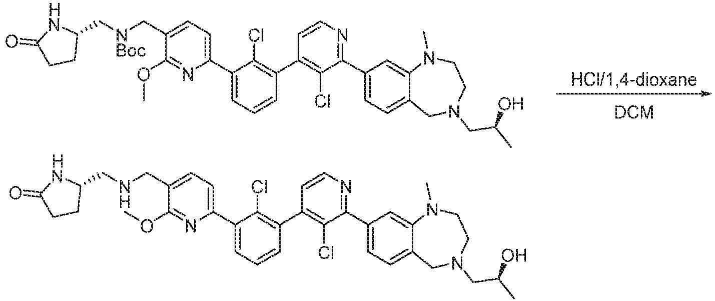

*tert-*Butyl N-[[6-[2-chloro-3-[3-chloro-2-[4-[(2S)-2-hydroxypropyl]-1-methyl-3,5-dihydro-2H-1,4-benzodiazepin-8-yl]-4-pyridyl]phenyl]-2-methoxy-3-pyridyl]methyl]-N-[[(2S)-5-oxopyrrolidin-2-yl]methyl]carbamate (13 mg, 0.02 mmol) was dissolved in 0.5 mL of methylene chloride and a 4 M HCl solution in 1,4-dioxane (17 µL, 0.07 mmol) was added. The mixture was stirred at room temperature for 20 minutes and solvent was removed under reduced pressure. The residue was purified by reverse phase HPLC to afford (5S)-5-[[[6-[2-chloro-3-[3-chloro-2-[4-[(2S)-2-hydroxypropyl]-1-methyl-3,5-dihydro-2H-1,4-benzodiazepin-8-yl]-4-pyridyl]phenyl]-2-methoxy-3-pyridyl]methylamino]methyl]pyrrolidin-2-one (2 mg, 17 % yield) (formic acid salt). LCMS: *m*/*z* found 675.3, 677 [M+H]⁺, retention time = 2.07 min. (Method C). ¹H NMR (400 MHz, Methanol-*d*₄) δ 8.63 (d, 1H), 8.49 (s, 3H), 7.77 (d, 1H), 7.71 (dd, 1H), 7.55 (t, 1H), 7.46 - 7.38 (m, 3H), 7.31 (s, 1H), 7.28 (t, 2H), 4.33 (q, 2H), 4.15 (s, 1H), 4.03 (s, 3H), 3.90 (dd, 3H), 3.41 - 3.31 (m, 4H), 2.99 (s, 4H), 2.89 - 2.71 (m, 3H), 2.40 - 2.19 (m, 3H), 1.90 - 1.70 (m, 1H), 1.21 (d, 3H).

### Example 132: (5S,5'S)-5,5'-(((((3,3'-Dichloro-[4,4'-bipyridine]-2,2'-diyl)bis(2-methoxy-4,1-phenylene))-bis(methylene))-bis(azanediyl))bis(methylene))bis(pyrrolidin-2-one) (4)

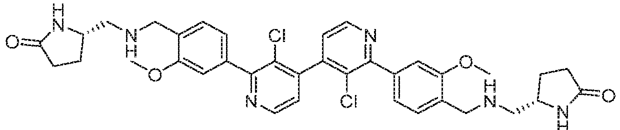

### (a) 2,2',3,3'-Tetrachloro-4,4'-bipyridine

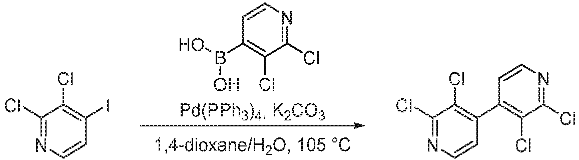

Pd(PPh₃)₄ (0.24 g, 0.21 mmol), potassium carbonate (0.43 g, 3.13 mmol), (2,3-dichloro-4-pyridyl)boronic acid (0.20 g, 1.04 mmol), and 2,3-dichloro-4-iodo-pyridine (0.37 g, 1.36 mmol) were suspended in 1,4-dioxane/water (4:1, 10 mL), then the solution was heated at 105 °C for 20 minutes. The mixture was allowed to cool to room temperature, diluted with 10 mL of water, and extracted with EtOAc (3 x 5 mL). The combined organics were concentrated under reduced pressure and crude sample was purified by normal phase SiO₂ chromatography (5-60 % EtOAc/hexane) to afford 2,3-dichloro-4-(2,3-dichloro-4-pyridyl)pyridine (96 mg, 44 % yield) as a tan solid.. MS: *m*/*z* found 295, 297 [M+H] ⁺.

### (b) 4,4'-(3,3'-Dichloro-[4,4'-bipyridine]-2,2'-diyl)bis(2-methoxybenzaldehyde)

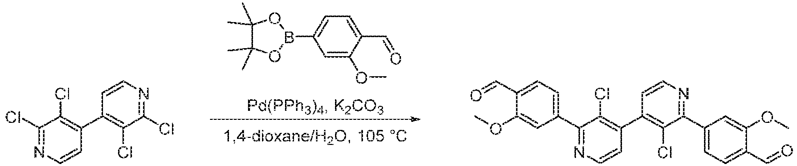

Pd(PPh₃)₄ (35 mg, 0.03 mmol), potassium carbonate (63 mg, 0.46 mmol), 2,3-dichloro-4-(2,3-dichloro-4-pyridyl)pyridine (45 mg, 0.15 mmol), and 2-methoxy-4-(4,4,5,5-tetramethyl-1,3,2-dioxaborolan-2-yl)benzaldehyde (100 mg, 0.38 mmol) were suspended in 1,4-dioxane/water (4:1, 2 mL), then the solution was heated at 105 °C for 20 minutes. The mixture was allowed to cool to room temperature, diluted with 3 mL water, and extracted with EtOAc (3 x 2 mL). The combined organics were concentrated, and crude sample was passed through a short SiO₂ column (5-90 % EtOAc/hexane) to remove some impurities. The clean fractions were combined and concentrated under reduced pressure to afford 4-[3-chloro-4-[3-chloro-2-(4-formyl-3-methoxy-phenyl)-4-pyridyl]-2-pyridyl]-2-methoxybenzaldehyde (75 mg, crude) as a yellow foam. MS: *m*/*z* found 493, 495 [M+H]⁺.

### (c) (5S,5'S)-5,5'-(((((3,3'-Dichloro-[4,4'-bipyridine]-2,2'-diyl)bis(2-methoxy-4,1-phenylene))-bis(methylene))-bis(azanediyl))bis(methylene))bis(pyrrolidin-2-one)

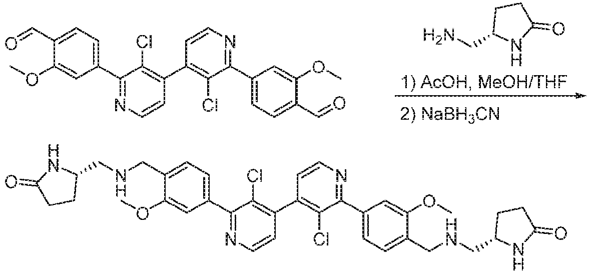

4-[3-Chloro-4-[3-chloro-2-(4-formyl-3-methoxy-phenyl)-4-pyridyl]-2-pyridyl]-2-methoxy-benzaldehyde (75 mg, 0.09 mmol, crude), (5S)-5-(aminomethyl)pyrrolidin-2-one (31 mg, 0.27 mmol), and acetic acid, (11 mg, 0.18 mmol) were dissolved in MeOH/THF (1:1, 2 mL), then the solution was stirred at 25 °C for 2 hours. Sodium cyanoborohydride (17 mg, 0.27 mmol) was added and the resulting mixture was stirred at 25 °C for 1 hour. Reaction was subsequently diluted with water (3 mL) and extracted with EtOAc (3 x 2 mL). The combined organics were further washed with brine, dried over sodium sulfate, filtered, and concentrated under reduced pressure. The residue was purified by reverse phase HPLC to afford (5S)-5-[[[4-[3-chloro-4-[3-chloro-2-[3-methoxy-4-[[[(2S)-5-oxopyrrolidin-2-yl]methylamino]methyl]phenyl]-4-pyridyl]-2-pyridyl]-2-methoxyphenyl]methylamino]methyl]pyrrolidin-2-one (6 mg, 10 % yield) as a white solid (formic acid salt). LCMS: *m*/*z* found 689.4, 691 [M+H] ⁺, retention time = 1.94 min (Method C). ¹H NMR (400 MHz, Methanol-*d*₄) δ 8.70 (d, 2H), 8.49 (s, 2H), 7.54 - 7.42 (m, 4H), 7.38 - 7.22 (m, 4H), 4.12 - 4.02 (m, 4H), 3.96 (s, 6H), 3.92 (q, 2H), 2.89 (h, 4H), 2.41 - 2.27 (m, 6H), 1.91 - 1.76 (m, 2H).

### Example 133: (5S,5'S)-5,5'-(((((3,3'-dichloro-[4,4'-bipyridine]-2,2'-diyl)bis(2-fluoro-6-methoxy-4,1-phenylene))-bis(methylene))bis(azanediyl))bis(methylene))bis(pyrrolidin-2-one) (12)

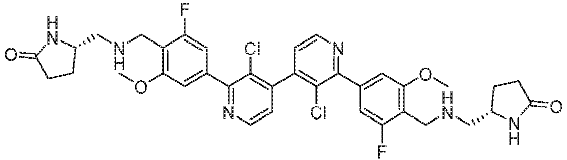

(5 S,5'S)-5,5'-(((((3,3'-dichloro-[4,4'-bipyridine]-2,2'-diyl)bis(2-fluoro-6-methoxy-4,1-phenylene))-bis(methylene))bis(azanediyl))bis(methylene))bis(pyrrolidin-2-one) was prepared in a similar manner as described for compound **4** utilizing intermediate 2,2',3,3'-tetrachloro-4,4'-bipyridine. 2-fluoro-6-methoxy-4-(4,4,5,5-tetramethyl-1,3,2-dioxaborolan-2-yl)benzaldehyde was used in place of 2-methoxy-4-(4,4,5,5-tetramethyl-1,3,2-dioxaborolan-2-yl)benzaldehyde. Product was obtained as a white solid (formic acid salt). LCMS: *m*/*z* found 725.3, 727.2 [M+H]⁺, retention time = 1.90 min. (Method C). ¹H NMR (400 MHz, Methanol-*d*₄) δ 8.72 (d, 2H), 8.41 (s, 1H), 7.51 (d, 2H), 7.21 (s, 2H), 7.15 (dd, 2H), 4.11 (s, 4H), 3.98 (s, 6H), 3.91 (s, 2H), 2.95 - 2.82 (m, 4H), 2.41 - 2.26 (m, 6H), 1.89 - 1.76 (m, 2H).

### Example 134: (S)-5-(((4-(3,3'-dichloro-2'-(1-methyl-3-(((((S)-5-oxopyrrolidin-2-yl)methyl)amino)methyl)-1H-indol-6-yl)-[4,4'-bipyridin]-2-yl)-2-methoxybenzyl)amino)methyl)pyrrolidin-2-one (14)

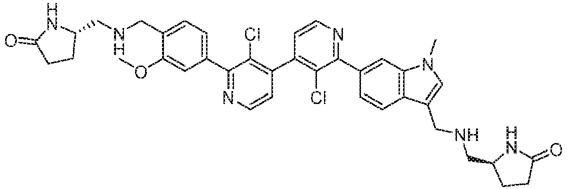

### (a) 6-(3,3'-Dichloro-2'-(4-formyl-3-methoxyphenyl)-[4,4'-bipyridin]-2-yl)-1-methyl-1H-indole-3-carbaldehyde

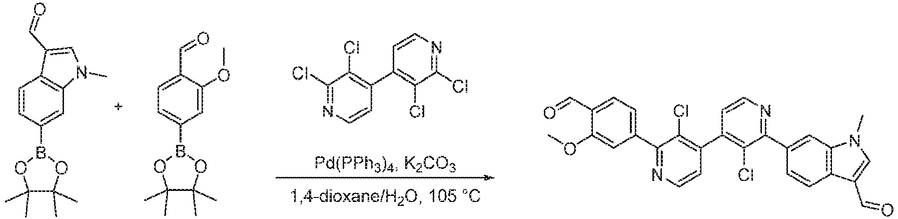

Pd(PPh₃)₄ (24 mg, 0.02 mmol), potassium carbonate (42 mg, 0.31 mmol), 2,3-dichloro-4-(2,3-dichloro-4-pyridyl)pyridine (30 mg, 0.10 mmol), 2-methoxy-4-(4,4,5,5-tetramethyl-1,3,2-dioxaborolan-2-yl)benzaldehyde (29 mg, 0.11 mmol), and 1-methyl-6-(4,4,5,5-tetramethyl-1,3,2-dioxaborolan-2-yl)indole-3-carbaldehyde (32 mg, 0.11 mmol) were suspended in 1,4-dioxane/water (4:1, 2 mL), then the solution was heated at 105 °C for 20 minutes. The mixture was allowed to cool to room temperature, diluted with 3 mL of water, and extracted with EtOAc (3 x 2 mL). The combined organics were concentrated under reduced pressure and crude sample was purified by flash chromatography (SiO₂, 0-50 % EtOAc/hexane) to afford desired product 6-[3-chloro-4-[3-chloro-2-(4-formyl-3-methoxyphenyl)-4-pyridyl]-2-pyridyl]-1-methyl-indole-3-carbaldehyde (27 mg, 51 % yield) as a grey solid. MS: *m*/*z* found 516.1, 518.1 [M+H] ⁺. Homo-dimer products were also isolated: 6-[3-chloro-4-[3-chloro-2-(3-formyl-1-methyl-indol-6-yl)-4-pyridyl]-2-pyridyl]-1-methyl-indole-3-carbaldehyde (8.60 mg, 15.6% yield, *m*/*z* found 540 [M+H]⁺) as a tan solid, 4-[3-chloro-4-[3-chloro-2-(4-formyl-3-methoxy-phenyl)-4-pyridyl]-2-pyridyl]-2-methoxy-benzaldehyde (6.00 mg, 11.9% yield, *m*/*z* found 494 [M+H]⁺) as a white solid.

### (b) (S)-5-(((4-(3,3'-Dichloro-2'-(1-methyl-3-(((((S)-5-oxopyrrolidin-2-yl)methyl)amino)methyl)-1H-indol-6-yl)-[4,4'-bipyridin]-2-yl)-2-methoxybenzyl)amino)methyl)pyrrolidin-2-one

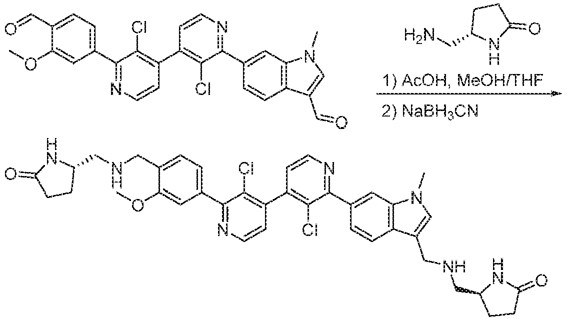

6-[3-Chloro-4-[3-chloro-2-(4-formyl-3-methoxy-phenyl)-4-pyridyl]-2-pyridyl]-1-methyl-indole-3-carbaldehyde (27 mg, 0.05 mmol), (5S)-5-(aminomethyl)pyrrolidin-2-one (18 mg, 0.16 mmol), and acetic acid (6 mg, 0.10 mmol) were dissolved in MeOH/THF (1:1, 1 mL), then the solution was stirred at 25 °C for 2 hours. Sodium cyanoborohydride (9.86 mg, 0.16 mmol) was added and the resulting mixture was stirred at 25 °C for 1 hour. The mixture was subsequently diluted with water (2 mL) and extracted with EtOAc (3 x 2 mL). The combined organics were further washed with brine, dried over sodium sulfate, filtered, and concentrated under reduced pressure. Crude sample was purified by reverse phase HPLC to afford (5S)-5-[[[4-[3-chloro-4-[3-chloro-2-[1-methyl-3-[[[(2S)-5-oxopyrrolidin-2-yl]-methylamino]-methyl]-indol-6-yl]-4-pyridyl]-2-pyridyl]-2-methoxyphenyl]methylamino]methyl]pyrrolidin-2-one (14 mg, 37 % yield) as a white solid (formic acid salt). LCMS: *m*/*z* found 712.35, 714 [M+H] ⁺, retention time = 1.95 min (Method C). ¹H NMR (400 MHz, Methanol-*d*₄) δ 8.70 (dd, 2H), 8.48 (s, 2H), 7.84 (d, 1H), 7.79 - 7.75 (m, 1H), 7.51 (s, 2H), 7.49 (d, 1H), 7.47 (d, 1H), 7.46 (s, 1H), 7.35 (s, 1H), 7.32 (d, 1H), 4.38 (d, 2H), 4.11 - 4.01 (m, 2H), 3.96 (s, 5H), 3.90 (s, 3H), 3.09 (t, 2H), 2.89 (t, 2H), 2.42 - 2.25 (m, 6H), 1.92 - 1.78 (m, 2H).

### Example 135: (5S,5'S)-5,5'-(((((3,3'-Dichloro-[4,4'-bipyridine]-2,2'-diyl)bis(1-methyl-1H-indole-6,3-diyl))bis(methylene))bis(azanediyl))bis(methylene))bis(pyrrolidin-2-one) (15)

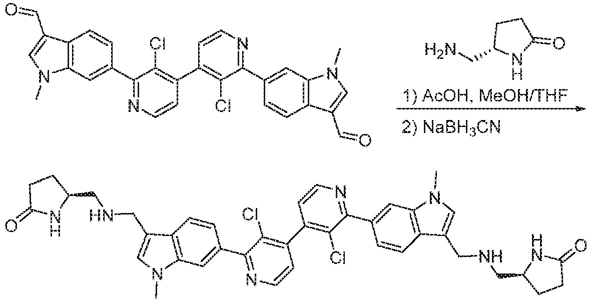

6-[3-Chloro-4-[3-chloro-2-(3-formyl-1-methyl-indol-6-yl)-4-pyridyl]-2-pyridyl]-1-methyl-indole-3-carbaldehyde (9 mg, 0.02 mmol), (5S)-5-(aminomethyl)pyrrolidin-2-one (5 mg, 0.05 mmol), and acetic acid (2 mg, 0.03 mmol) were dissolved in MeOH/THF (1:1, 1 mL), then the solution was stirred at 25 °C for 2 hours. Sodium cyanoborohydride (3 mg, 0.05 mmol) was added and the resulting mixture was stirred at 25 °C for 1 hour. The mixture was subsequently diluted with water (2 mL) and extracted with EtOAc (3 x 2 mL). The combined organics were further washed with brine, dried over sodium sulfate, filtered, and concentrated under reduced pressure. Crude sample was purified by reverse phase HPLC to afford (5S)-5-[[[6-[3-chloro-4-[3-chloro-2-[1-methyl-3-[[[(2S)-5-oxopyrrolidin-2-yl]methylamino]methyl]indol-6-yl]-4-pyridyl]-2-pyridyl]-1-methyl-indol-3-yl]methylamino]methyl]pyrrolidin-2-one (7 mg, 50 % yield) as a white solid (formic acid salt). LCMS: *m*/*z* found 736.6 [M+H] ⁺, retention time = 2.01 min. (Method C ¹H NMR (400 MHz, Methanol-*d*₄) δ 8.68 (dd, 2H), 7.79 (d, 2H), 7.74 (s, 2H), 7.50 - 7.43 (m, 4H), 7.37 (s, 2H), 4.22 - 4.04 (m, 4H), 3.93 - 3.82 (m, 8H), 2.88 (q, 4H), 2.38 - 2.21 (m, 6H), 1.88 - 1.74 (m, 2H).

### Example 136: (S)-5-(((4-(3,3'-Dichloro-2'-(1-methyl-3-(((((S)-5-oxopyrrolidin-2-yl)methyl)amino)methyl)-1H-pyrrolo[3,2-b]pyridin-6-yl)-[4,4'-bipyridin]-2-yl)-2-methoxybenzyl)amino)methyl)pyrrolidin-2-one (16)

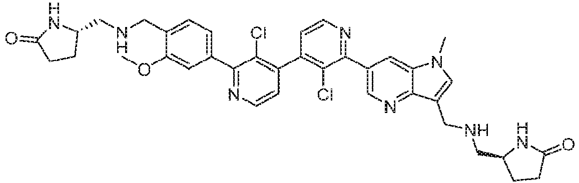

Compound was prepared in the same manner as described for compound **14** utilizing intermediate 2,2',3,3'-tetrachloro-4,4'-bipyridine. Product was obtained as a white solid (formic acid salt). LCMS: *m*/*z* found 713.35, 715.3 [M+H]⁺, retention time = 1.89 min. (Method C). ¹H NMR (400 MHz, Methanol-*d*₄) δ 8.77 (d, 2H), 8.71 (d, 1H), 8.50 (s, 1H), 8.31 (d, 1H), 7.79 (s, 1H), 7.52 (dd, 2H), 7.46 (d, 1H), 7.36 - 7.28 (m, 2H), 4.44 - 4.27 (m, 2H), 4.10 - 4.02 (m, 2H), 3.95 (d, 8H), 3.06 (qd, 2H), 2.87 (h, 2H), 2.42 - 2.28 (m, 6H), 1.92 - 1.77 (m, 2H).

### Example 137: (S)-5-((((3'-Chloro-2'-((S)-1-((5-((((S)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)methyl)pyrrolidin-2-one (128)

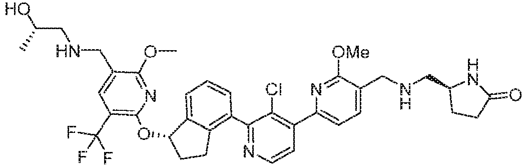

### (a) (S)-6-((4-Bromo-2,3-dihydro-1H-inden-1-yl)oxy)-2-methoxynicotinonitrile

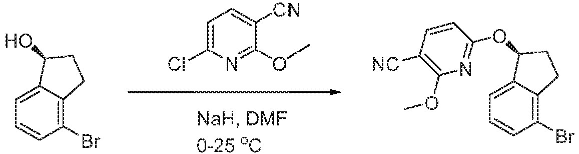

Sodium hydride (60 % in oil dispersion) (0.15 g, 3.86 mmol) was suspended in 6 mL anhydrous DMF and the solution was cooled to 0 °C. A solution of (1S)-4-bromoindan-1-ol (0.63 g, 2.97 mmol) in 2 mL DMF was slowly added over 2 minutes. The resulting mixture allowed to warm to room temperature and stirred to 30 minutes, then cooled back to 0 °C. 6-Chloro-2-methoxy-pyridine-3-carbonitrile (500 mg, 2.97 mmol) in 3 ml DMF was added drop wise, then the reaction mixture was warmed to 25 °C and stirred for 3 h. The mixture was then diluted with 20 mL of water and extracted with EtOAc (3 x 20 mL). The combined organic extracts were washed with water (30 mL), brine (30 mL), dried with sodium sulfate, filtered, and concentrated under reduced pressure. The residue was purified by flash chromatography (SiO₂, 0-10 % EtOAc/hexane) to afford 6-[(1S)-4-bromoindan-1-yl]oxy-2-methoxy-pyridine-3-carbonitrile (735 mg, 72 % yield) . MS: *m*/*z* found 345, 347 [M+H] ⁺.

### (b) (S)-6-((4-Bromo-2,3-dihydro-1H-inden-1-yl)oxy)-5-iodo-2-methoxynicotinonitrile

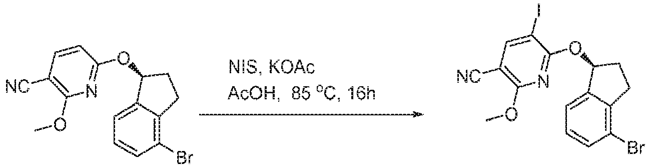

6-[(1S)-4-Bromoindan-1-yl|oxy-2-methoxy-pyridine-3-carbonitrile (0.30 g, 0.87 mmol), 1-iodopyrrolidine-2,5-dione (0.22 g, 0.96 mmol), and potassium acetate (0.85 g, 8.69 mmol) were suspended in 6 mL of acetic acid and the mixture was stirred at 85 °C for 16 h. The mixture was then diluted with 20 mL of EtOAc and 20 mL of water. The solution was cooled to 0 °C and a 2 M NaOH solution was slowly added until the solution reached ~pH 9. The mixture was extracted with EtOAc (3 x 20 mL) and the combined organics were washed with brine, dried over sodium sulfate, filtered, and concentrated under reduced pressure. The residue was purified by flash chromatography (SiO₂, 0-10 % EtOAc/hexane) to afford 6-[(1S)-4-bromoindan-1-yl]oxy-5-iodo-2-methoxy-pyridine-3-carbonitrile (218 mg, 53 % yield). MS: *m*/*z* found 472 [M+H]⁺. ¹H NMR (400 MHz, Chloroform-*d*) δ 8.10 (d, 1H), 7.49 (d, 1H), 7.38 (d, 1H), 7.14 (t, 1H), 6.56 - 6.47 (m, 1H), 4.07 (d, 3H), 3.22 - 3.13 (m, 1H), 2.96 (m, 1H), 2.72 (m, 1H), 2.32 - 2.18 (m, 1H).

### (c) (S)-6-((4-Bromo-2,3-dihydro-1H-inden-1-yl)oxy)-2-methoxy-5-(trifluoromethyl)-nicotinonitrile

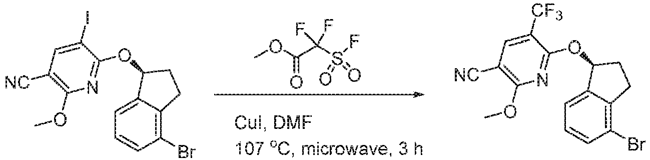

In a microwave vial, 6-[(1S)-4-bromoindan-1-yl]oxy-5-iodo-2-methoxy-pyridine-3-carbonitrile (150 mg, 0.32 mmol) and copper iodide (90 mg, 0.47 mmol) were suspended in 4 ml of DMF. Methyl 2,2-difluoro-2-fluorosulfonyl-acetate (122 mg, 0.63 mmol) was added, and the mixture was purged with nitrogen gas for 5 minutes. The mixture was then subjected to microwave irradiation, maintaining a reaction temperature of 107 °C for 3 h. The mixture was cooled to room temperature, diluted with 5 mL of EtOAc, and the insoluble copper salts were removed by filtration. The filtrate was washed with water (3 x 10 mL) and the organic solution was concentrated under reduced pressure. The residue was purified by flash chromatography (SiO₂, 0-10 % EtOAc/hexane) to afford 6-[(1S)-4-bromoindan-1-yl]oxy-2-methoxy-5-(trifluoromethyl)pyridine-3-carbonitrile (84 mg, 64 % yield). ¹H NMR (400 MHz, Chloroform-d) δ 8.03 (s, 1H), 7.50 (d, 1H), 7.34 (d, 1H), 7.13 (t, 1H), 6.63 (dd, 1H), 4.14 (s, 3H), 3.18 (m, 1H), 3.04 - 2.92 (m, 1H), 2.81 - 2.66 (m, 1H), 2.26 (m, 1H).

### (d) (S)-6-((4-Bromo-2,3-dihydro-1H-inden-1-yl)oxy)-2-methoxy-5-(trifluoromethyl)-nicotinaldehyde

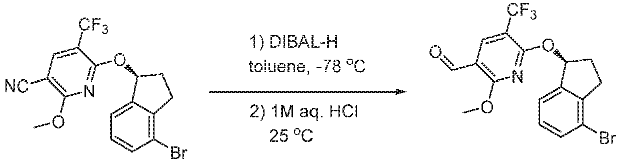

6-[(1S)-4-Bromoindan-1-yl]oxy-2-methoxy-5-(trifluoromethyl)pyridine-3-carbonitrile (84 mg, 0.20 mmol) was dissolved in 2 mL of toluene under a nitrogen atmosphere and the mixture was cooled to -78 °C. A solution of DIBAL-H (0.41 mL, 0.41 mmol, 1 M in methylene chloride) was added drop-wise over 15 minutes, then the mixture was warmed to rt and stirred for an additional 30 minutes. The mixture was subsequently cooled to 0 °C and a mixture of 1 M aqueous HCl and MeOH (1:1 (*v*/*v*), 10 mL) was added to quench the reaction. The resulting solution was warmed to rt and stirred vigorously for 1 hour, then diluted with EtOAc (10 mL) and basified with aqueous 2 M NaOH solution. The mixture was extracted with EtOAc (3 x 5 mL) and the combined organics were further washed with brine (5 mL), dried over sodium sulfate, then filtered and concentrated under reduced pressure. The residue was purified by flash chromatography (SiO₂, 0-10 % EtOAc/hexane) to afford 6-[(1S)-4-bromoindan-1-yl]oxy-2-methoxy-5-(trifluoromethyl)pyridine-3-carbaldehyde (30 mg, 36 % yield). ¹H NMR (400 MHz, Chloroform-d) δ 10.23 (s, 1H), 8.36 (s, 1H), 7.49 (d, 1H), 7.37 (d, 1H), 7.13 (t, 1H), 6.67 (t, 1H), 4.15 (d, 3H), 3.28 - 3.10 (m, 1H), 2.96 (m, 1H), 2.83 - 2.70 (m, 1H), 2.28 (m, 1H).

### (e) (S)-2-Methoxy-6-((4-(4,4,5,5-tetramethyl-1,3,2-dioxaborolan-2-yl)-2,3-dihydro-1H-inden-1-yl)oxy)-5-(trifluoromethyl)nicotinaldehyde

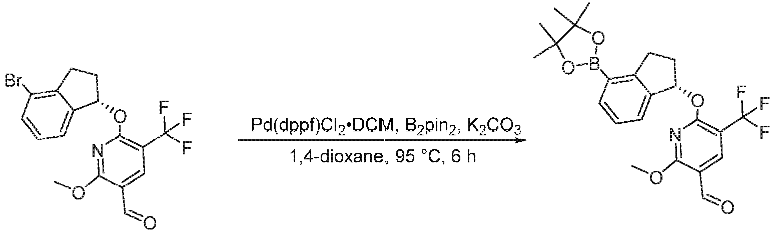

6-[(1S)-4-Bromoindan-1-yl]oxy-2-methoxy-5-(trifluoromethyl)pyridine-3-carbaldehyde (87 mg, 0.21 mmol), 4,4,5,5-tetramethyl-2-(4,4,5,5-tetramethyl-1,3,2-dioxaborolan-2-yl)-1,3,2-dioxaborolane (106 mg, 0.42 mmol), 1,1'-bis(diphenylphosphino)-ferrocene-palladium(II) dichloride dichloromethane complex (17 mg, 0.02 mmol), and potassium carbonate (81 mg, 0.59 mmol) were suspended in 4 mL 1,4-dioxane in a sealed tube. Nitrogen gas was bubbled through the reaction solution for 2 minutes, then the vessel was sealed and heated at 95 °C for 6 hours. The mixture was then allowed to cool to room temperature and filtered through CELITE^{®}. The filtrate was concentrated under reduced pressure and the residue was purified by flash chromatography (SiO₂, 0-10 % EtOAc/hexane) to afford 2-methoxy-6-[(1S)-4-(4,4,5,5-tetramethyl-1,3,2-dioxaborolan-2-yl)indan-1-yl]oxy-5-(trifluoromethyl)pyridine-3-carbaldehyde (80 mg, 83 % yield). MS: *m*/*z* found 464 [M+H]⁺. ¹H NMR (400 MHz, Chloroform-*d*) δ 10.22 (s, 1H), 8.34 (s, 1H), 7.78 (d, 1H), 7.52 (d, 1H), 7.23 (d, 1H), 6.62 (dd, 1H), 4.15 (s, 3H), 3.40 (m, 1H), 3.21 - 3.08 (m, 1H), 2.68 (m, 1H), 2.31 - 2.10 (m, 1H), 1.34 (s, 12H).

### (f) 2',3'-Dichloro-6-methoxy-[2,4'-bipyridine]-5-carbaldehyde

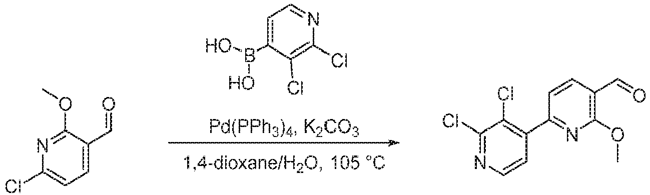

Pd(PPh₃)₄ (0.24 g, 0.21 mmol), 6-chloro-2-methoxy-pyridine-3-carbaldehyde (0.72 g, 4.17 mmol), potassium carbonate (0.86 g, 6.26 mmol), and (2,3-dichloro-4-pyridyl)boronic acid (0.40 g, 2.09 mmol) were suspended in 1,4-dioxane/water (4:1 (*v*/*v*), 10 mL), then the solution was heated at 105 °C for 20 minutes. The mixture was allowed to cool to room temperature, diluted with 20 mL of water, and extracted with EtOAc (3 x 10 mL). The combined organics were concentrated under reduced pressure and crude sample was purified by flash chromatography (SiO₂, 0-30 % EtOAc/hexane) to afford *tert-*butyl N-[2-[3-[3-[5-[[tert-butoxycarbonyl-[[(2S)-5-oxopyrrolidin-2-yl]methyl]amino]methyl]-6-methoxy-2-pyridyl]-2-chloro-phenyl]-2-chloro-phenyl]-5,6,7,8-tetrahydroquinolin-5-yl]-N-[[(2S)-5-oxopyrrolidin-2-yl]methyl]carbamate (186 mg, 32 % yield). MS: *m*/*z* found 283, 285 [M+H] ⁺.

### (g) tert-butyl (S)-((2',3'-Dichloro-6-methoxy-[2,4'-bipyridin]-5-yl)-methyl)-((5-oxopyrrolidin-2-yl)-methyl)-carbamate

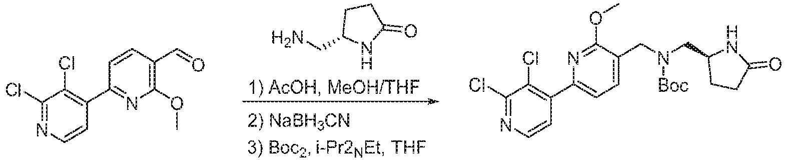

6-(2,3-Dichloro-4-pyridyl)-2-methoxy-pyridine-3-carbaldehyde (186 mg, 0.66 mmol), acetic acid (39 mg, 0.66 mmol), and (5S)-5-(aminomethyl)pyrrolidin-2-one (113 mg, 0.99 mmol were dissolved in MeOH/THF (1:1 (*v*/*v*), 5 mL), then the solution was stirred at 25 °C for 2 h. Sodium cyanoborohydride (83 mg, 1.31 mmol) was added and the resulting mixture was stirred at 25 °C for 1 h. The mixture was subsequently diluted with water (10 mL) and extracted with EtOAc (3 x 10 mL). The combined organics were further washed with brine, dried over sodium sulfate, filtered, and concentrated under reduced pressure to afford (5S)-5-[[[6-(2,3-dichloro-4-pyridyl)-2-methoxy-3-pyridyl]methylamino]methyl]pyrrolidin-2-one (250 mg, crude). MS: *m*/*z* found 381, 383 [M+H]⁺. The material was used for the next step without further purification.

(55)-5-[[16-(2,3-Dichloro-4-pyridyl)-2-methoxy-3-pyridyl]-methylamino]-methyl]pyrrolidin-2-one (0.25 g, 0.66 mmol, crude) and *N,N*-diisopropylethylamine (0.23 mL, 1.31 mmol) were dissolved in 5 ml of THF, then di-*tert-*butyl dicarbonate (0.22 g, 0.98 mmol) was added portion-wise. The resulting mixture was stirred at room temperature for 4 h. EtOAc (15 ml) was added to the reaction mixture and the resulting solution was washed with saturated aqueous NaHCO₃ solution (10 mL × 2), and brine (10 mL × 2). The organic phase was concentrated under reduced pressure and the residue was purified by flash chromatography (SiO₂, 0-5 % MeOH/DCM) to afford *tert-*butyl N-[[6-(2,3-dichloro-4-pyridyl)-2-methoxy-3-pyridyl]methyl]-N-[[(2S)-5-oxopyrrolidin-2-yl]methyl]carbamate (0.30 g, 96 % yield) . MS: *m*/*z* found 481, 483 [M+H] ⁺.

### (h) tert-Butyl ((3'-chloro-2'-((S)-1-((5-formyl-6-methoxy-3-(trifluoromethyl)pyridin-2-yl)oxy)-2,3-dihydro-1H-inden-4-yl)-6-methoxy-[2,4'-bipyridin]-5-yl)methyl)(((S)-5-oxopyrrolidin-2-yl)methyl)carbamate

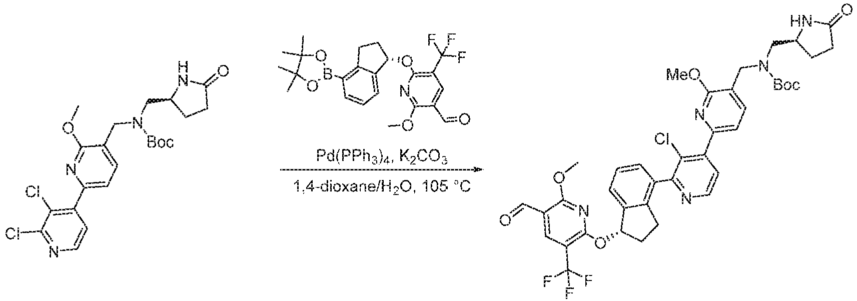

Pd(PPh₃)₄ (24 mg, 0.02 mmol), potassium carbonate (43 mg, 0.31 mmol), 2-methoxy-6-[(1S)-4-(4,4,5,5-tetramethyl-1,3,2-dioxaborolan-2-yl)indan-1-yloxy-5-(trifluoromethyl)pyridine-3-carbaldehyde (48 mg, 0.10 mmol), and *tert-*butyl N-[[6-(2,3-dichloro-4-pyridyl)-2-methoxy-3-pyridyl]methyl]-N-[[(2S)-5-oxopyrrolidin-2-yl]methyl]carbamate (75 mg, 0.16 mmol) were suspended in 1,4-dioxane/water (4:1 *(v*/*v),* 2 mL), and the solution was heated at 105 °C for 20 min. The mixture was allowed to cooled to room temperature, diluted with 10 mL of water, and extracted with EtOAc (3 x 5 mL). The combined organics were concentrated under reduced pressure and residue was purified by flash chromatography (SiO₂, 0-4 % MeOH/DCM) to afford *tert-*butyl N-[[6-[3-chloro-2-[(1S)-1-[[5-formyl-6-methoxy-3-(trifluoromethyl)-2-pyridyl]oxy]indan-4-yl]-4-pyridyl]-2-methoxy-3-pyridyl]methyl]-N-[[(2S)-5-oxopyrrolidin-2-yl]methyl] carbamate (45 mg, 56 % yield). MS: *m*/*z* found 782, 784 [M+H] ⁺.

### (i) tert-Butyl ((3'-chloro-2'-((S)-1-((5-((((S)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)(((S)-5-oxopyrrolidin-2-yl)methyl)carbamate

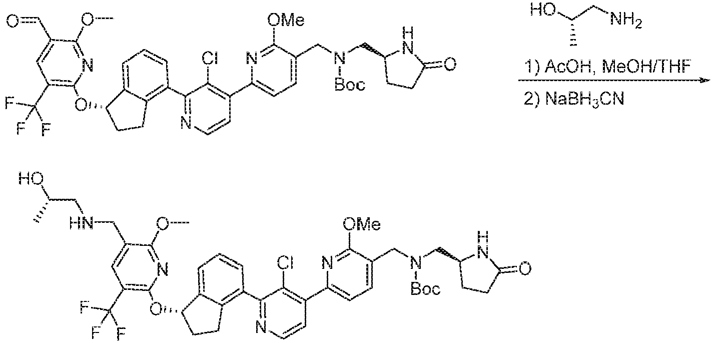

*tert-*Butyl N-[[6-[3-chloro-2-[(1S)-1-[[5-formyl-6-methoxy-3-(trifluoromethyl)-2-pyridyl]oxy]indan-4-yl]-4-pyridyl]-2-methoxy-3-pyridyl]methyl]-N-[[(2S)-5-oxopyrrolidin-2-yl]methyl]carbamate (15 mg, 0.02 mmol), acetic acid (2 mg, 0.04 mmol), and (2S)-1-aminopropan-2-ol (4 mg, 0.06 mmol) were dissolved in MeOH/THF (1:1 (*v*/*v*)*,* 1 mL) and the resulting solution was stirred at 25 °C for 6 h. Sodium cyanoborohydride (2 mg, 0.04 mmol) was added and the resulting mixture was stirred at 25 °C for 1 h. The mixture was subsequently diluted with water (2 mL) and extracted with EtOAc (3 x 2 mL). The combined organics were further washed with brine, dried over sodium sulfate, filtered, and concentrated under reduced pressure to afford *tert-*butyl N-[[6-[3-chloro-2-[(1S)-1-[[5-[[[(2S)-2-hydroxypropyl]amino]methyl]-6-methoxy-3-(trifluoromethyl)-2-pyridyl]oxy]indan-4-yl]-4-pyridyl]-2-methoxy-3-pyridyl]methyl]-N-[[(2S)-5-oxopyrrolidin-2-yl]methyl]carbamate (16 mg, crude) as a yellow oil. MS: *m*/*z* found 841, 843 [M+H]⁺. Material was used for the next step without further purification.

### (j) (S)-5-((((3'-Chloro-2'-((S)-1-((5-((((S)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)methyl)pyrrolidin-2-one

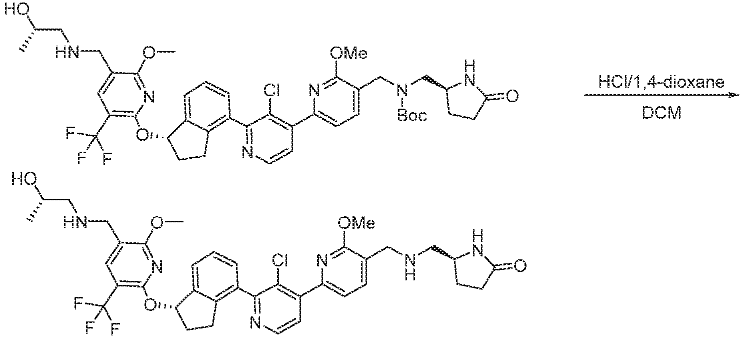

tert-Butyl N-[[6-[3-chloro-2-[(1S)-1-[[5-[[[(2S)-2-hydroxypropyl]amino]methyl]-6-methoxy-3-(trifluoromethyl)-2-pyridyl]oxy]indan-4-yl]-4-pyridyl]-2-methoxy-3-pyridyl]methyl]-N-[[(2S)-5-oxopyrrolidin-2-yl]methyl]carbamate (16 mg, 0.02 mmol, crude) was dissolved in 1 mL MeOH and 4 M HCl solution in 1,4-dioxane (19 µL, 0.08 mmol) was added. The mixture was stirred at room temperature for 1 h and solvent was removed under reduced pressure. The residue was purified by reverse phase HPLC to afford (5S)-5-[[[6-[3-chloro-2-[(1S)-1-[[5-[[[(2S)-2-hydroxypropyl]amino]methyl]-6-methoxy-3-(trifluoromethyl)-2-pyridyl]oxy]indan-4-yl]-4-pyridyl]-2-methoxy-3-pyridyl]methylamino]methyl]pyrrolidin-2-one (8 mg, 56 % yield) as a white solid (formic acid salt) LCMS: *m*/*z* found 741, 744 [M+H]⁺, retention time = 2.28 min (Method C). ¹H NMR (400 MHz, Methanol-*d*₄) δ 8.61 (d, 1H), 8.05 (s, 1H), 7.85 (d, 1H), 7.71 (d, 1H), 7.56 (s, 1H), 7.48 - 7.37 (m, 3H), 6.71 (s, 1H), 4.21 (d, 2H), 4.17 (t, 3H), 4.05 (t, 4H), 3.97 (s, 2H), 3.90 (s, 1H), 3.14 - 2.97 (m, 2H), 2.92 - 2.70 (m, 5H), 2.35 (d, 4H), 1.84 (s, 1H), 1.24 (d, 3H).

### Example 138: 1-(((6-(((S)-4-(3'-Chloro-6-methoxy-5-(((((S)-5-oxopyrrolidin-2-yl)methyl)-amino)-methyl)-[2,4'-bipyridin]-2'-yl)-2,3-dihydro-1H-inden-1-yl)oxy)-2-methoxy-5-(trifluoromethyl)pyridin-3-yl)methyl)amino)cyclopropane-1-carboxylic acid (129)

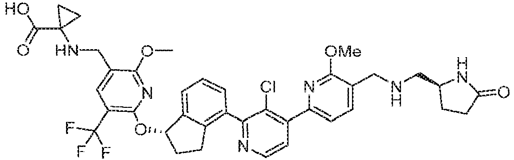

1-(((6-(((S)-4-(3'-Chloro-6-methoxy-5-(((((S)-5-oxopyrrolidin-2-yl)methyl)-amino)-methyl)-[2,4'-bipyridin]-2'-yl)-2,3-dihydro-1H-inden-1-yl)oxy)-2-methoxy-5-(trifluoromethyl)pyridin-3-yl)methyl)amino)cyclopropane-1-carboxylic acid was prepared in a similar manner as described for compound **128** utilizing intermediate *tert-*butyl ((3'-chloro-2'-((S)-1-((5-formyl-6-methoxy-3-(trifluoromethyl)pyridin-2-yl)oxy)-2,3-dihydro-1H-inden-4-yl)-6-methoxy-[2,4'-bipyridin]-5-yl)methyl)(((S)-5-oxopyrrolidin-2-yl)methyl)carbamate. 1-Aminocyclopropane-1-carboxylic acid was used in place of (S)-1-aminopropan-2-ol. LCMS: *m*/*z* found 767, 769 [M+H]⁺, retention time = 2.30 min. (Method C). ¹H NMR (400 MHz, Methanol-*d*₄) δ 8.61 (d, 1H), 8.42 (s, 1H), 8.04 (s, 1H), 7.84 (d, 1H), 7.74 - 7.66 (m, 1H), 7.56 (s, 1H), 7.46 - 7.35 (m, 3H), 6.70 (d, 1H), 4.32 (s, 2H), 4.15 (d, 3H), 4.05 (s, 3H), 3.91 (d, 3H), 3.00 (s, 1H), 2.89 - 2.66 (m, 4H), 2.38 - 2.14 (m, 4H), 1.84 (s, 1H), 1.39 (s, 2H), 1.18 (s, 2H).

### Example 139: (S)-5-((((3'-Chloro-2'-((S)-1-((5-((((R)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)methyl)pyrrolidin-2-one (165)

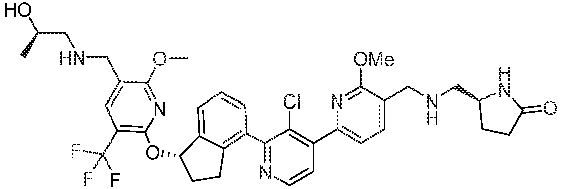

(S)-5-((((3'-Chloro-2'-((S)-1-((5-((((R)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)methyl)pyrrolidin-2-one was prepared in a similar manner as described for compound **128** utilizing intermediate *tert-*butyl ((3'-chloro-2'-((S)-1-((5-formyl-6-methoxy-3-(trifluoromethyl)pyridin-2-yl)oxy)-2,3-dihydro-1H-inden-4-yl)-6-methoxy-[2,4'-bipyridin]-5-yl)methyl)(((S)-5-oxopyrrolidin-2-yl)methyl)carbamate. (R)-1-aminopropan-2-ol was used in place of (S)-1-aminopropan-2-ol. LCMS: *m*/*z* found 741, 743 [M+H]⁺, retention time = 2.27 min. (Method C). ¹H NMR (400 MHz, Methanol-*d*₄) δ 8.61 (d, 1H), 8.04 (s, 1H), 7.84 (d, 1H), 7.71 (d, 1H), 7.56 (s, 1H), 7.42 (d, 3H), 6.71 (s, 1H), 4.18 (d, 5H), 4.04 (s, 4H), 3.90 (s, 3H), 3.03 (d, 2H), 2.80 (d, 5H), 2.28 (d, 4H), 1.84 (s, 1H), 1.24 (s, 3H).

### Example 140: (S)-5-((((3'-Chloro-6-methoxy-2'-((S)-1-((6-methoxy-5-(((((S)-5-oxopyrrolidin-2-yl)methyl)amino)methyl)-3-(trifluoromethyl)pyridin-2-yl)oxy)-2,3-dihydro-1H-inden-4-yl)-[2,4'-bipyridin]-5-yl)methyl)amino)methyl)pyrrolidin-2-one (166)

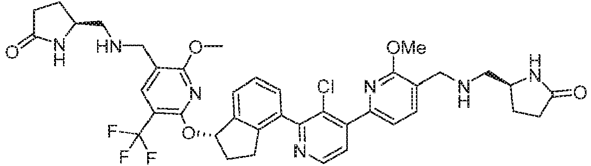

(S)-5-((((3'-Chloro-6-methoxy-2'-((S)-1-((6-methoxy-5-(((((S)-5-oxopyrrolidin-2-yl)methyl)amino)methyl)-3-(trifluoromethyl)pyridin-2-yl)oxy)-2,3-dihydro-1H-inden-4-yl)-[2,4'-bipyridin]-5-yl)methyl)amino)methyl)pyrrolidin-2-one was prepared in a similar manner as described for compound **128** utilizing intermediate *tert-*butyl ((3'-chloro-2'-((S)-1-((5-formyl-6-methoxy-3-(trifluoromethyl)pyridin-2-yl)oxy)-2,3-dihydro-1H-inden-4-yl)-6-methoxy-[2,4'-bipyridin]-5-yl)methyl)(((S)-5-oxopyrrolidin-2-yl)methyl)carbamate. (S)-5-(aminomethyl)pyrrolidin-2-one was used in place of (S)-1-aminopropan-2-ol. MS: *m*/*z* found 780, 782 [M+H] ⁺, LC retention time = 2.25 min. (Method C). ¹H NMR (400 MHz, Methanol-*d*₄) δ 8.61 (dd, 1H), 8.37 (s, 1H), 7.97 (s, 1H), 7.86 (d, 1H), 7.71 (d, 1H), 7.55 (s, 1H), 7.44 (d, 1H), 7.40 (d, 2H), 6.69 (s, 1H), 4.13 (d, 3H), 4.06 (d, 3H), 3.98 (d, 4H), 3.91 (s, 2H), 3.00 (s, 1H), 2.94 - 2.68 (m, 6H), 2.35 (q, 6H), 2.22 (s, 1H), 1.85 (t, 2H).

### Example 141: Methyl 1-(((6-(((S)-4-(3'-chloro-6-methoxy-5-(((((S)-5-oxopyrrolidin-2-yl)methyl)amino)methyl)-[2,4'-bipyridin]-2'-yl)-2,3-dihydro-1H-inden-1-yl)amino)-2-methoxy-5-(trifluoromethyl)pyridin-3-yl)methyl)amino)cyclopropane-1-carboxylate (149)

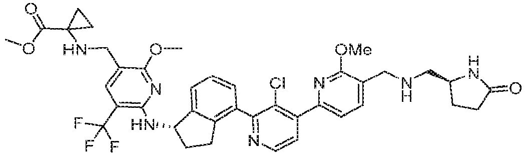

### (a) (S)-6-((4-Bromo-2,3-dihydro-1H-inden-1-yl)amino)-2-methoxynicotinonitrile

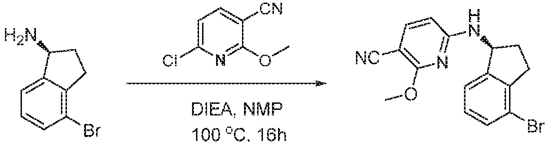

(1S)-4-Bromoindan-1-amine;hydrochloride (0.60 g, 2.41 mmol), 6-chloro-2-methoxy-pyridine-3-carbonitrile (0.41 g, 2.41 mmol), and *N,N*-diisopropylethylamine (624 mg, 4.83 mmol) were suspended in 10 ml of NMP and the mixture was stirred at 100 °C for 16 h. The mixture was allowed to cool to room temperature, as diluted with 30 ml EtOAc and washed with water (2 x 30mL), brine (2 x 20 mL), dried over sodium sulfate, filtered, and concentrated under reduced pressure. The residue was purified by flash chromatography (SiO₂, 0-30 % EtOAc/hexane) to afford 6-[[(1S)-4-bromoindan-1-yl]amino]-2-methoxy-pyridine-3-carbonitrile (224 mg, 27 % yield). MS: *m*/*z* found 344, 346 [M+H] ⁺.

### (b) (S)-6-((4-Bromo-2,3-dihydro-1H-inden-1-yl)amino)-5-iodo-2-methoxynicotinonitrile

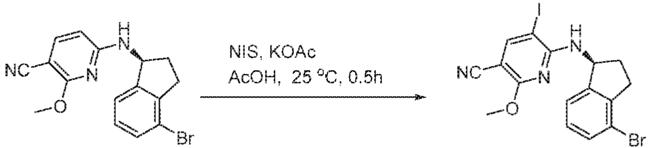

6-[[(1S)-4-Bromoindan-1-yl]amino]-2-methoxy-pyridine-3-carbonitrile (0.22 g, 0.65 mmol), 1-iodopyrrolidine-2,5-dione (0.16 g, 0.72 mmol), and potassium acetate (0.64 g, 6.51 mmol) were suspended in 4 ml of acetic acid and the mixture was stirred at room temperature for 30 min. The mixture was then diluted with 50 ml of water and the precipitate was collected by filtration. The filter cake was further washed with water (2 x 50 mL), then dried to afford 6-[[(1S)-4-bromoindan-1-yl]amino]-5-iodo-2-methoxy-pyridine-3-carbonitrile (0.29 g, 95 % yield). MS: *m*/*z* found 470, 472 [M+H] ⁺.

### (c) (S)-6-((4-Bromo-2,3-dihydro-1H-inden-1-yl)amino)-2-methoxy-5-(trifluoromethyl)-nicotinonitrile

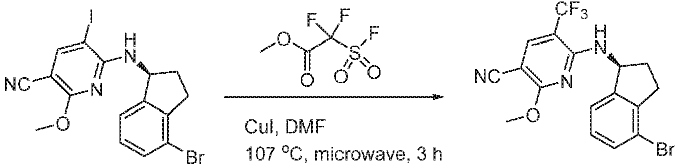

In a microwave vial 6-[[(1S)-4-bromoindan-1-yl]amino]-5-iodo-2-methoxy-pyridine-3-carbonitrile (0.29 g, 0.61 mmol) and copper iodide (0.18 g, 0.92 mmol) were suspended in 8 mL DMF. Methyl 2,2-difluoro-2-fluorosulfonyl-acetate (0.24 g, 1.23 mmol) was added, and the mixture was purged with nitrogen gas for 5 minutes. The mixture was then subjected to microwave irradiation maintaining a reaction temperature of 107 °C for 3 h. The mixture was cooled to room temperature, diluted with 10 mL of EtOAc, and the insoluble copper salts were removed by filtration. The filtrate was washed with water (3 x 20 mL) and the organic solution was concentrated under reduced pressure. The residue was purified by flash chromatography (SiO₂, 0-10 % EtOAc/hexane) to afford 6-[[(1S)-4-bromoindan-1-yl]amino]-2-methoxy-5-(trifluoromethyl)pyridine-3-carbonitrile (235 mg, 93 % yield). MS: *m*/*z* found 412, 414 [M+H] ⁺.

### (d) (S)-6-((4-Bromo-2,3-dihydro-1H-inden-1-yl)amino)-2-methoxy-5-(trifluoromethyl)-nicotinaldehyde

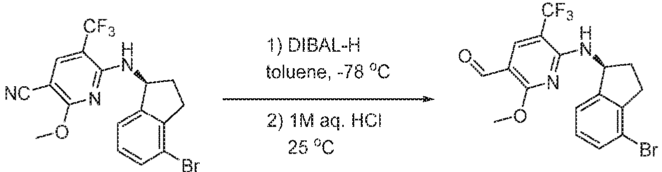

6-[[(1S)-4-Bromoindan-1-yl]-amino]-2-methoxy-5-(trifluoromethyl)-pyridine-3-carbonitrile (0.24 g, 0.57 mmol) was dissolved in 5 mL of toluene under a nitrogen atmosphere and the solution was cooled to -78 °C. A solution of DIBAL-H (1.14 mL, 1.14 mmol, 1 M in methylene chloride) was added drop-wise over 15 minutes, then the reaction was allowed to warm to room temperature and stirred for an additional 30 min. The mixture was subsequently cooled to 0 °C and a mixture of 1 M aqueous HCl and MeOH (1:1 (*v*/*v*), 10 mL) was added to quench the reaction. The resulting solution was warmed to rt and stirred vigorously for 1 h, then diluted with EtOAc (10 mL) and basified with aqueous 2 M NaOH solution. The mixture was then extracted with EtOAc (3 x 10 mL) and the combined organics were further washed with brine (10 mL), dried over sodium sulfate, then filtered and concentrated under reduced pressure. The residue was purified by flash chromatography (SiO₂, 0-10 % EtOAc/hexane) to afford 6-[[(1S)-4-bromoindan-1-yl]amino]-2-methoxy-5-(trifluoromethyl)pyridine-3-carbaldehyde (97 mg, 41 % yield). MS: m/z found 415, 417 [M+H] ⁺.

### (e) (S)-2-Methoxy-6-((4-(4,4,5,5-tetramethyl-1,3,2-dioxaborolan-2-yl)-2,3-dihydro-1H-inden-1-yl)amino)-5-(trifluoromethyl)nicotinaldehyde

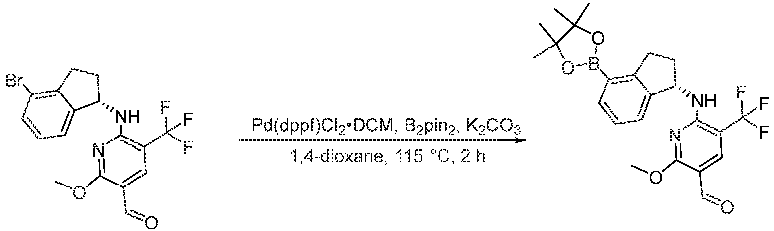

6-[[(1S)-4-Bromoindan-1-yl]amino]-2-methoxy-5-(trifluoromethyl)pyridine-3-carbaldehyde (30 mg, 0.07 mmol), 4,4,5,5-tetramethyl-2-(4,4,5,5-tetramethyl-1,3,2-dioxaborolan-2-yl)-1,3,2-dioxaborolane (37 mg, 0.14 mmol), 1,1'-bis(diphenylphosphino)-ferrocene-palladium(II) dichloride dichloromethane complex (6 mg, 0.01 mmol), and potassium carbonate (28 mg, 0.20 mmol) were suspended in 2 mL of 1,4-dioxane in a sealed tube. Nitrogen gas was bubbled through the reaction solution for 2 minutes, then the vessel was sealed and heated at 115 °C in the microwave for 2 h. The reaction was cooled to room temperature and the mixture was filtered through CELITE^{®}. The filtrate was concentrated under reduced pressure and the residue was purified by flash chromatography (SiO₂, 0-10 % EtOAc/hexane) to afford 2-methoxy-6-[[(1S)-4-(4,4,5,5-tetramethyl-1,3,2-dioxaborolan-2-yl)indan-1-yl]amino]-5-(trifluoromethyl)pyridine-3-carbaldehyde (21 mg, 63 % yield). MS: m/z found 463 [M+H] ⁺.

### (f) tert-Butyl ((3'-chloro-2'-((S)-1-((5-formyl-6-methoxy-3-(trifluoromethyl)-pyridin-2-yl)amino)-2,3-dihydro-1H-inden-4-yl)-6-methoxy-[2,4'-bipyridin]-5-yl)methyl)-(((S)-5-oxopyrrolidin-2-yl)methyl)carbamate

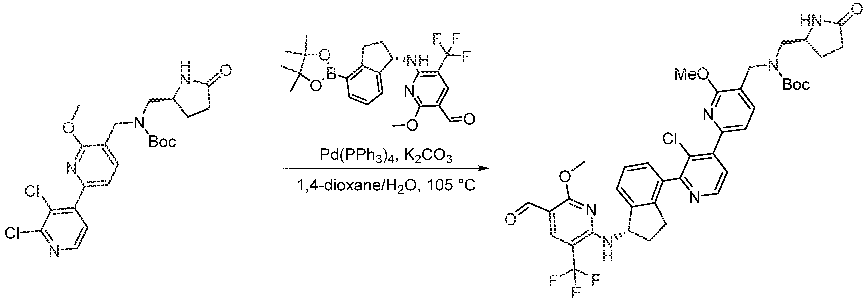

Pd(PPh₃)₄ (15 mg, 0.01 mmol), potassium carbonate (26 mg, 0.19 mmol), 2-methoxy-6-[[(1S)-4-(4,4,5,5-tetramethyl-1,3,2-dioxaborolan-2-yl)indan-1-yl]amino]-5-(trifluoromethyl)pyridine-3-carbaldehyde (29 mg, 0.06 mmol), and *tert-*butyl N-[[6-(2,3-dichloro-4-pyridyl)-2-methoxy-3-pyridyl]methyl]-N-[[(2S)-5-oxopyrrolidin-2-yl]methyl]carbamate (45 mg, 0.09 mmol) were suspended in 1,4-dioxane/water (4:1 *(v*/*v),* 2 mL), then the solution was heated at 105 °C for 20 minutes. The mixture was allowed to cool to room temperature, diluted with 10 mL water, and extracted with EtOAc (3 x 5 mL). The combined organics were concentrated under reduced pressure and the residue was purified by flash chromatography (SiO₂, 0-4 % MeOH/methylene chloride) to afford *tert-*butyl N-[[6-[3-chloro-2-[(1S)-1-[[5-formyl-6-methoxy-3-(trifluoromethyl)-2-pyridyl]amino]indan-4-yl]-4-pyridyl]-2-methoxy-3-pyridyl]methyl]-N-[[(2S)-5-oxopyrrolidin-2-yl]methyl]carbamate (25 mg, 51 % yield). MS: *m*/*z* found 781, 783 [M+H] ⁺.

### (g) Methyl 1-(((6-(((S)-4-(5-(((tert-butoxycarbonyl)(((S)-5-oxopyrrolidin-2-yl)methyl)amino)methyl)-3'-chloro-6-methoxy-[2,4'-bipyridin]-2'-yl)-2,3-dihydro-1H-inden-1-yl)amino)-2-methoxy-5-(trifluoromethyl)pyridin-3-yl)methyl)amino)cyclopropane-1-carboxylate

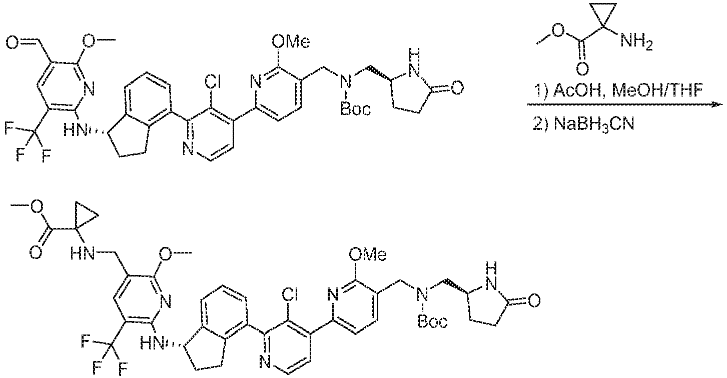

*tert-*Butyl N-[[6-[3-chloro-2-[(1S)-1-[[5-formyl-6-methoxy-3-(trifluoromethyl)-2-pyridyl]amino]indan-4-yl]-4-pyridyl]-2-methoxy-3-pyridyl]methyl]-N-[[(2S)-5-oxopyrrolidin-2-yl]methyl]carbamate (25 mg, 0.03 mmol), acetic acid (2 mg, 0.03 mmol), and methyl 1-aminocyclopropanecarboxylate (15 mg, 0.10 mmol) were dissolved in MeOH/THF (1:1 (*v*/*v*), 1 mL), then the solution was stirred at 25 °C for 16 hours. Sodium cyanoborohydride (4 mg, 0.06 mmol) was added and the resulting mixture was stirred at 25 °C for 3 hours. The mixture was subsequently diluted with water (2 mL) and extracted with EtOAc (3 x 2 mL). The combined organics were further washed with brine, dried over sodium sulfate, filtered, and concentrated under reduced pressure to afford methyl 1-[[6-[[(1S)-4-[4-[5-[[*tert*-butoxycarbonyl-[[(2S)-5-oxopyrrolidin-2-yl]methyl]amino]methyl]-6-methoxy-2-pyridyl]-3-chloro-2-pyridyl]indan-1-yl]amino]-2-methoxy-5-(trifluoromethyl)-3-pyridyl]methylamino]cyclopropanecarboxylate (32 mg, crude) as a yellow oil. MS: m/z found 880, 882 [M+H]⁺. Material was used for the next step without further purification.

### (h) Methyl 1-(((6-(((S)-4-(3'-chloro-6-methoxy-5-(((((S)-5-oxopyrrolidin-2-yl)-methyl)amino)methyl)-[2,4'-bipyridin]-2'-yl)-2,3-dihydro-1H-inden-1-yl)amino)-2-methoxy-5-(trifluoromethyl)pyridin-3-yl)methyl)amino)cyclopropane-1-carboxylate

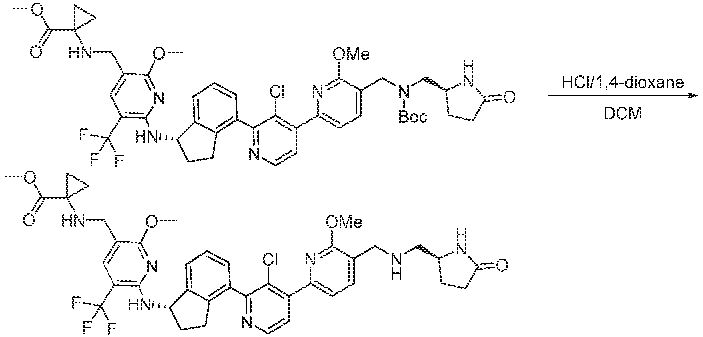

Methyl 1-[[6-[[(1S)-4-[4-[5-[[tert-butoxycarbonyl-[[(2S)-5-oxopyrrolidin-2-yl]methyl]amino]methyl]-6-methoxy-2-pyridyl]-3-chloro-2-pyridyl]indan-1-yl]amino]-2-methoxy-5-(trifluoromethyl)-3-pyridyl]methylamino]cyclopropanecarboxylate (32 mg, 0.02 mmol, crude) was dissolved in 1 mL MeOH and 4 M HCl solution in 1,4-dioxane (19 µL, 0.08 mmol) was added. The mixture was stirred at room temperature for 1 h and solvent was removed under reduced pressure. The residue was purified by reverse phase HPLC to afford methyl 1-[[6-[[(1S)-4-[3-chloro-4-[6-methoxy-5-[[[(2S)-5-oxopyrrolidin-2-yl]methylamino]methyl]-2-pyridyl]-2-pyridyl]indan-1-yl]amino]-2-methoxy-5-(trifluoromethyl)-3-pyridyl]methylamino]-cyclopropanecarboxylate (5 mg, 35 % yield) (formic acid salt) MS: *m*/*z* found 780, 782 [M+H] ⁺, LC retention time = 2.40 min (Method C). ¹H NMR (400 MHz, Methanol-*d*₄) δ 8.60 (d, 1H), 8.54 (s, 1H), 7.81 (d, 1H), 7.69 (d, 1H), 7.60 (s, 1H), 7.40 (d, 1H), 7.38 - 7.27 (m, 3H), 5.86 (q, 1H), 5.78 (d, 1H), 4.03 (s, 3H), 3.90 (s, 3H), 3.84 (t, 3H), 3.73 (s, 2H), 3.64 (s, 3H), 2.83 (dd, 2H), 2.67 (m, 3H), 2.37 - 2.22 (m, 3H), 2.11 - 1.98 (m, 1H), 1.87 - 1.75 (m, 1H), 1.24 (q, 2H), 1.03 (q, 2H).

### Example 142: 1-(((6-(((S)-4-(3'-Chloro-6-methoxy-5-(((((S)-5-oxopyrrolidin-2-yl)methyl)amino)methyl)-[2,4'-bipyridin]-2'-yl)-2,3-dihydro-1H-inden-1-yl)amino)-2-methoxy-5-(trifluoromethyl)pyridin-3-yl)methyl)amino)cyclopropane-1-carboxylic acid (164)

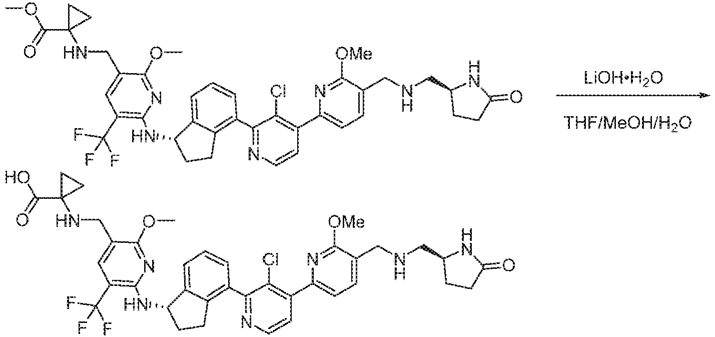

Methyl 1-[[6-[[(1S)-4-[3-chloro-4-[6-methoxy-5-[[[(2S)-5-oxopyrrolidin-2-yl]methylamino]methyl]-2-pyridyl]-2-pyridyl]indan-1-yl]amino]-2-methoxy-5-(trifluoromethyl)-3-pyridyl]methylamino]cyclopropanecarboxylate (3 mg, 0.004 mmol) was dissolved in THF/MeOH/water (3:1:1, 0.5 mL) and lithium hydroxide monohydrate (1 mg, 0.02 mmol) was added. Reaction was stirred at rt for 16 hours and solvent was removed under reduced pressure. Crude material was purified by reverse phase HPLC to afford 1-[[6-[[(1S)-4-[3-chloro-4-[6-methoxy-5-[[[(2S)-5-oxopyrrolidin-2-yl]methylamino]methyl]-2-pyridyl]-2-pyridyl]indan-1-yl]amino]-2-methoxy-5-(trifluoromethyl)-3-pyridyl]methylamino]cyclopropanecarboxylic acid (1.6 mg, 54 % yield) as a white solid (formic acid salt) MS: *m*/*z* found 766, 768 [M+H] ⁺, retention time = 2.30 min (Method C). ¹H NMR (400 MHz, Methanol-*d*₄) δ 8.60 (d, 1H), 8.54 (d, 1H), 7.81 (d, 2H), 7.73 - 7.65 (m, 1H), 7.40 (d, 1H), 7.32 (s, 3H), 6.13 (d, 1H), 5.88 (d, 1H), 4.16 (s, 2H), 4.03 (t, 3H), 3.92 (s, 3H), 3.84 (s, 3H), 2.83 (d, 2H), 2.76 - 2.57 (m, 3H), 2.39 - 2.21 (m, 3H), 2.16 - 2.00 (m, 1H), 1.81 (d, 1H), 1.33 (s, 2H), 1.11 (s, 2H).

### Example 143: 7-((6-(2-Chloro-3-(3-chloro-2-(3-methoxy-4-((2-oxo-1,7-diazaspiro[3.5]nonan-7-yl)methyl)phenyl)pyridin-4-yl)phenyl)-2-methoxypyridin-3-yl)methyl)-1,7-diazaspiro[3.5]nonan-2-one (353)

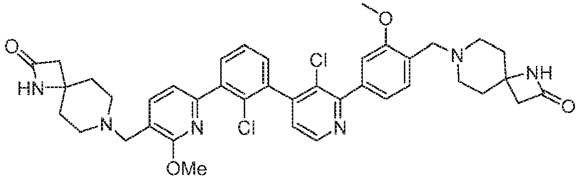

7-((6-(2-Chloro-3-(3-chloro-2-(3-methoxy-4-((2-oxo-1,7-diazaspiro[3.5]nonan-7-yl)methyl)phenyl)pyridin-4-yl)phenyl)-2-methoxypyridin-3-yl)methyl)-1,7-diazaspiro[3.5]nonan-2-one was prepared in similar manner as described for Example 11, replacing (5S)-5-(aminomethyl)pyrrolidin-2-one with 1,7-diazaspiro[3.5]-nonan-2-one in step (b). Product was obtained as a white solid (formic acid salt). LC/MS: *m*/*z* found 741, 743 [M+H]⁺, retention time = 2.08 min (Method C). ¹H NMR (400 MHz, Methanol-*d*₄) δ 8.64 (d, 1H), 8.43 (s, 1H), 7.79 (d, 1H), 7.72 (d, 1H), 7.55 (t, 1H), 7.50 (d, 1H), 7.45 (d, 1H), 7.41 (d, 1H), 7.33 (d, 2H), 7.28 (d, 1H), 4.02 (s, 2H), 4.00 (t, 3H), 3.94 (d, 3H), 3.71 (s, 2H), 3.01 (s, 4H), 2.75 (d, 4H), 2.69 (s, 2H), 2.62 (s, 2H), 1.98 (s, 4H), 1.89 (s, 4H).

### Example 144: (R)-5-((((6-(2-Chloro-3-(3-chloro-2-(3-methoxy-4-(((((R)-5-oxopyrrolidin-2-yl)methyl)amino)methyl)phenyl)pyridin-4-yl)phenyl)-2-methoxypyridin-3-yl)methyl)amino)methyl)pyrrolidin-2-one (354)

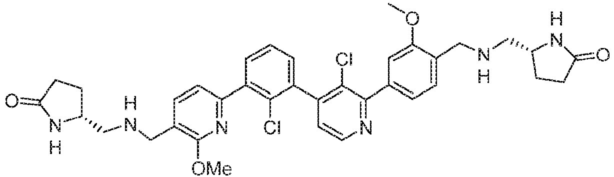

(R)-5-((((6-(2-chloro-3-(3-chloro-2-(3-methoxy-4-(((((R)-5-oxopyrrolidin-2-yl)methyl)amino)methyl)phenyl)pyridin-4-yl)phenyl)-2-methoxypyridin-3-yl)methyl)amino)methyl)pyrrolidin-2-one was prepared in similar manner as described for Example 11, replacing (5S)-5-(aminomethyl)pyrrolidin-2-one with (5R)-5-(aminomethyl)pyrrolidin-2-one in step (b). Product was obtained as a white solid (formic acid salt). LC/MS: *m*/*z* found 689, 691 [M+H]⁺, retention time = 2.01 min (Method C). ¹H NMR (400 MHz, Methanol-*d*₄) δ 8.67 - 8.62 (m, 1H), 8.43 (s, 1H), 7.79 (d, 1H), 7.73 - 7.68 (m, 1H), 7.55 (t, 1H), 7.47 (dd, 2H), 7.43 - 7.40 (m, 1H), 7.36 (s, 1H), 7.32 (d, 1H), 7.28 (d, 1H), 4.18 (d, 2H), 4.03 (d, 3H), 3.97 (t, 6H), 3.91 (d, 1H), 3.02 (d, 2H), 2.92 - 2.78 (m, 2H), 2.43 - 2.27 (m, 6H), 1.85 (s, 2H).

### Example 145: (S)-4-((((6-(2-Chloro-3-(3-chloro-2-(3-methoxy-4-(((((S)-5-oxopyrrolidin-3-yl)methyl)amino)methyl)phenyl)pyridin-4-yl)phenyl)-2-methoxypyridin-3-yl)methyl)amino)methyl)pyrrolidin-2-one (405)

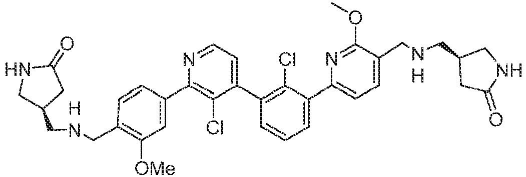

(S)-4-((((6-(2-Chloro-3-(3-chloro-2-(3-methoxy-4-(((((S)-5-oxopyrrolidin-3-yl)methyl)amino)methyl)phenyl)pyridin-4-yl)phenyl)-2-methoxypyridin-3-yl)methyl)amino)methyl)pyrrolidin-2-one was prepared in similar manner as described for Example 11, replacing (5S)-5-(aminomethyl)pyrrolidin-2-one with (S)-4-(aminomethyl)pyrrolidin-2-one in step (b). Product was obtained as a white solid (formic acid salt). LC/MS: *m*/*z* found 689, 691 [M+H]⁺, retention time = 1.98 min (Method C). ¹H NMR (400 MHz, Methanol-*d*₄) δ 8.65 (dd, 1H), 8.47 (s, 2H), 7.82 (d, 1H), 7.71 (d, 1H), 7.56 (t, 1H), 7.51 (d, 1H), 7.48 - 7.44 (m, 1H), 7.42 (d, 1H), 7.38 (s, 1H), 7.32 (dd, 2H), 4.24 (d, 2H), 4.05 (d, 5H), 3.98 (d, 3H), 3.64 - 3.53 (m, 2H), 3.20 - 3.10 (m, 4H), 2.97 (d, 2H), 2.87 (m, 2H), 2.52 (m, 2H), 2.26 - 2.13 (m, 2H).

### Example 146: (S)-1-((6-(2-Chloro-3-(3-chloro-2-(4-(((S)-3-hydroxypyrrolidin-1-yl)methyl)-3-methoxyphenyl)pyridin-4-yl)phenyl)-2-methoxypyridin-3-yl)methyl)pyrrolidin-3-ol (435)

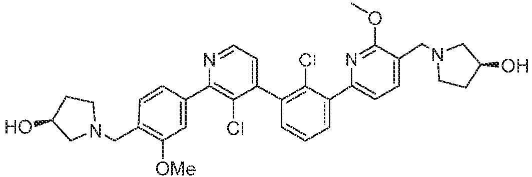

(S)-1-((6-(2-Chloro-3-(3-chloro-2-(4-(((S)-3-hydroxypyrrolidin-1-yl)methyl)-3-methoxyphenyl)pyridin-4-yl)phenyl)-2-methoxypyridin-3-yl)methyl)pyrrolidin-3-ol was prepared in similar manner as described for Example 11, replacing (5S)-5-(aminomethyl)pyrrolidin-2-one with (3S)-3-methylpyrrolidine in step (b). Product was obtained as a white solid (formic acid salt). LC/MS: *m*/*z* found 635, 637 [M+H]⁺, retention time = 2.07 min (Method C). ¹H NMR (400 MHz, Methanol-*d*₄) δ 8.68 - 8.61 (m, 1H), 8.52 (s, 1H), 7.84 (d, 1H), 7.72 (d, 1H), 7.59 - 7.51 (m, 2H), 7.47 (m, 1H), 7.45 - 7.41 (m, 1H), 7.39 (s, 1H), 7.35 (d, 1H), 7.30 (dd, 1H), 4.53 (s, 1H), 4.45 (s, 1H), 4.36 (s, 2H), 4.08 - 4.01 (m, 5H), 4.00 - 3.96 (m, 3H), 3.50 - 3.41 (m, 1H), 3.34 (d, 2H), 3.15 (t, 3H), 2.98 (s, 1H), 2.88 (d, 1H), 2.33 - 2.15 (m, 2H), 2.02 (s, 1H), 1.88 (d, 1H).

### Example 147: (S)-5-((((6-(2-Chloro-3-(3-chloro-2-(3-methoxy-4-((methyl(((S)-5-oxopyrrolidin-2-yl)methyl)amino)methyl)phenyl)pyridin-4-yl)phenyl)-2-methoxypyridin-3-yl)methyl)(methyl)amino)methyl)pyrrolidin-2-one (472)

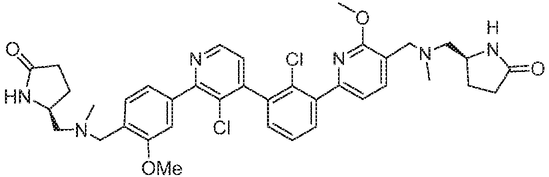

(S)-5-((((6-(2-Chloro-3-(3-chloro-2-(3-methoxy-4-((methyl(((S)-5-oxopyrrolidin-2-yl)methyl)amino)methyl)phenyl)pyridin-4-yl)phenyl)-2-methoxypyridin-3-yl)methyl)(methyl)amino)methyl)pyrrolidin-2-one was prepared in similar manner as described for Example 11 step (b), by reacting 6-(2-chloro-3-(3-chloro-2-(4-formyl-3-methoxyphenyl)pyridin-4-yl)phenyl)-2-methoxynicotinaldehyde first with (5S)-5-(aminomethyl)pyrrolidin-2-one, followed by formaldehyde (1 pot reaction). Product was obtained as a white solid (formic acid salt). LC/MS: *m*/*z* found 717, 719 [M+H]⁺, retention time = 2.04 min (Method C). ¹H NMR (400 MHz, Methanol-*d*₄) δ 8.62 (d, 1H), 7.79 (d, 1H), 7.71 (dd, 1H), 7.54 (t, 1H), 7.49 - 7.37 (m, 3H), 7.31 - 7.23 (m, 3H), 4.02 - 3.93 (m, 5H), 3.92 (d, 3H), 3.80 (d, 1H), 3.73 - 3.61 (m, 2H), 3.54 (d, 1H), 2.68 - 2.45 (m, 4H), 2.39 - 2.21 (m, 12H), 1.78 (d, 2H).

### Example 148: (R)-4-((((6-(2-Chloro-3-(3-chloro-2-(3-methoxy-4-((methyl(((R)-5-oxopyrrolidin-3-yl)methyl)amino)methyl)phenyl)pyridin-4-yl)phenyl)-2-methoxypyridin-3-yl)methyl)(methyl)amino)methyl)pyrrolidin-2-one (473)

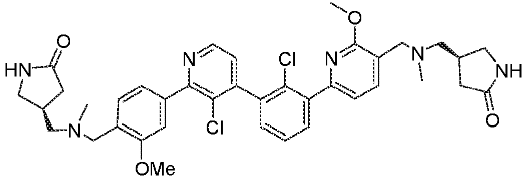

(R)-4-((((6-(2-Chloro-3-(3-chloro-2-(3-methoxy-4-((methyl(((R)-5-oxopyrrolidin-3-yl)methyl)amino)methyl)phenyl)pyridin-4-yl)phenyl)-2-methoxypyridin-3-yl)methyl)(methyl)amino)methyl)pyrrolidin-2-one was prepared in similar manner as described for Example 11 step (b), by reacting 6-(2-chloro-3-(3-chloro-2-(4-formyl-3-methoxyphenyl)pyridin-4-yl)phenyl)-2-methoxynicotinaldehyde first with (4S)-4-(aminomethyl)pyrrolidin-2-one, followed by formaldehyde (1 pot reaction). Product was obtained as a white solid (formic acid salt). LC/MS: *m*/*z* found 717, 719 [M+H]⁺, retention time = 2.00 min (Method C). ¹H NMR (400 MHz, Methanol-*d*₄) δ 8.64 (dd, 1H), 8.39 (s, 1H), 7.78 (d, 1H), 7.72 (d, 1H), 7.58 - 7.49 (m, 2H), 7.48 - 7.44 (m, 1H), 7.43 - 7.38 (m, 1H), 7.37 - 7.31 (m, 2H), 7.26 (d, 1H), 4.08 (s, 2H), 4.00 (t, 3H), 3.96 (d, 3H), 3.70 (s, 2H), 3.55 (dd, 2H), 3.15 (d, 2H), 3.00 (s, 3H), 2.92 - 2.80 (m, 1H), 2.66 - 2.44 (m, 7H), 2.35 (s, 3H), 2.24 - 2.10 (m, 2H).

### Example 149: 1-(3-((((6-(3-(2-(4-((((1-Acetylazetidin-3-yl)methyl)(methyl)amino)methyl)-3-methoxyphenyl)-3-chloropyridin-4-yl)-2-chlorophenyl)-2-methoxypyridin-3-yl)methyl)(methyl)amino)methyl)azetidin-1-yl)ethan-1-one (499)

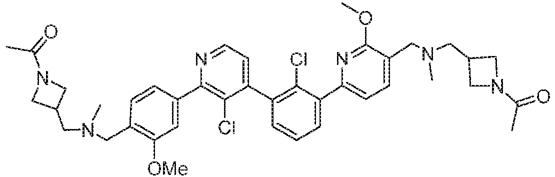

### (a) tert-Butyl 3-((((6-(3-(2-(4-((((1-(tert-butoxycarbonyl)azetidin-3-yl)-methyl)(methyl)amino)-methyl)-3-methoxyphenyl)-3-chloropyridin-4-yl)-2-chlorophenyl)-2-methoxypyridin-3-yl)methyl)(methyl)amino)methyl)azetidine-1-carboxylate

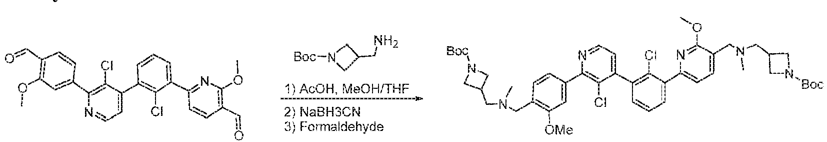

6-(2-Chloro-3-(3-chloro-2-(4-formyl-3-methoxyphenyl)pyridin-4-yl)phenyl)-2-methoxynicotinaldehyde (40 mg, 0.08 mmol), acetic acid (10 mg, 0.16 mmol), and tert-butyl 3-(aminomethyl)azetidine-1-carboxylate (39 mg, 0.21 mmol) were dissolved in MeOH/THF (1:1, 2 mL), then the solution was stirred at 25 °C for 2 hours. Sodium cyanoborohydride (8 mg, 0.13 mmol) was added and the resulting mixture was stirred at 25 °C for 1 hour. Formaldehyde (37 % aqueous solution) (0.03 mL, 0.32 mmol) was then added and the resulting mixture was stirred at room temperature for 1 hour. Reaction was concentrated under reduced pressure and the residue was suspended in 5 mL water, then extracted with dichloromethane (3 x 3 mL). The combined organics were dried over sodium sulfate, filtered, and concentrated under reduced pressure to afford tert-butyl 3-((((6-(3-(2-(4-((((1-(tert-butoxycarbonyl)azetidin-3-yl)methyl)(methyl)amino)methyl)-3-methoxyphenyl)-3-chloropyridin-4-yl)-2-chlorophenyl)-2-methoxypyridin-3-yl)methyl)(methyl)amino)methyl)azetidine-1-carboxylate (70 mg, crude) as a white foam. MS: *m*/*z* found 861, 863 [M+H]⁺. Material was used for the next step without further purification.

### (b) 1-(Azetidin-3-yl)-N-((6-(3-(2-(4-(((azetidin-3-ylmethyl)(methyl)amino)methyl)-3-methoxyphenyl)-3-chloropyridin-4-yl)-2-chlorophenyl)-2-methoxypyridin-3-yl)methyl)-N-methylmethanamine

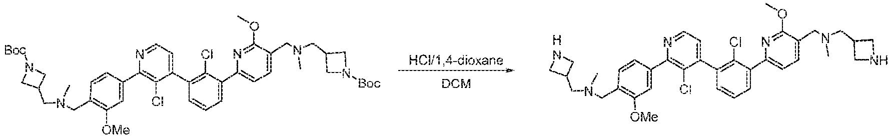

tert-Butyl 3-((((6-(3-(2-(4-((((1-(tert-butoxycarbonyl)azetidin-3-yl)-methyl)(methyl)-amino)methyl)-3-methoxyphenyl)-3-chloropyridin-4-yl)-2-chlorophenyl)-2-methoxypyridin-3-yl)methyl)(methyl)amino)methyl)azetidine-1-carboxylate (70 mg, 0.08 mmol, crude) was dissolved in 1 mL MeOH and 4M HCl solution in 1,4-dioxane (81 µL, 0.32 mmol) was added. Reaction was stirred at room temperature for 60 minutes and solvent was removed under reduced pressure to afford N-(azetidin-3-ylmethyl)-1-[4-[4-[3-[5-[[azetidin-3-ylmethyl(methyl)amino]methyl]-6-methoxy-2-pyridyl]-2-chloro-phenyl]-3-chloro-2-pyridyl]-2-methoxy-phenyl]-N-methyl-methanamine (53 mg, crude) as a yellow oil. MS: *m*/*z* found 661, 663 [M+H]⁺. Material was used for the next step without further purification.

### (c) 1-(3-((((6-(3-(2-(4-((((1-acetylazetidin-3-yl)methyl)(methyl)amino)methyl)-3-methoxyphenyl)-3-chloropyridin-4-yl)-2-chlorophenyl)-2-methoxypyridin-3-yl)methyl)(methyl)amino)methyl)azetidin-1-yl)ethan-1-one

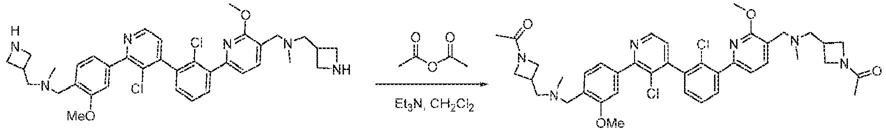

N-(azetidin-3-ylmethyl)-1-[4-[4-[3-[5-[[azetidin-3-ylmethyl(methyl)amino]methyl]-6-methoxy-2-pyridyl]-2-chloro-phenyl]-3-chloro-2-pyridyl]-2-methoxy-phenyl]-N-methylmethanamine (53 mg, 0.08 mmol, crude) and triethylamine (40.mg, 0.40 mmol) were suspended in 2 mL dichloromethane, then the solution was cooled to 0 °C. Acetic anhydride (41 mg, 0.40 mmol) was added dropwise over 2 minutes, then the resulting mixture was warmed to room temperature and stirred for 18 hours. Reaction was diluted with water (7 mL) and the solution was extracted with EtOAc (3 x 5 mL). The combined organics were concentrated under reduced pressure and crude sample was purified by reverse phase HPLC to afford 1-(3-((((6-(3-(2-(4-((((1-acetylazetidin-3-yl)methyl)(methyl)amino)methyl)-3-methoxyphenyl)-3-chloropyridin-4-yl)-2-chlorophenyl)-2-methoxypyridin-3-yl)methyl)(methyl)amino)methyl)azetidin-1-yl)ethan-1-one (17 mg, 28 % yield) as a white powder (formic acid salt). LC/MS: *m*/*z* found 745, 747 [M+H]⁺, retention time = 2.09 min (Method C). ¹H NMR (400 MHz, Methanol-*d*₄) δ 8.65 (d, 1H), 8.36 (s, 1H), 7.80 (d, 1H), 7.72 (d, 1H), 7.54 (dd, 2H), 7.46 (d, 1H), 7.44 - 7.32 (m, 3H), 7.28 (d, 1H), 4.34 (m, 2H), 4.20 - 4.05 (m, 4H), 4.01 (d, 3H), 3.97 (d, 4H), 3.89 (dd, 1H), 3.76 (s, 2H), 3.75 - 3.69 (m, 1H), 3.69 - 3.63 (m, 1H), 3.27 (d, 2H), 3.20 - 3.08 (m, 1H), 3.06 - 2.95 (m, 1H), 2.90 (d, 2H), 2.65 (s, 3H), 2.40 (s, 3H), 1.84 (d, 6H).

### Example 150: 2-(4-(3-chloro-4-(2-chloro-3-(6-methoxy-5-((6-methyl-7-oxo-2,6-diazaspiro[3.4]octan-2-yl)methyl)pyridin-2-yl)phenyl)pyridin-2-yl)-2-methoxybenzyl)-6-methyl-2,6-diazaspiro[3.4]octan-7-one (571)

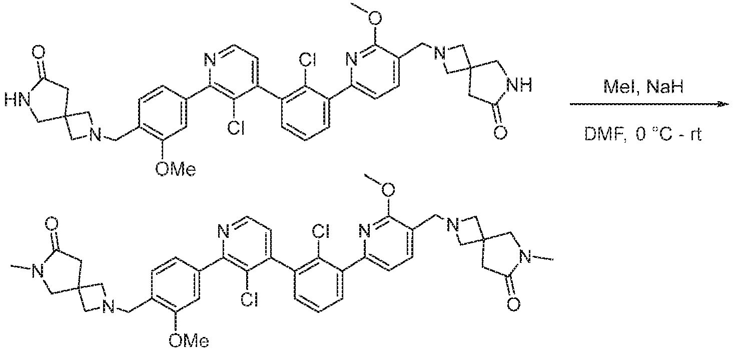

2-[[4-[3-chloro-4-[2-chloro-3-[6-methoxy-5-[(6-oxo-2,7-diazaspiro[3.4]octan-2-yl)methyl]-2-pyridyl]phenyl]-2-pyridyl]-2-methoxy-phenyl]methyl]-2,7-diazaspiro[3.4]octan-6-one (Example 2) (25 mg, 0.04 mmol) was dissolved in 1 mL DMF and the solution was cooled to 0 °C. Sodium hydride (60% dispersion in mineral oil) (15 mg, 0.14 mmol) was added, and the solution was stirred for 30 minutes. Iodomethane (11 mg, 0.08 mmol) was subsequently added and the reaction was stirred at room temperature for 3 hours. Reaction was diluted with 3 mL water and extracted with EtOAc (3 x 2 mL). The combined organics were concentrated under reduced pressure, and crude sample was purified by reverse phase HPLC to afford 2-(4-(3-chloro-4-(2-chloro-3-(6-methoxy-5-((6-methyl-7-oxo-2, 6-diazaspiro [3 .4] octan-2-yl)methyl)pyridin-2-yl)phenyl)pyridin-2-yl)-2-methoxybenzyl)-6-methyl-2,6-diazaspiro[3.4]octan-7-one (6 mg, 23 % yield) as a white powder (formic acid salt). LC/MS: *m*/*z* found 741, 743 [M+H]⁺, retention time = 2.13 min (Method C). ¹H NMR (400 MHz, Methanol-*d*₄) δ 8.65 (d, 1H), 8.40 (s, 2H), 7.76 (d, 1H), 7.70 (d, 1H), 7.55 (t, 1H), 7.48 (dd, 2H), 7.41 (d, 1H), 7.36 (s, 1H), 7.33 (d, 1H), 7.28 (d, 1H), 4.30 (s, 2H), 4.09 - 4.03 (m, 3H), 4.03 - 4.00 (m, 4H), 3.97 (d, 5H), 3.73 (dd, 6H), 3.67 (s, 2H), 2.82 (s, 6H), 2.76 (s, 2H), 2.70 (s, 2H).

### Example 151: N-(3-(((6-(3-(2-(4-(((3-Acetamidopropyl)amino)methyl)-3-methoxyphenyl)-3-chloropyridin-4-yl)-2-chlorophenyl)-2-methoxypyridin-3-yl)methyl)amino)propyl) acetamide (358)

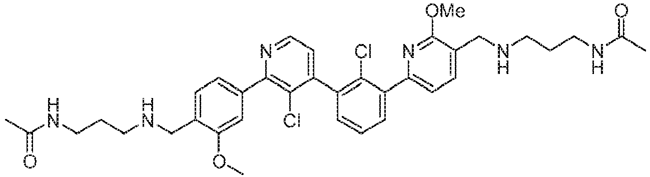

N-(3-(((6-(3-(2-(4-(((3-Acetamidopropyl)amino)methyl)-3-methoxyphenyl)-3-chloropyridin-4-yl)-2-chlorophenyl)-2-methoxypyridin-3-yl)methyl)amino)propyl) acetamide was prepared in a similar fashion to Example 11, replacing (S)-5-(aminomethyl)pyrrolidin-2-one with N-(3-aminopropyl)acetamide in step (b). MS: m/z found 693 [M+H]⁺, retention time = 1.67 min (Method D). ¹H NMR (400 MHz, Methanol-*d*₄) δ 8.62 (d, 1H), 8.49 (s, 2H), 7.81 (d, 1H), 7.68 (dd, 1H), 7.58 - 7.25 (m, 7H), 4.20 (s, 2H), 4.05 (d, 5H), 3.95 (s, 3H), 3.25 - 3.20 (m, 3H), 2.96 (m, 4H), 1.92 (d, 8H), 1.88 - 1.77 (m, 3H).

### Example 152: 4-(((6-(2-Chloro-3-(3-chloro-2-(4-(((2,6-dioxopiperidin-4-yl)amino)methyl)-3-methoxyphenyl)pyridin-4-yl)phenyl)-2-methoxypyridin-3-yl)methyl)amino)piperidine-2,6-dione (359)

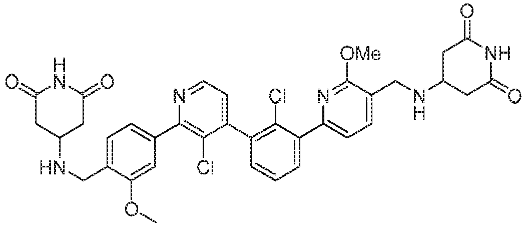

4-(((6-(2-Chloro-3-(3-chloro-2-(4-(((2,6-dioxopiperidin-4-yl)amino)methyl)-3-methoxyphenyl)pyridin-4-yl)phenyl)-2-methoxypyridin-3-yl)methyl)amino)piperidine-2,6-dione was prepared in a similar fashion to Example 11, replacing (S)-5-(aminomethyl)pyrrolidin-2-one with 4-aminopiperidine-2,6-dione in step (b). MS: m/z found 717 [M+H]⁺, retention time = 1.60 min (Method D). ¹H NMR (400 MHz, Methanol-*d*₄) δ 8.58 (d, 1H), 8.45 (s, 1H), 7.75 - 7.63 (m, 2H), 7.50 (t, 1H), 7.43 - 7.35 (m, 3H), 7.22 (d, 3H), 3.97 (s, 3H), 3.87 (d, 5H), 3.80 (s, 2H), 2.79 (dd, 5H), 2.58 (dd, 5H).

### Example 153: 5-(((6-(3-(2-(4-(((5-Amino-5-oxopentyl)amino)methyl)-3-methoxyphenyl)-3-chloropyridin-4-yl)-2-chlorophenyl)-2-methoxypyridin-3-yl)methyl)amino)pentanamide (373)

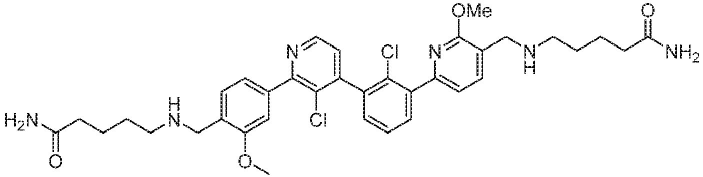

5-(((6-(3-(2-(4-(((5-Amino-5-oxopentyl)amino)methyl)-3-methoxyphenyl)-3-chloropyridin-4-yl)-2-chlorophenyl)-2-methoxypyridin-3-yl)methyl)amino)pentanamide was prepared in a similar fashion to Example 11, replacing (S)-5-(aminomethyl)pyrrolidin-2-one with 5-aminopentanamide in step (b). MS: m/z found 693 [M+H]⁺, retention time = 1.64 min (Method D). ¹H NMR (400 MHz, Methanol-*d*₄) δ 8.62 (d, 1H), 8.50 (s, 2H), 7.77 (d, 1H), 7.68 (dd, 1H), 7.57 - 7.23 (m, 7H), 4.17 (s, 3H), 4.01 (d, 5H), 3.94 (s, 3H), 2.97 (t, 2H), 2.86 (s, 3H), 2.29 - 2.19 (m, 4H), 1.66 (q, 4H), 1.25 (s, 2H).

### Example 154: 1-((R)-3-(((6-(3-(2-(4-((((R)-1-Acetylpyrrolidin-3-yl)amino)methyl)-3-methoxyphenyl)-3-chloropyridin-4-yl)-2-chlorophenyl)-2-methoxypyridin-3-yl)methyl) amino)pyrrolidin-1-yl)ethan-1-one (374)

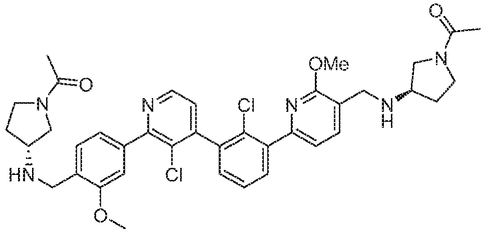

1-((R)-3-(((6-(3-(2-(4-((((R)-1-Acetylpyrrolidin-3-yl)amino)methyl)-3-methoxyphenyl)-3-chloropyridin-4-yl)-2-chlorophenyl)-2-methoxypyridin-3-yl)methyl) amino)pyrrolidin-1-yl)ethan-1-one was prepared in a similar fashion to Example 11, replacing (S)-5-(aminomethyl)pyrrolidin-2-one with (R)-1-(3-aminopyrrolidin-1-yl)ethan-1-one in step (b). MS: m/z found 717 [M+H]⁺, retention time = 1.64 min (Method D). ¹H NMR (400 MHz, Methanol-*d*₄) δ 8.60 (d, 1H), 8.49 (s, 2H), 7.73 (dd, 1H), 7.67 (dd, 1H), 7.51 (t, 1H), 7.45 - 7.34 (m, 3H), 7.30 - 7.19 (m, 3H), 4.02 - 3.88 (m, 8H), 3.83 (s, 2H), 3.77 - 3.56 (m, 4H), 3.59 - 3.48 (m, 4H), 3.45 - 3.32 (m, 2H), 2.30 - 2.08 (m, 2H), 2.05 - 1.90 (m, 7H), 1.91 - 1.79 (m, 1H).

### Example 155: 4-((6-(2-Chloro-3-(3-chloro-2-(3-methoxy-4-(morpholinomethyl)phenyl) pyridin-4-yl)phenyl)-2-methoxypyridin-3-yl)methyl)morpholine (411)

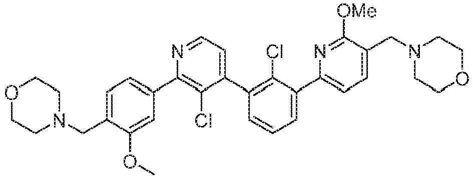

4-((6-(2-Chloro-3-(3-chloro-2-(3-methoxy-4-(morpholinomethyl)phenyl) pyridin-4-yl)phenyl)-2-methoxypyridin-3-yl)methyl)morpholine was prepared in a similar fashion to Example 11, replacing (S)-5-(aminomethyl)pyrrolidin-2-one with morpholine in step (b). MS: m/z found 635 [M+H]⁺, retention time = 2.07 min (Method C). ¹H NMR (400 MHz, Methanol-*d*₄) δ 8.60 (d, 1H), 8.35 (s, 1H), 7.77 (d, 1H), 7.67 (dd, 1H), 7.55 - 7.44 (m, 2H), 7.43 - 7.20 (m, 5H), 4.03 (s, 2H), 3.96 (s, 3H), 3.90 (s, 3H), 3.77 (t, 4H), 3.74 - 3.67 (m, 6H), 2.94 (t, 4H), 2.67 (t, 4H).

### Example 156: 1-((6-(2-Chloro-3-(3-chloro-2-(4-((4-hydroxypiperidin-1-yl)methyl)-3-methoxyphenyl)pyridin-4-yl)phenyl)-2-methoxypyridin-3-yl)methyl)piperidin-4-ol (412)

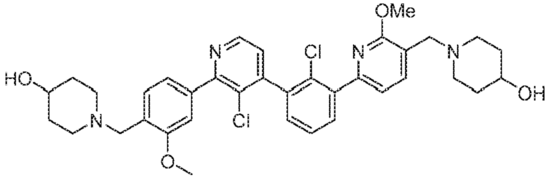

1-((6-(2-Chloro-3-(3-chloro-2-(4-((4-hydroxypiperidin-1-yl)methyl)-3-methoxyphenyl)pyridin-4-yl)phenyl)-2-methoxypyridin-3-yl)methyl)piperidin-4-ol was prepared in a similar fashion to Example 11 replacing (S)-5-(aminomethyl)pyrrolidin-2-one with piperidin-4-ol in step (b). MS: m/z found 663 [M+H]⁺, retention time = 2.05 min (Method C). ¹H NMR (400 MHz, Methanol-*d*₄) δ 8.63 (d, 1H), 8.46 (s, 2H), 7.86 (d, 1H), 7.70 (dd, 1H), 7.54 (t, 2H), 7.47 - 7.28 (m, 5H), 4.30 (s, 2H), 4.11 (s, 2H), 4.02 (s, 3H), 3.97 - 3.89 (m, 4H), 3.84 (s, 1H), 3.38 (s, 2H), 3.27 (m, 2H), 3.15 (s, 2H), 2.95 (s, 2H), 2.03 - 1.92 (m, 4H), 1.79 - 1.74 (m, 4H).

### Example 157: 3-Chloro-4-(2-chloro-3-(6-methoxy-5-((4-methoxypiperidin-1-yl)methyl) pyridin-2-yl)phenyl)-2-(3-methoxy-4-((4-methoxypiperidin-1-yl)methyl)phenyl)pyridine (447)

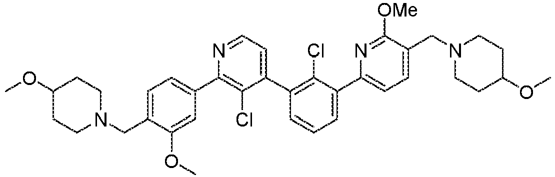

3-Chloro-4-(2-chloro-3-(6-methoxy-5-((4-methoxypiperidin-1-yl)methyl) pyridin-2-yl)phenyl)-2-(3-methoxy-4-((4-methoxypiperidin-1-yl)methyl)phenyl)pyridine was prepared in a similar fashion to Example 11, replacing (S)-5-(aminomethyl)pyrrolidin-2-one with 4-methoxypiperidine in step (b). MS: m/z found 691 [M+H]⁺, retention time = 1.83 min (Method D). ¹H NMR (400 MHz, Methanol-*d*₄) δ 8.61 (d, 8.52 (s, 1H), 7.78 - 7.66 (m, 2H), 7.52 (t, 1H), 7.48 - 7.35 (m, 3H), 7.32 - 7.21 (m, 3H), 3.97 (s, 3H), 3.87 (d, 3.62 (s, 2H), 3.34 - 3.29 (m, 6H), 2.96 (d, 2H), 2.84 (d, 2H), 2.58 (s, 2H), 2.38 (t, 2H), 1.93 (s, 4H), 1.73 - 1.57 (m, 5H).

### Example 158: (1s,4s)-N-((6-(2-Chloro-3-(3-chloro-2-(3-methoxy-4-((((1s,4s)-4-methoxycyclohexyl)amino)methyl)phenyl)pyridin-4-yl)phenyl)-2-methoxypyridin-3-yl)methyl)-4-methoxycyclohexan-1-amine (448)

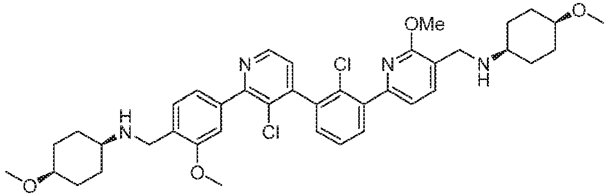

(1s,4s)-N-((6-(2-Chloro-3-(3-chloro-2-(3-methoxy-4-((((1s,4s)-4-methoxycyclohexyl)amino)methyl)phenyl)pyridin-4-yl)phenyl)-2-methoxypyridin-3-yl)methyl)-4-methoxycyclohexan-1-amine was prepared in a similar fashion to Example 11, replacing (S)-5-(aminomethyl)pyrrolidin-2-one with (1s,4s)-4-methoxycyclohexan-1-amine in step (b). MS: m/z found 719 [M+H]⁺, retention time = 2.00 min (Method D). ¹H NMR (400 MHz, Methanol-*d*₄) δ 8.65 (dd, 1H), 8.49 (d, 1H), 7.82 (d, 1H), 7.68 (d, 1H), 7.53 (t, 1H), 7.51 - 7.41 (m, 2H), 7.43 - 7.38 (m, 1H), 7.34 - 7.25 (m, 3H), 4.22 (s, 3H), 4.13 (s, 2H), 4.03 (d, 3.94 (d, 4H), 3.45 (q, 3H), 3.33 - 3.23 (m, 4H), 3.18 - 3.00 (m, 1H), 2.05 (d, 5H), 1.89 (m, 5H), 1.70 (m, 5H), 1.56 - 1.43 (m, 5H), 0.69 (s, 1H).

### Example 159: (1r,4r)-N-((6-(2-Chloro-3-(3-chloro-2-(3-methoxy-4-((((1r,4r)-4-methoxycyclohexyl)amino)methyl)phenyl)pyridin-4-yl)phenyl)-2-methoxypyridin-3-yl)methyl)-4-methoxycyclohexan-1-amine (450)

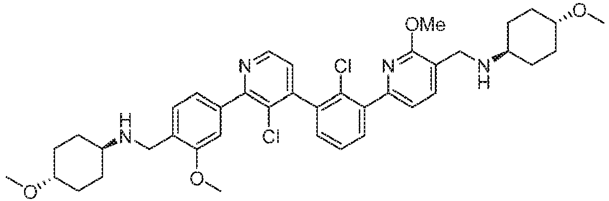

(1r,4r)-N-((6-(2-Chloro-3-(3-chloro-2-(3-methoxy-4-((((1r,4r)-4-methoxycyclohexyl)amino)methyl)phenyl)pyridin-4-yl)phenyl)-2-methoxypyridin-3-yl)methyl)-4-methoxycyclohexan-1-amine was prepared in a similar fashion to Example 11, replacing (S)-5-(aminomethyl)pyrrolidin-2-one with (1r,4r)-4-methoxycyclohexan-1-amine in step (b). MS: m/z found 719 [M+H] ⁺, retention time = 1.87 min (Method D). ¹H NMR (400 MHz, Methanol-*d*₄) δ 8.60 (d, 1H), 7.75 - 7.64 (m, 2H), 7.52 (t, 1H), 7.45 - 7.34 (m, 3H), 7.31 - 7.19 (m, 3H), 3.98 (d, 5H), 3.92 (s, 3H), 3.84 (s, 2H), 3.33 - 3.28 (m, 9H), 3.22 - 3.12 (m, 1H), 2.68 (s, 1H), 2.53 (s, 1H), 2.11 - 2.00 (m, 9H), 1.36 - 1.11 (m, 6H).

### Example 160: 4-((6-(2-Chloro-3-(3-chloro-2-(3-methoxy-4-((3-oxopiperazin-1-yl)methyl)phenyl)pyridin-4-yl)phenyl)-2-methoxypyridin-3-yl)methyl)piperazin-2-one (457)

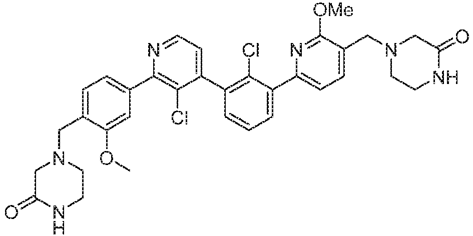

4-((6-(2-Chloro-3-(3-chloro-2-(3-methoxy-4-((3-oxopiperazin-1-yl)methyl)phenyl)pyridin-4-yl)phenyl)-2-methoxypyridin-3-yl)methyl)piperazin-2-one was prepared in a similar fashion to Example 11, replacing (S)-5-(aminomethyl)pyrrolidin-2-one with piperazin-2-one in step (b). MS: m/z found 661 [M+H]⁺, retention time = 1.51 min (Method D). ¹H NMR (400 MHz, Methanol-*d*₄) δ 8.59 (d, 1H), 8.15 (s, 1H), 7.77 (d, 1H), 7.69 (d, 1H), 7.51 (t, 1H), 7.48 - 7.34 (m, 3H), 7.29 - 7.21 (m, 3H), 3.97 (s, 2H), 3.88 (d, 3H), 3.75 (s, 2H), 3.67 (s, 2H), 3.34 - 3.29 (m, 4H), 3.35 - 3.25 (m, 2H), 3.16 (d, 3H), 2.80 - 2.69 (m, 4H).

### Example 161: 2-(2-((6-(2-Chloro-3-(3-chloro-2-(4-((6-(2-hydroxyethyl)-2,6-diazaspiro [3.4] octan-2-yl)methyl)-3-methoxyphenyl)pyridin-4-yl)phenyl)-2-methoxypyridin-3-yl)methyl)-2,6-diazaspiro[3.4]octan-6-yl)ethan-1-ol (670)

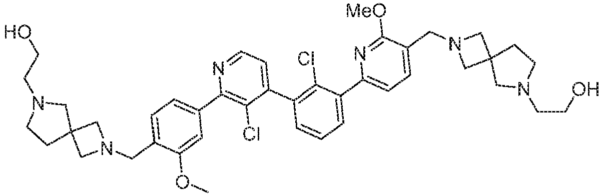

### (a) tert-Butyl 2-((6-(3-(2-(4-((6-(tert-butoxycarbonyl)-2,6-diazaspiro[3.4]octan-2-yl)methyl)-3-methoxyphenyl)-3-chloropyridin-4-yl)-2-chlorophenyl)-2-methoxypyridin-3-yl)methyl)-2,6-diazaspiro [3.4] octane-6-carboxylate

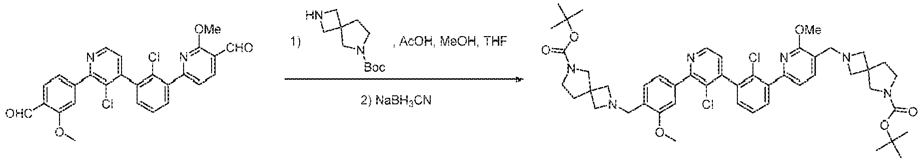

tert-Butyl 2-((6-(3-(2-(4-((6-(tert-butoxycarbonyl)-2,6-diazaspiro[3.4]octan-2-yl)methyl)-3-methoxyphenyl)-3-chloropyridin-4-yl)-2-chlorophenyl)-2-methoxypyridin-3-yl)methyl)-2,6-diazaspiro[3.4]octane-6-carboxylate was prepared in a similar fashion to Example 11, replacing (S)-5-(aminomethyl)pyrrolidin-2-one with tert-butyl 2,6-diazaspiro[3.4]octane-6-carboxylate in step (b). MS: m/z found 885 [M+H]⁺.

### (b) 2-(2-((6-(2-Chloro-3-(3-chloro-2-(4-((6-(2-hydroxyethyl)-2,6-diazaspiro [3.4]octan-2-yl)methyl)-3-methoxyphenyl)pyridin-4-yl)phenyl)-2-methoxypyridin-3-yl)methyl)-2,6-diazaspiro[3.4]octan-6-yl)ethan-1-ol

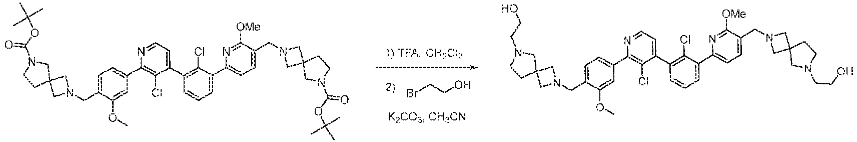

A vial was charged with tert-butyl 2-((6-(3-(2-(4-((6-(tert-butoxycarbonyl)-2,6-diazaspiro[3.4]octan-2-yl)methyl)-3-methoxyphenyl)-3-chloropyridin-4-yl)-2-chlorophenyl)-2-methoxypyridin-3-yl)methyl)-2,6-diazaspiro[3.4]octane-6-carboxylate (0.05 g, 0.06 mmol), then dichloromethane (3 ml) added followed by trifluoroacetic acid (1 ml) and the reaction was stirred at room temperature for 30 min, then evaporated under reduced pressure, and the residue dried on high vacuum pump for 2 hr, To the resulting TFA salt was added potassium carbonate (0.04 g 0.30 mmol), 2-bromoethanol (0.03 g, 0.25 mmol) and acetonitrile (3 mL), and the mixture was stirred at 90 °C for 1 hr. The mixture was filtered through CELITE^{®} and the filter cake was washed with ethyl acetate (3 x 15 mL). The combined filtrates were concentrated under reduced pressure and the residue purified by reverse phase HPLC (with 0.05% Formic acid modifier) to give 2-(2-((6-(2-chloro-3-(3-chloro-2-(4-((6-(2-hydroxyethyl)-2,6-diazaspiro [3.4]octan-2-yl)methyl)-3-methoxyphenyl)pyridin-4-yl)phenyl)-2-methoxypyridin-3-yl)methyl)-2,6-diazaspiro[3.4]octan-6-yl)ethan-1-ol (6.8 mg, 12% yield) as a white solid (formic acid salt). MS: m/z found 773 [M+H]⁺. ¹H NMR (400 MHz, Methanol-*d*₄) δ 8.60 (d, 1H), 8.34 (s, 2H), 7.73 (d, 1H), 7.66 (dd, 1H), 7.55 - 7.45 (m, 1H), 7.48 - 7.19 (m, 6H), 4.24 (s, 2H), 4.13 - 3.83 (m, 11H), 3.72 - 3.82 (m, 11H), 3.47 (d, 4H), 3.31 - 3.03 (m, 6H), 2.33 (t, 3H), 1.32 - 1.21 (m, 1H).

### Example 162: 1-(2-((6-(3-(2-(4-((6-Acetyl-2,6-diazaspiro[3.4]octan-2-yl)methyl)-3-methoxyphenyl)-3-chloropyridin-4-yl)-2-chlorophenyl)-2-methoxypyridin-3-yl)methyl)-2,6-diazaspiro[3.4]octan-6-yl)ethan-1-one (671)

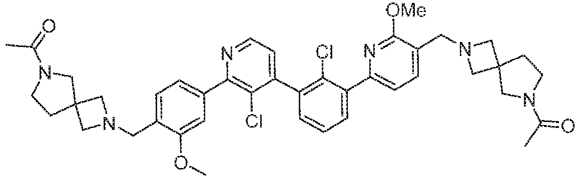

### (a) 1-(2-((6-(3-(2-(4-((6-Acetyl-2,6-diazaspiro[3.4]octan-2-yl)methyl)-3-methoxyphenyl)-3-chloropyridin-4-yl)-2-chlorophenyl)-2-methoxypyridin-3-yl)methyl)-2,6-diazaspiro[3.4]octan-6-yl)ethan-1-one

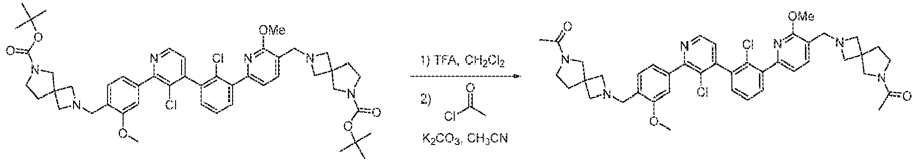

1-(2-((6-(3-(2-(4-((6-Acetyl-2,6-diazaspiro[3.4]octan-2-yl)methyl)-3-methoxyphenyl)-3-chloropyridin-4-yl)-2-chlorophenyl)-2-methoxypyridin-3-yl)methyl)-2,6-diazaspiro[3.4]octan-6-yl)ethan-1-one was prepared in a similar manner as described for Example 161, replacing 2-bromoethanol with acetyl chloride in step (b). MS: m/z found 769 [M+H]⁺. ¹H NMR (400 MHz, Methanol-*d*₄) δ 8.62 (d, 8.34 (s, 2H), 7.84 - 7.76 (m, 1H), 7.67 (dd, 1H), 7.57 - 7.46 (m, 2H), 7.43 - 7.25 (m, 5H), 4.41 (d, 2H), 4.25 - 4.09 (m, 4H), 4.04 - 3.84 (m, 9H), 3.72 (dd, 2H), 3.63 (d, 3H), 3.53 (t 3H), 3.41 (t, 2H), 2.33 - 2.11 (m, 5H), 2.01 (d, 6H).

### Example 163: 1-(4-(((6-(2-Chloro-3-(3-chloro-2-(3-methoxy-4-(((1-propionylpiperidin-4-yl)amino)methyl)phenyl)pyridin-4-yl)phenyl)-2-methoxypyridin-3-yl)methyl)amino)piperidin-1-yl)propan-1-one (344)

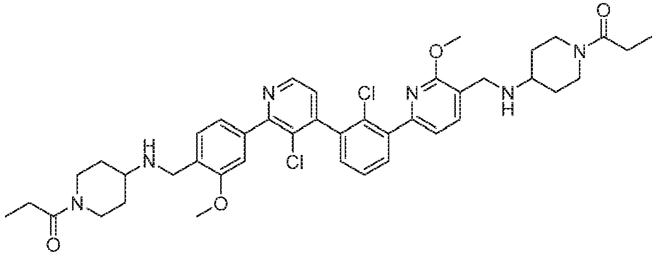

### (a) 1-(4-Aminopiperidin-1-yl)propan-1-one

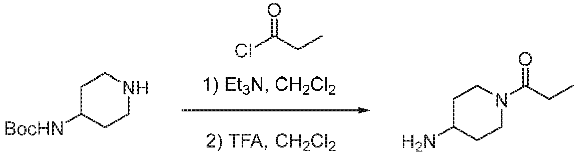

A solution of tert-butyl N-(4-piperidyl)carbamate (0.80 g, 3.99 mmol) in 32 mL DCM was cooled in an ice bath. Triethylamine (1.11 mL, 7.99 mmol) was added. A solution of propanoyl chloride (0.38 mL, 4.39 mmol) in 8 mL DCM was added slowly. After 15 minutes, the reaction mixture was diluted with 30 mL DCM and washed with saturated sodium bicarbonate solution (10 mL), 0.3 M HCl (10 mL), and saturated sodium bicarbonate solution (10 mL). The organics were dried over sodium sulfate and concentrated under reduced pressure to provide crude tert-butyl (1-propionylpiperidin-4-yl)carbamate (1.01 g, 99%) as a white solid. ¹H NMR (400 MHz, Chloroform-d) δ 4.44 (s, 1H), 3.66 (s, 1H), 2.91 (s, 3H), 2.35 (q, 2H), 1.98 (d, 2H), 1.44 (s, 9H), 1.35 - 1.23 (m, 2H), 1.27 - 1.10 (m, 3H).

Trifluoroacetic acid (7.84 mL, 102.40 mmol) was added to a solution of tert-butyl (1-propionylpiperidin-4-yl)carbamate (0.75 g, 2.93 mmol) in 10 mL DCM. After 3 h, the volatiles were removed *in vacuo.* To the resulting oil was added saturated sodium bicarbonate (15 mL). The mixture was extracted with 7 x 15 mL of 10% MeOH in DCM. The combined organics were evaporated to provide 1-(4-aminopiperidin-1-yl)propan-1-one; (0.12 g, 26%). MS: m/z found 157.1 [M+H]⁺.

### (b) 1-(4-(((6-(2-Chloro-3-(3-chloro-2-(3-methoxy-4-(((1-propionylpiperidin-4-yl)amino)methyl)phenyl)pyridin-4-yl)phenyl)-2-methoxypyridin-3-yl)methyl)amino)piperidin-1-yl)propan-1-one

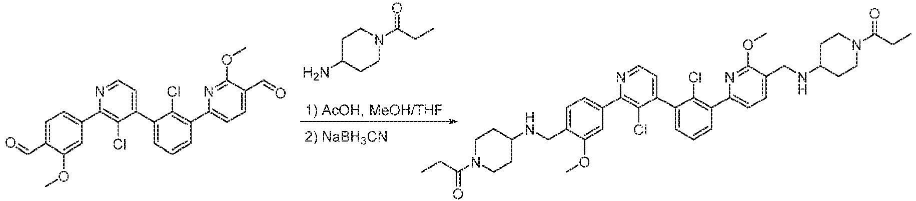

1-(4-(((6-(2-Chloro-3-(3-chloro-2-(3-methoxy-4-(((1-propionylpiperidin-4-yl)amino)methyl)phenyl)pyridin-4-yl)phenyl)-2-methoxypyridin-3-yl)methyl)amino)piperidin-1-yl)propan-1-one (37% yield ) was prepared as the formic acid salt, following Reductive Amination Procedure A from 6-(2-chloro-3-(3-chloro-2-(4-formyl-3-methoxyphenyl)pyridin-4-yl)phenyl)-2-methoxynicotinaldehyde (25 mg, 0.05 mmol) and 1-(4-amino-1-piperidyl)propan-1-one (32 mg, 0.20 mmol). LCMS: m/z found 773.2 [M+H]⁺, retention time = 3.03 min, (Method A); ¹H NMR (400 MHz, Methanol-*d*₄): δ 8.63 (m, 1H), 7.79 - 7.67 (m, 2H), 7.55 (m, 1H), 7.47 - 7.37 (m, 3H), 7.32 - 7.22 (m, 4H), 4.52 (t, 2H), 3.96 (d, 7H), 3.88 (s, 2H), 3.18 - 3.04 (m, 3H), 2.97 - 2.77 (m, 2H), 2.69 (q, 2H), 2.45 - 2.37 (m, 5H), 2.04 (s, 6H), 1.53 - 1.23 (m, 4H), 1.11 (t, 6H).

### Example 164: (4-(((6-(2-Chloro-3-(3-chloro-2-(4-(((1-(cyclopropanecarbonyl)piperidin-4-yl)amino)methyl)-3-methoxyphenyl)pyridin-4-yl)phenyl)-2-methoxypyridin-3-yl)methyl)amino)piperidin-1-yl)(cyclopropyl)methanone (345)

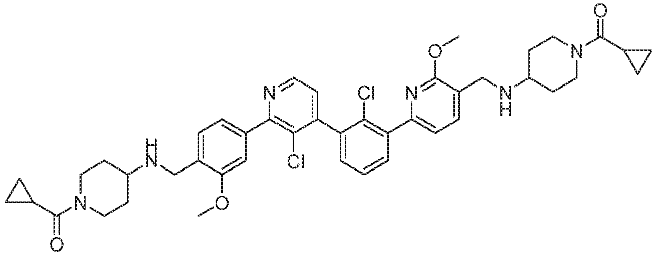

### (a) (4-Aminopiperidin-1-yl)(cyclopropyl)methanone

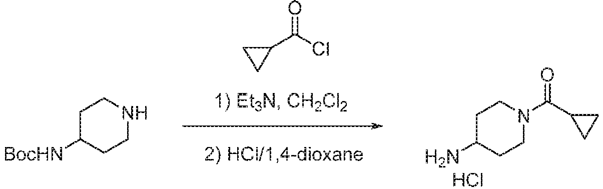

A solution of tert-butyl N-(4-piperidyl)carbamate (0.20 g, 1.00 mmol) in 8 mL DCM was cooled in an ice bath. Triethylamine (0.28 mL, 2.00 mmol) was added. A solution of cyclopropanecarbonyl chloride (0.10 mL, 1.05 mmol) in 3 mL DCM was added slowly. After 5 minutes, the rxn mixture was diluted with 30 mL DCM and washed with saturated sodium bicarbonate solution (10 mL), 0.3 M HCl (10 mL), and saturated sodium bicarbonate solution (10 mL). The organics were dried over sodium sulfate and concentrated under reduced pressure to provide crude tert-butyl (1-(cyclopropanecarbonyl)piperidin-4-yl)carbamate (0.26 g, 97%). MS: m/z found 269.1 [M+H]⁺.

Hydrogen chloride, 4 M in 1,4-dioxane (2.42 mL, 9.69 mmol) was added to tert-butyl (1-(cyclopropanecarbonyl)piperidin-4-yl)carbamate (0.26 g, 0.97 mmol). After stirring at room temperature for 6 h, the volatiles were removed *in vacuo.* The crude material was triturated with 2 x 5 mL anhydrous diethyl ether, and dried under vacuum to provide (4-Aminopiperidin-1-yl)(cyclopropyl)methanone hydrochloride (0.20 g, 100%). MS: m/z found 169.1 [M+H]⁺.

### (b) (4-(((6-(2-Chloro-3-(3-chloro-2-(4-(((1-(cyclopropanecarbonyl)piperidin-4-yl)amino)methyl)-3-methoxyphenyl)pyridin-4-yl)phenyl)-2-methoxypyridin-3-yl)methyl)amino)piperidin-1-yl)(cyclopropyl)methanone

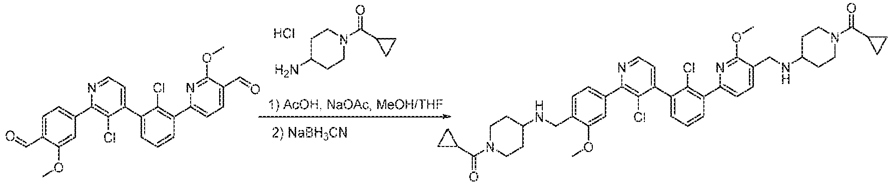

(4-(((6-(2-Chloro-3-(3-chloro-2-(4-(((1-(cyclopropanecarbonyl)piperidin-4-yl)amino)methyl)-3-methoxyphenyl)pyridin-4-yl)phenyl)-2-methoxypyridin-3-yl)methyl)amino)piperidin-1-yl)(cyclopropyl)methanone (77% yield) was prepared as the formic acid salt, following Reductive Amination Procedure A from 6-(2-chloro-3-(3-chloro-2-(4-formyl-3-methoxyphenyl)pyridin-4-yl)phenyl)-2-methoxynicotinaldehyde (30 mg, 0.06 mmol) and (4-aminopiperidin-1-yl)(cyclopropyl)methanone hydrochloride (34 mg, 0.24 mmol). LCMS: m/z found 797.3 [M+H]⁺, retention time = 3.20 min (Method A). ¹H NMR (400 MHz, Methanol-*d*₄): δ 8.65 (d, 1H), 7.79 (d, 1H), 7.72 (d, 1H), 7.60 - 7.38 (m, 4H), 7.38 - 7.25 (m, 3H), 4.67 - 4.35 (m, 4H), 4.17 (s, 2H), 4.04 (s, 3H), 3.97 (s, 5H), 3.28 - 3.18 (m, 3H), 2.98 (s, 1H), 2.74 (s, 2H), 2.25 - 1.96 (m, 6H), 1.59 - 1.29 (m, 4H), 0.85 (d, 8H).

### Example 165: (4-(((6-(2-Chloro-3-(3-chloro-2-(4-(((1-(cyclobutanecarbonyl)piperidin-4-yl)amino)methyl)-3-methoxyphenyl)pyridin-4-yl)phenyl)-2-methoxypyridin-3-yl)methyl)amino)piperidin-1-yl)(cyclobutyl)methanone (352)

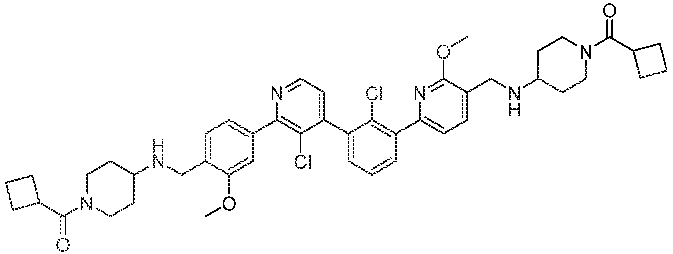

### (a) (4-Aminopiperidin-1-yl)(cyclobutyl)methanone

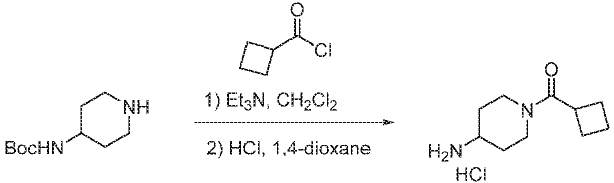

A solution of tert-butyl N-(4-piperidyl)carbamate (0.80 g, 3.99 mmol) in 32 mL DCM was cooled in an ice bath. Triethylamine (1.11 mL, 7.99 mmol) was added. A solution of cyclobutanecarbonyl chloride (0.50 mL, 4.39 mmol) in 8 mL DCM was added slowly. After 5 minutes, the reaction mixture was diluted with 30 mL DCM and washed with saturated sodium bicarbonate solution (10 mL), 0.3 M HCl (10 mL), and saturated sodium bicarbonate solution (10 mL). The organics were dried over sodium sulfate and concentrated under reduced pressure to provide crude tert-butyl (1-(cyclobutanecarbonyl)piperidin-4-yl)carbamate (1.06 g, 94.0 % yield). ¹H NMR (400 MHz, Chloroform-d) δ 4.47 (d, 2H), 3.65 (d, 2H), 3.30 - 3.17 (m, 1H), 3.00 (t, 1H), 2.72 (t, 1H), 2.40 - 2.28 (m, 2H), 2.32 - 2.19 (m, 1H), 2.19 - 2.06 (m, 2H), 2.06 - 1.93 (m, 1H), 1.97 - 1.78 (m, 2H), 1.44 (s, 9H), 1.23 (s, 2H).

Hydrogen chloride, 4 M in 1,4-dioxane (2.42 mL, 9.69 mmol) was added to tert-butyl (1-(cyclobutanecarbonyl)piperidin-4-yl)carbamate (0.26 g, 0.97 mmol). After stirring at room temperature for 6 h, the volatiles were removed *in vacuo.* The crude material was triturated with 2 x 5 mL anhydrous diethyl ether, and dried under vacuum to provide (4-aminopiperidin-1-yl)(cyclobutyl)methanone hydrochloride (0.20 g, 94% yield). MS: m/z found 183.1 [M+H]⁺.

### (b) (4-(((6-(2-Chloro-3-(3-chloro-2-(4-(((1-(cyclobutanecarbonyl)piperidin-4-yl)amino)methyl)-3-methoxyphenyl)pyridin-4-yl)phenyl)-2-methoxypyridin-3-yl)methyl)amino)piperidin-1-yl)(cyclobutyl)methanone

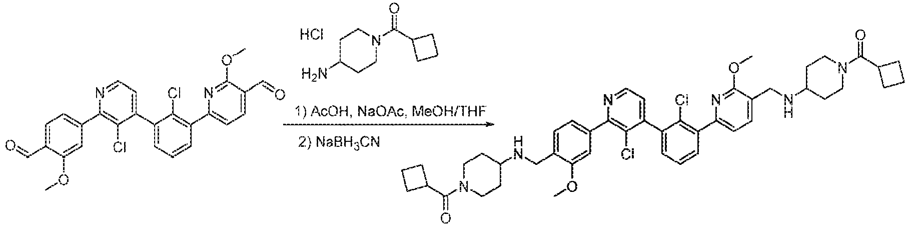

(4-(((6-(2-Chloro-3-(3-chloro-2-(4-(((1-(cyclobutanecarbonyl)piperidin-4-yl)amino)methyl)-3-methoxyphenyl)pyridin-4-yl)phenyl)-2-methoxypyridin-3-yl)methyl)amino)piperidin-1-yl)(cyclobutyl)methanone (37% yield) was prepared as the formic acid salt, following Reductive Amination Procedure A from 6-(2-chloro-3-(3-chloro-2-(4-formyl-3-methoxyphenyl)pyridin-4-yl)phenyl)-2-methoxynicotinaldehyde (30 mg, 0.06 mmol) and (4-aminopiperidin-1-yl)(cyclobutyl)methanone hydrochloride (34 mg, 0.24 mmol). LCMS: m/z found 824.6 [M+H]⁺, retention time = 3.51 min, (Method A). ¹H NMR (400 MHz, Methanol-*d*₄): δ 8.64 (d, 1H), 8.54 (s, 1H), 7.80 - 7.68 (m, 2H), 7.55 (t, 1H), 7.50 - 7.38 (m, 3H), 7.36 - 7.23 (m, 3H), 4.59 - 4.45 (m, 2H), 4.10 (s, 2H), 4.03 (s, 3H), 3.97 - 3.75 (m, 9H), 3.49 - 3.36 (m, 2H), 3.15 - 2.99 (m, 4H), 2.96 - 2.76 (m, 1H), 2.74 - 2.63 (m, 3H), 2.41 - 1.95 (m, 9H), 1.83 (d, 3H), 1.47 - 1.24 (m, 4H).

### Example 166: N-((6-(2-Chloro-3-(3-chloro-2-(3-methoxy-4-(((1-(methylsulfonyl)piperidin-4-yl)amino)methyl)phenyl)pyridin-4-yl)phenyl)-2-methoxypyridin-3-yl)methyl)-1-(methylsulfonyl)piperidin-4-amine (372)

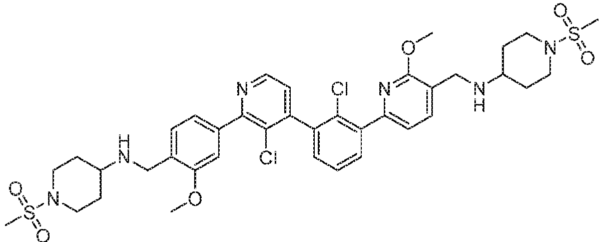

### (a) 1-(Methylsulfonyl)piperidin-4-amine

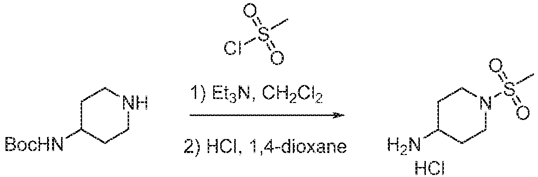

A solution of tert-butyl N-(4-piperidyl)carbamate (0.80 g, 3.99 mmol) in 32 mL DCM was cooled in an ice bath. Triethylamine (1.11 mL, 7.99 mmol) was added. A solution of methanesulfonyl chloride (0.34 mL, 4.39 mmol) in 8 mL DCM was added slowly. After 3h, the reaction mixture was diluted with 10 mL DCM and washed with 0.3 M HCl (10 mL), and saturated sodium bicarbonate solution (10 mL). The organics were dried over sodium sulfate and concentrated under reduced pressure to provide crude tert-butyl (1-(methylsulfonyl)piperidin-4-yl)carbamate (1.04 g, 94% yield) as a white solid. ¹H NMR (400 MHz, Chloroform-*d*) δ 3.75 (d, 2H), 3.57 (s, 1H), 2.78 (s, 3H), 2.84 - 2.73 (m, 2H), 2.05 (dd, 2H), 1.57 - 1.44 (m, 2H), 1.44 (s, 9H).

Hydrogen chloride, 4M in 1,4-dioxane (3.59 mL, 14.37 mmol) was added to tert-butyl (1-(methylsulfonyl)piperidin-4-yl)carbamate (0.40 g, 1.44 mmol). After stirring at room temperature for 6 h, the volatiles were removed *in vacuo.* The crude material was triturated with 2 x 5 mL anhydrous diethyl ether, and dried under vacuum to provide 1-(methylsulfonyl)piperidin-4-amine hydrochloride (0.30 g, 97% yield). MS: m/z found 179.1 [M+H]⁺.

### (b) N-((6-(2-Chloro-3-(3-chloro-2-(3-methoxy-4-(((1-(methylsulfonyl)piperidin-4-yl)amino)methyl)phenyl)pyridin-4-yl)phenyl)-2-methoxypyridin-3-yl)methyl)-1-(methylsulfonyl)piperidin-4-amine

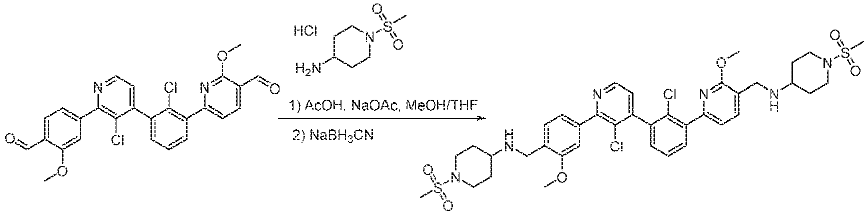

N-((6-(2-Chloro-3-(3-chloro-2-(3-methoxy-4-(((1-(methylsulfonyl)piperidin-4-yl)amino)methyl)phenyl)pyridin-4-yl)phenyl)-2-methoxypyridin-3-yl)methyl)-1-(methylsulfonyl)piperidin-4-amine (56% yield ) was prepared as the formic acid salt, following Reductive Amination Procedure A from 6-(2-chloro-3-(3-chloro-2-(4-formyl-3-methoxyphenyl)pyridin-4-yl)phenyl)-2-methoxynicotinaldehyde (23 mg, 0.05 mmol) and 1-(methylsulfonyl)piperidin-4-amine hydrochloride (35 mg, 0.17 mmol). LCMS: m/z found 817.1 [M+H]⁺, retention time = 3.05 min, (Method A). ¹H NMR (400 MHz, Methanol-*d*₄): δ 8.63 (d, 1H), 7.80 - 7.67 (m, 2H), 7.55 (t, 1H), 7.48 - 7.38 (m, 3H), 7.35 - 7.23 (m, 3H), 4.01 (m, 4H), 3.95 (s, 3H), 3.88 (s, 2H), 3.73 (m, 5H), 2.87 - 2.66 (m, 12H), 2.23 - 1.98 (m, 4H), 1.63 - 1.44 (m, 4H).

### Example 167: 1-(4-(((6-(2-Chloro-3-(3-chloro-2-(3-methoxy-4-(((1-(2-methoxyacetyl)piperidin-4-yl)amino)methyl)phenyl)pyridin-4-yl)phenyl)-2-methoxypyridin-3-yl)methyl)amino)piperidin-1-yl)-2-methoxyethan-1-one (397)

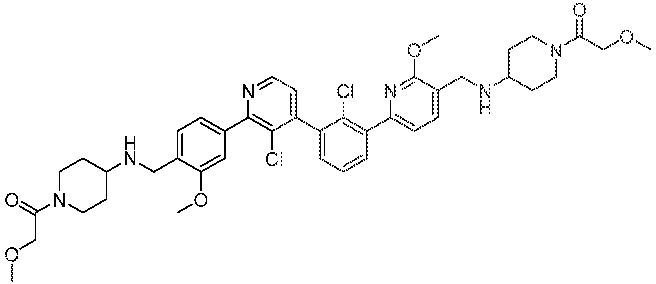

### (a) 1-(4-Aminopiperidin-1-yl)-2-methoxyethan-1-one

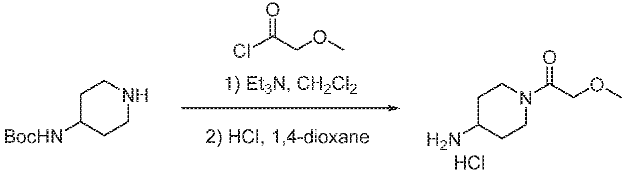

A solution of tert-butyl N-(4-piperidyl)carbamate (0.80 g, 3.99 mmol) in 32 mL DCM was cooled in an ice bath. Triethylamine (1.11 mL, 7.99 mmol) was added. A solution of 2-methoxyacetyl chloride (0.40 mL, 4.39 mmol) in 8 mL DCM was added slowly. After 10 minutes, the reaction mixture was diluted with 30 mL DCM and washed with saturated sodium bicarbonate solution (10 mL), 0.3 M HCl (10 mL), and saturated sodium bicarbonate solution (10 mL). The organics were dried over sodium sulfate and concentrated under reduced pressure to provide crude tert-butyl (1-(2-methoxyacetyl)piperidin-4-yl)carbamate (1.06 g, 97% yield) as a white solid. MS: m/z found 273.1 [M+H]⁺. Hydrogen chloride, 4 M in 1,4-dioxane (8.26 mL, 33.05 mmol) was added to tert-butyl (1-(2-methoxyacetyl)piperidin-4-yl)carbamate (0.60 g, 2.20 mmol). After stirring at room temperature for 6h, the volatiles were removed *in vacuo.* The crude material was triturated with 2 x 5 mL anhydrous diethyl ether, and dried under vacuum to provide 1-(4-aminopiperidin-1-yl)-2-methoxyethan-1-one hydrochloride (0.35 g, 76% yield). MS: m/z found 173.1 [M+H]⁺.

### (b) 1-(4-(((6-(2-Chloro-3-(3-chloro-2-(3-methoxy-4-(((1-(2-methoxyacetyl)piperidin-4-yl)amino)methyl)phenyl)pyridin-4-yl)phenyl)-2-methoxypyridin-3-yl)methyl)amino)piperidin-1-yl)-2-methoxyethan-1-one

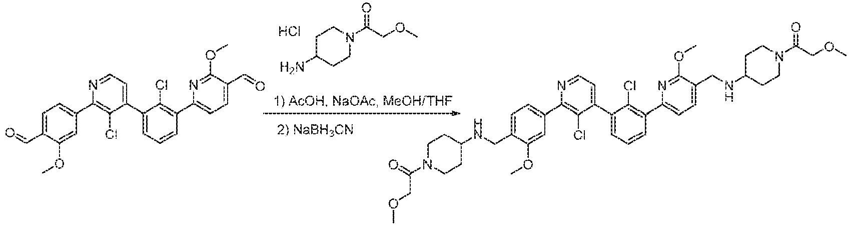

1-(4-(((6-(2-Chloro-3-(3-chloro-2-(3-methoxy-4-(((1-(2-methoxyacetyl)piperidin-4-yl)amino)methyl)phenyl)pyridin-4-yl)phenyl)-2-methoxypyridin-3-yl)methyl)amino)piperidin-1-yl)-2-methoxyethan-1-one (49% yield( was prepared as the formic acid salt, following Reductive Amination Procedure A from 6-(2-chloro-3-(3-chloro-2-(4-formyl-3-methoxyphenyl)pyridin-4-yl)phenyl)-2-methoxynicotinaldehyde (28 mg, 0.06 mmol) and 1-(4-aminopiperidin-1-yl)-2-methoxyethan-1-one hydrochloride (42 mg, 0.20 mmol). LCMS: m/z found 805.0 [M+H]⁺, retention time = 2.66 min (Method A). ¹H NMR (400 MHz, Methanol-*d*₄): δ 8.64 (d, 1H), 8.54 (s, 1H), 7.78 (d, 1H), 7.71 (dd, 1H), 7.55 (t, 1H), 7.50 - 7.44 (m, 2H), 7.41 (dd, 1H), 7.36 - 7.24 (m, 3H), 4.55 - 4.46 (m, 2H), 4.21 (m, 2H), 4.16 - 4.06 (m, 4H), 4.03 (s, 3H), 3.98 - 3.86 (m, 7H), 3.40 (d, 6H), 3.08 (m, 3H), 2.89 (s, 1H), 2.74 (m, 2H), 2.16 - 2.04 (m, 4H), 1.53 - 1.27 (m, 4H).

### Example 168: N-((6-(2-Chloro-3-(3-chloro-2-(3-methoxy-4-(((1-(2,2,2-trifluoroethyl)piperidin-4-yl)amino)methyl)phenyl)pyridin-4-yl)phenyl)-2-methoxypyridin-3-yl)methyl)-1-(2,2,2-trifluoroethyl)piperidin-4-amine (437)

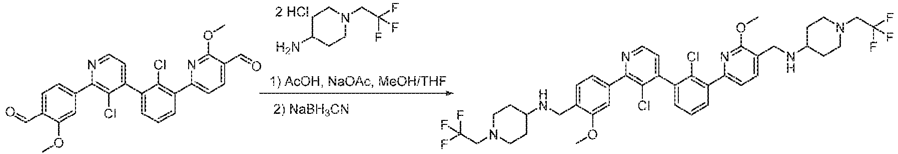

N-((6-(2-Chloro-3-(3-chloro-2-(3-methoxy-4-(((1-(2,2,2-trifluoroethyl)piperidin-4-yl)amino)methyl)phenyl)pyridin-4-yl)phenyl)-2-methoxypyridin-3-yl)methyl)-1-(2,2,2-trifluoroethyl)piperidin-4-amine (46% yield) was prepared as the formic acid salt, following Reductive Amination Procedure A from 6-(2-chloro-3-(3-chloro-2-(4-formyl-3-methoxyphenyl)pyridin-4-yl)phenyl)-2-methoxynicotinaldehyde (29 mg, 0.06 mmol) and 1-(2,2,2-trifluoroethyl)piperidin-4-amine dihydrochloride (53 mg, 0.21 mmol). LCMS: m/z found 825.0 [M+H]⁺, retention time = 3.71 min, (Method A). ¹H NMR (400 MHz, Methanol-*d*₄): δ 8.63 (d, 1H), 7.78 - 7.68 (m, 2H), 7.55 (t, 1H), 7.48 - 7.37 (m, 3H), 7.34 - 7.22 (m, 3H), 4.02 (s, 3H), 4.02 (s, 2H), 3.95 (s, 3H), 3.87 (s, 2H), 3.08 (m, 2H), 3.02 (s, 5H), 3.06 - 2.97 (m, 1H), 2.74 (s, 1H), 2.59 (t, 1H), 2.41 (t, 4H), 1.98 (t, 4H), 1.65 - 1.42 (m, 4H).

### Example 169: 1-(4-(((6-(2-Chloro-3-(3-chloro-2-(4-(((1-(2-hydroxyacetyl)piperidin-4-yl)amino)methyl)-3-methoxyphenyl)pyridin-4-yl)phenyl)-2-methoxypyridin-3-yl)methyl)amino)piperidin-1-yl)-2-hydroxyethan-1-one (443)

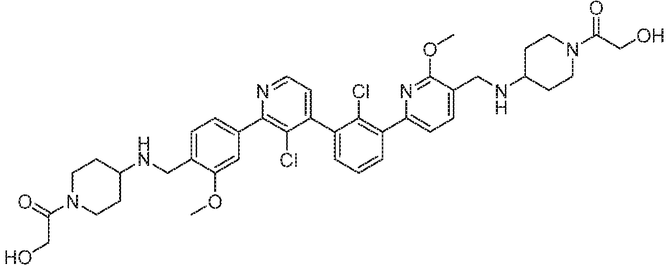

### (a) 1-(4-Aminopiperidin-1-yl)-2-hydroxyethan-1-one

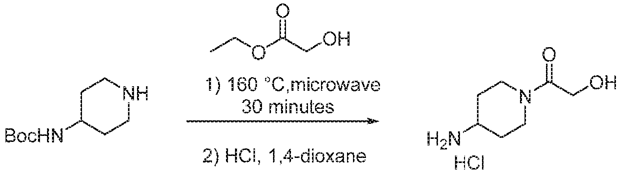

A mixture of ethyl 2-hydroxyacetate (0.91 mL, 9.53 mmol) and tert-butyl N-(4-piperidyl)carbamate (2.12 g, 10.59 mmol) was irradiated to 160 °C for 30 minutes in a Biotage Initiator microwave. The volatiles were removed *in vacuo,* and the residue was purified by normal phase SiO₂ chromatography (20-100% ethyl acetate/petroleum ether, visualizing fractions with iodine stain) to afford tert-butyl (1-(2-hydroxyacetyl)piperidin-4-yl)carbamate (1.01 g, 37% yield) as a white solid. ¹H NMR (400 MHz, Chloroform-*d*) δ 4.48 (d, 2H), 4.15 (d, 2H), 3.69 (s, 1H), 3.52 - 3.42 (m, 1H), 3.08 - 3.00 (m, 1H), 2.92 - 2.80 (m, 1H), 2.08 - 1.95 (m, 1H), 1.44 (s, 9H), 1.38 - 1.33 (m, 2H). Hydrogen chloride, 4 M in 1,4-dioxane (5.52 mL, 22.1 mmol) was added to tert-butyl (1-(2-hydroxyacetyl)piperidin-4-yl)carbamate (0.38 g, 1.47 mmol). After stirring at room temperature for 3 h, the volatiles were removed *in vacuo.* The crude material was triturated with 2 x 4 mL anhydrous diethyl ether, and dried under vacuum to provide crude 1-(4-aminopiperidin-1-yl)-2-hydroxyethan-1-one hydrochloride (0.35 g). MS: m/z found 159.1 [M+H]⁺.

### (b) 1-(4-(((6-(2-Chloro-3-(3-chloro-2-(4-(((1-(2-hydroxyacetyl)piperidin-4-yl)amino)methyl)-3-methoxyphenyl)pyridin-4-yl)phenyl)-2-methoxypyridin-3-yl)methyl)amino)piperidin-1-yl)-2-hydroxyethan-1-one

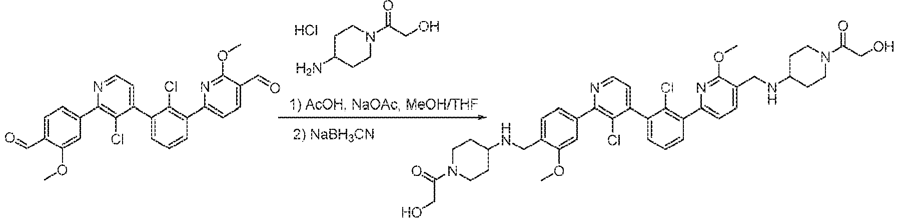

1-(4-(((6-(2-Chloro-3-(3-chloro-2-(4-(((1-(2-hydroxyacetyl)piperidin-4-yl)amino)methyl)-3-methoxyphenyl)pyridin-4-yl)phenyl)-2-methoxypyridin-3-yl)methyl)amino)piperidin-1-yl)-2-hydroxyethan-1-one (49% yield) was prepared as the formic acid salt, following Reductive Amination Procedure A from 6-(2-chloro-3-(3-chloro-2-(4-formyl-3-methoxyphenyl)pyridin-4-yl)phenyl)-2-methoxynicotinaldehyde (29 mg, 0.06 mmol) and 1-(4-aminopiperidin-1-yl)-2-hydroxyethan-1-one hydrochloride (40 mg, 0.21 mmol). LCMS: m/z found 777.2 [M+H]⁺, retention time = 2.36 min, (Method A). ¹H NMR (400 MHz, Methanol-*d*₄): δ 8.64 (d, 1H), 7.78 (d, 1H), 7.71 (dd, 1H), 7.55 (t, 1H), 7.51 - 7.38 (m, 3H), 7.37 - 7.28 (m, 2H), 7.27 (d, 1H), 4.59 - 4.44 (m, 2H), 4.31 - 4.16 (m, 4H), 4.13 (s, 2H), 4.03 (s, 3H), 3.95 (d, 5H), 3.85- 3.77 (m, 2H), 3.17 - 3.03 (m, 3H), 2.93 (s, 1H), 2.77 (q, 2H), 2.16- 2.04 (m, 4H), 1.54 - 1.27 (m, 4H).

### Example 170: Methyl 4-(((6-(2-chloro-3-(3-chloro-2-(3-methoxy-4-(((1-(methoxycarbonyl)piperidin-4-yl)amino)methyl)phenyl)pyridin-4-yl)phenyl)-2-methoxypyridin-3-yl)methyl)amino)piperidine-1-carboxylate (465)

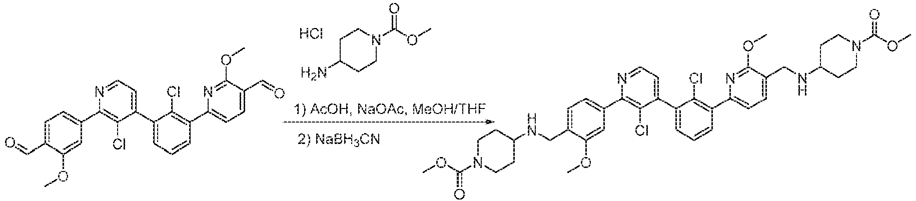

Methyl 4-(((6-(2-chloro-3-(3-chloro-2-(3-methoxy-4-(((1-(methoxycarbonyl)piperidin-4-yl)amino)methyl)phenyl)pyridin-4-yl)phenyl)-2-methoxypyridin-3-yl)methyl)amino)piperidine-1-carboxylate (43% yield) was prepared as the formic acid salt, following Reductive Amination Procedure A from 6-(2-chloro-3-(3-chloro-2-(4-formyl-3-methoxyphenyl)pyridin-4-yl)phenyl)-2-methoxynicotinaldehyde (29 mg, 0.06 mmol) and methyl 4-aminopiperidine-1-carboxylate hydrochloride (40 mg, 0.21 mmol). LCMS: m/z found 777.2 [M+H]⁺, retention time = 3.08 min (Method A). ¹H NMR (400 MHz, Methanol-*d*₄): δ 8.63 (d, 1H), 7.79 - 7.67 (m, 2H), 7.55 (t, 1H), 7.47 - 7.37 (m, 3H), 7.33 - 7.22 (m, 3H), 4.13 (s, 4H), 4.01 (m, 5H), 3.94 (s, 3H), 3.88 (s, 2H), 3.68 (d, 6H), 2.95 - 2.72 (m, 6H), 2.00 (m, 4H), 1.45 - 1.26 (m, 4H).

### Example 171: (3-(((6-(2-Chloro-3-(3-chloro-2-(4-(((3-(hydroxymethyl)bicyclo[1.1.1]pentan-1-yl)amino)methyl)-3-methoxyphenyl)pyridin-4-yl)phenyl)-2-methoxypyridin-3-yl)methyl)amino)bicyclo[1.1.1]pentan-1-yl)metanol (591)

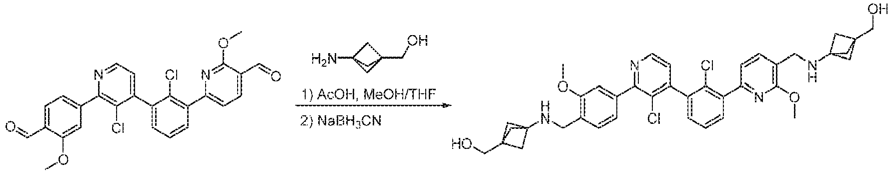

(3-(((6-(2-Chloro-3-(3-chloro-2-(4-(((3-(hydroxymethyl)bicyclo[1.1.1]pentan-1-yl)amino)methyl)-3-methoxyphenyl)pyridin-4-yl)phenyl)-2-methoxypyridin-3-yl)methyl)amino)bicyclo[1.1.1]pentan-1-yl)methanol (45% yield) was prepared as the formic acid salt, following Reductive Amination Procedure A from 6-(2-chloro-3-(3-chloro-2-(4-formyl-3-methoxyphenyl)pyridin-4-yl)phenyl)-2-methoxynicotinaldehyde (30 mg, 0.06 mmol) and (3-aminobicyclo[1.1.1]pentan-1-yl)methanol (24 mg, 0.21 mmol). 45% yield. LCMS: m/z found 687.4 [M+H]⁺, retention time = 2.81 min, (Method A); ¹H NMR (400 MHz, Methanol-*d*₄): δ 8.62 (d, 1H), 7.78 - 7.67 (m, 2H), 7.54 (t, 1H), 7.46 - 7.37 (m, 3H), 7.30 - 7.20 (m, 3H), 4.02 (s, 3H), 3.92 (d, 5H), 3.81 (s, 2H), 3.60 (d, 4H), 1.81 - 1.71 (m, 12H).

### Example 172: 1-(6-((6-(2-Chloro-3-(3-chloro-2-(3-methoxy-4-((6-(2-methoxyacetyl)-2,6-diazaspiro[3.3]heptan-2-yl)methyl)phenyl)pyridin-4-yl)phenyl)-2-methoxypyridin-3-yl)methyl)-2,6-diazaspiro[3.3]heptan-2-yl)-2-methoxyethan-1-one (594)

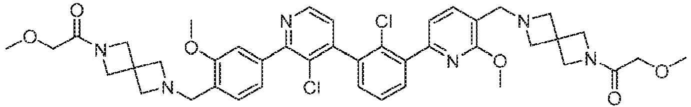

### (a) 2-Methoxy-1-(2,6-diazaspiro[3.3]heptan-2-yl)ethan-1-one

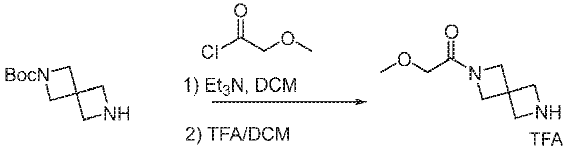

Triethylamine (1.89 mL, 13.56 mmol) was added to a mixture of 2,6-diazaspiro[3.3]heptane-2-carboxylic acid tert-butyl ester hemioxylate (1.50 g, 3.08 mmol) in 40 mL DCM. The mixture was cooled in an ice bath. A solution of 2-methoxyacetyl chloride (0.62 mL, 6.78 mmol) in 10 mL DCM was added slowly. After 3 h, the mixture was washed with 20 mL saturated sodium bicarbonate solution, 20 mL 0.2 M HCl, and 20 mL saturated sodium bicarbonate solution. The organics were dried over sodium sulfate, concentrated under reduced pressure, and purified by normal phase SiO₂ chromatography (15% to 100% EtOAc/hexanes) to provide tert-butyl 6-(2-methoxyacetyl)-2,6-diazaspiro[3.3]heptane-2-carboxylate (0.59 g, 71% yield). ¹H NMR (400 MHz, Chloroform-*d*) δ 4.37 (s, 2H), 4.16 (s, 2H), 4.17 - 4.03 (m, 4H), 3.97 (s, 2H), 3.38 (s, 3H), 1.43 (s, 9H).

A solution of trifluoroacetic acid (1.85 mL, 7.40 mmol) in 3 mL DCM was added to a 0 °C solution of tert-butyl 6-(2-methoxyacetyl)-2,6-diazaspiro[3.3]heptane-2-carboxylate (0.20 g, 0.74 mmol) in 7 mL DCM. After 10 min, the ice bath was removed. After 30 min, the volatiles were removed *in vacuo.* The resulting oil was azeotroped twice with 5 mL toluene to provide crude 2-methoxy-1-(2,6-diazaspiro[3.3]heptan-2-yl)ethan-1-one as the TFA salt (0.22 g). ¹H NMR (400 MHz, Methanol-*d*₄) δ 4.47 (s, 2H), 4.23 (d, 6H), 3.97 (s, 2H), 3.37 (s, 3H). MS: m/z found 171.1 [M+H]⁺.

### (b) 1-(6-((6-(2-Chloro-3-(3-chloro-2-(3-methoxy-4-((6-(2-methoxyacetyl)-2,6-diazaspiro[3.3]heptan-2-yl)methyl)phenyl)pyridin-4-yl)phenyl)-2-methoxypyridin-3-yl)methyl)-2,6-diazaspiro[3.3]heptan-2-yl)-2-methoxyethan-1-one

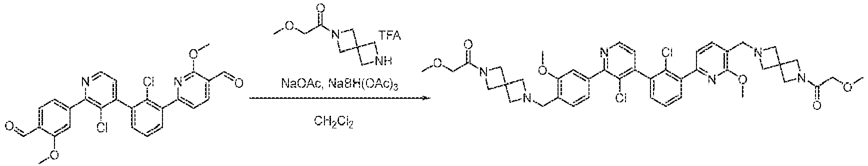

A mixture of 2-methoxy-1-(2,6-diazaspiro[3.3]heptan-2-yl)ethan-1-one TFA salt (43 mg, 0.15 mmol) and sodium acetate (17 mg, 0.20 mmol) in 0.45 mL DCM was stirred for 10 min at room temperature. After the additon of 6-(2-chloro-3-(3-chloro-2-(4-formyl-3-methoxyphenyl)pyridin-4-yl)phenyl)-2-methoxynicotinaldehyde (25 mg, 0.05 mmol), the mixture stirred for 5 min. Sodium triacetoxyborohydride (32 mg, 0.15 mmol) was added. After stirring overnight at room temperature, the reaction mixture was diluted with 2 mL MeOH and filtered through a syringe filter. The crude product was purified by reversed phase chromatography (5 to 65% acetonitrile gradient in water, with 0.05% formic acid) and the combined pure fractions were lyophilized to afford 1-[6-[[4-[3-chloro-4-[2-chloro-3-[6-methoxy-5-[[2-(2-methoxyacetyl)-2,6-diazaspiro[3.3]heptan-6-yl]methyl]-2-pyridyl]phenyl]-2-pyridyl]-2-methoxy-phenyl]methyl]-2,6-diazaspiro[3.3]heptan-2-yl]-2-methoxy-ethanone (12 mg, 28%) as formic acid salt. LCMS: m/z found 801.4 [M+H]⁺, retention time = 2.70 min, (Method A). ¹H NMR (400 MHz, Methanol-*d*₄) δ 8.65 (d, 1H), 8.50 (s, 1H), 7.75 - 7.67 (m, 2H), 7.55 (t, 1H), 7.49 - 7.38 (m, 3H), 7.38 - 7.30 (m, 2H), 7.27 (d, 1H), 4.41 (d, 4H), 4.20 - 4.11 (m, 6H), 4.05-4.01 (m, 7H), 3.96 (s, 7H), 3.85 (s, 2H), 3.72 (s, 4H), 3.36 (s, 6H).

### Example 173: N-((6-(2-chloro-3-(3-chloro-2-(3-methoxy-4-(((tetrahydro-2H-pyran-4-yl)amino)methyl)phenyl)pyridin-4-yl)phenyl)-2-methoxypyridin-3-yl)methyl)tetrahydro-2H-pyran-4-amine (298)

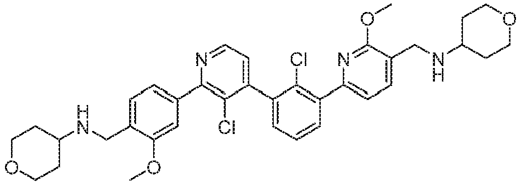

N-((6-(2-chloro-3-(3-chloro-2-(3-methoxy-4-(((tetrahydro-2H-pyran-4-yl)amino)methyl)phenyl)pyridin-4-yl)phenyl)-2-methoxypyridin-3-yl)methyl)tetrahydro-2H-pyran-4-amine was prepared as the formic acid salt following Reductive Amination Procedure A from 6-(2-chloro-3-(3-chloro-2-(4-formyl-3-methoxyphenyl)pyridin-4-yl)phenyl)-2-methoxynicotinaldehyde (30 mg, 0.06 mmol) and tetrahydropyran-4-amine (25 mg, 0.24 mmol). 75% yield. LCMS: m/z found 663.2 [M+H]⁺, retention time = 1.80 min (Method D). ¹H NMR (400 MHz, Methanol-d₄): δ 8.64 (d, 1H), 8.54 (s, 2H), 7.77 (d, 1H), 7.71 (dd, 1H), 7.55 (t, 1H), 7.49 - 7.44 (m, 2H), 7.41 (dd, 1H), 7.35 - 7.22 (m, 3H), 4.09 (s, 2H), 4.03 (s, 3H), 4.02 - 3.97 (m, 4H), 3.96 (s, 3H), 3.92 (s, 2H), 3.43 (t, 4H), 3.15 - 3.00 (m, 1H), 2.91 - 2.78 (m, 1H), 2.08 - 1.87 (m, 4H), 1.68 - 1.39 (m, 4H).

### Example 174: 4-(((6-(2-Chloro-3-(3-chloro-2-(4-(((1,1-dioxidotetrahydro-2H-thiopyran-4-yl)amino)methyl)-3-methoxyphenyl)pyridin-4-yl)phenyl)-2-methoxypyridin-3-yl)methyl)amino)tetrahydro-2H-thiopyran 1,1-dioxide (299)

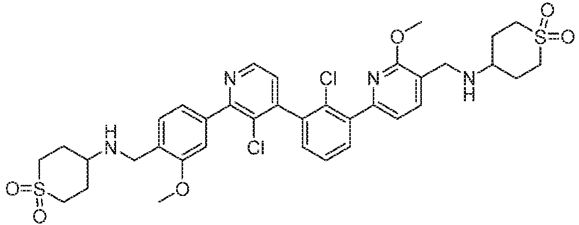

4-(((6-(2-Chloro-3-(3-chloro-2-(4-(((1,1-dioxidotetrahydro-2H-thiopyran-4-yl)amino)methyl)-3-methoxyphenyl)pyridin-4-yl)phenyl)-2-methoxypyridin-3-yl)methyl)amino)tetrahydro-2H-thiopyran 1,1-dioxide was prepared as the formic acid salt following Reductive Amination Procedure A from 6-(2-chloro-3-(3-chloro-2-(4-formyl-3-methoxyphenyl)pyridin-4-yl)phenyl)-2-methoxynicotinaldehyde (30 mg, 0.06 mmol) and 1,1-dioxothian-4-amine (36 mg, 0.24 mmol). 80% yield. LCMS: m/z found 759.2 [M+H]⁺, retention time = 1.68 min (Method D). ¹H NMR (400 MHz, Methanol-*d*₄): δ 8.64 (d, 1H), 8.44 (s, 2H), 7.79 (d, 1H), 7.71 (dd, 1H), 7.55 (t, 1H), 7.51 - 7.44 (m, 2H), 7.41 (dd, 1H), 7.35 - 7.29 (m, 2H), 7.27 (d, 1H), 4.10 (s, 2H), 4.03 (s, 3H), 3.96 (s, 3H), 3.90 (s, 2H), 3.26 - 3.14 (m, 7H), 3.13 - 3.04 (m, 2H), 3.01 - 2.93 (m, 1H), 2.46 - 2.36 (m, 2H), 2.36 - 2.27 (m, 2H), 2.23 - 2.04 (m, 4H).

### Example 175: 1-(3-(((6-(3-(2-(4-(((1-Acetylazetidin-3-yl)amino)methyl)-3-methoxyphenyl)-3-chloropyridin-4-yl)-2-chlorophenyl)-2-methoxypyridin-3-yl)methyl)amino)azetidin-1-yl)ethan-1-one (300)

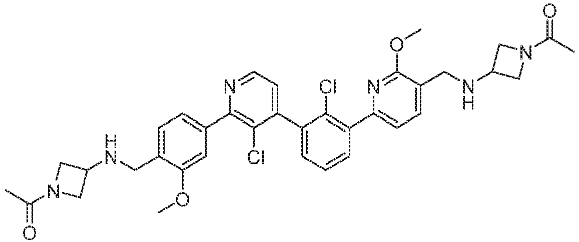

1-(3-(((6-(3-(2-(4-(((1-Acetylazetidin-3-yl)amino)methyl)-3-methoxyphenyl)-3-chloropyridin-4-yl)-2-chlorophenyl)-2-methoxypyridin-3-yl)methyl)amino)azetidin-1-yl)ethan-1-one was prepared as the formic acid salt following Reductive Amination Procedure A from 6-(2-chloro-3-(3-chloro-2-(4-formyl-3-methoxyphenyl)pyridin-4-yl)phenyl)-2-methoxynicotinaldehyde (30 mg, 0.06 mmol) and 1-(3-aminoazetidin-1-yl)ethanone (28 mg, 0.24 mmol). 83% yield. LCMS: m/z found 689.2 [M+H]⁺, retention time = 1.59 min (Method D). 1H NMR (400 MHz, Methanol-d4): δ 8.68 - 8.59 (m, 1H), 8.32 (s, 2H), 7.75 (d, 1H), 7.71 (d, 1H), 7.55 (t, 1H), 7.47 - 7.38 (m, 3H), 7.32 - 7.21 (m, 3H), 4.39 - 4.28 (m, 2H), 4.18 - 4.07 (m, 2H), 4.03 (s, 3H), 4.00 - 3.90 (m, 7H), 3.87 - 3.67 (m, 6H), 1.86 (s, 6H).

### Example 176: (S)-1-(((6-(2-Chloro-3-(3-chloro-2-(4-((((S)-2-hydroxypropyl)amino)methyl)-3-methoxyphenyl)pyridin-4-yl)phenyl)-2-methoxypyridin-3-yl)methyl)amino)propan-2-ol (301)

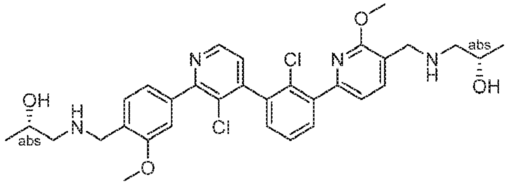

(S)-1-(((6-(2-Chloro-3-(3-chloro-2-(4-((((S)-2-hydroxypropyl)amino)methyl)-3-methoxyphenyl)pyridin-4-yl)phenyl)-2-methoxypyridin-3-yl)methyl)amino)propan-2-ol was prepared as the formic acid salt following Reductive Amination Procedure A from 6-(2-chloro-3-(3-chloro-2-(4-formyl-3-methoxyphenyl)pyridin-4-yl)phenyl)-2-methoxynicotinaldehyde (30 mg, 0.06 mmol) and (2S)-1-aminopropan-2-ol (18 mg, 0.24 mmol). 85% yield. LCMS: m/z found 611.2 [M+H]⁺, retention time = 1.70 min (Method D). ¹H NMR (400 MHz, Methanol-*d*₄): δ 8.66 (d, 1H), 8.52 (s, 2H), 7.84 (d, 1H), 7.73 (d, 1H), 7.57 (t, 1H), 7.51 (d, 1H), 7.48 (d, 1H), 7.44 (d, 1H), 7.39 (s, 1H), 7.34 (t, 2H), 4.28 (s, 2H), 4.20 - 4.13 (m, 2H), 4.10 - 4.01 (m, 5H), 3.99 (s, 3H), 3.05 (d, 1H), 2.98 (d, 1H), 2.90 - 2.74 (m, 2H), 1.23 (dd, 6H).

### Example 177: 2-((4-(3-Chloro-4-(2-chloro-3-(6-methoxy-5-(((5-oxo-6-azaspiro[3.4]octan-2-yl)amino)methyl)pyridin-2-yl)phenyl)pyridin-2-yl)-2-methoxybenzyl)amino)-6-azaspiro[3.4]octan-5-one (302)

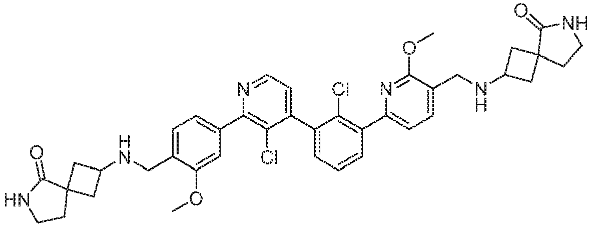

2-((4-(3-Chloro-4-(2-chloro-3-(6-methoxy-5-(((5-oxo-6-azaspiro[3.4]octan-2-yl)amino)methyl)pyridin-2-yl)phenyl)pyridin-2-yl)-2-methoxybenzyl)amino)-6-azaspiro[3.4]octan-5-one was prepared as the formic acid salt as a mixture of isomers (axial chirality) by following Reductive Amination Procedure A from 6-(2-chloro-3-(3-chloro-2-(4-formyl-3-methoxyphenyl)pyridin-4-yl)phenyl)-2-methoxynicotinaldehyde (30 mg, 0.06 mmol) and 2-amino-6-azaspiro[3.4]octan-5-one (34 mg, 0.24 mmol). 88% yield. LCMS: m/z found 741.3 [M+H]⁺, retention time = 1.73 min, 1.78 min and 1.85 min (Method D). ¹H NMR (400 MHz, Methanol-*d*₄): δ 8.65 (d, 1H), 8.53 (s, 2H), 7.76 (t, 1H), 7.71 (d, 1H), 7.56 (t, 1H), 7.50 - 7.44 (m, 2H), 7.42 (d, 1H), 7.35 (s, 1H), 7.32 (d, 1H), 7.29 - 7.24 (m, 1H), 4.10 (s, 1H), 4.07 - 4.01 (m, 4H), 4.01 - 3.95 (m, 3H), 3.91 - 3.80 (m, 2H), 3.77 - 3.63 (m, 1H), 3.60 - 3.45 (m, 1H), 3.28 - 3.21 (m, 4H), 2.66 - 2.52 (m, 2H), 2.46 - 2.35 (m, 2H), 2.33 - 2.17 (m, 6H), 2.17 - 2.09 (m, 1H), 2.03 - 1.92 (m, 1H).

### Example 178: 1-(2-(((6-(2-Chloro-3-(3-chloro-2-(3-methoxy-4-(((2-(2-oxopyrrolidin-1-yl)ethyl)amino)methyl)phenyl)pyridin-4-yl)phenyl)-2-methoxypyridin-3-yl)methyl)amino)ethyl)pyrrolidin-2-one (303)

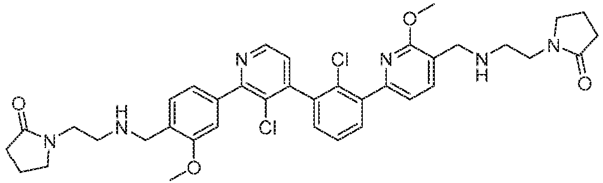

1-(2-(((6-(2-Chloro-3-(3-chloro-2-(3-methoxy-4-(((2-(2-oxopyrrolidin-1-yl)ethyl)amino)methyl)phenyl)pyridin-4-yl)phenyl)-2-methoxypyridin-3-yl)methyl)amino)ethyl)pyrrolidin-2-one was prepared as the formic acid salt following Reductive Amination Procedure A from 6-(2-chloro-3-(3-chloro-2-(4-formyl-3-methoxyphenyl)pyridin-4-yl)phenyl)-2-methoxynicotinaldehyde (30 mg, 0.06 mmol) and 1-(2-aminoethyl)pyrrolidin-2-one (31 mg, 0.24 mmol). 87% yield. LCMS: m/z found 717.3 [M+H]⁺, retention time = 1.75 min (Method D). ¹H NMR (400 MHz, Methanol-*d*₄): δ 8.66 (d, 1H), 8.44 (s, 2H), 7.82 (d, 1H), 7.72 (d, 1H), 7.57 (t, 1H), 7.50 (d, 1H), 7.47 (d, 1H), 7.43 (d, 1H), 7.39 (s, 1H), 7.36 - 7.28 (m, 2H), 4.28 (s, 2H), 4.11 (s, 2H), 4.06 (s, 3H), 3.99 (s, 3H), 3.63 (t, 2H), 3.57 (t, 2H), 3.50 (t, 4H), 3.23 (t, 2H), 3.10 (t, 2H), 2.46 - 2.36 (m, 4H), 2.14 - 2.02 (m, 4H).

### Example 179: N-((6-(2-Chloro-3-(3-chloro-2-(4-(((1-isopropylpiperidin-4-yl)amino)methyl)-3-methoxyphenyl)pyridin-4-yl)phenyl)-2-methoxypyridin-3-yl)methyl)-1-isopropylpiperidin-4-amine (326)

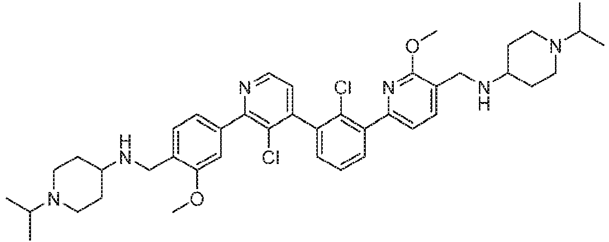

N-((6-(2-Chloro-3-(3-chloro-2-(4-(((1-isopropylpiperidin-4-yl)amino)methyl)-3-methoxyphenyl)pyridin-4-yl)phenyl)-2-methoxypyridin-3-yl)methyl)-1-isopropylpiperidin-4-amine was prepared as the formic acid salt following Reductive Amination Procedure A from 6-(2-chloro-3-(3-chloro-2-(4-formyl-3-methoxyphenyl)pyridin-4-yl)phenyl)-2-methoxynicotinaldehyde (25 mg, 0.05 mmol) and 1-isopropylpiperidin-4-amine (29 mg, 0.20 mmol). 73% yield. LCMS: m/z found 745.4 [M+H]⁺, retention time = 1.51 min (Method D). ¹H NMR (400 MHz, Methanol-*d*₄): δ 8.65 (d, 1H), 8.47 (s, 4H), 7.81 (d, 1H), 7.71 (dd, 1H), 7.56 (t, 1H), 7.51 - 7.44 (m, 2H), 7.42 (dd, 1H), 7.35 (d, 1H), 7.33 - 7.23 (m, 2H), 4.12 (s, 2H), 4.04 (s, 3H), 3.99 - 3.91 (m, 5H), 3.46 - 3.33 (m, 5H), 3.29 - 3.22 (m, 1H), 3.14 - 3.05 (m, 1H), 3.03 - 2.91 (m, 3H), 2.83 (t, 2H), 2.31 - 2.15 (m, 4H), 1.89 - 1.67 (m, 4H), 1.31 (d, 6H), 1.27 (d, 6H).

### Example 180: N-((6-(2-Chloro-3-(3-chloro-2-(4-(((4,4-difluorocyclohexyl)amino)methyl)-3-methoxyphenyl)pyridin-4-yl)phenyl)-2-methoxypyridin-3-yl)methyl)-4,4-difluorocyclohexan-1-amine (327)

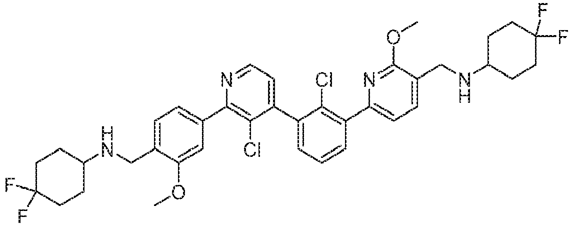

N-((6-(2-Chloro-3-(3-chloro-2-(4-(((4,4-difluorocyclohexyl)amino)methyl)-3-methoxyphenyl)pyridin-4-yl)phenyl)-2-methoxypyridin-3-yl)methyl)-4,4-difluorocyclohexan-1-amine was prepared as the formic acid salt following Reductive Amination Procedure A from 6-(2-chloro-3-(3-chloro-2-(4-formyl-3-methoxyphenyl)pyridin-4-yl)phenyl)-2-methoxynicotinaldehyde (25 mg, 0.05 mmol) and 4,4-difluorocyclohexanamine (27 mg, 0.20 mmol). 80% yield. LCMS: m/z found 731.3 [M+H]⁺, retention time = 2.27 min (Method D). ¹H NMR (400 MHz, Methanol-*d*₄): δ 8.69 - 8.64 (m, 1H), 8.51 - 8.46 (m, 2H), 7.85 (d, 1H), 7.75 - 7.69 (m, 1H), 7.57 (td, 1H), 7.54 - 7.50 (m, 1H), 7.49 - 7.46 (m, 1H), 7.44 (d, 1H), 7.39 (s, 1H), 7.33 (t, 2H), 4.28 (s, 2H), 4.13 (s, 2H), 4.06 (s, 3H), 3.99 (s, 3H), 3.28 - 3.20 (m, 1H), 3.17 - 3.05 (m, 1H), 2.30 - 2.11 (m, 8H), 2.05 - 1.82 (m, 4H), 1.80 - 1.60 (m, 4H).

### Example 181: 2-(4-(((6-(2-Chloro-3-(3-chloro-2-(4-(((1-(2-hydroxyethyl)piperidin-4-yl)amino)methyl)-3-methoxyphenyl)pyridin-4-yl)phenyl)-2-methoxypyridin-3-yl)methyl)amino)piperidin-1-yl)ethan-1-ol (328)

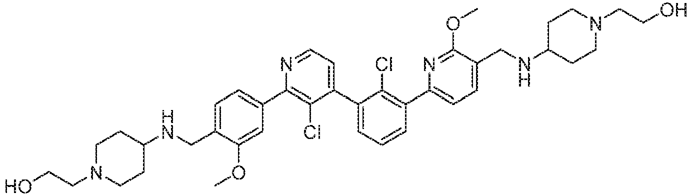

2-(4-(((6-(2-Chloro-3-(3-chloro-2-(4-(((1-(2-hydroxyethyl)piperidin-4-yl)amino)methyl)-3-methoxyphenyl)pyridin-4-yl)phenyl)-2-methoxypyridin-3-yl)methyl)amino)piperidin-1-yl)ethan-1-ol was prepared as the formic acid salt following Reductive Amination Procedure A from 6-(2-chloro-3-(3-chloro-2-(4-formyl-3-methoxyphenyl)pyridin-4-yl)phenyl)-2-methoxynicotinaldehyde (25 mg, 0.05 mmol) and 2-(4-amino-1-piperidyl)ethanol (29 mg, 0.20 mmol). 84% yield. LCMS: m/z found 749.3 [M+H]⁺, retention time = 1.38 min (Method D). ¹H NMR (400 MHz, Methanol-*d*₄): δ 8.69 - 8.63 (m, 1H), 8.40 (s, 4H), 7.85 (d, 1H), 7.72 (dt, 1H), 7.57 (t, 1H), 7.52 (d, 1H), 7.47 (d, 1H), 7.45 - 7.40 (m, 1H), 7.38 (s, 1H), 7.33 (t, 2H), 4.23 (s, 2H), 4.09 (s, 2H), 4.06 (s, 3H), 3.98 (s, 3H), 3.83 - 3.72 (m, 4H), 3.45 - 3.32 (m, 4H), 3.23 - 3.03 (m, 2H), 2.94 (t, 2H), 2.81 (t, 2H), 2.71 (t, 2H), 2.52 (t, 2H), 2.27 - 2.15 (m, 4H), 1.90 - 1.72 (m, 4H).

### Example 182: N-(2-(((6-(3-(2-(4-(((2-Acetamidoethyl)amino)methyl)-3-methoxyphenyl)-3-chloropyridin-4-yl)-2-chlorophenyl)-2-methoxypyridin-3-yl)methyl)amino)ethyl)acetamide (329)

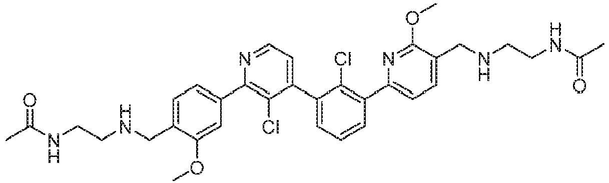

N-(2-(((6-(3-(2-(4-(((2-Acetamidoethyl)amino)methyl)-3-methoxyphenyl)-3-chloropyridin-4-yl)-2-chlorophenyl)-2-methoxypyridin-3-yl)methyl)amino)ethyl)acetamide was prepared as the formic acid salt following Reductive Amination Procedure A from 6-(2-chloro-3-(3-chloro-2-(4-formyl-3-methoxyphenyl)pyridin-4-yl)phenyl)-2-methoxynicotinaldehyde (30 mg, 0.06 mmol) and N-(2-aminoethyl)acetamide (25 mg, 0.24 mmol). 74% yield. LCMS: m/z found 665.2 [M+H]⁺, retention time = 1.63 min (Method D). ¹H NMR (400 MHz, Methanol-*d*₄): δ 8.66 (d, 1H), 8.43 (s, 2H), 7.86 (d, 1H), 7.72 (dd, 1H), 7.58 (t, 1H), 7.51 (d, 1H), 7.48 (d, 1H), 7.45 (dd, 1H), 7.39 (s, 1H), 7.37 - 7.31 (m, 2H), 4.31 (s, 2H), 4.23 (s, 2H), 4.08 (s, 3H), 4.00 (s, 3H), 3.55 - 3.46 (m, 4H), 3.23 - 3.09 (m, 4H), 2.03 - 1.95 (m, 6H).

### Example 183: 1-(6-(2-Chloro-3-(3-chloro-2-(3-methoxy-4-((((tetrahydro-2H-pyran-4-yl)methyl)amino)methyl)phenyl)pyridin-4-yl)phenyl)-2-methoxypyridin-3-yl)-N-((tetrahydro-2H-pyran-4-yl)methyl)methanamine (330)

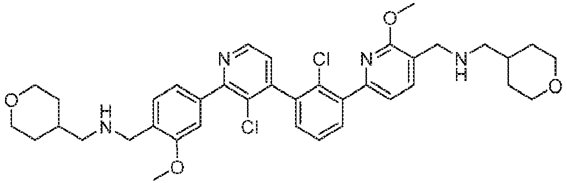

1-(6-(2-Chloro-3-(3-chloro-2-(3-methoxy-4-((((tetrahydro-2H-pyran-4-yl)methyl)amino)methyl)phenyl)pyridin-4-yl)phenyl)-2-methoxypyridin-3-yl)-N-((tetrahydro-2H-pyran-4-yl)methyl)methanamine was prepared as the formic acid salt following Reductive Amination Procedure A from 6-(2-chloro-3-(3-chloro-2-(4-formyl-3-methoxyphenyl)pyridin-4-yl)phenyl)-2-methoxynicotinaldehyde (30 mg, 0.06 mmol) and tetrahydropyran-4-ylmethanamine (28 mg, 0.24 mmol). 76% yield. LCMS: m/z found 691.3 [M+H]⁺, retention time = 1.88 min (Method D). ¹H NMR (400 MHz, Methanol-*d*₄): δ 8.66 (dd, 1H), 8.53 - 8.48 (m, 2H), 7.84 (d, 1H), 7.72 (dd, 1H), 7.57 (dd, 1H), 7.52 (d, 1H), 7.49 - 7.45 (m, 1H), 7.45 - 7.41 (m, 1H), 7.39 (s, 1H), 7.37 - 7.30 (m, 2H), 4.26 (s, 2H), 4.11 (s, 2H), 4.06 (s, 3H), 4.01 - 3.90 (m, 7H), 3.51 - 3.37 (m, 4H), 2.94 (d, 2H), 2.83 (d, 2H), 2.08 - 1.90 (m, 2H), 1.77 - 1.65 (m, 4H), 1.43 - 1.25 (m, 4H).

### Example 184: 1-(4-(((6-(2-Chloro-3-(3-chloro-2-(4-(((1-isobutyrylpiperidin-4-yl)amino)methyl)-3-methoxyphenyl)pyridin-4-yl)phenyl)-2-methoxypyridin-3-yl)methyl)amino)piperidin-1-yl)-2-methylpropan-1-one (346)

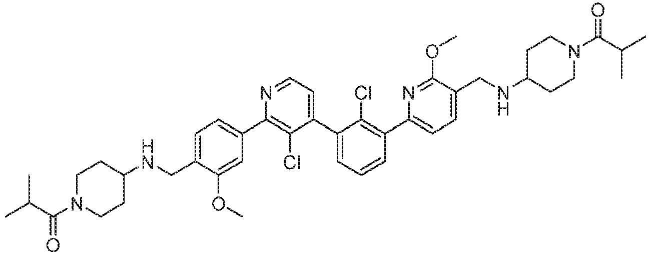

1-(4-(((6-(2-Chloro-3-(3-chloro-2-(4-(((1-isobutyrylpiperidin-4-yl)amino)methyl)-3-methoxyphenyl)pyridin-4-yl)phenyl)-2-methoxypyridin-3-yl)methyl)amino)piperidin-1-yl)-2-methylpropan-1-one was prepared as the formic acid salt following Reductive Amination Procedure A from 6-(2-chloro-3-(3-chloro-2-(4-formyl-3-methoxyphenyl)pyridin-4-yl)phenyl)-2-methoxynicotinaldehyde (30 mg, 0.06 mmol) and 1-(4-amino-1-piperidyl)-2-methyl-propan-1-one (41 mg, 0.24 mmol). 72% yield. LCMS: m/z found 801.4 [M+H]⁺, retention time = 2.00 min (Method D). ¹H NMR (400 MHz, Methanol-*d*₄): δ 8.66 (d, 1H), 8.46 (s, 2H), 7.88 (d, 1H), 7.72 (dd, 1H), 7.57 (t, 1H), 7.54 (d, 1H), 7.47 (d, 1H), 7.44 (dd, 1H), 7.39 (s, 1H), 7.34 (t, 2H), 4.68 (t, 2H), 4.31 (s, 2H), 4.27 - 4.15 (m, 4H), 4.07 (s, 3H), 3.99 (s, 3H), 3.50 - 3.40 (m, 1H), 3.38 - 3.32 (m, 1H), 3.20 (t, 2H), 2.99 (p, 2H), 2.69 (t, 2H), 2.35 - 2.14 (m, 5H), 1.67 - 1.39 (m, 4H), 1.17 - 1.00 (m, 12H).

### Example 185: 4-(((6-(2-Chloro-3-(3-chloro-2-(3-methoxy-4-(((1-methyl-2-oxopiperidin-4-yl)amino)methyl)phenyl)pyridin-4-yl)phenyl)-2-methoxypyridin-3-yl)methyl)amino)-1-methylpiperidin-2-one (418)

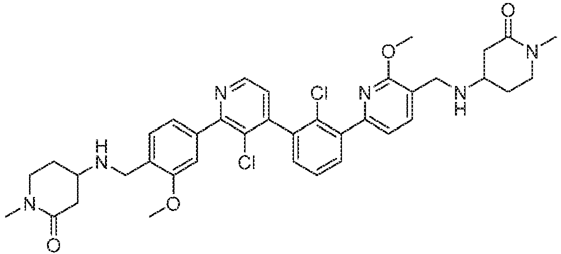

4-(((6-(2-Chloro-3-(3-chloro-2-(3-methoxy-4-(((1-methyl-2-oxopiperidin-4-yl)amino)methyl)phenyl)pyridin-4-yl)phenyl)-2-methoxypyridin-3-yl)methyl)amino)-1-methylpiperidin-2-one was prepared as the formic acid salt following Reductive Amination Procedure A from 6-(2-chloro-3-(3-chloro-2-(4-formyl-3-methoxyphenyl)pyridin-4-yl)phenyl)-2-methoxynicotinaldehyde (30 mg, 0.06 mmol) and 4-amino-1-methyl-piperidin-2-one (31 mg, 0.24 mmol). 83% yield. LCMS: m/z found 717.3 [M+H]⁺, retention time = 1.55 min (Method D). ¹H NMR (400 MHz, Methanol-*d*₄): δ 8.65 (dd, 1H), 8.47 (s, 1H), 7.79 (d, 1H), 7.71 (d, 1H), 7.56 (t, 1H), 7.49 (d, 1H), 7.46 (d, 1H), 7.42 (d, 1H), 7.35 (s, 1H), 7.32 (d, 1H), 7.27 (d, 1H), 4.22 - 4.09 (m, 2H), 4.03 (s, 3H), 3.97 (s, 3H), 3.96 - 3.92 (m, 2H), 3.51 - 3.36 (m, 5H), 3.23 - 3.14 (m, 1H), 2.99 - 2.89 (m, 6H), 2.82 (dd, 1H), 2.73 (dd, 1H), 2.46 - 2.16 (m, 4H), 1.97 - 1.75 (m, 2H).

### Example 186: 4-(((6-(2-Chloro-3-(3-chloro-2-(4-(((1-(dimethylcarbamoyl)piperidin-4-yl)amino)methyl)-3-methoxyphenyl)pyridin-4-yl)phenyl)-2-methoxypyridin-3-yl)methyl)amino)-N,N-dimethylpiperidine-1-carboxamide (419)

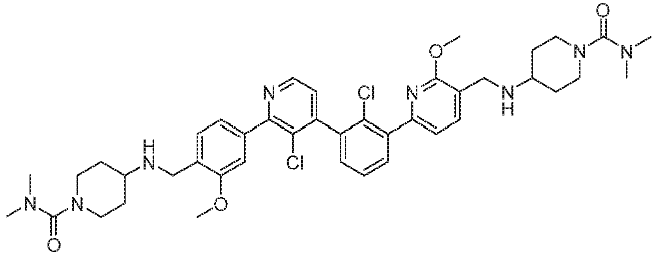

4-(((6-(2-Chloro-3-(3-chloro-2-(4-(((1-(dimethylcarbamoyl)piperidin-4-yl)amino)methyl)-3-methoxyphenyl)pyridin-4-yl)phenyl)-2-methoxypyridin-3-yl)methyl)amino)-N,N-dimethylpiperidine-1-carboxamide was prepared as the formic acid salt following Reductive Amination Procedure A from 6-(2-chloro-3-(3-chloro-2-(4-formyl-3-methoxyphenyl)pyridin-4-yl)phenyl)-2-methoxynicotinaldehyde (30 mg, 0.06 mmol) and 4-amino-N,N-dimethyl-piperidine-1-carboxamide (42 mg, 0.24 mmol). 82% yield. LCMS: m/z found 803.4 [M+H]⁺, retention time = 1.82 min (Method D). ¹H NMR (400 MHz, Methanol-*d*₄): δ 8.68 - 8.61 (m, 1H), 8.54 (s, 2H), 7.81 - 7.75 (m, 1H), 7.72 (d, 1H), 7.56 (t, 1H), 7.47 (dd, 2H), 7.42 (d, 1H), 7.34 (s, 1H), 7.31 (d, 1H), 7.27 (d, 1H), 4.13 (s, 2H), 4.03 (s, 3H), 3.97 (s, 3H), 3.94 (s, 2H), 3.79 - 3.63 (m, 4H), 3.10 - 3.00 (m, 1H), 2.92 - 2.73 (m, 17H), 2.13 - 1.93 (m, 4H), 1.63 - 1.38 (m, 4H).

### Example 187: N-((6-(2-Chloro-3-(3-chloro-2-(4-(((1-(2,2-difluoroethyl)piperidin-4-yl)amino)methyl)-3-methoxyphenyl)pyridin-4-yl)phenyl)-2-methoxypyridin-3-yl)methyl)-1-(2,2-difluoroethyl)piperidin-4-amine (420)

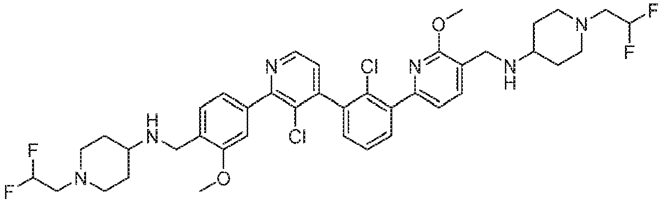

N-((6-(2-Chloro-3-(3-chloro-2-(4-(((1-(2,2-difluoroethyl)piperidin-4-yl)amino)methyl)-3-methoxyphenyl)pyridin-4-yl)phenyl)-2-methoxypyridin-3-yl)methyl)-1-(2,2-difluoroethyl)piperidin-4-amine was prepared as the formic acid salt following Reductive Amination Procedure A from 6-(2-chloro-3-(3-chloro-2-(4-formyl-3-methoxyphenyl)pyridin-4-yl)phenyl)-2-methoxynicotinaldehyde (30 mg, 0.06 mmol) and 1-(2,2-difluoroethyl)piperidin-4-amine (40 mg, 0.24 mmol). 63% yield. LCMS: m/z found 789.3 [M+H]⁺, retention time = 2.29 min (Method C). ¹H NMR (400 MHz, Methanol-*d*₄): δ 8.65 (d, 1H), 8.54 (s, 2H), 7.80 (d, 1H), 7.72 (dd, 1H), 7.56 (t, 1H), 7.50 (d, 1H), 7.47 (d, 1H), 7.42 (dd, 1H), 7.36 (d, 1H), 7.33 (dd, 1H), 7.29 (d, 1H), 5.96 (tt, 2H), 4.19 (s, 2H), 4.04 (s, 3H), 4.00 (s, 2H), 3.98 (s, 3H), 3.11 - 2.95 (m, 5H), 2.84 - 2.68 (m, 5H), 2.38 - 2.21 (m, 4H), 2.14 - 1.96 (m, 4H), 1.76 - 1.48 (m, 4H).

### Example 188: (4-(((6-(2-Chloro-3-(3-chloro-2-(4-(((1-(furan-2-carbonyl)piperidin-4-yl)amino)methyl)-3-methoxyphenyl)pyridin-4-yl)phenyl)-2-methoxypyridin-3-yl)methyl)amino)piperidin-1-yl)(furan-2-yl)methanone (421)

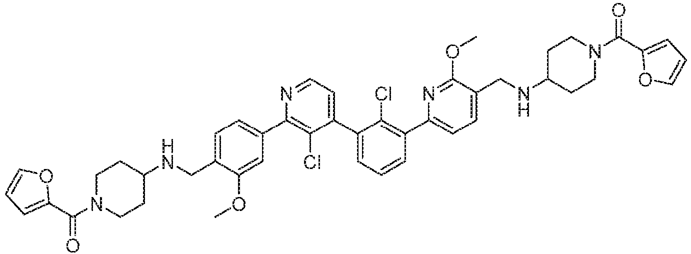

(4-(((6-(2-Chloro-3-(3-chloro-2-(4-(((1-(furan-2-carbonyl)piperidin-4-yl)amino)methyl)-3-methoxyphenyl)pyridin-4-yl)phenyl)-2-methoxypyridin-3-yl)methyl)amino)piperidin-1-yl)(furan-2-yl)methanone was prepared as the formic acid salt following Reductive Amination Procedure A from 6-(2-chloro-3-(3-chloro-2-(4-formyl-3-methoxyphenyl)pyridin-4-yl)phenyl)-2-methoxynicotinaldehyde (30 mg, 0.06 mmol) and (4-amino-1-piperidyl)-(2-furyl)methanone (47 mg, 0.24 mmol). 46% yield. LCMS: m/z found 849.3 [M+H]⁺, retention time = 1.93 min (Method D). ¹H NMR (400 MHz, Methanol-*d*₄): δ 8.67 - 8.59 (m, 1H), 8.55 (s, 2H), 7.78 (d, 1H), 7.74 - 7.66 (m, 3H), 7.58 - 7.52 (m, 1H), 7.48 - 7.43 (m, 2H), 7.41 (dd, 1H), 7.33 - 7.22 (m, 3H), 7.02 (t, 2H), 6.59 (dq, 2H), 4.60 - 4.42 (m, 4H), 4.09 - 3.99 (m, 5H), 3.95 (s, 3H), 3.90 (s, 2H), 3.28 - 2.82 (m, 6H), 2.21 - 2.04 (m, 4H), 1.58 - 1.37 (m, 4H).

### Example 189: (4-(((6-(3-(2-(4-(((1-Benzoylpiperidin-4-yl)amino)methyl)-3-methoxyphenyl)-3-chloropyridin-4-yl)-2-chlorophenyl)-2-methoxypyridin-3-yl)methyl)amino)piperidin-1-yl)(phenyl)methanone (422)

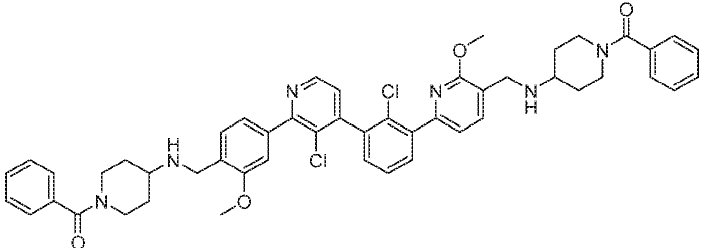

(4-(((6-(3-(2-(4-(((1-Benzoylpiperidin-4-yl)amino)methyl)-3-methoxyphenyl)-3-chloropyridin-4-yl)-2-chlorophenyl)-2-methoxypyridin-3-yl)methyl)amino)piperidin-1-yl)(phenyl)methanone was prepared as the formic acid salt following Reductive Amination Procedure A from 6-(2-chloro-3-(3-chloro-2-(4-formyl-3-methoxyphenyl)pyridin-4-yl)phenyl)-2-methoxynicotinaldehyde (30 mg, 0.06 mmol) and (4-amino-1-piperidyl)-phenyl-methanone (50 mg, 0.24 mmol). 45% yield. LCMS: m/z found 869.3 [M+H]⁺, retention time = 2.42 min (Method C). ¹H NMR (400 MHz, Methanol-*d*₄): δ 8.64 (d, 1H), 8.55 (s, 2H), 7.78 (d, 1H), 7.72 (d, 1H), 7.55 (t, 1H), 7.50 - 7.44 (m, 2H), 7.43 - 7.38 (m, 1H), 7.34 - 7.18 (m, 7H), 7.03 - 6.95 (m, 4H), 6.83 (dt, 2H), 4.08 (s, 2H), 4.04 (s, 3H), 3.96 (s, 3H), 3.93 (s, 2H), 3.73 (t, 4H), 2.97 - 2.87 (m, 1H), 2.74 (t, 5H), 2.18 - 2.05 (m, 4H), 1.75 - 1.52 (m, 4H).

### Example 190: N-((6-(2-Chloro-3-(3-chloro-2-(3-methoxy-4-(((1-phenylpiperidin-4-yl)amino)methyl)phenyl)pyridin-4-yl)phenyl)-2-methoxypyridin-3-yl)methyl)-1-phenylpiperidin-4-amine (423)

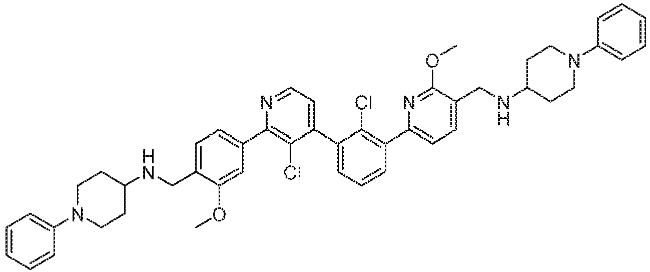

N-((6-(2-Chloro-3-(3-chloro-2-(3-methoxy-4-(((1-phenylpiperidin-4-yl)amino)methyl)phenyl)pyridin-4-yl)phenyl)-2-methoxypyridin-3-yl)methyl)-1-phenylpiperidin-4-amine was prepared as the formic acid salt following Reductive Amination Procedure A from 6-(2-chloro-3-(3-chloro-2-(4-formyl-3-methoxyphenyl)pyridin-4-yl)phenyl)-2-methoxynicotinaldehyde (30 mg, 0.06 mmol) and 1-phenylpiperidin-4-amine (43 mg, 0.24 mmol). 60% yield. LCMS: m/z found 813.3 [M+H]⁺, retention time = 2.81 min (Method C). ¹H NMR (400 MHz, Methanol-*d*₄): δ 8.63 (d, 1H), 8.54 (s, 2H), 7.76 (d, 1H), 7.71 (dd, 1H), 7.55 (t, 1H), 7.50 - 7.37 (m, 13H), 7.33 - 7.22 (m, 3H), 4.73 - 4.54 (m, 2H), 4.02 (s, 3H), 3.99 (s, 2H), 3.94 (s, 3H), 3.88 (s, 2H), 3.82 - 3.69 (m, 2H), 3.21 - 3.07 (m, 2H), 3.01 - 2.79 (m, 4H), 2.22 - 2.06 (m, 2H), 2.03 - 1.90 (m, 2H), 1.56 - 1.31 (m, 4H).

### Example 191: N-((6-(2-Chloro-3-(3-chloro-2-(3-methoxy-4-(((1-(pyridin-4-yl)piperidin-4-yl)amino)methyl)phenyl)pyridin-4-yl)phenyl)-2-methoxypyridin-3-yl)methyl)-1-(pyridin-4-yl)piperidin-4-amine (424)

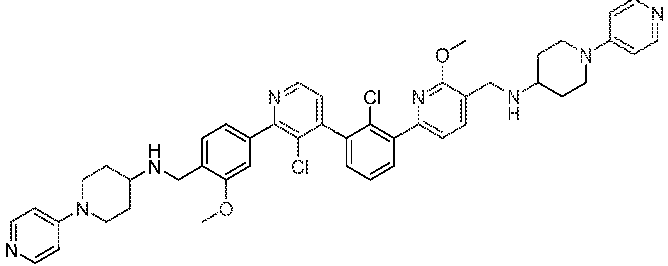

N-((6-(2-Chloro-3-(3-chloro-2-(3-methoxy-4-(((1-(pyridin-4-yl)piperidin-4-yl)amino)methyl)phenyl)pyridin-4-yl)phenyl)-2-methoxypyridin-3-yl)methyl)-1-(pyridin-4-yl)piperidin-4-amine was prepared as the formic acid salt following Reductive Amination Procedure A from 6-(2-chloro-3-(3-chloro-2-(4-formyl-3-methoxyphenyl)pyridin-4-yl)phenyl)-2-methoxynicotinaldehyde (30 mg, 0.06 mmol) and 1-(4-pyridyl)piperidin-4-amine (43 mg, 0.24 mmol). 59% yield. LCMS: m/z found 815.3 [M+H]⁺, retention time = 2.10 min (Method C). ¹H NMR (400 MHz, Methanol-*d*₄): δ 8.65 (d, 1H), 8.49 (s, 2H), 8.13 (t, 4H), 7.83 (d, 1H), 7.72 (d, 1H), 7.56 (t, 1H), 7.52 (d, 1H), 7.47 (d, 1H), 7.43 (d, 1H), 7.37 (s, 1H), 7.33 (d, 1H), 7.30 (d, 1H), 7.10 (t, 4H), 4.38 - 4.16 (m, 6H), 4.05 (s, 5H), 3.98 (s, 3H), 3.45 - 3.34 (m, 1H), 3.21 (q, 5H), 2.34 - 2.17 (m, 4H), 1.76 - 1.44 (m, 4H).

### Example 192: 2-((6-(3-(2-(4-((7-Oxa-2-azaspiro[3.5]nonan-2-yl)methyl)-3-methoxyphenyl)-3-chloropyridin-4-yl)-2-chlorophenyl)-2-methoxypyridin-3-yl)methyl)-7-oxa-2-azaspiro[3.5]nonane (470)

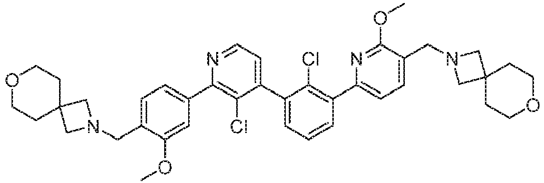

2-((6-(3-(2-(4-((7-Oxa-2-azaspiro[3.5]nonan-2-yl)methyl)-3-methoxyphenyl)-3-chloropyridin-4-yl)-2-chlorophenyl)-2-methoxypyridin-3-yl)methyl)-7-oxa-2-azaspiro[3.5]nonane was prepared as the formic acid salt following Reductive Amination Procedure A from 6-(2-chloro-3-(3-chloro-2-(4-formyl-3-methoxyphenyl)pyridin-4-yl)phenyl)-2-methoxynicotinaldehyde (30 mg, 0.06 mmol) and 7-oxa-2-azaspiro[3.5]nonane (31 mg, 0.24 mmol). 69% yield. LCMS: m/z found 715.3 [M+H]⁺, retention time = 1.79 min (Method D). ¹H NMR (400 MHz, Methanol-*d*₄): δ 8.65 (d, 1H), 8.52 (s, 2H), 7.77 (d, 1H), 7.71 (dd, 1H), 7.56 (t, 1H), 7.51 (d, 1H), 7.47 (d, 1H), 7.42 (dd, 1H), 7.39 - 7.31 (m, 2H), 7.29 (d, 1H), 4.33 (s, 2H), 4.06 - 3.99 (m, 5H), 3.97 (s, 3H), 3.86 (s, 4H), 3.66 - 3.58 (m, 8H), 3.56 (s, 4H), 1.92 - 1.77 (m, 8H).

### Example 193: N-((6-(2-Chloro-3-(3-chloro-2-(3-methoxy-4-(((1-(pyridin-2-yl)piperidin-4-yl)amino)methyl)phenyl)pyridin-4-yl)phenyl)-2-methoxypyridin-3-yl)methyl)-1-(pyridin-2-yl)piperidin-4-amine (444)

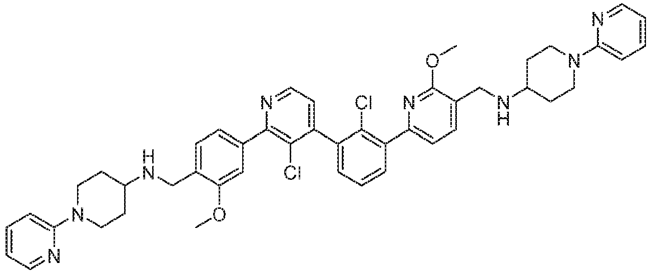

N-((6-(2-Chloro-3-(3-chloro-2-(3-methoxy-4-(((1-(pyridin-2-yl)piperidin-4-yl)amino)methyl)phenyl)pyridin-4-yl)phenyl)-2-methoxypyridin-3-yl)methyl)-1-(pyridin-2-yl)piperidin-4-amine was prepared as the formic acid salt following Reductive Amination Procedure A from 6-(2-chloro-3-(3-chloro-2-(4-formyl-3-methoxyphenyl)pyridin-4-yl)phenyl)-2-methoxynicotinaldehyde (30 mg, 0.06 mmol) and 1-(2-pyridyl)piperidin-4-amine (43 mg, 0.24 mmol). 61% yield. LCMS: m/z found 815.3 [M+H]⁺, retention time = 1.43 min (Method D). ¹H NMR (400 MHz, Methanol-*d*₄): δ 8.65 (dd, 1H), 8.54 (s, 2H), 8.09 (t, 2H), 7.82 (d, 1H), 7.72 (dd, 1H), 7.61 - 7.53 (m, 3H), 7.53 - 7.49 (m, 1H), 7.47 (dd, 1H), 7.42 (d, 1H), 7.37 (d, 1H), 7.35 - 7.27 (m, 2H), 6.88 (t, 2H), 6.73 - 6.62 (m, 2H), 4.49 - 4.28 (m, 4H), 4.23 (s, 2H), 4.11 - 4.00 (m, 5H), 3.98 (s, 3H), 3.11 - 3.00 (m, 1H), 2.93 (t, 4H), 2.26 - 2.08 (m, 4H), 1.72 - 1.47 (m, 4H).

### Example 194: N-((6-(2-Chloro-3-(3-chloro-2-(4-(((1-(3-chloropyridin-2-yl)piperidin-4-yl)amino)methyl)-3-methoxyphenyl)pyridin-4-yl)phenyl)-2-methoxypyridin-3-yl)methyl)-1-(3-chloropyridin-2-yl)piperidin-4-amine (445)

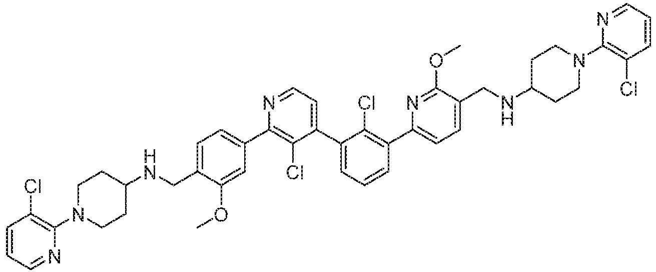

N-((6-(2-Chloro-3-(3-chloro-2-(4-(((1-(3-chloropyridin-2-yl)piperidin-4-yl)amino)methyl)-3-methoxyphenyl)pyridin-4-yl)phenyl)-2-methoxypyridin-3-yl)methyl)-1-(3-chloropyridin-2-yl)piperidin-4-amine was prepared as the formic acid salt following Reductive Amination Procedure A from 6-(2-chloro-3-(3-chloro-2-(4-formyl-3-methoxyphenyl)pyridin-4-yl)phenyl)-2-methoxynicotinaldehyde (30 mg, 0.06 mmol) and 1-(3-chloro-2-pyridyl)piperidin-4-amine (51 mg, 0.24 mmol). 56% yield. LCMS: m/z found 883.3 [M+H]⁺, retention time = 2.49 min (Method D). ¹H NMR (400 MHz, Methanol-*d*₄): δ 8.66 (dt, 1H), 8.56 - 8.49 (m, 2H), 8.21 - 8.13 (m, 2H), 7.83 (d, 1H), 7.77 - 7.69 (m, 3H), 7.60 - 7.50 (m, 2H), 7.47 (dt, 1H), 7.45 - 7.40 (m, 1H), 7.38 (s, 1H), 7.34 (d, 1H), 7.30 (dd, 1H), 7.00 - 6.91 (m, 2H), 4.25 (s, 2H), 4.11 - 4.03 (m, 5H), 3.99 (t, 3H), 3.96 - 3.80 (m, 4H), 3.21 (d, 1H), 2.99 (s, 1H), 2.90 (q, 4H), 2.23 (d, 2H), 2.16 (d, 2H), 1.90 - 1.62 (m, 4H).

### Example 195: N-((6-(2-chloro-3-(3-chloro-2-(3-methoxy-4-(((2-methoxyethyl)amino)methyl)phenyl)pyridin-4-yl)phenyl)-2-methoxypyridin-3-yl)methyl)-2-methoxyethan-1-amine (446)

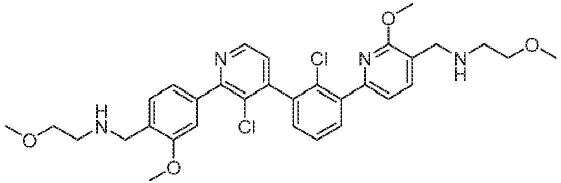

N-((6-(2-chloro-3-(3-chloro-2-(3-methoxy-4-(((2-methoxyethyl)amino)methyl)phenyl)pyridin-4-yl)phenyl)-2-methoxypyridin-3-yl)methyl)-2-methoxyethan-1-amine was prepared as the formic acid salt following Reductive Amination Procedure A from 6-(2-chloro-3-(3-chloro-2-(4-formyl-3-methoxyphenyl)pyridin-4-yl)phenyl)-2-methoxynicotinaldehyde (30 mg, 0.06 mmol) and 2-methoxyethanamine (18 mg, 0.24 mmol). 54% yield. LCMS: m/z found 611.2 [M+H]⁺, retention time = 1.67 min (Method D). ¹H NMR (400 MHz, Methanol-*d*₄): δ 8.72 - 8.63 (m, 1H), 8.57 - 8.49 (m, 2H), 7.82 (d, 1H), 7.72 (dd, 1H), 7.57 (t, 1H), 7.52 - 7.45 (m, 2H), 7.43 (dd, 1H), 7.38 (d, 1H), 7.36 - 7.29 (m, 2H), 4.25 (s, 2H), 4.10 (s, 2H), 4.08 - 4.03 (m, 3H), 3.98 (s, 3H), 3.70 - 3.57 (m, 4H), 3.42 (s, 3H), 3.40 (s, 3H), 3.18 (t, 2H), 3.08 (t, 2H).

### Example 196: (4-(((6-(2-Chloro-3-(3-chloro-2-(4-(((1-(cyclohexanecarbonyl)piperidin-4-yl)amino)methyl)-3-methoxyphenyl)pyridin-4-yl)phenyl)-2-methoxypyridin-3-yl)methyl)amino)piperidin-1-yl)(cyclohexyl)methanone (456)

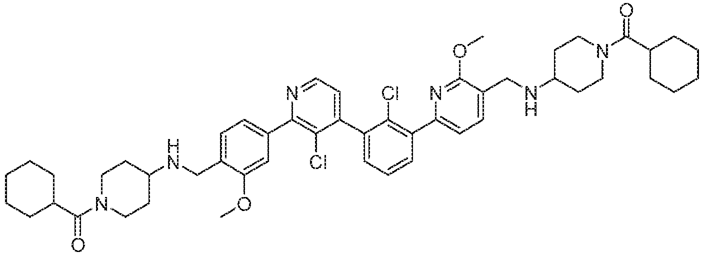

(4-(((6-(2-Chloro-3-(3-chloro-2-(4-(((1-(cyclohexanecarbonyl)piperidin-4-yl)amino)methyl)-3-methoxyphenyl)pyridin-4-yl)phenyl)-2-methoxypyridin-3-yl)methyl)amino)piperidin-1-yl)(cyclohexyl)methanone was prepared as the formic acid salt following Reductive Amination Procedure A from 6-(2-chloro-3-(3-chloro-2-(4-formyl-3-methoxyphenyl)pyridin-4-yl)phenyl)-2-methoxynicotinaldehyde (30 mg, 0.06 mmol) and (4-amino-1-piperidyl)-cyclohexyl-methanone (51 mg, 0.24 mmol). 75% yield. LCMS: m/z found 881.4 [M+H]⁺, retention time = 2.31 min (Method D). ¹H NMR (400 MHz, Methanol-*d*₄): δ 8.63 (dd, 1H), 8.55 (s, 2H), 7.76 (d, 1H), 7.73 - 7.68 (m, 1H), 7.58 - 7.51 (m, 1H), 7.47 - 7.38 (m, 3H), 7.35 - 7.19 (m, 3H), 4.52 (t, 2H), 4.12 - 4.03 (m, 2H), 4.02 (s, 3H), 3.99 (s, 2H), 3.94 (s, 3H), 3.88 (s, 2H), 3.12 (t, 2H), 2.99 - 2.88 (m, 1H), 2.87 - 2.78 (m, 1H), 2.74 - 2.57 (m, 4H), 2.19 - 1.89 (m, 4H), 1.88 - 1.62 (m, 10H), 1.55 - 1.16 (m, 14H).

### Example 197: N-((6-(2-Chloro-3-(3-chloro-2-(3-methoxy-4-(((tetrahydro-2H-thiopyran-4-yl)amino)methyl)phenyl)pyridin-4-yl)phenyl)-2-methoxypyridin-3-yl)methyl)tetrahydro-2H-thiopyran-4-amine (484)

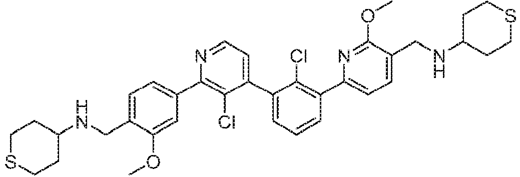

N-((6-(2-Chloro-3-(3-chloro-2-(3-methoxy-4-(((tetrahydro-2H-thiopyran-4-yl)amino)methyl)phenyl)pyridin-4-yl)phenyl)-2-methoxypyridin-3-yl)methyl)tetrahydro-2H-thiopyran-4-amine was prepared as the formic acid salt following Reductive Amination Procedure A from 6-(2-chloro-3-(3-chloro-2-(4-formyl-3-methoxyphenyl)pyridin-4-yl)phenyl)-2-methoxynicotinaldehyde (30 mg, 0.06 mmol) and tetrahydrothiopyran-4-amine (29 mg, 0.24 mmol). 71% yield. LCMS: m/z found 695.2 [M+H]⁺, retention time = 2.12 min (Method D). ¹H NMR (400 MHz, Methanol-*d*₄): δ 8.63 (d, 1H), 8.55 (s, 2H), 7.74 (d, 1H), 7.71 (dd, 1H), 7.55 (t, 1H), 7.47 - 7.37 (m, 3H), 7.32 - 7.21 (m, 3H), 4.02 (s, 3H), 3.96 (s, 2H), 3.94 (s, 3H), 3.86 (s, 2H), 2.78 - 2.59 (m, 9H), 2.58 - 2.49 (m, 1H), 2.35 - 2.20 (m, 4H), 1.71 - 1.46 (m, 4H).

### Example 198: 1-(2-((6-(3-(2-(4-((7-Acetyl-2,7-diazaspiro[3.5]nonan-2-yl)methyl)-3-methoxyphenyl)-3-chloropyridin-4-yl)-2-chlorophenyl)-2-methoxypyridin-3-yl)methyl)-2,7-diazaspiro[3.5]nonan-7-yl)ethan-1-one (485)

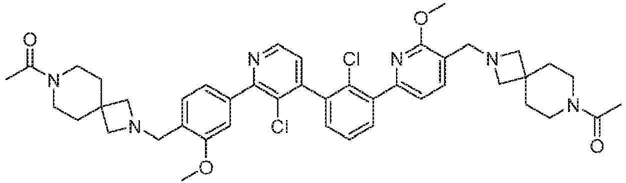

1-(2-((6-(3-(2-(4-((7-Acetyl-2,7-diazaspiro[3.5]nonan-2-yl)methyl)-3-methoxyphenyl)-3-chloropyridin-4-yl)-2-chlorophenyl)-2-methoxypyridin-3-yl)methyl)-2,7-diazaspiro[3.5]nonan-7-yl)ethan-1-one was prepared as the formic acid salt following Reductive Amination Procedure A from 6-(2-chloro-3-(3-chloro-2-(4-formyl-3-methoxyphenyl)pyridin-4-yl)phenyl)-2-methoxynicotinaldehyde (30 mg, 0.06 mmol) and 1-(2,7-diazaspiro[3.5]nonan-7-yl)ethanone (41 mg, 0.24 mmol). 82% yield. LCMS: m/z found 797.3 [M+H]⁺, retention time = 1.77 min (Method D). ¹H NMR (400 MHz, Methanol-*d*₄): δ 8.66 - 8.61 (m, 1H), 8.54 (s, 2H), 7.75 - 7.66 (m, 2H), 7.58 - 7.51 (m, 1H), 7.47 - 7.38 (m, 3H), 7.33 - 7.28 (m, 2H), 7.28 - 7.23 (m, 1H), 4.07 (s, 2H), 4.03 - 3.97 (m, 3H), 3.96 - 3.90 (m, 3H), 3.83 (s, 2H), 3.62 - 3.41 (m, 12H), 3.38 - 3.32 (m, 4H), 2.13 - 2.04 (m, 6H), 1.90 - 1.80 (m, 4H), 1.81 - 1.73 (m, 4H).

### Example 199: N-((6-(2-Chloro-3-(3-chloro-2-(4-((cyclohexylamino)methyl)-3-methoxyphenyl)pyridin-4-yl)phenyl)-2-methoxypyridin-3-yl)methyl)cyclohexanamine (524)

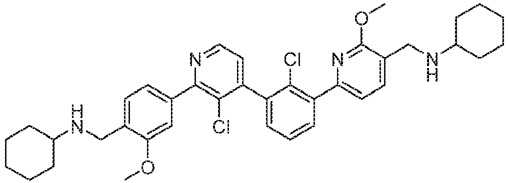

N-((6-(2-Chloro-3-(3-chloro-2-(4-((cyclohexylamino)methyl)-3-methoxyphenyl)pyridin-4-yl)phenyl)-2-methoxypyridin-3-yl)methyl)cyclohexanamine was prepared as the formic acid salt following Reductive Amination Procedure A from 6-(2-chloro-3-(3-chloro-2-(4-formyl-3-methoxyphenyl)pyridin-4-yl)phenyl)-2-methoxynicotinaldehyde (30 mg, 0.06 mmol) and cyclohexanamine (24 mg, 0.24 mmol). 62% yield. LCMS: m/z found 659.3 [M+H]⁺, retention time = 2.37 min (Method D). ¹H NMR (400 MHz, Methanol-*d*₄): δ 8.63 (d, 1H), 8.55 (s, 2H), 7.73 (dd, 2H), 7.55 (t, 1H), 7.48 - 7.39 (m, 3H), 7.35 - 7.20 (m, 3H), 4.03 (s, 5H), 3.95 (s, 3H), 3.88 (s, 2H), 2.78 - 2.67 (m, 1H), 2.63 - 2.48 (m, 1H), 2.09 - 1.96 (m, 4H), 1.87 - 1.74 (m, 4H), 1.74 - 1.61 (m, 2H), 1.40 - 1.10 (m, 10H).

### Example 200: (1r,4r)-4-(((6-(2-Chloro-3-(3-chloro-2-(4-((((1r,4r)-4-hydroxycyclohexyl)amino)methyl)-3-methoxyphenyl)pyridin-4-yl)phenyl)-2-methoxypyridin-3-yl)methyl)amino)cyclohexan-1-ol (364)

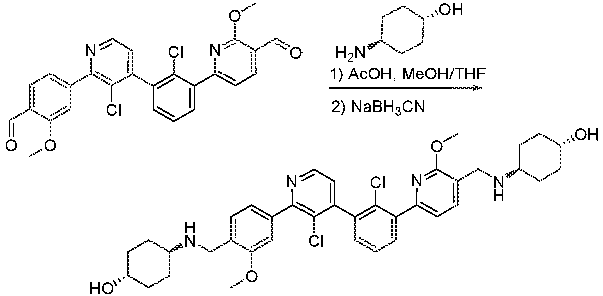

(1r,4r)-4-(((6-(2-Chloro-3-(3-chloro-2-(4-((((1r,4r)-4-hydroxycyclohexyl)amino)methyl)-3-methoxyphenyl)pyridin-4-yl)phenyl)-2-methoxypyridin-3-yl)methyl)amino)cyclohexan-1-ol was prepared using Reductive Amination Procedure A from 6-(2-chloro-3-(3-chloro-2-(4-formyl-3-methoxyphenyl)pyridin-4-yl)phenyl)-2-methoxynicotinaldehyde (20 mg, 0.04 mmol), acetic acid (5 uL, 0.08 mmol), 4-aminocyclohexanol (18 mg, 0.16 mmol) and sodium cyanoborohydride (7 mg, 0.12 mmol). 13 mg, 48% yield. MS: m/z found 691.2 [M+H]⁺, retention time = 1.69 min (Method D). ¹H NMR (400 MHz, Methanol-*d₄*) δ 8.62 (m, 1H), 7.77 - 7.67 (m, 2H), 7.59 - 7.50 (m, 1H), 7.47 - 7.36 (m, 3H), 7.34 - 7.21 (m, 3H), 4.02 (q, 5H), 3.94 (t, 3H), 3.87 (s, 2H), 3.53 (s, 2H), 2.72 (s, 1H), 2.56 (s, 1H), 2.16 - 1.90 (m, 8H), 1.41 - 1.19 (m, 8H).

### Example 201: N-(4-(4-(3-(5-(((2-Oxaspiro[3.3]heptan-6-yl)amino)methyl)-6-methoxypyridin-2-yl)-2-chlorophenyl)-3-chloropyridin-2-yl)-2-methoxybenzyl)-2-oxaspiro[3.3]heptan-6-amine (365)

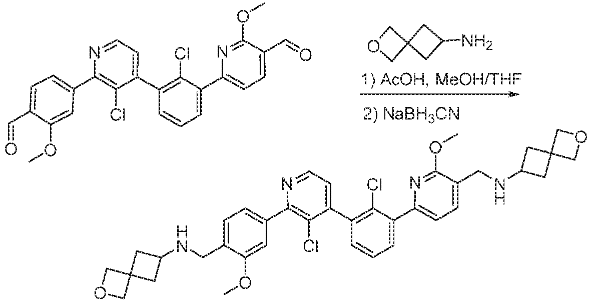

N-(4-(4-(3-(5-(((2-Oxaspiro[3.3]heptan-6-yl)amino)methyl)-6-methoxypyridin-2-yl)-2-chlorophenyl)-3-chloropyridin-2-yl)-2-methoxybenzyl)-2-oxaspiro[3.3]heptan-6-amine was prepared using Reductive Amination Procedure A from 6-(2-chloro-3-(3-chloro-2-(4-formyl-3-methoxyphenyl)pyridin-4-yl)phenyl)-2-methoxynicotinaldehyde (20 mg, 0.04 mmol), acetic acid (5 uL, 0.08 mmol), 2-oxaspiro[3.3]heptan-6-amine;hydrochloride (24 mg, 0.16 mmol) and sodium cyanoborohydride (7 mg, 0.12 mmol). 7.3 mg, 27% yield. MS: m/z found 687.2 [M+H]⁺, retention time = 1.77 min (Method D). ¹H NMR (400 MHz, Methanol-*d₄*) δ 8.61 (m, 1H), 7.69 (d, 2H), 7.58 - 7.50 (m, 1H), 7.45 - 7.34 (m, 3H), 7.28 - 7.19 (m, 3H), 4.69 (d, 4H), 4.58 (s, 4H), 4.00 (t, 3H), 3.92 (t, 3H), 3.78 (s, 2H), 3.69 (s, 2H), 3.25 - 3.11 (m, 2H), 2.52 (m, 4H), 2.01 (q, 4H).

### Example 202: (1r,3r)-3-(((6-(2-Chloro-3-(3-chloro-2-(4-((((1r,3r)-3-hydroxy-3-methylcyclobutyl)amino)methyl)-3-methoxyphenyl)pyridin-4-yl)phenyl)-2-methoxypyridin-3-yl)methyl)amino)-1-methylcyclobutan-1-ol (385)

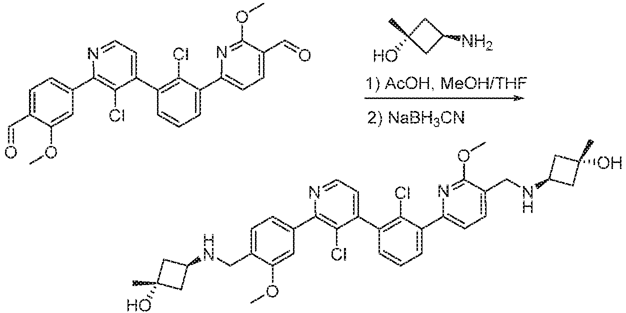

(1r,3r)-3-(((6-(2-Chloro-3-(3-chloro-2-(4-((((1r,3r)-3-hydroxy-3-methylcyclobutyl)amino)methyl)-3-methoxyphenyl)pyridin-4-yl)phenyl)-2-methoxypyridin-3-yl)methyl)amino)-1-methylcyclobutan-1-ol was prepared using Reductive Amination Procedure A from 6-(2-chloro-3-(3-chloro-2-(4-formyl-3-methoxyphenyl)pyridin-4-yl)phenyl)-2-methoxynicotinaldehyde (20 mg, 0.04 mmol), acetic acid (5 uL, 0.08 mmol), 3-amino-1-methyl-cyclobutanol;hydrochloride (22 mg, 0.16 mmol) and sodium cyanoborohydride (7 mg, 0.12 mmol). 8.5 mg, 33% yield. MS: m/z found 663.2 [M+H]⁺ , retention time = 1.69 min (Method D). ¹H NMR (400 MHz, Methanol-*d₄*) δ 8.64 (d, 1H), 7.75 (d, 1H), 7.73 - 7.67 (m, 1H), 7.55 (t, 1H), 7.46 (d, 2H), 7.43 - 7.39 (m, 1H), 7.34 (s, 1H), 7.31 (d, 1H), 7.26 (d, 1H), 4.06 - 4.01 (m, 5H), 3.97 (d, 3H), 3.84 (s, 2H), 3.78 (t, 1H), 3.65 - 3.57 (m, 1H), 2.39 - 2.24 (m, 4H), 2.09 (t, 2H), 1.95 (t, 2H), 1.35 (dd, 6H).

### Example 203: 2-((6-(2-Chloro-3-(3-chloro-2-(4-((6-hydroxy-2-azaspiro[3.3]heptan-2-yl)methyl)-3-methoxyphenyl)pyridin-4-yl)phenyl)-2-methoxypyridin-3-yl)methyl)-2-azaspiro[3.3]heptan-6-ol (386)

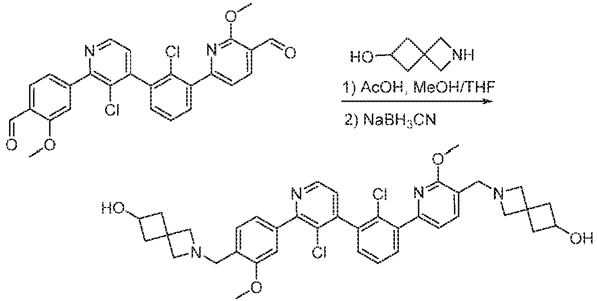

2-((6-(2-Chloro-3-(3-chloro-2-(4-((6-hydroxy-2-azaspiro[3.3]heptan-2-yl)methyl)-3-methoxyphenyl)pyridin-4-yl)phenyl)-2-methoxypyridin-3-yl)methyl)-2-azaspiro[3.3]heptan-6-ol was prepared using Reductive Amination Procedure A from 6-(2-chloro-3-(3-chloro-2-(4-formyl-3-methoxyphenyl)pyridin-4-yl)phenyl)-2-methoxynicotinaldehyde (20 mg, 0.04 mmol), acetic acid (5 uL, 0.08 mmol), 2-azaspiro[3.3]heptan-6-ol (18 mg, 0.16 mmol) and sodium cyanoborohydride (7 mg, 0.12 mmol). 4.3mg, 16% yield. MS: m/z found 687.2 [M+H]⁺, retention time = 1.71 min (Method D). ¹H NMR (400 MHz, Methanol-*d₄*) δ 8.64 (d, 1H), 7.78 - 7.67 (m, 2H), 7.55 (t, 1H), 7.50 - 7.39 (m, 3H), 7.36 (s, 1H), 7.34 - 7.25 (m, 2H), 4.28 (s, 2H), 4.11 (q, 2H), 4.04 - 4.01 (m, 5H), 4.00 (s, 4H), 3.96 (t, 3H), 3.74 (d, 4H), 2.67 - 2.50 (m, 4H), 2.19 - 2.00 (m, 4H).

### Example 204: 6-((6-(3-(2-(4-((2-Oxa-6-azaspiro[3.3]heptan-6-yl)methyl)-3-methoxyphenyl)-3-chloropyridin-4-yl)-2-chlorophenyl)-2-methoxypyridin-3-yl)methyl)-2-oxa-6-azaspiro[3.3]heptane (387)

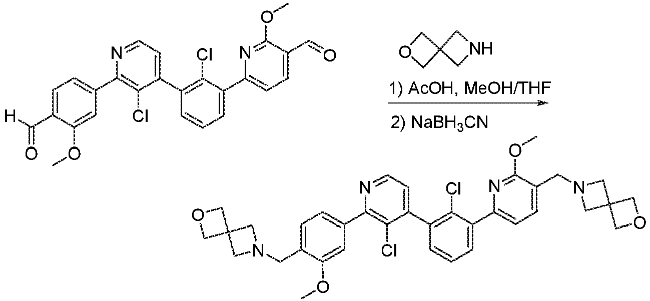

6-((6-(3-(2-(4-((2-Oxa-6-azaspiro[3.3]heptan-6-yl)methyl)-3-methoxyphenyl)-3-chloropyridin-4-yl)-2-chlorophenyl)-2-methoxypyridin-3-yl)methyl)-2-oxa-6-azaspiro[3.3]heptane was prepared using Reductive Amination Procedure A from 6-(2-chloro-3-(3-chloro-2-(4-formyl-3-methoxyphenyl)pyridin-4-yl)phenyl)-2-methoxynicotinaldehyde (20 mg, 0.04 mmol), acetic acid (5 uL, 0.08 mmol), 2-oxa-6-azaspiro[3.3]heptane (16 mg, 0.16 mmol) and sodium cyanoborohydride (7 mg, 0.12 mmol). 6.2 mg, 24% yield. MS: m/z found 659.2 [M+H]⁺, retention time = 1.70 min (Method D). ¹H NMR (400 MHz, Methanol-*d₄*) δ 8.61 (dd, 1H), 7.67 (dd, 2H), 7.53 (t, 1H), 7.44 - 7.37 (m, 2H), 7.34 (d, 1H), 7.28 - 7.19 (m, 3H), 4.74 (dd, 8H), 3.98 (t, 3H), 3.89 (d, 3H), 3.73 (s, 2H), 3.64 (s, 2H), 3.56 (s, 4H), 3.53 (s, 4H).

### Example 205: 1-((6-(2-Chloro-3-(3-chloro-2-(4-((3-hydroxy-3-methylazetidin-1-yl)methyl)-3-methoxyphenyl)pyridin-4-yl)phenyl)-2-methoxypyridin-3-yl)methyl)-3-methylazetidin-3-ol (396)

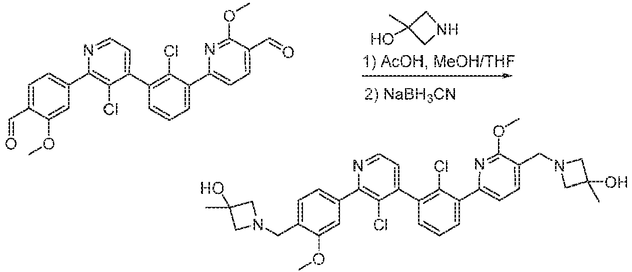

1-((6-(2-Chloro-3-(3-chloro-2-(4-((3-hydroxy-3-methylazetidin-1-yl)methyl)-3-methoxyphenyl)pyridin-4-yl)phenyl)-2-methoxypyridin-3-yl)methyl)-3-methylazetidin-3-ol was prepared using Reductive Amination Procedure A from 6-(2-chloro-3-(3-chloro-2-(4-formyl-3-methoxyphenyl)pyridin-4-yl)phenyl)-2-methoxynicotinaldehyde (20 mg, 0.04 mmol), acetic acid (5 uL, 0.08 mmol), 3-methylazetidin-3-ol;hydrochloride (19 mg, 0.16 mmol) and sodium cyanoborohydride (7 mg, 0.12 mmol). 4.7 mg, 19% yield. MS: m/z found 635.1 [M+H]⁺, retention time = 0.65 min (Method B). ¹H NMR (400 MHz, Methanol-*d₄*). δ 8.65 (d, 1H), 7.78 (d, 1H), 7.73 - 7.68 (m, 1H), 7.56 (dd, 1H), 7.51 - 7.40 (m, 3H), 7.37 (s, 1H), 7.32 (dd, 2H), 4.38 (s, 2H), 4.09 (s, 2H), 4.06 - 4.00 (m, 5H), 3.97 (t, 3H), 3.90 (d, 2H), 3.79 (d, 2H), 3.62 (d, 2H), 1.51 (q, 6H).

### Example 206: 2-((6-(2-Chloro-3-(3-chloro-2-(3-methoxy-4-((6-oxo-2,5-diazaspiro[3.4]octan-2-yl)methyl)phenyl)pyridin-4-yl)phenyl)-2-methoxypyridin-3-yl)methyl)-2,5-diazaspiro[3.4]octan-6-one (398)

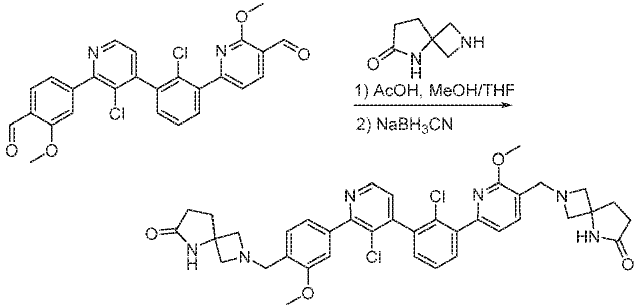

2-((6-(2-Chloro-3-(3-chloro-2-(3-methoxy-4-((6-oxo-2,5-diazaspiro[3.4]octan-2-yl)methyl)phenyl)pyridin-4-yl)phenyl)-2-methoxypyridin-3-yl)methyl)-2,5-diazaspiro[3.4]octan-6-one was prepared using Reductive Amination Procedure A from 6-(2-chloro-3-(3-chloro-2-(4-formyl-3-methoxyphenyl)pyridin-4-yl)phenyl)-2-methoxynicotinaldehyde (20 mg, 0.04 mmol), acetic acid (5 uL, 0.08 mmol), 2,5-diazaspiro[3.4]octan-6-one (20 mg, 0.16 mmol) and sodium cyanoborohydride (7 mg, 0.12 mmol). 5.7 mg, 20% yield. MS: m/z found 715.1 [M+H]⁺, retention time = 1.57 min (Method D). ¹H NMR (400 MHz, Methanol-*d₄*) δ 9.99 (t, 1H), 9.07 (t, 2H), 8.91 (t, 1H), 8.84 - 8.74 (m, 3H), 8.70 - 8.60 (m, 3H), 5.41 (s, 2H), 5.37 (t, 3H), 5.30 (d, 3H), 5.21 (d, 2H), 5.16 (s, 2H), 5.10 (d, 2H), 5.01 (d, 2H), 4.83 (s, 2H), 3.80 (d, 4H), 3.73 (d, 4H).

### Example 207: (1s,4s)-4-(((6-(2-Chloro-3-(3-chloro-2-(4-((((1s,4s)-4-hydroxycyclohexyl)amino)methyl)-3-methoxyphenyl)pyridin-4-yl)phenyl)-2-methoxypyridin-3-yl)methyl)amino)cyclohexan-1-ol (410)

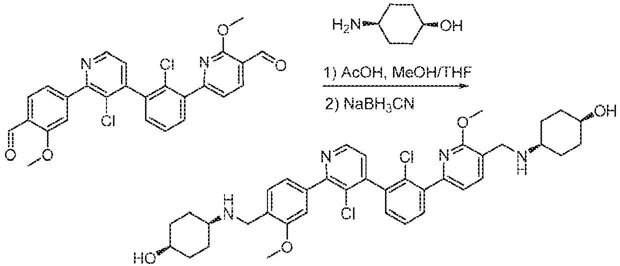

(1s,4s)-4-(((6-(2-Chloro-3-(3-chloro-2-(4-((((1s,4s)-4-hydroxycyclohexyl)amino)methyl)-3-methoxyphenyl)pyridin-4-yl)phenyl)-2-methoxypyridin-3-yl)methyl)amino)cyclohexan-1-ol was prepared using Reductive Amination Procedure A from 6-(2-chloro-3-(3-chloro-2-(4-formyl-3-methoxyphenyl)pyridin-4-yl)phenyl)-2-methoxynicotinaldehyde (20 mg, 0.04 mmol), acetic acid (5 uL, 0.08 mmol), 4-aminocyclohexanol (18 mg, 0.16 mmol) and sodium cyanoborohydride (7 mg, 0.12 mmol). 21 mg, 77% yield. MS: m/z found 691.2 [M+H]⁺, retention time = 1.75 min (Method D). ¹H NMR (400 MHz, Methanol-*d₄*) δ 8.66 (d, 1H), 7.90 (d, 1H), 7.72 (d, 1H), 7.61 - 7.51 (m, 2H), 7.45 (dd, 2H), 7.38 (s, 1H), 7.34 (dd, 2H), 4.29 (d, 4H), 4.08 (s, 3H), 3.98 (s, 3H), 3.20 (q, 2H), 2.05 - 1.82 (m, 14H), 1.71 - 1.51 (m, 4H).

### Example 208: 1-((((6-(2-Chloro-3-(3-chloro-2-(4-((((1-hydroxycyclopropyl)methyl)amino)methyl)-3-methoxyphenyl)pyridin-4-yl)phenyl)-2-methoxypyridin-3-yl)methyl)amino)methyl)cyclopropan-1-ol (504)

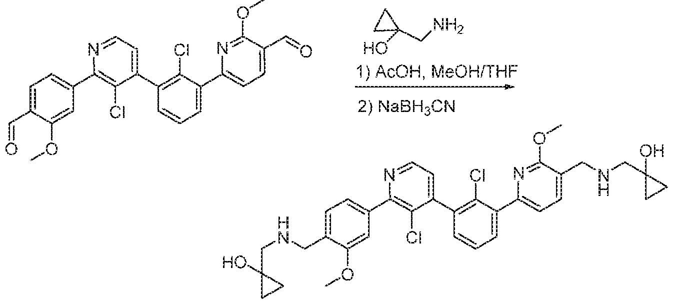

1-((((6-(2-Chloro-3-(3-chloro-2-(4-((((1-hydroxycyclopropyl)methyl)amino)methyl)-3-methoxyphenyl)pyridin-4-yl)phenyl)-2-methoxypyridin-3-yl)methyl)amino)methyl)cyclopropan-1-ol was prepared using Reductive Amination Procedure A from 6-(2-chloro-3-(3-chloro-2-(4-formyl-3-methoxyphenyl)pyridin-4-yl)phenyl)-2-methoxynicotinaldehyde (20 mg, 0.04 mmol), acetic acid (5 uL, 0.08 mmol), 1-(aminomethyl)cyclopropanol (14 mg, 0.16 mmol) and sodium cyanoborohydride (7 mg, 0.12 mmol). 6.5 mg, 26% yield. MS: m/z found 635.3 [M+H]⁺, retention time = 1.73 min (Method D). ¹H NMR (400 MHz, Methanol-*d₄*) δ 8.66 (m, 1H), 7.90 (d, 1H), 7.75 - 7.69 (m, 1H), 7.58 (dd, 1H), 7.53 (d, 1H), 7.49 - 7.42 (m, 2H), 7.39 (s, 1H), 7.37 - 7.32 (m, 2H), 4.38 (d, 4H), 4.08 (t, 3H), 3.99 (t, 3H), 3.19 (s, 2H), 3.16 (s, 2H), 0.90 (d, 4H), 0.73 (d, 4H).

### Example 209: N-((6-(2-Chloro-3-(3-chloro-2-(4-(((2,2-difluoroethyl)amino)methyl)-3-methoxyphenyl)pyridin-4-yl)phenyl)-2-methoxypyridin-3-yl)methyl)-2,2-difluoroethan-1-amine (505)

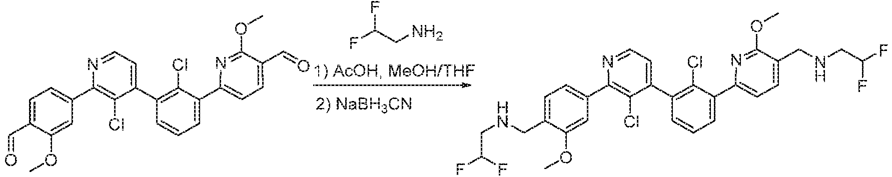

N-((6-(2-Chloro-3-(3-chloro-2-(4-(((2,2-difluoroethyl)amino)methyl)-3-methoxyphenyl)pyridin-4-yl)phenyl)-2-methoxypyridin-3-yl)methyl)-2,2-difluoroethan-1-amine was prepared using Reductive Amination Procedure A from 6-(2-chloro-3-(3-chloro-2-(4-formyl-3-methoxyphenyl)pyridin-4-yl)phenyl)-2-methoxynicotinaldehyde (20 mg, 0.04 mmol), acetic acid (5 uL, 0.08 mmol), 2,2-difluoroethanamine (13 mg, 0.16 mmol) and sodium cyanoborohydride (8 mg, 0.12 mmol). 11.6 mg, 46% yield. MS: m/z found 623.1 [M+H]⁺, retention time = 1.78 min (Method D). ¹H NMR (400 MHz, Methanol-*d₄*) δ 8.66 - 8.59 (m, 1H), 8.27 (d, 1H), 7.70 (dd, 1H), 7.58 - 7.49 (m, 1H), 7.46 - 7.41 (m, 2H), 7.41 - 7.37 (m, 1H), 7.31 (dd, 2H), 7.26 (dd, 1H), 6.39 - 5.67 (m, 1H), 4.66 - 4.59 (m, 1H), 4.08 (s, 2H), 4.01 (m, 3H), 3.94 (t, 3H), 3.91 (m, 2H), 3.59 (q, 1H), 3.16 (dd, 2H), 3.09 - 2.95 (m, 1H).

### Example 210: N-((6-(2-Chloro-3-(3-chloro-2-(4-((isopropylamino)methyl)-3-methoxyphenyl)pyridin-4-yl)phenyl)-2-methoxypyridin-3-yl)methyl)propan-2-amine (514)

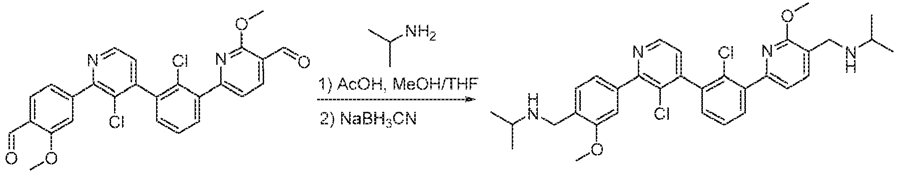

N-((6-(2-Chloro-3-(3-chloro-2-(4-((isopropylamino)methyl)-3-methoxyphenyl)pyridin-4-yl)phenyl)-2-methoxypyridin-3-yl)methyl)propan-2-amine was prepared using Reductive Amination Procedure A from 6-(2-chloro-3-(3-chloro-2-(4-formyl-3-methoxyphenyl)pyridin-4-yl)phenyl)-2-methoxynicotinaldehyde (20 mg, 0.04 mmol), acetic acid (5 uL, 0.08 mmol), propan-2-amine (13 mg, 0.16 mmol) and sodium cyanoborohydride (8 mg, 0.12 mmol). 9.8 mg, 42% yield. MS: m/z found 579.3 [M+H]⁺ , retention time = 0.67 min (Method B). ¹H NMR (400 MHz, Methanol-*d₄*) δ 8.66 (dd, 1H), 7.90 (d, 1H), 7.76 - 7.68 (m, 1H), 7.61 - 7.51 (m, 2H), 7.48 - 7.42 (m, 2H), 7.38 (s, 1H), 7.34 (dd, 2H), 4.26 (d, 4H), 4.08 (d, 3H), 3.99 (d, 3H), 3.48 (s, 2H), 1.42 (m, 12H).

### Example 211: 1-(1-((6-(3-(2-(4-((4-Acetylpiperidin-1-yl)methyl)-3-methoxyphenyl)-3-chloropyridin-4-yl)-2-chlorophenyl)-2-methoxypyridin-3-yl)methyl)piperidin-4-yl)ethan-1-one (536)

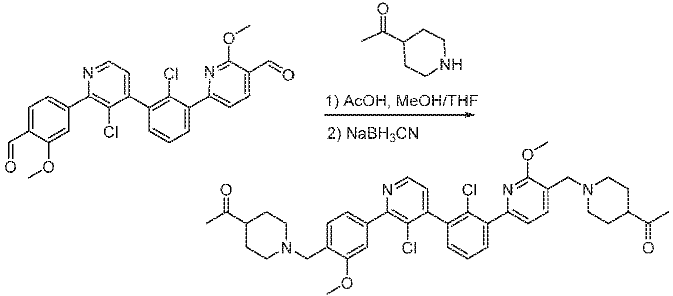

1-(1-((6-(3-(2-(4-((4-Acetylpiperidin-1-yl)methyl)-3-methoxyphenyl)-3-chloropyridin-4-yl)-2-chlorophenyl)-2-methoxypyridin-3-yl)methyl)piperidin-4-yl)ethan-1-one was prepared using Reductive Amination Procedure A from of 6-(2-chloro-3-(3-chloro-2-(4-formyl-3-methoxyphenyl)pyridin-4-yl)phenyl)-2-methoxynicotinaldehyde (20 mg, 0.04 mmol), acetic acid (5 uL, 0.08 mmol), 1-(4-piperidyl)ethanone (21 mg, 0.16 mmol) and sodium cyanoborohydride (8 mg, 0.12 mmol). 5.3 mg, 18% yield. MS: m/z found 717.3 [M+H]⁺, retention time = 2.33 min (Method C). ¹H NMR (400 MHz, Methanol-*d₄*) δ 8.67 (d, 1H), 7.91 (d, 1H), 7.74 (d, 1H), 7.59 - 7.52 (m, 2H), 7.49 - 7.45 (m, 1H), 7.41 (s, 1H), 7.37 (d, 2H), 4.37 (s, 2H), 4.25 (s, 2H), 4.06 (d, 3H), 3.98 (d, 3H), 3.63 - 3.41 (m, 4H), 3.21 - 2.97 (m, 4H), 2.75 (d, 1H), 2.20 (t, 5H), 2.13 (t, 3H), 1.85 (s, 3H), 1.57 (s, 1H).

### Example 212: N-(1-((6-(3-(2-(4-((4-Acetamidopiperidin-1-yl)methyl)-3-methoxyphenyl)-3-chloropyridin-4-yl)-2-chlorophenyl)-2-methoxypyridin-3-yl)methyl)piperidin-4-yl)acetamide (322)

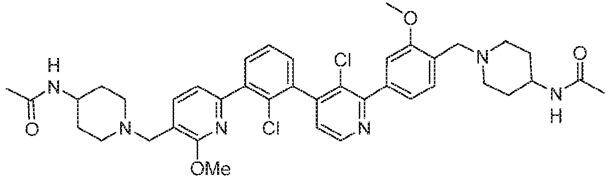

N-(1-((6-(3-(2-(4-((4-Acetamidopiperidin-1-yl)methyl)-3-methoxyphenyl)-3-chloropyridin-4-yl)-2-chlorophenyl)-2-methoxypyridin-3-yl)methyl)piperidin-4-yl)acetamide was prepared in similar manner as described for Example 11, replacing (5S)-5-(aminomethyl)pyrrolidin-2-one with N-(4-piperidyl)-acetamide in step (b). Product was obtained as a white solid (formic acid salt). LC/MS: *m*/*z* found 745, 747 [M+H]⁺, retention time = 2.12 min (Method C). ¹H NMR (400 MHz, Methanol-*d*₄) δ 8.64 (d, 1H), 8.49 (s, 1H), 7.79 (d, 1H), 7.72 (d, 1H), 7.59 - 7.49 (m, 2H), 7.46 (d, 1H), 7.41 (d, 1H), 7.39 - 7.31 (m, 2H), 7.28 (d, 1H), 4.09 (s, 2H), 4.00 (d, 3H), 3.95 (d, 3H), 3.87 - 3.63 (m, 4H), 3.06 (d, 2H), 2.83 (s, 2H), 2.44 (t, 2H), 2.01 (d, 2H), 1.95 - 1.86 (m, 8H), 1.79 - 1.45 (m, 4H).

### Example 213: N-(1-(4-(4-(3-(5-(((1-Acetylpiperidin-4-yl)amino)methyl)-6-methoxypyridin-2-yl)-2-chlorophenyl)-3-chloropyridin-2-yl)-2-methoxybenzyl)piperidin-4-yl)acetamide (325)

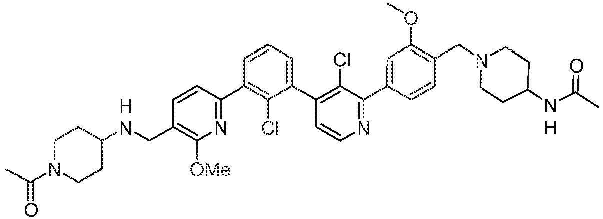

### (a) 1-(4-(((6-(2-chloro-3-(2,3-dichloropyridin-4-yl)phenyl)-2-methoxypyridin-3-yl)methyl)amino)piperidin-1-yl)ethan-1-one

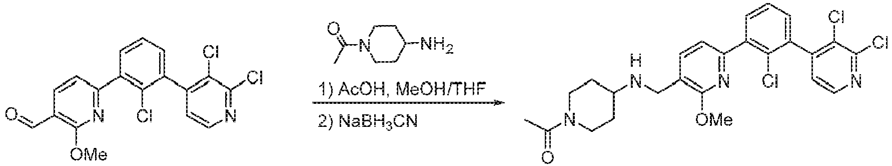

6-[2-Chloro-3-(2,3-dichloro-4-pyridyl)phenyl]-2-methoxy-pyridine-3-carbaldehyde (100 mg, 0.25 mmol), acetic acid (15 mg, 0.25 mmol), and 1-(4-amino-1-piperidyl)ethanone (51 mg, 0.36 mmol) were dissolved in MeOH/THF (1:1, 3 mL), then the solution was stirred at 25 °C for 2 hours. Sodium cyanoborohydride (24 mg, 0.38 mmol) was added and the resulting mixture was stirred at 25 °C for 1 hour. Reaction was subsequently diluted with water (10 mL) and extracted with EtOAc (3 x 5 mL). The combined organics were further washed with brine, dried over sodium sulfate, filtered, and concentrated under reduced pressure to afford 1-(4-(((6-(2-chloro-3-(2,3-dichloropyridin-4-yl)phenyl)-2-methoxypyridin-3-yl)methyl)amino)piperidin-1-yl)ethan-1-one (132 mg, crude) as a yellow oil. MS: *m*/z found 519, 521 [M+H] ⁺. Material was used for the next step without further purification.

### (b) tert-butyl (1-acetylpiperidin-4-yl)((6-(2-chloro-3-(2,3-dichloropyridin-4-yl)phenyl)-2-methoxypyridin-3-yl)methyl)carbamate

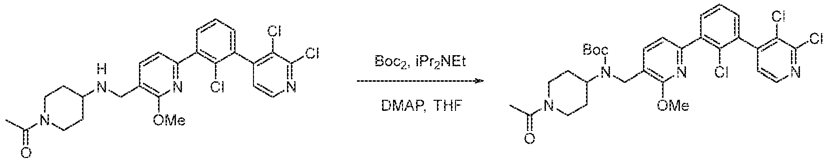

1-(4-(((6-(2-chloro-3-(2,3-dichloropyridin-4-yl)phenyl)-2-methoxypyridin-3-yl)methyl)amino)piperidin-1-yl)ethan-1-one (132 mg, crude), DMAP (6 mg, 0.05 mmol), and diisopropylethylamine (66 mg, 0.51 mmol) were dissolved in 5 ml THF, then tert-butoxycarbonyl tert-butyl carbonate (111 mg, 0.51 mmol) was added portion-wise. The resulting mixture was stirred at room temperature for 18 hours. Reaction was diluted with water (10 mL) and the aqueous solution was extracted with EtOAc (3 x 5 mL). The combined organics were concentrated under reduced pressure and crude sample was purified by normal phase SiO₂ chromatography (0-100% EtOAc/hexane) to afford tert-butyl (1-acetylpiperidin-4-yl)((6-(2-chloro-3-(2,3-dichloropyridin-4-yl)phenyl)-2-methoxypyridin-3-yl)methyl)carbamate (114 mg, 72 % yield) as a white foam. MS: *m*/z found 619, 621 [M+H]⁺.

### (c) tert-butyl (1-acetylpiperidin-4-yl)((6-(2-chloro-3-(3-chloro-2-(4-formyl-3-methoxyphenyl)-pyridin-4-yl)-phenyl)-2-methoxypyridin-3-yl)methyl)carbamate

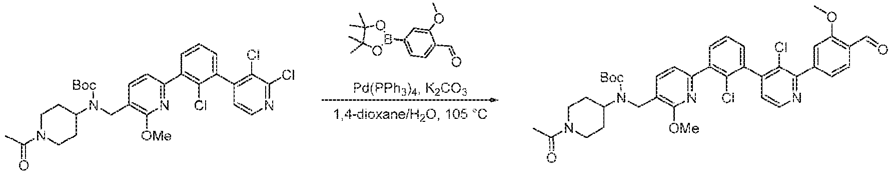

Tetrakis(triphenylphosphine)palladium(0) (43 mg, 0.04 mmol), potassium carbonate (76 mg, 0.55 mmol), tert-butyl (1-acetylpiperidin-4-yl)((6-(2-chloro-3-(2,3-dichloropyridin-4-yl)phenyl)-2-methoxypyridin-3-yl)methyl)carbamate (114 mg, 0.18 mmol), and 2-methoxy-4-(4,4,5,5-tetramethyl-1,3,2-dioxaborolan-2-yl)benzaldehyde (72 mg, 0.28 mmol) were suspended in 1,4-dioxane /water (4:1, 4 mL), then the solution was heated at 105 °C for 20 minutes. Reaction was cooled to rt, diluted with 10 mL water, and extracted with EtOAc (3 x 5 mL). The combined organics were concentrated under reduced pressure and the crude sample was purified by silica gel chromatography (0-50% EtOAc/hexane) to afford tert-butyl (1-acetylpiperidin-4-yl)((6-(2-chloro-3-(3-chloro-2-(4-formyl-3-methoxyphenyl)-pyridin-4-yl)-phenyl)-2-methoxypyridin-3-yl)methyl)carbamate (114 mg, 86 % yield) as a yellow foam. MS: *m*/z found 719, 721 M+H]⁺.

### (d) tert-butyl ((6-(3-(2-(4-((4-acetamidopiperidin-1-yl)methyl)-3-methoxyphenyl)-3-chloropyridin-4-yl)-2-chlorophenyl)-2-methoxypyridin-3-yl)methyl)(1-acetylpiperidin-4-yl)carbamate

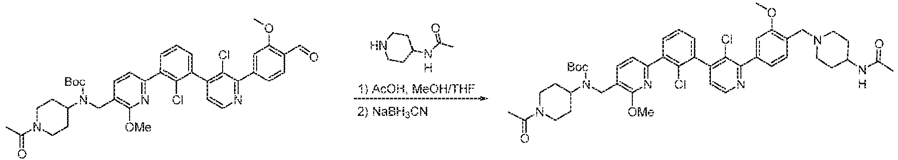

tert-Butyl (1-acetylpiperidin-4-yl)((6-(2-chloro-3-(3-chloro-2-(4-formyl-3-methoxyphenyl)-pyridin-4-yl)-phenyl)-2-methoxypyridin-3-yl)methyl)carbamate (20 mg, 0.03 mmol), acetic acid (3 mg, 0.06 mmol), and N-(4-piperidyl)acetamide (8 mg, 0.06 mmol) were dissolved in MeOH/THF (1:1, 1 mL), then the solution was stirred at 25 °C for 2 hours. Sodium cyanoborohydride (7 mg, 0.11 mmol) was added and the resulting mixture was stirred at 25 °C for 1 hour. Reaction was subsequently diluted with water (2 mL) and extracted with EtOAc (3 x 2 mL). The combined organics were further washed with brine, dried over sodium sulfate, filtered, and concentrated under reduced pressure to afford tert-butyl ((6-(3-(2-(4-((4-acetamidopiperidin-1-yl)methyl)-3-methoxyphenyl)-3-chloropyridin-4-yl)-2-chlorophenyl)-2-methoxypyridin-3-yl)methyl)(1-acetylpiperidin-4-yl)carbamate (23 mg, crude) as a clear oil. MS: *m*/z found 845, 847 [M+H] ⁺. Material was used for the next step without further purification.

### (e) N-(1-(4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)piperidin-4-yl)acetamide

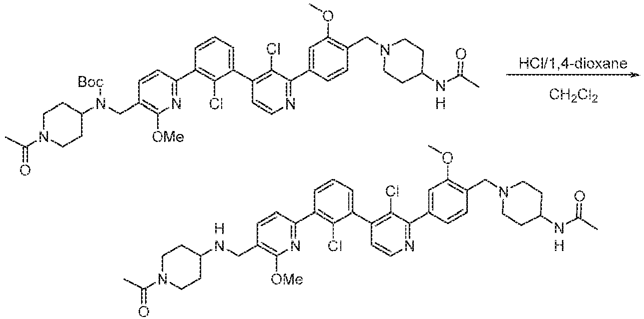

tert-Butyl ((6-(3-(2-(4-((4-acetamidopiperidin-1-yl)methyl)-3-methoxyphenyl)-3-chloropyridin-4-yl)-2-chlorophenyl)-2-methoxypyridin-3-yl)methyl)(1-acetylpiperidin-4-yl)carbamate (23 mg, 0.03 mmol, crude) was dissolved in 1 mL MeOH and 4M HCl solution in 1,4-dioxane (27 µL, 0.11 mmol) was added. Reaction was stirred at room temperature for 60 minutes and solvent was removed under reduced pressure. Crude sample was purified by reverse phase HPLC to afford N-(1-(4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)piperidin-4-yl)acetamide (10 mg, 49 % yield) as a white solid (formic acid salt). LC/MS: *m*/z found 745, 747 [M+H]⁺, retention time = 2.12 min (Method C). ¹H NMR (400 MHz, Methanol-*d*₄) δ 8.67 - 8.61 (m, 1H), 8.48 (s, 1H), 7.84 (d, 1H), 7.71 (d, 1H), 7.56 (t, 1H), 7.50 (d, 1H), 7.44 (dd, 2H), 7.37 - 7.27 (m, 3H), 4.58 (d, 1H), 4.11 (s, 2H), 4.05 (t, 3H), 4.03 (s, 3H), 3.94 (s, 3H), 3.78 (s, 1H), 3.25 - 3.12 (m, 4H), 2.80 - 2.64 (m, 3H), 2.23 - 2.06 (m, 5H), 1.98 (d, 2H), 1.92 (t, 3H), 1.67 (d, 2H), 1.47 (dd, 2H).

### Example 214: (S)-4-((4-(4-(3-(5-(((1-Acetylpiperidin-4-yl)amino)methyl)-6-methoxypyridin-2-yl)-2-chlorophenyl)-3-chloropyridin-2-yl)-2-methoxybenzyl)amino)dihydrofuran-2(3H)-one (338)

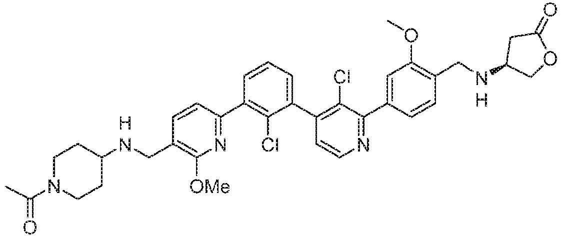

(S)-4-((4-(4-(3-(5-(((1-Acetylpiperidin-4-yl)amino)methyl)-6-methoxypyridin-2-yl)-2-chlorophenyl)-3-chloropyridin-2-yl)-2-methoxybenzyl)amino)dihydrofuran-2(3H)-one was prepared in similar manner as described for **Example 213,** utilizing intermediate tert-butyl N-(1-acetyl-4-piperidyl)-N-[[6-[2-chloro-3-[3-chloro-2-(4-formyl-3-methoxy-phenyl)-4-pyridyl]phenyl]-2-methoxy-3-pyridyl]methyl]carbamate and replacing N-(4-piperidyl)-acetamide with (S)-4-aminodihydrofuran-2(3H)-one in step (d). Product was obtained as a tan solid (formic acid salt). LC/MS: *m*/z found 704, 706 [M+H]⁺, retention time = 2.13 min (Method C). ¹H NMR (400 MHz, Methanol-*d*₄) δ 8.62 (d, 1H), 8.48 (s, 2H), 7.84 (d, 1H), 7.71 (d, 1H), 7.56 (t, 1H), 7.42 (t, 3H), 7.32 (d, 1H), 7.26 (d, 2H), 4.60 (d, 1H), 4.41 (t, 1H), 4.21 (d, 1H), 4.14 (s, 2H), 4.07 - 3.97 (m, 4H), 3.92 (s, 3H), 3.86 (d, 2H), 3.67 (s, 1H), 3.18 (t, 2H), 2.83 - 2.64 (m, 2H), 2.44 (d, 1H), 2.12 (s, 5H), 1.48 (dd, 2H).

### Example 215: Methyl (S)-3-((4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)amino)-4-hydroxybutanoate (339)

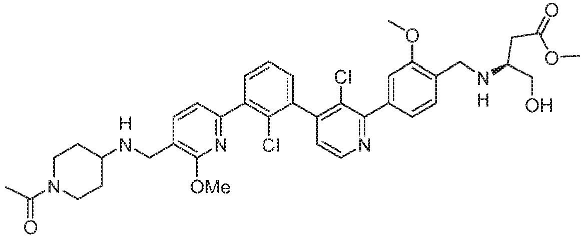

Methyl (S)-3-((4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)amino)-4-hydroxybutanoate was prepared in the same manner as described for Example 213, utilizing intermediate tert-butyl N-(1-acetyl-4-piperidyl)-N-[[6-[2-chloro-3-[3-chloro-2-(4-formyl-3-methoxy-phenyl)-4-pyridyl]phenyl]-2-methoxy-3-pyridyl]methyl]carbamate and replacing N-(4-piperidyl)-acetamide with methyl (S)-3-amino-4-hydroxybutanoate in step (d). Product was obtained as a tan solid (formic acid salt). LC/MS: *m*/z found 736, 738 [M+H] ⁺, retention time = 2.17 min (Method C). ¹H NMR (400 MHz, Methanol-*d*₄) δ 8.65 (s, 1H), 8.49 (s, 2H), 7.84 (d, 1H), 7.71 (d, 1H), 7.56 (t, 1H), 7.52 - 7.40 (m, 3H), 7.38 - 7.25 (m, 3H), 4.58 (d, 1H), 4.24 (d, 2H), 4.13 (d, 2H), 4.05 (d, 3H), 3.98 (s, 3H), 3.95 - 3.81 (m, 2H), 3.72 (s, 3H), 3.50 (s, 1H), 3.18 (t, 3H), 2.81 - 2.64 (m, 3H), 2.12 (s, 5H), 1.58 - 1.36 (m, 2H).

### Example 216: (S)-3-((4-(4-(3-(5-(((1-Acetylpiperidin-4-yl)amino)methyl)-6-methoxypyridin-2-yl)-2-chlorophenyl)-3-chloropyridin-2-yl)-2-methoxybenzyl)amino)dihydrofuran-2(3H)-one (340)

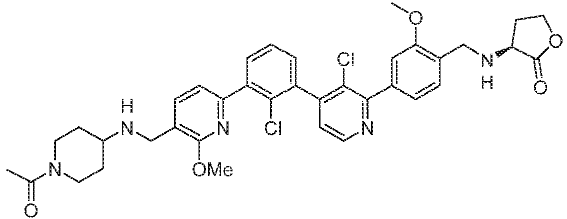

(S)-3-((4-(4-(3-(5-(((1-Acetylpiperidin-4-yl)amino)methyl)-6-methoxypyridin-2-yl)-2-chlorophenyl)-3-chloropyridin-2-yl)-2-methoxybenzyl)amino)dihydrofuran-2(3H)-one was prepared in the same manner as described for Example 213, utilizing intermediate tert-butyl N-(1-acetyl-4-piperidyl)-N-[[6-[2-chloro-3-[3-chloro-2-(4-formyl-3-methoxy-phenyl)-4-pyridyl]phenyl]-2-methoxy-3-pyridyl]methyl]carbamate and replacing N-(4-piperidyl)-acetamide with (S)-3-aminodihydrofuran-2(3H)-one in step (d). Product was obtained as a tan solid (formic acid salt). LC/MS: *m*/z found 704, 706 [M+H] ⁺, retention time = 2.13 min (Method C). ¹H NMR (400 MHz, Methanol-*d*₄) δ 8.62 (d, 1H), 8.53 (s, 1H), 7.78 (d, 1H), 7.70 (d, 1H), 7.56 (d, 1H), 7.43 (d, 3H), 7.28 (s, 3H), 4.41 (s, 1H), 4.03 (s, 3H), 3.99 - 3.92 (m, 7H), 3.62 (s, 1H), 3.16 (s, 1H), 2.10 (s, 6H).

### Example 217: N-(1-((6-(3-(2-(4-(((1-acetylpiperidin-4-yl)amino)methyl)-3-methoxyphenyl)-3-chloropyridin-4-yl)-2-chlorophenyl)-2-methoxypyridin-3-yl)methyl)piperidin-4-yl)acetamide (341)

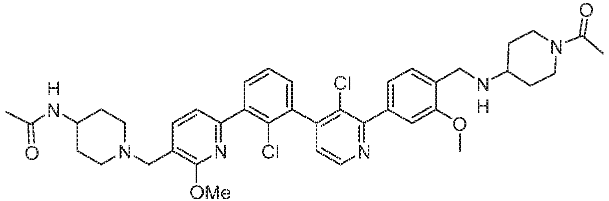

### (a) N-(1-((6-(2-chloro-3-(2,3-dichloropyridin-4-yl)phenyl)-2-methoxypyridin-3-yl)methyl)piperidin-4-yl)acetamide

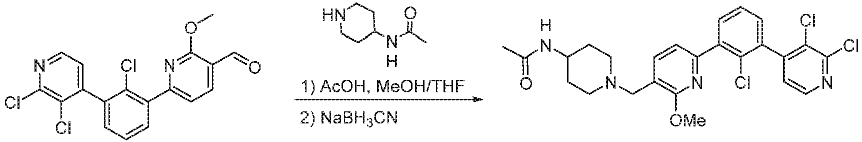

6-[2-Chloro-3-(2,3-dichloro-4-pyridyl)phenyl]-2-methoxy-pyridine-3-carbaldehyde (150 mg, 0.38 mmol), N-(4-piperidyl)acetamide (108 mg, 0.76 mmol), and acetic acid (23 mg, 0.38 mmol) were dissolved in MeOH/THF (1:1, 5 mL), then the solution was stirred at 25 °C for 2 hours. Sodium cyanoborohydride (48 mg, 0.76 mmol) was added and the resulting mixture was stirred at 25 °C for 1 hour. Reaction was subsequently diluted with water (10 mL) and extracted with EtOAc (3 x 5 mL). The combined organics were further washed with brine, dried over sodium sulfate, filtered, and concentrated under reduced pressure to afford N-(1-((6-(2-chloro-3-(2,3-dichloropyridin-4-yl)phenyl)-2-methoxypyridin-3-yl)methyl)piperidin-4-yl)acetamide (198 mg, crude) as a yellow foam. MS: *m*/*z* found 519, 521 [M+H] ⁺. Material was used for the next step without further purification.

### (b) N-(1-((6-(2-chloro-3-(3-chloro-2-(4-formyl-3-methoxyphenyl)pyridin-4-yl)phenyl)-2-methoxypyridin-3-yl)methyl)piperidin-4-yl)acetamide

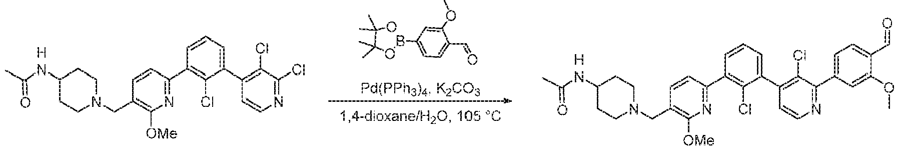

Tetrakis(triphenylphosphine)palladium(0) (88 mg, 0.08 mmol), potassium carbonate (158 mg, 1.14 mmol), N-(1-((6-(2-chloro-3-(2,3-dichloropyridin-4-yl)phenyl)-2-methoxypyridin-3-yl)methyl)piperidin-4-yl)-acetamide (198 mg, 0.38 mmol, crude), and 2-methoxy-4-(4,4,5,5-tetramethyl-1,3,2-dioxaborolan-2-yl)benzaldehyde (150 mg, 0.57 mmol) were suspended in 1,4-dioxane /water (4:1, 6 mL), then the solution was heated at 105 °C for 20 minutes. Reaction was cooled to room temperature, diluted with 10 mL water, and extracted with EtOAc (3 x 5 mL). The combined organics were concentrated under reduced pressure and the crude sample was purified by silica gel chromatography (0-50% EtOAc/hexane) to improve the purity, affording N-[1-[[6-[2-chloro-3-[3-chloro-2-(4-formyl-3-methoxy-phenyl)-4-pyridyl]phenyl]-2-methoxy-3-pyridyl]methyl]-4-piperidyl]acetamide (235 mg, crude) as a yellow solid. MS: m/z found 619, 621 [M+H] ⁺. Material was used for the next step without further purification.

### (c) N-(1-((6-(3-(2-(4-(((1-acetylpiperidin-4-yl)amino)methyl)-3-methoxyphenyl)-3-chloropyridin-4-yl)-2-chlorophenyl)-2-methoxypyridin-3-yl)methyl)piperidin-4-yl)acetamide

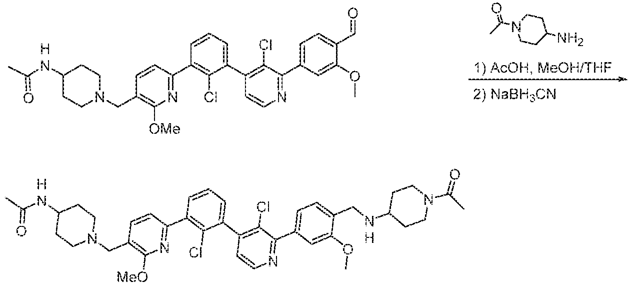

N-[1-[[6-[2-chloro-3-[3-chloro-2-(4-formyl-3-methoxy-phenyl)-4-pyridyl]phenyl]-2-methoxy-3-pyridyl]methyl]-4-piperidyl]acetamide (25 mg, 0.04 mmol, crude), acetic acid (5 mg, 0.08 mmol), and 1-(4-amino-1-piperidyl)ethanone (12 mg, 0.08 mmol) were dissolved in MeOH/THF (1:1, 1 mL), then the solution was stirred at 25 °C for 2 hours. Sodium cyanoborohydride (5 mg, 0.08 mmol) was added and the resulting mixture was stirred at 25 °C for 1 hour. Reaction was subsequently diluted with water (2 mL) and extracted with EtOAc (3 x 2 mL). The combined organics were further washed with brine, dried over sodium sulfate, filtered, and concentrated under reduced pressure. Crude sample was purified by reverse phase HPLC to afford N-(1-((6-(3-(2-(4-(((1-acetylpiperidin-4-yl)amino)methyl)-3-methoxyphenyl)-3-chloropyridin-4-yl)-2-chlorophenyl)-2-methoxypyridin-3-yl)methyl)piperidin-4-yl)acetamide (8 mg, 26 % yield) as a white solid (formic acid salt). LC/MS: *m*/z found 745, 747 [M+H] ⁺, retention time = 2.13 min (Method C). ¹H NMR (400 MHz, Methanol-*d*₄) δ 8.65 (d, 1H), 8.48 (s, 2H), 7.79 (d, 1H), 7.72 (d, 1H), 7.58 - 7.50 (m, 2H), 7.47 (d, 1H), 7.43 - 7.37 (m, 2H), 7.34 (d, 1H), 7.27 (d, 1H), 4.65 (d, 1H), 4.27 (s, 2H), 4.06 (d, 1H), 3.99 (d, 6H), 3.73 (s, 3H), 3.19 (t, 2H), 3.04 (d, 2H), 2.70 (d, 1H), 2.41 (t, 2H), 2.22 (t, 2H), 2.13 (s, 3H), 1.91 (s, 5H), 1.68 - 1.44 (m, 4H).

### Example 218: (S)-3-((4-(4-(3-(5-(((1-Acetylpiperidin-4-yl)amino)methyl)-6-methoxypyridin-2-yl)-2-chlorophenyl)-3-chloropyridin-2-yl)-2-methoxybenzyl)amino)-4-hydroxybutanoic acid (342)

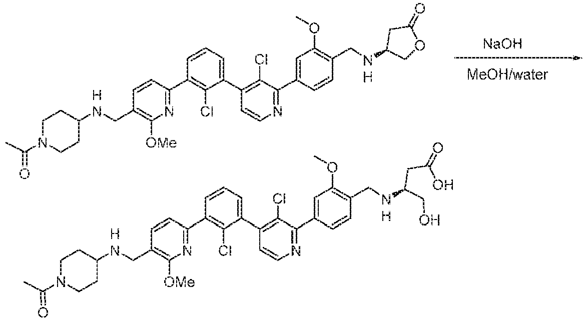

(S)-4-((4-(4-(3-(5-(((1-Acetylpiperidin-4-yl)amino)methyl)-6-methoxypyridin-2-yl)-2-chlorophenyl)-3-chloropyridin-2-yl)-2-methoxybenzyl)amino)dihydrofuran-2(3H)-one (Example 214) (5 mg, 0.01 mmol) was dissolved in water/MeOH (1:1, 0.5 mL) and sodium hydroxide (71 uL, 0.07 mmol, 1M aqueous solution) was added. Reaction was stirred at room temperature for 60 minutes and solvent was removed under reduced pressure. Crude sample was purified by reverse phase HPLC to afford (S)-3-((4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)amino)-4-hydroxybutanoic acid (2 mg, 29 % yield) as a white solid (formic acid salt). LC/MS: *m*/z found 722, 724 [M+H] ⁺, retention time = 2.10 min (Method C). ¹H NMR (400 MHz, Methanol-*d*₄) δ 8.67 - 8.62 (m, 1H), 8.46 (s, 2H), 7.86 (d, 1H), 7.71 (d, 1H), 7.57 (t, 1H), 7.52 (d, 1H), 7.48 - 7.42 (m, 2H), 7.37 (s, 1H), 7.33 (t, 2H), 4.62 (d, 1H), 4.39 (d, 1H), 4.30 (d, 1H), 4.19 (s, 2H), 4.07 (d, 3H), 4.00 (s, 4H), 3.94 (d, 1H), 3.71 - 3.65 (m, 1H), 3.45 (s, 1H), 3.19 (t, 2H), 2.70 (t, 1H), 2.63 - 2.41 (m, 2H), 2.16 (d, 5H), 1.51 (d, 2H).

### Example 219: (4-(4-(3-(5-(((1-Acetylpiperidin-4-yl)amino)methyl)-6-methoxypyridin-2-yl)-2-chlorophenyl)-3-chloropyridin-2-yl)-2-methoxybenzyl)-L-homoserine (343)

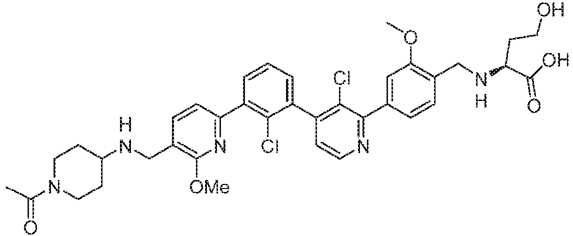

(4-(4-(3-(5-(((1-Acetylpiperidin-4-yl)amino)methyl)-6-methoxypyridin-2-yl)-2-chlorophenyl)-3-chloropyridin-2-yl)-2-methoxybenzyl)-L-homoserine was prepared in similar manner as described for Example 218, replacing (S)-4-((4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)-amino)-dihydrofuran-2(3H)-one with methyl (S)-3-((4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)amino)-4-hydroxybutanoate (Example 215). Product was obtained as a tan solid (formic acid salt). LC/MS: *m*/z found 722, 724 [M+H] ⁺, retention time = 2.12 min (Method C). ¹H NMR (400 MHz, Methanol-*d*₄) δ 8.65 (d, 1H), 8.46 (s, 2H), 7.85 (d, 1H), 7.71 (d, 1H), 7.56 (t, 1H), 7.51 - 7.41 (m, 3H), 7.37 (s, 1H), 7.33 (d, 2H), 4.61 (d, 1H), 4.34 (d, 1H), 4.28 (d, 1H), 4.17 (s, 2H), 4.06 (s, 3H), 4.02 (s, 1H), 3.98 (s, 3H), 3.85 (d, 1H), 3.78 (d, 1H), 3.67 (d, 1H), 3.19 (t, 2H), 2.70 (s, 1H), 2.15 (d, 6H).

### Example 220: N-(1-((6-(2-Chloro-3-(3-chloro-2-(3-methoxy-4-((7-oxo-2,6-diazaspiro [3.4] octan-2-yl)methyl)phenyl)pyridin-4-yl)phenyl)-2-methoxypyridin-3-yl)methyl)piperidin-4-yl)acetamide (355)

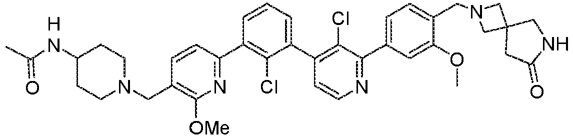

N-(1-((6-(2-Chloro-3-(3-chloro-2-(3-methoxy-4-((7-oxo-2,6-diazaspiro[3.4]octan-2-yl)methyl)phenyl)pyridin-4-yl)phenyl)-2-methoxypyridin-3-yl)methyl)piperidin-4-yl)acetamide was prepared in similar manner as described for Example 217, utilizing intermediate N-[1-[[6-[2-chloro-3-[3-chloro-2-(4-formyl-3-methoxy-phenyl)-4-pyridyl]phenyl]-2-methoxy-3-pyridyl]methyl]-4-piperidyl]acetamide and replacing 1-(4-amino-1-piperidyl)ethanone with 2,6-diazaspiro[3.4]octan-7-one in step (c). Product was obtained as a white solid (formic acid salt). LC/MS: *m*/z found 729, 731 [M+H] ⁺, retention time = 2.06 min (Method C). ¹H NMR (400 MHz, Methanol-*d*₄) δ 8.63 (d, 1H), 8.48 (d, 1H), 7.79 (d, 1H), 7.71 (d, 1H), 7.55 (t, 1H), 7.43 (dd, 3H), 7.35 - 7.23 (m, 3H), 4.09 (s, 2H), 4.00 (t, 3H), 3.94 (d, 3H), 3.80 (s, 4H), 3.73 (s, 3H), 3.62 (d, 2H), 3.05 (d, 2H), 2.64 (d, 2H), 2.42 (s, 2H), 1.91 (t, 5H), 1.58 (d, 2H).

### Example 221: (R)-N-(1-((6-(2-Chloro-3-(3-chloro-2-(3-methoxy-4-((((5-oxopyrrolidin-2-yl)methyl)amino)methyl)phenyl)pyridin-4-yl)phenyl)-2-methoxypyridin-3-yl)methyl)piperidin-4-yl)acetamide (356)

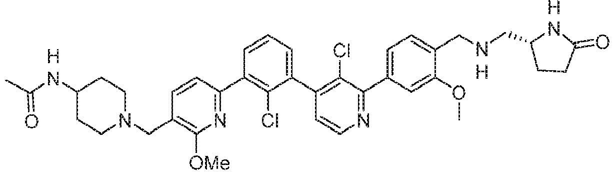

(R)-N-(1-((6-(2-Chloro-3-(3-chloro-2-(3-methoxy-4-((((5-oxopyrrolidin-2-yl)methyl)amino)methyl)phenyl)pyridin-4-yl)phenyl)-2-methoxypyridin-3-yl)methyl)piperidin-4-yl)acetamide was prepared in similar manner as described for Example 217, utilizing intermediate N-[1-[[6-[2-chloro-3-[3-chloro-2-(4-formyl-3-methoxy-phenyl)-4-pyridyl]phenyl]-2-methoxy-3-pyridyl]methyl]-4-piperidyl]acetamide and replacing 1-(4-amino-1-piperidyl)ethanone with (5R)-5-(aminomethyl)pyrrolidin-2-one in step (c). Product was obtained as a white solid (formic acid salt). LC/MS: *m*/z found 717, 719 [M+H] ⁺, retention time = 2.07 min (Method C). ¹H NMR (400 MHz, Methanol-*d*₄) δ 8.67 - 8.61 (m, 1H), 8.44 (s, 1H), 7.81 (d, 1H), 7.72 (d, 1H), 7.55 (t, 1H), 7.47 (t, 2H), 7.42 (d, 1H), 7.32 (dd, 3H), 4.14 (s, 2H), 4.01 (d, 3H), 3.97 (d, 4H), 3.83 (s, 2H), 3.73 (s, 1H), 3.12 (d, 2H), 2.99 (s, 2H), 2.54 (s, 2H), 2.36 (s, 3H), 1.92 (d, 5H), 1.85 (s, 1H), 1.61 (d, 2H).

### Example 222: (S)-N-(1-((6-(2-Chloro-3-(3-chloro-2-(3-methoxy-4-((((5-oxopyrrolidin-2-yl)methyl)amino)methyl)phenyl)pyridin-4-yl)phenyl)-2-methoxypyridin-3-yl)methyl)piperidin-4-yl)acetamide (357)

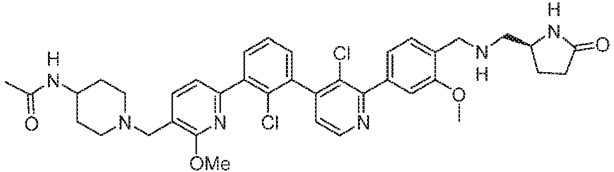

(S)-N-(1-((6-(2-Chloro-3-(3-chloro-2-(3-methoxy-4-((((5-oxopyrrolidin-2-yl)methyl)amino)methyl)phenyl)pyridin-4-yl)phenyl)-2-methoxypyridin-3-yl)methyl)piperidin-4-yl)acetamide was prepared in similar manner as described for Example 217, utilizing intermediate N-[1-[[6-[2-chloro-3-[3-chloro-2-(4-formyl-3-methoxy-phenyl)-4-pyridyl]phenyl]-2-methoxy-3-pyridyl]methyl]-4-piperidyl]-acetamide and replacing 1-(4-amino-1-piperidyl)ethanone with (5S)-5-(aminomethyl)pyrrolidin-2-one in step (c). Product was obtained as a white solid (formic acid salt). LC/MS: *m*/z found 717, 719 [M+H] ⁺, retention time = 2.08 min (Method C). ¹H NMR (400 MHz, Methanol-*d*₄) δ 8.64 (d, 1H), 8.45 (s, 1H), 7.80 (d, 1H), 7.72 (d, 1H), 7.55 (t, 1H), 7.47 (d, 2H), 7.41 (d, 1H), 7.36 - 7.27 (m, 3H), 4.12 (d, 2H), 4.01 (d, 3H), 3.96 (d, 4H), 3.79 (s, 2H), 3.72 (s, 1H), 3.09 (d, 2H), 2.96 (s, 2H), 2.49 (s, 2H), 2.34 (d, 3H), 1.92 (s, 5H), 1.85 (s, 1H), 1.60 (d, 2H).

### Example 223: N-(1-(4-(3-chloro-4-(2-chloro-3-(6-methoxy-5-((7-oxo-2,6-diazaspiro[3.4]-octan-2-yl)methyl)pyridin-2-yl)-phenyl)pyridin-2-yl)-2-methoxybenzyl)piperidin-4-yl)acetamide (400)

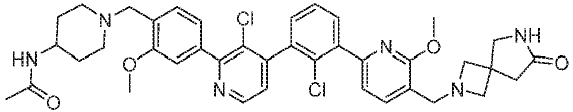

### (a) N-(1-(2-methoxy-4-(4,4,5,5-tetramethyl-1,3,2-dioxaborolan-2-yl)benzyl)piperidin-4-yl)acetamide

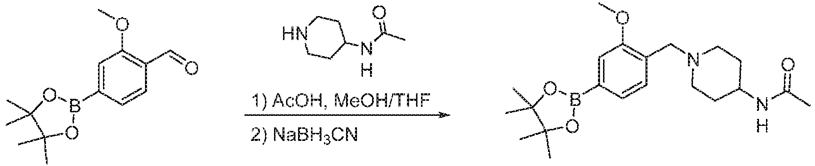

2-Methoxy-4-(4,4,5,5-tetramethyl-1,3,2-dioxaborolan-2-yl)benzaldehyde (200 mg, 0.76 mmol), acetic acid (46 mg, 0.76 mmol), and N-(4-piperidyl)acetamide (141 mg, 0.99 mmol) were dissolved in MeOH/THF (1:1, 6 mL), then the solution was stirred at 25 °C for 2 hours. Sodium cyanoborohydride (67 mg, 1.07 mmol) was added portion-wise and the resulting mixture was stirred at 25 °C for 1 hour. Reaction was subsequently diluted with water (15 mL) and extracted with EtOAc (3 x 5 mL). The combined organics were further washed with brine, dried over sodium sulfate, filtered, and concentrated under reduced pressure to afford N-(1-(2-methoxy-4-(4,4,5,5-tetramethyl-1,3,2-dioxaborolan-2-yl)benzyl)piperidin-4-yl)acetamide (182 mg, crude) as a yellow oil. MS: *m*/z found 389 [M+H] ⁺. Material was used for the next step without further purification.

### (b) N-(1-(4-(3-chloro-4-(2-chloro-3-(5-formyl-6-methoxypyridin-2-yl)phenyl)pyridin-2-yl)-2-methoxybenzyl)piperidin-4-yl)acetamide

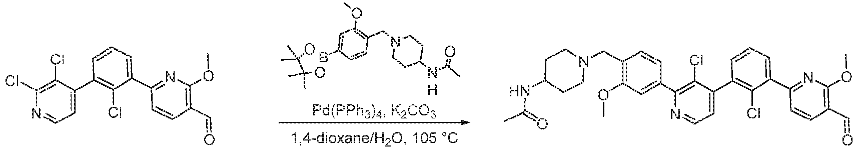

Tetrakis(triphenylphosphine)palladium(0) (70 mg, 0.06 mmol), potassium carbonate (126 mg, 0.91 mmol), 6-[2-chloro-3-(2,3-dichloro-4-pyridyl)phenyl]-2-methoxy-pyridine-3-carbaldehyde (120mg, 0.30 mmol), and N-(1-(2-methoxy-4-(4,4,5,5-tetramethyl-1,3,2-dioxaborolan-2-yl)benzyl)piperidin-4-yl)acetamide (178 mg, 0.46 mmol) were suspended in 1,4-dioxane/water (4:1, 5 mL), then the solution was heated at 105 °C for 20 minutes. Reaction was cooled to rt, diluted with 10 mL water, and extracted with EtOAc (3 x 5 mL). The combined organics were concentrated under reduced pressure and the crude sample was purified by silica gel chromatography (0-10% MeOH/DCM) to afford N-(1-(4-(3-chloro-4-(2-chloro-3-(5-formyl-6-methoxypyridin-2-yl)phenyl)pyridin-2-yl)-2-methoxybenzyl)piperidin-4-yl)acetamide (82 mg, 43 % yield) as a light yellow foam. MS: *m*/z found 619, 621 [M+H] ⁺.

### (c) N-(1-(4-(3-chloro-4-(2-chloro-3-(6-methoxy-5-((7-oxo-2,6-diazaspiro[3.4]-octan-2-yl)methyl)pyridin-2-yl)-phenyl)pyridin-2-yl)-2-methoxybenzyl)piperidin-4-yl)acetamide

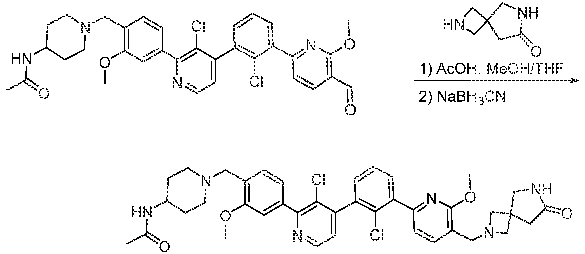

N-(1-(4-(3-chloro-4-(2-chloro-3-(5-formyl-6-methoxypyridin-2-yl)phenyl)pyridin-2-yl)-2-methoxybenzyl)piperidin-4-yl)acetamide (20 mg, 0.03 mmol), acetic acid (4 mg, 0.06 mmol), and 2,6-diazaspiro[3.4]octan-7-one (8 mg, 0.06 mmol) were dissolved in MeOH/THF (1:1, 1 mL), then the solution was stirred at 25 °C for 2 hours. Sodium cyanoborohydride (4 mg, 0.06 mmol) was added and the resulting mixture was stirred at 25 °C for 1 hour. Reaction was subsequently diluted with water (2 mL) and extracted with EtOAc (3 x 2 mL). The combined organics were further washed with brine, dried over sodium sulfate, filtered, and concentrated under reduced pressure. Crude sample was purified by reverse phase HPLC to afford N-(1-(4-(3-chloro-4-(2-chloro-3-(6-methoxy-5-((7-oxo-2,6-diazaspiro[3.4]-octan-2-yl)methyl)pyridin-2-yl)-phenyl)pyridin-2-yl)-2-methoxybenzyl)piperidin-4-yl)acetamide (10 mg, 42 % yield) as a white solid (formic acid salt). LC/MS: *m*/z found 729, 731 [M+H] ⁺, retention time = 2.08 min (Method C). ¹H NMR (400 MHz, Methanol-*d*₄) δ 8.62 (d, 1H), 8.53 (s, 1H), 7.69 (d, 2H), 7.54 (t, 1H), 7.45 (t, 2H), 7.40 (d, 1H), 7.29 (d, 2H), 7.24 (d, 1H), 3.99 (s, 3H), 3.91 (s, 3H), 3.84 (s, 2H), 3.73 (s, 3H), 3.58 (s, 2H), 3.44 (s, 4H), 3.09 (s, 2H), 2.58 (s, 2H), 2.48 (s, 2H), 1.91 (s, 5H), 1.59 (d, 2H).

### Example 224: N-(1-(4-(3-Chloro-4-(2-chloro-3-(6-methoxy-5-((6-oxo-2,5-diazaspiro[3.4]-octan-2-yl)methyl)pyridin-2-yl)phenyl)pyridin-2-yl)-2-methoxybenzyl)piperidin-4-yl)acetamide (401)

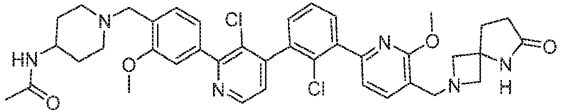

N-(1-(4-(3-Chloro-4-(2-chloro-3-(6-methoxy-5-((6-oxo-2,5-diazaspiro[3.4]-octan-2-yl)methyl)pyridin-2-yl)phenyl)pyridin-2-yl)-2-methoxybenzyl)piperidin-4-yl)acetamide was prepared in similar manner as described for Example 223, utilizing intermediate N-(1-(4-(3-chloro-4-(2-chloro-3-(5-formyl-6-methoxypyridin-2-yl)phenyl)pyridin-2-yl)-2-methoxybenzyl)piperidin-4-yl)acetamide and replacing 2,6-diazaspiro[3.4]octan-7-one with 2,5-diazaspiro[3.4]octan-6-one in step (c). Product was obtained as a white solid (formic acid salt). LC/MS: *m*/z found 729, 731 [M+H] ⁺, retention time = 2.09 min (Method C). ¹H NMR (400 MHz, Methanol-*d*₄) δ 8.63 (d, 1H), 8.51 (s, 1H), 7.69 (d, 2H), 7.54 (t, 1H), 7.46 (dd, 2H), 7.40 (d, 1H), 7.30 (d, 2H), 7.24 (d, 1H), 3.99 (t, 3H), 3.92 (d, 5H), 3.73 (s, 4H), 3.57 (d, 2H), 3.38 (d, 2H), 3.14 (s, 1H), 2.59 (s, 2H), 2.38 (dd, 4H), 2.07 - 1.89 (m, 6H), 1.62 (d, 1H).

### Example 225: N-(1-(4-(4-(3-(5-((6-acetyl-2,6-diazaspiro[3.3]heptan-2-yl)methyl)-6-methoxypyridin-2-yl)-2-chlorophenyl)-3-chloropyridin-2-yl)-2-methoxybenzyl)piperidin-4-yl)acetamide (402)

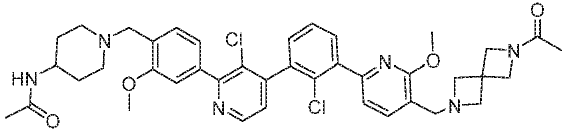

N-(1-(4-(4-(3-(5-((6-acetyl-2,6-diazaspiro[3.3]heptan-2-yl)methyl)-6-methoxypyridin-2-yl)-2-chlorophenyl)-3-chloropyridin-2-yl)-2-methoxybenzyl)piperidin-4-yl)acetamide was prepared in similar manner as described for Example 223, utilizing intermediate N-(1-(4-(3-chloro-4-(2-chloro-3-(5-formyl-6-methoxypyridin-2-yl)phenyl)pyridin-2-yl)-2-methoxybenzyl)piperidin-4-yl)acetamide and replacing 2,6-diazaspiro[3.4]octan-7-one with 1-(2,6-diazaspiro[3.3]heptan-2-yl)-ethanone in step (c). Product was obtained as a white solid (formic acid salt). LC/MS: *m*/z found 743, 745 [M+H] ⁺, retention time = 2.10 min (Method C). ¹H NMR (400 MHz, Methanol-*d*₄) δ 8.63 (d, 1H), 8.51 (s, 1H), 7.69 (d, 2H), 7.54 (t, 1H), 7.46 (dd, 2H), 7.40 (d, 1H), 7.35 - 7.28 (m, 2H), 7.24 (d, 1H), 4.30 (s, 2H), 4.05 (s, 2H), 3.99 (t, 4H), 3.92 (d, 5H), 3.73 (s, 4H), 3.58 (s, 5H), 3.16 (s, 1H), 2.63 (s, 1H), 1.98 - 1.90 (m, 5H), 1.84 (d, 3H), 1.63 (d, 1H).

### Example 226: (S)-N-(1-(4-(3-Chloro-4-(2-chloro-3-(6-methoxy-5-((((5-oxopyrrolidin-2-yl)methyl)amino)methyl)pyridin-2-yl)phenyl)pyridin-2-yl)-2-methoxybenzyl)piperidin-4-yl)acetamide (403)

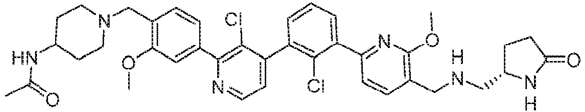

(S)-N-(1-(4-(3-Chloro-4-(2-chloro-3-(6-methoxy-5-((((5-oxopyrrolidin-2-yl)methyl)amino)methyl)pyridin-2-yl)phenyl)pyridin-2-yl)-2-methoxybenzyl)piperidin-4-yl)acetamide was prepared in similar manner as described for Example 223, utilizing intermediate N-(1-(4-(3-chloro-4-(2-chloro-3-(5-formyl-6-methoxypyridin-2-yl)phenyl)pyridin-2-yl)-2-methoxybenzyl)piperidin-4-yl)acetamide and replacing 2,6-diazaspiro[3.4]octan-7-one with (5S)-5-(aminomethyl)pyrrolidin-2-one in step (c). Product was obtained as a white solid (formic acid salt). LC/MS: *m*/z found 717, 719 [M+H] ⁺, retention time = 2.08 min (Method C). ¹H NMR (400 MHz, Methanol-*d*₄) δ 8.63 (d, 1H), 8.52 (s, 1H), 7.75 (d, 1H), 7.70 (d, 1H), 7.54 (t, 1H), 7.46 (dd, 2H), 7.40 (d, 1H), 7.30 (d, 2H), 7.25 (d, 1H), 4.02 (t, 3H), 3.94 - 3.88 (m, 5H), 3.85 (d, 3H), 3.73 (s, 1H), 3.15 (d, 2H), 2.78 - 2.66 (m, 2H), 2.57 (s, 2H), 2.30 (dd, 3H), 2.03 - 1.90 (m, 6H), 1.62 (d, 2H).

### Example 227: 1-(4-(((6-(3-(2-(4-((4-Amino-4-methylpiperidin-1-yl)methyl)-3-methoxyphenyl)-3-chloropyridin-4-yl)-2-chlorophenyl)-2-methoxypyridin-3-yl)-methyl)-amino)piperidin-1-yl)ethan-1-one (406)

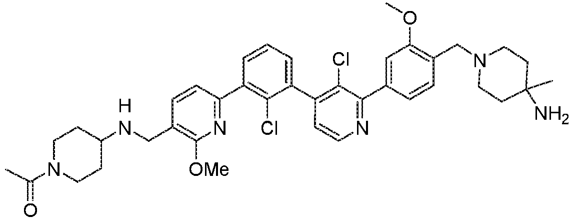

1-(4-(((6-(3-(2-(4-((4-Amino-4-methylpiperidin-1-yl)methyl)-3-methoxyphenyl)-3-chloropyridin-4-yl)-2-chlorophenyl)-2-methoxypyridin-3-yl)-methyl)-amino)piperidin-1-yl)ethan-1-one was prepared in similar manner as described for Example 213, utilizing intermediate tert-butyl N-(1-acetyl-4-piperidyl)-N-[[6-[2-chloro-3-[3-chloro-2-(4-formyl-3-methoxy-phenyl)-4-pyridyl]phenyl]-2-methoxy-3-pyridyl]methyl]carbamate and replacing N-(4-piperidyl)-acetamide with 4-methylpiperidin-4-amine in step (d). Product was obtained as a tan solid (formic acid salt). LC/MS: *m*/z found 717, 719 [M+H] ⁺, retention time = 2.07 min (Method C). ¹H NMR (400 MHz, Methanol-*d*₄) δ 8.63 (d, 1H), 8.49 (s, 2H), 7.83 (d, 1H), 7.71 (d, 1H), 7.56 (t, 1H), 7.53 - 7.40 (m, 3H), 7.29 (q, 3H), 4.58 (d, 1H), 4.10 (s, 2H), 4.05 (d, 3H), 4.01 (d, 1H), 3.94 - 3.85 (m, 3H), 3.73 (s, 2H), 3.18 (t, 2H), 2.83 (d, 2H), 2.70 (t, 1H), 2.52 (s, 2H), 2.21 - 2.06 (m, 5H), 1.98 - 1.70 (m, 4H), 1.58 - 1.38 (m, 2H), 1.36 (d, 3H).

### Example 228: (S)-4-(((4-(4-(3-(5-(((1-Acetylpiperidin-4-yl)amino)methyl)-6-methoxypyridin-2-yl)-2-chlorophenyl)-3-chloropyridin-2-yl)-2-methoxybenzyl)amino)methyl)pyrrolidin-2-one (407)

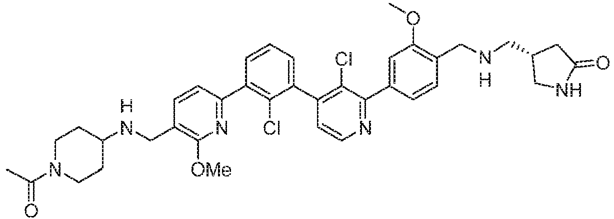

(S)-4-(((4-(4-(3-(5-(((1-Acetylpiperidin-4-yl)amino)methyl)-6-methoxypyridin-2-yl)-2-chlorophenyl)-3-chloropyridin-2-yl)-2-methoxybenzyl)amino)methyl)pyrrolidin-2-one was prepared in similar manner as described for Example 213, utilizing intermediate tert-butyl N-(1-acetyl-4-piperidyl)-N-[[6-[2-chloro-3-[3-chloro-2-(4-formyl-3-methoxy-phenyl)-4-pyridyl]phenyl]-2-methoxy-3-pyridyl]methyl]carbamate and replacing N-(4-piperidyl)-acetamide with (4S)-4-(aminomethyl)pyrrolidin-2-one in step (d). Product was obtained as a tan solid (formic acid salt). LC/MS: *m*/z found 717, 719 [M+H] ⁺, retention time = 2.06 min (Method C). ¹H NMR (400 MHz, Methanol-*d*₄) δ 8.67 - 8.61 (m, 1H), 8.50 (s, 1H), 7.81 (d, 1H), 7.74 - 7.68 (m, 1H), 7.56 (t, 1H), 7.51 - 7.39 (m, 3H), 7.32 (dd, 3H), 4.55 (d, 1H), 4.14 (s, 2H), 4.04 (d, 5H), 3.97 (d, 4H), 3.57 (t, 1H), 3.17 (t, 2H), 3.02 (d, 3H), 2.92 - 2.79 (m, 1H), 2.70 (t, 1H), 2.52 (dd, 1H), 2.22 - 2.04 (m, 6H), 1.43 (dd, 2H).

### Example 229: N-(1-((6-(2-Chloro-3-(3-chloro-2-(3-methoxy-4-(((tetrahydro-2H-pyran-4-yl)amino)methyl)phenyl)pyridin-4-yl)phenyl)-2-methoxypyridin-3-yl)methyl)piperidin-4-yl)acetamide (432)

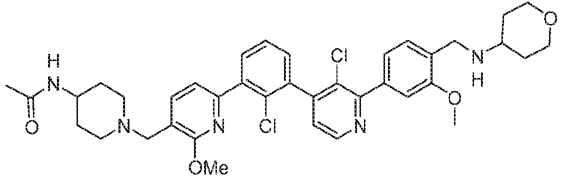

N-(1-((6-(2-Chloro-3-(3-chloro-2-(3-methoxy-4-(((tetrahydro-2H-pyran-4-yl)amino)methyl)phenyl)pyridin-4-yl)phenyl)-2-methoxypyridin-3-yl)methyl)piperidin-4-yl)acetamide was prepared in similar manner as described for Example 217, utilizing intermediate N-[1-[[6-[2-chloro-3-[3-chloro-2-(4-formyl-3-methoxy-phenyl)-4-pyridyl]phenyl]-2-methoxy-3-pyridyl]methyl]-4-piperidyl]acetamide acetamide and replacing 1-(4-amino-1-piperidyl)ethanone with tetrahydropyran-4-amine in step (c). Product was obtained as a white solid (formic acid salt). LC/MS: *m*/z found 704, 706 [M+H] ⁺, retention time = 2.12 min (Method C). ¹H NMR (400 MHz, Methanol-*d*₄) δ 8.66 - 8.62 (m, 1H), 8.54 (s, 1H), 7.76 (d, 1H), 7.71 (d, 1H), 7.54 (t, 1H), 7.46 (t, 2H), 7.40 (d, 1H), 7.34 (s, 1H), 7.31 (d, 1H), 7.25 (d, 1H), 4.11 (s, 2H), 4.02 (s, 1H), 3.99 (d, 4H), 3.96 (s, 3H), 3.63 (d, 3H), 3.43 (t, 2H), 3.11 (s, 1H), 2.95 (d, 2H), 2.25 (t, 2H), 2.01 (d, 2H), 1.91 (d, 3H), 1.86 (d, 2H), 1.57 (dd, 4H).

### Example 230: 2-(1-((6-(2-Chloro-3-(3-chloro-2-(4-((3-(2-hydroxypropan-2-yl)azetidin-1-yl)methyl)-3-methoxyphenyl)pyridin-4-yl)phenyl)-2-methoxypyridin-3-yl)methyl)azetidin-3-yl)propan-2-ol (471)

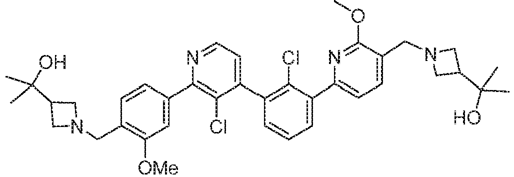

2-(1-((6-(2-Chloro-3-(3-chloro-2-(4-((3-(2-hydroxypropan-2-yl)azetidin-1-yl)methyl)-3-methoxyphenyl)pyridin-4-yl)phenyl)-2-methoxypyridin-3-yl)methyl)azetidin-3-yl)propan-2-ol was prepared in similar manner as described for Example 11, utilizing intermediate 6-(2-chloro-3-(3-chloro-2-(4-formyl-3-methoxyphenyl)pyridin-4-yl)phenyl)-2-methoxynicotinaldehyde and replacing (5S)-5-(aminomethyl)pyrrolidin-2-one with 2-(azetidin-3-yl)propan-2-ol in step (b). Product was obtained as a white solid (formic acid salt). LC/MS: *m*/z found 691, 693 [M+H] ⁺, retention time = 2.25 min (Method C). ¹H NMR (400 MHz, Methanol-*d*₄) δ 8.66 (d, 1H), 8.52 (s, 1H), 7.85 - 7.80 (m, 1H), 7.72 (d, 1H), 7.56 (dd, 1H), 7.50 (d, 1H), 7.47 (d, 1H), 7.43 (d, 1H), 7.35 (dd, 3H), 4.39 (s, 2H), 4.22 (s, 2H), 4.13 - 4.03 (m, 7H), 3.98 (d, 7H), 2.83 (m, 2H), 1.13 (t, 12H).

### Example 231: 2-((6-(3-(2-(4-((6-Acetyl-2,6-diazaspiro[3.3]heptan-2-yl)methyl)-3-methoxyphenyl)-3-chloropyridin-4-yl)-2-chlorophenyl)-2-methoxypyridin-3-yl)methyl)-2,6-diazaspiro[3.4]octan-7-one (493)

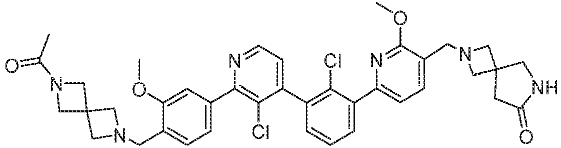

### (a) 2-((6-(2-chloro-3-(2,3-dichloropyridin-4-yl)phenyl)-2-methoxypyridin-3-yl)methyl)-2,6-diazaspiro [3.4] octan-7-one

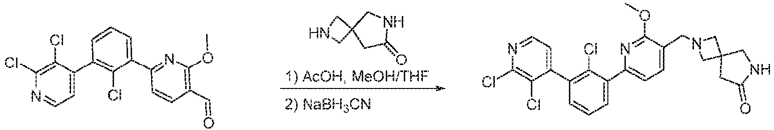

6-[2-Chloro-3-(2,3-dichloro-4-pyridyl)phenyl]-2-methoxy-pyridine-3-carbaldehyde (400 mg, 1.02 mmol), acetic acid (61 mg, 1.02 mmol), and 2,6-diazaspiro[3.4]octan-7-one (179 mg, 1.42 mmol) were dissolved in MeOH/THF (1:1, 10 mL), then the solution was stirred at 25 °C for 2 hours. Reaction was cooled to 0 °C and sodium cyanoborohydride (89 mg, 1.42 mmol) was added portion-wise over 3 minutes. The resulting mixture was warmed to 25 °C and stirred for an additional 1 hour. Reaction was diluted with 15 mL water and extracted with EtOAc (3 x 10 mL). The combined organics were washed with water, brine, dried over sodium sulfate, filtered and concentrated under reduced pressure to afford 2-((6-(2-chloro-3-(2,3-dichloropyridin-4-yl)phenyl)-2-methoxypyridin-3-yl)methyl)-2,6-diazaspiro[3.4]octan-7-one (520 mg, crude) as a yellow oil. MS: *m*/z found 503, 505 [M+H] ⁺. Material was used for the next step without further purification.

### (b) 4-(3-chloro-4-(2-chloro-3-(6-methoxy-5-((7-oxo-2,6-diazaspiro[3.4]octan-2-yl)methyl)pyridin-2-yl)phenyl)pyridin-2-yl)-2-methoxybenzaldehyde

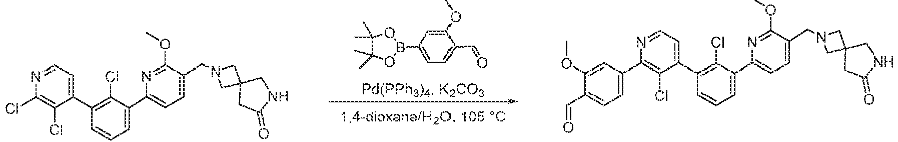

Tetrakis(triphenylphosphine)palladium(0) (115 mg, 0.10 mmol), potassium carbonate (205 mg, 1.49 mmol), 2-((6-(2-chloro-3-(2,3-dichloropyridin-4-yl)phenyl)-2-methoxypyridin-3-yl)methyl)-2,6-diazaspiro[3.4]octan-7-one (250 mg, 0.50 mmol, crude), and 2-methoxy-4-(4,4,5,5-tetramethyl-1,3,2-dioxaborolan-2-yl)benzaldehyde (169 mg, 0.65 mmol) were suspended in 1,4-dioxane/water (4:1, 12 mL), then the solution was heated at 105 °C for 20 minutes. Reaction was cooled to room temperature, diluted with 20 mL water, and extracted with EtOAc (3 x 10 mL). The combined organics were concentrated under reduced pressure and the crude sample was purified by silica gel chromatography (0-10 % DCM/MeOH) to afford 4-(3-chloro-4-(2-chloro-3-(6-methoxy-5-((7-oxo-2,6-diazaspiro[3.4]octan-2-yl)methyl)pyridin-2-yl)phenyl)pyridin-2-yl)-2-methoxybenzaldehyde (157 mg, 52 % yield) as a yellow foam. MS: *m*/z found 603, 605 [M+H] ⁺.

### (c) 2-((6-(3-(2-(4-((6-acetyl-2,6-diazaspiro[3.3]heptan-2-yl)methyl)-3-methoxyphenyl)-3-chloropyridin-4-yl)-2-chlorophenyl)-2-methoxypyridin-3-yl)methyl)-2,6-diazaspiro[3.4]octan-7-one

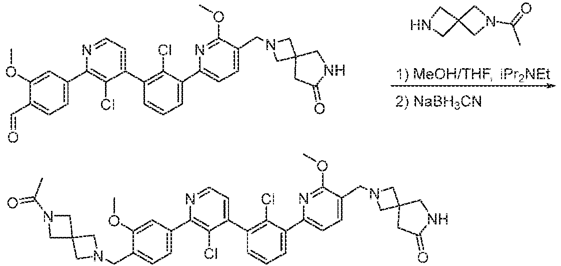

4-(3-Chloro-4-(2-chloro-3-(6-methoxy-5-((7-oxo-2,6-diazaspiro[3.4]octan-2-yl)methyl)pyridin-2-yl)phenyl)pyridin-2-yl)-2-methoxybenzaldehyde (19 mg, 0.03 mmol), 1-(2,6-diazaspiro[3.3]heptan-2-yl)ethenone hydrochloride (11 mg, 0.06 mmol), and N,N-diisopropylethylamine (12 mg, 0.09 mmol) were dissolved in MeOH/THF (1:1, 1 mL), then the solution was stirred at 25 °C for 18 hours. Sodium cyanoborohydride (6 mg, 0.09 mmol) was added, and the resulting mixture was stirred at 25 °C for 1 hour. Reaction was filtered through celite, and the filtrate was concentrated under reduced pressure. Crude sample was purified by reverse phase HPLC to afford 2-((6-(3-(2-(4-((6-acetyl-2,6-diazaspiro[3.3]heptan-2-yl)methyl)-3-methoxyphenyl)-3-chloropyridin-4-yl)-2-chlorophenyl)-2-methoxypyridin-3-yl)methyl)-2,6-diazaspiro[3.4]octan-7-one (8 mg, 33 % yield) as a white powder (formic acid salt). LC/MS: *m*/z found 727, 729 [M+H]⁺, retention time = 2.09 min (Method C). ¹H NMR (400 MHz, Methanol-*d*₄) δ 8.64 (d, 1H), 7.72 (dd, 2H), 7.55 (t, 1H), 7.47 (d, 2H), 7.41 (d, 1H), 7.39 - 7.30 (m, 2H), 7.27 (d, 1H), 4.35 (s, 2H), 4.25 (s, 2H), 4.12 (s, 5H), 4.01 (d, 3H), 3.96 (d, 3H), 3.91 (s, 3H), 3.68 - 3.62 (m, 4H), 3.61 (t, 2H), 2.62 (d, 2H), 1.84 (s, 3H).

### Example 232: 2-((6-(3-(2-(4-((2,6-Diazaspiro[3.3]heptan-2-yl)methyl)-3-methoxyphenyl)-3-chloropyridin-4-yl)-2-chlorophenyl)-2-methoxypyridin-3-yl)methyl)-2,6-diazaspiro[3.4]-octan-7-one (494)

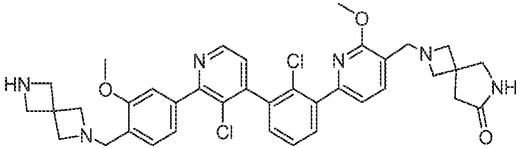

2-((6-(3-(2-(4-((2,6-Diazaspiro[3.3]heptan-2-yl)methyl)-3-methoxyphenyl)-3-chloropyridin-4-yl)-2-chlorophenyl)-2-methoxypyridin-3-yl)methyl)-2,6-diazaspiro[3.4]-octan-7-one was isolated as a by-product from reaction (c) in Example 231. Compound was obtained as a white solid (formic acid salt). LC/MS: *m*/*z* found 685, 687 [M+H] ⁺, retention time = 2.19 min (Method C). ¹H NMR (400 MHz, Methanol-*d*₄) δ 8.63 (d, 1H), 8.39 (s, 4H), 7.78 (d, 1H), 7.73 - 7.67 (m, 1H), 7.55 (t, 1H), 7.46 - 7.37 (m, 3H), 7.29 (d, 3H), 4.19 (s, 4H), 4.03 (d, 5H), 3.96 (s, 2H), 3.92 (s, 3H), 3.81 (d, 8H), 3.63 (s, 2H), 2.64 (s, 2H)

### Example 233: 2-((6-(2-Chloro-3-(3-chloro-2-(3-methoxy-4-(((tetrahydro-2H-pyran-4-yl)amino)methyl)phenyl)pyridin-4-yl)phenyl)-2-methoxypyridin-3-yl)methyl)-2,6-diazaspiro[3.4]octan-7-one (495)

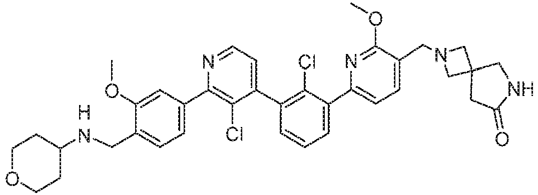

2-((6-(2-Chloro-3-(3-chloro-2-(3-methoxy-4-(((tetrahydro-2H-pyran-4-yl)amino)methyl)phenyl)pyridin-4-yl)phenyl)-2-methoxypyridin-3-yl)methyl)-2,6-diazaspiro[3.4]octan-7-one was prepared in similar manner as described for Example 231, utilizing intermediate 4-(3-chloro-4-(2-chloro-3-(6-methoxy-5-((7-oxo-2,6-diazaspiro[3.4]octan-2-yl)methyl)pyridin-2-yl)phenyl)pyridin-2-yl)-2-methoxybenzaldehyde and replacing 1-(2,6-diazaspiro[3.3]heptan-2-yl)ethenone hydrochloride with tetrahydropyran-4-amine in step (c). Product was obtained as a white solid (formic acid salt). LC/MS: *m*/*z* found 688, 690 [M+H] ⁺, retention time = 2.13 min (Method C). ¹H NMR (400 MHz, Methanol-*d*₄) δ 8.65 (d, 1H), 8.51 (s, 1H), 7.70 (d, 2H), 7.58 - 7.49 (m, 2H), 7.47 (d, 1H), 7.43 - 7.37 (m, 2H), 7.34 (d, 1H), 7.27 - 7.21 (m, 1H), 4.27 (s, 2H), 4.04 (d, 2H), 3.99 (t, 6H), 3.75 (s, 2H), 3.58 (d, 2H), 3.46 (d, 6H), 3.40 (d, 1H), 2.58 (s, 2H), 2.10 (d, 2H), 1.70 (d, 2H).

### Example 234: 2-((6-(2-Chloro-3-(3-chloro-2-(4-((((1r,3r)-3-hydroxy-3-methylcyclobutyl)amino)methyl)-3-methoxyphenyl)pyridin-4-yl)phenyl)-2-methoxypyridin-3-yl)methyl)-2,6-diazaspiro[3.4]octan-7-one (496)

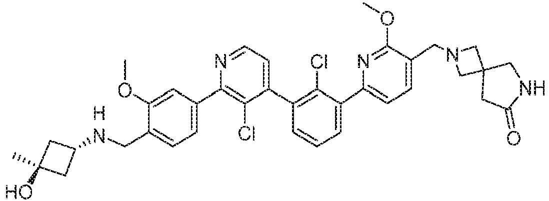

2-((6-(2-Chloro-3-(3-chloro-2-(4-((((1r,3r)-3-hydroxy-3-methylcyclobutyl)amino)methyl)-3-methoxyphenyl)pyridin-4-yl)phenyl)-2-methoxypyridin-3-yl)methyl)-2,6-diazaspiro[3.4]octan-7-one was prepared in similar manner as described for Example 231, utilizing intermediate 4-(3-chloro-4-(2-chloro-3-(6-methoxy-5-((7-oxo-2,6-diazaspiro[3.4]octan-2-yl)methyl)pyridin-2-yl)phenyl)pyridin-2-yl)-2-methoxybenzaldehyde and replacing 1-(2,6-diazaspiro[3.3]heptan-2-yl)ethenone hydrochloride with 3-amino-1-methyl-cyclobutanol in step (c). Product was obtained as a white solid (formic acid salt). LC/MS: *m*/*z* found 688, 690 [M+H] ⁺, retention time = 2.06 min (Method C). ¹H NMR (400 MHz, Methanol-*d*₄) δ 8.65 (d, 1H), 8.47 (s, 1H), 7.71 (t, 2H), 7.54 (t, 1H), 7.48 (dd, 2H), 7.40 (d, 2H), 7.34 (d, 1H), 7.25 (d, 1H), 4.17 (d, 2H), 3.99 (t, 6H), 3.97 - 3.88 (m, 1H), 3.82 (s, 2H), 3.59 (d, 2H), 3.55 (s, 4H), 2.60 (s, 2H), 2.43 - 2.33 (m, 2H), 2.19 (t, 2H), 1.38 (d, 3H).

### Example 235: 2-((6-(2-Chloro-3-(3-chloro-2-(4-((6-hydroxy-2-azaspiro[3.3]heptan-2-yl)methyl)-3-methoxyphenyl)pyridin-4-yl)phenyl)-2-methoxypyridin-3-yl)methyl)-2,6-diazaspiro[3.4]octan-7-one (497)

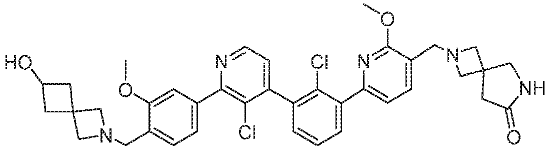

2-((6-(2-Chloro-3-(3-chloro-2-(4-((6-hydroxy-2-azaspiro[3.3]heptan-2-yl)methyl)-3-methoxyphenyl)pyridin-4-yl)phenyl)-2-methoxypyridin-3-yl)methyl)-2,6-diazaspiro[3.4]octan-7-one was prepared in similar manner as described for Example 231, utilizing intermediate 4-(3-chloro-4-(2-chloro-3-(6-methoxy-5-((7-oxo-2,6-diazaspiro[3.4]octan-2-yl)methyl)pyridin-2-yl)phenyl)pyridin-2-yl)-2-methoxybenzaldehyde and replacing 1-(2,6-diazaspiro[3.3]heptan-2-yl)ethenone hydrochloride with 2-azaspiro[3.3]heptan-6-ol in step (c). Product was obtained as a white solid (formic acid salt). LC/MS: *m*/*z* found 700, 702 [M+H] ⁺, retention time = 2.08 min (Method C). ¹H NMR (400 MHz, Methanol-*d*₄) δ 8.65 (d, 1H), 8.43 (s, 2H), 7.72 (dd, 2H), 7.55 (t, 1H), 7.48 (dd, 2H), 7.43 - 7.37 (m, 2H), 7.34 (d, 1H), 7.26 (d, 1H), 4.39 (s, 2H), 4.14 (d, 5H), 4.02 - 3.99 (m, 3H), 3.97 (d, 3H), 3.88 (s, 2H), 3.61 (d, 6H), 2.61 (s, 4H), 2.15 (t, 2H).

### Example 236: 2-((6-(2-Chloro-3-(3-chloro-2-(4-((3-hydroxy-3-methylazetidin-1-yl)methyl)-3-methoxyphenyl)pyridin-4-yl)phenyl)-2-methoxypyridin-3-yl)methyl)-2,6-diazaspiro[3.4]octan-7-one (498)

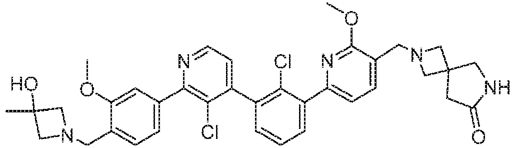

2-((6-(2-Chloro-3-(3-chloro-2-(4-((3-hydroxy-3-methylazetidin-1-yl)methyl)-3-methoxyphenyl)pyridin-4-yl)phenyl)-2-methoxypyridin-3-yl)methyl)-2,6-diazaspiro[3.4]octan-7-one was prepared in similar manner as described for Example 231, utilizing intermediate 4-(3-chloro-4-(2-chloro-3-(6-methoxy-5-((7-oxo-2,6-diazaspiro[3.4]octan-2-yl)methyl)pyridin-2-yl)phenyl)pyridin-2-yl)-2-methoxybenzaldehyde and replacing 1-(2,6-diazaspiro[3.3]heptan-2-yl)ethenone hydrochloride with 3-methylazetidin-3-ol in step (c). Product was obtained as a white solid (formic acid salt). LC/MS: *m*/*z* found 674, 676 [M+H] ⁺, retention time = 2.08 min (Method C). ¹H NMR (400 MHz, Methanol-*d*₄) δ 8.65 (d, 1H), 8.46 (s, 1H), 7.71 (t, 2H), 7.54 (t, 1H), 7.48 (dd, 2H), 7.43 - 7.37 (m, 2H), 7.34 (d, 1H), 7.25 (d, 1H), 4.40 (s, 2H), 4.05 (d, 2H), 4.00 (t, 3H), 3.98 - 3.91 (m, 5H), 3.82 (d, 2H), 3.59 (t, 2H), 3.55 (s, 4H), 2.60 (s, 2H), 1.52 (d, 3H).

### Example 237: 2-((6-(2-Chloro-3-(3-chloro-2-(4-((((1r,4r)-4-hydroxycyclohexyl)-amino)-methyl)-3-methoxyphenyl)pyridin-4-yl)phenyl)-2-methoxypyridin-3-yl)methyl)-2,6-diazaspiro[3.4]octan-7-one (502)

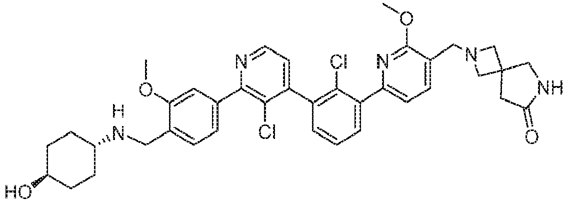

2-((6-(2-Chloro-3-(3-chloro-2-(4-((((1r,4r)-4-hydroxycyclohexyl)-amino)-methyl)-3-methoxyphenyl)pyridin-4-yl)phenyl)-2-methoxypyridin-3-yl)methyl)-2,6-diazaspiro[3.4]octan-7-one was prepared in similar manner as described for Example 231, utilizing intermediate 4-(3-chloro-4-(2-chloro-3-(6-methoxy-5-((7-oxo-2,6-diazaspiro[3.4]octan-2-yl)methyl)pyridin-2-yl)phenyl)pyridin-2-yl)-2-methoxybenzaldehyde and replacing 1-(2,6-diazaspiro[3.3]heptan-2-yl)ethenone hydrochloride with (1r,4r)-4-aminocyclohexan-1-ol in step (c). Product was obtained as a white solid (formic acid salt). LC/MS: *m*/*z* found 702, 704 [M+H] ⁺, retention time = 2.07 min (Method C). ¹H NMR (400 MHz, Methanol-*d*₄) δ 8.65 (d, 1H), 8.45 (s, 2H), 7.71 (t, 2H), 7.58 - 7.49 (m, 2H), 7.47 (d, 1H), 7.40 (d, 2H), 7.34 (d, 1H), 7.25 (d, 1H), 4.28 (s, 2H), 3.99 (dd, 6H), 3.85 (s, 2H), 3.59 (d, 7H), 3.15 (s, 1H), 2.60 (d, 2H), 2.23 (d, 2H), 2.07 (d, 2H), 1.53 (q, 2H), 1.38 (t, 2H).

### Example 238: 2-((6-(2-Chloro-3-(3-chloro-2-(4-((((1s,4s)-4-hydroxycyclohexyl)-amino)-methyl)-3-methoxyphenyl)pyridin-4-yl)phenyl)-2-methoxypyridin-3-yl)methyl)-2,6-diazaspiro[3.4]octan-7-one (503)

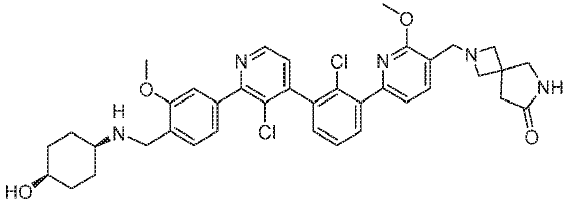

2-((6-(2-Chloro-3-(3-chloro-2-(4-((((1s,4s)-4-hydroxycyclohexyl)-amino)-methyl)-3-methoxyphenyl)pyridin-4-yl)phenyl)-2-methoxypyridin-3-yl)methyl)-2,6-diazaspiro[3.4]octan-7-one was prepared in similar manner as described for Example 231, utilizing intermediate 4-(3-chloro-4-(2-chloro-3-(6-methoxy-5-((7-oxo-2,6-diazaspiro[3.4]octan-2-yl)methyl)pyridin-2-yl)phenyl)pyridin-2-yl)-2-methoxybenzaldehyde and replacing 1-(2,6-diazaspiro[3.3]heptan-2-yl)ethenone hydrochloride with (1s,4s)-4-aminocyclohexan-1-ol in step (c). Product was obtained as a white solid (formic acid salt). LC/MS: *m*/*z* found 702, 704 [M+H] ⁺, retention time = 2.13 min (Method C). ¹H NMR (400 MHz, Methanol-*d*₄) δ 8.65 (d, 1H), 8.48 (s, 1H), 7.73 - 7.67 (m, 2H), 7.54 (dd, 2H), 7.49 - 7.45 (m, 1H), 7.40 (d, 2H), 7.34 (d, 1H), 7.25 (d, 1H), 4.29 (s, 2H), 4.03 - 3.94 (m, 7H), 3.84 - 3.78 (m, 2H), 3.59 (s, 2H), 3.53 (s, 4H), 3.18 (s, 1H), 2.59 (s, 2H), 1.92 (d, 6H), 1.64 (d, 2H).

### Example 239: 2-(4-(3-Chloro-4-(2-chloro-3-(5-((((1r,4r)-4-hydroxycyclohexyl)-amino)methyl)-6-methoxypyridin-2-yl)phenyl)pyridin-2-yl)-2-methoxybenzyl)-2,6-diazaspiro[3.4]octan-7-one (515)

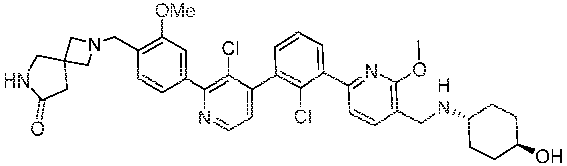

### (a) 2-(2-methoxy-4-(4,4,5,5-tetramethyl-1,3,2-dioxaborolan-2-yl)benzyl)-2,6-diazaspiro[3.4]octan-7-one

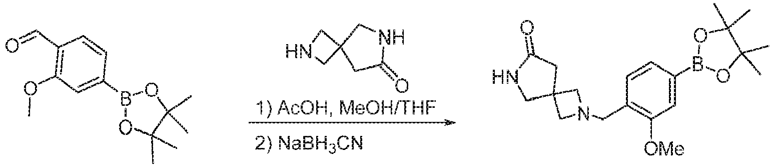

2-Methoxy-4-(4,4,5,5-tetramethyl-1,3,2-dioxaborolan-2-yl)benzaldehyde (300 mg, 1.14 mmol), acetic acid (69 mg, 1.14 mmol), and 2,6-diazaspiro[3.4]octan-7-one (188 mg, 1.49 mmol) were dissolved in MeOH/THF (1:1, 10 mL), then the solution was stirred at 25 °C for 2 hours. Reaction was cooled to 0 °C and sodium cyanoborohydride (144 mg, 2.29 mmol) was added portion-wise over 3 minutes. The resulting mixture was warmed to 25 °C and stirred for an additional 1 hour. Reaction was concentrated under reduced pressure and the residue was dissolved in 15 mL DCM. The organic solution was washed with water (2 x 15 mL) and brine solution, then dried over sodium sulfate. Solution was concentrated under reduced pressure to afford 2-(2-methoxy-4-(4,4,5,5-tetramethyl-1,3,2-dioxaborolan-2-yl)benzyl)-2,6-diazaspiro[3.4]octan-7-one (423 mg, crude) as a yellow oil MS: *m*/*z* found 373 [M+H] ⁺. Material was used for the next step without further purification.

### (b) 6-(2-chloro-3-(3-chloro-2-(3-methoxy-4-((7-oxo-2,6-diazaspiro[3.4]octan-2-yl)-methyl)-phenyl)pyridin-4-yl)phenyl)-2-methoxynicotinaldehyde

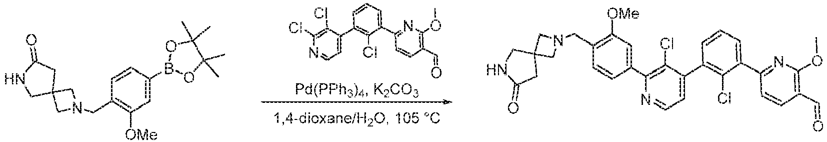

Tetrakis(triphenylphosphine)palladium(0) (176 mg, 0.15 mmol), potassium carbonate (316 mg, 2.29 mmol), 6-[2-chloro-3-(2,3-dichloro-4-pyridyl)phenyl]-2-methoxy-pyridine-3-carbaldehyde (300 mg, 0.76 mmol), and 2-(2-methoxy-4-(4,4,5,5-tetramethyl-1,3,2-dioxaborolan-2-yl)benzyl)-2,6-diazaspiro[3.4]octan-7-one (369 mg, 0.99 mmol, crude) were suspended in 1,4-dioxane/water (4:1, 10 mL), then the solution was heated at 105 °C for 20 minutes. Reaction was cooled to room temperature, diluted with 15 mL water, and extracted with EtOAc (3 x 10 mL). The combined organics were concentrated under reduced pressure and the crude sample was purified by silica gel chromatography (2-20% DCM/MeOH) to afford 6-(2-chloro-3-(3-chloro-2-(3-methoxy-4-((7-oxo-2,6-diazaspiro[3.4]octan-2-yl)-methyl)-phenyl)pyridin-4-yl)phenyl)-2-methoxynicotinaldehyde (119 mg, 26 % yield) as a yellow oil. MS: *m*/*z* found 603, 605 [M+H] ⁺.

### (c) 2-(4-(3-chloro-4-(2-chloro-3-(5-((((1r,4r)-4-hydroxycyclohexyl)-amino)methyl)-6-methoxypyridin-2-yl)phenyl)pyridin-2-yl)-2-methoxybenzyl)-2,6-diazaspiro[3.4]octan-7-one

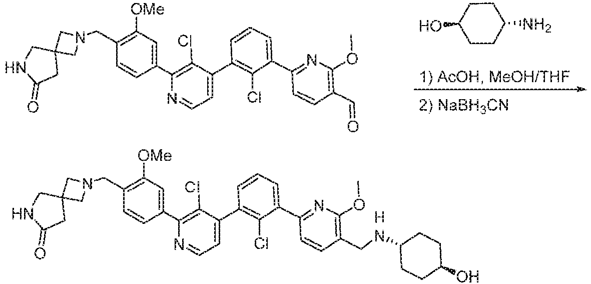

6-(2-Chloro-3-(3-chloro-2-(3-methoxy-4-((7-oxo-2,6-diazaspiro[3.4]octan-2-yl)-methyl)-phenyl)pyridin-4-yl)phenyl)-2-methoxynicotinaldehyde (19 mg, 0.03 mmol), acetic acid (2 mg, 0.03 mmol), and (1r,4r)-4-aminocyclohexan-1-ol (8 mg, 0.06 mmol) were dissolved in MeOH/THF (1:1, 1 mL), then the solution was stirred at 25 °C for 2 hours. Sodium cyanoborohydride (4 mg, 0.06 mmol) was added and the resulting mixture was stirred at 25 °C for 1 hour. Reaction was filtered through celite, and the filtrate was concentrated under reduced pressure. Crude sample was purified by reverse phase HPLC to afford 2-(4-(3-chloro-4-(2-chloro-3-(5-((((1r,4r)-4-hydroxycyclohexyl)-amino)methyl)-6-methoxypyridin-2-yl)phenyl)pyridin-2-yl)-2-methoxybenzyl)-2,6-diazaspiro[3.4]octan-7-one (13 mg, 60 % yield) as a white solid (formic acid salt). LC/MS: *m*/*z* found 702, 704 [M+H] ⁺, retention time = 2.05 min (Method C). ¹H NMR (400 MHz, Methanol-*d*₄) δ 8.63 (d, 1H), 8.49 (s, 2H), 7.87 (d, 1H), 7.71 (d, 1H), 7.57 (t, 1H), 7.43 (t, 3H), 7.34 (d, 1H), 7.29 (d, 2H), 4.23 (s, 2H), 4.07 (d, 3H), 4.02 (s, 2H), 3.92 (d, 3H), 3.71 (s, 4H), 3.60 (s, 3H), 3.11 (s, 1H), 2.62 (d, 2H), 2.21 (d, 2H), 2.07 (d, 2H), 1.50 (q, 2H), 1.38 (t, 2H).

### Example 240: 2-(4-(3-Chloro-4-(2-chloro-3-(5-((((1s,4s)-4-hydroxycyclohexyl)amino)-methyl)-6-methoxypyridin-2-yl)phenyl)pyridin-2-yl)-2-methoxybenzyl)-2,6-diazaspiro[3.4]octan-7-one (516)

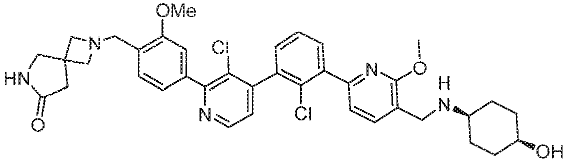

2-(4-(3-Chloro-4-(2-chloro-3-(5-((((1s,4s)-4-hydroxycyclohexyl)amino)-methyl)-6-methoxypyridin-2-yl)phenyl)pyridin-2-yl)-2-methoxybenzyl)-2,6-diazaspiro[3.4]octan-7-one was prepared in similar manner as described for Example 239, utilizing intermediate 6-(2-chloro-3-(3-chloro-2-(3-methoxy-4-((7-oxo-2,6-diazaspiro[3.4]octan-2-yl)-methyl)-phenyl)pyridin-4-yl)phenyl)-2-methoxynicotinaldehyde and replacing (1r,4r)-4-aminocyclohexan-1-ol with (1s,4s)-4-aminocyclohexan-1-ol in step (c). Product was obtained as a white solid (formic acid salt). LC/MS: *m*/*z* found 702, 704 [M+H] ⁺, retention time = 2.10 min (Method C). ¹H NMR (400 MHz, Methanol-*d*₄) δ 8.63 (d, 1H), 8.50 (d, 1H), 7.88 (d, 1H), 7.71 (d, 1H), 7.57 (t, 1H), 7.43 (dd, 3H), 7.35 (d, 1H), 7.28 (d, 2H), 4.25 (s, 2H), 4.08 (d, 3H), 3.98 (d, 3H), 3.92 (d, 3H), 3.65 (s, 4H), 3.59 (d, 2H), 3.17 (s, 1H), 2.60 (d, 2H), 1.91 (d, 6H), 1.64 (d, 2H).

### Example 241: 2-(4-(3-Chloro-4-(2-chloro-3-(5-((((1r,3r)-3-hydroxy-3-methylcyclobutyl)-amino)methyl)-6-methoxypyridin-2-yl)phenyl)pyridin-2-yl)-2-methoxybenzyl)-2,6-diazaspiro[3.4]octan-7-one (517)

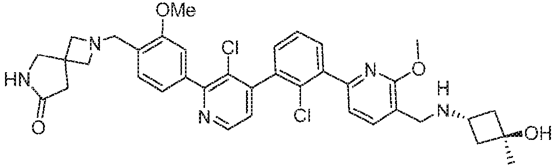

2-(4-(3-Chloro-4-(2-chloro-3-(5-((((1r,3r)-3-hydroxy-3-methylcyclobutyl)amino)methyl)-6-methoxypyridin-2-yl)phenyl)pyridin-2-yl)-2-methoxybenzyl)-2,6-diazaspiro[3.4]octan-7-one was prepared in similar manner as described for Example 239, utilizing intermediate 6-(2-chloro-3-(3-chloro-2-(3-methoxy-4-((7-oxo-2,6-diazaspiro[3.4]octan-2-yl)-methyl)-phenyl)pyridin-4-yl)phenyl)-2-methoxynicotinaldehyde and replacing (1r,4r)-4-aminocyclohexan-1-ol with 3-amino-1-methyl-cyclobutanol in step (c). Product was obtained as a white solid (formic acid salt). LC/MS: *m*/*z* found 688, 690 [M+H] ⁺, retention time = 2.04 min (Method C). ¹H NMR (400 MHz, Methanol-*d*₄) δ 8.63 (t, 1H), 8.51 (d, 1H), 7.82 (d, 1H), 7.74 - 7.68 (m, 1H), 7.56 (t, 1H), 7.48 - 7.37 (m, 3H), 7.35 - 7.24 (m, 3H), 4.06 (t, 3H), 4.03 (s, 2H), 3.95 (s, 2H), 3.92 (d, 3H), 3.80 (d, 1H), 3.69 - 3.51 (m, 6H), 2.61 (d, 2H), 2.34 (d, 2H), 2.11 (d, 2H), 1.41 - 1.29 (m, 3H).

### Example 242: 2-(4-(3-Chloro-4-(2-chloro-3-(5-((3-hydroxy-3-methylazetidin-1-yl)methyl)-6-methoxypyridin-2-yl)phenyl)pyridin-2-yl)-2-methoxybenzyl)-2,6-diazaspiro[3.4]octan-7-one (518)

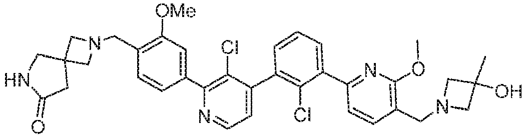

2-(4-(3-Chloro-4-(2-chloro-3-(5-((3-hydroxy-3-methylazetidin-1-yl)methyl)-6-methoxypyridin-2-yl)phenyl)pyridin-2-yl)-2-methoxybenzyl)-2,6-diazaspiro[3.4]octan-7-one was prepared in similar manner as described for Example 239, utilizing intermediate 6-(2-chloro-3-(3-chloro-2-(3-methoxy-4-((7-oxo-2,6-diazaspiro[3.4]octan-2-yl)-methyl)-phenyl)pyridin-4-yl)phenyl)-2-methoxynicotinaldehyde and replacing (1r,4r)-4-aminocyclohexan-1-ol with 3-methylazetidin-3-ol in step (c). Product was obtained as a white solid (formic acid salt). LC/MS: *m*/*z* found 674, 676 [M+H] ⁺, retention time = 2.06 min (Method C). ¹H NMR (400 MHz, Methanol-*d*₄) δ 8.64 (m, 1H), 8.46 (d, 2H), 7.81 (d, 1H), 7.71 (m, 1H), 7.56 (dd, 1H), 7.51 - 7.40 (m, 3H), 7.33 (dd, 3H), 4.21 (s, 4H), 4.04 (t, 3H), 3.97 - 3.87 (m, 9H), 3.76 (d, 2H), 3.64 (d, 2H), 2.67 (d, 2H), 1.51 (d, 3H).

### Example 243: 2-(4-(4-(3-(5-((6-Acetyl-2,6-diazaspiro[3.3]heptan-2-yl)methyl)-6-methoxypyridin-2-yl)-2-chlorophenyl)-3-chloropyridin-2-yl)-2-methoxybenzyl)-2,6-diazaspiro[3.4]octan-7-one (519)

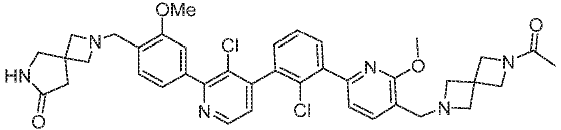

2-(4-(4-(3-(5-((6-Acetyl-2,6-diazaspiro[3.3]heptan-2-yl)methyl)-6-methoxypyridin-2-yl)-2-chlorophenyl)-3-chloropyridin-2-yl)-2-methoxybenzyl)-2,6-diazaspiro[3.4]octan-7-one was prepared in similar manner as described for Example 239, utilizing intermediate 6-(2-chloro-3-(3-chloro-2-(3-methoxy-4-((7-oxo-2,6-diazaspiro[3.4]octan-2-yl)-methyl)-phenyl)pyridin-4-yl)phenyl)-2-methoxynicotinaldehyde and replacing (1r,4r)-4-aminocyclohexan-1-ol with 1-(2,6-diazaspiro[3.3]heptan-2-yl)ethanone in step (c). Product was obtained as a white solid (formic acid salt). LC/MS: *m*/*z* found 727, 729 [M+H] ⁺, retention time = 2.06 min (Method C). ¹H NMR (400 MHz, Methanol-*d*₄) δ 8.65 (d, 1H), 8.42 (s, 2H), 7.75 (d, 1H), 7.70 (d, 1H), 7.55 (t, 1H), 7.47 (dd, 2H), 7.42 (d, 1H), 7.37 - 7.30 (m, 2H), 7.28 (d, 1H), 4.33 (s, 2H), 4.29 (s, 2H), 4.08 (s, 2H), 4.05 - 4.00 (m, 7H), 3.97 - 3.94 (m, 5H), 3.84 (s, 4H), 3.65 (s, 2H), 2.69 (d, 2H), 1.84 (d, 3H).

### Example 244: 2-(4-(4-(3-(5-((2,6-Diazaspiro[3.3]heptan-2-yl)methyl)-6-methoxypyridin-2-yl)-2-chlorophenyl)-3-chloropyridin-2-yl)-2-methoxybenzyl)-2,6-diazaspiro[3.4]octan-7-one (520)

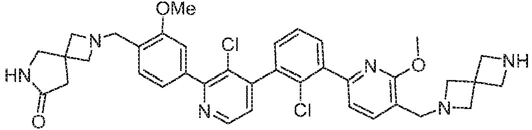

2-(4-(4-(3-(5-((2,6-Diazaspiro[3.3]heptan-2-yl)methyl)-6-methoxypyridin-2-yl)-2-chlorophenyl)-3-chloropyridin-2-yl)-2-methoxybenzyl)-2,6-diazaspiro[3.4]octan-7-one was isolated as a by-product from reaction (c) in Example 243. Compound was obtained as a white solid (formic acid salt). LC/MS: *m*/*z* found 685, 687 [M+H] ⁺, retention time = 2.10 min (Method C). ¹H NMR (400 MHz, Methanol-*d*₄) δ 8.65 (t, 1H), 8.39 (s, 3H), 7.69 (s, 2H), 7.58 - 7.48 (m, 2H), 7.46 (d, 1H), 7.43 - 7.39 (m, 1H), 7.38 - 7.31 (m, 2H), 7.25 (dd, 1H), 4.34 (s, 2H), 4.18 (d, 4H), 4.09 (d, 4H), 3.98 (dd, 6H), 3.75 (d, 2H), 3.65 (dd, 6H), 2.70 (d, 2H).

### Example 245: 2-(4-(3-Chloro-4-(2-chloro-3-(5-((6-hydroxy-2-azaspiro[3.3]heptan-2-yl)methyl)-6-methoxypyridin-2-yl)phenyl)pyridin-2-yl)-2-methoxybenzyl)-2,6-diazaspiro[3.4]octan-7-one (521)

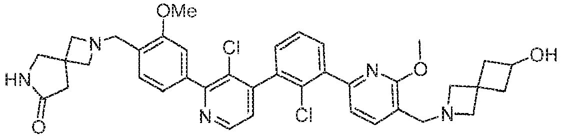

2-(4-(3-Chloro-4-(2-chloro-3-(5-((6-hydroxy-2-azaspiro[3.3]heptan-2-yl)methyl)-6-methoxypyridin-2-yl)phenyl)pyridin-2-yl)-2-methoxybenzyl)-2,6-diazaspiro[3.4]octan-7-one was prepared in similar manner as described for Example 239, utilizing intermediate 6-(2-chloro-3-(3-chloro-2-(3-methoxy-4-((7-oxo-2,6-diazaspiro[3.4]octan-2-yl)-methyl)-phenyl)pyridin-4-yl)phenyl)-2-methoxynicotinaldehyde and replacing (1r,4r)-4-aminocyclohexan-1-ol with 2-azaspiro[3.3]heptan-6-ol in step (c). Product was obtained as a white solid (formic acid salt). LC/MS: *m*/*z* found 700, 702 [M+H] ⁺, retention time = 2.07 min (Method C). ¹H NMR (400 MHz, Methanol-*d*₄) δ 8.63 (t, 1H), 7.81 (d, 1H), 7.70 (d, 1H), 7.56 (dd, 1H), 7.48 - 7.40 (m, 3H), 7.36 - 7.25 (m, 3H), 4.20 (s, 2H), 4.15 - 4.09 (m, 1H), 4.09 - 4.02 (m, 5H), 4.01 - 3.91 (m, 7H), 3.78 (s, 4H), 3.62 (d, 2H), 2.66 - 2.55 (m, 4H), 2.13 (d, 2H).

### Example 246: 1-(4-((6-(3-(2-(4-((4-Acetylpiperazin-1-yl)methyl)-3-methoxyphenyl)-3-chloropyridin-4-yl)-2-chlorophenyl)-2-methoxypyridin-3-yl)methyl)piperazin-1-yl)ethan-1-one (655)

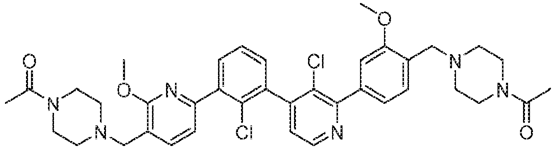

1-(4-((6-(3-(2-(4-((4-Acetylpiperazin-1-yl)methyl)-3-methoxyphenyl)-3-chloropyridin-4-yl)-2-chlorophenyl)-2-methoxypyridin-3-yl)methyl)piperazin-1-yl)ethan-1-one was prepared in similar manner as described for Example 11, replacing (5S)-5-(aminomethyl)pyrrolidin-2-one with 1-(piperazin-1-yl)ethan-1-one in step (b). Product was obtained as a white solid (formic acid salt). LC/MS: *m*/*z* found 717 [M+H]⁺, retention time = 2.57 min (Method A). ¹H NMR (400 MHz, Methanol-*d*₄) δ 8.63 (d, 1H), 8.40 (brs), 7.80 (d, 1H), 7.72 (dd, 1H), 7.55 (dd, 1H), 7.50 - 7.46 (m, 1H), 7.44 (d, 1H), 7.41 (dd, 1H), 7.31 - 7.25 (m, 3H), 3.99 (s, 3H), 3.90 (s, 3H), 3.75 (s, 2H), 3.64 (s, 2H), 3.63 - 3.55 (m, 8H), 2.70 - 2.48 (m, 8H), 2.10 (s, 3H), 2.09 (s, 3H).

### Example 247: 1-(4-(((6-(2-Chloro-3-(3-chloro-2-(3-methoxy-4-(morpholinomethyl) phenyl)pyridin-4-yl)phenyl)-2-methoxypyridin-3-yl)methyl)amino)piperidin-1-yl)ethan-1-one (458)

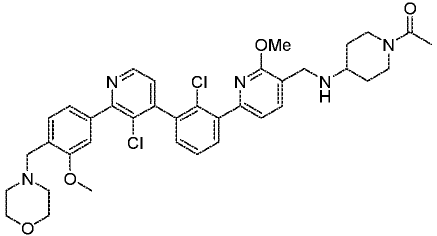

### (a) 1-(4-(((6-(2-Chloro-3-(2,3-dichloropyridin-4-yl)phenyl)-2-methoxypyridin-3-yl)methyl)amino)piperidin-1-yl)ethan-1-one:

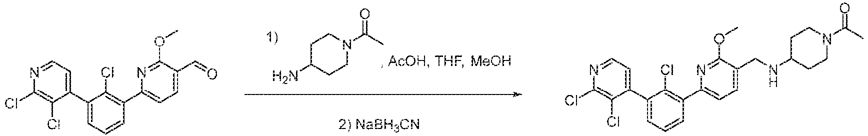

To a mixture of 6-(2-chloro-3-(2,3-dichloropyridin-4-yl)phenyl)-2-methoxynicotinaldehyde (0.30 g, 0.76 mmol), and 1-(4-aminopiperidin-1-yl)ethan-1-one (0.22 g, 1.52 mmol), in 1:1 THF / MeOH (10 mL) was added acetic acid (0.09 g, 1.52 mmol) and 4 A molecular sieve (1 g). The mixture was stirred at room temperature for 3 hrs. Sodium cyanoborohydride (0.12 g, 1.91 mmol) was added and the mixture was stirred at room temperature for 1 hr. The reaction was quenched by addition of water (1 mL). The mixture was diluted with ethyl acetate (50 mL), then washed with saturated aqueous brine solution (30 mL), the aqueous layer extracted with ethyl acetate (3 x 30 mL), then the combined organic layers dried over sodium sulfate, filtered and concentrated under reduced pressure. The residue was purified by normal phase flash SiO₂ chromatography using a linear gradient (0-5 % MeOH/CH₂Cl₂) to afford 1-(4-(((6-(2-chloro-3-(2,3-dichloropyridin-4-yl)phenyl)-2-methoxypyridin-3-yl)methyl)amino)piperidin-1-yl)ethan-1-one (0.36 g, 90% yield). MS: m/z found 519 [M+H]⁺.

### (b) tert-Butyl (1-acetylpiperidin-4-yl)((6-(2-chloro-3-(2,3-dichloropyridin-4-yl)phenyl)-2-methoxypyridin-3-yl)methyl)carbamate

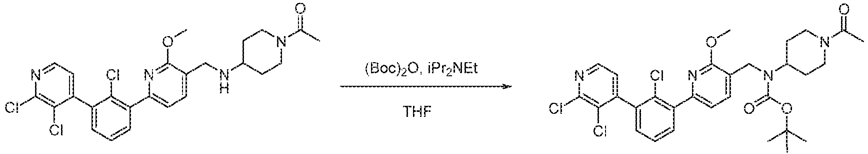

A round bottom flask was charged with 1-(4-(((6-(2-chloro-3-(2,3-dichloropyridin-4-yl)phenyl)-2-methoxypyridin-3-yl)methyl)amino)piperidin-1-yl)ethan-1-one (0.72 g, 1.39 mmol) and di-tert-butyl dicarbonate (0.60 g, 2.77 mmol). The flask was purged with nitrogen for 5 min, then dry THF (30 mL) was added by syringe, followed by N,N-diisopropylethylamine (0.72 ml, 5.54 mmol). The reaction was stirred at room temperature for 3 hrs. The mixture was diluted with ethyl acetate (50 mL), then washed with saturated aqueous brine solution (30 mL), then the aqueous layer extracted with ethyl acetate (3 x 30 mL), the combined organic phases were dried over sodium sulfate, filtered and concentrated under reduced pressure. The residue was purified by normal phase flash SiO₂ chromatography using a linear gradient (0-5 % MeOH/CH₂Cl₂) to afford tert-butyl (1-acetylpiperidin-4-yl)((6-(2-chloro-3-(2,3-dichloropyridin-4-yl)phenyl)-2-methoxypyridin-3-yl)methyl)carbamate (0.86 g, 100% yield). MS: m/z found 619 [M+H]⁺.

### (c) 2-Methoxy-4-(4,4,5,5-tetramethyl-1,3,2-dioxaborolan-2-yl)benzaldehyde

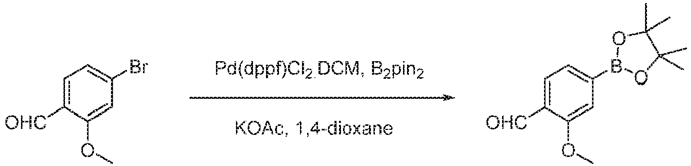

A microwave vial was charged with 4-bromo-2-methoxybenzaldehyde (1 g, 4.65 mmol), bis(pinacolato)diborane (1.30 g, 5.12 mmol), potassium acetate (1.28g, 13.02 mmol), and [1,1'- bis(diphenylphosphino)ferrocene]dichloropalladium(II) complex with dichloromethane (0.38 g, 0.47 mmol). The flask was purged with nitrogen for 5 min, then dry 1,4-dioxane (2 mL) added, and the reaction was sparged with nitrogen for 5 min. The mixture was then stirred at 90 °C for 1 hr. The mixture was filtered through CELITE^{®} and the filter cake was washed with ethyl acetate (3 x 40 mL). The combined organic layers were concentrated under reduced pressure and the residue purified by normal phase flash SiO₂ chromatography using a gradient (0-10% methanol/CH₂Cl₂) to afford 2-methoxy-4-(4,4,5,5-tetramethyl-1,3,2-dioxaborolan-2-yl)benzaldehyde (0.92 g, 75% yield). MS: m/z found 263 [M+H]⁺.

### (d) tert-Butyl (1-acetylpiperidin-4-yl)((6-(2-chloro-3-(3-chloro-2-(4-formyl-3-methoxyphenyl) pyridin-4-yl)phenyl)-2-methoxypyridin-3-yl)methyl)carbamate:

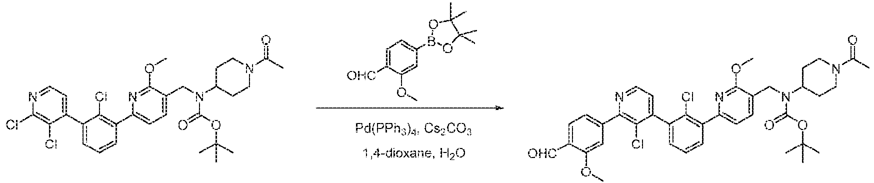

A microwave vial was charged with 2-methoxy-4-(4,4,5,5-tetramethyl-1,3,2-dioxaborolan-2-yl)benzaldehyde (0.22 g, 0.83 mmol), tert-butyl (1-acetylpiperidin-4-yl)((6-(2-chloro-3-(2,3-dichloropyridin-4-yl)phenyl)-2-methoxypyridin-3-yl)methyl)carbamate (0.47 g, 0.76 mmol), cesium carbonate (0.74 g 2.27 mmol), and tetrakis(triphenylphosphine)palladium(0) (0.09 g, 0.08 mmol). 1,4-Dioxane (2 mL) was added, and the reaction was sparged with nitrogen, then water (0.3 ml) added. The mixture was stirred at 90 °C for 30 min thermally, then the mixture filtered through CELITE^{®} and the filter cake was washed with ethyl acetate (3 x 15 mL). The filtrate was concentrated under reduced pressure and the residue was purified by normal phase flash SiO₂ chromatography using a gradient (0-10% methanol/CH₂Cl₂) to afford tert-butyl (1-acetylpiperidin-4-yl)((6-(2-chloro-3-(3-chloro-2-(4-formyl-3-methoxyphenyl) pyridin-4-yl)phenyl)-2-methoxypyridin-3-yl)methyl)carbamate (0.53 g, 96% yield). MS: m/z found 719 [M+H]⁺.

### (e) 1-(4-(((6-(2-Chloro-3-(3-chloro-2-(3-methoxy-4-(morpholinomethyl) phenyl)pyridin-4-yl)phenyl)-2-methoxypyridin-3-yl)methyl)amino)piperidin-1-yl)ethan-1-one

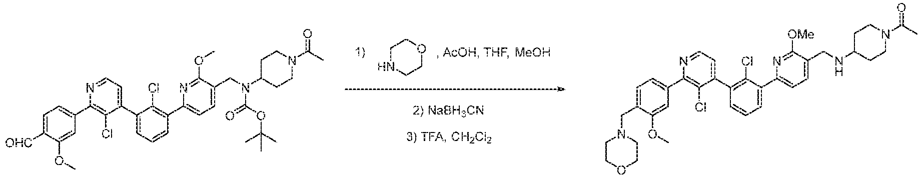

To a mixture of tert-butyl (1-acetylpiperidin-4-yl)((6-(2-chloro-3-(3-chloro-2-(4-formyl-3-methoxyphenyl) pyridin-4-yl)phenyl)-2-methoxypyridin-3-yl)methyl)carbamate (0.06 g, 0.08 mmol) and morpholine (0.01 g, 0.17 mmol), in 1:1 THF / MeOH (10 mL) was added acetic acid (0.01 g, 0.17 mmol) and 4 A molecular sieve (1 g). The mixture was stirred at room temperature for 3 hrs. Sodium cyanoborohydride (0.01 g, 0.211 mmol) was added and the mixture was stirred at room temperature for 1 hr. The reaction was quenched by addition of water (1 mL). The mixture was diluted with ethyl acetate (50 ml), then washed with saturated aqueous brine solution (30 mL), the aqueous layer was extracted with ethyl acetate (3 x 30 mL), then the combined organic phases dried over sodium sulfate and concentrated under reduced pressure. The residue was purified by normal phase flash SiO₂ chromatography using a linear gradient (0-5 % MeOH/CH₂Cl₂) to afford tert-butyl (1-acetylpiperidin-4-yl)((6-(2-chloro-3-(3-chloro-2-(3-methoxy-4-(morpholinomethyl)phenyl)pyridin-4-yl)phenyl)-2-methoxypyridin-3-yl)methyl)carbamate MS: m/z found 790 [M+H]⁺. The protected amine was then diluted with dichloromethane (3 ml), trifluoroacetic acid (1 ml) added and the reaction stirred for 20 min, then concentrated under reduced pressure. The residue purified by reverse phase HPLC (with 0.05% Formic acid modifier) to afford 1-(4-(((6-(2-chloro-3-(3-chloro-2-(3-methoxy-4-(morpholinomethyl) phenyl)pyridin-4-yl)phenyl)-2-methoxypyridin-3-yl)methyl) amino)piperidin-1-yl)ethan-1-one 6.8 mg (12%) as a white solid (formic acid salt). MS: m/z found 690 [M+H]⁺. ¹H NMR (400 MHz, Methanol-*d*₄) δ 8.60 (d, 1H), 8.44 (s, 1H), 7.84 (d, 1H), 7.68 (d, 1H), 7.54 (t, 1H), 7.48 - 7.37 (m, 3H), 7.31 (d, 1H), 7.29 - 7.21 (m, 2H), 4.60 (d, 1H), 4.18 (s, 2H), 4.04 (s, 3H), 4.02 (d, 1H), 3.88 (s, 3H), 3.78 - 3.67 (m, 6H), 3.23 - 3.11 (m, 1H), 2.73 - 2.62 (m, 5H), 2.23 - 2.07 (m, 5H), 1.63 - 1.39 (m, 2H).

### Example 248: 1-(4-(((6-(2-Chloro-3-(3-chloro-2-(4-((4-hydroxypiperidin-1-yl)methyl)-3-methoxyphenyl)pyridin-4-yl)phenyl)-2-methoxypyridin-3-yl)methyl)amino)piperidin-1-yl)ethan-1-one (459)

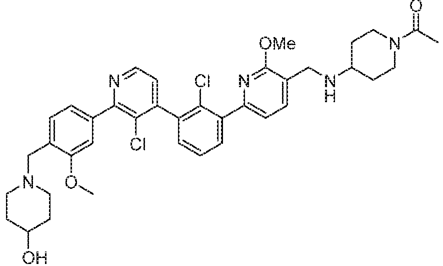

1-(4-(((6-(2-Chloro-3-(3-chloro-2-(4-((4-hydroxypiperidin-1-yl)methyl)-3-methoxphenyl)pyridin-4-yl)phenyl)-2-methoxypyridin-3-yl)methyl)amino)piperidin-1-yl)ethan-1-one was prepared in a similar fashion to Example 247, replacing morpholine with piperidin-4-ol in step (e). MS: m/z found 704 [M+H]⁺. ¹H NMR (400 MHz, Methanol-*d*₄) δ 8.61 (d, 1H), 8.52 (s, 1H), 7.75 (d, 1H), 7.68 (dd, 1H), 7.52 (t, 1H), 7.49 - 7.35 (m, 3H), 7.32 - 7.20 (m, 3H), 4.47 (d, 1H), 4.00 (s, 3H), 3.89 (d, 6H), 3.71 (s, 1H), 3.12 (t, 1H), 3.08 - 3.03 (m, 2H), 2.87 - 2.82 (m, 2H), 2.68 (t, 1H), 2.60 (s, 2H), 2.08 (s, 3H), 2.00 (d, 2H), 1.94 - 1.86 (m, 2H), 1.69 - 1.64 (m, 2H), 1.46 - 1.24 (m, 2H).

### Example 249: 4-(2-((4-(4-(3-(5-(((1-Acetylpiperidin-4-yl)amino)methyl)-6-methoxypyridin-2-yl)-2-chlorophenyl)-3-chloropyridin-2-yl)-2-methoxybenzyl)amino)ethyl)piperazin-2-one (479)

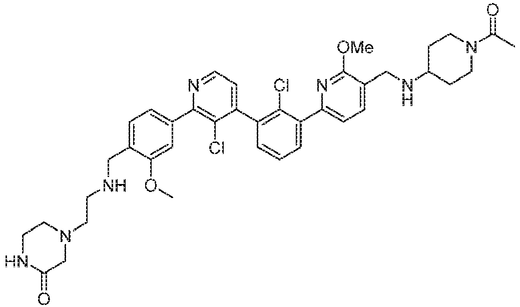

4-(2-((4-(4-(3-(5-(((1-Acetylpiperidin-4-yl)amino)methyl)-6-methoxypyridin-2-yl)-2-chlorophenyl)-3-chloropyridin-2-yl)-2-methoxybenzyl)amino)ethyl)piperazin-2-one was prepared in a similar fashion to Example 247, replacing morpholine with 4-(2-aminoethyl)piperazin-2-one in step (e). MS: m/z found 746 [M+H]⁺. ¹H NMR (400 MHz, Methanol-*d*₄) δ 8.63 (d, 1H), 8.43 (s, 2H), 7.84 (d, 1H), 7.68 (dd, 1H), 7.58 - 7.46 (m, 2H), 7.47 - 7.34 (m, 3H), 7.37 - 7.26 (m, 2H), 4.60 (d, 1H), 4.29 (s, 2H), 4.19 (s, 2H), 3.99 (d, 7H), 3.36 - 3.27 (m, 3H), 3.18 - 3.11 (m, 4H), 2.75 (t, 2H), 2.75 - 2.61 (m, 3H), 2.24 - 2.07 (m, 5H), 1.64 - 1.39 (m, 2H).

### Example 250: (R)-1-(4-(((6-(2-Chloro-3-(3-chloro-2-(4-((3-hydroxypiperidin-1-yl)methyl)-3-methoxyphenyl)pyridin-4-yl)phenyl)-2-methoxypyridin-3-yl)methyl)amino)piperidin-1-yl)ethan-1-one (489)

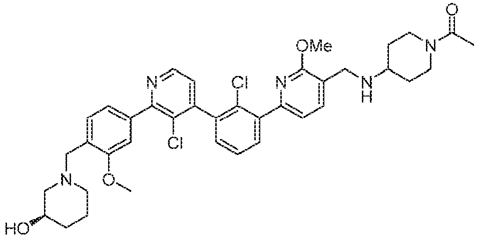

(R)-1-(4-(((6-(2-Chloro-3-(3-chloro-2-(4-((3-hydroxypiperidin-1-yl)methyl)-3-methoxyphenyl)pyridin-4-yl)phenyl)-2-methoxypyridin-3-yl)methyl)amino)piperidin-1-yl)ethan-1-one was prepared in a similar fashion to Example 247, replacing morpholine with (R)-piperidin-3-ol in step (e). MS: m/z found 704 [M+H]⁺. ¹H NMR (400 MHz, Methanol-*d*₄) δ 8.62 (d, 1H), 8.49 (s, 1H), 7.79 (d, 1H), 7.68 (dd, 1H), 7.58 - 7.45 (m, 2H), 7.46 - 7.36 (m, 2H), 7.36 - 7.24 (m, 3H), 4.53 (d, 1H), 4.02 (d, 7H), 3.92 (s, 4H), 3.86 (s, 1H), 3.20 - 2.98 (m, 6H), 2.68 (t, 3H), 2.09 (s, 4H), 1.97 (s, 1H), 1.83 (d, 1H), 1.68 (s, 1H), 1.54 - 1.25 (m, 2H).

### Example 251: (S)-1-(4-(((6-(2-Chloro-3-(3-chloro-2-(4-((3-hydroxypiperidin-1-yl)methyl)-3-methoxyphenyl)pyridin-4-yl)phenyl)-2-methoxypyridin-3-yl)methyl)amino)piperidin-1-yl)ethan-1-one (507)

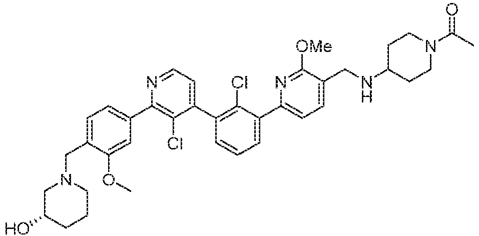

(S)-1-(4-(((6-(2-Chloro-3-(3-chloro-2-(4-((3-hydroxypiperidin-1-yl)methyl)-3-methoxyphenyl)pyridin-4-yl)phenyl)-2-methoxypyridin-3-yl)methyl)amino)piperidin-1-yl)ethan-1-one was prepared in a similar fashion to Example 247, replacing morpholine with (S)-piperidin-3-ol in step (e). MS: m/z found 704 [M+H]⁺. ¹H NMR (400 MHz, Methanol-*d*₄) δ 8.63 (d, 1H), 8.41 (s, 2H), 7.85 (d, 1H), 7.68 (dd, 1H), 7.58 - 7.48 (m, 2H), 7.47 - 7.27 (m, 5H), 4.61 (d, 1H), 4.29 (s, 2H), 4.21 (s, 2H), 4.07 - 3.92 (m, 7H), 3.41 - 3.33 (m, 1H), 3.27 (d, 3H), 3.16 (t, 4H), 2.96 (s, 1H), 2.67 (t, 1H), 2.19 (t, 3H), 2.09 (s, 3H), 1.78 - 1.67 (m, 1H), 1.66 - 1.52 (m, 1H), 1.54 - 1.41 (m, 1H).

### Example 252: 1-(4-(((6-(2-Chloro-3-(3-chloro-2-(4-((4-(hydroxymethyl)piperidin-1-yl)methyl)-3-methoxyphenyl)pyridin-4-yl)phenyl)-2-methoxypyridin-3-yl)methyl) amino)piperidin-1-yl)ethan-1-one (508)

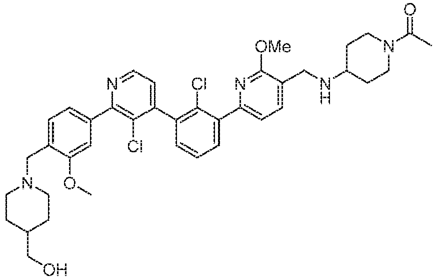

1-(4-(((6-(2-Chloro-3-(3-chloro-2-(4-((4-(hydroxymethyl)piperidin-1-yl)methyl)-3-methoxyphenyl)pyridin-4-yl)phenyl)-2-methoxypyridin-3-yl)methyl) amino)piperidin-1-yl)ethan-1-one was prepared in a similar fashion to Example 247, replacing morpholine with piperidin-4-ylmethanol in step (e). MS: m/z found 718 [M+H]⁺. ¹H NMR (400 MHz, Methanol-*d*₄) δ 8.63 (d, 1H), 8.44 (s, 2H), 7.84 (d, 1H), 7.68 (dd, 1H), 7.53 (t, 2H), 7.47 - 7.30 (m, 4H), 7.30 (d, 1H), 4.59 (d, 1H), 4.32 (s, 2H), 4.17 (s, 2H), 4.06 - 3.92 (m, 7H), 3.46 (dd, 4H), 3.22 - 3.10 (m, 1H), 3.02 (t, 2H), 2.67 (t, 1H), 2.09 (s, 5H), 1.94 (d, 2H), 1.65-1.80 (m, 1H), 1.63 - 1.37 (m, 4H).

### Example 253: 1-(4-(((6-(2-Chloro-3-(3-chloro-2-(3-methoxy-4-((4-(methoxymethyl) piperidin-1-yl)methyl)phenyl)pyridin-4-yl)phenyl)-2-methoxypyridin-3-yl)methyl) amino)piperidin-1-yl)ethan-1-one (509)

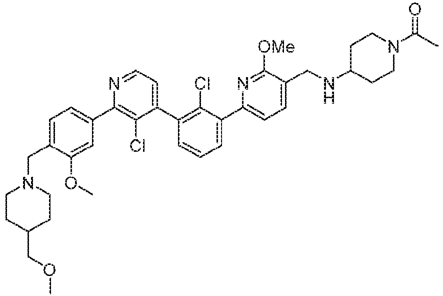

1-(4-(((6-(2-Chloro-3-(3-chloro-2-(3-methoxy-4-((4-(methoxymethyl) piperidin-1-yl)methyl)phenyl)pyridin-4-yl)phenyl)-2-methoxypyridin-3-yl)methyl) amino)piperidin-1-yl)ethan-1-one was prepared in a similar fashion to Example 247, replacing morpholine with 4-(methoxymethyl)piperidine in step (e). MS: m/z found 732 [M+H]⁺. ¹H NMR (400 MHz, Methanol-*d*₄) δ 8.63 (d, 1H), 8.48 (s, 1H), 7.81 (d, 1H), 7.68 (dd, 1H), 7.58 - 7.48 (m, 2H), 7.47 - 7.24 (m, 5H), 4.55 (d, 1H), 4.26 (s, 2H), 4.08 (s, 2H), 3.98 (dd, 7H), 3.44 (d, 2H), 3.31 - 3.28 (m, 4H), 3.14 (t, 2H), 2.96 (t, 2H), 2.67 (t, 1H), 2.18 - 2.06 (m, 5H), 1.89 (t, 3H), 1.56 - 1.33 (m, 4H).

### Example 254: 1-(4-((4-(3-Chloro-4-(2-chloro-3-(5-((4-hydroxypiperidin-1-yl)methyl)-6-methoxypyridin-2-yl)phenyl)pyridin-2-yl)-2-methoxybenzyl)amino)piperidin-1-yl)ethan-1-one (487)

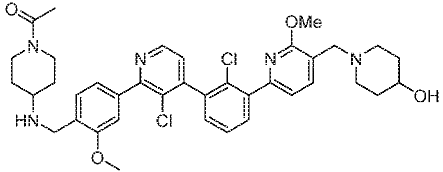

### (a) 1-(4-((4-Bromo-2-methoxybenzyl)amino)piperidin-1-yl)ethan-1-one

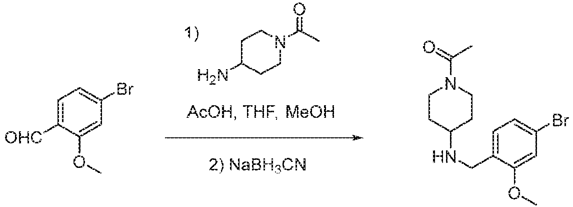

A scintillation vial was charged with 4-bromo-2-methoxybenzaldehyde (0.20 g, 0.93 mmol), 1-(4-aminopiperidin-1-yl)ethan-1-one (0.26 g, 1.86 mmol), followed by a 1:1 THF/MeOH solution (6 mL), acetic acid (0.11 g, 1.86 mmol) and 4 A molecular sieve (1 g). The mixture was stirred at room temperature for 3 hrs. Sodium cyanoborohydride (0.15 g, 2.33 mmol) was added, the mixture stirred at room temperature for 1 hr and then the reaction quenched by addition of water (1 ml). The mixture was diluted with ethyl acetate (50 ml), then washed with saturated aqueous brine solution (30 mL), then aqueous layer extracted with ethyl acetate (3 x 30 mL), and the combined organic phases dried over sodium sulfate, filtered and concentrated under reduced pressure. The residue was purified by normal phase flash SiO₂ chromatography using a linear gradient (0-10 % MeOH/CH₂Cl₂) to afford 1-(4-((4-bromo-2-methoxybenzyl)amino)piperidin-1-yl)ethan-1-one. MS: m/z found 341 [M+H]⁺.

### (b) tert-Butyl (1-acetylpiperidin-4-yl)(4-bromo-2-methoxybenzyl)carbamate

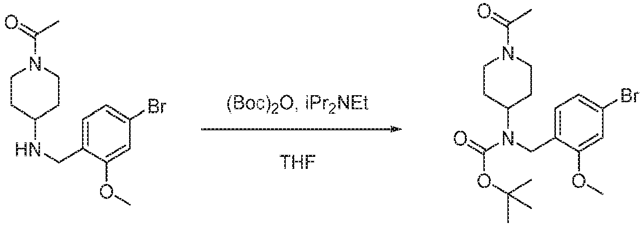

A round bottom flask was charged with 1-(4-((4-bromo-2-methoxybenzyl)amino)piperidin-1-yl)ethan-1-one (0.59 g, 1.73 mmol) and di-tert-butyl dicarbonate (0.75 g, 3.46 mmol). The flask was purged with nitrogen for 5 min, then dry THF (20 mL) was added by syringe, followed by N,N-diisopropylethylamine (1.2 ml, 6.92 mmol). The reaction was stirred at room temperature for 3 hrs. The mixture was diluted with ethyl acetate (50 ml), then washed with saturated aqueous brine solution (30 mL), and the aqueous layer extracted with ethyl acetate (3 x 30 mL), the combined organic phases were dried over sodium sulfate, filtered and concentrated under reduced pressure. The residue was purified by normal phase flash SiO₂ chromatography using a linear gradient (0-5 % MeOH/CH₂Cl₂) to afford tert-butyl (1-acetylpiperidin-4-yl)(4-bromo-2-methoxybenzyl)carbamate (0.68 g, 89% yield). MS: m/z found 441 [M+H]⁺.

### (c) tert-Butyl (1-acetylpiperidin-4-yl)(2-methoxy-4-(4,4,5,5-tetramethyl-1,3,2-dioxaborolan-2-yl)benzyl)carbamate

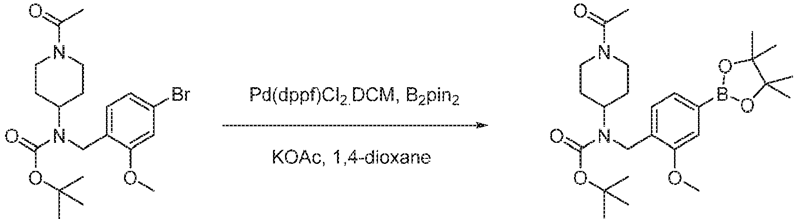

A microwave vial was charged with tert-butyl (1-acetylpiperidin-4-yl)(4-bromo-2-methoxybenzyl)carbamate (0.68 g, 1.54 mmol), bis(pinacolato)diborane (0.43 g, 1.69 mmol), potassium acetate (0.42 g, 4.31 mmol), and [1,1'- bis(diphenylphosphino)ferrocene] dichloropalladium(II) complex with dichloromethane (0.13 g, 0.15 mmol). The flask was purged with nitrogen for 5 min, then 5 ml of dry 1,4-dioxane was added, and the reaction was sparged with nitrogen for 5 min. The mixture was stirred at 90 °C for 1 hr then the mixture was filtered through CELITE^{®} and the filter cake was washed with ethyl acetate (3 x 40 mL). The filtrate was concentrated under reduced pressure and the residue purified by normal phase flash SiO₂ chromatography using a gradient (0-5 % methanol/CH₂Cl₂) to afford tert-butyl (1-acetylpiperidin-4-yl)(2-methoxy-4-(4,4,5,5-tetramethyl-1,3,2-dioxaborolan-2-yl)benzyl)carbamate (0.75 g, 100% yield). MS: m/z found 489 [M+H]⁺.

### (d) tert-Butyl (1-acetylpiperidin-4-yl)(4-(3-chloro-4-(2-chloro-3-(5-formyl-6-methoxypyridin-2-yl)phenyl)pyridin-2-yl)-2-methoxybenzyl)carbamate

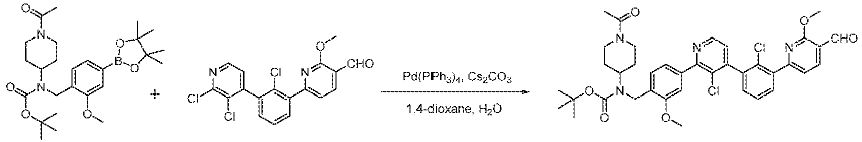

A microwave vial was charged with tert-butyl (1-acetylpiperidin-4-yl)(2-methoxy-4-(4,4,5,5-tetramethyl-1,3,2-dioxaborolan-2-yl)benzyl)carbamate (0.40 g, 0.82 mmol), 6-(2-chloro-3-(2,3-dichloropyridin-4-yl)phenyl)-2-methoxynicotinaldehyde (0.40 g, 0.74 mmol), cesium carbonate (0.72 g 2.22 mmol), and tetrakis(triphenylphosphine)palladium(0) (0.09 g, 0.07 mmol). 1,4-Dioxane (4 ml) was added, and the reaction was sparged with nitrogen, then water (1 ml) was added. The mixture was stirred at 90 °C for 30 min then the mixture was filtered through CELITE^{®} and the filter cake was washed with ethyl acetate (3 x 15 mL). The filtrate was concentrated under reduced pressure and the residue purified by normal phase flash SiO₂ chromatography using a gradient (0-10% methanol/CH₂Cl₂) to afford tert-butyl (1-acetylpiperidin-4-yl)(4-(3-chloro-4-(2-chloro-3-(5-formyl-6-methoxypyridin-2-yl)phenyl)pyridin-2-yl)-2-methoxybenzyl)carbamate (0.70 g, 95% yield). MS: m/z found 719 [M+H]⁺.

### (e) 1-(4-((4-(3-Chloro-4-(2-chloro-3-(5-((4-hydroxypiperidin-1-yl)methyl)-6-methoxypyridin-2-yl)phenyl)pyridin-2-yl)-2-methoxybenzyl)amino)piperidin-1-yl)ethan-1-one

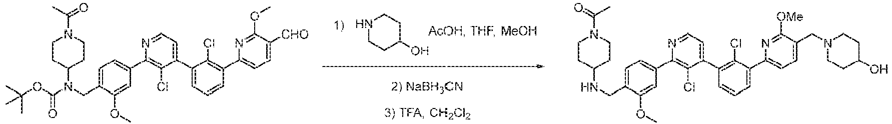

To a mixture of tert-butyl (1-acetylpiperidin-4-yl)(4-(3-chloro-4-(2-chloro-3-(5-formyl-6-methoxypyridin-2-yl)phenyl)pyridin-2-yl)-2-methoxybenzyl)carbamate (0.12 g, 0.17 mmol) and piperidin-4-ol (0.03 g, 0.33 mmol), in 1:1 THF/MeOH (6 mL) was added acetic acid (0.02 g, 0.3 mmol) and 4 A molecular sieve (1 g). The mixture was stirred at room temperature for 3 hrs. Sodium cyanoborohydride (0.03 g, 0.42 mmol) was then added and the mixture was stirred at room temperature for 1 hr. The reaction was then quenched by the addition of water (1 mL). The mixture was diluted with ethyl acetate (50 ml), then washed with saturated aqueous brine solution (30 mL), and the aqueous layer extracted with ethyl acetate (3 x 30 mL), the combined organic phases were dried over sodium sulfate, dried and concentrated under reduced pressure. The residue was purified by normal phase flash SiO₂ chromatography using a linear gradient (0-5 % MeOH/CH₂Cl₂) to afford tert-butyl (1-acetylpiperidin-4-yl)(4-(3-chloro-4-(2-chloro-3-(5-((4-hydroxypiperidin-1-yl)methyl)-6-methoxypyridin-2-yl)phenyl)pyridin-2-yl)-2-methoxybenzyl) carbamate. MS: m/z found 804 [M+H]⁺. The protected amine was then diluted with dichloromethane (3 ml), trifluoroacetic acid (1 ml) added and reaction mixture stirred for 20 min, then concentrated under reduced pressure, and the residue purified by reverse phase HPLC (with 0.05% Formic acid modifier) to give 1-(4-((4-(3-chloro-4-(2-chloro-3-(5-((4-hydroxypiperidin-1-yl)methyl)-6-methoxypyridin-2-yl)phenyl)pyridin-2-yl)-2-methoxybenzyl)amino)piperidin-1-yl)ethan-1-one 8.9 mg (34%) as a white solid (formic acid salt). MS: m/z found 804 [M+H]⁺. ¹H NMR (400 MHz, Methanol-*d*₄) δ 8.64 (d, 1H), 8.52 (s, 1H), 7.80 (d, 1H), 7.71 (dd, 1H), 7.59 - 7.37 (m, 4H), 7.38 - 7.24 (m, 3H), 4.60 (d, 1H), 4.18 (s, 2H), 4.06 - 3.94 (m, 7H), 3.79 - 3.66 (m, 3H), 3.28 - 3.11 (m, 2H), 3.04 - 2.96 (m, 2H), 2.68 (t, 1H), 2.52 (s, 2H), 2.18 (d, 2H), 2.11 (s, 3H), 1.91 (d, 2H), 1.67 - 1.63 (m, 3H), 1.67 - 1.51 (m, 1H), 1.52 - 1.38 (m, 1H).

### Example 255: 1-(4-((4-(3-Chloro-4-(2-chloro-3-(6-methoxy-5-((((1r,4r)-4-methoxy cyclohexyl)amino)methyl)pyridin-2-yl)phenyl)pyridin-2-yl)-2-methoxybenzyl) amino)piperidin-1-yl)ethan-1-one (488)

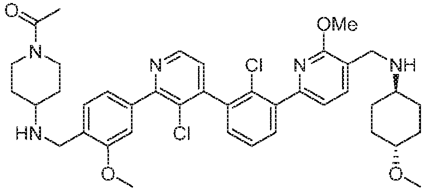

### 1-(4-((4-(3-Chloro-4-(2-chloro-3-(6-methoxy-5-((((1r,4r)-4-methoxy cyclohexyl)amino)methyl)pyridin-2-yl)phenyl)pyridin-2-yl)-2-methoxybenzyl) amino)piperidin-1-yl)ethan-1-one

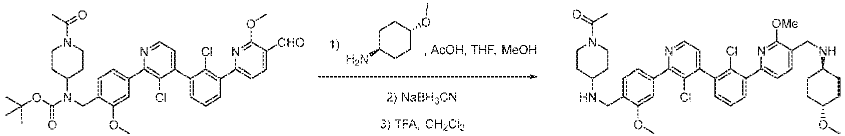

1-(4-((4-(3-Chloro-4-(2-chloro-3-(6-methoxy-5-((((1r,4r)-4-methoxy cyclohexyl)amino)methyl)pyridin-2-yl)phenyl)pyridin-2-yl)-2-methoxybenzyl) amino)piperidin-1-yl)ethan-1-one was prepared in a similar fashion to Example 254 replacing piperidin-4-ol with (1r,4r)-4-methoxycyclohexan-1-amine in step (e). MS: m/z found 732 [M+H]⁺. ¹H NMR (400 MHz, Methanol-*d*₄) δ 8.60 (d, 1H), 8.51 (s, 1H), 7.75 (d, 1H), 7.68 (dd, 1H), 7.53 (t, 1H), 7.45 - 7.35 (m, 3H), 7.27 (t, 3H), 4.49 (d, 1H), 4.02 - 3.86 (m, 11H), 3.23 - 3.06 (m, 2H), 2.92 - 2.87 (m, 1H), 2.65 (t, 2H), 2.15 - 1.97 (m, 10H), 1.28 (m, 5H).

### Example 256: 2-((6-(3-(2-(4-(((1-Acetylpiperidin-4-yl)amino)methyl)-3-methoxyphenyl)-3-chloropyridin-4-yl)-2-chlorophenyl)-2-methoxypyridin-3-yl)methyl)-2,6-diazaspiro[3.4] octan-5-one (525)

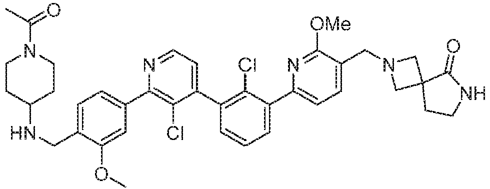

2-((6-(3-(2-(4-(((1-Acetylpiperidin-4-yl)amino)methyl)-3-methoxyphenyl)-3-chloropyridin-4-yl)-2-chlorophenyl)-2-methoxypyridin-3-yl)methyl)-2,6-diazaspiro[3.4] octan-5-one was prepared in a similar fashion to Example 254 replacing piperidin-4-ol with 2,6-diazaspiro[3.4]octan-5-one in step (e). MS: m/z found 729 [M+H]⁺. ¹H NMR (400 MHz, Methanol-*d*₄) δ 8.63 (s, 1H), 8.44 (s, 3H), 7.78 - 7.64 (m, 2H), 7.57 - 7.21 (m, 6H), 4.63 (d, 1H), 4.28 (s, 2H), 4.09 - 3.97 (m, 1H), 4.02 - 3.91 (m, 7H), 3.79 (d, 2H), 3.66 (d, 2H), 3.45 - 3.35 (m, 1H), 3.30 (d, 1H), 3.17 (t, 1H), 2.66 (t, 1H), 2.48 (t, 2H), 2.21 (t, 2H), 2.10 (s, 3H), 1.67 - 1.42 (m, 2H), 1.26 (s, 1H).

### Example 257: 2-((6-(3-(2-(4-(((1-Acetylpiperidin-4-yl)amino)methyl)-3-methoxyphenyl)-3-chloropyridin-4-yl)-2-chlorophenyl)-2-methoxypyridin-3-yl)methyl)-2,5,7-triazaspiro[3.4]octan-6-one (526)

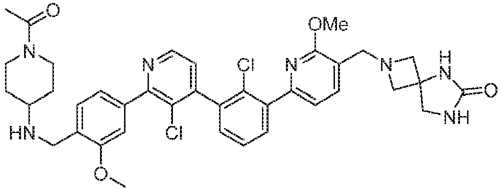

2-((6-(3-(2-(4-(((1-Acetylpiperidin-4-yl)amino)methyl)-3-methoxyphenyl)-3-chloropyridin-4-yl)-2-chlorophenyl)-2-methoxypyridin-3-yl)methyl)-2,5,7-triazaspiro[3.4]octan-6-one was prepared in a similar fashion to Example 254 replacing piperidin-4-ol with 2,5,7-triazaspiro[3.4]octan-6-one in step (e). MS: m/z found 730 [M+H]⁺. ¹H NMR (400 MHz, Methanol-*d*₄) δ 8.63 (d, 1H), 8.39 (s, 2H), 7.72 - 7.64 (m, 2H), 7.52 (t, 2H), 7.44 (d, 1H), 7.42 - 7.29 (m, 3H), 7.23 (d, 1H), 4.64 (d, 1H), 4.29 (s, 2H), 4.05 (d, 1H), 3.97 (d, 6H), 3.79 (s, 2H), 3.73 - 3.62 (m, 4H), 3.52 - 3.37 (m, 2H), 3.18 (t, 1H), 2.67 (t, 1H), 2.22 (t, 2H), 2.11 (s, 3H), 1.69 - 1.55 (m, 1H), 1.58 - 1.44 (m, 1H).

### Example 258: (R)-5-(((6-(3-(2-(4-(((1-Acetylpiperidin-4-yl)amino)methyl)-3-methoxyphenyl)-3-chloropyridin-4-yl)-2-chlorophenyl)-2-methoxypyridin-3-yl)methyl)amino)piperidin-2-one (541)

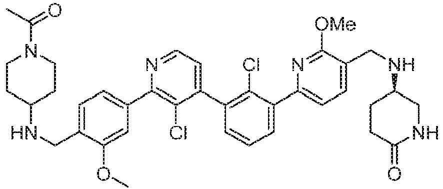

(R)-5-(((6-(3-(2-(4-(((1-Acetylpiperidin-4-yl)amino)methyl)-3-methoxyphenyl)-3-chloropyridin-4-yl)-2-chlorophenyl)-2-methoxypyridin-3-yl)methyl)amino)piperidin-2-one was prepared in a similar fashion to Example 254 replacing piperidin-4-ol with (R)-5-aminopiperidin-2-one in step (e). MS: m/z found 717 [M+H]⁺. ¹H NMR (400 MHz, Methanol-*d*₄) δ 8.61 (d, 1H), 8.49 (s, 2H), 7.74 (d, 1H), 7.67 (dd, 1H), 7.55 - 7.44 (m, 2H), 7.43 (d, 1H), 7.40 - 7.25 (m, 3H), 7.21 (d, 1H), 4.58 (d, 1H), 4.18 (s, 2H), 3.96 (d, 7H), 3.85 (q, 2H), 3.46 (dd, 1H), 3.20 - 3.05 (m, 2H), 3.05 - 2.95 (m, 1H), 2.65 (t, 1H), 2.49 - 2.36 (m, 1H), 2.37 - 2.23 (m, 1H), 2.18 - 2.02 (m, 7H), 1.83 - 1.71 (m, 1H), 1.58 - 1.41 (m, 2H).

### Example 259: (S)-5-(((6-(3-(2-(4-(((1-Acetylpiperidin-4-yl)amino)methyl)-3-methoxyphenyl)-3-chloropyridin-4-yl)-2-chlorophenyl)-2-methoxypyridin-3-yl)methyl)amino)piperidin-2-one (542)

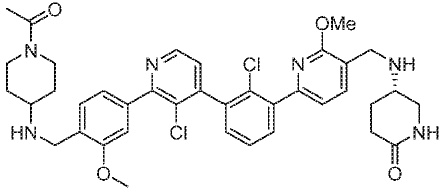

(S)-5-(((6-(3-(2-(4-(((1-Acetylpiperidin-4-yl)amino)methyl)-3-methoxyphenyl)-3-chloropyridin-4-yl)-2-chlorophenyl)-2-methoxypyridin-3-yl)methyl)amino)piperidin-2-one was prepared in a similar fashion to Example 254 replacing piperidin-4-ol with (S)-5-aminopiperidin-2-one in step (e). MS: m/z found 717 [M+H]⁺; ¹H NMR (400 MHz, Methanol-*d*₄) δ 8.63 (d, 1H), 7.89 (d, 1H), 7.68 (dd, 1H), 7.59 - 7.46 (m, 2H), 7.47 - 7.35 (m, 2H), 7.39 - 7.28 (m, 3H), 4.62 (s, 1H), 4.40 - 4.27 (m, 4H), 4.06 (s, 4H), 3.96 (s, 3H), 3.78 - 3.66 (m, 2H), 3.50 - 3.39 (m, 2H), 3.27 (d, 2H), 3.18 (t, 1H), 2.66 (t, 1H), 2.47 (t, 2H), 2.39 - 2.28 (m, 1H), 2.22 (t, 2H), 2.09 - 1.98 (m, 2H), 1.70 - 1.43 (m, 2H).

### Example 260: 1-(4-(((6-(2-Chloro-3-(3-chloro-2-(3-methoxy-4-(((1-(methylsulfonyl)piperidin-4-yl)amino)methyl)phenyl)pyridin-4-yl)phenyl)-2-methoxypyridin-3-yl)methyl)amino)piperidin-1-yl)ethan-1-one (383)

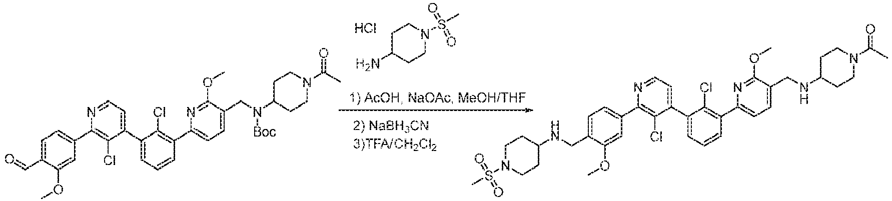

1-(4-(((6-(2-Chloro-3-(3-chloro-2-(3-methoxy-4-(((1-(methylsulfonyl)piperidin-4-yl)amino)methyl)phenyl)pyridin-4-yl)phenyl)-2-methoxypyridin-3-yl)methyl)amino)piperidin-1-yl)ethan-1-one was prepared as the formic acid salt, following Reductive Amination Procedure B from tert-butyl (1-acetylpiperidin-4-yl)((6-(2-chloro-3-(3-chloro-2-(4-formyl-3-methoxyphenyl)pyridin-4-yl)phenyl)-2-methoxypyridin-3-yl)methyl)carbamate (31 mg, 0.04 mmol) and 1-(methylsulfonyl)piperidin-4-amine HCl (23 mg, 0.11 mmol), followed by General Boc Deprotection Procedure. 27% yield. LCMS: m/z found 781.1 [M+H]⁺, retention time = 2.91 min (Method A). ¹H NMR (400 MHz, Methanol-*d*₄): δ 8.64 (d, 1H), 8.54 (s, 1H), 7.78 (d, 1H), 7.75 - 7.68 (m, 1H), 7.55 (t, 1H), 7.50 - 7.38 (m, 3H), 7.36 - 7.24 (m, 3H), 4.50 (d, 1H), 4.08 (s, 2H), 4.03 (s, 3H), 3.94 (d, 5H), 3.78 (d, 2H), 3.17 (d, 1H), 3.01 - 2.65 (m, 9H), 2.21 - 1.99 (m, 7H), 1.59 (dd, 2H), 1.49 - 1.23 (m, 2H).

### Example 261: 1-(4-((4-(4-(3-(5-(((1-Acetylpiperidin-4-yl)amino)methyl)-6-methoxypyridin-2-yl)-2-chlorophenyl)-3-chloropyridin-2-yl)-2-methoxybenzyl)amino)piperidin-1-yl)-2-methoxyethan-1-one (413)

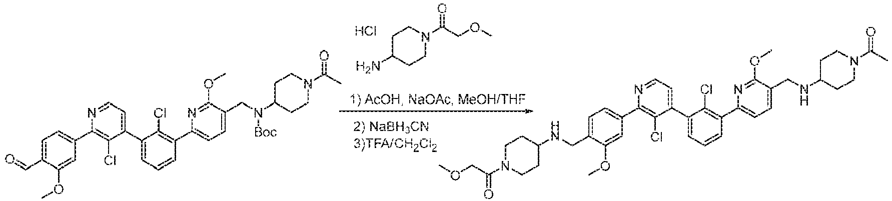

1-(4-((4-(4-(3-(5-(((1-Acetylpiperidin-4-yl)amino)methyl)-6-methoxypyridin-2-yl)-2-chlorophenyl)-3-chloropyridin-2-yl)-2-methoxybenzyl)amino)piperidin-1-yl)-2-methoxyethan-1-one was prepared as the formic acid salt, following Reductive Amination Procedure B from tert-butyl (1-acetylpiperidin-4-yl)((6-(2-chloro-3-(3-chloro-2-(4-formyl-3-methoxyphenyl)pyridin-4-yl)phenyl)-2-methoxypyridin-3-yl)methyl)carbamate (31 mg, 0.04 mmol) and 1-(4-aminopiperidin-1-yl)-2-methoxyethan-1-one HCl (22 mg, 0.11 mmol), followed by General Boc Deprotection Procedure. 50% yield. LCMS: m/z found 775.2 [M+H]⁺, retention time = 2.62 min, (Method A). ¹H NMR (400 MHz, Methanol-*d*₄): δ 8.65 (d, 1H), 8.53 (s, 1H), 7.80 (d, 1H), 7.71 (dd, 1H), 7.56 (t, 1H), 7.52 - 7.44 (m, 2H), 7.42 (dd, 1H), 7.36 (d, 1H), 7.36 - 7.25 (m, 2H), 4.65 - 4.49 (m, 2H), 4.26 - 4.08 (m, 5H), 4.04 (s, 3H), 3.98 (d, 5H), 3.40 (s, 3H), 3.27 - 3.05 (m, 4H), 2.98 (s, 1H), 2.72 (q, 2H), 2.11 (s, 8H), 1.69 - 1.25 (m, 4H).

### Example 262: 1-(4-(((6-(2-chloro-3-(3-chloro-2-(3-methoxy-4-((piperidin-4-ylamino)methyl)phenyl)pyridin-4-yl)phenyl)-2-methoxypyridin-3-yl)methyl)amino)piperidin-1-yl)ethan-1-one (417)

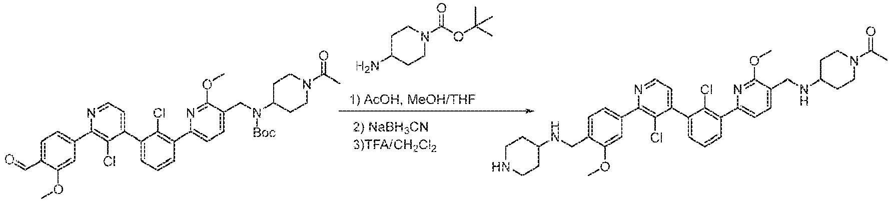

1-(4-(((6-(2-Chloro-3-(3-chloro-2-(3-methoxy-4-((piperidin-4-ylamino)methyl)phenyl)pyridin-4-yl)phenyl)-2-methoxypyridin-3-yl)methyl)amino)piperidin-1-yl)ethan-1-one was prepared as the formic acid salt, following Reductive Amination Procedure B from tert-butyl (1-acetylpiperidin-4-yl)((6-(2-chloro-3-(3-chloro-2-(4-formyl-3-methoxyphenyl)pyridin-4-yl)phenyl)-2-methoxypyridin-3-yl)methyl)carbamate (32 mg, 0.04 mmol) and tert-butyl 4-aminopiperidine-1-carboxylate (15 mg, 0.07 mmol), followed by General Boc Deprotection Procedure. 35% yield. LCMS: m/z found 703.3 [M+H]⁺, retention time = 1.97 min, (Method C). ¹H NMR (400 MHz, Methanol-*d*₄): δ 8.65 (d, 1H), 8.49 (s, 2H), 7.87 (d, 1H), 7.72 (dd, 1H), 7.57 (t, 1H), 7.50 - 7.40 (m, 3H), 7.36 - 7.27 (m, 3H), 4.60 (m, 1H), 4.18 (s, 2H), 4.06 (d, 6H), 3.95 (s, 3H), 3.48 - 3.40 (m, 2H), 3.31 - 3.14 (m, 2H), 3.12 - 2.94 (m, 3H), 2.77 - 2.65 (m, 1H), 2.22 (t, 4H), 2.13 (s, 3H), 1.78 - 1.64 (m, 2H), 1.68 - 1.51 (m, 1H), 1.50 - 1.42 (m, 1H).

### Example 263: 1-(4-(((6-(3-(2-(4-(((1-Acetylpiperidin-4-yl)amino)methyl)-3-methoxyphenyl)-3-chloropyridin-4-yl)-2-chlorophenyl)-2-methoxypyridin-3-yl)methyl)amino)piperidin-1-yl)-2-methoxyethan-1-one (481)

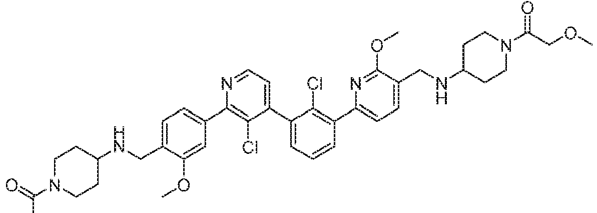

### (a) tert-Butyl N-[[6-[2-chloro-3-(2,3-dichloro-4-pyridyl)phenyl]-2-methoxy-3-pyridyl]methyl]-N-[1-(2-methoxyacetyl)-4-piperidyl]carbamate

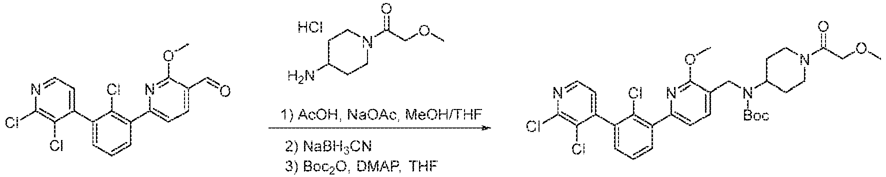

Acetic acid (36 µL, 0.64 mmol) was added to a mixture of sodium acetate (73 mg, 0.89 mmol), 1-(4-amino-1-piperidyl)-2-methoxy-ethanone hydrochloride (186 mg, 0.89 mmol) and 6-[2-chloro-3-(2,3-dichloro-4-pyridyl)phenyl]-2-methoxy-pyridine-3-carbaldehyde (250 mg, 0.64 mmol) in 1 mL THF and 1 mL MeOH. After stirring for 3 h at room temperature, the mixture was cooled in an ice bath and sodium cyanoborohydride (80 mg, 1.27 mmol) was added in 2 portions. After 3 h, the mixture was partitioned between EtOAc (20 mL) and water (15 mL). The layers were separated, and the aqueous layer was extracted with 2 x 10 mL EtOAc. The combined organics were washed with brine (20 mL), dried over sodium sulfate, and concentrated under reduced pressure to provide crude 1-[4-[[6-[2-chloro-3-(2,3-dichloro-4-pyridyl)phenyl]-2-methoxy-3-pyridyl]methylamino]-1-piperidyl]-2-methoxy-ethanone (346 mg). MS: m/z found 549.0 [M+H]⁺.

Crude 1-[4-[[6-[2-chloro-3-(2,3-dichloro-4-pyridyl)phenyl]-2-methoxy-3-pyridyl]methylamino]-1-piperidyl]-2-methoxy-ethanone (346 mg, 0.63 mmol), 4-dimethylaminopyridine (15 mg, 0.13 mmol) and N,N-diisopropylethylamine (219 µL, 1.26 mmol) were dissolved in 8 ml THF. Di-tert-butyl dicarbonate (192 mg, 0.88 mmol) was added portion-wise. The resulting mixture was stirred at room temperature for 16 hours. The reaction mixture was diluted with water (7 mL) and the mixture was extracted with EtOAc (3 x 5 mL). The combined organics were concentrated under reduced pressure and crude sample was purified by normal phase SiO₂ chromatography (0-100% EtOAc/hexane) to afford tert-butyl N-[[6-[2-chloro-3-(2,3-dichloro-4-pyridyl)phenyl]-2-methoxy-3-pyridyl]methyl]-N-[1-(2-methoxyacetyl)-4-piperidyl]carbamate (250 mg, 61% yield) as a white foam. MS: m/z found 649.1 [M+H]⁺.

### (b) tert-Butyl N-[[6-[2-chloro-3-[3-chloro-2-(4-formyl-3-methoxy-phenyl)-4-pyridyl]phenyl]-2-methoxy-3-pyridyl]methyl]-N-[1-(2-methoxyacetyl)-4-piperidyl]carbamate

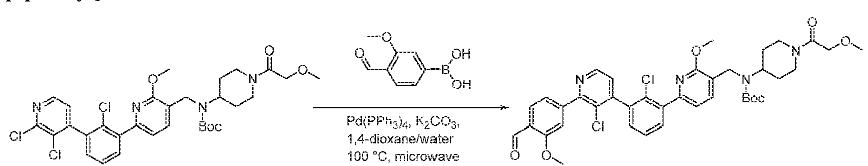

Tetrakis(triphenylphosphine)palladium(0) (26 mg, 0.02 mmol), potassium carbonate (116 mg, 0.84 mmol), tert-butyl N-[[6-[2-chloro-3-(2,3-dichloro-4-pyridyl)phenyl]-2-methoxy-3-pyridyl]methyl]-N-[1-(2-methoxyacetyl)-4-piperidyl]carbamate (182 mg, 0.28 mmol), and (4-formyl-3-methoxy-phenyl)boronic acid (55 mg, 0.31 mmol) were suspended in 1,4-dioxane/water (4:1, 2 mL). The solution was irradiated to 100 °C for 30 minutes in a Biotage Initiator microwave. The reaction mixture was cooled to room temperature, diluted with 10 mL water, and extracted with EtOAc (3 x 5 mL). The combined organics were concentrated under reduced pressure and the crude sample was purified by normal phase silica gel chromatography (0-8% MeOH/DCM) to afford tert-butyl N-[[6-[2-chloro-3-[3-chloro-2-(4-formyl-3-methoxy-phenyl)-4-pyridyl]phenyl]-2-methoxy-3-pyridyl]methyl]-N-[1-(2-methoxyacetyl)-4-piperidyl]carbamate (168 mg, 80% yield) as a white foam . MS: m/z found 749.2 M+H]⁺.

### (c) 1-(4-(((6-(3-(2-(4-(((1-Acetylpiperidin-4-yl)amino)methyl)-3-methoxyphenyl)-3-chloropyridin-4-yl)-2-chlorophenyl)-2-methoxypyridin-3-yl)methyl)amino)piperidin-1-yl)-2-methoxyethan-1-one

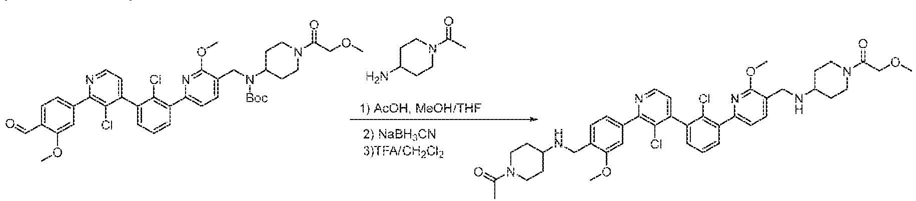

1-(4-(((6-(3-(2-(4-(((1-Acetylpiperidin-4-yl)amino)methyl)-3-methoxyphenyl)-3-chloropyridin-4-yl)-2-chlorophenyl)-2-methoxypyridin-3-yl)methyl)amino)piperidin-1-yl)-2-methoxyethan-1-one was prepared as the formic acid salt, following Reductive Amination Procedure B from tert-butyl ((6-(2-chloro-3-(3-chloro-2-(4-formyl-3-methoxyphenyl)pyridin-4-yl)phenyl)-2-methoxypyridin-3-yl)methyl)(1-(2-methoxyacetyl)piperidin-4-yl)carbamate (32 mg, 0.04 mmol) and 1-(4-aminopiperidin-1-yl)ethan-1-one (13 mg, 0.09 mmol), followed by General Boc Deprotection Procedure. 35% yield. LCMS: m/z found 775.2 [M+H]⁺, retention time = 2.78 min, (Method C). ¹H NMR (400 MHz, Methanol-*d*₄): δ 8.64 (dd, 1H), 8.54 (s, 1H), 7.77 (d, 1H), 7.71 (dd, 1H), 7.55 (t, 1H), 7.50 - 7.42 (m, 2H), 7.41 (dd, 1H), 7.36 - 7.27 (m, 2H), 7.27 (d, 1H), 4.62 (s, 1H), 4.56 (d, 1H), 4.48 (d, 1H), 4.20 (d, 1H), 4.12 (d, 1H), 4.08 (s, 2H), 4.03 (s, 2H), 4.03 (d, 1H), 3.96 (s, 3H), 3.91 (s, 3H), 3.40 (s, 2H), 3.40 (d, 1H), 3.34 - 3.28 (m, 1H), 3.21 - 3.07 (m, 2H), 3.06 (s, 1H), 2.93 - 2.83 (m, 1H), 2.81 - 2.64 (m, 2H), 2.12 (s, 3H), 2.05 (d, 3H), 1.55 - 1.27 (m, 4H).

### Example 264: 2-(4-(3-Chloro-4-(2-chloro-3-(6-methoxy-5-(((1-(2-methoxyacetyl)piperidin-4-yl)amino)methyl)pyridin-2-yl)phenyl)pyridin-2-yl)-2-methoxybenzyl)-2,6-diazaspiro[3.4]octan-7-one (490)

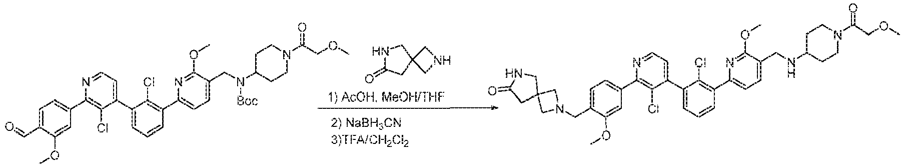

2-(4-(3-Chloro-4-(2-chloro-3-(6-methoxy-5-(((1-(2-methoxyacetyl)piperidin-4-yl)amino)methyl)pyridin-2-yl)phenyl)pyridin-2-yl)-2-methoxybenzyl)-2,6-diazaspiro[3.4]octan-7-onewas prepared as the formic acid salt, following Reductive Amination Procedure B from tert-butyl ((6-(2-chloro-3-(3-chloro-2-(4-formyl-3-methoxyphenyl)pyridin-4-yl)phenyl)-2-methoxypyridin-3-yl)methyl)(1-(2-methoxyacetyl)piperidin-4-yl)carbamate (30 mg, 0.04 mmol) and 2,6-diazaspiro[3.4]octan-7-one (11 mg, 0.09 mmol), followed by General Boc Deprotection Procedure. 43% yield. LCMS: m/z found 759.0 [M+H]⁺, retention time = 2.66 min, (Method A). ¹H NMR (400 MHz, Methanol-*d*₄): δ 8.62 (d, 1H), 7.78 (d, 1H), 7.71 (dd, 1H), 7.55 (t, 1H), 7.46 - 7.36 (m, 3H), 7.28 (dd, 3H), 4.49 (d, 1H), 4.20 (d, 1H), 4.12 (d, 1H), 4.03 (s, 3H), 3.93 (d, 5H), 3.84 (s, 2H), 3.58 (s, 2H), 3.51 (s, 4H), 3.40 (s, 3H), 3.32 (1H, obscured by CD₃OD), 3.09 (t, 1H), 2.92 (s, 1H), 2.75 (t, 1H), 2.58 (s, 2H), 2.07 (d, 2H), 1.47 - 1.37 (m, 1H), 1.39 - 1.27 (m, 1H).

### Example 265: 1-(4-(((6-(3-(2-(4-((6-Acetyl-2,6-diazaspiro[3.3]heptan-2-yl)methyl)-3-methoxyphenyl)-3-chloropyridin-4-yl)-2-chlorophenyl)-2-methoxypyridin-3-yl)methyl)amino)piperidin-1-yl)-2-methoxyethan-1-one (491)

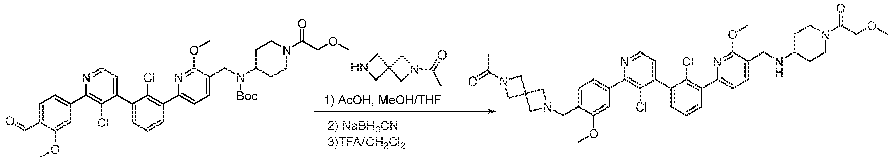

1-(4-(((6-(3-(2-(4-((6-Acetyl-2,6-diazaspiro[3.3]heptan-2-yl)methyl)-3-methoxyphenyl)-3-chloropyridin-4-yl)-2-chlorophenyl)-2-methoxypyridin-3-was prepared as the formic acid salt, following Reductive Amination Procedure B from tert-butyl ((6-(2-chloro-3-(3-chloro-2-(4-formyl-3-methoxyphenyl)pyridin-4-yl)phenyl)-2-methoxypyridin-3-yl)methyl)(1-(2-methoxyacetyl)piperidin-4-yl)carbamate (30 mg, 0.04 mmol) and 1-(2,6-diazaspiro[3.3]heptan-2-yl)ethanone (14 mg, 0.10 mmol), followed by General Boc Deprotection Procedure. 22% yield. LCMS: m/z found 773.2 [M+H]⁺, retention time = 2.74 min, (Method A). ¹H NMR (400 MHz, Methanol-*d*₄): δ 8.64 (d, 1H), 7.83 (d, 1H), 7.71 (dd, 1H), 7.56 (t, 1H), 7.48 - 7.39 (m, 3H), 7.34 - 7.26 (m, 3H), 4.55 (d, 1H), 4.33 (s, 2H), 4.22 (d, 1H), 4.17 - 3.96 (m, 11H), 3.94 (s, 3H), 3.84 (s, 4H), 3.40 (s, 3H), 3.12 (t, 2H), 2.75 (t, 1H), 2.14 (d, 2H), 1.84 (s, 3H), 1.57 - 1.38 (m, 2H).

### Example 266: 1-(4-(((6-(2-Chloro-3-(3-chloro-2-(4-((4-hydroxypiperidin-1-yl)methyl)-3-methoxyphenyl)pyridin-4-yl)phenyl)-2-methoxypyridin-3-yl)methyl)amino)piperidin-1-yl)-2-methoxyethan-1-one (510)

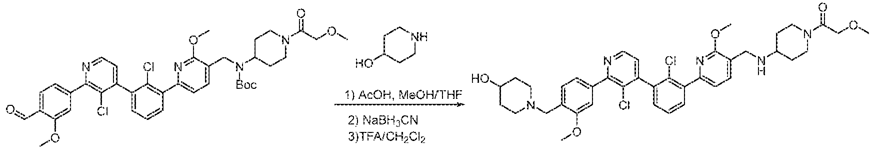

1-(4-(((6-(2-Chloro-3-(3-chloro-2-(4-((4-hydroxypiperidin-1-yl)methyl)-3-methoxyphenyl)pyridin-4-yl)phenyl)-2-methoxypyridin-3-yl)methyl)amino)piperidin-1-yl)-2-methoxyethan-1-one was prepared as the formic acid salt, following Reductive Amination Procedure B from tert-butyl ((6-(2-chloro-3-(3-chloro-2-(4-formyl-3-methoxyphenyl)pyridin-4-yl)phenyl)-2-methoxypyridin-3-yl)methyl)(1-(2-methoxyacetyl)piperidin-4-yl)carbamate (31 mg, 0.04 mmol) and piperidin-4-ol (9 mg, 0.09 mmol), followed by General Boc Deprotection Procedure. 33% yield. LCMS: m/z found 734.3 [M+H]⁺, retention time = 2.74 min, (Method A). ¹H NMR (400 MHz, Methanol-*d*₄): δ 8.65 (d, 1H), 8.56 (s, 1H), 7.79 (d, 1H), 7.71 (dd, 1H), 7.60 - 7.38 (m, 4H), 7.37 - 7.24 (m, 3H), 4.49 (d, 1H), 4.20 (d, 1H), 4.12 (d, 1H), 4.03 (s, 3H), 4.03 (s, 1H), 3.94 (s, 4H), 3.80 (s, 1H), 3.40 (s, 3H), 3.17 (s, 2H), 3.09 (t, 1H), 2.93 (s, 1H), 2.80 - 2.70 (m, 3H), 2.07 (d, 2H), 2.00 - 1.92 (m, 2H), 1.72 (s, 2H), 1.47 - 1.29 (m, 4H).

### Example 267: 1-(4-(((6-(3-(2-(4-(((1-Acetylpiperidin-4-yl)amino)methyl)-3-methoxyphenyl)-3-chloropyridin-4-yl)-2-chlorophenyl)-2-methoxypyridin-3-yl)methyl)amino)piperidin-1-yl)-2-hydroxyethan-1-one (533)

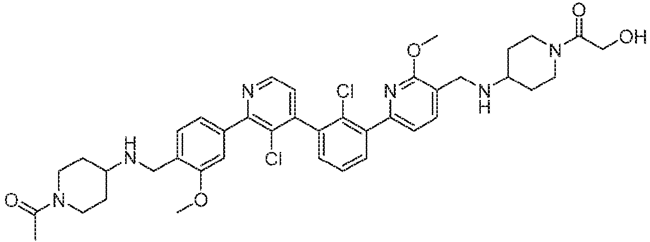

### (a) tert-Butyl (1-(2-((tert-butoxycarbonyl)oxy)acetyl)piperidin-4-yl)((6-(2-chloro-3-(2,3-dichloropyridin-4-yl)phenyl)-2-methoxypyridin-3-yl)methyl)carbamate

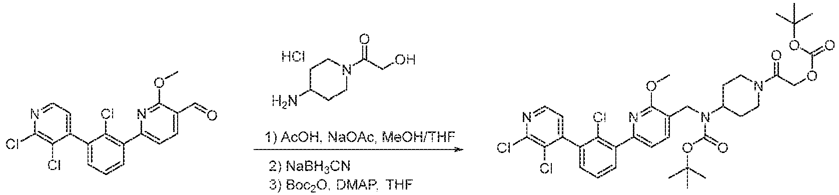

Acetic acid (29 µL, 0.51 mmol) was added to a mixture of sodium acetate (58 mg, 0.71 mmol), 1-(4-aminopiperidin-1-yl)-2-hydroxyethan-1-one (138 mg, 0.71 mmol) and 6-[2-chloro-3-(2,3-dichloro-4-pyridyl)phenyl]-2-methoxy-pyridine-3-carbaldehyde (200 mg, 0.51 mmol) in 1 mL THF and 1 mL MeOH. After stirring overnight at room temperature, the mixture was cooled in an ice bath and sodium cyanoborohydride (64 mg, 1.02 mmol) was added in 2 portions. After 3h, the mixture was partitioned between EtOAc (20 mL) and water (15 mL). The layers were separated, and the aqueous layer was extracted with 2 x 10 mL EtOAc. The combined organics were washed with brine (20 mL), dried over sodium sulfate, and concentrated under reduced pressure. The crude material was triturated with diethyl ether (3 x 20 mL), followed by normal phase silica gel chromatography (1% to 10% MeOH/DCM) to provide 1-[4-[[6-[2-chloro-3-(2,3-dichloro-4-pyridyl)phenyl]-2-methoxy-3-pyridyl]methylamino]-1-piperidyl]-2-hydroxy-ethanone (85 mg, 31%). MS: m/z found 537.1 [M+H]⁺.

1-[4-[[6-[2-Chloro-3-(2,3-dichloro-4-pyridyl)phenyl]-2-methoxy-3-pyridyl]methylamino]-1-piperidyl]-2-hydroxy-ethanone (80 mg, 0.15 mmol), 4-dimethylaminopyridine (4 mg, 0.03 mmol), and N,N-diisopropylethylamine (52 µL, 0.30 mmol) were dissolved in 2 ml THF and di-tert-butyl dicarbonate (46 mg, 0.21 mmol) was added portion-wise. The resulting mixture was stirred at room temperature for 72 hours. The reaction mixture was diluted with water (7 mL) and the mixture was extracted with EtOAc (3 x 5 mL). The combined organics were concentrated under reduced pressure and crude sample was purified by normal phase SiO₂ chromatography (0-100% EtOAc/hexane) to afford tert-butyl (1-(2-((tert-butoxycarbonyl)oxy)acetyl)piperidin-4-yl)((6-(2-chloro-3-(2,3-dichloropyridin-4-yl)phenyl)-2-methoxypyridin-3-yl)methyl)carbamate (90 mg, 82% yield). MS: m/z found 737.2 [M+H]⁺.

### (b) tert-Butyl (1-(2-((tert-butoxycarbonyl)oxy)acetyl)piperidin-4-yl)((6-(2-chloro-3-(3-chloro-2-(4-formyl-3-methoxyphenyl)pyridin-4-yl)phenyl)-2-methoxypyridin-3-yl)methyl)carbamate

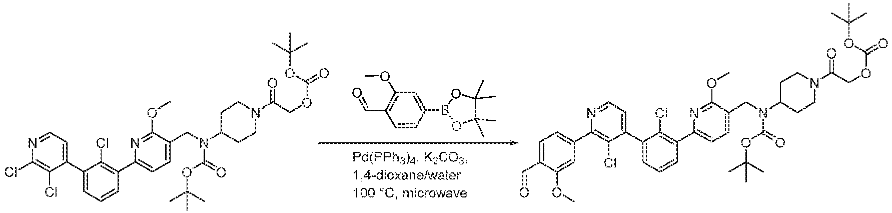

Tetrakis(triphenylphosphine)palladium(0) (7 mg, 0.01 mmol), potassium carbonate (34 mg, 0.24 mmol), tert-butyl N-[[6-[2-chloro-3-(2,3-dichloro-4-pyridyl)phenyl]-2-methoxy-3-pyridyl]methyl]-N-[1-(2-methoxyacetyl)-4-piperidyl]carbamate (60 mg, 0.08 mmol), and 2-methoxy-4-(4,4,5,5-tetramethyl-1,3,2-dioxaborolan-2-yl)benzaldehyde (24 mg, 0.09 mmol) were suspended in 1,4-dioxane/water (4:1, 1 mL). The solution was irradiated to 100 °C for 30 minutes in a Biotage Initiator microwave. The reaction mixture was diluted with 5 mL water, and extracted with EtOAc (3 x 5 mL). The combined organics were concentrated under reduced pressure and the crude sample was purified by normal phase silica gel chromatography (20% to 98% EtOAc/hexanes) to afford tert-butyl (1-(2-((tert-butoxycarbonyl)oxy)acetyl)piperidin-4-yl)((6-(2-chloro-3-(3-chloro-2-(4-formyl-3-methoxyphenyl)pyridin-4-yl)phenyl)-2-methoxypyridin-3-yl)methyl)carbamate (56 mg, 82% yield). MS: m/z found 835.1 [M+H]⁺.

### (c) 1-(4-(((6-(3-(2-(4-(((1-Acetylpiperidin-4-yl)amino)methyl)-3-methoxyphenyl)-3-chloropyridin-4-yl)-2-chlorophenyl)-2-methoxypyridin-3-yl)methyl)amino)piperidin-1-yl)-2-hydroxyethan-1-one

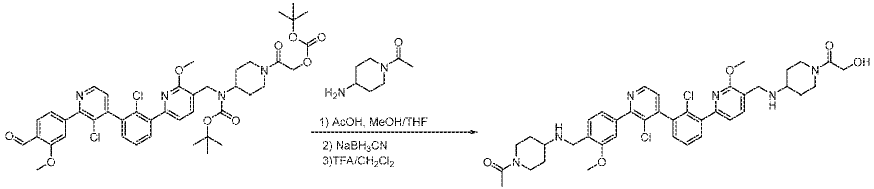

1-(4-(((6-(3-(2-(4-(((1-Acetylpiperidin-4-yl)amino)methyl)-3-methoxyphenyl)-3-chloropyridin-4-yl)-2-chlorophenyl)-2-methoxypyridin-3-yl)methyl)amino)piperidin-1-yl)-2-hydroxyethan-1-one was prepared as the formic acid salt, following Reductive Amination Procedure B from tert-butyl (1-(2-((tert-butoxycarbonyl)oxy)acetyl)piperidin-4-yl)((6-(2-chloro-3-(3-chloro-2-(4-formyl-3-methoxyphenyl)pyridin-4-yl)phenyl)-2-methoxypyridin-3-yl)methyl)carbamate (27 mg, 0.03 mmol) and 1-(4-aminopiperidin-1-yl)ethan-1-one (11 mg, 0.08 mmol), followed by General Boc Deprotection Procedure. 52% yield. LCMS: m/z found 761.4 [M+H]⁺, retention time = 2.69 min, (Method C). ¹H NMR (400 MHz, Methanol-*d*₄): δ 8.64 (d, 1H), 8.54 (s, 1H), 7.77 (d, 1H), 7.71 (dd, 1H), 7.55 (t, 1H), 7.50 - 7.42 (m, 2H), 7.41 (dd, 1H), 7.36 - 7.27 (m, 2H), 7.26 (d, 1H), 4.56 (d, 1H), 4.48 (d, 1H), 4.23 (d, 2H), 4.08 (s, 2H), 4.03 (s, 3H), 3.96 (s, 3H), 3.91 (s, 2H), 3.78 (d, 1H), 3.17 (d, 1H), 3.06 (t, 2H), 2.93 - 2.63 (m, 3H), 2.15 - 2.05 (m, 8H), 1.56 - 1.27 (m, 4H).

### Example 268: 1-(4-(((6-(2-Chloro-3-(3-chloro-2-(4-((((1r,4r)-4-hydroxycyclohexyl)amino)methyl)-3-methoxyphenyl)pyridin-4-yl)phenyl)-2-methoxypyridin-3-yl)methyl)amino)piperidin-1-yl)-2-hydroxyethan-1-one (539)

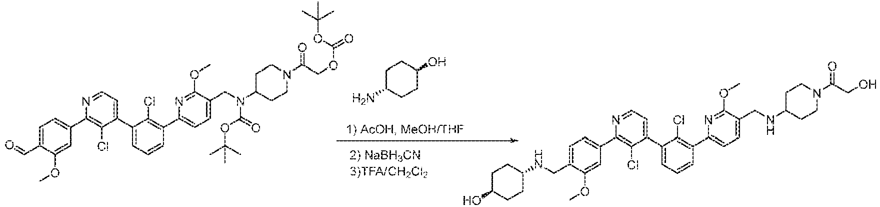

1-(4-(((6-(2-Chloro-3-(3-chloro-2-(4-((((1r,4r)-4-hydroxycyclohexyl)amino)methyl)-3-methoxyphenyl)pyridin-4-yl)phenyl)-2-methoxypyridin-3-yl)methyl)amino)piperidin-1-yl)-2-hydroxyethan-1-one was prepared as the formic acid salt, following Reductive Amination Procedure B from tert-butyl(1-(2-((tert-butoxycarbonyl)oxy)acetyl)piperidin-4-yl)((6-(2-chloro-3-(3-chloro-2-(4-formyl-3-methoxyphenyl)pyridin-4-yl)phenyl)-2-methoxypyridin-3-yl)methyl)carbamate (28 mg, 0.03 mmol) and trans-4-aminocyclohexanol (10 mg, 0.03 mmol), followed by General Boc Deprotection Procedure. 37% yield. LCMS: m/z found 734.4 [M+H]⁺, retention time = 2.69 min, (Method A). ¹H NMR (400 MHz, Methanol-*d*₄): δ 8.66 (d, 1H), 8.52 (s, 1H), 7.81 (d, 1H), 7.72 (dd, 1H), 7.60 - 7.26 (m, 7H), 4.63 (s, 1H), 4.52 (d, 1H), 4.26 (d, 3H), 4.23 (s, 1H), 4.06 - 3.97 (m, 8H), 3.82 (d, 1H), 3.63-3.53 (m, 1H), 3.13 - 3.02 (m, 3H), 2.78 (t, 1H), 2.23 (d, 2H), 2.09 (t, 4H), 1.60 - 1.27 (m, 5H).

### Example 269: 1-(4-(((6-(2-Chloro-3-(3-chloro-2-(3-methoxy-4-(((1-(3-methoxypropanoyl)piperidin-4-yl)amino)methyl)phenyl)pyridin-4-yl)phenyl)-2-methoxypyridin-3-yl)methyl)amino)piperidin-1-yl)-3-methoxypropan-1-one (546)

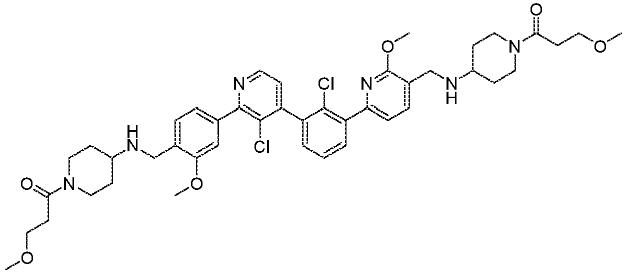

### (a) 1-(4-Aminopiperidin-1-yl)-3-methoxypropan-1-one hydrochloride

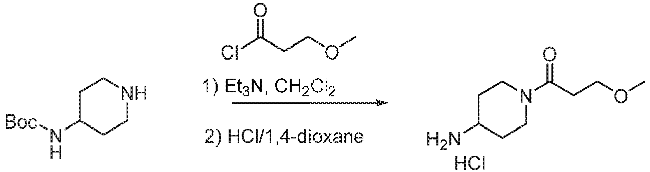

A solution of tert-butyl N-(4-piperidyl)carbamate (0.80 g, 3.99 mmol) in 32 mL DCM was cooled in an ice bath. Triethylamine (1.11 mL, 7.99 mmol) was added. A solution of 2-methoxyacetyl chloride (0.45 mL, 4.39 mmol) in 8 mL DCM was added slowly. After 30 minutes, the reaction mixture was diluted with 30 mL DCM and washed with saturated sodium bicarbonate solution (10 mL), 0.3M HCl (10 mL), and saturated sodium bicarbonate solution (10 mL). The organics were dried over sodium sulfate and concentrated under reduced pressure to provide crude tert-butyl N-[1-(3-methoxypropanoyl)-4-piperidyl]carbamate (1.04 g, 91% yield) as a white solid. MS: m/z found 287.2 [M+H]⁺. Hydrogen chloride, 4M in 1,4-dioxane (5.06 mL, 20.25 mmol) was added to tert-butyl N-[1-(3-methoxypropanoyl)-4-piperidyl]carbamate (0.58 g, 2.03 mmol). After stirring at room temperature for 2 h, the volatiles were removed *in vacuo.* The crude material was triturated with 3 x 10 mL anhydrous diethyl ether, and dried under vacuum to provide 1-(4-aminopiperidin-1-yl)-3-methoxypropan-1-one hydrochloride (0.45 g) MS: m/z found 187.1 [M+H]⁺.

### (b) 1-(4-(((6-(2-Chloro-3-(3-chloro-2-(3-methoxy-4-(((1-(3-methoxypropanoyl)piperidin-4-yl)amino)methyl)phenyl)pyridin-4-yl)phenyl)-2-methoxypyridin-3-yl)methyl)amino)piperidin-1-yl)-3-methoxypropan-1-one

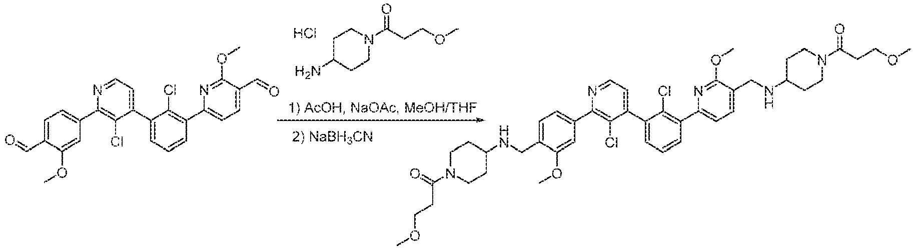

1-(4-(((6-(2-Chloro-3-(3-chloro-2-(3-methoxy-4-(((1-(3-methoxypropanoyl)piperidin-4-yl)amino)methyl)phenyl)pyridin-4-yl)phenyl)-2-methoxypyridin-3-yl)methyl)amino)piperidin-1-yl)-3-methoxypropan-1-one was prepared as the formic acid salt, following Reductive Amination Procedure A from 6-(2-chloro-3-(3-chloro-2-(4-formyl-3-methoxyphenyl)pyridin-4-yl)phenyl)-2-methoxynicotinaldehyde (30 mg, 0.06 mmol) and 1-(4-aminopiperidin-1-yl)-3-methoxypropan-1-one hydrochloride (44 mg, 0.21 mmol). 44% yield. LCMS: m/z found 833.3 [M+H]⁺, retention time = 2.93 min (Method A). ¹H NMR (400 MHz, Methanol-*d*₄): δ 8.65 (d, 1H), 8.53 (s, 1H), 7.81 (d, 1H), 7.72 (dd, 1H), 7.60 - 7.39 (m, 4H), 7.39 - 7.26 (m, 3H), 4.72 - 4.48 (m, 3H), 4.21 (s, 2H), 4.04 (m, 6H), 4.00 (d, 5H), 3.68 - 3.64 (m, 4H), 3.34 (dd, 6H), 3.26 (d, 1H), 3.21 - 3.10 (m, 2H), 3.03 (s, 1H), 2.80 - 2.56 (m, 5H), 2.25 - 1.98 (m, 4H), 1.67 - 1.26 (m, 3H).

### Example 270: 1-(4-(((6-(3-(2-(4-(((1-Acetylpiperidin-4-yl)amino)methyl)-3-methoxyphenyl)-3-chloropyridin-4-yl)-2-chlorophenyl)-2-methoxypyridin-3-yl)methyl)amino)piperidin-1-yl)-3-methoxypropan-1-one (607)

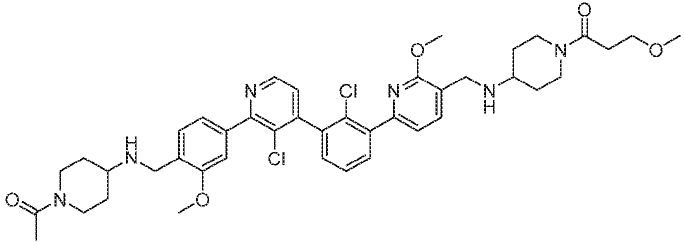

### (a) tert-Butyl N-[[6-[2-chloro-3-(2,3-dichloro-4-pyridyl)phenyl]-2-methoxy-3-pyridyl]methyl]-N-[1-(3-methoxypropanoyl)-4-piperidyl]carbamate

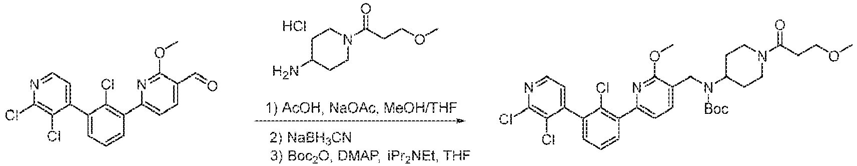

Acetic acid (32 µL, 0.55 mmol) was added to a mixture of sodium acetate (64 mg, 0.78 mmol), 1-(4-amino-1-piperidyl)-3-methoxy-propan-1-one hydrochloride (173 mg, 0.78 mmol) and 6-[2-chloro-3-(2,3-dichloro-4-pyridyl)phenyl]-2-methoxy-pyridine-3-carbaldehyde (218 mg, 0.55 mmol) in 1 mL THF and 1 mL MeOH. After stirring overnight at room temperature, the mixture was cooled in an ice bath and sodium cyanoborohydride (70 mg, 1.11 mmol) was added in 2 portions. After 3 h at room temperature, the mixture was partitioned between EtOAc (20 mL) and water (15 mL). The layers were separated, and the aqueous layer was extracted with 2 x 10 mL EtOAc. The combined organics were washed with brine (20 mL), dried over sodium sulfate, and concentrated under reduced pressure to provide crude 1-[4-[[6-[2-chloro-3-(2,3-dichloro-4-pyridyl)phenyl]-2-methoxy-3-pyridyl]methylamino]-1-piperidyl]-3-methoxy-propan-1-one (330 mg). MS: found 563.2 M+H]⁺.

Di-tert-butyl dicarbonate (0.19 g, 0.88 mmol) was added portion-wise to a mixture of 1-[4-[[6-[2-chloro-3-(2,3-dichloro-4-pyridyl)phenyl]-2-methoxy-3-pyridyl]methylamino]-1-piperidyl]-3-methoxy-propan-1-one (0.33 g, 0.59 mmol), 4-dimethylaminopyridine (14.30 mg, 0.12 mmol), and N,N-diisopropylethylamine (0.20 mL, 1.17 mmol) in 6 mL THF. After stirring at room temperature overnight, the mixture was dissolved in 20 mL EtOAc, and washed with water (15 mL) and brine (15 mL). The organics were dried over sodium sulfate and concentrated under reduced pressure. The crude sample was purified by normal phase silica gel chromatography (15% to 100% EtOAc/hexanes) to afford tert-butyl N-[[6-[2-chloro-3-(2,3-dichloro-4-pyridyl)phenyl]-2-methoxy-3-pyridyl]methyl]-N-[1-(3-methoxypropanoyl)-4-piperidyl]carbamate (0.25 g, 64%). MS: found 664.3 M+H]⁺.

### (b) tert-Butyl N-[[6-[2-chloro-3-[3-chloro-2-(4-formyl-3-methoxy-phenyl)-4-pyridyl]phenyl]-2-methoxy-3-pyridyl]methyl]-N-[1-(3-methoxypropanoyl)-4-piperidyl]carbamate

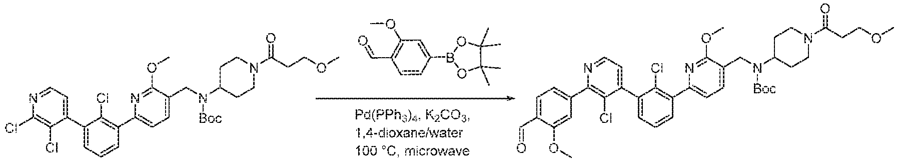

Tetrakis(triphenylphosphine)palladium(0) (27 mg, 0.02 mmol), potassium carbonate (120 mg, 0.87 mmol), tert-butyl N-[[6-[2-chloro-3-(2,3-dichloro-4-pyridyl)phenyl]-2-methoxy-3-pyridyl]methyl]-N-[1-(3-methoxypropanoyl)-4-piperidyl]carbamate (192 mg, 0.29 mmol), and 2-methoxy-4-(4,4,5,5-tetramethyl-1,3,2-dioxaborolan-2-yl)benzaldehyde (82 mg, 0.31 mmol) were suspended in 1,4-dioxane/water (4:1, 2 mL). The solution was irradiated to 100 °C for 30 minutes in a Biotage Initiator microwave. The reaction mixture was diluted with 10 mL water, and extracted with EtOAc (3 x 5 mL). The combined organics were concentrated under reduced pressure and the crude sample was purified by normal phase silica gel chromatography (20-100% EtOAc/hexanes) to afford tert-butyl N-[[6-[2-chloro-3-[3-chloro-2-(4-formyl-3-methoxy-phenyl)-4-pyridyl]phenyl]-2-methoxy-3-pyridyl]methyl]-N-[1-(3-methoxypropanoyl)-4-piperidyl]carbamate (200 mg, 91%) as a white foam . MS: m/z found 763.3 M+H]⁺.

### (c) 1-(4-(((6-(3-(2-(4-(((1-Acetylpiperidin-4-yl)amino)methyl)-3-methoxyphenyl)-3-chloropyridin-4-yl)-2-chlorophenyl)-2-methoxypyridin-3-yl)methyl)amino)piperidin-1-yl)-3-methoxypropan-1-one

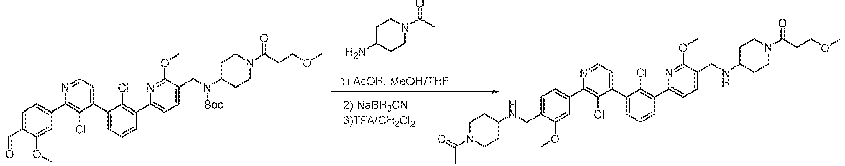

1-(4-(((6-(3-(2-(4-(((1-Acetylpiperidin-4-yl)amino)methyl)-3-methoxyphenyl)-3-chloropyridin-4-yl)-2-chlorophenyl)-2-methoxypyridin-3-yl)methyl)amino)piperidin-1-yl)-3-methoxypropan-1-one was prepared as the formic acid salt, following Reductive Amination Procedure B from tert-butyl ((6-(2-chloro-3-(3-chloro-2-(4-formyl-3-methoxyphenyl)pyridin-4-yl)phenyl)-2-methoxypyridin-3-yl)methyl)(1-(3-methoxypropanoyl)piperidin-4-yl)carbamate (32 mg, 0.04 mmol) and 1-(4-aminopiperidin-1-yl)ethan-1-one (15 mg, 0.10 mmol), followed by General Boc Deprotection Procedure. 51% yield. LCMS: m/z found 789.3 [M+H]⁺, retention time = 2.82 min, (Method C). ¹H NMR (400 MHz, Methanol-*d*₄): δ 8.66 (d, 1H), 8.49 (s, 2H), 7.87 (d, 1H), 7.72 (dd, 1H), 7.62 - 7.50 (m, 2H), 7.50 - 7.41 (m, 2H), 7.39 (d, 1H), 7.37 - 7.30 (m, 2H), 4.64 (s, 1H), 4.30 (s, 2H), 4.23 - 4.02 (m, 7H), 3.99 (s, 3H), 3.67 (td, 2H), 3.46 - 3.36 (m, 1H), 3.34 (s, 3H), 3.36 - 3.11 (m, 3H), 2.79 - 2.57 (m, 4H), 2.30 - 2.11 (m, 5H), 2.14 (s, 3H), 1.71 - 1.41 (m, 4H).

### Example 271: 1-(4-(((6-(2-Chloro-3-(3-chloro-2-(4-((4-hydroxypiperidin-1-yl)methyl)-3-methoxyphenyl)pyridin-4-yl)phenyl)-2-methoxypyridin-3-yl)methyl)amino)piperidin-1-yl)-3-methoxypropan-1-one (616)

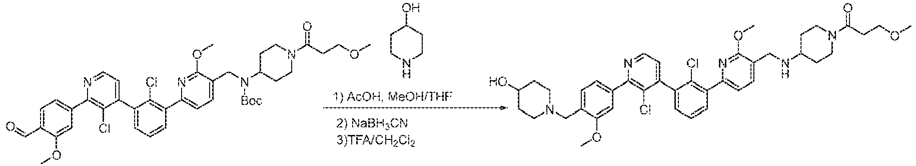

1-(4-(((6-(2-Chloro-3-(3-chloro-2-(4-((4-hydroxypiperidin-1-yl)methyl)-3-methoxyphenyl)pyridin-4-yl)phenyl)-2-methoxypyridin-3-yl)methyl)amino)piperidin-1-yl)-3-methoxypropan-1-one was prepared as the formic acid salt, following Reductive Amination Procedure B from tert-butyl ((6-(2-chloro-3-(3-chloro-2-(4-formyl-3-methoxyphenyl)pyridin-4-yl)phenyl)-2-methoxypyridin-3-yl)methyl)(1-(3-methoxypropanoyl)piperidin-4-yl)carbamate (31 mg, 0.04 mmol) and piperidin-4-ol (10 mg, 0.10 mmol), followed by General Boc Deprotection Procedure. 38% yield. LCMS: m/z found 748.3 [M+H]⁺, retention time = 2.79 min, (Method A). ¹H NMR (400 MHz, Methanol-*d*₄): δ 8.65 (d, 1H), 8.54 (s, 1H), 7.79 (d, 1H), 7.71 (dd, 1H), 7.60 - 7.39 (m, 4H), 7.38 - 7.25 (m, 3H), 4.55 (d, 1H), 4.04 (s, 4H), 3.98 - 3.94 (m, 5H), 3.88 - 3.75 (m, 1H), 3.66 (t, 2H), 3.33 (s, 3H), 3.28 - 3.09 (m, 3H), 2.97 (s, 1H), 2.85 - 2.58 (m, 6H), 2.08 (t, 2H), 2.02 - 1.92 (m, 2H), 1.73 (s, 2H), 1.56 - 1.25 (m, 2H).

### Example 272: 2-(4-(3-Chloro-4-(2-chloro-3-(6-methoxy-5-(((1-(3-methoxypropanoyl)piperidin-4-yl)amino)methyl)pyridin-2-yl)phenyl)pyridin-2-yl)-2-methoxybenzyl)-2,6-diazaspiro[3.4]octan-7-one (625)

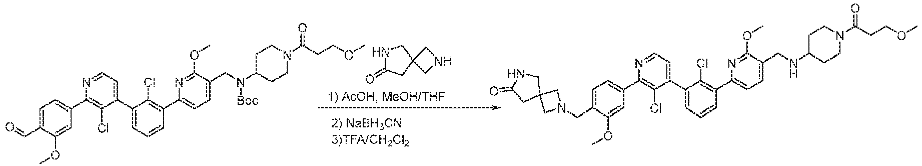

2-(4-(3-Chloro-4-(2-chloro-3-(6-methoxy-5-(((1-(3-methoxypropanoyl)piperidin-4-yl)amino)methyl)pyridin-2-yl)phenyl)pyridin-2-yl)-2-methoxybenzyl)-2,6-diazaspiro[3.4]octan-7-one was prepared as the formic acid salt, following Reductive Amination Procedure B from tert-butyl ((6-(2-chloro-3-(3-chloro-2-(4-formyl-3-methoxyphenyl)pyridin-4-yl)phenyl)-2-methoxypyridin-3-yl)methyl)(1-(3-methoxypropanoyl)piperidin-4-yl)carbamate (32 mg, 0.04 mmol) and 2,6-diazaspiro[3.4]octan-7-one (13 mg, 0.10 mmol), followed by General Boc Deprotection Procedure. 40% yield. LCMS: m/z found 773.4 [M+H]⁺, retention time = 2.71 min, (Method A). ¹H NMR (400 MHz, Methanol-*d*₄): δ 8.65 (d, 1H), 8.59 (s, 2H), 7.86 (d, 1H), 7.72 (dd, 1H), 7.57 (t, 1H), 7.49 - 7.40 (m, 3H), 7.36 - 7.28 (m, 3H), 4.65 (d, 1H), 4.16 (d, 4H), 4.07 (s, 3H), 3.95 (s, 3H), 3.86 (s, 4H), 3.69 - 3.64 (m, 2H), 3.63 (s, 2H), 3.34 (m, 4H), 3.17 (t, 1H), 2.78 - 2.68 (m, 2H), 2.66 (s, 3H), 2.64 - 2.60 (m, 1H), 2.19 (t, 2H), 1.65 - 1.40 (m, 2H).

### Example 273: 1-(4-(((6-(3-(2-(4-((6-Acetyl-2,6-diazaspiro[3.3]heptan-2-yl)methyl)-3-methoxyphenyl)-3-chloropyridin-4-yl)-2-chlorophenyl)-2-methoxypyridin-3-yl)methyl)amino)piperidin-1-yl)-3-methoxypropan-1-one (631)

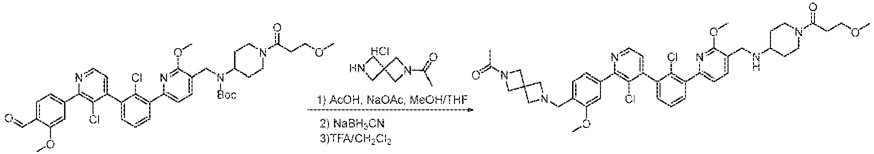

1-(4-(((6-(3-(2-(4-((6-Acetyl-2,6-diazaspiro[3.3]heptan-2-yl)methyl)-3-methoxyphenyl)-3-chloropyridin-4-yl)-2-chlorophenyl)-2-methoxypyridin-3-yl)methyl)amino)piperidin-1-yl)-3-methoxypropan-1-one was prepared as the formic acid salt, following Reductive Amination Procedure B from tert-butyl ((6-(2-chloro-3-(3-chloro-2-(4-formyl-3-methoxyphenyl)pyridin-4-yl)phenyl)-2-methoxypyridin-3-yl)methyl)(1-(3-methoxypropanoyl)piperidin-4-yl)carbamate (32 mg, 0.04 mmol) and 1-(2,6-diazaspiro[3.3]heptan-2-yl)ethan-1-one hydrochloride (19 mg, 0.11 mmol), followed by General Boc Deprotection Procedure. 34% yield. LCMS: m/z found 787.7 [M+H]⁺, retention time = 2.77 min, (Method A). ¹H NMR (400 MHz, Methanol-*d*₄): δ 8.64 (d, 1H), 8.53 (s, 1H), 7.85 (d, 1H), 7.72 (dd, 1H), 7.57 (t, 1H), 7.48 - 7.40 (m, 3H), 7.35 - 7.27 (m, 3H), 4.62 (d, 1H), 4.33 (s, 2H), 4.15 - 4.04 (m, 10H), 3.92 (d, 6H), 3.67 -3.63 (m, 2H), 3.34 (s, 3H), 3.20 (s, 2H), 3.15 (d, 1H), 2.78 - 2.67 (m, 2H), 2.66 - 2.58 (m, 1H), 2.16 (t, 2H), 1.84 (s, 3H), 1.62 - 1.38 (m, 2H).

### Example 274: (S)-1-(4-(((6-(2-Chloro-3-(3-chloro-2-(4-(((2-hydroxypropyl)amino)methyl)-3-methoxyphenyl)pyridin-4-yl)phenyl)-2-methoxypyridin-3-yl)methyl)amino)piperidin-1-yl)-3-methoxypropan-1-one (632)

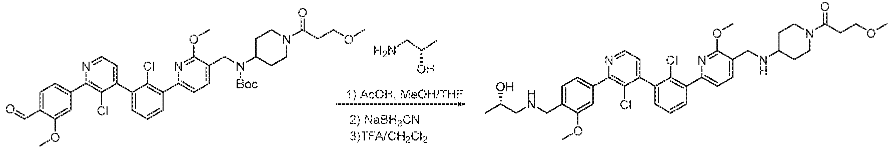

(S)-1-(4-(((6-(2-Chloro-3-(3-chloro-2-(4-(((2-hydroxypropyl)amino)methyl)-3-methoxyphenyl)pyridin-4-yl)phenyl)-2-methoxypyridin-3-yl)methyl)amino)piperidin-1-yl)-3-methoxypropan-1-one was prepared as the formic acid salt, following Reductive Amination Procedure B from tert-butyl ((6-(2-chloro-3-(3-chloro-2-(4-formyl-3-methoxyphenyl)pyridin-4-yl)phenyl)-2-methoxypyridin-3-yl)methyl)(1-(3-methoxypropanoyl)piperidin-4-yl)carbamate (29 mg, 0.04 mmol) and (S)-1-aminopropan-2-ol (7 mg, 0.09 mmol), followed by General Boc Deprotection Procedure. 46% yield. LCMS: m/z found 722.3 [M+H]⁺, retention time = 2.79 min, (Method A). ¹H NMR (400 MHz, Methanol-*d*₄): δ 8.66 (dd, 1H), 8.54 (s, 1H), 7.79 (d, 1H), 7.72 (dd, 1H), 7.56 (t, 1H), 7.52 - 7.45 (m, 2H), 7.42 (dd, 1H), 7.39 - 7.31 (m, 2H), 7.27 (dd, 1H), 4.54 (d, 1H), 4.24 (d, 2H), 4.10 - 4.02 (m, 1H), 4.03 (s, 3H), 3.97 (d, 5H), 3.66 (t, 2H), 3.35 (s, 3H), 3.19 - 3.08 (m, 1H), 3.05 - 2.88 (m, 3H), 2.81 (dd, 1H), 2.76 - 2.57 (m, 3H), 2.07 (t, 2H), 1.51 - 1.27 (m, 2H), 1.22 (dd, 3H).

### Example 275: 1-(6-((6-(2-Chloro-3-(3-chloro-2-(4-((6-(2-hydroxyacetyl)-2,6-diazaspiro[3.3]heptan-2-yl)methyl)-3-methoxyphenyl)pyridin-4-yl)phenyl)-2-methoxypyridin-3-yl)methyl)-2,6-diazaspiro[3.3]heptan-2-yl)-2-hydroxyethan-1-one (667)

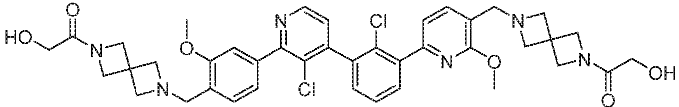

### (a) 1-(2,6-Diazaspiro[3.3]heptan-2-yl)-2-hydroxy-ethanone

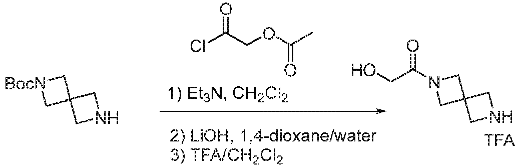

Triethylamine (0.94 mL, 6.78 mmol) was added to a mixture of 2,6-diazaspiro[3.3]heptane-2-carboxylic acid tert-butyl ester hemioxylate (0.75 g, 1.54 mmol) in 40 mL DCM. The mixture was cooled in an ice bath. A solution of (2-chloro-2-oxo-ethyl) acetate (0.36 mL, 3.39 mmol) in 10 mL DCM was added slowly. After 3h, the mixture was washed with 20 mL saturated sodium bicarbonate, 20 mL 0.2 M HCl, and 20 mL saturated sodium bicarbonate. The organics were dried over sodium sulfate, concentrated under reduced pressure, and purified by normal phase SiO₂ chromatography (15% to 100% EtOAc/hexanes) to provide tert-butyl 6-(2-acetoxyacetyl)-2,6-diazaspiro[3.3]heptane-2-carboxylate (0.59 g, 61% yield). MS: m/z found 299.2 [M+H]⁺. ¹H NMR (400 MHz, Chloroform-d) δ 4.48 (s, 2H), 4.33 (s, 2H), 4.16 (d, 2H), 4.13 - 4.03 (m, 4H), 2.15 (s, 2H), 1.43 (s, 9H).

A solution of lithium hydroxide hydrate (42 mg, 1.00 mmol) in 1.5 mL water was added slowly to a mixture of tert-butyl 6-(2-acetoxyacetyl)-2,6-diazaspiro[3.3]heptane-2-carboxylate (142 mg, 0.48 mmol) in 1.5 mL dioxane. After 10 minutes the mixture was cooled in an ice bath and acidified to pH=6 with 1 M HCl. The mixture was extracted with 4 x 10 mL DCM. The combined organics were dried over sodium sulfate. The organics were removed *in vacuo* to provide tert-butyl 6-(2-hydroxyacetyl)-2,6-diazaspiro[3.3]heptane-2-carboxylate (82 mg, 68% yield). MS: m/z found 257.2 [M+H]⁺. ¹H NMR (400 MHz, Chloroform-d) δ 4.20 (d, 4H), 4.08 (d, 4H), 3.98 (s, 2H), 1.43 (s, 9H).

Trifluoroacetic acid (0.80 mL, 3.19 mmol) was added to a 0 °C solution of tert-butyl 6-(2-hydroxyacetyl)-2,6-diazaspiro[3.3]heptane-2-carboxylate (82 mg, 0.32 mmol) in 3 mL DCM. After 10 min, the ice bath was removed. After 30 min, the volatiles were removed *in vacuo.* The resulting oil was azeotroped twice with 5 mL toluene to provide crude 1-(2,6-diazaspiro[3.3]heptan-2-yl)-2-hydroxy-ethanone as the TFA salt (86 mg). MS: m/z found 157.1 [M+H]⁺.

### (b) 1-[6-[[4-[3-Chloro-4-[2-chloro-3-[5-[[2-(2-hydroxyacetyl)-2,6-diazaspiro[3.3]heptan-6-yl]methyl]-6-methoxy-2-pyridyl]phenyl]-2-pyridyl]-2-methoxy-phenyl]methyl]-2,6-diazaspiro[3.3]heptan-2-yl]-2-hydroxy-ethanone

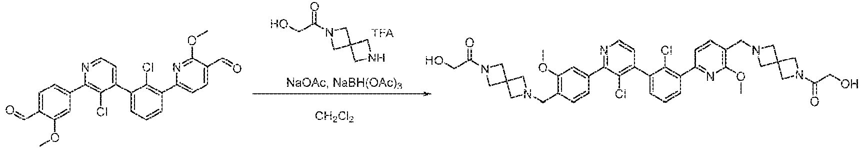

A mixture of 1-(2,6-diazaspiro[3.3]heptan-2-yl)-2-hydroxy-ethanone TFA salt (43 mg, 0.16 mmol) and sodium acetate (19 mg, 0.23 mmol) in 0.45 mL DCM was stirred for 10 min at room temperature. After the addition of 6-(2-chloro-3-(3-chloro-2-(4-formyl-3-methoxyphenyl)pyridin-4-yl)phenyl)-2-methoxynicotinaldehyde (28 mg, 0.06 mmol), the mixture was stirred for 5 min. Sodium triacetoxyborohydride (35 mg, 0.16 mmol) was added. After stirring overnight at room temperature, the reaction mixture was diluted with 2 mL MeOH and filtered through a syringe filter. The crude product was purified by reversed phase chromatography (5 to 65% acetonitrile gradient in water, with 0.05% formic acid) and the combined pure fractions were lyophilized to afford 1-[6-[[4-[3-chloro-4-[2-chloro-3-[5-[[2-(2-hydroxyacetyl)-2,6-diazaspiro[3.3]heptan-6-yl]methyl]-6-methoxy-2-pyridyl]phenyl]-2-pyridyl]-2-methoxy-phenyl]methyl]-2,6-diazaspiro[3.3]heptan-2-yl]-2-hydroxy-ethanone (9 mg, 19% yield) as the formic acid salt. LCMS: m/z found 773.4 [M+H]⁺, retention time = 2.47 min, (Method A). ¹H NMR (400 MHz, Methanol-*d*₄) δ 8.65 (d, 1H), 8.49 (s, 1H), 7.75 - 7.67 (m, 2H), 7.56 (t, 1H), 7.51 - 7.38 (m, 3H), 7.38 - 7.24 (m, 3H), 4.41 (d, 4H), 4.21 - 4.11 (m, 6H), 4.07 - 3.99 (m, 11H), 3.96 (s, 3H), 3.85 (s, 2H), 3.72 (s, 4H).

### Example 276: 1-(6-((6-(2-Chloro-3-(3-chloro-2-(3-methoxy-4-((6-(3-methoxypropanoyl)-2,6-diazaspiro[3.3]heptan-2-yl)methyl)phenyl)pyridin-4-yl)phenyl)-2-methoxypyridin-3-yl)methyl)-2,6-diazaspiro[3.3]heptan-2-yl)-3-methoxypropan-1-one (679)

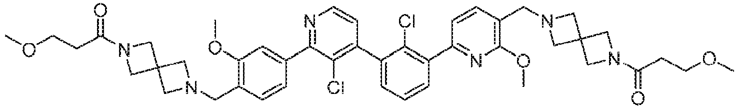

### (a) 1-(2,6-Diazaspiro[3.3]heptan-2-yl)-2-methoxy-ethanone

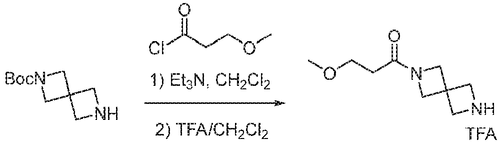

Triethylamine (0.94 mL, 6.78 mmol) was added to a mixture of 2,6-diazaspiro[3.3]heptane-2-carboxylic acid tert-butyl ester hemioxylate (0.75 g, 1.54 mmol) in 40 mL DCM. The mixture was cooled in an ice bath. A solution of 2-methoxyacetyl chloride (0.62 mL, 6.78 mmol) in 10 mL DCM was added slowly. After 3 h, the mixture was washed with 20 mL saturated sodium bicarbonate solution, 20 mL 0.2 M HCl, and 20 mL saturated sodium bicarbonate solution. The organics were dried over sodium sulfate, concentrated under reduced pressure, and purified by normal phase SiO₂ chromatography (15% to 100% EtOAc/hexanes) to provide tert-butyl 6-(3-methoxypropanoyl)-2,6-diazaspiro[3.3]heptane-2-carboxylate (0.59 g, 67% yield). ¹H NMR (400 MHz, Chloroform-*d*) δ 4.26 (s, 2H), 4.11 (s, 2H), 4.05 (s, 4H), 3.65 (t, 2H), 3.33 (s, 3H), 2.32 (t, 2H), 1.43 (s, 9H).

A solution of trifluoroacetic acid (1.76 mL, 7.03 mmol) in 3 mL DCM was added to a 0 °C solution of tert-butyl 6-(3-methoxypropanoyl)-2,6-diazaspiro[3.3]heptane-2-carboxylate (0.20 g, 0.70 mmol) in 7 mL DCM. After 10 min, the ice bath was removed. After 45 min, the volatiles were removed *in vacuo.* The resulting oil was azeotroped twice with 5 mL toluene to provide crude 1-(2,6-diazaspiro[3.3]heptan-2-yl)-2-methoxy-ethanone as the TFA salt (0.19 g). ¹H NMR (400 MHz, Methanol-*d*₄) δ 4.41 (d, 2H), 4.24 (s, 4H), 4.16 (s, 2H), 3.62 (t, 2H), 3.31 (s, 3H, overlaps with CD3OD), 2.35 (t, 2H). MS: m/z found 185.1 [M+H]⁺.

### (b) 1-[6-[[4-[3-Chloro-4-[2-chloro-3-[6-methoxy-5-[[2-(3-methoxypropanoyl)-2,6-diazaspiro[3.3]heptan-6-yl]methyl]-2-pyridyl]phenyl]-2-pyridyl]-2-methoxy-phenyl]methyl]-2,6-diazaspiro[3.3]heptan-2-yl]-3-methoxy-propan-1-one

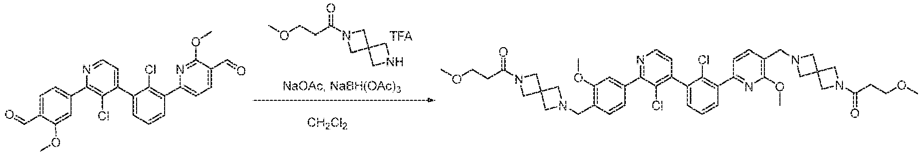

A mixture of 1-(2,6-diazaspiro[3.3]heptan-2-yl)-3-methoxy-propan-1-one TFA salt (47 mg, 0.16 mmol) and sodium acetate (19 mg, 0.23 mmol) in 0.45 mL DCM was stirred for 10 min at room temperature. After the additon of 6-(2-chloro-3-(3-chloro-2-(4-formyl-3-methoxyphenyl)pyridin-4-yl)phenyl)-2-methoxynicotinaldehyde (28 mg, 0.06 mmol), the mixture stirred for 5 min. Sodium triacetoxyborohydride (35 mg, 0.16 mmol) was added. After stirring overnight at room temperature, the reaction mixture was diluted with 2 mL MeOH and filtered through a syringe filter. The crude product was purified by reversed phase chromatography (5 to 65% acetonitrile gradient in water, with 0.05% formic acid) and the combined pure fractions were lyophilized to afford 1-[6-[[4-[3-chloro-4-[2-chloro-3-[6-methoxy-5-[[2-(3-methoxypropanoyl)-2,6-diazaspiro[3.3]heptan-6-yl]methyl]-2-pyridyl]phenyl]-2-pyridyl]-2-methoxy-phenyl]methyl]-2,6-diazaspiro[3.3]heptan-2-yl]-3-methoxy-propan-1-one (17 mg, 36% yield) as formic acid salt. LCMS: m/z found 829.3 [M+H]⁺, retention time = 2.74 min (Method A). ¹H NMR (400 MHz, Methanol-*d*₄) δ 8.64 (d, 1H), 8.52 (s, 1H), 7.74 - 7.67 (m, 2H), 7.55 (t, 1H), 7.48 - 7.38 (m, 3H), 7.36 - 7.23 (m, 3H), 4.35 (d, 4H), 4.13 - 4.06 (m, 6H), 4.01 (s, 3H), 3.94 (d, 7H), 3.80 (s, 2H), 3.68 - 3.57 (m, 8H), 3.33 - 3.29 (m, 6H), 2.36 - 2.32 (m, 4H).

### Example 277: N-(4-(3-chloro-4-(2-chloro-3-(6-methoxy-5-((methylamino)methyl)pyridin-2-yl)phenyl)pyridin-2-yl)-2-methoxybenzyl)propan-2-amine (211)

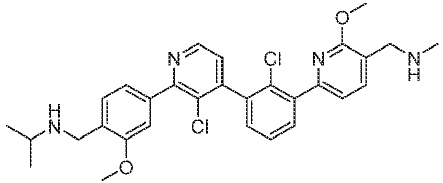

### (a) tert-Butyl ((6-(2-chloro-3-(2,3-dichloropyridin-4-yl)phenyl)-2-methoxypyridin-3-yl)methyl)(methyl)carbamate

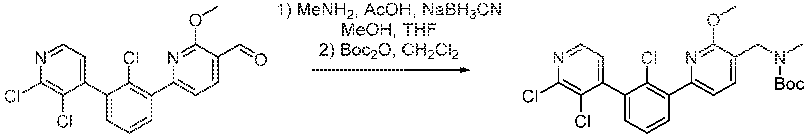

Reductive Amination Procedure B from 6-[2-chloro-3-(2,3-dichloro-4-pyridyl)phenyl]-2-methoxy-pyridine-3-carbaldehyde (400 mg, 1.02 mmol) and methanamine solution (33% wt in ethanol, 2.04 mmol) gave 1-[6-[2-chloro-3-(2,3-dichloro-4-pyridyl)phenyl]-2-methoxy-3-pyridyl]-N-methyl-methanamine, which was used in the next step without further purification. LCMS: m/z found 408.0 [M+H]⁺, retention time = 0.78 min (Method B).

To a solution of crude 1-[6-[2-chloro-3-(2,3-dichloro-4-pyridyl)phenyl]-2-methoxy-3-pyridyl]-N-methyl-methanamine in DCM was added di-tert-butyl dicarbonate (278 mg, 1.27 mmol). The mixture was stirred for 5 hours, concentrated in vacuo, and purified on silica gel column using MeOH in DCM (0 to 10% gradient) as eluent to give tert-butyl ((6-(2-chloro-3-(2,3-dichloropyridin-4-yl)phenyl)-2-methoxypyridin-3-yl)methyl)(methyl)carbamate. 75% yield over two steps. MS: m/z found 508.1 [M+H]⁺.

### (b) tert-Butyl ((6-(2-chloro-3-(3-chloro-2-(4-formyl-3-methoxyphenyl)pyridin-4-yl)phenyl)-2-methoxypyridin-3-yl)methyl)(methyl)carbamate

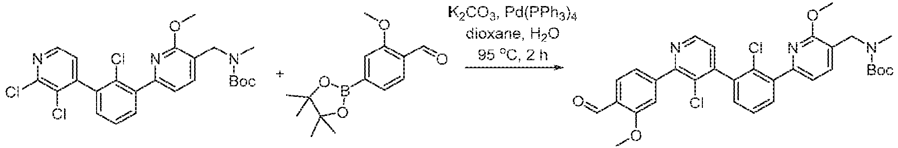

This intermediate was synthesized using Suzuki Coupling Procedure B from tert-butyl N-[[6-[2-chloro-3-(2,3-dichloro-4-pyridyl)phenyl]-2-methoxy-3-pyridyl]methyl]-N-methylcarbamate (110 mg, 0.22 mmol) and 2-methoxy-4-(4,4,5,5-tetramethyl-1,3,2-dioxaborolan-2-yl)benzaldehyde (57 mg, 0.22 mmol). 76% yield. LCMS: m/z found 608.1 [M+H]⁺, retention time = 1.21 min (Method B).

### (c) N-(4-(3-chloro-4-(2-chloro-3-(6-methoxy-5-((methylamino)methyl)pyridin-2-yl)phenyl)pyridin-2-yl)-2-methoxybenzyl)propan-2-amine

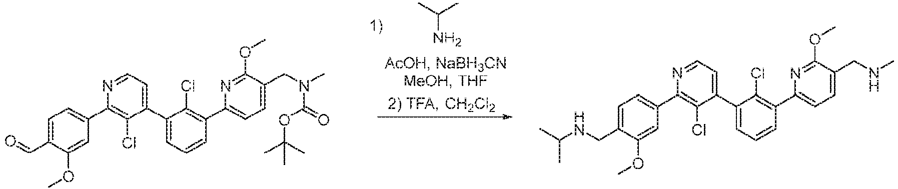

Reductive Amination Procedure B from tert-butyl ((6-(2-chloro-3-(3-chloro-2-(4-formyl-3-methoxyphenyl)pyridin-4-yl)phenyl)-2-methoxypyridin-3-yl)methyl)(methyl)carbamate (50 mg, 0.08 mmol) and propan-2-amine (10 mg, 0.16 mmol) gave tert-butyl N-[[6-[2-chloro-3-[3-chloro-2-[4-[(isopropylamino)methyl]-3-methoxy-phenyl]-4-pyridyl]phenyl]-2-methoxy-3-pyridyl]methyl]-N-methyl-carbamate as crude, LCMS: m/z found 651.2 [M+H]⁺, retention time = 0.99 min (Method B), which was deprotected and purified by General Boc Deprotection Procedure to afford the final product as formic acid salt. 65% yield over two steps. LCMS: m/z found 551.2 [M+H]⁺, retention time = 1.79 min (Method D). ¹H NMR (400 MHz, Methanol-*d*₄): δ 8.67 (d, 1H), 8.49 (s, 2H), 7.87 (d, 1H), 7.73 (d, 1H), 7.58 (t, 1H), 7.53 (d, 1H), 7.50 - 7.43 (m, 2H), 7.39 (d, 1H), 7.36 (d, 2H), 4.27 (s, 2H), 4.23 (s, 2H), 4.09 (s, 3H), 4.00 (s, 3H), 3.48 (p, 1H), 2.75 (s, 3H), 1.42 (d, 6H).

### Example 278: 1-(4-((4-(3-chloro-4-(2-chloro-3-(6-methoxy-5-((methylamino)methyl)pyridin-2-yl)phenyl)pyridin-2-yl)-2-methoxybenzyl)amino)piperidin-1-yl)ethan-1-one (212)

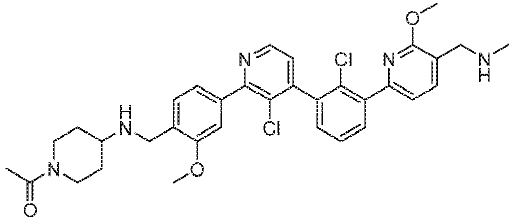

Reductive Amination Procedure B from tert-butyl ((6-(2-chloro-3-(3-chloro-2-(4-formyl-3-methoxyphenyl)pyridin-4-yl)phenyl)-2-methoxypyridin-3-yl)methyl)(methyl)carbamate (Example 277, step (b)) (35 mg, 0.06 mmol) and 1-(4-amino-1-piperidyl)ethanone (16 mg, 0.12 mmol) gave tert-butyl N-[[6-[3-[2-[4-[[(1-acetyl-4-piperidyl)amino]methyl]-3-methoxy-phenyl]-3-chloro-4-pyridyl]-2-chloro-phenyl]-2-methoxy-3-pyridyl]methyl]-N-methyl-carbamate as crude, LCMS: m/z found 734.3 [M+H]⁺, retention time = 0.95 min (Method B), which was deprotected and purified by General Boc Deprotection Procedure to afford the final product as formic acid salt. 80% yield over two steps. LCMS: m/z found 634.2 [M+H]⁺, retention time = 1.68 min (Method D). ¹H NMR (400 MHz, Methanol-*d*₄): δ 8.66 (dd, 1H), 8.47 (s, 2H), 7.90 - 7.83 (m, 1H), 7.76 - 7.68 (m, 1H), 7.58 (t, 1H), 7.53 (d, 1H), 7.49 - 7.43 (m, 2H), 7.39 (s, 1H), 7.38 - 7.31 (m, 2H), 4.66 (d, 1H), 4.29 (s, 2H), 4.24 (s, 2H), 4.09 (s, 3H), 3.99 (s, 3H), 3.52 - 3.34 (m, 2H), 3.20 (t, 1H), 2.76 (s, 3H), 2.69 (t, 1H), 2.22 (t, 2H), 2.13 (s, 3H), 1.69 - 1.44 (m, 2H).

### Example 279: 2-(4-(3-chloro-4-(2-chloro-3-(6-methoxy-5-((methylamino)methyl)pyridin-2-yl)phenyl)pyridin-2-yl)-2-methoxybenzyl)-2,6-diazaspiro[3.4]octan-7-one (213)

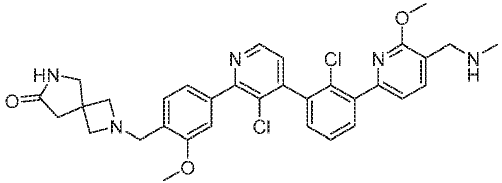

Reductive Amination Procedure B from tert-butyl ((6-(2-chloro-3-(3-chloro-2-(4-formyl-3-methoxyphenyl)pyridin-4-yl)phenyl)-2-methoxypyridin-3-yl)methyl)(methyl)carbamate (Example 277, step (b)) (35 mg, 0.06 mmol) and 2,6-diazaspiro[3.4]octan-7-one (15 mg, 0.12 mmol) gave tert-butyl N-[[6-[2-chloro-3-[3-chloro-2-[3-methoxy-4-[(6-oxo-2,7-diazaspiro[3.4]octan-2-yl)methyl]phenyl]-4-pyridyl]phenyl]-2-methoxy-3-pyridyl]methyl]-N-methyl-carbamate as crude, LCMS: m/z found 718.3 [M+H]⁺, retention time = 0.93 min (Method B), which was deprotected and purified by General Boc Deprotection Procedure to afford the final product as formic acid salt. 78% yield over two steps. LCMS: m/z found 618.2 [M+H]⁺, retention time = 1.63 min (Method D). ¹H NMR (400 MHz, Methanol-*d*₄): δ 8.65 (d, 1H), 8.41 (s, 2H), 7.90 (d, 1H), 7.76 - 7.69 (m, 1H), 7.58 (t, 1H), 7.52 (d, 1H), 7.47 - 7.41 (m, 2H), 7.38 - 7.30 (m, 3H), 4.33 - 4.18 (m, 4H), 4.09 (s, 3H), 4.05 - 3.98 (m, 4H), 3.97 (s, 3H), 3.66 (s, 2H), 2.77 (s, 3H), 2.68 (s, 2H).

### Example 280: (S)-4-((4-(3-chloro-4-(2-chloro-3-(6-methoxy-5-((methylamino)methyl)pyridin-2-yl)phenyl)pyridin-2-yl)-2-methoxybenzyl)amino)-3-hydroxybutanoic acid (220)

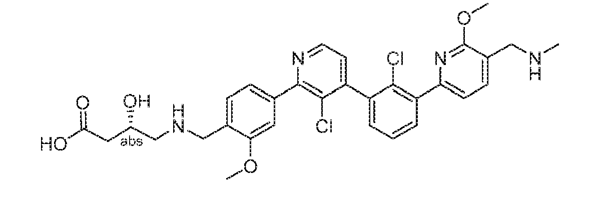

Reductive Amination Procedure B from tert-butyl ((6-(2-chloro-3-(3-chloro-2-(4-formyl-3-methoxyphenyl)pyridin-4-yl)phenyl)-2-methoxypyridin-3-yl)methyl)(methyl)carbamate (Example 277, step (b)) (30 mg, 0.05 mmol) and (3S)-4-amino-3-hydroxy-butanoic acid (12 mg, 0.10 mmol) gave (3S)-4-[[4-[4-[3-[5-[[tert-butoxycarbonyl(methyl)amino]methyl]-6-methoxy-2-pyridyl]-2-chloro-phenyl]-3-chloro-2-pyridyl]-2-methoxy-phenyl]methylamino]-3-hydroxy-butanoic acid as crude, MS: m/z found 711.2 [M+H]⁺, which was deprotected and purified by General Boc Deprotection Procedure to afford the final product as formic acid salt. 40% yield over two steps. LCMS: m/z found 611.2 [M+H]⁺, retention time = 1.62 min (Method D). ¹H NMR (400 MHz, Methanol-*d*₄): δ 8.70 - 8.63 (m, 1H), 8.41 - 8.35 (m, 2H), 7.87 (d, 1H), 7.76 - 7.69 (m, 1H), 7.61 - 7.55 (m, 1H), 7.55 - 7.50 (m, 1H), 7.48 - 7.43 (m, 2H), 7.40 - 7.31 (m, 3H), 4.32 (s, 2H), 4.25 (s, 2H), 4.24 - 4.18 (m, 1H), 4.09 (s, 3H), 3.99 (s, 3H), 3.20 (d, 1H), 3.02 (dd, 1H), 2.77 (s, 3H), 2.47 (d, 2H).

### Example 281: 3-((4-(3-chloro-4-(2-chloro-3-(6-methoxy-5-((methylamino)methyl)pyridin-2-yl)phenyl)pyridin-2-yl)-2-methoxybenzyl)amino)propanoic acid (221)

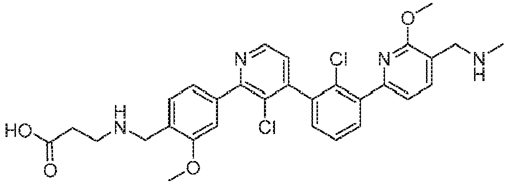

Reductive Amination Procedure B from tert-butyl ((6-(2-chloro-3-(3-chloro-2-(4-formyl-3-methoxyphenyl)pyridin-4-yl)phenyl)-2-methoxypyridin-3-yl)methyl)(methyl)carbamate (Example 277, step (b)) (30 mg, 0.05 mmol) and 3-aminopropanoic acid (9 mg, 0.10 mmol) gave 3-[[4-[4-[3-[5-[[tert-butoxycarbonyl(methyl)amino]methyl]-6-methoxy-2-pyridyl]-2-chloro-phenyl]-3-chloro-2-pyridyl]-2-methoxy-phenyl]methylamino]propanoic acid as crude, LCMS: m/z found 681.2 [M+H]⁺, retention time = 0.96 min (Method B), which was deprotected and purified by General Boc Deprotection Procedure to afford the final product as formic acid salt. 64% yield over two steps. LCMS: m/z found 581.2 [M+H]⁺, retention time = 1.65 min (Method D). ¹H NMR (400 MHz, Methanol-*d*₄): δ 8.66 (d, 1H), 8.36 (s, 2H), 7.87 (d, 1H), 7.77 - 7.68 (m, 1H), 7.58 (t, 1H), 7.51 (d, 1H), 7.46 (dd, 2H), 7.41 - 7.29 (m, 3H), 4.28 (s, 2H), 4.26 (s, 2H), 4.09 (s, 3H), 4.01 (s, 3H), 3.21 (t, 2H), 2.77 (s, 3H), 2.55 (t, 2H).

### Example 282: (S)-1-(4-(3-chloro-4-(2-chloro-3-(6-methoxy-5-((methylamino)methyl)pyridin-2-yl)phenyl)pyridin-2-yl)-2-methoxybenzyl)pyrrolidine-3-carboxylic acid (226)

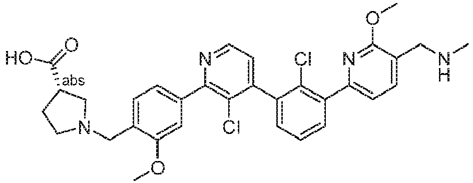

Reductive Amination Procedure B from tert-butyl ((6-(2-chloro-3-(3-chloro-2-(4-formyl-3-methoxyphenyl)pyridin-4-yl)phenyl)-2-methoxypyridin-3-yl)methyl)(methyl)carbamate (Example 277, step (b)) (30 mg, 0.05 mmol) and (3S)-pyrrolidine-3-carboxylic acid (11 mg, 0.10 mmol) gave (3S)-1-[[4-[4-[3-[5-[[tert-butoxycarbonyl(methyl)amino]methyl]-6-methoxy-2-pyridyl]-2-chloro-phenyl]-3-chloro-2-pyridyl]-2-methoxy-phenyl]methyl]pyrrolidine-3-carboxylic acid as crude, LCMS: m/z found 707.2 [M+H]⁺, retention time = 0.96 min (Method B), which was deprotected and purified by General Boc Deprotection Procedure to afford the final product as formic acid salt. 66% yield over two steps. LCMS: m/z found 607.2 [M+H]⁺, retention time = 2.02 min (Method C). ¹H NMR (400 MHz, Methanol-*d*₄): δ 8.67 (d, 1H), 8.44 (s, 2H), 7.87 (d, 1H), 7.76 - 7.71 (m, 1H), 7.62 - 7.50 (m, 2H), 7.48 - 7.44 (m, 2H), 7.43 - 7.31 (m, 3H), 4.48 (s, 2H), 4.25 (s, 2H), 4.09 (s, 3H), 4.01 (s, 3H), 3.66 - 3.56 (m, 1H), 3.43 (d, 3H), 3.15 - 3.06 (m, 1H), 2.77 (s, 3H), 2.43 - 2.31 (m, 1H), 2.32 - 2.17 (m, 1H).

### Example 283: 2-(4-(3-chloro-4-(2-chloro-3-(6-methoxy-5-((methylamino)methyl)pyridin-2-yl)phenyl)pyridin-2-yl)-2-methoxybenzyl)-2-azaspiro[3.3]heptane-6-carboxylic acid (227)

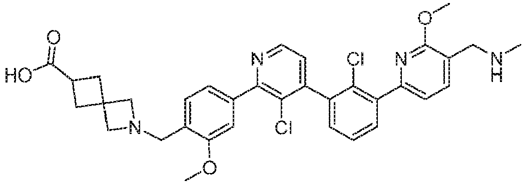

Reductive Amination Procedure B from tert-butyl ((6-(2-chloro-3-(3-chloro-2-(4-formyl-3-methoxyphenyl)pyridin-4-yl)phenyl)-2-methoxypyridin-3-yl)methyl)(methyl)carbamate (Example 277, step (b)) (30 mg, 0.05 mmol) and 2-azaspiro[3.3]heptane-6-carboxylic acid (14 mg, 0.10 mmol) gave 2-[[4-[4-[3-[5-[[tert-butoxycarbonyl(methyl)amino]methyl]-6-methoxy-2-pyridyl]-2-chloro-phenyl]-3-chloro-2-pyridyl]-2-methoxy-phenyl]methyl]-2-azaspiro[3.3]heptane-6-carboxylic acid as crude, LCMS: m/z found 733.3 [M+H]⁺, retention time = 0.97 min (Method B), which was deprotected and purified by General Boc Deprotection Procedure to afford the final product as formic acid salt. 60% yield over two steps. LCMS: m/z found 633.2 [M+H]⁺, retention time = 2.09 min (Method C). ¹H NMR (400 MHz, Methanol-*d*₄): δ 8.70 - 8.58 (m, 1H), 8.51 (s, 2H), 7.86 (d, 1H), 7.72 (dt, 1H), 7.58 (td, 1H), 7.52 - 7.42 (m, 3H), 7.42 - 7.31 (m, 2H), 7.32 - 7.23 (m, 1H), 4.44 - 4.33 (m, 2H), 4.22 (s, 2H), 4.12 (d, 2H), 4.09 (s, 3H), 4.01 - 3.88 (m, 4H), 2.90 - 2.82 (m, 1H), 2.78 (s, 1H), 2.75 (s, 3H), 2.51 - 2.37 (m, 2H), 2.36 - 2.27 (m, 1H), 1.92 - 1.84 (m, 1H).

### Example 284: 3-(((4-(3-chloro-4-(2-chloro-3-(6-methoxy-5-((methylamino)methyl)pyridin-2-yl)phenyl)pyridin-2-yl)-2-methoxybenzyl)amino)methyl)bicyclo[1.1.1]pentane-1-carboxylic acid (228)

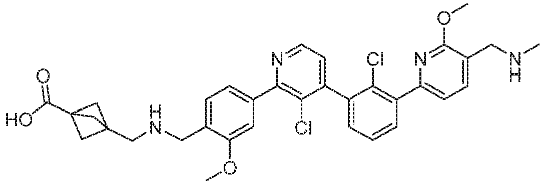

Reductive Amination Procedure B from tert-butyl ((6-(2-chloro-3-(3-chloro-2-(4-formyl-3-methoxyphenyl)pyridin-4-yl)phenyl)-2-methoxypyridin-3-yl)methyl)(methyl)carbamate (Example 277, step (b)) (30 mg, 0.05 mmol) and 3-(aminomethyl)bicyclo[1.1.1]pentane-1-carboxylic acid (14 mg, 0.10 mmol) gave 3-[[[4-[4-[3-[5-[[tert-butoxycarbonyl(methyl)amino]methyl]-6-methoxy-2-pyridyl]-2-chloro-phenyl]-3-chloro-2-pyridyl]-2-methoxy phenyl]methylamino]methyl]bicyclo[1.1.1]pentane-1-carboxylic acid as crude, LCMS: m/z found 733.3 [M+H]⁺, retention time = 0.96 min (Method B), which was deprotected and purified by General Boc Deprotection Procedure to afford the final product as formic acid salt. 61% yield over two steps. LCMS: m/z found 633.2 [M+H]⁺, retention time = 2.07 min (Method C). ¹H NMR (400 MHz, Methanol-*d*₄): δ 8.67 (d, 1H), 8.42 (s, 2H), 7.87 (d, 1H), 7.76 - 7.70 (m, 1H), 7.58 (t, 1H), 7.52 (d, 1H), 7.46 (t, 2H), 7.40 (s, 1H), 7.36 (t, 2H), 4.30 (s, 2H), 4.25 (s, 2H), 4.09 (s, 3H), 4.00 (s, 3H), 3.17 (s, 2H), 2.77 (s, 3H), 2.05 (s, 6H).

### Example 285: (S)-1-(4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)pyrrolidine-3-carboxylic acid (239)

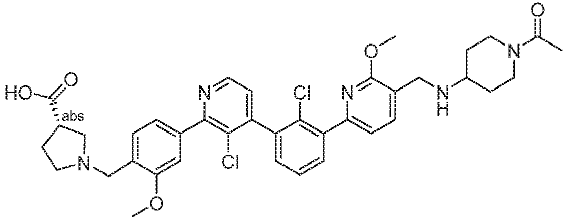

### (a) tert-Butyl (1-acetylpiperidin-4-yl)((6-(2-chloro-3-(2,3-dichloropyridin-4-yl)phenyl)-2-methoxypyridin-3-yl)methyl)carbamate

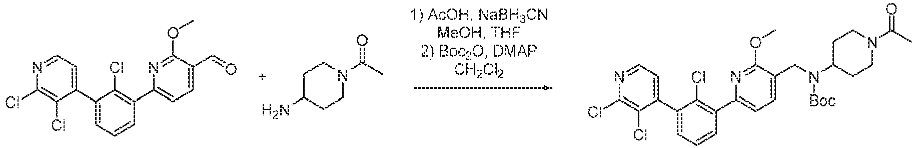

Reductive Amination Procedure B from 6-[2-chloro-3-(2,3-dichloro-4-pyridyl)phenyl]-2-methoxy-pyridine-3-carbaldehyde (250 mg, 0.64 mmol) and 1-(4-amino-1-piperidyl)ethanone (181 mg, 1.27 mmol) gave 1-[4-[[6-[2-chloro-3-(2,3-dichloro-4-pyridyl)phenyl]-2-methoxy-3-pyridyl]methylamino]-1-piperidyl]ethanone, which was used in the next step without further purification. LCMS: m/z found 519.1 [M+H]⁺, retention time = 0.79 min (Method B).

To a solution of crude 1-[4-[[6-[2-chloro-3-(2,3-dichloro-4-pyridyl)phenyl]-2-methoxy-3-pyridyl]methylamino]-1-piperidyl]ethenone (330 mg, 0.63 mmol) in DCM was added di-tert-butyl dicarbonate (153 mg, 0.70 mmol) and DMAP (7 mg, 0.06 mmol). The mixture was stirred for 2 hours, concentrated in vacuo, and purified on silica gel column using MeOH in DCM (0 to 10% gradient) as eluent to give tert-butyl (1-acetylpiperidin-4-yl)((6-(2-chloro-3-(2,3-dichloropyridin-4-yl)phenyl)-2-methoxypyridin-3-yl)methyl)carbamate (365 mg, 0.59 mmol). 92% yield over two steps. LCMS: m/z found 619.2 [M+H]⁺, retention time = 1.15 min (Method B).

### (b) tert-Butyl (1-acetylpiperidin-4-yl)((6-(2-chloro-3-(3-chloro-2-(4-formyl-3-methoxyphenyl)pyridin-4-yl)phenyl)-2-methoxypyridin-3-yl)methyl)carbamate

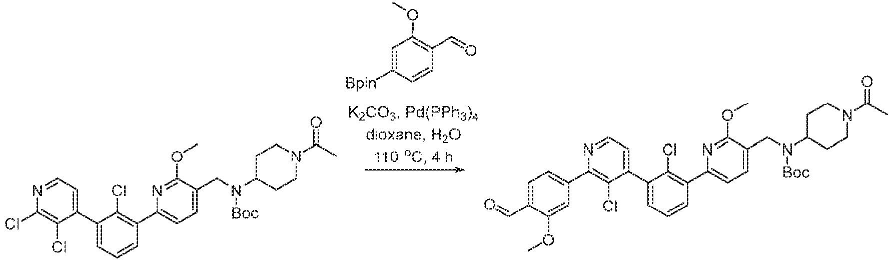

This intermediate was synthesized using Suzuki Coupling Procedure B from tert-butyl (1-acetylpiperidin-4-yl)((6-(2-chloro-3-(2,3-dichloropyridin-4-yl)phenyl)-2-methoxypyridin-3-yl)methyl)carbamate (280 mg, 0.45 mmol) and 2-methoxy-4-(4,4,5,5-tetramethyl-1,3,2-dioxaborolan-2-yl)benzaldehyde (118 mg, 0.45 mmol) by stirring at 110 °C for 4 hours. 77% yield. LCMS: m/z found 719.2 [M+H]⁺, retention time = 1.12 min (Method B).

### (c) (S)-1-(4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)pyrrolidine-3-carboxylic acid

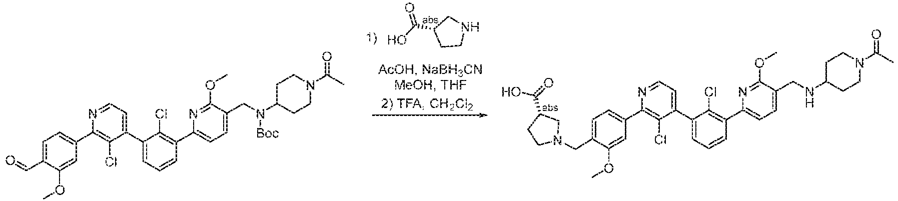

Reductive Amination Procedure B from tert-butyl (1-acetylpiperidin-4-yl)((6-(2-chloro-3-(3-chloro-2-(4-formyl-3-methoxyphenyl)pyridin-4-yl)phenyl)-2-methoxypyridin-3-yl)methyl)carbamate (30 mg, 0.04 mmol) and (3S)-pyrrolidine-3-carboxylic acid (10 mg, 0.08 mmol) gave (S)-1-(4-(4-(3-(5-(((1-acetylpiperidin-4-yl)(tert-butoxycarbonyl)amino)methyl)-6-methoxypyridin-2-yl)-2-chlorophenyl)-3-chloropyridin-2-yl)-2-methoxybenzyl)pyrrolidine-3-carboxylic acid as crude, LCMS: m/z found 818.3 [M+H]⁺, retention time = 0.87 min (Method B), which was deprotected and purified by General Boc Deprotection Procedure to afford the final product as formic acid salt. 55% yield over two steps. LCMS: m/z found 718.3 [M+H]⁺, retention time = 1.72 min (Method D). ¹H NMR (400 MHz, Methanol-*d*₄): δ 8.67 (d, 1H), 8.38 (s, 2H), 7.92 - 7.81 (m, 1H), 7.72 (d, 1H), 7.62 - 7.51 (m, 2H), 7.49 - 7.42 (m, 2H), 7.41 (s, 1H), 7.36 (t, 2H), 4.66 (d, 1H), 4.55 - 4.39 (m, 2H), 4.26 (s, 2H), 4.14 - 4.03 (m, 4H), 4.01 (s, 3H), 3.65 - 3.56 (m, 1H), 3.52 - 3.35 (m, 3H), 3.21 (t, 2H), 3.14 - 3.07 (m, 1H), 2.76 - 2.67 (m, 1H), 2.44 - 2.32 (m, 1H), 2.31 - 2.16 (m, 3H), 2.14 (s, 3H), 1.68 - 1.43 (m, 2H).

### Example 286: 1-(4-(((6-(2-chloro-3-(3-chloro-2-(3-methoxy-4-((methylamino)methyl)phenyl)pyridin-4-yl)phenyl)-2-methoxypyridin-3-yl)methyl)amino)piperidin-1-yl)ethan-1-one (240)

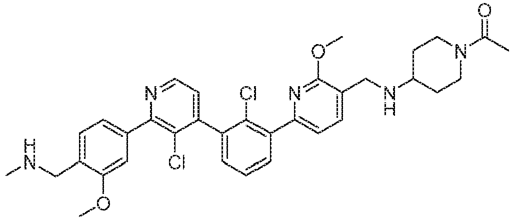

Reductive Amination Procedure B from tert-butyl (1-acetylpiperidin-4-yl)((6-(2-chloro-3-(3-chloro-2-(4-formyl-3-methoxyphenyl)pyridin-4-yl)phenyl)-2-methoxypyridin-3-yl)methyl)carbamate (Example 285, step (b)) (35 mg, 0.05 mmol) and methanamine solution (33% wt in ethanol, 0.10 mmol) gave tert-butyl N-(1-acetyl-4-piperidyl)-N-[[6-[2-chloro-3-[3-chloro-2-[3-methoxy-4-(methylaminomethyl)phenyl]-4-pyridyl]phenyl]-2-methoxy-3-pyridyl]methyl]carbamate as crude, LCMS: m/z found 734.3 [M+H]⁺, retention time = 0.88 min (Method B), which was deprotected and purified by General Boc Deprotection Procedure to afford the final product as formic acid salt. 60% yield over two steps. LCMS: m/z found 634.2 [M+H]⁺, retention time = 1.71 min (Method D). ¹H NMR (400 MHz, Methanol-*d*₄): δ 8.70 - 8.64 (m, 1H), 8.47 (s, 2H), 7.86 (d, 1H), 7.72 (d, 1H), 7.57 (t, 1H), 7.51 (d, 1H), 7.49 - 7.47 (m, 1H), 7.44 (d, 1H), 7.40 (s, 1H), 7.38 - 7.28 (m, 2H), 4.61 (d, 1H), 4.27 (s, 2H), 4.16 (s, 2H), 4.10 - 3.95 (m, 7H), 3.27 - 3.13 (m, 2H), 2.79 - 2.60 (m, 4H), 2.24 - 2.15 (m, 2H), 2.13 (s, 3H), 1.63 - 1.35 (m, 2H).

### Example 287: 2-(4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)-2-azaspiro[3.3]heptane-6-carboxylic acid (243)

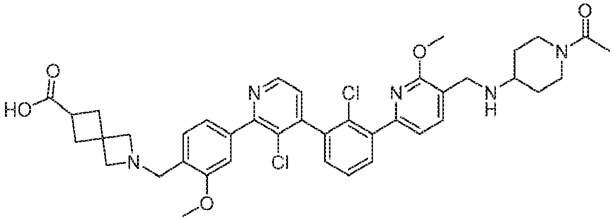

Reductive Amination Procedure B from tert-butyl (1-acetylpiperidin-4-yl)((6-(2-chloro-3-(3-chloro-2-(4-formyl-3-methoxyphenyl)pyridin-4-yl)phenyl)-2-methoxypyridin-3-yl)methyl)carbamate (Example 285, step (b)) (30 mg, 0.04 mmol) and 2-azaspiro[3.3]heptane-6-carboxylic acid (12 mg, 0.08 mmol) gave 2-[[4-[4-[3-[5-[[(1-acetyl-4-piperidyl)-tert-butoxycarbonyl-amino]methyl]-6-methoxy-2-pyridyl]-2-chloro-phenyl]-3-chloro-2-pyridyl]-2-methoxy-phenyl]methyl]-2-azaspiro[3.3]heptane-6-carboxylic acid as crude, LCMS: m/z found 844.3 [M+H]⁺, retention time = 0.89 min (Method B), which was deprotected and purified by General Boc Deprotection Procedure to afford the final product as formic acid salt. 48% yield over two steps. LCMS: m/z found 744.3 [M+H]⁺, retention time = 1.79 min (Method D). ¹H NMR (400 MHz, Methanol-*d*₄): δ 8.66 (d, 1H), 8.45 (s, 2H), 7.87 (d, 1H), 7.72 (d, 1H), 7.57 (t, 1H), 7.48 (ddd, 3H), 7.41 - 7.28 (m, 3H), 4.63 (d, 1H), 4.47 - 4.33 (m, 2H), 4.27 - 4.11 (m, 4H), 4.09 - 4.00 (m, 5H), 4.00 - 3.91 (m, 3H), 3.36 - 3.26 (m, 2H), 3.20 (t, 1H), 2.89 (t, 1H), 2.71 (t, 1H), 2.57 - 2.31 (m, 3H), 2.26 - 2.08 (m, 5H), 1.92 (d, 1H), 1.66 - 1.40 (m, 2H).

### Example 288: 2-(4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)-2,6-diazaspiro [3.4] octan-7-one (244)

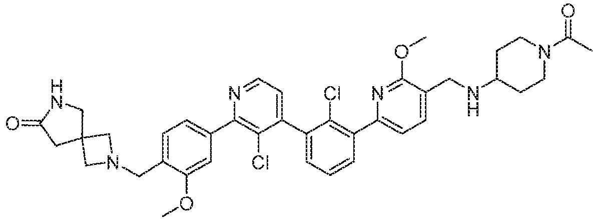

Reductive Amination Procedure B from tert-butyl (1-acetylpiperidin-4-yl)((6-(2-chloro-3-(3-chloro-2-(4-formyl-3-methoxyphenyl)pyridin-4-yl)phenyl)-2-methoxypyridin-3-yl)methyl)carbamate (Example 285, step (b)) (30 mg, 0.04 mmol) and 2,6-diazaspiro[3.4]octan-7-one (11 mg, 0.08 mmol) gave tert-butyl N-(1-acetyl-4-piperidyl)-N-[[6-[2-chloro-3-[3-chloro-2-[3-methoxy-4-[(6-oxo-2,7-diazaspiro[3.4]octan-2-yl)methyl]phenyl]-4-pyridyl]phenyl]-2-methoxy-3-pyridyl]methyl]carbamate as crude, LCMS: m/z found 829.3 [M+H]⁺, retention time = 0.86 min (Method B), which was deprotected and purified by General Boc Deprotection Procedure to afford the final product as formic acid salt. 30% yield over two steps. LCMS: m/z found 729.3 [M+H]⁺, retention time = 1.64 min (Method D). ¹H NMR (400 MHz, Methanol-*d*₄): δ 8.67 - 8.62 (m, 1H), 8.45 (s, 2H), 7.87 (d, 1H), 7.72 (d, 1H), 7.61 - 7.53 (m, 1H), 7.50 - 7.41 (m, 3H), 7.38 - 7.29 (m, 3H), 4.71 - 4.53 (m, 1H), 4.21 (s, 4H), 4.12 - 4.00 (m, 4H), 3.98 - 3.89 (m, 6H), 3.64 (s, 2H), 3.37 - 3.26 (m, 2H), 3.20 (t, 1H), 2.79 - 2.60 (m, 3H), 2.27 - 2.08 (m, 5H), 1.67 - 1.38 (m, 2H).

### Example 289: Isopropyl (S)-1-(4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)pyrrolidine-3-carboxylate (245)

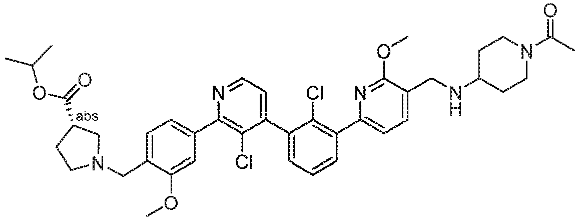

Reductive Amination Procedure B from tert-butyl (1-acetylpiperidin-4-yl)((6-(2-chloro-3-(3-chloro-2-(4-formyl-3-methoxyphenyl)pyridin-4-yl)phenyl)-2-methoxypyridin-3-yl)methyl)carbamate (Example 285, step (b)) (35 mg, 0.05 mmol) and isopropyl (3S)-pyrrolidine-3-carboxylate (15 mg, 0.10 mmol) gave isopropyl (3S)-1-[[4-[4-[3-[5-[[(1-acetyl-4-piperidyl)-tert-butoxycarbonyl-amino]methyl]-6-methoxy-2-pyridyl]-2-chloro-phenyl]-3-chloro-2-pyridyl]-2-methoxy-phenyl]methyl]pyrrolidine-3-carboxylate as crude, LCMS: m/z found 860.4 [M+H]⁺, retention time = 0.96 min (Method B), which was deprotected and purified by General Boc Deprotection Procedure to afford the final product as formic acid salt. 60% yield over two steps. LCMS: m/z found 760.3 [M+H]⁺, retention time = 2.07 min (Method D). ¹H NMR (400 MHz, Methanol-*d*₄): δ 8.64 (d, 1H), 8.51 (s, 2H), 7.83 (d, 1H), 7.72 (d, 1H), 7.56 (t, 1H), 7.50 (d, 1H), 7.47 - 7.40 (m, 2H), 7.36 - 7.28 (m, 3H), 5.05 - 4.97 (m, 1H), 4.57 (d, 1H), 4.15 - 3.96 (m, 8H), 3.95 (s, 3H), 3.28 - 2.95 (m, 7H), 2.71 (t, 1H), 2.33 - 2.06 (m, 7H), 1.57 - 1.35 (m, 2H), 1.25 (d, 6H).

### Example 290: Isopropyl (S)-1-(4-(3-chloro-4-(2-chloro-3-(6-methoxy-5-((methylamino)methyl)pyridin-2-yl)phenyl)pyridin-2-yl)-2-methoxybenzyl)pyrrolidine-3-carboxylate (278)

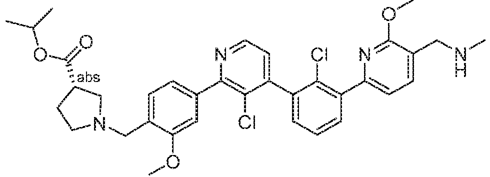

Reductive Amination Procedure B from tert-butyl ((6-(2-chloro-3-(3-chloro-2-(4-formyl-3-methoxyphenyl)pyridin-4-yl)phenyl)-2-methoxypyridin-3-yl)methyl)(methyl)carbamate (Example 277, step (b)) (50 mg, 0.08 mmol) and isopropyl (3S)-pyrrolidine-3-carboxylate (26 mg, 0.16 mmol) gave isopropyl (3S)-1-[[4-[4-[3-[5-[[tert-butoxycarbonyl(methyl)amino]methyl]-6-methoxy-2-pyridyl]-2-chloro-phenyl]-3-chloro-2-pyridyl]-2-methoxy-phenyl]methyl]pyrrolidine-3-carboxylate as crude, LCMS: m/z found 749.3 [M+H]⁺, retention time = 1.04 min (Method B), which was deprotected and purified by General Boc Deprotection Procedure to afford the final product as formic acid salt. 55% yield over two steps. LCMS: m/z found 649.3 [M+H]⁺, retention time = 2.08 min (Method D). ¹H NMR (400 MHz, Methanol-*d*₄): δ 8.65 - 8.60 (m, 1H), 8.58 - 8.52 (m, 2H), 7.79 (d, 1H), 7.71 (d, 1H), 7.56 (t, 1H), 7.48 - 7.39 (m, 3H), 7.34 - 7.21 (m, 3H), 5.05 - 4.91 (m, 1H), 4.05 (s, 3H), 4.00 (s, 2H), 3.91 (s, 3H), 3.80 (s, 2H), 3.09 - 3.00 (m, 1H), 3.00 - 2.92 (m, 1H), 2.87 - 2.75 (m, 2H), 2.72 - 2.67 (m, 1H), 2.15 - 2.05 (m, 2H), 1.23 (d, 6H).

### Example 291: Isopropyl 3-((4-(3-chloro-4-(2-chloro-3-(6-methoxy-5-((methylamino)methyl)pyridin-2-yl)phenyl)pyridin-2-yl)-2-methoxybenzyl)amino)propanoate (279)

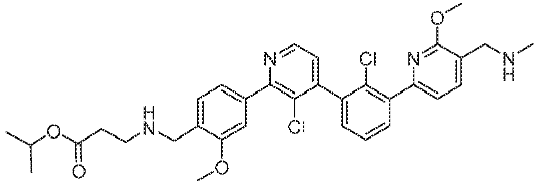

Reductive Amination Procedure B from tert-butyl ((6-(2-chloro-3-(3-chloro-2-(4-formyl-3-methoxyphenyl)pyridin-4-yl)phenyl)-2-methoxypyridin-3-yl)methyl)(methyl)carbamate (Example 277, step (b)) (50 mg, 0.08 mmol) and isopropyl 3-aminopropanoate (22 mg, 0.16 mmol) gave isopropyl 3-[[4-[4-[3-[5-[[tert-butoxycarbonyl(methyl)amino]methyl]-6-methoxy-2-pyridyl]-2-chloro-phenyl]-3-chloro-2-pyridyl]-2-methoxy-phenyl]methylamino]propanoate as crude, LCMS: m/z found 723.3 [M+H]⁺, retention time = 1.03 min (Method B), which was deprotected and purified by General Boc Deprotection Procedure to afford the final product as formic acid salt. 65% yield over two steps. LCMS: m/z found 623.2 [M+H]⁺, retention time = 2.05 min (Method D). ¹H NMR (400 MHz, Methanol-*d*₄): δ 8.67 (s, 1H), 7.87 (d, 1H), 7.76 - 7.70 (m, 1H), 7.68 - 7.63 (m, 1H), 7.63 - 7.55 (m, 2H), 7.54 - 7.43 (m, 3H), 7.42 - 7.32 (m, 2H), 5.07 (q, 1H), 4.34 (s, 2H), 4.26 (s, 2H), 4.09 (s, 3H), 4.01 (s, 3H), 3.34 (t, 2H), 2.82 - 2.73 (m, 5H), 1.27 (d, 6H).

### Example 292: Isopropyl (S)-4-((4-(3-chloro-4-(2-chloro-3-(6-methoxy-5-((methylamino)methyl)pyridin-2-yl)phenyl)pyridin-2-yl)-2-methoxybenzyl)amino)-3-hydroxybutanoate (280)

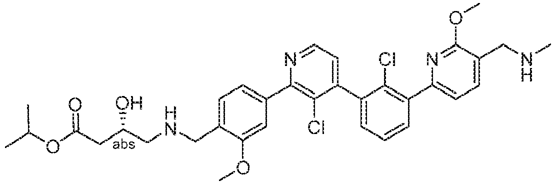

Reductive Amination Procedure B from tert-butyl ((6-(2-chloro-3-(3-chloro-2-(4-formyl-3-methoxyphenyl)pyridin-4-yl)phenyl)-2-methoxypyridin-3-yl)methyl)(methyl)carbamate (Example 277, step (b)) (50 mg, 0.08 mmol) and isopropyl (3S)-4-amino-3-hydroxy-butanoate (26 mg, 0.16 mmol) gave isopropyl 3-[[4-[4-[3-[5-[[tert-butoxycarbonyl(methyl)amino]methyl]-6-methoxy-2-pyridyl]-2-chloro-phenyl]-3-chloro-2-pyridyl]-2-methoxy-phenyl]methylamino]propanoate as crude, LCMS: m/z found 753.3 [M+H]⁺, retention time = 1.02 min (Method B), which was deprotected and purified by General Boc Deprotection Procedure to afford the final product as formic acid salt. 60% yield over two steps. LCMS: m/z found 653.2 [M+H]⁺, retention time = 1.96 min (Method D). ¹H NMR (400 MHz, Methanol-*d*₄): δ 8.66 (d, 1H), 8.52 (s, 2H), 7.86 (d, 1H), 7.73 (d, 1H), 7.58 (t, 1H), 7.52 - 7.42 (m, 3H), 7.39 - 7.30 (m, 3H), 5.06 - 4.96 (m, 1H), 4.36 - 4.26 (m, 1H), 4.24 (s, 2H), 4.20 (s, 2H), 4.08 (s, 3H), 3.99 (s, 3H), 3.11 (d, 1H), 2.99 - 2.89 (m, 1H), 2.73 (s, 3H), 2.56 - 2.49 (m, 2H), 1.24 (d, 6H).

### Example 293: Isopropyl 2-(4-(3-chloro-4-(2-chloro-3-(6-methoxy-5-((methylamino)methyl)pyridin-2-yl)phenyl)pyridin-2-yl)-2-methoxybenzyl)-2-azaspiro[3.3]heptane-6-carboxylate (281)

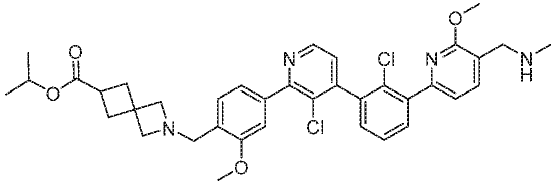

Reductive Amination Procedure B from tert-butyl ((6-(2-chloro-3-(3-chloro-2-(4-formyl-3-methoxyphenyl)pyridin-4-yl)phenyl)-2-methoxypyridin-3-yl)methyl)(methyl)carbamate (Example 277, step (b)) (50 mg, 0.08 mmol) and isopropyl 2-azaspiro[3.3]heptane-6-carboxylate (30 mg, 0.16 mmol) gave isopropyl 2-[[4-[4-[3-[5-[[tert-butoxycarbonyl(methyl)amino]methyl]-6-methoxy-2-pyridyl]-2-chloro-phenyl]-3-chloro-2-pyridyl]-2-methoxy-phenyl]methyl]-2-azaspiro[3.3]heptane-6-carboxylate as crude, LCMS: m/z found 775.3 [M+H]⁺, retention time = 1.05 min (Method B), which was deprotected and purified by General Boc Deprotection Procedure to afford the final product as formic acid salt. 50% yield over two steps. LCMS: m/z found 675.2 [M+H]⁺, retention time = 2.17 min (Method D). ¹H NMR (400 MHz, Methanol-*d*₄): δ 8.65 (dd, 1H), 8.52 (s, 2H), 7.85 (d, 1H), 7.73 (d, 1H), 7.58 (t, 1H), 7.50 - 7.42 (m, 3H), 7.40 - 7.29 (m, 3H), 5.03 - 4.93 (m, 1H), 4.24 (s, 2H), 4.19 (s, 2H), 4.08 (s, 3H), 4.02 - 3.93 (m, 7H), 3.05 - 2.93 (m, 1H), 2.73 (s, 3H), 2.57 - 2.47 (m, 2H), 2.47 - 2.38 (m, 2H), 1.22 (dt, 6H).

### Example 294: 1-(4-(((6-(3-(2-(4-(((1-acetylpiperidin-4-yl)(methyl)amino)methyl)-3-methoxyphenyl)-3-chloropyridin-4-yl)-2-chlorophenyl)-2-methoxypyridin-3-yl)methyl)amino)piperidin-1-yl)ethan-1-one (315)

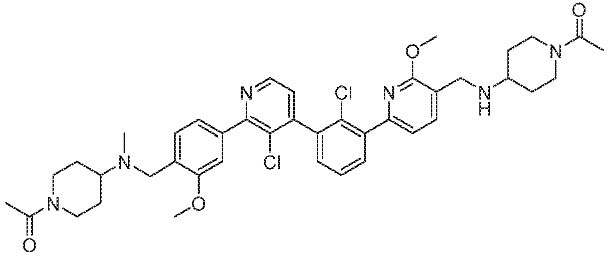

Reductive Amination Procedure B from tert-butyl (1-acetylpiperidin-4-yl)((6-(2-chloro-3-(3-chloro-2-(4-formyl-3-methoxyphenyl)pyridin-4-yl)phenyl)-2-methoxypyridin-3-yl)methyl)carbamate (Example 285, step (b)) (30 mg, 0.04 mmol) and 1-[4-(methylamino)-1-piperidyl]ethanone (13 mg, 0.08 mmol) gave tert-butyl N-(1-acetyl-4-piperidyl)-N-[[6-[3-[2-[4-[[(1-acetyl-4-piperidyl)-methyl-amino]methyl]-3-methoxy-phenyl]-3-chloro-4-pyridyl]-2-chloro-phenyl]-2-methoxy-3-pyridyl]methyl]carbamate as crude, LCMS: m/z found 859.4 [M+H]⁺, which was deprotected and purified by General Boc Deprotection Procedure to afford the final product as formic acid salt. 48% yield over two steps. LCMS: m/z found 759.3 [M+H]⁺, retention time = 1.73 min (Method D). ¹H NMR (400 MHz, Methanol-*d*₄): δ 8.67 - 8.61 (m, 1H), 8.52 (s, 2H), 7.81 (d, 1H), 7.74 - 7.68 (m, 1H), 7.56 (td, 1H), 7.50 (d, 1H), 7.47 - 7.39 (m, 2H), 7.36 - 7.24 (m, 3H), 4.68 (d, 1H), 4.54 (d, 1H), 4.14 - 3.88 (m, 12H), 3.22 - 3.09 (m, 3H), 3.07 - 2.95 (m, 1H), 2.78 - 2.59 (m, 2H), 2.53 (s, 3H), 2.17 - 2.01 (m, 10H), 1.82 - 1.54 (m, 2H), 1.53 - 1.29 (m, 2H).

### Example 295: 2-((6-(3-(2-(4-(((1-acetylpiperidin-4-yl)amino)methyl)-3-methoxyphenyl)-3-chloropyridin-4-yl)-2-chlorophenyl)-2-methoxypyridin-3-yl)methyl)-2,6-diazaspiro[3.4]octan-7-one (332)

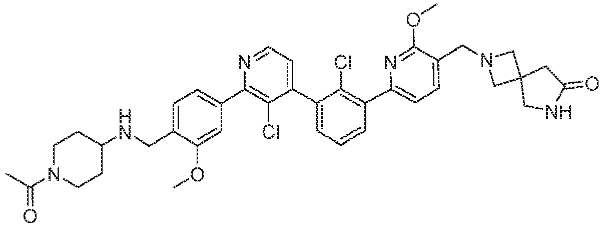

### (a) tert-Butyl (1-acetylpiperidin-4-yl)(2-methoxy-4-(4,4,5,5-tetramethyl-1,3,2-dioxaborolan-2-yl)benzyl)carbamate

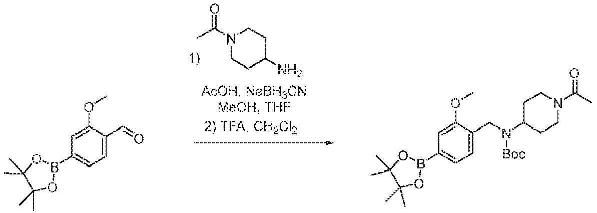

Reductive Amination Procedure B from 2-methoxy-4-(4,4,5,5-tetramethyl-1,3,2-dioxaborolan-2-yl)benzaldehyde (400 mg, 1.53 mmol) and 1-(4-amino-1-piperidyl)ethanone (391 mg, 2.75 mmol) gave 1-[4-[[2-methoxy-4-(4,4,5,5-tetramethyl-1,3,2-dioxaborolan-2-yl)phenyl]methylamino]-1-piperidyl]ethanone, which was used in the next step without further purification. LCMS: m/z found 389.3 [M+H]⁺, retention time = 0.69 min (Method B).

To a solution of crude 1-[4-[[2-methoxy-4-(4,4,5,5-tetramethyl-1,3,2-dioxaborolan-2-yl)phenyl]methylamino]-1-piperidyl]ethanone in DCM was added di-tert-butyl dicarbonate (333 mg, 1.53 mmol) and DMAP (37 mg, 0.31 mmol). The mixture was stirred for 2 hours, concentrated in vacuo, and purified on silica gel column using MeOH in DCM (0 to 10% gradient) as eluent to give tert-butyl (1-acetylpiperidin-4-yl)(2-methoxy-4-(4,4,5,5-tetramethyl-1,3,2-dioxaborolan-2-yl)benzyl)carbamate (395 mg, 0.81 mmol, 53% yield over two steps). LCMS: m/z found 489.3 [M+H]⁺, retention time = 1.06 min (Method B).

### (b) tert-Butyl (1-acetylpiperidin-4-yl)(4-(3-chloro-4-(2-chloro-3-(5-formyl-6-methoxypyridin-2-yl)phenyl)pyridin-2-yl)-2-methoxybenzyl)carbamate

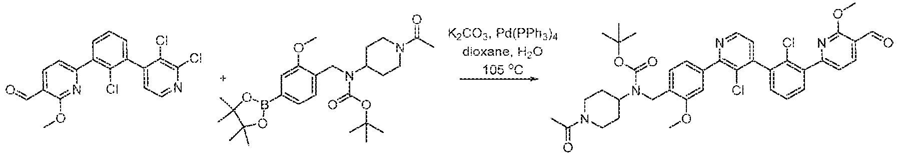

To a mixture of 6-[2-chloro-3-(2,3-dichloro-4-pyridyl)phenyl]-2-methoxy-pyridine-3-carbaldehyde (100 mg, 0.25 mmol), tert-butyl (1-acetylpiperidin-4-yl)(2-methoxy-4-(4,4,5,5-tetramethyl-1,3,2-dioxaborolan-2-yl)benzyl)carbamate (124 mg, 0.25 mmol) and potassium carbonate (105 mg, 0.76 mmol) in degassed dioxane/water (6: 1) was added tetrakis(triphenylphosphine)palladium(0) (29 mg, 0.03 mmol) and the mixture was stirred at 105 °C for 30 min. Water was added and the mixture was extracted into EtOAc three times. The combined organic layers were dried over anhydrous sodium sulfate, concentrated, and purified on silica gel column using 2% MeOH in DCM as eluent to provide tert-butyl (1-acetylpiperidin-4-yl)(4-(3-chloro-4-(2-chloro-3-(5-formyl-6-methoxypyridin-2-yl)phenyl)pyridin-2-yl)-2-methoxybenzyl)carbamate (170 mg, 93%). LCMS: m/z found 719.2 [M+H]⁺, retention time = 1.11 min (Method B).

### (c) 2-((6-(3-(2-(4-(((1-acetylpiperidin-4-yl)amino)methyl)-3-methoxyphenyl)-3-chloropyridin-4-yl)-2-chlorophenyl)-2-methoxypyridin-3-yl)methyl)-2,6-diazaspiro[3.4]octan-7-one

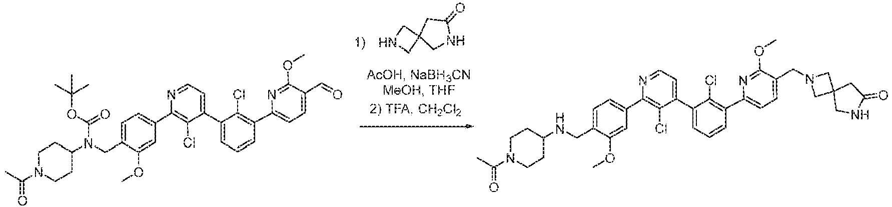

Reductive Amination Procedure B from tert-butyl (1-acetylpiperidin-4-yl)(4-(3-chloro-4-(2-chloro-3-(5-formyl-6-methoxypyridin-2-yl)phenyl)pyridin-2-yl)-2-methoxybenzyl)carbamate (50 mg, 0.07 mmol) and 2,6-diazaspiro[3.4]octan-7-one (18 mg, 0.14 mmol) gave tert-butyl N-(1-acetyl-4-piperidyl)-N-[[4-[3-chloro-4-[2-chloro-3-[6-methoxy-5-[(6-oxo-2,7-diazaspiro[3.4]octan-2-yl)methyl]-2-pyridyl]phenyl]-2-pyridyl]-2-methoxy-phenyl]methyl]carbamate as crude, LCMS: m/z found 829.3 [M+H]⁺, retention time = 0.84 min (Method B), which was deprotected and purified by General Boc Deprotection Procedure to afford the final product as formic acid salt. 82% yield over two steps. LCMS: m/z found 729.3 [M+H]⁺, retention time = 1.65 min (Method D). ¹H NMR (400 MHz, Methanol-*d*₄): δ 8.65 (d, 1H), 8.52 (s, 2H), 7.70 (d, 2H), 7.55 (t, 1H), 7.51 (d, 1H), 7.47 (d, 1H), 7.41 (d, 1H), 7.37 (s, 1H), 7.33 (d, 1H), 7.25 (d, 1H), 4.73 - 4.54 (m, 1H), 4.22 (s, 2H), 4.05 (d, 1H), 4.01 - 3.94 (m, 6H), 3.75 (s, 2H), 3.59 (s, 2H), 3.51 - 3.41 (m, 4H), 3.25 - 3.11 (m, 2H), 2.69 (t, 1H), 2.59 (s, 2H), 2.25 - 2.14 (m, 2H), 2.13 (s, 3H), 1.66 - 1.40 (m, 2H).

### Example 296: 1-(4-((4-(4-(3-(5-(((1-acetylpiperidin-4-yl)(methyl)amino)methyl)-6-methoxypyridin-2-yl)-2-chlorophenyl)-3-chloropyridin-2-yl)-2-methoxybenzyl)amino)piperidin-1-yl)ethan-1-one (333)

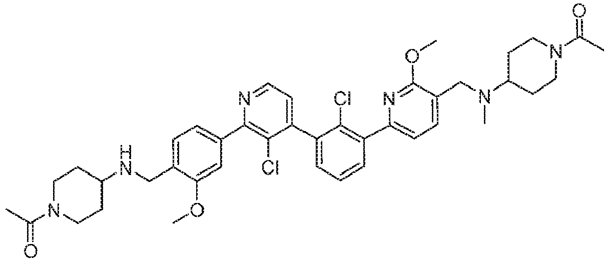

Reductive Amination Procedure B from tert-butyl (1-acetylpiperidin-4-yl)(4-(3-chloro-4-(2-chloro-3-(5-formyl-6-methoxypyridin-2-yl)phenyl)pyridin-2-yl)-2-methoxybenzyl)carbamate (Example 295, step (b)) (50 mg, 0.07 mmol) and 1-[4-(methylamino)-1-piperidyl]ethanone (22 mg, 0.14 mmol) gave tert-butyl N-(1-acetyl-4-piperidyl)-N-[[4-[4-[3-[5-[[(1-acetyl-4-piperidyl)-methyl-amino]methyl]-6-methoxy-2-pyridyl]-2-chloro-phenyl]-3-chloro-2-pyridyl]-2-methoxy-phenyl]methyl]carbamate as crude, LCMS: m/z found 859.3 [M+H]⁺, retention time = 0.84 min (Method B), which was deprotected and purified by General Boc Deprotection Procedure to afford the final product as formic acid salt. 75% yield over two steps. LCMS: m/z found 759.3 [M+H]⁺, retention time = 1.72 min (Method D). ¹H NMR (400 MHz, Methanol-*d*₄): δ 8.66 (d, 1H), 8.46 (s, 2H), 7.82 (d, 1H), 7.73 (d, 1H), 7.59 - 7.51 (m, 2H), 7.49 - 7.46 (m, 1H), 7.44 - 7.38 (m, 2H), 7.35 (d, 1H), 7.31 - 7.26 (m, 1H), 4.71 - 4.55 (m, 2H), 4.30 (s, 2H), 4.14 - 3.97 (m, 8H), 3.84 (s, 2H), 3.26 - 3.10 (m, 4H), 2.77 - 2.58 (m, 2H), 2.43 (s, 3H), 2.29 - 2.17 (m, 2H), 2.17 - 2.10 (m, 6H), 2.08 - 1.96 (m, 2H), 1.78 - 1.43 (m, 4H).

### Example 297: 1-(4-((4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)amino)piperidin-1-yl)-2-methylpropan-1-one (371)

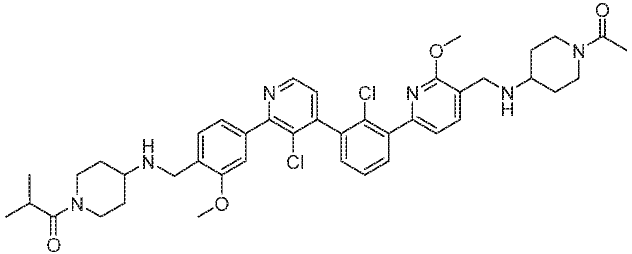

Reductive Amination Procedure B from tert-butyl (1-acetylpiperidin-4-yl)((6-(2-chloro-3-(3-chloro-2-(4-formyl-3-methoxyphenyl)pyridin-4-yl)phenyl)-2-methoxypyridin-3-yl)methyl)carbamate (Example 285, step (b)) (40 mg, 0.06 mmol) and 1-(4-amino-1-piperidyl)-2-methyl-propan-1-one (19 mg, 0.11 mmol) gave tert-butyl N-(1-acetyl-4-piperidyl)-N-[[6-[2-chloro-3-[3-chloro-2-[3-methoxy-4-[[[1-(2-methylpropanoyl)-4-piperidyl]amino]methyl]phenyl]-4-pyridyl]phenyl]-2-methoxy-3-pyridyl]methyl]carbamate as crude, LCMS: m/z found 873.4 [M+H]⁺, retention time = 0.90 min (Method B), which was deprotected and purified by General Boc Deprotection Procedure to afford the final product as formic acid salt. 58% yield over two steps. LCMS: m/z found 773.3 [M+H]⁺, retention time = 1.87 min (Method D). ¹H NMR (400 MHz, Methanol-*d*₄): δ 8.74 - 8.59 (m, 1H), 8.55 - 8.42 (m, 2H), 7.82 (d, 1H), 7.74 - 7.69 (m, 1H), 7.56 (dd, 1H), 7.51 (d, 1H), 7.49 - 7.45 (m, 1H), 7.45 - 7.40 (m, 1H), 7.37 (s, 1H), 7.34 (d, 1H), 7.30 (d, 1H), 4.66 (d, 1H), 4.56 (d, 1H), 4.24 (s, 2H), 4.22 - 4.14 (m, 1H), 4.08 - 3.95 (m, 9H), 3.22 - 3.13 (m, 2H), 3.11 - 3.02 (m, 1H), 2.99 - 2.92 (m, 1H), 2.77 - 2.67 (m, 2H), 2.30 - 2.03 (m, 8H), 1.63 - 1.26 (m, 4H), 1.17 - 1.04 (m, 6H).

### Example 298: 1-(4-(((6-(3-(2-(4-(((1-acetylpiperidin-4-yl)amino)methyl)-3-methoxyphenyl)-3-chloropyridin-4-yl)-2-chlorophenyl)-2-methoxypyridin-3-yl)methyl)amino)piperidin-1-yl)-2-methylpropan-1-one (382)

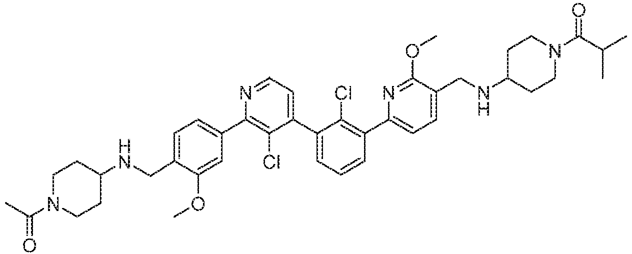

Reductive Amination Procedure B from tert-butyl (1-acetylpiperidin-4-yl)(4-(3-chloro-4-(2-chloro-3-(5-formyl-6-methoxypyridin-2-yl)phenyl)pyridin-2-yl)-2-methoxybenzyl)carbamate (Example 295, step (b)) (40 mg, 0.06 mmol) and 1-(4-amino-1-piperidyl)-2-methyl-propan-1-one (19 mg, 0.11 mmol) gave tert-butyl N-(1-acetyl-4-piperidyl)-N-[[4-[3-chloro-4-[2-chloro-3-[6-methoxy-5-[[[1-(2-methylpropanoyl)-4-piperidyl]amino]methyl]-2-pyridyl]phenyl]-2-pyridyl]-2-methoxy-phenyl]methyl]carbamate as crude, LCMS: m/z found 873.4 [M+H]⁺, which was deprotected and purified by General Boc Deprotection Procedure to afford the final product as formic acid salt. 85% yield over two steps. LCMS: m/z found 773.3 [M+H]⁺, retention time = 1.83 min (Method D). ¹H NMR (400 MHz, Methanol-*d*₄): δ 8.66 (d, 1H), 8.50 (s, 2H), 7.82 (d, 1H), 7.72 (d, 1H), 7.60 - 7.49 (m, 2H), 7.47 (d, 1H), 7.43 (d, 1H), 7.38 (s, 1H), 7.32 (dd, 2H), 4.61 (t, 2H), 4.24 (s, 2H), 4.18 - 4.10 (m, 1H), 4.09 - 4.01 (m, 6H), 3.98 (s, 3H), 3.25 - 3.06 (m, 4H), 3.02 - 2.93 (m, 1H), 2.75 - 2.60 (m, 2H), 2.26 - 2.05 (m, 7H), 1.64 - 1.26 (m, 4H), 1.16 - 1.02 (m, 6H).

### Example 299: (S)-1-((6-(3-(2-(4-(((1-acetylpiperidin-4-yl)amino)methyl)-3-methoxyphenyl)-3-chloropyridin-4-yl)-2-chlorophenyl)-2-methoxypyridin-3-yl)methyl)pyrrolidine-3-carboxylic acid (388)

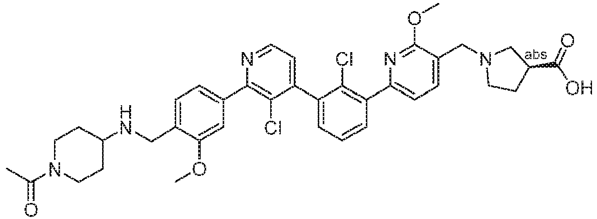

Reductive Amination Procedure B from tert-butyl (1-acetylpiperidin-4-yl)(4-(3-chloro-4-(2-chloro-3-(5-formyl-6-methoxypyridin-2-yl)phenyl)pyridin-2-yl)-2-methoxybenzyl)carbamate (Example 295, step (b)) (40 mg, 0.06 mmol) and (3S)-pyrrolidine-3-carboxylic acid (13 mg, 0.11mmol) gave (3S)-1-[[6-[3-[2-[4-[[(1-acetyl-4-piperidyl)-tert-butoxycarbonyl-amino]methyl]-3-methoxy-phenyl]-3-chloro-4-pyridyl]-2-chloro-phenyl]-2-methoxy-3-pyridyl]methyl]pyrrolidine-3-carboxylic acid as crude, LCMS: m/z found 818.3 [M+H]⁺, which was deprotected and purified by General Boc Deprotection Procedure to afford the final product as formic acid salt. 71% yield over two steps. LCMS: m/z found 718.3 [M+H]⁺, retention time = 1.69 min (Method D). ¹H NMR (400 MHz, Methanol-*d*₄): δ 8.66 (d, 1H), 8.49 (s, 2H), 7.92 (d, 1H), 7.74 (d, 1H), 7.58 (t, 1H), 7.53 (d, 1H), 7.50 - 7.47 (m, 1H), 7.45 (d, 1H), 7.42 - 7.28 (m, 3H), 4.76 - 4.58 (m, 1H), 4.41 (s, 2H), 4.28 (s, 2H), 4.09 (s, 3H), 4.07 - 4.02 (m, 1H), 3.99 (s, 3H), 3.56 (dd, 1H), 3.50 - 3.33 (m, 3H), 3.26 - 3.02 (m, 3H), 2.76 - 2.67 (m, 1H), 2.43 - 2.31 (m, 1H), 2.30 - 2.17 (m, 3H), 2.13 (s, 3H).

### Example 300: 3-(((6-(3-(2-(4-(((1-acetylpiperidin-4-yl)amino)methyl)-3-methoxyphenyl)-3-chloropyridin-4-yl)-2-chlorophenyl)-2-methoxypyridin-3-yl)methyl)amino)propanoic acid (389)

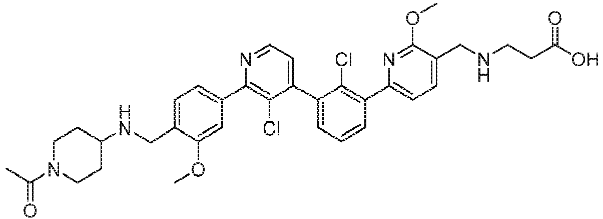

Reductive Amination Procedure B from tert-butyl (1-acetylpiperidin-4-yl)(4-(3-chloro-4-(2-chloro-3-(5-formyl-6-methoxypyridin-2-yl)phenyl)pyridin-2-yl)-2-methoxybenzyl)carbamate (Example 295, step (b)) (40 mg, 0.06 mmol) and 3-aminopropanoic acid (10 mg, 0.11 mmol) gave 3-[[6-[3-[2-[4-[[(1-acetyl-4-piperidyl)-tert-butoxycarbonyl-amino]methyl]-3-methoxy-phenyl]-3-chloro-4-pyridyl]-2-chloro-phenyl]-2-methoxy-3-pyridyl]methylamino]propanoic acid as crude, LCMS: m/z found 792.3 [M+H]⁺, which was deprotected and purified by General Boc Deprotection Procedure to afford the final product as formic acid salt. 78% yield over two steps. LCMS: m/z found 692.2 [M+H]⁺, retention time = 1.68 min (Method D). ¹H NMR (400 MHz, Methanol-*d*₄): δ 8.66 (d, 1H), 8.50 (s, 2H), 7.87 (d, 1H), 7.73 (dd, 1H), 7.58 (t, 1H), 7.53 (d, 1H), 7.48 (d, 1H), 7.44 (dd, 1H), 7.39 (s, 1H), 7.34 (dt, 2H), 4.65 (d, 1H), 4.33 - 4.23 (m, 4H), 4.10 (s, 3H), 4.07 - 4.02 (m, 1H), 3.99 (s, 3H), 3.43 - 3.33 (m, 1H), 3.25 - 3.19 (m, 3H), 2.75 - 2.65 (m, 1H), 2.53 (t, 2H), 2.27 - 2.16 (m, 2H), 2.13 (s, 3H), 1.69 - 1.42 (m, 2H).

### Example 301: 3-((4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)amino)propanoic acid (390)

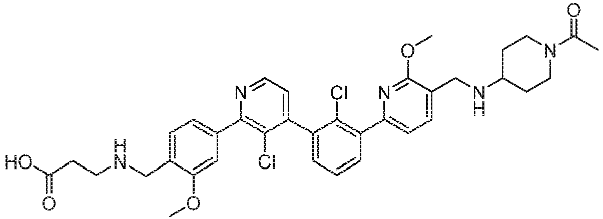

Reductive Amination Procedure B from tert-butyl (1-acetylpiperidin-4-yl)((6-(2-chloro-3-(3-chloro-2-(4-formyl-3-methoxyphenyl)pyridin-4-yl)phenyl)-2-methoxypyridin-3-yl)methyl)carbamate (Example 285, step (b)) (45 mg, 0.06 mmol) and 3-aminopropanoic acid (11 mg, 0.13 mmol) gave 3-[[4-[4-[3-[5-[[(1-acetyl-4-piperidyl)-tert-butoxycarbonylamino]methyl]-6-methoxy-2-pyridyl]-2-chloro-phenyl]-3-chloro-2-pyridyl]-2-methoxyphenyl]methylamino]propanoic acid as crude, LCMS: m/z found 792.3 [M+H]⁺, retention time = 0.87 min (Method B), which was deprotected and purified by General Boc Deprotection Procedure to afford the final product as formic acid salt. 52% yield over two steps. LCMS: m/z found 692.2 [M+H]⁺, retention time = 1.68 min (Method D). ¹H NMR (400 MHz, Methanol-*d*₄): δ 8.65 (d, 1H), 8.44 (s, 2H), 7.86 (d, 1H), 7.71 (d, 1H), 7.57 (t, 1H), 7.50 (d, 1H), 7.45 (t, 2H), 7.38 (s, 1H), 7.36 - 7.29 (m, 2H), 4.62 (d, 1H), 4.27 (s, 2H), 4.19 (s, 2H), 4.10 - 3.93 (m, 7H), 3.26 - 3.09 (m, 4H), 2.77 - 2.66 (m, 1H), 2.53 (t, 2H), 2.24 - 2.14 (m, 2H), 2.12 (s, 3H), 1.63 - 1.39 (m, 2H).

### Example 302: 1-(4-((4-(3-chloro-4-(2-chloro-3-(6-methoxy-5-(((tetrahydro-2H-pyran-4-yl)amino)methyl)pyridin-2-yl)phenyl)pyridin-2-yl)-2-methoxybenzyl)amino)piperidin-1-yl)ethan-1-one (391)

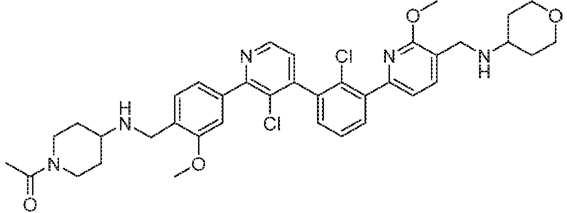

Reductive Amination Procedure B from tert-butyl (1-acetylpiperidin-4-yl)(4-(3-chloro-4-(2-chloro-3-(5-formyl-6-methoxypyridin-2-yl)phenyl)pyridin-2-yl)-2-methoxybenzyl)carbamate (Example 295, step (b)) (40 mg, 0.06 mmol) and tetrahydropyran-4-amine (11 mg, 0.11 mmol) gave tert-butyl N-(1-acetyl-4-piperidyl)-N-[[4-[3-chloro-4-[2-chloro-3-[6-methoxy-5-[(tetrahydropyran-4-ylamino)methyl]-2-pyridyl]phenyl]-2-pyridyl]-2-methoxy-phenyl]methyl]carbamate as crude, LCMS: m/z found 804.3 [M+H]⁺, which was deprotected and purified by General Boc Deprotection Procedure to afford the final product. 64% yield over two steps. LCMS: m/z found 704.3 [M+H]⁺, retention time = 1.76 min (Method D). ¹H NMR (400 MHz, Methanol-*d*₄): δ 8.68 (d, 1H), 7.91 (d, 1H), 7.73 (d, 1H), 7.59 (t, 1H), 7.55 (d, 1H), 7.50 (d, 1H), 7.48 - 7.44 (m, 1H), 7.41 (s, 1H), 7.39 - 7.34 (m, 2H), 4.69 (d, 1H), 4.35 (s, 2H), 4.31 (s, 2H), 4.14 - 4.02 (m, 6H), 4.00 (s, 3H), 3.56 - 3.40 (m, 4H), 3.22 (t, 1H), 2.77 - 2.66 (m, 1H), 2.33 - 2.19 (m, 2H), 2.16 - 2.06 (m, 5H), 1.83 - 1.46 (m, 4H).

### Example 303: 1-(4-(((6-(2-chloro-3-(3-chloro-2-(3-methoxy-4-(((tetrahydro-2H-pyran-4-yl)amino)methyl)phenyl)pyridin-4-yl)phenyl)-2-methoxypyridin-3-yl)methyl)amino)piperidin-1-yl)ethan-1-one (392)

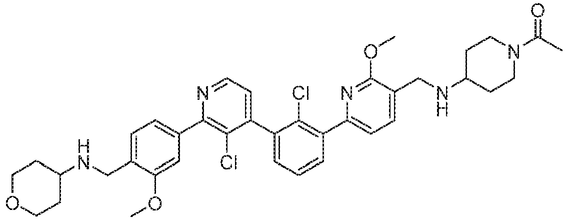

Reductive Amination Procedure B from tert-butyl (1-acetylpiperidin-4-yl)((6-(2-chloro-3-(3-chloro-2-(4-formyl-3-methoxyphenyl)pyridin-4-yl)phenyl)-2-methoxypyridin-3-yl)methyl)carbamate (Example 285, step (b)) (40 mg, 0.06 mmol) and tetrahydropyran-4-amine (11 mg, 0.11 mmol) gave tert-butyl N-(1-acetyl-4-piperidyl)-N-[[6-[2-chloro-3-[3-chloro-2-[3-methoxy-4-[(tetrahydropyran-4-ylamino)methyl]phenyl]-4-pyridyl]phenyl]-2-methoxy-3-pyridyl]methyl]carbamate as crude, LCMS: m/z found 804.3 [M+H]⁺, which was deprotected and purified by General Boc Deprotection Procedure to afford the final product as formic acid salt. 70% yield over two steps. LCMS: m/z found 704.3 [M+H]⁺, retention time = 2.79 min (Method A). ¹H NMR (400 MHz, Methanol-*d*₄): δ 8.70 - 8.60 (m, 1H), 8.52 (s, 2H), 7.80 (d, 1H), 7.74 - 7.67 (m, 1H), 7.56 (t, 1H), 7.53 - 7.49 (m, 1H), 7.49 - 7.45 (m, 1H), 7.42 (d, 1H), 7.38 (s, 1H), 7.34 (d, 1H), 7.29 (dd, 1H), 4.53 (d, 1H), 4.25 (s, 2H), 4.09 - 3.90 (m, 12H), 3.54 - 3.38 (m, 2H), 3.21 - 3.09 (m, 1H), 3.04 - 2.92 (m, 1H), 2.71 (t, 1H), 2.22 - 1.96 (m, 7H), 1.78 - 1.62 (m, 2H), 1.55 - 1.22 (m, 2H).

### Example 304: 1-(4-((4-(3-chloro-4-(2-chloro-3-(6-methoxy-5-((methyl(tetrahydro-2H-pyran-4-yl)amino)methyl)pyridin-2-yl)phenyl)pyridin-2-yl)-2-methoxybenzyl)amino)piperidin-1-yl)ethan-1-one (436)

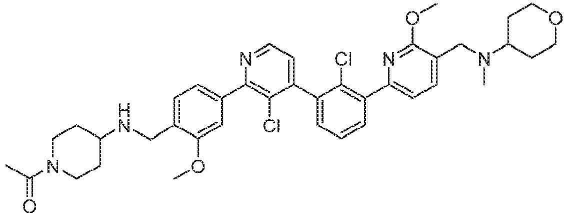

Reductive Amination Procedure B from tert-butyl (1-acetylpiperidin-4-yl)(4-(3-chloro-4-(2-chloro-3-(5-formyl-6-methoxypyridin-2-yl)phenyl)pyridin-2-yl)-2-methoxybenzyl)carbamate (Example 295, step (b)) (50 mg, 0.07 mmol) and N-methyltetrahydropyran-4-amine (16 mg, 0.14 mmol) gave tert-butyl N-(1-acetyl-4-piperidyl)-N-[[4-[3-chloro-4-[2-chloro-3-[6-methoxy-5-[[methyl(tetrahydropyran-4-yl)amino]methyl]-2-pyridyl]phenyl]-2-pyridyl]-2-methoxy-phenyl]methyl]carbamate as crude, LCMS: m/z found 818.3 [M+H]⁺, which was deprotected and purified by General Boc Deprotection Procedure to afford the final product. 65% yield over two steps. LCMS: m/z found 718.3 [M+H]⁺, retention time = 1.68 min (Method D). ¹H NMR (400 MHz, Methanol-*d*₄): δ 8.66 (dt, 1H), 8.51 (s, 2H), 7.83 (d, 1H), 7.73 (d, 1H), 7.61 - 7.49 (m, 2H), 7.47 (dd, 1H), 7.42 (d, 1H), 7.38 (s, 1H), 7.37 - 7.25 (m, 2H), 4.71 - 4.59 (m, 1H), 4.26 (s, 2H), 4.10 - 4.00 (m, 6H), 4.02 - 3.96 (m, 3H), 3.95 - 3.86 (m, 2H), 3.45 (t, 2H), 3.20 (t, 1H), 3.07 - 2.94 (m, 1H), 2.70 (t, 1H), 2.48 (s, 3H), 2.28 - 2.15 (m, 2H), 2.13 (s, 3H), 1.99 - 1.89 (m, 2H), 1.85 - 1.71 (m, 2H), 1.69 - 1.42 (m, 2H).

### Example 305: N-(4-(3-chloro-4-(2-chloro-3-(6-methoxy-5-(((tetrahydro-2H-pyran-4-yl)amino)methyl)pyridin-2-yl)phenyl)pyridin-2-yl)-2-methoxybenzyl)-N-methyltetrahydro-2H-pyran-4-amine (441)

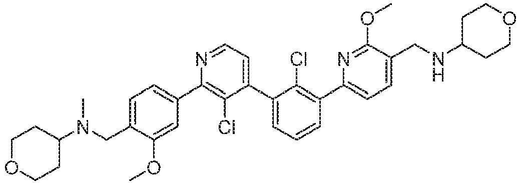

### (a) tert-Butyl ((6-(2-chloro-3-(2,3-dichloropyridin-4-yl)phenyl)-2-methoxypyridin-3-yl)methyl)(tetrahydro-2H-pyran-4-yl)carbamate

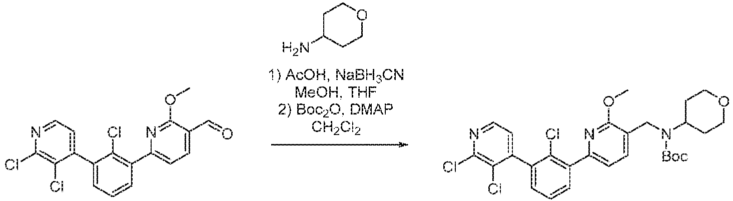

Reductive Amination Procedure B from 6-[2-chloro-3-(2,3-dichloro-4-pyridyl)phenyl]-2-methoxy-pyridine-3-carbaldehyde (220 mg, 0.56 mmol) and tetrahydropyran-4-amine (113 mg, 1.12 mmol) gave N-[[6-[2-chloro-3-(2,3-dichloro-4-pyridyl)phenyl]-2-methoxy-3-pyridyl]methyl]tetrahydropyran-4-amine, which was used in the next step without further purification. LCMS: m/z found 478.1 [M+H]⁺, retention time = 0.80 min (Method B).

To a solution of crude N-[[6-[2-chloro-3-(2,3-dichloro-4-pyridyl)phenyl]-2-methoxy-3-pyridyl]methyl]tetrahydropyran-4-amine (267 mg, 0.56 mmol) in DCM was added di-tert-butyl dicarbonate (153 mg, 0.70 mmol) and DMAP (7 mg, 0.06 mmol). The mixture was stirred for 2 hours, concentrated in vacuo, and purified on silica gel column using MeOH in DCM (0 to 10% gradient) as eluent to give tert-butyl ((6-(2-chloro-3-(2,3-dichloropyridin-4-yl)phenyl)-2-methoxypyridin-3-yl)methyl)(tetrahydro-2H-pyran-4-yl)carbamate (210 mg, 0.36 mmol, 65%). LCMS: m/z found 578.1 [M+H]⁺, retention time = 1.23 min (Method B).

### (b) tert-Butyl ((6-(2-chloro-3-(3-chloro-2-(4-formyl-3-methoxyphenyl)pyridin-4-yl)phenyl)-2-methoxypyridin-3-yl)methyl)(tetrahydro-2H-pyran-4-yl)carbamate

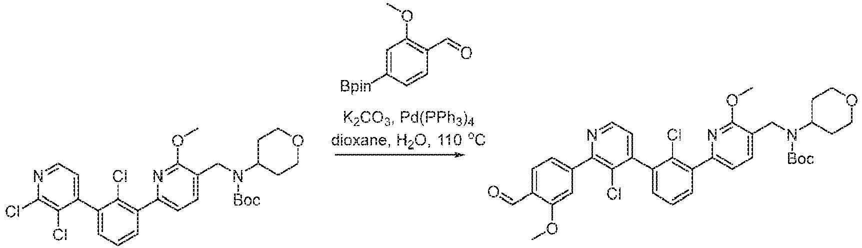

To a mixture of tert-butyl ((6-(2-chloro-3-(2,3-dichloropyridin-4-yl)phenyl)-2-methoxypyridin-3-yl)methyl)(tetrahydro-2H-pyran-4-yl)carbamate (300 mg, 0.52 mmol), 2-methoxy-4-(4,4,5,5-tetramethyl-1,3,2-dioxaborolan-2-yl)benzaldehyde (136 mg, 0.52 mmol) and potassium carbonate (215 mg, 1.55 mmol) in degassed dioxane/water (6:1) was added tetrakis(triphenylphosphine)palladium(0) (60 mg, 0.05 mmol) and the mixture was stirred at 105 °C for 30 min. Water was added and the mixture was extracted into EtOAc three times. The combined organic layers were dried over anhydrous sodium sulfate, concentrated, and purified on silica gel column using 2% MeOH in DCM as eluent to provide tert-butyl ((6-(2-chloro-3-(3-chloro-2-(4-formyl-3-methoxyphenyl)pyridin-4-yl)phenyl)-2-methoxypyridin-3-yl)methyl)(tetrahydro-2H-pyran-4-yl)carbamate (330 mg, 0.49 mmol, 94%). LCMS m/z found 678.2 [M+H]⁺, retention time = 1.20 min (Method B).

### (c) N-(4-(3-chloro-4-(2-chloro-3-(6-methoxy-5-(((tetrahydro-2H-pyran-4-yl)amino)methyl)pyridin-2-yl)phenyl)pyridin-2-yl)-2-methoxybenzyl)-N-methyltetrahydro-2H-pyran-4-amine

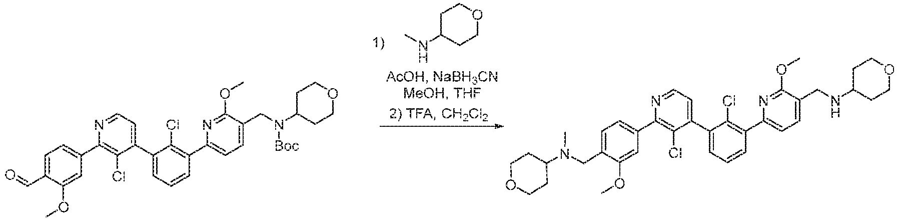

Reductive Amination Procedure B from tert-butyl ((6-(2-chloro-3-(3-chloro-2-(4-formyl-3-methoxyphenyl)pyridin-4-yl)phenyl)-2-methoxypyridin-3-yl)methyl)(tetrahydro-2H-pyran-4-yl)carbamate (50 mg, 0.07 mmol) and N-methyltetrahydropyran-4-amine (17 mg, 0.15 mmol) gave tert-butyl N-[[6-[2-chloro-3-[3-chloro-2-[3-methoxy-4-[[methyl(tetrahydropyran-4-yl)amino]methyl]phenyl]-4-pyridyl]phenyl]-2-methoxy-3-pyridyl]methyl]-N-tetrahydropyran-4-yl-carbamate as crude, LCMS: m/z found 777.3 [M+H]⁺, retention time = 0.95 min (Method B), which was deprotected and purified by General Boc Deprotection Procedure to afford the final product as formic acid salt. 47% yield over two steps. LCMS: m/z found 677.3 [M+H]⁺, retention time = 1.68 min (Method D). ¹H NMR (400 MHz, Methanol-*d*₄): δ 8.71 - 8.65 (m, 1H), 8.47 (s, 2H), 7.90 (d, 1H), 7.75 - 7.70 (m, 1H), 7.61 - 7.54 (m, 2H), 7.51 - 7.41 (m, 3H), 7.41 - 7.31 (m, 2H), 4.42 - 4.28 (m, 2H), 4.26 (s, 2H), 4.16 - 4.02 (m, 7H), 4.00 (s, 3H), 3.58 - 3.34 (m, 6H), 2.77 (s, 3H), 2.16 - 2.06 (m, 4H), 2.02 - 1.85 (m, 2H), 1.81 - 1.62 (m, 2H).

### Example 306: 1-(4-((4-(3-chloro-4-(2-chloro-3-(6-methoxy-5-(((tetrahydro-2H-pyran-4-yl)amino)methyl)pyridin-2-yl)phenyl)pyridin-2-yl)-2-methoxybenzyl)(methyl)amino)piperidin-1-yl)ethan-1-one (442)

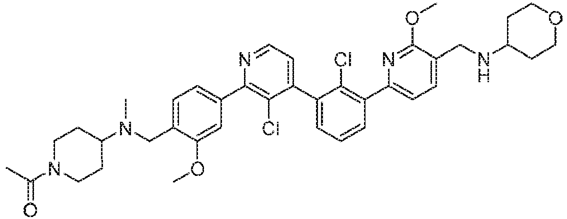

Reductive Amination Procedure B from tert-butyl ((6-(2-chloro-3-(3-chloro-2-(4-formyl-3-methoxyphenyl)pyridin-4-yl)phenyl)-2-methoxypyridin-3-yl)methyl)(tetrahydro-2H-pyran-4-yl)carbamate (Example 305, step (b)) (50 mg, 0.07 mmol) and 1-[4-(methylamino)-1-piperidyl]ethanone (23 mg, 0.15 mmol) gave tert-butyl N-[[6-[3-[2-[4-[[(1-acetyl-4-piperidyl)-methyl-amino]methyl]-3-methoxy-phenyl]-3-chloro-4-pyridyl]-2-chlorophenyl]-2-methoxy-3-pyridyl]methyl]-N-tetrahydropyran-4-yl-carbamate as crude, LCMS: m/z found 818.3 [M+H]⁺, which was deprotected and purified by General Boc Deprotection Procedure to afford the final product as formic acid salt. 47% yield over two steps. LCMS: m/z found 718.3 [M+H]⁺, retention time = 1.61 min (Method D). ¹H NMR (400 MHz, Methanol-*d*₄): δ 8.67 (dt, 1H), 8.48 (s, 2H), 7.89 (d, 1H), 7.72 (dq, 1H), 7.62 - 7.52 (m, 2H), 7.50 - 7.43 (m, 2H), 7.40 (d, 1H), 7.36 (ddd, 2H), 4.74 (d, 1H), 4.33 - 4.20 (m, 4H), 4.17 - 4.01 (m, 6H), 4.01 - 3.90 (m, 3H), 3.53 - 3.34 (m, 4H), 3.20 (t, 1H), 2.78 - 2.58 (m, 4H), 2.26 - 2.05 (m, 7H), 1.95 - 1.79 (m, 1H), 1.79 - 1.59 (m, 3H).

### Example 307: N-((6-(2-chloro-3-(3-chloro-2-(3-methoxy-4-(((tetrahydro-2H-pyran-4-yl)amino)methyl)phenyl)pyridin-4-yl)phenyl)-2-methoxypyridin-3-yl)methyl)-N-methyltetrahydro-2H-pyran-4-amine (468)

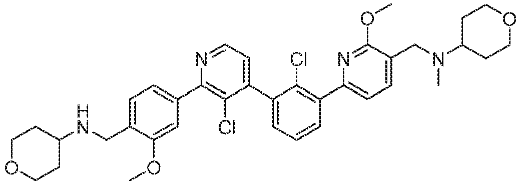

### (a) tert-Butyl (2-methoxy-4-(4,4,5,5-tetramethyl-1,3,2-dioxaborolan-2-yl)benzyl)(tetrahydro-2H-pyran-4-yl)carbamate

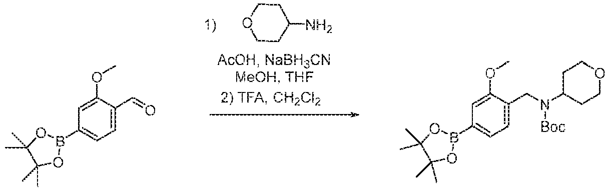

Reductive Amination Procedure B from 2-methoxy-4-(4,4,5,5-tetramethyl-1,3,2-dioxaborolan-2-yl)benzaldehyde (300 mg, 1.14 mmol) and tetrahydropyran-4-amine (232 mg, 2.29 mmol) gave N-(2-methoxy-4-(4,4,5,5-tetramethyl-1,3,2-dioxaborolan-2-yl)benzyl)tetrahydro-2H-pyran-4-amine, which was used in the next step without further purification. LCMS: m/z found 348.2 [M+H]⁺, retention time = 0.69 min (Method B).

To a solution of crude N-(2-methoxy-4-(4,4,5,5-tetramethyl-1,3,2-dioxaborolan-2-yl)benzyl)tetrahydro-2H-pyran-4-amine in DCM was added di-tert-butyl dicarbonate (248 mg, 1.14 mmol) and DMAP (28 mg, 0.23 mmol). The mixture was stirred for 2 hours, concentrated in vacuo, and purified on silica gel column using MeOH in DCM (0 to 10% gradient) as eluent to give tert-butyl (2-methoxy-4-(4,4,5,5-tetramethyl-1,3,2-dioxaborolan-2-yl)benzyl)(tetrahydro-2H-pyran-4-yl)carbamate (330 mg, 0.74 mmol, 65% over two steps). LCMS: m/z found 448.3 [M+H]⁺, retention time = 1.15 min (Method B).

### (b) tert-Butyl (4-(3-chloro-4-(2-chloro-3-(5-formyl-6-methoxypyridin-2-yl)phenyl)pyridin-2-yl)-2-methoxybenzyl)(tetrahydro-2H-pyran-4-yl)carbamate

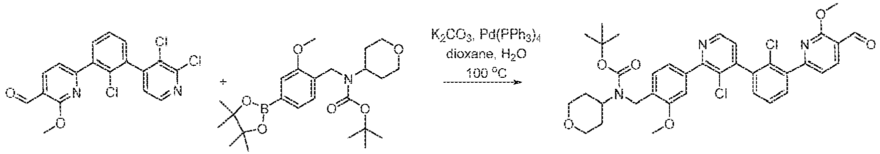

To a mixture of 6-[2-chloro-3-(2,3-dichloro-4-pyridyl)phenyl]-2-methoxy-pyridine-3-carbaldehyde (250 mg, 0.64 mmol), tert-butyl (2-methoxy-4-(4,4,5,5-tetramethyl-1,3,2-dioxaborolan-2-yl)benzyl)(tetrahydro-2H-pyran-4-yl)carbamate (284 mg, 0.64 mmol) and potassium carbonate (263 mg, 1.91 mmol) in degassed dioxane/water (6: 1) was added tetrakis(triphenylphosphine)palladium(0) (73 mg, 0.06 mmol) and the mixture was stirred at 100 °C for 30 min. Water was added and the mixture was extracted into EtOAc three times. The combined organic layers were dried over anhydrous sodium sulfate, concentrated, and purified on silica gel column using 2% MeOH in DCM as eluent to provide tert-butyl (4-(3-chloro-4-(2-chloro-3-(5-formyl-6-methoxypyridin-2-yl)phenyl)pyridin-2-yl)-2-methoxybenzyl)(tetrahydro-2H-pyran-4-yl)carbamate (350 mg, 81%). LCMS: m/z found 678.2 [M+H]⁺, retention time = 1.18 min (Method B).

### (c) N-((6-(2-chloro-3-(3-chloro-2-(3-methoxy-4-(((tetrahydro-2H-pyran-4-yl)amino)methyl)phenyl)pyridin-4-yl)phenyl)-2-methoxypyridin-3-yl)methyl)-N-methyltetrahydro-2H-pyran-4-amine

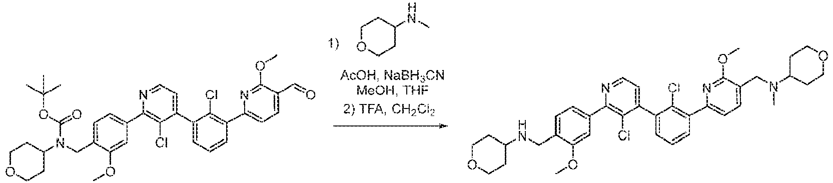

Reductive Amination Procedure B from tert-butyl (4-(3-chloro-4-(2-chloro-3-(5-formyl-6-methoxypyridin-2-yl)phenyl)pyridin-2-yl)-2-methoxybenzyl)(tetrahydro-2H-pyran-4-yl)carbamate (50 mg, 0.07 mmol), N-methyltetrahydropyran-4-amine (17 mg, 0.15 mmol) gave tert-butyl N-[[4-[3-chloro-4-[2-chloro-3-[6-methoxy-5-[[methyl(tetrahydropyran-4-yl)amino]methyl]-2-pyridyl]phenyl]-2-pyridyl]-2-methoxyphenyl]methyl]-N-tetrahydropyran-4-yl-carbamate as crude, LCMS: m/z found 777.3 [M+H]⁺, which was deprotected and purified by General Boc Deprotection Procedure to afford the final product as formic acid salt. 61% yield over two steps. LCMS: m/z found 677.3 [M+H]⁺, retention time = 1.72 min (Method D). ¹H NMR (400 MHz, Methanol-*d*₄): δ 8.69 - 8.62 (m, 1H), 8.51 (s, 2H), 7.83 (d, 1H), 7.73 (d, 1H), 7.55 (dd, 2H), 7.48 (dd, 1H), 7.42 (d, 1H), 7.39 (s, 1H), 7.35 (d, 1H), 7.30 (d, 1H), 4.28 (s, 2H), 4.16 - 3.96 (m, 10H), 3.88 (s, 2H), 3.51 - 3.35 (m, 5H), 3.05 - 2.89 (m, 1H), 2.45 (s, 3H), 2.11 (d, 2H), 1.94 (d, 2H), 1.83 - 1.64 (m, 4H).

### Example 308: 1-(4-(((6-(2-chloro-3-(3-chloro-2-(3-methoxy-4-(((tetrahydro-2H-pyran-4-yl)amino)methyl)phenyl)pyridin-4-yl)phenyl)-2-methoxypyridin-3-yl)methyl)(methyl)amino)piperidin-1-yl)ethan-1-one (469)

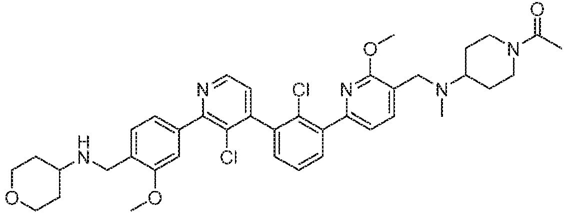

Reductive Amination Procedure B from tert-butyl (4-(3-chloro-4-(2-chloro-3-(5-formyl-6-methoxypyridin-2-yl)phenyl)pyridin-2-yl)-2-methoxybenzyl)(tetrahydro-2H-pyran-4-yl)carbamate (Example 307, step (b)) (50 mg, 0.07 mmol) and 1-[4-(methylamino)-1-piperidyl]ethanone (23 mg, 0.15 mmol) gave tert-butyl N-[[4-[4-[3-[5-[[(1-acetyl-4-piperidyl)-methyl-amino]methyl]-6-methoxy-2-pyridyl]-2-chloro-phenyl]-3-chloro-2-pyridyl]-2-methoxy-phenyl]methyl]-N-tetrahydropyran-4-yl-carbamate as crude, LCMS: m/z found 818.3 [M+H]⁺, which was deprotected and purified by General Boc Deprotection Procedure to afford the final product as formic acid salt. 62% yield over two steps. LCMS: m/z found 718.3 [M+H]⁺, retention time = 1.67 min (Method D). ¹H NMR (400 MHz, Methanol-*d*₄): δ 8.69 - 8.62 (m, 1H), 8.53 (s, 2H), 7.79 (d, 1H), 7.72 (d, 1H), 7.59 - 7.49 (m, 2H), 7.47 (d, 1H), 7.41 (d, 1H), 7.38 (s, 1H), 7.34 (d, 1H), 7.29 - 7.22 (m, 1H), 4.61 (d, 1H), 4.24 (s, 2H), 4.14 - 3.90 (m, 9H), 3.71 (s, 2H), 3.45 (t, 2H), 3.36 - 3.33 (m, 1H), 3.13 (t, 1H), 2.82 (t, 1H), 2.70 - 2.57 (m, 1H), 2.34 (s, 3H), 2.15 - 2.04 (m, 5H), 1.99 (t, 2H), 1.77 - 1.45 (m, 4H).

### Example 309: 1-(4-(((6-(2-chloro-3-(3-chloro-2-(3-methoxy-4-((methyl(tetrahydro-2H-pyran-4-yl)amino)methyl)phenyl)pyridin-4-yl)phenyl)-2-methoxypyridin-3-yl)methyl)amino)piperidin-1-yl)ethan-1-one (427)

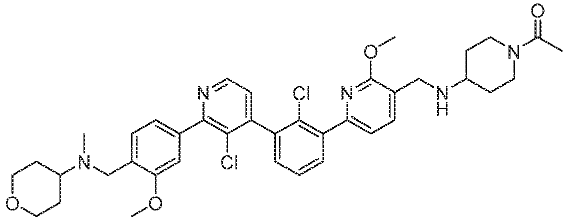

Reductive Amination Procedure B from tert-butyl (1-acetylpiperidin-4-yl)((6-(2-chloro-3-(3-chloro-2-(4-formyl-3-methoxyphenyl)pyridin-4-yl)phenyl)-2-methoxypyridin-3-yl)methyl)carbamate (Example 285, step (b)) (50 mg, 0.07 mmol) and N-methyltetrahydropyran-4-amine (16 mg, 0.14 mmol) gave tert-butyl N-(1-acetyl-4-piperidyl)-N-[[6-[2-chloro-3-[3-chloro-2-[3-methoxy-4-[[methyl(tetrahydropyran-4-yl)amino]methyl]phenyl]-4-pyridyl]phenyl]-2-methoxy-3-pyridyl]methyl]carbamate as crude, LCMS: m/z found 818.3 [M+H]⁺, which was deprotected and purified by General Boc Deprotection Procedure to afford the final product as formic acid salt. 72% yield over two steps. LCMS: m/z found 718.3 [M+H]⁺, retention time = 2.64 min (Method A). ¹H NMR (400 MHz, Methanol-*d*₄): δ 8.63 (d, 1H), 8.54 (s, 2H), 7.77 (d, 1H), 7.71 (d, 1H), 7.55 (t, 1H), 7.50 - 7.43 (m, 2H), 7.41 (d, 1H), 7.34 - 7.22 (m, 3H), 4.49 (d, 1H), 4.09 - 4.00 (m, 5H), 3.99 - 3.85 (m, 8H), 3.43 (t, 2H), 3.15 (t, 1H), 2.95 (s, 1H), 2.89 - 2.79 (m, 1H), 2.76 - 2.65 (m, 1H), 2.43 (s, 3H), 2.11 (s, 3H), 2.09 - 1.98 (m, 2H), 1.94 (d, 2H), 1.85 - 1.67 (m, 2H), 1.49 - 1.22 (m, 2H).

### Example 310: 1-(4-(((6-(3-(2-(4-((6-acetyl-2,6-diazaspiro[3.3]heptan-2-yl)methyl)-3-methoxyphenyl)-3-chloropyridin-4-yl)-2-chlorophenyl)-2-methoxypyridin-3-yl)methyl)amino)piperidin-1-yl)ethan-1-one (482)

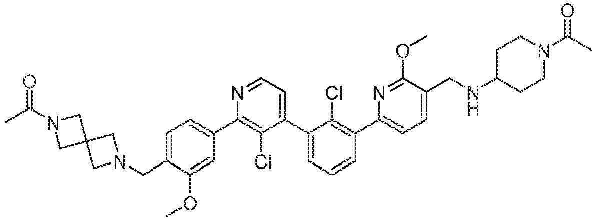

Reductive Amination Procedure B from tert-butyl (1-acetylpiperidin-4-yl)((6-(2-chloro-3-(3-chloro-2-(4-formyl-3-methoxyphenyl)pyridin-4-yl)phenyl)-2-methoxypyridin-3-yl)methyl)carbamate (Example 285, step (b)) (50 mg, 0.07 mmol) and 1-(2,6-diazaspiro[3.3]heptan-2-yl)ethanone (19 mg, 0.14 mmol) gave tert-butyl N-[[6-[3-[2-[4-[(2-acetyl-2,6-diazaspiro[3.3]heptan-6-yl)methyl]-3-methoxy-phenyl]-3-chloro-4-pyridyl]-2-chloro-phenyl]-2-methoxy-3-pyridyl]methyl]-N-(1-acetyl-4-piperidyl)carbamate as crude, LCMS: m/z found 843.3 [M+H]⁺, retention time = 0.86 min (Method B), which was deprotected and purified by General Boc Deprotection Procedure to afford the final product as formic acid salt. 68% yield over two steps. LCMS: m/z found 743.3 [M+H]⁺, retention time = 1.68 min (Method D). ¹H NMR (400 MHz, Methanol-*d*₄): δ 8.63 (d, 1H), 8.54 (s, 2H), 7.81 (d, 1H), 7.71 (d, 1H), 7.56 (t, 1H), 7.48 - 7.37 (m, 3H), 7.33 - 7.22 (m, 3H), 4.55 (d, 1H), 4.31 (s, 2H), 4.15 - 3.86 (m, 13H), 3.81 - 3.66 (m, 4H), 3.17 (t, 1H), 3.08 - 2.94 (m, 1H), 2.71 (t, 1H), 2.22 - 1.98 (m, 5H), 1.84 (s, 3H), 1.57 - 1.29 (m, 2H).

### Example 311: 1-(4-((4-(4-(3-(5-((6-acetyl-2,6-diazaspiro[3.3]heptan-2-yl)methyl)-6-methoxypyridin-2-yl)-2-chlorophenyl)-3-chloropyridin-2-yl)-2-methoxybenzyl)amino)piperidin-1-yl)ethan-1-one (483)

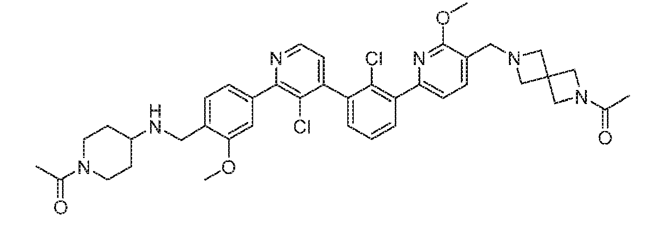

Reductive Amination Procedure B from tert-butyl (1-acetylpiperidin-4-yl)(4-(3-chloro-4-(2-chloro-3-(5-formyl-6-methoxypyridin-2-yl)phenyl)pyridin-2-yl)-2-methoxybenzyl)carbamate (Example 295, step (b)) (50 mg, 0.07 mmol) and 1-(2,6-diazaspiro[3.3]heptan-2-yl)ethanone (19 mg, 0.14 mmol) gave tert-butyl N-[[4-[4-[3-[5-[(2-acetyl-2,6-diazaspiro[3.3]heptan-6-yl)methyl]-6-methoxy-2-pyridyl]-2-chloro-phenyl]-3-chloro-2-pyridyl]-2-methoxy-phenyl]methyl]-N-(1-acetyl-4-piperidyl)carbamate as crude, LCMS: m/z found 843.3 [M+H]⁺, which was deprotected and purified by General Boc Deprotection Procedure to afford the final product as formic acid salt. 66% yield over two steps. LCMS: m/z found 743.3 [M+H]⁺, retention time = 1.68 min (Method D). ¹H NMR (400 MHz, Methanol-*d*₄): δ 8.65 (dd, 1H), 8.52 (s, 2H), 7.70 (dd, 2H), 7.59 - 7.49 (m, 2H), 7.47 (dd, 1H), 7.41 (d, 1H), 7.37 (s, 1H), 7.34 (d, 1H), 7.25 (dd, 1H), 4.63 (d, 1H), 4.31 (s, 2H), 4.23 (s, 2H), 4.11 - 3.93 (m, 9H), 3.74 (s, 2H), 3.66 - 3.51 (m, 4H), 3.27 - 3.09 (m, 2H), 2.78 - 2.60 (m, 1H), 2.28 - 2.06 (m, 5H), 1.85 (s, 3H), 1.69 - 1.38 (m, 2H).

### Example 312: 1-(6-(4-(3-chloro-4-(2-chloro-3-(6-methoxy-5-(((tetrahydro-2H-pyran-4-yl)amino)methyl)pyridin-2-yl)phenyl)pyridin-2-yl)-2-methoxybenzyl)-2,6-diazaspiro[3.3]heptan-2-yl)ethan-1-one (492)

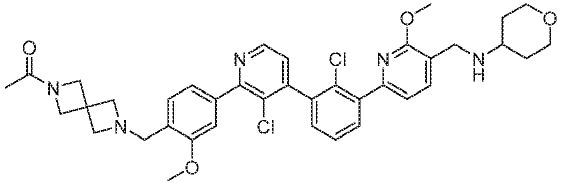

Reductive Amination Procedure B from tert-butyl ((6-(2-chloro-3-(3-chloro-2-(4-formyl-3-methoxyphenyl)pyridin-4-yl)phenyl)-2-methoxypyridin-3-yl)methyl)(tetrahydro-2H-pyran-4-yl)carbamate (Example 305, step (b)) (50 mg, 0.07 mmol) and 1-(2,6-diazaspiro[3.3]heptan-2-yl)ethanone (21 mg, 0.15 mmol) gave tert-butyl N-[[6-[3-[2-[4-[(2-acetyl-2,6-diazaspiro[3.3]heptan-6-yl)methyl]-3-methoxy-phenyl]-3-chloro-4-pyridyl]-2-chloro-phenyl]-2-methoxy-3-pyridyl]methyl]-N-tetrahydropyran-4-yl-carbamate as crude, LCMS: m/z found 802.3 [M+H]⁺, which was deprotected and purified by General Boc Deprotection Procedure to afford the final product as formic acid salt. 38% yield over two steps. LCMS: m/z found 702.3 [M+H]⁺, retention time = 1.75 min (Method D). ¹H NMR (400 MHz, Methanol-*d*₄): δ 8.61 (d, 1H), 8.54 (s, 2H), 7.76 (d, 1H), 7.70 (d, 1H), 7.54 (t, 1H), 7.46 - 7.32 (m, 3H), 7.30 - 7.22 (m, 3H), 4.28 (s, 2H), 4.06 - 4.00 (m, 5H), 4.00 - 3.93 (m, 2H), 3.93 - 3.86 (m, 5H), 3.75 (s, 2H), 3.57 - 3.36 (m, 6H), 2.90 - 2.77 (m, 1H), 1.99 - 1.90 (m, 2H), 1.83 (s, 3H), 1.57 - 1.42 (m, 2H).

### Example 313: 2-(4-(3-Chloro-4-(2-chloro-3-(6-methoxy-5-(((tetrahydro-2H-pyran-4-yl)amino)methyl)pyridin-2-yl)phenyl)pyridin-2-yl)-2-methoxybenzyl)-2,6-diazaspiro[3.4]octan-7-one (506)

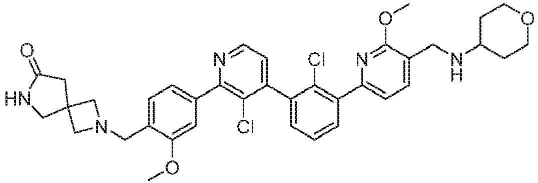

Reductive Amination Procedure B from tert-butyl ((6-(2-chloro-3-(3-chloro-2-(4-formyl-3-methoxyphenyl)pyridin-4-yl)phenyl)-2-methoxypyridin-3-yl)methyl)(tetrahydro-2H-pyran-4-yl)carbamate (Example 305, step (b)) (50 mg, 0.07 mmol) and 2,6-diazaspiro[3.4]octan-7-one (19 mg, 0.15 mmol) gave tert-butyl N-[[6-[2-chloro-3-[3-chloro-2-[3-methoxy-4-[(6-oxo-2,7-diazaspiro[3.4]octan-2-yl)methyl]phenyl]-4-pyridyl]phenyl]-2-methoxy-3-pyridyl]methyl]-N-tetrahydropyran-4-yl-carbamate as crude, LCMS: m/z found 788.3 [M+H]⁺, which was deprotected and purified by General Boc Deprotection Procedure to afford the final product as formic acid salt. 70% yield over two steps. LCMS: m/z found 688.2 [M+H]⁺, retention time = 1.69 min (Method D). ¹H NMR (400 MHz, Methanol-*d*₄): δ 8.65 (d, 1H), 8.49 (s, 2H), 7.89 (d, 1H), 7.72 (d, 1H), 7.58 (t, 1H), 7.50 - 7.40 (m, 3H), 7.38 - 7.28 (m, 3H), 4.25 (s, 2H), 4.21 (s, 2H), 4.11 - 4.06 (m, 4H), 4.05 - 4.01 (m, 1H), 3.98 - 3.87 (m, 7H), 3.64 (s, 2H), 3.47 (t, 2H), 3.42 - 3.34 (m, 1H), 2.71 - 2.62 (m, 2H), 2.11 (dd, 2H), 1.80 - 1.62 (m, 2H).

### Example 314: 1-(6-((6-(2-Chloro-3-(3-chloro-2-(3-methoxy-4-(((tetrahydro-2H-pyran-4-yl)amino)methyl)phenyl)pyridin-4-yl)phenyl)-2-methoxypyridin-3-yl)methyl)-2,6-diazaspiro[3.3]heptan-2-yl)ethan-1-one (511)

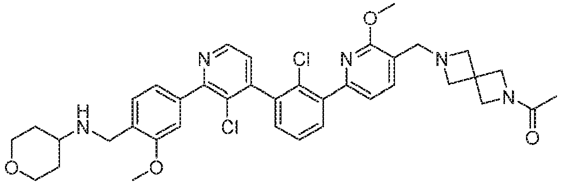

Reductive Amination Procedure B from tert-butyl (4-(3-chloro-4-(2-chloro-3-(5-formyl-6-methoxypyridin-2-yl)phenyl)pyridin-2-yl)-2-methoxybenzyl)(tetrahydro-2H-pyran-4-yl)carbamate (Example 307, step (b)) (50 mg, 0.07 mmol) and 1-(2,6-diazaspiro[3.3]heptan-2-yl)ethanone (21 mg, 0.15 mmol) gave tert-butyl N-[[4-[4-[3-[5-[(2-acetyl-2,6-diazaspiro[3.3]heptan-6-yl)methyl]-6-methoxy-2-pyridyl]-2-chloro-phenyl]-3-chloro-2-pyridyl]-2-methoxy-phenyl]methyl]-N-tetrahydropyran-4-yl-carbamate as crude, LCMS: m/z found 802.3 [M+H]⁺, which was deprotected and purified by General Boc Deprotection Procedure to afford the final product as formic acid salt. 62% yield over two steps. LCMS: m/z found 702.3 [M+H]⁺, retention time = 1.73 min (Method D). ¹H NMR (400 MHz, Methanol-*d*₄): δ 8.64 (d, 1H), 8.55 (s, 2H), 7.69 (t, 2H), 7.55 (t, 1H), 7.49 - 7.43 (m, 2H), 7.43 - 7.37 (m, 1H), 7.35 - 7.27 (m, 2H), 7.24 (d, 1H), 4.30 (s, 2H), 4.08 - 3.89 (m, 12H), 3.68 (s, 2H), 3.55 - 3.47 (m, 4H), 3.42 (t, 2H), 3.05 - 2.91 (m, 1H), 1.98 (d, 2H), 1.84 (s, 3H), 1.64 - 1.48 (m, 2H).

### Example 315: N-((6-(3-(2-(4-((7-Oxa-2-azaspiro[3.5]nonan-2-yl)methyl)-3-methoxyphenyl)-3-chloropyridin-4-yl)-2-chlorophenyl)-2-methoxypyridin-3-yl)methyl)tetrahydro-2H-pyran-4-amine (530)

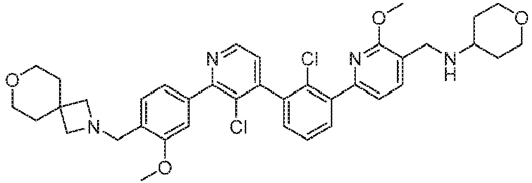

Reductive Amination Procedure B from tert-butyl ((6-(2-chloro-3-(3-chloro-2-(4-formyl-3-methoxyphenyl)pyridin-4-yl)phenyl)-2-methoxypyridin-3-yl)methyl)(tetrahydro-2H-pyran-4-yl)carbamate (Example 305, step (b)) (50 mg, 0.07 mmol) and 7-oxa-2-azaspiro[3.5]nonane (19 mg, 0.15 mmol) gave tert-butyl N-[[6-[2-chloro-3-[3-chloro-2-[3-methoxy-4-(7-oxa-2-azaspiro[3.5]nonan-2-ylmethyl)phenyl]-4-pyridyl]phenyl]-2-methoxy-3-pyridyl]methyl]-N-tetrahydropyran-4-yl-carbamate as crude, LCMS: m/z found 789.3 [M+H]⁺, which was deprotected and purified by General Boc Deprotection Procedure to afford the final product as formic acid salt. 66% yield over two steps. LCMS: m/z found 689.3 [M+H]⁺, retention time = 1.85 min (Method D). ¹H NMR (400 MHz, Methanol-*d*₄): δ 8.63 (d, 1H), 8.54 (s, 2H), 7.77 (d, 1H), 7.71 (dd, 1H), 7.55 (t, 1H), 7.47 - 7.38 (m, 3H), 7.34 - 7.21 (m, 3H), 4.03 (s, 3H), 3.98 (t, 4H), 3.92 (s, 5H), 3.60 (t, 4H), 3.51 - 3.37 (m, 6H), 2.94 - 2.80 (m, 1H), 1.95 (d, 2H), 1.80 (t, 4H), 1.51 (qd, 2H).

### Example 316: N-(4-(4-(3-(5-((7-oxa-2-azaspiro[3.5]nonan-2-yl)methyl)-6-methoxypyridin-2-yl)-2-chlorophenyl)-3-chloropyridin-2-yl)-2-methoxybenzyl)tetrahydro-2H-pyran-4-amine (531)

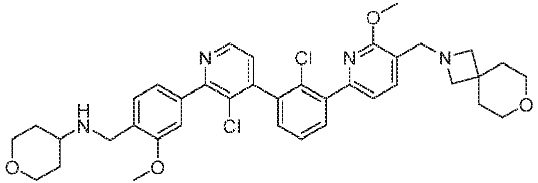

Reductive Amination Procedure B from tert-butyl (4-(3-chloro-4-(2-chloro-3-(5-formyl-6-methoxypyridin-2-yl)phenyl)pyridin-2-yl)-2-methoxybenzyl)(tetrahydro-2H-pyran-4-yl)carbamate (Example 307, step (b)) (50 mg, 0.07 mmol) and 7-oxa-2-azaspiro[3.5]nonane (19 mg, 0.15 mmol) gave tert-butyl N-[[4-[3-chloro-4-[2-chloro-3-[6-methoxy-5-(7-oxa-2-azaspiro[3.5]nonan-2-ylmethyl)-2-pyridyl]phenyl]-2-pyridyl]-2-methoxy-phenyl]methyl]-N-tetrahydropyran-4-yl-carbamate as crude, LCMS: m/z found 789.3 [M+H]⁺, which was deprotected and purified by General Boc Deprotection Procedure to afford the final product as formic acid salt. 68% yield over two steps. LCMS: m/z found 689.3 [M+H]⁺, retention time = 1.84 min (Method D). ¹H NMR (400 MHz, Methanol-*d*₄): δ 8.64 (dd, 1H), 8.59 - 8.51 (m, 2H), 7.73 - 7.66 (m, 2H), 7.55 (t, 1H), 7.49 - 7.43 (m, 2H), 7.41 (d, 1H), 7.36 - 7.28 (m, 2H), 7.25 (dd, 1H), 4.09 (s, 2H), 4.05 - 3.91 (m, 8H), 3.79 (s, 2H), 3.67 - 3.54 (m, 4H), 3.51 - 3.35 (m, 5H), 3.15 - 2.99 (m, 2H), 2.00 (d, 2H), 1.87 - 1.74 (m, 4H), 1.66 - 1.51 (m, 2H).

### Example 317: 1-(4-(((6-(2-Chloro-3-(3-chloro-2-(4-((((1r,4r)-4-hydroxycyclohexyl)amino)methyl)-3-methoxyphenyl)pyridin-4-yl)phenyl)-2-methoxypyridin-3-yl)methyl)amino)piperidin-1-yl)ethan-1-one (360)

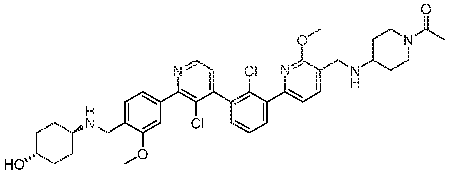

### (a) tert-Butyl (1-acetylpiperidin-4-yl)((6-(2-chloro-3-(3-chloro-2-(4-((((1r,4r)-4-hydroxycyclohexyl)amino)methyl)-3-methoxyphenyl)pyridin-4-yl)phenyl)-2-methoxypyridin-3-yl)methyl)carbamate

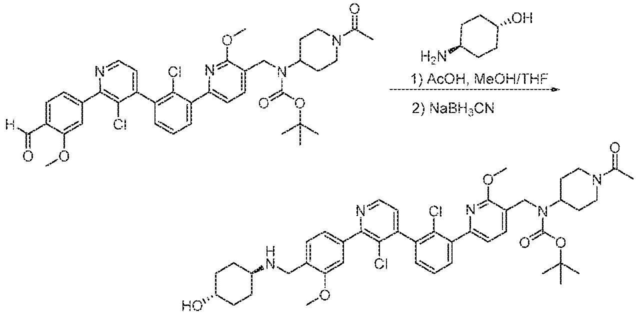

tert-Butyl (1-acetylpiperidin-4-yl)((6-(2-chloro-3-(3-chloro-2-(4-((((1r,4r)-4-hydroxycyclohexyl)amino)methyl)-3-methoxyphenyl)pyridin-4-yl)phenyl)-2-methoxypyridin-3-yl)methyl)carbamate was prepared using Reductive Amination Procedure B from tert-butyl (1-acetylpiperidin-4-yl)((6-(2-chloro-3-(3-chloro-2-(4-formyl-3-methoxyphenyl)pyridin-4-yl)phenyl)-2-methoxypyridin-3-yl)methyl)carbamate (20 mg, 0.03 mmol), acetic acid (3 uL, 0.06 mmol), 4-aminocyclohexanol (6 mg, 0.06 mmol) and sodium cyanoborohydride (3 mg, 0.06 mmol). 22 mg, 97% yield. MS: m/z found 818.3 [M+H]⁺.

### (b) 1-(4-(((6-(2-Chloro-3-(3-chloro-2-(4-((((1r,4r)-4-hydroxycyclohexyl)amino)methyl)-3-methoxyphenyl)pyridin-4-yl)phenyl)-2-methoxypyridin-3-yl)methyl)amino)piperidin-1-yl)ethan-1-one

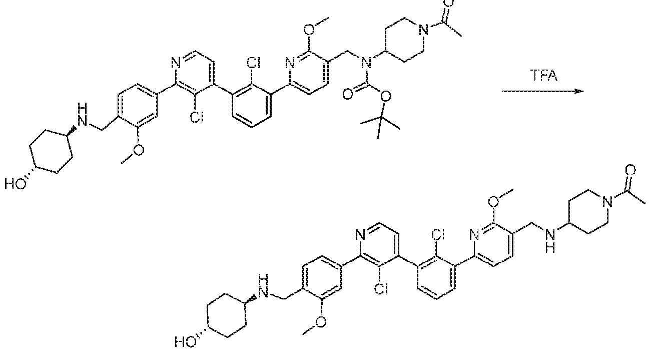

1-(4-(((6-(2-Chloro-3-(3-chloro-2-(4-((((1r,4r)-4-hydroxycyclohexyl)amino)methyl)-3-methoxyphenyl)pyridin-4-yl)phenyl)-2-methoxypyridin-3-yl)methyl)amino)piperidin-1-yl)ethan-1-one was prepared using General Boc Deprotection Procedure from tert-butyl (1-acetylpiperidin-4-yl)((6-(2-chloro-3-(3-chloro-2-(4-((((1r,4r)-4-hydroxycyclohexyl)amino)methyl)-3-methoxyphenyl)pyridin-4-yl)phenyl)-2-methoxypyridin-3-yl)methyl)carbamate (22 mg, 0.03 mmol) and trifluoroacetic acid (102 uL, 1.34 mmol). 6 mg, 32% yield. MS: m/z found 718.2 [M+H]⁺, retention time = 1.71 min (Method D). ¹H NMR (400 MHz, Methanol-*d₄*) δ 8.64 (m, 1H), 7.77 (d, 1H), 7.73 - 7.68 (m, 1H), 7.58 - 7.51 (m, 1H), 7.51 - 7.44 (m, 2H), 7.43 - 7.38 (m, 1H), 7.36 (s, 1H), 7.32 (dd, 1H), 7.28 - 7.23 (m, 1H), 4.49 (d, 1H), 4.20 (s, 2H), 4.02 (t, 3H), 3.97 (t, 4H), 3.91 (d, 3H), 3.56 (s, 1H), 3.15 (t, 1H), 3.01 (s, 1H), 2.85 (s, 1H), 2.70 (t, 1H), 2.18 (d, 2H), 2.10 (t, 3H), 2.03 (s, 4H), 1.57 - 1.24 (m, 6H).

### Example 318: 1-(4-(((6-(2-Chloro-3-(3-chloro-2-(4-((6-hydroxy-2-azaspiro[3.3]heptan-2-yl)methyl)-3-methoxyphenyl)pyridin-4-yl)phenyl)-2-methoxypyridin-3-yl)methyl)amino)piperidin-1-yl)ethan-1-one (361)

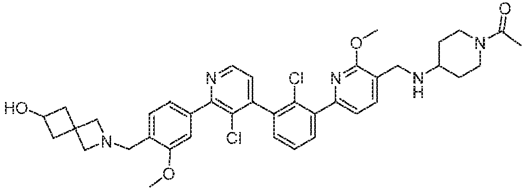

### (a) tert-Butyl (1-acetylpiperidin-4-yl)((6-(2-chloro-3-(3-chloro-2-(4-((6-hydroxy-2-azaspiro[3.3]heptan-2-yl)methyl)-3-methoxyphenyl)pyridin-4-yl)phenyl)-2-methoxypyridin-3-yl)methyl)carbamate

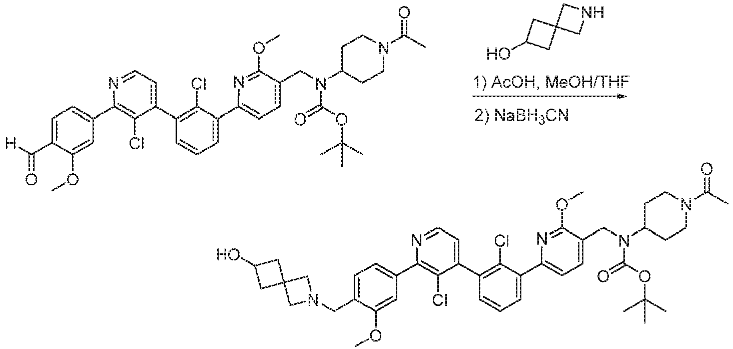

tert-Butyl (1-acetylpiperidin-4-yl)((6-(2-chloro-3-(3-chloro-2-(4-((6-hydroxy-2-azaspiro[3.3]heptan-2-yl)methyl)-3-methoxyphenyl)pyridin-4-yl)phenyl)-2-methoxypyridin-3-yl)methyl)carbamate was prepared using Reductive Amination Procedure B from tert-butyl (1-acetylpiperidin-4-yl)((6-(2-chloro-3-(3-chloro-2-(4-formyl-3-methoxyphenyl)pyridin-4-yl)phenyl)-2-methoxypyridin-3-yl)methyl)carbamate (20 mg, 0.03 mmol), acetic acid (3 uL, 0.06 mmol), 2-azaspiro[3.3]heptan-6-ol (6 mg, 0.06 mmol) and sodium cyanoborohydride (3 mg, 0.06 mmol). 21 mg, 92%. MS: m/z found 816.2 [M+H]⁺.

### (b) 1-(4-(((6-(2-Chloro-3-(3-chloro-2-(4-((6-hydroxy-2-azaspiro[3.3]heptan-2-yl)methyl)-3-methoxyphenyl)pyridin-4-yl)phenyl)-2-methoxypyridin-3-yl)methyl)amino)piperidin-1-yl)ethan-1-one

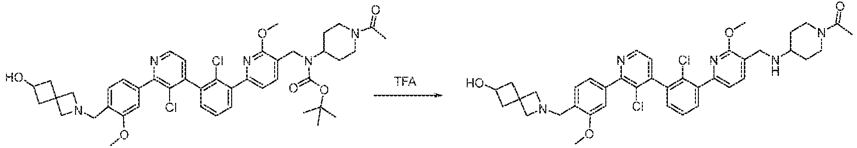

1-(4-(((6-(2-Chloro-3-(3-chloro-2-(4-((6-hydroxy-2-azaspiro[3.3]heptan-2-yl)methyl)-3-methoxyphenyl)pyridin-4-yl)phenyl)-2-methoxypyridin-3-yl)methyl)amino)piperidin-1-yl)ethan-1-one was prepared using General Boc Deprotection Procedure from tert-butyl (1-acetylpiperidin-4-yl)((6-(2-chloro-3-(3-chloro-2-(4-((6-hydroxy-2-azaspiro[3.3]heptan-2-yl)methyl)-3-methoxyphenyl)pyridin-4-yl)phenyl)-2-methoxypyridin-3-yl)methyl)carbamate (21 mg, 0.03 mmol) and trifluoroacetic acid (98 uL, 1.29 mmol). 2.5 mg, 14%. MS: m/z found 716.2 [M+H]⁺ retention time = 21.71 min (Method D). ¹H NMR (400 MHz, Methanol-*d₄*) δ 8.66 (d, 1H), 7.89 (d, 1H), 7.71 (d, 1H), 7.57 (t, 1H), 7.52 - 7.41 (m, 3H), 7.38 (s, 1H), 7.35 (d, 2H), 4.66 (d, 1H), 4.41 (s, 2H), 4.28 (s, 2H), 4.16 (d, 5H), 4.08 (t, 5H), 3.97 (d, 3H), 3.21 (t, 1H), 2.76 - 2.57 (m, 3H), 2.33 - 2.08 (m, 7H), 1.71 - 1.45 (m, 1H).

### Example 319: 1-(4-(((6-(3-(2-(4-(((2-Oxaspiro[3.3]heptan-6-yl)amino)methyl)-3-methoxyphenyl)-3-chloropyridin-4-yl)-2-chlorophenyl)-2-methoxypyridin-3-yl)methyl)amino)piperidin-1-yl)ethan-1-one (379)

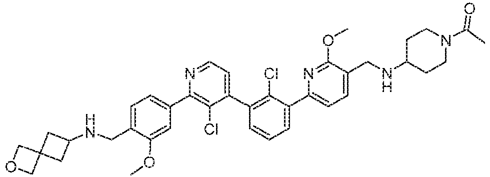

### (a) tert-Butyl ((6-(3-(2-(4-(((2-oxaspiro[3.3]heptan-6-yl)amino)methyl)-3-methoxyphenyl)-3-chloropyridin-4-yl)-2-chlorophenyl)-2-methoxypyridin-3-yl)methyl)(1-acetylpiperidin-4-yl)carbamate

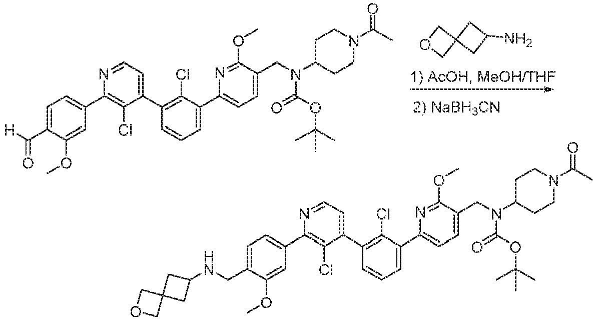

tert-Butyl ((6-(3-(2-(4-(((2-oxaspiro[3.3]heptan-6-yl)amino)methyl)-3-methoxyphenyl)-3-chloropyridin-4-yl)-2-chlorophenyl)-2-methoxypyridin-3-yl)methyl)(1-acetylpiperidin-4-yl)carbamate was prepared using Reductive Amination Procedure B from tert-butyl (1-acetylpiperidin-4-yl)((6-(2-chloro-3-(3-chloro-2-(4-formyl-3-methoxyphenyl)pyridin-4-yl)phenyl)-2-methoxypyridin-3-yl)methyl)carbamate (20 mg, 0.03 mmol), acetic acid (3 uL, 0.06 mmol), 2-oxaspiro[3.3]heptan-6-amine;hydrochloride (8 mg, 0.06 mmol) and sodium cyanoborohydride (3 mg, 0.06 mmol). 13 mg, 57% yield. MS: m/z found 816.2 [M+H]⁺.

### (b) 1-(4-(((6-(3-(2-(4-(((2-Oxaspiro[3.3]heptan-6-yl)amino)methyl)-3-methoxyphenyl)-3-chloropyridin-4-yl)-2-chlorophenyl)-2-methoxypyridin-3-yl)methyl)amino)piperidin-1-yl)ethan-1-one

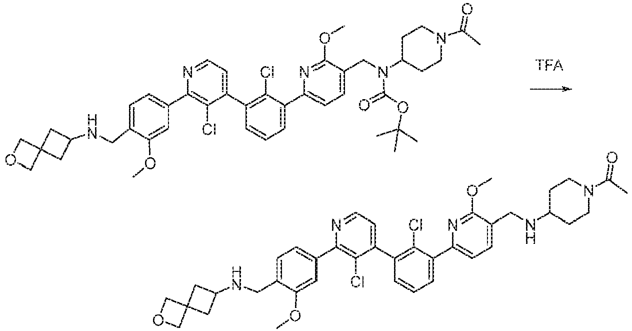

1-(4-(((6-(3-(2-(4-(((2-Oxaspiro[3.3]heptan-6-yl)amino)methyl)-3-methoxyphenyl)-3-chloropyridin-4-yl)-2-chlorophenyl)-2-methoxypyridin-3-yl)methyl)amino)piperidin-1-yl)ethan-1-one was prepared using General Boc Deprotection Procedure from tert-butyl ((6-(3-(2-(4-(((2-oxaspiro[3.3]heptan-6-yl)amino)methyl)-3-methoxyphenyl)-3-chloropyridin-4-yl)-2-chlorophenyl)-2-methoxypyridin-3-yl)methyl)(1-acetylpiperidin-4-yl)carbamate (13 mg, 0.02 mmol) and trifluoroacetic acid (60 uL, 0.80 mmol). 2.2 mg, 19% yield. MS: m/z found 718.2 [M+H]⁺, retention time = 2.21 min (Method C). ¹H NMR (400 MHz, Methanol-*d₄*). δ 8.69 - 8.62 (m, 1H), 7.85 (d, 1H), 7.71 (d, 1H), 7.56 (t, 1H), 7.51 - 7.40 (m, 3H), 7.38 (s, 1H), 7.32 (t, 2H), 4.73 (s, 1H), 4.62 (s, 1H), 4.58 (s, 1H), 4.14 (s, 4H), 4.11 (s, 1H), 4.06 (d, 4H), 3.99 (d, 4H), 3.65 (d, 1H), 3.24 - 3.12 (m, 2H), 2.68 (d, 2H), 2.35 (s, 1H), 2.18 (d, 2H), 2.12 (d, 5H), 1.16 (s, 1H).

### Example 320: 1-(4-(((6-(3-(2-(4-((2-Oxa-6-azaspiro[3.3]heptan-6-yl)methyl)-3-methoxyphenyl)-3-chloropyridin-4-yl)-2-chlorophenyl)-2-methoxypyridin-3-yl)methyl)amino)piperidin-1-yl)ethan-1-one (380)

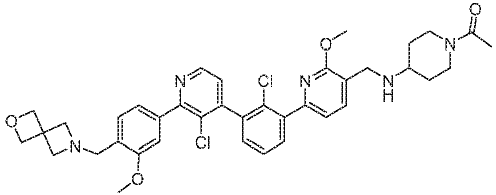

### (a) tert-Butyl ((6-(3-(2-(4-((2-oxa-6-azaspiro[3.3]heptan-6-yl)methyl)-3-methoxyphenyl)-3-chloropyridin-4-yl)-2-chlorophenyl)-2-methoxypyridin-3-yl)methyl)(1-acetylpiperidin-4-yl)carbamate

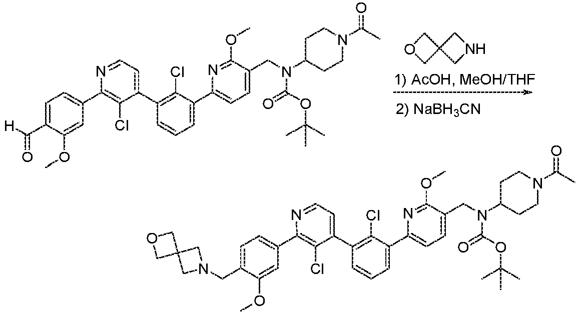

tert-Butyl ((6-(3-(2-(4-((2-oxa-6-azaspiro[3.3]heptan-6-yl)methyl)-3-methoxyphenyl)-3-chloropyridin-4-yl)-2-chlorophenyl)-2-methoxypyridin-3-yl)methyl)(1-acetylpiperidin-4-yl)carbamate was prepared using Reductive Amination Procedure B from tert-butyl (1-acetylpiperidin-4-yl)((6-(2-chloro-3-(3-chloro-2-(4-formyl-3-methoxyphenyl)pyridin-4-yl)phenyl)-2-methoxypyridin-3-yl)methyl)carbamate (20 mg, 0.03 mmol), acetic acid (3 uL, 0.06 mmol), 2-oxa-6-azaspiro[3.3]heptane (5 mg, 0.06 mmol) and sodium cyanoborohydride (3 mg, 0.06 mmol). 14 mg, 63% yield. MS: m/z found 802.2 [M+H]⁺.

### (b) 1-(4-(((6-(3-(2-(4-((2-Oxa-6-azaspiro[3.3]heptan-6-yl)methyl)-3-methoxyphenyl)-3-chloropyridin-4-yl)-2-chlorophenyl)-2-methoxypyridin-3-yl)methyl)amino)piperidin-1-yl)ethan-1-one

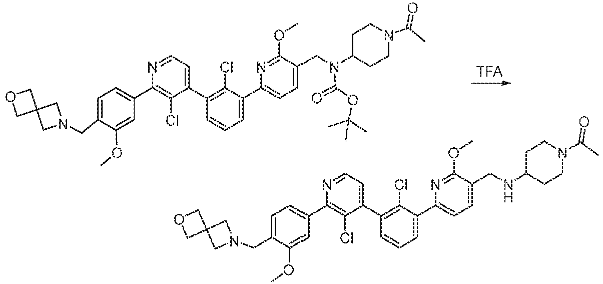

1-(4-(((6-(3-(2-(4-((2-Oxa-6-azaspiro[3.3]heptan-6-yl)methyl)-3-methoxyphenyl)-3-chloropyridin-4-yl)-2-chlorophenyl)-2-methoxypyridin-3-yl)methyl)amino)piperidin-1-yl)ethan-1-one was prepared using General Boc Deprotection Procedure from tert-butyl ((6-(3-(2-(4-((2-oxa-6-azaspiro[3.3]heptan-6-yl)methyl)-3-methoxyphenyl)-3-chloropyridin-4-yl)-2-chlorophenyl)-2-methoxypyridin-3-yl)methyl)(1-acetylpiperidin-4-yl)carbamate (14 mg, 0.02 mmol) and trifluoroacetic acid (66 uL, 0.87 mmol). 3.3 mg, 27%. MS: m/z found 702.1 [M+H]⁺, retention time = 1.70 min (Method D). ¹H NMR (400 MHz, Methanol-*d₄*). δ 8.67 - 8.62 (m, 1H), 7.86 (d, 1H), 7.71 (d, 1H), 7.56 (t, 1H), 7.44 (dd, 3H), 7.37 - 7.29 (m, 3H), 4.77 (t, 4H), 4.61 (d, 1H), 4.20 (d, 4H), 4.14 (s, 4H), 4.06 (t, 4H), 4.00 (d, 1H), 3.95 (t, 3H), 3.19 (t, 1H), 2.70 (t, 1H), 2.19 (t, 2H), 2.12 (t, 3H), 1.66 - 1.40 (m, 1H).

### Example 321: 1-(4-(((6-(2-Chloro-3-(3-chloro-2-(4-(((3,3-dimethylcyclobutyl)amino)methyl)-3-methoxyphenyl)pyridin-4-yl)phenyl)-2-methoxypyridin-3-yl)methyl)amino)piperidin-1-yl)ethan-1-one (381)

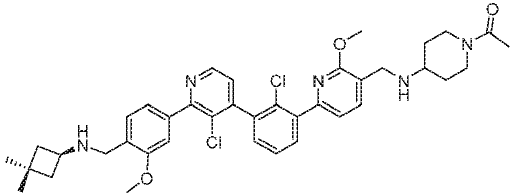

### (a) tert-Butyl (1-acetylpiperidin-4-yl)((6-(2-chloro-3-(3-chloro-2-(4-((((1r,3r)-3-hydroxy-3-methylcyclobutyl)amino)methyl)-3-methoxyphenyl)pyridin-4-yl)phenyl)-2-methoxypyridin-3-yl)methyl)carbamate

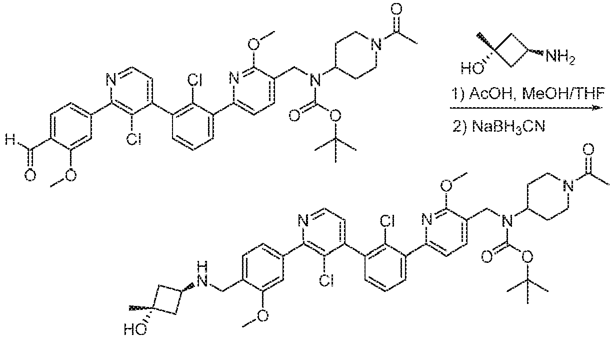

tert-Butyl (1-acetylpiperidin-4-yl)((6-(2-chloro-3-(3-chloro-2-(4-((((1r,3r)-3-hydroxy-3-methylcyclobutyl)amino)methyl)-3-methoxyphenyl)pyridin-4-yl)phenyl)-2-methoxypyridin-3-yl)methyl)carbamate was prepared using Reductive Amination Procedure B from tert-butyl (1-acetylpiperidin-4-yl)((6-(2-chloro-3-(3-chloro-2-(4-formyl-3-methoxyphenyl)pyridin-4-yl)phenyl)-2-methoxypyridin-3-yl)methyl)carbamate (20 mg, 0.03 mmol), acetic acid (3 uL, 0.06 mmol), 3-amino-1-methyl-cyclobutanol;hydrochloride (7 mg, 0.06 mmol) and sodium cyanoborohydride (3 mg, 0.06 mmol). 12 mg, 54% yield. MS: m/z found 804.2 [M+H]⁺.

### (b) 1-(4-(((6-(2-Chloro-3-(3-chloro-2-(4-(((3,3-dimethylcyclobutyl)amino)methyl)-3-methoxyphenyl)pyridin-4-yl)phenyl)-2-methoxypyridin-3-yl)methyl)amino)piperidin-1-yl)ethan-1-one

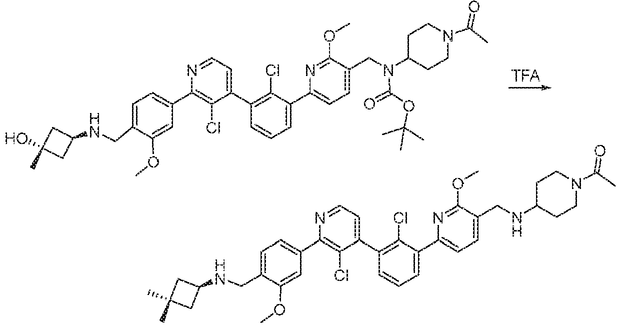

1-(4-(((6-(2-Chloro-3-(3-chloro-2-(4-(((3,3-dimethylcyclobutyl)amino)methyl)-3-methoxyphenyl)pyridin-4-yl)phenyl)-2-methoxypyridin-3-yl)methyl)amino)piperidin-1-yl)ethan-1-one was prepared using General Boc Deprotection Procedure from tert-butyl N-(1-acetyl-4-piperidyl)-N-[[6-[2-chloro-3-[3-chloro-2-[4-[[(3-hydroxy-3-methylcyclobutyl)amino]methyl]-3-methoxy-phenyl]-4-pyridyl]phenyl]-2-methoxy-3-pyridyl]methyl]carbamate (12 mg, 0.01 mmol) and trifluoroacetic acid (57 uL, 0.75 mmol). 2.5 mg, 24% yield. MS: m/z found 704.2 [M+H]⁺, retention time = 1.71 min (Method D). ¹H NMR (400 MHz, Methanol-*d₄*). δ 8.66 (d, 1H), 7.86 (d, 1H), 7.75 - 7.67 (m, 1H), 7.57 (t, 1H), 7.51 - 7.41 (m, 3H), 7.38 (s, 1H), 7.33 (t, 2H), 4.61 (d, 1H), 4.17 (s, 5H), 4.06 (t, 4H), 3.99 (t, 4H), 3.92 (t, 1H), 3.19 (t, 1H), 2.75 - 2.62 (m, 1H), 2.38 (t, 2H), 2.25 - 2.15 (m, 4H), 2.12 (d, 4H), 1.62 - 1.42 (m, 2H).

### Example 322: 1-(4-(((6-(2-Chloro-3-(3-chloro-2-(4-((((1-hydroxycyclopropyl)methyl)amino)methyl)-3-methoxyphenyl)pyridin-4-yl)phenyl)-2-methoxypyridin-3-yl)methyl)amino)piperidin-1-yl)ethan-1-one (512)

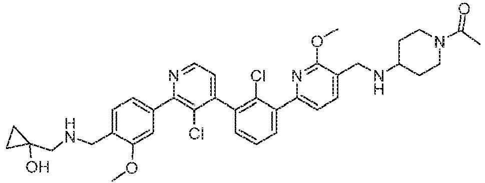

### (a) tert-Butyl (1-acetylpiperidin-4-yl)((6-(2-chloro-3-(3-chloro-2-(4-((((1-hydroxycyclopropyl)methyl)amino)methyl)-3-methoxyphenyl)pyridin-4-yl)phenyl)-2-methoxypyridin-3-yl)methyl)carbamate

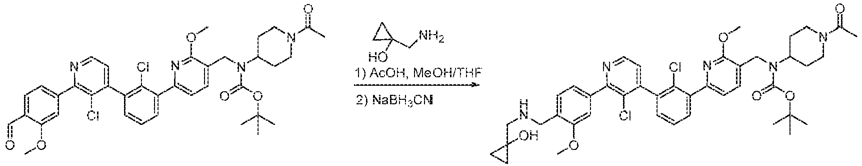

tert-Butyl (1-acetylpiperidin-4-yl)((6-(2-chloro-3-(3-chloro-2-(4-((((1-hydroxycyclopropyl)methyl)amino)methyl)-3-methoxyphenyl)pyridin-4-yl)phenyl)-2-methoxypyridin-3-yl)methyl)carbamate was prepared using Reductive Amination Procedure B from tert-butyl (1-acetylpiperidin-4-yl)((6-(2-chloro-3-(3-chloro-2-(4-formyl-3-methoxyphenyl)pyridin-4-yl)phenyl)-2-methoxypyridin-3-yl)methyl)carbamate (20 mg, 0.03 mmol), acetic acid (3 uL, 0.06 mmol), 1-(aminomethyl)cyclopropanol (5 mg, 0.06 mmol) and sodium cyanoborohydride (3.50 mg, 0.06 mmol). 14 mg, 48%. MS: m/z found 790.2 [M+H]⁺.

### (b) 1-(4-(((6-(2-Chloro-3-(3-chloro-2-(4-((((1-hydroxycyclopropyl)methyl)amino)methyl)-3-methoxyphenyl)pyridin-4-yl)phenyl)-2-methoxypyridin-3-yl)methyl)amino)piperidin-1-yl)ethan-1-one

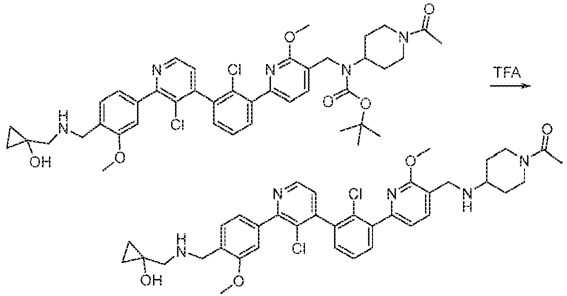

To a solution of tert-butyl (1-acetylpiperidin-4-yl)((6-(2-chloro-3-(3-chloro-2-(4-((((1-hydroxycyclopropyl)methyl)amino)methyl)-3-methoxyphenyl)pyridin-4-yl)phenyl)-2-methoxypyridin-3-yl)methyl)carbamate (14 mg, 0.02 mmol) in DCM (0.1 mL) was added trifluoroacetic acid (69 uL, 0.90 mmol). The reaction was stirred at room temperature for 1 h. The solvent was evaporated and the residue was purified by reverse phase HPLC (acetonitrile: water) to afford 1-(4-(((6-(2-chloro-3-(3-chloro-2-(4-((((1-hydroxycyclopropyl)methyl)amino)methyl)-3-methoxyphenyl)pyridin-4-yl)phenyl)-2-methoxypyridin-3-yl)methyl)amino)piperidin-1-yl)ethan-1-one (3.1 mg, 25%). MS: m/z found 690.3 [M+H]⁺, retention time = 1.71 min (Method D). ¹H NMR (400 MHz, Methanol-*d₄*). δ 8.66 (d, 1H), 7.90 (d, 1H), 7.72 (d, 1H), 7.57 (t, 1H), 7.52 (d, 1H), 7.46 (dd, 2H), 7.39 (s, 1H), 7.35 (d, 2H), 4.66 (d, 1H), 4.39 (s, 2H), 4.28 (s, 2H), 4.08 (s, 3H), 3.99 (d, 3H), 3.15 (s, 2H), 2.75 - 2.64 (m, 1H), 2.24 (t, 2H), 2.13 (d, 3H), 1.67 - 1.49 (m, 1H), 0.90 (s, 2H), 0.72 (s, 2H).

### Example 323: 1-(4-(((6-(2-Chloro-3-(3-chloro-2-(4-(((2,2-difluoroethyl)amino)methyl)-3-methoxyphenyl)pyridin-4-yl)phenyl)-2-methoxypyridin-3-yl)methyl)amino)piperidin-1-yl)ethan-1-one (513)

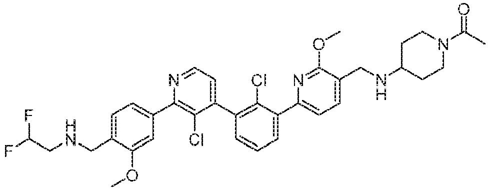

### (a) tert-Butyl (1-acetylpiperidin-4-yl)((6-(2-chloro-3-(3-chloro-2-(4-(((2,2-difluoroethyl)amino)methyl)-3-methoxyphenyl)pyridin-4-yl)phenyl)-2-methoxypyridin-3-yl)methyl)carbamate

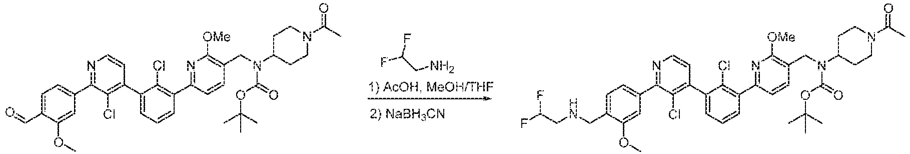

tert-Butyl (1-acetylpiperidin-4-yl)((6-(2-chloro-3-(3-chloro-2-(4-(((2,2-difluoroethyl)amino)methyl)-3-methoxyphenyl)pyridin-4-yl)phenyl)-2-methoxypyridin-3-yl)methyl)carbamate was prepared using Reductive Amination Procedure B from tert-butyl (1-acetylpiperidin-4-yl)((6-(2-chloro-3-(3-chloro-2-(4-formyl-3-methoxyphenyl)pyridin-4-yl)phenyl)-2-methoxypyridin-3-yl)methyl)carbamate (20 mg, 0.03 mmol), 2,2-difluoroethanamine (5 mg, 0.06 mmol), acetic acid (3 mg, 0.06 mmol) and sodium cyanoborohydride (3 mg, 0.06 mmol). 15 mg, 69% yield. MS: m/z found 784.2 [M+H]⁺.

### (b) 1-(4-(((6-(2-Chloro-3-(3-chloro-2-(4-(((2,2-difluoroethyl)amino)methyl)-3-methoxyphenyl)pyridin-4-yl)phenyl)-2-methoxypyridin-3-yl)methyl)amino)piperidin-1-yl)ethan-1-one

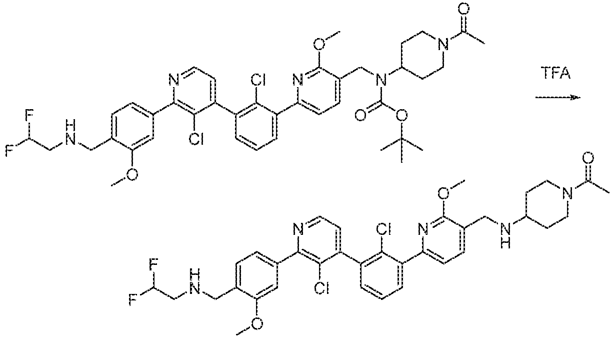

1-(4-(((6-(2-Chloro-3-(3-chloro-2-(4-(((2,2-difluoroethyl)amino)methyl)-3-methoxyphenyl)pyridin-4-yl)phenyl)-2-methoxypyridin-3-yl)methyl)amino)piperidin-1-yl)ethan-1-one was prepared using General Boc Deprotection Procedure from tert-butyl (1-acetylpiperidin-4-yl)((6-(2-chloro-3-(3-chloro-2-(4-(((2,2-difluoroethyl)amino)methyl)-3-methoxyphenyl)pyridin-4-yl)phenyl)-2-methoxypyridin-3-yl)methyl)carbamate (14 mg, 0.02 mmol) and trifluoroacetic acid (69 uL, 0.90 mmol). 4.9 mg, 40% yield. MS: m/z found 684.3 [M+H]⁺, retention time = 1.71 min (Method D). ¹H NMR (400 MHz, Methanol-*d₄*) δ 8.63 (dd, 1H), 7.90 (d, 1H), 7.71 (d, 1H), 7.57 (dd, 1H), 7.44 (q, 4H), 7.38 - 7.34 (m, 1H), 7.33-7.26 (m, 2H), 4.67 (d, 1H), 4.30 (s, 2H), 4.08 (t, 4H), 4.05 (s, 2H), 3.94 (d, 3H), 3.45 (d, 1H), 3.26 - 3.07 (m, 3H), 2.76 - 2.64 (m, 2H), 2.25 (t, 2H), 2.13 (d, 4H), 1.68 - 1.50 (m, 1H).

### Example 324: N-((6-(2-Chloro-3-(3-chloro-2-(4-(((3-fluoropropyl)amino)methyl)-3-methoxyphenyl)pyridin-4-yl)phenyl)-2-methoxypyridin-3-yl)methyl)-3-fluoropropan-1-amine (534)

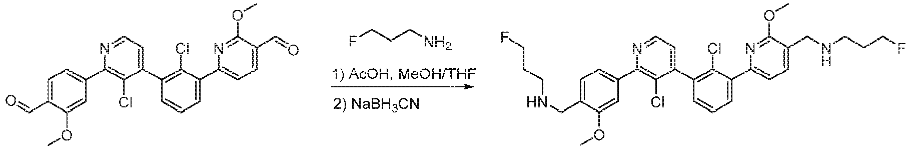

N-((6-(2-Chloro-3-(3-chloro-2-(4-(((3-fluoropropyl)amino)methyl)-3-methoxyphenyl)pyridin-4-yl)phenyl)-2-methoxypyridin-3-yl)methyl)-3-fluoropropan-1-amine was prepared as the formic acid salt following Reductive Amination Procedure A from 6-(2-chloro-3-(3-chloro-2-(4-formyl-3-methoxyphenyl)pyridin-4-yl)phenyl)-2-methoxynicotinaldehyde (14 mg, 0.02 mmol), 3-fluoropropan-1-amine (9 mg, 0.11 mmol), acetic acid (3 µL, 0.06 mmol) and sodium cyanoborohydride (3.5 mg, 0.06 mmol). 8.6 mg, 49% yield. MS: m/z found 615.2 [M+H]⁺, retention time = 1.87 min (Method D) ¹H NMR (400 MHz, Methanol-*d₄*) δ 8.66 (d, 1H), 7.89 (d, 1H), 7.72 (d, 1H), 7.57 (dd, 1H), 7.53 (d, 1H), 7.48 - 7.42 (m, 2H), 7.39 (s, 1H), 7.35 (d, 2H), 4.65 (t, 2H), 4.53 (t, 2H), 4.30 (d, 4H), 4.08 (d, 3H), 3.99 (d, 3H), 3.24 (q, 4H), 2.24 - 2.06 (m, 4H).

### Example 325: 1-(4-(((6-(2-Chloro-3-(3-chloro-2-(4-(((3-fluoropropyl)amino)methyl)-3-methoxyphenyl)pyridin-4-yl)phenyl)-2-methoxypyridin-3-yl)methyl)amino)piperidin-1-yl)ethan-1-one (537)

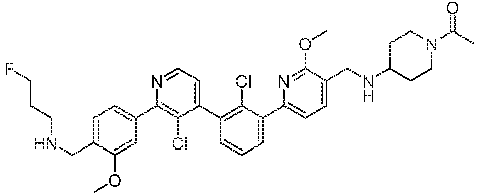

### (a) tert-Butyl (1-acetylpiperidin-4-yl)((6-(2-chloro-3-(3-chloro-2-(4-(((3-fluoropropyl)amino)methyl)-3-methoxyphenyl)pyridin-4-yl)phenyl)-2-methoxypyridin-3-yl)methyl)carbamate

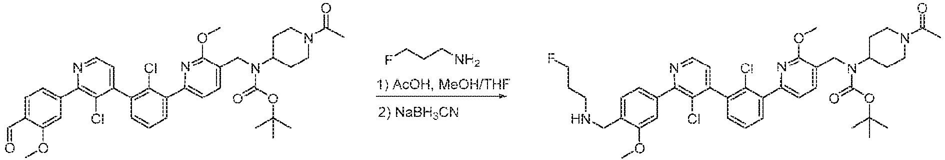

tert-Butyl (1-acetylpiperidin-4-yl)((6-(2-chloro-3-(3-chloro-2-(4-(((3-fluoropropyl)amino)methyl)-3-methoxyphenyl)pyridin-4-yl)phenyl)-2-methoxypyridin-3-yl)methyl)carbamate was prepared using Reductive Amination Procedure B from tert-butyl (1-acetylpiperidin-4-yl)((6-(2-chloro-3-(3-chloro-2-(4-formyl-3-methoxyphenyl)pyridin-4-yl)phenyl)-2-methoxypyridin-3-yl)methyl)carbamate (20 mg, 0.03 mmol), acetic acid (3 uL, 0.06 mmol), 3-fluoropropan-1-amine (4 mg, 0.06 mmol) and sodium cyanoborohydride (3 mg, 0.06 mmol). 14 mg, 65% yield. MS: m/z found 780.3 [M+H]⁺.

### (b) 1-(4-(((6-(2-Chloro-3-(3-chloro-2-(4-(((3-fluoropropyl)amino)methyl)-3-methoxyphenyl)pyridin-4-yl)phenyl)-2-methoxypyridin-3-yl)methyl)amino)piperidin-1-yl)ethan-1-one

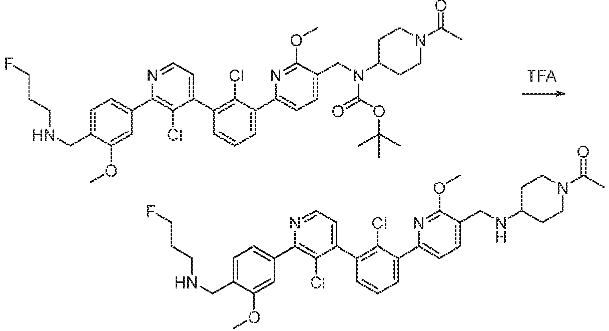

1-(4-(((6-(2-Chloro-3-(3-chloro-2-(4-(((3-fluoropropyl)amino)methyl)-3-methoxyphenyl)pyridin-4-yl)phenyl)-2-methoxypyridin-3-yl)methyl)amino)piperidin-1-yl)ethan-1-one was prepared using General Boc Deprotection Procedure from tert-butyl (1-acetylpiperidin-4-yl)((6-(2-chloro-3-(3-chloro-2-(4-(((3-fluoropropyl)amino)methyl)-3-methoxyphenyl)pyridin-4-yl)phenyl)-2-methoxypyridin-3-yl)methyl)carbamate (14 mg, 0.02 mmol) and trifluoroacetic acid (69 uL, 0.90 mmol). 3.2 mg, 26% yield. MS: m/z found 680.3 [M+H]⁺, retention time = 1.77 min (Method D). ¹H NMR (400 MHz, Methanol-*d₄*) δ 8.70 - 8.62 (m, 1H), 7.90 (d, 1H), 7.57 (t, 1H), 7.53 (d, 1H), 7.46 (dd, 2H), 7.39 (s, 1H), 7.35 (d, 2H), 4.64 (t, 2H), 4.53 (t, 1H), 4.30 (d, 4H), 4.08 (t, 4H), 3.99 (t, 3H), 3.43 (s, 1H), 3.23 (t, 3H), 2.75 - 2.62 (m, 1H), 2.31 - 2.17 (m, 3H), 2.13 (d, 4H).

### Example 326: (4-(3-Chloro-4-(2-chloro-3-(6-methoxy-5-(((((S)-5-oxopyrrolidin-2-yl)methyl)amino)methyl)pyridin-2-yl)phenyl)pyridin-2-yl)-2-methoxybenzyl)-L-alanine (130)

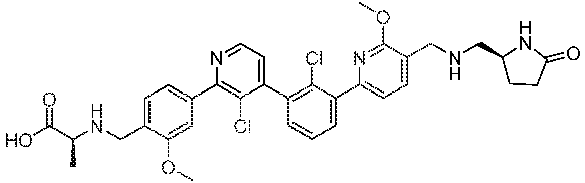

### (a) (4-(4-(3-(5-(((tert-butoxycarbonyl)(((S)-5-oxopyrrolidin-2-yl)methyl)amino)methyl)-6-methoxypyridin-2-yl)-2-chlorophenyl)-3-chloropyridin-2-yl)-2-methoxybenzyl)-L-alanine

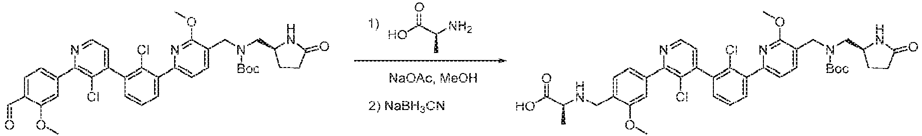

To a mixture of tert-butyl (S)-((6-(2-chloro-3-(3-chloro-2-(4-formyl-3-methoxyphenyl)pyridin-4-yl)phenyl)-2-methoxypyridin-3-yl)methyl)((5-oxopyrrolidin-2-yl)methyl)carbamate (Example 80, step (a)) (150 mg, 0.22 mmol) and (S)-2-aminopropanoic acid (58 mg, 0.65 mmol) in MeOH (5 mL) was added sodium acetate (53.4 mg, 0.65 mmol), then the mixture was stirred at room temperature for 1.5 h. Sodium cyanoborohydride (40.9 mg, 0.65 mmol) was then added and the mixture was stirred at room temperature for 0.5 h. The mixture was filtered and the filtrate concentrated to afford (4-(4-(3-(5-(((tert-butoxycarbonyl)(((S)-5-oxopyrrolidin-2-yl)methyl)amino)methyl)-6-methoxypyridin-2-yl)-2-chlorophenyl)-3-chloropyridin-2-yl)-2-methoxybenzyl)-L-alanine (170 mg, crude). LCMS *m*/*z* found 764, 766 [M+H]⁺. The crude product was used for next step without further purification.

### (b) (4-(3-chloro-4-(2-chloro-3-(6-methoxy-5-(((((S)-5-oxopyrrolidin-2-yl)methyl)amino)methyl)pyridin-2-yl)phenyl)pyridin-2-yl)-2-methoxybenzyl)-L-alanine

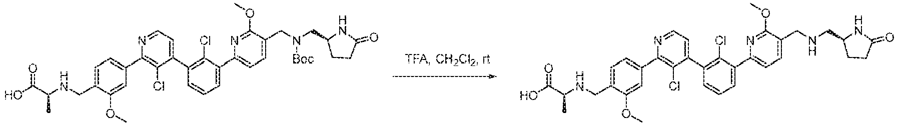

To a solution of (4-(4-(3-(5-(((tert-butoxycarbonyl)(((S)-5-oxopyrrolidin-2-yl)methyl)amino)methyl)-6-methoxypyridin-2-yl)-2-chlorophenyl)-3-chloropyridin-2-yl)-2-methoxybenzyl)-L-alanine (160 mg, 0.21 mmol) in dichloromethane (3 mL) was added trifluoroacetic acid (3 mL, 40.5 mmol) and the mixture was stirred at room temperature for 10 min under N₂. The mixture was concentrated and the residue was purified by reverse phase HPLC to afford (4-(3-chloro-4-(2-chloro-3-(6-methoxy-5-(((((S)-5-oxopyrrolidin-2-yl)methyl)amino)methyl)pyridin-2-yl)phenyl)pyridin-2-yl)-2-methoxybenzyl)-L-alanine (33.1 mg, 22% yield) as a white solid (formic acid salt). MS: *m*/*z* found 664 [M+H]⁺, retention time = 2.64 min (Method A). ¹H NMR (400 MHz, Methanol-*d*₄): δ 8.64 (d, 1H), 7.81 (d, 1H), 7.70 (dd, 1H), 7.57-7.50 (m, 2H), 7.45-7.41 (m, 2H), 7.37 (s, 1H), 7.34-7.29 (m, 2H), 4.33-4.25 (m, 2H), 4.06-4.02 (m, 5H), 3.98 (s, 3H), 9.95-3.94 (m, 1H), 3.62-3.57 (m, 1H), 3.00-2.90 (m, 2H), 2.39-2.32 (m, 3H), 1.88-1.82 (m, 1H), 1.53 (d, 1H).

### Example 327: Isopropyl (S)-1-((4-(3-chloro-4-(2-chloro-3-(6-methoxy-5-((((5-oxopyrrolidin-2-yl)methyl)amino)methyl)pyridin-2-yl)phenyl)pyridin-2-yl)-2-methoxybenzyl)amino)cyclopropane-1-carboxylate (135)

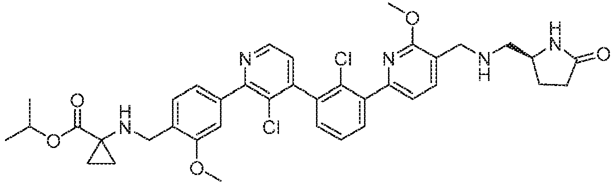

### (a) Isopropyl 1-aminocyclopropane-1-carboxylate

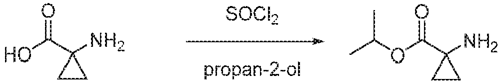

To a mixture of 1-aminocyclopropanecarboxylic acid (1 g, 9.89 mmol) and propan-2-ol (5.35 g, 89.0 mmol) was added thionyl chloride (2.46 g, 20.6 mmol), and the mixture was stirred at 95 °C under N₂ for 24 h. The mixture was then concentrated to give isopropyl 1-aminocyclopropane-1-carboxylate (1.2 g, 68% yield) as a white solid that was used in the next step without further purification.

### (b) Isopropyl (S)-1-((4-(4-(3-(5-(((tert-butoxycarbonyl)((5-oxopyrrolidin-2-yl)methyl)amino)methyl)-6-methoxypyridin-2-yl)-2-chlorophenyl)-3-chloropyridin-2-yl)-2-methoxybenzyl)amino)cyclopropane-1-carboxylate

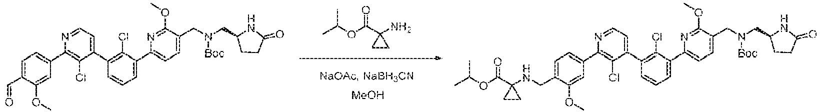

To a mixture tert-butyl (S)-((6-(2-chloro-3-(3-chloro-2-(4-formyl-3-methoxyphenyl)pyridin-4-yl)phenyl)-2-methoxypyridin-3-yl)methyl)((5-oxopyrrolidin-2-yl)methyl)carbamate (Example 80, step (a)) (300 mg, 433 umol), isopropyl 1-aminocyclopropane-1-carboxylate (248 mg, 1.74 mmol) in methanol (10 mL) was added sodium cyanoborohydride (81.7 mg, 1.30 mmol) and sodium acetate (177 mg, 2.17 mmol) and the mixture stirred at room temperature under N₂ for 3 h. The mixture was then concentrated, and the residue purified by normal phase SiO₂ chromatography (100% ethyl acetate) to afford isopropyl (S)-1-((4-(4-(3-(5-(((tert-butoxycarbonyl)((5-oxopyrrolidin-2-yl)methyl)amino)methyl)-6-methoxypyridin-2-yl)-2-chlorophenyl)-3-chloropyridin-2-yl)-2-methoxybenzyl)amino)cyclopropane-1-carboxylate (210 mg, 59% yield) as a white solid. MS: *m*/*z* found 818 [M+H]⁺.

### (c) Isopropyl (S)-1-((4-(3-chloro-4-(2-chloro-3-(6-methoxy-5-((((5-oxopyrrolidin-2-yl)methyl)amino)methyl)pyridin-2-yl)phenyl)pyridin-2-yl)-2-methoxybenzyl)amino)cyclopropane-1-carboxylate

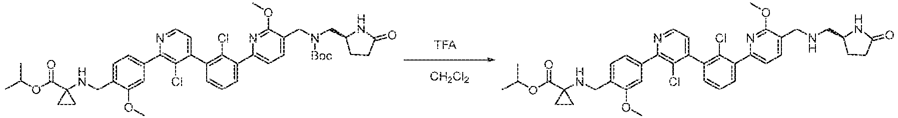

To a solution of isopropyl (S)-1-((4-(4-(3-(5-(((tert-butoxycarbonyl)((5-oxopyrrolidin-2-yl)methyl)amino)methyl)-6-methoxypyridin-2-yl)-2-chlorophenyl)-3-chloropyridin-2-yl)-2-methoxybenzyl)amino)cyclopropane-1-carboxylate (200 mg, 244 umol) in dichloromethane (3 mL) was added trifluoroacetic acid (9.24 g, 81.04 mmol, 6.00 mL, 332 eq), and the mixture was stirred at room temperature under N₂ for 0.5 h. The mixture was then filtered, the filtrate concentrated and the residue purified by reverse phase HPLC to afford isopropyl (S)-1-((4-(3-chloro-4-(2-chloro-3-(6-methoxy-5-((((5-oxopyrrolidin-2-yl)methyl)amino)methyl)pyridin-2-yl)phenyl)pyridin-2-yl)-2-methoxybenzyl)amino)cyclopropane-1-carboxylate (18.6 mg, 10.6 % yield) as a white solid (formic acid salt). MS: *m*/*z* found 718 [M+H]⁺, retention time = 3.37 min (Method A). ¹H NMR (400 MHz, Methanol-*d*₄): δ 8.66 (d, 1H), 7.90 (d, 1H), 7.72 (dd, 1H), 7.58 (t, 1H), 7.52 (d, 1H), 7.47-7.44 (m, 2H), 7.36-7.32 (m, 3H), 5.17-5.13 (m, 1H), 4.53 (s, 2H), 4.35-4.34 (m, 2H), 4.10 (s, 3H), 4.08-4.04 (m, 1H), 4.00 (s, 3H), 3.26-3.25 (m, 2H), 2.43-2.36 (m, 3H), 1.92-1.89(m, 1H), 1.63-1.59 (m, 2H), 1.50-1.47 (m, 2H), 1.32 (d, 6H).

### Example 328: (S)-5-((((6-(2-chloro-3-(3-chloro-2-(4-(4,5-dihydro-1H-imidazol-2-yl)-3-methoxyphenyl)pyridin-4-yl)phenyl)-2-methoxypyridin-3-yl)methyl)amino)methyl)pyrrolidin-2-one (179)

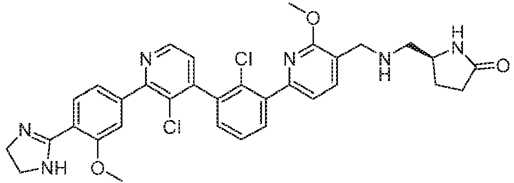

### (a) tert-butyl (S)-((6-(2-chloro-3-(3-chloro-2-(4-(4,5-dihydro-1H-imidazol-2-yl)-3-methoxyphenyl)pyridin-4-yl)phenyl)-2-methoxypyridin-3-yl)methyl)((5-oxopyrrolidin-2-yl)methyl)carbamate

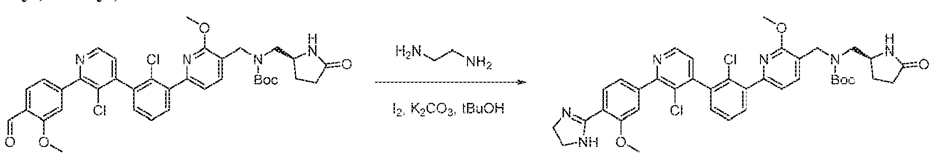

To a mixture of tert-butyl (S)-((6-(2-chloro-3-(3-chloro-2-(4-formyl-3-methoxyphenyl)pyridin-4-yl)phenyl)-2-methoxypyridin-3-yl)methyl)((5-oxopyrrolidin-2-yl)methyl)carbamate (see Example 80, step (a)) (200 mg, 0.289 mmol) and ethane-1,2-diamine (52.1 mg, 0.867 mmol) in tert-butyl alcohol (10 mL) was added iodine (220 mg, 0.867 umol) and potassium carbonate (239 mg, 1.74 mmol), then the mixture was stirred at 70 °C for 3 h under N₂. Water (10 mL) was then added, and the mixture extracted with ethyl acetate (2 x 5 mL). The combined organic phases were dried over anhydrous sodium sulfate, filtered and concentrated to afford tert-butyl (S)-((6-(2-chloro-3-(3-chloro-2-(4-(4,5-dihydro-1H-imidazol-2-yl)-3-methoxyphenyl)pyridin-4-yl)phenyl)-2-methoxypyridin-3-yl)methyl)((5-oxopyrrolidin-2-yl)methyl)carbamate (205 mg, 53% yield) as a black solid. MS: *m*/*z* found 731 [M+H]⁺. The crude product was used for next step without further purification.

### (b) (S)-5-((((6-(2-chloro-3-(3-chloro-2-(4-(4,5-dihydro-1H-imidazol-2-yl)-3-methoxyphenyl)pyridin-4-yl)phenyl)-2-methoxypyridin-3-yl)methyl)amino)methyl)pyrrolidin-2-one

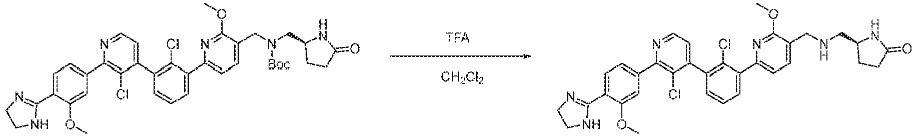

To a solution of tert-butyl (S)-((6-(2-chloro-3-(3-chloro-2-(4-(4,5-dihydro-1H-imidazol-2-yl)-3-methoxyphenyl)pyridin-4-yl)phenyl)-2-methoxypyridin-3-yl)methyl)((5-oxopyrrolidin-2-yl)methyl)carbamate (200 mg, 0.273 mmol) in dichloromethane (5 mL) was added trifluoroacetic acid (67.53 mmol, 5 mL), then the mixture was stirred at room temperature for 0.5 h under N₂ . The mixture was then filtered, the filtrate concentrated and the residue was purified by reverse phase HPLC to afford (S)-5-((((6-(2-chloro-3-(3-chloro-2-(4-(4,5-dihydro-1H-imidazol-2-yl)-3-methoxyphenyl)pyridin-4-yl)phenyl)-2-methoxypyridin-3-yl)methyl)amino)methyl)pyrrolidin-2-one (81.9 mg, 43% yield) as a white solid (formic acid salt). MS: *m*/*z* found 631 [M+H]⁺, retention time = 2.62 min (Method A). ¹H NMR (400 MHz, Methanol-*d*₄): δ 8.73 (d, 1H), 7.91 (d, 1H), 7.86 (d, 1H), 7.75 (dd, 1H), 7.64-7.53 (m, 4H), 7.47 (dd,1H), 3.34 (d, 1H), 4.16-3.97 (m, 12H), 4.00-3.97 (m, 1H), 3.05-3.02 (m, 2H), 2.42-2.36 (m, 3H), 1.91-1.88 (m, 1H).

### Example 329: (S)-4-((4-(3-chloro-4-(2-chloro-3-(5-(4,5-dihydro-1H-imidazol-2-yl)-6-methoxypyridin-2-yl)phenyl)pyridin-2-yl)-2-methoxybenzyl)amino)-3-hydroxybutanoic acid (180)

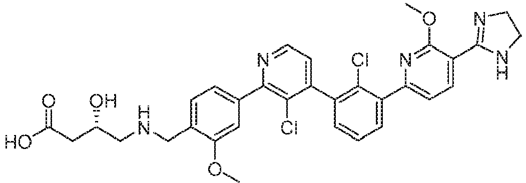

### (a) 2,3-Dichloro-4-(2-chloro-3-(5-(4,5-dihydro-1H-imidazol-2-yl)-6-methoxypyridin-2-yl)phenyl)pyridine

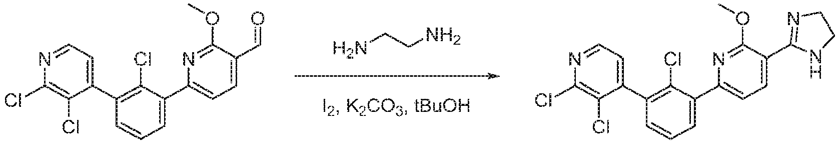

A mixture of 6-(2-chloro-3-(2,3-dichloropyridin-4-yl)phenyl)-2-methoxynicotinaldehyde (Example 1, step (a)) (2 g, 5.08 mmol) and ethane-1,2-diamine (916 mg, 15.2 mmol) in tert-butyl alcohol (15 mL) was added potassium carbonate (4.21 g, 30.4 mmol) and iodine (3.87 g, 15.2 mmol), and then the mixture was stirred at 70 °C for 3 h under N₂. Water (10 mL) was then added, and the mixture was extracted with ethyl acetate (2 x 5 mL). The combined organic phases were dried with anhydrous sodium sulfate, filtered and concentrated to give 2,3-dichloro-4-(2-chloro-3-(5-(4,5-dihydro-1H-imidazol-2-yl)-6-methoxypyridin-2-yl)phenyl)pyridine (1.8 g, 32% yield) as a yellow crude solid. MS: *m*/*z* found 433 [M+H]⁺. The crude product was used for next step without further purification.

### (b) 4-(3-Chloro-4-(2-chloro-3-(5-(4,5-dihydro-1H-imidazol-2-yl)-6-methoxypyridin-2-yl)phenyl)pyridin-2-yl)-2-methoxybenzaldehyde

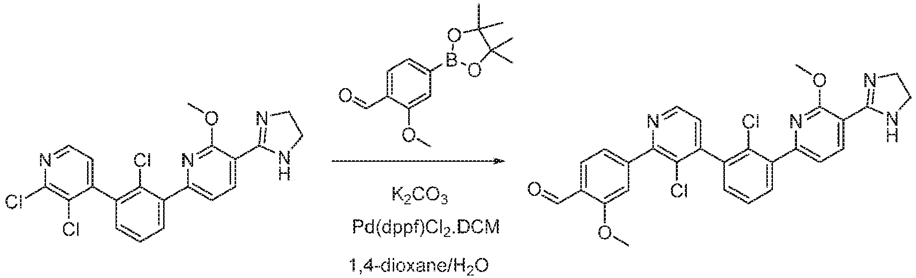

To a mixture of 2,3-dichloro-4-(2-chloro-3-(5-(4,5-dihydro-1H-imidazol-2-yl)-6-methoxypyridin-2-yl)phenyl)pyridine (400 mg, 0.922 mmol) and 2-methoxy-4-(4,4,5,5-tetramethyl-1,3,2-dioxaborolan-2-yl)benzaldehyde (435 mg, 1.66 mmol) in dioxane/ H₂O (5:1, 6 mL) was added [1,1'- bis(diphenylphosphino)ferrocene]dichloropalladium(II) complex with dichloromethane (112 mg, 0.138 mmol) and potassium carbonate (382 mg, 2.77 mmol) and then the mixture was stirred at 110 °C for 1 h under N₂. The mixture was filtered, the filtrate diluted with water (5 mL) and then extracted with ethyl acetate (2 x 5 mL). The combined organic phases were dried with anhydrous sodium sulfate, filtered and concentrated to give 4-(3-chloro-4-(2-chloro-3-(5-(4,5-dihydro-1H-imidazol-2-yl)-6-methoxypyridin-2-yl)phenyl)pyridin-2-yl)-2-methoxybenzaldehyde (500 mg, 54% yield) as a black crude solid. MS: *m*/*z* found 533 [M+H]⁺. The crude product was used for next step without further purification.

### (c) (S)-4-((4-(3-Chloro-4-(2-chloro-3-(5-(4,5-dihydro-1H-imidazol-2-yl)-6-methoxypyridin-2-yl)phenyl)pyridin-2-yl)-2-methoxybenzyl)amino)-3-hydroxybutanoic acid

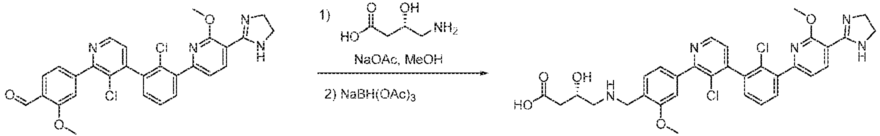

To a mixture of 4-(3-chloro-4-(2-chloro-3-(5-(4,5-dihydro-1H-imidazol-2-yl)-6-methoxypyridin-2-yl)phenyl)pyridin-2-yl)-2-methoxybenzaldehyde (150 mg, 0.281 mmol) and isopropyl (S)-4-amino-3-hydroxybutanoate (100 mg, 0.843 mmol) in methanol (2 mL) was added sodium acetate (115 mg, 1.41 mmol) and then the mixture was stirred at room temperature for 2 h under N₂. Sodium triacetoxyborohydride (119 mg, 0.562 mmol) was then added and the mixture was stirred at room temperature for 0.5 h under N₂. The mixture was filtered, the filtrate concentrated and the residue was purified by reverse phase HPLC to afford (S)-4-((4-(3-chloro-4-(2-chloro-3-(5-(4,5-dihydro-1H-imidazol-2-yl)-6-methoxypyridin-2-yl)phenyl)pyridin-2-yl)-2-methoxybenzyl)amino)-3-hydroxybutanoic acid (10 mg, 5% yield) as a white solid (formic acid salt). MS: *m*/*z* found 636 [M+H]⁺, retention time = 2.64 min (Method A). ¹H NMR (400 MHz, Methanol-*d*₄) : δ 8.65 (d, 1H), 8.28 (d, 1H), 7.78 (dd, 1H), 7.61 (m, 1H), 7.57 (d, 1H), 7.56-7.52 (m, 2H), 7.46 (d, 1H), 7.37 (s, 1H), 7.34-7.33 (m, 1H) , 4.30 (s, 2H), 4.29-4.19 (m, 4H), 4.10 (s, 4H), 3.98 (s, 3H), 3.19-3.15 (m, 1H), 3.02-2.97 (m, 1H), 2.45-2.43 (m, 2H).

### Example 330: Isopropyl (S)-4-((4-(3-chloro-4-(2-chloro-3-(5-(4,5-dihydro-1H-imidazol-2-yl)-6-methoxypyridin-2-yl)phenyl)pyridin-2-yl)-2-methoxybenzyl)amino)-3-hydroxybutanoate (209)

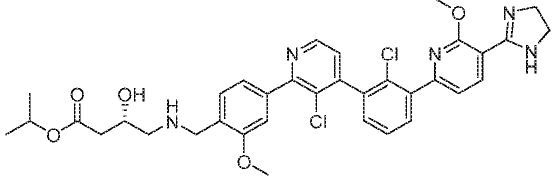

### (a) Isopropyl (S)-4-((4-(3-chloro-4-(2-chloro-3-(5-(4,5-dihydro-1H-imidazol-2-yl)-6-methoxypyridin-2-yl)phenyl)pyridin-2-yl)-2-methoxybenzyl)amino)-3-hydroxybutanoate

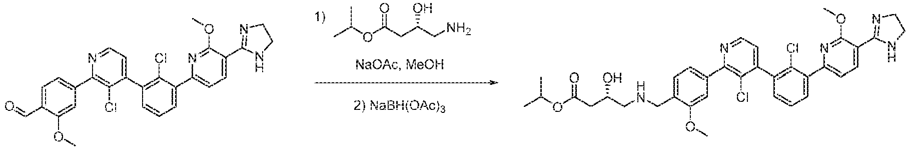

To a mixture of 4-(3-chloro-4-(2-chloro-3-(5-(4,5-dihydro-1H-imidazol-2-yl)-6-methoxypyridin-2-yl)phenyl)pyridin-2-yl)-2-methoxybenzaldehyde (Example 329, step (b)) (150 mg, 0.281 mmol) and isopropyl (S)-4-amino-3-hydroxybutanoate (135 mg, 0.843 mmol) in methanol (2 mL) was added sodium acetate (115 mg, 1.41 mmol) and the mixture was stirred at room temperature for 2 h under N₂. Sodium triacetoxyborohydride (119 mg, 0.056 mmol) and then added and the mixture was stirred at room temperature for 0.5 h under N₂. The mixture was filtered, the filtrate concentrated and the residue was purified by reverse phase HPLC to afford isopropyl (S)-4-((4-(3-chloro-4-(2-chloro-3-(5-(4,5-dihydro-1H-imidazol-2-yl)-6-methoxypyridin-2-yl)phenyl)pyridin-2-yl)-2-methoxybenzyl)amino)-3-hydroxybutanoate (16 mg, 7.93% yield) as a white solid (formic acid salt). MS: *m*/*z* found 678 [M+H]⁺. ¹H NMR (400 MHz, Methanol-*d*₄) : δ 8.66 (d, 1H), 8.28 (d, 1H), 7.78 (dd, 1H), 7.64-7.56 (m, 2H), 7.52-7.46 (m, 3H), 7.37 (s, 1H), 7.35-7.33 (m, 1H), 5.02-4.99 (m, 1H), 4.29-4.25 (m, 3H), 4.25-4.24 (m, 3H), 4.10 (s, 3H), 3.98 (s, 3H), 3.13-3.12 (m, 2H), 2.98-2.93 (m, 1H), 2.53-2.51 (m, 2H), 1.24-1.22 (m, 6H).

### Example 331: (S)-5-((((6-(3-(2-(4-(((2-acetyl-2-azaspiro[3.3]heptan-6-yl)amino)methyl)-3-methoxyphenyl)-3-chloropyridin-4-yl)-2-chlorophenyl)-2-methoxypyridin-3-yl)methyl)amino)methyl)pyrrolidin-2-one (237)

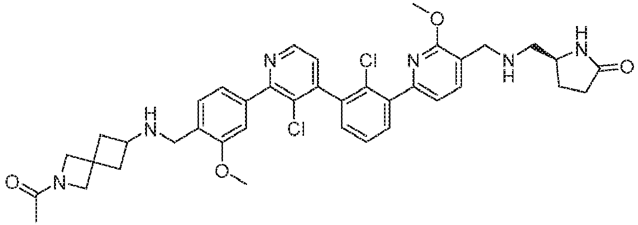

### (a) tert-butyl (S)-((6-(3-(2-(4-(((2-acetyl-2-azaspiro[3.3]heptan-6-yl)amino)methyl)-3-methoxyphenyl)-3-chloropyridin-4-yl)-2-chlorophenyl)-2-methoxypyridin-3-yl)methyl)((5-oxopyrrolidin-2-yl)methyl)carbamate

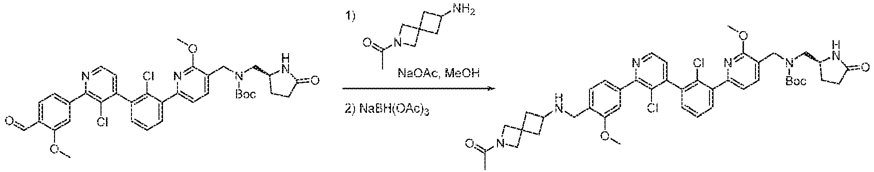

To a mixture of tert-butyl (S)-((6-(2-chloro-3-(3-chloro-2-(4-formyl-3-methoxyphenyl)pyridin-4-yl)phenyl)-2-methoxypyridin-3-yl)methyl)((5-oxopyrrolidin-2-yl)methyl)carbamate (Example 80, step (a)) (200 mg, 289 umol) and 1-(6-amino-2-azaspiro[3.3]heptan-2-yl)ethan-1-one (82.7 mg, 433 umol) in methanol (8 mL) was added sodium acetate (71.1 mg, 867 umol) and the mixture was stirred at room temperature for 12 h under N₂. Sodium triacetoxyborohydride (183 mg, 867umol) was then added and the mixture was stirred at room temperature for 0.5 h under N₂. The mixture was filtered, the filtrate concentrated and the residue purified by normal phase SiO₂ chromatography (15-25% ethyl acetate / methanol) to afford tert-butyl (S)-((6-(3-(2-(4-(((2-acetyl-2-azaspiro[3.3]heptan-6-yl)amino)methyl)-3-methoxyphenyl)-3-chloropyridin-4-yl)-2-chlorophenyl)-2-methoxypyridin-3-yl)methyl)((5-oxopyrrolidin-2-yl)methyl)carbamate (150 mg, 41% yield) as a white solid. MS: *m*/*z* found 829 [M+H]⁺.

### (b) (S)-5-((((6-(3-(2-(4-(((2-acetyl-2-azaspiro[3.3]heptan-6-yl)amino)methyl)-3-methoxyphenyl)-3-chloropyridin-4-yl)-2-chlorophenyl)-2-methoxypyridin-3-yl)methyl)amino)methyl)pyrrolidin-2-one

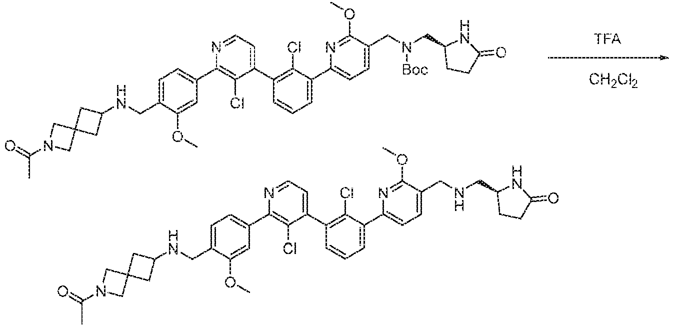

To a mixture of tert-butyl (S)-((6-(3-(2-(4-(((2-acetyl-2-azaspiro[3.3]heptan-6-yl)amino)methyl)-3-methoxyphenyl)-3-chloropyridin-4-yl)-2-chlorophenyl)-2-methoxypyridin-3-yl)methyl)((5-oxopyrrolidin-2-yl)methyl)carbamate (140 mg, 168 umol) in dichloromethane (3 mL) was added trifluoroacetic acid (4.62 mg, 40.5 umol) and the mixture stirred at room temperature for 0.5 h under N₂. The mixture was filtered, the filtrate concentrated and the residue was purified by reverse phase HPLC to afford (S)-5-((((6-(3-(2-(4-(((2-acetyl-2-azaspiro[3.3]heptan-6-yl)amino)methyl)-3-methoxyphenyl)-3-chloropyridin-4-yl)-2-chlorophenyl)-2-methoxypyridin-3-yl)methyl)amino)methyl)pyrrolidin-2-one (16 mg 12% yield) as a white solid (formic acid salt). MS: *m*/*z* found 729 [M+H]⁺, retention time = 2.60 min (Method A). ¹H NMR (400 MHz, Methanol-*d*₄) : δ 8.67 (d, 1H), 7.81 (d, 1H), 7.73 (dd, 1H), 7.58 (t, 1H), 7.52-7.49 (m, 2H), 7.43-7.37 (m, 2H), 7.36 (d, 1H), 7.30 (d, 1H), 4.30 (s, 1H), 4.20-4.18 (m, 3H), 4.06-3.76 (m, 10H), 3.77-3.76 (m, 1H), 3.32-3.26 (m, 3H), 2.91-2.71 (m, 2H), 2.70-2.64 (m, 2H), 2.45-2.33 (m, 4H), 1.86 (d, 3H).

### Example 332: (S)-5-((((6-(2-chloro-3-(3-chloro-2-(3-methoxy-5-(((((S)-5-oxopyrrolidin-2-yl)methyl)amino)methyl)phenyl)pyridin-4-yl)phenyl)-2-methoxypyridin-3-yl)methyl)amino)methyl)pyrrolidin-2-one (153)

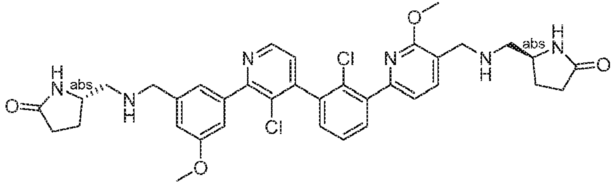

### (a) 6-(2-Chloro-3-(3-chloro-2-(3-formyl-5-methoxyphenyl)pyridin-4-yl)phenyl)-2-methoxynicotinaldehyde

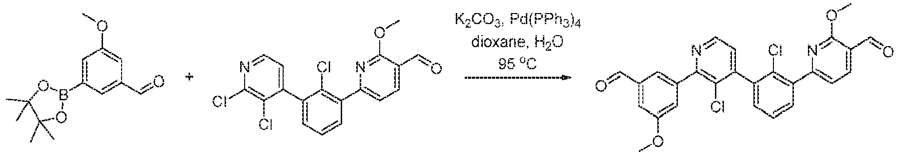

Following Suzuki Coupling Procedure A from 6-(2-chloro-3-(2,3-dichloropyridin-4-yl)phenyl)-2-methoxynicotinaldehyde (150 mg, 0.38 mmol) and 3-methoxy-5-(4,4,5,5-tetramethyl-1,3,2-dioxaborolan-2-yl)benzaldehyde (100 mg, 0.38 mmol) afforded 6-(2-chloro-3-(3-chloro-2-(3-formyl-5-methoxyphenyl)pyridin-4-yl)phenyl)-2-methoxynicotinaldehyde in 85% yield. LCMS: m/z found 493.1 [M+H]⁺, retention time = 1.10 min (Method B).

### (b) (S)-5-((((6-(2-chloro-3-(3-chloro-2-(3-methoxy-5-(((((S)-5-oxopyrrolidin-2-yl)methyl)amino)methyl)phenyl)pyridin-4-yl)phenyl)-2-methoxypyridin-3-yl)methyl)amino)methyl)pyrrolidin-2-one

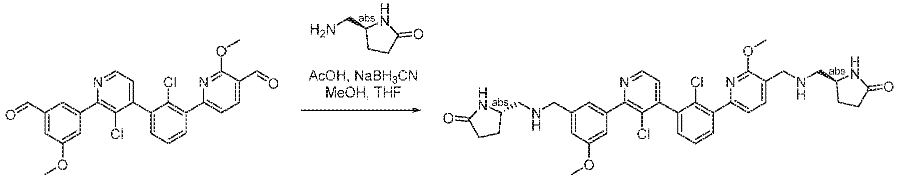

This compound was prepared as the formic acid salt following Reductive Amination Procedure A from 6-(2-chloro-3-(3-chloro-2-(3-formyl-5-methoxyphenyl)pyridin-4-yl)phenyl)-2-methoxynicotinaldehyde (30 mg, 0.06 mmol) and (5S)-5-(aminomethyl)pyrrolidin-2-one (28 mg, 0.24 mmol). 68% yield. LCMS: m/z found 689.2 [M+H]⁺, retention time = 1.65 min (Method D). ¹H NMR (400 MHz, Methanol-*d*₄): δ 8.64 (dd, 1H), 8.46 - 8.39 (m, 2H), 7.81 (d, 1H), 7.72 (dt, 1H), 7.56 (td, 1H), 7.46 (dd, 1H), 7.42 (dd, 1H), 7.34 - 7.26 (m, 2H), 7.22 (d, 1H), 7.15 (s, 1H), 4.09 (d, 2H), 4.05 (s, 3H), 4.04 - 4.01 (m, 2H), 3.97 - 3.89 (m, 2H), 3.88 (s, 3H), 3.01 - 2.84 (m, 4H), 2.42 - 2.25 (m, 6H), 1.92 - 1.77 (m, 2H).

### Example 333: 1-(4-(((6-(3-(2-(3-(((1-acetylpiperidin-4-yl)amino)methyl)-5-methoxyphenyl)-3-chloropyridin-4-yl)-2-chlorophenyl)-2-methoxypyridin-3-yl)methyl)amino)piperidin-1-yl)ethan-1-one

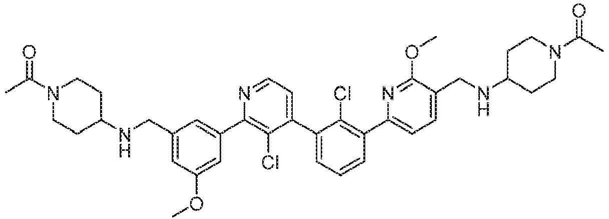

This compound was prepared as the formic acid salt following Reductive Amination Procedure A from 6-(2-chloro-3-(3-chloro-2-(3-formyl-5-methoxyphenyl)pyridin-4-yl)phenyl)-2-methoxynicotinaldehyde (Example 332, step (a)) (30 mg, 0.06 mmol) and 1-(4-amino-1-piperidyl)ethanone (35 mg, 0.24 mmol). 80% yield. LCMS: m/z found 745.3 [M+H]⁺, retention time = 1.71 min (Method D). ¹H NMR (400 MHz, Methanol-*d*₄): δ 8.64 (d, 1H), 8.55 - 8.48 (m, 2H), 7.82 (d, 1H), 7.71 (d, 1H), 7.56 (t, 1H), 7.45 (dd, 1H), 7.42 (d, 1H), 7.32 - 7.27 (m, 2H), 7.20 (s, 1H), 7.15 (s, 1H), 4.55 (d, 2H), 4.08 (s, 2H), 4.06 - 4.02 (m, 5H), 4.02 - 3.93 (m, 2H), 3.88 (s, 3H), 3.22 - 3.00 (m, 4H), 2.78 - 2.57 (m, 2H), 2.21 - 2.00 (m, 10H), 1.56 - 1.29 (m, 4H).

### Example 334: 1-(6-(2-chloro-3-(3-chloro-2-(3-methoxy-5-((methylamino)methyl)phenyl)pyridin-4-yl)phenyl)-2-methoxypyridin-3-yl)-N-methylmethanamine (155)

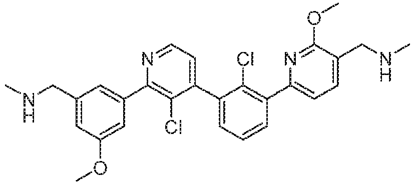

This compound was prepared as the formic acid salt following Reductive Amination Procedure A from 6-(2-chloro-3-(3-chloro-2-(3-formyl-5-methoxyphenyl)pyridin-4-yl)phenyl)-2-methoxynicotinaldehyde (Example 332, step (a)) (30 mg, 0.06 mmol) and methanamine solution (33% wt in ethanol, 0.24 mmol). 35% yield. LCMS: m/z found 523.2 [M+H]⁺, retention time = 1.71 min (Method D). ¹H NMR (400 MHz, Methanol-*d*₄): δ 8.66 (dt, 1H), 8.53 (s, 2H), 7.86 (dd, 1H), 7.75 - 7.69 (m, 1H), 7.58 (ddd, 1H), 7.50 - 7.40 (m, 2H), 7.37 - 7.31 (m, 2H), 7.29 (d, 1H), 7.18 (s, 1H), 4.20 (s, 4H), 4.08 (s, 3H), 3.90 (s, 3H), 2.73 (s, 3H), 2.71 (s, 3H).

### Example 335: 2-(((6-(2-chloro-3-(3-chloro-2-(3-(((2-hydroxyethyl)amino)methyl)-5-methoxyphenyl)pyridin-4-yl)phenyl)-2-methoxypyridin-3-yl)methyl)amino)ethan-1-ol (156)

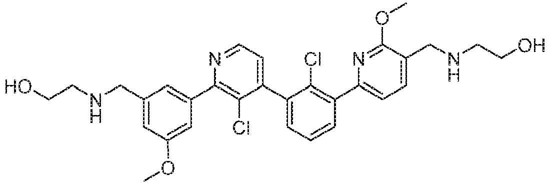

This compound was prepared as the formic acid salt following Reductive Amination Procedure A from 6-(2-chloro-3-(3-chloro-2-(3-formyl-5-methoxyphenyl)pyridin-4-yl)phenyl)-2-methoxynicotinaldehyde (Example 332, step (a)) (30 mg, 0.06 mmol) and 2-aminoethanol (15 mg, 0.24 mmol). 60% yield. LCMS: m/z found 583.2 [M+H]⁺, retention time = 1.63 min (Method D). ¹H NMR (400 MHz, Methanol-*d*₄): δ 8.66 (dt, 1H), 8.54 - 8.47 (m, 2H), 7.89 - 7.83 (m, 1H), 7.76 - 7.69 (m, 1H), 7.58 (ddd, 1H), 7.47 (dt, 1H), 7.44 (d, 1H), 7.37 - 7.31 (m, 2H), 7.29 (d, 1H), 7.20 (s, 1H), 4.24 (s, 2H), 4.22 (s, 2H), 4.08 (s, 3H), 3.90 (s, 3H), 3.81 (ddt, 4H), 3.11 (t, 4H).

### Example 336: (S)-1-(((6-(2-chloro-3-(3-chloro-2-(3-((((S)-2-hydroxypropyl)amino)methyl)-5-methoxyphenyl)pyridin-4-yl)phenyl)-2-methoxypyridin-3-yl)methyl)amino)propan-2-ol (157)

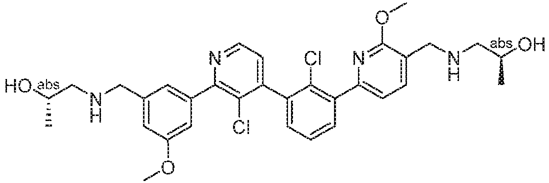

This compound was prepared as the formic acid salt following Reductive Amination Procedure A from 6-(2-chloro-3-(3-chloro-2-(3-formyl-5-methoxyphenyl)pyridin-4-yl)phenyl)-2-methoxynicotinaldehyde (Example 332, step (a)) (30 mg, 0.06 mmol) and (2S)-1-aminopropan-2-ol (18 mg, 0.24 mmol). 73% yield. LCMS: m/z found 611.2 [M+H]⁺, retention time = 1.72 min (Method D). ¹H NMR (400 MHz, Methanol-*d*₄): δ 8.66 (dd, 1H), 8.55 - 8.49 (m, 2H), 7.85 (d, 1H), 7.73 (dd, 1H), 7.62 - 7.55 (m, 1H), 7.50 - 7.45 (m, 1H), 7.44 (dd, 1H), 7.37 - 7.31 (m, 2H), 7.28 (d, 1H), 7.20 (d, 1H), 4.26 - 4.14 (m, 4H), 4.07 (s, 3H), 4.05 - 3.96 (m, 2H), 3.90 (s, 3H), 3.01 (d, 2H), 2.82 (dd, 2H), 1.23 (d, 3H), 1.20 (d, 3H).

### Example 337: (S)-4-((3-(3-chloro-4-(2-chloro-3-(6-methoxy-5-((methylamino)methyl)pyridin-2-yl)phenyl)pyridin-2-yl)-5-methoxybenzyl)amino)-3-hydroxybutanoic acid (169)

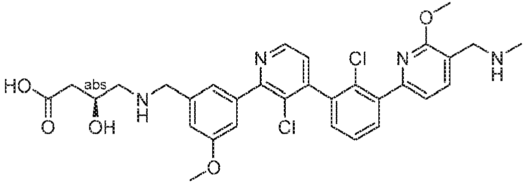

### (a) tert-Butyl ((6-(2-chloro-3-(3-chloro-2-(3-formyl-5-methoxyphenyl)pyridin-4-yl)phenyl)-2-methoxypyridin-3-yl)methyl)(methyl)carbamate

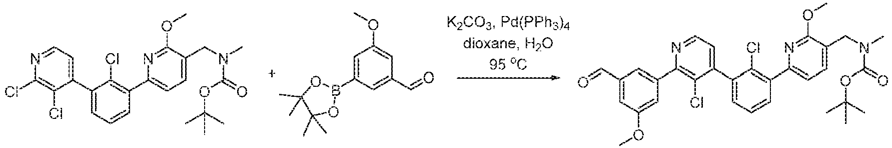

Following Suzuki Coupling Procedure A from tert-butyl ((6-(2-chloro-3-(2,3-dichloropyridin-4-yl)phenyl)-2-methoxypyridin-3-yl)methyl)(methyl)carbamate (200 mg, 0.39 mmol) and 3-methoxy-5-(4,4,5,5-tetramethyl-1,3,2-dioxaborolan-2-yl)benzaldehyde (103 mg, 0.39 mmol) gave tert-butyl ((6-(2-chloro-3-(3-chloro-2-(3-formyl-5-methoxyphenyl)pyridin-4-yl)phenyl)-2-methoxypyridin-3-yl)methyl)(methyl)carbamate with 87% yield. LCMS: m/z found 608.2 [M+H]⁺, retention time = 1.21 min (Method B).

### (b) (S)-4-((3-(3-chloro-4-(2-chloro-3-(6-methoxy-5-((methylamino)methyl)pyridin-2-yl)phenyl)pyridin-2-yl)-5-methoxybenzyl)amino)-3-hydroxybutanoic acid

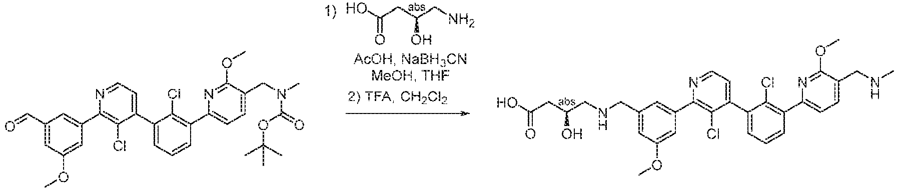

Reductive Amination Procedure B from tert-butyl ((6-(2-chloro-3-(3-chloro-2-(3-formyl-5-methoxyphenyl)pyridin-4-yl)phenyl)-2-methoxypyridin-3-yl)methyl)(methyl)carbamate (40 mg, 0.07 mmol) and (3S)-4-amino-3-hydroxy-butanoic acid (16 mg, 0.13 mmol) gave (3S)-4-[[3-[4-[3-[5-[[tert-butoxycarbonyl(methyl)amino]methyl]-6-methoxy-2-pyridyl]-2-chloro-phenyl]-3-chloro-2-pyridyl]-5-methoxy-phenyl]methylamino]-3-hydroxy-butanoic acid as crude, LCMS: m/z found 711.2 [M+H]⁺, which was deprotected and purified by General Boc Deprotection Procedure to afford the final product as formic acid salt. 30% yield over two steps. LCMS: m/z found 611.2 [M+H]⁺, retention time = 1.68 min (Method D). ¹H NMR (400 MHz, Methanol-*d*₄): δ 8.65 (d, 1H), 8.54 (s, 2H), 7.84 (d, 1H), 7.76 - 7.67 (m, 1H), 7.58 (s, 1H), 7.51 - 7.41 (m, 2H), 7.38 - 7.32 (m, 2H), 7.27 (s, 1H), 7.20 (s, 1H), 4.23 (s, 2H), 4.19 - 4.12 (m, 3H), 4.12 - 4.02 (m, 3H), 3.90 (s, 3H), 3.17 - 3.06 (m, 1H), 3.01 - 2.91 (m, 1H), 2.70 (s, 3H), 2.46 - 2.35 (m, 2H).

### Example 338: 3-((3-(3-Chloro-4-(2-chloro-3-(6-methoxy-5-((methylamino)methyl)pyridin-2-yl)phenyl)pyridin-2-yl)-5-methoxybenzyl)amino)propanoic acid (170)

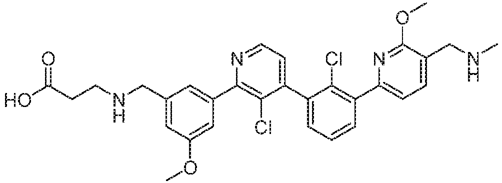

Reductive Amination Procedure B from tert-butyl ((6-(2-chloro-3-(3-chloro-2-(3-formyl-5-methoxyphenyl)pyridin-4-yl)phenyl)-2-methoxypyridin-3-yl)methyl)(methyl)carbamate (Example 337, step (a)) (40 mg, 0.07 mmol) and 3-aminopropanoic acid (12 mg, 0.13 mmol) gave 3-[[3-[4-[3-[5-[[tert-butoxycarbonyl(methyl)amino]methyl]-6-methoxy-2-pyridyl]-2-chloro-phenyl]-3-chloro-2-pyridyl]-5-methoxy-phenyl]methylamino]propanoic acid as crude, LCMS: m/z found 681.2 [M+H]⁺, which was deprotected and purified by General Boc Deprotection Procedure to afford the final product as formic acid salt. 62% yield over two steps. LCMS: m/z found 581.2 [M+H]⁺, retention time = 1.73 min (Method D). ¹H NMR (400 MHz, Methanol-*d*₄): δ 8.65 (dd, 1H), 8.54 (s, 2H), 7.84 (d, 1H), 7.72 (dd, 1H), 7.57 (t, 1H), 7.48 - 7.40 (m, 2H), 7.37 (d, 1H), 7.33 (d, 1H), 7.28 (d, 1H), 7.20 (d, 1H), 4.26 (s, 2H), 4.17 - 4.11 (m, 2H), 4.07 (s, 3H), 3.90 (s, 3H), 3.19 (t, 2H), 2.70 (s, 3H), 2.50 (t, 2H).

### Example 339: (S)-1-(3-(3-chloro-4-(2-chloro-3-(6-methoxy-5-((methylamino)methyl)pyridin-2-yl)phenyl)pyridin-2-yl)-5-methoxybenzyl)pyrrolidine-3-carboxylic acid (171)

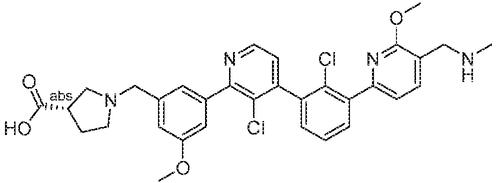

Reductive Amination Procedure B from tert-butyl ((6-(2-chloro-3-(3-chloro-2-(3-formyl-5-methoxyphenyl)pyridin-4-yl)phenyl)-2-methoxypyridin-3-yl)methyl)(methyl)carbamate (Example 337, step (a)) (40 mg, 0.07 mmol) and (3S)-pyrrolidine-3-carboxylic acid (15 mg, 0.13 mmol) gave (3S)-1-[[3-[4-[3-[5-[[tert-butoxycarbonyl(methyl)amino]methyl]-6-methoxy-2-pyridyl]-2-chloro-phenyl]-3-chloro-2-pyridyl]-5-methoxy-phenyl]methyl]pyrrolidine-3-carboxylic acid as crude, LCMS: m/z found 707.2 [M+H]⁺, which was deprotected and purified by General Boc Deprotection Procedure to afford the final product as formic acid salt. 63% yield over two steps. LCMS: m/z found 607.2 [M+H]⁺, retention time = 1.73 min (Method D). ¹H NMR (400 MHz, Methanol-*d*₄): δ 8.64 (d, 1H), 8.54 (s, 2H), 7.85 (d, 1H), 7.72 (dd, 1H), 7.57 (t, 1H), 7.48 - 7.41 (m, 2H), 7.38 (s, 1H), 7.34 (d, 1H), 7.27 (d, 1H), 7.20 (t, 1H), 4.35 - 4.21 (m, 2H), 4.18 (s, 2H), 4.08 (s, 3H), 3.90 (s, 3H), 3.42 - 3.34 (m, 2H), 3.25 (t, 2H), 3.05 (p, 1H), 2.72 (s, 3H), 2.35 - 2.15 (m, 2H).

### Example 340: 2-(1-(3-(3-chloro-4-(2-chloro-3-(6-methoxy-5-((methylamino)methyl)pyridin-2-yl)phenyl)pyridin-2-yl)-5-methoxybenzyl)azetidin-3-yl)acetic acid (172)

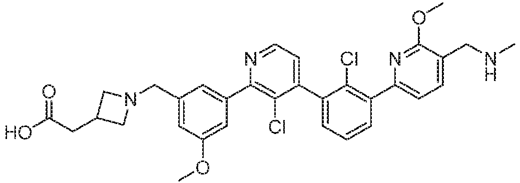

Reductive Amination Procedure B from tert-butyl ((6-(2-chloro-3-(3-chloro-2-(3-formyl-5-methoxyphenyl)pyridin-4-yl)phenyl)-2-methoxypyridin-3-yl)methyl)(methyl)carbamate (Example 337, step (a)) (40 mg, 0.07 mmol) and 2-(azetidin-3-yl)acetic acid (15 mg, 0.13 mmol) gave 2-[1-[[3-[4-[3-[5-[[tert-butoxycarbonyl(methyl)amino]methyl]-6-methoxy-2-pyridyl]-2-chloro-phenyl]-3-chloro-2-pyridyl]-5-methoxy-phenyl]methyl]azetidin-3-yl]acetic acid as crude, LCMS: m/z found 707.2 [M+H]⁺, which was deprotected and purified by General Boc Deprotection Procedure to afford the final product as formic acid salt. 73% yield over two steps. LCMS: m/z found 607.2 [M+H]⁺, retention time = 1.75 min (Method D). ¹H NMR (400 MHz, Methanol-*d*₄): δ 8.62 (d, 1H), 8.54 (s, 2H), 7.84 (d, 1H), 7.72 (dd, 1H), 7.57 (t, 1H), 7.48 - 7.39 (m, 2H), 7.34 (d, 1H), 7.23 (s, 1H), 7.15 - 7.04 (m, 2H), 4.44 (dd, 1H), 4.15 (s, 2H), 4.12 - 4.08 (m, 1H), 4.08 (s, 3H), 3.91 - 3.81 (m, 5H), 2.83 - 2.58 (m, 7H), 2.34 (dd, 1H).

### Example 341: 2-(3-(3-Chloro-4-(2-chloro-3-(6-methoxy-5-((methylamino)methyl)pyridin-2-yl)phenyl)pyridin-2-yl)-5-methoxybenzyl)-2-azaspiro[3.3]heptane-6-carboxylic acid (173)

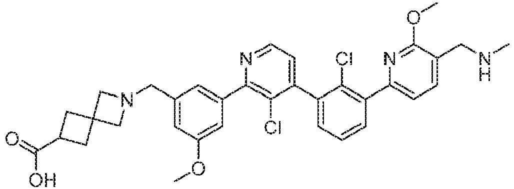

Reductive Amination Procedure B from tert-butyl ((6-(2-chloro-3-(3-chloro-2-(3-formyl-5-methoxyphenyl)pyridin-4-yl)phenyl)-2-methoxypyridin-3-yl)methyl)(methyl)carbamate (Example 337, step (a)) (25 mg, 0.04 mmol) and 2-azaspiro[3.3]heptane-6-carboxylic acid (12 mg, 0.08 mmol) gave 2-[[3-[4-[3-[5-[[tert-butoxycarbonyl(methyl)amino]methyl]-6-methoxy-2-pyridyl]-2-chloro-phenyl]-3-chloro-2-pyridyl]-5-methoxy-phenyl]methyl]-2-azaspiro[3.3]heptane-6-carboxylic acid as crude, LCMS: m/z found 733.3 [M+H]⁺, which was deprotected and purified by General Boc Deprotection Procedure to afford the final product as formic acid salt. 35% yield over two steps. LCMS: m/z found 633.2 [M+H]⁺, retention time = 1.78 min (Method D). ¹H NMR (400 MHz, Methanol-*d*₄): δ 8.67 - 8.61 (m, 1H), 8.47 (s, 2H), 7.88 (d, 1H), 7.76 - 7.69 (m, 1H), 7.58 (t, 1H), 7.46 (dd, 2H), 7.36 (d, 1H), 7.31 (d, 1H), 7.27 - 7.06 (m, 2H), 4.39 (s, 1H), 4.29 (s, 1H), 4.25 (s, 2H), 4.11 - 4.03 (m, 5H), 3.96 - 3.82 (m, 4H), 2.92 - 2.81 (m, 1H), 2.79 (s, 1H), 2.77 (s, 3H), 2.51 - 2.37 (m, 2H), 2.32 (t, 1H), 1.85 (d, 1H).

### Example 342: 3-((3-(3-chloro-4-(2-chloro-3-(6-methoxy-5-((methylamino)methyl)pyridin-2-yl)phenyl)pyridin-2-yl)-5-methoxybenzyl)amino)bicyclo[1.1.1]pentane-1-carboxylic acid (174)

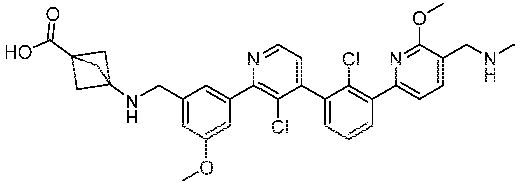

Reductive Amination Procedure B from tert-butyl ((6-(2-chloro-3-(3-chloro-2-(3-formyl-5-methoxyphenyl)pyridin-4-yl)phenyl)-2-methoxypyridin-3-yl)methyl)(methyl)carbamate (Example 337, step (a)) (20 mg, 0.03 mmol) and 3-aminobicyclo[1.1.1]pentane-1-carboxylic acid (8 mg, 0.07 mmol) gave 3-[[3-[4-[3-[5-[[tert-butoxycarbonyl(methyl)amino]methyl]-6-methoxy-2-pyridyl]-2-chloro-phenyl]-3-chloro-2-pyridyl]-5-methoxy-phenyl]methylamino]bicyclo[1.1.1]pentane-1-carboxylic acid as crude, LCMS: m/z found 719.2 [M+H]⁺, which was deprotected and purified by General Boc Deprotection Procedure to afford the final product as formic acid salt. 55% yield over two steps. LCMS: m/z found 619.2 [M+H]⁺, retention time = 1.76 min (Method D). ¹H NMR (400 MHz, Methanol-*d*₄): δ 8.61 (d, 1H), 8.52 (s, 2H), 7.86 (d, 1H), 7.72 (d, 1H), 7.57 (t, 1H), 7.47 - 7.40 (m, 2H), 7.36 (d, 1H), 7.25 (d, 1H), 7.10 (s, 2H), 4.23 (s, 2H), 4.09 (s, 3H), 3.90 - 3.82 (m, 5H), 2.76 (s, 3H), 2.00 (s, 6H).

### Example 343: 3-(((3-(3-chloro-4-(2-chloro-3-(6-methoxy-5-((methylamino)methyl)pyridin-2-yl)phenyl)pyridin-2-yl)-5-methoxybenzyl)amino)methyl)bicyclo[1.1.1]pentane-1-carboxylic acid (175)

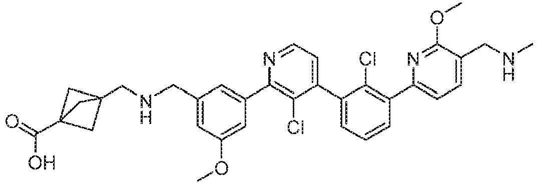

Reductive Amination Procedure B from tert-butyl ((6-(2-chloro-3-(3-chloro-2-(3-formyl-5-methoxyphenyl)pyridin-4-yl)phenyl)-2-methoxypyridin-3-yl)methyl)(methyl)carbamate (Example 337, step (a)) (20 mg, 0.03 mmol) and 3-(aminomethyl)bicyclo[1.1.1]pentane-1-carboxylic acid (9 mg, 0.07 mmol) gave 3-[[[3-[4-[3-[5-[[tert-butoxycarbonyl(methyl)amino]methyl]-6-methoxy-2-pyridyl]-2-chloro-phenyl]-3-chloro-2-pyridyl]-5-methoxy-phenyl]methylamino]methyl]bicyclo[1.1.1]pentane-1-carboxylic acid as crude, LCMS: m/z found 733.3 [M+H]⁺, which was deprotected and purified by General Boc Deprotection Procedure to afford the final product as formic acid salt. 43% yield over two steps. LCMS: m/z found 633.2 [M+H]⁺, retention time = 1.80 min (Method D). ¹H NMR (400 MHz, Methanol-*d*₄): δ 8.65 (d, 1H), 8.54 (s, 2H), 7.84 (d, 1H), 7.76 - 7.68 (m, 1H), 7.57 (t, 1H), 7.49 - 7.42 (m, 2H), 7.31 (d, 2H), 7.24 (d, 1H), 7.14 (s, 1H), 4.13 (s, 4H), 4.07 (s, 3H), 3.89 (s, 3H), 3.03 - 2.94 (m, 2H), 2.69 (s, 3H), 1.95 (s, 6H).

### Example 344: 1-(6-(3-(3-Chloro-2-(3-methoxy-4-((methylamino)methyl)phenyl)pyridin-4-yl)-2-methylphenyl)-2-methoxypyridin-3-yl)-N-methylmethanamine (395)

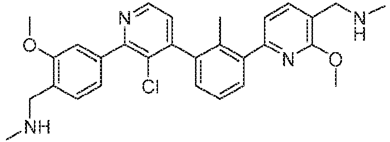

### (a) 2-(3-Bromo-2-methylphenyl)-4,4,5,5-tetramethyl-1,3,2-dioxaborolane

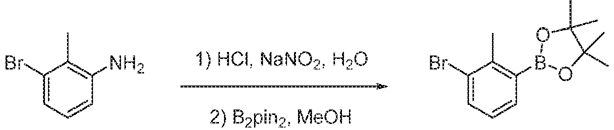

To a solution of 3-bromo-2-methylaniline (10 g, 53.7 mmol) and HCl (1mol/L, 5mL) in MeOH (150 mL) was added sodium nitrite (4.08 g, 59.12 mmol) in H₂O (30 mL) dropwise at 0 °C. The reaction mixture was stirred at 0 °C for 0.5 h. Bis(pinacolato)diborane (40.9 g, 161 mmol) in methanol (50 mL) was then added to the mixture at 0 °C, and the mixture stirred at room temperature for 1 h. Water (20 mL) was added, and the mixture extracted with ethyl acetate (2 x10 mL). The combined organic phases were dried over anhydrous sulfate, filtered and concentrated under reduced pressure. The residue was purified by normal phase SiO₂ chromatography (100 % DCM) to afford 2-(3-bromo-2-methylphenyl)-4,4,5,5-tetramethyl-1,3,2-dioxaborolane (4 g, 10% yield) as a yellow oil. MS: *m*/*z* found 297 and 299 [M+H]⁺.

### (b) 6-(3-Bromo-2-methylphenyl)-2-methoxynicotinaldehyde

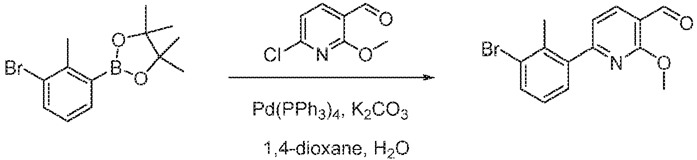

To a mixture of 2-(3-bromo-2-methylphenyl)-4,4,5,5-tetramethyl-1,3,2-dioxaborolane (1.8 g, 6.06 mmol) and 6-chloro-2-methoxynicotinaldehyde (866 mg, 5.05 mmol) in dioxane / H₂O (6:1, 35 mL) was added tetrakis(triphenylphosphine)palladium(0) (291 mg, 0.252 mmol) and potassium carbonate (2.09 g, 15.1 mmol), then the mixture was stirred at 95 °C for 3 h under N₂. The mixture was filtered, the filtrate concentrated, and the residue purified by normal phase SiO₂ chromatography (0-25% ethyl acetate/petroleum ether) to afford 6-(3-bromo-2-methylphenyl)-2-methoxynicotinaldehyde (2.8g, 65% yield) as a yellow solid. MS: *m*/*z* found 306 and 308 [M+H]⁺.

### (c) 6-(3-(2,3-Dichloropyridin-4-yl)-2-methylphenyl)-2-methoxynicotinaldehyde

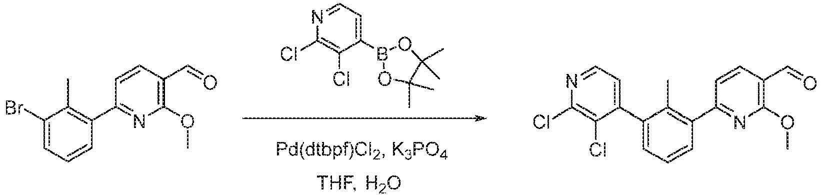

To a mixture of 2,3-dichloro-4-(4,4,5,5-tetramethyl-1,3,2-dioxaborolan-2-yl)pyridine (2.68 g, 9.78 mmol) and 6-(3-bromo-2-methylphenyl)-2-methoxynicotinaldehyde (1.5 g, 4.90 mmol) in H₂O / THF (1:5, 60 mL) was added [1,1'-bis(di-tert-butylphosphino)ferrocene]dichloropalladium(II) (159 mg, 0.244 mmol), potassium phosphate (3.12 g, 14.7 mmol), then the mixture was stirred at 80 °C for 4 h under N₂. Water (20 mL) was added, and the mixture extracted with ethyl acetate (2 x10 mL). The combined organic phases were dried over anhydrous sulfate, filtered and concentrated under reduced pressure. The residue was purified by normal phase SiO₂ chromatography (20-30% ethyl acetate/petroleum ether) to afford 6-(3-(2,3-dichloropyridin-4-yl)-2-methylphenyl)-2-methoxynicotinaldehyde (1.8 g, 34% yield) as a yellow solid. MS: *m*/*z* found 373 [M+H]⁺.

### (d) 6-(3-(3-Chloro-2-(4-formyl-3-methoxyphenyl)pyridin-4-yl)-2-methylphenyl)-2-methoxynicotinaldehyde

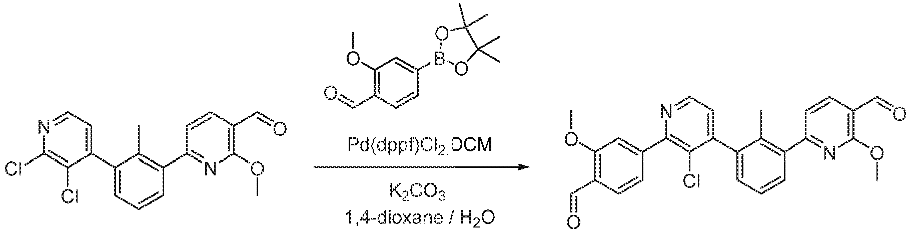

To a mixture of 6-(3-(2,3-dichloropyridin-4-yl)-2-methylphenyl)-2-methoxynicotinaldehyde (1.7 g, 4.55 mmol) and 2-methoxy-4-(4,4,5,5-tetramethyl-1,3,2-dioxaborolan-2-yl)benzaldehyde (Example 77, step (a)) (1.79 g, 6.83 mmol) in dioxane / H₂O (5:1, 48 mL) was added [1,1'- bis(diphenylphosphino)ferrocene]dichloropalladium(II) complex with dichloromethane (185 mg, 227 umol) and potassium carbonate (1.89 g, 13.6 mmol), and then the mixture stirred at 95 °C for 4 h under N₂. Water (20 mL) was added and then the mixture extracted with ethyl acetate (2 x50 mL). The combined organic phases were dried over anhydrous sulfate, filtered and concentrated under reduced pressure. The residue was purified by normal phase SiO₂ chromatography (20-30% ethyl acetate/petroleum ether) to afford 6-(3-(3-chloro-2-(4-formyl-3-methoxyphenyl)pyridin-4-yl)-2-methylphenyl)-2-methoxynicotinaldehyde (800 mg, 19.4% yield) as a yellow solid. MS: *m*/*z* found 473 [M+H]⁺.

### (e) 1-(6-(3-(3-chloro-2-(3-methoxy-4-((methylamino)methyl)phenyl)pyridin-4-yl)-2-methylphenyl)-2-methoxypyridin-3-yl)-N-methylmethanamine

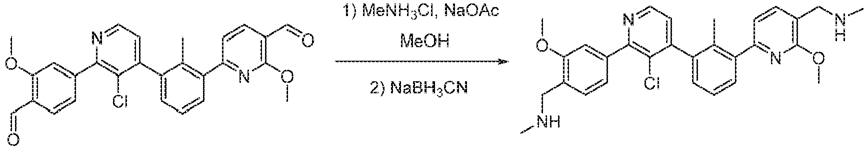

To a mixture of 6-(3-(3-chloro-2-(4-formyl-3-methoxyphenyl)pyridin-4-yl)-2-methylphenyl)-2-methoxynicotinaldehyde (150 mg, 317 umol) and methylammonium chloride (64.2 mg, 0.951 mmol) in MeOH (1 mL) was added sodium acetate (156 mg, 1.90 mmol) and then the mixture stirred at room temperature for 11.5 h under N₂. Sodium triacetoxyborohydride (69.7 mg, 1.11 mmol) was then added and the mixture stirred at room temperature for 0.5 h under N₂. The mixture was filtered, the filtrate concentrated and the residue was purified by reverse phase HPLC to afford 1-(6-(3-(3-chloro-2-(3-methoxy-4-((methylamino)methyl)phenyl)pyridin-4-yl)-2-methylphenyl)-2-methoxypyridin-3-yl)-N-methylmethanamine (36.3 mg, 20% yield) as a white solid (formic acid salt). MS: *m*/*z* found 503 [M+H]⁺, retention time = 2.56 min (Method A). ¹H NMR (400 MHz, Methanol-*d*₄): δ 8.66 (d, 1H), 7.87(d, 1H), 7.55-7.51(m, 2H), 7.46-7.44 (m, 3H), 7.37 (dd, 1H), 7.28 (dd, 1H), 7.21 (d, 1H), 4.27 (s, 2H), 4.21 (s, 2H), 4.07(s, 3H), 4.01 (s, 3H), 2.75 (s, 6H), 2.19 (s, 3H).

### Example 345: 1-(4-(((6-(3-(2-(4-(((1-acetylpiperidin-4-yl)amino)methyl)-3-methoxyphenyl)-3-chloropyridin-4-yl)-2-methylphenyl)-2-methoxypyridin-3-yl)methyl)amino)piperidin-1-yl)ethan-1-one (399)

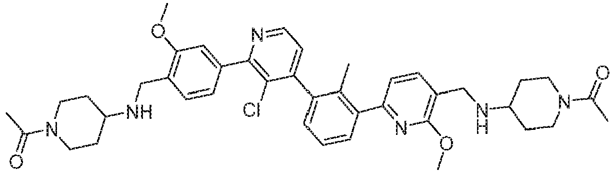

To a mixture of 6-(3-(3-chloro-2-(4-formyl-3-methoxyphenyl)pyridin-4-yl)-2-methylphenyl)-2-methoxynicotinaldehyde (Example 344, step (d)) (150 mg, 0.317 mmol) and 1-(4-aminopiperidin-1-yl)ethan-1-one (135 mg, 951 umol) in MeOH (1 mL) was added sodium acetate (156 mg, 1.9 mmol) and then the mixture stirred at room temperature for 11.5 h under N₂. Sodium cyanoborohydride (69.76 mg, 1.11 mmol) was then added and the mixture stirred at room temperature for 0.5 h under N₂. The mixture was then filtered, the filtrate concentrated and the residue purified by reverse phase HPLC to afford 1-(4-(((6-(3-(2-(4-(((1-acetylpiperidin-4-yl)amino)methyl)-3-methoxyphenyl)-3-chloropyridin-4-yl)-2-methylphenyl)-2-methoxypyridin-3-yl)methyl)amino)piperidin-1-yl)ethan-1-one (62.4 mg, 24.9% yield) as a white solid (formic acid salt). MS: *m*/*z* found 725 [M+H]⁺, retention time = 2.64 min (Method A). ¹H NMR (400 MHz, Methanol-*d*₄): δ 8.57 (d, 1H), 7.79 (d, 1H), 7.45 (t, 2H), 7.38-7.34 (m, 3H), 7.29-7.27 (m, 1H), 7.19 (d, 1H), 7.11(d, 1H), 4.61-4.53 (m, 2H), 4.22 (s, 2H), 4.08 (s, 2H), 3.97-3.93 (m, 8H), 3.17-3.11 (m, 4H), 2.66-2.62 (m, 2H), 2.2-2.07 (m, 13H), 1.58-1.41 (m, 4H).

### Example 346: 2-((6-(3-(3-chloro-2-(3-methoxy-4-((7-oxo-2,6-diazaspiro[3.4]octan-2-yl)methyl)phenyl)pyridin-4-yl)-2-methylphenyl)-2-methoxypyridin-3-yl)methyl)-2,6-diazaspiro[3.4]octan-7-one (408)

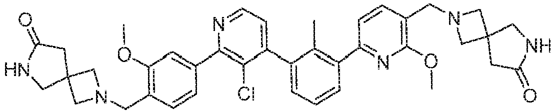

To a mixture of 6-(3-(3-chloro-2-(4-formyl-3-methoxyphenyl)pyridin-4-yl)-2-methylphenyl)-2-methoxynicotinaldehyde (Example 344, step (d)) (150 mg, 0.317 umol) and 2,6-diazaspiro[3.4]octan-7-one (154 mg, 0.951 umol) in MeOH (5 mL) was added sodium acetate (156 mg, 1.9 mmol) and then the mixture was stirred at room temperature for 11.5 h under N₂. Sodium cyanoborohydride (69.7 mg, 1.11 mmol) was then added and the mixture was stirred at room temperature for 0.5 h under N₂. The mixture was filtered, the filtrate concentrated and the residue purified by reverse phase HPLC to afford 2-((6-(3-(3-chloro-2-(3-methoxy-4-((7-oxo-2,6-diazaspiro[3.4]octan-2-yl)methyl)phenyl)pyridin-4-yl)-2-methylphenyl)-2-methoxypyridin-3-yl)methyl)-2,6-diazaspiro[3.4]octan-7-one (38 mg, 15% yield) as a white solid (formic acid salt). MS: *m*/*z* found 693 [M+H]⁺, retention time = 2.30 min (Method A). ¹H NMR (400 MHz, Methanol-*d*₄): δ 8.64 (d, 1H), 7.77 (d, 1H), 7.53-7.34 (m, 6H), 7.26-7.25 (m, 1H), 7.14 (d, 2H), 4.30 (s, 2H), 4.05-3.96 (m, 12H), 3.71 (s, 4H), 3.67 (s, 2H), 3.64 (s, 2H), 2.71(s, 2H), 2.65 (s, 2H), 2.18 (s, 3H).

### Example 347: 1-(6-((6-(3-(2-(4-((6-acetyl-2,6-diazaspiro[3.3]heptan-2-yl)methyl)-3-methoxyphenyl)-3-chloropyridin-4-yl)-2-methylphenyl)-2-methoxypyridin-3-yl)methyl)-2,6-diazaspiro[3.3]heptan-2-yl)ethan-1-one (409)

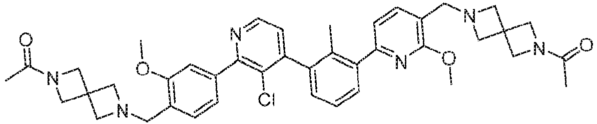

To a mixture of 6-(3-(3-chloro-2-(4-formyl-3-methoxyphenyl)pyridin-4-yl)-2-methylphenyl)-2-methoxynicotinaldehyde (Example 344, step (d)) (150 mg, 0.317 mmol) and 1-(2,6-diazaspiro[3.3]heptan-2-yl)ethan-1-one (241 mg, 0.951 mmol) in MeOH (6 mL) was added sodium acetate (156 mg, 1.9 mmol) and then the mixture was stirred at room temperature for 11.5 h under N₂. Sodium cyanoborohydride (69.7 mg, 1.11 mmol) was then added and the mixture stirred at room temperature for 0.5 h under N₂. The mixture was filtered, the filtrate was concentrated and the residue purified by reverse phase HPLC to afford 1-(6-((6-(3-(2-(4-((6-acetyl-2,6-diazaspiro[3.3]heptan-2-yl)methyl)-3-methoxyphenyl)-3-chloropyridin-4-yl)-2-methylphenyl)-2-methoxypyridin-3-yl)methyl)-2,6-diazaspiro[3.3]heptan-2-yl)ethan-1-one (91.1 mg, 36% yield) as a white solid (formic acid salt). MS: *m*/*z* found 721 [M+H]⁺, retention time = 2.52 min (Method A). ¹H NMR (400 MHz, Methanol-*d*₄): δ 8.65 (d, 1H), 7.78 (d, 1H), 7.53-7.35 (m, 6H), 7.27-7.25 (m, 1H), 7.16 (d, 1H), 4.39-4.36 (m, 6H), 4.33 (s, 4H), 4.19 (s, 2H), 4.18 (s, 2H), 4.01(d, 8H), 3.88 (s, 4H), 2.18 (s, 3H), 1.87 (s, 6H).

### Example 348: N-((6-(3-(3-chloro-2-(3-methoxy-4-(((tetrahydro-2H-pyran-4-yl)amino)methyl)phenyl)pyridin-4-yl)-2-methylphenyl)-2-methoxypyridin-3-yl)methyl)tetrahydro-2H-pyran-4-amine (426)

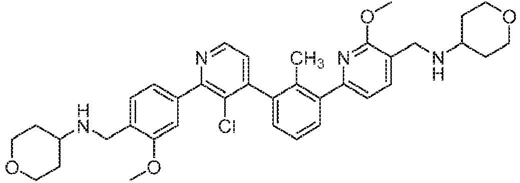

To a mixture of 6-(3-(3-chloro-2-(4-formyl-3-methoxyphenyl)pyridin-4-yl)-2-methylphenyl)-2-methoxynicotinaldehyde (Example 344, step (d)) (45 mg, 95.1 umol) and tetrahydro-2H-pyran-4-amine (28.87 mg, 285 umol) in MeOH (2 mL) was added sodium acetate (46.8 mg, 570 umol) and then the mixture was stirred at room temperature for 10 h under N₂. Sodium triacetoxyborohydride (40.3 mg, 190 umol) was then added and the mixture stirred at room temperature for 2 h under N₂. The mixture was filtered, the filtrate concentrated and the residue purified by reverse phase HPLC to afford N-((6-(3-(3-chloro-2-(3-methoxy-4-(((tetrahydro-2H-pyran-4-yl)amino)methyl)phenyl)pyridin-4-yl)-2-methylphenyl)-2-methoxypyridin-3-yl)methyl)tetrahydro-2H-pyran-4-amine (19 mg, 28% yield) as a white solid (formic acid salt). MS: *m*/*z* found 643 [M+H]⁺, retention time = 2.54 min (Method A). ¹H NMR (400 MHz, Methanol-*d*₄): δ 8.65 (d, 1H), 7.87 (d, 1H), 7.56-7.46 (m, 2H), 7.45-7.41 (m, 3H), 7.36 (d, 1H), 7.28 (d, 1H), 7.19 (d, 1H), 4.30 (s, 2H), 4.19 (s, 2H), 4.08-4.05 (m, 7H), 4.01 (s, 3H), 3.51-3.40 (m, 4H), 3.33-3.26 (m, 2H), 2.19 (s, 3H), 2.14-2.06 (m, 4H), 1.78-1.64 (m, 4H).

### Example 349: N-(1-((6-(3-(2-(4-((4-acetamidopiperidin-1-yl)methyl)-3-methoxyphenyl)-3-chloropyridin-4-yl)-2-methylphenyl)-2-methoxypyridin-3-yl)methyl)piperidin-4-yl)acetamide (467)

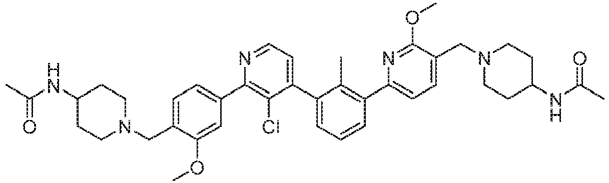

To a mixture of 6-(3-(3-chloro-2-(4-formyl-3-methoxyphenyl)pyridin-4-yl)-2-methylphenyl)-2-methoxynicotinaldehyde (Example 344, step (d)) (40 mg, 84.5 umol) and N-(piperidin-4-yl)acetamide (36.0 mg, 253 umol) in MeOH (2 mL) was added sodium acetate (41.6 mg, 507 umol) and then the mixture stirred at room temperature for 11.5 h under N₂. Sodium cyanoborohydride (21.2 mg, 338 umol) was then added and the mixture stirred at room temperature for 0.5 h under N₂. The mixture was filtered, the filtrate concentrated and the residue purified by reverse phase HPLC to afford N-(1-((6-(3-(2-(4-((4-acetamidopiperidin-1-yl)methyl)-3-methoxyphenyl)-3-chloropyridin-4-yl)-2-methylphenyl)-2-methoxypyridin-3-yl)methyl)piperidin-4-yl)acetamide (13 mg, 18% yield) as a white solid (formic acid salt). MS: *m*/*z* found 725 [M+H]⁺, retention time = 2.67 min (Method A). ¹H NMR (400 MHz, Methanol-*d*₄): δ 8.58 (d, 1H), 7.87-7.28 (m, 1H), 7.51-7.47 (m, 2H), 7.40-7.30 (m, 4H), 7.12 (d, 1H), 7.10-7.09 (m, 1H), 4.24 (s, 2H), 4.20-3.83 (m, 8H), 3.78-3.76 (m, 2H), 3.41-3.37 (m, 2H), 3.24-3.22 (m, 2H), 3.07-3.06 (m, 2H), 2.80-2.75 (m, 2H), 2.12 (s, 3H), 2.05-1.97 (m, 4H), 1.88 (s, 6H), 1.69-1.54 (m, 4H).

### Example 350: 2-((2-methoxy-6-(3-(2-(3-methoxy-4-((7-oxo-2,6-diazaspiro[3.4]octan-2-yl)methyl)phenyl)-3-methylpyridin-4-yl)-2-methylphenyl)pyridin-3-yl)methyl)-2,6-diazaspiro[3.4]octan-7-one (415)

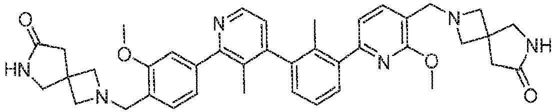

### (a) 2-Methoxy-6-(2-methyl-3-(4,4,5,5-tetramethyl-1,3,2-dioxaborolan-2-yl)phenyl)nicotinaldehyde

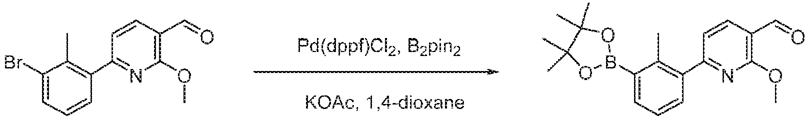

A mixture of 6-(3-bromo-2-methylphenyl)-2-methoxynicotinaldehyde (Example 344, step (b)) (1.95 g, 6.37 mmol), bis(pinacolato)diborane (3.23 g, 12.74 mmol), potassium acetate (1.88 g, 19.11 mmol) and [1,1'-bis(diphcnylphosphino)fcrroccnc]dichloropalladium(II) (466 mg, 637 umol) in 1,4-dioxane (30 mL) was degassed and purged with N₂ three times, and then the mixture was stirred at 100 °C for 3 h under N₂. The mixture was combined with another batch at 50 mg scale. The reaction mixture was quenched by the addition of H₂O (30 mL) and extracted with ethyl acetate (3 x 25 mL). The combined organic extracts were washed with saturated aqueous brine solution (2 x 20 mL), dried over anhydrous sodium sulfate, filtered and concentrated under reduced pressure to give a residue. The residue was purified by normal phase SiO₂ chromatography (0-20% ethyl acetate/petroleum ether) to afford 2-methoxy-6-(2-methyl-3-(4,4,5,5-tetramethyl-1,3,2-dioxaborolan-2-yl)phenyl)nicotinaldehyde (1.9 g, 84% yield) as a yellow solid. ¹H NMR (400 MHz, CDCl₃-*d*): δ = 10.42 (s, 1H), 8.17 (d, 1H), 7.85 (dd, 1H), 7.47 (dd, 1H), 7.31 - 7.27 (m, 1H), 7.11 (d, 1H), 4.09 (s, 3H), 2.59 (s, 3H), 1.38 (s, 12H).

### (b) 6-(3-(2-Chloro-3-methylpyridin-4-yl)-2-methylphenyl)-2-methoxynicotinaldehyde

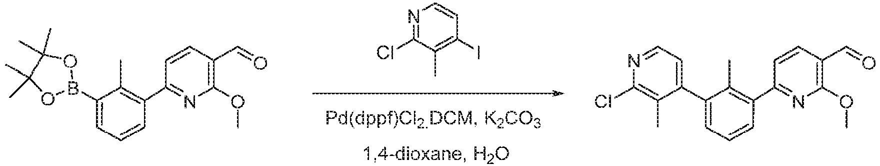

A mixture of 2-methoxy-6-(2-methyl-3-(4,4,5,5-tetramethyl-1,3,2-dioxaborolan-2-yl)phenyl)nicotinaldehyde (1.9 g, 5.38 mmol), 2-chloro-4-iodo-3-methylpyridine (2.73 g, 10.76 mmol), potassium carbonate (2.23 g, 16.14 mmol) and [1,1'- bis(diphenylphosphino)-ferrocene]dichloropalladium(II) complex with dichloromethane (439 mg, 538 µmol) in 1,4-dioxane / H₂O (10:1, 33 mL) was degassed and purged with N₂ three times, and then the mixture was stirred at 95 °C for 3 h under N₂. The mixture was combined with another batch at 50 mg scale. The reaction mixture was quenched by addition H₂O (30 mL) and extracted with ethyl acetate (3 x 25 mL). The combined organic extracts were washed with saturated aqueous brine solution (2 x 20 mL), dried over anhydrous sodium sulfate, filtered and concentrated under reduced pressure to give a residue. The residue was purified by normal phase SiO₂ chromatography (10-50% ethyl acetate/petroleum ether) to afford 6-(3-(2-chloro-3-methylpyridin-4-yl)-2-methylphenyl)-2-methoxynicotinaldehyde (1 g, 52% yield) as yellow oil. ¹H NMR (400 MHz, CDCl₃-*d*): δ = 10.43 (s, 1H), 8.29 (d, 1H), 8.21 (d, 1H), 7.52 (dd, 1H), 7.39 (t, 1H), 7.20 - 7.14 (m, 2H), 7.09 (d, 1H), 4.11 (s, 3H), 2.19 (s, 3H), 2.11 (s, 3H).

### (c) 6-(3-(2-(4-Formyl-3-methoxyphenyl)-3-methylpyridin-4-yl)-2-methylphenyl)-2-methoxynicotinaldehyde

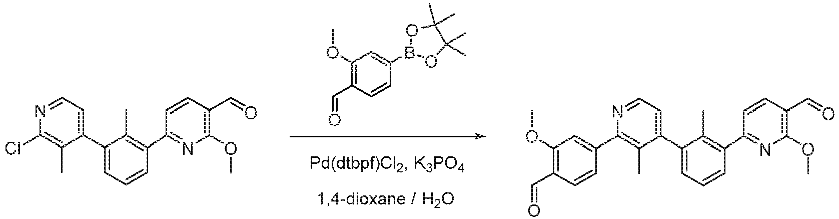

A mixture of 6-(3-(2-chloro-3-methylpyridin-4-yl)-2-methylphenyl)-2-methoxynicotinaldehyde (0.95 g, 2.69 mmol), 2-methoxy-4-(4,4,5,5-tetramethyl-1,3,2-dioxaborolan-2-yl)benzaldehyde (1.76 g, 6.73 mmol), potassium phosphate (1.71 g, 8.08 mmol) and [1,1'-bis(di-tert-butylphosphino)ferrocene]dichloropalladium(II) (175 mg, 269 µmol) in 1,4-dioxane / H₂O (10:1, 22 mL) was degassed and purged with N₂ three times, and then the mixture was stirred at 95 °C for 2 h under N₂. The mixture was combined with another batch at 20 mg scale. The reaction mixture was quenched by addition H₂O (30 mL) and extracted with ethyl acetate (3 x 25 mL). The combined organic extracts were washed with saturated aqueous brine solution (2 x 15 mL), dried over anhydrous sodium sulfate, filtered and concentrated under reduced pressure to give a residue. The residue was purified by normal phase SiO₂ chromatography (10-50% ethyl acetate/petroleum ether) to afford 6-(3-(2-(4-formyl-3-methoxyphenyl)-3-methylpyridin-4-yl)-2-methylphenyl)-2-methoxynicotinaldehyde (1 g, 82% yield) as a white solid. ¹H NMR (400 MHz, CDCl₃-*d*). δ 10.53 (s, 1H), 10.43 (s, 1H), 8.61 (d, 1H), 8.22 (d, 1H), 7.92 (d, 1H), 7.52 (dd, 1H), 7.41 (t, 1H), 7.24 - 7.19 (m, 5H), 4.11 (s, 3H), 4.03 - 3.99 (m, 3H), 2.18 (s, 3H), 2.11 (s, 3H).

### (d) 2-((2-Methoxy-6-(3-(2-(3-methoxy-4-((7-oxo-2,6-diazaspiro[3.4]octan-2-yl)methyl)phenyl)-3-methylpyridin-4-yl)-2-methylphenyl)pyridin-3-yl)methyl)-2,6-diazaspiro[3.4]octan-7-one

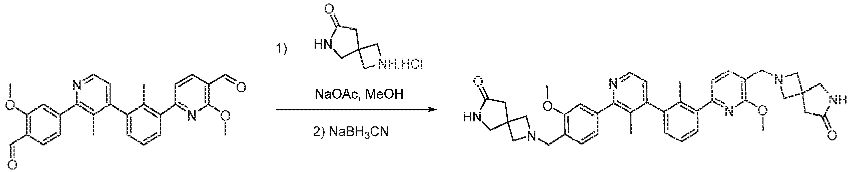

A mixture of 6-(3-(2-(4-formyl-3-methoxyphenyl)-3-methylpyridin-4-yl)-2-methylphenyl)-2-methoxynicotinaldehyde (150 mg, 0.3 mmol), 2,6-diazaspiro[3.4]octan-7-one hydrochloride salt (162 mg, 1 mmol) and sodium acetate (163 mg, 2 mmol) in MeOH (1.5 mL) was stirred at room temperature for 3 hr under N₂. Sodium cyanoborohydride (73 mg, 1.2 mmol) was then added and the mixture stirred at room temperature for 12 hr under N₂. The reaction mixture was filtered, the filtrate concentrated and the residue was purified by reverse phase HPLC to afford 2-((2-methoxy-6-(3-(2-(3-methoxy-4-((7-oxo-2,6-diazaspiro[3.4]octan-2-yl)methyl)phenyl)-3-methylpyridin-4-yl)-2-methylphenyl)pyridin-3-yl)methyl)-2,6-diazaspiro[3.4]octan-7-one (50.2 mg, 22% yield) as a white solid. MS: *m*/*z* found 673 [M+H]⁺, retention time = 1.84 min (Method A). ¹H NMR (400 MHz, Methanol-*d*₄): δ. 8.40-8.35 (m, 1H), 7.57 (d, 1H), 7.40-7.35 (m, 1H), 7.32-7.26 (m, 2H), 7.16 (d, 1H), 7.11 (d, 1H), 7.02-6.98 (m, 3H), 3.87 (s, 3H), 3.81 (s, 3H), 3.67 (s, 2H), 3.61 (s, 2H), 3.49 (d, 4H), 3.32 (d, 8H), 2.49-2.47 (m, 4H), 2.02 (s, 3H), 1.98 (s, 3H).

### Example 351: 1-(6-((6-(3-(2-(4-((6-Acetyl-2,6-diazaspiro[3.3]heptan-2-yl)methyl)-3-methoxyphenyl)-3-methylpyridin-4-yl)-2-methylphenyl)-2-methoxypyridin-3-yl)methyl)-2,6-diazaspiro[3.3]heptan-2-yl)ethan-1-one (416)

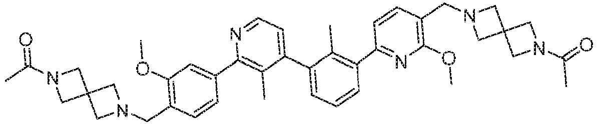

A mixture of 6-(3-(2-(4-formyl-3-methoxyphenyl)-3-methylpyridin-4-yl)-2-methylphenyl)-2-methoxynicotinaldehyde (150 mg, 0.3 mmol) (Example 350, step c), 1-(2,6-diazaspiro[3.3]heptan-2-yl)ethan-1-one trifluoroacetate salt (253 mg, 1.0 mmol) and sodium acetate (163 mg, 2.0 mmol) in MeOH (1.5 mL) was stirred at room temperature for 3 hr under N₂. Sodium cyanoborohydride (73 mg, 1.2 mmol) was then added and the mixture stirred at room temperature for 12 hr under N₂. The reaction mixture was then filtered, the filtrate concentrated and the residue purified by reverse phase HPLC to afford 1-(6-((6-(3-(2-(4-((6-acetyl-2,6-diazaspiro[3.3]heptan-2-yl)methyl)-3-methoxyphenyl)-3-methylpyridin-4-yl)-2-methylphenyl)-2-methoxypyridin-3-yl)methyl)-2,6-diazaspiro[3.3]heptan-2-yl)ethan-1-one (64.4 mg, 27% yield) as a white solid. MS: *m*/*z* found 701 [M+H]⁺, retention time = 2.00 min (Method A). ¹H NMR (400 MHz, Methanol-*d*₄): δ. 8.37 (d, 1H), 7.58 (d, 1H), 7.38-7.36 (m, 1H), 7.31 (d, 1H), 7.28-7.25 (m, 1H), 7.17-7.15 (m, 1H), 7.12-7.10 (m, 1H), 7.02 (s, 1H), 6.99 (d, 2H), 4.20 (d, 4H), 3.95 (d, 4H), 3.87 (s, 3H), 3.81 (s, 3H), 3.64 (s, 2H), 3.58 (s, 2H), 3.43-3.42 (m, 8H), 2.02 (s, 3H), 1.97 (s, 3H), 1.76 (s, 3H), 1.75 (s, 3H).

### Example 352: N-(2-Methoxy-4-(4-(3-(6-methoxy-5-(((tetrahydro-2H-pyran-4-yl)amino)methyl)pyridin-2-yl)-2-methylphenyl)-3-methylpyridin-2-yl)benzyl)tetrahydro-2H-pyran-4-amine (425)

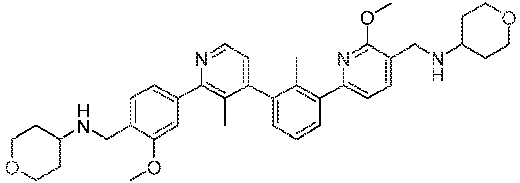

To a solution of 6-(3-(2-(4-formyl-3-methoxyphenyl)-3-methylpyridin-4-yl)-2-methylphenyl)-2-methoxynicotinaldehyde (150 mg, 0.3 mmol) (Example 350, step c) (150 mg, 332 µmol) in MeOH (5 mL) was added tetrahydro-2H-pyran-4-amine (50 mg, 497 µmol) and sodium acetate (109 mg, 1.33 mmol) and the mixture was stirred at room temperature for 12 h. Sodium cyanoborohydride (42 mg, 663 µmol) was then added and the solution was stirred at room temperature for 1h. The reaction was then concentrated under reduced pressure and the residue purified by reverse phase HPLC to afford N-(2-methoxy-4-(4-(3-(6-methoxy-5-(((tetrahydro-2H-pyran-4-yl)amino)methyl)pyridin-2-yl)-2-methylphenyl)-3-methylpyridin-2-yl)benzyl)tetrahydro-2H-pyran-4-amine (51.5 mg, 23% yield) as a white solid (formic acid salt). MS: *m*/*z* found 623 [M+H]⁺, retention time = 1.95 min (Method A). ¹H NMR (400 MHz, Methanol-*d*₄): δ = 8.52 - 8.48 (m, 3H), 7.88 (d, 1H), 7.54-7.48 (m, 2H), 7.42 (t, 1H), 7.29 (d, 1H), 7.25 - 7.16 (m, 4H), 4.30 (s, 2H), 4.24 (s, 2H), 4.07-4.05 (m, 7H), 3.99 (s, 3H), 3.50 - 3.38 (m, 6H), 2.13 - 2.07 (m, 10H), 1.76-1.69 (m, 4H).

### Example 353: 2-(4-(4-(2-chloro-3-(6-methoxy-5-((7-oxo-2,6-diazaspiro[3.4]octan-2-yl)methyl)pyridin-2-yl)phenyl)-3-fluoropyridin-2-yl)-2-methoxybenzyl)-2,6-diazaspiro[3.4]octan-7-one (324)

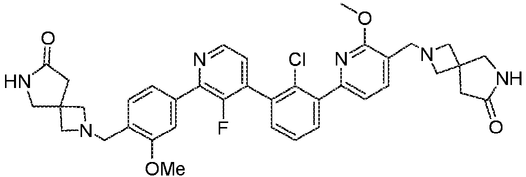

### (a) 6-(2-Chloro-3-(2-chloro-3-fluoropyridin-4-yl)phenyl)-2-methoxynicotinaldehyde

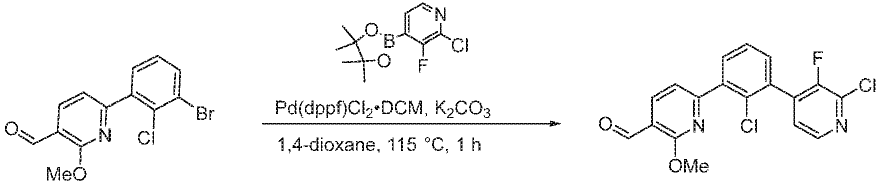

2-Chloro-3-fluoro-4-(4,4,5,5-tetramethyl-1,3,2-dioxaborolan-2-yl)pyridine (150 mg, 0.58 mmol), 6-(3-bromo-2-chlorophenyl)-2-methoxynicotinaldehyde (228 mg, 0.70 mmol), [1,1'- bis(diphenylphosphino)ferrocene]dichloropalladium(II) complex with dichloromethane (48 mg, 0.06 mmol), and potassium carbonate (201 mg, 1.46 mmol) were suspended in 1,4-dioxane/water (4:1, 5 mL), then the solution was heated at 105 °C for 60 minutes. Reaction was cooled to room temperature, diluted with 10 mL water, and extracted with EtOAc (3 x 5 mL). The combined organics were concentrated under reduced pressure and crude sample was purified by silica gel chromatography (0-30% EtOAc/hexane) to afford 6-(2-chloro-3-(2-chloro-3-fluoropyridin-4-yl)phenyl)-2-methoxynicotinaldehyde (54 mg, 25 % yield) as a yellow solid. MS: *m*/*z* found 377, 379 [M+H]⁺.

### (b) 6-(2-Chloro-3-(3-fluoro-2-(4-formyl-3-methoxyphenyl)pyridin-4-yl)phenyl)-2-methoxynicotinaldehyde

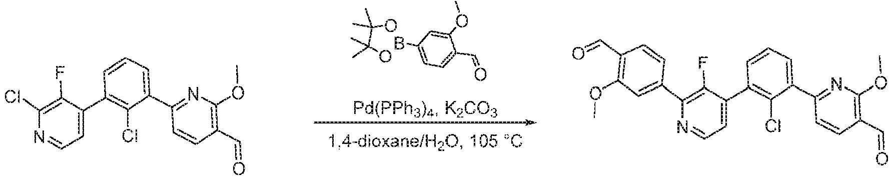

Tetrakis(triphenylphosphine)palladium(0) (33 mg, 0.03 mmol), potassium carbonate (59 mg, 0.43 mmol), 6-(2-chloro-3-(2-chloro-3-fluoropyridin-4-yl)phenyl)-2-methoxynicotinaldehyde (54 mg, 0.14 mmol), and 2-methoxy-4-(4,4,5,5-tetramethyl-1,3,2-dioxaborolan-2-yl)benzaldehyde (56 mg, 0.21 mmol) were suspended in 1,4-dioxane /water (4:1, 3 mL), then the solution was heated at 105 °C for 20 minutes. Reaction was cooled to room temperature, diluted with 10 mL water, and extracted with EtOAc (3 x 5 mL). The combined organics were concentrated under reduced pressure and the crude sample was purified by silica gel chromatography (0-35 % EtOAc/hexane) to afford 6-(2-chloro-3-(3-fluoro-2-(4-formyl-3-methoxyphenyl)pyridin-4-yl)phenyl)-2-methoxynicotinaldehyde (29 mg, 43 % yield) as a white solid . MS: *m*/*z* found 477, 479 [M+H]⁺.

### (c) 2-(4-(4-(2-Chloro-3-(6-methoxy-5-((7-oxo-2,6-diazaspiro[3.4]octan-2-yl)methyl)-pyridin-2-yl)phenyl)-3-fluoropyridin-2-yl)-2-methoxybenzyl)-2,6-diazaspiro[3.4]octan-7-one

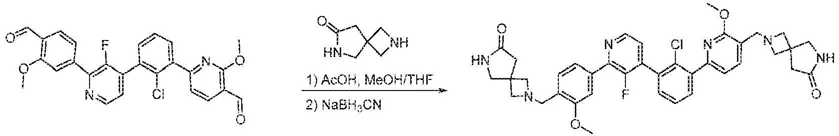

6-(2-chloro-3-(3-fluoro-2-(4-formyl-3-methoxyphenyl)pyridin-4-yl)phenyl)-2-methoxynicotinaldehyde (15 mg, 0.03 mmol), acetic acid (4 mg, 0.06 mmol) and 2,6-diazaspiro[3.4]octan-7-one (16 mg, 0.13 mmol) were dissolved in MeOH/THF (1:1, 1 mL), then the solution was stirred at 25 °C for 2 hours. Sodium cyanoborohydride (8 mg, 0.13 mmol) was added and the resulting mixture was stirred at 25 °C for 1 hour. Reaction was subsequently diluted with water (2 mL) and extracted with EtOAc (3 x 2 mL). The combined organics were further washed with brine, dried over sodium sulfate, filtered, and concentrated under reduced pressure. Crude sample was purified by reverse phase HPLC to afford 2-(4-(4-(2-chloro-3-(6-methoxy-5-((7-oxo-2,6-diazaspiro[3.4]octan-2-yl)methyl)-pyridin-2-yl)phenyl)-3-fluoropyridin-2-yl)-2-methoxybenzyl)-2,6-diazaspiro[3.4]octan-7-one (12 mg, 55 % yield) as a white solid (formic acid salt). LC/MS: *m*/*z* found 697, 699 [M+H]⁺, retention time = 2.00 min (Method C). ¹H NMR (400 MHz, Methanol-*d*₄) δ 8.59 (d, 1H), 8.40 (s, 2H), 7.79 (d, 1H), 7.71 (d, 1H), 7.65 (s, 1H), 7.60 - 7.54 (m, 2H), 7.51 (d, 2H), 7.47 (s, 1H), 7.28 (d, 1H), 4.33 (s, 2H), 4.09 (s, 4H), 4.04 (s, 2H), 4.03 (d, 3H), 3.99 (d, 3H), 3.81 (s, 4H), 3.64 (d, 4H), 2.70 (s, 2H), 2.65 (s, 2H).

### Example 354: 1-(4-(((6-(3-(2-(4-(((1-Acetylpiperidin-4-yl)amino)methyl)-3-methoxyphenyl)-3-fluoropyridin-4-yl)-2-chlorophenyl)-2-methoxypyridin-3-yl)methyl)amino)piperidin-1-yl)ethan-1-one (337)

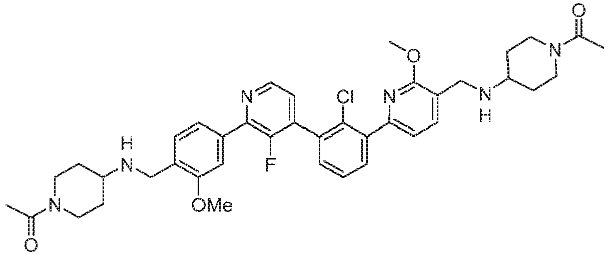

1-(4-(((6-(3-(2-(4-(((1-Acetylpiperidin-4-yl)amino)methyl)-3-methoxyphenyl)-3-fluoropyridin-4-yl)-2-chlorophenyl)-2-methoxypyridin-3-yl)methyl)amino)piperidin-1-yl)ethan-1-one was prepared in the same manner as described for Example 353, utilizing intermediate 6-(2-chloro-3-(3-fluoro-2-(4-formyl-3-methoxyphenyl)pyridin-4-yl)phenyl)-2-methoxynicotinaldehyde and replacing 2,6-diazaspiro[3.4]octan-7-one with 1-(4-amino-1-piperidyl)ethanone in step (c). Product was obtained as a tan solid (formic acid salt). LC/MS: *m*/*z* found 729, 731 [M+H] ⁺, retention time = 2.10 min (Method C). ¹H NMR (400 MHz, Methanol-*d*₄) δ 8.63 - 8.55 (m, 1H), 8.48 (s, 2H), 7.87 (d, 1H), 7.72 (d, 1H), 7.67 (s, 1H), 7.61 - 7.51 (m, 4H), 7.50 - 7.45 (m, 1H), 7.32 (d, 1H), 4.63 (t, 2H), 4.29 (s, 2H), 4.19 (s, 2H), 4.06 (s, 4H), 4.01 (s, 4H), 3.38 (d, 1H), 3.19 (t, 2H), 2.77 - 2.61 (m, 2H), 2.21 (m, 5H), 2.12 (s, 6H), 1.55 (m, 4H).

### Example 355: 2-(4-(3-chloro-4-(2-fluoro-3-(6-methoxy-5-((7-oxo-2,6-diazaspiro[3.4]octan-2-yl)methyl)pyridin-2-yl)phenyl)pyridin-2-yl)-2-methoxybenzyl)-2,6-diazaspiro[3.4]octan-7-one (438)

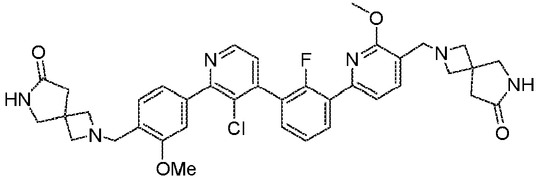

### (a) 4-(3-Bromo-2-fluorophenyl)-2,3-dichloropyridine

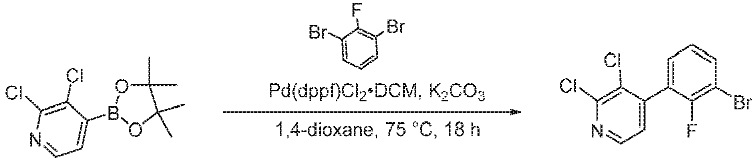

1,3-dibromo-2-fluoro-benzene (649 mg, 2.56 mmol), 2,3-dichloro-4-(4,4,5,5-tetramethyl-1,3,2-dioxaborolan-2-yl)pyridine (500 mg, 1.83 mmol), [1,1'-bis(diphenylphosphino)ferrocene]dichloropalladium(II) complex with dichloromethane (149 mg, 0.18 mmol), and potassium carbonate (705 mg, 5.11 mmol) were suspended in 10 mL 1,4-dioxane in a sealed tube. Nitrogen gas was bubbled through the reaction solution for 2 minutes, then the vessel was sealed and heated at 75 °C for 18 hours. Reaction was cooled to room temperature, diluted with 10 ml water, and extracted with EtOAc (3 x 10 mL). The combined organics were concentrated under reduced pressure and the crude sample was purified by silica gel chromatography (0-40 % EtOAc/hexane) to afford 4-(3-bromo-2-fluorophenyl)-2,3-dichloropyridine (290 mg, 50 % yield) as a white solid. MS: *m*/*z* found 321, 323 [M+H]⁺.

### (b) 4-(4-(3-Bromo-2-fluorophenyl)-3-chloropyridin-2-yl)-2-methoxybenzaldehyde

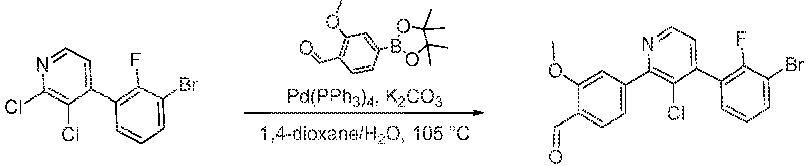

Tetrakis(triphenylphosphine)palladium(0) (108 mg, 0.09 mmol), potassium carbonate (193 mg, 1.40 mmol), 2-methoxy-4-(4,4,5,5-tetramethyl-1,3,2-dioxaborolan-2-yl)benzaldehyde (123 mg, 0.47 mmol), and 4-(3-bromo-2-fluorophenyl)-2,3-dichloropyridine (150 mg, 0.47 mmol) were suspended in 1,4-dioxane/water (4:1, 6 mL), then the solution was heated at 105 °C for 20 minutes. Reaction was cooled to room temperature, diluted with 10 mL water, and extracted with EtOAc (3 x 5 mL). The combined organics were concentrated under reduced pressure and the crude sample was purified by silica gel chromatography (0-45% EtOAc/hexane) to afford 4-(4-(3-bromo-2-fluorophenyl)-3-chloropyridin-2-yl)-2-methoxybenzaldehyde (74 mg, 38 % yield) as a yellow solid. MS: *m*/*z* found 420, 422 [M+H]⁺.

### (c) 4-(3-Chloro-4-(2-fluoro-3-(4,4,5,5-tetramethyl-1,3,2-dioxaborolan-2-yl)phenyl)-pyridin-2-yl)-2-methoxybenzaldehyde

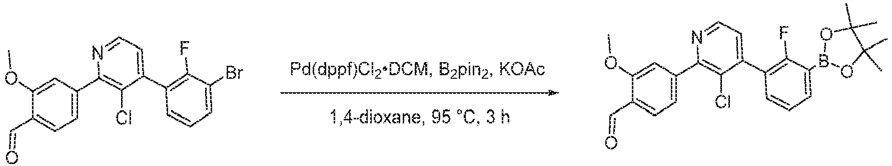

4-(4-(3-Bromo-2-fluorophenyl)-3-chloropyridin-2-yl)-2-methoxybenzaldehyde (74 mg, 0.18 mmol), bis(pinacolato)diborane (63 mg, 0.25 mmol), [1,1'-bis(diphenylphosphino)ferrocene]dichloropalladium(II) complex with dichloromethane (14 mg, 0.02 mmol), and potassium acetate (48 mg, 0.49 mmol) were suspended in 4 mL 1,4-dioxane in a sealed tube. Nitrogen gas was bubbled through the reaction solution for 2 minutes, then the vessel was sealed and heated at 95 °C for 3 hours. Reaction was cooled to room temperature and the mixture was filtered through celite. The filtrate was concentrated under reduced pressure and the crude oil was purified by silica gel chromatography (5-50% EtOAc/hexane) to afford 4-(3-chloro-4-(2-fluoro-3-(4,4,5,5-tetramethyl-1,3,2-dioxaborolan-2-yl)phenyl)-pyridin-2-yl)-2-methoxybenzaldehyde (80 mg, 97 % yield) as a yellow oil. MS: *m*/*z* found 468, 470 [M+H]⁺.

### (d) 6-(3-(3-Chloro-2-(4-formyl-3-methoxyphenyl)pyridin-4-yl)-2-fluorophenyl)-2-methoxynicotinaldehyde

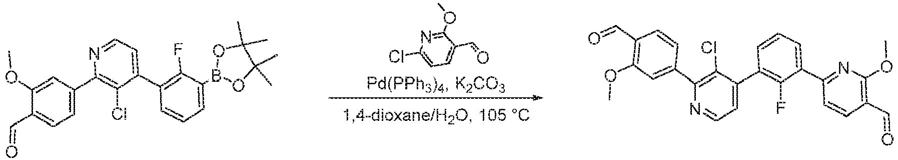

Tetrakis(triphenylphosphine)palladium(0) (62 mg, 0.05 mmol), potassium carbonate (109 mg, 0.79 mmol), 4-(3-chloro-4-(2-fluoro-3-(4,4,5,5-tetramethyl-1,3,2-dioxaborolan-2-yl)phenyl)-pyridin-2-yl)-2-methoxybenzaldehyde (80 mg, 0.17 mmol), and 6-chloro-2-methoxynicotinaldehyde (44 mg, 0.26 mmol) were suspended in 1,4-dioxane/water (4:1, 4 mL), then the solution was heated at 105 °C for 20 minutes. Reaction was cooled to room temperature, diluted with 10 mL water, and 5 mL EtOAc was also added. The sample was sonicated and the insoluble solid was filtered, washed with EtOAc/Hexane, and dried to afford 6-(3-(3-chloro-2-(4-formyl-3-methoxyphenyl)pyridin-4-yl)-2-fluorophenyl)-2-methoxynicotinaldehyde (43 mg, 53 % yield) as a white solid. MS: *m*/*z* found 477, 479 [M+H]⁺.

### (e) 2-(4-(3-Chloro-4-(2-fluoro-3-(6-methoxy-5-((7-oxo-2,6-diazaspiro[3.4]octan-2-yl)methyl)pyridin-2-yl)phenyl)pyridin-2-yl)-2-methoxybenzyl)-2,6-diazaspiro[3.4]octan-7-one

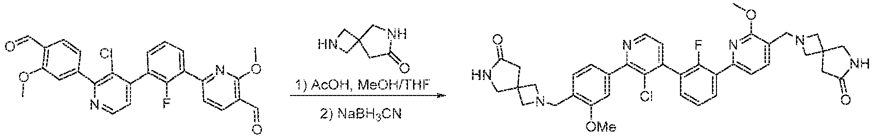

6-(3-(3-Chloro-2-(4-formyl-3-methoxyphenyl)pyridin-4-yl)-2-fluorophenyl)-2-methoxynicotinaldehyde (16 mg, 0.03 mmol), acetic acid (4 mg, 0.07 mmol), and 2,6-diazaspiro[3.4]octan-7-one (13 mg, 0.10 mmol) were dissolved in MeOH/THF (1:1, 1 mL), then the solution was stirred at 25 °C for 2 hours. Sodium cyanoborohydride (6 mg, 0.10 mmol) was added and the resulting mixture was stirred at 25 °C for 1 hour. Reaction was filtered through celite, and the filtrate was concentrated under reduced pressure. Crude sample was purified by reverse phase HPLC to afford 2-(4-(3-chloro-4-(2-fluoro-3-(6-methoxy-5-((7-oxo-2,6-diazaspiro[3.4]octan-2-yl)methyl)pyridin-2-yl)phenyl)pyridin-2-yl)-2-methoxybenzyl)-2,6-diazaspiro[3.4]octan-7-one (10 mg, 45 % yield) as a white solid (formic acid salt). LC/MS: *m*/*z* found 697, 699 [M+H] ⁺, retention time = 2.04 min (Method C). ¹H NMR (400 MHz, Methanol-*d*₄) δ 8.65 (dd, 1H), 8.41 (s, 1H), 8.22 (s, 1H), 7.74 (d, 1H), 7.53 (dd, 1H), 7.51 - 7.42 (m, 4H), 7.36 (s, 1H), 7.33 (d, 1H), 4.30 (s, 2H), 4.10 - 4.02 (m, 7H), 3.96 (d, 5H), 3.71 (s, 4H), 3.65 (d, 2H), 3.61 (d, 2H), 2.69 (d, 2H), 2.62 (d, 2H).

### Example 356: 1-(4-(((6-(3-(2-(4-(((1-Acetylpiperidin-4-yl)amino)methyl)-3-methoxyphenyl)-3-chloropyridin-4-yl)-2-fluorophenyl)-2-methoxypyridin-3-yl)methyl)amino)piperidin-1-yl)ethan-1-one (439)

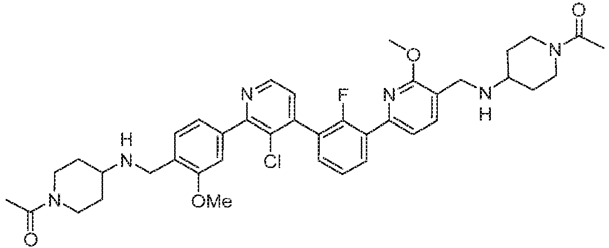

1-(4-(((6-(3-(2-(4-(((1-Acetylpiperidin-4-yl)amino)methyl)-3-methoxyphenyl)-3-chloropyridin-4-yl)-2-fluorophenyl)-2-methoxypyridin-3-yl)methyl)amino)piperidin-1-yl)ethan-1-one was prepared in similar manner as described for Example 355, utilizing intermediate 6-(3-(3-chloro-2-(4-formyl-3-methoxyphenyl)pyridin-4-yl)-2-fluorophenyl)-2-methoxynicotinaldehyde and replacing 2,6-diazaspiro[3.4]octan-7-one with 1-(4-amino-1-piperidyl)ethenone in step (e). Product was obtained as a white solid (formic acid salt). LC/MS: *m*/*z* found 729, 731 [M+H]⁺, retention time = 2.18 min (Method C). ¹H NMR (400 MHz, Methanol-*d*₄) δ 8.66 (dd, 1H), 8.51 (d, 1H), 8.22 (d, 1H), 7.83 - 7.76 (m, 1H), 7.56 - 7.48 (m, 3H), 7.45 (d, 2H), 7.37 (s, 1H), 7.33 (d, 1H), 4.59 (dd, 2H), 4.23 (s, 2H), 4.12 - 4.08 (m, 3H), 4.04 (s, 4H), 4.00 - 3.94 (m, 4H), 3.17 (q, 2H), 3.05 (d, 1H), 2.69 (t, 2H), 2.12 (dd, 10H), 1.69 - 1.20 (m, 4H).

### Example 357: N-((6-(3-(3-Chloro-2-(3-methoxy-4-(((tetrahydro-2H-pyran-4-yl)amino)-methyl)phenyl)pyridin-4-yl)-2-fluorophenyl)-2-methoxypyridin-3-yl)methyl)tetrahydro-2H-pyran-4-amine (440)

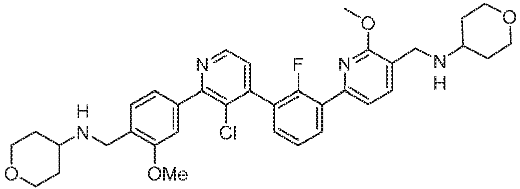

N-((6-(3-(3-Chloro-2-(3-methoxy-4-(((tetrahydro-2H-pyran-4-yl)amino)-methyl)phenyl)pyridin-4-yl)-2-fluorophenyl)-2-methoxypyridin-3-yl)methyl)tetrahydro-2H-pyran-4-amine was prepared in similar manner as described for Example 355, utilizing intermediate 6-(3-(3-chloro-2-(4-formyl-3-methoxyphenyl)pyridin-4-yl)-2-fluorophenyl)-2-methoxynicotinaldehyde and replacing 2,6-diazaspiro[3.4]octan-7-one with tetrahydropyran-4-amine in step (e). Product was obtained as a white solid (formic acid salt). LC/MS: *m*/*z* found 647, 649 [M+H] ⁺, retention time = 2.23 min (Method C). ¹H NMR (400 MHz, Methanol-*d*₄) δ 8.69 - 8.63 (m, 1H), 8.50 (s, 2H), 8.24 (s, 1H), 7.83 (d, 1H), 7.53 (t, 3H), 7.47 (d, 2H), 7.39 (s, 1H), 7.36 - 7.31 (m, 1H), 4.27 (s, 2H), 4.12 (q, 5H), 4.08 - 3.96 (m, 7H), 3.50 - 3.33 (m, 5H), 3.19 (s, 1H), 2.07 (dd, 4H), 1.67 (dd, 4H).

### Example 358: 1-(4-(((6-(3-(2-(4-(((1-acetylpiperidin-4-yl)amino)methyl)-3-methoxyphenyl)-3-fluoropyridin-4-yl)-2-fluorophenyl)-2-methoxypyridin-3-yl)methyl)amino)piperidin-1-yl)ethan-1-one (449)

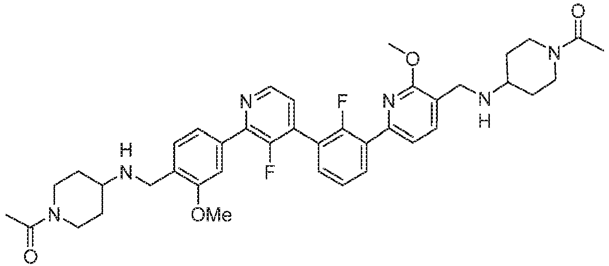

### (a) 4-(3-Bromo-2-fluorophenyl)-2-chloro-3-fluoropyridine

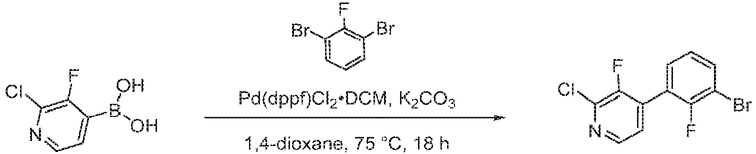

1,3-Dibromo-2-fluoro-benzene (1013 mg, 3.99 mmol), (2-chloro-3-fluoropyridin-4-yl)boronic acid (500 mg, 2.85 mmol), [1,1'- bis(diphenylphosphino)ferrocene]-dichloropalladium(II) complex with dichloromethane (233 mg, 0.29 mmol), and potassium carbonate (1102 mg, 7.98 mmol) were suspended in 25 mL 1,4-dioxane in a sealed tube. Nitrogen gas was bubbled through the reaction solution for 2 minutes, then the vessel was sealed and heated at 75 °C for 18 hours. Reaction was cooled to room temperature, diluted with 30 mL water, and extracted with EtOAc (3 x 15 mL). The combined organics were concentrated under reduced pressure and the crude sample was purified by silica gel chromatography (0-20 % EtOAc/hexane) to afford 4-(3-bromo-2-fluorophenyl)-2-chloro-3-fluoropyridine (341 mg, 39 % yield) as a white solid. MS: *m*/*z* found 304, 306 [M+H]⁺.

### (b) 4-(4-(3-Bromo-2-fluorophenyl)-3-fluoropyridin-2-yl)-2-methoxybenzaldehyde

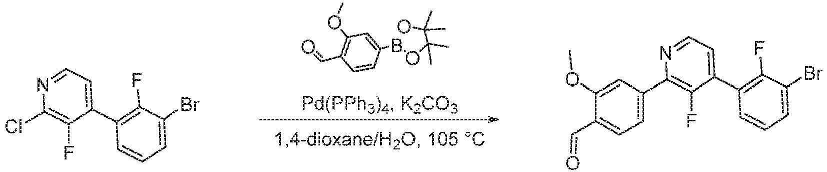

Tetrakis(triphenylphosphine)palladium(0) (190 mg, 0.16 mmol), potassium carbonate (340 mg, 2.46 mmol), 2-methoxy-4-(4,4,5,5-tetramethyl-1,3,2-dioxaborolan-2-yl)benzaldehyde (215 mg, 0.82 mmol), and 4-(3-bromo-2-fluorophenyl)-2-chloro-3-fluoropyridine (250 mg, 0.82 mmol) were suspended in 1,4-dioxane/water (4:1, 7 mL), then the solution was heated at 105 °C for 20 minutes. Reaction was cooled to room temperature, diluted with 10 mL water, and extracted with EtOAc (3 x 5 mL). The combined organics were concentrated under reduced pressure and the crude sample was purified by silica gel chromatography (0-30 % EtOAc/hexane) to afford 4-(4-(3-bromo-2-fluorophenyl)-3-fluoropyridin-2-yl)-2-methoxybenzaldehyde (155 mg, 47 % yield) as a yellow solid. MS: *m*/*z* found 404, 406 [M+H]⁺.

### (c) 4-(3-Fluoro-4-(2-fluoro-3-(4,4,5,5-tetramethyl-1,3,2-dioxaborolan-2-yl)-phenyl)-pyridin-2-yl)-2-methoxybenzaldehyde

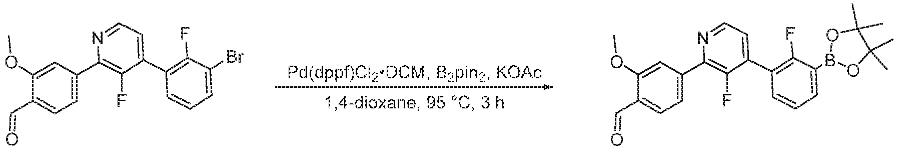

4-(4-(3-Bromo-2-fluorophenyl)-3-fluoropyridin-2-yl)-2-methoxybenzaldehyde (155 mg, 0.38 mmol), bis(pinacolato)diborane (136 mg, 0.54 mmol), [1,1'-bis(diphenylphosphino)-ferrocene]dichloropalladium(II) complex with dichloromethane (31 mg, 0.04 mmol), and potassium acetate (105 mg, 1.07 mmol) were suspended in 6 mL 1,4-dioxane in a sealed tube. Nitrogen gas was bubbled through the reaction solution for 2 minutes, then the vessel was sealed and heated at 95 °C for 3 hours. Reaction was cooled to room temperature and the mixture was filtered through celite. The filtrate was concentrated under reduced pressure and the crude oil was purified by silica gel chromatography (5-50 % EtOAc/hexane) to afford 4-(3-fluoro-4-(2-fluoro-3-(4,4,5,5-tetramethyl-1,3,2-dioxaborolan-2-yl)-phenyl)-pyridin-2-yl)-2-methoxybenzaldehyde (130 mg, 75 % yield) as a yellow oil. MS: *m*/*z* found 452 [M+H]⁺.

### (d) 6-(2-Fluoro-3-(3-fluoro-2-(4-formyl-3-methoxyphenyl)pyridin-4-yl)phenyl)-2-methoxynicotinaldehyde

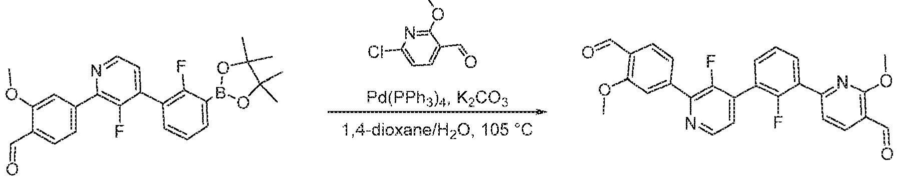

Tetrakis(triphenylphosphine)palladium(0) (67 mg, 0.06 mmol), potassium carbonate (119 mg, 0.86 mmol), 4-(3-fluoro-4-(2-fluoro-3-(4,4,5,5-tetramethyl-1,3,2-dioxaborolan-2-yl)-phenyl)-pyridin-2-yl)-2-methoxybenzaldehyde (130 mg, 0.29 mmol), and 6-chloro-2-methoxynicotinaldehyde (124 mg, 0.72 mmol) were suspended in 1,4-dioxane/water (4:1, 4 mL), then the solution was heated at 105 °C for 20 minutes. Reaction was cooled to room temperature, diluted with 10 mL water, and 5 mL EtOAc was also added. The sample was sonicated and the insoluble solid was filtered, washed with EtOAc/hexane, and dried to afford 6-(2-fluoro-3-(3-fluoro-2-(4-formyl-3-methoxyphenyl)pyridin-4-yl)phenyl)-2-methoxynicotinaldehyde (48 mg, 36 % yield) as a white solid. MS: *m*/*z* found 461 [M+H]⁺.

### (e) 1-(4-(((6-(3-(2-(4-(((1-acetylpiperidin-4-yl)amino)methyl)-3-methoxyphenyl)-3-fluoropyridin-4-yl)-2-fluorophenyl)-2-methoxypyridin-3-yl)methyl)amino)piperidin-1-yl)ethan-1-one

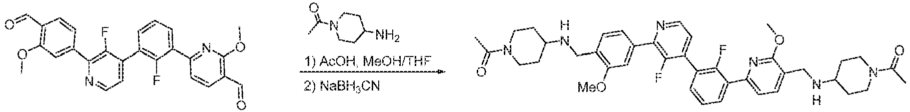

6-(2-Fluoro-3-(3-fluoro-2-(4-formyl-3-methoxyphenyl)pyridin-4-yl)phenyl)-2-methoxynicotinaldehyde (16 mg, 0.03 mmol), acetic acid (4 mg, 0.07 mmol), and 1-(4-amino-1-piperidyl)ethanone (15 mg, 0.10 mmol) were dissolved in MeOH/THF (1:1, 1 mL), then the solution was stirred at 25 °C for 2 hours. Sodium cyanoborohydride (7 mg, 0.10 mmol) was added and the resulting mixture was stirred at 25 °C for 1 hour. Reaction was filtered through celite, and the filtrate was concentrated under reduced pressure. Crude sample was purified by reverse phase HPLC to afford 1-(4-(((6-(3-(2-(4-(((1-acetylpiperidin-4-yl)amino)methyl)-3-methoxyphenyl)-3-fluoropyridin-4-yl)-2-fluorophenyl)-2-methoxypyridin-3-yl)methyl)amino)piperidin-1-yl)ethan-1-one (15 mg, 60 % yield) as a white solid (formic acid salt). LC/MS: *m*/*z* found 713 [M+H]⁺, retention time = 2.14 min (Method C). ¹H NMR (400 MHz, Methanol-*d*₄) δ 8.64 - 8.58 (m, 1H), 8.43 (s, 2H), 8.25 (t, 1H), 7.86 (d, 1H), 7.68 (s, 1H), 7.64 - 7.53 (m, 5H), 7.49 (t, 1H), 4.64 (t, 2H), 4.31 (s, 2H), 4.21 (d, 2H), 4.13 (t, 3H), 4.11 - 3.98 (m, 5H), 3.36 (d, 2H), 3.19 (t, 2H), 2.69 (t, 2H), 2.21 (q, 4H), 2.13 (q, 6H), 1.70 - 1.40 (m, 4H).

### Example 359: 2-(4-(3-Fluoro-4-(2-fluoro-3-(6-methoxy-5-((7-oxo-2,6-diazaspiro[3.4]octan-2-yl)methyl)pyridin-2-yl)phenyl)pyridin-2-yl)-2-methoxybenzyl)-2,6-diazaspiro[3.4]octan-7-one (451)

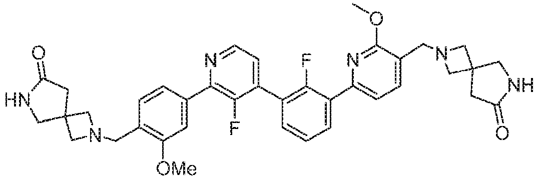

2-(4-(3-Fluoro-4-(2-fluoro-3-(6-methoxy-5-((7-oxo-2,6-diazaspiro[3.4]octan-2-yl)methyl)pyridin-2-yl)phenyl)pyridin-2-yl)-2-methoxybenzyl)-2,6-diazaspiro[3.4]octan-7-one was prepared in similar manner as described for Example 358, utilizing intermediate 6-(2-fluoro-3-(3-fluoro-2-(4-formyl-3-methoxyphenyl)pyridin-4-yl)phenyl)-2-methoxynicotinaldehyde and replacing 1-(4-amino-1-piperidyl)ethanone with 2,6-diazaspiro[3.4]octan-7-one in step (e). Product was obtained as a white solid (formic acid salt). LC/MS: *m*/*z* found 681 [M+H]⁺, retention time = 2.04 min (Method C). ¹H NMR (400 MHz, Methanol-*d*₄) δ 8.60 (d, 1H), 8.36 (s, 2H), 8.23 (t, 1H), 7.78 (d, 1H), 7.66 (s, 1H), 7.58 (d, 3H), 7.54 - 7.45 (m, 3H), 4.36 (s, 2H), 4.12 (s, 4H), 4.10 - 4.07 (m, 5H), 4.00 (d, 3H), 3.85 (s, 4H), 3.64 (dd, 4H), 2.70 (d, 2H), 2.65 (d, 2H).

### Example 360: N-((6-(2-Fluoro-3-(3-fluoro-2-(3-methoxy-4-(((tetrahydro-2H-pyran-4-yl)amino)methyl)phenyl)pyridin-4-yl)phenyl)-2-methoxypyridin-3-yl)methyl)tetrahydro-2H-pyran-4-amine (452)

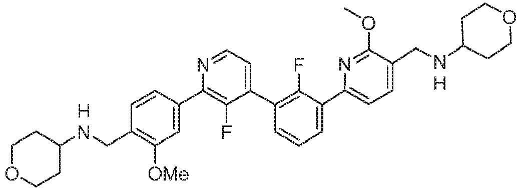

N-((6-(2-Fluoro-3-(3-fluoro-2-(3-methoxy-4-(((tetrahydro-2H-pyran-4-yl)amino)methyl)phenyl)pyridin-4-yl)phenyl)-2-methoxypyridin-3-yl)methyl)tetrahydro-2H-pyran-4-amine was prepared in similar manner as described for Example 358, utilizing intermediate 6-(2-fluoro-3-(3-fluoro-2-(4-formyl-3-methoxyphenyl)pyridin-4-yl)phenyl)-2-methoxynicotinaldehyde and replacing 1-(4-amino-1-piperidyl)ethanone with tetrahydropyran-4-amine in step (e). Product was obtained as a white solid (formic acid salt). LC/MS: *m*/*z* found 631 [M+H]⁺, retention time = 2.19 min (Method C). ¹H NMR (400 MHz, Methanol-*d*₄) δ 8.61 (d, 1H), 8.49 (s, 2H), 8.25 (t, 1H), 7.85 (d, 1H), 7.67 (s, 1H), 7.63 - 7.52 (m, 5H), 7.49 (t, 1H), 4.28 (s, 2H), 4.16 (s, 2H), 4.13 (t, 3H), 4.08 - 4.00 (m, 7H), 3.50 - 3.34 (m, 5H), 3.24 (s, 1H), 2.08 (t, 4H), 1.80 - 1.56 (m, 4H).

### Example 361: 1-(4-(((6-(3-(2-(4-(((1-Acetylpiperidin-4-yl)amino)methyl)-3-methoxyphenyl)-3-chloropyridin-4-yl)-2-(trifluoromethyl)phenyl)-2-methoxypyridin-3-yl)methyl)amino)piperidin-1-yl)ethan-1-one (528)

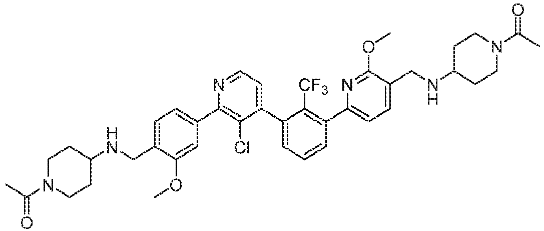

### (a) 6-(3-Bromo-2-(trifluoromethyl)phenyl)-2-methoxynicotinaldehyde

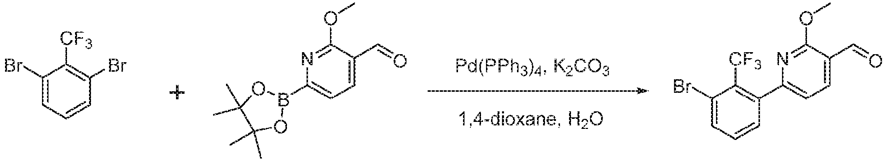

To a mixture of 1,3-dibromo-2-(trifluoromethyl)benzene (10 g, 32.9 mmol) and 2-methoxy-6-(4,4,5,5-tetramethyl-1,3,2-dioxaborolan-2-yl)nicotinaldehyde (Example 376, step (a)) (26 g, 98.7 mmol) in 1,4-dioxane / H₂O (9:1, 200 mL) was added potassium carbonate (11.4 g, 82 mmol) and tetrakis(triphenylphosphine)palladium(0) (3.80 g, 3.29 mmol), and then the mixture was stirred at 95 °C for 3 h under N₂. Water (50 mL) was added, and the mixture extracted with ethyl acetate (2 x100 mL). The combined organic extracts were dried over anhydrous sulfate, filtered and concentrated under reduced pressure. The residue was purified by normal phase SiO₂ chromatography (0-10 % EtOAc/petroleum ether) to afford 6-(3-bromo-2-(trifluoromethyl)phenyl)-2-methoxynicotinaldehyde (8 g, 67% yield) as a yellow solid. ¹H NMR (400 MHz, DMSO-*d*₆): δ 10.29 (s, 1H), 8.22 (d, 1H), 8.03 (d, 1H), 7.67 (t, 1H), 7.55 (d, 1H), 7.32 (d, 1H), 3.98 (s, 3H).

### (b) 2-Methoxy-6-(3-(4,4,5,5-tetramethyl-1,3,2-dioxaborolan-2-yl)-2-(trifluoromethyl)phenyl)nicotinaldehyde

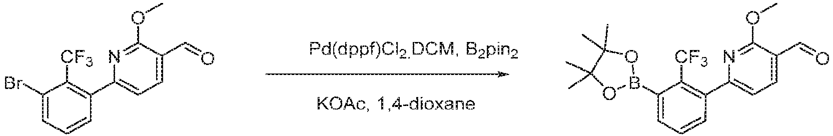

To a mixture of 6-(3-bromo-2-(trifluoromethyl)phenyl)-2-methoxynicotinaldehyde (9 g, 25.0 mmol) and bis(pinacolato)diborane (12.7 g, 49.9 mmol) in 1,4-dioxane (100 mL) was added potassium acetate (7.36 g, 75.1 mmol) and [1,1'- bis(diphenylphosphino)ferrocene]-dichloropalladium(II) complex with dichloromethane (2.04 g, 2.50 mmol) and then the mixture was stirred at 95 °C for 3 h under N₂. The mixture was combined with another batch at 1.0 g scale. The mixture was filtered, the filtrate concentrated and the residue purified by normal phase SiO₂ chromatography (0-15 % EtOAc/petroleum ether) to afford 2-methoxy-6-(3-(4,4,5,5-tetramethyl-1,3,2-dioxaborolan-2-yl)-2-(trifluoromethyl)phenyl)nicotinaldehyde (9 g, 88% yield) as a yellow solid. ¹H NMR (400 MHz, DMSO-*d*₆): δ 10.31 (s, 1H), 8.23 (d, 1H), 7.80-7.67 (m, 3H), 7.35 (d, 1H), 4.01 (s, 3H), 1.34 (s, 12H).

### (c) 6-(3-(2,3-Dichloropyridin-4-yl)-2-(trifluoromethyl)phenyl)-2-methoxynicotinaldehyde

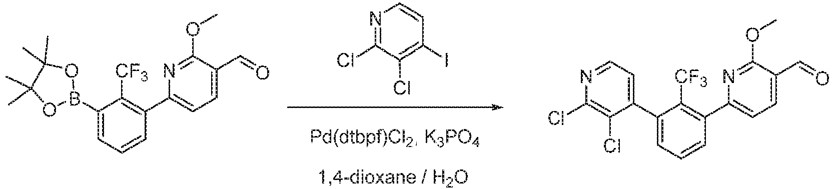

To a mixture of 2-methoxy-6-(3-(4,4,5,5-tetramethyl-1,3,2-dioxaborolan-2-yl)-2-(trifluoromethyl)phenyl)nicotinaldehyde (3 g, 7.37 mmol) and 2,3-dichloro-4-iodopyridine (1.01 g, 3.68 mmol) in 1,4-dioxane / H₂O (5:1, 90 mL) was added [1,1'-bis(di-tert-butylphosphino)ferrocene]dichloropalladium(II) (0.24 g, 0.37 mmol) and potassium phosphate (2.35 g, 11 mmol) and then the mixture was stirred at 95 °C for 2 h under N₂. The mixture was combined with another batch at 1.1 g scale. The mixture was concentrated and the residue was purified by normal phase SiO₂ chromatography (0-20 % EtOAc/petroleum ether) to afford 6-(3-(2,3-dichloropyridin-4-yl)-2-(trifluoromethyl)phenyl)-2-methoxynicotinaldehyde (1.6 g, 78% yield) as a yellow oil. ¹H NMR (400 MHz, DMSO-*d*₆): δ 10.31 (s, 1H), 8.51 (d, 1H), 8.25 (d, 1H), 7.92 (t, 1H), 7.76 (d, 1H), 7.64-7.59 (m, 2H), 7.39 (d, 1H), 4.04 (s, 3H).

### (d) 6-(3-(3-Chloro-2-(4-formyl-3-methoxyphenyl)pyridin-4-yl)-2-(trifluoromethyl)phenyl)-2-methoxynicotinaldehyde

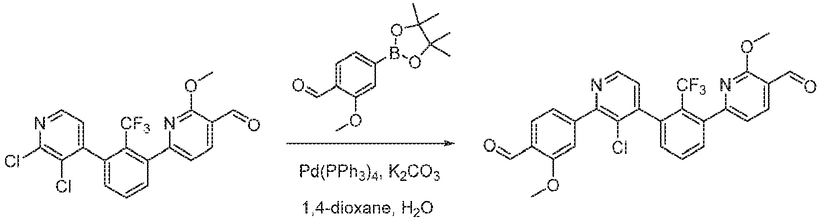

To a mixture of 6-(3-(2,3-dichloropyridin-4-yl)-2-(trifluoromethyl)phenyl)-2-methoxynicotinaldehyde (1.5 g, 3.51 mmol), 2-methoxy-4-(4,4,5,5-tetramethyl-1,3,2-dioxaborolan-2-yl)benzaldehyde (1.38 g, 5.27 mmol) in 1,4-dioxane / water (5:1, 24 mL) was added potassium carbonate (1.46 g, 10.5 mmol) and tetrakis(triphenylphosphine) palladium(0) (0.40 g, 0.35mmol) and then the mixture stirred at 95 °C for 1 h under N₂. The mixture was concentrated to give a residue that was purified by normal phase SiO₂ chromatography (0-50 % EtOAc/petroleum ether) to afford 6-(3-(3-chloro-2-(4-formyl-3-methoxyphenyl)pyridin-4-yl)-2-(trifluoromethyl)phenyl)-2-methoxynicotinaldehyde (1.5 g, 79% yield) as a yellow solid. ¹H NMR (400 MHz, DMSO-*d*₆): δ 10.39 (s, 1H), 10.28 (s, 1H), 8.72 (d, 1H), 8.22 (d, 1H), 7.91-7.87 (m, 1H), 7.80 (d, 1H), 7.72 (d, 1H), 7.62 (d, 1H), 7.57 (d, 1H), 7.47 (s, 1H), 7.37 (d, 2H), 4.00 (s, 3H), 3.96 (s, 3H).

### (e) 1-(4-(((6-(3-(2-(4-(((1-Acetylpiperidin-4-yl)amino)methyl)-3-methoxyphenyl)-3-chloropyridin-4-yl)-2-(trifluoromethyl)phenyl)-2-methoxypyridin-3-yl)methyl)amino)piperidin-1-yl)ethan-1-one

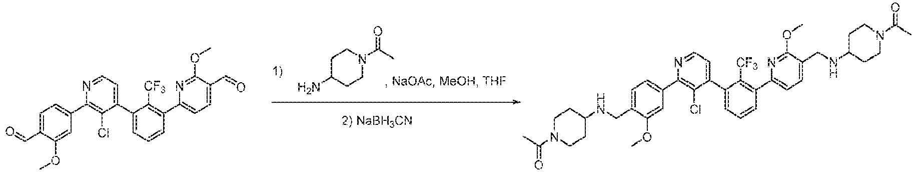

To a mixture of 6-(3-(3-chloro-2-(4-formyl-3-methoxyphenyl)pyridin-4-yl)-2-(trifluoromethyl)phenyl)-2-methoxynicotinaldehyde (200 mg, 0.38 mmol) and 1-(4-aminopiperidin-1-yl)ethan-1-one (162 mg, 1.14 mmol) in MeOH / THF (1:1, 10 mL) was added sodium acetate (124 mg, 1.52 mmol) and the mixture stirred at room temperature for 1h. Sodium cyanoborohydride (95.4 mg, 1.52 mmol) was then added and the mixture stirred at room temperature for 0.5h. The mixture was concentrated and the residue was purified by reverse phase HPLC to afford 1-(4-(((6-(3-(2-(4-(((1-acetylpiperidin-4-yl)amino)methyl)-3-methoxyphenyl)-3-chloropyridin-4-yl)-2-(trifluoromethyl)phenyl)-2-methoxypyridin-3-yl)methyl)amino)piperidin-1-yl)ethan-1-one (37.8 mg, 12% yield) as a white solid (formic acid salt). MS: *m*/*z* found 779 [M+H]⁺, retention time = 2.68 min (Method A). ¹H NMR (400 MHz, Methanol-*d*₄): δ 8.66 (d, 1H), 8.48 (br s, 2H), 7.90 (d, 1H), 7.82 (t, 1H), 7.65 (d, 1H) , 7.56 (d, 1H), 7.51-7.49 (m, 2H), 7.41 (s, 1H) , 7.37 (d, 1H), 7.21 (d, 1H), 4.70-4.63 (m, 2H), 4.33 (s, 2H), 4.22 (s, 2H), 4.11-4.02 (m, 8H), 3.47-3.41 (m, 2H), 3.26-3.19 (m, 2H), 2.75-2.69 (m, 2H), 2.29-2.15 (m, 10H), 1.67-1.52 (m, 4H).

### Example 362: N-((6-(3-(3-Chloro-2-(3-methoxy-4-(((tetrahydro-2H-pyran-4-yl)amino)methyl)phenyl)pyridin-4-yl)-2-(trifluoromethyl)phenyl)-2-methoxypyridin-3-yl)methyl)tetrahydro-2H-pyran-4-amine (529)

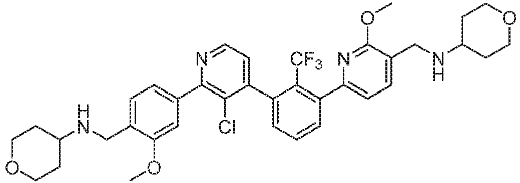

### (a) N-((6-(3-(3-Chloro-2-(3-methoxy-4-(((tetrahydro-2H-pyran-4-yl)amino)methyl)phenyl)pyridin-4-yl)-2-(trifluoromethyl)phenyl)-2-methoxypyridin-3-yl)methyl)tetrahydro-2H-pyran-4-amine

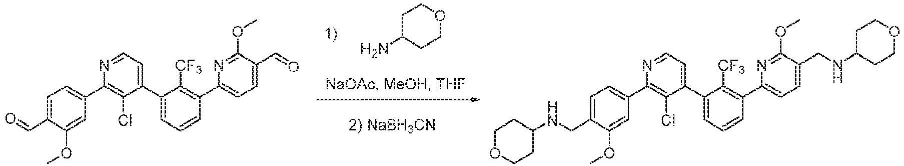

To a mixture of 6-(3-(3-chloro-2-(4-formyl-3-methoxyphenyl)pyridin-4-yl)-2-(trifluoromethyl)phenyl)-2-methoxynicotinaldehyde (Example 361, step (d)) (200 mg, 0.38 mmol) and tetrahydro-2H-pyran-4-amine (115 mg, 1.14 mmol) in MeOH / THF (1: 1, 10 mL) was added sodium acetate (124 mg, 1.52 mmol) and the mixture stirred at room temperature for 1h. Sodium cyanoborohydride (95.4 mg, 1.52 mmol) was then added and the mixture stirred at room temperature for 0.5h. The mixture was then concentrated and the residue purified by reverse phase HPLC to afford N-((6-(3-(3-chloro-2-(3-methoxy-4-(((tetrahydro-2H-pyran-4-yl)amino)methyl)phenyl)pyridin-4-yl)-2-(trifluoromethyl)phenyl)-2-methoxypyridin-3-yl)methyl)tetrahydro-2H-pyran-4-amine (54.8 mg, 19% yield) as a white solid (formic acid salt). MS: *m*/*z* found 697 [M+H]⁺, retention time = 2.89 min (Method A). ¹H NMR (400 MHz, Methanol-*d*₄): δ 8.66 (d, 1H), 7.88 (d, 1H), 7.82 (t, 1H), 7.65 (d, 1H), 7.55 (d, 1H), 7.51-7.49 (m, 2H), 7.41 (s, 1H), 7.36 (d, 1H), 7.20 (d, 1H), 4.30 (s, 2H), 4.16 (s, 2H), 4.09-4.01 (m, 10H), 3.51-3.45 (m, 4H), 3.26-3.23 (m, 2H), 2.14-2.06 (m, 4H), 1.78-1.65 (m, 4H).

### Example 363: 2-((6-(3-(3-chloro-2-(3-methoxy-4-((7-oxo-2,6-diazaspiro[3.4]octan-2-yl)methyl)phenyl)pyridin-4-yl)-2-(trifluoromethyl)phenyl)-2-methoxypyridin-3-yl)methyl)-2,6-diazaspiro[3.4]octan-7-one (576)

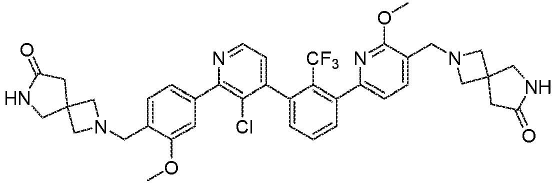

### (a) 2-((6-(3-(3-Chloro-2-(3-methoxy-4-((7-oxo-2,6-diazaspiro[3.4]octan-2-yl)methyl)phenyl)pyridin-4-yl)-2-(trifluoromethyl)phenyl)-2-methoxypyridin-3-yl)methyl)-2,6-diazaspiro [3.4] octan-7-one

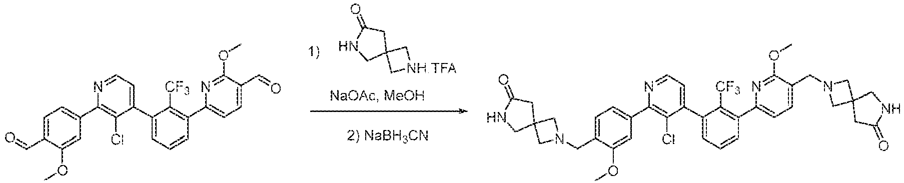

To a solution of 6-(3-(3-chloro-2-(4-formyl-3-methoxyphenyl)pyridin-4-yl)-2-(trifluoromethyl)phenyl)-2-methoxynicotinaldehyde (Example 361, step (d)) (100 mg, 190 umol) in MeOH (2 mL) was added sodium acetate (93 mg, 1.14 mmol) and 2,6-diazaspiro[3.4]octan-7-one trifluoroacetic acid salt (228 mg, 949 umol) and the solution was stirred at room temperature for 12 h. Sodium cyanoborohydride (36 mg, 569 umol) was then added and the solution stirred at room temperature for 1 h. The reaction mixture was then concentrated under reduced pressure and the residue purified by reverse phase HPLC to afford 2-((6-(3-(3-chloro-2-(3-methoxy-4-((7-oxo-2,6-diazaspiro[3.4]octan-2-yl)methyl)phenyl)pyridin-4-yl)-2-(trifluoromethyl)phenyl)-2-methoxypyridin-3-yl)methyl)-2,6-diazaspiro[3.4]octan-7-one (25.1 mg, 16 yield) as a white solid (formic acid salt). MS: *m*/*z* found 747 [M+H]⁺, retention time = 2.37 min (Method A). ¹H NMR (400 MHz, Methanol-*d*₄): δ 8.63 (d, 1H), 8.42 (s, 1H), 7.81-7.77 (m, 2H), 7.62 (d, 1H), 7.51 - 7.46 (m, 3H), 7.37 - 7.33 (m, 2H), 7.15-7.14 (m, 1H), 4.32 (s, 2H), 4.08 (br s, 4H), 3.98 (m, 8H), 3.75 (s, 4H), 3.65 (br d, 4H), 2.70 (s, 2H), 2.64 (s, 2H).

### Example 364: 1-(6-((6-(3-(2-(4-((6-Acetyl-2,6-diazaspiro[3.3]heptan-2-yl)methyl)-3-methoxyphenyl)-3-chloropyridin-4-yl)-2-(trifluoromethyl)phenyl)-2-methoxypyridin-3-yl)methyl)-2,6-diazaspiro[3.3]heptan-2-yl)ethan-1-one (577)

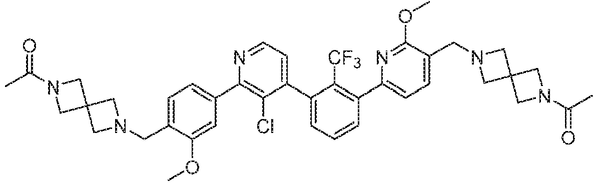

### (a) 1-(6-((6-(3-(2-(4-((6-Acetyl-2,6-diazaspiro[3.3]heptan-2-yl)methyl)-3-methoxyphenyl)-3-chloropyridin-4-yl)-2-(trifluoromethyl)phenyl)-2-methoxypyridin-3-yl)methyl)-2,6-diazaspiro[3.3]heptan-2-yl)ethan-1-one

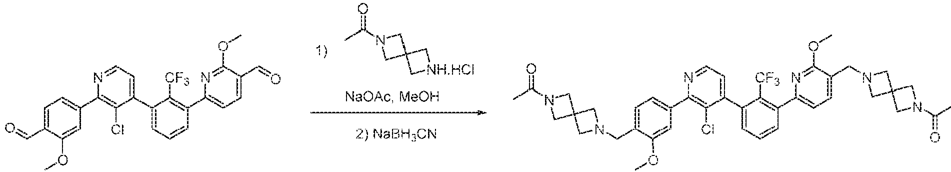

To a solution of 6-(3-(3-chloro-2-(4-formyl-3-methoxyphenyl)pyridin-4-yl)-2-(trifluoromethyl)phenyl)-2-methoxynicotinaldehyde (Example 361, step (d)) (90 mg, 171 umol) in MeOH (2 mL) was added sodium acetate (56 mg, 683 umol) and 1-(2,6-diazaspiro[3.3]heptan-2-yl)ethan-1-one hydrochloride salt (91 mg, 512 umol) and the solution stirred at room temperature for 12 h. Sodium cyanoborohydride (2 mg, 342 umol) was then added and the solution stirred at room temperature for 1 h. The reaction was then concentrated under reduced pressure and the residue purified by reverse phase HPLC to afford 1-(6-((6-(3-(2-(4-((6-acetyl-2,6-diazaspiro[3.3]heptan-2-yl)methyl)-3-methoxyphenyl)-3-chloropyridin-4-yl)-2-(trifluoromethyl)phenyl)-2-methoxypyridin-3-yl)methyl)-2,6-diazaspiro[3.3]heptan-2-yl)ethan-1-one (23.5 mg, 16% yield) as a white solid (formic acid salt). MS: *m*/*z* found 775 [M+H]⁺, retention time = 2.52 min (Method A). ¹H NMR (400 MHz, Methanol-*d*₄): δ 8.62 (d, 1H), 8.46 (br s, 1H), 7.80 - 7.72 (m, 2H), 7.62 (d, 1H), 7.46 (m, 3H), 7.35 - 7.32 (m, 2H), 7.12 (d, 1H), 4.34 (d, 4H), 4.19 (s, 2H), 4.11 (s, 2H), 4.06 (d, 6H), 3.98 (s, 3H), 3.97 (s, 3H), 3.85 (s, 2H), 3.72 (m, 4H), 1.85 (s, 6H).

### Example 365: 6-(4-(4-(3-(5-((2-Oxa-6-azaspiro[3.3]heptan-6-yl)methyl)-6-methoxypyridin-2-yl)-2-(trifluoromethyl)phenyl)-3-chloropyridin-2-yl)-2-methoxybenzyl)-2-oxa-6-azaspiro[3.3]heptane (578)

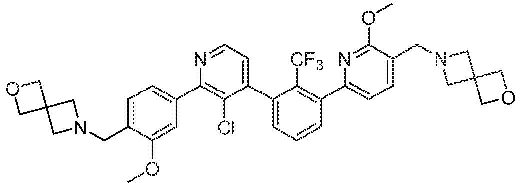

### (a) 6-(4-(4-(3-(5-((2-Oxa-6-azaspiro[3.3]heptan-6-yl)methyl)-6-methoxypyridin-2-yl)-2-(trifluoromethyl)phenyl)-3-chloropyridin-2-yl)-2-methoxybenzyl)-2-oxa-6-azaspiro[3.3]heptane

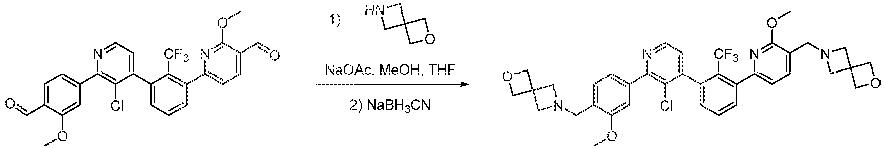

To a solution of 6-(3-(3-chloro-2-(4-formyl-3-methoxyphenyl)pyridin-4-yl)-2-(trifluoromethyl)phenyl)-2-methoxynicotinaldehyde (Example 361, step (d)) (0.1 g, 0.2 mmol) and 2-oxa-6-azaspiro[3.3]heptane (0.05 g, 0.47 mmol) in THF / MeOH mixture (1:1, 4 mL) was added sodium acetate (0.03 g, 0.38 mmol) and the mixture stirred at room temperature for 1 h. Sodium cyanoborohydride (0.036 g, 0.57 mmol) was then added and the mixture stirred at room temperature for 1hr. The mixture was then concentrated and the residue was purified by reverse phase HPLC to afford 6-(4-(4-(3-(5-((2-oxa-6-azaspiro[3.3]heptan-6-yl)methyl)-6-methoxypyridin-2-yl)-2-(trifluoromethyl)phenyl)-3-chloropyridin-2-yl)-2-methoxybenzyl)-2-oxa-6-azaspiro[3.3]heptane (23.3 mg, 98% yield) as a white solid (formic acid salt). MS: *m*/*z* found 693 [M+H]⁺, retention time = 2.61 min (Method A). ¹H NMR (400 MHz, Methanol-*d*₄) δ 8.64 (d, 1H), 7.82-7.78 (m, 1H), 7.73 (br d, 1H), 7.64 (d, 1H), 7.48-7.45 (m, 3H), 7.35-7.32 (m, 2H), 7.14 (d, 1H), 4.79-4.78 (m, 8H), 4.12 (s, 2H), 4.02-3.97 (m, 10H), 3.82 (s, 2H), 3.73 (s, 4H).

### Example 366: (S)-1-(((6-(3-(3-Chloro-2-(4-((((S)-2-hydroxypropyl)amino)methyl)-3-methoxyphenyl)pyridin-4-yl)-2-(trifluoromethyl)phenyl)-2-methoxypyridin-3-yl)methyl)amino)propan-2-ol (580)

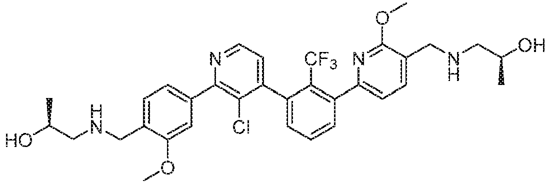

### (a) (S)-1-(((6-(3-(3-Chloro-2-(4-((((S)-2-hydroxypropyl)amino)methyl)-3-methoxyphenyl)pyridin-4-yl)-2-(trifluoromethyl)phenyl)-2-methoxypyridin-3-yl)methyl)amino)propan-2-ol

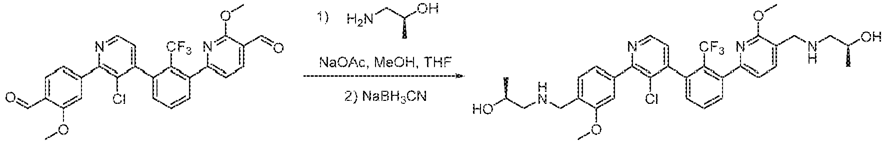

A mixture of 6-(3-(3-chloro-2-(4-formyl-3-methoxyphenyl)pyridin-4-yl)-2-(trifluoromethyl)phenyl)-2-methoxynicotinaldehyde (Example 361, step (d)) (100 mg, 189 umol), (S)-1-aminopropan-2-ol (71 mg, 949 umol) and sodium acetate (62 mg, 759 umol) in MeOH / THF (1: 1, 14 mL) was stirred at room temperature for 1h. Sodium cyanoborohydride (95 mg, 1.52 mmol) was then added and the mixture stirred at room temperature for 0.5 h. The mixture was concentrated and the residue purified by reverse phase HPLC to afford (S)-1-(((6-(3-(3-chloro-2-(4-((((S)-2-hydroxypropyl)amino)methyl)-3-methoxyphenyl)pyridin-4-yl)-2-(trifluoromethyl)phenyl)-2-methoxypyridin-3-yl)methyl)amino)propan-2-ol (31.9 mg, 24% yield) as a white solid (formic acid salt). MS: *m*/*z* found 645 [M+H]⁺, retention time = 2.53 min (Method A). ¹H NMR (400 MHz, Methanol-*d*₄): δ 8.66 (d, 1H), 7.87-7.80 (m, 2H), 7.65 (d, 1H), 7.54-7.49 (m, 3H), 7.41-7.35 (m, 2H), 7.20 (d, 1H), 4.30 (s, 2H), 4.17 (s, 2H), 4.08-4.01 (m, 8H), 3.09-3.05 (m, 1H), 3.00-2.96 (m, 1H), 2.89-2.81 (m, 2H), 1.26-1.24 (m, 6H).

### Example 367: N-((6-(3-(3-Chloro-2-(4-(((3-fluoropropyl)amino)methyl)-3-methoxyphenyl)pyridin-4-yl)-2-(trifluoromethyl)phenyl)-2-methoxypyridin-3-yl)methyl)-3-fluoropropan-1-amine (587)

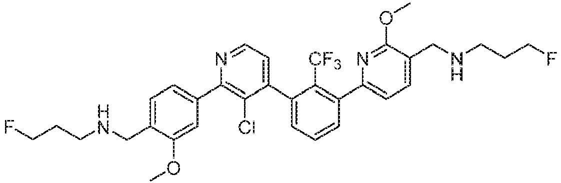

### (a) N-((6-(3-(3-Chloro-2-(4-(((3-fluoropropyl)amino)methyl)-3-methoxyphenyl)pyridin-4-yl)-2-(trifluoromethyl)phenyl)-2-methoxypyridin-3-yl)methyl)-3-fluoropropan-1-amine

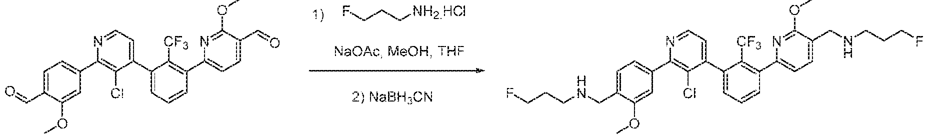

To a mixture of 6-(3-(3-chloro-2-(4-formyl-3-methoxyphenyl)pyridin-4-yl)-2-(trifluoromethyl)phenyl)-2-methoxynicotinaldehyde (Example 361, step (d)) (95 mg, 180 umol) and 3-fluoropropan-1-amine hydrochloride salt (61 mg, 540 umol) in tetrahydrofuran / methanol (1:1, 2 mL) was added sodium acetate (59 mg, 721 umol) and the mixture stirred at room temperature for 1 h under N₂. Sodium cyanoborohydride (45 mg, 721 umol) was then added and the mixture stirred at room temperature for 0.5 h under N₂. The mixture was filtered, the filtrate concentrated and the residue purified by reverse phase HPLC to afford N-((6-(3-(3-chloro-2-(4-(((3-fluoropropyl)amino)methyl)-3-methoxyphenyl)pyridin-4-yl)-2-(trifluoromethyl)phenyl)-2-methoxypyridin-3-yl)methyl)-3-fluoropropan-1-amine (42 mg, 31% yield) as a white solid (formic acid salt). MS: *m*/*z* found 649 [M+H]⁺, retention time = 2.85 min (Method A). ¹H NMR (400 MHz, Methanol-*d*₄): δ 8.65 (d, 1H), 7.87-7.80 (m, 2H), 7.65 (d, 1H), 7.55-7.49 (m, 3H), 7.41 (d, 1H), 7.36 (dd, 1H), 7.20 (d, 1H), 4.68-4.62 (m, 2H), 4.56-4.52 (m, 2H), 4.29 (s, 2H), 4.14 (s, 2H), 4.05 (s, 3H), 4.01 (s, 3H), 3.23-3.19 (m, 2H), 3.12-3.08 (m, 2H), 2.20-2.04 (m, 4H).

### Example 368: (1r,4r)-4-(((6-(3-(3-Chloro-2-(4-((((1r,4r)-4-hydroxycyclohexyl)amino)methyl)-3-methoxyphenyl)pyridin-4-yl)-2-(trifluoromethyl)phenyl)-2-methoxypyridin-3-yl)methyl)amino)cyclohexan-1-ol (588)

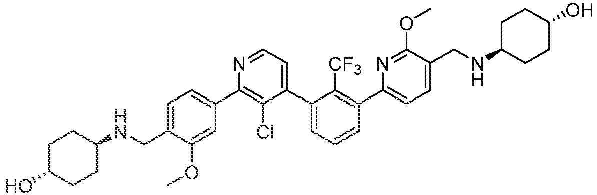

### (a) (1r,4r)-4-(((6-(3-(3-Chloro-2-(4-((((1r,4r)-4-hydroxycyclohexyl)amino)methyl)-3-methoxyphenyl)pyridin-4-yl)-2-(trifluoromethyl)phenyl)-2-methoxypyridin-3-yl)methyl)amino)cyclohexan-1-ol

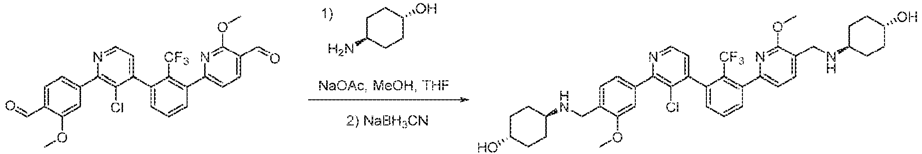

To a mixture of 6-(3-(3-chloro-2-(4-formyl-3-methoxyphenyl)pyridin-4-yl)-2-(trifluoromethyl)phenyl)-2-methoxynicotinaldehyde (Example 361, step (d)) (95 mg, 180 umol) and (1r,4r)-4-aminocyclohexan-1-ol (73 mg, 631 umol) in THF / MeOH (1:1, 2 mL) was added sodium acetate (59 mg, 721 umol) and the mixture stirred at room temperature for 1 h under N₂. Sodium cyanoborohydride (45 mg, 721 umol) was then added and the mixture stirred at room temperature for 0.5 h under N₂. The mixture was filtered, the filtrate concentrated and the residue purified by reverse phase HPLC to afford (1r,4r)-4-(((6-(3-(3-chloro-2-(4-((((1r,4r)-4-hydroxycyclohexyl)amino)methyl)-3-methoxyphenyl)pyridin-4-yl)-2-(trifluoromethyl)phenyl)-2-methoxypyridin-3-yl)methyl)amino)cyclohexan-1-ol (17 mg,12% yield) as a white solid (formic acid salt). MS: *m*/*z* found 725 [M+H]⁺, retention time = 2.70 min (Method A). ¹H NMR (400 MHz, Methanol-*d*₄): δ 8.66 (d, 1H), 7.87 (d, 1H), 7.84-7.80 (m, 1H), 7.65 (d, 1H), 7.55-7.49 (m, 3H), 7.40 (d, 1H), 7.36 (dd, 1H), 7.21 (d, 1H), 4.28 (s, 2H), 4.18 (s, 2H), 4.06 (s, 3H), 4.01 (s, 3H), 3.61-3.58 (m, 2H), 3.16-3.01 (m, 2H), 225-222 (m, 4H), 2.13-2.07 (m, 4H), 1.56-1.39 (m, 8H).

### Example 369: 1-(4-(((6-(3-(3-Chloro-2-(3-methoxy-4-(((1-(2-methoxyacetyl)piperidin-4-yl)amino)methyl)phenyl)pyridin-4-yl)-2-(trifluoromethyl)phenyl)-2-methoxypyridin-3-yl)methyl)amino)piperidin-1-yl)-2-methoxyethan-1-one (602)

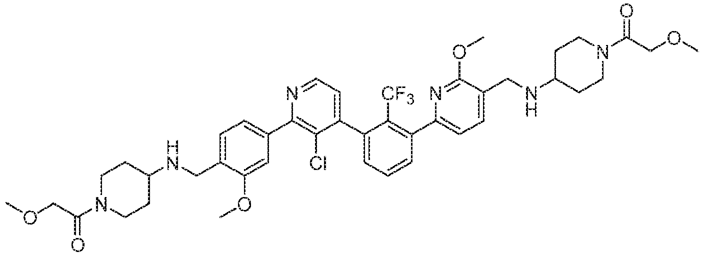

### (a) 1-(4-(((6-(3-(3-Chloro-2-(3-methoxy-4-(((1-(2-methoxyacetyl)piperidin-4-yl)amino)methyl)phenyl)pyridin-4-yl)-2-(trifluoromethyl)phenyl)-2-methoxypyridin-3-yl)methyl)amino)piperidin-1-yl)-2-methoxyethan-1-one

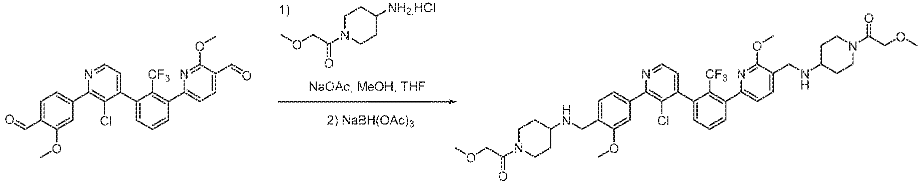

To a mixture of 6-(3-(3-chloro-2-(4-formyl-3-methoxyphenyl)pyridin-4-yl)-2-(trifluoromethyl)phenyl)-2-methoxynicotinaldehyde (Example 361, step (d)) (150 mg, 0.284 mmol) and 1-(4-aminopiperidin-1-yl)-2-methoxyethan-1-one hydrochloride salt (149 mg, 0.711 mmol) in methanol / tetrahydrofuran (1:1, 6 mL) was added sodium acetate (140 mg, 1.71 mmol) and the mixture stirred at room temperature for 11.5 h under N₂. Sodium triacetoxyborohydride (362 mg, 1.71 mmol) was then added and the mixture stirred at room temperature for 0.5 h under N₂. The mixture was then filtered, the filtrate concentrated and the residue purified by reverse phase HPLC to afford 1-(4-(((6-(3-(3-chloro-2-(3-methoxy-4-(((1-(2-methoxyacetyl)piperidin-4-yl)amino)methyl)phenyl)pyridin-4-yl)-2-(trifluoromethyl)phenyl)-2-methoxypyridin-3-yl)methyl)amino)piperidin-1-yl)-2-methoxyethan-1-one (37 mg, 15% yield) as a white solid (formic acid salt). MS: *m*/*z* found 839 [M+H]⁺, retention time = 2.52 min (Method A). ¹H NMR (400 MHz, Methanol-*d*₄): δ 8.66 (d, 1H), 7.89-7.87 (m, 1H), 7.82 (t, 1H), 7.64 (d, 1H), 7.55 (d, 1H), 7.51-7.49 (m, 2H), 7.41 (d, 1H), 7.36 (dd, 1H), 7.20 (d, 1H), 4.67-4.59 (m, 3H), 4.31 (s, 2H), 4.27-4.26 (m, 2H), 4.17-4.16 (m, 4H), 4.05 (s, 3H), 4.03 (s, 3H), 3.50-3.43 (m, 7H), 3.32-3.16 (m, 2H), 3.15-3.12 (m, 2H), 2.80-2.73 (m, 2H), 2.27-2.18 (m, 4H), 1.66-1.50 (m, 4H).

### Example 370: N-((6-(3-(2-(4-(((2-Oxaspiro[3.3]heptan-6-yl)amino)methyl)-3-methoxyphenyl)-3-chloropyridin-4-yl)-2-(trifluoromethyl)phenyl)-2-methoxypyridin-3-yl)methyl)-2-oxaspiro[3.3]heptan-6-amine (604)

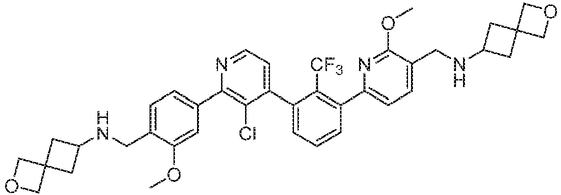

### (a) N-((6-(3-(2-(4-(((2-Oxaspiro[3.3]heptan-6-yl)amino)methyl)-3-methoxyphenyl)-3-chloropyridin-4-yl)-2-(trifluoromethyl)phenyl)-2-methoxypyridin-3-yl)methyl)-2-oxaspiro [3.3] heptan-6-amine

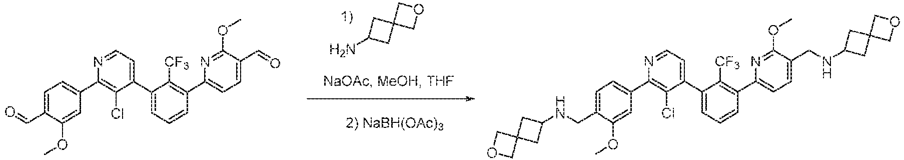

To a mixture of 6-(3-(3-chloro-2-(4-formyl-3-methoxyphenyl)pyridin-4-yl)-2-(trifluoromethyl)phenyl)-2-methoxynicotinaldehyde (Example 361, step (d)) (0.1 g, 0.19 mmol) and 2-oxaspiro[3.3]heptan-6-amine (0.07 g, 0.47 mmol) in THF / MeOH mixture (1:1, 4 mL) was added sodium acetate (0.09 g, 1.14 mmol) in one portion and the mixture stirred at room temperature for 2 h under N₂ atmosphere. Sodium triacetoxyborohydride (0.24 g, 1.14 mmol) was then added and the mixture was stirred at room temperature for 1 h. The mixture was then concentrated and the residue purified by reverse phase HPLC to afford N-((6-(3-(2-(4-((2-oxaspiro[3.3]heptan-6-ylamino)methyl)-3-methoxyphenyl)-3-chloropyridin-4-yl)-2-(trifluoromethyl)phenyl)-2-methoxypyridin-3-yl)methyl)-2-oxaspiro[3.3]heptan-6-amine (68.4 mg, 46% yield) as a white solid (formic acid salt). MS: *m*/*z* found 721 [M+H]⁺, retention time = 2.72 min (Method A). ¹H NMR (400 MHz, Methanol-*d*₄): δ 8.65 (d, 1H), 7.86-7.80 (m, 2H), 7.65 (d, 1H), 7.52-7.49 (m, 3H), 7.39-7.34 (m, 2H), 7.19 (d, 1H), 4.75 (s, 4H), 4.65 (d, 4H), 4.14 (s, 2H), 4.05 (s, 3H), 4.01-4.01 (m, 5H), 3.70-3.50 (m, 2H), 2.72-2.66 (m, 4H), 2.42-2.30 (m, 4H).

### Example 371: (S)-5-((((6-(3-(3-Chloro-2-(3-methoxy-4-(((((S)-5-oxopyrrolidin-2-yl)methyl)amino)methyl)phenyl)pyridin-4-yl)-2-(trifluoromethyl)phenyl)-2-methoxypyridin-3-yl)methyl)amino)methyl)pyrrolidin-2-one (610)

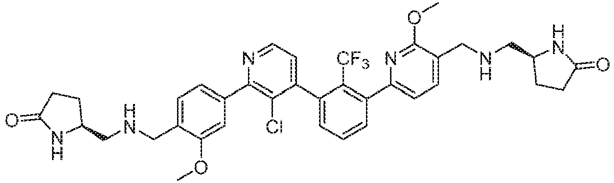

### (a) (S)-5-((((6-(3-(3-Chloro-2-(3-methoxy-4-(((((S)-5-oxopyrrolidin-2-yl)methyl)amino)methyl)phenyl)pyridin-4-yl)-2-(trifluoromethyl)phenyl)-2-methoxypyridin-3-yl)methyl)amino)methyl)pyrrolidin-2-one

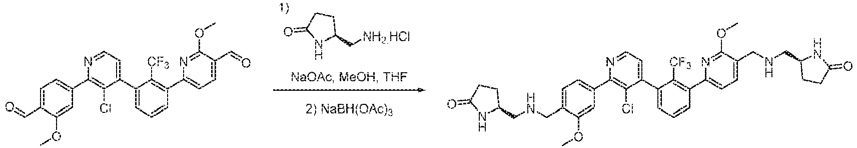

To a mixture of 6-(3-(3-chloro-2-(4-formyl-3-methoxyphenyl)pyridin-4-yl)-2-(trifluoromethyl)phenyl)-2-methoxynicotinaldehyde (Example 361, step (d)) (100 mg, 189 umol), (S)-5-(aminomethyl)pyrrolidin-2-one hydrochloride (86 mg, 569 umol) in THF / MeOH (1:1, 8 mL) was added sodium acetate (62mg, 759 umol) and the mixture stirred at room temperature for 0.5 h. Sodium triacetoxyborohydride (161 mg, 759 umol) was then added and the mixture was stirred at room temperature for 2.5 h under N₂. The mixture was then concentrated and the residue purified by reverse phase HPLC to afford (S)-5-((((6-(3-(3-chloro-2-(3-methoxy-4-(((((S)-5-oxopyrrolidin-2-yl)methyl)amino)methyl)phenyl)pyridin-4-yl)-2-(trifluoromethyl)phenyl)-2-methoxypyridin-3-yl)methyl)amino)methyl)pyrrolidin-2-one (67.2 mg, 45% yield) as a yellow solid (formic acid salt). MS: *m*/*z* found 723 [M+H]⁺, retention time = 2.46 min (Method A). ¹H NMR (400 MHz, Methanol-*d*₄): δ 8.65 (d, 1H), 8.39 (br s), 7.84-7.79 (m, 2H), 7.65 (d, 1H), 7.53-7.48 (m, 3H), 7.39-7.35 (m, 2H), 7.17 (d, 1H), 4.25-4.24 (m, 2H), 4.06-3.98 (m, 10H), 3.12-3.08 (m, 2H), 2.93-2.87 (m, 2H), 2.46-2.30 (m, 6H), 1.93-1.83 (m, 2H).

### Example 372: 2-(((6-(3-(3-Chloro-2-(4-(((2-hydroxyethyl)amino)methyl)-3-methoxyphenyl)pyridin-4-yl)-2-(trifluoromethyl)phenyl)-2-methoxypyridin-3-yl)methyl)amino)ethan-1-ol (612)

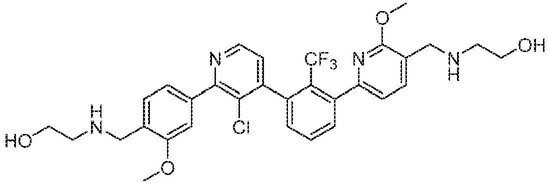

### (a) 2-(((6-(3-(3-Chloro-2-(4-(((2-hydroxyethyl)amino)methyl)-3-methoxyphenyl)pyridin-4-yl)-2-(trifluoromethyl)phenyl)-2-methoxypyridin-3-yl)methyl)amino)ethan-1-ol

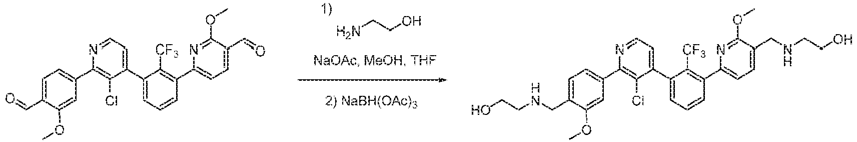

To a mixture of 6-(3-(3-chloro-2-(4-formyl-3-methoxyphenyl)pyridin-4-yl)-2-(trifluoromethyl)phenyl)-2-methoxynicotinaldehyde (Example 361, step (d)) (0.09 g, 0.17 mmol) and 2-aminoethanol (0.05 g, 0.85 mmol) in THF/methanol mixture (4:3, 3.5 mL) was added sodium acetate (0.08 g, 1.02 mmol) in one portion under N₂ atmosphere. The mixture was stirred at room temperature for 1 h. Sodium triacetoxyborohydride (0.22 g, 1.02 mmol) was then added and the mixture was stirred at room temperature for 1 h. The mixture was combined with another batch at 10 mg scale. The mixture was concentrated and the residue was purified by reverse phase HPLC to afford 2-(((6-(3-(3-chloro-2-(4-(((2-hydroxyethyl)amino)methyl)-3-methoxyphenyl)pyridin-4-yl)-2-(trifluoromethyl)phenyl)-2-methoxypyridin-3-yl)methyl)amino)ethan-1-ol (22.2 mg, 21% yield) as a pink solid. MS: *m*/*z* found 617 [M+H]⁺, retention time = 2.33 min (Method A). ¹H NMR (400 MHz, Methanol-*d*₄): 8.62 (d, 1H), 7.81-7.45 (m, 2H), 7.63 (d, 1H), 7.47-7.41 (m, 3H), 7.30-7.27 (m, 2H), 7.12 (d, 1H), 4.01 (s, 3H), 3.95 (s, 3H), 3.91 (s, 2H), 3.85 (s, 2H), 3.73-3.70 (m, 4H), 2.79-2.76 (m, 4H).

### Example 373: 1-(6-(3-(3-Chloro-2-(3-methoxy-4-((methylamino)methyl)phenyl)pyridin-4-yl)-2-(trifluoromethyl)phenyl)-2-methoxypyridin-3-yl)-N-methylmethanamine (613)

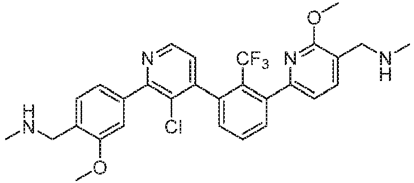

To a mixture of 6-(3-(3-chloro-2-(4-formyl-3-methoxyphenyl)pyridin-4-yl)-2-(trifluoromethyl)phenyl)-2-methoxynicotinaldehyde (Example 361, step (d)) (100 mg, 0.19 mmol) and methylamine hydrochloride (38.4 mg, 0.569 mmol) in methanol / THF (1:1, 3 ml) was added sodium acetate (108 mg, 1.33 mmol) and the mixture stirred at room temperature for 3.5 h under N₂. Sodium triacetoxyborohydride (160 mg, 0.76 mmol) was then added and the mixture stirred at room temperature for 0.5 h under N₂. The mixture was filtered, the filtrate concentrated and the residue purified by reverse phase HPLC to afford 1-(6-(3-(3-chloro-2-(3-methoxy-4-((methylamino)methyl)phenyl)pyridin-4-yl)-2-(trifluoromethyl)phenyl)-2-methoxypyridin-3-yl)-N-methylmethanamine (35 mg, 30% yield) as a white solid (formic acid salt). MS: *m*/*z* found 557 [M+H]⁺, retention time = 2.55 min (Method A). ¹H NMR (400 MHz, Methanol-*d*₄): δ 8.66 (d, 1H), 8.55 (brs, 2H), 7.88 (d, 1H), 7.84-7.81 (m, 1H), 7.65 (d, 1H), 7.54-7.49 (m, 3H), 7.41 (m, 1H), 7.36 (dd, 1H), 7.22 (d, 1H), 4.27 (s, 2H), 4.25 (s, 2H), 4.07 (s, 3H), 4.02 (s, 3H), 2.76-2.74 (m, 6H).

### Example 374: N-((6-(3-(3-Chloro-2-(3-methoxy-4-(((2-methoxyethyl)amino)methyl)phenyl)pyridin-4-yl)-2-(trifluoromethyl)phenyl)-2-methoxypyridin-3-yl)methyl)-2-methoxyethan-1-amine (614)

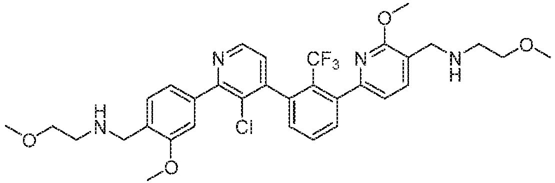

To a mixture of 6-(3-(3-chloro-2-(4-formyl-3-methoxyphenyl)pyridin-4-yl)-2-(trifluoromethyl)phenyl)-2-methoxynicotinaldehyde (Example 361, step (d)) (100 mg, 0.189 mmol) and 2-methoxyethan-1-amine (42.7 mg, 0.569 mmol) in methanol (1.5 mL) and THF (1.5 mL) was added sodium acetate (62.2 mg, 0.759 mmol) and the mixture stirred at room temperature for 4 h under N₂. Sodium cyanoborohydride (47.7 mg, 0.759 mmol) was then added and the mixture was stirred at room temperature for 0.5 h under N₂. The mixture was filtered, the filtrate concentrated and the residue purified by reverse phase HPLC to afford N-((6-(3-(3-chloro-2-(3-methoxy-4-(((2-methoxyethyl)amino)methyl)phenyl)pyridin-4-yl)-2-(trifluoromethyl)phenyl)-2-methoxypyridin-3-yl)methyl)-2-methoxyethan-1-amine (26 mg,19% yield) as a white solid (formic acid salt). MS: *m*/*z* found 645 [M+H]⁺, retention time = 2.85 min (Method A). ¹H NMR (400 MHz, Methanol-*d*₄): δ 8.63 (d, 1H), 8.57 (brs, 1H), 7.82-7.76 (m, 2H), 7.64 (d, 1H), 7.48-7.45 (m, 3H), 7.35-7.30 (m, 2H), 7.13 (d, 1H), 4.07 (s, 2H), 4.02 (s, 3H), 4.00 (s, 3H), 3.90 (s, 2H), 3.62-3.56 (m, 4H), 3.40 (s, 3H), 3.38 (s, 3H), 3.01-3.00 (m, 2H), 2.89-2.86 (m, 2H).

### Example 375: 1-(6-(((6-(3-(2-(4-(((2-Acetyl-2-azaspiro[3.3]heptan-6-yl)amino)methyl)-3-methoxyphenyl)-3-chloropyridin-4-yl)-2-(trifluoromethyl)phenyl)-2-methoxypyridin-3-yl)methyl)amino)-2-azaspiro[3.3]heptan-2-yl)ethan-1-one (651)

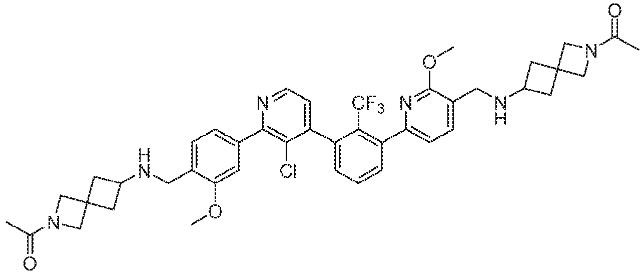

### (a) tert-Butyl 6-(((benzyloxy)carbonyl)amino)-2-azaspiro[3.3]heptane-2-carboxylate

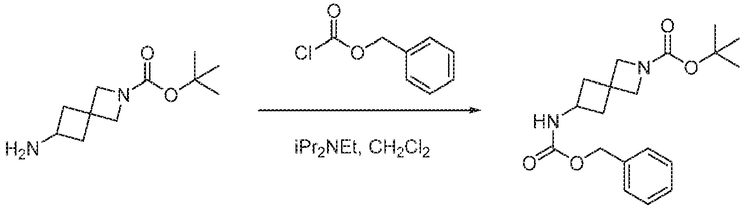

To a mixture of tert-butyl 6-amino-2-azaspiro[3.3]heptane-2-carboxylate (1 g, 4.71 mmol) and benzyl chloroformate (1.21 g, 7.07 mmol) in dichloromethane (15 mL) was added N,N-diisopropylethylamine (1.8 g, 14.1 mmol) and the mixture stirred at room temperature for 2 h under N₂. Water (10 mL) was added and the mixture and extracted with ethyl acetate (2 x 10 mL). The combined organic phases were dried over anhydrous sulfate, filtered and concentrated under reduced pressure. The residue was purified by normal phase SiO₂ chromatography (22-25% ethyl acetate/petroleum ether) to afford tert-butyl 6-(((benzyloxy)carbonyl)amino)-2-azaspiro[3.3]heptane-2-carboxylate (1.4 g, crude) as a yellow oil. ¹H NMR (400 MHz, DMSO-*d*₆): δ 7.51-7.49 (m, 1H), 7.32-7.27 (m, 5H), 4.94 (s, 2H), 3.80-3.78 (m, 3H), 3.76-3.68 (m, 2H), 2.36-3.34 (m, 2H), 2.02-1.99 (m, 2H), 1.33 (s, 9H).

### (b) Benzyl (2-azaspiro[3.3]heptan-6-yl)carbamate

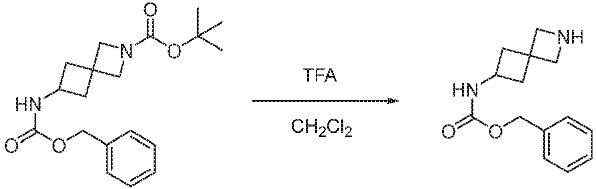

To a solution of tert-butyl 6-(((benzyloxy)carbonyl)amino)-2-azaspiro[3.3]heptane-2-carboxylate (1 g, 2.89 mmol) in dichloromethane (5 mL) was added trifluoroacetic acid (5.56 mL, 75.0 mmol) and the mixture stirred at room temperature for 0.5 h under N₂. The mixture was filtered and concentrated to afford benzyl (2-azaspiro[3.3]heptan-6-yl)carbamate (2 g, crude) as a yellow oil. MS: *m*/*z* found 247 [M+H]⁺. The crude product was used for next step without further purification.

### (c) Benzyl (2-acetyl-2-azaspiro[3.3]heptan-6-yl)carbamate

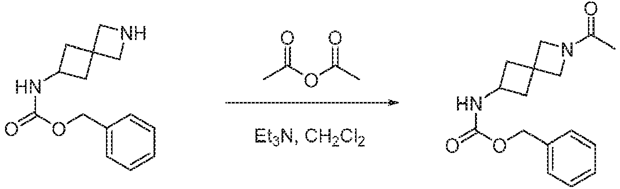

To a mixture of benzyl (2-azaspiro[3.3]heptan-6-yl)carbamate (1.9 g, 8.00 mmol) and acetic anhydride (1.2 g, 12.0 mmol) in dichloromethane (20 mL) was added triethylamine (2.4 g, 23.9 mmol) and the mixture stirred at room temperature for 2 h under N₂. The mixture was filtered and concentrated to give benzyl (2-acetyl-2-azaspiro[3.3]heptan-6-yl)carbamate (900 mg, crude) as a yellow oil. The crude product was used for next step without further purification. ¹H NMR (400 MHz, DMSO-*d*₆): δ 7.58-7.57 (m, 1H), 7.37-7.32 (m, 5H), 5.00 (s, 2H), 4.12 (s, 1H), 3.91 (s, 1H), 3.87-3.85 (m, 2H), 3.72 (s, 1H), 3.14 (s, 3H), 2.44-2.43 (m, 2H), 2.09-2.08 (m, 2H).

### (d) 1-(6-Amino-2-azaspiro[3.3]heptan-2-yl)ethan-1-one

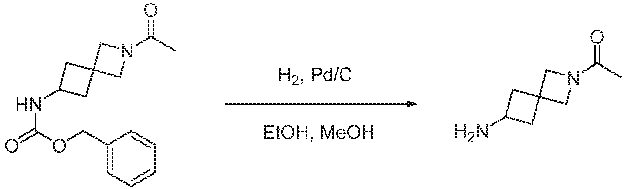

To a mixture of benzyl (2-acetyl-2-azaspiro[3.3]heptan-6-yl)carbamate (400 mg, 1.39 mmol) in ethanol/methanol (1: 1, 10 mL) was added palladium on carbon (10%, 1.00 g), then the mixture was stirred at 80 °C for 12 h under hydrogen (50 psi). The mixture was filtered and concentrated to afford 1-(6-amino-2-azaspiro[3.3]heptan-2-yl)ethan-1-one (200 mg, crude) as a white oil. MS: *m*/*z* found 155 [M+H]⁺. The crude product was used for next step without further purification.

### (e) 1-(6-(((6-(3-(2-(4-(((2-Acetyl-2-azaspiro[3.3]heptan-6-yl)amino)methyl)-3-methoxyphenyl)-3-chloropyridin-4-yl)-2-(trifluoromethyl)phenyl)-2-methoxypyridin-3-yl)methyl)amino)-2-azaspiro[3.3]heptan-2-yl)ethan-1-one

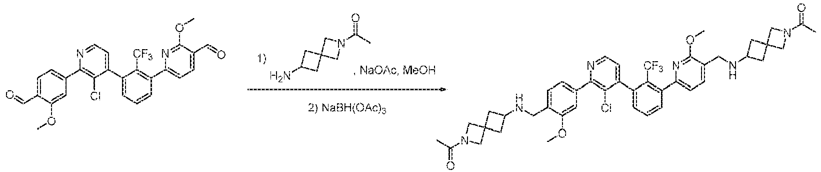

To a mixture of 1-(6-amino-2-azaspiro[3.3]heptan-2-yl)ethan-1-one (105 mg, 0.68 mmol) and 6-(3-(3-chloro-2-(4-formyl-3-methoxyphenyl)pyridin-4-yl)-2-(trifluoromethyl)phenyl)-2-methoxynicotinaldehyde (Example 361, step (d)) (120 mg, 0.23 mmol) in methanol (5 mL) was added sodium acetate (112 mg, 1.37 mmol) and then the mixture was stirred at room temperature for 11.5 h under N₂. Sodium triacetoxyborohydride (289 mg, 1.37 mmol) was the added and the mixture stirred at room temperature for 0.5 h under N₂. The mixture was filtered, the filtrate concentrated and the residue purified by reverse phase HPLC to afford 1-(6-(((6-(3-(2-(4-(((2-acetyl-2-azaspiro[3.3]heptan-6-yl)amino)methyl)-3-methoxyphenyl)-3-chloropyridin-4-yl)-2-(trifluoromethyl)phenyl)-2-methoxypyridin-3-yl)methyl)amino)-2-azaspiro[3.3]heptan-2-yl)ethan-1-one (31 mg, 16% yield) as a white solid (formic acid salt). MS: *m*/*z* found 803 [M+H]⁺, retention time = 2.57 min (Method A). ¹H NMR (400 MHz, Methanol-*d*₄): δ 8.65 (d, 1H), 8.45 (brs, 1H), 7.85-7.79 (m, 2H), 7.64 (d, 1H), 7.52-7.35 (m, 5H), 7.18 (d, 1H), 4.28 (d, 2H), 4.20-4.15 (m, 4H), 4.05-3.93 (m, 10H), 3.96 (d, 2H), 3.77-3.59 (m, 2H), 2.70-2.67 (m, 4H), 2.42-2.28 (m, 4H), 1.87 (s, 3H), 1.86 (s, 3H).

### Example 376: 2-(2-(4-(((1-Acetylpiperidin-4-yl)amino)methyl)-3-methoxyphenyl)-3-chloropyridin-4-yl)-6-(5-(((1-acetylpiperidin-4-yl)amino)methyl)-6-methoxypyridin-2-yl)benzonitrile (615)

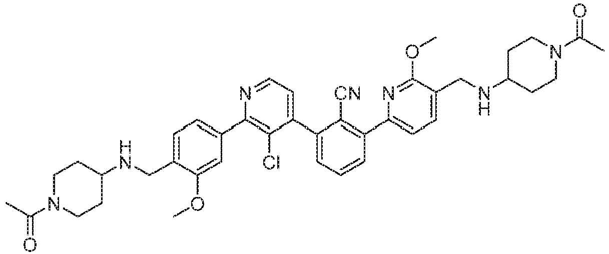

### (a) 2-Methoxy-6-(4,4,5,5-tetramethyl-1,3,2-dioxaborolan-2-yl)nicotinaldehyde

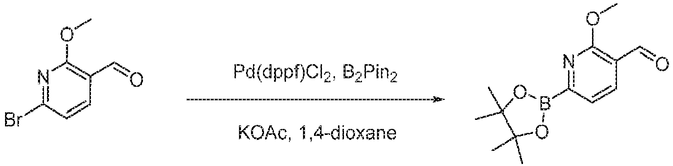

To a solution of 6-bromo-2-methoxynicotinaldehyde (50 g, 291 mmol) in 1,4-dioxane (1 L) was added bis(pinacolato)diborane (81.4 g, 320 mmol), potassium acetate (80 g, 815 mmol) and [1,1'-bis(diphenylphosphino)ferrocene]dichloropalladium(II) (9.52 g, 11.6 mmol), and the mixture stirred at 95 °C for 3 hours under N₂. The reaction mixture was cooled to room temperature and the mixture filtered through Celite^{®}. The filtrate was concentrated, and the residue dissolved in petroleum ether/ethyl acetate mixture (2:1, 600 mL) and stirred for 15 min. The mixture was filtered and the filtrate was concentrated in vacuum to afford 2-methoxy-6-(4,4,5,5-tetramethyl-1,3,2-dioxaborolan-2-yl)nicotinaldehyde (120 g, 86% purity) as a black oil. (120 g, 86% purity). ¹H NMR (400 MHz, CDCl₃): δ 10.40 (s, 1H), 8.05 (d, 1H), 7.54 (d, 1H), 4.15 (s, 3H), 1.38 (s, 12H).

### (b) 2-Bromo-6-(5-formyl-6-methoxypyridin-2-yl)benzonitrile

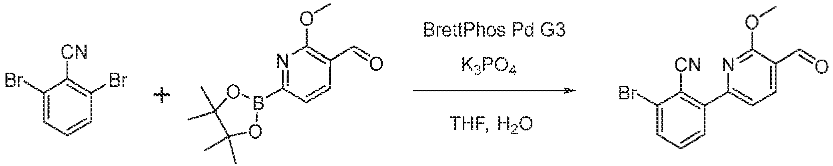

To a mixture of 2-methoxy-6-(4,4,5,5-tetramethyl-1,3,2-dioxaborolan-2-yl)nicotinaldehyde (1.5 g, 5.70 mmol, 86% purity), 2,6-dibromobenzonitrile (1.93 g, 7.41 mmol), and [(2-Di-cyclohexylphosphino-3,6-dimethoxy-2',4',6'-triisopropyl-1,1'-biphenyl)-2-(2'-amino-1,1' -biphenyl)]palladium(II) methanesulfonate (0.26 g, 0.29 mmol) in THF (56 mL) was added potassium phosphate (2.42 g, 11.4 mmol) in water (14 mL), then the mixture was stirred at 80 °C for 12 hours under N₂. The reaction mixture (3 batches) was cooled to room temperature and the reaction mixture was concentrated under reduced pressure to remove THF. To the residue was added water and the mixture extracted with ethyl acetate (3 x 10 mL). The combined organic layers were washed with water (3 x 5 mL), dried over anhydrous sodium sulfate, filtered and concentrated under reduced pressure. The residue was purified by reversed-phase HPLC to afford 2-bromo-6-(5-formyl-6-methoxypyridin-2-yl)benzonitrile (2.4 g, 34% yield) as a yellow solid. ¹H NMR (400 MHz, DMSO-*d*₆): δ 10.29 (s, 1H), 8.28 (d, 1 H), 8.06 (d, 1 H), 8.00 (d, 1 H), 7.77 (t, 1 H), 7.67 (d, 1 H), 4.15 (s, 3 H).

### (c) 2-(2,3-Dichloropyridin-4-yl)-6-(5-formyl-6-methoxypyridin-2-yl)benzonitrile

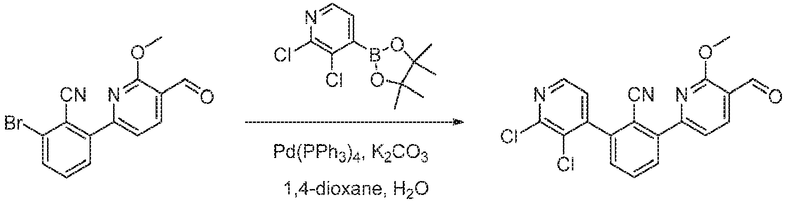

To a mixture of 2-bromo-6-(5-formyl-6-methoxypyridin-2-yl)benzonitrile (0.3 g, 0.95 mmol) and 2,3-dichloro-4-(4,4,5,5-tetramethyl-1,3,2-dioxaborolan-2-yl)pyridine (0.52 g, 1.89 mmol) in 1,4-dioxane/water (5:1, 12 mL) was added potassium carbonate (0.4 g, 2.84 mmol) and tetrakis(triphenylphosphine)palladium(0) (0.11 g, 0.95 mol), and then the mixture was stirred at 95 °C for 2 hours under N₂. The reaction mixture was cooled to room temperature, water (10 mL) added, and the mixture extracted with ethyl acetate (2 x 20 mL). The combined organic phases were dried over anhydrous sodium sulfate, filtered and concentrated. The residue was purified by normal phase SiO₂ chromatography (0-35% ethyl acetate/Petroleum ether) to afford 2-(2,3-dichloropyridin-4-yl)-6-(5-formyl-6-methoxypyridin-2-yl)benzonitrile (300 mg, 82% yield) as a white solid. MS: *m*/*z* found 384 [M+H]⁺.

### (d) 2-(3-Chloro-2-(4-formyl-3-methoxyphenyl)pyridin-4-yl)-6-(5-formyl-6-methoxypyridin-2-yl)benzonitrile

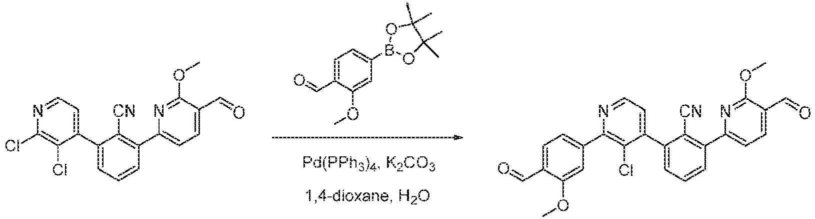

To a mixture of 2-(2,3-dichloropyridin-4-yl)-6-(5-formyl-6-methoxypyridin-2-yl)benzonitrile (0.25 g, 0.65 mmol) and 2-methoxy-4-(4,4,5,5-tetramethyl-1,3,2-dioxaborolan-2-yl)benzaldehyde (0.26 g, 0.98 mmol) in 1,4-dioxane/water (5:1, 12 mL) was added potassium carbonate (0.27 g, 1.95 mmol) and tetrakis(triphenylphosphine)palladium(0) (75.2 mg, 65.07 µmol), and then the mixture was stirred at 95 °C for 2 hours under N₂. The reaction mixture was cooled to room temperature, water (10 mL) added and the mixture extracted with ethyl acetate (2 x 30 mL). The combined organic phases were dried with anhydrous sodium sulfate, filtered and concentrated. The residue was purified by normal phase SiO₂ chromatography (0-60% ethyl acetate/Petroleum ether) to afford the product as a white solid (130 mg). The product was further purified by prep-TLC to afford 2-(3-chloro-2-(4-formyl-3-methoxyphenyl)pyridin-4-yl)-6-(5-formyl-6-methoxypyridin-2-yl)benzonitrile (40 mg, 22% yield) as a white solid. MS: *m*/*z* found 484 [M+H]⁺.

### (e) 2-(2-(4-(((1-Acetylpiperidin-4-yl)amino)methyl)-3-methoxyphenyl)-3-chloropyridin-4-yl)-6-(5-(((1-acetylpiperidin-4-yl)amino)methyl)-6-methoxypyridin-2-yl)benzonitrile

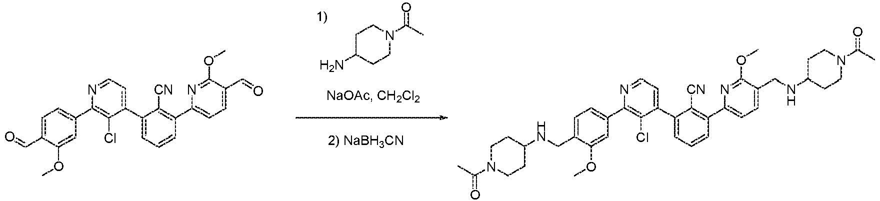

A mixture of 2-(3-chloro-2-(4-formyl-3-methoxyphenyl)pyridin-4-yl)-6-(5-formyl-6-methoxypyridin-2-yl)benzonitrile (35 mg, 72.33 µmol), 1-(4-aminopiperidin-1-yl)ethan-1-one (38.7 mg, 0.22 mmol) and sodium acetate (35.6 mg, 0.43 mmol) in dichloromethane (2 mL) was stirred at room temperature for 3 hours under N₂. Sodium cyanoborohydride (13.6 mg, 0.22 mmol) was then added, and the mixture stirred at room temperature for 30 min under N₂. The reaction mixture was purified by reverse phase HPLC to afford 2-(2-(4-(((1-acetylpiperidin-4-yl)amino)methyl)-3-methoxyphenyl)-3-chloropyridin-4-yl)-6-(5-(((1-acetylpiperidin-4-yl)amino)methyl)-6-methoxypyridin-2-yl)benzonitrile (7.3 mg, 13% yield) as a white solid. MS: *m*/*z* found 736 [M+H]⁺, retention time = 2.50 min (Method A). ¹H NMR (400 MHz, Methanol-*d*₄): δ 8.69 (d, 1H), 8.01 (d, 1H), 7.89 (t, 1H), 7.85 (d, 1H), 7.61 (d, 1H), 7.53 (d, 1H), 7.48 (d, 1H), 7.43 (d, 1H), 7.31-7.28 (m, 2H), 4.51-4.44 (m, 2H), 4.1 (s, 3H), 3.98-3.89 (m, 7H), 3.87 (s, 2H), 3.15-3.13 (m, 2H), 2.79-2.68 (m, 4H), 2.10 (s, 6H), 2.05-1.97 (m, 4H), 1.43-1.24 (m, 4H).

### Example 377: 2-(2-(4-((6-Acetyl-2,6-diazaspiro[3.3]heptan-2-yl)methyl)-3-methoxyphenyl)-3-chloropyridin-4-yl)-6-(5-((6-acetyl-2,6-diazaspiro[3.3]heptan-2-yl)methyl)-6-methoxypyridin-2-yl)benzonitrile (636)

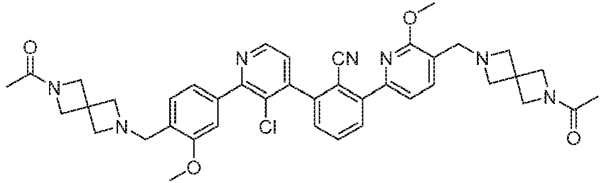

### (a) 2-(2-(4-((6-Acetyl-2,6-diazaspiro[3.3]heptan-2-yl)methyl)-3-methoxyphenyl)-3-chloropyridin-4-yl)-6-(5-((6-acetyl-2,6-diazaspiro[3.3]heptan-2-yl)methyl)-6-methoxypyridin-2-yl)benzonitrile

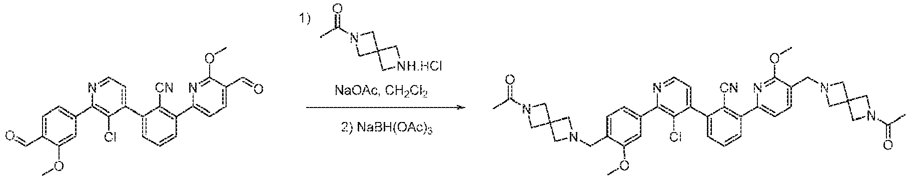

To a mixture of 2-(3-chloro-2-(4-formyl-3-methoxyphenyl)pyridin-4-yl)-6-(5-formyl-6-methoxypyridin-2-yl)benzonitrile (Example 376, step (d)) (70 mg, 145 umol) and 1-(2,6-diazaspiro[3.3]heptan-2-yl)ethanone hydrochloride salt (63.9 mg, 362 umol) in dichloromethane (5 mL) was added sodium acetate (59.3 mg, 723 umol), then the mixture was stirred at room temperature for 12 h under N₂. Sodium triacetoxyborohydride (92 mg, 434 umol) was then added and the mixture stirred at room temperature for 0.5 h under N₂. The mixture was concentrated and the residue purified by reverse phase HPLC to afford 2-(2-(4-((6-acetyl-2,6-diazaspiro[3.3]heptan-2-yl)methyl)-3-methoxyphenyl)-3-chloropyridin-4-yl)-6-(5-((6-acetyl-2,6-diazaspiro[3.3]heptan-2-yl)methyl)-6-methoxypyridin-2-yl)benzonitrile (15.1 mg, 13% yield) as a white solid (formic acid salt). MS: *m*/*z* found 732. ¹H NMR (400 MHz, Methanol-*d*₄): δ 8.73 (d, 1H), 8.42 (br s, 2H), 8.04 (d, 1H), 7.92 (t, 1H), 7.85 (d, 1H), 7.64 (d, 1H), 7.58 (d, 1H), 7.53-7.50 (m, 2H), 7.42 (s, 1H), 7.39 (d, 1H), 4.38 (s, 2H), 4.35 (s, 2H), 4.33 (s, 2H), 4.20 (s, 4H), 4.15-4.11 (m, 7H), 3.99 (s, 3H), 3.96 (s, 2H), 3.82 (s, 4H), 1.86 (s, 6H).

### Example 378: 2-(3-Chloro-2-(3-methoxy-4-(((tetrahydro-2H-pyran-4-yl)amino)methyl)phenyl)pyridin-4-yl)-6-(6-methoxy-5-(((tetrahydro-2H-pyran-4-yl)amino)methyl)pyridin-2-yl)benzonitrile (652)

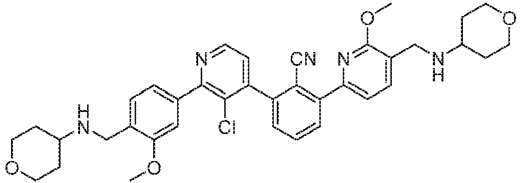

### (a) 2-(3-Chloro-2-(3-methoxy-4-(((tetrahydro-2H-pyran-4-yl)amino)methyl)phenyl)pyridin-4-yl)-6-(6-methoxy-5-(((tetrahydro-2H-pyran-4-yl)amino)methyl)pyridin-2-yl)benzonitrile

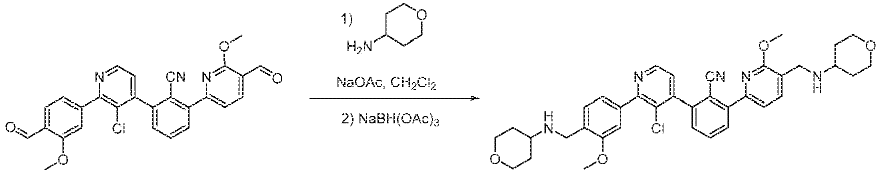

To a mixture of 2-(3-chloro-2-(4-formyl-3-methoxyphenyl)pyridin-4-yl)-6-(5-formyl-6-methoxypyridin-2-yl)benzonitrile (Example 376, step (d)) (60 mg, 124 umol) and tetrahydropyran-4-amine (37.6 mg, 372 umol) in dichloromethane (5 mL) was added sodium acetate (50.8 mg, 620 umol) and then the mixture was stirred at room temperature for 12 h under N₂. Sodium triacetoxyborohydride (78.8 mg, 372 umol) was then added and the mixture stirred at room temperature for 0.5 h under N₂. The mixture was concentrated and the residue was purified by reverse phase HPLC to afford 2-(3-chloro-2-(3-methoxy-4-(((tetrahydro-2H-pyran-4-yl)amino)methyl)phenyl)pyridin-4-yl)-6-(6-methoxy-5-(((tetrahydro-2H-pyran-4-yl)amino)methyl)pyridin-2-yl)benzonitrile (6.3 mg, 7% yield) as a white solid (formic acid salt). MS: *m*/*z* found 654 [M+H]⁺, retention time = 2.58 min (Method A). ¹H NMR (400 MHz, Methanol-*d*₄): δ 8.73 (d, 1H), 8.53 (br s, 2H), 8.05 (d, 1H), 7.95-7.91 (m, 2H), 7.65 (d, 1H), 7.59-7.53 (m, 3H), 7.42-7.37 (m, 2H), 4.28 (s, 2H), 4.15 (s, 3H), 4.12-4.01 (m, 9H), 3.56-3.43 (m, 5H), 3.15-3.10 (m, 1H), 2.13-2.02 (m, 4H), 1.77-1.60 (m, 4H).

### Example 379: 2-(3-Chloro-2-(3-methoxy-4-((7-oxo-2,6-diazaspiro[3.4]octan-2-yl)methyl)phenyl)pyridin-4-yl)-6-(6-methoxy-5-((7-oxo-2,6-diazaspiro [3.4] octan-2-yl)methyl)pyridin-2-yl)benzonitrile (653)

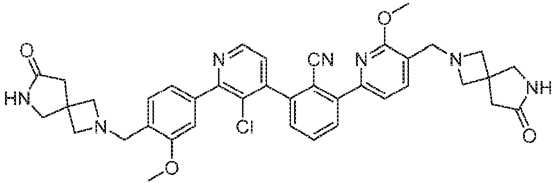

### (a) 2-(3-Chloro-2-(3-methoxy-4-((7-oxo-2,6-diazaspiro[3.4]octan-2-yl)methyl)phenyl)pyridin-4-yl)-6-(6-methoxy-5-((7-oxo-2,6-diazaspiro [3.4] octan-2-yl)methyl)pyridin-2-yl)benzonitrile

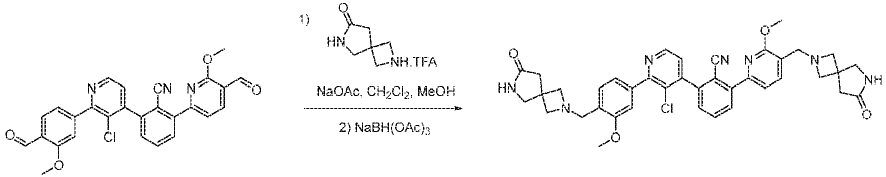

To a mixture of 2-(3-chloro-2-(4-formyl-3-methoxyphenyl)pyridin-4-yl)-6-(5-formyl-6-methoxypyridin-2-yl)benzonitrile (Example 376, step (d)) (60 mg, 0.12 mmol), 2,6-diazaspiro[3.4]octan-7-one trifluoroacetate salt (238 mg, 0.99 mmol) in dichloromethane / methanol (2:1, 7.5 mL) was added sodium acetate (102 mg, 1.24 mmol) and then the mixture was stirred at room temperature for 12 h under N₂. Sodium triacetoxyborohydride (78.8 mg, 0.37 mmol) was then added and the mixture stirred at room temperature for 0.5 h under N₂. The mixture was concentrated and the residue was purified by reverse phase HPLC to afford 2-(3-chloro-2-(3-methoxy-4-((7-oxo-2,6-diazaspiro[3.4]octan-2-yl)methyl)phenyl)pyridin-4-yl)-6-(6-methoxy-5-((7-oxo-2,6-diazaspiro[3.4]octan-2-yl)methyl)pyridin-2-yl)benzonitrile (6.4 mg, 6% yield) as a white solid (formic acid salt). MS: *m*/*z* found 704 [M+H]⁺, retention time = 2.20 min (Method A). ¹H NMR (400 MHz, Methanol-*d*₄): δ 8.73 (d, 1H), 8.41 (br s, 2H), 8.03 (d, 1H), 7.92 (t, 1H), 7.84 (d, 1H), 7.63 (d, 1H), 7.57 (d, 1H), 7.51 (d, 2H), 7.40-7.36 (m, 2H), 4.28 (s, 2H), 4.11 (s, 3H), 4.02 (s, 4H), 3.99 (s, 3H), 3.88 (s, 2H), 3.67 (s, 2H), 3.62-3.60 (m, 6H), 2.70 (s, 2H), 2.63 (s, 2H).

### Example 380: 2-(4-(((1-Acetylpiperidin-4-yl)amino)methyl)-3-methoxyphenyl)-4-(3-(5-(((1-acetylpiperidin-4-yl)amino)methyl)-6-methoxypyridin-2-yl)-2-chlorophenyl)nicotinonitrile (404)

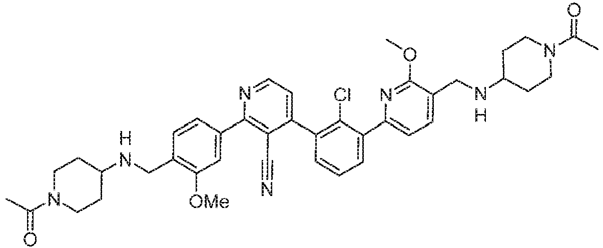

### (a) 2-Chloro-4-(2-chloro-3-(5-formyl-6-methoxypyridin-2-yl)phenyl)nicotinonitrile

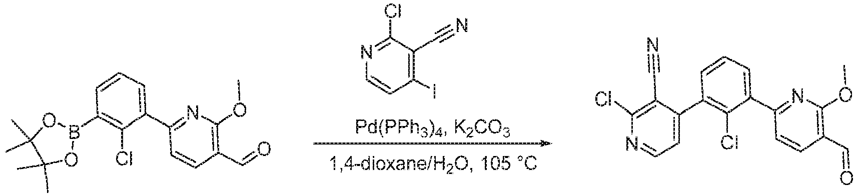

Tetrakis(triphenylphosphine)palladium(0) (31 mg, 0.03 mmol), potassium carbonate (55 mg, 0.40 mmol), 2-chloro-4-iodonicotinonitrile (46 mg, 0.17 mmol), and 6-(2-chloro-3-(4,4,5,5-tetramethyl-1,3,2-dioxaborolan-2-yl)phenyl)-2-methoxynicotinaldehyde (50 mg, 0.13 mmol) were suspended in 1,4-dioxane/water (4:1, 3 mL), then the solution was heated at 110 °C for 20 minutes. Reaction was cooled to room temperature, diluted with 10 mL water, and extracted with EtOAc (3 x 5 mL). The combined organics were concentrated under reduced pressure and the crude sample was purified by silica gel chromatography (0-40% EtOAc/hexane) to afford 2-chloro-4-(2-chloro-3-(5-formyl-6-methoxypyridin-2-yl)phenyl)nicotinonitrile (49 mg, 95 % yield) as a yellow oil. MS: *m*/*z* found 384, 386 [M+H]⁺.

### (b) 4-(2-Chloro-3-(5-formyl-6-methoxypyridin-2-yl)phenyl)-2-(4-formyl-3-methoxyphenyl)nicotinonitrile

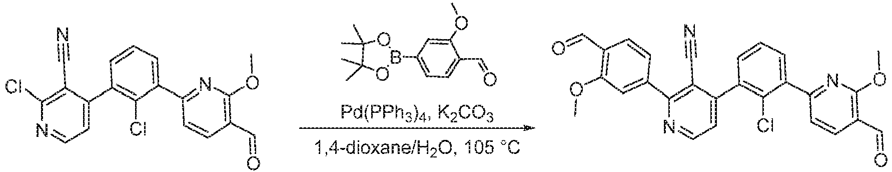

Tetrakis(triphenylphosphine)palladium(0) (30 mg, 0.03 mmol), potassium carbonate (53 mg, 0.38 mmol), 2-chloro-4-(2-chloro-3-(5-formyl-6-methoxypyridin-2-yl)phenyl)nicotinonitrile (49 mg, 0.13 mmol), and 2-methoxy-4-(4,4,5,5-tetramethyl-1,3,2-dioxaborolan-2-yl)benzaldehyde (50 mg, 0.19 mmol) were suspended in 1,4-dioxane/water (4:1, 3 mL), then the solution was heated at 105 °C for 20 minutes. Reaction was cooled to room temperature, diluted with 10 mL water, and extracted with EtOAc (3 x 5 mL). The combined organics were concentrated under reduced pressure and the crude sample was purified by silica gel chromatography (0-100% EtOAc/hexane) to afford 4-(2-chloro-3-(5-formyl-6-methoxypyridin-2-yl)phenyl)-2-(4-formyl-3-methoxyphenyl)nicotinonitrile (31 mg, 50 % yield) as a yellow foam. MS: *m*/*z* found 484, 486 [M+H]⁺.

### (c) 2-(4-(((1-Acetylpiperidin-4-yl)amino)methyl)-3-methoxyphenyl)-4-(3-(5-(((1-acetylpiperidin-4-yl)amino)methyl)-6-methoxypyridin-2-yl)-2-chlorophenyl)nicotinonitrile

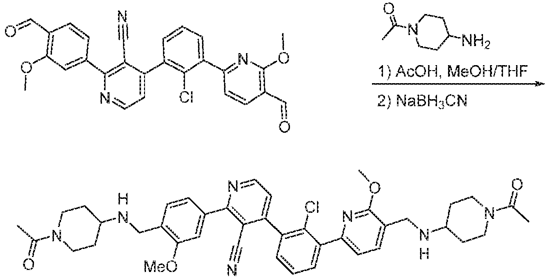

4-(2-chloro-3-(5-formyl-6-methoxypyridin-2-yl)phenyl)-2-(4-formyl-3-methoxyphenyl)-nicotinonitrile (31 mg, 0.06 mmol), acetic acid (8 mg, 0.13 mmol), and 1-(4-amino-1-piperidyl)ethanone (27 mg, 0.19 mmol) were dissolved in MeOH/THF (1:1, 1 mL), then the solution was stirred at 25 °C for 2 hours. Sodium cyanoborohydride (12 mg, 0.19 mmol) was added and the resulting mixture was stirred at 25 °C for 1 hour. Reaction was subsequently diluted with water (3 mL) and extracted with EtOAc (3 x 2 mL). The combined organics were further washed with brine, dried over sodium sulfate, filtered, and concentrated under reduced pressure. Crude sample was purified by reverse phase HPLC to afford 2-(4-(((1-acetylpiperidin-4-yl)amino)methyl)-3-methoxyphenyl)-4-(3-(5-(((1-acetylpiperidin-4-yl)amino)methyl)-6-methoxypyridin-2-yl)-2-chlorophenyl)nicotinonitrile (22 mg, 48 % yield) as a white solid (formic acid salt). LC/MS: *m*/*z* found 736, 739 [M+H]⁺, retention time = 2.04 min (Method C). ¹H NMR (400 MHz, Methanol-*d*₄) δ 8.93 (d, 1H), 7.77 (d, 2H), 7.56 (m, 6H), 7.28 (d, 1H), 4.47 (d, 2H), 4.02 (d, 3H), 3.98 (d, 5H), 3.92 (s, 2H), 3.88 (s, 2H), 3.14 (t, 2H), 2.93 - 2.76 (m, 2H), 2.69 (q, 2H), 2.10 (d, 6H), 2.03 (s, 4H), 1.45 - 1.26 (m, 4H).

### Example 381: 4-(2-Chloro-3-(6-methoxy-5-((7-oxo-2,6-diazaspiro[3.4]octan-2-yl)methyl)pyridin-2-yl)phenyl)-2-(3-methoxy-4-((7-oxo-2,6-diazaspiro[3.4]octan-2-yl)methyl)phenyl)nicotinonitrile (433)

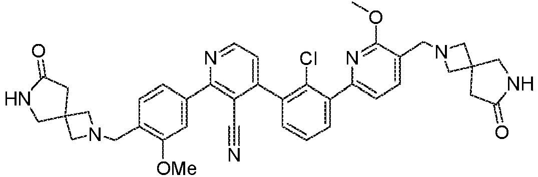

4-(2-Chloro-3-(6-methoxy-5-((7-oxo-2,6-diazaspiro[3.4]octan-2-yl)methyl)pyridin-2-yl)phenyl)-2-(3-methoxy-4-((7-oxo-2,6-diazaspiro[3.4]octan-2-yl)methyl)phenyl)nicotinonitrile was prepared in similar manner as described for Example 380, utilizing intermediate 4-(2-chloro-3-(5-formyl-6-methoxypyridin-2-yl)phenyl)-2-(4-formyl-3-methoxyphenyl)-nicotinonitrile and replacing 1-(4-amino-1-piperidyl)ethanone with 2,6-diazaspiro[3.4]octan-7-one in step (c). Product was obtained as a white solid (formic acid salt). LC/MS: *m*/*z* found 704, 706 [M+H]⁺, retention time = 1.94 min (Method C). ¹H NMR (400 MHz, Methanol-*d*₄) δ 8.98 - 8.92 (m, 1H), 8.45 (s, 1H), 7.76 (t, 2H), 7.63 - 7.57 (m, 3H), 7.57 - 7.51 (m, 3H), 7.29 (d, 1H), 4.21 (s, 2H), 4.03 - 4.00 (m, 3H), 3.99 (t, 3H), 3.93 (d, 6H), 3.66 (s, 4H), 3.63 (t, 2H), 3.61 (d, 2H), 2.67 (d, 2H), 2.62 (d, 2H).

### Example 382: 4-(2-Chloro-3-(6-methoxy-5-(((tetrahydro-2H-pyran-4-yl)amino)methyl)pyridin-2-yl)phenyl)-2-(3-methoxy-4-(((tetrahydro-2H-pyran-4-yl)amino)methyl)phenyl)nicotinonitrile (434)

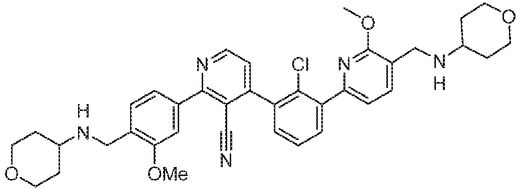

4-(2-Chloro-3-(6-methoxy-5-(((tetrahydro-2H-pyran-4-yl)amino)methyl)pyridin-2-yl)phenyl)-2-(3-methoxy-4-(((tetrahydro-2H-pyran-4-yl)amino)methyl)phenyl)nicotinonitrile was prepared in similar manner as described for Example 380, utilizing intermediate 4-(2-chloro-3-(5-formyl-6-methoxypyridin-2-yl)phenyl)-2-(4-formyl-3-methoxyphenyl)-nicotinonitrile and replacing 1-(4-amino-1-piperidyl)ethanone with tetrahydropyran-4-amine in step (c). Product was obtained as a white solid (formic acid salt). LC/MS: *m*/*z* found 654, 656 [M+H]⁺, retention time = 2.10 min (Method C). ¹H NMR (400 MHz, Methanol-*d*₄) δ 8.95 (m, 1H), 8.53 (s, 1H), 7.78 (dd, 2H), 7.63 - 7.58 (m, 3H), 7.57 - 7.52 (m, 3H), 7.32 - 7.26 (m, 1H), 4.12 (s, 2H), 4.03 (t, 3H), 4.01 - 3.97 (m, 6H), 3.95 (s, 3H), 3.43 (t, 4H), 3.10 (s, 1H), 2.91 (s, 1H), 2.05 - 1.91 (m, 4H), 1.67 - 1.46 (m, 4H).

### Example 383: 1-(4-(((6-(3-(2-(4-(((1-Acetylpiperidin-4-yl)amino)methyl)-3-methoxyphenyl)-3-methylpyridin-4-yl)-2-chlorophenyl)-2-methoxypyridin-3-yl)methyl)amino)piperidin-1-yl)ethan-1-one (271)

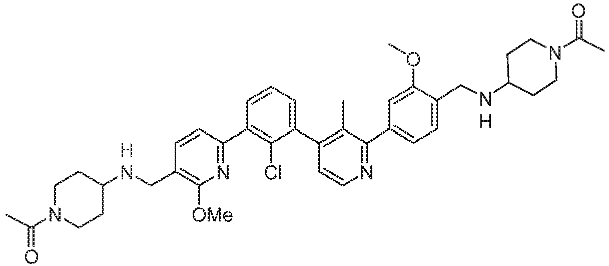

### (a) 6-(2-chloro-3-(2-(4-formyl-3-methoxyphenyl)-3-methylpyridin-4-yl)phenyl)-2-methoxynicotinaldehyde

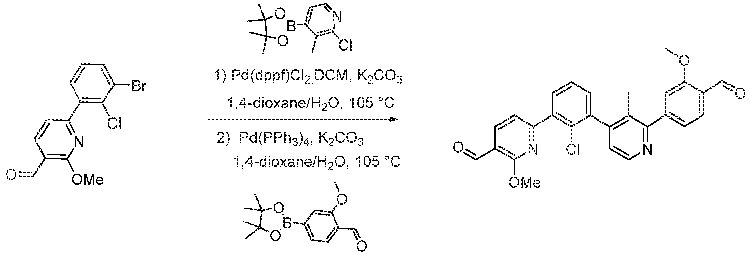

2-Chloro-3-methyl-4-(4,4,5,5-tetramethyl-1,3,2-dioxaborolan-2-yl)pyridine (100 mg, 0.39 mmol), 6-(3-bromo-2-chlorophenyl)-2-methoxynicotinaldehyde (155 mg, 0.47 mmol), [1,1'- bis(diphenylphosphino)ferrocene]dichloropalladium(II) complex with dichloromethane (32 mg, 0.04 mmol), and potassium carbonate (136 mg, 0.99 mmol) were suspended in 1,4-dioxane/water (4:1, 3 mL), then the solution was heated at 105 °C for 2 hours. LC/MS showed complete conversion to intermediate 6-(2-chloro-3-(2-chloro-3-methylpyridin-4-yl)phenyl)-2-methoxynicotinaldehyde. 2-Methoxy-4-(4,4,5,5-tetramethyl-1,3,2-dioxaborolan-2-yl)benzaldehyde (145 mg, 0.55 mmol) was added to the reaction mixture and the resulting solution was stirred at 105 °C for an additional 18 hours. Reaction was cooled to room temperature, diluted with 10 mL water, and extracted with EtOAc (3 x 5 mL). The combined organics were concentrated under reduced pressure and crude sample was purified by silica gel chromatography (0-50% EtOAc/hexane) to afford 6-(2-chloro-3-(2-(4-formyl-3-methoxyphenyl)-3-methylpyridin-4-yl)phenyl)-2-methoxynicotinaldehyde (30 mg, 16 % yield) as a yellow film. MS: *m*/*z* found 473 [M+H]⁺.

### (b) 1-(4-(((6-(3-(2-(4-(((1-Acetylpiperidin-4-yl)amino)methyl)-3-methoxyphenyl)-3-methylpyridin-4-yl)-2-chlorophenyl)-2-methoxypyridin-3-yl)methyl)amino)piperidin-1-yl)ethan-1-one

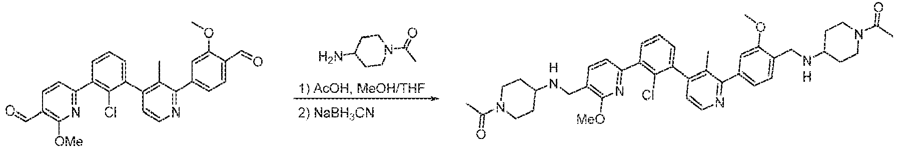

6-(2-Chloro-3-(2-(4-formyl-3-methoxyphenyl)-3-methylpyridin-4-yl)phenyl)-2-methoxynicotinaldehyde (10 mg, 0.02 mmol), acetic acid (3 mg, 0.04 mmol) and 1-(4-amino-1-piperidyl)ethanone (12 mg, 0.08 mmol) were dissolved in MeOH/THF (1:1, 1 mL), then the solution was stirred at 25 °C for 2 hours. Sodium cyanoborohydride (5 mg, 0.08 mmol) was added and the resulting mixture was stirred at 25 °C for 1 hour. Reaction was subsequently diluted with water (2 mL) and extracted with EtOAc (3 x 2 mL). The combined organics were further washed with brine, dried over sodium sulfate, filtered, and concentrated under reduced pressure. Crude sample was purified by reverse phase HPLC to afford 1-(4-(((6-(3-(2-(4-(((1-acetylpiperidin-4-yl)amino)methyl)-3-methoxyphenyl)-3-methylpyridin-4-yl)-2-chlorophenyl)-2-methoxypyridin-3-yl)methyl)amino)piperidin-1-yl)ethan-1-one (8 mg, 54 % yield) as a white solid (formic acid salt). LC/MS: *m*/*z* found 725, 727 [M+H]⁺, retention time = 2.01 min (Method C). ¹H NMR (400 MHz, Methanol-*d*₄) δ 8.52 (s, 1H), 8.46 (s, 2H), 7.85 (d, 1H), 7.68 (d, 1H), 7.54 (dd, 2H), 7.40 (d, 1H), 7.32 (d, 2H), 7.23 (s, 1H), 7.16 (d, 1H), 4.63 (t, 2H), 4.29 (s, 2H), 4.16 (s, 2H), 4.06 (s, 5H), 3.98 (s, 3H), 3.41 (s, 1H), 3.17 (d, 3H), 2.67 (d, 2H), 2.20 (d, 4H), 2.14 - 2.07 (m, 9H), 1.74 - 1.30 (m, 4H).

### Example 384: (S)-1-(((6-(2-Chloro-3-(2-(4-((((S)-2-hydroxypropyl)amino)methyl)-3-methoxyphenyl)-3-methylpyridin-4-yl)-phenyl)-2-methoxypyridin-3-yl)-methyl)-amino)propan-2-ol (274)

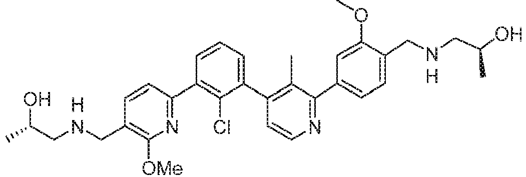

(S)-1-(((6-(2-Chloro-3-(2-(4-((((S)-2-hydroxypropyl)amino)methyl)-3-methoxyphenyl)-3-methylpyridin-4-yl)-phenyl)-2-methoxypyridin-3-yl)-methyl)-amino)propan-2-ol was prepared in the same manner as described for Example 383, utilizing intermediate 6-(2-chloro-3-(2-(4-formyl-3-methoxyphenyl)-3-methylpyridin-4-yl)phenyl)-2-methoxynicotinaldehyde and replacing 1-(4-amino-1-piperidyl)-ethanone with (S)-1-aminopropan-2-ol in step (b). Product was obtained as a white solid (formic acid salt). LC/MS: *m*/*z* found 591, 593 [M+H]⁺, retention time = 1.98 min (Method C). ¹H NMR (400 MHz, Methanol-*d*₄) δ 8.53 (d, 1H), 7.85 (d, 1H), 7.69 (d, 1H), 7.56 (t, 1H), 7.50 (d, 1H), 7.40 (d, 1H), 7.32 (t, 2H), 7.23 (s, 1H), 7.16 (d, 1H), 4.29 (s, 2H), 4.20 (s, 2H), 4.06 (d, 5H), 3.98 (s, 3H), 3.04 (t, 2H), 2.84 (q, 2H), 2.12 (s, 3H), 1.22 (d, 6H).

### Example 385. 1-(6-(2-Chloro-3-(2-(3-methoxy-4-((methylamino)methyl)phenyl)-3-methylpyridin-4-yl)phenyl)-2-methoxypyridin-3-yl)-N-methylmethanamine (334)

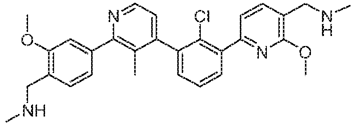

### (a) 6-(2-Chloro-3-(2-chloro-3-methylpyridin-4-yl)phenyl)-2-methoxynicotinaldehyde

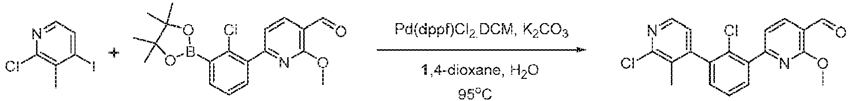

To a solution of 6-(2-chloro-3-(4,4,5,5-tetramethyl-1,3,2-dioxaborolan-2-yl)phenyl)-2-methoxynicotinaldehyde (6 g, 16.1 mmol) and 2-chloro-4-iodo-3-methylpyridine (4.07 g, 16.1 mmol) in 1,4-dioxane/water mixture (10:1, 120 mL) was added potassium carbonate (6.66 g, 48.2 mmol) and [1,1'- bis(diphenylphosphino)ferrocene]dichloropalladium(II) complex with dichloromethane (0.39 g, 0.48 mmol) and the mixture was stirred at 95 °C for 4 h. The reaction was filtered, concentrated and diluted with water (50 mL). The aqueous layer was extracted with ethyl acetate (2 x 40 mL). The combined organic phases were dried with anhydrous sodium sulfate, filtered and concentrated. The residue was purified by normal phase SiO₂ chromatography (0-20% ethyl acetatc/petroleum ether) to afford 6-(2-chloro-3-(2-chloro-3-methylpyridin-4-yl)phenyl)-2-methoxynicotinaldehyde (4.7 g, 78% yield). ¹H NMR (400 MHz, Chloroform-d): δ 10.36 (s, 1H), 8.25 (m, 1H), 8.14 (d, 1H), 7.65 (d, 1H), 7.45 (t, 1H), 7.35 (d, 1H), 7.19 (s, 1H), 7.09 (d, 1H), 4.06 (s, 3H), 2.15 (s, 3H).

### (b) 6-(2-Chloro-3-(2-(4-formyl-3-methoxyphenyl)-3-methylpyridin-4-yl)phenyl)-2-methoxynicotinaldehyde

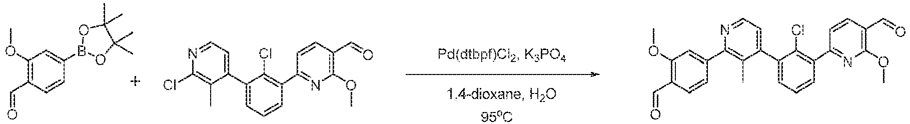

To a solution of 6-(2-chloro-3-(2-chloro-3-methylpyridin-4-yl)phenyl)-2-methoxynicotinaldehyde (1.5 g, 4.02 mmol) and 2-methoxy-4-(4,4,5,5-tetramethyl-1,3,2-dioxaborolan-2-yl)benzaldehyde (2.11 g, 8.04 mmol) in 1,4-dioxane/water mixture (10:1, 33 mL) was added potassium phosphate (2.56 g, 12.1 mmol) and [1,1'-bis(di-tert-butylphosphino)ferrocene]dichloropalladium(II) (0.13 g, 0.2 mmol) and the mixture was stirred at 95 °C for 4 h. The mixture was concentrated followed by addition of water (10mL). The aqueous layer was extracted with ethyl acetate (2 x 15 mL). The combined organic phases were dried with anhydrous sodium sulfate, filtered and concentrated. The residue was purified by normal phase SiO₂ chromatography (0-100% ethyl acetate/petroleum ether) to afford 6-(2-chloro-3-(2-(4-formyl-3-methoxyphenyl)-3-methylpyridin-4-yl)phenyl)-2-methoxynicotinaldehyde (0.23 g, 12% yield). ¹H NMR (400 MHz, Chloroform-*d*): δ 10.41 (s, 2H), 7.77-7.74 (m, 2H), 7.48-7.46 (m, 2H), 6.97-6.91 (m, 5H), 3.86 (s, 6H), 1.97 (s, 3H).

### (c) 1-(6-(2-Chloro-3-(2-(3-methoxy-4-((methylamino)methyl)phenyl)-3-methylpyridin-4-yl)phenyl)-2-methoxypyridin-3-yl)-N-methylmethanamine

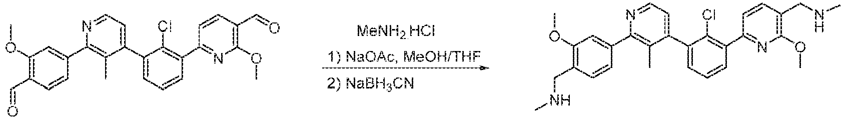

To a mixture of 6-(2-chloro-3-(2-(4-formyl-3-methoxyphenyl)-3-methylpyridin-4-yl)phenyl)-2-methoxynicotinaldehyde (0.17 g, 0.95 mmol) in methanol (0.5 mL) and THF (0.5 mL) was added sodium acetate (0.19 g, 2.28 mmol) and methylamine hydrochloride. The mixture was stirred at room temperature for 2 h. To the mixture was added sodium cyanoborohydride (0.096 g, 1.52 mmol) and the mixture was stirred at room temperature for 30 minutes. The mixture was concentrated, and the residue was purified by reverse phase HPLC to afford 1-(6-(2-chloro-3-(2-(3-methoxy-4-((methylamino)methyl)phenyl)-3-methylpyridin-4-yl)phenyl)-2-methoxypyridin-3-yl)-N-methylmethanamine (25.4 mg, 9% yield) as a white solid (formic acid salt). MS: m/z found 503 [M+H]⁺, retention time = 1.88 min (Method A). ¹H NMR (400 MHz, Methanol-*d*₄) : δ 8.52 (d, 1H), 8.51 (m, 2H), 7.85 (d, 1H), 7.69 (dd, 1H), 7.57 (t, 1H), 7.49 (d, 1H), 7.41 (dd, 1H), 7.35-7.31 (m, 2H), 7.24 (d, 1H), 7.16 (dd, 1H), 4.25 (s, 2H), 4.21 (s, 2H) , 4.08 (s, 3H), 3.98 (s, 3H), 2.73 (s, 6H), 2.11 (s, 3H).

### Example 386: 2-((6-(2-Chloro-3-(2-(3-methoxy-4-((7-oxo-2,6-diazaspiro[3.4]octan-2-yl)methyl)phenyl)-3-methylpyridin-4-yl)phenyl)-2-methoxypyridin-3-yl)methyl)-2,6-diazaspiro[3.4]octan-7-one (368)

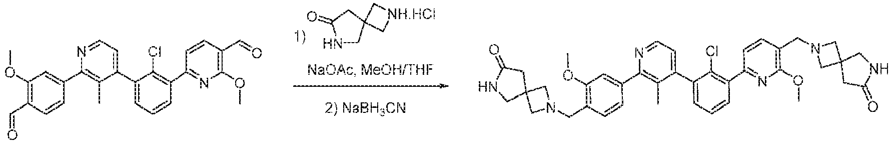

To a mixture of 6-(2-chloro-3-(2-(4-formyl-3-methoxyphenyl)-3-methylpyridin-4-yl)phenyl)-2-methoxynicotinaldehyde (Example 385, step (b)) (0.15 g, 0.32 mmol) and 2,6-diazaspiro[3.4]octan-7-one hydrochloride salt (0.11 g, 0.7 mmol) in THF/MeOH mixture (1:1, 1 mL) was added sodium acetate (0.16 g, 1.9 mmol). The mixture was stirred at room temperature for 1 h. To the mixture was added sodium cyanoborohydride (0.08 g, 1.27 mmol) and the mixture was stirred at room temperature for 0.5 h. The mixture was concentrated, and the residue was purified by reverse phase HPLC to afford 2-((6-(2-chloro-3-(2-(3-methoxy-4-((7-oxo-2,6-diazaspiro[3.4]octan-2-yl)methyl)phenyl)-3-methylpyridin-4-yl)phenyl)-2-methoxypyridin-3-yl)methyl)-2,6-diazaspiro[3.4]octan-7-one (47.5 mg, 16% yield) as a yellow solid (formic acid salt). MS: m/z found 693 [M+H]⁺, retention time = 1.85 min (Method A). ¹H NMR (400 MHz, Methanol-*d*₄): δ 8.54 (d, 1H), 8.42 (br s, 2H), 7.81 (d, 1H), 7.70 (dd, 1H), 7.57 (t, 1H), 7.52 (d, 1H), 7.41 (dd, 1H), 7.34-7.30 (m, 2H), 7.24 (s, 1H), 7.18 (dd, 1H), 4.39 (s, 2H), 4.15 (s, 4H), 4.10 (s, 2H), 4.05 (s, 3H), 3.99 (s, 3H), 3.87 (m, 4H), 3.68 (d, 4H), 2.73-2.68 (m, 4H), 2.13 (s, 3H).

### Example 387: 1-(6-((6-(3-(2-(4-((6-Acetyl-2,6-diazaspiro[3.3]heptan-2-yl)methyl)-3-methoxyphenyl)-3-methylpyridin-4-yl)-2-chlorophenyl)-2-methoxypyridin-3-yl)methyl)-2,6-diazaspiro[3.3]heptan-2-yl)ethan-1-one (369)

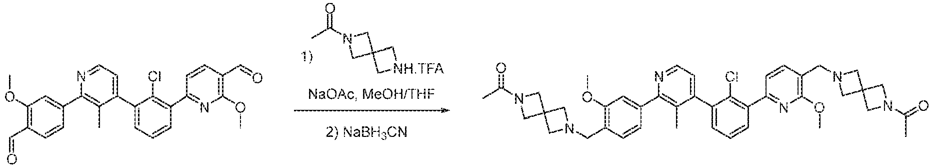

To a mixture of 6-(2-chloro-3-(2-(4-formyl-3-methoxyphenyl)-3-methylpyridin-4-yl)phenyl)-2-methoxynicotinaldehyde (Example 385, step (b)) (0.1 g, 0.21 mmol) and 1-(2,6-diazaspiro[3.3]heptan-2-yl)ethanone trifluoroacetic acid salt (0.11 g, 0.42 mmol) in THF/methanol mixture (1:1, 4 mL) was added sodium acetate (0.1 g, 1.27 mmol) and the mixture was stirred at room temperature for 1 h. To the mixture was added sodium cyanoborohydride (0.05 g, 0.85 mmol) and the mixture was stirred at room temperature for 0.5 h. The mixture was concentrated and the residue was purified by reverse phase HPLC to afford 1-(6-((6-(3-(2-(4-((6-acetyl-2,6-diazaspiro[3.3]heptan-2-yl)methyl)-3-methoxyphenyl)-3-methylpyridin-4-yl)-2-chlorophenyl)-2-methoxypyridin-3-yl)methyl)-2,6-diazaspiro[3.3]heptan-2-yl)ethan-1-one (48 mg, 25% yield) as a white solid (formic acid salt). MS: m/z found 721 [M+H]⁺, retention time = 2.05 min (Method A). ¹H NMR (400 MHz, Methanol-*d*₄): δ 8.55 (d, 1H), 8.38 (br s, 1H), 7.81 (d, 1H), 7.70 (dd, 1H), 7.58 (t, 1H), 7.51 (d, 1H), 7.42 (dd, 1H), 7.34-7.31 (m, 2H), 7.26 (s, 1H), 7.19 (d, 1H), 4.40 (s, 2H), 4.37 (s, 4H), 4.26 (s, 4H), 4.16-4.10 (m, 6H), 4.03 (s, 3H), 4.00-3.97 (m, 7H), 2.13 (s, 3H), 1.87 (s, 6H).

### Example 388: (S)-5-((((6-(2-Chloro-3-(2-(3-methoxy-4-(((((S)-5-oxopyrrolidin-2-yl)methyl)amino)methyl)phenyl)-3-methylpyridin-4-yl)phenyl)-2-methoxypyridin-3-yl)methyl)amino)methyl)pyrrolidin-2-one (414)

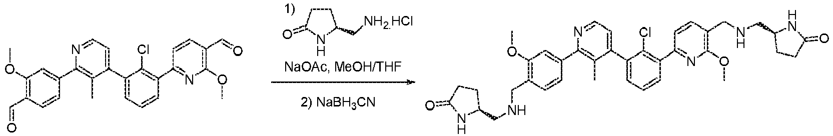

To a mixture of 6-(2-chloro-3-(2-(4-formyl-3-methoxyphenyl)-3-methylpyridin-4-yl)phenyl)-2-methoxynicotinaldehyde (Example 385, step (b)) (0.18 g, 0.38 mmol) and (S)-5-(aminomethyl)pyrrolidin-2-one hydrochloride salt (0.14 g, 0.95 mmol) in THF/water mixture (1:3, 4 mL) was added sodium acetate (0.19 g, 2.28 mmol) and the mixture was stirred at room temperature for 2 h. Sodium cyanoborohydride (0.1 g, 1.52 mmol) was added to the mixture and the mixture was stirred at room temperature for 1 h. The mixture was concentrated, and the residue was purified by reverse phase HPLC to afford (S)-5-((((6-(2-chloro-3-(2-(3-methoxy-4-(((((S)-5-oxopyrrolidin-2-yl)methyl)amino)methyl)phenyl)-3-methylpyridin-4-yl)phenyl)-2-methoxypyridin-3-yl)methyl)amino)methyl)pyrrolidin-2-one (27.5 mg, 10% yield) as a white solid (formic acid salt). MS: m/z found m/z: 669 [M+H]⁺, retention time = 1.87 min (Method A). ¹H NMR (400 MHz, Methanol-*d*₄): δ 8.53 (d, 1H), 8.35 (br s, 2H), 7.81 (d, 1H), 7.68 (dd, 1H), 7.55 (t, 1H), 7.50 (d, 1H), 7.39 (dd, 1H), 7.32-7.28 (m, 2H), 7.23 (d, 1H), 7.15 (dd, 1H), 4.28-4.21 (m, 2H), 4.04-3.98 (m, 9H), 3.93-3.92 (m, 1H), 3.14-3.09 (m, 2H), 2.93-2.89 (m, 2H), 2.41-2.32 (m, 6H), 2.12 (s, 3H), 1.90-1.85 (m, 2H).

### Example 389: N-((6-(2-Chloro-3-(2-(3-methoxy-4-(((tetrahydro-2H-pyran-4-yl)amino)methyl)phenyl)-3-methylpyridin-4-yl)phenyl)-2-methoxypyridin-3-yl)methyl)tetrahydro-2H-pyran-4-amine

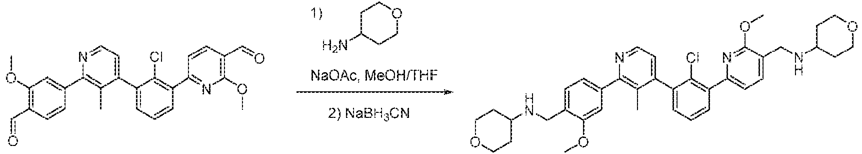

To a mixture of 6-(2-chloro-3-(2-(4-formyl-3-methoxyphenyl)-3-methylpyridin-4-yl)phenyl)-2-methoxynicotinaldehyde (Example 385, step (b)) (0.11 g, 0.23 mmol) and tetrahydro-2H-pyran-4-amine (0.06 g, 0.58 mmol) in THF/MeOH mixture (1:1, 2 mL) was added sodium acetate (0.08 g, 0.93 mmol) and the mixture was stirred at room temperature for 1 h. Sodium cyanoborohydride (0.07 g, 1.05 mmol) was added and the mixture was stirred at room temperature for 0.5 h. The mixture was concentrated, and the residue was purified by reverse phase HPLC to afford N-((6-(2-chloro-3-(2-(3-methoxy-4-(((tetrahydro-2H-pyran-4-yl)amino)methyl)phenyl)-3-methylpyridin-4-yl)phenyl)-2-methoxypyridin-3-yl)methyl)tetrahydro-2H-pyran-4-amine (43.4 mg, 26% yield) as a white solid (formic acid salt). MS: m/z found 643 [M+H]⁺, retention time = 2.18 min (Method A). ¹H NMR (400 MHz, Methanol-*d*₄): δ 8.55 (d, 1H), 8.50 (br s, 2H), 7.88 (d, 1H), 7.71 (dd, 1H), 7.61-7.53 (m, 2H), 7.43 (dd, 1H), 7.36-7.33 (m, 2H), 7.26 (s, 1H), 7.19 (d, 1H), 4.31 (s, 2H), 4.20 (s, 2H), 4.09-4.04 (m, 7H), 4.01 (s, 3H), 3.51-3.42 (m, 6H), 2.14-2.08 (m, 7H), 1.76-1.67 (m, 4H).

### Example 390: (1r,4r)-4-(((6-(2-Chloro-3-(2-(4-((((1r,4r)-4-hydroxycyclohexyl)amino)methyl)-3-methoxyphenyl)-3-methylpyridin-4-yl)phenyl)-2-methoxypyridin-3-yl)methyl)amino)cyclohexan-1-ol (463)

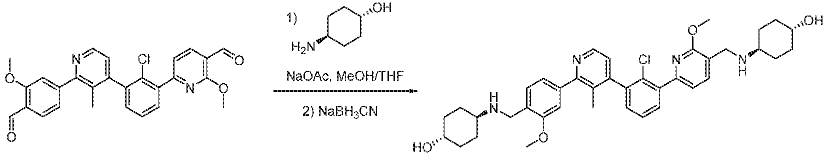

To a mixture of 6-(2-chloro-3-(2-(4-formyl-3-methoxyphenyl)-3-methylpyridin-4-yl)phenyl)-2-methoxynicotinaldehyde (Example 385, step (b)) (0.1 g, 0.21 mmol) and (1r,4r)-4-aminocyclohexanol (0.06 g, 0.49 mmol) in THF/MeOH mixture (1:1, 2 mL) was added sodium acetate (0.07 g, 0.85 mmol) and the mixture was stirred at room temperature for 12 h. Sodium cyanoborohydride (0.06 g, 0.95 mmol) was added and the mixture was stirred at room temperature for 1 h. The mixture was concentrated, and the residue was purified by reverse phase HPLC to afford (1r,4r)-4-(((6-(2-chloro-3-(2-(4-((((1r,4r)-4-hydroxycyclohexyl)amino)methyl)-3-methoxyphenyl)-3-methylpyridin-4-yl)phenyl)-2-methoxypyridin-3-yl)methyl)amino)cyclohexan-1-ol (31.6 mg, 19% yield) as a white solid (formic acid salt). MS: m/z found m/z: 671 [M+H]⁺, retention time = 2.04 min (Method A). ¹H NMR (400 MHz, Methanol-*d*₄): δ 8.53 (d, 1H), 8.49 (br s, 2H), 7.87 (d, 1H), 7.68 (dd, 1H), 7.56 (t, 1H), 7.51 (d, 1H), 7.41 (dd, 1H), 7.34-7.31 (m, 2H), 7.23 (d, 1H), 7.16 (dd, 1H), 4.28 (s, 2H), 4.22 (s, 2H), 4.07 (s, 3H), 3.98 (s, 3H), 3.61-3.54 (m, 2H), 3.14-3.05 (m, 2H), 2.23-2.20 (m, 4H), 2.11-2.03 (m, 7H), 1.58-1.49 (m, 4H), 1.41-1.32 (m, 4H).

### Example 391: 6-(4-(4-(3-(5-((2-Oxa-6-azaspiro[3.3]heptan-6-yl)methyl)-6-methoxypyridin-2-yl)-2-chlorophenyl)-3-methylpyridin-2-yl)-2-methoxybenzyl)-2-oxa-6-azaspiro[3.3]heptane (464)

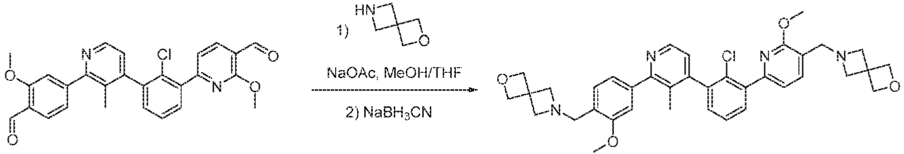

To a mixture of 6-(2-chloro-3-(2-(4-formyl-3-methoxyphenyl)-3-methylpyridin-4-yl)phenyl)-2-methoxynicotinaldehyde (Example 385, step (b)) (0.11 g, 0.23 mmol) and 2-oxa-6-azaspiro[3.3]heptane (0.05 g, 0.53 mmol) in THF/methanol mixture (1:1, 1 mL) was added sodium acetate (0.08 g, 0.93 mmol) and the mixture was stirred at room temperature for 0.5 h. Sodium cyanoborohydride (0.07 g, 1.05 mmol) was added and the mixture was stirred at room temperature for 0.5 h. The mixture was concentrated, and the residue was purified by reverse phase HPLC to afford 6-(4-(4-(3-(5-((2-oxa-6-azaspiro[3.3]heptan-6-yl)methyl)-6-methoxypyridin-2-yl)-2-chlorophenyl)-3-methylpyridin-2-yl)-2-methoxybenzyl)-2-oxa-6-azaspiro[3.3]heptane (13.5 mg, 8% yield) as a yellow solid (formic acid salt). MS: m/z found 639 [M+H]⁺, retention time = 2.01 min (Method A). ¹H NMR (400 MHz, Methanol-*d*₄): δ 8.52 (d, 1H), 8.39 (br s, 2H), 7.76-7.74 (m, 1H), 7.68 (dd, 1H), 7.55 (t, 1H), 7.46 (d, 1H), 7.38 (dd, 1H), 7.32-7.28 (m, 2H), 7.22 (s, 1H), 7.15 (d, 1H), 4.78-4.74 (m, 8H), 4.32-4.23 (m, 6H), 4.15-4.13 (m, 1H), 4.02-3.89 (m, 10H), 3.70-3.67 (m, 1H), 2.11 (s, 3H).

### Example 392: N-(1-((6-(3-(2-(4-((4-Acetamidopiperidin-1-yl)methyl)-3-methoxyphenyl)-3-methylpyridin-4-yl)-2-chlorophenyl)-2-methoxypyridin-3-yl)methyl)piperidin-4-yl)acetamide (466)

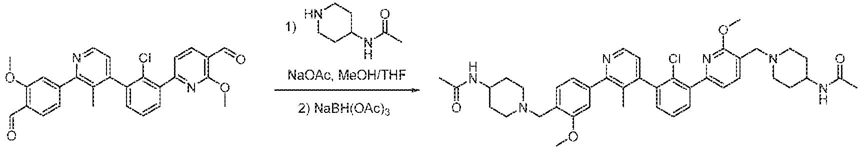

To a mixture of 6-(2-chloro-3-(2-(4-formyl-3-methoxyphenyl)-3-methylpyridin-4-yl)phenyl)-2-methoxynicotinaldehyde (Example 385, step (b)) (0.1 g, 0.21 mmol) and N-(piperidin-4-yl)acetamide (0.09 g, 0.63 mmol) in THF/methanol mixture (2:3, 5 mL) was added sodium acetate (0.07 g, 0.85 mmol) and the mixture was stirred at room temperature for 8 h. To the mixture was added sodium triacetoxyborohydride (0.22 g, 1.06 mmol) and the mixture was stirred at room temperature for 8 h. The mixture was concentrated, and the residue was purified by reverse phase HPLC to afford N-(1-((6-(3-(2-(4-((4-acetamidopiperidin-1-yl)methyl)-3-methoxyphenyl)-3-methylpyridin-4-yl)-2-chlorophenyl)-2-methoxypyridin-3-yl)methyl)piperidin-4-yl)acetamide (29.6 mg, 17% yield) as a yellow solid (formic acid salt). MS: m/z found 725 [M+H]⁺, retention time = 2.03 min (Method A). ¹H NMR (400 MHz, Methanol-*d*₄): 8.56 (d, 1H), 8.41 (br s, 2H), 7.85 (d, 1H), 7.72 (d, 1H), 7.60-7.54 (m, 2H), 7.42 (d, 1H), 7.35-7.32 (m, 2H), 7.26 (s, 1H), 7.20 (d, 1H), 4.28 (s, 2H), 4.05 (s, 3H), 3.99 (s, 3H), 3.92 (m, 3H), 3.82-3.76 (m, 1H), 3.45 (m, 1H), 3.21-3.05 (m, 4H), 2.68-2.62 (m, 2H), 2.15-2.09 (m, 6H), 2.00-1.95 (m, 8H), 1.82-1.65 (m, 4H).

### Example 393: 1-(2-Methoxy-4-(4-(3-(6-methoxy-5-((methylamino)methyl)pyridin-2-yl)-2-methylphenyl)-3-methylpyridin-2-yl)phenyl)-N-methylmethanamine (362)

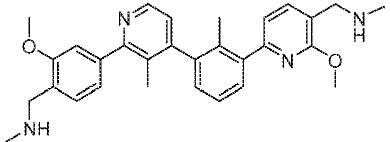

### (a) 2-Methoxy-6-(2-methyl-3-(4,4,5,5-tetramethyl-1,3,2-dioxaborolan-2-yl)phenyl)nicotinaldehyde

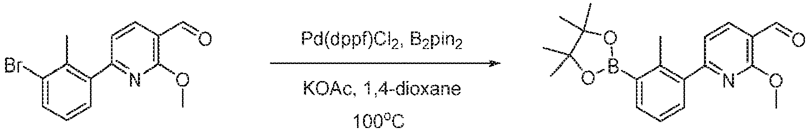

A mixture of 6-(3-bromo-2-methylphenyl)-2-methoxynicotinaldehyde (Example 344, step (b)) (1.95 g, 6.37 mmol), bis(pinacolato)diborane (3.23 g, 12.74 mmol), potassium acetate (1.88 g, 19.11 mmol) and [1,1'-bis(diphenylphosphino)ferrocene]dichloropalladium(II) (466 mg, 637 umol) in 1,4-dioxane (30 mL) was degassed and purged with N₂ three times, and then the mixture was stirred at 100 °C for 3 h under N₂ atmosphere. The mixture was combined with another batch at 50 mg scale. The reaction mixture was quenched by addition of water (30 mL) and extracted with ethyl acetate (3 x 25 mL). The combined organic layers were washed with saturated aqueous brine solution (2 x 20 mL), dried over anhydrous sodium sulfate, filtered and concentrated under reduced pressure. The residue was purified by normal phase SiO₂ chromatography (0-20% ethyl acetate/petroleum ether) to afford 2-methoxy-6-(2-methyl-3-(4,4,5,5-tetramethyl-1,3,2-dioxaborolan-2-yl)phenyl)nicotinaldehyde (1.9 g, 84% yield) as a yellow solid. ¹H NMR (400MHz, CDCl₃): δ 10.42 (s, 1H), 8.17 (d, 1H), 7.85 (dd, 1H), 7.47 (dd, 1H), 7.31 - 7.27 (m, 1H), 7.11 (d, 1H), 4.09 (s, 3H), 2.59 (s, 3H), 1.38 (s, 12H), 1.27 (s, 9H).

### (b) 6-(3-(2-Chloro-3-methylpyridin-4-yl)-2-methylphenyl)-2-methoxynicotinaldehyde

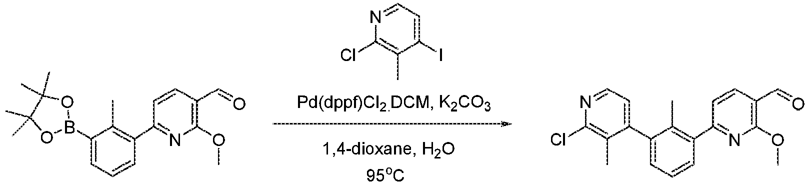

A mixture of 2-methoxy-6-(2-methyl-3-(4,4,5,5-tetramethyl-1,3,2-dioxaborolan-2-yl)phenyl)nicotinaldehyde (1.9 g, 5.38 mmol), 2-chloro-4-iodo-3-methylpyridine (2.73 g, 10.76 mmol), potassium carbonate (2.23 g, 16.14 mmol) and [1,1'-bis(diphenylphosphino)ferrocene]-dichloropalladium(II) complex with dichloromethane (439 mg, 538 umol) in 1,4-dioxane / water (10:1, 33 mL) was degassed and purged with N₂ three times, and then the mixture was stirred at 95 °C for 3 h under N₂ atmosphere. The mixture was combined with another batch at 50 mg scale. The reaction mixture was quenched by addition water (30 mL) and extracted with ethyl acetate (3 x 25 mL). The combined organic layers were washed with saturated aqueous brine solution (2 x 20 mL), dried over anhydrous sodium sulfate, filtered and concentrated under reduced pressure. The residue was purified by normal phase SiO₂ chromatography (10-50% ethyl acetate/petroleum ether) to afford 6-(3-(2-chloro-3-methylpyridin-4-yl)-2-methylphenyl)-2-methoxynicotinaldehyde (1 g, 52% yield) as yellow oil. ¹H NMR (400MHz, CDCl₃): δ 10.43 (s, 1H), 8.29 (d, 1H), 8.21 (d, 1H), 7.52 (dd, 1H), 7.39 (t, 1H), 7.20 - 7.14 (m, 2H), 7.09 (d, 1H), 4.11 (s, 3H), 2.19 (s, 3H), 2.11 (s, 3H).

### (c) 6-(3-(2-(4-Formyl-3-methoxyphenyl)-3-methylpyridin-4-yl)-2-methylphenyl)-2-methoxynicotinaldehyde

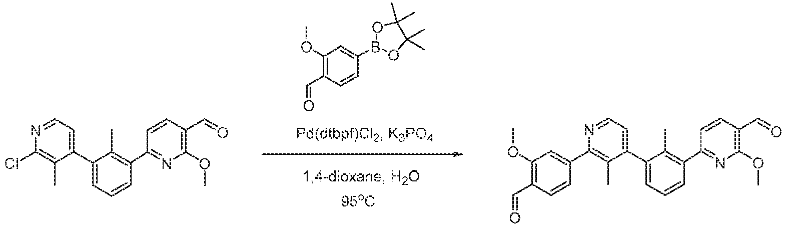

A mixture of 6-(3-(2-chloro-3-methylpyridin-4-yl)-2-methylphenyl)-2-methoxynicotinaldehyde (0.95 g, 2.69 mmol), 2-methoxy-4-(4,4,5,5-tetramethyl-1,3,2-dioxaborolan-2-yl)benzaldehyde (1.76 g, 6.73 mmol), potassium phosphate (1.71 g, 8.08 mmol) and [1,1'-bis(di-tert-butylphosphino)ferrocene]dichloropalladium(II) (175.5 mg, 269 umol) in 1,4-dioxane / water (10:1, 22 mL) was degassed and purged with N₂ three times, and then the mixture was stirred at 95 °C for 2 hr under N₂ atmosphere. The mixture was combined with another batch at 20 mg scale. The reaction mixture was quenched by the addition of water (30 mL) and extracted with ethyl acetate (3 x 25 mL). The combined organic layers were washed with saturated aqueous brine solution (2 x 15 mL), dried over anhydrous sodium sulfate, filtered and concentrated under reduced pressure. The residue was purified by normal phase SiO₂ chromatography (10-50% ethyl acetate/petroleum ether) to afford 6-(3-(2-(4-formyl-3-methoxyphenyl)-3-methylpyridin-4-yl)-2-methylphenyl)-2-methoxynicotinaldehyde (1 g, 82% yield) as a white solid. ¹H NMR (400 MHz, CDCl₃): δ 10.53 (s, 1H), 10.43 (s, 1H), 8.61 (d, 1H), 8.22 (d, 1H), 7.92 (d, 1H), 7.52 (dd, 1H), 7.41 (t, 1H), 7.24 - 7.19 (m, 5H), 4.11 (s, 3H), 4.03 - 3.99 (m, 3H), 2.18 (s, 3H), 2.11 (s, 3H).

### (d) 1-(2-Methoxy-4-(4-(3-(6-methoxy-5-((methylamino)methyl)pyridin-2-yl)-2-methylphenyl)-3-methylpyridin-2-yl)phenyl)-N-methylmethanamine

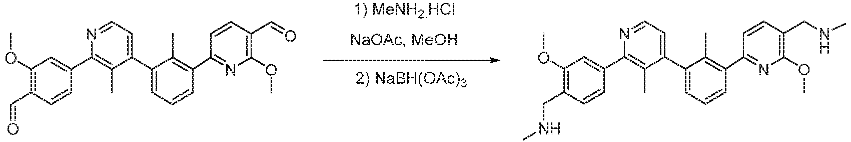

To a solution of 6-(3-(2-(4-formyl-3-methoxyphenyl)-3-methylpyridin-4-yl)-2-methylphenyl)-2-methoxynicotinaldehyde (150 mg, 331.5 umol) in MeOH (5 mL) was added methylamine hydrochloride (68.5 mg, 994.5 umol) and sodium acetate (108.8 mg, 1.33 mmol) and the solution was stirred at room temperature for 12 h. Then sodium triacetoxyborohydride (140.5 mg, 663 umol) was added and the resulting solution stirred at 20°C for 1 h. The reaction was concentrated under reduced pressure and the residue was purified by reverse phase HPLC to afford 1-(2-methoxy-4-(4-(3-(6-methoxy-5-((methylamino)methyl)pyridin-2-yl)-2-methylphenyl)-3-methylpyridin-2-yl)phenyl)-N-methylmethanamine (24.2 mg, 15% yield) as a white solid (formic acid salt). MS: m/z found 483.3 [M+H]⁺, retention time = 1.70 min (Method A). ¹H NMR (400 MHz, Methanol-*d*₄): δ 8.52 - 8.50 (m, 3H), 7.87 (d, 1H), 7.51 - 7.49 (m, 2H), 7.42 (t, 1H), 7.29 (d, 1H), 7.26 - 7.23 (m, 2H), 7.21 - 7.17 (m, 2H), 4.26 (s, 2H), 4.22 (s, 2H), 4.06 (s, 3H), 3.99 (s, 3H), 2.74 (d, 6H), 2.13 (s, 3H), 2.08 (s, 3H).

### Example 394: 1-(4-(((6-(3-(2-(4-(((1-Acetylpiperidin-4-yl)amino)methyl)-3-methoxyphenyl)-3-methylpyridin-4-yl)-2-methylphenyl)-2-methoxypyridin-3-yl)methyl)amino)piperidin-1-yl)ethan-1-one (363)

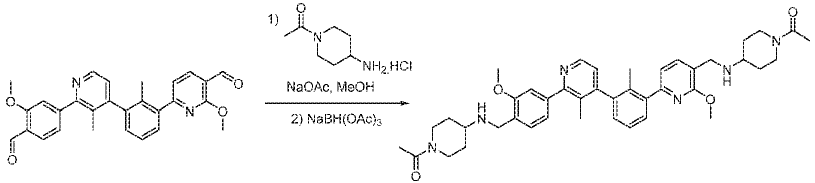

To a solution of 6-(3-(2-(4-formyl-3-methoxyphenyl)-3-methylpyridin-4-yl)-2-methylphenyl)-2-methoxynicotinaldehyde (Example 393, step (c)) (150 mg, 331.5 umol) and 1-(4-amino-1- piperidyl)ethanone as hydrochloride salt (177.7 mg, 994.5 umol) in methanol (3 mL) was added sodium acetate (108.8 mg, 1.33 mmol) and the solution was stirred at room temperature for 12 h. Then sodium triacetoxyborohydride (70.3 mg, 331.5 umol) was added and the solution was stirred at room temperature for 1 h. The reaction was concentrated under reduced pressure and the residue purified by reverse phase HPLC to afford 1-(4-(((6-(3-(2-(4-(((1-acetylpiperidin-4-yl)amino)methyl)-3-methoxyphenyl)-3-methylpyridin-4-yl)-2-methylphenyl)-2-methoxypyridin-3-yl)methyl)amino)piperidin-1-yl)ethan-1-one (80 mg, 34% yield) as a white solid (formic acid salt). MS: m/z found 705.4 [M+H]⁺, retention time = 2.02 min (Method A). ¹H NMR (400 MHz, Methanol-*d*₄): δ 8.51 - 8.49 (m, 2H), 7.88 (d, 1H), 7.54 - 7.48 (m, 2H), 7.41 (t, 1H), 7.29 (d, 1H), 7.25 - 7.16 (m, 4H), 4.68 - 4.62 (m, 2H), 4.31 (s, 2H), 4.22 (s, 2H), 4.08 - 4.05 (m, 5H), 3.99 (s, 3H), 3.45 - 3.34 (m, 2H), 3.24 - 3.17 (m, 2H), 2.75 - 2.67 (m, 2H), 2.28 - 2.18 (m, 4H), 2.14 (d, 9H), 2.07 (s, 3H), 1.70 - 1.45 (m, 4H).

### Example 395: N-(1-((6-(3-(2-(4-((4-Acetamidopiperidin-1-yl)methyl)-3-methoxyphenyl)-3-methylpyridin-4-yl)-2-methylphenyl)-2-methoxypyridin-3-yl)methyl)piperidin-4-yl)acetamide (461)

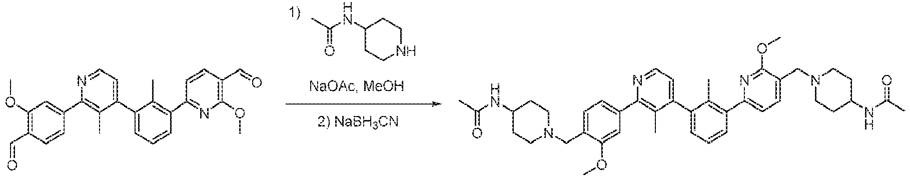

To a solution of 6-(3-(2-(4-formyl-3-methoxyphenyl)-3-methylpyridin-4-yl)-2-methylphenyl)-2-methoxynicotinaldehyde (Example 393, step (c)) (140 mg, 309.4 umol) and N-(piperidin-4-yl)acetamide (132 mg, 928 umol) in methanol (10 mL) was added sodium acetate (101.5 mg, 1.24 mmol) and the solution was stirred at room temperature for 12 h. Then sodium cyanoborohydride (77.8 mg, 1.24 mmol) was added and the solution was stirred at room temperature for 2 h. The reaction mixture was concentrated under reduced pressure and the residue was purified by reverse phase HPLC to afford N-(1-((6-(3-(2-(4-((4-acetamidopiperidin-1-yl)methyl)-3-methoxyphenyl)-3-methylpyridin-4-yl)-2-methylphenyl)-2-methoxypyridin-3-yl)methyl)piperidin-4-yl)acetamide (51.7 mg, 22% yield) as a white solid (formic acid salt). MS: m/z found 705.3 [M+H]⁺, retention time = 1.95 min (Method A). ¹H NMR (400 MHz, Methanol-*d*₄): δ 8.51 (d, 1H), 8.44 (br s, 2H), 7.86 (d, 1H), 7.55 - 7.49 (m, 2H), 7.41 (t, 1H), 7.29 (d, 1H), 7.25 - 7.17 (m, 4H), 4.29 (s, 2H), 4.01 (s, 5H), 3.97 (s, 3H), 3.92 - 3.79 (m, 2H), 3.44 (d, 2H), 3.26 (s, 2H), 3.09 (t, 2H), 2.79 (br t, 2H), 2.14 (s, 3H), 2.09 (s, 5H), 2.01 (d, 2H), 1.94 (d, 6H), 1.82 - 1.66 (m, 4H).

### Example 396: (S)-5-((((6-(3-(2-(4-((S)-1-Aminoethyl)-3-methoxyphenyl)-3-chloropyridin-4-yl)-2-chlorophenyl)-2-methoxypyridin-3-yl)methyl)amino)methyl)pyrrolidin-2-one (335)

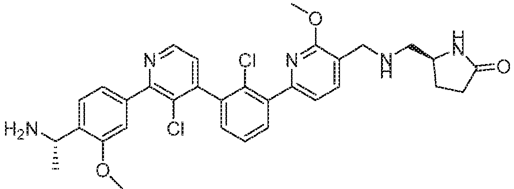

### (a) (S)-N-((S)-1-(4-Bromo-2-methoxyphenyl)ethyl)-2-methylpropane-2-sulfinamide

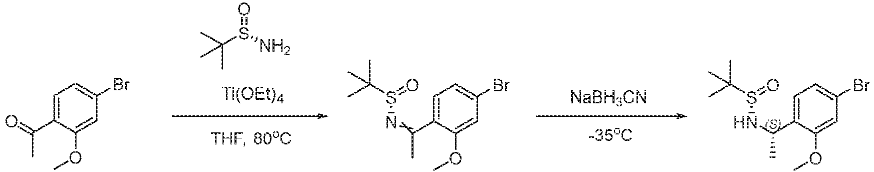

To a mixture of 1-(4-bromo-2-methoxyphenyl)ethanone (0.2 g, 0.87 mmol) and (S)-2-methylpropane-2-sulfinamide (0.21 g, 1.75 mmol) in THF (10 mL) was added titanium(IV) ethoxide (2 mL, 2.62 mmol) in one portion under N₂. The mixture was stirred at 80 °C for 48 hr to give (S)-N-(1-(4-bromo-2-methoxyphenyl)ethylidene)-2-methylpropane-2-sulfinamide as a white suspensions (MS: m/z found 332 and 334 [M+H]⁺). The mixture was cooled to -35 °C and sodium cyanoborohydride (0.17 g, 4.37 mmol) added in one portion under N₂. The mixture was stirred at -35 °C for 30 min. The mixture was combined with another batch at 0.5 g scale. Water (50 mL) was then added and the combined mixture extracted with ethyl acetate (2 x 25 mL). The combined organic phases were washed with saturated aqueous brine solution (2 x 25 mL), dried with anhydrous sodium sulfate, filtered and concentrated. The residue was purified by normal phase SiO₂ chromatography (0-30% ethyl acetate/petroleum ether) to afford (S)-N-((S)-1-(4-bromo-2-methoxyphenyl)ethyl)-2-methylpropane-2-sulfinamide (0.4 g, 40% yield for 2 steps) as a white solid. ¹H NMR (400 MHz, CDCl₃): δ 7.09 (d, 1H), 7.01 (dd, 1H), 6.93 (d, 1H), 4.72-4.65 (m, 1H), 3.77 (s, 3H), 3.62 (d, 1H), 1.38 (d,, 3H), 1.14 (s, 9H).

### (b) (S)-N-((S)-1-(2-Methoxy-4-(4,4,5,5-tetramethyl-1,3,2-dioxaborolan-2-yl)phenyl)ethyl)-2-methylpropane-2-sulfinamide

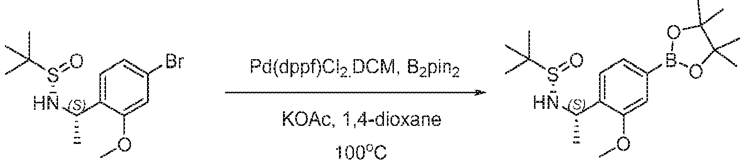

To a mixture of (S)-N-((S)-1-(4-bromo-2-methoxyphenyl)ethyl)-2-methylpropane-2-sulfinamide (0.3 g, 0.9 mmol) and bis(pinacolato)diborane (0.46 g, 1.79 mmol) in 1,4-dioxane (3 mL) was added [1,1'- bis(diphenylphosphino)ferrocene]dichloropalladium(II) complex with dichloromethane (0.07 g, 0.09 mmol) and potassium acetate (0.26 g, 2.69 mmol) in one portion under N₂ and the mixture was stirred at 100 °C for 12 hr. The mixture was concentrated and the residue purified by normal phase SiO₂ chromatography (0-25% ethyl acetate/petroleum ether) to afford (S)-N-((S)-1-(2-methoxy-4-(4,4,5,5-tetramethyl-1,3,2-dioxaborolan-2-yl)phenyl)ethyl)-2-methylpropane-2-sulfinamide (0.3 g, 66% yield) as a yellow oil. ¹H NMR (400 MHz, CDCl₃): δ 7.34 (d, 1H), 7.25-7.19 (m, 2H), 4.82 (d, 1H), 3.82 (s, 3H), 3.72 (d, 1H), 1.38 (d, 3H), 1.26 (s, 12H). 1.14 (s, 9H).

### (c) tert-Butyl ((6-(3-(2-(4-((S)-1-(((S)-tert-butylsulfinyl)amino)ethyl)-3-methoxyphenyl)-3-chloropyridin-4-yl)-2-chlorophenyl)-2-methoxypyridin-3-yl)methyl)(((S)-5-oxopyrrolidin-2-yl)methyl)carbamate

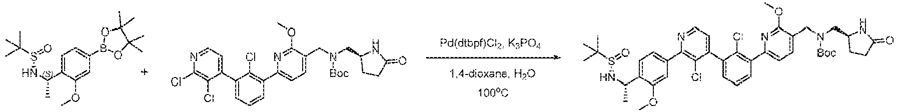

To a mixture of (S)-N-((S)-1-(2-methoxy-4-(4,4,5,5-tetramethyl-1,3,2-dioxaborolan-2-yl)phenyl)ethyl)-2-methylpropane-2-sulfinamide (0.3 g, 0.79 mmol) and tert-butyl (S)-((6-(2-chloro-3-(2,3-dichloropyridin-4-yl)phenyl)-2-methoxypyridin-3-yl)methyl)((5-oxopyrrolidin-2-yl)methyl)carbamate (0.3 g, 0.51 mmol) in 1,4-dioxane / water mixture (6:1, 3.5 mL) was added potassium phosphate (0.32 g, 1.52 mmol) and [1,1'-bis(di-tert-butylphosphino)ferrocene]-dichloropalladium(II) (0.03 g, 0.05 mmol) in one portion under N₂ and the mixture was stirred at 100 °C for 12 hr. To the mixture was added water (50 mL) and the mixture was extracted with ethyl acetate (2 x 25 mL). The combined organic phases were washed with saturated aqueous brine solution (2 x 15 mL), dried with anhydrous sodium sulfate, filtered and concentrated. The residue was purified by prep-TLC (silica gel, petroleum ether : ethyl acetate = 0:1) to afford tert-butyl ((6-(3-(2-(4-((S)-1-(((S)-tert-butylsulfinyl)amino)ethyl)-3-methoxyphenyl)-3-chloropyridin-4-yl)-2-chlorophenyl)-2-methoxypyridin-3-yl)methyl)(((S)-5-oxopyrrolidin-2-yl)methyl)carbamate (0.18 g, 27% yield) as a white solid. MS: m/z found 810 [M+H]⁺.

### (d) (S)-5-((((6-(3-(2-(4-((S)-1-Aminoethyl)-3-methoxyphenyl)-3-chloropyridin-4-yl)-2-chlorophenyl)-2-methoxypyridin-3-yl)methyl)amino)methyl)pyrrolidin-2-one

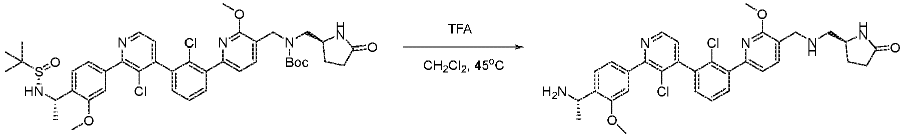

To a mixture of tert-butyl ((6-(3-(2-(4-((S)-1-(((S)-tert-butylsulfinyl)amino)ethyl)-3-methoxyphenyl)-3-chloropyridin-4-yl)-2-chlorophenyl)-2-methoxypyridin-3-yl)methyl)(((S)-5-oxopyrrolidin-2-yl)methyl)carbamate (0.18 g, 0.22 mmol) in dichloromethane (0.3 mL) was added trifluoroacetic acid (3 mL, 0.03 mmol) in one portion under N₂ and the mixture was stirred at 45 °C for 3 hr. The mixture was concentrated and the residue purified by reverse phase HPLC to afford (S)-5-((((6-(3-(2-(4-((S)-1-aminoethyl)-3-methoxyphenyl)-3-chloropyridin-4-yl)-2-chlorophenyl)-2-methoxypyridin-3-yl)methyl)amino)methyl)pyrrolidin-2-one (7.1 mg, 5% yield) as a white solid (formic acid salt). MS: m/z found 606 and 608 [M+H]⁺, retention time = 2.69 min (Method A). ¹H NMR (400 MHz, Methanol-*d*₄): δ 8.67 (d, 1H), 8.44 (br s, 2H), 7.81 (d, 1H), 7.73 (dd, 1H), 7.58 (t, 1H), 7.52-7.48 (m, 2H), 7.45-7.38 (m, 3H), 7.30 (d, 1H), 4.75 (q, 1H), 4.06 (s, 3H), 4.01 (s, 3H), 3.98 (d, 2H), 3.94-3.87 (m, 1H), 2.90-2.80 (m, 2H), 2.40-2.28 (m, 3H), 1.91-1.82 (m, 1H), 1.70 (d, 3H).

### Example 397: (S)-1-(6-(3-(2-(4-((S)-1-Aminoethyl)-3-methoxyphenyl)-3-chloropyridin-4-yl)-2-chlorophenyl)-2-methoxypyridin-3-yl)ethan-1-amine (393)

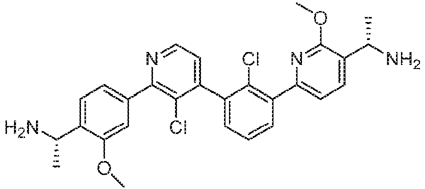

### (a) (S)-N-((E)-(6-(3-(2-(4-((E)-(((S)-tert-butylsulfinyl)imino)methyl)-3-methoxyphenyl)-3-chloropyridin-4-yl)-2-chlorophenyl)-2-methoxypyridin-3-yl)methylene)-2-methylpropane-2-sulfinamide

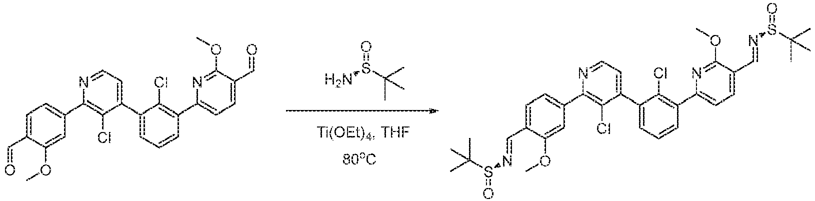

To a mixture of 6-(2-chloro-3-(3-chloro-2-(4-formyl-3-methoxyphenyl)pyridin-4-yl)phenyl)-2-methoxynicotinaldehyde (0.3 g, 0.61 mmol) and (S)-2-methylpropane-2-sulfinamide (0.16 g, 1.34 mmol) in THF (6 mL) was added titanium(IV) ethoxide (0.6 mL, 2.89 mmol) in one portion under N₂ atmosphere and the mixture was stirred at 80 °C for 12 h. To the mixture was added water (5 mL), the mixture filtered and the filtrate concentrated. To the residue was added water (5 mL) and saturated aqueous brine solution (5 mL) and the mixture was extracted with ethyl acetate/THF mixture (1:1, 10 mL). The organic phase was dried with anhydrous sodium sulfate, filtered and concentrated. The residue was purified by normal phase SiO₂ chromatography (20-50% ethyl acetate/petroleum ether) to afford (S)-N-((E)-(6-(3-(2-(4-((E)-(((S)-tert-butylsulfinyl)imino)methyl)-3-methoxyphenyl)-3-chloropyridin-4-yl)-2-chlorophenyl)-2-methoxypyridin-3-yl)methylene)-2-methylpropane-2-sulfinamide (0.32 g, 75% yield) as a yellow solid. MS: m/z found 699 [M+H]⁺.

### (b) (S)-N-((S)-1-(6-(3-(2-(4-((S)-1-(((S)-tert-Butylsulfinyl)amino)ethyl)-3-methoxyphenyl)-3-chloropyridin-4-yl)-2-chlorophenyl)-2-methoxypyridin-3-yl)ethyl)-2-methylpropane-2-sulfinamide

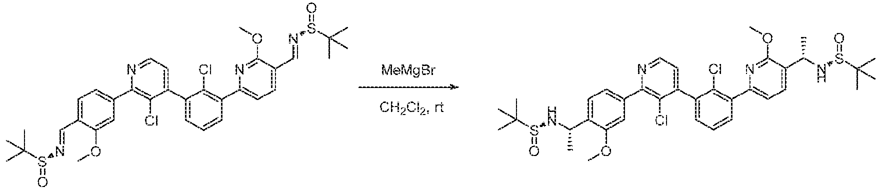

To a mixture of (S)-N-((E)-(6-(3-(2-(4-((E)-(((S)-tert-butylsulfinyl)imino)methyl)-3-methoxyphenyl)-3-chloropyridin-4-yl)-2-chlorophenyl)-2-methoxypyridin-3-yl)methylene)-2-methylpropane-2-sulfinamide (0.29 g, 0.41 mmol) in dichloromethane (10 mL) was dropwise added methylmagnesium bromide (3 M, 0.55 mL) at 0 °C under N₂ atmosphere and the mixture was stirred at room temperature for 2 h. To the mixture was dropwise added methylmagnesium bromide (3 M, 0.28 mL) and the mixture was stirred at room temperature for 14 h. To the mixture was added water (5 mL) to quench methylmagnesium bromide at 0 °C. The mixture was filtered and the filtrate was concentrated. To the residue was added water (10 mL) and saturated aqueous brine solution (50 mL) and the mixture was extracted with ethyl acetate/THF mixture (1:1, 20 mL). The organic phase was dried with anhydrous sodium sulfate, filtered and concentrated. The residue was purified by prep-TLC (silica gel, 100% ethyl acetate/petroleum ether) to afford (S)-N-((S)-1-(6-(3-(2-(4-((S)-1-(((S)-tert-butylsulfinyl)amino)ethyl)-3-methoxyphenyl)-3-chloropyridin-4-yl)-2-chlorophenyl)-2-methoxypyridin-3-yl)ethyl)-2-methylpropane-2-sulfinamide (125 mg, 32% yield) as a yellow solid. MS: m/z found 731 and 733 [M+H]⁺.

### (c) (S)-1-(6-(3-(2-(4-((S)-1-Aminoethyl)-3-methoxyphenyl)-3-chloropyridin-4-yl)-2-chlorophenyl)-2-methoxypyridin-3-yl)ethan-1-amine

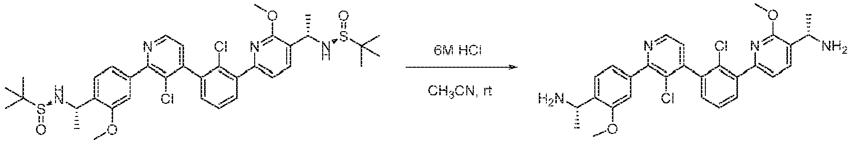

To a mixture of (S)-N-((S)-1-(6-(3-(2-(4-((S)-1-(((S)-tert-butylsulfinyl)amino)ethyl)-3-methoxyphenyl)-3-chloropyridin-4-yl)-2-chlorophenyl)-2-methoxypyridin-3-yl)ethyl)-2-methylpropane-2-sulfinamide (122 mg, 0.17 mmol) in acetonitrile (1 mL) was added HCl solution (6 M, 0.5 mL) in one portion. The mixture was stirred at room temperature for 1 h. The mixture was purified directly by reverse phase HPLC to afford (S)-1-(6-(3-(2-(4-((S)-1-aminoethyl)-3-methoxyphenyl)-3-chloropyridin-4-yl)-2-chlorophenyl)-2-methoxypyridin-3-yl)ethan-1-amine (54.7 mg, 56% yield) as a white solid (formic acid salt). MS: m/z found 523 [M+H]⁺, retention time = 2.57 min (Method A). ¹H NMR (400 MHz, Methanol-*d*₄): δ 8.67 (d, 1H), 8.56 (br s, 1H), 7.87 (d, 1H), 7.74 (dd, 1H), 7.59 (t, 1H), 7.52-7.46 (m, 3H), 7.40-7.36 (m, 3H), 4.75 (q, 1H), 4.65 (q, 1H), 4.10 (s, 3H), 4.01 (s, 3H), 1.69 (t, 6H).

### Example 398: 1-(4-(((S)-1-(6-(3-(2-(4-((S)-1-((1-Acetylpiperidin-4-yl)amino)ethyl)-3-methoxyphenyl)-3-chloropyridin-4-yl)-2-chlorophenyl)-2-methoxypyridin-3-yl)ethyl)amino)piperidin-1-yl)ethan-1-one (599)

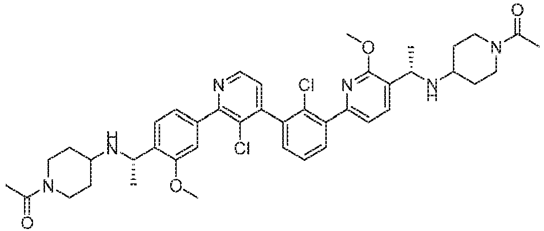

### (a) (S)-1-(6-(3-(2-(4-((S)-1-Aminoethyl)-3-methoxyphenyl)-3-chloropyridin-4-yl)-2-chlorophenyl)-2-methoxypyridin-3-yl)ethan-1-amine

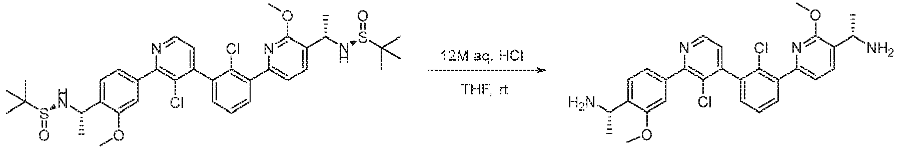

To a mixture of (S)-N-((S)-1-(6-(3-(2-(4-((S)-1-(((S)-tert-butylsulfinyl)amino)ethyl)-3-methoxyphenyl)-3-chloropyridin-4-yl)-2-chlorophenyl)-2-methoxypyridin-3-yl)ethyl)-2-methylpropane-2-sulfinamide (Example 397, step (b)) 0.09 g, 0.12 mmol) in THF (2 mL) was added aqueous HCl solution (12 M, 0.06 mL) (about 2 drops) in one portion. The mixture was stirred at r.t. for 12 h. The mixture was concentrated to afford the crude (S)-1-(6-(3-(2-(4-((S)-1-aminoethyl)-3-methoxyphenyl)-3-chloropyridin-4-yl)-2-chlorophenyl)-2-methoxypyridin-3-yl)ethan-1-amine hydrochloride salt (110 mg, crude) as a yellow solid. MS: m/z found 523 [M+H]⁺.

### (b) 1-(4-(((S)-1-(6-(3-(2-(4-((S)-1-((1-Acetylpiperidin-4-yl)amino)ethyl)-3-methoxyphenyl)-3-chloropyridin-4-yl)-2-chlorophenyl)-2-methoxypyridin-3-yl)ethyl)amino)piperidin-1-yl)ethan-1-one

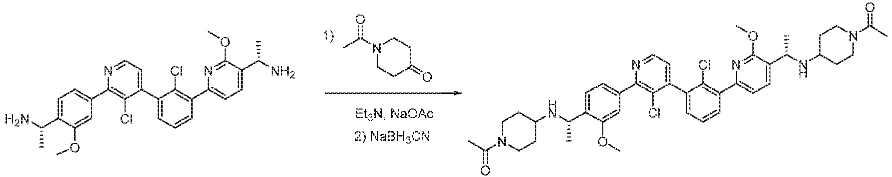

A mixture of (S)-1-(6-(3-(2-(4-((S)-1-aminoethyl)-3-methoxyphenyl)-3-chloropyridin-4-yl)-2-chlorophenyl)-2-methoxypyridin-3-yl)ethan-1-amine hydrochloride salt (0.11 g, 0.2 mmol), sodium acetate (0.08 g, 0.98 mmol), triethylamine (0.08 mL, 0.59 mmol) and 1-acetylpiperidin-4-one (0.07 g, 0.49 mmol) in methanol (3 mL) was stirred at room temperature for 12 h under N₂. Sodium cyanoborohydride (0.05 g, 0.79 mmol) was then added and the mixture was stirred at room temperature for 1 h. The mixture was combined with another batch at 40 mg scale. The mixture was purified directly by reverse phase HPLC to afford 1-(4-(((S)-1-(6-(3-(2-(4-((S)-1-((1-acetylpiperidin-4-yl)amino)ethyl)-3-methoxyphenyl)-3-chloropyridin-4-yl)-2-chlorophenyl)-2-methoxypyridin-3-yl)ethyl)amino)piperidin-1-yl)ethan-1-one (20.4 mg , 10% yield) as a white solid. MS: m/z found 773 [M+H]+, retention time = 2.94 min (Method A). ¹H NMR (400 MHz, Methanol-*d₄):* δ 8.64 (d, 1H), 7.82 (d, 1H), 7.74 (dd, 1H), 7.57 (t, 1H), 7.48-7.42 (m, 3H), 7.33-7.30 (m, 3H), 4.47-4.42 (m, 3H), 4.32 (q, 1H), 3.94 (s, 3H), 3.92-3.88 (m, 5H), 3.12-2.94 (m, 2H), 2.68-2.52 (m, 4H), 2.09-1.99 (m, 8H), 1.86-1.79 (m, 2H), 1.43-1.22 (m, 10H).

### Example 399: (S)-2-(4-(3-Chloro-4-(2-chloro-3-(6-methoxy-5-((((5-oxopyrrolidin-2-yl)methyl)amino)methyl)pyridin-2-yl)phenyl)pyridin-2-yl)-2-fluoro-6-methoxybenzyl)-2,6-diazaspiro[3.4]octan-7-one (145)

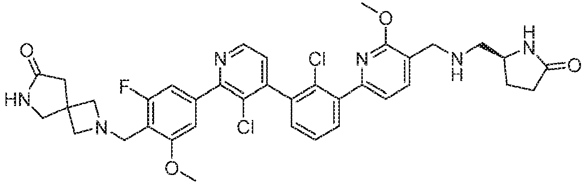

### (a) tert-Butyl (S)-((6-(2-chloro-3-(3-chloro-2-(3-fluoro-5-methoxy-4-((7-oxo-2,6-diazaspiro[3.4]octan-2-yl)methyl)phenyl)pyridin-4-yl)phenyl)-2-methoxypyridin-3-yl)methyl)((5-oxopyrrolidin-2-yl)methyl)carbamate

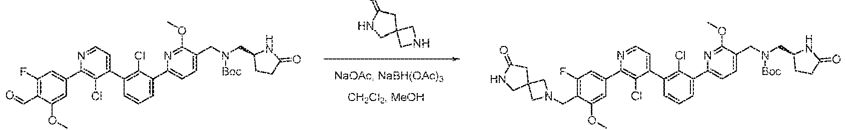

To a mixture of tert-butyl (S)-((6-(2-chloro-3-(3-chloro-2-(3-fluoro-4-formyl-5-methoxyphenyl)pyridin-4-yl)phenyl)-2-methoxypyridin-3-yl)methyl)((5-oxopyrrolidin-2-yl)methyl)carbamate (Example 95, step a) (200 mg, 282 umol) and 2,6-diazaspiro[3.4]octan-7-one (71 mg, 563 umol) in dichloromethane / methanol (2:1, 9 mL) was added sodium acetate (69 mg, 845 umol) and sodium triacetoxyborohydride (179 mg, 845 umol) and then the mixture was stirred at room temperature for 3 h under N₂. The mixture was filtered, the filtrate concentrated and the residue purified by normal phase SiO₂ chromatography (100% ethyl acetate/petroleum ether) to afford tert-butyl (S)-((6-(2-chloro-3-(3-chloro-2-(3-fluoro-5-methoxy-4-((7-oxo-2,6-diazaspiro[3.4]octan-2-yl)methyl)phenyl)pyridin-4-yl)phenyl)-2-methoxypyridin-3-yl)methyl)((5-oxopyrrolidin-2-yl)methyl)carbamate (155 mg, 32% yield) as a white solid. MS: *m*/*z* found 819 [M+H]⁺.

### (b) (S)-2-(4-(3-Chloro-4-(2-chloro-3-(6-methoxy-5-((((5-oxopyrrolidin-2-yl)methyl)amino)methyl)pyridin-2-yl)phenyl)pyridin-2-yl)-2-fluoro-6-methoxybenzyl)-2,6-diazaspiro[3.4]octan-7-one

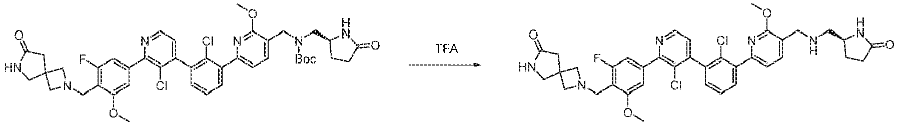

A mixture of tert-butyl (S)-((6-(2-chloro-3-(3-chloro-2-(3-fluoro-5-methoxy-4-((7-oxo-2,6-diazaspiro[3.4]octan-2-yl)methyl)phenyl)pyridin-4-yl)phenyl)-2-methoxypyridin-3-yl)methyl)((5-oxopyrrolidin-2-yl)methyl)carbamate (150 mg, 0.183 umol) in trifluoroacetic acid (50.6 mmol, 3.75 mL) was stirred at room temperature for 0.5 h under N₂ . The mixture was concentrated and the residue purified by reverse phase HPLC to afford (S)-2-(4-(3-chloro-4-(2-chloro-3-(6-methoxy-5-((((5-oxopyrrolidin-2-yl)methyl)amino)methyl)pyridin-2-yl)phenyl)pyridin-2-yl)-2-fluoro-6-methoxybenzyl)-2,6-diazaspiro[3.4]octan-7-one (101 mg, 72% yield) as a white solid (formic acid salt). MS: *m*/*z* found 719 [M+H]⁺, retention time = 2.58 min (Method A). ¹H NMR (400 MHz, Methanol-*d*₄): δ 8.67 (d, 1H), 7.89 (d, 1H), 7.73-7.68 (m, 1H), 7.57 (t, 1H), 7.50-7.45 (m, 2H), 7.36 (d, 1H), 7.24-7.21 (m, 2H), 4.58 (s, 2H), 4.34-4.33 (m, 5H), 4.09-4.04 (m, 4H), 4.01 (s, 3H), 3.68 (s, 2H), 3.25-3.22 (m, 2H), 2.73 (m, 2H), 2.43-2.33 (m, 4H), 1.92-1.89 (m, 1H).

### Example 400: (S)-5-((((6-(2-Chloro-3-(3-chloro-2-(3-fluoro-4-((6-hydroxy-2-azaspiro [3.3]heptan-2-yl)methyl)-5-methoxyphenyl)pyridin-4-yl)phenyl)-2-methoxypyridin-3-yl)methyl)amino)methyl)pyrrolidin-2-one (146)

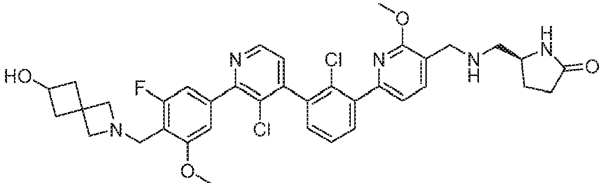

### (a) tert-Butyl (S)-((6-(2-chloro-3-(3-chloro-2-(3-fluoro-4-((6-hydroxy-2-azaspiro [3.3]heptan-2-yl)methyl)-5-methoxyphenyl)pyridin-4-yl)phenyl)-2-methoxypyridin-3-yl)methyl)((5-oxopyrrolidin-2-yl)methyl)carbamate

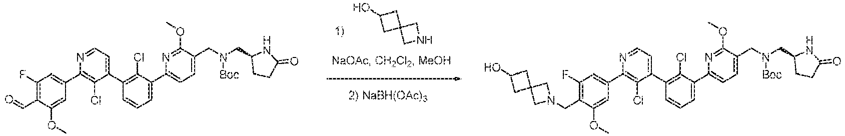

To a mixture of tert-butyl (S)-((6-(2-chloro-3-(3-chloro-2-(3-fluoro-4-formyl-5-methoxyphenyl)pyridin-4-yl)phenyl)-2-methoxypyridin-3-yl)methyl)((5-oxopyrrolidin-2-yl)methyl)carbamate (Example 95, step a) (200 mg, 0.281 mmol) and 2-azaspiro[3.3]heptan-6-ol (84.3 mg, 0.563 mmol) in dichloromethane / MeOH (1:1, 6 mL) was added sodium acetate (115 mg, 1.41 mmol) and then the mixture was stirred at room temperature for 3 h under N₂. Sodium triacetoxyborohydride (179 mg, 0.846 mmol) was then added and the mixture stirred at room temperature for 0.5 h under N₂. The mixture was filtered, the filtrate concentrated and the residue purified by normal phase SiO₂ chromatography (0 to 100% ethyl acetate/petroleum ether) to afford tert-butyl (S)-((6-(2-chloro-3-(3-chloro-2-(3-fluoro-4-((6-hydroxy-2-azaspiro[3.3]heptan-2-yl)methyl)-5-methoxyphenyl)pyridin-4-yl)phenyl)-2-methoxypyridin-3-yl)methyl)((5-oxopyrrolidin-2-yl)methyl)carbamate (155 mg, 54% yield) as a white solid. MS: *m*/*z* found 806 [M+H]⁺.

### (b) (S)-5-((((6-(2-Chloro-3-(3-chloro-2-(3-fluoro-4-((6-hydroxy-2-azaspiro[3.3]heptan-2-yl)methyl)-5-methoxyphenyl)pyridin-4-yl)phenyl)-2-methoxypyridin-3-yl)methyl)amino)methyl)pyrrolidin-2-one

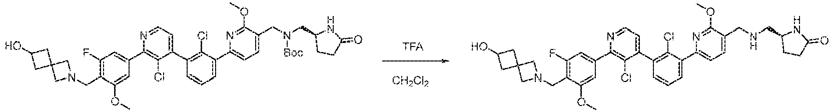

To a solution of tert-butyl (S)-((6-(2-chloro-3-(3-chloro-2-(3-fluoro-4-((6-hydroxy-2-azaspiro[3.3]heptan-2-yl)methyl)-5-methoxyphenyl)pyridin-4-yl)phenyl)-2-methoxypyridin-3-yl)methyl)((5-oxopyrrolidin-2-yl)methyl)carbamate (150 mg, 185 umol) in dichloromethane (5 mL) was added trifluoroacetic acid (67.5 mmol, 5 mL) and the mixture stirred at room temperature for 0.5 h under N₂. The mixture was then concentrated and the residue purified by reverse phase HPLC to afford (S)-5-((((6-(2-chloro-3-(3-chloro-2-(3-fluoro-4-((6-hydroxy-2-azaspiro[3.3]heptan-2-yl)methyl)-5-methoxyphenyl)pyridin-4-yl)phenyl)-2-methoxypyridin-3-yl)methyl)amino)methyl)pyrrolidin-2-one (88.5 mg, 61 % yield) as a white solid (formic acid salt). MS: *m*/*z* found 706 [M+H]⁺, retention time = 2.75 min (Method A). ¹H NMR (400 MHz, Methanol-*d*₄) : δ 8.67 (d, 1H), 7.90 (d, 1H), 7.72 (dd, 1H), 7.57 (t, 1H), 7.48 (d, 1H), 7.45 (dd, 1H), 7.37 (d, 1H), 7.27 (s, 1H), 7.21 (dd, 1H), 4.50 (s, 2H), 4.38-4.34 (m, 2H), 4.21-4.14 (m, 5H), 4.12 (s, 3H), 4.07-4.05 (m, 1H), 4.01 (s, 3H), 3.25-3.21 (m, 2H), 2.66-2.59 (m, 2H), 2.43-2.37 (m, 3H), 2.18-2.13 (m, 2H), 1.92-1.89 (m, 1H).

### Example 401: (S)-5-((((6-(2-Chloro-3-(3-chloro-2-(3-fluoro-4-(((3-fluoropropyl)amino)methyl)-5-methoxyphenyl)pyridin-4-yl)phenyl)-2-methoxypyridin-3-yl)methyl)amino)methyl)pyrrolidin-2-one (147)

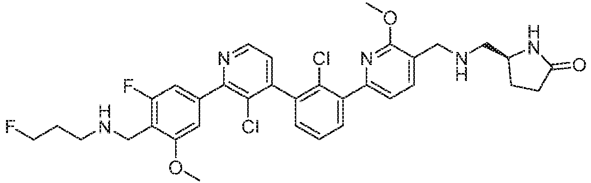

(S)-5-((((6-(2-Chloro-3-(3-chloro-2-(3-fluoro-4-(((3-fluoropropyl)amino)methyl)-5-methoxyphenyl)pyridin-4-yl)phenyl)-2-methoxypyridin-3-yl)methyl)amino)methyl)pyrrolidin-2-one (formic acid salt) was prepared in a similar fashion to Example 400, replacing 2-azaspiro[3.3]heptan-6-ol with 3-fluoropropan-1-amine hydrochloride in step (a). MS: *m*/*z* found 670 [M+H]⁺, retention time = 2.79 min (Method A). ¹H NMR (400 MHz, Methanol-*d*₄) δ 8.68 (d, 1H), 8.44 (brs, 1H), 7.81 (d, 1H), 7.74-7.70 (m, 1H), 7.57 (t, 1H), 7.50 (d, 1H), 7.44-7.40 (m, 1H), 7.31-7.26 (m, 2H), 7.21 (d, 1H), 4.65 (t, 1H), 4.53 (t, 1H), 4.35 (s, 2H), 4.05 (s, 3H), 4.02 (s, 5H), 3.97-3.89 (m, 1H), 3.27-3.22 (m, 2H), 2.96-2.82 (m, 2H), 2.40-2.29 (m, 3H), 2.22-2.07 (m, 2H), 1.91-1.79 (m, 1H).

### Example 402: (S)-5-((((6-(3-(2-(4-(((1-Acetylpiperidin-4-yl)amino)methyl)-3-fluoro-5-methoxyphenyl)-3-chloropyridin-4-yl)-2-chlorophenyl)-2-methoxypyridin-3-yl)methyl)amino)methyl)pyrrolidin-2-one (148)

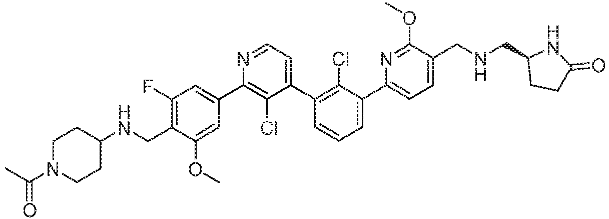

(S)-5-((((6-(3-(2-(4-(((1-Acetylpiperidin-4-yl)amino)methyl)-3-fluoro-5-methoxyphenyl)-3-chloropyridin-4-yl)-2-chlorophenyl)-2-methoxypyridin-3-yl)methyl)amino)methyl)pyrrolidin-2-one was prepared in a similar fashion to Example 400, replacing 2-azaspiro[3.3]heptan-6-ol with 1-(4-aminopiperidin-1-yl)ethan-1-one in step (a). MS: *m*/*z* found 735 [M+H]⁺, retention time = 2.72 min (Method A). ¹H NMR (400 MHz, Methanol-*d*₄) δ 8.64 (d, 1H), 7.76-7.70 (m, 2H), 7.55 (t, 1H), 7.46 (d, 1H), 7.40 (dd, 1H), 7.25 (d, 1H), 7.17 (s, 1H), 7.11 (d, 1H), 4.49 (br d, 1H), 4.02 (s, 5H), 3.96 (s, 3H), 3.94-3.89 (m, 1H), 3.89-3.78 (m, 3H), 3.19-3.10 (m, 1H), 2.85 (br t, 1H), 2.76-2.62 (m, 3H), 2.38-2.22 (m, 3H), 2.13-1.98 (m, 5H), 1.88-1.75 (m, 1H), 1.48-1.22 (m, 2H).

### Example 403: (S)-5-((((6-(2-Chloro-3-(3-chloro-2-(3-fluoro-5-methoxy-4-(((((S)-oxetan-2-yl)methyl)amino)methyl)phenyl)pyridin-4-yl)phenyl)-2-methoxypyridin-3-yl)methyl)amino)methyl)pyrrolidin-2-one (181)

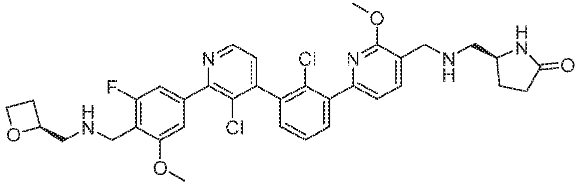

((S)-5-((((6-(2-Chloro-3-(3-chloro-2-(3-fluoro-5-methoxy-4-(((((S)-oxetan-2-yl)methyl)amino)methyl)phenyl)pyridin-4-yl)phenyl)-2-methoxypyridin-3-yl)methyl)amino)methyl)pyrrolidin-2-one (formic acid salt) was prepared in a similar fashion to Example 400, replacing 2-azaspiro[3.3]heptan-6-ol with (S)-oxetan-2-ylmethanamine in step (a). MS: *m*/*z* found 680 [M+H]⁺, retention time = 2.68 min (Method A). ¹H NMR ((400MHz, Methanol-*d*₄): δ. 8.67 (d, 1H), 8.39 (s, 2H), 7.81 (d, 1H), 7.73-7.71 (m, 1H), 7.56 (t, 1H), 7.49 (d, 1H), 7.44-7.41 (m, 1H), 7.30 (d, 1H), 7.25 (s, 1H), 7.21-7.18 (m, 1H), 5.13-5.08 (m, 1H), 4.76-4.71 (m, 1H), 4.66-4.61 (m, 1H), 4.37-4.30 (m, 2H), 4.05-4.03 (m, 5H), 4.01 (s, 3H), 3.93 (br s, 1H), 3.49-3.44 (m, 1H), 3.25-3.21 (m, 1H), 2.97-2.80 (m, 3H), 2.56-2.49 (m, 1H), 2.39-2.28 (m, 3H), 1.91-1.82 (m, 1H).

### Example 404: (S)-5-((((6-(3-(2-(4-(((2-Acetyl-2-azaspiro[3.3]heptan-6-yl)amino)methyl)-3-fluoro-5-methoxyphenyl)-3-chloropyridin-4-yl)-2-chlorophenyl)-2-methoxypyridin-3-yl)methyl)amino)methyl)pyrrolidin-2-one (210)

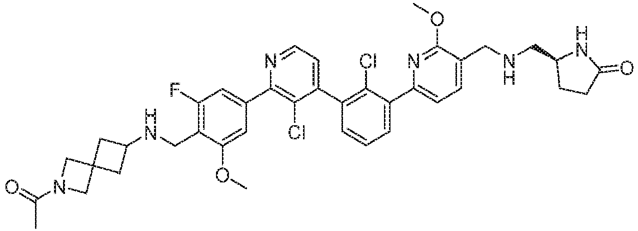

(S)-5-((((6-(3-(2-(4-(((2-Acetyl-2-azaspiro[3.3]heptan-6-yl)amino)methyl)-3-fluoro-5-methoxyphenyl)-3-chloropyridin-4-yl)-2-chlorophenyl)-2-methoxypyridin-3-yl)methyl)amino)methyl)pyrrolidin-2-one (formic acid salt) was prepared in a similar fashion to Example 400, replacing 2-azaspiro[3.3]heptan-6-ol with 1-(6-amino-2-azaspiro[3.3]heptan-2-yl)ethan-1-one hydrochloride in step (a). MS: *m*/*z* found 747 [M+H]⁺, retention time = 2.66 min (Method A). ¹H NMR (400 MHz, Methanol-*d*₄): δ 8.67 (d, 1H), 8.38 (brs, 2H), 7.81 (d, 1H), 7.71 (dd, 1H), 7.56 (t, 1H), 7.49 (d, 1H), 7.41 (dd, 1H), 7.29 (d, 1H), 7.25 (s, 1H), 7.19 (d, 1H), 4.27 (s, 1H), 4.18 (s, 3H), 4.06-3.98 (m, 9H), 3.93 (s, 2H), 3.77-3.73 (m, 1H), 2.96-2.91 (m, 2H), 2.70-2.62 (m, 2H), 2.40-2.32 (m, 5H), 1.84 (d, 1H).

### Example 405: 1-(4-(((6-(3-(2-(4-(((1-Acetylpiperidin-4-yl)amino)methyl)-3-fluoro-5-methoxyphenyl)-3-chloropyridin-4-yl)-2-chlorophenyl)-2-methoxypyridin-3-yl)methyl)amino)piperidin-1-yl)ethan-1-one (285)

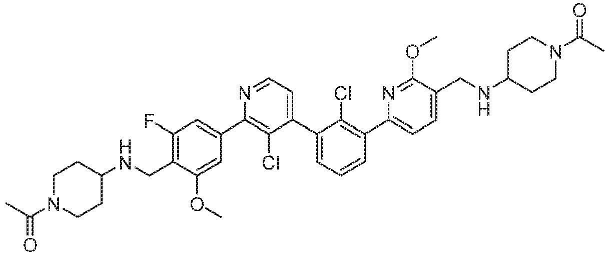

### (a) 6-(2-Chloro-3-(3-chloro-2-(3-fluoro-4-formyl-5-methoxyphenyl)pyridin-4-yl)phenyl)-2-methoxynicotinaldehyde

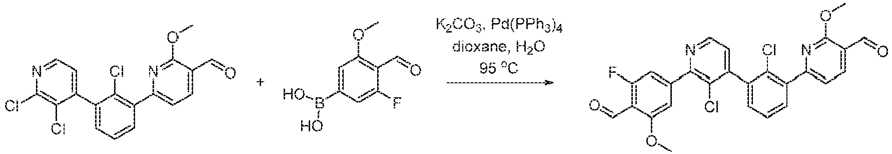

Following Suzuki Coupling Procedure A from 6-(2-chloro-3-(2,3-dichloropyridin-4-yl)phenyl)-2-methoxynicotinaldehyde (325 mg, 0.83 mmol) and (3-fluoro-4-formyl-5-methoxyphenyl)boronic acid (163 mg, 0.83 mmol) afforded 6-(2-chloro-3-(3-chloro-2-(3-fluoro-4-formyl-5-methoxyphenyl)pyridin-4-yl)phenyl)-2-methoxynicotinaldehyde in 90% yield. LCMS: m/z found 511.2 [M+H]⁺, retention time = 1.08 min (Method B).

### (b) 1-(4-(((6-(3-(2-(4-(((1-Acetylpiperidin-4-yl)amino)methyl)-3-fluoro-5-methoxyphenyl)-3-chloropyridin-4-yl)-2-chlorophenyl)-2-methoxypyridin-3-yl)methyl)amino)piperidin-1-yl)ethan-1-one

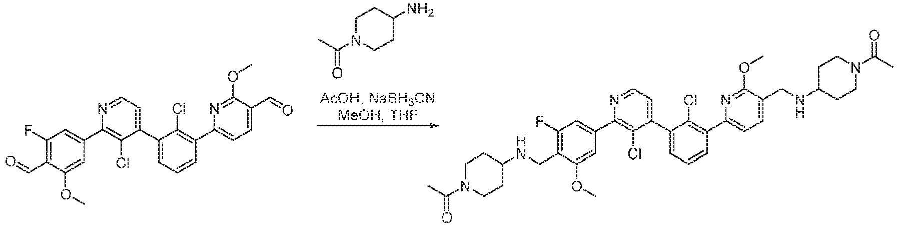

Following Reductive Amination Procedure A from 6-(2-chloro-3-(3-chloro-2-(3-fluoro-4-formyl-5-methoxyphenyl)pyridin-4-yl)phenyl)-2-methoxynicotinaldehyde (50 mg, 0.10 mmol) and 1-(4-amino-1-piperidyl)ethanone (56 mg, 0.39 mmol) gave 1-(4-(((6-(3-(2-(4-(((1-acetylpiperidin-4-yl)amino)methyl)-3-fluoro-5-methoxyphenyl)-3-chloropyridin-4-yl)-2-chlorophenyl)-2-methoxypyridin-3-yl)methyl)amino)piperidin-1-yl)ethan-1-one (formic acid salt) in 80% yield. LCMS: m/z found 763.3 [M+H]⁺, retention time = 1.82 min (Method D). ¹H NMR (400 MHz, Methanol-*d*₄): δ 8.66 (d, 1H), 8.53 (s, 2H), 7.80 (d, 1H), 7.72 (d, 1H), 7.56 (t, 1H), 7.48 (d, 1H), 7.42 (d, 1H), 7.29 (d, 1H), 7.20 (s, 1H), 7.14 (d, 1H), 4.59 - 4.47 (m, 2H), 4.12 (s, 2H), 4.04 (s, 3H), 4.02 - 3.93 (m, 7H), 3.17 (t, 2H), 3.07 - 2.96 (m, 2H), 2.70 (t, 2H), 2.19 - 2.00 (m, 10H), 1.57 - 1.29 (m, 4H).

### Example 406: 1-(6-(2-Chloro-3-(3-chloro-2-(3-fluoro-5-methoxy-4-((methylamino)methyl)phenyl)pyridin-4-yl)phenyl)-2-methoxypyridin-3-yl)-N-methylmethanamine (286)

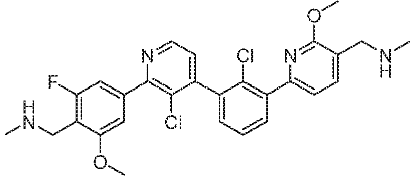

1-(6-(2-Chloro-3-(3-chloro-2-(3-fluoro-5-methoxy-4-((methylamino)methyl)phenyl)pyridin-4-yl)phenyl)-2-methoxypyridin-3-yl)-N-methylmethanamine was prepared as the formic acid salt following Reductive Amination Procedure A from 6-(2-chloro-3-(3-chloro-2-(3-fluoro-4-formyl-5-methoxyphenyl)pyridin-4-yl)phenyl)-2-methoxynicotinaldehyde (Example 405, step (a)) (50 mg, 0.10 mmol) and methanamine solution (33% wt in ethanol, 0.39 mmol). 76% yield. LCMS: m/z found 541.2 [M+H]⁺, retention time = 1.79 min (Method D). ¹H NMR (400 MHz, Methanol-*d*₄): δ 8.68 (d, 1H), 8.53 (s, 2H), 7.85 (d, 1H), 7.73 (d, 1H), 7.58 (t, 1H), 7.50 (d, 1H), 7.45 (dd, 1H), 7.34 (d, 1H), 7.26 (s, 1H), 7.20 (d, 1H), 4.27 (s, 2H), 4.17 (s, 2H), 4.08 (s, 3H), 4.01 (s, 3H), 2.71 (s, 6H).

### Example 407: 2-((6-(2-Chloro-3-(3-chloro-2-(3-fluoro-5-methoxy-4-((7-oxo-2,6-diazaspiro[3.4]octan-2-yl)methyl)phenyl)pyridin-4-yl)phenyl)-2-methoxypyridin-3-yl)methyl)-2,6-diazaspiro[3.4]octan-7-one (287)

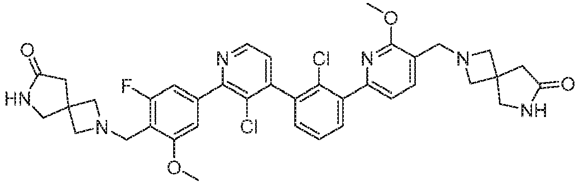

2-((6-(2-Chloro-3-(3-chloro-2-(3-fluoro-5-methoxy-4-((7-oxo-2,6-diazaspiro[3.4]octan-2-yl)methyl)phenyl)pyridin-4-yl)phenyl)-2-methoxypyridin-3-yl)methyl)-2,6-diazaspiro[3.4]octan-7-one was prepared as the formic acid salt following Reductive Amination Procedure A from 6-(2-chloro-3-(3-chloro-2-(3-fluoro-4-formyl-5-methoxyphenyl)pyridin-4-yl)phenyl)-2-methoxynicotinaldehyde (Example 405, step (a)) (50 mg, 0.10 mmol) and 2,6-diazaspiro[3.4]octan-7-one (49 mg, 0.39 mmol). 56% yield. LCMS: m/z found 731.2 [M+H]⁺, retention time = 1.67 min (Method D). ¹H NMR (400 MHz, Methanol-*d*₄): δ 8.65 (d, 1H), 8.47 (s, 2H), 7.72 (t, 2H), 7.55 (t, 1H), 7.47 (d, 1H), 7.41 (d, 1H), 7.27 (d, 1H), 7.19 (s, 1H), 7.13 (d, 1H), 4.01 (s, 5H), 3.95 (s, 3H), 3.85 (s, 2H), 3.66 (s, 4H), 3.63 - 3.54 (m, 8H), 2.63 - 2.54 (m, 4H).

### Example 408: (4-(((6-(2-Chloro-3-(3-chloro-2-(4-(((1-(cyclopropanecarbonyl)piperidin-4-yl)amino)methyl)-3-fluoro-5-methoxyphenyl)pyridin-4-yl)phenyl)-2-methoxypyridin-3-yl)methyl)amino)piperidin-1-yl)(cyclopropyl)methanone (316)

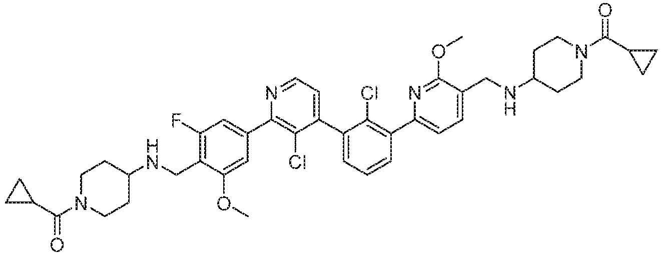

(4-(((6-(2-Chloro-3-(3-chloro-2-(4-(((1-(cyclopropanecarbonyl)piperidin-4-yl)amino)methyl)-3-fluoro-5-methoxyphenyl)pyridin-4-yl)phenyl)-2-methoxypyridin-3-yl)methyl)amino)piperidin-1-yl)(cyclopropyl)methanone was prepared as the formic acid salt following Reductive Amination Procedure A from 6-(2-chloro-3-(3-chloro-2-(3-fluoro-4-formyl-5-methoxyphenyl)pyridin-4-yl)phenyl)-2-methoxynicotinaldehyde (Example 405, step (a)) (25 mg, 0.05 mmol) and (4-aminopiperidin-1-yl)(cyclopropyl)methanone (33 mg, 0.20 mmol). 63% yield. LCMS: m/z found 815.3 [M+H]⁺, retention time = 3.25 min (Method A). ¹H NMR (400 MHz, Methanol-*d*₄): δ 8.68 - 8.63 (m, 1H), 8.55 - 8.49 (m, 2H), 7.80 (d, 1H), 7.72 (d, 1H), 7.60 - 7.52 (m, 1H), 7.50 - 7.46 (m, 1H), 7.42 (d, 1H), 7.28 (d, 1H), 7.20 (s, 1H), 7.13 (d, 1H), 4.52 (d, 2H), 4.39 (d, 2H), 4.09 (s, 2H), 4.04 (s, 3H), 4.00 - 3.91 (m, 5H), 3.26 - 3.16 (m, 2H), 3.06 - 2.91 (m, 2H), 2.82 - 2.66 (m, 2H), 2.19 - 2.10 (m, 2H), 2.09 - 2.02 (m, 2H), 1.98 (d, 2H), 1.53 - 1.41 (m, 2H), 1.40 - 1.27 (m, 2H), 0.89 - 0.76 (m, 8H).

### Example 409: 1-(4-(((6-(2-Chloro-3-(3-chloro-2-(3-fluoro-5-methoxy-4-(((1-propionylpiperidin-4-yl)amino)methyl)phenyl)pyridin-4-yl)phenyl)-2-methoxypyridin-3-yl)methyl)amino)piperidin-1-yl)propan-1-one (331)

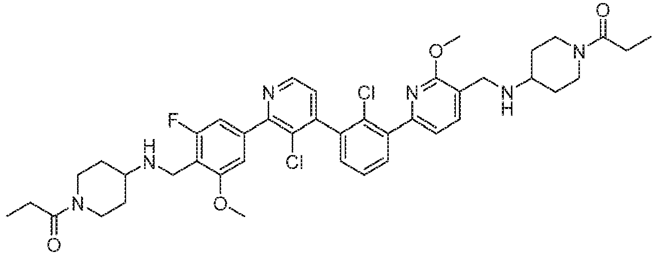

1-(4-(((6-(2-Chloro-3-(3-chloro-2-(3-fluoro-5-methoxy-4-(((1-propionylpiperidin-4-yl)amino)methyl)phenyl)pyridin-4-yl)phenyl)-2-methoxypyridin-3-yl)methyl)amino)piperidin-1-yl)propan-1-one was prepared as the formic acid salt following Reductive Amination Procedure A from 6-(2-chloro-3-(3-chloro-2-(3-fluoro-4-formyl-5-methoxyphenyl)pyridin-4-yl)phenyl)-2-methoxynicotinaldehyde (Example 405, step (a)) (25 mg, 0.05 mmol) and 1-(4-aminopiperidin-1-yl)propan-1-one (31 mg, 0.20 mmol). 83% yield. LCMS: m/z found 791.3 [M+H]⁺, retention time = 1.88 min (Method D). ¹H NMR (400 MHz, Methanol-*d*₄): δ 8.67 (d, 1H), 8.49 (s, 2H), 7.83 (d, 1H), 7.72 (d, 1H), 7.57 (t, 1H), 7.49 (d, 1H), 7.43 (d, 1H), 7.31 (d, 1H), 7.23 (s, 1H), 7.17 (d, 1H), 4.61 (d, 2H), 4.22 (s, 2H), 4.13 - 4.03 (m, 7H), 4.00 (s, 3H), 3.20 - 3.10 (m, 4H), 2.70 (t, 2H), 2.44 (q, 4H), 2.23 - 2.08 (m, 4H), 1.57 - 1.36 (m, 4H), 1.12 (t, 6H).

### Example 410: 1-(4-(((6-(2-Chloro-3-(3-chloro-2-(3-fluoro-4-(((1-isobutyrylpiperidin-4-yl)amino)methyl)-5-methoxyphenyl)pyridin-4-yl)phenyl)-2-methoxypyridin-3-yl)methyl)amino)piperidin-1-yl)-2-methylpropan-1-one (347)

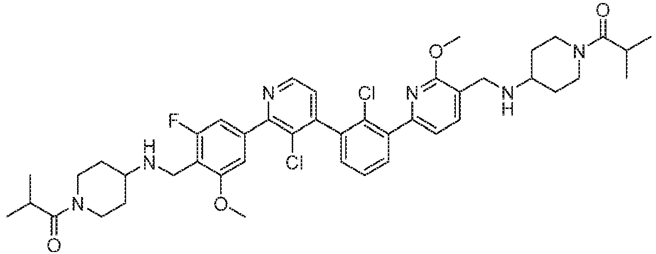

1-(4-(((6-(2-Chloro-3-(3-chloro-2-(3-fluoro-4-(((1-isobutyrylpiperidin-4-yl)amino)methyl)-5-methoxyphenyl)pyridin-4-yl)phenyl)-2-methoxypyridin-3-yl)methyl)amino)piperidin-1-yl)-2-methylpropan-1-one was prepared as the formic acid salt following Reductive Amination Procedure A from 6-(2-chloro-3-(3-chloro-2-(3-fluoro-4-formyl-5-methoxyphenyl)pyridin-4-yl)phenyl)-2-methoxynicotinaldehyde (Example 405, step (a)) (30 mg, 0.06 mmol) and 1-(4-aminopiperidin-1-yl)-2-methylpropan-1-one (40 mg, 0.23 mmol). 73% yield. LCMS: m/z found 819.4 [M+H]⁺, retention time = 2.05 min (Method D). ¹H NMR (400 MHz, Methanol-*d*₄): δ 8.69 - 8.63 (m, 1H), 8.50 (s, 2H), 7.82 (d, 1H), 7.71 (d, 1H), 7.56 (t, 1H), 7.47 (d, 1H), 7.42 (d, 1H), 7.30 (d, 1H), 7.21 (s, 1H), 7.15 (d, 1H), 4.68 - 4.53 (m, 2H), 4.21 - 4.10 (m, 4H), 4.09 - 4.02 (m, 5H), 3.98 (s, 3H), 3.21 - 3.09 (m, 4H), 3.02 - 2.92 (m, 2H), 2.76 - 2.61 (m, 2H), 2.24 - 2.05 (m, 4H), 1.54 - 1.31 (m, 4H), 1.16 - 0.98 (m, 12H).

### Example 411: N-((6-(2-Chloro-3-(3-chloro-2-(3-fluoro-5-methoxy-4-(((tetrahydro-2H-pyran-4-yl)amino)methyl)phenyl)pyridin-4-yl)phenyl)-2-methoxypyridin-3-yl)methyl)tetrahydro-2H-pyran-4-amine (348)

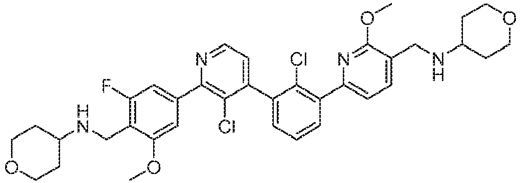

N-((6-(2-Chloro-3-(3-chloro-2-(3-fluoro-5-methoxy-4-(((tetrahydro-2H-pyran-4-yl)amino)methyl)phenyl)pyridin-4-yl)phenyl)-2-methoxypyridin-3-yl)methyl)tetrahydro-2H-pyran-4-amine was prepared as the formic acid salt following Reductive Amination Procedure A from 6-(2-chloro-3-(3-chloro-2-(3-fluoro-4-formyl-5-methoxyphenyl)pyridin-4-yl)phenyl)-2-methoxynicotinaldehyde (Example 405, step (a)) (30 mg, 0.06 mmol) and tetrahydropyran-4-amine (24 mg, 0.23 mmol). 70% yield. LCMS: m/z found 681.2 [M+H]⁺, retention time = 1.83 min (Method D). ¹H NMR (400 MHz, Methanol-*d*₄): δ 8.64 (dd, 1H), 8.55 - 8.50 (m, 2H), 7.77 (d, 1H), 7.71 (d, 1H), 7.55 (dd, 1H), 7.46 (dt, 1H), 7.41 (d, 1H), 7.26 (d, 1H), 7.18 (s, 1H), 7.11 (d, 1H), 4.07 - 4.01 (m, 5H), 4.00 - 3.97 (m, 2H), 3.97 - 3.94 (m, 5H), 3.92 (s, 2H), 3.48 - 3.36 (m, 4H), 2.93 - 2.82 (m, 2H), 2.00 - 1.88 (m, 4H), 1.57 - 1.44 (m, 4H).

### Example 412: 4-(((6-(2-Chloro-3-(3-chloro-2-(4-(((1,1-dioxidotetrahydro-2H-thiopyran-4-yl)amino)methyl)-3-fluoro-5-methoxyphenyl)pyridin-4-yl)phenyl)-2-methoxypyridin-3-yl)methyl)amino)tetrahydro-2H-thiopyran 1,1-dioxide (349)

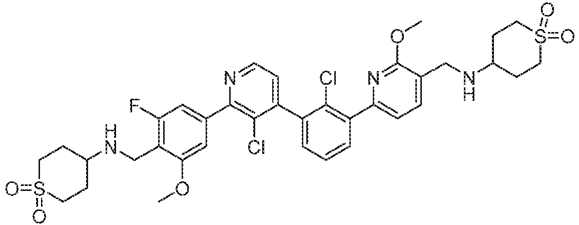

4-(((6-(2-Chloro-3-(3-chloro-2-(4-(((1,1-dioxidotetrahydro-2H-thiopyran-4-yl)amino)methyl)-3-fluoro-5-methoxyphenyl)pyridin-4-yl)phenyl)-2-methoxypyridin-3-yl)methyl)amino)tetrahydro-2H-thiopyran 1,1-dioxide was prepared as the formic acid salt following Reductive Amination Procedure A from 6-(2-chloro-3-(3-chloro-2-(3-fluoro-4-formyl-5-methoxyphenyl)pyridin-4-yl)phenyl)-2-methoxynicotinaldehyde (Example 405, step (a)) (30 mg, 0.06 mmol) and 4-aminotetrahydro-2H-thiopyran 1,1-dioxide (35 mg, 0.23 mmol). 70% yield. LCMS: m/z found 777.2 [M+H]⁺, retention time = 1.70 min (Method D). ¹H NMR (400 MHz, Methanol-*d*₄): δ 8.68 - 8.61 (m, 1H), 8.45 (s, 2H), 7.79 (d, 1H), 7.71 (d, 1H), 7.55 (td, 1H), 7.49 - 7.45 (m, 1H), 7.41 (d, 1H), 7.27 (d, 1H), 7.18 (s, 1H), 7.12 (d, 1H), 4.03 (s, 5H), 3.96 (s, 3H), 3.90 (s, 2H), 3.26 - 3.16 (m, 4H), 3.16 - 3.03 (m, 4H), 3.03 - 2.94 (m, 2H), 2.38 - 2.26 (m, 4H), 2.18 - 2.03 (m, 4H).

### Example 413: (S)-5-((((6-(3-(2-(2-((1R,3S,4S)-2-azabicyclo[2.2.1]heptan-3-yl)-1H-benzo[d]imidazol-6-yl)-3-chloropyridin-4-yl)-2-chlorophenyl)-2-methoxypyridin-3-yl)methyl)amino)methyl)pyrrolidin-2-one (152)

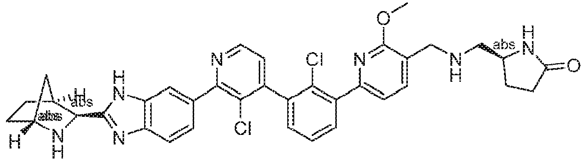

Following Suzuki Coupling Procedure A from tert-butyl (1R,3S,4S)-3-(6-(4,4,5,5-tetramethyl-1,3,2-dioxaborolan-2-yl)-1H-benzo[d]imidazol-2-yl)-2-azabicyclo[2.2.1]heptane-2-carboxylate (40 mg, 0.09 mmol) and tert-butyl (S)-((6-(2-chloro-3-(2,3-dichloropyridin-4-yl)phenyl)-2-methoxypyridin-3-yl)methyl)((5-oxopyrrolidin-2-yl)methyl)carbamate (54 mg, 0.09 mmol) afforded tert-butyl (1R,3S,4S)-3-(6-(4-(3-(5-(((tert-butoxycarbonyl)(((S)-5-oxopyrrolidin-2-yl)methyl)amino)methyl)-6-methoxypyridin-2-yl)-2-chlorophenyl)-3-chloropyridin-2-yl)-1H-benzo[d]imidazol-2-yl)-2-azabicyclo[2.2.1]heptane-2-carboxylate, LCMS: m/z found 868.3 [M+H]⁺, which was deprotected and purified by General Boc Deprotection Procedure to afford the final product as formic acid salt. 90% yield over two steps. LCMS: m/z found 668.2 [M+H]⁺, retention time = 1.69 min (Method D). ¹H NMR (400 MHz, Methanol-*d*₄): δ 8.63 (d, 1H), 8.50 (s, 2H), 7.90 (d, 1H), 7.76 (d, 1H), 7.70 (dd, 2H), 7.61 - 7.52 (m, 2H), 7.43 (dd, 2H), 7.27 (d, 1H), 4.48 (s, 1H), 4.08 - 3.95 (m, 4H), 3.91 - 3.80 (m, 3H), 2.95 (s, 1H), 2.81 - 2.68 (m, 2H), 2.40 - 2.21 (m, 3H), 1.97 - 1.71 (m, 6H), 1.61 (d, 1H).

### Example 414: (S)-5-((((6-(2-Chloro-3-(3-chloro-2-(1,1-dioxido-2,3,4,5-tetrahydrobenzo[f][1,4]thiazepin-8-yl)pyridin-4-yl)phenyl)-2-methoxypyridin-3-yl)methyl)amino)methyl)pyrrolidin-2-one (177)

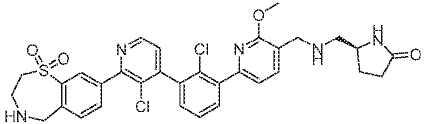

### (a) 2-((3-Bromophenyl)thio)ethan-1-amine

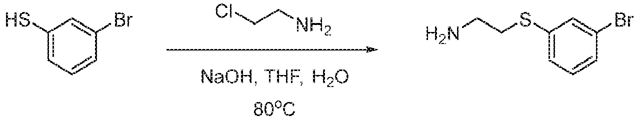

To a mixture of 3-bromobenzenethiol (2.7 mL, 26.4 mmol) and 2-chloroethan-1-amine hydrochloride salt (6.8 g, 58.2 mmol) in THF/water mixture (1:1, 50 mL) was added sodium hydroxide (2.6 g, 66.1 mmol) and the mixture was stirred at 80 °C for 12 h. The reaction was quenched with water (50 mL) and the mixture extracted with ethyl acetate (2 x 25 mL). The combined organic phases were washed with brine (2 x 25 mL), dried with anhydrous sodium sulfate, filtered and concentrated. The residue was purified by normal phase SiO₂ chromatography (0-50% MeOH/ ethyl acetate) to afford 2-((3-bromophenyl)thio)ethan-1-amine (6.1 g, 99% yield) as a white solid. ¹H NMR (400 MHz, Methanol-*d*₄): δ 7.66-7.65 (m, 1H), 7.48-7.44 (m, 2H), 7.32-7.30 (m, 1H), 3.28-3.24 (m, 2H), 3.16-3.11 (m, 2H).

### (b) N-(2-((3-Bromophenyl)thio)ethyl)acetamide

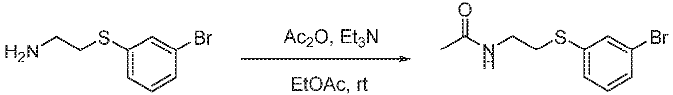

To a mixture of 2-((3-bromophenyl)thio)ethan-1-amine (3.6 g, 15.7 mmol) in ethyl acetate (30 mL) was added acetic anhydride (1.8 mL, 18.8 mmol) and triethylamine (3.3 mL, 23.5 mmol) and the mixture stirred at room temperature for 2 h. The reaction was quenched with water (50 mL) and the mixture extracted with ethyl acetate (2 x 25 mL). The combined organic phases were washed with brine (2 x 25 mL), dried with anhydrous sodium sulfate, filtered and concentrated. The residue was purified by normal phase SiO₂ chromatography (0-90% MeOH/ ethyl acetate) to afford N-(2-((3-bromophenyl)thio)ethyl)acetamide (2 g, 47% yield) as a white solid. ¹H NMR (400 MHz, Methanol-*d*₄): δ 7.56 (s, 1H), 7.38-7.34 (m, 2H), 7.24-7.22 (m, 1H), 3.41-3.32 (m, 2H), 3.10-3.07 (m, 2H) 1.92 (s, 3H).

### (c) 1-(8-Bromo-2,3-dihydrobenzo[f][1,4]thiazepin-4(5H)-yl)ethan-1-one

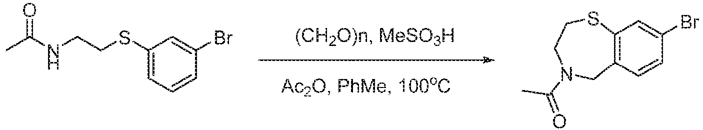

To a mixture of N-(2-((3-bromophenyl)thio)ethyl)acetamide (2 g, 7.29 mmol) in toluene (10 mL) was added paraformaldehyde (0.11 g, 3.65 mmol), methanesulfonic acid (0.62 mL, 8.75 mmol) and acetic anhydride (0.89 g, 8.75 mmol) and the mixture was stirred at 70 °C under N₂. Additional paraformaldehyde (0.55 g, 18.2 mmol) was added, and the mixture was stirred at 100 °C for 3 h under N₂. The reaction was quenched with water (10 mL) and the mixture extracted with ethyl acetate (2 x 15 mL). The combined organic phases were washed with saturated aqueous brine solution (2 x 15 mL), dried with anhydrous sodium sulfate, filtered and concentrated. The residue was purified by normal phase SiO₂ chromatography (0-90% MeOH/ethyl acetate) to afford 1-(8-bromo-2,3-dihydrobenzo[f][1,4]thiazepin-4(5H)-yl)ethan-1-one (0.85 g, 41% yield) as a white solid. ¹H NMR (400 MHz, Chloroform-*d*): δ 7.67 (d, 1H), 7.42-7.40 (d, 1H), 7.13 (d, 1H), 4.62-4.59 (m, 2H), 4.04-3.92 (m, 2H), 2.83-2.81 (m, 2H), 1.84-1.81 (m, 3H).

### (d) 1-(8-Bromo-1,1-dioxido-2,3-dihydrobenzo[f][1,4]thiazepin-4(5H)-yl)ethan-1-one

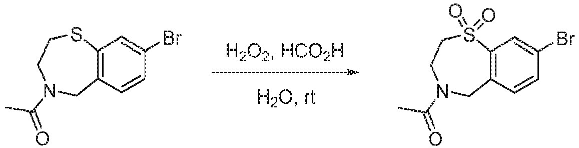

To a mixture of 1-(8-bromo-2,3-dihydrobenzo[f][1,4]thiazepin-4(5H)-yl)ethan-1-one (0.8 g, 2.8 mmol) in formic acid (5 mL) and water (5 mL) was added hydrogen peroxide (2.3 mL, 28 mmol) and the mixture was stirred at room temperature for 12 h. To the mixture was added saturated aqueous sodium sulfite solution (10 mL) and the mixture extracted with ethyl acetate (2 x 10 mL). The combined organic phases were washed with brine (2 x 5 mL), dried with anhydrous sodium sulfate, filtered and concentrated. The residue was purified by normal phase SiO₂ chromatography (0-100% ethyl acetate/petroleum ether) to afford 1-(8-bromo-1,1-dioxido-2,3-dihydrobenzo[f][1,4]thiazepin-4(5H)-yl)ethan-1-one (0.35 g, 39%) as a white solid. MS: m/z found 318 and 320 [M+H]⁺.

### (e) 8-Bromo-2,3,4,5-tetrahydrobenzo[f][1,4]thiazepine 1,1-dioxide

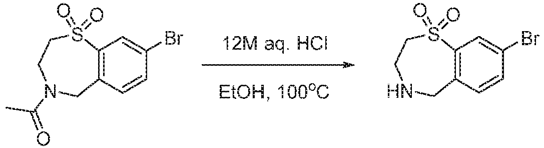

To a mixture of 1-(8-bromo-1,1-dioxido-2,3-dihydrobenzo[f][1,4]thiazepin-4(5H)-yl)ethan-1-one (0.35 g, 1.1 mmol) in ethanol (3 mL) was added aqueous HCl solution (12 M, 8 mL) and the mixture was stirred at 100°C for 12 h. To the mixture was added saturated aqueous sodium bicarbonate solution (50 mL) and the mixture extracted with ethyl acetate (2 x 25 mL). The combined organic phases were washed with brine (2 x 10 mL), dried with anhydrous sodium sulfate, filtered and concentrated. The residue was purified by normal phase SiO₂ chromatography (0-100% ethyl acetate/petroleum ether) to afford 8-bromo-2,3,4,5-tetrahydrobenzo[f][1,4]thiazepine 1,1-dioxide (0.17 g, 56%) as a white solid. ¹H NMR (400 MHz, Chloroform-d): δ 8.13 (d, 1H), 7.58 (dd, 1H), 7.11 (d, 1H), 4.17 (s, 2H), 3.50-3.42 (m, 2H), 3.22-3.20 (m, 2H).

### (f) tert-Butyl 8-bromo-2,3-dihydrobenzo[f] [1,4]thiazepine-4(5H)-carboxylate 1,1-dioxide

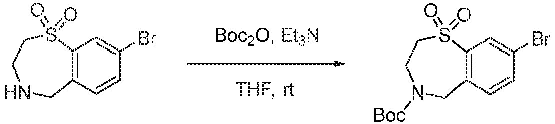

To a mixture of 8-bromo-2,3,4,5-tetrahydrobenzo[f] [1,4]thiazepine 1,1-dioxide (0.4 g, 1.45 mmol) in THF (5 mL) was added di-tert-butyl dicarbonate (0.41 g, 1.88 mmol) and triethylamine (0.2 mL, 1.45 mmol) and the mixture was stirred at room temperature for 0.5 h. The mixture was concentrated followed by addition of water (5 mL) and brine (5 mL) and the mixture extracted with ethyl acetate (10 mL). The organic phase was dried with anhydrous sodium sulfate, filtered and concentrated. The residue was purified by normal phase SiO₂ chromatography (0-15% ethyl acetate/petroleum ether) to afford tert-butyl 8-bromo-2,3-dihydrobenzo[f][1,4]thiazepine-4(5H)-carboxylate 1,1-dioxide (0.45 g, 82%) as a yellow solid. ¹H NMR (400 MHz, Chloroform-*d*): δ 8.16-8.13 (m, 1H), 7.63-7.61 (m, 1H), 7.36-7.18 (m, 1H), 4.61 (s, 2H), 4.03 (br s, 2H), 3.28-3.20 (m, 2H), 1.32 (s, 9H).

### (g) tert-Butyl 8-(4,4,5,5-tetramethyl-1,3,2-dioxaborolan-2-yl)-2,3-dihydrobenzo[f] [1,4]thiazepine-4(5H)-carboxylate 1,1-dioxide

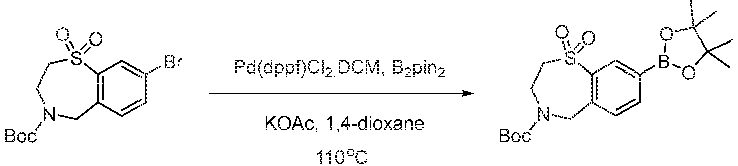

To a mixture of tert-butyl 8-bromo-2,3-dihydrobenzo[f][1,4]thiazepine-4(5H)-carboxylate 1,1-dioxide (0.42 g, 1.12 mmol) and bis(pinacolato)diborane (0.43 g, 1.67 mmol) in 1,4-dioxane (8 mL) was added [1,1'-bis(diphenylphosphino)ferrocene]dichloropalladium(II) complex with dichloromethane (0.04 g, 0.04 mmol) and potassium acetate (0.33 g, 3.35 mmol) and the mixture was stirred at 110 °C for 6 h. The mixture was concentrated, and the residue was purified by normal phase SiO₂ chromatography (0-20% ethyl acetate/petroleum ether) to afford tert-butyl 8-(4,4,5,5-tetramethyl-1,3,2-dioxaborolan-2-yl)-2,3-dihydrobenzo[f][1,4]thiazepine-4(5H)-carboxylate 1,1-dioxide (0.45 g, 95%) as a white solid. ¹H NMR (400 MHz, Chloroform-d): δ 8.44-8.43 (m, 1H), 7.90 (d, 1H), 7.48-7.19 (m, 1H), 4.66 (s, 2H), 4.04 (br s, 2H), 3.25-3.18 (m, 2H), 1.30-1.27 (m, 21H).

### (h) tert-Butyl (S)-8-(4-(3-(5-(((tert-butoxycarbonyl)((5-oxopyrrolidin-2-yl)methyl)amino)methyl)-6-methoxypyridin-2-yl)-2-chlorophenyl)-3-chloropyridin-2-yl)-2,3-dihydrobenzo[f][1,4]thiazepine-4(5H)-carboxylate 1,1-dioxide

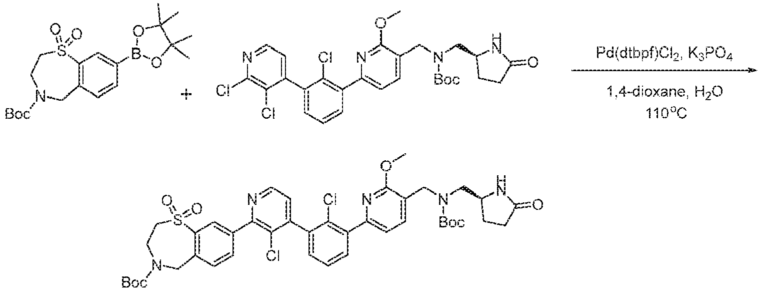

To a mixture of tert-butyl 8-(4,4,5,5-tetramethyl-1,3,2-dioxaborolan-2-yl)-2,3-dihydrobenzo[f][1,4]thiazepine-4(5H)-carboxylate 1,1-dioxide (0.2 g, 0.47 mmol) and tert-butyl (S)-((6-(2-chloro-3-(2,3-dichloropyridin-4-yl)phenyl)-2-methoxypyridin-3-yl)methyl)((5-oxopyrrolidin-2-yl)methyl)carbamate (0.2 g, 0.34 mmol) in 1,4-dioxane/water (10:1, 5.5 mL) was added potassium phosphate (0.22 g, 1.01 mmol) and [1,1'-bis(di-tert-butylphosphino)ferrocene]dichloropalladium(II) (0.02 g, 0.03 mmol) and the mixture was stirred at 110 °C for 16 h. The mixture was concentrated, water (5 mL) and brine (5 mL) were added and the mixture was extracted with ethyl acetate/THF mixture (1:1, 10 mL). The organic phase was dried with anhydrous sodium sulfate, filtered and concentrated. The residue was purified by prep-TLC (silica gel, ethyl acetate: MeOH =10:1) to afford tert-butyl (S)-8-(4-(3-(5-(((tert-butoxycarbonyl)((5-oxopyrrolidin-2-yl)methyl)amino)methyl)-6-methoxypyridin-2-yl)-2-chlorophenyl)-3-chloropyridin-2-yl)-2,3-dihydrobenzo[f][1,4]thiazepine-4(5H)-carboxylate 1,1-dioxide (0.2 g, 54%) as a light yellow solid. MS: m/z found 852 [M+H]⁺.

### (i) (S)-5-((((6-(2-Chloro-3-(3-chloro-2-(1,1-dioxido-2,3,4,5-tetrahydrobenzo[f][1,4]thiazepin-8-yl)pyridin-4-yl)phenyl)-2-methoxypyridin-3-yl)methyl)amino)methyl)pyrrolidin-2-one

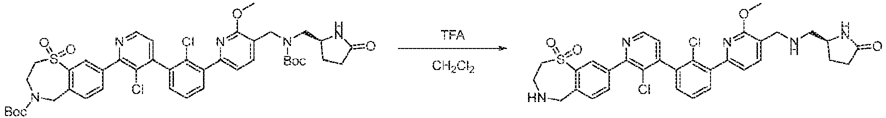

To a mixture of tert-butyl (S)-8-(4-(3-(5-(((tert-butoxycarbonyl)((5-oxopyrrolidin-2-yl)methyl)amino)methyl)-6-methoxypyridin-2-yl)-2-chlorophenyl)-3-chloropyridin-2-yl)-2,3-dihydrobenzo[f][1,4]thiazepine-4(5H)-carboxylate 1,1-dioxide (0.2 g, 0.23 mmol) in dichloromethane (0.5 mL) was added trifluoroacetic acid (2 mL, 27 mmol) and the mixture was stirred at room temperature for 15 min. The mixture was concentrated, and the residue was purified by reverse phase HPLC to afford (S)-5-((((6-(2-chloro-3-(3-chloro-2-(1,1-dioxido-2,3,4,5-tetrahydrobenzo[f][1,4]thiazepin-8-yl)pyridin-4-yl)phenyl)-2-methoxypyridin-3-yl)methyl)amino)methyl)pyrrolidin-2-one (73.2 mg, 44%) as a white solid (formic acid salt). MS: m/z found 652 [M+H]⁺, retention time = 2.53 min (Method A).¹H NMR (400 MHz, MeOD): δ 8.72 (d, 1H), 8.42 (d, 1H), 8.08 (dd, 1H), 7.93 (d, 1H), 7.77-7.72 (m, 2H), 7.61 (t, 1H), 7.52-7.48 (m, 2H), 7.40 (d, 1H), 4.57 (s, 2H), 4.39-4.30 (m, 2H), 4.12-4.05 (m, 4H), 3.71-3.70 (m, 2H), 3.61-3.60 (m, 2H), 3.31-3.22 (m, 2H), 2.48-2.34 (m, 3H), 1.99-1.88 (m, 1H).

### Example 415: (S)-5-((((6-(2-Chloro-3-(3-chloro-2-(4-methyl-1,1-dioxido-2,3,4,5-tetrahydrobenzo[f][1,4]thiazepin-8-yl)pyridin-4-yl)phenyl)-2-methoxypyridin-3-yl)methyl)amino)methyl)pyrrolidin-2-one (561)

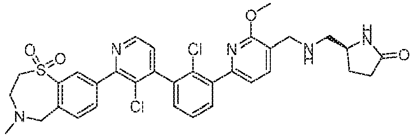

### (a) tert-Butyl 8-(3-chloro-4-(2-chloro-3-(5-formyl-6-methoxypyridin-2-yl)phenyl)pyridin-2-yl)-2,3-dihydrobenzo[f][1,4]thiazepine-4(5H)-carboxylate 1,1-dioxide

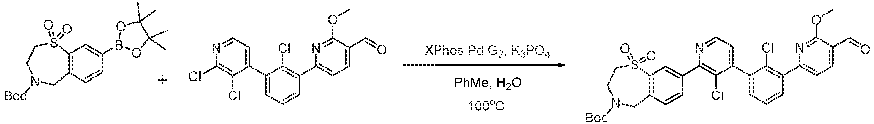

To a mixture of tert-butyl 8-(4,4,5,5-tetramethyl-1,3,2-dioxaborolan-2-yl)-2,3-dihydrobenzo[f][1,4]thiazepine-4(5H)-carboxylate 1,1-dioxide (Example 414, step (g) (2.9 g, 6.86 mmol) and 6-(2-chloro-3-(2,3-dichloropyridin-4-yl)phenyl)-2-methoxynicotinaldehyde (1.5 g, 3.81 mmol) in water (6 mL) and toluene (36 mL) was added potassium phosphate (2.43 g, 11.4 mmol) and chloro(2-dicyclohexylphosphino-2',4',6'-triisopropyl-1,1'-biphenyl)[2-(2'-amino-1,1'-biphenyl)]palladium(II) (0.24 g, 0.31 mmol) and the mixture was stirred at 100 °C for 1 h. To the mixture was added water (200 mL) and the mixture was extracted with ethyl acetate (2 x 100 mL). The combined organic phase was washed with brine (2 x 50 mL), dried with anhydrous sodium sulfate, filtered and concentrated. The residue was purified by normal phase SiO₂ chromatography (0-30% ethyl acetate/petroleum ether) to afford tert-butyl 8-(3-chloro-4-(2-chloro-3-(5-formyl-6-methoxypyridin-2-yl)phenyl)pyridin-2-yl)-2,3-dihydrobenzo[f][1,4]thiazepine-4(5H)-carboxylate 1,1-dioxide (1.6 g, 56%). ¹H NMR (400 MHz, Chloroform-d): δ 10.36 (s, 1H), 8.63 (m, 1H), 8.45-8.42 (m, 1H), 8.14 (d, 1H), 7.96-7.93 (m, 1H), 7.67 (d, 1H), 7.45 (m, 2H), 7.35-7.34 (m, 1H), 7.31-7.29 (m, 2H), 4.74 (s, 2H), 4.07 (s, 3H), 3.31-3.30 (m, 2H), 1.72-1.70 (m, 2H), 1.17 (s, 9H).

### (b) 6-(2-Chloro-3-(3-chloro-2-(1,1-dioxido-2,3,4,5-tetrahydrobenzo[f][1,4]thiazepin-8-yl)pyridin-4-yl)phenyl)-2-methoxynicotinaldehyde

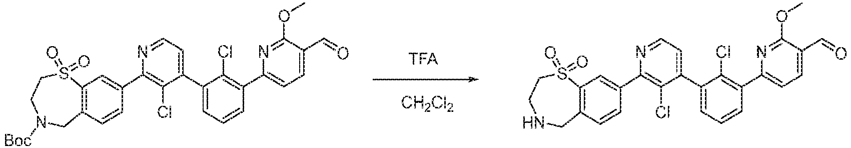

To a mixture of tert-butyl 8-(3-chloro-4-(2-chloro-3-(5-formyl-6-methoxypyridin-2-yl)phenyl)pyridin-2-yl)-2,3-dihydrobenzo[f][1,4]thiazepine-4(5H)-carboxylate 1,1-dioxide (1.6 g, 2.44 mmol) in dicloromethane (10 mL) was added trifluoroacetic acid (10 mL, 135 mmol) and the mixture was stirred at room temperature for 12 h. Aqueous HCl solution (12 M, 3 mL) was added to the mixture and the mixture was stirred at room temperature for 1 h. The mixture was concentrated and to the residue was added saturated aqueous sodium carbonate (50 mL). The mixture was extracted with ethyl acetate (2 x 25 mL). The combined organic phases were washed with brine (2 x 5 mL), dried with anhydrous sodium sulfate, filtered and concentrated to afford 6-(2-chloro-3-(3-chloro-2-(1,1-dioxido-2,3,4,5-tetrahydrobenzo[f][1,4]thiazepin-8-yl)pyridin-4-yl)phenyl)-2-methoxynicotinaldehyde (1 g, 56% yield). MS: m/z found 554 and 556 [M+H]⁺.

### (c) 8-(3-Chloro-4-(2-chloro-3-(5-(hydroxymethyl)-6-methoxypyridin-2-yl)phenyl)pyridin-2-yl)-4-methyl-2,3,4,5-tetrahydrobenzo[f][1,4]thiazepine 1,1-dioxide

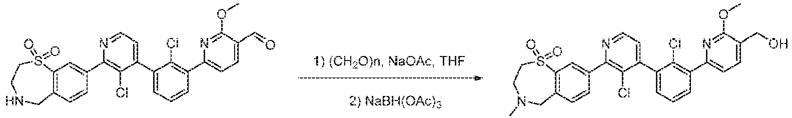

To a mixture of 6-(2-chloro-3-(3-chloro-2-(1,1-dioxido-2,3,4,5-tetrahydrobenzo[f][1,4]thiazepin-8-yl)pyridin-4-yl)phenyl)-2-methoxynicotinaldehyde (0.25 g, 0.45 mmol) in THF (3 mL) was added paraformaldehyde (0.03 g, 0.9 mmol) and sodium acetate (0.11 g, 1.35 mmol) and the mixture was stirred at room temperature for 12 h. Sodium triacetoxyborohydride (0.38 g, 1.8 mmol) was added and the mixture was stirred at room temperature for 1 h. To the mixture was added water (5 mL) and the mixture was extracted with ethyl acetate (2 x 5 mL). The combined organic phases were washed with brine (2 x 5 mL), dried with sodium sulfate, filtered and concentrated. The residue was purified by prep-TLC (silica gel, ethyl acetate : MeOH = 5:1) to afford 8-(3-chloro-4-(2-chloro-3-(5-(hydroxymethyl)-6-methoxypyridin-2-yl)phenyl)pyridin-2-yl)-4-methyl-2,3,4,5-tetrahydrobenzo[f][1,4]thiazepine 1,1-dioxide (0.18 g, 55%) as a white solid. MS: m/z found 570 [M+H]⁺.

### (d) 6-(2-Chloro-3-(3-chloro-2-(4-methyl-1,1-dioxido-2,3,4,5-tetrahydrobenzo[f][1,4]thiazepin-8-yl)pyridin-4-yl)phenyl)-2-methoxynicotinaldehyde

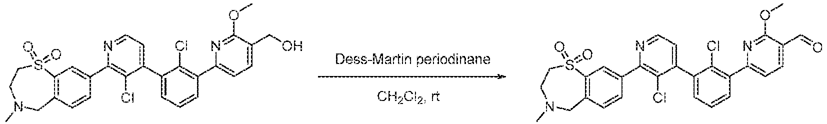

To a mixture of 8-(3-chloro-4-(2-chloro-3-(5-(hydroxymethyl)-6-methoxypyridin-2-yl)phenyl)pyridin-2-yl)-4-methyl-2,3,4,5-tetrahydrobenzo[f] [1,4]thiazepine 1,1-dioxide (0.18 g, 0.32 mmol) in dichloromethane (2 mL) was added Dess-Martin periodinane (0.27 g, 0.63 mmol) and the mixture was stirred at room temperature for 12 h. The mixture was filtered, the filtrate was concentrated, and the residue purified by prep-TLC (silica gel, ethyl acetate : MeOH = 10:1) to afford 6-(2-chloro-3-(3-chloro-2-(4-methyl-1,1-dioxido-2,3,4,5-tetrahydrobenzo[f][1,4]thiazepin-8-yl)pyridin-4-yl)phenyl)-2-methoxynicotinaldehyde (0.11 g, 46%) as a white solid. ¹H NMR (400 MHz, DMSO-d₆): δ 10.32 (s, 1H), 8.78 (d, 1H), 8.24 (d, 2H), 8.06 (dd, 1H), 7.80 (dd, 1H), 7.68-7.61 (m, 4H), 7.51 (d, 1H), 4.28-4.24 (m, 2H), 4.08 (s, 3H), 3.59-3.56 (m, 2H), 3.35-3.34 (m, 2H), 2.20 (s, 3H).

### (e) (S)-5-((((6-(2-Chloro-3-(3-chloro-2-(4-methyl-1,1-dioxido-2,3,4,5-tetrahydrobenzo[f][1,4]thiazepin-8-yl)pyridin-4-yl)phenyl)-2-methoxypyridin-3-yl)methyl)amino)methyl)pyrrolidin-2-one

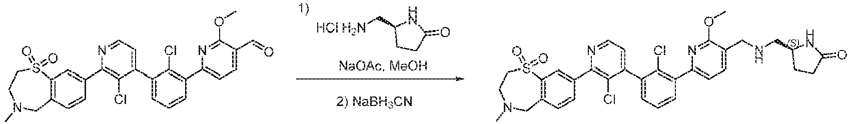

To a mixture of 6-(2-chloro-3-(3-chloro-2-(4-methyl-1,1-dioxido-2,3,4,5-tetrahydrobenzo[f][1,4]thiazepin-8-yl)pyridin-4-yl)phenyl)-2-methoxynicotinaldehyde (0.14 g, 0.25 mmol) and (S)-5-(aminomethyl)-2-pyrrolidinone hydrochloride salt (0.06 g, 0.37 mmol) in methanol (3 mL) was added sodium acetate (0.08 g, 0.99 mmol) and the mixture was stirred at room temperature for 12 h. Sodium cyanoborohydride (0.05 g, 0.74 mmol) was added and the mixture was stirred at room temperature for 1 h. The mixture was filtered, the filtrate was concentrated and the residue was purified by reverse phase HPLC to afford (S)-5-((((6-(2-chloro-3-(3-chloro-2-(4-methyl-1,1-dioxido-2,3,4,5-tetrahydrobenzo[f][1,4]thiazepin-8-yl)pyridin-4-yl)phenyl)-2-methoxypyridin-3-yl)methyl)amino)methyl)pyrrolidin-2-one (0.04 g, 24%) as a white solid (formic acid salt). MS: m/z found 666 [M+H]⁺, retention time = 2.54 min (Method A). ¹H NMR (400 MHz, Methanol-*d*₄): δ 8.62 (d, 1H), 8.28 (d, 1H), 7.94 (dd, 1H), 7.73 (d, 1H), 7.66 (dd, 1H), 7.57 (d, 1H), 7.50 (t, 1H), 7.43-7.37 (m, 2H), 7.23 (d, 1H), 4.40-4.16 (m, 2H), 3.98 (s, 3H), 3.95-3.80 (m, 3H), 3.45-3.42 (m, 4H), 2.84-2.75 (m, 2H), 2.33-2.24 (m, 6H), 1.81-1.76 (m, 1H).

### Example 416: (S)-5-((((6-(2-Chloro-3-(3-chloro-2-(1,1-dioxido-4-(((S)-5-oxopyrrolidin-2-yl)methyl)-2,3,4,5-tetrahydrobenzo[f][1,4]thiazepin-8-yl)pyridin-4-yl)phenyl)-2-methoxypyridin-3-yl)methyl)amino)methyl)pyrrolidin-2-one (582)

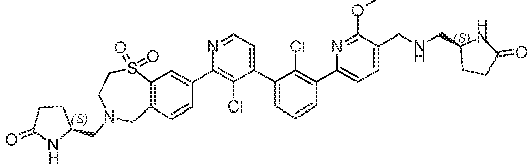

### (a) (S)-6-(2-Chloro-3-(3-chloro-2-(1,1-dioxido-4-((5-oxopyrrolidin-2-yl)methyl)-2,3,4,5-tetrahydrobenzo [f] [1,4] thiazepin-8-yl)pyridin-4-yl)phenyl)-2-methoxynicotinaldehyde

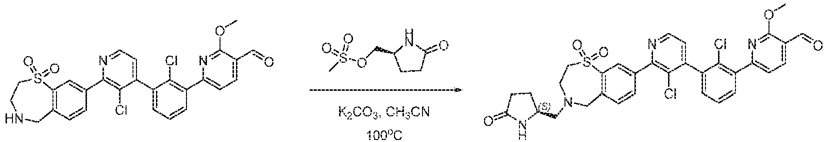

To a mixture of 6-(2-chloro-3-(3-chloro-2-(1,1-dioxido-2,3,4,5-tetrahydrobenzo[f][1,4]thiazepin-8-yl)pyridin-4-yl)phenyl)-2-methoxynicotinaldehyde (Example 415, step (b)) (0.27 g, 487 mmol) and (S)-(5-oxopyrrolidin-2-yl)methyl methanesulfonate (0.28 g, 1.46 mmol) in acetonitrile (6 mL) was added potassium carbonate (0.27 g, 1.95 mmol) and the mixture was stirred at 100 °C for 12 h. The mixture was filtered, the filtrate was concentrated and the residue purified by normal phase SiO₂ chromatography (0-10% MeOH/ethyl acetate) to afford (S)-6-(2-chloro-3-(3-chloro-2-(1,1-dioxido-4-((5-oxopyrrolidin-2-yl)methyl)-2,3,4,5-tetrahydrobenzo[f][1,4]thiazepin-8-yl)pyridin-4-yl)phenyl)-2-methoxynicotinaldehyde (0.15 g, 23%) as a yellow oil. MS: m/z found m/z: 651 [M+H]⁺.

### (b) (S)-5-((((6-(2-Chloro-3-(3-chloro-2-(1,1-dioxido-4-(((S)-5-oxopyrrolidin-2-yl)methyl)-2,3,4,5-tetrahydrobenzo[f][1,4]thiazepin-8-yl)pyridin-4-yl)phenyl)-2-methoxypyridin-3-yl)methyl)amino)methyl)pyrrolidin-2-one

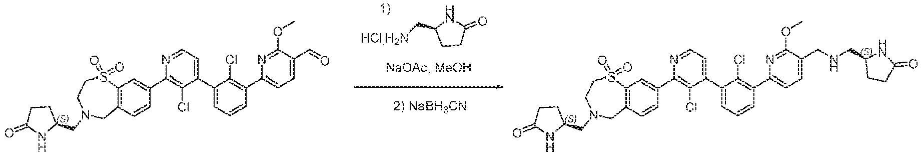

To a mixture of (S)-6-(2-chloro-3-(3-chloro-2-(1,1-dioxido-4-((5-oxopyrrolidin-2-yl)methyl)-2,3,4,5-tetrahydrobenzo[f][1,4]thiazepin-8-yl)pyridin-4-yl)phenyl)-2-methoxynicotinaldehyde (0.14 g, 215 mmol) and (S)-5-(aminomethyl)-2-pyrrolidinone hydrochloride salt (0.05 g, 0.32 mmol) in MeOH (4 mL) was added sodium acetate (0.05 g, 0.64 mmol) and the mixture was stirred at room temperature for 12 h. Sodium cyanoborohydride (0.04 g, 0.64 mmol) was added and the mixture was stirred at room temperature for 1 h. The mixture was filtered, the filtrate was concentrated and the residue purified by reverse phase HPLC to afford (S)-5-((((6-(2-chloro-3-(3-chloro-2-(1,1-dioxido-4-(((S)-5-oxopyrrolidin-2-yl)methyl)-2,3,4,5-tetrahydrobenzo[f][1,4]thiazepin-8-yl)pyridin-4-yl)phenyl)-2-methoxypyridin-3-yl)methyl)amino)methyl)pyrrolidin-2-one (12.3 mg, 8%) as a white solid. MS: m/z found 749 [M+H]⁺, retention time = 3.01 min (Method A). ¹H NMR (400 MHz, Methanol-*d*₄): δ 8.64 (d, 1H), 8.30 (d, 1H), 7.94 (dd, 1H), 7.79-7.75 (m, 1H), 7.68-7.66 (m, 1H), 7.57 (d, 1H), 7.52 (t, 1H), 7.43-7.39 (m, 2H), 7.29-7.24 (m, 1H), 4.46-4.36 (m, 2H), 4.05-4.01 (m, 5H), 3.90-3.85 (m, 2H), 3.56-3.54 (m, 2H), 3.43-3.37 (m, 2H), 2.95-2.93 (m, 2H), 2.45-2.27 (m, 7H), 2.19-2.13 (m, 1H), 1.86-1.81 (m, 1H), 1.68-1.66 (m, 1H).

### Example 417: (S)-5-((((6-(2-Chloro-3-(3-chloro-2-(4-((S)-2-hydroxypropyl)-1,1-dioxido-2,3,4,5-tetrahydrobenzo[f][1,4]thiazepin-8-yl)pyridin-4-yl)phenyl)-2-methoxypyridin-3-yl)methyl)amino)methyl)pyrrolidin-2-one (611)

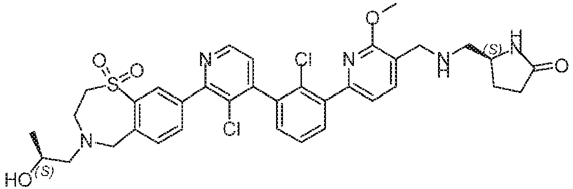

### (a) (S)-6-(2-Chloro-3-(3-chloro-2-(4-(2-hydroxypropyl)-1,1-dioxido-2,3,4,5-tetrahydrobenzo[f][1,4]thiazepin-8-yl)pyridin-4-yl)phenyl)-2-methoxynicotinaldehyde

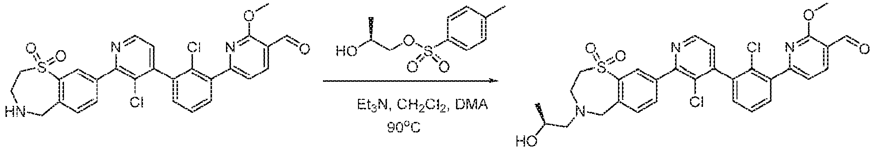

To a mixture of 6-(2-chloro-3-(3-chloro-2-(1,1-dioxido-2,3,4,5-tetrahydrobenzo[f][1,4]thiazepin-8-yl)pyridin-4-yl)phenyl)-2-methoxynicotinaldehyde (Example 415, step (b)) (150 mg, 0.27 mmol) and triethylamine (151 uL, 1.08 mmol) in dichloromethane/DMA (1:1, 1 mL) was added (S)-2-hydroxypropyl 4-methylbenzenesulfonate (249 mg, 1.08 mmol) in a sealed tube and then the mixture was stirred for 12 h at 90 °C. The mixture was diluted with brine (20 mL) and extracted with dichloromethane (100 mL). The organic phase was dried with anhydrous sodium sulfate, filtered and concentrated. The residue was purified by normal phase SiO₂ chromatography (0-100% EtOAc/petroleum ether) to afford (S)-6-(2-chloro-3-(3-chloro-2-(4-(2-hydroxypropyl)-1,1-dioxido-2,3,4,5-tetrahydrobenzo[f][1,4]thiazepin-8-yl)pyridin-4-yl)phenyl)-2-methoxynicotinaldehyde (135 mg, 34%) as a light yellow solid. MS: m/z found 612 and 614 [M+H]⁺. ¹H NMR (400 MHz, Chloroform-*d*): δ 10.36 (s, 1 H), 8.62 (d,1 H), 8.45 (d, 1 H), 8.14 (d, 1 H), 7.92 (d, 1 H), 7.67 (dd, 1 H), 7.43 (t,1 H), 7.35 (d, 1 H), 7.30 (dd, 1 H), 7.25 (d, 1 H), 4.07 (s, 3 H), 3.98 (br s, 1 H), 3.31-3.23 (m, 2 H), 2.95 (s, 2 H), 2.87 (s, 1 H), 2.37 (d,1 H), 2.08 (d, 1 H), 1.03-1.01 (m, 3 H).

### (b) (S)-5-((((6-(2-Chloro-3-(3-chloro-2-(4-((S)-2-hydroxypropyl)-1,1-dioxido-2,3,4,5-tetrahydrobenzo[f][1,4]thiazepin-8-yl)pyridin-4-yl)phenyl)-2-methoxypyridin-3-yl)methyl)amino)methyl)pyrrolidin-2-one

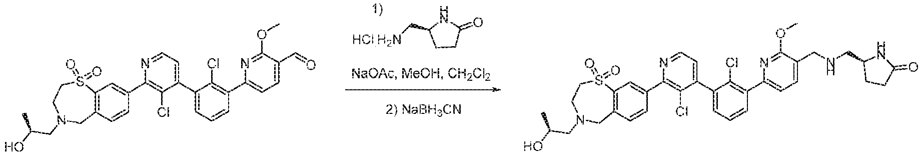

To a mixture of (S)-6-(2-chloro-3-(3-chloro-2-(4-(2-hydroxypropyl)-1,1-dioxido-2,3,4,5-tetrahydrobenzo[f][1,4]thiazepin-8-yl)pyridin-4-yl)phenyl)-2-methoxynicotinaldehyde (130 mg, 0.21 mmol) in MeOH/dichloromethane (6 mL, 1/1) was added (S)-5-(aminomethyl)pyrrolidin-2-one hydrochloride salt (48 mg, 0.32 mmol) and sodium acetate (87 mg, 1.06 mmol) and the mixture was stirred for 2 h at room temperature. Sodium cyanoborohydride (26 mg, 0.42 mmol) was added and the mixture stirred for another 10 h. The mixture was concentrated under reduced pressure and the residue was purified by reverse phase HPLC to afford (S)-5-((((6-(2-chloro-3-(3-chloro-2-(4-((S)-2-hydroxypropyl)-1,1-dioxido-2,3,4,5-tetrahydrobenzo[f][1,4]thiazepin-8-yl)pyridin-4-yl)phenyl)-2-methoxypyridin-3-yl)methyl)amino)methyl)pyrrolidin-2-one (66 mg, 41% yield) as a white solid (formic acid salt). MS: m/z found 710 and 712 [M+H]⁺, retention time = 2.63 min (Method A). ¹H NMR (400 MHz, Methanol-*d*₄): δ 8.70 (d, 1H), 8.37 (d, 1H), 8.00 (dd, 1H), 7.80 (d, 1H), 7.74 (dd, 1H), 7.62-7.56 (m, 2H), 7.50 (d, 1H), 7.46 (d, 1H), 7.31 (d, 1H), 4.44 (br s, 2H), 4.06 (s, 3H), 4.02-3.92 (m, 4H), 3.65-3.64 (m, 2H), 3.50-3.45 (m, 2H), 2.87-2.84 (m, 2H), 2.42-2.33 (m, 5H), 1.89-1.84 (m, 1H), 1.11 (d, 3H).

### Example 418: 5-(3-Chloro-4-(2-chloro-3-(5-((((S)-2-hydroxypropyl)amino)methyl)-6-methoxypyridin-2-yl)phenyl)pyridin-2-yl)-2-(2-((((S)-5-oxopyrrolidin-2-yl)methyl) amino)ethyl)isoindolin-1-one (142)

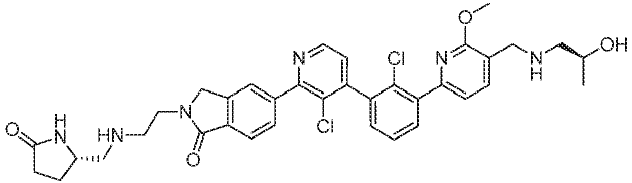

### (a) 5-Bromo-2-(2,2-dimethoxyethyl)isoindolin-1-one

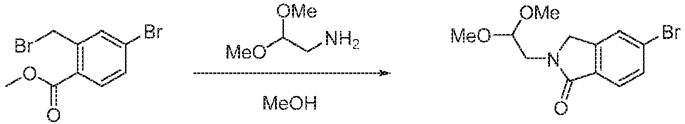

To a mixture of methyl 4-bromo-2-(bromomethyl)benzoate (3 g, 9.74 mmol) in methanol (60 mL) was added 2,2-dimethoxyethanamine (5.31 mL, 48.7 mmol) in one portion under N₂. The mixture was stirred at room temperature for 2 hr. The mixture was concentrated. To the residue was added water (30 mL) and saturated aqueous brine solution (30 mL). The mixture was extracted with ethyl acetate/THF mixture (1:1, 100 mL). The organic phase was dried with anhydrous sodium sulfate, filtered and concentrated. The residue was purified by normal phase SiO₂ chromatography (0-50% ethyl acetate/petroleum ether) to afford 5-bromo-2-(2,2-dimethoxyethyl)isoindolin-1-one as a white solid (2.7 g, 92% yield).

### (b) 2-(5-Bromo-1-oxoisoindolin-2-yl)acetaldehyde

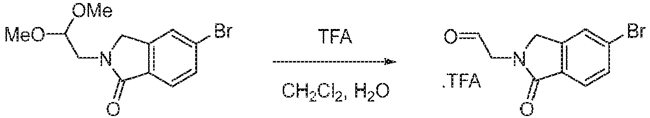

To a mixture of 5-bromo-2-(2,2-dimethoxyethyl)isoindolin-1-one (1.3 g, 4.33 mmol) in dichloromethane (30 mL) was added trifluoroacetic acid (6.50 mL, 87.8 mmol) and water (1 mL) in one portion. The mixture was stirred at room temperature for 2 h. The mixture was concentrated under vacuum directly to afford 2-(5-bromo-1-oxoisoindolin-2-yl)acetaldehyde trifluoroacetic acid salt as a yellow oil (1.8 g) which was used into the next step without further purification.

### (c) (S)-5-Bromo-2-(2-(((5-oxopyrrolidin-2-yl)methyl)amino)ethyl)isoindolin-1-one

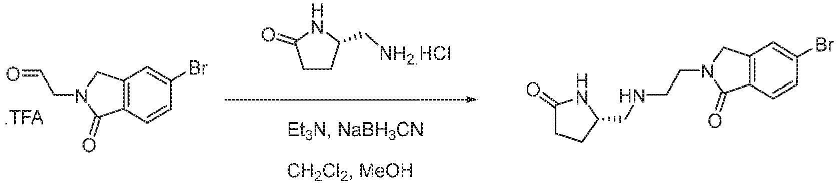

To a mixture of 2-(5-bromo-1-oxoisoindolin-2-yl)acetaldehyde (1.8 g, 7.08 mmol) in dichloromethane / methanol (1:1, 40 mL) was added triethylamine (9.86 mL, 70.8 mmol) followed by (S)-5-(aminomethyl)pyrrolidin-2-one hydrochloride salt (1.07 g, 7.08 mmol) and sodium cyanoborohydride (1.34 g, 21.2 mmol) . The mixture was stirred at room temperature for 12 h. The crude light yellow suspension of (S)-5-bromo-2-(2-(((5-oxopyrrolidin-2-yl)methyl)amino)ethyl)isoindolin-1-one (2.5 g) in dichloromethane / methanol (1:1 40 mL) was used in the next step.

### (d) tert-Butyl (S)-(2-(5-bromo-1-oxoisoindolin-2-yl)ethyl)((5-oxopyrrolidin-2-yl)methyl)carbamate

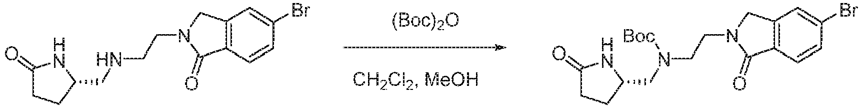

To a solution of crude (S)-5-bromo-2-(2-(((5-oxopyrrolidin-2-yl)methyl)amino)ethyl) isoindolin-1-one (2.5 g, 7.10 mmol) in dichloromethane / methanol (1: 140 mL), was added di-tert-butyl dicarbonate (3.10 g, 14.2 mmol), and the reaction was stirred at room temperature for 2 hr. The mixture was concentrated under vacuum, water (30 mL) was added and the mixture was extracted with THF/ethyl acetate (1/3, 100 mL), dried over anhydrous sodium sulfate and filtered. The filtrate was concentrated and the residue purified by normal phase SiO₂ chromatography (0-100% ethyl acetate/petroleum ether to 0-20% methanol/ethyl acetate) to afford (S)-tert-butyl (2-(5-bromo-1-oxoisoindolin-2-yl)ethyl)((5-oxopyrrolidin-2-yl)methyl) carbamate (1.5 g, 44% yield) as a light yellow oil.

### (e) (S)-tert-Butyl (2-(1-oxo-5-(4,4,5,5-tetramethyl-1,3,2-dioxaborolan-2-yl)isoindolin-2-yl)ethyl)((5-oxopyrrolidin-2-yl)methyl)carbamate

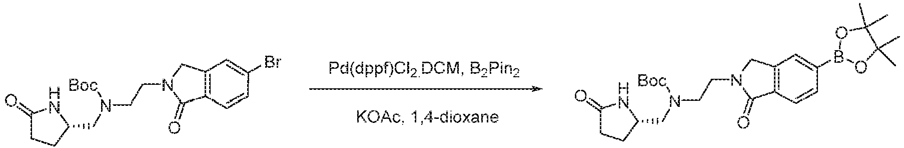

To a mixture of (S)-tert-butyl(2-(5-bromo-1-oxoisoindolin-2-yl)ethyl) ((5-oxopyrrolidin -2-yl)methyl)carbamate (1 g, 2.21 mmol) and bis(pinacolato)diborane (1.68 g, 6.63 mmol) in 1,4-dioxane (20 mL) was added potassium acetate (434 mg, 4.42 mmol), followed by [1,1'- bis(diphenylphosphino)ferrocene]dichloropalladium(II) complex with dichloromethane (181 mg, 0.22 mmol). The reaction was stirred for 3 hr at 130 °C. After cooling, the mixture was filtered and washed with ethyl acetate (100 ml). The filtrate was concentrated under reduced pressure. The residue was purified by normal phase SiO₂ chromatography (0-100% ethyl acetate/petroleum ether to 0- 20% methanol/ethyl acetate) to afford (S)-tert butyl (2-(1-oxo-5-(4,4,5,5-tetramethyl-1,3,2-dioxaborolan-2-yl)isoindolin-2-yl) ethyl)((5-oxopyrrolidin-2-yl)methyl)carbamate (0.41 g, 37% yield) as an orange solid. ¹H NMR (400 MHz, Methanol-*d*₄): δ 7.93 (s, 1H), 7.90-7.84 (m, 1H), 7.81-7.73 (m, 1H), 4.61 (d, 2 H), 3.98 (s, 1 H), 3.82 (s, 2 H), 3.68-3.57 (m, 2 H), 3.37 (s, 2 H), 2.40-2.24 (m, 3 H), 1.84 (br, 1 H), 1.39 (s, 12 H), 1.22 (s, 9 H).

### (f) tert-butyl (S)-(2-(5-(3-chloro-4-(2-chloro-3-(5-formyl-6-methoxypyridin-2-yl)phenyl)pyridin-2-yl)-1-oxoisoindolin-2-yl)ethyl)((5-oxopyrrolidin-2-yl)methyl)carbamate

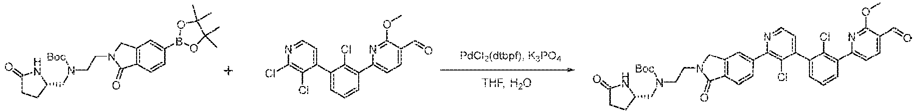

To a mixture of 6-(2-chloro-3-(2,3-dichloropyridin-4-yl)phenyl)-2-methoxynicotinaldehyde (100 mg, 0.25 mmol) and (S)-tert-butyl (2-(1-oxo-5-(4,4,5,5-tetramethyl-1,3,2-dioxaborolan-2-yl)isoindolin-2-yl) ethyl)((5-oxopyrrolidin-2-yl)methyl)carbamate (178 mg, 0.36 mmol) in THF / water (5/1, 6 mL) was added potassium phosphate (162 mg, 0.76 mmol), followed by [1,1'-bis(di-tert-butylphosphino)ferrocene]dichloropalladium(II) (16.6 mg, 0.03 mmol) and then the reaction was stirred for 12 hr at 85 °C. After cooling, water (10 mL) was added and the mixture extracted with ethyl acetate (200 ml). The organic layer was dried over anhydrous sodium sulfate, filtered and concentrated. The residue was purified by prep-TLC (20% methanol/ethyl acetate) to afford tert-butyl (S)-(2-(5-(3-chloro-4-(2-chloro-3-(5-formyl-6-methoxypyridin-2-yl)phenyl)pyridin-2-yl)-1-oxoisoindolin-2-yl)ethyl)((5-oxopyrrolidin-2-yl)methyl)carbamate (0.06 g, 24% yield) as a white solid.

### (g) tert-Butyl (2-(5-(3-chloro-4-(2-chloro-3-(5-((((S)-2-hydroxypropyl)amino)methyl)-6-methoxypyridin-2-yl)phenyl)pyridin-2-yl)-1-oxoisoindolin-2-yl)ethyl)(((S)-5-oxopyrrolidin-2-yl)methyl)carbamate

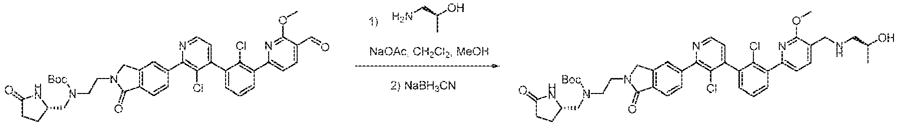

To a mixture of tert-butyl (S)-(2-(5-(3-chloro-4-(2-chloro-3-(5-formyl-6-methoxypyridin-2-yl)phenyl)pyridin-2-yl)-1-oxoisoindolin-2-yl)ethyl)((5-oxopyrrolidin-2-yl)methyl)carbamate (53 mg, 0.07 mmol) in dichloromethane / methanol (1/1, 4 mL) was added sodium acetate (17.9 mg, 0.22 mmol) and (S)-1-aminopropan-2-ol (6.54 mg, 0.09 mmol). The mixture was stirred at room temperature for 2 h. Sodium cyanoborohydride (13.7 mg, 0.22 mmol) was then added and the mixture stirred for an additional 13 hr. The mixture was concentrated under reduced pressure to afford tert-butyl (2-(5-(3-chloro-4-(2-chloro-3-(5-((((S)-2-hydroxypropyl)amino)methyl)-6-methoxypyridin-2-yl)phenyl)pyridin-2-yl)-1-oxoisoindolin-2-yl)ethyl)(((S)-5-oxopyrrolidin-2-yl)methyl)carbamate as a yellow solid (63 mg, crude) which was used into the next step without further purification.

### (h) 5-(3-Chloro-4-(2-chloro-3-(5-((((S)-2-hydroxypropyl)amino)methyl)-6-methoxypyridin-2-yl)phenyl)pyridin-2-yl)-2-(2-((((S)-5-oxopyrrolidin-2-yl)methyl) amino)ethyl)isoindolin-1-one

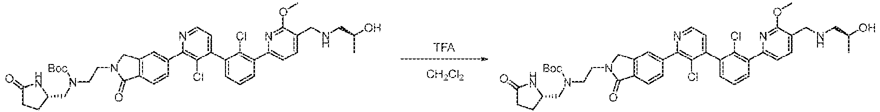

To a mixture of tert-butyl (2-(5-(3-chloro-4-(2-chloro-3-(5-((((S)-2-hydroxypropyl)amino)methyl)-6-methoxypyridin-2-yl)phenyl)pyridin-2-yl)-1-oxoisoindolin-2-yl)ethyl)(((S)-5-oxopyrrolidin-2-yl)methyl)carbamate (63 mg, 0.08 mmol) in dichloromethane (1 mL) was added trifluoroacetic acid (1 mL, 13.5 mmol) and the mixture stirred for 0.5 hr at room temperature. The mixture was concentrated under reduced pressure and the residue was purified by reverse phase HPLC to afford 5-(3-chloro-4-(2-chloro-3-(5-((((S)-2-hydroxypropyl)amino)methyl)-6-methoxypyridin-2-yl)phenyl)pyridin-2-yl)-2-(2-((((S)-5-oxopyrrolidin-2-yl)methyl) amino)ethyl)isoindolin-1-one (16 mg, 26% yield) as a white solid (formic acid salt). MS: m/z found 689 [M+H]⁺, retention time 2.54 min (Method A). ¹H NMR (400 MHz, Methanol-*d*₄): δ 8.67 (d, 1 H), 8.45 (br s, 1 H), 7.91-7.80 (m, 4 H), 7.72 (dd, 1H), 7.57 (t, 1H), 7.48-7.44 (m, 2H), 7.35 (d, 1H), 4.67 (s, 2H), 4.24 (s, 2H), 4.08-4.04 (m, 4H), 3.83-3.78 (m, 3H), 3.06-3.02 (m, 3H), 2.87-2.76 (m, 3H), 2.34-2.25 (m, 3H), 1.82-1.80 (m, 1H), 1.23 (d, 3H).

### Example 419: (S)-5-(4-(3-(5-(((1-Acetylpiperidin-4-yl)amino)methyl)-6-methoxypyridin-2-yl)-2-chlorophenyl)-3-chloropyridin-2-yl)-2-(2-(((5-oxopyrrolidin-2-yl)methyl)amino)ethyl)isoindolin-1-one (143)

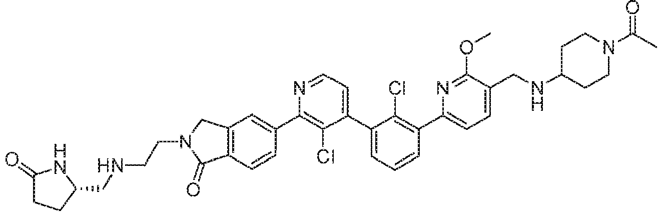

(S)-5-(4-(3-(5-(((1-Acetylpiperidin-4-yl)amino)methyl)-6-methoxypyridin-2-yl)-2-chlorophenyl)-3-chloropyridin-2-yl)-2-(2-(((5-oxopyrrolidin-2-yl)methyl)amino)ethyl)isoindolin-1-one (27.3 mg, 20% yield) was prepared in a similar fashion to Example 418, using tert-butyl (S)-(2-(5-(3-chloro-4-(2-chloro-3-(5-formyl-6-methoxypyridin-2-yl)phenyl)pyridin-2-yl)-1-oxoisoindolin-2-yl)ethyl)((5-oxopyrrolidin-2-yl)methyl)carbamate and 1-(4-aminopiperidin-1-yl)ethanone. MS: m/z found 756 and 758 [M+H]⁺, retention time = 2.57 min (Method A). ¹H NMR (400 MHz, Methanol-*d*₄): δ 8.70 (d, 1 H), 8.37 (br s, 1 H), 7.94-7.90 (m, 3 H), 7.85 (dd, 1 H), 7.74 (dd, 1 H), 7.60 (t, 1 H), 7.51-7.47 (m, 2 H), 7.38 (d, 1 H), 4.71 (s, 2 H), 4.66 (s, 1 H), 4.28 (s, 2 H), 4.11 (s, 3 H), 4.08 (s, 1 H), 3.92-3.87 (m, 3 H), 3.43-3.33 (m, 1 H), 3.18-3.15 (m, 3 H), 2.96-2.92 (m, 2 H), 2.73-2.70 (m, 1 H), 2.39-2.21 (m, 5 H), 2.15 (s, 3 H), 1.89-1.84 (m, 1 H), 1.66-1.51 (m, 2 H).

### Example 420: (S)-5-(4-(3-(5-((6-Acetyl-2,6-diazaspiro[3.3]heptan-2-yl)methyl)-6-methoxypyridin-2-yl)-2-chlorophenyl)-3-chloropyridin-2-yl)-2-(2-(((5-oxopyrrolidin-2-yl)methyl)amino)ethyl)isoindolin-1-one (144)

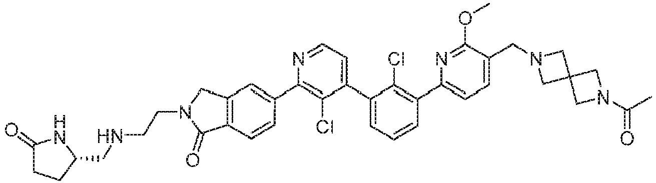

(S)-5-(4-(3-(5-((6-Acetyl-2,6-diazaspiro[3.3]heptan-2-yl)methyl)-6-methoxypyridin-2-yl)-2-chlorophenyl)-3-chloropyridin-2-yl)-2-(2-(((5-oxopyrrolidin-2-yl)methyl)amino)ethyl)isoindolin-1-one (17.6 mg, 12% yield) was prepared in a similar fashion to Example 418, using tert-butyl (S)-(2-(5-(3-chloro-4-(2-chloro-3-(5-formyl-6-methoxypyridin-2-yl)phenyl)pyridin-2-yl)-1-oxoisoindolin-2-yl)ethyl)((5-oxopyrrolidin-2-yl)methyl)carbamate and 1-(2,6-diazaspiro[3.3]heptan-2-yl)ethanone trifluoroacetic acid salt. MS: m/z found 754 and 756 [M+H]⁺, retention time = 2.48 (Method A). ¹H NMR (400 MHz, Methanol-*d*₄): δ 8.70 (d, 1H), 8.32 (br s, 2H), 7.95-7.92 (m, 2H), 7.86-7.81 (m, 2H), 7.74 (dd, 1H), 7.60 (t, 1H), 7.50 (d, 1H), 7.47 (dd, 1H), 7.34 (d, 1H), 4.71 ( s, 2H), 4.38 (s, 2H), 4.14 (d, 4H), 4.07 (s, 3H), 4.05 (s, 4H), 3.94-3.91 (m, 3H), 3.24-3.22 (m, 2H), 3.02-3.00 (m, 2H), 2.41-2.32 (m, 3H), 1.91-1.87 (m, 4H).

### Example 421: 5-(3-Chloro-4-(2-chloro-3-(4-fluoro-6-methoxy-5-(((((S)-5-oxopyrrolidin-2-yl)methyl)amino)methyl)pyridin-2-yl)phenyl)pyridin-2-yl)-2-(2-((((S)-5-oxopyrrolidin-2-yl)methyl)amino)ethyl)isoindolin-1-one (178)

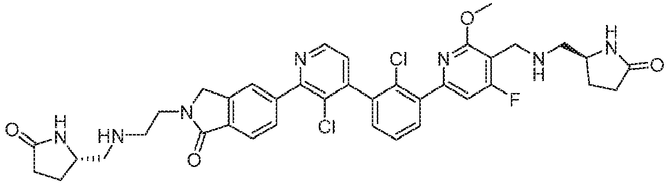

### (a) 6-(3-Bromo-2-chlorophenyl)-4-fluoro-2-methoxynicotinaldehyde

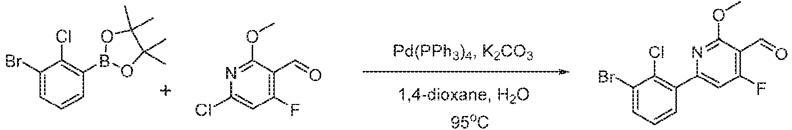

To a solution of 2-(3-bromo-2-chlorophenyl)-4,4,5,5-tetramethyl-1,3,2-dioxaborolane (4 g, 12.7 mmol), 6-chloro-4-fluoro-2-methoxynicotinaldehyde (3 g, 15.8 mmol) and potassium carbonate (4.37 g, 31.7 mmol) in 1,4-dioxane / water mixture (6:1, 35 mL) was added tetrakis(triphenylphosphine)palladium(0) (0.9 g, 0.8 mmol) and the mixture was stirred at 95 °C for 1 h under N₂ atmosphere. To the mixture was added water (50 mL) and the mixture was extracted with ethyl acetate (3 x 20 mL). The combined organic phases were washed with saturated aqueous brine solution (2 x 10 mL), dried with anhydrous sodium sulfate, filtered and concentrated. The residue was purified by normal phase SiO₂ chromatography (0-2% ethyl acetate/petroleum ether) to afford 6-(3-bromo-2-chlorophenyl)-4-fluoro-2-methoxynicotinaldehyde (3.3 g, 58% yield) as a yellow solid. MS: m/z found 344 [M+H]⁺.

### (b) 6-(2-Chloro-3-(2,3-dichloropyridin-4-yl)phenyl)-4-fluoro-2-methoxynicotinaldehyde

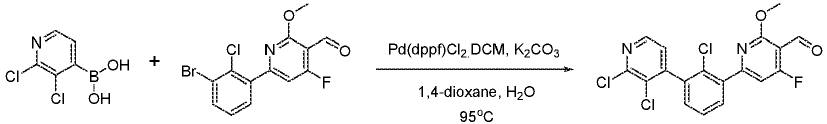

To a mixture of 6-(3-bromo-2-chlorophenyl)-4-fluoro-2-methoxynicotinaldehyde (2.3 g, 6.62 mmol) in 1,4-dioxane/H₂O mixture (6:1, 14 mL) was added potassium carbonate (2.74 g, 19.9 mmol) and [1,1'- bis(diphenylphosphino)ferrocene]dichloropalladium(II) complex with dichloromethane (0.27 g, 0.33 mmol) in one portion under N₂ atmosphere. (2,3-Dichloropyridin-4-yl)boronic acid (1.27 g, 6.62 mmol) in 1,4-dioxane (8 mL) was dropwise added to the mixture at 95 °C for 2 h. To the mixture was added water (5 mL) and the mixture was extracted with ethyl acetate (2 x 5 mL). The combined organic phases were washed with saturated aqueous brine solution (2 x 5 mL), dried with anhydrous sodium sulfate, filtered and concentrated. The residue was purified by normal phase SiO₂ chromatography (0-30% ethyl acetate/petroleum ether) to afford 6-(2-chloro-3-(2,3-dichloropyridin-4-yl)phenyl)-4-fluoro-2-methoxynicotinaldehyde (1.25 g, 45% yield) as yellow oil. MS: m/z found 411 and 413[M+H]⁺.

### (c) tert-Butyl (S)-((6-(2-chloro-3-(2,3-dichloropyridin-4-yl)phenyl)-4-fluoro-2-methoxypyridin-3-yl)methyl)((5-oxopyrrolidin-2-yl)methyl)carbamate

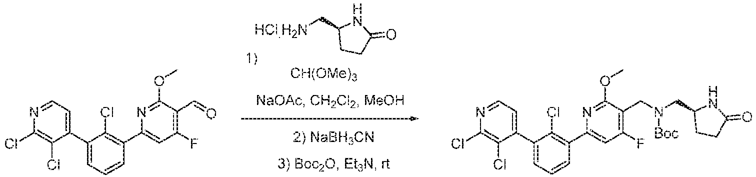

A solution of 6-(2-chloro-3-(2,3-dichloropyridin-4-yl)phenyl)-4-fluoro-2-methoxynicotinaldehyde (1.28 g, 3.11 mmol), (S)-5-(aminomethyl)-2-pyrrolidinone hydrochloride salt (0.7 g, 4.66 mmol) and sodium acetate (0.89 g, 10.9 mmol) in dichloromethane / MeOH mixture (1:1, 20 mL) and trimethyl orthoformate (3 mL) was stirred at room temperature for 12 h. Sodium cyanoborohydride (0.59 g, 9.33 mmol) was then added and the mixture stirred at room temperature for 0.5 h to give (S)-5-((((6-(2-chloro-3-(2,3-dichloropyridin-4-yl)phenyl)-4-fluoro-2-methoxypyridin-3-yl)methyl)amino)methyl)pyrrolidin-2-one (MS: m/z found 509 and 511 [M+H]⁺ observed).

Di-tert-butyl dicarbonate (1.8 mL, 7.65 mmol) and triethylamine (0.64 mL, 4.59 mmol) were then added and the mixture was stirred at room temperature for 20 min. To the mixture was added water (5 mL) and the mixture was extracted with ethyl acetate (2 x 5 mL). The combined organic phases were washed with saturated aqueous brine solution (2 x 5 mL), dried with anhydrous sodium sulfate, filtered and concentrated. The residue was purified by normal phase SiO₂ chromatography (0-100% ethyl acetate/petroleum ether) to afford tert-butyl (S)-((6-(2-chloro-3-(2,3-dichloropyridin-4-yl)phenyl)-4-fluoro-2-methoxypyridin-3-yl)methyl)((5-oxopyrrolidin-2-yl)methyl)carbamate (1.5 g, 80% yield for two steps) as a yellow solid. (MS: m/z found 609 [M+H]⁺.

### (d) tert-Butyl ((6-(3-(2-(2-(2-((tert-butoxycarbonyl)(((S)-5-oxopyrrolidin-2-yl)methyl)amino)ethyl)-1-oxoisoindolin-5-yl)-3-chloropyridin-4-yl)-2-chlorophenyl)-4-fluoro-2-methoxypyridin-3-yl)methyl)(((S)-5-oxopyrrolidin-2-yl)methyl)carbamate

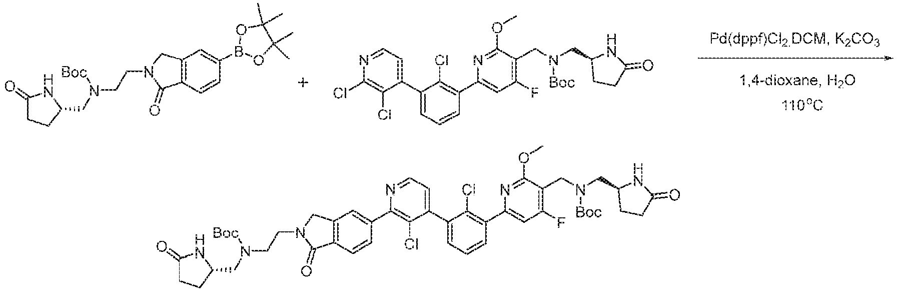

To a mixture of tert-butyl (S)-(2-(1-oxo-5-(4,4,5,5-tetramethyl-1,3,2-dioxaborolan-2-yl)isoindolin-2-yl)ethyl)((5-oxopyrrolidin-2-yl)methyl)carbamate (Example 418, step (e)) (0.1 g, 0.2 mmol) and tert-butyl (S)-((6-(2-chloro-3-(2,3-dichloropyridin-4-yl)phenyl)-4-fluoro-2-methoxypyridin-3-yl)methyl)((5-oxopyrrolidin-2-yl)methyl)carbamate (0.11 g, 0.18 mmol) in 1,4-dioxane/water mixture (7.5:1, 3.4 mL) was added potassium carbonate (0.07 g, 0.54 mmol) and [1,1'- bis(diphenylphosphino)ferrocene]dichloropalladium(II) complex with dichloromethane (15 mg, 18 umol) in one portion under N₂ atmosphere and the mixture was stirred at 110 °C for 3 h. The mixture was concentrated. To the residue was added water (5 mL) and saturated aqueous brine solution (5 mL). The mixture was extracted with ethyl acetate/THF mixture (1:1, 10 mL), the organic phase was dried with anhydrous sodium sulfate, filtered and concentrated. The residue was purified by prep-TLC (silica gel, ethyl acetate: MeOH = 4:1) to afford tert-butyl ((6-(3-(2-(2-(2-((tert-butoxycarbonyl)(((S)-5-oxopyrrolidin-2-yl)methyl)amino)ethyl)-1-oxoisoindolin-5-yl)-3-chloropyridin-4-yl)-2-chlorophenyl)-4-fluoro-2-methoxypyridin-3-yl)methyl)(((S)-5-oxopyrrolidin-2-yl)methyl)carbamate (0.06 g, 40% yield) as a yellow solid. MS: m/z found 946 [M+H]⁺.

### (e) 5-(3-Chloro-4-(2-chloro-3-(4-fluoro-6-methoxy-5-(((((S)-5-oxopyrrolidin-2-yl)methyl)amino)methyl)pyridin-2-yl)phenyl)pyridin-2-yl)-2-(2-((((S)-5-oxopyrrolidin-2-yl)methyl)amino)ethyl)isoindolin-1-one

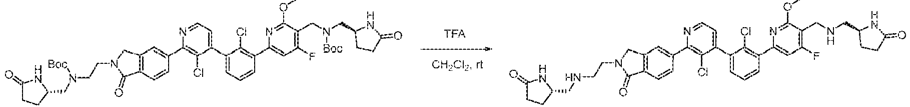

To a mixture of tert-butyl ((6-(3-(2-(2-(2-((tert-butoxycarbonyl)(((S)-5-oxopyrrolidin-2-yl)methyl)amino)ethyl)-1-oxoisoindolin-5-yl)-3-chloropyridin-4-yl)-2-chlorophenyl)-4-fluoro-2-methoxypyridin-3-yl)methyl)(((S)-5-oxopyrrolidin-2-yl)methyl)carbamate (57 mg, 0.06 mmol) in dichloromethane (0.5 mL) was added trifluoroacetic acid (1.5 mL, 20 mmol) in one portion and the mixture was stirred at room temperature for 15 min. The mixture was concentrated and the residue was purified by reverse phase HPLC to afford 5-(3-chloro-4-(2-chloro-3-(4-fluoro-6-methoxy-5-(((((S)-5-oxopyrrolidin-2-yl)methyl)amino)methyl)pyridin-2-yl)phenyl)pyridin-2-yl)-2-(2-((((S)-5-oxopyrrolidin-2-yl)methyl)amino)ethyl)isoindolin-1-one (7.3 mg, 15% yield) as a light yellow solid (formic acid salt). MS: m/z found 746 [M+H]⁺, retention time = 2.48 min (Method A). ¹H NMR (400 MHz, Methanol-*d*₄): δ 8.70 (d, 1H), 7.95-7.92 (m, 2H), 7.85 (d, 1H), 7.76 (dd, 1H), 7.60 (t, 1H), 7.51-7.47 (m, 2H), 7.19 (d, 1H), 4.71 (s, 2H), 4.09 (s, 3H), 4.02 (s, 2H), 3.94-3.88 (m, 4H), 3.23-3.22 (m, 2H), 3.03-2.96 (m, 2H), 2.86-2.81 (m, 2H), 2.43-2.31 (m, 6H), 1.91-1.80 (m, 2H).

### Example 422: 5-(3-Chloro-4-(2-chloro-3'-fluoro-5'-methoxy-4'-(((((S)-5-oxopyrrolidin-2-yl)methyl)amino)methyl)-[1,1'-biphenyl]-3-yl)pyridin-2-yl)-2-(2-((((S)-5-oxopyrrolidin-2-yl)methyl)amino)ethyl)isoindolin-1-one (242)

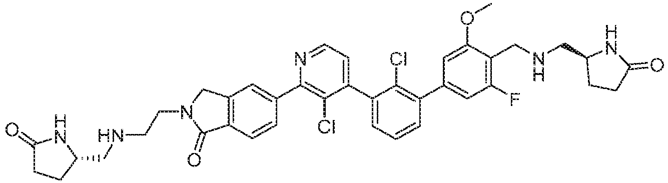

### (a) 3'-Bromo-2'-chloro-3-fluoro-5-methoxy-[1,1'-biphenyl]-4-carbaldehyde

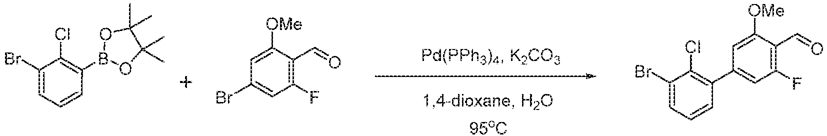

To a mixture of 2-(3-bromo-2-chlorophenyl)-4,4,5,5-tetramethyl-1,3,2-dioxaborolane (2.7 g, 8.5 mmol) and 4-bromo-2-fluoro-6-methoxybenzaldehyde (1.8 g, 7.7 mmol) in 1,4-dioxane/water mixture (6:1, 60 mL) was added potassium carbonate (3.2 g, 23 mmol) and tetrakis(triphenylphosphine)palladium(0) (0.4 g, 0.3 mmol) and the mixture was stirred at 95 °C for 3 hr. The mixture was concentrated, water (20 mL) was added to the residue, and then the mixture extracted with EtOAc (2 x 40 mL). The combined organic phases were dried with anhydrous sodium sulfate, filtered and concentrated. The residue was purified by normal phase SiO₂ chromatography (0-10% EtOAc/petroleum ether) to afford 3'-bromo-2'-chloro-3-fluoro-5-methoxy-[1,1'-biphenyl]-4-carbaldehyde (2.1 g, 79% yield) as a yellow solid. ¹H NMR (400 MHz, CDCl₃): δ 10.39 (s, 1H), 7.64-7.62 (m, 1H), 7.20-7.14 (m, 2H), 6.73-6.69 (m, 2H), 3.89 (s, 3H).

### (b) 2'-Chloro-3'-(2,3-dichloropyridin-4-yl)-3-fluoro-5-methoxy-[1,1'-biphenyl]-4-carbaldehyde

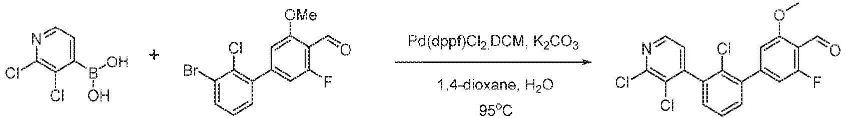

To a mixture of 3'-bromo-2'-chloro-3-fluoro-5-methoxy-[1,1'-biphenyl]-4-carbaldehyde (1.5 g, 4.4 mmol) in 1,4-dioxane/water mixture (6:1, 18 mL) was added potassium carbonate (1.8 g, 13 mmol) and [1,1'-bis(diphenylphosphino)ferrocene]dichloropalladium(II) complex with dichloromethane (0.2 g, 0.22 mmol) in one portion under N₂. (2,3-Dichloropyridin-4-yl)boronic acid (0.9 g, 4.8 mmol) in 1,4-dioxane (10 mL) was dropwise added to the mixture at 95 °C for 4 hr. The mixture was concentrated. To the residue was added water (5 mL), then the mixture extracted with EtOAc (2 x 5 mL). The combined organic phases were dried with anhydrous sodium sulfate, filtered and concentrated. The residue was purified by normal phase SiO₂ chromatography (20-25% EtOAc/petroleum ether) to afford 2'-chloro-3'-(2,3-dichloropyridin-4-yl)-3-fluoro-5-methoxy-[1,1'-biphenyl]-4-carbaldehyde (0.6 g, 33% yield) as a yellow solid. MS: m/z found 410 and 412 [M+H]⁺. ¹H NMR (400 MHz, DMSO-*d*₆): δ 10.35 (s, 1H), 8.54 (d, 1H), 7.64-7.63 (m, 2H), 7.58-7.55 (m, 2H), 7.15 (s, 1H), 7.07-7.04 (d, 1H), 3.99 (s, 3H).

### (c) tert-Butyl (S)-((2'-chloro-3'-(2,3-dichloropyridin-4-yl)-3-fluoro-5-methoxy-[1,1'-biphenyl]-4-yl)methyl)((5-oxopyrrolidin-2-yl)methyl)carbamate

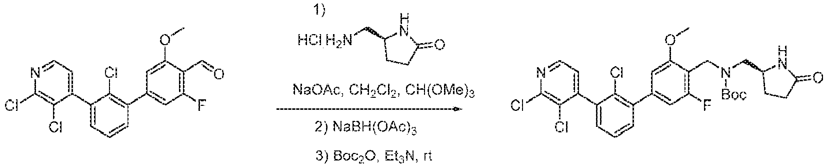

A mixture of 2'-chloro-3'-(2,3-dichloropyridin-4-yl)-3-fluoro-5-methoxy-[1,1,1'-biphenyl]-4-carbaldehyde (0.9 g, 2.2 mmol), (S)-5-(aminomethyl)-2-pyrrolidinone hydrochloride salt (0.4 g, 2.6 mmol) and sodium acetate (0.54 g, 6.6 mmol) in dichloromethane (30 mL) and trimethyl orthoformate (3 mL) was stirred at room temperature for 12 hr. Sodium triacetoxyborohydride (0.93 g, 4.4 mmol) was then added and the mixture was stirred at room temperature for 0.5 hr. to give (S)-5-((((2'-chloro-3'-(2,3-dichloropyridin-4-yl)-3-fluoro-5-methoxy-[1,1'-biphenyl]-4-yl)methyl)amino)methyl)pyrrolidin-2-one (MS: m/z found 508 and 510 [M+H]⁺).

Triethylamine (0.45 mL, 3.24 mmol) and di-tert-butyl dicarbonate (0.94 g, 4.32 mmol) were then added and the mixture was stirred at room temperature for 0.5 hr. Water (10 mL) was added and the mixture extracted with EtOAc (2 x 5 mL). The combined organic phases were dried with anhydrous sodium sulfate, filtered and concentrated. The residue was purified by prep-TLC (silica gel, EtOAc : MeOH = 10: 1) to afford tert-butyl (S)-((2'-chloro-3'-(2,3-dichloropyridin-4-yl)-3-fluoro-5-methoxy-[1,1'-biphenyl]-4-yl)methyl)((5-oxopyrrolidin-2-yl)methyl)carbamate (0.9 g, 67% yield) as a yellow solid. MS: m/z found 552 and 554 [M-t-butyl]⁺).

### (d) tert-Butyl ((3'-(2-(2-(2-((tert-butoxycarbonyl)(((S)-5-oxopyrrolidin-2-yl)methyl)amino)ethyl)-1-oxoisoindolin-5-yl)-3-chloropyridin-4-yl)-2'-chloro-3-fluoro-5-methoxy-[1,1'-biphenyl]-4-yl)methyl)(((S)-5-oxopyrrolidin-2-yl)methyl)carbamate

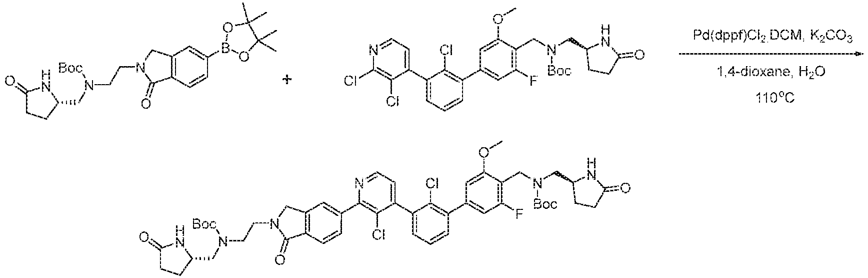

To a mixture of (S)-((2'-chloro-3'-(2,3-dichloropyridin-4-yl)-3-fluoro-5-methoxy-[1,1'-biphenyl]-4-yl)methyl)((5-oxopyrrolidin-2-yl)methyl)carbamate (0.2 g, 0.33 mmol) and tert-butyl (S)-(2-(1-oxo-5-(4,4,5,5-tetramethyl-1,3,2-dioxaborolan-2-yl)isoindolin-2-yl)ethyl)((5-oxopyrrolidin-2-yl)methyl)carbamate (0.2 g, 0.4 mmol) in 1,4-dioxane/water mixture (10: 1, 13 mL) was added [1,1'-bis(diphenylphosphino)ferrocene]dichloropalladium(II) complex with dichloromethane (0.01 g, 0.02 mmol) and potassium carbonate (0.14 g, 1 mmol) and the mixture stirred at 110 °C for 3 hr. To the mixture was added water (5 mL), then the mixture extracted with EtOAc (2 x 10 mL). The combined organic phases were dried with anhydrous sodium sulfate, filtered and concentrated. The residue was purified by prep-TLC (silica gel, EtOAc : MeOH = 3:1) to afford tert-butyl ((3'-(2-(2-(2-((tert-butoxycarbonyl)(((S)-5-oxopyrrolidin-2-yl)methyl)amino)ethyl)-1-oxoisoindolin-5-yl)-3-chloropyridin-4-yl)-2'-chloro-3-fluoro-5-methoxy-[1,1'-biphenyl]-4-yl)methyl)(((S)-5-oxopyrrolidin-2-yl)methyl)carbamate (0.2 g, 61% yield) as a yellow solid. MS: m/z found 945 [M+H]⁺.

### (e) 5-(3-Chloro-4-(2-chloro-3'-fluoro-5'-methoxy-4'-(((((S)-5-oxopyrrolidin-2-yl)methyl)amino)methyl)-[1,1'-biphenyl]-3-yl)pyridin-2-yl)-2-(2-((((S)-5-oxopyrrolidin-2-yl)methyl)amino)ethyl)isoindolin-1-one

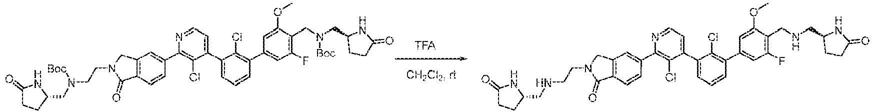

A mixture of tert-butyl ((3'-(2-(2-(2-((tert-butoxycarbonyl)(((S)-5-oxopyrrolidin-2-yl)methyl)amino)ethyl)-1-oxoisoindolin-5-yl)-3-chloropyridin-4-yl)-2'-chloro-3-fluoro-5-methoxy-[1,1'-biphenyl]-4-yl)methyl)(((S)-5-oxopyrrolidin-2-yl)methyl)carbamate (0.19 g, 0.2 mmol) in trifluoroacetic acid (1.5 mL) and dichloromethane (0.5 mL) was stirred at room temperature for 10 min. The mixture was concentrated and the residue purified by reverse phase HPLC to afford 5-(3-chloro-4-(2-chloro-3'-fluoro-5'-methoxy-4'-(((((S)-5-oxopyrrolidin-2-yl)methyl)amino)methyl)-[1,1'-biphenyl]-3-yl)pyridin-2-yl)-2-(2-((((S)-5-oxopyrrolidin-2-yl)methyl)amino)ethyl)isoindolin-1-one (32.5 mg, 20% yield) as a white solid (formic acid salt). MS: m/z found 745 [M+H]+, retention time = 2.55 min (Method A). ¹H NMR (400 MHz, Methanol-*d*₄): δ 8.70 (d, 1H), 8.34 (s, 1H), 7.95-7.91 (m, 2H), 7.85 (d, 1H), 7.61-7.56 (m, 2H), 7.50-7.46 (m, 2H), 7.02 (s, 1H), 6.97 (d, 1H), 4.71 (s, 2H), 4.23 (s, 2H), 4.00 (s, 3H), 3.97-3.92 (m, 4H), 3.26-3.24 (m, 2H), 3.05-3.04 (m, 4H), 2.42-2.33 (m, 6H), 1.89-1.87 (m, 2H).

### Example 423: 1-(6-(2-Chloro-3-(3-chloro-2-(isoindolin-5-yl)pyridin-4-yl)phenyl)-2-methoxypyridin-3-yl)-N-methylmethanamine (159)

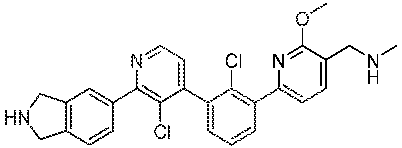

### (a) tert-Butyl 5-(3-chloro-4-(2-chloro-3-(5-formyl-6-methoxypyridin-2-yl)phenyl)pyridin-2-yl)isoindoline-2-carboxylate

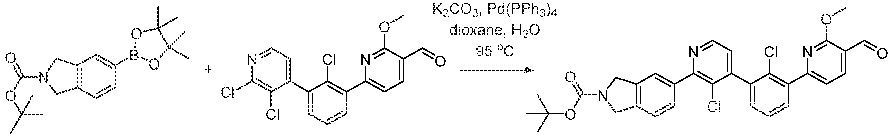

Following Suzuki Coupling Procedure A from tert-butyl 5-(4,4,5,5-tetramethyl-1,3,2-dioxaborolan-2-yl)isoindoline-2-carboxylate (150 mg, 0.43 mmol) and 6-(2-chloro-3-(2,3-dichloropyridin-4-yl)phenyl)-2-methoxynicotinaldehyde (171 mg, 0.43 mmol) afforded tert-butyl 5-(3-chloro-4-(2-chloro-3-(5-formyl-6-methoxypyridin-2-yl)phenyl)pyridin-2-yl)isoindoline-2-carboxylate in 71% yield. LCMS: m/z found 576.1 [M+H]⁺, retention time = 1.20 min (Method B).

### (b) tert-Butyl 5-(3-chloro-4-(2-chloro-3-(6-methoxy-5-((methylamino)methyl)pyridin-2-yl)phenyl)pyridin-2-yl)isoindoline-2-carboxylate

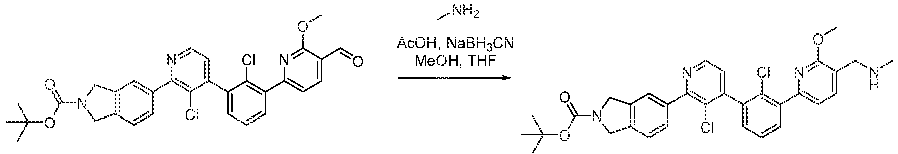

Following Reductive Amination Procedure B from tert-butyl 5-(3-chloro-4-(2-chloro-3-(5-formyl-6-methoxypyridin-2-yl)phenyl)pyridin-2-yl)isoindoline-2-carboxylate (40 mg, 0.07 mmol) and methanamine solution (33% wt in methanol, 0.14 mmol) gave tert-butyl 5-(3-chloro-4-(2-chloro-3-(6-methoxy-5-((methylamino)methyl)pyridin-2-yl)phenyl)pyridin-2-yl)isoindoline-2-carboxylate, which was used in the next step without further purification. LCMS: m/z found 591.2 [M+H]⁺, retention time = 0.92 min (Method B).

### (c) 1-(6-(2-Chloro-3-(3-chloro-2-(isoindolin-5-yl)pyridin-4-yl)phenyl)-2-methoxypyridin-3-yl)-N-methylmethanamine

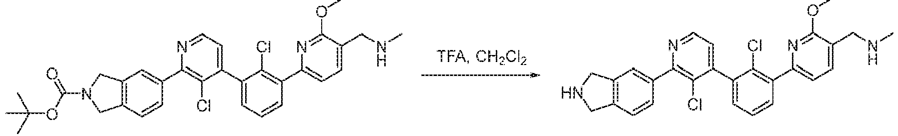

A solution of crude tert-butyl 5-(3-chloro-4-(2-chloro-3-(6-methoxy-5-((methylamino)methyl)pyridin-2-yl)phenyl)pyridin-2-yl)isoindoline-2-carboxylate (25 mg, 0.04 mmol) in dichloromethane / trifluoroacetic acid (2:1) was stirred for 30 min, concentrated and purified by preparative HPLC to give 1-(6-(2-chloro-3-(3-chloro-2-(isoindolin-5-yl)pyridin-4-yl)phenyl)-2-methoxypyridin-3-yl)-N-methylmethanamine (formic acid salt) in 62% yield over two steps. LCMS: m/z found 491.1 [M+H]⁺, retention time = 1.54 min (Method D). ¹H NMR (400 MHz, Methanol-*d*₄): δ 8.71 - 8.59 (m, 1H), 8.53 (s, 2H), 7.86 (d, 1H), 7.76 - 7.65 (m, 3H), 7.61 - 7.50 (m, 2H), 7.48 - 7.41 (m, 2H), 7.35 (d, 1H), 4.62 (s, 4H), 4.19 (s, 2H), 4.08 (s, 3H), 2.73 (s, 3H).

### Example 424: 2-(((6-(2-Chloro-3-(3-chloro-2-(isoindolin-5-yl)pyridin-4-yl)phenyl)-2-methoxypyridin-3-yl)methyl)amino)ethan-1-ol (160)

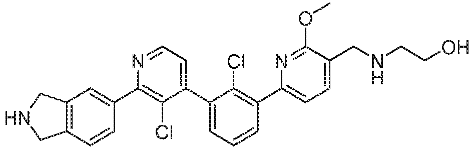

Following Reductive Amination Procedure B from tert-butyl 5-(3-chloro-4-(2-chloro-3-(5-formyl-6-methoxypyridin-2-yl)phenyl)pyridin-2-yl)isoindoline-2-carboxylate (40 mg, 0.07 mmol) and 2-aminoethanol (9 mg, 0.14 mmol) gave tert-butyl 5-(3-chloro-4-(2-chloro-3-(5-(((2-hydroxyethyl)amino)methyl)-6-methoxypyridin-2-yl)phenyl)pyridin-2-yl)isoindoline-2-carboxylate as crude, LCMS: m/z found 621.2 [M+H]⁺, which was deprotected and purified by General Boc Deprotection Procedure to afford 2-(((6-(2-chloro-3-(3-chloro-2-(isoindolin-5-yl)pyridin-4-yl)phenyl)-2-methoxypyridin-3-yl)methyl)amino)ethan-1-ol (formic acid salt) in 74% yield over two steps. LCMS: m/z found 521.1 [M+H]⁺, retention time = 1.52 min (Method D). ¹H NMR (400 MHz, Methanol-*d*₄): δ 8.65 (d, 1H), 8.52 (s, 2H), 7.85 (d, 1H), 7.75 - 7.67 (m, 3H), 7.62 - 7.52 (m, 2H), 7.48 - 7.40 (m, 2H), 7.33 (d, 1H), 4.64 (s, 4H), 4.19 (s, 2H), 4.07 (d, 3H), 3.81 (t, 2H), 3.08 (t, 2H).

### Example 425: (S)-1-(((6-(2-Chloro-3-(3-chloro-2-(isoindolin-5-yl)pyridin-4-yl)phenyl)-2-methoxypyridin-3-yl)methyl)amino)propan-2-ol (161)

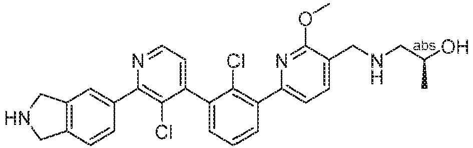

Following Reductive Amination Procedure B from tert-butyl 5-(3-chloro-4-(2-chloro-3-(5-formyl-6-methoxypyridin-2-yl)phenyl)pyridin-2-yl)isoindoline-2-carboxylate (40 mg, 0.07 mmol) and (2S)-1-aminopropan-2-ol (10 mg, 0.14 mmol) gave tert-butyl (S)-5-(3-chloro-4-(2-chloro-3-(5-(((2-hydroxypropyl)amino)methyl)-6-methoxypyridin-2-yl)phenyl)pyridin-2-yl)isoindoline-2-carboxylate as crude, LCMS: m/z found 635.2 [M+H]⁺, which was deprotected and purified by General Boc Deprotection Procedure to afford (S)-1-(((6-(2-chloro-3-(3-chloro-2-(isoindolin-5-yl)pyridin-4-yl)phenyl)-2-methoxypyridin-3-yl)methyl)amino)propan-2-ol (formic acid salt) in 78% yield over two steps. LCMS: m/z found 535.1 [M+H]⁺, retention time = 1.58 min (Method D). ¹H NMR (400 MHz, Methanol-*d*₄): δ 8.65 (d, 1H), 8.53 (s, 2H), 7.84 (d, 1H), 7.75 - 7.67 (m, 3H), 7.56 (q, 2H), 7.48 - 7.41 (m, 2H), 7.32 (d, 1H), 4.63 (s, 4H), 4.21 - 4.10 (m, 2H), 4.07 (s, 3H), 4.04 - 3.96 (m, 1H), 2.97 (dd, 1H), 2.85 - 2.74 (m, 1H), 1.22 (d, 3H).

### Example 426: 1-(4-(((6-(2-Chloro-3-(3-chloro-2-(isoindolin-5-yl)pyridin-4-yl)phenyl)-2-methoxypyridin-3-yl)methyl)amino)piperidin-1-yl)ethan-1-one (162)

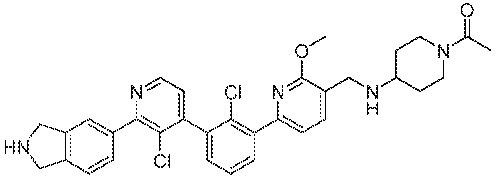

Following Reductive Amination Procedure B from tert-butyl 5-(3-chloro-4-(2-chloro-3-(5-formyl-6-methoxypyridin-2-yl)phenyl)pyridin-2-yl)isoindoline-2-carboxylate (40 mg, 0.07 mmol) and 1-(4-amino-1-piperidyl)ethanone (20 mg, 0.14 mmol) gave tert-butyl 5-(4-(3-(5-(((1-acetylpiperidin-4-yl)amino)methyl)-6-methoxypyridin-2-yl)-2-chlorophenyl)-3-chloropyridin-2-yl)isoindoline-2-carboxylate as crude, LCMS: m/z found 702.3 [M+H]⁺, which was deprotected and purified by General Boc Deprotection Procedure to afford 1-(4-(((6-(2-chloro-3-(3-chloro-2-(isoindolin-5-yl)pyridin-4-yl)phenyl)-2-methoxypyridin-3-yl)methyl)amino)piperidin-1-yl)ethan-1-one (formic acid salt) in 80% yield over two steps. LCMS: m/z found 602.2 [M+H]⁺, retention time = 1.58 min (Method D). ¹H NMR (400 MHz, Methanol-*d*₄): δ 8.65 (d, 1H), 8.50 (s, 2H), 7.87 - 7.79 (m, 1H), 7.71 (d, 3H), 7.56 (t, 2H), 7.45 (dd, 2H), 7.31 (d, 1H), 4.67 (s, 4H), 4.57 (d, 1H), 4.09 (s, 2H), 4.05 (d, 3H), 4.01 (d, 1H), 3.23 - 3.09 (m, 2H), 2.71 (t, 1H), 2.12 (d, 5H), 1.59 - 1.33 (m, 2H).

### Example 427: 1-(4-(((6-(2-Chloro-3-(3-chloro-2-(isoindolin-5-yl)pyridin-4-yl)phenyl)-2-methoxypyridin-3-yl)methyl)(methyl)amino)piperidin-1-yl)ethan-1-one (163)

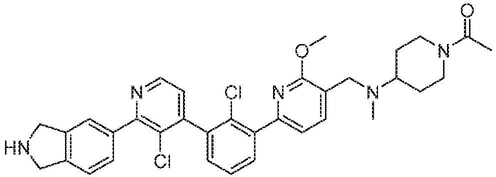

Following Reductive Amination Procedure B from tert-butyl 5-(3-chloro-4-(2-chloro-3-(5-formyl-6-methoxypyridin-2-yl)phenyl)pyridin-2-yl)isoindoline-2-carboxylate (40 mg, 0.07 mmol) and 1-[4-(methylamino)-1-piperidyl]ethanone (22 mg, 0.14 mmol) gave tert-butyl 5-(4-(3-(5-(((1-acetylpiperidin-4-yl)(methyl)amino)methyl)-6-methoxypyridin-2-yl)-2-chlorophenyl)-3-chloropyridin-2-yl)isoindoline-2-carboxylate as crude, LCMS: m/z found 716.3 [M+H]⁺, which was deprotected and purified by General Boc Deprotection Procedure to afford 1-(4-(((6-(2-chloro-3-(3-chloro-2-(isoindolin-5-yl)pyridin-4-yl)phenyl)-2-methoxypyridin-3-yl)methyl)(methyl)amino)piperidin-1-yl)ethan-1-one (formic acid salt) in 40% yield over two steps. LCMS: m/z found 616.2 [M+H]⁺, retention time = 1.59 min (Method D). ¹H NMR (400 MHz, Methanol-*d*₄): δ 8.65 (d, 1H), 8.51 (s, 2H), 7.80 (d, 1H), 7.72 (dt, 3H), 7.56 (dt, 2H), 7.46 (d, 1H), 7.41 (dd, 1H), 7.27 (d, 1H), 4.67 (s, 4H), 4.65 - 4.57 (m, 1H), 4.08 - 4.02 (m, 1H), 4.01 (s, 3H), 3.79 (s, 2H), 3.14 (t, 1H), 2.91 (t, 1H), 2.65 (t, 1H), 2.39 (s, 3H), 2.12 (s, 3H), 2.07 - 1.93 (m, 2H), 1.74 - 1.46 (m, 2H).

### Example 428: 2-((6-(2-Chloro-3-(3-chloro-2-(isoindolin-5-yl)pyridin-4-yl)phenyl)-2-methoxypyridin-3-yl)methyl)-2,6-diazaspiro[3.4]octan-7-one (230)

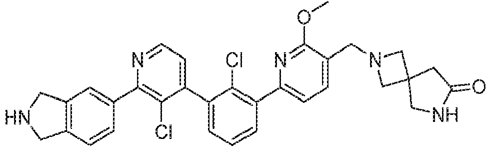

Following Reductive Amination Procedure B from tert-butyl 5-(3-chloro-4-(2-chloro-3-(5-formyl-6-methoxypyridin-2-yl)phenyl)pyridin-2-yl)isoindoline-2-carboxylate (50 mg, 0.09 mmol) and 2,6-diazaspiro[3.4]octan-7-one (22 mg, 0.17 mmol) gave tert-butyl 5-(3-chloro-4-(2-chloro-3-(6-methoxy-5-((7-oxo-2,6-diazaspiro[3.4]octan-2-yl)methyl)pyridin-2-yl)phenyl)pyridin-2-yl)isoindoline-2-carboxylate as crude, LCMS: m/z found 686.2 [M+H]⁺, which was deprotected and purified by General Boc Deprotection Procedure to afford 2-((6-(2-chloro-3-(3-chloro-2-(isoindolin-5-yl)pyridin-4-yl)phenyl)-2-methoxypyridin-3-yl)methyl)-2,6-diazaspiro[3.4]octan-7-one (formic acid salt) in 78% yield over two steps. LCMS: m/z found 586.2 [M+H]⁺, retention time = 1.52 min (Method D). ¹H NMR (400 MHz, Methanol-*d*₄): δ 8.65 (d, 1H), 8.47 (s, 2H), 7.76 - 7.67 (m, 4H), 7.59 - 7.51 (m, 2H), 7.47 (d, 1H), 7.41 (d, 1H), 7.25 (d, 1H), 4.68 (s, 4H), 4.00 (s, 3H), 3.79 (s, 2H), 3.60 (s, 2H), 3.55 - 3.44 (m, 4H), 2.60 (s, 2H).

### Example 429: (S)-5-((((6-(3-(2-(3-(((1-Acetylpiperidin-4-yl)amino)methyl)-1-methyl-1H-indazol-6-yl)-3-chloropyridin-4-yl)-2-chlorophenyl)-2-methoxypyridin-3-yl)methyl)amino)methyl)pyrrolidin-2-one (167)

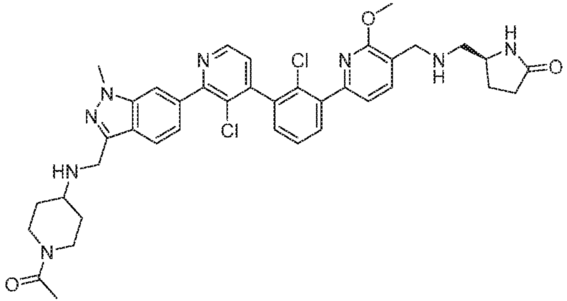

### (a) 6-Bromo-1-methyl-1H-indazole-3-carbaldehyde

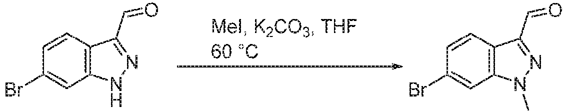

Iodomethane (0.20 mL, 3.20 mmol) and potassium carbonate (0.55 g, 4.00 mmol) were added to a solution of 6-bromo-1H-indazole-3-carbaldehyde (0.60 g, 2.67 mmol) in 6 mL THF. The mixture was stirred at room temperature overnight. The reaction mixture was transferred to a Biotage MW vessel, capped, and heated to 50 °C for 4 h, then 60 °C for an additional 4 h. The reaction mixture was diluted with 15 mL EtOAc and filtered through a pad of Celite. The filtrate was concentrated and the resulting crude product was purified by normal phase silica gel chromatography (0% to 30% EtOAc/hexanes) to provide 6-bromo-1-methyl-1H-indazole-3-carbaldehyde (0.37 g, 58% yield). ¹H NMR (400 MHz, Chloroform-d) δ 10.19 (s, 1H), 8.16 (d, 1H), 7.67 - 7.66 (m, 1H), 7.48 - 7.44 (m, 1H), 4.21 - 4.07 (s, 3H).

### (b) 1-Methyl-6-(4,4,5,5-tetramethyl-1,3,2-dioxaborolan-2-yl)-1H-indazole-3-carbaldehyde

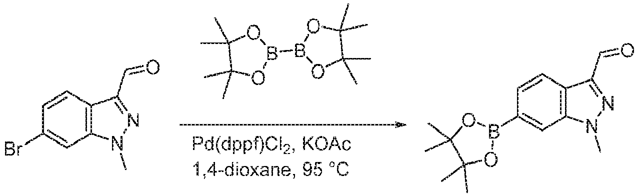

A mixture of 6-bromo-1-methyl-1H-indazole-3-carbaldehyde (151 mg, 0.63 mmol), bis(pinacolato)diborane (224 mg, 0.88 mmol), potassium acetate (173 mg, 1.77 mmol) and [1,1'-bis(diphenylphosphino)ferrocene]dichloropalladium(II), complex with dichloromethane (52 mg, 0.06 mmol) were weighed out into a Biotage microwave vial. Anhydrous 1,4-dioxane (4 mL) was added. The mixture was degassed with nitrogen for 2 minutes. The vessel was capped and irradiated to 95 °C for 75 min in a Biotage Initiator microwave. The mixture was diluted with 10 mL EtOAc and filtered through a pad of Celite. The Celite pad was washed with 20 mL EtOAc. The combined filtrate was concentrated under reduced pressure and the crude material purified by normal phase silica gel chromatography (0% to 28% EtOAc/hexanes) to provide 1-methyl-6-(4,4,5,5-tetramethyl-1,3,2-dioxaborolan-2-yl)-1H-indazole-3-carbaldehyde (157 mg, 87% yield). ¹H NMR (400 MHz, Chloroform-d) δ 10.23 (s, 1H), 8.29 (dd, 1H), 7.97 (d, 1H), 7.78 (dd, 1H), 4.22 (s, 3H), 1.39 (s, 12H).

### (c) tert-Butyl (S)-((6-(2-chloro-3-(3-chloro-2-(3-formyl-1-methyl-1H-indazol-6-yl)pyridin-4-yl)phenyl)-2-methoxypyridin-3-yl)methyl)((5-oxopyrrolidin-2-yl)methyl)carbamate

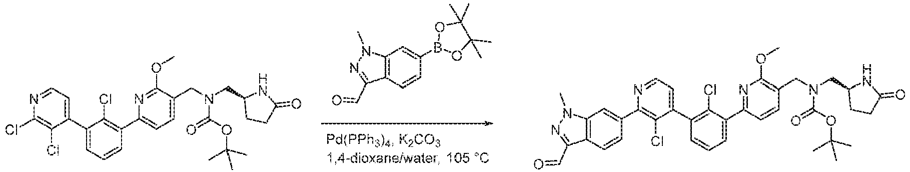

To a solution of tert-butyl (S)-((6-(2-chloro-3-(2,3-dichloropyridin-4-yl)phenyl)-2-methoxypyridin-3-yl)methyl)((5-oxopyrrolidin-2-yl)methyl)carbamate (192 mg, 0.32 mmol), 1-methyl-6-(4,4,5,5-tetramethyl-1,3,2-dioxaborolan-2-yl)-1H-indazole-3-carbaldehyde (102 mg, 0.36 mmol) and potassium carbonate (134 mg, 0.97 mmol) in degassed 1,4-dioxane/water (6:1, 3.7 mL) was added tetrakis(triphenylphosphine)palladium(0) (37 mg, 0.03 mmol). The mixture was irradiated to 105 °C for 25 min in a Biotage Initiator microwave. Water (10 mL) was added and the mixture was extracted into EtOAc (3 x 12 mL). The combined organic layers were dried over anhydrous sodium sulfate, concentrated, and purified by normal phase silica gel chromatography (0% to 8% MeOH/DCM) to provide tert-butyl (S)-((6-(2-chloro-3-(3-chloro-2-(3-formyl-1-methyl-1H-indazol-6-yl)pyridin-4-yl)phenyl)-2-methoxypyridin-3-yl)methyl)((5-oxopyrrolidin-2-yl)methyl)carbamate (203 mg, 88% yield). MS: m/z found 715.2 [M+H]⁺.

### (d) (S)-5-((((6-(3-(2-(3-(((1-Acetylpiperidin-4-yl)amino)methyl)-1-methyl-1H-indazol-6-yl)-3-chloropyridin-4-yl)-2-chlorophenyl)-2-methoxypyridin-3-yl)methyl)amino)methyl)pyrrolidin-2-one

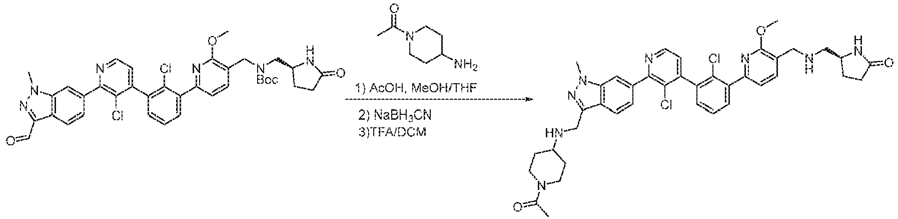

(S)-5-((((6-(3-(2-(3-(((1-Acetylpiperidin-4-yl)amino)methyl)-1-methyl-1H-indazol-6-yl)-3-chloropyridin-4-yl)-2-chlorophenyl)-2-methoxypyridin-3-yl)methyl)amino)methyl)pyrrolidin-2-one was prepared as the formic acid salt, following Reductive Amination Procedure B from tert-butyl (S)-((6-(2-chloro-3-(3-chloro-2-(3-formyl-1-methyl-1H-indazol-6-yl)pyridin-4-yl)phenyl)-2-methoxypyridin-3-yl)methyl)((5-oxopyrrolidin-2-yl)methyl)carbamate (32 mg, 0.04 mmol) and 1-(4-aminopiperidin-1-yl)ethan-1-one (13 mg, 0.09 mmol), followed by General Boc Deprotection Procedure. 38% yield. LCMS: m/z found 742.6 [M+H]⁺, retention time = 2.72 min (Method A). ¹H NMR (400 MHz, Methanol-*d*₄): δ 8.67 (d, 1H), 8.51 (s, 1H), 7.97 (dd, 1H), 7.87 (t, 1H), 7.79 - 7.68 (m, 2H), 7.56 (t, 1H), 7.53 - 7.46 (m, 2H), 7.43 (dd, 1H), 7.27 (d, 1H), 4.56 (d, 1H), 4.44 (s, 2H), 4.13 (s, 3H), 4.03 (s, 3H), 3.98 (s, 1H), 3.85 (dd, 3H), 3.17 (s, 2H), 2.80 - 2.65 (m, 3H), 2.39 - 2.30 (m, 2H), 2.34 - 2.22 (m, 1H), 2.18 (d, 2H), 2.12 (s, 3H), 1.89 - 1.76 (m, 1H), 1.58 - 1.37 (m, 2H).

### Example 430: (S)-5-((((6-(3-(2-(3-(((1-Acetylpiperidin-4-yl)(methyl)amino)methyl)-1-methyl-1H-indazol-6-yl)-3-chloropyridin-4-yl)-2-chlorophenyl)-2-methoxypyridin-3-yl)methyl)amino)methyl)pyrrolidin-2-one (168)

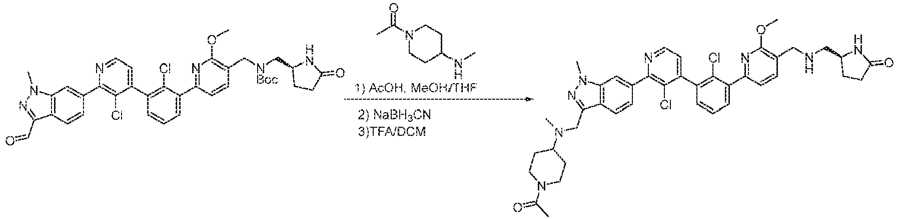

(S)-5-((((6-(3-(2-(3-(((1-acetylpiperidin-4-yl)(methyl)amino)methyl)-1-methyl-1H-indazol-6-yl)-3-chloropyridin-4-yl)-2-chlorophenyl)-2-methoxypyridin-3-yl)methyl)amino)methyl)pyrrolidin-2-one was prepared as the formic acid salt, following Reductive Amination Procedure B from tert-butyl (S)-((6-(2-chloro-3-(3-chloro-2-(3-formyl-1-methyl-1H-indazol-6-yl)pyridin-4-yl)phenyl)-2-methoxypyridin-3-yl)methyl)((5-oxopyrrolidin-2-yl)methyl)carbamate (38 mg, 0.05 mmol) and 1-(4-(methylamino)piperidin-1-yl)ethan-1-one (10 mg, 0.03 mmol), followed by General Boc Deprotection Procedure. 46% yield. LCMS: m/z found 755.1 [M+H]⁺, retention time = 2.77 min, (Method A). ¹H NMR (400 MHz, Methanol-*d*₄): δ 8.67 (d, 1H), 8.44 (s, 1H), 7.98 (dd, 1H), 7.86 (t, 1H), 7.79 (d, 1H), 7.72 (dd, 1H), 7.57 (t, 1H), 7.52 - 7.41 (m, 3H), 7.29 (d, 1H), 4.64 (d, 1H), 4.27 (s, 2H), 4.12 (s, 3H), 4.04 (s, 4H), 3.97 - 3.85 (m, 3H), 3.35 (s, 2H), 3.21 - 2.98 (m, 1H), 2.90 - 2.76 (m, 2H), 2.50 (s, 3H), 2.41 - 2.26 (m, 3H), 2.13 (s, 5H), 1.92 - 1.46 (m, 3H).

### Example 431: (S)-5-((((6-(2-Chloro-3-(3-chloro-2-(1-methyl-3-(((((S)-5-oxopyrrolidin-2-yl)methyl)amino)methyl)-1H-indazol-6-yl)pyridin-4-yl)phenyl)-2-methoxypyridin-3-yl)methyl)amino)methyl)pyrrolidin-2-one (183)

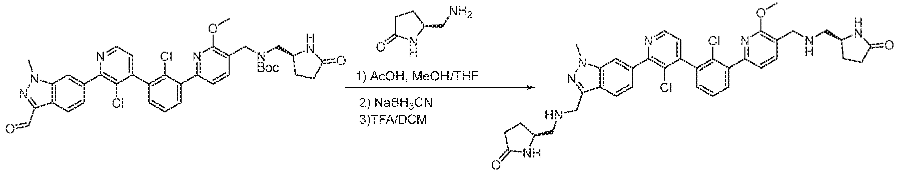

(S)-5-((((6-(2-Chloro-3-(3-chloro-2-(1-methyl-3-(((((S)-5-oxopyrrolidin-2-yl)methyl)amino)methyl)-1H-indazol-6-yl)pyridin-4-yl)phenyl)-2-methoxypyridin-3-yl)methyl)amino)methyl)pyrrolidin-2-one was prepared as the formic acid salt, following Reductive Amination Procedure B from tert-butyl (S)-((6-(2-chloro-3-(3-chloro-2-(3-formyl-1-methyl-1H-indazol-6-yl)pyridin-4-yl)phenyl)-2-methoxypyridin-3-yl)methyl)((5-oxopyrrolidin-2-yl)methyl)carbamate (34 mg, 0.05 mmol) and (S)-5-(aminomethyl)pyrrolidin-2-one (10 mg, 0.03 mmol), followed by General Boc Deprotection Procedure. 56% yield. LCMS: m/z found 714.8 [M+H]⁺, retention time = 2.62 min, (Method A). ¹H NMR (400 MHz, Methanol-*d*₄): δ 8.67 (d, 1H), 7.97 (dd, 1H), 7.85 (t, 1H), 7.79 (d, 1H), 7.72 (dd, 1H), 7.57 (t, 1H), 7.52 - 7.41 (m, 3H), 7.29 (d, 1H), 4.40 - 4.28 (m, 2H), 4.12 (s, 3H), 4.04 (s, 3H), 4.02 - 3.85 (m, 4H), 2.99 - 2.76 (m, 4H), 2.43 - 2.23 (m, 6H), 1.89 - 1.77 (m, 2H).

### Example 432: (S)-5-((((6-(2-Chloro-3-(3-chloro-2-(1-methyl-3-(((((S)-oxetan-2-yl)methyl)amino)methyl)-1H-indazol-6-yl)pyridin-4-yl)phenyl)-2-methoxypyridin-3-yl)methyl)amino)methyl)pyrrolidin-2-one (235)

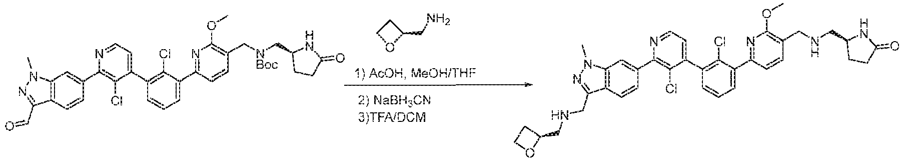

(S)-5-((((6-(2-Chloro-3-(3-chloro-2-(1-methyl-3-(((((S)-oxetan-2-yl)methyl)amino)methyl)-1H-indazol-6-yl)pyridin-4-yl)phenyl)-2-methoxypyridin-3-yl)methyl)amino)methyl)pyrrolidin-2-one was prepared as the formic acid salt, following Reductive Amination Procedure B from tert-butyl (S)-((6-(2-chloro-3-(3-chloro-2-(3-formyl-1-methyl-1H-indazol-6-yl)pyridin-4-yl)phenyl)-2-methoxypyridin-3-yl)methyl)((5-oxopyrrolidin-2-yl)methyl)carbamate (37 mg, 0.05 mmol) and (S)-oxetan-2-ylmethanamine (10 mg, 0.03 mmol), followed by General Boc Deprotection Procedure. 50% yield. LCMS: m/z found 686.4 [M+H]⁺, retention time = 2.77 min, (Method A). ¹H NMR (400 MHz, Methanol-*d*₄): δ 8.67 (d, 1H), 8.48 (s, 1H), 7.96 (dd, 1H), 7.93 - 7.86 (m, 1H), 7.77 (d, 1H), 7.72 (dd, 1H), 7.56 (t, 1H), 7.55 - 7.47 (m, 2H), 7.44 (dd, 1H), 7.28 (d, 1H), 5.12 - 5.04 (m, 1H), 4.75 - 4.68 (m, 1H), 4.63 - 4.59 (m, 1H), 4.51 (s, 2H), 4.14 (s, 3H), 4.03 (s, 3H), 3.97 - 3.84 (m, 3H), 3.39 (dd, 1H), 3.32 - 3.16 (m, 1H), 2.86 - 2.71 (m, 3H), 2.56 - 2.48 (m, 1H), 2.42 - 2.23 (m, 3H), 1.90 - 1.77 (m, 1H).

### Example 433: (S)-5-((((6-(2-Chloro-3-(3-chloro-2-(3-(((2-hydroxyethyl)(methyl)amino)methyl)-1-methyl-1H-indazol-6-yl)pyridin-4-yl)phenyl)-2-methoxypyridin-3-yl)methyl)amino)methyl)pyrrolidin-2-one (247)

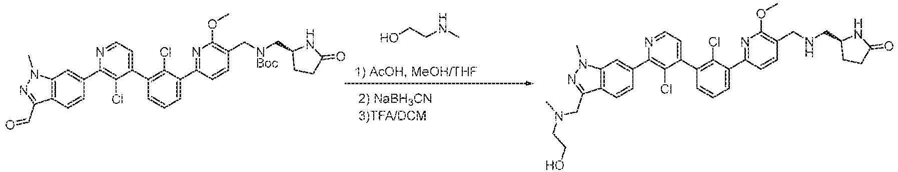

(S)-5-((((6-(2-Chloro-3-(3-chloro-2-(3-(((2-hydroxyethyl)(methyl)amino)methyl)-1-methyl-1H-indazol-6-yl)pyridin-4-yl)phenyl)-2-methoxypyridin-3-yl)methyl)amino)methyl)pyrrolidin-2-one was prepared as the formic acid salt, following Reductive Amination Procedure B from tert-butyl (S)-((6-(2-chloro-3-(3-chloro-2-(3-formyl-1-methyl-1H-indazol-6-yl)pyridin-4-yl)phenyl)-2-methoxypyridin-3-yl)methyl)((5-oxopyrrolidin-2-yl)methyl)carbamate (30 mg, 0.04 mmol) and 2-(methylamino)ethan-1-ol (6 mg, 0.08 mmol), followed by General Boc Deprotection Procedure. 41% yield. LCMS: m/z found 674.0 [M+H]⁺, retention time = 2.64 min, (Method A). ¹H NMR (400 MHz, Methanol-*d*₄): δ 8.68 (d, 1H), 8.48 (s, 1H), 8.00 (d, 1H), 7.90 (s, 1H), 7.77 (d, 1H), 7.72 (dd, 1H), 7.61 - 7.46 (m, 3H), 7.44 (dd, 1H), 7.28 (d, 1H), 4.46 (s, 2H), 4.15 (s, 2H), 4.03 (s, 3H), 3.98 - 3.82 (m, 5H), 3.08 (t, 2H), 2.85 - 2.72 (m, 2H), 2.71 (s, 3H), 2.40 - 2.32 (m, 1H), 2.36 - 2.23 (m, 2H), 1.88 - 1.77 (m, 1H).

### Example 434: 1-(4-(((6-(3-(2-(3-(((1-Acetylpiperidin-4-yl)amino)methyl)-1-methyl-1H-indazol-6-yl)-3-chloropyridin-4-yl)-2-chlorophenyl)-2-methoxypyridin-3-yl)methyl)amino)piperidin-1-yl)ethan-1-one (263)

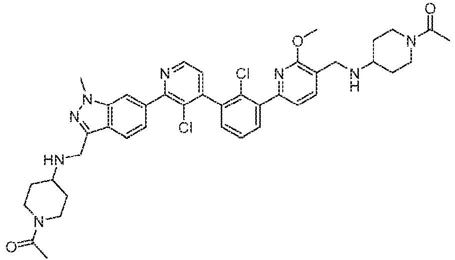

### (a) 6-(3-Chloro-4-(2-chloro-3-(5-formyl-6-methoxypyridin-2-yl)phenyl)pyridin-2-yl)-1-methyl-1H-indazole-3-carbaldehyde

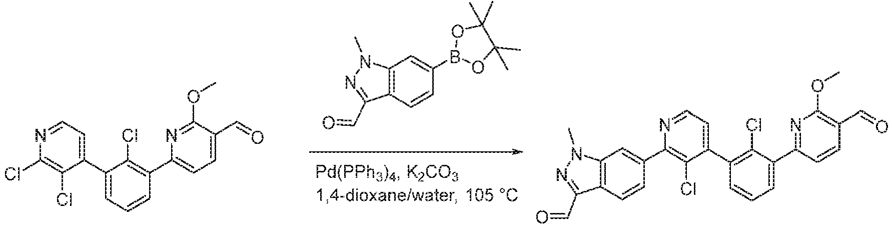

To a mixture of 1-methyl-6-(4,4,5,5-tetramethyl-1,3,2-dioxaborolan-2-yl)-1H-indazole-3-carbaldehyde (34 mg, 0.12 mmol), 6-(2-chloro-3-(2,3-dichloropyridin-4-yl)phenyl)-2-methoxynicotinaldehyde (46 mg, 0.12 mmol) and potassium carbonate (49 mg, 0.35 mmol) in degassed 1,4-dioxane/water (6:1, 1.15 mL) was added tetrakis(triphenylphosphine)palladium(0) (14 mg, 0.01 mmol). The mixture was irradiated to 105 °C for 25 min in a Biotage Initiator microwave. Water (7 mL) was added and the mixture was extracted into EtOAc (3 x 15 mL). The combined organic layers were dried over anhydrous sodium sulfate, concentrated, and purified by normal phase silica gel chromatography (15% to 80% EtOAc/hexanes) to provide 6-(3-chloro-4-(2-chloro-3-(5-formyl-6-methoxypyridin-2-yl)phenyl)pyridin-2-yl)-1-methyl-1H-indazole-3-carbaldehyde (41 mg, 67% yield). ¹H NMR (400 MHz, Chloroform-d) δ 10.43 (d, 1H), 10.26 (s, 1H), 8.71 (d, 1H), 8.39 (dd, 1H), 8.21 (d, 1H), 7.89 (t, 1H), 7.81 - 7.71 (m, 2H), 7.53 (t, 1H), 7.46 - 7.36 (m, 2H), 7.33 (d, 1H), 4.25 (s, 3H), 4.14 (s, 3H).

### (b) 1-(4-(((6-(3-(2-(3-(((1-Acetylpiperidin-4-yl)amino)methyl)-1-methyl-1H-indazol-6-yl)-3-chloropyridin-4-yl)-2-chlorophenyl)-2-methoxypyridin-3-yl)methyl)amino)piperidin-1-yl)ethan-1-one

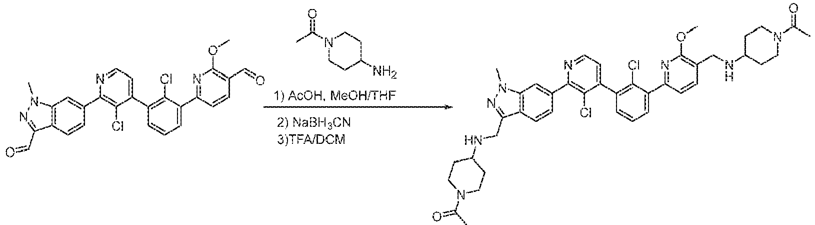

1-(4-(((6-(3-(2-(3-(((1-Acetylpiperidin-4-yl)amino)methyl)-1-methyl-1H-indazol-6-yl)-3-chloropyridin-4-yl)-2-chlorophenyl)-2-methoxypyridin-3-yl)methyl)amino)piperidin-1-yl)ethan-1-one was prepared as the formic acid salt, following Reductive Amination Procedure A from 6-(3-chloro-4-(2-chloro-3-(5-formyl-6-methoxypyridin-2-yl)phenyl)pyridin-2-yl)-1-methyl-1H-indazole-3-carbaldehyde (38 mg, 0.07 mmol) and 1-(4-aminopiperidin-1-yl)ethan-1-one (41 mg, 0.29 mmol). 50% yield. LCMS: m/z found 769.3 [M+H]⁺, retention time = 2.81 min, (Method A). ¹H NMR (400 MHz, DMSO-*d*₆): δ 9.55 (d, 1H), 9.02 (s, 1H), 8.79 (d, 1H), 8.70 - 8.63 (m, 2H), 8.54 (dd, 1H), 8.45 - 8.29 (m, 3H), 8.20 (dd, 1H), 8.09 (d, 1H), 4.98 - 4.88 (m, 4H), 4.84 (s, 3H), 4.73 (s, 3H), 4.55 (s, 4H), 3.92 - 3.80 (m, 1H), 3.56 - 3.43 (m, 4H), 2.79 (d, 6H), 2.69 (d, 3H), 2.62 (s, 1H), 2.07 (s, 3H), 2.01 - 1.91 (m, 2H).

### Example 435: (S)-2-((6-(3-Chloro-4-(2-chloro-3-(6-methoxy-5-((((5-oxopyrrolidin-2-yl)methyl)amino)methyl)pyridin-2-yl)phenyl)pyridin-2-yl)-1-methyl-1H-indazol-3-yl)methyl)-2,6-diazaspiro[3.4]octan-7-one (288)

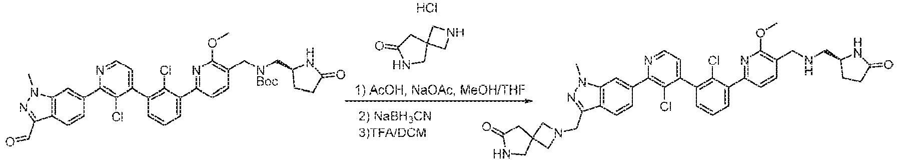

(S)-2-((6-(3-Chloro-4-(2-chloro-3-(6-methoxy-5-((((5-oxopyrrolidin-2-yl)methyl)amino)methyl)pyridin-2-yl)phenyl)pyridin-2-yl)-1-methyl-1H-indazol-3-yl)methyl)-2,6-diazaspiro[3.4]octan-7-one was prepared as the formic acid salt, following Reductive Amination Procedure B from tert-butyl (S)-((6-(2-chloro-3-(3-chloro-2-(3-formyl-1-methyl-1H-indazol-6-yl)pyridin-4-yl)phenyl)-2-methoxypyridin-3-yl)methyl)((5-oxopyrrolidin-2-yl)methyl)carbamate (33 mg, 0.05 mmol) and 2,6-diazaspiro[3.4]octan-7-one hydrochloride (15 mg, 0.09 mmol), followed by General Boc Deprotection Procedure. 41% yield. LCMS: m/z found 725.0 [M+H]⁺, retention time = 2.64 min, (Method A). ¹H NMR (400 MHz, Methanol-*d*₄): δ 8.67 (dd, 1H), 7.95 (dd, 1H), 7.85 (dd, 1H), 7.79 (dd, 1H), 7.72 (dd, 1H), 7.57 (t, 1H), 7.52 - 7.45 (m, 2H), 7.45 - 7.42 (m, 1H), 7.29 (dd, 1H), 4.25 (s, 2H), 4.11 (s, 2H), 4.04 (s, 3H), 3.95 (s, 3H), 3.93 - 3.85 (m, 1H), 3.68 (s, 4H), 3.59 (d, 2H), 2.89 - 2.76 (m, 2H), 2.60 (d, 2H), 2.40 - 2.24 (m, 3H), 1.89 - 1.78 (m, 1H).

### Example 436: (S)-5-((((6-(3-(2-(3-((6-Acetyl-2,6-diazaspiro[3.3]heptan-2-yl)methyl)-1-methyl-1H-indazol-6-yl)-3-chloropyridin-4-yl)-2-chlorophenyl)-2-methoxypyridin-3-yl)methyl)amino)methyl)pyrrolidin-2-one (308)

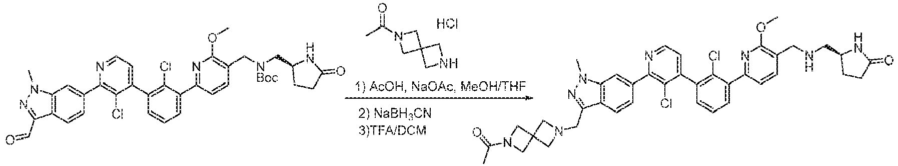

(S)-5-((((6-(3-(2-(3-((6-Acetyl-2,6-diazaspiro[3.3]heptan-2-yl)methyl)-1-methyl-1H-indazol-6-yl)-3-chloropyridin-4-yl)-2-chlorophenyl)-2-methoxypyridin-3-yl)methyl)amino)methyl)pyrrolidin-2-one was prepared as the formic acid salt, following Reductive Amination Procedure B from tert-butyl (S)-((6-(2-chloro-3-(3-chloro-2-(3-formyl-1-methyl-1H-indazol-6-yl)pyridin-4-yl)phenyl)-2-methoxypyridin-3-yl)methyl)((5-oxopyrrolidin-2-yl)methyl)carbamate (33 mg, 0.05 mmol) and 1-(2,6-diazaspiro[3.3]heptan-2-yl)ethan-1-one (8 mg, 0.09 mmol), followed by General Boc Deprotection Procedure. 30% yield. LCMS: m/z found 740.6 [M+H]⁺, retention time = 2.70 min, (Method A). ¹H NMR (400 MHz, Methanol-*d*₄): δ 8.67 (d, 1H), 7.94 (dd, 1H), 7.84 (t, 1H), 7.77 (d, 1H), 7.72 (dd, 1H), 7.56 (t, 1H), 7.52 - 7.40 (m, 3H), 7.28 (d, 1H), 4.30 (s, 2H), 4.15 (d, 2H), 4.11 (s, 3H), 4.04 (d, 5H), 3.91 (d, 2H), 3.87 (s, 2H), 3.71 (s, 4H), 2.82 - 2.74 (m, 2H), 2.40 - 2.25 (m, 3H), 1.83 (s, 3H).

### Example 437: (S)-1-((6-(3-Chloro-4-(2-chloro-3-(6-methoxy-5-(((((S)-5-oxopyrrolidin-2-yl)methyl)amino)methyl)pyridin-2-yl)phenyl)pyridin-2-yl)-1-methyl-1H-indazol-3-yl)methyl)pyrrolidine-3-carboxylic acid (309)

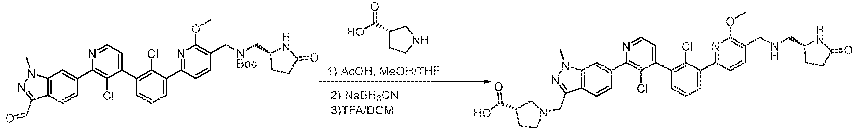

(S)-1-((6-(3-Chloro-4-(2-chloro-3-(6-methoxy-5-(((((S)-5-oxopyrrolidin-2-yl)methyl)amino)methyl)pyridin-2-yl)phenyl)pyridin-2-yl)-1-methyl-1H-indazol-3-yl)methyl)pyrrolidine-3-carboxylic acid was prepared as the formic acid salt, following Reductive Amination Procedure B from tert-butyl (S)-((6-(2-chloro-3-(3-chloro-2-(3-formyl-1-methyl-1H-indazol-6-yl)pyridin-4-yl)phenyl)-2-methoxypyridin-3-yl)methyl)((5-oxopyrrolidin-2-yl)methyl)carbamate (35 mg, 0.05 mmol) and (S)-pyrrolidine-3-carboxylic acid (11 mg, 0.10 mmol), followed by General Boc Deprotection Procedure. 35% yield. LCMS: m/z found 714.0 [M+H]⁺, retention time = 2.75 min, (Method A). ¹H NMR (400 MHz, Methanol-*d*₄): δ 8.68 (d, 1H), 8.00 (d, 1H), 7.92 (s, 1H), 7.80 - 7.68 (m, 2H), 7.60 - 7.52 (m, 2H), 7.49 (d, 1H), 7.44 (dd, 1H), 7.27 (d, 1H), 4.82 - 4.69 (m, 2H), 4.17 (s, 3H), 4.03 (s, 3H), 3.88 (dd, 3H), 3.68 - 3.59 (m, 1H), 3.54 (d, 1H), 3.44 (s, 2H), 3.10 (d, 1H), 2.83 - 2.69 (m, 2H), 2.39 - 2.22 (m, 5H), 1.88 - 1.76 (m, 1H).

### Example 438: 1-(6-(2-Chloro-3-(3-chloro-2-(1-methyl-3-((methylamino)methyl)-1H-indol-6-yl)pyridin-4-yl)phenyl)-2-methoxypyridin-3-yl)-N-methylmethanamine (276)

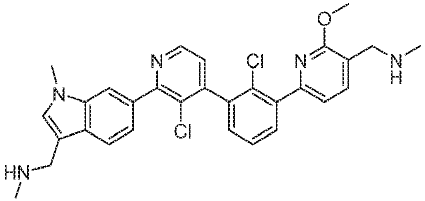

### (a) 6-(3-Chloro-4-(2-chloro-3-(5-formyl-6-methoxypyridin-2-yl)phenyl)pyridin-2-yl)-1-methyl-1H-indole-3-carbaldehyde

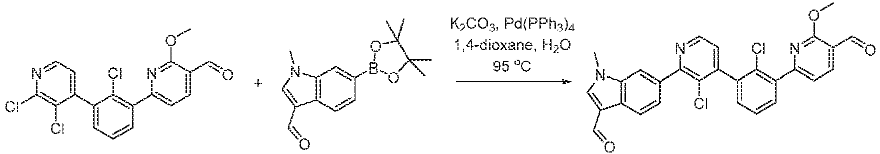

Following Suzuki Coupling Procedure A from 6-(2-chloro-3-(2,3-dichloropyridin-4-yl)phenyl)-2-methoxynicotinaldehyde (65 mg, 0.17 mmol) and 1-methyl-6-(4,4,5,5-tetramethyl-1,3,2-dioxaborolan-2-yl)-1H-indole-3-carbaldehyde (47 mg, 0.17 mmol) provided 6-[3-chloro-4-[2-chloro-3-(5-formyl-6-methoxy-2-pyridyl)phenyl]-2-pyridyl]-1-methyl-indole-3-carbaldehyde (75 mg, 88%). LCMS: m/z found 516.1 [M+H]⁺, retention time = 1.02 min (Method B).

### (b) 1-(6-(2-Chloro-3-(3-chloro-2-(1-methyl-3-((methylamino)methyl)-1H-indol-6-yl)pyridin-4-yl)phenyl)-2-methoxypyridin-3-yl)-N-methylmethanamine

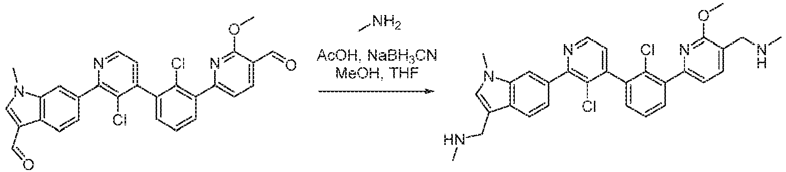

Following Reductive Amination Procedure A from 6-[3-chloro-4-[2-chloro-3-(5-formyl-6-methoxy-2-pyridyl)phenyl]-2-pyridyl]-1-methyl-indole-3-carbaldehyde (30 mg, 0.06 mmol) and methanamine solution (33% wt in methanol, 0.23 mmol) gave 1-(6-(2-chloro-3-(3-chloro-2-(1-methyl-3-((methylamino)methyl)-1H-indol-6-yl)pyridin-4-yl)phenyl)-2-methoxypyridin-3-yl)-N-methylmethanamine (formic acid salt) in 78% yield. LCMS: m/z found 546.2 [M+H]⁺, retention time = 1.72 min (Method D). ¹H NMR (400 MHz, Methanol-*d*₄): δ 8.65 (dd, 1H), 8.54 (s, 2H), 7.87 - 7.80 (m, 2H), 7.79 (s, 1H), 7.72 (d, 1H), 7.58 (dd, 1H), 7.55 - 7.48 (m, 2H), 7.48 - 7.41 (m, 2H), 7.34 (d, 1H), 4.41 (s, 2H), 4.11 (s, 2H), 4.07 (s, 3H), 3.91 (s, 3H), 2.73 (s, 3H), 2.67 (s, 3H).

### Example 439: 1-(4-(((6-(3-(2-(3-(((1-Acetylpiperidin-4-yl)amino)methyl)-1-methyl-1H-indol-6-yl)-3-chloropyridin-4-yl)-2-chlorophenyl)-2-methoxypyridin-3-yl)methyl)amino)piperidin-1-yl)ethan-1-one (277)

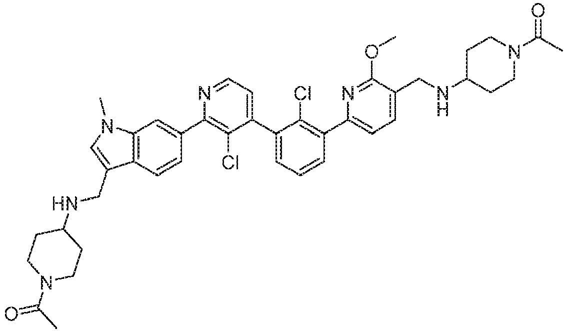

Following Reductive Amination Procedure A from 6-[3-chloro-4-[2-chloro-3-(5-formyl-6-methoxy-2-pyridyl)phenyl]-2-pyridyl]-1-methyl-indole-3-carbaldehyde (30 mg, 0.06 mmol) and 1-(4-amino-1-piperidyl)ethanone (33 mg, 0.23 mmol) gave 1-(4-(((6-(3-(2-(3-(((1-acetylpiperidin-4-yl)amino)methyl)-1-methyl-1H-indol-6-yl)-3-chloropyridin-4-yl)-2-chlorophenyl)-2-methoxypyridin-3-yl)methyl)amino)piperidin-1-yl)ethan-1-one (formic acid salt) in 92% yield. LCMS: m/z found 768.3 [M+H]⁺, retention time = 1.76 min (Method D). ¹H NMR (400 MHz, Methanol-*d*₄): δ 8.64 (d, 1H), 8.53 (s, 2H), 7.86 - 7.75 (m, 3H), 7.72 (dd, 1H), 7.56 (t, 1H), 7.52 (s, 1H), 7.50 (dd, 1H), 7.46 - 7.41 (m, 2H), 7.29 (d, 1H), 4.65 (d, 1H), 4.53 (d, 1H), 4.43 (s, 2H), 4.11 - 4.02 (m, 4H), 4.01 - 3.93 (m, 3H), 3.91 (s, 3H), 3.45 - 3.34 (m, 1H), 3.18 (q, 2H), 3.02 - 2.89 (m, 1H), 2.70 (q, 2H), 2.24 (t, 2H), 2.16 - 1.98 (m, 8H), 1.67 - 1.28 (m, 4H).

### Example 440: (S)-5-((((6-(2-Chloro-3-(3-chloro-2-((S)-5-((((S)-5-oxopyrrolidin-2-yl)methyl)amino)-5,6,7,8-tetrahydronaphthalen-2-yl)pyridin-4-yl)phenyl)-2-methoxypyridin-3-yl)methyl)amino)methyl)pyrrolidin-2-one (184)

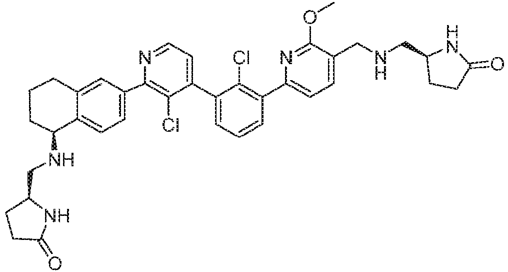

### (a) (S)-5-((((S)-6-Bromo-1,2,3,4-tetrahydronaphthalen-1-yl)amino)methyl)pyrrolidin-2-one

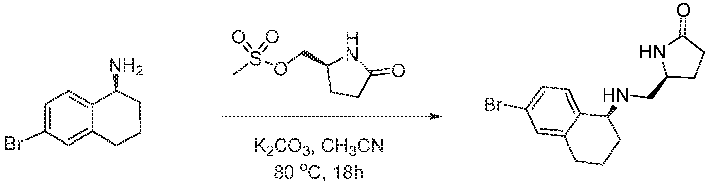

(S)-6-Bromo-1,2,3,4-tetrahydronaphthalen-1-amine (75 mg, 0.33 mmol), (S)-(5-oxopyrrolidin-2-yl)methyl methanesulfonate (77 mg, 0.40 mmol), and potassium carbonate (137 mg, 1.00 mmol) were suspended in 3 mL acetonitrile. The reaction mixture was then heated at 80 °C for 18 hours. The reaction was cooled to room temperature, diluted with water (6 mL), and extracted with EtOAc (3 x 5 mL). The combined organics were washed with water (5 mL), brine (5 mL), dried over sodium sulfate, filtered, and concentrated under reduced pressure to afford (S)-5-((((S)-6-bromo-1,2,3,4-tetrahydronaphthalen-1-yl)amino)methyl)pyrrolidin-2-one (104 mg, crude) as a light brown oil. MS: *m*/*z* found 323, 325 [M+H]⁺. Material was used for the next step without further purification.

### (b) (S)-5-((((S)-6-(4,4,5,5-Tetramethyl-1,3,2-dioxaborolan-2-yl)-1,2,3,4-tetrahydronaphthalen-1-yl)amino)methyl)pyrrolidin-2-one

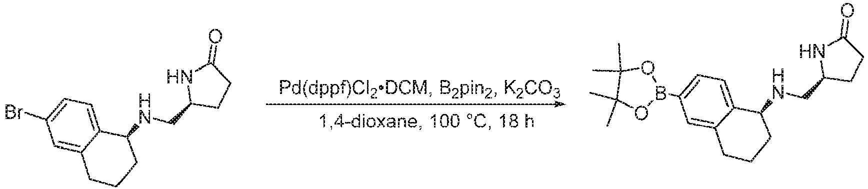

(S)-5-((((S)-6-bromo-1,2,3,4-tetrahydronaphthalen-1-yl)amino)methyl)pyrrolidin-2-one (50 mg, 0.15 mmol), bis(pinacolato)diborane (55 mg, 0.22 mmol), [1,1'-bis(diphenylphosphino)ferrocene]dichloropalladium(II) complex with dichloromethane (13 mg, 0.02 mmol), and potassium carbonate (60 mg, 0.43 mmol) were suspended in 2 mL 1,4-dioxane in a sealed tube. Nitrogen gas was bubbled through the reaction solution for 2 minutes, then the vessel was sealed and heated at 100 °C for 18 hours. Reaction was cooled to room temperature and the mixture was filtered through celite The filtrate was concentrated under reduced pressure and the crude oil was purified by reverse phase HPLC to afford (S)-5-((((S)-6-(4,4,5,5-tetramethyl-1,3,2-dioxaborolan-2-yl)-1,2,3,4-tetrahydronaphthalen-1-yl)amino)methyl)pyrrolidin-2-one (12 mg, 21 % yield) as a white solid. MS: *m*/*z* found 289 [M+H]⁺, (boronic acid fragment).

### (c) (S)-5-((((6-(2-Chloro-3-(3-chloro-2-((S)-5-((((S)-5-oxopyrrolidin-2-yl)methyl)amino)-5,6,7,8-tetrahydronaphthalen-2-yl)pyridin-4-yl)phenyl)-2-methoxypyridin-3-yl)methyl)amino)methyl)pyrrolidin-2-one

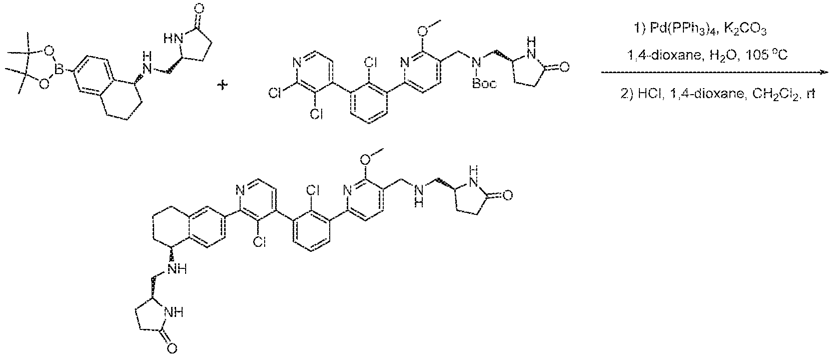

(S)-5-((((6-(2-Chloro-3-(3-chloro-2-((S)-5-((((S)-5-oxopyrrolidin-2-yl)methyl)amino)-5,6,7,8-tetrahydronaphthalen-2-yl)pyridin-4-yl)phenyl)-2-methoxypyridin-3-yl)methyl)amino)methyl)pyrrolidin-2-one was prepared in the same manner as described for Example 48, utilizing intermediate tert-butyl (S)-((6-(2-chloro-3-(2,3-dichloropyridin-4-yl)phenyl)-2-methoxypyridin-3-yl)methyl)((5-oxopyrrolidin-2-yl)methyl)carbamate and replacing (S)-1-(8-(4,4,5,5-tetramethyl-1,3,2-dioxaborolan-2-yl)-2,3-dihydrobenzo[f][1,4]oxazepin-4(5H)-yl)propan-2-ol with (S)-5-((((S)-6-(4,4,5,5-tetramethyl-1,3,2-dioxaborolan-2-yl)-1,2,3,4-tetrahydronaphthalen-1-yl)amino)methyl)pyrrolidin-2-one in step (b). Product was obtained as a white solid (formic acid salt). LC/MS: *m*/*z* found 699, 701 [M+H] ⁺, retention time = 2.11 min, (Method C). ¹H NMR (400 MHz, Methanol-*d*₄) δ 8.61 (d, 1H), 8.44 (s, 1H), 7.80 (d, 1H), 7.70 (d, 1H), 7.60 - 7.49 (m, 3H), 7.49 - 7.37 (m, 3H), 7.2 (d, 1H), 4.18 (s, 1H), 4.03 (d, 5H), 3.91 (s, 2H), 3.08 - 2.81 (m, 6H), 2.35 (q, 6H), 2.07 (s, 3H), 1.85 (d, 3H).

### Example 441: (S)-5-((((6-(2-Chloro-3-(3-chloro-2-((R)-5-((((S)-5-oxopyrrolidin-2-yl)methyl)amino)-5,6,7,8-tetrahydronaphthalen-2-yl)pyridin-4-yl)phenyl)-2-methoxypyridin-3-yl)methyl)amino)methyl)pyrrolidin-2-one (185)

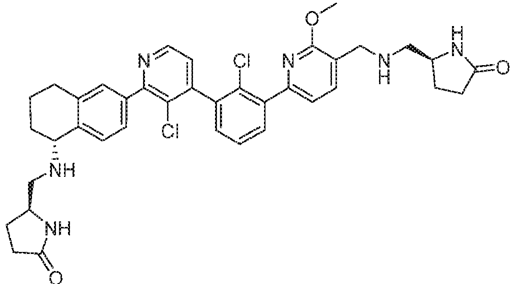

### (a) (S)-5-((((R)-6-Bromo-1,2,3,4-tetrahydronaphthalen-1-yl)amino)methyl)pyrrolidin-2-one

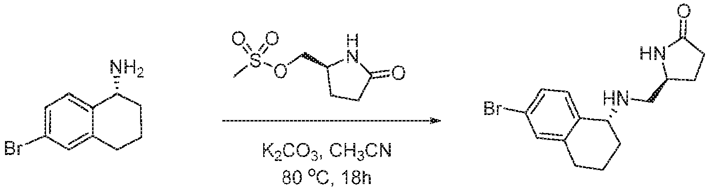

(R)-6-Bromo-1,2,3,4-tetrahydronaphthalen-1-amine (75 mg, 0.33 mmol), (S)-(5-oxopyrrolidin-2-yl)methyl methanesulfonate (77 mg, 0.40 mmol), and potassium carbonate (137 mg, 1.00 mmol) were suspended in 3 mL acetonitrile. The reaction mixture was then heated at 80 °C for 18 hours. The reaction was cooled to room temperature, diluted with water (6 mL), and extracted with EtOAc (3 x 5 mL). The combined organics were washed with water (5 mL), brine (5 mL), dried over sodium sulfate, filtered, and concentrated under reduced pressure to afford (S)-5-((((R)-6-bromo-1,2,3,4-tetrahydronaphthalen-1-yl)amino)methyl)pyrrolidin-2-one (107 mg, crude) as a light brown oil. MS: *m*/*z* found 323, 325 [M+H] ⁺. Material was used for the next step without further purification.

### (b) tert-Butyl ((R)-6-bromo-1,2,3,4-tetrahydronaphthalen-1-yl)(((S)-5-oxopyrrolidin-2-yl)methyl)carbamate

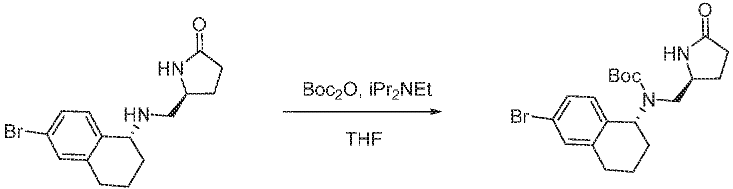

(S)-5-((((R)-6-Bromo-1,2,3,4-tetrahydronaphthalen-1-yl)amino)methyl)pyrrolidin-2-one (107 mg, 0.33 mmol, crude) and N,N-diisopropylethylamine (85 mg, 0.66 mmol) were dissolved in 5 mL THF and di-tert-butyl dicarbonate (145 mg, 0.66 mmol) was added portion-wise. The resulting mixture was stirred at room temperature for 18 hours. EtOAc (15 mL) was added to the crude mixture and the resulting solution was washed with saturated aqueous sodium hydrogen carbonate (2 x 10 mL) and brine (2 x 10 mL). The organic layer was concentrated under reduced pressure and crude sample was purified by reverse phase HPLC to afford tert-butyl ((R)-6-bromo-1,2,3,4-tetrahydronaphthalen-1-yl)(((S)-5-oxopyrrolidin-2-yl)methyl)carbamate (39 mg, 28 % yield) as a white foam. MS: *m*/*z* found 423, 425 [M+H]⁺.

### (c) tert-Butyl (((S)-5-oxopyrrolidin-2-yl)methyl)((R)-6-(4,4,5,5-tetramethyl-1,3,2-dioxaborolan-2-yl)-1,2,3,4-tetrahydronaphthalen-1-yl)carbamate

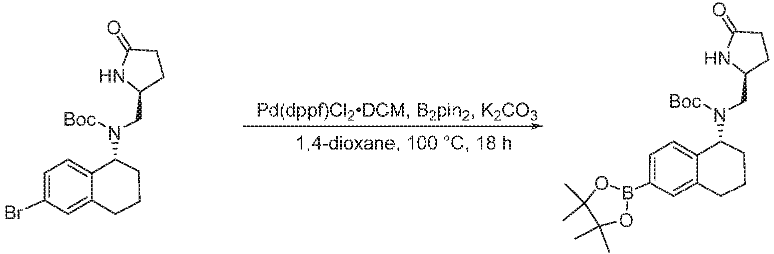

tert-Butyl ((R)-6-bromo-1,2,3,4-tetrahydronaphthalen-1-yl)(((S)-5-oxopyrrolidin-2-yl)methyl)carbamate (39 mg, 0.09 mmol), bis(pinacolato)diborane (33 mg, 0.13 mmol), [1,1'-bis(diphenylphosphino)ferrocene]dichloropalladium(II) complex with dichloromethane (8 mg, 0.01 mmol), and potassium carbonate (36 mg, 0.26 mmol) were suspended in 2 mL 1,4-dioxane in a sealed tube. Nitrogen gas was bubbled through the reaction solution for 2 minutes, then the vessel was sealed and heated at 100 °C for 18 hours. Reaction was cooled to room temperature and the mixture was filtered through celite. The filtrate was concentrated under reduced pressure and the crude oil was purified by reverse phase HPLC to afford tert-butyl (((S)-5-oxopyrrolidin-2-yl)methyl)((R)-6-(4,4,5,5-tetramethyl-1,3,2-dioxaborolan-2-yl)-1,2,3,4-tetrahydronaphthalen-1-yl)carbamate (17 mg, 39 % yield) as a white solid. MS: m/z found 471 [M+H]⁺.

### (d) (S)-5-((((6-(2-Chloro-3-(3-chloro-2-((R)-5-((((S)-5-oxopyrrolidin-2-yl)methyl)amino)-5,6,7,8-tetrahydronaphthalen-2-yl)pyridin-4-yl)phenyl)-2-methoxypyridin-3-yl)methyl)amino)methyl)pyrrolidin-2-one

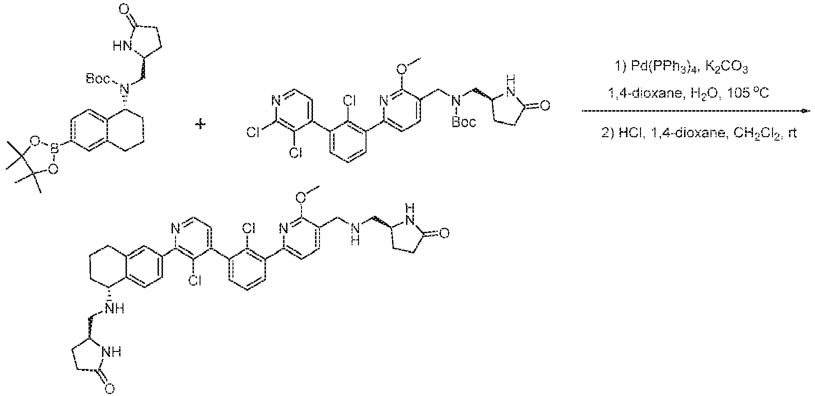

(S)-5-((((6-(2-Chloro-3-(3-chloro-2-((R)-5-((((S)-5-oxopyrrolidin-2-yl)methyl)amino)-5,6,7,8-tetrahydronaphthalen-2-yl)pyridin-4-yl)phenyl)-2-methoxypyridin-3-yl)methyl)amino)methyl)pyrrolidin-2-one was prepared in the same manner as described for Example 48, utilizing intermediate tert-butyl (S)-((6-(2-chloro-3-(2,3-dichloropyridin-4-yl)phenyl)-2-methoxypyridin-3-yl)methyl)((5-oxopyrrolidin-2-yl)methyl)carbamate and replacing (S)-1-(8-(4,4,5,5-tetramethyl-1,3,2-dioxaborolan-2-yl)-2,3-dihydrobenzo[f][1,4]oxazepin-4(5H)-yl)propan-2-ol with (((S)-5-oxopyrrolidin-2-yl)methyl)((R)-6-(4,4,5,5-tetramethyl-1,3,2-dioxaborolan-2-yl)-1,2,3,4-tetrahydronaphthalen-1-yl)carbamate in step (b). Product was obtained as a white solid (formic acid salt). LC/MS: *m*/*z* found 699, 701 [M+H] ⁺, retention time = 2.10 min (Method C). ¹H NMR (400 MHz, Methanol-*d*₄) δ 8.61 (d, 1H), 8.44 (s, 1H), 7.80 (d, 1H), 7.70 (d, 1H), 7.60 - 7.49 (m, 3H), 7.49 - 7.37 (m, 3H), 7.2 (d, 1H), 4.18 (s, 1H), 4.03 (d, 5H), 3.91 (s, 2H), 3.08 - 2.81 (m, 6H), 2.35 (q, 6H), 2.07 (s, 3H), 1.85 (d, 3H).

### Example 442: 2-((6-(2-chloro-3-(3-chloro-2-((R)-5-((((S)-5-oxopyrrolidin-2-yl)-methyl)-amino)-5,6,7,8-tetrahydronaphthalen-2-yl)pyridin-4-yl)phenyl)-2-methoxypyridin-3-yl)methyl)-2,6-diazaspiro[3.4]octan-7-one (255)

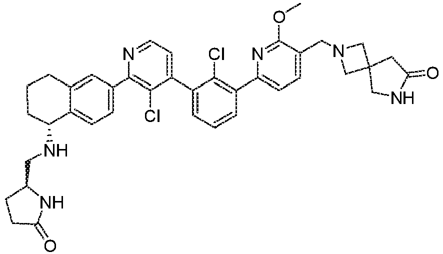

### (a) tert-Butyl ((R)-6-(3-chloro-4-(2-chloro-3-(5-formyl-6-methoxypyridin-2-yl)phenyl)pyridin-2-yl)-1,2,3,4-tetrahydronaphthalen-1-yl)(((S)-5-oxopyrrolidin-2-yl)methyl)carbamate

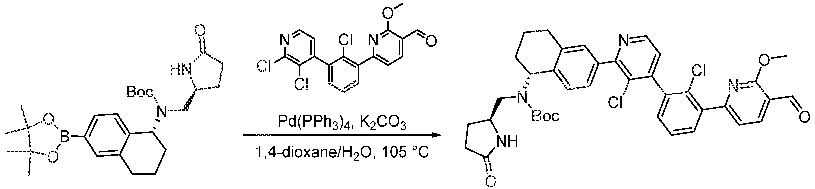

6-(2-Chloro-3-(2,3-dichloropyridin-4-yl)phenyl)-2-methoxynicotinaldehyde (188 mg, 0.48 mmol), tetrakis(triphenylphosphine)palladium(0) (74 mg, 0.06 mmol), potassium carbonate (132 mg, 0.96 mmol), and tert-butyl (((S)-5-oxopyrrolidin-2-yl)methyl)((R)-6-(4,4,5,5-tetramethyl-1,3,2-dioxaborolan-2-yl)-1,2,3,4-tetrahydronaphthalen-1-yl)carbamate (Example 441, step (c)) (150 mg, 0.32 mmol) were suspended in 1,4-dioxane /water (4:1, 5 mL), then the solution was heated at 105 °C for 20 minutes. Reaction was cooled to room temperature, diluted with 10 mL water, and extracted with EtOAc (3 x 5 mL). The combined organics were concentrated under reduced pressure and crude sample was purified by silica gel chromatography (0-4% MeOH/DCM) to afford tert-butyl ((R)-6-(3-chloro-4-(2-chloro-3-(5-formyl-6-methoxypyridin-2-yl)phenyl)pyridin-2-yl)-1,2,3,4-tetrahydronaphthalen-1-yl)(((S)-5-oxopyrrolidin-2-yl)methyl)carbamate (26 mg, 55 % yield) as a yellow oil. MS: m/z found 701, 703 [M+H] ⁺.

### (b) tert-Butyl ((R)-6-(3-chloro-4-(2-chloro-3-(6-methoxy-5-((7-oxo-2,6-diazaspiro [3.4]octan-2-yl)methyl)pyridin-2-yl)phenyl)pyridin-2-yl)-1,2,3,4-tetrahydronaphthalen-1-yl)(((S)-5-oxopyrrolidin-2-yl)methyl)carbamate

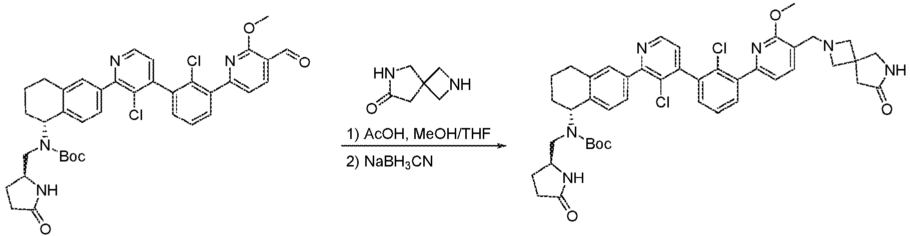

tert-Butyl ((R)-6-(3-chloro-4-(2-chloro-3-(5-formyl-6-methoxypyridin-2-yl)phenyl)pyridin-2-yl)-1,2,3,4-tetrahydronaphthalen-1-yl)(((S)-5-oxopyrrolidin-2-yl)methyl)carbamate (20 mg, 0.03 mmol), 2,6-diazaspiro[3.4]octan-7-one (7 mg, 0.04 mmol), and acetic acid (3 mg, 0.06 mmol) were dissolved in MeOH/THF (1:1, 1 mL), then the solution was stirred at 25 °C for 4 hours. Sodium cyanoborohydride (4 mg, 0.06 mmol) was added and the resulting mixture was stirred at 25 °C for 2 hours. Reaction was subsequently diluted with water (3 mL) and extracted with EtOAc (3 x 2 mL). The combined organics were further washed with brine, dried over sodium sulfate, filtered, and concentrated under reduced pressure to afford tert-butyl ((R)-6-(3-chloro-4-(2-chloro-3-(6-methoxy-5-((7-oxo-2,6-diazaspiro[3.4]octan-2-yl)methyl)pyridin-2-yl)phenyl)pyridin-2-yl)-1,2,3,4-tetrahydronaphthalen-1-yl)(((S)-5-oxopyrrolidin-2-yl)methyl)carbamate (23 mg, crude) as a yellow oil. MS: *m*/*z* found 811, 813 [M+H]⁺. Material was used for the next step without further purification.

### (c) 2-((6-(2-Chloro-3-(3-chloro-2-((R)-5-((((S)-5-oxopyrrolidin-2-yl)methyl)amino)-5,6,7,8-tetrahydronaphthalen-2-yl)pyridin-4-yl)phenyl)-2-methoxypyridin-3-yl)methyl)-2,6-diazaspiro[3.4]octan-7-one

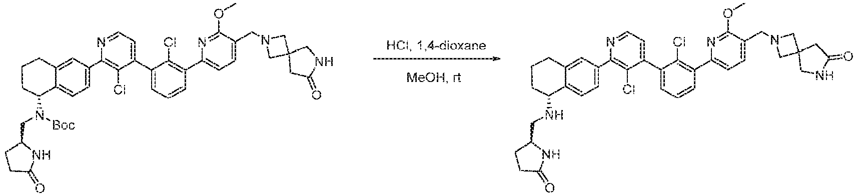

tert-Butyl ((R)-6-(3-chloro-4-(2-chloro-3-(6-methoxy-5-((7-oxo-2,6-diazaspiro[3.4]octan-2-yl)methyl)pyridin-2-yl)phenyl)pyridin-2-yl)-1,2,3,4-tetrahydronaphthalen-1-yl)(((S)-5-oxopyrrolidin-2-yl)methyl)carbamate (23 mg, 0.03 mmol) was dissolved in 1 mL MeOH and 4M HCl solution in 1,4-dioxane (28.33 µL, 0.11 mmol) was added drop-wise. Reaction was stirred at room temperature for 60 minutes and solvent was removed under reduced pressure. Crude sample was purified by reverse phase HPLC to afford 2-((6-(2-chloro-3-(3-chloro-2-((R)-5-((((S)-5-oxopyrrolidin-2-yl)methyl)amino)-5,6,7,8-tetrahydronaphthalen-2-yl)pyridin-4-yl)phenyl)-2-methoxypyridin-3-yl)methyl)-2,6-diazaspiro[3.4]octan-7-one (5 mg, 25 % yield) as a white solid (formic acid salt). LC/MS: *m*/*z* found 711, 713 [M+H]⁺, retention time = 2.11 min (Method C). ¹H NMR (400 MHz, Methanol-*d*₄) δ 8.61 (d, 1H), 8.43 (s, 1H), 7.76 (d, 1H), 7.69 (d, 1H), 7.59 - 7.48 (m, 3H), 7.46 (s, 1H), 7.44 - 7.38 (m, 2H), 7.28 (d, 1H), 4.17 (s, 1H), 4.02 (d, 3H), 3.99 (s, 2H), 3.88 (s, 1H), 3.75 (s, 4H), 3.62 (s, 2H), 3.08 - 2.77 (m, 4H), 2.63 (s, 2H), 2.33 (d, 3H), 2.04 (d, 3H), 1.93 - 1.76 (m, 2H).

### Example 443: (S)-5-((((R)-6-(3-Chloro-4-(2-chloro-3-(5-((((S)-2-hydroxypropyl)amino)methyl)-6-methoxypyridin-2-yl)phenyl)pyridin-2-yl)-1,2,3,4-tetrahydronaphthalen-1-yl)amino)methyl)pyrrolidin-2-one (256)

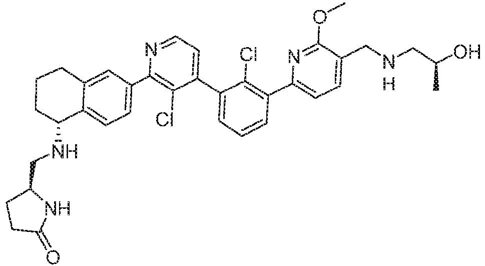

(S)-5-((((R)-6-(3-Chloro-4-(2-chloro-3-(5-((((S)-2-hydroxypropyl)amino)methyl)-6-methoxypyridin-2-yl)phenyl)pyridin-2-yl)-1,2,3,4-tetrahydronaphthalen-1-yl)amino)methyl)pyrrolidin-2-one was prepared in the same manner as described for Example 442, utilizing intermediate tert-butyl ((R)-6-(3-chloro-4-(2-chloro-3-(5-formyl-6-methoxypyridin-2-yl)phenyl)pyridin-2-yl)-1,2,3,4-tetrahydronaphthalen-1-yl)(((S)-5-oxopyrrolidin-2-yl)methyl)carbamate and replacing 2,6-diazaspiro-[3.4]octan-7-one with (S)-1-aminopropan-2-ol in step (b). Product was obtained as a white solid (formic acid salt). LC/MS: *m*/*z* found 660, 662 [M+H] ⁺, retention time = 2.12 min (Method C). ¹H NMR (400 MHz, Methanol-*d*₄) δ 8.61 (d, 1H), 8.46 (s, 1H), 7.89 - 7.82 (m, 1H), 7.74 - 7.68 (m, 1H), 7.55 (d, 2H), 7.49 (d, 1H), 7.43 (q, 3H), 7.37 - 7.30 (m, 1H), 4.26 (s, 2H), 4.13 - 3.98 (m, 5H), 3.85 (s, 1H), 3.09 (d, 1H), 2.88 (m, 5H), 2.40 - 2.24 (m, 3H), 2.03 (s, 3H), 1.86 (dd, 2H), 1.27 - 1.13 (m, 3H).

### Example 444: (S)-5-((((R)-6-(3-Chloro-4-(2-chloro-3-(6-methoxy-5-((((S)-2-oxotetrahydrofuran-3-yl)amino)methyl)pyridin-2-yl)phenyl)pyridin-2-yl)-1,2,3,4-tetrahydronaphthalen-1-yl)amino)methyl)pyrrolidin-2-one (257)

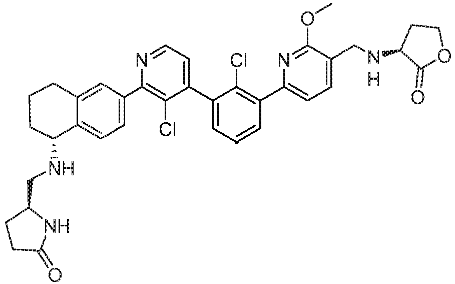

(S)-5-((((R)-6-(3-Chloro-4-(2-chloro-3-(6-methoxy-5-((((S)-2-oxotetrahydrofuran-3-yl)amino)methyl)pyridin-2-yl)phenyl)pyridin-2-yl)-1,2,3,4-tetrahydronaphthalen-1-yl)amino)methyl)pyrrolidin-2-one was prepared in the same manner as described for Example 442, utilizing intermediate tert-butyl ((R)-6-(3-chloro-4-(2-chloro-3-(5-formyl-6-methoxypyridin-2-yl)phenyl)pyridin-2-yl)-1,2,3,4-tetrahydronaphthalen-1-yl)(((S)-5-oxopyrrolidin-2-yl)methyl)carbamate and replacing 2,6-diazaspiro[3.4]octan-7-one with (S)-3-aminodihydrofuran-2(3H)-one in step (b). Product was obtained as a white solid (formic acid salt). LC/MS: *m*/*z* found 686, 688 [M+H]⁺, retention time = 2.20 min (Method C). ¹H NMR (400 MHz, Methanol-*d*₄) δ 8.60 (d, 1H), 7.77 (d, 7.70 (d, 1H), 7.53 (t, 2H), 7.48 (d, 1H), 7.44 - 7.36 (m, 3H), 7.25 (d, 1H), 4.41 (t, 1H), 4.23 (q, 1H), 4.02 (d, 4H), 3.94 (d, 2H), 3.83 (s, 1H), 3.66 (m, 1H), 2.98 - 2.73 (m, 4H), 2.56 (m, 1H), 2.39 - 2.22 (m, 3H), 2.19 - 2.08 (m, 1H), 2.03 (d, 3H), 1.85 (d, 2H).

### Example 445: ((6-(2-Chloro-3-(3-chloro-2-((R)-5-((((S)-5-oxopyrrolidin-2-yl)methyl)amino)-5,6,7,8-tetrahydronaphthalen-2-yl)pyridin-4-yl)phenyl)-2-methoxypyridin-3-yl)methyl)-L-homoserine (265)

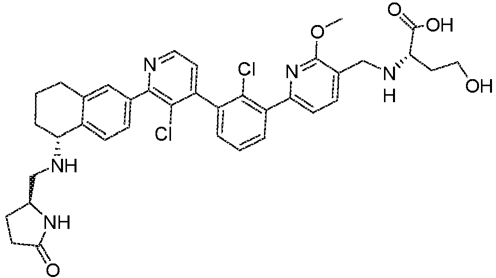

((6-(2-Chloro-3-(3-chloro-2-((R)-5-((((S)-5-oxopyrrolidin-2-yl)methyl)amino)-5,6,7,8-tetrahydronaphthalen-2-yl)pyridin-4-yl)phenyl)-2-methoxypyridin-3-yl)methyl)-L-homoserine was prepared in the same manner as described for Example 442, utilizing intermediate tert-butyl ((R)-6-(3-chloro-4-(2-chloro-3-(5-formyl-6-methoxypyridin-2-yl)phenyl)pyridin-2-yl)-1,2,3,4-tetrahydronaphthalen-1-yl)(((S)-5-oxopyrrolidin-2-yl)methyl)carbamate and replacing 2,6-diazaspiro[3.4]octan-7-one with L-homoserine in step (b). Product was obtained as a white solid (formic acid salt). LC/MS: *m*/*z* found 704, 706 [M+H] ⁺, retention time = 2.14 min (Method C). ¹H NMR (400 MHz, Methanol-*d*₄) δ 8.65 - 8.58 (m, 1H), 8.35 (s, 1H), 7.85 (d, 1H), 7.71 (d, 1H), 7.56 (t, 3H), 7.48 (s, 1H), 7.43 (d, 2H), 7.33 (d, 1H), 4.27 (d, 3H), 4.07 (d, 3H), 3.95 - 3.75 (m, 3H), 3.69 (d, 1H), 3.08 (s, 1H), 3.03 - 2.83 (m, 3H), 2.36 (m, 3H), 2.06 (d, 5H), 1.89 (s, 2H).

### Example 446: (S)-5-((((6-(2-Chloro-3-(3-chloro-2-(8-chloro-2-(3-hydroxy-3-methylbutyl)-1,2,3,4-tetrahydroisoquinolin-6-yl)pyridin-4-yl)phenyl)-2-methoxypyridin-3-yl)methyl)amino)methyl)pyrrolidin-2-one (208)

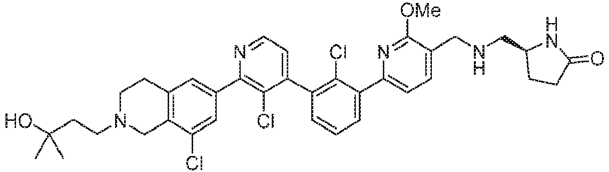

### (a) 4-(6-Bromo-8-chloro-3,4-dihydroisoquinolin-2(1H)-yl)-2-methylbutan-2-ol

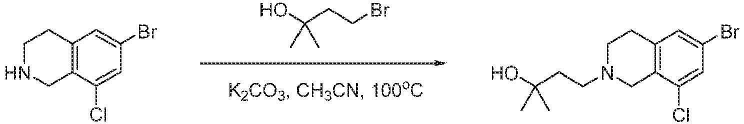

A mixture of 6-bromo-8-chloro-1,2,3,4-tetrahydroisoquinoline (0.5 g, 2.03 mmol), 4-bromo-2-methylbutan-2-ol (0.51 g, 3.04 mmol) and potassium carbonate (0.56 g, 4.06 mmol) in acetonitrile (6 mL) was degassed and purged with N₂ three times, and then the mixture was stirred at 100 °C for 12 h under N₂ atmosphere. The reaction mixture was concentrated under reduced pressure to remove solvent and the mixture was poured into ethyl acetate (15 mL). The mixture was filtered and the filtrate concentrated to afford 4-(6-bromo-8-chloro-3,4-dihydroisoquinolin-2(1H)-yl)-2-methylbutan-2-ol (0.72 g, crude) as a yellow oil. MS: *m*/*z* found 332 [M+H]⁺.

### (b) 4-(8-Chloro-6-(4,4,5,5-tetramethyl-1,3,2-dioxaborolan-2-yl)-3,4-dihydroisoquinolin-2(1H)-yl)-2-methylbutan-2-ol

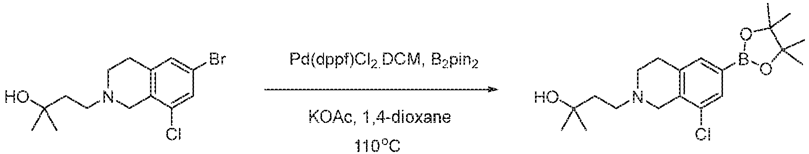

To a mixture of 4-(6-bromo-8-chloro-3,4-dihydroisoquinolin-2(1H)-yl)-2-methylbutan-2-ol (0.7 g, 2.1 mmol) and bis(pinacolato)diborane (0.8 g, 3.16 mmol) in 1,4-dioxane (8 mL) was added potassium acetate (0.41 g, 4.21 mmol) and [1,1'-bis(diphenylphosphino)ferrocene]-dichloropalladium(II) complex with dichloromethane (0.09 g, 0.11 mmol) in one portion under N₂ atmosphere and the mixture was stirred at 110 °C for 12 h. The mixture was concentrated and the residue was purified by normal phase SiO₂ chromatography (0-100% ethyl acetate/petroleum ether to 10% MeOH/ethyl acetate) to afford 4-(8-chloro-6-(4,4,5,5-tetramethyl-1,3,2-dioxaborolan-2-yl)-3,4-dihydroisoquinolin-2(1H)-yl)-2-methylbutan-2-ol (0.57 g, 71% yield) as a yellow gum. ¹H NMR (400 MHz, Methanol-*d*₄): δ 7.42 (s, 1H), 7.36 (s, 1H), 3.76 (s, 2H), 2.92-2.82 (m, 6H), 1.76-1.71 (m, 2H), 1.23 (s, 12H), 1.15 (s, 6H).

### (c) tert-Butyl (S)-((6-(2-chloro-3-(3-chloro-2-(8-chloro-2-(3-hydroxy-3-methylbutyl)-1,2,3,4-tetrahydroisoquinolin-6-yl)pyridin-4-yl)phenyl)-2-methoxypyridin-3-yl)methyl)((5-oxopyrrolidin-2-yl)methyl)carbamate

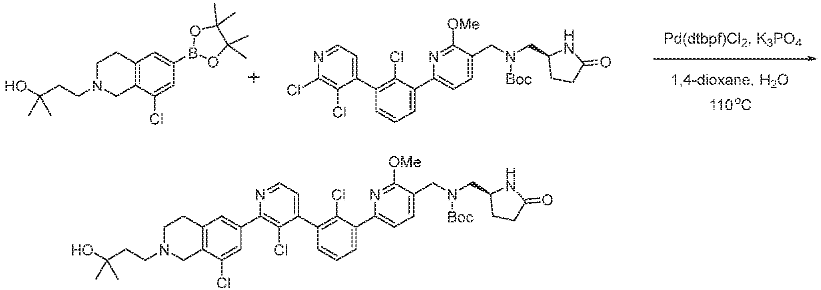

To a mixture of 4-(8-chloro-6-(4,4,5,5-tetramethyl-1,3,2-dioxaborolan-2-yl)-3,4-dihydroisoquinolin-2(1H)-yl)-2-methylbutan-2-ol (0.25 g, 0.66 mmol) and tert-butyl (S)-((6-(2-chloro-3-(2,3-dichloropyridin-4-yl)phenyl)-2-methoxypyridin-3-yl)methyl)((5-oxopyrrolidin-2-yl)methyl)carbamate (0.18 g, 0.30 mmol) in 1,4-dioxane/water mixture (10:1, 6.6 mL) was added [1,1'-bis(di-tert-butylphosphino)ferrocene]dichloropalladium(II) (0.02 g, 0.03 mmol) and potassium phosphate (0.19 g, 0.91 mmol) in one portion under N₂ atmosphere. The mixture was stirred at 110 °C for 1.5 h. The mixture was combined with another batch at the same scale. The mixture was concentrated. To the residue was added water (5 mL) and saturated aqueous brine solution (5 mL) and the mixture was extracted with ethyl acetate/THF mixture (1:1, 10 mL). The organic phase was dried with anhydrous sodium sulfate, filtered and concentrated. The residue was purified by normal phase SiO₂ chromatography (0-100% 30% THF in ethyl acetate/petroleum ether) to afford 0.2 g of the product that was further purified by prep-TLC (silica gel, ethyl acetate: MeOH =4:1) to give tert-butyl (S)-((6-(2-chloro-3-(3-chloro-2-(8-chloro-2-(3-hydroxy-3-methylbutyl)-1,2,3,4-tetrahydroisoquinolin-6-yl)pyridin-4-yl)phenyl)-2-methoxypyridin-3-yl)methyl)((5-oxopyrrolidin-2-yl)methyl)carbamate (0.09 g, 14% yield) as a light yellow solid. MS: *m*/*z* found 808 [M+H]⁺.

### (d) (S)-5-((((6-(2-Chloro-3-(3-chloro-2-(8-chloro-2-(3-hydroxy-3-methylbutyl)-1,2,3,4-tetrahydroisoquinolin-6-yl)pyridin-4-yl)phenyl)-2-methoxypyridin-3-yl)methyl)amino)methyl)pyrrolidin-2-one

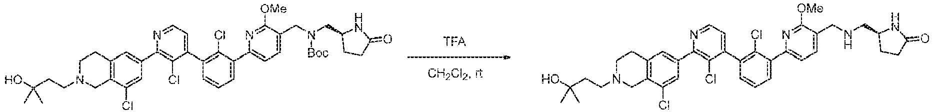

To a mixture of tert-butyl (S)-((6-(2-chloro-3-(3-chloro-2-(8-chloro-2-(3-hydroxy-3-methylbutyl)-1,2,3,4-tetrahydroisoquinolin-6-yl)pyridin-4-yl)phenyl)-2-methoxypyridin-3-yl)methyl)((5-oxopyrrolidin-2-yl)methyl)carbamate (95 mg, 0.12 mmol) in dichloromethane (0.5 mL) was added trifluoroacetic acid (1.4 mL, 19 mmol) in one portion and the mixture was stirred at room temperature for 15 min. The mixture was concentrated and the residue was purified by reverse phase HPLC to afford (S)-5-((((6-(2-chloro-3-(3-chloro-2-(8-chloro-2-(3-hydroxy-3-methylbutyl)-1,2,3,4-tetrahydroisoquinolin-6-yl)pyridin-4-yl)phenyl)-2-methoxypyridin-3-yl)methyl)amino)methyl)pyrrolidin-2-one (31.6 mg, 34% yield) as a light yellow solid (formic acid salt). MS: *m*/*z* found 708 and 710 [M+H]⁺, retention time = 2.98 min (Method A). ¹H NMR (400 MHz, Methanol-*d*₄): δ 8.67 (d, 1H), 8.31 (br s, 1H), 7.87 (d, 1H), 7.74 (dd, 1H), 7.66 (d, 1H), 7.59 (t, 1H), 7.55 (s, 1H), 7.48 (d, 1H), 7.45 (dd, 1H), 7.35 (d, 1H), 4.27-4.15 (m, 4H), 4.09 (s, 3H), 4.06-4.01 (m, 1H), 3.32-3.30 (m, 2H), 3.26-3.18 (m, 4H), 3.10-3.08 (m, 2H), 2.48-2.32 (m, 3H), 1.98-1.84 (m, 3H), 1.32 (s, 6H).

### Example 447: (S)-5-((((6-(3-(2-(2-((1-Acetylpiperidin-4-yl)methyl)-8-chloro-1,2,3,4-tetrahydroisoquinolin-6-yl)-3-chloropyridin-4-yl)-2-chlorophenyl)-2-methoxypyridin-3-yl)methyl)amino)methyl)pyrrolidin-2-one (289)

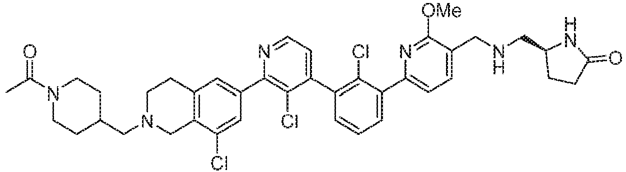

(S)-5-((((6-(3-(2-(2-((1-Acetylpiperidin-4-yl)methyl)-8-chloro-1,2,3,4-tetrahydroisoquinolin-6-yl)-3-chloropyridin-4-yl)-2-chlorophenyl)-2-methoxypyridin-3-yl)methyl)amino)methyl)pyrrolidin-2-one (15 mg, 28% yield) was prepared in a similar fashion to Example 446, using 6-bromo-8-chloro-1,2,3,4-tetrahydroisoquinoline and 1-(4-(bromomethyl)piperidin-1-yl)ethan-1-one in step (a). MS: *m*/*z* found 761 and 763 [M+H]⁺, retention time = 3.01 min (Method A). ¹H NMR (400 MHz, Methanol-*d*₄): δ 8.65 (d, 1H), 7.83 (d, 1H), 7.73 (dd, 1H), 7.60-7.56 (m, 2H), 7.46-7.43 (m, 3H), 7.33 (d, 1H), 4.60-4.53 (m, 1H), 4.08 (m, 5H), 3.98-3.95 (m, 2H), 3.79 (s, 2H), 3.18-3.15 (m, 1H), 3.05-2.98 (m, 4H), 2.87 (m, 2H), 2.74-2.68 (m, 1H), 2.58 (d, 2H), 2.42-2.35 (m, 3H), 2.12 (s, 3H), 2.04-1.85 (m, 4H), 1.31-1.11 (m, 2H).

### Example 448: (S)-5-((((6-(2-Chloro-3-(3-chloro-2-(8-chloro-2-(oxetan-3-ylmethyl)-1,2,3,4-tetrahydroisoquinolin-6-yl)pyridin-4-yl)phenyl)-2-methoxypyridin-3-yl)methyl)amino)methyl)pyrrolidin-2-one (307)

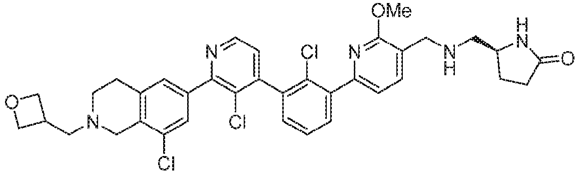

(S)-5-((((6-(2-Chloro-3-(3-chloro-2-(8-chloro-2-(oxetan-3-ylmethyl)-1,2,3,4-tetrahydroisoquinolin-6-yl)pyridin-4-yl)phenyl)-2-methoxypyridin-3-yl)methyl)amino)methyl)pyrrolidin-2-one (0.9 mg, 1.8% yield, formic acid salt) was prepared in a similar fashion to Example 446, using 6-bromo-8-chloro-1,2,3,4-tetrahydroisoquinoline and 3-(bromomethyl)oxetane in step (a). MS: *m*/*z* found 692 [M+H]⁺, retention time = 0.64 min (Method C). ¹H NMR (400 MHz, Methanol-*d*₄): δ 8.61 (d, 1H), 7.74-7.68 (m, 2H), 7.53 (t, 2H), 7.44-7.40 (m, 3H), 7.24 (d, 1H), 4.60 (s, 2H), 4.51 (t, 2H), 4.01 (s, 3H), 3.83-3.82 (m, 3H), 3.69-3.64 (m, 2H), 3.00-2.74 (m, 7H), 2.69-2.68 (m, 3H), 2.35-2.31 (m, 3H).

### Example 449: (S)-5-((((6-(2-Chloro-3-(3-chloro-2-(2-((R)-2-hydroxypropyl)-8-methoxy-1,2,3,4-tetrahydroisoquinolin-6-yl)pyridin-4-yl)phenyl)-4-fluoro-2-methoxypyridin-3-yl)methyl)amino)methyl)pyrrolidin-2-one (131)

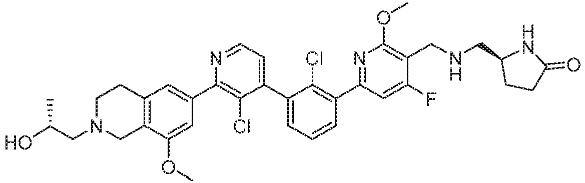

### (a) (R)-1-(6-Bromo-8-methoxy-3,4-dihydroisoquinolin-2(1H)-yl)propan-2-ol

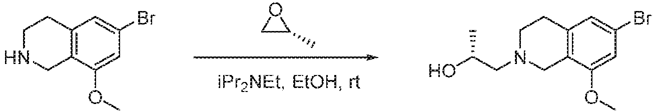

To a mixture of 6-bromo-8-methoxy-1,2,3,4-tetrahydroisoquinoline hydrochloride salt (0.5 g, 1.8 mmol) in EtOH (6 mL) was added N,N-diisopropylethylamine (1.9 mL, 10.8 mmol) and (2R)-2-methyloxirane (1 mL, 14.4 mmol) in one portion under N₂ atmosphere. The mixture was stirred at room temperature for 12 h. The mixture was then concentrated to afford (R)-1-(6-bromo-8-methoxy-3,4-dihydroisoquinolin-2(1H)-yl)propan-2-ol (0.6 g, crude) as a yellow gum. MS: *m*/*z* found 300 and 302 [M+H]⁺.

### (b) (R)-1-(8-Methoxy-6-(4,4,5,5-tetramethyl-1,3,2-dioxaborolan-2-yl)-3,4-dihydroisoquinolin-2(1H)-yl)propan-2-ol

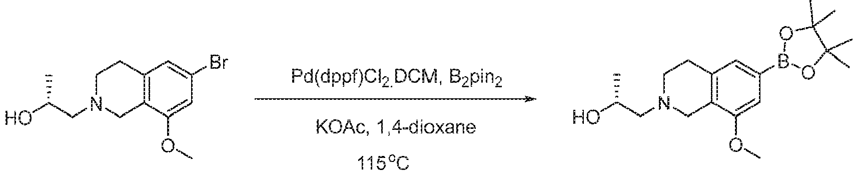

To a mixture of (R)-1-(6-bromo-8-methoxy-3,4-dihydroisoquinolin-2(1H)-yl)propan-2-ol (0.6 g, 2 mmol) and bis(pinacolato)diborane (1.02 g, 4 mmol) in 1,4-dioxane (15 mL) was added [1,1'- bis(diphenylphosphino)ferrocene]dichloropalladium(II) complex with dichloromethane (0.065 g, 0.08 mmol) and potassium acetate (0.39 g, 4 mmol) in one portion under N₂ atmosphere. The mixture was stirred at 115 °C for 12 h. The mixture was concentrated and the residue was purified by normal phase SiO₂ chromatography (0-100% 30% THF in ethyl acetate/petroleum ether to 5% MeOH/ethyl acetate) to afford (R)-1-(8-methoxy-6-(4,4,5,5-tetramethyl-1,3,2-dioxaborolan-2-yl)-3,4-dihydroisoquinolin-2(1H)-yl)propan-2-ol (0.45 g, 55% yield) as a yellow gum. ¹H NMR (400 MHz, Methanol-*d*₄): δ 7.20 (s, 1H), 7.14 (s, 1H), 4.17-4.12 (m, 1H), 3.87 (s, 3H), 3.24-2.90 (m, 6H), 2.82-2.80 (m, 2H), 1.36 (s, 12H), 1.24-1.22 (m, 3H).

### (c) tert-Butyl ((6-(2-chloro-3-(3-chloro-2-(2-((R)-2-hydroxypropyl)-8-methoxy-1,2,3,4-tetrahydroisoquinolin-6-yl)pyridin-4-yl)phenyl)-4-fluoro-2-methoxypyridin-3-yl)methyl)(((S)-5-oxopyrrolidin-2-yl)methyl)carbamate

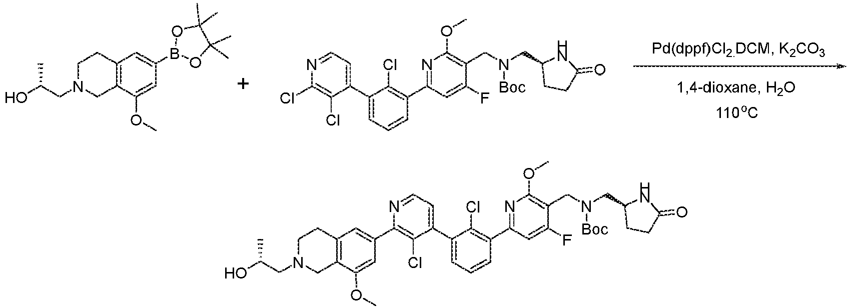

To a mixture of (R)-1-(8-methoxy-6-(4,4,5,5-tetramethyl-1,3,2-dioxaborolan-2-yl)-3,4-dihydroisoquinolin-2(1H)-yl)propan-2-ol (0.17 g, 0.5 mmol) and tert-butyl (S)-((6-(2-chloro-3-(2,3-dichloropyridin-4-yl)phenyl)-4-fluoro-2-methoxypyridin-3-yl)methyl)((5-oxopyrrolidin-2-yl)methyl)carbamate (0.18 g, 0.29 mmol) in 1,4-dioxane/water mixture (8:1, 4.5 mL) was added [1,1'- bis(diphenylphosphino)ferrocene]dichloropalladium(II) complex with dichloromethane (0.024 g, 0.03 mmol) and potassium carbonate (0.12 g, 0.88 mmol) in one portion under N₂ atmosphere. The mixture was stirred at 110 °C for 12 h. The mixture was concentrated. To the residue was added water (5 mL) and saturated aqueous brine solution (5 mL) and the mixture was extracted with ethyl acetate/THF mixture (1:1, 10 mL). The organic phase was dried with anhydrous sodium sulfate, filtered and concentrated. The residue was purified by normal phase SiO₂ chromatography (30% 25% THF in ethyl acetate/petroleum ether to ethyl acetate/THF/ MeOH=10/3/1) to afford tert-butyl ((6-(2-chloro-3-(3-chloro-2-(2-((R)-2-hydroxypropyl)-8-methoxy-1,2,3,4-tetrahydroisoquinolin-6-yl)pyridin-4-yl)phenyl)-4-fluoro-2-methoxypyridin-3-yl)methyl)(((S)-5-oxopyrrolidin-2-yl)methyl)carbamate (0.175 g, 50% yield) as a green gum. MS: *m*/*z* found 794 [M+H]⁺.

### (d) (S)-5-((((6-(2-Chloro-3-(3-chloro-2-(2-((R)-2-hydroxypropyl)-8-methoxy-1,2,3,4-tetrahydroisoquinolin-6-yl)pyridin-4-yl)phenyl)-4-fluoro-2-methoxypyridin-3-yl)methyl)amino)methyl)pyrrolidin-2-one

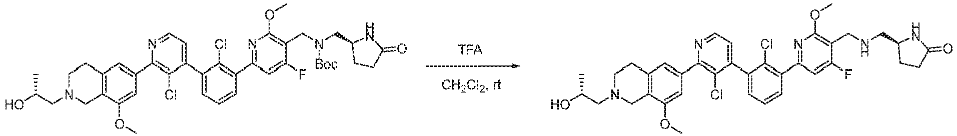

To a mixture of tert-butyl ((6-(2-chloro-3-(3-chloro-2-(2-((R)-2-hydroxypropyl)-8-methoxy-1,2,3,4-tetrahydroisoquinolin-6-yl)pyridin-4-yl)phenyl)-4-fluoro-2-methoxypyridin-3-yl)methyl)(((S)-5-oxopyrrolidin-2-yl)methyl)carbamate (0.17 g, 0.21 mmol) in dichloromethane (0.5 mL) was added trifluoroacetic acid (1.8 mL) in one portion. The mixture was stirred at room temperature for 20 min and then the mixture was concentrated. The residue was purified by reverse phase HPLC to afford (S)-5-((((6-(2-chloro-3-(3-chloro-2-(2-((R)-2-hydroxypropyl)-8-methoxy-1,2,3,4-tetrahydroisoquinolin-6-yl)pyridin-4-yl)phenyl)-4-fluoro-2-methoxypyridin-3-yl)methyl)amino)methyl)pyrrolidin-2-one (52.1 mg, 32% yield) as a light yellow solid (formic acid salt). MS: *m*/*z* found 694 [M+H]⁺, retention time = 2.73 min (Method A). ¹H NMR (400 MHz, Methanol-*d*₄): δ 8.66 (d, 1H), 7.76 (dd, 1H), 7.60 (t, 1H), 7.48-7.40 (m, 2H), 7.20-7.19 (m, 3H), 4.47-4.39 (m, 2H), 4.30 (m, 1H), 4.09-4.06 (m, 5H), 3.95-3.91 (m, 4H), 3.60-3.59 (m, 2H), 3.28-3.17 (m, 4H), 2.88-2.83 (m, 2H), 2.42-2.30 (m, 3H), 1.88-1.81 (m, 1H), 1.30 (d, 3H).

### Example 450: (S)-5-((((6-(2-Chloro-3-(3-chloro-2-(2-(2-hydroxyethyl)-8-methoxy-1,2,3,4-tetrahydroisoquinolin-6-yl)pyridin-4-yl)phenyl)-4-fluoro-2-methoxypyridin-3-yl)methyl)amino)methyl)pyrrolidin-2-one (132)

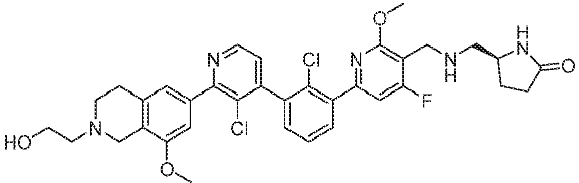

### (a) 6-Bromo-2-(2-((tert-butyldimethylsilyl)oxy)ethyl)-8-methoxy-1,2,3,4-tetrahydroisoquinoline

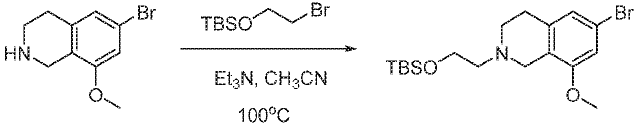

To a mixture of 6-bromo-8-methoxy-1,2,3,4-tetrahydroisoquinoline (0.4 g, 1.65 mmol) and (2-bromoethoxy)(tert-butyl)dimethylsilane (0.59 g, 2.48 mmol) in acetonitrile (8 mL) was added triethylamine (1.4 mL, 9.91 mmol) in one portion under N₂ atmosphere and the mixture stirred at 100 °C for 12 h. The mixture was concentrated. To the residue was added water (5 mL) and saturated aqueous brine solution (5 mL). The mixture was extracted with ethyl acetate/THF mixture (1:1, 20 mL). The organic phase was dried with anhydrous sodium sulfate, filtered and concentrated. The residue was purified by normal phase SiO₂ chromatography (0-30% ethyl acetate/petroleum ether) to afford 6-bromo-2-(2-((tertbutyldimethylsilyl)oxy)ethyl)-8-methoxy-1,2,3,4-tetrahydroisoquinoline (0.45 g, 62% yield) as a yellow gum. ¹H NMR (400 MHz, CDCl₃): δ 6.80 (s, 1H), 6.70 (s, 1H), 3.79-3.76 (m, 2H), 3.71 (s, 3H), 3.50 (m, 2H), 2.76-2.65 (m, 6H), 0.84 (s, 9H), 0.00 (s, 6H).

### (b) 2-(2-((tert-Butyldimethylsilyl)oxy)ethyl)-8-methoxy-6-(4,4,5,5-tetramethyl-1,3,2-dioxaborolan-2-yl)-1,2,3,4-tetrahydroisoquinoline

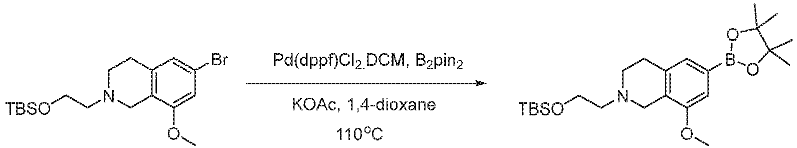

To a mixture of 6-bromo-2-(2-((tert-butyldimethylsilyl)oxy)ethyl)-8-methoxy-1,2,3,4-tetrahydroisoquinoline (0.42 g, 1.05 mmol) and bis(pinacolato)diborane (0.45 g, 1.78 mmol) in 1,4-dioxane (5 mL) was added potassium acetate (0.21 g, 2.1 mmol) and [1,1'-bis(diphenylphosphino)ferrocene]dichloropalladium(II) complex with dichloromethane (0.04 g, 0.05 mmol) in one portion under N₂ atmosphere and the mixture was stirred at 110 °C for 12 h. The mixture was concentrated and the residue was purified by normal phase SiO₂ chromatography (0-30% 30% THF in ethyl acetate/petroleum ether) to afford 2-(2-((tertbutyldimethylsilyl)oxy)ethyl)-8-methoxy-6-(4,4,5,5-tetramethyl-1,3,2-dioxaborolan-2-yl)-1,2,3,4-tetrahydroisoquinoline (0.4 g, 78% yield) as a yellow gum. ¹H NMR (400 MHz, CDCl₃): δ 7.13 (s, 1H), 6.98 (s, 1H), 3.85-3.71 (m, 5H), 3.67-3.58 (m, 2H), 2.83 (m, 2H), 2.75-2.70 (m, 4H), 1.27 (s, 12H), 0.83 (s, 9H), 0.00 (s, 6H).

### (c) tert-Butyl (S)-((6-(3-(2-(2-(2-((tert-butyldimethylsilyl)oxy)ethyl)-8-methoxy-1,2,3,4-tetrahydroisoquinolin-6-yl)-3-chloropyridin-4-yl)-2-chlorophenyl)-4-fluoro-2-methoxypyridin-3-yl)methyl)((5-oxopyrrolidin-2-yl)methyl)carbamate

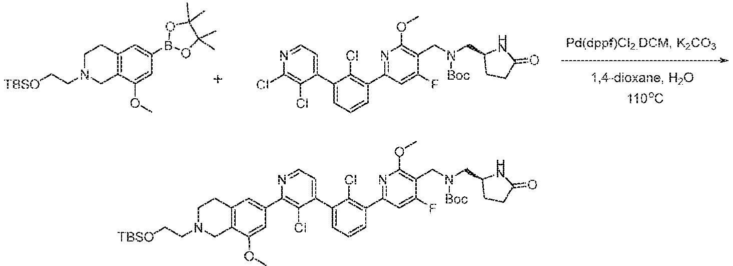

To a mixture of 2-(2-((tert-butyldimethylsilyl)oxy)ethyl)-8-methoxy-6-(4,4,5,5-tetramethyl-1,3,2-dioxaborolan-2-yl)-1,2,3,4-tetrahydroisoquinoline (0.18 g, 0.41 mmol) and tert-butyl (S)-((6-(2-chloro-3-(2,3-dichloropyridin-4-yl)phenyl)-4-fluoro-2-methoxypyridin-3-yl)methyl)((5-oxopyrrolidin-2-yl)methyl)carbamate (0.18 g, 0.3 mmol) in 1,4-dioxane/water mixture (9:1, 5 mL) was added potassium carbonate (0.12 g, 0.89 mmol) and [1,1'- bis(diphenylphosphino)ferrocene]dichloropalladium(II) complex with dichloromethane (0.024 g, 0.03 mmol) in one portion under N₂ atmosphere and the mixture stirred at 110 °C for 12 h. The mixture was concentrated. To the residue was added water (5 mL) and saturated aqueous brine solution (5 mL) and the mixture was extracted with ethyl acetate/THF mixture (1:1, 10 mL). The organic phase was dried with anhydrous sodium sulfate, filtered and concentrated. The residue was purified by normal phase SiO₂ chromatography (0-70% 25% THF in ethyl acetate/petroleum ether to 15% 30% THF in MeOH/ethyl acetate) to afford tert-butyl (S)-((6-(3-(2-(2-(2-((tertbutyldimethylsilyl)oxy)ethyl)-8-methoxy-1,2,3,4-tetrahydroisoquinolin-6-yl)-3-chloropyridin-4-yl)-2-chlorophenyl)-4-fluoro-2-methoxypyridin-3-yl)methyl)((5-oxopyrrolidin-2-yl)methyl)carbamate (0.27 g, 54% yield) as a brown gum MS: *m*/*z* found 894 [M+H]⁺.

### (d) (S)-5-((((6-(2-Chloro-3-(3-chloro-2-(2-(2-hydroxyethyl)-8-methoxy-1,2,3,4-tetrahydroisoquinolin-6-yl)pyridin-4-yl)phenyl)-4-fluoro-2-methoxypyridin-3-yl)methyl)amino)methyl)pyrrolidin-2-one

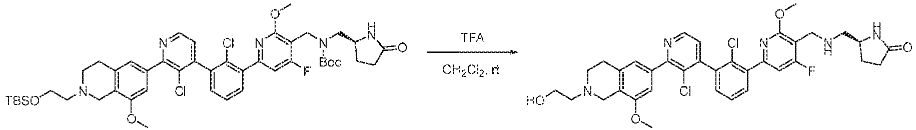

To a mixture of tert-butyl (S)-((6-(3-(2-(2-(2-((tert-butyldimethylsilyl)oxy)ethyl)-8-methoxy-1,2,3,4-tetrahydroisoquinolin-6-yl)-3-chloropyridin-4-yl)-2-chlorophenyl)-4-fluoro-2-methoxypyridin-3-yl)methyl)((5-oxopyrrolidin-2-yl)methyl)carbamate (0.265 g, 0.3 mmol) in dichloromethane (0.5 mL) was added trifluoroacetic acid (3.5 mL) in one portion and the mixture was stirred at room temperature for 5 h. The mixture was concentrated and the residue purified by reverse phase HPLC to afford (S)-5-((((6-(2-chloro-3-(3-chloro-2-(2-(2-hydroxyethyl)-8-methoxy-1,2,3,4-tetrahydroisoquinolin-6-yl)pyridin-4-yl)phenyl)-4-fluoro-2-methoxypyridin-3-yl)methyl)amino)methyl)pyrrolidin-2-one (35.4 mg, 15% yield) as a yellow solid (formic acid salt). MS: *m*/*z* found 680 [M+H]⁺, retention time = 2.61 min (Method A). ¹H NMR (400 MHz, Methanol-*d*₄): δ 8.65 (d, 1H), 7.75 (dd, 1H), 7.59 (t, 1H), 7.47-7.45 (m, 2H), 7.17-7.15 (m, 3H), 4.27-4.25 (m, 2H), 4.08 (s, 3H), 3.95-3.93 (m, 7H), 3.89-3.85 (m, 1H), 3.44-3.42 (m, 2H), 3.29-3.27 (m, 2H), 3.19 (m, 2H), 2.80-2.72 (m, 2H), 2.38-2.27 (m, 3H), 1.87-1.79 (m, 1H).

### Example 451: (S)-5-((((6-(2-Chloro-3-(3-chloro-2-(2-((S)-2-hydroxypropyl)-8-methoxy-1,2,3,4-tetrahydroisoquinolin-6-yl)pyridin-4-yl)phenyl)-4-fluoro-2-methoxypyridin-3-yl)methyl)amino)methyl)pyrrolidin-2-one (176)

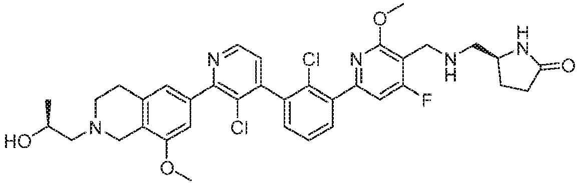

(S)-5-((((6-(2-Chloro-3-(3-chloro-2-(2-((S)-2-hydroxypropyl)-8-methoxy-1,2,3,4-tetrahydroisoquinolin-6-yl)pyridin-4-yl)phenyl)-4-fluoro-2-methoxypyridin-3-yl)methyl)amino)methyl)pyrrolidin-2-one (27.8 mg, 29% yield, formic acid salt) was prepared in a similar fashion to Example 449, using 6-bromo-8-methoxy-1,2,3,4-tetrahydroisoquinoline and (S)-2-methyloxirane in step (a). MS: *m*/*z* found 694 [M+H]⁺, retention time = 2.58 min (Method A). ¹H NMR (400 MHz, Methanol-*d*₄): δ 8.66 (d, 1H), 7.76 (dd, 1H), 7.60 (t, 1H), 7.48-7.45 (m, 2H), 7.19-7.17 (m, 3H), 4.43-4.27 (m, 3H), 4.09 (s, 3H), 4.01 (s, 2H), 3.95 (s, 3H), 3.93-3.88 (m, 1H), 3.58-3.53 (m, 2H), 3.26-3.13 (m, 4H), 2.85-2.77 (m, 2H), 2.39-2.29 (m, 3H), 1.87-1.80 (m, 1H), 1.29 (d, 3H).

### Example 452: (S)-1-(((6-(2-chloro-3-(3-chloro-2-(2-((R)-2-hydroxypropyl)-8-methoxy-1,2,3,4-tetrahydroisoquinolin-6-yl)pyridin-4-yl)phenyl)-2-methoxypyridin-3-yl)methyl)amino)propan-2-ol (214)

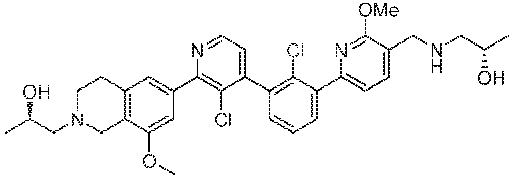

### (a) (R)-1-(6-Bromo-8-methoxy-3,4-dihydroisoquinolin-2(1H)-yl)propan-2-ol

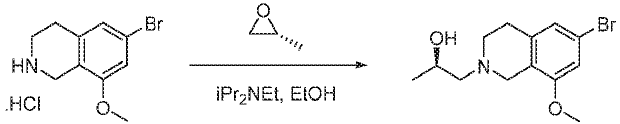

A scintillation vial was charged with 6-bromo-8-methoxy-1,2,3,4-tetrahydroisoquinoline hydrochloride (0.5 g, 1.79 mmol), (R)-2-methyloxirane (1.04 g, 17.95 mmol), and N,N-diisopropylethylamine (0.94 ml, 5.38 mmol). Ethanol (15 mL) was then added, and the reaction was stirred at 85°C, for 1 hr. The mixture was filtered through CELITE^{®} and the filter cake was washed with ethyl acetate (3 x 40 mL). The filtrate was concentrated under reduced pressure. The residue was purified by normal phase flash SiO₂ chromatography using a gradient (0-60% ethyl acetate/hexanes) to afford (R)-1-(6-bromo-8-methoxy-3,4-dihydroisoquinolin-2(1H)-yl)propan-2-ol (0.4 g, 74 % yield). MS: m/z found 300 [M+H]⁺.

### (b) (R)-1-(8-Methoxy-6-(4,4,5,5-tetramethyl-1,3,2-dioxaborolan-2-yl)-3,4-dihydroisoquinolin-2(1H)-yl)propan-2-ol

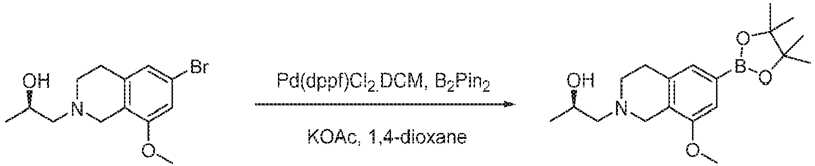

A microwave vial was charged with (R)-1-(6-bromo-8-methoxy-3,4-dihydroisoquinolin-2(1H)-yl)propan-2-ol (0.40 g, 1.33 mmol), bis(pinacolato)diborane (0.37 g, 1.47 mmol), potassium acetate (0.37g, 3.73 mmol), and 1,1'-bis(diphenylphosphino)ferrocene-palladium(II) dichloromethane complex (0.08 g, 0.09 mmol). The flask was purged with nitrogen for 5 min, then dry dioxane (2 mL) was added, and the reaction was sparged with nitrogen for 5 min. The mixture was stirred at 90 °C for 1 hr thermally. The mixture was filtered through CELITE^{®} and the filter cake was washed with ethyl acetate (3 x 40 mL). The filtrate was concentrated under reduced pressure. The residue was purified by normal phase flash SiO₂ chromatography using a gradient (0-10% methanol / CH₂Cl₂) to afford (R)-1-(8-methoxy-6-(4,4,5,5-tetramethyl-1,3,2-dioxaborolan-2-yl)-3,4-dihydroisoquinolin-2(1H)-yl)propan-2-ol (0.36 g, 78% yield). MS: m/z found 348 [M+H]⁺.

### (c) (R)-6-(2-Chloro-3-(3-chloro-2-(2-(2-hydroxypropyl)-8-methoxy-1,2,3,4-tetrahydroisoquinolin-6-yl)pyridin-4-yl)phenyl)-2-methoxynicotinaldehyde

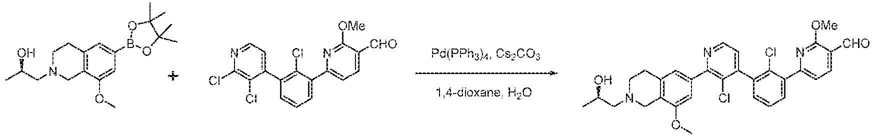

A microwave vial was charged with (R)-1-(8-methoxy-6-(4,4,5,5-tetramethyl-1,3,2-dioxaborolan-2-yl)-3,4-dihydroisoquinolin-2(1H)-yl)propan-2-ol (0.36 g, 1.04 mmol), 6-(2-chloro-3-(2,3-dichloropyridin-4-yl)phenyl)-2-methoxynicotinaldehyde (0.41 g, 1.04 mmol), cesium carbonate (1.01 g, 3.11 mmol), and tetrakis(triphenylphosphine)palladium(0) (0.08 g, 0.07 mmol). 1,4-Dioxane (2 ml) was added, and the reaction was sparged with nitrogen, then water (0.3 ml) added. The mixture was stirred at 90 °C for 1 hr thermally. The mixture was filtered through CELITE^{®} and the filter cake was washed with ethyl acetate (3 x 15 mL). The filtrate was concentrated under reduced pressure. The residue was purified by normal phase flash SiO₂ chromatography using a gradient (0-5 % methanol DCM) to afford (R)-6-(2-chloro-3-(3-chloro-2-(2-(2-hydroxypropyl)-8-methoxy-1,2,3,4-tetrahydroisoquinolin-6-yl)pyridin-4-yl)phenyl)-2-methoxynicotinaldehyde (0.35 g, 58 % yield). MS: m/z found 578 [M+H]⁺.

### (d) (S)-1-(((6-(2-Chloro-3-(3-chloro-2-(2-((R)-2-hydroxypropyl)-8-methoxy-1,2,3,4-tetrahydroisoquinolin-6-yl)pyridin-4-yl)phenyl)-2-methoxypyridin-3-yl)methyl) amino)propan-2-ol

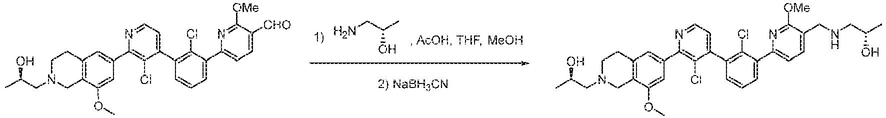

To a mixture of (R)-6-(2-chloro-3-(3-chloro-2-(2-(2-hydroxypropyl)-8-methoxy-1,2,3,4-tetrahydroisoquinolin-6-yl)pyridin-4-yl)phenyl)-2-methoxynicotinaldehyde (0.07 g, 0.11 mmol), and (S)-1-aminopropan-2-ol (0.02 g, 0.22 mmol), in 1:1 THF/MeOH (6 mL) was added acetic acid (0.01 g, 0.22 mmol) and 4 A molecular sieve (1 g). The mixture was stirred at room temperature for 3 hrs. Sodium cyanoborohydride (0.02 g, 0.28 mmol) was added and the mixture was stirred at room temperature for 1 hr. The reaction was quenched by addition of water (1 mL). The mixture was diluted with ethyl acetate (50 ml), then washed with saturated aqueous brine solution (30 mL), then aqueous layer extracted with ethyl acetate (3 x 30 mL), the combined organic phases dried over sodium sulfate, filtered, and concentrated under reduced pressure. The residue was purified reverse phase HPLC to give (S)-1-(((6-(2-chloro-3-(3-chloro-2-(2-((R)-2-hydroxypropyl)-8-methoxy-1,2,3,4-tetrahydroisoquinolin-6-yl)pyridin-4-yl)phenyl)-2-methoxypyridin-3-yl)methyl)amino)propan-2-ol (15 mg, 21% yield) as a white solid (formic acid salt). MS: m/z found 637 [M+H]⁺. ¹H NMR (400 MHz, Methanol-*d*₄) δ 8.58 (d, 1H), 8.50 (s, 1H), 7.79 (d, 1H), 7.68 (dd, 1H), 7.53 (t, 1H), 7.43 - 7.36 (m, 2H), 7.29 (d, 1H), 7.04 (d, 2H), 4.15 - 4.00 (m, 6H), 4.03 - 3.93 (m, 1H), 3.85 (s, 5H), 3.00 (s, 4H), 2.91 (d, 1H), 2.79 - 2.67 (m, 3H), 1.19 (dd, 6H).

### Example 453: (1r,3r)-3-(((6-(2-Chloro-3-(3-chloro-2-(2-((R)-2-hydroxypropyl)-8-methoxy-1,2,3,4-tetrahydroisoquinolin-6-yl)pyridin-4-yl)phenyl)-2-methoxypyridin-3-yl)methyl) amino)-1-methylcyclobutan-1-ol (215)

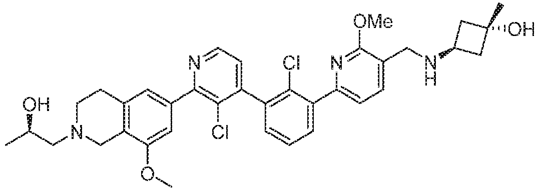

(1r,3r)-3-(((6-(2-Chloro-3-(3-chloro-2-(2-((R)-2-hydroxypropyl)-8-methoxy-1,2,3,4-tetrahydroisoquinolin-6-yl)pyridin-4-yl)phenyl)-2-methoxypyridin-3-yl)methyl) amino)-1-methylcyclobutan-1-ol was prepared in a similar fashion to Example 452, replacing (S)-1-aminopropan-2-ol with (1r,3r)-3-amino-1-methylcyclobutan-1-ol in step (d). MS: m/z found 663 [M+H]⁺; ¹H NMR (400 MHz, Methanol-*d*₄) δ 8.58 (d, 1H), 8.50 (s, 1H), 7.77 (d, 1H), 7.67 (dd, 1H), 7.53 (t, 1H), 7.39 (d, 2H), 7.27 (d, 1H), 7.04 (d, 2H), 4.14 - 4.05 (m, 1H), 4.03 (s, 3H), 3.94 (s, 2H), 3.88 (s, 1H), 3.85 (s, 4H), 3.76 - 3.65 (m, 1H), 3.01 (s, 4H), 2.72 (d, 2H), 2.31 (t, 2H), 2.03 (d, 2H), 1.33 (s, 3H), 1.19 (d, 3H).

### Example 454: (R)-1-(6-(3-Chloro-4-(2-chloro-3-(6-methoxy-5-(((((S)-tetrahydrofuran-2-yl)methyl)amino)methyl)pyridin-2-yl)phenyl)pyridin-2-yl)-8-methoxy-3,4-dihydroisoquinolin-2(1H)-yl)propan-2-ol (216)

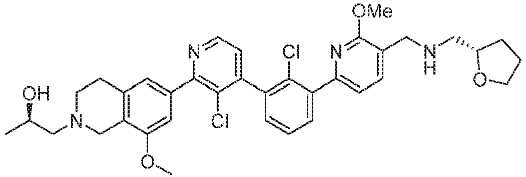

(R)-1-(6-(3-Chloro-4-(2-chloro-3-(6-methoxy-5-(((((S)-tetrahydrofuran-2-yl)methyl)amino)methyl)pyridin-2-yl)phenyl)pyridin-2-yl)-8-methoxy-3,4-dihydroisoquinolin-2(1H)-yl)propan-2-ol was prepared in a similar fashion to Example 452, replacing (S)-1-aminopropan-2-ol with (S)-(tetrahydrofuran-2-yl)methanamine in step (d). MS: m/z found 663 [M+H]⁺; ¹H NMR (400 MHz, Methanol-*d*₄) δ 8.57 (d, 1H), 8.50 (s, 1H), 7.75 (d, 1H), 7.68 (dd, 1H), 7.52 (t, 1H), 7.42 - 7.35 (m, 2H), 7.26 (d, 1H), 7.02 (d, 2H), 4.00 (d, 6H), 3.85 (s, 4H), 3.80 - 3.70 (m, 2H), 2.95 (d, 5H), 2.88 (dd, 1H), 2.77 (t, 1H), 2.64 (s, 2H), 2.11 - 1.98 (m, 1H), 1.96 - 1.83 (m, 2H), 1.62 - 1.48 (m, 1H), 1.18 (d, 3H).

### Example 455: (R)-1-((6-(2-Chloro-3-(3-chloro-2-(2-(2-hydroxypropyl)-8-methoxy-1,2,3,4-tetrahydroisoquinolin-6-yl)pyridin-4-yl)phenyl)-2-methoxypyridin-3-yl)methyl)-3-methylazetidin-3-ol (217)

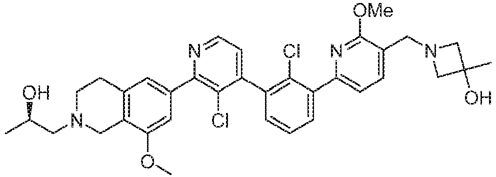

(R)-1-((6-(2-Chloro-3-(3-chloro-2-(2-(2-hydroxypropyl)-8-methoxy-1,2,3,4-tetrahydroisoquinolin-6-yl)pyridin-4-yl)phenyl)-2-methoxypyridin-3-yl)methyl)-3-methylazetidin-3-ol was prepared in a similar fashion to Example 452, replacing (S)-1-aminopropan-2-ol with 3-methylazetidin-3-ol in step (d). MS: m/z found 649 [M+H]⁺; ¹H NMR (400 MHz, Methanol-*d*₄) δ 8.59 (d, 1H), 8.44 (s, 1H), 7.75 (d, 1H), 7.68 (dd, 1H), 7.52 (t, 1H), 7.44 - 7.35 (m, 2H), 7.28 (d, 1H), 7.10 (s, 2H), 4.20 - 4.15 (m, 3H), 4.08 (s, 3H), 4.00 (s, 3H), 3.88 (s, 3H), 3.78 (d, 3H), 3.64 - 3.59 (m, 3H), 3.11 (s, 2H), 3.00 - 2.92 (m, 2H), 1.48 (s, 3H), 1.22 (d, 3H).

### Example 456: Methyl (R)-((6-(2-chloro-3-(3-chloro-2-(2-(2-hydroxypropyl)-8-methoxy-1,2,3,4-tetrahydroisoquinolin-6-yl)pyridin-4-yl)phenyl)-2-methoxypyridin-3-yl)methyl)glycinate (218)

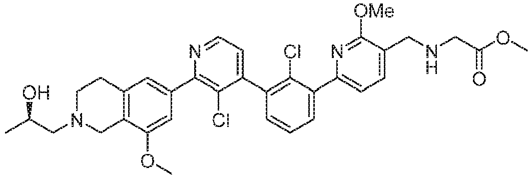

Methyl (R)-((6-(2-chloro-3-(3-chloro-2-(2-(2-hydroxypropyl)-8-methoxy-1,2,3,4-tetrahydroisoquinolin-6-yl)pyridin-4-yl)phenyl)-2-methoxypyridin-3-yl)methyl)glycinate was prepared in a similar fashion to Example 452, replacing (S)-1-aminopropan-2-ol with methyl glycinate in step (d). MS: m/z found 651 [M+H]⁺; ¹H NMR (400 MHz, Methanol-*d*₄) δ 8.57 (d, 1H), 8.50 (s, 1H), 7.68 (t, 2H), 7.51 (t, 1H), 7.42 - 7.33 (m, 2H), 7.21 (d, 1H), 7.03 (d, 2H), 4.13 - 4.03 (m, 1H), 3.98 (s, 3H), 3.82 (d, 7H), 3.67 (s, 3H), 3.41 (s, 2H), 2.98 (s, 4H), 2.66 (s, 2H), 1.18 (d, 3H).

### Example 457: (R)-((6-(2-Chloro-3-(3-chloro-2-(2-(2-hydroxypropyl)-8-methoxy-1,2,3,4-tetrahydroisoquinolin-6-yl)pyridin-4-yl)phenyl)-2-methoxypyridin-3-yl)methyl)glycine (229)

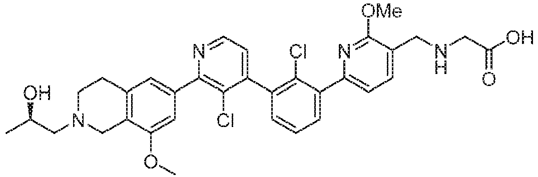

### (a) (R)-((6-(2-Chloro-3-(3-chloro-2-(2-(2-hydroxypropyl)-8-methoxy-1,2,3,4-tetrahydroisoquinolin-6-yl)pyridin-4-yl)phenyl)-2-methoxypyridin-3-yl)methyl)glycine

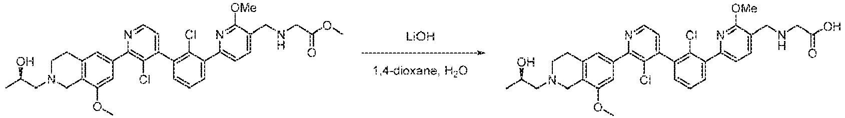

A vial was charged with methyl (R)-((6-(2-chloro-3-(3-chloro-2-(2-(2-hydroxypropyl)-8-methoxy-1,2,3,4-tetrahydroisoquinolin-6-yl)pyridin-4-yl)phenyl)-2-methoxypyridin-3-yl)methyl)glycinate (Example 456) (0.01 g, 0.02 mmol), then 1,4-dioxane (1.5 ml) was added. Separately, another vial was charged with lithium hydroxide monohydrate (0.002 g, 0.05 mmol), which was then dissolved in water (0.5 ml). Solutions were combined, and stirred at room temperature for 1 hr, then purified directly by reverse phase HPLC (with 0.05% Formic acid modifier to give (R)-((6-(2-chloro-3-(3-chloro-2-(2-(2-hydroxypropyl)-8-methoxy-1,2,3,4-tetrahydroisoquinolin-6-yl)pyridin-4-yl)phenyl)-2-methoxypyridin-3-yl)methyl)glycine (8 mg, 50 % yield) as a white solid (formic acid salt). MS: m/z found 637 [M+H]⁺; ¹H NMR (400 MHz, Methanol-*d*₄) δ 8.59 (d, 1H), 8.49 (s, 1H), 7.84 (d, 1H), 7.70 (dd, 1H), 7.54 (t, 1H), 7.44 - 7.37 (m, 2H), 7.31 (d, 1H), 7.07 (d, 2H), 4.26 (s, 2H), 4.19 - 4.09 (m, 1H), 4.06 (s, 3H), 4.04 - 3.92 (m, 2H), 3.87 (s, 3H), 3.51 (s, 2H), 3.20 - 3.11 (m, 2H), 3.06 (s, 2H), 2.90 - 2.81 (m, 2H), 1.21 (d, 3H).

### Example 458: 1-(6-((6-(2-Chloro-3-(3-chloro-2-(2-(3-fluoropropyl)-8-methoxy-1,2,3,4-tetrahydroisoquinolin-6-yl)pyridin-4-yl)phenyl)-2-methoxypyridin-3-yl)methyl)-2,6-diazaspiro[3.3]heptan-2-yl)ethan-1-one (249)

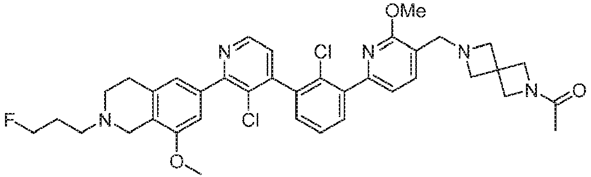

### (a) 6-Bromo-2-(3-fluoropropyl)-8-methoxy-1,2,3,4-tetrahydroisoquinoline

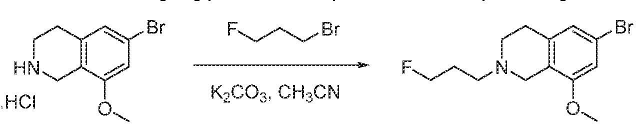

A scintillation vial was charged with 6-bromo-8-methoxy-1,2,3,4-tetrahydroisoquinoline hydrochloride (0.5 g, 1.79 mmol), 1-bromo-3-fluoropropane (0.73 g, 3.59 mmol), and potassium carbonate (0.74 ml, 5.38 mmol). Acetonitrile (15 ml) was then added, and the reaction was stirred at room temperature for 2 hr. The mixture was filtered through CELITE^{®} and the filter cake was washed with ethyl acetate (3 x 40 mL). The filtrate was concentrated under reduced pressure. The residue was purified by normal phase flash SiO₂ chromatography using a gradient (0-60% ethyl acetate/hexanes) to afford 6-bromo-2-(3-fluoropropyl)-8-methoxy-1,2,3,4-tetrahydroisoquinoline (0.41 g, 74 % yield). MS: m/z found 302 [M+H]⁺.

### (b) 2-(3-Fluoropropyl)-8-methoxy-6-(4,4,5,5-tetramethyl-1,3,2-dioxaborolan-2-yl)-1,2,3,4-tetrahydroisoquinoline

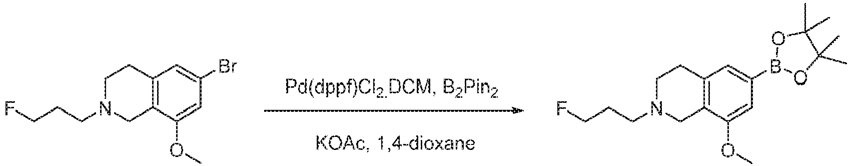

A microwave vial was charged with 6-bromo-2-(3-fluoropropyl)-8-methoxy-1,2,3,4-tetrahydroisoquinoline (0.41 g, 1.36 mmol), bis(pinacolato)diborane (0.38 g, 1.49 mmol), potassium acetate (0.37 g, 3.80 mmol), and [1,1'- bis(diphenylphosphino)ferrocene]-dichloropalladium(II) complex with dichloromethane (0.08 g, 0.09 mmol). The flask was purged with nitrogen for 5 min, then dry 1,4-dioxane (2 mL) was added, and the reaction was sparged with nitrogen for 5 min. The mixture was stirred at 90 °C for 1 hr thermally. The mixture was filtered through CELITE^{®} and the filter cake was washed with ethyl acetate (3 x 40 mL). The filtrate was concentrated under reduced pressure. The residue was purified by normal phase flash SiO₂ chromatography using a gradient (0-10% methanol / CH₂Cl₂) to afford 2-(3-fluoropropyl)-8-methoxy-6-(4,4,5,5-tetramethyl-1,3,2-dioxaborolan-2-yl)-1,2,3,4-tetrahydroisoquinoline (0.45 g, 96% yield). MS: m/z found 350 [M+H]⁺.

### (c) 6-(2-Chloro-3-(3-chloro-2-(2-(3-fluoropropyl)-8-methoxy-1,2,3,4-tetrahydroisoquinolin-6-yl)pyridin-4-yl)phenyl)-2-methoxynicotinaldehyde

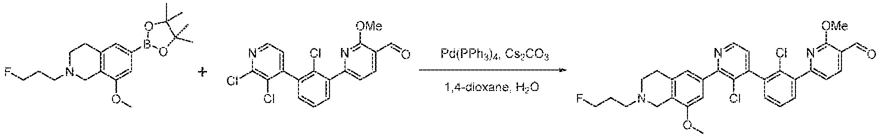

A microwave vial was charged with 2-(3-fluoropropyl)-8-methoxy-6-(4,4,5,5-tetramethyl-1,3,2-dioxaborolan-2-yl)-1,2,3,4-tetrahydroisoquinoline (0.25 g, 0.72 mmol), 6-(2-chloro-3-(2,3-dichloropyridin-4-yl)phenyl)-2-methoxynicotinaldehyde (0.28 g, 0.72 mmol), cesium carbonate (0.70 g, 2.15 mmol), and tetrakis(triphenylphosphine)palladium(0) (0.06 g, 0.05 mmol). 1,4-Dioxane (2 ml) was added, and the reaction was sparged with nitrogen, then water (0.3 ml) added. The mixture was stirred at 90 °C for 1 hr thermally. The mixture was filtered through CELITE^{®} and the filter cake was washed with ethyl acetate (3 x 15 mL). The filtrate was concentrated under reduced pressure. The residue was purified by normal phase flash SiO₂ chromatography using a gradient (0-5 % methanol / CH₂Cl₂) to afford 6-(2-chloro-3-(3-chloro-2-(2-(3-fluoropropyl)-8-methoxy-1,2,3,4-tetrahydroisoquinolin-6-yl)pyridin-4-yl)phenyl)-2-methoxynicotinaldehyde (0.41 g, 100 % yield). MS: m/z found 580 [M+H]⁺.

### (d) 1-(6-((6-(2-Chloro-3-(3-chloro-2-(2-(3-fluoropropyl)-8-methoxy-1,2,3,4-tetrahydroisoquinolin-6-yl)pyridin-4-yl)phenyl)-2-methoxypyridin-3-yl)methyl)-2,6-diazaspiro[3.3]heptan-2-yl)ethan-1-one

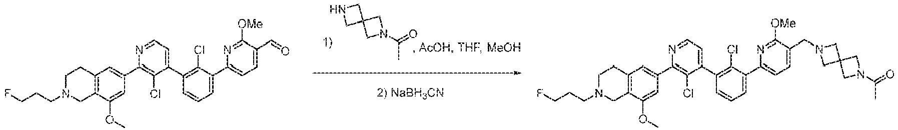

To a mixture of 6-(2-chloro-3-(3-chloro-2-(2-(3-fluoropropyl)-8-methoxy-1,2,3,4-tetrahydroisoquinolin-6-yl)pyridin-4-yl)phenyl)-2-methoxynicotinaldehyde (0.08 g, 0.14 mmol), and 1-(2,6-diazaspiro[3.3]heptan-2-yl)ethan-1-one (0.05 g, 0.28 mmol), in 1:1 THF/MeOH (6 mL) was added acetic acid (0.02 g, 0.28 mmol) and 4 A molecular sieve (1 g). The mixture was stirred at room temperature for 3 hrs. Sodium cyanoborohydride (0.02 g, 0.34 mmol) was added and the mixture was stirred at room temperature for 1 hr. The reaction was quenched by addition of water (1 mL). The mixture was diluted with ethyl acetate (50 ml), washed with saturated aqueous brine solution (30 mL), then the aqueous layer extracted with ethyl acetate (3 x 30 mL), the combined organic phases were dried over sodium sulfate, filtered and concentrated under reduced pressure. The residue was purified by reverse phase HPLC (with 0.05% Formic acid modifier) to give 1-(6-((6-(2-chloro-3-(3-chloro-2-(2-(3-fluoropropyl)-8-methoxy-1,2,3,4-tetrahydroisoquinolin-6-yl)pyridin-4-yl)phenyl)-2-methoxypyridin-3-yl)methyl)-2,6-diazaspiro[3.3]heptan-2-yl)ethan-1-one (7.5 mg, 8 % yield) as a white solid (formic acid salt). MS: m/z found 704 [M+H]⁺. ¹H NMR (400 MHz, Methanol-*d*₄) δ 8.58 (d, 1H), 8.43 (s, 1H), 7.72 (d, 1H), 7.67 (dd, 1H), 7.52 (t, 1H), 7.43 - 7.34 (m, 2H), 7.25 (d, 1H), 7.08 (d, 2H), 4.87 (s, 1H), 4.60 (t, 1H), 4.48 (t, 1H), 4.30 (s, 2H), 4.05 (s, 2H), 4.02 - 3.90 (m, 7H), 3.89 - 3.73 (m, 7H), 3.14 - 2.98 (m, 6H), 2.19 - 2.01 (m, 1H), 1.81 (s, 3H).

### Example 459: 2-((6-(2-Chloro-3-(3-chloro-2-(2-(3-fluoropropyl)-8-methoxy-1,2,3,4-tetrahydroisoquinolin-6-yl)pyridin-4-yl)phenyl)-2-methoxypyridin-3-yl)methyl)-2,6-diazaspiro[3.4]octan-7-one (250)

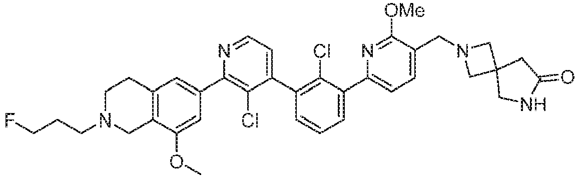

2-((6-(2-Chloro-3-(3-chloro-2-(2-(3-fluoropropyl)-8-methoxy-1,2,3,4-tetrahydroisoquinolin-6-yl)pyridin-4-yl)phenyl)-2-methoxypyridin-3-yl)methyl)-2,6-diazaspiro[3.4]octan-7-one was prepared in a similar fashion to Example 458 replacing 1-(2,6-diazaspiro[3.3]heptan-2-yl)ethan-1-one with 2,6-diazaspiro[3.4]octan-7-one in step (d). MS: m/z found 690 [M+H]⁺. ¹H NMR (400 MHz, Methanol-*d*₄) δ 8.59 (d, 1H), 8.40 (s, 1H), 7.75 (d, 1H), 7.67 (dd, 1H), 7.52 (t, 1H), 7.43 - 7.34 (m, 2H), 7.26 (d, 1H), 7.09 (s, 2H), 4.61 (t, 1H), 4.49 (t, 1H), 4.06 - 3.97 (m, 7H), 3.88 (s, 3H), 3.76 (s, 4H), 3.60 (s, 2H), 3.20 (t, 2H), 3.15 - 3.06 (m, 4H), 2.61 (s, 2H), 2.22 - 2.04 (m, 2H).

### Example 460: N-((6-(2-Chloro-3-(3-chloro-2-(2-(3-fluoropropyl)-8-methoxy-1,2,3,4-tetrahydroisoquinolin-6-yl)pyridin-4-yl)phenyl)-2-methoxypyridin-3-yl)methyl)-2-oxaspiro[3.3]heptan-6-amine (251)

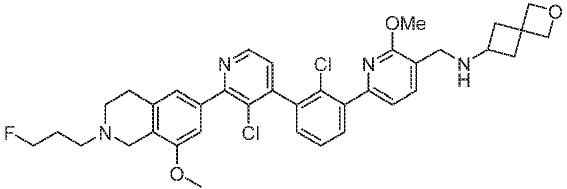

N-((6-(2-Chloro-3-(3-chloro-2-(2-(3-fluoropropyl)-8-methoxy-1,2,3,4-tetrahydroisoquinolin-6-yl)pyridin-4-yl)phenyl)-2-methoxypyridin-3-yl)methyl)-2-oxaspiro[3.3]heptan-6-amine was prepared in a similar fashion to Example 458 replacing 1-(2,6-diazaspiro[3.3]heptan-2-yl)ethan-1-one with 2-oxaspiro[3.3]heptan-6-amine in step (d). MS: m/z found 677 [M+H]⁺. ¹H NMR (400 MHz, Methanol-*d*₄) δ 8.58 (d, 1H), 8.48 (s, 1H), 7.77 (d, 1H), 7.67 (dd, 1H), 7.53 (t, 1H), 7.43 - 7.34 (m, 2H), 7.27 (d, 1H), 7.06 - 7.02 (m, 2H), 4.69 (s, 2H), 4.59 (d, 3H), 4.46 (t, 1H), 4.02 (s, 3H), 3.82 (d, 6H), 3.53 - 3.40 (m, 1H), 2.99 (d, 2H), 2.96 - 2.88 (m, 2H), 2.84 (t, 3H), 2.61 (t, 2H), 2.21 (t, 2H), 2.13 - 1.95 (m, 1H).

### Example 461: (1-((((6-(2-Chloro-3-(3-chloro-2-(2-(3-fluoropropyl)-8-methoxy-1,2,3,4-tetrahydroisoquinolin-6-yl)pyridin-4-yl)phenyl)-2-methoxypyridin-3-yl)methyl)amino) methyl)cyclopropyl)methanol (252)

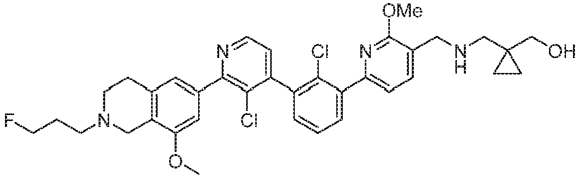

(1-((((6-(2-Chloro-3-(3-chloro-2-(2-(3-fluoropropyl)-8-methoxy-1,2,3,4-tetrahydroisoquinolin-6-yl)pyridin-4-yl)phenyl)-2-methoxypyridin-3-yl)methyl)amino) methyl)cyclopropyl)methanol was prepared in a similar fashion to Example 458 replacing 1-(2,6-diazaspiro[3.3]heptan-2-yl)ethan-1-one with (1-(aminomethyl)cyclopropyl)methanol in step (d). MS: m/z found 665 [M+H]⁺. ¹H NMR (400 MHz, Methanol-*d*₄) δ 8.58 (d, 1H), 8.49 (s, 1H), 7.81 (d, 1H), 7.69 (dd, 1H), 7.54 (t, 1H), 7.44 - 7.36 (m, 2H), 7.32 (d, 1H), 7.03 (d, 2H), 4.57 (t, 1H), 4.46 (t, 1H), 4.21 (s, 2H), 4.05 (s, 3H), 3.85 (s, 3H), 3.72 (s, 2H), 3.55 (s, 2H), 3.13 (s, 2H), 2.98 (t, 2H), 2.87 (t, 2H), 2.79 (t, 2H), 2.12 - 1.94 (m, 2H), 0.64 (d, 4H).

### Example 462: Methyl (S)-3-(6-(3-chloro-4-(2-chloro-3-(5-(((2-hydroxypropyl)amino) methyl)-6-methoxypyridin-2-yl)phenyl)pyridin-2-yl)-8-methoxy-3,4-dihydroisoquinolin-2(1H)-yl)propanoate (272)

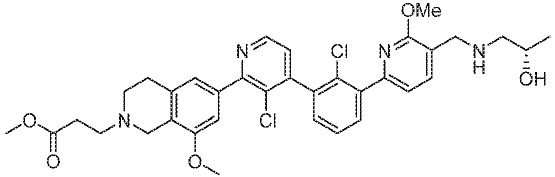

### (a) Methyl 3-(6-bromo-8-methoxy-3,4-dihydroisoquinolin-2(1H)-yl)propanoate

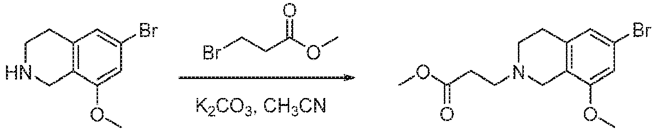

A scintillation vial was charged with 6-bromo-8-methoxy-1,2,3,4-tetrahydroisoquinoline hydrochloride (0.05 g, 0.18 mmol), methyl 3-bromopropanoate (0.04 g, 0.27 mmol), and potassium carbonate (0.07 ml, 0.54 mmol). Acetonitrile (15 ml) was then added, and the reaction was stirred at room temperature for 2 hr. The mixture was filtered through CELITE^{®} and the filter cake was washed with ethyl acetate (3 x 40 mL). The filtrate was concentrated under reduced pressure. The residue was purified by normal phase flash SiO₂ chromatography using a gradient (0-60% ethyl acetate/hexanes) to afford methyl 3-(6-bromo-8-methoxy-3,4-dihydroisoquinolin-2(1H)-yl)propanoate (0.16 g, 75 % yield). MS: m/z found 328 [M+H]⁺.

### (b) Methyl 3-(8-methoxy-6-(4,4,5,5-tetramethyl-1,3,2-dioxaborolan-2-yl)-3,4-dihydroisoquinolin-2(1H)-yl)propanoate

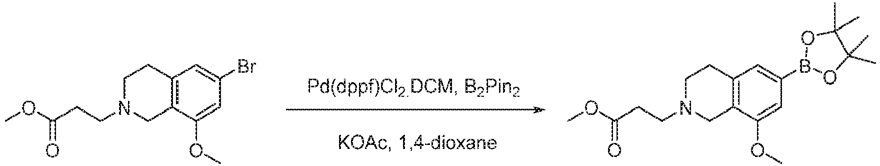

A microwave vial was charged with methyl 3-(6-bromo-8-methoxy-3,4-dihydroisoquinolin-2(1H)-yl)propanoate (0.16 g, 0.49 mmol), bis(pinacolato)diborane (0.14 g, 0.54 mmol), potassium acetate (0.13g, 1.37 mmol), and [1,1'-bis(diphenylphosphino)ferrocene]-dichloropalladium(II) complex with dichloromethane (0.04 g, 0.05 mmol). The flask was purged with nitrogen for 5 min, then dry 1,4-dioxane (2 mL) was added, and the reaction was sparged with nitrogen for 5 min. The mixture was stirred at 90 °C for 1 hr thermally. The mixture was filtered through CELITE^{®} and the filter cake was washed with ethyl acetate (3 x 40 mL). The filtrate was concentrated under reduced pressure and the residue was purified by normal phase flash SiO₂ chromatography using a gradient (0-5% methanol / CH₂Cl₂) to afford methyl 3-(8-methoxy-6-(4,4,5,5-tetramethyl-1,3,2-dioxaborolan-2-yl)-3,4-dihydroisoquinolin-2(1H)-yl)propanoate (0.16g, 88 % yield). MS: m/z found 376 [M+H]⁺.

### (c) Methyl 3-(6-(3-chloro-4-(2-chloro-3-(5-formyl-6-methoxypyridin-2-yl)phenyl)pyridin-2-yl)-8-methoxy-3,4-dihydroisoquinolin-2(1H)-yl)propanoate

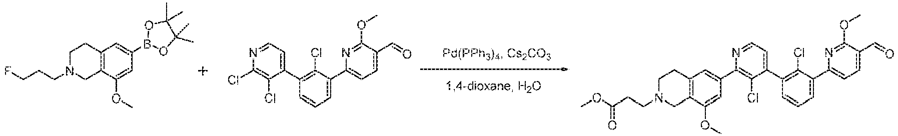

A microwave vial was charged with methyl 3-(8-methoxy-6-(4,4,5,5-tetramethyl-1,3,2-dioxaborolan-2-yl)-3,4-dihydroisoquinolin-2(1H)-yl)propanoate (0.15 g, 0.40 mmol), 6-(2-chloro-3-(2,3-dichloropyridin-4-yl)phenyl)-2-methoxynicotinaldehyde (0.16 g, 0.40 mmol), cesium carbonate (0.39 g, 1.20 mmol), and tetrakis(triphenylphosphine)palladium(0) (0.05 g, 0.04 mmol). 1,4-Dioxane (2 mL) was added, and the reaction was sparged with nitrogen, then water (0.3 ml) added. The mixture was stirred at 90 °C for 1 hr thermally. The mixture was filtered through CELITE^{®} and the filter cake was washed with ethyl acetate (3 x 15 mL). The filtrate was concentrated under reduced pressure. The residue was purified by normal phase flash SiO₂ chromatography using a gradient (0-5 % methanol / CH₂Cl₂) to afford methyl 3-(6-(3-chloro-4-(2-chloro-3-(5-formyl-6-methoxypyridin-2-yl)phenyl)pyridin-2-yl)-8-methoxy-3,4-dihydroisoquinolin-2(1H)-yl)propanoate (0.24 g, 100 % yield). MS: m/z found 606 [M+H]⁺.

### (d) Methyl (S)-3-(6-(3-chloro-4-(2-chloro-3-(5-(((2-hydroxypropyl)amino)methyl)-6-methoxypyridin-2-yl)phenyl)pyridin-2-yl)-8-methoxy-3,4-dihydroisoquinolin-2(1H)-yl)propanoate

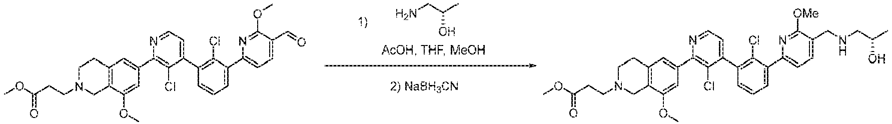

To a mixture of methyl 3-(6-(3-chloro-4-(2-chloro-3-(5-formyl-6-methoxypyridin-2-yl)phenyl)pyridin-2-yl)-8-methoxy-3,4-dihydroisoquinolin-2(1H)-yl)propanoate (0.12 g, 0.20 mmol), and (S)-1-aminopropan-2-ol (0.03 g, 0.40 mmol), in 1:1 THF/MeOH (6 mL) was added acetic acid (0.02 g, 0.40 mmol) and 4 A molecular sieve (1 g). The mixture was stirred at room temperature for 3 hrs. Sodium cyanoborohydride (0.03 g, 0.49 mmol) was added and the mixture was stirred at room temperature for 1 hr. The reaction was quenched by addition of water (1 ml). The mixture was diluted with ethyl acetate (50 ml), then washed with saturated aqueous brine solution (30 mL), then aqueous layer extracted with ethyl acetate (3 x 30 mL), and the combined organic phases were dried over sodium sulfate, filtered and concentrated under reduced pressure. The residue was purified by reverse phase HPLC (with 0.05% Formic acid modifier) to give methyl (S)-3-(6-(3-chloro-4-(2-chloro-3-(5-(((2-hydroxypropyl)amino)methyl)-6-methoxypyridin-2-yl)phenyl)pyridin-2-yl)-8-methoxy-3,4-dihydroisoquinolin-2(1H)-yl)propanoate (20 mg, 15 % yield) as a white solid (formic acid salt). MS: m/z found 665 [M+H]⁺. ¹H NMR (400 MHz, Methanol-*d*₄) δ 8.57 (d, 1H), 7.76 - 7.64 (m, 2H), 7.52 (t, 1H), 7.42 - 7.34 (m, 2H), 7.25 (d, 1H), 7.01 (d, 2H), 4.87 (s, 1H), 4.01 (s, 3H), 3.99 - 3.83 (m, 6H), 3.65 (d, 5H), 2.98 - 2.86 (m, 4H), 2.77 (t, 2H), 2.72 - 2.54 (m, 3H), 1.16 (d, 3H).

### Example 463: Methyl 3-(6-(3-chloro-4-(2-chloro-3-(6-methoxy-5-((7-oxo-2,6-diazaspiro[3.4]octan-2-yl)methyl)pyridin-2-yl)phenyl)pyridin-2-yl)-8-methoxy-3,4-dihydroisoquinolin-2(1H)-yl)propanoate (273)

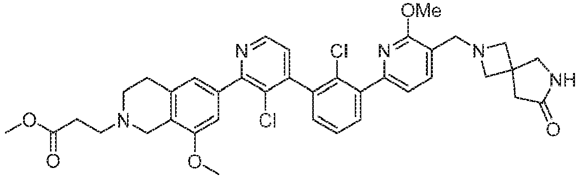

### (a) Methyl 3-(6-(3-chloro-4-(2-chloro-3-(6-methoxy-5-((7-oxo-2,6-diazaspiro [3.4] octan-2-yl)methyl)pyridin-2-yl)phenyl)pyridin-2-yl)-8-methoxy-3,4-dihydroisoquinolin-2(1H)-yl)propanoate

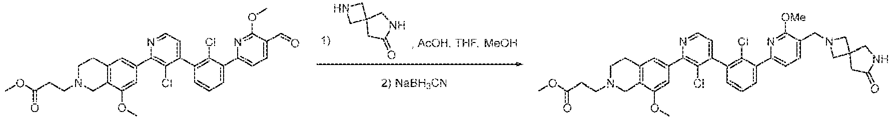

Methyl 3-(6-(3-chloro-4-(2-chloro-3-(6-methoxy-5-((7-oxo-2,6-diazaspiro[3.4]octan-2-yl)methyl)pyridin-2-yl)phenyl)pyridin-2-yl)-8-methoxy-3,4-dihydroisoquinolin-2(1H)-yl)propanoate was prepared in a similar fashion to Example 462 replacing (S)-1-aminopropan-2-ol with 2,6-diazaspiro[3.4]octan-7-one in step (d). MS: m/z found 716 [M+H]⁺. ¹H NMR (400 MHz, Methanol-*d*₄) δ 8.57 (d, 1H), 8.50 (s, 1H), 7.71 - 7.63 (m, 2H), 7.51 (t, 1H), 7.38 (t, 2H), 7.22 (d, 1H), 7.02 (d, 2H), 3.97 (s, 3H), 3.86 (s, 3H), 3.73 (s, 2H), 3.67 (d, 5H), 3.56 (s, 2H), 3.45 (s, 4H), 2.92 (d, 2H), 2.80 (t, 2H), 2.66 (t, 2H), 2.56 (s, 2H).

### Example 464: (S)-3-(6-(3-Chloro-4-(2-chloro-3-(5-(((2-hydroxypropyl)amino)methyl)-6-methoxypyridin-2-yl)phenyl)pyridin-2-yl)-8-methoxy-3,4-dihydroisoquinolin-2(1H)-yl)propanoic acid (293)

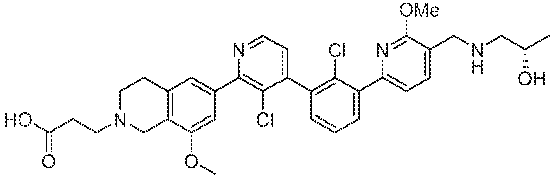

### (a) (S)-3-(6-(3-Chloro-4-(2-chloro-3-(5-(((2-hydroxypropyl)amino)methyl)-6-methoxypyridin-2-yl)phenyl)pyridin-2-yl)-8-methoxy-3,4-dihydroisoquinolin-2(1H)-yl)propanoic acid

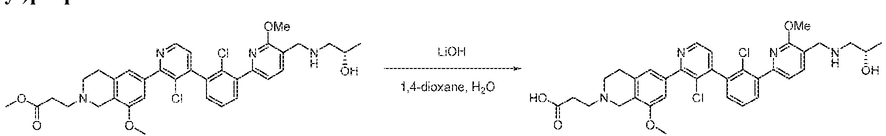

A vial was charged with methyl (S)-3-(6-(3-chloro-4-(2-chloro-3-(5-(((2-hydroxypropyl)amino)methyl)-6-methoxypyridin-2-yl)phenyl)pyridin-2-yl)-8-methoxy-3,4-dihydroisoquinolin-2(1H)-yl)propanoate (Example 462) (0.01 g, 0.02 mmol), then 1,4-dioxane (1.5 ml) was added. Separately, another vial was charged with lithium hydroxide monohydrate (0.002 g, 0.05 mmol), which was then dissolved in water (0.5 ml). The solutions were combined, and stirred at room temperature for 1 hr, then purified directly by reverse phase HPLC (with 0.05% Formic acid modifier) to give (S)-3-(6-(3-chloro-4-(2-chloro-3-(5-(((2-hydroxypropyl)amino)methyl)-6-methoxypyridin-2-yl)phenyl)pyridin-2-yl)-8-methoxy-3,4-dihydroisoquinolin-2(1H)-yl)propanoic acid (8 mg, 50 % yield) as a white solid (formic acid salt). MS: m/z found 651 [M+H]⁺. ¹H NMR (400 MHz, Methanol-*d*₄) δ 8.59 (d, 1H), 8.50 (s, 1H), 7.78 (d, 1H), 7.68 (dd, 1H), 7.53 (t, 1H), 7.43 - 7.35 (m, 2H), 7.28 (d, 1H), 7.10 (d, 2H), 4.12 (s, 2H), 4.07 (d, 2H), 4.02 - 3.91 (m, 1H), 3.88 (s, 3H), 3.27 (m, 6H), 3.11 (t, 2H), 2.88 (dd, 1H), 2.72 (t, 1H), 2.60 (t, 2H), 1.18 (dd, 3H).

### Example 465: 3-(6-(3-Chloro-4-(2-chloro-3-(6-methoxy-5-((7-oxo-2,6-diazaspiro [3.4] octan-2-yl)methyl)pyridin-2-yl)phenyl)pyridin-2-yl)-8-methoxy-3,4-dihydroisoquinolin-2(1H)-yl)propanoic acid (294)

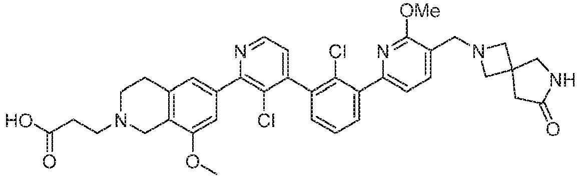

3-(6-(3-Chloro-4-(2-chloro-3-(6-methoxy-5-((7-oxo-2,6-diazaspiro[3.4]octan-2-yl)methyl)pyridin-2-yl)phenyl)pyridin-2-yl)-8-methoxy-3,4-dihydroisoquinolin-2(1H)-yl)propanoic acid was prepared in a similar fashion to Example 464 replacing methyl (S)-3-(6-(3-chloro-4-(2-chloro-3-(5-(((2-hydroxypropyl)amino)methyl)-6-methoxypyridin-2-yl)phenyl)pyridin-2-yl)-8-methoxy-3,4-dihydroisoquinolin-2(1H)-yl)propanoate with methyl 3-(6-(3-chloro-4-(2-chloro-3-(6-methoxy-5-((7-oxo-2,6-diazaspiro[3.4]octan-2-yl)methyl)pyridin-2-yl)phenyl)pyridin-2-yl)-8-methoxy-3,4-dihydroisoquinolin-2(1H)-yl)propanoate (Example 463) in step (a). MS: m/z found 702 [M+H]⁺. ¹H NMR (400 MHz, Methanol-*d*₄) δ 8.60 (d, 1H), 8.45 (s, 1H), 7.74 - 7.64 (m, 2H), 7.52 (t, 1H), 7.45 - 7.34 (m, 2H), 7.24 (d, 1H), 7.14 (s, 2H), 4.29 (s, 2H), 3.99 (s, 3H), 3.88 (d, 5H), 3.59 (d, 6H), 3.50 - 3.36 (m, 4H), 3.17 (t, 2H), 2.69 - 2.56 (m, 4H).

### Example 466: (S)-2-(6-(3-Chloro-4-(2-chloro-3-(5-(((2-hydroxypropyl)amino)methyl)-6-methoxypyridin-2-yl)phenyl)pyridin-2-yl)-8-methoxy-3,4-dihydroisoquinolin-2(1H)-yl)acetamide (275)

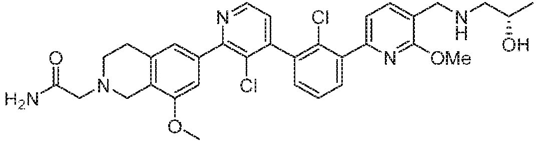

(S)-2-(6-(3-Chloro-4-(2-chloro-3-(5-(((2-hydroxypropyl)amino)methyl)-6-methoxypyridin-2-yl)phenyl)pyridin-2-yl)-8-methoxy-3,4-dihydroisoquinolin-2(1H)-yl)acetamide was prepared in a similar fashion to Example 462, using 6-bromo-8-methoxy-1,2,3,4-tetrahydroisoquinoline hydrochloride and 2-bromoacetamide in step (a), and (S)-1-aminopropan-2-ol in step (d). MS: m/z found 636 [M+H]⁺. ¹H NMR (400 MHz, Methanol-*d*₄) δ 8.58 (d, 1H), 8.52 (s, 1H), 7.79 (d, 1H), 7.69 (dd, 1H), 7.53 (t, 1H), 7.44 - 7.36 (m, 2H), 7.29 (d, 1H), 7.02 (d, 2H), 4.87 (s, 3H), 4.04 (s, 4H), 3.84 (s, 3H), 3.69 (s, 2H), 3.23 (s, 2H), 2.97 (t, 2H), 2.88 (dd, 1H), 2.82 (t, 2H), 2.72 (t, 1H), 1.19 (d, 3H).

### Example 467: Methyl 1-((6-(3-chloro-4-(2-chloro-3-(6-methoxy-5-((7-oxo-2,6-diazaspiro [3.4]octan-2-yl)methyl)pyridin-2-yl)phenyl)pyridin-2-yl)-8-methoxy-3,4-dihydroisoquinolin -2(1H)-yl)methyl)cyclopropane-1-carboxylate (295)

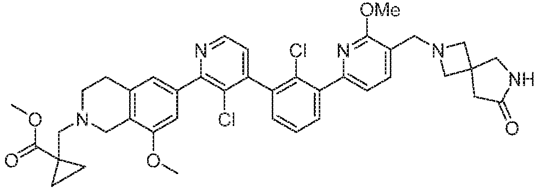

Methyl 1-((6-(3-chloro-4-(2-chloro-3-(6-methoxy-5-((7-oxo-2,6-diazaspiro [3.4]octan-2-yl)methyl)pyridin-2-yl)phenyl)pyridin-2-yl)-8-methoxy-3,4-dihydroisoquinolin -2(1H)-yl)methyl)cyclopropane-1-carboxylate was prepared in a similar fashion to Example 462, using 6-bromo-8-methoxy-1,2,3,4-tetrahydroisoquinoline hydrochloride and methyl 1-(bromomethyl)cyclopropane-1-carboxylate in step (a), and 2,6-diazaspiro[3.4]octan-7-one in step (d). MS: m/z found 742 [M+H]⁺. ¹H NMR (400 MHz, Methanol-*d*₄) δ 8.60 (d, 1H), 8.40 (s, 1H), 7.74 (d, 1H), 7.68 (dd, 1H), 7.52 (t, 1H), 7.44 - 7.35 (m, 2H), 7.26 (d, 1H), 7.10 (s, 2H), 4.13 (s, 2H), 4.02 - 3.93 (m, 5H), 3.88 (s, 3H), 3.73 - 3.65 (m, 7H), 3.60 (s, 2H), 3.11 (t, 2H), 2.61 (s, 2H), 1.46 - 1.38 (m, 2H), 1.14 - 1.06 (m, 2H).

### Example 468: 2-((6-(2-Chloro-3-(3-chloro-2-(2-(3-fluoro-2-hydroxypropyl)-8-methoxy-1,2,3,4-tetrahydroisoquinolin-6-yl)pyridin-4-yl)phenyl)-2-methoxypyridin-3-yl)methyl)-2,6-diazaspiro[3.4]octan-7-one (296)

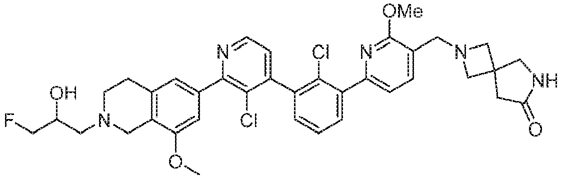

2-((6-(2-Chloro-3-(3-chloro-2-(2-(3-fluoro-2-hydroxypropyl)-8-methoxy-1,2,3,4-tetrahydroisoquinolin-6-yl)pyridin-4-yl)phenyl)-2-methoxypyridin-3-yl)methyl)-2,6-diazaspiro[3.4]octan-7-one was prepared in a similar fashion to Example 462, using 6-bromo-8-methoxy-1,2,3,4-tetrahydroisoquinoline hydrochloride and 1-chloro-3-fluoropropan-2-ol in step (a), and 2,6-diazaspiro[3.4]octan-7-one in step (d). MS: m/z found 706 [M+H]⁺. ¹H NMR (400 MHz, Methanol-*d*₄) δ 8.59 (d, 1H), 8.42 (s, 2H), 7.70 (dd, 2H), 7.52 (t, 1H), 7.44 - 7.35 (m, 2H), 7.26 (d, 1H), 7.07 (d, 2H), 4.55 - 4.41 (m, 1H), 4.43 - 4.29 (m, 1H), 4.27 - 4.13 (m, 1H), 4.01 (d, 5H), 3.94 (s, 2H), 3.87 (s, 3H), 3.69 (s, 4H), 3.59 (s, 2H), 3.18 (t, 2H), 3.11 - 3.04 (m, 2H), 3.05 - 2.91 (m, 2H), 2.61 (s, 2H).

### Example 469: 1-((6-(3-Chloro-4-(2-chloro-3-(6-methoxy-5-((7-oxo-2,6-diazaspiro [3.4] octan-2-yl)methyl)pyridin-2-yl)phenyl)pyridin-2-yl)-8-methoxy-3,4-dihydroisoquinolin-2(1H)-yl)methyl)cyclopropane-1-carboxylic acid (297)

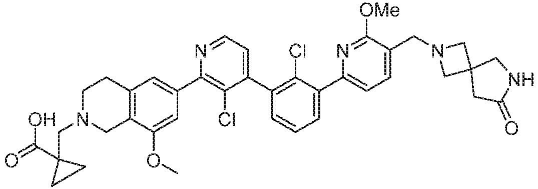

1-((6-(3-Chloro-4-(2-chloro-3-(6-methoxy-5-((7-oxo-2,6-diazaspiro[3.4]octan-2-yl)methyl)pyridin-2-yl)phenyl)pyridin-2-yl)-8-methoxy-3,4-dihydroisoquinolin-2(1H)-yl)methyl)cyclopropane-1-carboxylic acid was prepared in a similar fashion to Example 464 replacing methyl (S)-3-(6-(3-chloro-4-(2-chloro-3-(5-(((2-hydroxypropyl)amino)methyl)-6-methoxypyridin-2-yl)phenyl)pyridin-2-yl)-8-methoxy-3,4-dihydroisoquinolin-2(1H)-yl)propanoate with methyl 1-((6-(3-chloro-4-(2-chloro-3-(6-methoxy-5-((7-oxo-2,6-diazaspiro [3.4]octan-2-yl)methyl)pyridin-2-yl)phenyl)pyridin-2-yl)-8-methoxy-3,4-dihydroisoquinolin -2(1H)-yl)methyl)cyclopropane-1-carboxylate (Example 467) in step (a). MS: m/z found 728 [M+H]⁺. ¹H NMR (400 MHz, Methanol-*d*₄) δ 8.60 (d, 1H), 8.47 (s, 1H), 7.68 (t, 2H), 7.52 (t, 1H), 7.45 - 7.34 (m, 2H), 7.23 (d, 1H), 7.15 (s, 2H), 4.39 (s, 2H), 3.94 (d, 6H), 3.81 (s, 2H), 3.56 (d, 9H), 3.34 - 3.29 (m, 3H), 2.58 (s, 2H), 1.30 (s, 2H), 0.78 (s, 2H).

### Example 470: 2-(3-(6-(4-(3-(5-(((1-Acetylpiperidin-4-yl)amino)methyl)-6-methoxypyridin-2-yl)-2-chlorophenyl)-3-chloropyridin-2-yl)-8-methoxy-3,4-dihydroisoquinolin-2(1H)-yl)-2-oxopyrrolidin-1-yl)acetic acid (480)

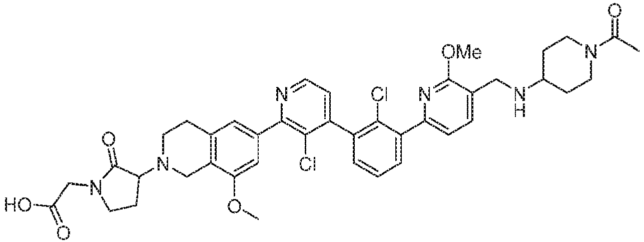

### (a) tert-Butyl 2-(3-(6-bromo-8-methoxy-3,4-dihydroisoquinolin-2(1H)-yl)-2-oxopyrrolidin-1-yl)acetate

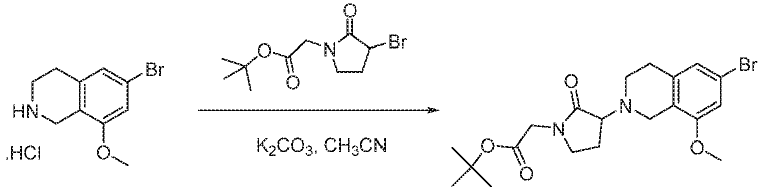

A scintillation vial was charged with 6-bromo-8-methoxy-1,2,3,4-tetrahydroisoquinoline hydrochloride (0.15 g, 0.54 mmol), tert-butyl 2-(3-bromo-2-oxopyrrolidin-1-yl)acetate (0.15 g, 0.54 mmol), and potassium carbonate (0.22 g, 1.62 mmol). Acetonitrile (2 ml) was then added, and the reaction was stirred at 80 °C for 2 hr. The mixture was filtered through CELITE^{®} and the filter cake was washed with ethyl acetate (3 x 40 mL). The filtrate was concentrated under reduced pressure. The residue was purified by normal phase flash SiO₂ chromatography using a gradient (0-5% MeOH/CH₂Cl₂) to afford tert-butyl 2-(3-(6-bromo-8-methoxy-3,4-dihydroisoquinolin-2(1H)-yl)-2-oxopyrrolidin-1-yl)acetate (0.23 g, 100 % yield). MS: m/z found 439 [M+H]⁺.

### (b) tert-Butyl 2-(3-(8-methoxy-6-(4,4,5,5-tetramethyl-1,3,2-dioxaborolan-2-yl)-3,4-dihydroisoquinolin-2(1H)-yl)-2-oxopyrrolidin-1-yl)acetate

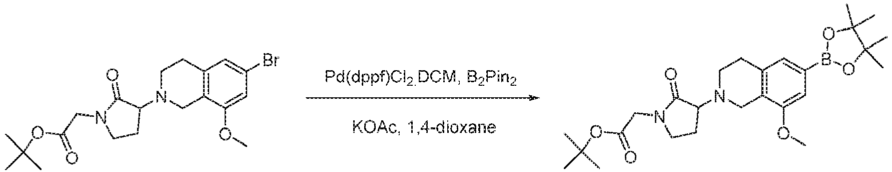

A microwave vial was charged with tert-butyl 2-(3-(6-bromo-8-methoxy-3,4-dihydroisoquinolin-2(1H)-yl)-2-oxopyrrolidin-1-yl)acetate (0.31 g, 0.71 mmol), bis(pinacolato)diborane (0.20 g, 0.78 mmol), potassium acetate (0.19 g, 1.98 mmol), and [1,1'- bis(diphenylphosphino)ferrocene]dichloropalladium(II) complex with dichloromethane (0.06 g, 0.07 mmol). The flask was purged with nitrogen for 5 min, then dry 1,4-dioxane (2 mL) was added, and the reaction was sparged with nitrogen for 5 min. The mixture was stirred at 95 °C for 30 min. The mixture was filtered through CELITE^{®} and the filter cake was washed with ethyl acetate (3 x 40 mL). The filtrate was concentrated under reduced pressure. The residue was purified by normal phase flash SiO₂ chromatography using a gradient (0-5% methanol/CH₂Cl₂) to afford tert-butyl 2-(3-(8-methoxy-6-(4,4,5,5-tetramethyl-1,3,2-dioxaborolan-2-yl)-3,4-dihydroisoquinolin-2(1H)-yl)-2-oxopyrrolidin-1-yl)acetate (0.29 g, 85 % yield). MS: m/z found 487 [M+H]⁺.

### (c) tert-Butyl 2-(3-(6-(3-chloro-4-(2-chloro-3-(5-formyl-6-methoxypyridin-2-yl)phenyl)pyridin-2-yl)-8-methoxy-3,4-dihydroisoquinolin-2(1H)-yl)-2-oxopyrrolidin-1-yl)acetate

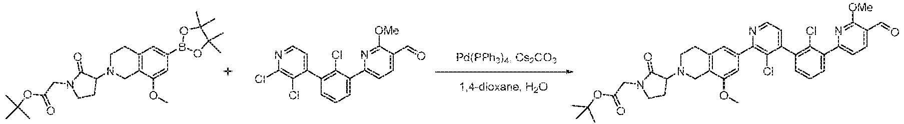

A microwave vial was charged with tert-butyl 2-(3-(8-methoxy-6-(4,4,5,5-tetramethyl-1,3,2-dioxaborolan-2-yl)-3,4-dihydroisoquinolin-2(1H)-yl)-2-oxopyrrolidin-1-yl)acetate (0.29 g, 0.60 mmol), 6-(2-chloro-3-(2,3-dichloropyridin-4-yl)phenyl)-2-methoxynicotinaldehyde (0.23 g, 0.60 mmol), cesium carbonate (0.58 g, 1.79 mmol), and tetrakis(triphenylphosphine)palladium(0) (0.07 g, 0.06 mmol). 1,4-Dioxane (2 ml) was added, the reaction was sparged with nitrogen, then water (0.3 ml) added. The mixture was stirred at 95 °C for 30 min. The mixture was filtered through CELITE^{®} and the filter cake was washed with ethyl acetate (3 x 15 mL). The filtrate was concentrated under reduced pressure. The residue was purified by normal phase flash SiO₂ chromatography using a gradient (0-5 % methanol/CH₂Cl₂) to afford tert-butyl 2-(3-(6-(3-chloro-4-(2-chloro-3-(5-formyl-6-methoxypyridin-2-yl)phenyl)pyridin-2-yl)-8-methoxy-3,4-dihydroisoquinolin-2(1H)-yl)-2-oxopyrrolidin-1-yl)acetate (0.25 g, 59 % yield). MS: m/z found 717 [M+H]⁺.

### (d) tert-Butyl 2-(3-(6-(4-(3-(5-(((1-acetylpiperidin-4-yl)amino)methyl)-6-methoxypyridin-2-yl)-2-chlorophenyl)-3-chloropyridin-2-yl)-8-methoxy-3,4-dihydroisoquinolin-2(1H)-yl)-2-oxopyrrolidin-1-yl)acetate

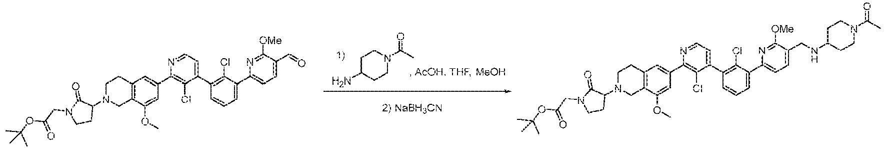

To a mixture of tert-butyl 2-(3-(6-(3-chloro-4-(2-chloro-3-(5-formyl-6-methoxypyridin-2-yl)phenyl)pyridin-2-yl)-8-methoxy-3,4-dihydroisoquinolin-2(1H)-yl)-2-oxopyrrolidin-1-yl)acetate (0.12 g, 0.17 mmol), and 1-(4-aminopiperidin-1-yl)ethan-1-one (0.05 g, 0.33 mmol), in 1:1 THF/MeOH (2 mL) was added acetic acid (0.02 g, 0.33 mmol) and 4 A molecular sieve (1 g). The mixture was stirred at room temperature for 3 hrs. Sodium cyanoborohydride (0.03 g, 0.42 mmol) was added and the mixture was stirred at room temperature for 1 hr, then the reaction quenched by addition of water (1 ml). The mixture was diluted with ethyl acetate (50 ml), washed with saturated aqueous brine solution (30 mL), and the aqueous layer extracted with ethyl acetate (3 x 30 mL). The combined organic phases were dried over sodium sulfate, filtered and concentrated under reduced pressure to give tert-butyl 2-(3-(6-(4-(3-(5-(((1-acetylpiperidin-4-yl)amino)methyl)-6-methoxypyridin-2-yl)-2-chlorophenyl)-3-chloropyridin-2-yl)-8-methoxy-3,4-dihydroisoquinolin-2(1H)-yl)-2-oxopyrrolidin-1-yl)acetate. MS: m/z found 843 [M+H]⁺. The crude product was used in the next step without further purification.

### (e) 2-(3-(6-(4-(3-(5-(((1-Acetylpiperidin-4-yl)amino)methyl)-6-methoxypyridin-2-yl)-2-chlorophenyl)-3-chloropyridin-2-yl)-8-methoxy-3,4-dihydroisoquinolin-2(1H)-yl)-2-oxopyrrolidin-1-yl)acetic acid

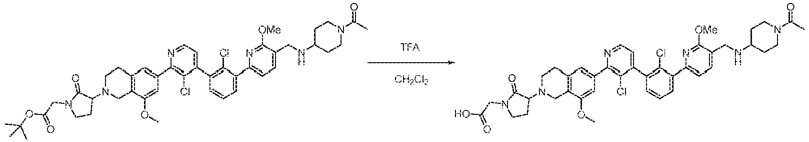

Crude tert-butyl 2-(3-(6-(4-(3-(5-(((1-acetylpiperidin-4-yl)amino)methyl)-6-methoxypyridin-2-yl)-2-chlorophenyl)-3-chloropyridin-2-yl)-8-methoxy-3,4-dihydroisoquinolin-2(1H)-yl)-2-oxopyrrolidin-1-yl)acetate was diluted with dichloromethane (6 ml) and treated with trifluoroacetic acid (2 ml). The reaction mixture was stirred at room temperature for 1 hr, then concentrated under reduced pressure, and the residue purified by reverse phase HPLC (with 0.05% Formic acid modifier) to give 2-(3-(6-(4-(3-(5-(((1-acetylpiperidin-4-yl)amino)methyl)-6-methoxypyridin-2-yl)-2-chlorophenyl)-3-chloropyridin-2-yl)-8-methoxy-3,4-dihydroisoquinolin-2(1H)-yl)-2-oxopyrrolidin-1-yl)acetic acid (23 mg, 81 % yield) as a white solid (formic acid salt). MS: m/z found 787 [M+H]⁺; ¹H NMR (400 MHz, Methanol-*d*₄) δ 8.55 (d, 1H), 8.35 (s, 1H), 7.85 (d, 1H), 7.67 (dd, 1H), 7.52 (t, 1H), 7.42 - 7.33 (m, 2H), 7.31 (d, 1H), 7.03 - 6.96 (m, 2H), 4.63 (d, 1H), 4.24 (s, 2H), 4.12 - 3.95 (m, 5H), 3.88 (t, 1H), 3.85 - 3.73 (m, 5H), 3.55 - 3.35 (m, 2H), 3.27 (q, 2H), 3.23 - 3.08 (m, 2H), 2.90 (d, 3H), 2.67 (t, 1H), 2.31 - 2.14 (m, 3H), 2.10 (s, 3H), 1.67 - 1.41 (m, 2H).

### Example 471: Methyl (S)-1-((6-(3-chloro-4-(2-chloro-3-(6-methoxy-5-((((5-oxopyrrolidin-2-yl)methyl)amino)methyl)pyridin-2-yl)phenyl)pyridin-2-yl)-3-methyl-4-oxo-3,4-dihydropyrrolo[2,1-f][1,2,4] triazin-2-yl)methyl)azetidine-3-carboxylate (182)

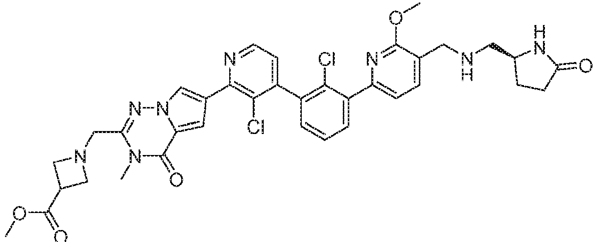

### (a) Methyl (S)-1-((6-(4-(3-(5-(((tert-butoxycarbonyl)((5-oxopyrrolidin-2-yl)methyl)amino)methyl)-6-methoxypyridin-2-yl)-2-chlorophenyl)-3-chloropyridin-2-yl)-3-methyl-4-oxo-3,4-dihydropyrrolo[2,1-f][1,2,4] triazin-2-yl)methyl)azetidine-3-carboxylate

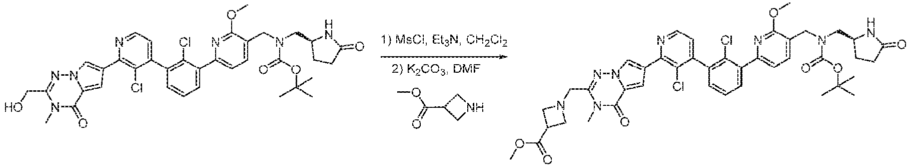

To a solution of tert-butyl (S)-((6-(2-chloro-3-(3-chloro-2-(2-(hydroxymethyl)-3-methyl-4-oxo-3,4-dihydropyrrolo[2,1-f][1,2,4]triazin-6-yl)pyridin-4-yl)phenyl)-2-methoxypyridin-3-yl)methyl)((5-oxopyrrolidin-2-yl)methyl)carbamate (33 mg, 0.04 mmol) in dichloromethane (0.4 mL) was added methanesulfonyl chloride (4 µL, 0.05 mmol) and triethylamine (19 µL, 0.13 mmol). The reaction was stirred at room temperature for 1h. LCMS suggested formation of the desired mesylated product. The solvent was evaporated, and the residue was taken up in DMF (0.4 mL). Potassium carbonate (19 mg, 0.13 mmol) and methyl azetidine-3-carboxylate hydrochloride (20 mg, 0.13 mmol) were added, and the reaction was stirred at room temperature for 12 h. LCMS suggested formation of the desired product. The solvent was evaporated, and the residue was suspended in water. The precipitate formed was collected to afford methyl (S)-1-((6-(4-(3-(5-(((tert-butoxycarbonyl)((5-oxopyrrolidin-2-yl)methyl)amino)methyl)-6-methoxypyridin-2-yl)-2-chlorophenyl)-3-chloropyridin-2-yl)-3-methyl-4-oxo-3,4-dihydropyrrolo[2,1-f][1,2,4]triazin-2-yl)methyl)azetidine-3-carboxylate (31 mg, 83%). The material was used as is without any purification. MS: m/z found 831 [M+H]⁺.

### (b) Methyl (S)-1-((6-(3-chloro-4-(2-chloro-3-(6-methoxy-5-((((5-oxopyrrolidin-2-yl)methyl)amino)methyl)pyridin-2-yl)phenyl)pyridin-2-yl)-3-methyl-4-oxo-3,4-dihydropyrrolo[2,1-f][1,2,4]triazin-2-yl)methyl)azetidine-3-carboxylate

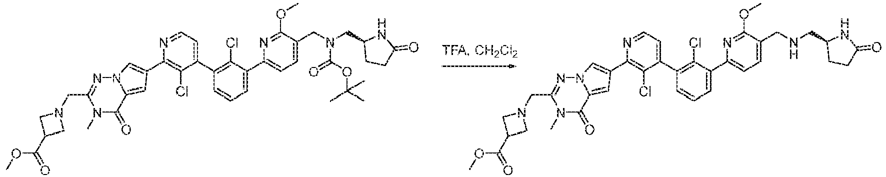

Methyl (S)-1-((6-(3-chloro-4-(2-chloro-3-(6-methoxy-5-((((5-oxopyrrolidin-2-yl)methyl)amino)methyl)pyridin-2-yl)phenyl)pyridin-2-yl)-3-methyl-4-oxo-3,4-dihydropyrrolo[2,1-f][1,2,4]triazin-2-yl)methyl)azetidine-3-carboxylate was prepared using the General Boc Deprotection Procedure from methyl (S)-1-((6-(4-(3-(5-(((tert-butoxycarbonyl)((5-oxopyrrolidin-2-yl)methyl)amino)methyl)-6-methoxypyridin-2-yl)-2-chlorophenyl)-3-chloropyridin-2-yl)-3-methyl-4-oxo-3,4-dihydropyrrolo[2,1-f][1,2,4]triazin-2-yl)methyl)azetidine-3-carboxylate (31 mg, 0.04 mmol) and trifluoroacetic acid (57 µL, 0.75 mmol). 11 mg, 40% yield. MS: m/z found 731.1 [M+H]⁺, retention time = 1.72 min (Method D). ¹H NMR (400 MHz, Methanol-*d*₄) δ 8.62 - 8.56 (m, 1H), 8.17 (d, 1H), 7.80 (d,1H), 7.70 (d,1H), 7.66 (s, 1H), 7.54 (t, 1H), 7.38 (d,1H), 7.29 (t,2H), 4.04 (t, 3H), 4.02 (s, 2H), 3.92 (s, 1H), 3.77 (s, 2H), 3.71 (q, 5H), 3.60 - 3.54 (m, 5H), 3.41 (p, 1H), 2.91 (t, 2H), 2.43 - 2.26 (m, 3H), 1.86 (d, 1H).

### Example 472: 6-(3-Chloro-4-(2-chloro-3-(5-((3-hydroxy-3-methylazetidin-1-yl)methyl)-6-methoxypyridin-2-yl)phenyl)pyridin-2-yl)-2-((3-hydroxy-3-methylazetidin-1-yl)methyl)-3-methylpyrrolo[2,1-f][1,2,4]triazin-4(3H)-one (231)

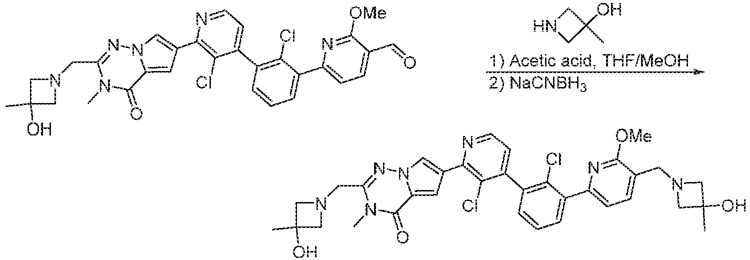

6-(3-Chloro-4-(2-chloro-3-(5-((3-hydroxy-3-methylazetidin-1-yl)methyl)-6-methoxypyridin-2-yl)phenyl)pyridin-2-yl)-2-((3-hydroxy-3-methylazetidin-1-yl)methyl)-3-methylpyrrolo[2,1-f][1,2,4]triazin-4(3H)-one was prepared using Reductive Amination Procedure B from 6-(2-chloro-3-(3-chloro-2-(2-((3-hydroxy-3-methylazetidin-1-yl)methyl)-3-methyl-4-oxo-3,4-dihydropyrrolo[2,1-f][1,2,4]triazin-6-yl)pyridin-4-yl)phenyl)-2-methoxynicotinaldehyde (15 mg, 0.02 mmol), acetic acid (3 µL, 0.05 mmol), 3-methylazetidin-3-ol;hydrochloride (6 mg, 0.05 mmol) and sodium cyanoborohydride (3 mg, 0.05 mmol). 4.9 mg, 29% yield. MS: m/z found 676 [M+H]⁺, retention time = 1.67 min (Method D). ¹H NMR (400 MHz, Methanol-*d*₄) δ 8.60 (d, 1H), 8.44 (s, 1H), 8.19 (d, 1H), 7.84 (d, 1H), 7.71 (d, 1H), 7.67 (q, 1H), 7.58 - 7.50 (m, 1H), 7.41 (d, 1H), 7.34 (d, 1H), 7.28 (d, 1H), 4.32 (s, 2H), 4.06 (t, 3H), 4.02 (d, 2H), 3.90 (s, 1H), 3.87 (s, 3H), 3.61 - 3.56 (m, 3H), 3.54 (d, 2H), 1.51 (d, 6H).

### Example 473: 6-(3-Chloro-4-(2-chloro-3-(5-((6-hydroxy-2-azaspiro[3.3]heptan-2-yl)methyl)-6-methoxypyridin-2-yl)phenyl)pyridin-2-yl)-2-((3-hydroxy-3-methylazetidin-1-yl)methyl)-3-methylpyrrolo[2,1-f][1,2,4]triazin-4(3H)-one (222)

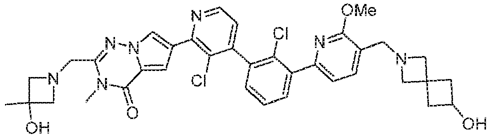

### (a) 6-Bromo-2-((3-hydroxy-3-methylazetidin-1-yl)methyl)-3-methylpyrrolo[2,1-f][1,2,4]triazin-4(3H)-one

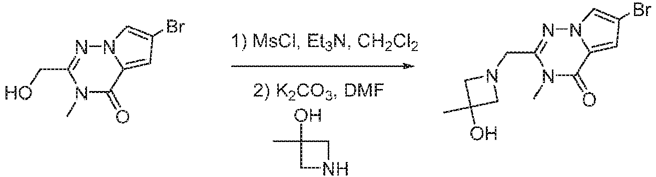

To a solution of 6-bromo-2-(hydroxymethyl)-3-methylpyrrolo[2,1-f][1,2,4]triazin-4(3H)-one (200 mg, 0.77 mmol) in dichloromethane (0.5 mL), triethylamine (216 µL, 1.55 mmol) and methanesulfonyl chloride (66 µL, 0.85 mmol) were added. The reaction was stirred at room temperature for 1h. LCMS suggested formation of the desired mesylated product. The solvent was evaporated and the residue was suspended in DMF (2 mL) followed by addition of potassium carbonate (211 mg, 1.55 mmol) and 3-methylazetidin-3-ol hydrochloride (192 mg, 1.55 mmol). The mixture was stirred at room temperature for 12 h. The solvent was evaporated, and the residue was suspended in water. The precipitate formed was collected to afford 6-bromo-2-((3-hydroxy-3-methylazetidin-1-yl)methyl)-3-methylpyrrolo[2,1-f][1,2,4]triazin-4(3H)-one (203 mg, 80%). MS: m/z found 326.9 [M+H]⁺.

### (b) 2-((3-Hydroxy-3-methylazetidin-1-yl)methyl)-3-methyl-6-(4,4,5,5-tetramethyl-1,3,2-dioxaborolan-2-yl)pyrrolo[2,1-f][1,2,4]triazin-4(3H)-one

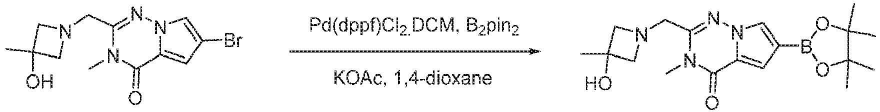

6-Bromo-2-((3-hydroxy-3-methylazetidin-1-yl)methyl)-3-methylpyrrolo[2,1-f][1,2,4]triazin-4(3H)-one (200 mg, 0.61 mmol), bis(pinacolato)diborane (217 mg, 0.86 mmol), potassium acetate (180 mg, 1.83 mmol) and [1,1'- bis(diphenylphosphino)ferrocene]-dichloropalladium(II) complex with dichloromethane (50 mg, 0.06 mmol) were suspended in 1,4-dioxane (5 ml). Nitrogen gas was bubbled through the reaction for 2 minutes. The reaction was heated at 95 °C for 3 hours. The reaction was concentrated and the residue was purified by silica gel chromatography (0-100% EtOAc in Hexanes) to afford 2-((3-hydroxy-3-methylazetidin-1-yl)methyl)-3-methyl-6-(4,4,5,5-tetramethyl-1,3,2-dioxaborolan-2-yl)pyrrolo[2,1-f][1,2,4]triazin-4(3H)-one (115 mg, 50%). MS: m/z found 375.1 [M+H]⁺.

### (c) 6-(2-Chloro-3-(3-chloro-2-(2-((3-hydroxy-3-methylazetidin-1-yl)methyl)-3-methyl-4-oxo-3,4-dihydropyrrolo[2,1-f][1,2,4]triazin-6-yl)pyridin-4-yl)phenyl)-2-methoxynicotinaldehyde

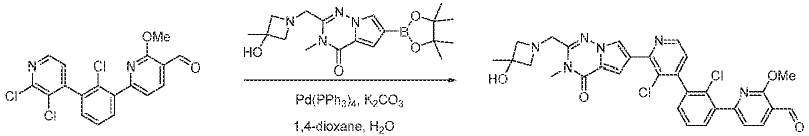

To a mixture 2-((3-hydroxy-3-methylazetidin-1-yl)methyl)-3-methyl-6-(4,4,5,5-tetramethyl-1,3,2-dioxaborolan-2-yl)pyrrolo[2,1-f][1,2,4]triazin-4(3H)-one (114 mg, 0.30 mmol) and 6-(2-chloro-3-(2,3-dichloropyridin-4-yl)phenyl)-2-methoxynicotinaldehyde (100 mg, 0.25 mmol) in 1,4-dioxane/water (4:1, 3 mL), potassium carbonate (105 mg, 0.76 mmol) was added. Nitrogen gas was bubbled through the mixture for two minutes. Tetrakis(triphenylphosphine)palladium(0) (59 mg, 0.05 mmol) was added, and the reaction was stirred at 100 °C for 30 minutes. The reaction mixture was added to water (15 mL), and then extracted with EtOAc (3 x 15 ml). The combined organic layers were washed with saturated aqueous brine solution (20 mL), dried over anhydrous sodium sulfate, filtered, and concentrated under reduced pressure to give a residue. The residue was purified by normal phase Silica gel chromatography (0-5% MeOH in DCM) to afford 6-(2-chloro-3-(3-chloro-2-(2-((3-hydroxy-3-methylazetidin-1-yl)methyl)-3-methyl-4-oxo-3,4-dihydropyrrolo[2,1-f][1,2,4]triazin-6-yl)pyridin-4-yl)phenyl)-2-methoxynicotinaldehyde (104 mg, 68%). MS: m/z found 605.1 [M+H]⁺.

### (d) 6-(3-Chloro-4-(2-chloro-3-(5-((6-hydroxy-2-azaspiro[3.3]heptan-2-yl)methyl)-6-methoxypyridin-2-yl)phenyl)pyridin-2-yl)-2-((3-hydroxy-3-methylazetidin-1-yl)methyl)-3-methylpyrrolo[2,1-f][1,2,4]triazin-4(3H)-one

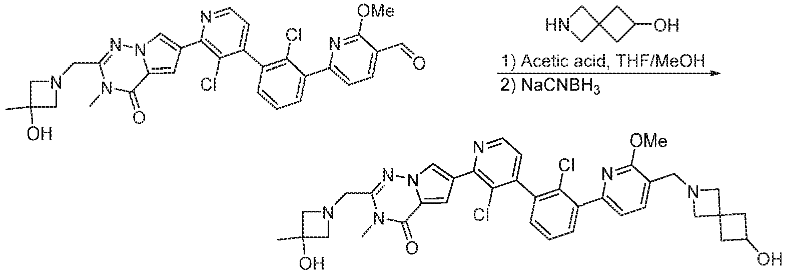

6-(3-Chloro-4-(2-chloro-3-(5-((6-hydroxy-2-azaspiro[3.3]heptan-2-yl)methyl)-6-methoxypyridin-2-yl)phenyl)pyridin-2-yl)-2-((3-hydroxy-3-methylazetidin-1-yl)methyl)-3-methylpyrrolo[2,1-f][1,2,4]triazin-4(3H)-one was prepared using Reductive Amination Procedure B from 6-(2-chloro-3-(3-chloro-2-(2-((3-hydroxy-3-methylazetidin-1-yl)methyl)-3-methyl-4-oxo-3,4-dihydropyrrolo[2,1-f][1,2,4]triazin-6-yl)pyridin-4-yl)phenyl)-2-methoxynicotinaldehyde (15 mg, 0.02 mmol), acetic acid (3 µL, 0.05 mmol), 2-azaspiro[3.3]heptan-6-ol hydrochloride (7 mg, 0.05 mmol) and sodium cyanoborohydride (3 mg, 0.05 mmol). 14 mg, 79% yield. MS: m/z found 702.1 [M+H]⁺, retention time = 1.67 min (Method D). ¹H NMR (400 MHz, Methanol-*d*₄) δ 8.24 - 8.07 (m, 1H), 7.85 - 7.69 (m, 1H), 7.27 (s, 3H), 7.11 (d, , 1H), 6.97 (s, 1H), 6.85 (dd, 2H), 3.69 (s, 1H), 3.60 (m, 3H), 3.52 (s, 2H), 3.41 - 3.32 (m, 2H), 3.17 (m, 4H), 3.09 - 2.96 (m, 2H), 2.95 - 2.68 (m, 8H), 2.12 (s, 2H), 1.65 (s, 2H), 1.12 - 1.00 (m, 3H).

### Example 474: 6-(3-Chloro-4-(2-chloro-3-(5-((((1r,3r)-3-hydroxy-3-methylcyclobutyl)amino)methyl)-6-methoxypyridin-2-yl)phenyl)pyridin-2-yl)-2-((3-hydroxy-3-methylazetidin-1-yl)methyl)-3-methylpyrrolo[2,1-f][1,2,4]triazin-4(3H)-one (232)

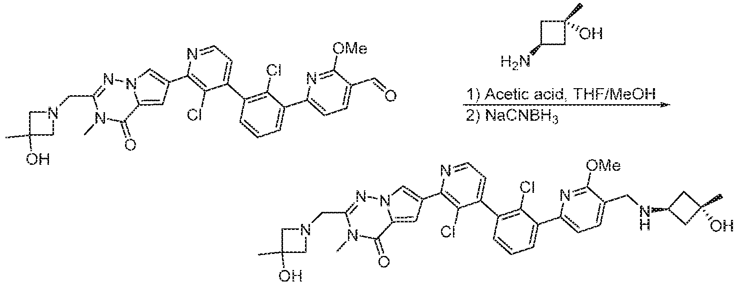

6-(3-Chloro-4-(2-chloro-3-(5-((((1r,3r)-3-hydroxy-3-methylcyclobutyl)amino)methyl)-6-methoxypyridin-2-yl)phenyl)pyridin-2-yl)-2-((3-hydroxy-3-methylazetidin-1-yl)methyl)-3-methylpyrrolo[2,1-f][1,2,4]triazin-4(3H)-one was prepared using Reductive Amination Procedure B from 6-(2-chloro-3-(3-chloro-2-(2-((3-hydroxy-3-methylazetidin-1-yl)methyl)-3-methyl-4-oxo-3,4-dihydropyrrolo[2,1-f][1,2,4]triazin-6-yl)pyridin-4-yl)phenyl)-2-methoxynicotinaldehyde (15 mg, 0.02 mmol), acetic acid (3 µL, 0.05 mmol), 3-amino-1-methyl-cyclobutanol hydrochloride (7 mg, 0.05 mmol) and sodium cyanoborohydride (3 mg, 0.05 mmol). 5.1 mg, 30% yield. MS: m/z found 690.3 [M+H]⁺, retention time = 1.67 min (Method D). ¹H NMR (400 MHz, Methanol-*d*₄) δ 8.59 (m, 1H), 8.52 (s, 1H), 8.18 (q, 1H), 7.81 (d, 1H), 7.72 - 7.68 (m, 1H), 7.66 (q, 1H), 7.55 (dd, 1H), 7.40 (d, 1H), 7.34 - 7.25 (m, 2H), 4.07 (t, 3H), 4.02 (s, 2H), 3.79 (s, 3H), 3.59 (t,3H), 3.47 (d, 2H), 3.22 (d, 2H), 2.35 (t, 2H), 2.09 (t, 2H), 1.48 (s, 3H), 1.36 (t, 3H).

### Example 475: 6-(3-Chloro-4-(2-chloro-3-(5-((isopropylamino)methyl)-6-methoxypyridin-2-yl)phenyl)pyridin-2-yl)-2-((3-hydroxy-3-methylazetidin-1-yl)methyl)-3-methylpyrrolo[2,1-f] [1,2,4]triazin-4(3H)-one (233)

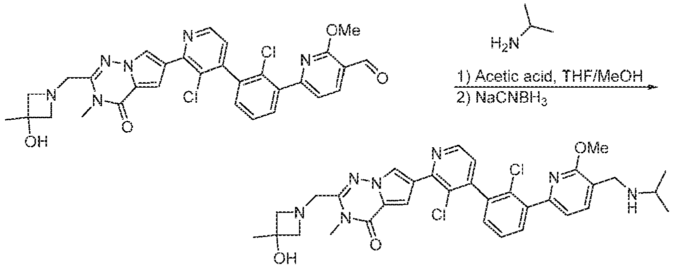

6-(3-Chloro-4-(2-chloro-3-(5-((isopropylamino)methyl)-6-methoxypyridin-2-yl)phenyl)pyridin-2-yl)-2-((3-hydroxy-3-methylazetidin-1-yl)methyl)-3-methylpyrrolo[2,1-f][1,2,4]triazin-4(3H)-one was prepared using Reductive Amination Procedure B from 6-(2-chloro-3-(3-chloro-2-(2-((3-hydroxy-3-methylazetidin-1-yl)methyl)-3-methyl-4-oxo-3,4-dihydropyrrolo[2,1-f][1,2,4]triazin-6-yl)pyridin-4-yl)phenyl)-2-methoxynicotinaldehyde (15 mg, 0.02 mmol), acetic acid (3 µL, 0.05 mmol), propan-2-amine (3 mg, 0.05 mmol) and sodium cyanoborohydride (3 mg, 0.05 mmol). 5.3 mg, 33% yield. MS: m/z found 650.1 [M+H]⁺, retention time = 1.78 min (Method D). ¹H NMR (400 MHz, Methanol-*d₄*) δ 8.59 (dd, 1H), 8.54 (d, 1H), 8.18 (d, 1H), 7.81 (d, 1H), 7.70 (d, 1H), 7.66 (d, 1H), 7.54 (m, 1H), 7.39 (d, 1H), 7.33 - 7.25 (m, 2H), 4.07 - 4.05 (m, 3H), 4.03 (s, 2H), 3.78 (s, 2H), 3.61 - 3.56 (m, 3H), 3.45 (d, 2H), 3.19 (dd, 3H), 1.48 (s, 3H), 1.28 (d, 6H).

### Example 476: 6-(3-Chloro-4-(2-chloro-3-(5-((((1r,4r)-4-hydroxycyclohexyl)amino)methyl)-6-methoxypyridin-2-yl)phenyl)pyridin-2-yl)-2-((3-hydroxy-3-methylazetidin-1-yl)methyl)-3-methylpyrrolo[2,1-f][1,2,4]triazin-4(3H)-one (234)

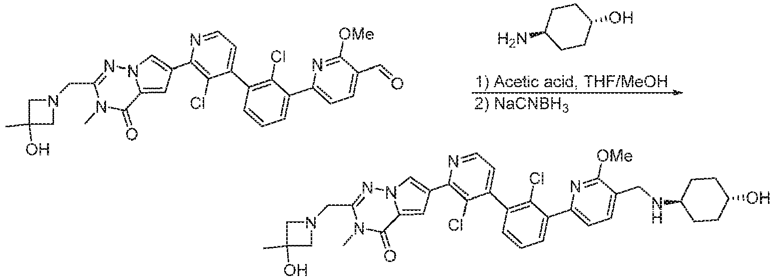

6-(3-Chloro-4-(2-chloro-3-(5-((((1r,4r)-4-hydroxycyclohexyl)amino)methyl)-6-methoxypyridin-2-yl)phenyl)pyridin-2-yl)-2-((3-hydroxy-3-methylazetidin-1-yl)methyl)-3-methylpyrrolo[2,1-f][1,2,4]triazin-4(3H)-one was prepared using Reductive Amination Procedure B from 6-(2-chloro-3-(3-chloro-2-(2-((3-hydroxy-3-methylazetidin-1-yl)methyl)-3-methyl-4-oxo-3,4-dihydropyrrolo[2,1-f][1,2,4]triazin-6-yl)pyridin-4-yl)phenyl)-2-methoxynicotinaldehyde (15 mg, 0.02 mmol), acetic acid (3 µL, 0.05 mmol), (1r,4r)-4-aminocyclohexan-1-ol (6 mg, 0.05 mmol) and sodium cyanoborohydride (3 mg, 0.05 mmol). 7.1 mg, 41% yield. MS: m/z found 704 [M+H]⁺, retention time = 1.67 min (Method D). ¹H NMR (400 MHz, Methanol-*d*₄). δ 8.64 - 8.57 (m, 1H), 8.53 (s, 1H), 8.17 (q, 1H), 7.83 (d, 1H), 7.72 - 7.68 (m, 1H), 7.66 (q,1H), 7.55 (t, 1H), 7.40 (dd, 1H), 7.35 - 7.25 (m, 2H), 4.13 (s, 2H), 4.06 (t, 3H), 3.78 (d, 2H), 3.59 (t, 3H), 3.55 (d, 1H), 3.46 (d, 2H), 3.21 (d, 2H), 2.95 (d, 1H), 2.17 (d, 2H), 2.04 (d,2H), 1.48 (s, 3H), 1.46 - 1.28 (m, 4H).

### Example 477: (S)-(2-(6-(3-Chloro-4-(2-chloro-3-(6-methoxy-5-((((5-oxopyrrolidin-2-yl)methyl)amino)methyl)pyridin-2-yl)phenyl)pyridin-2-yl)-2-methoxy-4-oxopyrrolo[2,1-f][1,2,4]triazin-3(4H)-yl)ethyl)glycine (460)

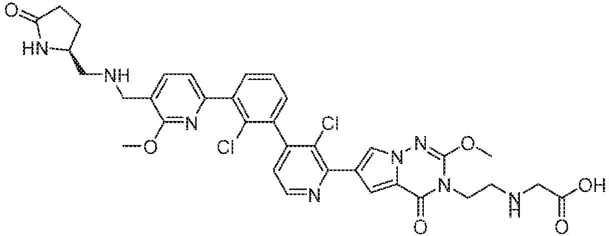

### (a) Methyl 1-amino-4-bromo-1H-pyrrole-2-carboxylate

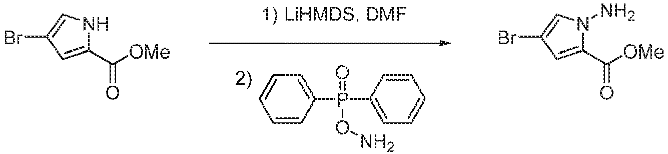

A 1.0 M solution of lithium bis(trimethylsilyl)amide in THF (53.92 mL, 53.92 mmol) was slowly added to a mixture of methyl 4-bromo-1H-pyrrole-2-carboxylate (10 g, 49.0 mmol) suspended in DMF (400 mL) at -10° C under nitrogen atmosphere. After stirring for 1 hour, O-(diphenylphosphinyl)hydroxylamine (11.4 g, 49.0 mmol) was added. The resulting reaction mixture was warmed up to room temperature and stirred for 11 hours. The reaction (6 batches in total) was neutralized with saturated aqueous ammonium chloride solution and extracted with ethyl acetate (2 L). The combined organic layers were washed with water (1 L) and brine, dried over sodium sulfate, and concentrated by evaporation. The residue was purified by normal phase SiO₂ chromatography (0-30% EtOAc/petroleum ether) to afford methyl 1-amino-4-bromo-1H-pyrrole-2-carboxylate as a yellow solid (38.3 g, 59% yield). ¹H NMR (400 MHz, CDCl₃): δ 6.88 (d, 1 H), 6.74 (d, 1 H), 5.51-5.41 (m, 2 H), 3.76 (s, 3 H).

### (b) Methyl 1-(((benzyloxy)carbonyl)amino)-4-bromo-1H-pyrrole-2-carboxylate

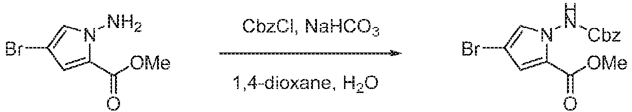

To the mixture of methyl 1-amino-4-bromo-1H-pyrrole-2-carboxylate (15.3 g, 69.9 mmol) in 1,4-dioxane (40 mL) was added water (40 mL) and sodium hydrogen carbonate (8.80 g, 105 mmol). Then benzyl chloroformate (10.9 mL, 76.8 mmol) was added by dropwise. The reaction was stirred for 12 hr at room temperature. To the mixture (3 batches in total) was added ethyl acetate (500 mL) and the mixture washed with water (600 mL) and concentrated under vacuum. The residue was purified by normal phase SiO₂ chromatography (0-50% EtOAc/petroleum ether) to afford methyl 1-(((benzyloxy)carbonyl)amino)-4-bromo-1H-pyrrole-2-carboxylate as a dark yellow oil (80 g, crude). The product was used in the next step without further purification.

### (c) 6-Bromopyrrolo[2,1-f][1,2,4]triazine-2,4(1H,3H)-dione

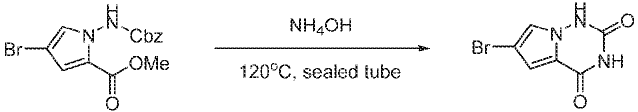

To methyl 1-(((benzyloxy)carbonyl)amino)-4-bromo-1H-pyrrole-2-carboxylate (10 g, 28.3 mmol) was added 28% ammonium hydroxide solution (30 mL, 218 mmol) and the mixture heated to 120 °C in a sealed tube for 4 hr. Then the combined mixture (7 batches) was concentrated under vacuum directly. To the residue was added ethanol (150 mL), the mixture stirred for 1 hr at room temperature, then filtered, and washed with ethanol (60 mL). The filter-cake was dried under reduced pressure to afford 6-bromopyrrolo[2,1-f][1,2,4]triazine-2,4(1H,3H)-dione as a yellow solid (15 g, 32% yield).

### (d) 6-Bromo-2,4-dichloropyrrolo[2,1-f][1,2,4]triazine

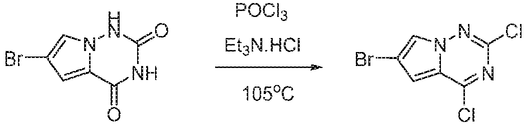

To the mixture of 6-bromopyrrolo[2,1-f][1,2,4]triazine-2,4(1H,3H)-dione (7 g, 30.4 mmol) and triethylamine hydrochloride salt (12.6 g, 91.3 mmol) was added phosphoryl trichloride (48.1 mL, 517 mmol). Then the solution was heated for 12 hr at 105 °C. After cooling to 0 °C, the mixture (2 batches) was quenched with saturated aqueous sodium hydrogen carbonate to pH 8 slowly and extracted with EtOAc (300 mL). The organic layer was dried over anhydrous sodium sulfate, filtered and concentrated under reduced pressure. The residue was purified by normal phase SiO₂ chromatography (0-10% EtOAc/petroleum ether) to afford 6-bromo-2,4-dichloropyrrolo[2,1-f][1,2,4]triazine as a white solid (13 g, 80% yield). ¹H NMR (400 MHz, DMSO-d6): δ 8.62(d, 1 H), 7.46 (d, 1 H).

### (e) 6-bromo-2-chloropyrrolo[2,1-f][1,2,4]triazin-4(3H)-one

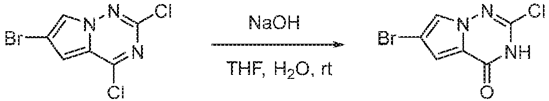

To a solution of 6-bromo-2,4-dichloropyrrolo[2,1-f][1,2,4]triazine (12 g, 45 mmol) in THF (80 mL) was added sodium hydroxide (3.96 g, 98.9 mmol) and water (80 mL).The mixture was stirred at room temperature for 12 hr . To the mixture was added 1N HCl to pH 3, and the mixture extracted with ethyl acetate (100 mL). The organic layer was washed with brine (200 mL), dried over anhydrous sodium sulfate and concentrated under reduced pressure to afford 6-bromo-2-chloropyrrolo[2,1-f][1,2,4]triazin-4(3H)-one as a white solid (10 g, 89% yield) which was used directly without further purification. ¹H NMR (400 MHz, DMSO-*d*₆): δ 13.07 (br s, 1 H), 7.91 (s, 1 H), 7.09 (d, 1 H).

### (f) 3-Allyl-6-bromo-2-chloropyrrolo[2,1-f][1,2,4]triazin-4(3H)-one

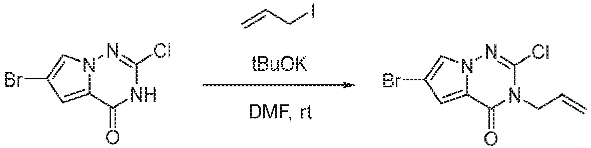

To the mixture of 6-bromo-2-chloropyrrolo[2,1-f][1,2,4]triazin-4(3H)-one (3 g, 12.1 mmol) in DMF (30 mL) was added potassium tert-butoxide (2.03 g, 18.1 mmol) and 3-iodoprop-1-ene (1.65 mL, 18.1 mmol) and the reaction was stirred for 24 hr at room temperature. The reaction was quenched with water (20 mL) and extracted with ethyl acetate (60 mL). The organic layer was washed with brine (80 ml), dried over anhydrous sodium sulfate, filtered and concentrated under reduced pressure. The residue was purified by normal phase SiO₂ chromatography (0-10% EtOAc/petroleum ether) to afford 3-allyl-6-bromo-2-chloropyrrolo[2,1-f][1,2,4]triazin-4(3H)-one as a light yellow solid (3 g, 86% yield).

### (g) 3-Allyl-6-bromo-2-methoxypyrrolo[2,1-f][1,2,4]triazin-4(3H)-one

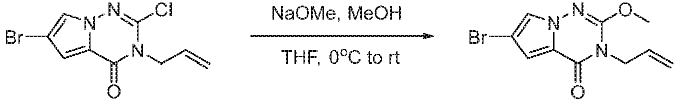

To a mixture of 3-allyl-6-bromo-2-chloropyrrolo[2,1-f][1,2,4]triazin-4 (3H)-one (2 g, 6.93 mmol) in THF (50 mL) at 0 °C was added a 30% solution of sodium methoxide in methanol (1.19 g, 6.59 mmol). Then the mixture was warmed to room temperature and stirred for 1 hr. To the reaction was added water (1 mL) and the mixture was concentrated. To the residue was added water (20 mL) and the mixture extracted with dichloromethane (50 mL). The organic layer was washed with brine (20 mL), dried over anhydrous sodium sulfate, filtered and the filtrate was concentrated under reduced pressure. The residue was purified by normal phase SiO₂ chromatography (0-20% EtOAc/petroleum ether) to afford 3-allyl-6-bromo-2-methoxypyrrolo[2,1-f][1,2,4]triazin-4(3H)-one as a yellow solid (1.41 g, 69% yield). ¹H NMR (400 MHz, DMSO-*d*₆): δ 7.67 (d, 1 H), 6.94 (d, 1 H), 5.94-5.87 (m, 1 H), 5.17-5.11 (m, 2 H), 4.55-4.52 (m, 2 H), 3.99 (s, 3 H).

### (h) 2-(6-Bromo-2-methoxy-4-oxopyrrolo[2,1-f][1,2,4]triazin-3(4H)-yl)acetaldehyde

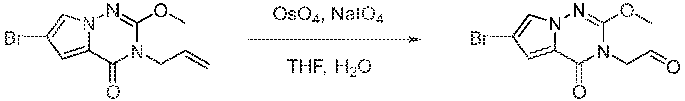

To the solution of 3-allyl-6-bromo-2-methoxypyrrolo[2,1-f][1,2,4]triazin-4(3H)-one (1.4 g, 4.93 mmol) in THF/water (1/1, 20 mL) was added a 0.16 M solution of osmium tetroxide in water (12.3 mL) at 0 °C and then sodium periodate (6.96 g, 32.5 mmol) was added. The reaction was warmed up to room temperature and stirred for 12 hr. The reaction was quenched with saturated aqueous sodium sulfite (200 mL), and extracted with dichloromethane (100 mL). The organic layer was dried over anhydrous sodium sulfate, filtered and concentrated to afford 2-(6-bromo-2-methoxy-4-oxopyrrolo[2,1-f][1,2,4]triazin-3(4H)-yl)acetaldehyde as a yellow solid (1 g, crude), which was used into the next step without further purification. ¹H NMR (400 MHz, CDCl₃): δ 9.62 (s, 1 H), 7.81 (s, 1 H), 7.03 (s, 1 H), 4.87 (s, 2 H), 3.93 (s, 3 H).

### (i) tert-Butyl N-(2-(6-bromo-2-methoxy-4-oxopyrrolo[2,1-f][1,2,4]triazin-3(4H)-yl)ethyl)-N-(tert-butoxycarbonyl)glycinate

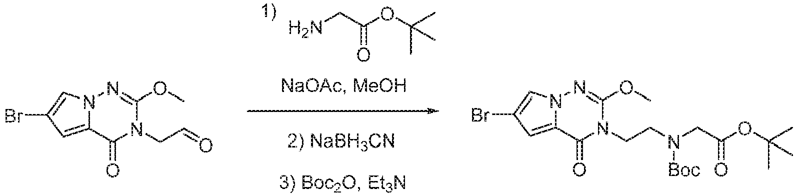

To the mixture of 2-(6-bromo-2-methoxy-4-oxopyrrolo[2,1-f][1,2,4]triazin-3(4H)-yl)acetaldehyde (0.75 g, 2.62 mmol) in methanol (30 mL) was added *tert*-butyl glycinate (516 mg, 3.93 mmol) and sodium acetate (645 mg, 7.86 mmol) and the mixture stirred for 2 hr at room temperature. Sodium cyanoborohydride (494 mg, 7.86 mmol) was then added and stirring continued for another 2 hr to give tert-butyl (2-(6-bromo-2-methoxy-4-oxopyrrolo[2,1-f][1,2,4]triazin-3(4H)-yl)ethyl)glycinate. Triethylamine (1.04 mL 7.48 mmol) and di-tert-butyl dicarbonate (2.72 g, 12.5 mmol) were then added and the reaction was stirred for 12 hr at room temperature. The mixture was concentrated under reduced pressure and the residue was purified by normal phase SiO₂ chromatography (0-10% EtOAc/petroleum ether) to afford tert-butyl N-(2-(6-bromo-2-methoxy-4-oxopyrrolo[2,1-f][1,2,4]triazin-3(4H)-yl)ethyl)-N-(tert-butoxycarbonyl)glycinate as a yellow oil (0.75 g, 57% yield). ¹H NMR (400 MHz, CDCl₃): δ 7.18 (dd, 1 H), 6.91 (dd, 1 H), 4.11-1.07 (m, 2 H), 3.98 (d, 3 H), 3.85 (s, 1 H), 3.75 (s, 1 H), 3.54-3.48 (m, 2 H), 1.42 (d, 9 H), 1.28 (s, 9 H).

### (j) tert-Butyl N-(tert-butoxycarbonyl)-N-(2-(2-methoxy-4-oxo-6-(4,4,5,5-tetramethyl-1,3,2-dioxaborolan-2-yl)pyrrolo[2,1-f][1,2,4]triazin-3(4H)-yl)ethyl)glycinate

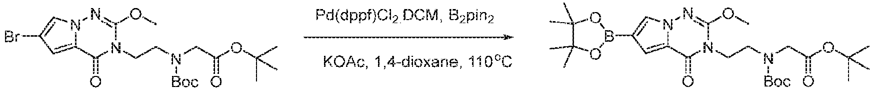

To a mixture of tert-butyl N-(2-(6-bromo-2-methoxy-4-oxopyrrolo[2,1-f][1,2,4]triazin-3(4H)-yl)ethyl)-N-(tert-butoxycarbonyl)glycinate (245 mg, 0.49 mmol) and bis(pinacolato)diborane (372 mg, 1.47 mmol) in 1,4-dioxane (5 mL) was added potassium acetate (95.9 mg,0.98 mmol), followed by [1,1'-bis(diphenylphosphino)ferrocene]dichloropalladium(II) complex with dichloromethane (39.9 mg, 0.05 mmol) and then the reaction was stirred for 5 hr at 110 °C. After cooling, the mixture combined with another batch at 300 mg scale and water (10 mL) was added and the mixture extracted with ethyl acetate (20 mL). The organic layer was dried over anhydrous sodium sulfate, filtered and concentrated. The residue was purified by normal phase SiO₂ chromatography (0-30% EtOAc/petroleum ether) twice to afford tert-butyl N-(tert-butoxycarbonyl)-N-(2-(2-methoxy-4-oxo-6-(4,4,5,5-tetramethyl-1,3,2-dioxaborolan-2-yl)pyrrolo[2,1-f][1,2,4]triazin-3(4H)-yl)ethyl)glycinate as a yellow oil (270 mg in total). MS: m/z found 571 [M+Na]⁺.

### (k) tert-Butyl (S)-N-(tert-butoxycarbonyl)-N-(2-(6-(4-(3-(5-(((tert-butoxycarbonyl)((5-oxopyrrolidin-2-yl)methyl)amino)methyl)-6-methoxypyridin-2-yl)-2-chlorophenyl)-3-chloropyridin-2-yl)-2-methoxy-4-oxopyrrolo[2,1-f][1,2,4]triazin-3(4H)-yl)ethyl)glycinate

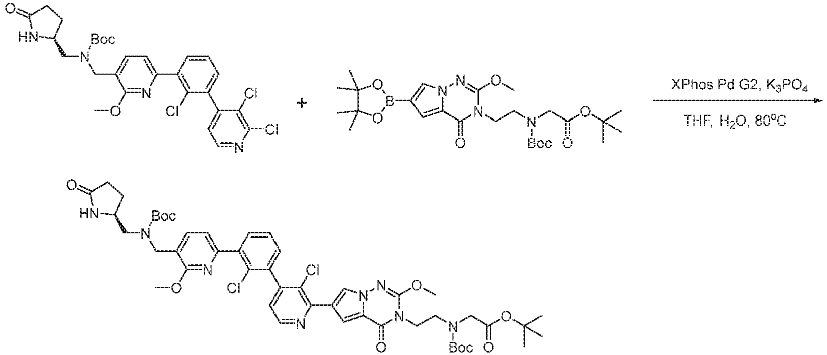

To a mixture of tert-butyl N-(tert-butoxycarbonyl)-N-(2-(2-methoxy-4-oxo-6-(4,4,5,5-tetramethyl-1,3,2-dioxaborolan-2-yl)pyrrolo[2,1-f][1,2,4]triazin-3(4H)-yl)ethyl)glycinate (104 mg, 0.18 mmol) and tert-butyl (S)-((6-(2-chloro-3-(2,3-dichloropyridin-4-yl)phenyl)-2-methoxypyridin-3-yl)methyl)((5-oxopyrrolidin-2-yl)methyl)carbamate (Example 45, step (c)) (80 mg, 0.13 mmol) in THF/water (4/1, 5 mL) was added potassium phosphate (86.1 mg, 0.4 mmol) and chloro(2-dicyclohexylphosphino-2',4',6'-triisopropyl-1,1'-biphenyl)[2-(2'-amino-1,1'-biphenyl)]palladium(II) (XPhos Pd G2) (10.6 mg, 0.01 mmol) in one portion under N₂. The mixture was stirred at 80 °C for 12 hr. To the reaction was added water (20 mL) and the mixture extracted with ethyl acetate (100 mL). The organic phase was dried with anhydrous sodium sulfate, filtered and concentrated and the residue purified by normal phase SiO₂ chromatography (0-100% EtOAc/petroleum ether) to afford tert-butyl (S)-N-(tert-butoxycarbonyl)-N-(2-(6-(4-(3-(5-(((tert-butoxycarbonyl)((5-oxopyrrolidin-2-yl)methyl)amino)methyl)-6-methoxypyridin-2-yl)-2-chlorophenyl)-3-chloropyridin-2-yl)-2-methoxy-4-oxopyrrolo[2,1-f][1,2,4]triazin-3(4H)-yl)ethyl)glycinate (210 mg, 43% yield) as a yellow solid.

### (l) (S)-(2-(6-(3-Chloro-4-(2-chloro-3-(6-methoxy-5-((((5-oxopyrrolidin-2-yl)methyl)amino)methyl)pyridin-2-yl)phenyl)pyridin-2-yl)-2-methoxy-4-oxopyrrolo[2,1-f][1,2,4]triazin-3(4H)-yl)ethyl)glycine

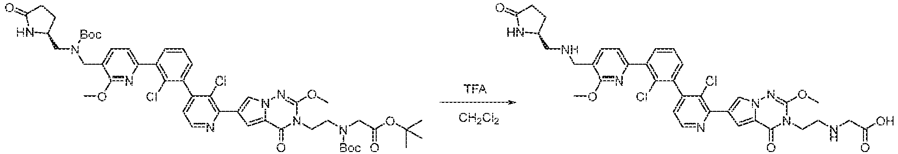

To a mixture of tert-butyl (S)-N-(tert-butoxycarbonyl)-N-(2-(6-(4-(3-(5-(((tert-butoxycarbonyl)((5-oxopyrrolidin-2-yl)methyl)amino)methyl)-6-methoxypyridin-2-yl)-2-chlorophenyl)-3-chloropyridin-2-yl)-2-methoxy-4-oxopyrrolo[2,1-f][1,2,4]triazin-3(4H)-yl)ethyl)glycinate (200 mg, 205 mmol) in dichloromethane (1.5 mL) was added trifluoroacetic acid (3.00 mL,40.5 mmol) and the reaction stirred for 12 hr at room temperature. The mixture was concentrated under vacuum and the residue purified by reverse phase HPLC to afford (S)-(2-(6-(3-chloro-4-(2-chloro-3-(6-methoxy-5-((((5-oxopyrrolidin-2-yl)methyl)amino)methyl)pyridin-2-yl)phenyl)pyridin-2-yl)-2-methoxy-4-oxopyrrolo[2,1-f][1,2,4]triazin-3(4H)-yl)ethyl)glycine (16.4 mg, 10% yield) as a white solid (formic acid salt). MS: m/z found 721 and 723 [M+H]⁺, retention time = 2.76 min (Method A). ¹H NMR (400 MHz, Methanol-*d*₄): δ 8.58 (d, 1H), 8.33 (s, 1H), 8.13 (d, 1H), 7.80 (d, 1H), 7.71-7.69 (m, 2H), 7.55 (t, 1H), 7.39 (dd, 1H), 7.31 (d, 1H), 7.27 (d, 1H), 4.39-4.36 (m, 2H), 4.12 ( s, 3H), 4.05 (s, 3H), 4.04-4.03 (m, 2H), 3.94-3.89 (m, 1H), 3.52 (s, 2H), 3.36-3.33 (m, 2H), 2.93-2.91 (m, 2H), 2.36-2.32 (m, 4H).

### Example 478: (S)-5-((((6-(2-chloro-3-(3-chloro-2-(3-fluoro-4-((6-hydroxy-2-azaspiro[3.3] heptan-2-yl)methyl)-5-methoxyphenyl)pyridin-4-yl)phenyl)-2-methoxypyridin-3-yl)methyl) amino)methyl)pyrrolidin-2-one (555)

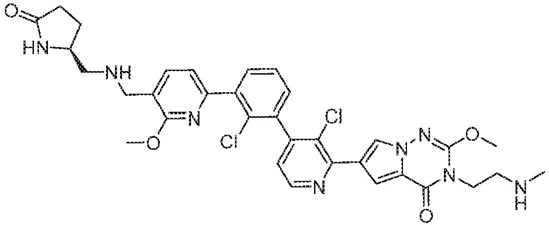

### (a) tert-Butyl (2-(6-bromo-2-methoxy-4-oxopyrrolo[2,1-f] [1,2,4]triazin-3(4H)-yl)ethyl)(methyl)carbamate

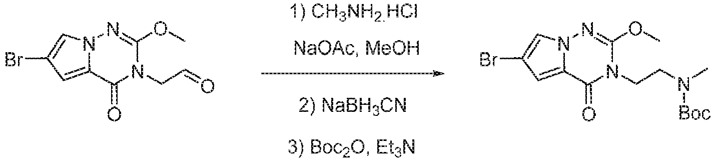

To the mixture of 2-(6-bromo-2-methoxy-4-oxopyrrolo[2,1-f][1,2,4]triazin-3(4H)-yl)acetaldehyde (850 mg, 2.97 mmol) (Example 477, step (h)) in methanol (10 mL) was added methylamine hydrochloride (1.00 g, 14.9 mmol) and sodium acetate (1.46 g, 17.8 mmol), then the mixture stirred for 2 hr at room temperature. Sodium cyanoborohydride (560 mg, 8.91 mmol) was added and the mixture stirred for another 10 hr. Triethylamine (2.07 mL, 14.9 mmol) and di-tert-butyl dicarbonate (3.89 g, 17.8 mmol) were then added, and the mixture was stirred for 2 hr at room temperature. The mixture combined with another batch on 316 mg scale and water (15 mL) was added, and the mixture extracted with dichloromethane (60 mL). The organic layer was dried over anhydrous sodium sulfate, filtered and concentrated. The residue was purified by normal phase SiO₂ chromatography (0-30% EtOAc/petroleum ether) to afford tert-butyl (2-(6-bromo-2-methoxy-4-oxopyrrolo[2,1-f][1,2,4]triazin-3(4H)-yl)ethyl)(methyl)carbamate (770 mg, 85% purity) as a yellow solid. MS: m/z found 423 and 425 [M+Na]⁺.

### (b) tert-Butyl (2-(2-methoxy-4-oxo-6-(4,4,5,5-tetramethyl-1,3,2-dioxaborolan-2-yl)pyrrolo[2,1-f][1,2,4]triazin-3(4H)-yl)ethyl)(methyl)carbamate

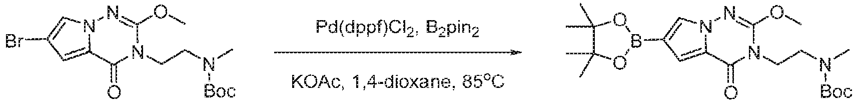

A mixture of tert-butyl (2-(6-bromo-2-methoxy-4-oxopyrrolo[2,1-f][1,2,4]triazin-3(4H)-yl)ethyl)(methyl)carbamate (400 mg, 1.0 mmol), bis(pinacolato)diborane (1.01 g, 4.0 mmol), potassium acetate (294 mg, 2.99 mmol) and [1,1'-bis(diphenylphosphino)-ferrocene]dichloropalladium(II) (72.9 mg, 99.7 mmol) in 1,4-dioxane (4 mL) was degassed for 0.3 hr at room temperature. Then the reaction mixture was stirred at 85 °C for 12 hr. After cooling water (15 mL) was added and the mixture extracted with ethyl acetate (50 mL). The organic layer was washed with brine (30 mL), dried over anhydrous sodium sulfate, filtered and concentrated under reduced pressure. The residue was purified by normal phase SiO₂ chromatography (0-30% EtOAc/petroleum ether) twice to afford tert-butyl (2-(2-methoxy-4-oxo-6-(4,4,5,5-tetramethyl-1,3,2-dioxaborolan-2-yl)pyrrolo[2,1-f][1,2,4]triazin-3(4H)-yl)ethyl)(methyl)carbamate (180 mg, 15% yield) as a yellow oil.

### (c) tert-Butyl (S)-((6-(3-(2-(3-(2-((tert-butoxycarbonyl)(methyl)amino)ethyl)-2-methoxy-4-oxo-3,4-dihydropyrrolo[2,1-f][1,2,4]triazin-6-yl)-3-chloropyridin-4-yl)-2-chlorophenyl)-2-methoxypyridin-3-yl)methyl)((5-oxopyrrolidin-2-yl)methyl)carbamate

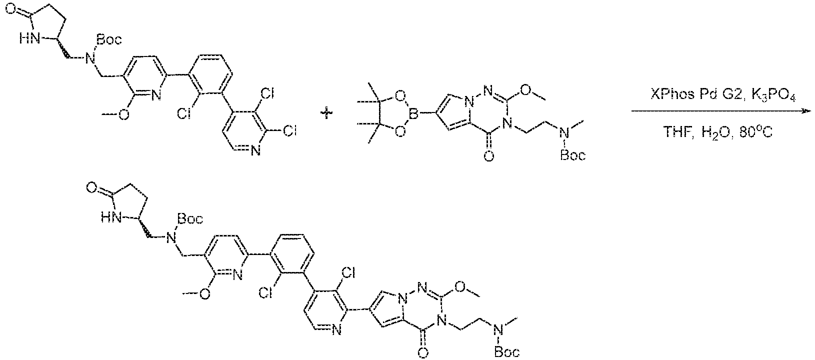

To a mixture of tert-butyl (2-(2-methoxy-4-oxo-6-(4,4,5,5-tetramethyl-1,3,2-dioxaborolan-2-yl)pyrrolo[2,1-f][1,2,4]triazin-3(4H)-yl)ethyl)(methyl)carbamate (45.4 mg, 0.10 mmol) and tert-butyl (S)-((6-(2-chloro-3-(2,3-dichloropyridin-4-yl)phenyl)-2-methoxypyridin-3-yl)methyl)((5-oxopyrrolidin-2-yl)methyl)carbamate (Example 45, step (c)) (50 mg, 0.08 mmol) in ethanol/water (4/1, 1.25 mL) was added potassium phosphate (35.9 mg, 0.17 mmol) and chloro(2-dicyclohexylphosphino-2',4',6'-triisopropyl-1,1'-biphenyl)[2-(2'-amino-1,1'-biphenyl)]palladium(II) (XPhos Pd G2) (6.65 mg, 8.45 µmol) in one portion under N₂. The mixture was stirred at 80 °C for 12 hr. Water (5 mL) was added and the mixture extracted with ethyl acetate (40 mL). The organic phase was dried with anhydrous sodium sulfate, filtered and concentrated and the residue purified by normal phase SiO₂ chromatography (0-30% ethyl acetate /petroleum ether) to afford a yellow oil. The oil was purified by normal phase SiO₂ chromatography (100% EtOAc first and 10% methanol/ ethyl acetate second) twice to afford tert-butyl (S)-((6-(3-(2-(3-(2-((tert-butoxycarbonyl)(methyl)amino)ethyl)-2-methoxy-4-oxo-3,4-dihydropyrrolo[2,1-f][1,2,4]triazin-6-yl)-3-chloropyridin-4-yl)-2-chlorophenyl)-2-methoxypyridin-3-yl)methyl)((5-oxopyrrolidin-2-yl)methyl)carbamate (120 mg, 40% yield) as a yellow oil. MS: m/z found 877 and 879 [M+H]⁺.

### (d) (S)-6-(3-chloro-4-(2-chloro-3-(6-methoxy-5-((((5-oxopyrrolidin-2-yl)methyl)amino)methyl)pyridin-2-yl)phenyl)pyridin-2-yl)-2-methoxy-3-(2-(methylamino)ethyl)pyrrolo[2,1-f] [1,2,4]triazin-4(3H)-one

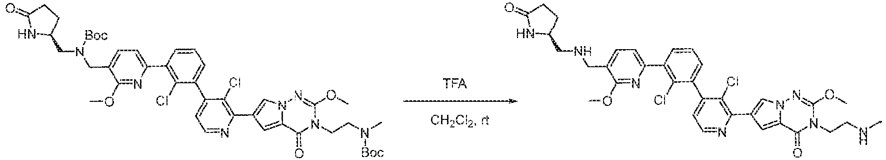

A mixture of tert-butyl (S)-((6-(3-(2-(3-(2-((tert-butoxycarbonyl)(methyl)amino)ethyl)-2-methoxy-4-oxo-3,4-dihydropyrrolo[2,1-f][1,2,4]triazin-6-yl)-3-chloropyridin-4-yl)-2-chlorophenyl)-2-methoxypyridin-3-yl)methyl)((5-oxopyrrolidin-2-yl)methyl)carbamate (110 mg, 0.13 mmol) and trifluoroacetic acid (2 mL, 27.0 mmol) in dichloromethane (1 mL) was stirred for 0.5 hr at room temperature. The mixture was concentrated under reduced pressure and the residue purified by reverse phase HPLC to afford (S)-6-(3-chloro-4-(2-chloro-3-(6-methoxy-5-((((5-oxopyrrolidin-2-yl)methyl)amino)methyl)pyridin-2-yl)phenyl)pyridin-2-yl)-2-methoxy-3-(2-(methylamino)ethyl)pyrrolo[2,1-f][1,2,4]triazin-4(3H)-one (4.6 mg, 4.8% yield) as a brown solid (formic acid salt). MS: m/z found 677 and 679 [M+H]⁺, retention time = 2.79 min (Method A). ¹H NMR (400 MHz, Methanol-*d*₄): δ 8.62 (d, 1H), 8.17 (d, 1H), 7.93 (d, 1H), 7.74 (dd, 2H), 7.60 (t, 1H), 7.45 (dd, 1H), 7.41 (d, 1H), 7.31 (d, 1H), 4.40-4.37 (m, 4 H), 4.14-4.07 (m, 7H), 3.39-3.37 (m, 2 H), 3.29 (s, 2 H), 2.77 (s, 3 H), 2.46-2.39 (m, 3H), 1.96-1.92 (m, 1 H).

### Example 479: (S)-5-((((6-(2-chloro-3-(3-chloro-2-(8-methoxy-2-(((((S)-5-oxopyrrolidin-2-yl)methyl)amino)methyl)-[1,2,4]triazolo[1,5-a]pyridin-6-yl)pyridin-4-yl)phenyl)-2-methoxypyridin-3-yl)methyl)amino)methyl)pyrrolidin-2-one (258)

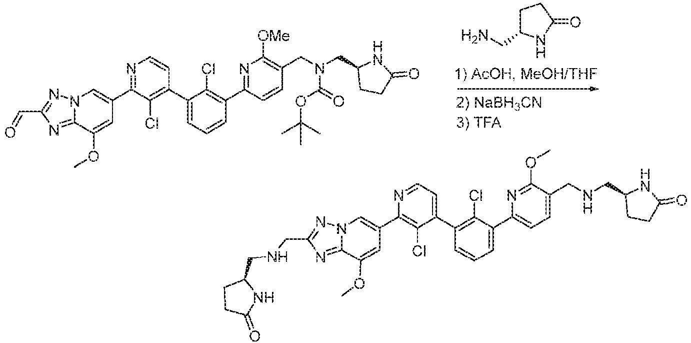

tert-Butyl ((6-(2-chloro-3-(3-chloro-2-(8-methoxy-2-(((((S)-5-oxopyrrolidin-2-yl)methyl)amino)methyl)-[1,2,4]triazolo[1,5-a]pyridin-6-yl)pyridin-4-yl)phenyl)-2-methoxypyridin-3-yl)methyl)(((S)-5-oxopyrrolidin-2-yl)methyl)carbamate was prepared using Reductive Amination Procedure B from tert-butyl (S)-((6-(2-chloro-3-(3-chloro-2-(2-formyl-8-methoxy-[1,2,4]triazolo[1,5-a]pyridin-6-yl)pyridin-4-yl)phenyl)-2-methoxypyridin-3-yl)methyl)((5-oxopyrrolidin-2-yl)methyl)carbamate (Example 480, step (b)) (15 mg, 0.02 mmol), acetic acid (2 µL, 0.04 mmol), (5S)-5-(aminomethyl)pyrrolidin-2-one (5 mg, 0.04 mmol) and sodium cyanoborohydride (3 mg, 0.04 mmol). (S)-5-((((6-(2-Chloro-3-(3-chloro-2-(8-methoxy-2-(((((S)-5-oxopyrrolidin-2-yl)methyl)amino)methyl)-[1,2,4]triazolo[1,5-a]pyridin-6-yl)pyridin-4-yl)phenyl)-2-methoxypyridin-3-yl)methyl)amino)methyl)pyrrolidin-2-one was prepared using General Boc Deprotection Procedure from tert-butyl ((6-(2-chloro-3-(3-chloro-2-(8-methoxy-2-(((((S)-5-oxopyrrolidin-2-yl)methyl)amino)methyl)-[1,2,4]triazolo[1,5-a]pyridin-6-yl)pyridin-4-yl)phenyl)-2-methoxypyridin-3-yl)methyl)(((S)-5-oxopyrrolidin-2-yl)methyl)carbamate and trifluoroacetic acid (24 µL, 0.31 mmol). 5.6 mg, 37% yield. MS: m/z found 730.2 [M+H]⁺, retention time = 1.51 min (Method D). ¹H NMR (400 MHz, Methanol-*d*₄) δ 8.85 (s, 1H), 8.06 (s, 1H), 7.92 (s, 1H), 7.73 (s, 1H), 7.60 (s, 1H), 7.53 (s, 1H), 7.50 (s, 2H), 4.62 (s, 2H), 4.35 (s, 2H), 4.13 (s, 4H), 4.10 (d, 4H), 3.38 (s, 2H), 2.41 (s, 6H).

### Example 480: (S)-5-((((6-(2-Chloro-3-(3-chloro-2-(2-((3-hydroxy-3-methylazetidin-1-yl)methyl)-8-methoxy-[1,2,4]triazolo[1,5-a]pyridin-6-yl)pyridin-4-yl)phenyl)-2-methoxypyridin-3-yl)methyl)amino)methyl)pyrrolidin-2-one (259)

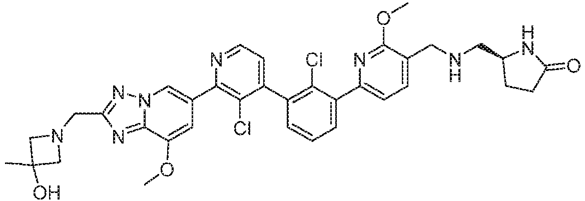

### (a) tert-Butyl (S)-((6-(2-chloro-3-(3-chloro-2-(2-(hydroxymethyl)-8-methoxy-[1,2,4]triazolo[1,5-a]pyridin-6-yl)pyridin-4-yl)phenyl)-2-methoxypyridin-3-yl)methyl)((5-oxopyrrolidin-2-yl)methyl)carbamate

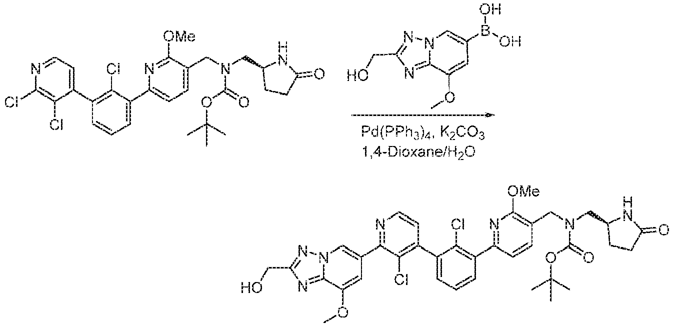

To a mixture of (2-(hydroxymethyl)-8-methoxy-[1,2,4]triazolo[1,5-a]pyridin-6-yl)boronic acid (53 mg, 0.24 mmol) and tert-butyl (S)-((6-(2-chloro-3-(2,3-dichloropyridin-4-yl)phenyl)-2-methoxypyridin-3-yl)methyl)((5-oxopyrrolidin-2-yl)methyl)carbamate (100 mg, 0.16 mmol) in 1,4-dioxane/water (4:1, 3 mL), potassium carbonate (65 mg, 0.47 mmol) was added. Nitrogen gas was bubbled through the mixture for two minutes. Tetrakis(triphenylphosphine)palladium(0) (36 mg, 0.03 mmol) was added, and the reaction was stirred at 100 °C for 30 minutes. The reaction mixture was diluted with water (30 mL), and the aqueous layer was extracted with EtOAc (3 x 30 ml). The combined organic layers were washed with brine (20 mL), dried over anhydrous sodium sulfate, filtered, and concentrated. The residue was purified by normal phase silica gel chromatography (0-5% MeOH in DCM) to afford tert-butyl (S)-((6-(2-chloro-3-(3-chloro-2-(2-(hydroxymethyl)-8-methoxy-[1,2,4]triazolo[1,5-a]pyridin-6-yl)pyridin-4-yl)phenyl)-2-methoxypyridin-3-yl)methyl)((5-oxopyrrolidin-2-yl)methyl)carbamate (109 mg, 94% yield). MS: m/z found 734 [M+H]⁺.

### (b) tert-Butyl (S)-((6-(2-chloro-3-(3-chloro-2-(2-formyl-8-methoxy-[1,2,4]triazolo[1,5-a]pyridin-6-yl)pyridin-4-yl)phenyl)-2-methoxypyridin-3-yl)methyl)((5-oxopyrrolidin-2-yl)methyl)carbamate

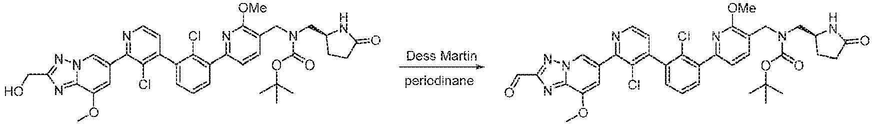

To a solution of tert-butyl (S)-((6-(2-chloro-3-(3-chloro-2-(2-(hydroxymethyl)-8-methoxy-[1,2,4]triazolo[1,5-a]pyridin-6-yl)pyridin-4-yl)phenyl)-2-methoxypyridin-3-yl)methyl)((5-oxopyrrolidin-2-yl)methyl)carbamate (109 mg, 0.15 mmol) in dichloromethane (3 mL), Dess Martin periodinane (126 mg, 0.30 mmol) was added. The reaction was stirred at room temperature for 2 h. The reaction mixture was concentrated and the residue was purified by normal phase silica gel chromatography (0-5% MeOH in DCM) to afford tert-butyl (S)-((6-(2-chloro-3-(3-chloro-2-(2-formyl-8-methoxy-[1,2,4]triazolo[1,5-a]pyridin-6-yl)pyridin-4-yl)phenyl)-2-methoxypyridin-3-yl)methyl)((5-oxopyrrolidin-2-yl)methyl)carbamate (92 mg, 85% yield). MS: m/z found 731.9 [M+H]⁺.

### (c) (S)-5-((((6-(2-Chloro-3-(3-chloro-2-(2-((3-hydroxy-3-methylazetidin-1-yl)methyl)-8-methoxy-[1,2,4]triazolo[1,5-a]pyridin-6-yl)pyridin-4-yl)phenyl)-2-methoxypyridin-3-yl)methyl)amino)methyl)pyrrolidin-2-one

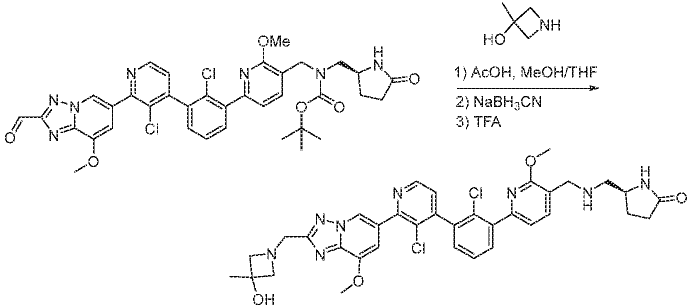

tert-Butyl (S)-((6-(2-chloro-3-(3-chloro-2-(2-((3-hydroxy-3-methylazetidin-1-yl)methyl)-8-methoxy-[1,2,4]triazolo[1,5-a]pyridin-6-yl)pyridin-4-yl)phenyl)-2-methoxypyridin-3-yl)methyl)((5-oxopyrrolidin-2-yl)methyl)carbamate was prepared using Reductive Amination Procedure B from tert-butyl (S)-((6-(2-chloro-3-(3-chloro-2-(2-formyl-8-methoxy-[1,2,4]triazolo[1,5-a]pyridin-6-yl)pyridin-4-yl)phenyl)-2-methoxypyridin-3-yl)methyl)((5-oxopyrrolidin-2-yl)methyl)carbamate (15 mg, 0.02 mmol), acetic acid (2 µL, 0.04 mmol), 3-methylazetidin-3-ol hydrochloride (5 mg, 0.04 mmol) and sodium cyanoborohydride (3 mg, 0.04 mmol). (S)-5-((((6-(2-Chloro-3-(3-chloro-2-(2-((3-hydroxy-3-methylazetidin-1-yl)methyl)-8-methoxy-[1,2,4]triazolo[1,5-a]pyridin-6-yl)pyridin-4-yl)phenyl)-2-methoxypyridin-3-yl)methyl)amino)methyl)pyrrolidin-2-one was prepared using General Boc Deprotection Procedure from tert-butyl (S)-((6-(2-chloro-3-(3-chloro-2-(2-((3-hydroxy-3-methylazetidin-1-yl)methyl)-8-methoxy-[1,2,4]triazolo[1,5-a]pyridin-6-yl)pyridin-4-yl)phenyl)-2-methoxypyridin-3-yl)methyl)((5-oxopyrrolidin-2-yl)methyl)carbamate and trifluoroacetic acid (21 µL, 0.27 mmol). 4.2 mg, 29% yield. MS: m/z found 703 [M+H]⁺, retention time = 1.55 min (Method D). ¹H NMR (400 MHz, Methanol-*d*₄) δ 8.82 (s, 1H), 8.72 (d, 1H), 8.27 (s, 1H), 7.86 (d, 1H), 7.73 (d, 1H), 7.58 (t, 1H), 7.52 (d, 1H), 7.46 (d, 2H), 7.34 (d, 1H), 4.54 (s, 2H), 4.20 (s, 2H), 4.11 (d, 4H), 4.07 (t, 4H), 4.00 (s, 1H), 3.95 (d, 2H), 3.09 (t, 2H), 2.35 (d, 3H), 1.88 (d, 1H), 1.53 (s, 3H).

### Example 481: (S)-5-((((6-(2-Chloro-3-(3-chloro-2-(2-((((1r,4r)-4-hydroxycyclohexyl)amino)methyl)-8-methoxy-[1,2,4]triazolo[1,5-a]pyridin-6-yl)pyridin-4-yl)phenyl)-2-methoxypyridin-3-yl)methyl)amino)methyl)pyrrolidin-2-one (260)

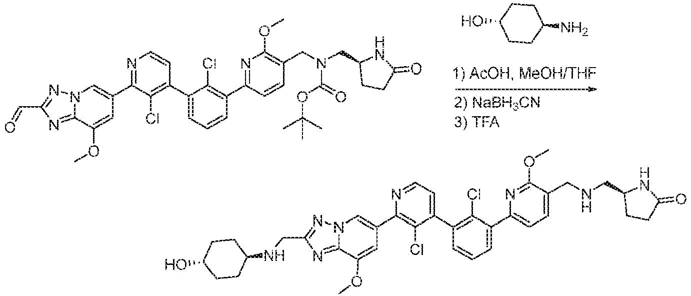

tert-Butyl ((6-(2-chloro-3-(3-chloro-2-(2-((((1r,4r)-4-hydroxycyclohexyl)amino)methyl)-8-methoxy-[1,2,4]triazolo[1,5-a]pyridin-6-yl)pyridin-4-yl)phenyl)-2-methoxypyridin-3-yl)methyl)(((S)-5-oxopyrrolidin-2-yl)methyl)carbamate was prepared using Reductive Amination Procedure B from tert-butyl (S)-((6-(2-chloro-3-(3-chloro-2-(2-formyl-8-methoxy-[1,2,4]triazolo[1,5-a]pyridin-6-yl)pyridin-4-yl)phenyl)-2-methoxypyridin-3-yl)methyl)((5-oxopyrrolidin-2-yl)methyl)carbamate (15 mg, 0.02 mmol), acetic acid (2 µL, 0.04 mmol), (1r,4r)-4-aminocyclohexan-1-ol (5 mg, 0.04 mmol) and sodium cyanoborohydride (3 mg, 0.04 mmol). (S)-5-((((6-(2-Chloro-3-(3-chloro-2-(2-((((1r,4r)-4-hydroxycyclohexyl)amino)methyl)-8-methoxy-[1,2,4]triazolo[1,5-a]pyridin-6-yl)pyridin-4-yl)phenyl)-2-methoxypyridin-3-yl)methyl)amino)methyl)pyrrolidin-2-one was prepared using general Boc Deprotection Procedure from tert-butyl ((6-(2-chloro-3-(3-chloro-2-(2-((((1r,4r)-4-hydroxycyclohexyl)amino)methyl)-8-methoxy-[1,2,4]triazolo[1,5-a]pyridin-6-yl)pyridin-4-yl)phenyl)-2-methoxypyridin-3-yl)methyl)(((S)-5-oxopyrrolidin-2-yl)methyl)carbamate and trifluoroacetic acid (24 µL, 0.31 mmol). 2.6 mg, 17% yield. MS: m/z found 731 [M+H]⁺, retention time = 1.53 min (Method D). ¹H NMR (400 MHz, Methanol-*d*₄). δ 8.82 (s, 1H), 8.72 (d, 1H), 7.77 (d, 1H), 7.72 (d, 1H), 7.60 - 7.51 (m, 2H), 7.46 (s, 1H), 7.43 (d, 1H), 7.27 (d, 1H), 4.37 (s, 2H), 4.12 (d, 3H), 4.02 (d, 3H), 3.97 - 3.84 (m, 3H), 2.85 - 2.72 (m, 2H), 2.31 (q, 4H), 2.17 (d, 2H), 2.02 (d, 2H), 1.83 (s, 1H), 1.51 - 1.26 (m, 4H).

### Example 482: (S)-1-((6-(3-Chloro-4-(2-chloro-3-(6-methoxy-5-((((5-oxopyrrolidin-2-yl)methyl)amino)methyl)pyridin-2-yl)phenyl)pyridin-2-yl)-8-methoxy-[1,2,4]triazolo[1,5-a]pyridin-2-yl)methyl)azetidine-3-carboxylic acid (261)

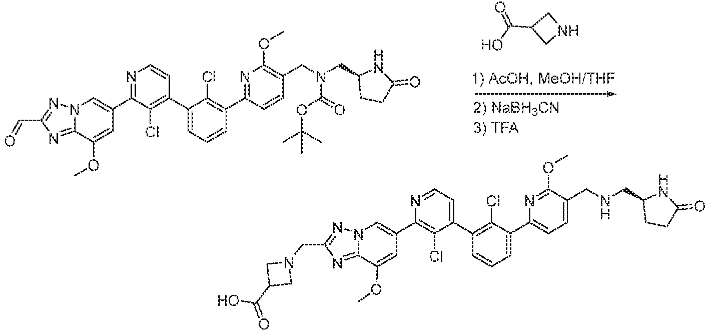

(S)-1-((6-(4-(3-(5-(((tert-Butoxycarbonyl)((5-oxopyrrolidin-2-yl)methyl)amino)methyl)-6-methoxypyridin-2-yl)-2-chlorophenyl)-3-chloropyridin-2-yl)-8-methoxy-[1,2,4]triazolo[1,5-a]pyridin-2-yl)methyl)azetidine-3-carboxylic acid was prepared using Reductive Amination Procedure B from tert-butyl (S)-((6-(2-chloro-3-(3-chloro-2-(2-formyl-8-methoxy-[1,2,4]triazolo[1,5-a]pyridin-6-yl)pyridin-4-yl)phenyl)-2-methoxypyridin-3-yl)methyl)((5-oxopyrrolidin-2-yl)methyl)carbamate (15 mg, 0.02 mmol), acetic acid (2 µL, 0.04 mmol), azetidine-3-carboxylic acid (4 mg, 0.04 mmol) and sodium cyanoborohydride (3 mg, 0.04 mmol). (S)-1-((6-(3-Chloro-4-(2-chloro-3-(6-methoxy-5-((((5-oxopyrrolidin-2-yl)methyl)amino)methyl)pyridin-2-yl)phenyl)pyridin-2-yl)-8-methoxy-[1,2,4]triazolo[1,5-a]pyridin-2-yl)methyl)azetidine-3-carboxylic acid was prepared using General Boc Deprotection Procedure from (S)-1-((6-(4-(3-(5-(((tert-Butoxycarbonyl)((5-oxopyrrolidin-2-yl)methyl)amino)methyl)-6-methoxypyridin-2-yl)-2-chlorophenyl)-3-chloropyridin-2-yl)-8-methoxy-[1,2,4]triazolo[1,5-a]pyridin-2-yl)methyl)azetidine-3-carboxylic acid and trifluoroacetic acid (26 µL, 0.34 mmol). 3.3 mg, 22% yield. MS: m/z found 717 [M+H]⁺, retention time = 1.53 min (Method D). ¹H NMR (400 MHz, Methanol-*d*₄) δ 8.83 (s, 1H), 8.75 - 8.70 (m, 1H), 7.89 (d, 1H), 7.73 (d, 1H), 7.59 (t, 1H), 7.52 (d, 1H), 7.47 (s, 2H), 7.37 (d, 1H), 4.66 (s, 2H), 4.37 (t, 4H), 4.30 (s, 2H), 4.11 (d, 3H), 4.09 (t, 3H), 4.04 (s, 1H), 3.54 (t, 1H), 3.20 (d, 2H), 2.41 (d, 3H), 1.91 (s, 1H).

### Example 483: (S)-1-(2-(((6-(3-Chloro-4-(2-chloro-3-(6-methoxy-5-((((5-oxopyrrolidin-2-yl)methyl)amino)methyl)pyridin-2-yl)phenyl)pyridin-2-yl)-8-methoxy-[1,2,4]triazolo[1,5-a]pyridin-2-yl)methyl)amino)ethyl)cyclopropane-1-carboxylic acid (262)

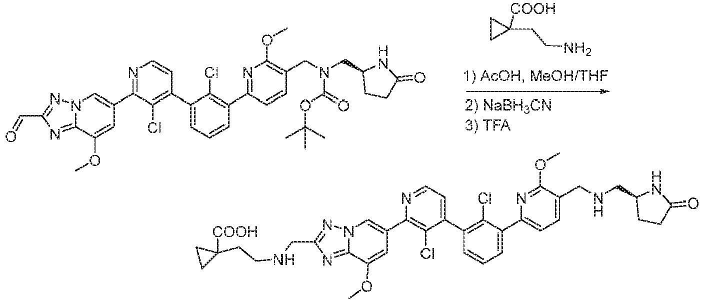

(S)-1-(2-(((6-(4-(3-(5-(((tert-Butoxycarbonyl)((5-oxopyrrolidin-2-yl)methyl)amino)methyl)-6-methoxypyridin-2-yl)-2-chlorophenyl)-3-chloropyridin-2-yl)-8-methoxy-[1,2,4]triazolo[1,5-a]pyridin-2-yl)methyl)amino)ethyl)cyclopropane-1-carboxylic acid was prepared using Reductive Amination Procedure B from tert-butyl (S)-((6-(2-chloro-3-(3-chloro-2-(2-formyl-8-methoxy-[1,2,4]triazolo[1,5-a]pyridin-6-yl)pyridin-4-yl)phenyl)-2-methoxypyridin-3-yl)methyl)((5-oxopyrrolidin-2-yl)methyl)carbamate (15 mg, 0.02 mmol), acetic acid (2 µL, 0.04 mmol), 1-(2-aminoethyl)cyclopropanecarboxylic acid (5 mg, 0.04 mmol) and sodium cyanoborohydride (3 mg, 0.04 mmol). (S)-1-(2-(((6-(3-Chloro-4-(2-chloro-3-(6-methoxy-5-((((5-oxopyrrolidin-2-yl)methyl)amino)methyl)pyridin-2-yl)phenyl)pyridin-2-yl)-8-methoxy-[1,2,4]triazolo[1,5-a]pyridin-2-yl)methyl)amino)ethyl)cyclopropane-1-carboxylic acid was prepared using General Boc Deprotection Procedure from (S)-1-(2-(((6-(4-(3-(5-(((tert-Butoxycarbonyl)((5-oxopyrrolidin-2-yl)methyl)amino)methyl)-6-methoxypyridin-2-yl)-2-chlorophenyl)-3-chloropyridin-2-yl)-8-methoxy-[1,2,4]triazolo[1,5-a]pyridin-2-yl)methyl)amino)ethyl)cyclopropane-1-carboxylic acid and trifluoroacetic acid (27 µL, 0.36 mmol). 2.2 mg, 14% yield. MS: m/z found 745 [M+H]⁺, retention time = 0.61 min (Method B). ¹H NMR (400 MHz, Methanol-*d*₄) δ 8.83 (s, 1H), 8.72 (d, 1H), 7.82 (d, 1H), 7.72 (d, 1H), 7.57 (t, 1H), 7.52 (d, 1H), 7.49 - 7.41 (m, 2H), 7.31 (d, 1H), 4.51 (s, 2H), 4.11 (s, 3H), 4.10 (s, 2H), 4.05 (d, 3H), 3.96 (s, 1H), 3.34 (s, 2H), 2.98 (d, 2H), 2.35 (s, 3H), 1.88 (d, 3H), 1.20 (s, 2H), 0.71 (s, 2H).

### Example 484: (S)-5-((((6-(2-Chloro-3-(3-chloro-2-(2-((((1r,3r)-3-hydroxy-3-methylcyclobutyl)amino)methyl)-8-methoxy-[1,2,4]triazolo[1,5-a]pyridin-6-yl)pyridin-4-yl)phenyl)-2-methoxypyridin-3-yl)methyl)amino)methyl)pyrrolidin-2-one (370)

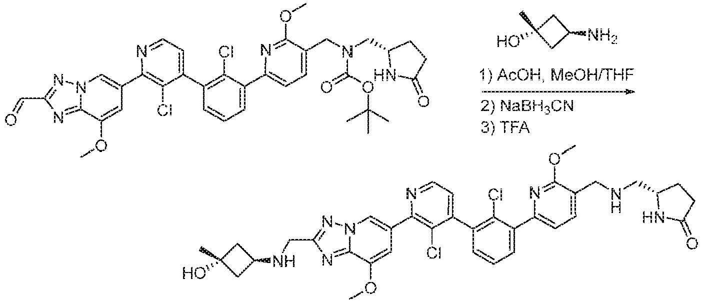

tert-Butyl ((6-(2-chloro-3-(3-chloro-2-(2-((((1r,3r)-3-hydroxy-3-methylcyclobutyl)amino)methyl)-8-methoxy-[1,2,4]triazolo[1,5-a]pyridin-6-yl)pyridin-4-yl)phenyl)-2-methoxypyridin-3-yl)methyl)(((S)-5-oxopyrrolidin-2-yl)methyl)carbamate was prepared using Reductive Amination Procedure B from tert-butyl (S)-((6-(2-chloro-3-(3-chloro-2-(2-formyl-8-methoxy-[1,2,4]triazolo[1,5-a]pyridin-6-yl)pyridin-4-yl)phenyl)-2-methoxypyridin-3-yl)methyl)((5-oxopyrrolidin-2-yl)methyl)carbamate (15 mg, 0.02 mmol), acetic acid (2 µL, 0.04 mmol), 3-amino-1-methyl-cyclobutanol hydrochloride (6 mg, 0.04 mmol) and sodium cyanoborohydride (3 mg, 0.04 mmol). (S)-5-((((6-(2-Chloro-3-(3-chloro-2-(2-((((1r,3r)-3-hydroxy-3-methylcyclobutyl)amino)methyl)-8-methoxy-[1,2,4]triazolo[1,5-a]pyridin-6-yl)pyridin-4-yl)phenyl)-2-methoxypyridin-3-yl)methyl)amino)methyl)pyrrolidin-2-one was prepared using General Boc Deprotection Procedure from tert-Butyl ((6-(2-chloro-3-(3-chloro-2-(2-((((1r,3r)-3-hydroxy-3-methylcyclobutyl)amino)methyl)-8-methoxy-[1,2,4]triazolo[1,5-a]pyridin-6-yl)pyridin-4-yl)phenyl)-2-methoxypyridin-3-yl)methyl)(((S)-5-oxopyrrolidin-2-yl)methyl)carbamate and trifluoroacetic acid (28 µL, 0.36 mmol). 3.7 mg, 25% yield. MS: m/z found 717.1 [M+H]⁺, retention time = 1.92 min (Method D). ¹H NMR (400 MHz, Methanol-*d*₄) δ 8.84 (s, 1H), 8.73 (d, 1H), 7.91 (d, 1H), 7.74 (d, 1H), 7.59 (t, 1H), 7.53 (d, 1H), 7.48 (d, 2H), 7.38 (d, 1H), 4.46 (s, 2H), 4.35 (s, 2H), 4.12 (s, 4H), 4.10 (t, 4H), 4.07 (d, 2H), 3.25 (d, 2H), 2.41 (t, 6H), 2.26 (t, 2H).

### Example 485: 1-(4-(((6-(2-Chloro-3-(3-chloro-2-(2-((((1r,4r)-4-hydroxycyclohexyl)amino)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one (595)

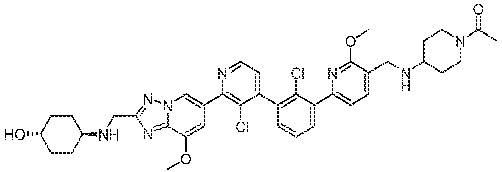

### (a) tert-Butyl (1-acetylpiperidin-4-yl)((6-(2-chloro-3-(3-chloro-2-(2-(hydroxymethyl)-8-methoxy-[1,2,4]triazolo[1,5-a]pyridin-6-yl)pyridin-4-yl)phenyl)-2-methoxypyridin-3-yl)methyl)carbamate

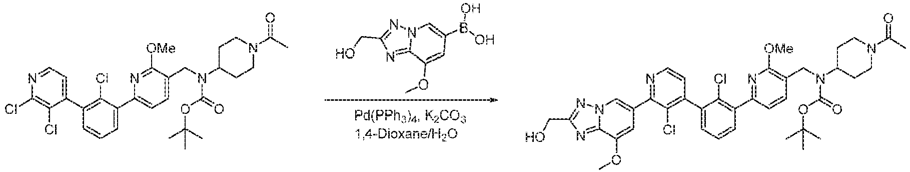

To a mixture of tert-butyl (1-acetylpiperidin-4-yl)((6-(2-chloro-3-(2,3-dichloropyridin-4-yl)phenyl)-2-methoxypyridin-3-yl)methyl)carbamate (417 mg, 0.67 mmol) and (2-(hydroxymethyl)-8-methoxy-[1,2,4]triazolo[1,5-a]pyridin-6-yl)boronic acid (150 mg, 0.67 mmol) in 1,4-dioxane/water (4:1, 3 mL), potassium carbonate (279 mg, 2.02 mmol) was added. Nitrogen gas was bubbled through the mixture for two minutes. Tetrakis(triphenylphosphine)palladium(0) (78 mg, 0.07 mmol) was added, and the reaction was stirred at 100 °C for 30 minutes. The reaction mixture was diluted with water (30 mL), and the aqueous layer was extracted with EtOAc (3 x 30 ml). The combined organic layers were washed with brine (20 mL), dried over anhydrous sodium sulfate, filtered, and concentrated. The residue was purified by normal phase silica gel chromatography (0-5% MeOH in DCM) to afford tert-butyl (1-acetylpiperidin-4-yl)((6-(2-chloro-3-(3-chloro-2-(2-(hydroxymethyl)-8-methoxy-[1,2,4]triazolo[1,5-a]pyridin-6-yl)pyridin-4-yl)phenyl)-2-methoxypyridin-3-yl)methyl)carbamate (339 mg, 66% yield). MS: m/z found 762.2 [M+H]⁺.

### (b) tert-Butyl (1-acetylpiperidin-4-yl)((6-(2-chloro-3-(3-chloro-2-(2-formyl-8-methoxy-[1,2,4]triazolo[1,5-a]pyridin-6-yl)pyridin-4-yl)phenyl)-2-methoxypyridin-3-yl)methyl)carbamate

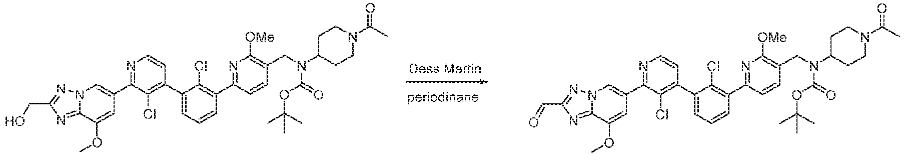

To a solution of tert-butyl (1-acetylpiperidin-4-yl)((6-(2-chloro-3-(3-chloro-2-(2-(hydroxymethyl)-8-methoxy-[1,2,4]triazolo[1,5-a]pyridin-6-yl)pyridin-4-yl)phenyl)-2-methoxypyridin-3-yl)methyl)carbamate (339 mg, 0.44 mmol) in dichloromethane (3 mL), Dess Martin periodinane (377 mg, 0.89 mmol) was added. The reaction was stirred at room temperature for 2 h. The reaction mixture was concentrated and the residue was purified by normal phase silica gel chromatography (0-5% MeOH in DCM) to afford tert-butyl (1-acetylpiperidin-4-yl)((6-(2-chloro-3-(3-chloro-2-(2-formyl-8-methoxy-[1,2,4]triazolo[1,5-a]pyridin-6-yl)pyridin-4-yl)phenyl)-2-methoxypyridin-3-yl)methyl)carbamate (145 mg, 43% yield). MS: m/z found 760.4 [M+H]⁺.

### (c) 1-(4-(((6-(2-Chloro-3-(3-chloro-2-(2-((((1r,4r)-4-hydroxycyclohexyl)amino)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one

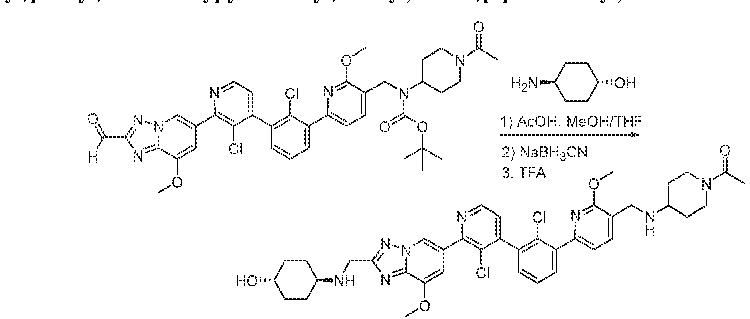

tert-Butyl (1-acetylpiperidin-4-yl)((6-(2-chloro-3-(3-chloro-2-(2-((((1r,4r)-4-hydroxycyclohexyl)amino)methyl)-8-methoxy-[1,2,4]triazolo[1,5-a]pyridin-6-yl)pyridin-4-yl)phenyl)-2-methoxypyridin-3-yl)methyl)carbamate was prepared using Reductive Amination Procedure B from tert-butyl (1-acetylpiperidin-4-yl)((6-(2-chloro-3-(3-chloro-2-(2-formyl-8-methoxy-[1,2,4]triazolo[1,5-a]pyridin-6-yl)pyridin-4-yl)phenyl)-2-methoxypyridin-3-yl)methyl)carbamate (20 mg, 0.03 mmol), acetic acid (3 µL, 3.16 mg, 0.05 mmol), (1r,4r)-4-aminocyclohexan-1-ol (6 mg, 0.05 mmol) and sodium cyanoborohydride (3 mg, 0.05 mmol). 1-(4-(((6-(2-Chloro-3-(3-chloro-2-(2-((((1r,4r)-4-hydroxycyclohexyl)amino)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one was prepared using General Boc Deprotection Procedure from tert-butyl (1-acetylpiperidin-4-yl)((6-(2-chloro-3-(3-chloro-2-(2-((((1r,4r)-4-hydroxycyclohexyl)amino)methyl)-8-methoxy-[1,2,4]triazolo[1,5-a]pyridin-6-yl)pyridin-4-yl)phenyl)-2-methoxypyridin-3-yl)methyl)carbamate and trifluoroacetic acid (32 µL, 0.42 mmol). 5.8 mg, 29% yield. MS: m/z found 761.1 [M+H]⁺, retention time = 1.57 min (Method D). ¹H NMR (400 MHz, Methanol-*d*₄) δ 8.84 (s, 1H), 8.73 (d, 1H), 7.91 (d, 1H), 7.73 (d, 1H), 7.59 (t, 1H), 7.48 (s, 2H), 7.37 (d, 1H), 4.68 (d, 1H), 4.57 (s, 2H), 4.33 (s, 2H), 4.12 (s, 4H), 4.09 (d, 4H), 3.51 (s, 1H), 3.22 (t, 1H), 2.72 (d, 1H), 2.65 (d, 1H), 2.26 (s, 4H), 2.13 (s, 4H), 2.07 (d, 2H), 1.55 (q, 3H), 1.43 - 1.26 (m, 2H).

### Example 486: 1-(4-(((6-(2-Chloro-3-(3-chloro-2-(2-((((1r,3r)-3-hydroxy-3-methylcyclobutyl)amino)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one (596)

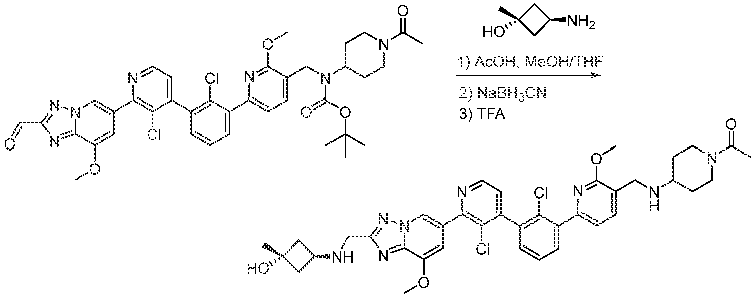

tert-Butyl (1-acetylpiperidin-4-yl)((6-(2-chloro-3-(3-chloro-2-(2-((((1r,3r)-3-hydroxy-3-methylcyclobutyl)amino)methyl)-8-methoxy-[1,2,4]triazolo[1,5-a]pyridin-6-yl)pyridin-4-yl)phenyl)-2-methoxypyridin-3-yl)methyl)carbamate was prepared using Reductive Amination Procedure B form tert-butyl (1-acetylpiperidin-4-yl)((6-(2-chloro-3-(3-chloro-2-(2-formyl-8-methoxy-[1,2,4]triazolo[1,5-a]pyridin-6-yl)pyridin-4-yl)phenyl)-2-methoxypyridin-3-yl)methyl)carbamate (20 mg, 0.03 mmol), acetic acid (3 µL, 0.05 mmol), (1r,3r)-3-amino-1-methylcyclobutan-1-ol hydrochloride (7 mg, 0.05 mmol) and sodium cyanoborohydride (3 mg, 0.05 mmol). 1-(4-(((6-(2-Chloro-3-(3-chloro-2-(2-((((1r,3r)-3-hydroxy-3-methylcyclobutyl)amino)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one was prepared using General Boc Deprotection Procedure from tert-butyl (1-acetylpiperidin-4-yl)((6-(2-chloro-3-(3-chloro-2-(2-((((1r,3r)-3-hydroxy-3-methylcyclobutyl)amino)methyl)-8-methoxy-[1,2,4]triazolo[1,5-a]pyridin-6-yl)pyridin-4-yl)phenyl)-2-methoxypyridin-3-yl)methyl)carbamate and trifluoroacetic acid (0.07 mL, 0.89 mmol). 6 mg, 31% yield. MS: m/z found 747.3 [M+H]⁺, retention time = 1.55 min (Method D). ¹H NMR (400 MHz, Methanol-*d*₄) δ 8.83 (d, 1H), 8.73 (d, 1H), 7.91 (d,1H), 7.73 (d, 1H), 7.59 (t, 1H), 7.55 - 7.50 (m, 1H), 7.48 (t, 2H), 7.36 (d, 1H), 4.67 (d, 1H), 4.39 (d, 2H), 4.30 (s, 2H), 4.12 (d, 3H), 4.08 (d, 4H), 3.45 (d, 1H), 3.21 (t, 1H), 2.70 (t, 1H), 2.39 (t, 2H), 2.30 - 2.18 (m, 4H), 2.13 (s, 4H), 1.72 - 1.59 (m, 1H), 1.54 (dd, 1H), 1.38 (d, 3H).

### Example 487: 1-(4-(((6-(2-Chloro-3-(3-chloro-2-(2-((3-hydroxy-3-methylazetidin-1-yl)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one

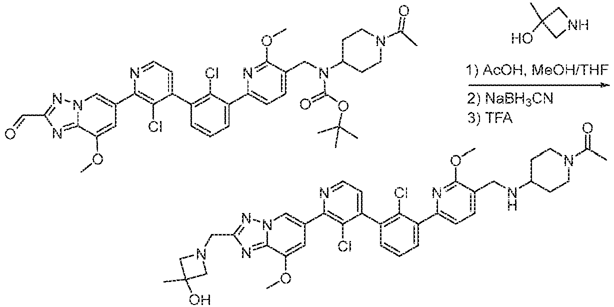

tert-Butyl (1-acetylpiperidin-4-yl)((6-(2-chloro-3-(3-chloro-2-(2-((3-hydroxy-3-methylazetidin-1-yl)methyl)-8-methoxy-[1,2,4]triazolo[1,5-a]pyridin-6-yl)pyridin-4-yl)phenyl)-2-methoxypyridin-3-yl)methyl)carbamate was prepared using Reductive Amination Procedure B from tert-butyl (1-acetylpiperidin-4-yl)((6-(2-chloro-3-(3-chloro-2-(2-formyl-8-methoxy-[1,2,4]triazolo[1,5-a]pyridin-6-yl)pyridin-4-yl)phenyl)-2-methoxypyridin-3-yl)methyl)carbamate (20 mg, 0.03 mmol), acetic acid (3 µL, 0.05 mmol), 3-methylazetidin-3-ol hydrochloride (7 mg, 0.05 mmol) and sodium cyanoborohydride (3 mg, 0.05 mmol). 1-(4-(((6-(2-Chloro-3-(3-chloro-2-(2-((3-hydroxy-3-methylazetidin-1-yl)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one was prepared using General Boc Deprotection Procedure from tert-butyl (1-acetylpiperidin-4-yl)((6-(2-chloro-3-(3-chloro-2-(2-((3-hydroxy-3-methylazetidin-1-yl)methyl)-8-methoxy-[1,2,4]triazolo[1,5-a]pyridin-6-yl)pyridin-4-yl)phenyl)-2-methoxypyridin-3-yl)methyl)carbamate (15 mg, 0.02 mmol) and trifluoroacetic acid (0.07 mL, 0.89 mmol). 4.6 mg, 24% yield. MS: m/z found 731.4 [M+H]⁺, retention time = 1.57 min (Method D). ¹H NMR (400 MHz, Methanol-*d*₄) δ 8.78 (d,1H), 8.71 (d, 1H), 8.52 (d, 1H), 7.81 (d, 1H), 7.72 (d, 1H), 7.57 (t, 1H), 7.51 (d,1H), 7.46 - 7.39 (m, 2H), 7.29 (d, 1H), 4.54 (d, 1H), 4.11 (d, 3H), 4.04 (d,3H), 4.02 (s, 2H), 4.00 (s, 3H), 3.53 (d,2H), 3.35 (d, 3H), 3.17 (t, 1H), 3.03 (s, 1H), 2.70 (t, 1H), 2.11 (s, 3H), 1.47 (s, 4H), 1.37 (d, 1H).

### Example 488: (R)-1-(4-(((6-(2-Chloro-3-(3-chloro-2-(2-(((2-hydroxypropyl)amino)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one (638)

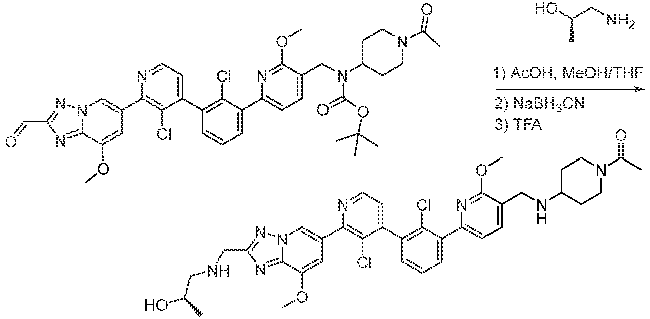

tert-Butyl (R)-(1-acetylpiperidin-4-yl)((6-(2-chloro-3-(3-chloro-2-(2-(((2-hydroxypropyl)amino)methyl)-8-methoxy-[1,2,4]triazolo[1,5-a]pyridin-6-yl)pyridin-4-yl)phenyl)-2-methoxypyridin-3-yl)methyl)carbamate was prepared using Reductive Amination Procedure B from tert-butyl (1-acetylpiperidin-4-yl)((6-(2-chloro-3-(3-chloro-2-(2-formyl-8-methoxy-[1,2,4]triazolo[1,5-a]pyridin-6-yl)pyridin-4-yl)phenyl)-2-methoxypyridin-3-yl)methyl)carbamate (20 mg, 0.03 mmol), acetic acid (3 µL, 0.05 mmol), (2R)-1-aminopropan-2-ol (4 mg, 0.05 mmol) and sodium cyanoborohydride (3 mg, 0.05 mmol). (R)-1-(4-(((6-(2-chloro-3-(3-chloro-2-(2-(((2-hydroxypropyl)amino)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one was prepared using General Boc Deprotection Procedure from tert-Butyl (R)-(1-acetylpiperidin-4-yl)((6-(2-chloro-3-(3-chloro-2-(2-(((2-hydroxypropyl)amino)methyl)-8-methoxy-[1,2,4]triazolo[1,5-a]pyridin-6-yl)pyridin-4-yl)phenyl)-2-methoxypyridin-3-yl)methyl)carbamate and trifluoroacetic acid (0.06 mL, 0.79 mmol). 4.6 mg, 24% yield. MS: m/z found 719.3 [M+H]⁺, retention time = 1.55 min (Method D). ¹H NMR (400 MHz, Methanol-*d*₄) δ 8.79 (s, 1H), 8.71 (d, 1H), 8.53 (s, 1H), 7.80 (d, 1H), 7.72 (d, 1H), 7.57 (t, 1H), 7.51 (d, 1H), 7.44 (d, 2H), 7.28 (d, 1H), 4.52 (d, 1H), 4.17 (d, 2H), 4.11 (s, 3H), 4.03 (d, 3H), 3.98 (s, 3H), 3.95 (s, 2H), 3.16 (t, 1H), 2.97 (s, 1H), 2.86 - 2.78 (m, 1H), 2.70 (t,2H), 2.11 (s, 4H), 2.06 (d, 1H), 1.48 - 1.32 (m, 1H), 1.18 (d, 3H).

### Example 489: 1-(4-(((6-(2-Chloro-3-(3-chloro-2-(2-((isopropylamino)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one (644)

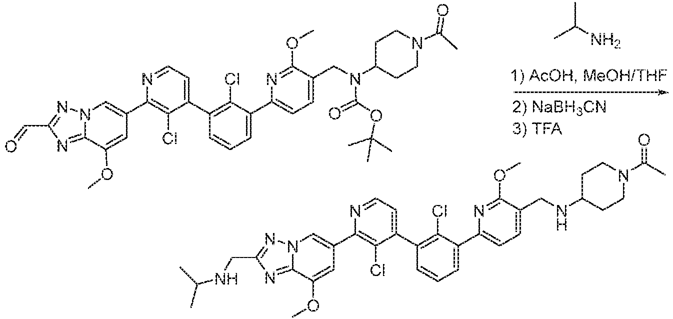

tert-Butyl (1-acetylpiperidin-4-yl)((6-(2-chloro-3-(3-chloro-2-(2-((isopropylamino)methyl)-8-methoxy-[1,2,4]triazolo[1,5-a]pyridin-6-yl)pyridin-4-yl)phenyl)-2-methoxypyridin-3-yl)methyl)carbamate was prepared using Reductive Amination Procedure B from tert-butyl (1-acetylpiperidin-4-yl)((6-(2-chloro-3-(3-chloro-2-(2-formyl-8-methoxy-[1,2,4]triazolo[1,5-a]pyridin-6-yl)pyridin-4-yl)phenyl)-2-methoxypyridin-3-yl)methyl)carbamate (20 mg, 0.03 mmol), acetic acid (3 µL, 0.05 mmol), propan-2-amine (3 mg, 0.05 mmol) and sodium cyanoborohydride (3 mg, 0.05 mmol). 1-(4-(((6-(2-Chloro-3-(3-chloro-2-(2-((isopropylamino)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one was prepared using General Boc Deprotection Procedure from tert-butyl (1-acetylpiperidin-4-yl)((6-(2-chloro-3-(3-chloro-2-(2-((isopropylamino)methyl)-8-methoxy-[1,2,4]triazolo[1,5-a]pyridin-6-yl)pyridin-4-yl)phenyl)-2-methoxypyridin-3-yl)methyl)carbamate and trifluoroacetic acid (0.07 mL, 0.89 mmol). 8.4 mg, 46% yield. MS: m/z found 705.4 [M+H]⁺, retention time = 2.06 min (Method C). ¹H NMR (400 MHz, Methanol-*d*₄) δ 8.84 (s, 1H), 8.73 (d, 1H), 8.07 (s, 1H), 7.91 (d, 1H), 7.73 (d, 1H), 7.59 (t, 1H), 7.53 (d, 1H), 7.47 (d, 2H), 7.36 (d, 1H), 4.68 (d, 1H), 4.55 (s, 2H), 4.33 (s, 2H), 4.12 (s, 4H), 4.08 (s, 4H), 3.54 - 3.45 (m, 1H), 3.22 (t, 1H), 2.76 - 2.61 (m, 1H), 2.26 (t, 2H), 2.13 (s, 4H), 1.70 - 1.52 (m, 1H), 1.43 (d, 6H).

### Example 490: 1-(4-(((6-(2-Chloro-3-(3-chloro-2-(2-(((2-hydroxyethyl)amino)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one (654)

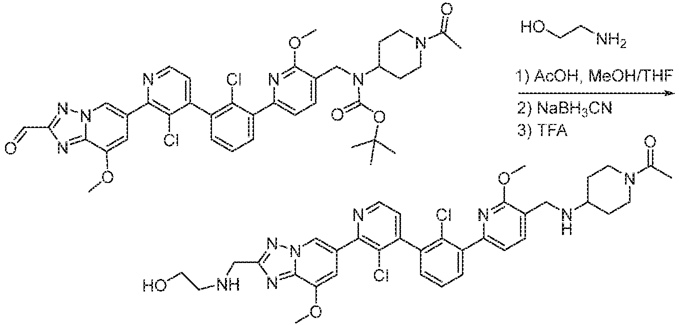

tert-Butyl (1-acetylpiperidin-4-yl)((6-(2-chloro-3-(3-chloro-2-(2-(((2-hydroxyethyl)amino)methyl)-8-methoxy-[1,2,4]triazolo[1,5-a]pyridin-6-yl)pyridin-4-yl)phenyl)-2-methoxypyridin-3-yl)methyl)carbamate was prepared using Reductive Amination Procedure B from tert-butyl (1-acetylpiperidin-4-yl)((6-(2-chloro-3-(3-chloro-2-(2-formyl-8-methoxy-[1,2,4]triazolo[1,5-a]pyridin-6-yl)pyridin-4-yl)phenyl)-2-methoxypyridin-3-yl)methyl)carbamate (20 mg, 0.03 mmol), acetic acid (3 µL, 0.05 mmol), 2-aminoethanol (3.21 mg, 0.05 mmol) and sodium cyanoborohydride (3 mg, 0.05 mmol). 1-(4-(((6-(2-Chloro-3-(3-chloro-2-(2-(((2-hydroxyethyl)amino)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one was prepared using General Boc Deprotection Procedure from tert-butyl (1-acetylpiperidin-4-yl)((6-(2-chloro-3-(3-chloro-2-(2-(((2-hydroxyethyl)amino)methyl)-8-methoxy-[1,2,4]triazolo[1,5-a]pyridin-6-yl)pyridin-4-yl)phenyl)-2-methoxypyridin-3-yl)methyl)carbamate (15 mg, 0.02 mmol) and trifluoroacetic acid (0.07 mL, 0.89 mmol). 7 mg, 50% yield. MS: m/z found 705.6 [M+H]⁺, retention time = 1.98 min (Method C). ¹H NMR (400 MHz, Methanol-*d*₄) δ 8.84 (s, 1H), 8.73 (d, 1H), 7.91 (d, 1H), 7.73 (d, 1H), 7.59 (t, 1H), 7.53 (dd, 1H), 7.47 (d, 2H), 7.36 (d, 1H), 4.67 (d, 1H), 4.54 (s, 2H), 4.30 (s, 2H), 4.12 (d, 3H), 4.08 (d, 4H), 3.86 (t, 2H), 3.51 - 3.41 (m, 1H), 3.21 (t, 1H), 2.76 - 2.62 (m, 1H), 2.25 (t, 2H), 2.13 (d, 3H), 1.71 - 1.46 (m, 1H).

### Example 491: 2-((6-(4-(3-(5-(((1-Acetylpiperidin-4-yl)amino)methyl)-6-methoxypyridin-2-yl)-2-chlorophenyl)-3-chloropyridin-2-yl)-8-methoxy-[1,2,4]triazolo[1,5-a]pyridin-2-yl)methyl)-2,6-diazaspiro[3.4]octan-7-one (659)

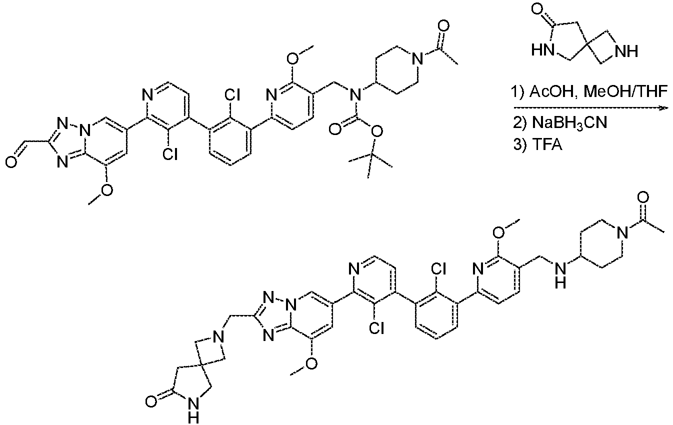

tert-Butyl (1-acetylpiperidin-4-yl)((6-(2-chloro-3-(3-chloro-2-(8-methoxy-2-((7-oxo-2,6-diazaspiro[3.4]octan-2-yl)methyl)-[1,2,4]triazolo[1,5-a]pyridin-6-yl)pyridin-4-yl)phenyl)-2-methoxypyridin-3-yl)methyl)carbamate was prepared using Reductive Amination Procedure B from tert-butyl (1-acetylpiperidin-4-yl)((6-(2-chloro-3-(3-chloro-2-(2-formyl-8-methoxy-[1,2,4]triazolo[1,5-a]pyridin-6-yl)pyridin-4-yl)phenyl)-2-methoxypyridin-3-yl)methyl)carbamate (20 mg, 0.03 mmol), acetic acid (3 µL, 0.05 mmol), 2,6-diazaspiro[3.4]octan-7-one hydrochloride (9 mg, 0.05 mmol) and sodium cyanoborohydride (3 mg, 0.05 mmol). 2-((6-(4-(3-(5-(((1-Acetylpiperidin-4-yl)amino)methyl)-6-methoxypyridin-2-yl)-2-chlorophenyl)-3-chloropyridin-2-yl)-8-methoxy-[1,2,4]triazolo[1,5-a]pyridin-2-yl)methyl)-2,6-diazaspiro[3.4]octan-7-one was prepared using General Boc Deprotection Procedure from tert-butyl (1-acetylpiperidin-4-yl)((6-(2-chloro-3-(3-chloro-2-(8-methoxy-2-((7-oxo-2,6-diazaspiro[3.4]octan-2-yl)methyl)-[1,2,4]triazolo[1,5-a]pyridin-6-yl)pyridin-4-yl)phenyl)-2-methoxypyridin-3-yl)methyl)carbamate and trifluoroacetic acid (0.06 mL, 0.80 mmol). 8.2 mg, 40% yield. MS: m/z found 770.4 [M+H]⁺, retention time = 1.49 min (Method D). ¹H NMR (400 MHz, Methanol-*d*₄) δ 8.78 (d, 1H), 8.72 (dd, 1H), 8.45 (s, 1H), 7.88 (d, 1H), 7.73 (d, 1H), 7.58 (t, 1H), 7.51 (dd, 1H), 7.48 - 7.44 (m, 1H), 7.42 (s, 1H), 7.34 (d, 1H), 4.63 (d, 1H), 4.22 (s, 2H), 4.10 (d, 3H), 4.07 (d, 3H), 4.04 (s, 1H), 3.99 (d, 2H), 3.58 (s, 2H), 3.56 (s, 3H), 3.36 (d, 1H), 3.20 (t, 1H), 2.70 (t, 1H), 2.58 (s, 2H), 2.21 (t, 2H), 2.13 (s, 3H), 1.66 - 1.42 (m, 1H).

### Example 492: 1-(4-(((6-(2-Chloro-3-(3-chloro-2-(2-((6-hydroxy-2-azaspiro[3.3]heptan-2-yl)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one (660)

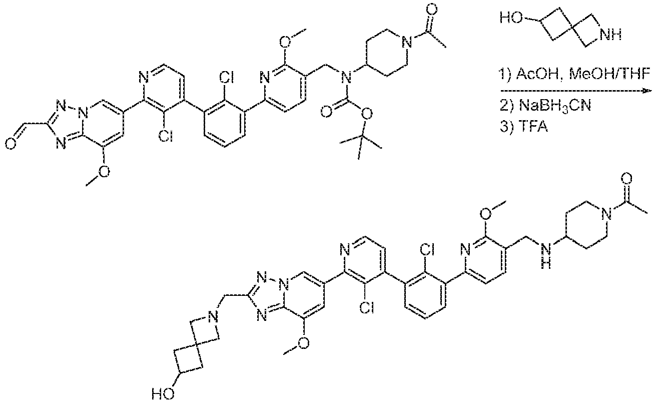

tert-Butyl (1-acetylpiperidin-4-yl)((6-(2-chloro-3-(3-chloro-2-(2-((6-hydroxy-2-azaspiro[3.3]heptan-2-yl)methyl)-8-methoxy-[1,2,4]triazolo[1,5-a]pyridin-6-yl)pyridin-4-yl)phenyl)-2-methoxypyridin-3-yl)methyl)carbamate was prepared using Reductive Amination Procedure B from tert-butyl (1-acetylpiperidin-4-yl)((6-(2-chloro-3-(3-chloro-2-(2-formyl-8-methoxy-[1,2,4]triazolo[1,5-a]pyridin-6-yl)pyridin-4-yl)phenyl)-2-methoxypyridin-3-yl)methyl)carbamate (20 mg, 0.03 mmol), acetic acid (3 µL, 0.05 mmol), 2-azaspiro[3.3]heptan-6-ol hydrochloride (8 mg, 0.05 mmol) and sodium cyanoborohydride (3 mg, 0.05 mmol). 1-(4-(((6-(2-Chloro-3-(3-chloro-2-(2-((6-hydroxy-2-azaspiro[3.3]heptan-2-yl)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one was prepared using General Boc Deprotection Procedure from tert-butyl (1-acetylpiperidin-4-yl)((6-(2-chloro-3-(3-chloro-2-(2-((6-hydroxy-2-azaspiro[3.3]heptan-2-yl)methyl)-8-methoxy-[1,2,4]triazolo[1,5-a]pyridin-6-yl)pyridin-4-yl)phenyl)-2-methoxypyridin-3-yl)methyl)carbamate and trifluoroacetic acid (0.06 mL, 0.76 mmol). 6.4 mg, 32% yield. MS: m/z found 757.4 [M]⁺, retention time = 1.54 min (Method D). ¹H NMR (400 MHz, Methanol-*d₄*). δ 8.79 (s, 1H), 8.72 (d, 1H), 8.48 (s, 1H), 7.86 (d, 1H), 7.73 (d,1H), 7.58 (t, 1H), 7.52 (d, 1H), 7.45 (d, 2H), 7.33 (d, 1H), 4.61 (d, 1H), 4.20 (s, 2H), 4.17 (s, 2H), 4.11 (s, 3H), 4.06 (d, 3H), 4.02 (s, 1H), 3.81 (s, 2H), 3.78 (s, 2H), 3.19 (t, 1H), 2.70 (t, 1H), 2.54 (t, 2H), 2.20 (d, 2H), 2.12 (s, 3H), 2.07 (t, 2H), 1.63 - 1.39 (m, 1H).

### Example 493: 1-(4-(((6-(2-Chloro-3-(3-chloro-2-(2-(((2-hydroxyethyl)amino)methyl)-4-methoxypyrrolo[2,1-f][1,2,4]triazin-6-yl)pyridin-4-yl)phenyl)-2-methoxypyridin-3-yl)methyl)amino)piperidin-1-yl)ethan-1-one (661)

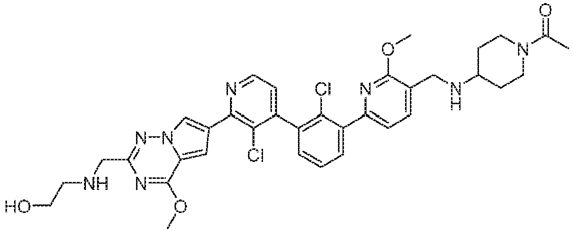

### (a) Ethyl 6-bromo-4-chloropyrrolo[2,1-f][1,2,4]triazine-2-carboxylate

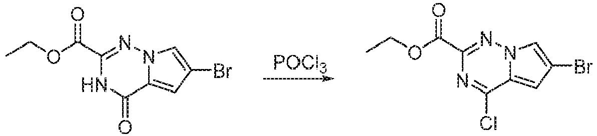

A mixture of ethyl 6-bromo-4-oxo-3,4-dihydropyrrolo[2,1-f][1,2,4]triazine-2-carboxylate (100 mg, 0.35 mmol) and phosphorus oxychloride (0.49 mL, 5.24 mmol) in toluene (3 mL) was heated to reflux for 18 h. The mixture was concentrated, and the residue was diluted with dichloromethane (10 mL) and sodium bicarbonate solution (10 mL). The resulting mixture was stirred at room temperature for 10 min. The separated organic layer was washed with cold brine (30 mL), dried, and concentrated under reduced pressure. The crude material was purified by chromatography on silica gel eluting with dichloromethane to provide ethyl 6-bromo-4-chloropyrrolo[2,1-f][1,2,4]triazine-2-carboxylate (76 mg, 71% yield). MS: m/z found 306 [M+H]⁺.

### (b) Methyl 6-bromo-4-methoxypyrrolo[2,1-f][1,2,4]triazine-2-carboxylate

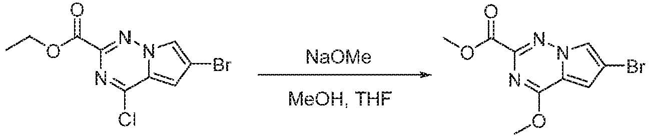

To a solution of ethyl 6-bromo-4-chloropyrrolo[2,1-f][1,2,4]triazine-2-carboxylate (175 mg, 0.57 mmol) in THF (2 mL) at 0 °C was added sodium methoxide in methanol (25% w/w, 37 mg, 0.69 mmol). The reaction was stirred at 0 °C for 1h. Additional sodium methoxide in methanol (25% w/w, 37 mg, 0.69 mmol) was added and the reaction was stirred for another1 h. The reaction was diluted with ethyl acetate (15 mL), washed with ammonium chloride solution and brine. The organic layer was dried, concentrated and the residue was purified by chromatography on silica gel (eluting with 50% ethyl acetate in hexane) to afford methyl 6-bromo-4-methoxypyrrolo[2,1-f][1,2,4]triazine-2-carboxylate (85 mg, 52% yield). MS: m/z found 288 [M+H]⁺.

### (c) (6-Bromo-4-methoxypyrrolo[2,1-f][1,2,4]triazin-2-yl)methanol

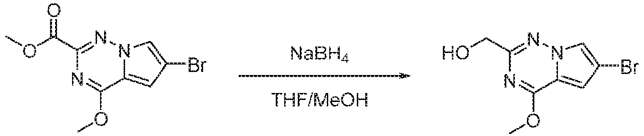

To a solution of methyl 6-bromo-4-methoxypyrrolo[2,1-f][1,2,4]triazine-2-carboxylate (85 mg, 0.30 mmol) in THF/MeOH (1: 1, 1 mL) at 0°C, sodium borohydride (34 mg, 0.89 mmol) was added. The mixture was stirred at 20°C for 2 hours. LCMS suggested conversion to the desired product. The reaction was quenched by addition of water (5 mL) and extracted with EtOAc (3 x 10 ml). The combined organic layers were washed with brine (20 mL), dried over anhydrous sodium sulfate, filtered and concentrated under reduced pressure. The residue was purified by SiO₂ chromatography (0-5% MeOH in DCM) to afford (6-bromo-4-methoxypyrrolo[2,1-f][1,2,4]triazin-2-yl)methanol (39 mg, 51% yield). MS: m/z found 260.0 [M+H]⁺.

### (d) (4-Methoxy-6-(4,4,5,5-tetramethyl-1,3,2-dioxaborolan-2-yl)pyrrolo[2,1-f][1,2,4]triazin-2-yl)methanol

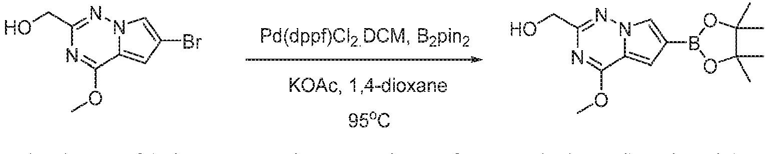

A mixture of (6-bromo-4-methoxypyrrolo[2,1-f][1,2,4]triazin-2-yl)methanol (55 mg, 0.21 mmol), bis(pinacolato)diborane (81 mg, 0.32 mmol), potassium acetate (42 mg, 0.43 mmol) and [1,1'- bis(diphenylphosphino)ferrocene]dichloropalladium(II) complex with dichloromethane (17 mg, 0.02 mmol) in 3 ml 1,4-dioxane was degassed and heated at 95 °C for 3 h. The reaction was concentrated and the residue was purified by silica gel chromatography (0-100% EtOAc in Hexanes) to afford (4-methoxy-6-(4,4,5,5-tetramethyl-1,3,2-dioxaborolan-2-yl)pyrrolo[2,1-f][1,2,4]triazin-2-yl)methanol (29 mg, 45% yield). MS: m/z found 306.2 [M+H]⁺.

### (e) tert-Butyl (1-acetylpiperidin-4-yl)((6-(2-chloro-3-(3-chloro-2-(2-(hydroxymethyl)-4-methoxypyrrolo[2,1-f][1,2,4]triazin-6-yl)pyridin-4-yl)phenyl)-2-methoxypyridin-3-yl)methyl)carbamate

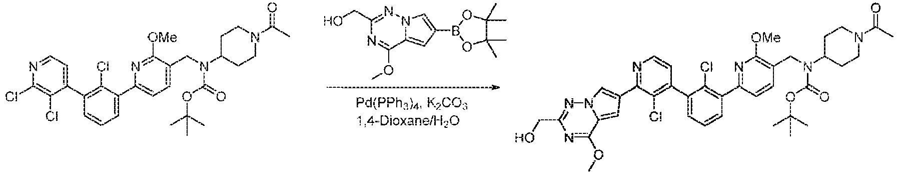

To a mixture of (4-methoxy-6-(4,4,5,5-tetramethyl-1,3,2-dioxaborolan-2-yl)pyrrolo[2,1-f][1,2,4]triazin-2-yl)methanol (28 mg, 0.09 mmol) and tert-butyl (1-acetylpiperidin-4-yl)((6-(2-chloro-3-(2,3-dichloropyridin-4-yl)phenyl)-2-methoxypyridin-3-yl)methyl)carbamate (57 mg, 0.09 mmol) in 1,4-dioxane/ water (4:1, 1 mL) was added potassium carbonate (38 mg, 0.28 mmol) and tetrakis(triphenylphosphine)palladium(0) (11 mg, 0.01 mmol). The mixture was stirred at 110 °C for 30 minutes. The solvent was evaporated and the residue was purified by silica gel chromatography (0-10% MeOH in DCM) to afford tert-butyl (1-acetylpiperidin-4-yl)((6-(2-chloro-3-(3-chloro-2-(2-(hydroxymethyl)-4-methoxypyrrolo[2,1-f][1,2,4]triazin-6-yl)pyridin-4-yl)phenyl)-2-methoxypyridin-3-yl)methyl)carbamate (31 mg, 44% yield). MS: m/z found 762.4 [M+H]⁺.

### (f) tert-Butyl (1-acetylpiperidin-4-yl)((6-(2-chloro-3-(3-chloro-2-(2-(((2-hydroxyethyl)amino)methyl)-4-methoxypyrrolo[2,1-f][1,2,4]triazin-6-yl)pyridin-4-yl)phenyl)-2-methoxypyridin-3-yl)methyl)carbamate

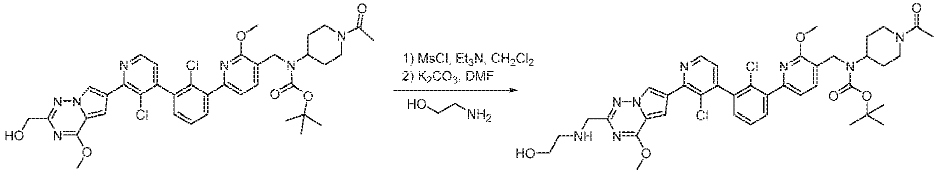

To a solution of tert-butyl (1-acetylpiperidin-4-yl)((6-(2-chloro-3-(3-chloro-2-(2-(hydroxymethyl)-4-methoxypyrrolo[2,1-f][1,2,4]triazin-6-yl)pyridin-4-yl)phenyl)-2-methoxypyridin-3-yl)methyl)carbamate (30 mg, 0.04 mmol) in dichloromethane (0.5 mL), triethylamine (11 µL, 0.08 mmol) and methanesulfonyl chloride (6 µL, 0.08 mmol) were added. The reaction was stirred at room temperature for 1 h. LCMS suggested formation of the desired mesylated product. The solvent was evaporated, and the residue was suspended in DMF (0.4 mL) followed by addition of potassium carbonate (11 mg, 0.08 mmol) and 2-aminoethanol (5 mg, 0.08 mmol). The mixture was stirred at room temperature for 12 h. The solvent was evaporated, and the residue was suspended in water. The precipitate formed was collected to afford tert-butyl (1-acetylpiperidin-4-yl)((6-(2-chloro-3-(3-chloro-2-(2-(((2-hydroxyethyl)amino)methyl)-4-methoxypyrrolo[2,1-f][1,2,4]triazin-6-y])pyridin-4-yl)phenyl)-2-methoxypyridin-3-yl)methyl)carbamate (4 mg, 13% yield). MS: m/z found 805.4 [M+H]⁺.

### (g) 1-(4-(((6-(2-Chloro-3-(3-chloro-2-(2-(((2-hydroxyethyl)amino)methyl)-4-methoxypyrrolo[2,1-f][1,2,4]triazin-6-yl)pyridin-4-yl)phenyl)-2-methoxypyridin-3-yl)methyl)amino)piperidin-1-yl)ethan-1-one

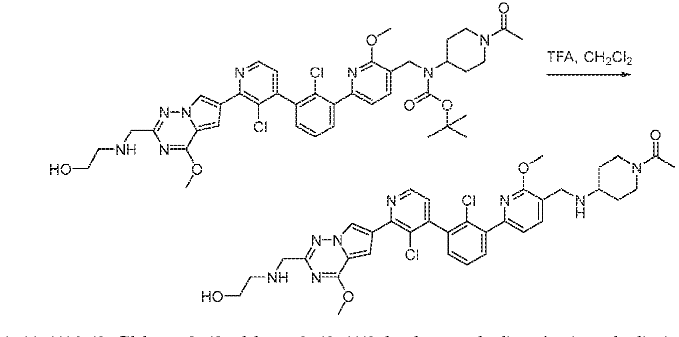

1-(4-(((6-(2-Chloro-3-(3-chloro-2-(2-(((2-hydroxyethyl)amino)methyl)-4-methoxypyrrolo[2,1-f][1,2,4]triazin-6-yl)pyridin-4-yl)phenyl)-2-methoxypyridin-3-yl)methyl)amino)piperidin-1-yl)ethan-1-one was prepared using General Boc Deprotection Procedure from tert-butyl (1-acetylpiperidin-4-yl)((6-(2-chloro-3-(3-chloro-2-(2-(((2-hydroxyethyl)amino)methyl)-4-methoxypyrrolo[2,1-f][1,2,4]triazin-6-yl)pyridin-4-yl)phenyl)-2-methoxypyridin-3-yl)methyl)carbamate (4 mg, 0.005 mmol). 1.6 mg, 46% yield. MS: m/z found 707.2 [M+H]⁺, retention time = 2.13 min (Method C). ¹H NMR (400 MHz, Methanol-*d4*) δ 8.64 (d, 1H), 8.52 (s, 1H), 7.90 (d, 1H), 7.72 (d, 1H), 7.59 (d, 2H), 7.43 (d, 1H), 7.39 - 7.33 (m, 2H), 4.65 (s, 1H), 4.36 (s, 2H), 4.30 (s, 2H), 4.23 (d, 3H), 4.09 (s, 4H), 3.89 (s, 2H), 2.24 (s, 2H), 2.13 (d, 3H).

### Example 494: 1-(4-(((6-(2-Chloro-3-(3-chloro-2-(8-ethoxy-2-((((1r,4r)-4-hydroxycyclohexyl)amino)methyl)-[1,2,4]triazolo[1,5-a]pyridin-6-yl)pyridin-4-yl)phenyl)-2-methoxypyridin-3-yl)methyl)amino)piperidin-1-yl)ethan-1-one (583)

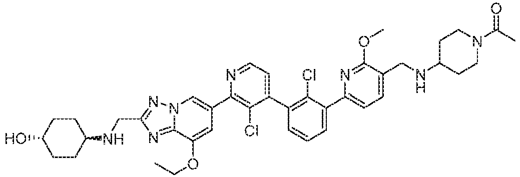

### (a) (6-Bromo-8-ethoxy-[1,2,4]triazolo[1,5-a]pyridin-2-yl)methanol

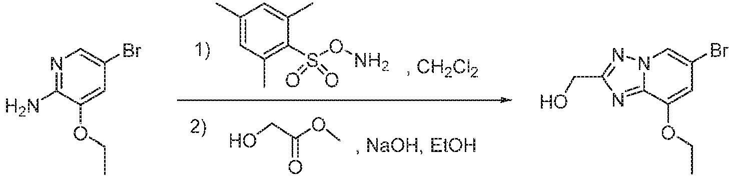

To a suspension of amino 2,4,6-trimethylbenzene-1-sulfonate (195 mg, 0.92 mmol) in dichloromethane (110 mL) was added 5-bromo-3-ethoxy-pyridin-2-amine (200 mg, 0.92 mmol) at 0-5 °C. The ice bath was removed after 10 min and the reaction mixture was stirred at room temperature for 1 h. The suspension was diluted with diethyl ether (100 mL), filtered, washed with diethyl ether, and dried under reduced pressure to afford 5-bromo-3-ethoxy-2-imino-pyridin-1-amine 2,4,6-trimethylbenzenesulfonic acid (124 mg, 32% yield). To a solution of 5-bromo-3-ethoxy-2-imino-pyridin-1-amine 2,4,6-trimethylbenzenesulfonic acid (124 mg, 0.30 mmol) in ethanol (2 mL) was added sodium hydroxide (24 mg, 0.59 mmol). The mixture was heated to 60°C for 1 h. Methyl 2-hydroxyacetate (53 mg, 0.59 mmol) was added and the mixture was heated to 80°C for 3 h. The resulting mixture was cooled to room temperature and concentrated under reduced pressure. The residue was washed with water (10 mL) to afford (6-Bromo-8-ethoxy-[1,2,4]triazolo[1,5-a]pyridin-2-yl)methanol (36 mg, 45% yield). MS: m/z found 272 [M+H]⁺.

### (b) [8-ethoxy-2-(hydroxymethyl)-[1,2,4]triazolo[1,5-a]pyridin-6-yl]boronic acid

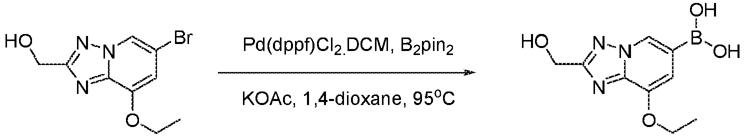

(6-Bromo-8-ethoxy-[1,2,4]triazolo[1,5-a]pyridin-2-yl)methanol (36 mg, 0.13 mmol), bis(pinacolato)diborane (50 mg, 0.20 mmol), potassium acetate (39 mg, 0.40 mmol) and [1,1'- bis(diphenylphosphino)ferrocene]dichloropalladium(II) complex with dichloromethane (11 mg, 0.01 mmol) were suspended in 10 ml 1,4-dioxane. Nitrogen gas was bubbled through the reaction for 2 minutes and then the reaction was heated at 95 °C for 3 h. The reaction was concentrated to afford [8-ethoxy-2-(hydroxymethyl)-[1,2,4]triazolo[1,5-a]pyridin-6-yl]boronic acid (30.00 mg, 96% yield). The material was used in the next step without further purification. MS: m/z found 238.1 [M+H]⁺.

### (c) tert-Butyl (1-acetylpiperidin-4-yl)((6-(2-chloro-3-(3-chloro-2-(8-ethoxy-2-(hydroxymethyl)-[1,2,4]triazolo[1,5-a]pyridin-6-yl)pyridin-4-yl)phenyl)-2-methoxypyridin-3-yl)methyl)carbamate

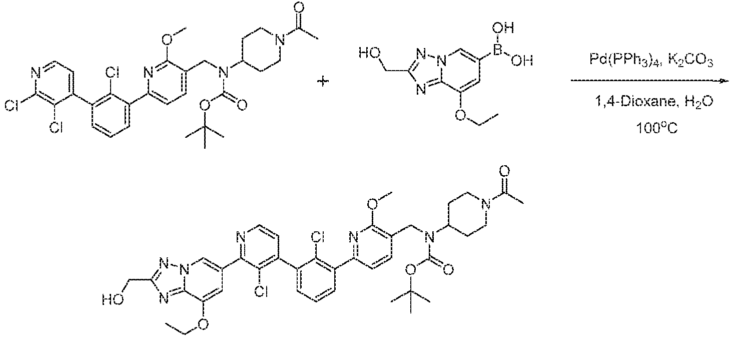

To a mixture of tert-butyl (1-acetylpiperidin-4-yl)((6-(2-chloro-3-(2,3-dichloropyridin-4-yl)phenyl)-2-methoxypyridin-3-yl)methyl)carbamate (78 mg, 0.13 mmol) and [8-ethoxy-2-(hydroxymethyl)-[1,2,4]triazolo[1,5-a]pyridin-6-yl]boronic acid (30 mg, 0.13 mmol) in 1,4-dioxane/water (4:1, 3 mL), potassium carbonate (52 mg, 0.38 mmol) was added. Nitrogen gas was bubbled through the mixture for two minutes. Tetrakis(triphenylphosphine)palladium(0) (29 mg, 0.03 mmol) was added, and the reaction was stirred at 100 °C for 30 minutes. The reaction mixture was diluted with water (30 mL), and the aqueous layer was extracted with EtOAc (3 x 30 ml). The combined organic layers were washed with brine (20 mL), dried over anhydrous sodium sulfate, filtered, and concentrated. The residue was purified by normal phase silica gel chromatography (0-5% MeOH in DCM) to afford tert-butyl (1-acetylpiperidin-4-yl)((6-(2-chloro-3-(3-chloro-2-(8-ethoxy-2-(hydroxymethyl)-[1,2,4]triazolo[1,5-a]pyridin-6-yl)pyridin-4-yl)phenyl)-2-methoxypyridin-3-yl)methyl)carbamate (89 mg, 91% yield). MS: m/z found 776.2 [M+H]⁺.

### (d) tert-Butyl (1-acetylpiperidin-4-yl)((6-(2-chloro-3-(3-chloro-2-(8-ethoxy-2-formyl-[1,2,4]triazolo[1,5-a]pyridin-6-yl)pyridin-4-yl)phenyl)-2-methoxypyridin-3-yl)methyl)carbamate

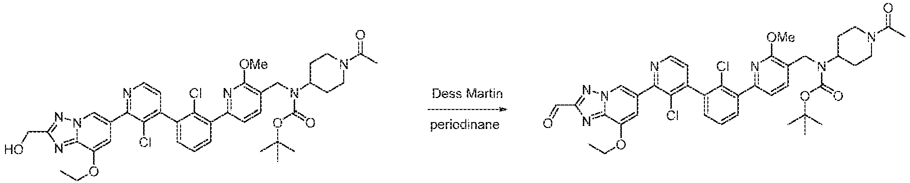

To a solution of tert-butyl (1-acetylpiperidin-4-yl)((6-(2-chloro-3-(3-chloro-2-(8-ethoxy-2-(hydroxymethyl)-[1,2,4]triazolo[1,5-a]pyridin-6-yl)pyridin-4-yl)phenyl)-2-methoxypyridin-3-yl)methyl)carbamate (89 mg, 0.11 mmol) in dichloromethane (3 mL), Dess Martin periodinane (97 mg, 0.23 mmol) was added. The reaction was stirred at room temperature for 2 h. The reaction mixture was concentrated and the residue was purified by normal phase silica gel chromatography (0-5% MeOH in DCM) to afford tert-butyl (1-acetylpiperidin-4-yl)((6-(2-chloro-3-(3-chloro-2-(8-ethoxy-2-formyl-[1,2,4]triazolo[1,5-a]pyridin-6-yl)pyridin-4-yl)phenyl)-2-methoxypyridin-3-yl)methyl)carbamate (71 mg, 80%). MS: m/z found 774 [M+H]⁺.

### (e) tert-Butyl (1-acetylpiperidin-4-yl)((6-(2-chloro-3-(3-chloro-2-(8-ethoxy-2-((((1r,4r)-4-hydroxycyclohexyl)amino)methyl)-[1,2,4]triazolo[1,5-a]pyridin-6-yl)pyridin-4-yl)phenyl)-2-methoxypyridin-3-yl)methyl)carbamate

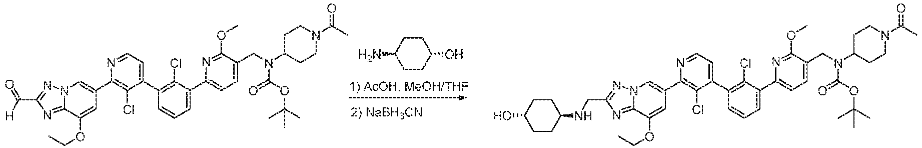

tert-Butyl (1-acetylpiperidin-4-yl)((6-(2-chloro-3-(3-chloro-2-(8-ethoxy-2-((((1r,4r)-4-hydroxycyclohexyl)amino)methyl)-[1,2,4]triazolo[1,5-a]pyridin-6-yl)pyridin-4-yl)phenyl)-2-methoxypyridin-3-yl)methyl)carbamate was prepared using Reductive Amination Procedure B from tert-butyl (1-acetylpiperidin-4-yl)((6-(2-chloro-3-(3-chloro-2-(8-ethoxy-2-formyl-[1,2,4]triazolo[1,5-a]pyridin-6-yl)pyridin-4-yl)phenyl)-2-methoxypyridin-3-yl)methyl)carbamate (15 mg, 0.02 mmol), acetic acid (2 µL, 0.04 mmol), (1r,4r)-4-aminocyclohexan-1-ol (4 mg, 0.04 mmol) and sodium cyanoborohydride (2 mg, 0.04 mmol). 13 mg, 77% yield. MS: m/z found 875.0 [M+H]⁺.

### (f) 1-(4-(((6-(2-Chloro-3-(3-chloro-2-(8-ethoxy-2-((((1r,4r)-4-hydroxycyclohexyl)amino)methyl)-[1,2,4]triazolo[1,5-a]pyridin-6-yl)pyridin-4-yl)phenyl)-2-methoxypyridin-3-yl)methyl)amino)piperidin-1-yl)ethan-1-one

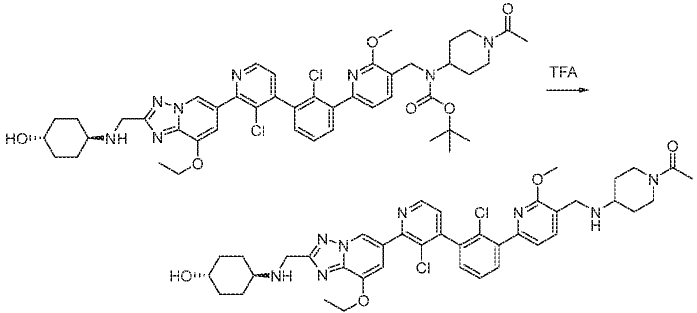

1-(4-(((6-(2-Chloro-3-(3-chloro-2-(8-ethoxy-2-((((1r,4r)-4-hydroxycyclohexyl)amino)methyl)-[1,2,4]triazolo[1,5-a]pyridin-6-yl)pyridin-4-yl)phenyl)-2-methoxypyridin-3-yl)methyl)amino)piperidin-1-yl)ethan-1-one was prepared using General Boc Deprotection Procedure from tert-butyl (1-acetylpiperidin-4-yl)((6-(2-chloro-3-(3-chloro-2-(8-ethoxy-2-((((1r,4r)-4-hydroxycyclohexyl)amino)methyl)-[1,2,4]triazolo[1,5-a]pyridin-6-yl)pyridin-4-yl)phenyl)-2-methoxypyridin-3-yl)methyl)carbamate (13 mg, 0.01 mmol) and trifluoroacetic acid (23 µL, 0.30 mmol). 9.4 mg, 82% yield. MS: m/z found 774.3 [M+H]⁺, retention time = 1.67 min (Method D). ¹H NMR (400 MHz, Methanol-*d*₄) δ 8.77 (s, 1H), 8.67 (d, 1H), 7.87 (d, 1H), 7.68 (d, 1H), 7.54 (t, 1H), 7.48 (d, 1H), 7.42 (s, 2H), 7.31 (d, 1H), 4.62 (d, 2H), 4.49 (s, 2H), 4.32 (d, 2H), 4.28 (s, 2H), 4.03 (s, 5H), 3.60 - 3.39 (m, 2H), 2.65 (t, 2H), 2.20 (s, 5H), 2.02 (d, 3H), 1.63 (s, 1H), 1.50 (d, 7H).

### Example 495: 1-(4-(((6-(2-Chloro-3-(3-chloro-2-(8-ethoxy-2-((((1r,3r)-3-hydroxy-3-methylcyclobutyl)amino)methyl)-[1,2,4]triazolo[1,5-a]pyridin-6-yl)pyridin-4-yl)phenyl)-2-methoxypyridin-3-yl)methyl)amino)piperidin-1-yl)ethan-1-one (584)

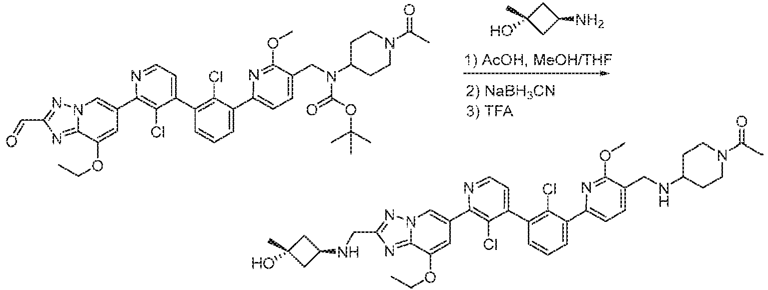

tert-Butyl (1-acetylpiperidin-4-yl)((6-(2-chloro-3-(3-chloro-2-(8-ethoxy-2-((((1r,3r)-3-hydroxy-3-methylcyclobutyl)amino)methyl)-[1,2,4]triazolo[1,5-a]pyridin-6-yl)pyridin-4-yl)phenyl)-2-methoxypyridin-3-yl)methyl)carbamate was prepared using Reductive Amination Procedure B from tert-butyl (1-acetylpiperidin-4-yl)((6-(2-chloro-3-(3-chloro-2-(8-ethoxy-2-formyl-[1,2,4]triazolo[1,5-a]pyridin-6-yl)pyridin-4-yl)phenyl)-2-methoxypyridin-3-yl)methyl)carbamate (Example 494, step (d)) (15 mg, 0.02 mmol), acetic acid (2 µL, 0.04 mmol), (1r,3r)-3-amino-1-methylcyclobutan-1-ol hydrochloride (5 mg, 0.04 mmol) and sodium cyanoborohydride (2 mg, 0.04 mmol). 1-(4-(((6-(2-Chloro-3-(3-chloro-2-(8-ethoxy-2-((((1r,3r)-3-hydroxy-3-methylcyclobutyl)amino)methyl)-[1,2,4]triazolo[1,5-a]pyridin-6-yl)pyridin-4-yl)phenyl)-2-methoxypyridin-3-yl)methyl)amino)piperidin-1-yl)ethan-1-one was prepared using the General Boc Deprotection Procedure from tert-butyl (1-acetylpiperidin-4-yl)((6-(2-chloro-3-(3-chloro-2-(8-ethoxy-2-((((1r,3r)-3-hydroxy-3-methylcyclobutyl)amino)methyl)-[1,2,4]triazolo[1,5-a]pyridin-6-yl)pyridin-4-yl)phenyl)-2-methoxypyridin-3-yl)methyl)carbamate and trifluoroacetic acid (0.07 mL, 0.10 g, 0.86 mmol). 4.6 mg, 31% yield. MS: m/z found 761.1 [M+H]⁺, retention time = 1.67 min (Method D). ¹H NMR (400 MHz, Methanol-*d*₄) δ 8.80 (s, 1H), 8.72 (d, 1H), 7.90 (d, 1H), 7.73 (d, 1H), 7.59 (t, 1H), 7.54 - 7.50 (m, 1H), 7.49 - 7.42 (m, 2H), 7.35 (d, 1H), 4.66 (d, 1H), 4.41 - 4.34 (m, 2H), 4.33 (s, 2H), 4.28 (s, 2H), 4.08 (d, 4H), 3.96 (t, 1H), 3.43 (s, 1H), 3.21 (t, 1H), 2.78 - 2.62 (m, 1H), 2.38 (t, 2H), 2.30 - 2.16 (m, 4H), 2.13 (d, 4H), 1.63 (d, 1H), 1.57 - 1.49 (m, 4H), 1.38 (d, 3H).

### Example 496: 1-(4-(((6-(4-(3-(5-(((1-Acetylpiperidin-4-yl)amino)methyl)-6-methoxypyridin-2-yl)-2-chlorophenyl)-3-chloropyridin-2-yl)-8-ethoxy-[1,2,4]triazolo[1,5-a]pyridin-2-yl)methyl)amino)piperidin-1-yl)ethan-1-one (585)

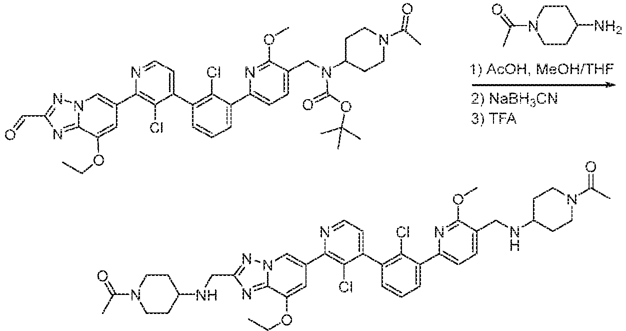

tert-Butyl (1-acetylpiperidin-4-yl)((6-(3-(2-(2-(((1-acetylpiperidin-4-yl)amino)methyl)-8-ethoxy-[1,2,4]triazolo[1,5-a]pyridin-6-yl)-3-chloropyridin-4-yl)-2-chlorophenyl)-2-methoxypyridin-3-yl)methyl)carbamate was prepared using Reductive Amination Procedure B from tert-butyl (1-acetylpiperidin-4-yl)((6-(2-chloro-3-(3-chloro-2-(8-ethoxy-2-formyl-[1,2,4]triazolo[1,5-a]pyridin-6-yl)pyridin-4-yl)phenyl)-2-methoxypyridin-3-yl)methyl)carbamate (Example 494, step (d)) (20 mg, 0.03 mmol), acetic acid (3 µL, 0.05 mmol), 1-(4-amino-1-piperidyl)ethanone (7 mg, 0.05 mmol) and sodium cyanoborohydride (3 mg, 0.05 mmol). 1-(4-(((6-(4-(3-(5-(((1-Acetylpiperidin-4-yl)amino)methyl)-6-methoxypyridin-2-yl)-2-chlorophenyl)-3-chloropyridin-2-yl)-8-ethoxy-[1,2,4]triazolo[1,5-a]pyridin-2-yl)methyl)amino)piperidin-1-yl)ethan-1-one was prepared using the General Boc Deprotection Procedure from tert-butyl (1-acetylpiperidin-4-yl)((6-(3-(2-(2-(((1-acetylpiperidin-4-yl)amino)methyl)-8-ethoxy-[1,2,4]triazolo[1,5-a]pyridin-6-yl)-3-chloropyridin-4-yl)-2-chlorophenyl)-2-methoxypyridin-3-yl)methyl)carbamate and trifluoroacetic acid (76 µL, 1.00 mmol). 9 mg, 44% yield. MS: m/z found 800.3 [M+H]⁺ , retention time = 1.67 min (Method D). ¹H NMR (400 MHz, Methanol-*d*₄) δ 8.82 (s, 1H), 8.72 (d, 1H), 7.91 (d, 1H), 7.73 (d, 1H), 7.59 (t, 1H), 7.53 (d, 1H), 7.48 (s, 1H), 7.46 (s, 1H), 7.37 (d, 1H), 4.68 (d, 2H), 4.62 (s, 2H), 4.38 (q, 2H), 4.33 (s, 2H), 4.09 (s, 5H), 3.67 - 3.45 (m, 2H), 3.22 (t, 2H), 2.72 (d, 2H), 2.27 (s, 5H), 2.13 (s, 7H), 1.72 - 1.62 (m, 2H), 1.53 (d, 3H).

### Example 497: 2-((6-(4-(3-(5-(((1-Acetylpiperidin-4-yl)amino)methyl)-6-methoxypyridin-2-yl)-2-chlorophenyl)-3-chloropyridin-2-yl)-8-ethoxy-[1,2,4]triazolo[1,5-a]pyridin-2-yl)methyl)-2,6-diazaspiro[3.4]octan-7-one (586)

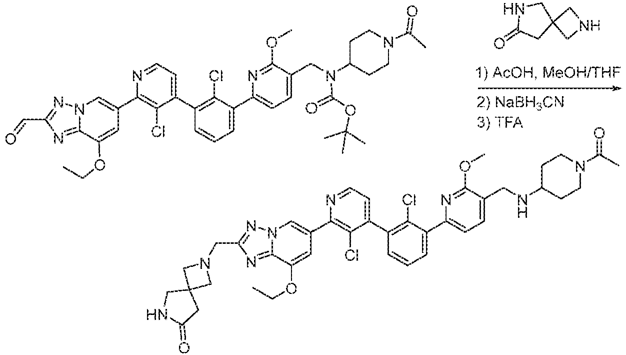

tert-Butyl (1-acetylpiperidin-4-yl)((6-(2-chloro-3-(3-chloro-2-(8-ethoxy-2-((7-oxo-2,6-diazaspiro[3.4]octan-2-yl)methyl)-[1,2,4]triazolo[1,5-a]pyridin-6-yl)pyridin-4-yl)phenyl)-2-methoxypyridin-3-yl)methyl)carbamate was prepared using Reductive Amination Procedure B from tert-butyl (1-acetylpiperidin-4-yl)((6-(2-chloro-3-(3-chloro-2-(8-ethoxy-2-formyl-[1,2,4]triazolo[1,5-a]pyridin-6-yl)pyridin-4-yl)phenyl)-2-methoxypyridin-3-yl)methyl)carbamate (Example 494, step (d)) (20 mg, 0.03 mmol), acetic acid (3 µL, 0.05 mmol) and 2,6-diazaspiro[3.4]octan-7-one (6 mg, 0.05 mmol) and sodium cyanoborohydride (3 mg, 0.05 mmol). 2-((6-(4-(3-(5-(((1-Acetylpiperidin-4-yl)amino)methyl)-6-methoxypyridin-2-yl)-2-chlorophenyl)-3-chloropyridin-2-yl)-8-ethoxy-[1,2,4]triazolo[1,5-a]pyridin-2-yl)methyl)-2,6-diazaspiro[3.4]octan-7-one was prepared using the General Boc Deprotection Procedure from tert-butyl (1-acetylpiperidin-4-yl)((6-(2-chloro-3-(3-chloro-2-(8-ethoxy-2-((7-oxo-2,6-diazaspiro[3.4]octan-2-yl)methyl)-[1,2,4]triazolo[1,5-a]pyridin-6-yl)pyridin-4-yl)phenyl)-2-methoxypyridin-3-yl)methyl)carbamate and trifluoroacetic acid (56 µL, 0.73 mmol). 2 mg, 10% yield. MS: m/z found 784.2 [M+H]⁺ , retention time = 1.63 min (Method D). ¹H NMR (400 MHz, Methanol-*d*₄) δ 8.77 (s, 1H), 8.72 (d, 1H), 7.90 (d, 1H), 7.73 (d, 1H), 7.59 (t, 1H), 7.51 (d, 1H), 7.47 (d, 1H), 7.41 (s, 1H), 7.37 (d, 1H), 4.36 (t, 2H), 4.31 (s, 3H), 4.25 (s, 2H), 4.09 (s, 5H), 3.85 (s, 5H), 3.63 (s, 2H), 3.21 (s, 1H), 2.65 (d, 3H), 2.25 (s, 2H), 2.13 (s, 4H), 1.53 (d, 3H).

### Example 498: (S)-5-((((6-(2-Chloro-3-(3-chloro-2-(8-methoxy-2-(((((S)-5-oxopyrrolidin-2-yl)methyl)amino)methyl)imidazo[1,2-a]pyridin-6-yl)pyridin-4-yl)phenyl)-2-methoxypyridin-3-yl)methyl)amino)methyl)pyrrolidin-2-one (310)

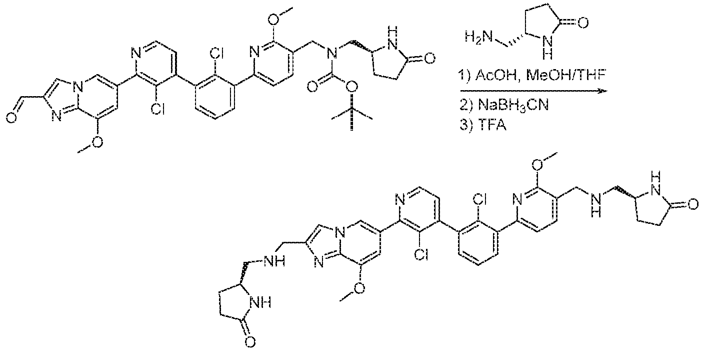

tert-Butyl ((6-(2-chloro-3-(3-chloro-2-(8-methoxy-2-(((((S)-5-oxopyrrolidin-2-yl)methyl)amino)methyl)imidazo[1,2-a]pyridin-6-yl)pyridin-4-yl)phenyl)-2-methoxypyridin-3-yl)methyl)(((S)-5-oxopyrrolidin-2-yl)methyl)carbamate was prepared using Reductive Amination Procedure B from tert-butyl (S)-((6-(2-chloro-3-(3-chloro-2-(2-formyl-8-methoxyimidazo[1,2-a]pyridin-6-yl)pyridin-4-yl)phenyl)-2-methoxypyridin-3-yl)methyl)((5-oxopyrrolidin-2-yl)methyl)carbamate (Example 499, step (c)) (20 mg, 0.03 mmol), acetic acid (3 µL, 0.05 mmol), (5S)-5-(aminomethyl)pyrrolidin-2-one (6 mg, 0.05 mmol) and sodium cyanoborohydride (3 mg, 0.05 mmol). (S)-5-((((6-(2-Chloro-3-(3-chloro-2-(8-methoxy-2-(((((S)-5-oxopyrrolidin-2-yl)methyl)amino)methyl)imidazo [ 1,2-a]pyridin-6-yl)pyridin-4-yl)phenyl)-2-methoxypyridin-3-yl)methyl)amino)methyl)pyrrolidin-2-one was prepared using General Boc Deprotection Procedure from tert-butyl ((6-(2-chloro-3-(3-chloro-2-(8-methoxy-2-(((((S)-5-oxopyrrolidin-2-yl)methyl)amino)methyl)imidazo[1,2-a]pyridin-6-yl)pyridin-4-yl)phenyl)-2-methoxypyridin-3-yl)methyl)(((S)-5-oxopyrrolidin-2-yl)methyl)carbamate and trifluoroacetic acid (39 µL, 0.51 mmol). 10 mg, 50% yield. MS: m/z found 729.1 [M+H]⁺, retention time = 0.62 min (Method B). ¹H NMR (400 MHz, Methanol-*d*₄) δ 8.72 (d, 1H), 8.22 (s, 1H), 8.06 (t, 1H), 7.91 (d, 1H), 7.77 - 7.71 (m, 1H), 7.59 (t, 1H), 7.52 (d, 1H), 7.46 (d, 1H), 7.36 (dd, 1H), 7.33 (s, 1H), 4.50 (s, 2H), 4.34 (s, 2H), 4.12 (t, 3H), 4.10 - 4.02 (m, 6H), 3.27 - 3.24 (m, 2H), 2.39 (q, 7H), 1.91 (s, 2H).

### Example 499: (S)-5-((((6-(2-chloro-3-(3-chloro-2-(2-((((1r,3r)-3-hydroxy-3-methylcyclobutyl)amino)methyl)-8-methoxyimidazo[1,2-a]pyridin-6-yl)pyridin-4-yl)phenyl)-2-methoxypyridin-3-yl)methyl)amino)methyl)pyrrolidin-2-one (312)

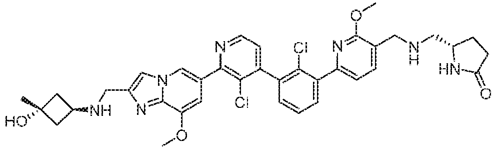

### (a) [2-(hydroxymethyl)-8-methoxy-imidazo[1,2-a]pyridin-6-yl]boronic acid

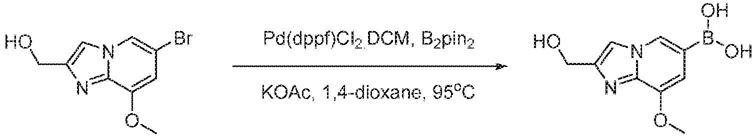

(6-Bromo-8-methoxyimidazo[1,2-a]pyridin-2-yl)methanol (161 mg, 0.63 mmol), bis(pinacolato)diborane (318 mg, 1.25 mmol), potassium acetate (184 mg, 1.88 mmol) and [1,1'- bis(diphenylphosphino)ferrocene]dichloropalladium(II) complex with dichloromethane (51 mg, 0.06 mmol) were suspended in 5 ml 1,4-dioxane. Nitrogen gas was bubbled through the suspension for 2 minutes and then the mixture heated at 95 °C for 3 hours. The reaction was concentrated and the residue was purified by silica gel chromatography (0-50% MeOH in DCM) to afford (2-(hydroxymethyl)-8-methoxyimidazo[1,2-a]pyridin-6-yl)boronic acid (131 mg, 69%). MS: m/z found 223 [M+H]⁺.

### (b) tert-Butyl (S)-((6-(2-chloro-3-(3-chloro-2-(2-(hydroxymethyl)-8-methoxyimidazo[1,2-a]pyridin-6-yl)pyridin-4-yl)phenyl)-2-methoxypyridin-3-yl)methyl)((5-oxopyrrolidin-2-yl)methyl)carbamate

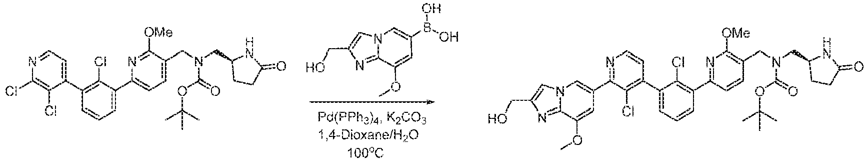

To a mixture of (2-(hydroxymethyl)-8-methoxyimidazo[1,2-a]pyridin-6-yl)boronic acid (130.8 mg, 0.59 mmol) and tert-butyl (S)-((6-(2-chloro-3-(2,3-dichloropyridin-4-yl)phenyl)-2-methoxypyridin-3-yl)methyl)((5-oxopyrrolidin-2-yl)methyl)carbamate (250 mg, 0.39 mmol) in 1,4-dioxane/water (4:1, 3 mL), potassium carbonate (163 mg, 1.18 mmol) was added. Nitrogen gas was bubbled through the mixture for two minutes. Tetrakis(triphenylphosphine)palladium(0) (45 mg, 0.04 mmol) was added, and the reaction was stirred at 100 °C for 30 minutes. The reaction mixture was diluted with water (30 mL), and the aqueous layer was extracted with EtOAc (3 x 30 ml). The combined organic layers were washed with brine (20 mL), dried over anhydrous sodium sulfate, filtered, and concentrated. The residue was purified by normal phase silica gel chromatography (0-5% MeOH in DCM) to afford tert-butyl (S)-((6-(2-chloro-3-(3-chloro-2-(2-(hydroxymethyl)-8-methoxyimidazo[1,2-a]pyridin-6-yl)pyridin-4-yl)phenyl)-2-methoxypyridin-3-yl)methyl)((5-oxopyrrolidin-2-yl)methyl)carbamate (255 mg, 89% yield). MS: m/z found 733.2 [M+H]⁺.

### (c) tert-Butyl (S)-((6-(2-chloro-3-(3-chloro-2-(2-formyl-8-methoxyimidazo[1,2-a]pyridin-6-yl)pyridin-4-yl)phenyl)-2-methoxypyridin-3-yl)methyl)((5-oxopyrrolidin-2-yl)methyl)carbamate

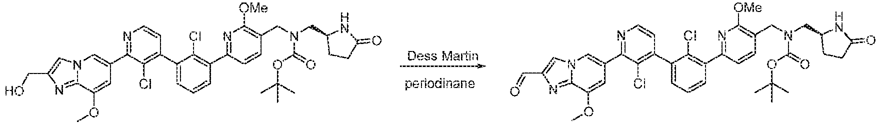

To a solution of tert-butyl (S)-((6-(2-chloro-3-(3-chloro-2-(2-(hydroxymethyl)-8-methoxyimidazo[1,2-a]pyridin-6-yl)pyridin-4-yl)phenyl)-2-methoxypyridin-3-yl)methyl)((5-oxopyrrolidin-2-yl)methyl)carbamate (255 mg, 0.35 mmol) in dichloromethane (3 mL), Dess Martin periodinane (294 mg, 0.70 mmol) was added. The reaction was stirred at room temperature for 2 h. The reaction mixture was concentrated and the residue was purified by normal phase silica gel chromatography (0-5% MeOH in DCM) to afford tert-butyl (S)-((6-(2-chloro-3-(3-chloro-2-(2-formyl-8-methoxyimidazo[1,2-a]pyridin-6-yl)pyridin-4-yl)phenyl)-2-methoxypyridin-3-yl)methyl)((5-oxopyrrolidin-2-yl)methyl)carbamate (233 mg, 36% yield). MS: m/z found 731 [M+H]⁺.

### (d) (S)-5-((((6-(2-Chloro-3-(3-chloro-2-(2-((((1r,3r)-3-hydroxy-3-methylcyclobutyl)amino)methyl)-8-methoxyimidazo[1,2-a]pyridin-6-yl)pyridin-4-yl)phenyl)-2-methoxypyridin-3-yl)methyl)amino)methyl)pyrrolidin-2-one

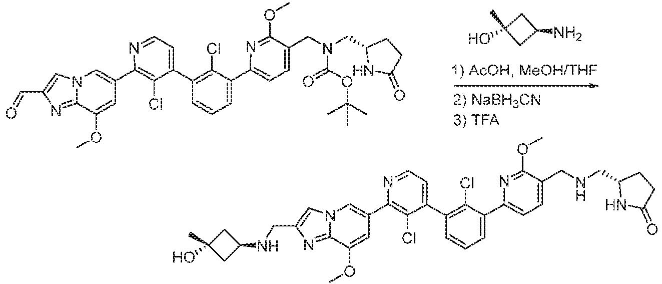

tert-Butyl ((6-(2-chloro-3-(3-chloro-2-(2-((((1r,3r)-3-hydroxy-3-methylcyclobutyl)amino)methyl)-8-methoxyimidazo[1,2-a]pyridin-6-yl)pyridin-4-yl)phenyl)-2-methoxypyridin-3-yl)methyl)(((S)-5-oxopyrrolidin-2-yl)methyl)carbamate was prepared using Reductive Amination Procedure B from tert-butyl (S)-((6-(2-chloro-3-(3-chloro-2-(2-formyl-8-methoxyimidazo[1,2-a]pyridin-6-yl)pyridin-4-yl)phenyl)-2-methoxypyridin-3-yl)methyl)((5-oxopyrrolidin-2-yl)methyl)carbamate (20 mg, 0.03 mmol), acetic acid (3 µL, 0.05 mmol), (1r,3r)-3-amino-1-methylcyclobutan-1-ol hydrochloride (7 mg, 0.05 mmol) and sodium cyanoborohydride (3 mg, 0.05 mmol). (S)-5-((((6-(2-Chloro-3-(3-chloro-2-(2-((((1r,3r)-3-hydroxy-3-methylcyclobutyl)amino)methyl)-8-methoxyimidazo[1,2-a]pyridin-6-yl)pyridin-4-yl)phenyl)-2-methoxypyridin-3-yl)methyl)amino)methyl)pyrrolidin-2-one was prepared using General Boc Deprotection Procedure from tert-Butyl ((6-(2-chloro-3-(3-chloro-2-(2-((((1r,3r)-3-hydroxy-3-methylcyclobutyl)amino)methyl)-8-methoxyimidazo[1,2-a]pyridin-6-yl)pyridin-4-yl)phenyl)-2-methoxypyridin-3-yl)methyl)(((S)-5-oxopyrrolidin-2-yl)methyl)carbamate and trifluoroacetic acid (0.07 mL, 0.86 mmol). 5 mg, 26% yield. MS: m/z found 716.0 [M+H]⁺, retention time = 1.53 min (Method D). ¹H NMR (400 MHz, Methanol-*d*₄) δ 8.71 - 8.65 (m, 1H), 8.52 (s, 1H), 7.95 (d, 1H), 7.73 (dd, 2H), 7.55 (t, 1H), 7.49 (d, 1H), 7.41 (d, 1H), 7.28 - 7.21 (m, 1H), 7.06 (s, 1H), 4.11 - 4.05 (m, 5H), 4.01 (t, 3H), 3.85 (d, 3H), 3.83 - 3.73 (m, 1H), 2.80 - 2.62 (m, 2H), 2.33 (d, 4H), 2.29 - 2.21 (m, 1H), 2.06 (t, 2H), 1.81 (d, 1H), 1.36 (d, 3H).

### Example 500: (S)-2-((6-(3-Chloro-4-(2-chloro-3-(6-methoxy-5-((((5-oxopyrrolidin-2-yl)methyl)amino)methyl)pyridin-2-yl)phenyl)pyridin-2-yl)-8-methoxyimidazo[1,2-a]pyridin-2-yl)methyl)-2,6-diazaspiro[3.4]octan-7-one (314)

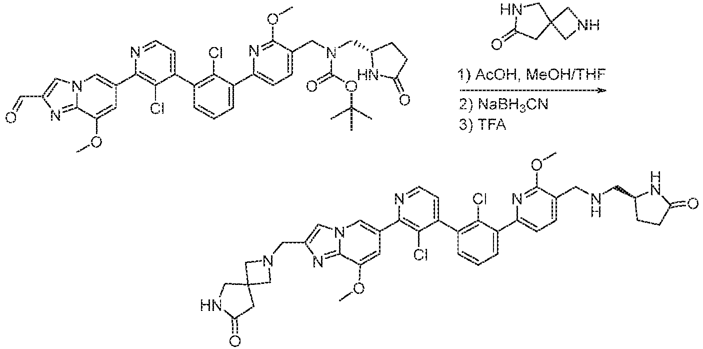

tert-Butyl (S)-((6-(2-chloro-3-(3-chloro-2-(8-methoxy-2-((7-oxo-2,6-diazaspiro[3.4]octan-2-yl)methyl)imidazo[1,2-a]pyridin-6-yl)pyridin-4-yl)phenyl)-2-methoxypyridin-3-yl)methyl)((5-oxopyrrolidin-2-yl)methyl)carbamate was prepared using Reductive Amination Procedure B from tert-butyl (S)-((6-(2-chloro-3-(3-chloro-2-(2-formyl-8-methoxyimidazo[1,2-a]pyridin-6-yl)pyridin-4-yl)phenyl)-2-methoxypyridin-3-yl)methyl)((5-oxopyrrolidin-2-yl)methyl)carbamate (Example 499, step (c)) (20 mg, 0.03 mmol), acetic acid (3 µL, 0.05 mmol), 2,6-diazaspiro[3.4]octan-7-one;hydrochloride (9 mg, 0.05 mmol) and sodium cyanoborohydride (3 mg, 0.05 mmol). (S)-2-((6-(3-Chloro-4-(2-chloro-3-(6-methoxy-5-((((5-oxopyrrolidin-2-yl)methyl)amino)methyl)pyridin-2-yl)phenyl)pyridin-2-yl)-8-methoxyimidazo[1,2-a]pyridin-2-yl)methyl)-2,6-diazaspiro[3.4]octan-7-one was prepared using General Boc Deprotection Procedure from tert-butyl (S)-((6-(2-chloro-3-(3-chloro-2-(8-methoxy-2-((7-oxo-2,6-diazaspiro[3.4]octan-2-yl)methyl)imidazo[1,2-a]pyridin-6-yl)pyridin-4-yl)phenyl)-2-methoxypyridin-3-yl)methyl)((5-oxopyrrolidin-2-yl)methyl)carbamate and trifluoroacetic acid (50 µL, 0.65 mmol). 4.1 mg, 20% yield. MS: m/z found 741.2 [M+H]⁺, retention time = 1.45 min (Method D). ¹H NMR (400 MHz, Methanol-*d*₄) δ 8.68 (dd, 1H), 8.49 (s, 1H), 7.98 (s, 1H), 7.83 (d, 1H), 7.72 (d, 1H), 7.57 (t, 1H), 7.51 - 7.46 (m, 1H), 7.43 (d, 1H), 7.31 (d, 1H), 7.06 (s, 1H), 4.23 (s, 2H), 4.10 (s, 2H), 4.07 - 4.02 (m, 6H), 3.94 (s, 4H), 3.62 (s, 2H), 2.99 (t, 2H), 2.65 (d, 2H), 2.34 (d, 3H), 1.86 (s, 1H).

### Example 501: (S)-5-((((6-(2-Chloro-3-(3-chloro-2-(2-((3-hydroxy-3-methylazetidin-1-yl)methyl)-8-methoxyimidazo[1,2-a]pyridin-6-yl)pyridin-4-yl)phenyl)-2-methoxypyridin-3-yl)methyl)amino)methyl)pyrrolidin-2-one (317)

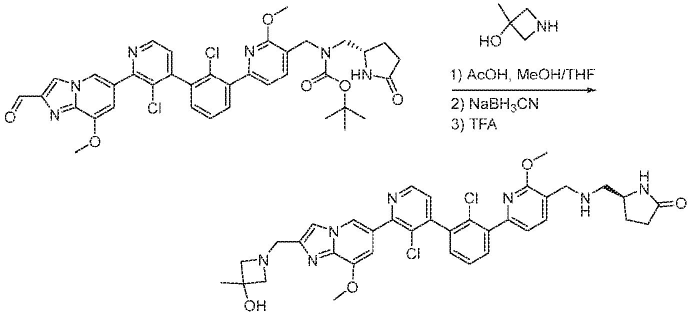

tert-Butyl (S)-((6-(2-chloro-3-(3-chloro-2-(2-((3-hydroxy-3-methylazetidin-1-yl)methyl)-8-methoxyimidazo[1,2-a]pyridin-6-yl)pyridin-4-yl)phenyl)-2-methoxypyridin-3-yl)methyl)((5-oxopyrrolidin-2-yl)methyl)carbamate was prepared using Reductive Amination Procedure B from tert-butyl (S)-((6-(2-chloro-3-(3-chloro-2-(2-formyl-8-methoxyimidazo[1,2-a]pyridin-6-yl)pyridin-4-yl)phenyl)-2-methoxypyridin-3-yl)methyl)((5-oxopyrrolidin-2-yl)methyl)carbamate (Example 499, step (c)) (20 mg, 0.03 mmol), acetic acid (3 µL, 0.05 mmol), 3-methylazetidin-3-ol hydrochloride (7 mg, 0.05 mmol) and sodium cyanoborohydride (11 mg, 0.05 mmol). (S)-5-((((6-(2-Chloro-3-(3-chloro-2-(2-((3-hydroxy-3-methylazetidin-1-yl)methyl)-8-methoxyimidazo[1,2-a]pyridin-6-yl)pyridin-4-yl)phenyl)-2-methoxypyridin-3-yl)methyl)amino)methyl)pyrrolidin-2-one was prepared using General Boc Deprotection Procedure from tert-butyl (S)-((6-(2-chloro-3-(3-chloro-2-(2-((3-hydroxy-3-methylazetidin-1-yl)methyl)-8-methoxyimidazo[1,2-a]pyridin-6-yl)pyridin-4-yl)phenyl)-2-methoxypyridin-3-yl)methyl)((5-oxopyrrolidin-2-yl)methyl)carbamate and trifluoroacetic acid (57 µL, 0.75 mmol). 7.6 mg, 72% yield. MS: m/z found 702.1 [M+H]⁺, retention time = 1.55 min (Method D). ¹H NMR (400 MHz, Methanol-*d*₄) δ 8.73 - 8.68 (m, 1H), 8.55 (d, 1H), 8.14 (s, 1H), 7.91 (d, 1H), 7.73 (d, 1H), 7.59 (t, 1H), 7.51 (d, 1H), 7.48 - 7.43 (m, 1H), 7.38 (d, 1H), 7.17 (d, 1H), 4.56 (s, 2H), 4.35 (s, 2H), 4.20 (d, 2H), 4.11 - 4.07 (m, 7H), 3.26 (t, 2H), 2.45 - 2.34 (m, 3H), 1.92 (d, 1H), 1.52 (s, 3H).

### Example 502: (S)-5-((((6-(3-(2-(2-(((1-Acetylpiperidin-4-yl)amino)methyl)-8-methoxyimidazo[1,2-a]pyridin-6-yl)-3-chloropyridin-4-yl)-2-chlorophenyl)-2-methoxypyridin-3-yl)methyl)amino)methyl)pyrrolidin-2-one (318)

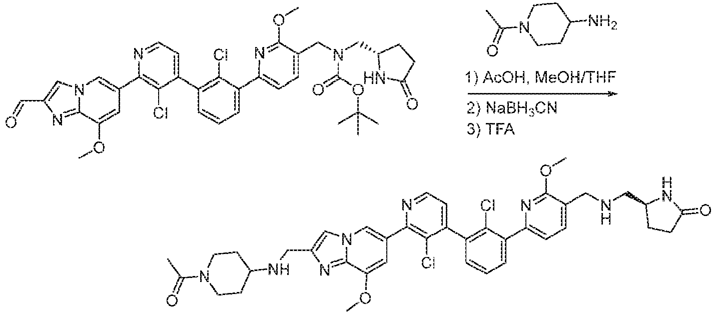

tert-Butyl (S)-((6-(3-(2-(2-(((1-acetylpiperidin-4-yl)amino)methyl)-8-methoxyimidazo[1,2-a]pyridin-6-yl)-3-chloropyridin-4-yl)-2-chlorophenyl)-2-methoxypyridin-3-yl)methyl)((5-oxopyrrolidin-2-yl)methyl)carbamate was prepared using Reductive Amination Procedure B from tert-butyl (S)-((6-(2-chloro-3-(3-chloro-2-(2-formyl-8-methoxyimidazo[1,2-a]pyridin-6-yl)pyridin-4-yl)phenyl)-2-methoxypyridin-3-yl)methyl)((5-oxopyrrolidin-2-yl)methyl)carbamate (Example 499, step (c)) (20 mg, 0.03 mmol), acetic acid (3 µL, 0.05 mmol), 1-(4-amino-1-piperidyl)ethanone (8 mg, 0.05 mmol) and sodium cyanoborohydride (3 mg, 0.05 mmol). (S)-5-((((6-(3-(2-(2-(((1-Acetylpiperidin-4-yl)amino)methyl)-8-methoxyimidazo[1,2-a]pyridin-6-yl)-3-chloropyridin-4-yl)-2-chlorophenyl)-2-methoxypyridin-3-yl)methyl)amino)methyl)pyrrolidin-2-one was prepared using general Boc Deprotection procedure from tert-butyl (S)-((6-(3-(2-(2-(((1-acetylpiperidin-4-yl)amino)methyl)-8-methoxyimidazo[1,2-a]pyridin-6-yl)-3-chloropyridin-4-yl)-2-chlorophenyl)-2-methoxypyridin-3-yl)methyl)((5-oxopyrrolidin-2-yl)methyl)carbamate and trifluoroacetic acid (67 µL, 0.87 mmol). 7.2 mg, 35% yield. MS: m/z found 757.1 [M+H]⁺, retention time = 1.53 min (Method D). ¹H NMR (400 MHz, Methanol-*d*₄) δ 8.71 - 8.65 (m, 1H), 8.49 (d, 2H), 7.98 (s, 1H), 7.77 (d, 1H), 7.73 - 7.69 (m, 1H), 7.56 (t, 1H), 7.51 - 7.46 (m, 1H), 7.44 - 7.39 (m, 1H), 7.27 (dd, 1H), 7.06 (d, 1H), 4.57 (d, 1H), 4.23 (s, 2H), 4.07 (t, 3H), 4.02 (t, 3H), 3.97 (d, 1H), 3.92 - 3.83 (m, 3H), 3.25 - 3.11 (m, 2H), 2.80 (m, 2H), 2.68 (t, 1H), 2.39 - 2.24 (m, 3H), 2.16 (d, 2H), 2.11 (t, 3H), 1.60 - 1.32 (m, 1H).

### Example 503: (S)-5-((((6-(3-(2-(2-((6-Acetyl-2,6-diazaspiro[3.3]heptan-2-yl)methyl)-8-methoxyimidazo[1,2-a]pyridin-6-yl)-3-chloropyridin-4-yl)-2-chlorophenyl)-2-methoxypyridin-3-yl)methyl)amino)methyl)pyrrolidin-2-one (319)

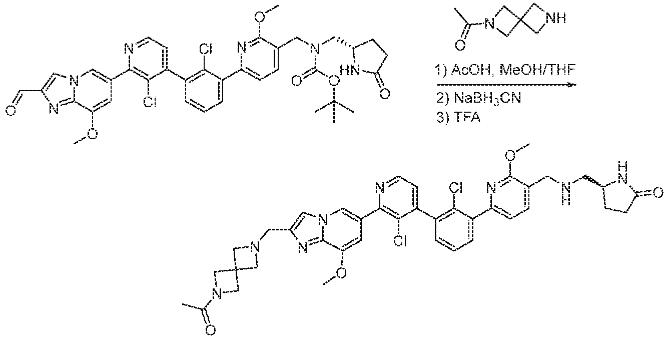

tert-Butyl (S)-((6-(3-(2-(2-((6-acetyl-2,6-diazaspiro[3.3]heptan-2-yl)methyl)-8-methoxyimidazo[1,2-a]pyridin-6-yl)-3-chloropyridin-4-yl)-2-chlorophenyl)-2-methoxypyridin-3-yl)methyl)((5-oxopyrrolidin-2-yl)methyl)carbamate was prepared using Reductive Amination Procedure B from tert-butyl (S)-((6-(2-chloro-3-(3-chloro-2-(2-formyl-8-methoxyimidazo[1,2-a]pyridin-6-yl)pyridin-4-yl)phenyl)-2-methoxypyridin-3-yl)methyl)((5-oxopyrrolidin-2-yl)methyl)carbamate (Example 499, step (c)) (20 mg, 0.03 mmol), acetic acid (3 µL, 0.05 mmol), 1-(2,6-diazaspiro[3.3]heptan-2-yl)ethan-1-one hydrochloride (10 mg, 0.05 mmol) and sodium cyanoborohydride (3 mg, 0.05 mmol). (S)-5-((((6-(3-(2-(2-((6-Acetyl-2,6-diazaspiro[3.3]heptan-2-yl)methyl)-8-methoxyimidazo[1,2-a]pyridin-6-yl)-3-chloropyridin-4-yl)-2-chlorophenyl)-2-methoxypyridin-3-yl)methyl)amino)methyl)pyrrolidin-2-one was prepared using General Boc Deprotection Procedure from tert-butyl (S)-((6-(3-(2-(2-((6-acetyl-2,6-diazaspiro[3.3]heptan-2-yl)methyl)-8-methoxyimidazo[1,2-a]pyridin-6-yl)-3-chloropyridin-4-yl)-2-chlorophenyl)-2-methoxypyridin-3-yl)methyl)((5-oxopyrrolidin-2-yl)methyl)carbamate and trifluoroacetic acid (49 µL, 0.64 mmol). 2.7 mg, 13% yield. MS: m/z found 755 [M+H]⁺ , retention time = 1.54 min (Method D). ¹H NMR (400 MHz, Methanol-*d*₄) δ 8.68 (d, 1H), 8.48 (d, 1H), 7.92 (s, 1H), 7.79 (d, 1H), 7.72 (d, 1H), 7.56 (t, 1H), 7.48 (d, 1H), 7.43 (d, 1H), 7.28 (d, 1H), 7.05 (s, 1H), 4.32 (s, 2H), 4.10 - 4.01 (m, 11H), 3.98 (d, 2H), 3.87 (s, 4H), 2.86 (t, 2H), 2.42 - 2.24 (m, 3H), 1.83 (s, 4H).

### Example 504: (S)-2-((6-(3-Chloro-4-(2-chloro-3-(6-methoxy-5-((((5-oxopyrrolidin-2-yl)methyl)amino)methyl)pyridin-2-yl)phenyl)pyridin-2-yl)-8-methoxyimidazo[1,2-a]pyridin-2-yl)methyl)-2,5-diazaspiro[3.4]octan-6-one (320)

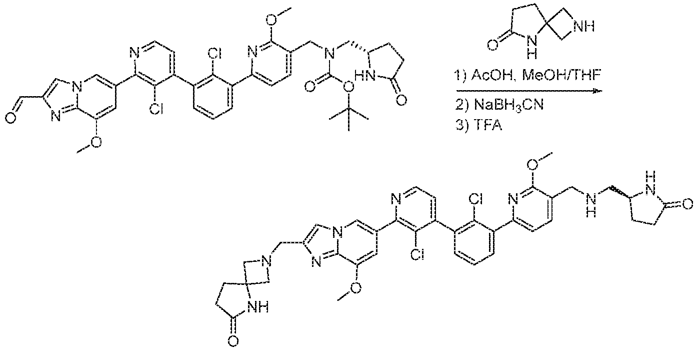

tert-Butyl (S)-((6-(2-chloro-3-(3-chloro-2-(8-methoxy-2-((6-oxo-2,5-diazaspiro[3.4]octan-2-yl)methyl)imidazo[1,2-a]pyridin-6-yl)pyridin-4-yl)phenyl)-2-methoxypyridin-3-yl)methyl)((5-oxopyrrolidin-2-yl)methyl)carbamate was prepared using Reductive Amination Procedure B from tert-butyl (S)-((6-(2-chloro-3-(3-chloro-2-(2-formyl-8-methoxyimidazo[1,2-a]pyridin-6-yl)pyridin-4-yl)phenyl)-2-methoxypyridin-3-yl)methyl)((5-oxopyrrolidin-2-yl)methyl)carbamate (Example 499, step (c)) (20 mg, 0.03 mmol), acetic acid (3 µL, 0.05 mmol), 2,5-diazaspiro[3.4]octan-6-one hydrochloride (9 mg, 0.05 mmol) and sodium cyanoborohydride (3 mg, 0.05 mmol). (S)-2-((6-(3-Chloro-4-(2-chloro-3-(6-methoxy-5-((((5-oxopyrrolidin-2-yl)methyl)amino)methyl)pyridin-2-yl)phenyl)pyridin-2-yl)-8-methoxyimidazo[1,2-a]pyridin-2-yl)methyl)-2,5-diazaspiro[3.4]octan-6-one was prepared using the general Boc Deprotection Procedure from tert-butyl (S)-((6-(2-chloro-3-(3-chloro-2-(8-methoxy-2-((6-oxo-2,5-diazaspiro[3.4]octan-2-yl)methyl)imidazo[1,2-a]pyridin-6-yl)pyridin-4-yl)phenyl)-2-methoxypyridin-3-yl)methyl)((5-oxopyrrolidin-2-yl)methyl)carbamate and trifluoroacetic acid (59 µL, 0.77 mmol). 6.3 mg, 31% yield. MS: m/z found 741.1 [M+H]⁺, retention time = 1.53 min (Method D). ¹H NMR (400 MHz, Methanol-*d*₄) δ 8.67 (dd, 1H), 8.44 (d, 1H), 7.86 (s, 1H), 7.79 (d, 1H), 7.71 (d, 1H), 7.59 - 7.52 (m, 1H), 7.47 (dd, 1H), 7.42 (d, 1H), 7.28 (dd, 1H), 7.02 (s, 1H), 4.04 (m, 6H), 3.99 (s, 2H), 3.95 (s, 2H), 3.90 (d, 1H), 3.69 (d, 2H), 3.55 (d, 2H), 2.93 - 2.81 (m, 2H), 2.41 (d, 2H), 2.38 - 2.25 (m, 4H), 1.85 (t, 1H).

### Example 505: (S)-5-((((6-(3-(2-(2-(((2-Oxaspiro[3.3]heptan-6-yl)amino)methyl)-8-methoxyimidazo[1,2-a]pyridin-6-yl)-3-chloropyridin-4-yl)-2-chlorophenyl)-2-methoxypyridin-3-yl)methyl)amino)methyl)pyrrolidin-2-one (321)

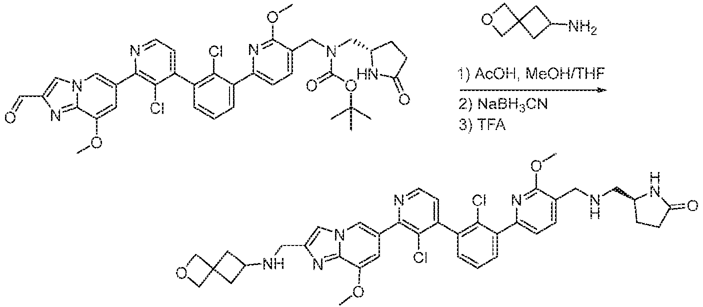

tert-Butyl (S)-((6-(3-(2-(2-(((2-oxaspiro[3.3]heptan-6-yl)amino)methyl)-8-methoxyimidazo[1,2-a]pyridin-6-yl)-3-chloropyridin-4-yl)-2-chlorophenyl)-2-methoxypyridin-3-yl)methyl)((5-oxopyrrolidin-2-yl)methyl)carbamate was prepared using Reductive Amination Procedure B from tert-butyl (S)-((6-(2-chloro-3-(3-chloro-2-(2-formyl-8-methoxyimidazo[1,2-a]pyridin-6-yl)pyridin-4-yl)phenyl)-2-methoxypyridin-3-yl)methyl)((5-oxopyrrolidin-2-yl)methyl)carbamate (Example 499, step (c)) (20 mg, 0.03 mmol), acetic acid (3 µL, 0.05 mmol), 2-oxaspiro[3.3]heptan-6-amine hydrochloride (8 mg, 0.05 mmol) and sodium cyanoborohydride (3 mg, 0.05 mmol). (S)-5-((((6-(3-(2-(2-(((2-Oxaspiro[3.3]heptan-6-yl)amino)methyl)-8-methoxyimidazo[1,2-a]pyridin-6-yl)-3-chloropyridin-4-yl)-2-chlorophenyl)-2-methoxypyridin-3-yl)methyl)amino)methyl)pyrrolidin-2-one was prepared using the General Boc Deprotection Procedure from tert-butyl (S)-((6-(3-(2-(2-(((2-oxaspiro[3.3]heptan-6-yl)amino)methyl)-8-methoxyimidazo[1,2-a]pyridin-6-yl)-3-chloropyridin-4-yl)-2-chlorophenyl)-2-methoxypyridin-3-yl)methyl)((5-oxopyrrolidin-2-yl)methyl)carbamate and trifluoroacetic acid (64 µL, 0.84 mmol). 4.7 mg, 24 % yield. MS: m/z found 730.1 [M+H]⁺, retention time = 1.64 min (Method D). ¹H NMR (400 MHz, Methanol-*d*₄) δ 8.68 (d, 1H), 7.99 (s, 1H), 7.77 (d, 1H), 7.72 (d, 1H), 7.56 (t, 1H), 7.49 (d, 1H), 7.42 (d, 1H), 7.26 (d, 1H), 7.07 (s, 1H), 4.14 (s, 2H), 4.07 (s, 3H), 4.02 (t, 3H), 3.93 - 3.82 (m, 3H), 3.71 (d, 1H), 3.68 (s, 2H), 3.62 (s, 2H), 2.78 (t, 2H), 2.31 (q, 5H), 2.01 - 1.91 (m, 2H), 1.83 (s, 1H).

### Example 506: (S)-1-((6-(3-Chloro-4-(2-chloro-3-(6-methoxy-5-((((5-oxopyrrolidin-2-yl)methyl)amino)methyl)pyridin-2-yl)phenyl)pyridin-2-yl)-8-methoxyimidazo[1,2-a]pyridin-2-yl)methyl)azetidine-3-carboxylic acid (350)

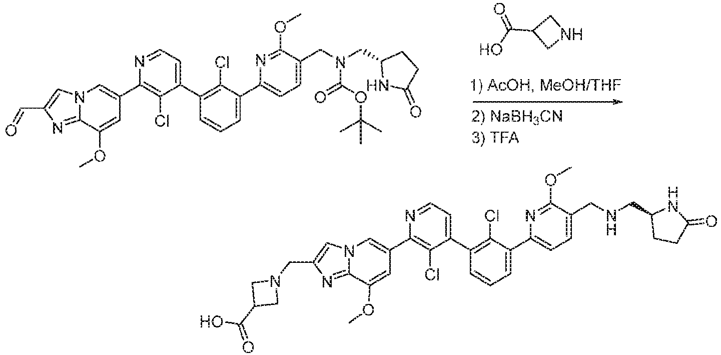

(S)-1-((6-(4-(3-(5-(((tert-Butoxycarbonyl)((5-oxopyrrolidin-2-yl)methyl)amino)methyl)-6-methoxypyridin-2-yl)-2-chlorophenyl)-3-chloropyridin-2-yl)-8-methoxyimidazo[1,2-a]pyridin-2-yl)methyl)azetidine-3-carboxylic acid was prepared using Reductive Amination Procedure B from tert-butyl (S)-((6-(2-chloro-3-(3-chloro-2-(2-formyl-8-methoxyimidazo[1,2-a]pyridin-6-yl)pyridin-4-yl)phenyl)-2-methoxypyridin-3-yl)methyl)((5-oxopyrrolidin-2-yl)methyl)carbamate (Example 499, step (c)) (20 mg, 0.03 mmol), acetic acid (3 µL, 0.05 mmol), azetidine-3-carboxylic acid (6 mg, 0.05 mmol) and sodium cyanoborohydride (3 mg, 0.05 mmol). (S)-1-((6-(3-Chloro-4-(2-chloro-3-(6-methoxy-5-((((5-oxopyrrolidin-2-yl)methyl)amino)methyl)pyridin-2-yl)phenyl)pyridin-2-yl)-8-methoxyimidazo[1,2-a]pyridin-2-yl)methyl)azetidine-3-carboxylic acid was prepared using General Boc Deprotection Procedure from S)-1-((6-(4-(3-(5-(((tert-butoxycarbonyl)((5-oxopyrrolidin-2-yl)methyl)amino)methyl)-6-methoxypyridin-2-yl)-2-chlorophenyl)-3-chloropyridin-2-yl)-8-methoxyimidazo[1,2-a]pyridin-2-yl)methyl)azetidine-3-carboxylic acid and trifluoroacetic acid (70 µL, 0.92 mmol). 8.1 mg, 41% yield. MS: m/z found 716 [M+H]⁺, retention time = 1.50 min (Method D). ¹H NMR (400 MHz, Methanol-*d*₄) δ 8.68 (m, 1H), 8.50 (q, 1H), 8.07 (s, 1H), 7.80 (d, 1H), 7.73 - 7.66 (m, 1H), 7.55 (dd, 1H), 7.47 (m, 1H), 7.42 (m, 1H), 7.28 (d, 1H), 7.07 (d, 1H), 4.45 (s, 2H), 4.29 - 4.17 (m, 4H), 4.05 (t, 3H), 4.03 (d, 3H), 4.02 (s, 1H), 3.93 (s, 1H), 3.39 (m, 1H), 2.98 - 2.84 (m, 2H), 2.41 - 2.26 (m, 3H), 1.84 (m, 1H).

### Example 507: (S)-1-(2-(((6-(3-Chloro-4-(2-chloro-3-(6-methoxy-5-((((5-oxopyrrolidin-2-yl)methyl)amino)methyl)pyridin-2-yl)phenyl)pyridin-2-yl)-8-methoxyimidazo[1,2-a]pyridin-2-yl)methyl)amino)ethyl)cyclopropane-1-carboxylic acid (351)

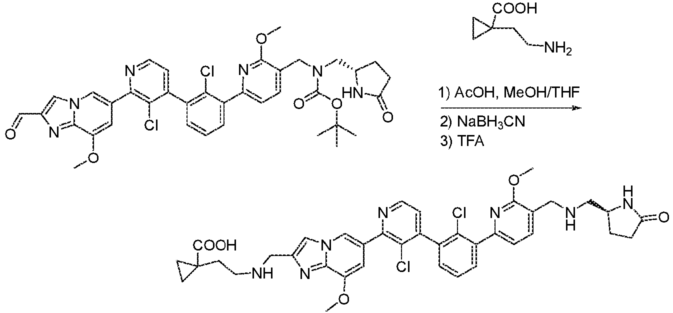

(S)-1-(2-(((6-(4-(3-(5-(((tert-Butoxycarbonyl)((5-oxopyrrolidin-2-yl)methyl)amino)methyl)-6-methoxypyridin-2-yl)-2-chlorophenyl)-3-chloropyridin-2-yl)-8-methoxyimidazo[1,2-a]pyridin-2-yl)methyl)amino)ethyl)cyclopropane-1-carboxylic acid was prepared using Reductive Amination Procedure B from tert-butyl (S)-((6-(2-chloro-3-(3-chloro-2-(2-formyl-8-methoxyimidazo[1,2-a]pyridin-6-yl)pyridin-4-yl)phenyl)-2-methoxypyridin-3-yl)methyl)((5-oxopyrrolidin-2-yl)methyl)carbamate (Example 499, step (c)) (20 mg, 0.03 mmol), acetic acid (3 µL, 0.05 mmol), 1-(2-aminoethyl)cyclopropane-1-carboxylic acid (7 mg, 0.05 mmol) and sodium cyanoborohydride (3 mg, 0.05 mmol). (S)-1-(2-(((6-(3-Chloro-4-(2-chloro-3-(6-methoxy-5-((((5-oxopyrrolidin-2-yl)methyl)amino)methyl)pyridin-2-yl)phenyl)pyridin-2-yl)-8-methoxyimidazo[1,2-a]pyridin-2-yl)methyl)amino)ethyl)cyclopropane-1-carboxylic acid was prepared using general Boc Deprotection Procedure from (S)-1-(2-(((6-(4-(3-(5-(((tert-Butoxycarbonyl)((5-oxopyrrolidin-2-yl)methyl)amino)methyl)-6-methoxypyridin-2-yl)-2-chlorophenyl)-3-chloropyridin-2-yl)-8-methoxyimidazo[1,2-a]pyridin-2-yl)methyl)amino)ethyl)cyclopropane-1-carboxylic acid and trifluoroacetic acid (68 µL, 0.89 mmol). 3.7 mg, 18% yield. MS: m/z found 744 [M+H]⁺, retention time = 1.66 min (Method D). ¹H NMR (400 MHz, Methanol-*d*₄) δ 8.68 (d, 1H), 8.51 (s, 1H), 8.07 (s, 1H), 7.82 (d, 1H), 7.71 (d, 1H), 7.57 (t, 1H), 7.48 (d, 1H), 7.43 (d, 1H), 7.30 (d, 1H), 7.08 (s, 1H), 4.34 (s, 2H), 4.15 (s, 2H), 4.06 (s, 3H), 4.04 (s, 3H), 4.00 (s, 1H), 3.26 (t, 2H), 3.06 (t, 2H), 2.40 - 2.31 (m, 3H), 1.86 (d, 3H), 1.17 (s, 2H), 0.70 (s, 2H).

### Example 508: (S)-5-((((6-(2-Chloro-3-(3-chloro-2-(2-((((1r,4r)-4-hydroxycyclohexyl)amino)methyl)-8-methoxyimidazo[1,2-a]pyridin-6-yl)pyridin-4-yl)phenyl)-2-methoxypyridin-3-yl)methyl)amino)methyl)pyrrolidin-2-one (384)

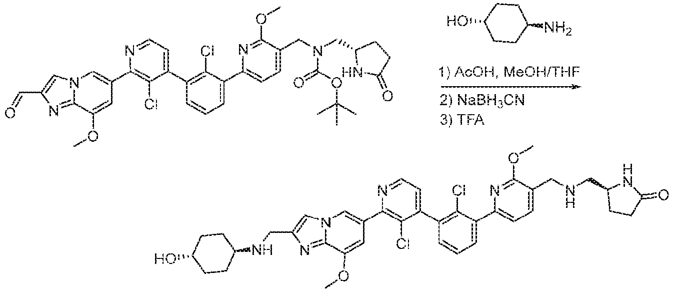

tert-Butyl ((6-(2-chloro-3-(3-chloro-2-(2-((((1r,4r)-4-hydroxycyclohexyl)amino)methyl)-8-methoxyimidazo[1,2-a]pyridin-6-yl)pyridin-4-yl)phenyl)-2-methoxypyridin-3-yl)methyl)(((S)-5-oxopyrrolidin-2-yl)methyl)carbamate was prepared using Reductive Amination Procedure B from tert-butyl (S)-((6-(2-chloro-3-(3-chloro-2-(2-formyl-8-methoxyimidazo[1,2-a]pyridin-6-yl)pyridin-4-yl)phenyl)-2-methoxypyridin-3-yl)methyl)((5-oxopyrrolidin-2-yl)methyl)carbamate (Example 499, step (c)) (20 mg, 0.03 mmol), acetic acid (3 µL, 0.05 mmol), (1r,4r)-4-aminocyclohexan-1-ol (6 mg, 0.05 mmol) and sodium cyanoborohydride (3 mg, 0.05 mmol). (S)-5-((((6-(2-Chloro-3-(3-chloro-2-(2-((((1r,4r)-4-hydroxycyclohexyl)amino)methyl)-8-methoxyimidazo[1,2-a]pyridin-6-yl)pyridin-4-yl)phenyl)-2-methoxypyridin-3-yl)methyl)amino)methyl)pyrrolidin-2-one was prepared using general Boc Deprotection Procedure from tert-butyl ((6-(2-chloro-3-(3-chloro-2-(2-((((1r,4r)-4-hydroxycyclohexyl)amino)methyl)-8-methoxyimidazo[1,2-a]pyridin-6-yl)pyridin-4-yl)phenyl)-2-methoxypyridin-3-yl)methyl)(((S)-5-oxopyrrolidin-2-yl)methyl)carbamate and trifluoroacetic acid (24 µL, 0.31 mmol). 4.7 mg, 24% yield. MS: m/z found 730.2 [M+H]⁺, retention time = 1.50 min (Method D). ¹H NMR (400 MHz, Methanol-*d*₄) δ 8.69 (d, 1H), 8.51 (s, 1H), 8.07 (s, 1H), 7.84 (d, 1H), 7.77 - 7.68 (m, 1H), 7.57 (t, 1H), 7.50 (d, 1H), 7.44 (d, 1H), 7.32 (d, 1H), 7.09 (s, 1H), 4.38 (s, 2H), 4.13 (d, 2H), 4.08 (d, 3H), 4.06 (t, 3H), 3.98 (s, 1H), 3.57 (s, 1H), 3.16 (d, 1H), 3.02 (t, 2H), 2.34 (d, 3H), 2.24 (d, 2H), 2.06 (d, 2H), 1.87 (d, 1H), 1.51 (q, 2H), 1.36 (q, 2H).

### Example 509: 1-(4-(((6-(2-Chloro-3-(3-chloro-2-(8-methoxy-2-(morpholinomethyl)imidazo[1,2-a]pyridin-6-yl)pyridin-4-yl)phenyl)-2-methoxypyridin-3-yl)methyl)amino)piperidin-1-yl)ethan-1-one (475)

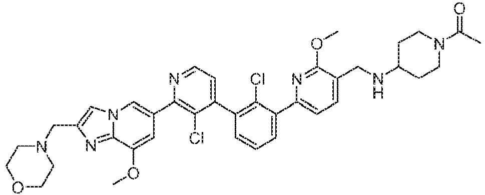

### (a) tert-Butyl (1-acetylpiperidin-4-yl)((6-(2-chloro-3-(3-chloro-2-(2-(hydroxymethyl)-8-methoxyimidazo[1,2-a]pyridin-6-yl)pyridin-4-yl)phenyl)-2-methoxypyridin-3-yl)methyl)carbamate

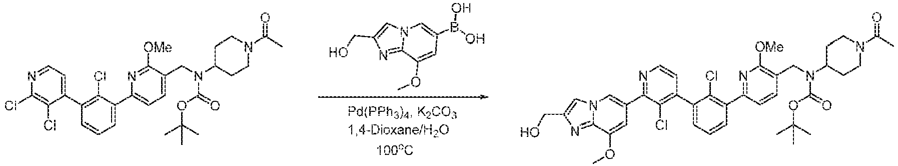

To a mixture of (2-(hydroxymethyl)-8-methoxyimidazo[1,2-a]pyridin-6-yl)boronic acid (Example 499, step (a)) (140.7 mg, 0.63 mmol) and tert-butyl (1-acetylpiperidin-4-yl)((6-(2-chloro-3-(2,3-dichloropyridin-4-yl)phenyl)-2-methoxypyridin-3-yl)methyl)carbamate (262 mg, 0.42 mmol) in 1,4-dioxane/water (4:1, 3 mL), potassium carbonate (175 mg, 1.27 mmol) was added. Nitrogen gas was bubbled through the mixture for two minutes. Tetrakis(triphenylphosphine)palladium(0) (49 mg, 0.04 mmol) was added, and the reaction was stirred at 100 °C for 30 minutes. The reaction mixture was diluted with water (30 mL), and the aqueous layer was extracted with EtOAc (3 x 30 ml). The combined organic layers were washed with brine (20 mL), dried over anhydrous sodium sulfate, filtered, and concentrated. The residue was purified by normal phase silica gel chromatography (0-5% MeOH in DCM) to afford tert-butyl (1-acetylpiperidin-4-yl)((6-(2-chloro-3-(3-chloro-2-(2-(hydroxymethyl)-8-methoxyimidazo[1,2-a]pyridin-6-yl)pyridin-4-yl)phenyl)-2-methoxypyridin-3-yl)methyl)carbamate (110 mg, 34% yield). MS: m/z found 761.1 [M+H]⁺.

### (b) tert-Butyl (1-acetylpiperidin-4-yl)((6-(2-chloro-3-(3-chloro-2-(2-formyl-8-methoxyimidazo[1,2-a]pyridin-6-yl)pyridin-4-yl)phenyl)-2-methoxypyridin-3-yl)methyl)carbamate

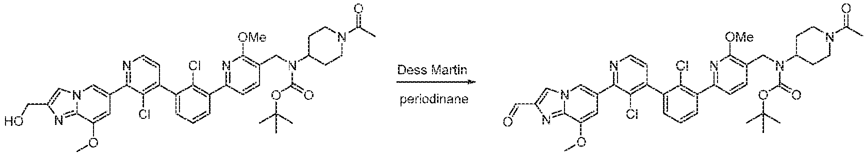

To a solution of tert-butyl (1-acetylpiperidin-4-yl)((6-(2-chloro-3-(3-chloro-2-(2-(hydroxymethyl)-8-methoxyimidazo[1,2-a]pyridin-6-yl)pyridin-4-yl)phenyl)-2-methoxypyridin-3-yl)methyl)carbamate (110 mg, 0.14 mmol) in dichloromethane (3 mL), Dess Martin periodinane (122 mg, 0.29 mmol) was added. The reaction was stirred at room temperature for 2 h. The reaction mixture was concentrated and the residue was purified by normal phase silica gel chromatography (0-5% MeOH in DCM) to tert-butyl (1-acetylpiperidin-4-yl)((6-(2-chloro-3-(3-chloro-2-(2-formyl-8-methoxyimidazo[1,2-a]pyridin-6-yl)pyridin-4-yl)phenyl)-2-methoxypyridin-3-yl)methyl)carbamate (91 mg, 83% yield). MS: m/z found 759 [M+H]⁺.

### (c) 1-(4-(((6-(2-Chloro-3-(3-chloro-2-(8-methoxy-2-(morpholinomethyl)imidazo[1,2-a]pyridin-6-yl)pyridin-4-yl)phenyl)-2-methoxypyridin-3-yl)methyl)amino)piperidin-1-yl)ethan-1-one

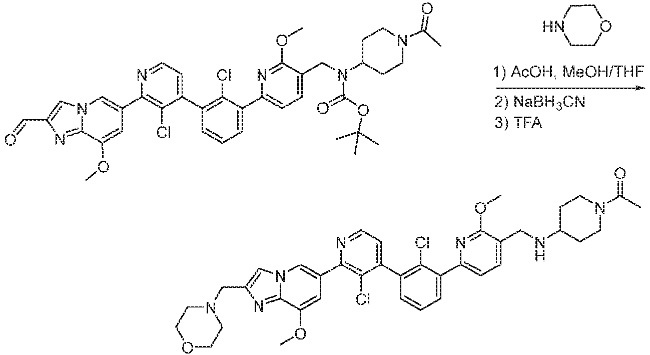

tert-Butyl (1-acetylpiperidin-4-yl)((6-(2-chloro-3-(3-chloro-2-(8-methoxy-2-(morpholinomethyl)imidazo[1,2-a]pyridin-6-yl)pyridin-4-yl)phenyl)-2-methoxypyridin-3-yl)methyl)carbamate was prepared using Reductive Amination Procedure B from tert-butyl (1-acetylpiperidin-4-yl)((6-(2-chloro-3-(3-chloro-2-(2-formyl-8-methoxyimidazo[1,2-a]pyridin-6-yl)pyridin-4-yl)phenyl)-2-methoxypyridin-3-yl)methyl)carbamate (15 mg, 0.02 mmol), acetic acid (2 µL, 0.04 mmol), morpholine (3 mg, 0.04 mmol) and sodium cyanoborohydride (3 mg, 0.04 mmol). 1-(4-(((6-(2-Chloro-3-(3-chloro-2-(8-methoxy-2-(morpholinomethyl)imidazo[1,2-a]pyridin-6-yl)pyridin-4-yl)phenyl)-2-methoxypyridin-3-yl)methyl)amino)piperidin-1-yl)ethan-1-one was prepared using General Boc Deprotection Procedure from tert-butyl (1-acetylpiperidin-4-yl)((6-(2-chloro-3-(3-chloro-2-(8-methoxy-2-(morpholinomethyl)imidazo[1,2-a]pyridin-6-yl)pyridin-4-yl)phenyl)-2-methoxypyridin-3-yl)methyl)carbamate and trifluoroacetic acid (55 µL, 0.72 mmol). 4.7 mg, 33% yield. MS: m/z found 730.1 [M+H]⁺, retention time = 1.46 min (Method D). ¹H NMR (400 MHz, Methanol-*d*₄) δ 8.69 (d, 1H), 8.48 (s, 1H), 7.94 (s, 1H), 7.90 (d, 1H), 7.72 (d, 1H), 7.58 (t, 1H), 7.47 (t, 2H), 7.36 (d, 1H), 7.04 (s, 1H), 4.67 (d, 1H), 4.30 (s, 2H), 4.13 - 4.02 (m, 8H), 3.91 (s, 2H), 3.75 (s, 4H), 3.46 (s, 1H), 3.21 (t, 1H), 2.77 (s, 4H), 2.74 - 2.62 (m, 1H), 2.25 (t, 2H), 2.13 (s, 3H), 1.77 - 1.44 (m, 1H).

### Example 510: 1-(4-(((6-(2-Chloro-3-(3-chloro-2-(2-((3,3-difluoropyrrolidin-1-yl)methyl)-8-methoxyimidazo[1,2-a]pyridin-6-yl)pyridin-4-yl)phenyl)-2-methoxypyridin-3-yl)methyl)amino)piperidin-1-yl)ethan-1-one (476)

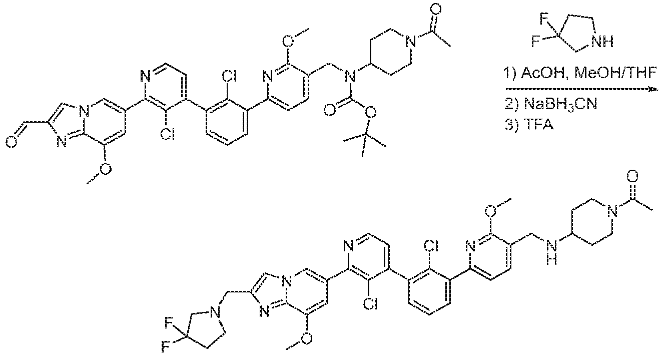

tert-Butyl (1-acetylpiperidin-4-yl)((6-(2-chloro-3-(3-chloro-2-(2-((3,3-difluoropyrrolidin-1-yl)methyl)-8-methoxyimidazo[1,2-a]pyridin-6-yl)pyridin-4-yl)phenyl)-2-methoxypyridin-3-yl)methyl)carbamate was prepared using Reductive Amination Procedure B from tert-butyl (1-acetylpiperidin-4-yl)((6-(2-chloro-3-(3-chloro-2-(2-formyl-8-methoxyimidazo[1,2-a]pyridin-6-yl)pyridin-4-yl)phenyl)-2-methoxypyridin-3-yl)methyl)carbamate (Example 509, step (b)) (15 mg, 0.02 mmol), acetic acid (2 µL, 0.04 mmol), 3,3-difluoropyrrolidine (4 mg, 0.04 mmol) and sodium cyanoborohydride (3 mg, 0.04 mmol). 1-(4-(((6-(2-Chloro-3-(3-chloro-2-(2-((3,3-difluoropyrrolidin-1-yl)methyl)-8-methoxyimidazo[1,2-a]pyridin-6-yl)pyridin-4-yl)phenyl)-2-methoxypyridin-3-yl)methyl)amino)piperidin-1-yl)ethan-1-one was prepared using General Boc Deprotection Procedure from tert-butyl (1-acetylpiperidin-4-yl)((6-(2-chloro-3-(3-chloro-2-(2-((3,3-difluoropyrrolidin-1-yl)methyl)-8-methoxyimidazo[1,2-a]pyridin-6-yl)pyridin-4-yl)phenyl)-2-methoxypyridin-3-yl)methyl)carbamate and trifluoroacetic acid (58 µL, 0.76 mmol). 3.4 mg, 23% yield. MS: m/z found 750.1 [M+H]⁺, retention time = 2.26 min (Method C). ¹H NMR (400 MHz, Methanol-*d*₄) δ 8.74 (d, 1H), 8.71 (s, 1H), 8.19 (s, 1H), 7.91 (d, 1H), 7.73 (d, 1H), 7.59 (t, 1H), 7.54 (d, 2H), 7.47 (d, 1H), 7.37 (d, 1H), 4.68 (d, 1H), 4.33 (s, 2H), 4.17 (s, 6H), 4.09 (t, 5H), 3.51 (s, 1H), 3.17 (t, 3H), 2.75 - 2.61 (m, 1H), 2.43 (m, 2H), 2.32 - 2.19 (m, 2H), 2.14 (d, 4H).

### Example 511: 1-((6-(4-(3-(5-(((1-Acetylpiperidin-4-yl)amino)methyl)-6-methoxypyridin-2-yl)-2-chlorophenyl)-3-chloropyridin-2-yl)-8-methoxyimidazo[1,2-a]pyridin-2-yl)methyl)-3-methylazetidine-3-carbonitrile (477)

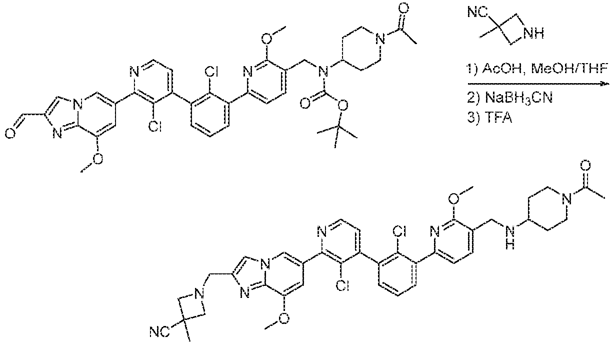

tert-Butyl (1-acetylpiperidin-4-yl)((6-(2-chloro-3-(3-chloro-2-(2-((3-cyano-3-methylazetidin-1-yl)methyl)-8-methoxyimidazo[1,2-a]pyridin-6-yl)pyridin-4-yl)phenyl)-2-methoxypyridin-3-yl)methyl)carbamate was prepared using Reductive Amination Procedure B from tert-butyl (1-acetylpiperidin-4-yl)((6-(2-chloro-3-(3-chloro-2-(2-formyl-8-methoxyimidazo[1,2-a]pyridin-6-yl)pyridin-4-yl)phenyl)-2-methoxypyridin-3-yl)methyl)carbamate (Example 509, step (b)) (15 mg, 0.02 mmol), acetic acid (2 µL, 0.04 mmol), 3-methylazetidine-3-carbonitrile (4 mg, 0.04 mmol) and sodium cyanoborohydride (3 mg, 0.04 mmol). 1-((6-(4-(3-(5-(((1-Acetylpiperidin-4-yl)amino)methyl)-6-methoxypyridin-2-yl)-2-chlorophenyl)-3-chloropyridin-2-yl)-8-methoxyimidazo[1,2-a]pyridin-2-yl)methyl)-3-methylazetidine-3-carbonitrile was prepared using General Boc Deprotection Procedure from tert-butyl (1-acetylpiperidin-4-yl)((6-(2-chloro-3-(3-chloro-2-(2-((3-cyano-3-methylazetidin-1-yl)methyl)-8-methoxyimidazo[1,2-a]pyridin-6-yl)pyridin-4-yl)phenyl)-2-methoxypyridin-3-yl)methyl)carbamate and trifluoroacetic acid (50 µL, 0.65 mmol). 3.9 mg, 27% yield. MS: m/z found 739.2 [M+H]⁺, retention time = 2.17 min (Method C). ¹H NMR (400 MHz, Methanol-*d*₄) δ 8.72 (d, 1H), 8.62 (s, 1H), 8.15 (s, 1H), 7.91 (d, 1H), 7.73 (d, 1H), 7.59 (t, 1H), 7.52 (d, 1H), 7.46 (d, 1H), 7.35 (d, 1H), 4.74 - 4.61 (m, 1H), 4.36 (s, 1H), 4.33 (s, 2H), 4.30 (s, 1H), 4.13 (s, 3H), 4.09 (d, 4H), 3.97 (d, 2H), 3.49 (d, 1H), 3.25 - 3.14 (m, 1H), 2.70 (t, 1H), 2.33 - 2.18 (m, 2H), 2.13 (s, 3H), 1.70 (s, 3H), 1.63 (s, 1H), 1.52 (s, 1H).

### Example 512: 1-(4-(((6-(2-Chloro-3-(3-chloro-2-(2-(((2,2-difluoroethyl)amino)methyl)-8-methoxyimidazo[1,2-a]pyridin-6-yl)pyridin-4-yl)phenyl)-2-methoxypyridin-3-yl)methyl)amino)piperidin-1-yl)ethan-1-one (478)

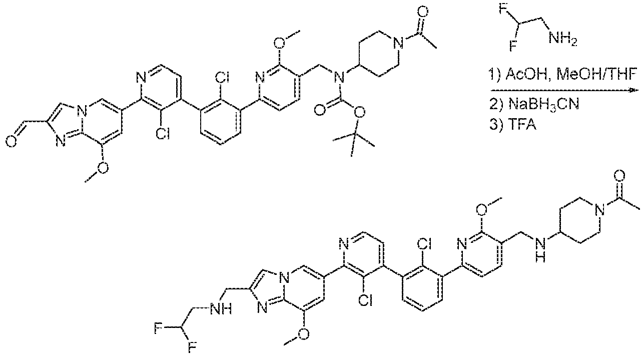

tert-Butyl (1-acetylpiperidin-4-yl)((6-(2-chloro-3-(3-chloro-2-(2-(((2,2-difluoroethyl)amino)methyl)-8-methoxyimidazo[1,2-a]pyridin-6-yl)pyridin-4-yl)phenyl)-2-methoxypyridin-3-yl)methyl)carbamate was prepared using Reductive Amination Procedure B from tert-butyl (1-acetylpiperidin-4-yl)((6-(2-chloro-3-(3-chloro-2-(2-formyl-8-methoxyimidazo[1,2-a]pyridin-6-yl)pyridin-4-yl)phenyl)-2-methoxypyridin-3-yl)methyl)carbamate (Example 509, step (b)) (15 mg, 0.02 mmol), acetic acid (2 µL, 0.04 mmol), 2,2-difluoroethanamine (5 mg, 0.04 mmol) and sodium cyanoborohydride (3 mg, 0.04 mmol). 1-(4-(((6-(2-Chloro-3-(3-chloro-2-(2-(((2,2-difluoroethyl)amino)methyl)-8-methoxyimidazo[1,2-a]pyridin-6-yl)pyridin-4-yl)phenyl)-2-methoxypyridin-3-yl)methyl)amino)piperidin-1-yl)ethan-1-one was prepared using General Boc Deprotection Procedure from tert-Butyl (1-acetylpiperidin-4-yl)((6-(2-chloro-3-(3-chloro-2-(2-(((2,2-difluoroethyl)amino)methyl)-8-methoxyimidazo[1,2-a]pyridin-6-yl)pyridin-4-yl)phenyl)-2-methoxypyridin-3-yl)methyl)carbamate and trifluoroacetic acid (56 µL, 0.73 mmol). 1.6 mg, 11% yield. MS: m/z found 724.2 [M+H]⁺, retention time = 1.59 min (Method D). ¹H NMR (400 MHz, Methanol-*d*₄) δ 8.71 (d, 1H), 8.56 (s, 1H), 8.11 (s, 1H), 7.91 (d, 1H), 7.73 (d, 1H), 7.59 (s, 1H), 7.51 (d, 1H), 7.46 (d, 1H), 7.37 (d, 1H), 7.21 (s, 1H), 4.67 (s, 1H), 4.43 (s, 2H), 4.33 (s, 2H), 4.10 (d, 9H), 3.55 (d, 3H), 3.22 (s, 1H), 2.70 (s, 1H), 2.26 (s, 2H), 2.13 (s, 4H).

### Example 513: (1r,4r)-4-(((6-(2-Chloro-3-(3-chloro-2-(2-((((1r,4r)-4-hydroxycyclohexyl)amino)methyl)-8-methoxyimidazo[1,2-a]pyridin-6-yl)pyridin-4-yl)phenyl)-2-methoxypyridin-3-yl)methyl)amino)cyclohexan-1-ol (453)

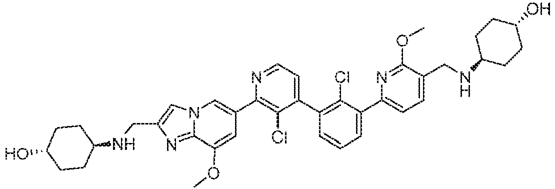

### (a) 6-(2-Chloro-3-(3-chloro-2-(2-(hydroxymethyl)-8-methoxyimidazo[1,2-a]pyridin-6-yl)pyridin-4-yl)phenyl)-2-methoxynicotinaldehyde

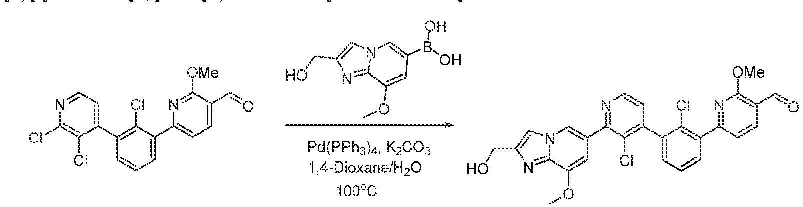

To a mixture of (2-(hydroxymethyl)-8-methoxyimidazo[1,2-a]pyridin-6-yl)boronic acid (Example 499, step (a)) (76 mg, 0.34 mmol) and 6-(2-chloro-3-(2,3-dichloropyridin-4-yl)phenyl)-2-methoxynicotinaldehyde (100 mg, 0.23 mmol) in 1,4-dioxane/water (4:1, 3 mL), potassium carbonate (95 mg, 0.68 mmol) was added. Nitrogen gas was bubbled through the mixture for two minutes. Tetrakis(triphenylphosphine)palladium(0) (26 mg, 0.02 mmol) was added, and the reaction was stirred at 100 °C for 30 minutes. The reaction mixture was diluted with water (30 mL), and the aqueous layer was extracted with EtOAc (3 x 30 ml). The combined organic layers were washed with brine (20 mL), dried over anhydrous sodium sulfate, filtered, and concentrated. The residue was purified by normal phase silica gel chromatography (0-5% MeOH in DCM) to afford 6-(2-chloro-3-(3-chloro-2-(2-(hydroxymethyl)-8-methoxyimidazo[1,2-a]pyridin-6-yl)pyridin-4-yl)phenyl)-2-methoxynicotinaldehyde (91 mg, 75% yield). MS: m/z found 535.1 [M+H]⁺.

### (b) 6-(3-Chloro-4-(2-chloro-3-(5-formyl-6-methoxypyridin-2-yl)phenyl)pyridin-2-yl)-8-methoxyimidazo[1,2-a]pyridine-2-carbaldehyde

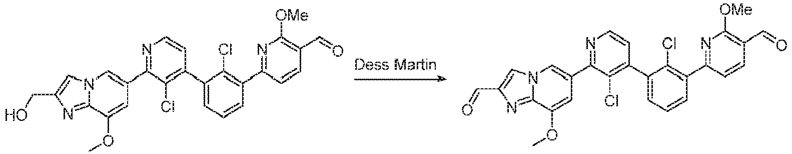

To a solution of 6-(2-chloro-3-(3-chloro-2-(2-(hydroxymethyl)-8-methoxyimidazo[1,2-a]pyridin-6-yl)pyridin-4-yl)phenyl)-2-methoxynicotinaldehyde (91 mg, 0.17 mmol) in dichloromethane (3 mL), Dess Martin periodinane (144 mg, 0.34 mmol) was added. The reaction was stirred at room temperature for 2 h. The reaction mixture was concentrated and the residue was purified by normal phase silica gel chromatography (0-5% MeOH in DCM) to afford 6-(3-chloro-4-(2-chloro-3-(5-formyl-6-methoxypyridin-2-yl)phenyl)pyridin-2-yl)-8-methoxyimidazo[1,2-a]pyridine-2-carbaldehyde (88 mg, 97% yield). MS: m/z found 533 [M+H]⁺.

### (c) (1r,4r)-4-(((6-(2-Chloro-3-(3-chloro-2-(2-((((1r,4r)-4-hydroxycyclohexyl)amino)methyl)-8-methoxyimidazo[1,2-a]pyridin-6-yl)pyridin-4-yl)phenyl)-2-methoxypyridin-3-yl)methyl)amino)cyclohexan-1-ol

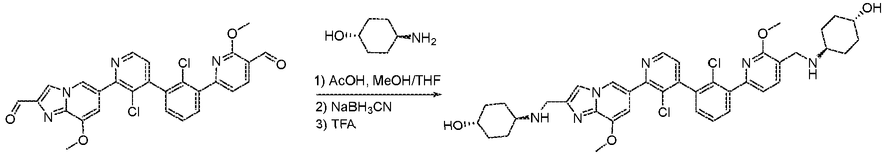

(1r,4r)-4-(((6-(2-Chloro-3-(3-chloro-2-(2-((((1r,4r)-4-hydroxycyclohexyl)amino)methyl)-8-methoxyimidazo[1,2-a]pyridin-6-yl)pyridin-4-yl)phenyl)-2-methoxypyridin-3-yl)methyl)amino)cyclohexan-1-ol was prepared using Reductive Amination Procedure A from 6-(3-chloro-4-(2-chloro-3-(5-formyl-6-methoxypyridin-2-yl)phenyl)pyridin-2-yl)-8-methoxyimidazo[1,2-a]pyridine-2-carbaldehyde (15 mg, 0.03 mmol), acetic acid (3 µL, 0.06 mmol), (1r,4r)-4-aminocyclohexan-1-ol (13 mg, 0.11 mmol) and sodium cyanoborohydride (5 mg, 0.08 mmol). 8.4 mg, 41% yield. MS: m/z found 731.2 [M+H]⁺, retention time = 1.49 min (Method D). ¹H NMR (400 MHz, Methanol-*d*₄) δ 8.73 - 8.67 (m, 1H), 8.51 (d, 1H), 8.07 (s, 1H), 7.89 (d, 1H), 7.76 - 7.68 (m, 1H), 7.58 (t, 1H), 7.50 (dd, 1H), 7.48 - 7.42 (m, 1H), 7.35 (d, 1H), 7.09 (s, 1H), 4.38 (s, 2H), 4.27 (s, 2H), 4.08 (t, 6H), 3.57 (s, 2H), 3.18 (t, 2H), 2.24 (d, 4H), 2.06 (s, 4H), 1.53 (p, 4H), 1.38 (d, 4H).

### Example 514: 2-((6-(2-Chloro-3-(3-chloro-2-(8-methoxy-2-((6-oxo-2,5-diazaspiro[3.4]octan-2-yl)methyl)imidazo[1,2-a]pyridin-6-yl)pyridin-4-yl)phenyl)-2-methoxypyridin-3-yl)methyl)-2,5-diazaspiro[3.4]octan-6-one (454)

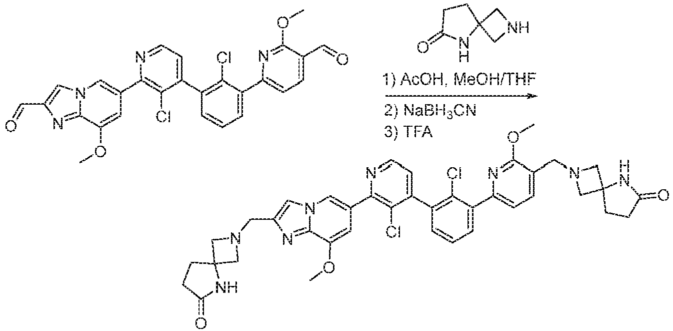

2-((6-(2-Chloro-3-(3-chloro-2-(8-methoxy-2-((6-oxo-2,5-diazaspiro[3.4]octan-2-yl)methyl)imidazo[1,2-a]pyridin-6-yl)pyridin-4-yl)phenyl)-2-methoxypyridin-3-yl)methyl)-2,5-diazaspiro[3.4]octan-6-one was prepared using Reductive Amination Procedure A from 6-(3-chloro-4-(2-chloro-3-(5-formyl-6-methoxypyridin-2-yl)phenyl)pyridin-2-yl)-8-methoxyimidazo[1,2-a]pyridine-2-carbaldehyde (Example 513, step (b)) (15 mg, 0.03 mmol), acetic acid (3 µL, 0.06 mmol) and 2,5-diazaspiro[3.4]octan-6-one hydrochloride (18 mg, 0.11 mmol) and sodium cyanoborohydride (5 mg, 0.08 mmol). 3.5 mg, 17% yield. MS: m/z found 753.1 [M+H]⁺, retention time = 1.46 min (Method D). ¹H NMR (400 MHz, Methanol-*d*₄) 8.69 (d, 1H), 8.49 (s, 1H), 7.98 (s, 1H), 7.80 (d, 1H), 7.72 (d, 1H), 7.57 (t, 1H), 7.51 - 7.46 (m, 1H), 7.44 (d, 1H), 7.31 (d, 1H), 7.07 (d, 1H), 4.23 (s, 2H), 4.12 (s, 2H), 4.07 (s, 3H), 4.04 (d, 5H), 4.00 (d, 3H), 3.95 (d, 2H), 3.89 (d, 2H), 2.45 (s, 4H), 2.38 (d, 4H).

### Example 515: 1-((6-(2-Chloro-3-(3-chloro-2-(2-((3-hydroxy-3-methylazetidin-1-yl)methyl)-8-methoxyimidazo[1,2-a]pyridin-6-yl)pyridin-4-yl)phenyl)-2-methoxypyridin-3-yl)methyl)-3-methylazetidin-3-ol (455)

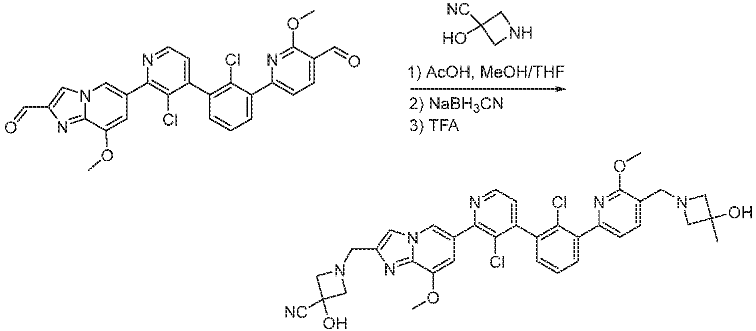

1-((6-(2-Chloro-3-(3-chloro-2-(2-((3-hydroxy-3-methylazetidin-1-yl)methyl)-8-methoxyimidazo[1,2-a]pyridin-6-yl)pyridin-4-yl)phenyl)-2-methoxypyridin-3-yl)methyl)-3-methylazetidin-3-ol was prepared using Reductive Amination Procedure A from 6-(3-chloro-4-(2-chloro-3-(5-formyl-6-methoxypyridin-2-yl)phenyl)pyridin-2-yl)-8-methoxyimidazo[1,2-a]pyridine-2-carbaldehyde (Example 513, step (b)) (15 mg, 0.03 mmol), acetic acid (3 µL, 0.06 mmol), 3-methylazetidin-3-ol hydrochloride (14 mg, 0.11 mmol) and sodium cyanoborohydride (5 mg, 0.08 mmol). 3.5 mg, 18% yield. MS: m/z found 675.2 [M+H]⁺, retention time = 1.46 min (Method D). ¹H NMR (400 MHz, Methanol-*d*₄) δ 8.70 (d, 1H), 8.51 (d, 1H), 8.08 (s, 1H), 7.88 (d, 1H), 7.73 (d, 1H), 7.58 (t, 1H), 7.50 (d, 1H), 7.45 (d, 1H), 7.36 (d, 1H), 7.09 (s, 1H), 4.48 (s, 2H), 4.42 (s, 2H), 4.13 (d, 4H), 4.07 (dd, 6H), 4.02 (t, 4H), 1.52 (d, 6H).

### Example 516: 2-((6-(2-Chloro-3-(3-chloro-2-(2-((6-hydroxy-2-azaspiro[3.3]heptan-2-yl)methyl)-8-methoxyimidazo[1,2-a]pyridin-6-yl)pyridin-4-yl)phenyl)-2-methoxypyridin-3-yl)methyl)-2-azaspiro[3.3]heptan-6-ol (474)

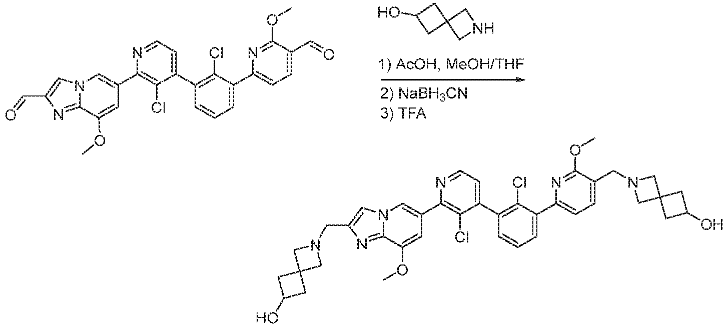

2-((6-(2-Chloro-3-(3-chloro-2-(2-((6-hydroxy-2-azaspiro[3.3]heptan-2-yl)methyl)-8-methoxyimidazo[1,2-a]pyridin-6-yl)pyridin-4-yl)phenyl)-2-methoxypyridin-3-yl)methyl)-2-azaspiro[3.3]heptan-6-ol was prepared using Reductive Amination Procedure A from 6-(3-chloro-4-(2-chloro-3-(5-formyl-6-methoxypyridin-2-yl)phenyl)pyridin-2-yl)-8-methoxyimidazo[1,2-a]pyridine-2-carbaldehyde (Example 513, step (b)) (15 mg, 0.03 mmol), acetic acid (3 µL, 0.06 mmol), 2-azaspiro[3.3]heptan-6-ol hydrochloride (17 mg, 0.11 mmol) and sodium cyanoborohydride (5 mg, 0.08 mmol). 3.4 mg, 17% yield. MS: m/z found 727.2 [M+H]⁺, retention time = 0.56 min (Method B). ¹H NMR (400 MHz, Methanol-*d*₄) δ 8.72 (d, 1H), 8.62 (s, 1H), 8.15 (s, 1H), 7.91 (d, 1H), 7.73 (d, 1H), 7.59 (t, 1H), 7.52 (d, 1H), 7.46 (d, 1H), 7.35 (d, 1H), 4.74 - 4.61 (m, 1H), 4.36 (s, 1H), 4.33 (s, 2H), 4.30 (s, 1H), 4.13 (s, 3H), 4.09 (d, 4H), 3.97 (d, 2H), 3.49 (d, 1H), 3.25 - 3.14 (m, 1H), 2.70 (t, 1H), 2.33 - 2.18 (m, 2H), 2.13 (s, 3H), 1.70 (s, 3H), 1.63 (s, 1H), 1.52 (s, 1H).

### Example 517: (S)-N-(1-((6-(3-chloro-4-(2-chloro-3-(6-methoxy-5-((((5-oxopyrrolidin-2-yl)methyl)amino)methyl)pyridin-2-yl)phenyl)pyridin-2-yl)-1-methyl-1H-pyrrolo[3,2-b]pyridin-3-yl)methyl)piperidin-4-yl)acetamide (377)

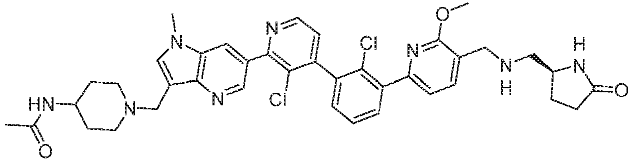

### (a) 1-Methyl-6-(4,4,5,5-tetramethyl-1,3,2-dioxaborolan-2-yl)-1H-pyrrolo[3,2-b]-pyridine-3-carbaldehyde

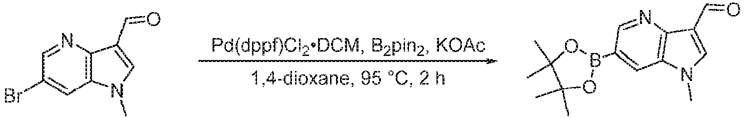

6-Bromo-1-methyl-pyrrolo[3,2-b]pyridine-3-carbaldehyde (457 mg, 1.91 mmol), bis(pinacolato)diborane (680 mg, 2.68 mmol), [1,1'- bis(diphenylphosphino)ferrocene]-dichloropalladium(II) complex with dichloromethane (156 mg, 0.19 mmol), and potassium acetate (525 mg, 5.35 mmol) were suspended in 15 mL 1,4-dioxane in a sealed tube. Nitrogen gas was bubbled through the reaction solution for 2 minutes, then the vessel was sealed and heated at 95 °C for 3 hours. Reaction was filtered through celite, and the filtrate was concentrated under reduced pressure. The dark crude oil was dissolved in 5 mL EOAc and hexane was slowly added until the catalyst precipitated out of solution. The solids were filtered, and the filtrate was concentrated to give a light brown oil. After standing for 15 minutes, a portion of the oil solidified. This entire sample was suspended in 10 mL hexane and sonicated until fine solids formed. The solids were filtered and dried to afford 1-methyl-6-(4,4,5,5-tetramethyl-1,3,2-dioxaborolan-2-yl)-1H-pyrrolo[3,2-b]-pyridine-3-carbaldehyde (401 mg, 73 % yield) as a yellow solid. MS: *m*/*z* found 205 [M+H] ⁺ (boronic acid fragment).

### (b) tert-Butyl (S)-((6-(2-chloro-3-(3-chloro-2-(3-formyl-1-methyl-1H-pyrrolo[3,2-b]pyridin-6-yl)pyridin-4-yl)phenyl)-2-methoxypyridin-3-yl)methyl)((5-oxopyrrolidin-2-yl)methyl)carbamate

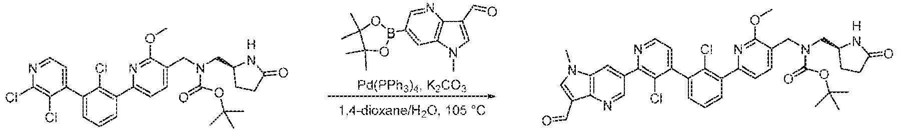

Tetrakis(triphenylphosphine)palladium(0) (20 mg, 0.02 mmol), potassium carbonate (35 mg, 0.25 mmol), tert-butyl N-[[6-[2-chloro-3-(2,3-dichloro-4-pyridyl)phenyl]-2-methoxy-3-pyridyl]methyl]-N-[[(2S)-5-oxopyrrolidin-2-yl]methyl]carbamate (50 mg, 0.08 mmol), and 1-methyl-6-(4,4,5,5-tetramethyl-1,3,2-dioxaborolan-2-yl)-1H-pyrrolo[3,2-b]-pyridine-3-carbaldehyde (36 mg, 0.13 mmol) were suspended in in 1,4-dioxane /water (4:1, 2 mL), then the solution was heated at 105 °C for 20 minutes. Reaction was cooled to room temperature, diluted with 5 mL water, and extracted with EtOAc (3 x 3 mL). The combined organics were concentrated under reduced pressure and the crude sample was purified by silica gel chromatography (0-6 % MeOH/DCM) to afford tert-butyl (S)-((6-(2-chloro-3-(3-chloro-2-(3-formyl-1-methyl-1H-pyrrolo[3,2-b]pyridin-6-yl)pyridin-4-yl)phenyl)-2-methoxypyridin-3-yl)methyl)((5-oxopyrrolidin-2-yl)methyl)carbamate (50 mg, 83 % yield) as a yellow oil. MS: *m*/*z* found 715, 717 [M+H]⁺.

### (c) tert-Butyl (S)-((6-(3-(2-(3-((4-acetamidopiperidin-1-yl)methyl)-1-methyl-1H-pyrrolo[3,2-b]pyridin-6-yl)-3-chloropyridin-4-yl)-2-chlorophenyl)-2-methoxypyridin-3-yl)methyl)((5-oxopyrrolidin-2-yl)methyl)carbamate

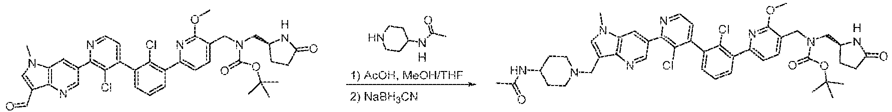

tert-Butyl (S)-((6-(2-chloro-3-(3-chloro-2-(3-formyl-1-methyl-1H-pyrrolo[3,2-b]pyridin-6-yl)pyridin-4-yl)phenyl)-2-methoxypyridin-3-yl)methyl)((5-oxopyrrolidin-2-yl)methyl)carbamate (25 mg, 0.03 mmol), acetic acid (4 mg, 0.07 mmol), and N-(4-piperidyl)acetamide (20 mg, 0.14 mmol) were dissolved in MeOH/THF (1:1, 1 mL), then the solution was stirred at 25 °C for 2 hours. Sodium cyanoborohydride (9 mg, 0.14 mmol) was added and the resulting mixture was stirred at 25 °C for 1 hour. Reaction was subsequently diluted with water (3 mL) and extracted with EtOAc (3 x 3 mL). The combined organics were further washed with brine, dried over sodium sulfate, filtered, and concentrated under reduced pressure to afford tert-butyl (S)-((6-(3-(2-(3-((4-acetamidopiperidin-1-yl)methyl)-1-methyl-1H-pyrrolo[3,2-b]pyridin-6-yl)-3-chloropyridin-4-yl)-2-chlorophenyl)-2-methoxypyridin-3-yl)methyl)((5-oxopyrrolidin-2-yl)methyl)carbamate (29 mg, crude) as a yellow oil. MS: *m*/*z* found 841, 843 [M+H]⁺. Material was used for the next step without further purification.

### (d) (S)-N-(1-((6-(3-chloro-4-(2-chloro-3-(6-methoxy-5-((((5-oxopyrrolidin-2-yl)methyl)amino)methyl)pyridin-2-yl)phenyl)pyridin-2-yl)-1-methyl-1H-pyrrolo [3,2-b]pyridin-3-yl)methyl)piperidin-4-yl)acetamide

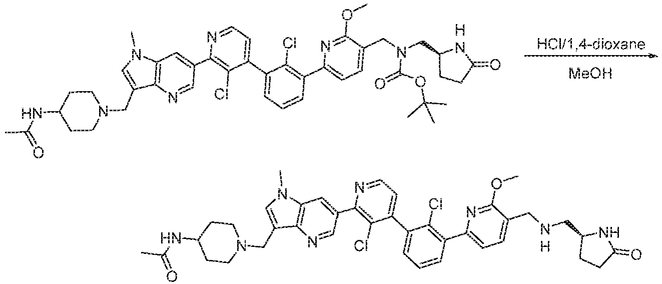

tert-Butyl (S)-((6-(3-(2-(3-((4-acetamidopiperidin-1-yl)methyl)-1-methyl-1H-pyrrolo[3,2-b]pyridin-6-yl)-3-chloropyridin-4-yl)-2-chlorophenyl)-2-methoxypyridin-3-yl)methyl)((5-oxopyrrolidin-2-yl)methyl)carbamate (29 mg, 0.03 mmol, crude) was dissolved in 1 mL MeOH and 4M HCl solution in 1,4-dioxane (27 µL, 0.11 mmol) was added. Reaction was stirred at room temperature for 60 minutes and solvent was removed under reduced pressure. Crude sample was purified by reverse phase HPLC to afford (S)-N-(1-((6-(3-chloro-4-(2-chloro-3-(6-methoxy-5-((((5-oxopyrrolidin-2-yl)methyl)amino)methyl)pyridin-2-yl)phenyl)pyridin-2-yl)-1-methyl-1H-pyrrolo[3,2-b]pyridin-3-yl)methyl)piperidin-4-yl)acetamide (7 mg, 29 % yield) as a white solid (formic acid salt). LC/MS: *m*/*z* found 741, 743 [M+H]⁺, retention time = 2.08 min (Method C). ¹H NMR (400 MHz, Methanol-*d*₄) δ 8.79 (s, 1H), 8.71 (s, 1H), 8.35 - 8.30 (m, 1H), 7.88 (s, 1H), 7.80 (d, 1H), 7.72 (d, 1H), 7.57 (t, 1H), 7.49 (d, 1H), 7.44 (d, 1H), 7.29 (d, 1H), 4.55 - 4.46 (m, 2H), 4.04 (s, 4H), 3.97 (s, 5H), 3.90 (s, 2H), 3.51 (s, 2H), 3.12 (s, 1H), 2.84 (d, 2H), 2.32 (d, 3H), 2.09 (d, 2H), 1.92 (s, 3H), 1.80 (d, 2H).

### Example 518: (S)-2-((6-(3-Chloro-4-(2-chloro-3-(6-methoxy-5-((((5-oxopyrrolidin-2-yl)methyl)amino)methyl)pyridin-2-yl)phenyl)pyridin-2-yl)-1-methyl-1H-pyrrolo[3,2-b]pyridin-3-yl)methyl)-2,6-diazaspiro[3.4]octan-7-one (378)

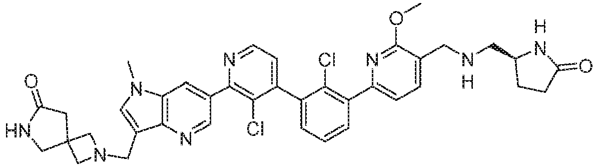

(S)-2-((6-(3-Chloro-4-(2-chloro-3-(6-methoxy-5-((((5-oxopyrrolidin-2-yl)methyl)amino)methyl)pyridin-2-yl)phenyl)pyridin-2-yl)-1-methyl-1H-pyrrolo[3,2-b]pyridin-3-yl)methyl)-2,6-diazaspiro[3.4]octan-7-one was prepared in similar manner as described for Example 517, utilizing intermediate tert-butyl (S)-((6-(2-chloro-3-(3-chloro-2-(3-formyl-1-methyl-1H-pyrrolo[3,2-b]pyridin-6-yl)pyridin-4-yl)phenyl)-2-methoxypyridin-3-yl)methyl)((5-oxopyrrolidin-2-yl)methyl)carbamate and replacing N-(4-piperidyl)acetamide with 2,6-diazaspiro[3.4]octan-7-one in step (c). Product was obtained as a white solid (formic acid salt). LC/MS: *m*/*z* found 725, 727 [M+H]⁺, retention time = 2.01 min (Method C). ¹H NMR (400 MHz, Methanol-*d*₄) δ 8.78 (d, 1H), 8.71 (d, 1H), 8.45 (s, 1H), 8.30 (d, 1H), 7.84 - 7.75 (m, 2H), 7.72 (d, 1H), 7.56 (t, 1H), 7.49 (d, 1H), 7.44 (d, 1H), 7.31 - 7.23 (m, 1H), 4.48 (s, 2H), 4.07 (s, 4H), 4.03 (d, 3H), 3.94 (dd, 5H), 3.88 (s, 1H), 3.61 (s, 2H), 2.79 (d, 2H), 2.64 (s, 2H), 2.33 (q, 3H), 1.83 (s, 1H).

### Example 519: 1-(4-(((6-(3-(2-(4-(((1-Acetylpiperidin-4-yl)amino)methyl)-3-fluorophenyl)-3-chloropyridin-4-yl)-2-chlorophenyl)-2-methoxypyridin-3-yl)methyl)amino)piperidin-1-yl)ethan-1-one (282)

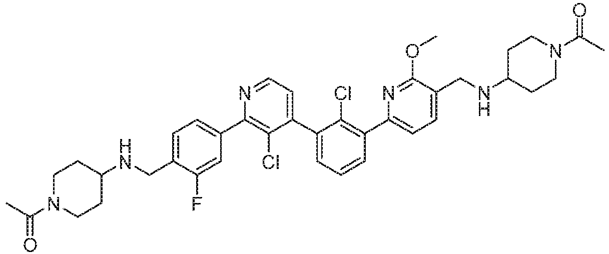

### (a) 6-(2-Chloro-3-(3-chloro-2-(3-fluoro-4-formylphenyl)pyridin-4-yl)phenyl)-2-methoxynicotinaldehyde

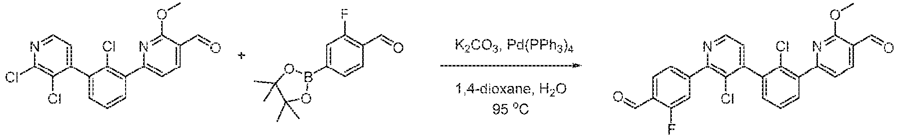

Following Suzuki Coupling Procedure A from 6-(2-chloro-3-(2,3-dichloropyridin-4-yl)phenyl)-2-methoxynicotinaldehyde (200 mg, 0.51 mmol) and 2-fluoro-4-(4,4,5,5-tetramethyl-1,3,2-dioxaborolan-2-yl)benzaldehyde (127 mg, 0.51 mmol) provided 6-(2-chloro-3-(3-chloro-2-(3-fluoro-4-formylphenyl)pyridin-4-yl)phenyl)-2-methoxynicotinaldehyde (220 mg, 90% yield). LCMS: m/z found 481.1 [M+H]⁺, retention time = 1.10 min (Method B).

### (b) 1-(4-(((6-(3-(2-(4-(((1-Acetylpiperidin-4-yl)amino)methyl)-3-fluorophenyl)-3-chloropyridin-4-yl)-2-chlorophenyl)-2-methoxypyridin-3-yl)methyl)amino)piperidin-1-yl)ethan-1-one

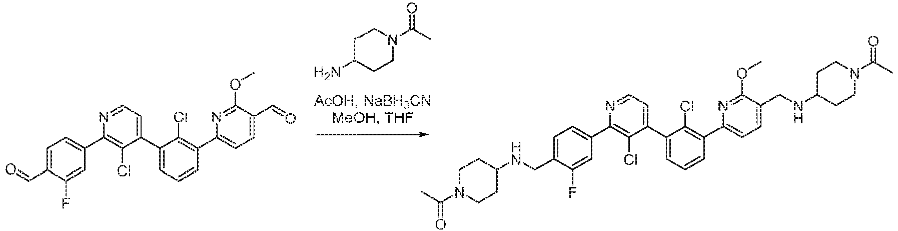

This compound was prepared as the formic acid salt following Reductive Amination Procedure A from 6-(2-chloro-3-(3-chloro-2-(3-fluoro-4-formylphenyl)pyridin-4-yl)phenyl)-2-methoxynicotinaldehyde (50 mg, 0.10 mmol) and 1-(4-amino-1-piperidyl)ethanone (59 mg, 0.42 mmol). 66% yield. LCMS: m/z found 733.3 [M+H]⁺, retention time = 1.64 min (Method D). ¹H NMR (400 MHz, Methanol-*d*₄): δ 8.64 (d, 1H), 8.54 (s, 2H), 7.78 (d, 1H), 7.71 (dd, 1H), 7.63 - 7.51 (m, 3H), 7.49 - 7.38 (m, 3H), 7.28 (d, 1H), 4.50 (t, 2H), 4.03 (s, 3H), 4.00 - 3.88 (m, 6H), 3.20 - 3.10 (m, 2H), 2.87 (dd, 2H), 2.71 (t, 2H), 2.12 - 2.09 (m, 6H), 2.09 - 1.98 (m, 4H), 1.49 - 1.22 (m, 4H).

### Example 520: 1-(6-(2-Chloro-3-(3-chloro-2-(3-fluoro-4-((methylamino)methyl)phenyl)pyridin-4-yl)phenyl)-2-methoxypyridin-3-yl)-N-methylmethanamine (283)

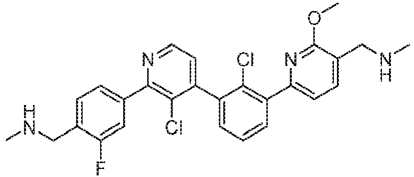

1-(6-(2-Chloro-3-(3-chloro-2-(3-fluoro-4-((methylamino)methyl)phenyl)pyridin-4-yl)phenyl)-2-methoxypyridin-3-yl)-N-methylmethanamine was prepared as the formic acid salt following Reductive Amination Procedure A from 6-(2-chloro-3-(3-chloro-2-(3-fluoro-4-formylphenyl)pyridin-4-yl)phenyl)-2-methoxynicotinaldehyde (Example 519, step (a)) (50 mg, 0.10 mmol) and methylamine solution (33% wt in ethanol, 0.42 mmol). 38% yield. LCMS: m/z found 511.1 [M+H]⁺, retention time = 1.60 min (Method D). ¹H NMR (400 MHz, Methanol-*d*₄): δ 8.71 - 8.63 (m, 1H), 8.54 (s, 2H), 7.84 (d, 1H), 7.77 - 7.68 (m, 1H), 7.65 - 7.52 (m, 4H), 7.49 - 7.42 (m, 2H), 7.34 (d, 1H), 4.16 (s, 4H), 4.08 (s, 3H), 2.71 (s, 3H), 2.65 (s, 3H).

### Example 521: 2-((6-(2-Chloro-3-(3-chloro-2-(3-fluoro-4-((7-oxo-2,6-diazaspiro[3.4]octan-2-yl)methyl)phenyl)pyridin-4-yl)phenyl)-2-methoxypyridin-3-yl)methyl)-2,6-diazaspiro[3.4]octan-7-one (284)

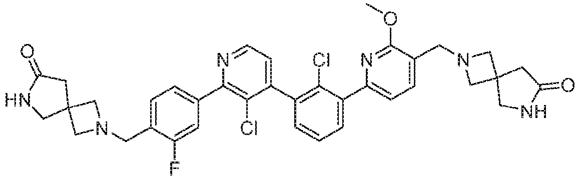

2-((6-(2-Chloro-3-(3-chloro-2-(3-fluoro-4-((7-oxo-2,6-diazaspiro[3.4]octan-2-yl)methyl)phenyl)pyridin-4-yl)phenyl)-2-methoxypyridin-3-yl)methyl)-2,6-diazaspiro[3.4]octan-7-one was prepared as the formic acid salt following Reductive Amination Procedure A from 6-(2-chloro-3-(3-chloro-2-(3-fluoro-4-formylphenyl)pyridin-4-yl)phenyl)-2-methoxynicotinaldehyde (Example 519, step (a)) (50 mg, 0.10 mmol) and 2,6-diazaspiro[3.4]octan-7-one (52 mg, 0.42 mmol). 42% yield. LCMS: m/z found 701.2 [M+H]⁺, retention time = 1.59 min (Method D). ¹H NMR (400 MHz, Methanol-*d*₄): δ 8.68 - 8.60 (m, 1H), 8.49 (s, 2H), 7.71 (t, 2H), 7.58 - 7.49 (m, 3H), 7.48 - 7.35 (m, 3H), 7.26 (d, 1H), 4.01 (s, 3H), 3.82 (s, 4H), 3.62 - 3.51 (m, 8H), 3.49 - 3.40 (m, 4H), 2.63 - 2.53 (m, 4H).

### Example 522: 1-(4-(((6-(3-(2-(4-(((1-Acetylpiperidin-4-yl)amino)methyl)-3-(difluoromethoxy)phenyl)-3-chloropyridin-4-yl)-2-chlorophenyl)-2-methoxypyridin-3-yl)methyl)amino)piperidin-1-yl)ethan-1-one (394)

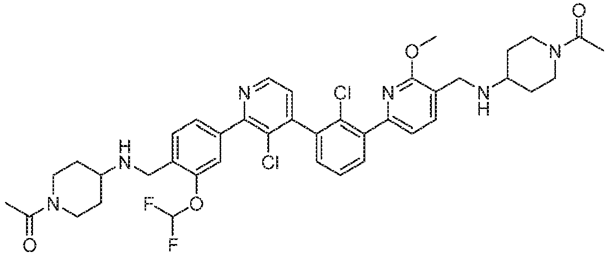

### (a) 6-(2-Chloro-3-(3-chloro-2-(3-(difluoromethoxy)-4-formylphenyl)pyridin-4-yl)phenyl)-2-methoxynicotinaldehyde

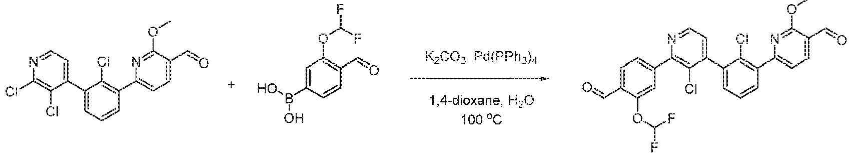

To a mixture of 6-(2-chloro-3-(2,3-dichloropyridin-4-yl)phenyl)-2-methoxynicotinaldehyde (150 mg, 0.38 mmol), (3-(difluoromethoxy)-4-formylphenyl)boronic acid (82 mg, 0.38 mmol) and potassium carbonate (158 mg, 1.14 mmol) in degassed 1,4-dioxane/water (6: 1) was added tetrakis(triphenylphosphine)palladium(0) (44 mg, 0.04 mmol) and the mixture was stirred at 100 °C for 30 min. Water was added and the mixture was extracted into EtOAc three times. The combined organic layers were dried over anhydrous sodium sulfate, concentrated, and purified on silica gel column using 2% MeOH in DCM as eluent to provide 6-(2-chloro-3-(3-chloro-2-(3-(difluoromethoxy)-4-formylphenyl)pyridin-4-yl)phenyl)-2-methoxynicotinaldehyde (190 mg, 94%). LCMS: m/z found 529.1 [M+H]⁺, retention time = 1.11 min (Method B).

### (b) 1-(4-(((6-(3-(2-(4-(((1-Acetylpiperidin-4-yl)amino)methyl)-3-(difluoromethoxy)phenyl)-3-chloropyridin-4-yl)-2-chlorophenyl)-2-methoxypyridin-3-yl)methyl)amino)piperidin-1-yl)ethan-1-one

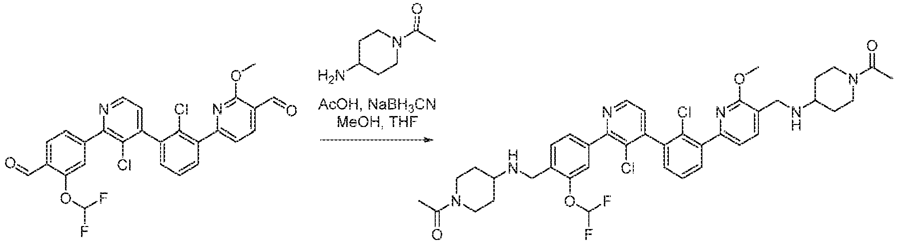

1-(4-(((6-(3-(2-(4-(((1-Acetylpiperidin-4-yl)amino)methyl)-3-(difluoromethoxy)phenyl)-3-chloropyridin-4-yl)-2-chlorophenyl)-2-methoxypyridin-3-yl)methyl)amino)piperidin-1-yl)ethan-1-one was prepared as the formic acid salt following Reductive Amination Procedure A from 6-(2-chloro-3-(3-chloro-2-(3-(difluoromethoxy)-4-formylphenyl)pyridin-4-yl)phenyl)-2-methoxynicotinaldehyde (20 mg, 0.04 mmol) and 1-(4-amino-1-piperidyl)ethanone (21 mg, 0.15 mmol). 58% yield. LCMS: m/z found 781.3 [M+H]⁺, retention time = 1.75 min (Method D). ¹H NMR (400 MHz, Methanol-*d*₄): δ 8.67 (d, 1H), 8.51 - 8.42 (m, 2H), 7.86 (d, 1H), 7.72 (d, 1H), 7.65 (q, 2H), 7.60 - 7.53 (m, 2H), 7.50 - 7.41 (m, 2H), 7.33 (dd, 1H), 7.03 (t, 1H), 4.67 - 4.51 (m, 2H), 4.17 (s, 4H), 4.07 (s, 3H), 4.05 - 3.96 (m, 1H), 3.26 - 3.08 (m, 4H), 2.71 (t, 2H), 2.25 - 2.05 (m, 10H), 1.66 - 1.31 (m, 4H).

### Example 523: 2-((6-(2-Chloro-3-(3-chloro-2-(3-(difluoromethoxy)-4-((7-oxo-2,6-diazaspiro[3.4]octan-2-yl)methyl)phenyl)pyridin-4-yl)phenyl)-2-methoxypyridin-3-yl)methyl)-2,6-diazaspiro[3.4]octan-7-one (597)

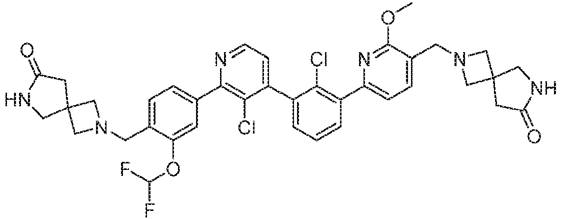

2-((6-(2-Chloro-3-(3-chloro-2-(3-(difluoromethoxy)-4-((7-oxo-2,6-diazaspiro[3.4]octan-2-yl)methyl)phenyl)pyridin-4-yl)phenyl)-2-methoxypyridin-3-yl)methyl)-2,6-diazaspiro[3.4]octan-7-one was prepared as the formic acid salt following Reductive Amination Procedure A from 6-(2-chloro-3-(3-chloro-2-(3-(difluoromethoxy)-4-formylphenyl)pyridin-4-yl)phenyl)-2-methoxynicotinaldehyde (Example 522, step (a)) (60 mg, 0.11 mmol) and 2,6-diazaspiro[3.4]octan-7-one (57 mg, 0.45 mmol). 47% yield. LCMS: m/z found 749.2 [M+H]⁺, retention time = 2.65 min (Method A). ¹H NMR (400 MHz, Methanol-*d*₄): δ 8.73 - 8.59 (m, 1H), 8.40 (s, 2H), 7.78 (d, 1H), 7.71 (d, 1H), 7.66 - 7.49 (m, 4H), 7.48 - 7.39 (m, 2H), 7.30 (d, 1H), 6.97 (t, 1H), 4.09 - 4.00 (m, 5H), 3.97 (s, 2H), 3.81 (s, 4H), 3.66 - 3.56 (m, 8H), 2.65 (s, 2H), 2.61 (s, 2H).

### Example 524: 1-(6-((6-(3-(2-(4-((6-Acetyl-2,6-diazaspiro[3.3]heptan-2-yl)methyl)-3-(difluoromethoxy)phenyl)-3-chloropyridin-4-yl)-2-chlorophenyl)-2-methoxypyridin-3-yl)methyl)-2,6-diazaspiro[3.3]heptan-2-yl)ethan-1-one (598)

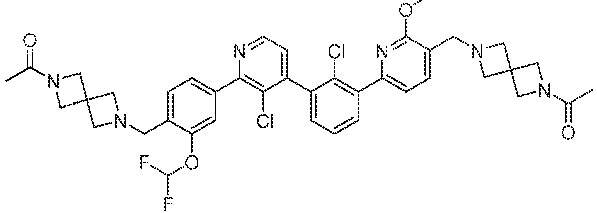

1-(6-((6-(3-(2-(4-((6-Acetyl-2,6-diazaspiro[3.3]heptan-2-yl)methyl)-3-(difluoromethoxy)phenyl)-3-chloropyridin-4-yl)-2-chlorophenyl)-2-methoxypyridin-3-yl)methyl)-2,6-diazaspiro[3.3]heptan-2-yl)ethan-1-one was prepared as the formic acid salt following Reductive Amination Procedure A from 6-(2-chloro-3-(3-chloro-2-(3-(difluoromethoxy)-4-formylphenyl)pyridin-4-yl)phenyl)-2-methoxynicotinaldehyde (Example 522, step (a)) (60 mg, 0.11 mmol) and 1-(2,6-diazaspiro[3.3]heptan-2-yl)ethanone (64 mg, 0.45 mmol). 51% yield. LCMS: m/z found 777.3 [M+H]⁺, retention time = 2.78 min (Method A). ¹H NMR (400 MHz, Methanol-*d*₄): δ 8.65 (d, 1H), 8.46 (s, 2H), 7.74 (d, 1H), 7.71 (dd, 1H), 7.62 - 7.51 (m, 4H), 7.46 (d, 1H), 7.42 (dd, 1H), 7.29 (d, 1H), 6.96 (t, 1H), 4.40 - 4.23 (m, 4H), 4.12 - 4.04 (m, 4H), 4.02 (s, 3H), 3.92 (s, 2H), 3.88 (s, 2H), 3.84 - 3.74 (m, 4H), 3.68 - 3.59 (m, 4H), 1.85 (s, 6H).

### Example 525: (S)-5-((((6-(2-Chloro-3-(3-chloro-2-(3-(difluoromethoxy)-4-(((((S)-5-oxopyrrolidin-2-yl)methyl)amino)methyl)phenyl)pyridin-4-yl)phenyl)-2-methoxypyridin-3-yl)methyl)amino)methyl)pyrrolidin-2-one (617)

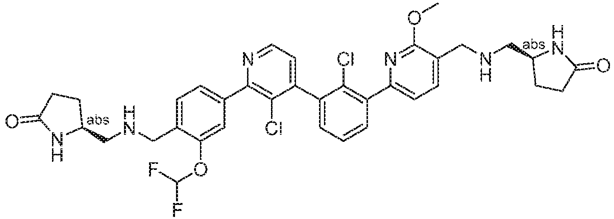

(S)-5-((((6-(2-Chloro-3-(3-chloro-2-(3-(difluoromethoxy)-4-(((((S)-5-oxopyrrolidin-2-yl)methyl)amino)methyl)phenyl)pyridin-4-yl)phenyl)-2-methoxypyridin-3-yl)methyl)amino)methyl)pyrrolidin-2-one was prepared as the formic acid salt following Reductive Amination Procedure A from 6-(2-chloro-3-(3-chloro-2-(3-(difluoromethoxy)-4-formylphenyl)pyridin-4-yl)phenyl)-2-methoxynicotinaldehyde (Example 522, step (a)) (50 mg, 0.09 mmol) and (S)-5-(aminomethyl)pyrrolidin-2-one (43 mg, 0.38 mmol). 38% yield. LCMS: m/z found 725.2 [M+H]⁺, retention time = 2.67 min (Method A). ¹H NMR (400 MHz, Methanol-*d*₄): δ 8.66 (d, 1H), 8.41 (s, 2H), 7.80 (d, 1H), 7.72 (dd, 1H), 7.66 - 7.51 (m, 4H), 7.46 (d, 1H), 7.43 (dd, 1H), 7.30 (d, 1H), 6.98 (t, 1H), 4.05 (s, 3H), 4.03 - 3.95 (m, 4H), 3.95 - 3.82 (m, 2H), 2.96 - 2.72 (m, 4H), 2.44 - 2.22 (m, 6H), 1.94 - 1.73 (m, 2H).

### Example 526: N-((6-(2-Chloro-3-(3-chloro-2-(3-(difluoromethoxy)-4-(((tetrahydro-2H-pyran-4-yl)amino)methyl)phenyl)pyridin-4-yl)phenyl)-2-methoxypyridin-3-yl)methyl)tetrahydro-2H-pyran-4-amine (618)

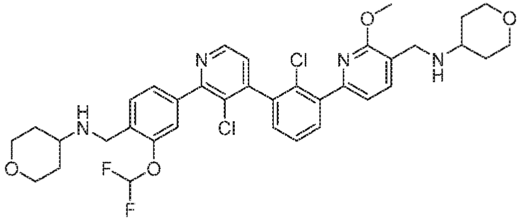

N-((6-(2-Chloro-3-(3-chloro-2-(3-(difluoromethoxy)-4-(((tetrahydro-2H-pyran-4-yl)amino)methyl)phenyl)pyridin-4-yl)phenyl)-2-methoxypyridin-3-yl)methyl)tetrahydro-2H-pyran-4-amine was prepared as the formic acid salt following Reductive Amination Procedure A from 6-(2-chloro-3-(3-chloro-2-(3-(difluoromethoxy)-4-formylphenyl)pyridin-4-yl)phenyl)-2-methoxynicotinaldehyde (Example 522, step (a)) (50 mg, 0.09 mmol) and tetrahydropyran-4-amine (38 mg, 0.38 mmol). 40% yield. LCMS: m/z found 699.2 [M+H]⁺, retention time = 3.01 min (Method A). ¹H NMR (400 MHz, Methanol-*d*₄): δ 8.66 (d, 1H), 8.53 (s, 2H), 7.81 (d, 1H), 7.72 (d, 1H), 7.67 - 7.51 (m, 4H), 7.46 (d, 1H), 7.43 (dd, 1H), 7.30 (d, 1H), 7.00 (t, 1H), 4.10 - 3.88 (m, 11H), 3.43 (tt, 4H), 3.12 - 2.87 (m, 2H), 1.99 (t, 4H), 1.67 - 1.43 (m, 4H).

### Example 527: 4-(((6-(2-Chloro-3-(3-chloro-2-(3-(difluoromethoxy)-4-(((1-methyl-2-oxopiperidin-4-yl)amino)methyl)phenyl)pyridin-4-yl)phenyl)-2-methoxypyridin-3-yl)methyl)amino)-1-methylpiperidin-2-one (619)

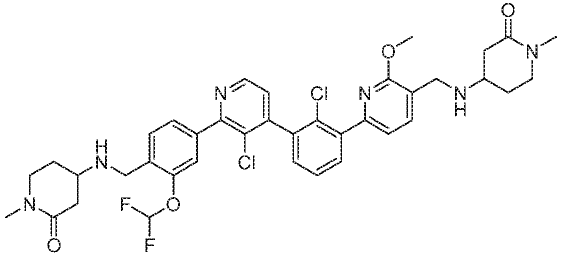

4-(((6-(2-Chloro-3-(3-chloro-2-(3-(difluoromethoxy)-4-(((1-methyl-2-oxopiperidin-4-yl)amino)methyl)phenyl)pyridin-4-yl)phenyl)-2-methoxypyridin-3-yl)methyl)amino)-1-methylpiperidin-2-one was prepared as racemate and the formic acid salt following Reductive Amination Procedure A from 6-(2-chloro-3-(3-chloro-2-(3-(difluoromethoxy)-4-formylphenyl)pyridin-4-yl)phenyl)-2-methoxynicotinaldehyde (Example 522, step (a)) (50 mg, 0.09 mmol) and 4-amino-1-methyl-piperidin-2-one (48 mg, 0.38 mmol). 42% yield. LCMS: m/z found 753.3 [M+H]⁺, retention time = 2.73 min (Method A). ¹H NMR (400 MHz, Methanol-*d*₄): δ 8.65 (d, 1H), 8.46 (s, 2H), 7.80 (d, 1H), 7.71 (dd, 1H), 7.67 - 7.58 (m, 2H), 7.58 - 7.50 (m, 2H), 7.46 (d, 1H), 7.42 (dd, 1H), 7.29 (d, 1H), 6.98 (t, 1H), 4.04 (s, 3H), 4.03 - 3.92 (m, 4H), 3.50 - 3.42 (m, 2H), 3.42 - 3.34 (m, 2H), 3.27 - 3.06 (m, 2H), 2.98 - 2.84 (m, 6H), 2.81 - 2.60 (m, 2H), 2.37 - 2.14 (m, 4H), 1.90 - 1.73 (m, 2H).

### Example 528: 2-(4-(((6-(2-Chloro-3-(3-chloro-2-(3-(difluoromethoxy)-4-(((1-(2-hydroxyethyl)piperidin-4-yl)amino)methyl)phenyl)pyridin-4-yl)phenyl)-2-methoxypyridin-3-yl)methyl)amino)piperidin-1-yl)ethan-1-ol (620)

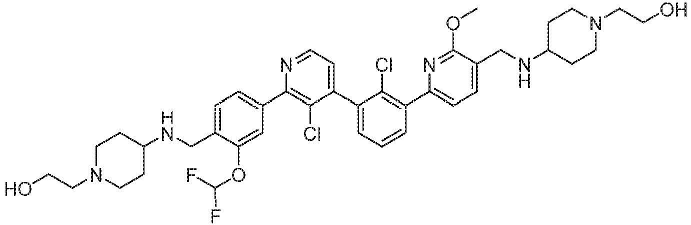

2-(4-(((6-(2-Chloro-3-(3-chloro-2-(3-(difluoromethoxy)-4-(((1-(2-hydroxyethyl)piperidin-4-yl)amino)methyl)phenyl)pyridin-4-yl)phenyl)-2-methoxypyridin-3-yl)methyl)amino)piperidin-1-yl)ethan-1-ol was prepared as the formic acid salt following Reductive Amination Procedure A from 6-(2-chloro-3-(3-chloro-2-(3-(difluoromethoxy)-4-formylphenyl)pyridin-4-yl)phenyl)-2-methoxynicotinaldehyde (Example 522, step (a)) (50 mg, 0.09 mmol) and 2-(4-amino-1-piperidyl)ethanol (54 mg, 0.38 mmol). 34% yield. LCMS: m/z found 785.3 [M+H]⁺, retention time = 2.23 min (Method A). ¹H NMR (400 MHz, Methanol-*d*₄): δ 8.67 (d, 1H), 8.44 (s, 4H), 7.83 (d, 1H), 7.72 (dd, 1H), 7.68 - 7.60 (m, 2H), 7.60 - 7.53 (m, 2H), 7.47 (d, 1H), 7.44 (dd, 1H), 7.31 (d, 1H), 7.01 (t, 1H), 4.15 - 4.01 (m, 7H), 3.86 - 3.73 (m, 4H), 3.50 - 3.41 (m, 2H), 3.41 - 3.34 (m, 3H), 3.10 - 2.94 (m, 4H), 2.94 - 2.75 (m, 3H), 2.74 - 2.55 (m, 2H), 2.26 - 2.11 (m, 4H), 1.85 - 1.65 (m, 4H).

### Example 529: 2-((6-(3-(2-(4-((6-Acetyl-2,6-diazaspiro[3.3]heptan-2-yl)methyl)-3-(difluoromethoxy)phenyl)-3-chloropyridin-4-yl)-2-chlorophenyl)-2-methoxypyridin-3-yl)methyl)-2,6-diazaspiro[3.4]octan-7-one (662)

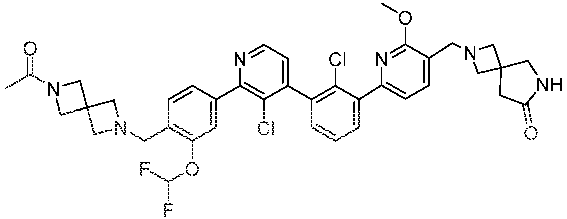

### (a) 2-((6-(2-Chloro-3-(2,3-dichloropyridin-4-yl)phenyl)-2-methoxypyridin-3-yl)methyl)-2,6-diazaspiro [3.4] octan-7-one

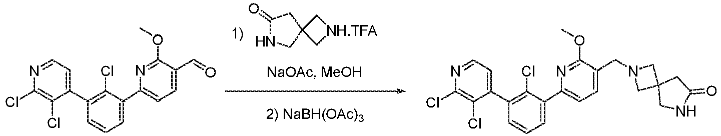

To a mixture of 6-(2-chloro-3-(2,3-dichloropyridin-4-yl)phenyl)-2-methoxynicotinaldehyde (Example 1, step (a)) (600 mg, 1.52 mmol) and 2,6-diazaspiro[3.4]octan-7-one trifluoroacetate (1.46 g, 6.10 mmol) in methanol (10 mL) was added sodium acetate (1.25 g, 15.2 mmol) and the mixture stirred at room temperature for 3 h under N₂. Sodium triacetoxyborohydride (1.62 g, 7.62 mmol) was then added and the mixture stirred at room temperature for 0.5 h under N₂. The mixture was filtered, concentrated and the residue purified by normal phase SiO₂ chromatography (0-45% ethyl acetate/petroleum ether) to afford 2-((6-(2-chloro-3-(2,3-dichloropyridin-4-yl)phenyl)-2-methoxypyridin-3-yl)methyl)-2,6-diazaspiro[3.4]octan-7-one (480 mg, 56% yield) as a white solid. MS: *m*/*z* found 503 [M+H]⁺.

### (b) 2-(Difluoromethoxy)-4-(4,4,5,5-tetramethyl-1,3,2-dioxaborolan-2-yl)benzaldehyde

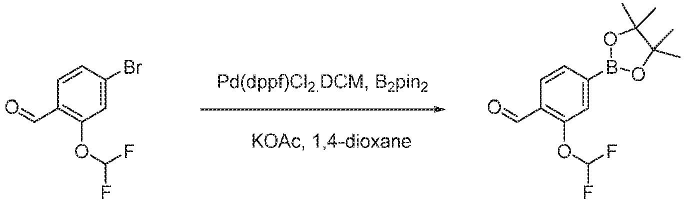

To a mixture of 4-bromo-2-(difluoromethoxy)benzaldehyde (300 mg, 1.20 mmol) and bis(pinacolato)diborane (394 mg, 1.55 mmol) in 1,4-dioxane (3 mL) was added potassium acetate (234 mg, 2.39 mmol), [1,1'- bis(diphenylphosphino)ferrocene]dichloropalladium(II) complex with dichloromethane (97.6 mg, 0.119 mmol) and the mixture stirred at 100 °C for 1 h under N₂. Water (10 mL) was added to the mixture and extracted with ethyl acetate (10 mL x 2). The combined organic phases were dried over anhydrous sulfate, filtered and concentrated under reduced pressure. The residue was purified by normal phase SiO₂ chromatography (0-15% ethyl acetate/petroleum ether) to afford 2-(difluoromethoxy)-4-(4,4,5,5-tetramethyl-1,3,2-dioxaborolan-2-yl)benzaldehyde (230 mg, crude) as a yellow oil. ¹H NMR (400 MHz, DMSO-*d*₆): δ 10.37 (s, 1H), 7.92 (d, 1H), 7.75-7.73 (m, 1H), 7.68-7.32 (m, 2H), 1.34 (s, 12H).

### (c) 4-(3-Chloro-4-(2-chloro-3-(6-methoxy-5-((7-oxo-2,6-diazaspiro[3.4]octan-2-yl)methyl)pyridin-2-yl)phenyl)pyridin-2-yl)-2-(difluoromethoxy)benzaldehyde

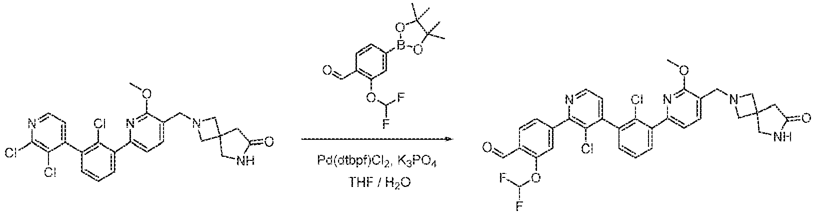

To a mixture of 2-((6-(2-chloro-3-(2,3-dichloropyridin-4-yl)phenyl)-2-methoxypyridin-3-yl)methyl)-2,6-diazaspiro[3.4]octan-7-one (200 mg, 0.397 mmol) and 2-(difluoromethoxy)-4-(4,4,5,5-tetramethyl-1,3,2-dioxaborolan-2-yl)benzaldehyde (177 mg, 0.59 mmol) in THF/ water (5:1, 6 mL) was added potassium phosphate (252 mg, 1.19 mmol) and [1,1'-bis(di-tert-butylphosphino)ferrocene]dichloropalladium(II) (25.8 mg, 0.04 mmol) and the mixture stirred at 80 °C for 2 h under N₂. Water (10 mL) was added, and the mixture extracted with ethyl acetate (10 mL x 2). The combined organic phases were dried over anhydrous sulfate, filtered and concentrated under reduced pressure. The residue was purified by normal phase SiO₂ chromatography (0-60% ethyl acetate/petroleum ether) to afford 4-(3-chloro-4-(2-chloro-3-(6-methoxy-5-((7-oxo-2,6-diazaspiro[3.4]octan-2-yl)methyl)pyridin-2-yl)phenyl)pyridin-2-yl)-2-(difluoromethoxy)benzaldehyde (130 mg, 24% yield) as a white solid. MS: *m*/*z* found 639 [M+H]⁺.

### (d) 2-((6-(3-(2-(4-((6-Acetyl-2,6-diazaspiro[3.3]heptan-2-yl)methyl)-3-(difluoromethoxy)phenyl)-3-chloropyridin-4-yl)-2-chlorophenyl)-2-methoxypyridin-3-yl)methyl)-2,6-diazaspiro[3.4]octan-7-one

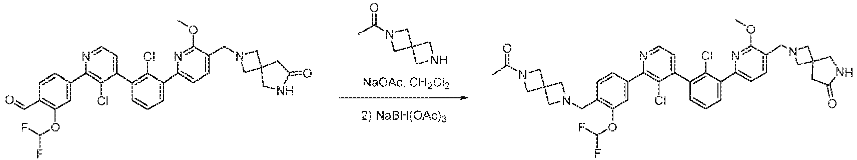

To a mixture of 4-(3-chloro-4-(2-chloro-3-(6-methoxy-5-((7-oxo-2,6-diazaspiro[3.4]octan-2-yl)methyl)pyridin-2-yl)phenyl)pyridin-2-yl)-2-(difluoromethoxy)benzaldehyde (200 mg, 0.31 mmol) and 1-(2,6-diazaspiro[3.3]heptan-2-yl)ethan-1-one hydrochloride (82.9 mg, 0.47 mmol) in dichloromethane (10 mL) was added sodium acetate (77 mg, 0.94 mmol) and the mixture was stirred at room temperature for 1.5 h under N₂. Sodium triacetoxyborohydride (199 mg, 0.94 mmol) was then added and the mixture stirred at room temperature for 0.5 h under N₂. The mixture was then concentrated and the residue purified by reverse phase HPLC to afford 2-((6-(3-(2-(4-((6-acetyl-2,6-diazaspiro[3.3]heptan-2-yl)methyl)-3-(difluoromethoxy)phenyl)-3-chloropyridin-4-yl)-2-chlorophenyl)-2-methoxypyridin-3-yl)methyl)-2,6-diazaspiro[3.4]octan-7-one (15.1 mg, 6% yield) as a white solid (formic acid salt). MS: *m*/*z* found 763 [M+H]⁺. ¹H NMR (400 MHz, Methanol-*d*₆): δ 8.67 (d, 1H), 8.32 (brs, 2H), 7.81 (d, 1H), 7.73 (dd, 1H), 7.62-7.56 (m, 4H), 7.48-7.44 (m, 2H), 7.32 (d, 1H), 6.99 (t, 1H), 4.34 (s, 2H), 4.10-4.08 (m, 4H), 4.06 (s, 3H), 3.99 (s, 2H), 3.91-3.90 (m, 4H), 3.77-3.75 (m, 4H), 3.65 (s, 2H), 2.67 (s, 2H), 1.86 (s, 3H).

### Example 530: 1-(6-(4-(3-Chloro-4-(2-chloro-3-(6-methoxy-5-(((tetrahydro-2H-pyran-4-yl)amino)methyl)pyridin-2-yl)phenyl)pyridin-2-yl)-2-(difluoromethoxy)benzyl)-2,6-diazaspiro[3.3]heptan-2-yl)ethan-1-one (663)

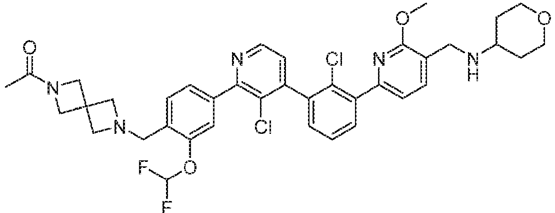

### (a) N-((6-(2-Chloro-3-(2,3-dichloropyridin-4-yl)phenyl)-2-methoxypyridin-3-yl)methyl)tetrahydro-2H-pyran-4-amine

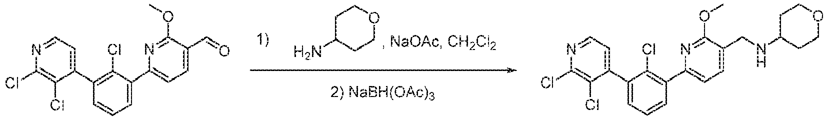

A mixture of 6-(2-chloro-3-(2,3-dichloropyridin-4-yl)phenyl)-2-methoxynicotinaldehyde (Example 1, step (a)) (600 mg, 1.52 mmol) and tetrahydro-2H-pyran-4-amine (308 mg, 3.05 mmol) in dichloromethane (10 mL) was added sodium acetate (375 mg, 4.57 mmol) and the mixture stirred at room temperature for 2.5 h under N₂. Sodium triacetoxyborohydride (969 mg, 4.57 mmol) was then added and the mixture stirred at room temperature for 0.5 h under N₂ to afford N-((6-(2-chloro-3-(2,3-dichloropyridin-4-yl)phenyl)-2-methoxypyridin-3-yl)methyl)tetrahydro-2H-pyran-4-amine (730 mg, crude), MS: *m*/*z* found 478 [M+H]⁺. The crude mixture was used in the next step without further purification.

### (b) tert-Butyl ((6-(2-chloro-3-(2,3-dichloropyridin-4-yl)phenyl)-2-methoxypyridin-3-yl)methyl)(tetrahydro-2H-pyran-4-yl)carbamate

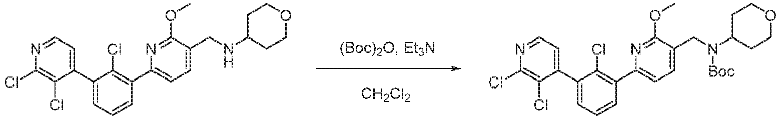

To the solution of N-((6-(2-chloro-3-(2,3-dichloropyridin-4-yl)phenyl)-2-methoxypyridin-3-yl)methyl)tetrahydro-2H-pyran-4-amine in dichloromethane (10 mL) was added di-tert-butyl dicarbonate (665 mg, 3.05 mmol) followed by triethylamine (463 mg, 4.57 mmol) and the mixture stirred at room temperature for 5 h under N₂. The mixture was then filtered and concentrated and the residue purified by normal phase SiO₂ chromatography (0-40 % ethyl acetate/petroleum ether) to afford tert-butyl ((6-(2-chloro-3-(2,3-dichloropyridin-4-yl)phenyl)-2-methoxypyridin-3-yl)methyl)(tetrahydro-2H-pyran-4-yl)carbamate (700 mg, 79% yield) as a white solid. ¹H NMR (400 MHz, DMSO-*d*₆): δ 8.52 (d, 1H), 7.76 (d, 1H), 7.74-7.58 (m, 3H), 7.49 (d, 1H), 7.29 (d, 1H), 4.32 (s, 2H), 3.96 (s, 3H), 3.89-3.86 (m, 3H), 3.82-3.79 (m, 2H), 1.78-1.76 (m, 2H), 1.69-1.66 (m, 2H), 1.39-1.35 (m, 9H).

### (c) tert-Butyl ((6-(2-chloro-3-(3-chloro-2-(3-(difluoromethoxy)-4-formylphenyl)pyridin-4-yl)phenyl)-2-methoxypyridin-3-yl)methyl)(tetrahydro-2H-pyran-4-yl)carbamate

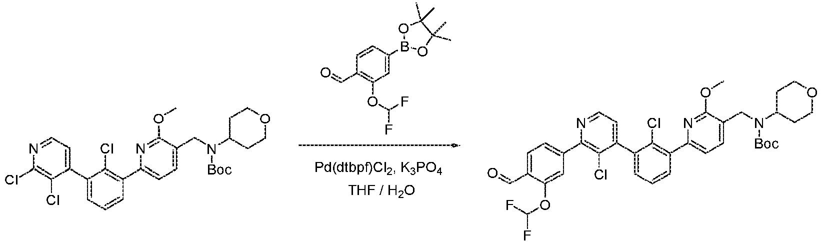

To a mixture of tert-butyl ((6-(2-chloro-3-(2,3-dichloropyridin-4-yl)phenyl)-2-methoxypyridin-3-yl)methyl)(tetrahydro-2H-pyran-4-yl)carbamate (600 mg, 1.04 mmol) and 2-(difluoromethoxy)-4-(4,4,5,5-tetramethyl-1,3,2-dioxaborolan-2-yl)benzaldehyde (Example 529, step (b)) (463 mg, 1.55 mmol) in THF / water (5:1, 12 mL) was added potassium phosphate (660 mg, 3.11 mmol) and [1,1'-bis(di-tert-butylphosphino)ferrocene]dichloropalladium(II) (67.5 mg, 0.10 mmol) and the mixture stirred at 80 °C for 1 h under N₂. Water (20 mL) was added and the mixture extracted with ethyl acetate (2 x 30 mL). The combined organic phases were dried over anhydrous sulfate, filtered and concentrated under reduced pressure. The residue was purified by normal phase SiO₂ chromatography (0-50 % ethyl acetate/petroleum ether) to afford tert-butyl ((6-(2-chloro-3-(3-chloro-2-(3-(difluoromethoxy)-4-formylphenyl)pyridin-4-yl)phenyl)-2-methoxypyridin-3-yl)methyl)(tetrahydro-2H-pyran-4-yl)carbamate (600 mg, 81% yield) as a white solid. ¹H NMR (400 MHz, DMSO-*d*₆): δ 10.30 (s, 1H), 8.75 (d, 1H), 7.95 (d, 1H), 7.76-7.74 (m, 1H), 7.70 (d, 1H), 7.66 (s, 1H), 7.61-7.43 (m, 5H), 7.25 (d, 1H), 4.27 (s, 2H), 3.91 (s, 3H), 3.84-3.58 (m, 2H), 3.57-3.55 (m, 4H), 1.74-1.70 (m, 4H), 1.32-1.22 (m, 9H).

### (d) tert-Butyl ((6-(3-(2-(4-((6-acetyl-2,6-diazaspiro[3.3]heptan-2-yl)methyl)-3-(difluoromethoxy)phenyl)-3-chloropyridin-4-yl)-2-chlorophenyl)-2-methoxypyridin-3-yl)methyl)(tetrahydro-2H-pyran-4-yl)carbamate

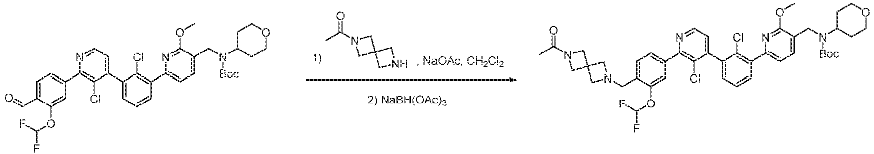

To a mixture of tert-butyl ((6-(2-chloro-3-(3-chloro-2-(3-(difluoromethoxy)-4-formylphenyl)pyridin-4-yl)phenyl)-2-methoxypyridin-3-yl)methyl)(tetrahydro-2H-pyran-4-yl)carbamate (200 mg, 0.28 mmol) and 1-(2,6-diazaspiro[3.3]heptan-2-yl)ethan-1-one hydrochloride (74.2 mg, 0.42 mmol) in dichloromethane (10 mL) was added sodium acetate (68.9 mg, 0.84 mmol) and the mixture stirred at room temperature for 1.5 h under N₂. Sodium triacetoxyborohydride (178 mg, 0.84 mmol) was then added and the mixture stirred at room temperature for 1.5 h under N₂. The mixture was filtered, the filtrate concentrated and the residue purified by preparative-Thin Layer Chromatography (SiO₂, EtOAc: MeOH = 5:1) to afford tert-butyl ((6-(3-(2-(4-((6-acetyl-2,6-diazaspiro[3.3]heptan-2-yl)methyl)-3-(difluoromethoxy)phenyl)-3-chloropyridin-4-yl)-2-chlorophenyl)-2-methoxypyridin-3-yl)methyl)(tetrahydro-2H-pyran-4-yl)carbamate (95 mg, 40% yield) as a white solid. MS: *m*/*z* found 838 [M+H]⁺.

### (e) 1-(6-(4-(3-Chloro-4-(2-chloro-3-(6-methoxy-5-(((tetrahydro-2H-pyran-4-yl)amino)methyl)pyridin-2-yl)phenyl)pyridin-2-yl)-2-(difluoromethoxy)benzyl)-2,6-diazaspiro[3.3]heptan-2-yl)ethan-1-one

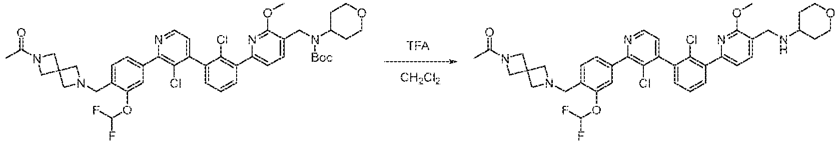

To a solution of tert-butyl ((6-(3-(2-(4-((6-acetyl-2,6-diazaspiro[3.3]heptan-2-yl)methyl)-3-(difluoromethoxy)phenyl)-3-chloropyridin-4-yl)-2-chlorophenyl)-2-methoxypyridin-3-yl)methyl)(tetrahydro-2H-pyran-4-yl)carbamate (90 mg, 0.11 mmol) in dichloromethane (5 mL) was added trifluoroacetic acid (3 mL, 40.5 mmol) and the mixture stirred at room temperature for 20 min under N₂. The mixture was concentrated and the residue purified by reverse phase HPLC to afford 1-(6-(4-(3-chloro-4-(2-chloro-3-(6-methoxy-5-(((tetrahydro-2H-pyran-4-yl)amino)methyl)pyridin-2-yl)phenyl)pyridin-2-yl)-2-(difluoromethoxy)benzyl)-2,6-diazaspiro[3.3]heptan-2-yl)ethan-1-one (29.3 mg, 35% yield) as a white solid (formic acid salt). MS: *m*/*z* found 738 [M+H]⁺. ¹H NMR (400 MHz, Methanol-*d*₆): δ 8.59 (d, 1H), 7.80 (d, 1H), 7.64-7.57 (m, 4H), 7.49 (t, 1H), 7.40-7.35 (m, 2H), 7.26 (d, 1H), 7.01 (t, 1H), 4.45 (s, 2H), 4.40-4.32 (m, 6H), 4.21 (s, 2H), 4.07 (brs, 2H), 3.99-3.95 (m, 5H), 3.41-3.38 (m, 3H), 2.05-2.02 (m, 2H), 1.78 (s, 3H), 1.75-1.59 (m, 2H).

### Example 531: 1-(4-(((6-(3-(2-(4-((6-Acetyl-2,6-diazaspiro[3.3]heptan-2-yl)methyl)-3-(difluoromethoxy)phenyl)-3-chloropyridin-4-yl)-2-chlorophenyl)-2-methoxypyridin-3-yl)methyl)amino)piperidin-1-yl)ethan-1-one (664)

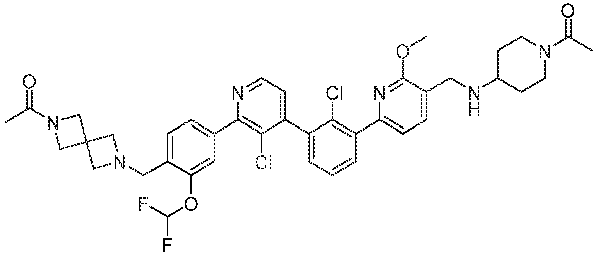

1-(4-(((6-(3-(2-(4-((6-Acetyl-2,6-diazaspiro[3.3]heptan-2-yl)methyl)-3-(difluoromethoxy)phenyl)-3-chloropyridin-4-yl)-2-chlorophenyl)-2-methoxypyridin-3-yl)methyl)amino)piperidin-1-yl)ethan-1-one was prepared in a similar manner as described for Example 530, replacing tetrahydro-2H-pyran-4-amine with 1-(4-aminopiperidin-1-yl)ethan-1-one in step (a). MS: *m*/*z* found 779 [M+H]⁺. ¹H NMR (400 MHz, Methanol-*d*₆): δ 8.71 (d, 1H), 7.93 (d, 1H), 7.75-7.69 (m, 4H), 7.61 (t, 1H), 7.51 (d, 1H), 7.47 (dd, 1H), 7.39 (d, 1H), 7.12 (t, 1H), 4.56 (s, 2H), 4.43-4.35 (m, 8H), 4.18-4.12 (m, 6H), 3.54-3.50 (m, 1H), 3.27-3.15 (m, 1H), 2.75-2.70 (m, 1H), 2.32-2.24 (m, 2H), 2.16 (s, 3H), 1.87 (s, 3H), 1.71-1.55 (m, 2H), 1.43-1.30 (m, 1H).

### Example 532: 2-((6-(3-(2-(4-(((1-Acetylpiperidin-4-yl)amino)methyl)-3-(difluoromethoxy)phenyl)-3-chloropyridin-4-yl)-2-chlorophenyl)-2-methoxypyridin-3-yl)methyl)-2,6-diazaspiro[3.4]octan-7-one (665)

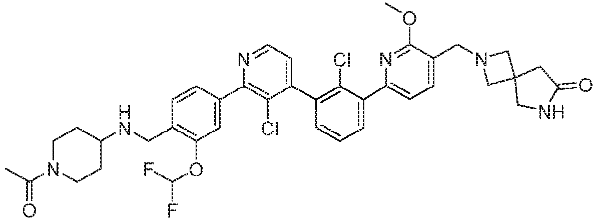

2-((6-(3-(2-(4-(((1-Acetylpiperidin-4-yl)amino)methyl)-3-(difluoromethoxy)phenyl)-3-chloropyridin-4-yl)-2-chlorophenyl)-2-methoxypyridin-3-yl)methyl)-2,6-diazaspiro[3.4]octan-7-one was prepared in a similar manner as described for Example 529, replacing 1-(2,6-diazaspiro[3.3]heptan-2-yl)ethan-1-one hydrochloride with 1-(4-aminopiperidin-1-yl)ethan-1-one in step (d). MS: *m*/*z* found 765 [M+H]⁺. ¹H NMR (400 MHz, Methanol-*d*₆): δ 8.67 (d, 1H), 8.52 (brs), 7.72 (dd, 2H), 7.65-7.64 (m, 2H), 7.58-7.54 (m, 2H), 7.48 (d, 1H), 7.43 (dd, 1H), 7.27 (d, 1H), 7.02 (t, 1H), 4.60-4.51 (m, 1H), 4.09-3.97 (m, 6H), 3.78 (s, 2H), 3.61 (s, 2H), 3.50-3.40 (m, 4H), 3.22-3.15 (m, 1H), 2.78-2.71 (m, 1H), 2.61 (s, 2H), 2.13-2.06 (m, 5H), 1.48-1.31 (m, 3H).

### Example 533: 2-(4-(3-Chloro-4-(2-chloro-3-(6-methoxy-5-(((tetrahydro-2H-pyran-4-yl)amino)methyl)pyridin-2-yl)phenyl)pyridin-2-yl)-2-(difluoromethoxy)benzyl)-2,6-diazaspiro[3.4]octan-7-one (683)

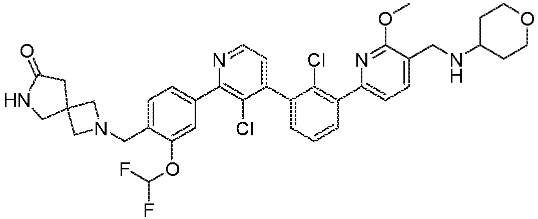

2-(4-(3-Chloro-4-(2-chloro-3-(6-methoxy-5-(((tetrahydro-2H-pyran-4-yl)amino)methyl)pyridin-2-yl)phenyl)pyridin-2-yl)-2-(difluoromethoxy)benzyl)-2,6-diazaspiro[3.4]octan-7-one (formic acid salt) was prepared in a similar manner as described for Example 530, replacing 1-(2,6-diazaspiro[3.3]heptan-2-yl)ethan-1-one hydrochloride with 2,6-diazaspiro[3.4]octan-7-one trifluoroacetate in step (d). MS: *m*/*z* found 724 [M+H]⁺. ¹H NMR (400 MHz, Methanol-*d*₄): δ 8.68 (d, 1H), 8.35 (brs, 1H), 7.91 (d, 1H), 7.74 (dd, 1H), 7.64-7.55 (m, 4H), 7.48-7.46 (m, 2H), 7.38 (d, 1H), 6.99 (t, 1H), 4.31 (s, 2H), 4.11 (s, 3H), 4.09-4.06 (m, 2H), 4.00 (s, 2H), 3.66 (s, 4H), 3.63 (s, 2H), 3.52-3.47 (m, 3H), 2.64 (s, 2H), 2.16-2.13 (m, 2H), 1.80-1.73 (m, 2H).

### Example 534: 2-(4-(4-(3-(5-(((1-Acetylpiperidin-4-yl)amino)methyl)-6-methoxypyridin-2-yl)-2-chlorophenyl)-3-chloropyridin-2-yl)-2-(difluoromethoxy)benzyl)-2,6-diazaspiro[3.4]octan-7-one (684)

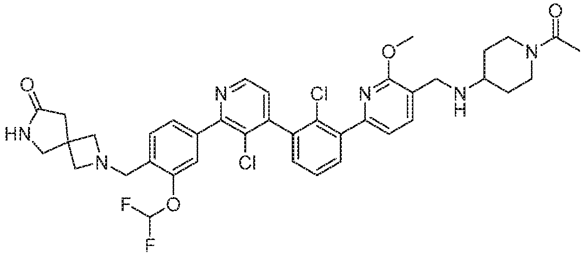

2-(4-(4-(3-(5-(((1-Acetylpiperidin-4-yl)amino)methyl)-6-methoxypyridin-2-yl)-2-chlorophenyl)-3-chloropyridin-2-yl)-2-(difluoromethoxy)benzyl)-2,6-diazaspiro[3.4]octan-7-one (formic acid salt) was prepared in a similar manner as described for Example 530, replacing tetrahydro-2H-pyran-4-amine with 1-(4-aminopiperidin-1-yl)ethan-1-one in step (a) and 1-(2,6-diazaspiro[3.3]heptan-2-yl)ethan-1-one hydrochloride with 2,6-diazaspiro[3.4]octan-7-one in step (d). MS: *m*/*z* found 765 [M+H]⁺. ¹H NMR (400 MHz, Methanol-*d*₄): δ 8.68 (d, 1H), 8.24 (brs, 1H), 7.91 (d, 1H), 7.74 (dd, 1H), 7.65-7.57 (m, 4H), 7.48-7.46 (m, 2H), 7.38 (d, 1H), 7.00 (t, 1H), 4.72-4.71 (m, 2H), 4.35 (s, 2H), 4.11 (s, 3H), 4.07 (s, 2H), 3.69 (s, 4H), 3.64 (s, 2H), 3.33-3.24 (m, 2H), 2.76-2.73 (m, 3H), 2.31-2.26 (m, 2H), 2.23 (s, 3H), 1.57-1.52 (m, 2H).

### Example 535: 1-(4-(((5-(3-(2-(4-(((1-Acetylpiperidin-4-yl)amino)methyl)-3-methoxyphenyl)-3-chloropyridin-4-yl)-2-chlorophenyl)-3-methoxypyrazin-2-yl)methyl)amino)piperidin-1-yl)ethan-1-one (311)

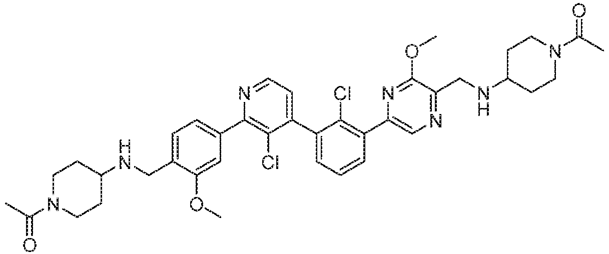

1-(4-(((5-(3-(2-(4-(((1-Acetylpiperidin-4-yl)amino)methyl)-3-methoxyphenyl)-3-chloropyridin-4-yl)-2-chlorophenyl)-3-methoxypyrazin-2-yl)methyl)amino)piperidin-1-yl)ethan-1-one was prepared following Reductive Amination Procedure A from 5-(2-chloro-3-(3-chloro-2-(4-formyl-3-methoxyphenyl)pyridin-4-yl)phenyl)-3-methoxypyrazine-2-carbaldehyde (15 mg, 0.03 mmol) and 1-(4-amino-1-piperidyl)ethanone (17 mg, 0.12 mmol). Formic acid salt, 65% yield. LCMS: m/z found 746.3 [M+H]⁺, retention time = 1.63 min (Method D). ¹H NMR (400 MHz, Methanol-*d*₄): δ 8.68 (d, 1H), 8.50 (s, 2H), 8.42 (s, 2H), 7.78 (d, 1H), 7.63 (t, 1H), 7.57 - 7.47 (m, 3H), 7.40 (s, 1H), 7.36 (d, 1H), 4.65 (t, 2H), 4.38 (s, 2H), 4.32 (s, 2H), 4.14 - 4.01 (m, 5H), 4.00 (s, 3H), 3.49 - 3.33 (m, 2H), 3.20 (t, 2H), 2.70 (t, 2H), 2.31 - 2.15 (m, 4H), 2.15 - 2.09 (m, 6H), 1.72 - 1.43 (m, 4H).

### Example 536: 1-(4-(((5-(3-(2-(4-(((1-acetylpiperidin-4-yl)amino)methyl)-3-methoxyphenyl)-3-chloropyridin-4-yl)-2-chlorophenyl)-3-methoxypyridin-2-yl)methyl)amino)piperidin-1-yl)ethan-1-one (565)

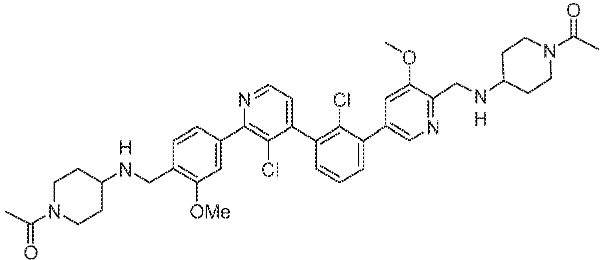

### (a) 5-(2-Chloro-3-(2,3-dichloropyridin-4-yl)phenyl)-3-methoxypicolinaldehyde

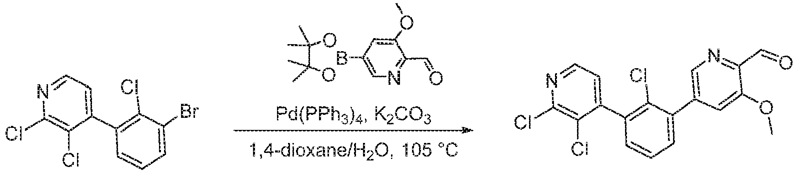

3-Methoxy-5-(4,4,5,5-tetramethyl-1,3,2-dioxaborolan-2-yl)picolinaldehyde (187 mg, 0.71 mmol), tetrakis(triphenylphosphine)palladium(0) (137 mg, 0.12 mmol), potassium carbonate (245 mg, 1.78 mmol), and 4-(3-bromo-2-chloro-phenyl)-2,3-dichloro-pyridine (200 mg, 0.59 mmol) were suspended in 1,4-dioxane/water (4:1, 5 mL), then the solution was heated at 105 °C for 20 minutes. Reaction was cooled to room temperature, diluted with 10 mL water, and extracted with EtOAc (3 x 10 mL). The combined organics were concentrated under reduced pressure and the crude sample was purified by silica gel chromatography (5-100% EtOAc/hexane) to afford 5-(2-chloro-3-(2,3-dichloropyridin-4-yl)phenyl)-3-methoxypicolinaldehyde (50 mg, 21 % yield) as a yellow oil. MS: *m*/*z* found 393, 395 [M+H]⁺.

### (b) 5-(2-Chloro-3-(3-chloro-2-(4-formyl-3-methoxyphenyl)pyridin-4-yl)phenyl)-3-methoxypicolinaldehyde

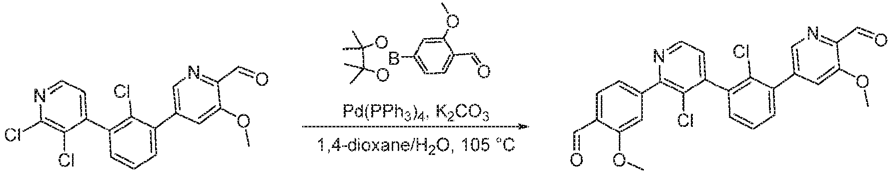

Tetrakis(triphenylphosphine)palladium(0) (48 mg, 0.04 mmol), potassium carbonate (85 mg, 0.62 mmol), 5-(2-chloro-3-(2,3-dichloropyridin-4-yl)phenyl)-3-methoxypicolinaldehyde (81 mg, 0.21 mmol), and 2-methoxy-4-(4,4,5,5-tetramethyl-1,3,2-dioxaborolan-2-yl)benzaldehyde (70 mg, 0.27 mmol) were suspended in 1,4-dioxane/water (4:1, 3 mL), then the solution was heated at 105 °C for 20 minutes. Reaction was cooled to room temperature, diluted with 5 mL water, and extracted with EtOAc (3 x 5 mL). The combined organics were concentrated under reduced pressure and the crude sample was purified by silica gel chromatography (10-80 % EtOAc/hexane) to afford 5-(2-chloro-3-(3-chloro-2-(4-formyl-3-methoxyphenyl)pyridin-4-yl)phenyl)-3-methoxypicolinaldehyde (23 mg, 23 % yield) as a yellow foam. MS: *m*/*z* found 493, 495 [M+H]⁺.

### (c) 1-(4-(((5-(3-(2-(4-(((1-acetylpiperidin-4-yl)amino)methyl)-3-methoxyphenyl)-3-chloropyridin-4-yl)-2-chlorophenyl)-3-methoxypyridin-2-yl)methyl)amino)piperidin-1-yl)ethan-1-one

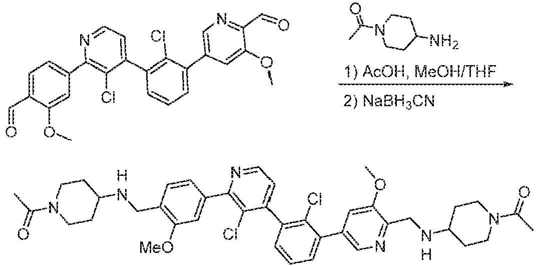

5-(2-chloro-3-(3-chloro-2-(4-formyl-3-methoxyphenyl)pyridin-4-yl)phenyl)-3-methoxypicolinaldehyde (12 mg, 0.02 mmol), acetic acid (3 mg, 0.05 mmol), and 1-(4-amino-1-piperidyl)ethanone (10 mg, 0.07 mmol) were dissolved in MeOH/THF (1:1, 1 mL), then the solution was stirred at 25 °C for 2 hours. Sodium cyanoborohydride (4 mg, 0.07 mmol) was added and the resulting mixture was stirred at 25 °C for 1 hour. Reaction was filtered through celite and the filtrate was concentrated under reduced pressure. Crude sample was purified by reverse phase HPLC to afford 1-(4-(((5-(3-(2-(4-(((1-acetylpiperidin-4-yl)amino)methyl)-3-methoxyphenyl)-3-chloropyridin-4-yl)-2-chlorophenyl)-3-methoxypyridin-2-yl)methyl)amino)piperidin-1-yl)ethan-1-one (8 mg, 48 % yield) as a white solid (formic acid salt). LC/MS: *m*/*z* found 745, 747 [M+H] ⁺, retention time = 2.08 min (Method C). ¹H NMR (400 MHz, Methanol-*d*₄) δ 8.66 (d, 1H), 8.47 (s, 2H), 8.27 (s, 1H), 7.59 (d, 3H), 7.52 (d, 1H), 7.46 (d, 2H), 7.38 (s, 1H), 7.34 (d, 1H), 4.62 (d, 2H), 4.36 (s, 2H), 4.27 (s, 2H), 4.05 (d, 2H), 3.98 (s, 6H), 3.35 (s, 2H), 3.19 (t, 2H), 2.69 (t, 2H), 2.21 (t, 4H), 2.13 (s, 6H), 1.56 (dd, 4H).

### Example 537: 2-(4-(3-Chloro-4-(2-chloro-3-(5-methoxy-6-((7-oxo-2,6-diazaspiro [3.4] octan-2-yl)methyl)pyridin-3-yl)phenyl)pyridin-2-yl)-2-methoxybenzyl)-2,6-diazaspiro[3.4]octan-7-one (566)

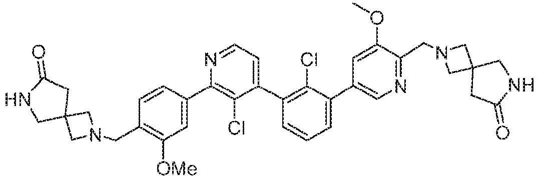

2-(4-(3-Chloro-4-(2-chloro-3-(5-methoxy-6-((7-oxo-2,6-diazaspiro[3.4]octan-2-yl)methyl)pyridin-3-yl)phenyl)pyridin-2-yl)-2-methoxybenzyl)-2,6-diazaspiro[3.4]octan-7-one was prepared in similar manner as described for Example 536, utilizing intermediate 5-(2-chloro-3-(3-chloro-2-(4-formyl-3-methoxyphenyl)pyridin-4-yl)phenyl)-3-methoxypicolinaldehyde and replacing 1-(4-amino-1-piperidyl)ethanone with 2,6-diazaspiro[3.4]octan-7-one in step (c). Product was obtained as a white solid (formic acid salt). LC/MS: *m*/*z* found 713, 715 [M+H]⁺, retention time = 1.99 min (Method C). ¹H NMR (400 MHz, Methanol-*d*₄) δ 8.65 (d, 1H), 8.43 (s, 2H), 8.23 (d, 1H), 7.58 (dd, 3H), 7.50 - 7.43 (m, 3H), 7.36 - 7.30 (m, 2H), 4.43 (s, 2H), 4.24 (s, 2H), 4.10 (s, 4H), 3.98 (s, 4H), 3.96 (d, 6H), 3.68 (s, 2H), 3.64 (d, 2H), 2.71 (d, 2H), 2.67 (d, 2H).

### Example 538: 1-(4-((4-(5-(((1-acetylpiperidin-4-yl)amino)methyl)-3',3"-dichloro-6-methoxy-[2,4':2',4"-terpyridin]-2"-yl)-2-methoxybenzyl)amino)piperidin-1-yl)ethan-1-one (668)

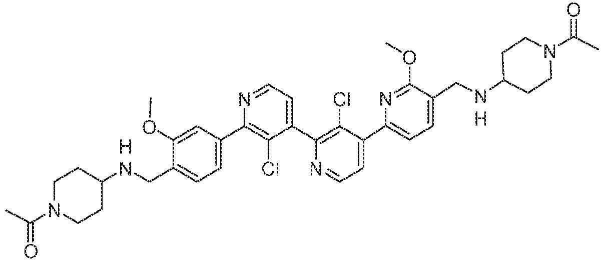

### (a) 2',3'-Dichloro-6-methoxy-[2,4'-bipyridine]-5-carbaldehyde

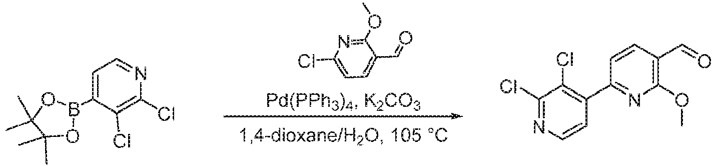

6-Chloro-2-methoxynicotinaldehyde (1.13 g, 6.57 mmol), tetrakis(triphenylphosphine)palladium(0) (0.38 g, 0.33 mmol), potassium carbonate (1.36 g, 9.86 mmol), and 2,3-dichloro-4-(4,4,5,5-tetramethyl-1,3,2-dioxaborolan-2-yl)pyridine (0.90 g, 3.29 mmol) were suspended in 1,4-dioxane/water (4:1, 20 mL), then the solution was heated at 105 °C for 20 minutes. Reaction was cooled to room temperature, diluted with 40 mL water, and extracted with dichloromethane (3 x 30 mL). The combined organics were dried over sodium sulfate and concentrated under reduced pressure. The crude solid was suspended in 4: 1 Hexane/EtOAc (50 mL) and sonicated, then filtered and further washed with hexane. The filter cake was dried to afford 2',3'-dichloro-6-methoxy-[2,4'-bipyridine]-5-carbaldehyde (0.50 g, 54 % yield) as a white solid. MS: *m*/*z* found 283, 285 [M+H]⁺.

### (b) 2",3',3"-Trichloro-6-methoxy-[2,4':2',4"-terpyridine]-5-carbaldehyde

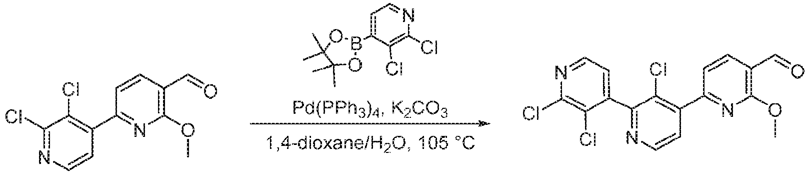

2',3'-dichloro-6-methoxy-[2,4'-bipyridine]-5-carbaldehyde (150 mg, 0.53 mmol), tetrakis(triphenylphosphine)palladium(0) (61 mg, 0.05 mmol), potassium carbonate (219 mg, 1.59 mmol), and 2,3-dichloro-4-(4,4,5,5-tetramethyl-1,3,2-dioxaborolan-2-yl)pyridine (145 mg, 0.53 mmol) were suspended in 1,4-dioxane/water (4:1, 6 mL), then the solution was heated at 105 °C for 20 minutes. Reaction was cooled to room temperature, diluted with 10 mL water, and extracted with dichloromethane (3 x 5 mL). The combined organics were concentrated under reduced pressure and the crude sample was purified by silica gel chromatography (0-50 % EtOAc/hexane) to afford 2",3',3"-trichloro-6-methoxy-[2,4':2',4"-terpyridine]-5-carbaldehyde (76 mg, 36 % yield) as a yellow oil. MS: *m*/*z* found 394, 396 [M+H] ⁺.

### (c) 3',3"-Dichloro-2"-(3-formyl-4-methoxyphenyl)-6-methoxy-[2,4':2',4''-terpyridine]-5-carbaldehyde

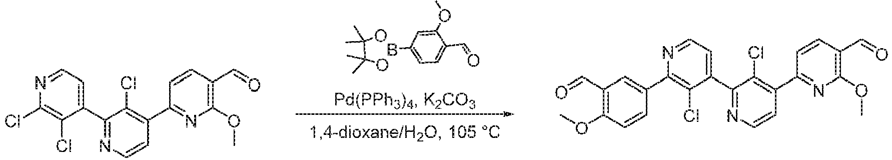

Tetrakis(triphenylphosphine)palladium(0) (44 mg, 0.04 mmol), potassium carbonate (79 mg, 0.57 mmol), 2",3',3"-trichloro-6-methoxy-[2,4':2',4"-terpyridine]-5-carbaldehyde (76 mg, 0.19 mmol), and 2-methoxy-4-(4,4,5,5-tetramethyl-1,3,2-dioxaborolan-2-yl)benzaldehyde (75 mg, 0.29 mmol) were suspended in 1,4-dioxane/water (4:1, 3 mL), then the solution was heated at 105 °C for 20 minutes. Reaction was cooled to room temperature, diluted with 10 mL water, and extracted with EtOAc (3 x 5 mL). The combined organics were concentrated under reduced pressure and the crude sample was purified by silica gel chromatography (0-70% EtOAc/hexane) to afford 3',3"-dichloro-2"-(3-formyl-4-methoxyphenyl)-6-methoxy-[2,4':2',4"-terpyridine]-5-carbaldehyde (86 mg, 91 % yield) as a tan solid. MS: *m*/*z* found 494, 496 [M+H]⁺.

### (d) 1-(4-((4-(5-(((1-Acetylpiperidin-4-yl)amino)methyl)-3',3"-dichloro-6-methoxy-[2,4':2',4"-terpyridin]-2"-yl)-2-methoxybenzyl)amino)piperidin-1-yl)ethan-1-one

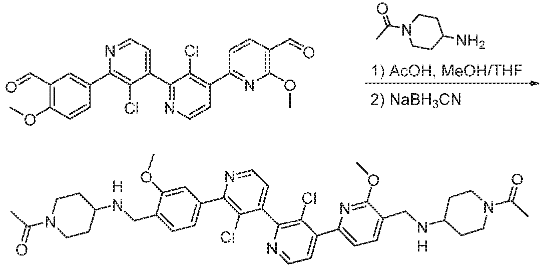

3',3"-Dichloro-2"-(3-formyl-4-methoxyphenyl)-6-methoxy-[2,4':2',4"-terpyridine]-5-carbaldehyde (20 mg, 0.04 mmol), acetic acid (5 mg, 0.08 mmol), and 1-(4-amino-1-piperidyl)ethanone (17 mg, 0.12 mmol) were dissolved in MeOH/THF (1:1, 1 mL), then the solution was stirred at 25 °C for 2 hours. Sodium cyanoborohydride (8 mg, 0.12 mmol) was added and the resulting mixture was stirred at 25 °C for 1 hour. Reaction was filtered through celite, and the filtrate was concentrated under reduced pressure. Crude sample was purified by reverse phase HPLC to afford 1-(4-((4-(5-(((1-acetylpiperidin-4-yl)amino)methyl)-3',3"-dichloro-6-methoxy-[2,4':2',4"-terpyridin]-2"-yl)-2-methoxybenzyl)amino)piperidin-1-yl)ethan-1-one (15 mg, 49 % yield) as a white solid (formic acid salt). LC/MS: *m*/*z* found 746, 748 [M+H]⁺, retention time = 1.95 min (Method C). ¹H NMR (400 MHz, Methanol-*d*₄) δ 8.75 - 8.70 (m, 2H), 8.46 (s, 2H), 7.91 (d, 1H), 7.82 (d, 1H), 7.55 (dd, 2H), 7.48 (d, 1H), 7.41 (s, 1H), 7.36 (d, 1H), 4.62 (dd, 2H), 4.30 (s, 2H), 4.12 (s, 2H), 4.07 (s, 4H), 3.99 (s, 4H), 3.19 (d, 4H), 2.71 (d, 2H), 2.28 - 2.09 (m, 10H), 1.52 (m, 4H).

### Example 539: 2-(4-(3',3"-Dichloro-6-methoxy-5-((7-oxo-2,6-diazaspiro[3.4]octan-2-yl)methyl)-[2,4':2',4"-terpyridin]-2"-yl)-2-methoxybenzyl)-2,6-diazaspiro[3.4]octan-7-one (669)

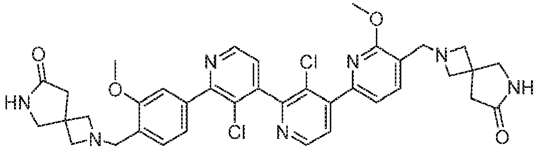

2-(4-(3',3"-Dichloro-6-methoxy-5-((7-oxo-2,6-diazaspiro[3.4]octan-2-yl)methyl)-[2,4':2',4"-terpyridin]-2"-yl)-2-methoxybenzyl)-2,6-diazaspiro[3.4]octan-7-one was prepared in similar manner as described for Example 538, utilizing intermediate 3',3"-dichloro-2"-(3-formyl-4-methoxyphenyl)-6-methoxy-[2,4':2',4"-terpyridine]-5-carbaldehyde and replacing 1-(4-amino-1-piperidyl)ethanone with 2,6-diazaspiro[3.4]octan-7-one in step (d). Product was obtained as a white solid (formic acid salt). LC/MS: *m*/*z* found 714, 716 [M+H] ⁺, retention time = 1.85 min (Method C). ¹H NMR (400 MHz, Methanol-*d*₄) δ 8.71 (dd, 2H), 8.35 (s, 2H), 7.87 - 7.79 (m, 2H), 7.56 (d, 1H), 7.52 (d, 1H), 7.45 (d, 1H), 7.40 (s, 1H), 7.36 (d, 1H), 4.40 (s, 2H), 4.17 (s, 4H), 4.07 - 4.00 (m, 5H), 3.98 (d, 3H), 3.77 (s, 4H), 3.67 (d, 2H), 3.63 (s, 2H), 2.71 (s, 2H), 2.64 (d, 2H).

### Example 540: (S)-5-((((6-(2-Chloro-3-(3-chloro-2-(5-methyl-2,3,4,5-tetrahydro-1H-pyrido[4,3-b]indol-7-yl)pyridin-4-yl)phenyl)-2-methoxypyridin-3-yl)methyl)amino)methyl)pyrrolidin-2-one (141)

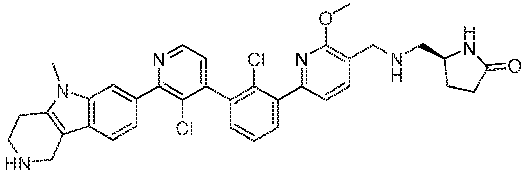

### (a) tert-Butyl 7-bromo-5-methyl-1,3,4,5-tetrahydro-2H-pyrido[4,3-b]indole-2-carboxylate

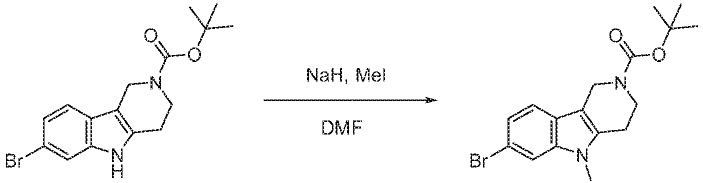

A solution of tert-butyl 7-bromo-1,3,4,5-tetrahydro-2H-pyrido[4,3-b]indole-2-carboxylate (242 mg, 0.69 mmol) in 3 mL anhydrous DMF under nitrogen was cooled in an ice bath. Sodium hydride, 60% in mineral oil (41 mg, 1.03 mmol) was added in 2 portions. The ice bath was removed, and the mixture allowed to stir at room temperature for 1 h. Iodomethane (64 µL, 1.03 mmol) was added, and the mixture was allowed to stir at room temperature for 2 h. The reaction mixture was cooled in an ice bath and ice water (15 mL) was slowly added. The resulting precipitate was collected, washed with 2 mL water, and dried *in vacuo* to provide tert-butyl 7-bromo-5-methyl-3,4-dihydro-1H-pyrido[4,3-b]indole-2-carboxylate (162 mg, 57%). ¹H NMR (400 MHz, DMSO-*d*₆) δ 7.68 (d, 1H), 7.39 (d, 1H), 7.12 (dd, 1H), 4.51 (s, 2H), 3.71 (t, 2H), 3.62 (s, 3H), 2.78 (t, 2H), 1.43 (s, 9H).

### (b) tert-Butyl 5-methyl-7-(4,4,5,5-tetramethyl-1,3,2-dioxaborolan-2-yl)-1,3,4,5-tetrahydro-2H-pyrido[4,3-b]indole-2-carboxylate

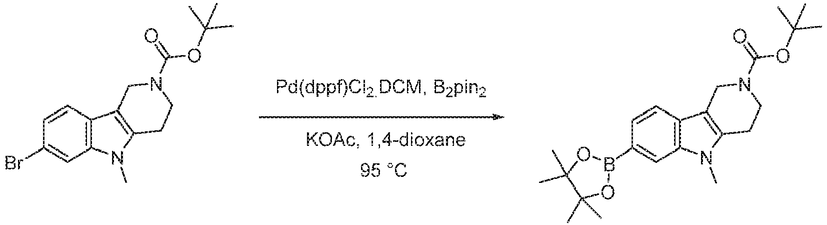

A mixture of tert-butyl 7-bromo-5-methyl-3,4-dihydro-1H-pyrido[4,3-b]indole-2-carboxylate (50 mg, 0.14 mmol), bis(pinacolato)diborane (42 mg, 0.16 mmol), potassium acetate (38 mg, 0.38 mmol), and [1,1'-bis(diphenylphosphino)ferrocene]dichloropalladium(II), complex with dichloromethane (9 mg, 0.01 mmol) were weighed out into a Biotage microwave vial. Anhydrous 1,4-dioxane (1 mL) was added, and the mixture bubbled with nitrogen for 2 minutes. The vessel was capped and placed into a 95 °C heating block for 2 h. The mixture was diluted with 10 mL EtOAc and filtered through a pad of Celite. The Celite pad was washed with 20 mL EtOAc. The combined filtrate was concentrated under reduced pressure and the crude material purified by normal phase silica gel chromatography (0% to 6% MeOH/DCM) to provide tert-butyl 5-methyl-7-(4,4,5,5-tetramethyl-1,3,2-dioxaborolan-2-yl)-3,4-dihydro-1H-pyrido[4,3-b]indole-2-carboxylate (27 mg, 48%).

### (c) tert-Butyl (S)-7-(4-(3-(5-(((tert-butoxycarbonyl)((5-oxopyrrolidin-2-yl)methyl)amino)methyl)-6-methoxypyridin-2-yl)-2-chlorophenyl)-3-chloropyridin-2-yl)-5-methyl-1,3,4,5-tetrahydro-2H-pyrido[4,3-b]indole-2-carboxylate

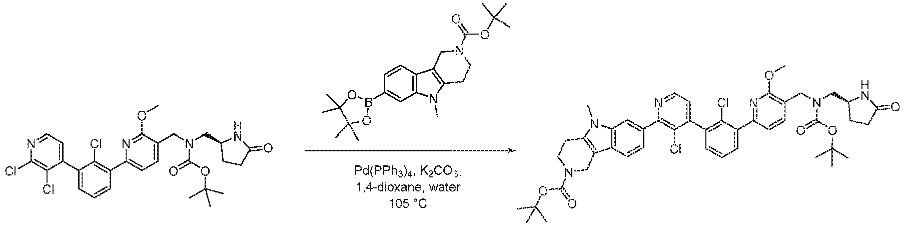

To a mixture tert-butyl (S)-((6-(2-chloro-3-(2,3-dichloropyridin-4-yl)phenyl)-2-methoxypyridin-3-yl)methyl)((5-oxopyrrolidin-2-yl)methyl)carbamate (38 mg, 0.06 mmol), tert-butyl 5-methyl-7-(4,4,5,5-tetramethyl-1,3,2-dioxaborolan-2-yl)-3,4-dihydro-1H-pyrido[4,3-b]indole-2-carboxylate (26 mg, 0.06 mmol) and potassium carbonate (27 mg, 0.19 mmol) in degassed 1,4-dioxane/water (6:1, 2.1 mL) was added tetrakis(triphenylphosphine)palladium(0) (7 mg, 0.01 mmol) and the mixture was irradiated to 105 °C for 25 min in a Biotage Initiator microwave. Water (8 mL) was added and the mixture was extracted into EtOAc (3 x 10 mL). The combined organic layers were dried over anhydrous sodium sulfate, concentrated, and purified by normal pahse silica gel chromatography (0% to 5% MeOH/DCM) to provide tert-butyl (S)-7-(4-(3-(5-(((tert-butoxycarbonyl)((5-oxopyrrolidin-2-yl)methyl)amino)methyl)-6-methoxypyridin-2-yl)-2-chlorophenyl)-3-chloropyridin-2-yl)-5-methyl-1,3,4,5-tetrahydro-2H-pyrido[4,3-b]indole-2-carboxylate (51 mg, 94%). MS: m/z found 841.0 [M+H]⁺.

### (d) (S)-5-((((6-(2-Chloro-3-(3-chloro-2-(5-methyl-2,3,4,5-tetrahydro-1H-pyrido[4,3-b]indol-7-yl)pyridin-4-yl)phenyl)-2-methoxypyridin-3-yl)methyl)amino)methyl)pyrrolidin-2-one

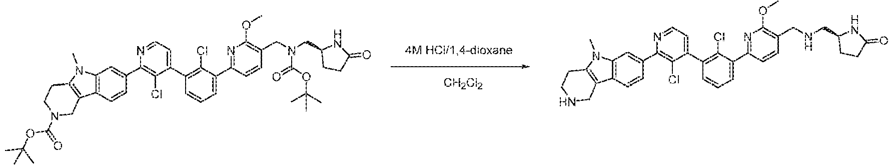

tert-Butyl (S)-7-(4-(3-(5-(((tert-butoxycarbonyl)((5-oxopyrrolidin-2-yl)methyl)amino)methyl)-6-methoxypyridin-2-yl)-2-chlorophenyl)-3-chloropyridin-2-yl)-5-methyl-1,3,4,5-tetrahydro-2H-pyrido[4,3-b]indole-2-carboxylate was converted to (S)-5-((((6-(2-chloro-3-(3-chloro-2-(5-methyl-2,3,4,5-tetrahydro-1H-pyrido[4,3-b]indol-7-yl)pyridin-4-yl)phenyl)-2-methoxypyridin-3-yl)methyl)amino)methyl)pyrrolidin-2-one formic acid salt following General Boc Deprotection Procedure. 27% yield. LCMS: m/z found 641.2 [M+H]⁺, retention time = 2.77 min, (Method A); ¹H NMR (400 MHz, Methanol-*d*₄)*:* δ 8.62 (d, 1H), 8.54 (s, 1H), 7.78 - 7.67 (m, 3H), 7.59 - 7.51 (m, 2H), 7.45 - 7.37 (m, 3H), 7.26 (d, 1H), 4.37 (s, 2H), 4.02 (d, 3H), 3.93 - 3.81 (m, 3H), 3.76 (s, 3H), 3.55 (t, 2H), 3.11 (d, 2H), 2.77 - 2.63 (m, 2H), 2.39 - 2.21 (m, 3H), 1.88 - 1.75 (m, 1H).

### Example 541: (S)-5-((((6-(2-Chloro-3-(3-chloro-2-(2-(2-hydroxyethyl)-5-methyl-2,3,4,5-tetrahydro-1H-pyrido[4,3-b]indol-7-yl)pyridin-4-yl)phenyl)-2-methoxypyridin-3-yl)methyl)amino)methyl)pyrrolidin-2-one (158)

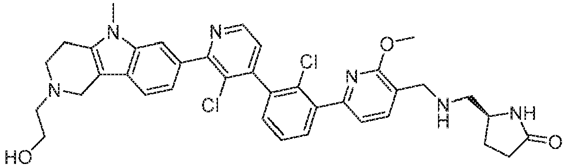

### (a) 7-Bromo-5-methyl-2,3,4,5-tetrahydro-1H-pyrido[4,3-b]indole

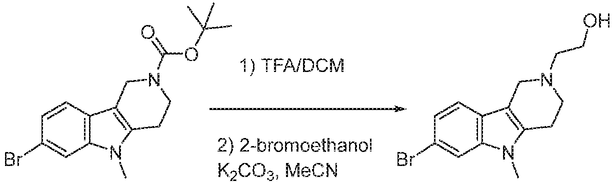

A solution of tert-butyl 7-bromo-5-methyl-1,3,4,5-tetrahydro-2H-pyrido[4,3-b]indole-2-carboxylate (112 mg, 0.31 mmol) in 1.5 mL dichloromethane/0.75 mL trifluoroacetic acid was stirred for 10 min. The volatiles were removed *in vacuo,* and the material was azeotroped with 2 mL toluene. The resulting material was dried under vacuum to provide 7-bromo-5-methyl-2,3,4,5-tetrahydro-1H-pyrido[4,3-b]indole as the trifluoroacetic acid salt (150 mg). ¹H NMR (400 MHz, DMSO-*d*₆) δ 9.04 (s, 2H), 7.76 (d, 1H), 7.46 (d, 1H), 7.19 (dd, 1H), 4.33 (d, 2H), 3.67 (s, 3H), 3.53 (m, 2H), 3.04 (t, 2H).

### (b) 2-(7-Bromo-5-methyl-1,3,4,5-tetrahydro-2H-pyrido[4,3-b]indol-2-yl)ethan-1-ol

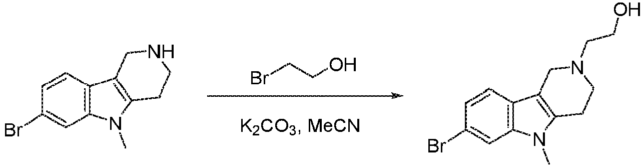

Potassium carbonate (54 mg, 0.39 mmol) was added to a solution of 7-bromo-5-methyl-2,3,4,5-tetrahydro-1H-pyrido[4,3-b]indole trifluoroacetic acid salt (74 mg, 0.20 mmol) in 1.5 mL acetonitrile. The mixture was stirred for 3 minutes. 2-Bromoethanol (42 µL, 0.59 mmol) was added, and the reaction vessel was capped. After stirring at room temperature for 16 h, the mixture was irradiated to 60 °C for 45 minutes in a Biotage Initiator microwave, followed by further irradiation for 30 min at 65 °C. The reaction mixture was diluted with 10 mL EtOAc, filtered through Celite, and evaporated to dryness. The crude material was purified by normal phase silica gel chromatography (0.5% to 10% MeOH/DCM) to provide 2-(7-bromo-5-methyl-1,3,4,5-tetrahydro-2H-pyrido[4,3-b]indol-2-yl)ethan-1-ol (53 mg, 88%). ¹H NMR (400 MHz, Methanol-*d*₄) δ 7.52 (d, 1H), 7.30 (d, 1H), 7.13 (dd, 1H), 3.94 (s, 2H), 3.85 (t, 2H), 3.64 (s, 3H), 3.17 (t, 2H), 2.96 (t, 4H).

### (c) 2-(5-Methyl-7-(4,4,5,5-tetramethyl-1,3,2-dioxaborolan-2-yl)-1,3,4,5-tetrahydro-2H-pyrido[4,3-b]indol-2-yl)ethan-1-ol

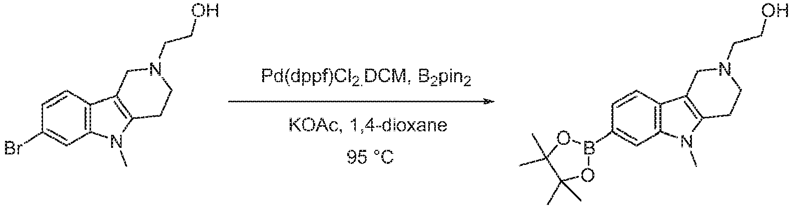

A mixture of 2-(7-bromo-5-methyl-1,3,4,5-tetrahydro-2H-pyrido[4,3-b]indol-2-yl)ethan-1-ol (48 mg, 0.15 mmol), bis(pinacolato)diborane (51 mg, 0.20 mmol), potassium acetate (42 mg, 0.43 mmol) and [1,1'-bis(diphenylphosphino)ferrocene]dichloropalladium(II), complex with dichloromethane (10 mg, 0.01 mmol) were weighed out into a Biotage micowave vial. Anhydrous 1,4-dioxane (1 mL) was added, and the mixture degassed with nitrogen for 2 minutes. The vessel was capped and irradiated to 95 °C in a Biotage Initiator microwave for 65 minutes. An additional portion of [1,1'- bis(diphenylphosphino)ferrocene]-dichloropalladium(II) complex with dichloromethane (2 mg) and bis(pinacolato)diborane (10 mg, 0.04 mmol) were added. The mixture was irradiated to 95 °C for 60 minutes. The mixture was diluted with 10 mL EtOAc and filtered through a pad of Celite. The Celite pad was washed with 20 mL EtOAc and 5 mL acetonitrile. The combined filtrates were concentrated under reduced pressure and the resulting crude material purified by normal phase silica gel chromatography (2% to 10% MeOH/DCM) to provide 2-(5-methyl-7-(4,4,5,5-tetramethyl-1,3,2-dioxaborolan-2-yl)-1,3,4,5-tetrahydro-2H-pyrido[4,3-b]indol-2-yl)ethan-1-ol (24 mg, 44 %). ¹H NMR (400 MHz, Chloroform-d) δ 7.79 (s, 1H), 7.56 (d, 1H), 7.39 (d, 1H), 4.27 (s, 2H), 3.95 (q, 2H), 3.69 (s, 3H), 3.43 (d, 2H), 3.12 (dt, 4H), 1.38 (s, 12H).

### (d) tert-Butyl (S)-((6-(2-chloro-3-(3-chloro-2-(2-(2-hydroxyethyl)-5-methyl-2,3,4,5-tetrahydro-1H-pyrido[4,3-b]indol-7-yl)pyridin-4-yl)phenyl)-2-methoxypyridin-3-yl)methyl)((5-oxopyrrolidin-2-yl)methyl)carbamate

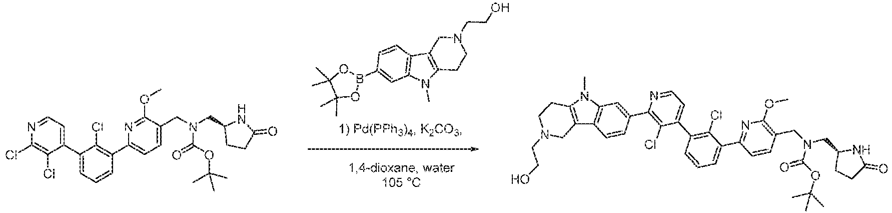

To a mixture of tert-butyl (S)-((6-(2-chloro-3-(2,3-dichloropyridin-4-yl)phenyl)-2-methoxypyridin-3-yl)methyl)((5-oxopyrrolidin-2-yl)methyl)carbamate (38 mg, 0.06 mmol), 2-(5-methyl-7-(4,4,5,5-tetramethyl-1,3,2-dioxaborolan-2-yl)-1,3,4,5-tetrahydro-2H-pyrido[4,3-b]indol-2-yl)ethan-1-ol (23 mg, 0.06 mmol) and potassium carbonate (27 mg, 0.19 mmol) in degassed 1,4-dioxane/water (6:1, 1.15 mL) was added tetrakis(triphenylphosphine)palladium(0) (7 mg, 0.01 mmol). The mixture was irradiated to 105 °C for 25 min in a Biotage Initiator microwave. Water was added and the mixture was extracted into EtOAc (3 x 15 mL), followed by MeOH/DCM (10%, 2 x 10 mL). The combined organic layers were dried over anhydrous sodium sulfate, concentrated, and purified by normal phase silica gel chromatography (0 to 20% MeOH/DCM) to provide tert-butyl (S)-((6-(2-chloro-3-(3-chloro-2-(2-(2-hydroxyethyl)-5-methyl-2,3,4,5-tetrahydro-1H-pyrido[4,3-b]indol-7-yl)pyridin-4-yl)phenyl)-2-methoxypyridin-3-yl)methyl)((5-oxopyrrolidin-2-yl)methyl)carbamate (46 mg, 91%). MS: m/z found 785.1 [M+H]⁺.

### (e) (S)-5-((((6-(2-Chloro-3-(3-chloro-2-(2-(2-hydroxyethyl)-5-methyl-2,3,4,5-tetrahydro-1H-pyrido[4,3-b]indol-7-yl)pyridin-4-yl)phenyl)-2-methoxypyridin-3-yl)methyl)amino)methyl)pyrrolidin-2-one

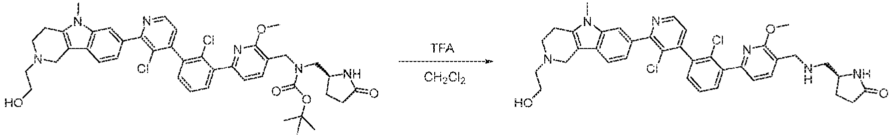

tert-Butyl (S)-((6-(2-chloro-3-(3-chloro-2-(2-(2-hydroxyethyl)-5-methyl-2,3,4,5-tetrahydro-1H-pyrido[4,3-b]indol-7-yl)pyridin-4-yl)phenyl)-2-methoxypyridin-3-yl)methyl)((5-oxopyrrolidin-2-yl)methyl)carbamate was converted to (S)-5-((((6-(2-Chloro-3-(3-chloro-2-(2-(2-hydroxyethyl)-5-methyl-2,3,4,5-tetrahydro-1H-pyrido[4,3-b]indol-7-yl)pyridin-4-yl)phenyl)-2-methoxypyridin-3-yl)methyl)amino)methyl)pyrrolidin-2-one following General Boc Deprotection Procedure. 55% yield. LCMS: m/z found 685.1 [M+H]⁺, retention time = 2.75 min, (Method A); ¹H NMR (400 MHz, Methanol-*d*₄): δ 8.62 (dd, 1H), 8.50 (s, 1H), 7.77 (dd, 1H), 7.72 - 7.69 (m, 2H), 7.60 - 7.51 (m, 2H), 7.46 - 7.37 (m, 3H), 7.27 (dd, 1H), 4.31 (s, 2H), 4.03 (s, 3H), 3.98 - 3.83 (m, 5H), 3.76 (d, 3H), 3.50 (s, 2H), 3.26 - 3.12 (m, 4H), 2.82 - 2.68 (m, 2H), 2.39 - 2.24 (m, 3H), 1.88 - 1.78 (m, 1H).

### Example 542: (S)-5-(((4-(3-Chloro-4-(2-chloro-3-(8-chloro-6-((((S)-2-hydroxypropyl)amino)methyl)-5-methoxyquinolin-2-yl)phenyl)pyridin-2-yl)-2-methoxybenzyl)amino)methyl)pyrrolidin-2-one (206)

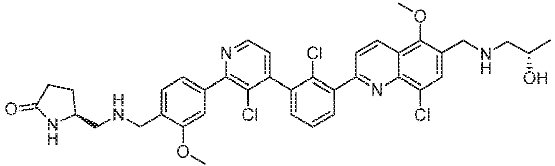

### (a) 4-(3-Bromo-2-chlorophenyl)-2,3-dichloropyridine

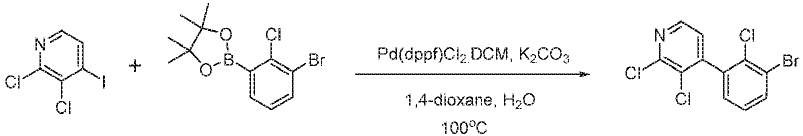

A mixture of 2,3-dichloro-4-iodo-pyridine (15 g, 54.8 mmol), 2-(3-bromo-2-chlorophenyl)-4,4,5,5-tetramethyl-1,3,2-dioxaborolane (26.1 g, 82.1 mmol), potassium carbonate (22.7 g, 164 mmol), [1,1'- bis(diphenylphosphino)ferrocene]dichloropalladium(II) complex with dichloromethane (447 mg, 0.55 mmol) in 1,4-dioxane/water (5/1, 240 mL) was degassed and purged with N₂, and then the mixture was stirred at 100 °C for 12 h. The reaction mixture was concentrated, and the residue was purified by normal phase SiO₂ chromatography (0-10% EtOAc/petroleum ethe) to afford 4-(3-bromo-2-chlorophenyl)-2,3-dichloropyridine (17 g, 91% yield) as a white solid.

### (b) 2,3-Dichloro-4-(2-chloro-3-(4,4,5,5-tetramethyl-1,3,2-dioxaborolan-2-yl)phenyl)pyridine

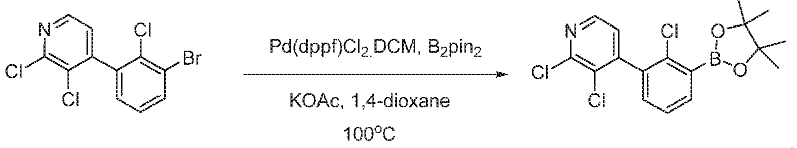

A mixture of 4-(3-bromo-2-chlorophenyl)-2,3-dichloropyridine (12 g, 35.6 mmol), bis(pinacolato)diborane (27.1 g, 107 mmol), [1,1'- bis(diphenylphosphino)ferrocene]-dichloropalladium(II) complex with dichloromethane (2.90 g, 3.56 mmol), potassium acetate (10.5 g, 107 mmol) in 1,4-dioxane (150 mL) was degassed and purged with N₂, and then the mixture was stirred at 100 °C for 12 h. The reaction mixture was filtered, concentrated and the residue was purified by normal phase SiO₂ chromatography (0-10% EtOAc/petroleum ether) to afford 2,3-dichloro-4-(2-chloro-3-(4,4,5,5-tetramethyl-1,3,2-dioxaborolan-2-yl)phenyl)pyridine (3 g, 22% yield) as a white solid.

### (c) 4-Amino-5-chloro-2-methoxybenzoic acid

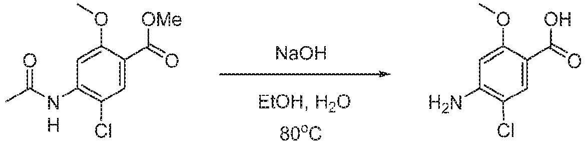

To a mixture methyl 4-acetamido-5-chloro-2-methoxybenzoate (100 g, 388 mmol) in ethanol / water (2:1, 600 mL) was added sodium hydroxide (62.1 g, 1.55 mol). The mixture was stirred at 80 °C for 12 h. The mixture was diluted with water and treated with concentrated HCl. The resulting precipitate was filtered, washed with water and dried to give 4-amino-5-chloro-2-methoxybenzoic acid (120 g, crude).

### (d) Methyl 8-chloro-5-methoxyquinoline-6-carboxylate

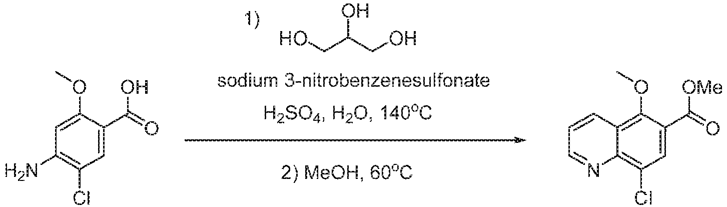

To a mixture 4-amino-5-chloro-2-methoxybenzoic acid (20 g, 99.2 mmol) in concentrated H₂SO₄/H₂O (5:1, 96 mL) was added glycerol (41.1 g, 446 mmol) and sodium 3-nitrobenzenesulfonate (44.7 g, 198 mmol). The mixture was stirred at 100 °C for 3 h and at 140 °C for 1 h. The mixture was cooled to 60 °C, methanol (40 mL) was added, and the mixture was stirred at 60 °C for 10 h. The solution was evaporated, the residue poured into ice-water mixture and basified with saturated aqueous ammonium hydroxide. The mixture was diluted with ethyl acetate (2 L) and filtered. The organic layer was separated, dried and evaporated. The residue was purified by normal phase SiO₂ chromatography (0-100% EtOAc/petroleum ether) to afford methyl 8-chloro-5-methoxyquinoline-6-carboxylate (4 g, 16 % yield). ¹H NMR (400 MHz, DMSO-d₆): δ 9.15 (dd, 1H), 8.70 (dd, 1H), 8.16 (s, 1H), 7.78 (dd, 1H), 4.00 (m, 3H), 3.93 (m, 3H).

### (e) 8-Chloro-5-methoxyquinoline-6-carboxylic acid

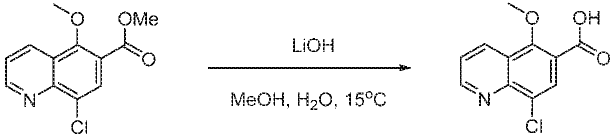

To a mixture methyl 8-chloro-5-methoxyquinoline-6-carboxylate (7 g, 27.8 mmol) in methanol / water (7:2, 90 mL) was added lithium hydroxide monohydrate (3.50 g, 83.4 mmol). The mixture was stirred at 15 °C for 15 hr and then concentrated under reduced pressure to give a residue. Water (30 mL) was added and the mixture extracted with EtOAc (3 x 30 ml). The aqueous phase was treated with concentrated HCl (to pH=3) and the resulting susension filtered to give 8-chloro-5-methoxyquinoline-6-carboxylic acid as a white solid (7 g, crude). ¹H NMR (400 MHz, DMSO-d₆): δ 9.07 (dd, 1H), 8.64 (dd, 1H), 8.10 (s, 1H), 7.66 (dd, 1H), 3.95 (s, 3H).

### (f) 5-Methoxyquinoline-6-carboxylic acid

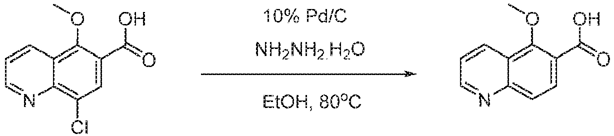

To a mixture 8-chloro-5-methoxyquinoline-6-carboxylic acid (1 g, 4.21 mmol) in ethanol (80 mL) was added hydrazine hydrate (1.05 g, 21 mmol) and 10% Pd/C (1 g). The mixture was then stirred at 80 °C for 14 hr. The reaction mixture was filtered and concentrated under reduced pressure to give a 5-methoxyquinoline-6-carboxylic acid as a red solid (5 g, crude). ¹H NMR (400 MHz, DMSO-d₆): δ 8.86 (dd, 1H), 8.50 (d, 1H), 7.80 (d, 1H), 7.66 (d, 1H), 7.53 (dd, 1H), 4.00 (s, 3H).

### (g) Methyl 5-methoxyquinoline-6-carboxylate

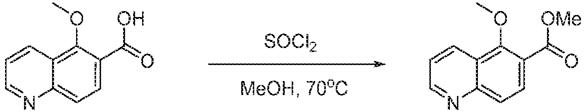

To a mixture 5-methoxyquinoline-6-carboxylic acid (5 g, 24.6 mmol) in methanol (20 mL) was added thionyl chloride (4.39 g, 36.9 mmol) and the mixture was stirred at 70 °C for 4 hr. The reaction mixture was concentrated under reduced pressure, water (30 mL) added and the mixture extracted with EtOAc (3 x 30 ml). The combined organic layers were washed with saturated aqueous brine solution (20 mL), dried over anhydrous sodium sulfate, filtered and concentrated under reduced pressure to give a residue. The residue was purified by normal phase SiO₂ chromatography (0-100% EtOAc/petroleum ether) to afford methyl 5-methoxyquinoline-6-carboxylate as a yellow solid (5 g, 93% yield).

### (h) 5-Methoxy-6-(methoxycarbonyl)quinoline 1-oxide

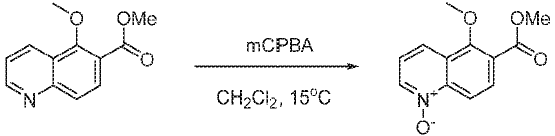

To a mixture methyl 5-methoxyquinoline-6-carboxylate (5 g, 23 mmol) in dichloromethane (5 mL) was added 3-chloroperbenzoic acid (5.96 g, 34.5 mmol) and the mixture stirred at 15 °C for 12 hr. Saturated sodium sulfite solution (50 mL) was added and the mixture extracted with dichloromethane (3 x 50 ml). The combined organic layers were washed with saturated aqueous brine solution (30 mL), dried over anhydrous sodium sulfate, filtered and concentrated under reduced pressure to give a residue. The residue was purified by normal phase SiO₂ chromatography (0-100% EtOAc/petroleum ether ~ 0-100% Ethyl acetate/MeOH) to give 5-methoxy-6-(methoxycarbonyl)quinoline 1-oxide as a white solid (3 g, 55% yield). ¹H NMR (400 MHz, DMSO-d₆): δ 8.71 (dd, 1H), 8.34 (d, 1H), 8.11 (d, 1H), 8.04 (d, 1H), 7.60-7.56 (m, 1H), 4.02 (s, 3H), 3.95 (m, 3H).

### (i) Methyl 2-bromo-5-methoxyquinoline-6-carboxylate

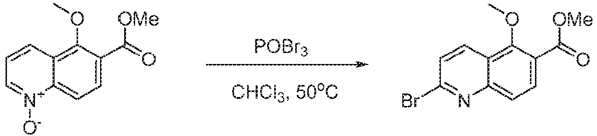

To a mixture 5-methoxy-6-(methoxycarbonyl)quinoline 1-oxide (3 g, 12.86 mmol,) in chloroform (10 mL) was added phosphorus oxybromide (11.1 g, 38.6 mmol) and the mixture stirred at 50 °C for 4 hr. Water (90 mL) was added and the mixture extracted with dichloromethane (3 x 90 ml). The combined organic layers were washed with saturated aqueous brine solution (60mL), dried over anhydrous sodium sulfate, filtered and concentrated under reduced pressure to give a residue. The residue was purified by normal phase SiO₂ chromatography (0-100% EtOAc/petroleum ether~ 0-100% Ethyl acetate/MeOH) to afford methyl 2-bromo-5-methoxyquinoline-6-carboxylate as a white solid (3 g, 79% yield). ¹H NMR (400 MHz, DMSO-d₆): δ 8.58 (dd, 1H), 8.11 (d, 1H), 7.88-7.84 (m, 2H), 4.05 (s, 3H), 3.98 (m, 3H).

### (j) Methyl 2,8-dichloro-5-methoxyquinoline-6-carboxylate

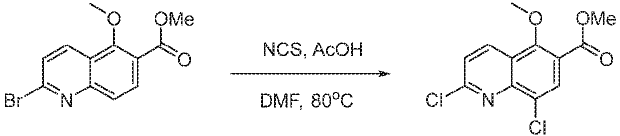

To a mixture of methyl 2-bromo-5-methoxyquinoline-6-carboxylate (0.6 g, 2.03 mmol) in DMF (10 mL) was added N-chlorosuccinimide (1.08 g, 8.1 mmol) and glacial acetic acid (0.01 g, 0.2 mmol). The mixture was stirred at 80 °C for 12 h. The reaction mixture was added to water (8 mL), and the resulting solid was collected by filtration to afford methyl 2,8-dichloro-5-methoxyquinoline-6-carboxylate (0.75 g, crude). ¹H NMR (400 MHz, DMSO-d₆): δ 8.70 (d, 1H), 8.20 (s, 1H), 7.81 (d, 1H), 3.99 (s, 3H), 3.93 (s, 3H).

### (k) (2,8-Dichloro-5-methoxyquinolin-6-yl)methanol

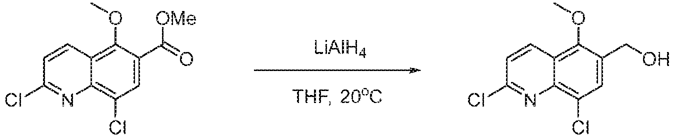

To a mixture of methyl 2,8-dichloro-5-methoxyquinoline-6-carboxylate (0.4 g, 1.4 mmol) in THF (5 mL) was added lithium aluminum hydride (0.06 g, 1.68 mmol) and the mixture was stirred at 20 °C for 1 h. The reaction mixture was added to water (5 mL) at 0 °C, dried over anhydrous sodium sulfate, filtered and concentrated under reduced pressure. The residue was purified by normal phase SiO₂ chromatography (0-100% EtOAc/petroleum ether) to afford (2,8-dichloro-5-methoxyquinolin-6-yl)methanol as a yellow solid (0.22 g). ¹H NMR (400 MHz, DMSO-d₆): δ 8.53 (d, 1H), 8.02 (s, 1H), 7.69 (d, 1H), 5.47 (br, 1H), 4.69 (s, 2H), 3.88 (s, 3H).

### (l) 2,8-Dichloro-5-methoxyquinoline-6-carbaldehyde

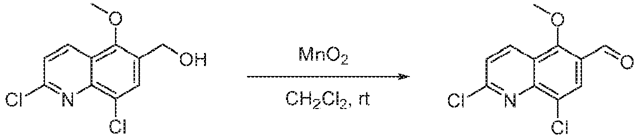

To a mixture (2,8-dichloro-5-methoxyquinolin-6-yl)methanol (0.2 g, 0.77 mmol) in dichloromethane (2 mL) was added manganese dioxide (0.67 g, 7.75 mmol) and the mixture was stirred at room temperature for 3 h. The reaction mixture was filtered and concentrated under reduced pressure to give 2,8-dichloro-5-methoxyquinoline-6-carbaldehyde (0.2 g, crude). ¹H NMR (400 MHz, Chloroform-d): δ 10.43 (s, 1H), 8.46 (d, 1H), 8.17 (s, 1H), 7.50 (d, 1H), 4.09 (s, 3H).

### (m) 8-Chloro-2-(2-chloro-3-(2,3-dichloropyridin-4-yl)phenyl)-5-methoxyquinoline-6-carbaldehyde

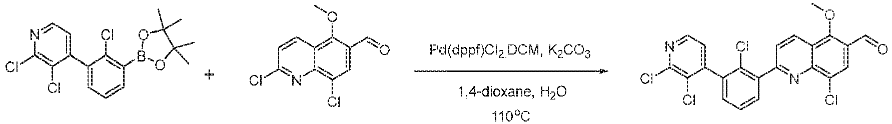

To a mixture of 2,8-dichloro-5-methoxyquinoline-6-carbaldehyde (400 mg, 1.56 mmol) and 2,3-dichloro-4-(2-chloro-3- (4,4,5,5-tetramethyl-1,3,2-dioxaborolan-2-yl)phenyl)pyridine (901 mg, 2.34 mmol) in 1,4-dioxane/water (12 mL, 5/1) was added potassium carbonate (648 mg, 4.69 mmol), followed by [1,1'-bis(diphenylphosphino)ferrocene]-dichloropalladium(II) complex with dichloromethane (128 mg, 0.16 mmol). The reaction was stirred for 3 h at 110 °C. The mixture was filtered and washed with EtOAc (50 mL). The filtrate was concentrated, and the residue was purified by normal phase SiO₂ chromatography (0-50% EtOAc/petroleum ether) to afford 8-chloro-2-(2-chloro-3-(2,3-dichloropyridin-4-yl)phenyl)-5-methoxyquinoline-6-carbaldehyde (516 mg) as a yellow solid. MS: m/z found 479 [M+H]⁺. ¹H NMR (400 MHz, DMSO-d₆): δ 10.49 (s, 1H), 8.58 (d, 1H), 8.32 (d, 1H), 8.19 (s, 1H), 7.97 (d, 1H), 7.87 (dd, 1H), 7.51 (t, 1H), 7.30 (d, 1H), 7.16 (d, 1H), 4.14 (s, 3H), 1.17 (s, 1H).

### (n) (S)-5-(((4-Bromo-2-methoxybenzyl)amino)methyl)pyrrolidin-2-one

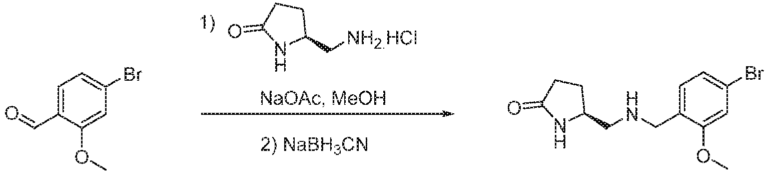

To a mixture of 4-bromo-2-methoxybenzaldehyde (3 g, 13.9 mmol) and (S)-5-(aminomethyl)pyrrolidin-2-one hydrochloride salt (2.52 g, 16.7 mmol) in methanol (40 mL) was added sodium acetate (3.43 g, 41.8 mmol) and the mixture was stirred at room temperature for 12 h. Sodium cyanoborohydride (1.75 g, 27.9 mmol) was added and the mixture was stirred at room temperature for 0.5 h. To the mixture was added water (50 mL) and the mixture was extracted with ethyl acetate (2 x 100 mL). The organic layer was dried over anhydrous sodium sulfate, filtered and concentrated to afford (S)-5-(((4-bromo-2-methoxybenzyl)amino)methyl)pyrrolidin-2-one as a yellow oil (4 g, crude) which was used for next step without further purification.

### (o) tert-Butyl (S)-(4-bromo-2-methoxybenzyl)((5-oxopyrrolidin-2-yl)methyl)carbamate

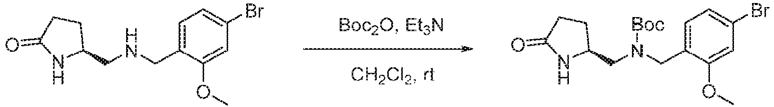

A mixture of (S)-5-(((4-bromo-2-methoxybenzyl)amino)methyl)pyrrolidin-2-one (3.9 g, 12.5 mmol), di-tert-butyl dicarbonate (8.15 g, 37.4 mmol) and triethylamine (5.20 mL, 37.4 mmol) in dichloromethane (40 mL) was degassed and purged with nitrogen. The mixture was stirred at room temperature for 3 h under N₂ atmosphere. The mixture was concentrated, and the residue was purified by normal phase SiO₂ chromatography (0-100% EtOAc/petroleum ether) to afford tert-butyl (S)-(4-bromo-2-methoxybenzyl)((5-oxopyrrolidin-2-yl)methyl)carbamate (4.2 g, 81% yield) as a white solid. ¹H NMR (400 MHz, DMSO-*d*₆): δ 7.73 (s, 1H), 7.19-7.13 (m, 2H), 6.93 (s, 1H), 4.38-4.27 (m, 2H), 3.83 (s, 3H), 3.71 (s, 1H), 3.21-3.16 (m, 2H), 2.15-1.99 (m, 3H), 1.13 (s, 1H), 1.42-1.31 (m, 9H).

### (p) tert-Butyl (S)-(2-methoxy-4-(4,4,5,5-tetramethyl-1,3,2-dioxaborolan-2-yl)benzyl)((5-oxopyrrolidin-2-yl)methyl)carbamate

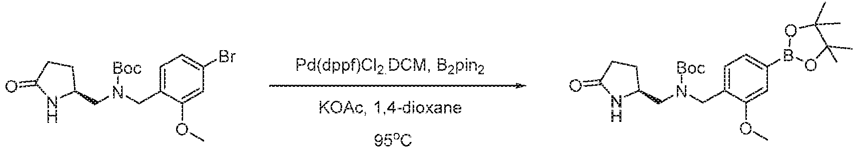

To a mixture of tert-butyl (S)-(4-bromo-2-methoxybenzyl)((5-oxopyrrolidin-2-yl)methyl)carbamate (4.1 g, 9.92 mmol), bis(pinacolato)diborane (7.56 g, 29.8 mmol) in 1,4-dioxane (40 mL) was added potassium acetate (2.92 g, 29.8 mmol) and [1,1'-bis(diphenylphosphino)ferrocene]-dichloropalladium(II) complex with dichloromethane (810 mg, 0.99 mmol). The mixture was stirred at 95 °C for 12 h under N₂. The mixture was filtered, the filtrate concentrated and the residue purified by normal phase SiO₂ chromatography (0-100% EtOAc/petroleum ether) to afford tert-butyl (S)-(2-methoxy-4-(4,4,5,5-tetramethyl-1,3,2-dioxaborolan-2-yl)benzyl)((5-oxopyrrolidin-2-yl)methyl)carbamate (2.6 g, 56% yield) as a black solid. ¹H NMR (400 MHz, DMSO-d₆): δ 7.72 (s, 1H), 7.27 (d, 1H), 7.18 (s, 1H), 7.01 (d, 1H), 4.41-4.39 (m, 2H), 3.93 (s, 3H), 3.72 (s, 1H), 3.21-3.17 (m, 2H), 2.17-2.04 (m, 3H), 1.75-1.71 (m, 1H), 1.43-1.30 (m, 21H).

### (q) tert-Butyl (S)-(4-(3-chloro-4-(2-chloro-3-(8-chloro-6-formyl-5-methoxyquinolin-2-yl)phenyl)pyridin-2-yl)-2-methoxybenzyl)((5-oxopyrrolidin-2-yl)methyl)carbamate

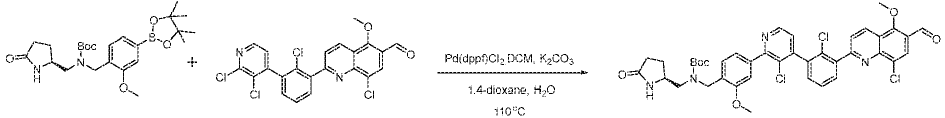

To a mixture of 8-chloro-2-(2-chloro-3-(2,3-dichloropyridin-4-yl)phenyl)-5-methoxy qui noline-6-carbaldehyde (260 mg, 0.54 mmol) and (S)-tert-butyl 2-methoxy-4-(4,4,5,5-tetra methyl-1,3,2-dioxaborolan-2-yl)benzyl((5-oxopyrrolidin-2-yl)methyl)carbamate (275 mg, 0.60 mmol) in 1,4-dioxane/water (6 mL, 5:1) was added potassium carbonate (225 mg, 1.63 mmol) , followed by 1,1'-bis(diphenylphosphino)ferrocene-palladium(ii) dichloride dichloromethane complex (44.4 mg, 0.05 mmol). The reaction was stirred for 3 h at 110 °C. After cooling, the mixture was diluted with water (20 mL) and extracted with EtOAC (100 mL). The organic layer was separated, dried over anhydrous sodium sulfate, filtered and concentrated. The residue was purified by normal phase SiO₂ chromatography (0-100% EtOAc/petroleum ether to 0~20% MeOH/EtOAc) to afford tert-butyl (S)-(4-(3-chloro-4-(2-chloro-3-(8-chloro-6-formyl-5-methoxyquinolin-2-yl)phenyl)pyridin-2-yl)-2-methoxybenzyl)((5-oxopyrrolidin-2-yl)methyl)carbamate (320 mg, 43% yield) as a yellow solid. ¹H NMR (400 MHz, DMSO-d₆): δ 10.60 (s, 1 H), 8.72-7.69 (m, 2 H), 8.31 (s, 1 H), 8.10 (d, 1 H), 7.98 (d, 1 H), 7.63 (t, 1 H), 7.48 (d, 2 H), 7.42 (d, 2 H), 7.32 (s, 1 H), 4.60-4.56 (m, 3 H), 4.25 (s, 3 H), 3.95 (s, 3 H), 3.37-3.32 (m, 3 H), 2.24-2.19 (m, 2 H), 1.80-1.77 (m, 2 H), 1.50 (m, 9 H).

### (r) tert-Butyl (4-(3-chloro-4-(2-chloro-3-(8-chloro-6-((((S)-2-hydroxypropyl)amino)methyl)-5-methoxyquinolin-2-yl)phenyl)pyridin-2-yl)-2-methoxybenzyl)(((S)-5-oxopyrrolidin-2-yl)methyl)carbamate

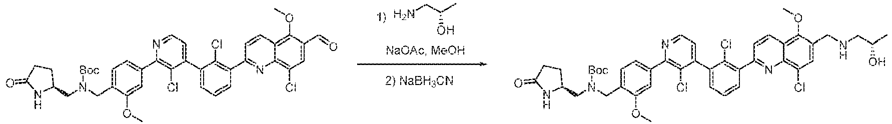

To a mixture of tert-butyl (S)-(4-(3-chloro-4-(2-chloro-3-(8-chloro-6-formyl-5-methoxyquinolin-2-yl)phenyl)pyridin-2-yl)-2-methoxybenzyl)((5-oxopyrrolidin-2-yl)methyl)carbamate (60 mg, 0.07 mmol) in methanol (2 mL) was added (S)-1-aminopropan-2-ol (12.00 mg, 0.16 mmol) and sodium acetate (19.0 mg, 0.23 mmol). The mixture was stirred at room temperature for 11 h. Sodium cyanoborohydride (15 mg, 0.23 mmol) was added and the mixture was stirred for another 1 h. The residue was purified by prep-TLC (20% MeOH/ Ethyl acetate) to afford tert-butyl (4-(3-chloro-4-(2-chloro-3-(8-chloro-6-((((S)-2-hydroxypropyl)amino)methyl)-5-methoxyquinolin-2-yl)phenyl)pyridin-2-yl)-2-methoxybenzyl)(((S)-5-oxopyrrolidin-2-yl)methyl)carbamate (50 mg, 61% yield) as a light yellow solid.

### (s) (S)-5-(((4-(3-Chloro-4-(2-chloro-3-(8-chloro-6-((((S)-2-hydroxypropyl)amino)methyl)-5-methoxyquinolin-2-yl)phenyl)pyridin-2-yl)-2-methoxybenzyl)amino)methyl)pyrrolidin-2-one

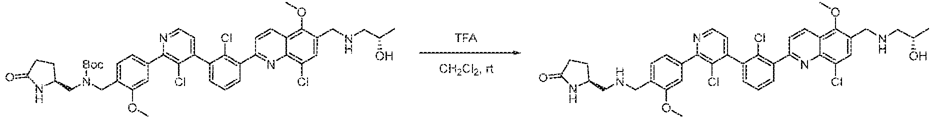

A mixture of tert-butyl (4-(3-chloro-4-(2-chloro-3-(8-chloro-6-((((S)-2-hydroxypropyl)amino)methyl)-5-methoxyquinolin-2-yl)phenyl)pyridin-2-yl)-2-methoxybenzyl)(((S)-5-oxopyrrolidin-2-yl)methyl)carbamate (50 mg, 0.06 mmol) and trifluoroacetic acid (1.00 ml, 13.5 mmol) in dichloromethane (0.5 mL) was stirred for 0.5 h at room temperature. The mixture was concentrated under reduced pressure and the residue was purified by reverse phase HPLC to afford (S)-5-(((4-(3-chloro-4-(2-chloro-3-(8-chloro-6-((((S)-2-hydroxypropyl)amino)methyl)-5-methoxyquinolin-2-yl)phenyl)pyridin-2-yl)-2-methoxybenzyl)amino)methyl)pyrrolidin-2-one (8.9 mg, 18%) as a white solid (formic acid salt). MS: m/z found 734 and 736 [M+H]⁺, retention time = 3.02 min (Method A). ¹H NMR (400 MHz, Methanol-*d*₄): δ 8.70-7.67 (m, 2H), 8.55 (br s, 1H), 8.06 (s, 1H), 7.98 (d, 1H), 7.85 (dd, 1H), 7.67 (t, 1H), 7.55 (dd, 1H), 7.51 (d, 1H), 7.45 (d, 1H), 7.33-7.30 (m, 2H), 4.22 ( s, 2H), 4.07 (s, 3H), 4.00-3.96 (m, 6H), 3.90-3.87 (m, 1H), 2.83-2.73 (m, 4H), 2.38-2.30 (m, 3H), 1.85-1.80 (m, 1H), 1.22 (d, 3H).

### Example 543: (S)-5-(((4-(3-chloro-4-(2-chloro-3-(8-chloro-6-(((2-hydroxyethyl)amino)methyl)-5-methoxyquinolin-2-yl)phenyl)pyridin-2-yl)-2-methoxybenzyl)amino)methyl)pyrrolidin-2-one (238)

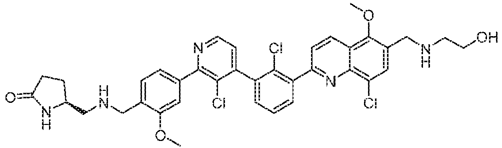

### (a) tert-Butyl (S)-(4-(3-chloro-4-(2-chloro-3-(8-chloro-6-(((2-hydroxyethyl)amino)methyl)-5-methoxyquinolin-2-yl)phenyl)pyridin-2-yl)-2-methoxybenzyl)((5-oxopyrrolidin-2-yl)methyl)carbamate

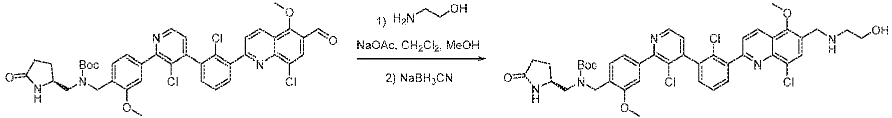

To a mixture of tert-butyl (S)-(4-(3-chloro-4-(2-chloro-3-(8-chloro-6-formyl-5-methoxyquinolin-2-yl)phenyl)pyridin-2-yl)-2-methoxybenzyl)((5-oxopyrrolidin-2-yl)methyl)carbamate (Example 542, step (q)) (0.07 g, 0.09 mmol) in dichloromethane/methanol mixture (1:1, 0.6 mL) was added 2-aminoethanol (17 mg, 0.27 mmol) and sodium acetate (0.04 g, 0.45 mmol). After 2 h, sodium cyanoborohydride (0.02 g, 0.27 mmol) was added and the mixture was stirred for another 10 h at room temperature. The reaction mixture was purified by prep-TLC (20% MeOH/ethyl acetate) to afford tert-butyl (S)-(4-(3-chloro-4-(2-chloro-3-(8-chloro-6-(((2-hydroxyethyl)amino)methyl)-5-methoxyquinolin-2-yl)phenyl)pyridin-2-yl)-2-methoxybenzyl)((5-oxopyrrolidin-2-yl)methyl)carbamate (70 mg, 70%) as a yellow solid. MS: m/z found 820 and 822 [M+H]⁺.

### (b) (S)-5-(((4-(3-Chloro-4-(2-chloro-3-(8-chloro-6-(((2-hydroxyethyl)amino)methyl)-5-methoxyquinolin-2-yl)phenyl)pyridin-2-yl)-2-methoxybenzyl)amino)methyl)pyrrolidin-2-one

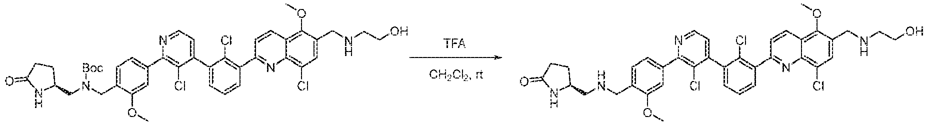

A mixture of tert-butyl (S)-(4-(3-chloro-4-(2-chloro-3-(8-chloro-6-(((2-hydroxyethyl)amino)methyl)-5-methoxyquinolin-2-yl)phenyl)pyridin-2-yl)-2-methoxybenzyl)((5-oxopyrrolidin-2-yl)methyl)carbamate (0.07 g, 0.09 mmol) and trifluoroacetic acid (3 mL, 40.5 mmol) in dichloromethane (1 mL) was stirred for 0.5 h at room temperature. The mixture was concentrated, and the residue was purified by reverse phase HPLC to afford (S)-5-(((4-(3-chloro-4-(2-chloro-3-(8-chloro-6-(((2-hydroxyethyl)amino)methyl)-5-methoxyquinolin-2-yl)phenyl)pyridin-2-yl)-2-methoxybenzyl)amino)methyl)pyrrolidin-2-one (17 mg, 26%) as a light yellow solid (formic acid salt). MS: m/z found 720 and 720 [M+H]⁺, retention time = 2.90 min (Method A).¹H NMR (400 MHz, Methanol-*d*₄): δ 8.71-8.66 (m, 2H), 8.42 (br s, 2H), 8.06 (s, 1H), 8.00 (d, 1H), 7.83 (dd, 1H), 7.66 (t, 1H), 7.55-7.48 (m, 3H), 7.37-7.33 (m, 2H), 4.43 (s, 2H), 4.22-4.14 (m, 2H), 4.09 (s, 3H), 3.98 (m, 4H), 3.83 (t, 2H), 3.16 (t, 2H), 3.06-2.99 (m, 2H), 2.45-2.31 (m, 3H), 1.91-1.80 (m, 1H).

### Example 544: (S)-5-(((4-(4-(3-(6-(((1-Acetylpiperidin-4-yl)amino)methyl)-8-chloro-5-methoxyquinolin-2-yl)-2-chlorophenyl)-3-chloropyridin-2-yl)-2-methoxybenzyl)amino)methyl)pyrrolidin-2-one (241)

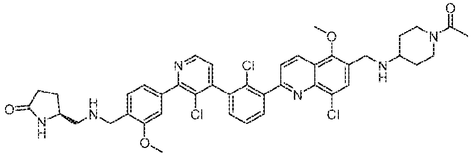

### (a) tert-Butyl (S)-(4-(4-(3-(6-(((1-acetylpiperidin-4-yl)amino)methyl)-8-chloro-5-methoxyquinolin-2-yl)-2-chlorophenyl)-3-chloropyridin-2-yl)-2-methoxybenzyl)((5-oxopyrrolidin-2-yl)methyl)carbamate

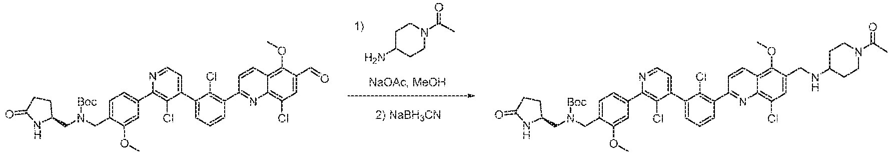

To a mixture of tert-butyl (S)-(4-(3-chloro-4-(2-chloro-3-(8-chloro-6-formyl-5-methoxyquinolin-2-yl)phenyl)pyridin-2-yl)-2-methoxybenzyl)((5-oxopyrrolidin-2-yl)methyl)carbamate (Example 542, step (q)) (60 mg, 0.08 mmol) in methanol (0.5 mL) was added 1-(4-aminopiperidin-1-yl)ethanone (33 mg, 0.23 mmol) and sodium acetate (32 mg, 0.39 mmol). The mixture was stirred for 11 h at room temperature. Sodium cyanoborohydride (15 mg, 0.23 mmol) was added and the mixture was stirred for another 1 h. The reaction mixture was concentrated the residue was purified by prep-TLC (20% MeOH/ Ethyl acetate) to afford tert-butyl (S)-(4-(4-(3-(6-(((1-acetylpiperidin-4-yl)amino)methyl)-8-chloro-5-methoxyquinolin-2-yl)-2-chlorophenyl)-3-chloropyridin-2-yl)-2-methoxybenzyl)((5-oxopyrrolidin-2-yl)methyl)carbamate (49 mg, 63%) as a yellow soild. MS: m/z found 901 and 903 [M+H]⁺.

### (b) (S)-5-(((4-(4-(3-(6-(((1-Acetylpiperidin-4-yl)amino)methyl)-8-chloro-5-methoxyquinolin-2-yl)-2-chlorophenyl)-3-chloropyridin-2-yl)-2-methoxybenzyl)amino)methyl)pyrrolidin-2-one

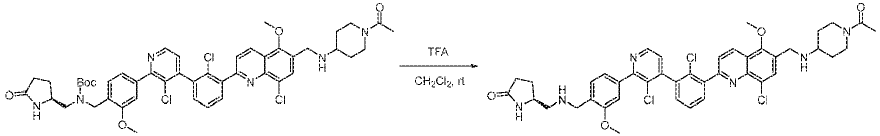

To a mixture of tert-butyl (S)-(4-(4-(3-(6-(((1-acetylpiperidin-4-yl)amino)methyl)-8-chloro-5-methoxyquinolin-2-yl)-2-chlorophenyl)-3-chloropyridin-2-yl)-2-methoxybenzyl)((5-oxopyrrolidin-2-yl)methyl)carbamate (49 mg, 0.05 mmol) in dichloromethane (1 mL) was added trifluoroacetic acid (2 mL, 27 mmol) and the mixture was stirred for 0.5 h at room temperature. The mixture was concentrated, and the residue was purified by reverse phase HPLC to afford (S)-5-(((4-(4-(3-(6-(((1-acetylpiperidin-4-yl)amino)methyl)-8-chloro-5-methoxyquinolin-2-yl)-2-chlorophenyl)-3-chloropyridin-2-yl)-2-methoxybenzyl)amino)methyl)pyrrolidin-2-one (23.7 mg, 46% yield) as a light yellow solid (formic acid salt). MS: m/z found 801 and 803 [M+H]⁺, retention time = 3.01 min (Method A). ¹H NMR (400 MHz, Methanol-*d*₄): δ 8.68-8.65 (m, 2H), 8.45 (br s, 1H), 8.06 (s, 1H), 7.95 (d, 1H), 7.82 (dd, 1H), 7.65 (t, 1H), 7.54-7.50 (m, 2H), 7.46 (d, 1H), 7.35-7.31 (m, 2H), 4.60 (s, 1H), 4.53-4.50 (m, 1H), 4.19 (s, 2H), 4.08 (s, 2H), 4.04 (s, 3H), 3.96-3.92 (m, 5H), 3.13 (m, 1H), 2.99-2.92 (m, 3H), 2.73-2.68 (m, 1H), 2.38-2.28 (m, 3H), 2.14-2.07 (m, 5H), 1.89-1.85 (m, 1H), 1.49-1.42 (m, 1H).

### Example 545: (S)-5-((((8-Chloro-2-(2-chloro-3-(3-chloro-2-(3-methoxy-4-(((((S)-5-oxopyrrolidin-2-yl)methyl)amino)methyl)phenyl)pyridin-4-yl)phenyl)-5-methoxyquinolin-6-yl)methyl)amino)methyl)pyrrolidin-2-one (207)

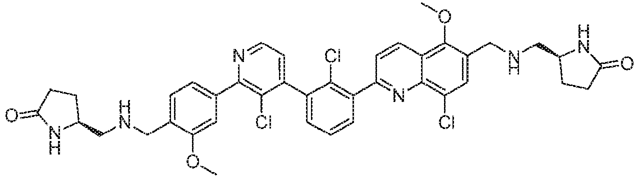

### (a) tert-Butyl (4-(3-chloro-4-(2-chloro-3-(8-chloro-5-methoxy-6-(((((S)-5-oxopyrrolidin-2-yl)methyl)amino)methyl)quinolin-2-yl)phenyl)pyridin-2-yl)-2-methoxybenzyl)(((S)-5-oxopyrrolidin-2-yl)methyl)carbamate

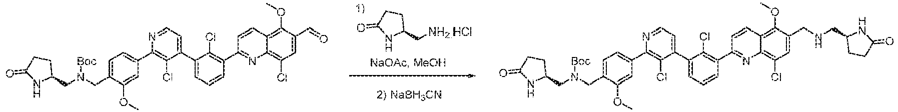

To a mixture of tert-butyl (S)-(4-(3-chloro-4-(2-chloro-3-(8-chloro-6-formyl-5-methoxyquinolin-2-yl)phenyl)pyridin-2-yl)-2-methoxybenzyl)((5-oxopyrrolidin-2-yl)methyl)carbamate (Example 542, step (q)) (60 mg, 0.08 mmol) in MeOH (0.5 mL) was added (S)-5-(aminomethyl)pyrrolidin-2-one hydrochloride salt (18 mg, 0.12 mmol) and sodium acetate (32 mg, 0.39 mmol) and the mixture was stirred at room temperature for 11 h. Sodium cyanoborohydride (15 mg, 0.23 mmol) was added and the mixture was stirred for another 1 h. The reaction was concentrated and the residue was purified by prep-TLC (20% MeOH/ Ethyl acetate) to afford tert-butyl (4-(3-chloro-4-(2-chloro-3-(8-chloro-5-methoxy-6-(((((S)-5-oxopyrrolidin-2-yl)methyl)amino)methyl)quinolin-2-yl)phenyl)pyridin-2-yl)-2-methoxybenzyl)(((S)-5-oxopyrrolidin-2-yl)methyl)carbamate (50 mg, 56% yield) as a yellow solid.

### (b) (S)-5-((((8-Chloro-2-(2-chloro-3-(3-chloro-2-(3-methoxy-4-(((((S)-5-oxopyrrolidin-2-yl)methyl)amino)methyl)phenyl)pyridin-4-yl)phenyl)-5-methoxyquinolin-6-yl)methyl)amino)methyl)pyrrolidin-2-one

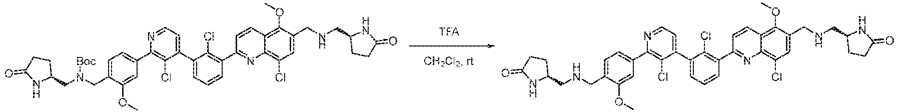

To a mixture of tert-butyl (4-(3-chloro-4-(2-chloro-3-(8-chloro-5-methoxy-6-(((((S)-5-oxopyrrolidin-2-yl)methyl)amino)methyl)quinolin-2-yl)phenyl)pyridin-2-yl)-2-methoxybenzyl)(((S)-5-oxopyrrolidin-2-yl)methyl)carbamate (50 mg, 0.06 mmol) in dichloromethane (0.5 mL) was added trifluoroacetic acid (1 mL, 13.5 mmol) and the mixture stirred for 0.5 h at room temperature. The mixture was concentrated and the residue was purified by reverse phase HPLC to afford (S)-5-((((8-chloro-2-(2-chloro-3-(3-chloro-2-(3-methoxy-4-(((((S)-5-oxopyrrolidin-2-yl)methyl)amino)methyl)phenyl)pyridin-4-yl)phenyl)-5-methoxyquinolin-6-yl)methyl)amino)methyl)pyrrolidin-2-one (14.5 mg, 30% yield) as a white solid (formic acid salt).MS: m/z found 773 and 775 [M+H]⁺, retention time = 2.97 min (Method A). ¹H NMR (400 MHz, Methanol-*d*₄): δ 8.69-8.66 (m, 2H), 8.07 (s, 1H), 7.94 (d, 1H), 7.84 (dd, 1H), 7.66 (t, 1H), 7.56-7.52 (m, 2H), 7.48 (d, 1H), 7.36-7.32 (m, 2H), 4.08 (s, 4H), 4.03 (s, 3H), 3.98 (s, 3H), 3.95-3.93 (m, 1H), 3.88-3.85 (m, 1H), 2.92-2.89 (m, 2H), 2.79-2.73 (m, 2H), 2.40-2.29 (m, 6H), 1.88-1.83 (m, 2H).

### Example 546: (S)-5-((((8-Chloro-2-(2-chloro-3-(3-chloro-2-(3-fluoro-5-methoxy-4-(((((S)-5-oxopyrrolidin-2-yl)methyl)amino)methyl)phenyl)pyridin-4-yl)phenyl)-5-methoxyquinolin-6-yl)methyl)amino)methyl)pyrrolidin-2-one (236)

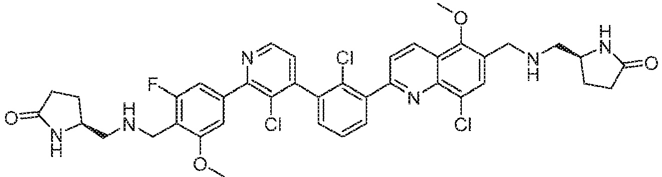

### (a) 8-Chloro-2-(2-chloro-3-(3-chloro-2-(3-fluoro-4-formyl-5-methoxyphenyl)pyridin-4-yl)phenyl)-5-methoxyquinoline-6-carbaldehyde

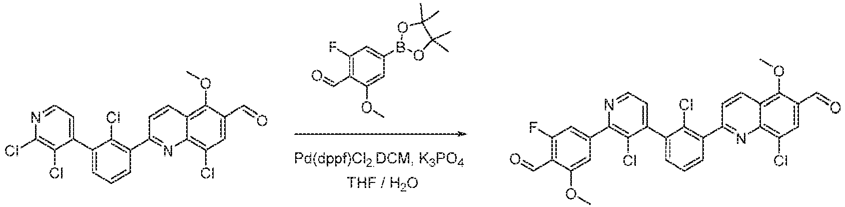

To a mixture of 8-chloro-2-(2-chloro-3-(2,3-dichloropyridin-4-yl)phenyl)-5-methoxyquinoline-6-carbaldehyde (100 mg, 209 µmol) (see Example 542, step (m)) and 2-fluoro-6-methoxy-4-(4,4,5,5-tetramethyl-1,3,2-dioxaborolan-2-yl)benzaldehyde (117 mg, 418 µmol) in tetrahydrofuran (3 mL) and water (0.6 mL) was added potassium phosphate (133.18 mg, 627 µmol) and [1,1'- bis(diphenylphosphino)ferrocene]dichloropalladium(II) complex with dichloromethane (13.6 mg, 20.9 µmol) and then the mixture was stirred at 85 °C for 1 h under N₂ . The mixture was filtered, the filtrate concentrated and the residue purified by reverse phase HPLC to afford compound 8-chloro-2-(2-chloro-3-(3-chloro-2-(3-fluoro-4-formyl-5-methoxyphenyl)pyridin-4-yl)phenyl)-5-methoxyquinoline-6-carbaldehyde (50 mg, 31.0% yield) as a white solid. MS: *m*/*z* found 595 [M+H]⁺.

### (b) (S)-5-((((8-Chloro-2-(2-chloro-3-(3-chloro-2-(3-fluoro-5-methoxy-4-(((((S)-5-oxopyrrolidin-2-yl)methyl)amino)methyl)phenyl)pyridin-4-yl)phenyl)-5-methoxyquinolin-6-yl)methyl)amino)methyl)pyrrolidin-2-one

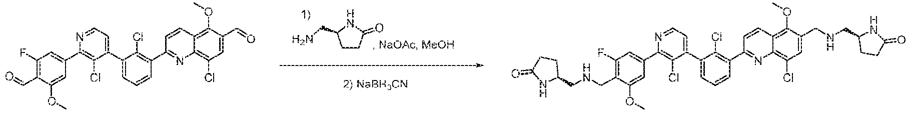

To a mixture of 8-chloro-2-(2-chloro-3-(3-chloro-2-(3-fluoro-4-formyl-5-methoxyphenyl)pyridin-4-yl)phenyl)-5-methoxyquinoline-6-carbaldehyde (40 mg, 67.1 µmol) and (S)-5-(aminomethyl)pyrrolidin-2-one (40.4 mg, 268 µmol) in methanol (1.5 mL) was added sodium acetate (33.0 mg, 402 µmol) and then the mixture stirred at room temperature for 12 h under N₂. Sodium cyanoborohydride (25.3 mg, 402 µmol) was then added and the mixture stirred at room temperature for 0.5 h under N₂. The mixture was filtered, the filtrate concentrated and the residue purified by reverse phase HPLC to afford compound (S)-5-((((8-Chloro-2-(2-chloro-3-(3-chloro-2-(3-fluoro-5-methoxy-4-(((((S)-5-oxopyrrolidin-2-yl)methyl)amino)methyl)phenyl)pyridin-4-yl)phenyl)-5-methoxyquinolin-6-yl)methyl)amino)methyl)pyrrolidin-2-one (10 mg, 17% yield) as a white solid (formic acid salt). MS: *m*/*z* found 791 [M+H]⁺, retention time = 3.03 min (Method A). ¹H NMR (400 MHz, Methanol-*d*₄): δ 8.71-8.67 (m, 2H), 8.39 (brs), 8.08 (s, 1H), 7.96 (d, 1H), 7.85 (dd, 1H), 7.67 (t, 1H), 7.56-7.54 (m, 2H), 7.24 (s, 1H), 7.19-7.17 (m, 1H), 4.19-4.09 (m, 4H), 4.04-4.01 (m, 6H), 3.94-3.87 (m, 2H), 2.95-2.77 (m, 4H), 2.40-2.28 (m, 6H), 1.88-1.82 (m, 2H).

### Example 547: 6-(2-Chloro-3-(3-chloro-2-(3-methoxy-4-(((((S)-5-oxopyrrolidin-2-yl)methyl)amino)methyl)phenyl)pyridin-4-yl)phenyl)-2-(2-((((S)-5-oxopyrrolidin-2-yl)methyl)amino)ethyl)-1,2-dihydro-3H-pyrrolo[3,4-c]pyridin-3-one (290)

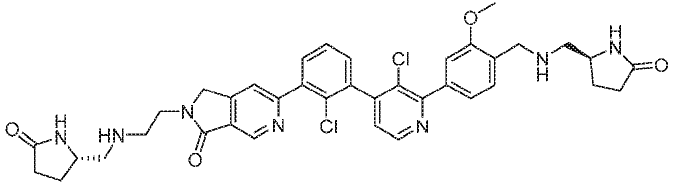

### (a) Methyl 4-(bromomethyl)-6-chloronicotinate

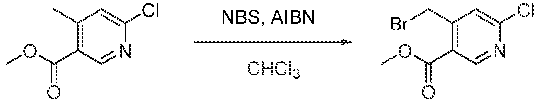

To a mixture of methyl 6-chloro-4-methylnicotinate (4 g, 21 mmol) in chloroform (90 mL) was added N-bromosuccinimide (4.99 g, 28 mmol) and 2,2'-azobis(2-methylpropionitrile) (0.35 g, 2.16 mmol) in one portion under N₂ atmosphere. The mixture was stirred at 70 °C for 16 h. Water (5 mL) was added to the mixture, the organic phase separated, washed with saturated aqueous brine solution (2 x 5 mL), dried over anhydrous sodium sulfate, filtered and concentrated. The residue was purified by normal phase SiO₂ chromatography (0-10% ethyl acetate/petroleum ether) to afford methyl 4-(bromomethyl)-6-chloronicotinate (7 g, 37% purity) as a colorless oil (7 g, 37% purity). MS: *m*/*z* found 264 and 266 [M+H]⁺.

### (b) 6-Chloro-2-(2,2-dimethoxyethyl)-1,2-dihydro-3H-pyrrolo[3,4-c]pyridin-3-one

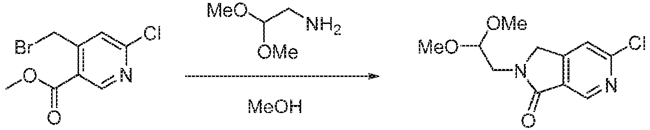

To a mixture of methyl 4-(bromomethyl)-6-chloronicotinate (7 g, 37% purity, 10.6 mmol) in methanol (40 mL) was added 2,2-dimethoxyethan-1-amine (3.5 mL, 31.8 mmol) in one portion under N₂ atmosphere. The mixture was stirred at room temperature for 1 h, then the mixture was concentrated. Water (20 mL) and saturated aqueous brine solution (5 mL) were added to the residue and the mixture was extracted with ethyl acetate/THF mixture (1:1, 20 mL). The organic phase was dried with anhydrous sodium sulfate, filtered and concentrated. The residue was purified by normal phase SiO₂ chromatography (0-40% ethyl acetate/petroleum ether) to afford 6-chloro-2-(2,2-dimethoxyethyl)-1,2-dihydro-3H-pyrrolo[3,4-c]pyridin-3-one (1.2 g, 44% yield) as a white solid. ¹H NMR (400 MHz, CDCl₃): δ 8.79 (s, 1H), 7.38 (s, 1H), 4.49 (s, 2H), 4.47 (t, 1H), 3.65 (d, 2H), 3.36 (s, 6H).

### (c) 2-(6-Chloro-3-oxo-1,3-dihydro-2H-pyrrolo[3,4-c]pyridin-2-yl)acetaldehyde

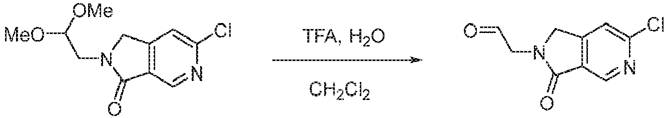

To a mixture of 6-chloro-2-(2,2-dimethoxyethyl)-1,2-dihydro-3H-pyrrolo[3,4-c]pyridin-3-one (0.4 g, 1.56 mmol) in dichloromethane (2 mL) was added trifluoroacetic acid (2 mL, 27.0 mmol) and water (0.08 mL, 4.44 mmol) in one portion. The mixture was stirred at room temperature for 2 h. The mixture was then concentrated to afford 2-(6-chloro-3-oxo-1,3-dihydro-2H-pyrrolo[3,4-c]pyridin-2-yl)acetaldehyde (0.35 g, crude) as a yellow gum. MS: *m*/*z* found 211 [M+H]⁺. The crude product was used in the next step without further purification.

### (d) (S)-6-Chloro-2-(2-(((5-oxopyrrolidin-2-yl)methyl)amino)ethyl)-1,2-dihydro-3H-pyrrolo[3,4-c]pyridin-3-one

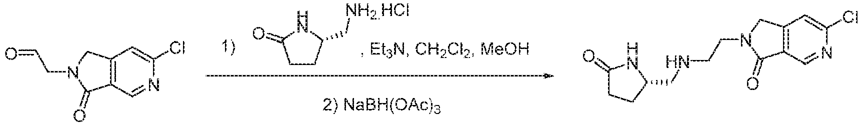

To a mixture of 2-(6-chloro-3-oxo-1H-pyrrolo[3,4-c]pyridin-2(3H)-yl)acetaldehyde (0.35 g, 1.66 mmol) and (S)-5-(aminomethyl)pyrrolidin-2-one hydrochloride salt (0.3 g, 1.99 mmol) in dichloromethane/methanol mixture (1:1, 4 mL) was added triethylamine (0.46 mL, 3.32 mmol) in one portion under N₂ atmosphere. The mixture was stirred at room temperature for 0.5 h. Sodium triacetoxyborohydride (0.7 g, 3.32 mmol) was then added and the mixture stirred at room temperature for 2.5 h. The crude light yellow suspension of (S)-6-chloro-2-(2-(((5-oxopyrrolidin-2-yl)methyl)amino)ethyl)-1,2-dihydro-3H-pyrrolo [3,4-c]pyridin-3-one (MS: *m*/*z* found 309 [M+H]⁺) in dichloromethane/methanol mixture was used in the next step without purification.

### (e) tert-Butyl (S)-(2-(6-chloro-3-oxo-1,3-dihydro-2H-pyrrolo[3,4-c]pyridin-2-yl)ethyl)((5-oxopyrrolidin-2-yl)methyl)carbamate

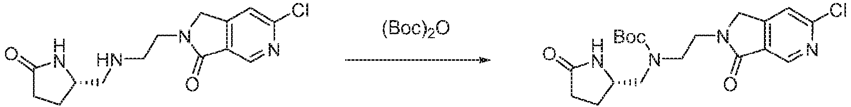

To the crude mixture of (S)-6-chloro-2-(2-(((5-oxopyrrolidin-2-yl)methyl)amino)ethyl)-1,2-dihydro-3H-pyrrolo[3,4-c]pyridin-3-one in dichloromethane/methanol (step d) was added di-tert-butyl dicarbonate (0.72 g, 3.32 mmol) in one portion under N₂ atmosphere. The mixture was stirred at room temperature for 0.5 h, then water (5 mL) was added, and the mixture was extracted with dichloromethane (2 x 10 mL). The combined organic phases were washed with saturated aqueous brine solution (2 x 5 mL), dried with anhydrous sodium sulfate, filtered and concentrated. The residue was purified by normal phase SiO₂ chromatography (0-100% ethyl acetate/petroleum ether to 10% MeOH/ ethyl acetate) to afford tert-butyl (S)-(2-(6-chloro-3-oxo-1,3-dihydro-2H-pyrrolo[3,4-c]pyridin-2-yl)ethyl)((5-oxopyrrolidin-2-yl)methyl)carbamate (0.32 g, 41% yield) as a yellow gum. ¹H NMR (400 MHz, Methanol-*d*₄): δ 8.64-8.58 (m, 1H), 7.63 (m, 1H), 4.59-4.53 (m, 2H), 3.89-3.79 (m, 1H), 3.68 (m, 2H), 3.54-3.41 (m, 2H), 3.31-3.25 (m, 2H), 2.28-2.16 (m, 3H), 1.75-1.71 (m, 1H), 1.23-1.11 (m, 9H).

### (f) tert-Butyl (S)-(2-(6-(2-chloro-3-(2,3-dichloropyridin-4-yl)phenyl)-3-oxo-1,3-dihydro-2H-pyrrolo[3,4-c]pyridin-2-yl)ethyl)((5-oxopyrrolidin-2-yl)methyl)carbamate

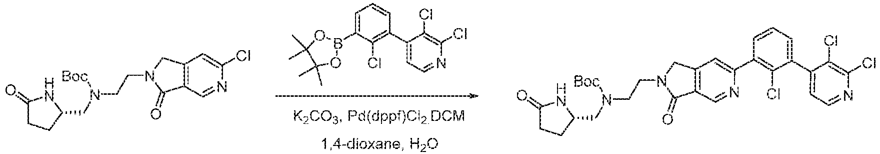

To a mixture of (tert-butyl (S)-(2-(6-chloro-3-oxo-1,3-dihydro-2H-pyrrolo[3,4-c]pyridin-2-yl)ethyl)((5-oxopyrrolidin-2-yl)methyl)carbamate (60 mg, 95.0 µmol) and 2,3-dichloro-4-(2-chloro-3-(4,4,5,5-tetramethyl-1,3,2-dioxaborolan-2-yl)phenyl)pyridine (Example 542, step (b)) (87.5 mg, 190 µmol) in 1,4-dioxane / water (1:1, 5 mL) was added potassium carbonate (39.4 mg, 285 µmol) and [1,1'-bis(diphenylphosphino)ferrocene]dichloropalladium(II) complex with dichloromethane (7.77 mg, 9.51 µmol) and the mixture stirred at 110 °C for 4 h under N₂. The mixture was filtered and the filtrate concentrated to afford tert-butyl (S)-(2-(6-(2-chloro-3-(2,3-dichloropyridin-4-yl)phenyl)-3-oxo-1,3-dihydro-2H-pyrrolo [3,4-c]pyridin-2-yl)ethyl)((5-oxopyrrolidin-2-yl)methyl)carbamate (80 mg, 39% yield) as a yellow solid. MS: *m*/*z* found 630 [M+H]⁺.

### (g) tert-Butyl (4-(4-(3-(2-(2-((tert-butoxycarbonyl)(((S)-5-oxopyrrolidin-2-yl)methyl)amino)ethyl)-3-oxo-2,3-dihydro-1H-pyrrolo[3,4-c]pyridin-6-yl)-2-chlorophenyl)-3-chloropyridin-2-yl)-2-methoxybenzyl)(((S)-5-oxopyrrolidin-2-yl)methyl)carbamate

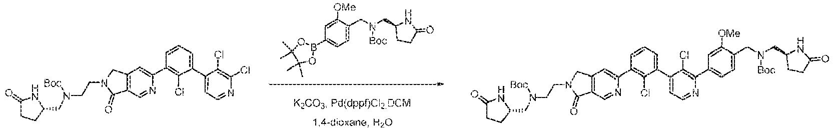

To a mixture of tert-butyl (S)-(2-(6-(2-chloro-3-(2,3-dichloropyridin-4-yl)phenyl)-3-oxo-1,3-dihydro-2H-pyrrolo[3,4-c]pyridin-2-yl)ethyl)((5-oxopyrrolidin-2-yl)methyl)carbamate (60 mg, 95.09 µmol) and tert-butyl (S)-(2-methoxy-4-(4,4,5,5-tetramethyl-1,3,2-dioxaborolan-2-yl)benzyl)((5-oxopyrrolidin-2-yl)methyl)carbamate (Example 542, step (p)) (87.5 mg, 190 µmol) in 1,4-dioxane / water (1:1, 5 mL) was added potassium carbonate (39.4 mg, 285 µmol) and [1,1'-bis(diphenylphosphino)ferrocene]dichloropalladium(II) complex with dichloromethane (7.77 mg, 9.51 µmol) and then the mixture stirred at 110 °C for 4 h under N₂. Anhydrous sodium sulfate was added to dry and the mixture was filtered and concentrated to afford tert-butyl (4-(4-(3-(2-(2-((tert-butoxycarbonyl)(((S)-5-oxopyrrolidin-2-yl)methyl)amino)ethyl)-3-oxo-2,3-dihydro-1H-pyrrolo[3,4-c]pyridin-6-yl)-2-chlorophenyl)-3-chloropyridin-2-yl)-2-methoxybenzyl)(((S)-5-oxopyrrolidin-2-yl)methyl)carbamate (77 mg, 25% yield) as a yellow solid. MS: *m*/*z* found 928 [M+H]⁺.

### (h) 6-(2-Chloro-3-(3-chloro-2-(3-methoxy-4-(((((S)-5-oxopyrrolidin-2-yl)methyl)amino)methyl)phenyl)pyridin-4-yl)phenyl)-2-(2-((((S)-5-oxopyrrolidin-2-yl)methyl)amino)ethyl)-1,2-dihydro-3H-pyrrolo[3,4-c]pyridin-3-one

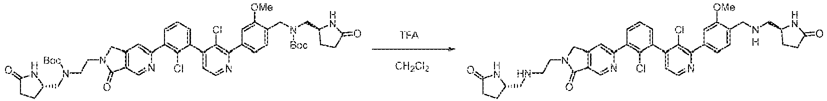

To a mixture of tert-butyl (4-(4-(3-(2-(2-((tert-butoxycarbonyl)(((S)-5-oxopyrrolidin-2-yl)methyl)amino)ethyl)-3-oxo-2,3-dihydro-1H-pyrrolo[3,4-c]pyridin-6-yl)-2-chlorophenyl)-3-chloropyridin-2-yl)-2-methoxybenzyl)(((S)-5-oxopyrrolidin-2-yl)methyl)carbamate (70 mg, 0.756 µmol) in dichloromethane (5 mL) was added trifluoroacetic acid (0.675 mmol, 5.00 ml) and the mixture stirred at room temperature for 0.5 h under N₂. The mixture was concentrated and the residue purified by reverse phase HPLC to afford 6-(2-Chloro-3-(3-chloro-2-(3-methoxy-4-(((((S)-5-oxopyrrolidin-2-yl)methyl)amino)methyl)phenyl)pyridin-4-yl)phenyl)-2-(2-((((S)-5-oxopyrrolidin-2-yl)methyl)amino)ethyl)-1,2-dihydro-3H-pyrrolo[3,4-c]pyridin-3-one (5 mg, 8% yield) as a white solid (formic acid salt). MS: *m*/*z* found 728 [M+H]⁺, retention time = 2.23 min (Method A). ¹H NMR (400 MHz, Methanol-*d*₄): δ 9.06 (s, 1H), 8.68 (d, 1H), 7.96 (d, 1H), 7.71-7.68 (m, 1H), 7.63 (t, 1H), 7.53-7.48 (m, 3H), 7.41 (s, 1H), 7.36 (d, 1H), 4.34 (m, 2H), 4.04-3.94 (m, 8H), 3.30-3.29 (m, 2H), 3.21-3.20 (m, 2H), 3.13-3.12 (m, 2H), 2.43-2.33 (m, 7H), 1.90-1.88 (m, 2H).

### Example 548: (S)-5-((((6-(2-Chloro-3-(3-chloro-2-(3-methoxy-4-(((((S)-5-oxopyrrolidin-2-yl)methyl)amino)methyl)phenyl)pyridin-4-yl)phenyl)imidazo[1,2-a]pyrazin-2-yl)methyl)amino)methyl)pyrrolidin-2-one (291)

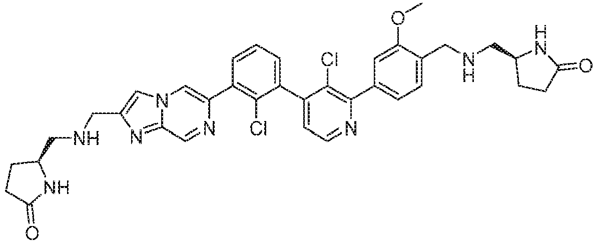

### (a) Ethyl 6-bromoimidazo[1,2-a]pyrazine-2-carboxylate

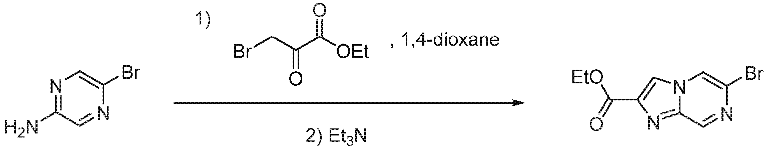

A mixture of 5-bromopyrazin-2-amine (10 g, 57.5 mmol) and ethyl 3-bromo-2-oxopropanoate (12.3 g, 63.2 mmol) in 1,4-dioxane (50 mL) was stirred at 95 °C for 12 h under N₂. Triethylamine (9.6 mL, 69.0 mmol) was then added, and the mixture stirred at 15 °C for 1 h under N₂. The mixture was filtered washing with dichloromethane / ethanol (9/1, 200 mL). The filtrate was concentrated, petroleum ether (10 mL) added to the residue the mixture concentrated under reduced pressure. Water (100 mL) was then added, the mixture filtered and the solid filter-cake purified by normal phase SiO₂ chromatography (0-40 % ethyl acetate/petroleum ether) to afford ethyl 6-bromoimidazo[1,2-a]pyrazine-2-carboxylate (1.9 g, 12% yield) as a yellow solid. ¹H NMR (400 MHz, DMSO-d₆): δ 9.11 (s, 1H), 8.99 (s, 1H), 8.64 (s, 1H), 4.43-4.38 (m, 2H), 1.40-1.37 (m, 3H).

### (b) 6-Bromoimidazo[1,2-a]pyrazine-2-carbaldehyde

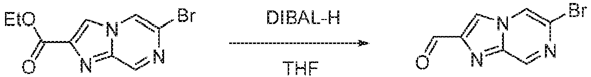

To a mixture of ethyl 6-bromoimidazo[1,2-a]pyrazine-2-carboxylate (1 g, 3.70 mmol) in THF (10 mL) was added diisobutyl aluminium hydride ( 9.26 mL, 1 mol/L, 9.26 mmol) at -78°C under N₂, and the mixture was stirred at -78 °C for 2 h. Saturated aqueous ammonium chloride solution (50 mL) was then added and the mixture extracted with ethyl acetate (2 x 100 mL). The combined organic phase were dried over anhydrous sulfate, filtered and concentrated under reduced pressure to afford 6-bromoimidazo[1,2-a]pyrazine-2-carbaldehyde (400 mg, crude) as a yellow solid. ¹H NMR (400 MHz, DMSO-d₆): δ 10.18 (s, 1H), 9.18 (s, 1H), 9.07 (d, 1H), 8.72 (s, 1H).
The crude product was used in the next step without further purification.

### (c) 6-(2-Chloro-3-(2,3-dichloropyridin-4-yl)phenyl)imidazo[1,2-a]pyrazine-2-carbaldehyde

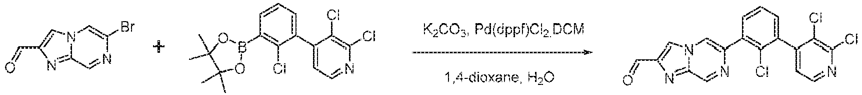

To a mixture of 6-bromoimidazo[1,2-a]pyrazine-2-carbaldehyde (250 mg, 1.11 mmol) and 2,3-dichloro-4-(2-chloro-3-(4,4,5,5-tetramethyl-1,3,2-dioxaborolan-2-yl)phenyl)pyridine (Example 542, step (b)) (595 mg, 1.55 mmol) in 1,4-dioxane / water (5:1, 12 mL) was added potassium carbonate (459 mg, 3.32 mmol) and [1,1'- bis(diphenylphosphino)ferrocene]-dichloropalladium(II) complex with dichloromethane and then the mixture stirred at 110 °C for 1 h under N₂. The mixture was filtered, the filtrate concentrated and the residue purified by normal phase SiO₂ chromatography (0-60 % ethyl acetate/petroleum ether) to give 6-(2-chloro-3-(2,3-dichloropyridin-4-yl)phenyl)imidazo[1,2-a]pyrazine-2-carbaldehyde (200 mg, 44% yield) as a yellow solid. ¹H NMR (400 MHz, DMSO-d₆): δ 10.17 (s, 1H), 9.39 (s, 1H), 9.02 (s, 1H), 8.83 (s, 1H), 8.54 (d, 1H), 7.84 (dd, 1H), 7.68 (t, 1H), 7.60 (d, 1H), 7.59-7.57 (m, 1H).

### (d) 6-(2-chloro-3-(3-chloro-2-(4-formyl-3-methoxyphenyl)pyridin-4-yl)phenyl)imidazo[1,2-a]pyrazine-2-carbaldehyde

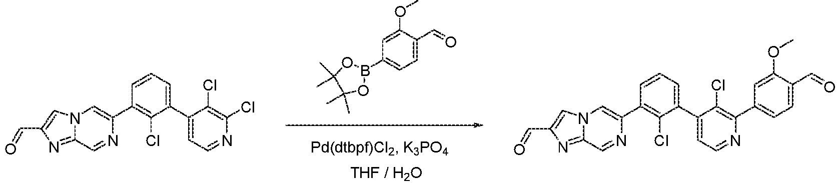

To a mixture of 6-(2-chloro-3-(2,3-dichloropyridin-4-yl)phenyl)imidazo[1,2-a]pyrazine-2-carbaldehyde (150 mg, 0.37 mmol) and 2-methoxy-4-(4,4,5,5-tetramethyl-1,3,2-dioxaborolan-2-yl)benzaldehyde (see Example 77, step (a)) (195 mg, 0.74 mmol) in THF / water (5:1, 6 mL) was added potassium phosphate (237 mg, 1.11 mmol) and [1,1'-bis(di-tert-butylphosphino)ferrocene]dichloropalladium(II) (24.2 mg, 0.04 mmol) and then the mixture was stirred at 80 °C for 1 h under N₂. The mixture was filtered, the filtrate concentrated and the residue purified by prep-TLC (SiO₂, petroleum ether: ethyl acetate = 1:3) to afford 6-(2-chloro-3-(3-chloro-2-(4-formyl-3-methoxyphenyl)pyridin-4-yl)phenyl)imidazo[1,2-a]pyrazine-2-carbaldehyde (80 mg, 42% yield) as a yellow solid. MS: *m*/*z* found 503 [M+H]⁺.

### (e) (S)-5-((((6-(2-Chloro-3-(3-chloro-2-(3-methoxy-4-(((((S)-5-oxopyrrolidin-2-yl)methyl)amino)methyl)phenyl)pyridin-4-yl)phenyl)imidazo[1,2-a]pyrazin-2-yl)methyl)amino)methyl)pyrrolidin-2-one

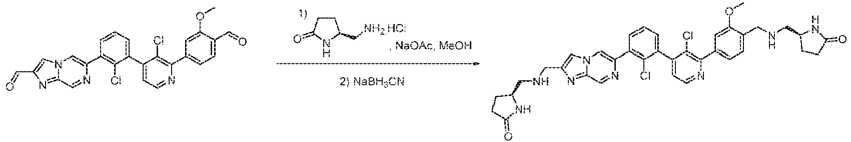

To a mixture of 6-(2-chloro-3-(3-chloro-2-(4-formyl-3-methoxyphenyl)pyridin-4-yl)phenyl)imidazo[1,2-a]pyrazine-2-carbaldehyde (75 mg, 149 µmol), (S)-5-(aminomethyl)pyrrolidin-2-one hydrochloride (89.8 mg, 596 µmol) in methanol (5 mL) was added sodium acetate (73.3 mg, 894 µmol) and then the mixture was stirred at room temperature for 3 h under N₂. Sodium cyanoborohydride (46.8 mg, 745 µmol) was then added and the mixture stirred at room temperature for 0.5 h under N₂. The mixture was concentrated and the residue purified by reverse phase HPLC to afford (S)-5-((((6-(2-chloro-3-(3-chloro-2-(3-methoxy-4-(((((S)-5-oxopyrrolidin-2-yl)methyl)amino)methyl)phenyl)pyridin-4-yl)phenyl)imidazo[1,2-a]pyrazin-2-yl)methyl)amino)methyl)pyrrolidin-2-one (15.1 mg, 13% yield) as a white solid (formic acid salt). MS: *m*/*z* found 699 [M+H]⁺, retention time = 2.10 min (Method A). ¹H NMR (400 MHz, Methanol-*d*₆): δ 9.05 (s, 1H), 8.78 (d, 1H), 8.67 (d, 1H), 8.31 (brs, 2H), 8.08 (s, 1H), 7.76 (dd, 1H), 7.61 (t, 1H), 7.51-7.47 (m, 3H), 7.40-7.34 (m, 2H), 4.29-4.13 (m, 4H), 4.01-4.00 (m, 4H), 3.89-3.87 (m, 1H), 3.13-3.09 (m, 2H), 2.87-2.83 (m, 2H), 2.41-2.28 (m, 6H), 1.89-1.84 (m, 2H).

### Example 549: (S)-2-((6-(2-Chloro-3-(3-chloro-2-(3-methoxy-4-((((5-oxopyrrolidin-2-yl)methyl)amino)methyl)phenyl)pyridin-4-yl)phenyl)-1-methyl-1H-pyrrolo[2,3-b]pyridin-3-yl)methyl)-2,6-diazaspiro[3.4]octan-7-one (219)

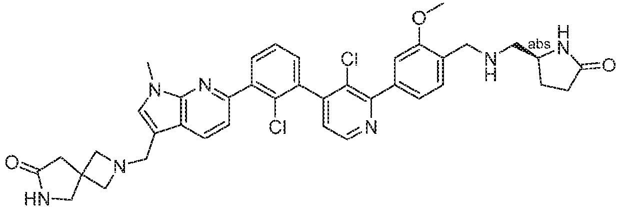

Reductive Amination Procedure B from tert-butyl (S)-(4-(3-chloro-4-(2-chloro-3-(3-formyl-1-methyl-1H-pyrrolo[2,3-b]pyridin-6-yl)phenyl)pyridin-2-yl)-2-methoxybenzyl)((5 - oxopyrrolidin-2-yl)methyl)carbamate (60 mg, 0.08 mmol) and 2,6-diazaspiro[3.4]octan-7-one (21 mg, 0.17 mmol) gave tert-butyl (S)-(4-(3-chloro-4-(2-chloro-3-(1-methyl-3-((7-oxo-2,6-diazaspiro[3.4]octan-2-yl)methyl)-1H-pyrrolo[2,3-b]pyridin-6-yl)phenyl)pyridin-2-yl)-2-methoxybenzyl)((5-oxopyrrolidin-2-yl)methyl)carbamate as crude, MS: m/z found 824.3 [M+H]⁺, which was deprotected and purified by General Boc Deprotection Procedure to afford (S)-2-((6-(2-chloro-3-(3-chloro-2-(3-methoxy-4-((((5-oxopyrrolidin-2-yl)methyl)amino)methyl)phenyl)pyridin-4-yl)phenyl)-1-methyl-1H-pyrrolo[2,3-b]pyridin-3-yl)methyl)-2,6-diazaspiro[3.4]octan-7-one as a formic acid salt. 67% yield over two steps. LCMS: m/z found 724.3 [M+H]⁺, retention time = 1.61 min (Method D). ¹H NMR (400 MHz, Methanol-*d*₄): δ 8.66 (dd, 1H), 8.43 (s, 2H), 8.23 (d, 1H), 7.75 - 7.68 (m, 1H), 7.65 - 7.55 (m, 2H), 7.51 - 7.42 (m, 4H), 7.37 (s, 1H), 7.33 (d, 1H), 4.31 (s, 2H), 4.23 - 4.11 (m, 2H), 4.01 - 3.95 (m, 4H), 3.92 (s, 3H), 3.89 (s, 4H), 3.62 (s, 2H), 3.08 - 2.95 (m, 2H), 2.65 (s, 2H), 2.42 - 2.28 (m, 3H), 1.94 - 1.80 (m, 1H).

### Example 550: 1-(4-((4-(4-(3-(3-(((1-acetylpiperidin-4-yl)amino)methyl)-1-methyl-1H-pyrrolo[2,3-b]pyridin-6-yl)-2-chlorophenyl)-3-chloropyridin-2-yl)-2-methoxybenzyl)amino)piperidin-1-yl)ethan-1-one (246)

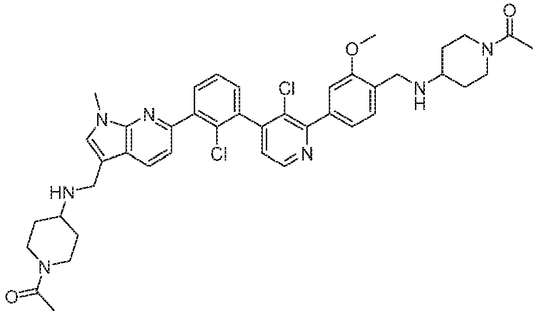

### (a) 6-(2-Chloro-3-(3-chloro-2-(4-formyl-3-methoxyphenyl)pyridin-4-yl)phenyl)-1-methyl-1H-pyrrolo[2,3-b]pyridine-3-carbaldehyde

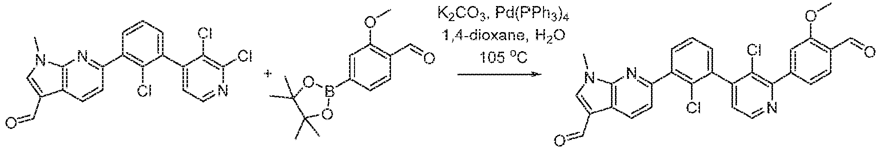

To a mixture of 6-(2-chloro-3-(2,3-dichloropyridin-4-yl)phenyl)-1-methyl-1H-pyrrolo[2,3-b]pyridine-3-carbaldehyde (100 mg, 0.24 mmol), 2-methoxy-4-(4,4,5,5-tetramethyl-1,3,2-dioxaborolan-2-yl)benzaldehyde (63 mg, 0.24 mmol) and potassium carbonate (100 mg, 0.72 mmol) in degassed 1,4-dioxane/water (6:1) was added tetrakis(triphenylphosphine)palladium(0) (28 mg, 0.02 mmol) and the mixture was stirred at 105 °C for 30 min. Water was added and the mixture was extracted into EtOAc three times. The combined organic layers were dried over anhydrous sodium sulfate, concentrated, and purified on silica gel column to provide 6-(2-chloro-3-(3-chloro-2-(4-formyl-3-methoxyphenyl)pyridin-4-yl)phenyl)-1-methyl-1H-pyrrolo[2,3-b]pyridine-3-carbaldehyde (60 mg, 48%). LCMS: m/z found 516.1 [M+H]⁺, retention time = 1.01 min (Method B).

### (b) 1-(4-((4-(4-(3-(3-(((1-Acetylpiperidin-4-yl)amino)methyl)-1-methyl-1H-pyrrolo[2,3-b]pyridin-6-yl)-2-chlorophenyl)-3-chloropyridin-2-yl)-2-methoxybenzyl)amino)piperidin-1-yl)ethan-1-one

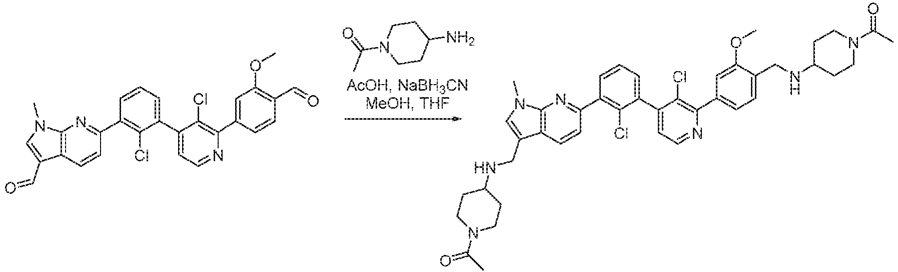

1-(4-((4-(4-(3-(3-(((1-Acetylpiperidin-4-yl)amino)methyl)-1-methyl-1H-pyrrolo[2,3-b]pyridin-6-yl)-2-chlorophenyl)-3-chloropyridin-2-yl)-2-methoxybenzyl)amino)piperidin-1-yl)ethan-1-one was prepared as the formic acid salt following Reductive Amination Procedure A from 6-(2-chloro-3-(3-chloro-2-(4-formyl-3-methoxyphenyl)pyridin-4-yl)phenyl)-1-methyl-1H-pyrrolo[2,3-b]pyridine-3-carbaldehyde (40 mg, 0.08 mmol) and 1-(4-amino-1-piperidyl)ethanone (44 mg, 0.31 mmol). 76% yield. LCMS: m/z found 768.3 [M+H]⁺, retention time = 1.74 min (Method D). ¹H NMR (400 MHz, Methanol-*d*₄): δ 8.67 (d, 1H), 8.50 (s, 2H), 8.25 (d, 1H), 7.72 (d, 1H), 7.67 (s, 1H), 7.60 (t, 1H), 7.53 (d, 1H), 7.51 - 7.44 (m, 3H), 7.39 (s, 1H), 7.35 (dd, 1H), 4.65 (d, 2H), 4.43 (s, 2H), 4.27 (s, 2H), 4.06 (d, 2H), 3.99 (s, 3H), 3.94 (s, 3H), 3.44 - 3.37 (m, 2H), 3.25 - 3.11 (m, 2H), 2.69 (t, 2H), 2.29 - 2.15 (m, 4H), 2.13 (s, 6H), 1.69 - 1.42 (m, 4H).

### Example 551: 1-(4-((4-(4-(3-(3-(((1-acetylpiperidin-4-yl)(methyl)amino)methyl)-1-methyl-1H-pyrrolo[2,3-b]pyridin-6-yl)-2-chlorophenyl)-3-chloropyridin-2-yl)-2-methoxybenzyl)amino)piperidin-1-yl)ethan-1-one (264)

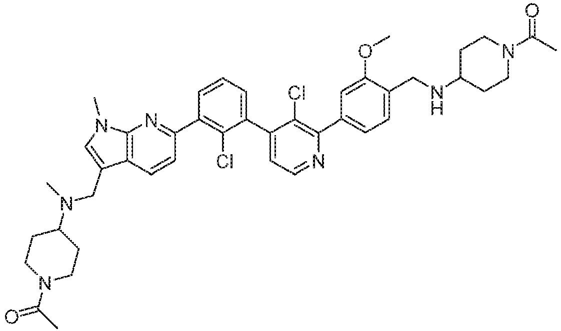

### (a) tert-Butyl (1-acetylpiperidin-4-yl)(4-(3-chloro-4-(2-chloro-3-(3-formyl-1-methyl-1H-pyrrolo[2,3-b]pyridin-6-yl)phenyl)pyridin-2-yl)-2-methoxybenzyl)carbamate

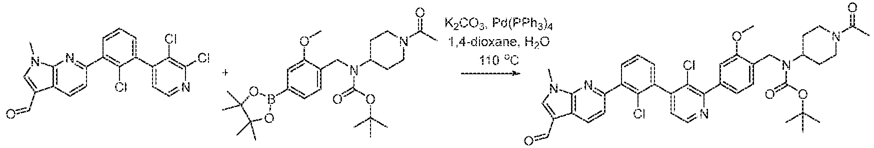

To a mixture of 6-(2-chloro-3-(2,3-dichloropyridin-4-yl)phenyl)-1-methyl-1H-pyrrolo[2,3-b]pyridine-3-carbaldehyde (140 mg, 0.34 mmol), tert-butyl (1-acetylpiperidin-4-yl)(2-methoxy-4-(4,4,5,5-tetramethyl-1,3,2-dioxaborolan-2-yl)benzyl)carbamate (164 mg, 0.34 mmol) and potassium carbonate (139 mg, 1.01 mmol) in degassed 1,4-dioxane/water (6:1) was added tetrakis(triphenylphosphine)palladium(0) (39 mg, 0.03 mmol) and the mixture was stirred at 110 °C for 30 min. Water was added and the mixture was extracted into EtOAc three times. The combined organic layers were dried over anhydrous sodium sulfate, concentrated, and purified on silica gel column using 2% MeOH in dichloromethane as eluent to provide tert-butyl (1-acetylpiperidin-4-yl)(4-(3-chloro-4-(2-chloro-3-(3-formyl-1-methyl-1H-pyrrolo[2,3-b]pyridin-6-yl)phenyl)pyridin-2-yl)-2-methoxybenzyl)carbamate (130 mg, 52%). LCMS: m/z found 742.3 [M+H]⁺, retention time = 1.05 min (Method B).

### (b) 1-(4-((4-(4-(3-(3-(((1-Acetylpiperidin-4-yl)(methyl)amino)methyl)-1-methyl-1H-pyrrolo[2,3-b]pyridin-6-yl)-2-chlorophenyl)-3-chloropyridin-2-yl)-2-methoxybenzyl)amino)piperidin-1-yl)ethan-1-one

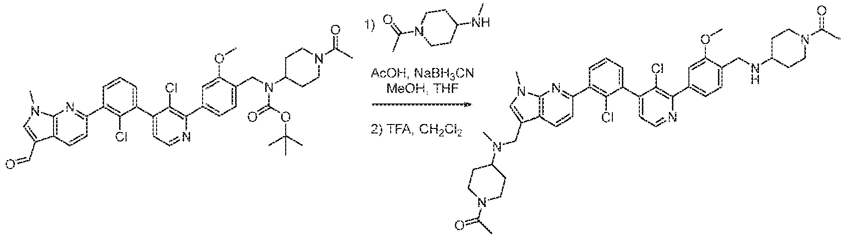

Reductive Amination Procedure B from tert-butyl (1-acetylpiperidin-4-yl)(4-(3-chloro-4-(2-chloro-3-(3-formyl-1-methyl-1H-pyrrolo[2,3-b]pyridin-6-yl)phenyl)pyridin-2-yl)-2-methoxybenzyl)carbamate (100 mg, 0.13 mmol) and 1-[4-(methylamino)-1-piperidyl]ethanone (42 mg, 0.27 mmol) gave tert-butyl (1-acetylpiperidin-4-yl)(4-(4-(3-(3-(((1-acetylpiperidin-4-yl)(methyl)amino)methyl)-1-methyl-1H-pyrrolo[2,3-b]pyridin-6-yl)-2-chlorophenyl)-3-chloropyridin-2-yl)-2-methoxybenzyl)carbamate as crude, MS: m/z found 882.4 [M+H]⁺, which was deprotected and purified by General Boc Deprotection Procedure to afford 1-(4-((4-(4-(3-(3-(((1-acetylpiperidin-4-yl)(methyl)amino)methyl)-1-methyl-1H-pyrrolo[2,3-b]pyridin-6-yl)-2-chlorophenyl)-3-chloropyridin-2-yl)-2-methoxybenzyl)amino)piperidin-1-yl)ethan-1-one as formic acid salt. 28% yield over two steps. LCMS: m/z found 782.3 [M+H]⁺, retention time = 1.74 min (Method D). ¹H NMR (400 MHz, Methanol-*d*₄): δ 8.70 - 8.64 (m, 1H), 8.52 - 8.39 (m, 2H), 8.23 (d, 1H), 7.72 (d, 1H), 7.69 - 7.56 (m, 2H), 7.53 (d, 1H), 7.51 - 7.43 (m, 3H), 7.41 - 7.38 (m, 1H), 7.38 - 7.33 (m, 1H), 4.71 - 4.60 (m, 2H), 4.38 - 4.24 (m, 3H), 4.20 - 3.78 (m, 9H), 3.48 - 3.31 (m, 2H), 3.27 - 3.07 (m, 2H), 2.79 - 2.53 (m, 5H), 2.36 - 2.03 (m, 10H), 1.88 - 1.40 (m, 4H).

### Example 552: 1-(4-((4-(3-Chloro-4-(2-chloro-3-(1-methyl-3-(((tetrahydro-2H-pyran-4-yl)amino)methyl)-1H-pyrrolo[2,3-b]pyridin-6-yl)phenyl)pyridin-2-yl)-2-methoxybenzyl)amino)piperidin-1-yl)ethan-1-one (556)

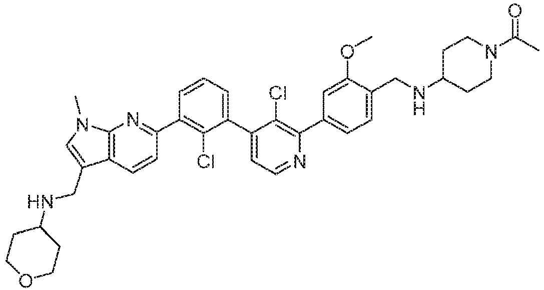

### (a) 6-(3-Bromo-2-chlorophenyl)-1-methyl-1H-pyrrolo[2,3-b]pyridine-3-carbaldehyde

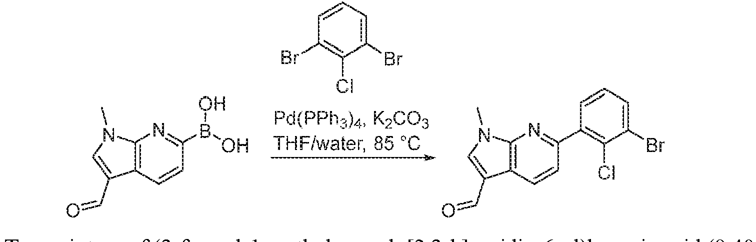

To a mixture of (3-formyl-1-methyl-pyrrolo[2,3-b]pyridin-6-yl)boronic acid (7.40 g, 46.1 mmol) and 1,3-dibromo-2-chlorobenzene (18.70 g, 69.1 mmol) in THF/H₂O (5/1, 120 mL) was added tetrakis(triphenylphosphine)palladium(0) (5.32 g, 4.61 mmol) and potassium carbonate (19.10 g, 138 mmol) in one portion under N₂. The mixture was stirred at 85 °C for 3 hr. To the mixture was added saturated aqueous brine solution (100 mL). The mixture was extracted with ethyl acetate (300 mL). The organic layer was dried over anhydrous sodium sulfate, filtered and concentrated under reduced pressure. The residue was purified by normal phase SiO₂ chromatography (0-100% ethyl acetate/petroleum ether) to afford 6-(3-bromo-2-chlorophenyl)-1-methyl-1H-pyrrolo[2,3-b]pyridine-3-carbaldehyde (6.10 g, 38% yield).

### (b) 6-(2-chloro-3-(2,3-dichloropyridin-4-yl)phenyl)-1-methyl-1H-pyrrolo[2,3-b]pyridine-3-carbaldehyde

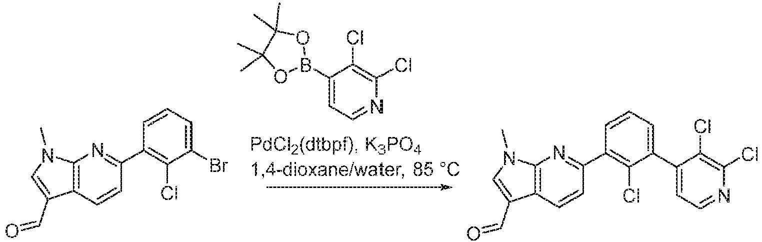

To a mixture of 6-(3-bromo-2-chlorophenyl)-1-methyl-1H-pyrrolo[2,3-b]pyridine-3-carbaldehyde (4.10 g, 11.3 mmol) and 2,3-dichloro-4-(4,4,5,5-tetramethyl-1,3,2-dioxaborolan-2 -yl)pyridine (3.86 g, 14.1 mmol) in 1,4-dioxane/H₂O (5/1, 48 mL) was added potassium phosphate (7.47 g, 35.2 mmol) and [1,1'-bis(di-tert-butylphosphino)ferrocene]dichloropalladium(II) (764 mg, 1.17 mmol) in one portion under N₂. The mixture was stirred at 85 °C for 3 hr. The mixture was combined with another batch at 540 mg scale. Water (30 mL) was added, and the mixture extracted with ethyl acetate (130 mL). The organic layer was concentrated. The residue was purified by normal phase SiO₂ chromatography (0-10% ethyl acetate/petroleum ether) to afford 6-(2-chloro-3-(2,3-dichloropyridin-4-yl)phenyl)-1-methyl-1H-pyrrolo[2,3-b]pyridine-3-carbaldehyde as a yellow solid (2.20 g, 40% yield). ¹H NMR (400 MHz, Chloroform-d): δ 9.94 (s, 1H), 8.56 (d, 1H), 8.31 (d, 1H), 7.84 (s, 1H), 7.66 (dd, 1H), 7.55 (t, 1H), 7.44 (t, 1H), 7.22 (dd, 1H), 7.17 (d, 1H), 3.95 (s, 3H).

### (c) tert-Butyl (1-acetylpiperidin-4-yl)(4-(3-chloro-4-(2-chloro-3-(3-formyl-1-methyl-1H-pyrrolo[2,3-b]pyridin-6-yl)phenyl)pyridin-2-yl)-2-methoxybenzyl)carbamate

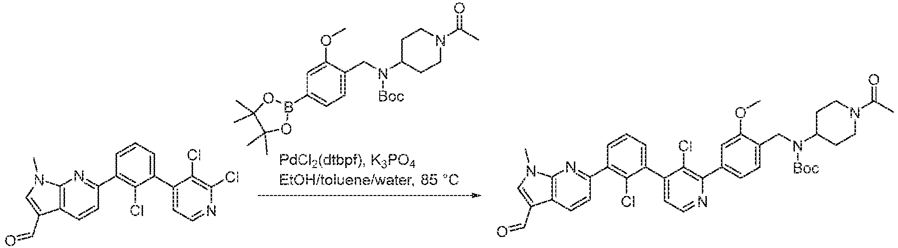

To a mixture of 6-(2-chloro-3-(2,3-dichloropyridin-4-yl)phenyl)-1-methyl-1H-pyrrolo [2,3-b]pyridine-3-carbaldehyde (400 mg, 0.96 mmol) and tert-butyl (1-acetylpiperidin-4-yl)(2-methoxy-4-(4,4,5,5-tetramethyl-1,3,2-dioxaborolan-2-yl)benzyl)carbamate (563 mg, 1.15 mmol) in EtOH/Toluene/H₂O (2/2/1, 5 mL) was added potassium phosphate (408 mg, 1.92 mmol) and [1,1'-bis(di-tert-butylphosphino)ferrocene]dichloropalladium(II) (63 mg, 0.10 mmol) in one portion under N₂. The mixture was stirred at 85 °C for 12 hr. The mixture combined with another batch at 200 mg. Water (20 mL) was added and the mixture extracted with ethyl acetate (100 mL). The organic layer was concentrated under vacuum. The residue was purified twice by normal phase SiO₂ chromatography (0-100% ethyl acetate/petroleum ether) to afford tert-butyl (1-acetylpiperidin-4-yl)(4-(3-chloro-4-(2-chloro-3-(3-formyl-1-methyl-1H-pyrrolo[2,3-b]pyridin-6-yl)phenyl)pyridin-2-yl)-2-methoxybenzyl)carbamate (340 mg, 63% purity). MS: m/z found 742 [M+H]⁺.

### (d) 1-(4-((4-(3-Chloro-4-(2-chloro-3-(1-methyl-3-(((tetrahydro-2H-pyran-4-yl)amino)methyl)-1H-pyrrolo[2,3-b]pyridin-6-yl)phenyl)pyridin-2-yl)-2-methoxybenzyl)amino)piperidin-1-yl)ethan-1-one

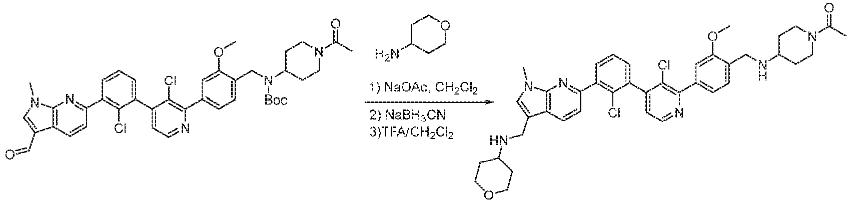

1-(4-((4-(3-Chloro-4-(2-chloro-3-(1-methyl-3-(((tetrahydro-2H-pyran-4-yl)amino)methyl)-1H-pyrrolo[2,3-b]pyridin-6-yl)phenyl)pyridin-2-yl)-2-methoxybenzyl)amino)piperidin-1-yl)ethan-1-one was prepared as the formic acid salt, following Reductive Amination Procedure B from tert-butyl (1-acetylpiperidin-4-yl)(4-(3-chloro-4-(2-chloro-3-(3-formyl-1-methyl-1H-pyrrolo[2,3-b]pyridin-6-yl)phenyl)pyridin-2-yl)-2-methoxybenzyl)carbamate (75 mg, 0.10 mmol) and tetrahydro-2H-pyran-4-amine (31 mg, 0.30 mmol), followed by General Boc Deprotection Procedure. 18% yield. LCMS: m/z found 727.1 [M+H]⁺, retention time = 2.86 min, (Method A); ¹H NMR (400 MHz, DMSO-*d*₆)*:* δ 8.68 (d, 1H), 8.53 (br s, 1H), 8.25 (d, 1H), 7.74 (dd, 1H), 7.66 (s, 1H), 7.61 (t, 1H), 7.52 - 7.47 (m, 4H), 7.38 (s, 1H), 7.35 (d, 1H), 4.60 (s, 2H), 4.42 (s, 2H), 4.18 (s, 2H), 4.09-4.02 (m, 3H), 3.99 (s, 3H), 3.96 (s, 3H), 3.51 - 3.45 (m, 3H), 3.23 - 3.15 (m, 2H), 2.71 - 2.68 (m, 1H), 2.18 - 2.11 (m, 7H), 1.72 - 1.67 (m, 1H), 1.57 - 1.44 (m, 2H).

### Example 553: 1-(4-((4-(3-chloro-4-(2-chloro-3-(1-methyl-3-((((tetrahydro-2H-pyran-4-yl)methyl)amino)methyl)-1H-pyrrolo[2,3-b]pyridin-6-yl)phenyl)pyridin-2-yl)-2-methoxybenzyl)amino)piperidin-1-yl)ethan-1-one (557)

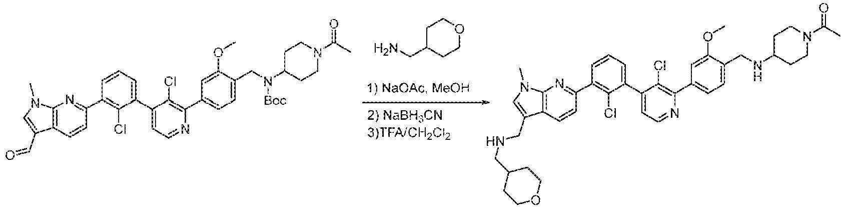

1-(4-((4-(3-Chloro-4-(2-chloro-3-(1-methyl-3-((((tetrahydro-2H-pyran-4-yl)methyl)amino)methyl)-1H-pyrrolo[2,3-b]pyridin-6-yl)phenyl)pyridin-2-yl)-2-methoxybenzyl)amino)piperidin-1-yl)ethan-1-one was prepared as the formic acid salt, following Reductive Amination Procedure B from tert-butyl (1-acetylpiperidin-4-yl)(4-(3-chloro-4-(2-chloro-3-(3-formyl-1-methyl-1H-pyrrolo[2,3-b]pyridin-6-yl)phenyl)pyridin-2-yl)-2-methoxybenzyl)carbamate (Example 552, step (c)) (75 mg, 0.10 mmol) and (tetrahydro-2H-pyran-4-yl)methanamine (35 mg, 0.20 mmol), followed by General Boc Deprotection Procedure. 6% yield. LCMS: m/z found 742.4 [M+H]⁺, retention time = 2.90 min, (Method A); ¹H NMR (400 MHz, Methanol-*d*₄): δ 8.68 (d, 1H), 8.52 (br s, 2H), 8.27 (d, 1H), 7.74 (dd, 1H), 7.69 (s, 1H), 7.61 (t, 1H), 7.53-7.47 (m, 4H), 7.39 (s, 1H), 7.35 (d, 1H), 4.64 (s, 1H), 4.60 (s, 3H), 4.44 (s, 2H), 4.22 (s, 2H), 4.08 - 4.03 (m, 1H), 3.98 (d, 7H), 3.50 - 3.41 (m, 3H), 3.24 - 3.15 (m, 1H), 2.98 (d, 2H), 2.74 - 2.68 (m, 1H), 2.23 - 2.14 (m, 5H), 2.00 - 1.98 (m, 1H), 1.72 (d, 2H), 1.47 - 1.32 (m, 2H).

### Example 554: 1-(4-((4-(3-Chloro-4-(2-chloro-3-(3-(((1,1-dioxidotetrahydro-2H-thiopyran-4-yl)amino)methyl)-1-methyl-1H-pyrrolo[2,3-b]pyridin-6-yl)phenyl)pyridin-2-yl)-2-methoxybenzyl)amino)piperidin-1-yl)ethan-1-one (558)

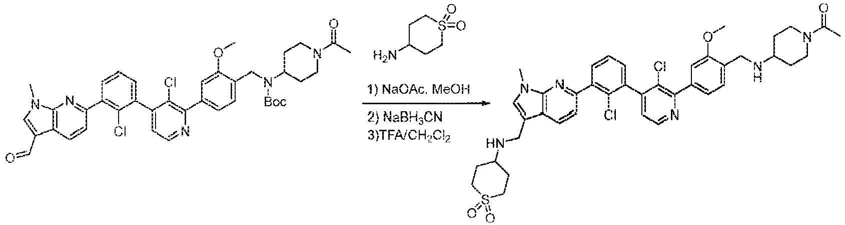

1-(4-((4-(3-Chloro-4-(2-chloro-3-(3-(((1,1-dioxidotetrahydro-2H-thiopyran-4-yl)amino)methyl)-1-methyl-1H-pyrrolo[2,3-b]pyridin-6-yl)phenyl)pyridin-2-yl)-2-methoxybenzyl)amino)piperidin-1-yl)ethan-1-one was prepared as the formic acid salt, following Reductive Amination Procedure B from tert-butyl (1-acetylpiperidin-4-yl)(4-(3-chloro-4-(2-chloro-3-(3-formyl-1-methyl-1H-pyrrolo[2,3-b]pyridin-6-yl)phenyl)pyridin-2-yl)-2-methoxybenzyl)carbamate (Example 552, step (c)) (75 mg, 0.10 mmol) and 4-aminotetrahydro-2H-thiopyran 1,1-dioxide hydrochloride (28 mg, 0.15 mmol), followed by General Boc Deprotection Procedure. 16% yield. LCMS: m/z found 775.3 [M+H]⁺, retention time = 2.74 min (Method A); ¹H NMR (400 MHz, Methanol-*d*₄): δ 8.68 (d, 1H), 8.49 (br s, 1H), 8.21 (d, 1H), 7.73 (dd, 1H), 7.60 (t, 1H), 7.54 (d, 1H), 7.51 (d, 1H), 7.48 (s, 1H), 7.46 (dd, 1H), 7.41 - 7.35 (m, 3H), 4.68 - 4.65 (m, 1H), 4.60 (s, 1H), 4.28 (s, 2H), 4.11 - 4.06 (m, 3H), 4.01 (s, 3H), 3.91 (s, 3H), 3.50 (t, 1H), 3.25 - 3.19 (m, 3H), 3.16 - 3.07 (m, 4H), 2.74 - 2.67 (m, 1H), 2.38 - 2.33 (m, 2H), 2.23 - 2.10 (m, 7H).

### Example 555: 1-(4-((4-(3-Chloro-4-(2-chloro-3-(3-(((2-hydroxyethyl)amino)methyl)-1-methyl-1H-pyrrolo[2,3-b]pyridin-6-yl)phenyl)pyridin-2-yl)-2-methoxybenzyl)amino)piperidin-1-yl)ethan-1-one (559)

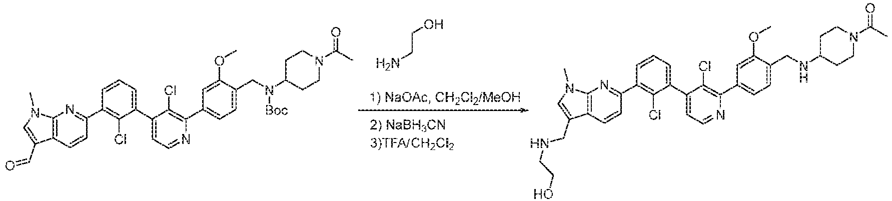

1-(4-((4-(3-Chloro-4-(2-chloro-3-(3-(((2-hydroxyethyl)amino)methyl)-1-methyl-1H-pyrrolo[2,3-b]pyridin-6-yl)phenyl)pyridin-2-yl)-2-methoxybenzyl)amino)piperidin-1-yl)ethan-1-one was prepared as the formic acid salt, following Reductive Amination Procedure B from tert-butyl (1-acetylpiperidin-4-yl)(4-(3-chloro-4-(2-chloro-3-(3-formyl-1-methyl-1H-pyrrolo[2,3-b]pyridin-6-yl)phenyl)pyridin-2-yl)-2-methoxybenzyl)carbamate (Example 552, step (c)) (75 mg, 0.10 mmol) and 2-aminoethan-1-ol (12 mg, 0.20 mmol), followed by General Boc Deprotection Procedure. 10% yield. LCMS: m/z found 688.9 [M+H]⁺, retention time = 2.65 min (Method A); ¹H NMR (400 MHz, Methanol-*d*₄): δ 8.88 (d, 1H), 8.52 (br s, 2H), 8.27 (d, 1H), 7.74 (dd, 1H), 7.69 (s, 1H), 7.61 (t, 1H), 7.53 - 7.45 (m, 4H), 7.39 (s, 1H), 7.35 (d, 1H), 4.65 - 4.60 (m, 1H), 4.47 (s, 2H), 4.22 (s, 2H), 4.07 - 4.05 (m, 2H), 4.00 (s, 3H), 3.96 (s, 3H), 3.84 (m, 2H), 3.24 - 3.15 (m, 4H), 2.75 - 2.68 (m, 1H), 2.23 - 2.19 (m, 2H), 2.14 (s, 3H), 1.60 - 1.58 (m, 1H), 1.50 - 1.46 (m, 1H).

### Example 556: 1-(4-(((6-(2-Chloro-3-(3-chloro-2-(4-(((2-hydroxyethyl)amino)methyl)-3-methoxyphenyl)pyridin-4-yl)phenyl)-1-methyl-1H-pyrrolo[2,3-b]pyridin-3-yl)methyl)amino)piperidin-1-yl)ethan-1-one (560)

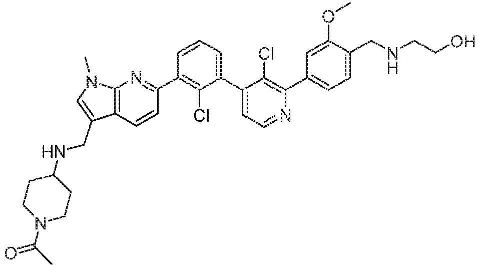

### (a) tert-Butyl (1-acetylpiperidin-4-yl)((6-(2-chloro-3-(2,3-dichloropyridin-4-yl)phenyl)-1-methyl-1H-pyrrolo[2,3-b]pyridin-3-yl)methyl)carbamate

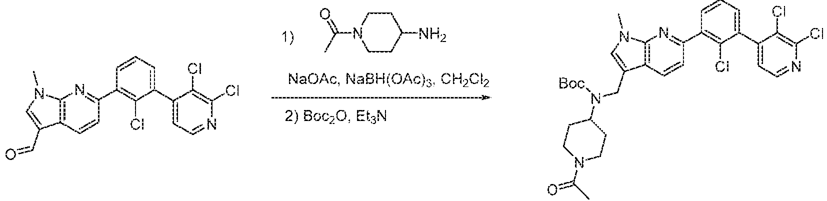

To a mixture of 6-(2-chloro-3-(2,3-dichloropyridin-4-yl)phenyl)-1-methyl-1H-pyrrolo[2,3-b]pyridine-3-carbaldehyde (0.30 g, 0.72 mmol) and 1-(4-aminopiperidin-1-yl)ethanone (0.13 g, 0.94 mmol) in CH₂Cl₂ (40 mL) was added sodium acetate (0.18 g, 2.16 mmol) and sodium triacetoxyborohydride (0.46 g, 2.16 mmol) in one portion under N₂. The mixture was stirred at room temperature for 6 h. To the above solution was added di-tert-butyl dicarbonate (0.47 g, 2.16 mmol) and triethylamine (1.00 mL, 0.72 mmol) in one portion under N₂. The mixture was stirred at room temperature for 1 h. To the mixture was added water (20 mL). The mixture was extracted with CH₂Cl₂ (100 mL). The organic phase was washed with saturated aqueous brine solution (2 x 50 mL), dried with anhydrous sodium sulfate, filtered and concentrated. The residue was purified by normal phase SiO₂ chromatography (0-100% ethyl acetate/petroleum ether) to afford tert-butyl (1-acetylpiperidin-4-yl)((6-(2-chloro-3-(2,3-dichloropyridin-4-yl)phenyl)-1-methyl-1H-pyrrolo[2,3-b]pyridin-3-yl)methyl)carbamate (0.80 g). ¹H NMR (400 MHz, Methanol-*d*₄): δ 8.29 (d, 1H), 8.03 (d, 1H), 7.60 (dd, 1H), 7.46 (t, 1H), 7.32 - 7.27 (m, 3H), 7.22 (d, 1H), 4.42 - 4.39 (m, 2H), 3.89 - 3.83 (m, 1H), 3.76 (s, 3H), 3.73 - 3.61 (m, 4H), 2.42 - 2.36 (m, 2H), 2.18 - 2.10 (m, 2H), 1.98 (s, 3H), 1.40 (s, 9H).

### (b) tert-Butyl (1-acetylpiperidin-4-yl)((6-(2-chloro-3-(3-chloro-2-(4-formyl-3-methoxyphenyl)pyridin-4-yl)phenyl)-1-methyl-1H-pyrrolo[2,3-b]pyridin-3-yl)methyl)carbamate

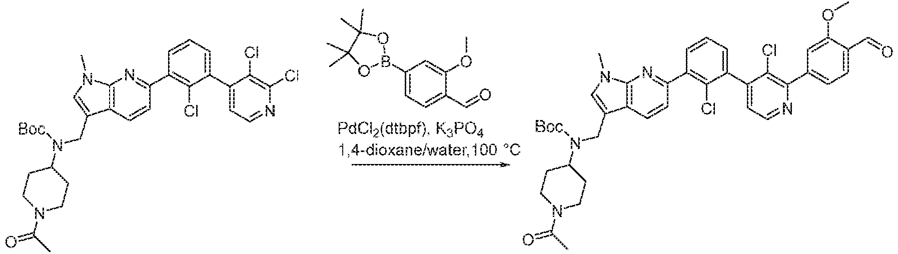

To a mixture of tert-butyl (1-acetylpiperidin-4-yl)((6-(2-chloro-3-(2,3-dichloropyridin-4-yl)phenyl)-1-methyl-1H-pyrrolo[2,3-b]pyridin-3-yl)methyl)carbamate (0.79 g, 1.23 mmol) and 2-methoxy-4-(4,4,5,5-tetramethyl-1,3,2-dioxaborolan-2-yl)benzaldehyde (0.39 g, 1.47 mmol) in 1,4-dioxane/H₂O (10:1, 22 mL) was added [1,1'-bis(di-tert-butylphosphino)ferrocene]dichloropalladium(II) (0.06 g, 0.09 mmol) and potassium phosphate (0.78 g, 3.69 mmol) in one portion under N₂. The mixture was stirred at 100 °C for 12 h. The mixture was combined with another batch at 50 mg scale. The mixture was concentrated. To the residue was added water (50 mL) and saturated aqueous brine solution (50 mL). The mixture was extracted with ethyl acetate/THF mixture (1:1, 100 mL). The organic phase was dried with anhydrous sodium sulfate, filtered and concentrated. The residue was purified by normal phase SiO₂ chromatography (20-100% ethyl acetate/petroleum ether) to afford tert-butyl (1-acetylpiperidin-4-yl)((6-(2-chloro-3-(3-chloro-2-(4-formyl-3-methoxyphenyl)pyridin-4-yl)phenyl)-1-methyl-1H-pyrrolo[2,3-b]pyridin-3-yl)methyl)carbamate as a yellow solid (0.38 g, 35% yield). MS: m/z found 742.3 [M+H]⁺.

### (c) 1-(4-(((6-(2-Chloro-3-(3-chloro-2-(4-(((2-hydroxyethyl)amino)methyl)-3-methoxyphenyl)pyridin-4-yl)phenyl)-1-methyl-1H-pyrrolo[2,3-b]pyridin-3-yl)methyl)amino)piperidin-1-yl)ethan-1-one

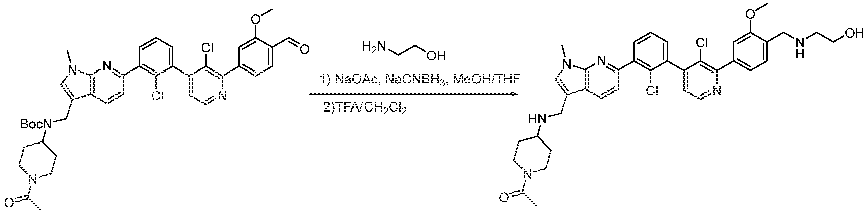

1-(4-(((6-(2-Chloro-3-(3-chloro-2-(4-(((2-hydroxyethyl)amino)methyl)-3-methoxyphenyl)pyridin-4-yl)phenyl)-1-methyl-1H-pyrrolo[2,3-b]pyridin-3-yl)methyl)amino)piperidin-1-yl)ethan-1-one was prepared as the formic acid salt, following Reductive Amination Procedure B from tert-butyl (1-acetylpiperidin-4-yl)((6-(2-chloro-3-(3-chloro-2-(4-formyl-3-methoxyphenyl)pyridin-4-yl)phenyl)-1-methyl-1H-pyrrolo[2,3-b]pyridin-3-yl)methyl)carbamate (120 mg, 0.16 mmol) and 1-(4-amino-1-piperidyl)propan-1-one (30 mg, 0.48 mmol), followed by General Boc Deprotection Procedure. 17% yield. LCMS: m/z found 688.4 [M+H]⁺, retention time = 2.59 min (Method A); ¹H NMR (400 MHz, Methanol-*d*₄): δ 8.68 (d, 1H), 8.22 (d, 1H), 7.74 - 7.72 (dd, 1H), 7.61 (t, 1H), 7.54 - 7.46 (m, 4H), 7.42 (d, 1H), 7.39 (s, 1H), 7.36 - 7.34 (m, 1H), 4.58 - 4.54 (m, 1H), 4.22 - 4.20 (m, 4H), 4.00 - 3.98 (m, 4H), 3.92 (s, 3H), 3.82 (t, 2H), 3.21 - 3.13 (m, 1H), 3.08 - 3.05 (m, 3H), 2.75 - 2.69 (m, 5H), 1.50-1.37 (m, 2H).

### Example 557: 1-(4-(((6-(3-(2-(4-((6-Acetyl-2,6-diazaspiro[3.3]heptan-2-yl)methyl)-3-methoxyphenyl)-3-chloropyridin-4-yl)-2-chlorophenyl)-1-methyl-1H-pyrrolo[2,3-b]pyridin-3-yl)methyl)amino)piperidin-1-yl)ethan-1-one (564)

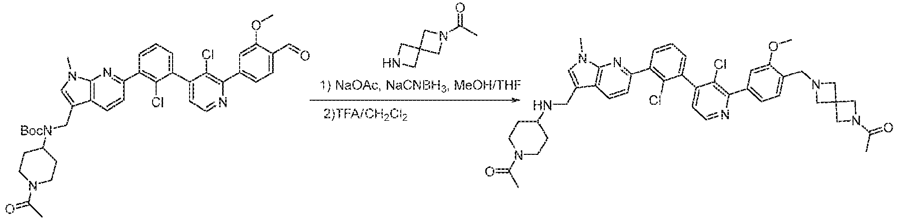

1-(4-(((6-(3-(2-(4-((6-Acetyl-2,6-diazaspiro[3.3]heptan-2-yl)methyl)-3-methoxyphenyl)-3-chloropyridin-4-yl)-2-chlorophenyl)-1-methyl-1H-pyrrolo[2,3-b]pyridin-3-yl)methyl)amino)piperidin-1-yl)ethan-1-one was prepared, following Reductive Amination Procedure B from tert-butyl (1-acetylpiperidin-4-yl)((6-(2-chloro-3-(3-chloro-2-(4-formyl-3-methoxyphenyl)pyridin-4-yl)phenyl)-1-methyl-1H-pyrrolo[2,3-b]pyridin-3-yl)methyl)carbamate (Example 556, step (b)) (120 mg, 0.16 mmol) and 1-(2,6-diazaspiro[3.3]heptan-2-yl)ethanone trifluoroacetate (0.05 g, 0.19 mmol) followed by General Boc Deprotection Procedure. 16% yield. LCMS: m/z found 767.4 [M+H]⁺, retention time = 2.71 min, (Method A); ¹H NMR (400 MHz, Methanol-*d*₄): δ 8.67 (d, 1H), 8.27 (d, 1H), 7.74 (dd, 1H), 7.69 (s, 1H), 7.61 (t, 1H), 7.50 - 7.45 (m, 4H), 7.36 - 7.33 (m, 2H), 4.69 - 4.66 (m, 1H), 4.51 (s, 2H), 4.36 (s, 2H), 4.13 - 4.07 (m, 5H), 4.02 - 3.96 (m, 10H), 3.45 - 3.41 (m, 1H), 3.25 - 3.22 (m, 1H), 2.75 - 2.66 (m, 1H), 2.31 - 2.24 (m, 2H), 2.15 (s, 3H), 1.86 (m, 3H), 1.65 - 1.50 (m, 2H).

### Example 558: 2-(4-(4-(3-(3-(((1-Acetylpiperidin-4-yl)amino)methyl)-1-methyl-1H-pyrrolo[2,3-b]pyridin-6-yl)-2-chlorophenyl)-3-chloropyridin-2-yl)-2-methoxybenzyl)-2,6-diazaspiro[3.4]octan-7-one (579)

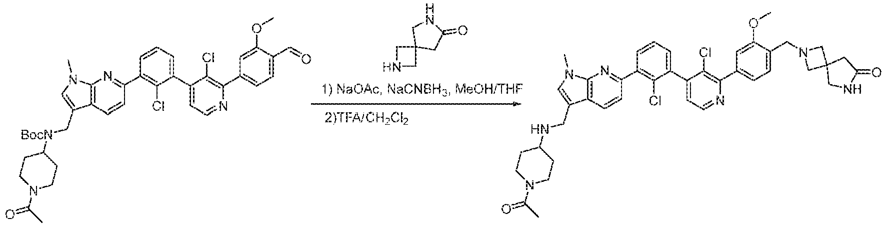

2-(4-(4-(3-(3-(((1-Acetylpiperidin-4-yl)amino)methyl)-1-methyl-1H-pyrrolo[2,3-b]pyridin-6-yl)-2-chlorophenyl)-3-chloropyridin-2-yl)-2-methoxybenzyl)-2,6-diazaspiro[3.4]octan-7-one was prepared as the formic acid salt, following Reductive Amination Procedure B from tert-butyl (1-acetylpiperidin-4-yl)((6-(2-chloro-3-(3-chloro-2-(4-formyl-3-methoxyphenyl)pyridin-4-yl)phenyl)-1-methyl-1H-pyrrolo[2,3-b]pyridin-3-yl)methyl)carbamate (Example 556, step (b)) (120 mg, 0.16 mmol) and 2,6-diazaspiro[3.4]octan-7-one trifluoroacetate (0.05 g, 0.16 mmol) followed by General Boc Deprotection Procedure. 8% yield. LCMS: m/z found 752.4 [M+H]⁺, retention time = 2.71 min (Method A); ¹H NMR (400 MHz, Methanol-*d*₄): δ 8.64 (d, 1H), 8.20 (d, 1H), 7.72 (dd, 1H), 7.60 (t, 1H), 7.47 - 7.45 (m, 3H), 7.40 - 7.37 (m, 2H), 7.28 - 7.27 (m, 2H), 4.52 - 4.48 (m, 1H), 4.07 (s, 2H), 3.98 - 3.90 (m, 7H), 3.77 (s, 2H), 3.59 (s, 2H), 3.44 - 3.40 (m, 4H), 3.19 - 3.12 (m, 1H), 2.92 - 2.85 (m, 1H), 2.74 - 2.68 (m, 1H), 2.58 (s, 2H), 2.12 - 2.03 (m, 5H), 1.46 - 1.31 (m, 2H).

### Example 559: 1-(4-(((6-(2-Chloro-3-(3-chloro-2-(3-methoxy-4-(((tetrahydro-2H-pyran-4-yl)amino)methyl)phenyl)pyridin-4-yl)phenyl)-1-methyl-1H-pyrrolo [2,3-b] pyridin-3-yl)methyl)(methyl)amino)piperidin-1-yl)ethan-1-one (600)

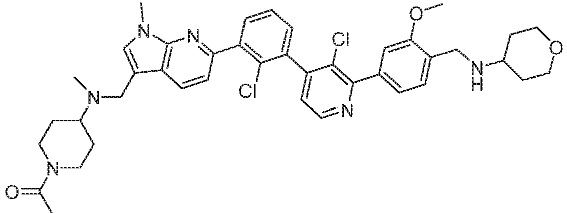

### (a) 1-(4-(((6-(2-Chloro-3-(2,3-dichloropyridin-4-yl)phenyl)-1-methyl-1H-pyrrolo[2,3-b]pyridin-3-yl)methyl)(methyl)amino)piperidin-1-yl)ethan-1-one

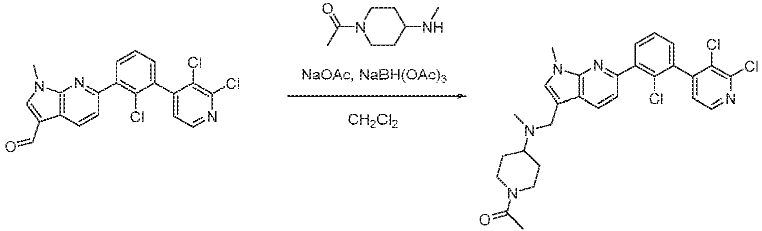

To a mixture of 6-(2-chloro-3-(2,3-dichloropyridin-4-yl)phenyl)-1-methyl-1H-pyrrolo[2,3-b]pyridine-3-carbaldehyde (500 mg, 1.20 mmol), 1-(4-amino-1-piperidyl)ethanone (255 mg, 1.80 mmol) in dichloromethane (5 mL) was added sodium acetate (295 mg, 3.60 mmol). The mixture was stirred at room temperature for 1 h under N₂. Sodium triacetoxyborohydride (1.02 g, 4.80 mmol) was added, and the mixture was stirred at room temperature for 1 h under N₂. The mixture was concentrated and the resulting residue was purified by normal phase SiO₂ chromatography (45-65% EtOAc/petroleum ether) to afford 1-(4-(((6-(2-Chloro-3-(2,3-dichloropyridin-4-yl)phenyl)-1-methyl-1H-pyrrolo[2,3-b]pyridin-3-yl)methyl)(methyl)amino)piperidin-1-yl)ethan-1-one (780 mg). MS: m/z found 556.2 [M+H]⁺.

### (b) 4-(4-(3-(3-(((1-Acetylpiperidin-4-yl)(methyl)amino)methyl)-1-methyl-1H-pyrrolo[2,3-b]pyridin-6-yl)-2-chlorophenyl)-3-chloropyridin-2-yl)-2-methoxybenzaldehyde

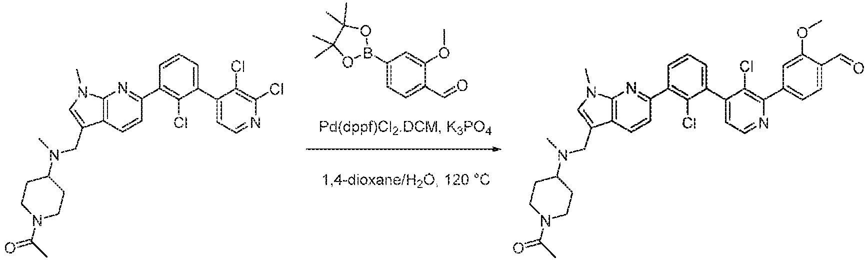

To a mixture of 1-(4-(((6-(2-Chloro-3-(2,3-dichloropyridin-4-yl)phenyl)-1-methyl-1H-pyrrolo[2,3-b]pyridin-3-yl)methyl)(methyl)amino)piperidin-1-yl)ethan-1-one (500 mg, 0.90 mmol) and 2-methoxy-4-(4,4,5,5-tetramethyl-1,3,2-dioxaborolan-2-yl)benzaldehyde (352 mg, 1.35 mmol) in dioxane/water (5:1, 18 mL) was added potassium carbonate (372 mg, 2.69 mmol) and 1,1'-bis(diphenylphosphino)ferrocene-palladium(II) dichloride dichloromethane complex (73 mg, 0.09 mmol). The mixture was stirred at 120 °C for 4 h under N₂. To the mixture was added water (10 mL). The mixture was extracted with ethyl acetate (3 x 10 mL). The combined organic phases were dried with anhydrous anhydrous sodium sulfate, filtered and concentrated. The resulting residue was purified by normal phase SiO₂ chromatography (47-55% MeOH/ethyl acetate) to afford 4-(4-(3-(3-(((1-acetylpiperidin-4-yl)(methyl)amino)methyl)-1-methyl-1H-pyrrolo[2,3-b]pyridin-6-yl)-2-chlorophenyl)-3-chloropyridin-2-yl)-2-methoxybenzaldehyde (350 mg, 50% yield). MS: m/z found 656 [M+H]⁺.

### (c) 1-(4-(((6-(2-Chloro-3-(3-chloro-2-(3-methoxy-4-(((tetrahydro-2H-pyran-4-yl)amino)methyl)phenyl)pyridin-4-yl)phenyl)-1-methyl-1H-pyrrolo[2,3-b]pyridin-3-yl)methyl)(methyl)amino)piperidin-1-yl)ethan-1-one

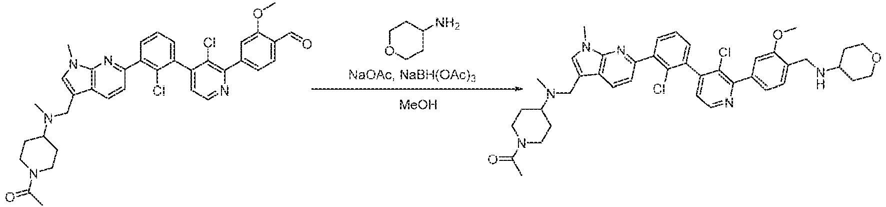

To a mixture of 4-(4-(3-(3-(((1-acetylpiperidin-4-yl)(methyl)amino)methyl)-1-methyl-1H-pyrrolo[2,3-b]pyridin-6-yl)-2-chlorophenyl)-3-chloropyridin-2-yl)-2-methoxybenzaldehyde (100 mg, 0.15 mmol) and tetrahydro-2H-pyran-4-amine (23 mg, 0.23 mmol) in MeOH (1 mL) was added sodium acetate (37 mg, 0.46 mmol). The mixture was stirred at room temperature for 5.5 h under N₂. Sodium triacetoxyborohydride (129 mg, 0.61 mmol) was added, and the mixture was stirred at room temperature for 0.5 h under N₂. The mixture was filtered, and the filtrate concentrated. The resulting residue was purified by reverse phase HPLC to afford 1-(4-(((6-(2-Chloro-3-(3-chloro-2-(3-methoxy-4-(((tetrahydro-2H-pyran-4-yl)amino)methyl)phenyl)pyridin-4-yl)phenyl)-1-methyl-1H-pyrrolo[2,3-b]pyridin-3-yl)methyl)(methyl)amino)piperidin-1-yl)ethan-1-one (54 mg, 44% yield). LCMS: m/z found 742.5 [M+H]⁺, retention time = 2.82 min (Method A); ¹H NMR (400 MHz, Methanol-*d*₄): δ 8.68 (d, 1H), 8.23 (d, 1H), 7.73 (dd, 1H), 7.63 - 7.59 (m, 2H), 7.58 (d, 1H), 7.51 (d, 1H), 7.48 - 7.38 (m, 4H), 4.68 - 4.60 (m, 2H), 4.35 - 4.32 (m, 4H), 4.13 - 4.06 (m, 3H), 4.05 (s, 3H), 3.95 (s, 3H), 3.51 - 3.45 (m, 2H), 3.31 - 3.16 (m, 2H), 2.60 - 2.61 (m, 4H), 2.15 - 2.12 (m, 7H), 1.75 - 1.73 (m, 4H).

### Example 560: 1-(4-(((6-(3-(2-(4-((6-Acetyl-2,6-diazaspiro[3.3]heptan-2-yl)methyl)-3-methoxyphenyl)-3-chloropyridin-4-yl)-2-chlorophenyl)-1-methyl-1H-pyrrolo[2,3-b]pyridin-3-yl)methyl)(methyl)amino)piperidin-1-yl)ethan-1-one (601)

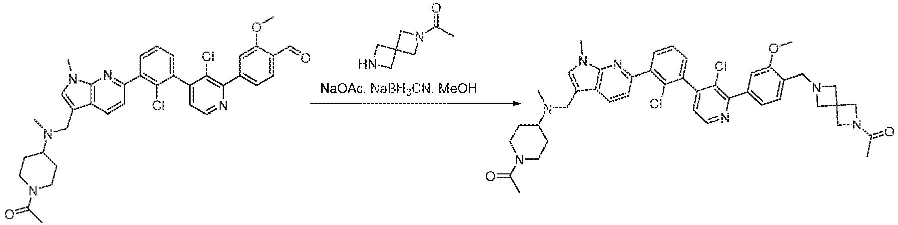

1-(4-(((6-(3-(2-(4-((6-Acetyl-2,6-diazaspiro[3.3]heptan-2-yl)methyl)-3-methoxyphenyl)-3-chloropyridin-4-yl)-2-chlorophenyl)-1-methyl-1H-pyrrolo[2,3-b]pyridin-3-yl)methyl)(methyl)amino)piperidin-1-yl)ethan-1-one was prepared as the formic acid salt, following Reductive Amination Procedure B from 4-(4-(3-(3-(((1-acetylpiperidin-4-yl)(methyl)amino)methyl)-1-methyl-1H-pyrrolo[2,3-b]pyridin-6-yl)-2-chlorophenyl)-3-chloropyridin-2-yl)-2-methoxybenzaldehyde (Example 559, step (b)) (100 mg, 0.15 mmol) and 1-(2,6-diazaspiro[3.3]heptan-2-yl)ethanone trifluoroacetate (58 mg, 0.23 mmol) followed by purification by reverse phase HPLC. 28% yield. LCMS: m/z found 780.3 [M+H]⁺, retention time = 2.70 min (Method A); ¹H NMR (400 MHz, Methanol-*d*₄): δ 8.66 (d, 1H), 8.22 (d, 1H), 7.75 - 7.72 (m, 1H), 7.63 - 7.59 (m, 2H), 7.49 - 7.43 (m, 4H), 7.35 - 7.32 (m, 2H), 4.63 - 4.60 (m, 1H), 4.35 (s, 2H), 4.24 (s, 2H), 4.11 - 4.02 (m, 5H), 3.96 - 3.91 (m, 10H), 3.21 - 3.17 (m, 2H), 2.69 - 2.57 (m, 4H), 2.15 - 2.10 (m, 5H), 1.86 (s, 3H), 1.77 - 1.63 (m, 2H).

### Example 561: 2-(4-(4-(3-(3-(((1-Acetylpiperidin-4-yl)(methyl)amino)methyl)-1-methyl-1H-pyrrolo[2,3-b]pyridin-6-yl)-2-chlorophenyl)-3-chloropyridin-2-yl)-2-methoxybenzyl)-2,6-diazaspiro[3.4]octan-7-one (603)

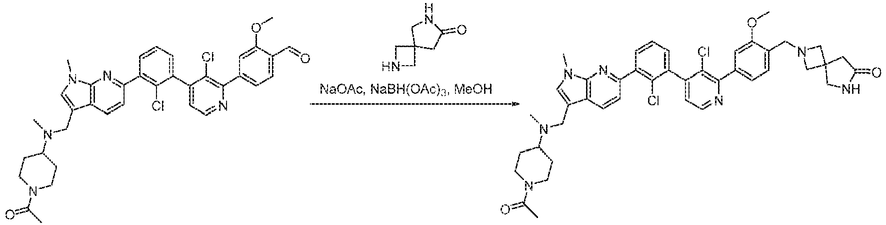

A mixture of 4-(4-(3-(3-(((1-acetylpiperidin-4-yl)(methyl)amino)methyl)-1-methyl-1H-pyrrolo[2,3-b]pyridin-6-yl)-2-chlorophenyl)-3-chloropyridin-2-yl)-2-methoxybenzaldehyde (Example 559, step (b)) (100 mg, 0.15 mmol) and 2,6-diazaspiro[3.4]octan-7-one trifluoroacetate (183 mg, 0.76 mmol) in MeOH (5 mL) was added sodium acetate (63 mg, 0.76 mol). The mixture was stirred at room temperature for 1.5 h under N₂. Sodium triacetoxyborohydride (161 mg, 0.76 mmol) was added to the mixture. After stirring at room temperature for 0.5 h under N₂, the mixture was concentrated. The resulting residue was purified by reverse phase HPLC to afford 2-(4-(4-(3-(3-(((1-acetylpiperidin-4-yl)(methyl)amino)methyl)-1-methyl-1H-pyrrolo[2,3-b]pyridin-6-yl)-2-chlorophenyl)-3-chloropyridin-2-yl)-2-methoxybenzyl)-2,6-diazaspiro[3.4]octan-7-one as formic acid salt (30 mg, 23% yield). LCMS: m/z found 766.4 [M+H]⁺, retention time = 2.68 min (Method A); ¹H NMR (400 MHz, Methanol-*d*₄): δ 8.67 (d, 1H), 8.24 (d, 1H), 7.74 (dd, 1H), 7.68 (s, 1H), 7.61 (t, 1H), 7.50 - 7.47 (m, 4H), 7.37 - 7.33 (m, 2H), 4.84 - 4.81 (m, 2H), 4.71 (s, 1H), 4.39 (s, 2H), 4.22 (s, 2H), 4.13 - 4.10 (m, 1H), 4.04 - 3.96 (m, 10H), 3.68 (s, 2H), 3.19 - 3.15 (m, 1H), 2.73 - 2.65 (m, 6H), 2.16 (m, 5H), 1.84 - 1.68 (m, 2H).

### Example 562: 2-(4-(3-Chloro-4-(2-chloro-3-(1-methyl-3-(((tetrahydro-2H-pyran-4-yl)amino)methyl)-1H-pyrrolo[2,3-b]pyridin-6-yl)phenyl)pyridin-2-yl)-2-methoxybenzyl)-2,6-diazaspiro[3.4]octan-7-one (605)

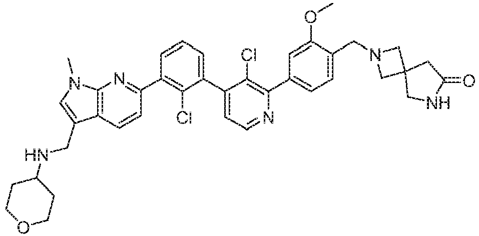

### (a) tert-Butyl ((6-(2-chloro-3-(2,3-dichloropyridin-4-yl)phenyl)-1-methyl-1H-pyrrolo[2,3-b]pyridin-3-yl)methyl)(tetrahydro-2H-pyran-4-yl)carbamate

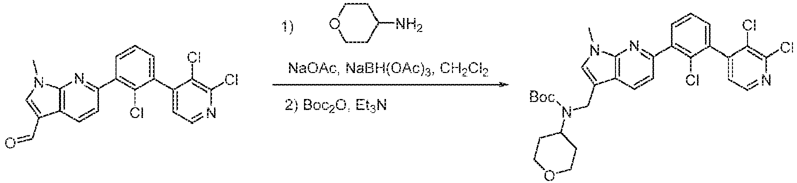

To a mixture of 6-(2-chloro-3-(2,3-dichloropyridin-4-yl)phenyl)-1-methyl-1H-pyrrolo[2,3-b]pyridine-3-carbaldehyde (0.50 g, 1.20 mmol) and tetrahydropyran-4-amine (0.60 g, 6.00 mmol) in DCM / MeOH (1:1, 20 mL) was added sodium acetate (0.29 g, 3.60 mmol) and 4A molecular sieves (0.10 g, 1.20 mmol). After stirring 12 h at room temperature, sodium cyanoborohydride (1.51 g, 24.0 mmol) was added. After stirring an additional 12 h at room temperature, the mixture was diluted with MeOH (5 mL). Di-tert-butyl dicarbonate (1.57 g, 7.17 mmol) and triethylamine (0.73 g, 7.17 mmol) were added. After stirring 4 h at room temperature under N₂, the mixture was concentrated. The resulting residue was purified by normal phase SiO₂ chromatography (0-30 % EtOAc/petroleum ether) to afford tert-butyl ((6-(2-chloro-3-(2,3-dichloropyridin-4-yl)phenyl)-1-methyl-1H-pyrrolo[2,3-b]pyridin-3-yl)methyl)(tetrahydro-2H-pyran-4-yl)carbamate (0.55 g, 76% yield) as a yellow solid. ¹H NMR (400 MHz, DMSO-*d*₆): δ 8.53 (d, 1H), 8.14 (d, 1H), 7.75 (d, 1H), 7.64 - 7.60 (m, 2H), 7.52 - 7.48 (m, 2H), 7.37 (d, 1H), 4.57 (s, 2H), 3.83 (s, 5H), 3.25 - 3.22 (m, 2H), 1.85 - 1.83 (m, 2H), 1.46 (m, 11H).

### (b) tert-Butyl((6-(2-chloro-3-(3-chloro-2-(4-formyl-3-methoxyphenyl)pyridin-4-yl)phenyl)-1-methyl-1H-pyrrolo[2,3-b]pyridin-3-yl)methyl)(tetrahydro-2H-pyran-4-yl)carbamate

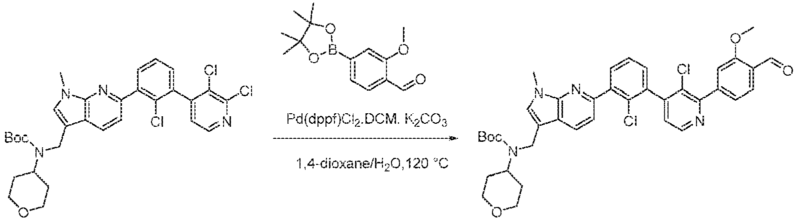

To a of mixture of tert-butyl ((6-(2-chloro-3-(2,3-dichloropyridin-4-yl)phenyl)-1-methyl-1H-pyrrolo[2,3-b]pyridin-3-yl)methyl)(tetrahydro-2H-pyran-4-yl)carbamate (500 mg, 0.83 mmol) and 2-methoxy-4-(4,4,5,5-tetramethyl-1,3,2-dioxaborolan-2-yl)benzaldehyde (435 mg, 1.66 mmol) in dioxane / H₂O (5:1, 12 mL) were added 1,1'-bis(diphenylphosphino)ferrocene-palladium(II) dichloride dichloromethane complex (68 mg, 0.08 mmol) and potassium carbonate (344 mg, 2.49 mmol). The mixture was stirred at 120 °C for 12 h under N₂. The mixture was diluted with water (20 mL) and extracted with EtOAc (2 x 30 mL). The combined organic phases were dried over anhydrous sulfate, filtered and concentrated under reduced pressure. The resulting residue was purified by normal phase SiO₂ chromatography (0-75 % EtOAc/petroleum ether) to afford tert-butyl((6-(2-chloro-3-(3-chloro-2-(4-formyl-3-methoxyphenyl)pyridin-4-yl)phenyl)-1-methyl-1H-pyrrolo[2,3-b]pyridin-3-yl)methyl)(tetrahydro-2H-pyran-4-yl)carbamate (514 mg, 80% yield). ¹H NMR (400 MHz, DMSO-*d*₆): δ 10.44 (s, 1H), 8.78 (d, 1H), 8.15 (d, 1H), 7.84 - 7.82 (m, 1H), 7.76 - 7.73 (m, 1H), 7.64-7.61 (m, 2H), 7.54 - 7.52 (m, 3H), 7.43 - 7.37 (m, 2H), 4.57 (s, 2H), 4.09 - 4.03 (m, 1H), 3.83 - 3.81 (m, 4H), 3.63 - 3.60 (m, 6H), 3.28 - 3.22 (m, 2H), 1.85 - 1.79 (m, 2H), 1.46 (s, 9H).

### (c) 2-(4-(3-Chloro-4-(2-chloro-3-(1-methyl-3-(((tetrahydro-2H-pyran-4-yl)amino)methyl)-1H-pyrrolo[2,3-b]pyridin-6-yl)phenyl)pyridin-2-yl)-2-methoxybenzyl)-2,6-diazaspiro[3.4]octan-7-one

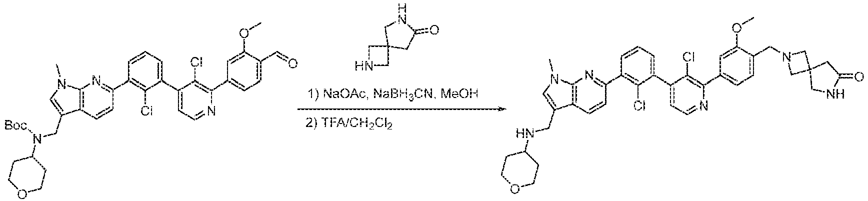

2-(4-(3-Chloro-4-(2-chloro-3-(1-methyl-3-(((tetrahydro-2H-pyran-4-yl)amino)methyl)-1H-pyrrolo[2,3-b]pyridin-6-yl)phenyl)pyridin-2-yl)-2-methoxybenzyl)-2,6-diazaspiro[3.4]octan-7-one was prepared as the formic acid salt, following Reductive Amination Procedure B from tert-butyl((6-(2-chloro-3-(3-chloro-2-(4-formyl-3-methoxyphenyl)pyridin-4-yl)phenyl)-1-methyl-1H-pyrrolo[2,3-b]pyridin-3-yl)methyl)(tetrahydro-2H-pyran-4-yl)carbamate (100 mg, 0.14 mmol) and 2,6-diazaspiro[3.4]octan-7-one trifluoroacetate (171 mg, 0.71 mmol), followed by General Boc Deprotection Procedure. 43% yield. LCMS: m/z found 711.3 [M+H]⁺, retention time = 2.72 min (Method A); ¹H NMR (400 MHz, Methanol-*d*₄): δ 8.69 (d, 1H), 8.27 (d, 1H), 7.73 - 7.72 (m, 2H), 7.62 (t, 1H), 7.56 (d, 1H), 7.52 - 7.47 (m, 3H), 7.43 (s, 1H), 7.40 - 7.38 (m, 1H), 4.34 - 4.29 (m, 6H), 4.11 - 4.05 (m, 3H), 4.02 (s, 3H), 3.97 (s, 3H), 3.72 - 3.70 (m, 3H), 3.51 - 3.47 (m, 3H), 2.76 (d, 2H), 2.19 - 2.15 (m, 2H), 1.80 - 1.70 (m, 2H).

### Example 563: 2-((4-(3-Chloro-4-(2-chloro-3-(1-methyl-3-(((tetrahydro-2H-pyran-4-yl)amino)methyl)-1H-pyrrolo[2,3-b]pyridin-6-yl)phenyl)pyridin-2-yl)-2-methoxybenzyl)amino)ethan-1-ol (606)

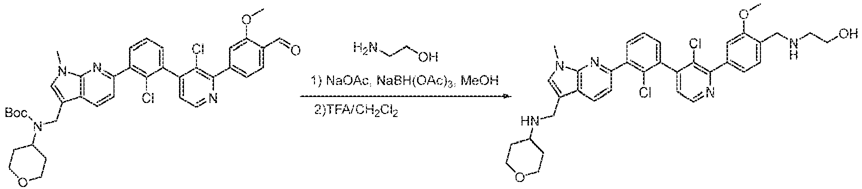

To a mixture of tert-butyl((6-(2-chloro-3-(3-chloro-2-(4-formyl-3-methoxyphenyl)pyridin-4-yl)phenyl)-1-methyl-1H-pyrrolo[2,3-b]pyridin-3-yl)methyl)(tetrahydro-2H-pyran-4-yl)carbamate (Example 562, step (b)) (150 mg, 0.21 mol) and 2-aminoethanol (39 mg, 0.64 mmol) in MeOH (3 mL) was added sodium acetate (70.1 mg, 0.86 mmol). After stirring at 2 h at room temperature, sodium triacetoxyborohydride (181 mg, 0.86 mmol) was added to the mixture. After 30 minutes, the mixture was diluted with DCM (5 mL) and trifluoroacetic acid (2 mL) was added. The mixture was concentrated and the resulting residue was purified by reverse phase HPLC to afford 2-((4-(3-Chloro-4-(2-chloro-3-(1-methyl-3-(((tetrahydro-2H-pyran-4-yl)amino)methyl)-1H-pyrrolo[2,3-b]pyridin-6-yl)phenyl)pyridin-2-yl)-2-methoxybenzyl)amino)ethan-1-ol as formic acid salt (17 mg, 12% yield). LCMS: m/z found 646.3 [M+H]⁺, retention time = 2.58 min, (Method A); ¹H NMR (400 MHz, Methanol-*d*₄): δ 8.69 (d, 1H), 8.26 (d, 1H), 7.75 - 7.73 (dd, 1H), 7.69 (s, 1H), 7.62 (t, 1H), 7.55 - 7.47 (m, 4H), 7.41 (s, 1H), 7.38 - 7.36 (dd, 1H), 4.46 (s, 2H), 4.32 (s, 2H), 4.09 - 4.05 (m, 2H), 4.01 (s, 3H), 3.96 (s, 3H), 3.85 (t, 2H), 3.51 - 3.45 (m, 3H), 3.16 (t, 2H), 2.19 - 2.14 (m, 2H), 1.77 - 1.67 (m, 2H).

### Example 564: 1-(6-(4-(3-Chloro-4-(2-chloro-3-(1-methyl-3-(((tetrahydro-2H-pyran-4-yl)amino)methyl)-1H-pyrrolo[2,3-b]pyridin-6-yl)phenyl)pyridin-2-yl)-2-methoxybenzyl)-2,6-diazaspiro[3.3]heptan-2-yl)ethan-1-one (609)

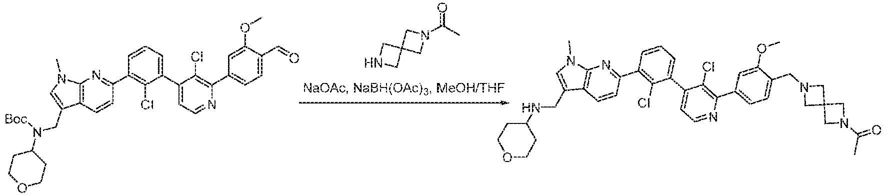

To a mixture oftert-butyl((6-(2-chloro-3-(3-chloro-2-(4-formyl-3-methoxyphenyl)pyridin-4-yl)phenyl)-1-methyl-1H-pyrrolo[2,3-b]pyridin-3-yl)methyl)(tetrahydro-2H-pyran-4-yl)carbamate (Example 562, step (b)) (100 mg, 0.14 mmol) and 1-(2,6-diazaspiro[3.3]heptan-2-yl)ethanone hydrochloride (33 mg, 0.19 mmol) in MeOH (3 mL) was added sodium acetate (47 mg, 0.57 mmol). After stirring at room temperature for 2.5 h, sodium triacetoxyborohydride (121 mg, 0.57 mmol) was added. The mixture was stirred at room temperature for 30 minutes. Water (20 mL) was added, and the mixture was extracted with EtOAc (2 x 30 mL). The combined organic phases were dried over anhydrous sulfate, filtered and concentrated under reduced pressure. The residue was purified by normal phase SiO₂ chromatography (0-50 % EtOAc/petroleum ether) to afford tert-butyl ((6-(3-(2-(4-((6-acetyl-2,6-diazaspiro[3.3]heptan-2-yl)methyl)-3-methoxyphenyl)-3-chloropyridin-4-yl)-2-chlorophenyl)-1-methyl-1H-pyrrolo[2,3-b]pyridin-3-yl)methyl)(tetrahydro-2H-pyran-4-yl)carbamate (107 mg, 91% yield). MS: m/z found 825.4 [M+H]⁺.

Trifluoroacetic acid (2 mL) was added to a mixture of tert-butyl((6-(3-(2-(4-((6-acetyl-2,6-diazaspiro[3.3]heptan-2-yl)methyl)-3-methoxyphenyl)-3-chloropyridin-4-yl)-2-chlorophenyl)-1-methyl-1H-pyrrolo[2,3-b]pyridin-3-yl)methyl)(tetrahydro-2H-pyran-4-yl)carbamate (100 mg, 0.12 mmol) in DCM (5 mL). After stirring for 30 minutes at room temperature, the mixture was concentrated. The resulting residue was purified by reverse phase HPLC to afford 1-(6-(4-(3-chloro-4-(2-chloro-3-(1-methyl-3-(((tetrahydro-2H-pyran-4-yl)amino)methyl)-1H-pyrrolo[2,3-b]pyridin-6-yl)phenyl)pyridin-2-yl)-2-methoxybenzyl)-2,6-diazaspiro[3.3]heptan-2-yl)ethan-1-one as formic acid salt (7.6 mg, 8% yield). LCMS: m/z found 725.4 [M+H]⁺, retention time = 2.80 min (Method A); ¹H NMR (400 MHz, Methanol-*d*₄): δ 8.66 (d, 1H), 8.26 (d, 1H), 7.75 - 7.72 (dd, 1H), 7.68 (s, 1H), 7.61 (t, 1H), 7.51 - 7.47 (m, 3H), 7.42 (d, 1H), 7.33 - 7.30 (m, 2H), 4.46 (s, 2H), 4.34 (s, 2H), 4.13 - 4.05 (m, 4H), 3.96 - 3.91 (m, 8H), 3.77 (s, 4H), 3.52 - 3.41 (m, 3H), 2.16 - 2.13 (m, 2H), 1.86 (s, 3H), 1.77 - 1.67 (m, 2H).

### Example 565: N-((6-(2-Chloro-3-(3-chloro-2-(3-methoxy-4-(((tetrahydro-2H-pyran-4-yl)amino)methyl)phenyl)pyridin-4-yl)phenyl)-1-methyl-1H-pyrrolo[2,3-b]pyridin-3-yl)methyl)-N-methyltetrahydro-2H-pyran-4-amine (635)

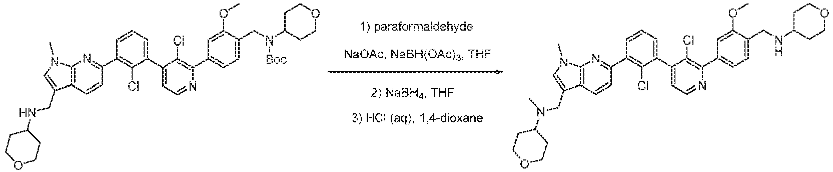

To a mixture of tert-butyl 4-(3-chloro-4-(2-chloro-3-(1-methyl-3-(((tetrahydro-2H-pyran-4-yl)amino)methyl)-1H-pyrrolo[2,3-b]pyridin-6-yl)phenyl)pyridin-2-yl)-2-methoxybenzyl(tetrahydro-2H-pyran-4-yl)carbamate (Example 566, step (d)) (0.50 g, 0.64 mmol) in THF (5 mL) was added paraformaldehyde (0.06 g, 1.91 mmol) and sodium acetate (0.16 g, 1.91 mmol) in one portion under N₂. The mixture was stirred at room temperature for 12 h. Sodium triacetoxyborohydride (0.54 g, 2.54 mmol) was added to the mixture in one portion under N₂. The mixture was stirred at room temperature for 0.5 h. The mixture was filtered. The filtrate was concentrated. The residue was purified by normal phase SiO₂ chromatography (0-90% MeOH/ethyl acetate) to afford 0.45 g of the imine intermediate. To a solution of 0.45 g of the imine intermediate in THF (5 mL) was added to sodium borohydride (0.17 g, 4.5 mmol). The mixture was stirred at 20 °C for 12 h. Water (3 mL) was added. The resulting mixture was purified by reverse phase HPLC to afford tert-butyl 4-(3-chloro-4-(2-chloro-3-(1-methyl-3-((methyl(tetrahydro-2H-pyran-4-yl)amino)methyl)-1H-pyrrolo[2,3-b]pyridin-6-yl)phenyl)pyridin-2-yl)-2-methoxybenzyl(tetrahydro-2H-pyran-4-yl)carbamate as a white solid (45 mg, 10% yield). MS: m/z found 800.3 [M+H]⁺. To a mixture of tert-butyl 4-(3-chloro-4-(2-chloro-3-(1-methyl-3-((methyl(tetrahydro-2H-pyran-4-yl)amino)methyl)-1H-pyrrolo[2,3-b]pyridin-6-yl)phenyl)pyridin-2-yl)-2-methoxybenzyl(tetrahydro-2H-pyran-4-yl)carbamate (40 mg, 0.05 mmol) in 1,4-dioxane (1.5 mL) was added aqueous HCl solution (12 M, 0.3 mL) in one portion. The mixture was stirred at room temperature for 2 h. The mixture was combined with another batch at 5 mg scale. The combined mixture was purified by reverse phase HPLC to afford N-((6-(2-chloro-3-(3-chloro-2-(3-methoxy-4-(((tetrahydro-2H-pyran-4-yl)amino)methyl)phenyl)pyridin-4-yl)phenyl)-1-methyl-1H-pyrrolo[2,3-b]pyridin-3-yl)methyl)-N-methyltetrahydro-2H-pyran-4-amine as a white solid (6 mg, 5% yield). LCMS: m/z found 700.3 [M+H]⁺, retention time = 2.94 min (Method A); ¹H NMR (400 MHz, Methanol-*d*₄): δ 8.69 (d, 1H), 8.23 (d, 1H), 7.74 (dd, 1H), 7.68 - 7.59 (m, 2H), 7.55 - 7.45 (m, 4H), 7.41 (s, 1H), 7.37 (d, 1H), 4.38 - 4.29 (m, 4H), 4.12 - 4.06 (m, 4H), 4.05 (s, 3H), 4.01 (s, 3H), 3.53 - 3.40 (m, 6H), 2.72 - 2.59 (m, 3H), 2.13 - 2.04 (m, 4H), 1.86 - 1.64 (m, 4H).

### Example 566: N-((6-(2-Chloro-3-(3-chloro-2-(3-methoxy-4-(((tetrahydro-2H-pyran-4-yl)amino)methyl)phenyl)pyridin-4-yl)phenyl)-1-methyl-1H-pyrrolo[2,3-b]pyridin-3-yl)methyl)tetrahydro-2H-pyran-4-amine (563)

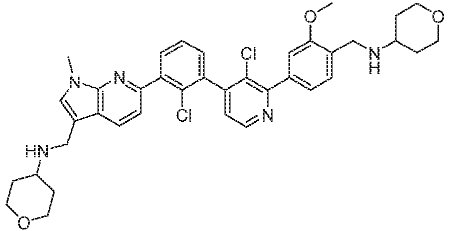

### (a) tert-Butyl 4-bromo-2-methoxybenzyl(tetrahydro-2H-pyran-4-yl)carbamate

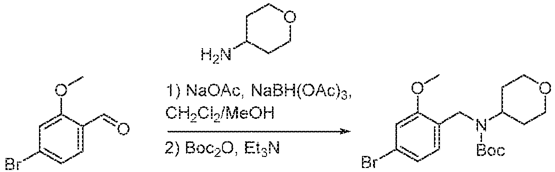

To a mixture of 4-bromo-2-methoxy-benzaldehyde (1.50 g, 6.98 mmol) and tetrahydropyran-4-amine (0.99 g, 9.77 mmol) in MeOH/DCM (1:1, 16 mL) was added sodium acetate (1.14 g, 14 mmol) in one portion under N₂. The mixture was stirred at room temperature for 12 hr. Sodium triacetoxyborohydride (4.44 g, 20.9 mmol) was added to the mixture in one portion under N₂. The mixture was stirred at room temperature for 1 hr. The above solution was diluted with THF (20 mL). Di-tert-butyl dicarbonate (4.00 mL, 17.4 mmol) and triethylamine (0.97 mL, 6.96 mmol) were added in one portion under N₂. The mixture was stirred at room temperature for 1 hr. To the mixture was added water (500 mL). The mixture was extracted with ethyl acetate (2 x 250 mL). The combined organic phases were washed with saturated aqueous brine solution (2 x 200 mL), dried with anhydrous sodium sulfate, filtered and concentrated. The resulting residue was purified by normal phase SiO₂ chromatography (0-10% ethyl acetate/petroleum ether) to afford tert-butyl 4-bromo-2-methoxybenzyl(tetrahydro-2H-pyran-4-yl)carbamate as a colorless oil (1.8 g, 60%). MS: m/z found: 300 [M-t-Butyl]⁺.

### (b) tert-Butyl 2-methoxy-4-(4,4,5,5-tetramethyl-1,3,2-dioxaborolan-2-yl)benzyl(tetrahydro-2H-pyran-4-yl)carbamate

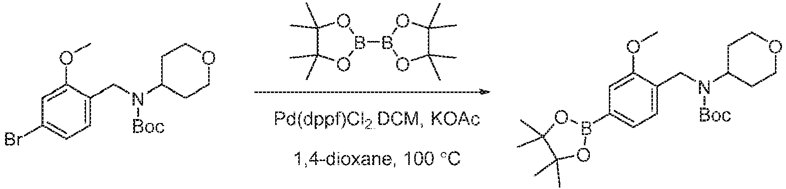

To a mixture of tert-butyl 4-bromo-2-methoxybenzyl(tetrahydro-2H-pyran-4-yl)carbamate (1.60 g, 4.00 mmol) and bis(pinacolato)diborane (1.83 g, 7.19 mmol) in 1,4-dioxane (15 mL) was added 1,1'-bis(diphenylphosphino)ferrocene-palladium(II) dichloride dichloromethane complex (0.1 g, 0.12 mmol) and potassium acetate (1.18 g, 12 mmol) in one portion under N₂. The mixture was stirred at 100 °C for 3 hr. The mixture was concentrated. The resulting residue was purified by normal phase SiO₂ chromatography (0-30% ethyl acetate/petroleum ether) to afford tert-butyl 2-methoxy-4-(4,4,5,5-tetramethyl-1,3,2-dioxaborolan-2-yl)benzyl(tetrahydro-2H-pyran-4-yl)carbamate as a white solid (2.00 g, 87% yield). ¹H NMR (400 MHz, Chloroform-d): δ 7.30 (d, 1H), 7.17 (s, 1H), 7.07 (d, 1H), 4.32 (m, 2H), 3.86-3.82 (m, 6H), 3.38-3.28 (m, 2H), 1.52-1.49 (m, 4H), 1.27 - 1.17 (m, 21H).

### (c) tert-Butyl 4-(3-chloro-4-(2-chloro-3-(3-formyl-1-methyl-1H-pyrrolo[2,3-b]pyridin-6-yl)phenyl)pyridin-2-yl)-2-methoxybenzyl(tetrahydro-2H-pyran-4-yl)carbamate

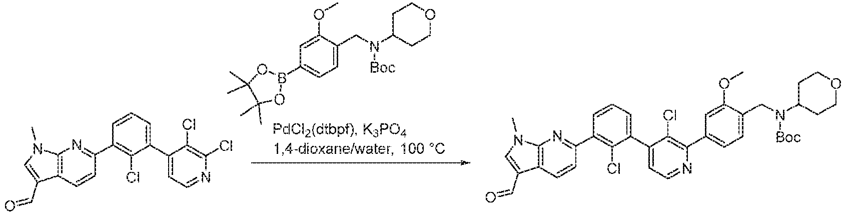

To a mixture of 6-(2-chloro-3-(2,3-dichloropyridin-4-yl)phenyl)-1-methyl-1H-pyrrolo[2,3-b]pyridine-3-carbaldehyde (0.15 g, 0.36 mmol) and tert-butyl 2-methoxy-4-(4,4,5,5-tetramethyl-1,3,2-dioxaborolan-2-yl)benzyl(tetrahydro-2H-pyran-4-yl)carbamate (0.29 g, 0.65 mmol) in 1,4-dioxane/H₂O mixture (4:1, 5 mL) was added [1,1'-bis(di-tert-butylphosphino)ferrocene]dichloropalladium(II) (0.02 g, 0.04 mmol) and potassium phosphate (0.23 g, 1.08 mmol) in one portion under N₂. The mixture was stirred at 100 °C for 12 hr. To the mixture was added water (50 mL). The mixture was extracted with ethyl acetate (2 x 50 mL). The combined organic phases were washed with saturated aqueous brine solution (2 x 25 mL), dried with anhydrous sodium sulfate, filtered and concentrated. The resulting residue was purified by prep-TLC (silica gel, petroleum ether: ethyl acetate = 2:1) to afford tert-butyl 4-(3-chloro-4-(2-chloro-3-(3-formyl-1-methyl-1H-pyrrolo[2,3-b]pyridin-6-yl)phenyl)pyridin-2-yl)-2-methoxybenzyl(tetrahydro-2H-pyran-4-yl)carbamate as a white solid (0.15 g, 55% yield). MS: m/z found 701 [M+H]⁺.

### (d) tert-Butyl 4-(3-chloro-4-(2-chloro-3-(1-methyl-3-(((tetrahydro-2H-pyran-4-yl)amino)methyl)-1H-pyrrolo[2,3-b]pyridin-6-yl)phenyl)pyridin-2-yl)-2-methoxybenzyl(tetrahydro-2H-pyran-4-yl)carbamate

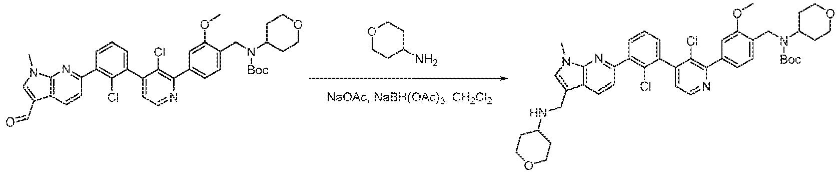

To a mixture of tert-butyl 4-(3-chloro-4-(2-chloro-3-(3-formyl-1-methyl-1H-pyrrolo[2,3-b]pyridin-6-yl)phenyl)pyridin-2-yl)-2-methoxybenzyl(tetrahydro-2H-pyran-4-yl)carbamate (0.10 g, 0.14 mmol) and tetrahydro-2H-pyran-4-amine (0.03 g, 0.29 mmol) in CH₂Cl₂ (3 mL) was added sodium acetate (0.04 g, 0.43 mmol) in one portion under N₂. The mixture was stirred at room temperature for 12 hr. Sodium triacetoxyborohydride (0.09 g, 0.43 mmol) was added to the mixture in one portion under N₂. The mixture was stirred at room temperature for 1 hr. The mixture was concentrated to afford tert-butyl 4-(3-chloro-4-(2-chloro-3-(1-methyl-3-(((tetrahydro-2H-pyran-4-yl)amino)methyl)-1H-pyrrolo[2,3-b]pyridin-6-yl)phenyl)pyridin-2-yl)-2-methoxybenzyl(tetrahydro-2H-pyran-4-yl)carbamate as a yellow oil (0.12 g). MS: m/z found 786 [M+H]⁺.

### (e) N-((6-(2-Chloro-3-(3-chloro-2-(3-methoxy-4-(((tetrahydro-2H-pyran-4-yl)amino)methyl)phenyl)pyridin-4-yl)phenyl)-1-methyl-1H-pyrrolo[2,3-b]pyridin-3-yl)methyl)tetrahydro-2H-pyran-4-amine

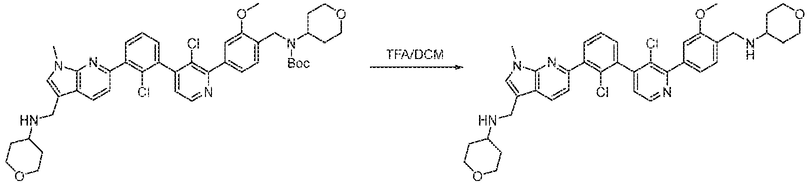

tert-Butyl 4-(3-chloro-4-(2-chloro-3-(1-methyl-3-(((tetrahydro-2H-pyran-4-yl)amino)methyl)-1H-pyrrolo[2,3-b]pyridin-6-yl)phenyl)pyridin-2-yl)-2-methoxybenzyl(tetrahydro-2H-pyran-4-yl)carbamate was subjected to General Boc Deprotection Procedure to provide N-((6-(2-chloro-3-(3-chloro-2-(3-methoxy-4-(((tetrahydro-2H-pyran-4-yl)amino)methyl)phenyl)pyridin-4-yl)phenyl)-1-methyl-1H-pyrrolo[2,3-b]pyridin-3-yl)methyl)tetrahydro-2H-pyran-4-amine (9% yield). LCMS: m/z found 686.3 [M+H]⁺, retention time = 2.93 min (Method A); ¹H NMR (400 MHz, Methanol-*d*₄): δ 8.79-8.73 (m, 3H), 8.35 - 8.25 (m, 1H), 7.84 - 7.79 (m, 1H), 7.74 - 7.72 (m, 1H), 7.63 (t, 1H), 7.58 - 7.34 (m, 5H), 4.41 - 4.17 (m, 3H), 3.96 - 3.85 (m, 8H), 3.37 (m, 3H), 3.32 (m, 6H), 2.08 - 1.99 (m, 4H), 1.66 - 1.52 (m, 4H).

### Example 567: 1,1'-(((((3,3'-Dichloro-[4,4'-bipyridine]-2,2'-diyl)bis(2-fluoro-6-methoxy-4,1-phenylene))bis(methylene))bis(azanediyl))bis(piperidine-4,1-diyl))bis(ethan-1-one) (313)

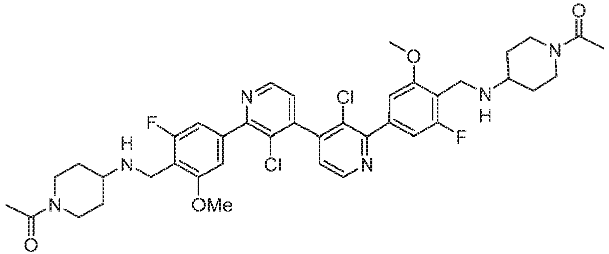

1,1'-(((((3,3'-Dichloro-[4,4'-bipyridine]-2,2'-diyl)bis(2-fluoro-6-methoxy-4,1-phenylene))bis(methylene))bis(azanediyl))bis(piperidine-4,1-diyl))bis(ethan-1-one) was prepared in similar manner as described for Example 132, replacing 2-methoxy-4-(4,4,5,5-tetramethyl-1,3,2-dioxaborolan-2-yl)-benzaldehyde with 2-fluoro-6-methoxy-4-(4,4,5,5-tetramethyl-1,3,2-dioxaborolan-2-yl)-benzaldehyde in step (b) and (S)-5-(aminomethyl)pyrrolidin-2-one with 1-(4-aminopiperidin-1-yl)ethan-1-one in step (c). Product was obtained as a tan solid (formic acid salt). LC/MS: *m*/*z* found 781, 783 [M+H]⁺, retention time = 2.02 min (Method C). ¹H NMR (400 MHz, Methanol-*d*₄) δ 8.73 (d, 2H), 8.43 (s, 2H), 7.52 (d, 2H), 7.26 (s, 2H), 7.20 (d, 2H), 4.63 (d, 2H), 4.29 (s, 4H), 4.00 (s, 8H), 3.34 (s, 2H), 3.19 (t, 2H), 2.70 (t, 2H), 2.21 (t, 4H), 2.12 (s, 6H), 1.67 - 1.39 (m, 4H).

### Example 568: 2,2'-(((3,3'-Dichloro-[4,4'-bipyridine]-2,2'-diyl)bis(2-fluoro-6-methoxy-4,1-phenylene))bis(methylene))bis(2,6-diazaspiro[3.4]octan-7-one) (323)

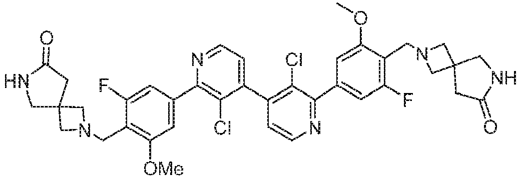

2,2'-(((3,3'-Dichloro-[4,4'-bipyridine]-2,2'-diyl)bis(2-fluoro-6-methoxy-4,1-phenylene))bis(methylene))bis(2,6-diazaspiro[3.4]octan-7-one) was prepared in similar manner as described for **Example 132,** replacing 2-methoxy-4-(4,4,5,5-tetramethyl-1,3,2-dioxaborolan-2-yl)-benzaldehyde with 2-fluoro-6-methoxy-4-(4,4,5,5-tetramethyl-1,3,2-dioxaborolan-2-yl)-benzaldehyde in step (b) and (S)-5-(aminomethyl)pyrrolidin-2-one with 2,6-diazaspiro[3.4]octan-7-one in step (c). Product was obtained as a tan solid (formic acid salt). LC/MS: *m*/*z* found 749, 751 [M+H]⁺, retention time = 1.91 min (Method C). ¹H NMR (400 MHz, Methanol-*d*₄) δ 8.75 - 8.68 (m, 2H), 8.38 (s, 2H), 7.54 - 7.47 (m, 2H), 7.23 (s, 2H), 7.20 - 7.15 (m, 2H), 4.17 (s, 4H), 3.97 (s, 6H), 3.85 (s, 8H), 3.60 (s, 4H), 2.62 (s, 4H).

### Example 569: (5S,5'S)-5,5'-(((3,3'-Dichloro-[4,4'-bipyridine]-2,2'-diyl)bis(8-methoxy-3,4-dihydroisoquinoline-6,2(1H)-diyl))bis(methylene))bis(pyrrolidin-2-one) (266)

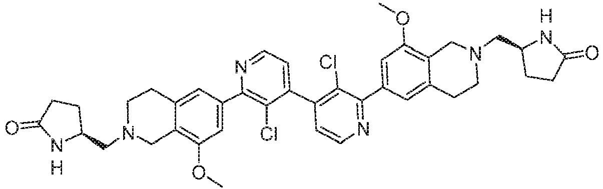

(5S,5'S)-5,5'-(((3,3'-Dichloro-[4,4'-bipyridine]-2,2'-diyl)bis(8-methoxy-3,4-dihydroisoquinoline-6,2(1H)-diyl))bis(methylene))bis(pyrrolidin-2-one) was prepared in the same manner as described for Example 132, replacing 2-methoxy-4-(4,4,5,5-tetramethyl-1,3,2-dioxaborolan-2-yl)-benzaldehyde with (5S)-5-[[8-methoxy-6-(4,4,5,5-tetramethyl-1,3,2-dioxaborolan-2-yl)-3,4-dihydro-1H-isoquinolin-2-yl]-methyl]pyrrolidin-2-one in step (b). Product was obtained as a tan solid (formic acid salt). LC/MS: *m*/*z* found 741, 743 [M+H]⁺, retention time = 1.98 min (Method C). ¹H NMR (400 MHz, Methanol-*d*₄) δ 8.65 (d, 2H), 7.44 (d, 2H), 7.05 (d, 4H), 4.03 (s, 2H), 3.87 (s, 8H), 3.75 (d, 2H), 3.62 (d, 2H), 2.97 (s, 4H), 2.92 - 2.57 (m, 8H), 2.32 (m, 6H).

### Example 570: Methyl ((3-chloro-4-(2-chloro-3-(6-methoxy-5-(((((S)-5-oxopyrrolidin-2-yl)methyl)amino)methyl)pyridin-2-yl)phenyl)-5'-methoxy-[2,3'-bipyridin]-6'-yl)methyl)-L-homoserinate (267)

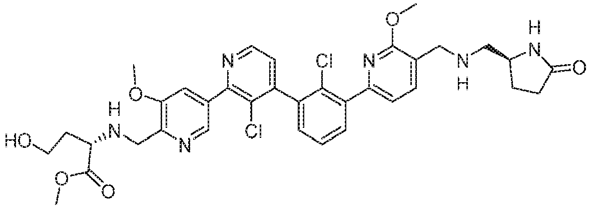

Methyl ((3-chloro-4-(2-chloro-3-(6-methoxy-5-(((((S)-5-oxopyrrolidin-2-yl)methyl)amino)methyl)pyridin-2-yl)phenyl)-5'-methoxy-[2,3'-bipyridin]-6'-yl)methyl)-L-homoserinate was prepared in similar manner as described for Example 45, replacing 1-methyl-6-(4,4,5,5-tetramethyl-1,3,2-dioxaborolan-2-yl)pyrrolo[3,2-b]pyridine-3-carbaldehyde with 3-methoxy-5-(4,4,5,5-tetramethyl-1,3,2-dioxaborolan-2-yl)-picolinaldehyde in step (d) and 1-(4-aminopiperidin-1-yl)ethan-1-one with methyl L-homoserinate in step (e). Product was obtained as a white solid (formic acid salt). LC/MS: *m*/*z* found 709, 711 [M+H]⁺, retention time = 2.05 min (Method C). ¹H NMR (400 MHz, Methanol-*d*₄) δ 8.69 (d, 1H), 8.49 (s, 1H), 8.43 (s, 1H), 7.76 (d, 1H), 7.71 (d, 2H), 7.55 (t, 1H), 7.48 (d, 1H), 7.42 (d, 1H), 7.27 (d, 1H), 4.02 (d, 5H), 3.96 (s, 3H), 3.88 (d, 3H), 3.79 - 3.55 (m, 5H), 2.76 (t, 2H), 2.32 (s, 3H), 2.08 - 1.69 (m, 3H).

### Example 571: (S)-5-((((6-(2-Chloro-3-(3-chloro-5'-methoxy-6'-((((S)-2-oxotetrahydrofuran-3-yl)amino)methyl)-[2,3'-bipyridin]-4-yl)phenyl)-2-methoxypyridin-3-yl)methyl)-amino)methyl)pyrrolidin-2-one (268)

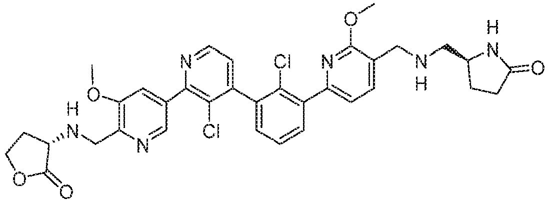

(S)-5-((((6-(2-Chloro-3-(3-chloro-5'-methoxy-6'-((((S)-2-oxotetrahydrofuran-3-yl)amino)methyl)-[2,3'-bipyridin]-4-yl)phenyl)-2-methoxypyridin-3-yl)methyl)-amino)methyl)pyrrolidin-2-one was prepared in the same manner as described for Example 45, replacing 1-methyl-6-(4,4,5,5-tetramethyl-1,3,2-dioxaborolan-2-yl)pyrrolo[3,2-b]pyridine-3-carbaldehyde with 3-methoxy-5-(4,4,5,5-tetramethyl-1,3,2-dioxaborolan-2-yl)-picolinaldehyde in step (d) and 1-(4-aminopiperidin-1-yl)ethan-1-one with (S)-3-aminodihydrofuran-2(3H)-one in step (e). Product was obtained as a white solid (formic acid salt). LC/MS: *m*/*z* found 677, 679 [M+H]⁺, retention time = 2.07 min (Method C). ¹H NMR (400 MHz, Methanol-*d*₄) δ 8.69 (d, 1H), 8.46 (s, 1H), 8.43 (s, 1H), 7.79 (d, 1H), 7.76 - 7.68 (m, 2H), 7.56 (t, 1H), 7.48 (d, 1H), 7.43 (d, 1H), 7.29 (d, 1H), 4.42 (t, 1H), 4.24 (m, 1H), 4.13 (s, 2H), 4.04 (s, 3H), 3.97 (s, 5H), 3.90 (s, 1H), 3.76 (t, 1H), 2.85 (d, 2H), 2.64 - 2.50 (m, 1H), 2.41 - 2.24 (m, 3H), 2.22 - 2.08 (m, 1H), 1.84 (s, 1H).

### Example 572: 1-(4-(((6-(3-(6'-(((1-Acetylpiperidin-4-yl)amino)methyl)-3-chloro-5'-methoxy-[2,3'-bipyridin]-4-yl)-2-chlorophenyl)-2-methoxypyridin-3-yl)methyl)amino)piperidin-1-yl)ethan-1-one (375)

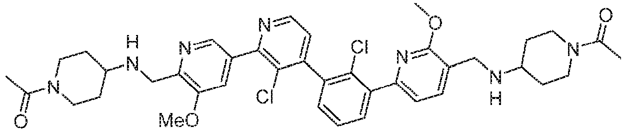

### (a) 3-Chloro-4-(2-chloro-3-(5-formyl-6-methoxypyridin-2-yl)phenyl)-5'-methoxy-[2,3'-bipyridine]-6'-carbaldehyde

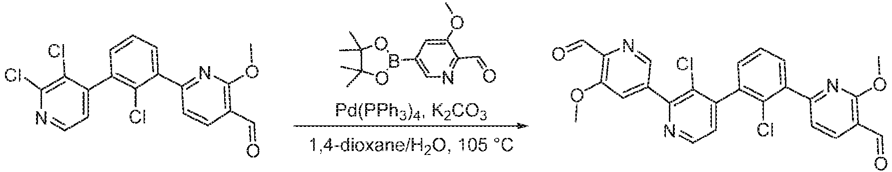

Tetrakis(triphenylphosphine)palladium(0) (41 mg, 0.04 mmol), potassium carbonate (74 mg, 0.53 mmol), 6-[2-chloro-3-(2,3-dichloro-4-pyridyl)phenyl]-2-methoxy-pyridine-3-carbaldehyde (70 mg, 0.18 mmol), and 3-methoxy-5-(4,4,5,5-tetramethyl-1,3,2-dioxaborolan-2-yl)pyridine-2-carbaldehyde (70 mg, 0.27 mmol) were suspended in 1,4-dioxane /water (4:1, 3 mL), then the solution was heated at 105 °C for 20 minutes. Reaction was cooled to room temperature, diluted with 10 mL water, and extracted with EtOAc (3 x 5 mL). The combined organics were concentrated under reduced pressure and the crude sample was purified by silica gel chromatography (0-50% EtOAc/hexane) to afford 3-chloro-4-(2-chloro-3-(5-formyl-6-methoxypyridin-2-yl)phenyl)-5'-methoxy-[2,3'-bipyridine]-6'-carbaldehyde (24 mg, 27 % yield) as a light yellow solid . MS: *m*/*z* found 494, 496 M+H]⁺.

### (b) 1-(4-(((6-(3-(6'-(((1-Acetylpiperidin-4-yl)amino)methyl)-3-chloro-5'-methoxy-[2,3'-bipyridin]-4-yl)-2-chlorophenyl)-2-methoxypyridin-3-yl)methyl)amino)piperidin-1-yl)ethan-1-one

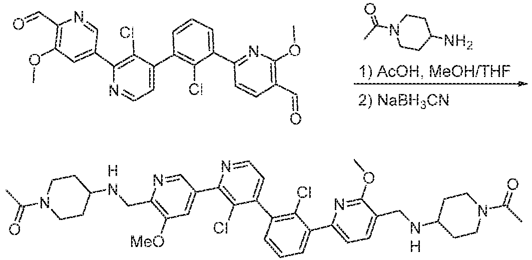

3-Chloro-4-(2-chloro-3-(5-formyl-6-methoxypyridin-2-yl)phenyl)-5'-methoxy-[2,3'-bipyridine]-6'-carbaldehyde (12 mg, 0.02 mmol), acetic acid (3 mg, 0.05 mmol), and 1-(4-amino-1-piperidyl)ethanone (14 mg, 0.10 mmol) were dissolved in MeOH/THF (1:1, 1 mL), then the solution was stirred at 25 °C for 2 hours. Sodium cyanoborohydride (6 mg, 0.10 mmol) was added and the resulting mixture was stirred at 25 °C for 1 hour. Reaction was subsequently diluted with water (2 mL) and extracted with EtOAc (3 x 2 mL). The combined organics were further washed with brine, dried over sodium sulfate, filtered, and concentrated under reduced pressure. Crude sample was purified by reverse phase HPLC to afford 1-(4-(((6-(3-(6'-(((1-acetylpiperidin-4-yl)amino)methyl)-3-chloro-5'-methoxy-[2,3'-bipyridin]-4-yl)-2-chlorophenyl)-2-methoxypyridin-3-yl)methyl)amino)piperidin-1-yl)ethan-1-one (9 mg, 50 % yield) as a white solid (formic acid salt). LC/MS: *m*/*z* found 746, 748 [M+H] ⁺, retention time = 2.07 min (Method C). ¹H NMR (400 MHz, Methanol-*d*₄) δ 8.72 - 8.68 (m, 1H), 8.51 (s, 1H), 8.49 (d, 1H), 7.83 (d, 1H), 7.79 (s, 1H), 7.75 - 7.69 (m, 1H), 7.57 (t, 1H), 7.52 - 7.48 (m, 1H), 7.43 (d, 1H), 7.30 (d, 1H), 4.59 (t, 2H), 4.35 (s, 2H), 4.07 (s, 2H), 4.04 (d, 3H), 4.00 (t, 5H), 3.18 (t, 3H), 2.70 (t, 2H), 2.12 (d, 11H), 1.50 (m, 4H).

### Example 573: 2-((3-Chloro-4-(2-chloro-3-(6-methoxy-5-((7-oxo-2,6-diazaspiro[3.4]octan-2-yl)methyl)pyridin-2-yl)phenyl)-5'-methoxy-[2,3'-bipyridin]-6'-yl)methyl)-2,6-diazaspiro[3.4]octan-7-one (376)

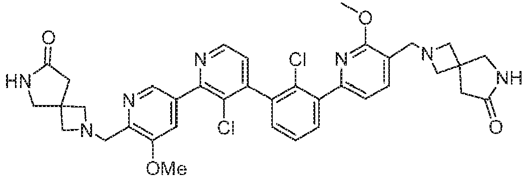

2-((3-Chloro-4-(2-chloro-3-(6-methoxy-5-((7-oxo-2,6-diazaspiro[3.4]octan-2-yl)methyl)pyridin-2-yl)phenyl)-5'-methoxy-[2,3'-bipyridin]-6'-yl)methyl)-2,6-diazaspiro[3.4]octan-7-one was prepared in similar manner as described for Example 572, replacing 1-(4-amino-1-piperidyl)-ethanone with 2,6-diazaspiro[3.4]octan-7-one in step (b). Product was obtained as a white solid (formic acid salt). LC/MS: *m*/*z* found 714, 716 [M+H]⁺, retention time = 1.98 min (Method C). ¹H NMR (400 MHz, Methanol-*d*₄) δ 8.70 (dd, 1H), 8.49 (s, 1H), 8.39 (s, 2H), 7.81 - 7.74 (m, 2H), 7.74 - 7.68 (m, 1H), 7.56 (t, 1H), 7.52 - 7.47 (m, 1H), 7.42 (d, 1H), 7.28 (d, 1H), 4.55 (s, 2H), 4.21 (s, 4H), 4.03 - 3.97 (m, 8H), 3.76 (s, 4H), 3.70 (d, 2H), 3.62 (d, 2H), 2.74 (d, 2H), 2.63 (d, 2H).

### Example 574: 1-(6-((6-(3-(6'-((6-Acetyl-2,6-diazaspiro[3.3]heptan-2-yl)methyl)-3-chloro-5'-methoxy-[2,3'-bipyridin]-4-yl)-2-chlorophenyl)-2-methoxypyridin-3-yl)methyl)-2,6-diazaspiro[3.3]heptan-2-yl)ethan-1-one (428)

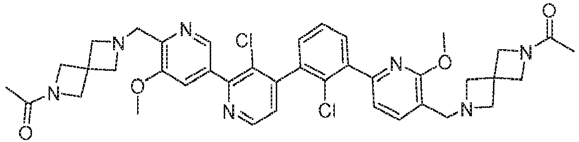

1-(6-((6-(3-(6'-((6-Acetyl-2,6-diazaspiro[3.3]heptan-2-yl)methyl)-3-chloro-5'-methoxy-[2,3'-bipyridin]-4-yl)-2-chlorophenyl)-2-methoxypyridin-3-yl)methyl)-2,6-diazaspiro[3.3]heptan-2-yl)ethan-1-one was prepared in similar manner as described for Example 572, replacing 1-(4-amino-1-piperidyl)-ethanone with 1-(2,6-diazaspiro[3.3]heptan-2-yl)ethenone in step (b). Product was obtained as a white solid (formic acid salt). LC/MS: *m*/*z* found 742, 744 [M+H]⁺, retention time = 2.09 min (Method C). ¹H NMR (400 MHz, Methanol-*d*₄) δ 8.70 (d, 1H), 8.48 (s, 1H), 7.79 (s, 1H), 7.72 (t, 2H), 7.56 (t, 1H), 7.50 (d, 1H), 7.42 (d, 1H), 7.27 (d, 1H), 4.46 (s, 2H), 4.40 (s, 2H), 4.32 (s, 2H), 4.26 (s, 3H), 4.16 (s, 2H), 4.08 (s, 2H), 4.01 (d, 3H), 3.98 (d, 4H), 3.90 (s, 2H), 3.77 (s, 4H), 1.85 (d, 6H).

### Example 575: N-((4-(3-(5-(((2-Oxaspiro[3.3]heptan-6-yl)amino)methyl)-6-methoxypyridin-2-yl)-2-chlorophenyl)-3-chloro-5'-methoxy-[2,3'-bipyridin]-6'-yl)methyl)-2-oxaspiro[3.3]heptan-6-amine (429)

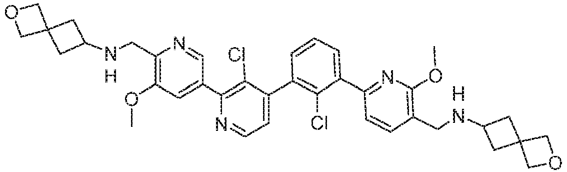

N-((4-(3-(5-(((2-Oxaspiro[3.3]heptan-6-yl)amino)methyl)-6-methoxypyridin-2-yl)-2-chlorophenyl)-3-chloro-5'-methoxy-[2,3'-bipyridin]-6'-yl)methyl)-2-oxaspiro[3.3]heptan-6-amine was prepared in similar manner as described for Example 572, replacing 1-(4-amino-1-piperidyl)-ethanone with 2-oxaspiro[3.3]heptan-6-amine in step (b). Product was obtained as a white solid (formic acid salt). LC/MS: *m*/*z* found 688, 690 [M+H]⁺, retention time = 2.10 min (Method C). ¹H NMR (400 MHz, Methanol-*d*₄) δ 8.71 (d, 1H), 8.51 (s, 1H), 8.46 (s, 1H), 7.83 (d, 1H), 7.80 (s, 1H), 7.72 (d, 1H), 7.57 (t, 1H), 7.51 (d, 1H), 7.44 (d, 1H), 7.32 (d, 1H), 4.73 (d, 4H), 4.63 (d, 4H), 4.26 (s, 2H), 4.09 - 4.03 (m, 5H), 3.99 (d, 3H), 3.79 - 3.64 (m, 1H), 3.58 (q, 1H), 2.69 (m, 4H), 2.41 (t, 2H), 2.33 (t, 2H).

### Example 576: 2-((3-Chloro-4-(2-chloro-3-(6-methoxy-5-((6-oxo-2,5-diazaspiro[3.4]octan-2-yl)methyl)pyridin-2-yl)phenyl)-5'-methoxy-[2,3'-bipyridin]-6'-yl)methyl)-2,5-diazaspiro[3.4]octan-6-one (430)

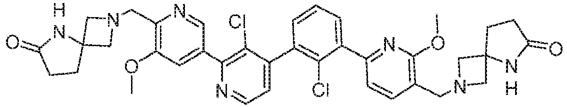

2-((3-Chloro-4-(2-chloro-3-(6-methoxy-5-((6-oxo-2,5-diazaspiro[3.4]octan-2-yl)methyl)pyridin-2-yl)phenyl)-5'-methoxy-[2,3'-bipyridin]-6'-yl)methyl)-2,5-diazaspiro[3.4]octan-6-one was prepared in similar manner as described for Example 572, replacing 1-(4-amino-1-piperidyl)-ethanone with 2,5-diazaspiro[3.4]octan-6-one in step (b). Product was obtained as a white solid (formic acid salt). LC/MS: *m*/*z* found 714, 716 [M+H]⁺, retention time = 2.03 min (Method C). ¹H NMR (400 MHz, Methanol-*d*₄) δ 8.69 (d, 1H), 8.47 (s, 1H), 8.40 (s, 1H), 7.78 (s, 1H), 7.71 (t, 2H), 7.55 (t, 1H), 7.49 (d, 1H), 7.41 (d, 1H), 7.26 (d, 1H), 4.34 (s, 2H), 4.10 (d, 2H), 4.03 - 3.96 (m, 6H), 3.93 (d, 2H), 3.83 (s, 2H), 3.71 - 3.64 (m, 2H), 3.51 (d, 2H), 2.53 - 2.34 (m, 8H).

### Example 577: N-((6-(2-Chloro-3-(3-chloro-5'-methoxy-6'-(((tetrahydro-2H-pyran-4-yl)amino)methyl)-[2,3'-bipyridin]-4-yl)phenyl)-2-methoxypyridin-3-yl)methyl)tetrahydro-2H-pyran-4-amine (431)

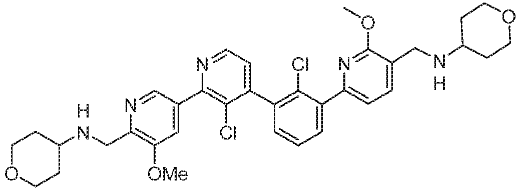

N-((6-(2-Chloro-3-(3-chloro-5'-methoxy-6'-(((tetrahydro-2H-pyran-4-yl)amino)methyl)-[2,3'-bipyridin]-4-yl)phenyl)-2-methoxypyridin-3-yl)methyl)tetrahydro-2H-pyran-4-amine was prepared in similar manner as described for Example 572, replacing 1-(4-amino-1-piperidyl)-ethanone with tetrahydropyran-4-amine in step (b). Product was obtained as a white solid (formic acid salt). LC/MS: *m*/*z* found 664, 666 [M+H]⁺, retention time = 2.10 min (Method C). ¹H NMR (400 MHz, Methanol-*d*₄) δ 8.70 (t, 1H), 8.51 (s, 2H), 7.83 (d, 1H), 7.79 (s, 1H), 7.72 (d, 1H), 7.57 (t, 1H), 7.50 (d, 1H), 7.43 (d, 1H), 7.30 (d, 1H), 4.34 (d, 2H), 4.13 - 3.96 (m, 13H), 3.44 (t, 4H), 3.12 (s, 1H), 2.11 - 2.00 (m, 4H), 1.76 - 1.53 (m, 4H).

### Example 578: 2-((6-(3-(6'-(((1-acetylpiperidin-4-yl)amino)methyl)-3-chloro-5'-methoxy-[2,3'-bipyridin]-4-yl)-2-chlorophenyl)-2-methoxypyridin-3-yl)methyl)-2,6-diazaspiro[3.4]octan-7-one (547)

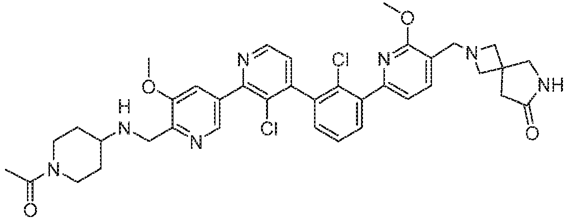

### (a) 3-Chloro-4-(2-chloro-3-(6-methoxy-5-((7-oxo-2,6-diazaspiro[3.4]octan-2-yl)methyl)pyridin-2-yl)phenyl)-5'-methoxy-[2,3'-bipyridine]-6'-carbaldehyde

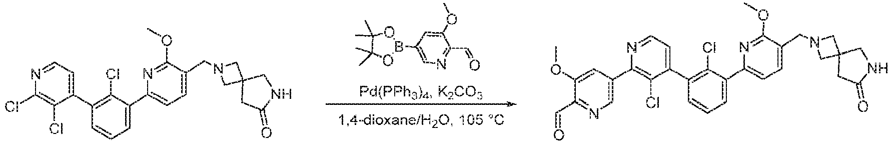

Tetrakis(triphenylphosphine)palladium(0) (115 mg, 0.10 mmol), potassium carbonate (205 mg, 1.49 mmol), 2-[[6-[2-chloro-3-(2,3-dichloro-4-pyridyl)phenyl]-2-methoxy-3-pyridyl]methyl]-2,7-diazaspiro[3.4]octan-6-one (250 mg, 0.50 mmol), and 3-methoxy-5-(4,4,5,5-tetramethyl-1,3,2-dioxaborolan-2-yl)pyridine-2-carbaldehyde (170 mg, 0.65 mmol) were suspended in 1,4-dioxane/water (4:1, 8 mL), then the solution was heated at 105 °C for 20 minutes. Reaction was cooled to room temperature, diluted with 15 mL water, and extracted with EtOAc (3 x 10 mL). The combined organics were concentrated under reduced pressure and the crude sample was purified by silica gel chromatography (0-10 % DCM/MeOH) to afford 3-chloro-4-(2-chloro-3-(6-methoxy-5-((7-oxo-2,6-diazaspiro[3.4]octan-2-yl)methyl)pyridin-2-yl)phenyl)-5'-methoxy-[2,3'-bipyridine]-6'-carbaldehyde (79 mg, 26 % yield) as a dark yellow foam. MS: *m*/*z* found 604, 606 [M+H]⁺.

### (b) 2-((6-(3-(6'-(((1-Acetylpiperidin-4-yl)amino)methyl)-3-chloro-5'-methoxy-[2,3'-bipyridin]-4-yl)-2-chlorophenyl)-2-methoxypyridin-3-yl)methyl)-2,6-diazaspiro [3.4] octan-7-one

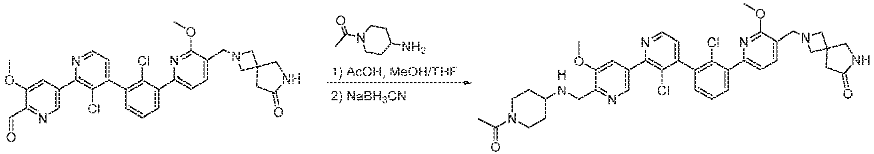

3-Chloro-4-(2-chloro-3-(6-methoxy-5-((7-oxo-2,6-diazaspiro[3.4]octan-2-yl)methyl)pyridin-2-yl)phenyl)-5'-methoxy-[2,3'-bipyridine]-6'-carbaldehyde (19 mg, 0.03 mmol), acetic acid (4 mg, 0.06 mmol), and 1-(4-amino-1-piperidyl)ethanone (13 mg, 0.09 mmol) were dissolved in MeOH/THF (1:1, 1 mL), then the solution was stirred at 25 °C for 2 hours. Sodium cyanoborohydride (56 mg, 0.09 mmol) was added and the resulting mixture was stirred at 25 °C for 1 hour. Reaction was filtered through celite, and the filtrate was concentrated under reduced pressure. Crude sample was purified by reverse phase HPLC to afford 2-((6-(3-(6'-(((1-acetylpiperidin-4-yl)amino)methyl)-3-chloro-5'-methoxy-[2,3'-bipyridin]-4-yl)-2-chlorophenyl)-2-methoxypyridin-3-yl)methyl)-2,6-diazaspiro[3.4]octan-7-one (12 mg, 51 % yield) as a white solid (formic acid salt). LC/MS: *m*/*z* found 730, 732 [M+H] ⁺, retention time = 2.00 min (Method C). ¹H NMR (400 MHz, Methanol-*d*₄) δ 8.71 (d, 1H), 8.53 (s, 1H), 7.81 (s, 1H), 7.73 (dd, 2H), 7.56 (t, 1H), 7.51 (d, 1H), 7.42 (d, 1H), 7.27 (d, 1H), 4.66 (d, 1H), 4.44 (s, 2H), 4.07 (d, 1H), 4.01 (s, 6H), 3.90 (s, 2H), 3.62 (d, 6H), 3.45 (s, 1H), 3.19 (t, 1H), 2.69 (t, 1H), 2.61 (s, 2H), 2.24 (t, 2H), 2.13 (s, 3H), 1.60 (dd, 2H).

### Example 579: 2-((6-(2-Chloro-3-(3-chloro-5'-methoxy-6'-(((tetrahydro-2H-pyran-4-yl)amino)methyl)-[2,3'-bipyridin]-4-yl)phenyl)-2-methoxypyridin-3-yl)methyl)-2,6-diazaspiro[3.4]octan-7-one (548)

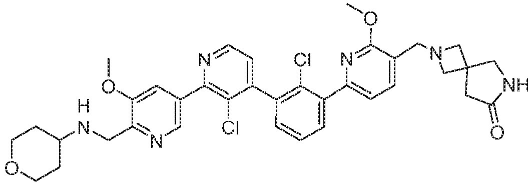

2-((6-(2-Chloro-3-(3-chloro-5'-methoxy-6'-(((tetrahydro-2H-pyran-4-yl)amino)methyl)-[2,3'-bipyridin]-4-yl)phenyl)-2-methoxypyridin-3-yl)methyl)-2,6-diazaspiro[3.4]octan-7-one was prepared in similar manner as described for Example 578, replacing 1-(4-amino-1-piperidyl)ethenone with tetrahydropyran-4-amine in step (b). Product was obtained as a white solid (formic acid salt). LC/MS: *m*/*z* found 689, 691 [M+H]⁺, retention time = 2.03 min (Method C). ¹H NMR (400 MHz, Methanol-*d*₄) δ 8.72 - 8.68 (m, 1H), 8.53 (s, 1H), 8.50 (s, 1H), 7.81 (d, 1H), 7.71 (d, 2H), 7.58 - 7.52 (m, 1H), 7.51 (d, 1H), 7.41 (d, 1H), 7.25 (d, 1H), 4.42 (s, 2H), 4.05 (d, 2H), 4.00 (d, 6H), 3.78 (s, 2H), 3.59 (s, 2H), 3.48 (d, 6H), 3.43 (s, 1H), 2.59 (s, 2H), 2.11 (d, 2H), 1.82 - 1.66 (m, 2H).

### Example 580: 2-((6-(2-Chloro-3-(3-chloro-6'-((((1r,4r)-4-hydroxycyclohexyl)amino)methyl)-5'-methoxy-[2,3'-bipyridin]-4-yl)phenyl)-2-methoxypyridin-3-yl)methyl)-2,6-diazaspiro[3.4]octan-7-one (549)

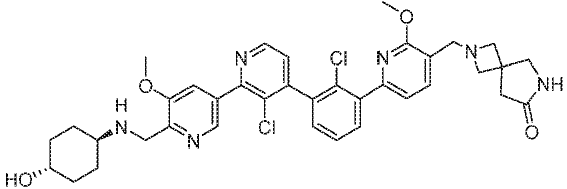

2-((6-(2-Chloro-3-(3-chloro-6'-((((1r,4r)-4-hydroxycyclohexyl)amino)methyl)-5'-methoxy-[2,3'-bipyridin]-4-yl)phenyl)-2-methoxypyridin-3-yl)methyl)-2,6-diazaspiro[3.4]octan-7-one was prepared in similar manner as described for Example 578, replacing 1-(4-amino-1-piperidyl)ethenone with (1r,4r)-4-aminocyclohexan-1-ol in step (b). Product was obtained as a white solid (formic acid salt). LC/MS: *m*/*z* found 703, 705 [M+H]⁺, retention time = 2.00 min (Method C). ¹H NMR (400 MHz, Methanol-*d*₄) δ 8.71 (d, 1H), 8.53 (s, 1H), 7.81 (s, 1H), 7.75 - 7.67 (m, 2H), 7.55 (t, 1H), 7.52 - 7.49 (m, 1H), 7.41 (d, 1H), 7.25 (d, 1H), 4.43 (s, 2H), 4.00 (d, 6H), 3.82 (s, 2H), 3.57 (d, 7H), 3.20 (s, 1H), 2.60 (d, 2H), 2.24 (d, 2H), 2.07 (d, 2H), 1.56 (q, 2H), 1.38 (t, 2H).

### Example 581: (S)-2-((6-(2-Chloro-3-(3-chloro-5'-methoxy-6'-((((5-oxopyrrolidin-2-yl)methyl)amino)methyl)-[2,3'-bipyridin]-4-yl)phenyl)-2-methoxypyridin-3-yl)methyl)-2,6-diazaspiro[3.4]octan-7-one (550)

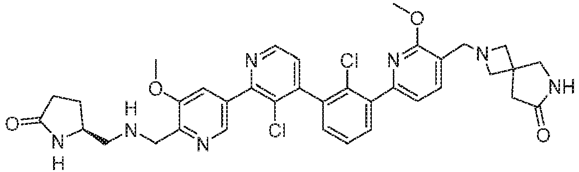

(S)-2-((6-(2-Chloro-3-(3-chloro-5'-methoxy-6'-((((5-oxopyrrolidin-2-yl)methyl)amino)methyl)-[2,3'-bipyridin]-4-yl)phenyl)-2-methoxypyridin-3-yl)methyl)-2,6-diazaspiro[3.4]octan-7-one was prepared in similar manner as described for Example 578, replacing 1-(4-amino-1-piperidyl)ethenone with (5S)-5-(aminomethyl)pyrrolidin-2-one in step (b). Product was obtained as a white solid (formic acid salt). LC/MS: *m*/*z* found 702, 704 [M+H]⁺, retention time = 1.98 min (Method C). ¹H NMR (400 MHz, Methanol-*d*₄) δ 8.70 (dd, 1H), 8.52 (d, 1H), 8.41 (s, 1H), 7.79 (s, 1H), 7.76 (d, 1H), 7.71 (d, 1H), 7.56 (t, 1H), 7.50 (d, 1H), 7.45 - 7.39 (m, 1H), 7.28 (d, 1H), 4.33 (d, 2H), 4.02 (d, 4H), 3.99 (d, 3H), 3.97 (s, 2H), 3.72 (s, 4H), 3.62 (d, 2H), 3.12 (q, 2H), 2.63 (d, 2H), 2.46 - 2.29 (m, 3H), 1.96 - 1.84 (m, 1H).

### Example 582: 1-(4-(((4-(4'-(((1-acetylpiperidin-4-yl)amino)methyl)-2-chloro-3'-methoxy-[1,1'-biphenyl]-3-yl)-3-chloro-5'-methoxy-[2,3'-bipyridin]-6'-yl)methyl)amino)piperidin-1-yl)ethan-1-one (500)

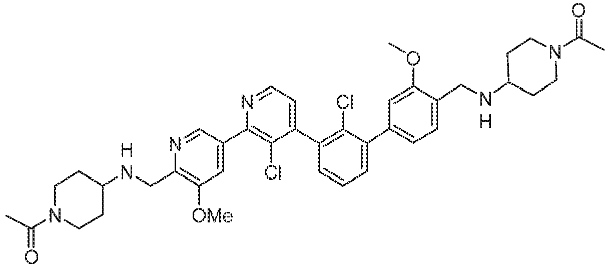

### (a) 4-(3-Bromo-2-chlorophenyl)-3-chloro-5'-methoxy-[2,3'-bipyridine]-6'-carbaldehyde

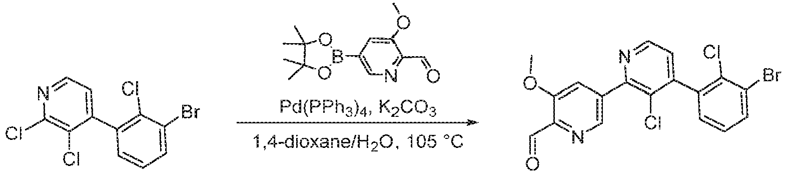

Tetrakis(triphenylphosphine)palladium(0) (103 mg, 0.09 mmol), potassium carbonate (184 mg, 1.33 mmol), 4-(3-bromo-2-chloro-phenyl)-2,3-dichloro-pyridine (150 mg, 0.44 mmol), and 3-methoxy-5-(4,4,5,5-tetramethyl-1,3,2-dioxaborolan-2-yl)pyridine-2-carbaldehyde (140 mg, 0.53 mmol) were suspended in 1,4-dioxane/water (4:1, 5 mL), then the solution was heated at 105 °C for 20 minutes. Reaction was cooled to room temperature, diluted with 10 mL water, and extracted with EtOAc (3 x 5 mL). The combined organics were concentrated under reduced pressure and the crude sample was purified by silica gel chromatography (5-100% EtOAc/hexane) to afford 4-(3-bromo-2-chlorophenyl)-3-chloro-5'-methoxy-[2,3'-bipyridine]-6'-carbaldehyde (62 mg, 32 % yield) as a yellow oil. MS: *m*/*z* found 438, 440 [M+H] ⁺.

### (b) 3-Chloro-4-(2-chloro-4'-formyl-3'-methoxy-[1,1'-biphenyl]-3-yl)-5'-methoxy-[2,3'-bipyridine]-6'-carbaldehyde

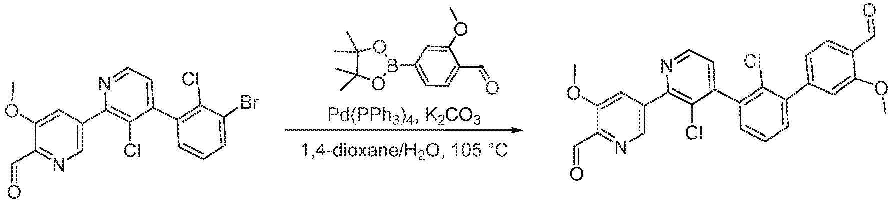

Tetrakis(triphenylphosphine)palladium(0) (33 mg, 0.03 mmol), potassium carbonate (59 mg, 0.42 mmol), 4-(3-bromo-2-chlorophenyl)-3-chloro-5'-methoxy-[2,3'-bipyridine]-6'-carbaldehyde (62 mg, 0.14 mmol), and 2-methoxy-4-(4,4,5,5-tetramethyl-1,3,2-dioxaborolan-2-yl)benzaldehyde (48 mg, 0.18 mmol) were suspended in 1,4-dioxane/water (4:1, 2 mL), then the solution was heated at 105 °C for 20 minutes. Reaction was cooled to room temperature, diluted with 10 mL water, and extracted with EtOAc (3 x 5 mL). The combined organics were concentrated under reduced pressure and the crude sample was purified by silica gel chromatography (10-100 % EtOAc/hexane) to afford 3-chloro-4-(2-chloro-4'-formyl-3'-methoxy-[1,1'-biphenyl]-3-yl)-5'-methoxy-[2,3'-bipyridine]-6'-carbaldehyde (40 mg, 57 % yield) as a yellow foam. MS: *m*/*z* found 493, 495 M+H] ⁺.

### (c) 1-(4-(((4-(4'-(((1-Acetylpiperidin-4-yl)amino)methyl)-2-chloro-3'-methoxy-[1,1'-biphenyl]-3-yl)-3-chloro-5'-methoxy-[2,3'-bipyridin]-6'-yl)methyl)amino)piperidin-1-yl)ethan-1-one

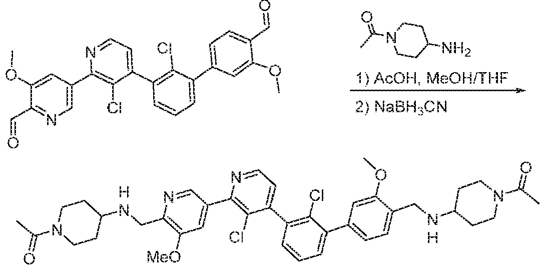

3-Chloro-4-(2-chloro-4'-formyl-3'-methoxy-[1,1'-biphenyl]-3-yl)-5'-methoxy-[2,3'-bipyridine]-6'-carbaldehyde (20 mg, 0.04 mmol), acetic acid (5 mg, 0.08 mmol) and 1-(4-amino-1-piperidyl)ethanone (17 mg, 0.12 mmol) were dissolved in MeOH/THF (1:1, 1 mL), then the solution was stirred at 25 °C for 2 hours. Sodium cyanoborohydride (8 mg, 0.12 mmol) was added and the resulting mixture was stirred at 25 °C for 1 hour. Reaction was filtered through celite, and the filtrate was concentrated under reduced pressure. Crude sample was purified by reverse phase HPLC to afford 1-(4-(((4-(4'-(((1-acetylpiperidin-4-yl)amino)methyl)-2-chloro-3'-methoxy-[1,1'-biphenyl]-3-yl)-3-chloro-5'-methoxy-[2,3'-bipyridin]-6'-yl)methyl)amino)piperidin-1-yl)ethan-1-one (13 mg, 43 % yield) as a white solid (formic acid salt). LC/MS: *m*/*z* found 745, 747 [M+H]⁺, retention time = 2.09 min (Method C). ¹H NMR (400 MHz, Methanol-*d*₄) δ 8.69 (d, 1H), 8.53 (s, 1H), 8.47 (d, 1H), 7.75 (s, 1H), 7.53 (d, 2H), 7.48 (d, 1H), 7.45 - 7.38 (m, 2H), 7.12 (s, 1H), 7.07 (d, 1H), 4.55 (t, 2H), 4.17 (s, 2H), 4.09 (s, 2H), 3.98 (t, 5H), 3.93 (d, 3H), 3.21 - 2.99 (m, 4H), 2.69 (t, 2H), 2.11 (d, 10H), 1.58 - 1.30 (m, 4H).

### Example 583: 2-((3-Chloro-4-(2-chloro-3'-methoxy-4'-((7-oxo-2,6-diazaspiro[3.4]octan-2-yl)methyl)-[1,1'-biphenyl]-3-yl)-5'-methoxy-[2,3'-bipyridin]-6'-yl)methyl)-2,6-diazaspiro[3.4]octan-7-one (501)

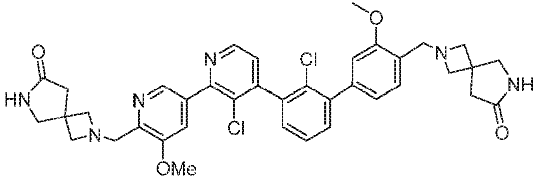

2-((3-Chloro-4-(2-chloro-3'-methoxy-4'-((7-oxo-2,6-diazaspiro[3.4]octan-2-yl)methyl)-[1,1'-biphenyl]-3-yl)-5'-methoxy-[2,3'-bipyridin]-6'-yl)methyl)-2,6-diazaspiro[3.4]octan-7-one was prepared in similar manner as described for Example 582, utilizing intermediate 3-chloro-4-(2-chloro-4'-formyl-3'-methoxy-[1,1'-biphenyl]-3-yl)-5'-methoxy-[2,3'-bipyridine]-6'-carbaldehyde and replacing 1-(4-amino-1-piperidyl)ethanone with 2,6-diazaspiro[3.4]octan-7-one in step (c). Product was obtained as a white solid (formic acid salt). LC/MS: *m*/*z* found 713, 715 [M+H] ⁺, retention time = 2.02 min (Method C).¹H NMR (400 MHz, Methanol-*d*₄) δ 8.68 (d, 1H), 8.52 (s, 1H), 8.46 - 8.40 (m, 1H), 7.73 (s, 1H), 7.56 - 7.49 (m, 2H), 7.47 (dd, 1H), 7.40 - 7.33 (m, 2H), 7.06 (s, 1H), 7.03 (d, 1H), 4.07 (s, 2H), 3.95 (d, 3H), 3.88 (d, 5H), 3.68 (s, 4H), 3.60 (d, 8H), 2.63 - 2.57 (m, 4H).

### Example 584: 1-(4-(((5-(3-(6'-(((1-acetylpiperidin-4-yl)amino)methyl)-3-chloro-5'-methoxy-[2,3'-bipyridin]-4-yl)-2-chlorophenyl)-3-methoxypyridin-2-yl)methyl)amino)piperidin-1-yl)ethan-1-one (567)

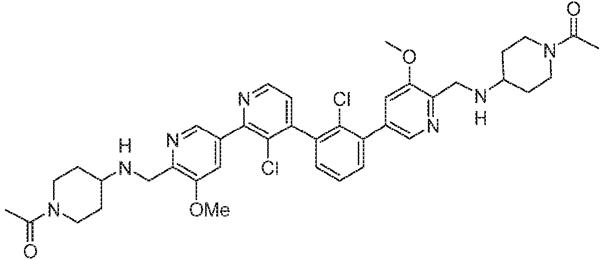

### (a) 3-Chloro-4-(2-chloro-3-(6-formyl-5-methoxypyridin-3-yl)phenyl)-5'-methoxy-[2,3'-bipyridine]-6'-carbaldehyde

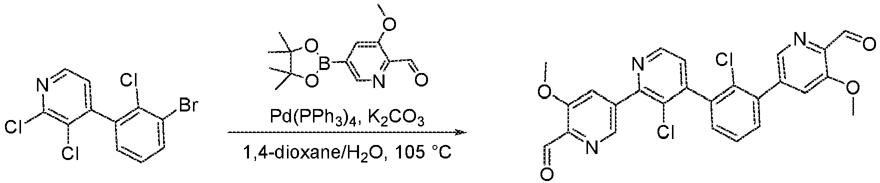

3-Methoxy-5-(4,4,5,5-tetramethyl-1,3,2-dioxaborolan-2-yl)pyridine-2-carbaldehyde (156 mg, 0.59 mmol), tetrakis(triphenylphosphine)palladium(0) (55 mg, 0.05 mmol), potassium carbonate (98 mg, 0.71 mmol), and 4-(3-bromo-2-chloro-phenyl)-2,3-dichloro-pyridine (80 mg, 0.24 mmol) were suspended in 1,4-dioxane /water (4:1, 4 mL), then the solution was heated at 105 °C for 20 minutes. Reaction was cooled to room temperature, diluted with 10 mL water, and extracted with EtOAc (3 x 5 mL). The combined organics were concentrated under reduced pressure and the crude sample was purified by silica gel chromatography (10-80 % EtOAc/hexane) to afford 3-chloro-4-(2-chloro-3-(6-formyl-5-methoxypyridin-3-yl)phenyl)-5'-methoxy-[2,3'-bipyridine]-6'-carbaldehyde (96 mg, 82 % yield) as a brown oil. MS: *m*/*z* found 494, 496 [M+H] ⁺.

### (b) 1-(4-(((5-(3-(6'-(((1-Acetylpiperidin-4-yl)amino)methyl)-3-chloro-5'-methoxy-[2,3'-bipyridin]-4-yl)-2-chlorophenyl)-3-methoxypyridin-2-yl)methyl)amino)piperidin-1-yl)ethan-1-one

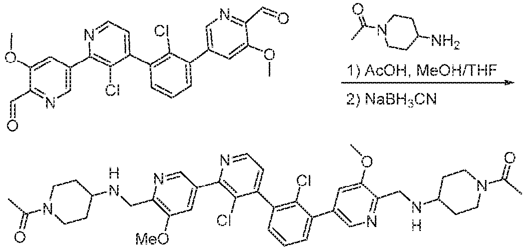

3-Chloro-4-(2-chloro-3-(6-formyl-5-methoxypyridin-3-yl)phenyl)-5'-methoxy-[2,3'-bipyridine]-6'-carbaldehyde (33 mg, 0.07 mmol), acetic acid (8 mg, 0.13 mmol), and 1-(4-amino-1-piperidyl)ethanone (29 mg, 0.20 mmol) were dissolved in MeOH/THF (1:1, 1 mL), then the solution was stirred at 25 °C for 2 hours. Sodium cyanoborohydride (13 mg, 0.20 mmol) was added and the resulting mixture was stirred at 25 °C for 1 hour. Reaction was filtered through celite and the filtrate was concentrated under reduced pressure. Crude sample was purified by reverse phase HPLC to afford 1-(4-(((5-(3-(6'-(((1-acetylpiperidin-4-yl)amino)methyl)-3-chloro-5'-methoxy-[2,3'-bipyridin]-4-yl)-2-chlorophenyl)-3-methoxypyridin-2-yl)methyl)amino)piperidin-1-yl)ethan-1-one (16 mg, 32 % yield) as a white solid (formic acid salt). MS: m/z found 746, 748 [M+H] ⁺, retention time = 2.03 min (Method C). ¹H NMR (400 MHz, Methanol-*d*₄) δ 8.72 (d, 1H), 8.53 (s, 1H), 8.44 (s, 2H), 8.29 (s, 1H), 7.81 (s, 1H), 7.61 (d, 3H), 7.53 - 7.45 (m, 2H), 4.65 (d, 2H), 4.43 (d, 4H), 4.07 (d, 2H), 3.99 (dd, 6H), 3.46 (d, 2H), 3.20 (t, 2H), 2.69 (t, 2H), 2.23 (t, 4H), 2.13 (d, 6H), 1.73 - 1.45 (m, 4H).

### Example 585: 2-((3-Chloro-4-(2-chloro-3-(5-methoxy-6-((7-oxo-2,6-diazaspiro[3.4]octan-2-yl)methyl)pyridin-3-yl)phenyl)-5'-methoxy-[2,3'-bipyridin]-6'-yl)methyl)-2,6-diazaspiro[3.4]octan-7-one (569)

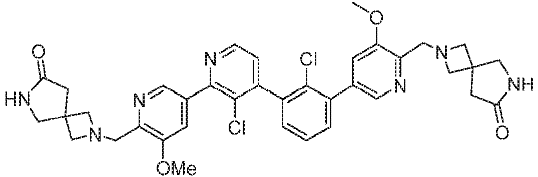

2-((3-Chloro-4-(2-chloro-3-(5-methoxy-6-((7-oxo-2,6-diazaspiro[3.4]octan-2-yl)methyl)pyridin-3-yl)phenyl)-5'-methoxy-[2,3'-bipyridin]-6'-yl)methyl)-2,6-diazaspiro[3.4]octan-7-one was prepared in similar manner as described for **Example 584,** utilizing intermediate 3-chloro-4-(2-chloro-3-(6-formyl-5-methoxypyridin-3-yl)phenyl)-5'-methoxy-[2,3'-bipyridine]-6'-carbaldehyde replacing 1-(4-amino-1-piperidyl)ethanone with 2,6-diazaspiro[3.4]octan-7-one in step (b). Product was obtained as a white solid (formic acid salt). LC/MS: *m*/*z* found 714, 716 [M+H] ⁺, retention time = 1.94 min (Method C). ¹H NMR (400 MHz, Methanol-*d*₄) δ 8.71 (d, 1H), 8.49 (s, 1H), 8.40 (s, 2H), 8.24 (d, 1H), 7.79 (s, 1H), 7.59 (dd, 3H), 7.52 - 7.44 (m, 2H), 4.53 (d, 4H), 4.21 (d, 8H), 3.97 (dd, 6H), 3.70 (s, 4H), 2.74 (d, 4H).

### Example 586: N-((5-(2-Chloro-3-(3-chloro-5'-methoxy-6'-(((tetrahydro-2H-pyran-4-yl)amino)methyl)-[2,3'-bipyridin]-4-yl)phenyl)-3-methoxypyridin-2-yl)methyl)tetrahydro-2H-pyran-4-amine (570)

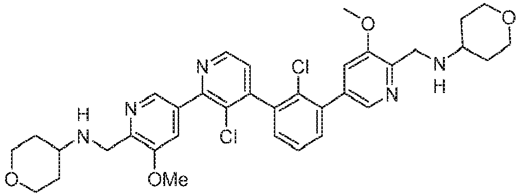

N-((5-(2-Chloro-3-(3-chloro-5'-methoxy-6'-(((tetrahydro-2H-pyran-4-yl)amino)methyl)-[2,3'-bipyridin]-4-yl)phenyl)-3-methoxypyridin-2-yl)methyl)tetrahydro-2H-pyran-4-amine was prepared in similar manner as described for Example 584, utilizing intermediate 3-chloro-4-(2-chloro-3-(6-formyl-5-methoxypyridin-3-yl)phenyl)-5'-methoxy-[2,3'-bipyridine]-6'-carbaldehyde and replacing 1-(4-amino-1-piperidyl)ethenone with tetrahydropyran-4-amine in step (b). Product was obtained as a white solid (formic acid salt). LC/MS: *m*/*z* found 664, 666 [M+H]⁺, retention time = 2.07 min (Method C). ¹H NMR (400 MHz, Methanol-*d*₄) δ 8.73 - 8.69 (m, 1H), 8.53 - 8.49 (m, 2H), 8.28 (d, 1H), 7.80 (s, 1H), 7.63 - 7.56 (m, 3H), 7.49 (m, 2H), 4.40 - 4.31 (m, 4H), 4.07 - 4.01 (m, 4H), 3.99 (dd, 6H), 3.45 (t, 4H), 3.34 (s, 2H), 2.14 - 2.04 (m, 4H), 1.79 - 1.63 (m, 4H).

### Example 587: 1-(4-(((6-(3-(5'-(((1-Acetylpiperidin-4-yl)amino)methyl)-3-chloro-6'-methoxy-[2,2'-bipyridin]-4-yl)-2-chlorophenyl)-2-methoxypyridin-3-yl)methyl)amino)piperidin-1-yl)ethan-1-one (627)

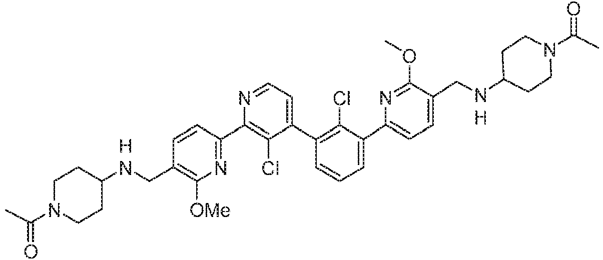

### (a) 3'-Chloro-4'-(2-chloro-3-(5-formyl-6-methoxypyridin-2-yl)phenyl)-6-methoxy-[2,2'-bipyridine]-5-carbaldehyde

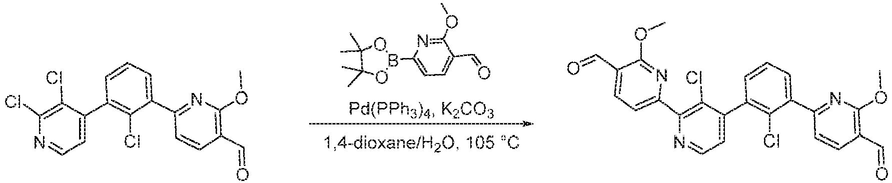

Tetrakis(triphenylphosphine)palladium(0) (117 mg, 0.10 mmol), potassium carbonate (210 mg, 1.52 mmol), 6-[2-chloro-3-(2,3-dichloro-4-pyridyl)phenyl]-2-methoxy-pyridine-3-carbaldehyde (200 mg, 0.51 mmol), and 2-methoxy-6-(4,4,5,5-tetramethyl-1,3,2-dioxaborolan-2-yl)pyridine-3-carbaldehyde (201 mg, 0.76 mmol) were suspended in 1,4-dioxane/water (4:1, 10 mL), then the solution was heated at 105 °C for 20 minutes. Reaction was cooled to room temperature, diluted with 20 mL water, and extracted with EtOAc (3 x 10 mL). The combined organics were concentrated under reduced pressure and the crude sample was purified by silica gel chromatography (0-50% EtOAc/hexane) to afford 3'-chloro-4'-(2-chloro-3-(5-formyl-6-methoxypyridin-2-yl)phenyl)-6-methoxy-[2,2'-bipyridine]-5-carbaldehyde (170 mg, 68 % yield) as a yellow foam. MS: *m*/*z* found 494, 496 [M+H]⁺.

### (b) 1-(4-(((6-(3-(5'-(((1-Acetylpiperidin-4-yl)amino)methyl)-3-chloro-6'-methoxy-[2,2'-bipyridin]-4-yl)-2-chlorophenyl)-2-methoxypyridin-3-yl)methyl)amino)piperidin-1-yl)ethan-1-one

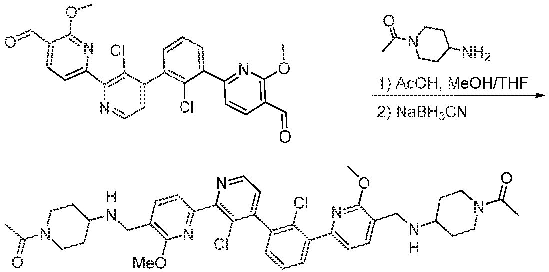

3'-Chloro-4'-(2-chloro-3-(5-formyl-6-methoxypyridin-2-yl)phenyl)-6-methoxy-[2,2'-bipyridine]-5-carbaldehyde (20 mg, 0.04 mmol), acetic acid (5 mg, 0.08 mmol), and 1-(4-amino-1-piperidyl)ethanone (17 mg, 0.12 mmol) were dissolved in MeOH/THF (1:1, 1 mL), then the solution was stirred at 25 °C for 2 hours. Sodium cyanoborohydride (8 mg, 0.12 mmol) was added and the resulting mixture was stirred at 25 °C for 1 hour. Reaction was filtered through celite, and the filtrate was concentrated under reduced pressure. Crude sample was purified by reverse phase HPLC to afford 1-(4-(((6-(3-(5'-(((1-acetylpiperidin-4-yl)amino)methyl)-3-chloro-6'-methoxy-[2,2'-bipyridin]-4-yl)-2-chlorophenyl)-2-methoxypyridin-3-yl)-methyl)-amino)-piperidin-1-yl)ethan-1-one (21 mg, 71 % yield) as a white solid (formic acid salt). LC/MS: *m*/*z* found 746, 748 [M+H]⁺, retention time = 2.19 min (Method C). ¹H NMR (400 MHz, Methanol-*d*₄) δ 8.66 (d, 1H), 8.46 (s, 2H), 7.92 (d, 1H), 7.86 (d, 1H), 7.71 (dd, 1H), 7.57 (t, 1H), 7.50 (d, 1H), 7.46 - 7.39 (m, 2H), 7.33 (d, 1H), 4.61 (d, 2H), 4.18 (d, 4H), 4.06 (d, 8H), 3.19 (t, 4H), 2.70 (t, 2H), 2.20 (d, 4H), 2.12 (s, 6H), 1.64 - 1.38 (m, 4H).

### Example 588: 2-((3'-Chloro-4'-(2-chloro-3-(6-methoxy-5-((7-oxo-2,6-diazaspiro[3.4]octan-2-yl)methyl)pyridin-2-yl)phenyl)-6-methoxy-[2,2'-bipyridin]-5-yl)methyl)-2,6-diazaspiro[3.4]octan-7-one (628)

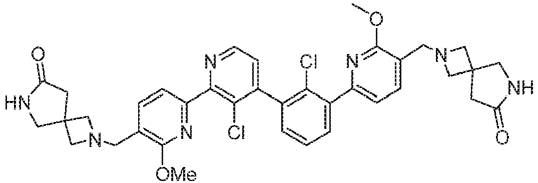

2-((3'-Chloro-4'-(2-chloro-3-(6-methoxy-5-((7-oxo-2,6-diazaspiro[3.4]octan-2-yl)methyl)pyridin-2-yl)phenyl)-6-methoxy-[2,2'-bipyridin]-5-yl)methyl)-2,6-diazaspiro[3.4]octan-7-one was prepared in similar manner as described for Example 587, utilizing intermediate 3'-chloro-4'-(2-chloro-3-(5-formyl-6-methoxypyridin-2-yl)phenyl)-6-methoxy-[2,2'-bipyridine]-5-carbaldehyde and replacing 1-(4-amino-1-piperidyl)ethanone with 2,6-diazaspiro[3.4]octan-7-one in step (b). Product was obtained as a white solid (formic acid salt). LC/MS: *m*/*z* found 714, 716 [M+H]⁺, retention time = 2.02 min (Method C). ¹H NMR (400 MHz, Methanol-*d*₄) δ 8.66 - 8.62 (m, 1H), 8.35 (s, 2H), 7.86 (d, 1H), 7.81 - 7.76 (m, 1H), 7.70 (d, 1H), 7.56 (t, 1H), 7.49 (d, 1H), 7.41 (t, 2H), 7.33 - 7.28 (m, 1H), 4.09 (s, 4H), 4.03 (s, 6H), 3.85 (d, 8H), 3.63 (d, 4H), 2.65 (s, 4H).

### Example 589: N-((6-(2-Chloro-3-(3-chloro-6'-methoxy-5'-(((tetrahydro-2H-pyran-4-yl)amino)methyl)-[2,2'-bipyridin]-4-yl)phenyl)-2-methoxypyridin-3-yl)methyl)tetrahydro-2H-pyran-4-amine (629)

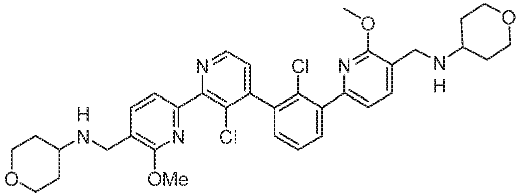

N-((6-(2-Chloro-3-(3-chloro-6'-methoxy-5'-(((tetrahydro-2H-pyran-4-yl)amino)methyl)-[2,2'-bipyridin]|-4-yl)phenyl)-2-methoxypyridin-3-yl)methyl)tetrahydro-2H-pyran-4-amine was prepared in similar manner as described for Example 587, utilizing intermediate 3'-chloro-4'-(2-chloro-3-(5-formyl-6-methoxypyridin-2-yl)phenyl)-6-methoxy-[2,2'-bipyridine]-5-carbaldehyde. and replacing 1-(4-amino-1-piperidyl)ethanone with tetrahydropyran-4-amine in step (b). Product was obtained as a white solid (formic acid salt). LC/MS: *m*/*z* found 664, 666 [M+H]⁺, retention time = 2.17 min (Method C). ¹H NMR (400 MHz, Methanol-*d*₄) δ 8.66 (dd, 1H), 8.50 (s, 1H), 7.92 (d, 1H), 7.86 (d, 1H), 7.72 (d, 1H), 7.57 (t, 1H), 7.54 - 7.49 (m, 1H), 7.48 - 7.41 (m, 2H), 7.33 (d, 1H), 4.22 - 4.13 (m, 4H), 4.07 (d, 8H), 4.02 (s, 2H), 3.45 (t, 4H), 2.07 (d, 4H), 1.75 - 1.56 (m, 4H).

### Example 590: (S)-5-((((6-(2-Chloro-3-(3-chloro-6'-methoxy-5'-(((((S)-5-oxopyrrolidin-2-yl)methyl)amino)methyl)-[2,2'-bipyridin]-4-yl)phenyl)-2-methoxypyridin-3-yl)methyl)amino)methyl)pyrrolidin-2-one (630)

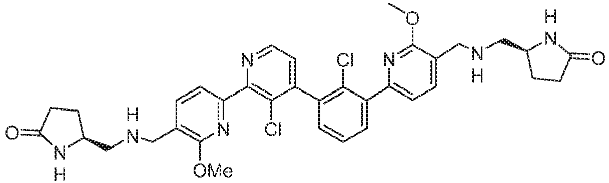

(S)-5-((((6-(2-Chloro-3-(3-chloro-6'-methoxy-5'-(((((S)-5-oxopyrrolidin-2-yl)methyl)amino)methyl)-[2,2'-bipyridin]-4-yl)phenyl)-2-methoxypyridin-3-yl)methyl)amino)methyl)pyrrolidin-2-one was prepared in similar manner as described for Example 587, utilizing intermediate 3'-chloro-4'-(2-chloro-3-(5-formyl-6-methoxypyridin-2-yl)phenyl)-6-methoxy-[2,2'-bipyridine]-5-carbaldehyde replacing 1-(4-amino-1-piperidyl)ethanone with (5S)-5-(aminomethyl)pyrrolidin-2-one in step (b). LC/MS: *m*/*z* found 690, 692 [M+H]⁺, retention time = 2.07 min (Method C). ¹H NMR (400 MHz, Methanol-*d*₄) δ 8.65 (d, 1H), 8.33 (s, 2H), 7.89 (d, 1H), 7.82 (d, 1H), 7.71 (d, 1H), 7.56 (t, 1H), 7.50 (d, 1H), 7.42 (t, 2H), 7.31 (d, 1H), 4.09 (s, 4H), 4.07 - 4.04 (m, 6H), 3.95 (s, 2H), 2.97 (q, 4H), 2.42 - 2.26 (m, 6H), 1.93 - 1.78 (m, 2H).

### Example 591: 1-(4-(((6-(3-(5-(4-(((1-Acetylpiperidin-4-yl)amino)methyl)-3-methoxyphenyl)-4-chloropyridin-3-yl)-2-chlorophenyl)-2-methoxypyridin-3-yl)methyl)amino)piperidin-1-yl)ethan-1-one (641)

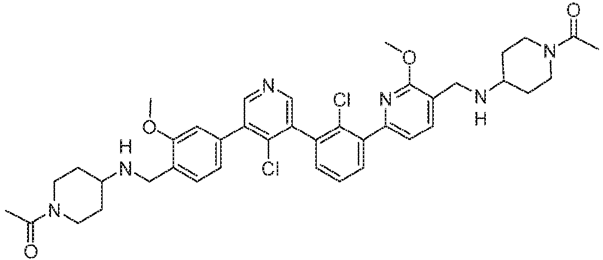

### (a) 6-(2-Chloro-3-(4-chloro-5-(4-formyl-3-methoxyphenyl)pyridin-3-yl)phenyl)-2-methoxynicotinaldehyde

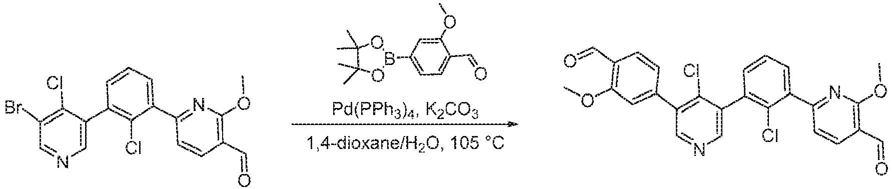

Tetrakis(triphenylphosphine)palladium(0) (47 mg, 0.04 mmol), potassium carbonate (85 mg, 0.62 mmol), 6-[3-(5-bromo-4-chloro-3-pyridyl)-2-chloro-phenyl]-2-methoxy-pyridine-3-carbaldehyde (90 mg, 0.21 mmol), and 2-methoxy-4-(4,4,5,5-tetramethyl-1,3,2-dioxaborolan-2-yl)benzaldehyde (81 mg, 0.31 mmol) were suspended in 1,4-dioxane/water (4:1, 3 mL), then the solution was heated at 105 °C for 20 minutes. Reaction was cooled to room temperature, diluted with 10 mL water, and extracted with EtOAc (3 x 5 mL). The combined organics were concentrated under reduced pressure and the crude sample was purified by silica gel chromatography (0-50% EtOAc/hexane) to afford 6-(2-chloro-3-(4-chloro-5-(4-formyl-3-methoxyphenyl)pyridin-3-yl)phenyl)-2-methoxynicotinaldehyde (23 mg, 23 % yield) as a yellow foam. MS: *m*/*z* found 493, 495 [M+H]⁺.

### (b) 1-(4-(((6-(3-(5-(4-(((1-Acetylpiperidin-4-yl)amino)methyl)-3-methoxyphenyl)-4-chloropyridin-3-yl)-2-chlorophenyl)-2-methoxypyridin-3-yl)methyl)amino)piperidin-1-yl)ethan-1-one

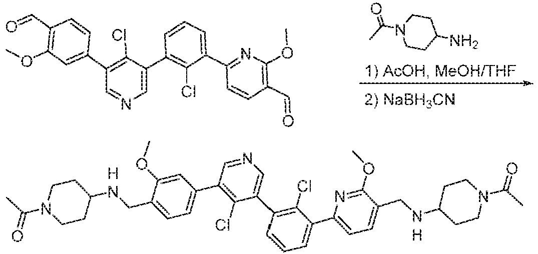

6-(2-Chloro-3-(4-chloro-5-(4-formyl-3-methoxyphenyl)pyridin-3-yl)phenyl)-2-methoxynicotinaldehyde (11 mg, 0.02 mmol), acetic acid (3 mg, 0.04 mmol), and 1-(4-amino-1-piperidyl)ethanone (10 mg, 0.07 mmol) were dissolved in MeOH/THF (1:1, 1 mL), then the solution was stirred at 25 °C for 2 hours. Sodium cyanoborohydride (4 mg, 0.07 mmol) was added and the resulting mixture was stirred at 25 °C for 1 hour. Reaction was filtered through celite, and the filtrate was concentrated under reduced pressure. Crude sample was purified by reverse phase HPLC to afford 1-(4-(((6-(3-(5-(4-(((1-acetylpiperidin-4-yl)amino)methyl)-3-methoxyphenyl)-4-chloropyridin-3-yl)-2-chlorophenyl)-2-methoxypyridin-3-yl)methyl)amino)piperidin-1-yl)ethan-1-one (3 mg, 17 % yield) as a white solid (formic acid salt). LC/MS: *m*/*z* found 745, 747 [M+H]⁺, retention time = 2.12 min (Method C). ¹H NMR (400 MHz, Methanol-*d*₄) δ 8.59 (s, 1H), 8.51 (d, 1H), 8.49 (s, 2H), 7.84 (d, 1H), 7.72 (d, 1H), 7.60 - 7.51 (m, 2H), 7.47 (d, 1H), 7.32 (d, 1H), 7.25 (s, 1H), 7.18 (d, 1H), 4.61 (t, 2H), 4.26 (s, 2H), 4.12 (s, 2H), 4.06 (d, 4H), 3.98 (s, 4H), 3.19 (t, 4H), 2.70 (t, 2H), 2.28 - 2.06 (m, 10H), 1.66 - 1.33 (m, 4H).

### Example 592: 2-(4-(4-Chloro-5-(2-chloro-3-(6-methoxy-5-((7-oxo-2,6-diazaspiro [3.4] octan-2-yl)methyl)pyridin-2-yl)phenyl)pyridin-3-yl)-2-methoxybenzyl)-2,6-diazaspiro[3.4]octan-7-one (642)

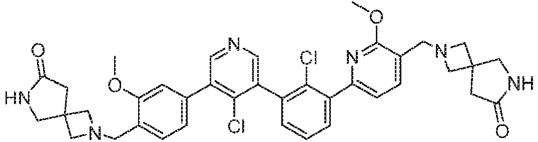

2-(4-(4-Chloro-5-(2-chloro-3-(6-methoxy-5-((7-oxo-2,6-diazaspiro[3.4]octan-2-yl)methyl)pyridin-2-yl)phenyl)pyridin-3-yl)-2-methoxybenzyl)-2,6-diazaspiro[3.4]octan-7-one was prepared in similar manner as described for **Example 591,** utilizing intermediate 6-(2-chloro-3-(4-chloro-5-(4-formyl-3-methoxyphenyl)pyridin-3-yl)phenyl)-2-methoxynicotinaldehyde and replacing 1-(4-amino-1-piperidyl)ethenone with 2,6-diazaspiro-[3.4]octan-7-one in step (b). Product was obtained as a white solid (formic acid salt). LC/MS: *m*/*z* found 713, 715 [M+H]⁺, retention time = 2.00 min (Method C). ¹H NMR (400 MHz, Methanol-*d*₄) δ 8.58 (s, 1H), 8.51 (s, 1H), 8.38 (s, 2H), 7.81 - 7.75 (m, 1H), 7.71 (d, 1H), 7.56 (t, 1H), 7.51 (d, 1H), 7.46 (d, 1H), 7.30 (d, 1H), 7.23 (s, 1H), 7.17 (d, 1H), 4.32 (s, 2H), 4.08 (s, 3H), 4.05 (s, 2H), 4.03 (d, 3H), 3.96 (s, 4H), 3.82 (s, 4H), 3.66 (s, 2H), 3.63 (s, 2H), 2.69 (s, 2H), 2.65 (s, 2H).

### Example 593: 1-(4-(((6-(3-(5-(((1-acetylpiperidin-4-yl)amino)methyl)-4'-chloro-6-methoxy-[2,3'-bipyridin]-5'-yl)-2-chlorophenyl)-2-methoxypyridin-3-yl)methyl)amino)piperidin-1-yl)ethan-1-one (639)

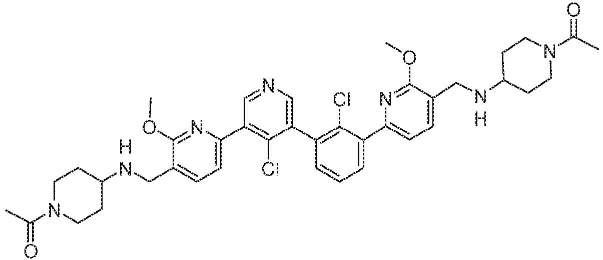

### (a) 6-(3-(5-Bromo-4-chloropyridin-3-yl)-2-chlorophenyl)-2-methoxynicotinaldehyde

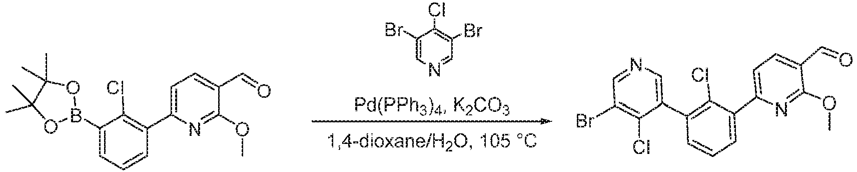

3,5-dibromo-4-chloro-pyridine (203 mg, 0.75 mmol), tetrakis(triphenylphosphine)palladium(0) (124 mg, 0.11 mmol), potassium carbonate (222 mg, 1.61 mmol), and 6-[2-chloro-3-(4,4,5,5-tetramethyl-1,3,2-dioxaborolan-2-yl)phenyl]-2-methoxy-pyridine-3-carbaldehyde (200 mg, 0.54 mmol) were suspended in 1,4-dioxane/water (4:1, 10 mL), then the solution was heated at 105 °C for 20 minutes. Reaction was cooled to room temperature, diluted with 10 mL water, and extracted with EtOAc (3 x 10 mL). The combined organics were concentrated under reduced pressure and the crude sample was purified by silica gel chromatography (10-60 % EtOAc/hexane) to afford 6-(3-(5-bromo-4-chloropyridin-3-yl)-2-chlorophenyl)-2-methoxynicotinaldehyde (184 mg, 79 % yield) as a yellow foam. MS: *m*/*z* found 438, 440 [M+H] ⁺.

### (b) 4'-Chloro-5'-(2-chloro-3-(5-formyl-6-methoxypyridin-2-yl)phenyl)-6-methoxy-[2,3'-bipyridine]-5-carbaldehyde

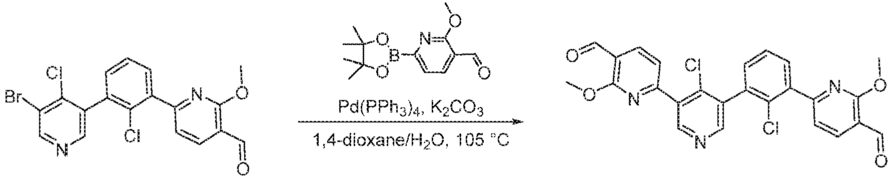

Tetrakis(triphenylphosphine)palladium(0) (47 mg, 0.04 mmol), potassium carbonate (85 mg, 0.62 mmol), 6-(3-(5-bromo-4-chloropyridin-3-yl)-2-chlorophenyl)-2-methoxynicotinaldehyde (90 mg, 0.21 mmol), and 2-methoxy-6-(4,4,5,5-tetramethyl-1,3,2-dioxaborolan-2-yl)pyridine-3-carbaldehyde (81 mg, 0.31 mmol) were suspended in 1,4-dioxane/water (4:1, 4 mL), then the solution was heated at 105 °C for 20 minutes. Reaction was cooled to room temperature, diluted with 10 mL water, and extracted with EtOAc (3 x 5 mL). The combined organics were concentrated under reduced pressure and the crude sample was purified by silica gel chromatography (0-50% EtOAc/hexane) to afford 4'-chloro-5'-(2-chloro-3-(5-formyl-6-methoxypyridin-2-yl)phenyl)-6-methoxy-[2,3'-bipyridine]-5-carbaldehyde (30 mg, 29 % yield) as a yellow solid. MS: *m*/*z* found 494, 496 M+H]⁺.

### (c) 1-(4-(((6-(3-(5-(((1-Acetylpiperidin-4-yl)amino)methyl)-4'-chloro-6-methoxy-[2,3'-bipyridin]-5'-yl)-2-chlorophenyl)-2-methoxypyridin-3-yl)methyl)amino)piperidin-1-yl)ethan-1-one

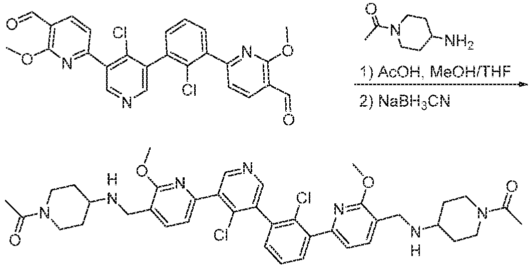

4'-Chloro-5'-(2-chloro-3-(5-formyl-6-methoxypyridin-2-yl)phenyl)-6-methoxy-[2,3'-bipyridine]-5-carbaldehyde (15 mg, 0.03 mmol), acetic acid (4 mg, 0.06 mmol), and 1-(4-amino-1-piperidyl)ethanone (13 mg, 0.09 mmol) were dissolved in MeOH/THF (1:1, 1 mL), then the solution was stirred at 25 °C for 2 hours. Sodium cyanoborohydride (6 mg, 0.09 mmol) was added and the resulting mixture was stirred at 25 °C for 1 hour. Reaction was filtered through celite, and the filtrate was concentrated under reduced pressure. Crude sample was purified by reverse phase HPLC to afford 1-(4-(((6-(3-(5-(((1-acetylpiperidin-4-yl)amino)methyl)-4'-chloro-6-methoxy-[2,3'-bipyridin]-5'-yl)-2-chlorophenyl)-2-methoxypyridin-3-yl)methyl)-amino)-piperidin-1-yl)ethan-1-one (7 mg, 29 % yield) as a white solid (formic acid salt). LC/MS: *m*/*z* found 746, 748 [M+H]⁺, retention time = 2.07 min (Method C). ¹H NMR (400 MHz, Methanol-*d*₄) δ 8.79 (s, 1H), 8.52 (s, 1H), 8.49 (d, 1H), 7.86 (dd, 2H), 7.72 (d, 1H), 7.57 (t, 1H), 7.48 (d, 1H), 7.42 (d, 1H), 7.32 (d, 1H), 4.58 (s, 2H), 4.11 (s, 2H), 4.09 - 4.05 (m, 8H), 4.01 (d, 2H), 3.18 (t, 4H), 2.70 (t, 2H), 2.12 (s, 10H), 1.46 (m, 4H).

### Example 594: 2-((4'-Chloro-5'-(2-chloro-3-(6-methoxy-5-((7-oxo-2,6-diazaspiro[3.4]octan-2-yl)methyl)pyridin-2-yl)phenyl)-6-methoxy-[2,3'-bipyridin]-5-yl)methyl)-2,6-diazaspiro[3.4]octan-7-one (640)

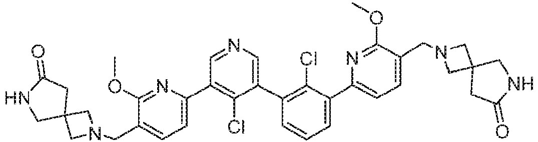

2-((4'-Chloro-5'-(2-chloro-3-(6-methoxy-5-((7-oxo-2,6-diazaspiro[3.4]octan-2-yl)methyl)pyridin-2-yl)phenyl)-6-methoxy-[2,3'-bipyridin]-5-yl)methyl)-2,6-diazaspiro[3.4]octan-7-one was prepared in similar manner as described for Example 593, utilizing intermediate 4'-chloro-5'-(2-chloro-3-(5-formyl-6-methoxypyridin-2-yl)phenyl)-6-methoxy-[2,3'-bipyridine]-5-carbaldehyde and replacing 1-(4-amino-1-piperidyl)ethenone with 2,6-diazaspiro-[3.4]octan-7-one in step (c). Product was obtained as a white solid (formic acid salt). LC/MS: *m*/*z* found 714, 716 [M+H]⁺, retention time = 1.98 min (Method C). ¹H NMR (400 MHz, Methanol-*d*₄) δ 8.78 (s, 1H), 8.51 (s, 1H), 8.38 (s, 2H), 7.81 (dd, 2H), 7.72 (d, 1H), 7.56 (t, 1H), 7.47 (d, 1H), 7.41 (d, 1H), 7.31 (d, 1H), 4.09 (s, 2H), 4.06 - 4.00 (m, 8H), 3.86 (s, 4H), 3.77 (s, 4H), 3.63 (t, 4H), 2.65 (d, 4H).

### Example 595: 1-(4-(((6-(3-(5-(((1-Acetylpiperidin-4-yl)amino)methyl)-3'-chloro-6-methoxy-[2,4'-bipyridin]-2'-yl)-2-chlorophenyl)-2-methoxypyridin-3-yl)methyl)amino)piperidin-1-yl)ethan-1-one (572)

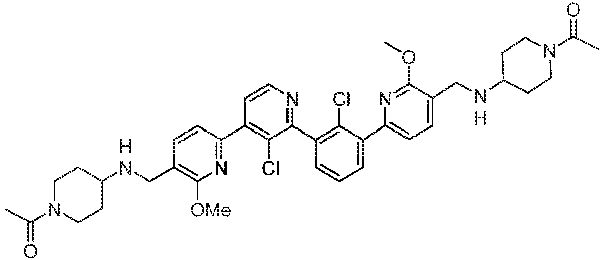

### (a) 3'-Chloro-2'-(2-chloro-3-(5-formyl-6-methoxypyridin-2-yl)phenyl)-6-methoxy-[2,4'-bipyridine]-5-carbaldehyde

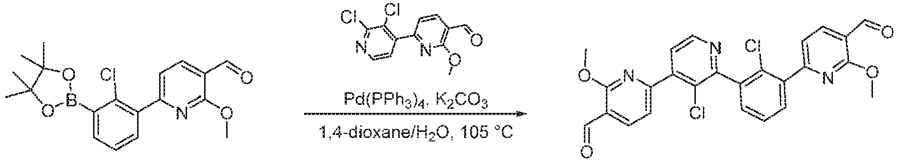

6-(2,3-Dichloro-4-pyridyl)-2-methoxy-pyridine-3-carbaldehyde (80 mg, 0.28 mmol), tetrakis(triphenylphosphine)palladium(0) (65 mg, 0.06 mmol), potassium carbonate (117 mg, 0.85 mmol), and 6-[2-chloro-3-(4,4,5,5-tetramethyl-1,3,2-dioxaborolan-2-yl)phenyl]-2-methoxy-pyridine-3-carbaldehyde (106 mg, 0.28 mmol) were suspended in 1,4-dioxane /water (4:1, 3 mL), then the solution was heated at 105 °C for 20 minutes. Reaction was cooled to room temperature, diluted with 10 mL water, and extracted with EtOAc (3 x 5 mL). The combined organics were concentrated under reduced pressure and the crude sample was purified by silica gel chromatography (10-60 % EtOAc/hexane) to afford 3'-chloro-2'-(2-chloro-3-(5-formyl-6-methoxypyridin-2-yl)phenyl)-6-methoxy-[2,4'-bipyridine]-5-carbaldehyde (138 mg, 99 % yield) as a yellow foam. MS: *m*/*z* found 494, 496 [M+H] ⁺.

### (b) 1-(4-(((6-(3-(5-(((1-Acetylpiperidin-4-yl)amino)methyl)-3'-chloro-6-methoxy-[2,4'-bipyridin]-2'-yl)-2-chlorophenyl)-2-methoxypyridin-3-yl)methyl)amino)piperidin-1-yl)ethan-1-one

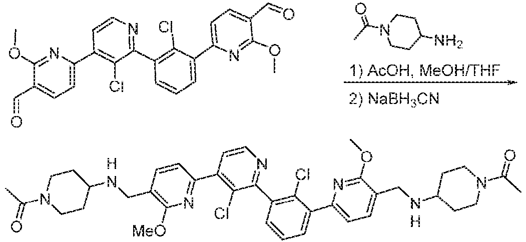

3'-Chloro-2'-(2-chloro-3-(5-formyl-6-methoxypyridin-2-yl)phenyl)-6-methoxy-[2,4'-bipyridine]-5-carbaldehyde (34 mg, 0.07 mmol), acetic acid (8.26 mg, 0.14 mmol), and 1-(4-amino-1-piperidyl)ethanone (29 mg, 0.21 mmol) were dissolved in MeOH/THF (1:1, 1 mL), then the solution was stirred at 25 °C for 2 hours. Sodium cyanoborohydride (13 mg, 0.21 mmol) was added and the resulting mixture was stirred at 25 °C for 1 hour. Reaction was filtered through celite and the filtrate was concentrated under reduced pressure. Crude sample was purified by reverse phase HPLC to afford 1-(4-(((6-(3-(5-(((1-acetylpiperidin-4-yl)amino)methyl)-3'-chloro-6-methoxy-[2,4'-bipyridin]-2'-yl)-2-chlorophenyl)-2-methoxypyridin-3-yl)methyl)amino)piperidin-1-yl)ethan-1-one (23 mg, 45 % yield) as a white solid (formic acid salt). LC/MS: *m*/*z* found 746, 748 [M+H] ⁺, retention time = 2.10 min (Method C). ¹H NMR (400 MHz, Methanol-*d*₄) δ 8.69 - 8.62 (m, 1H), 8.41 (s, 2H), 7.92 (d, 1H), 7.87 (d, 1H), 7.79 - 7.70 (m, 2H), 7.58 (t, 1H), 7.49 (dd, 2H), 7.35 (d, 1H), 4.63 (t, 2H), 4.22 (d, 4H), 4.08 (dd, 6H), 3.37 (s, 2H), 3.20 (t, 2H), 2.70 (t, 2H), 2.22 (t, 4H), 2.12 (d, 6H), 1.66 - 1.39 (m, 4H).

### Example 596: 2-((3'-Chloro-2'-(2-chloro-3-(6-methoxy-5-((7-oxo-2,6-diazaspiro[3.4]octan-2-yl)methyl)pyridin-2-yl)phenyl)-6-methoxy-[2,4'-bipyridin]-5-yl)methyl)-2,6-diazaspiro[3.4]octan-7-one (573)

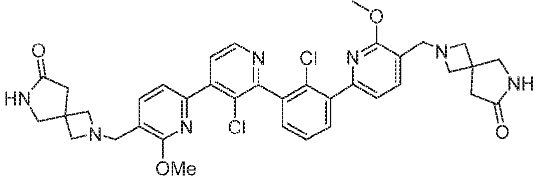

2-((3'-Chloro-2'-(2-chloro-3-(6-methoxy-5-((7-oxo-2,6-diazaspiro[3.4]octan-2-yl)methyl)pyridin-2-yl)phenyl)-6-methoxy-[2,4'-bipyridin]-5-yl)methyl)-2,6-diazaspiro[3.4]octan-7-one was prepared in similar manner as described for Example 595, utilizing intermediate 3'-chloro-2'-(2-chloro-3-(5-formyl-6-methoxypyridin-2-yl)phenyl)-6-methoxy-[2,4'-bipyridine]-5-carbaldehyde replacing 1-(4-amino-1-piperidyl)ethanone with 2,6-diazaspiro[3.4]octan-7-one in step (b). Product was obtained as a white solid (formic acid salt). LC/MS: *m*/*z* found 714, 716 [M+H] ⁺, retention time = 1.97 min (Method C). ¹H NMR (400 MHz, Methanol-*d*₄) δ 8.68 - 8.58 (m, 1H), 8.27 (s, 1H), 7.89 - 7.81 (m, 2H), 7.78 - 7.70 (m, 2H), 7.57 (dd, 1H), 7.52 - 7.44 (m, 2H), 7.35 (dd, 1H), 4.27 (s, 2H), 4.16 (d, 2H), 4.07 (q, 10H), 3.93 (s, 4H), 3.68 - 3.63 (m, 4H), 2.71 - 2.66 (m, 4H).

### Example 597: N-((3'-Chloro-2'-(2-chloro-3-(6-methoxy-5-(((tetrahydro-2H-pyran-4-yl)amino)methyl)pyridin-2-yl)phenyl)-6-methoxy-[2,4'-bipyridin]-5-yl)methyl)tetrahydro-2H-pyran-4-amine (574)

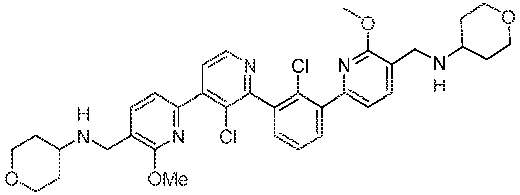

N-((3'-Chloro-2'-(2-chloro-3-(6-methoxy-5-(((tetrahydro-2H-pyran-4-yl)amino)methyl)pyridin-2-yl)phenyl)-6-methoxy-[2,4'-bipyridin]-5-yl)methyl)tetrahydro-2H-pyran-4-amine was prepared in similar manner as described for Example 595, utilizing intermediate 3'-chloro-2'-(2-chloro-3-(5-formyl-6-methoxypyridin-2-yl)phenyl)-6-methoxy-[2,4'-bipyridine]-5-carbaldehyde and replacing 1-(4-amino-1-piperidyl)ethanone with tetrahydropyran-4-amine in step (b). Product was obtained as a white solid (formic acid salt). LC/MS: *m*/*z* found 664, 666 [M+H]⁺, retention time = 2.16 min (Method C).¹H NMR (400 MHz, Methanol-*d*₄) δ 8.68 - 8.60 (m, 1H), 8.48 (d, 2H), 7.91 (d, 1H), 7.86 (d, 1H), 7.75 (dd, 2H), 7.58 (t, 1H), 7.49 (dd, 2H), 7.35 (d, 1H), 4.23 - 4.18 (m, 2H), 4.16 (d, 2H), 4.08 (dd, 6H), 4.07 - 3.98 (m, 4H), 3.45 (t, 4H), 3.23 (s, 1H), 2.05 (q, 4H), 1.66 (t, 4H).

### Example 598: (S)-5-((((3'-Chloro-2'-(2-chloro-3-(6-methoxy-5-(((((S)-5-oxopyrrolidin-2-yl)methyl)amino)methyl)pyridin-2-yl)phenyl)-6-methoxy-[2,4'-bipyridin]-5-yl)methyl)amino)methyl)pyrrolidin-2-one (575)

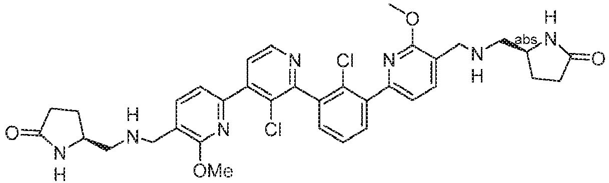

(S)-5-((((3'-Chloro-2'-(2-chloro-3-(6-methoxy-5-(((((S)-5-oxopyrrolidin-2-yl)methyl)amino)methyl)pyridin-2-yl)phenyl)-6-methoxy-[2,4'-bipyridin]-5-yl)methyl)amino)methyl)pyrrolidin-2-one was prepared in similar manner as described for Example 595, utilizing intermediate 3'-chloro-2'-(2-chloro-3-(5-formyl-6-methoxypyridin-2-yl)phenyl)-6-methoxy-[2,4'-bipyridine]-5-carbaldehyde and replacing 1-(4-amino-1-piperidyl)ethanone with (5S)-5-(aminomethyl)pyrrolidin-2-one in step (b). Product was obtained as a white solid (formic acid salt). LC/MS: *m*/*z* found 690, 692 [M+H]⁺, retention time = 2.00 min (Method C). ¹H NMR (400 MHz, Methanol-*d*₄) δ 8.67 - 8.62 (m, 1H), 8.32 (s, 2H), 7.87 (d, 1H), 7.82 (d, 1H), 7.77 (dd, 1H), 7.73 (d, 1H), 7.57 (dd, 1H), 7.47 (dd, 2H), 7.36 - 7.31 (m, 1H), 4.11 (d, 2H), 4.06 (m, 6H), 4.04 (s, 2H), 3.98 - 3.89 (m, 2H), 2.96 (m, 4H), 2.40 - 2.30 (m, 6H), 1.87 (d, 2H).

### Example 599: 2-((2'-(3-(5-(((1-Acetylpiperidin-4-yl)amino)methyl)-6-methoxypyridin-2-yl)-2-chlorophenyl)-3'-chloro-6-methoxy-[2,4'-bipyridin]-5-yl)methyl)-2,6-diazaspiro[3.4]octan-7-one (680)

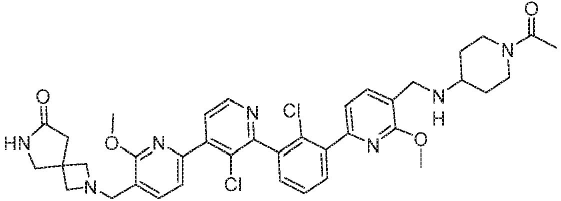

### (a) 2-((2',3'-Dichloro-6-methoxy-[2,4'-bipyridin]-5-yl)methyl)-2,6-diazaspiro [3.4] octan-7-one

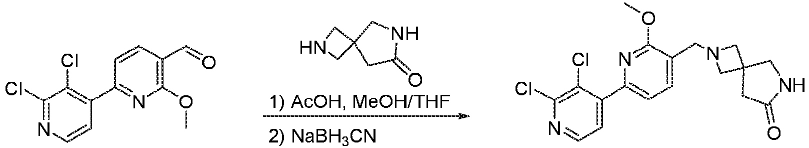

6-(2,3-Dichloro-4-pyridyl)-2-methoxy-pyridine-3-carbaldehyde (250 mg, 0.88 mmol), acetic acid (53 mg, 0.88 mmol), and 2,6-diazaspiro[3.4]octan-7-one (145 mg, 1.15 mmol) were dissolved in MeOH/THF (1:1, 5 mL), then the solution was stirred at 25 °C for 2 hours. Sodium cyanoborohydride (166 mg, 2.65 mmol) was added portion-wise, and the resulting mixture was stirred at 25 °C for 1 hour. Reaction was filtered through celite, and the filtrate was concentrated under reduced pressure. Crude sample was purified by silica gel chromatography (0-10% DCM/MeOH) to afford 2-((2',3'-dichloro-6-methoxy-[2,4'-bipyridin]-5-yl)methyl)-2,6-diazaspiro[3.4]octan-7-one (200 mg, 58 % yield) as a white foam. MS: *m*/*z* found 393, 395 [M+H]⁺

### (b) 6-(2-Chloro-3-(3'-chloro-6-methoxy-5-((7-oxo-2,6-diazaspiro[3.4]octan-2-yl)methyl)-[2,4'-bipyridin]-2'-yl)phenyl)-2-methoxynicotinaldehyde

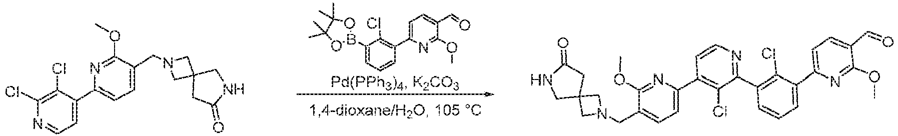

2-((2',3'-Dichloro-6-methoxy-[2,4'-bipyridin]-5-yl)methyl)-2,6-diazaspiro[3.4]octan-7-one (200 mg, 0.51 mmol), tetrakis(triphenylphosphine)palladium(0) (117 mg, 0.10 mmol), potassium carbonate (211 mg, 1.53 mmol), and 6-[2-chloro-3-(4,4,5,5-tetramethyl-1,3,2-dioxaborolan-2-yl)phenyl]-2-methoxy-pyridine-3-carbaldehyde (190 mg, 0.51 mmol) were suspended in 1,4-dioxane/water (4:1, 6 mL), then the solution was heated at 105 °C for 20 minutes. Reaction was cooled to room temperature, diluted with 10 mL water, and extracted with DCM (3 x 10 mL). The combined organics were concentrated under reduced pressure and the crude sample was purified by silica gel chromatography (0-10 % MeOH/DCM) to afford 6-(2-chloro-3-(3'-chloro-6-methoxy-5-((7-oxo-2,6-diazaspiro[3.4]octan-2-yl)methyl)-[2,4'-bipyridin]-2'-yl)phenyl)-2-methoxynicotinaldehyde (158 mg, 51 % yield) as a yellow foam. MS: *m*/*z* found 604, 606 [M+H]⁺.

### (c) 2-((2'-(3-(5-(((1-Acetylpiperidin-4-yl)amino)methyl)-6-methoxypyridin-2-yl)-2-chlorophenyl)-3'-chloro-6-methoxy-[2,4'-bipyridin]-5-yl)methyl)-2,6-diazaspiro[3.4]octan-7-one

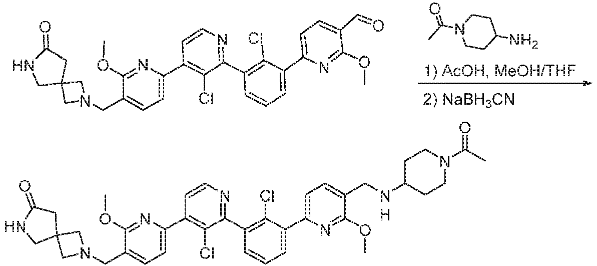

6-(2-Chloro-3-(3'-chloro-6-methoxy-5-((7-oxo-2,6-diazaspiro[3.4]octan-2-yl)methyl)-[2,4'-bipyridin]-2'-yl)phenyl)-2-methoxynicotinaldehyde (20 mg, 0.03 mmol), acetic acid (2 mg, 0.03 mmol), and 1-(4-amino-1-piperidyl)ethanone (7 mg, 0.05 mmol) were dissolved in MeOH/THF (1:1, 1 mL), then the solution was stirred at 25 °C for 2 hours. Sodium cyanoborohydride (4 mg, 0.07 mmol) was added and the resulting mixture was stirred at 25 °C for 1 hour. Reaction was filtered through celite, and the filtrate was concentrated under reduced pressure. Crude sample was purified by reverse phase HPLC to afford 2-((2'-(3-(5-(((1-acetylpiperidin-4-yl)amino)methyl)-6-methoxypyridin-2-yl)-2-chlorophenyl)-3'-chloro-6-methoxy-[2,4'-bipyridin]-5-yl)methyl)-2,6-diazaspiro[3.4]octan-7-one (9 mg, 39 % yield) as a white solid (formic acid salt). LC/MS: *m*/*z* found 730, 732 [M+H]⁺, retention time = 1.99 min (Method C). ¹H NMR (400 MHz, Methanol-*d*₄) δ 8.66 - 8.61 (m, 1H), 8.46 (s, 1H), 7.86 (d, 1H), 7.77 (d, 2H), 7.73 (d, 1H), 7.57 (dd, 1H), 7.50 (d, 1H), 7.42 (d, 1H), 7.35 (d, 1H), 4.63 (d, 1H), 4.20 (s, 2H), 4.07 (d, 3H), 4.02 (d, 4H), 3.81 (s, 2H), 3.60 (d, 2H), 3.53 (s, 4H), 3.20 (t, 2H), 2.70 (t,1H), 2.60 (s, 2H), 2.19 (t, 2H), 2.13 (d, 3H), 1.64 - 1.41 (m, 2H).

### Example 600: 2-((3'-Chloro-2'-(2-chloro-3-(5-((4-hydroxypiperidin-1-yl)methyl)-6-methoxypyridin-2-yl)phenyl)-6-methoxy-[2,4'-bipyridin]-5-yl)methyl)-2,6-diazaspiro[3.4]octan-7-one (681)

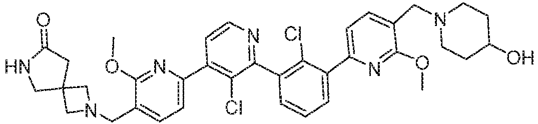

2-((3'-Chloro-2'-(2-chloro-3-(5-((4-hydroxypiperidin-1-yl)methyl)-6-methoxypyridin-2-yl)phenyl)-6-methoxy-[2,4'-bipyridin]-5-yl)methyl)-2,6-diazaspiro[3.4]octan-7-one was prepared in similar manner as described for Example 599, utilizing intermediate 6-(2-chloro-3-(3'-chloro-6-methoxy-5-((7-oxo-2,6-diazaspiro[3.4]octan-2-yl)methyl)-[2,4'-bipyridin]-2'-yl)phenyl)-2-methoxynicotinaldehyde and replacing 1-(4-amino-1-piperidyl)ethanone with piperidin-4-ol in step (c). Product was obtained as a white solid (formic acid salt). LC/MS: *m*/*z* found 689, 691 [M+H]⁺, retention time = 1.97 min (Method C). ¹H NMR (400 MHz, Methanol-*d*₄) δ 8.63 (d, 1H), 8.48 (s, 1H), 7.87 (d, 1H), 7.81 - 7.71 (m, 3H), 7.57 (t, 1H), 7.50 (d, 1H), 7.42 (d, 1H), 7.37 (d, 1H), 4.12 (s, 2H), 4.04 (d, 6H), 3.83 (s, 3H), 3.57 (d, 6H), 2.95 (s, 2H), 2.60 (s, 2H), 1.99 (s, 2H), 1.77 (s, 2H).

### Example 601: 2-((6-(3-(5-(((1-Acetylpiperidin-4-yl)amino)methyl)-3'-chloro-6-methoxy-[2,4'-bipyridin]-2'-yl)-2-chlorophenyl)-2-methoxypyridin-3-yl)methyl)-2,6-diazaspiro[3.4]octan-7-one (682)

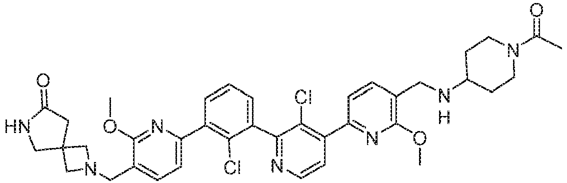

### (a) 1-(4-(((2',3'-Dichloro-6-methoxy-[2,4'-bipyridin]-5-yl)methyl)amino)piperidin-1-yl)ethan-1-one

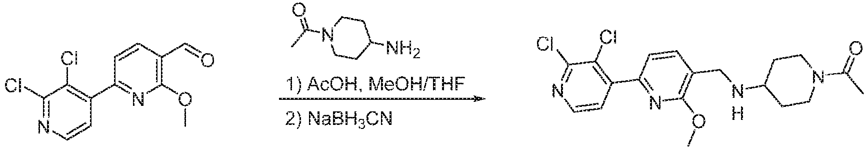

6-(2,3-Dichloro-4-pyridyl)-2-methoxy-pyridine-3-carbaldehyde (177 mg, 0.63 mmol), acetic acid (38 mg, 0.63 mmol), and 1-(4-amino-1-piperidyl)ethanone (125 mg, 0.88 mmol) were dissolved in MeOH/THF (1:1, 5 mL), then the solution was stirred at 25 °C for 2 hours. Sodium cyanoborohydride (79 mg, 1.25 mmol) was added portion-wise, and the resulting mixture was stirred at 25 °C for 1 hour. Reaction was filtered through celite, and the filtrate was concentrated under reduced pressure. The oily residue was dissolved in 10 mL water and extracted with DCM (3 x 5 mL). The combined organics were dried over sodium sulfate, filtered, and concentrated under reduced pressure to afford 1-(4-(((2',3'-dichloro-6-methoxy-[2,4'-bipyridin]-5-yl)methyl)amino)piperidin-1-yl)ethan-1-one (255 mg, crude) as a yellow oil. MS: *m*/*z* found 409, 411 [M+H] ⁺. Material was used for the next step without further purification.

### (b) tert-Butyl (1-acetylpiperidin-4-yl)((2',3'-dichloro-6-methoxy-[2,4'-bipyridin]-5-yl)methyl)carbamate

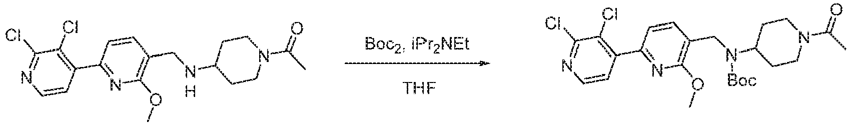

1-(4-(((2',3'-dichloro-6-methoxy-[2,4'-bipyridin]-5-yl)methyl)amino)piperidin-1-yl)ethan-1-one (255 mg, 0.63 mmol, crude), DMAP (15 mg, 0.13 mmol), and N,N-diisopropylethylamine (161 mg, 1.25 mmol) were dissolved in 8 mL THF, then di-tert-butyl dicarbonate (191 mg, 0.88 mmol) was added portion-wise. The resulting mixture was stirred at room temperature for 18 hours. Reaction was diluted with water (15 mL) and the solution was extracted with EtOAc (3 x 10 mL). The combined organics were concentrated under reduced pressure and crude sample was purified by normal phase SiO₂ chromatography (0-100% EtOAc/hexane) to afford tert-butyl (1-acetylpiperidin-4-yl)((2',3'-dichloro-6-methoxy-[2,4'-bipyridin]-5-yl)methyl)carbamate (198 mg, 62 % yield) as a white foam. MS: *m*/*z* found 509, 511 [M+H]⁺.

### (c) tert-Butyl (1-acetylpiperidin-4-yl)((3'-chloro-2'-(2-chloro-3-(5-formyl-6-methoxypyridin-2-yl)phenyl)-6-methoxy-[2,4'-bipyridin]-5-yl)methyl)carbamate

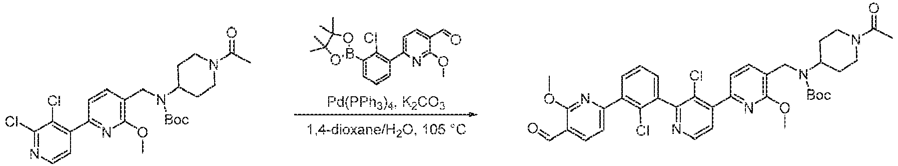

tert-Butyl (1-acetylpiperidin-4-yl)((2',3'-dichloro-6-methoxy-[2,4'-bipyridin]-5-yl)methyl)carbamate (100 mg, 0.20 mmol), tetrakis(triphenylphosphine)palladium(0) (45 mg, 0.04 mmol), potassium carbonate (81 mg, 0.59 mmol), and 6-[2-chloro-3-(4,4,5,5-tetramethyl-1,3,2-dioxaborolan-2-yl)phenyl]-2-methoxy-pyridine-3-carbaldehyde (73 mg, 0.20 mmol) were suspended in 1,4-dioxane/water (4:1, 3 mL), then the solution was heated at 105 °C for 20 minutes. Reaction was cooled to room temperature, diluted with 5 mL water, and extracted with EtOAc (3 x 5 mL). The combined organics were concentrated under reduced pressure and the crude sample was purified by silica gel chromatography (0-10 % MeOH/DCM) to afford tert-butyl (1-acetylpiperidin-4-yl)((3'-chloro-2'-(2-chloro-3-(5-formyl-6-methoxypyridin-2-yl)phenyl)-6-methoxy-[2,4'-bipyridin]-5-yl)methyl)carbamate (63 mg, 45 % yield) as a yellow foam. MS: *m*/*z* found 720, 722 [M+H]⁺.

### (d) tert-Butyl (1-acetylpiperidin-4-yl)((3'-chloro-2'-(2-chloro-3-(6-methoxy-5-((7-oxo-2,6-diazaspiro[3.4]octan-2-yl)methyl)pyridin-2-yl)phenyl)-6-methoxy-[2,4'-bipyridin]-5-yl)methyl)carbamate

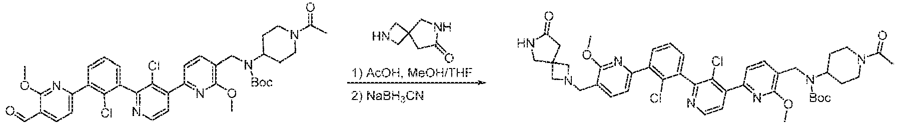

tert-Butyl (1-acetylpiperidin-4-yl)((3'-chloro-2'-(2-chloro-3-(5-formyl-6-methoxypyridin-2-yl)phenyl)-6-methoxy-[2,4'-bipyridin]-5-yl)methyl)carbamate (20 mg, 0.03 mmol), acetic acid (2 mg, 0.03 mmol), and 2,6-diazaspiro[3.4]octan-7-one (5 mg, 0.04 mmol) were dissolved in MeOH/THF (1:1, 1 mL), then the solution was stirred at 25 °C for 2 hours. Sodium cyanoborohydride (4 mg, 0.06 mmol) was added and the resulting mixture was stirred at 25 °C for 1 hour. Reaction was concentrated under reduced pressure and the residue was suspended in 5 mL water, then extracted with DCM (3 x 3 mL). The combined organics were washed with brine, dried over sodium sulfate, filtered, and concentrated under reduce pressure to afford tert-butyl (1-acetylpiperidin-4-yl)((3'-chloro-2'-(2-chloro-3-(6-methoxy-5-((7-oxo-2,6-diazaspiro[3.4]octan-2-yl)methyl)pyridin-2-yl)phenyl)-6-methoxy-[2,4'-bipyridin]-5-yl)methyl)carbamate (23 mg, crude) as a clear oil. MS: *m*/*z* found 830, 832 [M+H] ⁺. Material was used for the next step without further purification.

### (e) 2-((6-(3-(5-(((1-Acetylpiperidin-4-yl)amino)methyl)-3'-chloro-6-methoxy-[2,4'-bipyridin]-2'-yl)-2-chlorophenyl)-2-methoxypyridin-3-yl)methyl)-2,6-diazaspiro [3.4] octan-7-one

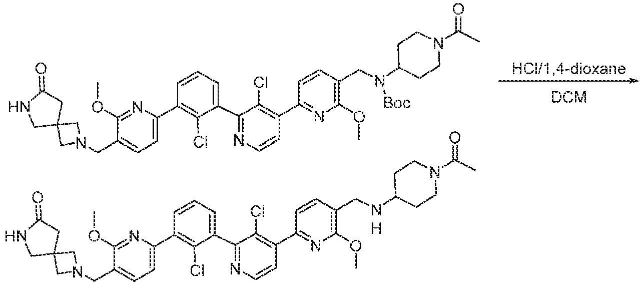

tert-Butyl (1-acetylpiperidin-4-yl)((3'-chloro-2'-(2-chloro-3-(6-methoxy-5-((7-oxo-2,6-diazaspiro[3.4]octan-2-yl)methyl)pyridin-2-yl)phenyl)-6-methoxy-[2,4'-bipyridin]-5-yl)methyl)carbamate (23 mg, 0.03 mmol, crude) was dissolved in 1 mL DCM and 4M HCl solution in 1,4-dioxane (28 µL, 0.11 mmol) was added. Reaction was stirred at room temperature for 60 minutes and solvent was removed under reduced pressure. Crude sample was purified by reverse phase HPLC to afford 2-((6-(3-(5-(((1-acetylpiperidin-4-yl)amino)methyl)-3'-chloro-6-methoxy-[2,4'-bipyridin]-2'-yl)-2-chlorophenyl)-2-methoxypyridin-3-yl)methyl)-2,6-diazaspiro[3.4]octan-7-one (14 mg, 67 % yield) as a white solid (formic acid salt). LC/MS: *m*/*z* found 730, 732 [M+H]⁺, retention time = 2.0 min (Method C). ¹H NMR (400 MHz, Methanol-*d*₄) δ 8.65 (d, 1H), 8.46 (s, 1H), 7.90 (d, 1H), 7.76 (d, 1H), 7.72 (d, 2H), 7.56 (t, 1H), 7.49 - 7.45 (m, 2H), 7.28 (d, 1H), 4.59 (d, 1H), 4.14 (s, 2H), 4.08 (d, 3H), 4.01 (s, 4H), 3.87 (s, 2H), 3.60 (s, 6H), 3.20 (d, 2H), 2.70 (t, 1H), 2.61 (s, 2H), 2.17 (d, 2H), 2.12 (s, 3H), 1.48 (dd, 2H).

### Example 602: 1-(4-(((6-(3-(4-(4-(((1-Acetylpiperidin-4-yl)amino)methyl)-3-methoxyphenyl)-3-chloropyridin-2-yl)-2-chlorophenyl)-2-methoxypyridin-3-yl)methyl)amino)piperidin-1-yl)ethan-1-one (621)

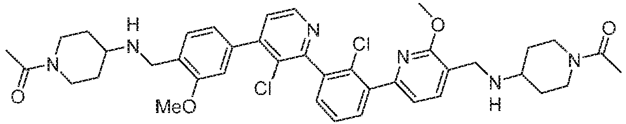

### (a) 4-(2,3-Dichloropyridin-4-yl)-2-methoxybenzaldehyde

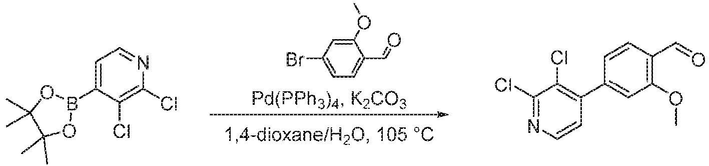

4-bromo-2-methoxy-benzaldehyde (1.10 g, 5.11 mmol), tetrakis(triphenylphosphine)palladium(0) (0.30 g, 0.26 mmol), potassium carbonate (1.06 g, 7.67 mmol), and 2,3-dichloro-4-(4,4,5,5-tetramethyl-1,3,2-dioxaborolan-2-yl)pyridine (0.70 g, 2.56 mmol) were suspended in 1,4-dioxane/water (4:1, 25 mL), then the solution was heated at 105 °C for 20 minutes. Reaction was cooled to room temperature, diluted with 40 mL water, and extracted with EtOAc (3 x 30 mL). The combined organics were concentrated under reduced pressure, and crude sample was purified by normal phase SiO₂ chromatography (0-30% EtOAc/hexane) to afford 4-(2,3-dichloropyridin-4-yl)-2-methoxybenzaldehyde (0.35 g, 49 % yield) as a white solid. MS: *m*/*z* found 282, 284 [M+H]⁺.

### (b) 6-(2-Chloro-3-(3-chloro-4-(4-formyl-3-methoxyphenyl)pyridin-2-yl)phenyl)-2-methoxynicotinaldehyde

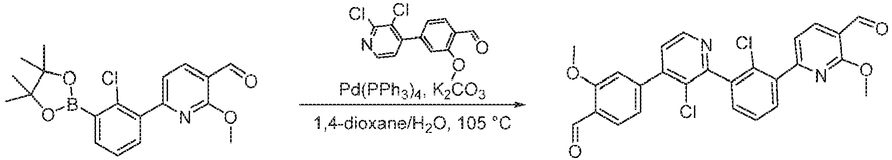

4-(2,3-Dichloropyridin-4-yl)-2-methoxybenzaldehyde (150 mg, 0.53 mmol), tetrakis(triphenylphosphine)palladium(0) (123 mg, 0.11 mmol), potassium carbonate (220 mg, 1.60 mmol), and 6-[2-chloro-3-(4,4,5,5-tetramethyl-1,3,2-dioxaborolan-2-yl)phenyl]-2-methoxy-pyridine-3-carbaldehyde (199 mg, 0.53 mmol) were suspended in 1,4-dioxane/water (4:1, 5 mL), then the solution was heated at 105 °C for 20 minutes. Reaction was cooled to room temperature, diluted with 10 mL water, and extracted with EtOAc (3 x 5 mL). The combined organics were concentrated under reduced pressure and the crude sample was purified by silica gel chromatography (10-60 % EtOAc/hexane) to afford 6-(2-chloro-3-(3-chloro-4-(4-formyl-3-methoxyphenyl)pyridin-2-yl)phenyl)-2-methoxynicotinaldehyde (262 mg, 100 % yield) as a yellow foam. MS: *m*/*z* found 493, 495 [M+H]⁺.

### (c) 1-(4-(((6-(3-(4-(4-(((1-Acetylpiperidin-4-yl)amino)methyl)-3-methoxyphenyl)-3-chloropyridin-2-yl)-2-chlorophenyl)-2-methoxypyridin-3-yl)methyl)amino)piperidin-1-yl)ethan-1-one

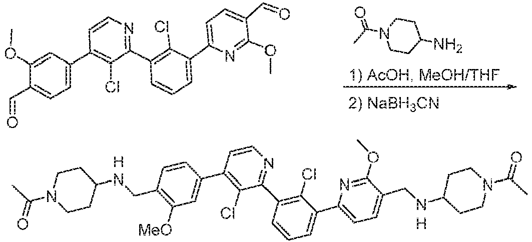

6-(2-Chloro-3-(3-chloro-4-(4-formyl-3-methoxyphenyl)pyridin-2-yl)phenyl)-2-methoxynicotinaldehyde (20 mg, 0.04 mmol), acetic acid (5 mg, 0.08 mmol), and 1-(4-amino-1-piperidyl)ethanone (17 mg, 0.12 mmol) were dissolved in MeOH/THF (1:1, 1 mL), then the solution was stirred at 25 °C for 2 hours. Sodium cyanoborohydride (8 mg, 0.12 mmol) was added and the resulting mixture was stirred at 25 °C for 1 hour. Reaction was filtered through celite, and the filtrate was concentrated under reduced pressure. Crude sample was purified by reverse phase HPLC to afford 1-(4-(((6-(3-(4-(4-(((1-acetylpiperidin-4-yl)amino)methyl)-3-methoxyphenyl)-3-chloropyridin-2-yl)-2-chlorophenyl)-2-methoxypyridin-3-yl)methyl)amino)piperidin-1-yl)ethan-1-one (19 mg, 64 % yield) as a white solid (formic acid salt). LC/MS: *m*/*z* found 745, 747 [M+H]⁺, retention time = 2.15 min (Method C). ¹H NMR (400 MHz, Methanol-*d*₄) δ 8.62 (d, 1H), 8.48 (s, 2H), 7.86 - 7.79 (m, 1H), 7.72 (d, 1H), 7.60 - 7.51 (m, 3H), 7.48 (d, 1H), 7.32 (d, 1H), 7.25 (s, 1H), 7.19 (d, 1H), 4.61 (t, 2H), 4.24 (s, 2H), 4.12 (s, 2H), 4.05 (d, 5H), 3.97 (s, 3H), 3.19 (t, 4H), 2.69 (t, 2H), 2.20 (t, 4H), 2.12 (d, 6H), 1.69 - 1.30 (m, 4H).

### Example 603: 2-(4-(3-Chloro-2-(2-chloro-3-(6-methoxy-5-((7-oxo-2,6-diazaspiro [3.4] octan-2-yl)methyl)pyridin-2-yl)phenyl)pyridin-4-yl)-2-methoxybenzyl)-2,6-diazaspiro[3.4]octan-7-one (622)

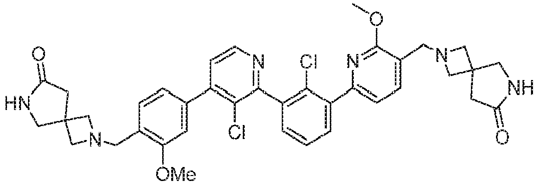

2-(4-(3-Chloro-2-(2-chloro-3-(6-methoxy-5-((7-oxo-2,6-diazaspiro[3.4]octan-2-yl)methyl)pyridin-2-yl)phenyl)pyridin-4-yl)-2-methoxybenzyl)-2,6-diazaspiro[3.4]octan-7-one was prepared in similar manner as described for Example 602, utilizing intermediate 6-(2-chloro-3-(3-chloro-4-(4-formyl-3-methoxyphenyl)pyridin-2-yl)phenyl)-2-methoxynicotinaldehyde and replacing 1-(4-amino-1-piperidyl)ethanone with 2,6-diazaspiro[3.4]octan-7-one in step (c). Product was obtained as a white solid (formic acid salt). LC/MS: *m*/*z* found 713, 715 [M+H]⁺, retention time = 2.05 min (Method C). ¹H NMR (400 MHz, Methanol-*d*₄) δ 8.62 - 8.58 (m, 1H), 8.46 (s, 1H), 7.72 (t, 2H), 7.55 (dd, 2H), 7.46 (d, 2H), 7.28 (d, 1H), 7.20 (s, 1H), 7.16 (d, 1H), 4.11 (s, 2H), 4.01 (d, 3H), 3.93 (s, 3H), 3.89 (s, 2H), 3.84 (s, 4H), 3.65 - 3.58 (m, 8H), 2.64 (s, 2H), 2.61 (s, 2H).

### Example 604: N-(4-(3-Chloro-2-(2-chloro-3-(6-methoxy-5-(((tetrahydro-2H-pyran-4-yl)amino)methyl)pyridin-2-yl)phenyl)pyridin-4-yl)-2-methoxybenzyl)tetrahydro-2H-pyran-4-amine (623)

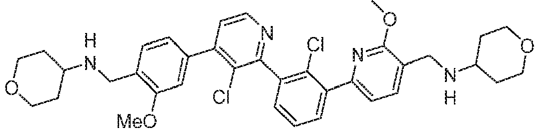

N-(4-(3-Chloro-2-(2-chloro-3-(6-methoxy-5-(((tetrahydro-2H-pyran-4-yl)amino)methyl)pyridin-2-yl)phenyl)pyridin-4-yl)-2-methoxybenzyl)tetrahydro-2H-pyran-4-amine was prepared in similar manner as described for Example 602, utilizing intermediate 6-(2-chloro-3-(3-chloro-4-(4-formyl-3-methoxyphenyl)pyridin-2-yl)phenyl)-2-methoxynicotinaldehyde and replacing 1-(4-amino-1-piperidyl)ethanone with tetrahydropyran-4-amine in step (c). Product was obtained as a white solid (formic acid salt). LC/MS: *m*/*z* found 663, 665 [M+H]⁺, retention time = 2.2 min (Method C). ¹H NMR (400 MHz, Methanol-*d*₄) δ 8.61 (d, 1H), 8.53 (s, 1H), 7.79 (d, 1H), 7.72 (d, 1H), 7.56 (dd, 2H), 7.49 (dd, 2H), 7.29 (d, 1H), 7.22 (s, 1H), 7.17 (d, 1H), 4.13 (s, 2H), 4.03 (d, 3H), 4.00 (s, 6H), 3.96 (d, 3H), 3.43 (t, 4H), 3.16 (s, 1H), 2.99 (s, 1H), 2.01 (t, 4H), 1.69 - 1.48 (m, 4H).

### Example 605: (S)-5-(((4-(3-Chloro-2-(2-chloro-3-(6-methoxy-5-(((((S)-5-oxopyrrolidin-2-yl)methyl)-amino)methyl)pyridin-2-yl)phenyl)pyridin-4-yl)-2-methoxybenzyl)-amino)methyl)pyrrolidin-2-one (624)

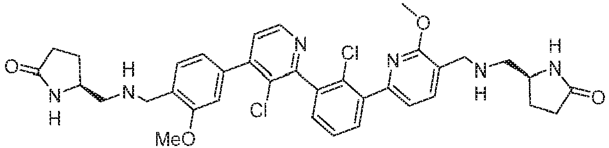

(S)-5-(((4-(3-Chloro-2-(2-chloro-3-(6-methoxy-5-(((((S)-5-oxopyrrolidin-2-yl)methyl)-amino)methyl)pyridin-2-yl)phenyl)pyridin-4-yl)-2-methoxybenzyl)-amino)methyl)pyrrolidin-2-one was prepared in similar manner as described for Example 602, utilizing intermediate 6-(2-chloro-3-(3-chloro-4-(4-formyl-3-methoxyphenyl)pyridin-2-yl)phenyl)-2-methoxynicotinaldehyde and replacing 1-(4-amino-1-piperidyl)ethanone with (5S)-5-(aminomethyl)-pyrrolidin-2-in step (c). Product was obtained as a white solid (formic acid salt). LC/MS: *m*/*z* found 689, 691 [M+H]⁺, retention time = 2.07 min (Method C). ¹H NMR (400 MHz, Methanol-*d*₄) δ 8.62 (d, 1H), 8.36 (s, 2H), 7.81 (d, 1H), 7.73 (d, 1H), 7.60 - 7.46 (m, 4H), 7.31 (d, 1H), 7.24 (s, 1H), 7.19 (d, 1H), 4.27 - 4.14 (m, 2H), 4.06 (dd, 5H), 3.97 (s, 5H), 3.06 (t, 2H), 2.95 (t, 2H), 2.44 - 2.28 (m, 6H), 1.86 (s, 2H).

### Example 606: 1-(4-(((6-(3-(6-(4-(((1-Acetylpiperidin-4-yl)amino)methyl)-3-methoxyphenyl)-5-methylpyrimidin-4-yl)-2-chlorophenyl)-2-methoxypyridin-3-yl)methyl)amino)piperidin-1-yl)ethan-1-one (522)

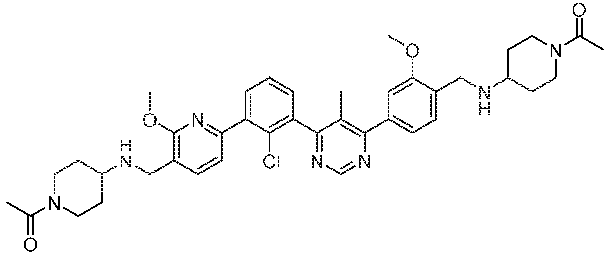

### (a) 4-(6-Chloro-5-methylpyrimidin-4-yl)-2-methoxybenzaldehyde

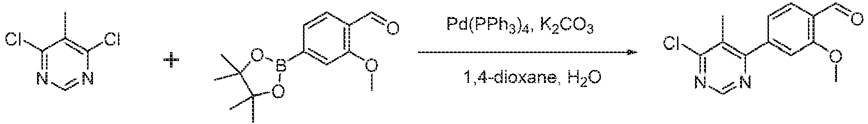

A mixture of 4,6-dichloro-5-methylpyrimidine (0.51 g, 3.10 mmol), 2-methoxy-4-(4,4,5,5-tetramethyl-1,3,2-dioxaborolan-2-yl)benzaldehyde (Example 77, step (a)) (0.81 g, 3.10 mmol) and potassium carbonate (1.29 g, 9.30 mmol) in 1,4-dioxane (18 mL) and water (4.5 mL) was purged with N₂ for 5 minutes. Tetrakis(triphenylphosphine)palladium(0) (110 mg, 0.09 mmol) was then added and the mixture stirred at 95°C in a sealed vial for 2 hours. The cooled reaction mixture was partitioned between ethyl acetate (75 mL) and water (75 mL). The organic phase was separated, washed with saturated brine (2 x 50 mL), dried over magnesium sulfate, filtered and concentrated under reduced pressure to give a brown solid (1.13 g,). The crude product was purified by normal phase SiO₂ chromatography (0 to 100% ethyl acetate in hexane) to give 4-(6-chloro-5-methylpyrimidin-4-yl)-2-methoxybenzaldehyde (0.44 g, 54.0% yield) as a cream solid. MS: *m*/*z* found 263 [M+H]⁺, retention time = 0.87 min (Method B). ¹H NMR (400 MHz, Chloroform-d) δ 10.53 (d, 1H), 8.90 (dd, 1H), 7.94 (dd, 1H), 7.19 (d, 1H), 7.16 (m, 1H), 4.00 (s, 3H), 2.43 (s, 3H).

### (b) 6-(2-Chloro-3-(6-(4-formyl-3-methoxyphenyl)-5-methylpyrimidin-4-yl)phenyl)-2-methoxynicotinaldehyde

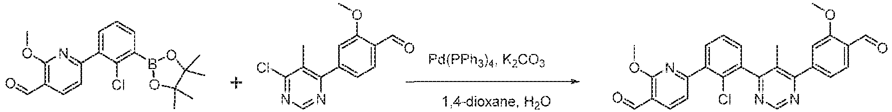

A mixture of 6-(2-chloro-3-(4,4,5,5-tetramethyl-1,3,2-dioxaborolan-2-yl)phenyl)-2-methoxynicotinaldehyde (181 mg, 0.48 mmol), 4-(6-chloro-5-methylpyrimidin-4-yl)-2-methoxybenzaldehyde (127 mg, 0.48 mmol) and potassium carbonate (201 mg, 1.45 mmol) in 1,4-dioxane (4 mL) and water (1 mL) was purged with N₂ for 5 minutes. Tetrakis(triphenylphosphine)palladium(0) (17 mg, 0.01 mmol) was added and the mixture stirred at 95°C in a sealed vial for 2 hours. The cooled reaction mixture was then partitioned between ethyl acetate (40 mL) and water (40 mL). The organic phase was separated, washed with saturated brine (2 x 40 mL), dried over magnesium sulfate, filtered and concentrated under reduced pressure to give a yellow syrup (0.371 g). The crude product was purified by normal phase SiO₂ chromatography (0 to 100% ethyl acetate in hexanes) to give 6-(2-chloro-3-(6-(4-formyl-3-methoxyphenyl)-5-methylpyrimidin-4-yl)phenyl)-2-methoxynicotinaldehyde (189.5 mg, 82.5% yield) as a yellow foam. MS: *m*/*z* found 474 [M+H]⁺, retention time = 1.02 min (Method B). ¹H NMR (400 MHz, Chloroform-*d*) δ 10.54 (d, 1H), 10.43 (d, 1H), 9.24 (d, 1H), 8.20 (dd, 1H), 7.95 (d, 1H), 7.75 (dd, 1H), 7.55 (dd, 1H), 7.45 - 7.42 (m, 1H), 7.40 (dd, 1H), 7.30 (d, 1H), 7.27 - 7.23 (m, 1H), 4.13 (s, 3H), 4.02 (s, 3H), 2.24 (s, 3H).

### (c) 1-(4-(((6-(3-(6-(4-(((1-Acetylpiperidin-4-yl)amino)methyl)-3-methoxyphenyl)-5-methylpyrimidin-4-yl)-2-chlorophenyl)-2-methoxypyridin-3-yl)methyl)amino)piperidin-1-yl)ethan-1-one

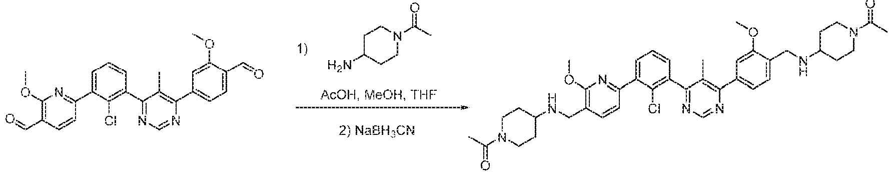

A mixture of 6-(2-chloro-3-(6-(4-formyl-3-methoxyphenyl)-5-methylpyrimidin-4-yl)phenyl)-2-methoxynicotinaldehyde (50 mg, 0.11 mmol), 1-(4-aminopiperidin-1-yl)ethan-1-one (45 mg, 0.32 mmol) and acetic acid (13 mg, 0.21 mmol) in dry tetrahydrofuran (1.5 mL) and dry methanol (1.5 mL) was stirred at room temperature under N₂ for 2 h 20 min. Sodium cyanoborohydride (20 mg, 0.32 mmol) was then added and the mixture stirred for 2 hours. The reaction was quenched by the addition of saturated aqueous sodium hydrogen carbonate solution (5 mL) and the cloudy suspension stirred vigorously for 15 minutes. The mixture was then partitioned between ethyl acetate (60 mL) and 1/2 saturated brine (30 mL), the organic phase separated, washed with saturated brine (30 mL), dried over sodium sulfate, filtered and concentrated under reduced pressure to give a glass (62 mg). The crude product was purified using reverse phase HPLC to give 1-(4-(((6-(3-(6-(4-(((1-acetylpiperidin-4-yl)amino)methyl)-3-methoxyphenyl)-5-methylpyrimidin-4-yl)-2-chlorophenyl)-2-methoxypyridin-3-yl)methyl)amino)piperidin-1-yl)ethan-1-one (24.7 mg, 32% yield) as a white solid (formic acid salt). MS: *m*/*z* found 726 [M+H]⁺, retention time 0.57 min (Method B). ¹H NMR (400 MHz, Methanol-*d*₄) δ 9.14 (s, 1H), 7.83 (d, 1H), 7.76 (dd, 1H), 7.61 (dd, 1H), 7.55 (d, 1H), 7.50 (dd, 1H), 7.36 (d, 1H), 7.32 (d, 1H), 7.28 (dd, 1H), 4.68 - 4.52 (m, 2H), 4.22 (s, 2H), 4.07 (s, 2H), 4.05 (s, 3H), 4.04 - 4.01 (m, 2H), 3.99 (s, 3H), 3.29 - 3.04 (m, 4H), 2.77 - 2.64 (m, 2H), 2.23 (s, 3H), 2.14 - 2.09 (m, 4H), 2.13 (s, 3H), 2.12 (s, 3H), 1.65 - 1.34 (m, 4H).

### Example 607: (S)-5-((((6-(2-Chloro-3-(6-(3-methoxy-4-(((((S)-5-oxopyrrolidin-2-yl)methyl)amino)methyl)phenyl)-5-methylpyrimidin-4-yl)phenyl)-2-methoxypyridin-3-yl)methyl)amino)methyl)pyrrolidin-2-one (523)

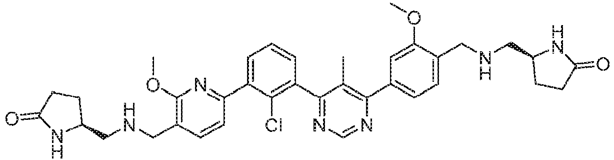

### (a) (S)-5-((((6-(2-chloro-3-(6-(3-methoxy-4-(((((S)-5-oxopyrrolidin-2-yl)methyl)amino)methyl)phenyl)-5-methylpyrimidin-4-yl)phenyl)-2-methoxypyridin-3-yl)methyl)amino)methyl)pyrrolidin-2-one

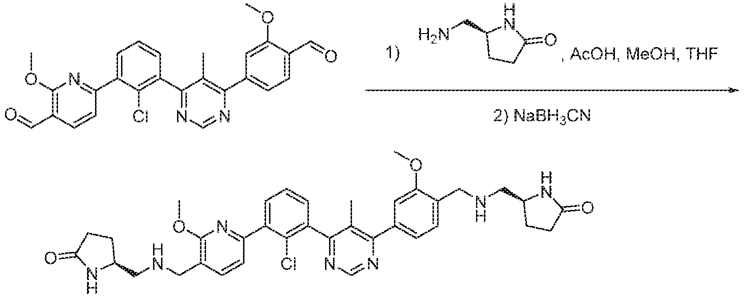

(S)-5-((((6-(2-Chloro-3-(6-(3-methoxy-4-(((((S)-5-oxopyrrolidin-2-yl)methyl)amino)methyl)phenyl)-5-methylpyrimidin-4-yl)phenyl)-2-methoxypyridin-3-yl)methyl)amino)methyl)pyrrolidin-2-one was prepared in a similar manner as described for Example 606, replacing 1-(4-aminopiperidin-1-yl)ethan-1-one with (S)-5-(aminomethyl)pyrrolidin-2-one in step (c). MS: *m*/*z* found 670 [M+H]⁺, retention time = 2.14 min (Method A). ¹H NMR (400 MHz, Methanol-*d*₄) δ 9.13 (q, 1H), 8.43 (s, 1H), 7.81 (d, 1H), 7.76 (dd, 1H), 7.61 (dd, 1H), 7.53 (d, 1H), 7.50 (dd, 1H), 7.35 (d, 1H), 7.31 (d, 1H), 7.28 (dd, 1.6 Hz, 1H), 4.22 - 4.12 (m, 2H), 4.04 (s, 3H), 4.00 (d, 2H), 3.99 (s, 3H), 3.98 - 3.87 (m, 2H), 3.07 - 2.94 (m, 2H), 2.94 - 2.81 (m, 2H), 2.47 - 2.26 (m, 6H), 2.23 (d, 3H), 1.93 - 1.79 (m, 2H).

### Example 608: 2-((6-(2-Chloro-3-(6-(3-methoxy-4-((7-oxo-2,6-diazaspiro[3.4]octan-2-yl)methyl)phenyl)-5-methylpyrimidin-4-yl)phenyl)-2-methoxypyridin-3-yl)methyl)-2,6-diazaspiro[3.4]octan-7-one (535)

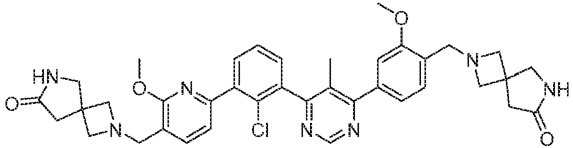

### (a) 2-((6-(2-chloro-3-(6-(3-methoxy-4-((7-oxo-2,6-diazaspiro[3.4]octan-2-yl)methyl)phenyl)-5-methylpyrimidin-4-yl)phenyl)-2-methoxypyridin-3-yl)methyl)-2,6-diazaspiro [3.4] octan-7-one

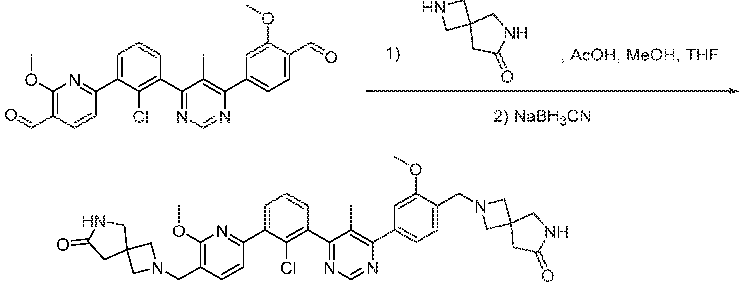

2-((6-(2-Chloro-3-(6-(3-methoxy-4-((7-oxo-2,6-diazaspiro[3.4]octan-2-yl)methyl)phenyl)-5-methylpyrimidin-4-yl)phenyl)-2-methoxypyridin-3-yl)methyl)-2,6-diazaspiro[3.4]octan-7-one was prepared in a similar manner as described for Example 606, replacing 1-(4-aminopiperidin-1-yl)ethan-1-one with 2,6-diazaspiro[3.4]octan-7-one in step (c). MS: *m*/*z* found 694 [M+H]⁺, retention time = 2.11 min (Method A). ¹H NMR (400 MHz, Methanol-*d*₄) δ 9.13 (s, 1H), 8.44 (brs, 1H), 7.80 - 7.73 (m, 2H), 7.60 (dd, 1H), 7.54 - 7.46 (m, 2H), 7.34 (d, 1H), 7.30 (d, 1H), 7.28 (dd, 1H), 4.24 (s, 2H), 4.02 (s, 3H), 3.97 (s, 3H), 3.97 (s, 6H), 3.73 (s, 4H), 3.65 (s, 2H), 3.62 (s, 2H), 2.68 (s, 2H), 2.64 (s, 2H), 2.22 (s, 3H).

### Example 609: 1-(6-((6-(3-(6-(4-((6-Acetyl-2,6-diazaspiro[3.3]heptan-2-yl)methyl)-3-methoxyphenyl)-5-methylpyrimidin-4-yl)-2-chlorophenyl)-2-methoxypyridin-3-yl)methyl)-2,6-diazaspiro[3.3]heptan-2-yl)ethan-1-one (538)

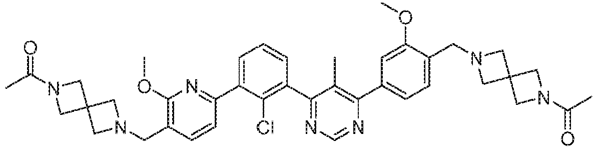

### (a) 1-(6-((6-(3-(6-(4-((6-Acetyl-2,6-diazaspiro[3.3]heptan-2-yl)methyl)-3-methoxyphenyl)-5-methylpyrimidin-4-yl)-2-chlorophenyl)-2-methoxypyridin-3-yl)methyl)-2,6-diazaspiro[3.3]heptan-2-yl)ethan-1-one

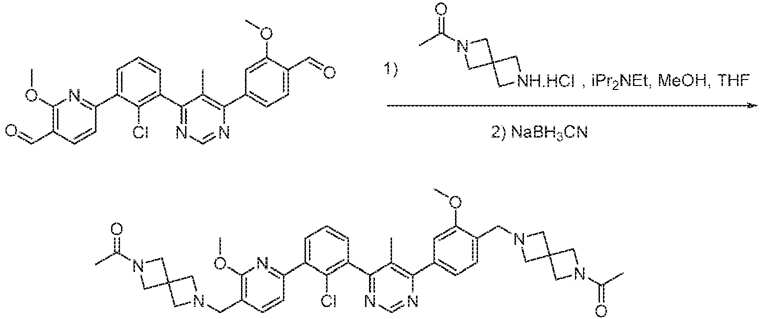

A mixture of 6-(2-chloro-3-(6-(4-formyl-3-methoxyphenyl)-5-methylpyrimidin-4-yl)phenyl)-2-methoxynicotinaldehyde (Example 606, step (b)) (41 mg, 0.09 mmol), 1-(2,6-diazaspiro[3.3]heptan-2-yl)ethan-1-one hydrochloride (46 mg, 0.26 mmol) and diisopropyl ethylamine (37 mg, 0.28 mmol) in methanol (1.5 mL) and tetrahydrofuran (1.5 mL) was stirred at room temperature under N₂ for 21 hours. Sodium cyanoborohydride (22 mg, 0.35 mmol) was then added and the reaction mixture stirred at room temperature for 2 3/4 hours. The reaction was then quenched by the addition of saturated aqueous sodium hydrogen carbonate (5 mL) and the resulting suspension stirred vigorously for 15 min. The mixture was then partitioned between ethyl acetate (60 mL) and 1/2 saturated brine (30 mL), the organic phase separated, washed with saturated brine (20 mL), dried over sodium sulfate and concentrated under reduced pressure to give a colorless glass (50 mg). The crude product was purified using reverse phase HPLC to give 1-(6-((6-(3-(6-(4-((6-acetyl-2,6-diazaspiro[3.3]heptan-2-yl)methyl)-3-methoxyphenyl)-5-methylpyrimidin-4-yl)-2-chlorophenyl)-2-methoxypyridin-3-yl)methyl)-2,6-diazaspiro[3.3]heptan-2-yl)ethan-1-one (7.0 mg, 11.2%) as a white solid (formic acid salt). MS: *m*/*z* found 722 [M+H]⁺, retention time = 2.23 min (Method A). ¹H NMR (400 MHz, Methanol-*d*₄) δ 9.13 (d, 1H), 8.45 (brs, 1H), 7.79 - 7.73 (m, 2H), 7.61 (dd, 1H), 7.53 - 7.47 (m, 2H), 7.35 (d, 1H), 7.31 (d, 1H), 7.28 (dd, 1H), 4.36 (s, 2H), 4.34 (s, 2H), 4.22 (s, 2H), 4.12 (s, 2H), 4.09 (s, 2H), 4.08 - 4.05 (m, 4H), 4.02 (s, 3H), 3.98 (s, 3H), 3.96 (s, 2H), 3.88 - 3.80 (m, 4H), 2.22 (d, 3H), 1.85 (s, 6H).

### Example 610: 1-(4-(((6-(3-(6-(4-(((1-Acetylpiperidin-4-yl)amino)methyl)-3-methoxyphenyl)-5-chloropyrimidin-4-yl)-2-chlorophenyl)-2-methoxypyridin-3-yl)methyl)amino)piperidin-1-yl)ethan-1-one (540)

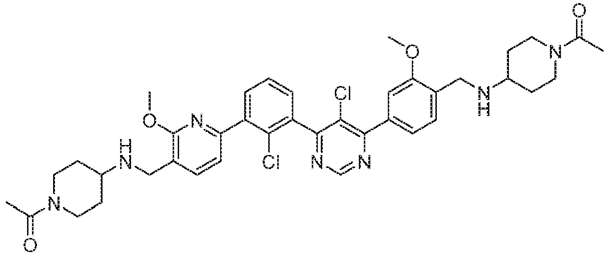

### (a) 4-(5,6-Dichloropyrimidin-4-yl)-2-methoxybenzaldehyde

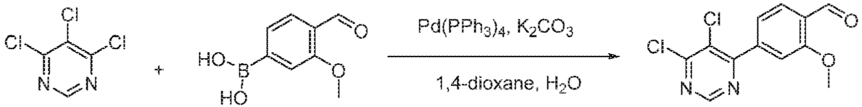

A mixture of (4-formyl-3-methoxyphenyl)boronic acid (0.84 g, 4.65 mmol), 4,5,6-trichloropyrimidine (0.85 g, 4.65 mmol) and potassium carbonate (1.93 g, 13.95 mmol) in 1,4-dioxane (16 mL) and H₂O (4 mL) was purged with N₂ for 5 minutes. Tetrakis(triphenylphosphine)palladium(0) (160 mg, 0.14 mmol) was added and the mixture stirred at 95°C in a sealed vial for 3 hours. The cooled reaction mixture was then partitioned between ethyl acetate (75 mL) and water (75 mL), the organic phase separated, washed with saturated brine (2 x 40 mL), dried over magnesium sulfate, filtered and concentrated under reduced pressure to give a brown solid (1.28 g). The crude product was purified by normal phase SiO₂ chromatography (0 to 100% ethyl acetate in hexane to give 4-(5,6-dichloropyrimidin-4-yl)-2-methoxybenzaldehyde (0.47 g, 36% yield) as a white solid. ¹H NMR (400 MHz, Chloroform-d) δ 10.54 (d, 1H), 8.94 (s, 1H), 7.95 (d, 1H), 7.46 (m, 1H), 7.39 (d, 1H), 4.01 (s, 3H).

### (b) 6-(2-Chloro-3-(5-chloro-6-(4-formyl-3-methoxyphenyl)pyrimidin-4-yl)phenyl)-2-methoxynicotinaldehyde

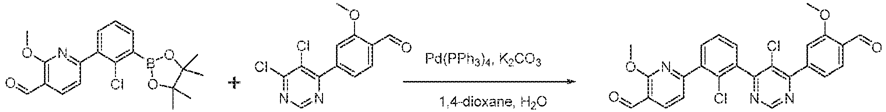

A mixture of 4-(5,6-dichloropyrimidin-4-yl)-2-methoxybenzaldehyde (0.44 g, 1.55 mmol), 6-(2-chloro-3-(4,4,5,5-tetramethyl-1,3,2-dioxaborolan-2-yl)phenyl)-2-methoxynicotinaldehyde (0.58 g, 1.55 mmol) and potassium carbonate (0.64 g, 4.66 mmol) in 1,4-dioxane (12 mL) and H₂O (3 mL) was purged with N₂ for 5 minutes. Tetrakis(triphenylphosphine)palladium(0) (54 mg, 0.05 mmol) was then added and the mixture stirred at 95°C in a sealed vial for 16 hours. The cooled reaction mixture was partitioned between ethyl acetate (75 mL) and H₂O (70 mL), the organic phase separated, washed with saturated brine (2 x 40 mL), dried over magnesium sulfate, filtered and concentrated under reduced pressure to give an orange syrup (1.08 g). The crude product was purified by normal phase SiO₂ chromatography (0-70% ethyl acetate in hexane) to give 6-(2-chloro-3-(5-chloro-6-(4-formyl-3-methoxyphenyl)pyrimidin-4-yl)phenyl)-2-methoxynicotinaldehyde (0.52g, 67.7%) as an off-white solid. MS: *m*/*z* found 494 [M+H]⁺, retention time = 1.07 min (Method B). ¹H NMR (400 MHz, Chloroform-d) δ 10.55 (s, 1H), 10.43 (s, 1H), 9.27 (s, 1H), 8.20 (d, 1H), 7.97 (d, 1H), 7.78 (dd, 1H), 7.60 - 7.52 (m, 2H), 7.51 - 7.45 (m, 2H), 7.45 - 7.39 (m, 1H), 4.13 (s, 3H), 4.03 (s, 3H).

### (c) 1-(4-(((6-(3-(6-(4-(((1-Acetylpiperidin-4-yl)amino)methyl)-3-methoxyphenyl)-5-chloropyrimidin-4-yl)-2-chlorophenyl)-2-methoxypyridin-3-yl)methyl)amino)piperidin-1-yl)ethan-1-one

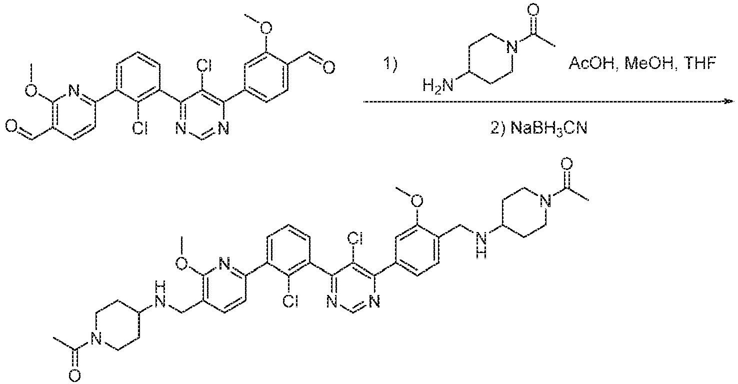

A mixture of 6-(2-chloro-3-(5-chloro-6-(4-formyl-3-methoxyphenyl)pyrimidin-4-yl)phenyl)-2-methoxynicotinaldehyde (50 mg, 0.10 mmol), 1-(4-aminopiperidin-1-yl)ethan-1-one (43 mg, 0.30 mmol) and acetic acid (12 mg, 0.20 mmol) in dry tetrahydrofuran (1.5 mL) and dry methanol (1.5 mL) was stirred at room temperature under N₂ for 14 hours. Sodium cyanoborohydride (25 mg, 0.40 mmol) was then added and the mixture stirred for 2 hours. The reaction was quenched by the addition of saturated aqueous sodium hydrogen carbonate solution (5 mL) and the cloudy suspension stirred vigorously for 15 minutes. The mixture was then partitioned between ethyl acetate (60 mL) and 1/2 saturated brine (30 mL), the organic phase separated, washed with saturated brine (20 mL), dried over sodium sulfate, filtered and concentrated under reduced pressure to give a colorless syrup (83.5 mg). The crude product was purified using reverse phase HPLC to give 1-(4-(((6-(3-(6-(4-(((1-acetylpiperidin-4-yl)amino)methyl)-3-methoxyphenyl)-5-chloropyrimidin-4-yl)-2-chlorophenyl)-2-methoxypyridin-3-yl)methyl)amino)piperidin-1-yl)ethan-1-one (51 mg, 68%) as a white solid (formic acid salt). MS: *m*/*z* found 746 [M+H]⁺, retention time = 2.57 min (Method A). ¹H NMR (400 MHz, Methanol-*d*₄) δ 9.23 (s, 1H), 8.56 (brs), 7.83 (d, 1H), 7.78 (dd, 1H), 7.61 (dd, 1H), 7.58 - 7.50 (m, 4H), 7.32 (d, 1H), 4.67 - 4.51 (m, 2H), 4.20 (s, 2H), 4.07 (s, 2H), 4.06 (s, 4H), 4.04 - 4.01 (m, 2H), 4.00 (s, 3H), 3.29 - 3.04 (m, 4H), 2.76 - 2.64 (m, 2H), 2.24 - 2.07 (m, 4H), 2.13 (s, 3H), 2.12 (s, 3H), 1.63 - 1.33 (m, 4H).

### Example 611: (S)-5-((((6-(2-chloro-3-(5-chloro-6-(3-methoxy-4-(((((S)-5-oxopyrrolidin-2-yl)methyl)amino)methyl)phenyl)pyrimidin-4-yl)phenyl)-2-methoxypyridin-3-yl)methyl)amino)methyl)pyrrolidin-2-one (543)

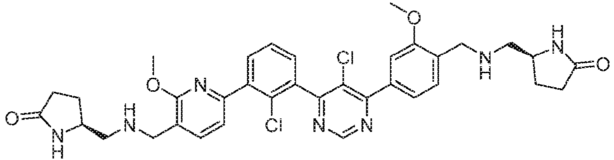

### (a) (S)-5-((((6-(2-Chloro-3-(5-chloro-6-(3-methoxy-4-(((((S)-5-oxopyrrolidin-2-yl)methyl)amino)methyl)phenyl)pyrimidin-4-yl)phenyl)-2-methoxypyridin-3-yl)methyl)amino)methyl)pyrrolidin-2-one

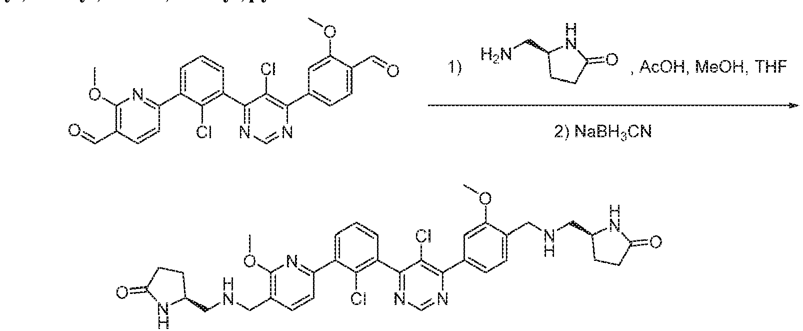

(S)-5-((((6-(2-Chloro-3-(5-chloro-6-(3-methoxy-4-(((((S)-5-oxopyrrolidin-2-yl)methyl)amino)methyl)phenyl)pyrimidin-4-yl)phenyl)-2-methoxypyridin-3-yl)methyl)amino)methyl)pyrrolidin-2-one was prepared in a similar manner as described for Example 610, replacing 1-(4-aminopiperidin-1-yl)ethan-1-one with (S)-5-(aminomethyl)pyrrolidin-2-one in step (c). MS: *m*/*z* found 690 [M+H]⁺, retention time = 2.38 min (Method A). ¹H NMR (400 MHz, Methanol-*d*₄) δ 9.23 (s, 1H), 8.39 (brs), 7.83 (d, 1H), 7.78 (dd, 1H), 7.61 (dd, 1H), 7.58 - 7.51 (m, 4H), 7.33 (d, 1H), 4.27 - 4.18 (m, 2H), 4.13 - 4.05 (m, 2H), 4.06 (s, 3H), 4.00 (s, 3H), 4.00 - 3.91 (m, 2H), 3.12 - 3.02 (m, 2H), 3.02 - 2.91 (m, 2H), 2.46 - 2.27 (m, 6H), 1.95 - 1.80 (m, 2H).

### Example 612: 2-((6-(2-chloro-3-(5-chloro-6-(3-methoxy-4-((7-oxo-2,6-diazaspiro[3.4]octan-2-yl)methyl)phenyl)pyrimidin-4-yl)phenyl)-2-methoxypyridin-3-yl)methyl)-2,6-diazaspiro[3.4]octan-7-one (554)

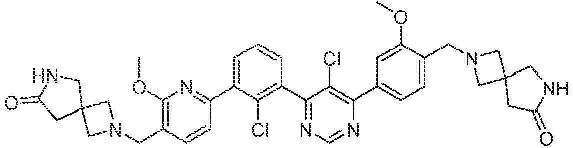

### (a) 2-((6-(2-Chloro-3-(5-chloro-6-(3-methoxy-4-((7-oxo-2,6-diazaspiro[3.4]octan-2-yl)methyl)phenyl)pyrimidin-4-yl)phenyl)-2-methoxypyridin-3-yl)methyl)-2,6-diazaspiro [3.4] octan-7-one

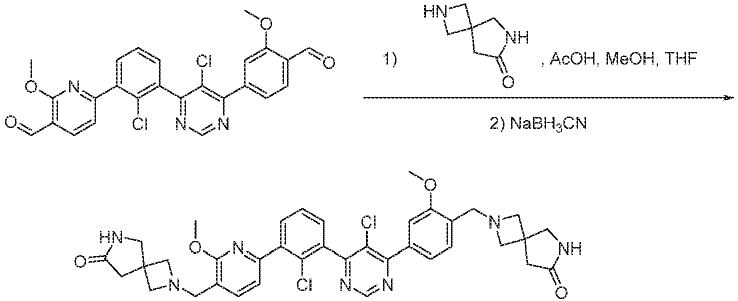

2-((6-(2-Chloro-3-(5-chloro-6-(3-methoxy-4-((7-oxo-2,6-diazaspiro[3.4]octan-2-yl)methyl)phenyl)pyrimidin-4-yl)phenyl)-2-methoxypyridin-3-yl)methyl)-2,6-diazaspiro[3.4]octan-7-one was prepared in a similar manner as described for Example 610, replacing 1-(4-aminopiperidin-1-yl)ethan-1-one with 2,6-diazaspiro[3.4]octan-7-one in step (c). MS: *m*/*z* found 714 [M+H]⁺, retention time = 2.32 min (Method A). ¹H NMR (400 MHz, Methanol-*d*₄) δ 9.22 (s, 1H), 8.49 (brs), 7.80 - 7.73 (m, 2H), 7.60 (dd, 1H), 7.55 - 7.49 (m, 4H), 7.30 (d, 1H), 4.17 (s, 2H), 4.02 (s, 3H), 3.97 (s, 3H), 3.94 (s, 2H), 3.89 (s, 4H), 3.68 (s, 4H), 3.64 (s, 2H), 3.62 (s, 2H), 2.66 (s, 2H), 2.63 (s, 2H).

### Example 613: 1-(4-((6-(3-(6-(4-((4-acetylpiperazin-1-yl)methyl)-3-methoxyphenyl)-5-chloropyrimidin-4-yl)-2-chlorophenyl)-2-methoxypyridin-3-yl)methyl)piperazin-1-yl)ethan-1-one (562)

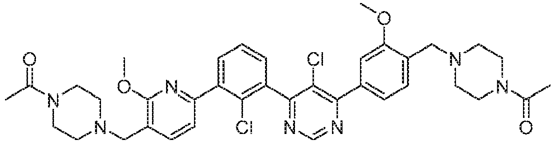

### (a) 1-(4-((6-(3-(6-(4-((4-Acetylpiperazin-1-yl)methyl)-3-methoxyphenyl)-5-chloropyrimidin-4-yl)-2-chlorophenyl)-2-methoxypyridin-3-yl)methyl)piperazin-1-yl)ethan-1-one

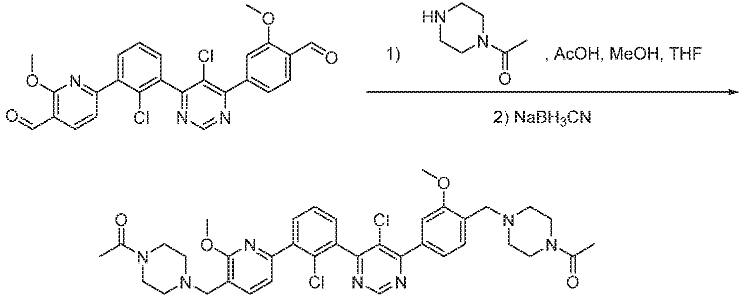

1-(4-((6-(3-(6-(4-((4-Acetylpiperazin-1-yl)methyl)-3-methoxyphenyl)-5-chloropyrimidin-4-yl)-2-chlorophenyl)-2-methoxypyridin-3-yl)methyl)piperazin-1-yl)ethan-1-one was prepared in a similar manner as described for Example 610, replacing 1-(4-aminopiperidin-1-yl)ethan-1-one with 1-(piperazin-1-yl)ethan-1-one in step (c). MS: *m*/*z* found 718 [M+H]⁺, retention time = 2.41 min (Method A). ¹H NMR (400 MHz, Methanol-*d*₄) δ 9.21 (s, 1H), 7.82 (d, 1H), 7.78 (dd, 1H), 7.59 (dd, 1H), 7.56 - 7.47 (m, 4H), 7.29 (d, 1H), 4.00 (s, 3H), 3.93 (s, 3H), 3.80 (s, 2H), 3.67 (s, 2H), 3.66 - 3.56 (m, 8H), 2.72 - 2.53 (m, 8H), 2.10 (s, 6H).

### Example 614: 2-((6-(2-chloro-3-(5-chloro-6-(4-((6-hydroxy-2-azaspiro[3.3]heptan-2-yl)methyl)-3-methoxyphenyl)pyrimidin-4-yl)phenyl)-2-methoxypyridin-3-yl)methyl)-2-azaspiro[3.3]heptan-6-ol (568)

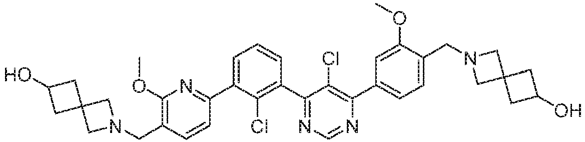

A mixture of 6-(2-chloro-3-(5-chloro-6-(4-formyl-3-methoxyphenyl)pyrimidin-4-yl)phenyl)-2-methoxynicotinaldehyde (Example 610, step (b)) (50 mg, 0.10 mmol), 2-azaspiro[3.3]heptan-6-ol hydrochloride (45 mg, 0.30 mmol) and sodium acetate (25 mg, 0.30 mmol) in dry methanol (1.5 mL) and dry THF (1.5 mL) was stirred at room temperature under N₂ for 19 hours. Sodium cyanoborohydride (25 mg, 0.40 mmol) was then added and stirring continued for 2 hours. The reaction was then quenched by the addition of saturated aqueous sodium hydrogen carbonate solution (5 mL) and the resulting suspension stirred vigorously for 15 min. The mixture was then partitioned between ethyl acetate (60 mL) and 1/2 saturated brine (30 mL) and the aqueous phase back extracted with ethyl acetate (30 mL). The combined organic extracts were washed with saturated brine (30 mL), dried over sodium sulfate, filtered and concentrated under reduced pressure to give a white foam (70 mg). The crude product was purified using reverse phase HPLC to give 2-((6-(2-chloro-3-(5-chloro-6-(4-((6-hydroxy-2-azaspiro[3.3]heptan-2-yl)methyl)-3-methoxyphenyl)pyrimidin-4-yl)phenyl)-2-methoxypyridin-3-yl)methyl)-2-azaspiro[3.3]heptan-6-ol (38.1 mg, 55%) as a white solid (formic acid salt). MS: *m*/*z* found 688 [M+H]⁺, retention time = 2.49 min (Method A). ¹H NMR (400 MHz, Methanol-*d*₄) δ 9.24 (s, 1H), 8.51 (brs), 7.84 (d, 1H), 7.78 (dd, 1H), 7.61 (dd, 1H), 7.58 - 7.50 (m, 4H), 7.35 (d, 1H), 4.38 (s, 2H), 4.27 (s, 2H), 4.18 - 4.09 (m, 6H), 4.07 (s, 2H), 4.06 (s, 3H), 4.04 (s, 2H), 4.00 (s, 3H), 2.68 - 2.56 (m, 4H), 2.20 - 2.08 (m, 4H).

### Example 615: 2-(((6-(2-Chloro-3-(5-chloro-6-(4-(((2-hydroxyethyl)amino)methyl)-3-methoxyphenyl)pyrimidin-4-yl)phenyl)-2-methoxypyridin-3-yl)methyl)amino)ethan-1-ol

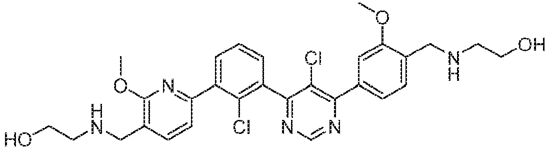

2-(((6-(2-Chloro-3-(5-chloro-6-(4-(((2-hydroxyethyl)amino)methyl)-3-methoxyphenyl)pyrimidin-4-yl)phenyl)-2-methoxypyridin-3-yl)methyl)amino)ethan-1-ol was prepared in a similar manner as described for Example 610, replacing 1-(4-aminopiperidin-1-yl)ethan-1-one with 2-aminoethan-1-ol in step (c). MS: *m*/*z* found 584 [M+H]⁺, retention time = 2.31 min (Method A). ¹H NMR (400 MHz, Methanol-*d*₄) δ 9.24 (s, 1H), 8.48 (brs), 7.89 (d, 1H), 7.79 (dd, 1H), 7.62 (dd, 1H), 7.60 - 7.52 (m, 4H), 7.37 (d, 1H), 4.34 (s, 2H), 4.29 (s, 2H), 4.09 (s, 3H), 4.02 (s, 3H), 3.87 - 3.81 (m, 4H), 3.20 - 3.14 (m, 4H).

### Example 616: 1-(6-((6-(3-(6-(4-((6-Acetyl-2,6-diazaspiro[3.3]heptan-2-yl)methyl)-3-methoxyphenyl)-5-chloropyrimidin-4-yl)-2-chlorophenyl)-2-methoxypyridin-3-yl)methyl)-2,6-diazaspiro[3.3]heptan-2-yl)ethan-1-one (589)

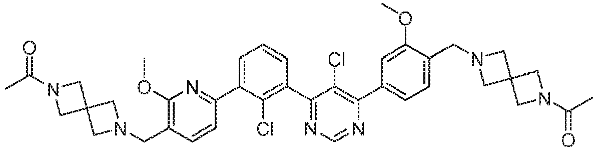

1-(6-((6-(3-(6-(4-((6-Acetyl-2,6-diazaspiro[3.3]heptan-2-yl)methyl)-3-methoxyphenyl)-5-chloropyrimidin-4-yl)-2-chlorophenyl)-2-methoxypyridin-3-yl)methyl)-2,6-diazaspiro[3.3]heptan-2-yl)ethan-1-one was prepared in a similar manner as described for Example 614, replacing 2-azaspiro[3.3]heptan-6-ol hydrochloride with 1-(2,6-diazaspiro[3.3]heptan-2-yl)ethan-1-one hydrochloride in step (a). MS: *m*/*z* found 742 [M+H]⁺, retention time = 2.43 min (Method A). ¹H NMR (400 MHz, Methanol-*d*₄) δ 9.23 (s, 1H), 8.42 (brs), 7.82 - 7.74 (m, 2H), 7.61 (dd, 1H), 7.57 - 7.50 (m, 4H), 7.33 (d, 1H), 4.37 (s, 2H), 4.35 (s, 2H), 4.30 (s, 2H), 4.20 - 4.15 (m, 4H), 4.15 - 4.07 (m, 7H), 4.05 (s, 3H), 4.02 - 3.95 (m, 8H), 1.85 (s, 6H).

### Example 617: (1s,3s)-3-(((6-(2-Chloro-3-(5-chloro-6-(4-((((1s,3s)-3-hydroxycyclobutyl)amino)methyl)-3-methoxyphenyl)pyrimidin-4-yl)phenyl)-2-methoxypyridin-3-yl)methyl)amino)cyclobutan-1-ol (592)

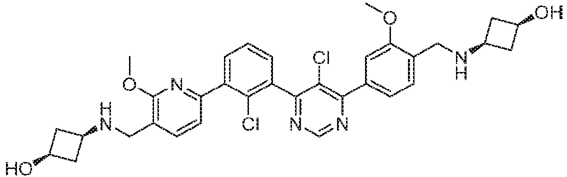

(1s,3s)-3-(((6-(2-Chloro-3-(5-chloro-6-(4-((((1s,3s)-3-hydroxycyclobutyl)amino)methyl)-3-methoxyphenyl)pyrimidin-4-yl)phenyl)-2-methoxypyridin-3-yl)methyl)amino)cyclobutan-1-ol was prepared in a similar manner as described for Example 610, replacing 1-(4-aminopiperidin-1-yl)ethan-1-one with cis-3-aminocyclobutanol in step (c). MS: *m*/*z* found 636 [M+H]⁺, retention time = 2.43 min (Method A). ¹H NMR (400 MHz, Methanol-*d*₄) δ 9.24 (s, 1H), 8.49 (brs), 7.86 (d, 1H), 7.78 (dd, 1H), 7.62 (dd, 1H), 7.58 - 7.51 (m, 4H), 7.35 (d, 1H), 4.18 (s, 2H), 4.11 (s, 2H), 4.09 (s, 3H), 4.10 - 4.04 (m, 2H), 4.01 (s, 3H), 3.38 - 3.27 (m, 2H), 2.78 - 2.65 (m, 4H), 2.13 - 1.96 (m, 4H).

### Example 618: (1r,3r)-3-(((6-(2-Chloro-3-(5-chloro-6-(4-((((1r,3r)-3-hydroxycyclobutyl)amino)methyl)-3-methoxyphenyl)pyrimidin-4-yl)phenyl)-2-methoxypyridin-3-yl)methyl)amino)cyclobutan-1-ol (593)

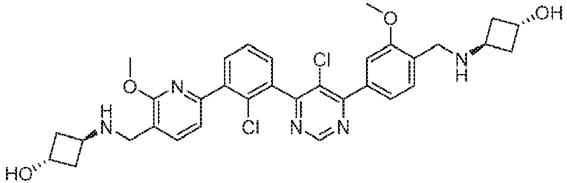

(1r,3r)-3-(((6-(2-Chloro-3-(5-chloro-6-(4-((((1r,3r)-3-hydroxycyclobutyl)amino)methyl)-3-methoxyphenyl)pyrimidin-4-yl)phenyl)-2-methoxypyridin-3-yl)methyl)amino)cyclobutan-1-ol was prepared in a similar manner as described for Example 610, replacing 1-(4-aminopiperidin-1-yl)ethan-1-one with trans-3-aminocyclobutanol in step (c). MS: *m*/*z* found 636 [M+H]⁺, retention time = 2.38 min (Method A). ¹H NMR (400 MHz, Methanol-*d*₄) δ 9.24 (s, 1H), 8.50 (brs, 2H), 7.86 (d, 1H), 7.78 (dd, 1H), 7.62 (dd, 1H), 7.58 - 7.51 (m, 4H), 7.35 (d, 1H), 4.51 - 4.43 (m, 2H), 4.17 (s, 2H), 4.10 (s, 2H), 4.08 (s, 3H), 4.01 (s, 3H), 3.98 - 3.82 (m, 2H), 2.56 - 2.42 (m, 4H), 2.38 - 2.26 (m, 4H).

### Example 619: N-((6-(2-Chloro-3-(5-chloro-6-(3-methoxy-4-(((tetrahydro-2H-pyran-4-yl)amino)methyl)phenyl)pyrimidin-4-yl)phenyl)-2-methoxypyridin-3-yl)methyl)tetrahydro-2H-pyran-4-amine (626)

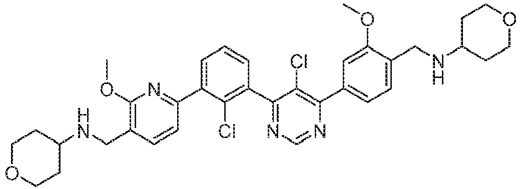

N-((6-(2-Chloro-3-(5-chloro-6-(3-methoxy-4-(((tetrahydro-2H-pyran-4-yl)amino)methyl)phenyl)pyrimidin-4-yl)phenyl)-2-methoxypyridin-3-yl)methyl)tetrahydro-2H-pyran-4-amine was prepared in a similar manner as described for Example 610, replacing 1-(4-aminopiperidin-1-yl)ethan-1-one with tetrahydro-2H-pyran-4-amine in step (c). MS: *m*/*z* found 664 [M+H]⁺, retention time = 2.65 min (Method A). ¹H NMR (400 MHz, Methanol-*d*₄) d 9.23 (s, 1H), 8.53 (brs), 7.84 (d, 1H), 7.78 (dd, 1H), 7.61 (dd, 1H), 7.58 - 7.51 (m, 4H), 7.33 (d, 1H), 4.22 (s, 2H), 4.10 (s, 2H), 4.06 (s, 3H), 4.06 - 4.01 (m, 4H), 4.00 (s, 3H), 3.51 - 3.40 (m, 4H), 3.29 - 3.21 (m, 1H), 3.19 - 3.08 (m, 1H), 2.12 - 1.98 (m, 4H), 1.75 - 1.54 (m, 4H).

### Example 620: 1-(6-(2-Chloro-3-(5-chloro-6-(3-methoxy-4-(((oxazol-5-ylmethyl)amino)methyl)phenyl)pyrimidin-4-yl)phenyl)-2-methoxypyridin-3-yl)-N-(oxazol-5-ylmethyl)methanamine (643)

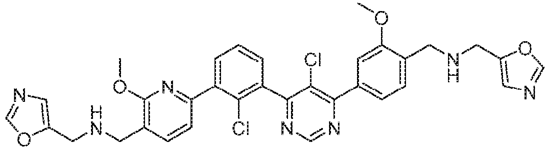

1-(6-(2-Chloro-3-(5-chloro-6-(3-methoxy-4-(((oxazol-5-ylmethyl)amino)methyl)phenyl)pyrimidin-4-yl)phenyl)-2-methoxypyridin-3-yl)-N-(oxazol-5-ylmethyl)methanamine was prepared in a similar manner as described for Example 614, replacing 2-azaspiro[3.3]heptan-6-ol hydrochloride with oxazol-5-ylmethanamine hydrochloride in step (c). MS: *m*/*z* found 658 [M+H]⁺, retention time = 2.41 min (Method A). ¹H NMR (400 MHz, Methanol-*d*₄) δ 9.22 (s, 1H), 8.35 (brs), 8.28 (s, 1H), 8.24 (s, 1H), 7.80 - 7.75 (m, 2H), 7.60 (dd, 1H), 7.56 - 7.49 (m, 4H), 7.30 (d, 1H), 7.25 (s, 1H), 7.18 (s, 1H), 4.26 - 4.20 (m, 2H), 4.13 (s, 2H), 4.12 - 4.10 (m, 2H), 4.03 (s, 3H), 3.99 (s, 2H), 3.97 (s, 3H).

### Example 621: N-(2-Chloro-3-(3-chloro-2-(3-methoxy-4-((7-oxo-2,6-diazaspiro[3.4]octan-2-yl)methyl)phenyl)pyridin-4-yl)phenyl)-5-((7-oxo-2,6-diazaspiro[3.4]octan-2-yl)methyl)picolinamide (672)

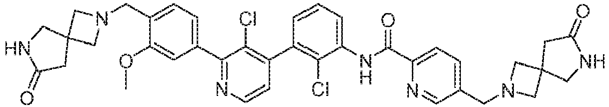

### (a) 2-Chloro-3-(2,3-dichloropyridin-4-yl)aniline

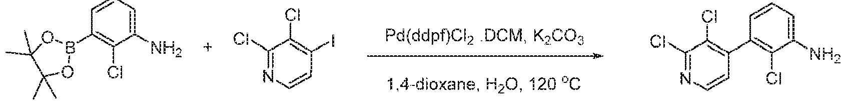

2-Chloro-3-(4,4,5,5-tetramethyl-1,3,2-dioxaborolan-2-yl)aniline (1000 mg, 3.94 mmol), 2,3-dichloro-4-iodo-pyridine (1080 mg, 3.94 mmol), [1,1'-bis(diphenylphosphino)ferrocene] dichloropalladium(II) complex with dichloromethane (322 mg, 0.39 mmol) and potassium carbonate (1633 mg, 11.83 mmol) were suspended in 1,4-dioxane/water (5:1) in a sealed tube. Nitrogen gas was bubbled through the reaction solution for 10 minutes, then the vessel was sealed and heated at 120 °C for 1 hour. Reaction was cooled to room temperature, diluted with water, and extracted with EtOAc. The combined organics were concentrated and the crude sample was purified by silica gel column, eluting with 0-40% EtOAc gradient in hexane to give 2-chloro-3-(2,3-dichloropyridin-4-yl)aniline (950 mg, 88%). LCMS: m/z found 273.0 [M+H]⁺, retention time = 0.94 min (Method B).

### (b) N-(2-Chloro-3-(2,3-dichloropyridin-4-yl)phenyl)-5-(dimethoxymethyl)picolinamide

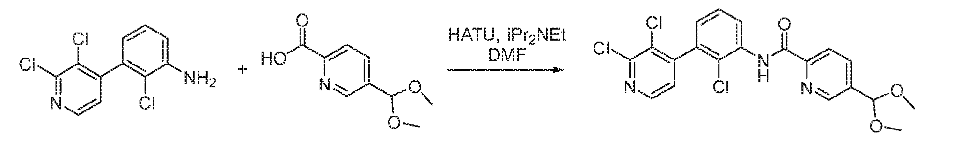

To a solution of 5-(dimethoxymethyl)pyridine-2-carboxylic acid (1153 mg, 5.85 mmol) and HATU (2224 mg, 5.85 mmol) in DMF was added N,N-diisopropylethylamine (1.02 mL, 756 mg, 5.85 mmol). After stirring 10 minutes, 2-chloro-3-(2,3-dichloro-4-pyridyl)aniline (400 mg, 1.46 mmol) was added in portions and the reaction mixture was stirred overnight at room temperature. The mixture was diluted with water and extracted with EtOAc. The combined organic extracts were washed with brine, dried over sodium sulfate, concentrated in vacuo, and purified by flash column chromatography to give N-[2-chloro-3-(2,3-dichloro-4-pyridyl)phenyl]-5-(dimethoxymethyl)pyridine-2-carboxamide (650 mg, 98%). LCMS: m/z found 452.0 [M+H]⁺, retention time = 1.14 min (Method B).

### (c) N-(2-Chloro-3-(2,3-dichloropyridin-4-yl)phenyl)-5-formylpicolinamide

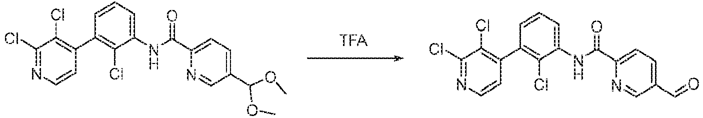

A mixture of N-[2-chloro-3-(2,3-dichloro-4-pyridyl)phenyl]-5-(dimethoxymethyl)pyridine-2-carboxamide (700 mg, 1.55 mmol) and trifluoroacetic acid (5 mL) was stirred at room temperature for 2 hrs. Excess trifluoroacetic acid was evaporated. The resulting mixture was neutralized to pH=8 by sat. aq. sodium hydrogen carbonate and extracted with DCM three times. The combined organic layer was dried over sodium sulfate and concentrated under reduced pressure to give N-[2-chloro-3-(2,3-dichloro-4-pyridyl)phenyl]-5-formyl-pyridine-2-carboxamide (620 mg, 99%). LCMS: m/z found 406.0 [M+H]⁺, retention time = 1.06 min (Method B).

### (d) N-(2-Chloro-3-(3-chloro-2-(4-formyl-3-methoxyphenyl)pyridin-4-yl)phenyl)-5-formylpicolinamide

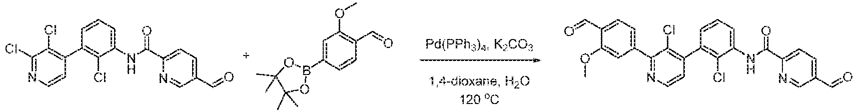

To a mixture of N-[2-chloro-3-(2,3-dichloro-4-pyridyl)phenyl]-5-formyl-pyridine-2-carboxamide (500 mg, 1.23 mmol), 2-methoxy-4-(4,4,5,5-tetramethyl-1,3,2-dioxaborolan-2-yl)benzaldehyde (322 mg, 1.23 mmol) and potassium carbonate (510 mg, 3.69 mmol) in degassed 1,4-dioxane/water (6: 1) was added tetrakis(triphenylphosphine)palladium(0) (142 mg, 0.12 mmol) and the mixture was stirred at 120 °C for 2 hrs. Water was added and the mixture was extracted into EtOAc three times. The combined organic layers were dried over anhydrous sodium sulfate, concentrated, and purified on silica gel column to provide N-[2-chloro-3-[3-chloro-2-(4-formyl-3-methoxy-phenyl)-4-pyridyl]phenyl]-5-formyl-pyridine-2-carboxamide (350 mg, 56%). LCMS: m/z found 506.1 [M+H]⁺, retention time = 1.03 min (Method B).

### (e) N-(2-Chloro-3-(3-chloro-2-(3-methoxy-4-((7-oxo-2,6-diazaspiro[3.4] octan-2-yl)methyl)phenyl)pyridin-4-yl)phenyl)-5-((7-oxo-2,6-diazaspiro[3.4]octan-2-yl)methyl)picolinamide

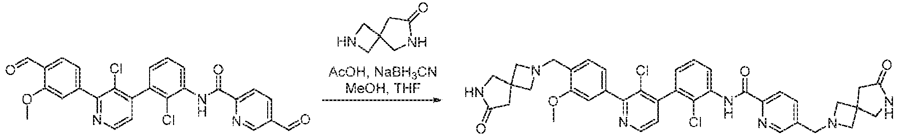

This compound was prepared as the formic acid salt following Reductive Amination Procedure A from N-[2-chloro-3-[3-chloro-2-(4-formyl-3-methoxy-phenyl)-4-pyridyl]phenyl]-5-formyl-pyridine-2-carboxamide (30 mg, 0.06 mmol) and 2,6-diazaspiro[3.4]octan-7-one (30 mg, 0.24 mmol). 81% yield. LCMS: m/z found 726.2 [M+H]⁺, retention time = 2.46 min (Method A). ¹H NMR (400 MHz, Methanol-*d*₄): δ 8.70 - 8.60 (m, 3H), 8.37 (s, 2H), 8.24 (d, 1H), 8.00 (d, 1H), 7.58 - 7.49 (m, 2H), 7.46 (d, 1H), 7.39 (s, 1H), 7.36 (d, 1H), 7.24 - 7.15 (m, 1H), 4.37 (s, 2H), 4.13 (s, 4H), 3.99 (s, 3H), 3.83 (s, 2H), 3.67 (s, 2H), 3.58 (s, 2H), 3.41 (s, 4H), 2.71 (s, 2H), 2.58 (s, 2H).

### Example 622: 5-(((1-Acetylpiperidin-4-yl)amino)methyl)-N-(3-(2-(4-(((1-acetylpiperidin-4-yl)amino)methyl)-3-methoxyphenyl)-3-chloropyridin-4-yl)-2-chlorophenyl)picolinamide (673)

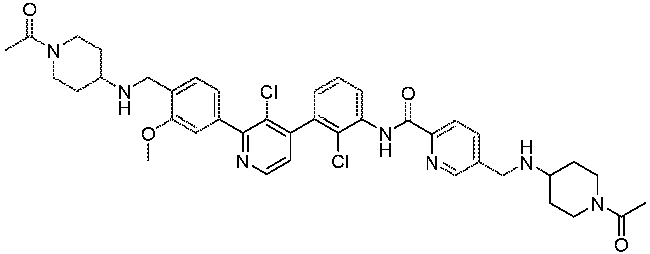

5-(((1-Acetylpiperidin-4-yl)amino)methyl)-N-(3-(2-(4-(((1-acetylpiperidin-4-yl)amino)methyl)-3-methoxyphenyl)-3-chloropyridin-4-yl)-2-chlorophenyl)picolinamide was prepared as the formic acid salt following Reductive Amination Procedure A from N-[2-chloro-3-[3-chloro-2-(4-formyl-3-methoxy-phenyl)-4-pyridyl]phenyl]-5-formyl-pyridine-2-carboxamide (30 mg, 0.06 mmol) and 1-(4-amino-1-piperidyl)ethanone (34 mg, 0.24 mmol). 82% yield. LCMS: m/z found 758.3 [M+H]⁺, retention time = 2.64 min (Method A). ¹H NMR (400 MHz, Methanol-*d*₄): δ 8.74 (d, 1H), 8.71 - 8.59 (m, 2H), 8.43 (s, 1H), 8.28 (d, 1H), 8.10 (d, 1H), 7.54 (dt, 2H), 7.47 (d, 1H), 7.41 (s, 1H), 7.39 - 7.34 (m, 1H), 7.20 (dd, 1H), 4.67 (d, 1H), 4.50 (d, 1H), 4.32 (s, 2H), 4.08 (s, 3H), 4.04 - 3.89 (m, 4H), 3.45 (t, 1H), 3.26 - 3.09 (m, 2H), 3.01 - 2.87 (m, 1H), 2.79 - 2.63 (m, 2H), 2.24 (t, 2H), 2.16 - 1.98 (m, 8H), 1.77 - 1.21 (m, 4H).

### Example 623: N-(2-Chloro-3-(3-chloro-2-(3-methoxy-4-(((tetrahydro-2H-pyran-4-yl)amino)methyl)phenyl)pyridin-4-yl)phenyl)-5-(((tetrahydro-2H-pyran-4-yl)amino)methyl)picolinamide (674)

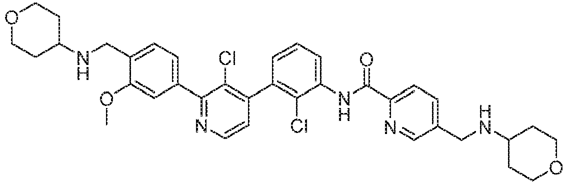

N-(2-Chloro-3-(3-chloro-2-(3-methoxy-4-(((tetrahydro-2H-pyran-4-yl)amino)methyl)phenyl)pyridin-4-yl)phenyl)-5-(((tetrahydro-2H-pyran-4-yl)amino)methyl)picolinamide was prepared as the formic acid salt following Reductive Amination Procedure A from N-[2-chloro-3-[3-chloro-2-(4-formyl-3-methoxy-phenyl)-4-pyridyl]phenyl]-5-formyl-pyridine-2-carboxamide (30 mg, 0.06 mmol) and tetrahydropyran-4-amine (24 mg, 0.24 mmol). 87% yield. LCMS: m/z found 676.2 [M+H]⁺, retention time = 2.73 min (Method A). ¹H NMR (400 MHz, Methanol-*d*₄): δ 8.74 (d, 1H), 8.71 - 8.62 (m, 2H), 8.46 (s, 1H), 8.28 (d, 1H), 8.17 - 8.05 (m, 1H), 7.59 - 7.50 (m, 2H), 7.47 (d, 1H), 7.41 (s, 1H), 7.37 (dd, 1H), 7.21 (dd, 1H), 4.31 (s, 2H), 4.10 (s, 2H), 4.09 - 4.03 (m, 2H), 4.03 - 3.93 (m, 5H), 3.52 - 3.36 (m, 5H), 3.04 - 2.90 (m, 1H), 2.12 (d, 2H), 1.98 (d, 2H), 1.82 - 1.67 (m, 2H), 1.62 - 1.43 (m, 2H).

### Example 624: N-(2-Chloro-3-(3-chloro-2-(3-methoxy-4-(((((S)-5-oxopyrrolidin-2-yl)methyl)amino)methyl)phenyl)pyridin-4-yl)phenyl)-5-(((((S)-5-oxopyrrolidin-2-yl)methyl)amino)methyl)picolinamide (675)

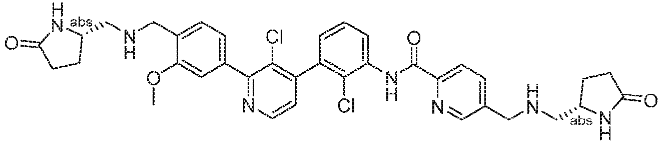

N-(2-Chloro-3-(3-chloro-2-(3-methoxy-4-(((((S)-5-oxopyrrolidin-2-yl)methyl)amino)methyl)phenyl)pyridin-4-yl)phenyl)-5-(((((S)-5-oxopyrrolidin-2-yl)methyl)amino)methyl)picolinamide was prepared as the formic acid salt following Reductive Amination Procedure A from N-[2-chloro-3-[3-chloro-2-(4-formyl-3-methoxyphenyl)-4-pyridyl]phenyl]-5-formyl-pyridine-2-carboxamide (35 mg, 0.07 mmol) and (5S)-5-(aminomethyl)pyrrolidin-2-one (32 mg, 0.28 mmol). 90% yield. LCMS: m/z found 702.2 [M+H]⁺, retention time = 2.48 min (Method A). ¹H NMR (400 MHz, Methanol-*d*₄): δ 8.76 - 8.58 (m, 3H), 8.40 (s, 2H), 8.25 (d, 1H), 8.12 - 8.01 (m, 1H), 7.60 - 7.49 (m, 2H), 7.47 (d, 1H), 7.39 (s, 1H), 7.36 (d, 1H), 7.20 (d, 1H), 4.24 (d, 2H), 4.06 - 3.89 (m, 6H), 3.84 (q, 1H), 3.16 - 3.00 (m, 2H), 2.84 - 2.62 (m, 2H), 2.48 - 2.19 (m, 6H), 1.98 - 1.73 (m, 2H).

### Example 625: N-(2-Chloro-3-(3-chloro-2-(4-(((2-hydroxyethyl)amino)methyl)-3-methoxyphenyl)pyridin-4-yl)phenyl)-5-(((2-hydroxyethyl)amino)methyl)picolinamide (676)

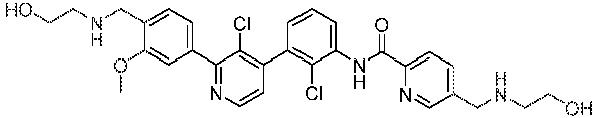

N-(2-Chloro-3-(3-chloro-2-(4-(((2-hydroxyethyl)amino)methyl)-3-methoxyphenyl)pyridin-4-yl)phenyl)-5-(((2-hydroxyethyl)amino)methyl)picolinamide was prepared as the formic acid salt following Reductive Amination Procedure A from N-[2-chloro-3-[3-chloro-2-(4-formyl-3-methoxy-phenyl)-4-pyridyl]phenyl]-5-formyl-pyridine-2-carboxamide (40 mg, 0.08 mmol) and 2-aminoethanol (19 mg, 0.32 mmol). 91% yield. LCMS: m/z found 596.2 [M+H]⁺, retention time = 2.43 min (Method A). ¹H NMR (400 MHz, Methanol-*d*₄): δ 8.76 (d, 1H), 8.71 - 8.61 (m, 2H), 8.50 (s, 1H), 8.30 (d, 1H), 8.12 (dd, 1H), 7.59 - 7.49 (m, 2H), 7.47 (d, 1H), 7.41 (d, 1H), 7.36 (dd, 1H), 7.21 (dd, 1H), 4.32 (s, 2H), 4.15 (s, 2H), 4.00 (s, 3H), 3.87 - 3.81 (m, 2H), 3.76 (t, 2H), 3.16 (t, 2H), 2.96 (t, 2H).

### Example 626: 5-((6-Acetyl-2,6-diazaspiro[3.3]heptan-2-yl)methyl)-N-(3-(2-(4-((6-acetyl-2,6-diazaspiro[3.3]heptan-2-yl)methyl)-3-methoxyphenyl)-3-chloropyridin-4-yl)-2-chlorophenyl)picolinamide (677)

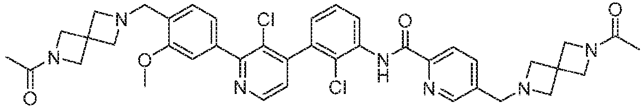

5-((6-Acetyl-2,6-diazaspiro[3.3]heptan-2-yl)methyl)-N-(3-(2-(4-((6-acetyl-2,6-diazaspiro[3.3]heptan-2-yl)methyl)-3-methoxyphenyl)-3-chloropyridin-4-yl)-2-chlorophenyl)picolinamide was prepared as the formic acid salt following Reductive Amination Procedure A from N-[2-chloro-3-[3-chloro-2-(4-formyl-3-methoxy-phenyl)-4-pyridyl]phenyl]-5-formyl-pyridine-2-carboxamide (40 mg, 0.08 mmol) and 1-(2,6-diazaspiro[3.3]heptan-2-yl)ethanone (44 mg, 0.32 mmol). 34% yield. LCMS: m/z found 754.3 [M+H]⁺, retention time = 2.59 min (Method A). ¹H NMR (400 MHz, Methanol-*d*₄): δ 8.70 - 8.61 (m, 3H), 8.40 (s, 2H), 8.24 (d, 1H), 7.99 (d, 1H), 7.54 (t, 1H), 7.51 - 7.43 (m, 2H), 7.39 (s, 1H), 7.36 (d, 1H), 7.24 - 7.15 (m, 1H), 4.37 (s, 2H), 4.30 (s, 4H), 4.23 - 4.09 (m, 6H), 4.05 (s, 2H), 3.98 (s, 3H), 3.79 (s, 2H), 3.56 - 3.42 (m, 4H), 1.91 - 1.79 (m, 6H).

### Example 627: N-(2-Chloro-3-(3-chloro-2-(4-((((S)-2-hydroxypropyl)amino)methyl)-3-methoxyphenyl)pyridin-4-yl)phenyl)-5-((((S)-2-hydroxypropyl)amino)methyl)picolinamide (678)

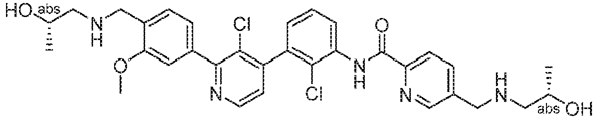

N-(2-Chloro-3-(3-chloro-2-(4-((((S)-2-hydroxypropyl)amino)methyl)-3-methoxyphenyl)pyridin-4-yl)phenyl)-5-((((S)-2-hydroxypropyl)amino)methyl)picolinamide was prepared as the formic acid salt following Reductive Amination Procedure A from N-[2-chloro-3-[3-chloro-2-(4-formyl-3-methoxy-phenyl)-4-pyridyl]phenyl]-5-formyl-pyridine-2-carboxamide (40 mg, 0.08 mmol) and (2S)-1-aminopropan-2-ol (24 mg, 0.32 mmol). 90% yield. LCMS: m/z found 624.2 [M+H]⁺, retention time = 2.62 min (Method A). ¹H NMR (400 MHz, Methanol-*d*₄): δ 8.73 (s, 1H), 8.70 - 8.62 (m, 2H), 8.52 (s, 1H), 8.28 (d, 1H), 8.10 (dd, 1H), 7.59 - 7.49 (m, 2H), 7.47 (d, 1H), 7.40 (d, 1H), 7.36 (dd, 1H), 7.21 (dd, 1H), 4.30 (s, 2H), 4.13 - 4.02 (m, 3H), 4.00 (s, 3H), 3.98 - 3.87 (m, 1H), 3.07 (dd, 1H), 2.86 (dd, 1H), 2.78 (dd, 1H), 2.66 (dd, 1H), 1.23 (d, 3H), 1.19 (d, 3H).

### Example 628: N-(3-(3-Chloro-2-(3-methoxy-4-((7-oxo-2,6-diazaspiro[3.4]octan-2-yl)methyl)phenyl)pyridin-4-yl)-2-methylphenyl)-5-((7-oxo-2,6-diazaspiro[3.4]octan-2-yl)methyl)picolinamide (590)

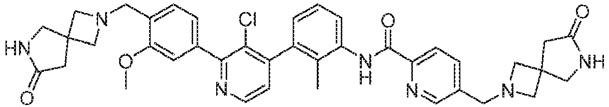

### (a) 3-(2,3-Dichloropyridin-4-yl)-2-methylaniline

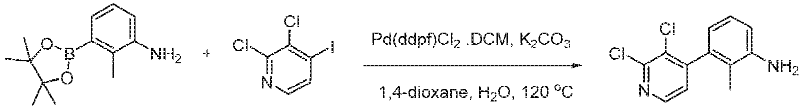

2-Methyl-3-(4,4,5,5-tetramethyl-1,3,2-dioxaborolan-2-yl)aniline (500 mg, 2.14 mmol), 2,3-dichloro-4-iodo-pyridine (587 mg, 2.14 mmol), [1,1'- bis(diphenylphosphino)-ferrocene]dichloropalladium(II) complex with dichloromethane (175 mg, 0.21 mmol) and potassium carbonate (888 mg, 6.43 mmol) were suspended in 1,4-dioxane/water (5:1) in a sealed tube. Nitrogen gas was bubbled through the reaction solution for 10 minutes, then the vessel was sealed and heated at 120 °C for 90 minutes. Reaction was cooled to room temperature, diluted with water, and extracted with EtOAc. The combined organics were concentrated, and the crude sample was purified by silica gel column, eluting with 0-40% EtOAc gradient in hexane to give 3-(2,3-dichloro-4-pyridyl)-2-methyl-aniline (500 mg, 92%). LCMS: m/z found 253.0 [M+H]⁺, retention time = 0.73 min (Method B).

### (b) N-(3-(2,3-Dichloropyridin-4-yl)-2-methylphenyl)-5-(dimethoxymethyl)picolinamide

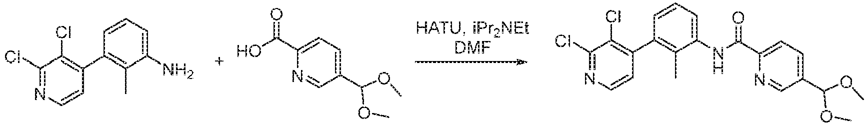

To a solution of 5-(dimethoxymethyl)pyridine-2-carboxylic acid (584 mg, 2.96 mmol) and HATU (1127 mg, 2.96 mmol) in DMF was added N,N-diisopropylethylamine (0.52 mL, 383 mg, 2.96 mmol). After stirring 10 minutes, 3-(2,3-dichloro-4-pyridyl)-2-methyl-aniline (250 mg, 0.99 mmol) was added in portions and the reaction mixture was stirred overnight at room temperature. The mixture was diluted with water and extracted with EtOAc. The combined organic extracts were washed with brine, dried over sodium sulfate, concentrated in vacuo, and purified by flash column chromatography to give N-[3-(2,3-dichloro-4-pyridyl)-2-methyl-phenyl]-5-(dimethoxymethyl) pyridine-2-carboxamide (406 mg, 95%). LCMS: m/z found 432.1 [M+H]⁺, retention time = 1.08 min (Method B).

### (c) N-(3-(2,3-Dichloropyridin-4-yl)-2-methylphenyl)-5-formylpicolinamide

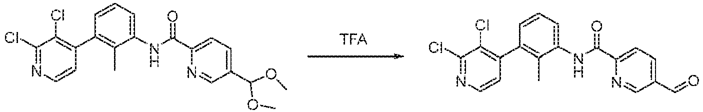

A mixture of N-[3-(2,3-dichloro-4-pyridyl)-2-methyl-phenyl]-5-(dimethoxymethyl)pyridine-2-carboxamide (420 mg, 0.97 mmol) and trifluoroacetic acid (4 mL) was stirred at room temperature for 2 hrs. Excess trifluoroacetic acid was evaporated. The resulting mixture was neutralized to pH=8 by sat. aq. sodium hydrogen carbonate and extracted with DCM three times. The combined organic layer was dried over sodium sulfate and concentrated under reduced pressure to give N-[3-(2,3-dichloro-4-pyridyl)-2-methyl-phenyl]-5-formyl-pyridine-2-carboxamide (370 mg, 99%). LCMS: m/z found 386.0 [M+H]⁺, retention time = 0.99 min (Method B).

### (d) N-(2-Chloro-3-(3-chloro-2-(4-formyl-3-methoxyphenyl)pyridin-4-yl)phenyl)-5-formylpicolinamide

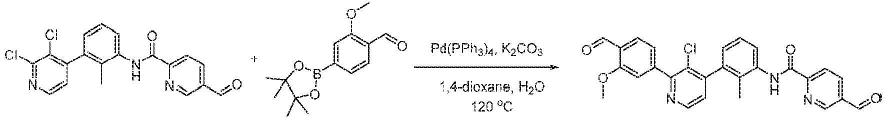

To a mixture of N-[3-(2,3-dichloro-4-pyridyl)-2-methyl-phenyl]-5-formyl-pyridine-2-carboxamide (300 mg, 0.78 mmol), 2-methoxy-4-(4,4,5,5-tetramethyl-1,3,2-dioxaborolan-2-yl)benzaldehyde (204 mg, 0.78 mmol) and potassium carbonate (322 mg, 2.33 mmol) in degassed 1,4-dioxane/water (6:1) was added tetrakis(triphenylphosphine)palladium(0) (90 mg, 0.08 mmol) and the mixture was stirred at 120 °C for 4 hrs. Water was added and the mixture was extracted into EtOAc three times. The combined organic layers were dried over anhydrous sodium sulfate, concentrated, and purified on silica gel column to provide N-[3-[3-chloro-2-(4-formyl-3-methoxy-phenyl)-4-pyridyl]-2-methyl-phenyl]-5-formyl-pyridine-2-carboxamide (200 mg, 53%). LCMS: m/z found 486.1 [M+H]⁺, retention time = 0.98 min (Method B).

### (e) N-(3-(3-Chloro-2-(3-methoxy-4-((7-oxo-2,6-diazaspiro[3.4]octan-2-yl)methyl)phenyl)pyridin-4-yl)-2-methylphenyl)-5-((7-oxo-2,6-diazaspiro[3.4]octan-2-yl)methyl)picolinamide

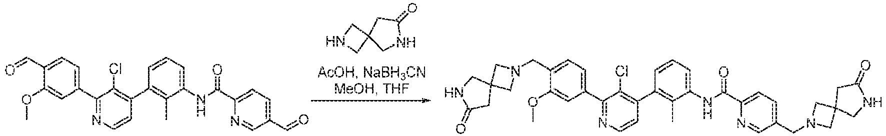

N-(3-(3-Chloro-2-(3-methoxy-4-((7-oxo-2,6-diazaspiro[3.4]octan-2-yl)methyl)phenyl)pyridin-4-yl)-2-methylphenyl)-5-((7-oxo-2,6-diazaspiro[3.4]octan-2-yl)methyl)picolinamide was prepared as the formic acid salt following Reductive Amination Procedure A from N-[3-[3-chloro-2-(4-formyl-3-methoxy-phenyl)-4-pyridyl]-2-methylphenyl]-5-formyl-pyridine-2-carboxamide (30 mg, 0.06 mmol) and 2,6-diazaspiro[3.4]octan-7-one (31 mg, 0.25 mmol). 82% yield. LCMS: m/z found 706.3 [M+H]⁺, retention time = 2.27 min (Method A). ¹H NMR (400 MHz, Methanol-*d*₄): δ 8.69 - 8.58 (m, 2H), 8.36 (s, 2H), 8.20 (d, 1H), 7.97 (t, 2H), 7.51 (d, 1H), 7.45 - 7.37 (m, 3H), 7.35 (d, 1H), 7.11 (d, 1H), 4.37 (s, 2H), 4.13 (s, 4H), 3.98 (s, 3H), 3.83 (s, 2H), 3.67 (s, 2H), 3.59 (s, 2H), 3.45 - 3.36 (m, 4H), 2.71 (s, 2H), 2.58 (s, 2H), 2.17 (s, 3H).

### Example 629: 5-(((1-Acetylpiperidin-4-yl)amino)methyl)-N-(3-(2-(4-(((1-acetylpiperidin-4-yl)amino)methyl)-3-methoxyphenyl)-3-chloropyridin-4-yl)-2-methylphenyl)picolinamide (645)

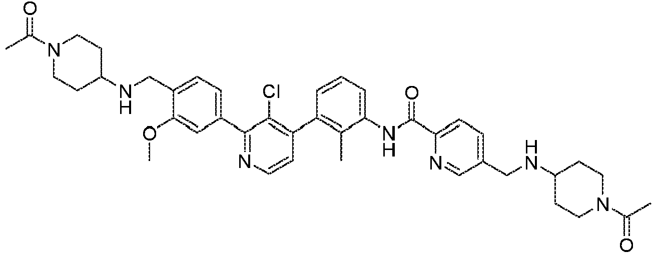

5-(((1-Acetylpiperidin-4-yl)amino)methyl)-N-(3-(2-(4-(((1-acetylpiperidin-4-yl)amino)methyl)-3-methoxyphenyl)-3-chloropyridin-4-yl)-2-methylphenyl)picolinamide was prepared as the formic acid salt following Reductive Amination Procedure A from N-[3-[3-chloro-2-(4-formyl-3-methoxy-phenyl)-4-pyridyl]-2-methyl-phenyl]-5-formyl-pyridine-2-carboxamide (30 mg, 0.06 mmol) and 1-(4-amino-1-piperidyl)ethanone (35 mg, 0.25 mmol). 50% yield. LCMS: m/z found 738.4 [M+H]⁺, retention time = 2.45 min (Method A). ¹H NMR (400 MHz, Methanol-*d*₄): δ 8.74 (s, 1H), 8.64 (d, 1H), 8.41 (s, 2H), 8.24 (d, 1H), 8.13 - 8.04 (m, 1H), 7.96 (d, 1H), 7.54 (d, 1H), 7.47 - 7.31 (m, 4H), 7.12 (d, 1H), 4.67 (d, 1H), 4.51 (d, 1H), 4.32 (s, 2H), 4.15 - 4.04 (m, 3H), 4.03 - 3.90 (m, 4H), 3.54 - 3.38 (m, 1H), 3.26 - 3.10 (m, 2H), 3.06 - 2.90 (m, 1H), 2.71 (q, 2H), 2.24 (t, 2H), 2.17 (s, 3H), 2.14 (s, 3H), 2.12 - 2.02 (m, 5H), 1.73 - 1.26 (m, 4H).

### Example 630: N-(3-(3-Chloro-2-(3-methoxy-4-(((tetrahydro-2H-pyran-4-yl)amino)methyl)phenyl)pyridin-4-yl)-2-methylphenyl)-5-(((tetrahydro-2H-pyran-4-yl)amino)methyl)picolinamide (646)

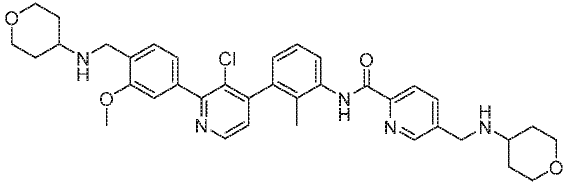

N-(3-(3-Chloro-2-(3-methoxy-4-(((tetrahydro-2H-pyran-4-yl)amino)methyl)phenyl)pyridin-4-yl)-2-methylphenyl)-5-(((tetrahydro-2H-pyran-4-yl)amino)methyl)picolinamide was prepared as the formic acid salt following Reductive Amination Procedure A from N-[3-[3-chloro-2-(4-formyl-3-methoxy-phenyl)-4-pyridyl]-2-methyl-phenyl]-5-formyl-pyridine-2-carboxamide (30 mg, 0.06 mmol) and tetrahydropyran-4-amine (25 mg, 0.25 mmol). 52% yield. LCMS: m/z found 656.3 [M+H]⁺, retention time = 2.57 min (Method A). ¹H NMR (400 MHz, Methanol-*d*₄): δ 8.72 (s, 1H), 8.64 (d, 1H), 8.51 (s, 2H), 8.22 (d, 1H), 8.10 - 8.02 (m, 1H), 7.97 (d, 1H), 7.52 (d, 1H), 7.46 - 7.37 (m, 3H), 7.37 - 7.31 (m, 1H), 7.12 (d, 1H), 4.27 (s, 2H), 4.10 - 4.02 (m, 2H), 4.02 - 3.92 (m, 7H), 3.54 - 3.35 (m, 5H), 2.91 - 2.76 (m, 1H), 2.17 (s, 3H), 2.10 (d, 2H), 1.94 (d, 2H), 1.80 - 1.58 (m, 2H), 1.58 - 1.41 (m, 2H).

### Example 631: N-(3-(3-Chloro-2-(3-methoxy-4-(((((S)-5-oxopyrrolidin-2-yl)methyl)amino)methyl)phenyl)pyridin-4-yl)-2-methylphenyl)-5-(((((S)-5-oxopyrrolidin-2-yl)methyl)amino)methyl)picolinamide (647)

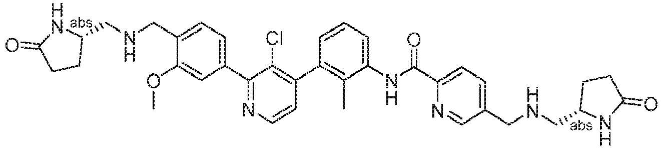

N-(3-(3-Chloro-2-(3-methoxy-4-(((((S)-5-oxopyrrolidin-2-yl)methyl)amino)methyl)phenyl)pyridin-4-yl)-2-methylphenyl)-5-(((((S)-5-oxopyrrolidin-2-yl)methyl)amino)methyl)picolinamide was prepared as the formic acid salt following Reductive Amination Procedure A from N-[3-[3-chloro-2-(4-formyl-3-methoxy-phenyl)-4-pyridyl]-2-methyl-phenyl]-5-formyl-pyridine-2-carboxamide (30 mg, 0.06 mmol) and (5S)-5-(aminomethyl)pyrrolidin-2-one (28 mg, 0.25 mmol). 66% yield. LCMS: m/z found 682.3 [M+H]⁺, retention time = 1.43 min (Method D). ¹H NMR (400 MHz, Methanol-*d*₄): δ 8.72 (d, 1H), 8.64 (d, 1H), 8.32 (s, 2H), 8.22 (d, 1H), 8.06 (dd, 1H), 7.97 (d, 1H), 7.53 (d, 1H), 7.47 - 7.32 (m, 4H), 7.12 (d, 1H), 4.39 - 4.22 (m, 2H), 4.09 - 3.94 (m, 6H), 3.91 - 3.78 (m, 1H), 3.24 - 3.08 (m, 2H), 2.91 - 2.67 (m, 2H), 2.48 - 2.21 (m, 6H), 2.17 (s, 3H), 1.98 - 1.75 (m, 2H).

### Example 632: N-(3-(3-Chloro-2-(4-(((2-hydroxyethyl)amino)methyl)-3-methoxyphenyl)pyridin-4-yl)-2-methylphenyl)-5-(((2-hydroxyethyl)amino)methyl)picolinamide (648)

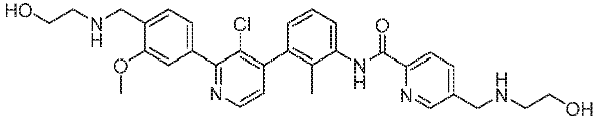

N-(3-(3-Chloro-2-(4-(((2-hydroxyethyl)amino)methyl)-3-methoxyphenyl)pyridin-4-yl)-2-methylphenyl)-5-(((2-hydroxyethyl)amino)methyl)picolinamide was prepared as the formic acid salt following Reductive Amination Procedure A from N-[3-[3-chloro-2-(4-formyl-3-methoxy-phenyl)-4-pyridyl]-2-methyl-phenyl]-5-formyl-pyridine-2-carboxamide (30 mg, 0.06 mmol) and 2-aminoethanol (15 mg, 0.25 mmol). 65% yield. LCMS: m/z found 576.2 [M+H]⁺, retention time = 2.27 min (Method A). ¹H NMR (400 MHz, Methanol-*d*₄): δ 8.77 (s, 1H), 8.64 (dd, 1H), 8.46 (s, 2H), 8.27 (d, 1H), 8.15 - 8.08 (m, 1H), 7.96 (d, 1H), 7.52 (d, 1H), 7.45 - 7.31 (m, 5H), 7.13 (d, 1H), 4.32 (s, 2H), 4.20 (s, 2H), 4.00 (s, 3H), 3.84 (t, 2H), 3.78 (t, 2H), 3.16 (t, 2H), 3.01 (t, 2H), 2.17 (s, 3H).

### Example 633: 5-((6-Acetyl-2,6-diazaspiro[3.3]heptan-2-yl)methyl)-N-(3-(2-(4-((6-acetyl-2,6-diazaspiro[3.3]heptan-2-yl)methyl)-3-methoxyphenyl)-3-chloropyridin-4-yl)-2-methylphenyl)picolinamide (649)

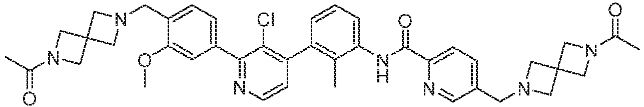

5-((6-Acetyl-2,6-diazaspiro[3.3]heptan-2-yl)methyl)-N-(3-(2-(4-((6-acetyl-2,6-diazaspiro[3.3]heptan-2-yl)methyl)-3-methoxyphenyl)-3-chloropyridin-4-yl)-2-methylphenyl)picolinamide was prepared as the formic acid salt following Reductive Amination Procedure A from N-[3-[3-chloro-2-(4-formyl-3-methoxy-phenyl)-4-pyridyl]-2-methyl-phenyl]-5-formyl-pyridine-2-carboxamide (30 mg, 0.06 mmol) and 1-(2,6-diazaspiro[3.3]heptan-2-yl)ethanone (35 mg, 0.25 mmol). 33% yield. LCMS: m/z found 734.3 [M+H]⁺, retention time = 2.42 min (Method A). ¹H NMR (400 MHz, Methanol-*d*₄): δ 8.74 - 8.52 (m, 2H), 8.33 (s, 2H), 8.21 (d, 1H), 7.97 (d, 2H), 7.50 (d, 1H), 7.47 - 7.30 (m, 4H), 7.12 (d, 1H), 4.38 (s, 4H), 4.31 (s, 2H), 4.26 (s, 4H), 4.14 (s, 2H), 4.06 (s, 2H), 3.99 (s, 3H), 3.81 (s, 2H), 3.56 - 3.50 (m, 4H), 2.17 (s, 3H), 1.85 (s, 6H).

### Example 634: N-(3-(3-Chloro-2-(4-((((S)-2-hydroxypropyl)amino)methyl)-3-methoxyphenyl)pyridin-4-yl)-2-methylphenyl)-5-((((S)-2-hydroxypropyl)amino)methyl)picolinamide (650)

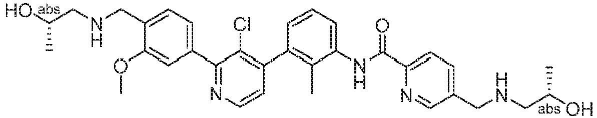

N-(3-(3-Chloro-2-(4-((((S)-2-hydroxypropyl)amino)methyl)-3-methoxyphenyl)pyridin-4-yl)-2-methylphenyl)-5-((((S)-2-hydroxypropyl)amino)methyl)picolinamide was prepared as the formic acid salt following Reductive Amination Procedure A from N-[3-[3-chloro-2-(4-formyl-3-methoxy-phenyl)-4-pyridyl]-2-methyl-phenyl]-5-formyl-pyridine-2-carboxamide (30 mg, 0.06 mmol) and (2S)-1-aminopropan-2-ol (19 mg, 0.25 mmol). 80% yield. LCMS: m/z found 604.3 [M+H]⁺, retention time = 1.51 min (Method D). ¹H NMR (400 MHz, Methanol-*d*₄): δ 8.77 (s, 1H), 8.64 (d, 1H), 8.47 (s, 2H), 8.27 (d, 1H), 8.12 (dd, 1H), 7.96 (d, 1H), 7.52 (d, 1H), 7.47 - 7.38 (m, 3H), 7.37 - 7.31 (m, 1H), 7.13 (d, 1H), 4.32 (s, 2H), 4.21 (s, 2H), 4.11 - 4.03 (m, 1H), 4.03 - 3.92 (m, 4H), 3.09 (dd, 1H), 2.97 - 2.82 (m, 2H), 2.77 (dd, 1H), 2.17 (s, 3H), 1.30 - 1.15 (m, 6H).

### Example 635: (S)-5-((((3'-Chloro-6-methoxy-2'-((S)-1-((6-methoxy-5-(((((S)-5-oxopyrrolidin-2-yl)methyl)amino)methyl)-3-(trifluoromethyl)pyridin-2-yl)amino)-2,3-dihydro-1H-inden-4-yl)-[2,4'-bipyridin]-5-yl)methyl)amino)methyl)pyrrolidin-2-one (223)

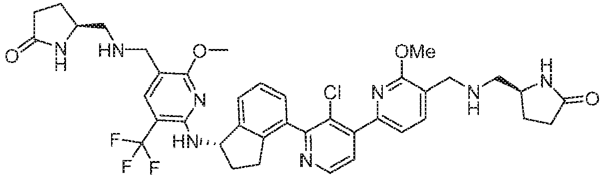

### (a) (S)-3'-Chloro-2'-(1-((5-formyl-6-methoxy-3-(trifluoromethyl)pyridin-2-yl)amino)-2,3-dihydro-1H-inden-4-yl)-6-methoxy-[2,4'-bipyridine]-5-carbaldehyde

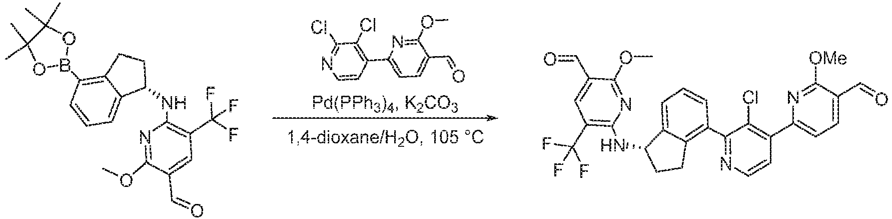

Tetrakis(triphenylphosphine)palladium(0) (16 mg, 0.01 mmol), potassium carbonate (29 mg, 0.21 mmol), 2-methoxy-6-[[(1S)-4-(4,4,5,5-tetramethyl-1,3,2-dioxaborolan-2-yl)indan-1-yl]amino]-5-(trifluoromethyl)pyridine-3-carbaldehyde (32 mg, 0.07 mmol), and 6-(2,3-dichloro-4-pyridyl)-2-methoxy-pyridine-3-carbaldehyde (29 mg, 0.10 mmol) were suspended in 1,4-dioxane /water (4:1, 2 mL), then the solution was heated at 105 °C for 20 minutes. Reaction was cooled to room temperature, diluted with 10 mL water, and extracted with EtOAc (3 x 5 mL). The combined organics were concentrated under reduced pressure and crude sample was purified by normal phase SiO₂ chromatography (0-3% MeOH/DCM) to afford (S)-3'-chloro-2'-(1-((5-formyl-6-methoxy-3-(trifluoromethyl)pyridin-2-yl)amino)-2,3-dihydro-1H-inden-4-yl)-6-methoxy-[2,4'-bipyridine]-5-carbaldehyde (30 mg, 74 % yield) as a tan foam. MS: *m*/*z* found 583, 585 [M+H]⁺.

### (b) (S)-5-((((3'-Chloro-6-methoxy-2'-((S)-1-((6-methoxy-5-(((((S)-5-oxopyrrolidin-2-yl)methyl)amino)methyl)-3-(trifluoromethyl)pyridin-2-yl)amino)-2,3-dihydro-1H-inden-4-yl)-[2,4'-bipyridin]-5-yl)methyl)amino)methyl)pyrrolidin-2-one

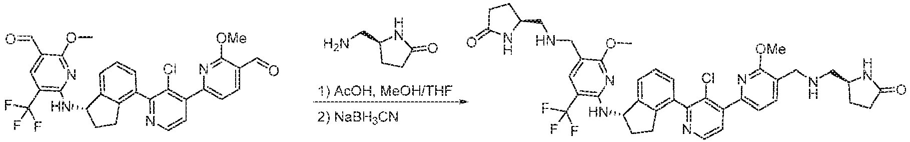

(S)-3'-Chloro-2'-(1-((5-formyl-6-methoxy-3-(trifluoromethyl)pyridin-2-yl)amino)-2,3-dihydro-1H-inden-4-yl)-6-methoxy-[2,4'-bipyridine]-5-carbaldehyde (10 mg, 0.02 mmol), acetic acid (2 mg, 0.03 mmol), and (5S)-5-(aminomethyl)pyrrolidin-2-one (8 mg, 0.07 mmol)(5S)-5-(aminomethyl)pyrrolidin-2-one (8 mg, 0.07 mmol) were dissolved in MeOH/THF (1:1, 1 mL), then the solution was stirred at 25 °C for 2 hours. Sodium cyanoborohydride (2 mg, 0.03 mmol) was added and the resulting mixture was stirred at 25 °C for 1 hour. Reaction was subsequently diluted with water (2 mL) and extracted with EtOAc (3 x 2 mL). The combined organics were further washed with brine, dried over sodium sulfate, filtered, and concentrated under reduced pressure. Crude sample was purified by reverse phase HPLC to afford (S)-5-((((3'-chloro-6-methoxy-2'-((S)-1-((6-methoxy-5-(((((S)-5-oxopyrrolidin-2-yl)methyl)amino)methyl)-3-(trifluoromethyl)pyridin-2-yl)amino)-2,3-dihydro-1H-inden-4-yl)-[2,4'-bipyridin]-5-yl)methyl)amino)methyl)pyrrolidin-2-one (9 mg, 65 % yield) as a white solid (formic acid salt). LC/MS: *m*/*z* found 779, 781 [M+H] ⁺, retention time = 2.22 min (Method C). ¹H NMR (400 MHz, Methanol-*d*₄) δ 8.66 - 8.57 (m, 1H), 8.39 (s, 2H), 7.86 (d, 1H), 7.81 (s, 1H), 7.69 (d, 1H), 7.43 (d, 1H), 7.33 (s, 3H), 6.30 (d, 1H), 5.89 (t, 1H), 4.06 (d, 5H), 3.99 (d, 3H), 3.94 (s, 4H), 3.06 (s, 2H), 2.87 (d, 4H), 2.62 (s, 1H), 2.35 (d, 6H), 2.17 - 2.02 (m, 1H), 1.86 (s, 2H).

### Example 636: (S)-N-(4-(3'-Chloro-6-methoxy-5-((methylamino)methyl)-[2,4'-bipyridin]-2'-yl)-2,3-dihydro-1H-inden-1-yl)-6-methoxy-5-((methylamino)methyl)-3-(trifluoromethyl)pyridin-2-amine (224)

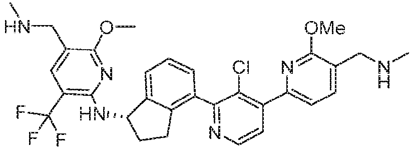

(S)-N-(4-(3'-Chloro-6-methoxy-5-((methylamino)methyl)-[2,4'-bipyridin]-2'-yl)-2,3-dihydro-1H-inden-1-yl)-6-methoxy-5-((methylamino)methyl)-3-(trifluoromethyl)pyridin-2-amine was prepared in the same manner as described for Example 635, utilizing intermediate (S)-3'-chloro-2'-(1-((5-formyl-6-methoxy-3-(trifluoromethyl)pyridin-2-yl)amino)-2,3-dihydro-1H-inden-4-yl)-6-methoxy-[2,4'-bipyridine]-5 -carbaldehyde and replacing (5S)-5-(aminomethyl)-pyrrolidin-2-one with methyl amine in step (b). Product was obtained as a white solid (formic acid salt). MS: *m*/*z* found 613, 615 [M+H]⁺, retention time = 2.27 min (Method C). ¹H NMR (400 MHz, Methanol-*d*₄) δ 8.68 - 8.59 (m, 1H), 8.51 (s, 2H), 7.92 (d, 1H), 7.81 (s, 1H), 7.72 - 7.63 (m, 1H), 7.48 (dd, 1H), 7.33 (d, 3H), 6.35 (d, 1H), 5.92 (q, 1H), 4.23 (s, 2H), 4.10 (t, 3H), 4.06 (s, 2H), 3.94 (d, 3H), 2.85 (t, 2H), 2.74 (d, 3H), 2.70 - 2.67 (m, 3H), 2.67 - 2.57 (m, 1H), 2.12 (q, 1H).

### Example 637: (1S,3r)-3-(((3'-Chloro-2'-((S)-1-((5-((((1r,3S)-3-hydroxy-3-methylcyclobutyl)-amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol (225)

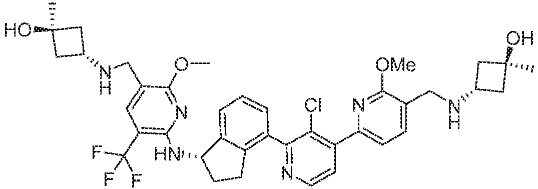

(1S,3r)-3-(((3'-Chloro-2'-((S)-1-((5-((((1r,3S)-3-hydroxy-3-methylcyclobutyl)-amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol was prepared in the same manner as described for Example 635, utilizing intermediate (S)-3'-chloro-2'-(1-((5-formyl-6-methoxy-3-(trifluoromethyl)-pyridin-2-yl)amino)-2,3-dihydro-1H-inden-4-yl)-6-methoxy-[2,4'-bipyridine]-5-carbaldehyde and replacing (5S)-5-(aminomethyl)-pyrrolidin-2-one with 3-amino-1-methyl-cyclobutanol in step (b). Product was obtained as a white solid (formic acid salt). LC/MS: *m*/*z* found 753, 755 [M+H] ⁺, retention time = 2.25 min (Method C). ¹H NMR (400 MHz, Methanol-*d*₄) δ 8.62 (d, 1H), 8.51 (s, 2H), 7.85 (d, 1H), 7.78 (d, 1H), 7.69 (s, 1H), 7.43 (d, 1H), 7.32 (s, 3H), 6.31 (d, 1H), 5.90 (d, 1H), 4.07 (d, 3H), 3.94 (s, 7H), 3.83 (t, 1H), 3.71 (s, 1H), 2.85 (s, 2H), 2.63 (s, 1H), 2.34 (d, 4H), 2.13 (t, 3H), 2.07 - 1.99 (m, 2H), 1.39 - 1.34 (m, 6H).

### Example 638: (S)-2-((3'-chloro-6-methoxy-2'-(1-((6-methoxy-5-((7-oxo-2,6-diazaspiro [3.4] octan-2-yl)methyl)-3-(trifluoromethyl)pyrazin-2-yl)amino)-2,3-dihydro-1H-inden-4-yl)-[2,4'-bipyridin]-5-yl)methyl)-2,6-diazaspiro[3.4]octan-7-one (304)

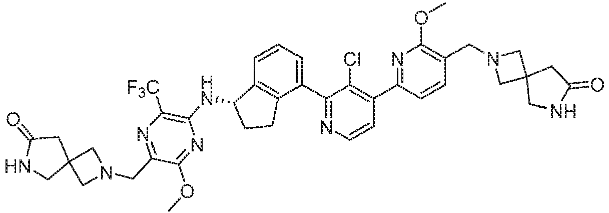

### (a) (S)-3'-Chloro-2'-(1-((5-formyl-6-methoxy-3-(trifluoromethyl)pyrazin-2-yl)amino)-2,3-dihydro-1H-inden-4-yl)-6-methoxy-[2,4'-bipyridine]-5-carbaldehyde

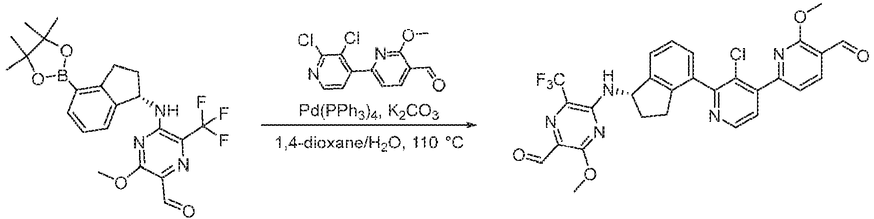

A mixture of (S)-3-methoxy-5-((4-(4,4,5,5-tetramethyl-1,3,2-dioxaborolan-2-yl)-2,3-dihydro-1H-inden-1-yl)amino)-6-(trifluoromethyl)pyrazine-2-carbaldehyde (Example 644, step (a)) (0.5 g, 1.08 mmol), 6-(2,3-dichloro-4-pyridyl)-2-methoxy-pyridine-3-carbaldehyde (183 mg, 648 µmol), potassium carbonate (298 mg, 2.16 mmol) and tetrakis(triphenylphosphine) palladium (125 mg, 108 µmol) in dioxane/water (10:1, 11 mL) was stirred at 110°C for 2 h. The reaction mixture was diluted with water (25 mL) and extracted with ethyl acetate (3 x 25 mL). The combined organic layers were washed with brine (2 x 20 mL), dried over anhydrous sodium sulfate, filtered and concentrated. The residue was purified by normal phase SiO₂ chromatography (10-50% ethyl acetate/petroleum ether) to afford (S)-3'-chloro-2'-(1-((5-formyl-6-methoxy-3-(trifluoromethyl)pyrazin-2-yl)amino)-2,3-dihydro-1H-inden-4-yl)-6-methoxy-[2,4'-bipyridine]-5-carbaldehyde as yellow oil (0.4 g). MS: m/z found 584.1 [M+H]⁺.

### (b) (S)-2-((3'-Chloro-6-methoxy-2'-(1-((6-methoxy-5-((7-oxo-2,6-diazaspiro [3.4] octan-2-yl)methyl)-3-(trifluoromethyl)pyrazin-2-yl)amino)-2,3-dihydro-1H-inden-4-yl)-[2,4'-bipyridin]-5-yl)methyl)-2,6-diazaspiro[3.4]octan-7-one

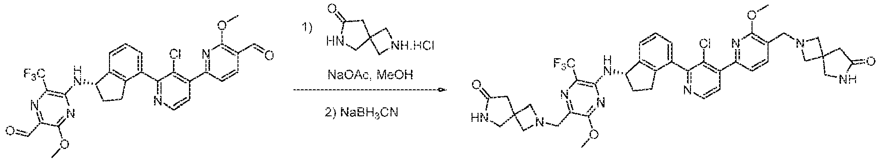

To a solution of (S)-3'-chloro-2'-(1-((5-formyl-6-methoxy-3-(trifluoromethyl)pyrazin-2-yl)amino)-2,3-dihydro-1H-inden-4-yl)-6-methoxy-[2,4'-bipyridine]-5-carbaldehyde (150 mg, 257 µmol) and 2,6-diazaspiro[3.4]octan-7-one hydrochloride salt (84 mg, 514 µmol) in methanol (5 mL) was added sodium acetate (84 mg, 1 mmol). The solution was stirred at room temperature for 12 h. Sodium cyanoborohydride (65 mg, 1 mmol) was added and the mixture was stirred at room temperature for 1 h. The reaction mixture was concentrated and the residue was purified by reverse phase HPLC to afford (S)-2-((3'-chloro-6-methoxy-2'-(1-((6-methoxy-5-((7-oxo-2,6-diazaspiro[3.4]o ctan-2-yl)methyl)-3-(trifluoromethyl)pyrazin-2-yl)amino)-2,3-dihydro-1H-inden-4-yl) -[2,4'-bipyridin]-5-yl)methyl)-2,6-diazaspiro[3.4]octan-7-one (35 mg). MS: m/z found 804.3 [M+H]⁺, retention time = 2.78 min (Method A). ¹H NMR (400MHz, Methanol-*d*₄): δ = 8.62 (d, 1H), 8.37 (br s, 1H), 7.80 (d, 1H), 7.69 (d, 1H), 7.42 (d, 1H), 7.34 - 7.33 (m, 3H), 5.84 (t, 1H), 4.19 - 4.15 (m, 2H), 4.04 - 4.01 (m, 7H), 3.94 - 3.90 (m, 5H), 3.65 - 3.62 (m, 8H), 288-2.84 (m, 2H), 2.68 (s, 2H), 2.62 (s, 3H), 2.19 - 2.09 (m, 1H).

### Example 639: (S)-1-(6-((5-((4-(5-((6-Acetyl-2,6-diazaspiro[3.3]heptan-2-yl)methyl)-3'-chloro-6-methoxy-[2,4'-bipyridin]-2'-yl)-2,3-dihydro-1H-inden-1-yl)amino)-3-methoxy-6-(trifluoromethyl)pyrazin-2-yl)methyl)-2,6-diazaspiro[3.3]heptan-2-yl)ethan-1-one (305)

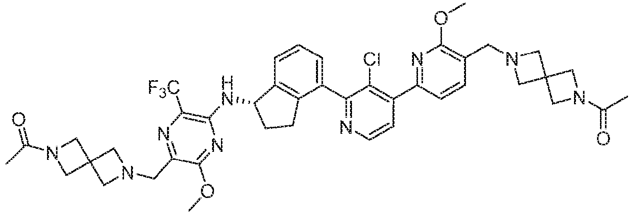

To a solution of (S)-3'-chloro-2'-(1-((5-formyl-6-methoxy-3-(trifluoromethyl)pyrazin-2-yl)amino)-2,3-dihydro-1H-inden-4-yl)-6-methoxy-[2,4'-bipyridine]-5-carbaldehyde (Example 638, step (a)) (150 mg, 257 µmol) and 1-(2,6-diazaspiro[3.3]heptan-2-yl)ethanone as formic acid salt (131 mg, 514 µmol) in methanol (1.5 mL) was added sodium acetate (84 mg, 1 mmol). The solution was stirred at room temperature for 12 h. Sodium cyanoborohydride (65 mg, 1 mmol) was added and the mixture was stirred at room temperature for 1 h. The reaction mixture was concentrated and the residue was purified by reverse phase HPLC to afford (S)-1-(6-((5-((4-(5-((6-acetyl-2,6-diazaspiro[3.3]heptan-2-yl)methyl)-3'-chloro-6-methoxy-[2,4'-bipyridin]-2'-yl)-2,3-dihydro-1H-inden-1-yl)amino)-3-methoxy-6-(trifluoromethyl)pyrazin-2-yl)methyl)-2,6-diazaspiro[3.3]heptan-2-yl)ethan-1-one (41 mg). MS: m/z found 832.3 [M+H]⁺, retention time = 2.89 min (Method A). ¹H NMR (400MHz, Methanol-*d*₄): δ 8.62 (d, 1H), 8.37 (s, 1H), 7.79 (d, 1H), 7.69 (d, 1H), 7.42 (d, 1H), 7.37 - 7.33 (m, 3H), 5.84 (t, 1H), 4.35 (d, 4H), 4.19 - 4.09 (m, 10H), 4.04 (s, 3H), 3.94 (s, 3H), 3.89 (s, 2H), 3.75 (s, 4H), 2.88-2.84 (m, 2H), 2.65 - 2.59 (m, 1H), 2.19 - 2.09 (m, 1H), 1.85 (s, 6H).

### Example 640: (S)-5-((((6-(((S)-4-(5-(((1-Acetylpiperidin-4-yl)amino)methyl)-3'-chloro-6-methoxy-[2,4'-bipyridin]-2'-yl)-2,3-dihydro-1H-inden-1-yl)amino)-2-methoxy-5-(trifluoromethyl)pyridin-3-yl)methyl)amino)methyl)pyrrolidin-2-one (486)

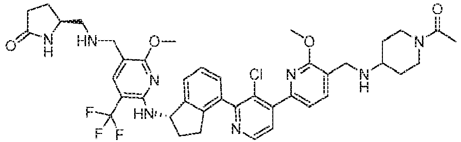

### (a) 2',3'-Dichloro-6-methoxy-[2,4'-bipyridine]-5-carbaldehyde

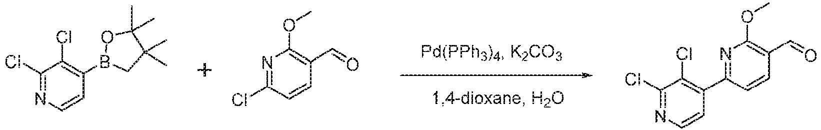

A mixture of 2,3-dichloro-4-(4,4,5,5-tetramethyl-1,2-oxaborolan-2-yl)pyridine (22.75 g, 83 mmol) , 6-chloro-2-methoxy-pyridine-3-carbaldehyde (15 g, 87 mmol), tetrakis(triphenylphosphine)palladium(0) (5.05 g, 4.37 mmol), and potassium carbonate (36.25 g, 262 mmol) in 1,4-dioxane (375 mL) and water (20 mL) was degassed and purged with N₂. The mixture was stirred at 95 °C for 4 h. The mixture was concentrated followed by addition of water (300 mL). The aqueous layer was extracted with EtOAc (2 x 400 mL), dried over with sodium sulfate, filtered and concentrated. The solid was washed with EtOAc (200mL) and dried to afford 2',3'-dichloro-6-methoxy-[2,4'-bipyridine]-5-carbaldehyde (10 g, 40% yield).

### (b) 1-(4-(((2',3'-Dichloro-6-methoxy-[2,4'-bipyridin]-5-yl)methyl)amino)piperidin-1-yl)ethan-1-one

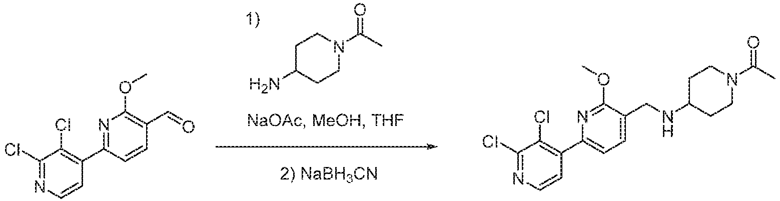

To a mixture of 2',3'-dichloro-6-methoxy-[2,4'-bipyridine]-5-carbaldehyde (1.5 g, 5.3 mmol) and 1-(4-amino-1-piperidyl)ethanone (1.13 g, 7.95 mmol) in THF/MeOH mixture (1:1, 30 mL) was added sodium acetate (0.87 g, 10.6 mmol). The mixture was stirred at room temperature for 1 h. Sodium cyanoborohydride (1 g, 15.9 mmol) was added and the mixture was stirred at room temperature for 12 h. The mixture was used for next step without work-up. 1-(4-(((2',3'-dichloro-6-methoxy-[2,4'-bipyridin]-5-yl)methyl)amino)piperidin-1-yl)ethan-1-one. MS: m/z found 409 [M+Na]⁺.

### (c) tert-Butyl (1-acetylpiperidin-4-yl)((2',3'-dichloro-6-methoxy-[2,4'-bipyridin]-5-yl)methyl)carbamate

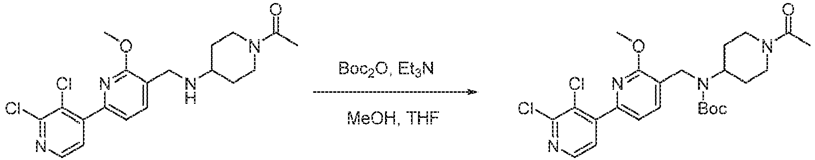

To a solution of 1-(4-(((2',3'-dichloro-6-methoxy-[2,4'-bipyridin]-5-yl)methyl)amino)piperidin-1-yl)ethan-1-one in THF/MeOH was added di-tert-butyl dicarbonate (3 mL, 13 mmol) and triethylamine (0.74 mL, 5.3 mmol). The mixture was stirred at room temperature for 5 h. The mixture was concentrated, and the residue was diluted with water (20 mL) and brine (30 mL). The mixture was extracted with ethyl acetate/THF mixture (3:1, 50 mL). The organic phase was dried with anhydrous sodium sulfate, filtered and concentrated. The residue was purified by normal phase SiO₂ chromatography (0-15% ethyl acetate/petroleum ether) to afford tert-butyl (1-acetylpiperidin-4-yl)((2',3'-dichloro-6-methoxy-[2,4'-bipyridin]-5-yl)methyl)carbamate (2.3 g, 68%). MS: m/z found 531 [M+Na]⁺.

### (d) (S)-6-((4-Bromo-2,3-dihydro-1H-inden-1-yl)amino)-2-methoxynicotinonitrile

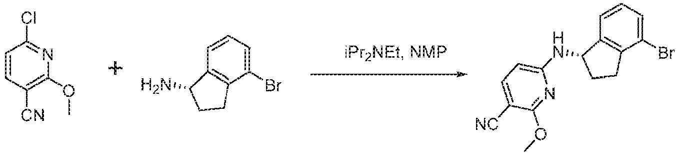

To a mixture of 6-chloro-2-methoxynicotinonitrile (4.5 g, 26.7 mmol) and (S)-4-bromo-2,3-dihydro-1H-inden-1-amine as a hydrochloride salt (7.3 g, 29.4 mmol) in NMP (80 mL) was added N,N-diisopropylethylamine (9.3 mL, 53.4 mmol) in one portion under N₂ atmosphere. The mixture was stirred at 100 °C for 6 h. To the mixture was added water (50 mL) and brine (50 mL). The mixture was extracted with ethyl acetate (40 mL). The combined organic phases were washed with brine (2 x 100 mL), dried with anhydrous sodium sulfate, filtered and concentrated. The residue was purified by normal phase SiO₂ chromatography (0-15% ethyl acetate/petroleum ether) to afford (S)-6-((4-bromo-2,3-dihydro-1H-inden-1-yl)amino)-2-methoxynicotinonitrile as a brown solid (3 g, 32% yield). ¹H NMR (400 MHz, Chloroform-d): δ 7.46 (d, 1H), 7.37 (d, 1H), 7.18 (d, 1H), 7.03 (t, 1H), 5.96 (d, 1H), 5.50 (br s, 1H), 5.04 (d, 1H), 3.87 (s, 3H), 3.01-2.97 (m, 1H), 2.87-2.83 (m, 1H), 2.61-2.58 (m, 1H), 1.91-1.85 (m, 1H).

### (e) (S)-6-((4-Bromo-2,3-dihydro-1H-inden-1-yl)amino)-5-iodo-2-methoxynicotinonitrile

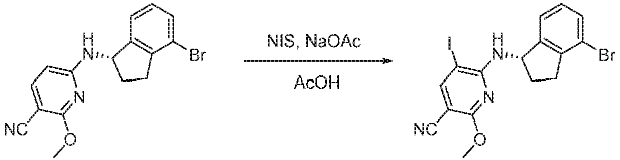

To a mixture of (S)-6-((4-bromo-2,3-dihydro-1H-inden-1-yl)amino)-2-methoxynicotinonitrile (3 g, 8.72 mmol) in acetic acid (50 mL) was added N-iodosuccinimide (2.16 g, 9.59 mmol) and sodium acetate (8.55 g, 87.2 mmol). The mixture was stirred at room temperature for 2 h. The mixture was neutralized with saturated aqueous NaOH solution (800 mL). The mixture was extracted with ethyl acetate (2 x 100 mL). The combined organic phases were washed with brine (2 x 100 mL), dried with anhydrous sodium sulfate, filtered and concentrated. The residue was purified directly by normal phase SiO₂ chromatography (0-7% ethyl acetate/petroleum ether) to afford (S)-6-((4-bromo-2,3-dihydro-1H-inden-1-yl)amino)-5-iodo-2-methoxynicotinonitrile (3.2 g, 78% yield). ¹H NMR (400 MHz, Chloroform-d): δ 7.81 (s, 1H), 7.38 (d, 1H), 7.16 (d, 1H), 7.05 (t, 1H), 5.67 (q, 1H), 5.54 (d, 1H), 3.89 (s, 3H), 3.03-3.00 (m, 1H), 2.88-2.84 (m, 1H), 2.66-2.64 (m, 1H), 1.95-1.88 (m, 1H).

### (f) (S)-6-((4-Bromo-2,3-dihydro-1H-inden-1-yl)amino)-2-methoxy-5-(trifluoromethyl)nicotinonitrile

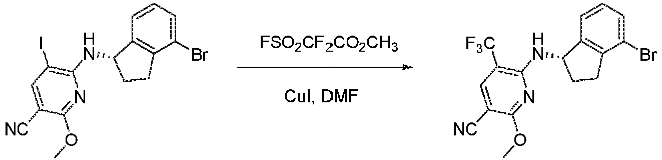

To a mixture of (S)-6-((4-bromo-2,3-dihydro-1H-inden-1-yl)amino)-5-iodo-2-methoxynicotinonitrile (3.2 g, 6.8 mmol) and methyl 2,2-difluoro-2-(fluorosulfonyl)acetate (1.73 mL, 13.6 mmol) in DMF (30 mL) was added copper(I) iodide (1.94 g, 10.2 mmol). The mixture was stirred at 110 °C for 2 h. The mixture was filtered then water (100 mL) and brine (100 mL) added to the filtrate. The mixture was extracted with ethyl acetate (2 x 100 mL). The combined organic phases were washed with brine (2 x 50 mL), dried with anhydrous sodium sulfate, filtered and concentrated. The residue was purified directly by normal phase SiO₂ chromatography (0-10% ethyl acetate/petroleum ether) to afford (S)-6-((4-bromo-2,3-dihydro-1H-inden-1-yl)amino)-2-methoxy-5-(trifluoromethyl)nicotinonitrile (2.7 g, 96%). ¹H NMR (400 MHz, Chloroform-d) δ 7.77 (s, 1H), 7.39 (d, 1H), 7.14 (d, 1H), 7.06 (t, 1H), 5.78 (q, 1H), 5.50 (d, 1H), 3.93 (s, 3H), 3.03-3.00 (m, 1H), 2.89-2.84 (m, 1H), 2.68-2.65 (m, 1H), 1.93-1.88 (m, 1H).

### (g) (S)-6-((4-Bromo-2,3-dihydro-1H-inden-1-yl)amino)-2-methoxy-5-(trifluoromethyl)nicotinaldehyde

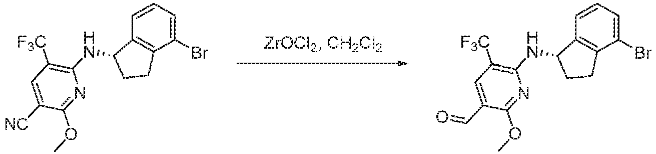

To a mixture of (S)-6-((4-bromo-2,3-dihydro-1H-inden-1-yl)amino)-2-methoxy-5-(trifluoromethyl)nicotinonitrile (2.4 g, 5.82 mmol) in dichloromethane (80 mL) was added zirconium dichloride oxide hydrate (metals basis) (2.64 g, 9.9 mmol) at 0 °C under N₂ atmosphere. The mixture was stirred at 0 °C for 20 min. The mixture was purified by normal phase SiO₂ chromatography (0-10% ethyl acetate/petroleum ether) to afford (S)-6-((4-bromo-2,3-dihydro-1H-inden-1-yl)amino)-2-methoxy-5-(trifluoromethyl)nicotinaldehyde (1.2 g, 49%). ¹H NMR (400 MHz, Chloroform-*d*): δ 10.03 (s, 1H), 8.14 (s, 1H), 7.39 (d, 1H), 7.16 (d, 1H), 7.06 (t, 1H), 5.87-5.79 (m, 1H), 5.58 (d, 1H), 3.96 (s, 3H), 3.05-3.01 (m, 1H), 2.89-2.85 (m, 1H), 2.70-2.68 (m, 1H), 1.95-1.52 (m, 1H).

### (h) (S)-2-Methoxy-6-((4-(4,4,5,5-tetramethyl-1,3,2-dioxaborolan-2-yl)-2,3-dihydro-1H-inden-1-yl)amino)-5-(trifluoromethyl)nicotinaldehyde

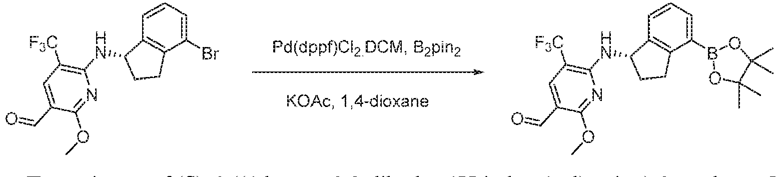

To a mixture of (S)-6-((4-bromo-2,3-dihydro-1H-inden-1-yl)amino)-2-methoxy-5-(trifluoromethyl)nicotinaldehyde (0.3 g, 0.72 mmol) and bis(pinacolato)diborane (0.55 g, 2.17 mmol) in 1,4-dioxane (5 mL) was added potassium acetate (0.21 g, 2.17 mmol) and [1,1'- bis(diphenylphosphino)ferrocene]dichloropalladium(II) complex with dichloromethane (0.03 g, 0.04 mmol). The mixture was stirred at 120 °C for 24 h. The mixture was concentrated, and the residue was purified by normal phase SiO₂ chromatography (0-15% ethyl acetate/petroleum ether) to afford (S)-2-methoxy-6-((4-(4,4,5,5-tetramethyl-1,3,2-dioxaborolan-2-yl)-2,3-dihydro-1H-inden-1-yl)amino)-5-(trifluoromethyl)nicotinaldehyde (0.54 g, crude). ¹H NMR (400 MHz, Chloroform*-d*) δ 10.03 (s, 1H), 8.13 (s, 1H), 7.69 (d, 1H), 7.31 (d, 1H), 7.19-7.18 (m, 2H), 5.75 (q, 1H), 5.57-5.56 (m, 1H), 3.97 (s, 3H), 3.29-3.27 (m, 1H), 3.03-2.99 (m, 1H), 2.67-2.63 (m, 1H), 1.88-1.83 (m, 1H), 1.28 (s, 12H).

### (i) tert-Butyl (S)-(1-acetylpiperidin-4-yl)((3'-chloro-2'-(1-((5-formyl-6-methoxy-3-(trifluoromethyl)pyridin-2-yl)amino)-2,3-dihydro-1H-inden-4-yl)-6-methoxy-[2,4'-bipyridin]-5-yl)methyl)carbamate

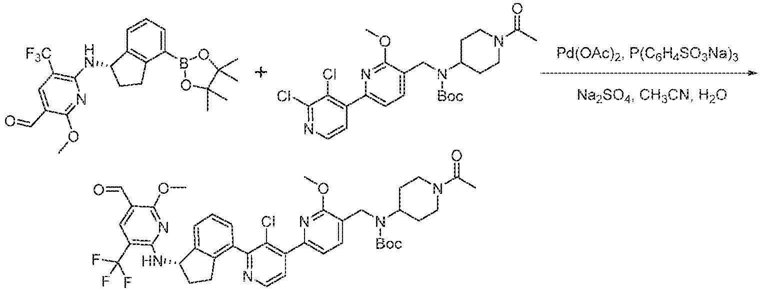

A mixture of palladium diacetate (10 mg, 0.04 mmol) and triphenylphosphine-3,3',3"-trisulfonic acid trisodium salt (0.1 g, 0.17 mmol) in MeCN/H₂O mixture (1:2, 2.7 mL) was kept under ultrasonic cleaner for 5 min under N₂. To the above mixture was added (S)-2-methoxy-6-((4-(4,4,5,5-tetramethyl-1,3,2-dioxaborolan-2-yl)-2,3-dihydro-1H-inden-1-yl)amino)-5-(trifluoromethyl)nicotinaldehyde (0.1 g, 0.22 mmol), tert-butyl (1-acetylpiperidin-4-yl)((2',3'-dichloro-6-methoxy-[2,4'-bipyridin]-5-yl)methyl)carbamate (0.1 g, 0.19 mmol) and sodium sulfate (0.07 g, 0.65 mmol) in MeCN/H₂O mixture (1:2, 6 mL) in one portion. The mixture was stirred at 100 °C for 1 h under N₂. The mixture was combined with another batch at 30 mg scale. To the mixture was added water (5 mL) and brine (10 mL). The mixture was extracted with ethyl acetate/THF mixture (1:1, 20 mL). The organic phase was dried with anhydrous sodium sulfate, filtered and concentrated. The residue was purified by prep-TLC (silica gel, petroleum ether : ethyl acetate = 0:1 to afford tert-butyl (S)-(1-acetylpiperidin-4-yl)((3'-chloro-2'-(1-((5-formyl-6-methoxy-3-(trifluoromethyl)pyridin-2-yl)amino)-2,3-dihydro-1H-inden-4-yl)-6-methoxy-[2,4'-bipyridin]-5-yl)methyl)carbamate (0.1 g, 50%). MS: m/z found 809 [M+H]⁺.

### (j) tert-Butyl (1-acetylpiperidin-4-yl)((3'-chloro-6-methoxy-2'-((S)-1-((6-methoxy-5-(((((S)-5-oxopyrrolidin-2-yl)methyl)amino)methyl)-3-(trifluoromethyl)pyridin-2-yl)amino)-2,3-dihydro-1H-inden-4-yl)-[2,4'-bipyridin]-5-yl)methyl)carbamate

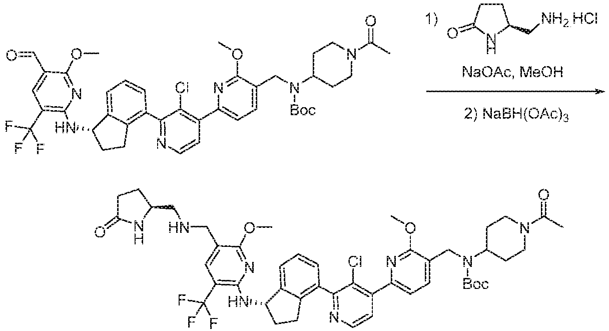

To a mixture of tert-butyl (S)-(1-acetylpiperidin-4-yl)((3'-chloro-2'-(1-((5-formyl-6-methoxy-3-(trifluoromethyl)pyridin-2-yl)amino)-2,3-dihydro-1H-inden-4-yl)-6-methoxy-[2,4'-bipyridin]-5-yl)methyl)carbamate (95 mg, 0.12 mmol) and (S)-5-(aminomethyl)-2-pyrrolidinone hydrochloride salt (26 mg, 0.18 mmol) in methanol (4 mL) was added sodium acetate (29 mg, 0.35 mmol). The mixture was stirred at room temperature for 12 h. Sodium triacetoxyborohydride (74 mg, 0.35 mmol) was added and the mixture was stirred at room temperature for 3 h. The mixture was concentrated to afford crude tert-butyl (1-acetylpiperidin-4-yl)((3'-chloro-6-methoxy-2'-((S)-1-((6-methoxy-5-(((((S)-5-oxopyrrolidin-2-yl)methyl)amino)methyl)-3-(trifluoromethyl)pyridin-2-yl)amino)-2,3-dihydro-1H-inden-4-yl)-[2,4'-bipyridin]-5-yl)methyl)carbamate (0.16 g, crude). MS: m/z found 907 [M+H]⁺.

### (k) (S)-5-((((6-(((S)-4-(5-(((1-Acetylpiperidin-4-yl)amino)methyl)-3'-chloro-6-methoxy-[2,4'-bipyridin]-2'-yl)-2,3-dihydro-1H-inden-1-yl)amino)-2-methoxy-5-(trifluoromethyl)pyridin-3-yl)methyl)amino)methyl)pyrrolidin-2-one

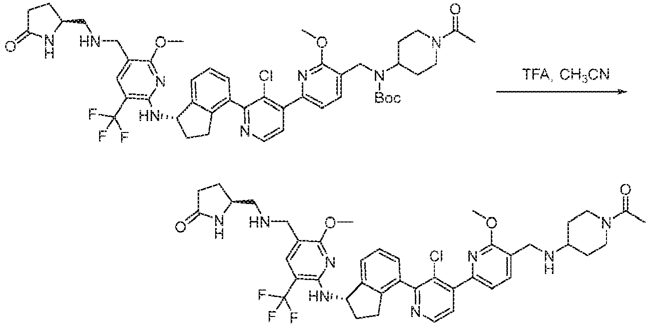

To a mixture of tert-butyl (1-acetylpiperidin-4-yl)((3'-chloro-6-methoxy-2'-((S)-1-((6-methoxy-5-(((((S)-5-oxopyrrolidin-2-yl)methyl)amino)methyl)-3-(trifluoromethyl)pyridin-2-yl)amino)-2,3-dihydro-1H-inden-4-yl)-[2,4'-bipyridin]-5-yl)methyl)carbamate (0.15 g, 0.16 mmol) in acetonitrile (0.2 mL) was added trifluoroacetic acid (3 mL, 41 mmol). The mixture was stirred at room temperature for 2 h. The mixture was concentrated and the residue was purified by reverse phase HPLC to afford (S)-5-((((6-(((S)-4-(5-(((1-acetylpiperidin-4-yl)amino)methyl)-3'-chloro-6-methoxy-[2,4'-bipyridin]-2'-yl)-2,3-dihydro-1H-inden-1-yl)amino)-2-methoxy-5-(trifluoromethyl)pyridin-3-yl)methyl)amino)methyl)pyrrolidin-2-one (3.5 mg, 2%). MS: m/z found 807 [M+H]⁺, retention time = 3.26 min (Method A). ¹H NMR (400 MHz, Methanol-*d*₄): δ 8.61 (d, 1H), 7.81 (d, 1H), 7.69 (d, 1H), 7.66 (s, 1H), 7.40 (d, 1H), 7.36-7.30 (m, 3H), 5.89-5.80 (m, 2H), 4.49-4.44 (m, 1H), 4.04 (s, 3H), 3.94-3.87 (m, 6H), 3.83-3.79 (m, 1H), 3.70-3.66 (m, 2H), 3.17-3.13 (m, 1H), 2.85-2.63 (m, 7H), 2.34-2.27 (m, 3H), 2.10-2.02 (m, 6H), 1.83-1.73 (m, 1H), 1.40-1.27 (m, 2H).

### Example 641: (S)-5-((((2'-((S)-1-((5-(((1-Acetylpiperidin-4-yl)amino)methyl)-6-methoxy-3-(trifluoromethyl)pyridin-2-yl)amino)-2,3-dihydro-1H-inden-4-yl)-3'-chloro-6-methoxy-[2,4'-bipyridin]-5-yl)methyl)amino)methyl)pyrrolidin-2-one (633)

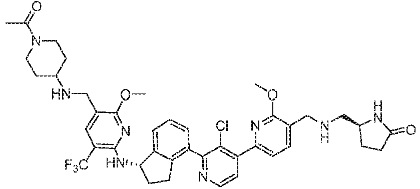

### (a) tert-Butyl (S)-((2',3'-dichloro-6-methoxy-[2,4'-bipyridin]-5-yl)methyl)((5-oxopyrrolidin-2-yl)methyl)carbamate

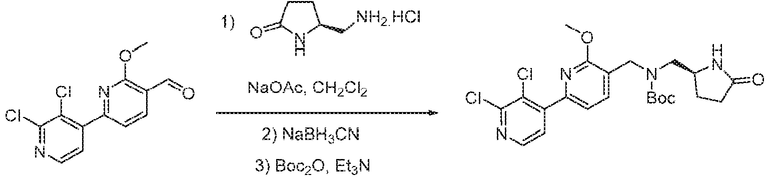

To a mixture of 2',3'-dichloro-6-methoxy-[2,4'-bipyridine]-5-carbaldehyde (Example 640, step (a)) (5.0 g, 5.3 mmol) and (5S)-5-(aminomethyl)pyrrolidin-2-one hydrochloride (1.6 g, 10.6 mmol) in dichloromethane (15 mL) was added sodium acetate (1.74 g, 21.2 mmol). The mixture was stirred at room temperature for 1.5 h under N₂. Sodium cyanoborohydride (0.67 g, 10.6 mmol) was added and the mixture was stirred at room temperature for 1.5 h under N₂. To the mixture was added di-tert-butyl dicarbonate (3.66 g, 16.8 mmol) and triethylamine (1.33 g, 13.11 mmol). The mixture was stirred at room temperature for 1 h under N₂. The mixture was concentrated, and the residue was purified by normal phase SiO₂ chromatography (0-100 % EtOAc/petroleum ether) followed by reverse phase HPLC to afford tert-butyl (S)-((2',3'-dichloro-6-methoxy-[2,4'-bipyridin]-5-yl)methyl)((5-oxopyrrolidin-2-yl)methyl)carbamate (500 mg, 62% yield). ¹H NMR (400 MHz, DMSO-d₆): δ 8.54 (d, 1H), 7.83 (s, 1H), 7.75 (d, 1H), 7.62-7.47 (m, 2H), 4.54-4.41 (m, 2H), 4.00 (s, 3H), 3.83-3.82 (m, 1H), 3.36-3.33 (m, 2H), 2.24-2.12 (m, 3H), 1.79-1.78 (m, 1H), 1.49-1.36 (m, 9H).

### (b) tert-Butyl ((3'-chloro-2'-((S)-1-((5-formyl-6-methoxy-3-(trifluoromethyl)pyridin-2-yl)amino)-2,3-dihydro-1H-inden-4-yl)-6-methoxy-[2,4'-bipyridin]-5-yl)methyl)(((S)-5-oxopyrrolidin-2-yl)methyl)carbamate

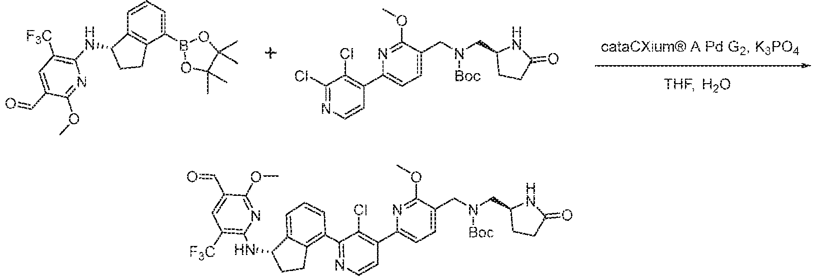

To a mixture of (S)-2-methoxy-6-((4-(4,4,5,5-tetramethyl-1,3,2-dioxaborolan-2-yl)-2,3-dihydro-1H-inden-1-yl)amino)-5-(trifluoromethyl)nicotinaldehyde (Example 640, step (h)) (0.49 g, 1.06 mmol) and tert-butyl (S)-((2',3'-dichloro-6-methoxy-[2,4'-bipyridin]-5-yl)methyl)((5-oxopyrrolidin-2-yl)methyl)carbamate (0.3 g, 0.62 mmol) in THF/water (20:3, 23 mL) was added potassium phosphate (0.4 g, 1.87 mmol) and chloro[(di(1-adamantyl)-N-butylphosphine)-2-(2-aminobiphenyl)]palladium(II) (0.04 g, 0.06 mmol). The mixture was stirred at 80 °C for 5 h. The mixture was concentrated, and the residue was diluted with water (50 mL) and brine (50 mL). The mixture was extracted with ethyl acetate/THF mixture (1:1, 100 mL). The organic phase was dried with anhydrous sodium sulfate, filtered and concentrated. The residue was purified by normal phase SiO₂ chromatography (0-100% ethyl acetate/petroleum ether) to afford tert-butyl ((3'-chloro-2'-((S)-1-((5-formyl-6-methoxy-3-(trifluoromethyl)pyridin-2-yl)amino)-2,3-dihydro-1H-inden-4-yl)-6-methoxy-[2,4'-bipyridin]-5-yl)methyl)(((S)-5-oxopyrrolidin-2-yl)methyl)carbamate as a yellow solid (0.8 g, 40%). MS: m/z found 781 [M+H]⁺.

### (c) tert-Butyl ((2'-((S)-1-((5-(((1-acetylpiperidin-4-yl)amino)methyl)-6-methoxy-3-(trifluoromethyl)pyridin-2-yl)amino)-2,3-dihydro-1H-inden-4-yl)-3'-chloro-6-methoxy-[2,4'-bipyridin]-5-yl)methyl)(((S)-5-oxopyrrolidin-2-yl)methyl)carbamate

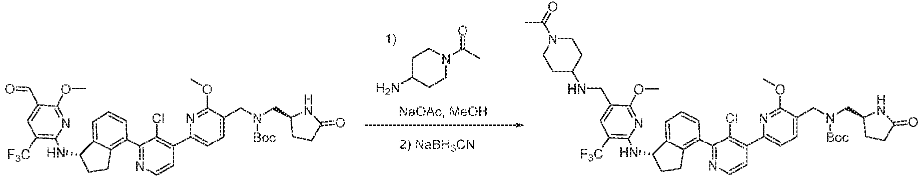

To a mixture of tert-butyl ((3'-chloro-2'-((S)-1-((5-formyl-6-methoxy-3-(trifluoromethyl)pyridin-2-yl)amino)-2,3-dihydro-1H-inden-4-yl)-6-methoxy-[2,4'-bipyridin]-5-yl)methyl)(((S)-5-oxopyrrolidin-2-yl)methyl)carbamate (0.38 g, 0.49 mmol) and 1-(4-aminopiperidin-1-yl)ethanone (0.08 g, 0.54 mmol) in methanol (6 mL) was added sodium acetate (0.12 g, 1.46 mmo). The mixture was stirred at room temperature for 2 h. Sodium cyanoborohydride (0.09 g, 1.46 mmol) was added and the mixture was stirred at room temperature for 1 h. The mixture was concentrated and the residue was purified by normal phase SiO₂ chromatography (50-100% ethyl acetate/petroleum ether to 20% MeOH/ethyl acetate) and further purified by prep-TLC (silica gel, ethyl acetate: methanol = 1:1.5) to afford tert-butyl ((2'-((S)-1-((5-(((1-acetylpiperidin-4-yl)amino)methyl)-6-methoxy-3-(trifluoromethyl)pyridin-2-yl)amino)-2,3-dihydro-1H-inden-4-yl)-3'-chloro-6-methoxy-[2,4'-bipyridin]-5-yl)methyl)(((S)-5-oxopyrrolidin-2-yl)methyl)carbamate (75 mg, 13%). MS: m/z found 907 [M+H]⁺.

### (d) (S)-5-((((2'-((S)-1-((5-(((1-Acetylpiperidin-4-yl)amino)methyl)-6-methoxy-3-(trifluoromethyl)pyridin-2-yl)amino)-2,3-dihydro-1H-inden-4-yl)-3'-chloro-6-methoxy-[2,4'-bipyridin]-5-yl)methyl)amino)methyl)pyrrolidin-2-one

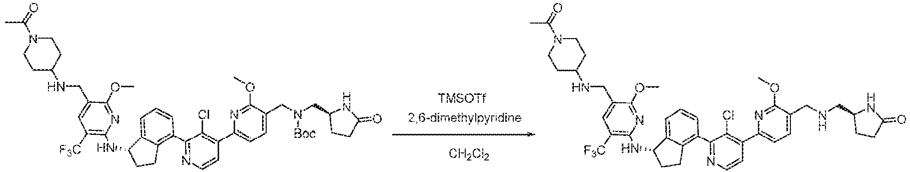

To a mixture of tert-butyl ((2'-((S)-1-((5-(((1-acetylpiperidin-4-yl)amino)methyl)-6-methoxy-3-(trifluoromethyl)pyridin-2-yl)amino)-2,3-dihydro-1H-inden-4-yl)-3'-chloro-6-methoxy-[2,4'-bipyridin]-5-yl)methyl)(((S)-5-oxopyrrolidin-2-yl)methyl)carbamate (0.07 g, 0.08 mmol) in dichloromethane (3 mL) was added 2,6-dimethylpyridine (0.04 g, 0.39 mmol) and trimethylsilyl trifluoromethanesulfonate (0.07 mL, 0.39 mmol). The mixture was stirred at room temperature for 1 h. The mixture was concentrated and the residue was purified by reverse phase HPLC to afford (S)-5-((((2'-((S)-1-((5-(((1-acetylpiperidin-4-yl)amino)methyl)-6-methoxy-3-(trifluoromethyl)pyridin-2-yl)amino)-2,3-dihydro-1H-inden-4-yl)-3'-chloro-6-methoxy-[2,4'-bipyridin]-5-yl)methyl)amino)methyl)pyrrolidin-2-one (2.7 mg, 2%). MS: m/z found 807 [M+H]⁺, retention time = 3.11 min (Method A). ¹H NMR (400 MHz, Methanol-*d₄*): δ 8.51 (d, 1H), 7.71 (d, 1H), 7.62-7.59 (m, 2H), 7.30 (d, 1H), 7.25-7.20 (m, 3H), 5.81-5.75 (m, 1H), 4.44-4.41 (m, 1H), 3.94 (s, 3H), 3.85-3.78 (m, 4H), 3.76-3.68 (m, 5H), 3.06-3.05 (m, 1H), 2.86-2.79 (m, 1H), 2.76-2.73 (m, 2H), 2.62-2.52 (m, 4H), 2.25-2.18 (m, 3H), 2.01-1.92 (m, 6H), 1.74-1.66 (m, 1H), 1.33-1.21 (m, 2H).

### Example 642: (S)-5-((((3'-Chloro-6-methoxy-2'-((S)-1-((6-methoxy-5-(((tetrahydro-2H-pyran-4-yl)amino)methyl)-3-(trifluoromethyl)pyridin-2-yl)amino)-2,3-dihydro-1H-inden-4-yl)-[2,4'-bipyridin]-5-yl)methyl)amino)methyl)pyrrolidin-2-one (634)

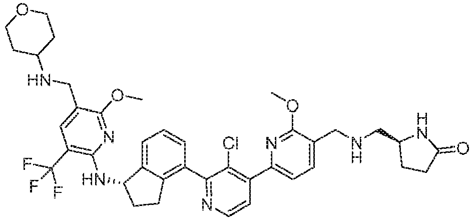

### (a) tert-Butyl ((3'-chloro-6-methoxy-2'-((S)-1-((6-methoxy-5-(((tetrahydro-2H-pyran-4-yl)amino)methyl)-3-(trifluoromethyl)pyridin-2-yl)amino)-2,3-dihydro-1H-inden-4-yl)-[2,4'-bipyridin]-5-yl)methyl)(((S)-5-oxopyrrolidin-2-yl)methyl)carbamate

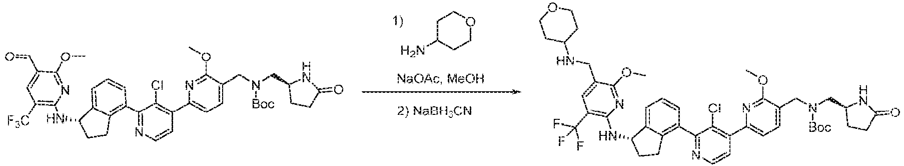

To a mixture of tert-butyl ((3'-chloro-2'-((S)-1-((5-formyl-6-methoxy-3-(trifluoromethyl)pyridin-2-yl)amino)-2,3-dihydro-1H-inden-4-yl)-6-methoxy-[2,4'-bipyridin]-5-yl)methyl)(((S)-5-oxopyrrolidin-2-yl)methyl)carbamate (Example 641, step (b)) (0.38 g, 0.49 mmol) and tetrahydro-2H-pyran-4-amine (0.06 g, 0.58 mmol) in methanol (6 mL) was added sodium acetate (0.12 g, 1.46 mmo). The mixture was stirred at room temperature for 2 h. Sodium cyanoborohydride (0.09 g, 1.46 mmol) was added and the mixture was stirred at room temperature for 1 h. The mixture was concentrated and the residue was purified by normal phase SiO₂ chromatography 50-100% ethyl acetate/petroleum ether to 20% methanol/ethyl acetate) and further purified by prep-TLC (silica gel, ethyl acetate : MeOH = 1:1) to afford tert-butyl ((3'-chloro-6-methoxy-2'-((S)-1-((6-methoxy-5-(((tetrahydro-2H-pyran-4-yl)amino)methyl)-3-(trifluoromethyl)pyridin-2-yl)amino)-2,3-dihydro-1H-inden-4-yl)-[2,4'-bipyridin]-5-yl)methyl)(((S)-5-oxopyrrolidin-2-yl)methyl)carbamate (0.1 g, 20%). MS: m/z found 866 [M+H]⁺.

### (b) (S)-5-((((3'-Chloro-6-methoxy-2'-((S)-1-((6-methoxy-5-(((tetrahydro-2H-pyran-4-yl)amino)methyl)-3-(trifluoromethyl)pyridin-2-yl)amino)-2,3-dihydro-1H-inden-4-yl)-[2,4'-bipyridin]-5-yl)methyl)amino)methyl)pyrrolidin-2-one

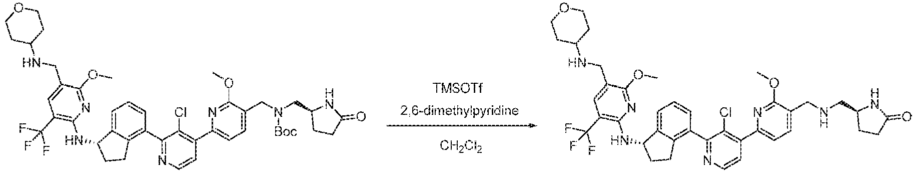

To a mixture of tert-butyl ((3'-chloro-6-methoxy-2'-((S)-1-((6-methoxy-5-(((tetrahydro-2H-pyran-4-yl)amino)methyl)-3-(trifluoromethyl)pyridin-2-yl)amino)-2,3-dihydro-1H-inden-4-yl)-[2,4'-bipyridin]-5-yl)methyl)(((S)-5-oxopyrrolidin-2-yl)methyl)carbamate (0.1 g, 0.11 mmol) in dichloromethane (3 mL) was added 2,6-dimethylpyridine (0.06 g, 0.58 mmol) and trimethylsilyl trifluoromethanesulfonate (0.1 mL, 0.58 mmol). The mixture was stirred at room temperature for 1 h. The mixture was concentrated and the residue was purified by reverse phase HPLC to afford (S)-5-((((3'-chloro-6-methoxy-2'-((S)-1-((6-methoxy-5-(((tetrahydro-2H-pyran-4-yl)amino)methyl)-3-(trifluoromethyl)pyridin-2-yl)amino)-2,3-dihydro-1H-inden-4-yl)-[2,4'-bipyridin]-5-yl)methyl)amino)methyl)pyrrolidin-2-one (6.7 mg, 2%) as a white solid. MS: m/z found 766 [M+H]⁺, retention time = 3.24 min (Method A). ¹H NMR (400 MHz, Methanol-*d*₄): δ 8.51 (d, 1H), 7.71 (d, 1H), 7.62-7.59 (m, 2H), 7.30 (d, 1H), 7.25-7.20 (m, 3H), 5.81-5.75 (m, 1H), 3.94 (s, 3H), 3.90-3.86 (m, 2H), 3.82 (s, 3H), 3.76-3.70 (m, 5H), 3.35-3.30 (m, 2H), 2.76-2.73 (m, 3H), 2.62-2.52 (m, 3H), 2.25-2.22 (m, 3H), 1.98-1.92 (m, 1H), 1.85-1.83 (m, 2H), 1.75-1.70 (m, 1H), 1.42-1.39 (m, 2H).

### Example 643: (S)-5-((((6-(((S)-4-(3'-Chloro-6-methoxy-5-(((tetrahydro-2H-pyran-4-yl)amino)methyl)-[2,4'-bipyridin]-2'-yl)-2,3-dihydro-1H-inden-1-yl)amino)-2-methoxy-5-(trifluoromethyl)pyridin-3-yl)methyl)amino)methyl)pyrrolidin-2-one (666)

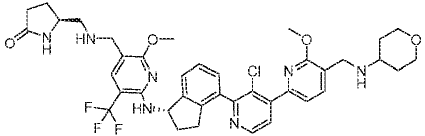

### (a) tert-Butyl (S)-((3'-chloro-2'-(1-((5-formyl-6-methoxy-3-(trifluoromethyl)pyridin-2-yl)amino)-2,3-dihydro-1H-inden-4-yl)-6-methoxy-[2,4'-bipyridin]-5-yl)methyl)(tetrahydro-2H-pyran-4-yl)carbamate

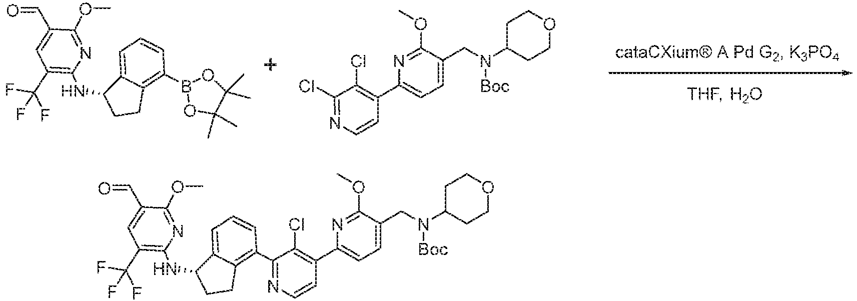

To a mixture of (S)-2-methoxy-6-((4-(4,4,5,5-tetramethyl-1,3,2-dioxaborolan-2-yl)-2,3-dihydro-1H-inden-1-yl)amino)-5-(trifluoromethyl)nicotinaldehyde (Example 640, step (h)) (0.18 g, 0.38 mmol) and tert-butyl ((2',3'-dichloro-6-methoxy-[2,4'-bipyridin]-5-yl)methyl)(tetrahydro-2H-pyran-4-yl)carbamate (0.1 g, 0.21 mmol) in THF/water mixture (20:1, 20.4 mL) was added potassium phosphate (0.14 g, 0.64 mmol) and chloro[(di(1-adamantyl)-N-butylphosphine)-2-(2-aminobiphenyl)]palladium(II) (0.02 g, 0.02 mmol) in one portion under N₂. The mixture was stirred at 85 °C for 5 h. To the mixture was added water (5 mL). The mixture was extracted with ethyl acetate (2 x 5 mL). The combined organic phase was washed with brine (2 x 5 mL), dried with anhydrous sodium sulfate, filtered and concentrated. The residue was purified by normal phase SiO₂ chromatography (0-90% MeOH/ethyl acetate) and purified by reverse phase HPLC to afford tert-butyl (S)-((3'-chloro-2'-(1-((5-formyl-6-methoxy-3-(trifluoromethyl)pyridin-2-yl)amino)-2,3-dihydro- 1H-inden-4-yl)-6-methoxy-[2,4'-bipyridin]-5-yl)methyl)(tetrahydro-2H-pyran-4-yl)carbamate as a yellow oil (13 mg, 4%). MS: m/z found 768 [M+H]⁺.

### (b) tert-Butyl ((3'-chloro-6-methoxy-2'-((S)-1-((6-methoxy-5-(((((S)-5-oxopyrrolidin-2-yl)methyl)amino)methyl)-3-(trifluoromethyl)pyridin-2-yl)amino)-2,3-dihydro-1H-inden-4-yl)-[2,4'-bipyridin]-5-yl)methyl)(tetrahydro-2H-pyran-4-yl)carbamate

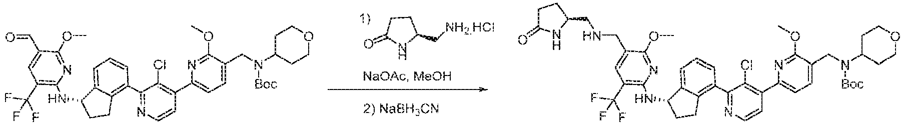

To a mixture of tert-butyl (S)-((3'-chloro-2'-(1-((5-formyl-6-methoxy-3-(trifluoromethyl)pyridin-2-yl)amino)-2,3-dihydro-1H-inden-4-yl)-6-methoxy-[2,4'-bipyridin]-5-yl)methyl)(tetrahydro-2H-pyran-4-yl)carbamate (8 mg, 10.4 µmol) and (S)-5-(aminomethyl)-2-pyrrolidinone hydrochloride salt (2 mg, 12.5 µmol) in methanol (1.5 mL) was added sodium acetate (1.7 mg, 20.8 µmol). The mixture was stirred at room temperature for 1.5 h. Sodium cyanoborohydride (2 mg, 31.2 µmol) was added and the mixture was stirred at room temperature for 0.5 h. The mixture was concentrated to afford tert-butyl ((3'-chloro-6-methoxy-2'-((S)-1-((6-methoxy-5-(((((S)-5-oxopyrrolidin-2-yl)methyl)amino)methyl)-3-(trifluoromethyl)pyridin-2-yl)amino)-2,3-dihydro-1H-inden-4-yl)-[2,4'-bipyridin]-5-yl)methyl)(tetrahydro-2H-pyran-4-yl)carbamate (15 mg, crude) as a white solid. MS: m/z found 866 [M+H]⁺.

### (c) (S)-5-((((6-(((S)-4-(3'-Chloro-6-methoxy-5-(((tetrahydro-2H-pyran-4-yl)amino)methyl)-[2,4'-bipyridin]-2'-yl)-2,3-dihydro-1H-inden-1-yl)amino)-2-methoxy-5-(trifluoromethyl)pyridin-3-yl)methyl)amino)methyl)pyrrolidin-2-one

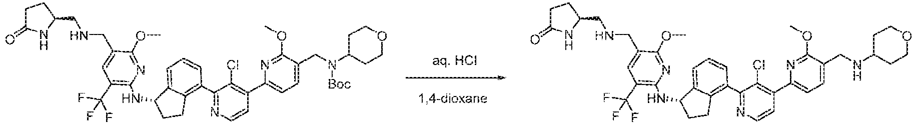

To a mixture of tert-butyl ((3'-chloro-6-methoxy-2'-((S)-1-((6-methoxy-5-(((((S)-5-oxopyrrolidin-2-yl)methyl)amino)methyl)-3-(trifluoromethyl)pyridin-2-yl)amino)-2,3-dihydro-1H-inden-4-yl)-[2,4'-bipyridin]-5-yl)methyl)(tetrahydro-2H-pyran-4-yl)carbamate (23 mg, 0.03 mmol) in 1,4-dioxane (1.5 mL) was added aqueous HCl solution (12 M, 0.4 mL) in one portion under N₂. The mixture was stirred at room temperature for 1 h. The mixture was purified by reverse phase HPLC to afford (S)-5-((((6-(((S)-4-(3'-chloro-6-methoxy-5-(((tetrahydro-2H-pyran-4-yl)amino)methyl)-[2,4'-bipyridin]-2'-yl)-2,3-dihydro-1H-inden-1-yl)amino)-2-methoxy-5-(trifluoromethyl)pyridin-3-yl)methyl)amino)methyl)pyrrolidin-2-one (1.1 mg, 4%) as a white solid (formic acid salt). MS: m/z found m/z: 766 [M+H]⁺. ¹H NMR (400 MHz, Methanol-*d*₄): δ 8.65 (d, 1H), 7.94-7.90 (m, 1H), 7.76 (s, 1H), 7.71 (d, 1H), 7.50-7.48 (m, 1H), 7.37-7.32 (m, 3H), 5.93-5.88 (m, 1H), 4.20 (s, 2H), 4.11 (s, 3H), 4.08-4.04 (m, 3H), 3.98 (s, 3H), 3.95-3.88 (m, 3H), 3.46-3.44 (m, 2H), 2.92-2.85 (m, 4H), 2.68-2.65 (m, 1H), 2.40-2.33 (m, 3H), 2.13-2.09 (m, 3H), 1.87-1.81 (m, 1H), 1.69-1.61 (m, 2H).

### Example 644: (S)-5-((((6-(3-Chloro-2-((S)-1-((6-methoxy-5-(((((S)-5-oxopyrrolidin-2-yl)methyl)amino)methyl)-3-(trifluoromethyl)pyrazin-2-yl)amino)-2,3-dihydro-1H-inden-4-yl)pyridin-4-yl)-1-methyl-1H-pyrrolo[2,3-b]pyridin-3-yl)methyl)amino)methyl)pyrrolidin-2-one (532)

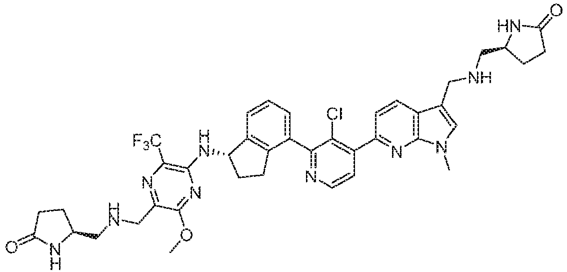

### (a) (S)-3-Methoxy-5-((4-(4,4,5,5-tetramethyl-1,3,2-dioxaborolan-2-yl)-2,3-dihydro-1H-inden-1-yl)amino)-6-(trifluoromethyl)pyrazine-2-carbaldehyde

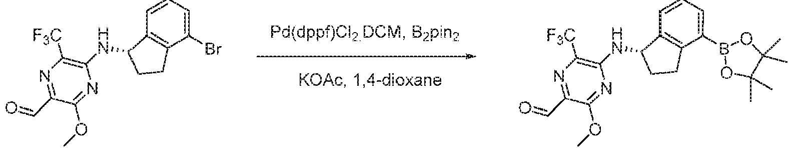

To a mixture of (S)-5-((4-bromo-2,3-dihydro-1H-inden-1-yl)amino)-3-methoxy-6-(trifluoromethyl)pyrazine-2-carbaldehyde (Example 646, step (e)) (2.25 g, 5.41 mmol), bis(pinacolato)diborane (2.06 g, 8.11 mmol) in 1,4-dioxane (40 mL) was added potassium acetate (1.59 g, 16.2 mmol) and [1,1'-bis(diphenylphosphino)ferrocene]dichloropalladium(II) complex with dichloromethane (441 mg, 0.540 mmol). The mixture was stirred at 110 °C for 5 h under N₂. The mixture was diluted with water (20 mL) and extracted with EtOAc (2 x15 mL). The combined organic phases were dried over anhydrous sulfate, filtered and concentrated. The residue was purified by normal phase SiO₂ chromatography (8-15% EtOAc /petroleum ether) to afford (S)-3-methoxy-5-((4-(4,4,5,5-tetramethyl-1,3,2-dioxaborolan-2-yl)-2,3-dihydro- 1H-inden-1-yl)amino)-6-(trifluoromethyl)pyrazine-2-carbaldehyde (2 g, 79% yield) as a yellow oil. ¹H NMR (400 MHz, DMSO-d₆): δ 9.85 (s, 1H), 8.23 (d, 1H), 7.61(d, 1H), 7.38-7.37(m, 1H), 7.26-7.26(m, 1H), 5.96-5.90 (m, 1H), 3.99(s, 3H), 3.57-3.48(m, 1H), 3.03-2.73(m, 1H), 2.57-2.56(m, 1H), 2.52-2.27(m, 1H), 1.39 (s, 12H).

### (b) 6-Chloro-1-methyl-1H-pyrrolo[2,3-b]pyridine-3-carbaldehyde

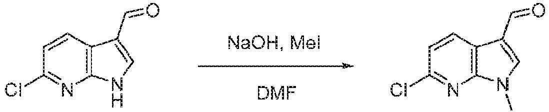

A mixture of 6-chloro-1H-pyrrolo[2,3-b]pyridine-3-carbaldehyde (10 g, 55.4 mmol), sodium hydroxide (4.43 g, 111 mmol) and iodomethane (15.7 g, 111 mmol) in DMF (150 mL) was stirred at room temperature for 12 h under N₂. The mixture was added water (500 mL) and extracted with ethyl acetate (3 x 500 mL). The combined organic phases were dried over anhydrous sulfate, filtered and concentrated. The residue was purified by normal phase SiO₂ chromatography (0-35 % EtOAc/petroleum ether) to afford 6-chloro-1-methyl-1H-pyrrolo[2,3-b]pyridine-3-carbaldehyde (8.1 g, 75% yield) as a white solid. ¹H NMR (400 MHz, Methanol-*d*₄): δ 9.92 (s, 1H), 8.52 (s, 1H), 8.42 (d, 1H), 7.39 (d, 1H), 3.88 (s, 3H).

### (c) 1-Methyl-6-(4,4,5,5-tetramethyl-1,3,2-dioxaborolan-2-yl)-1H-pyrrolo[2,3-b]pyridine-3-carbaldehyde

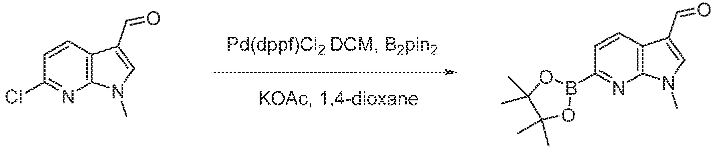

To a mixture of 6-chloro-1-methyl-1H-pyrrolo[2,3-b]pyridine-3-carbaldehyde (2.8 g, 14.4 mmol) and bis(pinacolato)diborane (11 g, 43.2 mmol) in 1,4-dioxane (60 mL) was added potassium acetate (4.24 g, 43.2 mmol) and [1,1'- bis(diphenylphosphino)ferrocene]-dichloropalladium(II) complex with dichloromethane (1.17 g, 1.44 mmol). The mixture was stirred at 85 °C for 2 h under N₂. The mixture was diluted with water (30 mL) and extracted with ethyl acetate (2 x 100 mL). The combined organic phases were dried over anhydrous sulfate, filtered and concentrated. The residue was purified by normal phase SiO₂ chromatography (0-40 % EtOAc/petroleum ether) to afford 1-methyl-6-(4,4,5,5-tetramethyl-1,3,2-dioxaborolan-2-yl)-1H-pyrrolo[2,3-b]pyridine-3-carbaldehyde (850 mg, 20%) as a brown solid. ¹H NMR (400 MHz, Methanol-*d*₄): δ 9.92 (s, 1H), 8.60 (s, 1H), 8.40 (d, 1H), 7.71 (d, 1H), 3.95 (s, 3H), 1.34 (s, 12H).

### (d) 6-(2,3-Dichloropyridin-4-yl)-1-methyl-1H-pyrrolo[2,3-b]pyridine-3-carbaldehyde

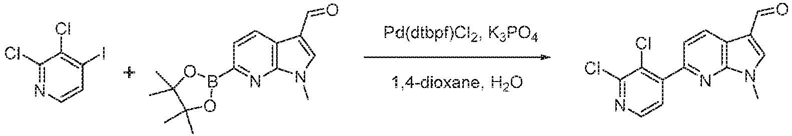

To a mixture of 1-methyl-6-(4,4,5,5-tetramethyl-1,3,2-dioxaborolan-2-yl)-1H-pyrrolo[2,3-b]pyridine-3-carbaldehyde (1 g, 3.49 mmol) and 2,3-dichloro-4-iodopyridine (0.96 g, 3.49 mmol) in dioxane/water (5:1, 24 mL) was added [1,1'-bis(di-tert-butylphosphino)-ferrocene]dichloropalladium(II) (0.23 g, 0.35 mmol) and potassium phosphate (2.23 g, 10.5 mmol). The mixture was stirred at 95 °C for 2 h under N₂. The mixture was diluted with water (50 mL) and extracted with ethyl acetate (2 x 30 mL). The combined organic phases were dried over anhydrous sulfate, filtered and concentrated under reduced pressure. The residue was purified by normal phase SiO₂ chromatography (0-40 % EtOAc/petroleum ether) to afford 6-(2,3-dichloropyridin-4-yl)-1-methyl-1H-pyrrolo[2,3-b]pyridine-3-carbaldehyde (480 mg, 44% yield) as a brown solid. ¹H NMR (400 MHz, Methanol-*d*₄): δ 10.03 (s, 1H), 8.70 (s, 1H), 8.63 (d, 1H), 8.57 (d, 1H), 7.78-7.76 (m, 2H), 4.00 (s, 3H).

### (e) (S)-6-(3-Chloro-2-(1-((5-formyl-6-methoxy-3-(trifluoromethyl)pyrazin-2-yl)amino)-2,3-dihydro-1H-inden-4-yl)pyridin-4-yl)-1-methyl-1H-pyrrolo[2,3-b]pyridine-3-carbaldehyde

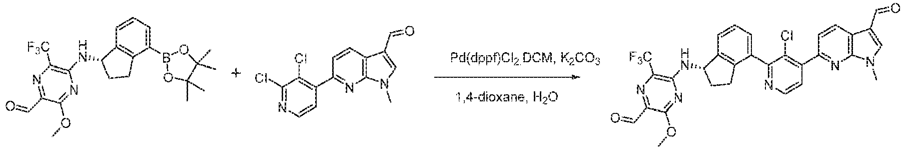

To a mixture of (S)-3-methoxy-5-((4-(4,4,5,5-tetramethyl-1,3,2-dioxaborolan-2-yl)-2,3-dihydro-1H-inden-1-yl)amino)-6-(trifluoromethyl)pyrazine-2-carbaldehyde (150 mg, 489 µmol) and 6-(2,3-dichloropyridin-4-yl)-1-methyl-1H-pyrrolo[2,3-b]pyridine-3-carbaldehyde (340 mg, 734 µmol) in 1,4-dioxane/water (4:1, 5 mL) was added potassium carbonate (203 mg, 1.47 mmol) and [1,1'- bis(diphenylphosphino)ferrocene]dichloropalladium(II) complex with dichloromethane (40 mg, 49 µmol). The mixture was stirred at 95 °C for 1 h under N₂. The solvent was evaporated, and the residue was purified by normal phase SiO₂ chromatography (20-28% ethyl acetate/petroleum ether) to afford (S)-6-(3-chloro-2-(1-((5-formyl-6-methoxy-3-(trifluoromethyl)pyrazin-2-yl)amino)-2,3-dihydro-1H-inden-4-yl)pyridin-4-yl)-1-methyl-1H-pyrrolo[2,3-b]pyridine-3-carbaldehyde (270 mg, 52%) as a yellow solid. MS: m/z found 607 [M+H]⁺.

### (f) (S)-5-((((6-(3-Chloro-2-((S)-1-((6-methoxy-5-(((((S)-5-oxopyrrolidin-2-yl)methyl)amino)methyl)-3-(trifluoromethyl)pyrazin-2-yl)amino)-2,3-dihydro-1H-inden-4-yl)pyridin-4-yl)-1-methyl-1H-pyrrolo[2,3-b]pyridin-3-yl)methyl)amino)methyl)pyrrolidin-2-one

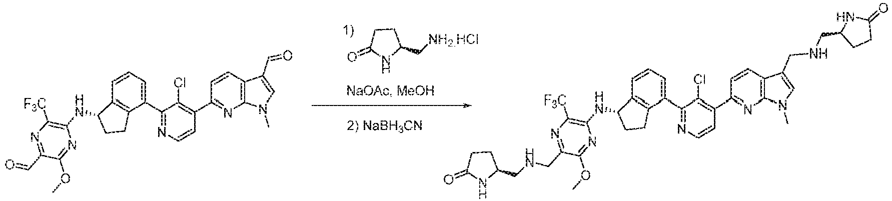

To a mixture of (S)-6-(3-chloro-2-(1-((5-formyl-6-methoxy-3-(trifluoromethyl)pyrazin-2-yl)amino)-2,3-dihydro-1H-inden-4-yl)pyridin-4-yl)-1-methyl-1H-pyrrolo[2,3-b]pyridine-3-carbaldehyde (260 mg, 0.43 mmol), ((S)-5-(aminomethyl)pyrrolidin-2-one hydrochloride salt (146 mg, 0.96 mmol) in methanol (4 mL) was added sodium acetate (105 mg, 1.29 mmol). The mixture was stirred at room temperature for 1 h under N₂. Sodium triacetoxyborohydride (272 mg, 1.29 mmol) was added and the mixture was stirred at room temperature for 0.5 h under N₂. The mixture was filtered and the filtrate was concentrated and the residue was purified by reverse phase HPLC to afford (S)-5-((((6-(3-chloro-2-((S)-1-((6-methoxy-5-(((((S)-5-oxopyrrolidin-2-yl)methyl)amino)methyl)-3-(trifluoromethyl)pyrazin-2-yl)amino)-2,3-dihydro-1H-inden-4-yl)pyridin-4-yl)-1-methyl-1H-pyrrolo[2,3-b]pyridin-3-yl)methyl)amino)methyl)pyrrolidin-2-one (51 mg, 13%) as a white solid (formic acid salt). MS: m/z found 803 [M+H]⁺, retention time = 2.89 min (Method A). ¹H NMR (400 MHz, Methanol-*d*₄): δ 8.66 (d, 1H), 8.29 (d, 1H), 7.72 (d, 1H), 7.65 (s, 1H), 7.58 (d, 1H), 7.37(s, 3H), 5.88-5.84 (m, 1H), 4.60 (s, 1H), 4.31-4.30 (m, 2H), 4.05-4.01 (m, 2H), 3.96-3.91 (m, 8H), 3.04-3.01(m, 2H), 2.93-2.89 (m, 4H), 2.66-2.63 (m, 1H), 2.38-2.33 (m, 6H), 1.91-1.86 (m, 2H).

### Example 645: (S)-5-((((6-(3-Chloro-2-((S)-1-((6-methoxy-5-(((((S)-5-oxopyrrolidin-2-yl)methyl)amino)methyl)-3-(trifluoromethyl)pyridin-2-yl)oxy)-2,3-dihydro-1H-inden-4-yl)pyridin-4-yl)-1-methyl-1H-pyrrolo [2,3-b]pyridin-3-yl)methyl)amino)methyl)pyrrolidin-2-one (545)

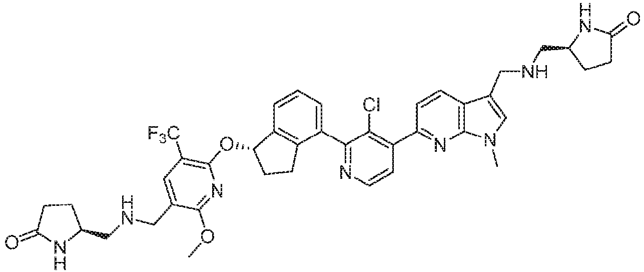

### (a) 2-Chloro-6-methoxy-3-(trifluoromethyl)pyridine

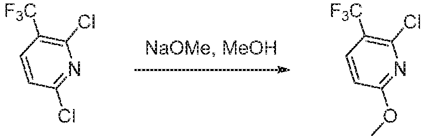

To a solution of 2,6-dichloro-3-(trifluoromethyl)pyridine (10 g, 46 mmol) in methanol (80 mL) was added sodium methanolate (9.2 g, 51 mmol, 30%). The mixture was stirred at room temperature for 1 h and at 60°C for 1 h. The reaction was diluted with water (100 mL) and filtered to give the filter cake. The filter cake was then dried to afford 2-chloro-6-methoxy-3-(trifluoromethyl)pyridine (7 g, 71% yield) as a white solid. The product was used for next step without further purification. ¹H NMR (400 MHz, DMSO-d₆): δ 8.17 (d, 1H), 7.02 (d, 1H), 3.95 (s, 3H).

### (b) 6-Chloro-2-methoxy-5-(trifluoromethyl)nicotinaldehyde

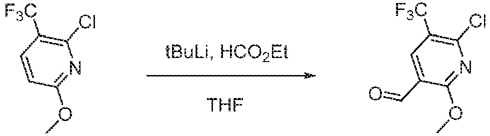

To a solution of 2-chloro-6-methoxy-3-(trifluoromethyl)pyridine (4 g, 19 mmol) in THF (30 mL) at -78°C was added tert-butyllithium (26.2 mL, 1.3 mol/L). The mixture was stirred at -78 °C for 1 h under N₂, followed by addition of ethyl formate (4.2 g, 57 mmol). The mixture was stirred for 0.5 h under N₂. The reaction was quenched with HCl and diluted with water (50 mL). The aqueous layer was extracted with EtOAc (2 x 200 mL). The combined organic phases were dried over anhydrous sulfate, filtered and concentrated. The residue was purified by normal phase SiO₂ chromatography (0-10 % EtOAc/petroleum ether) to afford 6-chloro-2-methoxy-5-(trifluoromethyl)nicotinaldehyde (2.4 g, 53% yield) as a yellow oil. ¹H NMR (400 MHz, DMSO -d₆): δ 10.23 (s, 1H), 8.32 (s, 1H), 4.10 (s, 3H).

### (c) 2-Chloro-5-(1,3-dioxolan-2-yl)-6-methoxy-3-(trifluoromethyl)pyridine

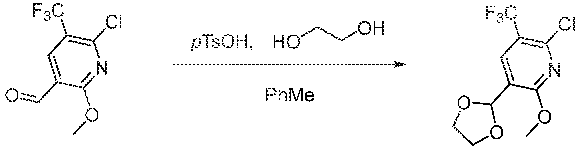

To a mixture of 6-chloro-2-methoxy-5-(trifluoromethyl)nicotinaldehyde (2.5 g, 10.4 mmol) and ethylene glycol (2.59 g, 41.74 mmol) in toluene (25 mL) was added 4-methylbenzenesulfonic acid hydrate (397 mg, 2.09 mmol). The mixture was stirred at 130 °C for 5 h under N₂. The reaction was quenched with saturated sodium bicarbonate (30 mL) solution and extracted with EtOAc (2 x100 mL). The combined organic phases were dried over anhydrous sulfate, filtered and concentrated. The residue was purified by normal phase SiO₂ chromatography (0-15 % EtOAc/petroleum ether) to afford 2-chloro-5-(1,3-dioxolan-2-yl)-6-methoxy-3-(trifluoromethyl)pyridine (1.9 g, 64% yield) as a colorless oil. ¹H NMR (400 MHz, DMSO -d₆): δ 8.03 (s, 1H), 5.87 (s, 1H), 4.02-3.86 (m, 7H).

### (d) (S)-2-((4-bromo-2,3-dihydro-1H-inden-1-yl)oxy)-5-(1,3-dioxolan-2-yl)-6-methoxy-3-(trifluoromethyl)pyridine

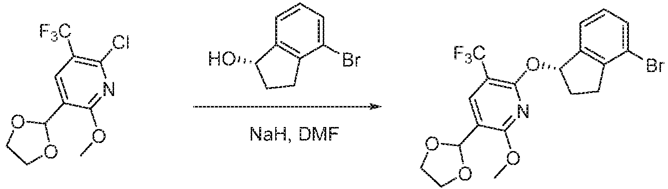

To a solution of (S)-4-bromo-2,3-dihydro-1H-inden-1-ol (1.89 g, 8.9 mmol) in DMF (35 mL) at 0 °C was added sodium hydride (385 mg, 9.7 mmol, 60%). The mixture was stirred at 0 °C for 1 h under N₂. 2-Chloro-5-(1,3-dioxolan-2-yl)-6-methoxy-3-(trifluoromethyl)pyridine (2.1 g, 7.4 mmol) was added and the mixture was stirred at room temperature for 0.5 h under N₂. Saturated sodium bicarbonate (200 mL) solution was added and the mixture extracted with EtOAc (2 x100 mL). The combined organic phases were dried over anhydrous sulfate, filtered and concentrated. The residue was purified by normal phase SiO₂ chromatography (0-15 % EtOAc/petroleum ether) to afford (S)-2-((4-bromo-2,3-dihydro-1H-inden-1-yl)oxy)-5-(1,3-dioxolan-2-yl)-6-methoxy-3-(trifluoromethyl)pyridine (2 g, 59% yield) as a yellow oil. ¹H NMR (400 MHz, DMSO-d₆): δ 7.95 (s, 1H), 7.56 (d, 1H), 7.42 (d, 1H), 7.25-7.21 (m, 1H), 6.69-6.64 (m, 1H), 5.90 (s, 1H), 4.09-3.94 (m, 7H), 3.04-3.01 (m, 1H), 2.93-2.91 (m, 1H), 2.77-2.75 (m, 1H), 2.17-2.14 (m, 1H).

### (e) (S)-6-((4-Bromo-2,3-dihydro-1H-inden-1-yl)oxy)-2-methoxy-5-(trifluoromethyl) nicotinaldehyde

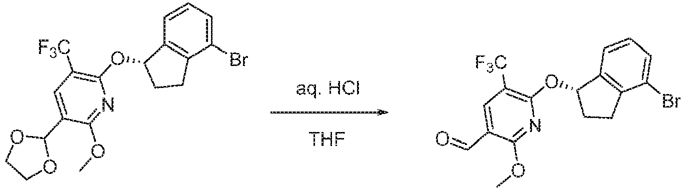

To a solution of (S)-2-((4-bromo-2,3-dihydro-1H-inden-1-yl)oxy)-5-(1,3-dioxolan-2-yl)-6-methoxy-3-(trifluoromethyl)pyridine (1.7 g, 3.69 mmol) in THF (45 mL) was added HCl (9 mL, 6 mol/L). The mixture was stirred at room temperature for 2 h under N₂. The mixture was diluted with saturated sodium bicarbonate solution and extracted with EtOAc (2 x100 mL). The combined organic phases were dried over anhydrous sulfate, filtered and concentrated. The residue was purified by normal phase SiO₂ chromatography (0-15 % EtOAc/petroleum ether) to afford (S)-6-((4-bromo-2,3-dihydro-1H-inden-1-yl)oxy)-2-methoxy-5-(trifluoromethyl)nicotinaldehyde (1.4 g, 77% yield) as a yellow solid. ¹H NMR (400 MHz, DMSO -d₆): δ 10.17 (s, 1H), 8.33 (s, 1H), 7.63 (d, 1H), 7.50 (d, 1H), 7.29 (t, 1H), 6.82-6.79 (m, 1H), 4.02 (s, 3H), 3.10-3.07 (m, 1H), 3.00-2.98 (m, 1H), 2.85-2.83 (m, 1H), 2.26-2.22 (m, 1H).

### (f) (S)-2-Methoxy-6-((4-(4,4,5,5-tetramethyl-1,3,2-dioxaborolan-2-yl)-2,3-dihydro-1H-inden-1-yl)oxy)-5-(trifluoromethyl)nicotinaldehyde

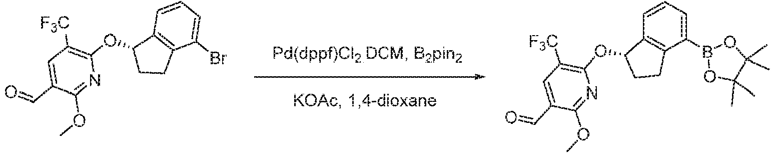

To a mixture of (S)-6-((4-bromo-2,3-dihydro-1H-inden-1-yl)oxy)-2-methoxy-5-(trifluoromethyl)nicotinaldehyde (150 mg, 0.36 mmol) and bis(pinacolato)diborane (275 mg, 1.08 mmol) in 1,4-dioxane (5 mL)was added [1,1'- bis(diphenylphosphino)ferrocene]-dichloropalladium(II) complex with dichloromethane (29 mg, 0.04 mmol) and potassium acetate (106 mg, 1.08 mmol). The mixture was stirred at 110 °C for 5 h under N₂. The mixture was filtered, the filtrate concentrated and the residue purified by normal phase SiO₂ chromatography (0-20 % EtOAc/petroleum ether) to afford (S)-2-methoxy-6-((4-(4,4,5,5-tetramethyl-1,3,2-dioxaborolan-2-yl)-2,3-dihydro-1H-inden-1-yl)oxy)-5-(trifluoromethyl)nicotinaldehyde (150 mg, 89% yield) as a white solid. ¹H NMR (400 MHz, DMSO-d₆): δ 10.12 (s, 1H), 8.29 (s, 1H), 7.68 (d, 1H), 7.57 (d, 1H), 7.31-7.26 (m, 1H), 6.71-6.8 (m, 1H), 4.17 (s, 3H), 3.24-3.20 (m, 1H), 3.12-3.10 (m, 1H), 2.72-2.69 (m, 1H), 2.17-2.14 (m, 1H), 1.32 (s, 12H).

### (g) (S)-6-(3-Chloro-2-(1-((5-formyl-6-methoxy-3-(trifluoromethyl)pyridin-2-yl)oxy)-2,3-dihydro-1H-inden-4-yl)pyridin-4-yl)-1-methyl-1H-pyrrolo[2,3-b]pyridine-3-carbaldehyde

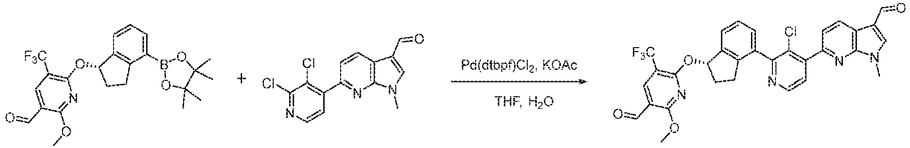

To a mixture of 6-(2,3-dichloropyridin-4-yl)-1-methyl-1H-pyrrolo[2,3-b]pyridine-3-carbaldehyde (Example 644, step (d)) (150 mg, 0.49 mmol) and (S)-2-methoxy-6-((4-(4,4,5,5-tetramethyl-1,3,2-dioxaborolan-2-yl)-2,3-dihydro-1H-inden-1-yl)oxy)-5-(trifluoromethyl)nicotinaldehyde (454 mg, 0.98 mmol) in THF/water (5:1, 6 mL) was added potassium acetate (312 mg, 1.47 mmol) and [1,1'-bis(di-tert-butylphosphino)-ferrocene]dichloropalladium(II) (32 mg, 0.05 mmol). The mixture was stirred at 80 °C for 3 h under N₂. The mixture was diluted with water (10 mL) and extracted with ethyl acetate (2 x 20 mL). The combined organic phases were dried over anhydrous sulfate, filtered and concentrated. The residue was purified by normal phase SiO₂ chromatography (0-100 % EtOAc/petroleum ether) to afford (S)-6-(3-chloro-2-(1-((5-formyl-6-methoxy-3-(trifluoromethyl)pyridin-2-yl)oxy)-2,3-dihydro-1H-inden-4-yl)pyridin-4-yl)-1-methyl-1H-pyrrolo[2,3-b]pyridine-3-carbaldehyde (110 mg, 37%) as a yellow solid. MS: m/z found 607 [M+H]⁺.

### (h) (S)-5-((((6-(3-Chloro-2-((S)-1-((6-methoxy-5-(((((S)-5-oxopyrrolidin-2-yl)methyl)amino)methyl)-3-(trifluoromethyl)pyridin-2-yl)oxy)-2,3-dihydro-1H-inden-4-yl)pyridin-4-yl)-1-methyl-1H-pyrrolo[2,3-b]pyridin-3-yl)methyl)amino)methyl)pyrrolidin-2-one

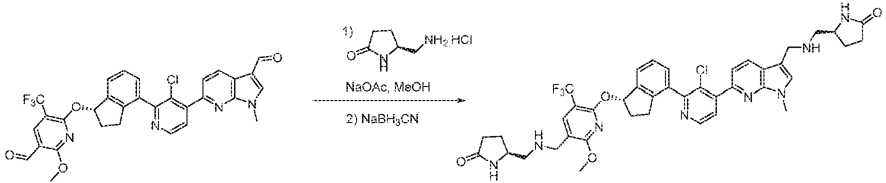

To a mixture of (S)-6-(3-chloro-2-(1-((5-formyl-6-methoxy-3-(trifluoromethyl)pyridin-2-yl)oxy)-2,3-dihydro-1H-inden-4-yl)pyridin-4-yl)-1-methyl-1H-pyrrolo[2,3-b]pyridine-3-carbaldehyde (100 mg, 165 µmol) and (S)-5-(aminomethyl)pyrrolidin-2-one hydrochloride (99 mg, 659 µmol) in methanol (5 mL) was added sodium acetate (68 mg, 824 µmol). The mixture was stirred at room temperature for 2 h under N₂. Sodium cyanoborohydride (41 mg, 659 µmol) was added and the mixture was stirred at room temperature for 0.5 h under N₂. The mixture was concentrated and the residue was purified by reverse phase HPLC to afford (S)-5-((((6-(3-chloro-2-((S)-1-((6-methoxy-5-(((((S)-5-oxopyrrolidin-2-yl)methyl)amino)methyl)-3-(trifluoromethyl)pyridin-2-yl)oxy)-2,3-dihydro-1H-inden-4-yl)pyridin-4-yl)-1-methyl-1H-pyrrolo[2,3-b]pyridin-3-yl)methyl)amino)methyl)pyrrolidin-2-one (28.5 mg, 20% yield) as a white solid. MS: m/z found 803 [M+H]⁺, retention time = 3.19 min (Method A). ¹H NMR (400 MHz, DMSO-d₆): δ 8.71 (d, 1H), 8.22 (d, 1H), 7.98 (s, 1H), 7.69-7.67 (m, 3H), 7.55 (s, 1H), 7.52-7.41 (m, 4H), 6.64 (t, 1H), 4.04 (s, 3H), 3.90 (s, 2H), 3.83 (s, 3H), 3.65-3.61 (m, 4H), 3.02-2.94 (m, 1H), 2.87-2.73 (m, 2H), 2.11-2.08 (m, 9H), 1.73-1.66 (m, 2H), 1.64-1.54 (m, 1H), 1.29-1.24 (m, 1H), 1.04-1.02 (m, 1H), 0.87-0.82 (m, 1H).

### Example 646: (S)-2-(4-(3-Chloro-4-(1-((6-methoxy-5-((7-oxo-2,6-diazaspiro[3.4]octan-2-yl)methyl)-3-(trifluoromethyl)pyrazin-2-yl)amino)-2,3-dihydro-1H-inden-4-yl)pyridin-2-yl)-2-methoxybenzyl)-2,6-diazaspiro[3.4]octan-7-one (366)

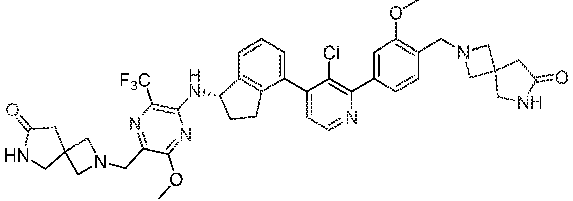

### (a) (S)-5-((4-Bromo-2,3-dihydro-1H-inden-1-yl)amino)-3-chloropyrazine-2-carbonitrile

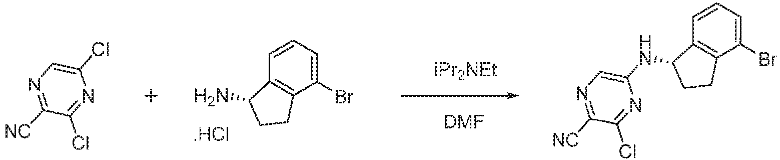

A solution of (S)-4-bromo-2,3-dihydro-1H-inden-1-amine hydrochloride salt (3 g, 12.1 mmol) in DMF (30 mL) and N,N-diisopropylethylamine (4.6 mL, 26.5 mmol) was stirred at 0 °C for 10 min. 3,5-dichloropyrazine-2-carbonitrile (2.2 g, 12.7 mmol) was added to the mixture. The resulting mixture was stirred at room temperature for 20 min. The reaction was combined with another batch at 2 g scale. The reaction mixture was poured into water (50 mL). The combined organic layers were washed with ethyl acetate (3 x 40 mL). The combined organic layers were washed with brine (2 x 50 mL), dried over anhydrous sodium sulfate, filtered and concentrated. The residue was purified by normal phase SiO₂ chromatography (5-50% ethyl acetate /petroleum ether) to afford (S)-5-((4-bromo-2,3-dihydro-1H-inden-1-yl)amino)-3-chloropyrazine-2-carbonitrile (5.2 g) as a light yellow solid. ¹H NMR (400MHz, Methanol-*d₄*): δ = 7.85 (s, 1H), 7.44 (d, 1H), 7.28 (d, 1H), 7.15 - 7.12 (m, 1H), 5.68 (t, 1H), 3.35 - 3.05 (m, 1H), 2.97 - 2.92 (m, 1H), 2.90-2.63 (m, 1H), 2.01 - 1.97 (m, 1H).

### (b) (S)-5-((4-Bromo-2,3-dihydro-1H-inden-1-yl)amino)-3-methoxypyrazine-2-carbonitrile

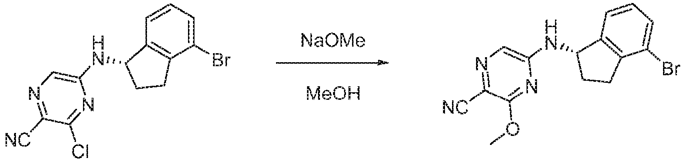

To a solution of (S)-5-((4-bromo-2,3-dihydro-1H-inden-1-yl)amino)-3-chloropyrazine-2-carbonitrile (5.1 g, 14.6 mmol) in methanol (50 mL) was added sodium methanolate (5.25 g, 29 mmol, 30% in MeOH). The mixture was stirred at 40 °C for 2 h. The reaction mixture was concentrated under reduced pressure and the residue was triturated with methanol (50 mL) at room temperature for 10 min to afford (S)-5-((4-bromo-2,3-dihydro-1H-inden-1-yl)amino)-3-methoxypyrazine-2-carbonitrile (4.3 g) as a white solid. ¹H NMR (400MHz, Methanol-*d₄*) δ = 7.53 (s, 1H), 7.42 (d, 1H), 7.27 (d, 1H), 7.14 - 7.10 (m, 1H), 5.71 (br s, 1H), 3.98 (d, 3H), 3.31 - 3.07 (m, 1H), 2.94 - 2.90 (m, 1H), 2.66 - 2.64 (m, 1H), 2.04 - 1.99 (m, 1H).

### (c) (S)-5-((4-Bromo-2,3-dihydro-1H-inden-1-yl)amino)-6-iodo-3-methoxypyrazine-2-carbonitrile

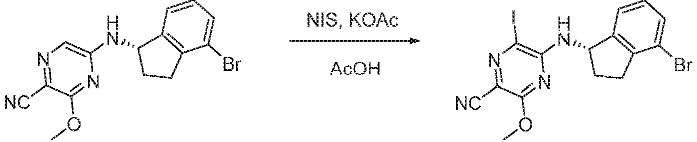

To a solution of S)-5-((4-bromo-2,3-dihydro-1H-inden-1-yl)amino)-3-methoxypyrazine-2-carbonitrile (4.3 g, 12.5 mmol) in acetic acid (60 mL) was added potassium acetate (3.07 g, 31.3 mmol). The suspension was stirred at room temperature for 20 min. N-lodosuccinimide (3.03 g, 13.4 mmol) was added and the resulting mixture was stirred at 50 °C for 3 h. The reaction mixture was diluted with water (40 mL). The solid formed was filtered and the filter cake was washed with water (100 mL). The solid was dried to afford (S)-5-((4-bromo-2,3-dihydro-1H-inden-1-yl)amino)-6-iodo-3-methoxypyrazine-2-carbonitrile (5.5 g, crude) as a white solid. ¹H NMR (400 MHz, DMSO-d₆): δ = 7.93 (d, 1H), 7.46 (d, 1H), 7.21 - 7.19 (m, 1H), 7.15 - 7.12 (m, 1H), 5.57-5.54 (m, 1 H), 3.79 (s, 3H), 3.05 - 2.98 (m, 1H), 2.90-2.50 (m, 1H), 2.50-2.48(m, 1H), 2.28 - 2.25 (m, 1H).

### (d) (S)-5-((4-Bromo-2,3-dihydro-1H-inden-1-yl)amino)-3-methoxy-6-(trifluoromethyl)pyrazine-2-carbonitrile

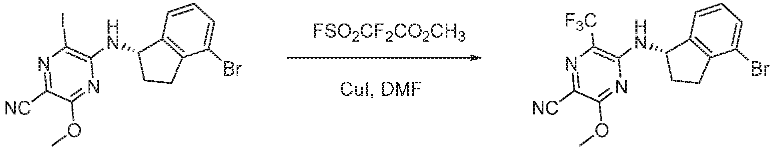

A mixture of (S)-5-((4-bromo-2,3-dihydro-1H-inden-1-yl)amino)-6-iodo-3-methoxypyrazine-2-carbonitrile (3 g, 6.4 mmol), copper(I) iodide (1.46 g, 7.6 mmol) and methyl 2,2-difluoro-2-fluorosulfonyl-acetate (6.12 g, 4.05 mL) in DMF (40 mL) was stirred at 120 °C for 3 h under N₂ atmosphere. The reaction mixture was poured into water (40 mL) and extracted with ethyl acetate (3 x 40 mL). The combined organic layers were washed with brine (2 x 25 mL), dried over anhydrous sodium sulfate, filtered and concentrated under reduced pressure. The residue was purified by normal phase SiO₂ chromatography (0-25% ethyl acetate/petroleum ether) to afford (S)-5-((4-bromo-2,3-dihydro-1H-inden-1-yl)amino)-3-methoxy-6-(trifluoromethyl)pyrazine-2-carbonitrile (1.82 g, 31% / yield for 3 steps) as a yellow solid. ¹H NMR (400MHz, Chloroform-d): δ = 7.46 (d, 1H), 7.19 - 7.11 (m, 2H), 5.80 - 5.76 (m, 2H), 4.04 (s, 3H), 3.11 - 3.08 (m, 1H), 2.98-2.92 (m, 1H), 2.74 - 2.72 (m, 1H), 2.01 - 1.96 (m, 1H)

### (e) (S)-5-((4-Bromo-2,3-dihydro-1H-inden-1-yl)amino)-3-methoxy-6-(trifluoromethyl)pyrazine-2-carbaldehyde

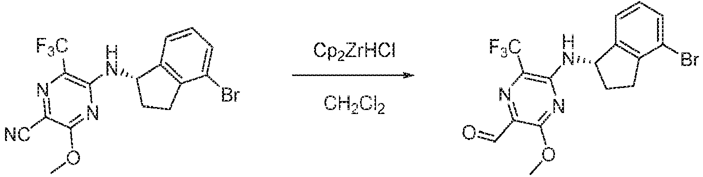

To a solution of (S)-5-((4-bromo-2,3-dihydro-1H-inden-1-yl)amino)-3-methoxy-6-(trifluoromethyl)pyrazine-2-carbonitrile (0.55 g, 1.33 mmol) in dichloromethane (20 mL) was added bis(cyclopentadienyl)zirconium(IV) chloride hydride (0.62 g, 2.33 mmol) at 0°C. The solution was stirred at room temperature for 1 h under N₂. The reaction mixture was diluted with water (20 mL) and extracted with ethyl acetate (3 x 20 mL x 3). The combined organic layers were washed with brine (2 x 10 mL), dried over anhydrous sodium sulfate, filtered and concentrated under reduced pressure. The residue was purified by normal phase SiO₂ chromatography (5-20% ethyl acetate/petroleum ether) to afford (S)-5-((4-bromo-2,3-dihydro-1H-inden-1-yl)amino)-3-methoxy-6-(trifluoromethyl)pyrazine-2-carbaldehyde (0.15 g, 27% yield). ¹H NMR (400 MHz, DMSO-d₆): δ = 9.79 (s, 1H), 8.31 (d, 1H), 7.48 - 7.42 (m, 1H), 7.21 - 7.19 (m, 1H), 7.16 - 7.12 (m, 1H), 5.97 (q, 1H), 3.85 (s, 3H), 3.01 - 2.99 (m, 1H), 2.92-2.90 (m, 1H), 2.88 - 2.86 (m, 1H), 2.31 - 2.26 (m, 1H).

### (f) (S)-5-((4-(2,3-Dichloropyridin-4-yl)-2,3-dihydro-1H-inden-1-yl)amino)-3-methoxy-6-(trifluoromethyl)pyrazine-2-carbaldehyde

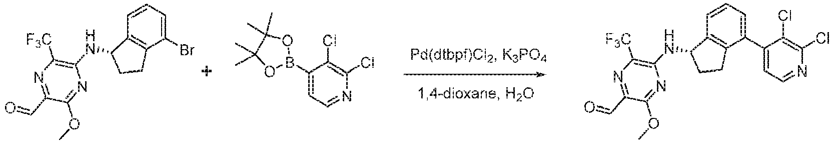

A mixture of (S)-5-((4-bromo-2,3-dihydro-1H-inden-1-yl)amino)-3-methoxy-6-(trifluoromethyl)pyrazine-2-carbaldehyde (1 g, 2.40 mmol), 2,3-dichloro-4-(4,4,5,5-tetramethyl-1,3,2-dioxaborolan-2-yl)pyridine (1.32 g, 4.81 mmol), potassium phosphate (1.53 g, 7.21 mmol) and [1,1'-bis(di-tert-butylphosphino)ferrocene]dichloropalladium(II) (156 mg, 240 µmol) in 1,4-dioxane/water (10:1, 33 mL) was degassed and purged with N₂. The mixture was stirred at 100°C for 3 h under N₂ atmosphere. The reaction mixture was diluted with water (25 mL) and extracted with ethyl acetate (3 x 25 mL). The combined organic layers were washed with brine (2 x 20 mL), dried over anhydrous sodium sulfate, filtered and concentrated under reduced pressure. The residue was purified by normal phase SiO₂ chromatography (10-50% ethyl acetate/petroleum ether) to afford (S)-5-((4-(2,3-dichloropyridin-4-yl)-2,3-dihydro-1H-inden-1-yl)amino)-3-methoxy-6-(trifluoromethyl)pyrazine-2-carbaldehyde (1.1 g, 63% yield) as yellow oil. ¹H NMR (400 MHz, Chloroform-*d*): δ = 9.98 (s, 1H), 8.36 (d, 1H), 7.40 - 7.39 (m, 2H), 7.27 - 7.22 (m, 1H), 7.18 (d, 1H), 5.89 (br s, 2H), 4.11 (s, 3H), 2.80 (br s, 2H), 2.06 - 2.0 (m, 2H).

### (g) (S)-5-((4-(3-Chloro-2-(4-formyl-3-methoxyphenyl)pyridin-4-yl)-2,3-dihydro-1H-inden-1-yl)amino)-3-methoxy-6-(trifluoromethyl)pyrazine-2-carbaldehyde

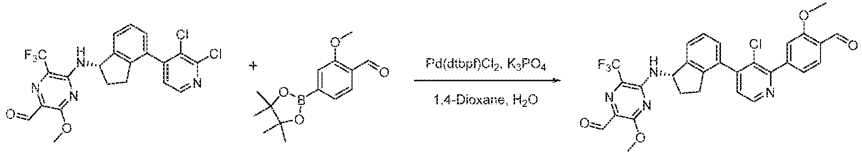

A mixture of (S)-5-((4-(2,3-dichloropyridin-4-yl)-2,3-dihydro-1H-inden-1-yl)amino)-3-methoxy-6-(trifluoromethyl)pyrazine-2-carbaldehyde (0.6 g, 1.24 mmol), 2-methoxy-4-(4,4,5,5-tetramethyl-1,3,2-dioxaborolan-2-yl)benzaldehyde (651 mg, 2.48 mmol), potassium phosphate (791 mg, 3.7 mmol) and [1,1'-bis(di-tert-butylphosphino)ferrocene]dichloropalladium(II) (405 mg, 620 µmol) in 1,4-dioxane/water (10:1, 2.2 mL) was degassed and purged with N₂. The mixture was stirred at 95 °C for 3 h. The reaction mixture was quenched by addition brine (25 mL) and extracted with ethyl acetate (3 x 25 mL). The combined organic layers were washed with brine (2 x 15 mL), dried over anhydrous sodium sulfate, filtered and concentrated under reduced pressure. The residue was purified by normal phase SiO₂ chromatography (10-50% ethyl acetate/petroleum ether) to afford (S)-5-((4-(3-chloro-2-(4-formyl-3-methoxyphenyl)pyridin-4-yl)-2,3-dihydro-1H-inden-1-yl)amino)-3-methoxy-6-(trifluoromethyl)pyrazine-2-carbaldehyde (0.32 g, 44%) as a yellow solid. MS: m/z found 583.2 [M+H]⁺.

### (h) (S)-2-(4-(3-Chloro-4-(1-((6-methoxy-5-((7-oxo-2,6-diazaspiro[3.4]octan-2-yl)methyl)-3-(trifluoromethyl)pyrazin-2-yl)amino)-2,3-dihydro-1H-inden-4-yl)pyridin-2-yl)-2-methoxybenzyl)-2,6-diazaspiro[3.4]octan-7-one

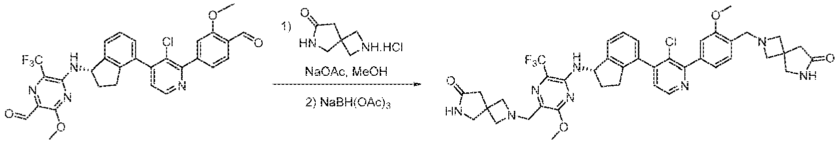

To a solution of (S)-5-((4-(3-chloro-2-(4-formyl-3-methoxyphenyl)pyridin-4-yl)-2,3-dihydro-1H-inden-1-yl)amino)-3-methoxy-6-(trifluoromethyl)pyrazine-2-carbaldehyde (150 mg, 257 µmol) and 2,6-diazaspiro[3.4]octan-7-one as hydrochloride salt (126 mg, 772 µmol) in methanol (1 mL) was added sodium acetate (84 mg, 1 mmol). The solution was stirred at room temperature for 12 h. Sodium triacetoxyborohydride (109 mg, 515 µmol) was added and the solution was stirred at room temperature for 1 h. The reaction was concentrated under reduced pressure and the residue purified by reverse phase HPLC to afford (S)-2-(4-(3-chloro-4-(1-((6-methoxy-5-((7-oxo-2,6-diazaspiro[3.4]octan-2-yl)methyl)-3-(trifluoromethyl)pyrazin-2-yl)amino)-2,3-dihydro-1H-inden-4-yl)pyridin-2-yl)-2-methoxybenzyl)-2,6-diazaspiro[3.4]octan-7-one (29.4 mg, 13%) as a white solid. MS: m/z found 803.3 [(M+H]⁺, retention time = 2.88 min (Method A). ¹H NMR (400 MHz, Methanol-*d*₄)*:* δ = 8.59 (d, 1H), 8.49 (br s, 1H), 7.45 (br d, 1H), 7.39 - 7.29 (m, 6H), 7.22 (br d, 1H), 5.82 (br t, 1H), 4.10 - 4.08 (m, 2H), 3.94 (s, 4H), 3.90 (s, 5H), 3.79 (br s, 4H), 3.68 (s, 4H), 3.62 (s, 2H), 3.60 (s, 2H), 2.86-2.84 (m, 2H), 2.64 (s, 2H), 2.61 (s, 2H), 2.17 - 2.12 (m, 1H).

### Example 647: (S)-1-(6-((5-((4-(2-(4-((6-Acetyl-2,6-diazaspiro[3.3]heptan-2-yl)methyl)-3-methoxyphenyl)-3-chloropyridin-4-yl)-2,3-dihydro-1H-inden-1-yl)amino)-3-methoxy-6-(trifluoromethyl)pyrazin-2-yl)methyl)-2,6-diazaspiro[3.3]heptan-2-yl)ethan-1-one (367)

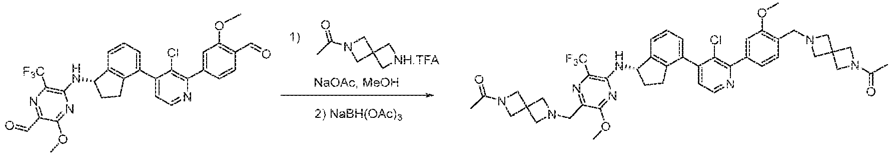

To a solution of (S)-5-((4-(3-chloro-2-(4-formyl-3-methoxyphenyl)pyridin-4-yl)-2,3-dihydro-1H-inden-1-yl)amino)-3-methoxy-6-(trifluoromethyl)pyrazine-2-carbaldehyde (Example 646, step (g)) (150 mg, 257 µmol) and 1-(2,6-diazaspiro[3.3]heptan-2-yl)ethanone trifluoroacetic acid salt (131 mg, 515 µmol) in methanol (1 mL) was added sodium acetate (84 mg, 1 mmol). The solution was stirred at room temperature for 12 h. Sodium triacetoxyborohydride (109 mg, 515 µmol) was added and the resulting solution was stirred at room temperature for 1 h. The reaction was concentrated under reduced pressure and the residue was purified by reverse phase HPLC to afford (S)-1-(6-((5-((4-(2-(4-((6-acetyl-2,6-diazaspiro[3.3]heptan-2-yl)methyl)-3-methoxyphenyl)-3-chloropyridin-4-yl)-2,3-dihydro-1H-inden-1-yl)amino)-3-methoxy-6-(trifluoromethyl)pyrazin-2-yl)methyl)-2,6-diazaspiro[3.3]heptan-2-yl)ethan-1-one (49 mg, 22%) as a white solid. MS: m/z found 831.3 [M+H]⁺, retention time = 3.06 min (Method A). ¹H NMR (400 MHz, Methanol-*d*₄): δ = 8.60 (d, 1H), 8.48 (br s, 1H), 7.45 (d, 1H), 7.39-7.38 (m, 1H), 7.35 - 7.30 (m, 4H), 7.22 (d, 1H), 5.83 (t, 1H), 4.34 (d, 4H), 4.14 (s, 2H), 4.09 (d, 4H), 3.99 - 3.86 (m, 17H), 2.86-2.83 (m, 2H), 2.66 - 2.59 (m, 1H), 2.20 - 2.10 (m, 1H), 1.85 (s, 6H).

### Example 648: (S)-5-((((6-(((S)-4-(3-chloro-2-(3-methoxy-4-(((((S)-5-oxopyrrolidin-2-yl)methyl)amino)methyl)phenyl)pyridin-4-yl)-2,3-dihydro-1H-inden-1-yl)oxy)-2-methoxy-5-(trifluoromethyl)pyridin-3-yl)methyl)amino)methyl)pyrrolidin-2-one (269)

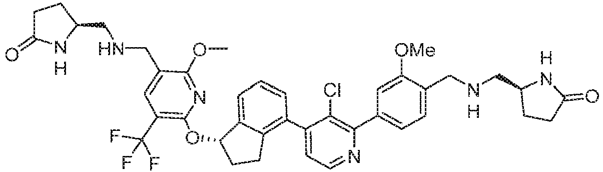

### (a) (S)-6-((4-(2,3-Dichloropyridin-4-yl)-2,3-dihydro-1H-inden-1-yl)oxy)-2-methoxy-5-(trifluoromethyl)nicotinaldehyde

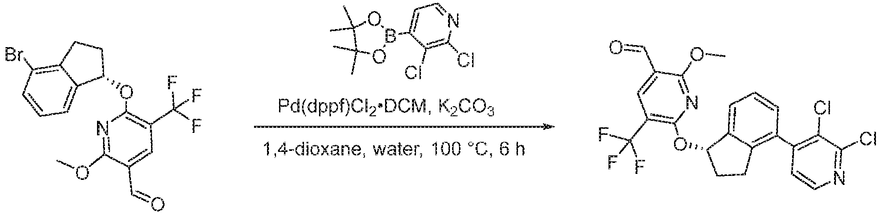

(S)-6-((4-bromo-2,3-dihydro-1H-inden-1-yl)oxy)-2-methoxy-5-(trifluoromethyl)nicotinaldehyde (25 mg, 0.06 mmol), 2,3-dichloro-4-(4,4,5,5-tetramethyl-1,3,2-dioxaborolan-2-yl)pyridine (17 mg, 0.06 mmol), [1,1'-bis(diphenylphosphino)ferrocene]-dichloropalladium(II) complex with dichloromethane (5 mg, 0.01 mmol), and potassium carbonate (21 mg, 0.15 mmol) were suspended in 1,4-dioxane /water (4:1, 2 mL), then the solution was heated at 100 °C for 6 hours. Reaction was cooled to room temperature, diluted with 5 mL water, and extracted with EtOAc (3 x 3 mL). The combined organics were concentrated under reduced pressure and crude sample was purified by silica gel chromatography (0-20% EtOAc/hexane) to afford (S)-6-((4-(2,3-dichloropyridin-4-yl)-2,3-dihydro-1H-inden-1-yl)oxy)-2-methoxy-5-(trifluoromethyl)nicotinaldehyde (8 mg, 27 % yield) as a yellow oil. MS: m/z found 483, 485 [M+H]⁺.

### (b) (S)-6-((4-(3-Chloro-2-(4-formyl-3-methoxyphenyl)pyridin-4-yl)-2,3-dihydro-1H-inden-1-yl)oxy)-2-methoxy-5-(trifluoromethyl)nicotinaldehyde

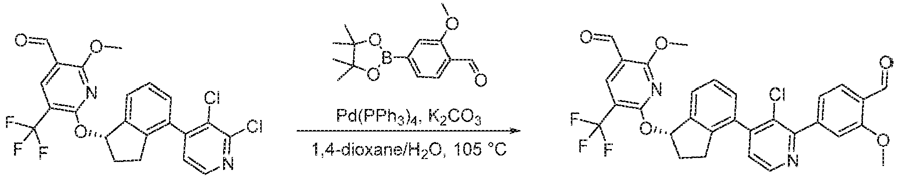

Tetrakis(triphenylphosphine)palladium(0) (4 mg, 0.003 mmol), potassium carbonate (7 mg, 0.05 mmol), (S)-6-((4-(2,3-dichloropyridin-4-yl)-2,3-dihydro-1H-inden-1-yl)oxy)-2-methoxy-5-(trifluoromethyl)nicotinaldehyde (8 mg, 0.02 mmol), and 2-methoxy-4-(4,4,5,5-tetramethyl-1,3,2-dioxaborolan-2-yl)benzaldehyde (6 mg, 0.02 mmol) were suspended in 1,4-dioxane /water (4:1, 1 mL), then the solution was heated at 105 °C for 20 minutes. Reaction was cooled to room temperature, diluted with 3 mL water, and extracted with EtOAc (3 x 2 mL). The combined organics were concentrated under reduced pressure and crude sample was purified by normal phase SiO₂ chromatography (0-50% EtOAc/hexane) to afford (S)-6-((4-(3-chloro-2-(4-formyl-3-methoxyphenyl)pyridin-4-yl)-2,3-dihydro-1H-inden-1-yl)oxy)-2-methoxy-5-(trifluoromethyl)nicotinaldehyde (7 mg, 72 % yield) as a white solid . MS: *m*/*z* found 583 [M+H]⁺.

### (c) (S)-5-((((6-(((S)-4-(3-Chloro-2-(3-methoxy-4-(((((S)-5-oxopyrrolidin-2-yl)methyl)-amino)methyl)phenyl)pyridin-4-yl)-2,3-dihydro-1H-inden-1-yl)oxy)-2-methoxy-5-(trifluoromethyl)pyridin-3-yl)methyl)amino)methyl)pyrrolidin-2-one

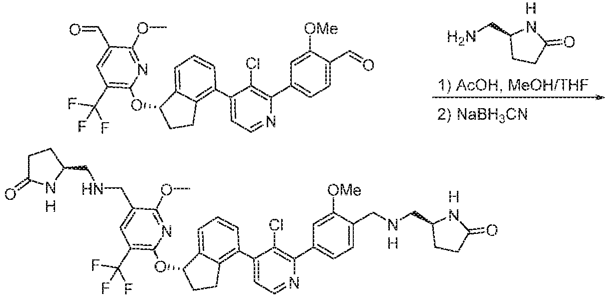

(S)-6-((4-(3-Chloro-2-(4-formyl-3-methoxyphenyl)pyridin-4-yl)-2,3-dihydro-1H-inden-1-yl)oxy)-2-methoxy-5-(trifluoromethyl)nicotinaldehyde (7 mg, 0.01 mmol), acetic acid (2 mg, 0.02 mmol), and (5S)-5-(aminomethyl)pyrrolidin-2-one (5 mg, 0.05 mmol) were dissolved in MeOH/THF (1:1, 1 mL), then the solution was stirred at 25 °C for 2 hours. Sodium cyanoborohydride (2 mg, 0.02 mmol) was added and the resulting mixture was stirred at 25 °C for 1 hour. Reaction was subsequently diluted with water (2 mL) and extracted with EtOAc (3 x 2 mL). The combined organics were further washed with brine, dried over sodium sulfate, filtered, and concentrated under reduced pressure. Crude sample was purified by reverse phase HPLC to afford (S)-5-((((6-(((S)-4-(3-chloro-2-(3-methoxy-4-(((((S)-5-oxopyrrolidin-2-yl)methyl)-amino)methyl)phenyl)pyridin-4-yl)-2,3-dihydro-1H-inden-1-yl)oxy)-2-methoxy-5-(trifluoromethyl)pyridin-3-yl)methyl)amino)methyl)pyrrolidin-2-one (8 mg, 84 % yield) as a white solid (formic acid salt). LC/MS: *m*/*z* found 779, 781 [M+H] ⁺, retention time = 2.31 min (Method C). ¹H NMR (400 MHz, Methanol-*d*₄) δ 8.61 (d, 1H), 7.94 (s, 1H), 7.54 (d, 1H), 7.49 (d, 1H), 7.44 (s, 1H), 7.37 (d, 2H), 7.31 (dd, 2H), 6.67 (s, 1H), 4.20 (s, 2H), 4.12 (d, 3H), 3.98 (d, 4H), 3.89 (s, 3H), 3.04 (s, 3H), 2.81 (s, 4H), 2.48 - 2.15 (m, 7H), 1.85 (s, 2H).

### Example 649: (S)-1-(((6-(((S)-4-(3-Chloro-2-(4-((((S)-2-hydroxypropyl)amino)methyl)-3-methoxyphenyl)pyridin-4-yl)-2,3-dihydro-1H-inden-1-yl)oxy)-2-methoxy-5-(trifluoromethyl)pyridin-3-yl)methyl)amino)propan-2-ol (270)

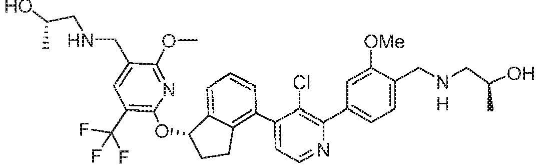

(S)-1-(((6-(((S)-4-(3-Chloro-2-(4-((((S)-2-hydroxypropyl)amino)methyl)-3-methoxyphenyl)pyridin-4-yl)-2,3-dihydro-1H-inden-1-yl)oxy)-2-methoxy-5-(trifluoromethyl)pyridin-3-yl)methyl)amino)propan-2-ol was prepared in the same manner as described for Example 648, utilizing intermediate (S)-6-((4-(3-chloro-2-(4-formyl-3-methoxyphenyl)pyridin-4-yl)-2,3-dihydro-1H-inden-1-yl)oxy)-2-methoxy-5-(trifluoromethyl)nicotinaldehyde and replacing (5S)-5-(aminomethyl)-pyrrolidin-2-one with (2S)-1-aminopropan-2-ol in step (c). Product was obtained as a white solid (formic acid salt). LC/MS: *m*/*z* found 701, 703 [M+H] ⁺, retention time = 2.38 min (Method C). ¹H NMR (400 MHz, Methanol-*d*₄) δ 8.61 (d, 1H), 8.53 (s, 2H), 7.96 (s, 1H), 7.54 (d, 1H), 7.49 (d, 1H), 7.44 (s, 1H), 7.40 (d, 1H), 7.37 (s, 1H), 7.31 (dd, 2H), 6.68 (s, 1H), 4.22 (s, 2H), 4.13 (s, 3H), 3.96 (d, 7H), 2.99 (d, 2H), 2.88 - 2.61 (m, 5H), 2.22 (s, 1H), 1.21 (t, 6H).

### Example 650: (S)-2-(4-(3-chloro-4-(1-((6-methoxy-5-((7-oxo-2,6-diazaspiro[3.4]octan-2-yl)methyl)-3-(trifluoromethyl)pyridin-2-yl)oxy)-2,3-dihydro-1H-inden-4-yl)pyridin-2-yl)-2-methoxybenzyl)-2,6-diazaspiro[3.4]octan-7-one (292)

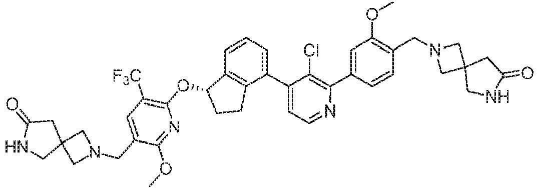

### (a) (S)-6-((4-(2,3-dichloropyridin-4-yl)-2,3-dihydro-1H-inden-1-yl)oxy)-2-methoxy-5-(trifluoromethyl)nicotinaldehyde

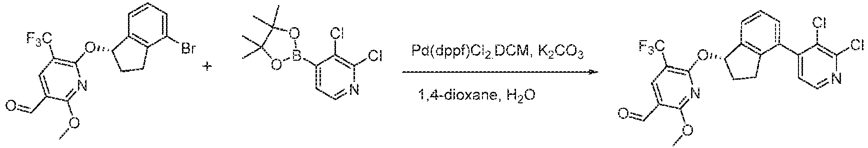

A mixture of (S)-6-((4-bromo-2,3-dihydro-1H-inden-1-yl)oxy)-2-methoxy-5-(trifluoromethyl)nicotinaldehyde (500 mg, 1.20 mmol), 2,3-dichloro-4-(4,4,5,5-tetramethyl-1,3,2-dioxaborolan-2-yl)pyridine (362 mg, 1.32 mmol), potassium carbonate (498 mg, 3.60 mmol) and [1,1'- bis(diphenylphosphino)ferrocene]dichloropalladium(II) complex with dichloromethane (98.1 mg, 0.12 mmol) in 1,4-dioxane/water (5:1, 18 mL) was stirred at 95 °C for 1.5 h under N₂. The mixture was combined with another batch at the 200 mg scale. The mixture was diluted with water (30 mL) and extracted with EtOAc (2 x 30 mL). The combined organic phases were dried over anhydrous sulfate, filtered and concentrated under reduced pressure. The residue was purified by normal phase SiO₂ chromatography (0-20 % EtOAc/petroleum ether) to afford (S)-6-((4-(2,3-dichloropyridin-4-yl)-2,3-dihydro-1H-inden-1-yl)oxy)-2-methoxy-5-(trifluoromethyl)nicotinaldehyde (280 mg). ¹H NMR (400 MHz, DMSO-d₆): δ 10.13 (s, 1H), 8.48 (d, 1H), 8.30 (s, 1H), 7.58-7.57 (m, 2H), 7.46-7.44 (m, 1H), 7.38-7.36 (m, 1H), 6.79 (m, 1H), 4.18 (s, 3H), 2.90-2.75 (m, 3H), 2.16-2.12 (m, 1H).

### (b) (S)-6-((4-(3-chloro-2-(4-formyl-3-methoxyphenyl)pyridin-4-yl)-2,3-dihydro-1H-inden-1-yl)oxy)-2-methoxy-5-(trifluoromethyl)nicotinaldehyde

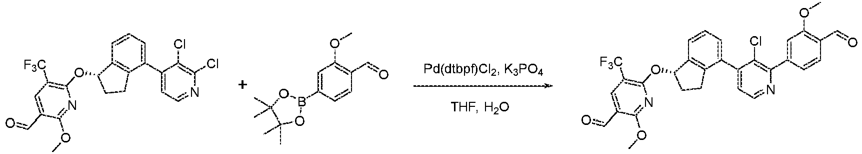

To a mixture of (S)-6-((4-(2,3-dichloropyridin-4-yl)-2,3-dihydro-1H-inden-1-yl)oxy)-2-methoxy-5-(trifluoromethyl)nicotinaldehyde (180 mg, 0.37 mmol) and 2-methoxy-4-(4,4,5,5-tetramethyl-1,3,2-dioxaborolan-2-yl)benzaldehyde (195 mg, 0.74 mmol) in THF/water (5:1, 6 mL) was added potassium phosphate (237 mg, 1.12 mmol) and [1,1'-bis(di-tert-butylphosphino)ferrocene]dichloropalladium(II) (24 mg, 0.04 mmol). The mixture was stirred at 80 °C for 1 h under N₂. The mixture was diluted with water (30 mL) and extracted with EtOAc (2 x10 mL). The combined organic phases were dried over anhydrous sulfate, filtered and concentrated under reduced pressure. The residue was purified by normal phase SiO₂ chromatography (0-40 % EtOAc/petroleum ether) to afford (S)-6-((4-(3-chloro-2-(4-formyl-3-methoxyphenyl)pyridin-4-yl)-2,3-dihydro-1H-inden-1-yl)oxy)-2-methoxy-5-(trifluoromethyl)nicotinaldehyde (130 mg, crude). MS: m/z found 583 [M+H]⁺.

### (c) (S)-2-(4-(3-chloro-4-(1-((6-methoxy-5-((7-oxo-2,6-diazaspiro[3.4]octan-2-yl)methyl)-3-(trifluoromethyl)pyridin-2-yl)oxy)-2,3-dihydro-1H-inden-4-yl)pyridin-2-yl)-2-methoxybenzyl)-2,6-diazaspiro[3.4]octan-7-one

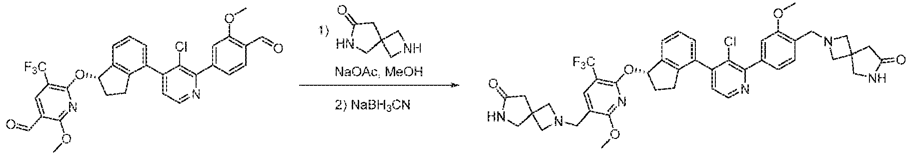

A mixture of (S)-6-((4-(3-chloro-2-(4-formyl-3-methoxyphenyl)pyridin-4-yl)-2,3-dihydro-1H-inden-1-yl)oxy)-2-methoxy-5-(trifluoromethyl)nicotinaldehyde (120 mg, 0.21 mmol), 2,6-diazaspiro[3.4]octan-7-one (104 mg, 0.82 mmol) and sodium acetate (101 mg, 1.24 mmol) in methanol (5 mL) was stirred at room temperature for 0.5 h under N₂. Sodium cyanoborohydride (78 mg, 1.24 mmol) was added and the mixture was stirred at room temperature for 0.5 h under N₂. The mixture was concentrated and the residue was purified by reverse phase HPLC to afford (S)-2-(4-(3-chloro-4-(1-((6-methoxy-5-((7-oxo-2,6-diazaspiro[3.4]octan-2-yl)methyl)-3-(trifluoromethyl)pyridin-2-yl)oxy)-2,3-dihydro-1H-inden-4-yl)pyridin-2-yl)-2-methoxybenzyl)-2,6-diazaspiro[3.4]octan-7-one (40.6 mg, 23%). MS: m/z found 803 [M+H]⁺, retention time = 3.28 min (Method A). ¹H NMR (400 MHz, DMSO-d₆): δ 8.64 (d, 1H), 7.79 (s, 1H), 7.54 (s, 1H), 7.47 (d, 1H), 7.40 (t, 1H), 7.34-7.31 (m, 2H), 7.24-7.22 (m, 2H), 6.61-6.58 (m, 1H), 3.99 (s, 3H), 3.86 (s, 3H), 3.57 (s, 2H), 3.47 (s, 2H), 3.38-3.35 (m, 4H), 3.21-3.14 (m, 8H), 3.86-3.76 (m, 3H), 2.33 (s, 2H), 2.31 (s, 2H), 2.09-2.05 (m, 1H).

### Example 651: (S)-2-(4-(3-chloro-4-(1-((6-methoxy-5-((7-oxo-2,6-diazaspiro[3.4]octan-2-yl)methyl)-3-(trifluoromethyl)pyridin-2-yl)oxy)-2,3-dihydro-1H-inden-4-yl)pyridin-2-yl)-2-fluoro-6-methoxybenzyl)-2,6-diazaspiro[3.4]octan-7-one (306)

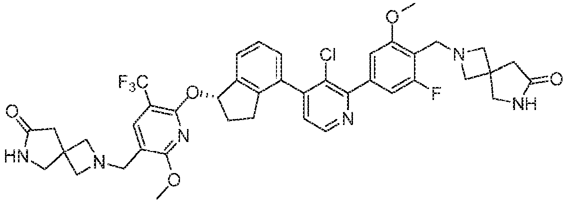

### (a) (S)-6-((4-(3-Chloro-2-(3-fluoro-4-formyl-5-methoxyphenyl)pyridin-4-yl)-2,3-dihydro-1H-inden-1-yl)oxy)-2-methoxy-5-(trifluoromethyl)nicotinaldehyde

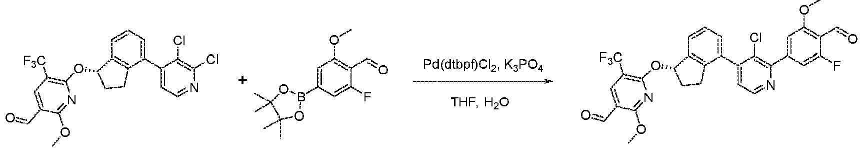

To a mixture of (S)-6-((4-(2,3-dichloropyridin-4-yl)-2,3-dihydro-1H-inden-1-yl)oxy)-2-methoxy-5-(trifluoromethyl)nicotinaldehyde (Example 650, step (a)) (100 mg, 0.21 mmol) and 2-fluoro-6-methoxy-4-(4,4,5,5-tetramethyl-1,3,2-dioxaborolan-2-yl)benzaldehyde (116 mg, 0.41 mmol) in THF/water (5:1, 6 mL) was added potassium phosphate (132 mg, 0. 62 mol) and [1,1'-bis(di-tert-butylphosphino)ferrocene]dichloropalladium(II) (14 mg, 0.02 mmol). The mixture was stirred at 80 °C for 1 h under N₂. The reaction mixture was diluted with water (15 mL) and extracted with EtOAc (2 x20 mL). The combined organic phases were dried over anhydrous sulfate, filtered and concentrated. The residue was purified by normal phase SiO₂ chromatography (0-30 % EtOAc/petroleum ether) to afford (S)-6-((4-(3-chloro-2-(3-fluoro-4-formyl-5-methoxyphenyl)pyridin-4-yl)-2,3-dihydro-1H-inden-1-yl)oxy)-2-methoxy-5-(trifluoromethyl)nicotinaldehyde (95 mg, crude). MS: m/z found 601 [M+H]⁺.

### (b) (S)-2-(4-(3-Chloro-4-(1-((6-methoxy-5-((7-oxo-2,6-diazaspiro[3.4]octan-2-yl)methyl)-3-(trifluoromethyl)pyridin-2-yl)oxy)-2,3-dihydro-1H-inden-4-yl)pyridin-2-yl)-2-fluoro-6-methoxybenzyl)-2,6-diazaspiro[3.4]octan-7-one

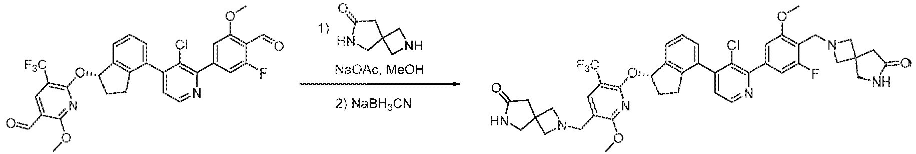

A mixture of (S)-6-((4-(3-chloro-2-(3-fluoro-4-formyl-5-methoxyphenyl)pyridin-4-yl)-2,3-dihydro-1H-inden-1-yl)oxy)-2-methoxy-5-(trifluoromethyl)nicotinaldehyde (76 mg, 599 µmol), 2,6-diazaspiro[3.4]octan-7-one and sodium acetate (73.71 mg, 899 µmol) in methanol (8 mL) was stirred at room temperature for 1.5 h under N₂. Sodium cyanoborohydride (57 mg, 899 µmol) was added and the mixture was stirred at room temperature for 0.5 h under N₂. The mixture was concentrated and the residue was purified by reverse phase HPLC to afford (S)-2-(4-(3-chloro-4-(1-((6-methoxy-5-((7-oxo-2,6-diazaspiro[3.4]octan-2-yl)methyl)-3-(trifluoromethyl)pyridin-2-yl)oxy)-2,3-dihydro-1H-inden-4-yl)pyridin-2-yl)-2-fluoro-6-methoxybenzyl)-2,6-diazaspiro[3.4]octan-7-one (27.9 mg, 22%). MS: m/z found 821 [M+H]⁺, retention time = 3.32 min (Method A). ¹H NMR (400 MHz, DMSO-d₆): δ 8.69 (d, 1H), 7.82 (s, 1H), 7.57-7.50 (m, 4H), 7.43 (t, 1H), 7.36-7.34 (m, 1H), 7.16-7.14 (m, 2H), 6.64-6.63 (m, 1H), 4.02 (s, 3H), 3.87 (s, 3H), 3.60 (s, 2H), 3.50 (s, 2H), 3.38 (s, 2H), 3.31 (s, 2H), 3.19 (s, 8H), 3.89-3.87 (m, 3H), 2.36 (s, 2H), 2.31 (s, 2H), 2.11-2.06 (m, 1H).

### Example 652: (S)-2-((2'-(1-((5-(((1-Acetylpiperidin-4-yl)amino)methyl)-6-methoxy-3-(trifluoromethyl)pyridin-2-yl)oxy)-2,3-dihydro-1H-inden-4-yl)-3'-chloro-6-methoxy-[2,4'-bipyridin]-5-yl)methyl)-2,6-diazaspiro[3.4]octan-7-one (551)

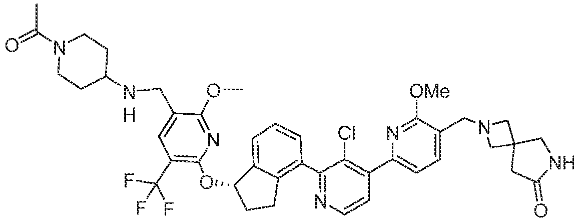

### (a) 2-((2',3'-Dichloro-6-methoxy-[2,4'-bipyridin]-5-yl)methyl)-2,6-diazaspiro[3.4]octan-7-one

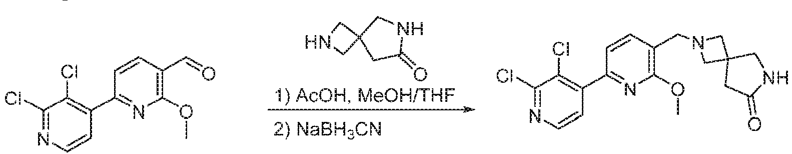

6-(2,3-Dichloro-4-pyridyl)-2-methoxy-pyridine-3-carbaldehyde (200 mg, 0.71 mmol), 2,6-diazaspiro[3.4]octan-7-one (125 mg, 0.99 mmol), and acetic acid (21 mg, 0.35 mmol) were dissolved in MeOH/THF (1:1, 4 mL), then the solution was stirred at 25 °C for 2 hours. Reaction was cooled to 0 °C and sodium cyanoborohydride (89 mg, 1.41 mmol) was added portion-wise over 3 minutes. The resulting mixture was warmed to 25 °C and stirred for an additional 1 hour. Reaction was concentrated under reduced pressure and the residue was dissolved in 15 mL DCM. The organic solution was washed with water (2 x 15 mL) and brine solution, then dried over sodium sulfate. Solution was concentrated under reduced pressure and crude sample was purified by silica gel chromatography (0-10% MeOH/DCM) to afford 2-((2',3'-dichloro-6-methoxy-[2,4'-bipyridin]-5-yl)methyl)-2,6-diazaspiro[3.4]octan-7-one (203 mg, 73 % yield) as a clear oil. MS: *m*/*z* found 393, 395 [M+H]⁺.

### (b) (S)-6-((4-(3'-Chloro-6-methoxy-5-((7-oxo-2,6-diazaspiro[3.4]octan-2-yl)methyl)-[2,4'-bipyridin]-2'-yl)-2,3-dihydro-1H-inden-1-yl)oxy)-2-methoxy-5-(trifluoromethyl)nicotinaldehyde

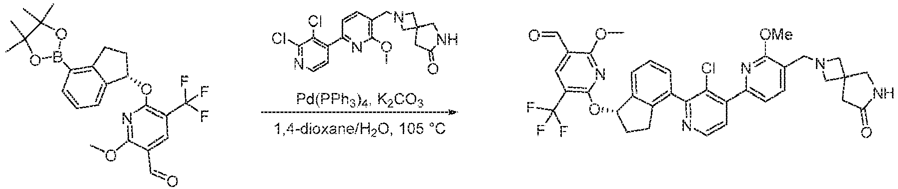

Tetrakis(triphenylphosphine)palladium(0) (19 mg, 0.02 mmol), potassium carbonate (35 mg, 0.25 mmol), (S)-2-methoxy-6-((4-(4,4,5,5-tetramethyl-1,3,2-dioxaborolan-2-yl)-2,3-dihydro-1H-inden-1-yl)oxy)-5-(trifluoromethyl)nicotinaldehyde (Example 645, step (f)) (39 mg, 0.08 mmol), and 2-((2',3'-dichloro-6-methoxy-[2,4'-bipyridin]-5-yl)methyl)-2,6-diazaspiro[3.4]octan-7-one (46 mg, 0.12 mmol) were suspended in 1,4-dioxane/water (4:1, 3 mL), then the solution was heated at 105 °C for 20 minutes. Reaction was cooled to room temperature, diluted with 10 mL water, and extracted with EtOAc (3 x 10 mL). The combined organics were concentrated under reduced pressure and crude sample was purified by normal phase SiO₂ chromatography (0-100% EtOAc/hexane) to afford (S)-6-((4-(3'-chloro-6-methoxy-5-((7-oxo-2,6-diazaspiro[3.4]octan-2-yl)methyl)-[2,4'-bipyridin]-2'-yl)-2,3-dihydro-1H-inden-1-yl)oxy)-2-methoxy-5-(trifluoromethyl)nicotinaldehyde (16 mg, 28 % yield) as a yellow solid. MS: *m*/*z* found 694, 696 [M+H]⁺.

### (c) (S)-2-((2'-(1-((5-(((1-Acetylpiperidin-4-yl)amino)methyl)-6-methoxy-3-(trifluoromethyl)pyridin-2-yl)oxy)-2,3-dihydro-1H-inden-4-yl)-3'-chloro-6-methoxy-[2,4'-bipyridin]-5-yl)methyl)-2,6-diazaspiro[3.4]octan-7-one

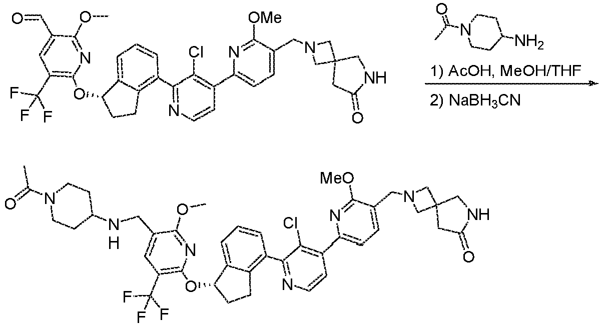

(S)-6-((4-(3'-Chloro-6-methoxy-5-((7-oxo-2,6-diazaspiro[3.4]octan-2-yl)methyl)-[2,4'-bipyridin]-2'-yl)-2,3-dihydro-1H-inden-1-yl)oxy)-2-methoxy-5-(trifluoromethyl)nicotinaldehyde (16 mg, 0.02 mmol), acetic acid (3 mg, 0.05 mmol), and 1-(4-amino-1-piperidyl)ethanone (7 mg, 0.05 mmol) were dissolved in MeOH/THF (1:1, 1 mL), then the solution was stirred at 25 °C for 2 hours. Sodium cyanoborohydride (4 mg, 0.07 mmol) was added and the resulting mixture was stirred at 25 °C for 1 hour. Reaction was filtered through celite, and the filtrate was concentrated under reduced pressure. Crude sample was purified by reverse phase HPLC to afford (S)-2-((2'-(1-((5-(((1-acetylpiperidin-4-yl)amino)methyl)-6-methoxy-3-(trifluoromethyl)pyridin-2-yl)oxy)-2,3-dihydro-1H-inden-4-yl)-3'-chloro-6-methoxy-[2,4'-bipyridin]-5-yl)methyl)-2,6-diazaspiro[3.4]octan-7-one (4 mg, 19 % yield) as a white solid (formic acid salt). LC/MS: *m*/*z* found 820, 822 [M+H] ⁺, retention time = 2.28 min (Method C). ¹H NMR (400 MHz, Methanol-*d*₄) δ 8.60 (d, 1H), 8.50 (s, 1H), 7.98 (s, 1H), 7.76 (d, 1H), 7.70 (d, 1H), 7.55 (s, 1H), 7.41 (d, 3H), 6.69 (d, 1H), 4.57 (d, 1H), 4.14 (d, 3H), 4.02 (d, 6H), 3.76 (s, 2H), 3.59 (s, 2H), 3.46 (s, 4H), 3.25 - 2.96 (m, 3H), 2.89 - 2.64 (m, 3H), 2.59 (s, 2H), 2.12 (s, 6H), 1.39 (s, 2H).

### Example 653: (S)-2-((3'-Chloro-6-methoxy-2'-(1-((6-methoxy-5-(((tetrahydro-2H-pyran-4-yl)amino)methyl)-3-(trifluoromethyl)pyridin-2-yl)oxy)-2,3-dihydro-1H-inden-4-yl)-[2,4'-bipyridin]-5-yl)methyl)-2,6-diazaspiro[3.4]octan-7-one (552)

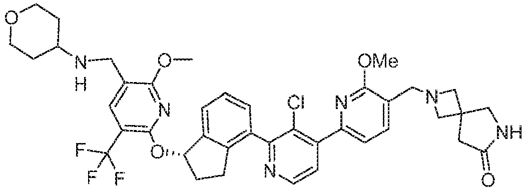

(S)-2-((3'-Chloro-6-methoxy-2'-(1-((6-methoxy-5-(((tetrahydro-2H-pyran-4-yl)amino)methyl)-3-(trifluoromethyl)pyridin-2-yl)oxy)-2,3-dihydro-1H-inden-4-yl)-[2,4'-bipyridin]-5-yl)methyl)-2,6-diazaspiro[3.4]octan-7-one was prepared in similar manner as described for Example 652, utilizing intermediate (S)-6-((4-(3'-chloro-6-methoxy-5-((7-oxo-2,6-diazaspiro[3.4]octan-2-yl)methyl)-[2,4'-bipyridin]-2'-yl)-2,3-dihydro-1H-inden-1-yl)oxy)-2-methoxy-5-(trifluoromethyl)nicotinaldehyde and replacing 1-(4-amino-1-piperidyl)ethanone with tetrahydropyran-4-amine in step (c). Product was obtained as a white solid (formic acid salt). LC/MS: *m*/*z* found 779, 781 [M+H] ⁺, retention time = 2.35 min (Method C). ¹H NMR (400 MHz, Methanol-*d*₄) δ 8.60 (d, 1H), 8.53 (s, 1H), 7.97 (s, 1H), 7.75 (d, 1H), 7.70 (d, 1H), 7.55 (d, 1H), 7.44 - 7.36 (m, 3H), 6.69 (t, 1H), 4.13 (s, 3H), 4.04 - 3.95 (m, 7H), 3.73 (s, 2H), 3.58 (s, 2H), 3.42 (d, 6H), 3.10 - 2.96 (m, 2H), 2.88 - 2.70 (m, 2H), 2.58 (s, 2H), 2.27 - 2.14 (m, 1H), 2.00 (d, 2H), 1.63 - 1.48 (m, 2H).

### Example 654: 2-((3'-Chloro-2'-((S)-1-((5-((((S)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)-2,6-diazaspiro[3.4]octan-7-one (553)

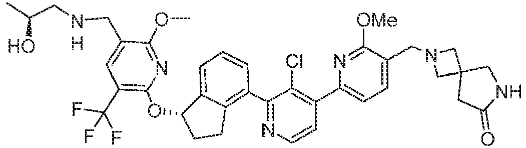

2-((3'-Chloro-2'-((S)-1-((5-((((S)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)-2,6-diazaspiro[3.4]octan-7-one was prepared in similar manner as described for Example 652, utilizing intermediate (S)-6-((4-(3'-chloro-6-methoxy-5-((7-oxo-2,6-diazaspiro[3.4]octan-2-yl)methyl)-[2,4'-bipyridin]-2'-yl)-2,3-dihydro-1H-inden-1-yl)oxy)-2-methoxy-5-(trifluoromethyl)nicotinaldehyde and replacing 1-(4-amino-1-piperidyl)ethanone with (2S)-1-aminopropan-2-ol was used in step (c). Product was obtained as a white solid (formic acid salt). LC/MS: *m*/*z* found 753, 755 [M+H]⁺, retention time = 2.31 min (Method C). ¹H NMR (400 MHz, Methanol-*d*₄) δ 8.60 (d, 1H), 8.52 (s, 1H), 7.99 (s, 1H), 7.75 (d, 1H), 7.70 (d, 1H), 7.56 (s, 1H), 7.43 - 7.37 (m, 3H), 6.69 (s, 1H), 4.15 (s, 3H), 4.07 (s, 2H), 4.01 (s, 4H), 3.73 (s, 2H), 3.58 (s, 2H), 3.44 (s, 4H), 2.92 (s, 2H), 2.76 (s, 3H), 2.58 (s, 2H), 2.21 (s, 1H), 1.21 (d, 3H).

### Example 655: (S)-5-((((2'-((S)-1-((5-(((1-acetylpiperidin-4-yl)amino)methyl)-6-methoxy-3-(trifluoromethyl)pyridin-2-yl)oxy)-2,3-dihydro-1H-inden-4-yl)-3'-chloro-6-methoxy-[2,4'-bipyridin]-5-yl)methyl)amino)methyl)pyrrolidin-2-one (527)

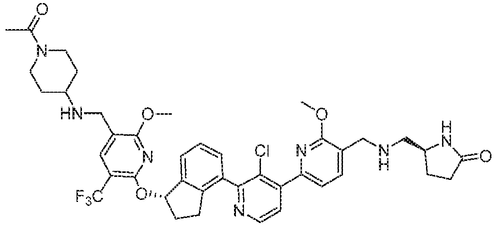

### (a) tert-Butyl (S)-((2',3'-dichloro-6-methoxy-[2,4'-bipyridin]-5-yl)methyl)((5-oxopyrrolidin-2-yl)methyl)carbamate

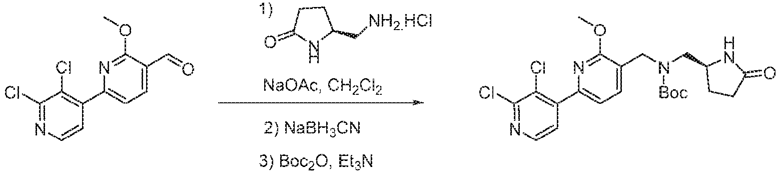

To a mixture of 2',3'-dichloro-6-methoxy-[2,4'-bipyridine]-5-carbaldehyde (Example 640, step (a)) (5.0 g, 5.3 mmol), (5S)-5-(aminomethyl)pyrrolidin-2-one hydrochloride (1.6 g, 10.6 mmol) in DCM (15 mL) was added sodium acetate (1.74 g, 21.2 mmol) then the mixture was stirred at room temperature for 1.5 h under N₂. Sodium cyanoborohydride (0.67 g, 10.6 mmol) was then added and the mixture was stirred at room temperature for 1.5 h under N₂ to afford (S)-5-((((2',3'-dichloro-6-methoxy-[2,4'-bipyridin]-5-yl)methyl)amino)methyl)pyrrolidin-2-one (MS: m/z found 381 [M+H]⁺). Di-tert-butyl dicarbonate (3.66 g, 16.8 mmol), and triethylamine (1.33 g, 13.11 mmol, 1.83 mL, 2.5 *eq*) were then added and the mixture stirred at room temperature for 1 h under N₂. The mixture was concentrated and the residue purified by normal phase SiO₂ chromatography (0-100 % EtOAc/petroleum ether) to afford crude product (1.9 g). 800 mg of crude product was purified by reverse phase HPLC to afford tert-butyl (S)-((2',3'-dichloro-6-methoxy-[2,4'-bipyridin]-5-yl)methyl)((5-oxopyrrolidin-2-yl)methyl)carbamate (500 mg, 62% yield). ¹H NMR (400 MHz, DMSO -d₆): δ 8.54 (d, 1H), 7.83 (s, 1H), 7.75 (d, 1H), 7.62-7.47 (m, 2H), 4.54-4.41 (m, 2H), 4.00 (s, 3H), 3.83-3.82 (m, 1H), 3.36-3.33 (m, 2H), 2.24-2.12 (m, 3H), 1.79-1.78 (m, 1H), 1.49-1.36 (m, 9H).

### (b) tert-Butyl ((3'-chloro-2'-((S)-1-((5-formyl-6-methoxy-3-(trifluoromethyl)pyridin-2-yl)oxy)-2,3-dihydro-1H-inden-4-yl)-6-methoxy-[2,4'-bipyridin]-5-yl)methyl)(((S)-5-oxopyrrolidin-2-yl)methyl)carbamate

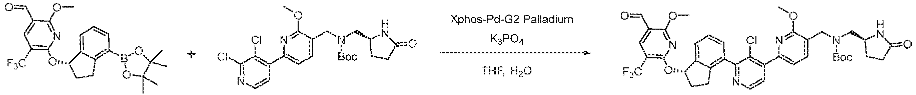

To a mixture of tert-butyl (S)-((2',3'-dichloro-6-methoxy-[2,4'-bipyridin]-5-yl)methyl)((5-oxopyrrolidin-2-yl)methyl)carbamate (300 mg, 0.62 mmol) and (S)-2-methoxy-6-((4-(4,4,5,5-tetramethyl-1,3,2-dioxaborolan-2-yl)-2,3-dihydro-1H-inden-1-yl)oxy)-5-(trifluoromethyl)nicotinaldehyde (Example 645, step (f)) (577 mg, 1.25 mmol) in THF/water (5:1, 12 mL) was added potassium phosphate (397 mg, 1.87 mmol) and [2-(Amino-κN)[1,1-biphenyl]-2-yl-κC]chloro[dicyclohexyl[2,4,6-tris(1-methylethyl)[1,1-biphenyl]-2-yl]phosphine]palladium (49 mg, 0.06 mmol). The mixture was stirred at 80 °C for 6 h under N₂. The mixture was diluted with water (10 mL) and extracted with EtOAc (2 x25 mL). The combined organic phases were dried over anhydrous sulfate, filtered and concentrated under reduced pressure. The residue was purified by normal phase SiO₂ chromatography (0-100 % EtOAc/petroleum ether) to tert-butyl ((3'-chloro-2'-((S)-1-((5-formyl-6-methoxy-3-(trifluoromethyl)pyridin-2-yl)oxy)-2,3-dihydro-1H-inden-4-yl)-6-methoxy-[2,4'-bipyridin]-5-yl)methyl)(((S)-5-oxopyrrolidin-2-yl)methyl)carbamate (190 mg, 38%). MS: m/z found 782 [M+H]⁺.

### (c) tert-Butyl ((2'-((S)-1-((5-(((1-acetylpiperidin-4-yl)amino)methyl)-6-methoxy-3-(trifluoromethyl)pyridin-2-yl)oxy)-2,3-dihydro-1H-inden-4-yl)-3'-chloro-6-methoxy-[2,4'-bipyridin]-5-yl)methyl)(((S)-5-oxopyrrolidin-2-yl)methyl)carbamate

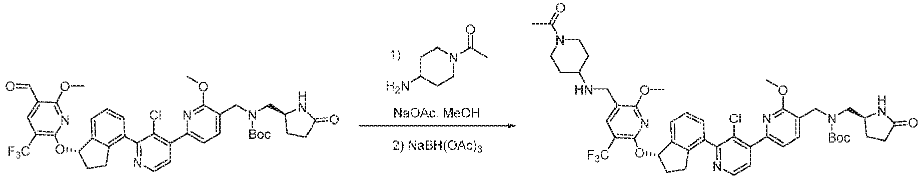

A mixture of tert-butyl ((3'-chloro-2'-((S)-1-((5-formyl-6-methoxy-3-(trifluoromethyl)pyridin-2-yl)oxy)-2,3-dihydro-1H-inden-4-yl)-6-methoxy-[2,4'-bipyridin]-5-yl)methyl)(((S)-5-oxopyrrolidin-2-yl)methyl)carbamate (180 mg, 230 µmol), 1-(4-aminopiperidin-1-yl)ethanone (65.4 mg, 460 µmol) and sodium acetate (57 mg, 690 µmol) in methanol (10 mL) was stirred at room temperature for 1 h under N₂. Sodium triacetoxyborohydride (146 mg, 690 µmol) was added and the mixture was stirred at room temperature for 0.5 h under N₂. The mixture was concentrated and the residue was purified by prep-TLC (SiO2, EtOAc:MeOH = 3:1) to afford tert-butyl ((2'-((S)-1-((5-(((1-acetylpiperidin-4-yl)amino)methyl)-6-methoxy-3-(trifluoromethyl)pyridin-2-yl)oxy)-2,3-dihydro-1H-inden-4-yl)-3'-chloro-6-methoxy-[2,4'-bipyridin]-5-yl)methyl)(((S)-5-oxopyrrolidin-2-yl)methyl)carbamate (145 mg, 69%). MS: m/z found 908 [M+H]⁺.

### (d) (S)-5-((((2'-((S)-1-((5-(((1-Acetylpiperidin-4-yl)amino)methyl)-6-methoxy-3-(trifluoromethyl)pyridin-2-yl)oxy)-2,3-dihydro-1H-inden-4-yl)-3'-chloro-6-methoxy-[2,4'-bipyridin]-5-yl)methyl)amino)methyl)pyrrolidin-2-one

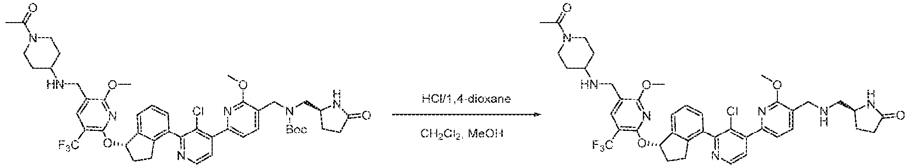

A mixture of tert-butyl ((2'-((S)-1-((5-(((1-acetylpiperidin-4-yl)amino)methyl)-6-methoxy-3-(trifluoromethyl)pyridin-2-yl)oxy)-2,3-dihydro-1H-inden-4-yl)-3'-chloro-6-methoxy-[2,4'-bipyridin]-5-yl)methyl)(((S)-5-oxopyrrolidin-2-yl)methyl)carbamate (140 mg, 154 µmol) and HCl/dioxane (2 mol/L, 12 mL) in DCM/MeOH (3:1, 8 mL) was stirred at room temperature for 5 h under N₂. The mixture was concentrated and the residue was purified by reverse phase HPLC to afford (S)-5-((((2'-((S)-1-((5-(((1-acetylpiperidin-4-yl)amino)methyl)-6-methoxy-3-(trifluoromethyl)pyridin-2-yl)oxy)-2,3-dihydro-1H-inden-4-yl)-3'-chloro-6-methoxy-[2,4'-bipyridin]-5-yl)methyl)amino) methyl)pyrrolidin-2-one (14.1 mg, 10%). MS: m/z found 808 [M+H]⁺, retention time = 3.14 min (Method A). ¹H NMR (400 MHz, DMSO-d₆): δ 8.70 (d, 1H), 7.99 (s, 1H), 7.89 (d, 1H), 7.71-7.69 (m, 2H), 7.50 (d, 1H), 7.45-7.38 (m, 3H), 6.63 (t, 1H), 4.16-4.13 (m, 1H), 4.04 (s, 3H), 3.95 (s, 3H), 3.74-3.63 (m, 6H), 3.09-3.06 (m, 1H), 2.93-2.81 (m, 1H), 2.75-2.67 (m, 6H), 2.34-2.09 (m, 6H), 1.98 (s, 3H), 1.83-1.70 (m, 3H), 1.25-1.10 (m, 2H).

### Example 656: (S)-5-((((6-(((S)-4-(5-(((1-acetylpiperidin-4-yl)amino)methyl)-3'-chloro-6-methoxy-[2,4'-bipyridin]-2'-yl)-2,3-dihydro-1H-inden-1-yl)oxy)-2-methoxy-5-(trifluoromethyl)pyridin-3-yl)methyl)amino)methyl)pyrrolidin-2-one (544)

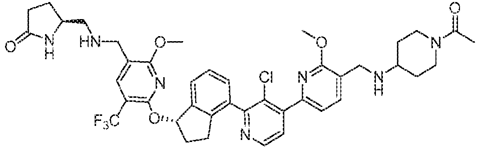

### (a) tert-Butyl (S)-(1-acetylpiperidin-4-yl)((3'-chloro-2'-(1-((5-formyl-6-methoxy-3-(trifluoromethyl)pyridin-2-yl)oxy)-2,3-dihydro-1H-inden-4-yl)-6-methoxy-[2,4'-bipyridin]-5-yl)methyl)carbamate

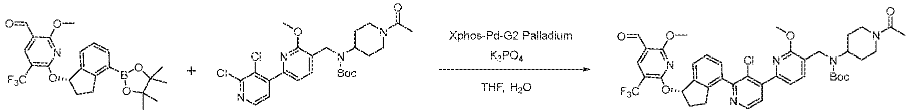

To a mixture of tert-butyl (1-acetylpiperidin-4-yl)((2',3'-dichloro-6-methoxy-[2,4'-bipyridin]-5-yl)methyl)carbamate (Example 640, step (c)) (300 mg, 0.59 mmol) and (S)-2-methoxy-6-((4-(4,4,5,5-tetramethyl-1,3,2-dioxaborolan-2-yl)-2,3-dihydro-1H-inden-1-yl)oxy)-5-(trifluoromethyl)nicotinaldehyde (Example 645, step (f)) (546 mg, 1.18 mmol) in THF/water (5:1, 6 mL) was added potassium phosphate (375 mg, 1.77 mmol) and [2-(amino-κN)[1,1-biphenyl]-2-yl-κC]chloro[dicyclohexyl[2,4,6-tris(1-methylethyl)[1,1-biphenyl]-2-yl]phosphine]palladium (46.3 mg, 0.065 mmol). The mixture was stirred at 80 °C for 4 h under N₂. The mixture was concentrated and the residue was purified by prep-TLC (SiO₂, EtOAc: MeOH = 9:1) to tert-butyl (S)-(1-acetylpiperidin-4-yl)((3'-chloro-2'-(1-((5-formyl-6-methoxy-3-(trifluoromethyl)pyridin-2-yl)oxy)-2,3-dihydro-1H-inden-4-yl)-6-methoxy-[2,4'-bipyridin]-5-yl)methyl)carbamate (150 mg, 31%) as a yellow oil. MS: m/z found 810 [M+H]⁺.

### (b) tert-Butyl (1-acetylpiperidin-4-yl)((3'-chloro-6-methoxy-2'-((S)-1-((6-methoxy-5-(((((S)-5-oxopyrrolidin-2-yl)methyl)amino)methyl)-3-(trifluoromethyl)pyridin-2-yl)oxy)-2,3-dihydro-1H-inden-4-yl)-[2,4'-bipyridin]-5-yl)methyl)carbamate

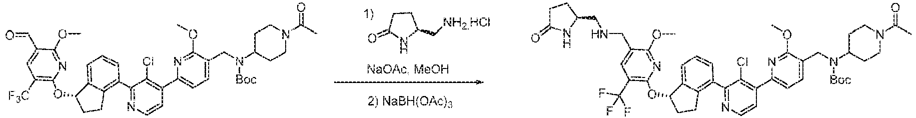

A mixture of tert-butyl (S)-(1-acetylpiperidin-4-yl)((3'-chloro-2'-(1-((5-formyl-6-methoxy-3-(trifluoromethyl)pyridin-2-yl)oxy)-2,3-dihydro-1H-inden-4-yl)-6-methoxy-[2,4'-bipyridin]-5-yl)methyl)carbamate (140 mg, 173 µmol), (5S)-5-(aminomethyl)pyrrolidin-2-one hydrochloride (52 mg, 346 µmol) and sodium acetate (43 mg, 518 µmol) in methanol (5 mL) was stirred at room temperature for 5 h under N₂. Sodium triacetoxyborohydride (110 mg, 518 µmol) was added and the mixture was stirred at room temperature for 0.5 h under N₂. The mixture was concentrated and the residue was purified by prep-TLC (SiO₂, EtOAc: MeOH = 3:1) to afford tert-butyl (1-acetylpiperidin-4-yl)((3'-chloro-6-methoxy-2'-((S)-1-((6-methoxy-5-(((((S)-5-oxopyrrolidin-2-yl)methyl)amino)methyl)-3-(trifluoromethyl)pyridin-2-yl)oxy)-2,3-dihydro-1H-inden-4-yl)-[2,4'-bipyridin]-5-yl)methyl)carbamate (60 mg, 38%) as a yellow oil. MS: m/z found 908 [M+H]⁺.

### (c) (S)-5-((((6-(((S)-4-(5-(((1-acetylpiperidin-4-yl)amino)methyl)-3'-chloro-6-methoxy-[2,4'-bipyridin]-2'-yl)-2,3-dihydro-1H-inden-1-yl)oxy)-2-methoxy-5-(trifluoromethyl)pyridin-3-yl)methyl)amino)methyl)pyrrolidin-2-one

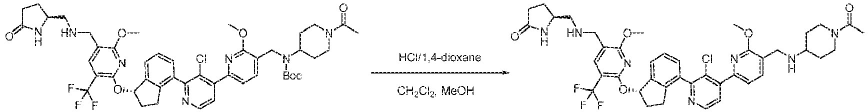

A mixture of tert-butyl (1-acetylpiperidin-4-yl)((3'-chloro-6-methoxy-2'-((S)-1-((6-methoxy-5-(((((S)-5-oxopyrrolidin-2-yl)methyl)amino)methyl)-3-(trifluoromethyl)pyridin-2-yl)oxy)-2,3-dihydro-1H-inden-4-yl)-[2,4'-bipyridin]-5-yl)methyl)carbamate (60 mg, 66 µmol) and HCl/dioxane (2 mol/L, 6 mL) in DCM/MeOH (4:1, 5 mL) was stirred at room temperature for 5 h under N₂. The mixture was concentrated and the residue was purified by reverse phase HPLC to afford (S)-5-((((6-(((S)-4-(5-(((1-acetylpiperidin-4-yl)amino)methyl)-3'-chloro-6-methoxy-[2,4'-bipyridin]-2'-yl)-2,3-dihydro-1H-inden-1-yl)oxy)-2-methoxy-5-(trifluoromethyl)pyridin-3-yl)methyl)amino)methyl)pyrrolidin-2-one (11.9 mg, 21%) as a white solid. MS: m/z found 808 [M+H]⁺, retention time = 3.21 min (Method A). ¹H NMR (400 MHz, DMSO-d₆): δ 8.70 (d, 1H), 7.98 (s, 1H), 7.91 (d, 1H), 7.70 (d, 2H), 7.50 (d, 1H), 7.44-7.38 (m, 3H), 6.63 (t, 1H), 4.17-4.14 (m, 1H), 4.04 (s, 3H), 3.95 (s, 3H), 3.76-3.73 (m, 3H), 3.65-3.60 (m, 3H), 3.10-3.07 (m, 1H), 3.00-2.90 (m, 1H), 2.82-2.67 (m, 4H), 2.13-2.09 (m, 6H), 1.99 (s, 3H), 1.87-1.81 (m, 2H), 1.69-1.64 (m, 1H), 1.31-1.13 (m, 2H).

### Example 657: (S)-5-((((3'-Chloro-6-methoxy-2'-((S)-1-((6-methoxy-5-(((tetrahydro-2H-pyran-4-yl)amino)methyl)-3-(trifluoromethyl)pyridin-2-yl)oxy)-2,3-dihydro-1H-inden-4-yl)-[2,4'-bipyridin]-5-yl)methyl)amino)methyl)pyrrolidin-2-one (608)

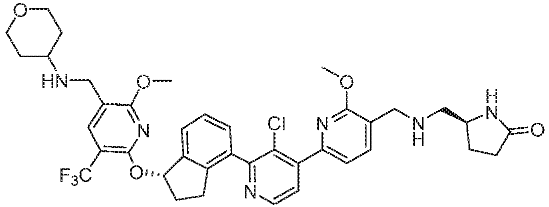

### (a) tert-Butyl ((3'-chloro-6-methoxy-2'-((S)-1-((6-methoxy-5-(((tetrahydro-2H-pyran-4-yl)amino)methyl)-3-(trifluoromethyl)pyridin-2-yl)oxy)-2,3-dihydro-1H-inden-4-yl)-[2,4'-bipyridin]-5-yl)methyl)(((S)-5-oxopyrrolidin-2-yl)methyl)carbamate

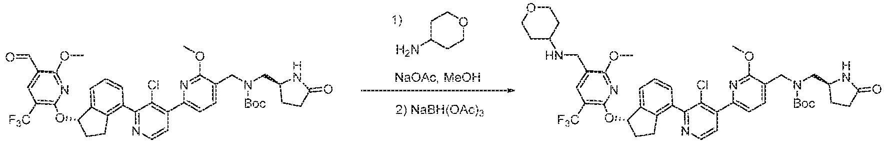

A mixture of tert-butyl ((3'-chloro-2'-((S)-1-((5-formyl-6-methoxy-3-(trifluoromethyl)pyridin-2-yl)oxy)-2,3-dihydro-1H-inden-4-yl)-6-methoxy-[2,4'-bipyridin]-5-yl)methyl)(((S)-5-oxopyrrolidin-2-yl)methyl)carbamate (Example 655, step (b)) (190 mg, 243 µmol), tetrahydro-2H-pyran-4-amine (49.1 mg, 486 µmol) and sodium acetate (60 mg, 729 µmol) in methanol (5 mL) was stirred at room temperature for 3 h under N₂. Sodium triacetoxyborohydride (154 mg, 729 µmol) was added and the mixture was stirred at room temperature for 0.5 h under N₂. The mixture was concentrated and the residue was purified by prep-TLC (SiO₂, EtOAc: MeOH = 3:1) to afford tert-butyl ((3'-chloro-6-methoxy-2'-((S)-1-((6-methoxy-5-(((tetrahydro-2H-pyran-4-yl)amino)methyl)-3-(trifluoromethyl)pyridin-2-yl)oxy)-2,3-dihydro-1H-inden-4-yl)-[2,4'-bipyridin]-5-yl)methyl)(((S)-5-oxopyrrolidin-2-yl)methyl)carbamate (45 mg, 21%) as a white solid. MS: m/z found 867 [M+H]⁺.

### (b) (S)-5-((((3'-Chloro-6-methoxy-2'-((S)-1-((6-methoxy-5-(((tetrahydro-2H-pyran-4-yl)amino)methyl)-3-(trifluoromethyl)pyridin-2-yl)oxy)-2,3-dihydro-1H-inden-4-yl)-[2,4'-bipyridin]-5-yl)methyl)amino)methyl)pyrrolidin-2-one

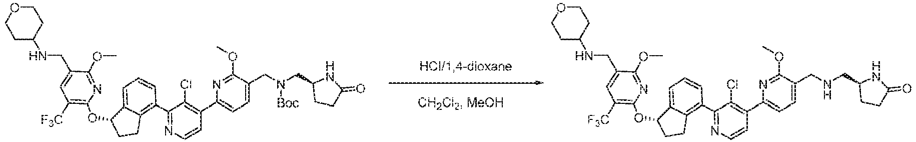

A mixture of tert-butyl ((3'-chloro-6-methoxy-2'-((S)-1-((6-methoxy-5-(((tetrahydro-2H-pyran-4-yl)amino)methyl)-3-(trifluoromethyl)pyridin-2-yl)oxy)-2,3-dihydro-1H-inden-4-yl)-[2,4'-bipyridin]-5-yl)methyl)(((S)-5-oxopyrrolidin-2-yl)methyl)carbamate (80 mg, 92 µmol) and HCl/dioxane (2 mol/L, 10 mL), in DCM/MeOH (3:1, 8 mL) was stirred at room temperature for 5 h under N₂. The mixture was concentrated and the residue was purified by reverse phase HPLC to afford (S)-5-((((3'-chloro-6-methoxy-2'-((S)-1-((6-methoxy-5-(((tetrahydro-2H-pyran-4-yl)amino)methyl)-3-(trifluoromethyl)pyridin-2-yl)oxy)-2,3-dihydro-1H-inden-4-yl)-[2,4'-bipyridin]-5-yl)methyl)amino)methyl)pyrrolidin-2-one (3.6 mg, 9%) as a white solid. MS: m/z found 767 [M+H]⁺, retention time = 3.28 min (Method A). ¹H NMR (400 MHz, Methanol-d₄): δ 8.62 (d, 1H), 7.93 (s, 1H), 7.83 (d, 1H), 7.73 (d, 1H), 7.59-7.57 (m, 1H), 7.44-7.41 (m, 3H), 6.69 (t, 1H), 4.13 (s, 3H), 4.06 (s, 3H), 4.00-3.97 (m, 2H), 3.87-3.81 (m, 5H), 3.51-3.44 (m, 2H), 3.07-2.99 (m, 1H), 2.77-2.70 (m, 5H), 2.38-2.26 (m, 4H), 1.95-1.83 (m, 3H), 1.53-1.31 (m, 2H).

### Example 658: (S)-1-(6-((6-((4-(5-((6-Acetyl-2,6-diazaspiro[3.3]heptan-2-yl)methyl)-3'-chloro-6-methoxy-[2,4'-bipyridin]-2'-yl)-2,3-dihydro-1H-inden-1-yl)oxy)-2-methoxy-5-(trifluoromethyl)pyridin-3-yl)methyl)-2,6-diazaspiro[3.3]heptan-2-yl)ethan-1-one (248)

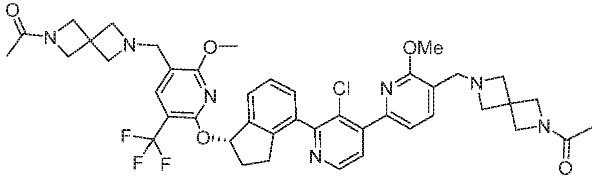

### (a) (S)-3'-Chloro-2'-(1-((5-formyl-6-methoxy-3-(trifluoromethyl)pyridin-2-yl)oxy)-2,3-dihydro-1H-inden-4-yl)-6-methoxy-[2,4'-bipyridine]-5-carbaldehyde

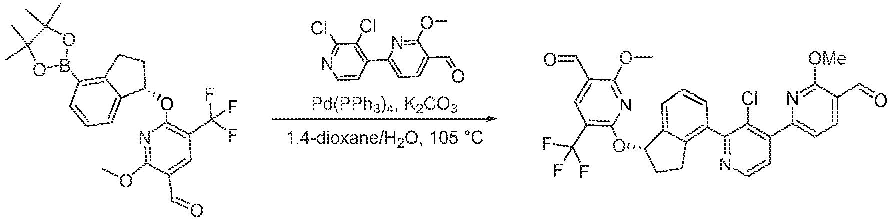

Tetrakis(triphenylphosphine)palladium(0) (51 mg, 0.04 mmol), potassium carbonate (91 mg, 0.66 mmol), (S)-2-methoxy-6-((4-(4,4,5,5-tetramethyl-1,3,2-dioxaborolan-2-yl)-2,3-dihydro-1H-inden-1-yl)oxy)-5-(trifluoromethyl)nicotinaldehyde (Example 645, step (f)) (102 mg, 0.22 mmol), and 2',3'-dichloro-6-methoxy-[2,4'-bipyridine]-5-carbaldehyde (Example 640, step (a)) (94 mg, 0.33 mmol) were suspended in 1,4-dioxane /water (4:1, 3 mL), then the solution was heated at 105 °C for 20 minutes. Reaction was cooled to room temperature, diluted with 10 mL water, and extracted with EtOAc (3 x 5 mL). The combined organics were concentrated under reduced pressure and crude sample was purified by normal phase SiO₂ chromatography (0-100 % EtOAc/hexane) to afford (S)-3'-chloro-2'-(1-((5-formyl-6-methoxy-3-(trifluoromethyl)pyridin-2-yl)oxy)-2,3-dihydro-1H-inden-4-yl)-6-methoxy-[2,4'-bipyridine]-5-carbaldehyde (67 mg, 52 % yield) as a yellow solid. MS: *m*/*z* found 584 [M+H]⁺.

### (b) (S)-1-(6-((6-((4-(5-((6-Acetyl-2,6-diazaspiro[3.3]heptan-2-yl)methyl)-3'-chloro-6-methoxy-[2,4'-bipyridin]-2'-yl)-2,3-dihydro-1H-inden-1-yl)oxy)-2-methoxy-5-(trifluoromethyl)pyridin-3-yl)methyl)-2,6-diazaspiro[3.3]heptan-2-yl)ethan-1-one

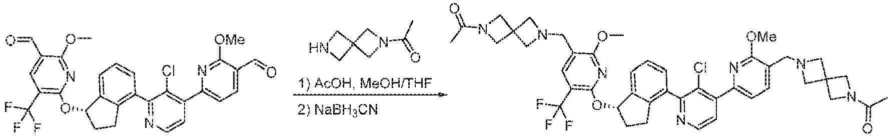

(S)-3'-Chloro-2'-(1-((5-formyl-6-methoxy-3-(trifluoromethyl)pyridin-2-yl)oxy)-2,3-dihydro-1H-inden-4-yl)-6-methoxy-[2,4'-bipyridine]-5-carbaldehyde (16 mg, 0.03 mmol), 1-(2,6-diazaspiro[3.3]heptan-2-yl)ethenone (5 mg, 0.03 mmol), and acetic acid (3 mg, 0.05 mmol) were dissolved in MeOH/THF (1:1, 1 mL), then the solution was stirred at 25 °C for 4 hours. Sodium cyanoborohydride (3 mg, 0.05 mmol) was subsequently added and the resulting mixture was stirred at 25 °C for 2 hours. Reaction was diluted with water (2 mL) and extracted with EtOAc (3 x 2 mL). The combined organics were further washed with brine, dried over sodium sulfate, filtered, and concentrated under reduced pressure. Crude sample was purified by reverse phase HPLC to afford (S)-1-(6-((6-((4-(5-((6-acetyl-2,6-diazaspiro[3.3]heptan-2-yl)methyl)-3'-chloro-6-methoxy-[2,4'-bipyridin]-2'-yl)-2,3-dihydro-1H-inden-1-yl)oxy)-2-methoxy-5-(trifluoromethyl)pyridin-3-yl)methyl)-2,6-diazaspiro[3.3]heptan-2-yl)ethan-1-one (2 mg, 7 % yield) as a white solid (formic acid salt). LC/MS: *m*/*z* found 832, 834 [M+H]⁺, retention time = 2.34 min (Method C). ¹H NMR (400 MHz, Methanol-*d*₄) δ 8.61 (d, 1H), 8.45 (s, 2H), 7.93 - 7.84 (m, 1H), 7.78 (d, 1H), 7.70 (d, 1H), 7.54 (s, 1H), 7.46 - 7.33 (m, 3H), 6.67 (s, 1H), 4.32 (s, 4H), 4.11 (t, 3H), 4.07 (s, 4H), 4.03 (t, 3H), 3.85 (d, 3H), 3.74 (d, 7H), 2.98 (s, 1H), 2.87 - 2.69 (m, 2H), 1.84 (s, 6H).

### Example 659: (S)-1-(((3'-Chloro-2'-((S)-1-((5-((((S)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)propan-2-ol (253)

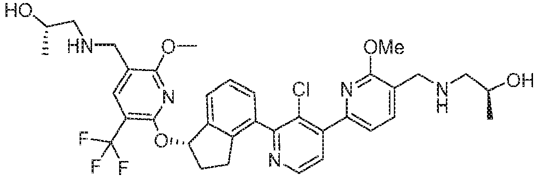

(S)-1-(((3'-Chloro-2'-((S)-1-((5-((((S)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)propan-2-ol was prepared in the same manner as described for Example 658, utilizing intermediate (S)-3'-chloro-2'-(1-((5-formyl-6-methoxy-3-(trifluoromethyl)pyridin-2-yl)oxy)-2,3-dihydro-1H-inden-4-yl)-6-methoxy-[2,4'-bipyridine]-5-carbaldehyde and replacing 1-(2,6-diazaspiro[3.3]heptan-2-yl)ethenone with (S)-1-aminopropan-2-ol in step (b). Product was obtained as a white solid (formic acid salt). LC/MS: *m*/*z* found 702, 704 [M+H] ⁺, retention time = 2.27 min (Method C). ¹H NMR (400 MHz, Methanol-*d*₄) δ 8.62 (m, 1H), 8.52 (s, 1H), 8.01 (s, 1H), 7.89 (d, 1H), 7.71 (m, 1H), 7.56 (d, 1H), 7.50 - 7.43 (m, 1H), 7.43 - 7.36 (m, 2H), 6.70 (t, 1H), 4.18 - 4.12 (m, 5H), 4.11 (s, 2H), 4.09 - 4.06 (m, 3H), 4.02 (d, 2H), 3.07 - 2.91 (m, 3H), 2.90 - 2.66 (m, 4H), 2.27 - 2.16 (m, 1H), 1.25 - 1.15 (m, 6H).

### Example 660: (S)-1-(4-(((6-((4-(5-(((1-Acetylpiperidin-4-yl)amino)methyl)-3'-chloro-6-methoxy-[2,4'-bipyridin]-2'-yl)-2,3-dihydro-1H-inden-1-yl)oxy)-2-methoxy-5-(trifluoromethyl)pyridin-3-yl)methyl)amino)piperidin-1-yl)ethan-1-one (254)

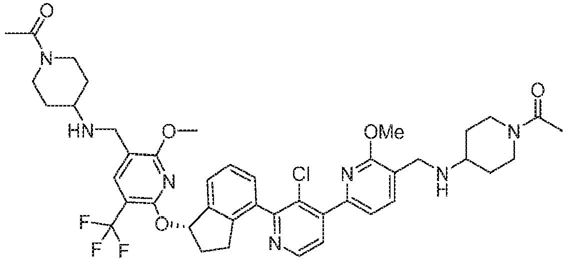

(S)-1-(4-(((6-((4-(5-(((1-Acetylpiperidin-4-yl)amino)methyl)-3'-chloro-6-methoxy-[2,4'-bipyridin]-2'-yl)-2,3-dihydro-1H-inden-1-yl)oxy)-2-methoxy-5-(trifluoromethyl)pyridin-3-yl)methyl)amino)piperidin-1-yl)ethan-1-one was prepared in the same manner as described for Example 658, utilizing intermediate (S)-3'-chloro-2'-(1-((5-formyl-6-methoxy-3-(trifluoromethyl)-pyridin-2-yl)oxy)-2,3-dihydro-1H-inden-4-yl)-6-methoxy-[2,4'-bipyridine]-5-carbaldehyde and replacing 1-(2,6-diazaspiro-[3.3]heptan-2-yl)ethenone with 1-(4-amino-1-piperidyl)-ethanone in step (b). Product was obtained as a white solid (formic acid salt). LC/MS: *m*/*z* found 836, 838 [M+H] ⁺, retention time = 2.31 min (Method C). ¹H NMR (400 MHz, Methanol-*d*₄) δ 8.66 - 8.58 (m, 1H), 8.51 (s, 1H), 8.00 (s, 1H), 7.88 (d, 1H), 7.71 (m, 1H), 7.56 (s, 1H), 7.48 - 7.42 (m, 1H), 7.39 (m, 2H), 6.70 (d, 1H), 4.55 (d, 2H), 4.16 - 4.11 (m, 3H), 4.09 - 4.05 (m, 5H), 4.01 (d, 4H), 3.23 - 2.95 (m, 5H), 2.90 - 2.62 (m, 4H), 2.11 (d, 11H), 1.43 (m, 4H).

### Example 661: (S)-2-((3'-Chloro-6-methoxy-2'-(1-((6-methoxy-5-((7-oxo-2,6-diazaspiro[3.4]octan-2-yl)methyl)-3-(trifluoromethyl)pyridin-2-yl)oxy)-2,3-dihydro-1H-inden-4-yl)-[2,4'-bipyridin]-5-yl)methyl)-2,6-diazaspiro[3.4]octan-7-one (336)

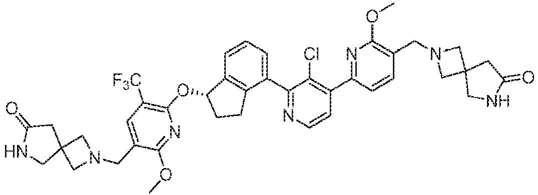

### (a) (S)-2-((3'-Chloro-6-methoxy-2'-(1-((6-methoxy-5-((7-oxo-2,6-diazaspiro[3.4]octan-2-yl)methyl)-3-(trifluoromethyl)pyridin-2-yl)oxy)-2,3-dihydro-1H-inden-4-yl)-[2,4'-bipyridin]-5-yl)methyl)-2,6-diazaspiro[3.4]octan-7-one

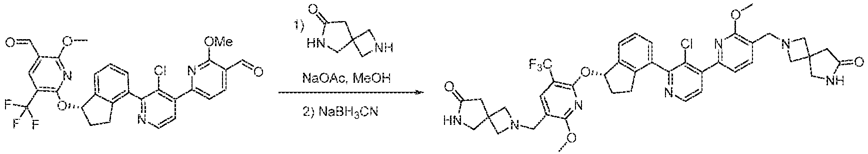

A mixture of (S)-3'-Chloro-2'-(1-((5-formyl-6-methoxy-3-(trifluoromethyl)pyridin-2-yl)oxy)-2,3-dihydro-1H-inden-4-yl)-6-methoxy-[2,4'-bipyridine]-5-carbaldehyde (17 mg, 29.1 µmol), 2,6-diazaspiro[3.4]octan-7-one (15 mg, 116 µmol) and sodium acetate (14.3 mg, 175 µmol) in methanol (3 mL) was stirred at room temperature for 1.5 h under N₂. Sodium cyanoborohydride (11.0 mg, 175 µmol) was added and the mixture was stirred at room temperature for 0.5 h. The mixture was concentrated and the residue was purified by reverse phase HPLC to afford (S)-2-((3'-chloro-6-methoxy-2'-(1-((6-methoxy-5-((7-oxo-2,6-diazaspiro[3.4]octan-2-yl)methyl)-3-(trifluoromethyl)pyridin-2-yl)oxy)-2,3-dihydro-1H-inden-4-yl)-[2,4'-bipyridin]-5-yl)methyl)-2,6-diazaspiro[3.4]octan-7-one (3.8 mg, 15%) as a white solid. MS: m/z found 804 [M+H]⁺, retention time = 3.02 min (Method A). ¹H NMR (400 MHz, DMSO-d₆): δ 8.69 (d, 1H), 7.82-7.78 (m, 2H), 7.68 (d, 1H), 7.56 (d, 2H), 7.50 (d, 1H), 7.45-7.40 (m, 3H), 6.64-6.61 (m, 1H), 4.03 (s, 3H), 3.93 (s, 3H), 3.58 (s, 2H), 3.50 (s, 2H), 3.43-3.40 (m, 4H), 3.28-3.19 (m, 8H), 2.97-2.96 (m, 1H), 2.82-2.76 (m, 1H), 2.38 (s, 2H), 2.36 (s, 2H), 2.10-2.09 (m, 1H).

### Example 662: N-(3-(2-(4-(((1-Acetylpiperidin-4-yl)amino)methyl)-3-methoxyphenyl)-3-chloropyridin-4-yl)-2-chlorophenyl)-1,3-dimethyl-2,4-dioxo-1,2,3,4-tetrahydropyrimidine-5-carboxamide (656)

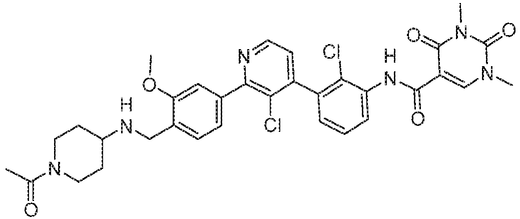

### (a) 2-Chloro-3-(2,3-dichloropyridin-4-yl)aniline

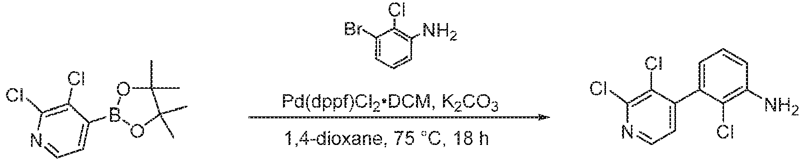

3-bromo-2-chloro-aniline (422 mg, 2.04 mmol), 2,3-dichloro-4-(4,4,5,5-tetramethyl-1,3,2-dioxaborolan-2-yl)pyridine (400 mg, 1.46 mmol), [1,1'-bis(diphenylphosphino)ferrocene]dichloropalladium(II) complex with dichloromethane (119 mg, 0.15 mmol), and potassium carbonate (564 mg, 4.09 mmol) were suspended in 15 mL 1,4-dioxane in a sealed tube. Nitrogen gas was bubbled through the reaction solution for 2 minutes, then the vessel was sealed and heated at 75 °C for 18 hours. Reaction was filtered though celite and the filtrate was concentrated under reduce pressure. Crude sample was purified by silica gel chromatography (0-30% EtOAc/hexane) to afford 2-chloro-3-(2,3-dichloropyridin-4-yl)aniline (206 mg, 52 % yield) as a white solid. MS: *m*/*z* found 273, 275 [M+H]⁺.

### (b) 4-(4-(3-Amino-2-chlorophenyl)-3-chloropyridin-2-yl)-2-methoxybenzaldehyde

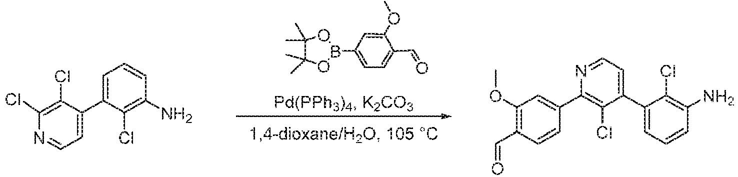

Tetrakis(triphenylphosphine)palladium(0) (174 mg, 0.15 mmol), potassium carbonate (312 mg, 2.26 mmol), 2-chloro-3-(2,3-dichloropyridin-4-yl)aniline (206 mg, 0.75 mmol), and 2-methoxy-4-(4,4,5,5-tetramethyl-1,3,2-dioxaborolan-2-yl)benzaldehyde (257 mg, 0.98 mmol) were suspended in 1,4-dioxane/water (4:1, 15 mL), then the solution was heated at 105 °C for 20 minutes. Reaction was cooled to room temperature, diluted with 20 mL water, and extracted with EtOAc (3 x 10 mL). The combined organics were concentrated under reduced pressure and the crude sample was purified by silica gel chromatography (10-50 % EtOAc/hexane) to afford 4-(4-(3-amino-2-chlorophenyl)-3-chloropyridin-2-yl)-2-methoxybenzaldehyde (168 mg, 60 % yield) as a yellow oil. MS: *m*/*z* found 373, 375 [M+H]⁺.

### (c) N-(2-Chloro-3-(3-chloro-2-(4-formyl-3-methoxyphenyl)pyridin-4-yl)phenyl)-1,3-dimethyl-2,4-dioxo-1,2,3,4-tetrahydropyrimidine-5-carboxamide

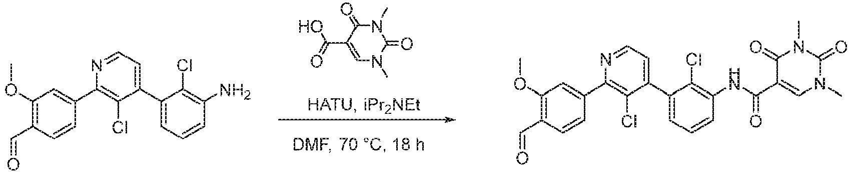

1-[Bis(dimethylamino)methylene]-1H-1,2,3-triazolo[4,5-b]pyridinium 3-oxide hexafluorophosphate (HATU) (257 mg, 0.68 mmol), 1,3-dimethyl-2,4-dioxo-pyrimidine-5-carboxylic acid (91 mg, 0.50 mmol), and 2 equivalents of N,N-diisopropylethylamine were dissolved in 5 mL DMF, then the mixture was stirred at room temperature for 20 minutes. This solution was added dropwise to a solution of 4-(4-(3-amino-2-chlorophenyl)-3-chloropyridin-2-yl)-2-methoxybenzaldehyde (168 mg, 0.45 mmol) and 2 equivalents of N,N-diisopropylethylamine in 5 mL DMF at room temperature. The resulting mixture was stirred at 70 °C for 18 hours. Reaction was diluted with water (30 mL) and extracted with EtOAc (3 x 10 mL). The combined organics were concentrated under reduced pressure and crude sample was purified by silica gel chromatography (5-80 % EtOAc/hexane) to afford N-(2-chloro-3-(3-chloro-2-(4-formyl-3-methoxyphenyl)pyridin-4-yl)phenyl)-1,3-dimethyl-2,4-dioxo-1,2,3,4-tetrahydropyrimidine-5-carboxamide (44 mg, 18 % yield) as a yellow solid. MS: *m*/*z* found 539, 541 [M+H]⁺.

### (d) N-(3-(2-(4-(((1-Acetylpiperidin-4-yl)amino)methyl)-3-methoxyphenyl)-3-chloropyridin-4-yl)-2-chlorophenyl)-1,3-dimethyl-2,4-dioxo-1,2,3,4-tetrahydropyrimidine-5-carboxamide

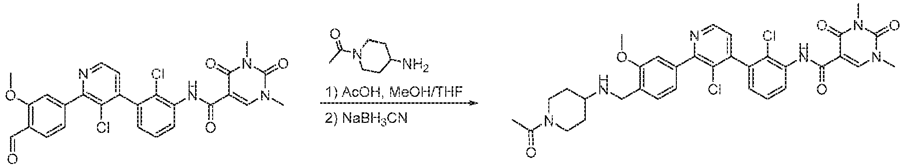

N-(2-Chloro-3-(3-chloro-2-(4-formyl-3-methoxyphenyl)pyridin-4-yl)phenyl)-1,3-dimethyl-2,4-dioxo-1,2,3,4-tetrahydropyrimidine-5-carboxamide (14 mg, 0.03 mmol), acetic acid (3 mg, 0.05 mmol), and 1-(4-amino-1-piperidyl)ethanone (11 mg, 0.08 mmol) were dissolved in MeOH/THF (1:1, 1 mL), then the solution was stirred at 25 °C for 2 hours. Sodium cyanoborohydride (5 mg, 0.08 mmol) was added and the resulting mixture was stirred at 25 °C for 1 hour. Reaction was filtered through celite, and the filtrate was concentrated under reduced pressure. Crude sample was purified by reverse phase HPLC to afford N-(3-(2-(4-(((1-acetylpiperidin-4-yl)amino)methyl)-3-methoxyphenyl)-3-chloropyridin-4-yl)-2-chlorophenyl)-1,3-dimethyl-2,4-dioxo-1,2,3,4-tetrahydropyrimidine-5-carboxamide (8 mg, 48 % yield) as a white solid (formic acid salt). LC/MS: *m*/*z* found 665, 667 [M+H] ⁺, retention time = 2.37 min (Method C). ¹H NMR (400 MHz, Methanol-*d*₄) δ 8.67 (s, 1H), 8.65 (d, 1H), 8.61 (d, 1H), 8.52 (s, 1H), 7.52 - 7.42 (m, 3H), 7.37 (s, 1H), 7.33 (d, 1H), 7.13 (d, 1H), 4.62 (d, 1H), 4.21 (s, 2H), 4.04 (d, 1H), 3.98 (s, 3H), 3.56 (s, 3H), 3.39 (s, 3H), 3.27 - 3.11 (m, 2H), 2.69 (t, 1H), 2.12 (s, 5H), 1.67 - 1.35 (m, 2H).

### Example 663: (S)-N-(2-Chloro-3-(3-chloro-2-(3-methoxy-4-((((5-oxopyrrolidin-2-yl)methyl)amino)methyl)phenyl)pyridin-4-yl)phenyl)-1,3-dimethyl-2,4-dioxo-1,2,3,4-tetrahydropyrimidine-5-carboxamide (657)

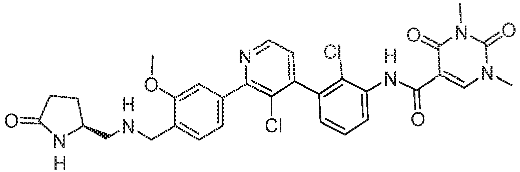

(S)-N-(2-Chloro-3-(3-chloro-2-(3-methoxy-4-((((5-oxopyrrolidin-2-yl)methyl)amino)methyl)phenyl)pyridin-4-yl)phenyl)-1,3-dimethyl-2,4-dioxo-1,2,3,4-tetrahydropyrimidine-5-carboxamide was prepared in similar manner as described for Example 662, utilizing intermediate N-(2-chloro-3-(3-chloro-2-(4-formyl-3-methoxyphenyl)pyridin-4-yl)phenyl)-1,3-dimethyl-2,4-dioxo-1,2,3,4-tetrahydropyrimidine-5-carboxamide and replacing 1-(4-amino-1-piperidyl)ethanone with (5S)-5-(aminomethyl)pyrrolidin-2-one in step (d). Product was obtained as a white solid (formic acid salt). LC/MS: *m*/*z* found 637, 639 [M+H] ⁺, retention time = 2.33 min (Method C). ¹H NMR (400 MHz, Methanol-*d*₄) δ 8.67 (s, 1H), 8.63 (dd, 2H), 8.46 (s, 1H), 7.46 (t, 2H), 7.42 (d, 1H), 7.36 (s, 1H), 7.32 (d, 1H), 7.13 (d, 1H), 4.14 (s, 2H), 3.97 (s, 4H), 3.56 (s, 3H), 3.39 (d, 3H), 2.98 (s, 2H), 2.37 (d, 3H), 1.85 (s, 1H).

### Example 664: N-(2-Chloro-3-(3-chloro-2-(3-methoxy-4-((7-oxo-2,6-diazaspiro[3.4]octan-2-yl)methyl)phenyl)pyridin-4-yl)phenyl)-1,3-dimethyl-2,4-dioxo-1,2,3,4-tetrahydropyrimidine-5-carboxamide (658)

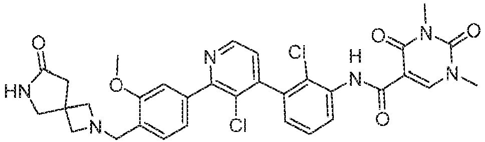

N-(2-Chloro-3-(3-chloro-2-(3-methoxy-4-((7-oxo-2,6-diazaspiro[3.4]octan-2-yl)methyl)phenyl)pyridin-4-yl)phenyl)-1,3-dimethyl-2,4-dioxo-1,2,3,4-tetrahydropyrimidine-5-carboxamide was prepared in similar manner as described for Example 662, utilizing intermediate N-(2-chloro-3-(3-chloro-2-(4-formyl-3-methoxyphenyl)pyridin-4-yl)phenyl)-1,3-dimethyl-2,4-dioxo-1,2,3,4-tetrahydropyrimidine-5-carboxamide and replacing 1-(4-amino-1-piperidyl)ethanone with 2,6-diazaspiro[3.4]octan-7-one in step (d). Product was obtained as a white solid (formic acid salt). LC/MS: *m*/*z* found 649, 651 [M+H]⁺, retention time = 2.34 min (Method C). ¹H NMR (400 MHz, Methanol-*d*₄) δ 8.67 (d, 1H), 8.63 (dd, 2H), 8.46 (s, 1H), 7.50 - 7.39 (m, 3H), 7.36 - 7.28 (m, 2H), 7.13 (d, 1H), 4.18 (s, 2H), 3.95 (s, 3H), 3.91 (s, 4H), 3.63 (s, 2H), 3.56 (d, 3H), 3.39 (s, 3H), 2.66 (s, 2H).

### Example 665: 1-(2-((6-(2-Chloro-3-(3-chloro-2-(4-((4-hydroxypiperidin-1-yl)methyl)-3-methoxyphenyl)pyridin-4-yl)phenyl)-2-methoxypyridin-3-yl)methyl)-2,6-diazaspiro[3.4]octan-6-yl)ethan-1-one (692)

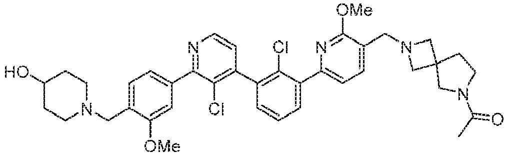

### (a) tert-Butyl 2-((6-(2-chloro-3-(2,3-dichloropyridin-4-yl)phenyl)-2-methoxypyridin-3-yl)methyl)-2,6-diazaspiro[3.4]octane-6-carboxylate

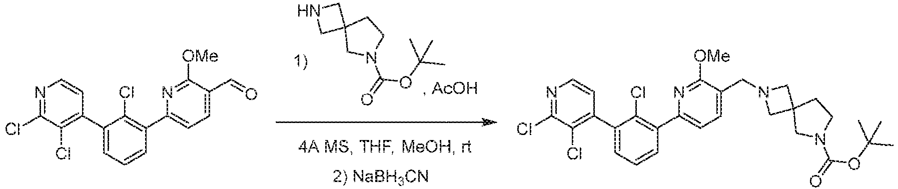

To a mixture of 6-(2-chloro-3-(2,3-dichloropyridin-4-yl)phenyl)-2-methoxynicotinaldehyde (0.10 g, 0.25 mmol), tert-butyl 2,6-diazaspiro[3.4]octane-6-carboxylate (0.11 g, 0.51 mmol), and 4 A molecular sieve (1 g) in 1:1 THF/MeOH (8 mL) was added acetic acid (0.03 g, 0.51 mmol) and the mixture was stirred at room temperature for 3 hrs. Sodium cyanoborohydride (0.04 g, 0.64 mmol) was then added, and the mixture was stirred at room temperature for 30 min. The reaction was quenched by addition of 1 ml water, then was diluted with 50 ml ethyl acetate, and washed with saturated aqueous brine solution (1 x 30 mL). The aqueous layer was extracted with ethyl acetate (3 x 30 mL), then the combined organic layers dried over sodium sulfate and concentrated under reduced pressure. The residue was purified by normal phase flash SiO₂ chromatography using a linear gradient (0-5 % methanol/ dichloromethane) to afford tert-butyl 2-((6-(2-chloro-3-(2,3-dichloropyridin-4-yl)phenyl)-2-methoxypyridin-3-yl)methyl)-2,6-diazaspiro[3.4]octane-6-carboxylate (0.14 g, 93 % yield). MS: m/z found 589 [M+H]⁺.

### (b) 1-(2-((6-(2-Chloro-3-(2,3-dichloropyridin-4-yl)phenyl)-2-methoxypyridin-3-yl)methyl)-2,6-diazaspiro[3.4]octan-6-yl)ethan-1-one

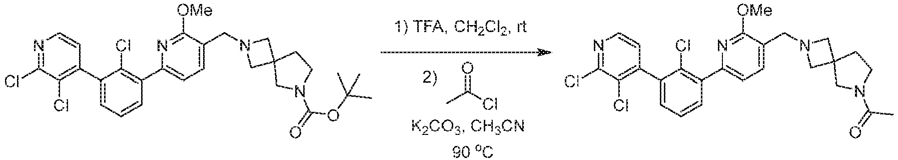

A flask containing tert-butyl 2-((6-(2-chloro-3-(2,3-dichloropyridin-4-yl)phenyl)-2-methoxypyridin-3-yl)methyl)-2,6-diazaspiro[3.4]octane-6-carboxylate (0.05 g, 0.08 mmol) was charged with dichloromethane (3 ml), and trifluoroacetic acid (1 ml). Reaction was stirred 10 min, then evaporated, and the product used crude as the TFA salt (0.06 g, 10 % yield). MS: m/z found 489 [M+H]⁺. To material was added potassium carbonate (0.06 g, 0.41 mmol), acetonitrile (5 ml), and acetyl chloride (0.02 g, 0.25 mmol). Reaction was stirred at 90°C for 2 hr, then evaporated, diluted with 50 ml ethyl acetate, then washed with saturated aqueous brine solution (1 x 30 mL). The aqueous layer was extracted with ethyl acetate (3 x 30 mL), then the combined organic phases dried over sodium sulfate and concentrated under reduced pressure. The residue was purified by normal phase flash SiO₂ chromatography using a linear gradient (0-10 % methanol/dichloromethane) to afford 1-(2-((6-(2-chloro-3-(2,3-dichloropyridin-4-yl)phenyl)-2-methoxypyridin-3-yl)methyl)-2,6-diazaspiro[3.4]octan-6-yl)ethan-1-one (0.094 g, 22 % yield). MS: m/z found 531 [M+H]⁺.

### (c) 4-(4-(3-(5-((6-Acetyl-2,6-diazaspiro[3.4]octan-2-yl)methyl)-6-methoxypyridin-2-yl)-2-chlorophenyl)-3-chloropyridin-2-yl)-2-methoxybenzaldehyde:

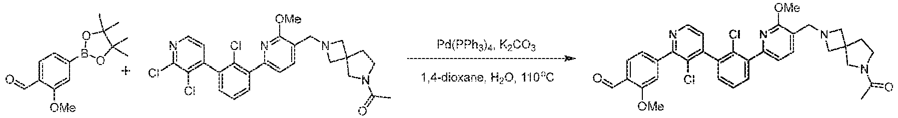

A flask was charged with 2-methoxy-4-(4,4,5-trimethyl-1,3,2-dioxaborolan-2-yl)benzaldehyde (0.06 g, 0.24 mmol), 1-(2-((6-(2-chloro-3-(2,3-dichloropyridin-4-yl)phenyl)-2-methoxypyridin-3-yl)methyl)-2,6-diazaspiro[3.4]octan-6-yl)ethan-1-one (0.10 g, 0.19 mmol), potassium carbonate (0.08 g, 0.56 mmol), and tetrakis(triphenylphosphine)palladium(0) (0.04 g, 0.04 mmol). The flask was purged with nitrogen, then 1,4-dioxane (16 ml) was added, and the reaction was sparged with nitrogen, then 4 ml of water added. The mixture was stirred at 110 °C for 30 min thermally. The mixture was diluted with 50 ml ethyl acetate, then washed with saturated aqueous brine solution (1 x 30 mL), then aqueous layer extracted with ethyl acetate (3 x 30 mL), the combined organic layers were dried over sodium sulfate and concentrated under reduced pressure. The residue was purified by normal phase flash SiO₂ chromatography using a gradient (0-5 % MeOH/dichloromethane) to afford 4-(4-(3-(5-((6-acetyl-2,6-diazaspiro[3.4]octan-2-yl)methyl)-6-methoxypyridin-2-yl)-2-chlorophenyl)-3-chloropyridin-2-yl)-2-methoxybenzaldehyde (0.085 g, 71 % yield). MS: m/z found 631 [M+H] ⁺.

### (d) 1-(2-((6-(2-Chloro-3-(3-chloro-2-(4-((4-hydroxypiperidin-1-yl)methyl)-3-methoxyphenyl)pyridin-4-yl)phenyl)-2-methoxypyridin-3-yl)methyl)-2,6-diazaspiro[3.4]octan-6-yl)ethan-1-one

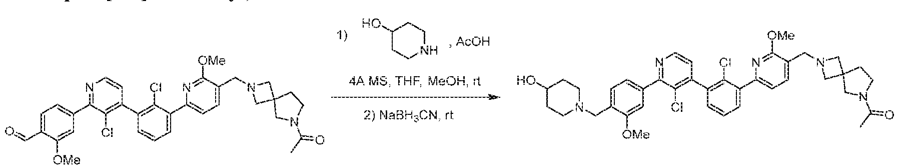

To a mixture of 4-(4-(3-(5-((6-Acetyl-2,6-diazaspiro[3.4]octan-2-yl)methyl)-6-methoxypyridin-2-yl)-2-chlorophenyl)-3-chloropyridin-2-yl)-2-methoxybenzaldehyde (0.09 g, 0.14 mmol), piperidin-4-ol (0.03 g, 0.29 mmol), and 4 A molecular sieve (1 g) in 1:1 THF/MeOH (20 mL) was added acetic acid (0.02 g, 0.29 mmol) and the mixture was stirred at room temperature for 3 hrs. Sodium cyanoborohydride (0.02 g, 0.36 mmol) was then added and the mixture, which was stirred at room temperature for 30 min. The reaction was quenched by addition of 1 ml water, then diluted with 50 ml ethyl acetate, and washed with saturated aqueous brine solution (1 x 30 mL). The aqueous layer was extracted with ethyl acetate (3 x 30 mL), and the combined organic layers dried over sodium sulfate and concentrated under reduced pressure. The residue was purified by preparative HPLC (with 0.05% Formic acid modifier) to give 1-(2-((6-(2-chloro-3-(3-chloro-2-(4-((4-hydroxypiperidin-1-yl)methyl)-3-methoxyphenyl)pyridin-4-yl)phenyl)-2-methoxypyridin-3-yl)methyl)-2,6-diazaspiro[3.4]octan-6-yl)ethan-1-one (0.038 g, 40 % yield). MS: m/z found 716 [M+H]⁺, retention time = 1.63 min (Method D). ¹H NMR (400 MHz, Methanol-*d*₄) δ 8.65 (d, 1H), 8.34 (s, 1H), 7.80 (t, 1H), 7.70 (dd, 1H), 7.55 (t, 2H), 7.49 - 7.33 (m, 4H), 7.30 (dd, 1H), 4.36 (s, 2H), 4.17 (s, 1H), 4.11 (s, 1H), 4.03 (d, 3H), 3.98 (s, 3H), 3.90 - 3.75 (m, 4H), 3.72 (s, 1H), 3.65 - 3.60 (m, 1H), 3.55 (t, 1H), 3.43 (t, 3H), 3.27-3.15 (m, 4H), 2.27 (t, 1H), 2.17 (t, 1H), 2.03 (d, 4H), 1.83 (s, 2H).

### Example 666: 1-(2-(4-(3-Chloro-4-(2-chloro-3-(5-((4-hydroxypiperidin-1-yl)methyl)-6-methoxypyridin-2-yl)phenyl)pyridin-2-yl)-2-methoxybenzyl)-2,6-diazaspiro[3.4]octan-6-yl)ethan-1-one (693)

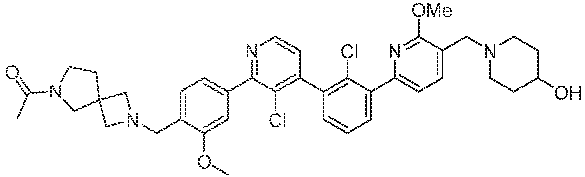

1-(2-(4-(3-Chloro-4-(2-chloro-3-(5-((4-hydroxypiperidin-1-yl)methyl)-6-methoxypyridin-2-yl)phenyl)pyridin-2-yl)-2-methoxybenzyl)-2,6-diazaspiro[3.4]octan-6-yl)ethan-1-one was prepared in a similar fashion to **Example 665,** replacing tert-butyl 2,6-diazaspiro[3.4]octane-6-carboxylate with piperidin-4-ol in step (a) and piperidin-4-ol with 2,6-diazaspiro[3.4]octan-7-one in step (d). MS: m/z found 716 [M+H]⁺; retention time = 1.62 min (Method D). ¹H NMR (400 MHz, Methanol-*d*₄) δ 8.63 (d, 1H), 8.46 (s, 1H), 7.85 (d, 1H), 7.72 (dd, 1H), 7.55 (t, 1H), 7.51 - 7.38 (m, 3H), 7.37 - 7.26 (m, 3H), 4.28 (s, 1H), 4.22 (s, 1H), 4.03 (d, 5H), 3.99 - 3.83 (m, 7H), 3.82 (s, 1H), 3.72 (s, 1H), 3.63 (s, 1H), 3.55 (t, 1H), 3.43 (t, 1H), 3.22 (s, 2H), 2.86 (s, 2H), 2.28 (t, 1H), 2.17 (t, 1H), 2.06 - 1.92 (m, 5H), 1.77 - 1.69 (m, 2H).

### Example 667: 1-((3'-Chloro-2'-(2-chloro-3-(5-((4-hydroxypiperidin-1-yl)methyl)-6-methoxypyridin-2-yl)phenyl)-6-methoxy-[2,4'-bipyridin]-5-yl)methyl)piperidin-4-ol (759)

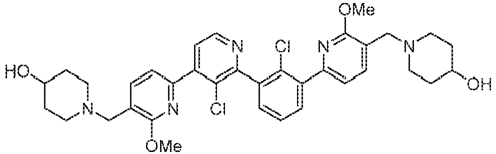

1-((3'-Chloro-2'-(2-chloro-3-(5-((4-hydroxypiperidin-1-yl)methyl)-6-methoxypyridin-2-yl)phenyl)-6-methoxy-[2,4'-bipyridin]-5-yl)methyl)piperidin-4-ol was prepared in a similar fashion to **Example 595,** replacing 1-(4-amino-1-piperidyl)ethanone with piperidin-4-ol in step (b). The resulting formic acid salt was converted to the free base. MS: m/z found 664 [M+H]⁺; retention time = 1.59 min (Method D). ¹H NMR (400 MHz, Methanol-*d*₄) δ 8.60 (d, 1H), 7.81 (d, 1H), 7.79 - 7.68 (m, 3H), 7.53 (t, 1H), 7.48 - 7.38 (m, 2H), 7.25 (d, 1H), 3.98 (d, 6H), 3.67 - 3.55 (m, 6H), 2.84 (d, 4H), 2.27 (t, 4H), 1.85 (dd, 4H), 1.58 (q, 4H).

### Example 668: 1-(2-((6-(3-(5-((6-Acetyl-2,6-diazaspiro[3.4]octan-2-yl)methyl)-3'-chloro-6-methoxy-[2,4'-bipyridin]-2'-yl)-2-chlorophenyl)-2-methoxypyridin-3-yl)methyl)-2,6-diazaspiro[3.4]octan-6-yl)ethan-1-one (760)

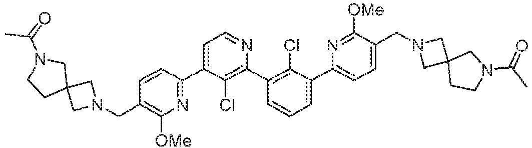

1-(2-((6-(3-(5-((6-Acetyl-2,6-diazaspiro[3.4]octan-2-yl)methyl)-3'-chloro-6-methoxy-[2,4'-bipyridin]-2'-yl)-2-chlorophenyl)-2-methoxypyridin-3-yl)methyl)-2,6-diazaspiro[3.4]octan-6-yl)ethan-1-one was prepared in a similar fashion to **Example 595,** replacing 1-(4-amino-1-piperidyl)ethanone with 1-(2,6-diazaspiro[3.4]octan-6-yl)ethan-1-one in step (b). The resulting formic acid salt was converted to the free base. MS: m/z found 770 [M+H]⁺, retention time = 1.65 min (Method D). ¹H NMR (400 MHz, Methanol-*d*₄) δ 8.60 (d, 1H), 7.77 - 7.64 (m, 4H), 7.53 (t, 1H), 7.48 - 7.36 (m, 2H), 7.24 (dd, 1H), 3.99 (dd, 6H), 3.73 (t, 4H), 3.65 (s, 2H), 3.56 - 3.48 (m, 4H), 3.42 - 3.34 (m, 10H), 2.19 (t, 2H), 2.14 - 1.97 (m, 8H).

### Example 669: 1-(4-(((6-(2-Chloro-3-(3'-chloro-5-((4-hydroxypiperidin-1-yl)methyl)-6-methoxy-[2,4'-bipyridin]-2'-yl)phenyl)-2-methoxypyridin-3-yl)methyl)amino)piperidin-1-yl)ethan-1-one (761)

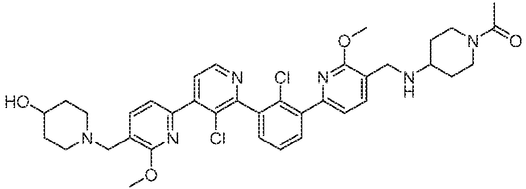

### (a) 1-((2',3'-Dichloro-6-methoxy-[2,4'-bipyridin]-5-yl)methyl)piperidin-4-ol

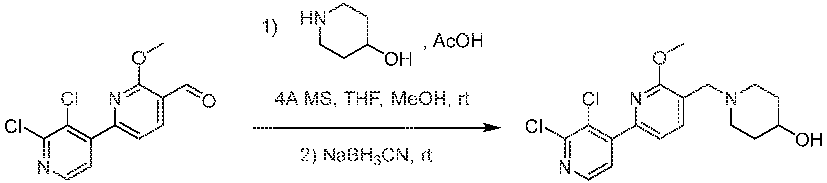

To a mixture of 2',3'-dichloro-6-methoxy-[2,4'-bipyridine]-5-carbaldehyde **(Example 640, step (a))** (0.10 g, 0.35 mmol), piperidin-4-ol (0.07 g, 0.71 mmol) and 4 A molecular sieves (1 g) in 1:1 THF/MeOH (20 mL) was added acetic acid (0.04 g, 0.71 mmol) and the mixture was stirred at room temperature for 3 hrs. Sodium cyanoborohydride (0.06 g, 0.88 mmol) was then added and the mixture was stirred at room temperature for 30 min. The reaction was quenched by addition of 1 ml water. Reaction was then diluted with 50 ml ethyl acetate, washed with saturated aqueous brine solution (1 x 30 mL), then the aqueous layer extracted with ethyl acetate (3 x 30 mL) The combined organic layers were dried over sodium sulfate and concentrated under reduced pressure. The residue was purified by normal phase flash SiO₂ chromatography using a linear gradient (0-10 % MeOH/dichloromethane) to afford 1-((2',3'-dichloro-6-methoxy-[2,4'-bipyridin]-5-yl)methyl)piperidin-4-ol (0.1 g, 77 % yield). MS: m/z found 368 [M+H]⁺.

### (b) 1-(4-(((6-(2-Chloro-3-(4,4,5,5-tetramethyl-1,3,2-dioxaborolan-2-yl)phenyl)-2-methoxy pyridin-3-yl)methyl)amino)piperidin-1-yl)ethan-1-one

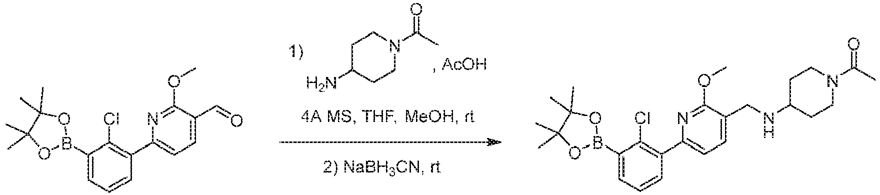

To a mixture of 6-(2-chloro-3-(4,4,5,5-tetramethyl-1,3,2-dioxaborolan-2-yl)phenyl)-2-methoxynicotinaldehyde (0.50 g, 1.34 mmol), 1-(4-aminopiperidin-1-yl)ethan-1-one (0.21 g, 1.47 mmol), and 4 A molecular sieve (1 g) in 1:1 THF/MeOH (20 mL) was added acetic acid (0.1 g, 1.61 mmol) and the mixture was stirred at room temperature for 3 hrs. Sodium cyanoborohydride (0.1 g, 1.61 mmol) was then added and the mixture was stirred at room temperature for 30 min. The reaction was quenched by addition of 1 ml water. The mixture was diluted with 50 ml ethyl acetate, then washed with saturated aqueous brine solution (1 x 30 mL), then aqueous layer extracted with ethyl acetate (3 x 30 mL). The combined organic phases were dried over sodium sulfate and concentrated under reduced pressure. The residue was purified by normal phase flash SiO₂ chromatography using a linear gradient (0-10 % MeOH/dichloromethane) to afford 1-(4-(((6-(2-chloro-3-(4,4,5,5-tetramethyl-1,3,2-dioxaborolan-2-yl)phenyl)-2-methoxypyridin-3-yl)methyl)amino)piperidin-1-yl)ethan-1-one (0.46 g, 69 % yield). MS: m/z found 500 [M+H]⁺.

### (c) 1-(4-(((6-(2-Chloro-3-(3'-chloro-5-((4-hydroxypiperidin-1-yl)methyl)-6-methoxy-[2,4'-bipyridin]-2'-yl)phenyl)-2-methoxypyridin-3-yl)methyl)amino)piperidin-1-yl)ethan-1-one

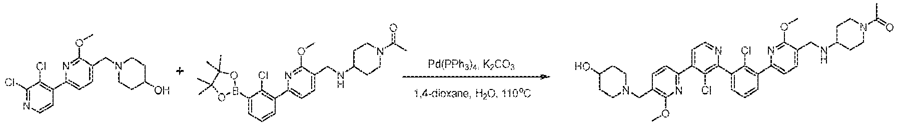

A flask was charged with 1-(4-(((6-(2-chloro-3-(4,4,5,5-tetramethyl-1,3,2-dioxaborolan-2-yl)phenyl)-2-methoxy pyridin-3-yl)methyl)amino)piperidin-1-yl)ethan-1-one (0.07 g, 0.14 mmol), 1-((2',3'-Dichloro-6-methoxy-[2,4'-bipyridin]-5-yl)methyl)piperidin-4-ol (0.05 g, 0.14 mmol), potassium carbonate (0.06 g, 0.41 mmol) and tetrakis(triphenylphosphine)palladium(0) (0.03 g, 0.03 mmol). The flask was purged with nitrogen, then 1,4-dioxane (16 ml) was added and the reaction was sparged with nitrogen, then 4 ml of water added. The mixture was stirred at 110 °C for 30 min. The mixture was diluted with 50 ml ethyl acetate, then washed with saturated aqueous brine solution (1 x 30 mL), then aqueous layer extracted with ethyl acetate (3 x 30 mL), then the combined organic phases dried over sodium sulfate and concentrated under reduced pressure. The residue was purified by preparative HPLC (with 0.05% Formic acid modifier). Acetonitrile was evaporated off, then aqueous layer was basified with 5% sodium carbonate, then extracted with ethyl acetate (5x 10 ml). The combined organic layers was evaporated, then 2 ml of water added and the aqueous portion was lyophilized to give 1-(4-(((6-(2-chloro-3-(3'-chloro-5-((4-hydroxypiperidin-1-yl)methyl)-6-methoxy-[2,4'-bipyridin]-2'-yl)phenyl)-2-methoxypyridin-3-yl)methyl)amino)piperidin-1-yl)ethan-1-one (0.014 g, 15 % yield). MS: m/z found 705 [M+H]⁺, retention time = 1.57 min (Method D). ¹H NMR (400 MHz, Methanol-*d*₄) δ 8.60 (d, 1H), 7.84 - 7.67 (m, 4H), 7.53 (t, 1H), 7.48 - 7.37 (m, 2H), 7.25 (d, 1H), 4.45 (d, 1H), 3.99 (d, 6H), 3.91 (d, 1H), 3.84 (s, 2H), 3.65 - 3.56 (m, 3H), 3.12 (t, 1H), 2.88 - 2.72 (m, 3H), 2.76 - 2.63 (m, 1H), 2.26 (t, 2H), 2.10 - 1.93 (m, 6H), 1.86 - 1.80 (m, 1H), 1.65 - 1.51 (m, 2H), 1.43 - 1.24 (m, 2H).

### Example 670: (R)-1-((3'-Chloro-2'-(2-chloro-3-(5-(((R)-3-hydroxypyrrolidin-1-yl)methyl)-6-methoxypyridin-2-yl)phenyl)-6-methoxy-[2,4'-bipyridin]-5-yl)methyl)pyrrolidin-3-ol (772)

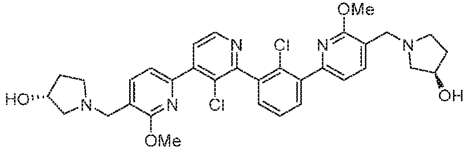

(R)-1-((3'-Chloro-2'-(2-chloro-3-(5-(((R)-3-hydroxypyrrolidin-1-yl)methyl)-6-methoxypyridin-2-yl)phenyl)-6-methoxy-[2,4'-bipyridin]-5-yl)methyl)pyrrolidin-3-ol was prepared in a similar fashion to Example 595, replacing 1-(4-amino-1-piperidyl)ethanone with (R)-pyrrolidin-3-ol in step (b). The resulting formic acid salt was converted to the free base. MS: m/z found 636 [M+H]⁺, retention time = 1.66 min (Method D). ¹H NMR (400 MHz, Methanol-*d*₄) δ 8.59 (d, 1H), 7.82 (d, 1H), 7.78 - 7.66 (m, 3H), 7.52 (t, 1H), 7.47 - 7.36 (m, 2H), 7.24 (d, 1H), 4.38 - 4.28 (m, 2H), 3.98 (d, 6H), 3.76 - 3.62 (m, 4H), 2.89 - 2.73 (m, 4H), 2.64 - 2.50 (m, 4H), 2.21 - 2.07 (m, 2H), 1.77 - 1.64 (m, 2H).

### Example 671: (S)-1-((3'-Chloro-2'-(2-chloro-3-(5-(((S)-3-hydroxypyrrolidin-1-yl)methyl)-6-methoxypyridin-2-yl)phenyl)-6-methoxy-[2,4'-bipyridin]-5-yl)methyl)pyrrolidin-3-ol (773)

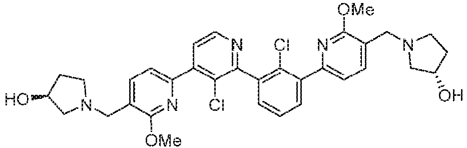

(S)-1-((3'-Chloro-2'-(2-chloro-3-(5-(((S)-3-hydroxypyrrolidin-1-yl)methyl)-6-methoxypyridin-2-yl)phenyl)-6-methoxy-[2,4'-bipyridin]-5-yl)methyl)pyrrolidin-3-ol was prepared in a similar fashion to **Example 595,** replacing 1-(4-amino-1-piperidyl)ethanone with (S)-pyrrolidin-3-ol in step (b). The resulting formic acid salt was converted to the free base. MS: m/z found 636 [M+H] ⁺, retention time = 1.66 min (Method D). ¹H NMR (400 MHz, Methanol-*d*₄) δ 8.59 (d, 1H), 7.82 (d, 1H), 7.78 - 7.66 (m, 3H), 7.52 (t, 1H), 7.47 - 7.36 (m, 2H), 7.24 (d, 1H), 4.38 - 4.28 (m, 2H), 3.98 (d, 6H), 3.77 - 3.63 (m, 4H), 2.90 - 2.75 (m, 4H), 2.65 - 2.51 (m, 4H), 2.21 - 2.07 (m, 2H), 1.77 - 1.66 (m, 2H).

### Example 672: 1-(4-(((3'-Chloro-2'-(2-chloro-3-(5-((4-hydroxypiperidin-1-yl)methyl)-6-methoxypyridin-2-yl)phenyl)-6-methoxy-[2,4'-bipyridin]-5-yl)methyl)amino)piperidin-1-yl)ethan-1-one (774)

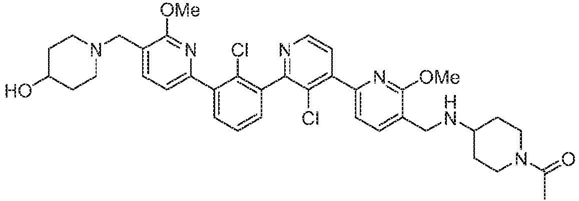

This compound was prepared in a similar fashion to **Example 669,** replacing piperidin-4-ol with 1-(4-aminopiperidin-1-yl)ethan-1-one in step (a) and 1-(4-aminopiperidin-1-yl)ethan-1-one with piperidin-4-ol in step (b). MS: m/z found 705 [M+H]⁺; ¹H NMR (400 MHz, Methanol-*d*₄) δ 8.58 (dd, 1H), 7.77 (d, 1H), 7.76 - 7.65 (m, 3H), 7.51 (t, 1H), 7.46 - 7.35 (m, 2H), 7.23 (dd, 1H), 4.42 (d, 1H), 4.02 - 3.92 (m, 6H), 3.82 (s, 2H), 3.55 (s, 2H), 3.09 (t, 1H), 2.87 - 2.63 (m, 4H), 2.24 (t, 3H), 2.08 - 1.91 (m, 5H), 1.81 (d, 2H), 1.59 - 1.52 (m, 2H), 1.37 - 1.22 (m, 3H).

### Example 673: N-(2-chloro-3-(3-chloro-2-(3-methoxy-4-((methylamino)methyl)phenyl) pyridine-4-yl)phenyl)-5-((methylamino)methyl)picolinamide (694)

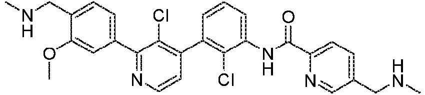

N-(2-Chloro-3-(3-chloro-2-(3-methoxy-4-((methylamino)methyl)phenyl)pyridin-4-yl)phenyl)-5-((methylamino)methyl)picolinamide was prepared as formic acid salt by Reductive Amination Procedure A from N-(2-chloro-3-(3-chloro-2-(4-formyl-3-methoxyphenyl)pyridin-4-yl)phenyl)-5-formylpicolinamide (40 mg, 0.08 mmol) and methanamine solution (33% wt in ethanol, 0.32 mmol). 87% yield. LCMS: m/z found 536.2 [M+H]⁺, retention time = 2.55 min (Method A). ¹H NMR (400 MHz, Methanol-*d₄*): δ 8.73 (d, 1H), 8.72 - 8.60 (m, 2H), 8.53 (s, 2H), 8.30 (d, 1H), 8.09 (dd, 1H), 7.55 (t, 1H), 7.51 (d, 1H), 7.47 (d, 1H), 7.41 (d, 1H), 7.36 (dd, 1H), 7.21 (dd, 1H), 4.26 (s, 2H), 4.07 (s, 2H), 4.00 (s, 3H), 2.74 (s, 3H), 2.58 (s, 3H).

### Example 674: N-(3-(3-Chloro-2-(3-methoxy-4-((methylamino)methyl)phenyl)pyridin-4-yl)-2-methylphenyl)-5-((methylamino)methyl)picolinamide (696)

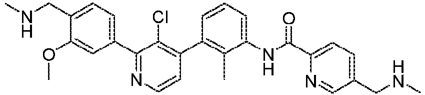

N-(3-(3-Chloro-2-(3-methoxy-4-((methylamino)methyl)phenyl)pyridin-4-yl)-2-methylphenyl)-5-((methylamino)methyl)picolinamide was prepared by Reductive Amination Procedure A from N-(3-(3-chloro-2-(4-formyl-3-methoxyphenyl)pyridin-4-yl)-2-methylphenyl)-5-formylpicolinamide (50 mg, 0.10 mmol) and methanamine solution (33% wt in ethanol, 0.41 mmol). 92% yield. LCMS: m/z found 516.2 [M+H]⁺, retention time = 1.46 min (Method D). ¹H NMR (400 MHz, Methanol-*d*₄): δ 8.72 - 8.65 (m, 1H), 8.60 (d, 1H), 8.21 (d, 1H), 8.05 - 7.91 (m, 2H), 7.46 - 7.35 (m, 3H), 7.31 - 7.22 (m, 2H), 7.11 (dd, 1H), 3.93 (s, 3H), 3.85 (s, 2H), 3.82 (s, 2H), 2.45 - 2.36 (m, 6H), 2.18 (s, 3H).

### Example 675: N-(3-(3-Chloro-2-(3-(difluoromethoxy)-4-(((((S)-5-oxopyrrolidin-2-yl)methyl)amino)methyl)phenyl)pyridin-4-yl)-2-methylphenyl)-5-(((((S)-5-oxopyrrolidin-2-yl)methyl)amino)methyl)picolinamide (725)

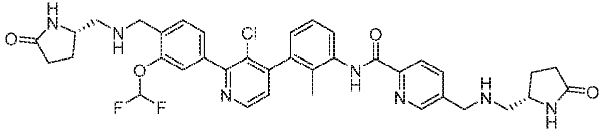

### (a) N-(3-(3-Chloro-2-(3-(difluoromethoxy)-4-formylphenyl)pyridin-4-yl)-2-methylphenyl)-5-formylpicolinamide

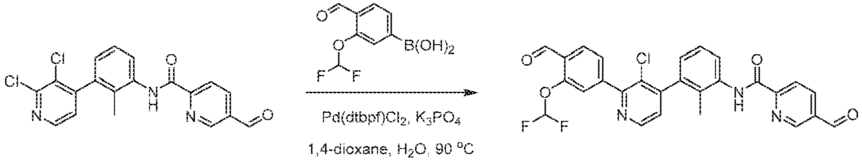

To a mixture of N-(3-(2,3-dichloropyridin-4-yl)-2-methylphenyl)-5-formylpicolinamide **(Example 628, step (c))** (50 mg, 0.13 mmol), (3-(difluoromethoxy)-4-formyl-phenyl)boronic acid (28 mg, 0.13 mmol) and potassium phosphate (82 mg, 0.39 mmol) in degassed 1,4-dioxane/water (6:1) was added [1,1'-bis(di-tert-butylphosphino)ferrocene]dichloropalladium(II) (8 mg, 0.01 mmol) and the mixture was stirred at 90 °C for 2 hrs. Water was added and the mixture was extracted into EtOAc three times. The combined organic layers were dried over anhydrous sodium sulfate, concentrated, and purified on silica gel column to provide N-(3-(3-chloro-2-(3-(difluoromethoxy)-4-formylphenyl)pyridin-4-yl)-2-methylphenyl)-5-formylpicolinamide (60 mg, 89%). LCMS m/z found 522.1 [M+H]⁺.

### (b) N-(3-(3-Chloro-2-(3-(difluoromethoxy)-4-(((((S)-5-oxopyrrolidin-2-yl)methyl)amino)methyl)phenyl)pyridin-4-yl)-2-methylphenyl)-5-(((((S)-5-oxopyrrolidin-2-yl)methyl)amino)methyl)picolinamide

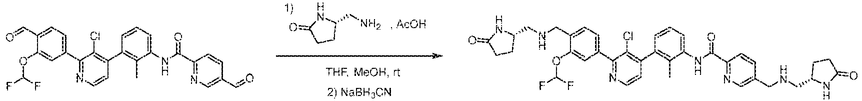

A mixture of N-(3-(3-chloro-2-(3-(difluoromethoxy)-4-formylphenyl)pyridin-4-yl)-2-methylphenyl)-5-formylpicolinamide (30 mg, 0.06 mmol), (S)-5-(aminomethyl)pyrrolidin-2-one (26 mg, 0.23 mmol) and acetic acid (3 uL, 3 mg, 0.06 mmol) in MeOH/THF (1:1) was stirred for 1 hour at room temperature, and then sodium cyanoborohydride (14 mg, 0.23 mmol) was added. The mixture was stirred for an additional 30 min, quenched with water, and directly purified by reversed phase chromatography to give the product as formic acid salt. The collected fractions were combined and acetonitrile was removed under vacuum. The concentrated aqueous solution was neutralized with aqueous sodium carbonate and the product was extracted to dichloromethane three times. The combined organic layers were dried over anhydrous magnesium sulfate and concentrated to give N-(3-(3-chloro-2-(3-(difluoromethoxy)-4-(((((S)-5-oxopyrrolidin-2-yl)methyl)amino)methyl)phenyl)pyridin-4-yl)-2-methylphenyl)-5-(((((S)-5-oxopyrrolidin-2-yl)methyl)amino)methyl)picolinamide as the free base (25 mg, 54%). LCMS: m/z found 718.3 [M+H]⁺, retention time = 1.54 min (Method D). ¹H NMR (400 MHz, Methanol-*d*₄): δ 8.70 (d, 1H), 8.63 (d, 1H), 8.21 (d, 1H), 8.03 (dd, 1H), 7.99 (d, 1H), 7.63 - 7.56 (m, 2H), 7.51 (s, 1H), 7.44 - 7.35 (m, 2H), 7.12 (d, 1H), 6.96 (t, 1H), 3.99 - 3.89 (m, 4H), 3.87 - 3.77 (m, 2H), 2.77 - 2.62 (m, 4H), 2.38 - 2.22 (m, 6H), 2.17 (s, 3H), 1.88 - 1.76 (m, 2H).

### Example 676: N-(3-(3-Chloro-2-(3-fluoro-5-methoxy-4-(((((S)-5-oxopyrrolidin-2-yl)methyl)amino)methyl)phenyl)pyridin-4-yl)-2-methylphenyl)-5-(((((S)-5-oxopyrrolidin-2-yl)methyl)amino)methyl)picolinamide (726)

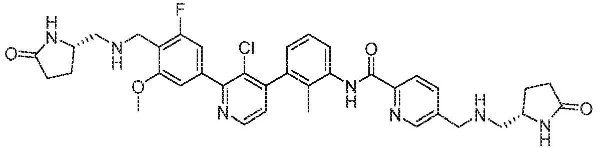

### (a) N-(3-(3-chloro-2-(3-fluoro-4-formyl-5-methoxyphenyl)pyridin-4-yl)-2-methylphenyl)-5-formylpicolinamide

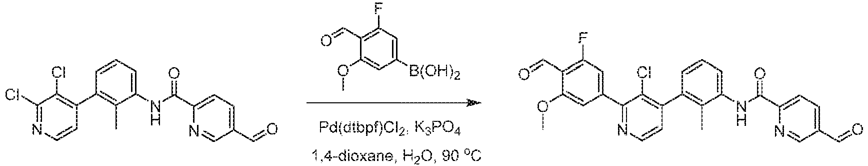

To a mixture of N-(3-(2,3-dichloropyridin-4-yl)-2-methylphenyl)-5-formylpicolinamide **(Example 628, step (c))** (30 mg, 0.08 mmol), (3-fluoro-4-formyl-5-methoxy-phenyl)boronic acid (15 mg, 0.08 mmol) and potassium phosphate (49 mg, 0.23 mmol) in degassed 1,4-dioxane/water (6:1) was added [1,1'-bis(di-tert-butylphosphino)ferrocene]dichloropalladium(II) (5 mg, 0.01 mmol) and the mixture was stirred at 90 °C overnight. Water was added and the mixture was extracted into EtOAc three times. The combined organic layers were dried over anhydrous sodium sulfate, concentrated, and purified on silica gel column to provide N-(3-(3-chloro-2-(3-fluoro-4-formyl-5-methoxyphenyl)pyridin-4-yl)-2-methylphenyl)-5-formylpicolinamide (13 mg, 33%). LCMS m/z found 504.1 [M+H]⁺.

### (b) N-(3-(3-Chloro-2-(3-fluoro-5-methoxy-4-(((((S)-5-oxopyrrolidin-2-yl)methyl)amino)methyl)phenyl)pyridin-4-yl)-2-methylphenyl)-5-(((((S)-5-oxopyrrolidin-2-yl)methyl)amino)methyl)picolinamide

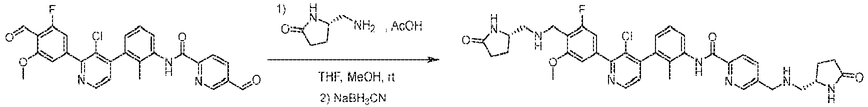

A mixture of N-(3-(3-chloro-2-(3-fluoro-4-formyl-5-methoxyphenyl)pyridin-4-yl)-2-methylphenyl)-5-formylpicolinamide (10 mg, 0.02 mmol), (S)-5-(aminomethyl)pyrrolidin-2-one (9 mg, 0.08 mmol) and acetic acid (1 uL, 1 mg, 0.02 mmol) in MeOH/THF (1:1) was stirred for 1 hour at room temperature, and then sodium cyanoborohydride (5 mg, 0.08 mmol) was added. The mixture was stirred for an additional 30 min, quenched with water, and directly purified by reversed phase chromatography to give the product as formic acid salt. The collected fractions were combined and acetonitrile was removed under vacuum. The concentrated aqueous solution was neutralized with aqueous sodium carbonate and the product was extracted to dichloromethane three times. The combined organic layers were dried over anhydrous magnesium sulfate and concentrated to give N-(3-(3-chloro-2-(3-fluoro-5-methoxy-4-(((((S)-5-oxopyrrolidin-2-yl)methyl)amino)methyl)phenyl)pyridin-4-yl)-2-methylphenyl)-5-(((((S)-5-oxopyrrolidin-2-yl)methyl)amino)methyl)picolinamide as the free base (6 mg, 43%). LCMS: m/z found 700.3 [M+H]⁺, retention time = 1.49 min (Method D).

### Example 677: (S)-N-(3-(2-(4-(((1-acetylpiperidin-4-yl)amino)methyl)-3-methoxyphenyl)-3-chloropyridin-4-yl)-2-methylphenyl)-5-((((5-oxopyrrolidin-2-yl)methyl)amino)methyl)picolinamide (747)

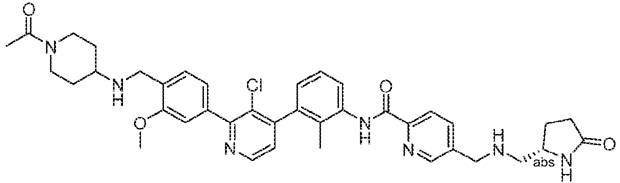

### (a) (S)-N-(3-(2,3-Dichloropyridin-4-yl)-2-methylphenyl)-5-((((5-oxopyrrolidin-2-yl)methyl)amino)methyl)picolinamide

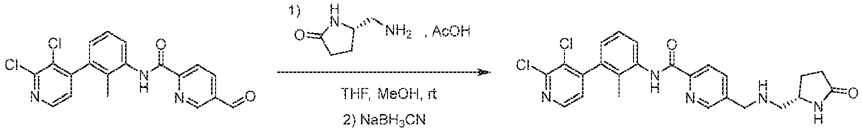

A mixture of N-(3-(2,3-dichloropyridin-4-yl)-2-methylphenyl)-5-formylpicolinamide **(Example 628, step (c))** (300 mg, 0.78 mmol), (S)-5-(aminomethyl)pyrrolidin-2-one (177 mg, 1.55 mmol) and acetic acid (44 uL, 47 mg, 0.78 mmol) in MeOH/THF (1:1) was stirred for 1 hour at room temperature. Sodium cyanoborohydride (98 mg, 1.55 mmol) was added, the mixture was stirred for an additional 2 hrs and quenched with water. Upon concentration, the crude was extracted into dichloromethane three times. The combined organic layers were dried anhydrous magnesium sulfate, concentrated under vacuum to give (S)-N-(3-(2,3-dichloropyridin-4-yl)-2-methylphenyl)-5-((((5-oxopyrrolidin-2-yl)methyl)amino)methyl)picolinamide that was used directly in the next step without further purification. LCMS: m/z found 484.1 [M+H]⁺, retention time = 0.74 min (Method B).

### (b) tert-Butyl (S)-((6-((3-(2,3-dichloropyridin-4-yl)-2-methylphenyl)carbamoyl)pyridin-3-yl)methyl)((5-oxopyrrolidin-2-yl)methyl)carbamate

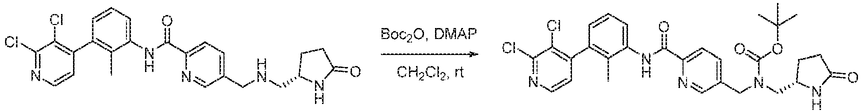

To a solution of (S)-N-(3-(2,3-dichloropyridin-4-yl)-2-methylphenyl)-5-((((5-oxopyrrolidin-2-yl)methyl)amino)methyl)picolinamide (300 mg, 0.62 mmol) in dichloromethane was added di-tert-butyl dicarbonate (149 mg, 0.68 mmol) and catalytic amount of DMAP (8 mg, 0.06 mmol). The mixture was stirred for 2 hours, concentrated in vacuo, and purified on silica gel column using MeOH in dichloromethane (0 to 10% gradient) as eluent to give tert-butyl (S)-((6-((3-(2,3-dichloropyridin-4-yl)-2-methylphenyl)carbamoyl)pyridin-3-yl)methyl)((5-oxopyrrolidin-2-yl)methyl)carbamate. LCMS: m/z found 584.2 [M+H]⁺, retention time = 1.02 min (Method B).

### (c) tert-Butyl (S)-((6-((3-(3-chloro-2-(4-formyl-3-methoxyphenyl)pyridin-4-yl)-2-methylphenyl)carbamoyl)pyridin-3-yl)methyl)((5-oxopyrrolidin-2-yl)methyl)carbamate

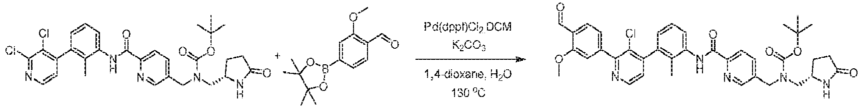

To a solution of tert-butyl (S)-((6-((3-(2,3-dichloropyridin-4-yl)-2-methylphenyl)carbamoyl)pyridin-3-yl)methyl)((5-oxopyrrolidin-2-yl)methyl)carbamate (280 mg, 0.48 mmol), 2-methoxy-4-(4,4,5,5-tetramethyl-1,3,2-dioxaborolan-2-yl)benzaldehyde (126 mg, 0.48 mmol) and potassium carbonate (199 mg, 1.44 mmol) in degassed 1,4-dioxane/water (6:1) was added [1,1'- bis(diphenylphosphino)ferrocene]dichloropalladium(II) complex with dichloromethane (39 mg, 0.05 mmol) and the mixture was stirred at 130 °C for 2 hrs. Water was added and the mixture was extracted into EtOAc three times. The combined organic layers were dried over anhydrous sodium sulfate, concentrated, and purified on silica gel column to provide tert-butyl (S)-((6-((3-(3-chloro-2-(4-formyl-3-methoxyphenyl)pyridin-4-yl)-2-methylphenyl)carbamoyl)pyridin-3-yl)methyl)((5-oxopyrrolidin-2-yl)methyl)carbamate (280 mg, 85%). LCMS: m/z found 684.3 [M+H]⁺, retention time = 1.00 min (Method B).

### (d) tert-Butyl (S)-((6-((3-(2-(4-(((1-acetylpiperidin-4-yl)amino)methyl)-3-methoxyphenyl)-3-chloropyridin-4-yl)-2-methylphenyl)carbamoyl)pyridin-3-yl)methyl)((5-oxopyrrolidin-2-yl)methyl)carbamate

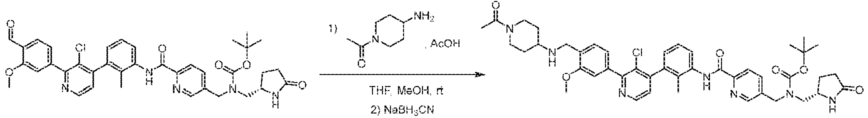

A mixture of tert-butyl (S)-((6-((3-(3-chloro-2-(4-formyl-3-methoxyphenyl)pyridin-4-yl)-2-methylphenyl)carbamoyl)pyridin-3-yl)methyl)((5-oxopyrrolidin-2-yl)methyl)carbamate (30 mg, 0.04 mmol), 1-(4-aminopiperidin-1-yl)ethan-1-one (12 mg, 0.09 mmol) and acetic acid (3 uL, 3 mg, 0.04 mmol) in MeOH/THF (1:1) was stirred for 1 hour at room temperature. Sodium cyanoborohydride (6 mg, 0.09 mmol) was added, the mixture was stirred for an additional 30 min, quenched with water and directly purified by reversed phase chromatography to give tert-butyl (S)-((6-((3-(2-(4-(((1-acetylpiperidin-4-yl)amino)methyl)-3-methoxyphenyl)-3-chloropyridin-4-yl)-2-methylphenyl)carbamoyl)pyridin-3-yl)methyl)((5-oxopyrrolidin-2-yl)methyl)carbamate (32 mg, 90% yield). LCMS: m/z found 810.4 [M+H]⁺, retention time = 2.31 min (Method D).

### (e) (S)-N-(3-(2-(4-(((1-Acetylpiperidin-4-yl)amino)methyl)-3-methoxyphenyl)-3-chloropyridin-4-yl)-2-methylphenyl)-5-((((5-oxopyrrolidin-2-yl)methyl)amino)methyl)picolinamide

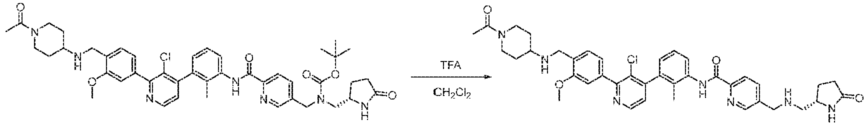

A mixture of tert-butyl (S)-((6-((3-(2-(4-(((1-acetylpiperidin-4-yl)amino)methyl)-3-methoxyphenyl)-3-chloropyridin-4-yl)-2-methylphenyl)carbamoyl)pyridin-3-yl)methyl)((5-oxopyrrolidin-2-yl)methyl)carbamate (30 mg, 0.04 mmol) and dichloromethane / trifluoroacetic acid (1:1) was stirred for 30 min, concentrated and directly purified by reversed phase chromatography. The collected fractions were combined and acetonitrile was removed under vacuum. The concentrated aqueous solution was neutralized with aqueous sodium carbonate and the product was extracted to dichloromethane three times. The combined organic layers were dried over anhydrous magnesium sulfate and concentrated to give (S)-N-(3-(2-(4-(((1-acetylpiperidin-4-yl)amino)methyl)-3-methoxyphenyl)-3-chloropyridin-4-yl)-2-methylphenyl)-5-((((5-oxopyrrolidin-2-yl)methyl)amino)methyl)picolinamide as the free base (19 mg, 73% yield). LCMS: m/z found 710.3 [M+H]⁺, retention time = 1.58 min (Method D). ¹H NMR (400 MHz, Methanol-*d*₄)*:* δ 8.70 (s, 1H), 8.60 (d, 1H), 8.20 (d, 1H), 8.08 - 8.01 (m, 1H), 7.99 (d, 1H), 7.46 - 7.33 (m, 3H), 7.32 - 7.22 (m, 2H), 7.11 (d, 1H), 4.47 (d, 1H), 3.96 - 3.86 (m, 8H), 3.86 - 3.77 (m, 1H), 3.17 - 3.10 (m, 1H), 2.80 - 2.62 (m, 4H), 2.37 - 2.30 (m, 2H), 2.30 - 2.22 (m, 1H), 2.18 (s, 3H), 2.10 (s, 3H), 2.06 - 1.93 (m, 2H), 1.87 - 1.77 (m, 1H), 1.43 - 1.30 (m, 2H).

### Example 678: (S)-N-(3-(3-chloro-2-(3-methoxy-4-(((tetrahydro-2H-pyran-4-yl)amino)methyl)phenyl)pyridin-4-yl)-2-methylphenyl)-5-((((5-oxopyrrolidin-2-yl)methyl)amino)methyl)picolinamide (748)

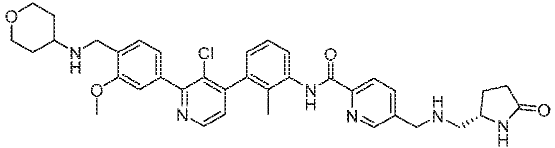

### (a) tert-Butyl (S)-((6-((3-(3-chloro-2-(3-methoxy-4-(((tetrahydro-2H-pyran-4-yl)amino)methyl)phenyl)pyridin-4-yl)-2-methylphenyl)carbamoyl)pyridin-3-yl)methyl)((5-oxopyrrolidin-2-yl)methyl)carbamate

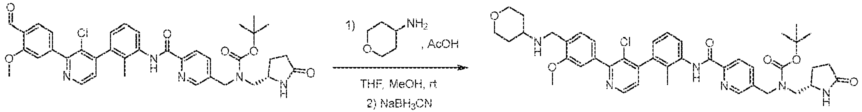

A mixture of tert-butyl (S)-((6-((3-(3-chloro-2-(4-formyl-3-methoxyphenyl)pyridin-4-yl)-2-methylphenyl)carbamoyl)pyridin-3-yl)methyl)((5-oxopyrrolidin-2-yl)methyl)carbamate **(Example 677, step (c))** (30 mg, 0.04 mmol), tetrahydro-2H-pyran-4-amine (9 mg, 0.09 mmol) and acetic acid (3 uL, 3 mg, 0.04 mmol) in MeOH/THF (1:1) was stirred for 1 hour at room temperature. Sodium cyanoborohydride (6 mg, 0.09 mmol) was added, the mixture was stirred for an additional 30 min, quenched with water and directly purified by reversed phase chromatography to give tert-butyl (S)-((6-((3-(3-chloro-2-(3-methoxy-4-(((tetrahydro-2H-pyran-4-yl)amino)methyl)phenyl)pyridin-4-yl)-2-methylphenyl)carbamoyl)pyridin-3-yl)methyl)((5-oxopyrrolidin-2-yl)methyl)carbamate (27 mg, 80%) . LCMS: m/z found 769.3 [M+H]⁺, retention time = 2.38 min (Method D).

### (b) (S)-N-(3-(3-Chloro-2-(3-methoxy-4-(((tetrahydro-2H-pyran-4-yl)amino)methyl)phenyl)pyridin-4-yl)-2-methylphenyl)-5-((((5-oxopyrrolidin-2-yl)methyl)amino)methyl)picolinamide

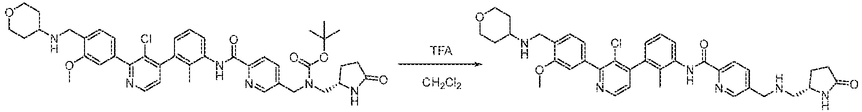

A mixture of tert-butyl (S)-((6-((3-(3-chloro-2-(3-methoxy-4-(((tetrahydro-2H-pyran-4-yl)amino)methyl)phenyl)pyridin-4-yl)-2-methylphenyl)carbamoyl)pyridin-3-yl)methyl)((5-oxopyrrolidin-2-yl)methyl)carbamate (30 mg, 0.04 mmol) and dichloromethane / trifluoroacetic acid (1:1) was stirred for 30 min, concentrated and directly purified by reversed phase chromatography. The collected fractions were combined and acetonitrile was removed under vacuum. The concentrated aqueous solution was neutralized with aqueous sodium carbonate and the product was extracted to dichloromethane three times. The combined organic layers were dried over anhydrous magnesium sulfate and concentrated to give (S)-N-(3-(3-chloro-2-(3-methoxy-4-(((tetrahydro-2H-pyran-4-yl)amino)methyl)phenyl)pyridin-4-yl)-2-methylphenyl)-5-((((5-oxopyrrolidin-2-yl)methyl)amino)methyl)picolinamide as the free base (18 mg, 69%). LCMS: m/z found 669.3 [M+H]⁺, retention time = 1.61 min (Method D). ¹H NMR (400 MHz, Methanol-*d*₄): δ 8.70 (s, 1H), 8.60 (d, 1H), 8.20 (d, 1H), 8.03 (dd, 1H), 8.01 - 7.95 (m, 1H), 7.40 (dd, 3H), 7.30 - 7.22 (m, 2H), 7.11 (d, 1H), 3.99 - 3.95 (m, 1H), 3.95 - 3.91 (m, 6H), 3.90 - 3.87 (m, 2H), 3.85 - 3.78 (m, 1H), 3.43 - 3.35 (m, 2H), 2.77 - 2.60 (m, 3H), 2.37 - 2.30 (m, 2H), 2.29 - 2.22 (m, 1H), 2.18 (s, 3H), 1.95 - 1.86 (m, 2H), 1.86 - 1.77 (m, 1H), 1.53 - 1.39 (m, 2H).

### Example 679: (S)-N-(3-(3-Chloro-2-(4-(((3-fluoropropyl)amino)methyl)-3-methoxyphenyl)pyridin-4-yl)-2-methylphenyl)-5-((((5-oxopyrrolidin-2-yl)methyl)amino)methyl)picolinamide (749)

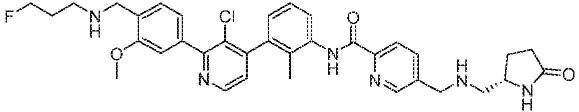

### (a) tert-Butyl (S)-((6-((3-(3-chloro-2-(4-(((3-fluoropropyl)amino)methyl)-3-methoxyphenyl)pyridin-4-yl)-2-methylphenyl)carbamoyl)pyridin-3-yl)methyl)((5-oxopyrrolidin-2-yl)methyl)carbamate

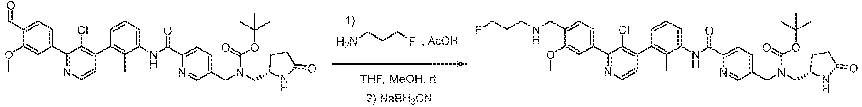

A mixture of tert-butyl (S)-((6-((3-(3-chloro-2-(4-formyl-3-methoxyphenyl)pyridin-4-yl)-2-methylphenyl)carbamoyl)pyridin-3-yl)methyl)((5-oxopyrrolidin-2-yl)methyl)carbamate **(Example 677, step (c))** (30 mg, 0.04 mmol), 3-fluoropropan-1-amine (7 mg, 0.09 mmol) and acetic acid (3 uL, 3 mg, 0.04 mmol) in MeOH/THF (1:1) was stirred for 1 hour at room temperature. Sodium cyanoborohydride (6 mg, 0.09 mmol) was added, the mixture was stirred for an additional 30 min, quenched with water and directly purified by reversed phase chromatography to give tert-butyl (S)-((6-((3-(3-chloro-2-(4-(((3-fluoropropyl)amino)methyl)-3-methoxyphenyl)pyridin-4-yl)-2-methylphenyl)carbamoyl)pyridin-3-yl)methyl)((5-oxopyrrolidin-2-yl)methyl)carbamate (27 mg, 82%). LCMS: m/z found 745.3 [M+H]⁺, retention time = 2.43 min (Method D).

### (b) (S)-N-(3-(3-Chloro-2-(4-(((3-fluoropropyl)amino)methyl)-3-methoxyphenyl)pyridin-4-yl)-2-methylphenyl)-5-((((5-oxopyrrolidin-2-yl)methyl)amino)methyl)picolinamide

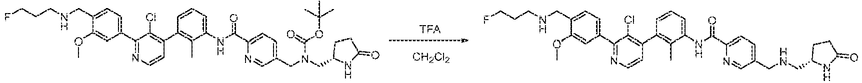

A mixture of tert-butyl (S)-((6-((3-(3-chloro-2-(4-(((3-fluoropropyl)amino)methyl)-3-methoxyphenyl)pyridin-4-yl)-2-methylphenyl)carbamoyl)pyridin-3-yl)methyl)((5-oxopyrrolidin-2-yl)methyl)carbamate (20 mg, 0.03 mmol) and dichloromethane / trifluoroacetic acid (1:1) was stirred for 30 min, concentrated and directly purified by reversed phase chromatography. The collected fractions were combined and acetonitrile was removed under vacuum. The concentrated aqueous solution was neutralized with aqueous sodium carbonate and the product was extracted to dichloromethane three times. The combined organic layers were dried over anhydrous magnesium sulfate and concentrated to give tert-butyl (S)-((6-((3-(3-chloro-2-(4-(((3-fluoropropyl)amino)methyl)-3-methoxyphenyl)pyridin-4-yl)-2-methylphenyl)carbamoyl)pyridin-3-yl)methyl)((5-oxopyrrolidin-2-yl)methyl)carbamate as the free base (12 mg, 68%). LCMS: m/z found 645.3 [M+H]⁺, retention time = 1.65 min (Method D). ¹H NMR (400 MHz, Acetonitrile-*d*₃): δ 10.14 (s, 1H), 8.73 - 8.47 (m, 2H), 8.31 - 8.09 (m, 2H), 7.97 (dd, 1H), 7.39 (dt, 2H), 7.33 - 7.19 (m, 3H), 7.06 (dd, 1H), 6.15 (s, 1H), 4.59 (t, 1H), 4.47 (t, 1H), 3.97 - 3.81 (m, 5H), 3.78 (s, 2H), 3.71 - 3.63 (m, 1H), 2.73 - 2.62 (m, 3H), 2.55 (dd, 1H), 2.24 - 2.07 (m, 6H), 1.92 - 1.67 (m, 3H).

### Example 680: (S)-N-(3-(3-Chloro-2-(4-(((2-hydroxyethyl)amino)methyl)-3-methoxyphenyl)pyridin-4-yl)-2-methylphenyl)-5-((((5-oxopyrrolidin-2-yl)methyl)amino)methyl)picolinamide (750)

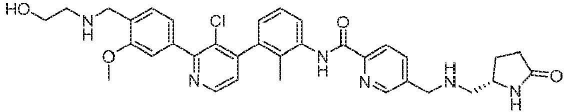

### (a) tert-Butyl (S)-((6-((3-(3-chloro-2-(4-(((2-hydroxyethyl)amino)methyl)-3-methoxyphenyl)pyridin-4-yl)-2-methylphenyl)carbamoyl)pyridin-3-yl)methyl)((5-oxopyrrolidin-2-yl)methyl)carbamate

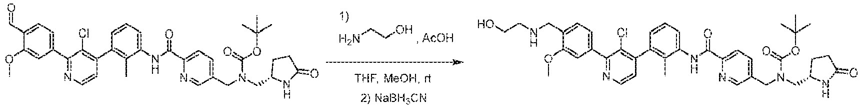

A mixture of tert-butyl (S)-((6-((3-(3-chloro-2-(4-formyl-3-methoxyphenyl)pyridin-4-yl)-2-methylphenyl)carbamoyl)pyridin-3-yl)methyl)((5-oxopyrrolidin-2-yl)methyl)carbamate (Example 677, step (c)) (30 mg, 0.04 mmol), 2-aminoethanol (5 mg, 0.09 mmol) and acetic acid (3 uL, 3 mg, 0.04 mmol) in MeOH/THF (1:1) was stirred for 1 hour at room temperature. Sodium cyanoborohydride (6 mg, 0.09 mmol) was added, the mixture was stirred for an additional 30 min, quenched with water and directly purified by reversed phase chromatography to give tert-butyl (S)-((6-((3-(3-chloro-2-(4-(((2-hydroxyethyl)amino)methyl)-3-methoxyphenyl)pyridin-4-yl)-2-methylphenyl)carbamoyl)pyridin-3-yl)methyl)((5-oxopyrrolidin-2-yl)methyl)carbamate (20 mg, 62%). LCMS: m/z found 729.3 [M+H]⁺, retention time = 2.27 min (Method D).

### (b) (S)-N-(3-(3-Chloro-2-(4-(((2-hydroxyethyl)amino)methyl)-3-methoxyphenyl)pyridin-4-yl)-2-methylphenyl)-5-((((5-oxopyrrolidin-2-yl)methyl)amino)methyl)picolinamide

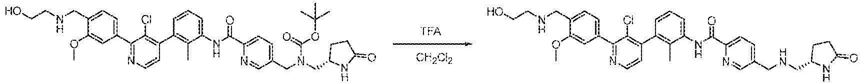

A mixture of tert-butyl (S)-((6-((3-(3-chloro-2-(4-(((2-hydroxyethyl)amino)methyl)-3-methoxyphenyl)pyridin-4-yl)-2-methylphenyl)carbamoyl)pyridin-3-yl)methyl)((5-oxopyrrolidin-2-yl)methyl)carbamate (20 mg, 0.03 mmol) and dichloromethane / trifluoroacetic acid (1:1) was stirred for 30 min, concentrated and directly purified by reversed phase chromatography. The collected fractions were combined and acetonitrile was removed under vacuum. The concentrated aqueous solution was neutralized with aqueous sodium carbonate and the product was extracted to dichloromethane three times. The combined organic layers were dried over anhydrous magnesium sulfate and concentrated to give (S)-N-(3-(3-chloro-2-(4-(((2-hydroxyethyl)amino)methyl)-3-methoxyphenyl)pyridin-4-yl)-2-methylphenyl)-5-((((5-oxopyrrolidin-2-yl)methyl)amino)methyl)picolinamide as the free base (10 mg, 57%). LCMS: m/z found 629.3 [M+H]⁺, retention time = 1.53 min (Method D).

### Example 681: 2-(4-(3-Chloro-4-(2-chloro-3-(6-methoxy-5-((6-(2-methoxyacetyl)-2,6-diazaspiro[3.3]heptan-2-yl)methyl)pyridin-2-yl)phenyl)pyridin-2-yl)-2-methoxybenzyl)-2,6-diazaspiro[3.4]octan-7-one (687)

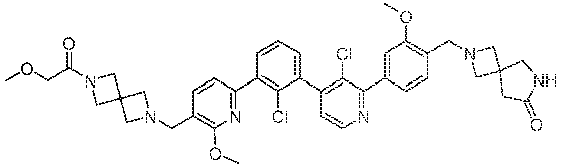

### (a) 2-Methoxy-1-(2,6-diazaspiro[3.3]heptan-2-yl)ethan-1-one trifluoroacetic acid salt

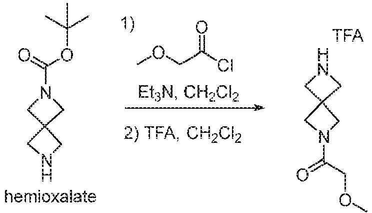

Triethylamine (1.89 mL, 13.56 mmol) was added to a mixture of tert-butyl 2,6-diazaspiro[3.3]heptane-2-carboxylate hemioxalate (0.75 g, 1.54 mmol) in 40 mL dichloromethane. The mixture was cooled in an ice bath. A solution of 2-methoxyacetyl chloride (0.42 mL, 4.62 mmol) in 10 mL dichloromethane was added slowly. After 3 h, the mixture was washed with saturated aqueous sodium bicarbonate (20 mL), aqueous HCl (0.2 M, 20 mL), and saturated aqueous sodium bicarbonate (20 mL). The organics were dried over sodium sulfate, evaporated, and purified by normal phase SiO₂ chromatography (15% to 100% EtOAc/hexanes) to provide tert-butyl 6-(2-methoxyacetyl)-2,6-diazaspiro[3.3]heptane-2-carboxylate (0.59 g, 71%).

A solution of trifluoroacetic acid (1.85 mL, 7.40 mmol) in 3 mL dichloromethane was added to a 0 °C solution of tert-butyl 6-(2-methoxyacetyl)-2,6-diazaspiro[3.3]heptane-2-carboxylate (0.20 g, 0.74 mmol) in 7 mL dichloromethane. After 10 min, the ice bath was removed. After 30 min, the volatiles were removed *in vacuo.* The resulting clear oil was azeotroped twice with toluene (5 mL) to provide crude 2-methoxy-1-(2,6-diazaspiro[3.3]heptan-2-yl)ethan-1-one trifluoroacetic acid salt (0.27 g). ¹H NMR (400 MHz, Methanol-*d*₄) δ 4.47 (s, 2H), 4.23 (d, 6H), 3.97 (s, 2H), 3.37 (d, 3H).

### (b) 1-(6-((6-(2-Chloro-3-(2,3-dichloropyridin-4-yl)phenyl)-2-methoxypyridin-3-yl)methyl)-2,6-diazaspiro[3.3]heptan-2-yl)-2-methoxyethan-1-one

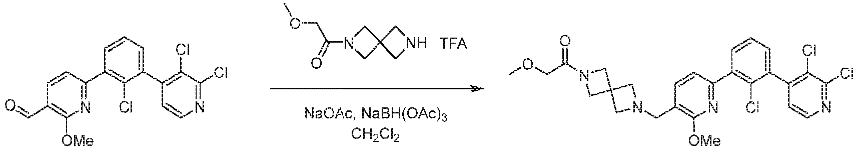

A mixture of 2-methoxy-1-(2,6-diazaspiro[3.3]heptan-2-yl)ethan-1-one trifluoroacetic acid salt (86 mg, 0.30 mmol) and sodium acetate (71 mg, 0.86 mmol) in 0.45 mL dichloromethane was stirred for 10 min at room temperature. After the addition of 6-(2-chloro-3-(2,3-dichloropyridin-4-yl)phenyl)-2-methoxynicotinaldehyde (85 mg, 0.22 mmol), the mixture was stirred for 5 minutes. Sodium triacetoxyborohydride (92 mg, 0.43 mmol) was added. After stirring overnight, the reaction mixture was diluted with EtOAc (20 mL) and washed with saturated aqueous sodium bicarbonate (20 mL). The aqueous layer was extracted with EtOAc (10 mL). The combined organics were washed with brine (10 mL), dried over sodium sulfate, and concentrated. The crude product was purified by normal phase SiO₂ chromatography (1% to 10% methanol/dichloromethane) to provide 1-(6-((6-(2-chloro-3-(2,3-dichloropyridin-4-yl)phenyl)-2-methoxypyridin-3-yl)methyl)-2,6-diazaspiro[3.3]heptan-2-yl)-2-methoxyethan-1-one (61 mg, 51%). MS: m/z found 547.2 and 549.2 [M+H]⁺.

### (c) 4-(3-Chloro-4-(2-chloro-3-(6-methoxy-5-((6-(2-methoxyacetyl)-2,6-diazaspiro[3.3]heptan-2-yl)methyl)pyridin-2-yl)phenyl)pyridin-2-yl)-2-methoxybenzaldehyde

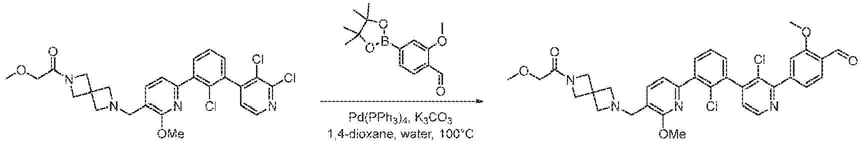

Tetrakis(triphenylphosphine)palladium(0) (10 mg, 0.01 mmol), potassium carbonate (45 mg, 0.33 mmol), 1-(6-((6-(2-chloro-3-(2,3-dichloropyridin-4-yl)phenyl)-2-methoxypyridin-3-yl)methyl)-2,6-diazaspiro[3.3]heptan-2-yl)-2-methoxyethan-1-one (60 mg, 0.11 mmol), and 2-methoxy-4-(4,4,5,5-tetramethyl-1,3,2-dioxaborolan-2-yl)benzaldehyde (32 mg, 0.12 mmol) were suspended in 1,4-dioxane/water (4:1, 2 mL) and the reaction mixture was heated at 100 °C for 30 minutes. Upon cooling, the reaction mixture was diluted with 10 mL water, and extracted with EtOAc (3 x 10 mL). The combined organics were dried over sodium sulfate, then concentrated under reduced pressure. The crude sample was purified by normal phase SiO₂ chromatography (20-100% EtOAc/hexanes) to afford 4-(3-chloro-4-(2-chloro-3-(6-methoxy-5-((6-(2-methoxyacetyl)-2,6-diazaspiro[3.3]heptan-2-yl)methyl)pyridin-2-yl)phenyl)pyridin-2-yl)-2-methoxybenzaldehyde (55 mg, 78%) as a white foam . MS: m/z found 647.3 and 649.3 [M+H]⁺.

### (d) 2-(4-(3-Chloro-4-(2-chloro-3-(6-methoxy-5-((6-(2-methoxyacetyl)-2,6-diazaspiro[3.3]heptan-2-yl)methyl)pyridin-2-yl)phenyl)pyridin-2-yl)-2-methoxybenzyl)-2,6-diazaspiro [3.4] octan-7-one

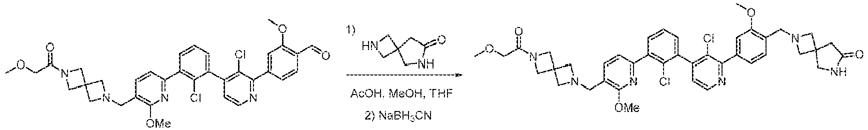

Acetic acid (3 µL, 0.05 mmol) was added to a solution of 2,6-diazaspiro[3.4]octan-7-one (12 mg, 0.09 mmol) and 4-(3-chloro-4-(2-chloro-3-(6-methoxy-5-((6-(2-methoxyacetyl)-2,6-diazaspiro[3.3]heptan-2-yl)methyl)pyridin-2-yl)phenyl)pyridin-2-yl)-2-methoxybenzaldehyde (33 mg, 0.05 mmol) in 0.12 mL THF and 0.12 mL MeOH. After 2h, sodium cyanoborohydride (8 mg, 0.13 mmol) was added. After stirring at room temperature overnight, the mixture was concentrated. The resulting residue was purified by reverse phase HPLC to afford 2-(4-(3-chloro-4-(2-chloro-3-(6-methoxy-5-((6-(2-methoxyacetyl)-2,6-diazaspiro[3.3]heptan-2-yl)methyl)pyridin-2-yl)phenyl)pyridin-2-yl)-2-methoxybenzyl)-2,6-diazaspiro[3.4]octan-7-one (18 mg, 46%) as a formic acid salt. LCMS: m/z found 757.4 [M+H]⁺, retention time = 2.58 min (Method A); ¹H NMR (400 MHz, Methanol-*d*₄) δ 8.65 (dd, 1H), 8.47 (s, 1H), 7.77 - 7.67 (m, 2H), 7.56 (td, 1H), 7.48 - 7.40 (m, 3H), 7.37 - 7.27 (m, 3H), 4.40 (s, 2H), 4.23 (s, 2H), 4.14 (s, 2H), 4.02 (d, 3H), 4.00 - 3.93 (m, 9H), 3.90 (s, 2H), 3.78 (s, 4H), 3.65 (m, 2H), 3.36 (m, 3H), 2.68 (s, 2H).

### Example 682: 1-(6-((6-(3-(2-(4-((6-Acetyl-2,6-diazaspiro[3.3]heptan-2-yl)methyl)-3-methoxyphenyl)-3-chloropyridin-4-yl)-2-chlorophenyl)-2-methoxypyridin-3-yl)methyl)-2,6-diazaspiro[3.3]heptan-2-yl)-2-methoxyethan-1-one (691)

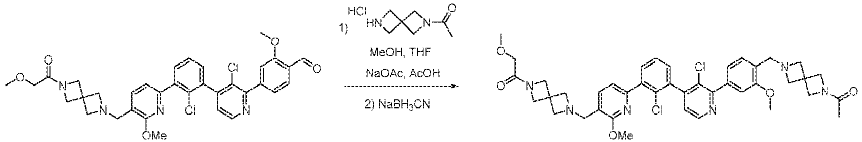

Acetic acid (3 µL, 0.05 mmol) was added to a mixture of sodium acetate (8 mg, 0.10 mmol), 1-(2,6-diazaspiro[3.3]heptan-2-yl)ethan-1-one HCl (17 mg, 0.10 mmol) and 4-(3-chloro-4-(2-chloro-3-(6-methoxy-5-((6-(2-methoxyacetyl)-2,6-diazaspiro[3.3]heptan-2-yl)methyl)pyridin-2-yl)phenyl)pyridin-2-yl)-2-methoxybenzaldehyde (34 mg, 0.05 mmol) in 0.12 mL THF and 0.12 mL MeOH. After 2 h, sodium cyanoborohydride (8 mg, 0.13 mmol) was added. After stirring at room temperature overnight, the mixture was concentrated. The resulting residue was purified by reverse phase HPLC to afford 1-(6-((6-(3-(2-(4-((6-acetyl-2,6-diazaspiro[3.3]heptan-2-yl)methyl)-3-methoxyphenyl)-3-chloropyridin-4-yl)-2-chlorophenyl)-2-methoxypyridin-3-yl)methyl)-2,6-diazaspiro[3.3]heptan-2-yl)-2-methoxyethan-1-one (8 mg, 18%) as a white solid (formic acid salt). LCMS: m/z found 771.4 [M+H]+, retention time = 2.64 min, (Method A); ¹H NMR (400 MHz, Methanol-*d*₄) δ 8.64 (d, 1H), 8.49 (s, 1H), 7.74 - 7.67 (m, 2H), 7.55 (t, 1H), 7.49 - 7.38 (m, 3H), 7.37 - 7.29 (m, 2H), 7.26 (d, 1H), 4.37 (d, 4H), 4.12 (d, 7H), 4.01 (s, 3H), 3.98 (s, 4H), 3.96 (d, 6H), 3.82 (s, 2H), 3.68 (s, 4H), 3.36 (s, 3H), 1.85 (s, 3H).

### Example 683: (S)-5-((((6-(2-Chloro-3-(3-chloro-2-(2-((3-hydroxy-3-methylazetidin-1-yl)methyl)-4-methoxypyrrolo[2,1-f][1,2,4]triazin-6-yl)pyridin-4-yl)phenyl)-2-methoxypyridin-3-yl)methyl)amino)methyl)pyrrolidin-2-one (697)

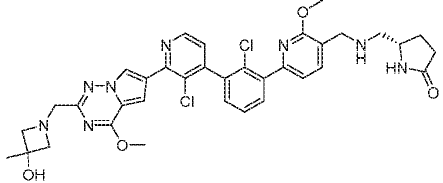

### (a) tert-Butyl (S)-((6-(2-chloro-3-(3-chloro-2-(2-((3-hydroxy-3-methylazetidin-1-yl)methyl)-4-methoxypyrrolo [2,1-f][1,2,4]triazin-6-yl)pyridin-4-yl)phenyl)-2-methoxypyridin-3-yl)methyl)((5-oxopyrrolidin-2-yl)methyl)carbamate

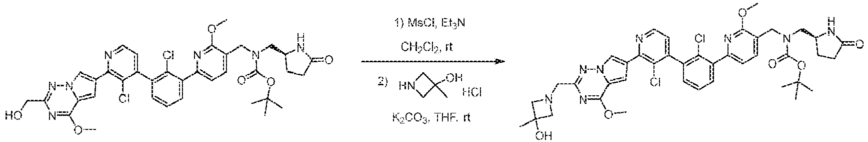

To a solution of tert-butyl (S)-((6-(2-chloro-3-(3-chloro-2-(2-(hydroxymethyl)-4-methoxypyrrolo[2,1-f][1,2,4]triazin-6-yl)pyridin-4-yl)phenyl)-2-methoxypyridin-3-yl)methyl)((5-oxopyrrolidin-2-yl)methyl)carbamate (20 mg, 0.03 mmol) in dichloromethane (0.5 mL), triethylamine (8 uL, 6 mg, 0.05 mmol) and mesyl chloride (4 uL, 6 mg, 0.05 mmol) were added. The reaction was stirred at room temperature for 1 hr. LCMS suggested formation of the desired mesylated product. The solvent was evaporated and the residue was suspended in THF (0.4 mL) followed by addition of potassium carbonate (7 mg, 0.05 mmol) and 3-methylazetidin-3-ol hydrochloride (7 mg, 0.05 mmol). The mixture was stirred at room temperature for 12 h. The solvent was evaporated and the residue was suspended in water. The precipitate formed was collected to afford tert-butyl (S)-((6-(2-chloro-3-(3-chloro-2-(2-((3-hydroxy-3-methylazetidin-1-yl)methyl)-4-methoxypyrrolo[2,1-f][1,2,4triazin-6-yl)pyridin-4-yl)phenyl)-2-methoxypyridin-3-yl)methyl)((5-oxopyrrolidin-2-yl)methyl)carbamate (12 mg, 91%). LCMS: m/z found 803.6 [M+H]⁺.

### (b) (S)-5-((((6-(2-Chloro-3-(3-chloro-2-(2-((3-hydroxy-3-methylazetidin-1-yl)methyl)-4-methoxypyrrolo[2,1-f] [1,2,4]triazin-6-yl)pyridin-4-yl)phenyl)-2-methoxypyridin-3-yl)methyl)amino)methyl)pyrrolidin-2-one

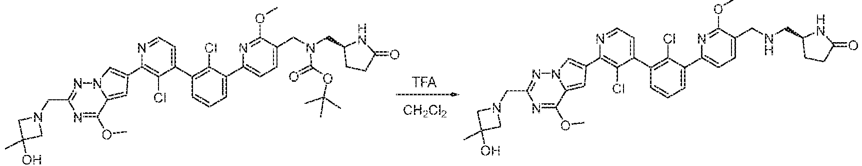

To a solution of tert-butyl (S)-((6-(2-chloro-3-(3-chloro-2-(2-((3-hydroxy-3-methylazetidin-1-yl)methyl)-4-methoxypyrrolo[2,1-f][1,2,4]triazin-6-yl)pyridin-4-yl)phenyl)-2-methoxypyridin-3-yl)methyl)((5-oxopyrrolidin-2-yl)methy)carbamate (12 mg, 0.01 mmol) in dichloromethane (0.3 mL) was added trifluoroacetic acid (0.06 mL, 0.09 g, 0.75 mmol) and the reaction was stirred at room temperature for 1 hr. The solvent was evaporated and the residue was purified by reversed phase HPLC to afford (S)-5-((((6-(2-chloro-3-(3-chloro-2-(2-((3-hydroxy-3-methylazetidin-1-yl)methyl)-4-methoxypyrrolo[2,1-f][1,2,4]triazin-6-yl)pyridin-4-yl)phenyl)-2-methoxypyridin-3-yl)methyl)amino)methyl)pyrrolidin-2-one (5 mg, 47%) as a white solid. LCMS: m/z found 703.4 [M+H]⁺, retention time = 0.65 min (Method B). ¹H NMR (400 MHz, Methanol-*d*₄): δ 8.63 (d, 1H), 8.50 (d, 1H), 7.82 (d, 1H), 7.71 (dd, 1H), 7.61 - 7.53 (m, 2H), 7.40 (dd, 1H), 7.35 - 7.27 (m, 2H), 4.37 (s, 2H), 4.20 (s, 3H), 4.14 (d, 2H), 4.08 (d, 2H), 4.06 (s, 2H), 3.97 (d, 3H), 2.97 (t, 2H), 2.43 - 2.29 (m, 3H), 1.86 (dd, 1H), 1.56 (s, 3H).

### Example 684: 1-(4-(((6-(3-(2-(2-((2-Oxa-6-azaspiro[3.3]heptan-6-yl)methyl)-8-methoxy-[1,2,4]triazolo[1,5-a]pyridin-6-yl)-3-chloropyridin-4-yl)-2-chlorophenyl)-2-methoxypyridin-3-yl)methyl)amino)piperidin-1-yl)ethan-1-one (698)

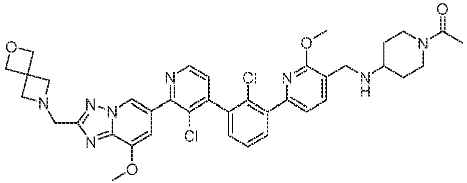

### (a) tert-Butyl ((6-(3-(2-(2-((2-oxa-6-azaspiro[3.3]heptan-6-yl)methyl)-8-methoxy-[1,2,4]triazolo[1,5-a]pyridin-6-yl)-3-chloropyridin-4-yl)-2-chlorophenyl)-2-methoxypyridin-3-yl)methyl)(1-acetylpiperidin-4-yl)carbamate

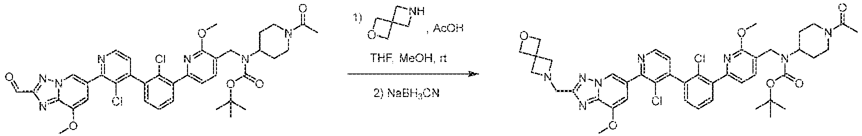

tert-Butyl (1-acetylpiperidin-4-yl)((6-(2-chloro-3-(3-chloro-2-(2-formyl-8-methoxy-[1,2,4]triazolo[1,5-a]pyridin-6-yl)pyridin-4-yl)phenyl)-2-methoxypyridin-3-yl)methyl)carbamate (20 mg, 0.03 mmol), acetic acid (3 uL, 3 mg, 0.05 mmol) and 2-oxa-6-azaspiro[3.3]heptane (5 mg, 0.05 mmol) were dissolved in 1 mL of 1: 1 THF/MeOH and the reaction was stirred at room temperature for 10 minutes. Sodium cyanoborohydride (3 mg, 0.05 mmol) was added in one portion and the resulting mixture was stirred at room temperature for 1 hr. The reaction mixture was concentrated and the residue was suspended in 2 mL water. The precipitate formed was collected by filtration and dried to afford tert-butyl ((6-(3-(2-(2-((2-oxa-6-azaspiro[3.3]heptan-6-yl)methyl)-8-methoxy-[1,2,4]triazolo[1,5-a]pyridin-6-yl)-3-chloropyridin-4-yl)-2-chlorophenyl)-2-methoxypyridin-3-yl)methyl)(1-acetylpiperidin-4-yl)carbamate (15 mg, 68%) as a white solid. LCMS: m/z found 843.5 [M+H]⁺.

### (b) 1-(4-(((6-(3-(2-(2-((2-Oxa-6-azaspiro [3.3] heptan-6-yl)methyl)-8-methoxy-[1,2,4]triazolo [1,5-a] pyridin-6-yl)-3-chloropyridin-4-yl)-2-chlorophenyl)-2-methoxypyridin-3-yl)methyl)amino)piperidin-1-yl)ethan-1-one

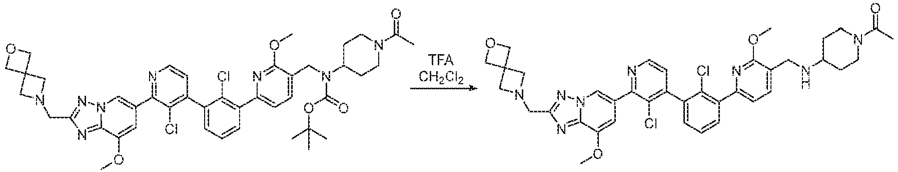

To a solution of tert-butyl ((6-(3-(2-(2-((2-oxa-6-azaspiro[3.3]heptan-6-yl)methyl)-8-methoxy-[1,2,4]triazolo[1,5-a]pyridin-6-yl)-3-chloropyridin-4-yl)-2-chlorophenyl)-2-methoxypyridin-3-yl)methyl)(1-acetylpiperidin-4-yl)carbamate (15 mg, 0.02 mmol) in dichloromethane (0.3 mL) was added trifluoroacetic acid (0.07 mL, 0.10 g, 0.89 mmol) and the reaction was stirred at room temperature for 1 h. The solvent was evaporated and the residue was purified by reversed phase HPLC to afford 1-(4-(((6-(3-(2-(2-((2-oxa-6-azaspiro[3.3]heptan-6-yl)methyl)-8-methoxy-[1,2,4]triazolo[1,5-a]pyridin-6-yl)-3-chloropyridin-4-yl)-2-chlorophenyl)-2-methoxypyridin-3-yl)methyl)amino)piperidin-1-yl)ethan-1-one (2.4 mg, 18%) as a white solid. LCMS: m/z found 743.5 [M+H]⁺, retention time = 0.52 min (Method B). ¹H NMR (400 MHz, Methanol-*d*₄): δ 9.20 (d, 1H), 8.79 (d, 1H), 8.15 (d, 1H), 7.88 (d, 1H), 7.74 (dd, 1H), 7.60 (d, 2H), 7.47 (dd, 1H), 7.34 (d, 1H), 5.00 (s, 2H), 4.63 (d, 1H), 4.57 (s, 2H), 4.25 (s, 3H), 4.22 (s, 2H), 4.07 (s, 7H), 3.74 (s, 2H), 3.34 (d, 2H), 3.20 (t, 1H), 2.68 (d, 1H), 2.21 (t, 2H), 2.13 (s, 3H), 1.48 (d, 1H).

### Example 685: 1-(4-(((6-(2-chloro-3-(3-chloro-2-(2-((((1-hydroxycyclopropyl)methyl)amino)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one (699)

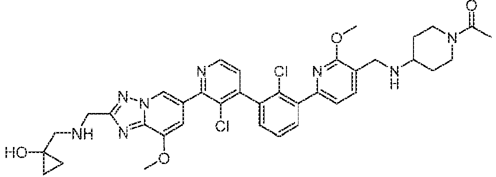

### (a) tert-Butyl (1-acetylpiperidin-4-yl)((6-(2-chloro-3-(3-chloro-2-(2-((((1-hydroxycyclopropyl)methyl)amino)methyl)-8-methoxy-[1,2,4]triazolo[1,5-a]pyridin-6-yl)pyridin-4-yl)phenyl)-2-methoxypyridin-3-yl)methyl)carbamate

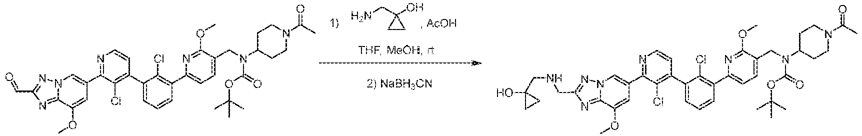

tert-Butyl (1-acetylpiperidin-4-yl)((6-(2-chloro-3-(3-chloro-2-(2-formyl-8-methoxy-[1,2,4]triazolo[1,5-a]pyridin-6-yl)pyridin-4-yl)phenyl)-2-methoxypyridin-3-yl)methyl)carbamate (20 mg, 0.03 mmol), acetic acid (3 uL, 3 mg, 0.05 mmol) and 1-(aminomethyl)cyclopropan-1-ol (5 mg, 0.05 mmol) were dissolved in 1:1 THF/MeOH (1 mL) and the reaction was stirred at room temperature for 10 minutes. Sodium cyanoborohydride (3 mg, 0.05 mmol) was added in one portion and the resulting mixture was stirred at room temperature for 1 h. The reaction mixture was concentrated and the residue was suspended in 2 mL water. The precipitate formed was collected by filtration and dried to afford tert-butyl (1-acetylpiperidin-4-yl)((6-(2-chloro-3-(3-chloro-2-(2-((((1-hydroxycyclopropyl)methyl)amino)methyl)-8-methoxy-[1,2,4]triazolo[1,5-a]pyridin-6-yl)pyridin-4-yl)phenyl)-2-methoxypyridin-3-yl)methyl)carbamate (17 mg, 78%) as a white solid. LCMS: m/z found 831.6 [M+H]⁺.

### (b) 1-(4-(((6-(2-Chloro-3-(3-chloro-2-(2-((((1-hydroxycyclopropyl)methyl)amino)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one

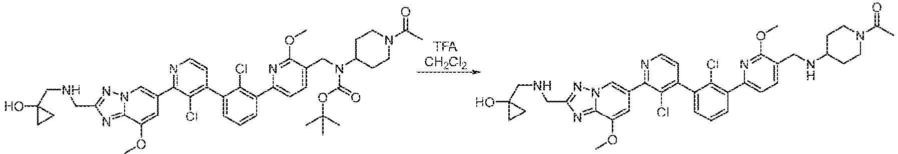

To a solution of tert-butyl (1-acetylpiperidin-4-yl)((6-(2-chloro-3-(3-chloro-2-(2-((((1-hydroxycyclopropyl)methyl)amino)methyl)-8-methoxy-[1,2,4]triazolo[1,5-a]pyridin-6-yl)pyridin-4-yl)phenyl)-2-methoxypyridin-3-yl)methyl)carbamate (17 mg, 0.02 mmol) in dichloromethane (0.3 mL) was added trifluoroacetic acid (0.08 mL, 0.12 g, 1.02 mmol) and the reaction was stirred at room temperature for 1 h. The solvent was evaporated and the residue was purified by preparative HPLC to afford 1-(4-(((6-(2-chloro-3-(3-chloro-2-(2-((((1-hydroxycyclopropyl)methyl)amino)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one (6 mg, 40%) as a white solid. LCMS: m/z found 731.5 [M+H]⁺, retention time = 0.61 min (Method B). ¹H NMR (400 MHz, Methanol-*d*₄): δ 8.81 (d, 1H), 8.72 (d, 1H), 7.86 (d, 1H), 7.73 (dd, 1H), 7.58 (t, 1H), 7.52 (d, 1H), 7.48 - 7.43 (m, 2H), 7.33 (d, 1H), 4.60 (d, 1H), 4.43 (s, 2H), 4.16 (d, 2H), 4.12 (s, 3H), 4.06 (s, 3H), 4.01 (s, 1H), 3.28 - 3.15 (m, 2H), 3.14 (s, 2H), 2.70 (t, 1H), 2.19 (d, 2H), 2.12 (s, 3H), 1.63 - 1.37 (m, 2H), 0.87 - 0.80 (m, 2H), 0.71 - 0.65 (m, 2H).

### Example 686: 1-(4-(((6-(2-Chloro-3-(3-chloro-2-(2-(((3-fluoropropyl)amino)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one (713)

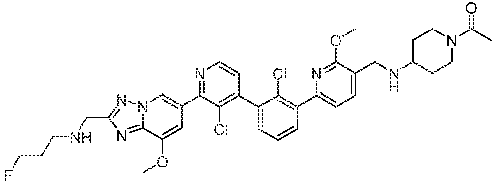

### (a) tert-Butyl (1-acetylpiperidin-4-yl)((6-(2-chloro-3-(3-chloro-2-(2-(((3-fluoropropyl)amino)methyl)-8-methoxy-[1,2,4]triazolo[1,5-a]pyridin-6-yl)pyridin-4-yl)phenyl)-2-methoxypyridin-3-yl)methyl)carbamate

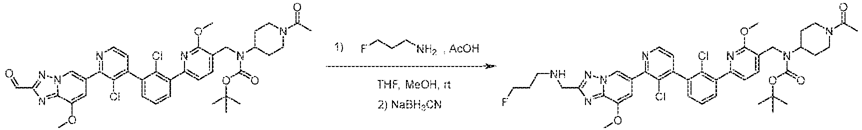

tert-Butyl (1-acetylpiperidin-4-yl)((6-(2-chloro-3-(3-chloro-2-(2-formyl-8-methoxy-[1,2,4]triazolo[1,5-a]pyridin-6-yl)pyridin-4-yl)phenyl)-2-methoxypyridin-3-yl)methyl)carbamate (20 mg, 0.03 mmol), acetic acid (3 uL, 3 mg, 0.05 mmol) and 3-fluoropropan-1-amine (4 mg, 0.05 mmol) were dissolved in 1:1 THF/MeOH (1 mL) and the reaction was stirred at room temperature for 10 minutes. Sodium cyanoborohydride (3 mg, 0.05 mmol) was added in one portion and the resulting mixture was stirred at room temperature for 1 h. Reaction was concentrated and the residue was dissolved in 2 mL water. The precipitate formed was collected by filtration and dried to afford tert-butyl (1-acetylpiperidin-4-yl)((6-(2-chloro-3-(3-chloro-2-(2-(((3-fluoropropyl)amino)methyl)-8-methoxy-[1,2,4]triazolo[1,5-a]pyridin-6-yl)pyridin-4-yl)phenyl)-2-methoxypyridin-3-yl)methyl)carbamate (15 mg, 69%) as a white solid. LCMS: m/z found 821.4 [M+H]⁺.

### (b) 1-(4-(((6-(2-Chloro-3-(3-chloro-2-(2-(((3-fluoropropyl)amino)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one

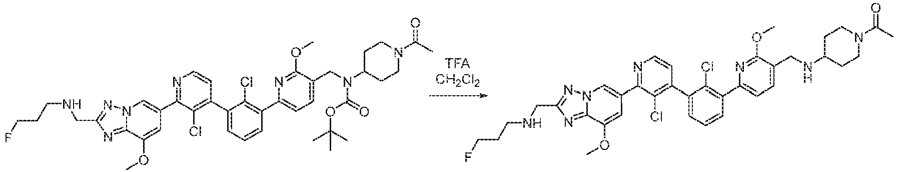

To a solution of tert-butyl (1-acetylpiperidin-4-yl)((6-(2-chloro-3-(3-chloro-2-(2-(((3-fluoropropyl)amino)methyl)-8-methoxy-[1,2,4]triazolo[1,5-a]pyridin-6-yl)pyridin-4-yl)phenyl)-2-methoxypyridin-3-yl)methyl)carbamate (15 mg, 0.02 mmol) in dichloromethane (0.3 mL) was added trifluoroacetic acid (0.07 mL, 0.10 g, 0.89 mmol) and the reaction was stirred at room temperature for 1 h. The solvent was evaporated and the residue was purified by reversed phase HPLC to afford 1-(4-(((6-(2-chloro-3-(3-chloro-2-(2-(((3-fluoropropyl)amino)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one (4 mg, 32%) as a white solid. LCMS: m/z found 721.3 [M+H]⁺, retention time = 1.61 min (Method D). ¹H NMR (400 MHz, Methanol-*d*₄): δ 8.81 (d, 1H), 8.74 - 8.70 (m, 1H), 7.86 (d, 1H), 7.73 (dd, 1H), 7.58 (t, 1H), 7.52 (d, 1H), 7.48 - 7.44 (m, 2H), 7.33 (d, 1H), 4.66 - 4.58 (m, 2H), 4.50 (t, 1H), 4.29 (s, 1H), 4.17 (d, 3H), 4.11 (d, 4H), 4.06 (s, 4H), 4.02 (s, 1H), 3.19 (t, 2H), 3.08 (t, 2H), 2.70 (t, 1H), 2.20 (d, 2H), 2.12 (s, 4H), 2.09 - 1.99 (m, 1H).

### Example 687: 1-(4-(((6-(2-chloro-3-(3-chloro-2-(2-(((2-hydroxy-2-methylpropyl)amino)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one (714)

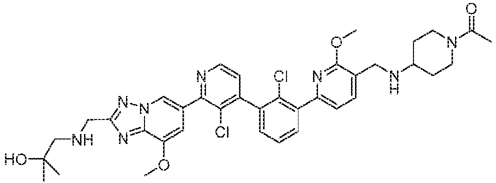

### (a) tert-Butyl (1-acetylpiperidin-4-yl)((6-(2-chloro-3-(3-chloro-2-(2-(((2-hydroxy-2-methylpropyl)amino)methyl)-8-methoxy-[1,2,4]triazolo[1,5-a]pyridin-6-yl)pyridin-4-yl)phenyl)-2-methoxypyridin-3-yl)methyl)carbamate

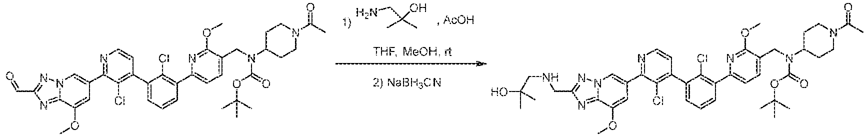

tert-Butyl (1-acetylpiperidin-4-yl)((6-(2-chloro-3-(3-chloro-2-(2-formyl-8-methoxy-[1,2,4]triazolo[1,5-a]pyridin-6-yl)pyridin-4-yl)phenyl)-2-methoxypyridin-3-yl)methyl)carbamate (26 mg, 0.03 mmol), acetic acid (4 uL, 4 mg, 0.07 mmol) and 1-amino-2-methyl-propan-2-ol (6 mg, 0.07 mmol) were dissolved in 1:1 THF/MeOH (1 mL) and the reaction was stirred at room temperature for 60 minutes. Sodium cyanoborohydride (4 mg, 0.07 mmol) was added in one portion and the resulting mixture was stirred at room temperature for 1 h. The reaction mixture was concentrated and the residue was suspended in 2 ml water. The precipitate formed was collected by filtration and dried to afford tert-butyl (1-acetylpiperidin-4-yl)((6-(2-chloro-3-(3-chloro-2-(2-(((2-hydroxy-2-methylpropyl)amino)methyl)-8-methoxy-[1,2,4]triazolo[1,5-a]pyridin-6-yl)pyridin-4-yl)phenyl)-2-methoxypyridin-3-yl)methyl)carbamate (17 mg, 60%) as a white solid. LCMS: m/z found 833.5 [M+H]⁺.

### (b) 1-(4-(((6-(2-chloro-3-(3-chloro-2-(2-(((2-hydroxy-2-methylpropyl)amino)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one

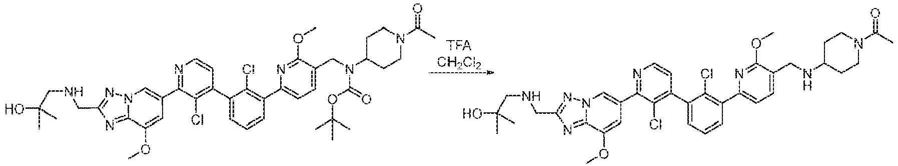

To a solution of tert-butyl (1-acetylpiperidin-4-yl)((6-(2-chloro-3-(3-chloro-2-(2-(((2-hydroxy-2-methylpropyl)amino)methyl)-8-methoxy-[1,2,4]triazolo[1,5-a]pyridin-6-yl)pyridin-4-yl)phenyl)-2-methoxypyridin-3-yl)methyl)carbamate (17 mg, 0.02 mmol) in dichloromethane (0.3 mL) was added trifluoroacetic acid (0.08 mL, 0.12 g, 1.02 mmol) and the reaction was stirred at room temperature for 1 h. The solvent was evaporated and the residue was purified by reversed phase HPLC to afford 1-(4-(((6-(2-chloro-3-(3-chloro-2-(2-(((2-hydroxy-2-methylpropyl)amino)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one (6 mg, 41%). LCMS: m/z found 733.4 [M+H]⁺, retention time = 0.61 min (Method B). ¹H NMR (400 MHz, Methanol-*d*₄): δ 8.85 (d, 1H), 8.73 (dd, 1H), 7.91 (d, 1H), 7.73 (dd, 1H), 7.59 (t, 1H), 7.53 (dd, 1H), 7.50 - 7.45 (m, 2H), 7.37 (d, 1H), 4.68 (d, 1H), 4.56 (s, 2H), 4.33 (s, 2H), 4.13 (d, 3H), 4.09 (s, 4H), 3.56 - 3.46 (m, 1H), 3.33 (d, 1H), 3.22 (s, 3H), 2.70 (t, 1H), 2.26 (t, 2H), 2.13 (s, 3H), 1.75 - 1.45 (m, 1H), 1.33 (s, 6H).

### Example 688: 1-(4-(((6-(2-Chloro-3-(3-chloro-2-(2-(((2-hydroxyethyl)(methyl)amino)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one (715)

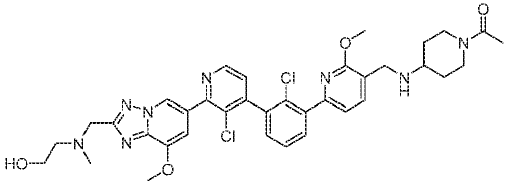

### (a) tert-Butyl (1-acetylpiperidin-4-yl)((6-(2-chloro-3-(3-chloro-2-(2-(((2-hydroxyethyl)(methyl)amino)methyl)-8-methoxy-[1,2,4]triazolo[1,5-a]pyridin-6-yl)pyridin-4-yl)phenyl)-2-methoxypyridin-3-yl)methyl)carbamate

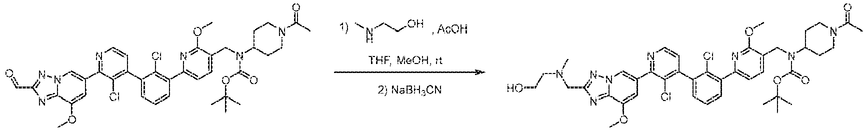

tert-Butyl (1-acetylpiperidin-4-yl)((6-(2-chloro-3-(3-chloro-2-(2-formyl-8-methoxy-[1,2,4]triazolo[1,5-a]pyridin-6-yl)pyridin-4-yl)phenyl)-2-methoxypyridin-3-yl)methyl)carbamate (26 mg, 0.03 mmol), acetic acid (4 uL, 4 mg, 0.07 mmol) and 2-(methylamino)ethanol (5 mg, 0.07 mmol) were dissolved in 1:1 THF/MeOH (1 mL) and the reaction was stirred at room temperature for 60 minutes. Sodium cyanoborohydride (4 mg, 0.07 mmol) was added in one portion and the resulting mixture was stirred at room temperature for 1 h. The reaction mixture was concentrated and the residue was suspended in 2 ml water. The precipitate formed was collected by filtration and dried to afford tert-butyl (1-acetylpiperidin-4-yl)((6-(2-chloro-3-(3-chloro-2-(2-(((2-hydroxyethyl)(methyl)amino)methyl)-8-methoxy-[1,2,4]triazolo[1,5-a]pyridin-6-yl)pyridin-4-yl)phenyl)-2-methoxypyridin-3-yl)methyl)carbamate (18 mg, 64%) as a white solid. LCMS: m/z found 819.4 [M+H]⁺.

### (b) 1-(4-(((6-(2-Chloro-3-(3-chloro-2-(2-(((2-hydroxyethyl)(methyl)amino)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one

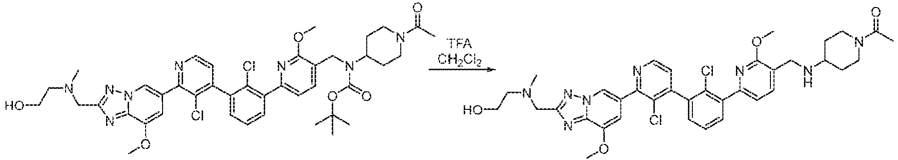

To a solution of tert-butyl (1-acetylpiperidin-4-yl)((6-(2-chloro-3-(3-chloro-2-(2-(((2-hydroxyethyl)(methyl)amino)methyl)-8-methoxy-[1,2,4]triazolo[1,5-a]pyridin-6-yl)pyridin-4-yl)phenyl)-2-methoxypyridin-3-yl)methyl)carbamate (18 mg, 0.02 mmol) in dichloromethane (0.3 mL) was added trifluoroacetic acid (0.08 mL, 0.13 g, 1.10 mmol) and the reaction was stirred at room temperature for 1 h. The solvent was evaporated and the residue was purified by reversed phase HPLC to afford 1-(4-(((6-(2-chloro-3-(3-chloro-2-(2-(((2-hydroxyethyl)(methyl)amino)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one (6 mg, 38%). LCMS: m/z found 719.4 [M+H]⁺, retention time = 0.59 min (Method B). ¹H NMR (400 MHz, Methanol-*d*₄): δ 8.83 (d, 1H), 8.73 (d, 1H), 7.91 (d, 1H), 7.73 (dd, 1H), 7.59 (t, 1H), 7.52 (d, 1H), 7.47 (td, 2H), 7.36 (d, 1H), 4.67 (d, 1H), 4.42 (s, 2H), 4.31 (s, 2H), 4.12 (s, 3H), 4.08 (s, 4H), 3.91 - 3.81 (m, 2H), 3.54 - 3.42 (m, 1H), 3.23 (d, 1H), 3.20 - 3.13 (m, 2H), 2.81 (s, 3H), 2.70 (t, 1H), 2.25 (t, 2H), 2.13 (s, 3H), 1.74 - 1.46 (m, 1H).

### Example 689: 2-((6-(2-Chloro-3-(3-chloro-2-(3-(difluoromethoxy)-4-(((tetrahydro-2H-pyran-4-yl)amino)methyl)phenyl)pyridin-4-yl)phenyl)-2-methoxypyridin-3-yl)methyl)-2,6-diazaspiro[3.4]octan-7-one (685)

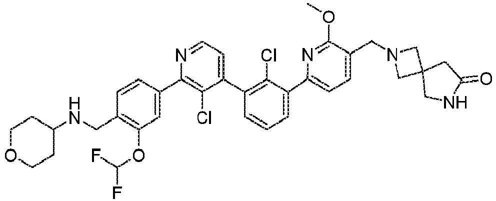

### (a) 2-((6-(2-Chloro-3-(2,3-dichloropyridin-4-yl)phenyl)-2-methoxypyridin-3-yl)methyl)-2,6-diazaspiro [3.4] octan-7-one

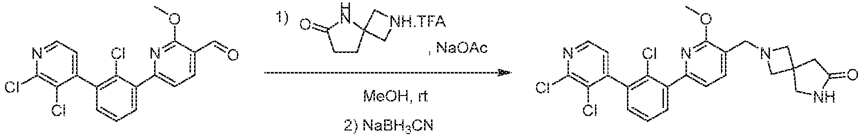

To a mixture of 6-(2-chloro-3-(2,3-dichloropyridin-4-yl)phenyl)-2-methoxynicotinaldehyde (600 mg, 1.52 mmol) and 2,6-diazaspiro[3.4]octan-7-one trifluoroacetate salt (1.46 g, 6.10 mmol) in methanol (10 mL) was added sodium acetate (1.25 g, 15.2 mmol) and the mixture stirred at room temperature for 3 h under N₂. Sodium triacetoxyborohydride (1.62 g, 7.62 mmol) was then added and the mixture was stirred at room temperature for 0.5 h under N₂. The mixture was then filtered and concentrated. The residue was purified by normal phase SiO₂ chromatography (0-45% EtOAc/petroleum ether) to afford compound 2-((6-(2-chloro-3-(2,3-dichloropyridin-4-yl)phenyl)-2-methoxypyridin-3-yl)methyl)-2,6-diazaspiro[3.4]octan-7-one (480 mg, 56% yield) as a white solid. MS: *m*/*z* found 503 [M+H]⁺.

### (b) 2-(Difluoromethoxy)-4-(4,4,5,5-tetramethyl-1,3,2-dioxaborolan-2-yl)benzaldehyde

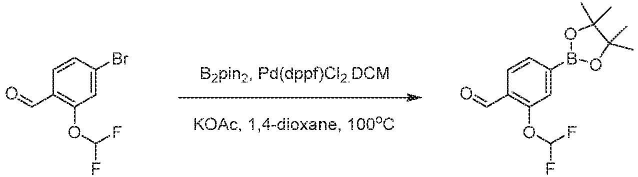

To a mixture of 4-bromo-2-(difluoromethoxy)benzaldehyde (300 mg, 1.20 mmol) and bis(pinacolato)diboron (394 mg, 1.55 mmol) in 1,4-dioxane (3 mL) was added potassium acetate (234 mg, 2.39 mmol) and [1,1'- bis(diphenylphosphino)ferrocene]-dichloropalladium(II) complex with dichloromethane (97.6 mg, 0.119 mmol) and the mixture stirred at 100 °C for 1 h under N₂. Water (10 mL) was then added and the mixture extracted with EtOAc (2 x 10 mL). The combined organic phases were dried over anhydrous sulfate, filtered and concentrated under reduced pressure. The residue was purified by normal phase SiO₂ chromatography (0-15% EtOAc/petroleum ether) to afford 2-(difluoromethoxy)-4-(4,4,5,5-tetramethyl-1,3,2-dioxaborolan-2-yl)benzaldehyde (230 mg, crude) as a yellow oil. ¹H NMR (400 MHz, DMSO-*d*₆): δ 10.37 (s, 1H), 7.92 (d, 1H), 7.75-7.35 (m, 1H), 7.68-7.32 (m, 2H), 1.34 (s, 12H).

### (c) 4-(3-Chloro-4-(2-chloro-3-(6-methoxy-5-((7-oxo-2,6-diazaspiro[3.4]octan-2-yl)methyl)pyridin-2-yl)phenyl)pyridin-2-yl)-2-(difluoromethoxy)benzaldehyde

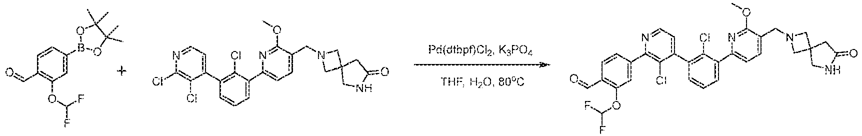

To a mixture of 2-((6-(2-chloro-3-(2,3-dichloropyridin-4-yl)phenyl)-2-methoxypyridin-3-yl)methyl)-2,6-diazaspiro[3.4]octan-7-one (200 mg, 0.397 mmol) and 2-(difluoromethoxy)-4-(4,4,5,5-tetramethyl-1,3,2-dioxaborolan-2-yl)benzaldehyde (177 mg, 0.59 mmol) in THF/ water (5:1, 6 mL) was added potassium phosphate (252 mg, 1.19 mmol) and [1,1'-bis(di-tert-butylphosphino)ferrocene]dichloropalladium(II) (25.8 mg, 0.04 mmol) and the mixture stirred at 80 °C for 2 h under N₂. Water (10 mL) was then added and the mixture extracted with EtOAc (2 x 10 mL). The combined organic phases were dried over anhydrous sulfate, filtered and concentrated under reduced pressure. The residue was purified by normal phase SiO₂ chromatography (0-60% EtOAc/petroleum ether) to afford 4-(3-chloro-4-(2-chloro-3-(6-methoxy-5-((7-oxo-2,6-diazaspiro[3.4]octan-2-yl)methyl)pyridin-2-yl)phenyl)pyridin-2-yl)-2-(difluoromethoxy)benzaldehyde (130 mg, 24% yield) as a white solid. MS: *m*/*z* found 639 [M+H]⁺.

### (d) 2-((6-(2-Chloro-3-(3-chloro-2-(3-(difluoromethoxy)-4-(((tetrahydro-2H-pyran-4-yl)amino)methyl)phenyl)pyridin-4-yl)phenyl)-2-methoxypyridin-3-yl)methyl)-2,6-diazaspiro[3.4]octan-7-one

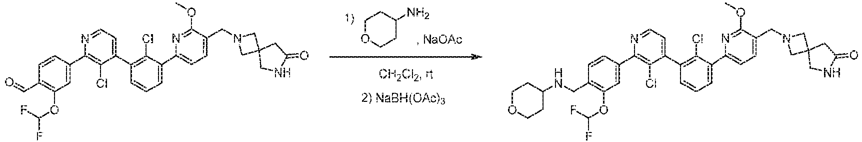

To mixture of 4-(3-chloro-4-(2-chloro-3-(6-methoxy-5-((7-oxo-2,6-diazaspiro[3.4]octan-2-yl)methyl)pyridin-2-yl)phenyl)pyridin-2-yl)-2-(difluoromethoxy)benzaldehyde (120 mg, 187 umol) and tetrahydro-2H-pyran-4-amine (28.4 mg, 281 umol) in dichloromethane (3 mL) was added sodium acetate (46.2 mg, 562 umol), and the mixture was stirred at room temperature for 1h under N₂. Sodium triacetoxyborohydride (119 mg, 562 umol) was then added and the mixture stirred at room temperature for 0.5 h under N₂. The mixture was concentrated and residue purified by reverse phase HPLC to afford 2-((6-(2-chloro-3-(3-chloro-2-(3-(difluoromethoxy)-4-(((tetrahydro-2H-pyran-4-yl)amino)methyl)phenyl)pyridin-4-yl)phenyl)-2-methoxypyridin-3-yl)methyl)-2,6-diazaspiro[3.4]octan-7-one (26 mg, 18% yield) as a white solid. MS: *m*/*z* found 724 [M+H]⁺, retention time = 2.94 min (Method A). ¹H NMR (400 MHz, Methanol-*d*₄): δ 8.65 (d, 1H), 7.73-7.68 (m, 2H), 7.60-7.50 (m, 4H), 7.47 (d, 1H), 7.45-7.43 (m, 1H), 7.24 (d, 1H), 6.97 (t, 1H), 4.05-3.97 (m, 7H), 3.82 (s, 2H), 3.60 (s, 2H), 3.43-3.41 (m, 6H), 2.82-2.79 (m, 1H), 2.59 (s, 2H), 1.96-1.93 (m, 2H), 1.56-1.50 (m, 2H).

### Example 690: 2-(4-(4-(3-(5-((6-Acetyl-2,6-diazaspiro[3.3]heptan-2-yl)methyl)-6-methoxypyridin-2-yl)-2-chlorophenyl)-3-chloropyridin-2-yl)-2-(difluoromethoxy)benzyl)-2,6-diazaspiro[3.4]octan-7-one (688)

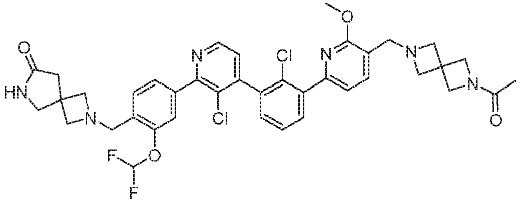

### (a) 1-(6-((6-(2-Chloro-3-(2,3-dichloropyridin-4-yl)phenyl)-2-methoxypyridin-3-yl)methyl)-2,6-diazaspiro[3.3]heptan-2-yl)ethan-1-one

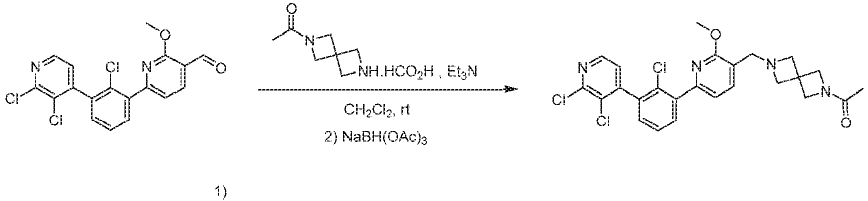

To a mixture of 6-(2-chloro-3-(2,3-dichloropyridin-4-yl)phenyl)-2-methoxynicotinaldehyde (0.60 g, 1.52 mmol) and 1-(2,6-diazaspiro[3.3]heptan-2-yl)ethan-1-one formic acid salt (1.16 g, 4.57 mmol) in dichloromethane (10 mL) was added triethylamine (0.62 g, 6.10 mmol) and the mixture stirred at room temperature for 2.5 h under N₂. Sodium triacetoxyborohydride (0.97 g, 4.57 mmol) was then added and the mixture stirred at room temperature for 0.5 h under N₂. Water (20 mL) was added and the mixture extracted with EtOAc (2 x 30 mL). The combined organic phases were dried over anhydrous sulfate, filtered and concentrated under reduced pressure. The residue was purified by normal phase SiO₂ chromatography (0-40 % MeOH /EtOAc) to afford 1-(6-((6-(2-chloro-3-(2,3-dichloropyridin-4-yl)phenyl)-2-methoxypyridin-3-yl)methyl)-2,6-diazaspiro[3.3]heptan-2-yl)ethan-1-one (600 mg, 76% yield) as a white solid. ¹H NMR (400 MHz, DMSO-*d*₆): δ 8.51 (d, 1H), 7.84-7.82 (m, 1H), 7.75 (dd, 1H), 7.63-7.57 (m, 2H), 7.51 (dd, 1H), 7.34 (d, 1H), 4.23 (s, 2H), 3.95 (s, 3H), 3.62-3.59 (m, 4H), 1.78-1.75 (m, 4H), 1.73 (s, 3H).

### (b) 4-(4-(3-(5-((6-acetyl-2,6-diazaspiro[3.3]heptan-2-yl)methyl)-6-methoxypyridin-2-yl)-2-chlorophenyl)-3-chloropyridin-2-yl)-2-(difluoromethoxy)benzaldehyde

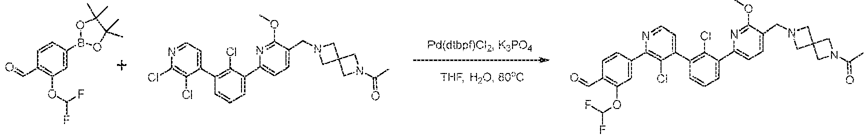

To a mixture of 1-(6-((6-(2-chloro-3-(2,3-dichloropyridin-4-yl)phenyl)-2-methoxypyridin-3-yl)methyl)-2,6-diazaspiro[3.3]heptan-2-yl)ethan-1-one (500 mg, 0.97 mmol) and 2-(difluoromethoxy)-4-(4,4,5,5-tetramethyl-1,3,2-dioxaborolan-2-yl)benzaldehyde (Example 689, step (b)) (432 mg, 1.45 mmol) in THF / water (5:1, 18 mL) was added potassium phosphate (615 mg, 2.90 mmol) and [1,1'-bis(di-tert-butylphosphino)ferrocene]-dichloropalladium(II) (62.9 mg, 0.09 mmol) and the mixture stirred at 80 °C for 3 h under N₂. Water (20 mL) was added and the mixture extracted with EtOAc (2 x30 mL). The combined organic phases were dried over anhydrous sulfate, filtered and concentrated under reduced pressure. The residue was purified by normal phase SiO₂ chromatography (0-30 % EtOAc/ MeOH) to afford 4-(4-(3-(5-((6-acetyl-2,6-diazaspiro[3.3]heptan-2-yl)methyl)-6-methoxypyridin-2-yl)-2-chlorophenyl)-3-chloropyridin-2-yl)-2-(difluoromethoxy)benzaldehyde (444 mg, 70% yield) as a white solid. ¹H NMR (400 MHz, DMSO-*d*₆): δ 10.30 (s, H), 8.75 (d, 1H), 7.95 (d, 1H), 7.68-7.66 (m, 4H), 7.59 (d, 2H), 7.45 (d, 1H), 7.22 (m, 2H), 4.14 (s, 2H), 3.86 (s, 5H), 3.50 (s, 2H), 3.32-3.31 (m, 4H), 1.68 (s, 3H).

### (c) 2-(4-(4-(3-(5-((6-Acetyl-2,6-diazaspiro[3.3]heptan-2-yl)methyl)-6-methoxypyridin-2-yl)-2-chlorophenyl)-3-chloropyridin-2-yl)-2-(difluoromethoxy)benzyl)-2,6-diazaspiro [3.4] octan-7-one

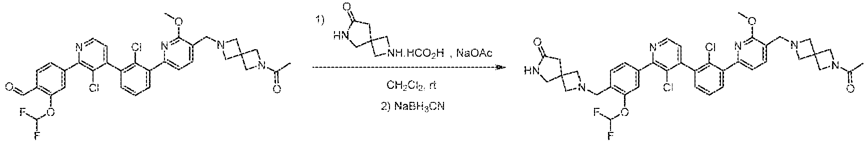

To a mixture of 4-(4-(3-(5-((6-acetyl-2,6-diazaspiro[3.3]heptan-2-yl)methyl)-6-methoxypyridin-2-yl)-2-chlorophenyl)-3-chloropyridin-2-yl)-2-(difluoromethoxy)benzaldehyde (150 mg, 0.23 mmol) and 2,6-diazaspiro[3.4]octan-7-one formic acid salt (276 mg, 1.15 mmol) in dichloromethane (5 mL) was added sodium acetate (151 mg, 1.84 mmol) and the mixture stirred at room temperature for 2.5 h. Sodium cyanoborohydride (57.7 mg, 0.92 mmol) was then added and the mixture stirred at room temperature for 0.5 h under N₂. The mixture was concentrated and the residue purified by reverse phase HPLC to afford 2-(4-(4-(3-(5-((6-acetyl-2,6-diazaspiro[3.3]heptan-2-yl)methyl)-6-methoxypyridin-2-yl)-2-chlorophenyl)-3-chloropyridin-2-yl)-2-(difluoromethoxy)benzyl)-2,6-diazaspiro[3.4]octan-7-one (35.3 mg, 19% yield) as a white solid (formic acid salt). MS: *m*/*z* found 763 [M+H]⁺, retention time = 2.63 min (Method A). ¹H NMR (400 MHz, Methanol-*d*₄): δ 8.67 (d, 1H), 7.77-7.71 (m, 2H), 7.61-7.54 (m, 4H), 7.48-7.43 (m, 2H), 7.31 (d, 1H), 6.97 (t, 1H), 4.60 (s, 2H), 4.35 (s, 2H), 4.11 (s, 2H), 4.05 (s, 3H), 3.96 (s, 2H), 3.92 (s, 2H), 3.84 (s, 3H), 3.62 (s, 2H), 3.55 (s, 3H), 2.62 (s, 2H), 1.87 (s, 3H).

### Example 691: 1-(6-((6-(2-Chloro-3-(3-chloro-2-(3-(difluoromethoxy)-4-(((tetrahydro-2H-pyran-4-yl)amino)methyl)phenyl)pyridin-4-yl)phenyl)-2-methoxypyridin-3-yl)methyl)-2,6-diazaspiro[3.3]heptan-2-yl)ethan-1-one (689)

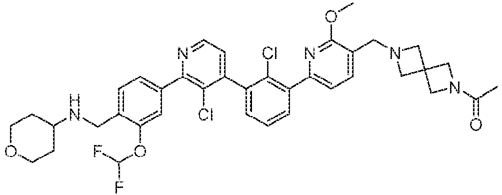

1-(6-((6-(2-Chloro-3-(3-chloro-2-(3-(difluoromethoxy)-4-(((tetrahydro-2H-pyran-4-yl)amino)methyl)phenyl)pyridin-4-yl)phenyl)-2-methoxypyridin-3-yl)methyl)-2,6-diazaspiro[3.3]heptan-2-yl)ethan-1-one was prepared in a similar fashion to Example 690, replacing 2,6-diazaspiro[3.4]octan-7-one formic acid salt with tetrahydro-2H-pyran-4-amine in step (c). White solid (formic acid salt), MS: *m*/*z* found 738 [M+H]⁺, retention time = 2.74 min (Method A). ¹H NMR (400 MHz, Methanol-*d*₄): δ 8.68 (d, 1H), 7.73-7.66 (m, 4H), 7.59-7.55 (m, 2H), 7.49 (d, 1H), 7.44-7.42 (m, 1H), 7.26 (d, 1H), 7.04 (t, 1H), 4.33 (s, 2H), 4.17 (s, 2H), 4.08-4.02 (m, 7H), 3.76 (s, 2H), 3.60 (s, 3H), 3.50-3.42 (m, 3H), 3.15-3.12 (m, 1H), 2.06-2.03 (m, 2H), 1.86 (s, 3H), 1.65-1.55 (m, 2H).

### Example 692: 1-(4-((4-(4-(3-(5-((6-Acetyl-2,6-diazaspiro[3.3]heptan-2-yl)methyl)-6-methoxypyridin-2-yl)-2-chlorophenyl)-3-chloropyridin-2-yl)-2-(difluoromethoxy)benzyl)amino)piperidin-1-yl)ethan-1-one (690)

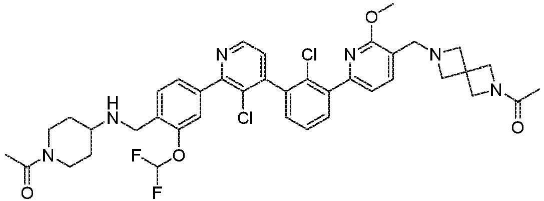

1-(4-((4-(4-(3-(5-((6-Acetyl-2,6-diazaspiro[3.3]heptan-2-yl)methyl)-6-methoxypyridin-2-yl)-2-chlorophenyl)-3-chloropyridin-2-yl)-2-(difluoromethoxy)benzyl)amino)piperidin-1-yl)ethan-1-one was prepared in a similar fashion to Example 690, replacing 2,6-diazaspiro[3.4]octan-7-one formic acid salt with 1-(4-aminopiperidin-1-yl)ethan-1-one hydrochloride salt and sodium cyanoborohydride with sodium triacetoxyborohydride in step (c). White solid (formic acid salt), MS: *m*/*z* found 779 [M+H]⁺, retention time = 2.85 min (Method A). ¹H NMR (400 MHz, Methanol-*d*₄): δ 8.65 (d, 1H), 7.74-7.64 (m, 4H), 7.57 (s, 1H), 7.54 (t, 1H), 7.45 (d, 1H), 7.40 (dd, 1H), 7.26 (d, 1H), 7.03 (t, 1H), 4.60-4.56 (m, 1H), 4.31 (s, 2H), 4.23 (s, 2H), 4.07 (s, 2H), 4.03-4.00 (m, 4H), 3.94-3.92 (m, 2H), 3.82-3.79 (m, 4H), 3.21-3.17 (m, 1H), 2.73-2.66 (m, 1H), 2.20-2.12 (m, 3H), 2.10 (s, 3H), 1.82 (s, 3H), 1.55-1.42 (m, 2H).

### Example 693: 1-(((6-(((S)-5-(3-Chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)cyclopropane-1-carboxylic acid (705)

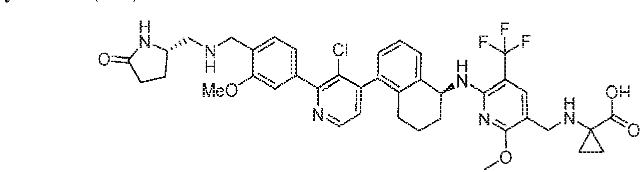

### (a) (S)-6-((5-Bromo-1,2,3,4-tetrahydronaphthalen-1-yl)amino)-2-methoxynicotinonitrile

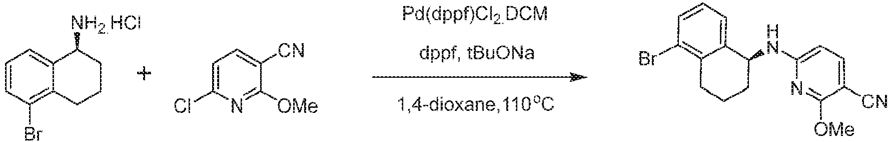

To a mixture of 6-chloro-2-methoxynicotinonitrile (5.4 g, 32 mmol) and (S)-5-bromo-1,2,3,4-tetrahydronaphthalen-1-amine as a hydrochloride salt (7.57 g, 28.8 mmol) in 1,4-dioxane (120 mL) was added sodium tert-butoxide (6.77 g, 70.5 mmol) and 1,1'-bis(diphenylphosphino)ferrocene (0.89 g, 1.6 mmol) and [1,1'-bis(diphenylphosphino)ferrocene]dichloropalladium(II) complex with dichloromethane (0.52 g, 0.64 mmol) in one portion under N₂. The mixture was stirred at 110 °C for 3 h. The mixture was concentrated, and then water (500 mL) and saturated aqueous brine solution (100 mL) added to the residue. The mixture was extracted with ethyl acetate/THF mixture (1:1, 500 mL), the organic phase dried with anhydrous sodium sulfate, filtered and concentrated. The residue was purified by normal phase SiO₂ chromatography (0-10% ethyl acetate/petroleum ether) to afford (S)-6-((5-bromo-1,2,3,4-tetrahydronaphthalen-1-yl)amino)-2-methoxynicotinonitrile (16.8 g, 60% yield) as a yellow solid. ¹H NMR (400 MHz, chloroform-d): δ 7.46-7.42 (m, 2H), 7.22-7.19 (m, 1H), 6.98 (t, 1H), 5.92 (d, 1H), 5.11-5.01 (m, 2H), 3.88 (s, 3H), 2.82-2.68 (m, 2H), 1.92-1.85 (m, 4H).

### (b) (S)-6-((5-Bromo-1,2,3,4-tetrahydronaphthalen-1-yl)amino)-5-iodo-2-methoxynicotinonitrile

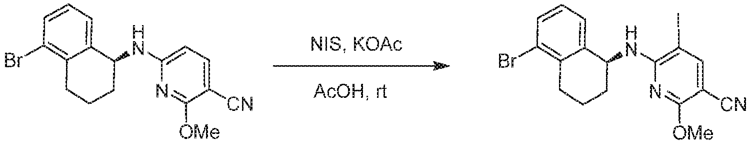

To a mixture of (S)-6-((5-bromo-1,2,3,4-tetrahydronaphthalen-1-yl)amino)-2-methoxynicotinonitrile (5.6 g, 15.6 mmol) in glacial acetic acid (55 mL) and potassium acetate (15.3 g, 156 mmol) was added N-Iodosuccinimide (3.87 g, 17.2 mmol) in one portion under N₂ and the mixture stirred at room temperature for 3 h. The mixture was neutralized with saturated aqueous sodium bicarbonate solution and water (500 mL) and saturated aqueous brine solution (100 mL) were then added. The mixture was extracted with ethyl acetate/THF mixture (1:1, 500 mL). The organic phase was dried with anhydrous sodium sulfate, filtered and concentrated. The residue was purified directly by normal phase SiO₂ chromatography (0-7% ethyl acetate/petroleum ether) to afford (S)-6-((5-bromo-1,2,3,4-tetrahydronaphthalen-1-yl)amino)-5-iodo-2-methoxynicotinonitrile (18 g, 63% yield) as a white solid. ¹H NMR (400 MHz, Choroform-d): δ 7.81 (s, 1H), 7.44 (d, 1H), 7.19-7.18 (m, 2H), 7.00 (t, 1H), 5.51-5.49 (m, 1H), 5.33-5.28 (m, 1H), 3.90 (s, 3H), 2.84-2.72 (m, 2H), 1.91-1.84 (m, 4H).

### (c) (S)-6-((5-Bromo-1,2,3,4-tetrahydronaphthalen-1-yl)amino)-2-methoxy-5-(trifluoromethyl)nicotinonitrile

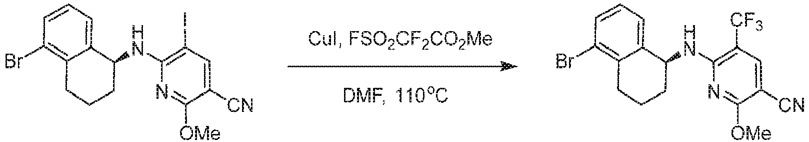

To a mixture of (S)-6-((5-bromo-1,2,3,4-tetrahydronaphthalen-1-yl)amino)-5-iodo-2-methoxynicotinonitrile (6 g, 12.4 mmol) and methyl 2,2-difluoro-2-(fluorosulfonyl)acetate (6.3 mL, 49.6 mmol) in DMF (70 mL) was added copper(I) iodide (3.5 g, 18.6 mmol) in one portion under N₂ and the mixture was stirred at 110 °C for 3 h. The mixture was combined with another batch at 7 g scale. The mixture was filtered and the filtrate concentrated. To the residue was added water (500 mL) and saturated aqueous brine solution (100 mL) and the mixture was extracted with ethyl acetate/THF mixture (1:1, 500 mL). The organic phase was dried with anhydrous sodium sulfate, filtered and concentrated. The residue was purified directly by normal phase SiO₂ chromatography (0-10% ethyl acetate/petroleum ether) to afford (S)-6-((5-bromo-1,2,3,4-tetrahydronaphthalen-1-yl)amino)-2-methoxy-5-(trifluoromethyl)nicotinonitrile (20 g, 79% purity) as a yellow oil. ¹H NMR (400 MHz, chloroform-d): δ 7.75 (s, 1H), 7.44 (dd, 1H), 7.19-7.16 (m, 1H), 7.00 (t, 1H), 5.43-5.41 (m, 2H), 3.95 (s, 3H), 2.79-2.72 (m, 2H), 2.02-1.90 (m, 1H), 1.88-1.53 (m, 3H).

### (d) (S)-6-((5-Bromo-1,2,3,4-tetrahydronaphthalen-1-yl)amino)-2-methoxy-5-(trifluoromethyl)nicotinaldehyde

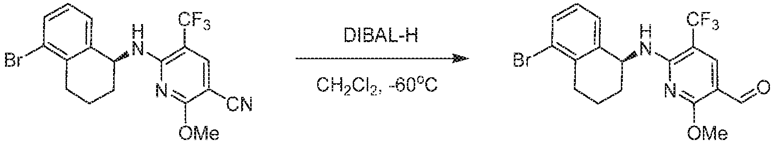

To a mixture of (S)-6-((5-bromo-1,2,3,4-tetrahydronaphthalen-1-yl)amino)-2-methoxy-5-(trifluoromethyl)nicotinonitrile (5 g, 11.7 mmol) in dichloromethane (50 mL) was dropwise added diisobutyl aluminium hydride (1 M, 16 mL) in one portion at -60 °C under N₂. The mixture was stirred at -60 °C for 1 h. To the mixture was added water (0.1 mL) and 2 N aqueous NaOH solution (0.1 mL) to quench diisobutyl aluminium hydride at -20 °C. Ethyl acetate/THF mixture (1:1, 100 mL) was then added and the mixture was dried with anhydrous sodium sulfate, filtered and concentrated. The residue was purified directly by normal phase SiO₂ chromatography (1-10% ethyl acetate/petroleum ether) to afford (S)-6-((5-bromo-1,2,3,4-tetrahydronaphthalen-1-yl)amino)-2-methoxy-5-(trifluoromethyl)nicotinaldehyde (8 g, 69% yield) as a yellow solid. ¹H NMR (400 MHz, chloroform-d): δ 10.03 (s, 1H), 8.13 (s, 1H), 7.45 (dd, 1H), 7.20-7.18 (m, 1H), 7.00 (t, 1H), 5.53-5.47 (m, 2H), 3.97 (s, 3H), 2.80-2.74 (m, 2H), 2.05-1.89 (m, 1H), 1.88-1.51 (m, 3H).

### (e) (S)-6-((5-(2,3-Dichloropyridin-4-yl)-1,2,3,4-tetrahydronaphthalen-1-yl)amino)-2-methoxy-5-(trifluoromethyl)nicotinaldehyde

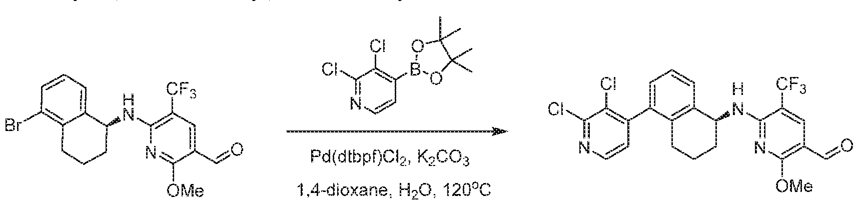

To a mixture of (S)-6-((5-bromo-1,2,3,4-tetrahydronaphthalen-1-yl)amino)-2-methoxy-5-(trifluoromethyl)nicotinaldehyde (0.5 g, 1.16 mmol) and 2,3-dichloro-4-(4,4,5,5-tetramethyl-1,3,2-dioxaborolan-2-yl)pyridine (0.7 g, 2.56 mmol) in 1,4-dioxane/water mixture (5:1, 18 mL) was added [1,1'-bis(di-tert-butylphosphino)ferrocene]dichloropalladium(II) (0.08 g, 0.12 mmol) and potassium carbonate (0.74 g, 3.49 mmol) in one portion under N₂ and the mixture was stirred at 120 °C for 12 h. To the mixture was added 2,3-dichloro-4-(4,4,5,5-tetramethyl-1,3,2-dioxaborolan-2-yl)pyridine (0.32 g, 1.16 mmol) and the mixture was stirred at 120 °C for an additional 12 h. The mixture was concentrated, water (50 mL) and saturated aqueous brine solution (50 mL) were added to the residue, and the mixture was extracted with ethyl acetate/THF mixture (1:1, 100 mL). The organic phase was dried with anhydrous sodium sulfate, filtered and concentrated. The residue was purified by normal phase SiO₂ chromatography (0-6% ethyl acetate/petroleum ether) to afford (S)-6-((5-(2,3-dichloropyridin-4-yl)-1,2,3,4-tetrahydronaphthalen-1-yl)amino)-2-methoxy-5-(trifluoromethyl)nicotinaldehyde (0.23 g, 28% yield) as a yellow gum. MS: *m*/*z* found 496 [M+H]⁺.

### (f) tert-Butyl 4-(3-chloro-4-((S)-5-((5-formyl-6-methoxy-3-(trifluoromethyl)pyridin-2-yl)amino)-5,6,7,8-tetrahydronaphthalen-1-yl)pyridin-2-yl)-2-methoxybenzyl(((S)-5-oxopyrrolidin-2-yl)methyl)carbamate

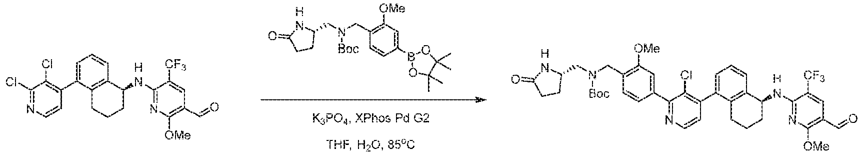

To a mixture of (S)-6-((5-(2,3-dichloropyridin-4-yl)-1,2,3,4-tetrahydronaphthalen-1-yl)amino)-2-methoxy-5-(trifluoromethyl)nicotinaldehyde (0.14 g, 0.28 mmol) and (S)-tert-butyl 2-methoxy-4-(4,4,5,5-tetramethyl-1,3,2-dioxaborolan-2-yl)benzyl((5-oxopyrrolidin-2-yl)methyl)carbamate (0.39 g, 0.85 mmol) in THF/water mixture (10:1, 5.5 mL) was added potassium phosphate (0.18 g, 0.85 mmol) and chloro(2-dicyclohexylphosphino-2',4',6'-triisopropyl-1,1'-biphenyl)[2-(2'-amino-1,1'-biphenyl) palladium (II) (0.02 g, 0.03 mmol) in one portion under N₂ and the mixture was stirred at 85 °C for 3 h. The mixture was concentrated, water (5 mL) and saturated aqueous brine solution (5 mL) added to the residue, and the mixture was extracted with ethyl acetate/THF mixture (1:1, 10 mL). The organic phase was dried with anhydrous sodium sulfate, filtered and concentrated. The residue was purified by normal phase SiO₂ chromatography (15-70% (10% THF in ethyl acetate)/petroleum ether) to afford tert-butyl 4-(3-chloro-4-((S)-5-((5-formyl-6-methoxy-3-(trifluoromethyl)pyridin-2-yl)amino)-5,6,7,8-tetrahydronaphthalen-1-yl)pyridin-2-yl)-2-methoxybenzyl(((S)-5-oxopyrrolidin-2-yl)methyl)carbamate (0.2 g, 67% yield) as a yellow gum. MS: *m*/*z* found 794 [M+H]⁺.

### (g) 1-(((6-(((S)-5-(2-(4-(((tert-Butoxycarbonyl)(((S)-5-oxopyrrolidin-2-yl)methyl)amino)methyl)-3-methoxyphenyl)-3-chloropyridin-4-yl)-1,2,3,4-tetrahydronaphthalen-1-yl)amino)-2-methoxy-5-(trifluoromethyl)pyridin-3-yl)methyl)amino)cyclopropanecarboxylic acid

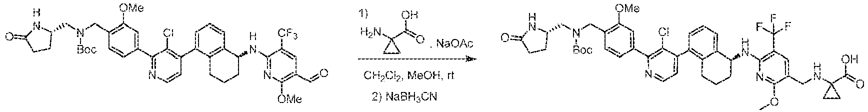

To a mixture of tert-butyl 4-(3-chloro-4-((S)-5-((5-formyl-6-methoxy-3-(trifluoromethyl)pyridin-2-yl)amino)-5,6,7,8-tetrahydronaphthalen-1-yl)pyridin-2-yl)-2-methoxybenzyl(((S)-5-oxopyrrolidin-2-yl)methyl)carbamate (0.19 g, 0.24 mmol) and 1-aminocyclopropanecarboxylic acid (24 mg, 0.24 mmol) in dichloromethane/methanol mixture (3:2, 5 mL) was added sodium acetate ( 0.04 g, 0.48 mmol) in one portion under N₂ and the mixture was stirred at room temperature for 5 h. To the mixture was added sodium cyanoborohydride (0.05 g, 0.72 mmol) and the mixture was stirred at room temperature for 1 h. The mixture was concentrated and the residue was purified by normal phase SiO₂ chromatography (10-100% (10% MeOH in ethyl acetate)/petroleum ether) to afford 1-(((6-(((S)-5-(2-(4-(((tert-butoxycarbonyl)(((S)-5-oxopyrrolidin-2-yl)methyl)amino)methyl)-3-methoxyphenyl)-3-chloropyridin-4-yl)-1,2,3,4-tetrahydronaphthalen-1-yl)amino)-2-methoxy-5-(trifluoromethyl)pyridin-3-yl)methyl)amino)cyclopropanecarboxylic acid (110 mg, 44% yield) as a white solid. MS: *m*/*z* found 879 [M+H]⁺.

### (h) 1-(((6-(((S)-5-(3-Chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)cyclopropanecarboxylic acid

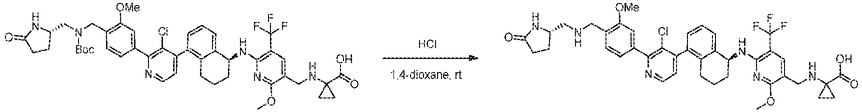

To a mixture of 1-(((6-(((S)-5-(2-(4-(((tert-butoxycarbonyl)(((S)-5-oxopyrrolidin-2-yl)methyl)amino)methyl)-3-methoxyphenyl)-3-chloropyridin-4-yl)-1,2,3,4-tetrahydronaphthalen- 1-yl)amino)-2-methoxy-5-(trifluoromethyl)pyridin-3-yl)methyl)amino)cyclopropanecarboxylic acid (0.11 g, 0.12 mmol) in 1,4-dioxane (2 mL) was added concentrated HCl solution (0.3 mL) in one portion and the mixture was stirred at room temperature for 4 h. The mixture was neutralized with aqueous ammonia and the mixture was directly purified by reverse phase HPLC to afford 1-(((6-(((S)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)cyclopropanecarboxylic acid (34.1 mg, 35% yield) as a white solid. MS: *m*/*z* found 779 [M+H]⁺, retention time = 3.47 min (Method A). ¹H NMR (400 MHz, Methanol-*d*₄): δ 8.61-8.59 (m, 1H), 7.84 (s, 1H), 7.45-7.39 (m, 2H), 7.34-7.27 (m, 4H), 7.08 (d, 1H), 6.22-6.13 (m, 1H), 5.64-5.54 (m, 1H), 4.24 (s, 2H), 4.01-3.96 (m, 8H), 3.91-3.87 (m, 1H), 2.80-2.75 (m, 2H), 2.65-2.45 (m, 2H), 2.38-2.32 (m, 3H), 2.14-2.12 (m, 2H), 1.96-1.82 (m, 3H), 1.40-1.37 (m, 2H), 1.19-1.16 (m, 2H).

### Example 694: (S)-5-((((6-(((S)-5-(3-Chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)methyl)pyrrolidin-2-one (710)

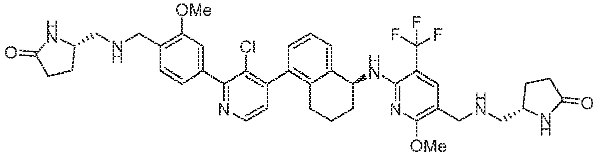

(S)-5-((((6-(((S)-5-(3-Chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)methyl)pyrrolidin-2-one was prepared in a similar fashion to Example 693, replacing 1-aminocyclopropanecarboxylic acid with (R)-5-(aminomethyl)pyrrolidin-2-one hydrochloride salt in step (g). White solid, MS: *m*/*z* found 792 [M+H]⁺, retention time = 3.34 min (Method A). ¹H NMR (400 MHz, Methanol-*d*₄): δ 8.60 (d, 1H), 7.68 (s, 1H), 7.45-7.41 (m, 2H), 7.35-7.23 (m, 4H), 7.08 (d, 1H), 5.87-5.78 (m, 1H), 5.59-5.53 (m, 1H), 4.00-3.82 (m, 10H), 3.72-3.68 (m, 2H), 2.71-2.24 (m, 12H), 2.12-1.73 (m, 6H).

### Example 695: 2-((6-(((S)-5-(3-Chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)-2,6-diazaspiro[3.4]octan-7-one (724)

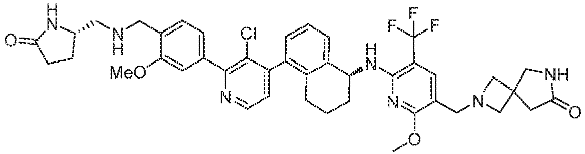

2-((6-(((S)-5-(3-Chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)-2,6-diazaspiro[3.4]octan-7-one was prepared in a similar fashion to Example 693, replacing 1-aminocyclopropanecarboxylic acid with 2,6-diazaspiro[3.4]octan-7-one trifluoroacetic acid salt and sodium cyanoborohydride with sodium triacetoxyborohydride in step (g). White solid, MS: *m*/*z* found 804 [M+H]⁺, retention time = 3.28 min (Method A). ¹H NMR (400 MHz, Methanol-*d*₄): δ 8.59 (d, 1H), 7.62 (s, 1H), 7.44-7.41 (m, 2H), 7.34-7.26 (m, 4H), 7.08 (d, 1H), 5.84-5.76 (m, 1H), 5.57-5.54 (m, 1H), 3.95-3.85 (m, 9H), 3.58-3.56 (m, 4H), 3.36-3.35 (m, 4H), 2.70-2.28 (m, 9H), 2.13-1.82 (m, 5H).

### Example 696: (S)-5-(((4-(3-Chloro-4-((S)-5-((5-((((S)-2-hydroxypropyl)amino)methyl)-6-methoxy-3-(trifluoromethyl)pyridin-2-yl)amino)-5,6,7,8-tetrahydronaphthalen-1-yl)pyridin-2-yl)-2-methoxybenzyl)amino)methyl)pyrrolidin-2-one (736)

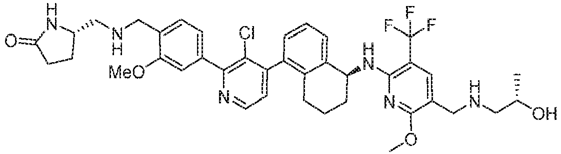

(S)-5-(((4-(3-Chloro-4-((S)-5-((5-((((S)-2-hydroxypropyl)amino)methyl)-6-methoxy-3-(trifluoromethyl)pyridin-2-yl)amino)-5,6,7,8-tetrahydronaphthalen-1-yl)pyridin-2-yl)-2-methoxybenzyl)amino)methyl)pyrrolidin-2-one was prepared in a similar fashion to Example 693, replacing 1-aminocyclopropanecarboxylic acid with (S)-1-aminopropan-2-ol in step (g). White solid, MS: *m*/*z* found 753 [M+H]⁺, retention time = 3.65 min (Method A). ¹H NMR (400 MHz, Methanol-*d*₄): δ 8.60 (d, 1H), 7.69 (s, 1H), 7.44-7.41 (m, 2H), 7.35-7.26 (m, 4H), 7.08 (d, 1H), 5.94-5.85 (m, 1H), 5.62-5.51 (m, 1H), 3.95-3.94 (m, 6H), 3.90-3.72 (m, 6H), 2.71-2.46 (m, 6H), 2.38-2.26 (m, 3H), 2.12-2.04 (m, 2H), 1.96-1.76 (m, 3H), 1.19 (d, 3H).

### Example 697: (S)-N-(5-(3-Chloro-2-(3-methoxy-4-((methylamino)methyl)phenyl)pyridin-4-yl)-1,2,3,4-tetrahydronaphthalen-1-yl)-6-methoxy-5-((methylamino)methyl)-3-(trifluoromethyl)pyridin-2-amine (741)

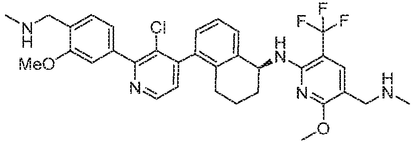

### (a) (S)-6-((5-(3-chloro-2-(4-formyl-3-methoxyphenyl)pyridin-4-yl)-1,2,3,4-tetrahydronaphthalen-1-yl)amino)-2-methoxy-5-(trifluoromethyl)nicotinaldehyde

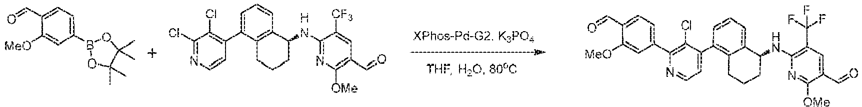

To a solution of (S)-6-((5-(2,3-dichloropyridin-4-yl)-1,2,3,4-tetrahydronaphthalen-1-yl)amino)-2-methoxy-5-(trifluoromethyl)nicotinaldehyde (0.32 g, 0.64 mmol) (Example 693, step (e)) and 2-methoxy-4-(4,4,5,5-tetramethyl-1,3,2-dioxaborolan-2-yl)benzaldehyde (0.24 g, 0.9 mmol) in THF/water mixture (6:1, 7.2 mL) was added chloro(2-dicyclohexylphosphino-2',4',6'-triisopropyl-1,1'-biphenyl)[2-(2'-amino-1,1'-biphenyl)]palladium(II) (0.05 g, 0.06 mmol) and potassium phosphate (0.41 g, 1.93 mmol) and the mixture stirred at 80 °C for 1 hr. Water (10 mL) was then added, and the mixture extracted with ethyl acetate (2 x 10 mL). The combined organic phases were washed with saturated aqueous brine solution (2 x 10 mL), dried with anhydrous sodium sulfate, filtered and concentrated. The residue was purified by normal phase SiO₂ chromatography (10-50% ethyl acetate/petroleum ether) to afford (S)-6-((5-(3-chloro-2-(4-formyl-3-methoxyphenyl)pyridin-4-yl)-1,2,3,4-tetrahydronaphthalen-1-yl)amino)-2-methoxy-5-(trifluoromethyl)nicotinaldehyde (230 mg, 46% yield) as a white solid. MS: *m*/*z* found 596 [M+H]⁺.

### (b) (S)-N-(5-(3-Chloro-2-(3-methoxy-4-((methylamino)methyl)phenyl)pyridin-4-yl)-1,2,3,4-tetrahydronaphthalen-1-yl)-6-methoxy-5-((methylamino)methyl)-3-(trifluoromethyl)pyridin-2-amine

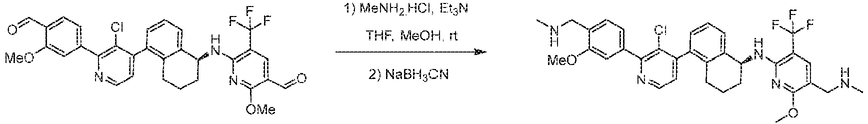

To a mixture of (S)-6-((5-(3-chloro-2-(4-formyl-3-methoxyphenyl)pyridin-4-yl)-1,2,3,4-tetrahydronaphthalen-1-yl)amino)-2-methoxy-5-(trifluoromethyl)nicotinaldehyde (0.1 g, 0.17 mmol) and methanamine hydrochloride salt (0.03 g, 0.5 mmol) in THF/Methanol (3:2, 5 mL) was added triethylamine (0.05 g, 0.5 mmol) in one portion under N₂ and the mixture was stirred at room temperature for 1 hr. To the mixture was added sodium cyanoborohydride (0.03 g, 0.5 mmol) and the mixture was stirred at room temperature for 0.5 hr. The mixture was concentrated and the residue was purified by reverse phase HPLC to afford (S)-N-(5-(3-chloro-2-(3-methoxy-4-((methylamino)methyl)phenyl)pyridin-4-yl)-1,2,3,4-tetrahydronaphthalen-1-yl)-6-methoxy-5-((methylamino)methyl)-3-(trifluoromethyl)pyridin-2-amine as a as a hydrochloride salt (20 mg). 20 mg of (S)-N-(5-(3-chloro-2-(3-methoxy-4-((methylamino)methyl)phenyl)pyridin-4-yl)-1,2,3,4-tetrahydronaphthalen-1-yl)-6-methoxy-5-((methylamino)methyl)-3-(trifluoromethyl)pyridin-2-amine hydrochloride salt was neutralized with saturated aqueous sodium bicarbonate solution and the mixture was extracted with dichloromethane (50 mL). The organic phase was dried with anhydrous sodium sulfate, filtered, concentrated and lyophilized to afford (S)-N-(5-(3-chloro-2-(3-methoxy-4-((methylamino)methyl)phenyl)pyridin-4-yl)-1,2,3,4-tetrahydronaphthalen-1-yl)-6-methoxy-5-((methylamino)methyl)-3-(trifluoromethyl)pyridin-2-amine (8.2 mg, 7% yield) as a white solid. MS: *m*/*z* found 626 [M+H]⁺, retention time = 3.48 min (Method A). ¹H NMR (400 MHz, Methanol-*d*₄): 8.48 (d, 1H), 7.54 (s, 1H), 7.33-7.27 (m, 2H), 7.21 (dd, 1H), 7.20-7.14 (m, 3H), 6.96 (d, 1H), 5.46-5.41 (m, 1H), 3.83-3.79 (m, 6H), 3.74 (s, 2H), 3.56-3.50 (m, 2H), 2.54-2.39 (m, 2H), 2.33 (s, 3H), 2.28 (s, 3H), 2.03-1.91 (m, 2H), 1.87-1.71 (m, 2H).

### Example 698: 1-(((6-(((S)-5-(3'-Chloro-6-methoxy-5-(((((S)-5-oxopyrrolidin-2-yl)methyl)amino)methyl)-[2,4'-bipyridin]-2'-yl)-1,2,3,4-tetrahydronaphthalen-1-yl)amino)-2-methoxy-5-(trifluoromethyl)pyridin-3-yl)methyl)amino)cyclopropanecarboxylic acid (704)

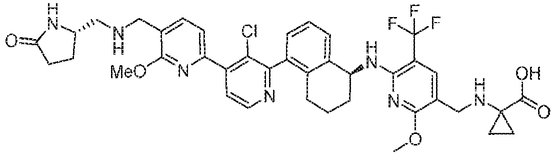

### (a) (S)-2-methoxy-6-((5-(4,4,5,5-tetramethyl-1,3,2-dioxaborolan-2-yl)-1,2,3,4-tetrahydronaphthalen-1-yl)amino)-5-(trifluoromethyl)nicotinaldehyde

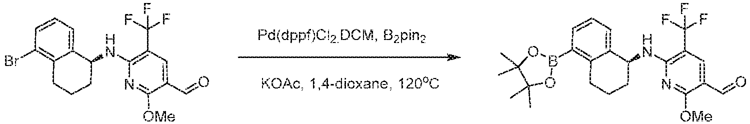

To a mixture of (S)-6-((5-bromo-1,2,3,4-tetrahydronaphthalen-1-yl)amino)-2-methoxy-5-(trifluoromethyl)nicotinaldehyde (Example 693, step (d)) (3 g, 6.99 mmol) and bis(pinacolato)diboron (4.44 g, 17.5 mmol) in 1,4-dioxane (100 mL) was added potassium acetate (2.06 g, 21 mmol) and [1,1'- bis(diphenylphosphino)ferrocene]dichloropalladium(II) complex with dichloromethane (0.29 g, 0.35 mmol) in one portion under N₂ and the mixture was stirred at 120 °C for 12 h. The mixture was concentrated and the residue was purified by normal phase SiO₂ chromatography (0-10% ethyl acetate/petroleum ether) to afford 5.5 g of the product. 0.5 g of the product was purified by reverse phase HPLC to afford (S)-2-methoxy-6-((5-(4,4,5,5-tetramethyl-1,3,2-dioxaborolan-2-yl)-1,2,3,4-tetrahydronaphthalen-1-yl)amino)-5-(trifluoromethyl)nicotinaldehyde (0.2 g, 36% yield) as a yellow gum. ¹H NMR (400 MHz, Chloroform-d): δ 10.03 (s, 1H), 8.12 (s, 1H), 7.67 (d, 1H), 7.28 (d, 1H), 7.12 (t, 1H), 5.55-5.53 (m, 1H), 5.48-5.43 (m, 1H), 3.98 (s, 3H), 3.17-3.11 (m, 1H), 3.00-2.98 (m, 1H), 1.97-1.77 (m, 4H), 1.28 (s, 12H).

### (b) tert-Butyl ((3'-chloro-2'-((S)-5-((5-formyl-6-methoxy-3-(trifluoromethyl)pyridin-2-yl)amino)-5,6,7,8-tetrahydronaphthalen-1-yl)-6-methoxy-[2,4'-bipyridin]-5-yl)methyl)(((S)-5-oxopyrrolidin-2-yl)methyl)carbamate

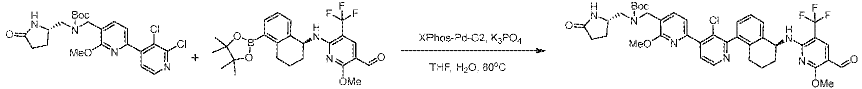

To a mixture of (S)-2-methoxy-6-((5-(4,4,5,5-tetramethyl-1,3,2-dioxaborolan-2-yl)-1,2,3,4-tetrahydronaphthalen-1-yl)amino)-5-(trifluoromethyl)nicotinaldehyde (0.15 g, 0.31 mmol) and (S)-tert-butyl ((2',3'-dichloro-6-methoxy-[2,4'-bipyridin]-5-yl)methyl)((5-oxopyrrolidin-2-yl)methyl)carbamate (0.08 g, 0.16 mmol) in THF/water mixture (10:1, 5.5 mL) was added potassium phosphate (0.1 g, 0.47 mmol) and chloro(2-dicyclohexylphosphino-2',4',6'-triisopropyl-1,1'-biphenyl)[2-(2'-amino-1,1'-biphenyl)]palladium(II) (12 mg, 16 umol) in one portion under N₂ and the mixture was stirred at 80 °C for 3 h. The mixture was concentrated and water (5 mL) and saturated aqueous brine solution (5 mL) were added to the residue. The mixture was extracted with ethyl acetate/THF mixture (1:1, 10 mL) and the organic phase dried with anhydrous sodium sulfate, filtered and concentrated. The residue was purified by normal phase SiO₂ chromatography (15-60% (10% THF in ethyl acetate)/petroleum ether) to afford tert-butyl ((3'-chloro-2'-((S)-5-((5-formyl-6-methoxy-3-(trifluoromethyl)pyridin-2-yl)amino)-5,6,7, 8-tetrahydronaphthalen-1-yl)-6-methoxy-[2,4'-bipyridin]-5-yl)methyl)(((S)-5-oxopyrrolidin-2-yl)methyl)carbamate (0.11 g, 59% yield) as a yellow gum. MS: *m*/*z* found 795 [M+H]⁺.

### (c) 1-(((6-(((S)-5-(5-(((tert-Butoxycarbonyl)(((S)-5-oxopyrrolidin-2-yl)methyl)amino)methyl)-3'-chloro-6-methoxy-[2,4'-bipyridin]-2'-yl)-1,2,3,4-tetrahydronaphthalen-1-yl)amino)-2-methoxy-5-(trifluoromethyl)pyridin-3-yl)methyl)amino)cyclopropanecarboxylic acid

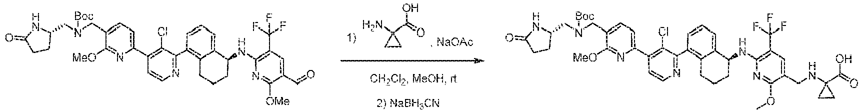

To a mixture of tert-butyl ((3'-chloro-2'-((S)-5-((5-formyl-6-methoxy-3-(trifluoromethyl)pyridin-2-yl)amino)-5,6,7,8-tetrahydronaphthalen-1-yl)-6-methoxy-[2,4'-bipyridin]-5-yl)methyl)(((S)-5-oxopyrrolidin-2-yl)methyl)carbamate (0.11 g, 0.14 mmol) and 1-aminocyclopropanecarboxylic acid (14 mg, 0.14 mmol) in dichloromethane/methanol mixture (3:2, 5 mL) was added sodium acetate (23 mg, 0.28 mmol) in one portion under N₂ and the mixture was stirred at room temperature for 12 h. Sodium cyanoborohydride (26 mg, 0.41 mmol) was then added and the mixture stirred at room temperature for 1 h. The mixture was directly purified by prep-TLC (silica gel, ethyl acetate: MeOH =2:1) to afford 1-(((6-(((S)-5-(5-(((tert-butoxycarbonyl)(((S)-5-oxopyrrolidin-2-yl)methyl)amino)methyl)-3'-chloro-6-methoxy-[2,4'-bipyridin]-2'-yl)-1,2,3,4-tetrahydronaphthalen-1-yl)amino)-2-methoxy-5-(trifluoromethyl)pyridin-3-yl)methyl)amino)cyclopropanecarboxylic acid (70 mg, 47% yield) as a white solid. MS: *m*/*z* found 902 [M+Na]⁺.

### (d) 1-(((6-(((S)-5-(3'-Chloro-6-methoxy-5-(((((S)-5-oxopyrrolidin-2-yl)methyl)amino)methyl)-[2,4'-bipyridin]-2'-yl)-1,2,3,4-tetrahydronaphthalen-1-yl)amino)-2-methoxy-5-(trifluoromethyl)pyridin-3-yl)methyl)amino)cyclopropanecarboxylic acid

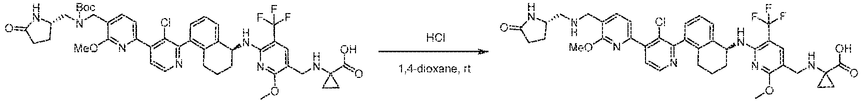

To a mixture of 1-(((6-(((S)-5-(5-(((tert-butoxycarbonyl)(((S)-5-oxopyrrolidin-2-yl)methyl)amino)methyl)-3'-chloro-6-methoxy-[2,4'-bipyridin]-2'-yl)-1,2,3,4-tetrahydronaphthalen- 1-yl)amino)-2-methoxy-5-(trifluoromethyl)pyridin-3-yl)methyl)amino)cyclopropanecarboxylic acid (0.14 g, 0.15 mmol) in 1,4-dioxane (2 mL) was added concentrated HCl solution (0.3 mL) in one portion and the mixture was stirred at room temperature for 4 h. The mixture was then neutralized with aqueous ammonia and the mixture directly purified by reverse phase HPLC to afford 1-(((6-(((S)-5-(3'-chloro-6-methoxy-5-(((((S)-5-oxopyrrolidin-2-yl)methyl)amino)methyl)-[2,4'-bipyridin]-2'-yl)-1,2,3,4-tetrahydronaphthalen-1-yl)amino)-2-methoxy-5-(trifluoromethyl)pyridin-3-yl)methyl)amino)cyclopropanecarboxylic acid (19.2 mg, 15% yield) as a white solid. MS: *m*/*z* found 780 [M+H]⁺, retention time = 3.30 min (Method A). ¹H NMR (400 MHz, Methanol-*d*₄): δ 8.51 (t, 1H), 7.74-7.73 (m, 2H), 7.61 (d, 1H), 7.34-7.28 (m, 2H), 7.20-7.15 (m, 1H), 7.07-7.05 (m, 1H), 6.14-5.91 (m, 1H), 5.53-5.42 (m, 1H), 4.17 (d, 2H), 4.00-3.78 (m, 9H), 2.72-2.66 (m, 2H), 2.51-2.21 (m, 5H), 2.02-1.98 (m, 2H), 1.76-1.71 (m, 3H), 1.31-1.27 (m, 2H), 1.11-1.07 (m, 2H).

### Example 699: (S)-5-((((3'-Chloro-2'-((S)-5-((5-((((S)-2-hydroxypropyl)amino)methyl)-6-methoxy-3-(trifluoromethyl)pyridin-2-yl)amino)-5,6,7,8-tetrahydronaphthalen-1-yl)-6-methoxy-[2,4'-bipyridin]-5-yl)methyl)amino)methyl)pyrrolidin-2-one (707)

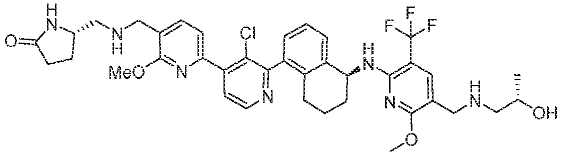

(S)-5-((((3'-Chloro-2'-((S)-5-((5-((((S)-2-hydroxypropyl)amino)methyl)-6-methoxy-3-(trifluoromethyl)pyridin-2-yl)amino)-5,6,7,8-tetrahydronaphthalen- 1-yl)-6-methoxy-[2,4'-bipyridin]-5-yl)methyl)amino)methyl)pyrrolidin-2-one was prepared in a similar fashion to Example 698, replacing 1-aminocyclopropanecarboxylic acid with (S)-1-aminopropan-2-ol in step (c). White solid, MS: *m*/*z* found 754 [M+H]⁺, retention time = 3.11 min (Method A). ¹H NMR (400 MHz, Methanol-*d*₄): δ 8.63-8.61 (m, 1H), 7.83 (d, 1H), 7.73 (d, 1H), 7.68 (s, 1H), 7.46-7.42 (m, 2H), 7.34-7.29 (m, 1H), 7.18 (d, 1H), 5.85-5.61 (m, 1H), 5.60-5.54 (m, 1H), 4.06 (s, 3H), 3.99-3.85 (m, 7H), 3.77-3.67 (m, 2H), 2.73-2.34 (m, 9H), 2.18-1.83 (m, 5H), 1.19 (d, 3H).

### Example 700: (S)-5-((((3'-Chloro-6-methoxy-2'-((S)-5-((6-methoxy-5-(((((S)-5-oxopyrrolidin-2-yl)methyl)amino)methyl)-3-(trifluoromethyl)pyridin-2-yl)amino)-5,6,7,8-tetrahydronaphthalen-1-yl)-[2,4'-bipyridin]-5-yl)methyl)amino)methyl)pyrrolidin-2-one (709)

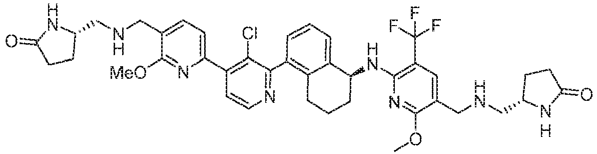

(S)-5-((((3'-Chloro-6-methoxy-2'-((S)-5-((6-methoxy-5-(((((S)-5-oxopyrrolidin-2-yl)methyl)amino)methyl)-3-(trifluoromethyl)pyridin-2-yl)amino)-5,6,7,8-tetrahydronaphthalen-1-yl)-[2,4'-bipyridin]-5-yl)methyl)amino)methyl)pyrrolidin-2-one was prepared in a similar fashion to Example 698, replacing 1-aminocyclopropanecarboxylic acid with (S)-5-(aminomethyl)pyrrolidin-2-one hydrochloride salt in step (c). White solid, MS: *m*/*z* found 793 [M+H]⁺, retention time = 3.21 min (Method A). ¹H NMR (400 MHz, Methanol-*d*₄): δ 8.63-8.61 (m, 1H), 7.83 (d, 1H), 7.73 (d, 1H), 7.69 (s, 1H), 7.46-7.42 (m, 2H), 7.34-7.29 (m, 1H), 7.18 (d, 1H), 5.84-5.62 (m, 1H), 5.60-5.54 (m, 1H), 4.06 (s, 3H), 3.99-3.83 (m, 7H), 3.71-3.67 (m, 2H), 2.74-2.23 (m, 12H), 2.21-1.74 (m, 6H).

### Example 701: 2-((6-(((S)-5-(3'-Chloro-6-methoxy-5-(((((S)-5-oxopyrrolidin-2-yl)methyl)amino)methyl)-[2,4'-bipyridin]-2'-yl)-1,2,3,4-tetrahydronaphthalen-1-yl)amino)-2-methoxy-5-(trifluoromethyl)pyridin-3-yl)methyl)-2,6-diazaspiro[3.4]octan-7-one (720)

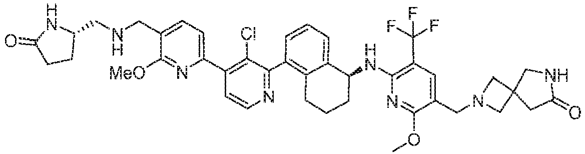

2-((6-(((S)-5-(3'-Chloro-6-methoxy-5-(((((S)-5-oxopyrrolidin-2-yl)methyl)amino)methyl)-[2,4'-bipyridin]-2'-yl)-1,2,3,4-tetrahydronaphthalen-1-yl)amino)-2-methoxy-5-(trifluoromethyl)pyridin-3-yl)methyl)-2,6-diazaspiro[3.4]octan-7-one was prepared in a similar fashion to Example 698, replacing 1-aminocyclopropanecarboxylic acid with 2,6-diazaspiro[3.4]octan-7-one trifluoroacetic acid salt and sodium acetate with triethylamine in step (c). White solid, MS: *m*/*z* found 805 [M+H]⁺, retention time = 3.06 min (Method A). ¹H NMR (400 MHz, Methanol-*d*₄): δ 8.51-8.49 (m, 1H), 7.71 (d, 1H), 7.61 (d, 1H), 7.51 (s, 1H), 7.33-7.30 (m, 2H), 7.21-7.17 (m, 1H), 7.06 (d, 1H), 5.72-5.50 (m, 1H), 5.48-5.40 (m, 1H), 3.94 (s, 3H), 3.89-3.85 (m, 3H), 3.79-3.66 (m, 3H), 3.46-3.45 (m, 4H), 3.25-3.24 (m, 4H), 2.60-2.17 (m, 9H), 2.05-1.92 (m, 2H), 1.85-1.62 (m, 3H).

### Example 702: (S)-N-(5-(3'-Chloro-6-methoxy-5-((methylamino)methyl)-[2,4'-bipyridin]-2'-yl)-1,2,3,4-tetrahydronaphthalen-1-yl)-6-methoxy-5-((methylamino)methyl)-3-(trifluoromethyl)pyridin-2-amine (732)

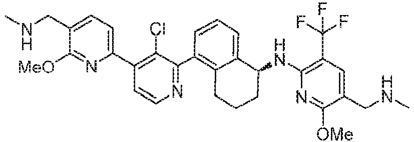

### (a) (S)-3'-Chloro-2'-(5-((5-formyl-6-methoxy-3-(trifluoromethyl)pyridin-2-yl)amino)-5,6,7,8-tetrahydronaphthalen-1-yl)-6-methoxy-[2,4'-bipyridine]-5-carbaldehyde

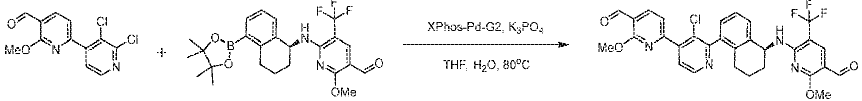

To a mixture of (S)-2-methoxy-6-((5-(4,4,5,5-tetramethyl-1,3,2-dioxaborolan-2-yl)-1,2,3,4-tetrahydronaphthalen-1-yl)amino)-5-(trifluoromethyl)nicotinaldehyde (0.21 g, 0.45 mmol) (Example 698, step (a)) and 2',3'-dichloro-6-methoxy-[2,4'-bipyridine]-5-carbaldehyde (0.07 g, 0.25 mmol) in THF/water mixture (10:1, 4.4 mL) was added chloro(2-dicyclohexylphosphino-2',4',6'-triisopropyl-1,1'-biphenyl)[2-(2'-amino-1,1'-biphenyl)]palladium(II) (0.02 g, 0.02 mmol) and potassium phosphate (0.16 g, 0.74 mmol) in one portion under N₂ and the mixture stirred at 80 °C for 4 h. The mixture was combined with another batch at the same scale and the mixture was concentrated. To the residue was added water (20 mL) and saturated aqueous brine solution (20 mL) and the mixture extracted with ethyl acetate/THF mixture (1:1, 50 mL). The organic phase was dried with anhydrous sodium sulfate, filtered and concentrated. The residue was purified by normal phase SiO₂ chromatography (0-15% ethyl acetate/petroleum ether) to afford (S)-3'-chloro-2'-(5-((5-formyl-6-methoxy-3-(trifluoromethyl)pyridin-2-yl)amino)-5,6,7,8-tetrahydronaphthalen-1-yl)-6-methoxy-[2,4'-bipyridine]-5-carbaldehyde (0.12 g, 40% yield) as a yellow gum. MS: *m*/*z* found 597 [M+H]⁺.

### (b) ((S)-N-(5-(3'-Chloro-6-methoxy-5-((methylamino)methyl)-[2,4'-bipyridin]-2'-yl)-1,2,3,4-tetrahydronaphthalen-1-yl)-6-methoxy-5-((methylamino)methyl)-3-(trifluoromethyl)pyridin-2-amine

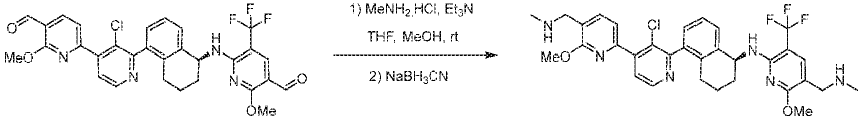

To a mixture of (S)-3'-chloro-2'-(5-((5-formyl-6-methoxy-3-(trifluoromethyl)pyridin-2-yl)amino)-5,6,7,8-tetrahydronaphthalen-1-yl)-6-methoxy-[2,4'-bipyridine]-5-carbaldehyde (0.11 g, 0.18 mmol) and methanamine hydrochloride salt (0.04 g, 0.52 mmol) in THF/Methanol mixture (3:2, 5 mL) was added triethylamine (0.04 g, 0.53 mmol) in one portion under N₂ and the mixture stirred at r.t. for 1 h. To the mixture was added sodium cyanoborohydride (0.06 g, 0.88 mmol)). The mixture was stirred at room temperature for 0.5 h, then water (1 mL) was added and the mixture concentrated. The residue was purified by reverse phase HPLC to afford (S)-N-(5-(3'-chloro-6-methoxy-5-((methylamino)methyl)-[2,4'-bipyridin]-2'-yl)-1,2,3,4-tetrahydronaphthalen-1-yl)-6-methoxy-5-((methylamino)methyl)-3-(trifluoromethyl)pyridin-2-amine hydrochloride salt (20 mg, 16% yield) as a yellow solid. The product was neutralized with saturated aqueous sodium bicarbonate solution and the mixture was extracted with dichloromethane (2 x 10 mL). The combined organic phases were dried with anhydrous sodium sulfate, filtered and concentrated. The residue was lyophilized to afford (S)-N-(5-(3'-chloro-6-methoxy-5-((methylamino)methyl)-[2,4'-bipyridin]-2'-yl)-1,2,3,4-tetrahydronaphthalen-1-yl)-6-methoxy-5-((methylamino)methyl)-3-(trifluoromethyl)pyridin-2-amine (17.2 mg, 58% yield) as a white solid. MS: *m*/*z* found 741 [M+TFA]⁺, retention time = 3.36 min (Method A). ¹H NMR (400 MHz, Methanol-*d*₄): δ 8.51-8.49 (m, 1H), 7.66 (d, 1H), 7.61 (d, 1H), 7.54 (s, 1H), 7.34-7.29 (m, 2H), 7.22-7.17 (m, 1H), 7.06 (d, 1H), 5.49-5.39 (m, 1H), 3.94 (s, 3H), 3.87-3.81 (m, 3H), 3.67 (s, 2H), 3.52 (s, 2H), 2.54-2.80 (m, 8H), 2.02-1.97 (m, 2H), 1.97-1.73 (m, 2H).

### Example 703: 5-(((2-Oxaspiro[3.3]heptan-6-yl)amino)methyl)-N-(3-(2-(4-(((2-oxaspiro[3.3]heptan-6-yl)amino)methyl)-3-methoxyphenyl)-3-chloropyridin-4-yl)-2-chlorophenyl)picolinamide (695)

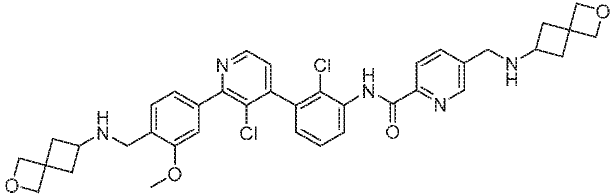

### (a) 2-Chloro-3-(2,3-dichloropyridin-4-yl)aniline

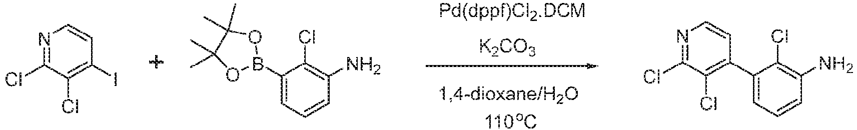

To a mixture of 2-chloro-3-(4,4,5,5-tetramethyl-1,3,2-dioxaborolan-2-yl)aniline (3.8 g, 15 mmol) and 2,3-dichloro-4-iodopyridine (3.5 g, 12.8 mmol) in 1,4-dioxane/water mixture (70:15, 85 mL) was added potassium carbonate (5.3 g, 38.3 mmol) and [1,1'-bis(diphenylphosphino)ferrocene]dichloropalladium(II) complex with dichloromethane (0.21 g, 0.26 mmo) in one portion under N₂. The mixture was stirred at 110 °C for 3 h. The mixture was concentrated. To the residue was added water (30 mL) and saturated aqueous brine solution (100 mL) and the mixture extracted with ethyl acetate/THF mixture (1:1, 200 mL). The organic phase was dried with anhydrous sodium sulfate, filtered and concentrated. The residue was purified by normal phase SiO₂ chromatography (0-15% ethyl acetate/petroleum ether) to afford 2-chloro-3-(2,3-dichloropyridin-4-yl)aniline (2.7 g, 77% yield) as a yellow solid. ¹H NMR (400 MHz, DMSO-*d*₆): δ 8.26 (d, 1H), 7.19-7.07 (m, 2H), 6.79 (dd, 1H), 6.52 (dd, 1H).

### (b) Methyl 6-((2-chloro-3-(2,3-dichloropyridin-4-yl)phenyl)carbamoyl)nicotinate

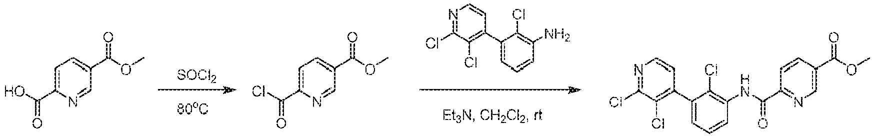

A mixture of 5-(methoxycarbonyl)picolinic acid (0.5 g, 2.76 mmol) in thionyl chloride (4 mL) was stirred at 80°C for 3 h under N₂. The mixture was concentrated to afford crude methyl 6-(chlorocarbonyl)nicotinate (0.55 g, crude) as a white solid. To a mixture of methyl 6-(chlorocarbonyl)nicotinate (0.55 g, 2.76 mmol) and 2-chloro-3-(2,3-dichloropyridin-4-yl)aniline (0.64 g, 2.34 mmol) in dichloromethane (3 mL) was added triethylamine (1.2 mL, 8.27 mmol) in one portion under N₂ and the mixture was stirred at room temperature for 12 h. Water (50 mL) was added to the reaction solution and the mixture was filtered. The filtrate cake was dried to give methyl 6-((2-chloro-3-(2,3-dichloropyridin-4-yl)phenyl)carbamoyl)nicotinate (0.65 g, 54% yield) as a white solid. MS: *m*/*z* found 436 and 438 [M+H]⁺.

### (c) N-(2-Chloro-3-(2,3-dichloropyridin-4-yl)phenyl)-5-(hydroxymethyl)picolinamide

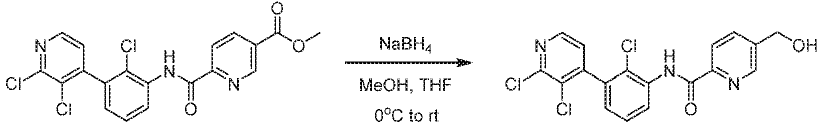

To a mixture of methyl 6-((2-chloro-3-(2,3-dichloropyridin-4-yl)phenyl)carbamoyl)nicotinate (0.65 g, 1.49 mmol) in MeOH/THF mixture (2:5, 70 mL) was added sodium borohydride (0.56 mg, 14.9 mmol) in portions at 0 °C under N₂. The mixture was stirred at room temperature for 12 h. To the mixture was added water (20 mL) to quench sodium borohydride and the mixture was concentrated. To the residue was added water (20 mL) and saturated aqueous brine solution (5 mL) and the mixture extracted with ethyl acetate/THF mixture (1:1, 50 mL). The organic phase was dried with anhydrous sodium sulfate, filtered and concentrated. The residue was purified by normal phase SiO₂ chromatography (0-50% (30% THF in ethyl acetate)/petroleum ether) to afford N-(2-chloro-3-(2,3-dichloropyridin-4-yl)phenyl)-5-(hydroxymethyl)picolinamide (0.4 g, 65% yield) as a light yellow solid. ¹H NMR (400 MHz, DMSO-*d*₆): δ 10.73 (s, 1H), 8.71 (d, 1H), 8.59 (d, 1H), 8.31 (d, 1H), 8.23 (d, 1H), 7.88 (dd, 1H), 7.38 (t, 1H), 7.13 (d, 1H), 6.93 (dd, 1H), 4.80 (s, 2H).

### (d) N-(2-chloro-3-(3-chloro-2-(4-formyl-3-methoxyphenyl)pyridin-4-yl)phenyl)-5-(hydroxymethyl)picolinamide

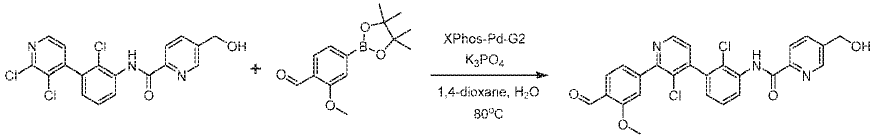

To a mixture of N-(2-chloro-3-(2,3-dichloropyridin-4-yl)phenyl)-5-(hydroxymethyl)picolinamide (0.2 g, 0.49 mmol) and 2-methoxy-4-(4,4,5,5-tetramethyl-1,3,2-dioxaborolan-2-yl)benzaldehyde (0.17 g, 0.64 mmol) (Example 714, step (f)) in 1,4-dioxane/water mixture (6:1, 7 mL) was added chloro(2-dicyclohexylphosphino-2',4',6'-triisopropyl-1,1'-biphenyl)[2-(2'-amino-1,1'-biphenyl) palladium (II) (0.04 g, 0.05 mmol) and potassium phosphate (0.31 g, 1.47 mmol) in one portion under N₂. The mixture was stirred at 80 °C for 12 hr. To the mixture was added water (10 mL) and the mixture was extracted with ethyl acetate (2 x 50 mL). The combined organic phases were washed with saturated aqueous brine solution (2 x 50 mL), dried with anhydrous sodium sulfate, filtered and concentrated. The residue was purified by normal phase SiO₂ chromatography (0-30% (30% THF in ethyl acetate)/petroleum ether) to afford N-(2-chloro-3-(3-chloro-2-(4-formyl-3-methoxyphenyl)pyridin-4-yl)phenyl)-5-(hydroxymethyl)picolinamide (90 mg, 32% yield) as a yellow solid. ¹H NMR (400 MHz, DMSO-*d*₆): δ 10.80 (s, 1H), 10.48 (s, 1H), 8.83 (d, 1H), 8.74 (d, 1H), 8.59-8.57 (m, 1H), 8.26 (d, 1H), 8.10 (dd, 1H), 7.88 (d, 1H), 7.67-7.62 (m, 2H), 7.57 (d, 1H), 7.46 (d, 1H), 7.34 (dd, 1H), 5.60 (t, 1H), 4.73 (d, 2H), 4.05 (s, 3H).

### (e) N-(2-Chloro-3-(3-chloro-2-(4-formyl-3-methoxyphenyl)pyridin-4-yl)phenyl)-5-formylpicolinamide

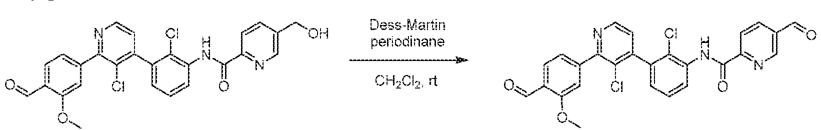

To a mixture of N-(2-chloro-3-(3-chloro-2-(4-formyl-3-methoxyphenyl)pyridin-4-yl)phenyl)-5-(hydroxymethyl)picolinamide (0.08 g, 0.16 mmol) in dichloromethane (4 mL) was added Dess-Martin periodinane (0.09 g, 0.2 mmol) in one portion under N₂ and the mixture was stirred at room temperature for 1 hr. The mixture was purified directly without work-up by normal phase SiO₂ chromatography (0-30% (30% THF in ethyl acetate)/petroleum ether) to afford N-(2-chloro-3-(3-chloro-2-(4-formyl-3-methoxyphenyl)pyridin-4-yl)phenyl)-5-formylpicolinamide (70 mg, 62% yield) as a light yellow solid. MS: *m*/*z* found 506 [M+H]⁺.

### (f) 5-(((2-Oxaspiro[3.3]heptan-6-yl)amino)methyl)-N-(3-(2-(4-(((2-oxaspiro[3.3]heptan-6-yl)amino)methyl)-3-methoxyphenyl)-3-chloropyridin-4-yl)-2-chlorophenyl)picolinamide

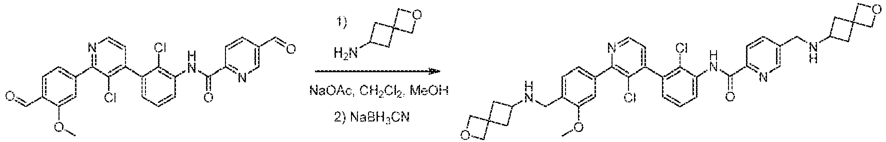

To a mixture of N-(2-chloro-3-(3-chloro-2-(4-formyl-3-methoxyphenyl)pyridin-4-yl)phenyl)-5-formylpicolinamide (0.06 g, 0.12 mmol) and 2-oxaspiro[3.3]heptan-6-amine hydrochloride salt (0.04 g, 0.3 mmol) in dichloromethane/methanol mixture (1:1, 4 mL) was added sodium acetate (0.06 g, 0.71 mmol) in one portion under N₂. The mixture was stirred at room temperature for 2 hr then sodium cyanoborohydride (0.03 g, 0.47 mmol) was added. The mixture was stirred at room temperature for 1 hr. The mixture was concentrated and the residue was purified by reverse phase HPLC to afford 5-(((2-oxaspiro[3.3]heptan-6-yl)amino)methyl)-N-(3-(2-(4-(((2-oxaspiro[3.3]heptan-6-yl)amino)methyl)-3-methoxyphenyl)-3-chloropyridin-4-yl)-2-chlorophenyl)picolinamide (17.6 mg , 19% yield) as a white solid (formic acid salt). MS: *m*/*z* found 700 [M+H]⁺, retention time = 0.70 min (Method A). ¹H NMR (400 MHz, Methanol-*d*₄): δ 8.63 (s, 1H), 8.60 (d, 1H), 8.57 (dd, 1H), 8.39 (br s, 1H), 8.19 (d, 1H), 7.99 (d, 1H), 7.47 (t, 1H), 7.43 (d, 1H), 7.39 (d, 1H), 7.32 (d, 1H), 7.28 (dd, 1H), 7.13 (dd, 1H), 4.67 (s, 2H), 4.64 (s, 2H), 4.57 (s, 2H), 4.54 (s, 2H), 4.08 (s, 2H), 3.93 (s, 3H), 3.88 (s, 2H), 3.64-3.57 (m, 1H), 3.29-3.27 (m, 1H), 2.65-2.61 (m, 2H), 2.53-2.52 (m, 2H), 2.35-2.32 (m, 2H), 2.08-2.07 (m, 2H).

### Example 704: 5-((2-Oxa-6-azaspiro[3.3]heptan-6-yl)methyl)-N-(3-(2-(4-((2-oxa-6-azaspiro[3.3]heptan-6-yl)methyl)-3-methoxyphenyl)-3-chloropyridin-4-yl)-2-chlorophenyl)picolinamide (701)

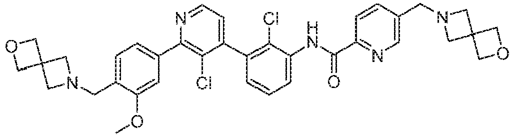

### (a) 5-((2-Oxa-6-azaspiro[3.3]heptan-6-yl)methyl)-N-(3-(2-(4-((2-oxa-6-azaspiro[3.3]heptan-6-yl)methyl)-3-methoxyphenyl)-3-chloropyridin-4-yl)-2-chlorophenyl)picolinamide

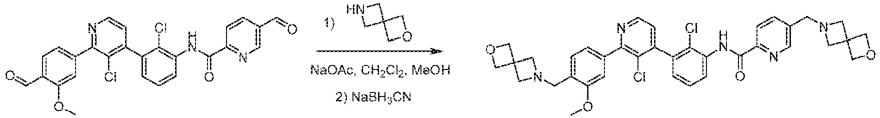

To a mixture of N-(2-chloro-3-(3-chloro-2-(4-formyl-3-methoxyphenyl)pyridin-4-yl)phenyl)-5-formylpicolinamide (0.1 g, 0.20 mmol) (Example 703, step (e)) and 2-oxa-6-azaspiro[3.3]heptane (0.05 g, 0.49 mmol) in methanol/ dichloromethane mixture (1:1, 4 mL) was added sodium acetate (0.1 g, 1.18 mmol) in one portion under N₂ and the mixture stirred at room temperature for 11 hr. Sodium cyanoborohydride (0.05 g, 0.79 mmol) was then added and the mixture stirred at room temperature for 1 hr. The mixture was concentrated and the residue purified by prep-HPLC to afford 5-((2-oxa-6-azaspiro[3.3]heptan-6-yl)methyl)-N-(3-(2-(4-((2-oxa-6-azaspiro[3.3]heptan-6-yl)methyl)-3-methoxyphenyl)-3-chloropyridin-4-yl)-2-chlorophenyl)picolinamide (11.7 mg, 8% yield) as as a white solid (formic acid salt). MS: m/z found 672 [M+H]⁺, retention time = 2.75 min (Method A). ¹H NMR (400 MHz, Methanol-*d*₄): δ 8.69-8.68 (m, 2H), 8.66-8.65 (m, 1H), 8.45 (br s, 1H), 8.25 (d, 1H), 7.99 (d, 1H), 7.56 (t, 1H), 7.51-7.47 (m, 2H), 7.41 (s, 1H), 7.38 (d, 1H), 7.22 (dd, 1H), 4.81 (s, 4H), 4.77 (s, 4H), 4.31 (s, 2H), 4.25 (s, 4H), 4.00 (s, 3H), 3.80 (s, 2H), 3.56 (s, 4H).

### Example 705: N-(2-Chloro-3-(3-chloro-2-(4-(((3-fluoropropyl)amino)methyl)-3-methoxyphenyl)pyridin-4-yl)phenyl)-5-(((3-fluoropropyl)amino)methyl)picolinamide (702)

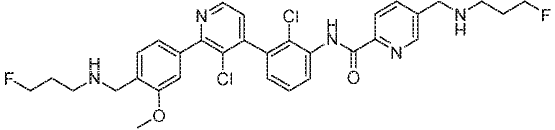

### (a) N-(2-Chloro-3-(3-chloro-2-(4-(((3-fluoropropyl)amino)methyl)-3-methoxyphenyl)pyridin-4-yl)phenyl)-5-(((3-fluoropropyl)amino)methyl)picolinamide

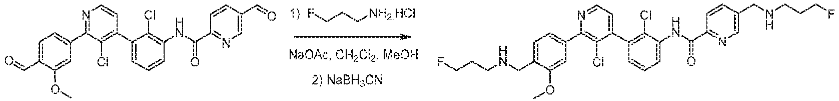

To a mixture of N-(2-chloro-3-(3-chloro-2-(4-formyl-3-methoxyphenyl)pyridin-4-yl)phenyl)-5-formylpicolinamide (0.1 g, 0.2 mmol) (Example 703, step (e)) and 3-fluoropropan-1-amine hydrochloride salt (0.06 g, 0.5 mmol) in dichloromethane/methanol mixture (1:1, 1 mL) was added sodium acetate (0.1 g, 1.18 mmol) in one portion under N₂ and the mixture stirred at room temperature for 12 hr. Sodium cyanoborohydride (0.05 g, 0.79 mmol) was then added and the mixture was stirred at room temperature for 1 hr. The mixture was concentrated and the residue was purified by reverse phase HPLC to afford N-(2-chloro-3-(3-chloro-2-(4-(((3-fluoropropyl)amino)methyl)-3-methoxyphenyl)pyridin-4-yl)phenyl)-5-(((3-fluoropropyl)amino)methyl)picolinamide (14.4 mg, 10% yield) as a white solid (formic acid salt). MS: *m*/*z* found 628 [M+H]⁺, retention time = 2.97 min (Method A). 1H NMR (400 MHz, Methanol-*d*₄): δ 8.75 (s, 1H), 8.70-8.66 (m, 2H), 8.51 (s, 1H), 8.30 (d, 1H), 8.10 (d, 1H), 7.58-7.54 (m, 2H), 7.49 (d, 1H), 7.43 (s, 1H), 7.38 (d, 1H), 7.22 (dd, 1H), 4.67 (t, 1H), 4.62 (t, 1H), 4.55 (t, 1H), 4.50 (t, 1H), 4.32 (s, 2H), 4.08 (s, 2H), 4.02 (s, 3H), 3.24 (t, 2H), 2.93 (t, 2H), 2.20-1.96 (m, 4H).

### Example 706: 5-(((2-Acetyl-2-azaspiro[3.3]heptan-6-yl)amino)methyl)-N-(3-(2-(4-(((2-acetyl-2-azaspiro[3.3]heptan-6-yl)amino)methyl)-3-methoxyphenyl)-3-chloropyridin-4-yl)-2-chlorophenyl)picolinamide (706)

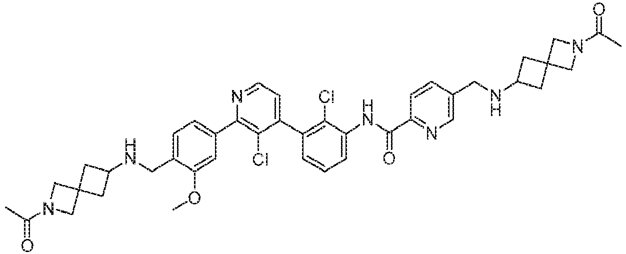

### (a) 5-(((2-Acetyl-2-azaspiro[3.3]heptan-6-yl)amino)methyl)-N-(3-(2-(4-(((2-acetyl-2-azaspiro[3.3]heptan-6-yl)amino)methyl)-3-methoxyphenyl)-3-chloropyridin-4-yl)-2-chlorophenyl)picolinamide

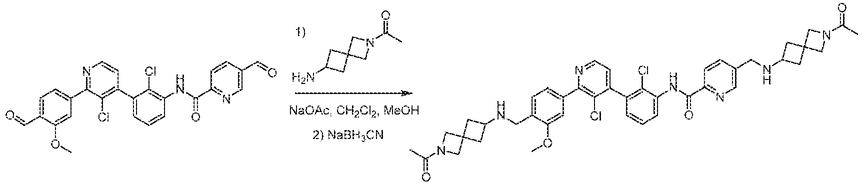

To a mixture of N-(2-chloro-3-(3-chloro-2-(4-formyl-3-methoxyphenyl)pyridin-4-yl)phenyl)-5-formylpicolinamide (0.13 g, 0.26 mmol) (Example 703, step (e)) and 1-(6-amino-2-azaspiro[3.3]heptan-2-yl)ethanone (0.1 g, 0.65 mmol) (Example 375, step (d)) in methanol/dichloromethane mixture (1:1, 4 mL) was added sodium acetate (0.1 g, 1.31 mmol) in one portion at room temperature under N₂ and the mixture stirred at room temperature for 11 hr. Sodium cyanoborohydride (0.07 g, 1.05 mmol) was then added and the mixture stirred at room temperature for 1 hr. The mixture was concentrated and the residue purified by prep-HPLC to afford 5-(((2-acetyl-2-azaspiro[3.3]heptan-6-yl)amino)methyl)-N-(3-(2-(4-(((2-acetyl-2-azaspiro[3.3]heptan-6-yl)amino)methyl)-3-methoxyphenyl)-3-chloropyridin-4-yl)-2-chlorophenyl)picolinamide (8.7 mg 4% yield) as a white solid. MS: *m*/*z* found 782 [M+H]⁺, retention time = 2.73 min (Method A). ¹H NMR (400 MHz, Methanol-*d*₄): δ 8.60-8.58 (m, 3H), 8.17 (d, 1H), 7.96 (dd, 1H), 7.48 (t, 1H), 7.38 (d, 1H), 7.36 (d, 1H), 7.25-7.22 (m, 2H), 7.13 (dd, 1H), 4.15 (d, 2H), 4.05 (d, 2H), 3.92-3.89 (m, 5H), 3.82-3.81 (m, 4H), 3.75 (s, 2H), 3.35-3.32 (m, 1H), 3.19-3.13 (m, 1H), 2.46-2.40 (m, 4H), 2.08-2.07 (m, 2H), 1.99-1.94 (m, 2H), 1.78-1.77 (m, 6H).

### Example 707: N-(3-(2-(4-(((1-Acetylpiperidin-4-yl)amino)methyl)-3-methoxyphenyl)-3-chloropyridin-4-yl)-2-chlorophenyl)-5-(((2-hydroxyethyl)amino)methyl)picolinamide (733)

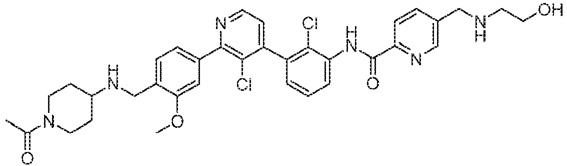

### (a) N-(2-Chloro-3-(2,3-dichloropyridin-4-yl)phenyl)-5-formylpicolinamide

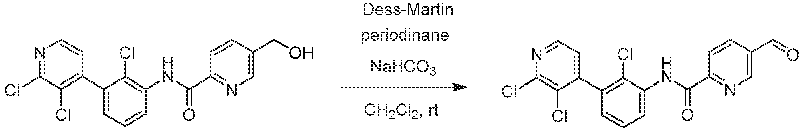

To a mixture of N-(2-chloro-3-(2,3-dichloropyridin-4-yl)phenyl)-5-(hydroxymethyl)picolinamide (Example 703, step (c)) (1 g, 2.45 mmol) in dichloromethane (10 mL) was added sodium bicarbonate (0.41 g, 4.89 mmol) and Dess-Martin periodinane (1.14 g, 2.69 mmol) in one portion under N₂ and the mixture was stirred at room temperature for 1 hr. The mixture was purified directly without work-up by normal phase SiO₂ chromatography (0-15% (10% THF in ethyl acetate)/petroleum ether) to afford N-(2-chloro-3-(2,3-dichloropyridin-4-yl)phenyl)-5-formylpicolinamide (0.37 g, 22% yield) as a white solid. ¹H NMR (400 MHz, DMSO-*d*₆): δ 10.78 (s, 1H), 10.24 (s, 1H), 9.24 (s, 1H), 8.54-8.53 (m, 2H), 8.46-8.40 (m, 2H), 7.56 (m, 2H), 7.30 (d, 1H).

### (b) tert-Butyl ((6-((2-chloro-3-(2,3-dichloropyridin-4-yl)phenyl)carbamoyl)pyridin-3-yl)methyl)(2-hydroxyethyl)carbamate

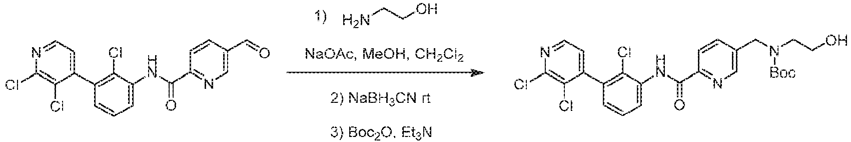

To a mixture of N-(2-chloro-3-(2,3-dichloropyridin-4-yl)phenyl)-5-formylpicolinamide (0.47 g, 1.16 mmol) and 2-aminoethanol (0.08 g, 1.27 mmol) in dichloromethane/methanol mixture (2:1, 30 mL) was added sodium acetate and the mixture stirred at room temperature for 12 hr under N₂. Sodium cyanoborohydride (0.22 g, 3.47 mmol) was then added and the mixture stirred at room temperature for 1 hr. Di-tert-butyl dicarbonate (0.31 mL, 1.35 mmol) and triethylamine (0.14 mL, 1.04 mmol) were then added in one portion under N₂ and the mixture was stirred at room temperature for 1 hr. Water (10 mL) was added and the mixture was extracted with ethyl acetate (2 x 10 mL). The combined organic phases were washed with saturated aqueous brine solution (2 x 10 mL), dried with anhydrous sodium sulfate, filtered and concentrated. The residue was purified by normal phase SiO₂ chromatography (15-50% ethyl acetate/petroleum ether) to afford tert-butyl ((6-((2-chloro-3-(2,3-dichloropyridin-4-yl)phenyl)carbamoyl)pyridin-3-yl)methyl)(2-hydroxyethyl)carbamate (0.47 g, 52% yield) as a yellow oil. ¹H NMR (400 MHz, DMSO-*d*₆): δ 11.75 (s, 1H), 10.76 (s, 1H), 8.59-8.56 (m, 2H), 8.26 (d, 1H), 8.02 (d, 1H), 7.66-7.61 (m, 2H), 7.32 (dd, 1H), 4.79 (t, 1H), 4.64-4.61 (m, 2H), 3.57-3.54 (m, 2H), 3.33-3.32 (m, 2H), 1.49-1.36 (m, 9H).

### (c) tert-Butyl ((6-((2-chloro-3-(3-chloro-2-(4-formyl-3-methoxyphenyl)pyridin-4-yl)phenyl)carbamoyl)pyridin-3-yl)methyl)(2-hydroxyethyl)carbamate

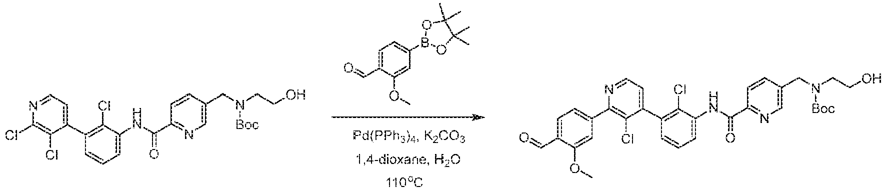

To a mixture of tert-butyl ((6-((2-chloro-3-(2,3-dichloropyridin-4-yl)phenyl)carbamoyl)pyridin-3-yl)methyl)(2-hydroxyethyl)carbamate (0.12 g, 0.22 mmol) and 2-methoxy-4-(4,4,5,5-tetramethyl-1,3,2-dioxaborolan-2-yl)benzaldehyde (Example 714, step (f)) (0.07 g, 0.28 mmol) in 1,4-dioxane /water mixture (4:1, 5 mL) was added tetrakis(triphenylphosphine)palladium(0) (0.02 g, 0.02 mmol) and potassium carbonate (0.09 g, 0.65 mmol) in one portion under N₂. The mixture was stirred at 110 °C for 3 hr then the mixture was combined with additional batches at 30 mg and 130 mg scale. The combined reaction mixtures were concentrated, water (20 mL) and ethyl acetate (20 mL) added to the residue and the mixture extracted with ethyl acetate (2 x 50 mL). The combined organic phases were washed with saturated aqueous brine solution (2 x 50 mL), dried with anhydrous sodium sulfate, filtered and concentrated. The residue was purified by normal phase SiO₂ chromatography (0-25% ethyl acetate/petroleum ether) to afford tert-butyl ((6-((2-chloro-3-(3-chloro-2-(4-formyl-3-methoxyphenyl)pyridin-4-yl)phenyl)carbamoyl)pyridin-3-yl)methyl)(2-hydroxyethyl)carbamate (0.24 g, 72% yield) as a white solid. MS: *m*/*z* found 651 [M+H]⁺.

### (d) tert-Butyl ((6-((3-(2-(4-(((1-acetylpiperidin-4-yl)amino)methyl)-3-methoxyphenyl)-3-chloropyridin-4-yl)-2-chlorophenyl)carbamoyl)pyridin-3-yl)methyl)(2-hydroxyethyl)carbamate

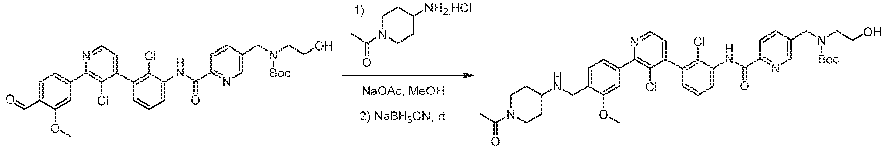

To a mixture of tert-butyl ((6-((2-chloro-3-(3-chloro-2-(4-formyl-3-methoxyphenyl)pyridin-4-yl)phenyl)carbamoyl)pyridin-3-yl)methyl)(2-hydroxyethyl)carbamate (0.2 g, 0.3 mmol) and 1-(4-aminopiperidin-1-yl)ethanone hydrochloride salt (0.08 g, 0.46 mmol) in methanol/THF mixture (1:1, 5 mL) was added sodium acetate (0.07 g, 0.9 mmol) in one portion under N₂ and the mixture was stirred at room temperature for 12 hr. Sodium cyanoborohydride (0.06 g, 0.92 mmol) was then added and the mixture stirred at room temperature for 1 hr. The mixture was concentrated and the residue purified by normal phase SiO₂ chromatography (0-50% methanol /ethyl acetate) to afford tert-butyl ((6-((3-(2-(4-(((1-acetylpiperidin-4-yl)amino)methyl)-3-methoxyphenyl)-3-chloropyridin-4-yl)-2-chlorophenyl)carbamoyl)pyridin-3-yl)methyl)(2-hydroxyethyl)carbamate (0.18 g, 62% yield) as a white solid. MS: *m*/*z* found 777 [M+H]⁺.

### (e) N-(3-(2-(4-(((1-Acetylpiperidin-4-yl)amino)methyl)-3-methoxyphenyl)-3-chloropyridin-4-yl)-2-chlorophenyl)-5-(((2-hydroxyethyl)amino)methyl)picolinamide

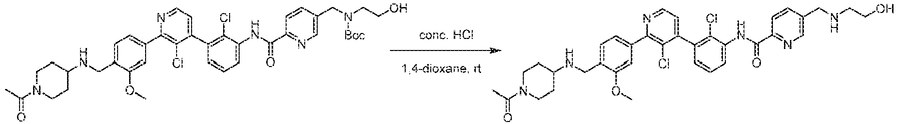

To a mixture of tert-butyl ((6-((3-(2-(4-(((1-acetylpiperidin-4-yl)amino)methyl)-3-methoxyphenyl)-3-chloropyridin-4-yl)-2-chlorophenyl)carbamoyl)pyridin-3-yl)methyl)(2-hydroxyethyl)carbamate (0.17 g, 0.22 mmol) in 1,4-dioxane (1 mL) was added concentrated HCl solution (3 mL) in one portion under N₂ and the mixture was stirred at room temperature for 3 hr. The mixture was neutralized with ammonia water and the mixture concentrated. The residue was purified by reverse phase HPLC to afford N-(3-(2-(4-(((1-acetylpiperidin-4-yl)amino)methyl)-3-methoxyphenyl)-3-chloropyridin-4-yl)-2-chlorophenyl)-5-(((2-hydroxyethyl)amino)methyl)picolinamide (9.3 mg, 5% yield) as a white solid. MS: *m*/*z* found 677 [M+H]⁺, retention time = 2.65 min (Method A). ¹H NMR (400 MHz, Methanol-*d*₄): δ 8.72 (d, 1H), 8.69-8.65 (m, 2H), 8.26 (d, 1H), 8.07 (dd, 1H), 7.55 (t, 1H), 7.45-7.44 (m, 2H), 7.32-7.30 (m, 2H), 7.21 (dd, 1H), 4.52-4.47 (m, 1H), 3.98-3.92 (m, 8H), 3.71 (t, 2H), 3.15-3.12 (m, 1H), 2.84-2.68 (m, 4H), 2.12 (s, 3H), 2.09-2.03 (m, 2H), 1.43-1.32 (m, 2H).

### Example 708: N-(2-Chloro-3-(3-chloro-2-(3-methoxy-4-((7-oxo-2,6-diazaspiro[3.4]octan-2-yl)methyl)phenyl)pyridin-4-yl)phenyl)-5-(((2-hydroxyethyl)amino)methyl)picolinamide (754)

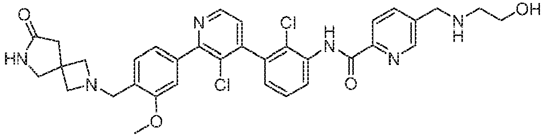

N-(2-Chloro-3-(3-chloro-2-(3-methoxy-4-((7-oxo-2,6-diazaspiro[3.4]octan-2-yl)methyl)phenyl)pyridin-4-yl)phenyl)-5-(((2-hydroxyethyl)amino)methyl)picolinamide was prepared in a similar fashion to Example 707, replacing 1-(4-aminopiperidin-1-yl)ethanone hydrochloride salt with 2,6-diazaspiro[3.4]octan-7-one in step (d). MS: *m*/*z* found 661 [M+H]⁺, retention time = 2.56 min (Method A). ¹H NMR (400 MHz, Methanol-*d*₄): δ 8.72-8.65 (m, 3H), 8.26 (d, 1H), 8.07 (dd, 1H), 7.55 (t, 1H), 7.45-7.39 (m, 2H), 7.30-7.28 (m, 2H), 7.21 (dd, 1H), 3.98 (s, 2H), 3.93 (s, 3H), 3.79 (s, 2H), 3.71 (t, 2H), 3.59 (s, 2H), 3.44 (s, 4H), 2.78 (t, 2H), 2.58 (s, 2H).

### Example 709: (S)-N-(2-Chloro-3-(3-chloro-2-(3-methoxy-4-((((5-oxopyrrolidin-2-yl)methyl)amino)methyl)phenyl)pyridin-4-yl)phenyl)-5-(((2-hydroxyethyl)amino)methyl)picolinamide (755)

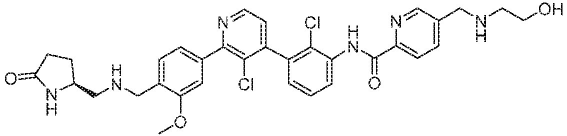

(S)-N-(2-Chloro-3-(3-chloro-2-(3-methoxy-4-((((5-oxopyrrolidin-2-yl)methyl)amino)methyl)phenyl)pyridin-4-yl)phenyl)-5-(((2-hydroxyethyl)amino)methyl)picolinamide was prepared in a similar fashion to Example 707, replacing 1-(4-aminopiperidin-1-yl)ethanone hydrochloride salt with (S)-5-(aminomethyl)pyrrolidin-2-one as a hydrochloride salt in step (d). MS: *m*/*z* found 649 [M+H]⁺, retention time = 2.56 min (Method A). ¹H NMR (400 MHz, Methanol-*d*₄): δ 8.72-8.65 (m, 3H), 8.26 (d, 1H), 8.07 (dd, 1 H), 7.55 (t, 1H), 7.45-7.42 (m, 2H), 7.31-7.29 (m, 2H), 7.21 (dd, 1H), 3.97 (s, 2H), 3.95 (s, 3H), 3.90-3.89 (m, 2H) 3.87-3.82 (m, 1H), 3.71 (t, 2H), 2.78 (t, 2H), 2.72 - 2.69 (m, 2H), 2.37-2.26 (m, 3H), 1.84-1.77 (m, 1H).

### Example 710: N-(3-(2-(4-((6-Acetyl-2,6-diazaspiro[3.3]heptan-2-yl)methyl)-3-methoxyphenyl)-3-chloropyridin-4-yl)-2-chlorophenyl)-5-(((2-hydroxyethyl)amino)methyl)picolinamide (757)

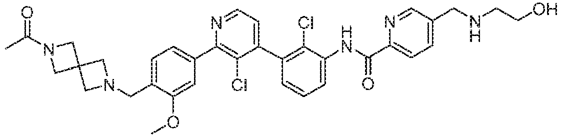

N-(3-(2-(4-((6-Acetyl-2,6-diazaspiro[3.3]heptan-2-yl)methyl)-3-methoxyphenyl)-3-chloropyridin-4-yl)-2-chlorophenyl)-5-(((2-hydroxyethyl)amino)methyl)picolinamide was prepared in a similar fashion to Example 707, replacing 1-(4-aminopiperidin-1-yl)ethanone hydrochloride salt with 1-(2,6-diazaspiro[3.3]heptan-2-yl)ethan-1-one trifluoroacetic acid salt in step (d). MS: *m*/*z* found 675 [M+H]⁺, retention time = 2.61 min (Method A). ¹H NMR (400 MHz, Methanol-*d*₄): δ 8.72-8.65 (m, 3H), 8.26 (d, 1H), 8.07 (dd, 1 H), 7.55 (t, 1H), 7.44 (d, 1H), 7.38 (d, 1H), 7.29-7.27 (m, 2H), 7.21 (dd, 1H), 4.30 (s, 2H), 4.05 (s, 2H), 3.97 (s, 2H), 3.92 (s, 3H), 3.74 (s, 2H), 3.71 (t, 2H), 3.51 (q, 4H), 2.77 (t, 2H), 1.86 (s, 3H).

### Example 711: N-(2-Chloro-3-(3-chloro-2-(3-methoxy-4-(((tetrahydro-2H-pyran-4-yl)amino)methyl)phenyl)pyridin-4-yl)phenyl)-5-(((2-hydroxyethyl)amino)methyl)picolinamide (768)

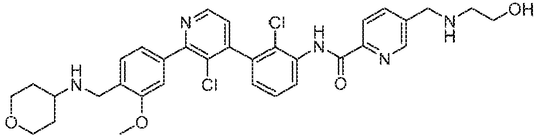

N-(2-Chloro-3-(3-chloro-2-(3-methoxy-4-(((tetrahydro-2H-pyran-4-yl)amino)methyl)phenyl)pyridin-4-yl)phenyl)-5-(((2-hydroxyethyl)amino)methyl)picolinamide was prepared in a similar fashion to Example 707, replacing 1-(4-aminopiperidin-1-yl)ethanone hydrochloride salt with tetrahydro-2H-pyran-4-amine in step (d). MS: *m*/*z* found 636 [M+H]⁺, retention time = 2.74 min (Method A). ¹H NMR (400 MHz, Methanol-*d*₄): δ 8.72-8.65 (m, 3H), 8.26 (d, 1H), 8.07 (dd, 1H), 7.55 (t, 1H), 7.45-7.42 (m, 2H), 7.31-7.28 (m, 2H), 7.21 (dd, 1H), 3.99-3.95 (m, 7H), 3.92 (s, 2H), 3.71 (t, 2H), 3.45-3.38 (m, 2H), 2.77 (t, 3H), 1.92 (dd, 2H), 1.54-1.44 (m, 2H).

### Example 712: (S)-N-(2-Chloro-3-(3-chloro-2-(3-methoxy-4-((((5-oxopyrrolidin-2-yl)methyl)amino)methyl)phenyl)pyridin-4-yl)phenyl)-5-((methylamino)methyl)picolinamide (751)

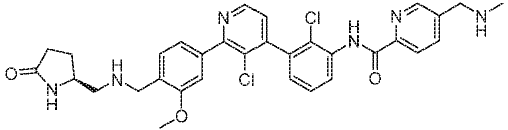

### (a) tert-Butyl (S)-(4-(3-chloro-4-(2-chloro-3-(5-(hydroxymethyl)picolinamido)phenyl)pyridin-2-yl)-2-methoxybenzyl)((5-oxopyrrolidin-2-yl)methyl)carbamate

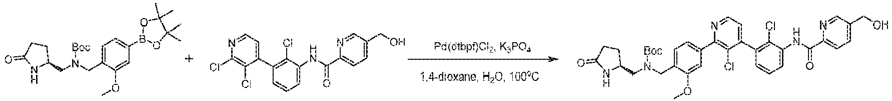

To a mixture of N-(2-chloro-3-(2,3-dichloropyridin-4-yl)phenyl)-5-(hydroxymethyl)picolinamide (Example 703, step (c)) (0.35 g, 0.86 mmol) and tert-butyl (S)-(2-methoxy-4-(4,4,5,5-tetramethyl-1,3,2-dioxaborolan-2-yl)benzyl)((5-oxopyrrolidin-2-yl)methyl)carbamate (Example 542, step (p)) (0.55 g, 1.2 mmol) in 1,4-dioxane/water mixture (10:1, 8.8 mL) was added [1,1'-bis(di-tert-butylphosphino)ferrocene] dichloropalladium(II) (0.03 g, 0.04 mmol) and potassium phosphate (0.55 g, 2.57 mmol) in one portion under N₂ and the mixture stirred at 100 °C for 12 h. The mixture was combined with another batch at 50 mg scale. The mixture was concentrated and the residue purified by normal phase SiO₂ chromatography (0-15% (30% THF in ethyl acetate)/petroleum ether) to afford tert-butyl (S)-(4-(3-chloro-4-(2-chloro-3-(5-(hydroxymethyl)picolinamido)phenyl)pyridin-2-yl)-2-methoxybenzyl)((5-oxopyrrolidin-2-yl)methyl)carbamate (0.35 g, 50% yield) as a yellow solid. ¹H NMR (400 MHz, Methanol-*d*₄) δ 8.70-8.65 (m, 3H), 8.27 (d, 1H), 8.04 (dd, 1H), 7.55 (t, 1H), 7.44 (d, 1H), 7.31-7.25 (m, 3H), 7.21 (dd, 1H), 4.78 (s, 2H), 3.95-3.92 (m, 4H), 3.42-3.37 (m, 2H), 2.32-2.26 (m, 3H), 1.86-1.81 (m, 1H), 1.53-1.44 (m, 9H).

### (b) tert-Butyl (S)-(4-(3-chloro-4-(2-chloro-3-(5-formylpicolinamido)phenyl)pyridin-2-yl)-2-methoxybenzyl)((5-oxopyrrolidin-2-yl)methyl)carbamate

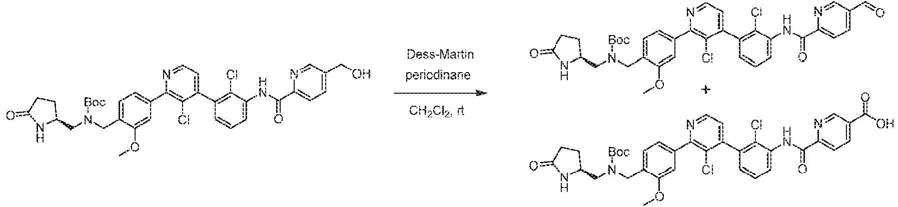

To a mixture of tert-butyl (S)-(4-(3-chloro-4-(2-chloro-3-(5-(hydroxymethyl)picolinamido)phenyl)pyridin-2-yl)-2-methoxybenzyl)((5-oxopyrrolidin-2-yl)methyl)carbamate (0.34 g, 0.48 mmol) in dichloromethane (10 mL) was added Dess-Martin periodinane (0.26 g, 0.63 mmol) in one portion under N₂ and the mixture was stirred at room temperature for 0.5 h. The mixture was purified directly by normal phase SiO₂ chromatography (30-100% 30% THF in ethyl acetate/petroleum ether) to afford a mixture of tert-butyl (S)-(4-(3-chloro-4-(2-chloro-3-(5-formylpicolinamido)phenyl)pyridin-2-yl)-2-methoxybenzyl)((5-oxopyrrolidin-2-yl)methyl)carbamate (MS: *m*/*z* found 704 [M+H]⁺) and (S)-6-((3-(2-(4-(((tert-butoxycarbonyl)((5-oxopyrrolidin-2-yl)methyl)amino)methyl)-3-methoxyphenyl)-3-chloropyridin-4-yl)-2-chlorophenyl)carbamoyl)nicotinic acid (MS: *m*/*z* found 720 [M+H]⁺) as a yellow solid (0.34 g).

### (c) tert-Butyl (S)-(4-(3-chloro-4-(2-chloro-3-(5-((methylamino)methyl)picolinamido)phenyl)pyridin-2-yl)-2-methoxybenzyl)((5-oxopyrrolidin-2-yl)methyl)carbamate

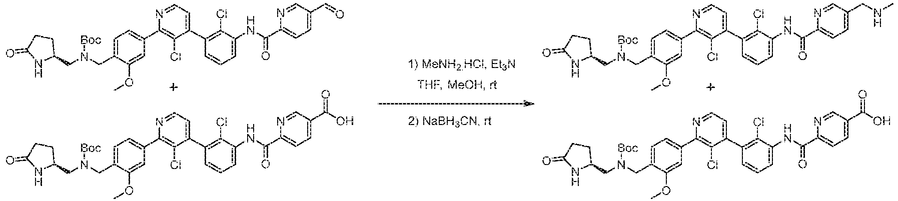

To a mixture of tert-butyl (S)-(4-(3-chloro-4-(2-chloro-3-(5-formylpicolinamido)phenyl)pyridin-2-yl)-2-methoxybenzyl)((5-oxopyrrolidin-2-yl)methyl)carbamate and (S)-6-((3-(2-(4-(((tert-butoxycarbonyl)((5-oxopyrrolidin-2-yl)methyl)amino)methyl)-3-methoxyphenyl)-3-chloropyridin-4-yl)-2-chlorophenyl)carbamoyl)nicotinic acid (0.34 g, 0.48 mmol) and methanamine hydrochloride salt (0.16 g, 2.41 mmol) in THF/MeOH mixture (5:1, 6 mL) was added triethylamine (0.33 mL, 2.41 mmol) in one portion under N₂ and the mixture stirred at room temperature for 0.5 h. Sodium cyanoborohydride (0.09 g, 1.45 mmol) was then added and the mixture stirred at room temperature for 0.5 h. The mixture was concentrated, water (5 mL) and saturated aqueous brine solution (5 mL) added to the residue and the mixture extracted with ethyl acetate/THF mixture (1:1, 10 mL). The organic phase was dried with anhydrous sodium sulfate, filtered and concentrated. The residue was purified by reverse phase HPLC, and the product neutralized with saturated aqueous sodium bicarbonate solution and extracted with dichloromethane (2 x 20 mL) to afford tert-butyl (S)-(4-(3-chloro-4-(2-chloro-3-(5-((methylamino)methyl)picolinamido)phenyl)pyridin-2-yl)-2-methoxybenzyl)((5-oxopyrrolidin-2-yl)methyl)carbamate (70 mg, 15% yield) as a yellow solid (MS: *m*/*z* found 719 [M+H]⁺) and (S)-6-((3-(2-(4-(((tert-butoxycarbonyl)((5-oxopyrrolidin-2-yl)methyl)amino)methyl)-3-methoxyphenyl)-3-chloropyridin-4-yl)-2-chlorophenyl)carbamoyl)nicotinic acid (70 mg, 18% yield) as a yellow solid. MS: *m*/*z* found 720 [M+H]⁺).

### (d) (S)-N-(2-Chloro-3-(3-chloro-2-(3-methoxy-4-((((5-oxopyrrolidin-2-yl)methyl)amino)methyl)phenyl)pyridin-4-yl)phenyl)-5-((methylamino)methyl)picolinamide

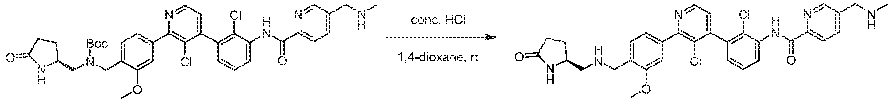

To a mixture of tert-butyl (S)-(4-(3-chloro-4-(2-chloro-3-(5-((methylamino)methyl)picolinamido)phenyl)pyridin-2-yl)-2-methoxybenzyl)((5-oxopyrrolidin-2-yl)methyl)carbamate (65 mg, 0.09 mmol) in 1,4-dioxane (1.5 mL) was added concentrated HCl solution (0.4 mL) in one portion and the mixture stirred at room temperature for 0.5 h. The mixture was combined with another batch at 5 mg scale. The mixture was neutralized with saturated aqueous sodium carbonate solution the mixture concentrated and the residue purified by reverse phase HPLC to afford (S)-N-(2-Chloro-3-(3-chloro-2-(3-methoxy-4-((((5-oxopyrrolidin-2-yl)methyl)amino)methyl)phenyl)pyridin-4-yl)phenyl)-5-((methylamino)methyl)picolinamide (14 mg, 20% yield) as a white solid. MS: *m*/*z* found 599 [M+H]⁺, retention time = 2.35 min (Method A). ¹H NMR (400 MHz, Methanol-*d*₄): δ 8.70-8.64 (m, 3H), 8.24 (d, 1H), 8.04 (dd, 1H), 7.55 (t, 1H), 7.45-7.41 (m, 2H), 7.30-7.28 (m, 2H), 7.21 (dd, 1H), 3.95 (s, 3H), 3.91-3.83 (m, 5H), 2.73-2.67 (m, 2H), 2.43 (s, 3H), 2.37-2.28 (m, 3H), 1.83-1.80 (m, 1H).

### Example 713: (S)-6-((2-Chloro-3-(3-chloro-2-(3-methoxy-4-((((5-oxopyrrolidin-2-yl)methyl)amino)methyl)phenyl)pyridin-4-yl)phenyl)carbamoyl)nicotinic acid (752)

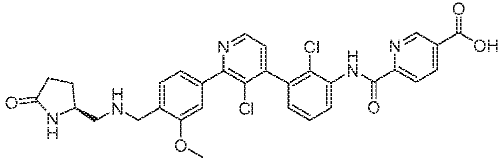

### (a) (S)-6-((2-Chloro-3-(3-chloro-2-(3-methoxy-4-((((5-oxopyrrolidin-2-yl)methyl)amino)methyl)phenyl)pyridin-4-yl)phenyl)carbamoyl)nicotinic acid

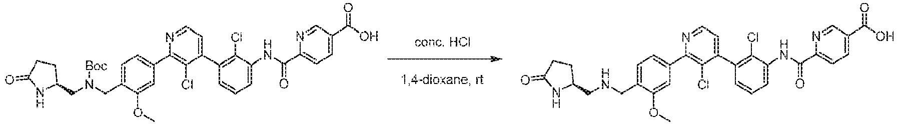

To a mixture of (S)-6-((3-(2-(4-(((tert-butoxycarbonyl)((5-oxopyrrolidin-2-yl)methyl)amino)methyl)-3-methoxyphenyl)-3-chloropyridin-4-yl)-2-chlorophenyl)carbamoyl)nicotinic acid (Example 712, step (c)) (65 mg, 0.09 mmol) in 1,4-dioxane (1 mL) was added concentrated HCl solution (0.3 mL) in one portion and the mixture stirred at room temperature for 0.5 h. The mixture was combined with another batch at 5 mg scale. The mixture was concentrated and the residue purified by reverse phase HPLC to afford (S)-6-((2-chloro-3-(3-chloro-2-(3-methoxy-4-((((5-oxopyrrolidin-2-yl)methyl)amino)methyl)phenyl)pyridin-4-yl)phenyl)carbamoyl)nicotinic acid (26.2 mg, 35% yield) as a white solid. MS: *m*/*z* found 620 [M+H]⁺, retention time = 3.51 min (Method A). ¹H NMR (400 MHz, Methanol-*d*₄): δ 9.17 (d, 1H), 8.69-8.66 (m, 2H), 8.50 (dd, 1H), 8.29 (d, 1H), 7.57-7.52 (m, 2H), 7.48 (d, 1H), 7.41 (s, 1H), 7.37 (d, 1H), 7.20 (dd, 1H), 4.33-4.25 (m, 2H), 4.07-3.96 (m, 4H), 3.16-3.14 (m, 2H), 2.47-2.36 (m, 3H), 1.94-1.86 (m, 1H).

### Example 714: N-(3-(2-(4-(((1-Acetylpiperidin-4-yl)amino)methyl)-3-methoxyphenyl)-3-chloropyridin-4-yl)-2-methylphenyl)-5-(((2-hydroxyethyl)amino)methyl)picolinamide (700)

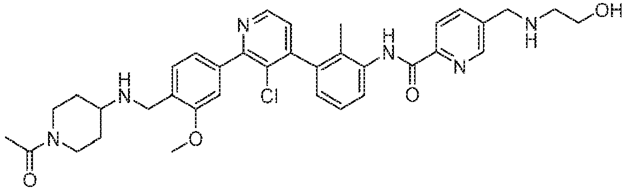

### (a) Methyl 6-((3-bromo-2-methylphenyl)carbamoyl)nicotinate

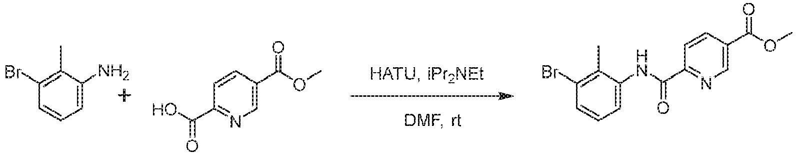

To a mixture of 5-(methoxycarbonyl)picolinic acid (3 g, 16.6 mmol) in DMF (2 mL) was added HATU (9.45 g, 24.84 mmol) and N,N-diisopropylethylamine (7.21 mL, 41.40 mmol). After 0.2 hr, 3-bromo-2-methylaniline (2.24 mL, 18.2 mmol) was added and the reaction was stirred for 12 hr at room temperature. To the reaction was added water (60 mL) and the mixture stirred for 1 hr. The mixture was then filtered, and the filter cake washed with water (100 mL) to give an off-white solid. The solid was dried under reduced pressure to give methyl 6-((3-bromo-2-methylphenyl)carbamoyl)nicotinate (4.71 g, 81% yield) as an off-white solid that was used into the next step without further purification. ¹H NMR (400 MHz, DMSO-*d*₆): δ 10.64 (s, 1 H), 9.15 (s, 1 H), 8.51 (dd, 1 H), 8.24 (d, 1 H), 7.54 (d, 1 H), 7.49 (d, 1 H), 7.16 (t, 1 H), 3.91 (s, 3 H), 2.29 (s, 3 H).

### (b) N-(3-Bromo-2-methylphenyl)-5-(hydroxymethyl)picolinamide

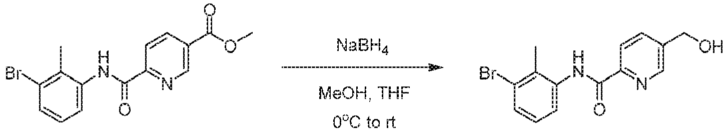

To a mixture of methyl 6-((3-bromo-2-methylphenyl)carbamoyl)nicotinate (4.1 g, 11.7 mmol) in MeOH/THF (1/1, 300 mL) was added sodium borohydride (1.78 g, 47.0 mmol) at 0 °C under N₂ and the mixture stirred at room temperature for 12 hr. Water (20 mL) was then added and the mixture concentrated. The residue was partitioned between ethyl acetate (200 mL) and water (200 mL), and the aqueous phase back extracted with ethyl acetate (200 mL). The combined organic phases were dried with anhydrous sodium sulfate, filtered and concentrated. The residue was purified by normal phase SiO₂ chromatography (0-100% ethyl acetate /petroleum ether) to afford N-(3-bromo-2-methylphenyl)-5-(hydroxymethyl)picolinamide (2.66 g, 70% yield) as a white solid. ¹H NMR (400 MHz, DMSO-*d*₆): δ 10.50 (s, 1 H), 8.69 (s, 1 H), 8.14 (d, 1 H), 8.00 (dd, 1 H), 7.69 (d, 1 H), 7.51 (d, 1 H), 7.21 (t, 1 H), 5.54 (t, 1 H), 4.68 (d, 2 H), 2.36 (s, 3 H).

### (c) N-(3-Bromo-2-methylphenyl)-5-formylpicolinamide

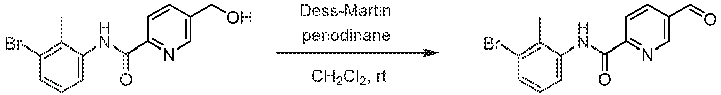

To a solution of N-(3-bromo-2-methylphenyl)-5-(hydroxymethyl)picolinamide (2.6 g, 8.10 mmol) in dichloromethane (40 mL) was added Dess-Martin periodinane (8.58 g, 20.2 mmol) and the mixture stirred at room temperature for 2 hr. The mixture was then concentrated and the residue purified by normal phase SiO₂ chromatography (20-30% EtOAc/petroleum ether) to afford N-(3-bromo-2-methylphenyl)-5-formylpicolinamide (2 g, 77% yield) as a yellow solid. ¹H NMR (400 MHz, DMSO-*d*₆): δ 10.73 (s, 1 H), 10.30 (s, 1 H), 9.28 (s, 1 H), 8.58-8.55 (m, 1 H), 8.39 (d 1 H), 7.66 (d, 1 H), 7.69 (d, 1 H), 7.27 (t, 1 H), 2.40 (s, 3 H).

### (d) tert-Butyl ((6-((3-bromo-2-methylphenyl)carbamoyl)pyridin-3-yl)methyl)(2-hydroxyethyl)carbamate

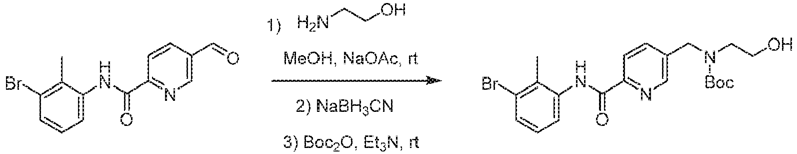

To a mixture of N-(3-bromo-2-methylphenyl)-5-formylpicolinamide (1 g, 3.13 mmol) and 2-aminoethanol (383 mg, 6.27 mmol) in methanol (80 mL) was added sodium acetate (771 mg, 9.40 mmol) and the mixture stirred for 12 hr. Sodium cyanoborohydride (591 mg, 9.40 mmol) was then added, and the mixture was stirred for an additional 2 hr at room temperature. Additional sodium cyanoborohydride (4 eq) was added and stirring continued for another 6 hr. Di-tert-butyl dicarbonate (1.71 g, 7.82 mmol) and triethylamine (1.31 mL, 9.39 mmol) were then added, and the mixture stirred for 2 hr at room temperature. To the mixture was added saturated aqueous brine solution (30 mL) and the mixture extracted with dichloromethane (40 ml x 3). The combined organic layers were concentrated under reduced pressure. The residue was purified by normal phase SiO₂ chromatography (0-100% EtOAc/petroleum ether) to afford tert-butyl ((6-((3-bromo-2-methylphenyl)carbamoyl)pyridin-3-yl)methyl)(2-hydroxyethyl)carbamate (1.2 g, 71% yield) as a yellow oil. MS: *m*/*z* found 464 and 466 [M+H]⁺.

### (e) tert-Butyl ((6-((3-(2,3-dichloropyridin-4-yl)-2-methylphenyl)carbamoyl)pyridin-3-yl)methyl)(2-hydroxyethyl)carbamate

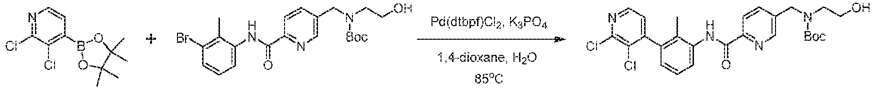

To a mixture of tert-butyl ((6-((3-bromo-2-methylphenyl)carbamoyl)pyridin-3-yl)methyl)(2-hydroxyethyl)carbamate (400 mg, 0.86 mmol, 3 batches) and 2,3-dichloro-4-(4,4,5,5-tetramethyl-1,3,2-dioxaborolan-2-yl)pyridine (519 mg, 1.90 mmol) in 1,4-dioxane/water (5/1, 24 mL) was added potassium phosphate (549 mg, 2.58 mmol) and [1,1'-bis(di-tert-butylphosphino)ferrocene]dichloropalladium(II) (56 mg, 0.09 mmol) in one portion under N₂ and the mixture was stirred at 85 °C for 6 hr. Water (20 mL) was added and the mixture extracted with ethyl acetate (100 mL). The organic layer was dried over anhydrous sodium sulfate, filtered and the filtrate concentrated. The residue was purified by normal phase SiO₂ chromatography (0-100% EtOAc/petroleum ether) to afford tert-butyl ((6-((3-(2,3-dichloropyridin-4-yl)-2-methylphenyl)carbamoyl)pyridin-3-yl)methyl)(2-hydroxyethyl)carbamate (340 mg, 16% yield) as a red oil. MS: *m*/*z* found 531 [M+H]⁺.

### (f) 2-Methoxy-4-(4,4,5,5-tetramethyl-1,3,2-dioxaborolan-2-yl)benzaldehyde

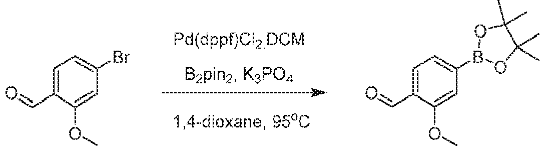

To a solution of 4-bromo-2-methoxybenzaldehyde (10 g, 46.5 mmol) and bis(pinacolato)diborane (23.6 g, 93.0 mmol) in 1,4-dioxane (100 mL) was added potassium acetate (13.7 g, 139 mmol) and [1,1'- bis(diphenylphosphino)ferrocene]dichloropalladium(II) complex with dichloromethane (1.90 g, 2.33 mmol) and the mixture was stirred at 95 °C for 12 hr. The cooled reaction mixture was then filtered, and the filtrate concentrated. The residue was purified by normal phase SiO₂ chromatography (0-100% EtOAc/petroleum ether) to afford a yellow solid. Then solid product was further purified by re-crystallization from petroleum ether / EtOAc (6/1, 50 mL) at -30 °C twice to give 2-methoxy-4-(4,4,5,5-tetramethyl-1,3,2-dioxaborolan-2-yl)benzaldehyde (12 g, 98% yield) as a white solid. ¹H NMR (400 MHz, DMSO-*d*₆): δ 10.40 (s, 1 H), 7.70 (d, 1 H), 7.37 (d, 2 H), 3.95 (s, 3 H), 1.32 (s, 12 H).

### (g) tert-Butyl ((6-((3-(3-chloro-2-(4-formyl-3-methoxyphenyl)pyridin-4-yl)-2-methylphenyl)carbamoyl)pyridin-3-yl)methyl)(2-hydroxyethyl)carbamate

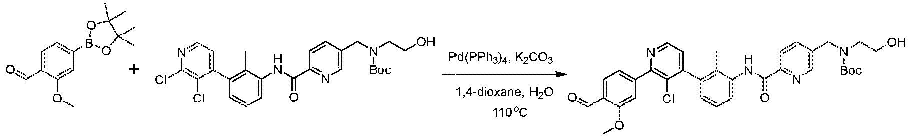

To a mixture of tert-butyl ((6-((3-(2,3-dichloropyridin-4-yl)-2-methylphenyl)carbamoyl)pyridin-3-yl)methyl)(2-hydroxyethyl)carbamate (200 mg, 0.38 mmol) and 2-methoxy-4-(4,4,5,5-tetramethyl-1,3,2-dioxaborolan-2-yl)benzaldehyde (109 mg, 0.41 mmol) in 1,4-dioxane/water (10/1, 9.9 mL) was added potassium carbonate (156 mg, 1.13 mmol) and tetrakis(triphenylphosphine)palladium(0) (43.5 mg, 0.04 mmol) in one portion under N₂ and the mixture stirred at 110 °C for 12 hr. Saturated aqueous brine solution (10 ml) was then added and the mixture extracted with EtOAc (30 ml). The organic layer was dried over anhydrous sodium sulfate, filtered and the filtrate concentrated under reduced pressure. The residue was purified by normal phase SiO₂ chromatography (0-25% EtOAc/petroleum ether) to afford tert-butyl ((6-((3-(3-chloro-2-(4-formyl-3-methoxyphenyl)pyridin-4-yl)-2-methylphenyl)carbamoyl)pyridin-3-yl)methyl)(2-hydroxyethyl)carbamate (230 mg, 35% yield) as a yellow oil. MS: *m*/*z* found 631 [M+H]⁺.

### (h) tert-Butyl ((6-((3-(2-(4-(((1-acetylpiperidin-4-yl)amino)methyl)-3-methoxyphenyl)-3-chloropyridin-4-yl)-2-methylphenyl)carbamoyl)pyridin-3-yl)methyl)(2-hydroxyethyl)carbamate

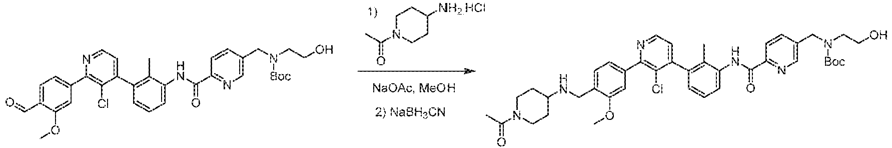

To a mixture of tert-butyl ((6-((3-(3-chloro-2-(4-formyl-3-methoxyphenyl)pyridin-4-yl)-2-methylphenyl)carbamoyl)pyridin-3-yl)methyl)(2-hydroxyethyl)carbamate (100 mg, 0.16 mmol) and 1-(4-aminopiperidin-1-yl)ethanone hydrochloride salt (42.5 mg, 0.24 mmol) in methanol (3 mL) was added sodium acetate (39 mg, 0.48 mmol) and the mixture stirred for 2 hr. Sodium cyanoborohydride (29.9 mg, 0.48 mmol) was added and the mixture was stirred for another 4 hr at room temperature. The mixture was then concentrated and the residue was purified by normal phase SiO₂ chromatography (20% MeOH/CH₂Cl₂) to afford tert-butyl ((6-((3-(2-(4-(((1-acetylpiperidin-4-yl)amino)methyl)-3-methoxyphenyl)-3-chloropyridin-4-yl)-2-methylphenyl)carbamoyl)pyridin-3-yl)methyl)(2-hydroxyethyl)carbamate (85 mg, 60% yield) as a white solid. MS: *m*/*z* found 757 [M+H]⁺.

### (i) N-(3-(2-(4-(((1-Acetylpiperidin-4-yl)amino)methyl)-3-methoxyphenyl)-3-chloropyridin-4-yl)-2-methylphenyl)-5-(((2-hydroxyethyl)amino)methyl)picolinamide

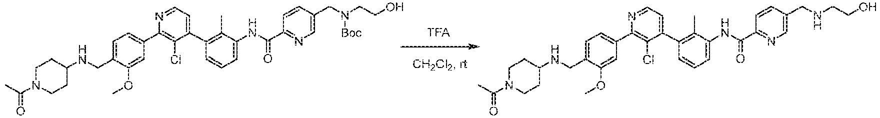

To the mixture of tert-butyl ((6-((3-(2-(4-(((1-acetylpiperidin-4-yl)amino)methyl)-3-methoxyphenyl)-3-chloropyridin-4-yl)-2-methylphenyl)carbamoyl)pyridin-3-yl)methyl)(2-hydroxyethyl)carbamate (80 mg, 0.11 mmol) in dichloromethane (2 mL) was added trifluoroacetic acid (1 mL, 13.5 mmol) and the reaction was stirred for 0.5 hr at room temperature. The mixture was concentrated under reduced pressure and the residue purified by reverse phase HPLC to afford N-(3-(2-(4-(((1-acetylpiperidin-4-yl)amino)methyl)-3-methoxyphenyl)-3-chloropyridin-4-yl)-2-methylphenyl)-5-(((2-hydroxyethyl)amino)methyl)picolinamide (36.5 mg, 48% yield) as a white solid (formic acid salt). MS: *m*/*z* found 657 [M+H]⁺, retention time = 1.49 min (Method A). ¹H NMR (400 MHz, Methanol-*d*₄): δ 8.80 (s, 1 H), 8.66 (d, 1 H), 8.47 (br s, 2 H), 8.29 (d, 1 H), 8.14 (dd, 1 H), 7.97 (d, 1 H), 7.55 (d, 1 H), 7.45-7.41 (m, 3 H), 7.37 (dd, 1 H), 7.14 (d, 1 H), 4.68 (d, 1 H), 4.32 (s, 2 H), 4.25 (s, 2 H), 4.09 (d, 1 H), 4.02 (s, 3 H), 3.81 (t, 2H), 3.44 (t, 1 H), 3.23 (t, 1 H), 3.05 (t, 2 H), 2.72 (t, 1 H), 2.29-2.22 (m, 2 H), 2.19 (s, 3 H), 2.15 (s, 3 H), 1.68-1.63 (m, 1 H), 1.57-1.53 (m, 1 H).

### Example 715: N-(3-(3-Chloro-2-(3-methoxy-4-(((tetrahydro-2H-pyran-4-yl)amino)methyl)phenyl)pyridin-4-yl)-2-methylphenyl)-5-(((2-hydroxyethyl)amino)methyl)picolinamide (703)

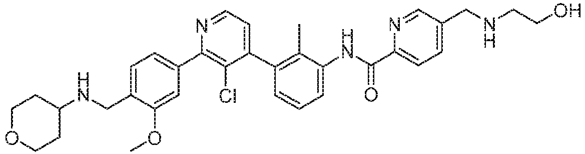

### (a) tert-Butyl ((6-((3-(3-chloro-2-(3-methoxy-4-(((tetrahydro-2H-pyran-4-yl)amino)methyl)phenyl)pyridin-4-yl)-2-methylphenyl)carbamoyl)pyridin-3-yl)methyl)(2-hydroxyethyl)carbamate

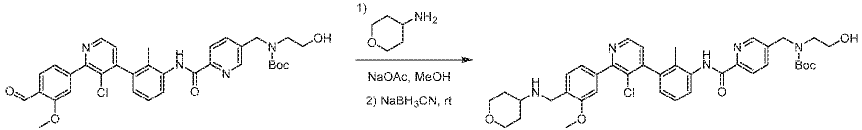

To a mixture of tert-butyl ((6-((3-(3-chloro-2-(4-formyl-3-methoxyphenyl)pyridin-4-yl)-2-methylphenyl)carbamoyl)pyridin-3-yl)methyl)(2-hydroxyethyl)carbamate (110 mg, 0.17 mmol) (Example 714, step (g)) and tetrahydro-2H-pyran-4-amine (26.4 mg, 0.26 mmol) in methanol (2 mL) was added sodium acetate (42.9 mg, 0.52 mmol) and the mixture stirred for 2 hr. Sodium cyanoborohydride (32.9 mg, 0.52 mmol) was then added and the mixture was stirred for another 10 hr at room temperature. The reaction mixture was purified by prep-TLC (20% methanol/dichloromethane) to afford tert-butyl ((6-((3-(3-chloro-2-(3-methoxy-4-(((tetrahydro-2H-pyran-4-yl)amino)methyl)phenyl)pyridin-4-yl)-2-methylphenyl)carbamoyl)pyridin-3-yl)methyl)(2-hydroxyethyl)carbamate (85 mg, 68% yield) as a white solid.

### (b) N-(3-(3-Chloro-2-(3-methoxy-4-(((tetrahydro-2H-pyran-4-yl)amino)methyl)phenyl)pyridin-4-yl)-2-methylphenyl)-5-(((2-hydroxyethyl)amino)methyl)picolinamide

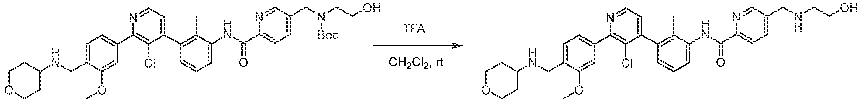

To a mixture of tert-butyl ((6-((3-(3-chloro-2-(3-methoxy-4-(((tetrahydro-2H-pyran-4-yl)amino)methyl)phenyl)pyridin-4-yl)-2-methylphenyl)carbamoyl)pyridin-3-yl)methyl)(2-hydroxyethyl)carbamate (80 mg, 0.11 mmol) in dichloromethane (2 mL) was added trifluoroacetic acid (1 mL, 13.51 mmol) and the mixture stirred for 20 min at room temperature. The mixture was concentrated under reduced pressure and the residue purified by reverse phase HPLC to afford N-(3-(3-chloro-2-(3-methoxy-4-(((tetrahydro-2H-pyran-4-yl)amino)methyl)phenyl)pyridin-4-yl)-2-methylphenyl)-5-(((2-hydroxyethyl)amino)methyl)picolinamide (27.3 mg, 39% yield) as a white solid. MS: *m*/*z* found 616 [M+H]⁺, retention time = 2.54 min (Method A). ¹H NMR (400 MHz, Methanol-*d*₄): δ 8.72 (d, 1 H), 8.64 (d, 1 H), 8.23 (d, 1 H), 8.06 (dd, 1 H), 8.00 (d, 1 H), 7.49 (d, 1 H), 7.44-7.40 (m, 2H), 7.36 (s, 1 H), 7.33 (d, 1 H), 7.13 (dd, 1 H), 4.60 (s, 1 H), 4.12 (s, 2 H), 4.03 (dd, 2 H), 3.98 (s, 4 H), 3.72 (t, 2 H), 3.48-3.42 (m, 2 H), 3.15-3.10 (m, 1 H), 2.80 (t, 2 H), 2.19 (s, 3 H), 2.03 (dd, 2 H), 1.67-1.58 (m, 2 H).

### Example 716: (S)-N-(3-(3-Chloro-2-(3-methoxy-4-((((5-oxopyrrolidin-2-yl)methyl)amino)methyl)phenyl)pyridin-4-yl)-2-methylphenyl)-5-(((2-hydroxyethyl)amino)methyl)picolinamide (735)

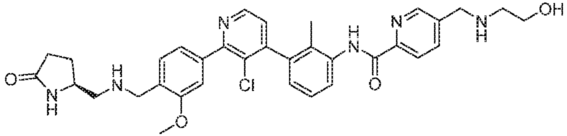

### (a) tert-Butyl (S)-((6-((3-(3-chloro-2-(3-methoxy-4-((((5-oxopyrrolidin-2-yl)methyl)amino)methyl)phenyl)pyridin-4-yl)-2-methylphenyl)carbamoyl)pyridin-3-yl)methyl)(2-hydroxyethyl)carbamate

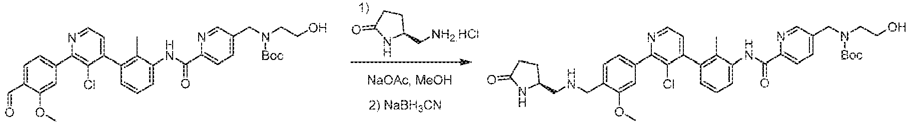

To a mixture of tert-butyl ((6-((3-(3-chloro-2-(4-formyl-3-methoxyphenyl)pyridin-4-yl)-2-methylphenyl)carbamoyl)pyridin-3-yl)methyl)(2-hydroxyethyl)carbamate (135 mg, 0.21 mmol) (Example 714, step (g)) and (S)-5-(aminomethyl)pyrrolidin-2-one hydrochloride salt (64.4 mg, 0.43 mmol) in methanol (2 mL) was added sodium acetate (52.6 mg, 0.64 mmol) and the mixture stirred for 11 hr. Sodium cyanoborohydride (40.3 mg, 0.64 mmol) was added and the mixture was stirred for another 1 hr at room temperature. The reaction mixture was concentrated under reduced pressure and the residue purified by prep-TLC (20% methanol/ dichloromethane) to afford tert-butyl (S)-((6-((3-(3-chloro-2-(3-methoxy-4-((((5-oxopyrrolidin-2-yl)methyl)amino)methyl)phenyl)pyridin-4-yl)-2-methylphenyl)carbamoyl)pyridin-3-yl)methyl)(2-hydroxyethyl)carbamate (130 mg, 75% yield) as a white solid. MS: *m*/*z* found 729 [M+H]⁺.

### (b) (S)-N-(3-(3-Chloro-2-(3-methoxy-4-((((5-oxopyrrolidin-2-yl)methyl)amino)methyl)phenyl)pyridin-4-yl)-2-methylphenyl)-5-(((2-hydroxyethyl)amino)methyl)picolinamide

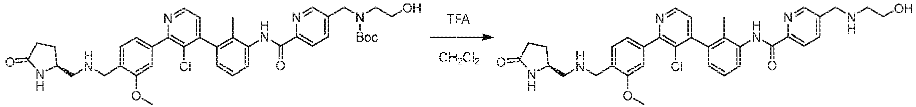

To a solution of tert-butyl (S)-((6-((3-(3-chloro-2-(3-methoxy-4-((((5-oxopyrrolidin-2-yl)methyl)amino)methyl)phenyl)pyridin-4-yl)-2-methylphenyl)carbamoyl)pyridin-3-yl)methyl)(2-hydroxyethyl)carbamate (120 mg, 0.16 mmol) in dichloromethane (2 mL) was added trifluoroacetic acid (2.40 mL, 32 mmol) and the mixture stirred for 0.5 hr at room temperature. The reaction was concentrated under reduced pressure and the residue purified by reverse phase HPLC to afford (S)-N-(3-(3-chloro-2-(3-methoxy-4-((((5-oxopyrrolidin-2-yl)methyl)amino)methyl)phenyl)pyridin-4-yl)-2-methylphenyl)-5-(((2-hydroxyethyl)amino)methyl)picolinamide (30.1 mg, 28% yield) as a white solid. MS: *m*/*z* found 629 [M+H]⁺, retention time = 2.37 min (Method A). ¹H NMR (400 MHz, Methanol-*d*₄): δ 8.72 (d, 1 H), 8.62 (d, 1 H), 8.23 (d, 1 H), 8.05 (dd, 1 H), 8.01 (d, 1 H), 7.44-7.40 (m, 3 H), 7.30-7.21 (m, 2 H), 7.13 (d, 1 H), 3.96 (d, 5 H), 3.90-3.84 (m, 3 H), 3.71 (t, 2 H), 2.78 (t, 2H), 2.71-2.69 (m, 2 H), 2.37-2.28 (m, 3 H), 2.20 (s, 3 H), 1.85-1.77 (m, 1 H).

### Example 717: N-(3-(3-Chloro-2-(3-methoxy-4-((7-oxo-2,6-diazaspiro[3.4]octan-2-yl)methyl)phenyl)pyridin-4-yl)-2-methylphenyl)-5-(((2-hydroxyethyl)amino)methyl)picolinamide (744)

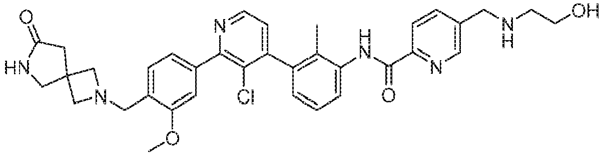

### (a) tert-Butyl ((6-((3-(3-chloro-2-(3-methoxy-4-((7-oxo-2,6-diazaspiro[3.4]octan-2-yl)methyl)phenyl)pyridin-4-yl)-2-methylphenyl)carbamoyl)pyridin-3-yl)methyl)(2-hydroxyethyl)carbamate

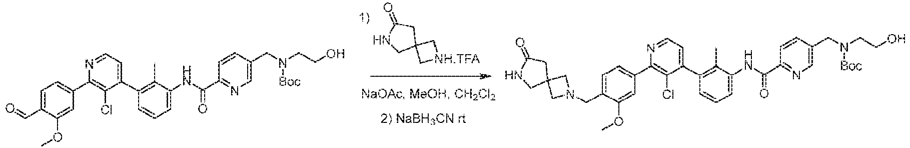

To a mixture of tert-butyl ((6-((3-(3-chloro-2-(4-formyl-3-methoxyphenyl)pyridin-4-yl)-2-methylphenyl)carbamoyl)pyridin-3-yl)methyl)(2-hydroxyethyl)carbamate (100 mg, 0.16 mmol) (Example 714, step (g)) and 2,6-diazaspiro[3.4]octan-7-one trifluoroacetic acid salt (114 mg, 0.48 mmol) in methanol/dichloromethane (1/1, 2.4 mL) was added sodium acetate (114 mg, 1.39 mmol) and the mixture stirred for 0.5 hr. Sodium cyanoborohydride (29.9 mg, 0.48 mmol) was added and the mixture was stirred at room temperature for another 11.5 hr. The reaction mixture was concentrated under reduced pressure and the residue purified by normal phase SiO₂ chromatography (0-10% methanol/ethyl acetate) to afford tert-butyl ((6-((3-(3-chloro-2-(3-methoxy-4-((7-oxo-2,6-diazaspiro[3.4]octan-2-yl)methyl)phenyl)pyridin-4-yl)-2-methylphenyl)carbamoyl)pyridin-3-yl)methyl)(2-hydroxyethyl)carbamate (140 mg, 59% yield) as a white solid.

### (b) N-(3-(3-Chloro-2-(3-methoxy-4-((7-oxo-2,6-diazaspiro[3.4]octan-2-yl)methyl)phenyl)pyridin-4-yl)-2-methylphenyl)-5-(((2-hydroxyethyl)amino)methyl)picolinamide

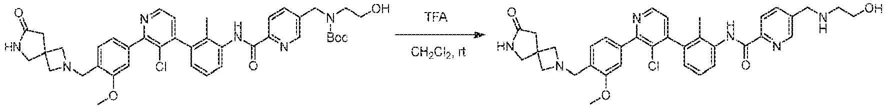

To a mixture of tert-butyl ((6-((3-(3-chloro-2-(3-methoxy-4-((7-oxo-2,6-diazaspiro[3.4]octan-2-yl)methyl)phenyl)pyridin-4-yl)-2-methylphenyl)carbamoyl)pyridin-3-yl)methyl)(2-hydroxyethyl)carbamate (130 mg, 0.18 mmol) in dichloromethane (3 mL) was added trifluoroacetic acid (1 mL, 13.5 mmol) and the mixture was stirred for 2 hr at room temperature. The reaction mixture was then concentrated under reduced pressure and the residue purified by reverse phase HPLC to afford N-(3-(3-chloro-2-(3-methoxy-4-((7-oxo-2,6-diazaspiro[3.4]octan-2-yl)methyl)phenyl)pyridin-4-yl)-2-methylphenyl)-5-(((2-hydroxyethyl)amino)methyl)picolinamide (21.6 mg, 18.7% yield) as a yellow solid. MS: *m*/*z* found 641 [M+H]⁺, retention time 2.32 min (Method A). ¹H NMR (400 MHz, Methanol-*d*₄): δ 8.80 (s, 1 H), 8.66 (d, 1 H), 8.29 (d, 1 H), 8.14 (dd, 1 H), 7.97 (d, 1 H), 7.55 (d, 1 H), 7.45-7.41 (m, 2 H), 7.38-7.34 (m, 2 H), 7.14 (d, 1 H), 4.25 (s, 4 H), 3.98 (s, 7 H), 3.81 (m, 2 H), 3.66 (s, 2 H), 3.06 (m, 2 H), 2.69 (s, 2 H), 2.19 (s, 3 H).

### Example 718: (S)-N-(3-(3-chloro-2-(3-methoxy-4-((((5-oxopyrrolidin-2-yl)methyl)amino) methyl)phenyl)pyridin-4-yl)-2-methylphenyl)-5-((methylamino)methyl)picolinamide (756)

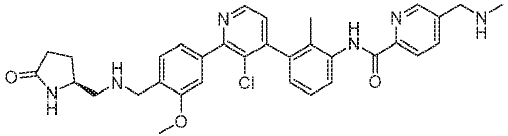

### (a) tert-Butyl ((6-((3-bromo-2-methylphenyl)carbamoyl)pyridin-3-yl)methyl)(methyl)carbamate

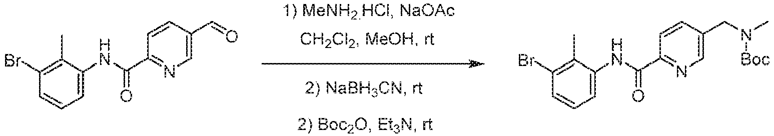

To a mixture of N-(3-bromo-2-methylphenyl)-5-formylpicolinamide (Example 714, step (c)) (1 g, 3.13 mmol) and methanamine hydrochloride salt (317 mg, 4.70 mmol) in MeOH/dichloromethane (1/1, 30 mL) was added sodium acetate (771 mg, 9.40 mmol) and stirred for 12 hr. Sodium cyanoborohydride (591 mg, 9.40 mmol) was then added and the mixture was stirred for another 1 hr at room temperature. Triethylamine (1.31 mL, 9.43 mmol) and di-tert-butyl decarbonate (1.37 g, 6.28 mmol) were then added and the reaction stirred for 12 hr at room temperature. Saturated aqueous brine solution (15 mL) was added and the mixture extracted with ethyl acetate (40 ml). The organic layer was dried over anhydrous sodium sulfate, filtered and the filtrate was concentrated under reduced pressure. The residue was purified by normal phase SiO₂ chromatography (0-50% ethyl acetate/petroleum ether) to afford tert-butyl ((6-((3-bromo-2-methylphenyl)carbamoyl)pyridin-3-yl)methyl)(methyl)carbamate (1 g, 73% yield) as a white solid. ₁H NMR (400 MHz, Chloroform-*d*): δ 10.07 (s, 1 H), 8.50 (s, 1 H), 8.25 (d, 1 H), 8.15 (d, 1 H), 7.70 (br s, 1 H), 7.38 (d, 1 H), 7.11 (t, 1 H), 4.51 (s, 2 H), 2.90-2.85 (m, 3 H), 2.49 (s, 3 H), 1.48 (s, 9 H).

### (b) tert-Butyl ((6-((3-(2,3-dichloropyridin-4-yl)-2-methylphenyl)carbamoyl)pyridin-3-yl)methyl)(methyl)carbamate

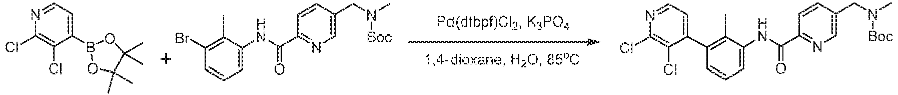

To a mixture of tert-butyl ((6-((3-bromo-2-methylphenyl)carbamoyl)pyridin-3-yl)methyl)(methyl)carbamate (200 mg, 0.46 mmol) and 2,3-dichloro-4-(4,4,5,5-tetramethyl-1,3,2-dioxaborolan-2-yl)pyridine (290 mg, 1.06 mmol) in 1,4-dioxane/water (5/1, 12 mL) was added potassium phosphate (293 mg, 1.38 mmol) and [1,1'-bis(di-tert-butylphosphino)ferrocene]dichloropalladium(II) (30.0 mg, 0.05 mmol) in one portion under N₂ and the mixture was stirred at 85 °C for 6 hr. Additional 2,3-dichloro-4-(4,4,5,5-tetramethyl-1,3,2-dioxaborolan-2-yl)pyridine (150 mg) was added and the mixture stirred for another 12 hr at 85 °C. The mixture combined with another batch at 20 mg scale, water (50 mL) was added and the mixture extracted with ethyl acetate (200 mL). The organic layer was dried over anhydrous sodium sulfate, filtered and the filtrate was concentrated under reduced pressure. The residue was purified by normal phase SiO₂ chromatography (0-100% ethyl acetate/petroleum ether) to afford tert-butyl ((6-((3-(2,3-dichloropyridin-4-yl)-2-methylphenyl)carbamoyl)pyridin-3-yl)methyl)(methyl)carbamate (250 mg ,79% yield) as a yellow oil. MS: *m*/*z* found 501 [M+H]⁺.

### (c) tert-Butyl (S)-((6-((3-(2-(4-(((tert-butoxycarbonyl)((5-oxopyrrolidin-2-yl)methyl)amino)methyl)-3-methoxyphenyl)-3-chloropyridin-4-yl)-2-methylphenyl)carbamoyl)pyridin-3-yl)methyl)(methyl)carbamate

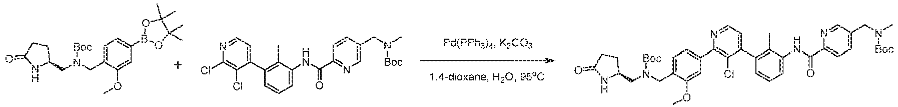

To a mixture of tert-butyl ((6-((3-(2,3-dichloropyridin-4-yl)-2-methylphenyl)carbamoyl)pyridin-3-yl)methyl)(methyl)carbamate (100 mg, 0.20 mmol) and tert-butyl (S)-(2-methoxy-4-(4,4,5,5-tetramethyl-1,3,2-dioxaborolan-2-yl)benzyl)((5-oxopyrrolidin-2-yl)methyl)carbamate (Example 542, step (p)) (119 mg, 0.26 mmol) in 1,4-dioxane/water (5/1, 6 mL) was added potassium carbonate (82.7 mg, 0.60 mmol) and tetrakis(triphenylphosphine)palladium(0) (23.0 mg, 0.02 mmol) in one portion under N₂ and the mixture stirred at 95 °C for 3 hr. Water(30 mL) was added and the mixture extracted with ethyl acetate (200 mL). The organic layer was dried over anhydrous sodium sulfate, filtered and the filtrate was concentrated under reduced pressure. The residue was purified by normal phase SiO₂ chromatography (0-100% ethyl acetate/petroleum ether) to afford tert-butyl (S)-((6-((3-(2-(4-(((tert-butoxycarbonyl)((5-oxopyrrolidin-2-yl)methyl)amino)methyl)-3-methoxyphenyl)-3-chloropyridin-4-yl)-2-methylphenyl)carbamoyl)pyridin-3-yl)methyl)(methyl)carbamate (150 mg, 34% yield) as a yellow solid. MS: *m*/*z* found 799 [M+H]⁺.

### (d) (S)-N-(3-(3-Chloro-2-(3-methoxy-4-((((5-oxopyrrolidin-2-yl)methyl)amino)methyl) phenyl)pyridin-4-yl)-2-methylphenyl)-5-((methylamino)methyl)picolinamide

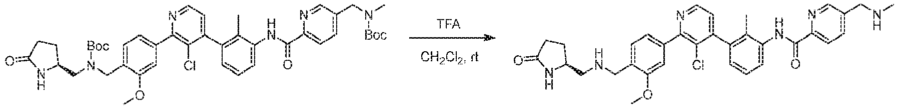

To a mixture of tert-butyl (S)-((6-((3-(2-(4-(((tert-butoxycarbonyl)((5-oxopyrrolidin-2-yl)methyl)amino)methyl)-3-methoxyphenyl)-3-chloropyridin-4-yl)-2-methylphenyl)carbamoyl)pyridin-3-yl)methyl)(methyl)carbamate (145 mg, 0.18 mmol) in dichloromethane (2 mL) was added trifluoroacetic acid (1 mL,13.5 mmol) and the reaction stirred for 0.5 hr at room temperature. The mixture was concentrated under reduced pressure and the residue was purified by reverse phase HPLC twice to afford (S)-N-(3-(3-chloro-2-(3-methoxy-4-((((5-oxopyrrolidin-2-yl)methyl)amino)methyl)phenyl)pyridin-4-yl)-2-methylphenyl)-5-((methylamino)methyl)picolinamide (20.6 mg, 18% yield) as a white solid. MS: m/z found 599 [M+H]⁺, retention time 2.35 min (Method A). ¹H NMR (400 MHz, Methanol-*d*₄): δ 8.72 (s, 1H), 8.63 (d, 1H), 8.25 (d, 1H), 8.05 (d, 1H), 8.00 (d, 1H), 7.44-7.40 (m, 3H), 7.31-7.28 (m, 2H), 7.14 (d, 1H), 3.97-3.92 (m, 7H), 3.88-3.84 (m, 1H), 2.77-2.72 (m, 2H), 2.52 (s, 3H), 2.37-2.27 (m, 3H), 2.20 (s, 3H), 1.85-1.79 (m, 1H).

### Example 719: N-(3-(3-chloro-2-(3-fluoro-4-(((2-hydroxyethyl)amino)methyl)-5-methoxyphenyl)pyridin-4-yl)-2-methylphenyl)-5-(((2-hydroxyethyl)amino)methyl)picolinamide (762)

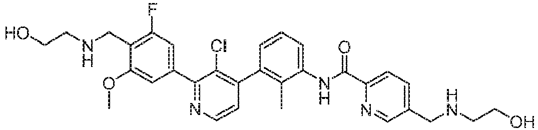

### (a) 2-Fluoro-6-methoxy-4-(4,4,5,5-tetramethyl-1,3,2-dioxaborolan-2-yl)benzaldehyde

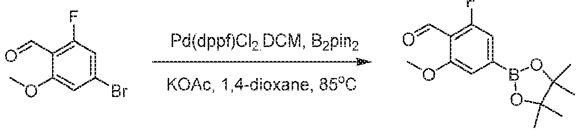

To a solution of 4-bromo-2-fluoro-6-methoxybenzaldehyde (980 mg, 4.21 mmol) and bis(pinacolato)diboron (2.14 g, 8.41 mmol) in 1,4-dioxane (20 mL) was added potassium acetate (825 mg, 8.41 mmol) and [1,1'-bis(diphenylphosphino)ferrocene]dichloropalladium(II) complex with dichloromethane (343 mg, 0.42 mmol) and the mixture was stirred at 85 °C for 6 hr. The mixture was filtered and the filtrate was concentrated. The residue was purified by normal phase SiO₂ chromatography (0-10% ethyl acetate/petroleum ether) to afford 2-fluoro-6-methoxy-4-(4,4,5,5-tetramethyl-1,3,2-dioxaborolan-2-yl)benzaldehyde (1.3 g, 99% yield) as a yellow solid. MS: m/z found 199 [M-82]⁺. ¹H NMR (400 MHz, DMSO-*d*₆): δ 10.32 (s, 1H), 7.17 (s, 1H), 7.02 (d, 1H), 3.95 (s, 3H), 1.32 (s, 12H).

### (b) tert-Butyl ((6-((3-(3-chloro-2-(3-fluoro-4-formyl-5-methoxyphenyl)pyridin-4-yl)-2-methylphenyl)carbamoyl)pyridin-3-yl)methyl)(2-hydroxyethyl)carbamate

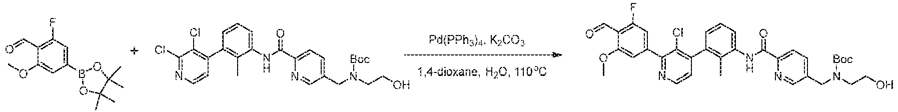

To a mixture of tert-butyl ((6-((3-(2,3-dichloropyridin-4-yl)-2-methylphenyl)carbamoyl)pyridin-3-yl)methyl)(2-hydroxyethyl)carbamate (50 mg, 0.09 mmol) (Example 714, step (e)) and 2-fluoro-6-methoxy-4-(4,4,5,5-tetramethyl-1,3,2-dioxaborolan-2-yl)benzaldehyde (36.9 mg, 0.13 mmol) in 1,4-dioxane/water (10/1, 3.3 mL) was added potassium carbonate (39.0 mg, 0.28 mmol) and tetrakis(triphenylphosphine)palladium(0) (10.9 mg, 9.41 umol) in one portion under N₂ and the mixture stirred at 110 °C for 12 hr. Saturated aqueous brine solution (20 mL) was added and the mixture extracted with ethyl acetate (80 mL). The organic layer was dried over anhydrous sodium sulfate, filtered and the filtrate was concentrated. The residue was purified by normal phase SiO₂ chromatography (0-80% ethyl acetate/petroleum ether) to afford tert-butyl ((6-((3-(3-chloro-2-(3-fluoro-4-formyl-5-methoxyphenyl)pyridin-4-yl)-2-methylphenyl)carbamoyl)pyridin-3-yl)methyl)(2-hydroxyethyl)carbamate (83 mg, 39% yield) as a yellow oil. MS: m/z found 649 [M+H]⁺.

### (c) tert-Butyl ((6-((3-(3-chloro-2-(3-fluoro-4-(((2-hydroxyethyl)amino)methyl)-5-methoxyphenyl)pyridin-4-yl)-2-methylphenyl)carbamoyl)pyridin-3-yl)methyl)(2-hydroxyethyl)carbamate

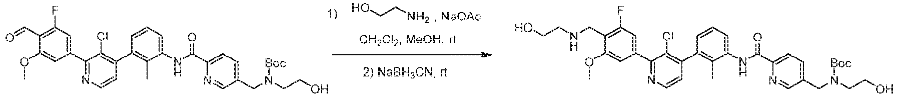

To a mixture of tert-butyl ((6-((3-(3-chloro-2-(3-fluoro-4-formyl-5-methoxyphenyl)pyridin-4-yl)-2-methylphenyl)carbamoyl)pyridin-3-yl)methyl)(2-hydroxyethyl)carbamate (78 mg, 0.12 mmol) and 2-aminoethanol (22.02 mg, 0.36 mmol) in MeOH/dichloromethane (1/1, 2 mL) was added sodium acetate (29.6 mg, 0.36 umol) and the mixture stirred for 12 hr. Sodium cyanoborohydride (22.7 mg, 0.36 umol) was added and the mixture was stirred for another 1 hr at room temperature. The reaction mixture was concentrated under reduced pressure and the residue purified by normal phase SiO₂ chromatography (0~20% methanol/dichloromethane) to afford tert-butyl ((6-((3-(3-chloro-2-(3-fluoro-4-(((2-hydroxyethyl)amino)methyl)-5-methoxyphenyl)pyridin-4-yl)-2-methylphenyl)carbamoyl)pyridin-3-yl)methyl)(2-hydroxyethyl)carbamate (73 mg, 77.0% yield) as an off-white solid. MS: m/z found 694 [M+H]⁺.

### (d) N-(3-(3-Chloro-2-(3-fluoro-4-(((2-hydroxyethyl)amino)methyl)-5-methoxyphenyl)pyridin-4-yl)-2-methylphenyl)-5-(((2-hydroxyethyl)amino)methyl)picolinamide

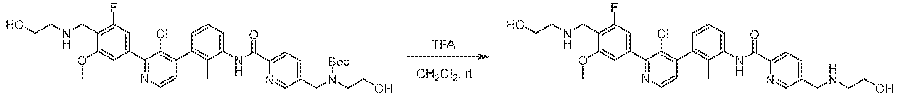

To a mixture of tert-butyl ((6-((3-(3-chloro-2-(3-fluoro-4-(((2-hydroxyethyl)amino)methyl)-5-methoxyphenyl)pyridin-4-yl)-2-methylphenyl)carbamoyl)pyridin-3-yl)methyl)(2-hydroxyethyl)carbamate (68 mg, 98 umol) in dichloromethane (1 mL) was added trifluoroacetic acid (0.5 mL, 6.75 mmol) and the mixture stirred for 0.5 hr at room temperature. The mixture was concentrated under reduced pressure and the residue purified by reverse phase HPLC to afford N-(3-(3-chloro-2-(3-fluoro-4-(((2-hydroxyethyl)amino)methyl)-5-methoxyphenyl)pyridin-4-yl)-2-methylphenyl)-5-(((2-hydroxyethyl)amino)methyl)picolinamide (21.7 mg, 37% yield) as a white solid. MS: m/z found 594 [M+H]⁺, retention time 2.43 min (Method A). ¹H NMR (400 MHz, Methanol-*d*₄): δ 8.68 (d, 1H), 8.60 (d, 1H), 8.19 (d, 1H), 8.01 (dd, 1H), 7.97 (d, 1H), 7.40-7.36 (m, 2H), 7.15 (s, 1H), 7.10 (s, 1H), 7.08 (d, 1H), 3.93 (d, 7H), 3.66 (dd, 4H), 2.74 (t, 4H), 2.15 (s, 3H).

### Example 720: N-(3-(3'-Chloro-6-methoxy-5-(((tetrahydro-2H-pyran-4-yl)amino)methyl)-[2,4'-bipyridin]-2'-yl)-2-methylphenyl)-5-(((2-hydroxyethyl)amino)methyl)picolinamide (766)

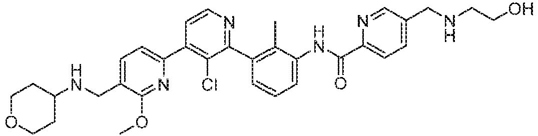

### (a) tert-Butyl (2-hydroxyethyl)((6-((2-methyl-3-(4,4,5,5-tetramethyl-1,3,2-dioxaborolan-2-yl)phenyl)carbamoyl)pyridin-3-yl)methyl)carbamate

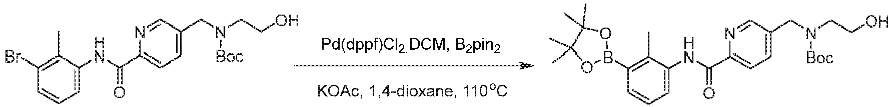

To a mixture of tert-butyl ((6-((3-bromo-2-methylphenyl)carbamoyl)pyridin-3-yl)methyl)(2-hydroxyethyl)carbamate (Example 714, step (d)) (1 g, 2.15 mmol) and bis(pinacolato)diboron (1.64 g, 6.46 mmol) in 1,4-dioxane (8 mL) was added potassium acetate (634 mg, 6.46 mmol) and [1,1'-bis(diphenylphosphino)ferrocene]dichloropalladium(II) complex with dichloromethane (176 mg, 0.22 mmol) and the mixture stirred at 110 °C for 3 hr under N₂. The mixture was concentrated and the residue purified by normal phase SiO₂ chromatography (10-50 % ethyl acetate / petroleum ether) to afford tert-butyl (2-hydroxyethyl)((6-((2-methyl-3-(4,4,5,5-tetramethyl-1,3,2-dioxaborolan-2-yl)phenyl)carbamoyl)pyridin-3-yl)methyl)carbamate (800 mg, 79% yield) as a white solid. MS: m/z found 512 [M+H]⁺.

### (b) tert-Butyl((6-((3-(3'-chloro-5-formyl-6-methoxy-[2,4'-bipyridin]-2'-yl)-2-methylphenyl)carbamoyl)pyridin-3-yl)methyl)(2-hydroxyethyl)carbamate

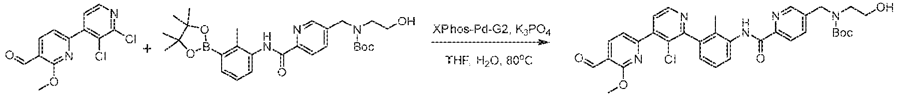

To a mixture of tert-butyl(2-hydroxyethyl)((6-((2-methyl-3-(4,4,5,5-tetramethyl-1,3,2-dioxaborolan-2-yl)phenyl)carbamoyl)pyridin-3-yl)methyl)carbamate (271 mg, 0.53 mmol) and 2',3'-dichloro-6-methoxy-[2,4'-bipyridine]-5-carbaldehyde (Example 640, step (a)) (100 mg, 0.35 mmol) in THF / water (10:1, 5.5 mL) was added chloro(2-dicyclohexylphosphino-2',4',6'-triisopropyl-1,1'-biphenyl)[2-(2'-amino-1,1'-biphenyl)]palladium(II) (27.8 mg, 0.04 umol) and potassium phosphate (225 mg, 1.06 mmol) and the mixture stirred at 80 °C for 1 h under N₂. Water (5 mL) was added and the mixture extracted with ethyl acetate (2 x10 mL). The combined organic phases were dried over anhydrous sulfate, filtered and concentrated under reduced pressure. The residue was purified by by prep-TLC (SiO₂, ethyl acetate : petroleum ether = 1:0) to afford tert-butyl((6-((3-(3'-chloro-5-formyl-6-methoxy-[2,4'-bipyridin]-2'-yl)-2-methylphenyl)carbamoyl)pyridin-3-yl)methyl)(2-hydroxyethyl)carbamate (70 mg, 31% yield) as a yellow oil. MS: m/z found 632 [M+H]⁺.

### (c) tert-Butyl ((6-((3-(3'-chloro-6-methoxy-5-(((tetrahydro-2H-pyran-4-yl)amino)methyl)-[2,4'-bipyridin]-2'-yl)-2-methylphenyl)carbamoyl)pyridin-3-yl)methyl)(2-hydroxyethyl)carbamate

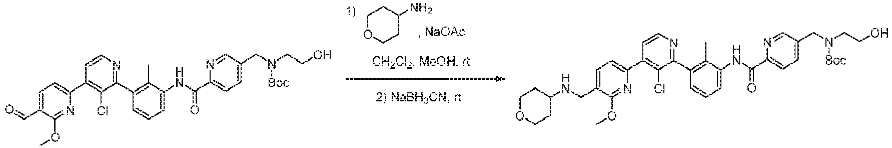

To a mixture of tert-butyl((6-((3-(3'-chloro-5-formyl-6-methoxy-[2,4'-bipyridin]-2'-yl)-2-methylphenyl)carbamoyl)pyridin-3-yl)methyl)(2-hydroxyethyl)carbamate (60 mg, 94.9 umol) and tetrahydro-2H-pyran-4-amine (19.2 mg, 190 umol) in dichloromethane/ MeOH (1:1, 6 mL) was added sodium acetate (23.4 mg, 285 umol) and the mixture stirred at room temperature for 1.5 h under N₂. Sodium cyanoborohydride (17.9 mg, 285 umol) was then added and the mixture was stirred at room temperature for 0.5 h under N₂. The mixture was concentrated and the residue purified by by prep-TLC (SiO₂, ethyl acetate : MeOH = 1:2) to afford tert-butyl ((6-((3-(3'-chloro-6-methoxy-5-(((tetrahydro-2H-pyran-4-yl)amino)methyl)-[2,4'-bipyridin]-2'-yl)-2-methylphenyl)carbamoyl)pyridin-3-yl)methyl)(2-hydroxyethyl)carbamate (45 mg, 66% yield) as a white solid. MS: m/z found 717 [M+H]⁺.

### (d) N-(3-(3'-Chloro-6-methoxy-5-(((tetrahydro-2H-pyran-4-yl)amino)methyl)-[2,4'-bipyridin]-2'-yl)-2-methylphenyl)-5-(((2-hydroxyethyl)amino)methyl)picolinamide

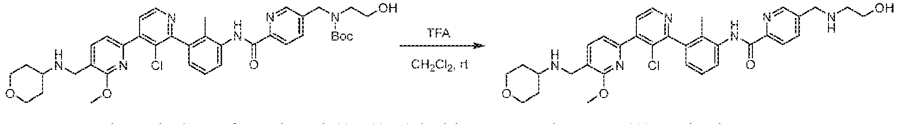

To the solution of tert-butyl ((6-((3-(3'-chloro-6-methoxy-5-(((tetrahydro-2H-pyran-4-yl)amino)methyl)-[2,4'-bipyridin]-2'-yl)-2-methylphenyl)carbamoyl)pyridin-3-yl)methyl)(2-hydroxyethyl)carbamate (40 mg, 0.06 mmol) in dichloromethane (3 mL) was added trifluoroacetic acid (1 mL, 13.5 mmol) and the mixture stirred at room temperature for 0.5 h under N₂. The mixture was concentrated and the residue purified by reverse phase HPLC to afford N-(3-(3'-chloro-6-methoxy-5-(((tetrahydro-2H-pyran-4-yl)amino)methyl)-[2,4'-bipyridin]-2'-yl)-2-methylphenyl)-5-(((2-hydroxyethyl)amino)methyl)picolinamide (12.3 mg, 34% yield) as a white solid. MS: m/z found 617 [M+H]⁺, retention time 2.32 min (Method A). ¹H NMR (400 MHz, Methanol-*d*₄): δ 8.72 (d, 1H), 8.64 (d, 1H), 8.23 (d, 1H), 8.08-8.04 (m, 2H), 7.84 (d, 1H), 7.76 (d, 1H), 7.45-7.41 (m, 2H), 7.21 (d, 1H), 4.06 (s, 3H), 4.00-3.97 (m, 4H), 3.91 (s, 2H), 3.71 (t, 2H), 3.47-3.44 (m, 2H), 2.83-2.77 (m, 3H), 2.19 (s, 3H), 1.96-1.93 (m, 2H), 1.54-1.49 (m, 2H).

### Example 721: N-(3-(5-(((1-Acetylpiperidin-4-yl)amino)methyl)-3'-chloro-6-methoxy-[2,4'-bipyridin]-2'-yl)-2-methylphenyl)-5-(((2-hydroxyethyl)amino)methyl)picolinamide (769)

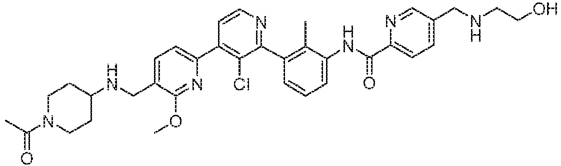

N-(3-(5-(((1-Acetylpiperidin-4-yl)amino)methyl)-3'-chloro-6-methoxy-[2,4'-bipyridin]-2'-yl)-2-methylphenyl)-5-(((2-hydroxyethyl)amino)methyl)picolinamide was prepared in a similar fashion to Example 720, replacing tetrahydro-2H-pyran-4-amine with 1-(4-aminopiperidin-1-yl)ethan-1-one hydrochloride salt in step (c). MS: m/z found 658 [M+H]⁺, retention time = 2.32 min (Method A). ¹H NMR (400 MHz, Methanol-*d*₄): δ 8.59 (d, 1H), 8.52 (d, 1H), 8.11 (d, 1H), 7.96-7.92 (m, 2H), 7.72 (d, 1H), 7.64 (d, 1H), 7.33-7.29 (m, 2H), 7.09 (d, 1H), 4.38-4.35 (m, 1H), 3.94 (s, 3H), 3.85 (s, 2H), 3.81-3.80 (m, 1H), 3.78 (s, 2H), 3.59 (t, 2H), 3.11-3.05 (m, 1H), 2.70-2.62 (m, 4H), 2.11 (s, 3H), 2.07 (s, 3H), 1.95-1.88 (m, 2H), 1.32-1.17 (m, 2H).

### Example 722: (S)-N-(3-(3'-chloro-6-methoxy-5-((((5-oxopyrrolidin-2-yl)methyl)amino)methyl)-[2,4'-bipyridin]-2'-yl)-2-methylphenyl)-5-(((2-hydroxyethyl)amino)methyl)picolinamide (771)

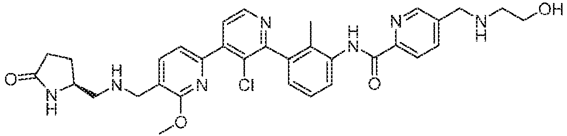

### (a) tert-Butyl (S)-((6-((3-(5-(((tert-butoxycarbonyl)((5-oxopyrrolidin-2-yl)methyl)amino)methyl)-3'-chloro-6-methoxy-[2,4'-bipyridin]-2'-yl)-2-methylphenyl)carbamoyl)pyridin-3-yl)methyl)(2-hydroxyethyl)carbamate

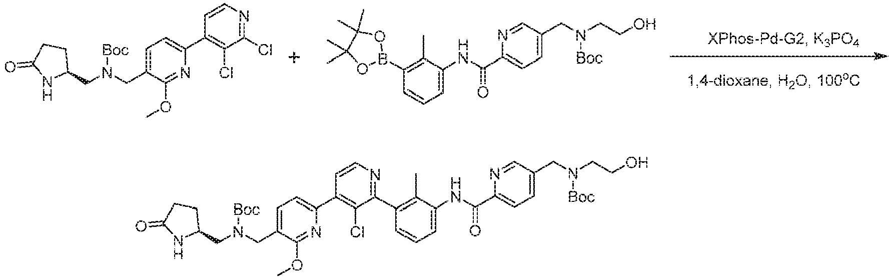

To a mixture of tert-butyl (S)-((2',3'-dichloro-6-methoxy-[2,4'-bipyridin]-5-yl)methyl)((5-oxopyrrolidin-2-yl)methyl)carbamate (0.2 g, 0.42 mmol) (Example 655, step (a)) and tert-butyl (2-hydroxyethyl)((6-((2-methyl-3-(4,4,5,5-tetramethyl-1,3,2-dioxaborolan-2-yl)phenyl)carbamoyl)pyridin-3-yl)methyl)carbamate (Example 720, step (a)) (0.32 g, 0.62 mmol) in 1,4-dioxane/water mixture (5:1, 6 mL) was added potassium phosphate (0.26 g, 1.25 mmol) and chloro(2-dicyclohexylphosphino-2',4',6'-triisopropyl-1,1'-biphenyl)[2-(2'-amino-1,1'-biphenyl)]palladium(II) (0.03 g, 0.04 mmol) in one portion under N₂ and the mixture stirred at 100 °C for 4 hr. The mixture was combined with another batch at 85 mg scale. Water (15 mL) was added and the mixture was extracted with ethyl acetate (2 x 15 mL). The combined organic phases were washed with saturated aqueous brine solution (2 x 15 mL), dried with anhydrous sodium sulfate, filtered and concentrated. The residue was purified by reverse phase HPLC to afford tert-butyl (S)-((6-((3-(5-(((tert-butoxycarbonyl)((5-oxopyrrolidin-2-yl)methyl)amino)methyl)-3'-chloro-6-methoxy-[2,4'-bipyridin]-2'-yl)-2-methylphenyl)carbamoyl)pyridin-3-yl)methyl)(2-hydroxyethyl)carbamate (0.12 g, 24% yield) as a colorless solid. MS: m/z found 830 [M+H]⁺.

### (b) (S)-N-(3-(3'-chloro-6-methoxy-5-((((5-oxopyrrolidin-2-yl)methyl)amino)methyl)-[2,4'-bipyridin]-2'-yl)-2-methylphenyl)-5-(((2-hydroxyethyl)amino)methyl)picolinamide

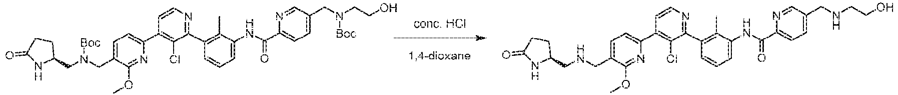

To a mixture of tert-butyl (S)-((6-((3-(5-(((tert-butoxycarbonyl)((5-oxopyrrolidin-2-yl)methyl)amino)methyl)-3'-chloro-6-methoxy-[2,4'-bipyridin]-2'-yl)-2-methylphenyl)carbamoyl)pyridin-3-yl)methyl)(2-hydroxyethyl)carbamate (0.11 g, 0.13 mmol) in 1,4-dioxane (1 mL) was added concentrated HCl solution (0.13 mL) in one portion under N₂ and the mixture was stirred at room temperature for 2 hr. The mixture was combined with another batch at 10 mg scale. The mixture was neutralized with saturated aqueous sodium bicarbonate solution. The mixture was purified by reverse phase HPLC to afford (S)-N-(3-(3'-chloro-6-methoxy-5-((((5-oxopyrrolidin-2-yl)methyl)amino)methyl)-[2,4'-bipyridin]-2'-yl)-2-methylphenyl)-5-(((2-hydroxyethyl)amino)methyl)picolinamide (25.2 mg, 28% yield) as a white solid. MS: m/z found 630 [M+H]⁺. ¹H NMR (400 MHz, Methanol-*d*₄): δ 8.68 (d, 1H), 8.60 (d, 1H), 8.19 (d, 1H), 8.04-8.01 (m, 2H), 7.79 (d, 1H), 7.72 (d, 1H), 7.41-7.37 (m, 2H), 7.17 (d, 1H), 4.02 (s, 3H), 3.94 (s, 2H), 3.83-3.79 (m, 3H), 3.67 (t, 2H), 2.75 (t, 2H), 2.70-2.63 (m, 2H), 2.34-2.22 (m, 3H), 2.15 (s, 3H), 1.83-1.76 (m, 1H).

### Example 723: (S)-N-(3-(3-Chloro-2-(3-(difluoromethoxy)-4-((((5-oxopyrrolidin-2-yl)methyl)amino)methyl)phenyl)pyridin-4-yl)-2-methylphenyl)-5-(((2-hydroxyethyl)amino)methyl)picolinamide (770)

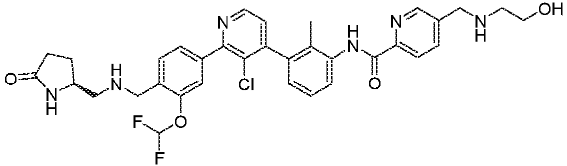

### (a) tert-Butyl ((6-((3-(3-chloro-2-(3-(difluoromethoxy)-4-formylphenyl)pyridin-4-yl)-2-methylphenyl)carbamoyl)pyridin-3-yl)methyl)(2-hydroxyethyl)carbamate

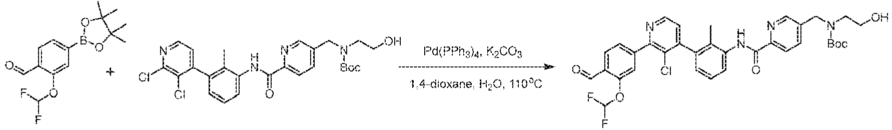

To a mixture of tert-butyl ((6-((3-(2,3-dichloropyridin-4-yl)-2-methylphenyl)carbamoyl)pyridin-3-yl)methyl)(2-hydroxyethyl)carbamate (Example 714, step (e)) (266 mg, 0.89 mmol) and 2-(difluoromethoxy)-4-(4,4,5,5-tetramethyl-1,3,2-dioxaborolan-2-yl)benzaldehyde (Example 529, step (b)) (190 mg, 0.38 mmol) in 1,4-dioxane / water (5:1, 12 mL) was added tetrakis(triphenylphosphine)palladium(0) (41.3 mg, 0.05 mmol) and potassium carbonate (148 mg, 1.07 mmol) and the mixture stirred at 110 °C for 12 h under N₂. The mixture was concentrated and the residue was purified by normal phase SiO₂ chromatography (10-50% ethyl acetate /petroleum ether) to afford tert-butyl ((6-((3-(3-chloro-2-(3-(difluoromethoxy)-4-formylphenyl)pyridin-4-yl)-2-methylphenyl)carbamoyl)pyridin-3-yl)methyl)(2-hydroxyethyl)carbamate (150 mg, 62 % yield) as white solid. MS: m/z found 667 [M+H]⁺.

### (b) tert-Butyl (S)-((6-((3-(3-chloro-2-(3-(difluoromethoxy)-4-((((5-oxopyrrolidin-2-yl)methyl)amino)methyl)phenyl)pyridin-4-yl)-2-methylphenyl)carbamoyl)pyridin-3-yl)methyl)(2-hydroxyethyl)carbamate

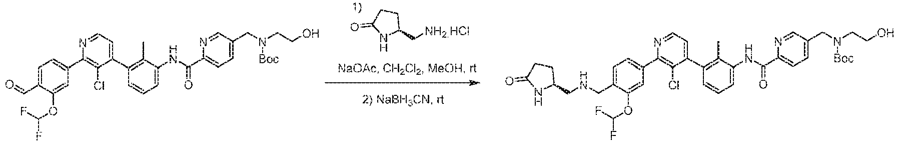

To a mixture of tert-butyl ((6-((3-(3-chloro-2-(3-(difluoromethoxy)-4-formylphenyl)pyridin-4-yl)-2-methylphenyl)carbamoyl)pyridin-3-yl)methyl)(2-hydroxyethyl)carbamate (140 mg, 0.21 mmol) and (S)-5-(aminomethyl)pyrrolidin-2-one hydrochloride salt (94.8 mg, 0.63 mmol) in dichloromethane/MeOH (1:1, 3 mL) was added sodium acetate (51.6 mg, 0.63 mmol) and the mixture stirred at room temperature for 2 h under N₂. Sodium cyanoborohydride (65.9 mg, 1.05 mmol) was then added and the mixture stirred at room temperature for 0.5 h under N₂. The mixture was concentrated and the residue purified by normal phase prep-TLC (SiO₂, ethyl acetate: methanol = 1:1) to afford tert-butyl (S)-((6-((3-(3-chloro-2-(3-(difluoromethoxy)-4-((((5-oxopyrrolidin-2-yl)methyl)amino)methyl)phenyl)pyridin-4-yl)-2-methylphenyl)carbamoyl)pyridin-3-yl)methyl)(2-hydroxyethyl)carbamate (80 mg, 77% yield) as white solid. MS: m/z found 765 [M+H]⁺.

### (c) (S)-N-(3-(3-Chloro-2-(3-(difluoromethoxy)-4-((((5-oxopyrrolidin-2 yl)methyl)amino)methyl)phenyl)pyridin-4-yl)-2-methylphenyl)-5-(((2-hydroxyethyl)amino)methyl)picolinamide

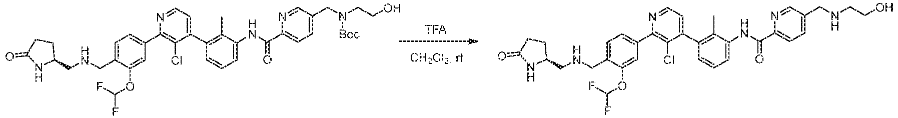

To a mixture of tert-butyl (S)-((6-((3-(3-chloro-2-(3-(difluoromethoxy)-4-((((5-oxopyrrolidin-2-yl)methyl)amino)methyl)phenyl)pyridin-4-yl)-2-methylphenyl)carbamoyl)pyridin-3-yl)methyl)(2-hydroxyethyl)carbamate (70 mg, 0.09 mmol) in dichloromethane (3 mL) was added trifluoroacetic acid (0.70 mL, 9.45 mmol) and the mixture was stirred at room temperature for 1 h under N₂. The mixture was concentrated and the residue purified by reverse phase HPLC to afford (S)-N-(3-(3-chloro-2-(3-(difluoromethoxy)-4-((((5-oxopyrrolidin-2 yl)methyl)amino)methyl)phenyl)pyridin-4-yl)-2-methylphenyl)-5-(((2-hydroxyethyl)amino)methyl)picolinamide (20.5 mg, 32 % yield) as white solid. MS: m/z found 665 [M+H]⁺, retention time = 2.68 min (Method A). ¹H NMR (400 MHz, Methanol-*d*₄): δ 8.72 (d, 1H), 8.64 (d, 1H), 8.23 (d, 1H), 8.05 (dd, 1H), 8.01 (d, 1H), 7.64-7.60 (m, 2H), 7.53 (s, 1H), 7.44-7.40 (m, 2H), 7.15-6.79 (m, 2H), 3.99-3.95 (m, 4H), 3.87-3.84 (m, 1H), 3.71 (t, 2H), 2.80-2.71 (m, 4H), 2.38-2.27 (m, 3H), 2.19 (s, 3H), 1.86-1.82 (m, 1H).

### Example 724: (S)-5-((((6-(((S)-4-(3'-chloro-6-methoxy-5-(((tetrahydro-2H-pyran-4-yl)amino)methyl)-[2,4'-bipyridin]-2'-yl)-2,3-dihydro-1H-inden-1-yl)oxy)-2-methoxy-5-(trifluoromethyl)pyridin-3-yl)methyl)amino)methyl)pyrrolidin-2-one (686)

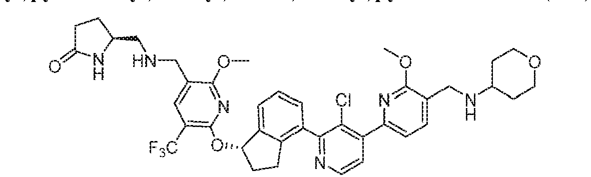

### (a) tert-Butyl ((2',3'-dichloro-6-methoxy-[2,4'-bipyridin]-5-yl)methyl)(tetrahydro-2H-pyran-4-yl)carbamate

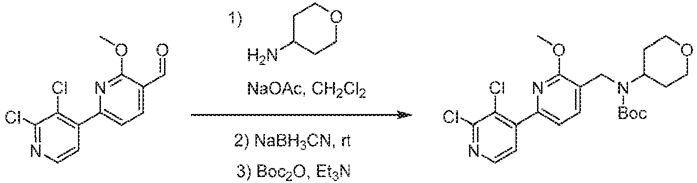

To a solution of 2',3'-dichloro-6-methoxy-[2,4'-bipyridine]-5-carbaldehyde (1.4 g, 4.95 mmol) (Example 640, step (a)) and tetrahydro-2H-pyran-4-amine (750 mg, 7.42 mmol) in dichloromethane (30 mL) was added sodium acetate (1.62 g, 19.8 mmol) and the solution was stirred at room temperature for 12 h. Sodium cyanoborohydride (932 mg, 14.8 mmol) was then added and the mixture stirred at room temperature for 2 h. Triethylamine (1.36 mL, 9.78 mmol) and di-tert-butyl dicarbonate (2.13 g, 9.78 mmol) were then added and the mixture stirred at room temperature for 4 h. The reaction was concentrated under reduced pressure and the residue purified by reverse phase HPLC to afford tert-butyl ((2',3'-dichloro-6-methoxy-[2,4'-bipyridin]-5-yl)methyl)(tetrahydro-2H-pyran-4-yl)carbamate (1.3 g, 56% yield) as a white solid.

### (b) tert-Butyl (S)-((3'-chloro-2'-(1-((5-formyl-6-methoxy-3-(trifluoromethyl)pyridin-2-yl)oxy)-2,3-dihydro-1H-inden-4-yl)-6-methoxy-[2,4'-bipyridin]-5-yl)methyl)(tetrahydro-2H-pyran-4-yl)carbamate

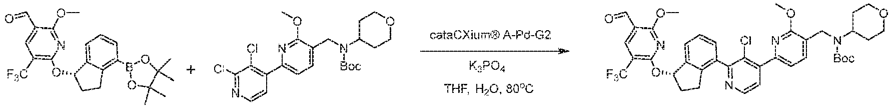

To a mixture of (S)-2-methoxy-6-((4-(4,4,5,5-tetramethyl-1,3,2-dioxaborolan-2-yl)-2,3-dihydro-1H-inden-1-yl)oxy)-5-(trifluoromethyl)nicotinaldehyde (Example 645, step (f) (640 mg, 1.38 mmol) and tert-butyl ((2',3'-dichloro-6-methoxy-[2,4'-bipyridin]-5-yl)methyl)(tetrahydro-2H-pyran-4-yl)carbamate (220 mg, 0.47 mmol) in THF/water (4/1, 7.5 mL) was added potassium phosphate (299 mg, 1.41 mmol) and Chloro[(di(1-adamantyl)-N-butylphosphine)-2-(2-aminobiphenyl)]palladium(II) (31.4 mg, 0.05 mmol) in one portion under N₂ and the mixture was stirred at 80 °C for 1 hr. Water (20 mL) was added and the mixture extracted with ethyl acetate (100 mL). The organic layer was dried over anhydrous sodium sulfate, filtered and concentrated. The residue was purified by normal phase SiO₂ chromatography (10-30% ethyl acetate/petroleum ether) to afford tert-butyl (S)-((3'-chloro-2'-(1-((5-formyl-6-methoxy-3-(trifluoromethyl)pyridin-2-yl)oxy)-2,3-dihydro-1H-inden-4-yl)-6-methoxy-[2,4'-bipyridin]-5-yl)methyl)(tetrahydro-2H-pyran-4-yl)carbamate (145 mg, 40% yield) as a yellow oil.

### (c) tert-Butyl((3'-chloro-6-methoxy-2'-((S)-1-((6-methoxy-5-(((((S)-5-oxopyrrolidin-2-yl)methyl)amino)methyl)-3-(trifluoromethyl)pyridin-2-yl)oxy)-2,3-dihydro-1H-inden-4-yl)-[2,4'-bipyridin]-5-yl)methyl)(tetrahydro-2H-pyran-4-yl)carbamate

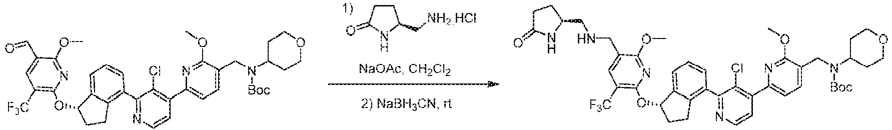

To a mixture of tert-butyl (S)-((3'-chloro-2'-(1-((5-formyl-6-methoxy-3-(trifluoromethyl)pyridin-2-yl)oxy)-2,3-dihydro-1H-inden-4-yl)-6-methoxy-[2,4'-bipyridin]-5-yl)methyl)(tetrahydro-2H-pyran-4-yl)carbamate (60 mg, 0.08 mmol) in dichloromethane (1 mL) was added (S)-5-(aminomethyl)pyrrolidin-2-one hydrochloride salt (17.6 mg, 0.11 mmol) and sodium acetate (19.2 mg, 0.23 mmol) and the mixture stirred for 2 hr at room temperature. Sodium cyanoborohydride (14.7 mg, 0.23 mmol) was then added and the mixture stirred for another 10 hr. Water (5 mL) was added and the mixture extracted with dichloromethane (30 mL). The organic layer was concentrated under vacuum and the residue purified by normal phase SiO₂ chromatography (0-100% ethyl acetate/petroleum ether to 0~50% MeOH/ethyl acetate) and then purified by prep-TLC (20% MeOH/ ethyl acetate) to afford tert-butyl ((3'-chloro-6-methoxy-2'-((S)-1-((6-methoxy-5-(((((S)-5-oxopyrrolidin-2-yl)methyl)amino)methyl)-3-(trifluoromethyl)pyridin-2-yl)oxy)-2,3-dihydro-1H-inden-4-yl)-[2,4'-bipyridin]-5-yl)methyl)(tetrahydro-2H-pyran-4-yl)carbamate (85 mg, 24% yield) as a white solid. MS: *m*/*z* found 867 [M+H]⁺.

### (d) (S)-5-((((6-(((S)-4-(3'-chloro-6-methoxy-5-(((tetrahydro-2H-pyran-4-yl)amino)methyl)-[2,4'-bipyridin]-2'-yl)-2,3-dihydro-1H-inden-1-yl)oxy)-2-methoxy-5-(trifluoromethyl)pyridin-3-yl)methyl)amino)methyl)pyrrolidin-2-one

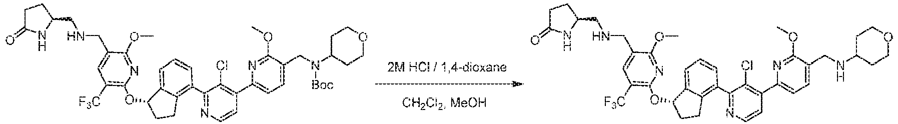

To a mixture of tert-butyl ((3'-chloro-6-methoxy-2'-((S)-1-((6-methoxy-5-(((((S)-5-oxopyrrolidin-2-yl)methyl)amino)methyl)-3-(trifluoromethyl)pyridin-2-yl)oxy)-2,3-dihydro-1H-inden-4-yl)-[2,4'-bipyridin]-5-yl)methyl)(tetrahydro-2H-pyran-4-yl)carbamate (74 mg, 85.3 umol) in dichloromethane/MeOH (3/1, 3.2 mL) was added 2M HCl in dioxane (2.5 mL, 5 mmol) and the reaction was stirred for 12 hr at room temperature. To the mixture was added 2M HCl in dioxane (1 mL) and the mixture stirred for another 12 hr. Saturated aqueous ammonia (0.5 mL) was then added and the mixture concentrated under reduced pressure. The residue was purified by reverse phase HPLC twice to afford (S)-5-((((6-(((S)-4-(3'-chloro-6-methoxy-5-(((tetrahydro-2H-pyran-4-yl)amino)methyl)-[2,4'-bipyridin]-2'-yl)-2,3-dihydro-1H-inden-1-yl)oxy)-2-methoxy-5-(trifluoromethyl)pyridin-3-yl)methyl)amino)methyl)pyrrolidin-2-one (2.3 mg, 3.4% yield) as a white solid (formic acid salt). MS: *m*/*z* found 767 [M+H]⁺. ¹H NMR (400 MHz, Methanol-*d*₄): δ 8.64 (d, 1H), 8.53 (br s, 1H), 7.91 (d, 2H), 7.73 (d, 1H), 7.59-7.56 (m, 1H), 7.48 (d, 1H), 7.41 (d, 2H), 6.69 (t, 1H), 4.13-4.10 (m, 7H), 4.04 (dd, 2H), 3.87-3.84 (m, 1H), 3.80 (d, 2H), 3.50-3.41 (m, 3H), 3.21-3.15 (m, 1H), 3.06-3.00 (m, 1H), 2.87-2.69 (m, 4H), 2.38-2.23 (m, 4 H), 2.07-2.03 (m, 2H), 1.86-1.81 (m, 1H), 1.65-1.61 (m, 2H).

### Example 725: 2-(4-(5-Chloro-6-(2-chloro-3-(5-(((2-hydroxyethyl)amino)methyl)-6-methoxypyridin-2-yl)phenyl)pyrimidin-4-yl)-2-methoxybenzyl)-2,6-diazaspiro[3.4]octan-7-one (711)

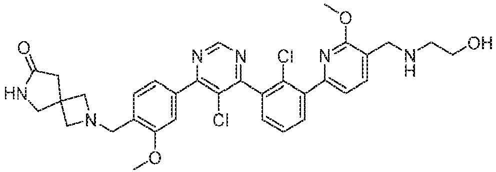

### (a) 6-(2-Chloro-3-(5,6-dichloropyrimidin-4-yl)phenyl)-2-methoxynicotinaldehyde

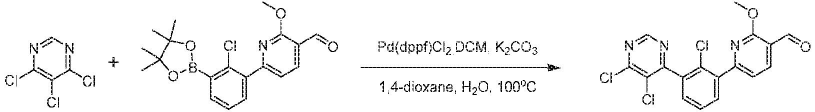

To a mixture of 6-(2-chloro-3-(4,4,5,5-tetramethyl-1,3,2-dioxaborolan-2-yl)phenyl)-2-methoxynicotinaldehyde (1 g, 2.68 mmol) and 4,5,6-trichloropyrimidine (0.98 g, 5.35 mmol) in 1,4-dioxane/water mixture (10:1, 20 mL) was added potassium carbonate (1.11 g, 8.03 mmol) and [1,1'- bis(diphenylphosphino)ferrocene]dichloropalladium(II) complex with dichloromethane (0.11 g, 0.13 mmol) in one portion under N₂ and the mixture was stirred at 100 °C for 3 hr. The mixture was concentrated, water (200 mL) and saturated aqueous brine solution (20 mL) were added to the residue, and the mixture was extracted with ethyl acetate/THF mixture (1:1, 200 mL). The organic phase was dried with anhydrous sodium sulfate, filtered and concentrated. The residue was purified by normal phase SiO₂ chromatography (0-15% ethyl acetate/petroleum ether) to afford 6-(2-chloro-3-(5,6-dichloropyrimidin-4-yl)phenyl)-2-methoxynicotinaldehyde (0.6 g, 51% yield) as a white solid. ¹H NMR (400 MHz, Chloroform-*d*) δ 10.36 (s, 1H), 8.91 (s, 1H), 8.13 (d, 1H), 7.71 (dd, 1H), 7.47 (t, 1H), 7.34-7.31 (m, 2H), 4.06 (s, 3H).

### (b) tert-Butyl ((6-(2-chloro-3-(5,6-dichloropyrimidin-4-yl)phenyl)-2-methoxypyridin-3-yl)methyl)(2-hydroxyethyl)carbamate

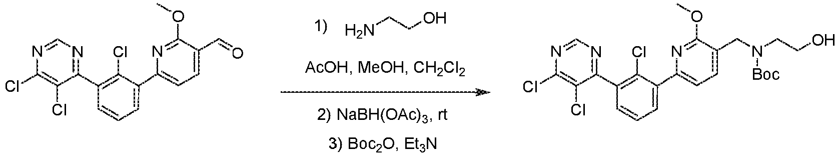

To a solution of 6-(2-chloro-3-(5,6-dichloropyrimidin-4-yl)phenyl)-2-methoxynicotinaldehyde (0.6 g, 1.5 mmol) and 2-aminoethanol (0.1 g, 2 mmol) in dichloromethane/methanol mixture (1:1, 10 mL) was added glacial acetic acid (0.2 mL, 3 mmol) and the mixture was stirred at room temperature for 0.5 hr. Sodium triacetoxyborohydride (0.3 g, 4.6 mmol) was then added and the mixture was stirred at room temperature for 0.5 hr. Triethylamine (0.2 mL, 1.5 mmol) and di-tert-butyl dicarbonate (1 mL, 3 mmol) were then added and the mixture was stirred at room temperature for 1 hr. Water (20 mL) was added and the mixture extracted with ethyl acetate (2 x 20 mL). The combined organic phases were washed with saturated aqueous brine solution (2 x 10 mL), dried with anhydrous sodium sulfate, filtered and concentrated. The residue was purified by normal phase SiO₂ chromatography (10-100% ethyl acetate/petroleum ether) to afford tert-butyl ((6-(2-chloro-3-(5,6-dichloropyrimidin-4-yl)phenyl)-2-methoxypyridin-3-yl)methyl)(2-hydroxyethyl)carbamate (570 mg, 70% yield) as a yellow solid. MS: *m*/*z* found 539 and 541 [M+H]⁺. ¹H NMR (400 MHz, DMSO-*d*₆): δ 9.16 (s, 1H), 7.80 (dd, 1H), 7.67-7.65 (m, 1H), 7.63-7.60 (m, 2H), 7.31 (d, 1H), 4.42 (s, 2H), 3.95 (s, 3H), 3.54-3.53 (m, 2H), 3.33-3.32 (m, 2H), 1.45-1.35 (m, 9H).

### (c) tert-Butyl ((6-(2-chloro-3-(5-chloro-6-(4-formyl-3-methoxyphenyl)pyrimidin-4-yl)phenyl)-2-methoxypyridin-3-yl)methyl)(2-hydroxyethyl)carbamate

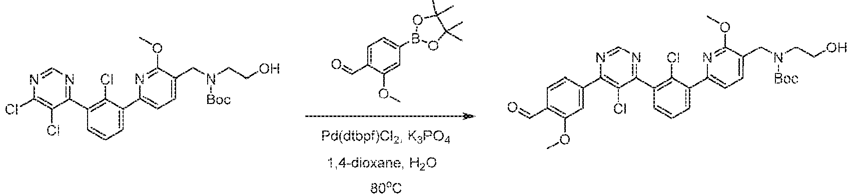

To a solution of tert-butyl ((6-(2-chloro-3-(5,6-dichloropyrimidin-4-yl)phenyl)-2-methoxypyridin-3-yl)methyl)(2-hydroxyethyl)carbamate (0.3 g, 0.6 mmol) and 2-methoxy-4-(4,4,5,5-tetramethyl-1,3,2-dioxaborolan-2-yl)benzaldehyde (Example 714, step (f)) (0.15 g, 0.6 mmol) in 1,4-dioxane/water mixture (5:1, 6 mL) was added potassium phosphate (0.35 g, 2 mmol) and [1,1'-bis(di-tert-butylphosphino)ferrocene]dichloropalladium(II) (18 mg, 0.03 mmol) and the mixture was stirred at 80 °C for 1 hr. Water (20 mL) was added and the mixture was extracted with ethyl acetate (2 x 20 mL). The combined organic phases were washed with saturated aqueous brine solution (2 x 20 mL), dried with anhydrous sodium sulfate, filtered and concentrated. The residue was purified by normal phase SiO₂ chromatography (0-50% ethyl acetate/petroleum ether) to afford tert-butyl ((6-(2-chloro-3-(5-chloro-6-(4-formyl-3-methoxyphenyl)pyrimidin-4-yl)phenyl)-2-methoxypyridin-3-yl)methyl)(2-hydroxyethyl)carbamate (250 mg, 70% yield) as a yellow solid. MS: *m*/*z* found 639 [M+H]⁺.

### (d) tert-Butyl ((6-(2-chloro-3-(5-chloro-6-(3-methoxy-4-((7-oxo-2,6-diazaspiro[3.4]octan-2-yl)methyl)phenyl)pyrimidin-4-yl)phenyl)-2-methoxypyridin-3-yl)methyl)(2-hydroxyethyl)carbamate

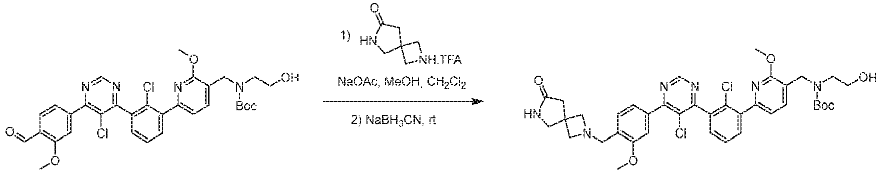

To a solution of tert-butyl ((6-(2-chloro-3-(5-chloro-6-(4-formyl-3-methoxyphenyl)pyrimidin-4-yl)phenyl)-2-methoxypyridin-3-yl)methyl)(2-hydroxyethyl)carbamate (0.23 g, 0.36 mmol) and 2,6-diazaspiro[3.4]octan-7-one trifluoroacetic acid salt (0.78 g, 3.24 mmol) in dichloromethane/methanol mixture (10:3, 13 mL) was added sodium acetate (0.35 g, 4.32 mmol) and the mixture was stirred at room temperature for 12 hr. Sodium cyanoborohydride (0.07 g, 1.08 mmol) was then added and the mixture was stirred at room temperature for 1 hr. The mixture was concentrated and the residue purified by normal phase SiO₂ chromatography (20-100% ethyl acetate/petroleum ether) to afford tert-butyl ((6-(2-chloro-3-(5-chloro-6-(3-methoxy-4-((7-oxo-2,6-diazaspiro[3.4]octan-2-yl)methyl)phenyl)pyrimidin-4-yl)phenyl)-2-methoxypyridin-3-yl)methyl)(2-hydroxyethyl)carbamate (0.55 g, crude) as a white solid. MS: *m*/*z* found 749 [M+H]⁺.

### (e) 2-(4-(5-Chloro-6-(2-chloro-3-(5-(((2-hydroxyethyl)amino)methyl)-6-methoxypyridin-2-yl)phenyl)pyrimidin-4-yl)-2-methoxybenzyl)-2,6-diazaspiro[3.4]octan-7-one

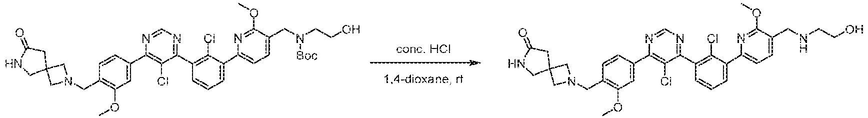

To a solution of tert-butyl ((6-(2-chloro-3-(5-chloro-6-(3-methoxy-4-((7-oxo-2,6-diazaspiro[3.4]octan-2-yl)methyl)phenyl)pyrimidin-4-yl)phenyl)-2-methoxypyridin-3-yl)methyl)(2-hydroxyethyl)carbamate (0.5 g, 0.7 mmol) in 1,4-dioxane (3 mL) was added concentrated HCl solution (0.4 mL) and the mixture stirred at room temperature for 1 hr. The reaction mixture was concentrated and the residue was purified by reverse phase HPLC to afford 2-(4-(5-chloro-6-(2-chloro-3-(5-(((2-hydroxyethyl)amino)methyl)-6-methoxypyridin-2-yl)phenyl)pyrimidin-4-yl)-2-methoxybenzyl)-2,6-diazaspiro[3.4]octan-7-one (29.6 mg, 6% yield) as a white solid. MS: *m*/*z* found 649 [M+H]⁺, retention time = 2.23 min (Method A). ¹H NMR (400 MHz, Methanol-*d*₄): δ 9.21 (s, 1H), 7.80-7.75 (m, 2H), 7.60 (t, 1H), 7.54-7.48 (m, 3H), 7.45-7.43 (m, 1H), 7.29 (d, 1H), 4.05 (s, 3H), 3.94 (s, 3H), 3.88 (s, 2H), 3.78 (s, 2H), 3.72 (t, 2H), 3.59 (s, 2H), 3.45-3.41 (m, 4H), 2.79 (t, 2H), 2.58 (s, 2H).

### Example 726: (S)-2-(4-(5-Chloro-6-(2-chloro-3-(6-methoxy-5-((((5-oxopyrrolidin-2-yl)methyl)amino)methyl)pyridin-2-yl)phenyl)pyrimidin-4-yl)-2-methoxybenzyl)-2,6-diazaspiro[3.4]octan-7-one (742)

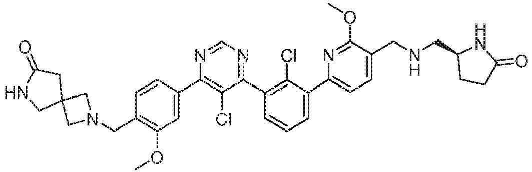

### (a) tert-Butyl (S)-((6-(2-chloro-3-(5,6-dichloropyrimidin-4-yl)phenyl)-2-methoxypyridin-3-yl)methyl)((5-oxopyrrolidin-2-yl)methyl)carbamate

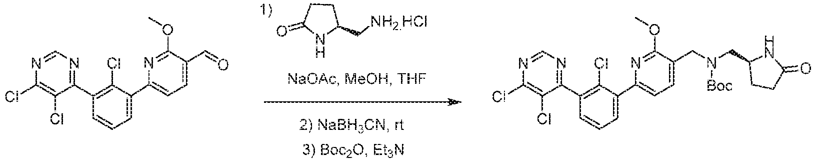

To a solution of 6-(2-chloro-3-(5,6-dichloropyrimidin-4-yl)phenyl)-2-methoxynicotinaldehyde (0.6 g, 1.52 mmol) (Example 725, step (a)) and (S)-5-(aminomethyl)-2-pyrrolidinone hydrochloride salt (0.25 g, 1.67 mmol) in THF/MeOH mixture (1:1, 6 mL) was added sodium acetate (0.37 g, 4.56 mmol) and the mixture was stirred at room temperature for 1 hr. Sodium cyanoborohydride (0.29 g, 4.56 mmol) was then added and the mixture stirred at room temperature for 1 hr. Di-tert-butyl dicarbonate (0.66 g, 3 mmol) and triethylamine (0.2 mL, 1.5 mmol) were then added and the mixture was stirred at room temperature for 1 hr. Water (20 mL) was then added and the mixture extracted with ethyl acetate (2 x 10 mL). The combined organic phases were washed with saturated aqueous brine solution (2 x 10 mL), dried with anhydrous sodium sulfate, filtered and concentrated. The residue was purified by normal phase SiO₂ chromatography (0-100% ethyl acetate/petroleum ether) to afford tert-butyl (S)-((6-(2-chloro-3-(5,6-dichloropyrimidin-4-yl)phenyl)-2-methoxypyridin-3-yl)methyl)((5-oxopyrrolidin-2-yl)methyl)carbamate (640 mg, 72% yield) as a white solid. MS: *m*/*z* found 592 and 594 [M+H]⁺.

### (b) tert-Butyl (S)-((6-(2-chloro-3-(5-chloro-6-(4-formyl-3-methoxyphenyl)pyrimidin-4-yl)phenyl)-2-methoxypyridin-3-yl)methyl)((5-oxopyrrolidin-2-yl)methyl)carbamate

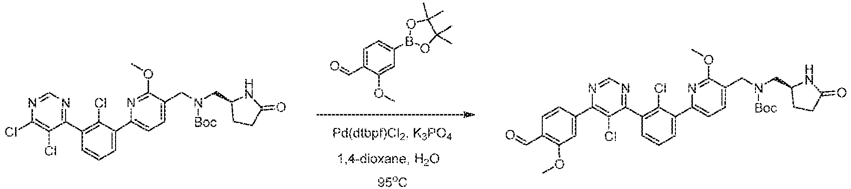

To a solution of tert-butyl (S)-((6-(2-chloro-3-(5,6-dichloropyrimidin-4-yl)phenyl)-2-methoxypyridin-3-yl)methyl)((5-oxopyrrolidin-2-yl)methyl)carbamate (0.1 g, 0.17 mmol) and 2-methoxy-4-(4,4,5,5-tetramethyl-1,3,2-dioxaborolan-2-yl)benzaldehyde (Example 714, step (f)) (0.07 g, 0.25 mmol) in 1,4-dioxane/water mixture (10:1, 3 mL) was added potassium phosphate (0.11 g, 0.5 mmol) and [1,1'-bis(di-tert-butylphosphino)ferrocene]dichloropalladium(II) (6 mg, 8 umol) and the mixture stirred at 95 °C for 2 hr. Water (20 mL) was then added and the mixture was extracted with ethyl acetate (2 x 20 mL). The combined organic phases were washed with saturated aqueous brine solution (2 x 20 mL), dried with anhydrous sodium sulfate, filtered and concentrated. The residue was purified by normal phase SiO₂ chromatography (0-50% ethyl acetate/petroleum ether) to afford tert-butyl (S)-((6-(2-chloro-3-(5-chloro-6-(4-formyl-3-methoxyphenyl)pyrimidin-4-yl)phenyl)-2-methoxypyridin-3-yl)methyl)((5-oxopyrrolidin-2-yl)methyl)carbamate (450 mg, 50% yield) as a white solid. MS: *m*/*z* found 692 [M+H]⁺.

### (c) tert-Butyl (S)-((6-(2-chloro-3-(5-chloro-6-(3-methoxy-4-((7-oxo-2,6-diazaspiro[3.4]octan-2-yl)methyl)phenyl)pyrimidin-4-yl)phenyl)-2-methoxypyridin-3-yl)methyl)((5-oxopyrrolidin-2-yl)methyl)carbamate

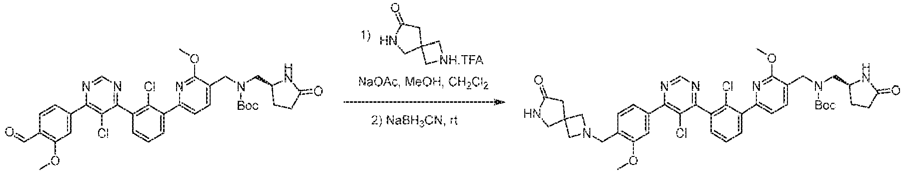

To a solution of tert-butyl (S)-((6-(2-chloro-3-(5-chloro-6-(4-formyl-3-methoxyphenyl)pyrimidin-4-yl)phenyl)-2-methoxypyridin-3-yl)methyl)((5-oxopyrrolidin-2-yl)methyl)carbamate (0.42 g, 0.61 mmol) and 2,6-diazaspiro[3.4]octan-7-one trifluoroacetic acid salt (0.66 g, 2.73 mmol) in dichloromethane/methanol mixture (1:1, 40 mL) was added sodium acetate (0.6 g, 7.28 mmol) and the mixture was stirred at room temperature for 1 hr. Sodium cyanoborohydride (0.11 g, 1.82 mmol) was then added and the mixture stirred at room temperature for 1 hr. The mixture was purified by reverse phase HPLC to afford tert-butyl (S)-((6-(2-chloro-3-(5-chloro-6-(3-methoxy-4-((7-oxo-2,6-diazaspiro[3.4]octan-2-yl)methyl)phenyl)pyrimidin-4-yl)phenyl)-2-methoxypyridin-3-yl)methyl)((5-oxopyrrolidin-2-yl)methyl)carbamate (70 mg, 14% yield) as a white solid. MS: *m*/*z* found 802 [M+H]⁺.

### (d) (S)-2-(4-(5-Chloro-6-(2-chloro-3-(6-methoxy-5-((((5-oxopyrrolidin-2-yl)methyl)amino)methyl)pyridin-2-yl)phenyl)pyrimidin-4-yl)-2-methoxybenzyl)-2,6-diazaspiro[3.4]octan-7-one

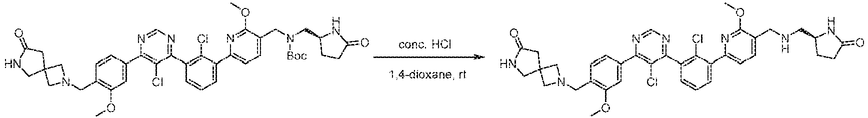

To a solution of tert-butyl (S)-((6-(2-chloro-3-(5-chloro-6-(3-methoxy-4-((7-oxo-2,6-diazaspiro[3.4]octan-2-yl)methyl)phenyl)pyrimidin-4-yl)phenyl)-2-methoxypyridin-3-yl)methyl)((5-oxopyrrolidin-2-yl)methyl)carbamate (65 mg, 0.081 mmol) in 1,4-dioxane (2 mL) was added concentrated HCl (0.4 mL) and the mixture was stirred at room temperature for 1 hr. The mixture was purified by reverse phase HPLC to afford (S)-2-(4-(5-chloro-6-(2-chloro-3-(6-methoxy-5-((((5-oxopyrrolidin-2-yl)methyl)amino)methyl)pyridin-2-yl)phenyl)pyrimidin-4-yl)-2-methoxybenzyl)-2,6-diazaspiro[3.4]octan-7-one (27.4 mg, 48% yield) as a white solid. MS: *m*/*z* found 702 [M+H]⁺, retention time = 2.36 min (Method A). ¹H NMR (400 MHz, Methanol-*d*₄): δ 9.21 (s, 1H), 7.79-7.76 (m, 2H), 7.60 (t, 1H), 7.52-7.46 (m, 3H), 7.44-7.42 (m, 1H), 7.28 (d, 1H), 4.04 (s, 3H), 3.94 (s, 3H), 3.89-3.82 (m, 3H), 3.78 (s, 2H), 3.59 (s, 2H), 3.45-3.40 (m, 4H), 2.73-2.69 (m, 2H), 2.58 (s, 2H), 2.37-2.30 (m, 3H), 1.88-1.79 (m, 1H).

### Example 727: 2-(4-(5-Chloro-6-(2-chloro-3-(5-((((1s,3s)-3-hydroxycyclobutyl)amino)methyl)-6-methoxypyridin-2-yl)phenyl)pyrimidin-4-yl)-2-methoxybenzyl)-2,6-diazaspiro[3.4]octan-7-one (737)

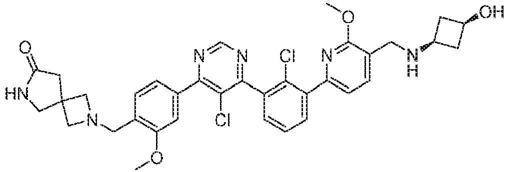

2-(4-(5-Chloro-6-(2-chloro-3-(5-((((1s,3s)-3-hydroxycyclobutyl)amino)methyl)-6-methoxypyridin-2-yl)phenyl)pyrimidin-4-yl)-2-methoxybenzyl)-2,6-diazaspiro[3.4]octan-7-one was prepared in a similar fashion to Example 726, replacing (S)-5-(aminomethyl)-2-pyrrolidinone hydrochloride salt with (1s,3s)-3-aminocyclobutan-1-ol hydrochloride salt in step (a). MS: *m*/*z* found 675 [M+H]⁺, retention time = 2.44 min (Method A). ¹H NMR (400 MHz, Methanol-*d*₄): δ 9.21 (s, 1H), 7.78 (dd, 1H), 7.73 (d, 1H), 7.60 (t, 1H), 7.54-7.48 (m, 3H), 7.43 (d, 1H), 7.27 (d, 1H), 4.05 (s, 3H), 3.99-3.89 (m, 4H), 3.78 (s, 2H), 3.75 (s, 2H), 3.59 (s, 2H), 3.45-3.40 (m, 4H), 2.89-2.81 (m, 1H), 2.62-2.56 (m, 4H), 1.73-1.66 (m, 2H).

### Example 728: 2-(4-(5-Chloro-6-(2-chloro-3-(5-((((1r,3r)-3-hydroxycyclobutyl)amino)methyl)-6-methoxypyridin-2-yl)phenyl)pyrimidin-4-yl)-2-methoxybenzyl)-2,6-diazaspiro[3.4]octan-7-one (740)

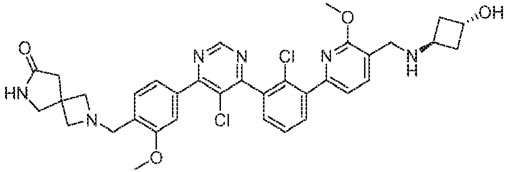

2-(4-(5-Chloro-6-(2-chloro-3-(5-((((1r,3r)-3-hydroxycyclobutyl)amino)methyl)-6-methoxypyridin-2-yl)phenyl)pyrimidin-4-yl)-2-methoxybenzyl)-2,6-diazaspiro[3.4]octan-7-one was prepared in a similar fashion to Example 726, replacing (S)-5-(aminomethyl)-2-pyrrolidinone hydrochloride salt with (1r,3r)-3-aminocyclobutan-1-ol hydrochloride salt in step (a). MS: *m*/*z* found 675 [M+H]⁺, retention time = 2.41 min (Method A). ¹H NMR (400 MHz, Methanol-*d*₄): δ 9.21 (s, 1H), 7.78 (dd, 1H), 7.73 (d, 1H), 7.60 (t, 1H), 7.53-7.48 (m, 3H), 7.43 (d, 1H), 7.27 (d, 1H), 4.45-4.40 (m, 1H), 4.04 (s, 3H), 3.94 (s, 3H), 3.78 (s, 2H), 3.73 (s, 2H), 3.59 (s, 2H), 3.51-3.44 (m, 1H), 3.42-3.33 (m, 4H), 2.58 (s, 2H), 2.22-2.12 (m, 4H).

### Example 729: (S)-5-((((6-(2-chloro-3-(5-methoxy-6-(3-methoxy-4-(((((S)-5-oxopyrrolidin-2-yl)methyl)amino)methyl)phenyl)pyrimidin-4-yl)phenyl)-2-methoxypyridin-3-yl)methyl)amino)methyl)pyrrolidin-2-one (716)

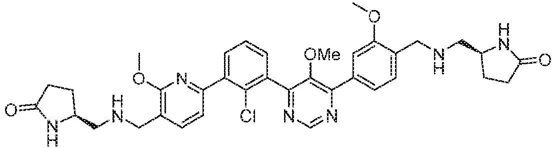

### (a) 6-(2-Chloro-3-(6-chloro-5-methoxypyrimidin-4-yl)phenyl)-2-methoxynicotinaldehyde

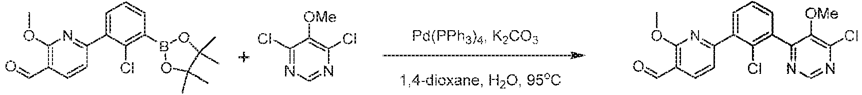

To a mixture of 6-(2-chloro-3-(4,4,5,5-tetramethyl-1,3,2-dioxaborolan-2-yl)phenyl)-2-methoxynicotinaldehyde (2 g, 5.35 mmol) and 4,6-dichloro-5-methoxypyrimidine (1.44 g, 8.03 mmol) in 1,4-dioxane/water (5/1, 120 mL) was added potassium carbonate (2.22 g, 16.1 mmol) and tetrakis(triphenylphosphine)palladium(0) (309 mg, 0.27 mmol) in one portion under N₂ and the mixture stirred at 95 °C for 5 hr. Saturated aqueous brine solution (30 ml) was added and the mixture extracted with ethyl acetate (100 ml). The organic phase was dried with anhydrous sodium sulfate, filtered and concentrated. The residue was purified by normal phase SiO₂ chromatography (0-30% ethyl acetate/petroleum ether) to afford 6-(2-chloro-3-(6-chloro-5-methoxypyrimidin-4-yl)phenyl)-2-methoxynicotinaldehyde (0.8 g, 32% yield) as a yellow oil. ¹H NMR (400 MHz, Chloroform-d): δ 10.41 (s, 1 H), 8.82 (s, 1 H), 8.18 (d, 1 H), 7.75 (dd, 1 H), 7.51 (t, 1 H), 7.44 (dd, 1 H), 7.39 (d, 1 H), 4.11 (s, 3 H), 3.73 (s, 3 H).

### (b) 6-(2-Chloro-3-(6-(4-formyl-3-methoxyphenyl)-5-methoxypyrimidin-4-yl)phenyl)-2-methoxynicotinaldehyde

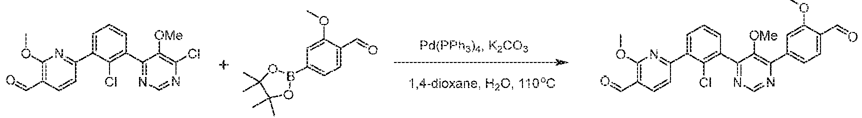

To a solution of 6-(2-chloro-3-(6-chloro-5-methoxypyrimidin-4-yl)phenyl)-2-methoxynicotinaldehyde (800 mg, 2.05 mmol) and 2-methoxy-4-(4,4,5,5-tetramethyl-1,3,2-dioxaborolan-2-yl)benzaldehyde (Example 714, step (f)) (591 mg, 2.26 mmol) in 1,4-dioxane/water (10/1, 44 mL) was added tetrakis(triphenylphosphine)palladium(0) (237 mg, 0.21 mmol) and potassium carbonate (850 mg, 6.15 mmol) and the mixture stirred at 110 °C for 12 hr. The mixture combined with another batch at 200 mg scale. Water (20 mL) was added and the mixture extracted with ethyl acetate (100 mL). The organic phase was dried with anhydrous sodium sulfate, filtered and concentrated. The residue was purified by normal phase SiO₂ chromatography (0-100% ethyl acetate/petroleum ether) to afford 6-(2-chloro-3-(6-(4-formyl-3-methoxyphenyl)-5-methoxypyrimidin-4-yl)phenyl)-2-methoxynicotinaldehyde (430 mg, 40% yield) as a white solid. MS: *m*/*z* found 490 and 492 [M+H]⁺. ¹H NMR (400 MHz, chloroform-d): δ 10.56 (s, 1H), 10.44 (s, 1H), 9.16 (s, 1H), 8.21 (d, 1H), 7.97 (d, 1H), 7.84-7.77 (m, 3H), 7.55 (d, 2H), 7.45 (d, 1H), 4.14 (s, 3H), 4.06 (s, 3H), 3.47 (s, 3H).

### (c) (S)-5-((((6-(2-Chloro-3-(5-methoxy-6-(3-methoxy-4-(((((S)-5-oxopyrrolidin-2-yl)methyl)amino)methyl)phenyl)pyrimidin-4-yl)phenyl)-2-methoxypyridin-3-yl)methyl)amino)methyl)pyrrolidin-2-one

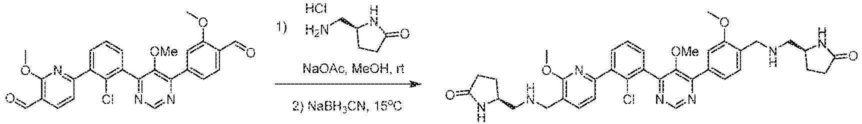

A mixture of 6-(2-chloro-3-(6-(4-formyl-3-methoxyphenyl)-5-methoxypyrimidin-4-yl)phenyl)-2-methoxynicotinaldehyde (80 mg, 0.16 mmol), (S)-5-(aminomethyl)pyrrolidin-2-one hydrochloride salt (98.4 mg, 0.65 mmol) and sodium acetate (67 mg, 0.82 mmol) in methanol (5 mL) was stirred for 12 hr. Sodium cyanoborohydride (51.3 mg, 0.82 mmol) was then added and the mixture stirred for another 1 hr at 15 °C. The reaction mixture was concentrated under reduced pressure and the residue purified by reverse phase HPLC to afford (S)-5-((((6-(2-chloro-3-(5-methoxy-6-(3-methoxy-4-(((((S)-5-oxopyrrolidin-2-yl)methyl)amino)methyl)phenyl)pyrimidin-4-yl)phenyl)-2-methoxypyridin-3-yl)methyl)amino)methyl)pyrrolidin-2-one (48.1 mg, 42% yield) as a white solid. MS: *m*/*z* found 686 [M+H]⁺, retention time = 2.13 min (Method A). ¹H NMR (400 MHz, Methanol-*d*₄): δ 9.04 (s, 1 H), 7.79-7.76 (m, 4 H), 7.60-7.59 (m, 2 H), 7.48 (d, 1 H), 7.30 (d, 1 H), 4.05 (s, 3 H), 3.98 (s, 3 H), 3.91-3.85 (m, 6 H), 3.46 (s, 3 H), 2.75-2.69 (m, 4 H), 2.38-2.28 (m, 6 H), 1.86-1.81 (m, 2 H).

### Example 730: 2-((6-(2-Chloro-3-(5-methoxy-6-(3-methoxy-4-((7-oxo-2,6-diazaspiro[3.4]octan-2-yl)methyl)phenyl)pyrimidin-4-yl)phenyl)-2-methoxypyridin-3-yl)methyl)-2,6-diazaspiro[3.4]octan-7-one (727)

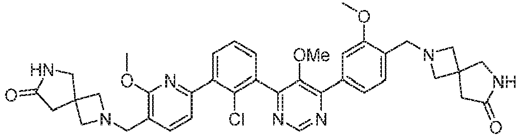

2-((6-(2-Chloro-3-(5-methoxy-6-(3-methoxy-4-((7-oxo-2,6-diazaspiro[3.4]octan-2-yl)methyl)phenyl)pyrimidin-4-yl)phenyl)-2-methoxypyridin-3-yl)methyl)-2,6-diazaspiro[3.4]octan-7-one was prepared in a similar fashion to Example 729, replacing (S)-5-(aminomethyl)pyrrolidin-2-one hydrochloride salt with 2,6-diazaspiro[3.4]octan-7-one trifluoroacetic acid salt in step (c). MS: *m*/*z* found 710 [M+H]⁺, retention time = 2.25 min (Method A). ¹H NMR (400 MHz, Methanol-*d*₄): δ 9.04 (s, 1H), 7.78-7.75 (m, 3H), 7.72 (d, 1H), 7.59-7.58 (m, 2H), 7.44 (d, 1H), 7.29 (d, 1H), 4.02 (s, 3H), 3.96 (s, 3H), 3.79 (s, 2H), 3.73 (s, 2H), 3.60 (d, 4H), 3.45-3.42 (m, 11H), 2.59 (d, 4H).

### Example 731: 2-((4-(6-(2-Chloro-3-(5-(((2-hydroxyethyl)amino)methyl)-6-methoxypyridin-2-yl)phenyl)-5-methoxypyrimidin-4-yl)-2-methoxybenzyl)amino)ethan-1-ol (728)

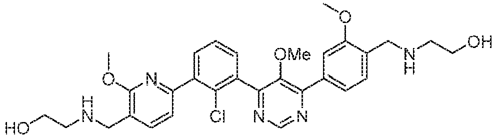

2-((4-(6-(2-Chloro-3-(5-(((2-hydroxyethyl)amino)methyl)-6-methoxypyridin-2-yl)phenyl)-5-methoxypyrimidin-4-yl)-2-methoxybenzyl)amino)ethan-1-ol was prepared in a similar fashion to Example 729, replacing (S)-5-(aminomethyl)pyrrolidin-2-one hydrochloride salt with 2-aminoethanol in step (c). MS: *m*/*z* found 580 [M+H]⁺, retention time = 2.22 min (Method A). ¹H NMR (400 MHz, Methanol-*d*₄): δ 9.05 (s, 1 H), 7.79-7.75 (m, 4 H), 7.60-7.59 (m, 2 H), 7.47 (d, 1 H), 7.30 (d, 1 H), 4.05 (s, 3 H), 3.99 (s, 3 H), 3.95 (s, 2 H), 3.88 (s, 2 H), 3.73-3.71 (m, 4 H), 3.46 (s, 3 H), 2.82-2.78 (m, 4 H).

### Example 732: N-((6-(2-Chloro-3-(5-methoxy-6-(3-methoxy-4-(((tetrahydro-2H-pyran-4-yl)amino)methyl)phenyl)pyrimidin-4-yl)phenyl)-2-methoxypyridin-3-yl)methyl)tetrahydro-2H-pyran-4-amine (734)

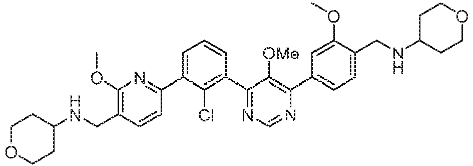

N-((6-(2-Chloro-3-(5-methoxy-6-(3-methoxy-4-(((tetrahydro-2H-pyran-4-yl)amino)methyl)phenyl)pyrimidin-4-yl)phenyl)-2-methoxypyridin-3-yl)methyl)tetrahydro-2H-pyran-4-amine was prepared in a similar fashion to Example 729, replacing (S)-5-(aminomethyl)pyrrolidin-2-one hydrochloride salt with tetrahydro-2H-pyran-4-amine in step (c). MS: *m*/*z* found 660 [M+H]⁺, retention time = 2.68 min (Method A). ¹H NMR (400 MHz, Methanol-*d*₄): δ 9.04 (s, 1 H), 7.79-7.76 (m, 4 H), 7.62-7.58 (m, 2 H), 7.48 (d, 1 H), 7.30 (d, 1 H), 4.05 (s, 3 H), 3.99-3.97 (m, 7 H), 3.93 (s, 2 H), 3.88 (s, 2 H), 3.46-3.39 (m, 7 H), 2.80-2.74 (m, 2 H), 1.95-1.92 (m, 4 H), 1.54-1.44 (m, 4 H).

### Example 733: 2-((6-(2-Chloro-3-(5-methoxy-6-(3-methoxy-4-((6-oxo-2,5-diazaspiro[3.4]octan-2-yl)methyl)phenyl)pyrimidin-4-yl)phenyl)-2-methoxypyridin-3-yl)methyl)-2,5-diazaspiro[3.4]octan-6-one (743)

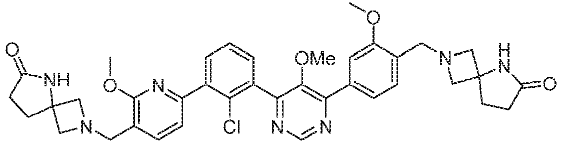

2-((6-(2-Chloro-3-(5-methoxy-6-(3-methoxy-4-((6-oxo-2,5-diazaspiro[3.4]octan-2-yl)methyl)phenyl)pyrimidin-4-yl)phenyl)-2-methoxypyridin-3-yl)methyl)-2,5-diazaspiro[3.4]octan-6-one was prepared in a similar fashion to Example 729, replacing (S)-5-(aminomethyl)pyrrolidin-2-one hydrochloride salt with 2,5-diazaspiro[3.4]octan-6-one trifluoroacetic acid salt in step (c). MS: *m*/*z* found 710 [M+H]⁺, retention time = 2.28 min (Method A). ¹H NMR (400 MHz, Methanol-*d*₄): δ 9.04 (s, 1 H), 7.78-7.76 (m, 3 H), 7.73 (d, 1 H), 7.62-7.57 (m, 2 H), 7.44 (d, 1 H), 7.30 (d, 1 H), 4.02 (s, 3 H), 3.96 (s, 3 H), 3.80 (s, 2 H), 3.74 (s, 2 H), 3.60-3.56 (m, 4 H), 3.45 (s, 3 H), 3.40 (d, 4 H), 2.45-2.35 (m, 8 H).

### Example 734: 1-(4-(((6-(3-(6-(4-(((1-Acetylpiperidin-4-yl)amino)methyl)-3-methoxyphenyl)-5-methoxypyrimidin-4-yl)-2-chlorophenyl)-2-methoxypyridin-3-yl)methyl)amino)piperidin-1-yl)ethan-1-one (717)

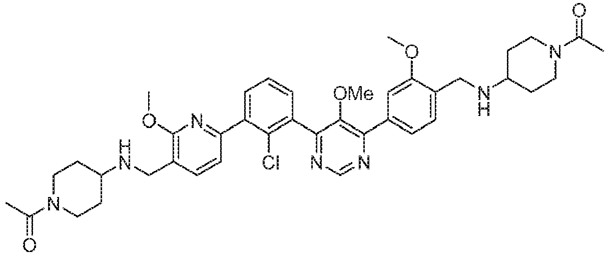

1-(4-(((6-(3-(6-(4-(((1-Acetylpiperidin-4-yl)amino)methyl)-3-methoxyphenyl)-5-methoxypyrimidin-4-yl)-2-chlorophenyl)-2-methoxypyridin-3-yl)methyl)amino)piperidin-1-yl)ethan-1-one was prepared in a similar fashion to Example 729, replacing (S)-5-(aminomethyl)pyrrolidin-2-one hydrochloride salt with 1-(4-aminopiperidin-1-yl)ethan-1-one in step (c). MS: *m*/*z* found 742 [M+H]⁺, retention time = 2.33 min (Method A). ¹H NMR (400 MHz, Methanol-*d*₄): δ 9.04 (s, 1H), 7.79-7.76 (m, 4H), 7.60-7.58 (m, 2H), 7.49 (d, 1H), 7.30 (d, 1H), 4.49 (d, 2H), 4.05 (s, 3H), 3.99-3.94 (m, 7H), 3.89 (s, 2H), 3.46 (s, 3H), 3.20-3.12 (m, 2H), 2.84-2.71 (m, 4H), 2.12 (d, 6H), 2.07-2.00 (m, 4H), 1.43-1.29 (m, 4H).

### Example 735: (S)-N-(2-Chloro-3-(3-chloro-6'-methoxy-5'-((((5-oxopyrrolidin-2-yl)methyl)amino)methyl)-[2,2'-bipyridin]-4-yl)phenyl)-1,3-dimethyl-2,4-dioxo-1,2,3,4-tetrahydropyrimidine-5-carboxamide (738)

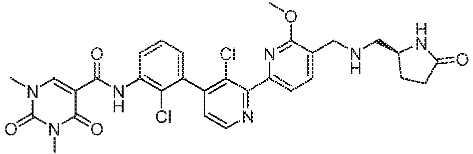

### (a) 1,3-Dimethyl-2,4-dioxo-1,2,3,4-tetrahydropyrimidine-5-carbonyl chloride

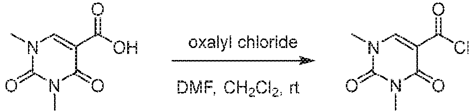

To a mixture of 1,3-dimethyl-2,4-dioxo-1,2,3,4-tetrahydropyrimidine-5-carboxylic acid (1 g, 5.43 mmol) in dichloromethane (15 mL) was added oxalyl chloride (0.82 g, 6.52 mmol) and DMF (0.039 g, 0.54 mmol) in one portion under N₂ and the mixture stirred at room temperature for 0.5 h. The mixture was concentrated to afford 1,3-dimethyl-2,4-dioxo-1,2,3,4-tetrahydropyrimidine-5-carbonyl chloride as a white solid (1.2 g, crude).

### (b) N-(2-Chloro-3-(4,4,5,5-tetramethyl-1,3,2-dioxaborolan-2-yl)phenyl)-1,3-dimethyl-2,4-dioxo-1,2,3,4-tetrahydropyrimidine-5-carboxamide

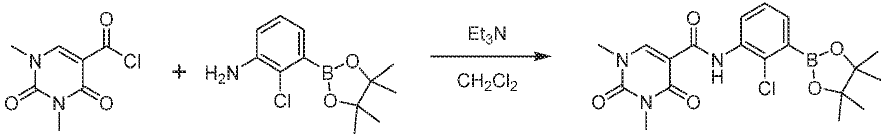

To a mixture of 1,3-dimethyl-2,4-dioxo-1,2,3,4-tetrahydropyrimidine-5-carbonyl chloride (1.2 g, 5.92 mmol) and 2-chloro-3-(4,4,5,5-tetramethyl-1,3,2-dioxaborolan-2-yl)aniline (1.65 g, 6.52 mmol) in dichloromethane (20 mL) was added triethylamine (2.47 mL, 17.7 mmol) in one portion at room temperature under N₂ and the mixture stirred at room temperature for 12 h. The mixture was concentrated, water (5 mL) added to the residue and the mixture filtered. The filter cake was dried under vacuum to obtain the crude product. The crude product was washed with petroleum ether (5 mL) and dried under vacuum to afford N-(2-chloro-3-(4,4,5,5-tetramethyl-1,3,2-dioxaborolan-2-yl)phenyl)-1,3-dimethyl-2,4-dioxo-1,2,3,4-tetrahydropyrimidine-5-carboxamide (2.8 g, 73% yield) as a white solid. MS: m/z found 420 [M+H]⁺.

### (c) N-(2-chloro-3-(2,3-dichloropyridin-4-yl)phenyl)-1,3-dimethyl-2,4-dioxo-1,2,3,4-tetrahydropyrimidine-5-carboxamide

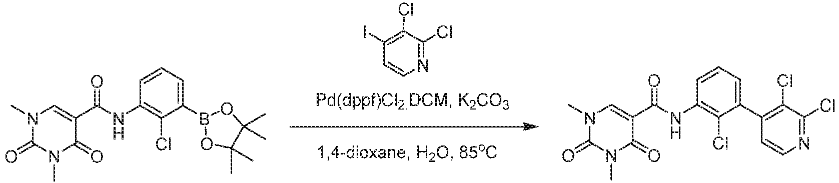

To a mixture of N-(2-chloro-3-(4,4,5,5-tetramethyl-1,3,2-dioxaborolan-2-yl)phenyl)-1,3-dimethyl-2,4-dioxo-1,2,3,4-tetrahydropyrimidine-5-carboxamide (0.6 g, 1.43 mmol) and 2,3-dichloro-4-iodopyridine (0.39 g, 1.43 mmol) in 1,4-dioxane/water mixture (6:1, 12.5 mL) was added [1,1'- bis(diphenylphosphino)ferrocene] dichloropalladium(II) complex with dichloromethane (0.12 g, 0.14 mol) and potassium carbonate (0.6 g, 4.29 mmol) in one portion under N₂ and the mixture was stirred at 85 °C for 3 hr. The mixture was concentrated and the residue was purified directly by column chromatography to afford N-(2-chloro-3-(2,3-dichloropyridin-4-yl)phenyl)-1,3-dimethyl-2,4-dioxo-1,2,3,4-tetrahydropyrimidine-5-carboxamide (0.6 g, 66% yield) as a yellow solid. MS: m/z found 439 and 441 [M+H]⁺.

### (d) N-(2-Chloro-3-(3-chloro-5'-formyl-6'-methoxy-[2,2'-bipyridin]-4-yl)phenyl)-1,3-dimethyl-2,4-dioxo-1,2,3,4-tetrahydropyrimidine-5-carboxamide

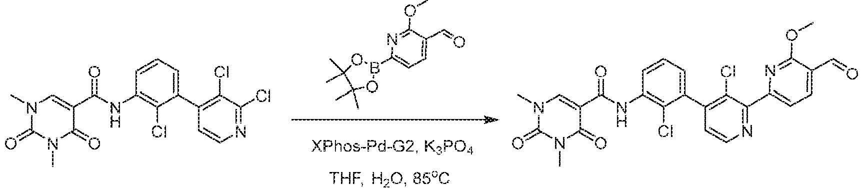

To a mixture of N-(2-chloro-3-(2,3-dichloropyridin-4-yl)phenyl)-1,3-dimethyl-2,4-dioxo-1,2,3,4-tetrahydropyrimidine-5-carboxamide (0.28 g, 0.64 mmol) and 2-methoxy-6-(4,4,5,5-tetramethyl-1,3,2-dioxaborolan-2-yl)nicotinaldehyde (Example 376, step (a)) (0.57 g, 2.17 mmol) in THF/water (5:1, 17 mL) was added chloro(2-dicyclohexylphosphino-2',4',6'-triisopropyl-1,1'-biphenyl)[2-(2'-amino-1,1'-biphenyl)]palladium(II) (0.03 g, 0.03 mmol) and potassium phosphate (0.41 g, 1.91 mmol) in one portion under N₂ and the mixture was stirred at 85 °C for 3 hr. The mixture was concentrated and the residue was purified directly by column chromatography to afford N-(2-chloro-3-(3-chloro-5'-formyl-6'-methoxy-[2,2'-bipyridin]-4-yl)phenyl)-1,3-dimethyl-2,4-dioxo-1,2,3,4-tetrahydropyrimidine-5-carboxamide (0.22 g, 25% yield) as a white solid. ¹H NMR (400 MHz, DMSO-*d*₆): δ 11.68 (s, 1H), 10.33 (s, 1H), 8.83 (s, 1H), 8.79 (d, 1H), 8.64 (d, 1H), 8.30 (d, 1H), 7.66-7.63 (m, 2H), 7.56 (t, 1H), 7.24 (d, 1H), 4.07 (s, 3H), 3.53 (s, 3H), 3.29 (s, 3H).

### (e) (S)-N-(2-Chloro-3-(3-chloro-6'-methoxy-5'-((((5-oxopyrrolidin-2-yl)methyl)amino)methyl)-[2,2'-bipyridin]-4-yl)phenyl)-1,3-dimethyl-2,4-dioxo-1,2,3,4-tetrahydropyrimidine-5-carboxamide

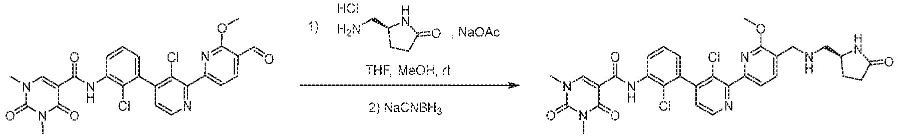

To a mixture of N-(2-chloro-3-(3-chloro-5'-formyl-6'-methoxy-[2,2'-bipyridin]-4-yl)phenyl)-1,3-dimethyl-2,4-dioxo-1,2,3,4-tetrahydropyrimidine-5-carboxamide (0.1 g, 0.19 mmol) and (S)-5-(aminomethyl)pyrrolidin-2-one hydrochloride salt (0.03 g, 0.2 mmol) in THF/MeOH (1:1, 3 mL) was added sodium acetate (0.05 g, 0.6 mmol) in one portion under N₂ and the mixture was stirred at room temperature for 1 hr. Sodium cyanoborohydride (0.03 g, 0.6 mmol) was then added and the mixture stirred at room temperature for 0.5 hr. The mixture was concentrated and the residue was purified by reverse phase HPLC to afford (S)-N-(2-chloro-3-(3-chloro-6'-methoxy-5'-((((5-oxopyrrolidin-2-yl)methyl)amino)methyl)-[2,2'-bipyridin]-4-yl)phenyl)-1,3-dimethyl-2,4-dioxo-1,2,3,4-tetrahydropyrimidine-5-carboxamide (17.3 mg, 14% yield) as a white solid. MS: m/z found 638 [M+H]⁺, retention time = 3.13 min (Method A). ¹H NMR (400 MHz, Methanol-*d*₄): δ 8.70 (s, 1H), 8.67-8.62 (m, 2H), 7.84 (d, 1H), 7.51-7.47 (m, 2H), 7.38 (d, 1H), 7.16 (d, 1H), 4.05 (s, 3H), 3.88-3.83 (m, 3H), 3.58 (s, 3H), 3.41 (s, 3H), 2.75-2.70 (m, 2H), 2.38-2.29 (m, 3H), 1.90-1.77 (m, 1H).

### Example 736: (S)-N-(2-Chloro-3-(3-chloro-5'-(((2-hydroxypropyl)amino)methyl)-6'-methoxy-[2,2'-bipyridin]-4-yl)phenyl)-1,3-dimethyl-2,4-dioxo-1,2,3,4-tetrahydropyrimidine-5-carboxamide (739)

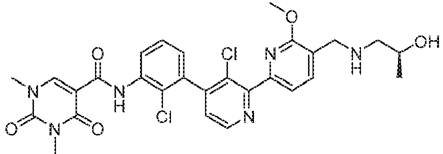

(S)-N-(2-Chloro-3-(3-chloro-5'-(((2-hydroxypropyl)amino)methyl)-6'-methoxy-[2,2'-bipyridin]-4-yl)phenyl)-1,3-dimethyl-2,4-dioxo-1,2,3,4-tetrahydropyrimidine-5-carboxamide was prepared in a similar fashion to Example 735, replacing (S)-5-(aminomethyl)pyrrolidin-2-one hydrochloride salt with (S)-1-aminopropan-2-ol in step (e). MS: m/z found 599 [M+H]⁺; retention time = 3.24 min (Method A). ¹H NMR (400 MHz, Methanol-*d*₄): δ 8.69 (s, 1H), 8.65 (d, 1H), 8.62 (dd, 1H), 7.83 (d, 1H), 7.50-7.46 (m, 2H), 7.39 (d, 1H), 7.16 (dd, 1H), 4.05 (s, 3H), 3.94-3.82 (m, 3H), 3.58 (s, 3H), 3.41 (s, 3H), 2.68-2.58 (m, 2H), 1.19 (d, 3H).

### Example 737: (S)-N-(2-Chloro-3-(4'-chloro-6-methoxy-5-((((5-oxopyrrolidin-2-yl)methyl)amino)methyl)-[2,3'-bipyridin]-5'-yl)phenyl)-1,3-dimethyl-2,4-dioxo-1,2,3,4-tetrahydropyrimidine-5-carboxamide (712)

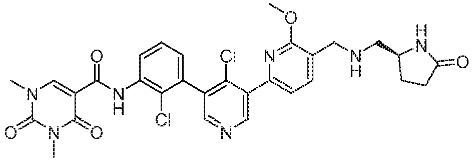

### (a) 5'-Bromo-4'-chloro-6-methoxy-[2,3'-bipyridine]-5-carbaldehyde

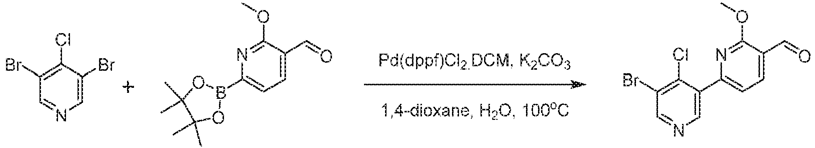

To a mixture of 2-methoxy-6-(4,4,5,5-tetramethyl-1,3,2-dioxaborolan-2-yl)nicotinaldehyde (Example 376, step (a)) (1 g, 3.80 mmol) and 3,5-dibromo-4-chloropyridine (2.58 g, 9.50 mmol) in 1,4-dioxane/ water (5:1, 12 mL) was added potassium carbonate (1.58 g, 11.4 mmol) and [1,1'-bis(diphenylphosphino)ferrocene]dichloropalladium(II) complex with dichloromethane (0.31 g, 0.38 mmol) and the mixture stirred at 100 °C for 2 h under N₂. The mixture was concentrated, water (50 mL) added to the residue and the mixture extracted with ethyl acetate (2 x50 mL). The combined organic phases were dried over anhydrous sulfate, filtered and concentrated under reduced pressure. The residue was purified by normal phase SiO₂ chromatography (0-25 % ethyl acetate / petroleum ether) to afford 5'-bromo-4'-chloro-6-methoxy-[2,3'-bipyridine]-5-carbaldehyde (0.5 g, 41% yield) as a white solid. ¹H NMR (400 MHz, DMSO-*d*₆): δ 10.31 (s, 1H), 8.98 (s, 1H), 8.79 (s, 1H), 8.26 (d, 1H), 7.57 (d, 1H), 4.06 (s, 3H).

### (b) N-(2-Chloro-3-(4'-chloro-5-formyl-6-methoxy-[2,3'-bipyridin]-5'-yl)phenyl)-1,3-dimethyl-2,4-dioxo-1,2,3,4-tetrahydropyrimidine-5-carboxamide

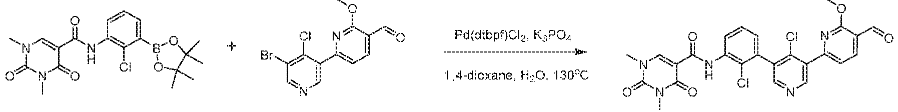

To a mixture of 5'-bromo-4'-chloro-6-methoxy-[2,3'-bipyridine]-5-carbaldehyde (120 mg, 0.37 mmol) and N-(2-chloro-3-(4,4,5,5-tetramethyl-1,3,2-dioxaborolan-2-yl)phenyl)-1,3-dimethyl-2,4-dioxo-1,2,3,4-tetrahydropyrimidine-5-carboxamide (Example 735, step (b)) (200 mg, 0.48 mmol) in 1,4-dioxane/ water (5:1, 6 mL) was added potassium phosphate (233 mg, 1.10 mmol) and [1,1'-bis(di-tert-butylphosphino)ferrocene]dichloropalladium(II) (23.9 mg, 0.04 mmol) and the mixture stirred at 130 °C for 3 h under N₂. The mixture was combined with another batch at the 0.3 g scale. The mixture was concentrated, water (10 mL) added to the residue and the mixture extracted with ethyl acetate (2 x30 mL). The combined organic phases were dried over anhydrous sulfate, filtered and concentrated under reduced pressure. The residue was purified by normal phase SiO₂ chromatography (0-25 % ethyl acetate / petroleum ether) to afford N-(2-chloro-3-(4'-chloro-5-formyl-6-methoxy-[2,3'-bipyridin]-5'-yl)phenyl)-1,3-dimethyl-2,4-dioxo-1,2,3,4-tetrahydropyrimidine-5-carboxamide (500 mg, crude) as a yellow solid. ¹H NMR (400 MHz, DMSO-*d*₆): δ 11.66 (s, 1H), 10.32 (s, 1H), 8.94 (s, 1H), 8.81 (s, 1H), 8.66-8.63 (m, 2H), 8.27 (d, 1H), 7.62 (d, 1H), 7.54 (t, 1H), 7.27 (d, 1H), 4.10 (s, 3H), 3.51 (s, 3H), 3.28 (s, 3H).

### (c) (S)-N-(2-Chloro-3-(4'-chloro-6-methoxy-5-((((5-oxopyrrolidin-2-yl)methyl)amino)methyl)-[2,3'-bipyridin]-5'-yl)phenyl)-1,3-dimethyl-2,4-dioxo-1,2,3,4-tetrahydropyrimidine-5-carboxamide

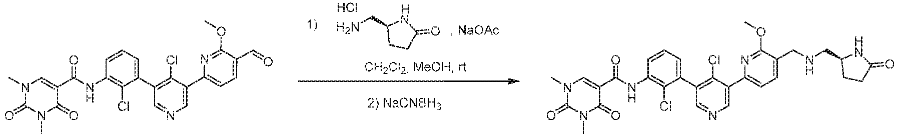

To a mixture of N-(2-chloro-3-(4'-chloro-5-formyl-6-methoxy-[2,3'-bipyridin]-5'-yl)phenyl)-1,3-dimethyl-2,4-dioxo-1,2,3,4-tetrahydropyrimidine-5-carboxamide (200 mg, 0.4 mmol), (S)-5-(aminomethyl)pyrrolidin-2-one hydrochloride salt (167 mg, 1.11 mmol) in dichloromethane/ MeOH (1:1, 10 mL) was added sodium acetate (152 mg, 1.85 mmol) and the mixture stirred at room temperature for 1.5 h under N₂. Sodium cyanoborohydride (69.8 mg, 1.11 mmol) was then added and the mixture stirred at room temperature for 0.5 h under N₂. The mixture was filtered and concentrated and the residue purified by reverse phase HPLC to afford (S)-N-(2-chloro-3-(4'-chloro-6-methoxy-5-((((5-oxopyrrolidin-2-yl)methyl)amino)methyl)-[2,3'-bipyridin]-5'-yl)phenyl)-1,3-dimethyl-2,4-dioxo-1,2,3,4-tetrahydropyrimidine-5-carboxamide (61.5 mg, 25% yield) as a white solid. MS: m/z found 638 [M+H]⁺; retention time = 2.95 min (Method A). ¹H NMR (400 MHz, Methanol-*d*₆): δ 8.81 (s, 1H), 8.69 (s, 1H), 8.64 (dd, 1H), 8.49 (s, 1H), 7.82 (d, 1H), 7.49 (t, 1H), 7.39 (d, 1H), 7.20 (dd, 1H), 4.06 (s, 3H), 3.91-3.83 (m, 3H), 3.58 (s, 3H), 3.41 (s, 3H), 2.77-2.67 (m, 2H), 2.38-2.29 (m, 3H), 1.87-1.82 (m, 1H).

### Example 738: N-(2-Chloro-3-(4'-chloro-6-methoxy-5-((7-oxo-2,6-diazaspiro[3.4]octan-2-yl)methyl)-[2,3'-bipyridin]-5'-yl)phenyl)-1,3-dimethyl-2,4-dioxo-1,2,3,4-tetrahydropyrimidine-5-carboxamide (722)

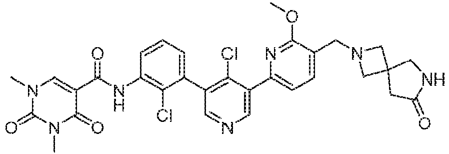

N-(2-Chloro-3-(4'-chloro-6-methoxy-5-((7-oxo-2,6-diazaspiro[3.4]octan-2-yl)methyl)-[2,3'-bipyridin]-5'-yl)phenyl)-1,3-dimethyl-2,4-dioxo-1,2,3,4-tetrahydropyrimidine-5-carboxamide was prepared in a similar fashion to Example 737, replacing (S)-5-(aminomethyl)pyrrolidin-2-one hydrochloride salt with 2,6-diazaspiro[3.4]octan-7-one in step (c). MS: m/z found 650 [M+H]⁺; retention time = 2.95 min (Method A). ¹H NMR (400 MHz, Methanol-*d*₄): δ 8.81 (s, 1H), 8.69 (s, 1H), 8.64 (dd, 1H), 8.49 (s, 1H), 7.77 (d, 1H), 7.49 (t, 1H), 7.39 (d, 1H), 7.20 (dd, 1H), 4.04 (s, 3H), 3.74 (s, 2H), 3.61 (s, 2H), 3.58 (s, 3H), 3.44-3.43 (m, 4H), 3.41 (s, 3H), 2.60 (s, 2H).

### Example 739: N-(3-(5-(((1-Acetylpiperidin-4-yl)amino)methyl)-4'-chloro-6-methoxy-[2,3'-bipyridin]-5'-yl)-2-chlorophenyl)-1,3-dimethyl-2,4-dioxo-1,2,3,4-tetrahydropyrimidine-5-carboxamide (723)

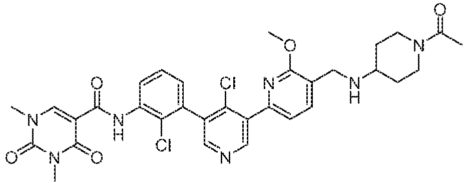

N-(3-(5-(((1-Acetylpiperidin-4-yl)amino)methyl)-4'-chloro-6-methoxy-[2,3'-bipyridin]-5'-yl)-2-chlorophenyl)-1,3-dimethyl-2,4-dioxo-1,2,3,4-tetrahydropyrimidine-5-carboxamide was prepared in a similar fashion to Example 737, replacing (S)-5-(aminomethyl)pyrrolidin-2-one hydrochloride salt with 1-(4-aminopiperidin-1-yl)ethan-1-one in step (c). MS: m/z found 666 [M+H]⁺; retention time = 3.11 min (Method A). ¹H NMR (400 MHz, Methanol-*d*₄): δ 8.82 (s, 1H), 8.69 (s, 1H), 8.65-8.63 (m, 1H), 8.49 (s, 1H), 7.83 (d, 1H), 7.49 (t, 1H), 7.39 (d, 1H), 7.20 (dd, 1H), 4.50-4.47 (m, 1H), 4.07 (s, 3H), 3.97-3.90 (m, 3H), 3.58 (s, 3H), 3.41 (s, 3H), 3.20-3.13 (m, 1H), 2.82-2.72 (m, 2H), 2.12 (s, 3H), 2.08-2.00 (m, 2H), 1.46-1.26 (m, 2H).

### Example 740: (S)-N-(2-Chloro-3-(4'-chloro-5-(((2-hydroxypropyl)amino)methyl)-6-methoxy-[2,3'-bipyridin]-5'-yl)phenyl)-1,3-dimethyl-2,4-dioxo-1,2,3,4-tetrahydropyrimidine-5-carboxamide (729)

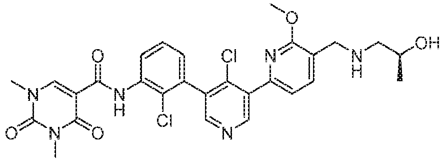

(S)-N-(2-Chloro-3-(4'-chloro-5-(((2-hydroxypropyl)amino)methyl)-6-methoxy-[2,3'-bipyridin]-5'-yl)phenyl)-1,3-dimethyl-2,4-dioxo-1,2,3,4-tetrahydropyrimidine-5-carboxamide was prepared in a similar fashion to Example 737, replacing (S)-5-(aminomethyl)pyrrolidin-2-one hydrochloride salt with (S)-1-aminopropan-2-ol in step (c). MS: m/z found 599 [M+H]⁺; retention time = 3.17 min (Method A). ¹H NMR (400 MHz, Methanol-*d*₄): δ 8.83 (s, 1H), 8.69 (s, 1H), 8.63 (d, 1H), 8.49 (s, 1H), 7.80 (d, 1H), 7.49 (t, 1H), 7.39 (d, 1H), 7.20 (d, 1H), 4.07 (s, 3H), 3.94-3.81 (m, 2H), 3.58 (s, 3H), 3.41 (s, 3H), 2.67-2.57 (m, 2H), 1.33-1.26 (m, 1H), 1.19 (d, 3H).

### Example 741: N-(3-(5-((6-Acetyl-2,6-diazaspiro[3.3]heptan-2-yl)methyl)-4'-chloro-6-methoxy-[2,3'-bipyridin]-5'-yl)-2-chlorophenyl)-1,3-dimethyl-2,4-dioxo-1,2,3,4-tetrahydropyrimidine-5-carboxamide (730)

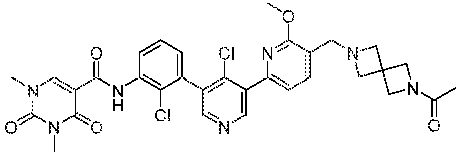

N-(3-(5-((6-Acetyl-2,6-diazaspiro[3.3]heptan-2-yl)methyl)-4'-chloro-6-methoxy-[2,3'-bipyridin]-5'-yl)-2-chlorophenyl)-1,3-dimethyl-2,4-dioxo-1,2,3,4-tetrahydropyrimidine-5-carboxamide was prepared in a similar fashion to Example 737, replacing (S)-5-(aminomethyl)pyrrolidin-2-one hydrochloride salt with 1-(2,6-diazaspiro[3.3]heptan-2-yl)ethan-1-one trifluoroacetate salt in step (c). MS: m/z found 664 [M+H]⁺; retention time = 3.13 min (Method A). ¹H NMR (400 MHz, Methanol-*d*₄): δ 8.80 (s, 1H), 8.69 (s, 1H), 8.64 (d, 1H), 8.49 (s, 1H), 7.75 (d, 1H), 7.49 (t, 1H), 7.38 (d, 1H), 7.20 (d, 1H), 4.32 (s, 2H), 4.07 (s, 2H), 4.04 (s, 3H), 3.71 (s, 2H), 3.58 (s, 3H), 3.56-3.51 (m, 4H), 3.41 (s, 3H), 1.86 (s, 3H).

### Example 742: (S)-N-(2-Chloro-3-(3'-chloro-6-methoxy-5-((((5-oxopyrrolidin-2-yl)methyl)amino)methyl)-[2,4'-bipyridin]-2'-yl)phenyl)-1,3-dimethyl-2,4-dioxo-1,2,3,4-tetrahydropyrimidine-5-carboxamide (718)

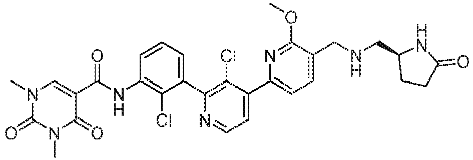

### (a) N-(2-Chloro-3-(3'-chloro-5-formyl-6-methoxy-[2,4'-bipyridin]-2'-yl)phenyl)-1,3-dimethyl-2,4-dioxo-1,2,3,4-tetrahydropyrimidine-5-carboxamide

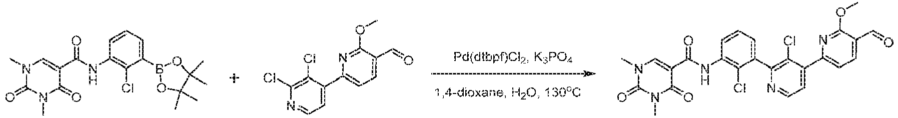

To a mixture of 2',3'-dichloro-6-methoxy-[2,4'-bipyridine]-5-carbaldehyde (Example 640, step (a)) (1 g, 3.53 mmol) and N-(2-chloro-3-(4,4,5,5-tetramethyl-1,3,2-dioxaborolan-2-yl)phenyl)-1,3-dimethyl-2,4-dioxo-1,2,3,4-tetrahydropyrimidine-5-carboxamide (Example 735, step (b)) (1.93 g, 4.59 mmol) in 1,4-dioxane/water (5:1, 36 mL) was added potassium phosphate (2.25 g, 10.6 mmol) and [1,1'-bis(di-tert-butylphosphino)ferrocene] dichloropalladium(II) (230 mg, 0.35 mmol) and the mixture stirred at 130 °C for 2 h under N₂. The mixture was concentrated, water (20 mL) added to the residue and the mixture extracted with ethyl acetae (2 x50 mL). The combined organic phases were dried over anhydrous sulfate, filtered and concentrated under reduced pressure. The residue was purified by normal phase SiO₂ chromatography (0-70 % ethyl acetate / petroleum ether) to afford N-(2-chloro-3-(3'-chloro-5-formyl-6-methoxy-[2,4'-bipyridin]-2'-yl)phenyl)-1,3-dimethyl-2,4-dioxo-1,2,3,4-tetrahydropyrimidine-5-carboxamide (700 mg, 37% yield) as a yellow solid. ¹H NMR (400 MHz, DMSO-*d*₆): δ 11.64 (s, 1H), 10.31 (s, 1H), 8.81-8.79 (m, 2H), 8.62 (dd, 1H), 8.28 (d, 1H), 7.82 (d, 1H), 7.61 (d, 1H), 7.53 (t, 1H), 7.25 (dd, 1H), 4.09 (s, 3H), 3.52 (s, 3H), 3.28 (s, 3H).

### (b) (S)-N-(2-Chloro-3-(3'-chloro-6-methoxy-5-((((5-oxopyrrolidin-2-yl)methyl)amino)methyl)-[2,4'-bipyridin]-2'-yl)phenyl)-1,3-dimethyl-2,4-dioxo-1,2,3,4-tetrahydropyrimidine-5-carboxamide

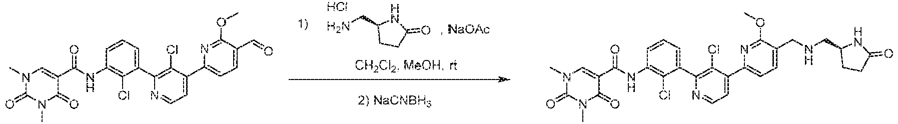

To a mixture of N-(2-chloro-3-(3'-chloro-5-formyl-6-methoxy-[2,4'-bipyridin]-2'-yl)phenyl)-1,3-dimethyl-2,4-dioxo-1,2,3,4-tetrahydropyrimidine-5-carboxamide (100 mg, 185 umol) and (S)-5-(aminomethyl)pyrrolidin-2-one hydrochloride salt (83.6 mg, 555 umol) in dichloromethane / MeOH (1:1, 10 mL) was added sodium acetate (75.9 mg, 925 umol) and the mixture stirred at room temperature for 1.5 h under N₂. Sodium cyanoborohydride (34.9 mg, 555 umol) was added and the mixture stirred at room temperature for 0.5 h under N₂. The mixture was concentrated and the residue purified by reverse phase HPLC to afford (S)-N-(2-chloro-3-(3'-chloro-6-methoxy-5-((((5-oxopyrrolidin-2-yl)methyl)amino)methyl)-[2,4'-bipyridin]-2'-yl)phenyl)-1,3-dimethyl-2,4-dioxo-1,2,3,4-tetrahydropyrimidine-5-carboxamide (27.9 mg, 23% yield) as a white solid. MS: m/z found 638 [M+H]⁺; retention time = 2.89 min (Method A). ¹H NMR (400 MHz, Methanol-*d*₄): δ 8.68 (s, 1H), 8.66-8.64 (m, 2H), 7.83 (d, 1H), 7.79 (d, 1H), 7.49 (t, 1H), 7.44 (d, 1H), 7.22 (dd, 1H), 4.06 (s, 3H), 3.87-3.83 (m, 3H), 3.58 (s, 3H), 3.41 (s, 3H), 2.75-2.68 (m, 2H), 2.38-2.27 (m, 3H), 1.86-1.83 (m, 1H).

### Example 743: N-(2-Chloro-3-(3'-chloro-6-methoxy-5-((7-oxo-2,6-diazaspiro[3.4]octan-2-yl)methyl)-[2,4'-bipyridin]-2'-yl)phenyl)-1,3-dimethyl-2,4-dioxo-1,2,3,4-tetrahydropyrimidine-5-carboxamide (719)

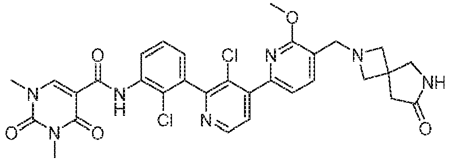

N-(2-Chloro-3-(3'-chloro-6-methoxy-5-((7-oxo-2,6-diazaspiro[3.4]octan-2-yl)methyl)-[2,4'-bipyridin]-2'-yl)phenyl)-1,3-dimethyl-2,4-dioxo-1,2,3,4-tetrahydropyrimidine-5-carboxamide was prepared in a similar fashion to Example 742, replacing (S)-5-(aminomethyl)pyrrolidin-2-one hydrochloride salt with 2,6-diazaspiro[3.4]octan-7-one trifluoroacetate salt in step (b). MS: m/z found 650 [M+H]⁺; retention time = 2.88 min (Method A). ¹H NMR (400 MHz, Methanol-*d*₄): δ 8.69 (s, 1H), 8.66-8.63 (m, 2H), 7.79-7.76 (m, 2H), 7.49 (t, 1H), 7.23 (d, 1H), 7.22 (dd, 1H), 4.04 (s, 3H), 3.73 (s, 2H), 3.60 (s, 2H), 3.58 (s, 3H), 3.45-3.41 (m, 7H), 2.60 (s, 2H).

### Example 744: N-(3-(5-(((1-Acetylpiperidin-4-yl)amino)methyl)-3'-chloro-6-methoxy-[2,4'-bipyridin]-2'-yl)-2-chlorophenyl)-1,3-dimethyl-2,4-dioxo-1,2,3,4-tetrahydropyrimidine-5-carboxamide (721)

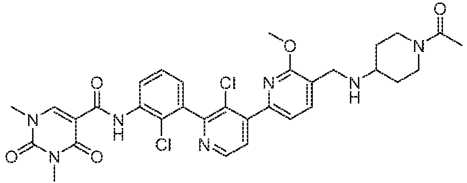

N-(3-(5-(((1-Acetylpiperidin-4-yl)amino)methyl)-3'-chloro-6-methoxy-[2,4'-bipyridin]-2'-yl)-2-chlorophenyl)-1,3-dimethyl-2,4-dioxo-1,2,3,4-tetrahydropyrimidine-5-carboxamide was prepared in a similar fashion to Example 742, replacing (S)-5-(aminomethyl)pyrrolidin-2-one hydrochloride salt with 1-(4-aminopiperidin-1-yl)ethan-1-one hydrochloride salt in step (b). MS: m/z found 666 [M+H]⁺; retention time = 3.03 min (Method A). ¹H NMR (400 MHz, Methanol-*d*₄) : δ 8.69 (s, 1H), 8.66-8.63 (m, 2H), 7.84 (d, 1H), 7.79 (d, 1H), 7.49 (t, 1H), 7.45 (d, 1H), 7.22 (dd, 1H), 4.50-4.46 (m, 1H), 4.07 (s, 3H), 3.94-3.93 (m, 1H), 3.90 (s, 2H), 3.58 (s, 3H), 3.41 (s, 3H), 3.20-3.13 (m, 1H), 2.82-2.71(m, 2H), 2.12 (s, 3H), 2.07-2.00 (m, 2H), 1.44-1.29 (m, 2H).

### Example 745: N-(3-(5-((6-Acetyl-2,6-diazaspiro[3.3]heptan-2-yl)methyl)-3'-chloro-6-methoxy-[2,4'-bipyridin]-2'-yl)-2-chlorophenyl)-1,3-dimethyl-2,4-dioxo-1,2,3,4-tetrahydropyrimidine-5-carboxamide (731)

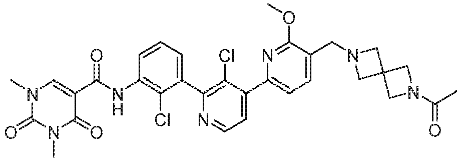

N-(3-(5-((6-Acetyl-2,6-diazaspiro[3.3]heptan-2-yl)methyl)-3'-chloro-6-methoxy-[2,4'-bipyridin]-2'-yl)-2-chlorophenyl)-1,3-dimethyl-2,4-dioxo-1,2,3,4-tetrahydropyrimidine-5-carboxamide was prepared in a similar fashion to Example 742, replacing (S)-5-(aminomethyl)pyrrolidin-2-one hydrochloride salt with 1-(2,6-diazaspiro[3.3]heptan-2-yl)ethan-1-one as trifluoroacetate salt in step (b). MS: m/z found 664 [M+H]⁺; retention time = 3.07 min (Method A). ¹H NMR (400 MHz, Methanol-*d*₄): δ 8.69-8.64 (m, 3H), 7.79-7.75 (m, 2H), 7.49 (t, 1H), 7.43 (d, 1H), 7.22 (d, 1H), 4.32 (s, 2H), 4.11 (s, 2H), 4.04 (s, 3H), 3.68 (s, 2H), 3.58-3.50 (m, 7H), 3.41 (s, 3H), 1.86 (s, 3H).

### Example 746: (S)-N-(2-Chloro-3-(3'-chloro-5-(((2-hydroxypropyl)amino)methyl)-6-methoxy-[2,4'-bipyridin]-2'-yl)phenyl)-1,3-dimethyl-2,4-dioxo-1,2,3,4-tetrahydropyrimidine-5-carboxamide (745)

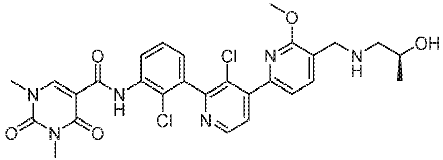

(S)-N-(2-Chloro-3-(3'-chloro-5-(((2-hydroxypropyl)amino)methyl)-6-methoxy-[2,4'-bipyridin]-2'-yl)phenyl)-1,3-dimethyl-2,4-dioxo-1,2,3,4-tetrahydropyrimidine-5-carboxamide was prepared in a similar fashion to Example 742, replacing (S)-5-(aminomethyl)pyrrolidin-2-one hydrochloride salt with (S)-1-aminopropan-2-ol in step (b). MS: m/z found 599 [M+H]⁺; retention time = 3.01 min (Method A). ¹H NMR (400 MHz, Methanol-*d*₄): δ 8.54 (s, 1H), 8.51-8.49 (m, 2H), 7.67-7.65 (m, 2H), 7.35 (t, 1H), 7.30 (d, 1H), 7.07 (dd, 1H), 3.92 (s, 3H), 3.79-3.65 (m, 3H), 3.44 (s, 3H), 3.27 (s, 3H), 2.51-2.41 (m, 2H), 1.04 (d, 3H).

### Example 747: (S)-N-(2-Chloro-3-(5-chloro-6-(3-methoxy-4-((((5-oxopyrrolidin-2-yl)methyl)amino)methyl)phenyl)pyrimidin-4-yl)phenyl)-1,3-dimethyl-2,4-dioxo-1,2,3,4-tetrahydropyrimidine-5-carboxamide (708)

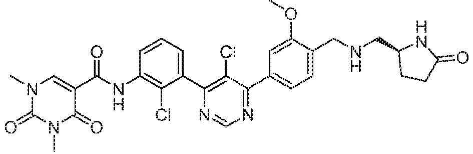

### (a) 4-(5,6-Dichloropyrimidin-4-yl)-2-methoxybenzaldehyde

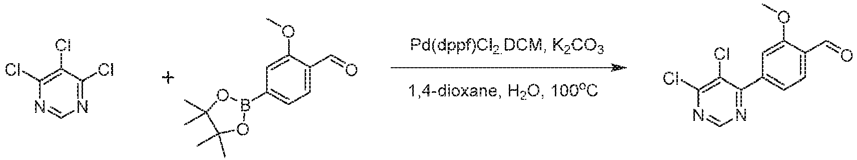

To a mixture of 2-methoxy-4-(4,4,5,5-tetramethyl-1,3,2-dioxaborolan-2-yl)benzaldehyde (Example 714, step (f)) (3 g, 11.5 mmol) and 4,5,6-trichloropyrimidine (4.2 g, 22.9 mmol) in 1,4-dioxane/water mixture (10:1, 77 mL) was added [1,1'-bis(diphenylphosphino)ferrocene]dichloropalladium(II) complex with dichloromethane (0.19 g, 0.23 mmol) and potassium carbonate (3.95 g, 28.6 mmol) in one portion under N₂ and the mixture was stirred at 100 °C for 2 hr. Water (100 mL) was added and the mixture filtered. The filter cake was washed with petroleum ether (5 mL) and dried under vacuum to afford the crude 4-(5,6-dichloropyrimidin-4-yl)-2-methoxybenzaldehyde (3.8 g, 92% yield) as a yellow solid. ¹H NMR (400 MHz, Chloroform-d): δ 10.47 (s, 1H), 8.87 (s, 1H), 7.88 (d, 1H), 7.39 (d, 1H), 7.33 (s, 1H), 3.94 (s, 3H).

### (b) N-(2-Chloro-3-(5-chloro-6-(4-formyl-3-methoxyphenyl)pyrimidin-4-yl)phenyl)-1,3-dimethyl-2,4-dioxo-1,2,3,4-tetrahydropyrimidine-5-carboxamide

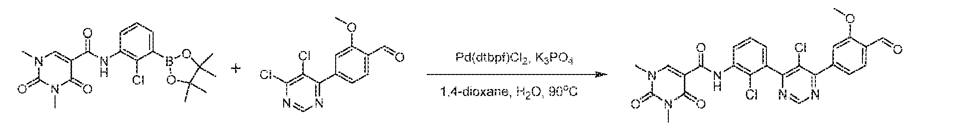

To a mixture of N-(2-chloro-3-(4,4,5,5-tetramethyl-1,3,2-dioxaborolan-2-yl)phenyl)-1,3-dimethyl-2,4-dioxo-1,2,3,4-tetrahydropyrimidine-5-carboxamide (0.41 g, 0.97 mmol) (Example 735, step (b)) and 4-(5,6-dichloropyrimidin-4-yl)-2-methoxybenzaldehyde (0.25 g, 0.88 mmol) in 1,4-dioxane/water mixture (5:1, 6 mL) was added potassium phosphate (0.56 g, 2.65 mmol) and [1,1'-bis(di-tert-butylphosphino)ferrocene]dichloropalladium(II) (0.06 g, 0.09 mmol) in one portion under N₂ and the mixture was stirred at 90 °C for 2 hr. The mixture was concentrated and water (50 mL) and saturated aqueous brine solution (10 mL) added to the residue. The mixture was extracted with ethyl acetate/THF mixture (1:1, 50 mL). The organic phase was dried with anhydrous sodium sulfate, filtered and concentrated. The residue was purified by normal phase SiO₂ chromatography (30-100% ethyl acetate/petroleum ether) to afford N-(2-chloro-3-(5-chloro-6-(4-formyl-3-methoxyphenyl)pyrimidin-4-yl)phenyl)-1,3-dimethyl-2,4-dioxo-1,2,3,4-tetrahydropyrimidine-5-carboxamide (0.1 g, 15% yield) as a white solid. ¹H NMR (400 MHz, DMSO-*d*₆): δ 11.74 (s, 1H), 10.50 (s, 1H), 9.44 (s, 1H), 8.87 (s, 1H), 8.72 (d, 1H), 7.93 (d, 1H), 7.70 (s, 1H), 7.64 (t, 1H), 7.58 (d, 1H), 7.38 (dd, 1H), 4.07 (s, 3H), 3.58 (s, 3H), 3.34 (s, 3H).

### (c) (S)-N-(2-Chloro-3-(5-chloro-6-(3-methoxy-4-((((5-oxopyrrolidin-2-yl)methyl)amino)methyl)phenyl)pyrimidin-4-yl)phenyl)-1,3-dimethyl-2,4-dioxo-1,2,3,4-tetrahydropyrimidine-5-carboxamide

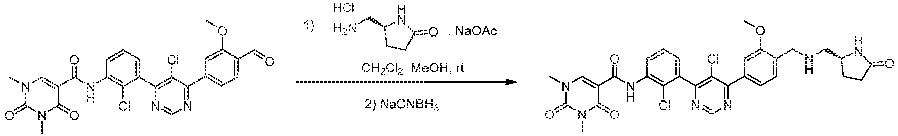

To a mixture of N-(2-chloro-3-(5-chloro-6-(4-formyl-3-methoxyphenyl)pyrimidin-4-yl)phenyl)-1,3-dimethyl-2,4-dioxo-1,2,3,4-tetrahydropyrimidine-5-carboxamide (0.08 g, 0.16 mmol) and (S)-5-(aminomethyl)pyrrolidin-2-one hydrochloride salt (0.03 g, 0.19 mmol) in dichloromethane/MeOH (2:1, 3 mL) was added sodium acetate (0.04 g, 0.47 mmol) in one portion under N₂ and the mixture stirred at room temperature for 11 hr. Sodium cyanoborohydride (0.03 g, 0.47 mmol) was then added and the mixture stirred at room temperature for 1 hr. The mixture was concentrated and the residue was purified by reverse phase HPLC to afford (S)-N-(2-chloro-3-(5-chloro-6-(3-methoxy-4-((((5-oxopyrrolidin-2-yl)methyl)amino)methyl)phenyl)pyrimidin-4-yl)phenyl)-1,3-dimethyl-2,4-dioxo-1,2,3,4-tetrahydropyrimidine-5-carboxamide (20.7 mg, 16% yield) as a white solid. MS: m/z found 638 [M+H]⁺; retention time = 2.89 min (Method A). ¹H NMR (400 MHz, Methanol-*d*₄): δ 9.22 (s, 1H), 8.70-8.69 (m, 2H), 7.55-7.48 (m, 4H), 7.26 (d, 1H), 3.98 (s, 3H), 3.95-3.87 (m, 3H), 3.58 (m, 3H), 3.42 (s, 3H), 2.79-2.70 (m, 2H), 2.38-2.28 (m, 3H), 1.87-1.78 (m, 1H).

### Example 748: (S)-N-(2-Chloro-3-(5-chloro-6-(4-(((2-hydroxypropyl)amino)methyl)-3-methoxyphenyl)pyrimidin-4-yl)phenyl)-1,3-dimethyl-2,4-dioxo-1,2,3,4-tetrahydropyrimidine-5-carboxamide (746)

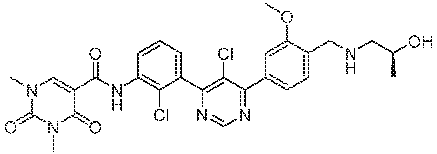

(S)-N-(2-Chloro-3-(5-chloro-6-(4-(((2-hydroxypropyl)amino)methyl)-3-methoxyphenyl)pyrimidin-4-yl)phenyl)-1,3-dimethyl-2,4-dioxo-1,2,3,4-tetrahydropyrimidine-5-carboxamide was prepared in a similar fashion to Example 747, replacing (S)-5-(aminomethyl)pyrrolidin-2-one hydrochloride salt with (S)-1-aminopropan-2-ol in step (c). MS: m/z found 599 [M+H]⁺; retention time = 3.11 min (Method A).

### Example 749: N-((6-(2-chloro-3-(3-chloro-2-(2-((isopropylamino)methyl)-8-methoxy-[1,2,4]triazolo[1,5-a]pyridin-6-yl)pyridin-4-yl)phenyl)-2-methoxypyridin-3-yl)methyl)propan-2-amine (763)

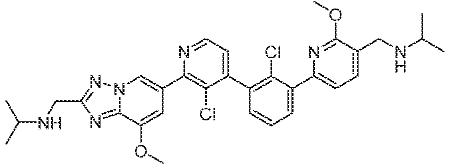

### (a) 1-Amino-5-bromo-3-methoxypyridin-2(1H)-iminium 2,4-dinitrophenolate

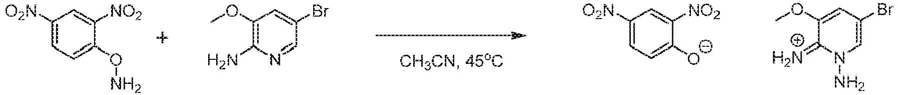

A mixture of O-(2,4-dinitrophenyl)hydroxylamine (3.0 g, 15.1 mmol) and 5-bromo-3-methoxypyridin-2-amine (3.1 g, 15.1 mmol) in acetonitrile (20 mL) was stirred at 45 °C for 12 h under N₂. The mixture was filtered to afford 1-amino-5-bromo-3-methoxypyridin-2(1H)-iminium 2,4-dinitrophenolate (205 mg, 53% yield) as a yellow solid. ¹H NMR (400 MHz, Methanol-*d*₄): δ 8.77 (d, 1H), 8.03-8.00 (m, 1H), 7.84 (d, 1H), 7.47 (d, 1H), 6.67 (d, 1H), 4.04 (s, 3H).

### (b) (6-Bromo-8-methoxy-[1,2,4]triazolo[1,5-a]pyridin-2-yl)methanol

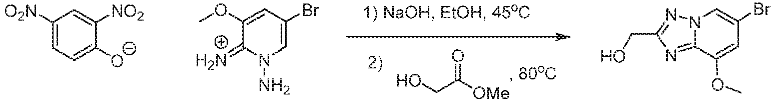

To a mixture of 1-amino-5-bromo-3-methoxypyridin-2(1H)-iminium 2,4-dinitrophenolate (11.5 g, 28.6 mmol) in ethanol (60 mL) was added sodium hydroxide (1.49 g, 37.2 mmol) then the mixture was stirred at 60°C for 1 h under N₂. Methyl 2-hydroxyacetate (2.42 mL, 31.5 mmol) was then added and the mixture stirred at 80°C for 4 h under N₂. The mixture was concentrated and the residue was purified by normal phase SiO₂ chromatography (0-11% ethyl acetate / petroleum ether) to afford (6-bromo-8-methoxy-[1,2,4]triazolo[1,5-a]pyridin-2-yl)methanol (4.8 g, 88% yield) as a yellow solid. ¹H NMR (400 MHz, Methanol-*d*₄): δ 8.49 (d, 1H), 7.11 (d, 1H), 4.68 (s, 2H), 3.96 (s, 3H).

### (c) (2-(Hydroxymethyl)-8-methoxy-[1,2,4]triazolo[1,5-a]pyridin-6-yl)boronic acid

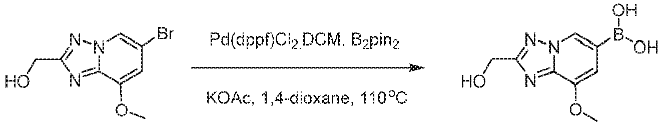

To a mixture of (6-bromo-8-methoxy-[1,2,4]triazolo[1,5-a]pyridin-2-yl)methanol (1.92 g, 7.44 mmol) and bis(pinacolato)diboron (4.72 g, 18.6 mmol) in 1,4-dioxane (25 mL) was added potassium acetate (2.19 g, 22.3 mmol) and [1,1'-bis(diphenylphosphino)ferrocene] dichloropalladium(II) complex with dichloromethane (607.56 mg, 0.744 mmol) and the mixture was stirred at 110 °C for 2 h under N₂. The mixture was concentrated and the residue was purified by normal phase SiO₂ chromatography (100% MeOH/ethyl acetate) to afford (2-(hydroxymethyl)-8-methoxy-[1,2,4]triazolo[1,5-a]pyridin-6-yl)boronic acid (2.1 g, 91% ) as a yellow solid. ¹H NMR (400 MHz, DMSO-*d*₆): δ 8.16 (s, 1H), 7.21 (s, 1H), 4.78 (s, 2H), 4.03 (s, 3H), 1.91 (s, 2H).

### (d) tert-Butyl ((6-(2-chloro-3-(2,3-dichloropyridin-4-yl)phenyl)-2-methoxypyridin-3-yl)methyl)(isopropyl)carbamate

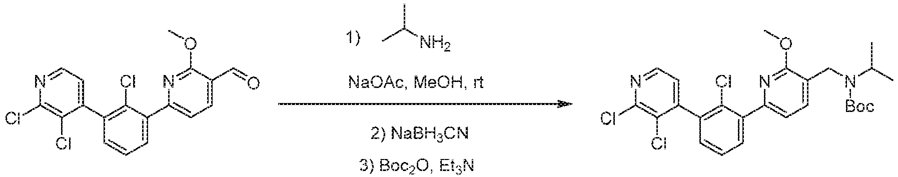

To a mixture of 6-(2-chloro-3-(2,3-dichloropyridin-4-yl)phenyl)-2-methoxynicotinaldehyde (Example 10, step (a)) (2 g, 5.10 mmol) and propan-2-amine (450 mg, 7.62 mmol) in methanol (30 mL) was added sodium acetate (1.25 g, 15.2 mmol) and the mixture stirred at room temperature for 2 h under N₂. Sodium cyanoborohydride (3.19 g, 50.2 mmol) was then added and the mixture stirred at room temperature for 0.5 h under N₂ to afford the N-((6-(2-chloro-3-(2,3-dichloropyridin-4-yl)phenyl)-2-methoxypyridin-3-yl)methyl)propan-2-amine (MS: m/z found 436 [M+H]⁺). Di-tert-butyl dicarbonate (3.70 mL, 16.0 mmol) and triethylamine (2.55 mL, 18.3 mmol) were then added and the mixture was stirred at room temperature for 2 h under N₂. Water (20 mL) was added and the mixture extracted with ethyl acetate (2 x 20 mL). The combined organic phases were dried over anhydrous sodium sulfate, filtered and concentrated under reduced pressure. The residue was purified by normal phase SiO₂ chromatography (0-16% ethyl acetate /petroleum ether) to afford tert-butyl ((6-(2-chloro-3-(2,3-dichloropyridin-4-yl)phenyl)-2-methoxypyridin-3-yl)methyl)(isopropyl)carbamate (2.1 g, 85% yield) as a white solid. MS: m/z found 536 [M+H]⁺.

### (e) tert-Butyl ((6-(2-chloro-3-(3-chloro-2-(2-(hydroxymethyl)-8-methoxy-[1,2,4]triazolo[1,5-a]pyridin-6-yl)pyridin-4-yl)phenyl)-2-methoxypyridin-3-yl)methyl)(isopropyl)carbamate

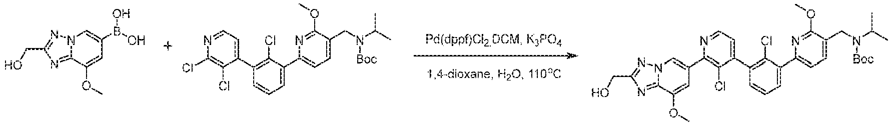

To a mixture of tert-butyl ((6-(2-chloro-3-(2,3-dichloropyridin-4-yl)phenyl)-2-methoxypyridin-3-yl)methyl)(isopropyl)carbamate (1 g, 1.86 mmol) and (2-(hydroxymethyl)-8-methoxy-[1,2,4]triazolo[1,5-a]pyridin-6-yl)boronic acid (1.66 g, 7.45 mmol) in 1,4-dioxane / water (5:1, 18 mL) was added [1,1'- bis(diphenylphosphino)ferrocene]dichloropalladium(II) complex with dichloromethane (121 mg, 0.19 umol) and potassium phosphate (1.20 g, 5.60 mmol) and the mixture stirred at 110 °C for 3 h under N₂. The mixture was concentrated and the residue was purified by normal phase SiO₂ chromatography (100% methanol / ethyl acetate) to afford tert-butyl ((6-(2-chloro-3-(3-chloro-2-(2-(hydroxymethyl)-8-methoxy-[1,2,4]triazolo[1,5-a]pyridin-6-yl)pyridin-4-yl)phenyl)-2-methoxypyridin-3-yl)methyl)(isopropyl)carbamate (900 mg, 70% yield) as a white solid. MS: m/z found 679 [M+H]⁺.

### (f) tert-Butyl ((6-(2-chloro-3-(3-chloro-2-(2-formyl-8-methoxy-[1,2,4]triazolo[1,5-a]pyridin-6-yl)pyridin-4-yl)phenyl)-2-methoxypyridin-3-yl)methyl)(isopropyl)carbamate

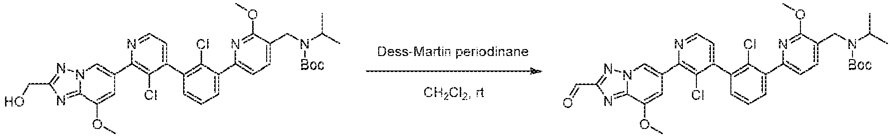

To a mixture of tert-butyl ((6-(2-chloro-3-(3-chloro-2-(2-(hydroxymethyl)-8-methoxy-[1,2,4]triazolo[1,5-a]pyridin-6-yl)pyridin-4-yl)phenyl)-2-methoxypyridin-3-yl)methyl)(isopropyl)carbamate (900 mg, 1.32 mmol) in dichloromethane (5 mL) was added Dess-Martin periodinane (1.00 g, 2.36 mmol) and the mixture was stirred at room temperature for 3 h under N₂. The mixture was concentrated and the residue was purified by normal phase SiO₂ chromatography (50-65% ethyl acetate / petroleum ether) to afford tert-butyl ((6-(2-chloro-3-(3-chloro-2-(2-formyl-8-methoxy-[1,2,4]triazolo[1,5-a]pyridin-6-yl)pyridin-4-yl)phenyl)-2-methoxypyridin-3-yl)methyl)(isopropyl)carbamate (700 mg, 70% yield) as a yellow solid. ¹H NMR (400 MHz, DMSO-*d*₆): δ 10.12 (s, 1H), 9.10 (s, 1H), 8.81 (d, 1H), 8.67 (dd, 1H), 7.75 (dd, 1H), 7.65-7.61 (m, 2H), 7.33-7.30 (m, 2H), 7.21 (d, 1H), 4.27 (s, 2H), 4.06-4.01 (m, 4H), 3.95 (s, 3H), 1.55-1.24 (m, 9H), 1.11 (d, 6H).

### (g) tert-Butyl ((6-(2-chloro-3-(3-chloro-2-(2-((isopropylamino)methyl)-8-methoxy-[1,2,4]triazolo[1,5-a]pyridin-6-yl)pyridin-4-yl)phenyl)-2-methoxypyridin-3-yl)methyl)(isopropyl)carbamate

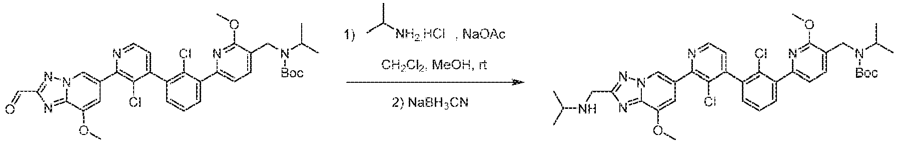

To a mixture of tert-butyl ((6-(2-chloro-3-(3-chloro-2-(2-formyl-8-methoxy-[1,2,4]triazolo[1,5-a]pyridin-6-yl)pyridin-4-yl)phenyl)-2-methoxypyridin-3-yl)methyl)(isopropyl)carbamate (120 mg, 177 umol) and propan-2-amine hydrochloride (33.8 mg, 354 umol) in dichloromethane / methanol (1:1, 4 mL) was added sodium acetate (43.6 mg, 531 umol) and the mixture stirred at room temperature for 2 h under N₂. Sodium cyanoborohydride (33.4 mg, 531 umol) was then added and the mixture stirred at room temperature for 0.5 h under N₂. The mixture was concentrated to afford tert-butyl ((6-(2-chloro-3-(3-chloro-2-(2-((isopropylamino)methyl)-8-methoxy-[1,2,4]triazolo[1,5-a]pyridin-6-yl)pyridin-4-yl)phenyl)-2-methoxypyridin-3-yl)methyl)(isopropyl)carbamate (250 mg, 65% yield) as a white solid. MS: m/z found 720 [M+H]⁺.

### (h) N-((6-(2-Chloro-3-(3-chloro-2-(2-((isopropylamino)methyl)-8-methoxy-[1,2,4]triazolo[1,5-a]pyridin-6-yl)pyridin-4-yl)phenyl)-2-methoxypyridin-3-yl)methyl)propan-2-amine

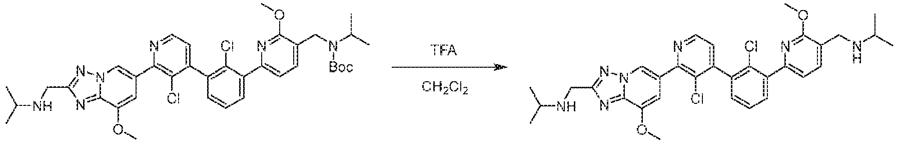

To a mixture of tert-butyl ((6-(2-chloro-3-(3-chloro-2-(2-((isopropylamino)methyl)-8-methoxy-[1,2,4]triazolo[1,5-a]pyridin-6-yl)pyridin-4-yl)phenyl)-2-methoxypyridin-3-yl)methyl)(isopropyl)carbamate (200 mg, 0.28 mmol) in dichloromethane (3 mL) was added trifluoroacetic acid (1.88 mL, 25.4 mmol) and the mixture stirred at room temperature for 0.5 h under N₂. The mixture was concentrated and the residue was purified by reverse phase HPLC to afford N-((6-(2-chloro-3-(3-chloro-2-(2-((isopropylamino)methyl)-8-methoxy-[1,2,4]triazolo[1,5-a]pyridin-6-yl)pyridin-4-yl)phenyl)-2-methoxypyridin-3-yl)methyl)propan-2-amine (1.8 mg, 1% yield) as a white solid. MS: m/z found 620 [M+H]⁺. ¹H NMR (400 MHz, Methanol-*d*₄): δ 8.80 (d, 1H), 8.73 (d, 1H), 7.77-7.73 (m, 2H), 7.60-7.56 (m, 1H), 7.54-7.53 (m, 1H), 7.46-7.44 (m, 2H), 7.27 (d, 1H), 4.14 (s, 3H), 4.09 (s, 2H), 4.05 (s, 3H), 3.85 (s, 2H), 2.95-2.90 (m, 2H), 1.18-1.16 (m, 12H).

### Example 750: 2-((6-(3-Chloro-4-(2-chloro-3-(5-((isopropylamino)methyl)-6-methoxypyridin-2-yl)phenyl)pyridin-2-yl)-8-methoxy-[1,2,4]triazolo[1,5-a]pyridin-2-yl)methyl)-2-azaspiro[3.3]heptan-6-ol (753)

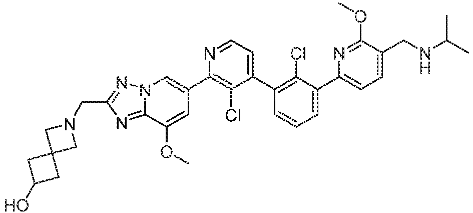

2-((6-(3-Chloro-4-(2-chloro-3-(5-((isopropylamino)methyl)-6-methoxypyridin-2-yl)phenyl)pyridin-2-yl)-8-methoxy-[1,2,4]triazolo[1,5-a]pyridin-2-yl)methyl)-2-azaspiro[3.3]heptan-6-ol was prepared in a similar fashion to Example 749, replacing propan-2-amine hydrochloride with 2-azaspiro[3.3]heptan-6-ol hydrochloride salt in step (g). MS: m/z found 674 [M+H]⁺; retention time = 2.79 min (Method A). ¹H NMR (400 MHz, Methanol-*d*₄): δ 8.80 (d, 1H), 8.73 (d, 1H), 7.78-7.73 (m, 2H), 7.60-7.56 (m, 1H), 7.52 (d, 1H), 7.46-7.43 (m, 2H), 7.27 (d, 1H), 4.18-4.08 (m, 4H), 4.05 (s, 3H), 3.90 (s, 2H), 3.87 (s, 2H), 3.49 (s, 2H), 3.45 (s, 2H), 2.96-2.89 (m, 1H), 2.52-2.47 (m, 2H), 2.06-2.01 (m, 2H), 1.18 (d, 6H).

### Example 751: (1r,4r)-4-(((6-(3-Chloro-4-(2-chloro-3-(5-((isopropylamino)methyl)-6-methoxypyridin-2-yl)phenyl)pyridin-2-yl)-8-methoxy-[1,2,4]triazolo[1,5-a]pyridin-2-yl)methyl)amino)cyclohexanol (758)

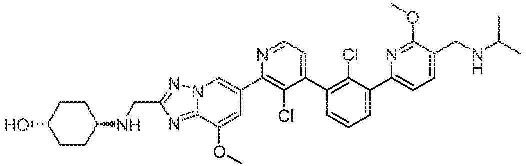

(1r,4r)-4-(((6-(3-Chloro-4-(2-chloro-3-(5-((isopropylamino)methyl)-6-methoxypyridin-2-yl)phenyl)pyridin-2-yl)-8-methoxy-[1,2,4]triazolo[1,5-a]pyridin-2-yl)methyl)amino)cyclohexanol was prepared in a similar fashion to Example 749, replacing propan-2-amine hydrochloride with (1r,4r)-4-aminocyclohexan-1-ol in step (g). MS: m/z found 676 [M+H]⁺; retention time = 2.76 min (Method A). ¹H NMR (400 MHz, Methanol-*d*₄): δ 8.80 (d, 1H), 8.73 (d, 1H), 7.82 (d, 1H), 7.74 (dd, 1H), 7.59 (t, 1H), 7.53 (d, 1H), 7.48-7.45 (m, 2H), 7.32 (d, 1H), 4.14 (s, 3H), 4.11 (s, 2H), 4.07 (s, 3H), 4.02 (s, 2H), 3.56-3.54 (m, 1H), 3.16-3.15 (m, 1H), 2.60-2.56 (m, 1H), 2.07-1.96 (m, 4H), 1.34-1.24 (m, 10H).

### Example 752: (S)-5-((((6-(3-chloro-4-(2-chloro-3-(5-((isopropylamino)methyl)-6-methoxypyridin-2-yl)phenyl)pyridin-2-yl)-8-methoxy-[1,2,4]triazolo[1,5-a]pyridin-2-yl)methyl)amino)methyl)pyrrolidin-2-one (764)

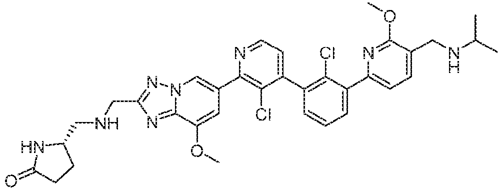

(S)-5-((((6-(3-Chloro-4-(2-chloro-3-(5-((isopropylamino)methyl)-6-methoxypyridin-2-yl)phenyl)pyridin-2-yl)-8-methoxy-[1,2,4]triazolo[1,5-a]pyridin-2-yl)methyl)amino)methyl)pyrrolidin-2-one was prepared in a similar fashion to Example 749, replacing propan-2-amine hydrochloride with (S)-5-(aminomethyl)pyrrolidin-2-one hydrochloride salt in step (g). MS: m/z found 675 [M+H]⁺; retention time = 2.71 min (Method A). ¹H NMR (400 MHz, Methanol-*d*₄): δ 8.68 (d, 1H), 8.62 (d, 1H), 7.78 (d, 1H), 7.63 (dd, 1H), 7.49 (t, 1H), 7.41 (d, 1H), 7.37 (dd, 1H), 7.33 (d, 1H), 7.26 (d, 1H), 4.12 (s, 2H), 4.02 (s, 3H), 3.99-3.94 (m, 5H), 3.75-3.72 (m, 1H), 3.38-3.31 (m, 1H), 2.73-2.62 (m, 2H), 2.26-2.09 (m, 3H), 1.75-1.72 (m, 1H), 1.29 (d, 6H).

### Example 753: 1-(4-(((6-(3-Chloro-4-(2-chloro-3-(5-((isopropylamino)methyl)-6-methoxypyridin-2-yl)phenyl)pyridin-2-yl)-8-methoxy-[1,2,4]triazolo[1,5-a]pyridin-2-yl)methyl)amino)piperidin-1-yl)ethan-1-one (765)

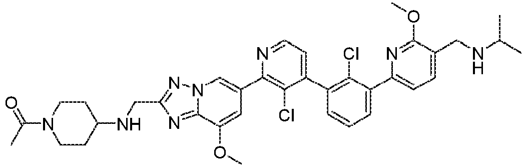

1-(4-(((6-(3-Chloro-4-(2-chloro-3-(5-((isopropylamino)methyl)-6-methoxypyridin-2-yl)phenyl)pyridin-2-yl)-8-methoxy-[1,2,4]triazolo[1,5-a]pyridin-2-yl)methyl)amino)piperidin-1-yl)ethan-1-one was prepared in a similar fashion to Example 749, replacing propan-2-amine hydrochloride with 1-(4-aminopiperidin-1-yl)ethan-1-one hydrochloride salt in step (g). MS: m/z found 703 [M+H]⁺; retention time = 2.78 min (Method A). ¹NMR (400 MHz, Methanol-*d*₄): δ 8.80 (d, 1H), 8.73 (d, 1H), 7.83-7.73 (m, 2H), 7.57 (t, 1H), 7.53 (d, 1H), 7.47-7.45 (m, 2H), 7.28 (d, 1H), 4.47-4.44 (m, 1H), 4.14-4.12 (m, 5H), 4.05 (s, 3H), 3.95-3.87 (m, 3H), 3.16-3.15 (m, 1H), 2.90-2.88 (m, 2H), 2.78-2.75 (m, 1H), 2.11 (s, 3H), 2.08-1.99 (m, 2H), 1.42-1.30 (m, 2H), 1.18 (d, 6H).

### Example 754: 1-(6-((6-(3-Chloro-4-(2-chloro-3-(5-((isopropylamino)methyl)-6-methoxypyridin-2-yl)phenyl)pyridin-2-yl)-8-methoxy-[1,2,4]triazolo[1,5-a]pyridin-2-yl)methyl)-2,6-diazaspiro[3.3]heptan-2-yl)ethan-1-one (767)

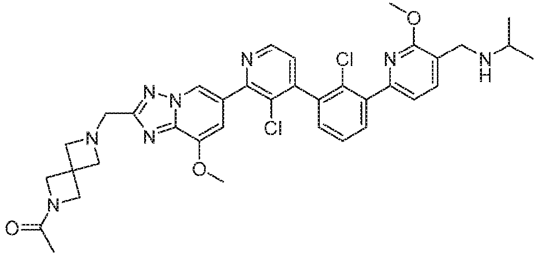

1-(6-((6-(3-Chloro-4-(2-chloro-3-(5-((isopropylamino)methyl)-6-methoxypyridin-2-yl)phenyl)pyridin-2-yl)-8-methoxy-[1,2,4]triazolo[1,5-a]pyridin-2-yl)methyl)-2,6-diazaspiro[3.3]heptan-2-yl)ethan-1-one was prepared in a similar fashion to Example 749, replacing propan-2-amine hydrochloride with 1-(2,6-diazaspiro[3.3]heptan-2-yl)ethan-1-one trifluoroacetate salt in step (g). MS: m/z found 701 [M+H]⁺; retention time = 2.75 min (Method A). ¹H NMR (400 MHz, Methanol-*d*₄): δ 8.75 (d, 1H), 8.69 (d, 1H), 7.73 (d, 1H), 7.70 (dd, 1H), 7.54 (t, 1H), 7.49 (d, 1H), 7.43-7.40 (m, 2H), 7.24 (d, 1H), 4.28 (s, 2H), 4.09 (s, 3H), 4.03 (s, 2H), 4.01 (s, 3H), 3.88 (s, 2H), 3.85 (s, 2H), 3.61-3.56 (m, 4H), 2.95-2.90 (m, 1H), 1.82 (s, 3H), 1.17 (d, 6H).

Compounds **196-205** can be prepared according to the synthetic routes described elsewhere herein and/or methods known to those skilled in the art in view of the teachings provided elsewhere herein.

### Example 755: HTRF Assay

PD-L1 His protein was prepared and added at the final concentration of 6 nM in the White opaque 384 well plate (Corning cat# 3824BC). PD-L1 small molecule inhibitors were diluted by 3-fold starting from 1 µM and a final concentration of 0.00001 µM and added to the well. PD-1 Fc protein was added at the final concentration of 6 nM. PD-L1 His protein, PD-L1 small molecule inhibitors, and PD1 Fc proteins were added to the well in this order, with each 5 µl volume, and were incubated for 15 minutes at room temperature. PAb antiHuman IgG-XL665 (Cisbio, cat# 61HFCXLA) and Mab anti-6HIS Tb cryptate Gold (Cisbio, cat# 61HI2TLA) were mixed at 6.7 nM and 0.35 nM respectively, and total 5 µL volume of mixture was added to the well and incubated at room temperature overnight. The plate was read using PerkinElmer Envision plate reader and data was analyzed by Prism 6 software. Results are illustrated in Table 1.

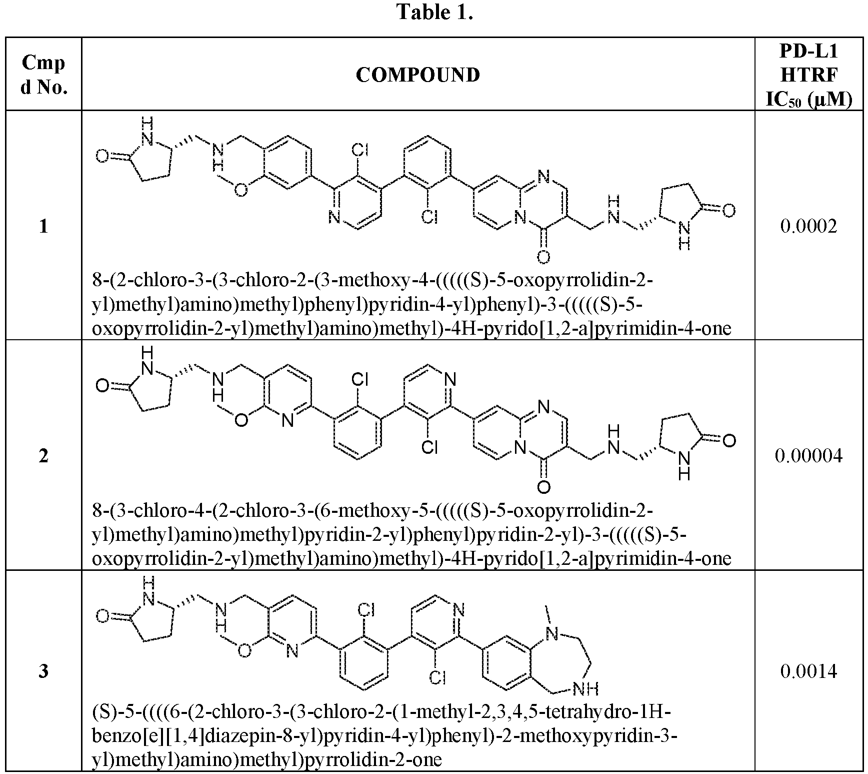

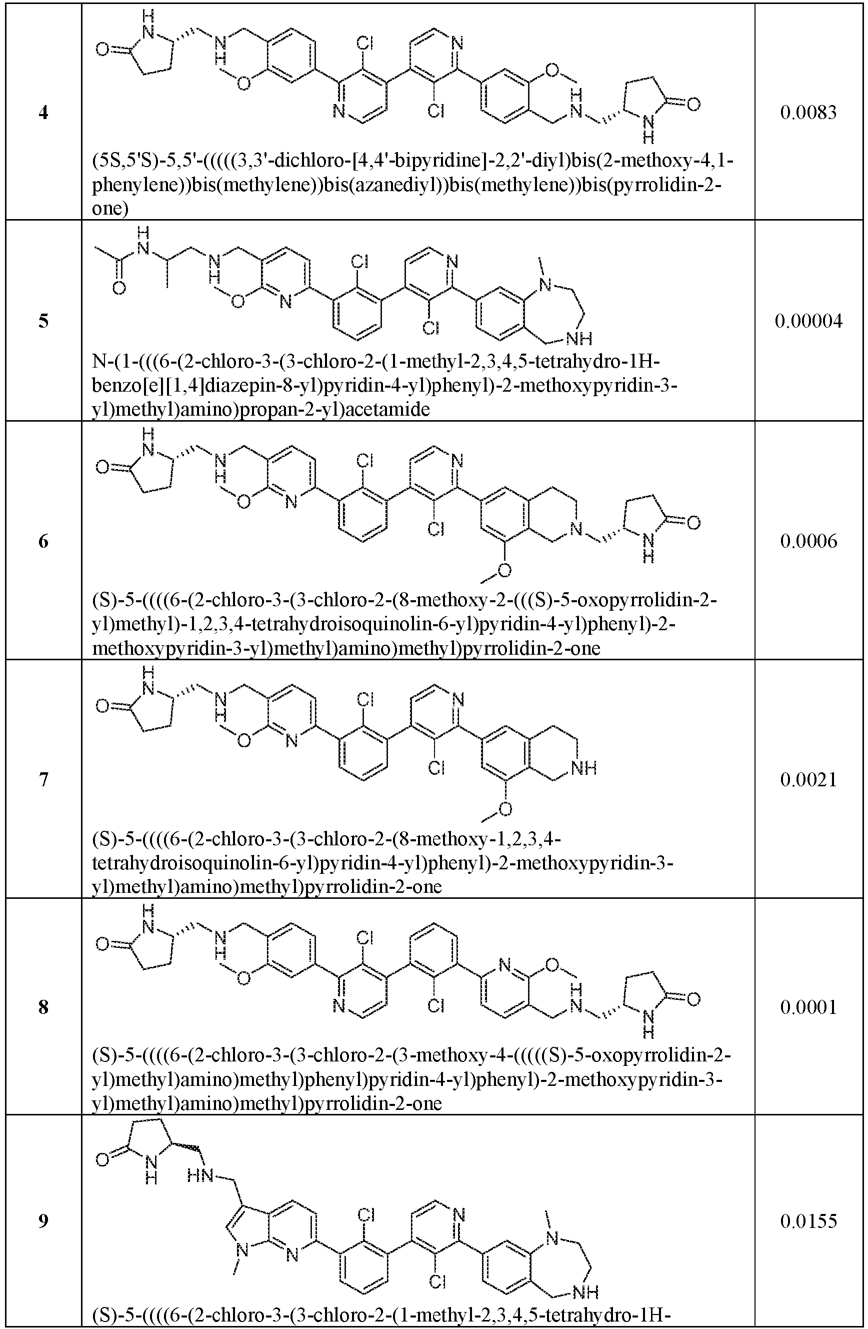

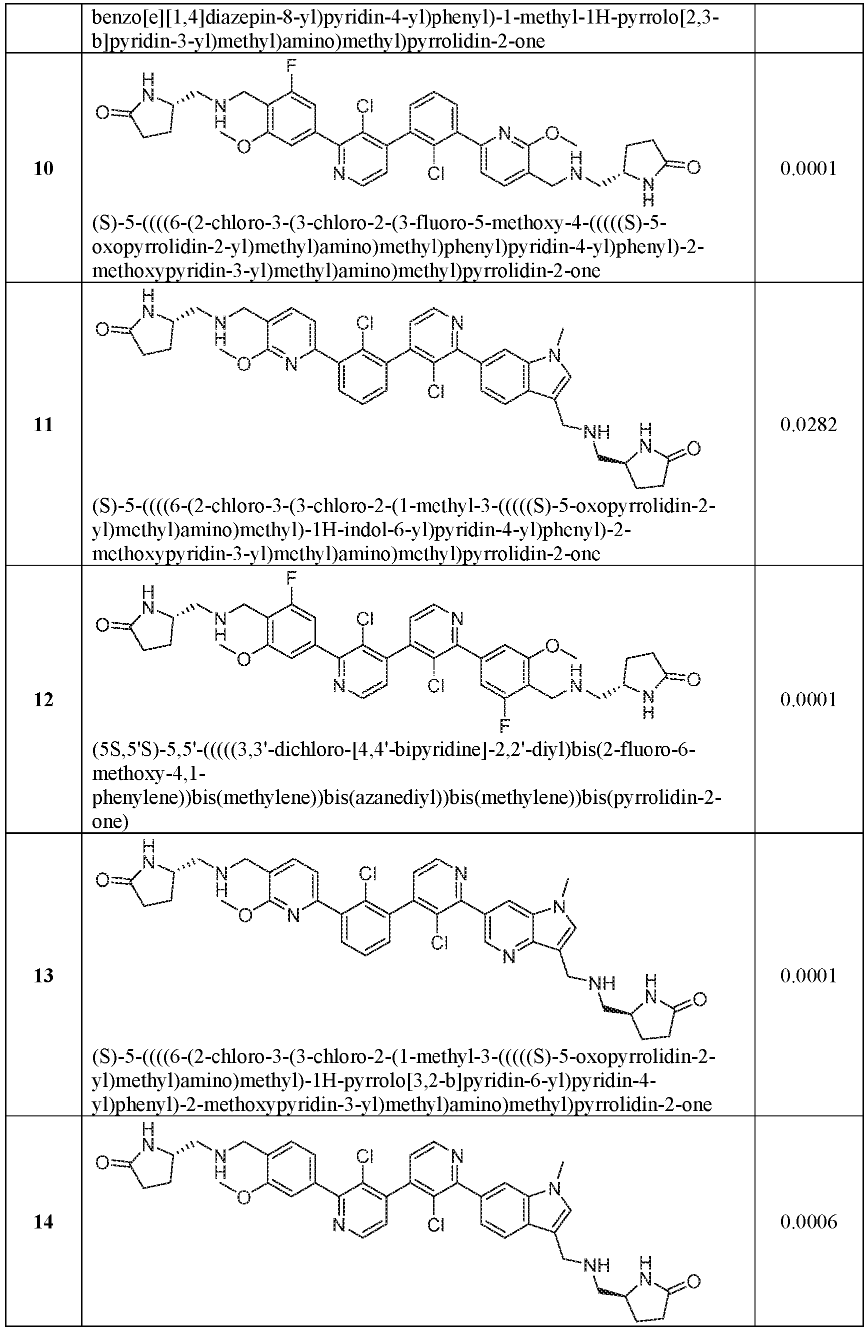

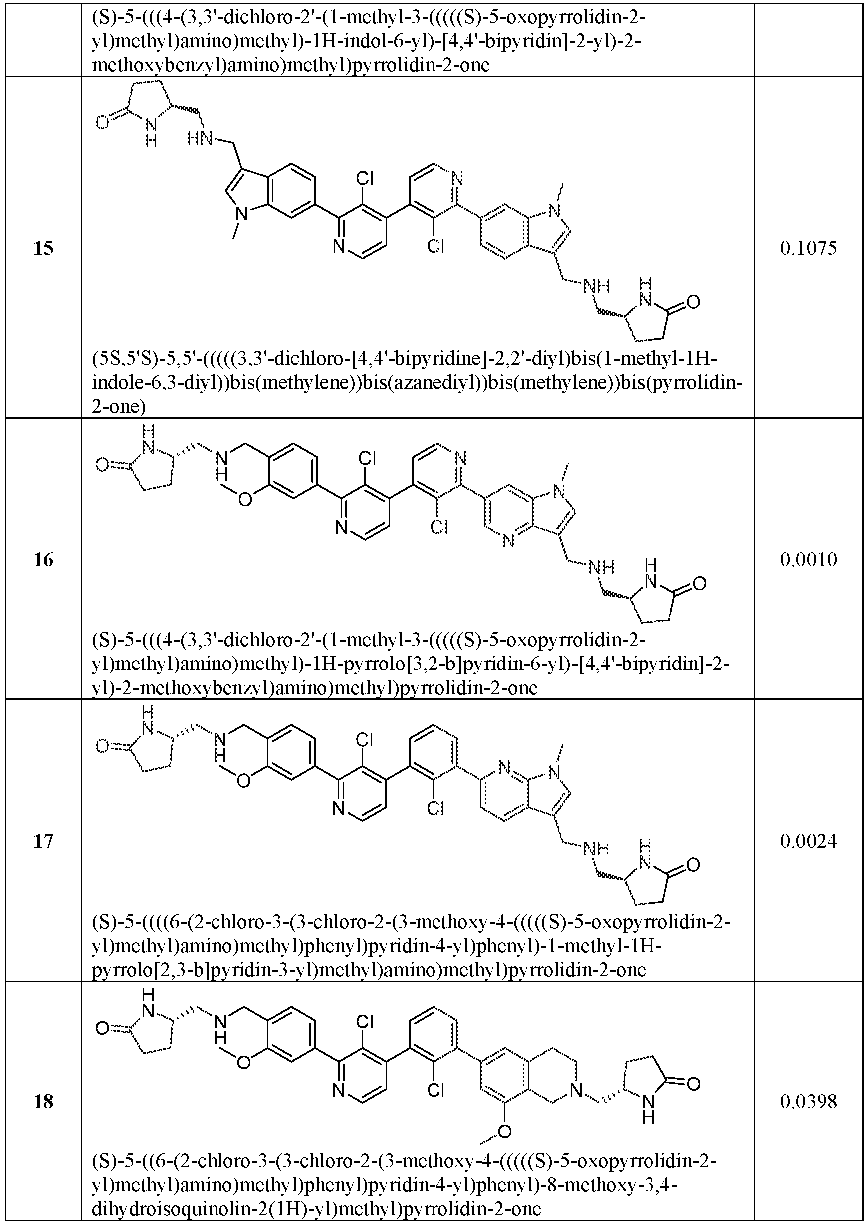

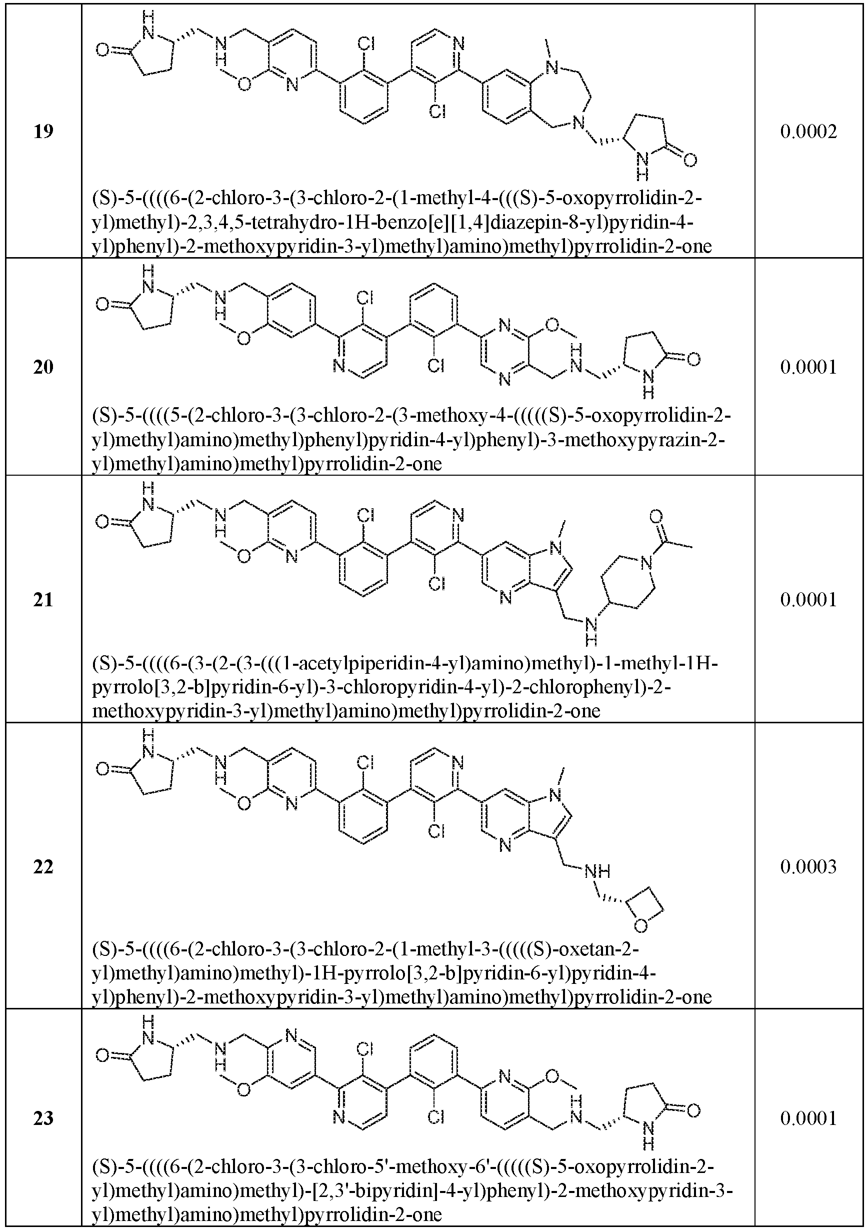

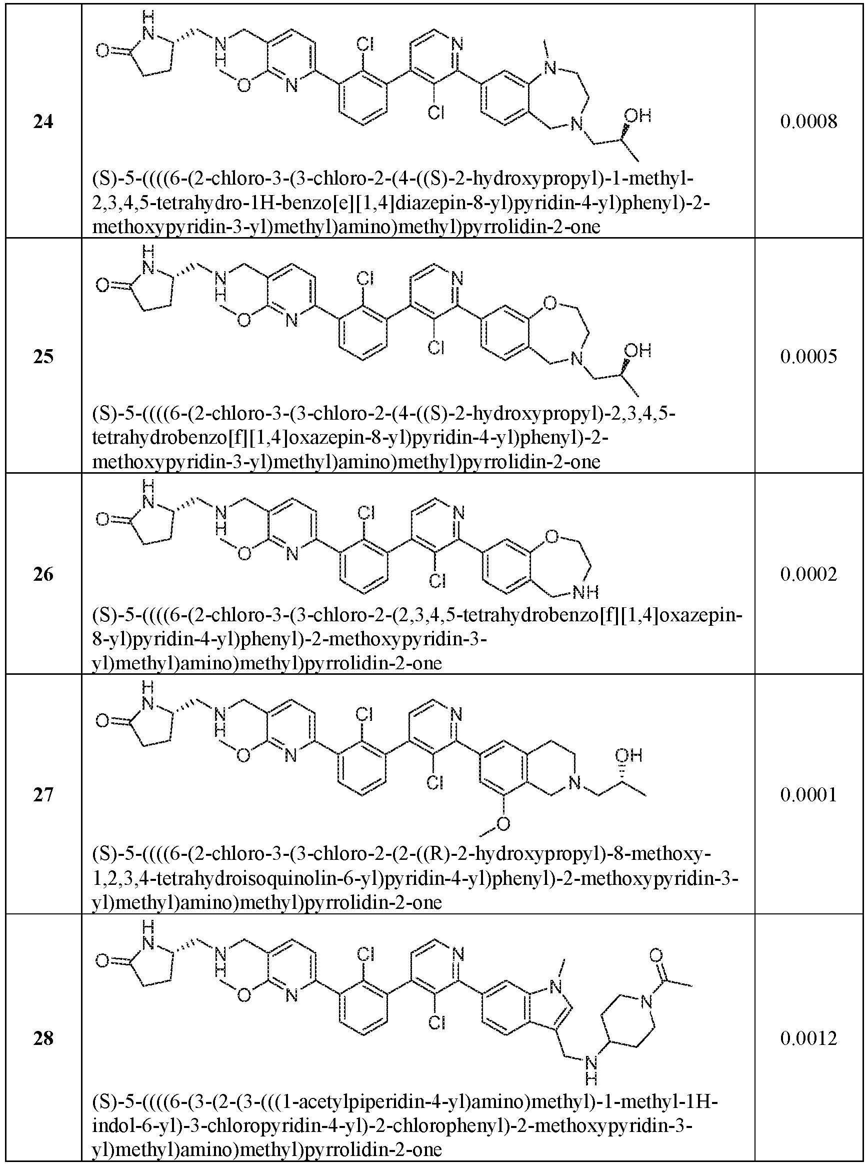

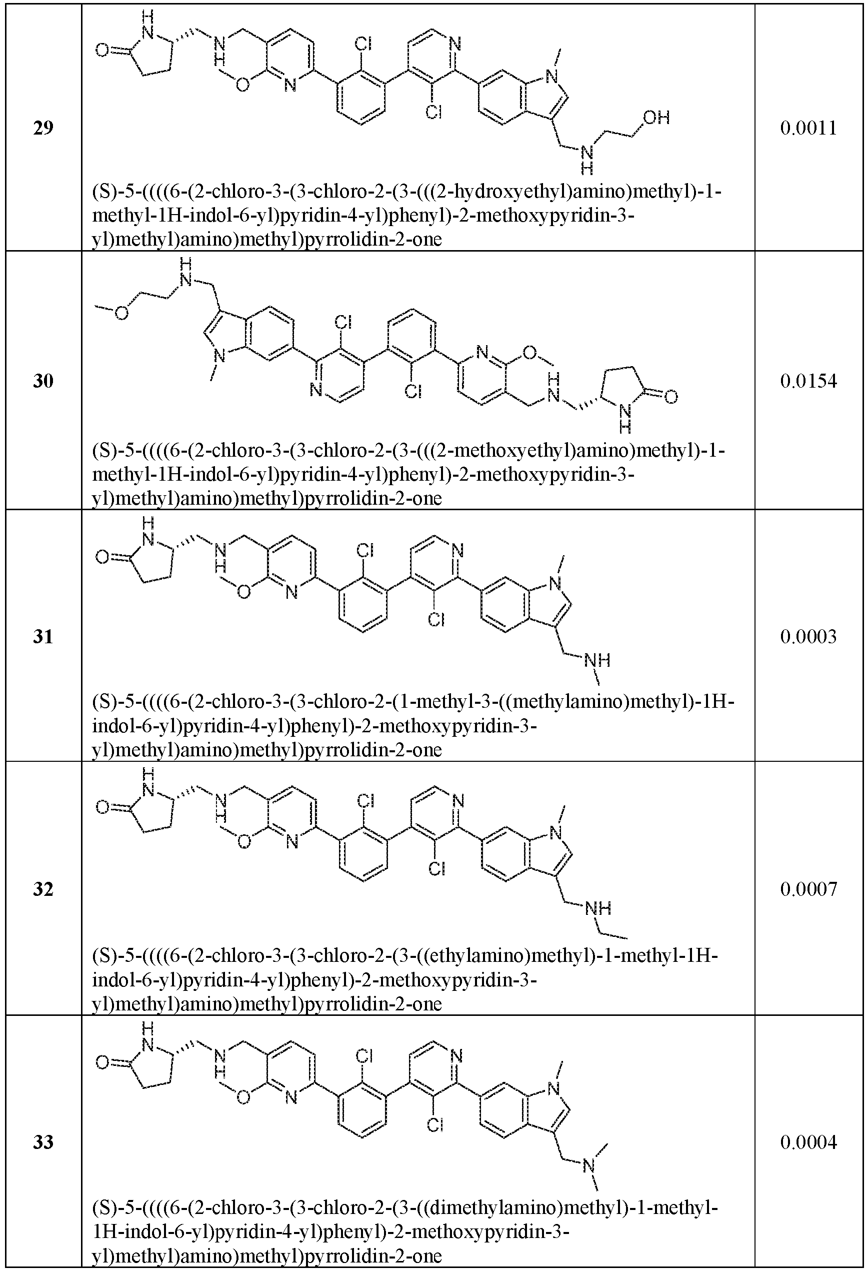

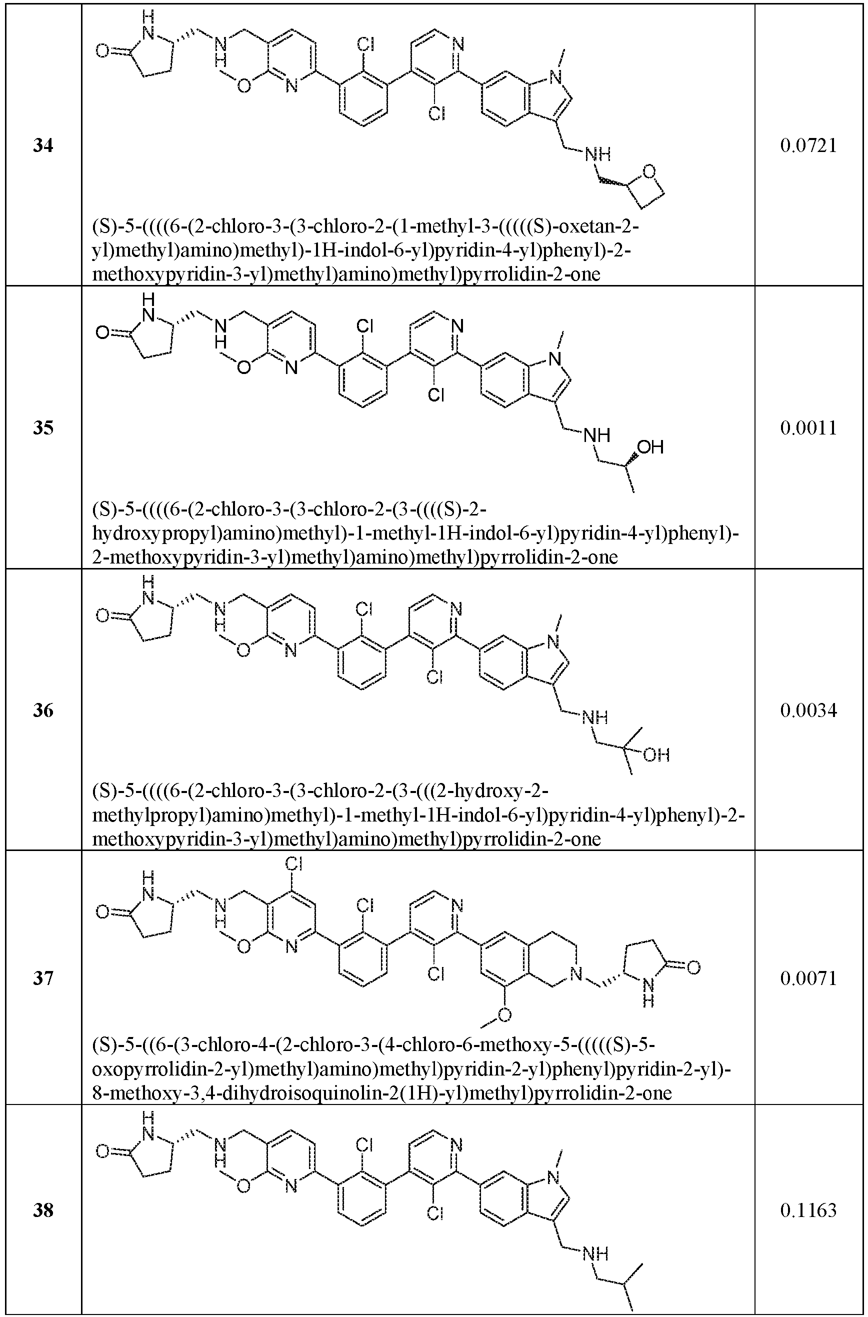

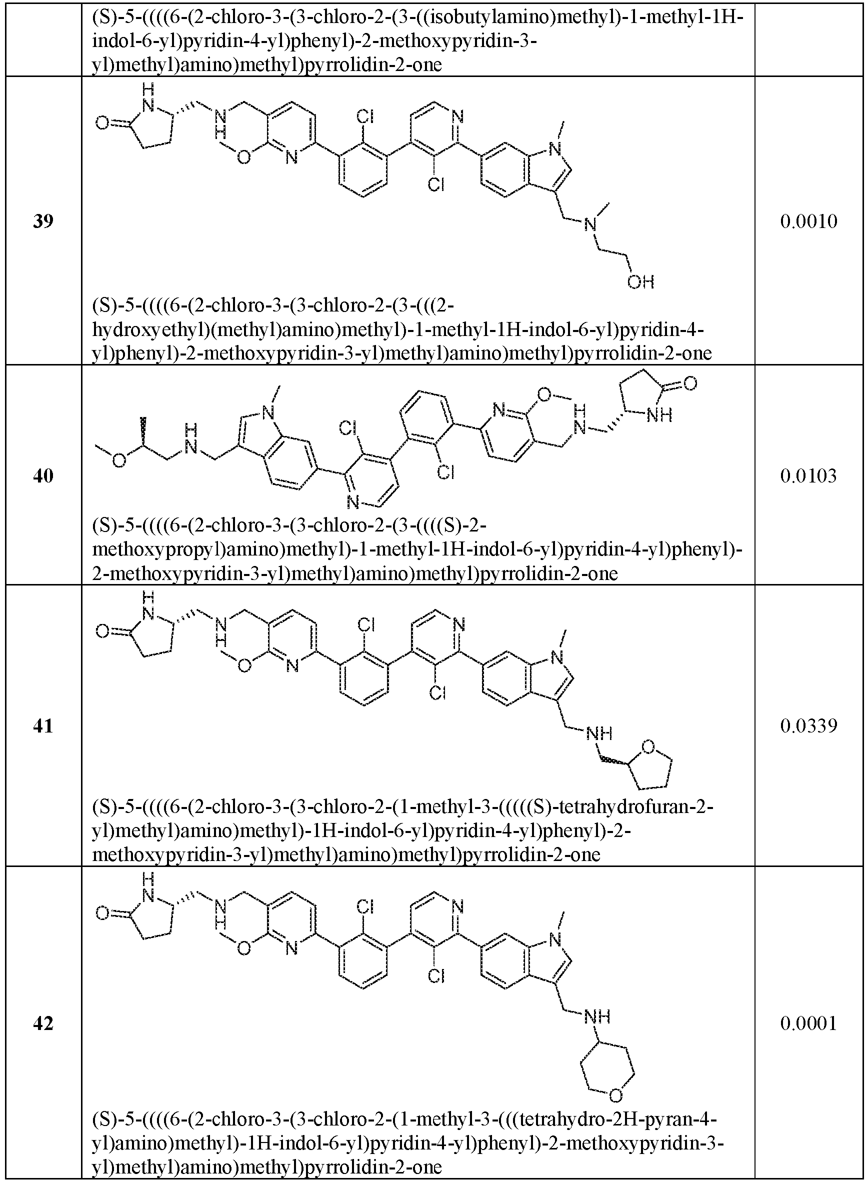

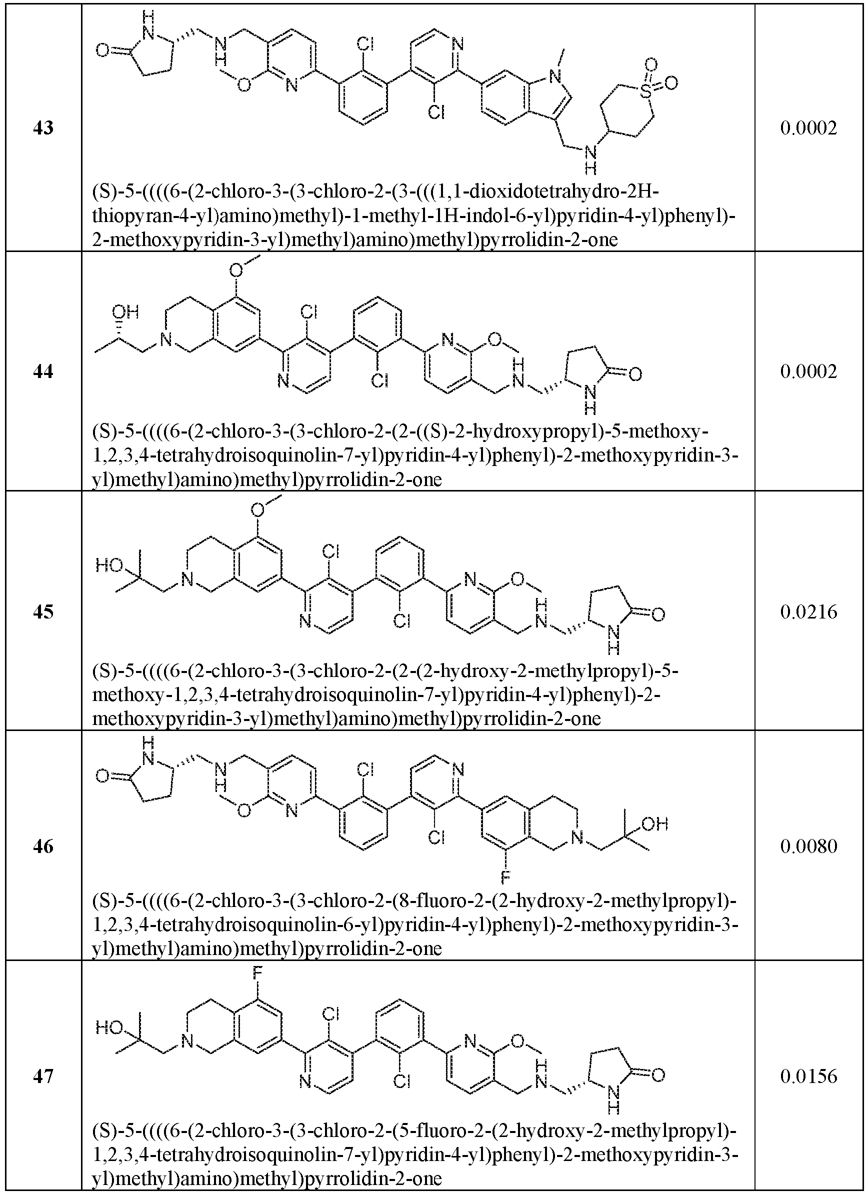

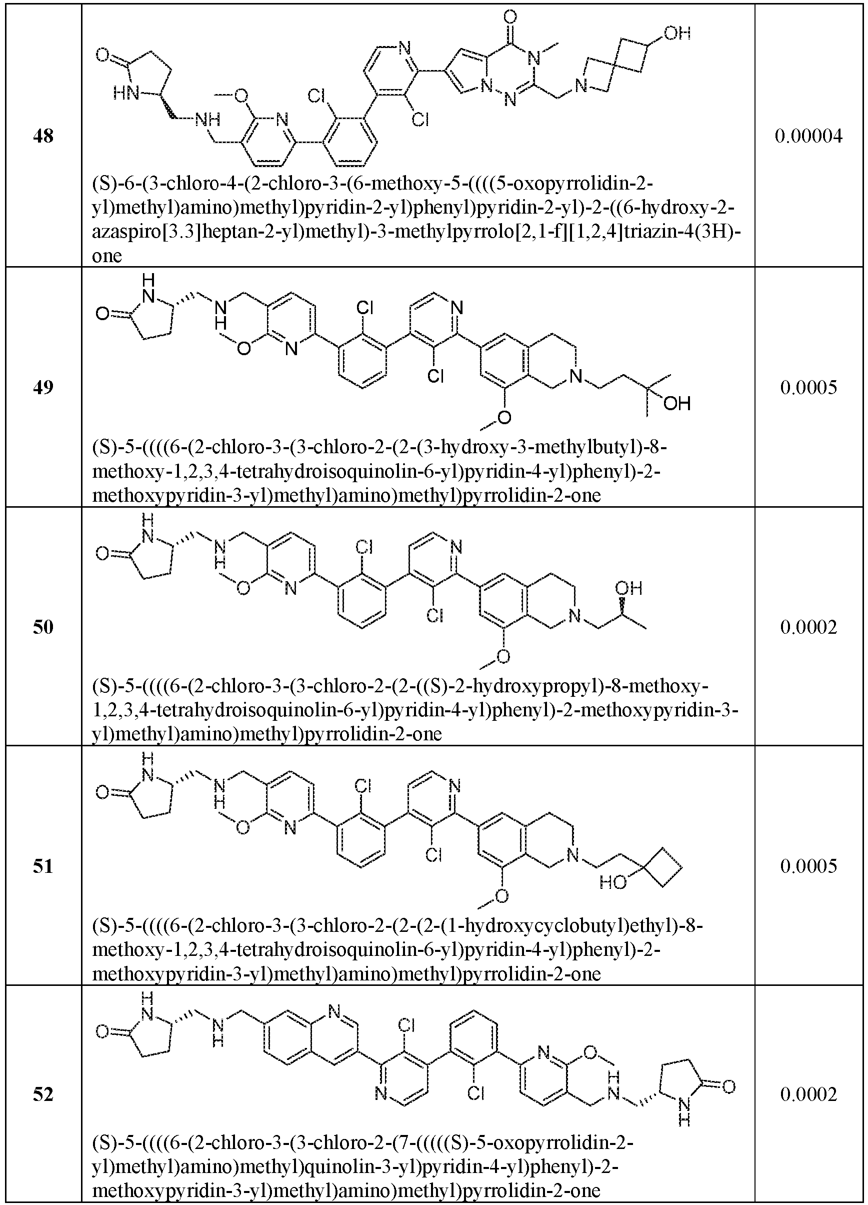

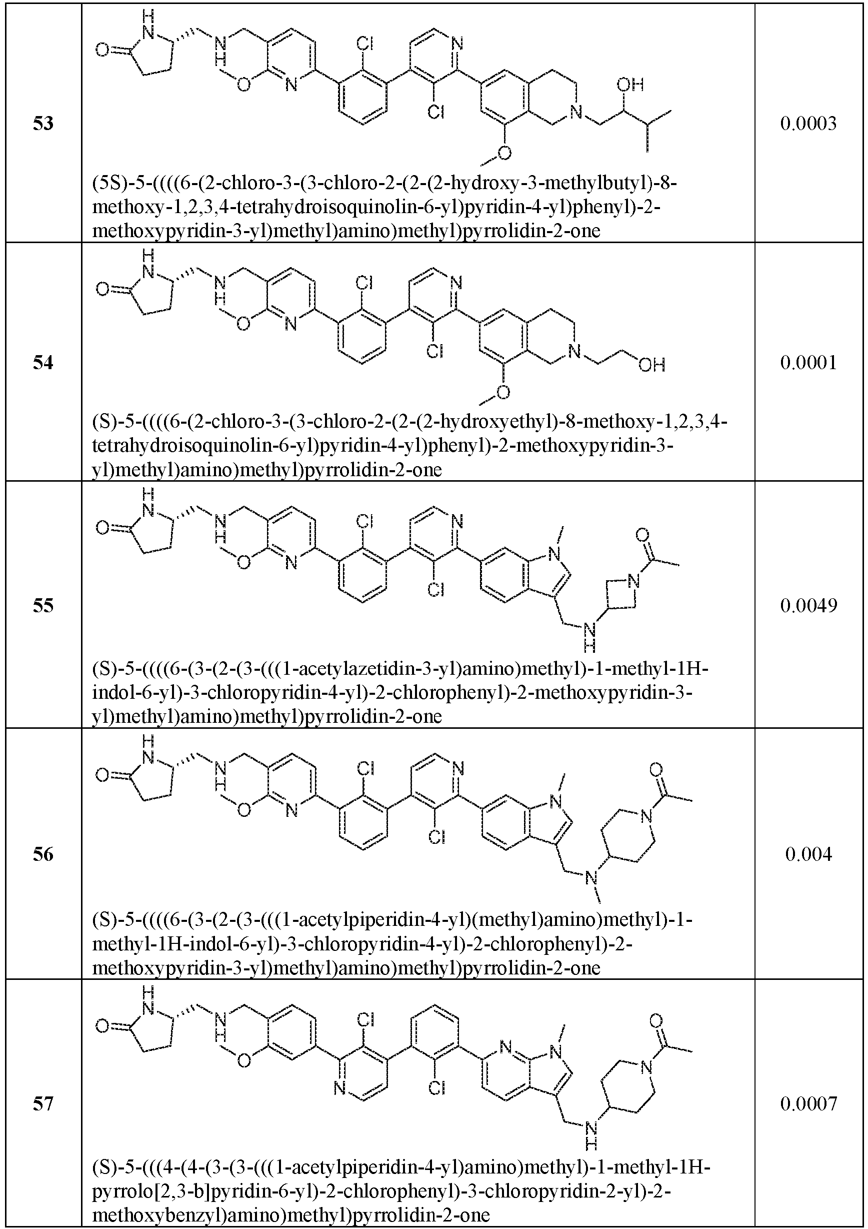

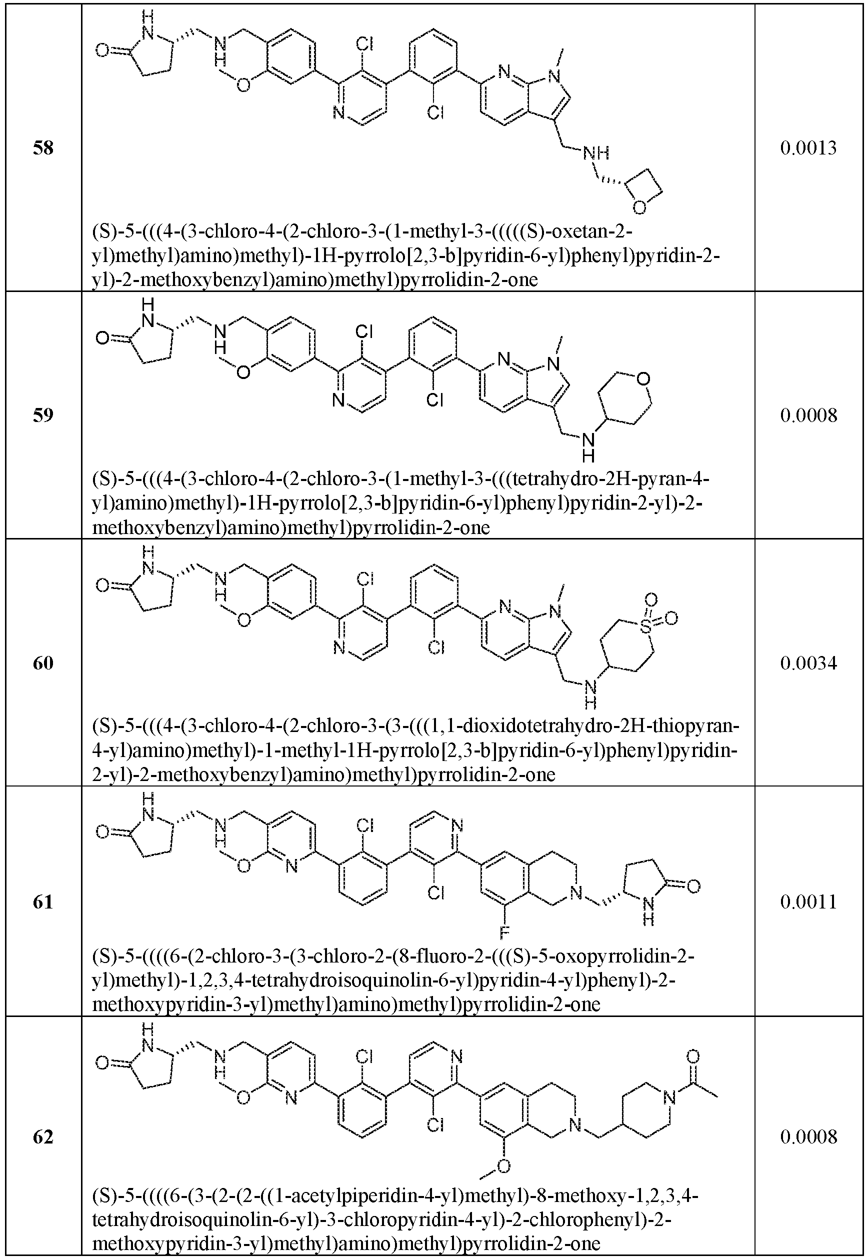

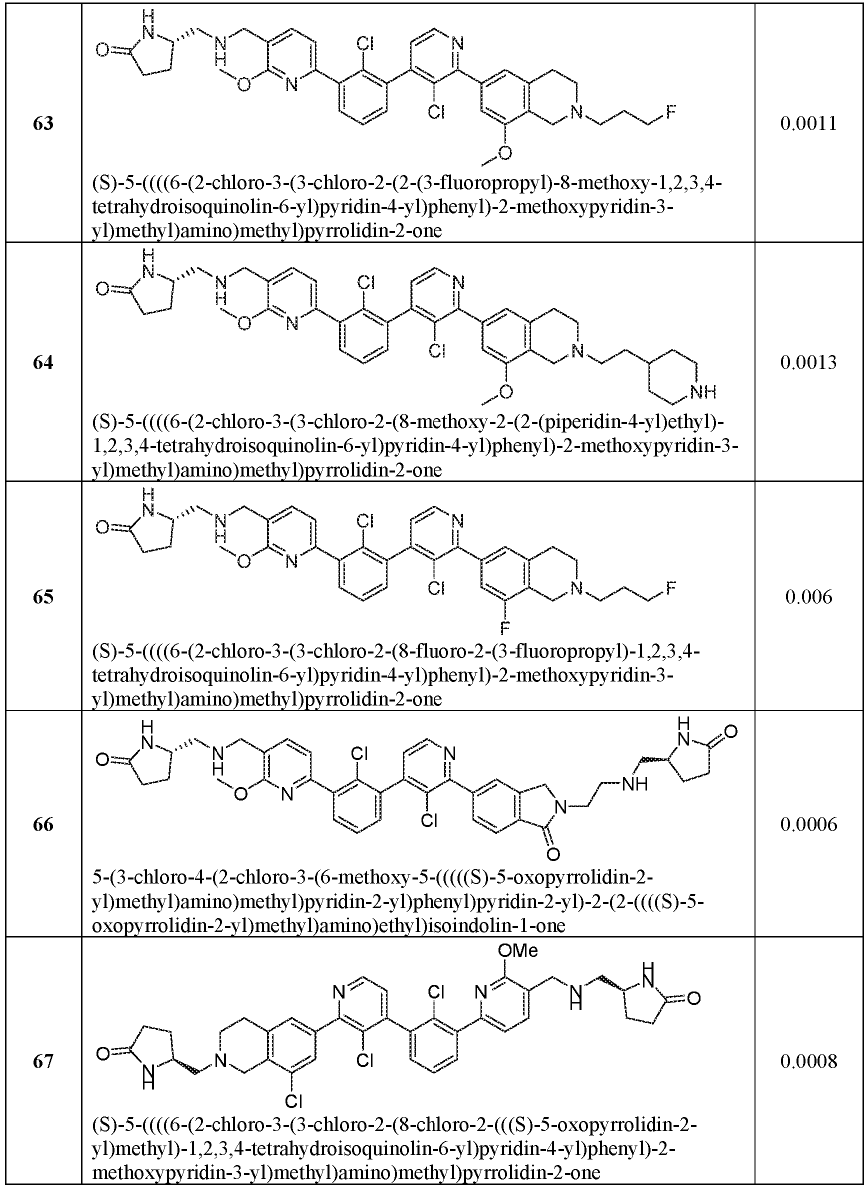

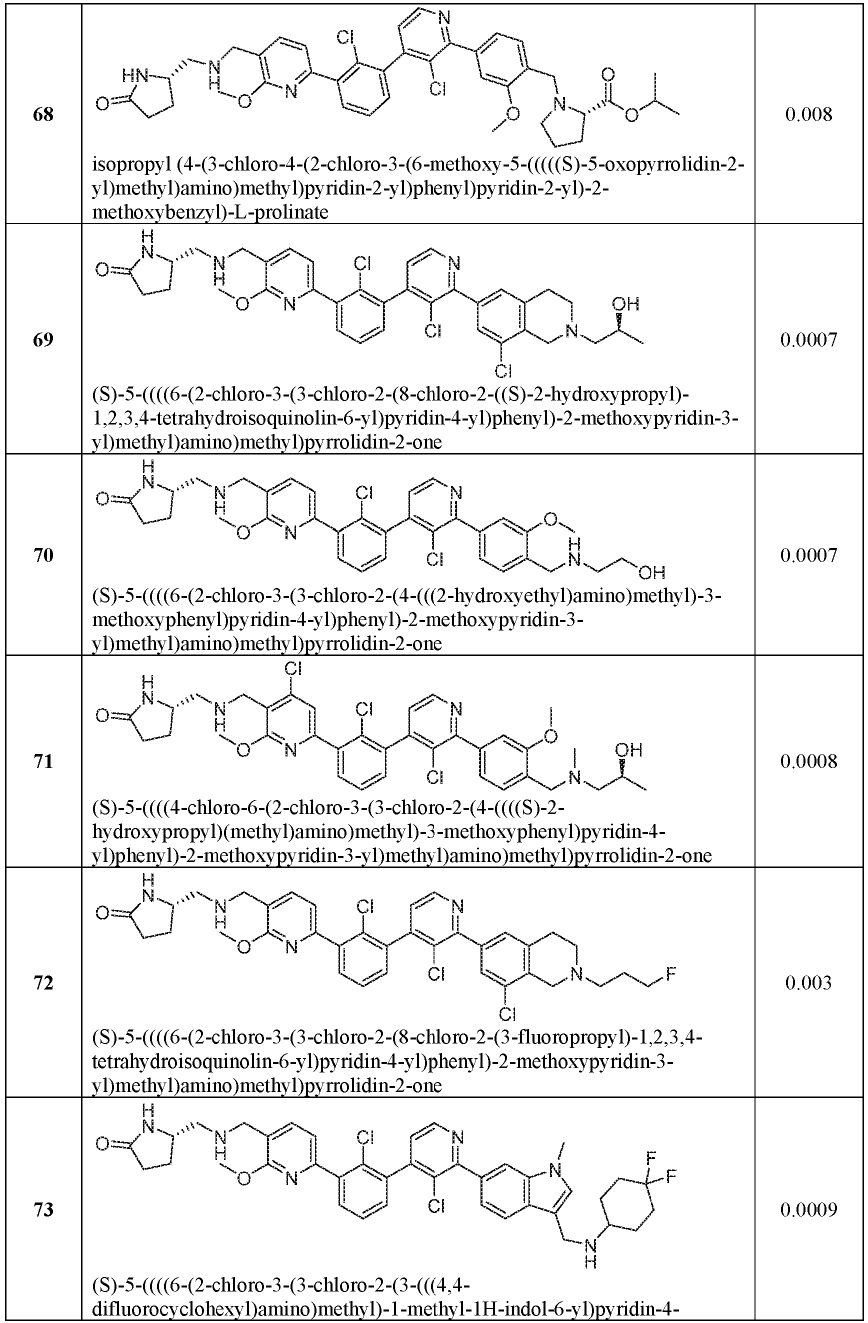

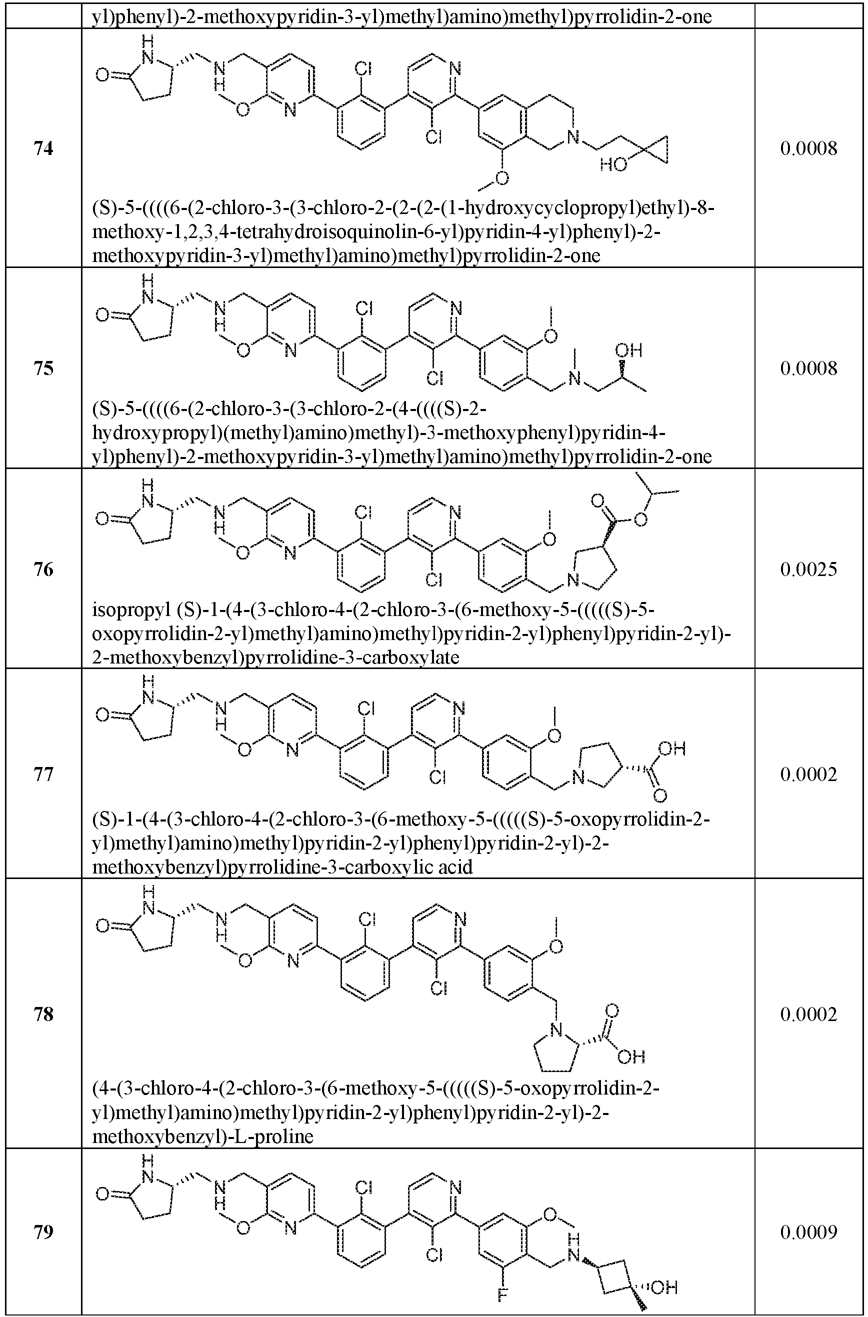

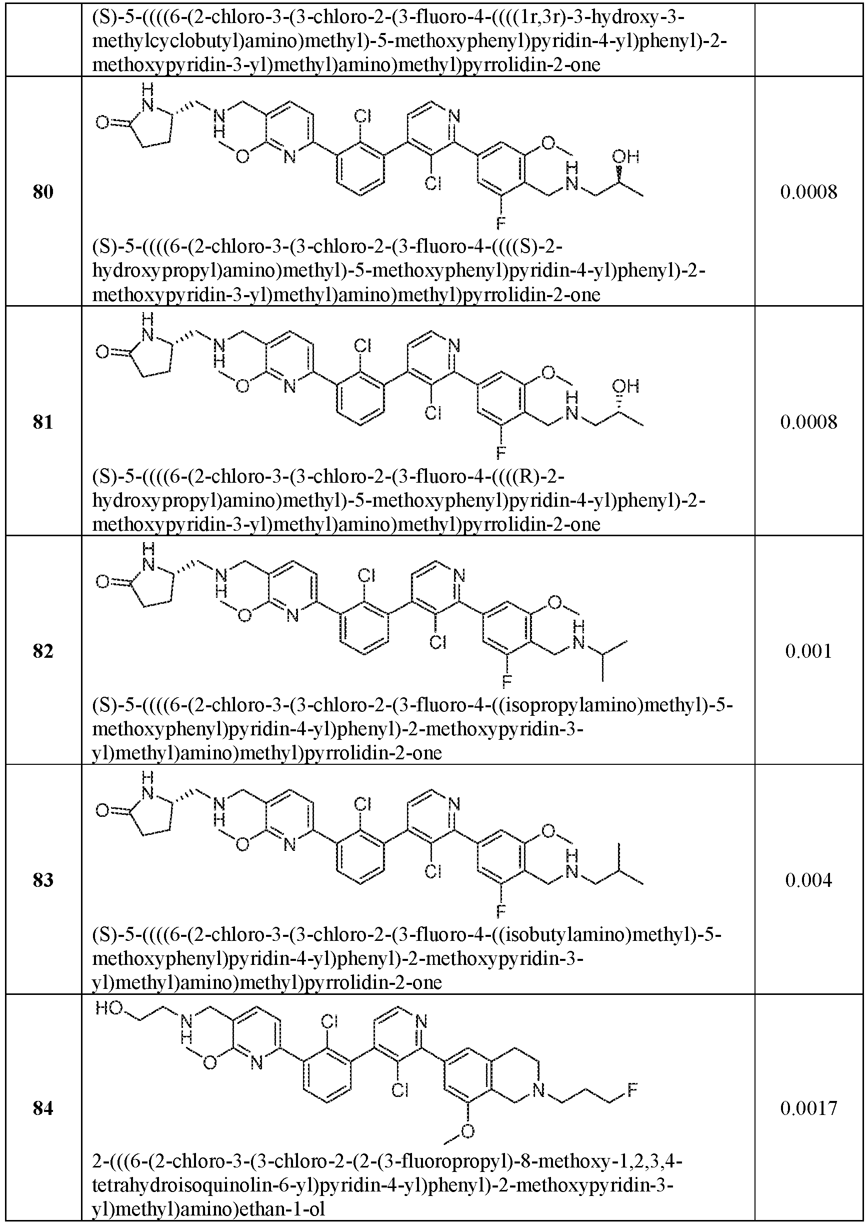

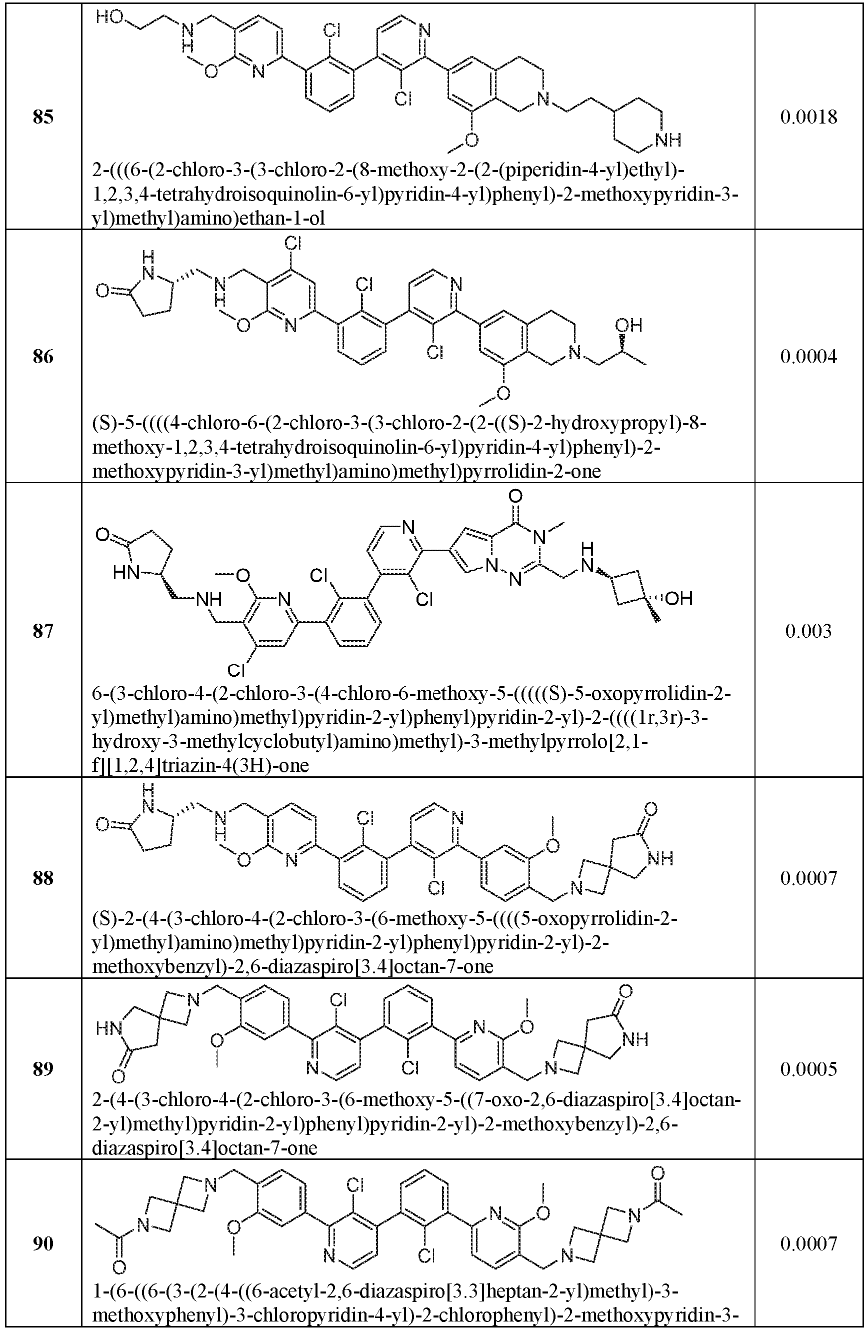

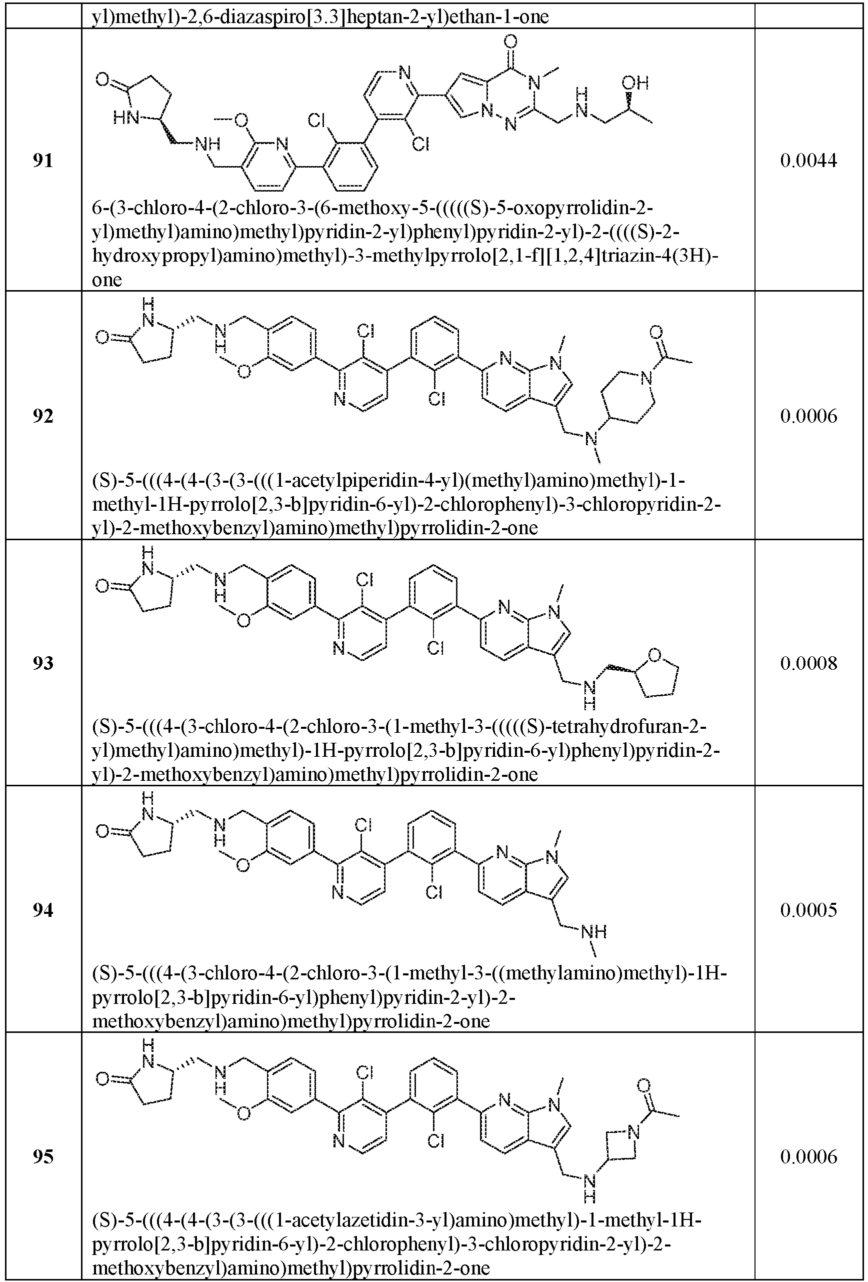

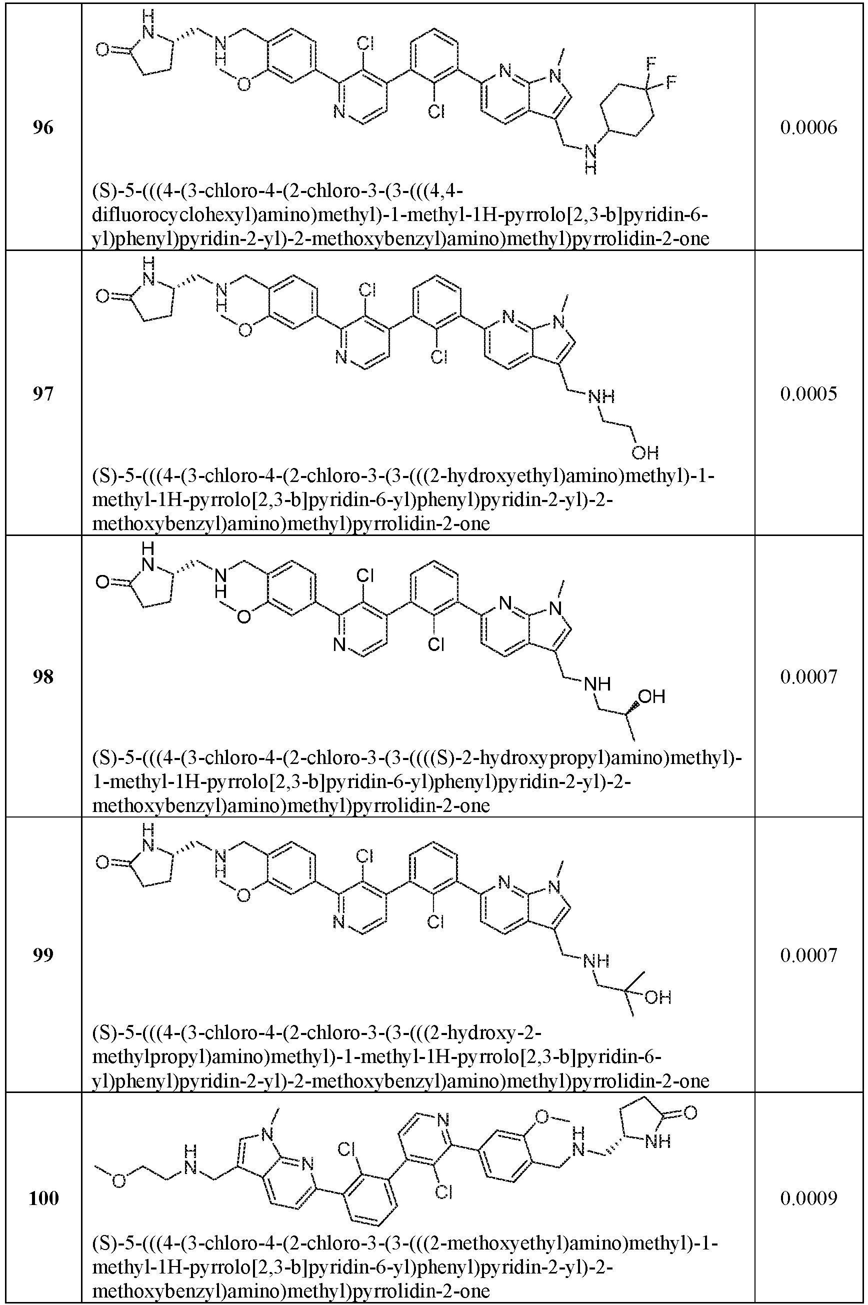

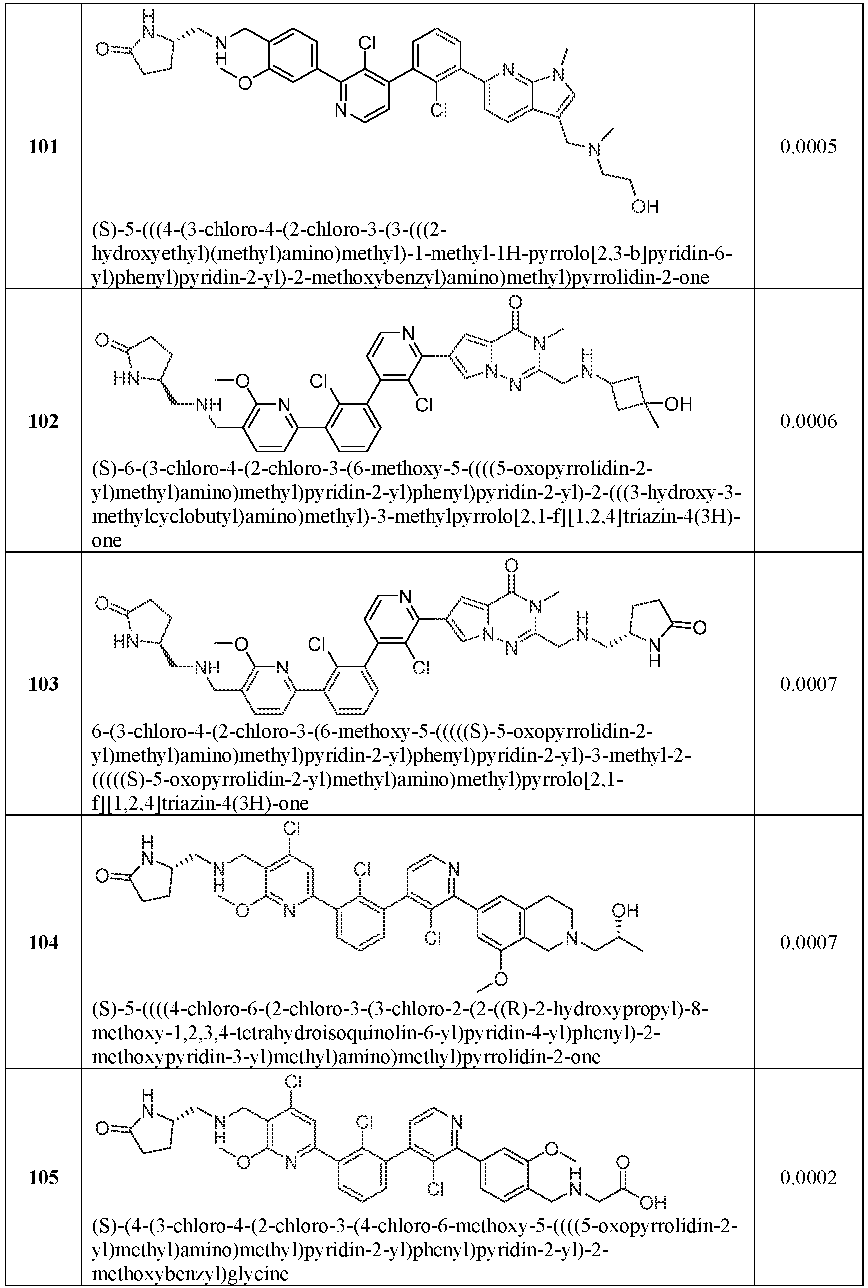

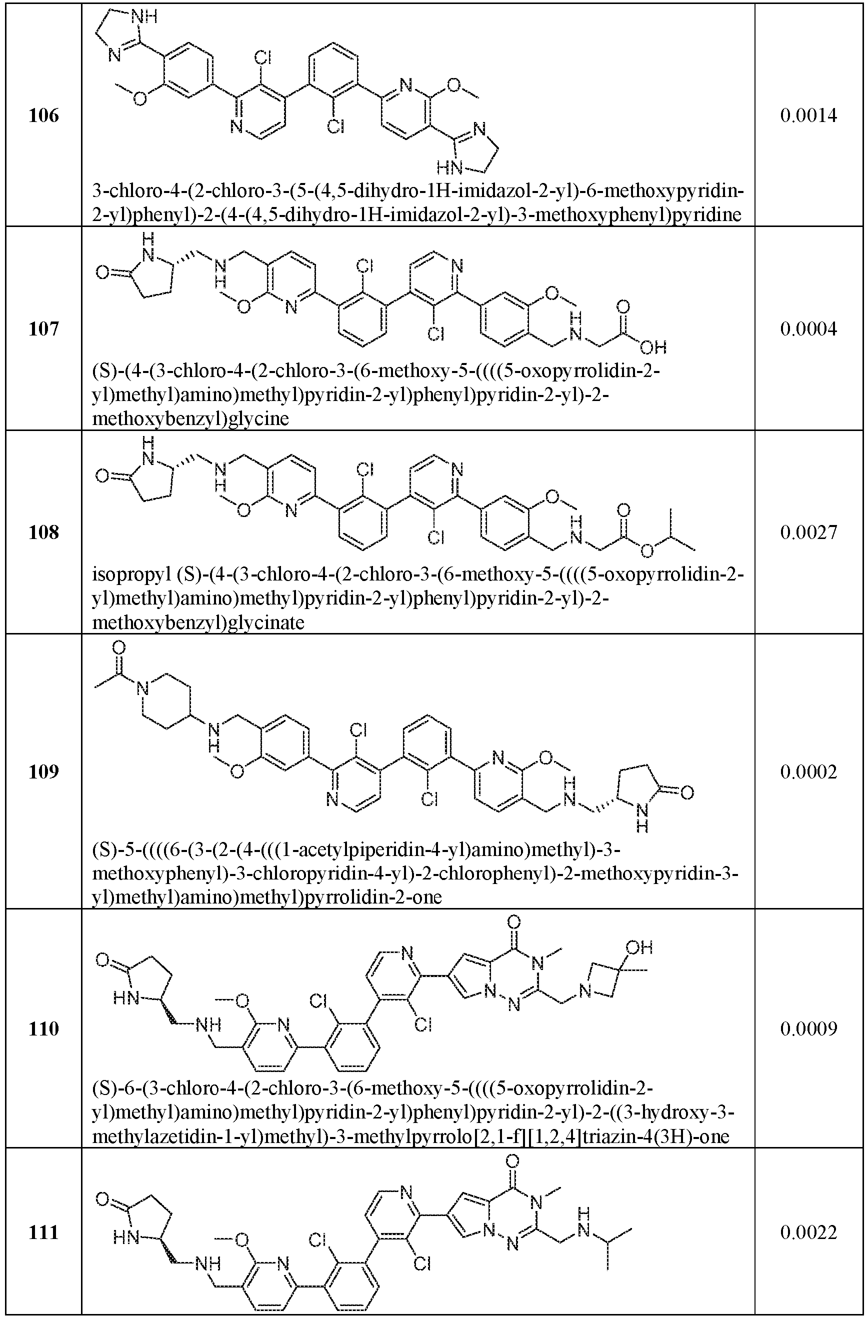

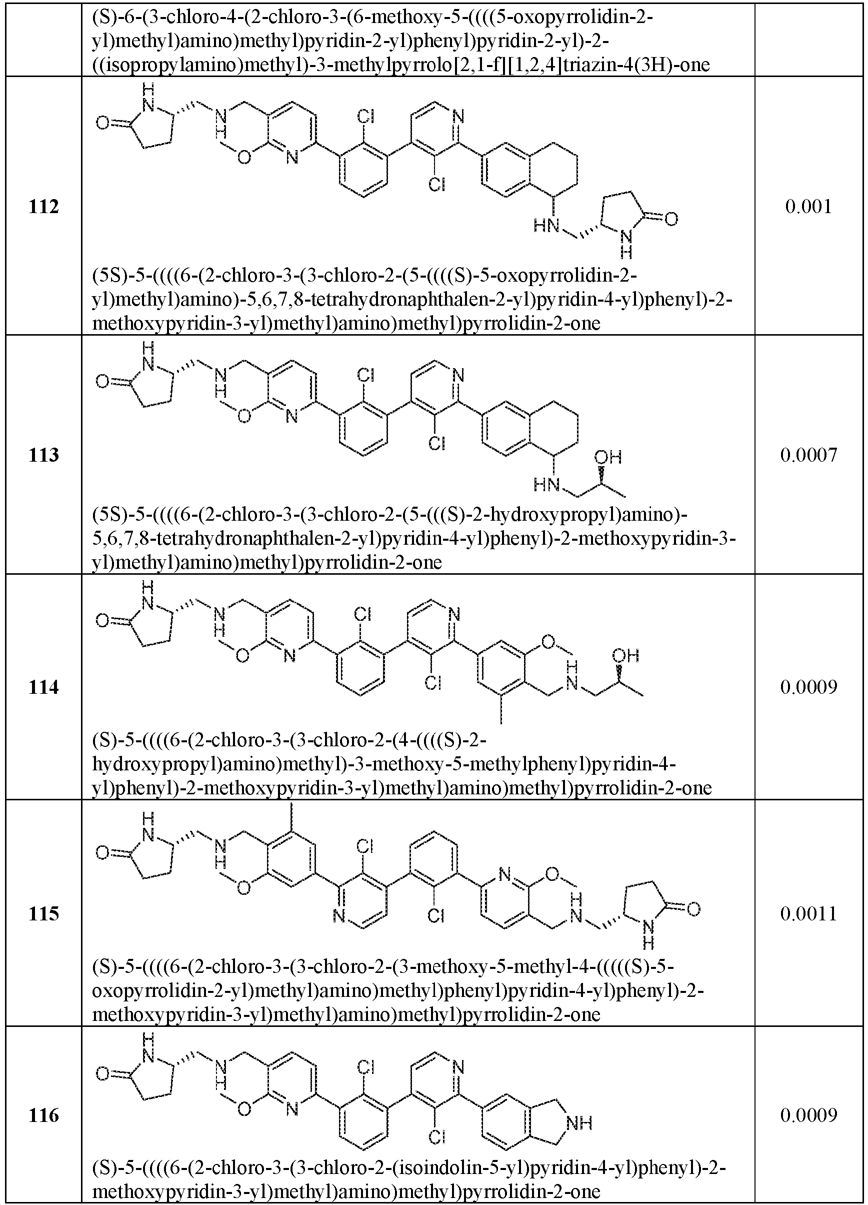

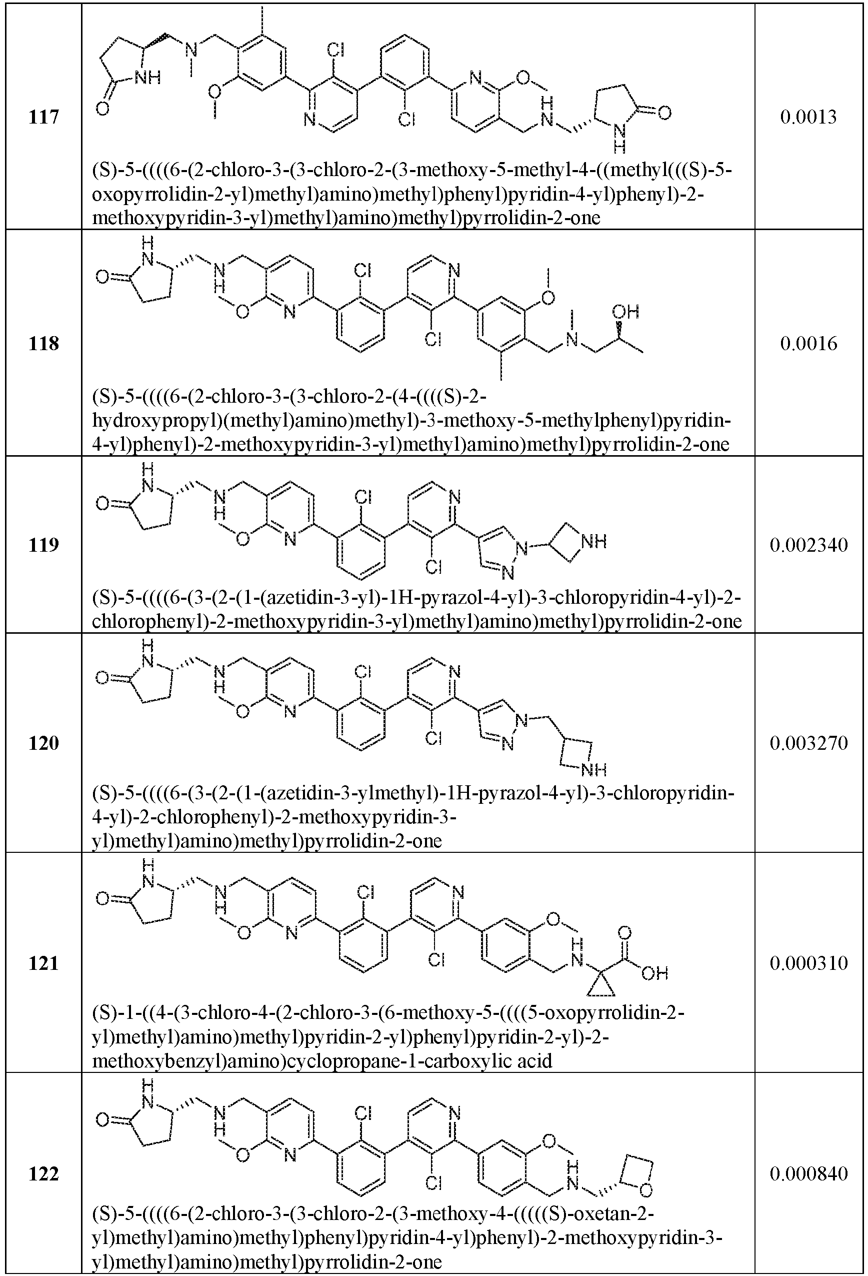

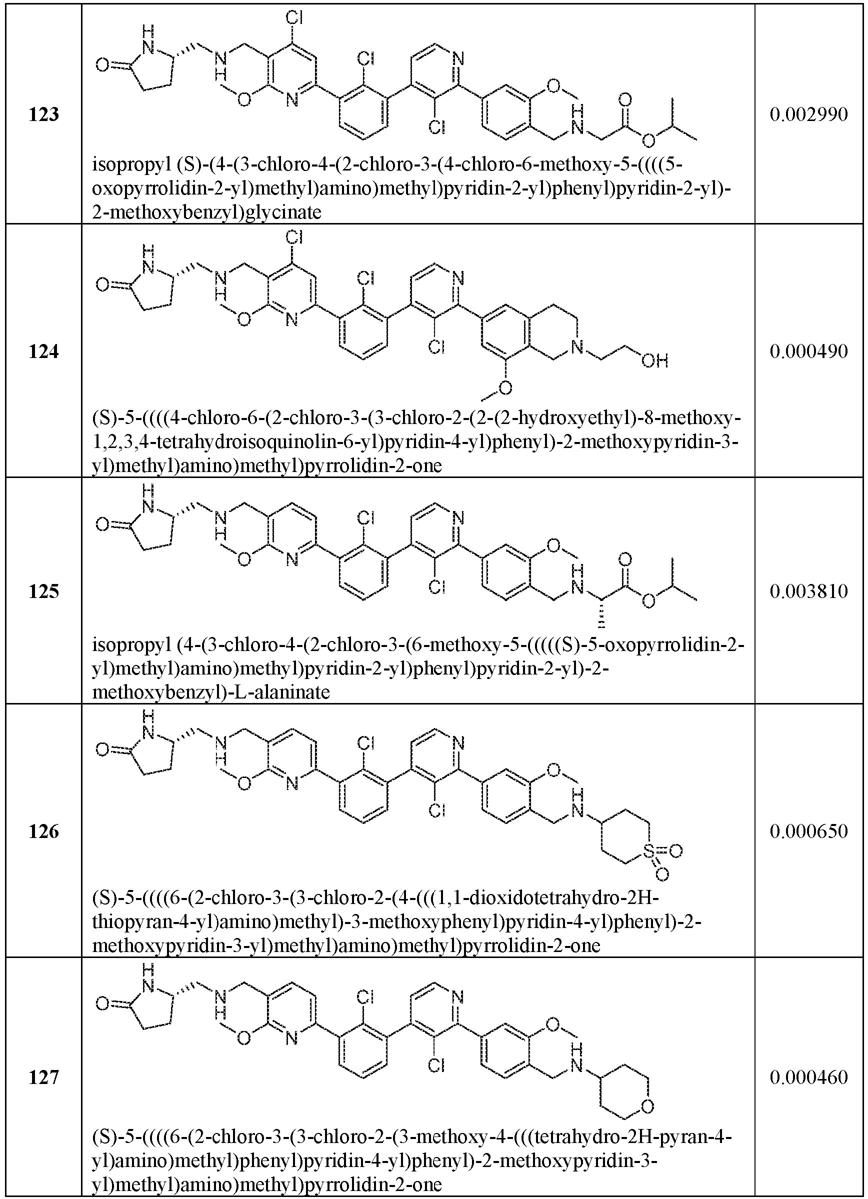

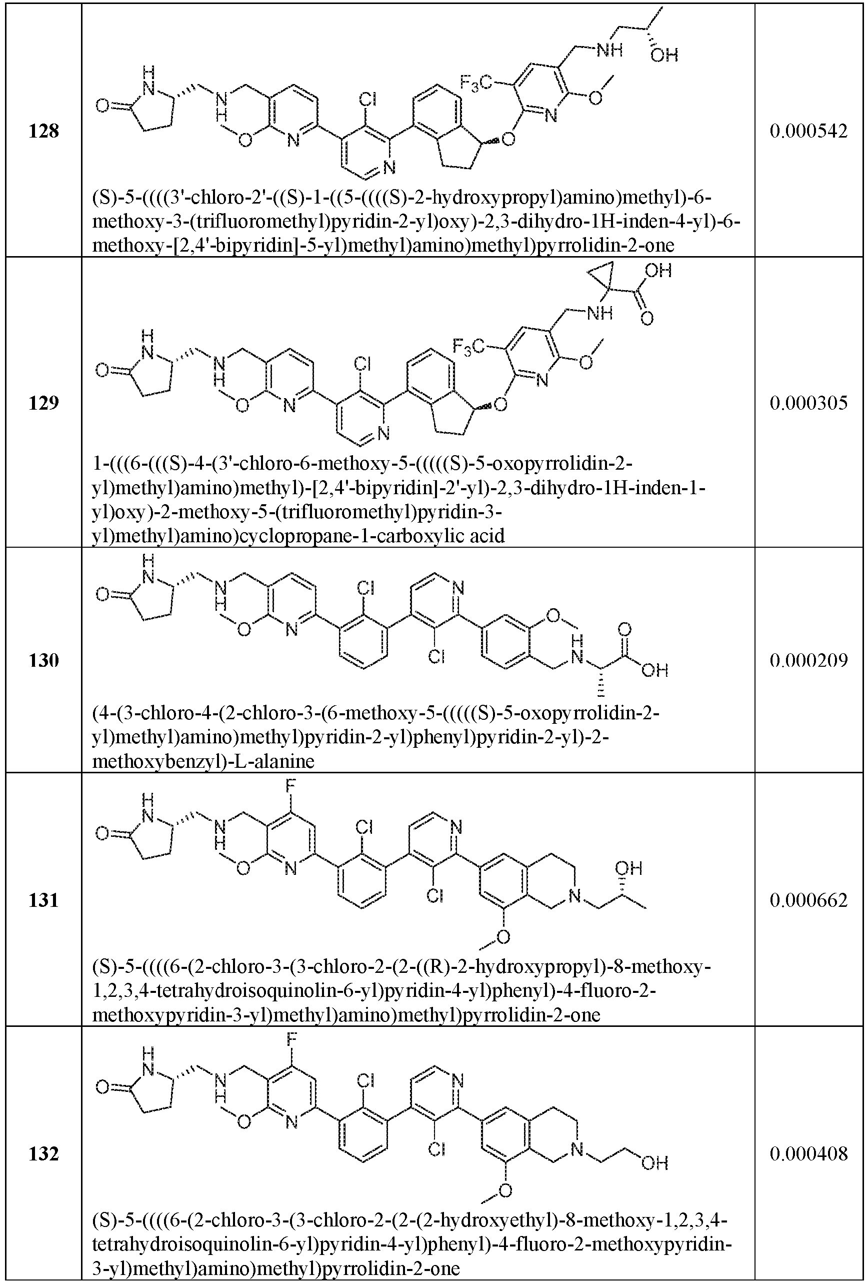

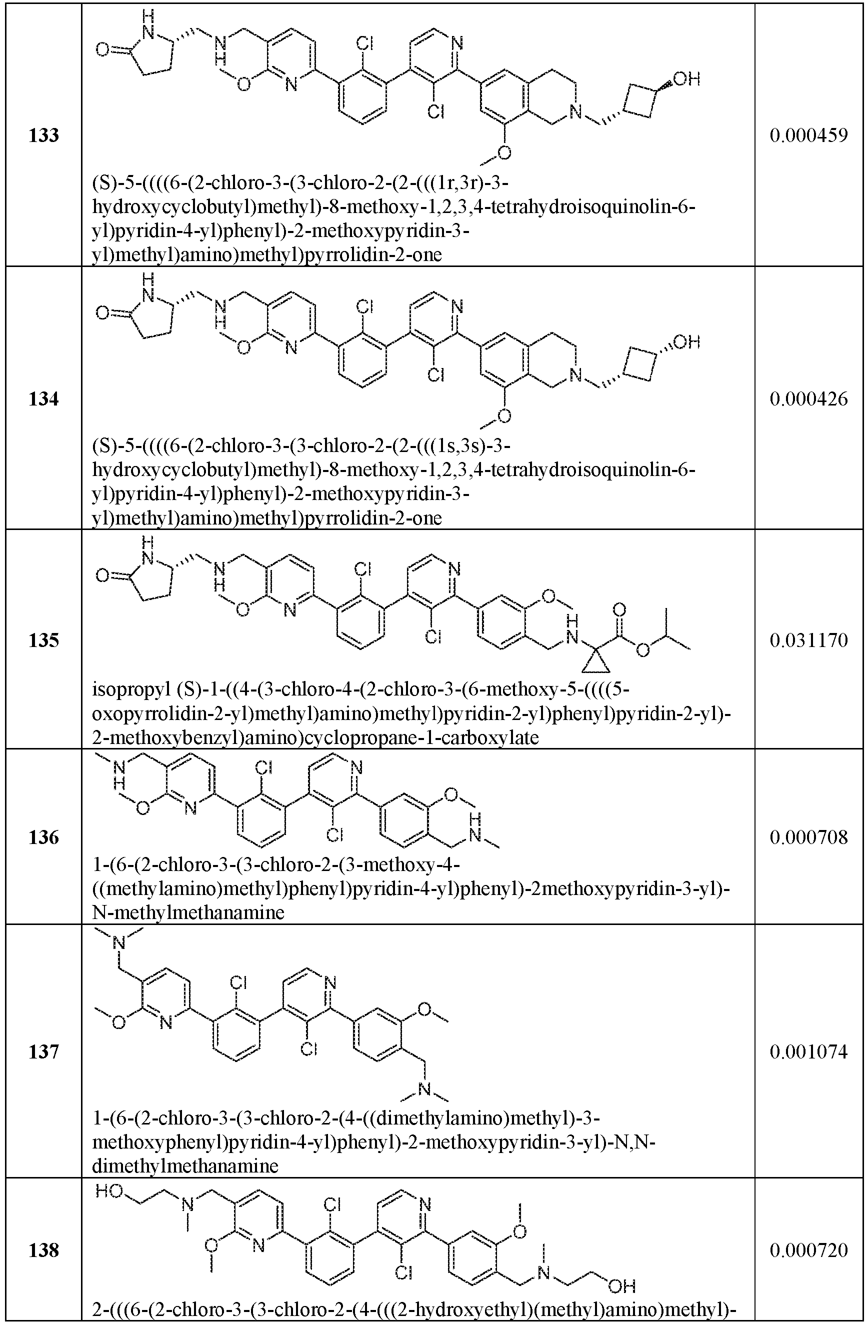

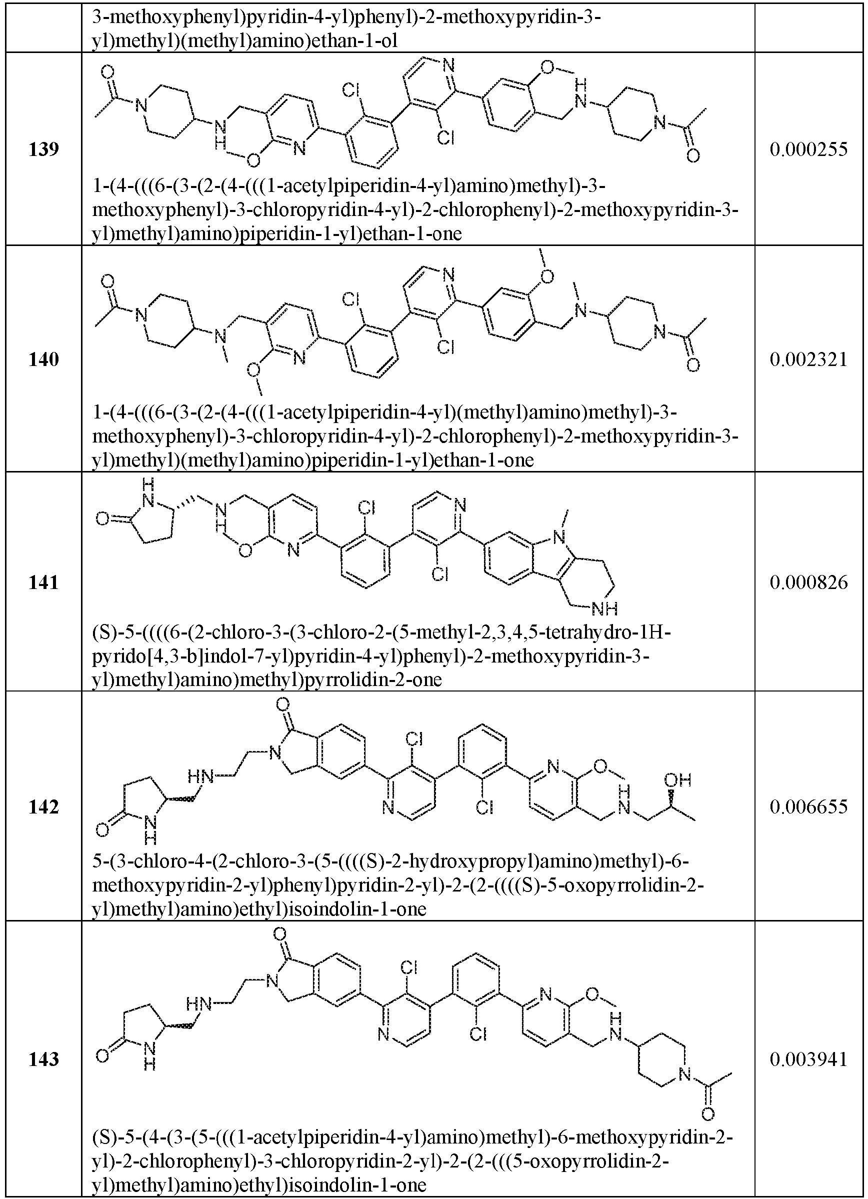

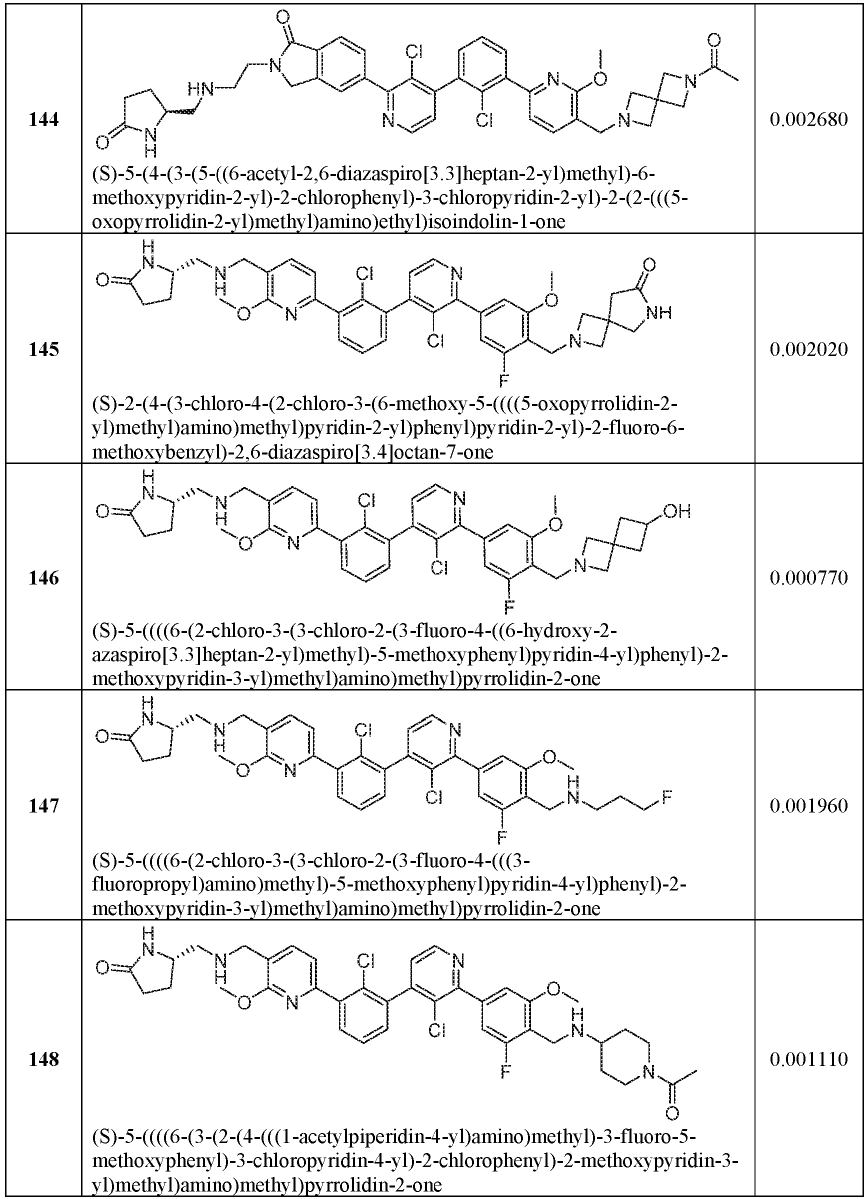

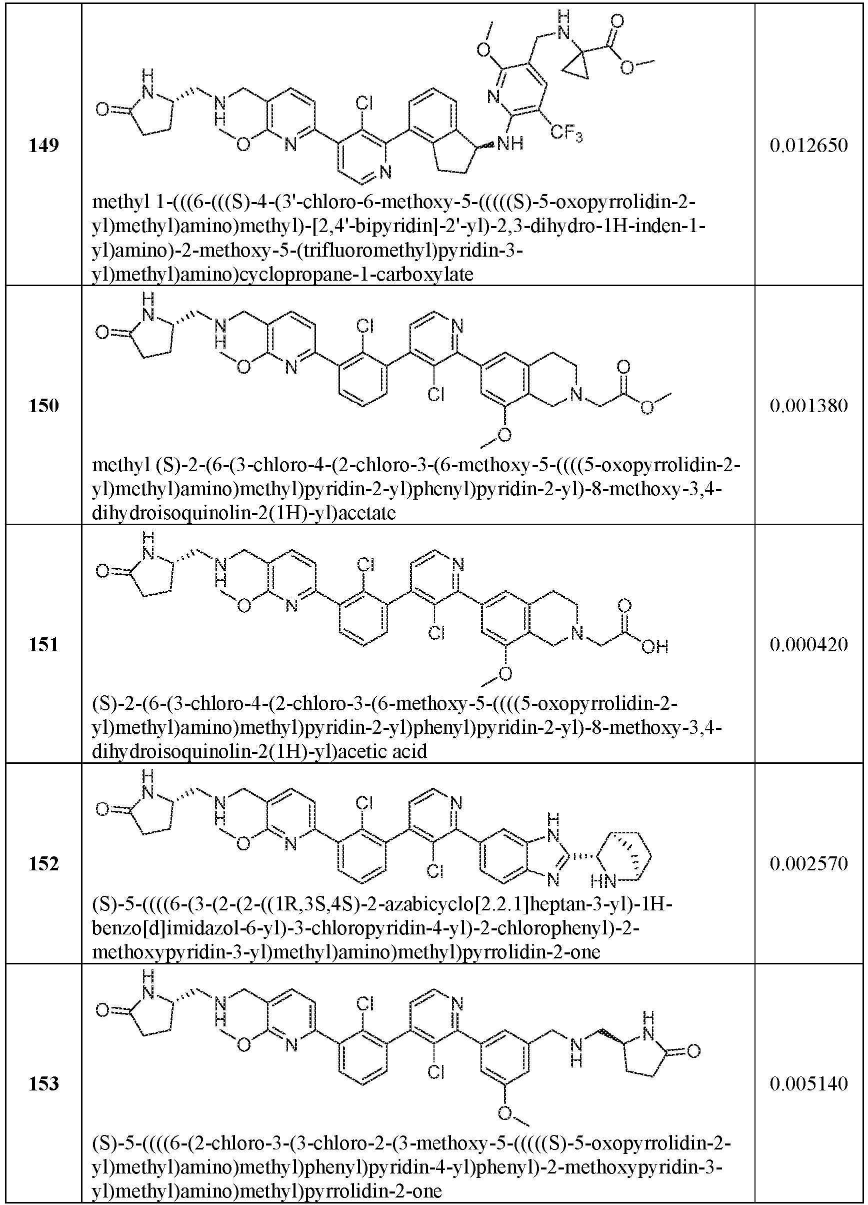

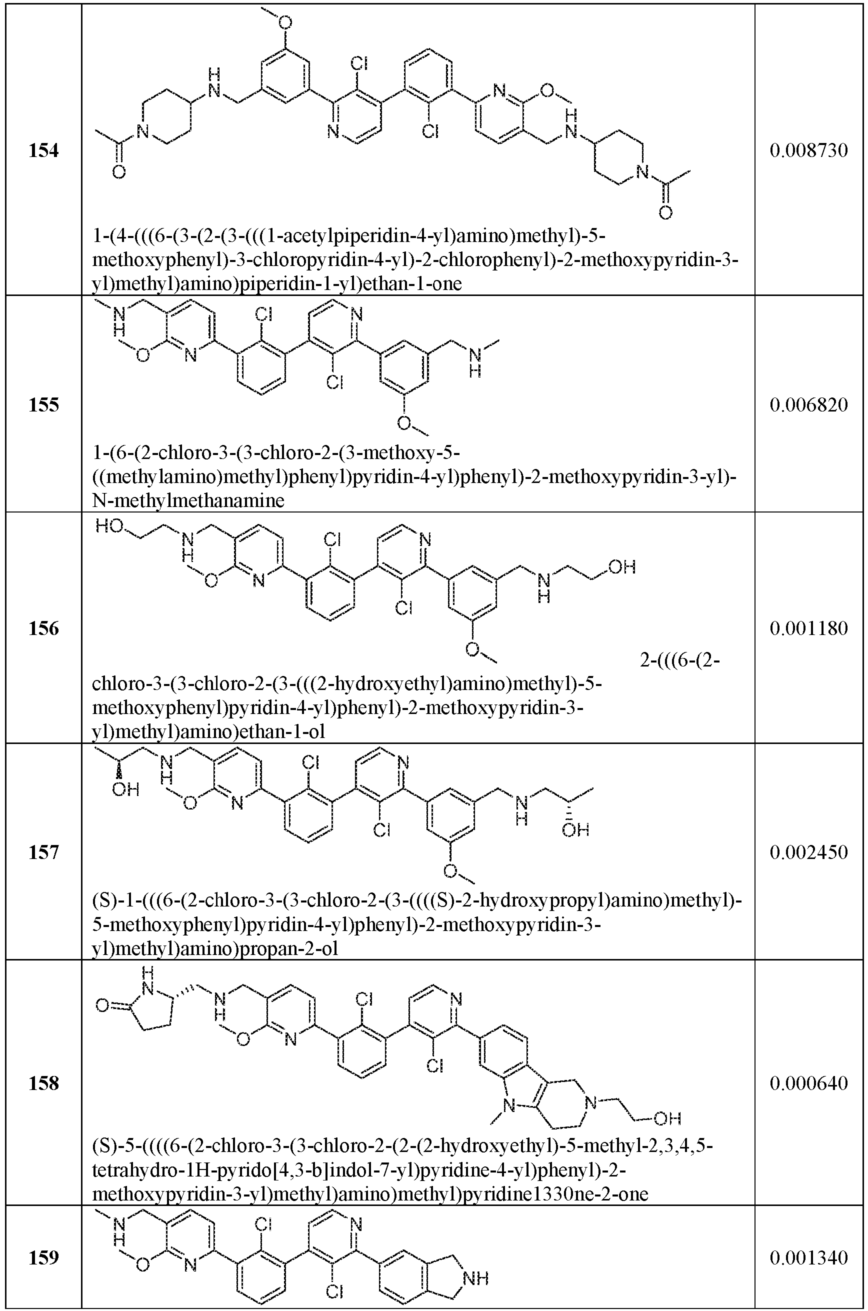

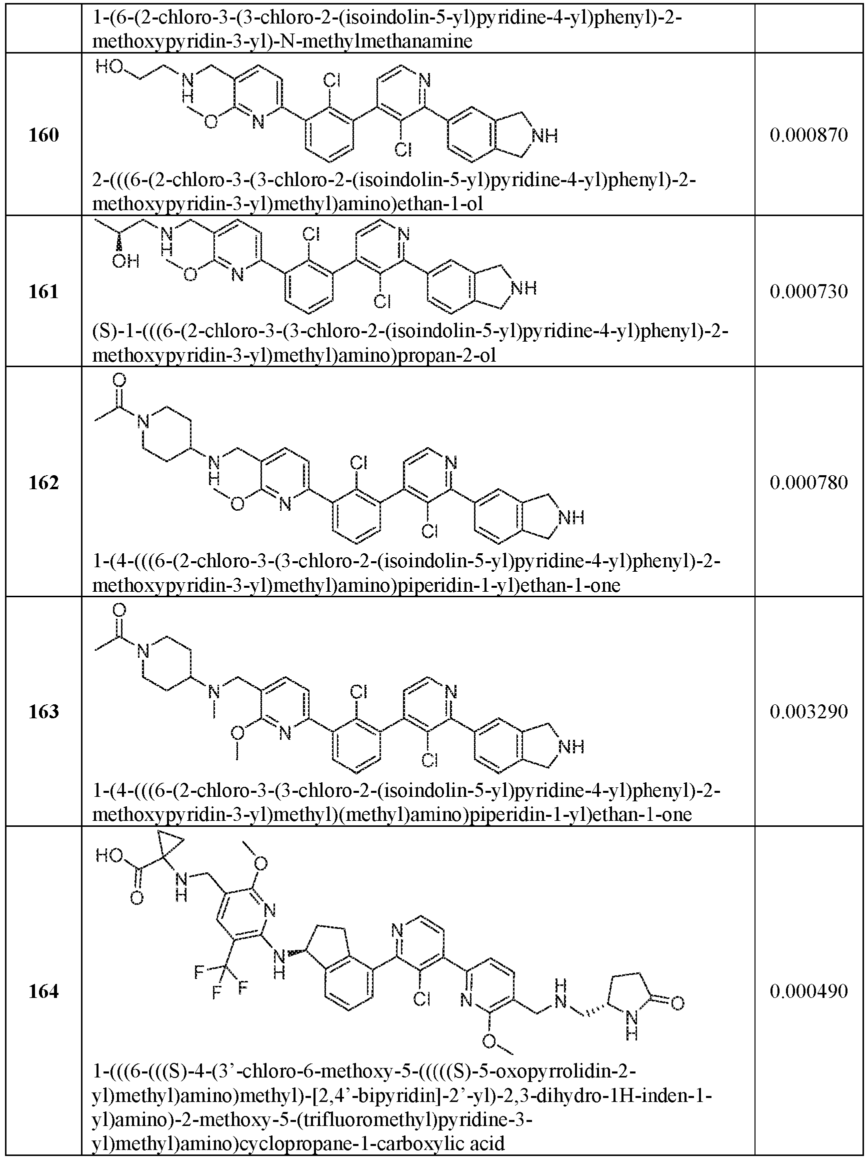

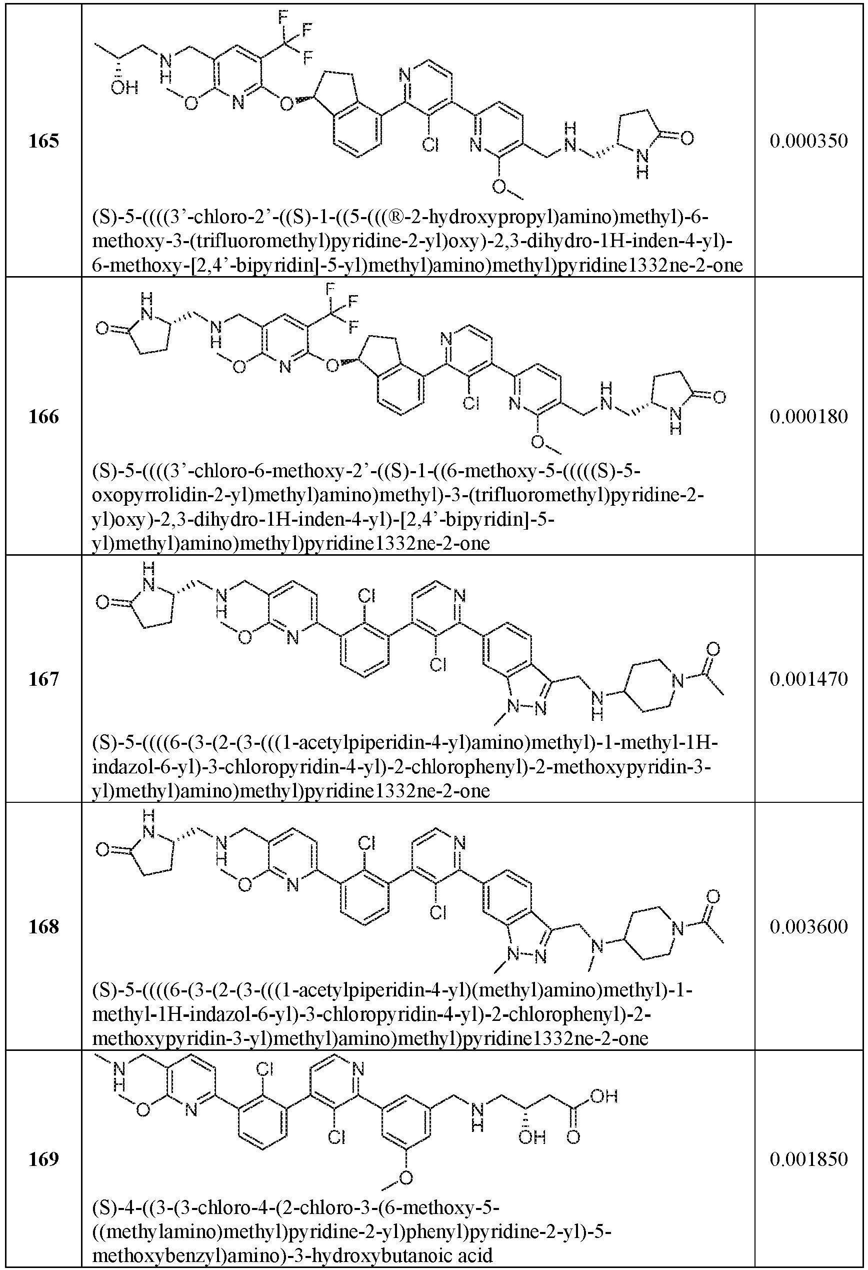

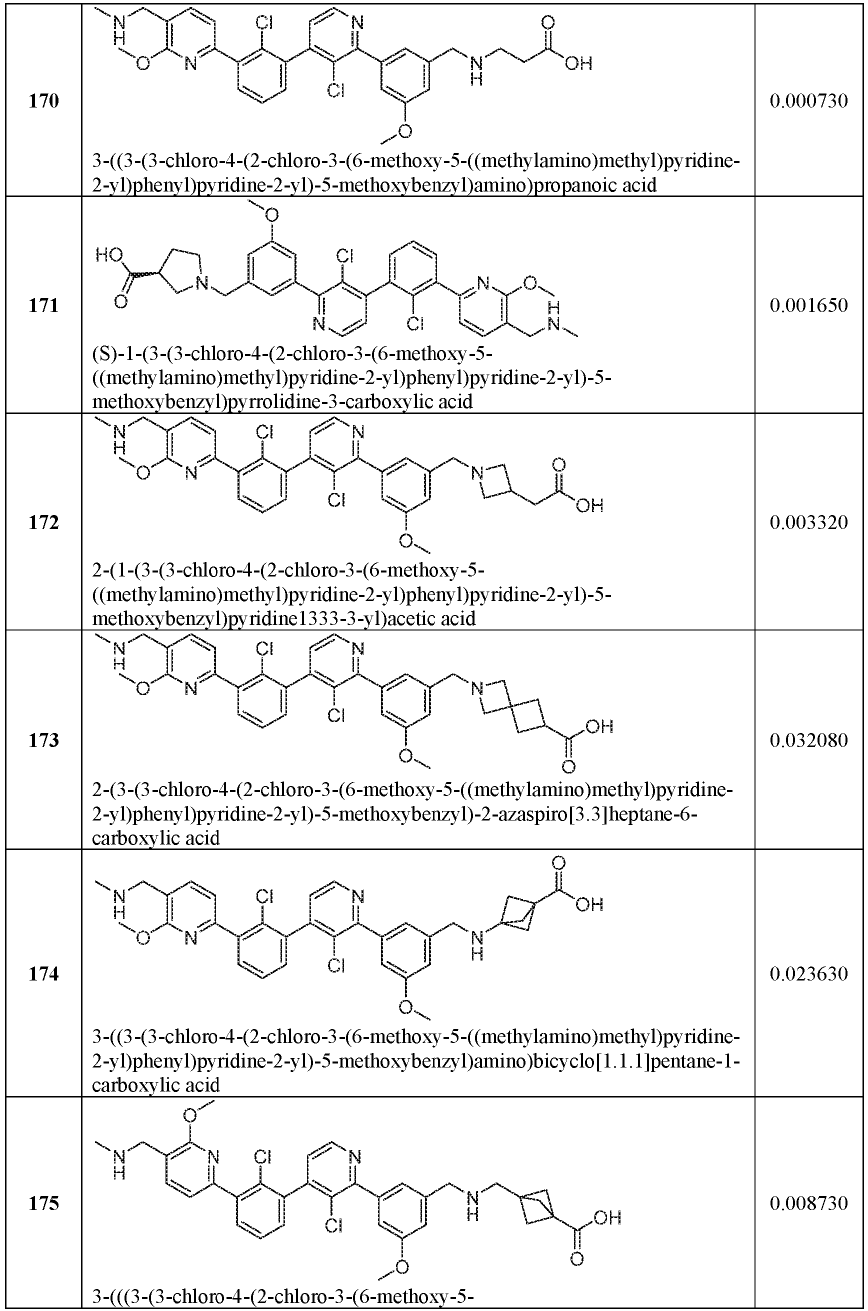

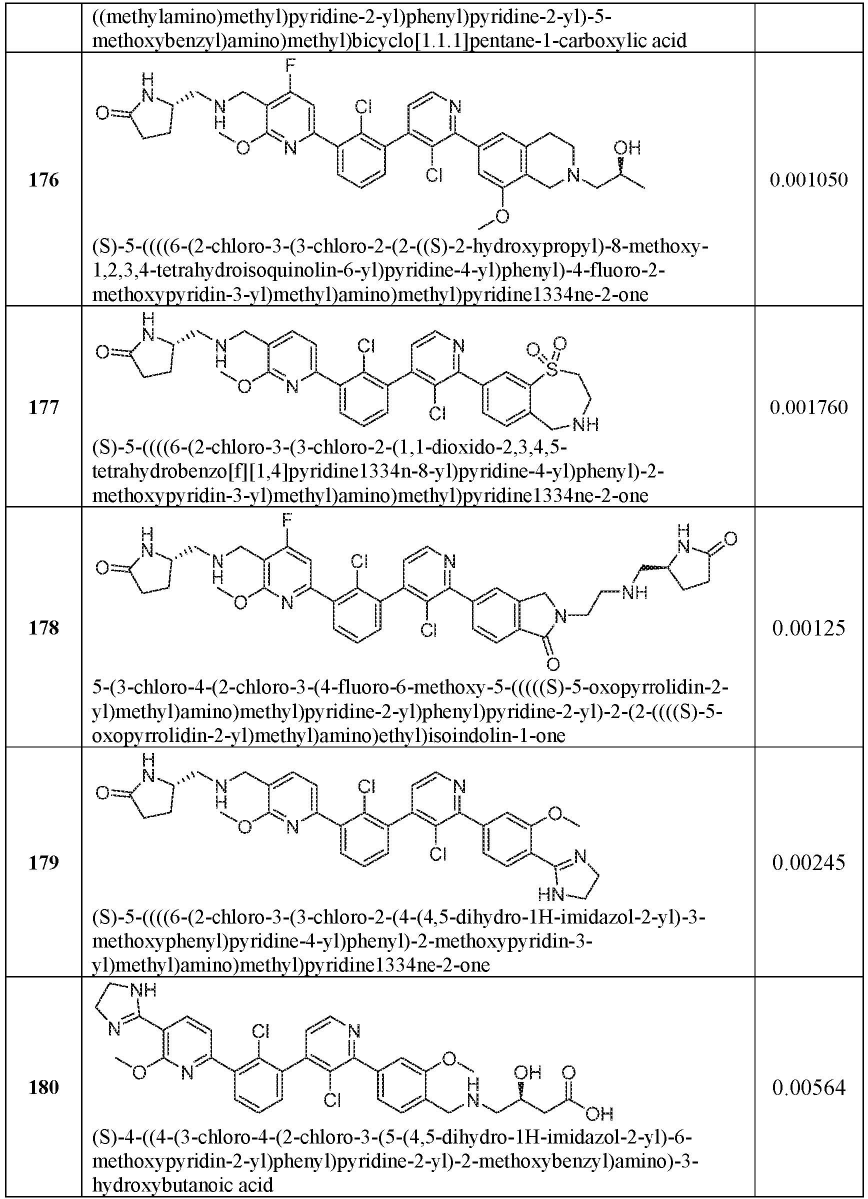

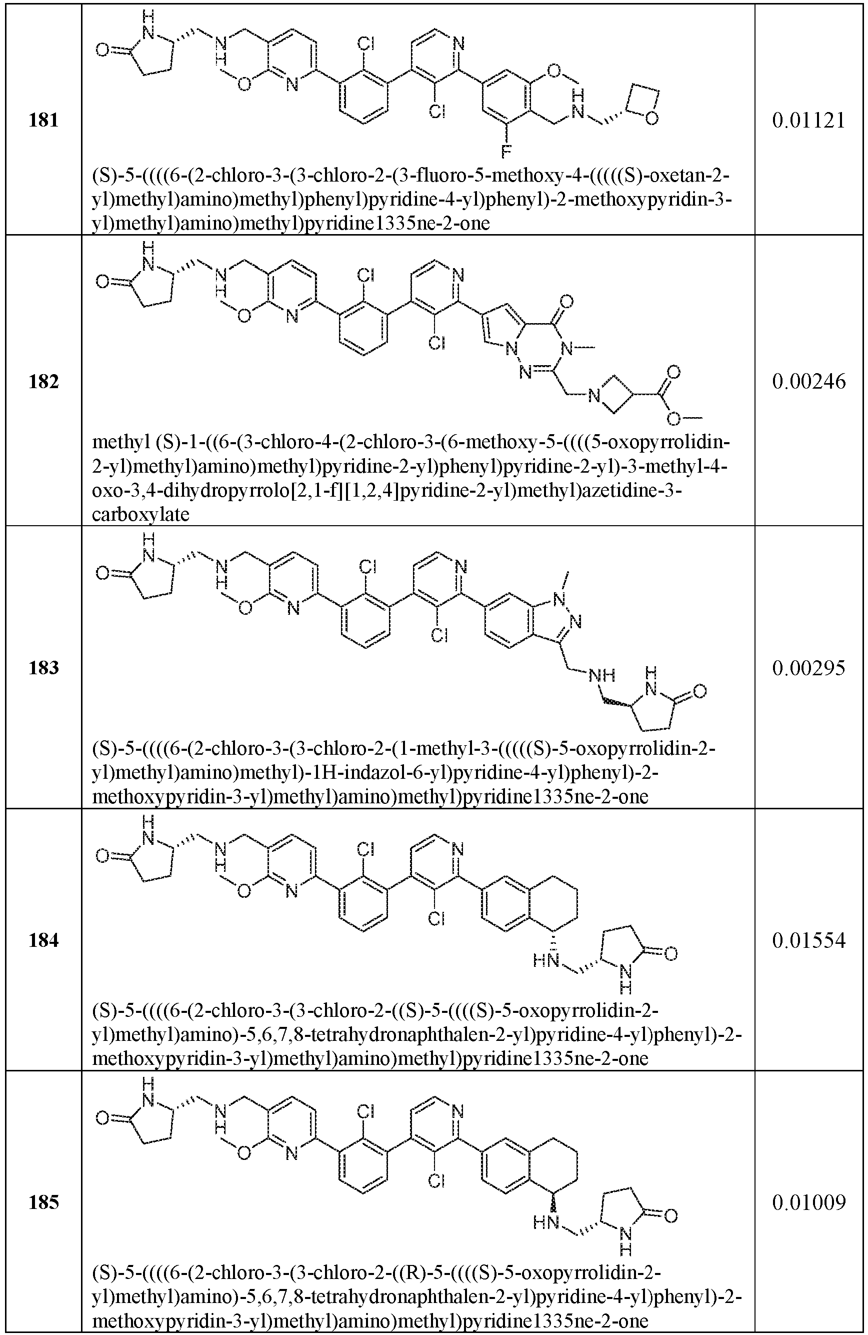

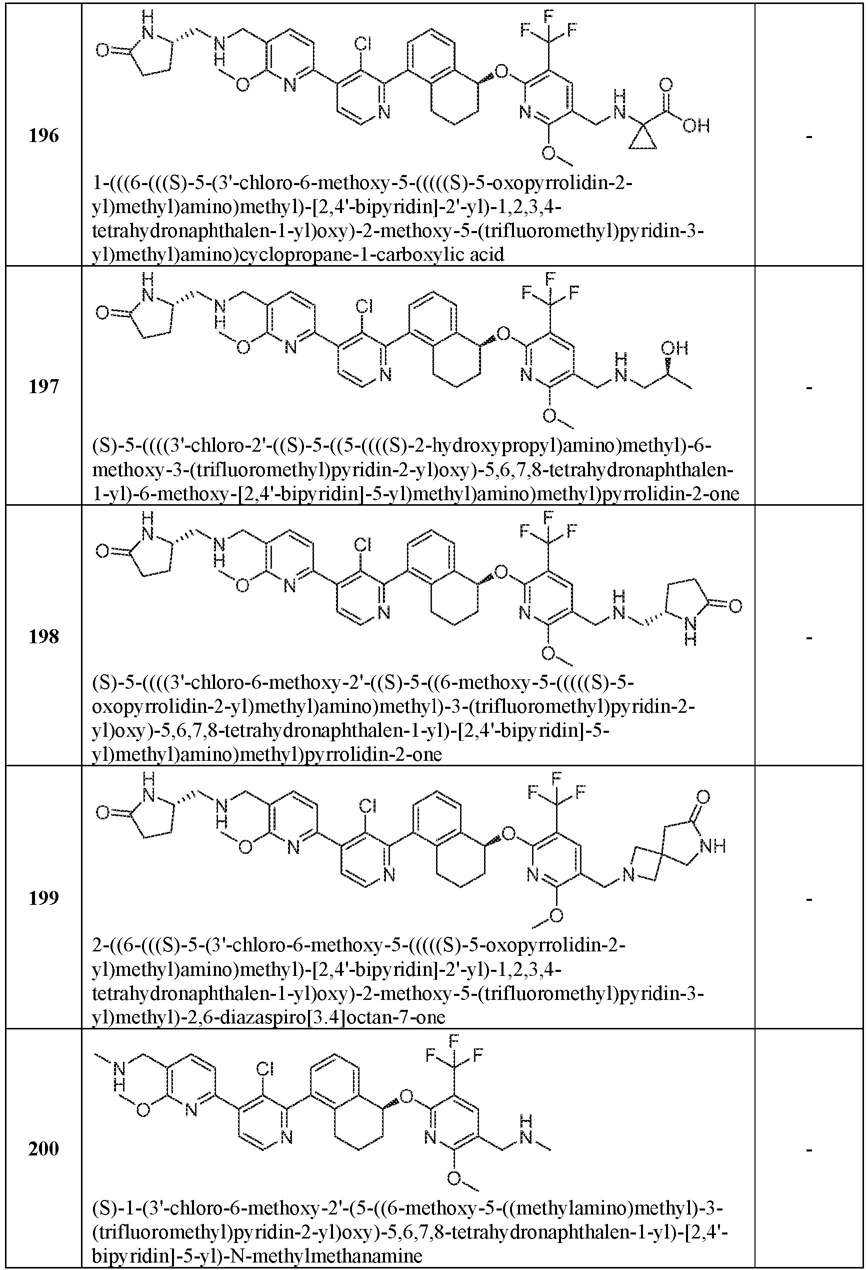

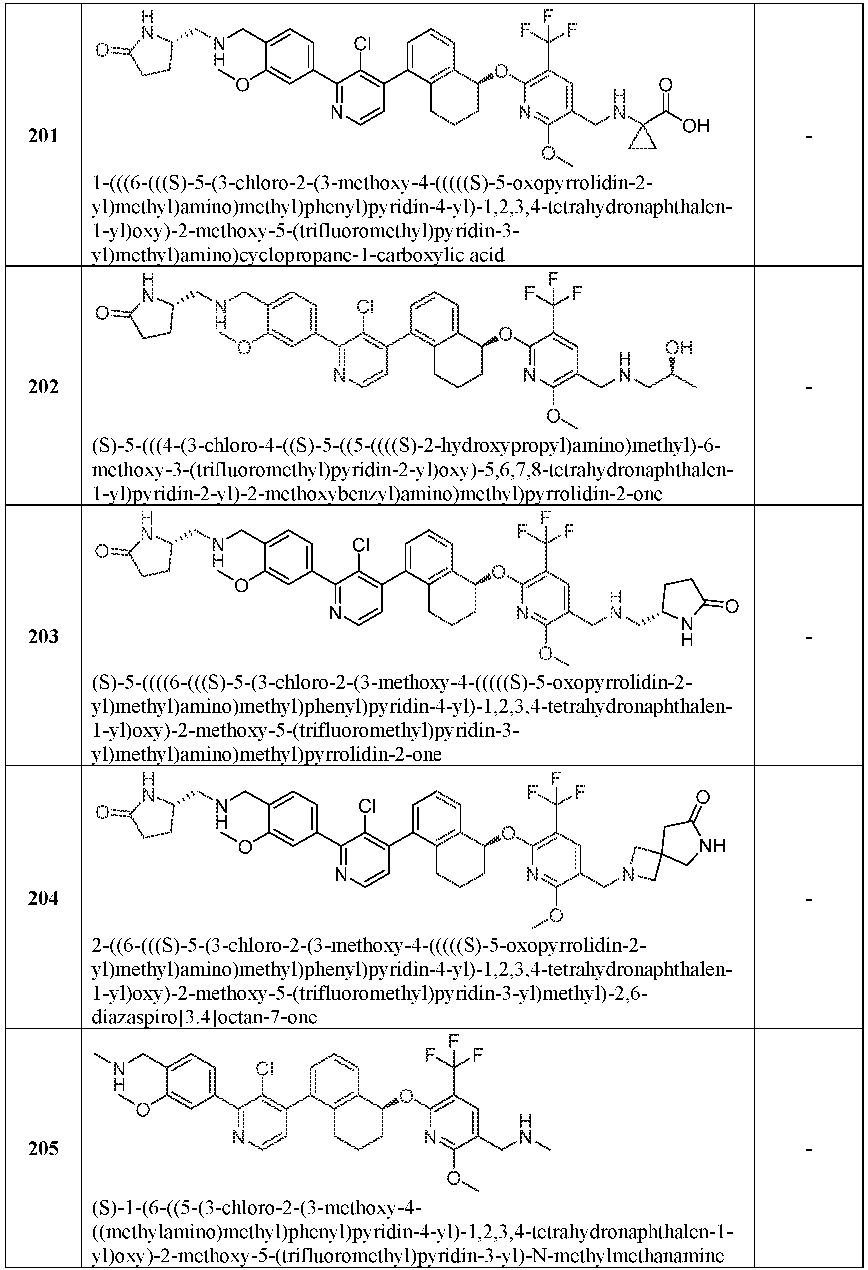

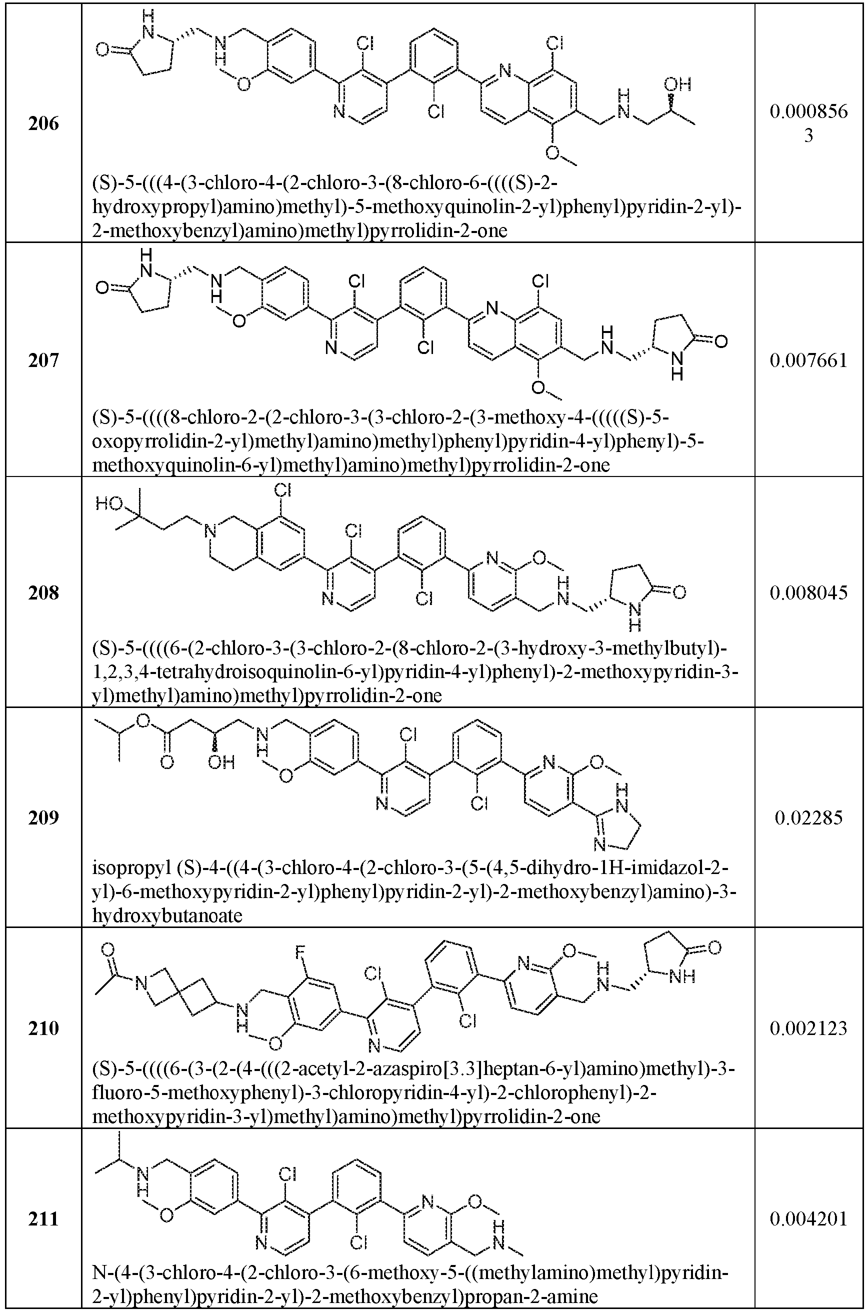

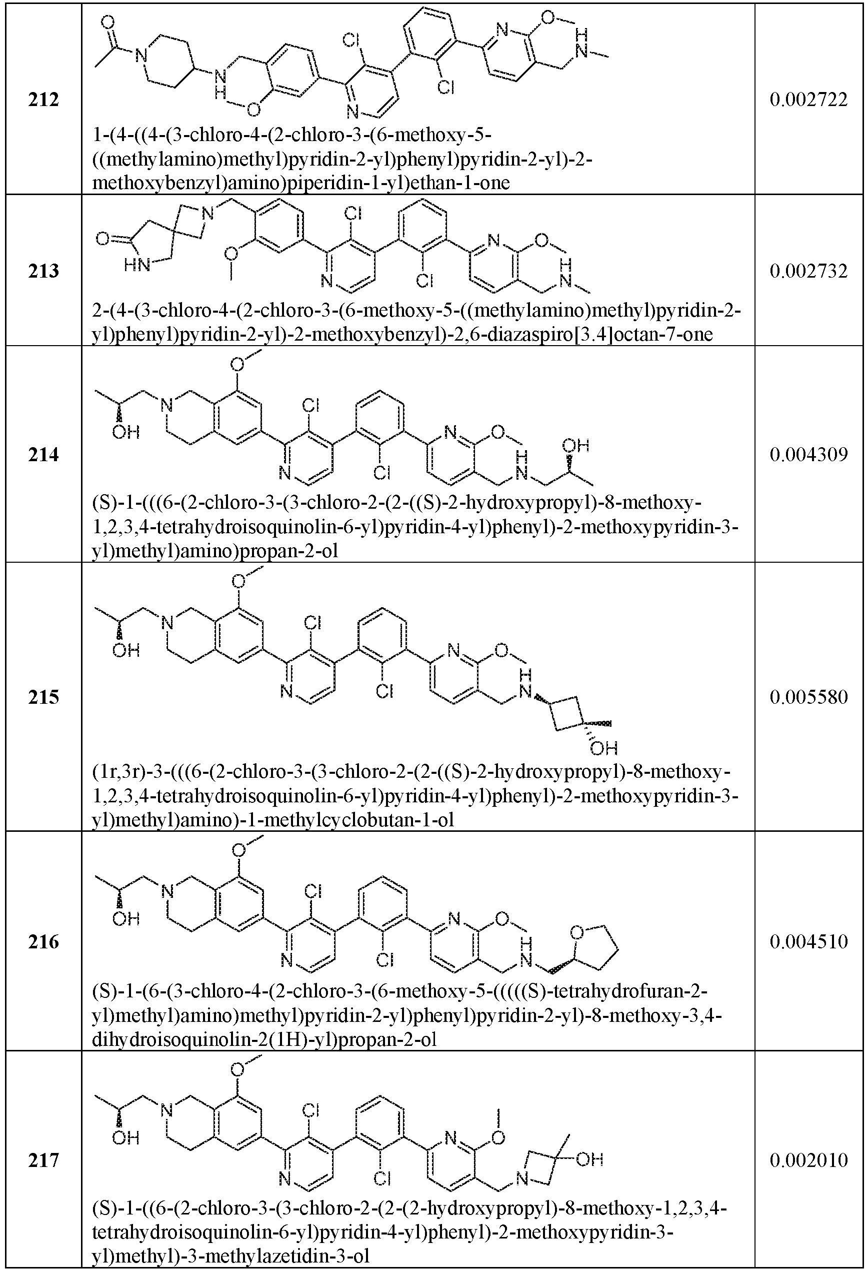

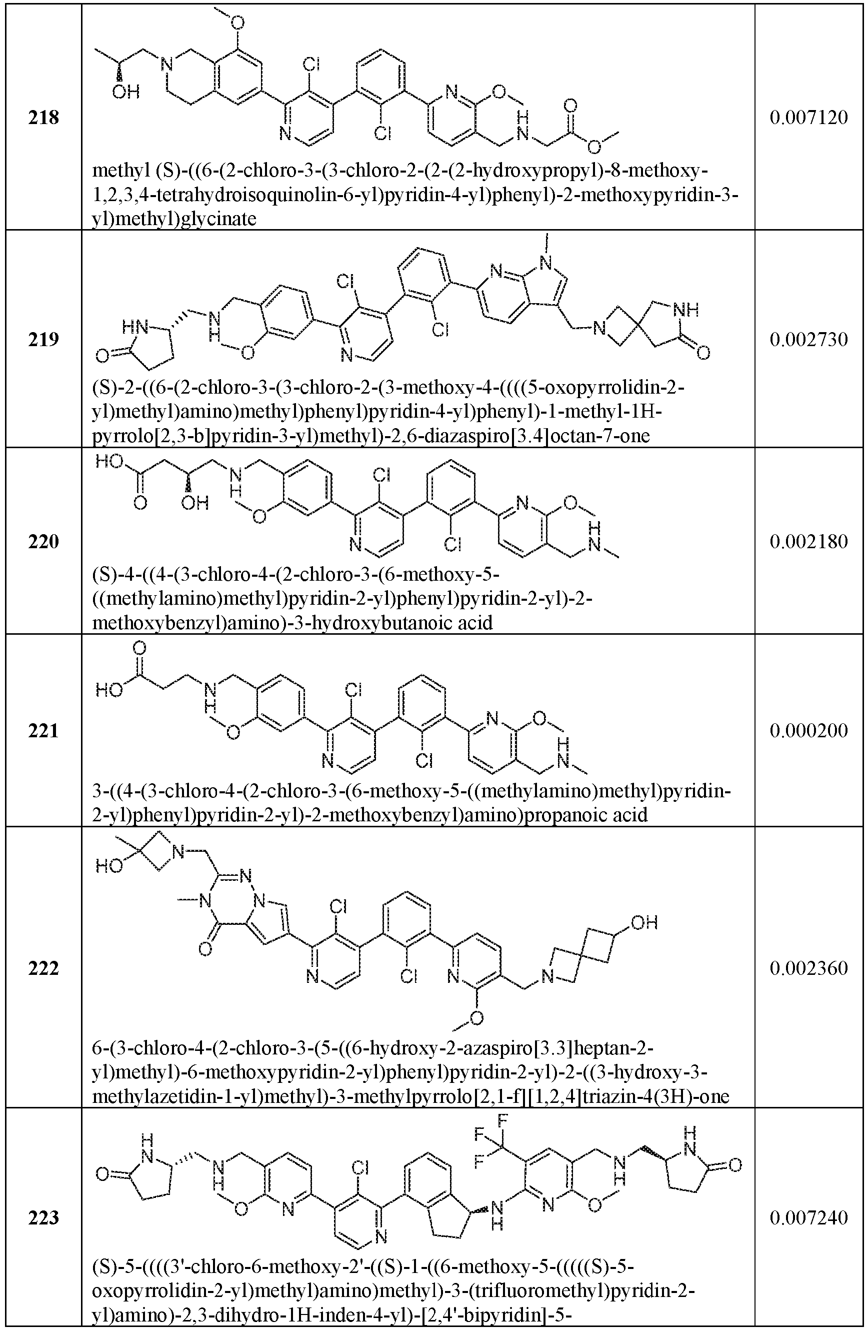

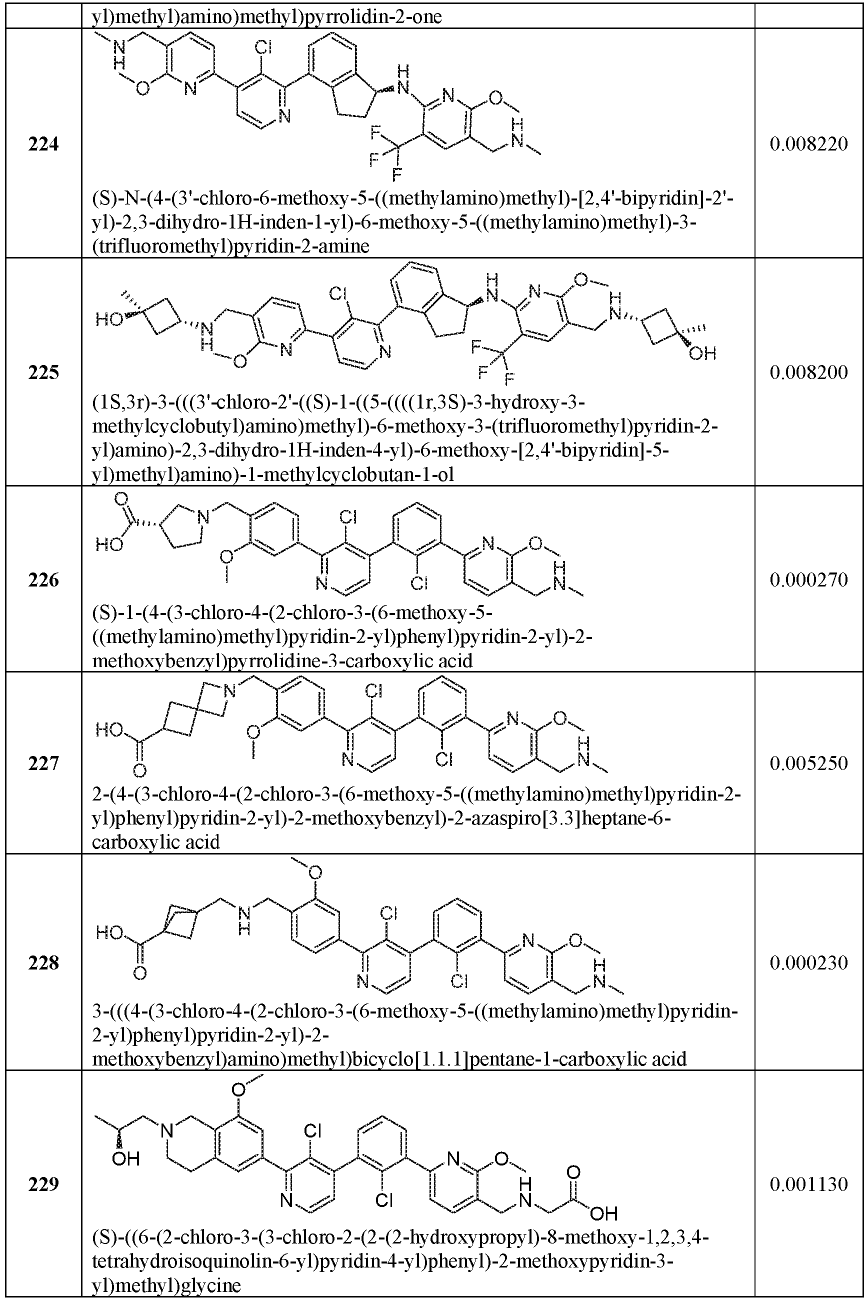

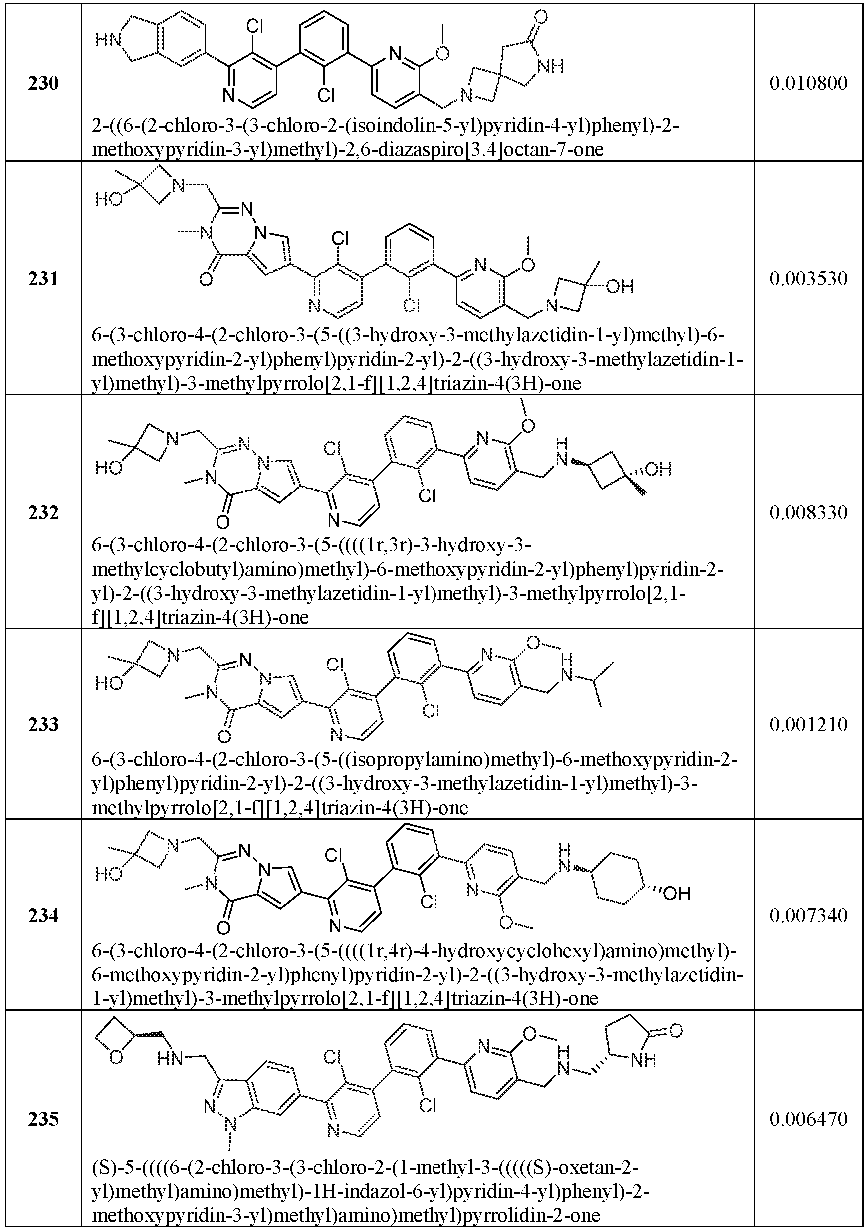

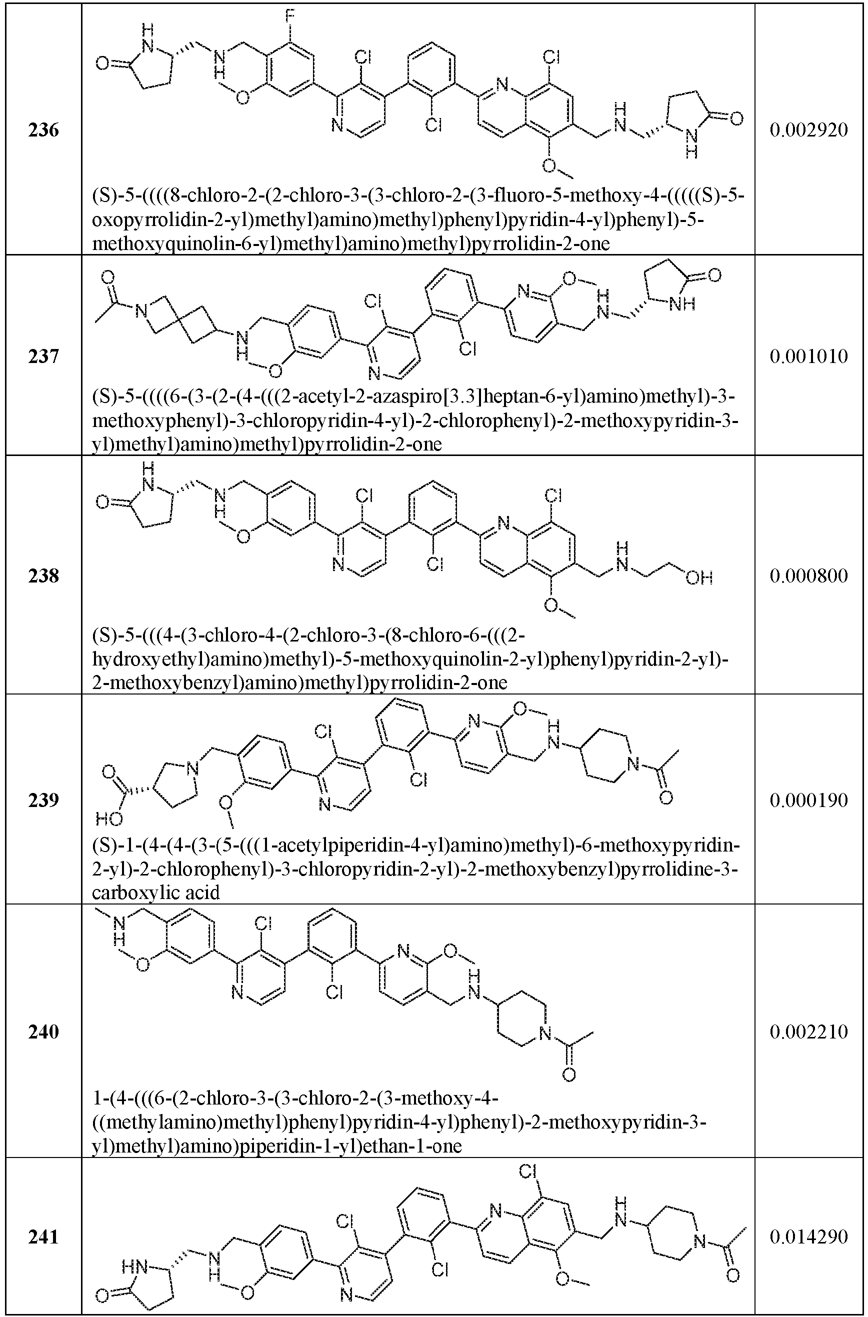

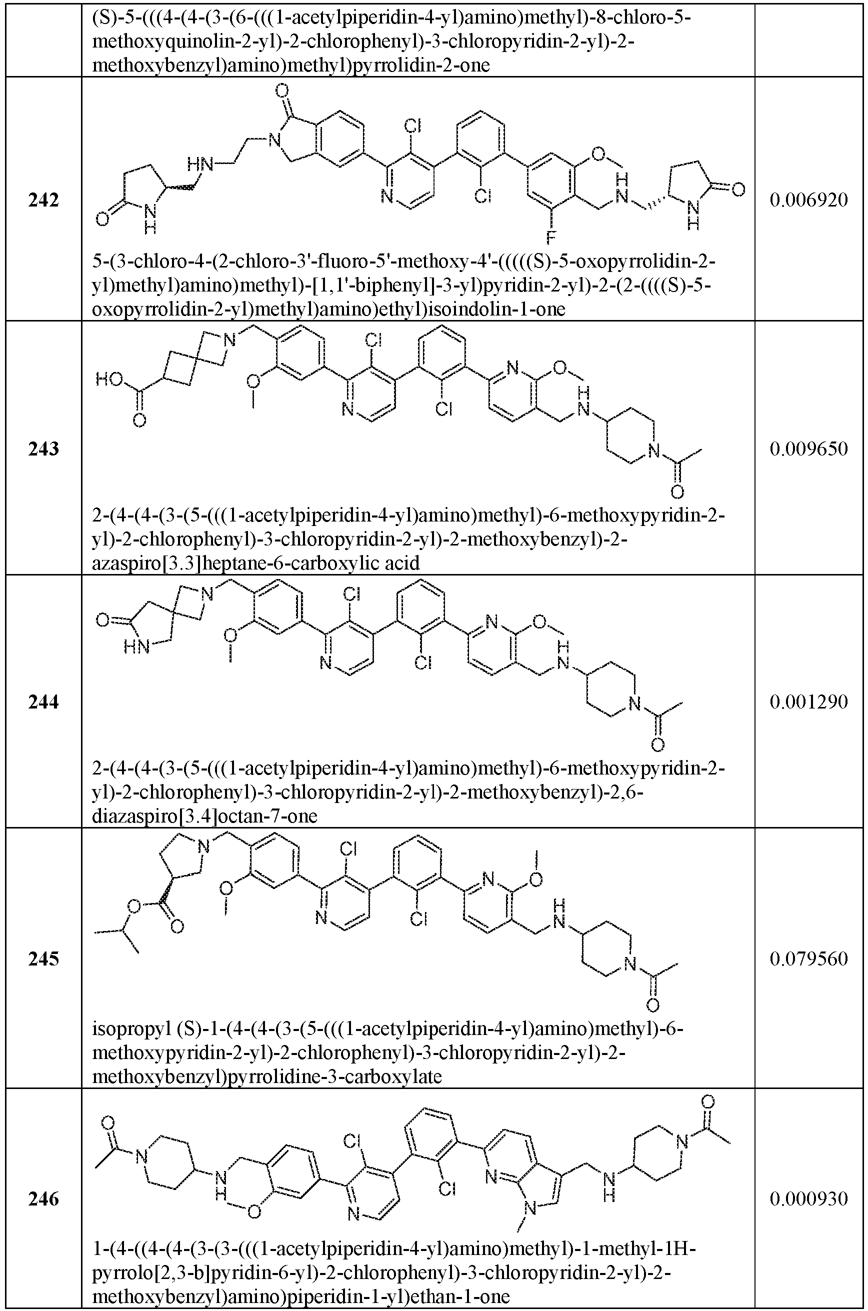

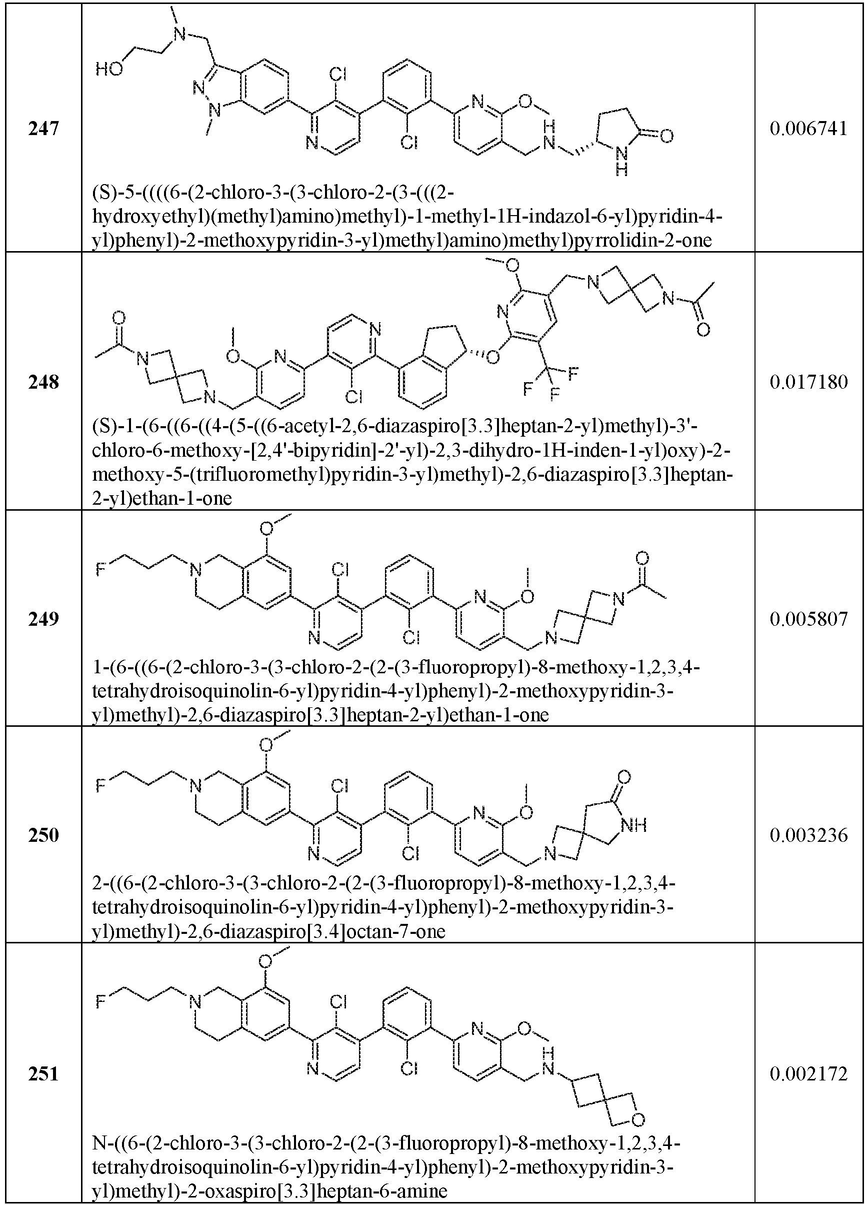

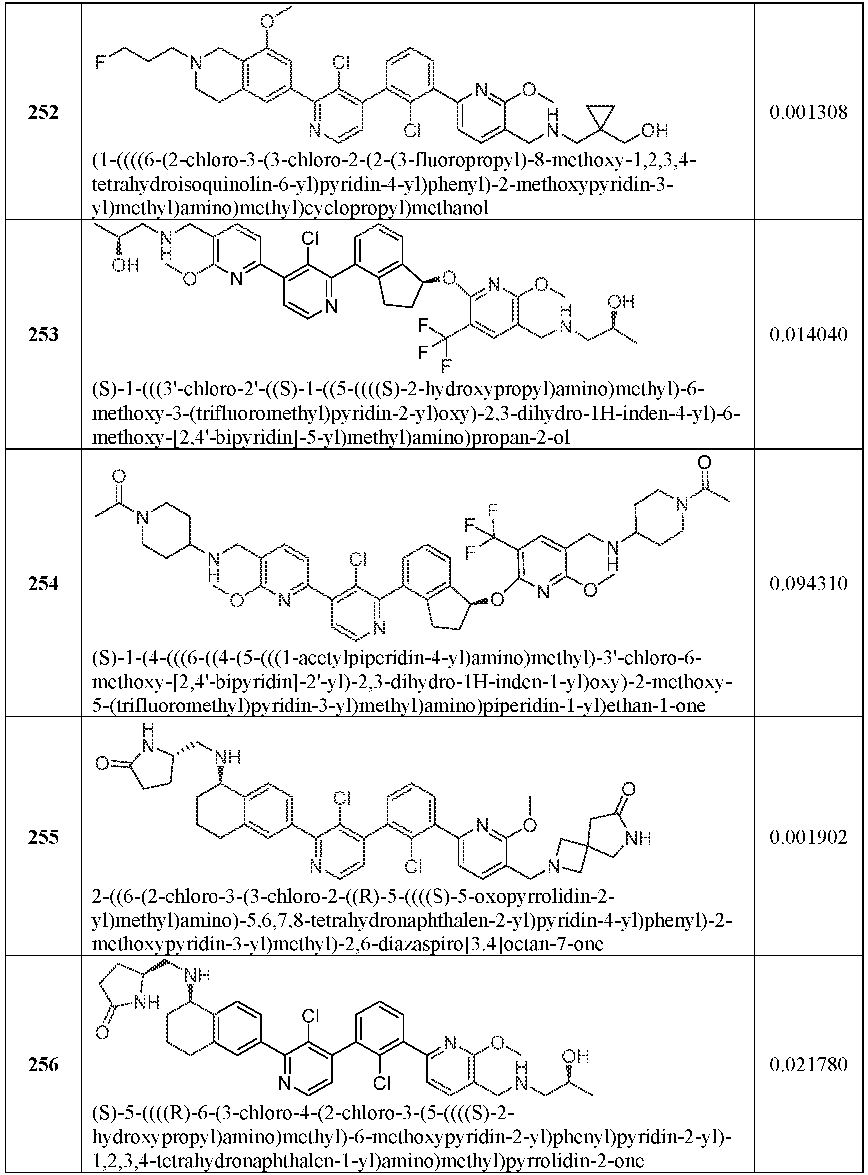

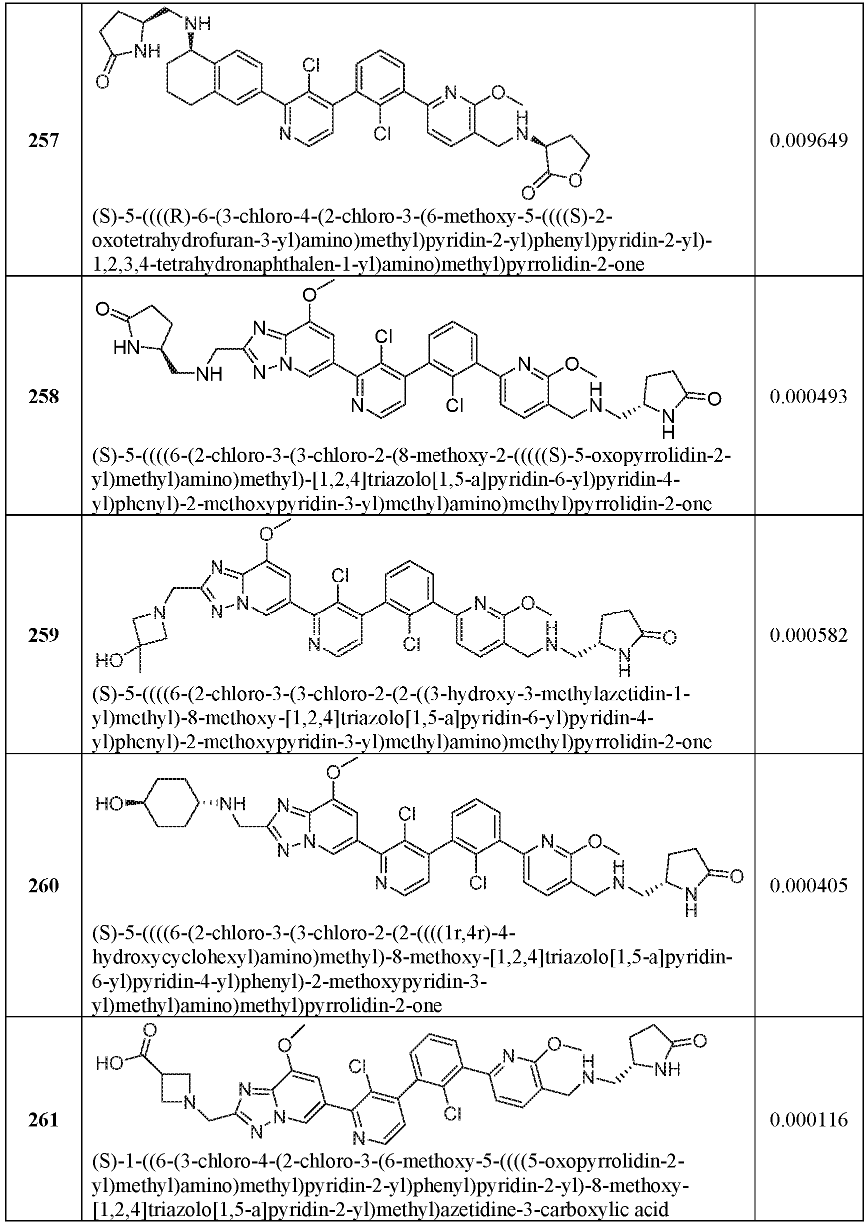

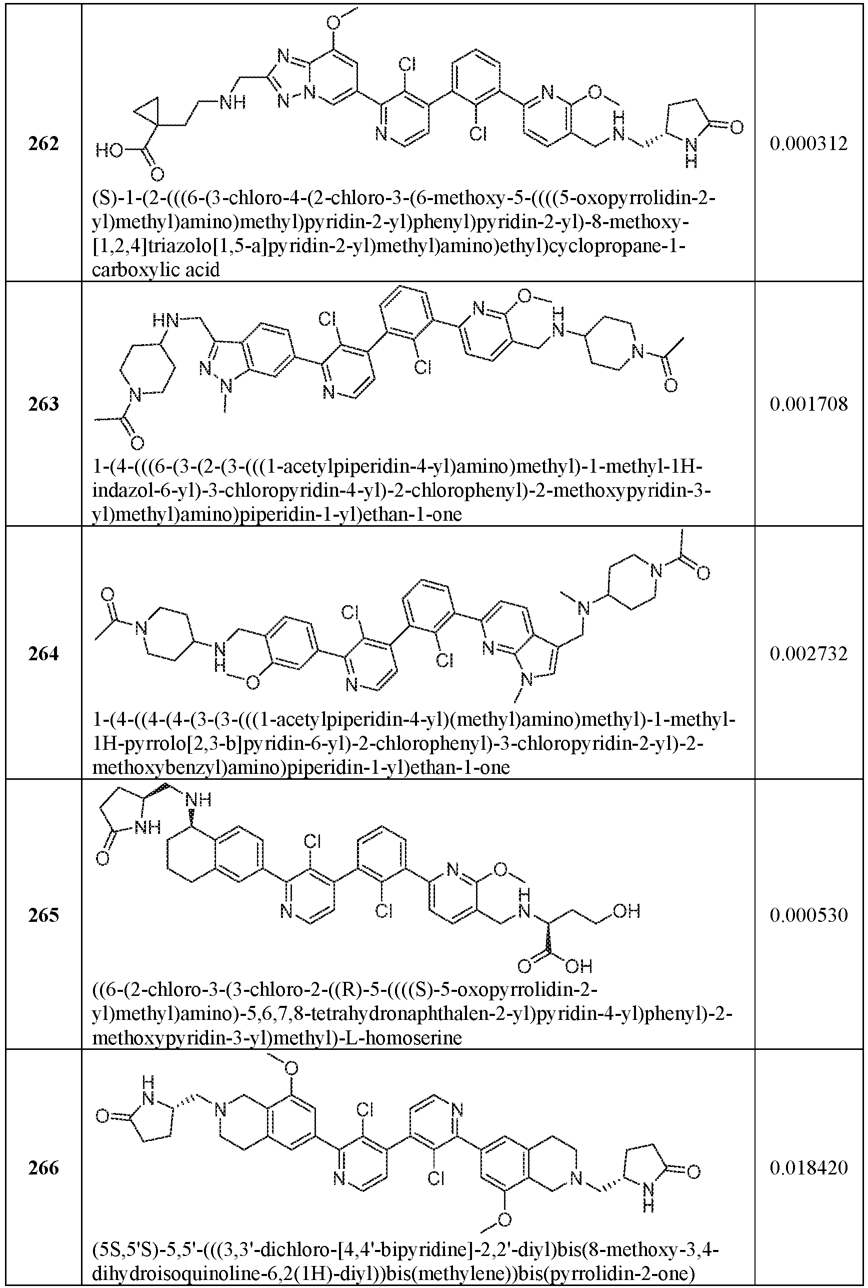

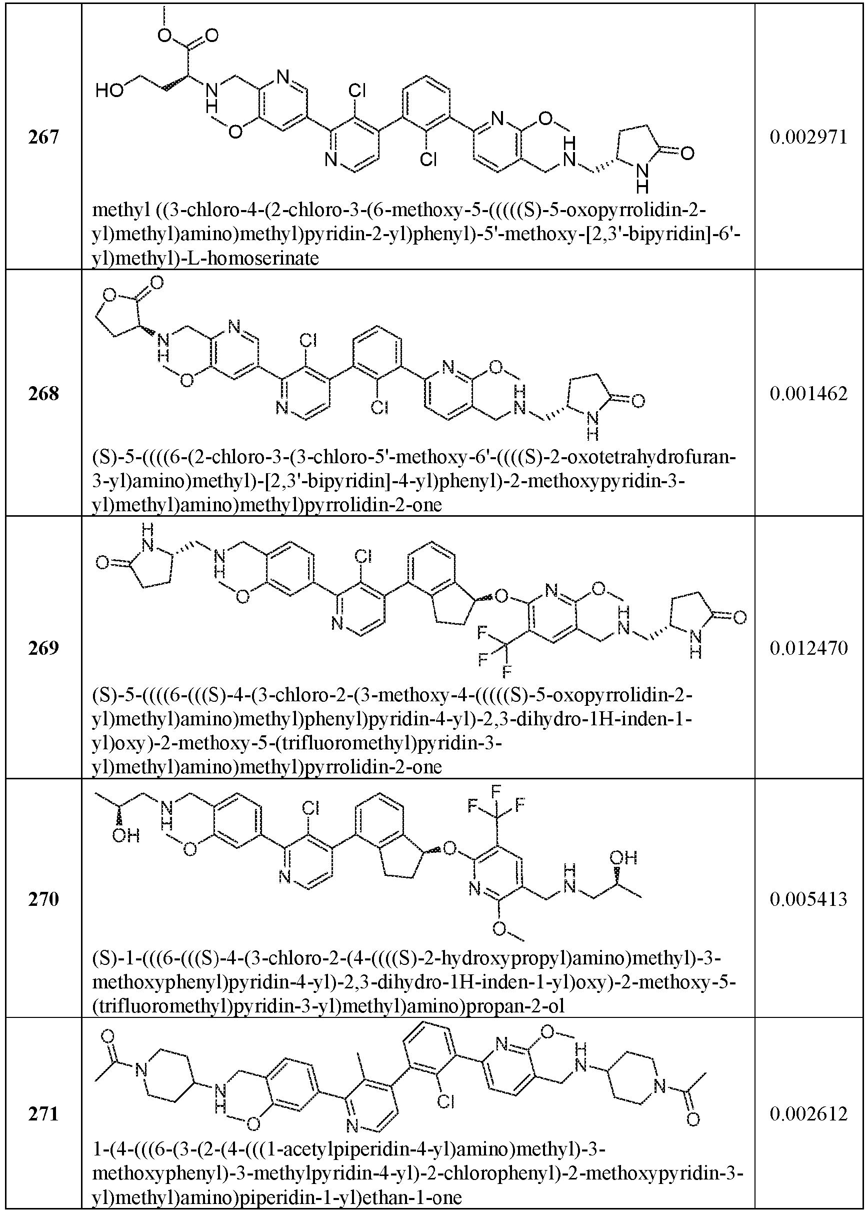

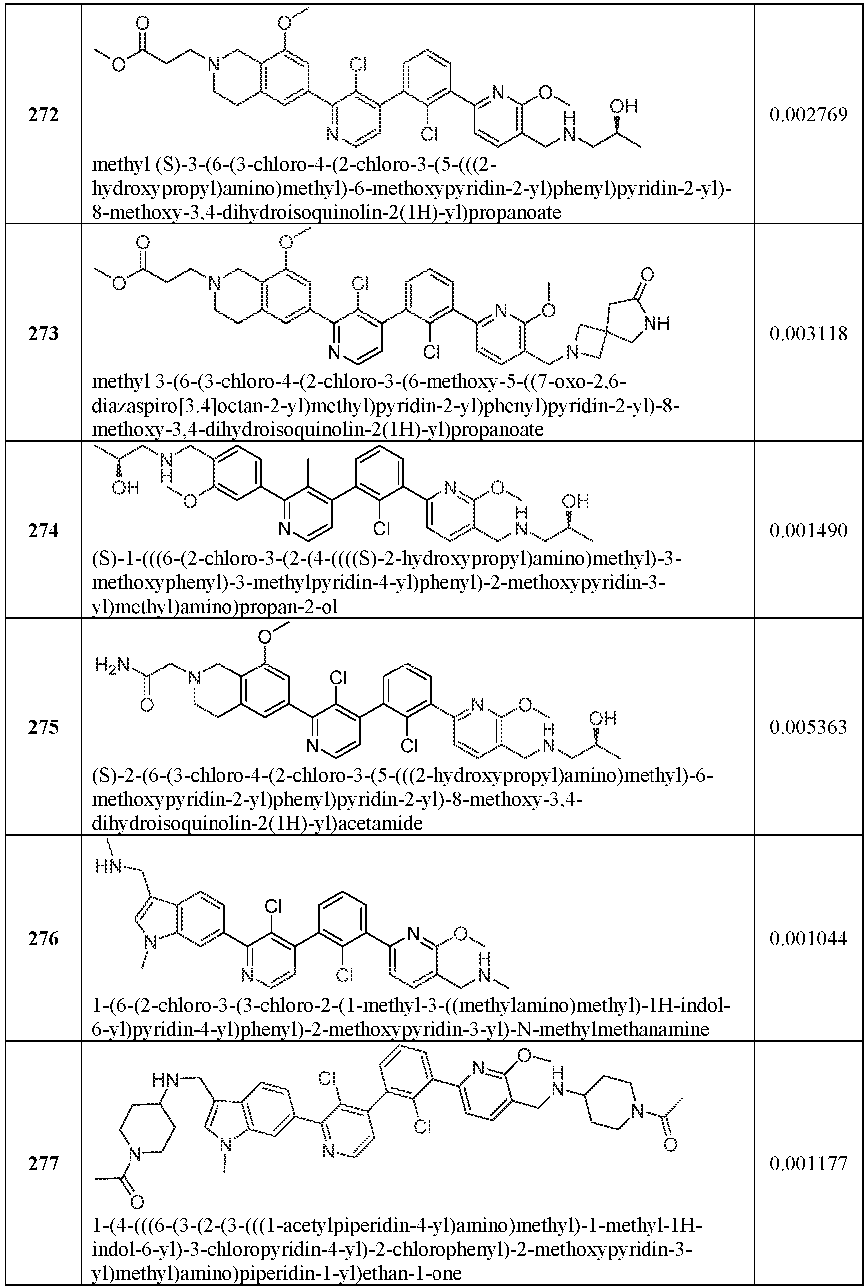

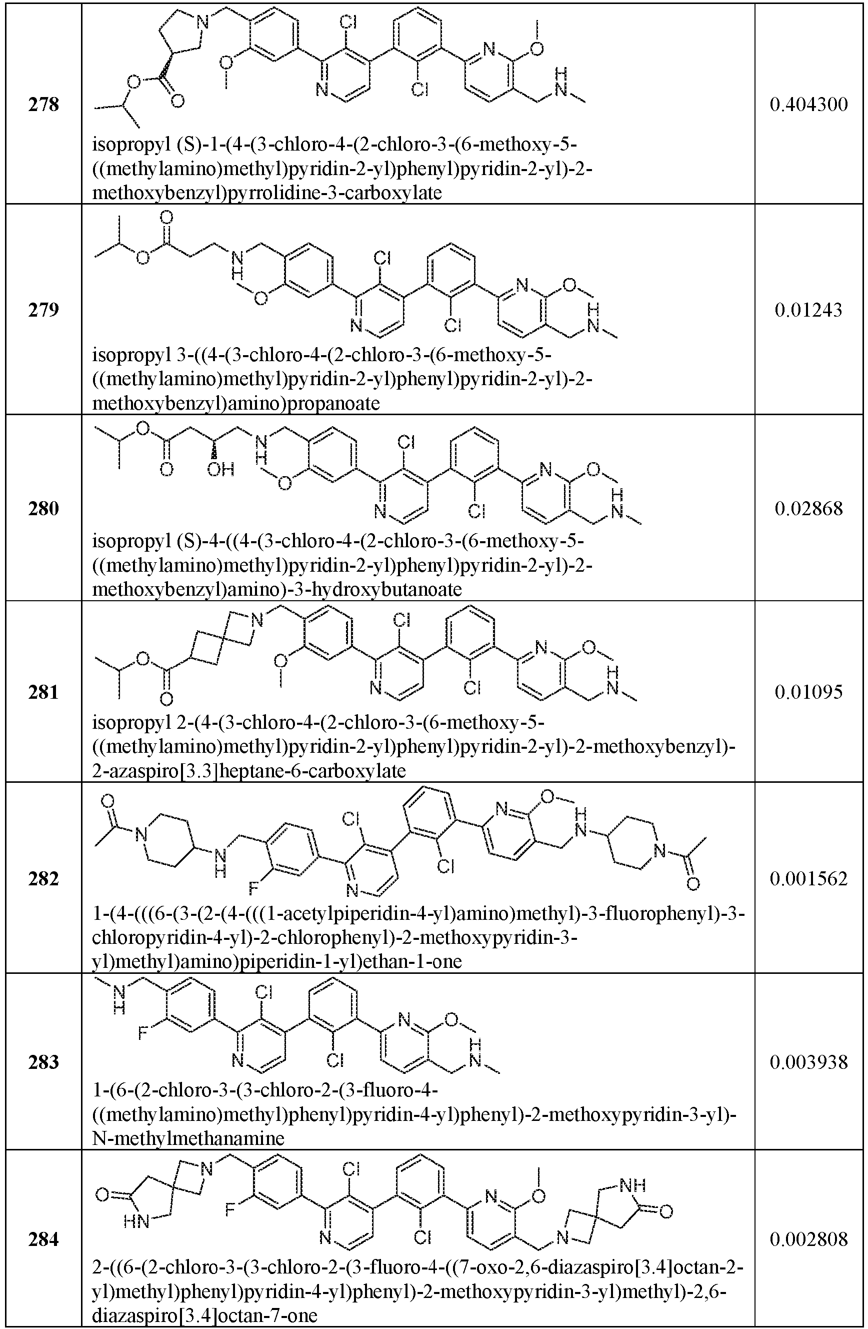

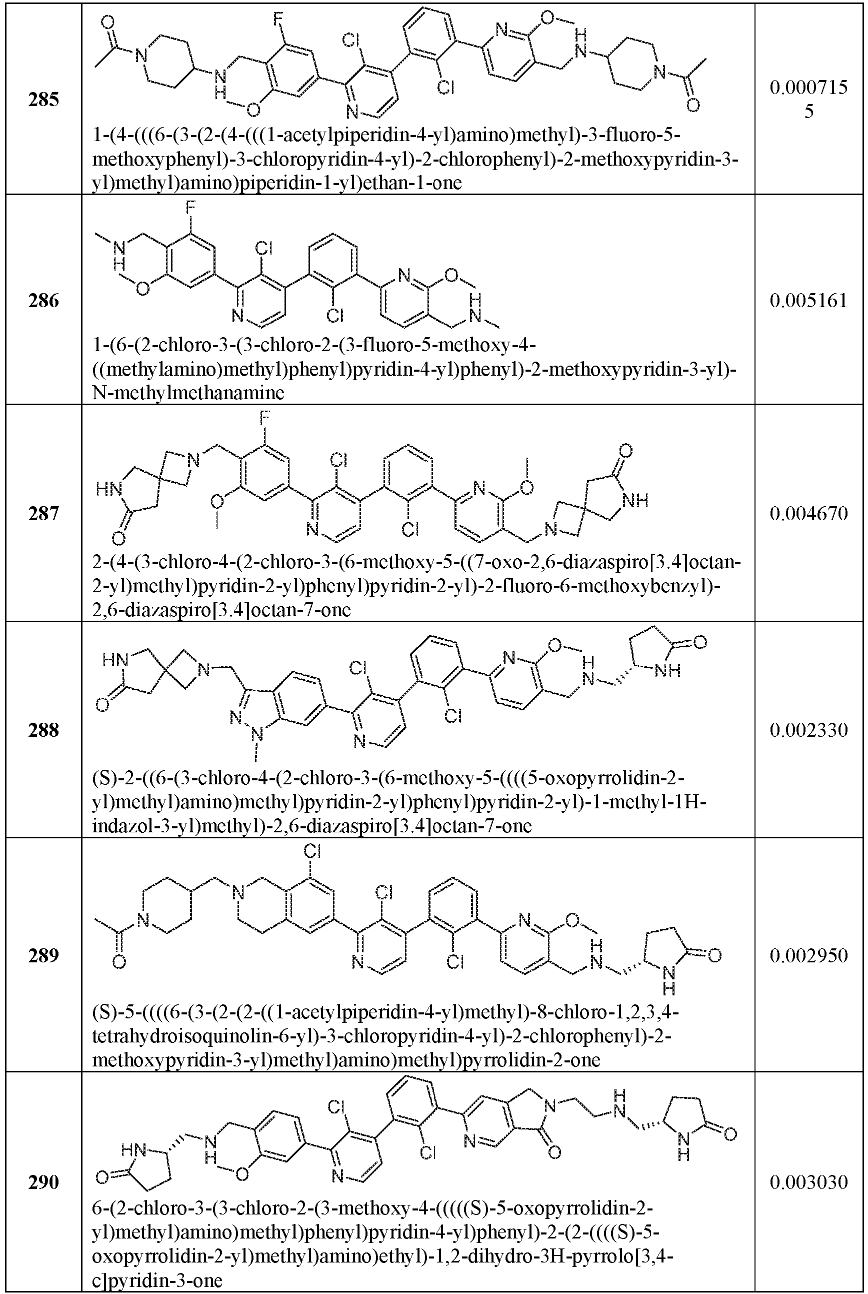

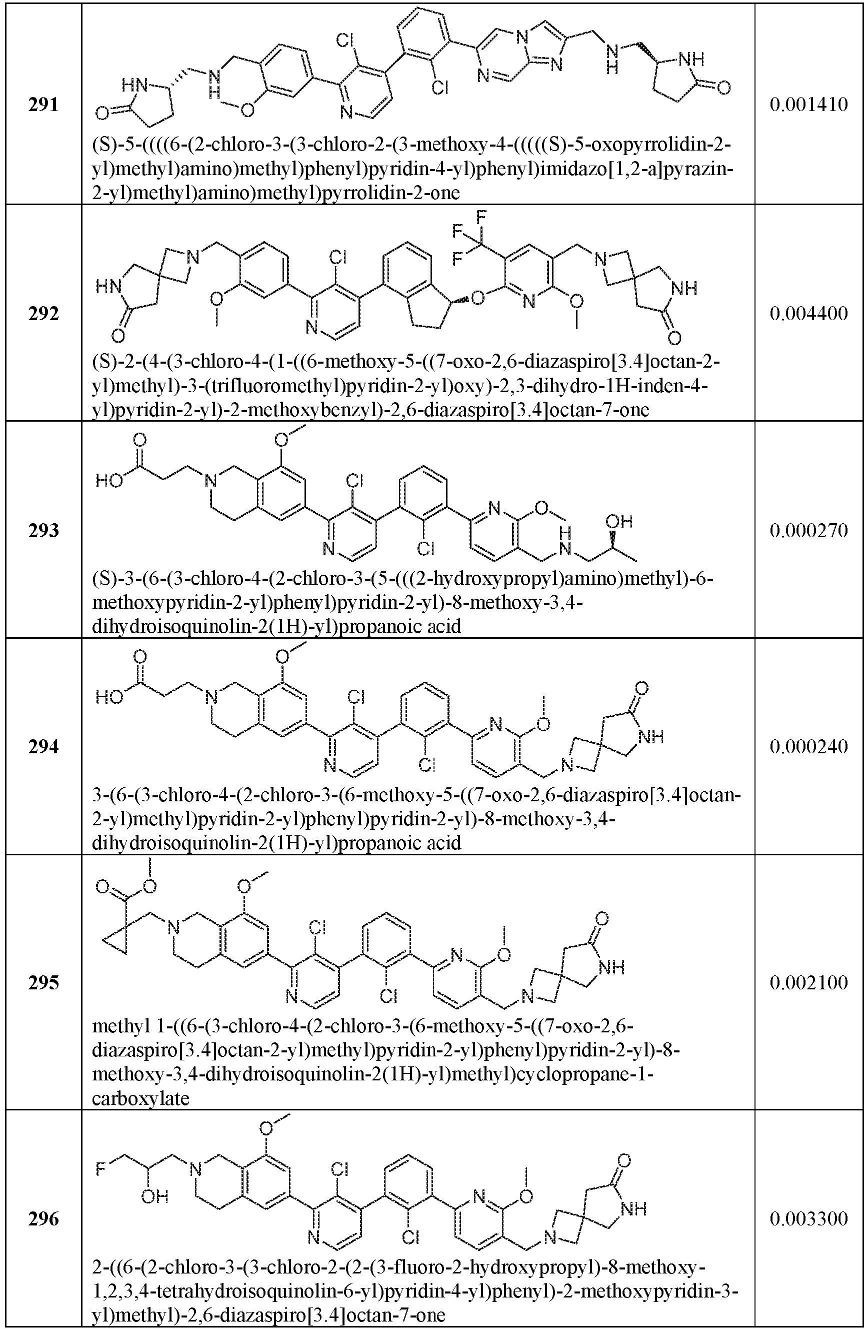

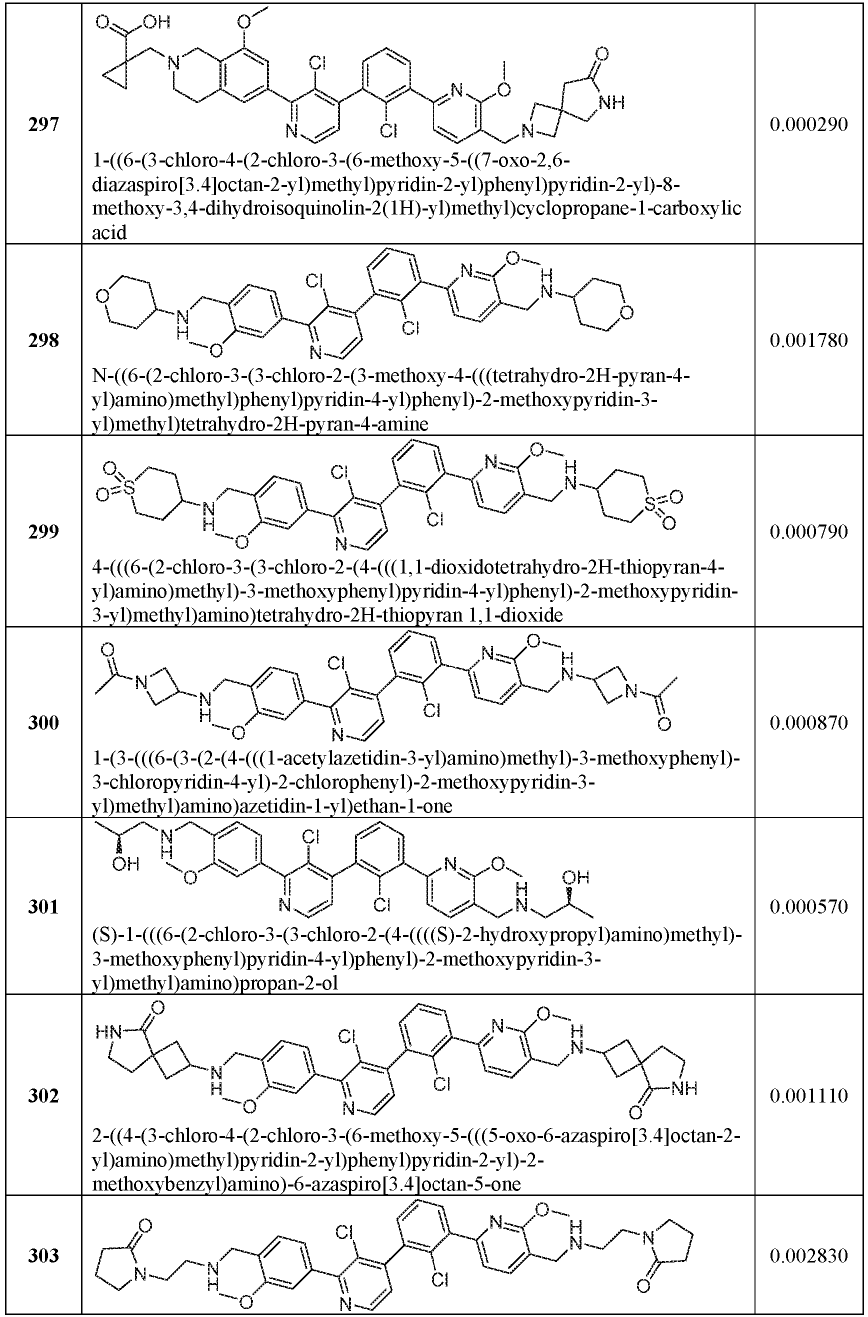

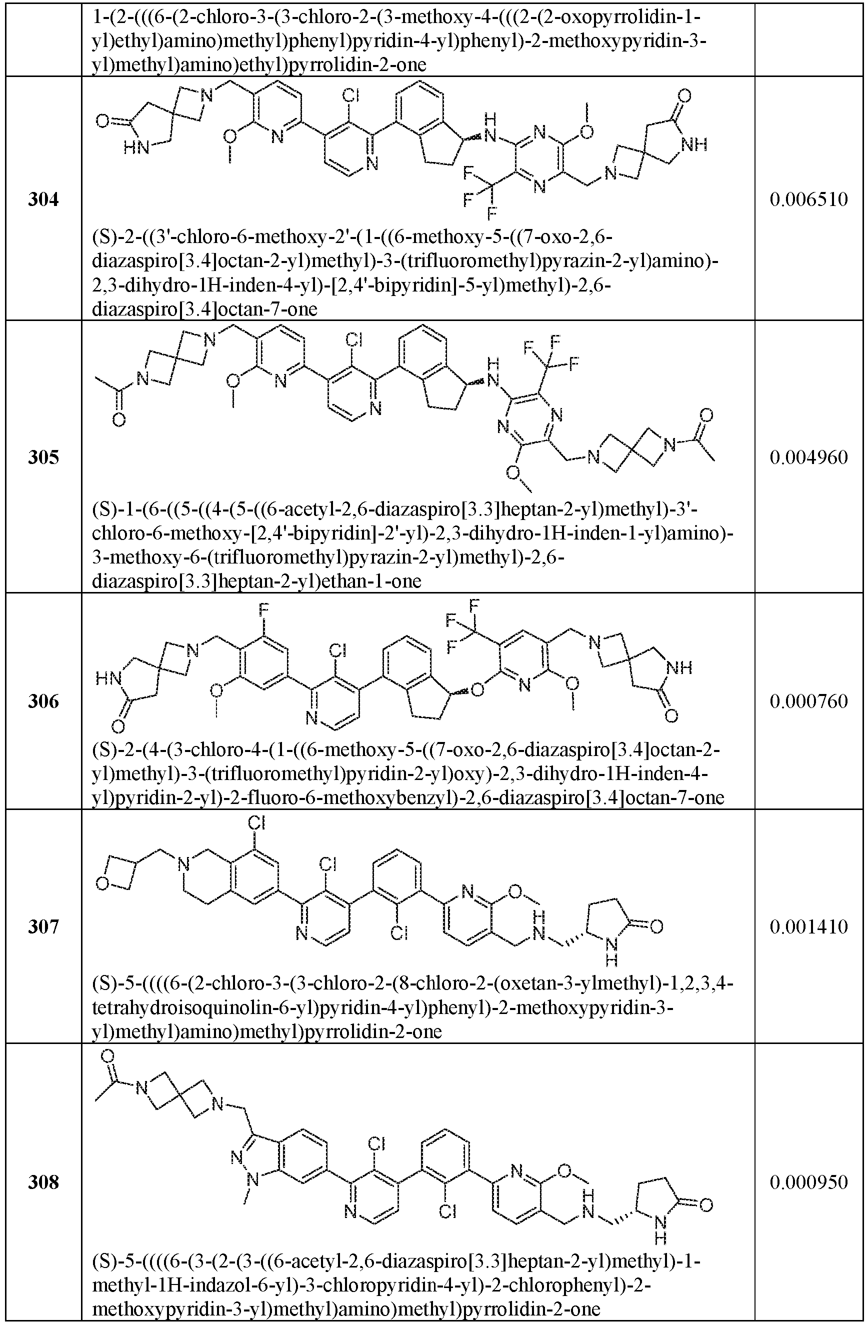

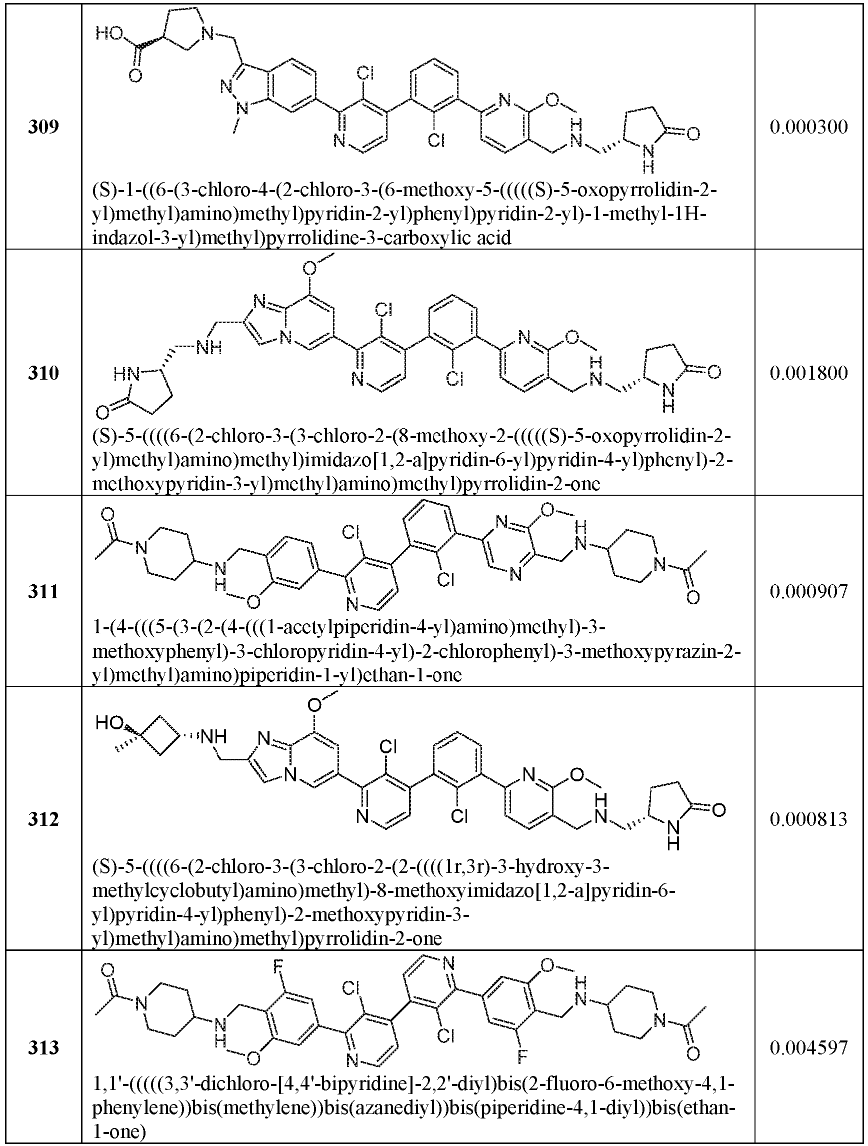

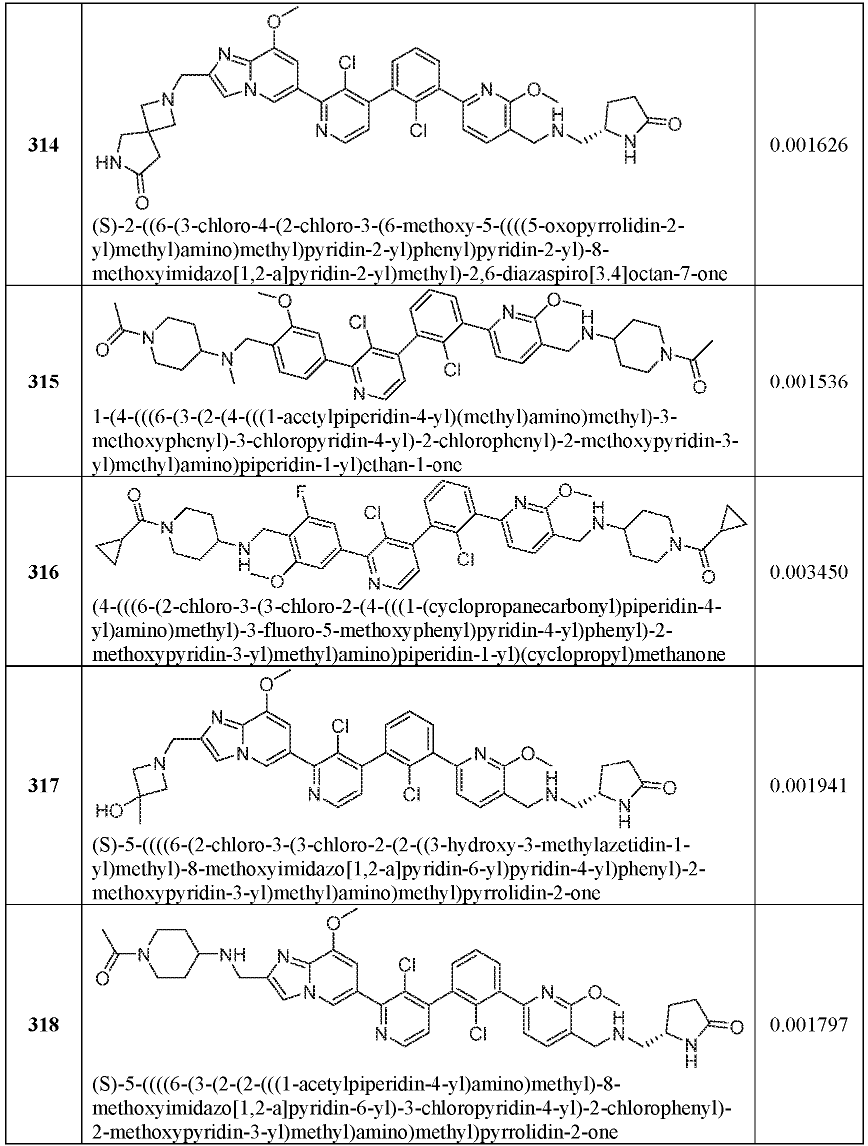

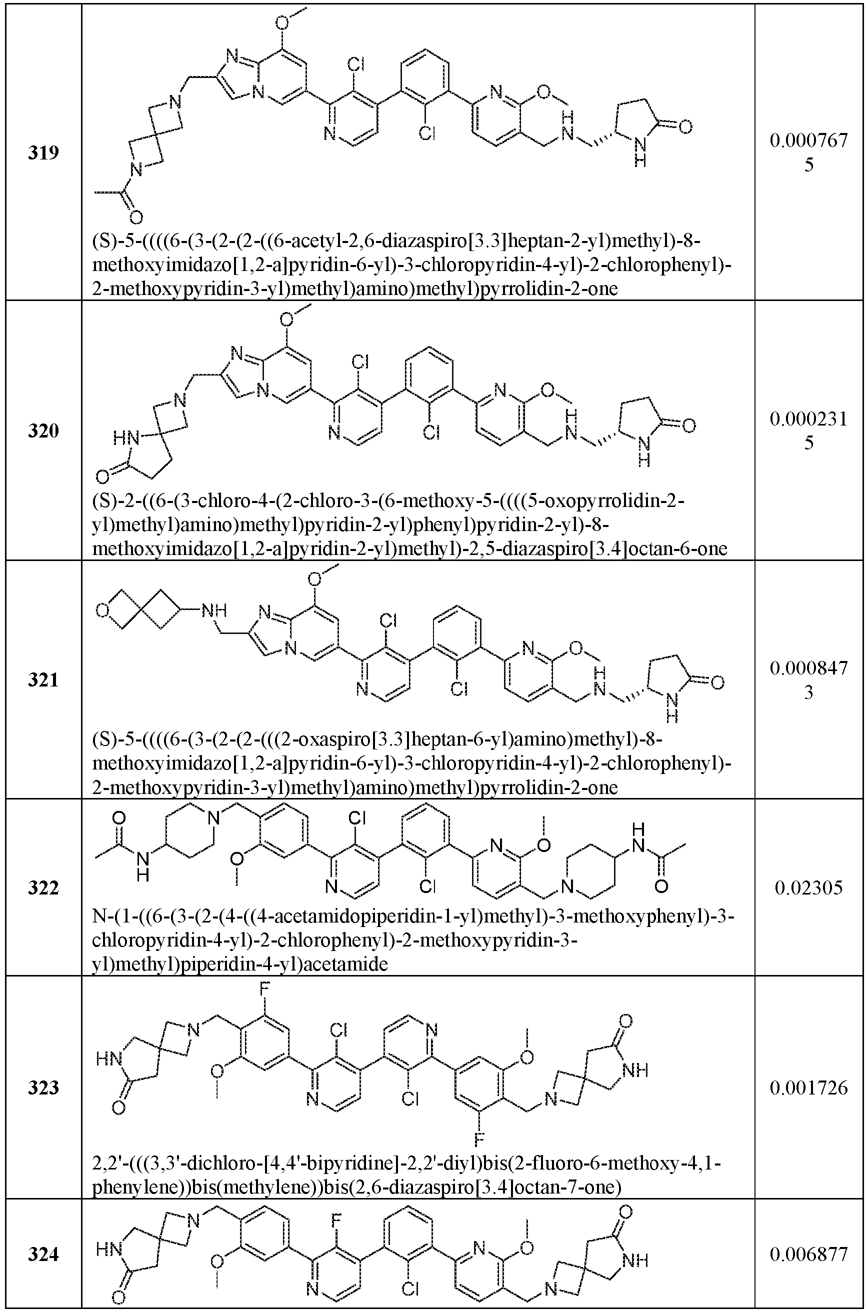

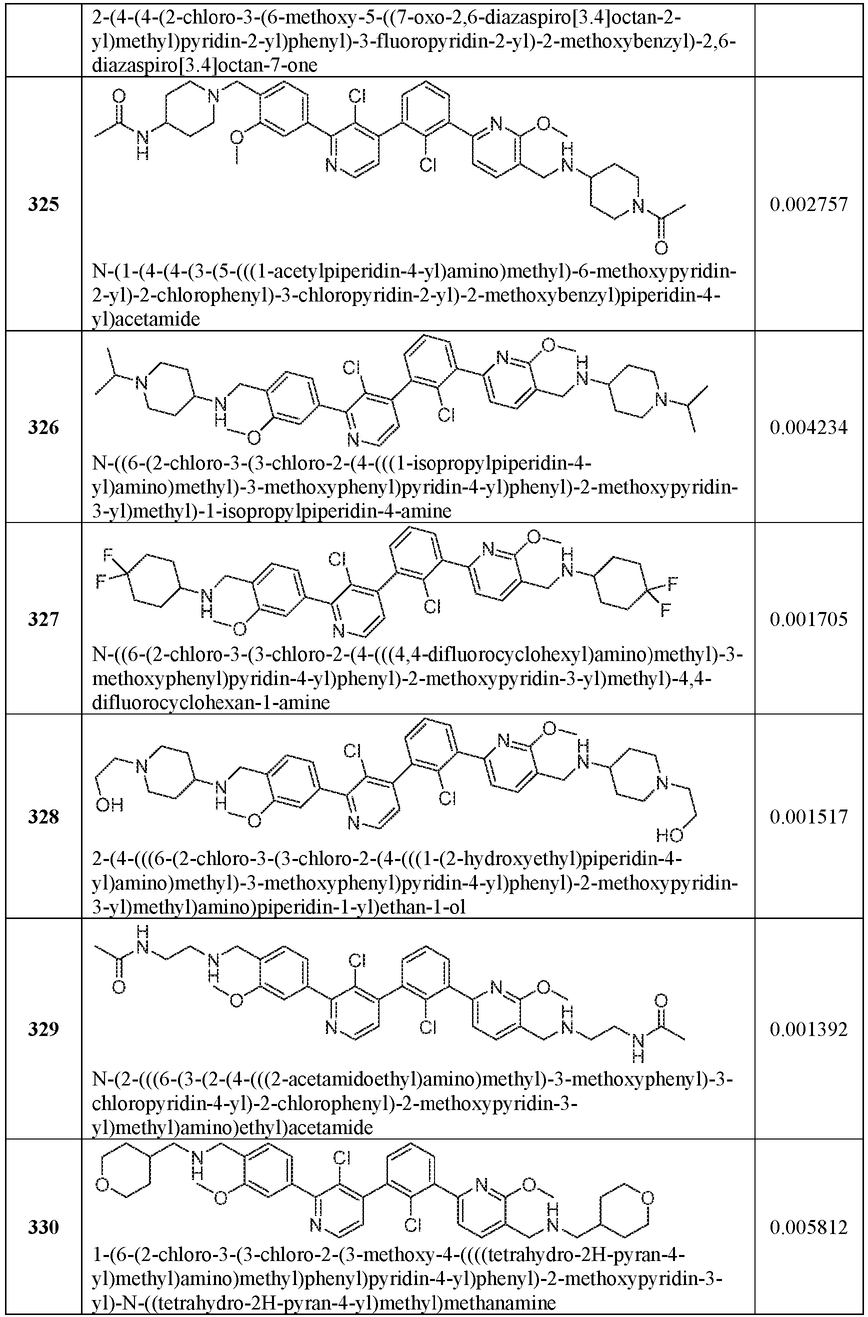

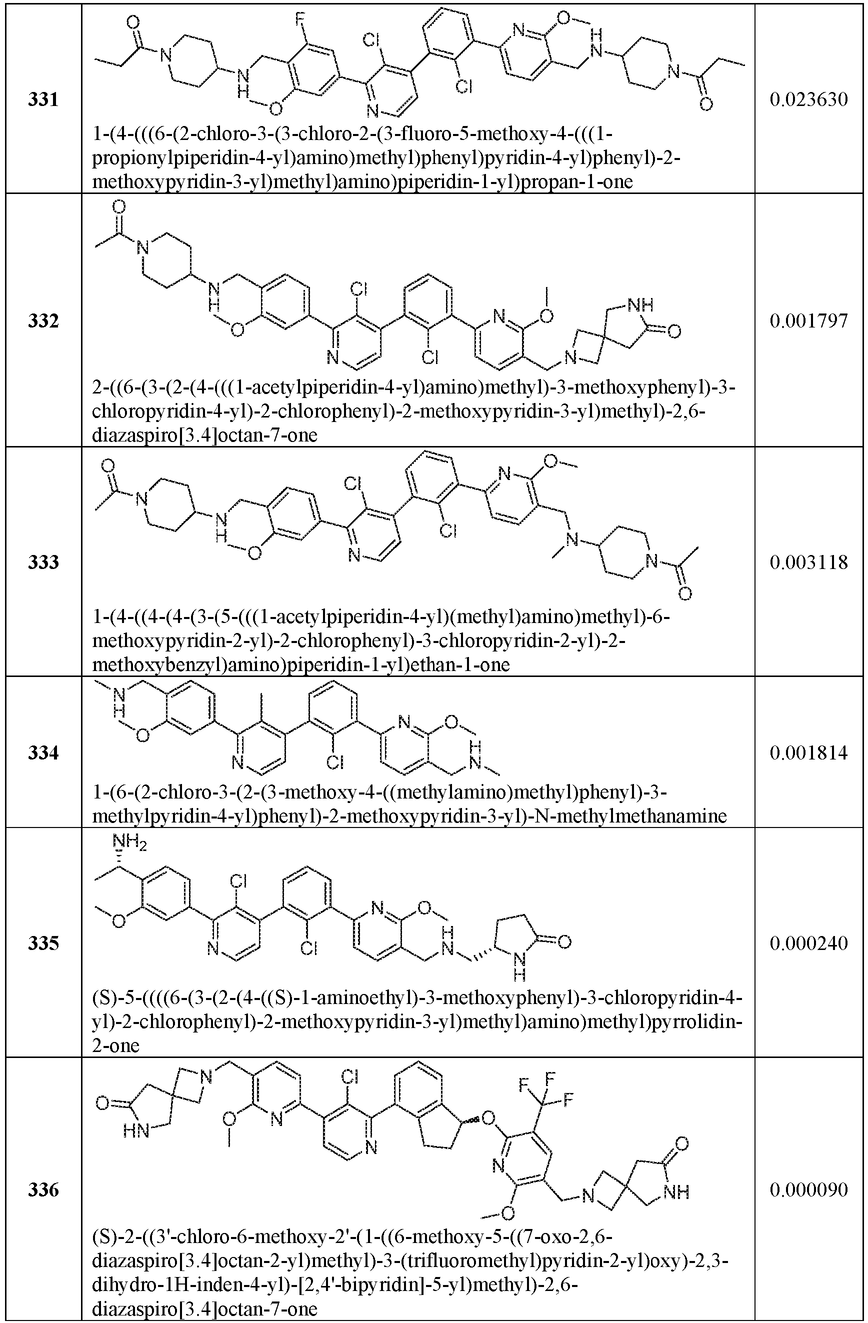

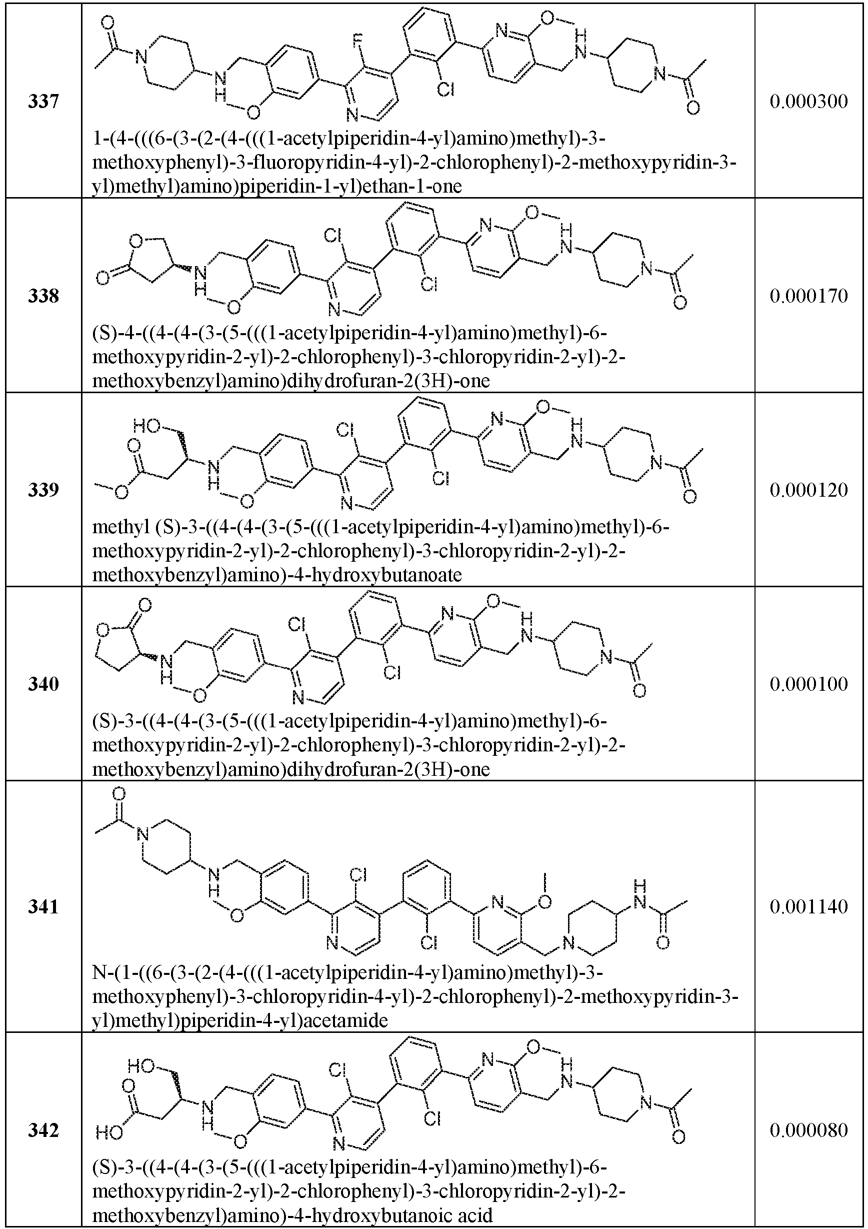

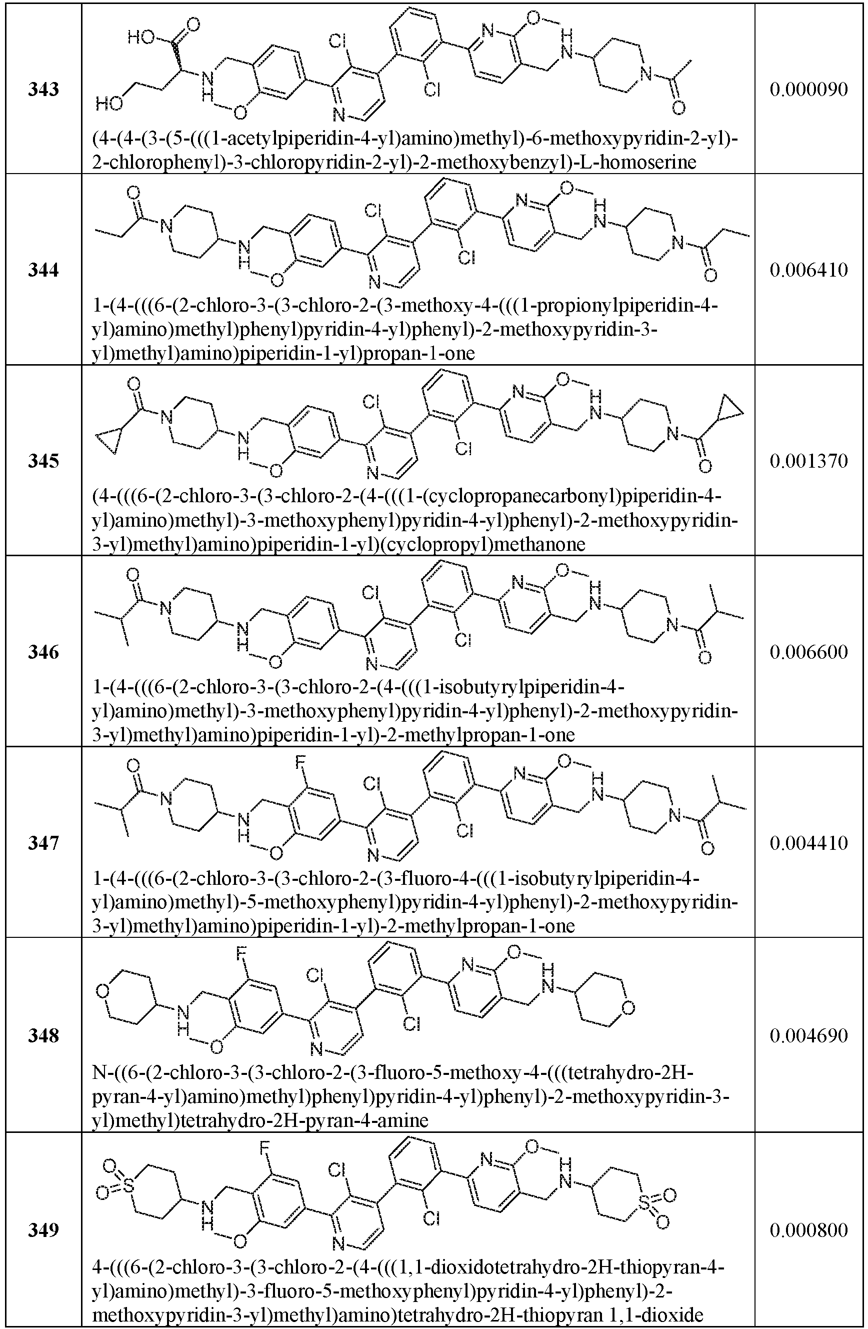

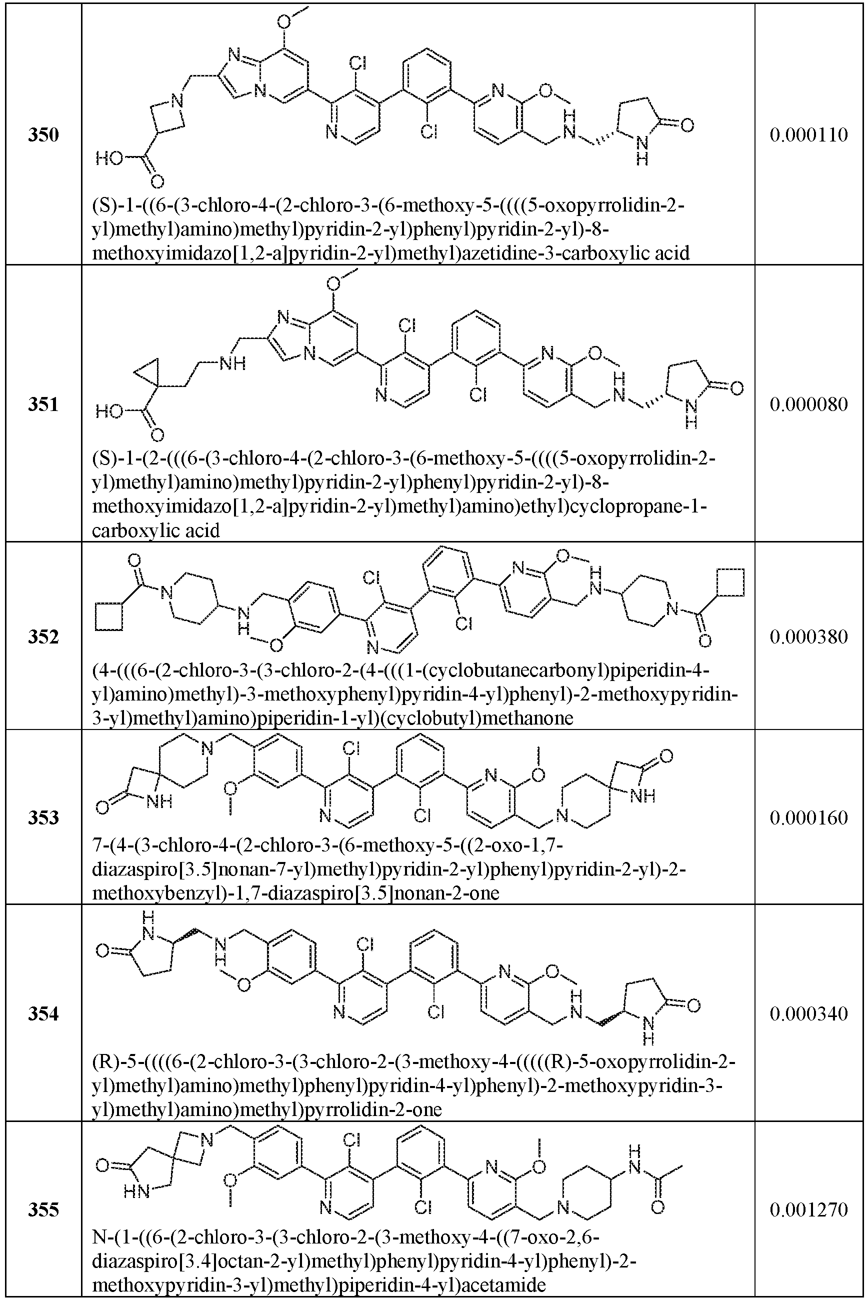

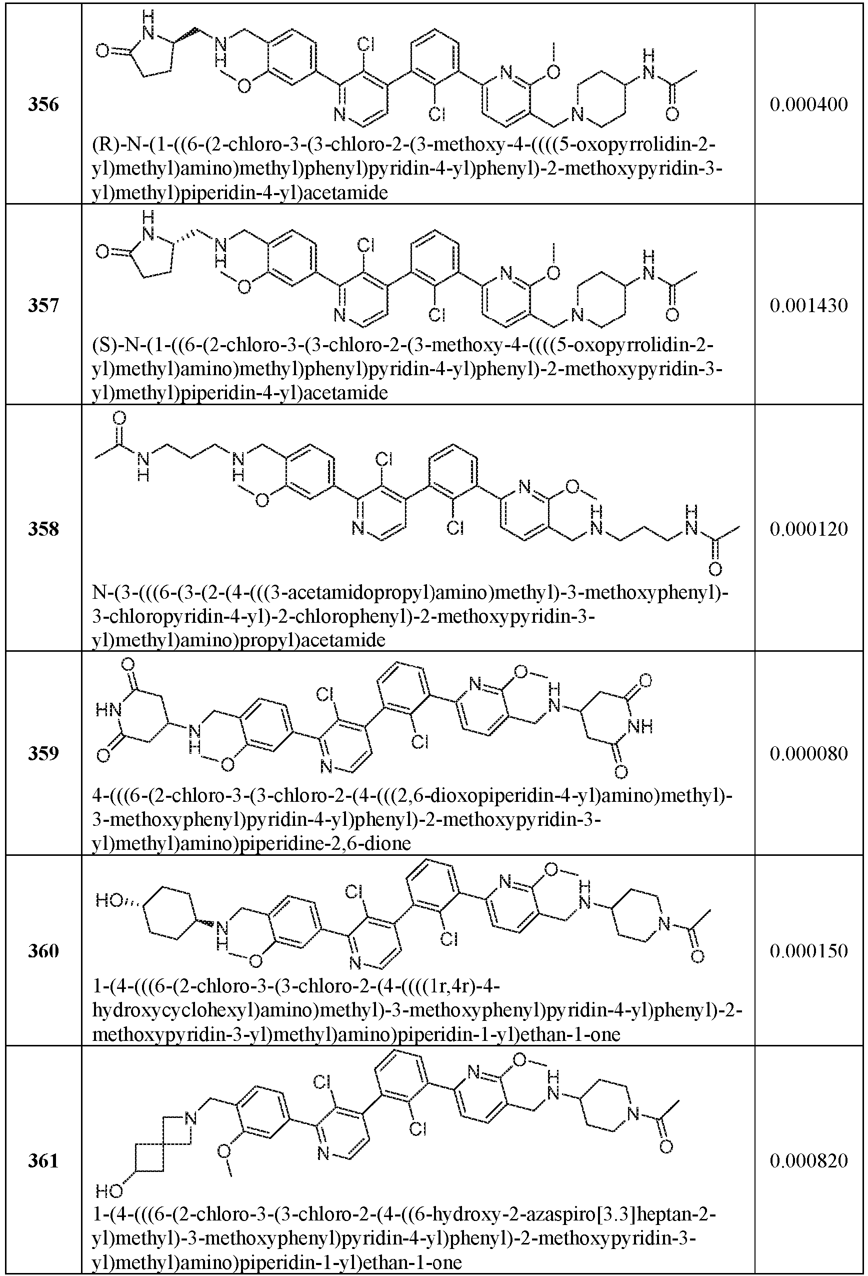

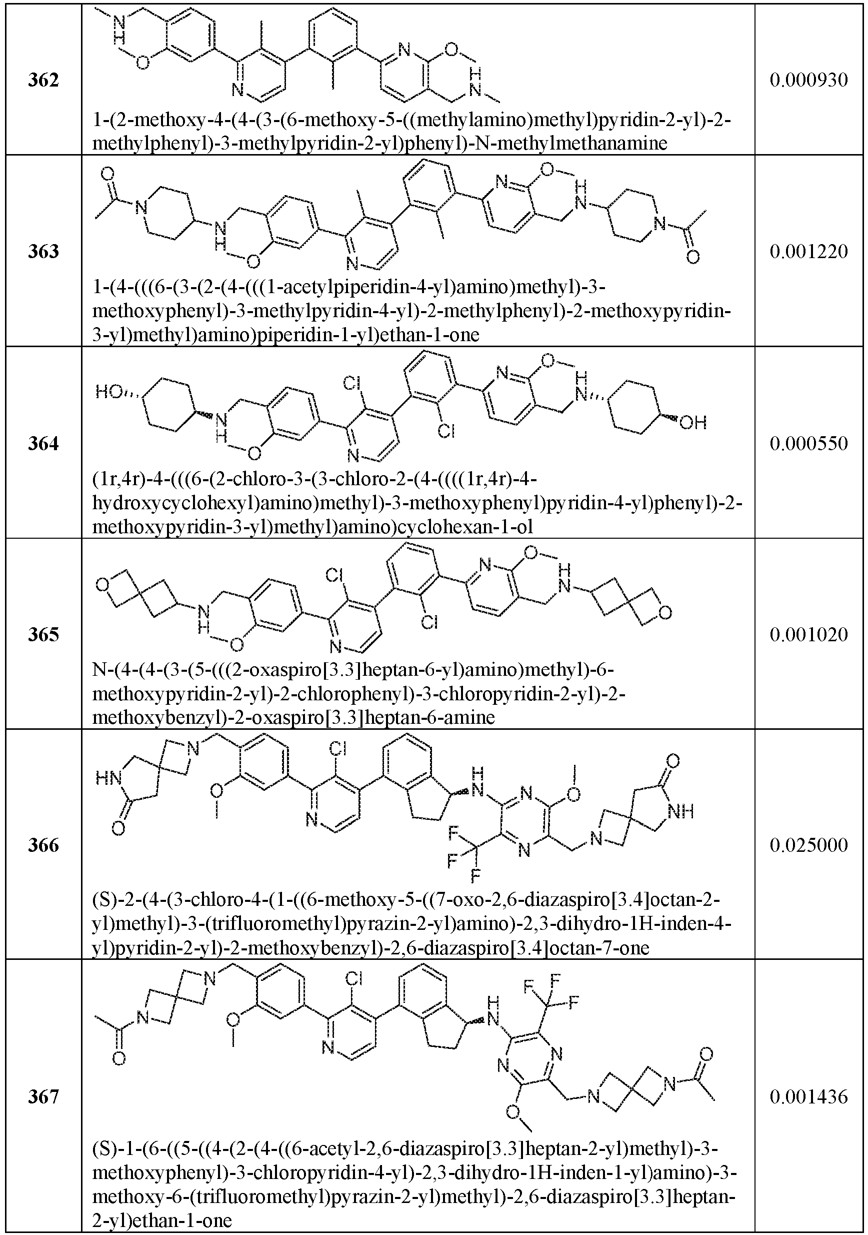

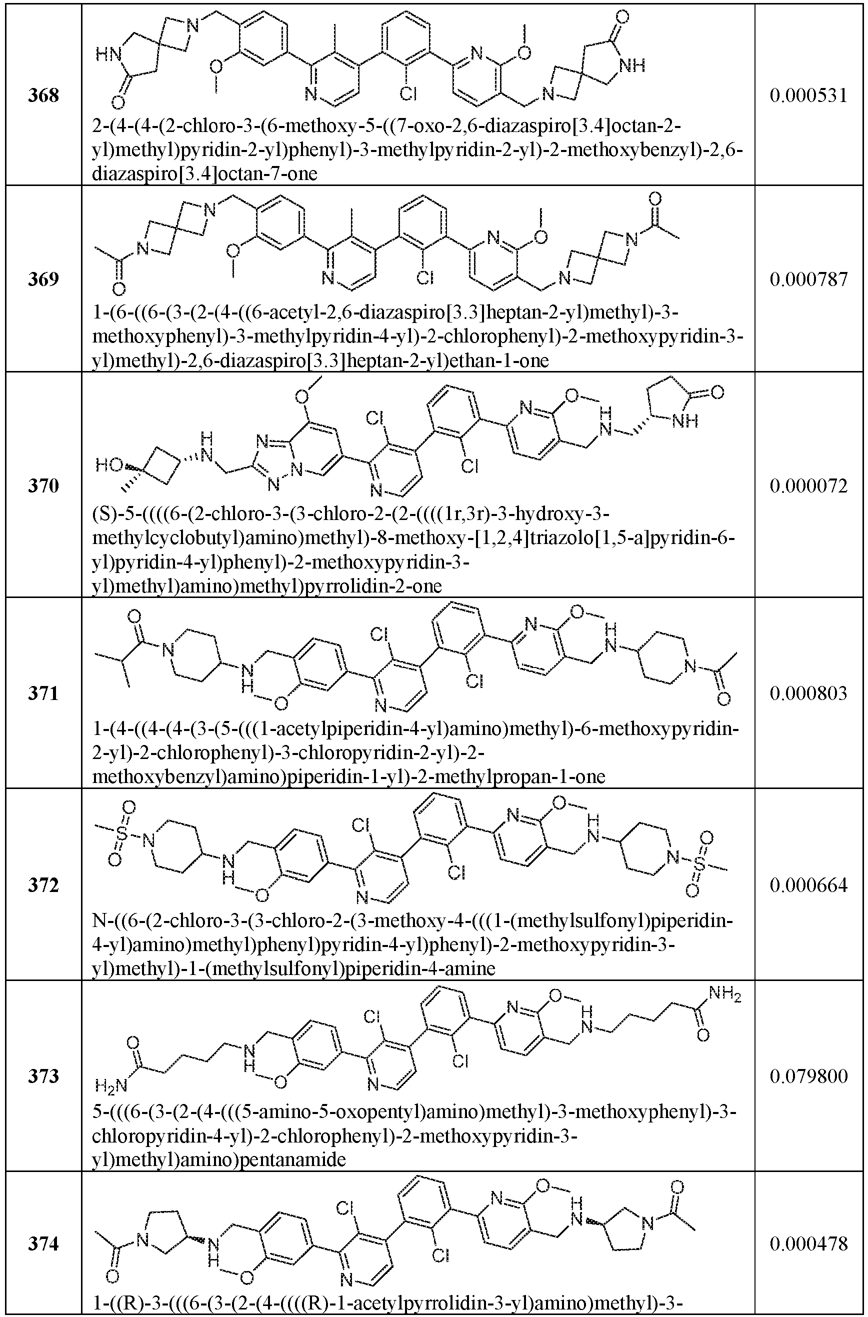

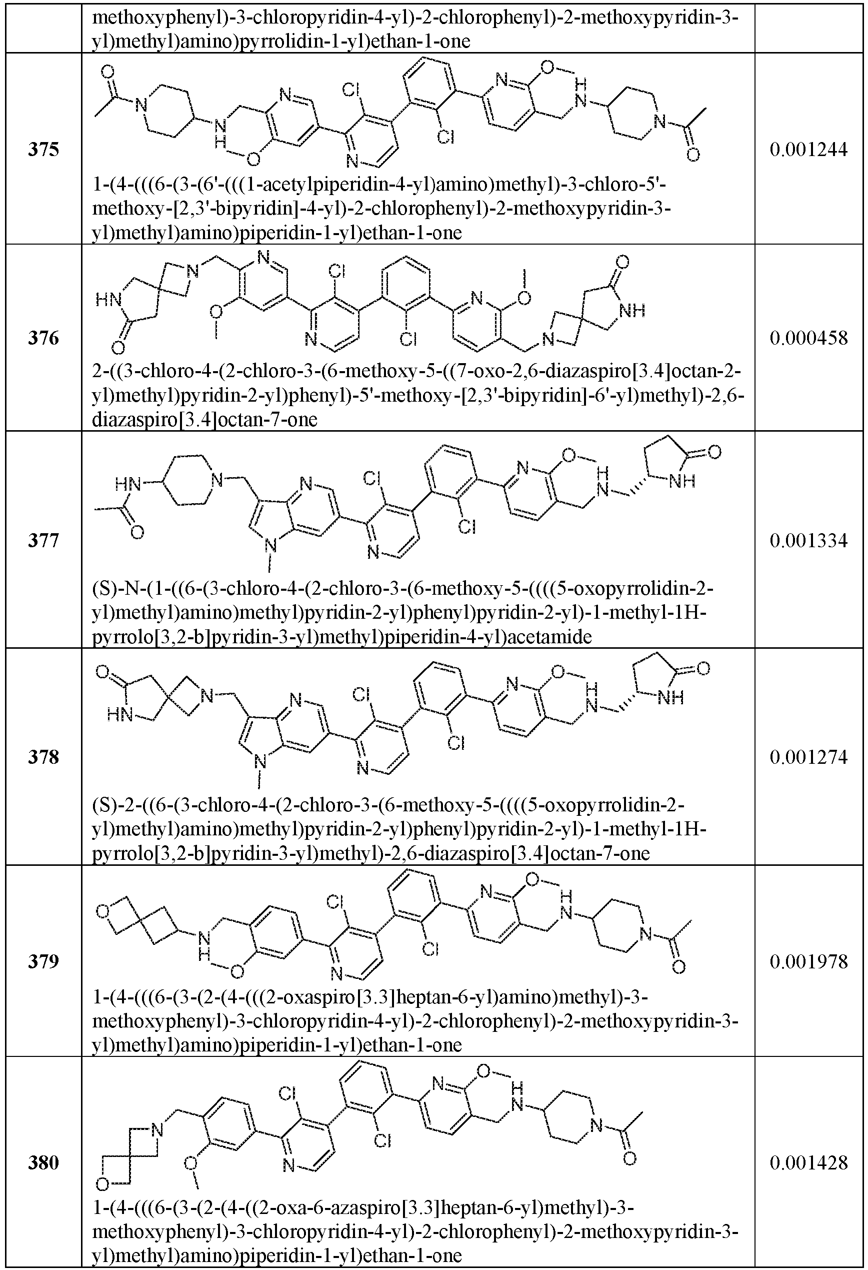

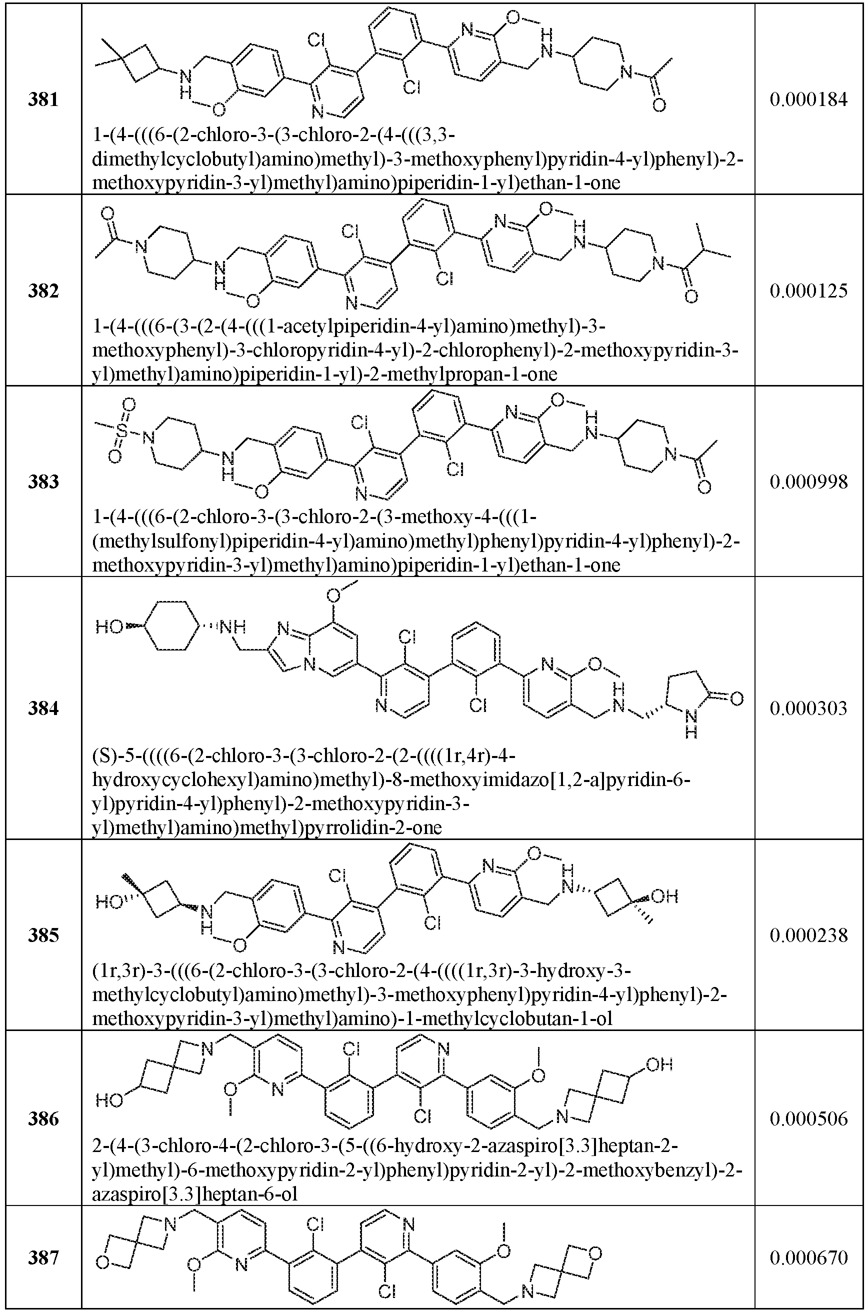

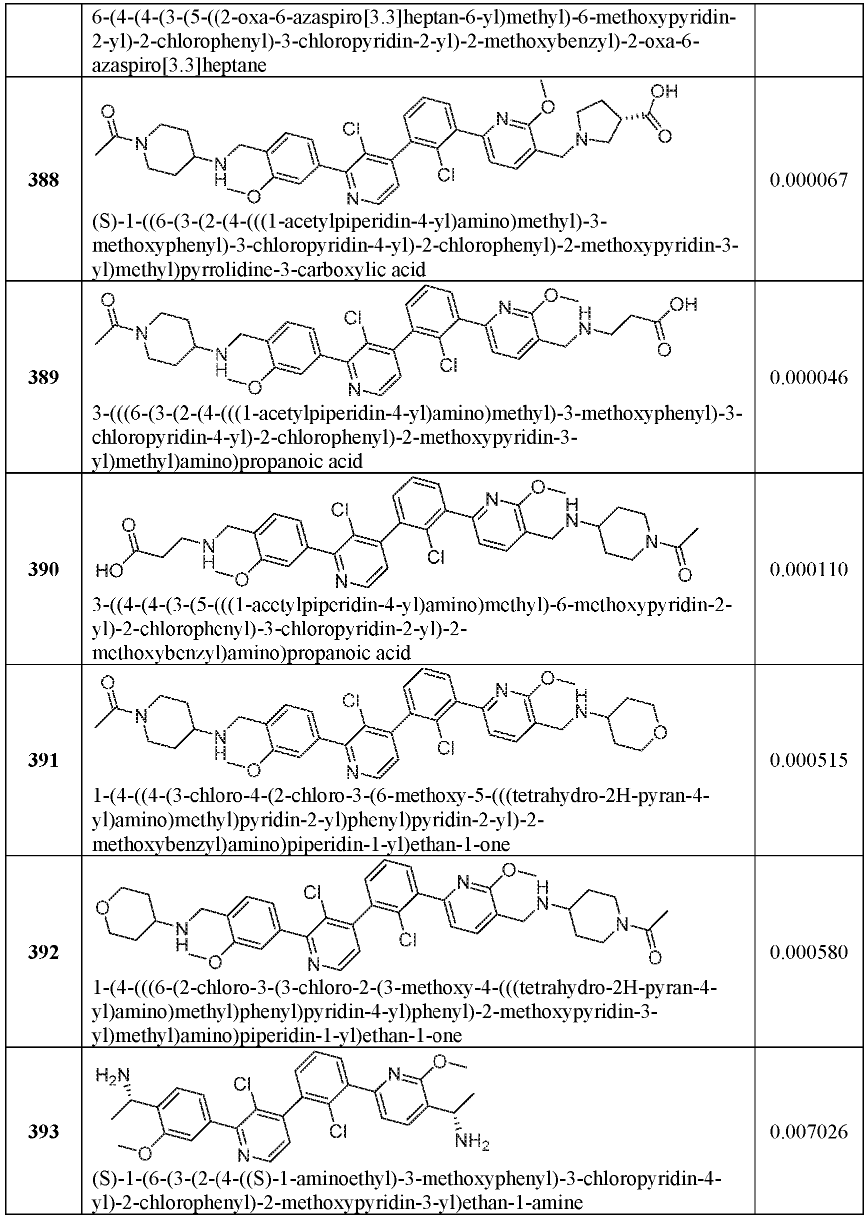

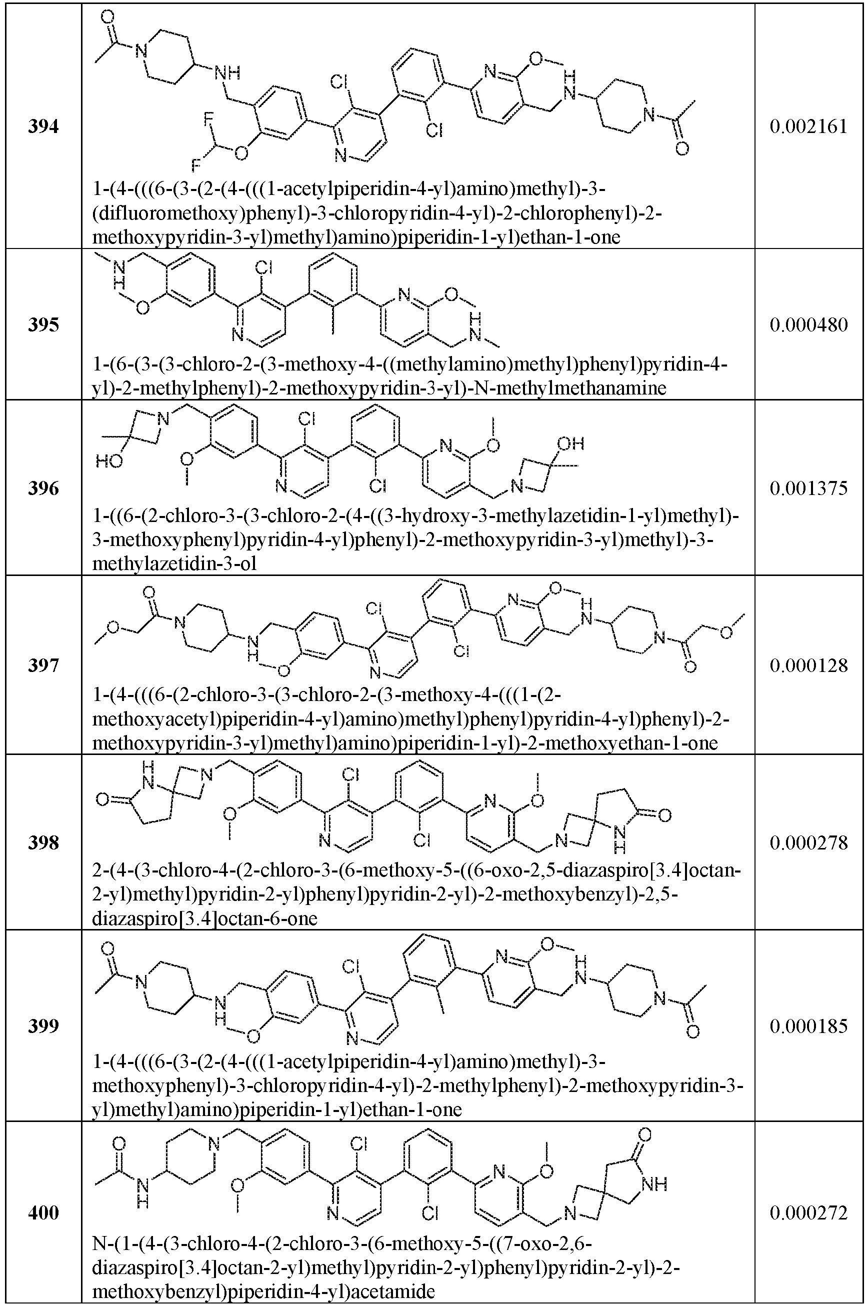

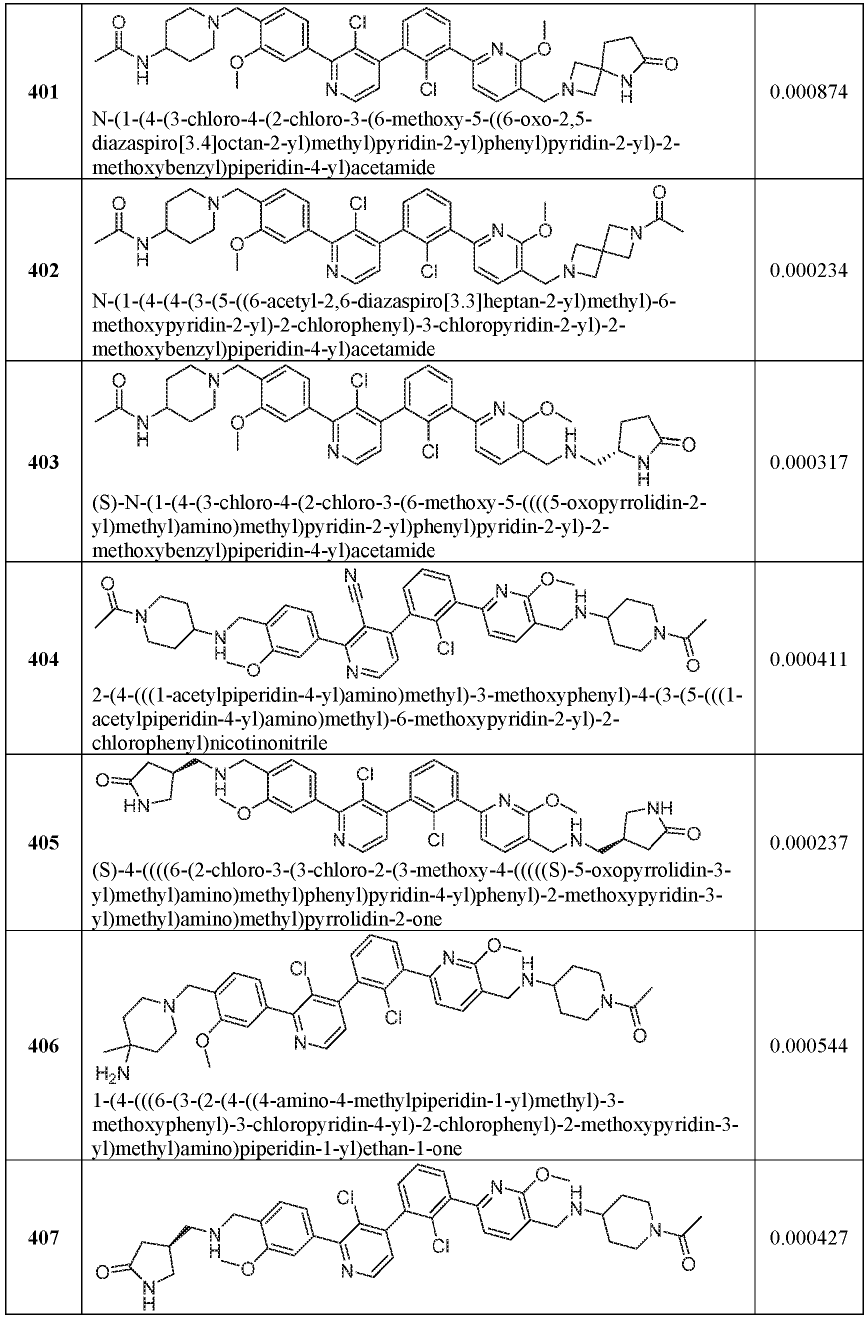

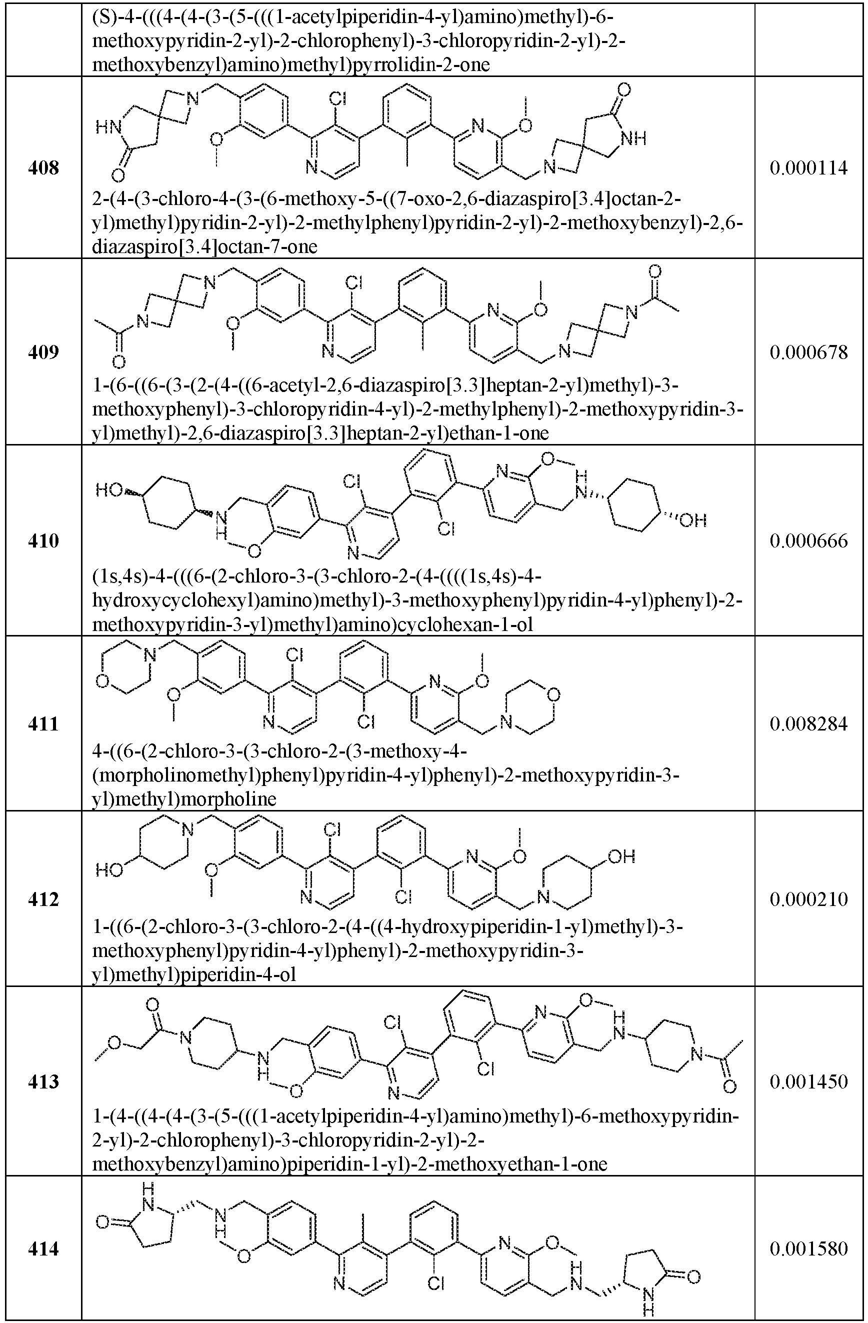

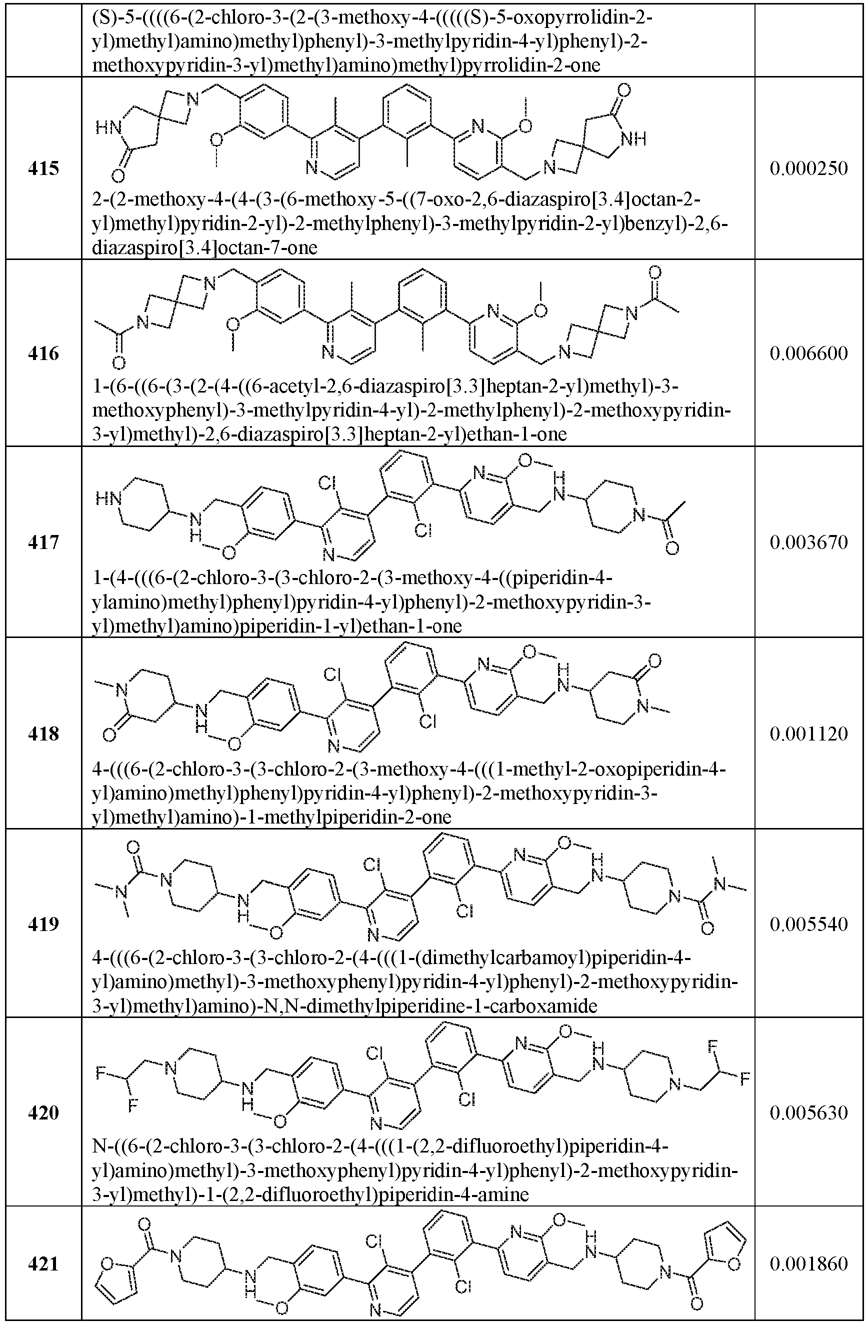

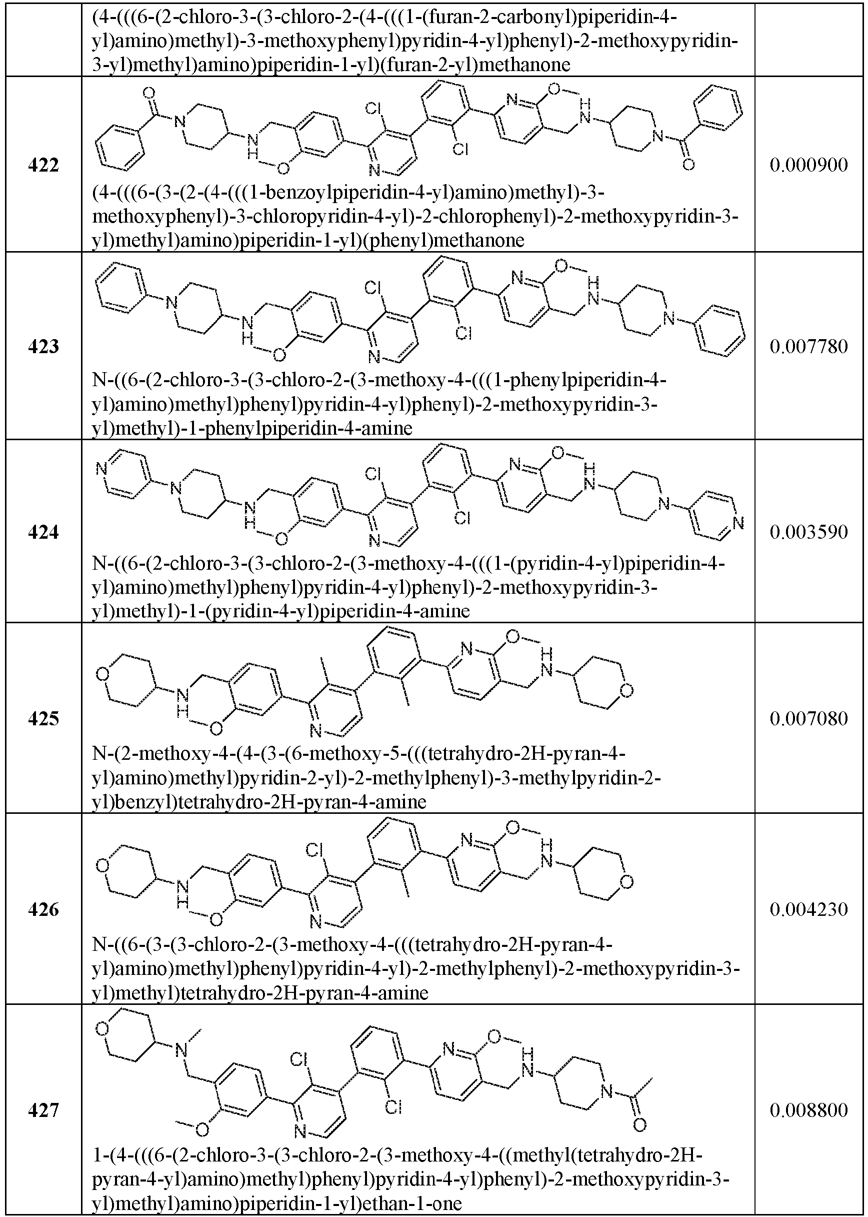

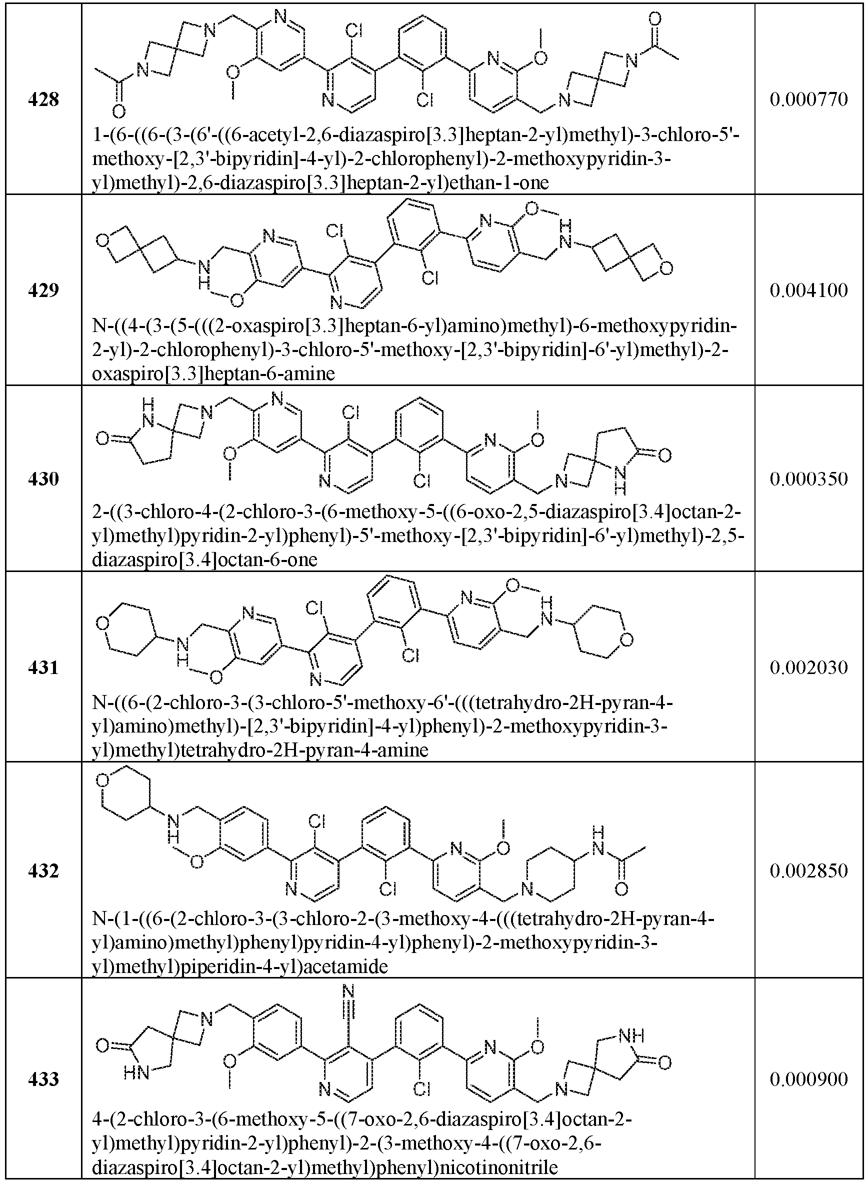

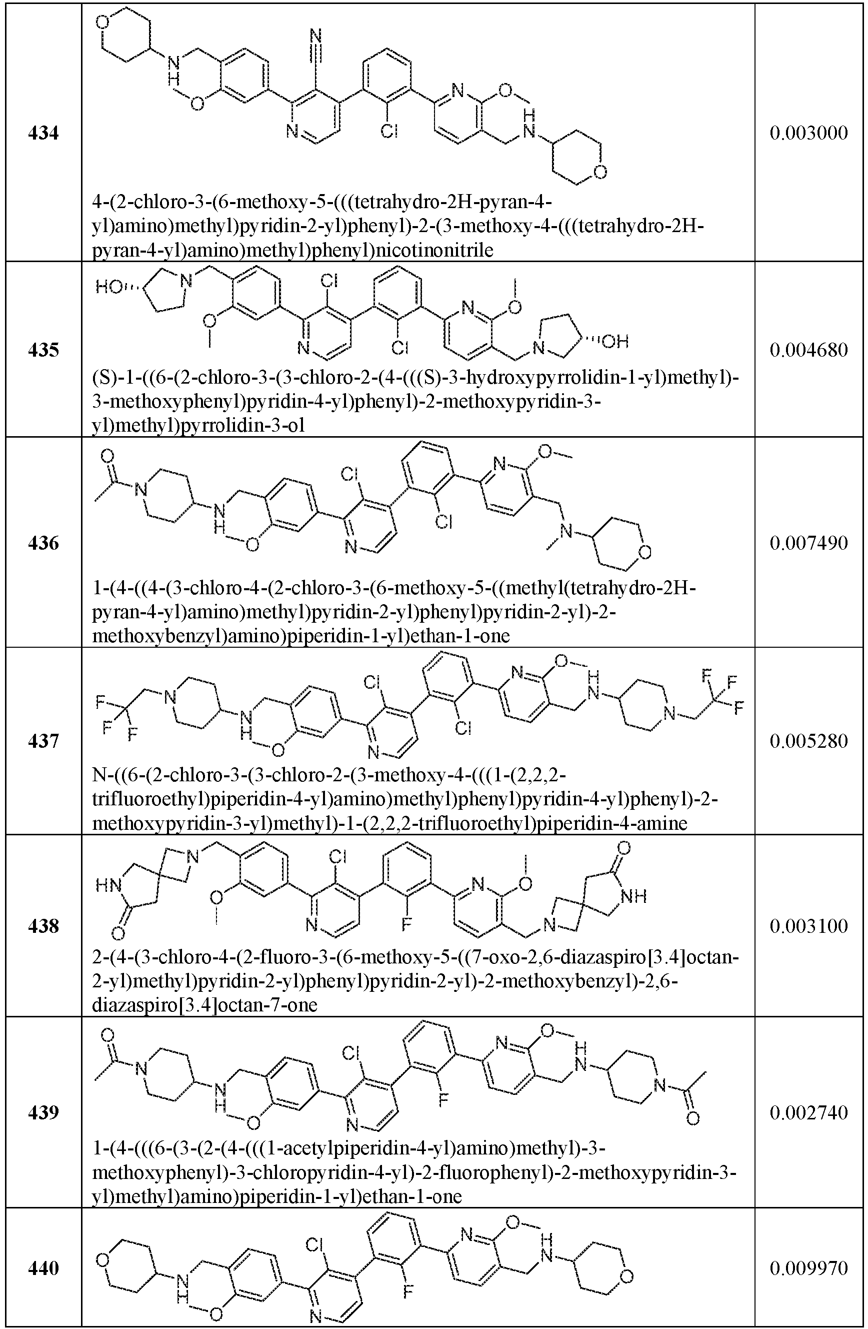

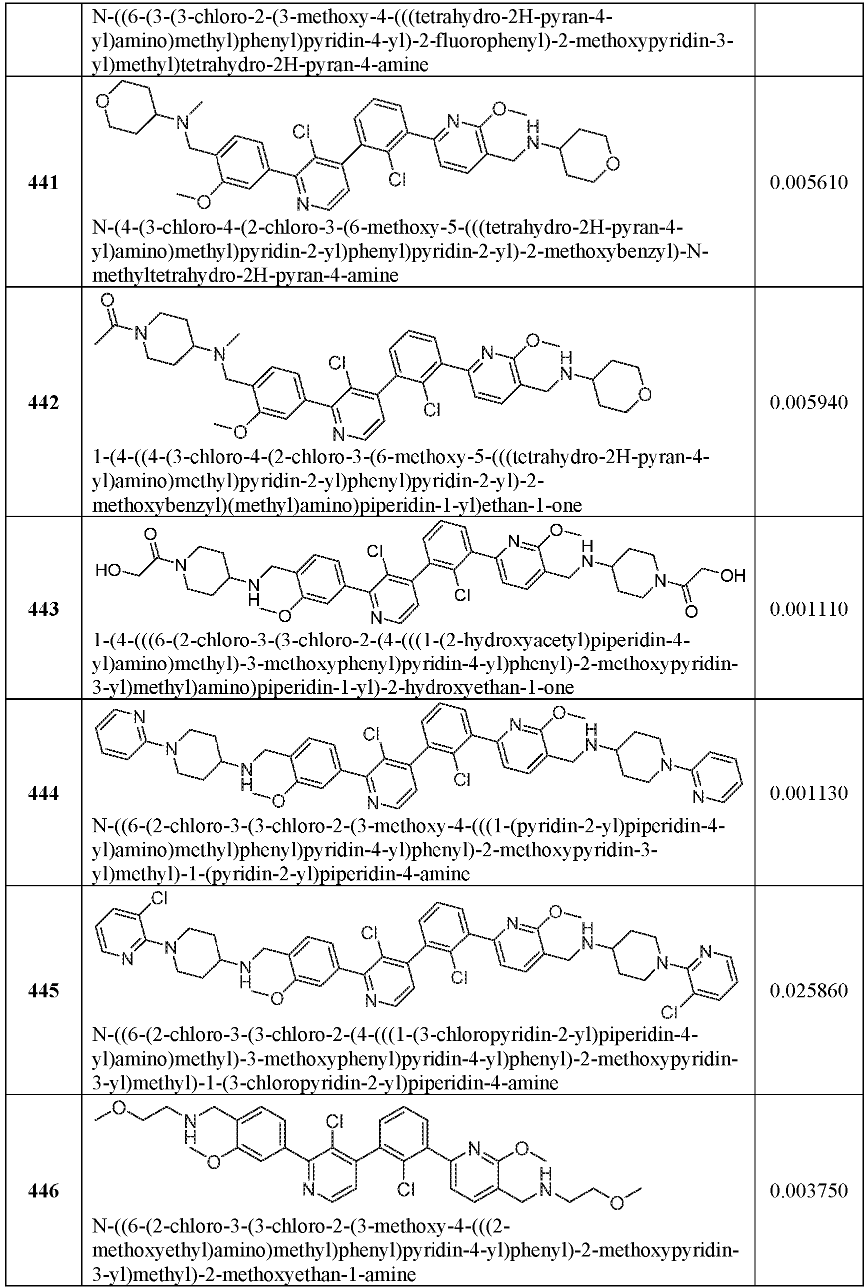

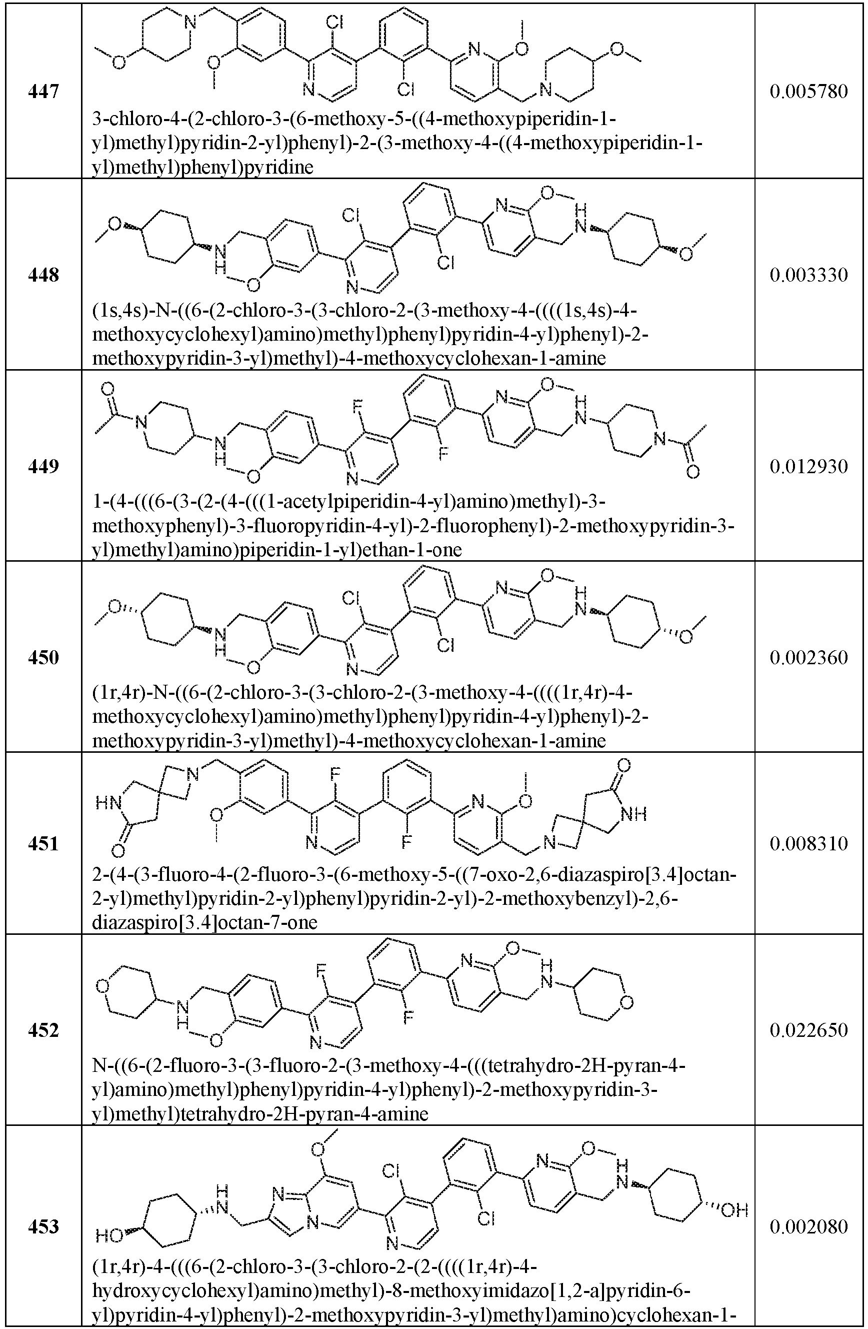

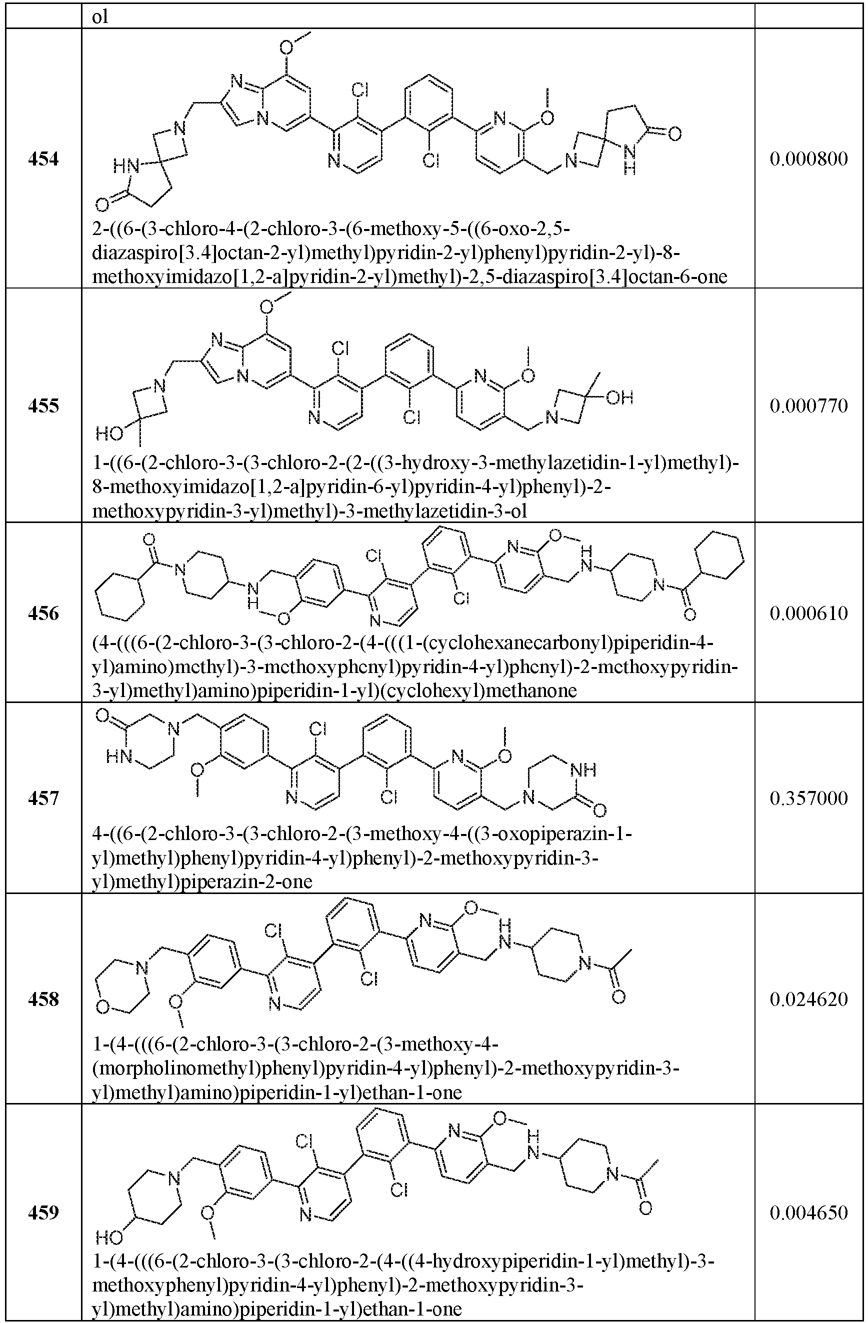

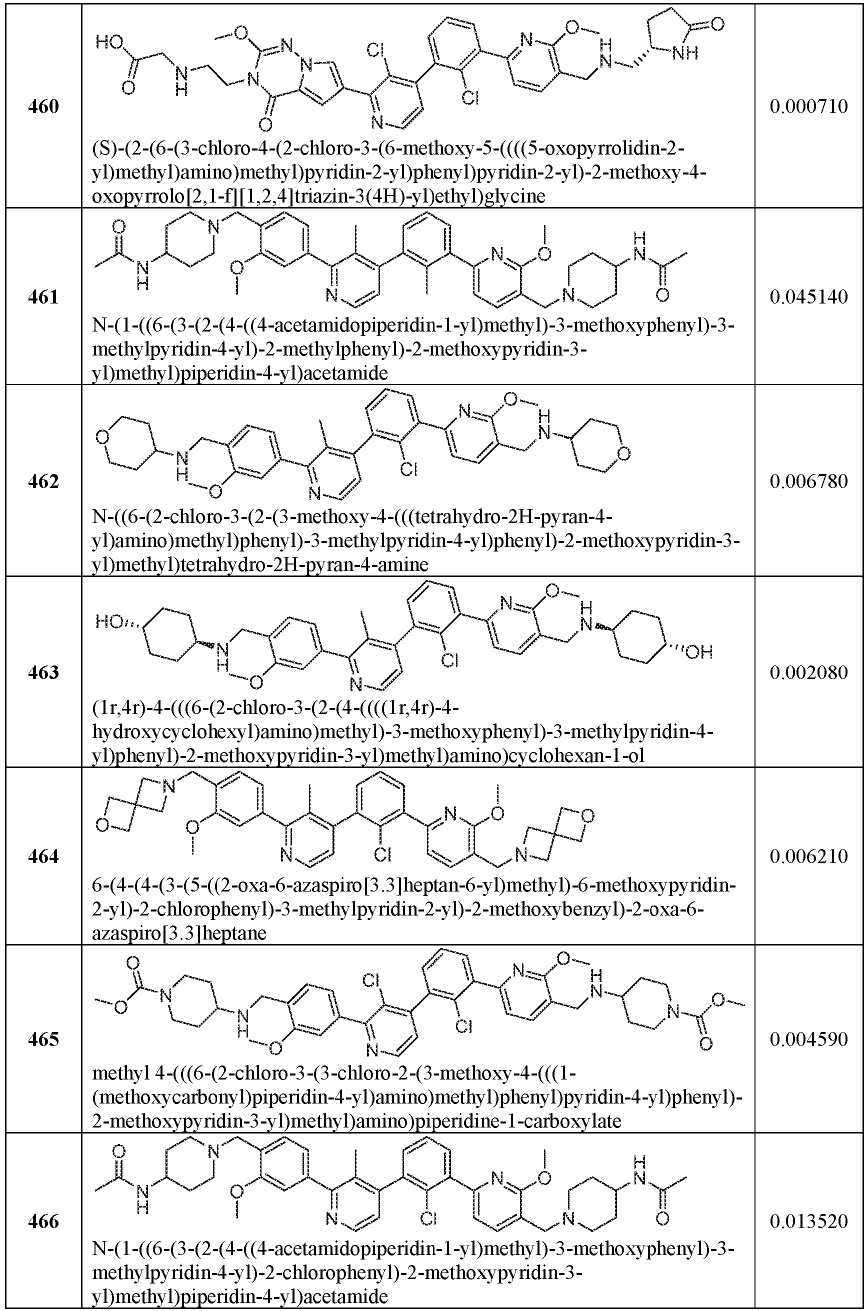

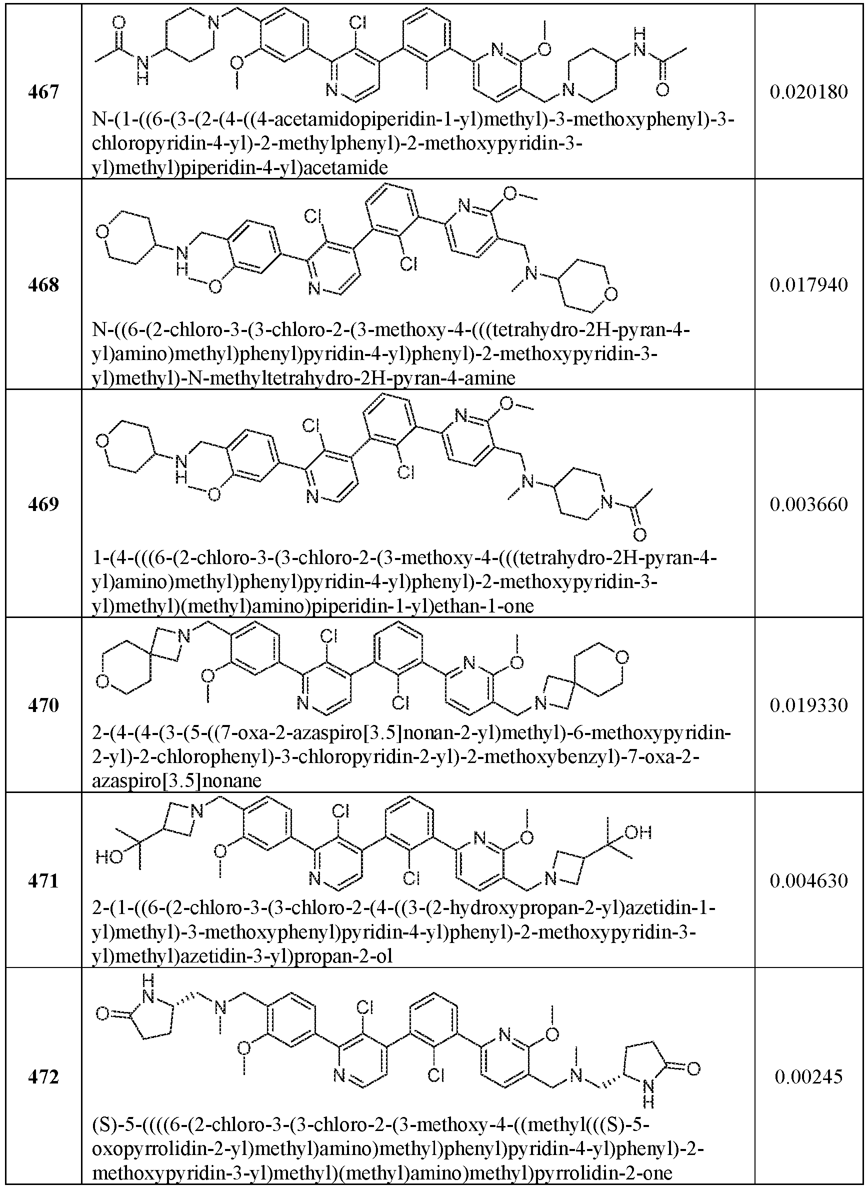

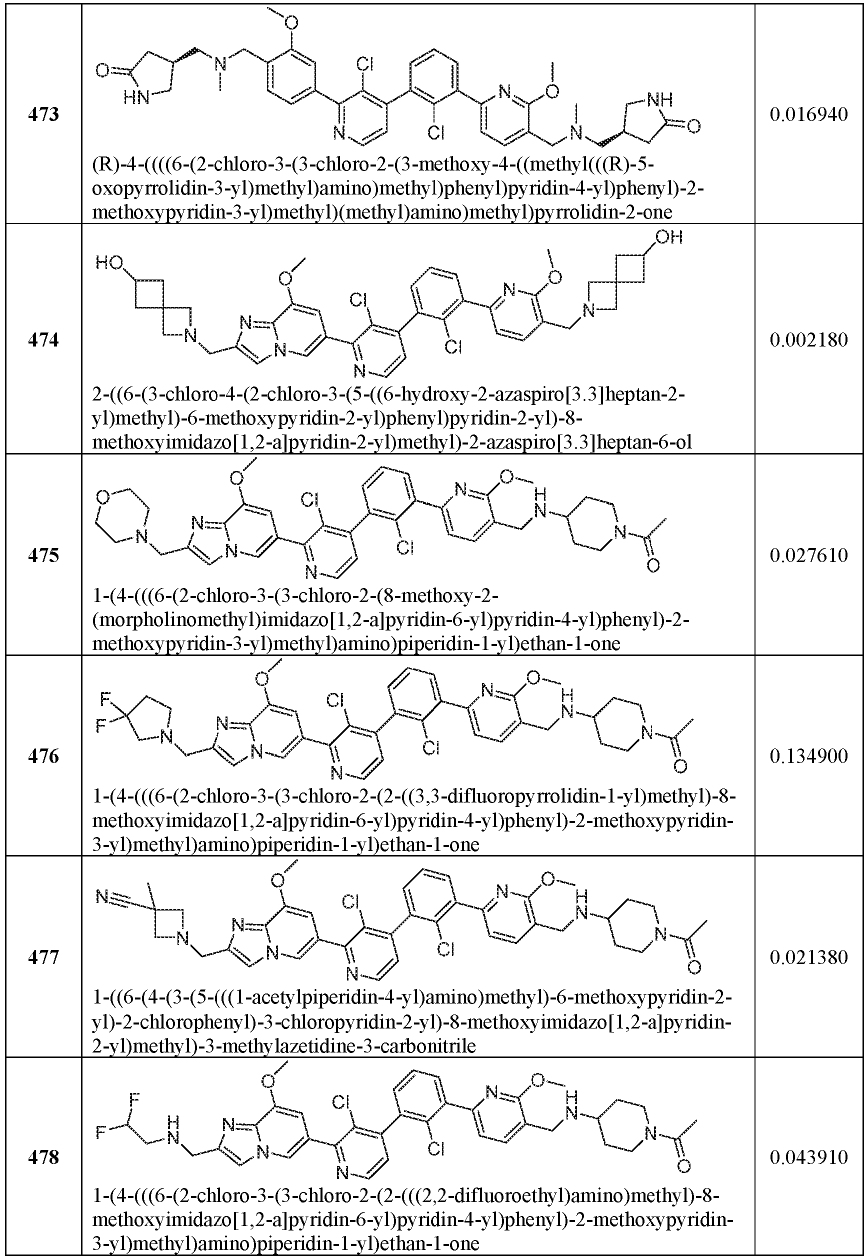

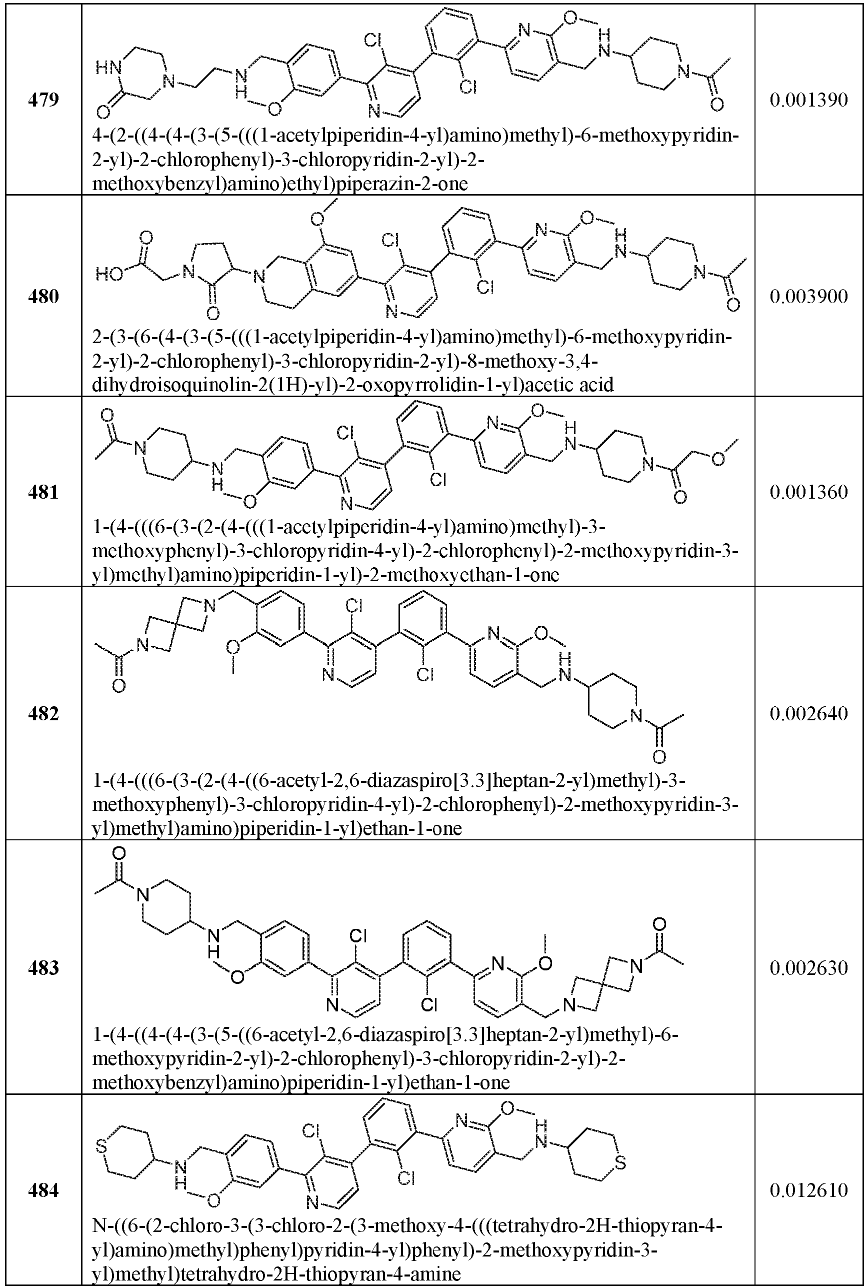

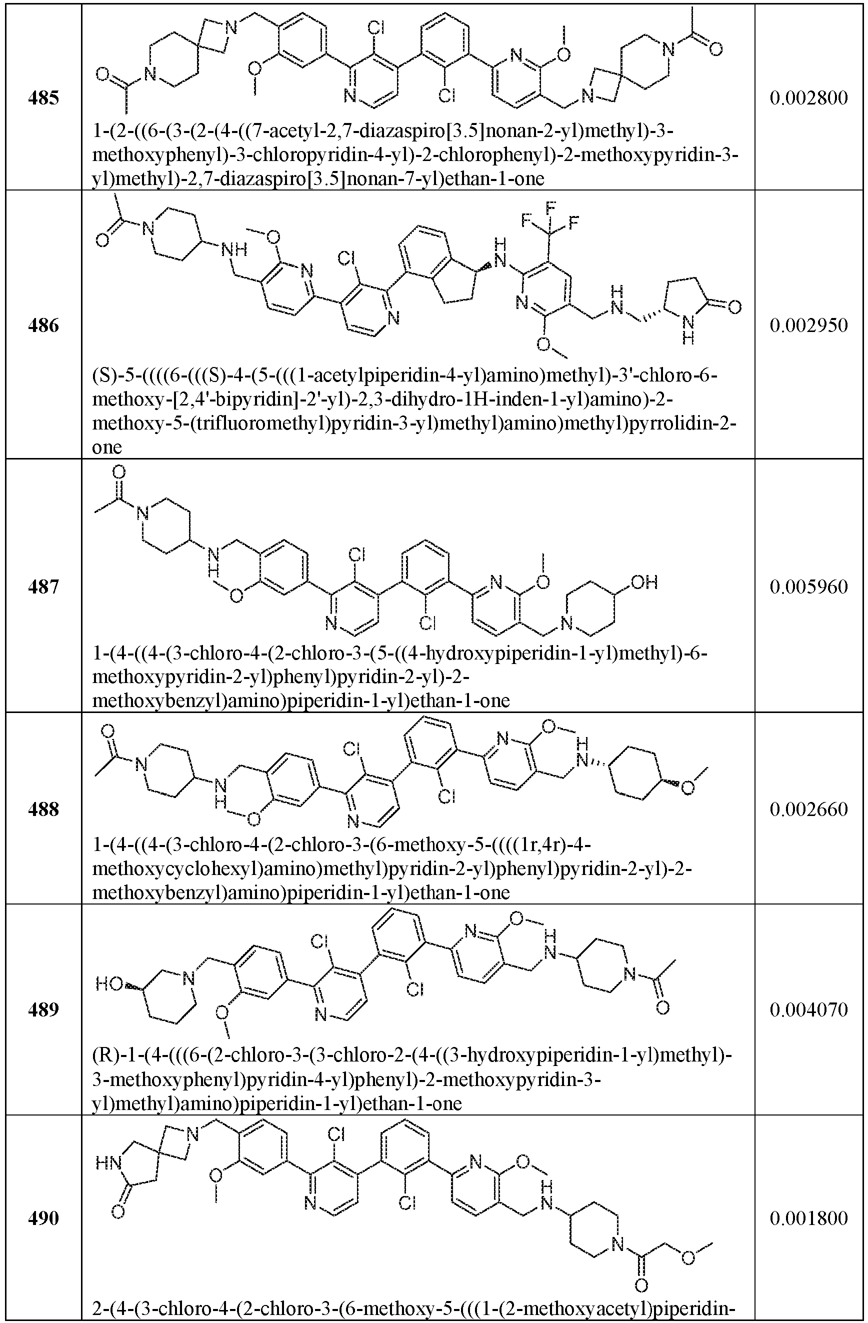

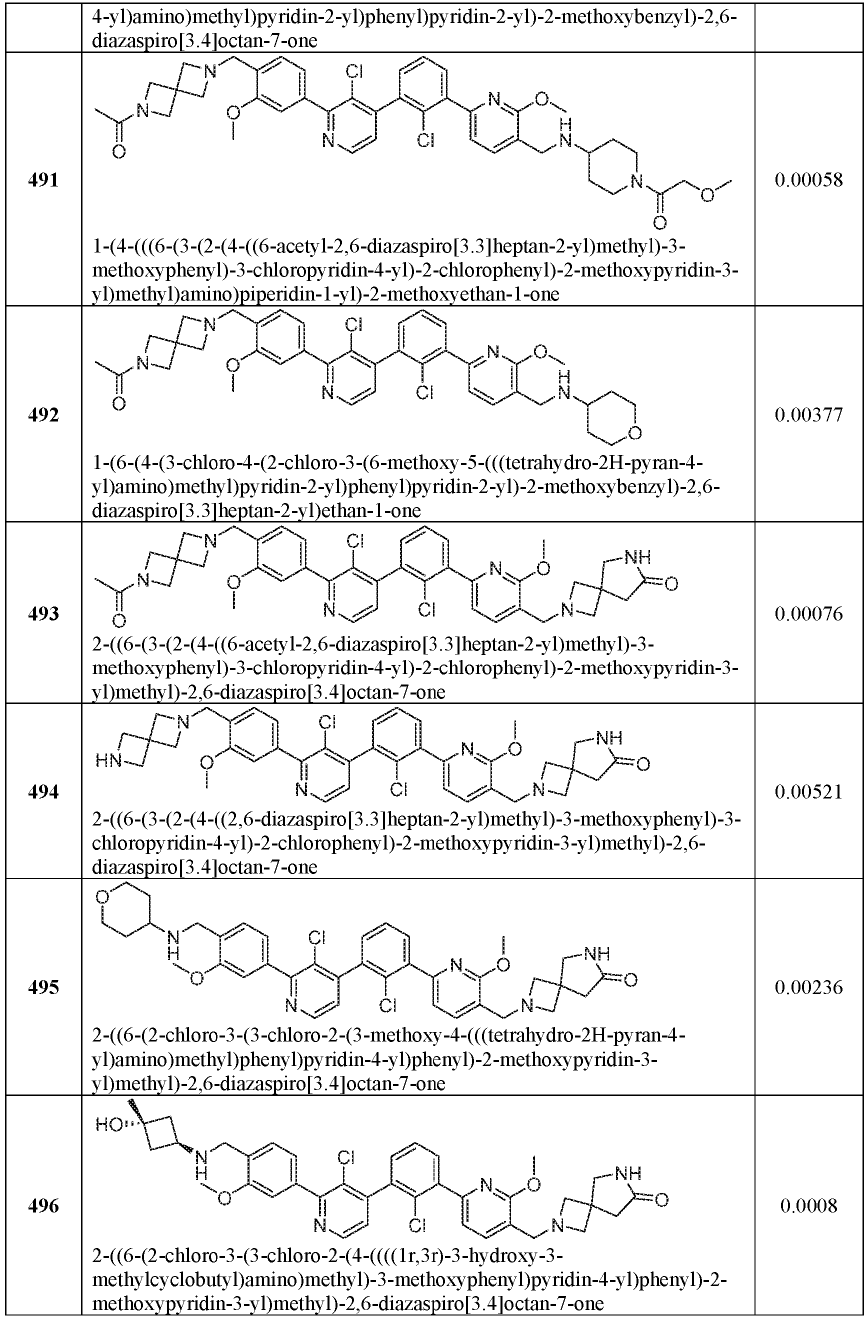

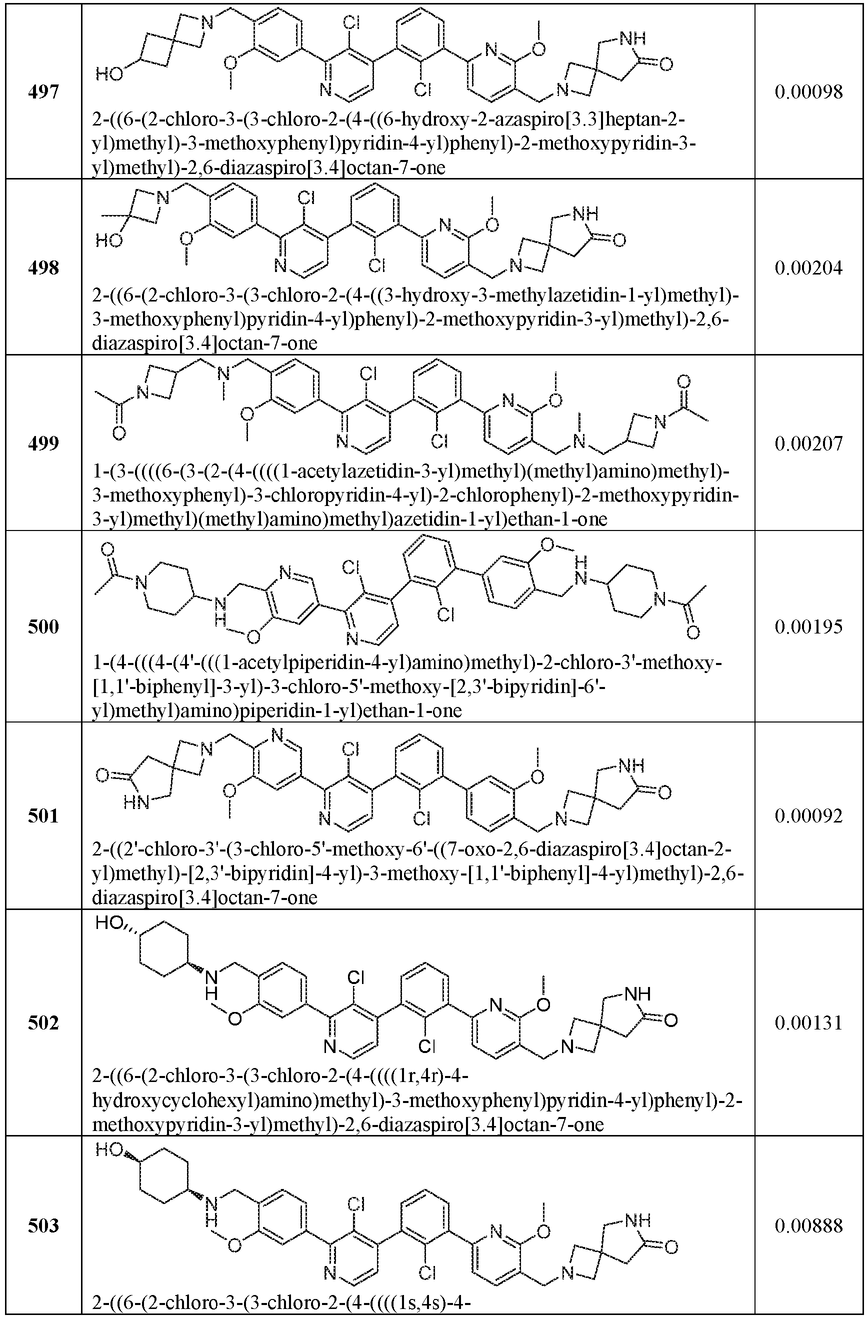

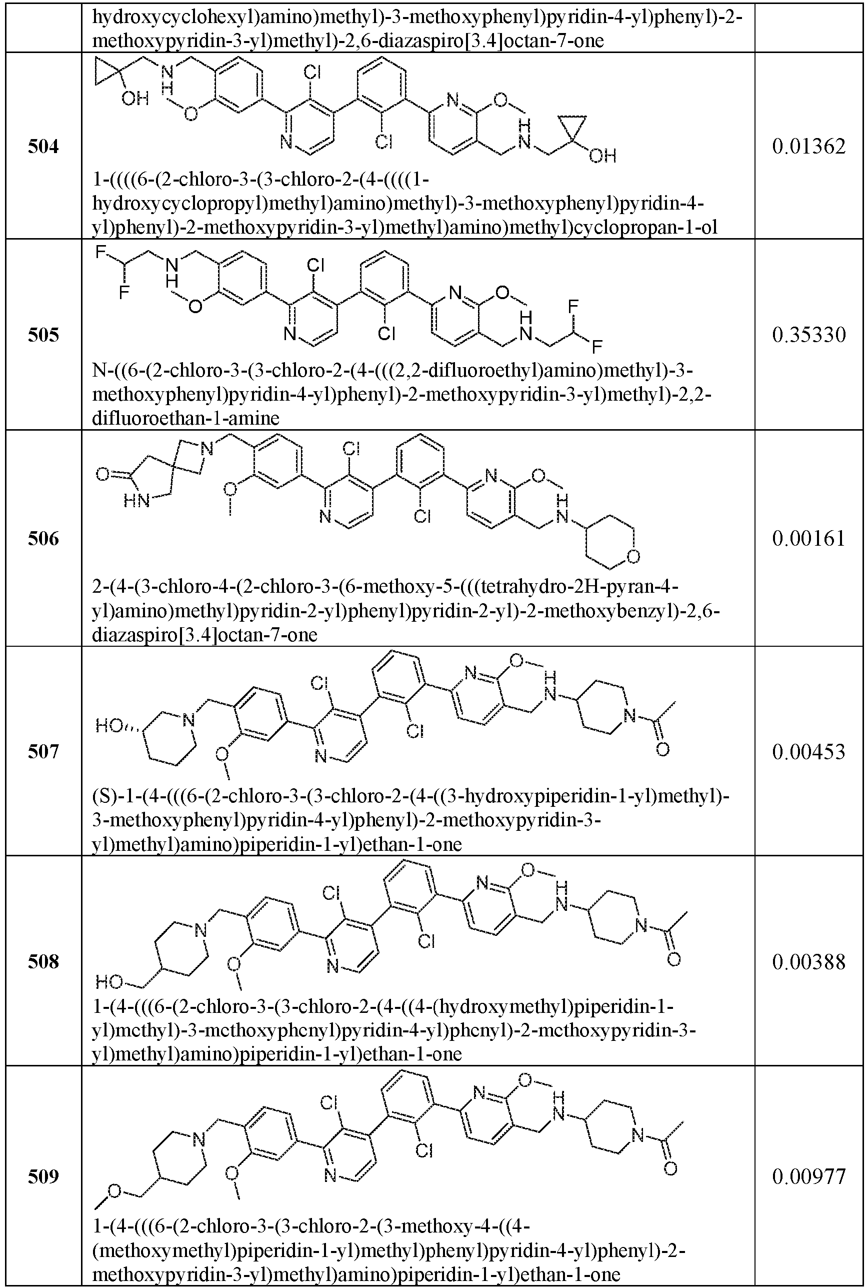

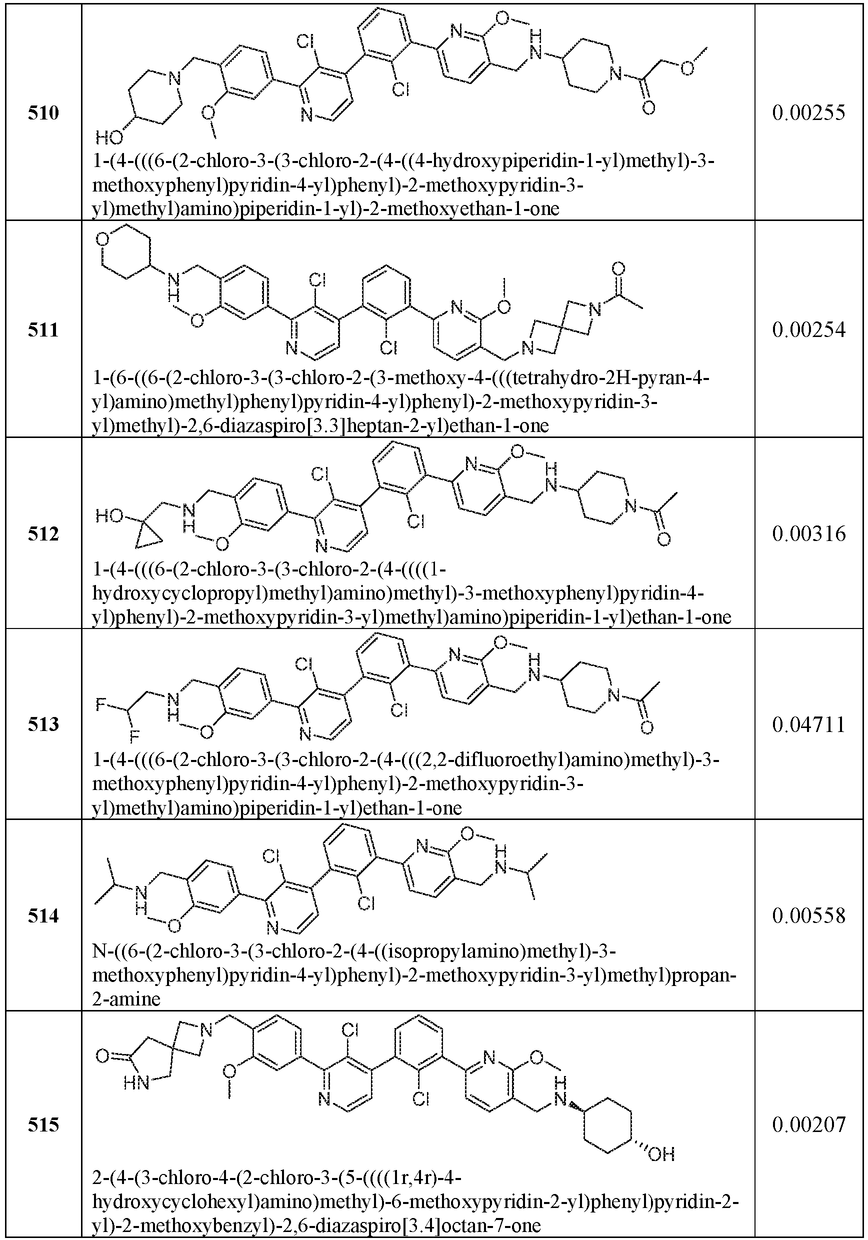

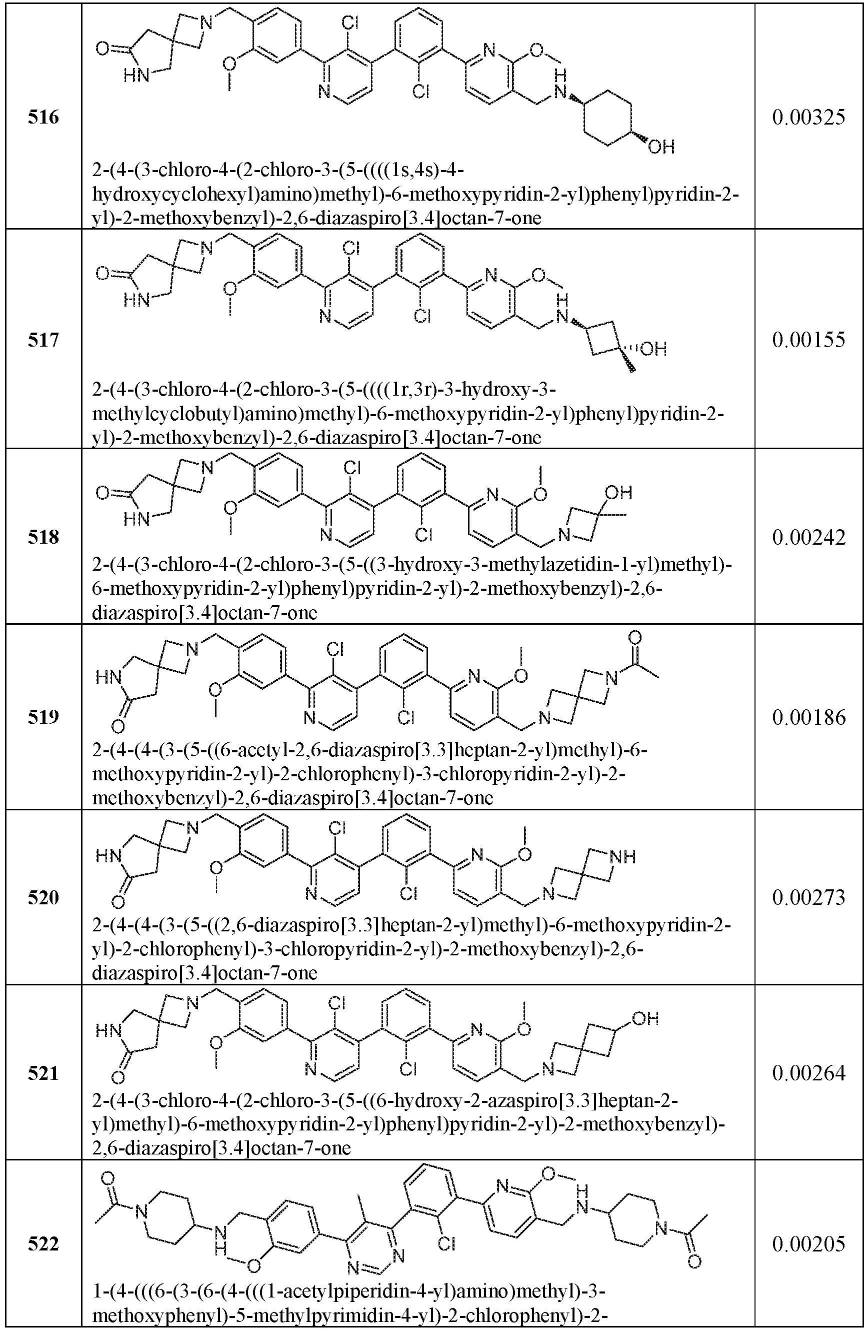

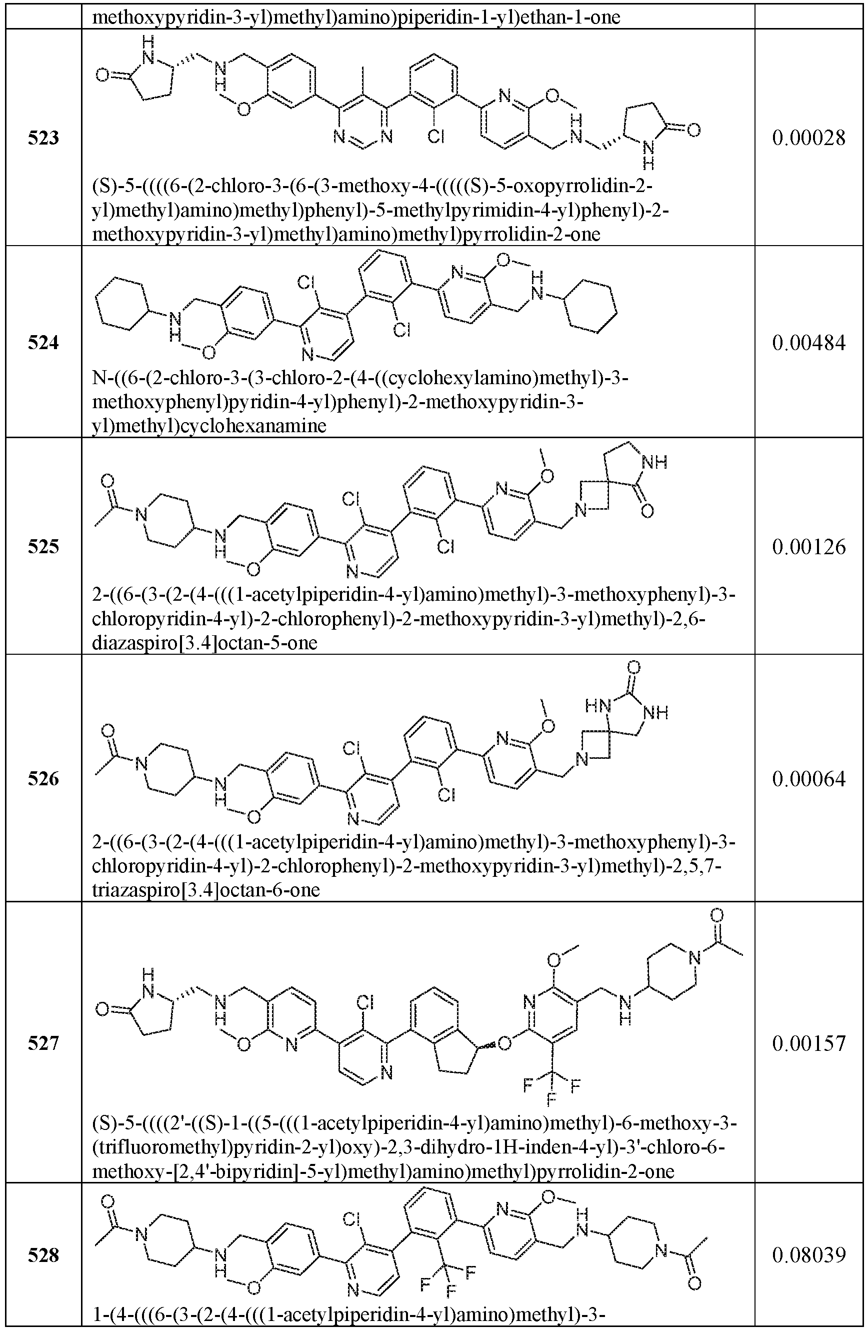

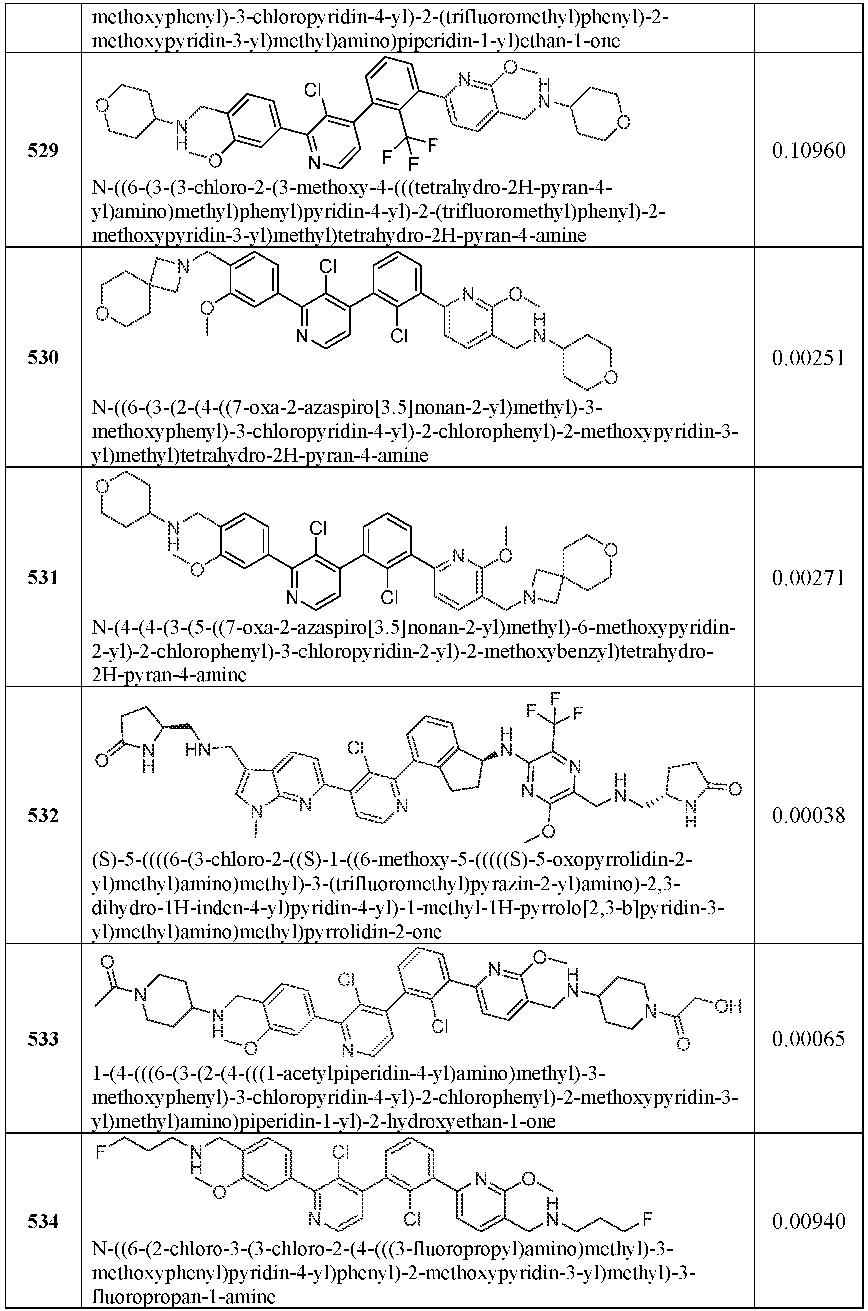

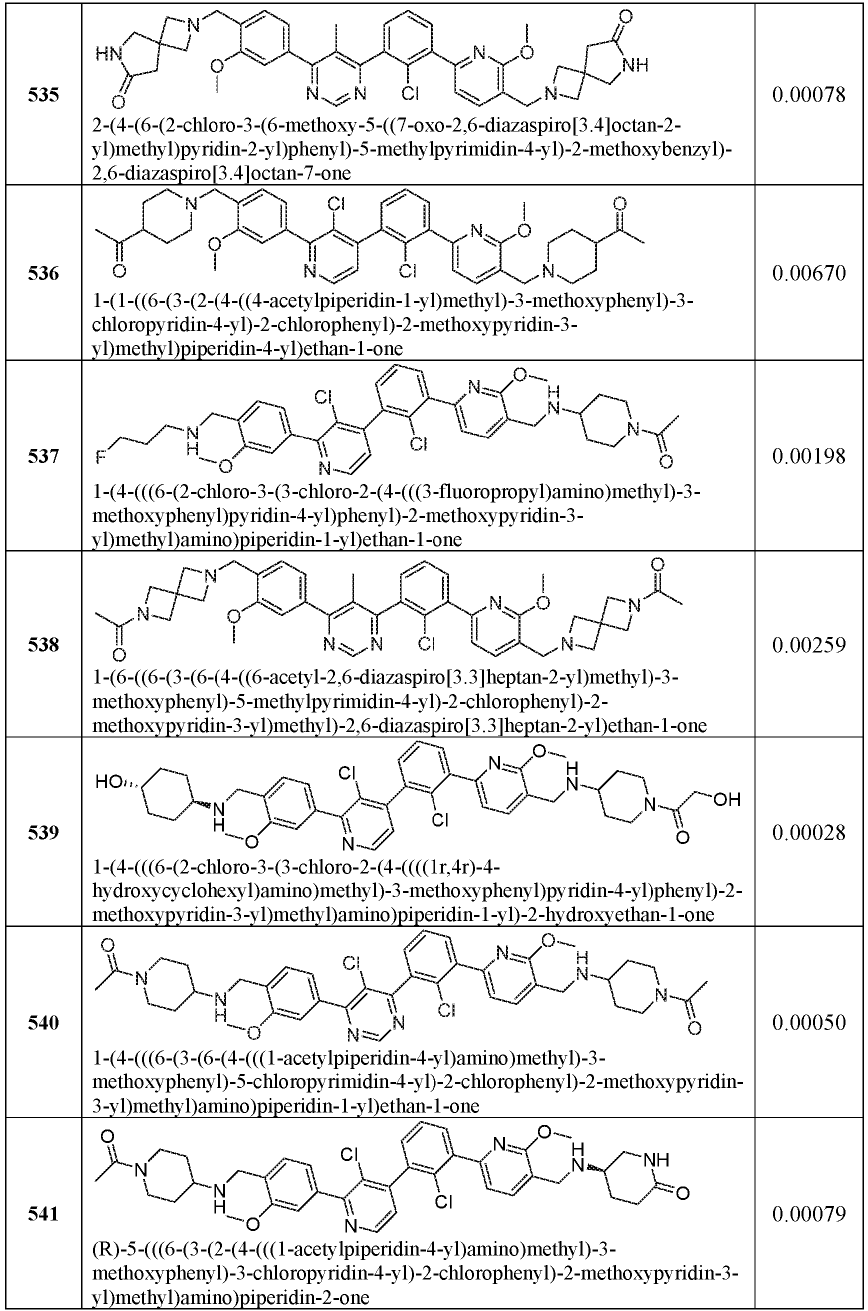

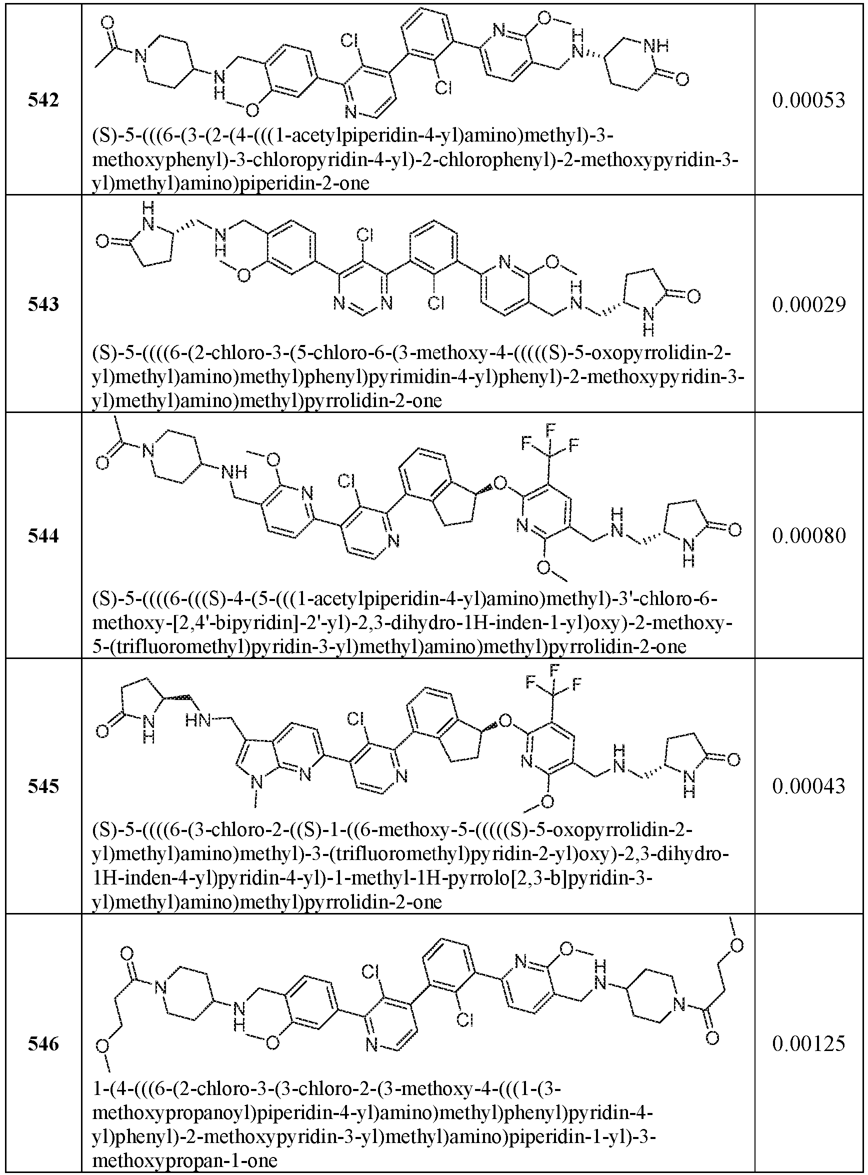

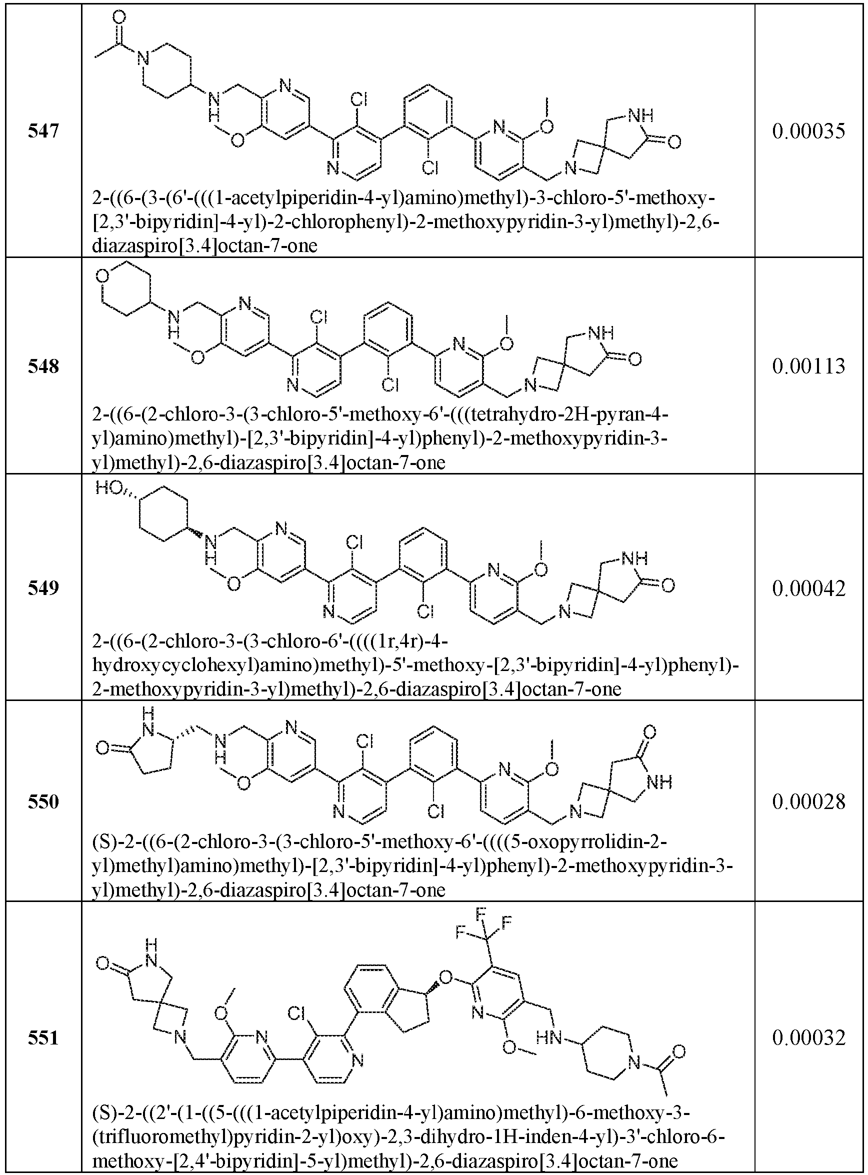

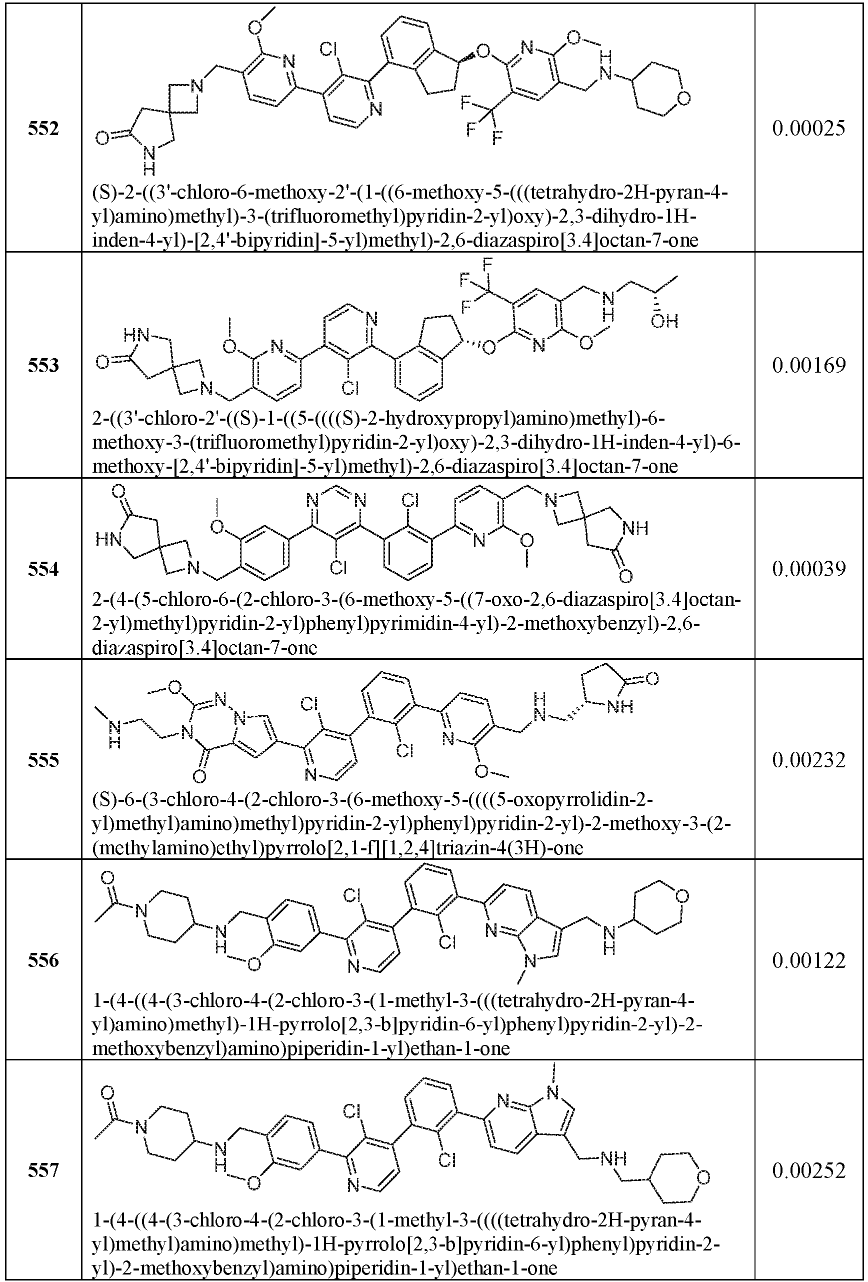

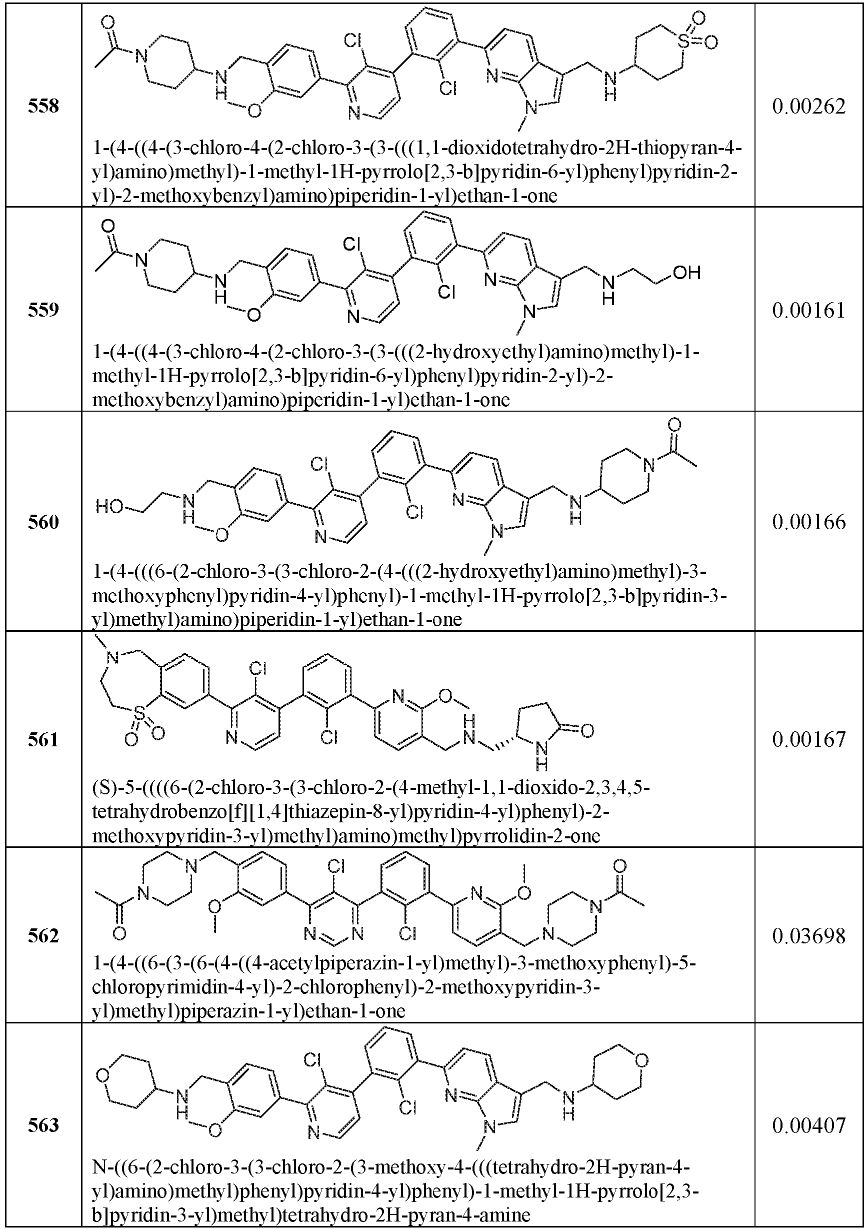

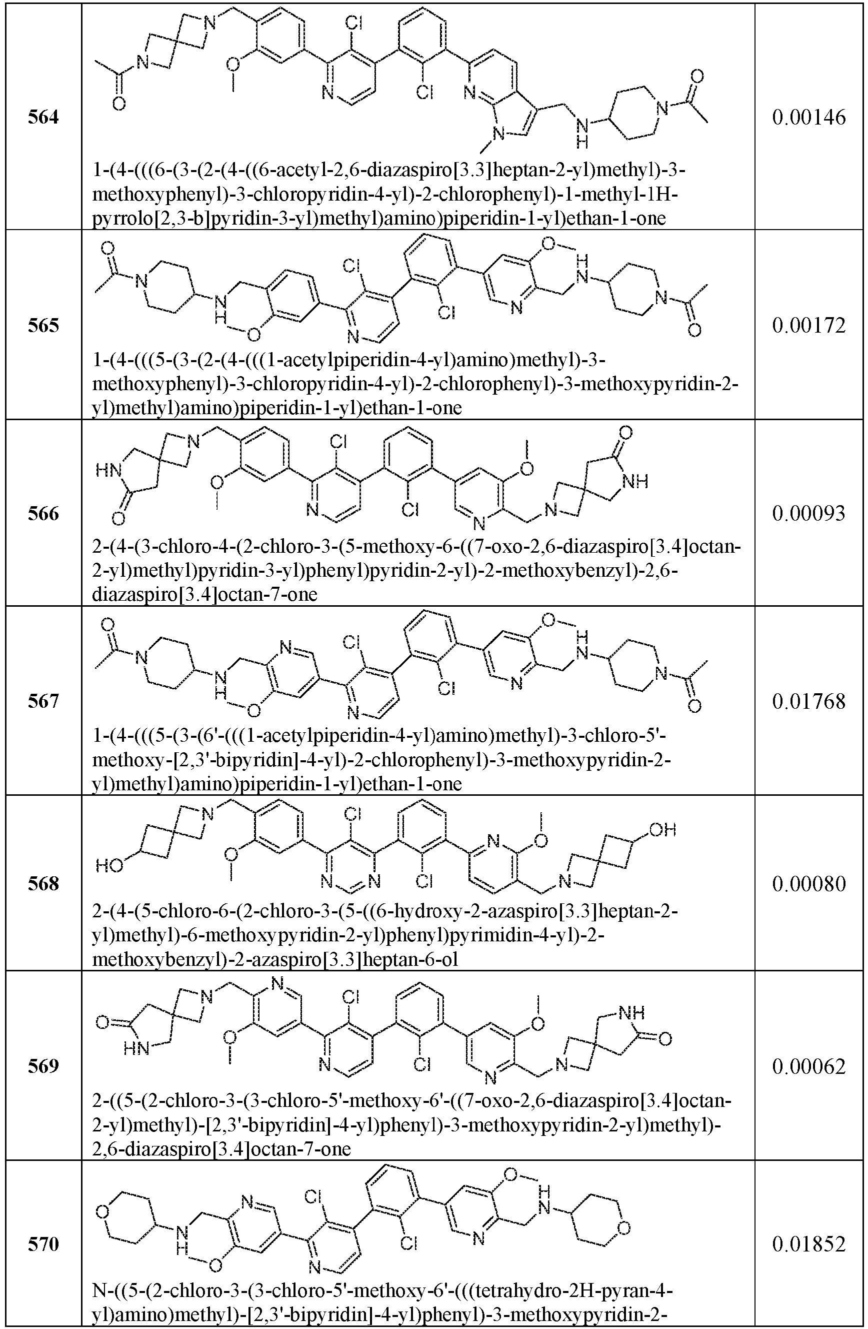

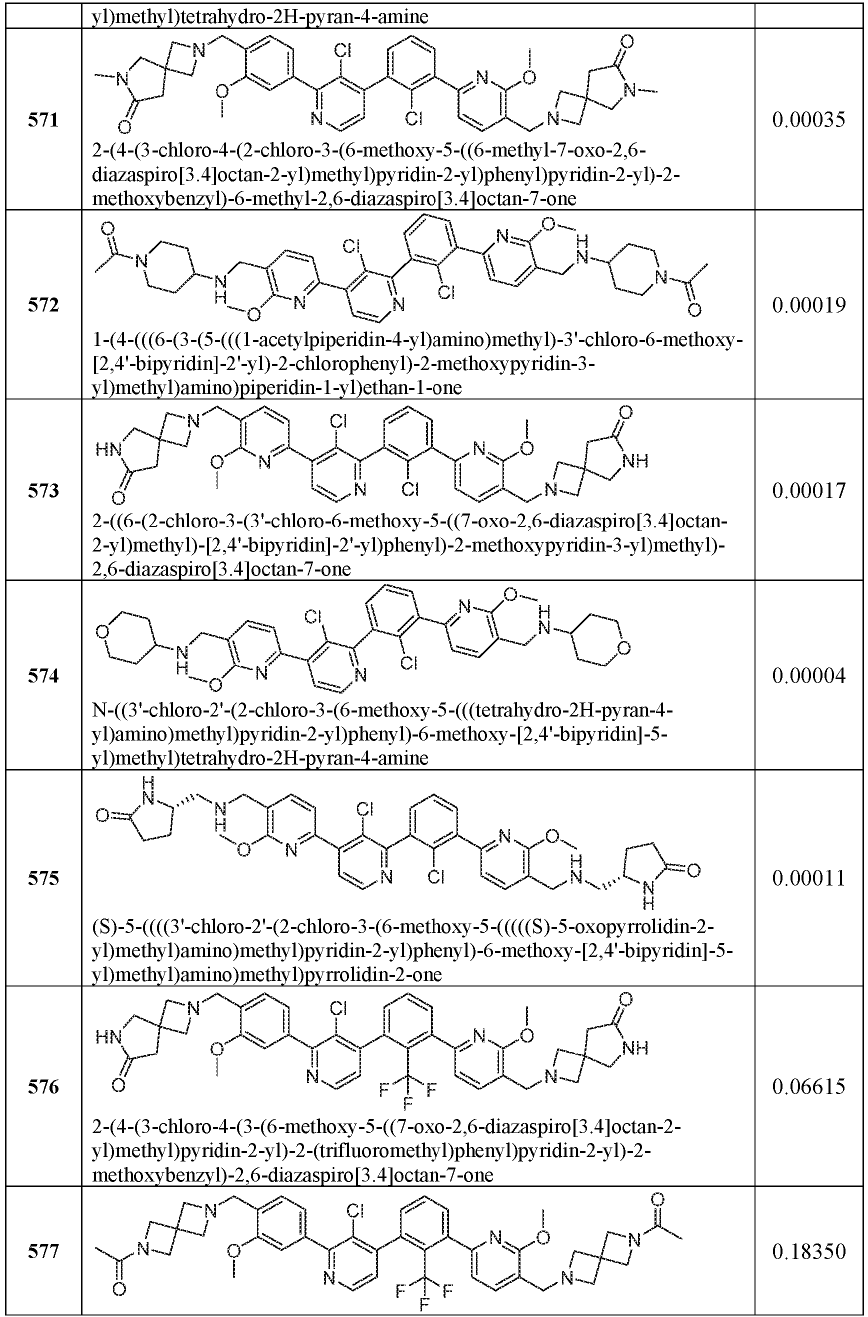

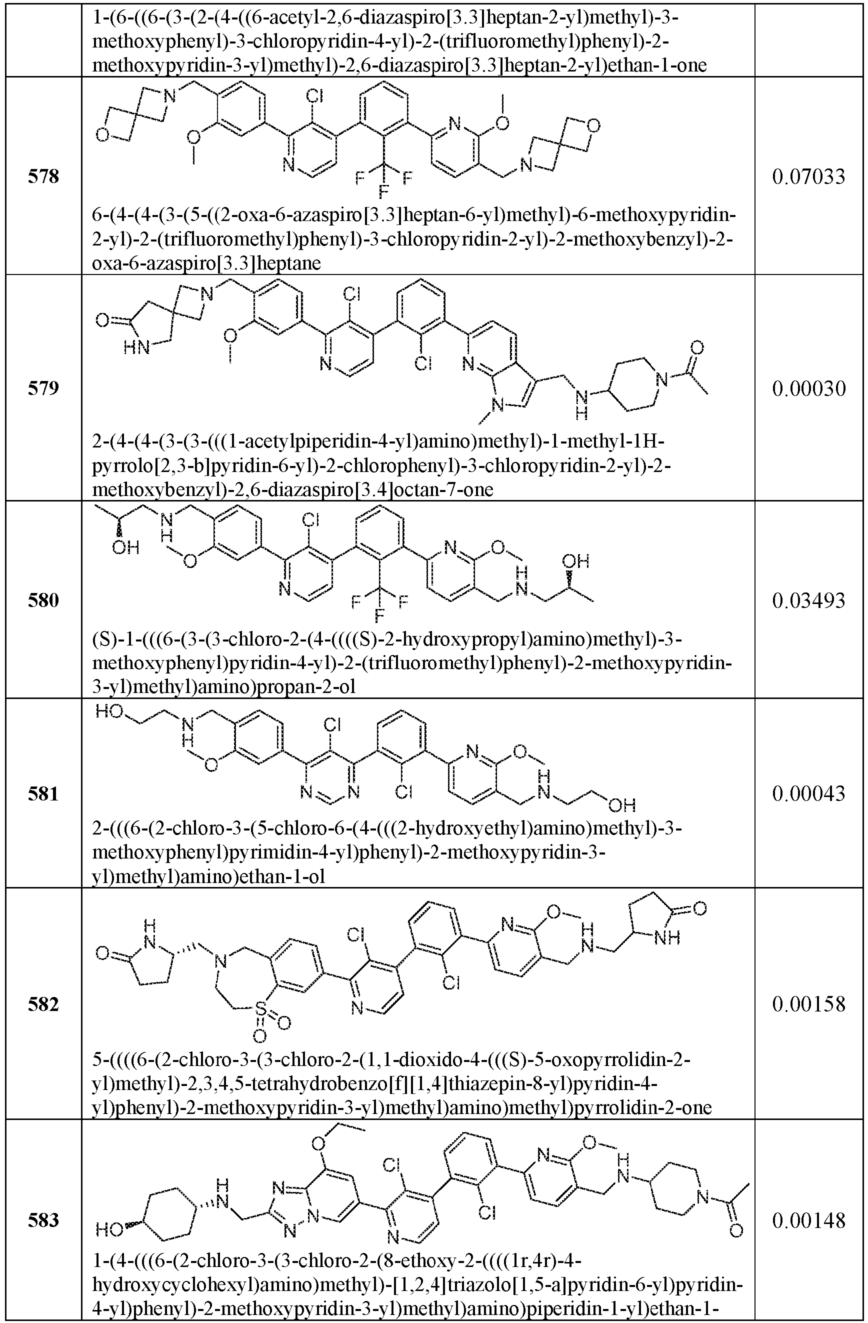

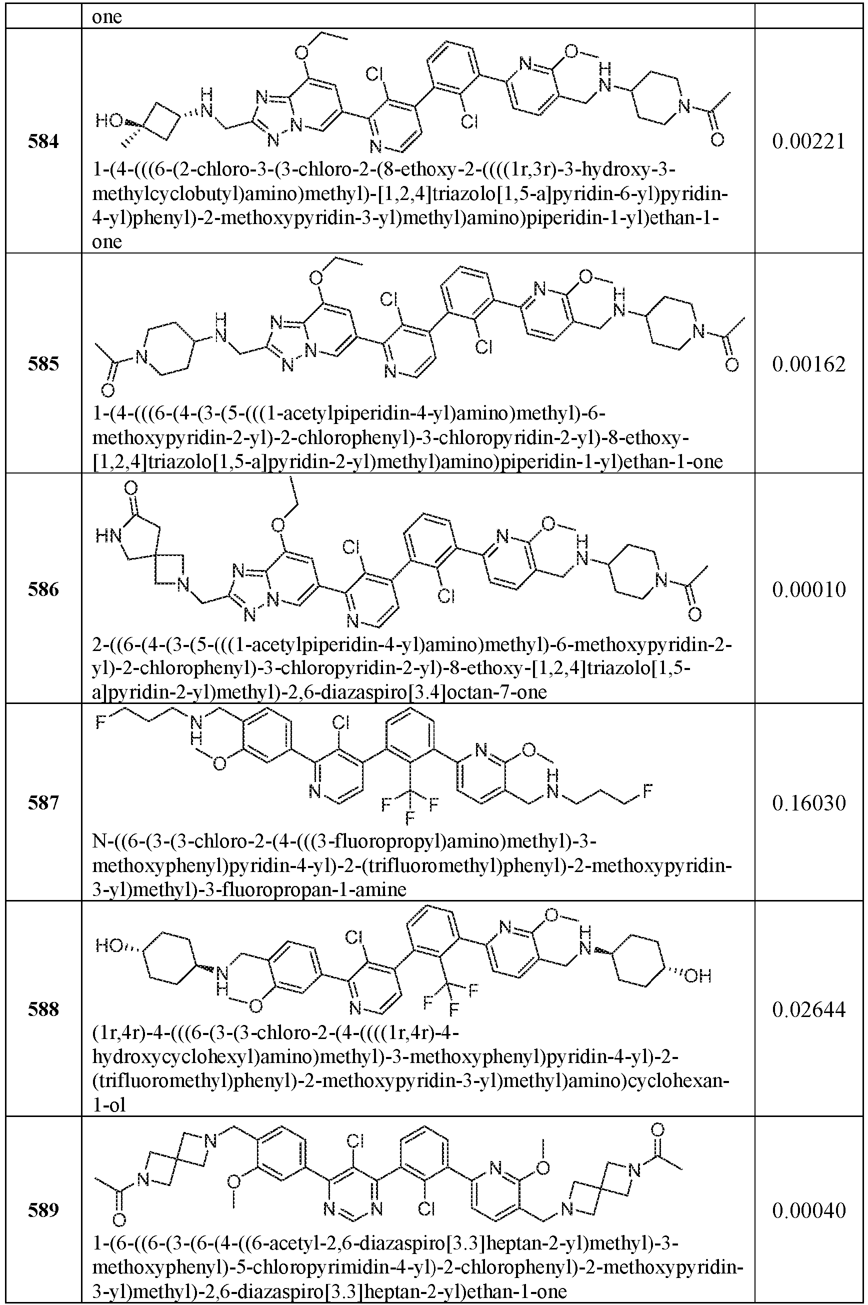

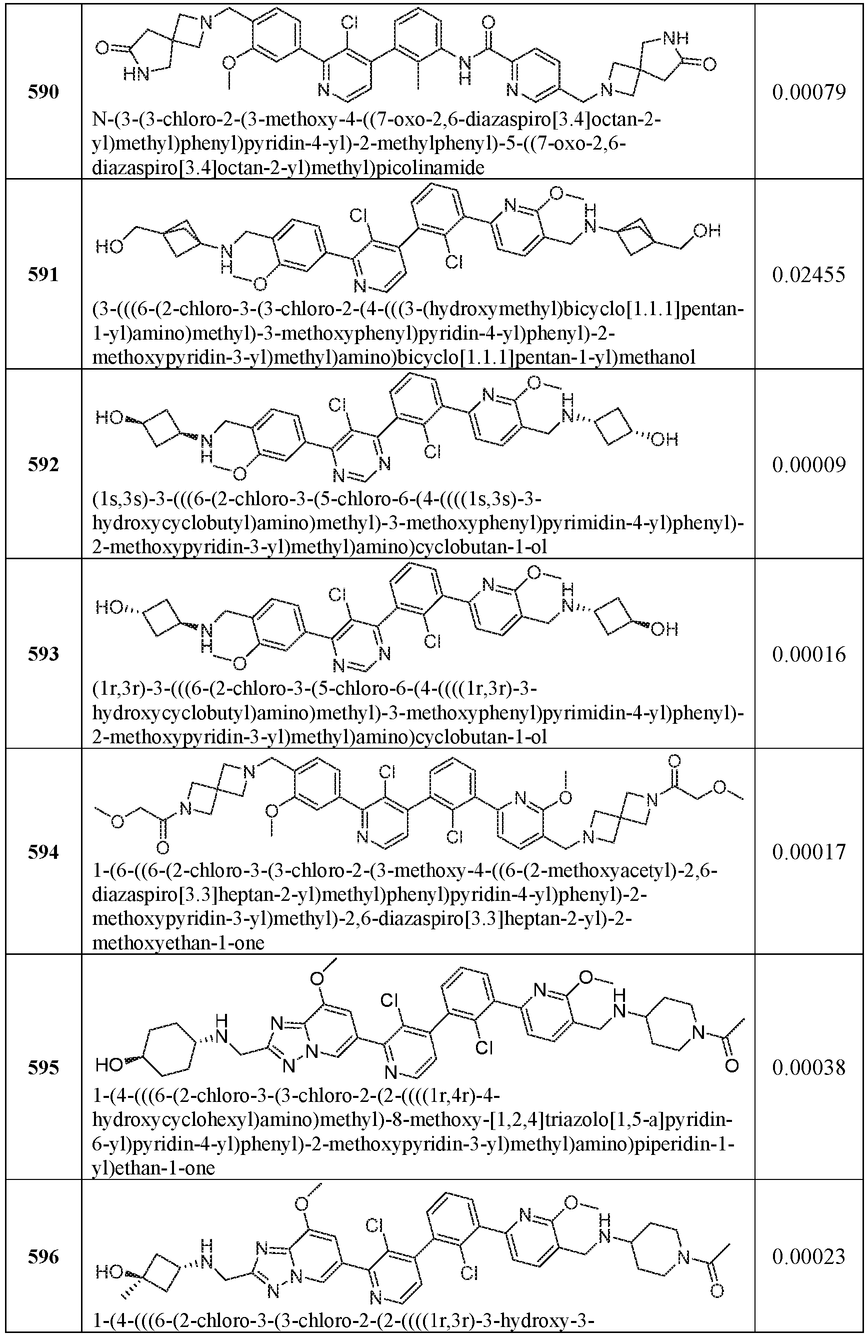

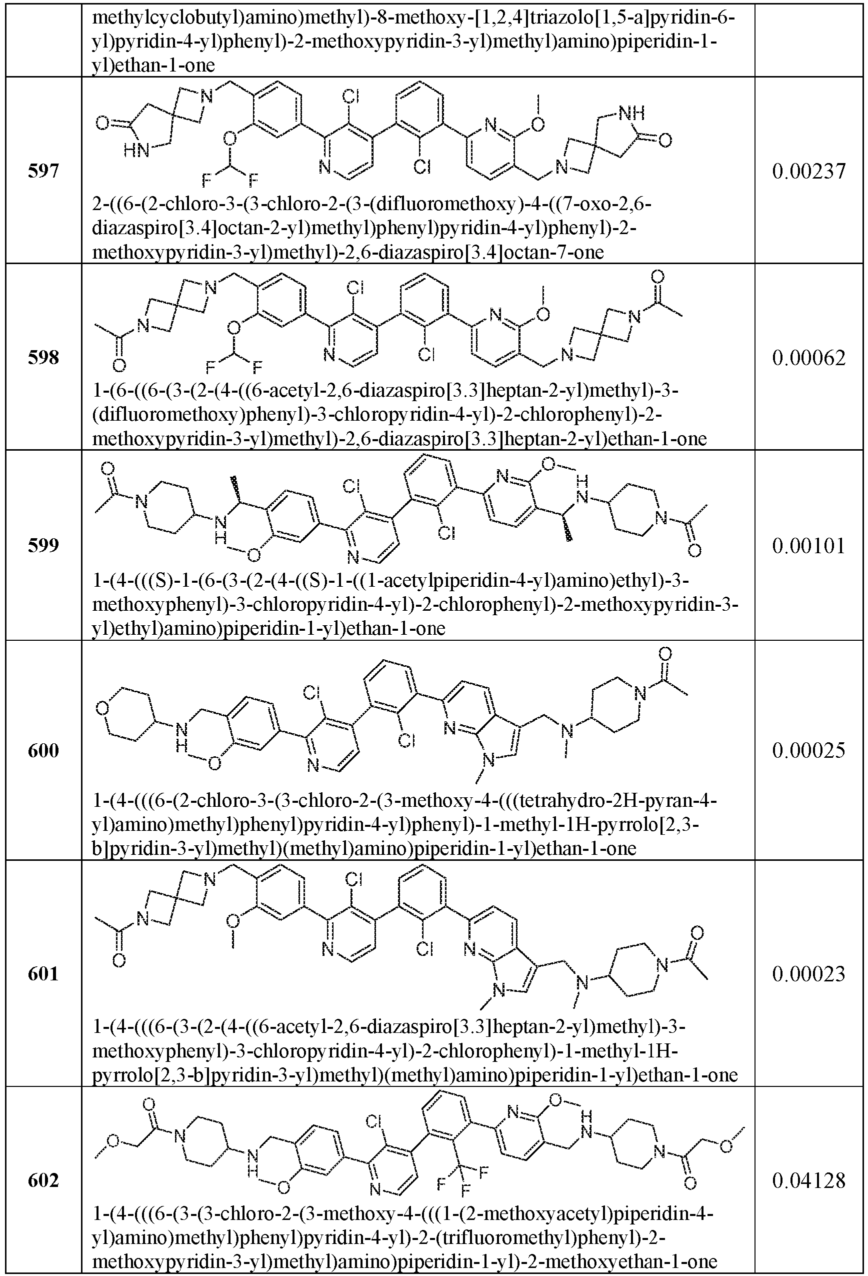

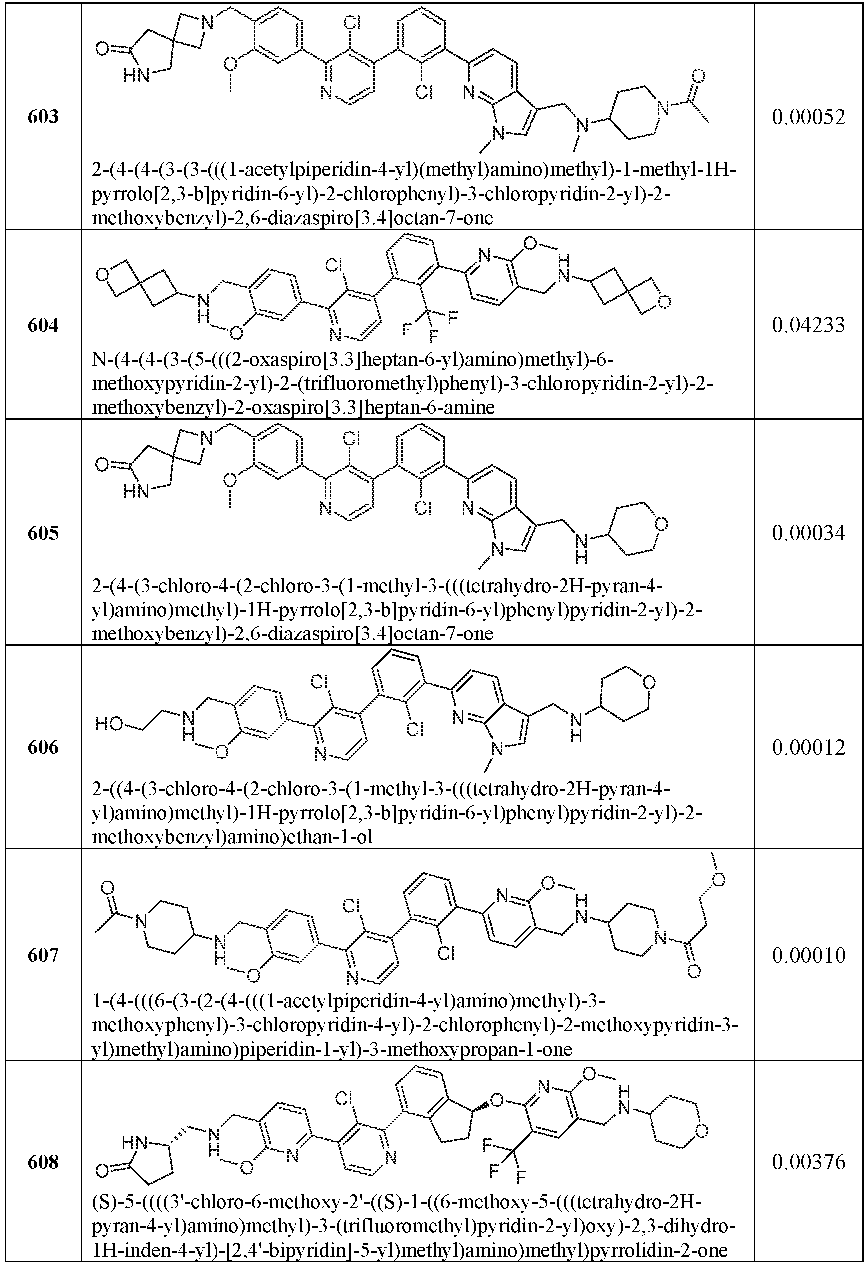

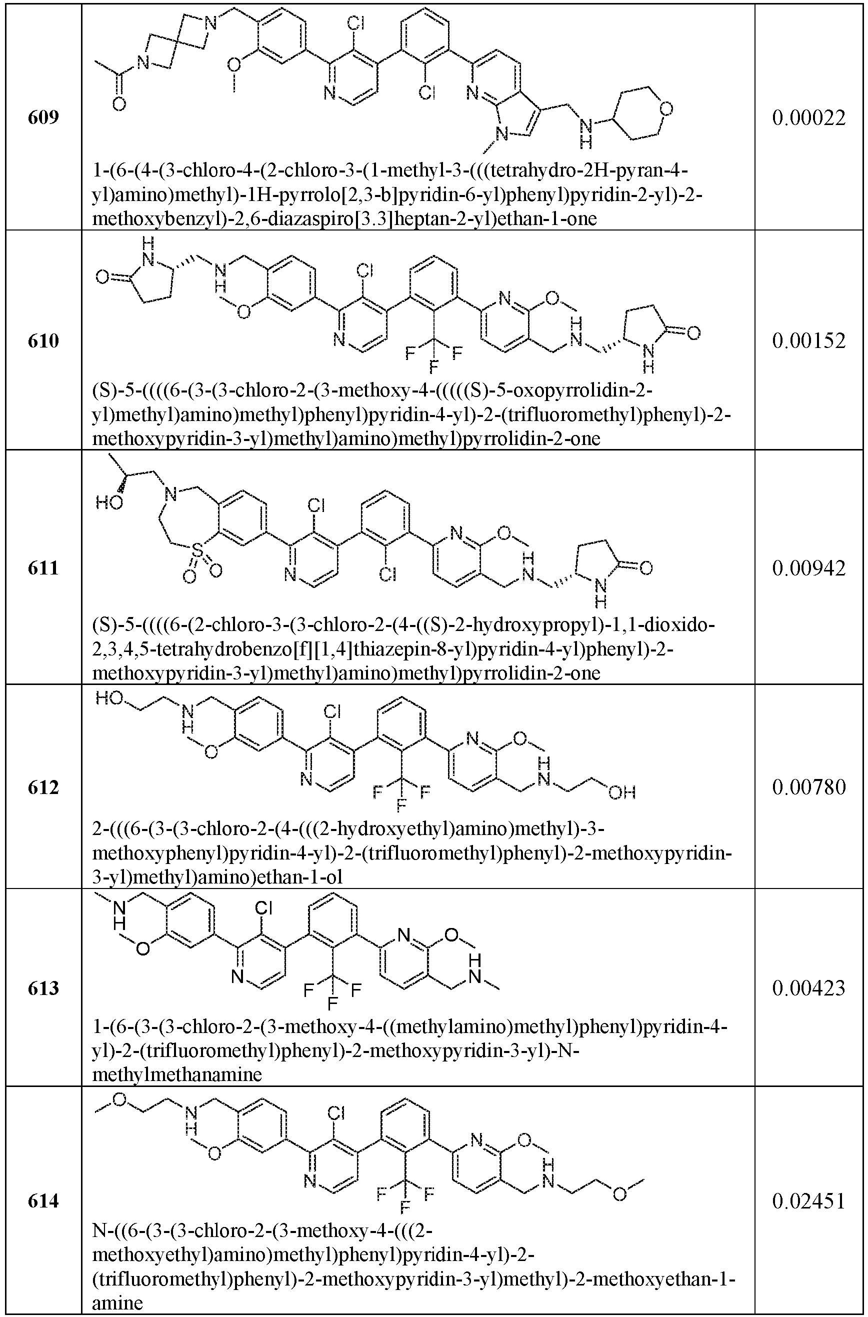

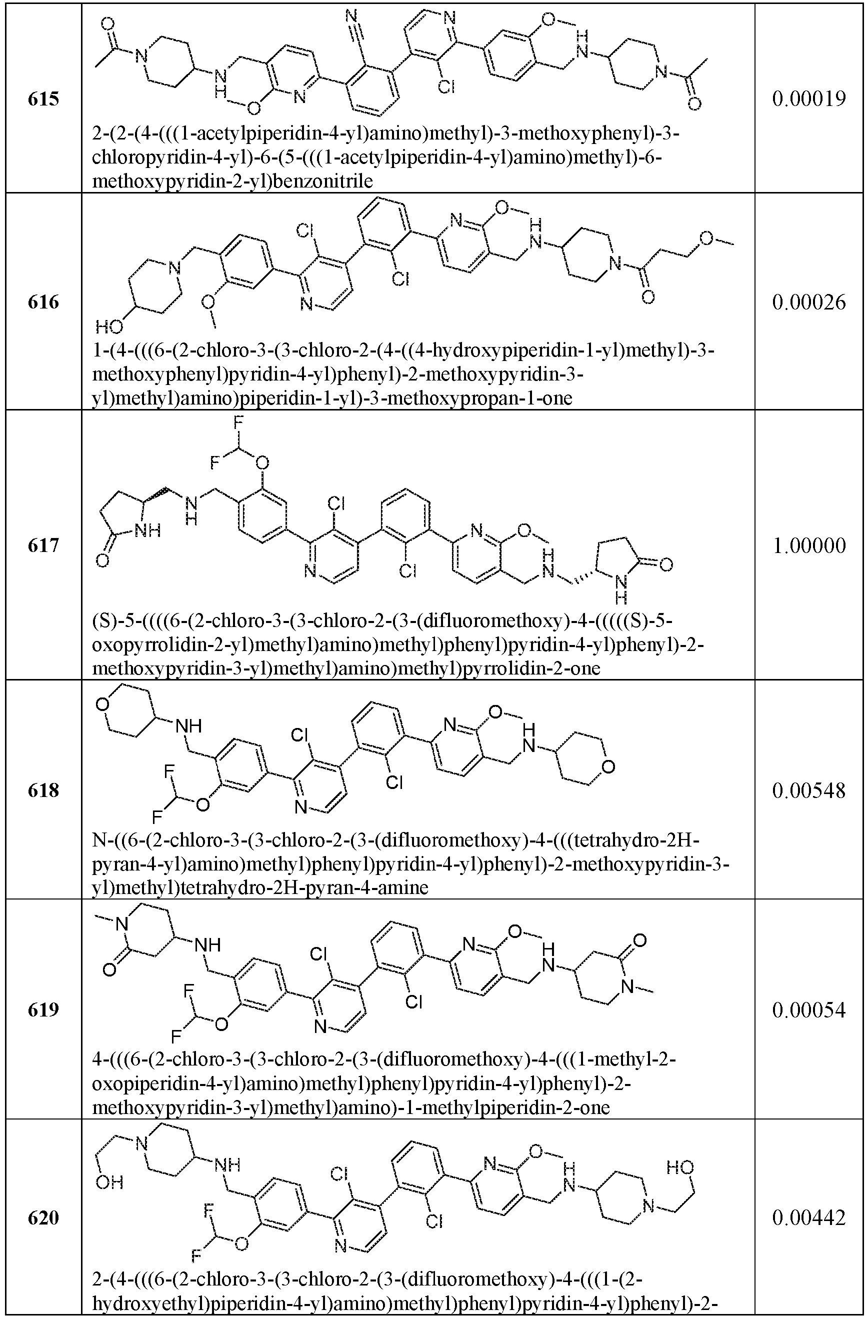

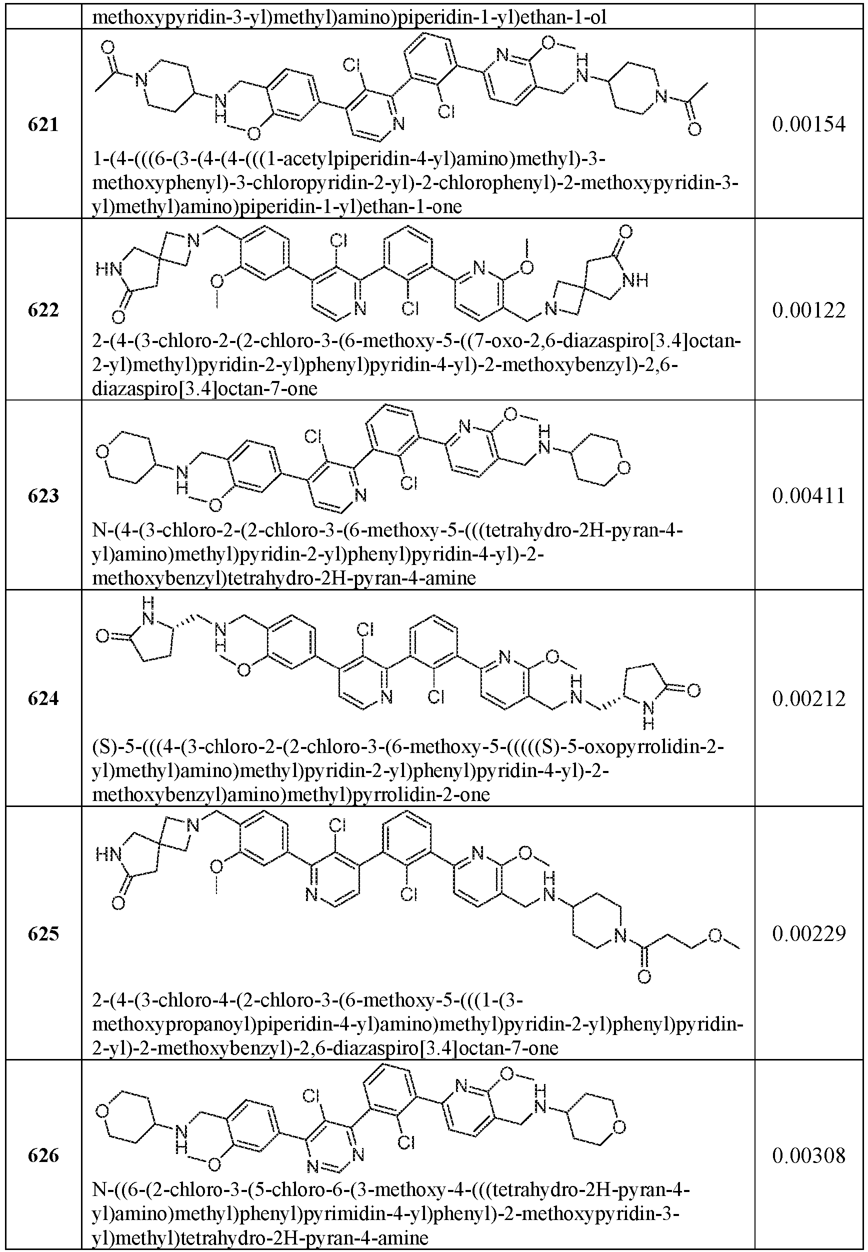

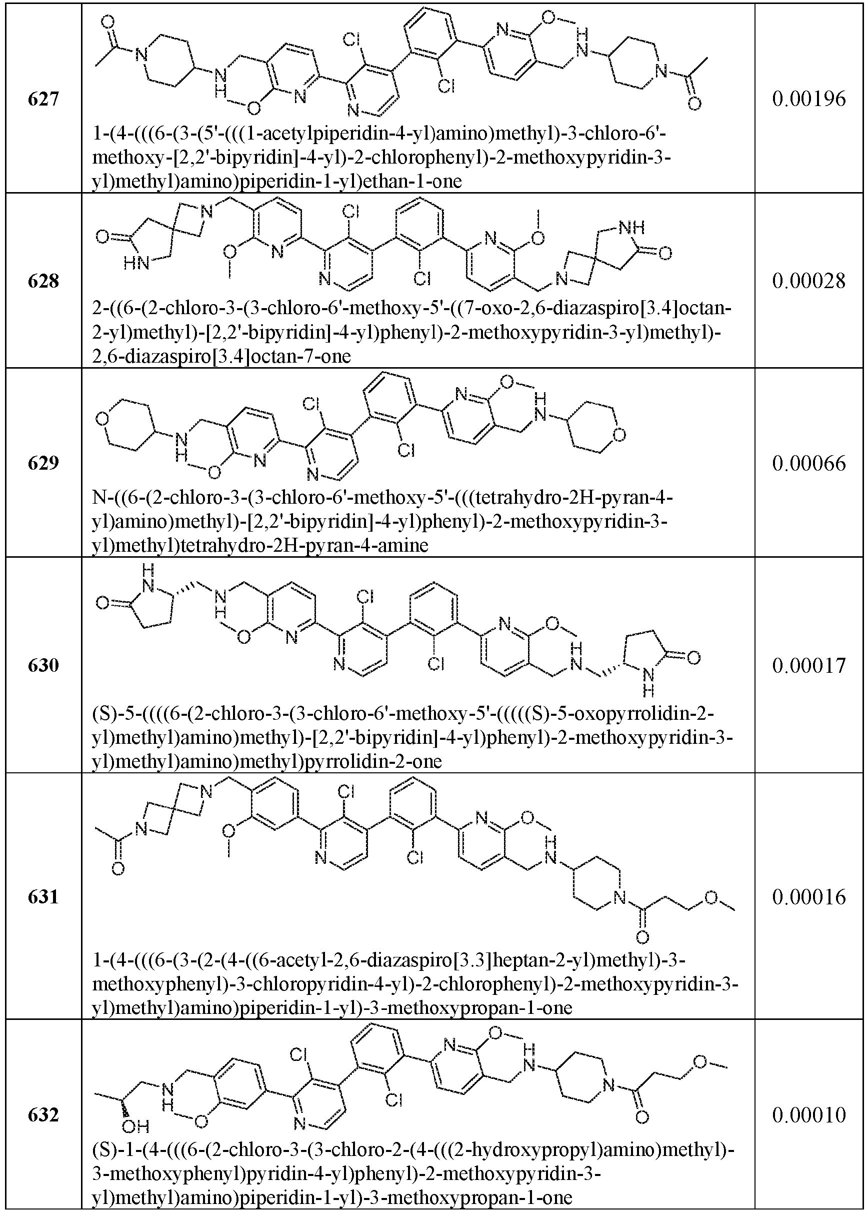

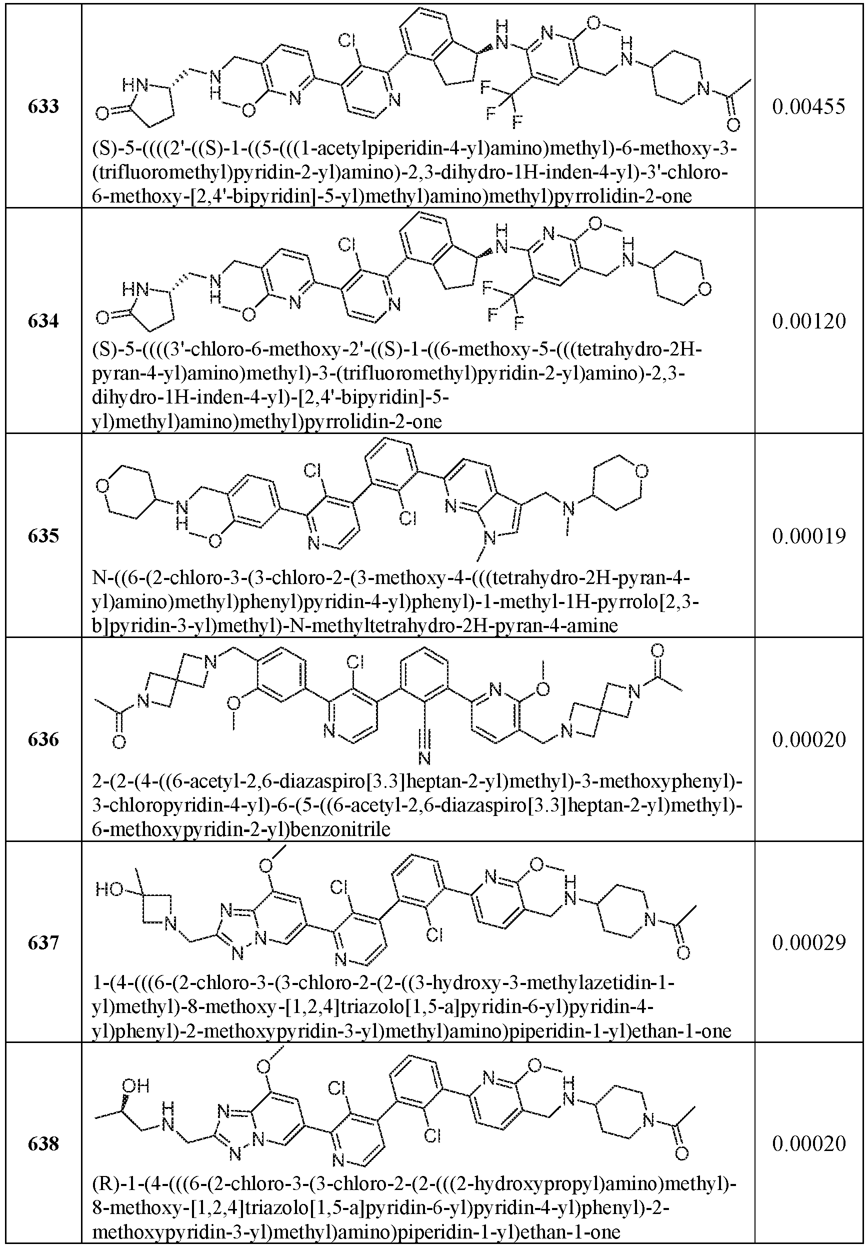

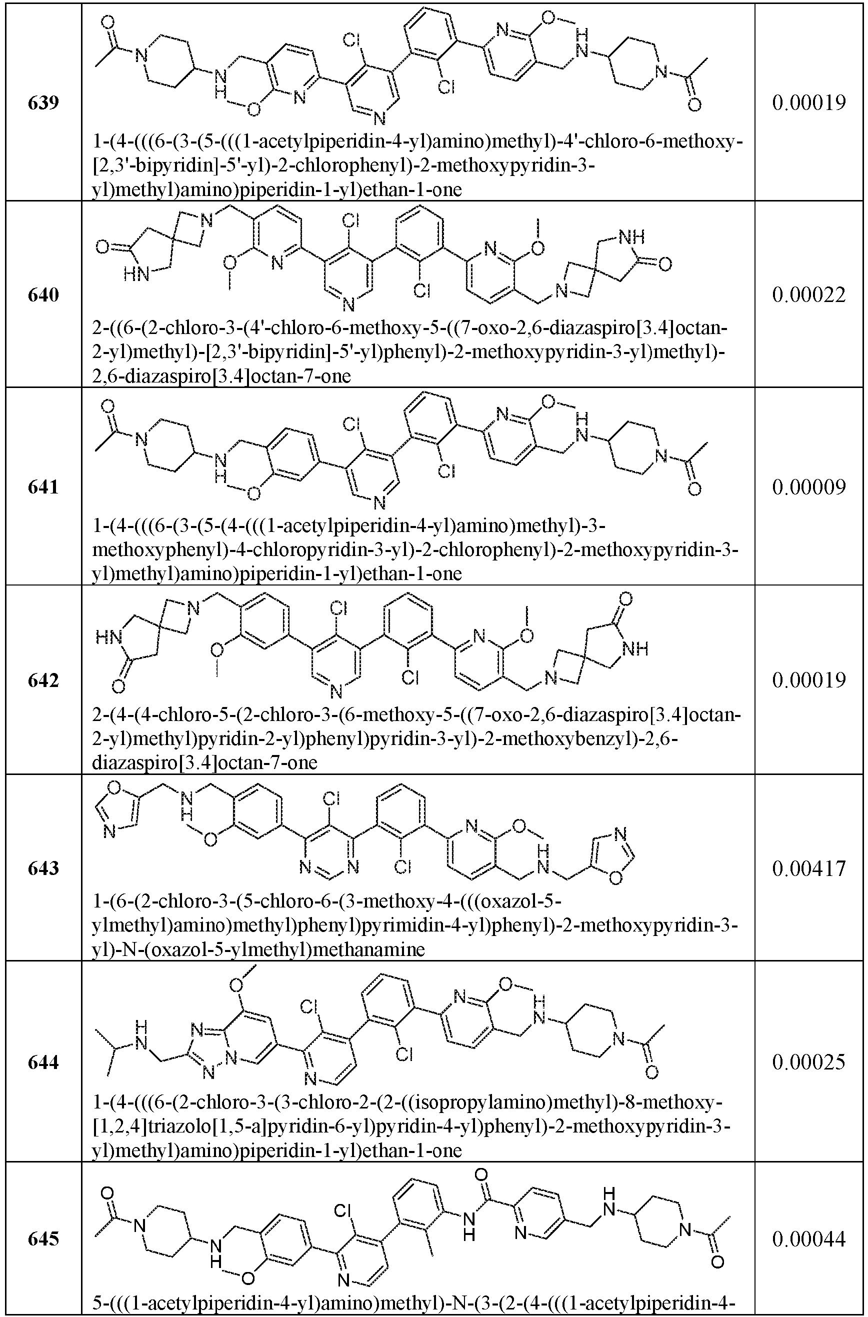

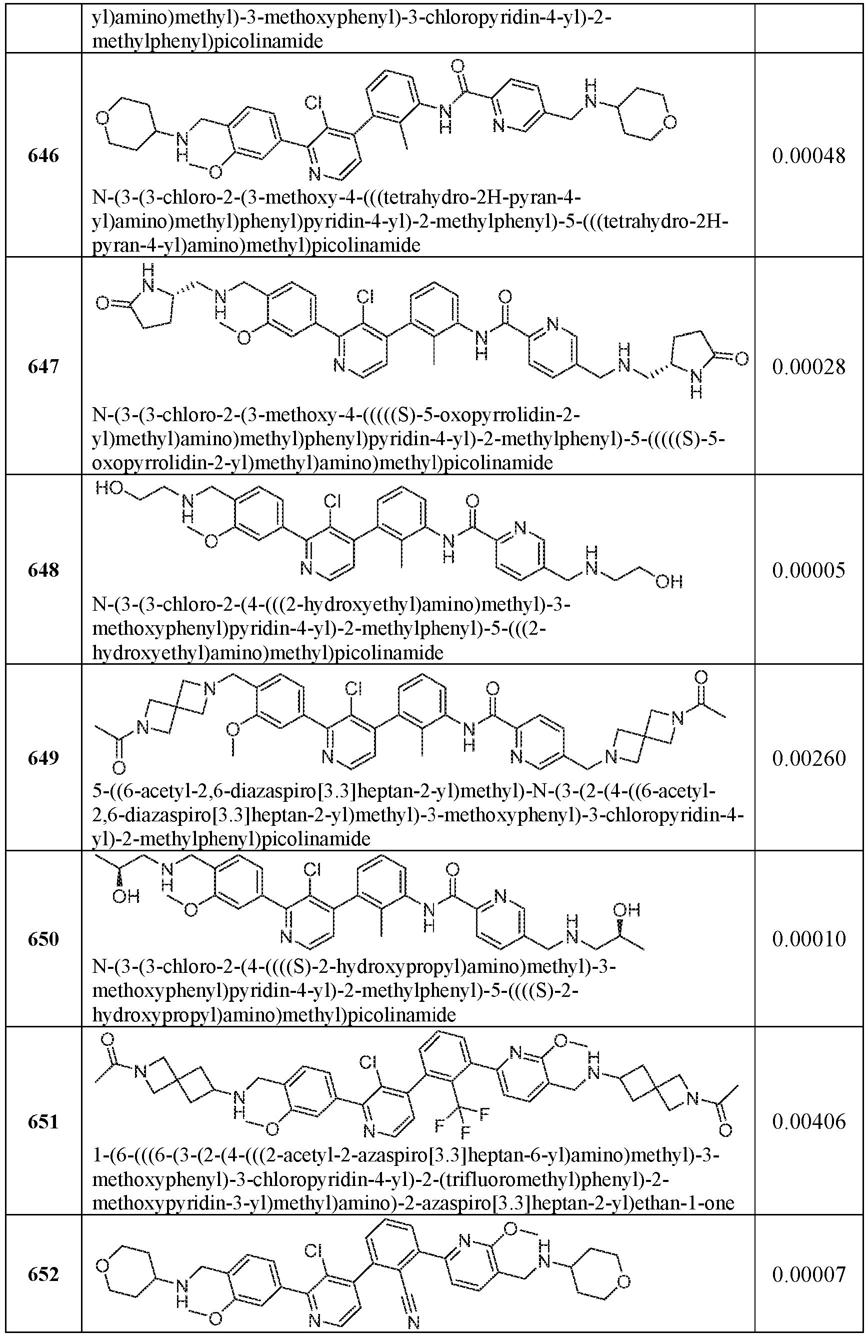

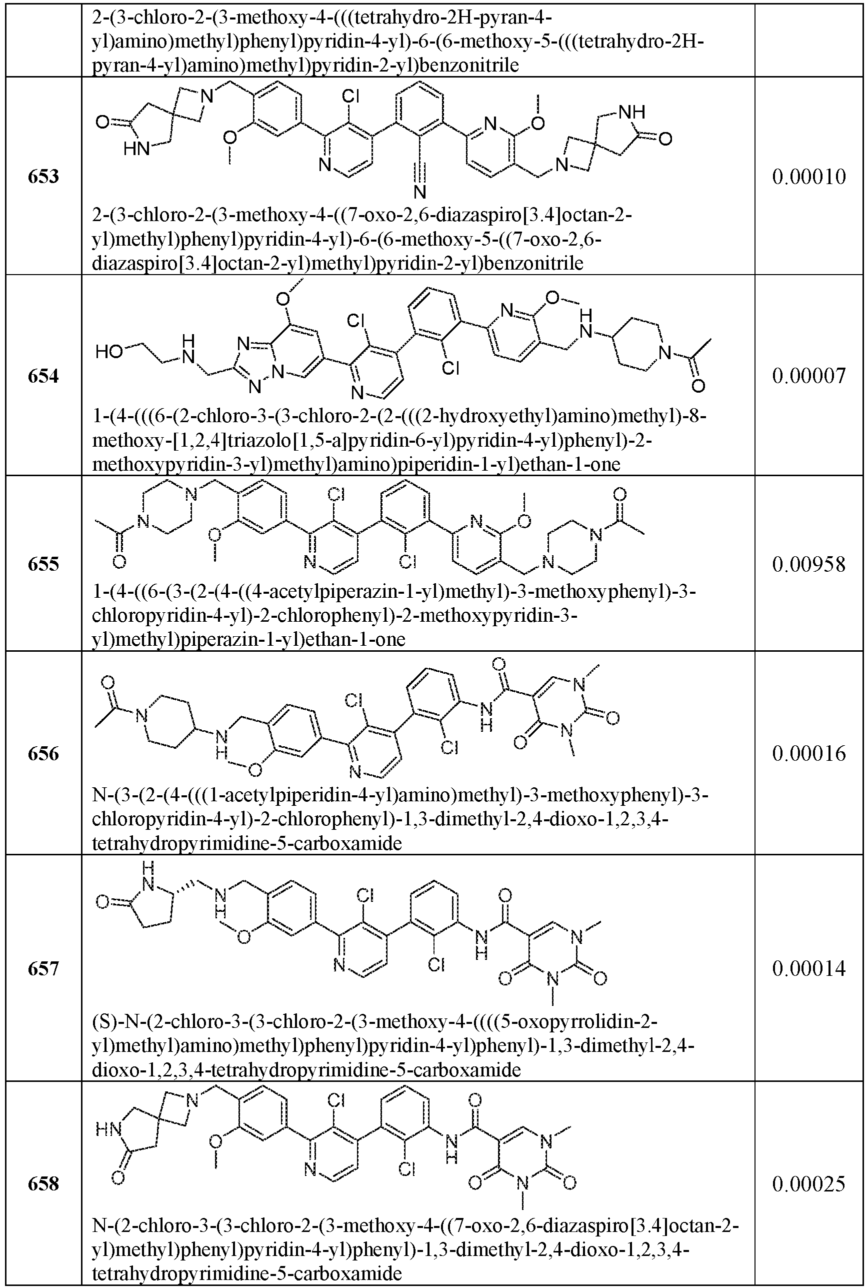

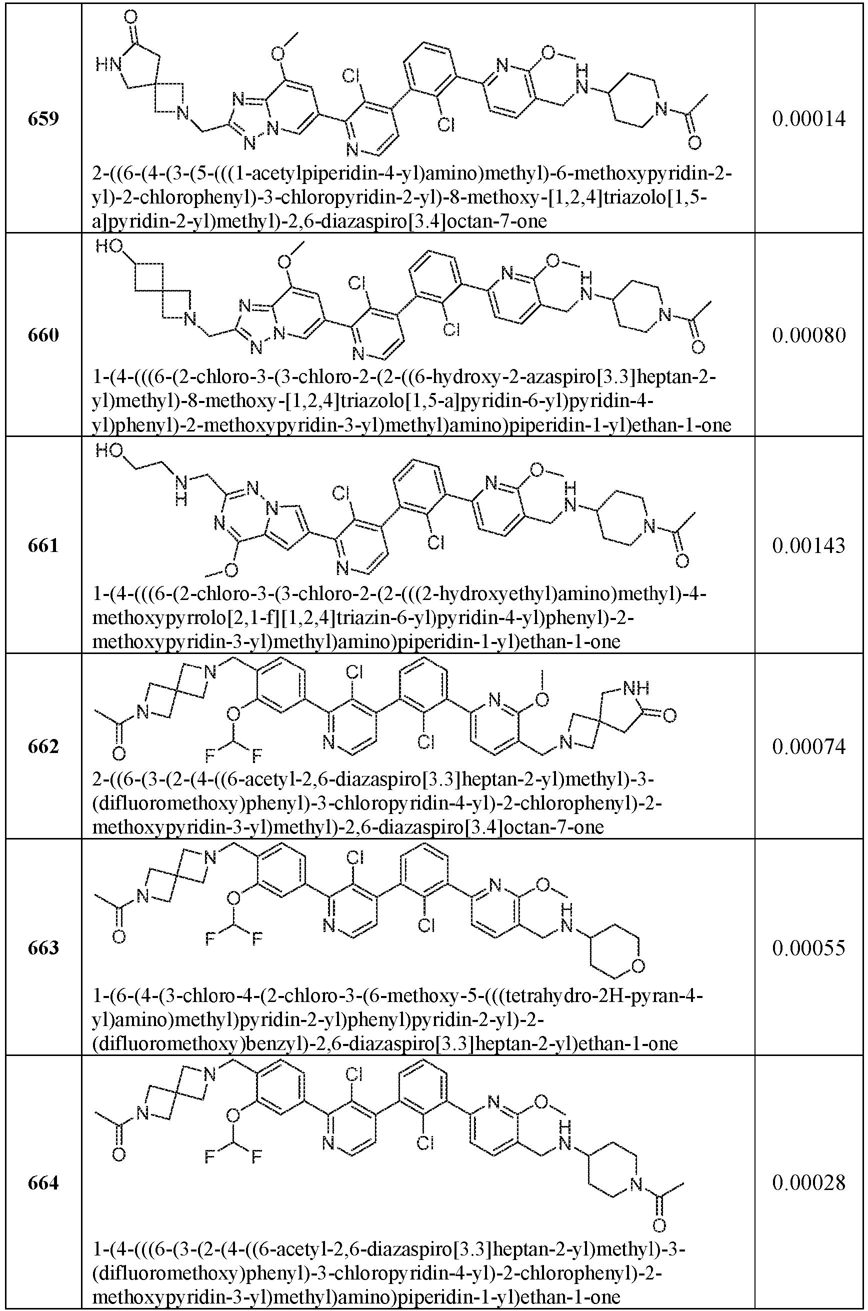

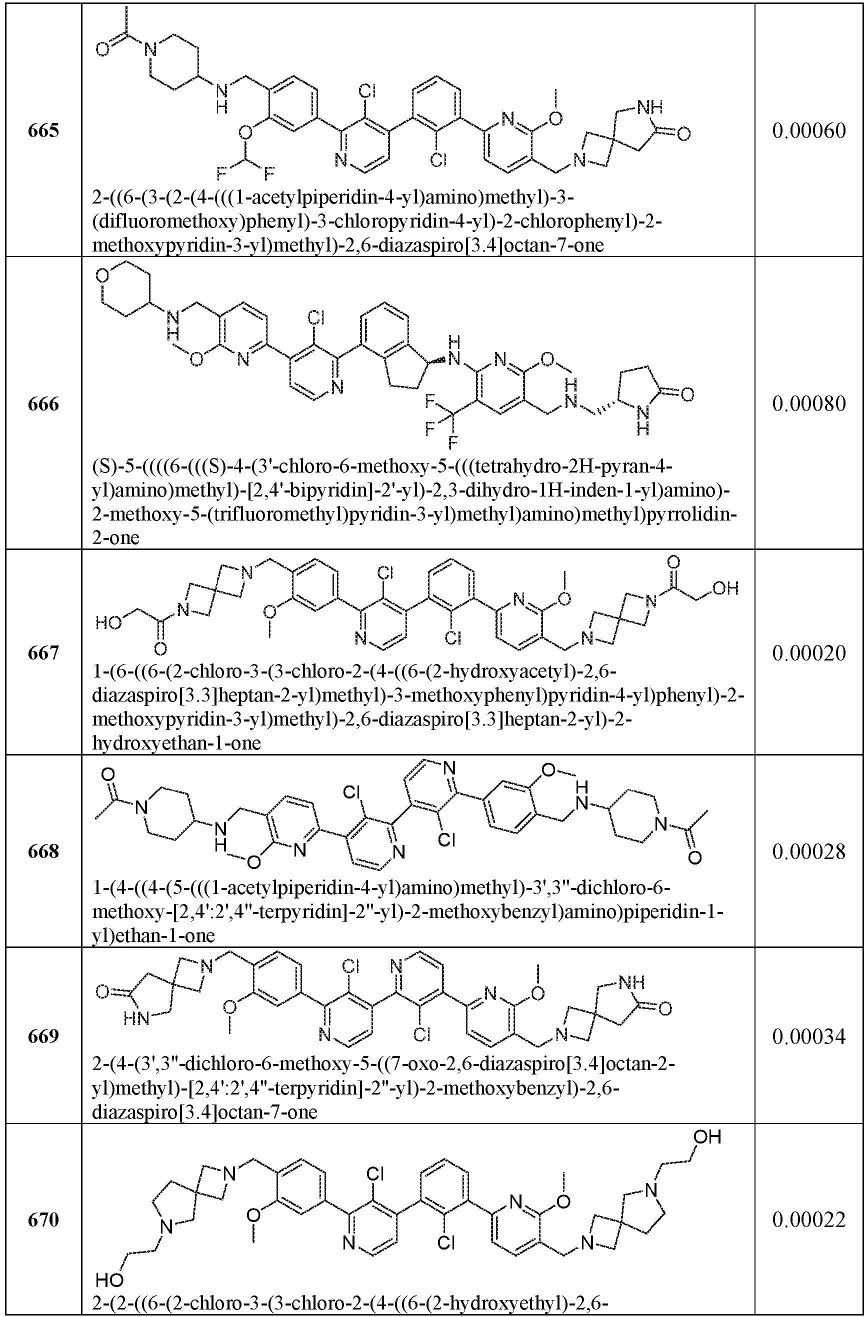

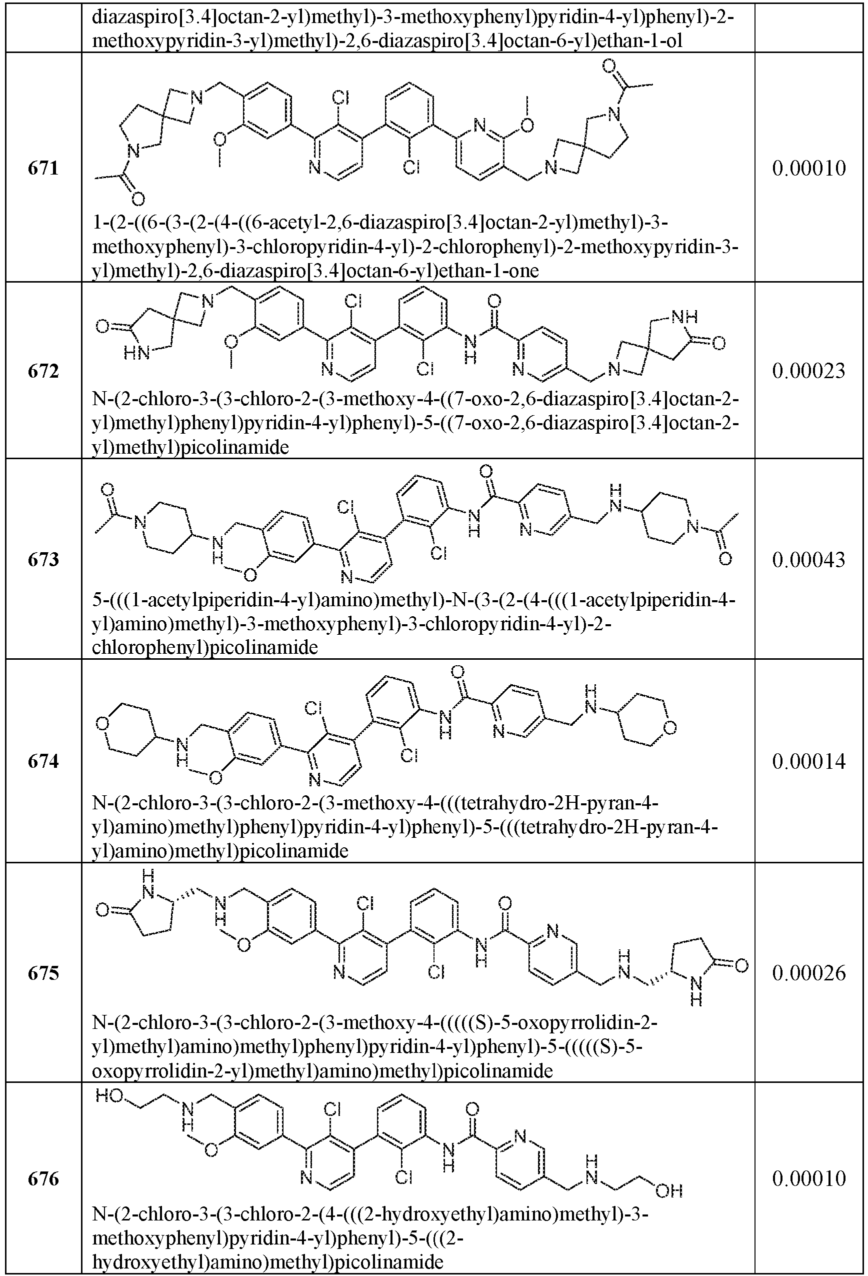

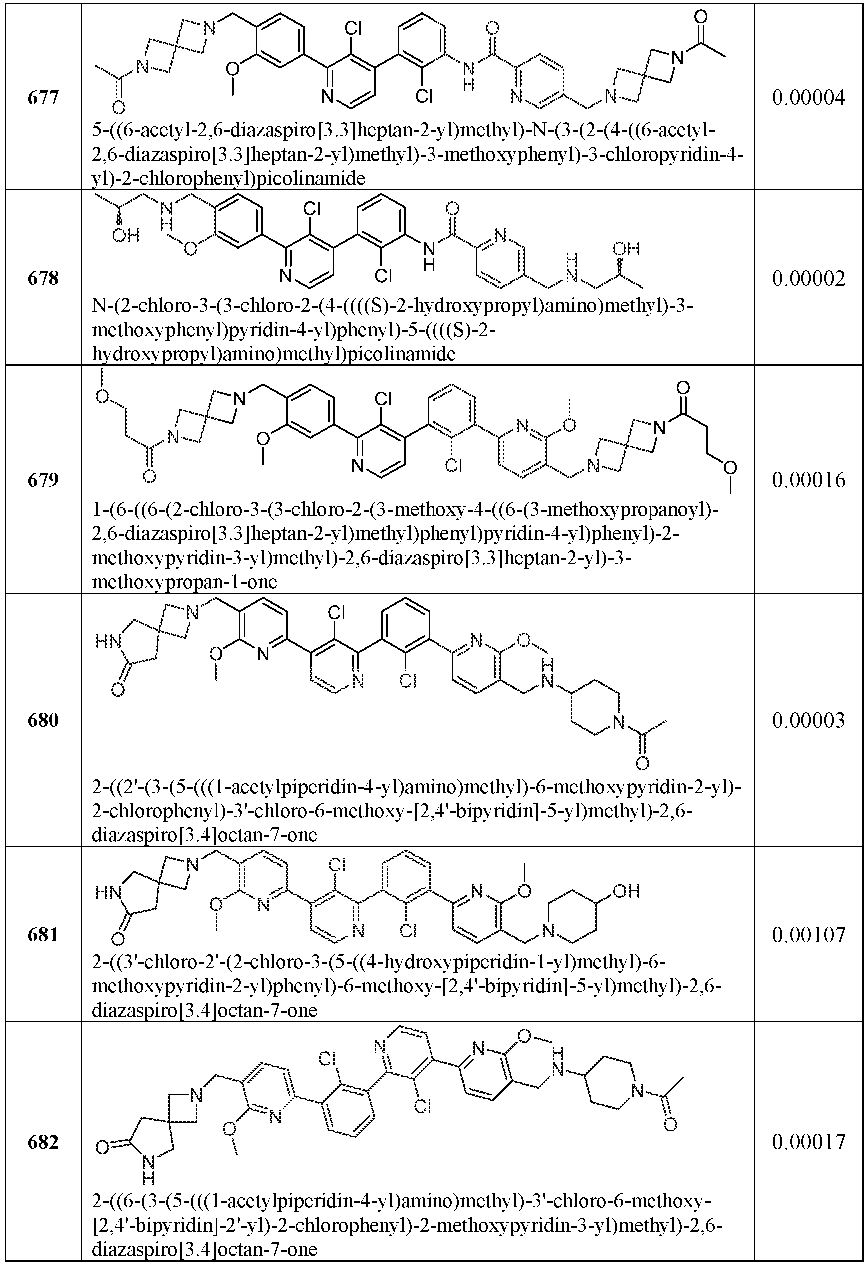

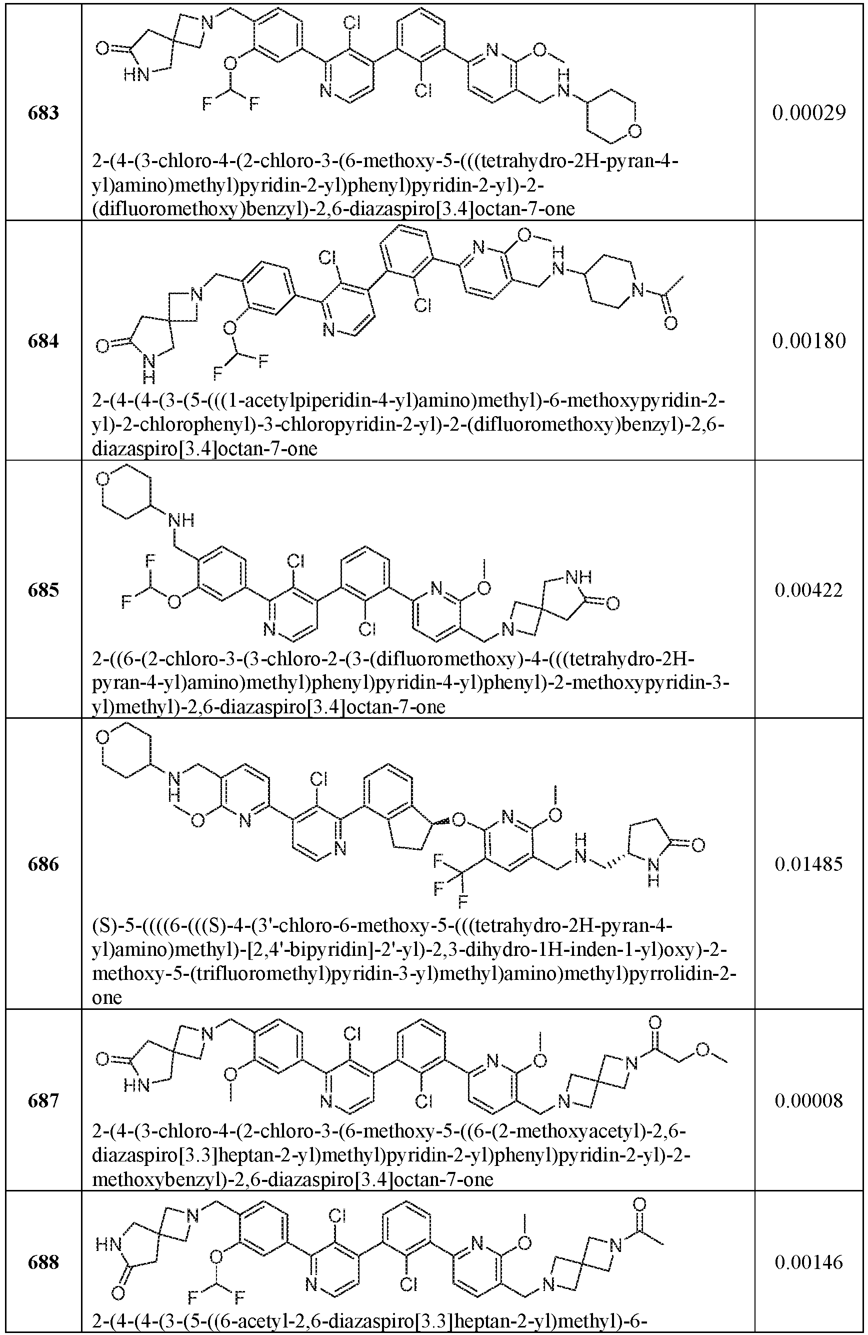

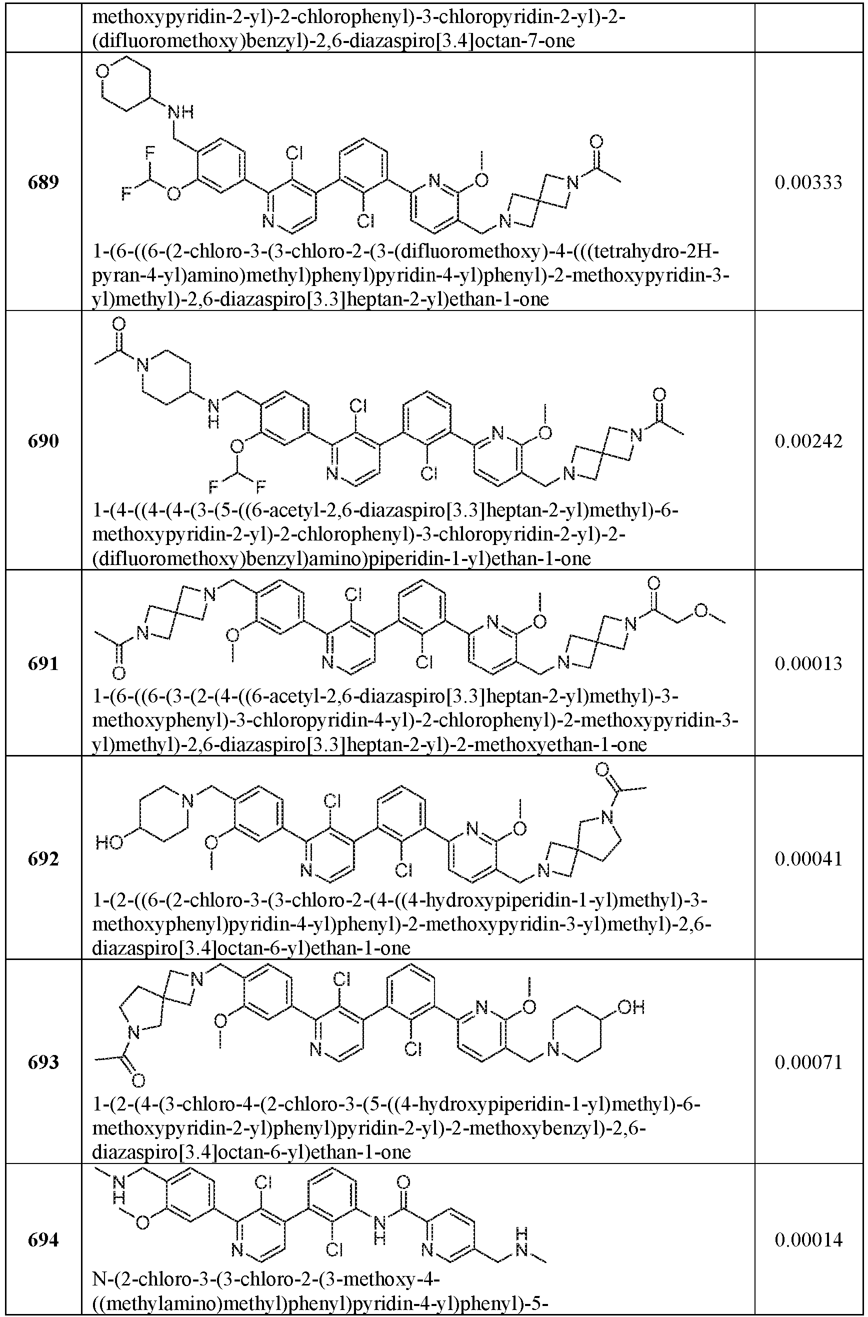

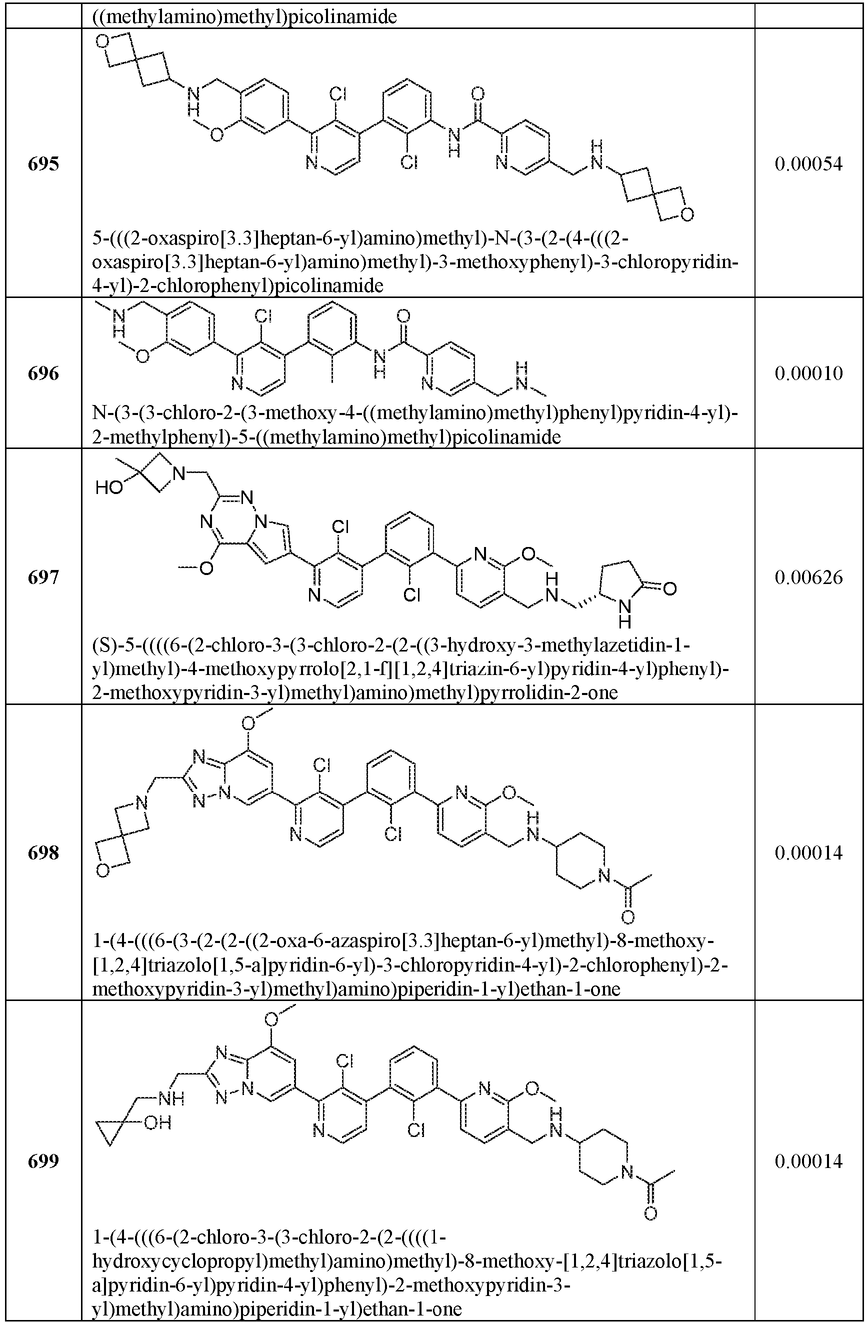

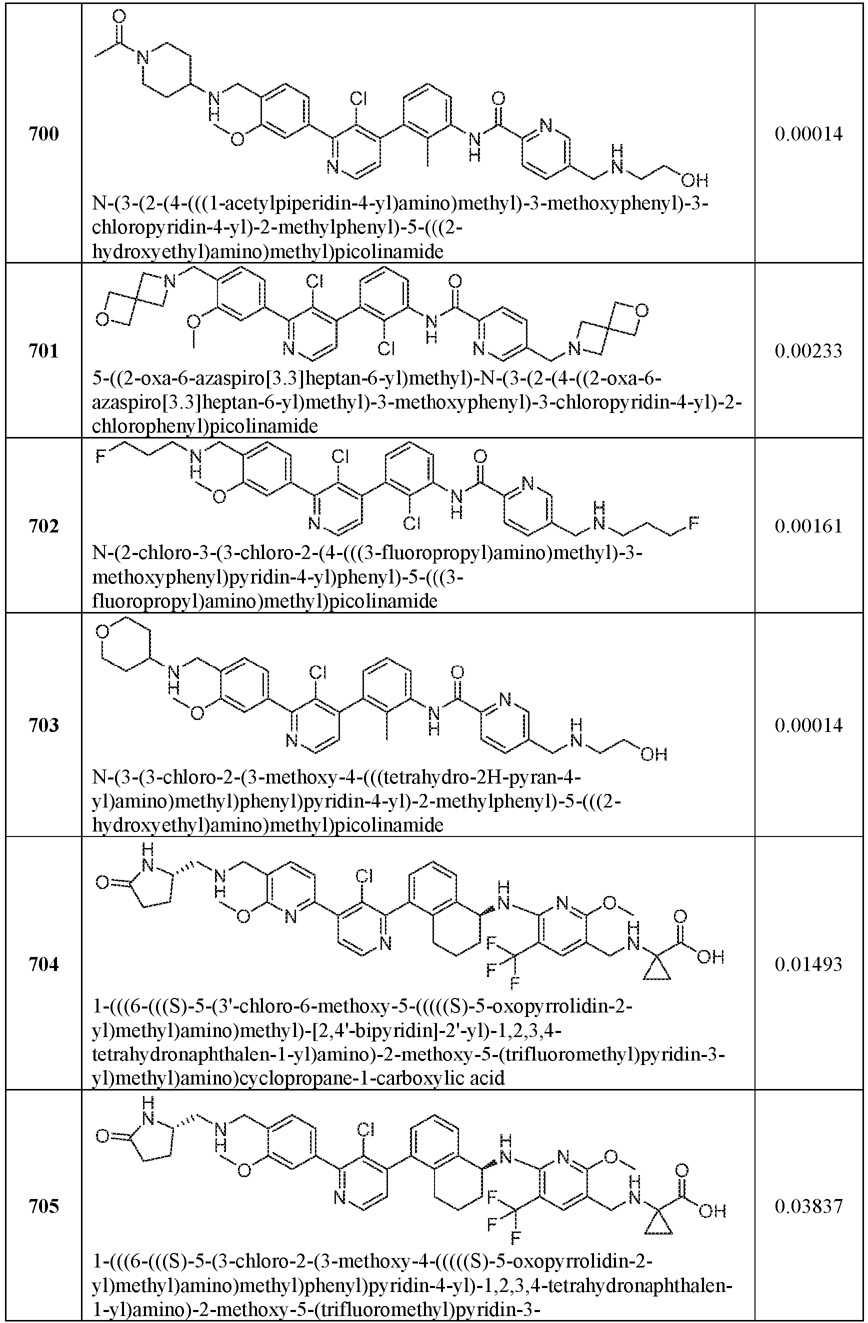

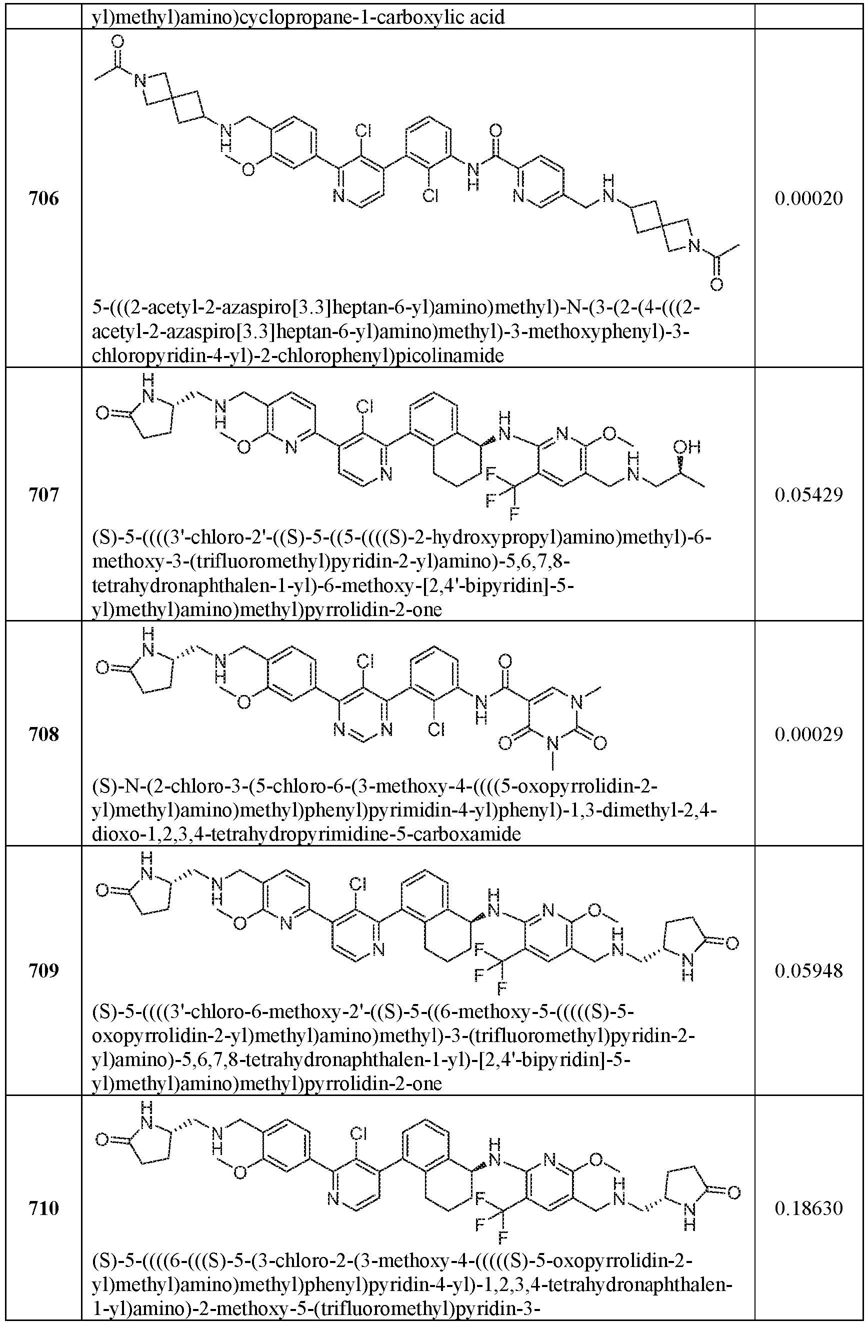

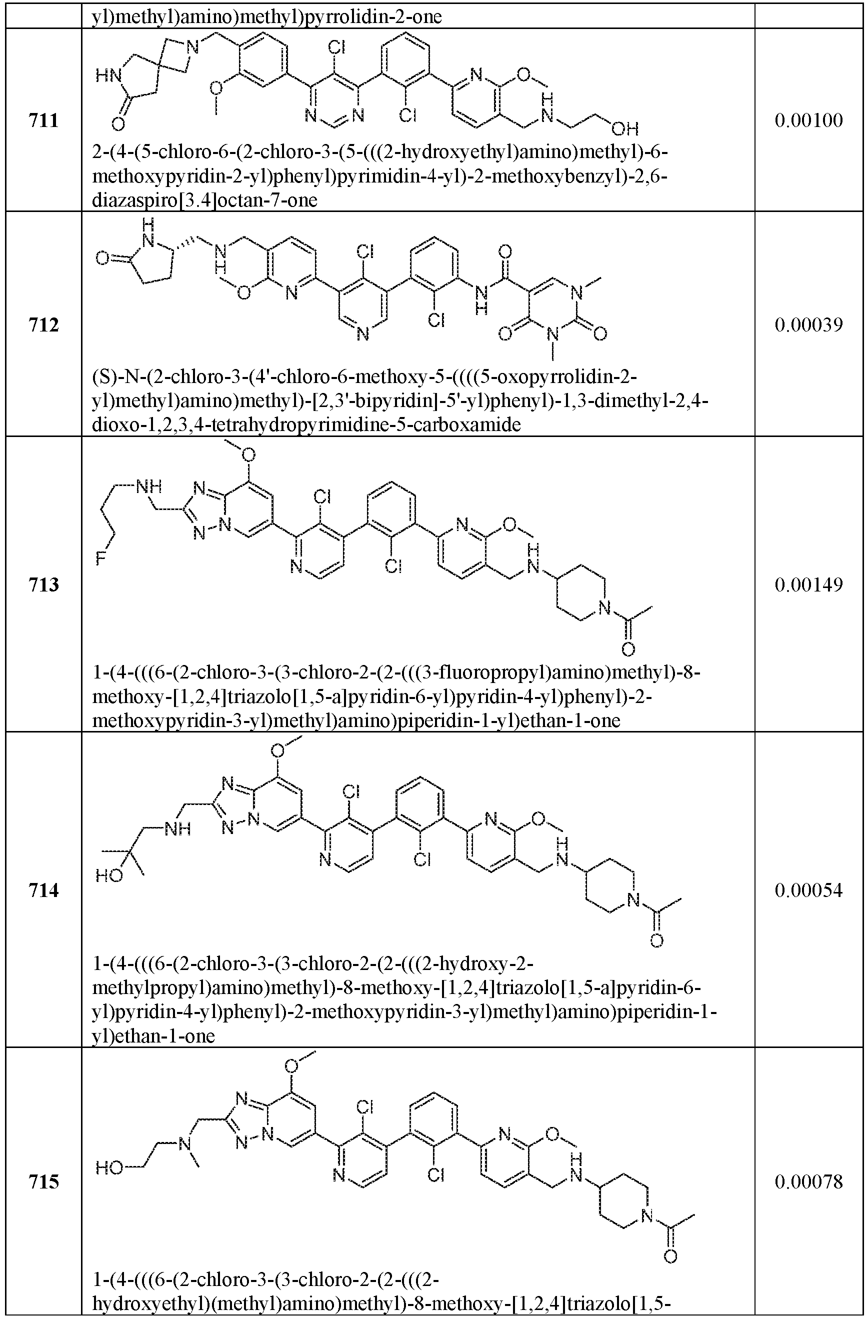

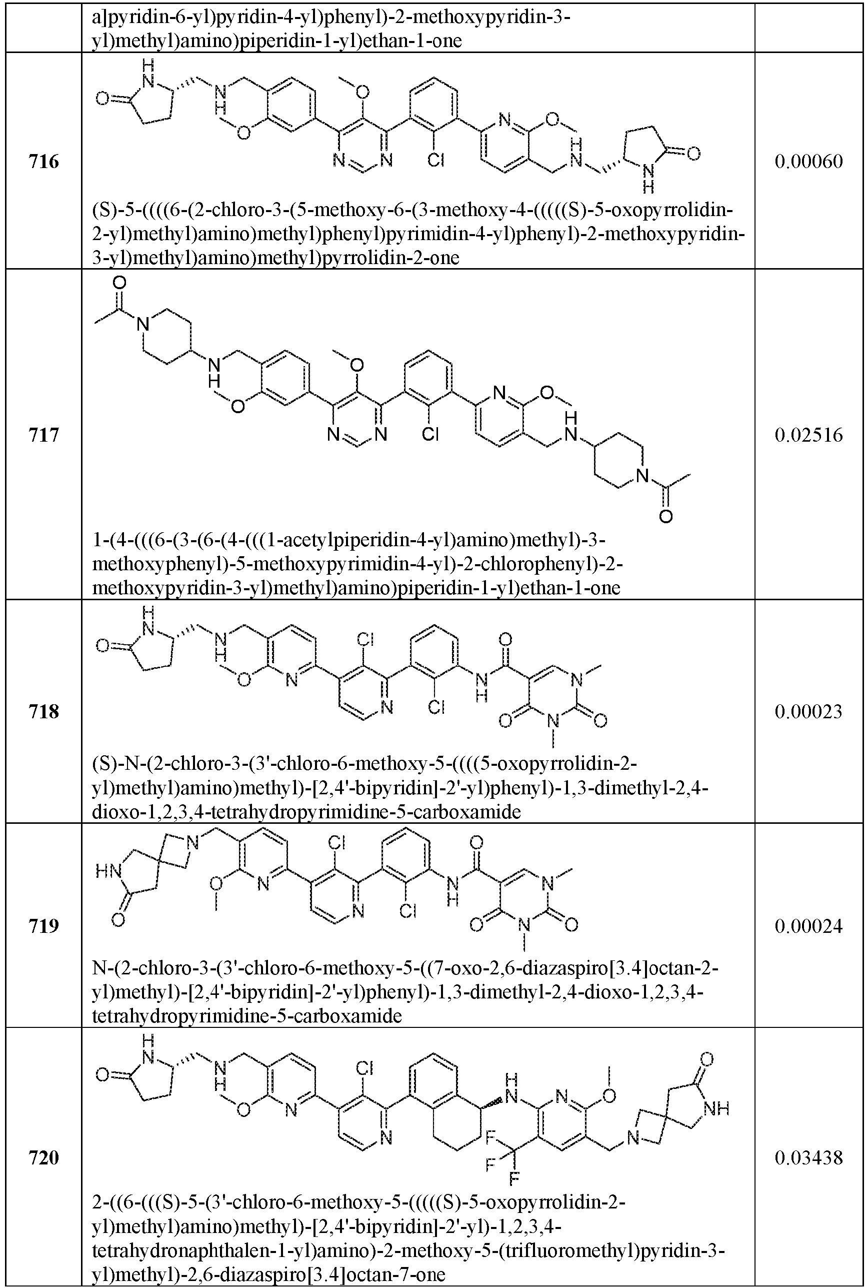

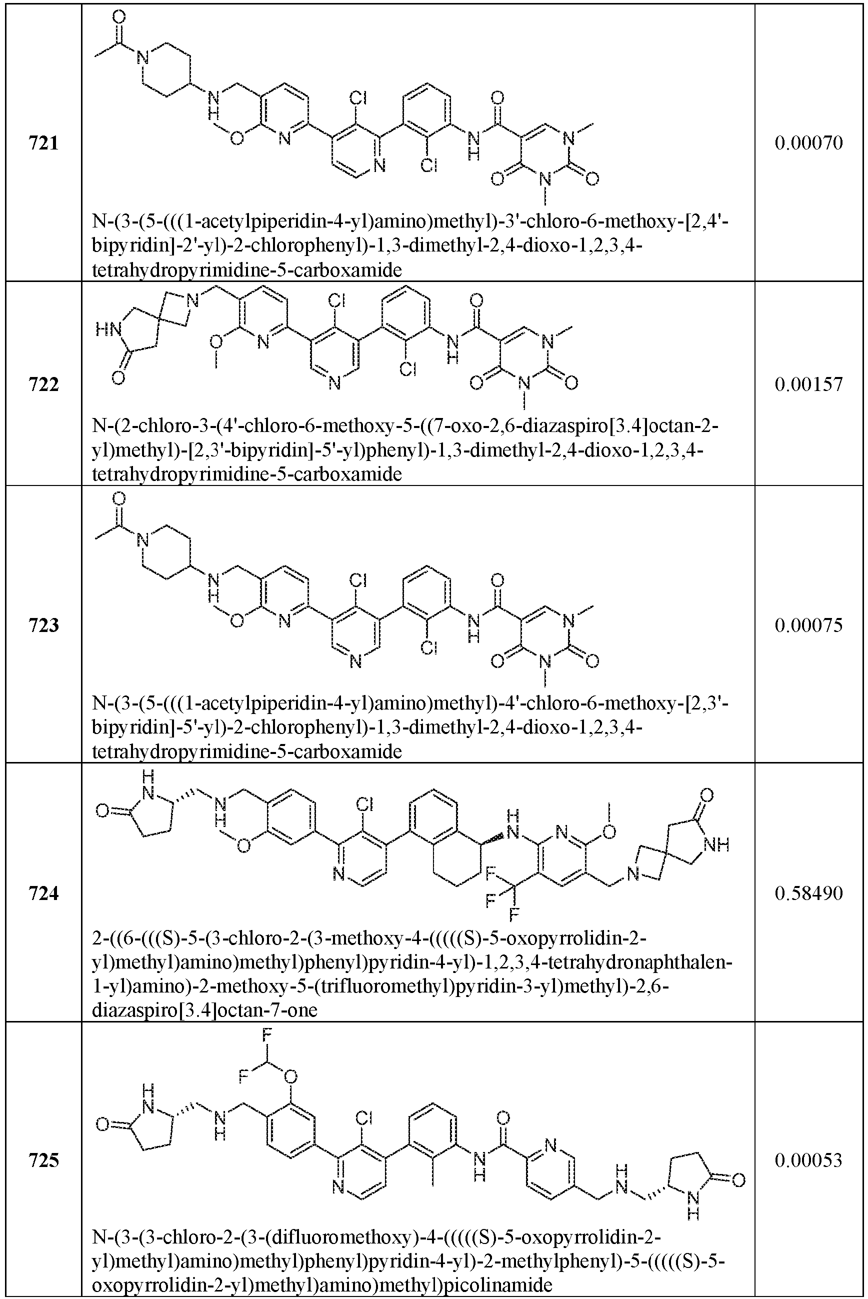

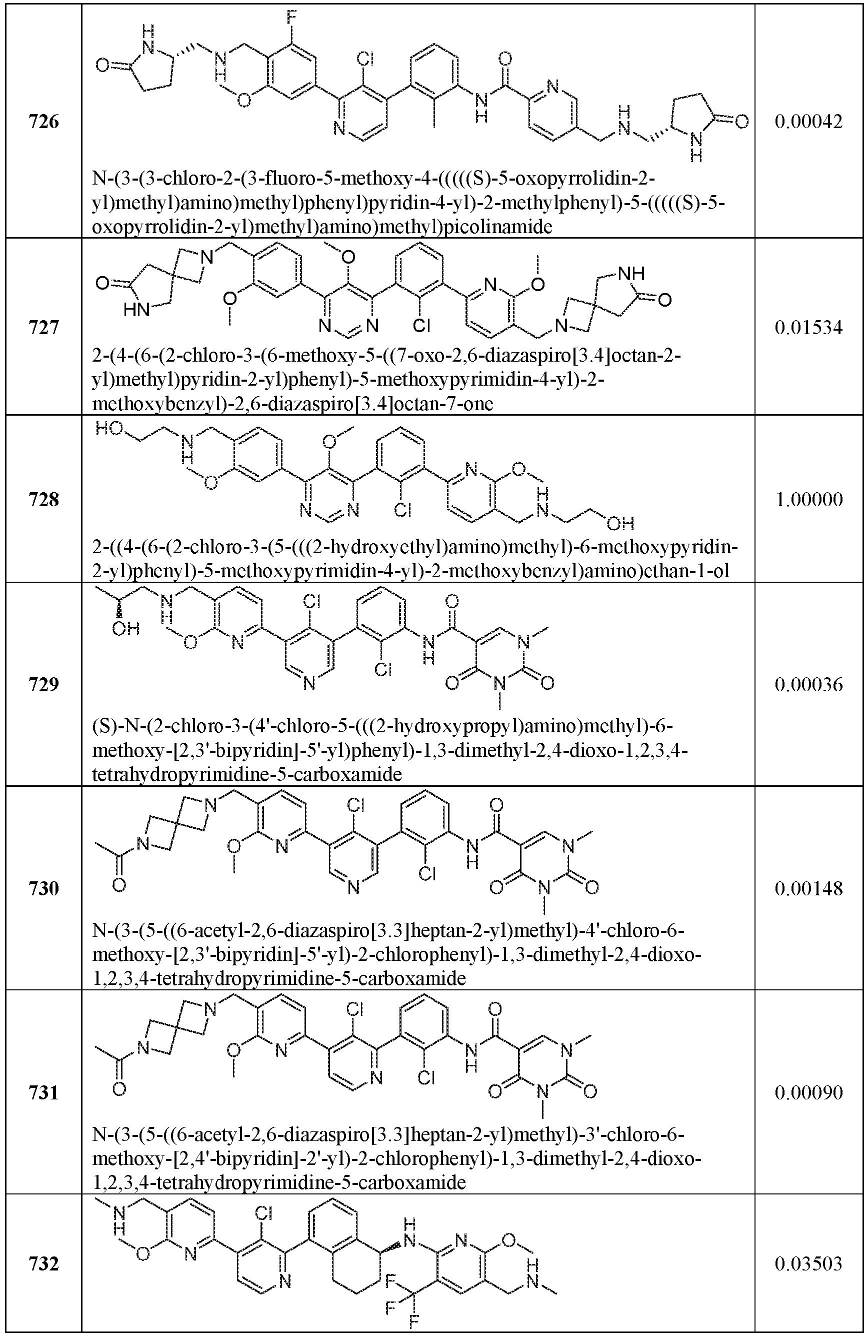

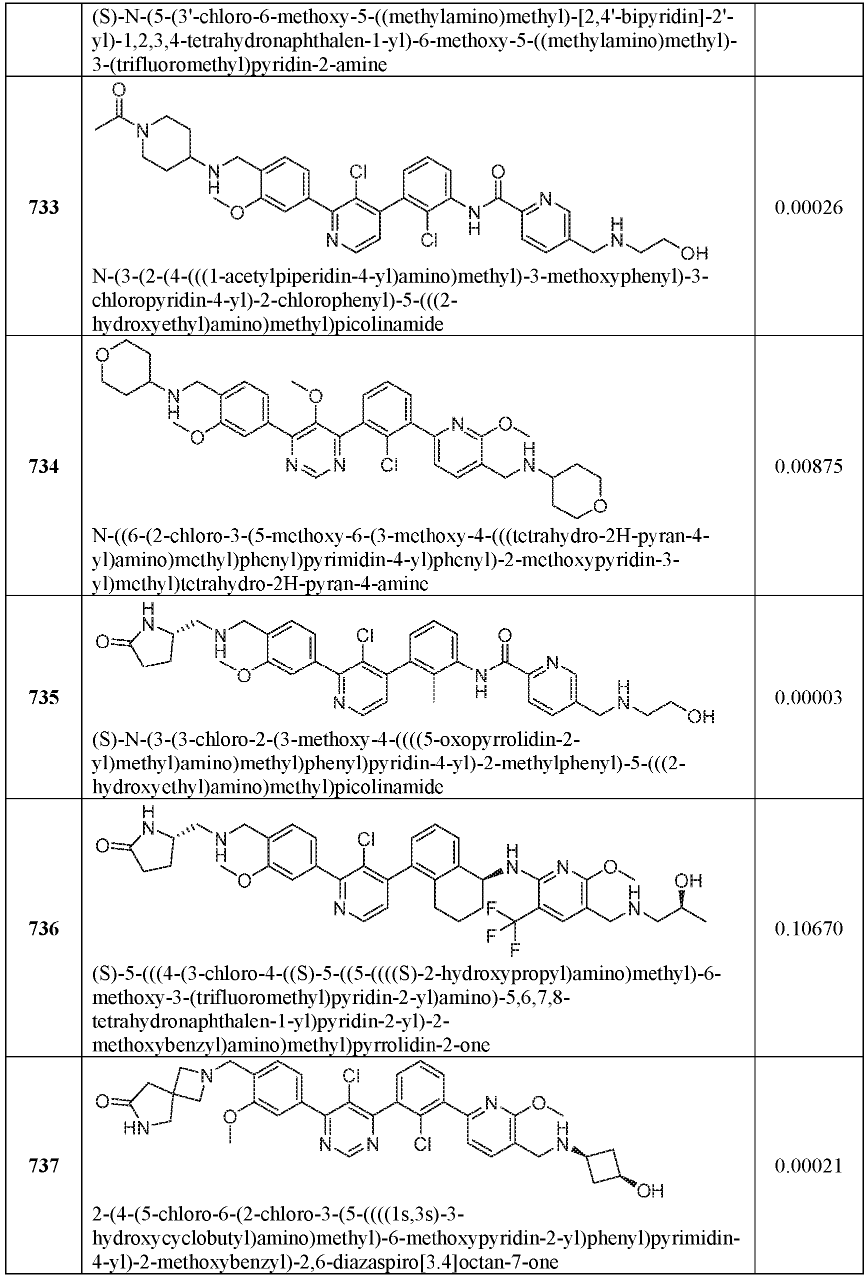

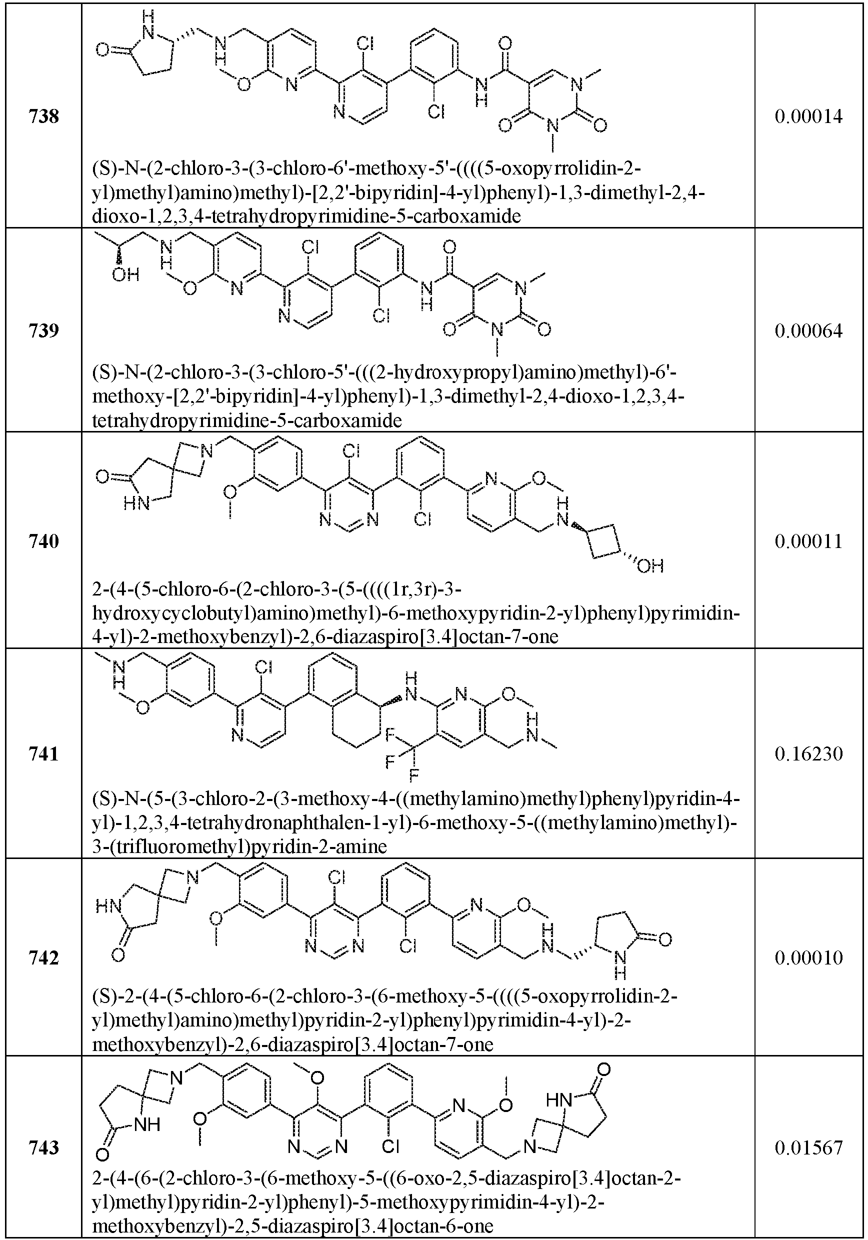

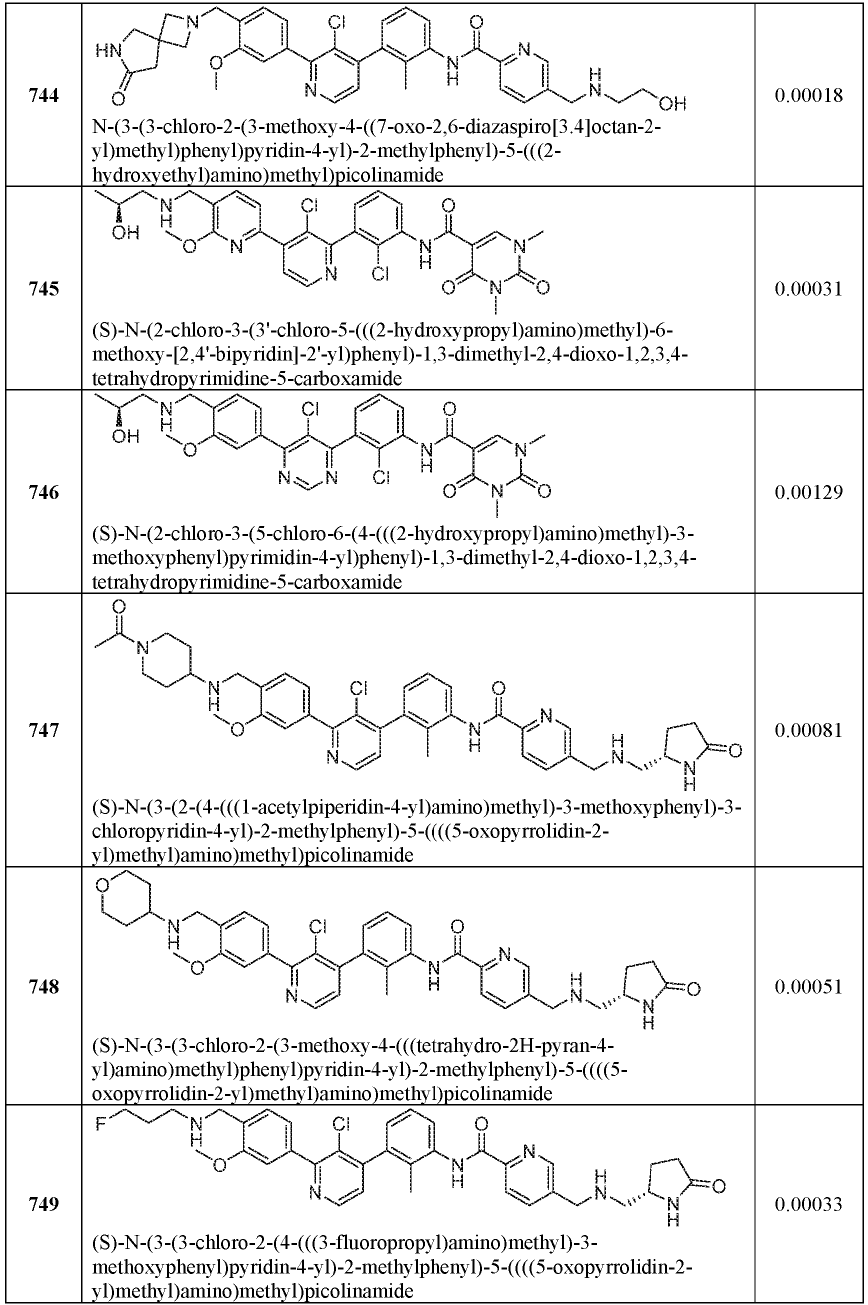

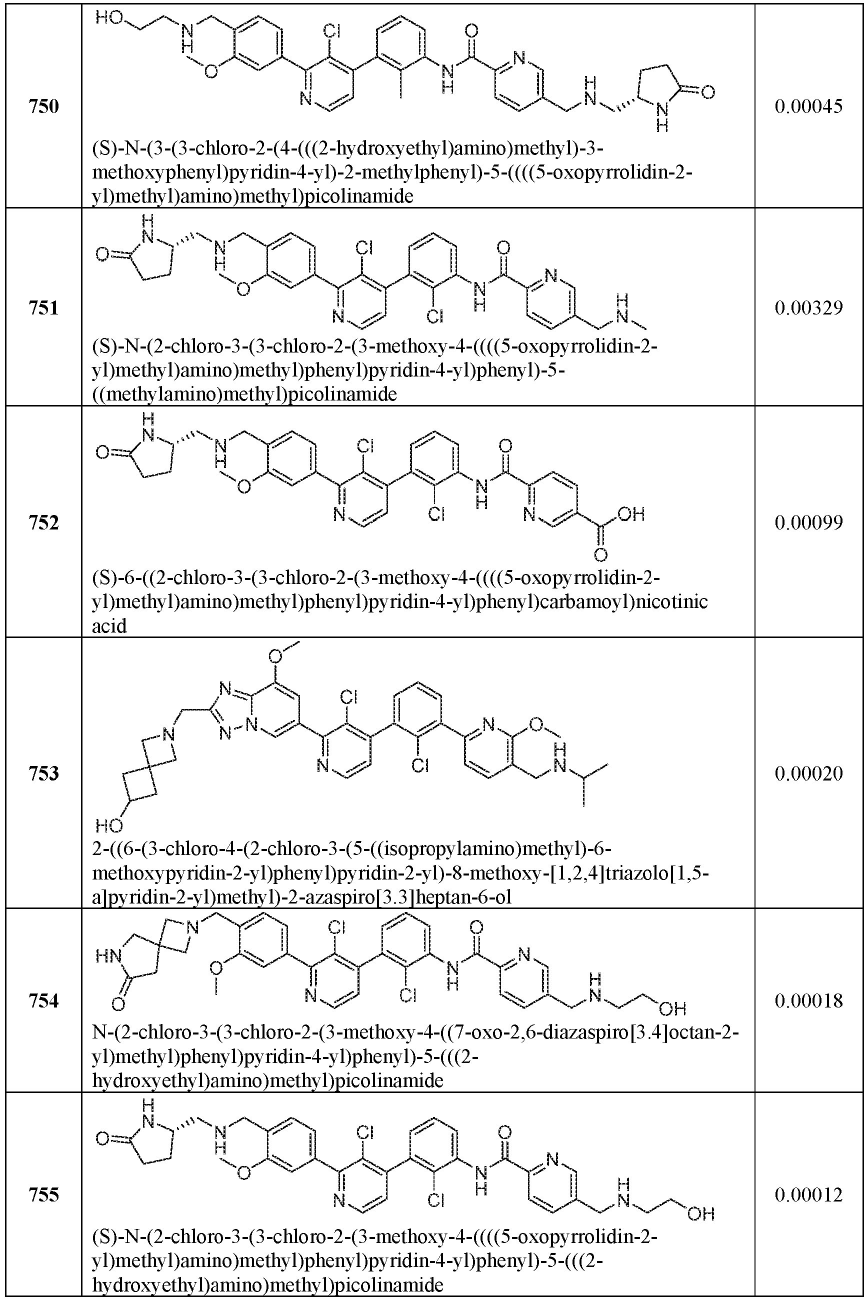

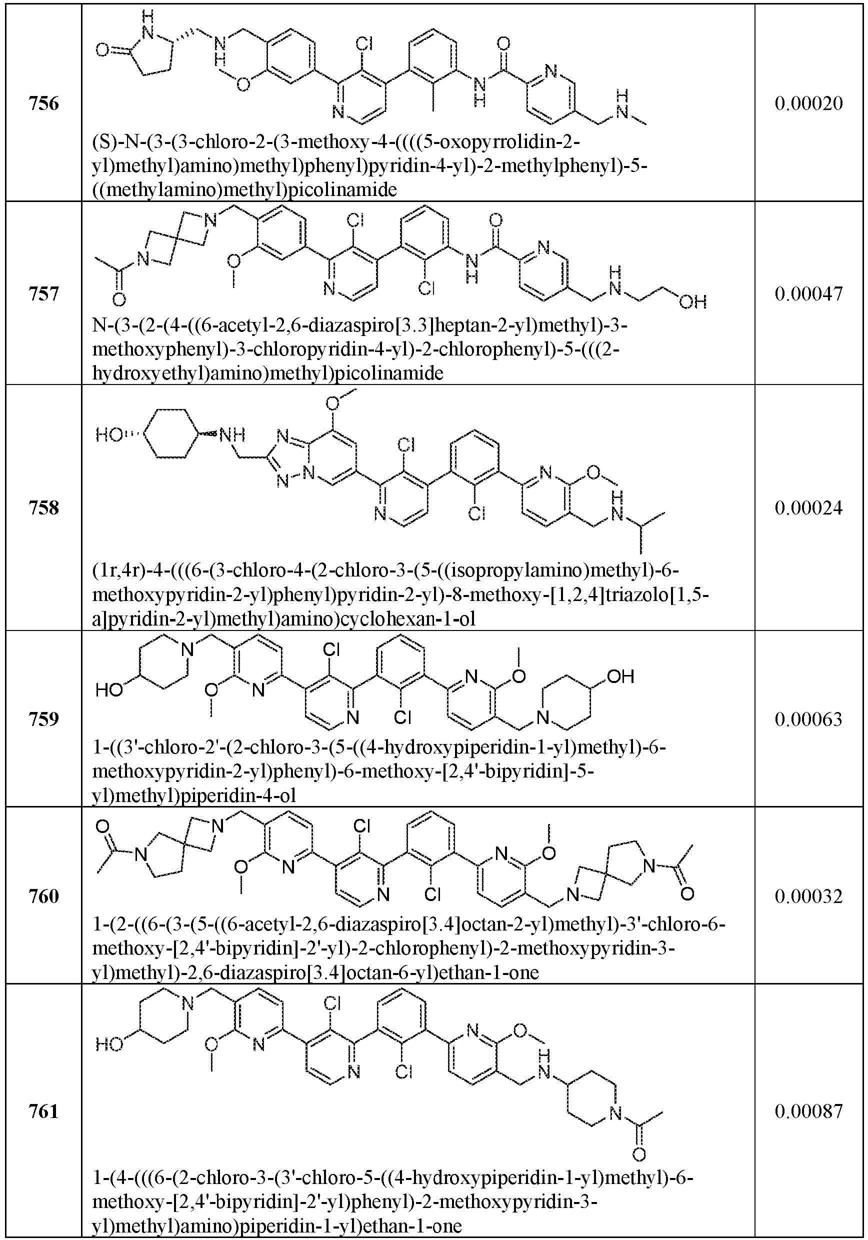

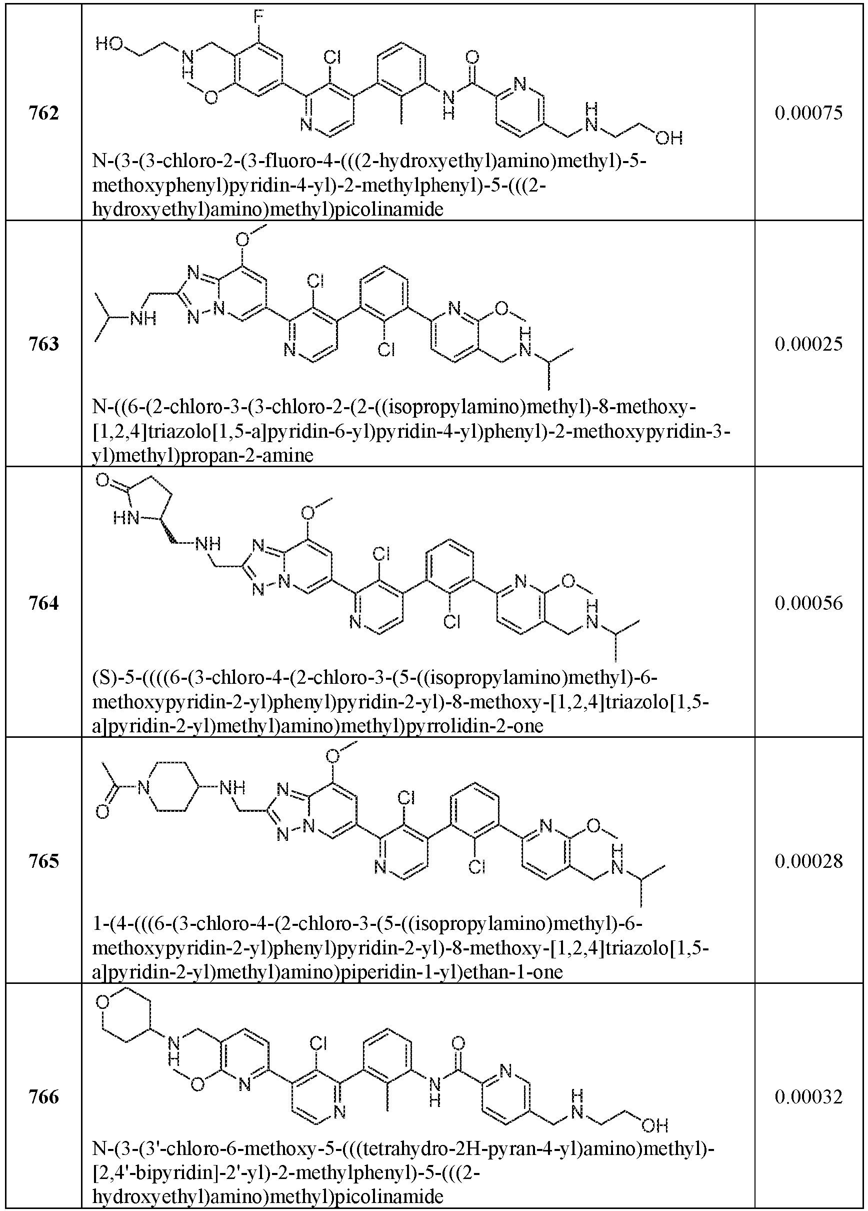

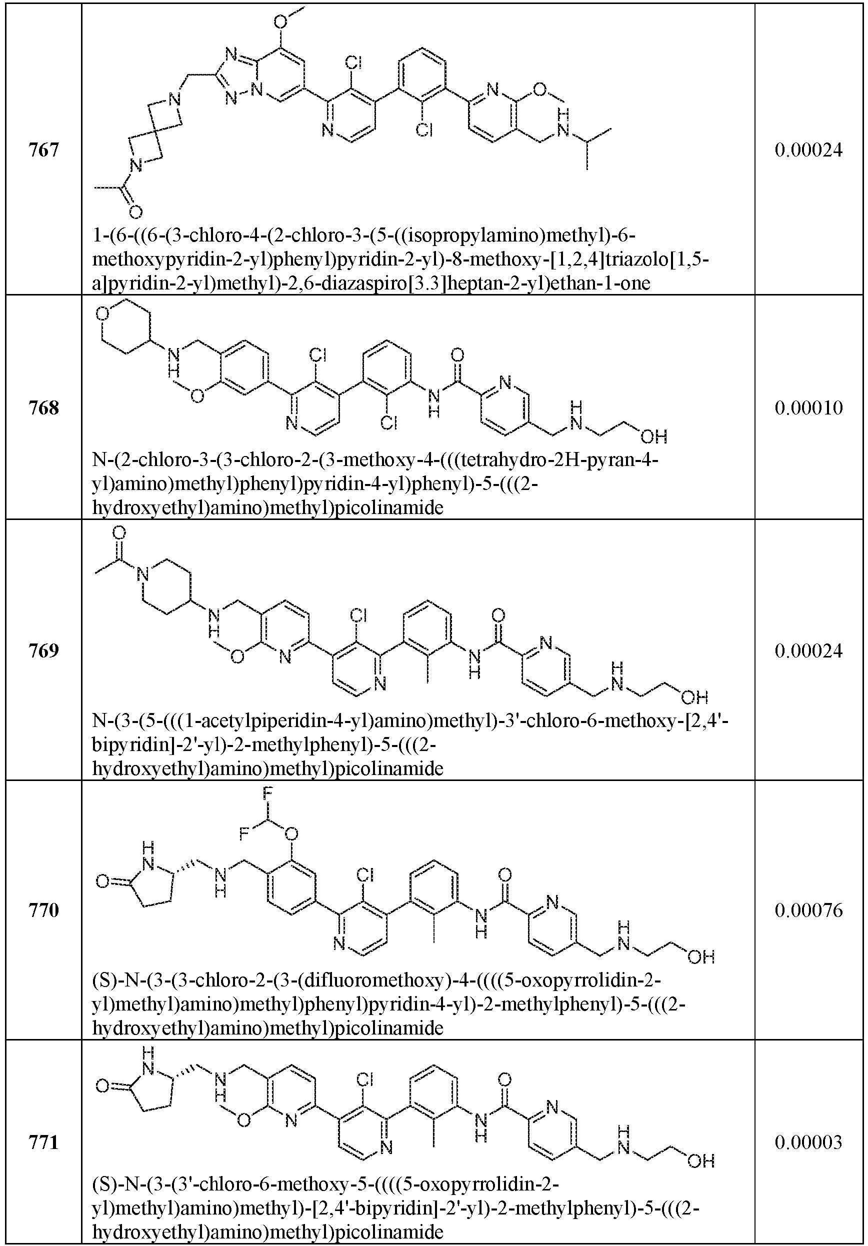

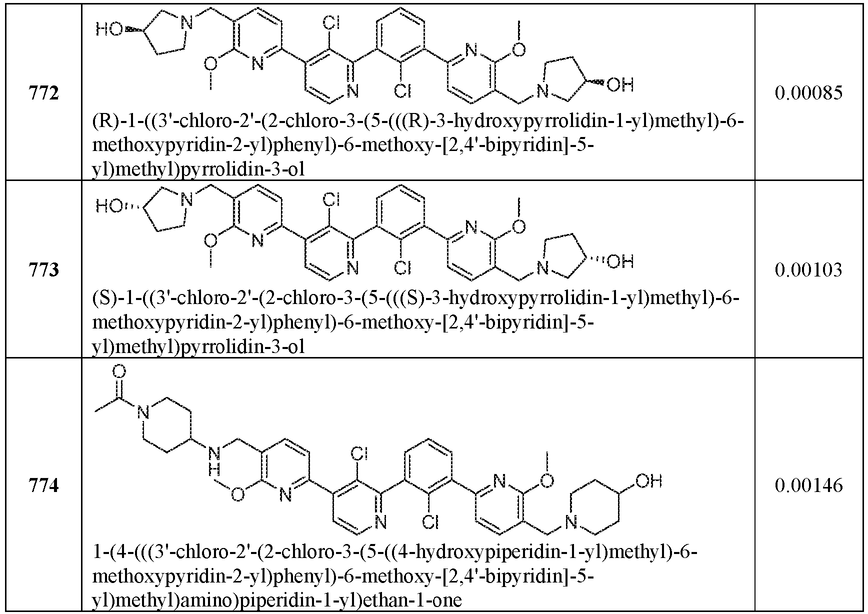

### Example 756: In vitro activity in a T cell activation luciferase reporter cell culture system (T Cell Activation Assay)

In one aspect, this assay measures the ability of a test compound to bind to and inhibit activity of PD-L1. PD-1/PD-L1 binding has the net effect of inhibiting T cell receptor signaling. A test compound that is capable of binding to and inhibiting PD-L1 will disrupt PD-1/PD-L1 binding and thus cause an increase in T cell receptor signaling.

The ability of compounds to mediate T cell activation in a T cell activation luciferase reporter cell culture system was assessed as described previously (Park et al., Nature Communications, 2021; 12:1222). CHO-K1 cells stably expressing human PD-L1 and a cell surface protein designed to activate cognate T cell receptor in an antigen-independent manner (PD-L1 aAPC/CHO-K1, Promega) were incubated with PD-1 effector Jurkat T cells stably expressing human PD-1 and NFAT-transcription factor-induced luciferase in the absence or presence of small molecules or anti-PD-L1 blocking antibody at indicated concentration range according to the manufacturer's instruction (Promega). Bio-Glo^{™} Reagent was added and luminescence quantified as a signal for Jurkat T cell activation. In the absence of PD-L1 inhibition, the PD-1/PD-L1 interaction in this co-culture system results in inhibition of T cell receptor signalling and inhibition of NFAT-mediated luciferase activity. Disruption of PD-1/PD-L1 interaction with compound or antibody treatment results in luciferase activity.

**Table 2. T Cell Activation EC₅₀ Values in Luciferase Reporter Assay**

| **Cmpd** | **T Cell Activation EC₅₀ (µM)** | **Cmpd** | **T Cell Activation EC₅₀ (µM)** | **Cmpd** | **T Cell Activation EC₅₀ (µM)** | **Cmpd** | **T Cell Activation EC₅₀ (µM)** |
|---|---|---|---|---|---|---|---|
| **1** | 0.2999 | **202** | - | **393** | 0.41610 | **584** | 10.00000 |
| **2** | 0.0876 | **203** | - | **394** | 0.02225 | **585** | 10.00000 |
| **3** | 0.0429 | **204** | - | **395** | 0.08176 | **586** | 0.20495 |
| **4** | 0.0963 | **205** | - | **396** | 0.01531 | **587** | 10.00000 |
| **5** | 0.1716 | **206** | 0.18185 | **397** | 0.00571 | **588** | 10.00000 |
| **6** | 0.0223 | **207** | 0.11805 | **398** | 0.00854 | **589** | 10.00000 |
| **7** | 0.0535 | **208** | 0.08201 | **399** | 0.07774 | **590** | 1.19820 |
| **8** | 0.0150 | **209** | 10.00000 | **400** | 0.01644 | **591** | 0.46900 |
| **9** | 0.2116 | **210** | 0.02655 | **401** | 0.02061 | **592** | 0.08642 |
| **10** | 0.0209 | **211** | 0.05617 | **402** | 0.04460 | **593** | 0.10373 |
| **11** | 0.0840 | **212** | 0.02060 | **403** | 0.01888 | **594** | 0.03373 |
| **12** | 0.0671 | **213** | 0.02348 | **404** | 0.02386 | **595** | 0.21080 |
| **13** | 0.0777 | **214** | 0.02704 | **405** | 0.02401 | **596** | 0.34980 |
| **14** | 0.1647 | **215** | 0.02549 | **406** | 0.06271 | **597** | 0.01637 |
| **15** | 10.0000 | **216** | 0.27075 | **407** | 0.02002 | **598** | 0.03678 |
| **16** | 0.3551 | **217** | 0.03818 | **408** | 0.03944 | **599** | 10.00000 |
| **17** | 0.0292 | **218** | 0.60100 | **409** | 0.27380 | **600** | 0.0326 |
| **18** | 0.0756 | **219** | 0.02220 | **410** | 0.04626 | **601** | 0.10986 |
| **19** | 0.06138 | **220** | 0.07611 | **411** | 10.00000 | **602** | 10.00000 |
| **20** | 0.02228 | **221** | 0.06438 | **412** | 0.01565 | **603** | 0.04966 |
| **21** | 0.07579 | **222** | 0.19575 | **413** | 0.01613 | **604** | 10.00000 |
| **22** | 0.13471 | **223** | 0.01675 | **414** | 0.02940 | **605** | 0.63590 |
| **23** | 0.03632 | **224** | 10.00000 | **415** | 5.10135 | **606** | 0.23670 |
| **24** | 0.0872 | **225** | 10.00000 | **416** | 10.00000 | **607** | 0.01834 |
| **25** | 0.3343 | **226** | 0.06268 | **417** | 0.05833 | **608** | 0.0224 |
| **26** | 0.07726 | **227** | 0.03128 | **418** | 0.01180 | **609** | 10.000 |
| **27** | 0.02512 | **228** | 0.07553 | **419** | 0.01949 | **610** | 2.10800 |
| **28** | 0.04836 | **229** | 0.24435 | **420** | 0.04259 | **611** | 10.00000 |
| **29** | 0.09401 | **230** | 0.39215 | **421** | 0.04770 | **612** | 10.00000 |
| **30** | 0.10033 | **231** | 0.10499 | **422** | 0.07553 | **613** | 10.00000 |
| **31** | 0.13427 | **232** | 0.10178 | **423** | 0.35355 | **614** | 10.00000 |
| **32** | 0.26053 | **233** | 10.00000 | **424** | 0.08659 | **615** | 0.27175 |
| **33** | 0.22420 | **234** | 0.14275 | **425** | 0.84110 | **616** | 0.03604 |
| **34** | 0.12246 | **235** | 0.06508 | **426** | 0.09875 | **617** | 0.03889 |
| **35** | 0.8552 | **236** | 0.05395 | **427** | 0.02499 | **618** | 0.05768 |
| **36** | 0.08209 | **237** | 0.02211 | **428** | 0.05123 | **619** | 0.06693 |
| **37** | 0.02565 | **238** | 0.12777 | **429** | 0.07140 | **620** | 0.04491 |
| **38** | 0.36885 | **239** | 0.16065 | **430** | 0.02326 | **621** | 0.30335 |
| **39** | 0.06178 | **240** | 0.02907 | **431** | 0.10645 | **622** | 0.21640 |
| **40** | 4.14620 | **241** | 10.00000 | **432** | 0.05206 | **623** | 0.22155 |
| **41** | 0.16380 | **242** | 0.43710 | **433** | 0.03995 | **624** | 0.01671 |
| **42** | 0.08856 | **243** | 0.10357 | **434** | 0.03784 | **625** | 0.02083 |
| **43** | 0.06252 | **244** | 0.02525 | **435** | 0.00995 | **626** | 0.07896 |
| **44** | 0.05411 | **245** | 0.18695 | **436** | 0.13425 | **627** | 0.04338 |
| **45** | 0.89247 | **246** | 0.10079 | **437** | 0.15715 | **628** | 0.02714 |
| **46** | 0.17063 | **247** | 0.05240 | **438** | 0.05868 | **629** | 0.11415 |
| **47** | 10.000 | **248** | 0.59645 | **439** | 0.12055 | **630** | 0.04960 |
| **48** | 0.01866 | **249** | 0.08169 | **440** | 0.21385 | **631** | 0.03768 |
| **49** | 0.03699 | **250** | 0.03099 | **441** | 0.14920 | **632** | 0.04117 |
| **50** | 0.02908 | **251** | 0.11750 | **442** | 0.04329 | **633** | 0.12108 |
| **51** | 0.07448 | **252** | 0.05405 | **443** | 0.01048 | **634** | 0.11029 |
| **52** | 0.12720 | **253** | 0.03916 | **444** | 10.00000 | **635** | 0.23660 |
| **53** | 0.3993 | **254** | 0.14770 | **445** | 10.00000 | **636** | 1.26890 |
| **54** | 0.02153 | **255** | 0.30543 | **446** | 0.03101 | **637** | 0.11620 |
| **55** | 0.12700 | **256** | 0.16768 | **447** | 0.06131 | **638** | 0.09490 |
| **56** | 0.03681 | **257** | 10.00000 | **448** | 0.07632 | **639** | 0.02304 |
| **57** | 0.01981 | **258** | 0.07544 | **449** | 10.00000 | **640** | 0.02624 |
| **58** | 0.03734 | **259** | 0.05858 | **450** | 0.05474 | **641** | 0.10727 |
| **59** | 0.05077 | **260** | 0.02676 | **451** | 1.9685 | **642** | 0.18140 |
| **60** | 0.04460 | **261** | 0.12598 | **452** | 10.00000 | **643** | 1.52550 |
| **61** | 0.07547 | **262** | 0.08280 | **453** | 0.26965 | **644** | 0.08483 |
| **62** | 0.02697 | **263** | 10.00000 | **454** | 0.20295 | **645** | 1.41500 |
| **63** | 0.04076 | **264** | 0.04773 | **455** | 0.17430 | **646** | 2.7095 |
| **64** | 0.07188 | **265** | 0.36760 | **456** | 0.29125 | **647** | 0.06535 |
| **65** | 0.19407 | **266** | 0.43938 | **457** | 10.00000 | **648** | 0.14250 |
| **66** | 0.15757 | **267** | 0.63930 | **458** | 0.08256 | **649** | 10.00000 |
| **67** | 0.10134 | **268** | 0.66895 | **459** | 0.03300 | **650** | 0.14405 |
| **68** | 0.41263 | **269** | 0.00554 | **460** | 0.09654 | **651** | 10.00000 |
| **69** | 0.05664 | **270** | 0.08548 | **461** | 10.00000 | **652** | 0.22670 |
| **70** | 0.04939 | **271** | 0.18295 | **462** | 0.17800 | **653** | 0.20850 |
| **71** | 0.03059 | **272** | 0.08821 | **463** | 0.28835 | **654** | 0.08281 |
| **72** | 0.39213 | **273** | 0.11600 | **464** | 0.22550 | **655** | 10.00000 |
| **73** | 0.18393 | **274** | 0.11395 | **465** | 0.04829 | **656** | 0.58930 |
| **74** | 0.06999 | **275** | 10.00000 | **466** | 10.00000 | **657** | 0.03327 |
| **75** | 0.01601 | **276** | 10.00000 | **467** | 10.00000 | **658** | 0.49185 |
| **76** | 0.08972 | **277** | 0.17005 | **468** | 0.09710 | **659** | 0.08215 |
| **77** | 0.10459 | **278** | 0.25760 | **469** | 0.07926 | **660** | 0.02204 |
| **78** | 0.20473 | **279** | 0.22395 | **470** | 0.19010 | **661** | 0.82020 |
| **79** | 0.05057 | **280** | 0.11115 | **471** | 0.11094 | **662** | 0.01846 |
| **80** | 0.04623 | **281** | 0.15315 | **472** | 0.03166 | **663** | 0.03993 |
| **81** | 0.03142 | **282** | 0.08533 | **473** | 10.00000 | **664** | 0.03238 |
| **82** | 0.06045 | **283** | 0.03666 | **474** | 0.11520 | **665** | 0.04630 |
| **83** | 0.06620 | **284** | 0.06241 | **475** | 3.77100 | **666** | 0.11355 |
| **84** | 0.07104 | **285** | 0.01424 | **476** | 10.00000 | **667** | 0.06615 |
| **85** | 0.14177 | **286** | 0.01539 | **477** | 7.47800 | **668** | 0.24450 |
| **86** | 0.02751 | **287** | 0.01645 | **478** | 10.00000 | **669** | 0.29635 |
| **87** | 0.31237 | **288** | 0.05274 | **479** | 0.05933 | **670** | 0.06223 |
| **88** | 0.02489 | **289** | 0.08758 | **480** | 1.49250 | **671** | 0.02959 |
| **89** | 0.01647 | **290** | 0.55075 | **481** | 0.01593 | **672** | 0.21675 |
| **90** | 0.05828 | **291** | 0.37575 | **482** | 0.02028 | **673** | 0.23360 |
| **91** | 0.22140 | **292** | 0.38820 | **483** | 0.03968 | **674** | 0.33655 |
| **92** | 0.01011 | **293** | 0.06051 | **484** | 0.19535 | **675** | 0.02686 |
| **93** | 0.04419 | **294** | 0.07392 | **485** | 0.21155 | **676** | 0.03728 |
| **94** | 0.04747 | **295** | 0.23345 | **486** | 0.11414 | **677** | 1.21200 |
| **95** | 0.10377 | **296** | 0.04980 | **487** | 0.03995 | **678** | 0.03696 |
| **96** | 0.23013 | **297** | 0.42210 | **488** | 0.03400 | **679** | 0.03046 |
| **97** | 0.02832 | **298** | 0.01462 | **489** | 0.02853 | **680** | 0.02625 |
| **98** | 0.03050 | **299** | 0.02265 | **490** | 0.01790 | **681** | 0.02338 |
| **99** | 0.04780 | **300** | 0.28230 | **491** | 0.03177 | **682** | 0.02460 |
| **100** | 0.05899 | **301** | 0.01966 | **492** | 0.0185 | **683** | 0.02589 |
| **101** | 0.03097 | **302** | 0.02354 | **493** | 0.01920 | **684** | 0.03489 |
| **102** | 0.34233 | **303** | 0.03082 | **494** | 0.06173 | **685** | 0.05168 |
| **103** | 0.30413 | **304** | 0.19655 | **495** | 0.02281 | **686** | 0.01105 |
| **104** | 0.03066 | **305** | 10.00000 | **496** | 0.02915 | **687** | 0.02632 |
| **105** | 0.19143 | **306** | 0.36115 | **497** | 0.03019 | **688** | 0.01703 |
| **106** | 0.12300 | **307** | 0.18645 | **498** | 0.02647 | **689** | 0.03741 |
| **107** | 0.25307 | **308** | 0.02409 | **499** | 0.08849 | **690** | 0.04218 |
| **108** | 0.14012 | **309** | 0.07503 | **500** | 0.11990 | **691** | 0.06764 |
| **109** | 0.02791 | **310** | 0.09048 | **501** | 0.09409 | **692** | 0.04350 |
| **110** | 0.06052 | **311** | 0.15162 | **502** | 0.02890 | **693** | 0.02612 |
| **111** | 0.26307 | **312** | 0.17780 | **503** | 0.03623 | **694** | 0.06973 |
| **112** | 0.05048 | **313** | 0.09731 | **504** | 0.03643 | **695** | 0.16880 |
| **113** | 0.18530 | **314** | 0.06918 | **505** | 10.00000 | **696** | 0.24368 |
| **114** | 0.08119 | **315** | 0.02888 | **506** | 0.02496 | **697** | 0.11970 |
| **115** | 0.03627 | **316** | 0.04568 | **507** | 0.04520 | **698** | 0.26065 |
| **116** | 0.06488 | **317** | 0.06261 | **508** | 0.03510 | **699** | 0.05147 |
| **117** | 0.03073 | **318** | 0.03155 | **509** | 0.10031 | **700** | 0.12420 |
| **118** | 0.04406 | **319** | 0.03826 | **510** | 0.02705 | **701** | 0.41035 |
| **119** | 0.37033 | **320** | 0.05118 | **511** | 0.02073 | **702** | 0.26600 |
| **120** | 0.97218 | **321** | 0.06474 | **512** | 0.03282 | **703** | 0.14630 |
| **121** | 0.13094 | **322** | 0.07291 | **513** | 0.68520 | **704** | 10.00000 |
| **122** | 0.05567 | **323** | 0.16370 | **514** | 0.09449 | **705** | 10.00000 |
| **123** | 0.12923 | **324** | 0.03137 | **515** | 0.01785 | **706** | 0.07263 |
| **124** | 0.03626 | **325** | 0.03949 | **516** | 0.05042 | **707** | 10.00000 |
| **125** | 0.36110 | **326** | 0.04304 | **517** | 0.01700 | **708** | 1.32425 |
| **126** | 0.03680 | **327** | 0.17840 | **518** | 0.00977 | **709** | 10.00000 |
| **127** | 0.03350 | **328** | 0.02815 | **519** | 0.01277 | **710** | 10.00000 |
| **128** | 0.00911 | **329** | 0.07020 | **520** | 0.06659 | **711** | 0.19500 |
| **129** | 0.01645 | **330** | 0.07376 | **521** | 0.00863 | **712** | 0.08912 |
| **130** | 0.15840 | **331** | 0.01700 | **522** | 10.00000 | **713** | 0.13038 |
| **131** | 0.03535 | **332** | 0.01238 | **523** | 0.09241 | **714** | 0.24885 |
| **132** | 0.05121 | **333** | 0.04906 | **524** | 0.34670 | **715** | 0.58090 |
| **133** | 0.02138 | **334** | 0.03851 | **525** | 0.05671 | **716** | 0.93950 |
| **134** | 0.02303 | **335** | 0.04979 | **526** | 0.03032 | **717** | 10.00000 |
| **135** | 10.00000 | **336** | 0.09564 | **527** | 0.01442 | **718** | 0.08154 |
| **136** | 0.01631 | **337** | 0.06196 | **528** | 10.00000 | **719** | 0.41970 |
| **137** | 0.08427 | **338** | 0.09632 | **529** | 10.00000 | **720** | 10.00000 |
| **138** | 5.16038 | **339** | 0.09759 | **530** | 0.03945 | **721** | 1.32275 |
| **139** | 0.01289 | **340** | 0.52260 | **531** | 0.04127 | **722** | 0.81770 |
| **140** | 10.00000 | **341** | 0.02265 | **532** | 0.14345 | **723** | 1.84950 |
| **141** | 10.00000 | **342** | 0.33910 | **533** | 0.01347 | **724** | 10.00000 |
| **142** | 3.3805 | **343** | 0.24775 | **534** | 0.05674 | **725** | 0.12047 |
| **143** | 5.43990 | **344** | 0.01491 | **535** | 1.06220 | **726** | 0.04870 |
| **144** | 10.00000 | **345** | 0.03230 | **536** | 10.00000 | **727** | 10.00000 |
| **145** | 0.01768 | **346** | 0.07048 | **537** | 0.04157 | **728** | 10.00000 |
| **146** | 0.06115 | **347** | 0.06961 | **538** | 10.00000 | **729** | 0.65155 |
| **147** | 0.04273 | **348** | 0.02895 | **539** | 0.03177 | **730** | 1.10450 |
| **148** | 0.01954 | **349** | 0.03833 | **540** | 0.08661 | **731** | 1.16550 |
| **149** | 10.00000 | **350** | 0.08918 | **541** | 0.09336 | **732** | 10.00000 |
| **150** | 0.12656 | **351** | 0.14581 | **542** | 0.07919 | **733** | 0.02857 |
| **151** | 0.07734 | **352** | 0.13383 | **543** | 0.01750 | **734** | 10.00000 |
| **152** | 10.00000 | **353** | 10.00000 | **544** | 0.02427 | **735** | 0.01561 |
| **153** | 0.78233 | **354** | 0.02632 | **545** | 0.02129 | **736** | 10.00000 |
| **154** | 10.00000 | **355** | 0.02867 | **546** | 0.01951 | **737** | 0.10852 |
| **155** | 10.00000 | **356** | 0.02554 | **547** | 0.06920 | **738** | 0.27173 |
| **156** | 10.00000 | **357** | 0.00910 | **548** | 0.07591 | **739** | 1.85150 |
| **157** | 10.00000 | **358** | 0.06444 | **549** | 0.10849 | **740** | 0.08038 |
| **158** | 10.00000 | **359** | 0.04037 | **550** | 0.02499 | **741** | 10.00000 |
| **159** | 10.00000 | **360** | 0.03101 | **551** | 0.18250 | **742** | 0.02861 |
| **160** | 0.14960 | **361** | 0.04054 | **552** | 0.14450 | **743** | 10.00000 |
| **161** | 0.38708 | **362** | 0.26070 | **553** | 0.05303 | **744** | 0.15393 |
| **162** | 5.15835 | **363** | 0.39970 | **554** | 0.10348 | **745** | 0.37155 |
| **163** | 10.00000 | **364** | 0.02849 | **555** | 0.05695 | **746** | 2.08150 |
| **164** | 0.06663 | **365** | 0.01986 | **556** | 0.22215 | **747** | 0.82545 |
| **165** | 0.00415 | **366** | 0.15925 | **557** | 0.37815 | **748** | 1.00075 |
| **166** | 0.00198 | **367** | 0.41195 | **558** | 10.00000 | **749** | 0.46120 |
| **167** | 0.01838 | **368** | 0.03740 | **559** | 0.07323 | **750** | 0.70260 |
| **168** | 0.01247 | **369** | 0.36865 | **560** | 0.05732 | **751** | 0.00568 |
| **169** | 0.63230 | **370** | 0.04123 | **561** | 10.00000 | **752** | 1.03000 |
| **170** | 0.41485 | **371** | 0.04873 | **562** | 10.00000 | **753** | 0.08705 |
| **171** | 0.67045 | **372** | 0.02351 | **563** | 0.95570 | **754** | 0.0091 |
| **172** | 10.00000 | **373** | 0.15230 | **564** | 0.25950 | **755** | 0.00448 |
| **173** | 10.00000 | **374** | 0.04464 | **565** | 1.03910 | **756** | 0.06378 |
| **174** | 10.00000 | **375** | 0.04636 | **566** | 0.34095 | **757** | 0.08649 |
| **175** | 1.16500 | **376** | 0.03842 | **567** | 10.00000 | **758** | 0.35410 |
| **176** | 0.00999 | **377** | 0.40470 | **568** | 0.18735 | **759** | 0.01690 |
| **177** | 0.17565 | **378** | 0.07384 | **569** | 0.57990 | **760** | 0.01921 |
| **178** | 0.14365 | **379** | 0.04528 | **570** | 10.00000 | **761** | 0.01692 |
| **179** | 0.03081 | **380** | 0.02051 | **571** | 0.08750 | **762** | 0.04988 |
| **180** | 0.03594 | **381** | 0.05327 | **572** | 0.01735 | **763** | 0.64960 |
| **181** | 0.03054 | **382** | 0.01528 | **573** | 0.01437 | **764** | 0.86515 |
| **182** | 0.03871 | **383** | 0.01441 | **574** | 0.01397 | **765** | 1.2760 |
| **183** | 0.04491 | **384** | 0.06998 | **575** | 0.01407 | **766** | 0.15179 |
| **184** | 0.08041 | **385** | 0.01776 | **576** | 10.00000 | **767** | 0.09214 |
| **185** | 0.01930 | **386** | 0.01163 | **577** | 10.00000 | **768** | 0.01666 |
| **196** | - | **387** | 0.00822 | **578** | 10.00000 | **769** | 0.06057 |
| **197** | - | **388** | 0.03850 | **579** | 0.10124 | **770** | 0.02431 |
| **198** | - | **389** | 0.03580 | **580** | 10.00000 | **771** | 0.02867 |
| **199** | - | **390** | 0.05659 | **581** | 0.04310 | **772** | 0.01156 |
| **200** | - | **391** | 0.01796 | **582** | 1.03615 | **773** | 0.01055 |
| **201** | - | **392** | 0.01762 | **583** | 10.00000 | **774** | 0.01296 |

### Example 757: HBV-specific T cell reinvigoration in vitro assay using peripheral blood mononuclear cells (PBMCs) from chronic hepatitis B patients

In one aspect, this assay measures the ability of a PD-L1 inhibitor to increase HBV specific T cell activity in an infected subject, as measured by the increase in interferon-gamma expression by those T cells. In certain embodiments, the test compound's ability to increase interferon-gamma expression in HBV-specific T cells indicates that the compound is useful in treating, ameliorating, and/or preventing HBV infection in the subject.

PD-L1 checkpoint inhibition has been shown to reinvigorate and increase HBV-specific T cell activity when peripheral blood mononuclear cells (PBMCs) isolated from chronic hepatitis B patients are treated with monoclonal antibodies targeting PD-L1 *ex vivo* (Fisicaro et al., Gastroenterology, 2010; 138:682-693). The ability of PD-L1 inhibitor compounds to reinvigorate HBV-specific T cell activity was assessed as described. PBMCs from nine chronic hepatitis B patients were obtained by Ficoll separation of fresh whole blood and frozen in liquid nitrogen as described previously (Park et al., Nature Communications, 2021; 12:1222). Frozen PBMCs were thawed and plated into 12-well plates at 2x10⁶ cells/ml. PBMCs were rested overnight at 37°C before they were stimulated with HBV-specific peptide pools and allowed to proliferate for 5 days. Expanded PBMCs were then restimulated with HBV-specific peptide pools alone or stimulated with HBV-specific peptide pools and incubated with compounds **89, 90,** and **139,** a negative control, or anti-PD-L1 antibody as reference. Supernatant was obtained after 24 hours and assayed for IFN-γ using the Luminex platform. Fold change increase in IFN-γ expression mediated by compound treatment relative to HBV-specific peptide treatment alone was determined (Table 3).

**Table 3. Fold Change in IFN-γ Expression of HBV-specific T Cells Treated with Compounds**

| | **Chronic Hepatitis B Donor** | | | | | | | | | **Mean** ± **SD** |
|---|---|---|---|---|---|---|---|---|---|---|
| **Compound** | **1** | **2** | **3** | **4** | **5** | **6** | **7** | **8** | **9** | |
| | **Fold Change** | | | | | | | | | |
| **89** | 2.0 | 2.0 | 1.5 | 3.7 | 1.5 | 2.7 | 1.3 | 2.2 | 1.5 | 2.0 ± 0.8 |
| **139** | 1.1 | 2.2 | 2.4 | 5.5 | 2.2 | 1.2 | 1.5 | 3.4 | 1.8 | 2.4 ± 1.4 |
| **90** | 1.7 | 2.2 | 1.9 | 3.0 | 1.2 | 2.5 | 1.5 | 2.1 | 1.2 | 1.9 ± 0.6 |
| Anti-PD-L1 antibody | 1.7 | 3.0 | 1.6 | 2.1 | 1.8 | 2.9 | 1.5 | 2.5 | 1.3 | 2.0 ± 0.6 |
| Negative control | 1.4 | 1.3 | 0.4 | 1.8 | 1.1 | 0.9 | 1.0 | 1.2 | 2.1 | 1.2 ± 0.5 |

### Example 758: In vivo anti-tumor efficacy in humanized PD-L1 and PD-1 expressing MC38 tumor mouse model

In one aspect, this assay measures the ability of a PD-L1 inhibitor to reduce the size of MC38 (a murine colon adenocarcinoma cell line) tumors in mice, as an indication of the PD-L1 inhibitor's ability to help treat, ameliorate, and/or prevent cancer in the subject.

*In vivo* anti-tumor efficacy of compounds was evaluated in mice expressing humanized PD-1 and PD-L1, in which the extracellular domains of murine PD-1 and PD-L1 were genetically replaced with the human extracellular domain counterparts (Huang et al., Scientific Reports, 2017;7:42687). Humanized PD-1/PD-L1 (hPD-1/hPD-L1) mice were subcutaneously implanted with the colorectal cancer cell line MC38 expressing human PD-L1 and compound treatment initiated when tumor volumes reached approximately 100 mm³. Compounds were administered *via* oral gavage once daily at: 30 mg/kg between study days 1 and 7; or 10 mg/kg for 28 days. The anti-PD-L1 monoclonal antibody atezolizumab was included as a positive reference control and administered *via* intraperitoneal injection at 0.3 mg/kg thrice weekly between study days 1 and 15. Tumor lengths and widths were measured by digital caliper and tumor volumes were calculated by the formula: Volume = ½ (Length × Width)².

**Table 4. Tumor volumes (group mean, mm³) of MC38-tumor-bearing hPD-1/hPD-L1 mice treated with compounds of the present disclosure**

| | **Study Days** | | | | | | | | | |
|---|---|---|---|---|---|---|---|---|---|---|
| **Treatment** | **-1** | **2** | **5** | **9** | **12** | **16** | **19** | **23** | **26** | **28** |

| | **Tumor Volume (mm³)** | | | | | | | | | |
|---|---|---|---|---|---|---|---|---|---|---|
| Vehicle | 106 | 125 | 184 | 324 | 420 | 579 | 724 | 1055 | 1394 | 1600 |

| | **Study Days** | | | | | | | | | |
|---|---|---|---|---|---|---|---|---|---|---|
| **Treatment** | **-1** | **2** | **5** | **9** | **12** | **16** | **19** | **23** | **26** | **28** |

| | **Tumor Volume (mm³)** | | | | | | | | | |
|---|---|---|---|---|---|---|---|---|---|---|
| **89** | 105 | 116 | 164 | 176 | 212 | 243 | 310 | 418 | 564 | 607 |
| 30 mg/kg x 7 days | | | | | | | | | | |
| **89** | 105 | 122 | 170 | 227 | 223 | 222 | 252 | 279 | 328 | 346 |
| 10 mg/kg x 28 days | | | | | | | | | | |
| **139** | 105 | 122 | 168 | 179 | 179 | 143 | 190 | 237 | 281 | 356 |
| 30 mg/kg x 7 days | | | | | | | | | | |
| **139** | 104 | 126 | 190 | 220 | 277 | 303 | 357 | 488 | 554 | 591 |
| 10 mg/kg x 28 days | | | | | | | | | | |
| **572** | 108 | 114 | 125 | 277 | 276 | 332 | 425 | 552 | 628 | 797 |
| 10 mg/kg x 28 days | | | | | | | | | | |
| **573** | 108 | 114 | 121 | 210 | 235 | 293 | 374 | 431 | 372 | 553 |
| 10 mg/kg x 28 days | | | | | | | | | | |
| Atezolizumab | 105 | 122 | 162 | 156 | 192 | 242 | 243 | 341 | 429 | 472 |

### Enumerated Embodiments

The following enumerated embodiments are provided, the numbering of which is not to be construed as designating levels of importance.

Embodiment 1 provides a compound of formula (I), or a salt, solvate, geometric isomer, stereoisomer, or tautomer thereof: 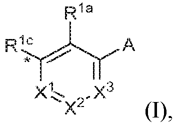 wherein:
A is 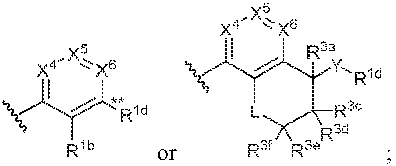
Y is NR³⁶ or O;
L is selected from the group consisting of -C(R^{3g})(R^{3h})- -(C(R^{3g})(R^{3h})(C(R³ⁱ)(R^{3j})-, and a bond;
X¹ is selected from the group consisting of CR^{'1} and N;
X² is selected from the group consisting of CR^{'2} and N;
X³ is selected from the group consisting of CR^{'3} and N;
X⁴ is selected from the group consisting of CR^{"1} and N;
X⁵ is selected from the group consisting of CR^{"2} and N;
X⁶ is selected from the group consisting of CR^{"3} and N;
   wherein one to four of X¹, X², X³, X⁴, X⁵, and X⁶ is N;
R^{'1}, R^{'2}, R^{'3}, R^{"1}, R^{"2}, and R^{"3} are each independently selected from the group consisting of H, halogen, C₁-C₃ alkyl, C₃-C₈ cycloalkyl, C₁-C₃ alkoxy, cyano, C₁-C₃ haloalkyl, and C₁-C₃ haloalkoxy;
R^{1a} and R^{1b} are each independently selected from the group consisting of H, halogen, C₁-C₃ alkyl, C₃-C₈ cycloalkyl, C₁-C₃ alkoxy, cyano, C₁-C₃ haloalkyl, and C₁-C₃ haloalkoxy;
   R^{1c} and R^{1d} are each independently selected from the group consisting of: 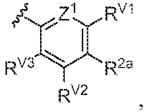 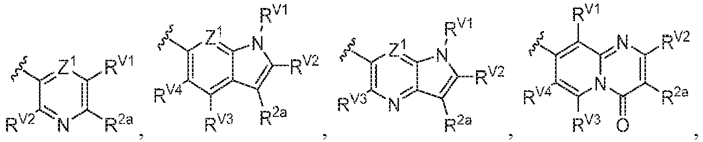 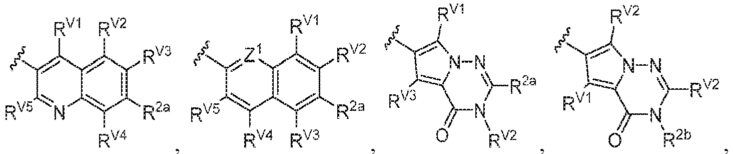 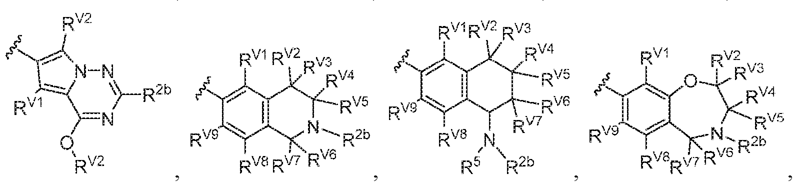 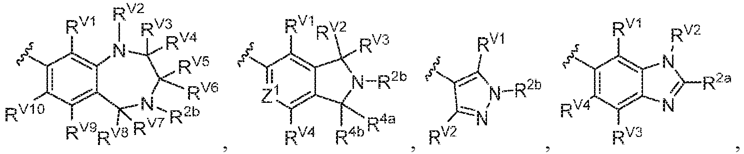 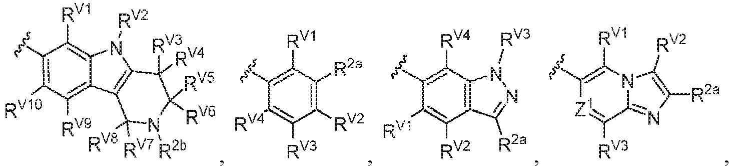 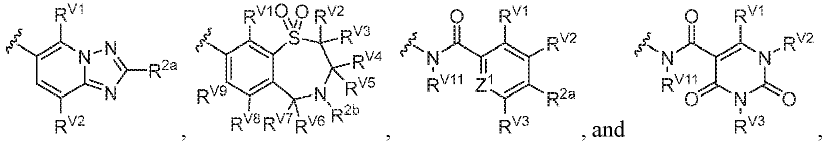 wherein each occurrence of Z¹ is CR^{V10} or N;
R^{2a} is selected from the group consisting of -C(=O)NR⁶R⁶, C(=O)OR^{I}, optionally substituted heterocyclyl, -(CH₂)₁₋₃(optionally substituted heterocyclyl), optionally substituted C₁-C₆ alkoxy, optionally substituted C₁-C₆ aminoalkyl, and optionally substituted C₁-C₆ hydroxyalkyl,
   wherein each optional substituent in R^{2a} is independently selected from the group consisting of optionally substituted heterocyclyl, optionally substituted C₁-C₆ alkyl, optionally substituted C₁-C₆ hydroxyalkyl, optionally substituted cycloalkyl, C(=O)(optionally substituted C₁-C₆ alkyl), and optionally substituted -(CH₂)₁₋₂(heterocyclyl),
   wherein two optional substituents in R^{2a} may combine with the atoms to which they are bound to form an optionally substituted C₂-C₈ heterocyclyl or optionally substituted C₃-C₈ cycloalkyl,
   wherein each occurrence of R^{I} is independently H, C₁-C₆ alkyl, or C₃-Cs cycloalkyl, wherein the alkyl or cycloalkyl is independently optionally substituted with at least one of halogen, -OH, C₁-C₆ alkoxy, -NH₂, -NH(C₁-C₆ alkyl), and -N(C₁-C₆ alkyl)(C₁-C₆ alkyl),
   wherein each occurrence of R⁶ is independently H, C₁-C₆ alkyl, or C₃-Cs cycloalkyl, wherein the alkyl or cycloalkyl is independently optionally substituted with at least one of halogen, -OH, C₁-C₆ alkoxy, -NH₂, -NH(C₁-C₆ alkyl), and -N(C₁-C₆ alkyl)(C₁-C₆ alkyl),
R^{2b} is selected from the group consisting of H, -(CH₂)₁₋₃C(=O)OR^{I}, -(CH₂)₁-₃C(=O)NR^{I}R^{I}, optionally substituted heterocyclyl, -(CH₂)₁₋₂(optionally substituted heterocyclyl), optionally substituted C₁-C₆ alkyl, optionally substituted C₁-C₆ haloalkyl, optionally substituted C₁-C₆ alkoxy, optionally substituted C₁-C₆ aminoalkyl, and optionally substituted C₁-C₆ hydroxyalkyl,
   wherein each optional substituent in R^{2b} is independently selected from the group consisting of optionally substituted C₁-C₆ alkyl, optionally substituted heterocyclyl, optionally substituted cycloalkyl, and optionally substituted -(CH₂)₁₋₃(heterocyclyl);
R^{3a}, R^{3b}, R^{3c}, R^{3d}, R^{3e}, R^{3f}, R^{3g}, R^{3h}, R³ⁱ, and R^{3j} are each independently selected from the group consisting of H, halogen, C₁-C₃ alkyl, C₃-C₈ cycloalkyl, C₁-C₃ alkoxy, C₁-C₃ haloalkyl, and C₁-C₃ haloalkoxy;
R⁵ is selected from the group consisting of H, C₁-C₃ alkyl, C₃-C₈ cycloalkyl, C₁-C₃ alkoxy, C₁-C₃ haloalkyl, and C₁-C₃ haloalkoxy,
   wherein R⁵ and R^{2b} may combine with the nitrogen atom to which they are bound to form a C₃-C₁₂ heterocyclyl;
each occurrence of R^{4a} and R^{4b} is independently selected from the group consisting of H, C₁-C₃ alkyl, C₃-C₈ cycloalkyl, C₁-C₃ alkoxy, C₁-C₃ haloalkyl, and C₁-C₃ haloalkoxy,
   wherein R^{4a} and R^{4b} may combine with the carbon atom to which they are bound to form a carbonyl (C=O);
each occurrence of R^{V1}, R^{V2}, R^{V3}, R^{V4}, R^{V5}, R^{V6}, R^{V7}, R^{V8}, R^{V9}, and R^{V10} is independently selected from the group consisting of H, C₁-C₃ alkyl, C₃-C₈ cycloalkyl, C₁-C₃ alkoxy, cyano, halogen, C₁-C₃ haloalkyl, C₁-C₃ haloalkoxy, and C(=O)OR^{I};
R^{V11} is selected from the group consisting of H, C₁-C₃ alkyl, C₃-C₈ cycloalkyl, C₁-C₃ alkoxy, C₁-C₃ haloalkyl, and C₁-C₃ haloalkoxy;
wherein if R^{1c} and R^{1d} are identical, then at least one of the following applies:
   (a) if X¹ is N, then Z¹ in R^{1c}, if present, is CR^{V10}, and;
   (b) if X⁶ is N, then Z¹ in R^{1d}, if present, is CR^{V10}.

Embodiment 2 provides the compound of Embodiment 1, which is selected from the group consisting of:

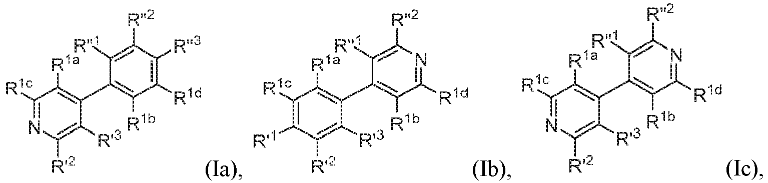

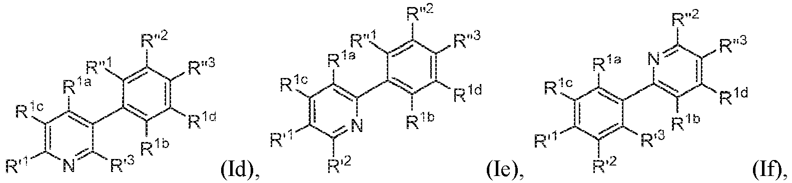

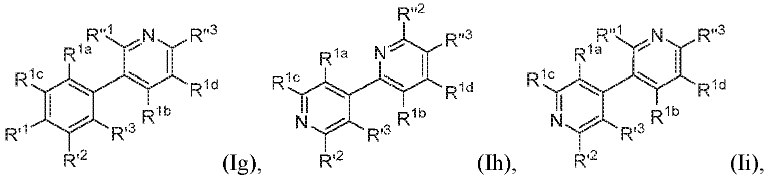

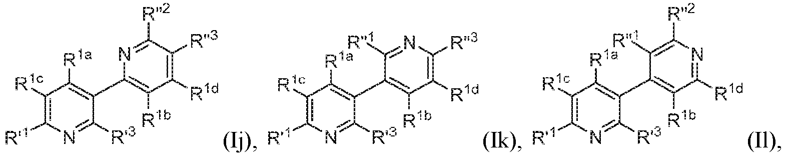

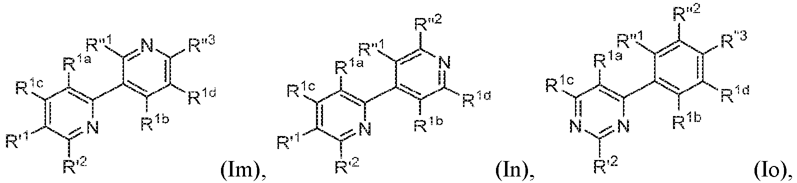

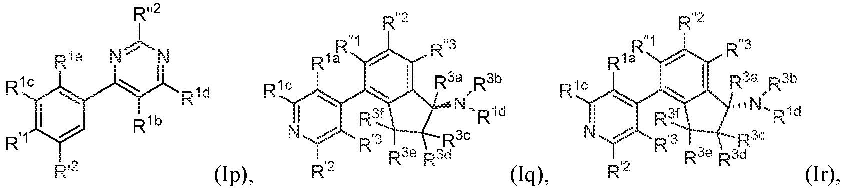

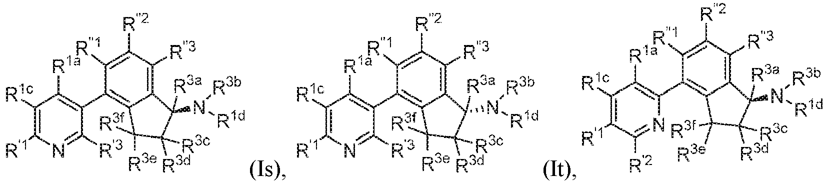

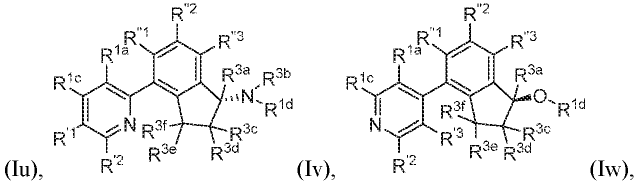

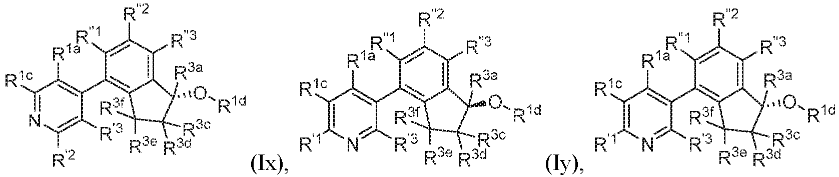

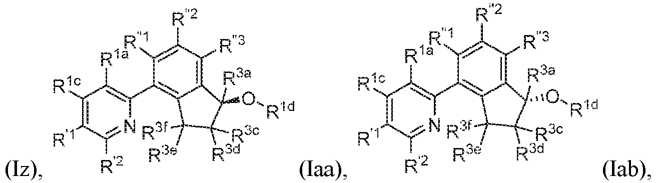

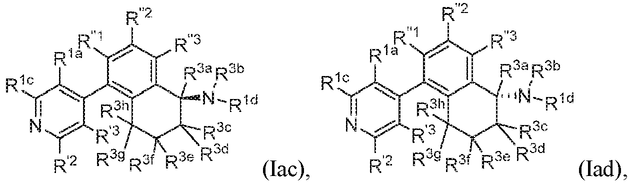

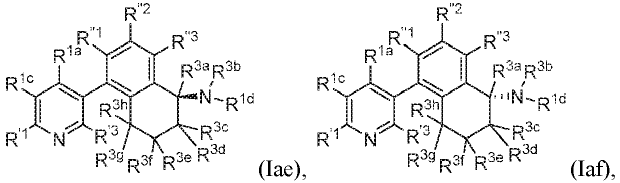

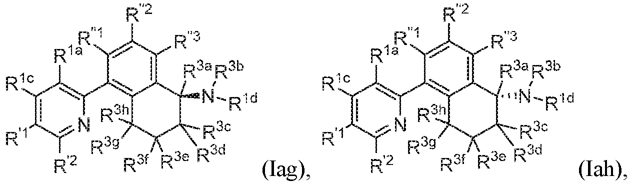

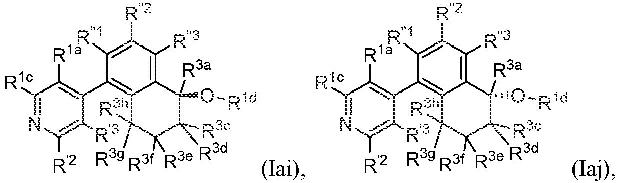

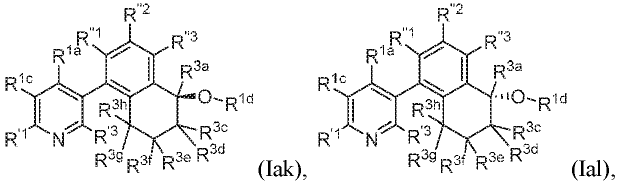

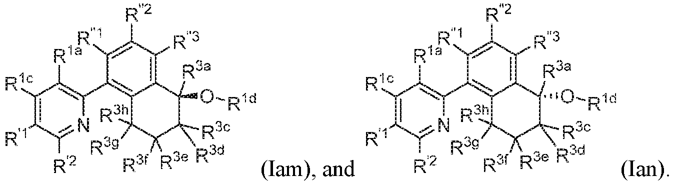

Embodiment 3 provides the compound of any one of Embodiments 1-2, wherein one of the following applies:
(a) each of R^{'2}, R^{'3}, R^{"1}, R^{"2}, and R^{"3} are H, and X¹ is N;
(b) each of R^{'1}, R^{'3}, R^{"1}, R^{"2}, and R^{"3} are H, and X² is N;
(c) each of R^{'1}, R^{'2}, R^{"1}, R^{"2}, and R^{"3} are H, and X³ is N;
(d) each of R^{'1}, R^{'2}, R^{'3}, R^{"2}, and R^{"3} are H, and X⁴ is N;
(e) each of R^{'1}, R^{'2}, R^{'3}, R^{"1}, and R^{"3} are H, and X⁵ is N;
(f) each of R^{'1}, R^{'2}, R^{'3}, R^{"1}, and R^{"2} are H, and X⁶ is N;
(g) each of R^{'2}, R^{'3}, R^{"2}, and R^{"3} are H, and X¹ and X⁴ are N;
(h) each of R^{'2}, R^{'3}, R^{"1}, and R^{"3} are H, and X¹ and X⁵ are N;
(i) each of R^{'2}, R^{'3}, R^{"1}, and R^{"2} are H, and X¹ and X⁶ are N;
(j) each of R^{'1}, R^{'3}, R^{"2}, and R^{"3} are H, and X² and X⁴ are N;
(k) each of R^{'1}, R^{'3}, R^{"1}, and R^{"3} are H, and X² and X⁵ are N;
(l) each of R^{'1}, R^{'3}, R^{"1}, and R^{"2} are H, and X² and X⁶ are N;
(m) each of R^{'1}, R^{'2}, R^{"1}, and R^{"3} are H, and X³ and X⁵ are N;
(n) each of R^{'1}, R^{'2}, R^{"1}, and R^{"2} are H, and X³ and X⁶ are N;
(o) each of R^{'2}, R^{"1}, R^{"2}, and R^{"3} are H, and X¹ and X³ are N; and
(p) each of R¹, R^{'2}, R^{'3}, and R^{"2} are H, and X⁴ and X⁶ are N.

Embodiment 4 provides the compound of any one of Embodiments 1-3, wherein one of the following applies:
(a) L is a bond and each of R^{3a}, R^{3c}, R^{3d}, R^{3e}, and R^{3f} is H;
(b) L is a bond, Y is NR^{3b}, and each of R^{3a}, R^{3b}, R^{3c}, R^{3d}, R^{3e}, and R^{3f} is H;
(c) L is -C(R^{3g})(R^{3h})- and each of R^{3a}, R^{3c}, R^{3d}, R^{3e}, R^{3f}, R^{3g}, and R^{3h} is H;
(d) L is -C(R^{3g})(R^{3h})-, Y is NR^{3b}, and each of R^{3a}, R^{3b}, R^{3c}, R^{3d}, R^{3e}, R^{3f}, R^{3g}, and R^{3h} is H;
(e) L is -(C(R^{3g})(R^{3h}))(C(R³ⁱ)(R^{3j}))- and each of R^{3a}, R^{3c}, R^{3d}, R^{3e}, R^{3f}, R^{3g}, R^{3h}, R³ⁱ, and R^{3j} is H; and
(f) L is -C(R^{3g})(R^{3h})-, Y is NR^{3b}, and each of R³³, R^{3b}, R^{3c}, R^{3d}, R^{3e}, R^{3f}, R^{3g}, R^{3h}, R³ⁱ, and R^{3j} are H.

Embodiment 5 provides the compound of any one of Embodiments 1-4, wherein each of of R^{1a} and R^{1b} is independently selected from the group consisting of H, F, Cl, Me, CF₃, and CN.

Embodiment 6 provides the compound of any one of Embodiments 1-5, wherein R^{1a} and R^{1b} are identical.

Embodiment 7 provides the compound of any one of Embodiments 1-6, wherein R^{2a} is selected from the group consisting of -(CH₂)₀₋₂NH(CH₂)₀₋₂(optionally substituted 5-oxopyrrolidin-2-yl), -(CH₂)₀₋₂N(C₁-C₆ alkyl)(CH₂)₀₋₂(optionally substituted 5-oxopyrrolidin-2-yl), -(CH₂)₀₋₂NH(CH₂)₀₋₂(C₁-C₆ alkyl), -(CH₂)₀₋₂N(C₁-C₆ alkyl)₂, -(CH₂)₀₋₂NH(CH₂)₀₋₂(C₁-C₆ haloalkyl), -(CH₂)₀₋₂N(C₁-C₆ alkyl)(C₁-C₆ haloalkyl), -(CH₂)₀₋₂NH(CH₂)₀₋₂(C₁-C₆ hydroxyalkyl), -(CH₂)₀₋₂N(C₁-C₆ alkyl)(CH₂)₀₋₂(C₁-C₆ hydroxyalkyl), -(CH₂)₀₋₂NH(CH₂)₀₋₂C(=O)O(C₁-C₆ alkyl), -(CH₂)₀₋₂N(C₁-C₆ alkyl)(CH₂)₀₋₂C(=O)O(C₁-C₆ alkyl), -(CH₂)₀₋₂NH(CH₂)₀₋₂N(C₁-C₆ alkyl)C(=O)(C₁-C₆ alkyl), -(CH₂)₀₋₂NH(CH₂)₀₋₂NHC(=O)(C₁-C₆ alkyl), -(CH₂)₀₋₂N(C₁-C₆ alkyl)(CH₂)₀₋₂N(C₁-C₆ alkyl)C(=O)(C₁-C₆ alkyl), -(CH₂)₀₋₂N(C₁-C₆ alkyl)(CH₂)₀₋₂NHC(=O)(C₁-C₆ alkyl), -(CH₂)₀₋₂NH(CH₂)₀₋₂(optionally substituted oxetanyl), - (CH₂)₀₋₂N(C₁-C₆ alkyl)(CH₂)₀₋₂(optionally substituted oxetanyl), -(CH₂)₀₋₂NH(CH₂)₀₋₂(optionally substituted oxolanyl), -(CH₂)₀₋₂N(C₁-C₆ alkyl)(CH₂)₀₋₂(optionally substituted oxolanyl), -(CH₂)₀₋₂NH(CH₂)₀₋₂(optionally substituted oxanyl), -(CH₂)₀₋₂N(C₁-C₆ alkyl)(CH₂)₀₋₂(optionally substituted oxanyl), -(CH₂)₀₋₂NH(CH₂)₀₋₂(optionally substituted cyclobutyl), -(CH₂)₀₋₂N(C₁-C₆ alkyl)(CH₂)₀₋₂(optionally substituted cyclobutyl), -(CH₂)₀₋₂NH(CH₂)₀₋₂(optionally substituted cyclohexyl), -(CH₂)₀₋₂N(C₁-C₆ alkyl)(CH₂)₀₋₂(optionally substituted cyclohexyl), -(CH₂)₀₋₂NH(CH₂)₀₋₂(optionally substituted C₅-C₆ cycloalkylsulfonyl), -(CH₂)₀₋₂N(C₁-C₆ alkyl)(CH₂)₀₋₂(optionally substituted C₅-C₆ cycloalkylsulfonyl), -(CH₂)₀₋₂NH(CH₂)₀₋₂(optionally substituted azetidinyl), -(CH₂)₀₋₂N(C₁-C₆ alkyl)(CH₂)₀₋₂(optionally substituted azetidinyl), -(CH₂)₀₋₂NH(CH₂)₀₋₂(optionally substituted pyrrolidinyl), -(CH₂)₀₋₂N(C₁-C₆ alkyl)(CH₂)₀₋₂(optionally substituted pyrrolidinyl), -(CH₂)₀₋₂NH(CH₂)₀₋₂(optionally substituted piperidinyl), -(CH₂)₀₋₂N(C₁-C₆ alkyl)(CH₂)₀₋₂(optionally substituted piperidinyl), -(CH₂)₀₋₂(optionally substituted guanidinyl), -(CH₂)₀₋₂NH(C(CH₂)₂-₅)C(=O)O(C₁-C₆ alkyl), -(CH₂)₀₋₂N(C₁-C₆ alkyl)(C(CH₂)₂₋₅)C(=O)O(C₁-C₆ alkyl), -(CH₂)₀₋₂NH(CH(C₁-C₃ alkyl))C(=O)O(C₁-C₆ alkyl), -(CH₂)₀₋₂N(C₁-C₆ alkyl)(CH(C₁-C₃ alkyl))C(=O)O(C₁-C₆ alkyl), -(CH₂)₀₋₂NH(CH₂)₀₋₁(C₅-C₉ bridged cycloalkyl)(CH₂)₀₋₁C(=O)O(C₁-C₆ alkyl), -(CH₂)₀₋₂N(C₁-C₆ alkyl)(CH₂)₀₋₁(C₅-C₉ bridged cycloalkyl)(CH₂)₀₋₁C(=O)O(C₁-C₆ alkyl), -(CH₂)₀₋₂NH(CH₂)₀₋₁(CHOH)(CH₂)₀₋₁C(=O)O(C₁-C₆ alkyl), -(CH₂)₀₋₂NH(CH₂)₀₋₁(CHO(C₁-C₆ alkyl))(CH₂)₀₋₁C(=O)O(C₁-C₆ alkyl), -(CH₂)₀₋₂N(C₁-C₆ alkyl)(CH₂)₀₋₁(CHOH)(CH₂)₀₋₁C(=O)O(C₁-C₆ alkyl), -(CH₂)₀₋₂N(C₁-C₆ alkyl)(CH₂)₀₋₁(CHO(C₁-C₆ alkyl))(CH₂)₀₋₁C(=O)O(C₁-C₆ alkyl), -(CH₂)₀₋₂(C₄-C₁₀ bridged heterocycloalkyl), -(CH₂)₀₋₂(optionally substituted azetidinyl), -(CH₂)₀₋₂(optionally substituted pyrrolidinyl), -(CH₂)₀₋₂(optionally substituted piperidinyl), -(CH₂)₀₋₂(optionally substituted morpholinyl), -(CH₂)₀₋₂(optionally substituted piperazinyl), -(CH₂)₀₋₂(optionally substituted piperazin-2-onyl), -C(CH₃)N(C₁-C₆ alkyl)₂, -(CH₂)₀₋₂N(C₁-C₆ alkyl)(CH₂)₀₋₂(CHF₂), -(CH₂)₀₋₂NH(CH₂)₀₋₂(CHF₂), -(CH₂)₀₋₂N(C₁-C₆ alkyl)(CH₂)₀₋₄(CH₂F), -(CH₂)₀₋₂NH(CH₂)₀₋₄(CH₂F), -(CH₂)₀₋₂NH(CH₂)₀₋₂N(C₁-C₆ alkyl)C(=O)CH₃, -(CH₂)₀₋₂NH(CH₂)₀₋₂NHC(=O)CH₃, -(CH₂)₀₋₂N(C₁-C₆ alkyl)(CH₂)₀₋₂N(C₁-C₆ alkyl)C(=O)CH₃, -(CH₂)₀₋₂N(C₁-C₆ alkyl)(CH₂)₀₋₂NHC(=O)CH₃, -(CH₂)₀₋₂N(C₁-C₆ alkyl)C((C(=O)OC₁-C₆ alkyl)((CH₂)₀₋₂O(C₁₋C₆ alkyl))), -(CH₂)₀₋₂NHC((C(=O)OC₁-C₆ alkyl)((CH₂)₀₋₂O(C₁-C₆ alkyl))), -(CH₂)₀₋₂N(C₁-C₆ alkyl)C((C(=O)OH((CH₂)₀₋₂O(C₁-C₆ alkyl))), -(CH₂)₀₋₂N(C₁-C₆ alkyl)C((C(=O)OC₁-C₆ alkyl)((CH₂)₀₋₂OH)), -(CH₂)₀₋₂NHC((C(=O)OH)((CH₂)₀₋₂0(C₁-C₆ alkyl))), -(CH₂)₀₋₂N(C₁-C₆ alkyl)C((C(=O)OH((CH₂)₀₋₂OH)), -(CH₂)₀₋₂NHC((C(=O)OC₁-C₆ alkyl)((CH₂)₀₋₂OH)), - (CH₂)₀₋₂NHC((C(=O)OH((CH₂)₀₋₂OH)), -(CH₂)₀₋₂N(C₁-C₆ alkyl)(CH₂)₁₋₄N(C₁-C₆ alkyl)C(=O)Me, -(CH₂)₀₋₂N(C₁-C₆ alkyl)(CH₂)₁₋₄NHC(=O)Me, -(CH₂)₀₋₂NH(CH₂)₁₋₄N(C₁-C₆ alkyl)C(=O)Me, -(CH₂)₀₋₂NH(CH₂)₁₋₄NHC(=O)Me, -(CH₂)₀₋₂N(C₁-C₆ alkyl)(CH₂)₁₋₅C(=O)N(C₁-C₆ alkyl)(C₁-C₆ alkyl), -(CH₂)₀₋₂NH(CH₂)₁₋₅C(=O)N(C₁-C₆ alkyl)(C₁-C₆ alkyl), -(CH₂)₀₋₂N(C₁-C₆ alkyl)(CH₂)₁₋₅C(=O)NH(C₁-C₆ alkyl), -(CH₂)₀₋₂N(C₁-C₆ alkyl)(CH₂)₁₋₅C(=O)NH₂, -(CH₂)₀₋₂NH(CH₂)₁₋₅C(=O)NH(C₁-C₆ alkyl), -(CH₂)₀₋₂NH(CH₂)₁₋₅C(=O)NH₂, - (CH₂)₀₋₂N(C₁-C₆ alkyl)(CH₂)₀₋₂(optionally substituted cyclopropyl), -(CH₂)₀₋₂NH(CH₂)₀-₂(optionally substituted cyclopropyl), -(CH₂)₀₋₂N(C₁-C₆ alkyl)(CH₂)₀₋₂(optionally substituted butanolid-2-yl), -(CH₂)₀₋₂NH(CH₂)₀₋₂(optionally substituted butanolide-2-yl), -(CH₂)₀₋₂N(C₁₋C₆ alkyl)(CH₂)₀₋₂(optionally substituted tetrahydro-4-thiopyranyl), -(CH₂)₀₋₂NH(CH₂)₀₋₂(optionally substituted oxazolyl), -(CH₂)₀₋₂NH(CH₂)₀₋₂(optionally substituted tetrahydro-4-thiopyranyl), -CH(CH₃)NH(CH₂)₀₋₂(optionally substituted piperidinyl), -CH(CH₃)N(C₁-C₆ alkyl)(CH₂)₀₋₂(optionally substituted piperidinyl), -(CH₂)₀₋₂N(C₁-C₆ alkyl)(CH₂)₀₋₂(optionally substituted piperazinyl), and -(CH₂)₀₋₂NH(CH₂)₀₋₂(optionally substituted piperazinyl).

Embodiment 8 provides the compound of any one of Embodiments 1-7, wherein R^{2b} is selected from the group consisting of -(CH₂)₀₋₂(optionally substituted 5-oxopyrrolidin-2-yl), -(CH₂)₀₋₂(optionally substituted piperidinyl), -(CH₂)₀₋₂(optionally substituted azetidinyl), - (CH₂)₀₋₂(C₁-C₆ alkyl), -(CH₂)₀₋₂(C₁-C₆ haloalkyl), -(CH₂)₀₋₂(C₁-C₆ hydroxyalkyl), -(CH₂)₀₋₂(optionally substituted cyclopropyl), -(CH₂)₀₋₂(optionally substituted cyclobutyl), -(CH₂)₀₋₂C(=O)OH, -(CH₂)₀₋₂N(C₁-C₆ alkyl)CH₃; -(CH₂)₀₋₂NHCH₃, -(CH₂)₀₋₂N(C₁-C₆ alkyl)(CH₂)₀₋₂C(=O)O(C₁-C₆ alkyl), -(CH₂)₀₋₂NH(CH₂)₀₋₂C(=O)O(C₁-C₆ alkyl), -(CH₂)₀₋₂N(C₁-C₆ alkyl)(CH₂)₀₋₂C(=O)OH, -(CH₂)₀₋₂NH(CH₂)₀₋₂C(=O)OH, -(CH₂)₀₋₂N(C₁-C₆ alkyl)(CH₂)₀₋₂(optionally substituted 5-oxopyrrolidin-2-yl), -(CH₂)₀₋₂NH(CH₂)₀₋₂(optionally substituted 5-oxopyrrolidin-2-yl), -(CH₂)₀₋₂C(=O)N(C₁-C₆ alkyl)(C₁-C₆ alky), -(CH₂)₀₋₂C(=O)NH(C₁-C₆ alkyl), -(CH₂)₀₋₂C(=O)NH₂, -(CH₂)₀₋₂CH(OC₁-C₆ alkyl)CH₂F, -(CH₂)₀₋₂CH(OH)CH₂F, and - (CH₂)₀₋₂C(=O)O(C₁-C₆ alkyl).

Embodiment 9 provides the compound of any one of Embodiments 1-8, wherein R^{2a} is selected from the group consisting of:
OR^{c}, 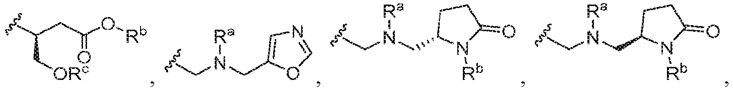 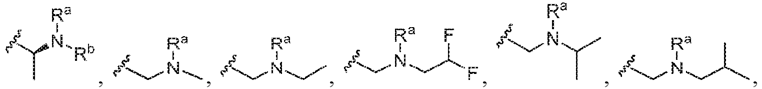 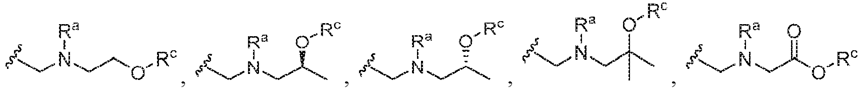 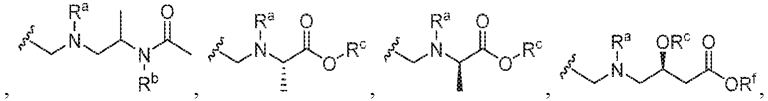 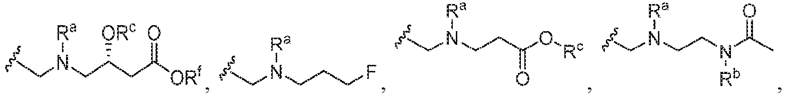 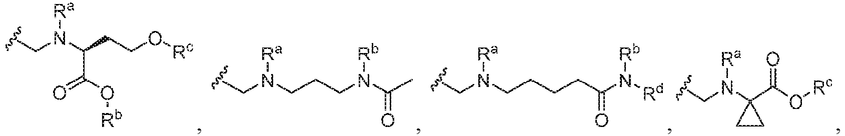 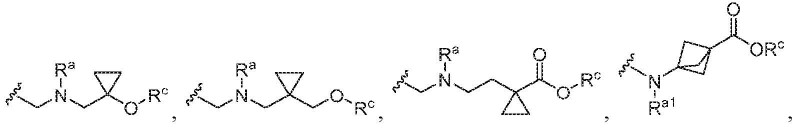 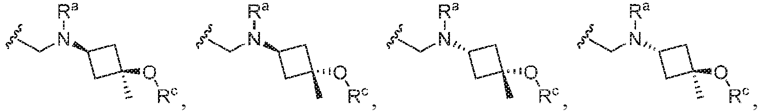 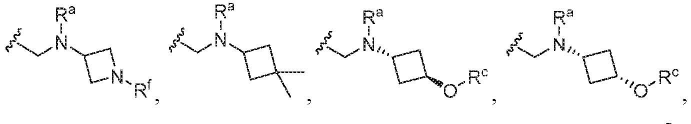 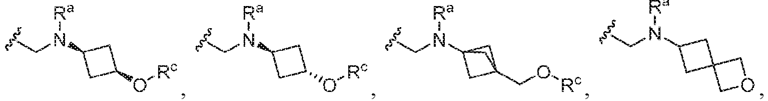 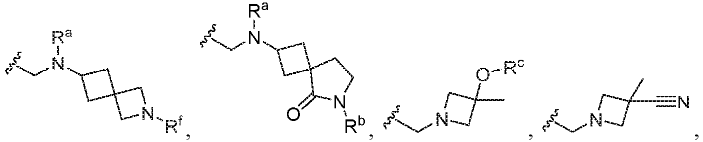 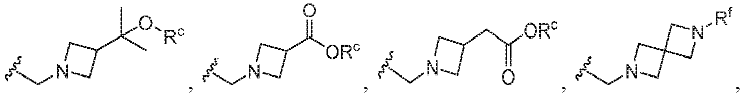 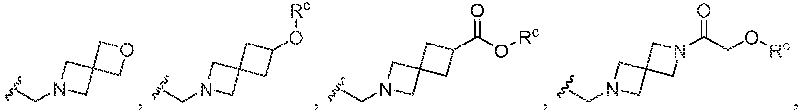 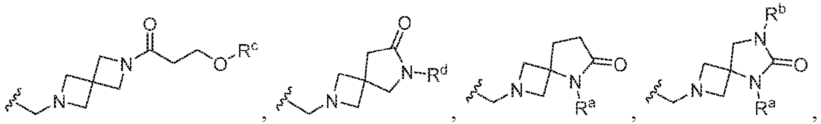 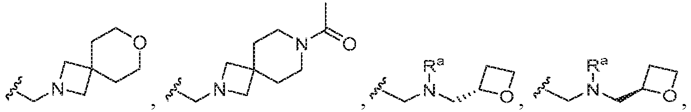 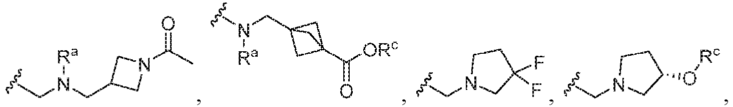 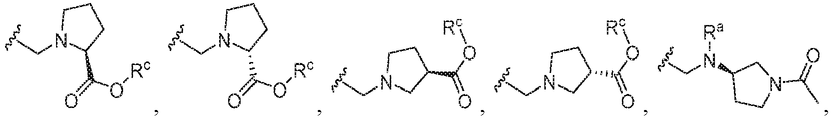 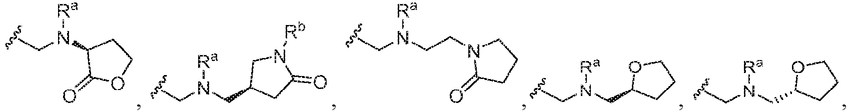 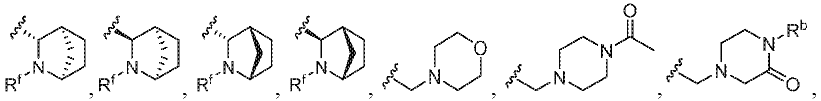 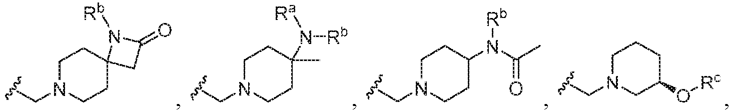 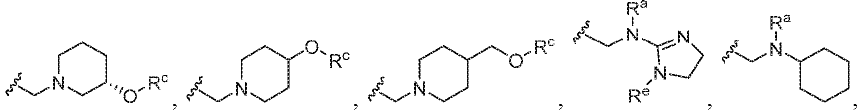 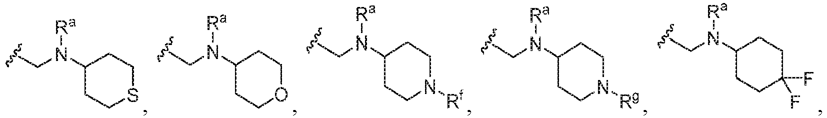 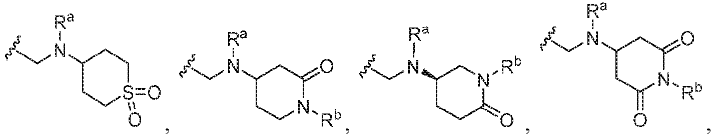 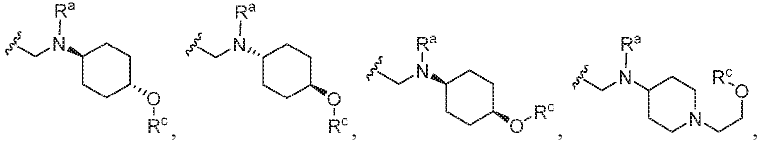 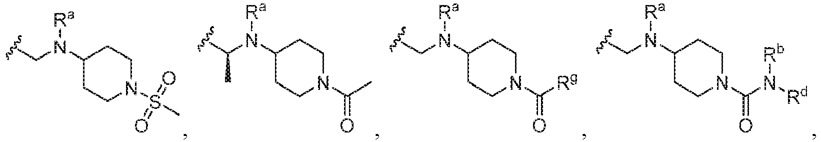 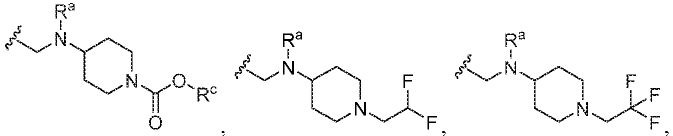 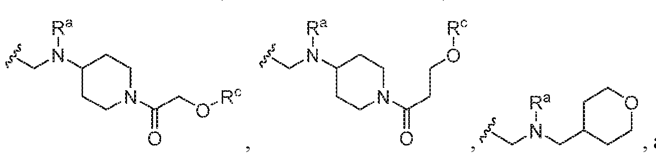 and 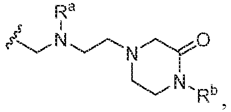
wherein each of R^{a}, R^{b}, R^{c}, R^{d}, R^{e}, and R^{f} is independently selected from the group consisting of H, C₁-C₃ alkyl, C₃-C₈ cycloalkyl, C₁-C₃ alkoxy, C₁-C₃ haloalkyl, C₁-C₃ haloalkoxy, and -C(=O)C₁-C₃ alkyl; and
R^{B} is selected from the group consisting of optionally substituted C₂-C₆ heteroaryl and optionally substituted phenyl.

Embodiment 10 provides the compound of any one of Embodiments 1-9, wherein at least one of R^{a}, R^{b}, R^{c}, R^{d}, R^{e}, and R^{f} is selected from the group consisting of methyl, isopropyl, and -C(=O)CH₃.

Embodiment 11 provides the compound of any one of Embodiments 1-10, wherein at least one of R^{a}, R^{b}, R^{c}, R^{d}, R^{e}, and R^{f}, if present, is H.

Embodiment 12 provides the compound of any one of Embodiments 1-11, wherein R^{2b} is selected from the group consisting of: 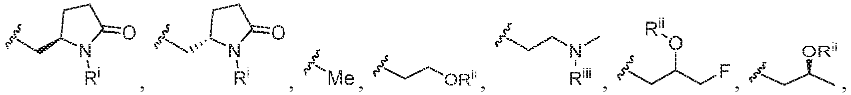 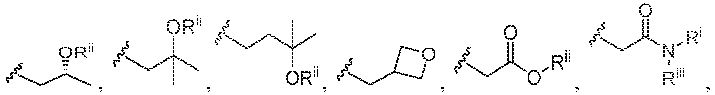 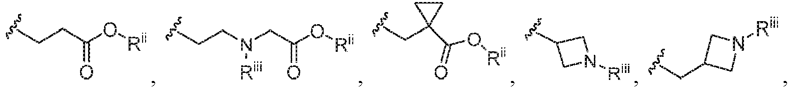 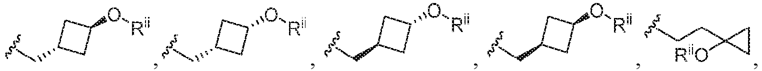 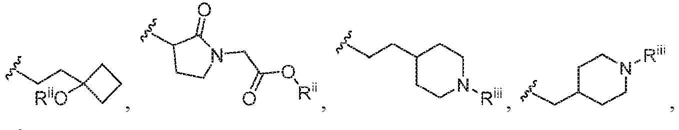 and 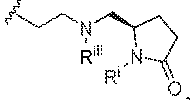 wherein each of Rⁱ, Rⁱⁱ, and Rⁱⁱⁱ is independently selected from the group consisting of H, C₁-C₃ alkyl, C₃-C₈ cycloalkyl, C₁-C₃ alkoxy, C₁-C₃ haloalkyl, C₁-C₃ haloalkoxy, and - C(=O)C₁-C₃ alkyl.

Embodiment 13 provides the compound of any one of Embodiments 1-12, wherein R^{1c} and R^{1d} are independently selected from the group consisting of: 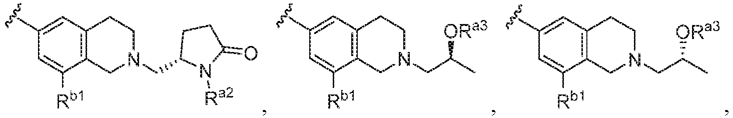 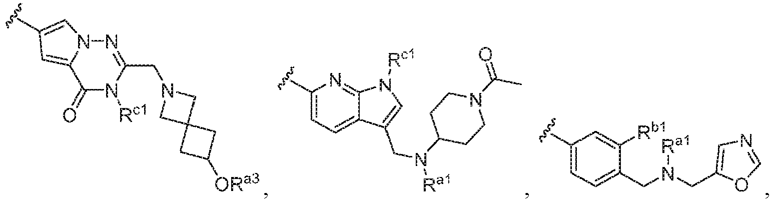 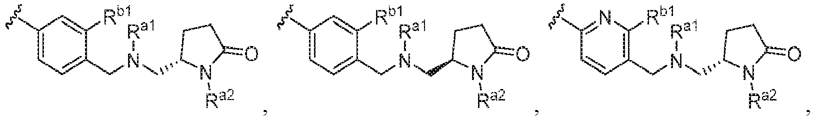 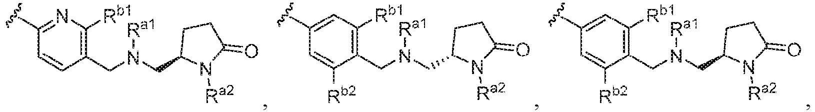 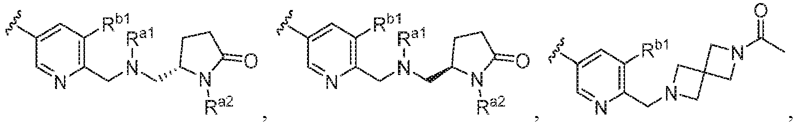 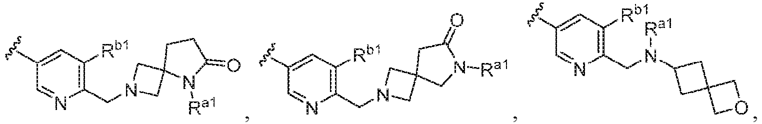 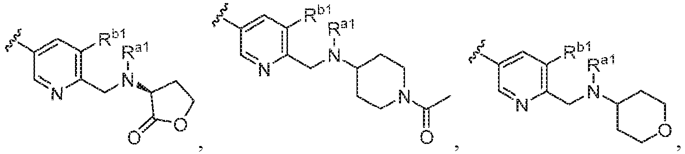 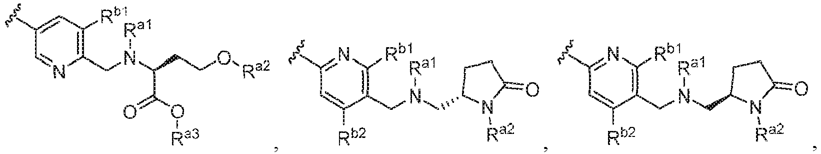 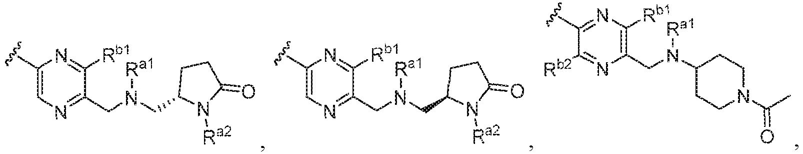 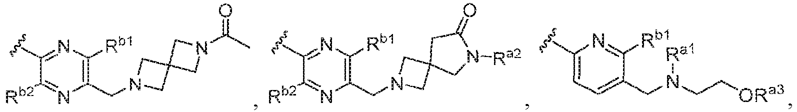 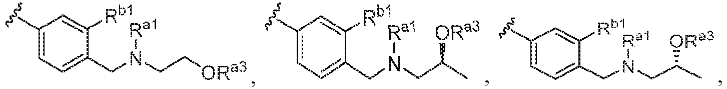 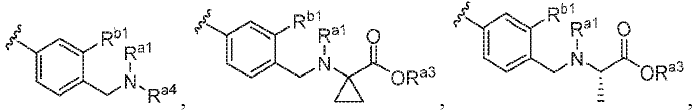 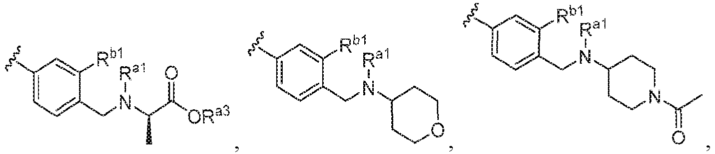 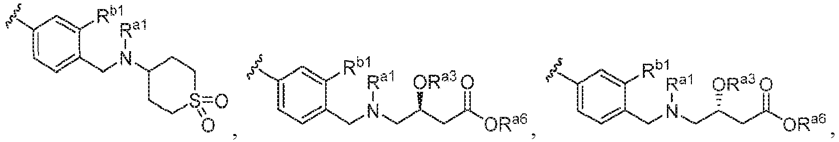 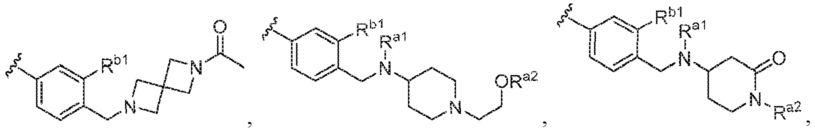 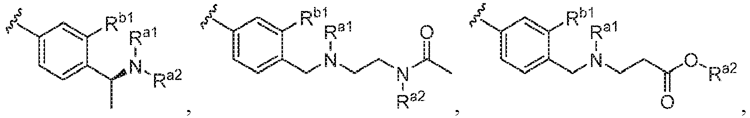 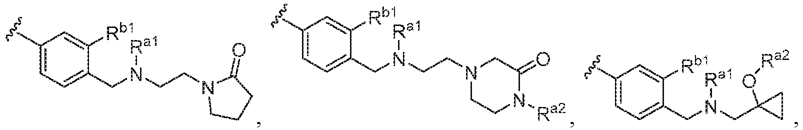 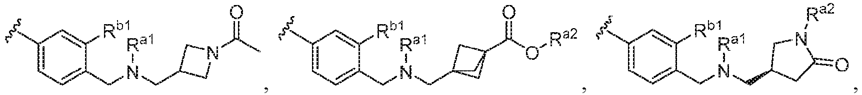 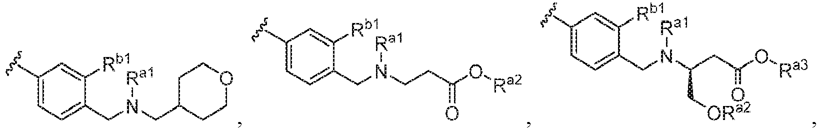 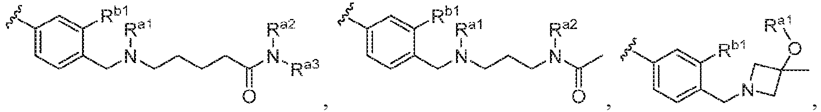 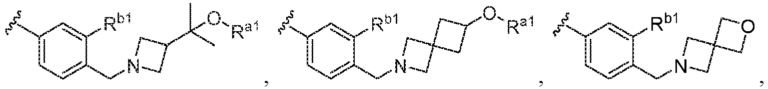 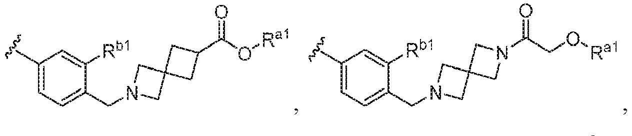 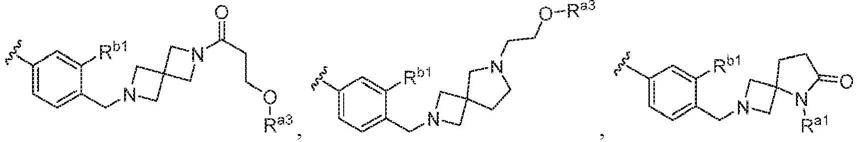 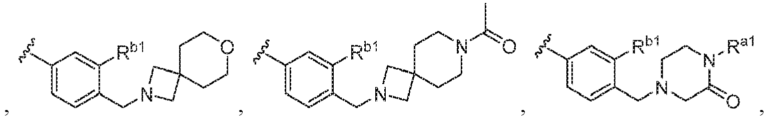 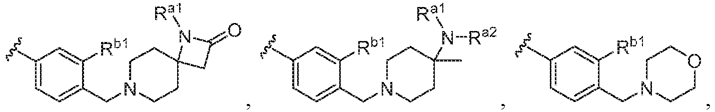 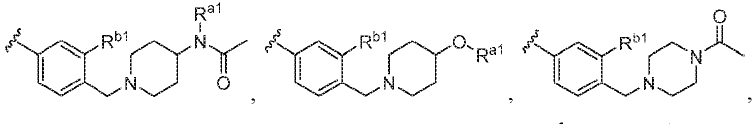 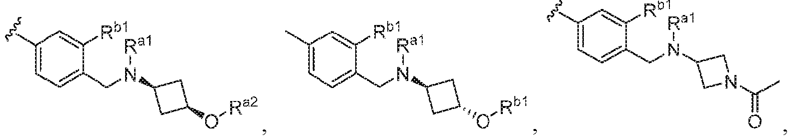 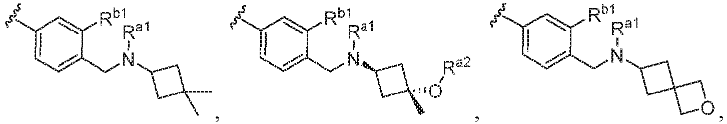 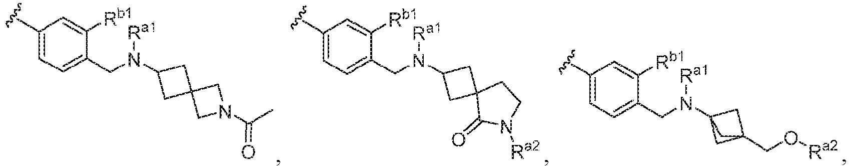 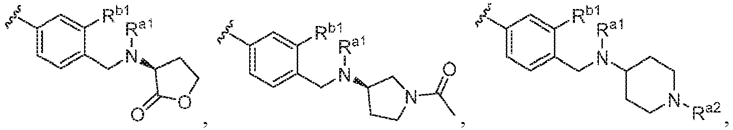 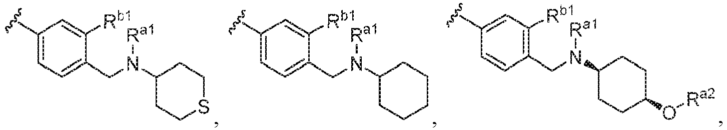 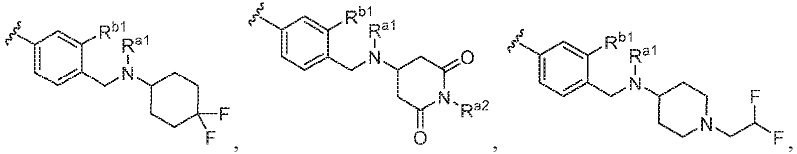 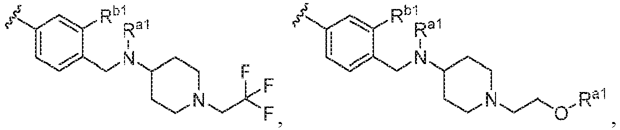 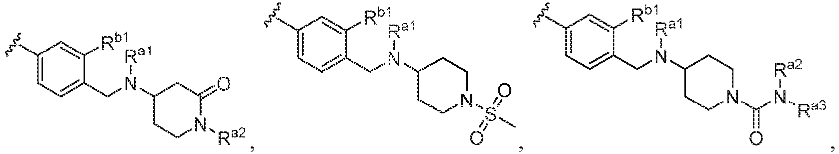 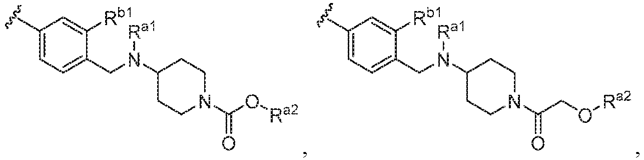 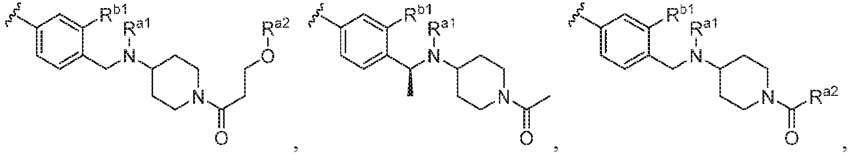 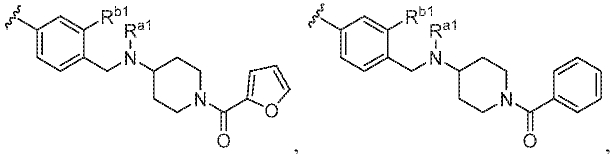 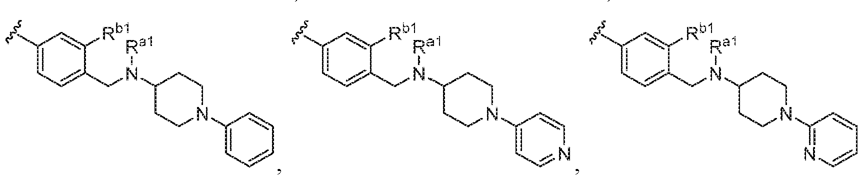 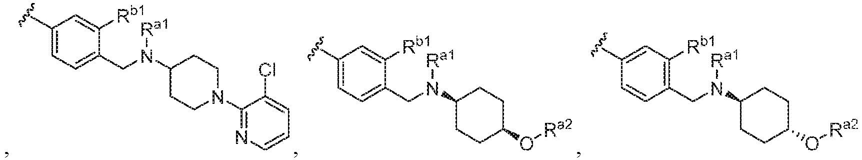 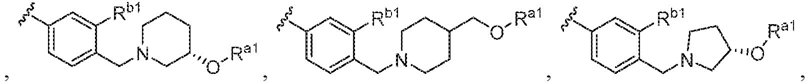 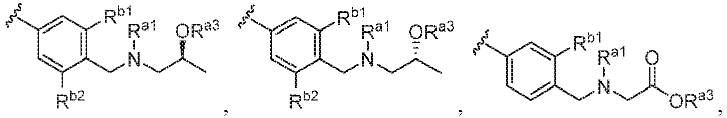 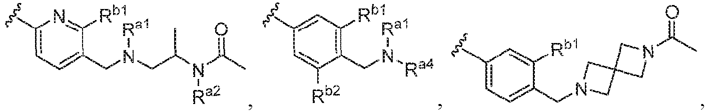 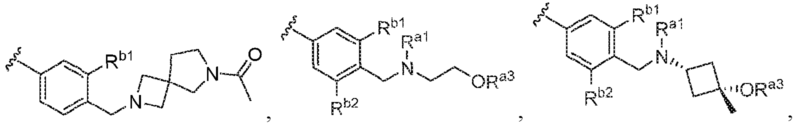 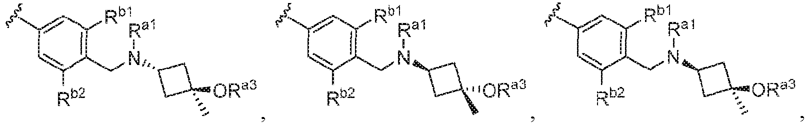 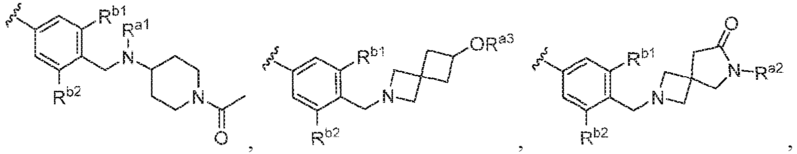 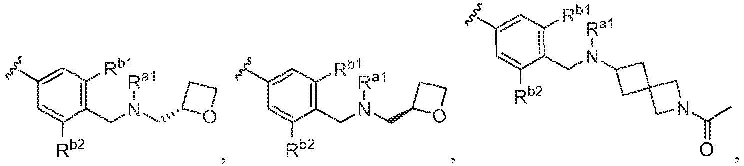 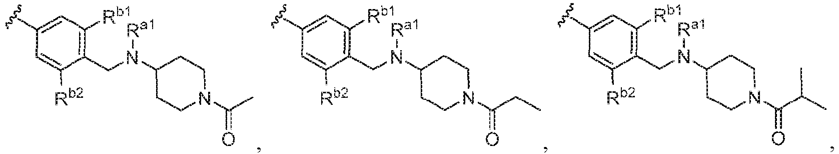 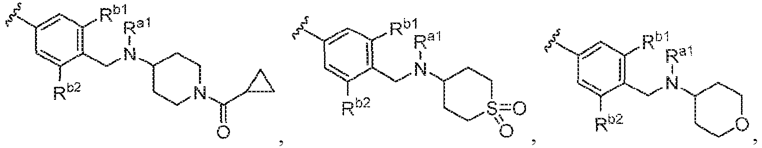 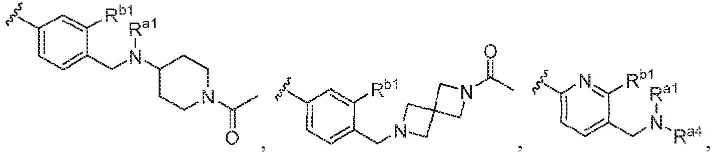 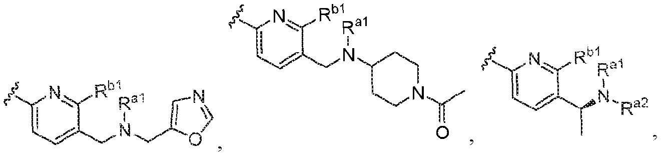 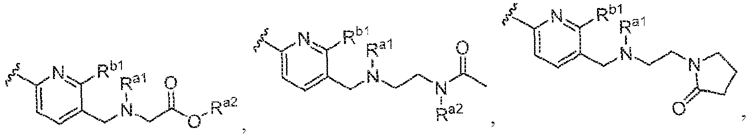 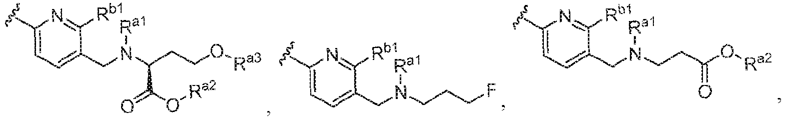 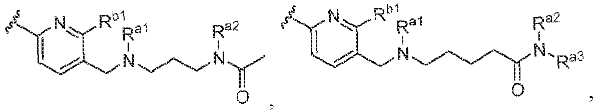 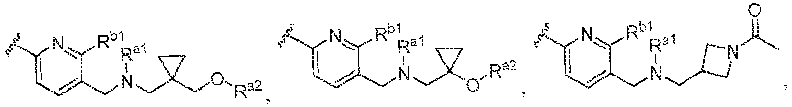 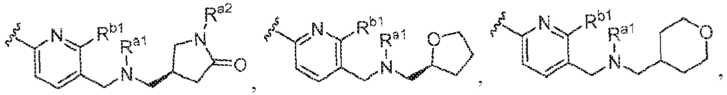 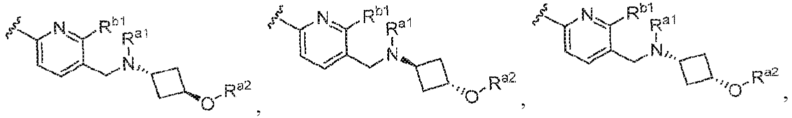 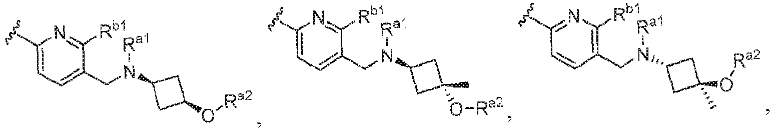 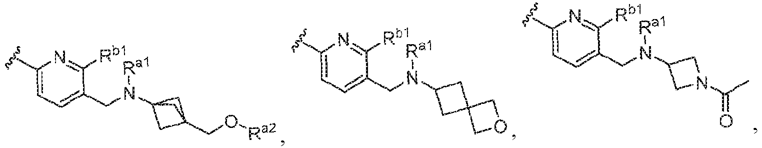 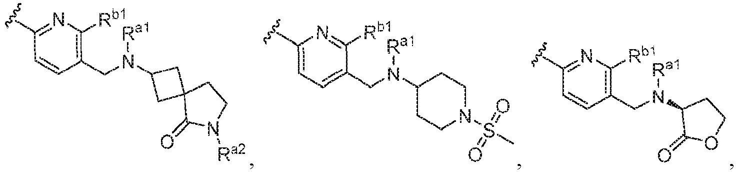 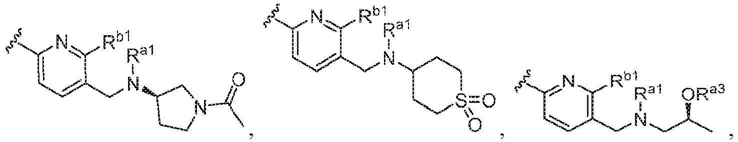 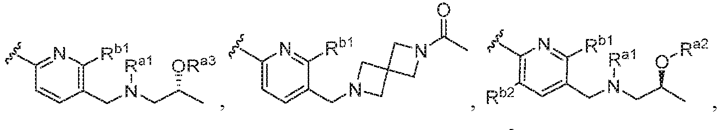 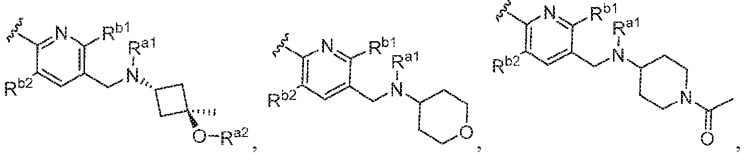 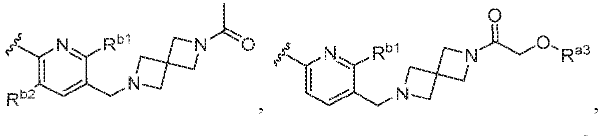 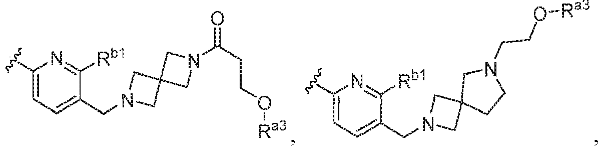 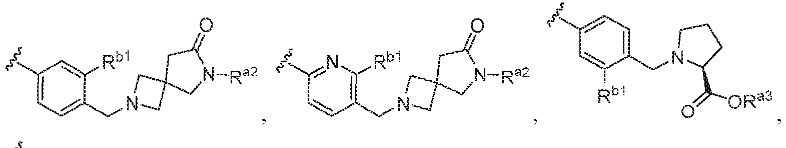 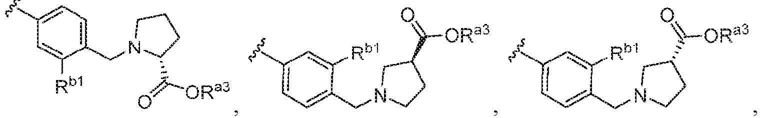 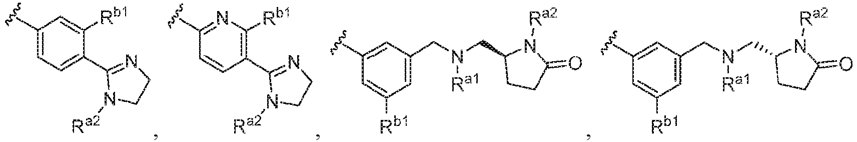 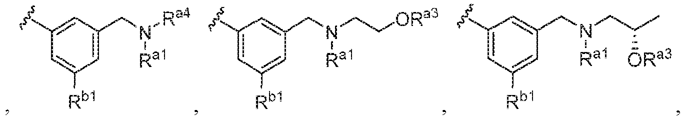 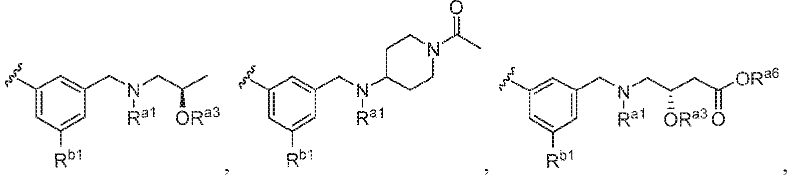 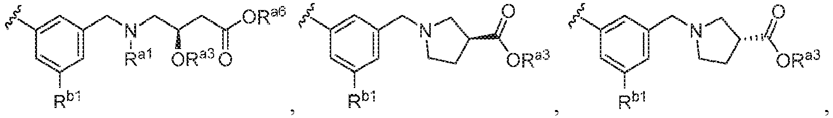 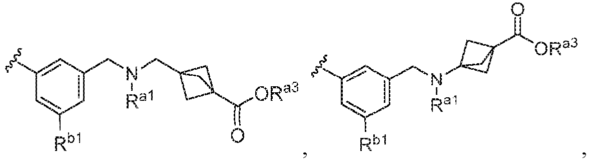 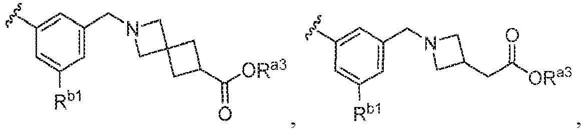 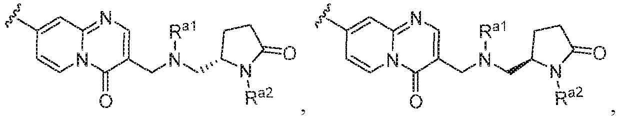 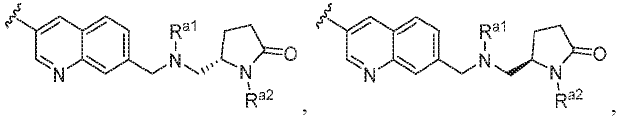 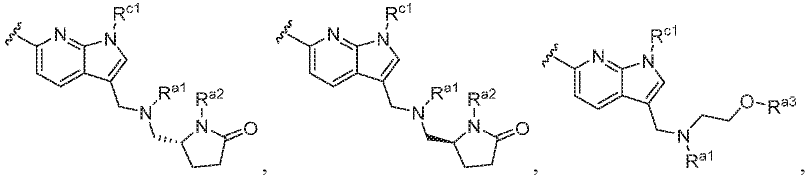 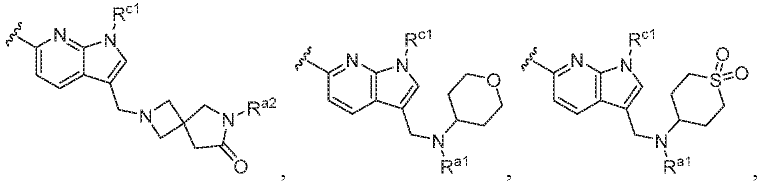 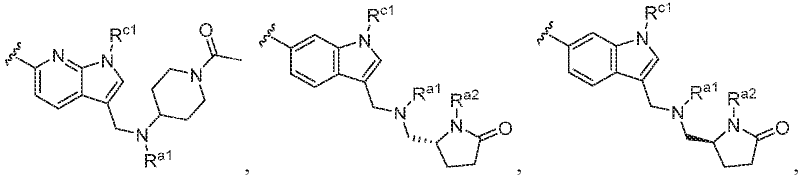 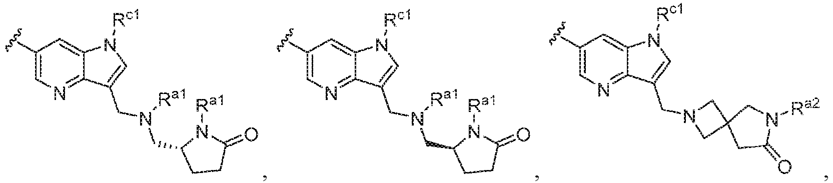 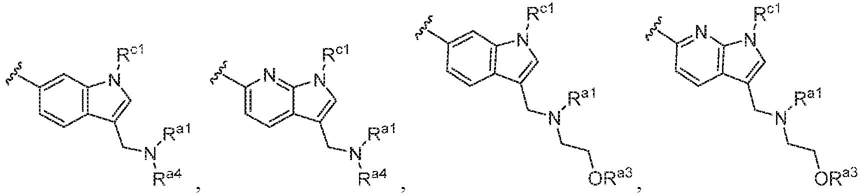 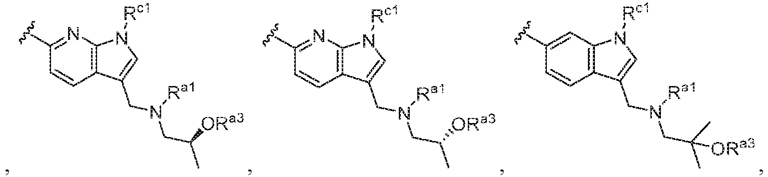 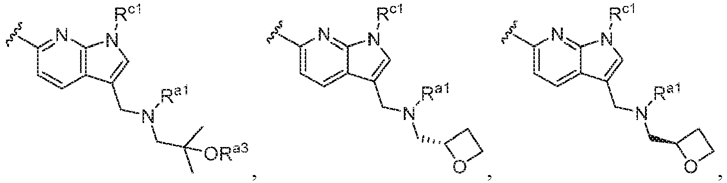 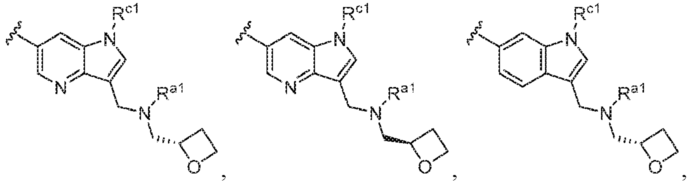 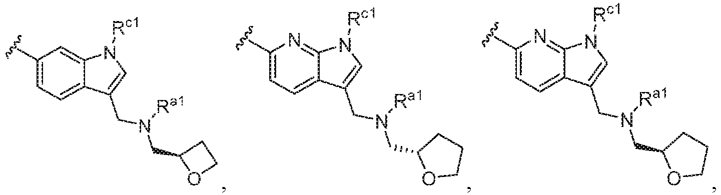 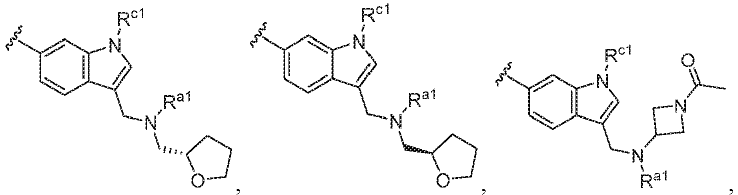 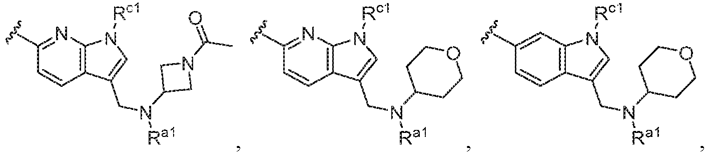 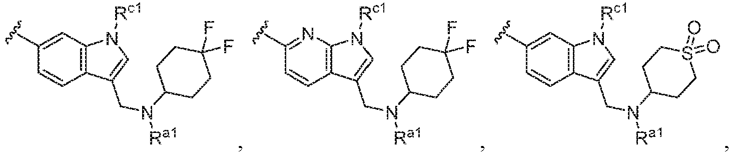 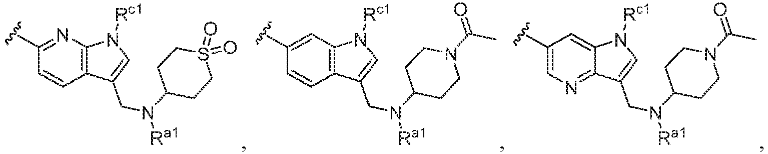 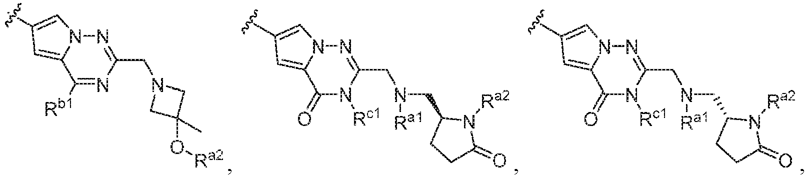 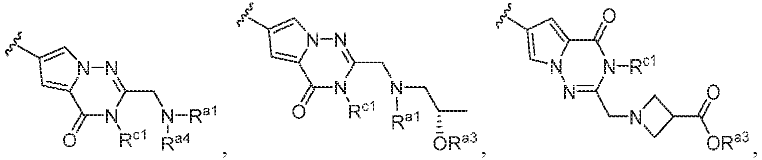 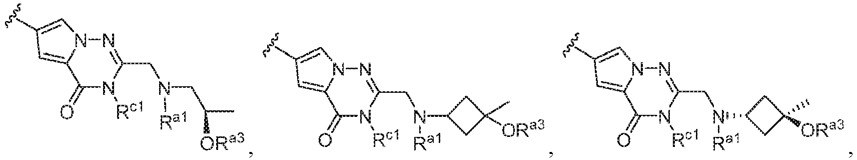 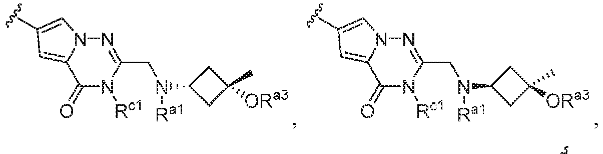 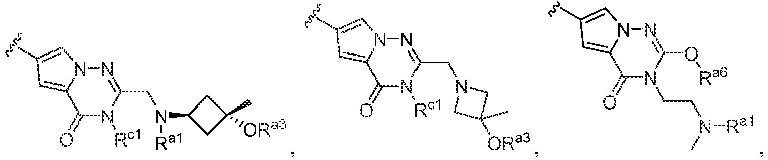 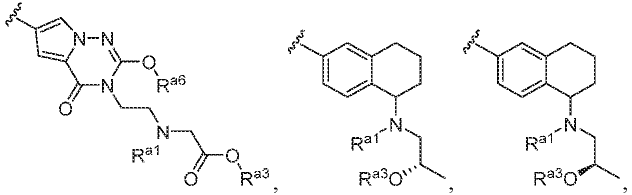 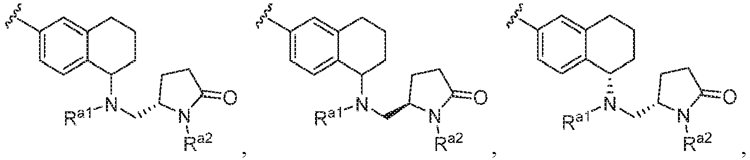 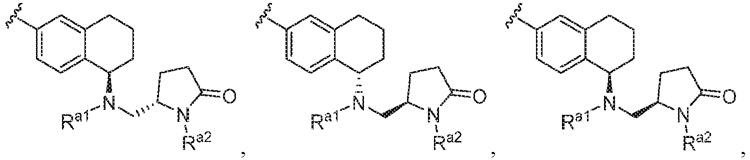 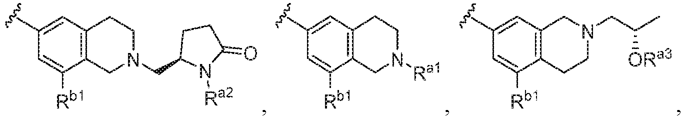 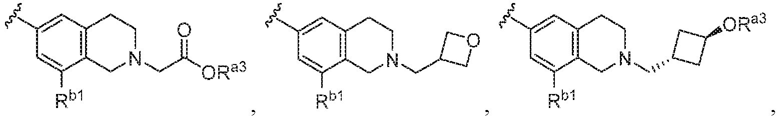 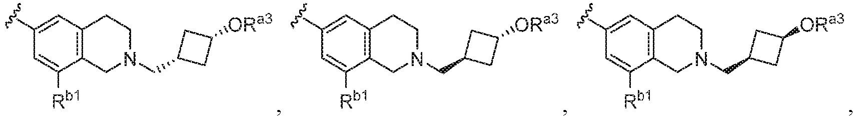 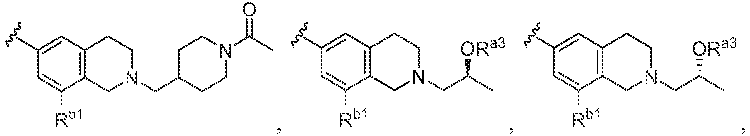 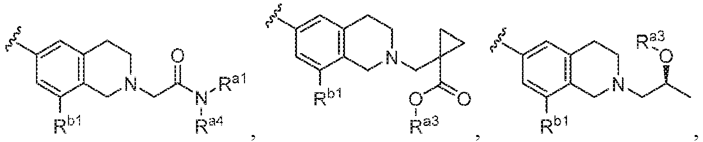 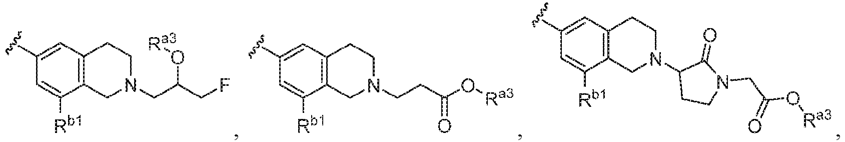 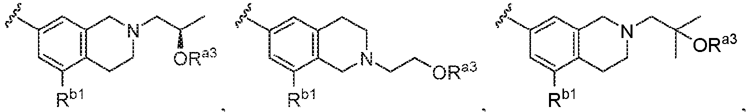 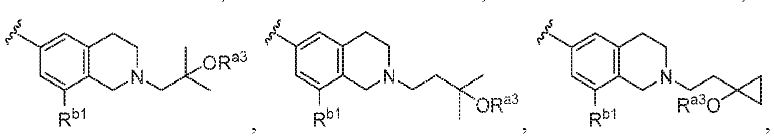 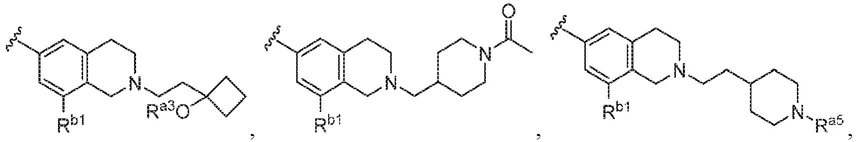 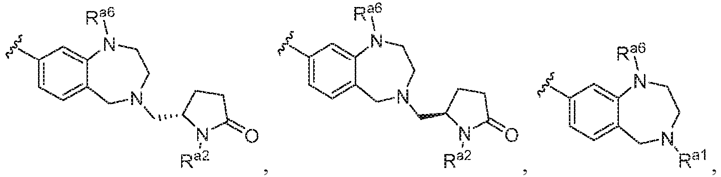 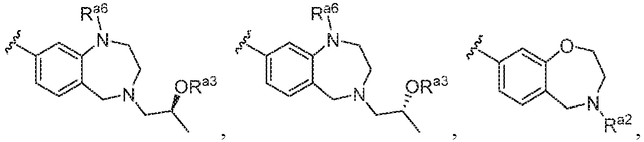 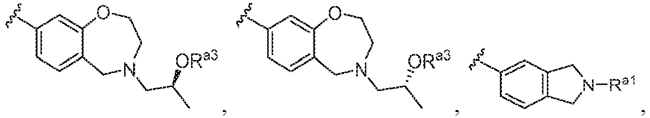 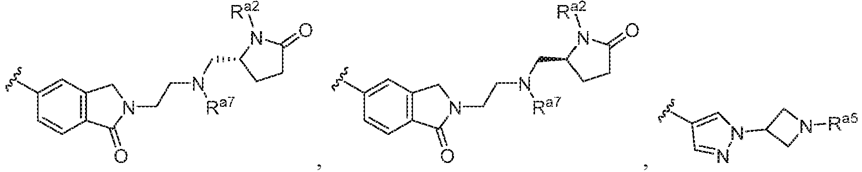 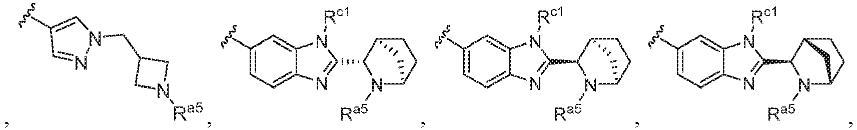 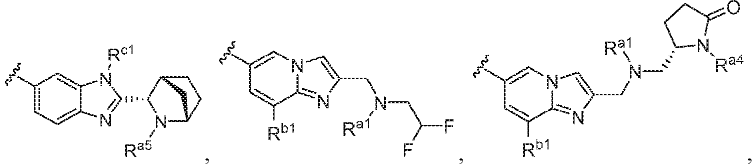 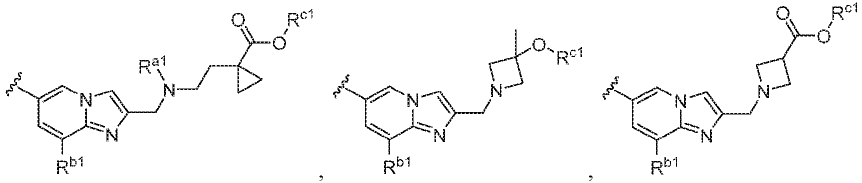 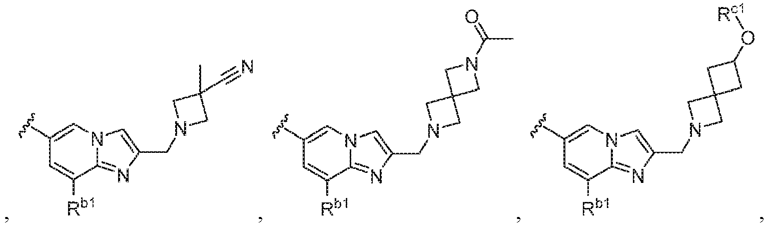 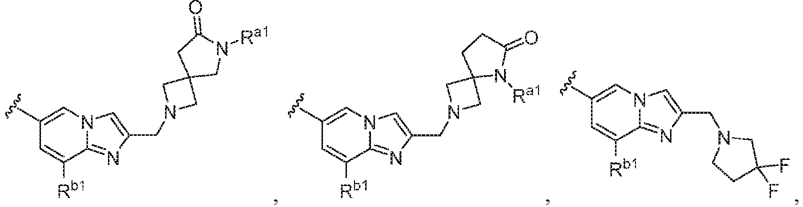 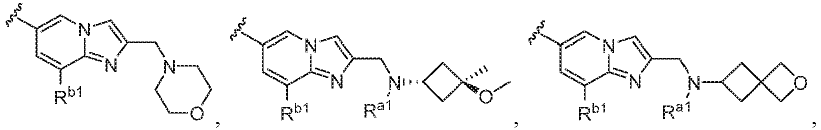 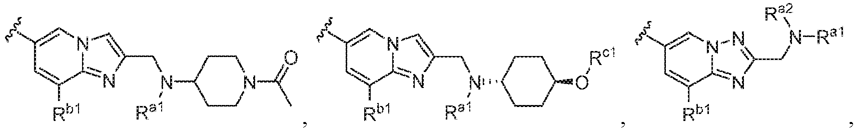 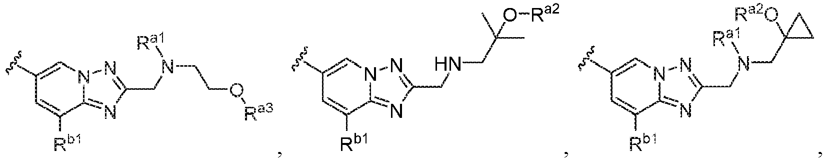 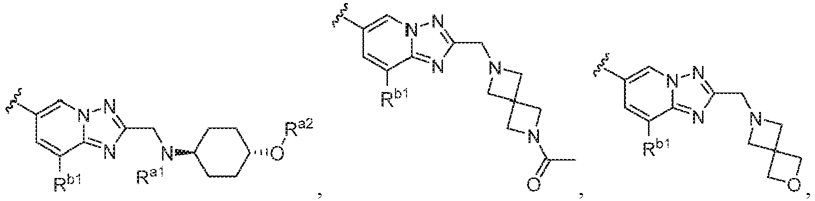 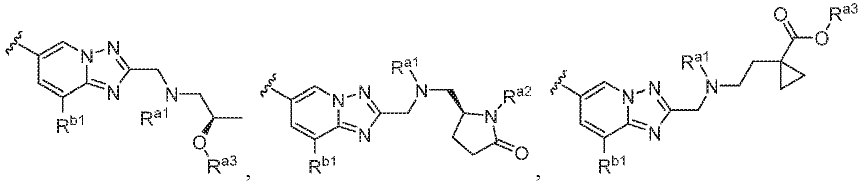 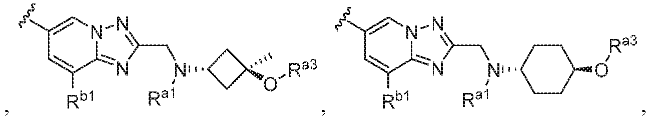 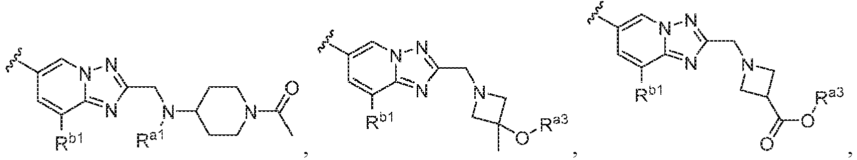 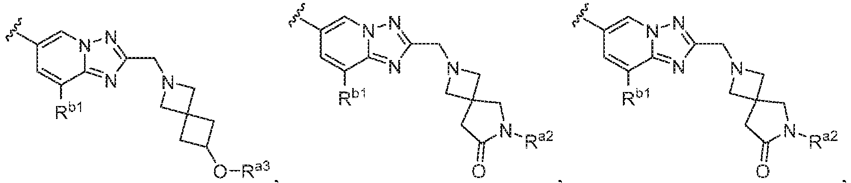 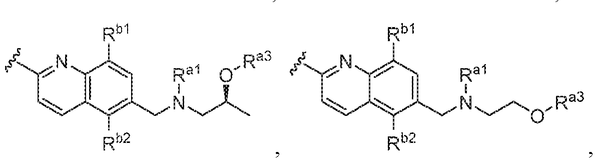 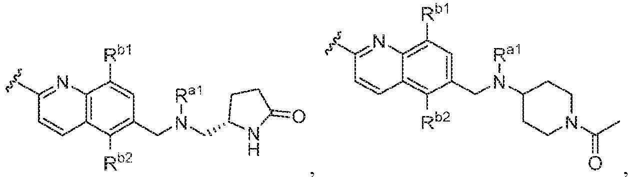 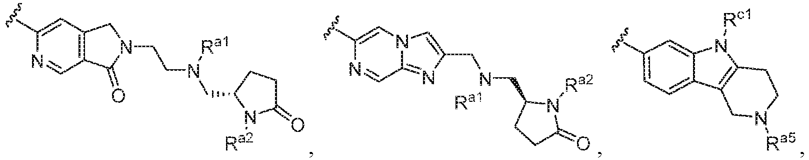 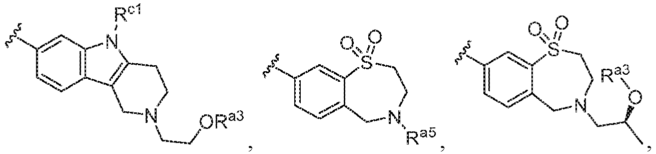 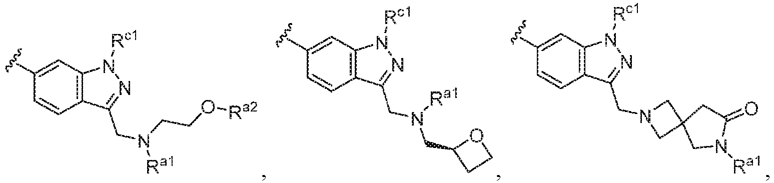 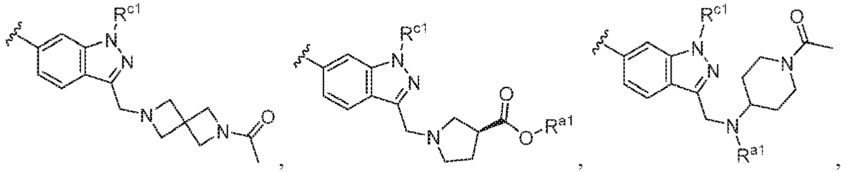 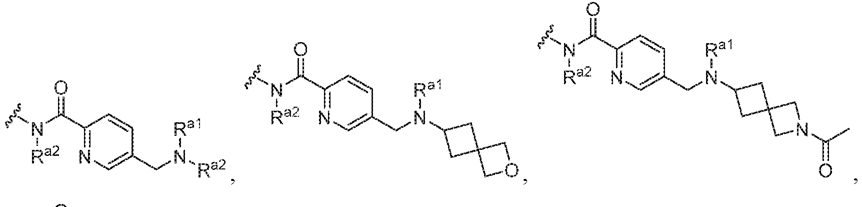 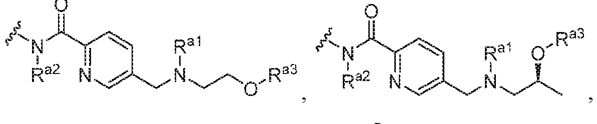 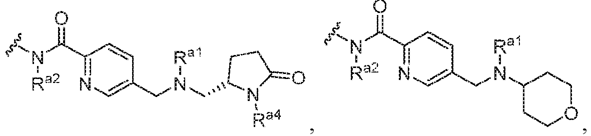 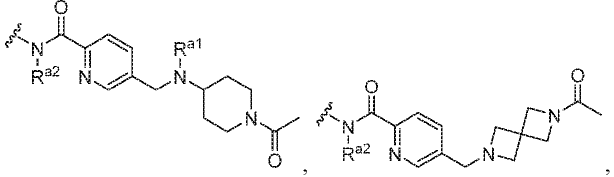 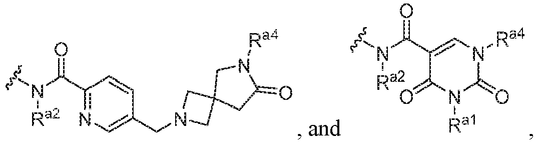 wherein:
each of R^{a1}, R^{az}, R^{a3}, R^{a4} R^{a5}, R^{a6}, and R^{a7} is independently selected from the group consisting of H, C₁-C₆ alkyl, C₃-C₈ cycloalkyl, C₁-C₆ alkoxy, C₁-C₆ haloalkyl, and C₁-C₆ haloalkoxy;
each of R^{b1} and R^{b2} is independently selected from the group consisting of H, C₁-C₃ alkyl, C₃-C₈ cycloalkyl, C₁-C₃ alkoxy, cyano, halogen, C₁-C₃ haloalkyl, and C₁-C₃ haloalkoxy; and
R^{1c} is selected from the group consisting of H, C₁-C₃ alkyl, C₃-C₈ cycloalkyl, C₁-C₃ alkoxy, C₁-C₃ haloalkyl, C₁-C₃ haloalkoxy, and C(=O)OH.

Embodiment 14 provides the compound of any one of Embodiments 1-13, wherein R^{1c} and R^{1d} are independently selected from the group consisting of:

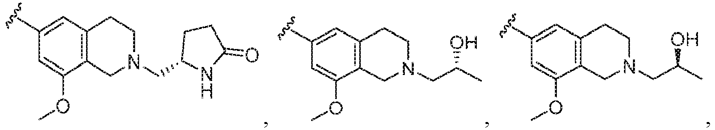

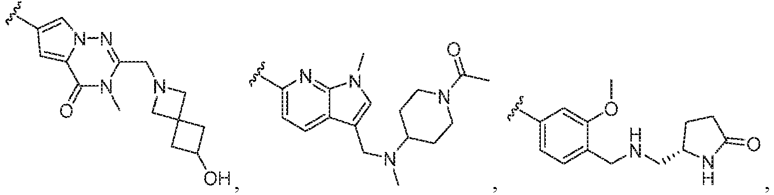

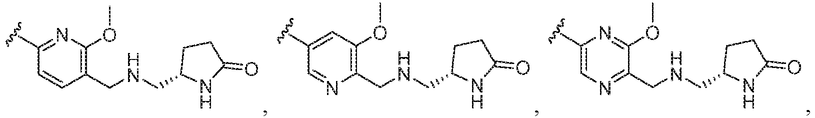

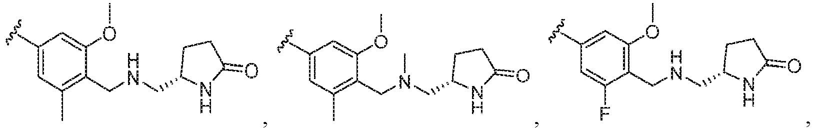

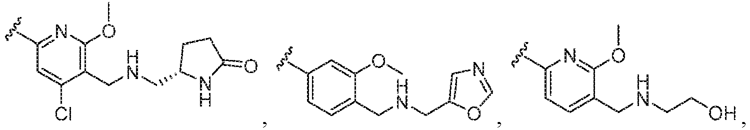

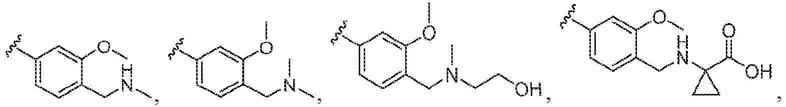

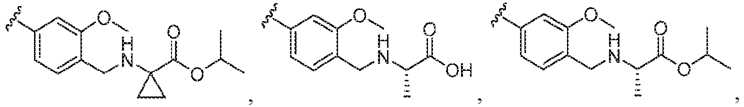

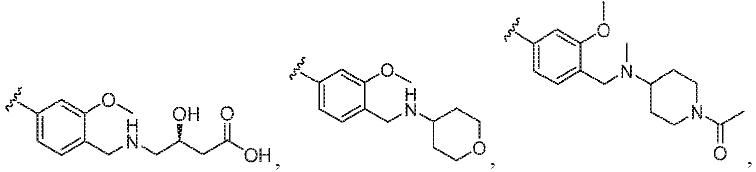

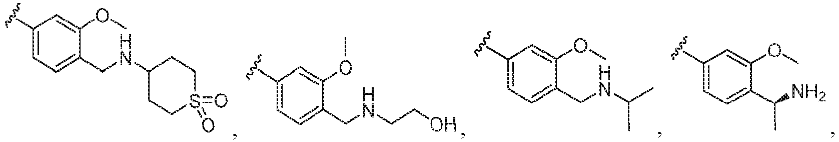

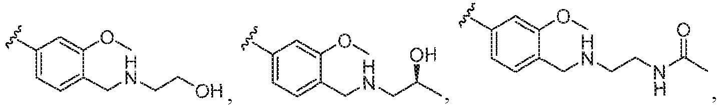

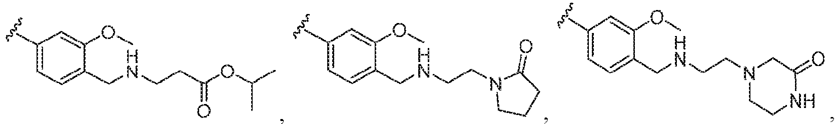

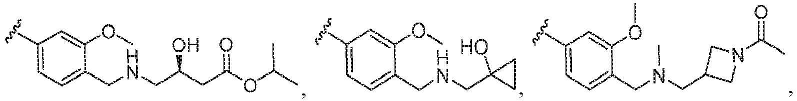

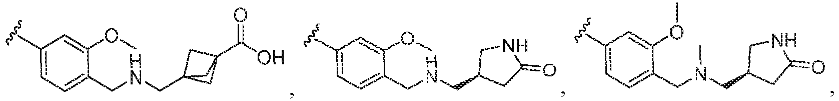

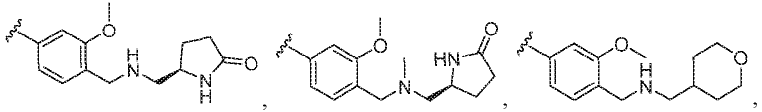

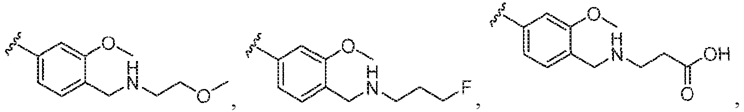

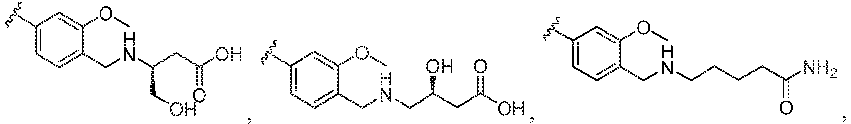

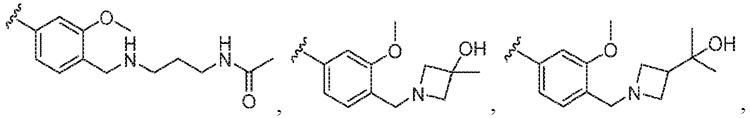

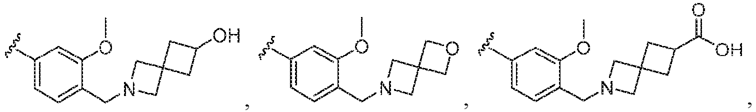

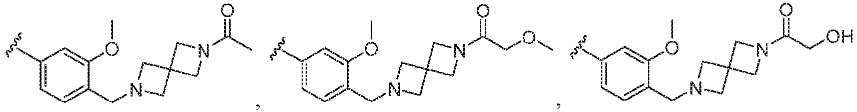

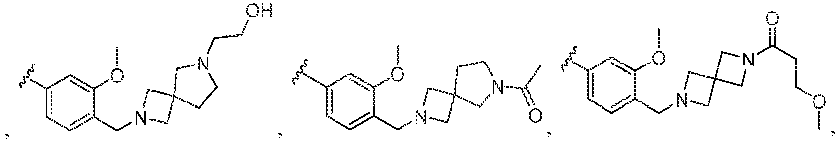

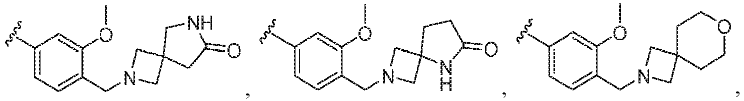

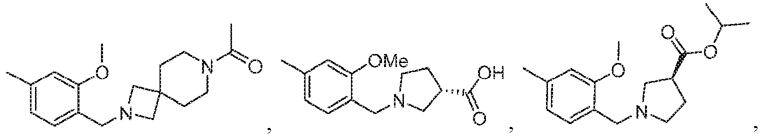

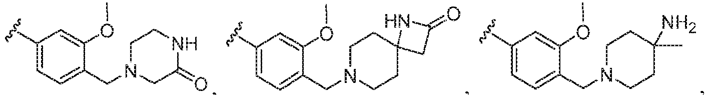

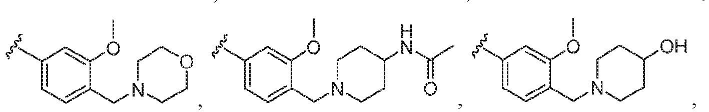

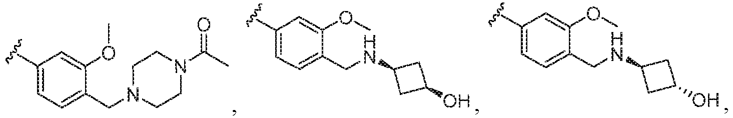

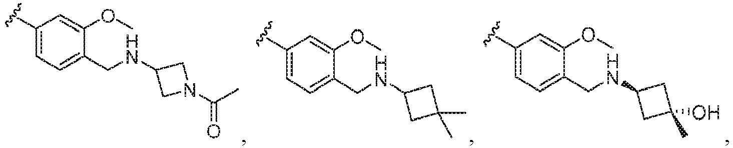

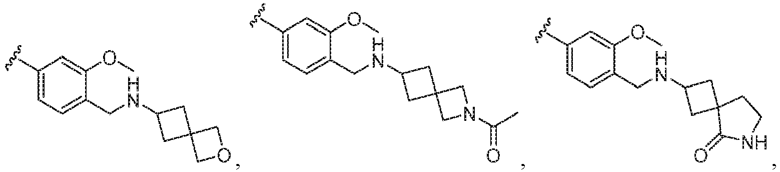

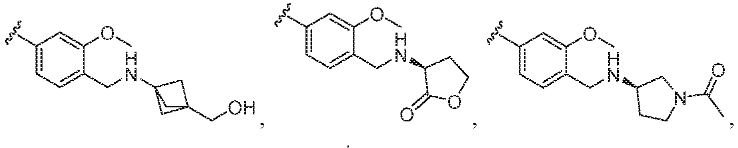

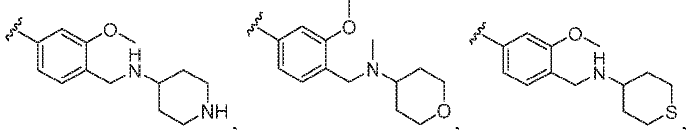

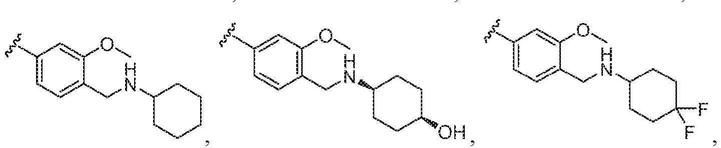

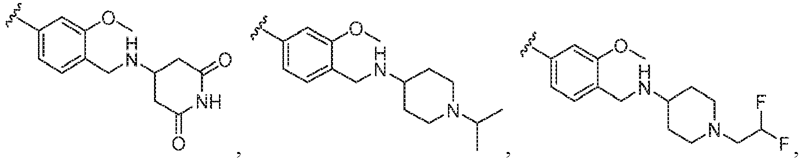

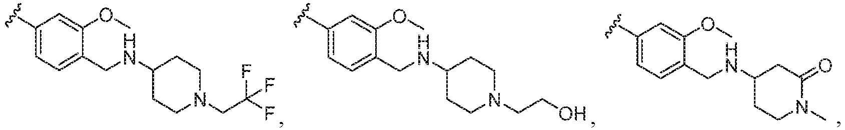

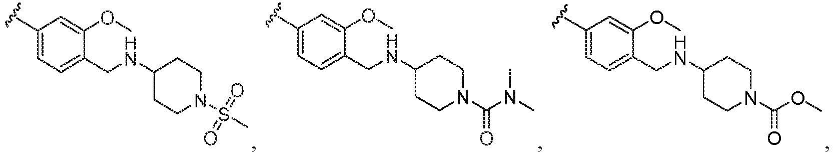

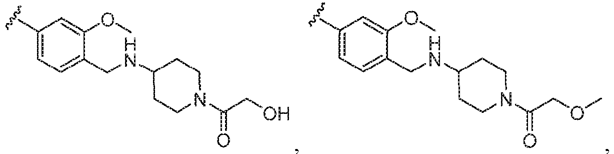

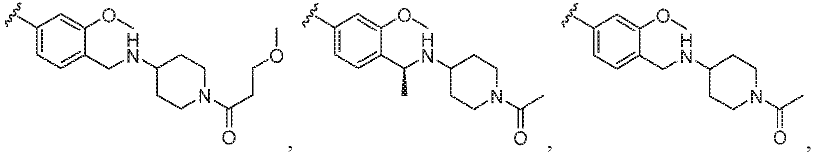

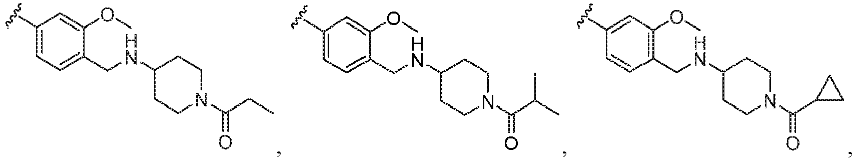

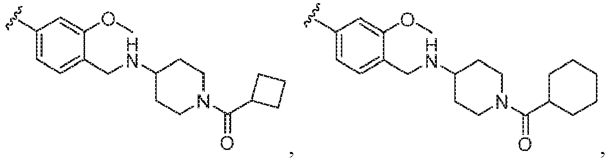

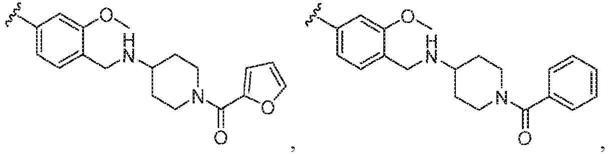

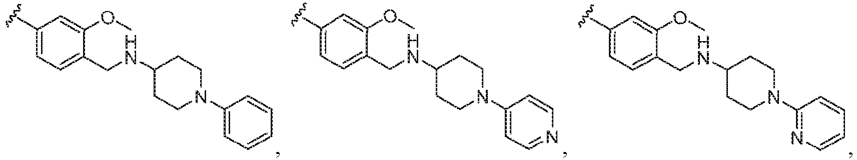

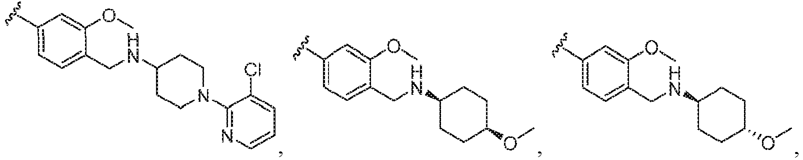

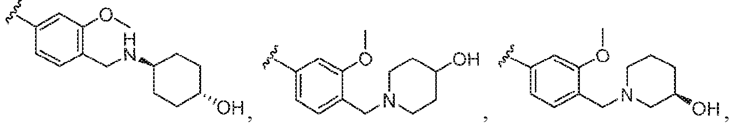

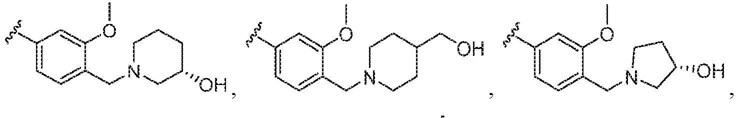

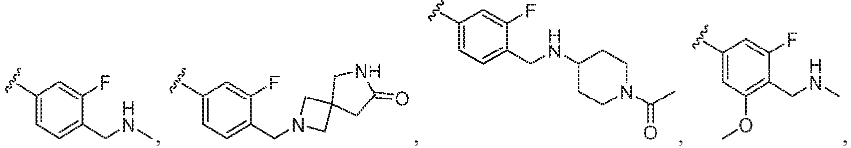

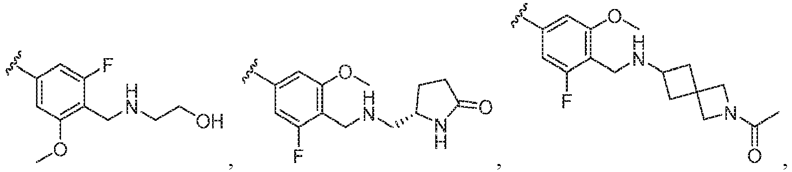

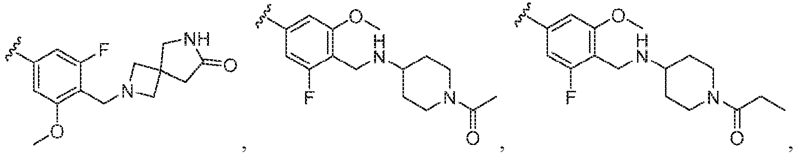

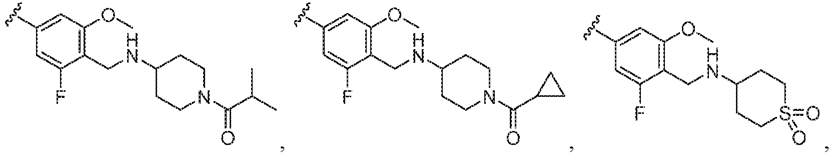

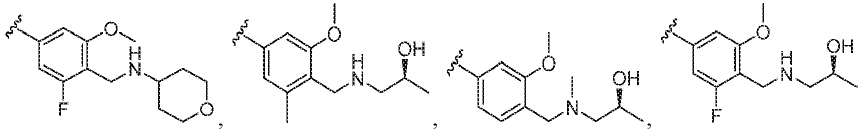

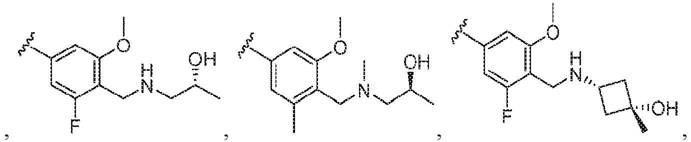

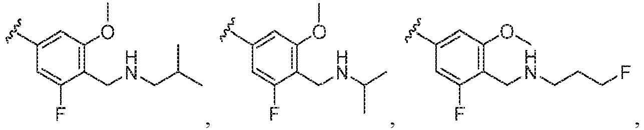

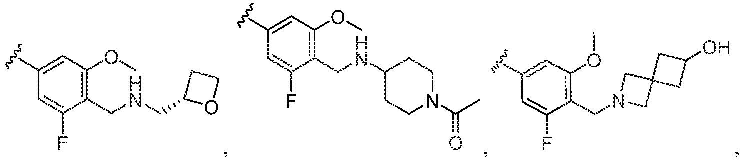

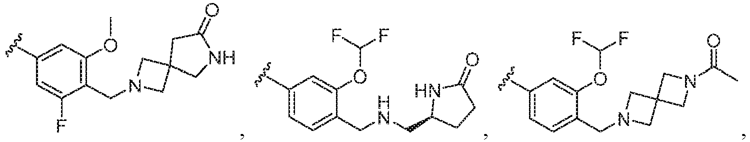

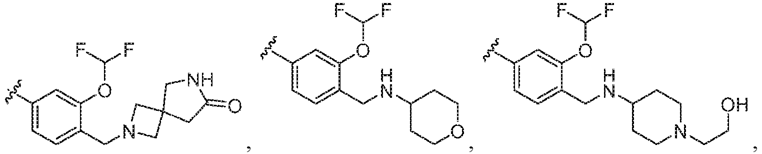

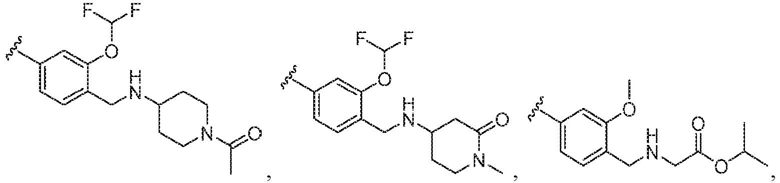

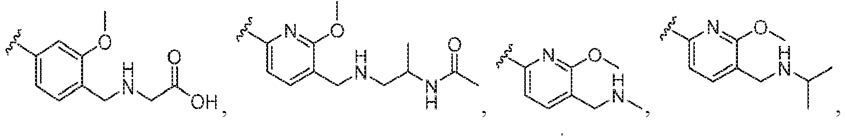

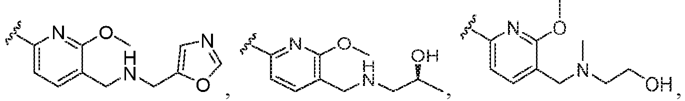

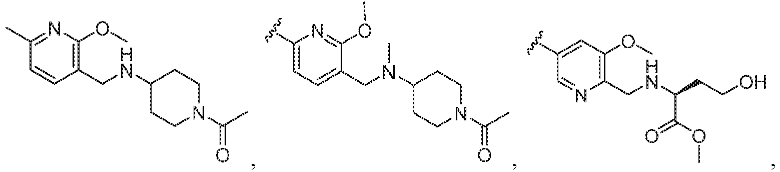

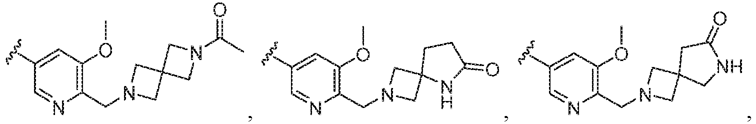

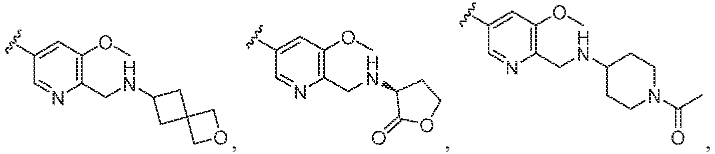

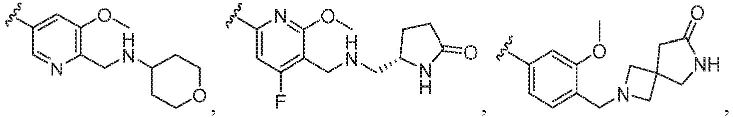

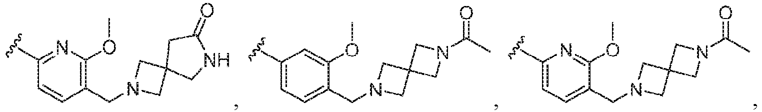

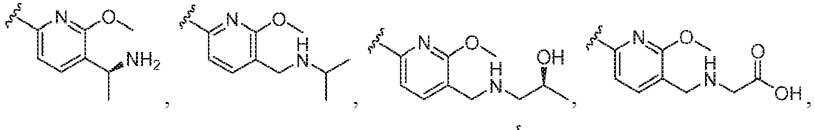

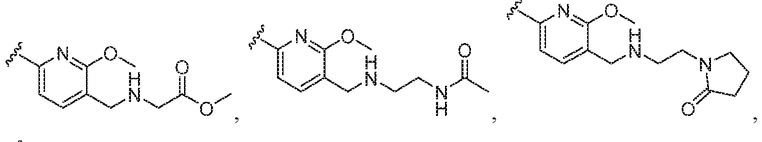

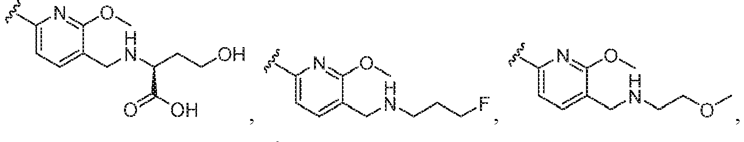

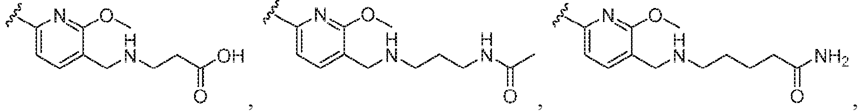

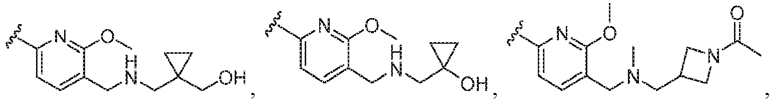

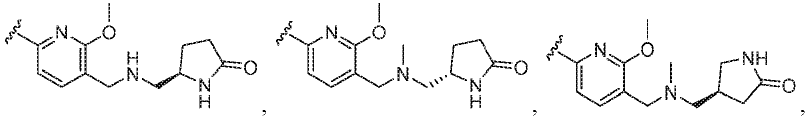

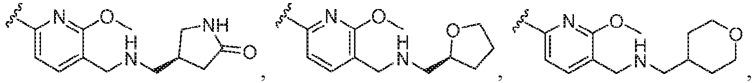

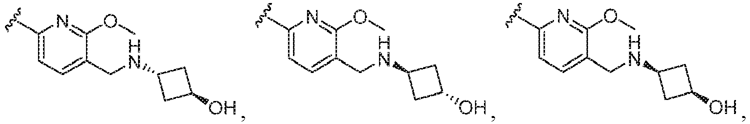

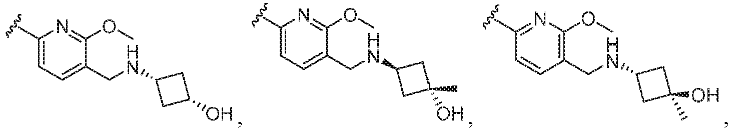

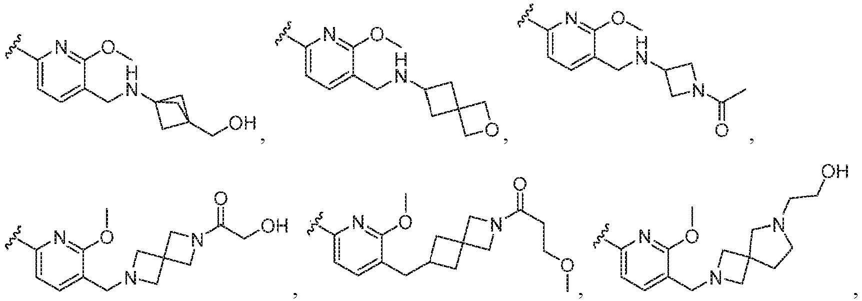

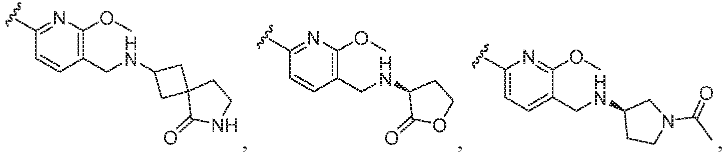

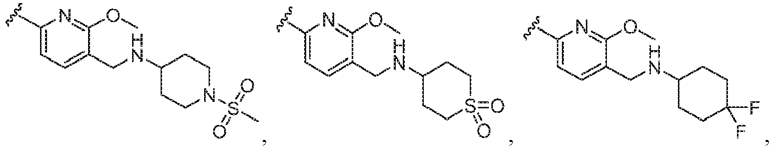

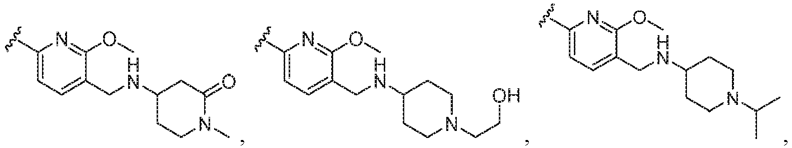

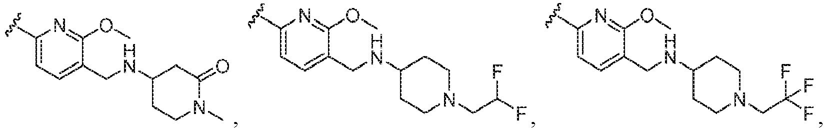

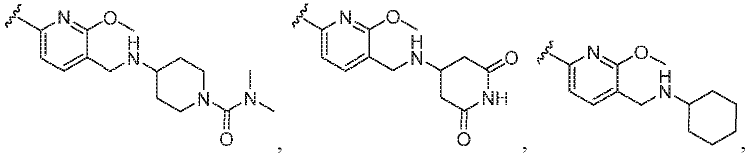

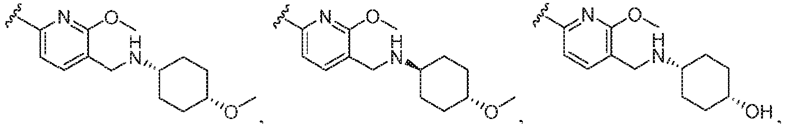

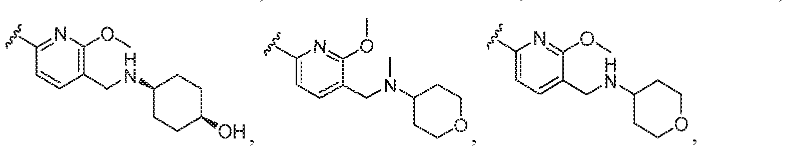

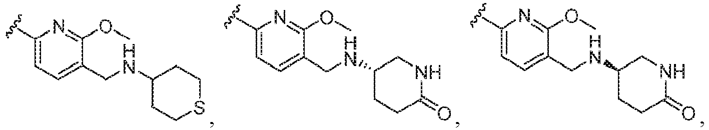

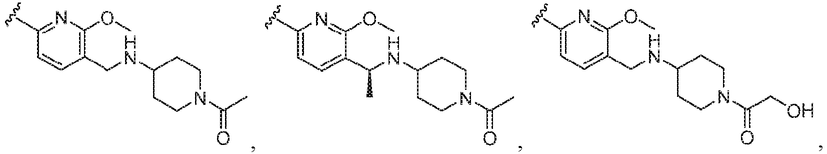

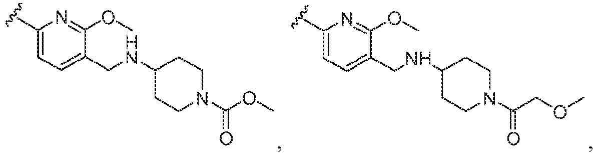

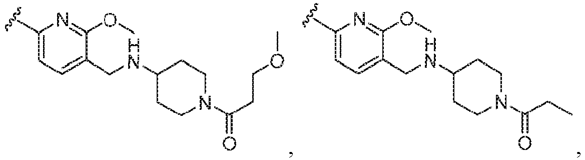

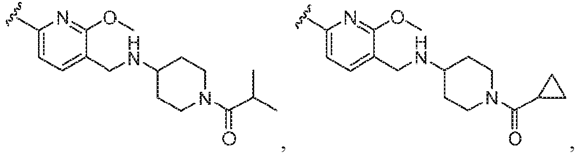

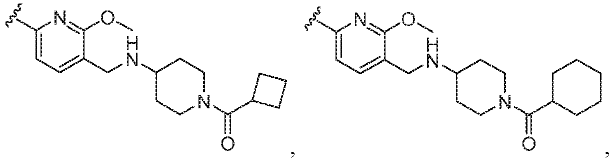

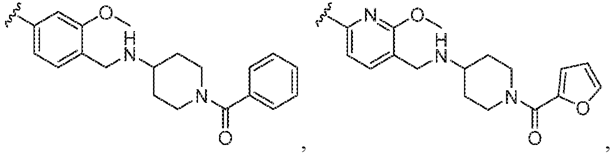

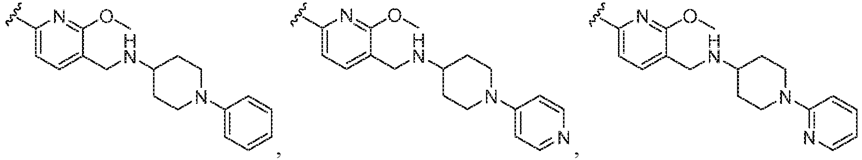

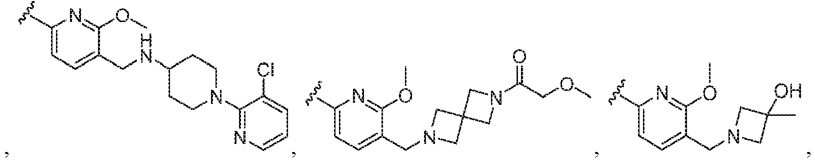

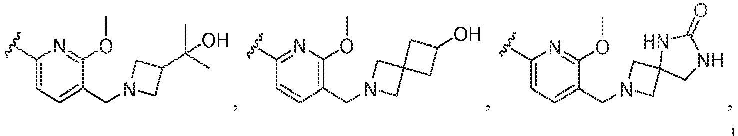

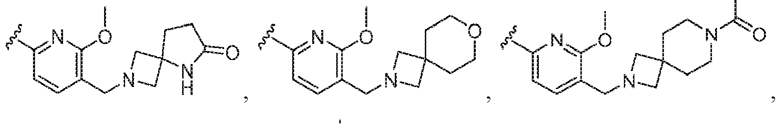

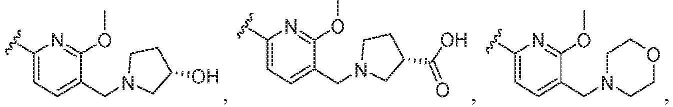

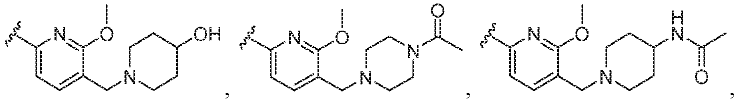

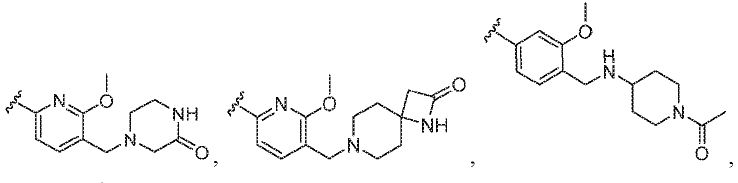

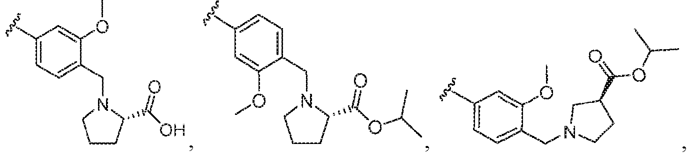

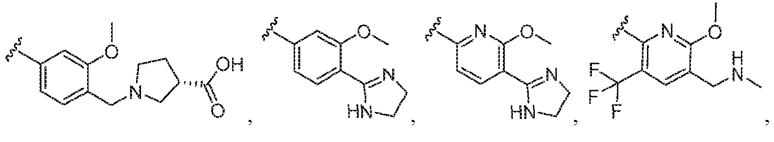

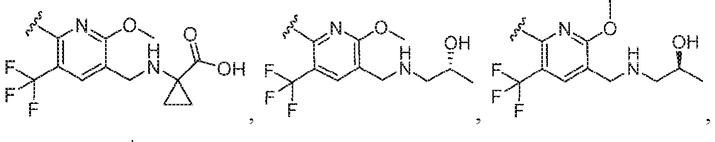

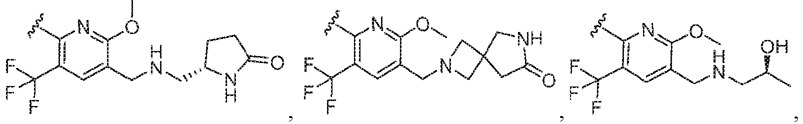

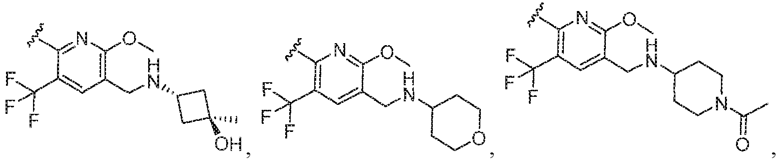

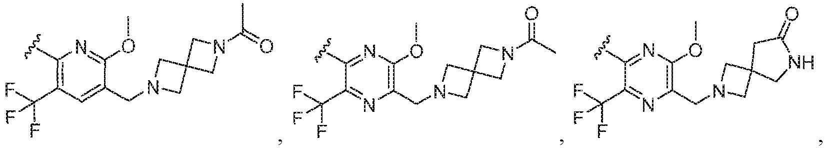

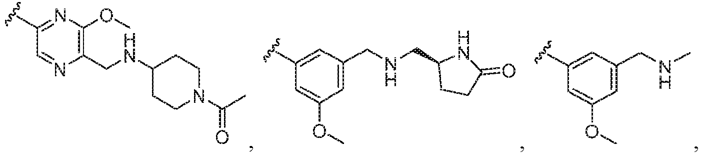

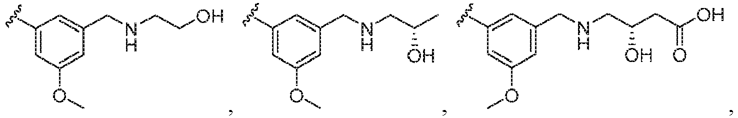

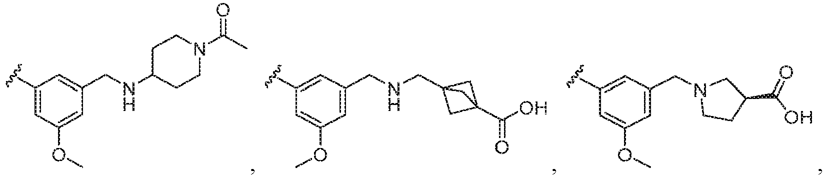

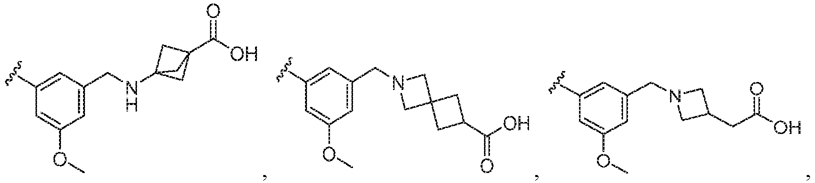

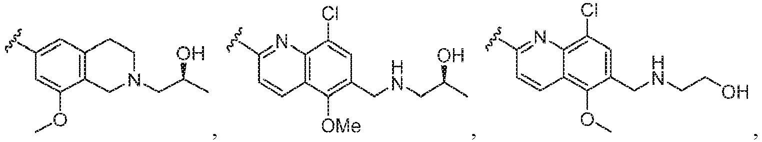

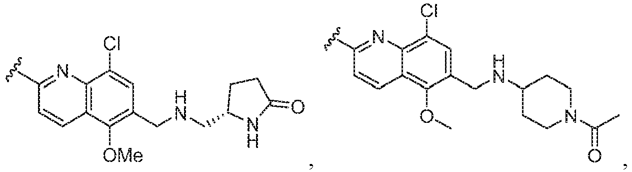

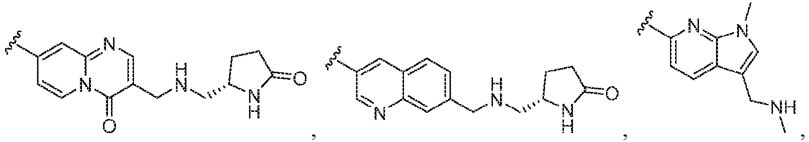

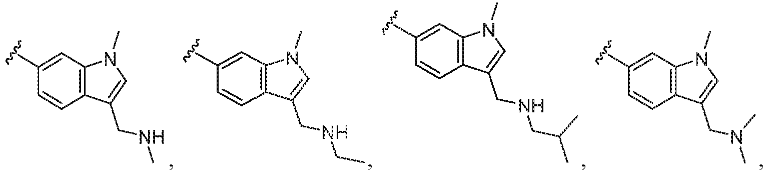

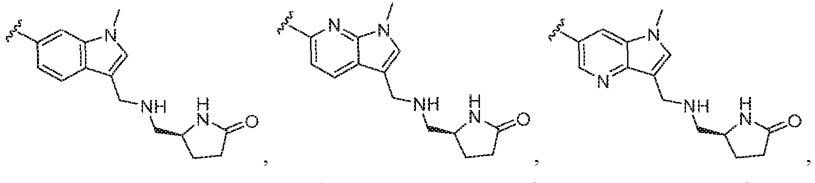

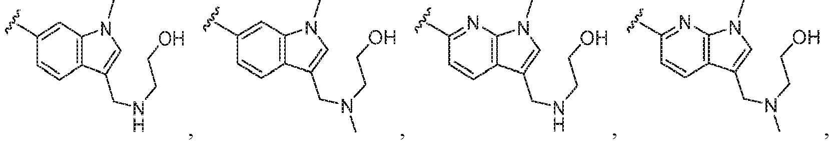

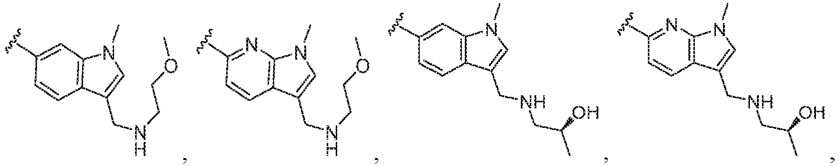

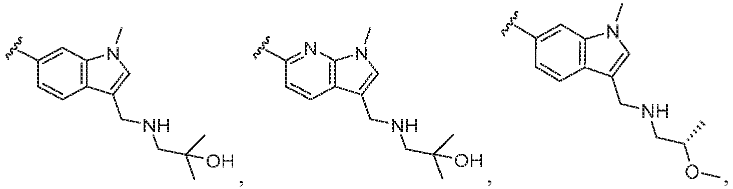

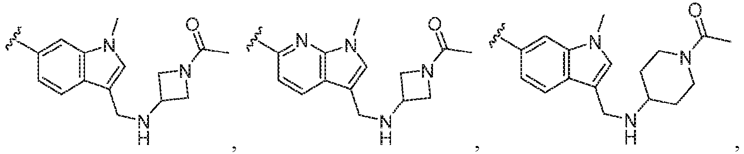

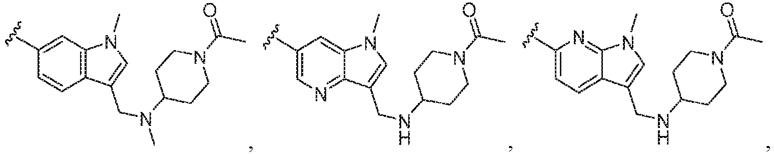

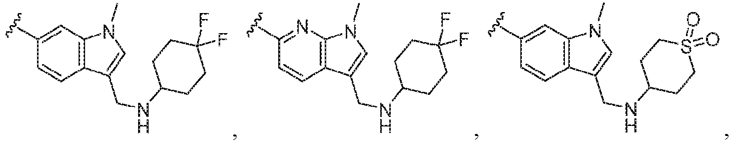

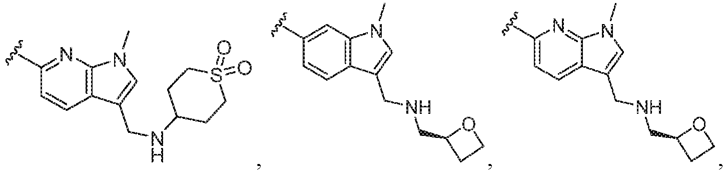

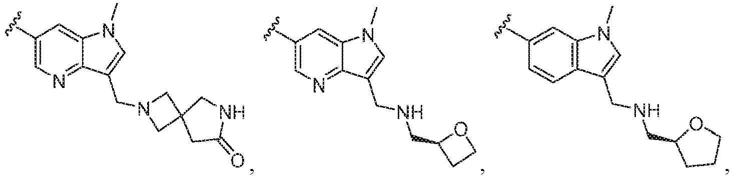

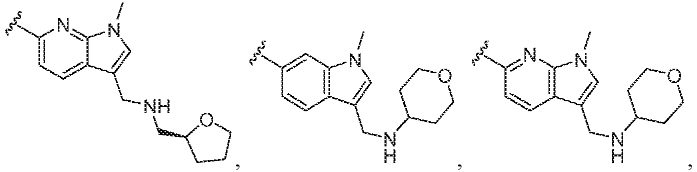

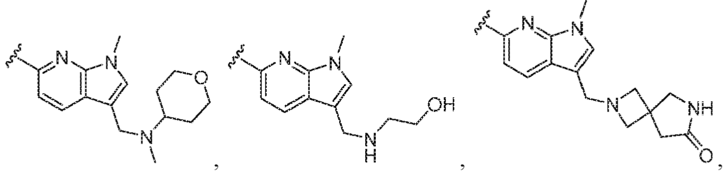

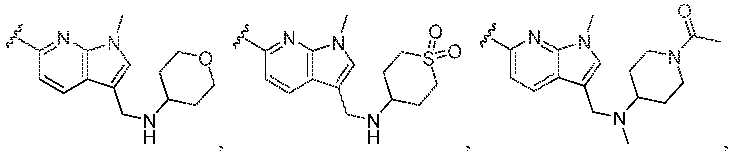

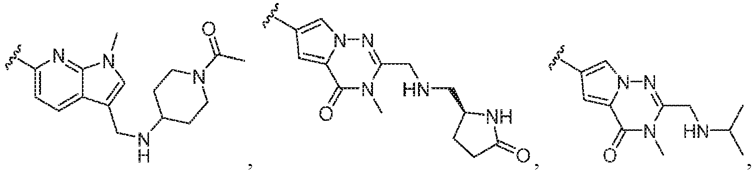

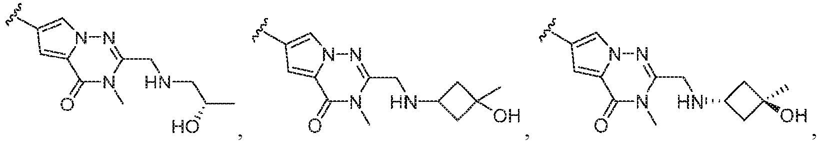

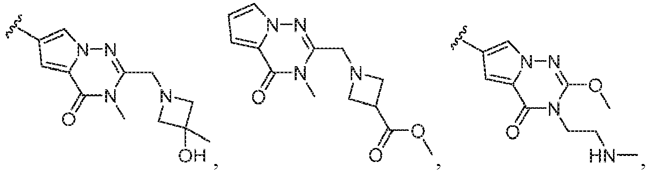

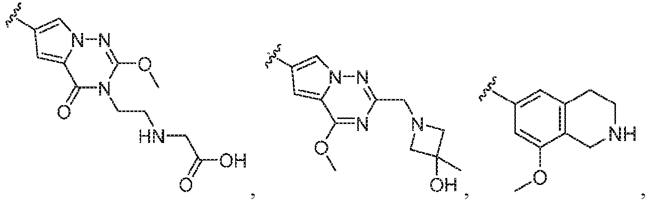

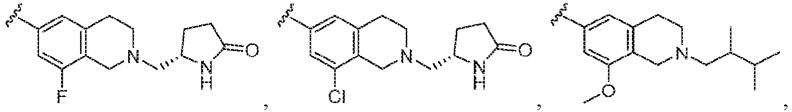

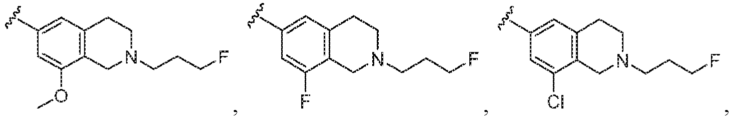

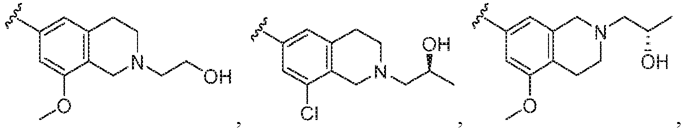

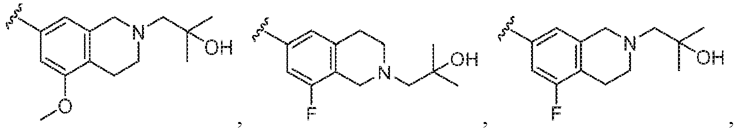

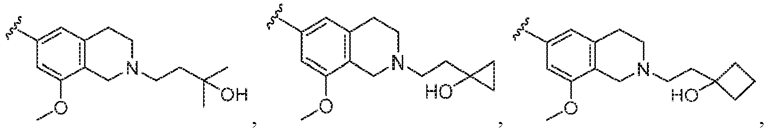

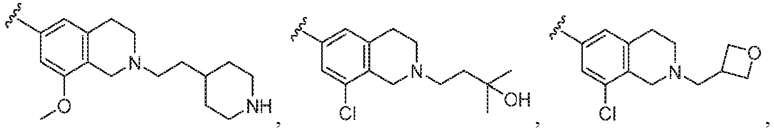

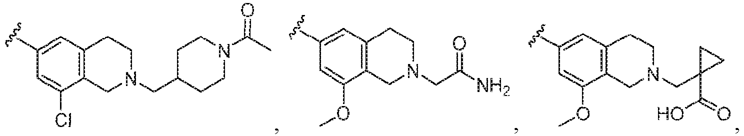

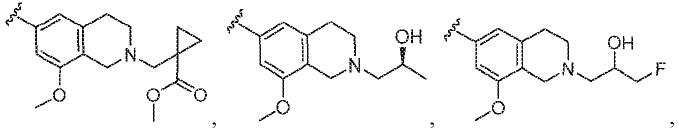

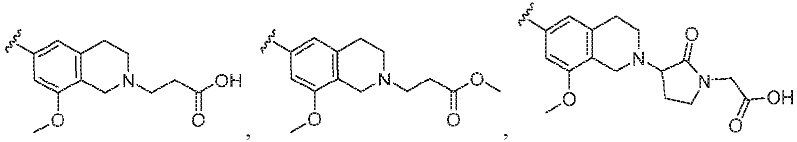

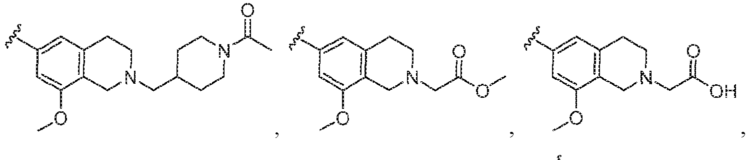

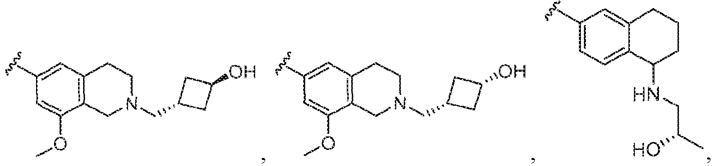

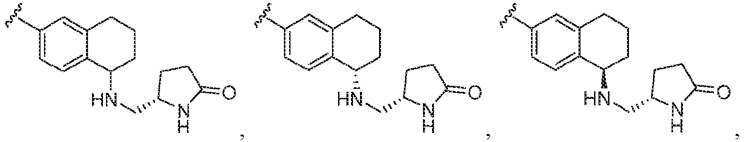

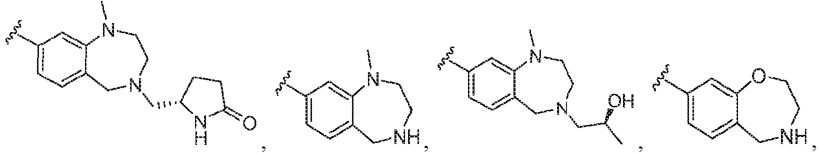

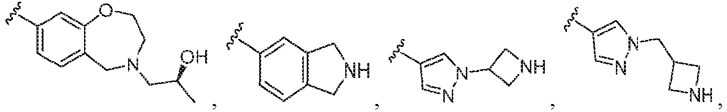

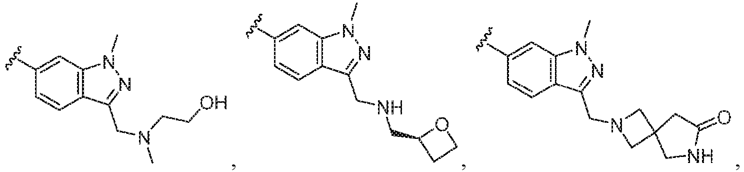

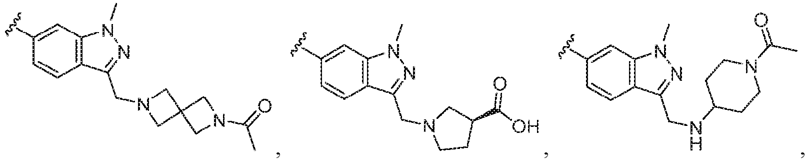

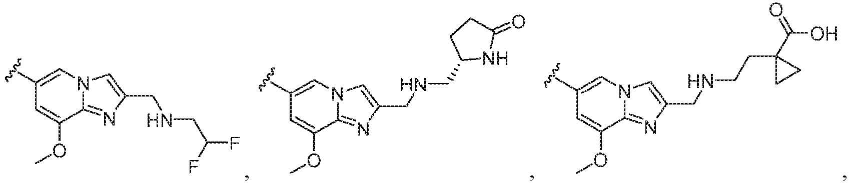

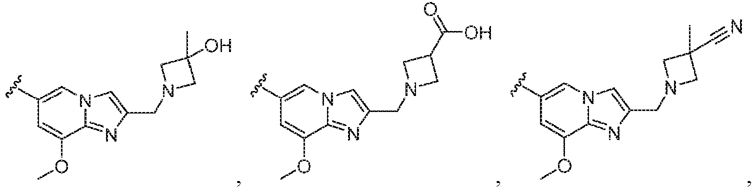

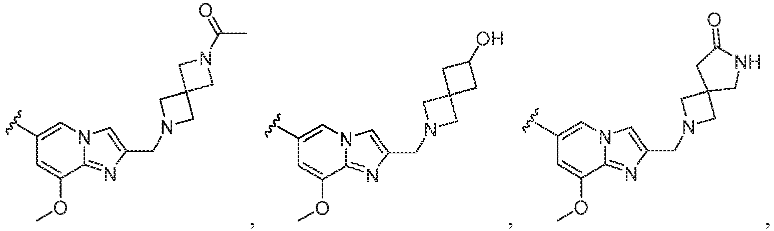

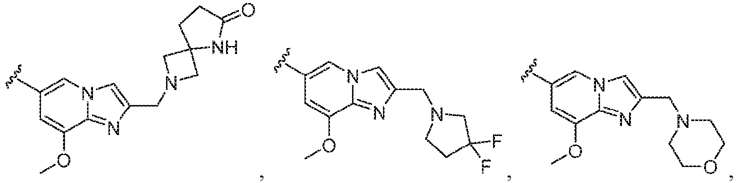

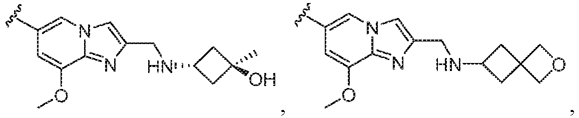

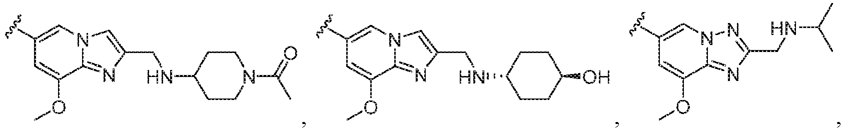

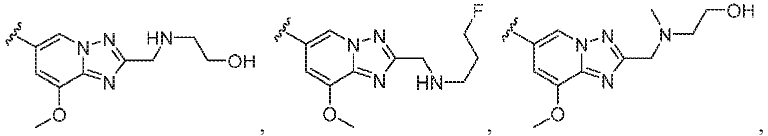

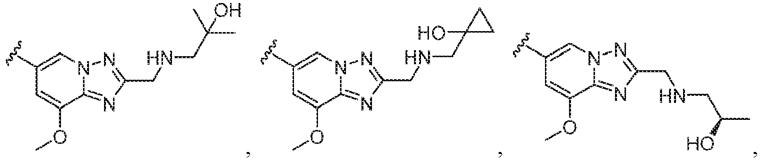

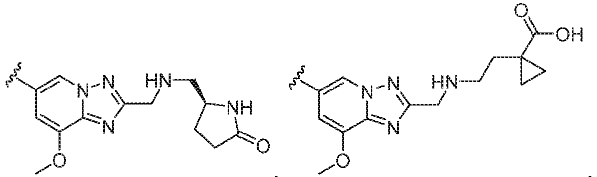

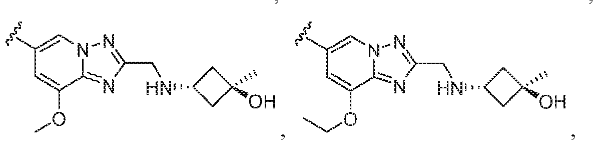

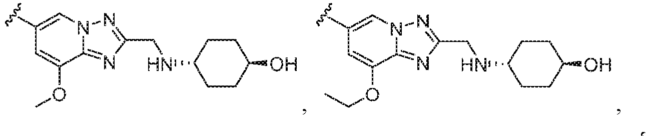

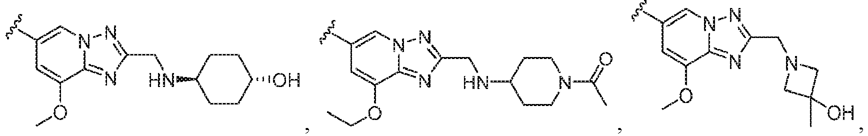

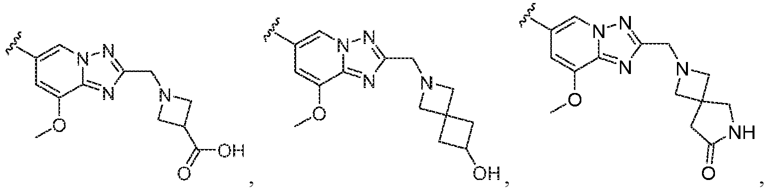

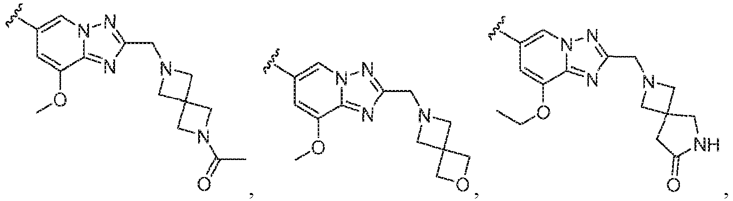

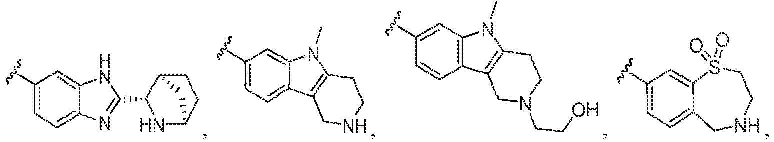

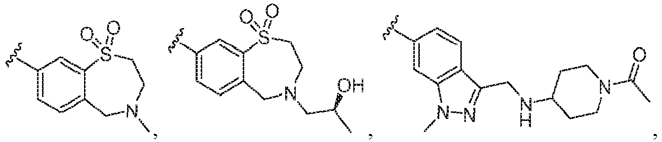

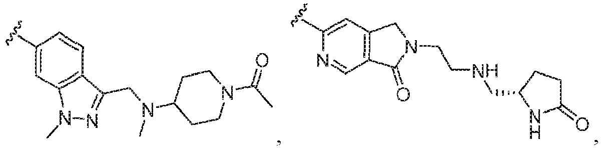

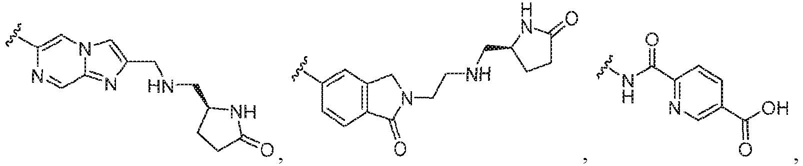

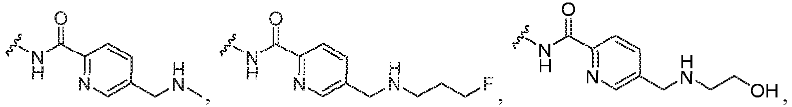

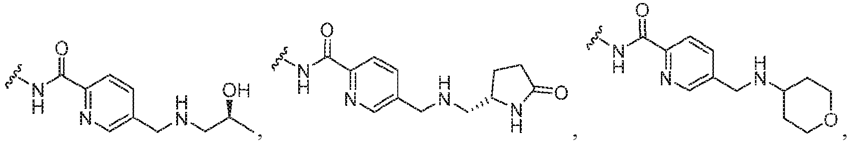

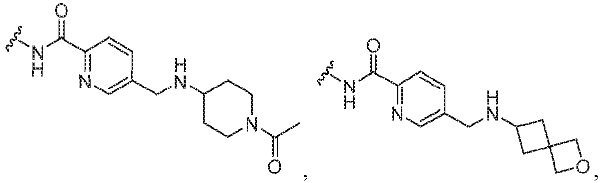

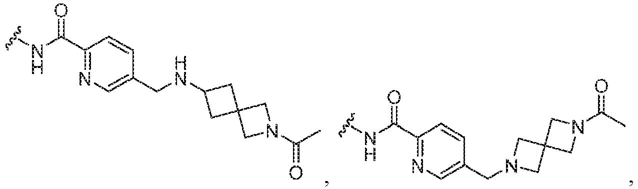

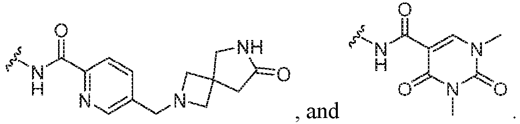

Embodiment 15 provides the compound of any one of Embodiments 1-14, which is selected from the group consisting of:
8-(2-chloro-3-(3-chloro-2-(3-methoxy-4-(((((S)-5-oxopyrrolidin-2-yl)methyl)amino)methyl)phenyl)pyridin-4-yl)phenyl)-3-(((((S)-5-oxopyrrolidin-2-yl)methyl)amino)methyl)-4H-pyrido[1,2-a]pyrimidin-4-one;
8-(2-chloro-3-(3-chloro-2-(3-methoxy-4-(((((S)-5-oxopyrrolidin-2-yl)methyl)amino)methyl)phenyl)pyridin-4-yl)phenyl)-3-(((((R)-5-oxopyrrolidin-2-yl)methyl)amino)methyl)-4H-pyrido[1,2-a]pyrimidin-4-one;
8-(2-chloro-3-(3-chloro-2-(3-methoxy-4-(((((R)-5-oxopyrrolidin-2-yl)methyl)amino)methyl)phenyl)pyridin-4-yl)phenyl)-3-(((((S)-5-oxopyrrolidin-2-yl)methyl)amino)methyl)-4H-pyrido[1,2-a]pyrimidin-4-one;
8-(2-chloro-3-(3-chloro-2-(3-methoxy-4-(((((R)-5-oxopyrrolidin-2-yl)methyl)amino)methyl)phenyl)pyridin-4-yl)phenyl)-3-(((((R)-5-oxopyrrolidin-2-yl)methyl)amino)methyl)-4H-pyrido[1,2-a]pyrimidin-4-one;
8-(3-chloro-4-(2-chloro-3-(6-methoxy-5-(((((S)-5-oxopyrrolidin-2-yl)methyl)amino)methyl)pyridin-2-yl)phenyl)pyridin-2-yl)-3-(((((S)-5-oxopyrrolidin-2-yl)methyl)amino)methyl)-4H-pyrido[1,2-a]pyrimidin-4-one;
8-(3-chloro-4-(2-chloro-3-(6-methoxy-5-(((((S)-5-oxopyrrolidin-2-yl)methyl)amino)methyl)pyridin-2-yl)phenyl)pyridin-2-yl)-3-(((((R)-5-oxopyrrolidin-2-yl)methyl)amino)methyl)-4H-pyrido[1,2-a]pyrimidin-4-one;
8-(3-chloro-4-(2-chloro-3-(6-methoxy-5-(((((R)-5-oxopyrrolidin-2-yl)methyl)amino)methyl)pyridin-2-yl)phenyl)pyridin-2-yl)-3-(((((S)-5-oxopyrrolidin-2-yl)methyl)amino)methyl)-4H-pyrido[1,2-a]pyrimidin-4-one;
8-(3-chloro-4-(2-chloro-3-(6-methoxy-5-(((((R)-5-oxopyrrolidin-2-yl)methyl)amino)methyl)pyridin-2-yl)phenyl)pyridin-2-yl)-3-(((((R)-5-oxopyrrolidin-2-yl)methyl)amino)methyl)-4H-pyrido[1,2-a]pyrimidin-4-one;
(S)-5-((((6-(2-chloro-3-(3-chloro-2-(1-methyl-2,3,4,5-tetrahydro-1H-benzo[e][1,4]diazepin-8-yl)pyridin-4-yl)phenyl)-2-methoxypyridin-3-yl)methyl)amino)methyl)pyrrolidin-2-one;
(R)-5-((((6-(2-chloro-3-(3-chloro-2-(1-methyl-2,3,4,5-tetrahydro-1H-benzo[e][1,4]diazepin-8-yl)pyridin-4-yl)phenyl)-2-methoxypyridin-3-yl)methyl)amino)methyl)pyrrolidin-2-one;
(5 S,5'S)-5,5'-(((((3,3'-dichloro-[4,4'-bipyridine]-2,2'-diyl)bis(2-methoxy-4,1-phenylene))bis(methylene))bis(azanediyl))bis(methylene))bis(pyrrolidin-2-one);
(5S,5'R)-5,5'-(((((3,3'-dichloro-[4,4'-bipyridine]-2,2'-diyl)bis(2-methoxy-4,1-phenylene))bis(methylene))bis(azanediyl))bis(methylene))bis(pyrrolidin-2-one);
(5R,5'S)-5,5'-(((((3,3'-dichloro-[4,4'-bipyridine]-2,2'-diyl)bis(2-methoxy-4,1-phenylene))bis(methylene))bis(azanediyl))bis(methylene))bis(pyrrolidin-2-one);
(5R,5'R)-5,5'-(((((3,3'-dichloro-[4,4'-bipyridine]-2,2'-diyl)bis(2-methoxy-4,1-phenylene))bis(methylene))bis(azanediyl))bis(methylene))bis(pyrrolidin-2-one);
N-(1-(((6-(2-chloro-3-(3-chloro-2-(1-methyl-2,3,4,5-tetrahydro-1H-benzo[e][1,4]diazepin-8-yl)pyridin-4-yl)phenyl)-2-methoxypyridin-3-yl)methyl)amino)propan-2-yl)acetamide;
(S)-N-(1-(((6-(2-chloro-3-(3-chloro-2-(1-methyl-2,3,4,5-tetrahydro-1H-benzo[e][1,4]diazepin-8-yl)pyridin-4-yl)phenyl)-2-methoxypyridin-3-yl)methyl)amino)propan-2-yl)acetamide;
(R)-N-(1-(((6-(2-chloro-3-(3-chloro-2-(1-methyl-2,3,4,5-tetrahydro-1H-benzo[e][1,4]diazepin-8-yl)pyridin-4-yl)phenyl)-2-methoxypyridin-3-yl)methyl)amino)propan-2-yl)acetamide;
(S)-5-((((6-(2-chloro-3-(3-chloro-2-(8-methoxy-2-(((S)-5-oxopyrrolidin-2-yl)methyl)-1,2,3,4-tetrahydroisoquinolin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(8-methoxy-2-(((R)-5-oxopyrrolidin-2-yl)methyl)-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(8-methoxy-2-(((S)-5-oxopyrrolidin-2-yl)methyl)-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(8-methoxy-2-(((R)-5-oxopyrrolidin-2-yl)methyl)-1,2,3,4-tetrahydroisoquinolin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(8-methoxy-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(8-methoxy-1,2,3,4-tetrahydroisoquinolin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(3-methoxy-4-(((((S)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
(S)-5-((((6-(2-chloro-3-(3-chloro-2-(3-methoxy-4-(((((R)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
(R)-5-((((6-(2-chloro-3-(3-chloro-2-(3-methoxy-4-(((((S)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
(R)-5-((((6-(2-chloro-3-(3-chloro-2-(3-methoxy-4-(((((R)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
(S)-5-((((6-(2-chloro-3-(3-chloro-2-(1-methyl-2,3,4,5-tetrahydro-1H-benzo[e][1,4]diazepin-8-yl)pyridin-4-yl)phenyl)-1-methyl-1H-pyrrolo[2,3-b]pyridin-3-yl)methyl)amino)methyl)pyrrolidin-2-one;
(R)-5-((((6-(2-chloro-3-(3-chloro-2-(1-methyl-2,3,4,5-tetrahydro-1H-benzo[e][1,4]diazepin-8-yl)pyridin-4-yl)phenyl)-1-methyl-1H-pyrrolo[2,3-b]pyridin-3-yl)methyl)amino)methyl)pyrrolidin-2-one;
(S)-5-((((6-(2-chloro-3-(3-chloro-2-(3-fluoro-5-methoxy-4-(((((S)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
(S)-5-((((6-(2-chloro-3-(3-chloro-2-(3-fluoro-5-methoxy-4-(((((R)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
(R)-5-((((6-(2-chloro-3-(3-chloro-2-(3-fluoro-5-methoxy-4-(((((S)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
(R)-5-((((6-(2-chloro-3-(3-chloro-2-(3-fluoro-5-methoxy-4-(((((R)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
(S)-5-((((6-(2-chloro-3-(3-chloro-2-(1-methyl-3-(((((S)-5-oxopyrrolidin-2-yl)methyl)amino)methyl)-1H-indol-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(1-methyl-3-(((((R)-5-oxopyrrolidin-2-yl)methyl)amino)methyl)-1H-indol-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(1-methyl-3-(((((S)-5-oxopyrrolidin-2-yl)methyl)amino)methyl)-1H-indol-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(1-methyl-3-(((((R)-5-oxopyrrolidin-2-yl)methyl)amino)methyl)-1H-indol-6-yl)pyridin-4-yl)phenyl)-2-methoxypyridin-3-yl)methyl)amino)methyl)pyrrolidin-2-one;
(5S,5'S)-5,5'-(((((3,3'-dichloro-[4,4'-bipyridine]-2,2'-diyl)bis(2-fluoro-6-methoxy-4,1-phenylene))bis(methylene))bis(azanediyl))bis(methylene))bis(pyrrolidin-2-one);
(5S,5'R)-5,5'-(((((3,3'-dichloro-[4,4'-bipyridine]-2,2'-diyl)bis(2-fluoro-6-methoxy-4,1-phenylene))bis(methylene))bis(azanediyl))bis(methylene))bis(pyrrolidin-2-one);
(5R,5'S)-5,5'-(((((3,3'-dichloro-[4,4'-bipyridine]-2,2'-diyl)bis(2-fluoro-6-methoxy-4,1-phenylene))bis(methylene))bis(azanediyl))bis(methylene))bis(pyrrolidin-2-one);
(5R,5'R)-5,5'-(((((3,3'-dichloro-[4,4'-bipyridine]-2,2'-diyl)bis(2-fluoro-6-methoxy-4,1-phenylene))bis(methylene))bis(azanediyl))bis(methylene))bis(pyrrolidin-2-one);
(S)-5-((((6-(2-chloro-3-(3-chloro-2-(1-methyl-3-(((((S)-5-oxopyrrolidin-2-yl)methyl)amino)methyl)-1H-pyrrolo[3,2-b]pyridin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(1-methyl-3-(((((R)-5-oxopyrrolidin-2-yl)methyl)amino)methyl)-1H-pyrrolo[3,2-b]pyridin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(1-methyl-3-(((((S)-5-oxopyrrolidin-2-yl)methyl)amino)methyl)-1H-pyrrolo[3,2-b]pyridin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(1-methyl-3-(((((R)-5-oxopyrrolidin-2-yl)methyl)amino)methyl)-1H-pyrrolo[3,2-b]pyridin-6-yl)pyridin-4-yl)phenyl)-2-methoxypyridin-3-yl)methyl)amino)methyl)pyrrolidin-2-one;
(S)-5-(((4-(3,3'-dichloro-2'-(1-methyl-3-(((((S)-5-oxopyrrolidin-2-yl)methyl)amino)methyl)-1H-indol-6-yl)-[4,4'-bipyridin]-2-yl)-2-methoxybenzyl)amino)methyl)pyrrolidin-2-one;
(S)-5-(((4-(3,3'-dichloro-2'-(1-methyl-3-(((((R)-5-oxopyrrolidin-2-yl)methyl)amino)methyl)-1H-indol-6-yl)-[4,4'-bipyridin]-2-yl)-2-methoxybenzyl)amino)methyl)pyrrolidin-2-one;
(R)-5-(((4-(3,3'-dichloro-2'-(1-methyl-3-(((((S)-5-oxopyrrolidin-2-yl)methyl)amino)methyl)-1H-indol-6-yl)-[4,4'-bipyridin]-2-yl)-2-methoxybenzyl)amino)methyl)pyrrolidin-2-one;
(R)-5-(((4-(3,3'-dichloro-2'-(1-methyl-3-(((((R)-5-oxopyrrolidin-2-yl)methyl)amino)methyl)-1H-indol-6-yl)-[4,4'-bipyridin]-2-yl)-2-methoxybenzyl)amino)methyl)pyrrolidin-2-one;
(5S,5'S)-5,5'-(((((3,3'-dichloro-[4,4'-bipyridine]-2,2'-diyl)bis(1-methyl-1H-indole-6,3-diyl))bis(methylene))bis(azanediyl))bis(methylene))bis(pyrrolidin-2-one);
(5S,5'R)-5,5'-(((((3,3'-dichloro-[4,4'-bipyridine]-2,2'-diyl)bis(1-methyl-1H-indole-6,3-diyl))bis(methylene))bis(azanediyl))bis(methylene))bis(pyrrolidin-2-one);
(5R,5'S)-5,5'-(((((3,3'-dichloro-[4,4'-bipyridine]-2,2'-diyl)bis(1-methyl-1H-indole-6,3-diyl))bis(methylene))bis(azanediyl))bis(methylene))bis(pyrrolidin-2-one);
(5R,5'R)-5,5'-(((((3,3'-dichloro-[4,4'-bipyridine]-2,2'-diyl)bis(1-methyl-1H-indole-6,3-diyl))bis(methylene))bis(azanediyl))bis(methylene))bis(pyrrolidin-2-one);
(S)-5-(((4-(3,3'-dichloro-2'-(1-methyl-3-(((((S)-5-oxopyrrolidin-2-yl)methyl)amino)methyl)-1H-pyrrolo[3,2-b]pyridin-6-yl)-[4,4'-bipyridin]-2-yl)-2-methoxybenzyl)amino)methyl)pyrrolidin-2-one;
(S)-5-(((4-(3,3'-dichloro-2'-(1-methyl-3-(((((R)-5-oxopyrrolidin-2-yl)methyl)amino)methyl)-1H-pyrrolo[3,2-b]pyridin-6-yl)-[4,4'-bipyridin]-2-yl)-2-methoxybenzyl)amino)methyl)pyrrolidin-2-one;
(R)-5-(((4-(3,3'-dichloro-2'-(1-methyl-3-(((((S)-5-oxopyrrolidin-2-yl)methyl)amino)methyl)-1H-pyrrolo[3,2-b]pyridin-6-yl)-[4,4'-bipyridin]-2-yl)-2-methoxybenzyl)amino)methyl)pyrrolidin-2-one;
(R)-5-(((4-(3,3'-dichloro-2'-(1-methyl-3-(((((R)-5-oxopyrrolidin-2-yl)methyl)amino)methyl)-1H-pyrrolo[3,2-b]pyridin-6-yl)-[4,4'-bipyridin]-2-yl)-2-methoxybenzyl)amino)methyl)pyrrolidin-2-one;
(S)-5-((((6-(2-chloro-3-(3-chloro-2-(3-methoxy-4-(((((S)-5-oxopyrrolidin-2-yl)methyl)amino)methyl)phenyl)pyridin-4-yl)phenyl)-1-methyl-1H-pyrrolo[2,3-b]pyridin-3-yl)methyl)amino)methyl)pyrrolidin-2-one;
(S)-5-((((6-(2-chloro-3-(3-chloro-2-(3-methoxy-4-(((((R)-5-oxopyrrolidin-2-yl)methyl)amino)methyl)phenyl)pyridin-4-yl)phenyl)-1-methyl-1H-pyrrolo[2,3-b]pyridin-3-yl)methyl)amino)methyl)pyrrolidin-2-one;
(R)-5-((((6-(2-chloro-3-(3-chloro-2-(3-methoxy-4-(((((S)-5-oxopyrrolidin-2-yl)methyl)amino)methyl)phenyl)pyridin-4-yl)phenyl)-1-methyl-1H-pyrrolo[2,3-b]pyridin-3-yl)methyl)amino)methyl)pyrrolidin-2-one;
(R)-5-((((6-(2-chloro-3-(3-chloro-2-(3-methoxy-4-(((((R)-5-oxopyrrolidin-2-yl)methyl)amino)methyl)phenyl)pyridin-4-yl)phenyl)-1-methyl-1H-pyrrolo[2,3-b]pyridin-3-yl)methyl)amino)methyl)pyrrolidin-2-one;
(S)-5-((6-(2-chloro-3-(3-chloro-2-(3-methoxy-4-(((((S)-5-oxopyrrolidin-2-yl)methyl)amino)methyl)phenyl)pyridin-4-yl)phenyl)-8-methoxy-3,4-dihydroisoquinolin-2(1H)-yl)methyl)pyrrolidin-2-one;
(S)-5-((6-(2-chloro-3-(3-chloro-2-(3-methoxy-4-(((((R)-5-oxopyrrolidin-2-yl)methyl)amino)methyl)phenyl)pyridin-4-yl)phenyl)-8-methoxy-3,4-dihydroisoquinolin-2(1H)-yl)methyl)pyrrolidin-2-one;
(R)-5-((6-(2-chloro-3-(3-chloro-2-(3-methoxy-4-(((((S)-5-oxopyrrolidin-2-yl)methyl)amino)methyl)phenyl)pyridin-4-yl)phenyl)-8-methoxy-3,4-dihydroisoquinolin-2(1H)-yl)methyl)pyrrolidin-2-one;
(R)-5-((6-(2-chloro-3-(3-chloro-2-(3-methoxy-4-(((((R)-5-oxopyrrolidin-2-yl)methyl)amino)methyl)phenyl)pyridin-4-yl)phenyl)-8-methoxy-3,4-dihydroisoquinolin-2(1H)-yl)methyl)pyrrolidin-2-one;
(S)-5-((((6-(2-chloro-3-(3-chloro-2-(1-methyl-4-(((S)-5-oxopyrrolidin-2-yl)methyl)-2,3,4,5-tetrahydro-1H-benzo[e][1,4]diazepin-8-yl)pyridin-4-yl)phenyl)-2-methoxypyridin-3-yl)methyl)amino)methyl)pyrrolidin-2-one;
(S)-5-((((6-(2-chloro-3-(3-chloro-2-(1-methyl-4-(((R)-5-oxopyrrolidin-2-yl)methyl)-2,3,4,5-tetrahydro-1H-benzo[e][1,4]diazepin-8-yl)pyridin-4-yl)phenyl)-2-methoxypyridin-3-yl)methyl)amino)methyl)pyrrolidin-2-one;
(R)-5-((((6-(2-chloro-3-(3-chloro-2-(1-methyl-4-(((S)-5-oxopyrrolidin-2-yl)methyl)-2,3,4,5-tetrahydro-1H-benzo[e][1,4]diazepin-8-yl)pyridin-4-yl)phenyl)-2-methoxypyridin-3-yl)methyl)amino)methyl)pyrrolidin-2-one;
(R)-5-((((6-(2-chloro-3-(3-chloro-2-(1-methyl-4-(((R)-5-oxopyrrolidin-2-yl)methyl)-2,3,4,5-tetrahydro-1H-benzo[e][1,4]diazepin-8-yl)pyridin-4-yl)phenyl)-2-methoxypyridin-3-yl)methyl)amino)methyl)pyrrolidin-2-one;
(S)-5-((((5-(2-chloro-3-(3-chloro-2-(3-methoxy-4-(((((S)-5-oxopyrrolidin-2-yl)methyl)amino)methyl)phenyl)pyridin-4-yl)phenyl)-3-methoxypyrazin-2-yl)methyl)amino)methyl)pyrrolidin-2-one;
(S)-5-((((5-(2-chloro-3-(3-chloro-2-(3-methoxy-4-(((((R)-5-oxopyrrolidin-2-yl)methyl)amino)methyl)phenyl)pyridin-4-yl)phenyl)-3-methoxypyrazin-2-yl)methyl)amino)methyl)pyrrolidin-2-one;
(R)-5-((((5-(2-chloro-3-(3-chloro-2-(3-methoxy-4-(((((S)-5-oxopyrrolidin-2-yl)methyl)amino)methyl)phenyl)pyridin-4-yl)phenyl)-3-methoxypyrazin-2-yl)methyl)amino)methyl)pyrrolidin-2-one;
(R)-5-((((5-(2-chloro-3-(3-chloro-2-(3-methoxy-4-(((((R)-5-oxopyrrolidin-2-yl)methyl)amino)methyl)phenyl)pyridin-4-yl)phenyl)-3-methoxypyrazin-2-yl)methyl)amino)methyl)pyrrolidin-2-one;
(S)-5-((((6-(3-(2-(3-(((1-acetylpiperidin-4-yl)amino)methyl)-1-methyl-1H-pyrrolo[3,2-b]pyridin-6-yl)-3-chloropyridin-4-yl)-2-chlorophenyl)-2-methoxypyridin-3-yl)methyl)amino)methyl)pyrrolidin-2-one;
(R)-5-((((6-(3-(2-(3-(((1-acetylpiperidin-4-yl)amino)methyl)-1-methyl-1H-pyrrolo[3,2-b]pyridin-6-yl)-3-chloropyridin-4-yl)-2-chlorophenyl)-2-methoxypyridin-3-yl)methyl)amino)methyl)pyrrolidin-2-one;
(S)-5-((((6-(2-chloro-3-(3-chloro-2-(1-methyl-3-(((((S)-oxetan-2-yl)methyl)amino)methyl)-1H-pyrrolo[3,2-b]pyridin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(1-methyl-3-(((((R)-oxetan-2-yl)methyl)amino)methyl)-1H-pyrrolo[3,2-b]pyridin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(1-methyl-3-(((((S)-oxetan-2-yl)methyl)amino)methyl)-1H-pyrrolo[3,2-b]pyridin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(1-methyl-3-(((((R)-oxetan-2-yl)methyl)amino)methyl)-1H-pyrrolo[3,2-b]pyridin-6-yl)pyridin-4-yl)phenyl)-2-methoxypyridin-3-yl)methyl)amino)methyl)pyrrolidin-2-one;
(S)-5-((((6-(2-chloro-3-(3-chloro-5'-methoxy-6'-(((((S)-5-oxopyrrolidin-2-yl)methyl)amino)methyl)-[2,3'-bipyridin]-4-yl)phenyl)-2-methoxypyridin-3-yl)methyl)amino)methyl)pyrrolidin-2-one;
(S)-5-((((6-(2-chloro-3-(3-chloro-5'-methoxy-6'-(((((R)-5-oxopyrrolidin-2-yl)methyl)amino)methyl)-[2,3'-bipyridin]-4-yl)phenyl)-2-methoxypyridin-3-yl)methyl)amino)methyl)pyrrolidin-2-one;
(R)-5-((((6-(2-chloro-3-(3-chloro-5'-methoxy-6'-(((((S)-5-oxopyrrolidin-2-yl)methyl)amino)methyl)-[2,3'-bipyridin]-4-yl)phenyl)-2-methoxypyridin-3-yl)methyl)amino)methyl)pyrrolidin-2-one;
(R)-5-((((6-(2-chloro-3-(3-chloro-5'-methoxy-6'-(((((R)-5-oxopyrrolidin-2-yl)methyl)amino)methyl)-[2,3'-bipyridin]-4-yl)phenyl)-2-methoxypyridin-3-yl)methyl)amino)methyl)pyrrolidin-2-one;
(S)-5-((((6-(2-chloro-3-(3-chloro-2-(4-((S)-2-hydroxypropyl)-1-methyl-2,3,4,5-tetrahydro-1H-benzo[e][1,4]diazepin-8-yl)pyridin-4-yl)phenyl)-2-methoxypyridin-3-yl)methyl)amino)methyl)pyrrolidin-2-one;
(S)-5-((((6-(2-chloro-3-(3-chloro-2-(4-((R)-2-hydroxypropyl)-1-methyl-2,3,4,5-tetrahydro-1H-benzo[e][1,4]diazepin-8-yl)pyridin-4-yl)phenyl)-2-methoxypyridin-3-yl)methyl)amino)methyl)pyrrolidin-2-one;
(R)-5-((((6-(2-chloro-3-(3-chloro-2-(4-((S)-2-hydroxypropyl)-1-methyl-2,3,4,5-tetrahydro-1H-benzo[e][1,4]diazepin-8-yl)pyridin-4-yl)phenyl)-2-methoxypyridin-3-yl)methyl)amino)methyl)pyrrolidin-2-one;
(R)-5-((((6-(2-chloro-3-(3-chloro-2-(4-((R)-2-hydroxypropyl)-1-methyl-2,3,4,5-tetrahydro-1H-benzo[e][1,4]diazepin-8-yl)pyridin-4-yl)phenyl)-2-methoxypyridin-3-yl)methyl)amino)methyl)pyrrolidin-2-one;
(S)-5-((((6-(2-chloro-3-(3-chloro-2-(4-((S)-2-hydroxypropyl)-2,3,4,5-tetrahydrobenzo[f][1,4]oxazepin-8-yl)pyridin-4-yl)phenyl)-2-methoxypyridin-3-yl)methyl)amino)methyl)pyrrolidin-2-one;
(S)-5-((((6-(2-chloro-3-(3-chloro-2-(4-((R)-2-hydroxypropyl)-2,3,4,5-tetrahydrobenzo[f][1,4]oxazepin-8-yl)pyridin-4-yl)phenyl)-2-methoxypyridin-3-yl)methyl)amino)methyl)pyrrolidin-2-one;
(R)-5-((((6-(2-chloro-3-(3-chloro-2-(4-((S)-2-hydroxypropyl)-2,3,4,5-tetrahydrobenzo[f][1,4]oxazepin-8-yl)pyridin-4-yl)phenyl)-2-methoxypyridin-3-yl)methyl)amino)methyl)pyrrolidin-2-one;
(R)-5-((((6-(2-chloro-3-(3-chloro-2-(4-((R)-2-hydroxypropyl)-2,3,4,5-tetrahydrobenzo[f][1,4]oxazepin-8-yl)pyridin-4-yl)phenyl)-2-methoxypyridin-3-yl)methyl)amino)methyl)pyrrolidin-2-one;
(S)-5-((((6-(2-chloro-3-(3-chloro-2-(2,3,4,5-tetrahydrobenzo[f][1,4]oxazepin-8-yl)pyridin-4-yl)phenyl)-2-methoxypyridin-3-yl)methyl)amino)methyl)pyrrolidin-2-one;
(R)-5-((((6-(2-chloro-3-(3-chloro-2-(2,3,4,5-tetrahydrobenzo[f][1,4]oxazepin-8-yl)pyridin-4-yl)phenyl)-2-methoxypyridin-3-yl)methyl)amino)methyl)pyrrolidin-2-one;
(S)-5-((((6-(2-chloro-3-(3-chloro-2-(2-((R)-2-hydroxypropyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(2-((S)-2-hydroxypropyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-((R)-2-hydroxypropyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-((S)-2-hydroxypropyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(S)-5-((((6-(3-(2-(3-(((1-acetylpiperidin-4-yl)amino)methyl)-1-methyl-1H-indol-6-yl)-3-chloropyridin-4-yl)-2-chlorophenyl)-2-methoxypyridin-3-yl)methyl)amino)methyl)pyrrolidin-2-one;
(R)-5-((((6-(3-(2-(3-(((1-acetylpiperidin-4-yl)amino)methyl)-1-methyl-1H-indol-6-yl)-3-chloropyridin-4-yl)-2-chlorophenyl)-2-methoxypyridin-3-yl)methyl)amino)methyl)pyrrolidin-2-one;
(S)-5-((((6-(2-chloro-3-(3-chloro-2-(3-(((2-hydroxyethyl)amino)methyl)-1-methyl-1H-indol-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(3-(((2-hydroxyethyl)amino)methyl)-1-methyl-1H-indol-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(3-(((2-methoxyethyl)amino)methyl)-1-methyl-1H-indol-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(3-(((2-methoxyethyl)amino)methyl)-1-methyl-1H-indol-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(1-methyl-3-((methylamino)methyl)-1H-indol-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(1-methyl-3-((methylamino)methyl)-1H-indol-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(3-((ethylamino)methyl)-1-methyl-1H-indol-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(3-((ethylamino)methyl)-1-methyl-1H-indol-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(3-((dimethylamino)methyl)-1-methyl-1H-indol-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(3-((dimethylamino)methyl)-1-methyl-1H-indol-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(1-methyl-3-(((((S)-oxetan-2-yl)methyl)amino)methyl)-1H-indol-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(1-methyl-3-(((((R)-oxetan-2-yl)methyl)amino)methyl)-1H-indol-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(1-methyl-3-(((((S)-oxetan-2-yl)methyl)amino)methyl)-1H-indol-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(1-methyl-3-(((((R)-oxetan-2-yl)methyl)amino)methyl)-1H-indol-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(3-((((S)-2-hydroxypropyl)amino)methyl)-1-methyl-1H-indol-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(3-((((R)-2-hydroxypropyl)amino)methyl)-1-methyl-1H-indol-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(3-((((S)-2-hydroxypropyl)amino)methyl)-1-methyl-1H-indol-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(3-((((R)-2-hydroxypropyl)amino)methyl)-1-methyl-1H-indol-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(3-(((2-hydroxy-2-methylpropyl)amino)methyl)-1-methyl-1H-indol-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(3-(((2-hydroxy-2-methylpropyl)amino)methyl)-1-methyl-1H-indol-6-yl)pyridin-4-yl)phenyl)-2-methoxypyridin-3-yl)methyl)amino)methyl)pyrrolidin-2-one;
(S)-5-((6-(3-chloro-4-(2-chloro-3-(4-chloro-6-methoxy-5-(((((S)-5-oxopyrrolidin-2-yl)methyl)amino)methyl)pyridin-2-yl)phenyl)pyridin-2-yl)-8-methoxy-3,4-dihydroisoquinolin-2(1H)-yl)methyl)pyrrolidin-2-one;
(S)-5-((6-(3-chloro-4-(2-chloro-3-(4-chloro-6-methoxy-5-(((((R)-5-oxopyrrolidin-2-yl)methyl)amino)methyl)pyridin-2-yl)phenyl)pyridin-2-yl)-8-methoxy-3,4-dihydroisoquinolin-2(1H)-yl)methyl)pyrrolidin-2-one;
(R)-5-((6-(3-chloro-4-(2-chloro-3-(4-chloro-6-methoxy-5-(((((S)-5-oxopyrrolidin-2-yl)methyl)amino)methyl)pyridin-2-yl)phenyl)pyridin-2-yl)-8-methoxy-3,4-dihydroisoquinolin-2(1H)-yl)methyl)pyrrolidin-2-one;
(R)-5-((6-(3-chloro-4-(2-chloro-3-(4-chloro-6-methoxy-5-(((((R)-5-oxopyrrolidin-2-yl)methyl)amino)methyl)pyridin-2-yl)phenyl)pyridin-2-yl)-8-methoxy-3,4-dihydroisoquinolin-2(1H)-yl)methyl)pyrrolidin-2-one;
(S)-5-((((6-(2-chloro-3-(3-chloro-2-(3-((isobutylamino)methyl)-1-methyl-1H-indol-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(3-((isobutylamino)methyl)-1-methyl-1H-indol-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(3-(((2-hydroxyethyl)(methyl)amino)methyl)-1-methyl-1H-indol-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(3-(((2-hydroxyethyl)(methyl)amino)methyl)-1-methyl-1H-indol-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(3-((((S)-2-methoxypropyl)amino)methyl)-1-methyl-1H-indol-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(3-((((R)-2-methoxypropyl)amino)methyl)-1-methyl-1H-indol-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(3-((((S)-2-methoxypropyl)amino)methyl)-1-methyl-1H-indol-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(3-((((R)-2-methoxypropyl)amino)methyl)-1-methyl-1H-indol-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(1-methyl-3-(((((S)-tetrahydrofuran-2-yl)methyl)amino)methyl)-1H-indol-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(1-methyl-3-(((((R)-tetrahydrofuran-2-yl)methyl)amino)methyl)-1H-indol-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(1-methyl-3-(((((S)-tetrahydrofuran-2-yl)methyl)amino)methyl)-1H-indol-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(1-methyl-3-(((((R)-tetrahydrofuran-2-yl)methyl)amino)methyl)-1H-indol-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(1-methyl-3-(((tetrahydro-2H-pyran-4-yl)amino)methyl)-1H-indol-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(1-methyl-3-(((tetrahydro-2H-pyran-4-yl)amino)methyl)-1H-indol-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(3-(((1,1-dioxidotetrahydro-2H-thiopyran-4-yl)amino)methyl)- 1-methyl-1H-indol-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(3-(((1,1-dioxidotetrahydro-2H-thiopyran-4-yl)amino)methyl)- 1-methyl-1H-indol-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(2-((S)-2-hydroxypropyl)-5-methoxy-1,2,3,4-tetrahydroisoquinolin-7-yl)pyridin-4-yl)phenyl)-2-methoxypyridin-3-yl)methyl)amino)methyl)pyrrolidin-2-one;
(S)-5-((((6-(2-chloro-3-(3-chloro-2-(2-((R)-2-hydroxypropyl)-5-methoxy-1,2,3,4-tetrahydroisoquinolin-7-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-((S)-2-hydroxypropyl)-5-methoxy-1,2,3,4-tetrahydroisoquinolin-7-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-((R)-2-hydroxypropyl)-5-methoxy-1,2,3,4-tetrahydroisoquinolin-7-yl)pyridin-4-yl)phenyl)-2-methoxypyridin-3-yl)methyl)amino)methyl)pyrrolidin-2-one;
(S)-5-((((6-(2-chloro-3-(3-chloro-2-(2-(2-hydroxy-2-methylpropyl)-5-methoxy-1,2,3,4-tetrahydroisoquinolin-7-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-(2-hydroxy-2-methylpropyl)-5-methoxy-1,2,3,4-tetrahydroisoquinolin-7-yl)pyridin-4-yl)phenyl)-2-methoxypyridin-3-yl)methyl)amino)methyl)pyrrolidin-2-one;
(S)-5-((((6-(2-chloro-3-(3-chloro-2-(8-fluoro-2-(2-hydroxy-2-methylpropyl)-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(8-fluoro-2-(2-hydroxy-2-methylpropyl)-1,2,3,4-tetrahydroisoquinolin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(5-fluoro-2-(2-hydroxy-2-methylpropyl)-1,2,3,4-tetrahydroisoquinolin-7-yl)pyridin-4-yl)phenyl)-2-methoxypyridin-3-yl)methyl)amino)methyl)pyrrolidin-2-one;
(R)-5-((((6-(2-chloro-3-(3-chloro-2-(5-fluoro-2-(2-hydroxy-2-methylpropyl)-1,2,3,4-tetrahydroisoquinolin-7-yl)pyridin-4-yl)phenyl)-2-methoxypyridin-3-yl)methyl)amino)methyl)pyrrolidin-2-one;
(S)-6-(3-chloro-4-(2-chloro-3-(6-methoxy-5-((((5-oxopyrrolidin-2-yl)methyl)amino)methyl)pyridin-2-yl)phenyl)pyridin-2-yl)-2-((6-hydroxy-2-azaspiro[3.3]heptan-2-yl)methyl)-3-methylpyrrolo[2,1-f][1,2,4]triazin-4(3H)-one;
(R)-6-(3-chloro-4-(2-chloro-3-(6-methoxy-5-((((5-oxopyrrolidin-2-yl)methyl)amino)methyl)pyridin-2-yl)phenyl)pyridin-2-yl)-2-((6-hydroxy-2-azaspiro[3.3]heptan-2-yl)methyl)-3-methylpyrrolo[2,1-f][1,2,4]triazin-4(3H)-one;
(S)-5-((((6-(2-chloro-3-(3-chloro-2-(2-(3-hydroxy-3-methylbutyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-(3-hydroxy-3-methylbutyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(2-((S)-2-hydroxypropyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(2-((R)-2-hydroxypropyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-((S)-2-hydroxypropyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-((R)-2-hydroxypropyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(2-(2-(1-hydroxycyclobutyl)ethyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-(2-(1-hydroxycyclobutyl)ethyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(7-(((((S)-5-oxopyrrolidin-2-yl)methyl)amino)methyl)quinolin-3-yl)pyridin-4-yl)phenyl)-2-methoxypyridin-3-yl)methyl)amino)methyl)pyrrolidin-2-one;
(S)-5-((((6-(2-chloro-3-(3-chloro-2-(7-(((((R)-5-oxopyrrolidin-2-yl)methyl)amino)methyl)quinolin-3-yl)pyridin-4-yl)phenyl)-2-methoxypyridin-3-yl)methyl)amino)methyl)pyrrolidin-2-one;
(R)-5-((((6-(2-chloro-3-(3-chloro-2-(7-(((((S)-5-oxopyrrolidin-2-yl)methyl)amino)methyl)quinolin-3-yl)pyridin-4-yl)phenyl)-2-methoxypyridin-3-yl)methyl)amino)methyl)pyrrolidin-2-one;
(R)-5-((((6-(2-chloro-3-(3-chloro-2-(7-(((((R)-5-oxopyrrolidin-2-yl)methyl)amino)methyl)quinolin-3-yl)pyridin-4-yl)phenyl)-2-methoxypyridin-3-yl)methyl)amino)methyl)pyrrolidin-2-one;
(5S)-5-((((6-(2-chloro-3-(3-chloro-2-(2-(2-hydroxy-3-methylbutyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(SR)-5-((((6-(2-chloro-3-(3-chloro-2-(2-(2-hydroxy-3-methylbutyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(2-(2-hydroxyethyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-(2-hydroxyethyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(S)-5-((((6-(3-(2-(3-(((1-acetylazetidin-3-yl)amino)methyl)-1-methyl-1H-indol-6-yl)-3-chloropyridin-4-yl)-2-chlorophenyl)-2-methoxypyridin-3-yl)methyl)amino)methyl)pyrrolidin-2-one;
(R)-5-((((6-(3-(2-(3-(((1-acetylazetidin-3-yl)amino)methyl)-1-methyl-1H-indol-6-yl)-3-chloropyridin-4-yl)-2-chlorophenyl)-2-methoxypyridin-3-yl)methyl)amino)methyl)pyrrolidin-2-one;
(S)-5-((((6-(3-(2-(3-(((1-acetylpiperidin-4-yl)(methyl)amino)methyl)-1-methyl-1H-indol-6-yl)-3-chloropyridin-4-yl)-2-chlorophenyl)-2-methoxypyridin-3-yl)methyl)amino)methyl)pyrrolidin-2-one;
(R)-5-((((6-(3-(2-(3-(((1-acetylpiperidin-4-yl)(methyl)amino)methyl)-1-methyl-1H-indol-6-yl)-3-chloropyridin-4-yl)-2-chlorophenyl)-2-methoxypyridin-3-yl)methyl)amino)methyl)pyrrolidin-2-one;
(S)-5-(((4-(4-(3-(3-(((1-acetylpiperidin-4-yl)amino)methyl)-1-methyl-1H-pyrrolo[2,3-b]pyridin-6-yl)-2-chlorophenyl)-3-chloropyridin-2-yl)-2-methoxybenzyl)amino)methyl)pyrrolidin-2-one;
(R)-5-(((4-(4-(3-(3-(((1-acetylpiperidin-4-yl)amino)methyl)-1-methyl-1H-pyrrolo[2,3-b]pyridin-6-yl)-2-chlorophenyl)-3-chloropyridin-2-yl)-2-methoxybenzyl)amino)methyl)pyrrolidin-2-one;
(S)-5-(((4-(3-chloro-4-(2-chloro-3-(1-methyl-3-(((((S)-oxetan-2-yl)methyl)amino)methyl)-1H-pyrrolo[2,3-b]pyridin-6-yl)phenyl)pyridin-2-yl)-2-methoxybenzyl)amino)methyl)pyrrolidin-2-one;
(S)-5-(((4-(3-chloro-4-(2-chloro-3-(1-methyl-3-(((((R)-oxetan-2-yl)methyl)amino)methyl)-1H-pyrrolo[2,3-b]pyridin-6-yl)phenyl)pyridin-2-yl)-2-methoxybenzyl)amino)methyl)pyrrolidin-2-one;
(R)-5-(((4-(3-chloro-4-(2-chloro-3-(1-methyl-3-(((((S)-oxetan-2-yl)methyl)amino)methyl)-1H-pyrrolo[2,3-b]pyridin-6-yl)phenyl)pyridin-2-yl)-2-methoxybenzyl)amino)methyl)pyrrolidin-2-one;
(R)-5-(((4-(3-chloro-4-(2-chloro-3-(1-methyl-3-(((((R)-oxetan-2-yl)methyl)amino)methyl)-1H-pyrrolo[2,3-b]pyridin-6-yl)phenyl)pyridin-2-yl)-2-methoxybenzyl)amino)methyl)pyrrolidin-2-one;
(S)-5-(((4-(3-chloro-4-(2-chloro-3-(1-methyl-3-(((tetrahydro-2H-pyran-4-yl)amino)methyl)-1H-pyrrolo[2,3-b]pyridin-6-yl)phenyl)pyridin-2-yl)-2-methoxybenzyl)amino)methyl)pyrrolidin-2-one;
(R)-5-(((4-(3-chloro-4-(2-chloro-3-(1-methyl-3-(((tetrahydro-2H-pyran-4-yl)amino)methyl)-1H-pyrrolo[2,3-b]pyridin-6-yl)phenyl)pyridin-2-yl)-2-methoxybenzyl)amino)methyl)pyrrolidin-2-one;
(S)-5-(((4-(3-chloro-4-(2-chloro-3-(3-(((1,1-dioxidotetrahydro-2H-thiopyran-4-yl)amino)methyl)-1-methyl-1H-pyrrolo [2,3-b]pyridin-6-yl)phenyl)pyridin-2-yl)-2-methoxybenzyl)amino)methyl)pyrrolidin-2-one;
(R)-5-(((4-(3-chloro-4-(2-chloro-3-(3-(((1,1-dioxidotetrahydro-2H-thiopyran-4-yl)amino)methyl)-1-methyl-1H-pyrrolo[2,3-b]pyridin-6-yl)phenyl)pyridin-2-yl)-2-methoxybenzyl)amino)methyl)pyrrolidin-2-one;
(S)-5-((((6-(2-chloro-3-(3-chloro-2-(8-fluoro-2-(((S)-5-oxopyrrolidin-2-yl)methyl)-1,2,3,4-tetrahydroisoquinolin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(8-fluoro-2-(((R)-5-oxopyrrolidin-2-yl)methyl)-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(8-fluoro-2-(((S)-5-oxopyrrolidin-2-yl)methyl)-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(8-fluoro-2-(((R)-5-oxopyrrolidin-2-yl)methyl)-1,2,3,4-tetrahydroisoquinolin-6-yl)pyridin-4-yl)phenyl)-2-methoxypyridin-3-yl)methyl)amino)methyl)pyrrolidin-2-one;
(S)-5-((((6-(3-(2-(2-((1-acetylpiperidin-4-yl)methyl)-8-methoxy-1,2,3,4-tetrahydroisoquinolin-6-yl)-3-chloropyridin-4-yl)-2-chlorophenyl)-2-methoxypyridin-3-yl)methyl)amino)methyl)pyrrolidin-2-one;
(R)-5-((((6-(3-(2-(2-((1-acetylpiperidin-4-yl)methyl)-8-methoxy-1,2,3,4-tetrahydroisoquinolin-6-yl)-3-chloropyridin-4-yl)-2-chlorophenyl)-2-methoxypyridin-3-yl)methyl)amino)methyl)pyrrolidin-2-one;
(S)-5-((((6-(2-chloro-3-(3-chloro-2-(2-(3-fluoropropyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-(3-fluoropropyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(8-methoxy-2-(2-(piperidin-4-yl)ethyl)-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(8-methoxy-2-(2-(piperidin-4-yl)ethyl)-1,2,3,4-tetrahydroisoquinolin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(8-fluoro-2-(3-fluoropropyl)-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(8-fluoro-2-(3-fluoropropyl)-1,2,3,4-tetrahydroisoquinolin-6-yl)pyridin-4-yl)phenyl)-2-methoxypyridin-3-yl)methyl)amino)methyl)pyrrolidin-2-one;
5-(3-chloro-4-(2-chloro-3-(6-methoxy-5-(((((S)-5-oxopyrrolidin-2-yl)methyl)amino)methyl)pyridin-2-yl)phenyl)pyridin-2-yl)-2-(2-((((S)-5-oxopyrrolidin-2-yl)methyl)amino)ethyl)isoindolin-1-one;
5-(3-chloro-4-(2-chloro-3-(6-methoxy-5-(((((S)-5-oxopyrrolidin-2-yl)methyl)amino)methyl)pyridin-2-yl)phenyl)pyridin-2-yl)-2-(2-((((R)-5-oxopyrrolidin-2-yl)methyl)amino)ethyl)isoindolin-1-one;
5-(3-chloro-4-(2-chloro-3-(6-methoxy-5-(((((R)-5-oxopyrrolidin-2-yl)methyl)amino)methyl)pyridin-2-yl)phenyl)pyridin-2-yl)-2-(2-((((S)-5-oxopyrrolidin-2-yl)methyl)amino)ethyl)isoindolin-1-one;
5-(3-chloro-4-(2-chloro-3-(6-methoxy-5-(((((R)-5-oxopyrrolidin-2-yl)methyl)amino)methyl)pyridin-2-yl)phenyl)pyridin-2-yl)-2-(2-((((R)-5-oxopyrrolidin-2-yl)methyl)amino)ethyl)isoindolin-1-one;
(S)-5-((((6-(2-chloro-3-(3-chloro-2-(8-chloro-2-(((S)-5-oxopyrrolidin-2-yl)methyl)-1,2,3,4-tetrahydroisoquinolin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(8-chloro-2-(((R)-5-oxopyrrolidin-2-yl)methyl)-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(8-chloro-2-(((S)-5-oxopyrrolidin-2-yl)methyl)-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(8-chloro-2-(((R)-5-oxopyrrolidin-2-yl)methyl)-1,2,3,4-tetrahydroisoquinolin-6-yl)pyridin-4-yl)phenyl)-2-methoxypyridin-3-yl)methyl)amino)methyl)pyrrolidin-2-one;
isopropyl (4-(3-chloro-4-(2-chloro-3-(6-methoxy-5-(((((S)-5-oxopyrrolidin-2-yl)methyl)amino)methyl)pyridin-2-yl)phenyl)pyridin-2-yl)-2-methoxybenzyl)-L-prolinate;
isopropyl (4-(3-chloro-4-(2-chloro-3-(6-methoxy-5-(((((S)-5-oxopyrrolidin-2-yl)methyl)amino)methyl)pyridin-2-yl)phenyl)pyridin-2-yl)-2-methoxybenzyl)-D-prolinate;
isopropyl (4-(3-chloro-4-(2-chloro-3-(6-methoxy-5-(((((R)-5-oxopyrrolidin-2-yl)methyl)amino)methyl)pyridin-2-yl)phenyl)pyridin-2-yl)-2-methoxybenzyl)-L-prolinate;
isopropyl (4-(3-chloro-4-(2-chloro-3-(6-methoxy-5-(((((R)-5-oxopyrrolidin-2-yl)methyl)amino)methyl)pyridin-2-yl)phenyl)pyridin-2-yl)-2-methoxybenzyl)-D-prolinate;
(S)-5-((((6-(2-chloro-3-(3-chloro-2-(8-chloro-2-((S)-2-hydroxypropyl)-1,2,3,4-tetrahydroisoquinolin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(8-chloro-2-((R)-2-hydroxypropyl)-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(8-chloro-2-((S)-2-hydroxypropyl)-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(8-chloro-2-((R)-2-hydroxypropyl)-1,2,3,4-tetrahydroisoquinolin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(4-(((2-hydroxyethyl)amino)methyl)-3-methoxyphenyl)pyridin-4-yl)phenyl)-2-methoxypyridin-3-yl)methyl)amino)methyl)pyrrolidin-2-one;
(R)-5-((((6-(2-chloro-3-(3-chloro-2-(4-(((2-hydroxyethyl)amino)methyl)-3-methoxyphenyl)pyridin-4-yl)phenyl)-2-methoxypyridin-3-yl)methyl)amino)methyl)pyrrolidin-2-one;
(S)-5-((((4-chloro-6-(2-chloro-3-(3-chloro-2-(4-((((S)-2-hydroxypropyl)(methyl)amino)methyl)-3-methoxyphenyl)pyridin-4-yl)phenyl)-2-methoxypyridin-3-yl)methyl)amino)methyl)pyrrolidin-2-one;
(S)-5-((((4-chloro-6-(2-chloro-3-(3-chloro-2-(4-((((R)-2-hydroxypropyl)(methyl)amino)methyl)-3-methoxyphenyl)pyridin-4-yl)phenyl)-2-methoxypyridin-3-yl)methyl)amino)methyl)pyrrolidin-2-one;
(R)-5-((((4-chloro-6-(2-chloro-3-(3-chloro-2-(4-((((S)-2-hydroxypropyl)(methyl)amino)methyl)-3-methoxyphenyl)pyridin-4-yl)phenyl)-2-methoxypyridin-3-yl)methyl)amino)methyl)pyrrolidin-2-one;
(R)-5-((((4-chloro-6-(2-chloro-3-(3-chloro-2-(4-((((R)-2-hydroxypropyl)(methyl)amino)methyl)-3-methoxyphenyl)pyridin-4-yl)phenyl)-2-methoxypyridin-3-yl)methyl)amino)methyl)pyrrolidin-2-one;
(S)-5-((((6-(2-chloro-3-(3-chloro-2-(8-chloro-2-(3-fluoropropyl)-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(8-chloro-2-(3-fluoropropyl)-1,2,3,4-tetrahydroisoquinolin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(3-(((4,4-difluorocyclohexyl)amino)methyl)-1-methyl-1H-indol-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(3-(((4,4-difluorocyclohexyl)amino)methyl)-1-methyl-1H-indol-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(2-(2-(1-hydroxycyclopropyl)ethyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-(2-(1-hydroxycyclopropyl)ethyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(4-((((S)-2-hydroxypropyl)(methyl)amino)methyl)-3-methoxyphenyl)pyridin-4-yl)phenyl)-2-methoxypyridin-3-yl)methyl)amino)methyl)pyrrolidin-2-one;
(S)-5-((((6-(2-chloro-3-(3-chloro-2-(4-((((R)-2-hydroxypropyl)(methyl)amino)methyl)-3-methoxyphenyl)pyridin-4-yl)phenyl)-2-methoxypyridin-3-yl)methyl)amino)methyl)pyrrolidin-2-one;
(R)-5-((((6-(2-chloro-3-(3-chloro-2-(4-((((S)-2-hydroxypropyl)(methyl)amino)methyl)-3-methoxyphenyl)pyridin-4-yl)phenyl)-2-methoxypyridin-3-yl)methyl)amino)methyl)pyrrolidin-2-one;
(R)-5-((((6-(2-chloro-3-(3-chloro-2-(4-((((R)-2-hydroxypropyl)(methyl)amino)methyl)-3-methoxyphenyl)pyridin-4-yl)phenyl)-2-methoxypyridin-3-yl)methyl)amino)methyl)pyrrolidin-2-one;
isopropyl (S)-1-(4-(3-chloro-4-(2-chloro-3-(6-methoxy-5-(((((S)-5-oxopyrrolidin-2-yl)methyl)amino)methyl)pyridin-2-yl)phenyl)pyridin-2-yl)-2-methoxybenzyl)pyrrolidine-3-carboxylate;
isopropyl (S)-1-(4-(3-chloro-4-(2-chloro-3-(6-methoxy-5-(((((R)-5-oxopyrrolidin-2-yl)methyl)amino)methyl)pyridin-2-yl)phenyl)pyridin-2-yl)-2-methoxybenzyl)pyrrolidine-3-carboxylate;
isopropyl (R)-1-(4-(3-chloro-4-(2-chloro-3-(6-methoxy-5-(((((S)-5-oxopyrrolidin-2-yl)methyl)amino)methyl)pyridin-2-yl)phenyl)pyridin-2-yl)-2-methoxybenzyl)pyrrolidine-3-carboxylate;
isopropyl (R)-1-(4-(3-chloro-4-(2-chloro-3-(6-methoxy-5-(((((R)-5-oxopyrrolidin-2-yl)methyl)amino)methyl)pyridin-2-yl)phenyl)pyridin-2-yl)-2-methoxybenzyl)pyrrolidine-3-carboxylate;
(S)-1-(4-(3-chloro-4-(2-chloro-3-(6-methoxy-5-(((((S)-5-oxopyrrolidin-2-yl)methyl)amino)methyl)pyridin-2-yl)phenyl)pyridin-2-yl)-2-methoxybenzyl)pyrrolidine-3-carboxylic acid;
(S)-1-(4-(3-chloro-4-(2-chloro-3-(6-methoxy-5-(((((R)-5-oxopyrrolidin-2-yl)methyl)amino)methyl)pyridin-2-yl)phenyl)pyridin-2-yl)-2-methoxybenzyl)pyrrolidine-3-carboxylic acid;
(R)-1-(4-(3-chloro-4-(2-chloro-3-(6-methoxy-5-(((((S)-5-oxopyrrolidin-2-yl)methyl)amino)methyl)pyridin-2-yl)phenyl)pyridin-2-yl)-2-methoxybenzyl)pyrrolidine-3-carboxylic acid;
(R)-1-(4-(3-chloro-4-(2-chloro-3-(6-methoxy-5-(((((R)-5-oxopyrrolidin-2-yl)methyl)amino)methyl)pyridin-2-yl)phenyl)pyridin-2-yl)-2-methoxybenzyl)pyrrolidine-3-carboxylic acid;
(4-(3-chloro-4-(2-chloro-3-(6-methoxy-5-(((((S)-5-oxopyrrolidin-2-yl)methyl)amino)methyl)pyridin-2-yl)phenyl)pyridin-2-yl)-2-methoxybenzyl)-L-proline;
(4-(3-chloro-4-(2-chloro-3-(6-methoxy-5-(((((S)-5-oxopyrrolidin-2-yl)methyl)amino)methyl)pyridin-2-yl)phenyl)pyridin-2-yl)-2-methoxybenzyl)-D-proline;
(4-(3-chloro-4-(2-chloro-3-(6-methoxy-5-(((((R)-5-oxopyrrolidin-2-yl)methyl)amino)methyl)pyridin-2-yl)phenyl)pyridin-2-yl)-2-methoxybenzyl)-L-proline;
(4-(3-chloro-4-(2-chloro-3-(6-methoxy-5-(((((R)-5-oxopyrrolidin-2-yl)methyl)amino)methyl)pyridin-2-yl)phenyl)pyridin-2-yl)-2-methoxybenzyl)-D-proline;
(S)-5-((((6-(2-chloro-3-(3-chloro-2-(3-fluoro-4-((((1r,3r)-3-hydroxy-3-methylcyclobutyl)amino)methyl)-5-methoxyphenyl)pyridin-4-yl)phenyl)-2-methoxypyridin-3-yl)methyl)amino)methyl)pyrrolidin-2-one;
(R)-5-((((6-(2-chloro-3-(3-chloro-2-(3-fluoro-4-((((1r,3r)-3-hydroxy-3-methylcyclobutyl)amino)methyl)-5-methoxyphenyl)pyridin-4-yl)phenyl)-2-methoxypyridin-3-yl)methyl)amino)methyl)pyrrolidin-2-one;
(R)-5-((((6-(2-chloro-3-(3-chloro-2-(3-fluoro-4-((((1r,3s)-3-hydroxy-3-methylcyclobutyl)amino)methyl)-5-methoxyphenyl)pyridin-4-yl)phenyl)-2-methoxypyridin-3-yl)methyl)amino)methyl)pyrrolidin-2-one;
(R)-5-((((6-(2-chloro-3-(3-chloro-2-(3-fluoro-4-((((1s,3r)-3-hydroxy-3-methylcyclobutyl)amino)methyl)-5-methoxyphenyl)pyridin-4-yl)phenyl)-2-methoxypyridin-3-yl)methyl)amino)methyl)pyrrolidin-2-one;
(S)-5-((((6-(2-chloro-3-(3-chloro-2-(3-fluoro-4-((((1s,3r)-3-hydroxy-3-methylcyclobutyl)amino)methyl)-5-methoxyphenyl)pyridin-4-yl)phenyl)-2-methoxypyridin-3-yl)methyl)amino)methyl)pyrrolidin-2-one;
(S)-5-((((6-(2-chloro-3-(3-chloro-2-(3-fluoro-4-((((1r,3s)-3-hydroxy-3-methylcyclobutyl)amino)methyl)-5-methoxyphenyl)pyridin-4-yl)phenyl)-2-methoxypyridin-3-yl)methyl)amino)methyl)pyrrolidin-2-one;
(R)-5-((((6-(2-chloro-3-(3-chloro-2-(3-fluoro-4-((((1s,3s)-3-hydroxy-3-methylcyclobutyl)amino)methyl)-5-methoxyphenyl)pyridin-4-yl)phenyl)-2-methoxypyridin-3-yl)methyl)amino)methyl)pyrrolidin-2-one;
(S)-5-((((6-(2-chloro-3-(3-chloro-2-(3-fluoro-4-((((1s,3s)-3-hydroxy-3-methylcyclobutyl)amino)methyl)-5-methoxyphenyl)pyridin-4-yl)phenyl)-2-methoxypyridin-3-yl)methyl)amino)methyl)pyrrolidin-2-one;
(S)-5-((((6-(2-chloro-3-(3-chloro-2-(3-fluoro-4-((((S)-2-hydroxypropyl)amino)methyl)-5-methoxyphenyl)pyridin-4-yl)phenyl)-2-methoxypyridin-3-yl)methyl)amino)methyl)pyrrolidin-2-one;
(R)-5-((((6-(2-chloro-3-(3-chloro-2-(3-fluoro-4-((((S)-2-hydroxypropyl)amino)methyl)-5-methoxyphenyl)pyridin-4-yl)phenyl)-2-methoxypyridin-3-yl)methyl)amino)methyl)pyrrolidin-2-one;
(R)-5-((((6-(2-chloro-3-(3-chloro-2-(3-fluoro-4-((((R)-2-hydroxypropyl)amino)methyl)-5-methoxyphenyl)pyridin-4-yl)phenyl)-2-methoxypyridin-3-yl)methyl)amino)methyl)pyrrolidin-2-one;
(S)-5-((((6-(2-chloro-3-(3-chloro-2-(3-fluoro-4-((((R)-2-hydroxypropyl)amino)methyl)-5-methoxyphenyl)pyridin-4-yl)phenyl)-2-methoxypyridin-3-yl)methyl)amino)methyl)pyrrolidin-2-one;
(S)-5-((((6-(2-chloro-3-(3-chloro-2-(3-fluoro-4-((isopropylamino)methyl)-5-methoxyphenyl)pyridin-4-yl)phenyl)-2-methoxypyridin-3-yl)methyl)amino)methyl)pyrrolidin-2-one;
(R)-5-((((6-(2-chloro-3-(3-chloro-2-(3-fluoro-4-((isopropylamino)methyl)-5-methoxyphenyl)pyridin-4-yl)phenyl)-2-methoxypyridin-3-yl)methyl)amino)methyl)pyrrolidin-2-one;
(S)-5-((((6-(2-chloro-3-(3-chloro-2-(3-fluoro-4-((isobutylamino)methyl)-5-methoxyphenyl)pyridin-4-yl)phenyl)-2-methoxypyridin-3-yl)methyl)amino)methyl)pyrrolidin-2-one;
(R)-5-((((6-(2-chloro-3-(3-chloro-2-(3-fluoro-4-((isobutylamino)methyl)-5-methoxyphenyl)pyridin-4-yl)phenyl)-2-methoxypyridin-3-yl)methyl)amino)methyl)pyrrolidin-2-one;
2-(((6-(2-chloro-3-(3-chloro-2-(2-(3-fluoropropyl)-8-methoxy-1,2,3,4-tetrahydroisoquinolin-6-yl)pyridin-4-yl)phenyl)-2-methoxypyridin-3-yl)methyl)amino)ethan-1-ol;
2-(((6-(2-chloro-3-(3-chloro-2-(8-methoxy-2-(2-(piperidin-4-yl)ethyl)-1,2,3,4-tetrahydroisoquinolin-6-yl)pyridin-4-yl)phenyl)-2-methoxypyridin-3-yl)methyl)amino)ethan-1-ol;
(S)-5-((((4-chloro-6-(2-chloro-3-(3-chloro-2-(2-((S)-2-hydroxypropyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(S)-5-((((4-chloro-6-(2-chloro-3-(3-chloro-2-(2-((R)-2-hydroxypropyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((4-chloro-6-(2-chloro-3-(3-chloro-2-(2-((S)-2-hydroxypropyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((4-chloro-6-(2-chloro-3-(3-chloro-2-(2-((R)-2-hydroxypropyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
6-(3-chloro-4-(2-chloro-3-(4-chloro-6-methoxy-5-(((((S)-5-oxopyrrolidin-2-yl)methyl)amino)methyl)pyridin-2-yl)phenyl)pyridin-2-yl)-2-((((1r,3r)-3-hydroxy-3-methylcyclobutyl)amino)methyl)-3-methylpyrrolo[2,1-f][1,2,4]triazin-4(3H)-one;
6-(3-chloro-4-(2-chloro-3-(4-chloro-6-methoxy-5-(((((R)-5-oxopyrrolidin-2-yl)methyl)amino)methyl)pyridin-2-yl)phenyl)pyridin-2-yl)-2-((((1r,3s)-3-hydroxy-3-methylcyclobutyl)amino)methyl)-3-methylpyrrolo[2,1-f][1,2,4]triazin-4(3H)-one;
6-(3-chloro-4-(2-chloro-3-(4-chloro-6-methoxy-5-(((((R)-5-oxopyrrolidin-2-yl)methyl)amino)methyl)pyridin-2-yl)phenyl)pyridin-2-yl)-2-((((1s,3r)-3-hydroxy-3-methylcyclobutyl)amino)methyl)-3-methylpyrrolo[2,1-f][1,2,4]triazin-4(3H)-one;
6-(3-chloro-4-(2-chloro-3-(4-chloro-6-methoxy-5-(((((S)-5-oxopyrrolidin-2-yl)methyl)amino)methyl)pyridin-2-yl)phenyl)pyridin-2-yl)-2-((((1s,3r)-3-hydroxy-3-methylcyclobutyl)amino)methyl)-3-methylpyrrolo[2,1-f][1,2,4]triazin-4(3H)-one;
6-(3-chloro-4-(2-chloro-3-(4-chloro-6-methoxy-5-(((((S)-5-oxopyrrolidin-2-yl)methyl)amino)methyl)pyridin-2-yl)phenyl)pyridin-2-yl)-2-((((1r,3s)-3-hydroxy-3-methylcyclobutyl)amino)methyl)-3-methylpyrrolo[2,1-f][1,2,4]triazin-4(3H)-one;
6-(3-chloro-4-(2-chloro-3-(4-chloro-6-methoxy-5-(((((R)-5-oxopyrrolidin-2-yl)methyl)amino)methyl)pyridin-2-yl)phenyl)pyridin-2-yl)-2-((((1s,3s)-3-hydroxy-3-methylcyclobutyl)amino)methyl)-3-methylpyrrolo[2,1-f][1,2,4]triazin-4(3H)-one;
6-(3-chloro-4-(2-chloro-3-(4-chloro-6-methoxy-5-(((((R)-5-oxopyrrolidin-2-yl)methyl)amino)methyl)pyridin-2-yl)phenyl)pyridin-2-yl)-2-((((1r,3r)-3-hydroxy-3-methylcyclobutyl)amino)methyl)-3-methylpyrrolo[2,1-f][1,2,4]triazin-4(3H)-one;
6-(3-chloro-4-(2-chloro-3-(4-chloro-6-methoxy-5-(((((S)-5-oxopyrrolidin-2-yl)methyl)amino)methyl)pyridin-2-yl)phenyl)pyridin-2-yl)-2-((((1s,3s)-3-hydroxy-3-methylcyclobutyl)amino)methyl)-3-methylpyrrolo[2,1-f][1,2,4]triazin-4(3H)-one;
(S)-2-(4-(3-chloro-4-(2-chloro-3-(6-methoxy-5-((((5-oxopyrrolidin-2-yl)methyl)amino)methyl)pyridin-2-yl)phenyl)pyridin-2-yl)-2-methoxybenzyl)-2,6-diazaspiro[3.4]octan-7-one;
(R)-2-(4-(3-chloro-4-(2-chloro-3-(6-methoxy-5-((((5-oxopyrrolidin-2-yl)methyl)amino)methyl)pyridin-2-yl)phenyl)pyridin-2-yl)-2-methoxybenzyl)-2,6-diazaspiro[3.4]octan-7-one;
2-(4-(3-chloro-4-(2-chloro-3-(6-methoxy-5-((7-oxo-2,6-diazaspiro[3.4]octan-2-yl)methyl)pyridin-2-yl)phenyl)pyridin-2-yl)-2-methoxybenzyl)-2,6-diazaspiro[3.4]octan-7-one;
1-(6-((6-(3-(2-(4-((6-acetyl-2,6-diazaspiro[3.3]heptan-2-yl)methyl)-3-methoxyphenyl)-3-chloropyridin-4-yl)-2-chlorophenyl)-2-methoxypyridin-3-yl)methyl)-2,6-diazaspiro[3.3]heptan-2-yl)ethan-1-one;
6-(3-chloro-4-(2-chloro-3-(6-methoxy-5-(((((S)-5-oxopyrrolidin-2-yl)methyl)amino)methyl)pyridin-2-yl)phenyl)pyridin-2-yl)-2-((((S)-2-hydroxypropyl)amino)methyl)-3-methylpyrrolo[2,1-f][1,2,4]triazin-4(3H)-one;
6-(3-chloro-4-(2-chloro-3-(6-methoxy-5-(((((S)-5-oxopyrrolidin-2-yl)methyl)amino)methyl)pyridin-2-yl)phenyl)pyridin-2-yl)-2-((((R)-2-hydroxypropyl)amino)methyl)-3-methylpyrrolo[2,1-f][1,2,4]triazin-4(3H)-one;
6-(3-chloro-4-(2-chloro-3-(6-methoxy-5-(((((R)-5-oxopyrrolidin-2-yl)methyl)amino)methyl)pyridin-2-yl)phenyl)pyridin-2-yl)-2-((((S)-2-hydroxypropyl)amino)methyl)-3-methylpyrrolo[2,1-f][1,2,4]triazin-4(3H)-one;
6-(3-chloro-4-(2-chloro-3-(6-methoxy-5-(((((R)-5-oxopyrrolidin-2-yl)methyl)amino)methyl)pyridin-2-yl)phenyl)pyridin-2-yl)-2-((((R)-2-hydroxypropyl)amino)methyl)-3-methylpyrrolo[2,1-f][1,2,4]triazin-4(3H)-one;
(S)-5-(((4-(4-(3-(3-(((1-acetylpiperidin-4-yl)(methyl)amino)methyl)-1-methyl-1H-pyrrolo[2,3-b]pyridin-6-yl)-2-chlorophenyl)-3-chloropyridin-2-yl)-2-methoxybenzyl)amino)methyl)pyrrolidin-2-one;
(R)-5-(((4-(4-(3-(3-(((1-acetylpiperidin-4-yl)(methyl)amino)methyl)-1-methyl-1H-pyrrolo[2,3-b]pyridin-6-yl)-2-chlorophenyl)-3-chloropyridin-2-yl)-2-methoxybenzyl)amino)methyl)pyrrolidin-2-one;
(S)-5-(((4-(3-chloro-4-(2-chloro-3-(1-methyl-3-(((((S)-tetrahydrofuran-2-yl)methyl)amino)methyl)-1H-pyrrolo[2,3-b]pyridin-6-yl)phenyl)pyridin-2-yl)-2-methoxybenzyl)amino)methyl)pyrrolidin-2-one;
(S)-5-(((4-(3-chloro-4-(2-chloro-3-(1-methyl-3-(((((R)-tetrahydrofuran-2-yl)methyl)amino)methyl)-1H-pyrrolo[2,3-b]pyridin-6-yl)phenyl)pyridin-2-yl)-2-methoxybenzyl)amino)methyl)pyrrolidin-2-one;
(R)-5-(((4-(3-chloro-4-(2-chloro-3-(1-methyl-3-(((((S)-tetrahydrofuran-2-yl)methyl)amino)methyl)-1H-pyrrolo[2,3-b]pyridin-6-yl)phenyl)pyridin-2-yl)-2-methoxybenzyl)amino)methyl)pyrrolidin-2-one;
(R)-5-(((4-(3-chloro-4-(2-chloro-3-(1-methyl-3-(((((R)-tetrahydrofuran-2-yl)methyl)amino)methyl)-1H-pyrrolo[2,3-b]pyridin-6-yl)phenyl)pyridin-2-yl)-2-methoxybenzyl)amino)methyl)pyrrolidin-2-one;
(S)-5-(((4-(3-chloro-4-(2-chloro-3-(1-methyl-3-((methylamino)methyl)-1H-pyrrolo[2,3-b]pyridin-6-yl)phenyl)pyridin-2-yl)-2-methoxybenzyl)amino)methyl)pyrrolidin-2-one;
(R)-5-(((4-(3-chloro-4-(2-chloro-3-(1-methyl-3-((methylamino)methyl)-1H-pyrrolo[2,3-b]pyridin-6-yl)phenyl)pyridin-2-yl)-2-methoxybenzyl)amino)methyl)pyrrolidin-2-one;
(S)-5-(((4-(4-(3-(3-(((1-acetylazetidin-3-yl)amino)methyl)-1-methyl-1H-pyrrolo[2,3-b]pyridin-6-yl)-2-chlorophenyl)-3-chloropyridin-2-yl)-2-methoxybenzyl)amino)methyl)pyrrolidin-2-one;
(R)-5-(((4-(4-(3-(3-(((1-acetylazetidin-3-yl)amino)methyl)-1-methyl-1H-pyrrolo[2,3-b]pyridin-6-yl)-2-chlorophenyl)-3-chloropyridin-2-yl)-2-methoxybenzyl)amino)methyl)pyrrolidin-2-one;
(S)-5-(((4-(3-chloro-4-(2-chloro-3-(3-(((4,4-difluorocyclohexyl)amino)methyl)-1-methyl-1H-pyrrolo[2,3-b]pyridin-6-yl)phenyl)pyridin-2-yl)-2-methoxybenzyl)amino)methyl)pyrrolidin-2-one;
(R)-5-(((4-(3-chloro-4-(2-chloro-3-(3-(((4,4-difluorocyclohexyl)amino)methyl)-1-methyl-1H-pyrrolo[2,3-b]pyridin-6-yl)phenyl)pyridin-2-yl)-2-methoxybenzyl)amino)methyl)pyrrolidin-2-one;
(S)-5-(((4-(3-chloro-4-(2-chloro-3-(3-(((2-hydroxyethyl)amino)methyl)-1-methyl-1H-pyrrolo[2,3-b]pyridin-6-yl)phenyl)pyridin-2-yl)-2-methoxybenzyl)amino)methyl)pyrrolidin-2-one;
(R)-5-(((4-(3-chloro-4-(2-chloro-3-(3-(((2-hydroxyethyl)amino)methyl)-1-methyl-1H-pyrrolo[2,3-b]pyridin-6-yl)phenyl)pyridin-2-yl)-2-methoxybenzyl)amino)methyl)pyrrolidin-2-one;
(S)-5-(((4-(3-chloro-4-(2-chloro-3-(3-((((S)-2-hydroxypropyl)amino)methyl)-1-methyl-1H-pyrrolo[2,3-b]pyridin-6-yl)phenyl)pyridin-2-yl)-2-methoxybenzyl)amino)methyl)pyrrolidin-2-one;
(S)-5-(((4-(3-chloro-4-(2-chloro-3-(3-((((R)-2-hydroxypropyl)amino)methyl)-1-methyl-1H-pyrrolo[2,3-b]pyridin-6-yl)phenyl)pyridin-2-yl)-2-methoxybenzyl)amino)methyl)pyrrolidin-2-one;
(R)-5-(((4-(3-chloro-4-(2-chloro-3-(3-((((S)-2-hydroxypropyl)amino)methyl)-1-methyl-1H-pyrrolo[2,3-b]pyridin-6-yl)phenyl)pyridin-2-yl)-2-methoxybenzyl)amino)methyl)pyrrolidin-2-one;
(R)-5-(((4-(3-chloro-4-(2-chloro-3-(3-((((R)-2-hydroxypropyl)amino)methyl)- 1 -methyl-1H-pyrrolo[2,3-b]pyridin-6-yl)phenyl)pyridin-2-yl)-2-methoxybenzyl)amino)methyl)pyrrolidin-2-one;
(S)-5-(((4-(3-chloro-4-(2-chloro-3-(3-(((2-hydroxy-2-methylpropyl)amino)methyl)-1-methyl-1H-pyrrolo[2,3-b]pyridin-6-yl)phenyl)pyridin-2-yl)-2-methoxybenzyl)amino)methyl)pyrrolidin-2-one;
(R)-5-(((4-(3-chloro-4-(2-chloro-3-(3-(((2-hydroxy-2-methylpropyl)amino)methyl)-1-methyl-1H-pyrrolo[2,3-b]pyridin-6-yl)phenyl)pyridin-2-yl)-2-methoxybenzyl)amino)methyl)pyrrolidin-2-one;
(S)-5-(((4-(3-chloro-4-(2-chloro-3-(3-(((2-methoxyethyl)amino)methyl)-1-methyl-1H-pyrrolo[2,3-b]pyridin-6-yl)phenyl)pyridin-2-yl)-2-methoxybenzyl)amino)methyl)pyrrolidin-2-one;
(R)-5-(((4-(3-chloro-4-(2-chloro-3-(3-(((2-methoxyethyl)amino)methyl)-1-methyl-1H-pyrrolo[2,3-b]pyridin-6-yl)phenyl)pyridin-2-yl)-2-methoxybenzyl)amino)methyl)pyrrolidin-2-one;
(S)-5-(((4-(3-chloro-4-(2-chloro-3-(3-(((2-hydroxyethyl)(methyl)amino)methyl)-1-methyl-1H-pyrrolo[2,3-b]pyridin-6-yl)phenyl)pyridin-2-yl)-2-methoxybenzyl)amino)methyl)pyrrolidin-2-one;
(R)-5-(((4-(3-chloro-4-(2-chloro-3-(3-(((2-hydroxyethyl)(methyl)amino)methyl)-1-methyl-1H-pyrrolo[2,3-b]pyridin-6-yl)phenyl)pyridin-2-yl)-2-methoxybenzyl)amino)methyl)pyrrolidin-2-one;
(S)-6-(3-chloro-4-(2-chloro-3-(6-methoxy-5-((((5-oxopyrrolidin-2-yl)methyl)amino)methyl)pyridin-2-yl)phenyl)pyridin-2-yl)-2-(((3-hydroxy-3-methylcyclobutyl)amino)methyl)-3-methylpyrrolo[2,1-f][1,2,4]triazin-4(3H)-one;
(R)-6-(3-chloro-4-(2-chloro-3-(6-methoxy-5-((((5-oxopyrrolidin-2-yl)methyl)amino)methyl)pyridin-2-yl)phenyl)pyridin-2-yl)-2-(((3-hydroxy-3-methylcyclobutyl)amino)methyl)-3-methylpyrrolo[2,1-f][1,2,4]triazin-4(3H)-one;
6-(3-chloro-4-(2-chloro-3-(6-methoxy-5-(((((S)-5-oxopyrrolidin-2-yl)methyl)amino)methyl)pyridin-2-yl)phenyl)pyridin-2-yl)-3-methyl-2-(((((S)-5-oxopyrrolidin-2-yl)methyl)amino)methyl)pyrrolo[2,1-f][1,2,4]triazin-4(3H)-one;
6-(3-chloro-4-(2-chloro-3-(6-methoxy-5-(((((S)-5-oxopyrrolidin-2-yl)methyl)amino)methyl)pyridin-2-yl)phenyl)pyridin-2-yl)-3-methyl-2-(((((R)-5-oxopyrrolidin-2-yl)methyl)amino)methyl)pyrrolo[2,1-f][1,2,4]triazin-4(3H)-one;
6-(3-chloro-4-(2-chloro-3-(6-methoxy-5-(((((R)-5-oxopyrrolidin-2-yl)methyl)amino)methyl)pyridin-2-yl)phenyl)pyridin-2-yl)-3-methyl-2-(((((S)-5-oxopyrrolidin-2-yl)methyl)amino)methyl)pyrrolo[2,1-f][1,2,4]triazin-4(3H)-one;
6-(3-chloro-4-(2-chloro-3-(6-methoxy-5-(((((R)-5-oxopyrrolidin-2-yl)methyl)amino)methyl)pyridin-2-yl)phenyl)pyridin-2-yl)-3-methyl-2-(((((R)-5-oxopyrrolidin-2-yl)methyl)amino)methyl)pyrrolo[2,1-f][1,2,4]triazin-4(3H)-one;
(S)-5-((((4-chloro-6-(2-chloro-3-(3-chloro-2-(2-((R)-2-hydroxypropyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(S)-5-((((4-chloro-6-(2-chloro-3-(3-chloro-2-(2-((S)-2-hydroxypropyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((4-chloro-6-(2-chloro-3-(3-chloro-2-(2-((R)-2-hydroxypropyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((4-chloro-6-(2-chloro-3-(3-chloro-2-(2-((S)-2-hydroxypropyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(S)-(4-(3-chloro-4-(2-chloro-3-(4-chloro-6-methoxy-5-((((5-oxopyrrolidin-2-yl)methyl)amino)methyl)pyridin-2-yl)phenyl)pyridin-2-yl)-2-methoxybenzyl)glycine;
(R)-(4-(3-chloro-4-(2-chloro-3-(4-chloro-6-methoxy-5-((((5-oxopyrrolidin-2-yl)methyl)amino)methyl)pyridin-2-yl)phenyl)pyridin-2-yl)-2-methoxybenzyl)glycine;
3-chloro-4-(2-chloro-3-(5-(4,5-dihydro-1H-imidazol-2-yl)-6-methoxypyridin-2-yl)phenyl)-2-(4-(4,5-dihydro-1H-imidazol-2-yl)-3-methoxyphenyl)pyridine;
(S)-(4-(3-chloro-4-(2-chloro-3-(6-methoxy-5-((((5-oxopyrrolidin-2-yl)methyl)amino)methyl)pyridin-2-yl)phenyl)pyridin-2-yl)-2-methoxybenzyl)glycine;
(R)-(4-(3-chloro-4-(2-chloro-3-(6-methoxy-5-((((5-oxopyrrolidin-2-yl)methyl)amino)methyl)pyridin-2-yl)phenyl)pyridin-2-yl)-2-methoxybenzyl)glycine;
isopropyl (S)-(4-(3-chloro-4-(2-chloro-3-(6-methoxy-5-((((5-oxopyrrolidin-2-yl)methyl)amino)methyl)pyridin-2-yl)phenyl)pyridin-2-yl)-2-methoxybenzyl)glycinate;
isopropyl (R)-(4-(3-chloro-4-(2-chloro-3-(6-methoxy-5-((((5-oxopyrrolidin-2-yl)methyl)amino)methyl)pyridin-2-yl)phenyl)pyridin-2-yl)-2-methoxybenzyl)glycinate;
(S)-5-((((6-(3-(2-(4-(((1-acetylpiperidin-4-yl)amino)methyl)-3-methoxyphenyl)-3-chloropyridin-4-yl)-2-chlorophenyl)-2-methoxypyridin-3-yl)methyl)amino)methyl)pyrrolidin-2-one;
(R)-5-((((6-(3-(2-(4-(((1-acetylpiperidin-4-yl)amino)methyl)-3-methoxyphenyl)-3-chloropyridin-4-yl)-2-chlorophenyl)-2-methoxypyridin-3-yl)methyl)amino)methyl)pyrrolidin-2-one;
(S)-6-(3-chloro-4-(2-chloro-3-(6-methoxy-5-((((5-oxopyrrolidin-2-yl)methyl)amino)methyl)pyridin-2-yl)phenyl)pyridin-2-yl)-2-((3-hydroxy-3-methylazetidin-1-yl)methyl)-3-methylpyrrolo[2, 1-f] [1,2,4]triazin-4(3H)-one;
(R)-6-(3-chloro-4-(2-chloro-3-(6-methoxy-5-((((5-oxopyrrolidin-2-yl)methyl)amino)methyl)pyridin-2-yl)phenyl)pyridin-2-yl)-2-((3-hydroxy-3-methylazetidin-1-yl)methyl)-3-methylpyrrolo[2, 1-f][1,2,4]triazin-4(3H)-one;
(S)-6-(3-chloro-4-(2-chloro-3-(6-methoxy-5-((((5-oxopyrrolidin-2-yl)methyl)amino)methyl)pyridin-2-yl)phenyl)pyridin-2-yl)-2-((isopropylamino)methyl)-3-methylpyrrolo[2,1-f][1,2,4]triazin-4(3H)-one;
(R)-6-(3-chloro-4-(2-chloro-3-(6-methoxy-5-((((5-oxopyrrolidin-2-yl)methyl)amino)methyl)pyridin-2-yl)phenyl)pyridin-2-yl)-2-((isopropylamino)methyl)-3-methylpyrrolo[2,1-f][1,2,4]triazin-4(3H)-one;
(5S)-5-((((6-(2-chloro-3-(3-chloro-2-(5-((((S)-5-oxopyrrolidin-2-yl)methyl)amino)-5,6,7,8-tetrahydronaphthalen-2-yl)pyridin-4-yl)phenyl)-2-methoxypyridin-3-yl)methyl)amino)methyl)pyrrolidin-2-one;
(5S)-5-((((6-(2-chloro-3-(3-chloro-2-(5-((((R)-5-oxopyrrolidin-2-yl)methyl)amino)-5,6,7,8-tetrahydronaphthalen-2-yl)pyridin-4-yl)phenyl)-2-methoxypyridin-3-yl)methyl)amino)methyl)pyrrolidin-2-one;
(5R)-5-((((6-(2-chloro-3-(3-chloro-2-(5-((((S)-5-oxopyrrolidin-2-yl)methyl)amino)-5,6,7,8-tetrahydronaphthalen-2-yl)pyridin-4-yl)phenyl)-2-methoxypyridin-3-yl)methyl)amino)methyl)pyrrolidin-2-one;
(5R)-5-((((6-(2-chloro-3-(3-chloro-2-(5-((((R)-5-oxopyrrolidin-2-yl)methyl)amino)-5,6,7,8-tetrahydronaphthalen-2-yl)pyridin-4-yl)phenyl)-2-methoxypyridin-3-yl)methyl)amino)methyl)pyrrolidin-2-one;
(5 S)-5-((((6-(2-chloro-3-(3-chloro-2-(5-(((S)-2-hydroxypropyl)amino)-5,6,7, 8-tetrahydronaphthalen-2-yl)pyridin-4-yl)phenyl)-2-methoxypyridin-3-yl)methyl)amino)methyl)pyrrolidin-2-one;
(5 S)-5-((((6-(2-chloro-3-(3-chloro-2-(5-(((R)-2-hydroxypropyl)amino)-5,6,7, 8-tetrahydronaphthalen-2-yl)pyridin-4-yl)phenyl)-2-methoxypyridin-3-yl)methyl)amino)methyl)pyrrolidin-2-one;
(5R)-5-((((6-(2-chloro-3-(3-chloro-2-(5-(((S)-2-hydroxypropyl)amino)-5,6,7,8-tetrahydronaphthalen-2-yl)pyridin-4-yl)phenyl)-2-methoxypyridin-3-yl)methyl)amino)methyl)pyrrolidin-2-one;
(5R)-5-((((6-(2-chloro-3-(3-chloro-2-(5-(((R)-2-hydroxypropyl)amino)-5,6,7,8-tetrahydronaphthalen-2-yl)pyridin-4-yl)phenyl)-2-methoxypyridin-3-yl)methyl)amino)methyl)pyrrolidin-2-one;
(S)-5-((((6-(2-chloro-3-(3-chloro-2-(4-((((S)-2-hydroxypropyl)amino)methyl)-3-methoxy-5-methylphenyl)pyridin-4-yl)phenyl)-2-methoxypyridin-3-yl)methyl)amino)methyl)pyrrolidin-2-one;
(S)-5-((((6-(2-chloro-3-(3-chloro-2-(4-((((R)-2-hydroxypropyl)amino)methyl)-3-methoxy-5-methylphenyl)pyridin-4-yl)phenyl)-2-methoxypyridin-3-yl)methyl)amino)methyl)pyrrolidin-2-one;
(R)-5-((((6-(2-chloro-3-(3-chloro-2-(4-((((S)-2-hydroxypropyl)amino)methyl)-3-methoxy-5-methylphenyl)pyridin-4-yl)phenyl)-2-methoxypyridin-3-yl)methyl)amino)methyl)pyrrolidin-2-one;
(R)-5-((((6-(2-chloro-3-(3-chloro-2-(4-((((R)-2-hydroxypropyl)amino)methyl)-3-methoxy-5-methylphenyl)pyridin-4-yl)phenyl)-2-methoxypyridin-3-yl)methyl)amino)methyl)pyrrolidin-2-one;
(S)-5-((((6-(2-chloro-3-(3-chloro-2-(3-methoxy-5-methyl-4-(((((S)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
(S)-5-((((6-(2-chloro-3-(3-chloro-2-(3-methoxy-5-methyl-4-(((((R)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
(R)-5-((((6-(2-chloro-3-(3-chloro-2-(3-methoxy-5-methyl-4-(((((S)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
(R)-5-((((6-(2-chloro-3-(3-chloro-2-(3-methoxy-5-methyl-4-(((((R)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
(S)-5-((((6-(2-chloro-3-(3-chloro-2-(isoindolin-5-yl)pyridin-4-yl)phenyl)-2-methoxypyridin-3-yl)methyl)amino)methyl)pyrrolidin-2-one;
(R)-5-((((6-(2-chloro-3-(3-chloro-2-(isoindolin-5-yl)pyridin-4-yl)phenyl)-2-methoxypyridin-3-yl)methyl)amino)methyl)pyrrolidin-2-one;
(S)-5-((((6-(2-chloro-3-(3-chloro-2-(3-methoxy-5-methyl-4-((methyl(((S)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
(S)-5-((((6-(2-chloro-3-(3-chloro-2-(3-methoxy-5-methyl-4-((methyl(((R)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
(R)-5-((((6-(2-chloro-3-(3-chloro-2-(3-methoxy-5-methyl-4-((methyl(((S)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
(R)-5-((((6-(2-chloro-3-(3-chloro-2-(3-methoxy-5-methyl-4-((methyl(((R)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
(S)-5-((((6-(2-chloro-3-(3-chloro-2-(4-((((S)-2-hydroxypropyl)(methyl)amino)methyl)-3-methoxy-5-methylphenyl)pyridin-4-yl)phenyl)-2-methoxypyridin-3-yl)methyl)amino)methyl)pyrrolidin-2-one;
(S)-5-((((6-(2-chloro-3-(3-chloro-2-(4-((((R)-2-hydroxypropyl)(methyl)amino)methyl)-3-methoxy-5-methylphenyl)pyridin-4-yl)phenyl)-2-methoxypyridin-3-yl)methyl)amino)methyl)pyrrolidin-2-one;
(R)-5-((((6-(2-chloro-3-(3-chloro-2-(4-((((S)-2-hydroxypropyl)(methyl)amino)methyl)-3-methoxy-5-methylphenyl)pyridin-4-yl)phenyl)-2-methoxypyridin-3-yl)methyl)amino)methyl)pyrrolidin-2-one;
(R)-5-((((6-(2-chloro-3-(3-chloro-2-(4-((((R)-2-hydroxypropyl)(methyl)amino)methyl)-3-methoxy-5-methylphenyl)pyridin-4-yl)phenyl)-2-methoxypyridin-3-yl)methyl)amino)methyl)pyrrolidin-2-one;
(S)-5-((((6-(3-(2-(1-(azetidin-3-yl)-1H-pyrazol-4-yl)-3-chloropyridin-4-yl)-2-chlorophenyl)-2-methoxypyridin-3-yl)methyl)amino)methyl)pyrrolidin-2-one;
(R)-5-((((6-(3-(2-(1-(azetidin-3-yl)-1H-pyrazol-4-yl)-3-chloropyridin-4-yl)-2-chlorophenyl)-2-methoxypyridin-3-yl)methyl)amino)methyl)pyrrolidin-2-one;
(S)-5-((((6-(3-(2-(1-(azetidin-3-ylmethyl)-1H-pyrazol-4-yl)-3-chloropyridin-4-yl)-2-chlorophenyl)-2-methoxypyridin-3-yl)methyl)amino)methyl)pyrrolidin-2-one;
(R)-5-((((6-(3-(2-(1-(azetidin-3-ylmethyl)-1H-pyrazol-4-yl)-3-chloropyridin-4-yl)-2-chlorophenyl)-2-methoxypyridin-3-yl)methyl)amino)methyl)pyrrolidin-2-one;
(S)-1-((4-(3-chloro-4-(2-chloro-3-(6-methoxy-5-((((5-oxopyrrolidin-2-yl)methyl)amino)methyl)pyridin-2-yl)phenyl)pyridin-2-yl)-2-methoxybenzyl)amino)cyclopropane-1-carboxylic acid;
(R)-1-((4-(3-chloro-4-(2-chloro-3-(6-methoxy-5-((((5-oxopyrrolidin-2-yl)methyl)amino)methyl)pyridin-2-yl)phenyl)pyridin-2-yl)-2-methoxybenzyl)amino)cyclopropane-1-carboxylic acid;
(S)-5-((((6-(2-chloro-3-(3-chloro-2-(3-methoxy-4-(((((S)-oxetan-2-yl)methyl)amino)methyl)phenyl)pyridin-4-yl)phenyl)-2-methoxypyridin-3-yl)methyl)amino)methyl)pyrrolidin-2-one;
(S)-5-((((6-(2-chloro-3-(3-chloro-2-(3-methoxy-4-(((((R)-oxetan-2-yl)methyl)amino)methyl)phenyl)pyridin-4-yl)phenyl)-2-methoxypyridin-3-yl)methyl)amino)methyl)pyrrolidin-2-one;
(R)-5-((((6-(2-chloro-3-(3-chloro-2-(3-methoxy-4-(((((S)-oxetan-2-yl)methyl)amino)methyl)phenyl)pyridin-4-yl)phenyl)-2-methoxypyridin-3-yl)methyl)amino)methyl)pyrrolidin-2-one;
(R)-5-((((6-(2-chloro-3-(3-chloro-2-(3-methoxy-4-(((((R)-oxetan-2-yl)methyl)amino)methyl)phenyl)pyridin-4-yl)phenyl)-2-methoxypyridin-3-yl)methyl)amino)methyl)pyrrolidin-2-one;
isopropyl (S)-(4-(3-chloro-4-(2-chloro-3-(4-chloro-6-methoxy-5-((((5-oxopyrrolidin-2-yl)methyl)amino)methyl)pyridin-2-yl)phenyl)pyridin-2-yl)-2-methoxybenzyl)glycinate;
isopropyl (R)-(4-(3-chloro-4-(2-chloro-3-(4-chloro-6-methoxy-5-((((5-oxopyrrolidin-2-yl)methyl)amino)methyl)pyridin-2-yl)phenyl)pyridin-2-yl)-2-methoxybenzyl)glycinate;
(S)-5-((((4-chloro-6-(2-chloro-3-(3-chloro-2-(2-(2-hydroxyethyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((4-chloro-6-(2-chloro-3-(3-chloro-2-(2-(2-hydroxyethyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
isopropyl (4-(3-chloro-4-(2-chloro-3-(6-methoxy-5-(((((S)-5-oxopyrrolidin-2-yl)methyl)amino)methyl)pyridin-2-yl)phenyl)pyridin-2-yl)-2-methoxybenzyl)-L-alaninate;
isopropyl (4-(3-chloro-4-(2-chloro-3-(6-methoxy-5-(((((S)-5-oxopyrrolidin-2-yl)methyl)amino)methyl)pyridin-2-yl)phenyl)pyridin-2-yl)-2-methoxybenzyl)-D-alaninate;
isopropyl (4-(3-chloro-4-(2-chloro-3-(6-methoxy-5-(((((R)-5-oxopyrrolidin-2-yl)methyl)amino)methyl)pyridin-2-yl)phenyl)pyridin-2-yl)-2-methoxybenzyl)-L-alaninate;
isopropyl (4-(3-chloro-4-(2-chloro-3-(6-methoxy-5-(((((R)-5-oxopyrrolidin-2-yl)methyl)amino)methyl)pyridin-2-yl)phenyl)pyridin-2-yl)-2-methoxybenzyl)-D-alaninate;
(S)-5-((((6-(2-chloro-3-(3-chloro-2-(4-(((1,1-dioxidotetrahydro-2H-thiopyran-4-yl)amino)methyl)-3-methoxyphenyl)pyridin-4-yl)phenyl)-2-methoxypyridin-3-yl)methyl)amino)methyl)pyrrolidin-2-one;
(R)-5-((((6-(2-chloro-3-(3-chloro-2-(4-(((1,1-dioxidotetrahydro-2H-thiopyran-4-yl)amino)methyl)-3-methoxyphenyl)pyridin-4-yl)phenyl)-2-methoxypyridin-3-yl)methyl)amino)methyl)pyrrolidin-2-one;
(S)-5-((((6-(2-chloro-3-(3-chloro-2-(3-methoxy-4-(((tetrahydro-2H-pyran-4-yl)amino)methyl)phenyl)pyridin-4-yl)phenyl)-2-methoxypyridin-3-yl)methyl)amino)methyl)pyrrolidin-2-one;
(R)-5-((((6-(2-chloro-3-(3-chloro-2-(3-methoxy-4-(((tetrahydro-2H-pyran-4-yl)amino)methyl)phenyl)pyridin-4-yl)phenyl)-2-methoxypyridin-3-yl)methyl)amino)methyl)pyrrolidin-2-one;
(4-(3-chloro-4-(2-chloro-3-(6-methoxy-5-(((((S)-5-oxopyrrolidin-2-yl)methyl)amino)methyl)pyridin-2-yl)phenyl)pyridin-2-yl)-2-methoxybenzyl)-L-alanine;
(4-(3-chloro-4-(2-chloro-3-(6-methoxy-5-(((((S)-5-oxopyrrolidin-2-yl)methyl)amino)methyl)pyridin-2-yl)phenyl)pyridin-2-yl)-2-methoxybenzyl)-D-alanine;
(4-(3-chloro-4-(2-chloro-3-(6-methoxy-5-(((((R)-5-oxopyrrolidin-2-yl)methyl)amino)methyl)pyridin-2-yl)phenyl)pyridin-2-yl)-2-methoxybenzyl)-L-alanine;
(4-(3-chloro-4-(2-chloro-3-(6-methoxy-5-(((((R)-5-oxopyrrolidin-2-yl)methyl)amino)methyl)pyridin-2-yl)phenyl)pyridin-2-yl)-2-methoxybenzyl)-D-alanine;
(S)-5-((((6-(2-chloro-3-(3-chloro-2-(2-((R)-2-hydroxypropyl)-8-methoxy-1,2,3,4-tetrahydroisoquinolin-6-yl)pyridin-4-yl)phenyl)-4-fluoro-2-methoxypyridin-3-yl)methyl)amino)methyl)pyrrolidin-2-one;
(S)-5-((((6-(2-chloro-3-(3-chloro-2-(2-((S)-2-hydroxypropyl)-8-methoxy-1,2,3,4-tetrahydroisoquinolin-6-yl)pyridin-4-yl)phenyl)-4-fluoro-2-methoxypyridin-3-yl)methyl)amino)methyl)pyrrolidin-2-one;
(R)-5-((((6-(2-chloro-3-(3-chloro-2-(2-((R)-2-hydroxypropyl)-8-methoxy-1,2,3,4-tetrahydroisoquinolin-6-yl)pyridin-4-yl)phenyl)-4-fluoro-2-methoxypyridin-3-yl)methyl)amino)methyl)pyrrolidin-2-one;
(R)-5-((((6-(2-chloro-3-(3-chloro-2-(2-((S)-2-hydroxypropyl)-8-methoxy-1,2,3,4-tetrahydroisoquinolin-6-yl)pyridin-4-yl)phenyl)-4-fluoro-2-methoxypyridin-3-yl)methyl)amino)methyl)pyrrolidin-2-one;
(S)-5-((((6-(2-chloro-3-(3-chloro-2-(2-(2-hydroxyethyl)-8-methoxy-1,2,3,4-tetrahydroisoquinolin-6-yl)pyridin-4-yl)phenyl)-4-fluoro-2-methoxypyridin-3-yl)methyl)amino)methyl)pyrrolidin-2-one;
(S)-5-((((6-(2-chloro-3-(3-chloro-2-(2-(2-hydroxyethyl)-8-methoxy-1,2,3,4-tetrahydroisoquinolin-6-yl)pyridin-4-yl)phenyl)-4-fluoro-2-methoxypyridin-3-yl)methyl)amino)methyl)pyrrolidin-2-one;
(S)-5-((((6-(2-chloro-3-(3-chloro-2-(2-(((1r,3r)-3-hydroxycyclobutyl)methyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(2-(((1s,3s)-3-hydroxycyclobutyl)methyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(2-(((1s,3r)-3-hydroxycyclobutyl)methyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-(((1s,3s)-3-hydroxycyclobutyl)methyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(2-(((1r,3r)-3-hydroxycyclobutyl)methyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-(((1s,3r)-3-hydroxycyclobutyl)methyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-(((1r,3s)-3-hydroxycyclobutyl)methyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-(((1r,3r)-3-hydroxycyclobutyl)methyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
isopropyl (S)-1-((4-(3-chloro-4-(2-chloro-3-(6-methoxy-5-((((5-oxopyrrolidin-2-yl)methyl)amino)methyl)pyridin-2-yl)phenyl)pyridin-2-yl)-2-methoxybenzyl)amino)cyclopropane-1-carboxylate;
isopropyl (R)-1-((4-(3-chloro-4-(2-chloro-3-(6-methoxy-5-((((5-oxopyrrolidin-2-yl)methyl)amino)methyl)pyridin-2-yl)phenyl)pyridin-2-yl)-2-methoxybenzyl)amino)cyclopropane-1-carboxylate;
1-(6-(2-chloro-3-(3-chloro-2-(3-methoxy-4-((methylamino)methyl)phenyl)pyridin-4-yl)phenyl)-2-methoxypyridin-3-yl)-N-methylmethanamine;
1-(6-(2-chloro-3-(3-chloro-2-(4-((dimethylamino)methyl)-3-methoxyphenyl)pyridin-4-yl)phenyl)-2-methoxypyridin-3-yl)-N,N-dimethylmethanamine;
2-(((6-(2-chloro-3-(3-chloro-2-(4-(((2-hydroxyethyl)(methyl)amino)methyl)-3-methoxyphenyl)pyridin-4-yl)phenyl)-2-methoxypyridin-3-yl)methyl)(methyl)amino)ethan-1-ol;
1-(4-(((6-(3-(2-(4-(((1-acetylpiperidin-4-yl)amino)methyl)-3-methoxyphenyl)-3-chloropyridin-4-yl)-2-chlorophenyl)-2-methoxypyridin-3-yl)methyl)amino)piperidin-1-yl)ethan-1-one;
1-(4-(((6-(3-(2-(4-(((1-acetylpiperidin-4-yl)(methyl)amino)methyl)-3-methoxyphenyl)-3-chloropyridin-4-yl)-2-chlorophenyl)-2-methoxypyridin-3-yl)methyl)(methyl)amino)piperidin-1-yl)ethan-1-one;
(S)-5-((((6-(2-chloro-3-(3-chloro-2-(5-methyl-2,3,4,5-tetrahydro-1H-pyrido[4,3-b]indol-7-yl)pyridin-4-yl)phenyl)-2-methoxypyridin-3-yl)methyl)amino)methyl)pyrrolidin-2-one;
(S)-5-((((6-(2-chloro-3-(3-chloro-2-(5-methyl-2,3,4,5-tetrahydro-1H-pyrido[4,3-b]indol-7-yl)pyridin-4-yl)phenyl)-2-methoxypyridin-3-yl)methyl)amino)methyl)pyrrolidin-2-one;
5-(3-chloro-4-(2-chloro-3-(5-((((S)-2-hydroxypropyl)amino)methyl)-6-methoxypyridin-2-yl)phenyl)pyridin-2-yl)-2-(2-((((S)-5-oxopyrrolidin-2-yl)methyl)amino)ethyl)isoindolin-1-one;
5-(3-chloro-4-(2-chloro-3-(5-((((S)-2-hydroxypropyl)amino)methyl)-6-methoxypyridin-2-yl)phenyl)pyridin-2-yl)-2-(2-((((R)-5-oxopyrrolidin-2-yl)methyl)amino)ethyl)isoindolin-1-one;
5-(3-chloro-4-(2-chloro-3-(5-((((R)-2-hydroxypropyl)amino)methyl)-6-methoxypyridin-2-yl)phenyl)pyridin-2-yl)-2-(2-((((S)-5-oxopyrrolidin-2-yl)methyl)amino)ethyl)isoindolin-1-one;
5-(3-chloro-4-(2-chloro-3-(5-((((R)-2-hydroxypropyl)amino)methyl)-6-methoxypyridin-2-yl)phenyl)pyridin-2-yl)-2-(2-((((R)-5-oxopyrrolidin-2-yl)methyl)amino)ethyl)isoindolin-1-one;
(S)-5-(4-(3-(5-(((1-acetylpiperidin-4-yl)amino)methyl)-6-methoxypyridin-2-yl)-2-chlorophenyl)-3-chloropyridin-2-yl)-2-(2-(((5-oxopyrrolidin-2-yl)methyl)amino)ethyl)isoindolin-1-one;
(R)-5-(4-(3-(5-(((1-acetylpiperidin-4-yl)amino)methyl)-6-methoxypyridin-2-yl)-2-chlorophenyl)-3-chloropyridin-2-yl)-2-(2-(((5-oxopyrrolidin-2-yl)methyl)amino)ethyl)isoindolin-1-one;
(S)-5-(4-(3-(5-((6-acetyl-2,6-diazaspiro[3.3]heptan-2-yl)methyl)-6-methoxypyridin-2-yl)-2-chlorophenyl)-3-chloropyridin-2-yl)-2-(2-(((5-oxopyrrolidin-2-yl)methyl)amino)ethyl)isoindolin-1-one;
(R)-5-(4-(3-(5-((6-acetyl-2,6-diazaspiro[3.3]heptan-2-yl)methyl)-6-methoxypyridin-2-yl)-2-chlorophenyl)-3-chloropyridin-2-yl)-2-(2-(((5-oxopyrrolidin-2-yl)methyl)amino)ethyl)isoindolin-1-one;
(S)-2-(4-(3-chloro-4-(2-chloro-3-(6-methoxy-5-((((5-oxopyrrolidin-2-yl)methyl)amino)methyl)pyridin-2-yl)phenyl)pyridin-2-yl)-2-fluoro-6-methoxybenzyl)-2,6-diazaspiro[3.4]octan-7-one;
(R)-2-(4-(3-chloro-4-(2-chloro-3-(6-methoxy-5-((((5-oxopyrrolidin-2-yl)methyl)amino)methyl)pyridin-2-yl)phenyl)pyridin-2-yl)-2-fluoro-6-methoxybenzyl)-2,6-diazaspiro[3.4]octan-7-one;
(S)-5-((((6-(2-chloro-3-(3-chloro-2-(3-fluoro-4-((6-hydroxy-2-azaspiro[3.3]heptan-2-yl)methyl)-5-methoxyphenyl)pyridin-4-yl)phenyl)-2-methoxypyridin-3-yl)methyl)amino)methyl)pyrrolidin-2-one;
(R)-5-((((6-(2-chloro-3-(3-chloro-2-(3-fluoro-4-((6-hydroxy-2-azaspiro[3.3]heptan-2-yl)methyl)-5-methoxyphenyl)pyridin-4-yl)phenyl)-2-methoxypyridin-3-yl)methyl)amino)methyl)pyrrolidin-2-one;
(S)-5-((((6-(2-chloro-3-(3-chloro-2-(3-fluoro-4-(((3-fluoropropyl)amino)methyl)-5-methoxyphenyl)pyridin-4-yl)phenyl)-2-methoxypyridin-3-yl)methyl)amino)methyl)pyrrolidin-2-one;
(R)-5-((((6-(2-chloro-3-(3-chloro-2-(3-fluoro-4-(((3-fluoropropyl)amino)methyl)-5-methoxyphenyl)pyridin-4-yl)phenyl)-2-methoxypyridin-3-yl)methyl)amino)methyl)pyrrolidin-2-one;
(S)-5-((((6-(3-(2-(4-(((1-acetylpiperidin-4-yl)amino)methyl)-3-fluoro-5-methoxyphenyl)-3-chloropyridin-4-yl)-2-chlorophenyl)-2-methoxypyridin-3-yl)methyl)amino)methyl)pyrrolidin-2-one;
(R)-5-((((6-(3-(2-(4-(((1-acetylpiperidin-4-yl)amino)methyl)-3-fluoro-5-methoxyphenyl)-3-chloropyridin-4-yl)-2-chlorophenyl)-2-methoxypyridin-3-yl)methyl)amino)methyl)pyrrolidin-2-one;
methyl (S)-2-(6-(3-chloro-4-(2-chloro-3-(6-methoxy-5-((((5-oxopyrrolidin-2-yl)methyl)amino)methyl)pyridin-2-yl)phenyl)pyridin-2-yl)-8-methoxy-3,4-dihydroisoquinolin-2(1H)-yl)acetate;
methyl (R)-2-(6-(3-chloro-4-(2-chloro-3-(6-methoxy-5-((((5-oxopyrrolidin-2-yl)methyl)amino)methyl)pyridin-2-yl)phenyl)pyridin-2-yl)-8-methoxy-3,4-dihydroisoquinolin-2(1H)-yl)acetate;
(S)-2-(6-(3-chloro-4-(2-chloro-3-(6-methoxy-5-((((5-oxopyrrolidin-2-yl)methyl)amino)methyl)pyridin-2-yl)phenyl)pyridin-2-yl)-8-methoxy-3,4-dihydroisoquinolin-2(1H)-yl)acetic acid;
(R)-2-(6-(3-chloro-4-(2-chloro-3-(6-methoxy-5-((((5-oxopyrrolidin-2-yl)methyl)amino)methyl)pyridin-2-yl)phenyl)pyridin-2-yl)-8-methoxy-3,4-dihydroisoquinolin-2(1H)-yl)acetic acid;
(S)-5-((((6-(3-(2-(2-((1R,3S,4S)-2-azabicyclo[2.2.1]heptan-3-yl)-1H-benzo[d]imidazol-6-yl)-3-chloropyridin-4-yl)-2-chlorophenyl)-2-methoxypyridin-3-yl)methyl)amino)methyl)pyrrolidin-2-one;
(R)-5-((((6-(3-(2-(2-((1R,3S,4S)-2-azabicyclo[2.2.1]heptan-3-yl)-1H-benzo[d]imidazol-6-yl)-3-chloropyridin-4-yl)-2-chlorophenyl)-2-methoxypyridin-3-yl)methyl)amino)methyl)pyrrolidin-2-one;
(S)-5-((((6-(3-(2-(2-((1R,3R,4S)-2-azabicyclo[2.2.1]heptan-3-yl)-1H-benzo[d]imidazol-6-yl)-3-chloropyridin-4-yl)-2-chlorophenyl)-2-methoxypyridin-3-yl)methyl)amino)methyl)pyrrolidin-2-one;
(R)-5-((((6-(3-(2-(2-((1R,3R,4S)-2-azabicyclo[2.2.1]heptan-3-yl)-1H-benzo[d]imidazol-6-yl)-3-chloropyridin-4-yl)-2-chlorophenyl)-2-methoxypyridin-3-yl)methyl)amino)methyl)pyrrolidin-2-one;
(S)-5-((((6-(3-(2-(2-((1S,3S,4R)-2-azabicyclo[22.1]heptan-3-yl)-1H-benzo[d]imidazol-6-yl)-3-chloropyridin-4-yl)-2-chlorophenyl)-2-methoxypyridin-3-yl)methyl)amino)methyl)pyrrolidin-2-one;
(R)-5-((((6-(3-(2-(2-((1S,3 S,4R)-2-azabicyclo[2.2.1]heptan-3-yl)-1H-benzo[d]imidazol-6-yl)-3-chloropyridin-4-yl)-2-chlorophenyl)-2-methoxypyridin-3-yl)methyl)amino)methyl)pyrrolidin-2-one;
(S)-5-((((6-(3-(2-(2-((1S,3R,4R)-2-azabicyclo[2.2.1]heptan-3-yl)-1H-benzo[d]imidazol-6-yl)-3-chloropyridin-4-yl)-2-chlorophenyl)-2-methoxypyridin-3-yl)methyl)amino)methyl)pyrrolidin-2-one;
(R)-5-((((6-(3-(2-(2-((1S,3R,4R)-2-azabicyclo[22.1]heptan-3-yl)-1H-benzo[d]imidazol-6-yl)-3-chloropyridin-4-yl)-2-chlorophenyl)-2-methoxypyridin-3-yl)methyl)amino)methyl)pyrrolidin-2-one;
(S)-5-((((6-(2-chloro-3-(3-chloro-2-(3-methoxy-5-(((((S)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
(S)-5-((((6-(2-chloro-3-(3-chloro-2-(3-methoxy-5-(((((R)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
(R)-5-((((6-(2-chloro-3-(3-chloro-2-(3-methoxy-5-(((((S)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
(R)-5-((((6-(2-chloro-3-(3-chloro-2-(3-methoxy-5-(((((R)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
1-(4-(((6-(3-(2-(3-(((1-acetylpiperidin-4-yl)amino)methyl)-5-methoxyphenyl)-3-chloropyridin-4-yl)-2-chlorophenyl)-2-methoxypyridin-3-yl)methyl)amino)piperidin-1-yl)ethan-1-one;
1-(6-(2-chloro-3-(3-chloro-2-(3-methoxy-5-((methylamino)methyl)phenyl)pyridin-4-yl)phenyl)-2-methoxypyridin-3-yl)-N-methylmethanamine;
chloro-3-(3-chloro-2-(3-(((2-hydroxyethyl)amino)methyl)-5-methoxyphenyl)pyridin-4-yl)phenyl)-2-methoxypyridin-3-yl)methyl)amino)ethan-1-ol;
(S)-1-(((6-(2-chloro-3-(3-chloro-2-(3-((((S)-2-hydroxypropyl)amino)methyl)-5-methoxyphenyl)pyridin-4-yl)phenyl)-2-methoxypyridin-3-yl)methyl)amino)propan-2-ol;
(S)-1-(((6-(2-chloro-3-(3-chloro-2-(3-((((R)-2-hydroxypropyl)amino)methyl)-5-methoxyphenyl)pyridin-4-yl)phenyl)-2-methoxypyridin-3-yl)methyl)amino)propan-2-ol;
(R)-1-(((6-(2-chloro-3-(3-chloro-2-(3-((((S)-2-hydroxypropyl)amino)methyl)-5-methoxyphenyl)pyridin-4-yl)phenyl)-2-methoxypyridin-3-yl)methyl)amino)propan-2-ol;
(R)-1-(((6-(2-chloro-3-(3-chloro-2-(3-((((R)-2-hydroxypropyl)amino)methyl)-5-methoxyphenyl)pyridin-4-yl)phenyl)-2-methoxypyridin-3-yl)methyl)amino)propan-2-ol;
(S)-5-((((3'-chloro-2'-((S)-1-((5-((((S)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)methyl)pyrrolidin-2-one;
(S)-5-((((3'-chloro-2'-((S)-1-((5-((((R)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)methyl)pyrrolidin-2-one;
(S)-5-((((3'-chloro-2'-((R)-1-((5-((((S)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)methyl)pyrrolidin-2-one;
(R)-5-((((3'-chloro-2'-((S)-1-((5-((((S)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)methyl)pyrrolidin-2-one;
(S)-5-((((3'-chloro-2'-((R)-1-((5-((((R)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)methyl)pyrrolidin-2-one;
(R)-5-((((3'-chloro-2'-((S)-1-((5-((((R)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)methyl)pyrrolidin-2-one;
(R)-5-((((3'-chloro-2'-((R)-1-((5-((((S)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)methyl)pyrrolidin-2-one;
(R)-5-((((3'-chloro-2'-((R)-1-((5-((((R)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)methyl)pyrrolidin-2-one;
1-(((6-(((S)-4-(3'-chloro-6-methoxy-5-(((((S)-5-oxopyrrolidin-2-yl)methyl)amino)methyl)-[2,4'-bipyridin]-2'-yl)-2,3-dihydro-1H-inden-1-yl)oxy)-2-methoxy-5-(trifluoromethyl)pyridin-3-yl)methyl)amino)cyclopropane-1-carboxylic acid;
1-(((6-(((S)-4-(3'-chloro-6-methoxy-5-(((((R)-5-oxopyrrolidin-2-yl)methyl)amino)methyl)-[2,4'-bipyridin]-2'-yl)-2,3-dihydro-1H-inden-1-yl)oxy)-2-methoxy-5-(trifluoromethyl)pyridin-3-yl)methyl)amino)cyclopropane-1-carboxylic acid;
1-(((6-(((R)-4-(3'-chloro-6-methoxy-5-(((((S)-5-oxopyrrolidin-2-yl)methyl)amino)methyl)-[2,4'-bipyridin]-2'-yl)-2,3-dihydro-1H-inden-1-yl)oxy)-2-methoxy-5-(trifluoromethyl)pyridin-3-yl)methyl)amino)cyclopropane-1-carboxylic acid;
1-(((6-(((R)-4-(3'-chloro-6-methoxy-5-(((((R)-5-oxopyrrolidin-2-yl)methyl)amino)methyl)-[2,4'-bipyridin]-2'-yl)-2,3-dihydro-1H-inden-1-yl)oxy)-2-methoxy-5-(trifluoromethyl)pyridin-3-yl)methyl)amino)cyclopropane-1-carboxylic acid;
methyl 1-(((6-(((S)-4-(3'-chloro-6-methoxy-5-(((((S)-5-oxopyrrolidin-2-yl)methyl)amino)methyl)-[2,4'-bipyridin]-2'-yl)-2,3-dihydro-1H-inden-1-yl)amino)-2-methoxy-5-(trifluoromethyl)pyridin-3-yl)methyl)amino)cyclopropane-1-carboxylate;
methyl 1-(((6-(((S)-4-(3'-chloro-6-methoxy-5-(((((R)-5-oxopyrrolidin-2-yl)methyl)amino)methyl)-[2,4'-bipyridin]-2'-yl)-2,3-dihydro-1H-inden-1-yl)amino)-2-methoxy-5-(trifluoromethyl)pyridin-3-yl)methyl)amino)cyclopropane-1-carboxylate;
methyl 1-(((6-(((R)-4-(3'-chloro-6-methoxy-5-(((((S)-5-oxopyrrolidin-2-yl)methyl)amino)methyl)-[2,4'-bipyridin]-2'-yl)-2,3-dihydro-1H-inden-1-yl)amino)-2-methoxy-5-(trifluoromethyl)pyridin-3-yl)methyl)amino)cyclopropane-1-carboxylate;
methyl 1-(((6-(((R)-4-(3'-chloro-6-methoxy-5-(((((R)-5-oxopyrrolidin-2-yl)methyl)amino)methyl)-[2,4'-bipyridin]-2'-yl)-2,3-dihydro-1H-inden-1-yl)amino)-2-methoxy-5-(trifluoromethyl)pyridin-3-yl)methyl)amino)cyclopropane-1-carboxylate;
(S)-5-((((6-(2-chloro-3-(3-chloro-2-(2-(2-hydroxyethyl)-5-methyl-2,3,4,5-tetrahydro-1H-pyrido[4,3-b]indol-7-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-(2-hydroxyethyl)-5-methyl-2,3,4,5-tetrahydro-1H-pyrido[4,3-b]indol-7-yl)pyridin-4-yl)phenyl)-2-methoxypyridin-3-yl)methyl)amino)methyl)pyrrolidin-2-one;
1-(6-(2-chloro-3-(3-chloro-2-(isoindolin-5-yl)pyridin-4-yl)phenyl)-2-methoxypyridin-3-yl)-N-methylmethanamine;
2-(((6-(2-chloro-3-(3-chloro-2-(isoindolin-5-yl)pyridin-4-yl)phenyl)-2-methoxypyridin-3-yl)methyl)amino)ethan-1-ol;
(S)-1-(((6-(2-chloro-3-(3-chloro-2-(isoindolin-5-yl)pyridin-4-yl)phenyl)-2-methoxypyridin-3-yl)methyl)amino)propan-2-ol;
(R)-1-(((6-(2-chloro-3-(3-chloro-2-(isoindolin-5-yl)pyridin-4-yl)phenyl)-2-methoxypyridin-3-yl)methyl)amino)propan-2-ol;
1-(4-(((6-(2-chloro-3-(3-chloro-2-(isoindolin-5-yl)pyridin-4-yl)phenyl)-2-methoxypyridin-3-yl)methyl)amino)piperidin-1-yl)ethan-1-one;
1-(4-(((6-(2-chloro-3-(3-chloro-2-(isoindolin-5-yl)pyridin-4-yl)phenyl)-2-methoxypyridin-3-yl)methyl)(methyl)amino)piperidin-1-yl)ethan-1-one;
1-(((6-(((S)-4-(3'-chloro-6-methoxy-5-(((((S)-5-oxopyrrolidin-2-yl)methyl)amino)methyl)-[2,4'-bipyridin]-2'-yl)-2,3-dihydro-1H-inden-1-yl)amino)-2-methoxy-5-(trifluoromethyl)pyridin-3-yl)methyl)amino)cyclopropane-1-carboxylic acid;
1-(((6-(((S)-4-(3'-chloro-6-methoxy-5-(((((R)-5-oxopyrrolidin-2-yl)methyl)amino)methyl)-[2,4'-bipyridin]-2'-yl)-2,3-dihydro-1H-inden-1-yl)amino)-2-methoxy-5-(trifluoromethyl)pyridin-3-yl)methyl)amino)cyclopropane-1-carboxylic acid;
1-(((6-(((R)-4-(3'-chloro-6-methoxy-5-(((((S)-5-oxopyrrolidin-2-yl)methyl)amino)methyl)-[2,4'-bipyridin]-2'-yl)-2,3-dihydro-1H-inden-1-yl)amino)-2-methoxy-5-(trifluoromethyl)pyridin-3-yl)methyl)amino)cyclopropane-1-carboxylic acid;
1-(((6-(((R)-4-(3'-chloro-6-methoxy-5-(((((R)-5-oxopyrrolidin-2-yl)methyl)amino)methyl)-[2,4'-bipyridin]-2'-yl)-2,3-dihydro-1H-inden-1-yl)amino)-2-methoxy-5-(trifluoromethyl)pyridin-3-yl)methyl)amino)cyclopropane-1-carboxylic acid;
(S)-5-((((3'-chloro-2'-((S)-1-((5-((((R)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)methyl)pyrrolidin-2-one;
(S)-5-((((3'-chloro-2'-((S)-1-((5-((((S)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)methyl)pyrrolidin-2-one;
(S)-5-((((3'-chloro-2'-((R)-1-((5-((((S)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)methyl)pyrrolidin-2-one;
(S)-5-((((3'-chloro-2'-((R)-1-((5-((((R)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)methyl)pyrrolidin-2-one;
(R)-5-((((3'-chloro-2'-((R)-1-((5-((((R)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)methyl)pyrrolidin-2-one;
(R)-5-((((3'-chloro-2'-((S)-1-((5-((((S)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)methyl)pyrrolidin-2-one;
(R)-5-((((3'-chloro-2'-((S)-1-((5-((((R)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)methyl)pyrrolidin-2-one;
(R)-5-((((3'-chloro-2'-((R)-1-((5-((((S)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)methyl)pyrrolidin-2-one;
(S)-5-((((3'-chloro-6-methoxy-2'-((S)-1-((6-methoxy-5-(((((S)-5-oxopyrrolidin-2-yl)methyl)amino)methyl)-3-(trifluoromethyl)pyridin-2-yl)oxy)-2,3-dihydro-1H-inden-4-yl)-[2,4'-bipyridin]-5-yl)methyl)amino)methyl)pyrrolidin-2-one;
(S)-5-((((3'-chloro-6-methoxy-2'-((S)-1-((6-methoxy-5-(((((R)-5-oxopyrrolidin-2-yl)methyl)amino)methyl)-3-(trifluoromethyl)pyridin-2-yl)oxy)-2,3-dihydro-1H-inden-4-yl)-[2,4'-bipyridin]-5-yl)methyl)amino)methyl)pyrrolidin-2-one;
(S)-5-((((3'-chloro-6-methoxy-2'-((R)-1-((6-methoxy-5-(((((R)-5-oxopyrrolidin-2-yl)methyl)amino)methyl)-3-(trifluoromethyl)pyridin-2-yl)oxy)-2,3-dihydro-1H-inden-4-yl)-[2,4'-bipyridin]-5-yl)methyl)amino)methyl)pyrrolidin-2-one;
(S)-5-((((3'-chloro-6-methoxy-2'-((R)-1-((6-methoxy-5-(((((R)-5-oxopyrrolidin-2-yl)methyl)amino)methyl)-3-(trifluoromethyl)pyridin-2-yl)oxy)-2,3-dihydro-1H-inden-4-yl)-[2,4'-bipyridin]-5-yl)methyl)amino)methyl)pyrrolidin-2-one;
(R)-5-((((3'-chloro-6-methoxy-2'-((R)-1-((6-methoxy-5-(((((R)-5-oxopyrrolidin-2-yl)methyl)amino)methyl)-3-(trifluoromethyl)pyridin-2-yl)oxy)-2,3-dihydro-1H-inden-4-yl)-[2,4'-bipyridin]-5-yl)methyl)amino)methyl)pyrrolidin-2-one;
(R)-5-((((3'-chloro-6-methoxy-2'-((S)- 1-((6-methoxy-5-(((((S)-5-oxopyrrolidin-2-yl)methyl)amino)methyl)-3-(trifluoromethyl)pyridin-2-yl)oxy)-2,3-dihydro-1H-inden-4-yl)-[2,4'-bipyridin]-5-yl)methyl)amino)methyl)pyrrolidin-2-one;
(R)-5-((((3'-chloro-6-methoxy-2'-((S)-1-((6-methoxy-5-(((((R)-5-oxopyrrolidin-2-yl)methyl)amino)methyl)-3-(trifluoromethyl)pyridin-2-yl)oxy)-2,3-dihydro-1H-inden-4-yl)-[2,4'-bipyridin]-5-yl)methyl)amino)methyl)pyrrolidin-2-one;
(R)-5-((((3'-chloro-6-methoxy-2'-((R)-1-((6-methoxy-5-(((((S)-5-oxopyrrolidin-2-yl)methyl)amino)methyl)-3-(trifluoromethyl)pyridin-2-yl)oxy)-2,3-dihydro-1H-inden-4-yl)-[2,4'-bipyridin]-5-yl)methyl)amino)methyl)pyrrolidin-2-one;
(S)-5-((((6-(3-(2-(3-(((1-acetylpiperidin-4-yl)amino)methyl)-1-methyl-1H-indazol-6-yl)-3-chloropyridin-4-yl)-2-chlorophenyl)-2-methoxypyridin-3-yl)methyl)amino)methyl)pyrrolidin-2-one;
(R)-5-((((6-(3-(2-(3-(((1-acetylpiperidin-4-yl)amino)methyl)-1-methyl-1H-indazol-6-yl)-3-chloropyridin-4-yl)-2-chlorophenyl)-2-methoxypyridin-3-yl)methyl)amino)methyl)pyrrolidin-2-one;
(S)-5-((((6-(3-(2-(3-(((1-acetylpiperidin-4-yl)(methyl)amino)methyl)-1-methyl-1H-indazol-6-yl)-3-chloropyridin-4-yl)-2-chlorophenyl)-2-methoxypyridin-3-yl)methyl)amino)methyl)pyrrolidin-2-one;
(R)-5-((((6-(3-(2-(3-(((1-acetylpiperidin-4-yl)(methyl)amino)methyl)-1-methyl-1H-indazol-6-yl)-3-chloropyridin-4-yl)-2-chlorophenyl)-2-methoxypyridin-3-yl)methyl)amino)methyl)pyrrolidin-2-one;
(S)-4-((3-(3-chloro-4-(2-chloro-3-(6-methoxy-5-((methylamino)methyl)pyridin-2-yl)phenyl)pyridin-2-yl)-5-methoxybenzyl)amino)-3-hydroxybutanoic acid;
(R)-4-((3-(3-chloro-4-(2-chloro-3-(6-methoxy-5-((methylamino)methyl)pyridin-2-yl)phenyl)pyridin-2-yl)-5-methoxybenzyl)amino)-3-hydroxybutanoic acid;
3-((3-(3-chloro-4-(2-chloro-3-(6-methoxy-5-((methylamino)methyl)pyridin-2-yl)phenyl)pyridin-2-yl)-5-methoxybenzyl)amino)propanoic acid;
(S)-1-(3-(3-chloro-4-(2-chloro-3-(6-methoxy-5-((methylamino)methyl)pyridin-2-yl)phenyl)pyridin-2-yl)-5-methoxybenzyl)pyrrolidine-3-carboxylic acid;
(R)-1-(3-(3-chloro-4-(2-chloro-3-(6-methoxy-5-((methylamino)methyl)pyridin-2-yl)phenyl)pyridin-2-yl)-5-methoxybenzyl)pyrrolidine-3-carboxylic acid;
2-(1-(3-(3-chloro-4-(2-chloro-3-(6-methoxy-5-((methylamino)methyl)pyridin-2-yl)phenyl)pyridin-2-yl)-5-methoxybenzyl)azetidin-3-yl)acetic acid;
2-(3-(3-chloro-4-(2-chloro-3-(6-methoxy-5-((methylamino)methyl)pyridin-2-yl)phenyl)pyridin-2-yl)-5-methoxybenzyl)-2-azaspiro[3.3]heptane-6-carboxylic acid;
3-((3-(3-chloro-4-(2-chloro-3-(6-methoxy-5-((methylamino)methyl)pyridin-2-yl)phenyl)pyridin-2-yl)-5-methoxybenzyl)amino)bicyclo[1.1.1]pentane-1-carboxylic acid;
3-(((3-(3-chloro-4-(2-chloro-3-(6-methoxy-5-((methylamino)methyl)pyridin-2-yl)phenyl)pyridin-2-yl)-5-methoxybenzyl)amino)methyl)bicyclo[1.1.1]pentane-1-carboxylic acid;
(S)-5-((((6-(2-chloro-3-(3-chloro-2-(2-((S)-2-hydroxypropyl)-8-methoxy-1,2,3,4-tetrahydroisoquinolin-6-yl)pyridin-4-yl)phenyl)-4-fluoro-2-methoxypyridin-3-yl)methyl)amino)methyl)pyrrolidin-2-one;
(S)-5-((((6-(2-chloro-3-(3-chloro-2-(2-((R)-2-hydroxypropyl)-8-methoxy-1,2,3,4-tetrahydroisoquinolin-6-yl)pyridin-4-yl)phenyl)-4-fluoro-2-methoxypyridin-3-yl)methyl)amino)methyl)pyrrolidin-2-one;
(R)-5-((((6-(2-chloro-3-(3-chloro-2-(2-((S)-2-hydroxypropyl)-8-methoxy-1,2,3,4-tetrahydroisoquinolin-6-yl)pyridin-4-yl)phenyl)-4-fluoro-2-methoxypyridin-3-yl)methyl)amino)methyl)pyrrolidin-2-one;
(R)-5-((((6-(2-chloro-3-(3-chloro-2-(2-((R)-2-hydroxypropyl)-8-methoxy-1,2,3,4-tetrahydroisoquinolin-6-yl)pyridin-4-yl)phenyl)-4-fluoro-2-methoxypyridin-3-yl)methyl)amino)methyl)pyrrolidin-2-one;
(S)-5-((((6-(2-chloro-3-(3-chloro-2-(1,1-dioxido-2,3,4,5-tetrahydrobenzo[f][1,4]thiazepin-8-yl)pyridin-4-yl)phenyl)-2-methoxypyridin-3-yl)methyl)amino)methyl)pyrrolidin-2-one;
(R)-5-((((6-(2-chloro-3-(3-chloro-2-(1,1-dioxido-2,3,4,5-tetrahydrobenzo[f][1,4]thiazepin-8-yl)pyridin-4-yl)phenyl)-2-methoxypyridin-3-yl)methyl)amino)methyl)pyrrolidin-2-one;
5-(3-chloro-4-(2-chloro-3-(4-fluoro-6-methoxy-5-(((((S)-5-oxopyrrolidin-2-yl)methyl)amino)methyl)pyridin-2-yl)phenyl)pyridin-2-yl)-2-(2-((((S)-5-oxopyrrolidin-2-yl)methyl)amino)ethyl)isoindolin-1-one;
5-(3-chloro-4-(2-chloro-3-(4-fluoro-6-methoxy-5-(((((S)-5-oxopyrrolidin-2-yl)methyl)amino)methyl)pyridin-2-yl)phenyl)pyridin-2-yl)-2-(2-((((R)-5-oxopyrrolidin-2-yl)methyl)amino)ethyl)isoindolin-1-one;
5-(3-chloro-4-(2-chloro-3-(4-fluoro-6-methoxy-5-(((((R)-5-oxopyrrolidin-2-yl)methyl)amino)methyl)pyridin-2-yl)phenyl)pyridin-2-yl)-2-(2-((((S)-5-oxopyrrolidin-2-yl)methyl)amino)ethyl)isoindolin-1-one;
5-(3-chloro-4-(2-chloro-3-(4-fluoro-6-methoxy-5-(((((R)-5-oxopyrrolidin-2-yl)methyl)amino)methyl)pyridin-2-yl)phenyl)pyridin-2-yl)-2-(2-((((R)-5-oxopyrrolidin-2-yl)methyl)amino)ethyl)isoindolin-1-one;
(S)-5-((((6-(2-chloro-3-(3-chloro-2-(4-(4,5-dihydro-1H-imidazol-2-yl)-3-methoxyphenyl)pyridin-4-yl)phenyl)-2-methoxypyridin-3-yl)methyl)amino)methyl)pyrrolidin-2-one;
(R)-5-((((6-(2-chloro-3-(3-chloro-2-(4-(4,5-dihydro-1H-imidazol-2-yl)-3-methoxyphenyl)pyridin-4-yl)phenyl)-2-methoxypyridin-3-yl)methyl)amino)methyl)pyrrolidin-2-one;
(S)-4-((4-(3-chloro-4-(2-chloro-3-(5-(4,5-dihydro-1H-imidazol-2-yl)-6-methoxypyridin-2-yl)phenyl)pyridin-2-yl)-2-methoxybenzyl)amino)-3-hydroxybutanoic acid;
(R)-4-((4-(3-chloro-4-(2-chloro-3-(5-(4,5-dihydro-1H-imidazol-2-yl)-6-methoxypyridin-2-yl)phenyl)pyridin-2-yl)-2-methoxybenzyl)amino)-3-hydroxybutanoic acid;
(S)-5-((((6-(2-chloro-3-(3-chloro-2-(3-fluoro-5-methoxy-4-(((((S)-oxetan-2-yl)methyl)amino)methyl)phenyl)pyridin-4-yl)phenyl)-2-methoxypyridin-3-yl)methyl)amino)methyl)pyrrolidin-2-one;
(S)-5-((((6-(2-chloro-3-(3-chloro-2-(3-fluoro-5-methoxy-4-(((((R)-oxetan-2-yl)methyl)amino)methyl)phenyl)pyridin-4-yl)phenyl)-2-methoxypyridin-3-yl)methyl)amino)methyl)pyrrolidin-2-one;
(R)-5-((((6-(2-chloro-3-(3-chloro-2-(3-fluoro-5-methoxy-4-(((((S)-oxetan-2-yl)methyl)amino)methyl)phenyl)pyridin-4-yl)phenyl)-2-methoxypyridin-3-yl)methyl)amino)methyl)pyrrolidin-2-one;
(S)-5-((((6-(2-chloro-3-(3-chloro-2-(3-fluoro-5-methoxy-4-(((((S)-oxetan-2-yl)methyl)amino)methyl)phenyl)pyridin-4-yl)phenyl)-2-methoxypyridin-3-yl)methyl)amino)methyl)pyrrolidin-2-one;
methyl (S)-1-((6-(3-chloro-4-(2-chloro-3-(6-methoxy-5-((((5-oxopyrrolidin-2-yl)methyl)amino)methyl)pyridin-2-yl)phenyl)pyridin-2-yl)-3-methyl-4-oxo-3,4-dihydropyrrolo[2,1-f][1,2,4]triazin-2-yl)methyl)azetidine-3-carboxylate;
methyl (R)-1-((6-(3-chloro-4-(2-chloro-3-(6-methoxy-5-((((5-oxopyrrolidin-2-yl)methyl)amino)methyl)pyridin-2-yl)phenyl)pyridin-2-yl)-3-methyl-4-oxo-3,4-dihydropyrrolo[2,1-f][1,2,4]triazin-2-yl)methyl)azetidine-3-carboxylate;
(S)-5-((((6-(2-chloro-3-(3-chloro-2-(1-methyl-3-(((((S)-5-oxopyrrolidin-2-yl)methyl)amino)methyl)-1H-indazol-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(1-methyl-3-(((((S)-5-oxopyrrolidin-2-yl)methyl)amino)methyl)-1H-indazol-6-yl)pyridin-4-yl)phenyl)-2-methoxypyridin-3-yl)methyl)amino)methyl)pyrrolidin-2-one;
(S)-5-((((6-(2-chloro-3-(3-chloro-2-((S)-5-((((S)-5-oxopyrrolidin-2-yl)methyl)amino)-5,6,7,8-tetrahydronaphthalen-2-yl)pyridin-4-yl)phenyl)-2-methoxypyridin-3-yl)methyl)amino)methyl)pyrrolidin-2-one;
(S)-5-((((6-(2-chloro-3-(3-chloro-2-((S)-5-((((R)-5-oxopyrrolidin-2-yl)methyl)amino)-5,6,7,8-tetrahydronaphthalen-2-yl)pyridin-4-yl)phenyl)-2-methoxypyridin-3-yl)methyl)amino)methyl)pyrrolidin-2-one;
(S)-5-((((6-(2-chloro-3-(3-chloro-2-((R)-5-((((S)-5-oxopyrrolidin-2-yl)methyl)amino)-5,6,7,8-tetrahydronaphthalen-2-yl)pyridin-4-yl)phenyl)-2-methoxypyridin-3-yl)methyl)amino)methyl)pyrrolidin-2-one;
(S)-5-((((6-(2-chloro-3-(3-chloro-2-((R)-5-((((R)-5-oxopyrrolidin-2-yl)methyl)amino)-5,6,7,8-tetrahydronaphthalen-2-yl)pyridin-4-yl)phenyl)-2-methoxypyridin-3-yl)methyl)amino)methyl)pyrrolidin-2-one;
(R)-5-((((6-(2-chloro-3-(3-chloro-2-((R)-5-((((R)-5-oxopyrrolidin-2-yl)methyl)amino)-5,6,7,8-tetrahydronaphthalen-2-yl)pyridin-4-yl)phenyl)-2-methoxypyridin-3-yl)methyl)amino)methyl)pyrrolidin-2-one;
(R)-5-((((6-(2-chloro-3-(3-chloro-2-((R)-5-((((S)-5-oxopyrrolidin-2-yl)methyl)amino)-5,6,7,8-tetrahydronaphthalen-2-yl)pyridin-4-yl)phenyl)-2-methoxypyridin-3-yl)methyl)amino)methyl)pyrrolidin-2-one;
(R)-5-((((6-(2-chloro-3-(3-chloro-2-((R)-5-((((R)-5-oxopyrrolidin-2-yl)methyl)amino)-5,6,7,8-tetrahydronaphthalen-2-yl)pyridin-4-yl)phenyl)-2-methoxypyridin-3-yl)methyl)amino)methyl)pyrrolidin-2-one;
(R)-5-((((6-(2-chloro-3-(3-chloro-2-((S)-5-((((S)-5-oxopyrrolidin-2-yl)methyl)amino)-5,6,7,8-tetrahydronaphthalen-2-yl)pyridin-4-yl)phenyl)-2-methoxypyridin-3-yl)methyl)amino)methyl)pyrrolidin-2-one;
1-(((6-(((S)-5-(3'-chloro-6-methoxy-5-(((((S)-5-oxopyrrolidin-2-yl)methyl)amino)methyl)-[2,4'-bipyridin]-2'-yl)-1,2,3,4-tetrahydronaphthalen-1-yl)amino)-2-methoxy-5-(trifluoromethyl)pyridin-3-yl)methyl)amino)cyclopropane-1-carboxylic acid;
1-(((6-(((S)-5-(3'-chloro-6-methoxy-5-(((((R)-5-oxopyrrolidin-2-yl)methyl)amino)methyl)-[2,4'-bipyridin]-2'-yl)-1,2,3,4-tetrahydronaphthalen-1-yl)amino)-2-methoxy-5-(trifluoromethyl)pyridin-3-yl)methyl)amino)cyclopropane-1-carboxylic acid;
1-(((6-(((R)-5-(3'-chloro-6-methoxy-5-(((((S)-5-oxopyrrolidin-2-yl)methyl)amino)methyl)-[2,4'-bipyridin]-2'-yl)-1,2,3,4-tetrahydronaphthalen-1-yl)amino)-2-methoxy-5-(trifluoromethyl)pyridin-3-yl)methyl)amino)cyclopropane-1-carboxylic acid;
1-(((6-(((R)-5-(3'-chloro-6-methoxy-5-(((((R)-5-oxopyrrolidin-2-yl)methyl)amino)methyl)-[2,4'-bipyridin]-2'-yl)-1,2,3,4-tetrahydronaphthalen-1-yl)amino)-2-methoxy-5-(trifluoromethyl)pyridin-3-yl)methyl)amino)cyclopropane-1-carboxylic acid;
(S)-5-((((3'-chloro-2'-((S)-5-((5-((((S)-2-hydroxypropyl)amino)methyl)-6-methoxy-3-(trifluoromethyl)pyridin-2-yl)amino)-5,6,7,8-tetrahydronaphthalen-1-yl)-6-methoxy-[2,4'-bipyridin]-5-yl)methyl)amino)methyl)pyrrolidin-2-one;
(S)-5-((((3'-chloro-2'-((S)-5-((5-((((R)-2-hydroxypropyl)amino)methyl)-6-methoxy-3-(trifluoromethyl)pyridin-2-yl)amino)-5,6,7,8-tetrahydronaphthalen-1-yl)-6-methoxy-[2,4'-bipyridin]-5-yl)methyl)amino)methyl)pyrrolidin-2-one;
(S)-5-((((3'-chloro-2'-((R)-5-((5-((((S)-2-hydroxypropyl)amino)methyl)-6-methoxy-3-(trifluoromethyl)pyridin-2-yl)amino)-5,6,7,8-tetrahydronaphthalen-1-yl)-6-methoxy-[2,4'-bipyridin]-5-yl)methyl)amino)methyl)pyrrolidin-2-one;
(S)-5-((((3'-chloro-2'-((R)-5-((5-((((R)-2-hydroxypropyl)amino)methyl)-6-methoxy-3-(trifluoromethyl)pyridin-2-yl)amino)-5,6,7,8-tetrahydronaphthalen-1-yl)-6-methoxy-[2,4'-bipyridin]-5-yl)methyl)amino)methyl)pyrrolidin-2-one;
(R)-5-((((3'-chloro-2'-((R)-5-((5-((((R)-2-hydroxypropyl)amino)methyl)-6-methoxy-3-(trifluoromethyl)pyridin-2-yl)amino)-5,6,7,8-tetrahydronaphthalen-1-yl)-6-methoxy-[2,4'-bipyridin]-5-yl)methyl)amino)methyl)pyrrolidin-2-one;
(R)-5-((((3'-chloro-2'-((R)-5-((5-((((S)-2-hydroxypropyl)amino)methyl)-6-methoxy-3-(trifluoromethyl)pyridin-2-yl)amino)-5,6,7,8-tetrahydronaphthalen-1-yl)-6-methoxy-[2,4'-bipyridin]-5-yl)methyl)amino)methyl)pyrrolidin-2-one;
(R)-5-((((3'-chloro-2'-((S)-5-((5-((((R)-2-hydroxypropyl)amino)methyl)-6-methoxy-3-(trifluoromethyl)pyridin-2-yl)amino)-5,6,7,8-tetrahydronaphthalen-1-yl)-6-methoxy-[2,4'-bipyridin]-5-yl)methyl)amino)methyl)pyrrolidin-2-one;
(R)-5-((((3'-chloro-2'-((S)-5-((5-((((S)-2-hydroxypropyl)amino)methyl)-6-methoxy-3-(trifluoromethyl)pyridin-2-yl)amino)-5,6,7,8-tetrahydronaphthalen-1-yl)-6-methoxy-[2,4'-bipyridin]-5-yl)methyl)amino)methyl)pyrrolidin-2-one;
(S)-5-((((3'-chloro-6-methoxy-2'-((S)-5-((6-methoxy-5-(((((S)-5-oxopyrrolidin-2-yl)methyl)amino)methyl)-3-(trifluoromethyl)pyridin-2-yl)amino)-5,6,7,8-tetrahydronaphthalen-1-yl)-[2,4'-bipyridin]-5-yl)methyl)amino)methyl)pyrrolidin-2-one;
(S)-5-((((3'-chloro-6-methoxy-2'-((S)-5-((6-methoxy-5-(((((R)-5-oxopyrrolidin-2-yl)methyl)amino)methyl)-3-(trifluoromethyl)pyridin-2-yl)amino)-5,6,7,8-tetrahydronaphthalen-1-yl)-[2,4'-bipyridin]-5-yl)methyl)amino)methyl)pyrrolidin-2-one;
(S)-5-((((3'-chloro-6-methoxy-2'-((R)-5-((6-methoxy-5-(((((S)-5-oxopyrrolidin-2-yl)methyl)amino)methyl)-3-(trifluoromethyl)pyridin-2-yl)amino)-5,6,7,8-tetrahydronaphthalen-1-yl)-[2,4'-bipyridin]-5-yl)methyl)amino)methyl)pyrrolidin-2-one;
(S)-5-((((3'-chloro-6-methoxy-2'-((R)-5-((6-methoxy-5-(((((R)-5-oxopyrrolidin-2-yl)methyl)amino)methyl)-3-(trifluoromethyl)pyridin-2-yl)amino)-5,6,7,8-tetrahydronaphthalen-1-yl)-[2,4'-bipyridin]-5-yl)methyl)amino)methyl)pyrrolidin-2-one;
(R)-5-((((3'-chloro-6-methoxy-2'-((R)-5-((6-methoxy-5-(((((R)-5-oxopyrrolidin-2-yl)methyl)amino)methyl)-3-(trifluoromethyl)pyridin-2-yl)amino)-5,6,7,8-tetrahydronaphthalen-1-yl)-[2,4'-bipyridin]-5-yl)methyl)amino)methyl)pyrrolidin-2-one;
(R)-5-((((3'-chloro-6-methoxy-2'-((R)-5-((6-methoxy-5-(((((S)-5-oxopyrrolidin-2-yl)methyl)amino)methyl)-3-(trifluoromethyl)pyridin-2-yl)amino)-5,6,7,8-tetrahydronaphthalen-1-yl)-[2,4'-bipyridin]-5-yl)methyl)amino)methyl)pyrrolidin-2-one;
(R)-5-((((3'-chloro-6-methoxy-2'-((S)-5-((6-methoxy-5-(((((R)-5-oxopyrrolidin-2-yl)methyl)amino)methyl)-3-(trifluoromethyl)pyridin-2-yl)amino)-5,6,7,8-tetrahydronaphthalen-1-yl)-[2,4'-bipyridin]-5-yl)methyl)amino)methyl)pyrrolidin-2-one;
(R)-5-((((3'-chloro-6-methoxy-2'-((S)-5-((6-methoxy-5-(((((S)-5-oxopyrrolidin-2-yl)methyl)amino)methyl)-3-(trifluoromethyl)pyridin-2-yl)amino)-5,6,7,8-tetrahydronaphthalen-1-yl)-[2,4'-bipyridin]-5-yl)methyl)amino)methyl)pyrrolidin-2-one;
2-((6-(((S)-5-(3'-chloro-6-methoxy-5-(((((S)-5-oxopyrrolidin-2-yl)methyl)amino)methyl)-[2,4'-bipyridin]-2'-yl)-1,2,3,4-tetrahydronaphthalen-1-yl)amino)-2-methoxy-5-(trifluoromethyl)pyridin-3-yl)methyl)-2,6-diazaspiro[3.4]octan-7-one;
2-((6-(((S)-5-(3'-chloro-6-methoxy-5-(((((R)-5-oxopyrrolidin-2-yl)methyl)amino)methyl)-[2,4'-bipyridin]-2'-yl)-1,2,3,4-tetrahydronaphthalen-1-yl)amino)-2-methoxy-5-(trifluoromethyl)pyridin-3-yl)methyl)-2,6-diazaspiro[3.4]octan-7-one;
2-((6-(((R)-5-(3'-chloro-6-methoxy-5-(((((S)-5-oxopyrrolidin-2-yl)methyl)amino)methyl)-[2,4'-bipyridin]-2'-yl)-1,2,3,4-tetrahydronaphthalen-1-yl)amino)-2-methoxy-5-(trifluoromethyl)pyridin-3-yl)methyl)-2,6-diazaspiro[3.4]octan-7-one;
2-((6-(((R)-5-(3'-chloro-6-methoxy-5-(((((R)-5-oxopyrrolidin-2-yl)methyl)amino)methyl)-[2,4'-bipyridin]-2'-yl)-1,2,3,4-tetrahydronaphthalen-1-yl)amino)-2-methoxy-5-(trifluoromethyl)pyridin-3-yl)methyl)-2,6-diazaspiro[3.4]octan-7-one;
(S)-N-(5-(3'-chloro-6-methoxy-5-((methylamino)methyl)-[2,4'-bipyridin]-2'-yl)-1,2,3,4-tetrahydronaphthalen-1-yl)-6-methoxy-5-((methylamino)methyl)-3-(trifluoromethyl)pyridin-2-amine;
(R)-N-(5-(3'-chloro-6-methoxy-5-((methylamino)methyl)-[2,4'-bipyridin]-2'-yl)-1,2,3,4-tetrahydronaphthalen-1-yl)-6-methoxy-5-((methylamino)methyl)-3-(trifluoromethyl)pyridin-2-amine;
1-(((6-(((S)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)cyclopropane-1-carboxylic acid;
1-(((6-(((S)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)cyclopropane-1-carboxylic acid;
1-(((6-(((R)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)cyclopropane-1-carboxylic acid;
1-(((6-(((R)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)cyclopropane-1-carboxylic acid;
(S)-5-(((4-(3-chloro-4-((S)-5-((5-((((S)-2-hydroxypropyl)amino)methyl)-6-methoxy-3-(trifluoromethyl)pyridin-2-yl)amino)-5,6,7,8-tetrahydronaphthalen-1-yl)pyridin-2-yl)-2-methoxybenzyl)amino)methyl)pyrrolidin-2-one;
(S)-5-(((4-(3-chloro-4-((S)-5-((5-((((R)-2-hydroxypropyl)amino)methyl)-6-methoxy-3-(trifluoromethyl)pyridin-2-yl)amino)-5,6,7,8-tetrahydronaphthalen-1-yl)pyridin-2-yl)-2-methoxybenzyl)amino)methyl)pyrrolidin-2-one;
(S)-5-(((4-(3-chloro-4-((R)-5-((5-((((S)-2-hydroxypropyl)amino)methyl)-6-methoxy-3-(trifluoromethyl)pyridin-2-yl)amino)-5,6,7,8-tetrahydronaphthalen-1-yl)pyridin-2-yl)-2-methoxybenzyl)amino)methyl)pyrrolidin-2-one;
(S)-5-(((4-(3-chloro-4-((R)-5-((5-((((R)-2-hydroxypropyl)amino)methyl)-6-methoxy-3-(trifluoromethyl)pyridin-2-yl)amino)-5,6,7,8-tetrahydronaphthalen-1-yl)pyridin-2-yl)-2-methoxybenzyl)amino)methyl)pyrrolidin-2-one;
(R)-5-(((4-(3-chloro-4-((R)-5-((5-((((R)-2-hydroxypropyl)amino)methyl)-6-methoxy-3-(trifluoromethyl)pyridin-2-yl)amino)-5,6,7,8-tetrahydronaphthalen-1-yl)pyridin-2-yl)-2-methoxybenzyl)amino)methyl)pyrrolidin-2-one;
(R)-5-(((4-(3-chloro-4-((R)-5-((5-((((S)-2-hydroxypropyl)amino)methyl)-6-methoxy-3-(trifluoromethyl)pyridin-2-yl)amino)-5,6,7,8-tetrahydronaphthalen-1-yl)pyridin-2-yl)-2-methoxybenzyl)amino)methyl)pyrrolidin-2-one;
(R)-5-(((4-(3-chloro-4-((S)-5-((5-((((R)-2-hydroxypropyl)amino)methyl)-6-methoxy-3-(trifluoromethyl)pyridin-2-yl)amino)-5,6,7,8-tetrahydronaphthalen-1-yl)pyridin-2-yl)-2-methoxybenzyl)amino)methyl)pyrrolidin-2-one;
(R)-5-(((4-(3-chloro-4-((S)-5-((5-((((S)-2-hydroxypropyl)amino)methyl)-6-methoxy-3-(trifluoromethyl)pyridin-2-yl)amino)-5,6,7,8-tetrahydronaphthalen-1-yl)pyridin-2-yl)-2-methoxybenzyl)amino)methyl)pyrrolidin-2-one;
(S)-5-((((6-(((S)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)methyl)pyrrolidin-2-one;
(S)-5-((((6-(((S)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)methyl)pyrrolidin-2-one;
(S)-5-((((6-(((R)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)methyl)pyrrolidin-2-one;
(S)-5-((((6-(((R)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)methyl)pyrrolidin-2-one;
(R)-5-((((6-(((R)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)methyl)pyrrolidin-2-one;
(R)-5-((((6-(((R)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)methyl)pyrrolidin-2-one;
(R)-5-((((6-(((S)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)methyl)pyrrolidin-2-one;
(R)-5-((((6-(((S)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)methyl)pyrrolidin-2-one;
2-((6-(((S)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)-2,6-diazaspiro[3.4]octan-7-one;
2-((6-(((S)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)-2,6-diazaspiro[3.4]octan-7-one;
2-((6-(((R)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)-2,6-diazaspiro[3.4]octan-7-one;
2-((6-(((R)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)-2,6-diazaspiro[3.4]octan-7-one;
(S)-N-(5-(3-chloro-2-(3-methoxy-4-((methylamino)methyl)phenyl)pyridin-4-yl)-1,2,3,4-tetrahydronaphthalen-1-yl)-6-methoxy-5-((methylamino)methyl)-3-(trifluoromethyl)pyridin-2-amine;
(R)-N-(5-(3-chloro-2-(3-methoxy-4-((methylamino)methyl)phenyl)pyridin-4-yl)-1,2,3,4-tetrahydronaphthalen-1-yl)-6-methoxy-5-((methylamino)methyl)-3-(trifluoromethyl)pyridin-2-amine;
1-(((6-(((S)-5-(3'-chloro-6-methoxy-5-(((((S)-5-oxopyrrolidin-2-yl)methyl)amino)methyl)-[2,4'-bipyridin]-2'-yl)-1,2,3,4-tetrahydronaphthalen-1-yl)oxy)-2-methoxy-5-(trifluoromethyl)pyridin-3-yl)methyl)amino)cyclopropane-1-carboxylic acid;
1-(((6-(((S)-5-(3'-chloro-6-methoxy-5-(((((R)-5-oxopyrrolidin-2-yl)methyl)amino)methyl)-[2,4'-bipyridin]-2'-yl)-1,2,3,4-tetrahydronaphthalen-1-yl)oxy)-2-methoxy-5-(trifluoromethyl)pyridin-3-yl)methyl)amino)cyclopropane-1-carboxylic acid;
1-(((6-(((R)-5-(3'-chloro-6-methoxy-5-(((((S)-5-oxopyrrolidin-2-yl)methyl)amino)methyl)-[2,4'-bipyridin]-2'-yl)-1,2,3,4-tetrahydronaphthalen-1-yl)oxy)-2-methoxy-5-(trifluoromethyl)pyridin-3-yl)methyl)amino)cyclopropane-1-carboxylic acid;
1-(((6-(((R)-5-(3'-chloro-6-methoxy-5-(((((R)-5-oxopyrrolidin-2-yl)methyl)amino)methyl)-[2,4'-bipyridin]-2'-yl)-1,2,3,4-tetrahydronaphthalen-1-yl)oxy)-2-methoxy-5-(trifluoromethyl)pyridin-3-yl)methyl)amino)cyclopropane-1-carboxylic acid;
(S)-5-((((3'-chloro-2'-((S)-5-((5-((((S)-2-hydroxypropyl)amino)methyl)-6-methoxy-3-(trifluoromethyl)pyridin-2-yl)oxy)-5,6,7,8-tetrahydronaphthalen-1-yl)-6-methoxy-[2,4'-bipyridin]-5-yl)methyl)amino)methyl)pyrrolidin-2-one;
(S)-5-((((3'-chloro-2'-((S)-5-((5-((((R)-2-hydroxypropyl)amino)methyl)-6-methoxy-3-(trifluoromethyl)pyridin-2-yl)oxy)-5,6,7,8-tetrahydronaphthalen-1-yl)-6-methoxy-[2,4'-bipyridin]-5-yl)methyl)amino)methyl)pyrrolidin-2-one;
(S)-5-((((3'-chloro-2'-((R)-5-((5-((((S)-2-hydroxypropyl)amino)methyl)-6-methoxy-3-(trifluoromethyl)pyridin-2-yl)oxy)-5,6,7,8-tetrahydronaphthalen-1-yl)-6-methoxy-[2,4'-bipyridin]-5-yl)methyl)amino)methyl)pyrrolidin-2-one; (S)-5-((((3'-chloro-2'-((R)-5-((5-((((R)-2-hydroxypropyl)amino)methyl)-6-methoxy-3-(trifluoromethyl)pyridin-2-yl)oxy)-5,6,7,8-tetrahydronaphthalen-1-yl)-6-methoxy-[2,4'-bipyridin]-5-yl)methyl)amino)methyl)pyrrolidin-2-one;
(R)-5-((((3'-chloro-2'-((R)-5-((5-((((R)-2-hydroxypropyl)amino)methyl)-6-methoxy-3-(trifluoromethyl)pyridin-2-yl)oxy)-5,6,7,8-tetrahydronaphthalen-1-yl)-6-methoxy-[2,4'-bipyridin]-5-yl)methyl)amino)methyl)pyrrolidin-2-one;
(R)-5-((((3'-chloro-2'-((R)-5-((5-((((S)-2-hydroxypropyl)amino)methyl)-6-methoxy-3-(trifluoromethyl)pyridin-2-yl)oxy)-5,6,7,8-tetrahydronaphthalen-1-yl)-6-methoxy-[2,4'-bipyridin]-5-yl)methyl)amino)methyl)pyrrolidin-2-one;
(R)-5-((((3'-chloro-2'-((S)-5-((5-((((R)-2-hydroxypropyl)amino)methyl)-6-methoxy-3-(trifluoromethyl)pyridin-2-yl)oxy)-5,6,7,8-tetrahydronaphthalen-1-yl)-6-methoxy-[2,4'-bipyridin]-5-yl)methyl)amino)methyl)pyrrolidin-2-one;
(R)-5-((((3'-chloro-2'-((S)-5-((5-((((S)-2-hydroxypropyl)amino)methyl)-6-methoxy-3-(trifluoromethyl)pyridin-2-yl)oxy)-5,6,7,8-tetrahydronaphthalen-1-yl)-6-methoxy-[2,4'-bipyridin]-5-yl)methyl)amino)methyl)pyrrolidin-2-one;
(S)-5-((((3'-chloro-6-methoxy-2'-((S)-5-((6-methoxy-5-(((((S)-5-oxopyrrolidin-2-yl)methyl)amino)methyl)-3-(trifluoromethyl)pyridin-2-yl)oxy)-5,6,7,8-tetrahydronaphthalen-1-yl)-[2,4'-bipyridin]-5-yl)methyl)amino)methyl)pyrrolidin-2-one;
(S)-5-((((3'-chloro-6-methoxy-2'-((S)-5-((6-methoxy-5-(((((R)-5-oxopyrrolidin-2-yl)methyl)amino)methyl)-3-(trifluoromethyl)pyridin-2-yl)oxy)-5,6,7,8-tetrahydronaphthalen-1-yl)-[2,4'-bipyridin]-5-yl)methyl)amino)methyl)pyrrolidin-2-one;
(S)-5-((((3'-chloro-6-methoxy-2'-((R)-5-((6-methoxy-5-(((((S)-5-oxopyrrolidin-2-yl)methyl)amino)methyl)-3-(trifluoromethyl)pyridin-2-yl)oxy)-5,6,7,8-tetrahydronaphthalen-1-yl)-[2,4'-bipyridin]-5-yl)methyl)amino)methyl)pyrrolidin-2-one;
(S)-5-((((3'-chloro-6-methoxy-2'-((R)-5-((6-methoxy-5-(((((R)-5-oxopyrrolidin-2-yl)methyl)amino)methyl)-3-(trifluoromethyl)pyridin-2-yl)oxy)-5,6,7,8-tetrahydronaphthalen-1-yl)-[2,4'-bipyridin]-5-yl)methyl)amino)methyl)pyrrolidin-2-one;
(R)-5-((((3'-chloro-6-methoxy-2'-((R)-5-((6-methoxy-5-(((((R)-5-oxopyrrolidin-2-yl)methyl)amino)methyl)-3-(trifluoromethyl)pyridin-2-yl)oxy)-5,6,7,8-tetrahydronaphthalen-1-yl)-[2,4'-bipyridin]-5-yl)methyl)amino)methyl)pyrrolidin-2-one;
(R)-5-((((3'-chloro-6-methoxy-2'-((R)-5-((6-methoxy-5-(((((S)-5-oxopyrrolidin-2-yl)methyl)amino)methyl)-3-(trifluoromethyl)pyridin-2-yl)oxy)-5,6,7,8-tetrahydronaphthalen-1-yl)-[2,4'-bipyridin]-5-yl)methyl)amino)methyl)pyrrolidin-2-one;
(R)-5-((((3'-chloro-6-methoxy-2'-((S)-5-((6-methoxy-5-(((((R)-5-oxopyrrolidin-2-yl)methyl)amino)methyl)-3-(trifluoromethyl)pyridin-2-yl)oxy)-5,6,7,8-tetrahydronaphthalen-1-yl)-[2,4'-bipyridin]-5-yl)methyl)amino)methyl)pyrrolidin-2-one;
(R)-5-((((3'-chloro-6-methoxy-2'-((S)-5-((6-methoxy-5-(((((S)-5-oxopyrrolidin-2-yl)methyl)amino)methyl)-3-(trifluoromethyl)pyridin-2-yl)oxy)-5,6,7,8-tetrahydronaphthalen-1-yl)-[2,4'-bipyridin]-5-yl)methyl)amino)methyl)pyrrolidin-2-one;
2-((6-(((S)-5-(3'-chloro-6-methoxy-5-(((((S)-5-oxopyrrolidin-2-yl)methyl)amino)methyl)-[2,4'-bipyridin]-2'-yl)-1,2,3,4-tetrahydronaphthalen-1-yl)oxy)-2-methoxy-5-(trifluoromethyl)pyridin-3-yl)methyl)-2,6-diazaspiro[3.4]octan-7-one;
2-((6-(((S)-5-(3'-chloro-6-methoxy-5-(((((R)-5-oxopyrrolidin-2-yl)methyl)amino)methyl)-[2,4'-bipyridin]-2'-yl)-1,2,3,4-tetrahydronaphthalen-1-yl)oxy)-2-methoxy-5-(trifluoromethyl)pyridin-3-yl)methyl)-2,6-diazaspiro[3.4]octan-7-one;
2-((6-(((S)-5-(3'-chloro-6-methoxy-5-(((((S)-5-oxopyrrolidin-2-yl)methyl)amino)methyl)-[2,4'-bipyridin]-2'-yl)-1,2,3,4-tetrahydronaphthalen-1-yl)oxy)-2-methoxy-5-(trifluoromethyl)pyridin-3-yl)methyl)-2,6-diazaspiro[3.4]octan-7-one;
2-((6-(((R)-5-(3'-chloro-6-methoxy-5-(((((R)-5-oxopyrrolidin-2-yl)methyl)amino)methyl)-[2,4'-bipyridin]-2'-yl)-1,2,3,4-tetrahydronaphthalen-1-yl)oxy)-2-methoxy-5-(trifluoromethyl)pyridin-3-yl)methyl)-2,6-diazaspiro[3.4]octan-7-one;
(S)-1-(3'-chloro-6-methoxy-2'-(5-((6-methoxy-5-((methylamino)methyl)-3-(trifluoromethyl)pyridin-2-yl)oxy)-5,6,7,8-tetrahydronaphthalen-1-yl)-[2,4'-bipyridin]-5-yl)-N-methylmethanamine;
(R)-1-(3'-chloro-6-methoxy-2'-(5-((6-methoxy-5-((methylamino)methyl)-3-(trifluoromethyl)pyridin-2-yl)oxy)-5,6,7,8-tetrahydronaphthalen-1-yl)-[2,4'-bipyridin]-5-yl)-N-methylmethanamine;
1-(((6-(((S)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)oxy)-2-methoxy-5-(trifluoromethyl)pyridin-3-yl)methyl)amino)cyclopropane-1-carboxylic acid;
1-(((6-(((S)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)oxy)-2-methoxy-5-(trifluoromethyl)pyridin-3-yl)methyl)amino)cyclopropane-1-carboxylic acid;
1-(((6-(((R)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)oxy)-2-methoxy-5-(trifluoromethyl)pyridin-3-yl)methyl)amino)cyclopropane-1-carboxylic acid;
1-(((6-(((R)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)oxy)-2-methoxy-5-(trifluoromethyl)pyridin-3-yl)methyl)amino)cyclopropane-1-carboxylic acid;
(S)-5-(((4-(3-chloro-4-((S)-5-((5-((((S)-2-hydroxypropyl)amino)methyl)-6-methoxy-3-(trifluoromethyl)pyridin-2-yl)oxy)-5,6,7,8-tetrahydronaphthalen-1-yl)pyridin-2-yl)-2-methoxybenzyl)amino)methyl)pyrrolidin-2-one;
(S)-5-(((4-(3-chloro-4-((S)-5-((5-((((R)-2-hydroxypropyl)amino)methyl)-6-methoxy-3-(trifluoromethyl)pyridin-2-yl)oxy)-5,6,7,8-tetrahydronaphthalen-1-yl)pyridin-2-yl)-2-methoxybenzyl)amino)methyl)pyrrolidin-2-one;
(S)-5-(((4-(3-chloro-4-((R)-5-((5-((((S)-2-hydroxypropyl)amino)methyl)-6-methoxy-3-(trifluoromethyl)pyridin-2-yl)oxy)-5,6,7,8-tetrahydronaphthalen-1-yl)pyridin-2-yl)-2-methoxybenzyl)amino)methyl)pyrrolidin-2-one;
(S)-5-(((4-(3-chloro-4-((R)-5-((5-((((R)-2-hydroxypropyl)amino)methyl)-6-methoxy-3-(trifluoromethyl)pyridin-2-yl)oxy)-5,6,7,8-tetrahydronaphthalen-1-yl)pyridin-2-yl)-2-methoxybenzyl)amino)methyl)pyrrolidin-2-one;
(R)-5-(((4-(3-chloro-4-((R)-5-((5-((((R)-2-hydroxypropyl)amino)methyl)-6-methoxy-3-(trifluoromethyl)pyridin-2-yl)oxy)-5,6,7,8-tetrahydronaphthalen-1-yl)pyridin-2-yl)-2-methoxybenzyl)amino)methyl)pyrrolidin-2-one;
(R)-5-(((4-(3-chloro-4-((R)-5-((5-((((S)-2-hydroxypropyl)amino)methyl)-6-methoxy-3-(trifluoromethyl)pyridin-2-yl)oxy)-5,6,7,8-tetrahydronaphthalen-1-yl)pyridin-2-yl)-2-methoxybenzyl)amino)methyl)pyrrolidin-2-one;
(R)-5-(((4-(3-chloro-4-((S)-5-((5-((((R)-2-hydroxypropyl)amino)methyl)-6-methoxy-3-(trifluoromethyl)pyridin-2-yl)oxy)-5,6,7,8-tetrahydronaphthalen-1-yl)pyridin-2-yl)-2-methoxybenzyl)amino)methyl)pyrrolidin-2-one;
(R)-5-(((4-(3-chloro-4-((S)-5-((5-((((S)-2-hydroxypropyl)amino)methyl)-6-methoxy-3-(trifluoromethyl)pyridin-2-yl)oxy)-5,6,7,8-tetrahydronaphthalen-1-yl)pyridin-2-yl)-2-methoxybenzyl)amino)methyl)pyrrolidin-2-one;
(S)-5-((((6-(((S)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)oxy)-2-methoxy-5-(trifluoromethyl)pyridin-3-yl)methyl)amino)methyl)pyrrolidin-2-one;
(S)-5-((((6-(((S)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)oxy)-2-methoxy-5-(trifluoromethyl)pyridin-3-yl)methyl)amino)methyl)pyrrolidin-2-one;
(S)-5-((((6-(((R)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)oxy)-2-methoxy-5-(trifluoromethyl)pyridin-3-yl)methyl)amino)methyl)pyrrolidin-2-one;
(S)-5-((((6-(((R)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)oxy)-2-methoxy-5-(trifluoromethyl)pyridin-3-yl)methyl)amino)methyl)pyrrolidin-2-one;
(R)-5-((((6-(((R)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)oxy)-2-methoxy-5-(trifluoromethyl)pyridin-3-yl)methyl)amino)methyl)pyrrolidin-2-one;
(R)-5-((((6-(((R)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)oxy)-2-methoxy-5-(trifluoromethyl)pyridin-3-yl)methyl)amino)methyl)pyrrolidin-2-one;
(R)-5-((((6-(((S)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)oxy)-2-methoxy-5-(trifluoromethyl)pyridin-3-yl)methyl)amino)methyl)pyrrolidin-2-one;
(R)-5-((((6-(((S)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)oxy)-2-methoxy-5-(trifluoromethyl)pyridin-3-yl)methyl)amino)methyl)pyrrolidin-2-one;
2-((6-(((S)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)oxy)-2-methoxy-5-(trifluoromethyl)pyridin-3-yl)methyl)-2,6-diazaspiro[3.4]octan-7-one;
2-((6-(((S)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)oxy)-2-methoxy-5-(trifluoromethyl)pyridin-3-yl)methyl)-2,6-diazaspiro[3.4]octan-7-one;
2-((6-(((R)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)oxy)-2-methoxy-5-(trifluoromethyl)pyridin-3-yl)methyl)-2,6-diazaspiro[3.4]octan-7-one;
2-((6-(((R)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)oxy)-2-methoxy-5-(trifluoromethyl)pyridin-3-yl)methyl)-2,6-diazaspiro[3.4]octan-7-one;
(S)-1-(6-((5-(3-chloro-2-(3-methoxy-4-((methylamino)methyl)phenyl)pyridin-4-yl)-1,2,3,4-tetrahydronaphthalen-1-yl)oxy)-2-methoxy-5-(trifluoromethyl)pyridin-3-yl)-N-methylmethanamine;
(R)-1-(6-((5-(3-chloro-2-(3-methoxy-4-((methylamino)methyl)phenyl)pyridin-4-yl)-1,2,3,4-tetrahydronaphthalen-1-yl)oxy)-2-methoxy-5-(trifluoromethyl)pyridin-3-yl)-N-methylmethanamine;
(S)-5-(((4-(3-chloro-4-(2-chloro-3-(8-chloro-6-((((S)-2-hydroxypropyl)amino)methyl)-5-methoxyquinolin-2-yl)phenyl)pyridin-2-yl)-2-methoxybenzyl)amino)methyl)pyrrolidin-2-one;
(S)-5-(((4-(3-chloro-4-(2-chloro-3-(8-chloro-6-((((R)-2-hydroxypropyl)amino)methyl)-5-methoxyquinolin-2-yl)phenyl)pyridin-2-yl)-2-methoxybenzyl)amino)methyl)pyrrolidin-2-one;
(R)-5-(((4-(3-chloro-4-(2-chloro-3-(8-chloro-6-((((S)-2-hydroxypropyl)amino)methyl)-5-methoxyquinolin-2-yl)phenyl)pyridin-2-yl)-2-methoxybenzyl)amino)methyl)pyrrolidin-2-one;
(R)-5-(((4-(3-chloro-4-(2-chloro-3-(8-chloro-6-((((R)-2-hydroxypropyl)amino)methyl)-5-methoxyquinolin-2-yl)phenyl)pyridin-2-yl)-2-methoxybenzyl)amino)methyl)pyrrolidin-2-one;
(S)-5-((((8-chloro-2-(2-chloro-3-(3-chloro-2-(3-methoxy-4-(((((S)-5-oxopyrrolidin-2-yl)methyl)amino)methyl)phenyl)pyridin-4-yl)phenyl)-5-methoxyquinolin-6-yl)methyl)amino)methyl)pyrrolidin-2-one;
(S)-5-((((8-chloro-2-(2-chloro-3-(3-chloro-2-(3-methoxy-4-(((((R)-5-oxopyrrolidin-2-yl)methyl)amino)methyl)phenyl)pyridin-4-yl)phenyl)-5-methoxyquinolin-6-yl)methyl)amino)methyl)pyrrolidin-2-one;
(R)-5-((((8-chloro-2-(2-chloro-3-(3-chloro-2-(3-methoxy-4-(((((S)-5-oxopyrrolidin-2-yl)methyl)amino)methyl)phenyl)pyridin-4-yl)phenyl)-5-methoxyquinolin-6-yl)methyl)amino)methyl)pyrrolidin-2-one;
(R)-5-((((8-chloro-2-(2-chloro-3-(3-chloro-2-(3-methoxy-4-(((((R)-5-oxopyrrolidin-2-yl)methyl)amino)methyl)phenyl)pyridin-4-yl)phenyl)-5-methoxyquinolin-6-yl)methyl)amino)methyl)pyrrolidin-2-one;
(S)-5-((((6-(2-chloro-3-(3-chloro-2-(8-chloro-2-(3-hydroxy-3-methylbutyl)-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(8-chloro-2-(3-hydroxy-3-methylbutyl)-1,2,3,4-tetrahydroisoquinolin-6-yl)pyridin-4-yl)phenyl)-2-methoxypyridin-3-yl)methyl)amino)methyl)pyrrolidin-2-one;
isopropyl (S)-4-((4-(3-chloro-4-(2-chloro-3-(5-(4,5-dihydro-1H-imidazol-2-yl)-6-methoxypyridin-2-yl)phenyl)pyridin-2-yl)-2-methoxybenzyl)amino)-3-hydroxybutanoate;
isopropyl (R)-4-((4-(3-chloro-4-(2-chloro-3-(5-(4,5-dihydro-1H-imidazol-2-yl)-6-methoxypyridin-2-yl)phenyl)pyridin-2-yl)-2-methoxybenzyl)amino)-3-hydroxybutanoate;
(S)-5-((((6-(3-(2-(4-(((2-acetyl-2-azaspiro[3.3]heptan-6-yl)amino)methyl)-3-fluoro-5-methoxyphenyl)-3-chloropyridin-4-yl)-2-chlorophenyl)-2-methoxypyridin-3-yl)methyl)amino)methyl)pyrrolidin-2-one;
(R)-5-((((6-(3-(2-(4-(((2-acetyl-2-azaspiro[3.3]heptan-6-yl)amino)methyl)-3-fluoro-5-methoxyphenyl)-3-chloropyridin-4-yl)-2-chlorophenyl)-2-methoxypyridin-3-yl)methyl)amino)methyl)pyrrolidin-2-one;
N-(4-(3-chloro-4-(2-chloro-3-(6-methoxy-5-((methylamino)methyl)pyridin-2-yl)phenyl)pyridin-2-yl)-2-methoxybenzyl)propan-2-amine;
1-(4-((4-(3-chloro-4-(2-chloro-3-(6-methoxy-5-((methylamino)methyl)pyridin-2-yl)phenyl)pyridin-2-yl)-2-methoxybenzyl)amino)piperidin-1-yl)ethan-1-one;
2-(4-(3-chloro-4-(2-chloro-3-(6-methoxy-5-((methylamino)methyl)pyridin-2-yl)phenyl)pyridin-2-yl)-2-methoxybenzyl)-2,6-diazaspiro[3.4]octan-7-one;
(S)-1-(((6-(2-chloro-3-(3-chloro-2-(2-((S)-2-hydroxypropyl)-8-methoxy-1,2,3,4-tetrahydroisoquinolin-6-yl)pyridin-4-yl)phenyl)-2-methoxypyridin-3-yl)methyl)amino)propan-2-ol;
(S)-1-(((6-(2-chloro-3-(3-chloro-2-(2-((R)-2-hydroxypropyl)-8-methoxy-1,2,3,4-tetrahydroisoquinolin-6-yl)pyridin-4-yl)phenyl)-2-methoxypyridin-3-yl)methyl)amino)propan-2-ol;
(R)-1-(((6-(2-chloro-3-(3-chloro-2-(2-((S)-2-hydroxypropyl)-8-methoxy-1,2,3,4-tetrahydroisoquinolin-6-yl)pyridin-4-yl)phenyl)-2-methoxypyridin-3-yl)methyl)amino)propan-2-ol;
(R)-1-(((6-(2-chloro-3-(3-chloro-2-(2-((R)-2-hydroxypropyl)-8-methoxy-1,2,3,4-tetrahydroisoquinolin-6-yl)pyridin-4-yl)phenyl)-2-methoxypyridin-3-yl)methyl)amino)propan-2-ol;
(1r,3r)-3-(((6-(2-chloro-3-(3-chloro-2-(2-((S)-2-hydroxypropyl)-8-methoxy-1,2,3,4-tetrahydroisoquinolin-6-yl)pyridin-4-yl)phenyl)-2-methoxypyridin-3-yl)methyl)amino)-1-methylcyclobutan-1-ol;
(1r,3r)-3-(((6-(2-chloro-3-(3-chloro-2-(2-((R)-2-hydroxypropyl)-8-methoxy-1,2,3,4-tetrahydroisoquinolin-6-yl)pyridin-4-yl)phenyl)-2-methoxypyridin-3-yl)methyl)amino)-1-methylcyclobutan-1-ol;
(1r,3s)-3-(((6-(2-chloro-3-(3-chloro-2-(2-((S)-2-hydroxypropyl)-8-methoxy-1,2,3,4-tetrahydroisoquinolin-6-yl)pyridin-4-yl)phenyl)-2-methoxypyridin-3-yl)methyl)amino)-1-methylcyclobutan-1-ol;
(1s,3s)-3-(((6-(2-chloro-3-(3-chloro-2-(2-((S)-2-hydroxypropyl)-8-methoxy-1,2,3,4-tetrahydroisoquinolin-6-yl)pyridin-4-yl)phenyl)-2-methoxypyridin-3-yl)methyl)amino)-1-methylcyclobutan-1-ol;
(1s,3s)-3-(((6-(2-chloro-3-(3-chloro-2-(2-((R)-2-hydroxypropyl)-8-methoxy-1,2,3,4-tetrahydroisoquinolin-6-yl)pyridin-4-yl)phenyl)-2-methoxypyridin-3-yl)methyl)amino)-1-methylcyclobutan-1-ol;
(1s,3r)-3-(((6-(2-chloro-3-(3-chloro-2-(2-((R)-2-hydroxypropyl)-8-methoxy-1,2,3,4-tetrahydroisoquinolin-6-yl)pyridin-4-yl)phenyl)-2-methoxypyridin-3-yl)methyl)amino)-1-methylcyclobutan-1-ol;
(1s,3r)-3-(((6-(2-chloro-3-(3-chloro-2-(2-((S)-2-hydroxypropyl)-8-methoxy-1,2,3,4-tetrahydroisoquinolin-6-yl)pyridin-4-yl)phenyl)-2-methoxypyridin-3-yl)methyl)amino)-1-methylcyclobutan-1-ol;
(1r,3s)-3-(((6-(2-chloro-3-(3-chloro-2-(2-((R)-2-hydroxypropyl)-8-methoxy-1,2,3,4-tetrahydroisoquinolin-6-yl)pyridin-4-yl)phenyl)-2-methoxypyridin-3-yl)methyl)amino)-1-methylcyclobutan-1-ol;
(S)-1-(6-(3-chloro-4-(2-chloro-3-(6-methoxy-5-(((((S)-tetrahydrofuran-2-yl)methyl)amino)methyl)pyridin-2-yl)phenyl)pyridin-2-yl)-8-methoxy-3,4-dihydroisoquinolin-2(1H)-yl)propan-2-ol;
(S)-1-(6-(3-chloro-4-(2-chloro-3-(6-methoxy-5-(((((R)-tetrahydrofuran-2-yl)methyl)amino)methyl)pyridin-2-yl)phenyl)pyridin-2-yl)-8-methoxy-3,4-dihydroisoquinolin-2(1H)-yl)propan-2-ol;
(R)-1-(6-(3-chloro-4-(2-chloro-3-(6-methoxy-5-(((((S)-tetrahydrofuran-2-yl)methyl)amino)methyl)pyridin-2-yl)phenyl)pyridin-2-yl)-8-methoxy-3,4-dihydroisoquinolin-2(1H)-yl)propan-2-ol;
(R)-1-(6-(3-chloro-4-(2-chloro-3-(6-methoxy-5-(((((R)-tetrahydrofuran-2-yl)methyl)amino)methyl)pyridin-2-yl)phenyl)pyridin-2-yl)-8-methoxy-3,4-dihydroisoquinolin-2(1H)-yl)propan-2-ol;
(S)-1-((6-(2-chloro-3-(3-chloro-2-(2-(2-hydroxypropyl)-8-methoxy-1,2,3,4-tetrahydroisoquinolin-6-yl)pyridin-4-yl)phenyl)-2-methoxypyridin-3-yl)methyl)-3-methylazetidin-3-ol;
(R)-1-((6-(2-chloro-3-(3-chloro-2-(2-(2-hydroxypropyl)-8-methoxy-1,2,3,4-tetrahydroisoquinolin-6-yl)pyridin-4-yl)phenyl)-2-methoxypyridin-3-yl)methyl)-3-methylazetidin-3-ol;
methyl (S)-((6-(2-chloro-3-(3-chloro-2-(2-(2-hydroxypropyl)-8-methoxy-1,2,3,4-tetrahydroisoquinolin-6-yl)pyridin-4-yl)phenyl)-2-methoxypyridin-3-yl)methyl)glycinate;
methyl (R)-((6-(2-chloro-3-(3-chloro-2-(2-(2-hydroxypropyl)-8-methoxy-1,2,3,4-tetrahydroisoquinolin-6-yl)pyridin-4-yl)phenyl)-2-methoxypyridin-3-yl)methyl)glycinate;
(S)-2-((6-(2-chloro-3-(3-chloro-2-(3-methoxy-4-((((5-oxopyrrolidin-2-yl)methyl)amino)methyl)phenyl)pyridin-4-yl)phenyl)-1-methyl-1H-pyrrolo[2,3-b]pyridin-3-yl)methyl)-2,6-diazaspiro[3.4]octan-7-one;
(R)-2-((6-(2-chloro-3-(3-chloro-2-(3-methoxy-4-((((5-oxopyrrolidin-2-yl)methyl)amino)methyl)phenyl)pyridin-4-yl)phenyl)-1-methyl-1H-pyrrolo[2,3-b]pyridin-3-yl)methyl)-2,6-diazaspiro[3.4]octan-7-one;
(S)-4-((4-(3-chloro-4-(2-chloro-3-(6-methoxy-5-((methylamino)methyl)pyridin-2-yl)phenyl)pyridin-2-yl)-2-methoxybenzyl)amino)-3-hydroxybutanoic acid;
(R)-4-((4-(3-chloro-4-(2-chloro-3-(6-methoxy-5-((methylamino)methyl)pyridin-2-yl)phenyl)pyridin-2-yl)-2-methoxybenzyl)amino)-3-hydroxybutanoic acid;
3-((4-(3-chloro-4-(2-chloro-3-(6-methoxy-5-((methylamino)methyl)pyridin-2-yl)phenyl)pyridin-2-yl)-2-methoxybenzyl)amino)propanoic acid;
6-(3-chloro-4-(2-chloro-3-(5-((6-hydroxy-2-azaspiro[3.3]heptan-2-yl)methyl)-6-methoxypyridin-2-yl)phenyl)pyridin-2-yl)-2-((3-hydroxy-3-methylazetidin-1-yl)methyl)-3-methylpyrrolo[2,1-f][1,2,4]triazin-4(3H)-one;
(S)-5-((((3'-chloro-6-methoxy-2'-((S)-1-((6-methoxy-5-(((((S)-5-oxopyrrolidin-2-yl)methyl)amino)methyl)-3-(trifluoromethyl)pyridin-2-yl)amino)-2,3-dihydro-1H-inden-4-yl)-[2,4'-bipyridin]-5-yl)methyl)amino)methyl)pyrrolidin-2-one;
(R)-5-((((3'-chloro-6-methoxy-2'-((R)-1-((6-methoxy-5-(((((R)-5-oxopyrrolidin-2-yl)methyl)amino)methyl)-3-(trifluoromethyl)pyridin-2-yl)amino)-2,3-dihydro-1H-inden-4-yl)-[2,4'-bipyridin]-5-yl)methyl)amino)methyl)pyrrolidin-2-one;
(R)-5-((((3'-chloro-6-methoxy-2'-((R)-1-((6-methoxy-5-(((((S)-5-oxopyrrolidin-2-yl)methyl)amino)methyl)-3-(trifluoromethyl)pyridin-2-yl)amino)-2,3-dihydro-1H-inden-4-yl)-[2,4'-bipyridin]-5-yl)methyl)amino)methyl)pyrrolidin-2-one;
(R)-5-((((3'-chloro-6-methoxy-2'-((S)-1-((6-methoxy-5-(((((S)-5-oxopyrrolidin-2-yl)methyl)amino)methyl)-3-(trifluoromethyl)pyridin-2-yl)amino)-2,3-dihydro-1H-inden-4-yl)-[2,4'-bipyridin]-5-yl)methyl)amino)methyl)pyrrolidin-2-one;
(R)-5-((((3'-chloro-6-methoxy-2'-((S)-1-((6-methoxy-5-(((((R)-5-oxopyrrolidin-2-yl)methyl)amino)methyl)-3-(trifluoromethyl)pyridin-2-yl)amino)-2,3-dihydro-1H-inden-4-yl)-[2,4'-bipyridin]-5-yl)methyl)amino)methyl)pyrrolidin-2-one;
(S)-5-((((3'-chloro-6-methoxy-2'-((S)-1-((6-methoxy-5-(((((R)-5-oxopyrrolidin-2-yl)methyl)amino)methyl)-3-(trifluoromethyl)pyridin-2-yl)amino)-2,3-dihydro-1H-inden-4-yl)-[2,4'-bipyridin]-5-yl)methyl)amino)methyl)pyrrolidin-2-one;
(S)-5-((((3'-chloro-6-methoxy-2'-((R)-1-((6-methoxy-5-(((((R)-5-oxopyrrolidin-2-yl)methyl)amino)methyl)-3-(trifluoromethyl)pyridin-2-yl)amino)-2,3-dihydro-1H-inden-4-yl)-[2,4'-bipyridin]-5-yl)methyl)amino)methyl)pyrrolidin-2-one;
(S)-5-((((3'-chloro-6-methoxy-2'-((R)-1-((6-methoxy-5-(((((S)-5-oxopyrrolidin-2-yl)methyl)amino)methyl)-3-(trifluoromethyl)pyridin-2-yl)amino)-2,3-dihydro-1H-inden-4-yl)-[2,4'-bipyridin]-5-yl)methyl)amino)methyl)pyrrolidin-2-one;
(S)-N-(4-(3'-chloro-6-methoxy-5-((methylamino)methyl)-[2,4'-bipyridin]-2'-yl)-2,3-dihydro-1H-inden-1-yl)-6-methoxy-5-((methylamino)methyl)-3-(trifluoromethyl)pyridin-2-amine;
(R)-N-(4-(3'-chloro-6-methoxy-5-((methylamino)methyl)-[2,4'-bipyridin]-2'-yl)-2,3-dihydro-1H-inden-1-yl)-6-methoxy-5-((methylamino)methyl)-3-(trifluoromethyl)pyridin-2-amine;
(1S,3r)-3-(((3'-chloro-2'-((S)-1-((5-((((1r,3S)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1R,3r)-3-(((3'-chloro-2'-((R)-1-((5-((((1r,3R)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1R,3r)-3-(((3'-chloro-2'-((R)-1-((5-((((1r,3S)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1R,3r)-3-(((3'-chloro-2'-((R)-1-((5-((((1s,3R)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1R,3r)-3-(((3'-chloro-2'-((R)-1-((5-((((1s,3S)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1R,3r)-3-(((3'-chloro-2'-((S)-1-((5-((((1r,3R)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1R,3r)-3-(((3'-chloro-2'-((S)-1-((5-((((1r,3S)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1R,3r)-3-(((3'-chloro-2'-((S)-1-((5-((((1s,3R)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1R,3r)-3-(((3'-chloro-2'-((S)-1-((5-((((1s,3S)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1R,3s)-3-(((3'-chloro-2'-((R)-1-((5-((((1r,3R)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1R,3s)-3-(((3'-chloro-2'-((R)-1-((5-((((1r,3S)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1R,3s)-3-(((3'-chloro-2'-((R)-1-((5-((((1s,3R)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1R,3s)-3-(((3'-chloro-2'-((R)-1-((5-((((1s,3S)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1R,3r)-3-(((3'-chloro-2'-((R)-1-((5-((((1r,3R)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1R,3s)-3-(((3'-chloro-2'-((S)-1-((5-((((1r,3S)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1R,3s)-3-(((3'-chloro-2'-((S)-1-((5-((((1s,3R)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1R,3s)-3-(((3'-chloro-2'-((S)-1-((5-((((1s,3S)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1S,3r)-3-(((3'-chloro-2'-((R)-1-((5-((((1r,3R)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1S,3r)-3-(((3'-chloro-2'-((R)-1-((5-((((1r,3S)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1S,3r)-3-(((3'-chloro-2'-((R)-1-((5-((((1s,3R)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1S,3r)-3-(((3'-chloro-2'-((R)-1-((5-((((1s,3S)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1S,3r)-3-(((3'-chloro-2'-((S)-1-((5-((((1r,3R)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1S,3r)-3-(((3'-chloro-2'-((S)-1-((5-((((1s,3R)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1S,3r)-3-(((3'-chloro-2'-((S)-1-((5-((((1s,3S)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1S,3s)-3-(((3'-chloro-2'-((R)-1-((5-((((1r,3R)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1S,3s)-3-(((3'-chloro-2'-((R)-1-((5-((((1r,3S)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1S,3s)-3-(((3'-chloro-2'-((R)-1-((5-((((1s,3R)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1S,3s)-3-(((3'-chloro-2'-((R)-1-((5-((((1s,3S)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1S,3r)-3-(((3'-chloro-2'-((R)-1-((5-((((1r,3R)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1S,3s)-3-(((3'-chloro-2'-((S)-1-((5-((((1r,3S)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1S,3s)-3-(((3'-chloro-2'-((S)-1-((5-((((1s,3R)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1S,3s)-3-(((3'-chloro-2'-((S)-1-((5-((((1s,3S)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(S)-1-(4-(3-chloro-4-(2-chloro-3-(6-methoxy-5-((methylamino)methyl)pyridin-2-yl)phenyl)pyridin-2-yl)-2-methoxybenzyl)pyrrolidine-3-carboxylic acid;
(R)-1-(4-(3-chloro-4-(2-chloro-3-(6-methoxy-5-((methylamino)methyl)pyridin-2-yl)phenyl)pyridin-2-yl)-2-methoxybenzyl)pyrrolidine-3-carboxylic acid;
2-(4-(3-chloro-4-(2-chloro-3-(6-methoxy-5-((methylamino)methyl)pyridin-2-yl)phenyl)pyridin-2-yl)-2-methoxybenzyl)-2-azaspiro[3.3]heptane-6-carboxylic acid;
3-(((4-(3-chloro-4-(2-chloro-3-(6-methoxy-5-((methylamino)methyl)pyridin-2-yl)phenyl)pyridin-2-yl)-2-methoxybenzyl)amino)methyl)bicyclo[1.1.1]pentane-1-carboxylic acid;
(S)-((6-(2-chloro-3-(3-chloro-2-(2-(2-hydroxypropyl)-8-methoxy-1,2,3,4-tetrahydroisoquinolin-6-yl)pyridin-4-yl)phenyl)-2-methoxypyridin-3-yl)methyl)glycine;
(R)-((6-(2-chloro-3-(3-chloro-2-(2-(2-hydroxypropyl)-8-methoxy-1,2,3,4-tetrahydroisoquinolin-6-yl)pyridin-4-yl)phenyl)-2-methoxypyridin-3-yl)methyl)glycine;
2-((6-(2-chloro-3-(3-chloro-2-(isoindolin-5-yl)pyridin-4-yl)phenyl)-2-methoxypyridin-3-yl)methyl)-2,6-diazaspiro[3.4]octan-7-one;
6-(3-chloro-4-(2-chloro-3-(5-((3-hydroxy-3-methylazetidin-1-yl)methyl)-6-methoxypyridin-2-yl)phenyl)pyridin-2-yl)-2-((3-hydroxy-3-methylazetidin-1-yl)methyl)-3-methylpyrrolo[2,1-f][1,2,4]triazin-4(3H)-one;
6-(3-chloro-4-(2-chloro-3-(5-((((1r,3r)-3-hydroxy-3-methylcyclobutyl)amino)methyl)-6-methoxypyridin-2-yl)phenyl)pyridin-2-yl)-2-((3-hydroxy-3-methylazetidin-1-yl)methyl)-3-methylpyrrolo[2,1-f][1,2,4]triazin-4(3H)-one;
6-(3-chloro-4-(2-chloro-3-(5-((((1r,3s)-3-hydroxy-3-methylcyclobutyl)amino)methyl)-6-**methoxypyridin-2-yl)phenyl)pyridin-2-yl)-2-((3-hydroxy-3-methylazetidin-1-yl)methyl)-3-**methylpyrrolo[2,1-f][1,2,4]triazin-4(3H)-one;
6-(3-chloro-4-(2-chloro-3-(5-((((1s,3r)-3-hydroxy-3-methylcyclobutyl)amino)methyl)-6-methoxypyridin-2-yl)phenyl)pyridin-2-yl)-2-((3-hydroxy-3-methylazetidin-1-yl)methyl)-3-methylpyrrolo[2,1-f][1,2,4]triazin-4(3H)-one;
6-(3-chloro-4-(2-chloro-3-(5-((((1s,3s)-3-hydroxy-3-methylcyclobutyl)amino)methyl)-6-methoxypyridin-2-yl)phenyl)pyridin-2-yl)-2-((3-hydroxy-3-methylazetidin-1-yl)methyl)-3-methylpyrrolo[2,1-f][1,2,4]triazin-4(3H)-one;
6-(3-chloro-4-(2-chloro-3-(5-((isopropylamino)methyl)-6-methoxypyridin-2-yl)phenyl)pyridin-2-yl)-2-((3-hydroxy-3-methylazetidin-1-yl)methyl)-3-methylpyrrolo[2,1-f][1,2,4]triazin-4(3H)-one;
6-(3-chloro-4-(2-chloro-3-(5-((((1r,4r)-4-hydroxycyclohexyl)amino)methyl)-6-methoxypyridin-2-yl)phenyl)pyridin-2-yl)-2-((3-hydroxy-3-methylazetidin-1-yl)methyl)-3-methylpyrrolo[2,1-f][1,2,4]triazin-4(3H)-one;
6-(3-chloro-4-(2-chloro-3-(5-((((1r,4s)-4-hydroxycyclohexyl)amino)methyl)-6-methoxypyridin-2-yl)phenyl)pyridin-2-yl)-2-((3-hydroxy-3-methylazetidin-1-yl)methyl)-3-methylpyrrolo[2,1-f][1,2,4]triazin-4(3H)-one;
6-(3-chloro-4-(2-chloro-3-(5-((((1s,4r)-4-hydroxycyclohexyl)amino)methyl)-6-methoxypyridin-2-yl)phenyl)pyridin-2-yl)-2-((3-hydroxy-3-methylazetidin-1-yl)methyl)-3-methylpyrrolo[2,1-f][1,2,4]triazin-4(3H)-one;
6-(3-chloro-4-(2-chloro-3-(5-((((1s,4s)-4-hydroxycyclohexyl)amino)methyl)-6-methoxypyridin-2-yl)phenyl)pyridin-2-yl)-2-((3-hydroxy-3-methylazetidin-1-yl)methyl)-3-methylpyrrolo[2,1-f][1,2,4]triazin-4(3H)-one;
(S)-5-((((6-(2-chloro-3-(3-chloro-2-(1-methyl-3-(((((S)-oxetan-2-yl)methyl)amino)methyl)-1H-indazol-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(1-methyl-3-(((((R)-oxetan-2-yl)methyl)amino)methyl)-1H-indazol-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(1-methyl-3-(((((S)-oxetan-2-yl)methyl)amino)methyl)-1H-indazol-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(1-methyl-3-(((((R)-oxetan-2-yl)methyl)amino)methyl)-1H-indazol-6-yl)pyridin-4-yl)phenyl)-2-methoxypyridin-3-yl)methyl)amino)methyl)pyrrolidin-2-one;
(S)-5-((((8-chloro-2-(2-chloro-3-(3-chloro-2-(3-fluoro-5-methoxy-4-(((((S)-5-oxopyrrolidin-2-yl)methyl)amino)methyl)phenyl)pyridin-4-yl)phenyl)-5-methoxyquinolin-6-yl)methyl)amino)methyl)pyrrolidin-2-one;
(S)-5-((((8-chloro-2-(2-chloro-3-(3-chloro-2-(3-fluoro-5-methoxy-4-(((((R)-5-oxopyrrolidin-2-yl)methyl)amino)methyl)phenyl)pyridin-4-yl)phenyl)-5-methoxyquinolin-6-yl)methyl)amino)methyl)pyrrolidin-2-one;
(R)-5-((((8-chloro-2-(2-chloro-3-(3-chloro-2-(3-fluoro-5-methoxy-4-(((((S)-5-oxopyrrolidin-2-yl)methyl)amino)methyl)phenyl)pyridin-4-yl)phenyl)-5-methoxyquinolin-6-yl)methyl)amino)methyl)pyrrolidin-2-one;
(R)-5-((((8-chloro-2-(2-chloro-3-(3-chloro-2-(3-fluoro-5-methoxy-4-(((((R)-5-oxopyrrolidin-2-yl)methyl)amino)methyl)phenyl)pyridin-4-yl)phenyl)-5-methoxyquinolin-6-yl)methyl)amino)methyl)pyrrolidin-2-one;
(S)-5-((((6-(3-(2-(4-(((2-acetyl-2-azaspiro[3.3]heptan-6-yl)amino)methyl)-3-methoxyphenyl)-3-chloropyridin-4-yl)-2-chlorophenyl)-2-methoxypyridin-3-yl)methyl)amino)methyl)pyrrolidin-2-one;
(R)-5-((((6-(3-(2-(4-(((2-acetyl-2-azaspiro[3.3]heptan-6-yl)amino)methyl)-3-methoxyphenyl)-3-chloropyridin-4-yl)-2-chlorophenyl)-2-methoxypyridin-3-yl)methyl)amino)methyl)pyrrolidin-2-one;
(S)-5-(((4-(3-chloro-4-(2-chloro-3-(8-chloro-6-(((2-hydroxyethyl)amino)methyl)-5-methoxyquinolin-2-yl)phenyl)pyridin-2-yl)-2-methoxybenzyl)amino)methyl)pyrrolidin-2-one;
(R)-5-(((4-(3-chloro-4-(2-chloro-3-(8-chloro-6-(((2-hydroxyethyl)amino)methyl)-5-methoxyquinolin-2-yl)phenyl)pyridin-2-yl)-2-methoxybenzyl)amino)methyl)pyrrolidin-2-one;
(S)-1-(4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)pyrrolidine-3-carboxylic acid;
(R)-1-(4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)pyrrolidine-3-carboxylic acid;
1-(4-(((6-(2-chloro-3-(3-chloro-2-(3-methoxy-4-((methylamino)methyl)phenyl)pyridin-4-yl)phenyl)-2-methoxypyridin-3-yl)methyl)amino)piperidin-1-yl)ethan-1-one;
(S)-5-(((4-(4-(3-(6-(((1-acetylpiperidin-4-yl)amino)methyl)-8-chloro-5-methoxyquinolin-2-yl)-2-chlorophenyl)-3-chloropyridin-2-yl)-2-methoxybenzyl)amino)methyl)pyrrolidin-2-one;
(R)-5-(((4-(4-(3-(6-(((1-acetylpiperidin-4-yl)amino)methyl)-8-chloro-5-methoxyquinolin-2-yl)-2-chlorophenyl)-3-chloropyridin-2-yl)-2-methoxybenzyl)amino)methyl)pyrrolidin-2-one;
5-(3-chloro-4-(2-chloro-3'-fluoro-5'-methoxy-4'-(((((S)-5-oxopyrrolidin-2-yl)methyl)amino)methyl)-[1,1'-biphenyl]-3-yl)pyridin-2-yl)-2-(2-((((S)-5-oxopyrrolidin-2-yl)methyl)amino)ethyl)isoindolin-1-one;
5-(3-chloro-4-(2-chloro-3'-fluoro-5'-methoxy-4'-(((((S)-5-oxopyrrolidin-2-yl)methyl)amino)methyl)-[1,1'-biphenyl]-3-yl)pyridin-2-yl)-2-(2-((((R)-5-oxopyrrolidin-2-yl)methyl)amino)ethyl)isoindolin-1-one;
5-(3-chloro-4-(2-chloro-3'-fluoro-5'-methoxy-4'-(((((R)-5-oxopyrrolidin-2-yl)methyl)amino)methyl)-[1,1'-biphenyl]-3-yl)pyridin-2-yl)-2-(2-((((S)-5-oxopyrrolidin-2-yl)methyl)amino)ethyl)isoindolin-1-one;
5-(3-chloro-4-(2-chloro-3'-fluoro-5'-methoxy-4'-(((((R)-5-oxopyrrolidin-2-yl)methyl)amino)methyl)-[1,1'-biphenyl]-3-yl)pyridin-2-yl)-2-(2-((((R)-5-oxopyrrolidin-2-yl)methyl)amino)ethyl)isoindolin-1-one;
2-(4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)-2-azaspiro[3.3]heptane-6-carboxylic acid;
2-(4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)-2,6-diazaspiro[3.4]octan-7-one;
isopropyl (S)-1-(4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)pyrrolidine-3-carboxylate;
isopropyl (R)-1-(4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)pyrrolidine-3-carboxylate;
1-(4-((4-(4-(3-(3-(((1-acetylpiperidin-4-yl)amino)methyl)-1-methyl-1H-pyrrolo[2,3-b]pyridin-6-yl)-2-chlorophenyl)-3-chloropyridin-2-yl)-2-methoxybenzyl)amino)piperidin-1-yl)ethan-1-one;
(S)-5-((((6-(2-chloro-3-(3-chloro-2-(3-(((2-hydroxyethyl)(methyl)amino)methyl)-1-methyl-1H-indazol-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(3-(((2-hydroxyethyl)(methyl)amino)methyl)-1-methyl-1H-indazol-6-yl)pyridin-4-yl)phenyl)-2-methoxypyridin-3-yl)methyl)amino)methyl)pyrrolidin-2-one;
(S)-1-(6-((6-((4-(5-((6-acetyl-2,6-diazaspiro[3.3]heptan-2-yl)methyl)-3'-chloro-6-methoxy-[2,4'-bipyridin]-2'-yl)-2,3-dihydro-1H-inden-1-yl)oxy)-2-methoxy-5-(trifluoromethyl)pyridin-3-yl)methyl)-2,6-diazaspiro[3.3]heptan-2-yl)ethan-1-one;
(R)-1-(6-((6-((4-(5-((6-acetyl-2,6-diazaspiro[3.3]heptan-2-yl)methyl)-3'-chloro-6-methoxy-[2,4'-bipyridin]-2'-yl)-2,3-dihydro-1H-inden-1-yl)oxy)-2-methoxy-5-(trifluoromethyl)pyridin-3-yl)methyl)-2,6-diazaspiro[3.3]heptan-2-yl)ethan-1-one;
1-(6-((6-(2-chloro-3-(3-chloro-2-(2-(3-fluoropropyl)-8-methoxy-1,2,3,4-tetrahydroisoquinolin-6-yl)pyridin-4-yl)phenyl)-2-methoxypyridin-3-yl)methyl)-2,6-diazaspiro[3.3]heptan-2-yl)ethan-1-one;
2-((6-(2-chloro-3-(3-chloro-2-(2-(3-fluoropropyl)-8-methoxy-1,2,3,4-tetrahydroisoquinolin-6-yl)pyridin-4-yl)phenyl)-2-methoxypyridin-3-yl)methyl)-2,6-diazaspiro[3.4]octan-7-one;
N-((6-(2-chloro-3-(3-chloro-2-(2-(3-fluoropropyl)-8-methoxy-1,2,3,4-tetrahydroisoquinolin-6-yl)pyridin-4-yl)phenyl)-2-methoxypyridin-3-yl)methyl)-2-oxaspiro[3.3]heptan-6-amine;
(1-((((6-(2-chloro-3-(3-chloro-2-(2-(3-fluoropropyl)-8-methoxy-1,2,3,4-tetrahydroisoquinolin-6-yl)pyridin-4-yl)phenyl)-2-methoxypyridin-3-yl)methyl)amino)methyl)cyclopropyl)methanol;
(S)-1-(((3'-chloro-2'-((S)-1-((5-((((S)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)propan-2-ol;
(S)-1-(((3'-chloro-2'-((R)-1-((5-((((R)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)propan-2-ol;
(S)-1-(((3'-chloro-2'-((R)-1-((5-((((S)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)propan-2-ol;
(S)-1-(((3'-chloro-2'-((S)-1-((5-((((R)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)propan-2-ol;
(R)-1-(((3'-chloro-2'-((R)-1-((5-((((R)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)propan-2-ol;
(R)-1-(((3'-chloro-2'-((R)-1-((5-((((S)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)propan-2-ol;
(R)-1-(((3'-chloro-2'-((S)-1-((5-((((R)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)propan-2-ol;
(R)-1-(((3'-chloro-2'-((S)-1-((5-((((S)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)propan-2-ol;
(S)-1-(4-(((6-((4-(5-(((1-acetylpiperidin-4-yl)amino)methyl)-3'-chloro-6-methoxy-[2,4'-bipyridin]-2'-yl)-2,3-dihydro-1H-inden-1-yl)oxy)-2-methoxy-5-(trifluoromethyl)pyridin-3-yl)methyl)amino)piperidin-1-yl)ethan-1-one;
(R)-1-(4-(((6-((4-(5-(((1-acetylpiperidin-4-yl)amino)methyl)-3'-chloro-6-methoxy-[2,4'-bipyridin]-2'-yl)-2,3-dihydro-1H-inden-1-yl)oxy)-2-methoxy-5-(trifluoromethyl)pyridin-3-yl)methyl)amino)piperidin-1-yl)ethan-1-one;
2-((6-(2-chloro-3-(3-chloro-2-((R)-5-((((S)-5-oxopyrrolidin-2-yl)methyl)amino)-5,6,7,8-tetrahydronaphthalen-2-yl)pyridin-4-yl)phenyl)-2-methoxypyridin-3-yl)methyl)-2,6-diazaspiro[3.4]octan-7-one;
2-((6-(2-chloro-3-(3-chloro-2-((S)-5-((((S)-5-oxopyrrolidin-2-yl)methyl)amino)-5,6,7,8-tetrahydronaphthalen-2-yl)pyridin-4-yl)phenyl)-2-methoxypyridin-3-yl)methyl)-2,6-diazaspiro[3.4]octan-7-one;
2-((6-(2-chloro-3-(3-chloro-2-((R)-5-((((R)-5-oxopyrrolidin-2-yl)methyl)amino)-5,6,7,8-tetrahydronaphthalen-2-yl)pyridin-4-yl)phenyl)-2-methoxypyridin-3-yl)methyl)-2,6-diazaspiro[3.4]octan-7-one;
2-((6-(2-chloro-3-(3-chloro-2-((S)-5-((((R)-5-oxopyrrolidin-2-yl)methyl)amino)-5,6,7,8-tetrahydronaphthalen-2-yl)pyridin-4-yl)phenyl)-2-methoxypyridin-3-yl)methyl)-2,6-diazaspiro[3.4]octan-7-one;
(S)-5-((((R)-6-(3-chloro-4-(2-chloro-3-(5-((((S)-2-hydroxypropyl)amino)methyl)-6-methoxypyridin-2-yl)phenyl)pyridin-2-yl)-1,2,3,4-tetrahydronaphthalen-1-yl)amino)methyl)pyrrolidin-2-one;
(S)-5-((((R)-6-(3-chloro-4-(2-chloro-3-(5-((((R)-2-hydroxypropyl)amino)methyl)-6-methoxypyridin-2-yl)phenyl)pyridin-2-yl)-1,2,3,4-tetrahydronaphthalen-1-yl)amino)methyl)pyrrolidin-2-one;
(S)-5-((((S)-6-(3-chloro-4-(2-chloro-3-(5-((((R)-2-hydroxypropyl)amino)methyl)-6-methoxypyridin-2-yl)phenyl)pyridin-2-yl)-1,2,3,4-tetrahydronaphthalen-1-yl)amino)methyl)pyrrolidin-2-one;
(S)-5-((((S)-6-(3-chloro-4-(2-chloro-3-(5-((((S)-2-hydroxypropyl)amino)methyl)-6-methoxypyridin-2-yl)phenyl)pyridin-2-yl)-1,2,3,4-tetrahydronaphthalen-1-yl)amino)methyl)pyrrolidin-2-one;
(R)-5-((((R)-6-(3-chloro-4-(2-chloro-3-(5-((((R)-2-hydroxypropyl)amino)methyl)-6-methoxypyridin-2-yl)phenyl)pyridin-2-yl)-1,2,3,4-tetrahydronaphthalen-1-yl)amino)methyl)pyrrolidin-2-one;
(R)-5-((((R)-6-(3-chloro-4-(2-chloro-3-(5-((((S)-2-hydroxypropyl)amino)methyl)-6-methoxypyridin-2-yl)phenyl)pyridin-2-yl)-1,2,3,4-tetrahydronaphthalen-1-yl)amino)methyl)pyrrolidin-2-one;
(R)-5-((((S)-6-(3-chloro-4-(2-chloro-3-(5-((((R)-2-hydroxypropyl)amino)methyl)-6-methoxypyridin-2-yl)phenyl)pyridin-2-yl)-1,2,3,4-tetrahydronaphthalen-1-yl)amino)methyl)pyrrolidin-2-one;
(R)-5-((((S)-6-(3-chloro-4-(2-chloro-3-(5-((((S)-2-hydroxypropyl)amino)methyl)-6-methoxypyridin-2-yl)phenyl)pyridin-2-yl)-1,2,3,4-tetrahydronaphthalen-1-yl)amino)methyl)pyrrolidin-2-one;
(S)-5-((((R)-6-(3-chloro-4-(2-chloro-3-(6-methoxy-5-((((S)-2-oxotetrahydrofuran-3-yl)amino)methyl)pyridin-2-yl)phenyl)pyridin-2-yl)-1,2,3,4-tetrahydronaphthalen-1-yl)amino)methyl)pyrrolidin-2-one;
(S)-5-((((S)-6-(3-chloro-4-(2-chloro-3-(6-methoxy-5-((((S)-2-oxotetrahydrofuran-3-yl)amino)methyl)pyridin-2-yl)phenyl)pyridin-2-yl)-1,2,3,4-tetrahydronaphthalen-1-yl)amino)methyl)pyrrolidin-2-one;
(S)-5-((((S)-6-(3-chloro-4-(2-chloro-3-(6-methoxy-5-((((R)-2-oxotetrahydrofuran-3-yl)amino)methyl)pyridin-2-yl)phenyl)pyridin-2-yl)-1,2,3,4-tetrahydronaphthalen-1-yl)amino)methyl)pyrrolidin-2-one;
(S)-5-((((R)-6-(3-chloro-4-(2-chloro-3-(6-methoxy-5-((((R)-2-oxotetrahydrofuran-3-yl)amino)methyl)pyridin-2-yl)phenyl)pyridin-2-yl)-1,2,3,4-tetrahydronaphthalen-1-yl)amino)methyl)pyrrolidin-2-one;
(R)-5-((((R)-6-(3-chloro-4-(2-chloro-3-(6-methoxy-5-((((S)-2-oxotetrahydrofuran-3-yl)amino)methyl)pyridin-2-yl)phenyl)pyridin-2-yl)-1,2,3,4-tetrahydronaphthalen-1-yl)amino)methyl)pyrrolidin-2-one;
(R)-5-((((S)-6-(3-chloro-4-(2-chloro-3-(6-methoxy-5-((((S)-2-oxotetrahydrofuran-3-yl)amino)methyl)pyridin-2-yl)phenyl)pyridin-2-yl)-1,2,3,4-tetrahydronaphthalen-1-yl)amino)methyl)pyrrolidin-2-one;
(R)-5-((((S)-6-(3-chloro-4-(2-chloro-3-(6-methoxy-5-((((R)-2-oxotetrahydrofuran-3-yl)amino)methyl)pyridin-2-yl)phenyl)pyridin-2-yl)-1,2,3,4-tetrahydronaphthalen-1-yl)amino)methyl)pyrrolidin-2-one;
(S)-5-((((S)-6-(3-chloro-4-(2-chloro-3-(6-methoxy-5-((((S)-2-oxotetrahydrofuran-3-yl)amino)methyl)pyridin-2-yl)phenyl)pyridin-2-yl)-1,2,3,4-tetrahydronaphthalen-1-yl)amino)methyl)pyrrolidin-2-one;
(S)-5-((((6-(2-chloro-3-(3-chloro-2-(8-methoxy-2-(((((S)-5-oxopyrrolidin-2-yl)methyl)amino)methyl)-[1,2,4]triazolo[1,5-a]pyridin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(8-methoxy-2-(((((R)-5-oxopyrrolidin-2-yl)methyl)amino)methyl)-[1,2,4]triazolo[1,5-a]pyridin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(8-methoxy-2-(((((S)-5-oxopyrrolidin-2-yl)methyl)amino)methyl)-[1,2,4]triazolo[1,5-a]pyridin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(8-methoxy-2-(((((R)-5-oxopyrrolidin-2-yl)methyl)amino)methyl)-[1,2,4]triazolo[1,5-a]pyridin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(2-((3-hydroxy-3-methylazetidin-1-yl)methyl)-8-methoxy-[1,2,4]triazolo[1,5-a]pyridin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-((3-hydroxy-3-methylazetidin-1-yl)methyl)-8-methoxy-[1,2,4]triazolo[1,5-a]pyridin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(2-((((1r,4r)-4-hydroxycyclohexyl)amino)methyl)-8-methoxy-[1,2,4]triazolo[1,5-a]pyridin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(2-((((1s,4s)-4-hydroxycyclohexyl)amino)methyl)-8-methoxy-[1,2,4]triazolo[1,5-a]pyridin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(2-((((1s,4r)-4-hydroxycyclohexyl)amino)methyl)-8-methoxy-[1,2,4]triazolo[1,5-a]pyridin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(2-((((1r,4s)-4-hydroxycyclohexyl)amino)methyl)-8-methoxy-[1,2,4]triazolo[1,5-a]pyridin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-((((1r,4s)-4-hydroxycyclohexyl)amino)methyl)-8-methoxy-[1,2,4]triazolo[1,5-a]pyridin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-((((1s,4s)-4-hydroxycyclohexyl)amino)methyl)-8-methoxy-[1,2,4]triazolo[1,5-a]pyridin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-((((1s,4r)-4-hydroxycyclohexyl)amino)methyl)-8-methoxy-[1,2,4]triazolo[1,5-a]pyridin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-((((1r,4r)-4-hydroxycyclohexyl)amino)methyl)-8-methoxy-[1,2,4]triazolo[1,5-a]pyridin-6-yl)pyridin-4-yl)phenyl)-2-methoxypyridin-3-yl)methyl)amino)methyl)pyrrolidin-2-one;
(S)-1-((6-(3-chloro-4-(2-chloro-3-(6-methoxy-5-((((5-oxopyrrolidin-2-yl)methyl)amino)methyl)pyridin-2-yl)phenyl)pyridin-2-yl)-8-methoxy-[1,2,4]triazolo[1,5-a]pyridin-2-yl)methyl)azetidine-3-carboxylic acid;
(R)-1-((6-(3-chloro-4-(2-chloro-3-(6-methoxy-5-((((5-oxopyrrolidin-2-yl)methyl)amino)methyl)pyridin-2-yl)phenyl)pyridin-2-yl)-8-methoxy-[1,2,4]triazolo[1,5-a]pyridin-2-yl)methyl)azetidine-3-carboxylic acid;
(S)-1-(2-(((6-(3-chloro-4-(2-chloro-3-(6-methoxy-5-((((5-oxopyrrolidin-2-yl)methyl)amino)methyl)pyridin-2-yl)phenyl)pyridin-2-yl)-8-methoxy-[1,2,4]triazolo[1,5-a]pyridin-2-yl)methyl)amino)ethyl)cyclopropane-1-carboxylic acid;
(R)-1-(2-(((6-(3-chloro-4-(2-chloro-3-(6-methoxy-5-((((5-oxopyrrolidin-2-yl)methyl)amino)methyl)pyridin-2-yl)phenyl)pyridin-2-yl)-8-methoxy-[1,2,4]triazolo[1,5-a]pyridin-2-yl)methyl)amino)ethyl)cyclopropane-1-carboxylic acid;
1-(4-(((6-(3-(2-(3-(((1-acetylpiperidin-4-yl)amino)methyl)-1-methyl-1H-indazol-6-yl)-3-chloropyridin-4-yl)-2-chlorophenyl)-2-methoxypyridin-3-yl)methyl)amino)piperidin-1-yl)ethan-1-one;
1-(4-((4-(4-(3-(3-(((1-acetylpiperidin-4-yl)(methyl)amino)methyl)-1-methyl-1H-pyrrolo[2,3-b]pyridin-6-yl)-2-chlorophenyl)-3-chloropyridin-2-yl)-2-methoxybenzyl)amino)piperidin-1-yl)ethan-1-one;
((6-(2-chloro-3-(3-chloro-2-((R)-5-((((S)-5-oxopyrrolidin-2-yl)methyl)amino)-5,6,7, 8-tetrahydronaphthalen-2-yl)pyridin-4-yl)phenyl)-2-methoxypyridin-3-yl)methyl)-L-homoserine;
((6-(2-chloro-3-(3-chloro-2-((R)-5-((((R)-5-oxopyrrolidin-2-yl)methyl)amino)-5,6,7,8-tetrahydronaphthalen-2-yl)pyridin-4-yl)phenyl)-2-methoxypyridin-3-yl)methyl)-L-homoserine;
((6-(2-chloro-3-(3-chloro-2-((S)-5-((((S)-5-oxopyrrolidin-2-yl)methyl)amino)-5,6,7,8-tetrahydronaphthalen-2-yl)pyridin-4-yl)phenyl)-2-methoxypyridin-3-yl)methyl)-L-homoserine;
((6-(2-chloro-3-(3-chloro-2-((S)-5-((((R)-5-oxopyrrolidin-2-yl)methyl)amino)-5,6,7,8-tetrahydronaphthalen-2-yl)pyridin-4-yl)phenyl)-2-methoxypyridin-3-yl)methyl)-L-homoserine;
((6-(2-chloro-3-(3-chloro-2-((R)-5-((((R)-5-oxopyrrolidin-2-yl)methyl)amino)-5,6,7,8-tetrahydronaphthalen-2-yl)pyridin-4-yl)phenyl)-2-methoxypyridin-3-yl)methyl)-D-homoserine;
((6-(2-chloro-3-(3-chloro-2-((S)-5-((((S)-5-oxopyrrolidin-2-yl)methyl)amino)-5,6,7,8-tetrahydronaphthalen-2-yl)pyridin-4-yl)phenyl)-2-methoxypyridin-3-yl)methyl)-D-homoserine;
((6-(2-chloro-3-(3-chloro-2-((S)-5-((((R)-5-oxopyrrolidin-2-yl)methyl)amino)-5,6,7,8-tetrahydronaphthalen-2-yl)pyridin-4-yl)phenyl)-2-methoxypyridin-3-yl)methyl)-D-homoserine;
((6-(2-chloro-3-(3-chloro-2-((R)-5-((((S)-5-oxopyrrolidin-2-yl)methyl)amino)-5,6,7, 8-tetrahydronaphthalen-2-yl)pyridin-4-yl)phenyl)-2-methoxypyridin-3-yl)methyl)-D-homoserine;
(5S,5'S)-5,5'-(((3,3'-dichloro-[4,4'-bipyridine]-2,2'-diyl)bis(8-methoxy-3,4-dihydroisoquinoline-6,2(1H)-diyl))bis(methylene))bis(pyrrolidin-2-one);
(5S,5'R)-5,5'-(((3,3'-dichloro-[4,4'-bipyridine]-2,2'-diyl)bis(8-methoxy-3,4-dihydroisoquinoline-6,2(1H)-diyl))bis(methylene))bis(pyrrolidin-2-one);
(5R,5'S)-5,5'-(((3,3'-dichloro-[4,4'-bipyridine]-2,2'-diyl)bis(8-methoxy-3,4-dihydroisoquinoline-6,2(1H)-diyl))bis(methylene))bis(pyrrolidin-2-one);
(5R,5'R)-5,5'-(((3,3'-dichloro-[4,4'-bipyridine]-2,2'-diyl)bis(8-methoxy-3,4-dihydroisoquinoline-6,2(1H)-diyl))bis(methylene))bis(pyrrolidin-2-one);
methyl ((3-chloro-4-(2-chloro-3-(6-methoxy-5-(((((S)-5-oxopyrrolidin-2-yl)methyl)amino)methyl)pyridin-2-yl)phenyl)-5'-methoxy-[2,3'-bipyridin]-6'-yl)methyl)-L-homoserinate;
methyl ((3-chloro-4-(2-chloro-3-(6-methoxy-5-(((((R)-5-oxopyrrolidin-2-yl)methyl)amino)methyl)pyridin-2-yl)phenyl)-5'-methoxy-[2,3'-bipyridin]-6'-yl)methyl)-L-homoserinate;
methyl ((3-chloro-4-(2-chloro-3-(6-methoxy-5-(((((R)-5-oxopyrrolidin-2-yl)methyl)amino)methyl)pyridin-2-yl)phenyl)-5'-methoxy-[2,3'-bipyridin]-6'-yl)methyl)-D-homoserinate;
methyl ((3-chloro-4-(2-chloro-3-(6-methoxy-5-(((((S)-5-oxopyrrolidin-2-yl)methyl)amino)methyl)pyridin-2-yl)phenyl)-5'-methoxy-[2,3'-bipyridin]-6'-yl)methyl)-D-homoserinate;
(S)-5-((((6-(2-chloro-3-(3-chloro-5'-methoxy-6'-((((S)-2-oxotetrahydrofuran-3-yl)amino)methyl)-[2,3'-bipyridin]-4-yl)phenyl)-2-methoxypyridin-3-yl)methyl)amino)methyl)pyrrolidin-2-one;
(S)-5-((((6-(2-chloro-3-(3-chloro-5'-methoxy-6'-((((R)-2-oxotetrahydrofuran-3-yl)amino)methyl)-[2,3'-bipyridin]-4-yl)phenyl)-2-methoxypyridin-3-yl)methyl)amino)methyl)pyrrolidin-2-one;
(R)-5-((((6-(2-chloro-3-(3-chloro-5'-methoxy-6'-((((S)-2-oxotetrahydrofuran-3-yl)amino)methyl)-[2,3'-bipyridin]-4-yl)phenyl)-2-methoxypyridin-3-yl)methyl)amino)methyl)pyrrolidin-2-one;
(R)-5-((((6-(2-chloro-3-(3-chloro-5'-methoxy-6'-((((R)-2-oxotetrahydrofuran-3-yl)amino)methyl)-[2,3'-bipyridin]-4-yl)phenyl)-2-methoxypyridin-3-yl)methyl)amino)methyl)pyrrolidin-2-one;
(S)-5-((((6-(((S)-4-(3-chloro-2-(3-methoxy-4-(((((S)-5-oxopyrrolidin-2-yl)methyl)amino)methyl)phenyl)pyridin-4-yl)-2,3-dihydro-1H-inden-1-yl)oxy)-2-methoxy-5-(trifluoromethyl)pyridin-3-yl)methyl)amino)methyl)pyrrolidin-2-one;
(S)-5-((((6-(((S)-4-(3-chloro-2-(3-methoxy-4-(((((R)-5-oxopyrrolidin-2-yl)methyl)amino)methyl)phenyl)pyridin-4-yl)-2,3-dihydro-1H-inden-1-yl)oxy)-2-methoxy-5-(trifluoromethyl)pyridin-3-yl)methyl)amino)methyl)pyrrolidin-2-one;
(S)-5-((((6-(((R)-4-(3-chloro-2-(3-methoxy-4-(((((S)-5-oxopyrrolidin-2-yl)methyl)amino)methyl)phenyl)pyridin-4-yl)-2,3-dihydro-1H-inden-1-yl)oxy)-2-methoxy-5-(trifluoromethyl)pyridin-3-yl)methyl)amino)methyl)pyrrolidin-2-one;
(S)-5-((((6-(((S)-4-(3-chloro-2-(3-methoxy-4-(((((R)-5-oxopyrrolidin-2-yl)methyl)amino)methyl)phenyl)pyridin-4-yl)-2,3-dihydro-1H-inden-1-yl)oxy)-2-methoxy-5-(trifluoromethyl)pyridin-3-yl)methyl)amino)methyl)pyrrolidin-2-one;
(R)-5-((((6-(((S)-4-(3-chloro-2-(3-methoxy-4-(((((S)-5-oxopyrrolidin-2-yl)methyl)amino)methyl)phenyl)pyridin-4-yl)-2,3-dihydro-1H-inden-1-yl)oxy)-2-methoxy-5-(trifluoromethyl)pyridin-3-yl)methyl)amino)methyl)pyrrolidin-2-one;
(R)-5-((((6-(((S)-4-(3-chloro-2-(3-methoxy-4-(((((R)-5-oxopyrrolidin-2-yl)methyl)amino)methyl)phenyl)pyridin-4-yl)-2,3-dihydro-1H-inden-1-yl)oxy)-2-methoxy-5-(trifluoromethyl)pyridin-3-yl)methyl)amino)methyl)pyrrolidin-2-one;
(R)-5-((((6-(((R)-4-(3-chloro-2-(3-methoxy-4-(((((S)-5-oxopyrrolidin-2-yl)methyl)amino)methyl)phenyl)pyridin-4-yl)-2,3-dihydro-1H-inden-1-yl)oxy)-2-methoxy-5-(trifluoromethyl)pyridin-3-yl)methyl)amino)methyl)pyrrolidin-2-one;
(R)-5-((((6-(((S)-4-(3-chloro-2-(3-methoxy-4-(((((R)-5-oxopyrrolidin-2-yl)methyl)amino)methyl)phenyl)pyridin-4-yl)-2,3-dihydro-1H-inden-1-yl)oxy)-2-methoxy-5-(trifluoromethyl)pyridin-3-yl)methyl)amino)methyl)pyrrolidin-2-one;
(S)-1-(((6-(((S)-4-(3-chloro-2-(4-((((S)-2-hydroxypropyl)amino)methyl)-3-methoxyphenyl)pyridin-4-yl)-2,3-dihydro-1H-inden-1-yl)oxy)-2-methoxy-5-(trifluoromethyl)pyridin-3-yl)methyl)amino)propan-2-ol;
(S)-1-(((6-(((S)-4-(3-chloro-2-(4-((((R)-2-hydroxypropyl)amino)methyl)-3-methoxyphenyl)pyridin-4-yl)-2,3-dihydro-1H-inden-1-yl)oxy)-2-methoxy-5-(trifluoromethyl)pyridin-3-yl)methyl)amino)propan-2-ol;
(S)-1-(((6-(((R)-4-(3-chloro-2-(4-((((S)-2-hydroxypropyl)amino)methyl)-3-methoxyphenyl)pyridin-4-yl)-2,3-dihydro-1H-inden-1-yl)oxy)-2-methoxy-5-(trifluoromethyl)pyridin-3-yl)methyl)amino)propan-2-ol;
(S)-1-(((6-(((R)-4-(3-chloro-2-(4-((((R)-2-hydroxypropyl)amino)methyl)-3-methoxyphenyl)pyridin-4-yl)-2,3-dihydro-1H-inden-1-yl)oxy)-2-methoxy-5-(trifluoromethyl)pyridin-3-yl)methyl)amino)propan-2-ol;
(R)-1-(((6-(((S)-4-(3-chloro-2-(4-((((S)-2-hydroxypropyl)amino)methyl)-3-methoxyphenyl)pyridin-4-yl)-2,3-dihydro-1H-inden-1-yl)oxy)-2-methoxy-5-(trifluoromethyl)pyridin-3-yl)methyl)amino)propan-2-ol;
(R)-1-(((6-(((S)-4-(3-chloro-2-(4-((((R)-2-hydroxypropyl)amino)methyl)-3-methoxyphenyl)pyridin-4-yl)-2,3-dihydro-1H-inden-1-yl)oxy)-2-methoxy-5-(trifluoromethyl)pyridin-3-yl)methyl)amino)propan-2-ol;
(R)-1-(((6-(((R)-4-(3-chloro-2-(4-((((S)-2-hydroxypropyl)amino)methyl)-3-methoxyphenyl)pyridin-4-yl)-2,3-dihydro-1H-inden-1-yl)oxy)-2-methoxy-5-(trifluoromethyl)pyridin-3-yl)methyl)amino)propan-2-ol;
(R)-1-(((6-(((R)-4-(3-chloro-2-(4-((((R)-2-hydroxypropyl)amino)methyl)-3-methoxyphenyl)pyridin-4-yl)-2,3-dihydro-1H-inden-1-yl)oxy)-2-methoxy-5-(trifluoromethyl)pyridin-3-yl)methyl)amino)propan-2-ol;
1-(4-(((6-(3-(2-(4-(((1-acetylpiperidin-4-yl)amino)methyl)-3-methoxyphenyl)-3-methylpyridin-4-yl)-2-chlorophenyl)-2-methoxypyridin-3-yl)methyl)amino)piperidin-1-yl)ethan-1-one;
methyl (S)-3-(6-(3-chloro-4-(2-chloro-3-(5-(((2-hydroxypropyl)amino)methyl)-6-methoxypyridin-2-yl)phenyl)pyridin-2-yl)-8-methoxy-3,4-dihydroisoquinolin-2(1H)-yl)propanoate;
methyl (R)-3-(6-(3-chloro-4-(2-chloro-3-(5-(((2-hydroxypropyl)amino)methyl)-6-methoxypyridin-2-yl)phenyl)pyridin-2-yl)-8-methoxy-3,4-dihydroisoquinolin-2(1H)-yl)propanoate;
methyl 3-(6-(3-chloro-4-(2-chloro-3-(6-methoxy-5-((7-oxo-2,6-diazaspiro[3.4]octan-2-yl)methyl)pyridin-2-yl)phenyl)pyridin-2-yl)-8-methoxy-3,4-dihydroisoquinolin-2(1H)-yl)propanoate;
(S)-1-(((6-(2-chloro-3-(2-(4-((((S)-2-hydroxypropyl)amino)methyl)-3-methoxyphenyl)-3-methylpyridin-4-yl)phenyl)-2-methoxypyridin-3-yl)methyl)amino)propan-2-ol;
(S)-1-(((6-(2-chloro-3-(2-(4-((((R)-2-hydroxypropyl)amino)methyl)-3-methoxyphenyl)-3-methylpyridin-4-yl)phenyl)-2-methoxypyridin-3-yl)methyl)amino)propan-2-ol;
(R)-1-(((6-(2-chloro-3-(2-(4-((((S)-2-hydroxypropyl)amino)methyl)-3-methoxyphenyl)-3-methylpyridin-4-yl)phenyl)-2-methoxypyridin-3-yl)methyl)amino)propan-2-ol;
(R)-1-(((6-(2-chloro-3-(2-(4-((((R)-2-hydroxypropyl)amino)methyl)-3-methoxyphenyl)-3-methylpyridin-4-yl)phenyl)-2-methoxypyridin-3-yl)methyl)amino)propan-2-ol;
(S)-2-(6-(3-chloro-4-(2-chloro-3-(5-(((2-hydroxypropyl)amino)methyl)-6-methoxypyridin-2-yl)phenyl)pyridin-2-yl)-8-methoxy-3,4-dihydroisoquinolin-2(1H)-yl)acetamide;
(R)-2-(6-(3-chloro-4-(2-chloro-3-(5-(((2-hydroxypropyl)amino)methyl)-6-methoxypyridin-2-yl)phenyl)pyridin-2-yl)-8-methoxy-3,4-dihydroisoquinolin-2(1H)-yl)acetamide;
1-(6-(2-chloro-3-(3-chloro-2-(1-methyl-3-((methylamino)methyl)-1H-indol-6-yl)pyridin-4-yl)phenyl)-2-methoxypyridin-3-yl)-N-methylmethanamine;
1-(4-(((6-(3-(2-(3-(((1-acetylpiperidin-4-yl)amino)methyl)-1-methyl-1H-indol-6-yl)-3-chloropyridin-4-yl)-2-chlorophenyl)-2-methoxypyridin-3-yl)methyl)amino)piperidin-1-yl)ethan-1-one;
isopropyl (S)-1-(4-(3-chloro-4-(2-chloro-3-(6-methoxy-5-((methylamino)methyl)pyridin-2-yl)phenyl)pyridin-2-yl)-2-methoxybenzyl)pyrrolidine-3-carboxylate;
isopropyl (R)-1-(4-(3-chloro-4-(2-chloro-3-(6-methoxy-5-((methylamino)methyl)pyridin-2-yl)phenyl)pyridin-2-yl)-2-methoxybenzyl)pyrrolidine-3-carboxylate;
isopropyl 3-((4-(3-chloro-4-(2-chloro-3-(6-methoxy-5-((methylamino)methyl)pyridin-2-yl)phenyl)pyridin-2-yl)-2-methoxybenzyl)amino)propanoate;
isopropyl (S)-4-((4-(3-chloro-4-(2-chloro-3-(6-methoxy-5-((methylamino)methyl)pyridin-2-yl)phenyl)pyridin-2-yl)-2-methoxybenzyl)amino)-3-hydroxybutanoate;
isopropyl (R)-4-((4-(3-chloro-4-(2-chloro-3-(6-methoxy-5-((methylamino)methyl)pyridin-2-yl)phenyl)pyridin-2-yl)-2-methoxybenzyl)amino)-3-hydroxybutanoate;
isopropyl 2-(4-(3-chloro-4-(2-chloro-3-(6-methoxy-5-((methylamino)methyl)pyridin-2-yl)phenyl)pyridin-2-yl)-2-methoxybenzyl)-2-azaspiro[3.3]heptane-6-carboxylate;
1-(4-(((6-(3-(2-(4-(((1-acetylpiperidin-4-yl)amino)methyl)-3-fluorophenyl)-3-chloropyridin-4-yl)-2-chlorophenyl)-2-methoxypyridin-3-yl)methyl)amino)piperidin-1-yl)ethan-1-one;
1-(6-(2-chloro-3-(3-chloro-2-(3-fluoro-4-((methylamino)methyl)phenyl)pyridin-4-yl)phenyl)-2-methoxypyridin-3-yl)-N-methylmethanamine;
2-((6-(2-chloro-3-(3-chloro-2-(3-fluoro-4-((7-oxo-2,6-diazaspiro[3.4]octan-2-yl)methyl)phenyl)pyridin-4-yl)phenyl)-2-methoxypyridin-3-yl)methyl)-2,6-diazaspiro[3.4]octan-7-one;
1-(4-(((6-(3-(2-(4-(((1-acetylpiperidin-4-yl)amino)methyl)-3-fluoro-5-methoxyphenyl)-3-chloropyridin-4-yl)-2-chlorophenyl)-2-methoxypyridin-3-yl)methyl)amino)piperidin-1-yl)ethan-1-one;
1-(6-(2-chloro-3-(3-chloro-2-(3-fluoro-5-methoxy-4-((methylamino)methyl)phenyl)pyridin-4-yl)phenyl)-2-methoxypyridin-3-yl)-N-methylmethanamine;
2-(4-(3-chloro-4-(2-chloro-3-(6-methoxy-5-((7-oxo-2,6-diazaspiro[3.4]octan-2-yl)methyl)pyridin-2-yl)phenyl)pyridin-2-yl)-2-fluoro-6-methoxybenzyl)-2,6-diazaspiro[3.4]octan-7-one;
(S)-2-((6-(3-chloro-4-(2-chloro-3-(6-methoxy-5-((((5-oxopyrrolidin-2-yl)methyl)amino)methyl)pyridin-2-yl)phenyl)pyridin-2-yl)-1-methyl-1H-indazol-3-yl)methyl)-2,6-diazaspiro[3.4]octan-7-one;
(R)-2-((6-(3-chloro-4-(2-chloro-3-(6-methoxy-5-((((5-oxopyrrolidin-2-yl)methyl)amino)methyl)pyridin-2-yl)phenyl)pyridin-2-yl)-1-methyl-1H-indazol-3-yl)methyl)-2,6-diazaspiro[3.4]octan-7-one;
(S)-5-((((6-(3-(2-(2-((1-acetylpiperidin-4-yl)methyl)-8-chloro-1,2,3,4-tetrahydroisoquinolin-6-yl)-3-chloropyridin-4-yl)-2-chlorophenyl)-2-methoxypyridin-3-yl)methyl)amino)methyl)pyrrolidin-2-one;
(R)-5-((((6-(3-(2-(2-((1-acetylpiperidin-4-yl)methyl)-8-chloro-1,2,3,4-tetrahydroisoquinolin-6-yl)-3-chloropyridin-4-yl)-2-chlorophenyl)-2-methoxypyridin-3-yl)methyl)amino)methyl)pyrrolidin-2-one;
6-(2-chloro-3-(3-chloro-2-(3-methoxy-4-(((((S)-5-oxopyrrolidin-2-yl)methyl)amino)methyl)phenyl)pyridin-4-yl)phenyl)-2-(2-((((S)-5-oxopyrrolidin-2-yl)methyl)amino)ethyl)-1,2-dihydro-3H-pyrrolo[3,4-c]pyridin-3-one;
6-(2-chloro-3-(3-chloro-2-(3-methoxy-4-(((((S)-5-oxopyrrolidin-2-yl)methyl)amino)methyl)phenyl)pyridin-4-yl)phenyl)-2-(2-((((R)-5-oxopyrrolidin-2-yl)methyl)amino)ethyl)-1,2-dihydro-3H-pyrrolo[3,4-c]pyridin-3-one;
6-(2-chloro-3-(3-chloro-2-(3-methoxy-4-(((((R)-5-oxopyrrolidin-2-yl)methyl)amino)methyl)phenyl)pyridin-4-yl)phenyl)-2-(2-((((S)-5-oxopyrrolidin-2-yl)methyl)amino)ethyl)-1,2-dihydro-3H-pyrrolo[3,4-c]pyridin-3-one;
6-(2-chloro-3-(3-chloro-2-(3-methoxy-4-(((((R)-5-oxopyrrolidin-2-yl)methyl)amino)methyl)phenyl)pyridin-4-yl)phenyl)-2-(2-((((R)-5-oxopyrrolidin-2-yl)methyl)amino)ethyl)-1,2-dihydro-3H-pyrrolo[3,4-c]pyridin-3-one;
(S)-5-((((6-(2-chloro-3-(3-chloro-2-(3-methoxy-4-(((((S)-5-oxopyrrolidin-2-yl)methyl)amino)methyl)phenyl)pyridin-4-yl)phenyl)imidazo[1,2-a]pyrazin-2-yl)methyl)amino)methyl)pyrrolidin-2-one;
(S)-5-((((6-(2-chloro-3-(3-chloro-2-(3-methoxy-4-(((((R)-5-oxopyrrolidin-2-yl)methyl)amino)methyl)phenyl)pyridin-4-yl)phenyl)imidazo[1,2-a]pyrazin-2-yl)methyl)amino)methyl)pyrrolidin-2-one;
(R)-5-((((6-(2-chloro-3-(3-chloro-2-(3-methoxy-4-(((((S)-5-oxopyrrolidin-2-yl)methyl)amino)methyl)phenyl)pyridin-4-yl)phenyl)imidazo[1,2-a]pyrazin-2-yl)methyl)amino)methyl)pyrrolidin-2-one;
(R)-5-((((6-(2-chloro-3-(3-chloro-2-(3-methoxy-4-(((((R)-5-oxopyrrolidin-2-yl)methyl)amino)methyl)phenyl)pyridin-4-yl)phenyl)imidazo[1,2-a]pyrazin-2-yl)methyl)amino)methyl)pyrrolidin-2-one;
(S)-2-(4-(3-chloro-4-(1-((6-methoxy-5-((7-oxo-2,6-diazaspiro[3.4]octan-2-yl)methyl)-3-(trifluoromethyl)pyridin-2-yl)oxy)-2,3-dihydro-1H-inden-4-yl)pyridin-2-yl)-2-methoxybenzyl)-2,6-diazaspiro[3.4]octan-7-one;
(R)-2-(4-(3-chloro-4-(1-((6-methoxy-5-((7-oxo-2,6-diazaspiro[3.4]octan-2-yl)methyl)-3-(trifluoromethyl)pyridin-2-yl)oxy)-2,3-dihydro-1H-inden-4-yl)pyridin-2-yl)-2-methoxybenzyl)-2,6-diazaspiro[3.4]octan-7-one;
(S)-3-(6-(3-chloro-4-(2-chloro-3-(5-(((2-hydroxypropyl)amino)methyl)-6-methoxypyridin-2-yl)phenyl)pyridin-2-yl)-8-methoxy-3,4-dihydroisoquinolin-2(1H)-yl)propanoic acid;
(R)-3-(6-(3-chloro-4-(2-chloro-3-(5-(((2-hydroxypropyl)amino)methyl)-6-methoxypyridin-2-yl)phenyl)pyridin-2-yl)-8-methoxy-3,4-dihydroisoquinolin-2(1H)-yl)propanoic acid;
3-(6-(3-chloro-4-(2-chloro-3-(6-methoxy-5-((7-oxo-2,6-diazaspiro[3.4]octan-2-yl)methyl)pyridin-2-yl)phenyl)pyridin-2-yl)-8-methoxy-3,4-dihydroisoquinolin-2(1H)-yl)propanoic acid;
methyl 1-((6-(3-chloro-4-(2-chloro-3-(6-methoxy-5-((7-oxo-2,6-diazaspiro[3.4]octan-2-yl)methyl)pyridin-2-yl)phenyl)pyridin-2-yl)-8-methoxy-3,4-dihydroisoquinolin-2(1H)-yl)methyl)cyclopropane-1-carboxylate;
2-((6-(2-chloro-3-(3-chloro-2-(2-(3-fluoro-2-hydroxypropyl)-8-methoxy-1,2,3,4-tetrahydroisoquinolin-6-yl)pyridin-4-yl)phenyl)-2-methoxypyridin-3-yl)methyl)-2,6-diazaspiro[3.4]octan-7-one;
(S)-2-((6-(2-chloro-3-(3-chloro-2-(2-(3-fluoro-2-hydroxypropyl)-8-methoxy-1,2,3,4-tetrahydroisoquinolin-6-yl)pyridin-4-yl)phenyl)-2-methoxypyridin-3-yl)methyl)-2,6-diazaspiro[3.4]octan-7-one;
(R)-2-((6-(2-chloro-3-(3-chloro-2-(2-(3-fluoro-2-hydroxypropyl)-8-methoxy-1,2,3,4-tetrahydroisoquinolin-6-yl)pyridin-4-yl)phenyl)-2-methoxypyridin-3-yl)methyl)-2,6-diazaspiro[3.4]octan-7-one;
1-((6-(3-chloro-4-(2-chloro-3-(6-methoxy-5-((7-oxo-2,6-diazaspiro[3.4]octan-2-yl)methyl)pyridin-2-yl)phenyl)pyridin-2-yl)-8-methoxy-3,4-dihydroisoquinolin-2(1H)-yl)methyl)cyclopropane-1-carboxylic acid;
N-((6-(2-chloro-3-(3-chloro-2-(3-methoxy-4-(((tetrahydro-2H-pyran-4-yl)amino)methyl)phenyl)pyridin-4-yl)phenyl)-2-methoxypyridin-3-yl)methyl)tetrahydro-2H-pyran-4-amine;
4-(((6-(2-chloro-3-(3-chloro-2-(4-(((1,1-dioxidotetrahydro-2H-thiopyran-4-yl)amino)methyl)-3-methoxyphenyl)pyridin-4-yl)phenyl)-2-methoxypyridin-3-yl)methyl)amino)tetrahydro-2H-thiopyran 1,1-dioxide;
1-(3-(((6-(3-(2-(4-(((1-acetylazetidin-3-yl)amino)methyl)-3-methoxyphenyl)-3-chloropyridin-4-yl)-2-chlorophenyl)-2-methoxypyridin-3-yl)methyl)amino)azetidin-1-yl)ethan-1-one;
(S)-1-(((6-(2-chloro-3-(3-chloro-2-(4-((((S)-2-hydroxypropyl)amino)methyl)-3-methoxyphenyl)pyridin-4-yl)phenyl)-2-methoxypyridin-3-yl)methyl)amino)propan-2-ol;
(R)-1-(((6-(2-chloro-3-(3-chloro-2-(4-((((S)-2-hydroxypropyl)amino)methyl)-3-methoxyphenyl)pyridin-4-yl)phenyl)-2-methoxypyridin-3-yl)methyl)amino)propan-2-ol;
(S)-1-(((6-(2-chloro-3-(3-chloro-2-(4-((((R)-2-hydroxypropyl)amino)methyl)-3-methoxyphenyl)pyridin-4-yl)phenyl)-2-methoxypyridin-3-yl)methyl)amino)propan-2-ol;
(R)-1-(((6-(2-chloro-3-(3-chloro-2-(4-((((R)-2-hydroxypropyl)amino)methyl)-3-methoxyphenyl)pyridin-4-yl)phenyl)-2-methoxypyridin-3-yl)methyl)amino)propan-2-ol;
2-((4-(3-chloro-4-(2-chloro-3-(6-methoxy-5-(((5-oxo-6-azaspiro[3.4]octan-2-yl)amino)methyl)pyridin-2-yl)phenyl)pyridin-2-yl)-2-methoxybenzyl)amino)-6-azaspiro[3.4]octan-5-one;
1-(2-(((6-(2-chloro-3-(3-chloro-2-(3-methoxy-4-(((2-(2-oxopyrrolidin-1-yl)ethyl)amino)methyl)phenyl)pyridin-4-yl)phenyl)-2-methoxypyridin-3-yl)methyl)amino)ethyl)pyrrolidin-2-one;
(S)-2-((3'-chloro-6-methoxy-2'-(1-((6-methoxy-5-((7-oxo-2,6-diazaspiro[3.4]octan-2-yl)methyl)-3-(trifluoromethyl)pyrazin-2-yl)amino)-2,3-dihydro-1H-inden-4-yl)-[2,4'-bipyridin]-5-yl)methyl)-2,6-diazaspiro[3.4]octan-7-one;
(R)-2-((3'-chloro-6-methoxy-2'-(1-((6-methoxy-5-((7-oxo-2,6-diazaspiro[3.4]octan-2-yl)methyl)-3-(trifluoromethyl)pyrazin-2-yl)amino)-2,3-dihydro-1H-inden-4-yl)-[2,4'-bipyridin]-5-yl)methyl)-2,6-diazaspiro[3.4]octan-7-one;
(S)-1-(6-((5-((4-(5-((6-acetyl-2,6-diazaspiro[3.3]heptan-2-yl)methyl)-3'-chloro-6-methoxy-[2,4'-bipyridin]-2'-yl)-2,3-dihydro-1H-inden-1-yl)amino)-3-methoxy-6-(trifluoromethyl)pyrazin-2-yl)methyl)-2,6-diazaspiro[3.3]heptan-2-yl)ethan-1-one;
(R)-1-(6-((5-((4-(5-((6-acetyl-2,6-diazaspiro[3.3]heptan-2-yl)methyl)-3'-chloro-6-methoxy-[2,4'-bipyridin]-2'-yl)-2,3-dihydro-1H-inden-1-yl)amino)-3-methoxy-6-(trifluoromethyl)pyrazin-2-yl)methyl)-2,6-diazaspiro[3.3]heptan-2-yl)ethan-1-one;
(S)-2-(4-(3-chloro-4-(1-((6-methoxy-5-((7-oxo-2,6-diazaspiro[3.4]octan-2-yl)methyl)-3-(trifluoromethyl)pyridin-2-yl)oxy)-2,3-dihydro-1H-inden-4-yl)pyridin-2-yl)-2-fluoro-6-methoxybenzyl)-2,6-diazaspiro[3.4]octan-7-one;
(R)-2-(4-(3-chloro-4-(1-((6-methoxy-5-((7-oxo-2,6-diazaspiro[3.4]octan-2-yl)methyl)-3-(trifluoromethyl)pyridin-2-yl)oxy)-2,3-dihydro-1H-inden-4-yl)pyridin-2-yl)-2-fluoro-6-methoxybenzyl)-2,6-diazaspiro[3.4]octan-7-one;
(S)-5-((((6-(2-chloro-3-(3-chloro-2-(8-chloro-2-(oxetan-3-ylmethyl)-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(8-chloro-2-(oxetan-3-ylmethyl)-1,2,3,4-tetrahydroisoquinolin-6-yl)pyridin-4-yl)phenyl)-2-methoxypyridin-3-yl)methyl)amino)methyl)pyrrolidin-2-one;
(S)-5-((((6-(3-(2-(3-((6-acetyl-2,6-diazaspiro[3.3]heptan-2-yl)methyl)-1-methyl-1H-indazol-6-yl)-3-chloropyridin-4-yl)-2-chlorophenyl)-2-methoxypyridin-3-yl)methyl)amino)methyl)pyrrolidin-2-one;
(R)-5-((((6-(3-(2-(3-((6-acetyl-2,6-diazaspiro[3.3]heptan-2-yl)methyl)-1-methyl-1H-indazol-6-yl)-3-chloropyridin-4-yl)-2-chlorophenyl)-2-methoxypyridin-3-yl)methyl)amino)methyl)pyrrolidin-2-one;
(S)-1-((6-(3-chloro-4-(2-chloro-3-(6-methoxy-5-(((((S)-5-oxopyrrolidin-2-yl)methyl)amino)methyl)pyridin-2-yl)phenyl)pyridin-2-yl)-1-methyl-1H-indazol-3-yl)methyl)pyrrolidine-3-carboxylic acid;
(S)-1-((6-(3-chloro-4-(2-chloro-3-(6-methoxy-5-(((((R)-5-oxopyrrolidin-2-yl)methyl)amino)methyl)pyridin-2-yl)phenyl)pyridin-2-yl)-1-methyl-1H-indazol-3-yl)methyl)pyrrolidine-3-carboxylic acid;
(R)-1-((6-(3-chloro-4-(2-chloro-3-(6-methoxy-5-(((((S)-5-oxopyrrolidin-2-yl)methyl)amino)methyl)pyridin-2-yl)phenyl)pyridin-2-yl)-1-methyl-1H-indazol-3-yl)methyl)pyrrolidine-3-carboxylic acid;
(R)-1-((6-(3-chloro-4-(2-chloro-3-(6-methoxy-5-(((((R)-5-oxopyrrolidin-2-yl)methyl)amino)methyl)pyridin-2-yl)phenyl)pyridin-2-yl)-1-methyl-1H-indazol-3-yl)methyl)pyrrolidine-3-carboxylic acid;
(S)-5-((((6-(2-chloro-3-(3-chloro-2-(8-methoxy-2-(((((S)-5-oxopyrrolidin-2-yl)methyl)amino)methyl)imidazo[1,2-a]pyridin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(8-methoxy-2-(((((R)-5-oxopyrrolidin-2-yl)methyl)amino)methyl)imidazo[1,2-a]pyridin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(8-methoxy-2-(((((S)-5-oxopyrrolidin-2-yl)methyl)amino)methyl)imidazo[1,2-a]pyridin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(8-methoxy-2-(((((R)-5-oxopyrrolidin-2-yl)methyl)amino)methyl)imidazo[1,2-a]pyridin-6-yl)pyridin-4-yl)phenyl)-2-methoxypyridin-3-yl)methyl)amino)methyl)pyrrolidin-2-one;
1-(4-(((5-(3-(2-(4-(((1-acetylpiperidin-4-yl)amino)methyl)-3-methoxyphenyl)-3-chloropyridin-4-yl)-2-chlorophenyl)-3-methoxypyrazin-2-yl)methyl)amino)piperidin-1-yl)ethan-1-one;
(S)-5-((((6-(2-chloro-3-(3-chloro-2-(2-((((1r,3r)-3-hydroxy-3-methylcyclobutyl)amino)methyl)-8-methoxyimidazo[1,2-a]pyridin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(2-((((1s,3s)-3-hydroxy-3-methylcyclobutyl)amino)methyl)-8-methoxyimidazo[1,2-a]pyridin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(2-((((1s,3r)-3-hydroxy-3-methylcyclobutyl)amino)methyl)-8-methoxyimidazo[1,2-a]pyridin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(2-((((1r,3s)-3-hydroxy-3-methylcyclobutyl)amino)methyl)-8-methoxyimidazo[1,2-a]pyridin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-((((1r,3s)-3-hydroxy-3-methylcyclobutyl)amino)methyl)-8-methoxyimidazo[1,2-a]pyridin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-((((1s,3s)-3-hydroxy-3-methylcyclobutyl)amino)methyl)-8-methoxyimidazo[1,2-a]pyridin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-((((1s,3r)-3-hydroxy-3-methylcyclobutyl)amino)methyl)-8-methoxyimidazo[1,2-a]pyridin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-((((1r,3r)-3-hydroxy-3-methylcyclobutyl)amino)methyl)-8-methoxyimidazo[1,2-a]pyridin-6-yl)pyridin-4-yl)phenyl)-2-methoxypyridin-3-yl)methyl)amino)methyl)pyrrolidin-2-one;
1,1'-(((((3,3'-dichloro-[4,4'-bipyridine]-2,2'-diyl)bis(2-fluoro-6-methoxy-4,1-phenylene))bis(methylene))bis(azanediyl))bis(piperidine-4,1-diyl))bis(ethan-1-one);
(S)-2-((6-(3-chloro-4-(2-chloro-3-(6-methoxy-5-((((5-oxopyrrolidin-2-yl)methyl)amino)methyl)pyridin-2-yl)phenyl)pyridin-2-yl)-8-methoxyimidazo[1,2-a]pyridin-2-yl)methyl)-2,6-diazaspiro[3.4]octan-7-one;
(R)-2-((6-(3-chloro-4-(2-chloro-3-(6-methoxy-5-((((5-oxopyrrolidin-2-yl)methyl)amino)methyl)pyridin-2-yl)phenyl)pyridin-2-yl)-8-methoxyimidazo[1,2-a]pyridin-2-yl)methyl)-2,6-diazaspiro[3.4]octan-7-one;
1-(4-(((6-(3-(2-(4-(((1-acetylpiperidin-4-yl)(methyl)amino)methyl)-3-methoxyphenyl)-3-chloropyridin-4-yl)-2-chlorophenyl)-2-methoxypyridin-3-yl)methyl)amino)piperidin-1-yl)ethan-1-one;
(4-(((6-(2-chloro-3-(3-chloro-2-(4-(((1-(cyclopropanecarbonyl)piperidin-4-yl)amino)methyl)-3-fluoro-5-methoxyphenyl)pyridin-4-yl)phenyl)-2-methoxypyridin-3-yl)methyl)amino)piperidin-1-yl)(cyclopropyl)methanone;
(S)-5-((((6-(2-chloro-3-(3-chloro-2-(2-((3-hydroxy-3-methylazetidin-1-yl)methyl)-8-methoxyimidazo[1,2-a]pyridin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-((3-hydroxy-3-methylazetidin-1-yl)methyl)-8-methoxyimidazo[1,2-a]pyridin-6-yl)pyridin-4-yl)phenyl)-2-methoxypyridin-3-yl)methyl)amino)methyl)pyrrolidin-2-one;
(S)-5-((((6-(3-(2-(2-(((1-acetylpiperidin-4-yl)amino)methyl)-8-methoxyimidazo[1,2-a]pyridin-6-yl)-3-chloropyridin-4-yl)-2-chlorophenyl)-2-methoxypyridin-3-yl)methyl)amino)methyl)pyrrolidin-2-one;
(R)-5-((((6-(3-(2-(2-(((1-acetylpiperidin-4-yl)amino)methyl)-8-methoxyimidazo[1,2-a]pyridin-6-yl)-3-chloropyridin-4-yl)-2-chlorophenyl)-2-methoxypyridin-3-yl)methyl)amino)methyl)pyrrolidin-2-one;
(S)-5-((((6-(3-(2-(2-((6-acetyl-2,6-diazaspiro[3.3]heptan-2-yl)methyl)-8-methoxyimidazo[1,2-a]pyridin-6-yl)-3-chloropyridin-4-yl)-2-chlorophenyl)-2-methoxypyridin-3-yl)methyl)amino)methyl)pyrrolidin-2-one;
(R)-5-((((6-(3-(2-(2-((6-acetyl-2,6-diazaspiro[3.3]heptan-2-yl)methyl)-8-methoxyimidazo[1,2-a]pyridin-6-yl)-3-chloropyridin-4-yl)-2-chlorophenyl)-2-methoxypyridin-3-yl)methyl)amino)methyl)pyrrolidin-2-one;
(S)-2-((6-(3-chloro-4-(2-chloro-3-(6-methoxy-5-((((5-oxopyrrolidin-2-yl)methyl)amino)methyl)pyridin-2-yl)phenyl)pyridin-2-yl)-8-methoxyimidazo[1,2-a]pyridin-2-yl)methyl)-2,5-diazaspiro[3.4]octan-6-one;
(R)-2-((6-(3-chloro-4-(2-chloro-3-(6-methoxy-5-((((5-oxopyrrolidin-2-yl)methyl)amino)methyl)pyridin-2-yl)phenyl)pyridin-2-yl)-8-methoxyimidazo[1,2-a]pyridin-2-yl)methyl)-2,5-diazaspiro[3.4]octan-6-one;
(S)-5-((((6-(3-(2-(2-(((2-oxaspiro[3.3]heptan-6-yl)amino)methyl)-8-methoxyimidazo[1,2-a]pyridin-6-yl)-3-chloropyridin-4-yl)-2-chlorophenyl)-2-methoxypyridin-3-yl)methyl)amino)methyl)pyrrolidin-2-one;
(R)-5-((((6-(3-(2-(2-(((2-oxaspiro[3.3]heptan-6-yl)amino)methyl)-8-methoxyimidazo[1,2-a]pyridin-6-yl)-3-chloropyridin-4-yl)-2-chlorophenyl)-2-methoxypyridin-3-yl)methyl)amino)methyl)pyrrolidin-2-one;
N-(1-((6-(3-(2-(4-((4-acetamidopiperidin-1-yl)methyl)-3-methoxyphenyl)-3-chloropyridin-4-yl)-2-chlorophenyl)-2-methoxypyridin-3-yl)methyl)piperidin-4-yl)acetamide;
2,2'-(((3,3'-dichloro-[4,4'-bipyridine]-2,2'-diyl)bis(2-fluoro-6-methoxy-4,1-phenylene))bis(methylene))bis(2,6-diazaspiro[3.4]octan-7-one);
2-(4-(4-(2-chloro-3-(6-methoxy-5-((7-oxo-2,6-diazaspiro[3.4]octan-2-yl)methyl)pyridin-2-yl)phenyl)-3-fluoropyridin-2-yl)-2-methoxybenzyl)-2,6-diazaspiro[3.4]octan-7-one;
N-(1-(4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)piperidin-4-yl)acetamide;
N-((6-(2-chloro-3-(3-chloro-2-(4-(((1-isopropylpiperidin-4-yl)amino)methyl)-3-methoxyphenyl)pyridin-4-yl)phenyl)-2-methoxypyridin-3-yl)methyl)-1-isopropylpiperidin-4-amine;
N-((6-(2-chloro-3-(3-chloro-2-(4-(((4,4-difluorocyclohexyl)amino)methyl)-3-methoxyphenyl)pyridin-4-yl)phenyl)-2-methoxypyridin-3-yl)methyl)-4,4-difluorocyclohexan-1-amine;
2-(4-(((6-(2-chloro-3-(3-chloro-2-(4-(((1-(2-hydroxyethyl)piperidin-4-yl)amino)methyl)-3-methoxyphenyl)pyridin-4-yl)phenyl)-2-methoxypyridin-3-yl)methyl)amino)piperidin-1-yl)ethan-1-ol;
N-(2-(((6-(3-(2-(4-(((2-acetamidoethyl)amino)methyl)-3-methoxyphenyl)-3-chloropyridin-4-yl)-2-chlorophenyl)-2-methoxypyridin-3-yl)methyl)amino)ethyl)acetamide;
1-(6-(2-chloro-3-(3-chloro-2-(3-methoxy-4-((((tetrahydro-2H-pyran-4-yl)methyl)amino)methyl)phenyl)pyridin-4-yl)phenyl)-2-methoxypyridin-3-yl)-N-((tetrahydro-2H-pyran-4-yl)methyl)methanamine;
1-(4-(((6-(2-chloro-3-(3-chloro-2-(3-fluoro-5-methoxy-4-(((1-propionylpiperidin-4-yl)amino)methyl)phenyl)pyridin-4-yl)phenyl)-2-methoxypyridin-3-yl)methyl)amino)piperidin-1-yl)propan-1-one;
2-((6-(3-(2-(4-(((1-acetylpiperidin-4-yl)amino)methyl)-3-methoxyphenyl)-3-chloropyridin-4-yl)-2-chlorophenyl)-2-methoxypyridin-3-yl)methyl)-2,6-diazaspiro[3.4]octan-7-one;
1-(4-((4-(4-(3-(5-(((1-acetylpiperidin-4-yl)(methyl)amino)methyl)-6-methoxypyridin-2-yl)-2-chlorophenyl)-3-chloropyridin-2-yl)-2-methoxybenzyl)amino)piperidin-1-yl)ethan-1-one;
1-(6-(2-chloro-3-(2-(3-methoxy-4-((methylamino)methyl)phenyl)-3-methylpyridin-4-yl)phenyl)-2-methoxypyridin-3-yl)-N-methylmethanamine;
(S)-5-((((6-(3-(2-(4-((S)-1-aminoethyl)-3-methoxyphenyl)-3-chloropyridin-4-yl)-2-chlorophenyl)-2-methoxypyridin-3-yl)methyl)amino)methyl)pyrrolidin-2-one;
(R)-5-((((6-(3-(2-(4-((S)-1-aminoethyl)-3-methoxyphenyl)-3-chloropyridin-4-yl)-2-chlorophenyl)-2-methoxypyridin-3-yl)methyl)amino)methyl)pyrrolidin-2-one;
(S)-5-((((6-(3-(2-(4-((R)-1-aminoethyl)-3-methoxyphenyl)-3-chloropyridin-4-yl)-2-chlorophenyl)-2-methoxypyridin-3-yl)methyl)amino)methyl)pyrrolidin-2-one;
(R)-5-((((6-(3-(2-(4-((R)-1-aminoethyl)-3-methoxyphenyl)-3-chloropyridin-4-yl)-2-chlorophenyl)-2-methoxypyridin-3-yl)methyl)amino)methyl)pyrrolidin-2-one;
(S)-2-((3'-chloro-6-methoxy-2'-(1-((6-methoxy-5-((7-oxo-2,6-diazaspiro[3.4]octan-2-yl)methyl)-3-(trifluoromethyl)pyridin-2-yl)oxy)-2,3-dihydro-1H-inden-4-yl)-[2,4'-bipyridin]-5-yl)methyl)-2,6-diazaspiro[3.4]octan-7-one;
(R)-2-((3'-chloro-6-methoxy-2'-(1-((6-methoxy-5-((7-oxo-2,6-diazaspiro[3.4]octan-2-yl)methyl)-3-(trifluoromethyl)pyridin-2-yl)oxy)-2,3-dihydro-1H-inden-4-yl)-[2,4'-bipyridin]-5-yl)methyl)-2,6-diazaspiro[3.4]octan-7-one;
1-(4-(((6-(3-(2-(4-(((1-acetylpiperidin-4-yl)amino)methyl)-3-methoxyphenyl)-3-fluoropyridin-4-yl)-2-chlorophenyl)-2-methoxypyridin-3-yl)methyl)amino)piperidin-1-yl)ethan-1-one;
(S)-4-((4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)amino)dihydrofuran-2(3H)-one;
(R)-4-((4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)amino)dihydrofuran-2(3H)-one;
methyl (S)-3-((4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)amino)-4-hydroxybutanoate;
methyl (R)-3-((4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)amino)-4-hydroxybutanoate;
(S)-3-((4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)amino)dihydrofuran-2(3H)-one;
(R)-3-((4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)amino)dihydrofuran-2(3H)-one;
N-(1-((6-(3-(2-(4-(((1-acetylpiperidin-4-yl)amino)methyl)-3-methoxyphenyl)-3-chloropyridin-4-yl)-2-chlorophenyl)-2-methoxypyridin-3-yl)methyl)piperidin-4-yl)acetamide;
(S)-3-((4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)amino)-4-hydroxybutanoic acid;
(R)-3-((4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)amino)-4-hydroxybutanoic acid;
(4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)-L-homoserine;
(4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)-D-homoserine;
1-(4-(((6-(2-chloro-3-(3-chloro-2-(3-methoxy-4-(((1-propionylpiperidin-4-yl)amino)methyl)phenyl)pyridin-4-yl)phenyl)-2-methoxypyridin-3-yl)methyl)amino)piperidin-1-yl)propan-1-one;
(4-(((6-(2-chloro-3-(3-chloro-2-(4-(((1-(cyclopropanecarbonyl)piperidin-4-yl)amino)methyl)-3-methoxyphenyl)pyridin-4-yl)phenyl)-2-methoxypyridin-3-yl)methyl)amino)piperidin-1-yl)(cyclopropyl)methanone;
1-(4-(((6-(2-chloro-3-(3-chloro-2-(4-(((1-isobutyrylpiperidin-4-yl)amino)methyl)-3-methoxyphenyl)pyridin-4-yl)phenyl)-2-methoxypyridin-3-yl)methyl)amino)piperidin-1-yl)-2-methylpropan-1-one;
1-(4-(((6-(2-chloro-3-(3-chloro-2-(3-fluoro-4-(((1-isobutyrylpiperidin-4-yl)amino)methyl)-5-methoxyphenyl)pyridin-4-yl)phenyl)-2-methoxypyridin-3-yl)methyl)amino)piperidin-1-yl)-2-methylpropan-1-one;
N-((6-(2-chloro-3-(3-chloro-2-(3-fluoro-5-methoxy-4-(((tetrahydro-2H-pyran-4-yl)amino)methyl)phenyl)pyridin-4-yl)phenyl)-2-methoxypyridin-3-yl)methyl)tetrahydro-2H-pyran-4-amine;
4-(((6-(2-chloro-3-(3-chloro-2-(4-(((1,1-dioxidotetrahydro-2H-thiopyran-4-yl)amino)methyl)-3-fluoro-5-methoxyphenyl)pyridin-4-yl)phenyl)-2-methoxypyridin-3-yl)methyl)amino)tetrahydro-2H-thiopyran 1,1-dioxide;
(S)-1-((6-(3-chloro-4-(2-chloro-3-(6-methoxy-5-((((5-oxopyrrolidin-2-yl)methyl)amino)methyl)pyridin-2-yl)phenyl)pyridin-2-yl)-8-methoxyimidazo[1,2-a]pyridin-2-yl)methyl)azetidine-3-carboxylic acid;
(R)-1-((6-(3-chloro-4-(2-chloro-3-(6-methoxy-5-((((5-oxopyrrolidin-2-yl)methyl)amino)methyl)pyridin-2-yl)phenyl)pyridin-2-yl)-8-methoxyimidazo[1,2-a]pyridin-2-yl)methyl)azetidine-3-carboxylic acid;
(S)-1-(2-(((6-(3-chloro-4-(2-chloro-3-(6-methoxy-5-((((5-oxopyrrolidin-2-yl)methyl)amino)methyl)pyridin-2-yl)phenyl)pyridin-2-yl)-8-methoxyimidazo[1,2-a]pyridin-2-yl)methyl)amino)ethyl)cyclopropane-1-carboxylic acid;
(R)-1-(2-(((6-(3-chloro-4-(2-chloro-3-(6-methoxy-5-((((5-oxopyrrolidin-2-yl)methyl)amino)methyl)pyridin-2-yl)phenyl)pyridin-2-yl)-8-methoxyimidazo[1,2-a]pyridin-2-yl)methyl)amino)ethyl)cyclopropane-1-carboxylic acid;
(4-(((6-(2-chloro-3-(3-chloro-2-(4-(((1-(cyclobutanecarbonyl)piperidin-4-yl)amino)methyl)-3-methoxyphenyl)pyridin-4-yl)phenyl)-2-methoxypyridin-3-yl)methyl)amino)piperidin-1-yl)(cyclobutyl)methanone;
7-(4-(3-chloro-4-(2-chloro-3-(6-methoxy-5-((2-oxo-1,7-diazaspiro[3.5]nonan-7-yl)methyl)pyridin-2-yl)phenyl)pyridin-2-yl)-2-methoxybenzyl)-1,7-diazaspiro[3.5]nonan-2-one;
(R)-5-((((6-(2-chloro-3-(3-chloro-2-(3-methoxy-4-(((((R)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
(R)-5-((((6-(2-chloro-3-(3-chloro-2-(3-methoxy-4-(((((S)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
(S)-5-((((6-(2-chloro-3-(3-chloro-2-(3-methoxy-4-(((((R)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
(S)-5-((((6-(2-chloro-3-(3-chloro-2-(3-methoxy-4-(((((S)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
N-(1-((6-(2-chloro-3-(3-chloro-2-(3-methoxy-4-((7-oxo-2,6-diazaspiro[3.4]octan-2-yl)methyl)phenyl)pyridin-4-yl)phenyl)-2-methoxypyridin-3-yl)methyl)piperidin-4-yl)acetamide;
(R)-N-(1-((6-(2-chloro-3-(3-chloro-2-(3-methoxy-4-((((5-oxopyrrolidin-2-yl)methyl)amino)methyl)phenyl)pyridin-4-yl)phenyl)-2-methoxypyridin-3-yl)methyl)piperidin-4-yl)acetamide;
(S)-N-(1-((6-(2-chloro-3-(3-chloro-2-(3-methoxy-4-((((5-oxopyrrolidin-2-yl)methyl)amino)methyl)phenyl)pyridin-4-yl)phenyl)-2-methoxypyridin-3-yl)methyl)piperidin-4-yl)acetamide;
(S)-N-(1-((6-(2-chloro-3-(3-chloro-2-(3-methoxy-4-((((5-oxopyrrolidin-2-yl)methyl)amino)methyl)phenyl)pyridin-4-yl)phenyl)-2-methoxypyridin-3-yl)methyl)piperidin-4-yl)acetamide;
(R)-N-(1-((6-(2-chloro-3-(3-chloro-2-(3-methoxy-4-((((5-oxopyrrolidin-2-yl)methyl)amino)methyl)phenyl)pyridin-4-yl)phenyl)-2-methoxypyridin-3-yl)methyl)piperidin-4-yl)acetamide;
N-(3-(((6-(3-(2-(4-(((3-acetamidopropyl)amino)methyl)-3-methoxyphenyl)-3-chloropyridin-4-yl)-2-chlorophenyl)-2-methoxypyridin-3-yl)methyl)amino)propyl)acetamide;
4-(((6-(2-chloro-3-(3-chloro-2-(4-(((2,6-dioxopiperidin-4-yl)amino)methyl)-3-methoxyphenyl)pyridin-4-yl)phenyl)-2-methoxypyridin-3-yl)methyl)amino)piperidine-2,6-dione;
1-(4-(((6-(2-chloro-3-(3-chloro-2-(4-((((1r,4r)-4-hydroxycyclohexyl)amino)methyl)-3-methoxyphenyl)pyridin-4-yl)phenyl)-2-methoxypyridin-3-yl)methyl)amino)piperidin-1-yl)ethan-1-one;
1-(4-(((6-(2-chloro-3-(3-chloro-2-(4-((((1r,4s)-4-hydroxycyclohexyl)amino)methyl)-3-methoxyphenyl)pyridin-4-yl)phenyl)-2-methoxypyridin-3-yl)methyl)amino)piperidin-1-yl)ethan-1-one;
1-(4-(((6-(2-chloro-3-(3-chloro-2-(4-((((1s,4r)-4-hydroxycyclohexyl)amino)methyl)-3-methoxyphenyl)pyridin-4-yl)phenyl)-2-methoxypyridin-3-yl)methyl)amino)piperidin-1-yl)ethan-1-one;
1-(4-(((6-(2-chloro-3-(3-chloro-2-(4-((((1s,4s)-4-hydroxycyclohexyl)amino)methyl)-3-methoxyphenyl)pyridin-4-yl)phenyl)-2-methoxypyridin-3-yl)methyl)amino)piperidin-1-yl)ethan-1-one;
1-(4-(((6-(2-chloro-3-(3-chloro-2-(4-((6-hydroxy-2-azaspiro[3.3]heptan-2-yl)methyl)-3-methoxyphenyl)pyridin-4-yl)phenyl)-2-methoxypyridin-3-yl)methyl)amino)piperidin-1-yl)ethan-1-one;
1-(2-methoxy-4-(4-(3-(6-methoxy-5-((methylamino)methyl)pyridin-2-yl)-2-methylphenyl)-3-methylpyridin-2-yl)phenyl)-N-methylmethanamine;
1-(4-(((6-(3-(2-(4-(((1-acetylpiperidin-4-yl)amino)methyl)-3-methoxyphenyl)-3-methylpyridin-4-yl)-2-methylphenyl)-2-methoxypyridin-3-yl)methyl)amino)piperidin-1-yl)ethan-1-one;
(1r,4r)-4-(((6-(2-chloro-3-(3-chloro-2-(4-((((1r,4r)-4-hydroxycyclohexyl)amino)methyl)-3-methoxyphenyl)pyridin-4-yl)phenyl)-2-methoxypyridin-3-yl)methyl)amino)cyclohexan-1-ol;
(1r,4r)-4-(((6-(2-chloro-3-(3-chloro-2-(4-((((1r,4s)-4-hydroxycyclohexyl)amino)methyl)-3-methoxyphenyl)pyridin-4-yl)phenyl)-2-methoxypyridin-3-yl)methyl)amino)cyclohexan-1-ol;
(1r,4r)-4-(((6-(2-chloro-3-(3-chloro-2-(4-((((1s,4r)-4-hydroxycyclohexyl)amino)methyl)-3-methoxyphenyl)pyridin-4-yl)phenyl)-2-methoxypyridin-3-yl)methyl)amino)cyclohexan-1-ol;
(1r,4r)-4-(((6-(2-chloro-3-(3-chloro-2-(4-((((1s,4s)-4-hydroxycyclohexyl)amino)methyl)-3-methoxyphenyl)pyridin-4-yl)phenyl)-2-methoxypyridin-3-yl)methyl)amino)cyclohexan-1-ol;
(1r,4s)-4-(((6-(2-chloro-3-(3-chloro-2-(4-((((1r,4r)-4-hydroxycyclohexyl)amino)methyl)-3-methoxyphenyl)pyridin-4-yl)phenyl)-2-methoxypyridin-3-yl)methyl)amino)cyclohexan-1-ol;
(1r,4s)-4-(((6-(2-chloro-3-(3-chloro-2-(4-((((1r,4s)-4-hydroxycyclohexyl)amino)methyl)-3-methoxyphenyl)pyridin-4-yl)phenyl)-2-methoxypyridin-3-yl)methyl)amino)cyclohexan-1-ol;
(1r,4s)-4-(((6-(2-chloro-3-(3-chloro-2-(4-((((1s,4r)-4-hydroxycyclohexyl)amino)methyl)-3-methoxyphenyl)pyridin-4-yl)phenyl)-2-methoxypyridin-3-yl)methyl)amino)cyclohexan-1-ol;
(1r,4s)-4-(((6-(2-chloro-3-(3-chloro-2-(4-((((1s,4s)-4-hydroxycyclohexyl)amino)methyl)-3-methoxyphenyl)pyridin-4-yl)phenyl)-2-methoxypyridin-3-yl)methyl)amino)cyclohexan-1-ol;
(1s,4r)-4-(((6-(2-chloro-3-(3-chloro-2-(4-((((1r,4r)-4-hydroxycyclohexyl)amino)methyl)-3-methoxyphenyl)pyridin-4-yl)phenyl)-2-methoxypyridin-3-yl)methyl)amino)cyclohexan-1-ol;
(1s,4r)-4-(((6-(2-chloro-3-(3-chloro-2-(4-((((1r,4s)-4-hydroxycyclohexyl)amino)methyl)-3-methoxyphenyl)pyridin-4-yl)phenyl)-2-methoxypyridin-3-yl)methyl)amino)cyclohexan-1-ol;
(1s,4r)-4-(((6-(2-chloro-3-(3-chloro-2-(4-((((1s,4r)-4-hydroxycyclohexyl)amino)methyl)-3-methoxyphenyl)pyridin-4-yl)phenyl)-2-methoxypyridin-3-yl)methyl)amino)cyclohexan-1-ol;
(1s,4r)-4-(((6-(2-chloro-3-(3-chloro-2-(4-((((1s,4s)-4-hydroxycyclohexyl)amino)methyl)-3-methoxyphenyl)pyridin-4-yl)phenyl)-2-methoxypyridin-3-yl)methyl)amino)cyclohexan-1-ol;
(1s,4s)-4-(((6-(2-chloro-3-(3-chloro-2-(4-((((1r,4r)-4-hydroxycyclohexyl)amino)methyl)-3-methoxyphenyl)pyridin-4-yl)phenyl)-2-methoxypyridin-3-yl)methyl)amino)cyclohexan-1-ol;
(1s,4s)-4-(((6-(2-chloro-3-(3-chloro-2-(4-((((1r,4s)-4-hydroxycyclohexyl)amino)methyl)-3-methoxyphenyl)pyridin-4-yl)phenyl)-2-methoxypyridin-3-yl)methyl)amino)cyclohexan-1-ol;
(1s,4s)-4-(((6-(2-chloro-3-(3-chloro-2-(4-((((1s,4r)-4-hydroxycyclohexyl)amino)methyl)-3-methoxyphenyl)pyridin-4-yl)phenyl)-2-methoxypyridin-3-yl)methyl)amino)cyclohexan-1-ol;
(1s,4s)-4-(((6-(2-chloro-3-(3-chloro-2-(4-((((1s,4s)-4-hydroxycyclohexyl)amino)methyl)-3-methoxyphenyl)pyridin-4-yl)phenyl)-2-methoxypyridin-3-yl)methyl)amino)cyclohexan-1-ol;
N-(4-(4-(3-(5-(((2-oxaspiro[3.3]heptan-6-yl)amino)methyl)-6-methoxypyridin-2-yl)-2-chlorophenyl)-3-chloropyridin-2-yl)-2-methoxybenzyl)-2-oxaspiro[3.3]heptan-6-amine;
(S)-2-(4-(3-chloro-4-(1-((6-methoxy-5-((7-oxo-2,6-diazaspiro[3.4]octan-2-yl)methyl)-3-(trifluoromethyl)pyrazin-2-yl)amino)-2,3-dihydro-1H-inden-4-yl)pyridin-2-yl)-2-methoxybenzyl)-2,6-diazaspiro[3.4]octan-7-one;
(R)-2-(4-(3-chloro-4-(1-((6-methoxy-5-((7-oxo-2,6-diazaspiro[3.4]octan-2-yl)methyl)-3-(trifluoromethyl)pyrazin-2-yl)amino)-2,3-dihydro-1H-inden-4-yl)pyridin-2-yl)-2-methoxybenzyl)-2,6-diazaspiro[3.4]octan-7-one;
(S)-1-(6-((5-((4-(2-(4-((6-acetyl-2,6-diazaspiro[3.3]heptan-2-yl)methyl)-3-methoxyphenyl)-3-chloropyridin-4-yl)-2,3-dihydro-1H-inden-1-yl)amino)-3-methoxy-6-(trifluoromethyl)pyrazin-2-yl)methyl)-2,6-diazaspiro[3.3]heptan-2-yl)ethan-1-one;
(R)-1-(6-((5-((4-(2-(4-((6-acetyl-2,6-diazaspiro[3.3]heptan-2-yl)methyl)-3-methoxyphenyl)-3-chloropyridin-4-yl)-2,3-dihydro-1H-inden-1-yl)amino)-3-methoxy-6-(trifluoromethyl)pyrazin-2-yl)methyl)-2,6-diazaspiro[3.3]heptan-2-yl)ethan-1-one;
2-(4-(4-(2-chloro-3-(6-methoxy-5-((7-oxo-2,6-diazaspiro[3.4]octan-2-yl)methyl)pyridin-2-yl)phenyl)-3-methylpyridin-2-yl)-2-methoxybenzyl)-2,6-diazaspiro[3.4]octan-7-one;
1-(6-((6-(3-(2-(4-((6-acetyl-2,6-diazaspiro[3.3]heptan-2-yl)methyl)-3-methoxyphenyl)-3-methylpyridin-4-yl)-2-chlorophenyl)-2-methoxypyridin-3-yl)methyl)-2,6-diazaspiro[3.3]heptan-2-yl)ethan-1-one;
(S)-5-((((6-(2-chloro-3-(3-chloro-2-(2-((((1r,3r)-3-hydroxy-3-methylcyclobutyl)amino)methyl)-8-methoxy-[1,2,4]triazolo[1,5-a]pyridin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-((((1r,3r)-3-hydroxy-3-methylcyclobutyl)amino)methyl)-8-methoxy-[1,2,4]triazolo[1,5-a]pyridin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-((((1r,3s)-3-hydroxy-3-methylcyclobutyl)amino)methyl)-8-methoxy-[1,2,4]triazolo[1,5-a]pyridin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-((((1s,3r)-3-hydroxy-3-methylcyclobutyl)amino)methyl)-8-methoxy-[1,2,4]triazolo[1,5-a]pyridin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-((((1s,3s)-3-hydroxy-3-methylcyclobutyl)amino)methyl)-8-methoxy-[1,2,4]triazolo[1,5-a]pyridin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(2-((((1r,3s)-3-hydroxy-3-methylcyclobutyl)amino)methyl)-8-methoxy-[1,2,4]triazolo[1,5-a]pyridin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(2-((((1s,3r)-3-hydroxy-3-methylcyclobutyl)amino)methyl)-8-methoxy-[1,2,4]triazolo[1,5-a]pyridin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(2-((((1s,3s)-3-hydroxy-3-methylcyclobutyl)amino)methyl)-8-methoxy-[1,2,4]triazolo[1,5-a]pyridin-6-yl)pyridin-4-yl)phenyl)-2-methoxypyridin-3-yl)methyl)amino)methyl)pyrrolidin-2-one;
1-(4-((4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)amino)piperidin-1-yl)-2-methylpropan-1-one;
N-((6-(2-chloro-3-(3-chloro-2-(3-methoxy-4-(((1-(methylsulfonyl)piperidin-4-yl)amino)methyl)phenyl)pyridin-4-yl)phenyl)-2-methoxypyridin-3-yl)methyl)-1-(methylsulfonyl)piperidin-4-amine;
5-(((6-(3-(2-(4-(((5-amino-5-oxopentyl)amino)methyl)-3-methoxyphenyl)-3-chloropyridin-4-yl)-2-chlorophenyl)-2-methoxypyridin-3-yl)methyl)amino)pentanamide;
1-((R)-3-(((6-(3-(2-(4-((((R)-1-acetylpyrrolidin-3-yl)amino)methyl)-3-methoxyphenyl)-3-chloropyridin-4-yl)-2-chlorophenyl)-2-methoxypyridin-3-yl)methyl)amino)pyrrolidin-1-yl)ethan-1-one;
1-((S)-3-(((6-(3-(2-(4-((((R)-1-acetylpyrrolidin-3-yl)amino)methyl)-3-methoxyphenyl)-3-chloropyridin-4-yl)-2-chlorophenyl)-2-methoxypyridin-3-yl)methyl)amino)pyrrolidin-1-yl)ethan-1-one;
1-((R)-3-(((6-(3-(2-(4-((((S)-1-acetylpyrrolidin-3-yl)amino)methyl)-3-methoxyphenyl)-3-chloropyridin-4-yl)-2-chlorophenyl)-2-methoxypyridin-3-yl)methyl)amino)pyrrolidin-1-yl)ethan-1-one;
1-((S)-3-(((6-(3-(2-(4-((((S)-1-acetylpyrrolidin-3-yl)amino)methyl)-3-methoxyphenyl)-3-chloropyridin-4-yl)-2-chlorophenyl)-2-methoxypyridin-3-yl)methyl)amino)pyrrolidin-1-yl)ethan-1-one;
1-(4-(((6-(3-(6'-(((1-acetylpiperidin-4-yl)amino)methyl)-3-chloro-5'-methoxy-[2,3'-bipyridin]-4-yl)-2-chlorophenyl)-2-methoxypyridin-3-yl)methyl)amino)piperidin-1-yl)ethan-1-one;
2-((3-chloro-4-(2-chloro-3-(6-methoxy-5-((7-oxo-2,6-diazaspiro[3.4]octan-2-yl)methyl)pyridin-2-yl)phenyl)-5'-methoxy-[2,3'-bipyridin]-6'-yl)methyl)-2,6-diazaspiro[3.4]octan-7-one;
(S)-N-(1-((6-(3-chloro-4-(2-chloro-3-(6-methoxy-5-((((5-oxopyrrolidin-2-yl)methyl)amino)methyl)pyridin-2-yl)phenyl)pyridin-2-yl)-1-methyl-1H-pyrrolo[3,2-b]pyridin-3-yl)methyl)piperidin-4-yl)acetamide;
(R)-N-(1-((6-(3-chloro-4-(2-chloro-3-(6-methoxy-5-((((5-oxopyrrolidin-2-yl)methyl)amino)methyl)pyridin-2-yl)phenyl)pyridin-2-yl)-1-methyl-1H-pyrrolo[3,2-b]pyridin-3-yl)methyl)piperidin-4-yl)acetamide;
(S)-2-((6-(3-chloro-4-(2-chloro-3-(6-methoxy-5-((((5-oxopyrrolidin-2-yl)methyl)amino)methyl)pyridin-2-yl)phenyl)pyridin-2-yl)-1-methyl-1H-pyrrolo[3,2-b]pyridin-3-yl)methyl)-2,6-diazaspiro[3.4]octan-7-one;
(R)-2-((6-(3-chloro-4-(2-chloro-3-(6-methoxy-5-((((5-oxopyrrolidin-2-yl)methyl)amino)methyl)pyridin-2-yl)phenyl)pyridin-2-yl)-1-methyl-1H-pyrrolo[3,2-b]pyridin-3-yl)methyl)-2,6-diazaspiro[3.4]octan-7-one;
1-(4-(((6-(3-(2-(4-(((2-oxaspiro[3.3]heptan-6-yl)amino)methyl)-3-methoxyphenyl)-3-chloropyridin-4-yl)-2-chlorophenyl)-2-methoxypyridin-3-yl)methyl)amino)piperidin-1-yl)ethan-1-one;
1-(4-(((6-(3-(2-(4-((2-oxa-6-azaspiro[3.3]heptan-6-yl)methyl)-3-methoxyphenyl)-3-chloropyridin-4-yl)-2-chlorophenyl)-2-methoxypyridin-3-yl)methyl)amino)piperidin-1-yl)ethan-1-one;
1-(4-(((6-(2-chloro-3-(3-chloro-2-(4-(((3,3-dimethylcyclobutyl)amino)methyl)-3-methoxyphenyl)pyridin-4-yl)phenyl)-2-methoxypyridin-3-yl)methyl)amino)piperidin-1-yl)ethan-1-one;
1-(4-(((6-(3-(2-(4-(((1-acetylpiperidin-4-yl)amino)methyl)-3-methoxyphenyl)-3-chloropyridin-4-yl)-2-chlorophenyl)-2-methoxypyridin-3-yl)methyl)amino)piperidin-1-yl)-2-methylpropan-1-one;
1-(4-(((6-(2-chloro-3-(3-chloro-2-(3-methoxy-4-(((1-(methylsulfonyl)piperidin-4-yl)amino)methyl)phenyl)pyridin-4-yl)phenyl)-2-methoxypyridin-3-yl)methyl)amino)piperidin-1-yl)ethan-1-one;
(S)-5-((((6-(2-chloro-3-(3-chloro-2-(2-((((1r,4r)-4-hydroxycyclohexyl)amino)methyl)-8-methoxyimidazo[1,2-a]pyridin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-((((Ir,4r)-4-hydroxycyclohexyl)amino)methyl)-8-methoxyimidazo[1,2-a]pyridin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-((((1r,4s)-4-hydroxycyclohexyl)amino)methyl)-8-methoxyimidazo[1,2-a]pyridin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-((((1s,4r)-4-hydroxycyclohexyl)amino)methyl)-8-methoxyimidazo[1,2-a]pyridin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-((((1s,4s)-4-hydroxycyclohexyl)amino)methyl)-8-methoxyimidazo[1,2-a]pyridin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(2-((((1r,4s)-4-hydroxycyclohexyl)amino)methyl)-8-methoxyimidazo[1,2-a]pyridin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(2-((((1s,4r)-4-hydroxycyclohexyl)amino)methyl)-8-methoxyimidazo[1,2-a]pyridin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(2-((((1s,4s)-4-hydroxycyclohexyl)amino)methyl)-8-methoxyimidazo[1,2-a]pyridin-6-yl)pyridin-4-yl)phenyl)-2-methoxypyridin-3-yl)methyl)amino)methyl)pyrrolidin-2-one;
(1r,3r)-3-(((6-(2-chloro-3-(3-chloro-2-(4-((((1r,3r)-3-hydroxy-3-methylcyclobutyl)amino)methyl)-3-methoxyphenyl)pyridin-4-yl)phenyl)-2-methoxypyridin-3-yl)methyl)amino)-1-methylcyclobutan-1-ol;
(1r,3r)-3-(((6-(2-chloro-3-(3-chloro-2-(4-((((1r,3s)-3-hydroxy-3-methylcyclobutyl)amino)methyl)-3-methoxyphenyl)pyridin-4-yl)phenyl)-2-methoxypyridin-3-yl)methyl)amino)-1-methylcyclobutan-1-ol;
(1r,3r)-3-(((6-(2-chloro-3-(3-chloro-2-(4-((((1s,3r)-3-hydroxy-3-methylcyclobutyl)amino)methyl)-3-methoxyphenyl)pyridin-4-yl)phenyl)-2-methoxypyridin-3-yl)methyl)amino)-1-methylcyclobutan-1-ol;
(1r,3r)-3-(((6-(2-chloro-3-(3-chloro-2-(4-((((1s,3s)-3-hydroxy-3-methylcyclobutyl)amino)methyl)-3-methoxyphenyl)pyridin-4-yl)phenyl)-2-methoxypyridin-3-yl)methyl)amino)-1-methylcyclobutan-1-ol;
(1r,3s)-3-(((6-(2-chloro-3-(3-chloro-2-(4-((((1r,3r)-3-hydroxy-3-methylcyclobutyl)amino)methyl)-3-methoxyphenyl)pyridin-4-yl)phenyl)-2-methoxypyridin-3-yl)methyl)amino)-1-methylcyclobutan-1-ol;
(1r,3s)-3-(((6-(2-chloro-3-(3-chloro-2-(4-((((1r,3s)-3-hydroxy-3-methylcyclobutyl)amino)methyl)-3-methoxyphenyl)pyridin-4-yl)phenyl)-2-methoxypyridin-3-yl)methyl)amino)-1-methylcyclobutan-1-ol;
(1r,3s)-3-(((6-(2-chloro-3-(3-chloro-2-(4-((((1s,3r)-3-hydroxy-3-methylcyclobutyl)amino)methyl)-3-methoxyphenyl)pyridin-4-yl)phenyl)-2-methoxypyridin-3-yl)methyl)amino)-1-methylcyclobutan-1-ol;
(1r,3s)-3-(((6-(2-chloro-3-(3-chloro-2-(4-((((1s,3s)-3-hydroxy-3-methylcyclobutyl)amino)methyl)-3-methoxyphenyl)pyridin-4-yl)phenyl)-2-methoxypyridin-3-yl)methyl)amino)-1-methylcyclobutan-1-ol;
(1s,3r)-3-(((6-(2-chloro-3-(3-chloro-2-(4-((((1r,3r)-3-hydroxy-3-methylcyclobutyl)amino)methyl)-3-methoxyphenyl)pyridin-4-yl)phenyl)-2-methoxypyridin-3-yl)methyl)amino)-1-methylcyclobutan-1-ol;
(1s,3r)-3-(((6-(2-chloro-3-(3-chloro-2-(4-((((1r,3s)-3-hydroxy-3-methylcyclobutyl)amino)methyl)-3-methoxyphenyl)pyridin-4-yl)phenyl)-2-methoxypyridin-3-yl)methyl)amino)-1-methylcyclobutan-1-ol;
(1s,3r)-3-(((6-(2-chloro-3-(3-chloro-2-(4-((((1s,3r)-3-hydroxy-3-methylcyclobutyl)amino)methyl)-3-methoxyphenyl)pyridin-4-yl)phenyl)-2-methoxypyridin-3-yl)methyl)amino)-1-methylcyclobutan-1-ol;
(1s,3r)-3-(((6-(2-chloro-3-(3-chloro-2-(4-((((1s,3s)-3-hydroxy-3-methylcyclobutyl)amino)methyl)-3-methoxyphenyl)pyridin-4-yl)phenyl)-2-methoxypyridin-3-yl)methyl)amino)-1-methylcyclobutan-1-ol;
(1s,3s)-3-(((6-(2-chloro-3-(3-chloro-2-(4-((((1r,3r)-3-hydroxy-3-methylcyclobutyl)amino)methyl)-3-methoxyphenyl)pyridin-4-yl)phenyl)-2-methoxypyridin-3-yl)methyl)amino)-1-methylcyclobutan-1-ol;
(1s,3s)-3-(((6-(2-chloro-3-(3-chloro-2-(4-((((1r,3s)-3-hydroxy-3-methylcyclobutyl)amino)methyl)-3-methoxyphenyl)pyridin-4-yl)phenyl)-2-methoxypyridin-3-yl)methyl)amino)-1-methylcyclobutan-1-ol;
(1s,3s)-3-(((6-(2-chloro-3-(3-chloro-2-(4-((((1s,3r)-3-hydroxy-3-methylcyclobutyl)amino)methyl)-3-methoxyphenyl)pyridin-4-yl)phenyl)-2-methoxypyridin-3-yl)methyl)amino)-1-methylcyclobutan-1-ol;
(1s,3s)-3-(((6-(2-chloro-3-(3-chloro-2-(4-((((1s,3s)-3-hydroxy-3-methylcyclobutyl)amino)methyl)-3-methoxyphenyl)pyridin-4-yl)phenyl)-2-methoxypyridin-3-yl)methyl)amino)-1-methylcyclobutan-1-ol;
2-(4-(3-chloro-4-(2-chloro-3-(5-((6-hydroxy-2-azaspiro[3.3]heptan-2-yl)methyl)-6-methoxypyridin-2-yl)phenyl)pyridin-2-yl)-2-methoxybenzyl)-2-azaspiro[3.3]heptan-6-ol;
6-(4-(4-(3-(5-((2-oxa-6-azaspiro[3.3]heptan-6-yl)methyl)-6-methoxypyridin-2-yl)-2-chlorophenyl)-3-chloropyridin-2-yl)-2-methoxybenzyl)-2-oxa-6-azaspiro[3.3]heptane;
(S)-1-((6-(3-(2-(4-(((1-acetylpiperidin-4-yl)amino)methyl)-3-methoxyphenyl)-3-chloropyridin-4-yl)-2-chlorophenyl)-2-methoxypyridin-3-yl)methyl)pyrrolidine-3-carboxylic acid;
(R)-1-((6-(3-(2-(4-(((1-acetylpiperidin-4-yl)amino)methyl)-3-methoxyphenyl)-3-chloropyridin-4-yl)-2-chlorophenyl)-2-methoxypyridin-3-yl)methyl)pyrrolidine-3-carboxylic acid;
3-(((6-(3-(2-(4-(((1-acetylpiperidin-4-yl)amino)methyl)-3-methoxyphenyl)-3-chloropyridin-4-yl)-2-chlorophenyl)-2-methoxypyridin-3-yl)methyl)amino)propanoic acid;
3-((4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)amino)propanoic acid;
1-(4-((4-(3-chloro-4-(2-chloro-3-(6-methoxy-5-(((tetrahydro-2H-pyran-4-yl)amino)methyl)pyridin-2-yl)phenyl)pyridin-2-yl)-2-methoxybenzyl)amino)piperidin-1-yl)ethan-1-one;
1-(4-(((6-(2-chloro-3-(3-chloro-2-(3-methoxy-4-(((tetrahydro-2H-pyran-4-yl)amino)methyl)phenyl)pyridin-4-yl)phenyl)-2-methoxypyridin-3-yl)methyl)amino)piperidin-1-yl)ethan-1-one;
(S)-1-(6-(3-(2-(4-((S)-1-aminoethyl)-3-methoxyphenyl)-3-chloropyridin-4-yl)-2-chlorophenyl)-2-methoxypyridin-3-yl)ethan-1-amine;
(R)-1-(6-(3-(2-(4-((S)-1-aminoethyl)-3-methoxyphenyl)-3-chloropyridin-4-yl)-2-chlorophenyl)-2-methoxypyridin-3-yl)ethan-1-amine;
(S)-1-(6-(3-(2-(4-((R)-1-aminoethyl)-3-methoxyphenyl)-3-chloropyridin-4-yl)-2-chlorophenyl)-2-methoxypyridin-3-yl)ethan-1-amine;
(R)-1-(6-(3-(2-(4-((R)-1-aminoethyl)-3-methoxyphenyl)-3-chloropyridin-4-yl)-2-chlorophenyl)-2-methoxypyridin-3-yl)ethan-1-amine;
1-(4-(((6-(3-(2-(4-(((1-acetylpiperidin-4-yl)amino)methyl)-3-(difluoromethoxy)phenyl)-3-chloropyridin-4-yl)-2-chlorophenyl)-2-methoxypyridin-3-yl)methyl)amino)piperidin-1-yl)ethan-1-one;
1-(6-(3-(3-chloro-2-(3-methoxy-4-((methylamino)methyl)phenyl)pyridin-4-yl)-2-methylphenyl)-2-methoxypyridin-3-yl)-N-methylmethanamine;
1-((6-(2-chloro-3-(3-chloro-2-(4-((3-hydroxy-3-methylazetidin-1-yl)methyl)-3-methoxyphenyl)pyridin-4-yl)phenyl)-2-methoxypyridin-3-yl)methyl)-3-methylazetidin-3-ol;
1-(4-(((6-(2-chloro-3-(3-chloro-2-(3-methoxy-4-(((1-(2-methoxyacetyl)piperidin-4-yl)amino)methyl)phenyl)pyridin-4-yl)phenyl)-2-methoxypyridin-3-yl)methyl)amino)piperidin-1-yl)-2-methoxyethan-1-one;
2-(4-(3-chloro-4-(2-chloro-3-(6-methoxy-5-((6-oxo-2,5-diazaspiro[3.4]octan-2-yl)methyl)pyridin-2-yl)phenyl)pyridin-2-yl)-2-methoxybenzyl)-2,5-diazaspiro[3.4]octan-6-one;
1-(4-(((6-(3-(2-(4-(((1-acetylpiperidin-4-yl)amino)methyl)-3-methoxyphenyl)-3-chloropyridin-4-yl)-2-methylphenyl)-2-methoxypyridin-3-yl)methyl)amino)piperidin-1-yl)ethan-1-one;
N-(1-(4-(3-chloro-4-(2-chloro-3-(6-methoxy-5-((7-oxo-2,6-diazaspiro[3.4]octan-2-yl)methyl)pyridin-2-yl)phenyl)pyridin-2-yl)-2-methoxybenzyl)piperidin-4-yl)acetamide;
N-(1-(4-(3-chloro-4-(2-chloro-3-(6-methoxy-5-((6-oxo-2,5-diazaspiro[3.4]octan-2-yl)methyl)pyridin-2-yl)phenyl)pyridin-2-yl)-2-methoxybenzyl)piperidin-4-yl)acetamide;
N-(1-(4-(4-(3-(5-((6-acetyl-2,6-diazaspiro[3.3]heptan-2-yl)methyl)-6-methoxypyridin-2-yl)-2-chlorophenyl)-3-chloropyridin-2-yl)-2-methoxybenzyl)piperidin-4-yl)acetamide;
(S)-N-(1-(4-(3-chloro-4-(2-chloro-3-(6-methoxy-5-((((5-oxopyrrolidin-2-yl)methyl)amino)methyl)pyridin-2-yl)phenyl)pyridin-2-yl)-2-methoxybenzyl)piperidin-4-yl)acetamide;
(R)-N-(1-(4-(3-chloro-4-(2-chloro-3-(6-methoxy-5-((((5-oxopyrrolidin-2-yl)methyl)amino)methyl)pyridin-2-yl)phenyl)pyridin-2-yl)-2-methoxybenzyl)piperidin-4-yl)acetamide;
2-(4-(((1-acetylpiperidin-4-yl)amino)methyl)-3-methoxyphenyl)-4-(3-(5-(((1-acetylpiperidin-4-yl)amino)methyl)-6-methoxypyridin-2-yl)-2-chlorophenyl)nicotinonitrile;
(S)-4-((((6-(2-chloro-3-(3-chloro-2-(3-methoxy-4-(((((S)-5-oxopyrrolidin-3-yl)methyl)amino)methyl)phenyl)pyridin-4-yl)phenyl)-2-methoxypyridin-3-yl)methyl)amino)methyl)pyrrolidin-2-one;
(S)-4-((((6-(2-chloro-3-(3-chloro-2-(3-methoxy-4-(((((R)-5-oxopyrrolidin-3-yl)methyl)amino)methyl)phenyl)pyridin-4-yl)phenyl)-2-methoxypyridin-3-yl)methyl)amino)methyl)pyrrolidin-2-one;
(R)-4-((((6-(2-chloro-3-(3-chloro-2-(3-methoxy-4-(((((S)-5-oxopyrrolidin-3-yl)methyl)amino)methyl)phenyl)pyridin-4-yl)phenyl)-2-methoxypyridin-3-yl)methyl)amino)methyl)pyrrolidin-2-one;
(R)-4-((((6-(2-chloro-3-(3-chloro-2-(3-methoxy-4-(((((R)-5-oxopyrrolidin-3-yl)methyl)amino)methyl)phenyl)pyridin-4-yl)phenyl)-2-methoxypyridin-3-yl)methyl)amino)methyl)pyrrolidin-2-one;
1-(4-(((6-(3-(2-(4-((4-amino-4-methylpiperidin-1-yl)methyl)-3-methoxyphenyl)-3-chloropyridin-4-yl)-2-chlorophenyl)-2-methoxypyridin-3-yl)methyl)amino)piperidin-1-yl)ethan-1-one;
(S)-4-(((4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)amino)methyl)pyrrolidin-2-one;
(R)-4-(((4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)amino)methyl)pyrrolidin-2-one;
2-(4-(3-chloro-4-(3-(6-methoxy-5-((7-oxo-2,6-diazaspiro[3.4]octan-2-yl)methyl)pyridin-2-yl)-2-methylphenyl)pyridin-2-yl)-2-methoxybenzyl)-2,6-diazaspiro[3.4]octan-7-one;
1-(6-((6-(3-(2-(4-((6-acetyl-2,6-diazaspiro[3.3]heptan-2-yl)methyl)-3-methoxyphenyl)-3-chloropyridin-4-yl)-2-methylphenyl)-2-methoxypyridin-3-yl)methyl)-2,6-diazaspiro[3.3]heptan-2-yl)ethan-1-one;
(1s,4s)-4-(((6-(2-chloro-3-(3-chloro-2-(4-((((1s,4s)-4-hydroxycyclohexyl)amino)methyl)-3-methoxyphenyl)pyridin-4-yl)phenyl)-2-methoxypyridin-3-yl)methyl)amino)cyclohexan-1-ol;
(1r,4r)-4-(((6-(2-chloro-3-(3-chloro-2-(4-((((1r,4r)-4-hydroxycyclohexyl)amino)methyl)-3-methoxyphenyl)pyridin-4-yl)phenyl)-2-methoxypyridin-3-yl)methyl)amino)cyclohexan-1-ol;
(1r,4r)-4-(((6-(2-chloro-3-(3-chloro-2-(4-((((1r,4s)-4-hydroxycyclohexyl)amino)methyl)-3-methoxyphenyl)pyridin-4-yl)phenyl)-2-methoxypyridin-3-yl)methyl)amino)cyclohexan-1-ol;
(1r,4r)-4-(((6-(2-chloro-3-(3-chloro-2-(4-((((1s,4r)-4-hydroxycyclohexyl)amino)methyl)-3-methoxyphenyl)pyridin-4-yl)phenyl)-2-methoxypyridin-3-yl)methyl)amino)cyclohexan-1-ol;
(1r,4r)-4-(((6-(2-chloro-3-(3-chloro-2-(4-((((1s,4s)-4-hydroxycyclohexyl)amino)methyl)-3-methoxyphenyl)pyridin-4-yl)phenyl)-2-methoxypyridin-3-yl)methyl)amino)cyclohexan-1-ol;
(1r,4s)-4-(((6-(2-chloro-3-(3-chloro-2-(4-((((1r,4r)-4-hydroxycyclohexyl)amino)methyl)-3-methoxyphenyl)pyridin-4-yl)phenyl)-2-methoxypyridin-3-yl)methyl)amino)cyclohexan-1-ol;
(1r,4s)-4-(((6-(2-chloro-3-(3-chloro-2-(4-((((1r,4s)-4-hydroxycyclohexyl)amino)methyl)-3-methoxyphenyl)pyridin-4-yl)phenyl)-2-methoxypyridin-3-yl)methyl)amino)cyclohexan-1-ol;
(1r,4s)-4-(((6-(2-chloro-3-(3-chloro-2-(4-((((1s,4r)-4-hydroxycyclohexyl)amino)methyl)-3-methoxyphenyl)pyridin-4-yl)phenyl)-2-methoxypyridin-3-yl)methyl)amino)cyclohexan-1-ol;
(1r,4s)-4-(((6-(2-chloro-3-(3-chloro-2-(4-((((1s,4s)-4-hydroxycyclohexyl)amino)methyl)-3-methoxyphenyl)pyridin-4-yl)phenyl)-2-methoxypyridin-3-yl)methyl)amino)cyclohexan-1-ol;
(1s,4r)-4-(((6-(2-chloro-3-(3-chloro-2-(4-((((1r,4r)-4-hydroxycyclohexyl)amino)methyl)-3-methoxyphenyl)pyridin-4-yl)phenyl)-2-methoxypyridin-3-yl)methyl)amino)cyclohexan-1-ol;
(1s,4r)-4-(((6-(2-chloro-3-(3-chloro-2-(4-((((1r,4s)-4-hydroxycyclohexyl)amino)methyl)-3-methoxyphenyl)pyridin-4-yl)phenyl)-2-methoxypyridin-3-yl)methyl)amino)cyclohexan-1-ol;
(1s,4r)-4-(((6-(2-chloro-3-(3-chloro-2-(4-((((1s,4r)-4-hydroxycyclohexyl)amino)methyl)-3-methoxyphenyl)pyridin-4-yl)phenyl)-2-methoxypyridin-3-yl)methyl)amino)cyclohexan-1-ol;
(1s,4r)-4-(((6-(2-chloro-3-(3-chloro-2-(4-((((1s,4s)-4-hydroxycyclohexyl)amino)methyl)-3-methoxyphenyl)pyridin-4-yl)phenyl)-2-methoxypyridin-3-yl)methyl)amino)cyclohexan-1-ol;
(1s,4s)-4-(((6-(2-chloro-3-(3-chloro-2-(4-((((1r,4r)-4-hydroxycyclohexyl)amino)methyl)-3-methoxyphenyl)pyridin-4-yl)phenyl)-2-methoxypyridin-3-yl)methyl)amino)cyclohexan-1-ol;
(1s,4s)-4-(((6-(2-chloro-3-(3-chloro-2-(4-((((1r,4s)-4-hydroxycyclohexyl)amino)methyl)-3-methoxyphenyl)pyridin-4-yl)phenyl)-2-methoxypyridin-3-yl)methyl)amino)cyclohexan-1-ol;
(1s,4s)-4-(((6-(2-chloro-3-(3-chloro-2-(4-((((1s,4r)-4-hydroxycyclohexyl)amino)methyl)-3-methoxyphenyl)pyridin-4-yl)phenyl)-2-methoxypyridin-3-yl)methyl)amino)cyclohexan-1-ol;
4-((6-(2-chloro-3-(3-chloro-2-(3-methoxy-4-(morpholinomethyl)phenyl)pyridin-4-yl)phenyl)-2-methoxypyridin-3-yl)methyl)morpholine;
1-((6-(2-chloro-3-(3-chloro-2-(4-((4-hydroxypiperidin-1-yl)methyl)-3-methoxyphenyl)pyridin-4-yl)phenyl)-2-methoxypyridin-3-yl)methyl)piperidin-4-ol;
1-(4-((4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)amino)piperidin-1-yl)-2-methoxyethan-1-one;
(S)-5-((((6-(2-chloro-3-(2-(3-methoxy-4-(((((S)-5-oxopyrrolidin-2-yl)methyl)amino)methyl)phenyl)-3-methylpyridin-4-yl)phenyl)-2-methoxypyridin-3-yl)methyl)amino)methyl)pyrrolidin-2-one;
(S)-5-((((6-(2-chloro-3-(2-(3-methoxy-4-(((((R)-5-oxopyrrolidin-2-yl)methyl)amino)methyl)phenyl)-3-methylpyridin-4-yl)phenyl)-2-methoxypyridin-3-yl)methyl)amino)methyl)pyrrolidin-2-one;
(R)-5-((((6-(2-chloro-3-(2-(3-methoxy-4-(((((S)-5-oxopyrrolidin-2-yl)methyl)amino)methyl)phenyl)-3-methylpyridin-4-yl)phenyl)-2-methoxypyridin-3-yl)methyl)amino)methyl)pyrrolidin-2-one;
(R)-5-((((6-(2-chloro-3-(2-(3-methoxy-4-(((((R)-5-oxopyrrolidin-2-yl)methyl)amino)methyl)phenyl)-3-methylpyridin-4-yl)phenyl)-2-methoxypyridin-3-yl)methyl)amino)methyl)pyrrolidin-2-one;
2-(2-methoxy-4-(4-(3-(6-methoxy-5-((7-oxo-2,6-diazaspiro[3.4]octan-2-yl)methyl)pyridin-2-yl)-2-methylphenyl)-3-methylpyridin-2-yl)benzyl)-2,6-diazaspiro[3.4]octan-7-one;
1-(6-((6-(3-(2-(4-((6-acetyl-2,6-diazaspiro[3.3]heptan-2-yl)methyl)-3-methoxyphenyl)-3-methylpyridin-4-yl)-2-methylphenyl)-2-methoxypyridin-3-yl)methyl)-2,6-diazaspiro[3.3]heptan-2-yl)ethan-1-one;
1-(4-(((6-(2-chloro-3-(3-chloro-2-(3-methoxy-4-((piperidin-4-ylamino)methyl)phenyl)pyridin-4-yl)phenyl)-2-methoxypyridin-3-yl)methyl)amino)piperidin-1-yl)ethan-1-one;
4-(((6-(2-chloro-3-(3-chloro-2-(3-methoxy-4-(((1-methyl-2-oxopiperidin-4-yl)amino)methyl)phenyl)pyridin-4-yl)phenyl)-2-methoxypyridin-3-yl)methyl)amino)-1-methylpiperidin-2-one;
4-(((6-(2-chloro-3-(3-chloro-2-(4-(((1-(dimethylcarbamoyl)piperidin-4-yl)amino)methyl)-3-methoxyphenyl)pyridin-4-yl)phenyl)-2-methoxypyridin-3-yl)methyl)amino)-N,N-dimethylpiperidine-1-carboxamide;
N-((6-(2-chloro-3-(3-chloro-2-(4-(((1-(2,2-difluoroethyl)piperidin-4-yl)amino)methyl)-3-methoxyphenyl)pyridin-4-yl)phenyl)-2-methoxypyridin-3-yl)methyl)-1-(2,2-difluoroethyl)piperidin-4-amine;
(4-(((6-(2-chloro-3-(3-chloro-2-(4-(((1-(furan-2-carbonyl)piperidin-4-yl)amino)methyl)-3-methoxyphenyl)pyridin-4-yl)phenyl)-2-methoxypyridin-3-yl)methyl)amino)piperidin-1-yl)(furan-2-yl)methanone;
(4-(((6-(3-(2-(4-(((1-benzoylpiperidin-4-yl)amino)methyl)-3-methoxyphenyl)-3-chloropyridin-4-yl)-2-chlorophenyl)-2-methoxypyridin-3-yl)methyl)amino)piperidin-1-yl)(phenyl)methanone;
N-((6-(2-chloro-3-(3-chloro-2-(3-methoxy-4-(((1-phenylpiperidin-4-yl)amino)methyl)phenyl)pyridin-4-yl)phenyl)-2-methoxypyridin-3-yl)methyl)-1-phenylpiperidin-4-amine;
N-((6-(2-chloro-3-(3-chloro-2-(3-methoxy-4-(((1-(pyridin-4-yl)piperidin-4-yl)amino)methyl)phenyl)pyridin-4-yl)phenyl)-2-methoxypyridin-3-yl)methyl)-1-(pyridin-4-yl)piperidin-4-amine;
N-(2-methoxy-4-(4-(3-(6-methoxy-5-(((tetrahydro-2H-pyran-4-yl)amino)methyl)pyridin-2-yl)-2-methylphenyl)-3-methylpyridin-2-yl)benzyl)tetrahydro-2H-pyran-4-amine;
N-((6-(3-(3-chloro-2-(3-methoxy-4-(((tetrahydro-2H-pyran-4-yl)amino)methyl)phenyl)pyridin-4-yl)-2-methylphenyl)-2-methoxypyridin-3-yl)methyl)tetrahydro-2H-pyran-4-amine;
1-(4-(((6-(2-chloro-3-(3-chloro-2-(3-methoxy-4-((methyl(tetrahydro-2H-pyran-4-yl)amino)methyl)phenyl)pyridin-4-yl)phenyl)-2-methoxypyridin-3-yl)methyl)amino)piperidin-1-yl)ethan-1-one;
1-(6-((6-(3-(6'-((6-acetyl-2,6-diazaspiro[3.3]heptan-2-yl)methyl)-3-chloro-5'-methoxy-[2,3'-bipyridin]-4-yl)-2-chlorophenyl)-2-methoxypyridin-3-yl)methyl)-2,6-diazaspiro[3.3]heptan-2-yl)ethan-1-one;
N-((4-(3-(5-(((2-oxaspiro[3.3]heptan-6-yl)amino)methyl)-6-methoxypyridin-2-yl)-2-chlorophenyl)-3-chloro-5'-methoxy-[2,3'-bipyridin]-6'-yl)methyl)-2-oxaspiro[3.3]heptan-6-amine;
2-((3-chloro-4-(2-chloro-3-(6-methoxy-5-((6-oxo-2,5-diazaspiro[3.4]octan-2-yl)methyl)pyridin-2-yl)phenyl)-5'-methoxy-[2,3'-bipyridin]-6'-yl)methyl)-2,5-diazaspiro[3.4]octan-6-one;
N-((6-(2-chloro-3-(3-chloro-5'-methoxy-6'-(((tetrahydro-2H-pyran-4-yl)amino)methyl)-[2,3'-bipyridin]-4-yl)phenyl)-2-methoxypyridin-3-yl)methyl)tetrahydro-2H-pyran-4-amine;
N-(1-((6-(2-chloro-3-(3-chloro-2-(3-methoxy-4-(((tetrahydro-2H-pyran-4-yl)amino)methyl)phenyl)pyridin-4-yl)phenyl)-2-methoxypyridin-3-yl)methyl)piperidin-4-yl)acetamide;
4-(2-chloro-3-(6-methoxy-5-((7-oxo-2,6-diazaspiro[3.4]octan-2-yl)methyl)pyridin-2-yl)phenyl)-2-(3-methoxy-4-((7-oxo-2,6-diazaspiro[3.4]octan-2-yl)methyl)phenyl)nicotinonitrile;
4-(2-chloro-3-(6-methoxy-5-(((tetrahydro-2H-pyran-4-yl)amino)methyl)pyridin-2-yl)phenyl)-2-(3-methoxy-4-(((tetrahydro-2H-pyran-4-yl)amino)methyl)phenyl)nicotinonitrile;
(S)-1-((6-(2-chloro-3-(3-chloro-2-(4-(((S)-3-hydroxypyrrolidin-1-yl)methyl)-3-methoxyphenyl)pyridin-4-yl)phenyl)-2-methoxypyridin-3-yl)methyl)pyrrolidin-3-ol;
(S)-1-((6-(2-chloro-3-(3-chloro-2-(4-(((R)-3-hydroxypyrrolidin-1-yl)methyl)-3-methoxyphenyl)pyridin-4-yl)phenyl)-2-methoxypyridin-3-yl)methyl)pyrrolidin-3-ol;
(R)-1-((6-(2-chloro-3-(3-chloro-2-(4-(((S)-3-hydroxypyrrolidin-1-yl)methyl)-3-methoxyphenyl)pyridin-4-yl)phenyl)-2-methoxypyridin-3-yl)methyl)pyrrolidin-3-ol;
(R)-1-((6-(2-chloro-3-(3-chloro-2-(4-(((R)-3-hydroxypyrrolidin-1-yl)methyl)-3-methoxyphenyl)pyridin-4-yl)phenyl)-2-methoxypyridin-3-yl)methyl)pyrrolidin-3-ol;
1-(4-((4-(3-chloro-4-(2-chloro-3-(6-methoxy-5-((methyl(tetrahydro-2H-pyran-4-yl)amino)methyl)pyridin-2-yl)phenyl)pyridin-2-yl)-2-methoxybenzyl)amino)piperidin-1-yl)ethan-1-one;
N-((6-(2-chloro-3-(3-chloro-2-(3-methoxy-4-(((1-(2,2,2-trifluoroethyl)piperidin-4-yl)amino)methyl)phenyl)pyridin-4-yl)phenyl)-2-methoxypyridin-3-yl)methyl)-1-(2,2,2-trifluoroethyl)piperidin-4-amine;
2-(4-(3-chloro-4-(2-fluoro-3-(6-methoxy-5-((7-oxo-2,6-diazaspiro[3.4]octan-2-yl)methyl)pyridin-2-yl)phenyl)pyridin-2-yl)-2-methoxybenzyl)-2,6-diazaspiro[3.4]octan-7-one;
1-(4-(((6-(3-(2-(4-(((1-acetylpiperidin-4-yl)amino)methyl)-3-methoxyphenyl)-3-chloropyridin-4-yl)-2-fluorophenyl)-2-methoxypyridin-3-yl)methyl)amino)piperidin-1-yl)ethan-1-one;
N-((6-(3-(3-chloro-2-(3-methoxy-4-(((tetrahydro-2H-pyran-4-yl)amino)methyl)phenyl)pyridin-4-yl)-2-fluorophenyl)-2-methoxypyridin-3-yl)methyl)tetrahydro-2H-pyran-4-amine;
N-(4-(3-chloro-4-(2-chloro-3-(6-methoxy-5-(((tetrahydro-2H-pyran-4-yl)amino)methyl)pyridin-2-yl)phenyl)pyridin-2-yl)-2-methoxybenzyl)-N-methyltetrahydro-2H-pyran-4-amine;
1-(4-((4-(3-chloro-4-(2-chloro-3-(6-methoxy-5-(((tetrahydro-2H-pyran-4-yl)amino)methyl)pyridin-2-yl)phenyl)pyridin-2-yl)-2-methoxybenzyl)(methyl)amino)piperidin-1-yl)ethan-1-one;
1-(4-(((6-(2-chloro-3-(3-chloro-2-(4-(((1-(2-hydroxyacetyl)piperidin-4-yl)amino)methyl)-3-methoxyphenyl)pyridin-4-yl)phenyl)-2-methoxypyridin-3-yl)methyl)amino)piperidin-1-yl)-2-hydroxyethan-1-one;
N-((6-(2-chloro-3-(3-chloro-2-(3-methoxy-4-(((1-(pyridin-2-yl)piperidin-4-yl)amino)methyl)phenyl)pyridin-4-yl)phenyl)-2-methoxypyridin-3-yl)methyl)-1-(pyridin-2-yl)piperidin-4-amine;
N-((6-(2-chloro-3-(3-chloro-2-(4-(((1-(3-chloropyridin-2-yl)piperidin-4-yl)amino)methyl)-3-methoxyphenyl)pyridin-4-yl)phenyl)-2-methoxypyridin-3-yl)methyl)-1-(3-chloropyridin-2-yl)piperidin-4-amine;
N-((6-(2-chloro-3-(3-chloro-2-(3-methoxy-4-(((2-methoxyethyl)amino)methyl)phenyl)pyridin-4-yl)phenyl)-2-methoxypyridin-3-yl)methyl)-2-methoxyethan-1-amine;
3-chloro-4-(2-chloro-3-(6-methoxy-5-((4-methoxypiperidin-1-yl)methyl)pyridin-2-yl)phenyl)-2-(3-methoxy-4-((4-methoxypiperidin-1-yl)methyl)phenyl)pyridine;
(1s,4s)-N-((6-(2-chloro-3-(3-chloro-2-(3-methoxy-4-((((1s,4s)-4-methoxycyclohexyl)amino)methyl)phenyl)pyridin-4-yl)phenyl)-2-methoxypyridin-3-yl)methyl)-4-methoxycyclohexan-1-amine;
(1r,4r)-N-((6-(2-chloro-3-(3-chloro-2-(3-methoxy-4-((((1r,4r)-4-methoxycyclohexyl)amino)methyl)phenyl)pyridin-4-yl)phenyl)-2-methoxypyridin-3-yl)methyl)-4-methoxycyclohexan-1-amine;
(1r,4r)-N-((6-(2-chloro-3-(3-chloro-2-(3-methoxy-4-((((1r,4s)-4-methoxycyclohexyl)amino)methyl)phenyl)pyridin-4-yl)phenyl)-2-methoxypyridin-3-yl)methyl)-4-methoxycyclohexan-1-amine;
(1r,4r)-N-((6-(2-chloro-3-(3-chloro-2-(3-methoxy-4-((((1s,4r)-4-methoxycyclohexyl)amino)methyl)phenyl)pyridin-4-yl)phenyl)-2-methoxypyridin-3-yl)methyl)-4-methoxycyclohexan-1-amine;
(1r,4r)-N-((6-(2-chloro-3-(3-chloro-2-(3-methoxy-4-((((1s,4s)-4-methoxycyclohexyl)amino)methyl)phenyl)pyridin-4-yl)phenyl)-2-methoxypyridin-3-yl)methyl)-4-methoxycyclohexan-1-amine;
(1r,4s)-N-((6-(2-chloro-3-(3-chloro-2-(3-methoxy-4-((((1r,4r)-4-methoxycyclohexyl)amino)methyl)phenyl)pyridin-4-yl)phenyl)-2-methoxypyridin-3-yl)methyl)-4-methoxycyclohexan-1-amine;
(1r,4s)-N-((6-(2-chloro-3-(3-chloro-2-(3-methoxy-4-((((1r,4s)-4-methoxycyclohexyl)amino)methyl)phenyl)pyridin-4-yl)phenyl)-2-methoxypyridin-3-yl)methyl)-4-methoxycyclohexan-1-amine;
(1r,4s)-N-((6-(2-chloro-3-(3-chloro-2-(3-methoxy-4-((((1s,4r)-4-methoxycyclohexyl)amino)methyl)phenyl)pyridin-4-yl)phenyl)-2-methoxypyridin-3-yl)methyl)-4-methoxycyclohexan-1-amine;
(1r,4s)-N-((6-(2-chloro-3-(3-chloro-2-(3-methoxy-4-((((1s,4s)-4-methoxycyclohexyl)amino)methyl)phenyl)pyridin-4-yl)phenyl)-2-methoxypyridin-3-yl)methyl)-4-methoxycyclohexan-1-amine;
(1s,4r)-N-((6-(2-chloro-3-(3-chloro-2-(3-methoxy-4-((((1r,4r)-4-methoxycyclohexyl)amino)methyl)phenyl)pyridin-4-yl)phenyl)-2-methoxypyridin-3-yl)methyl)-4-methoxycyclohexan-1-amine;
(1s,4r)-N-((6-(2-chloro-3-(3-chloro-2-(3-methoxy-4-((((1r,4s)-4-methoxycyclohexyl)amino)methyl)phenyl)pyridin-4-yl)phenyl)-2-methoxypyridin-3-yl)methyl)-4-methoxycyclohexan-1-amine;
(1s,4r)-N-((6-(2-chloro-3-(3-chloro-2-(3-methoxy-4-((((1s,4r)-4-methoxycyclohexyl)amino)methyl)phenyl)pyridin-4-yl)phenyl)-2-methoxypyridin-3-yl)methyl)-4-methoxycyclohexan-1-amine;
(1s,4r)-N-((6-(2-chloro-3-(3-chloro-2-(3-methoxy-4-((((1s,4s)-4-methoxycyclohexyl)amino)methyl)phenyl)pyridin-4-yl)phenyl)-2-methoxypyridin-3-yl)methyl)-4-methoxycyclohexan-1-amine;
(1s,4s)-N-((6-(2-chloro-3-(3-chloro-2-(3-methoxy-4-((((1r,4r)-4-methoxycyclohexyl)amino)methyl)phenyl)pyridin-4-yl)phenyl)-2-methoxypyridin-3-yl)methyl)-4-methoxycyclohexan-1-amine;
(1s,4s)-N-((6-(2-chloro-3-(3-chloro-2-(3-methoxy-4-((((1r,4s)-4-methoxycyclohexyl)amino)methyl)phenyl)pyridin-4-yl)phenyl)-2-methoxypyridin-3-yl)methyl)-4-methoxycyclohexan-1-amine;
(1s,4s)-N-((6-(2-chloro-3-(3-chloro-2-(3-methoxy-4-((((1s,4r)-4-methoxycyclohexyl)amino)methyl)phenyl)pyridin-4-yl)phenyl)-2-methoxypyridin-3-yl)methyl)-4-methoxycyclohexan-1-amine;
1-(4-(((6-(3-(2-(4-(((1-acetylpiperidin-4-yl)amino)methyl)-3-methoxyphenyl)-3-fluoropyridin-4-yl)-2-fluorophenyl)-2-methoxypyridin-3-yl)methyl)amino)piperidin-1-yl)ethan-1-one;
(1r,4r)-N-((6-(2-chloro-3-(3-chloro-2-(3-methoxy-4-((((1r,4r)-4-methoxycyclohexyl)amino)methyl)phenyl)pyridin-4-yl)phenyl)-2-methoxypyridin-3-yl)methyl)-4-methoxycyclohexan-1-amine;
(1r,4r)-N-((6-(2-chloro-3-(3-chloro-2-(3-methoxy-4-((((1r,4s)-4-methoxycyclohexyl)amino)methyl)phenyl)pyridin-4-yl)phenyl)-2-methoxypyridin-3-yl)methyl)-4-methoxycyclohexan-1-amine;
(1r,4r)-N-((6-(2-chloro-3-(3-chloro-2-(3-methoxy-4-((((1s,4r)-4-methoxycyclohexyl)amino)methyl)phenyl)pyridin-4-yl)phenyl)-2-methoxypyridin-3-yl)methyl)-4-methoxycyclohexan-1-amine;
(1r,4r)-N-((6-(2-chloro-3-(3-chloro-2-(3-methoxy-4-((((1s,4s)-4-methoxycyclohexyl)amino)methyl)phenyl)pyridin-4-yl)phenyl)-2-methoxypyridin-3-yl)methyl)-4-methoxycyclohexan-1-amine;
(1r,4s)-N-((6-(2-chloro-3-(3-chloro-2-(3-methoxy-4-((((1r,4r)-4-methoxycyclohexyl)amino)methyl)phenyl)pyridin-4-yl)phenyl)-2-methoxypyridin-3-yl)methyl)-4-methoxycyclohexan-1-amine;
(1r,4s)-N-((6-(2-chloro-3-(3-chloro-2-(3-methoxy-4-((((1r,4s)-4-methoxycyclohexyl)amino)methyl)phenyl)pyridin-4-yl)phenyl)-2-methoxypyridin-3-yl)methyl)-4-methoxycyclohexan-1-amine;
(1r,4s)-N-((6-(2-chloro-3-(3-chloro-2-(3-methoxy-4-((((1s,4r)-4-methoxycyclohexyl)amino)methyl)phenyl)pyridin-4-yl)phenyl)-2-methoxypyridin-3-yl)methyl)-4-methoxycyclohexan-1-amine;
(1r,4s)-N-((6-(2-chloro-3-(3-chloro-2-(3-methoxy-4-((((1s,4s)-4-methoxycyclohexyl)amino)methyl)phenyl)pyridin-4-yl)phenyl)-2-methoxypyridin-3-yl)methyl)-4-methoxycyclohexan-1-amine;
(1s,4r)-N-((6-(2-chloro-3-(3-chloro-2-(3-methoxy-4-((((1r,4r)-4-methoxycyclohexyl)amino)methyl)phenyl)pyridin-4-yl)phenyl)-2-methoxypyridin-3-yl)methyl)-4-methoxycyclohexan-1-amine;
(1s,4r)-N-((6-(2-chloro-3-(3-chloro-2-(3-methoxy-4-((((1r,4s)-4-methoxycyclohexyl)amino)methyl)phenyl)pyridin-4-yl)phenyl)-2-methoxypyridin-3-yl)methyl)-4-methoxycyclohexan-1-amine;
(1s,4r)-N-((6-(2-chloro-3-(3-chloro-2-(3-methoxy-4-((((1s,4r)-4-methoxycyclohexyl)amino)methyl)phenyl)pyridin-4-yl)phenyl)-2-methoxypyridin-3-yl)methyl)-4-methoxycyclohexan-1-amine;
(1s,4r)-N-((6-(2-chloro-3-(3-chloro-2-(3-methoxy-4-((((1s,4s)-4-methoxycyclohexyl)amino)methyl)phenyl)pyridin-4-yl)phenyl)-2-methoxypyridin-3-yl)methyl)-4-methoxycyclohexan-1-amine;
(1s,4s)-N-((6-(2-chloro-3-(3-chloro-2-(3-methoxy-4-((((1r,4r)-4-methoxycyclohexyl)amino)methyl)phenyl)pyridin-4-yl)phenyl)-2-methoxypyridin-3-yl)methyl)-4-methoxycyclohexan-1-amine;
(1s,4s)-N-((6-(2-chloro-3-(3-chloro-2-(3-methoxy-4-((((1r,4s)-4-methoxycyclohexyl)amino)methyl)phenyl)pyridin-4-yl)phenyl)-2-methoxypyridin-3-yl)methyl)-4-methoxycyclohexan-1-amine;
(1s,4s)-N-((6-(2-chloro-3-(3-chloro-2-(3-methoxy-4-((((1s,4r)-4-methoxycyclohexyl)amino)methyl)phenyl)pyridin-4-yl)phenyl)-2-methoxypyridin-3-yl)methyl)-4-methoxycyclohexan-1-amine;
(1s,4s)-N-((6-(2-chloro-3-(3-chloro-2-(3-methoxy-4-((((1s,4s)-4-methoxycyclohexyl)amino)methyl)phenyl)pyridin-4-yl)phenyl)-2-methoxypyridin-3-yl)methyl)-4-methoxycyclohexan-1-amine;
2-(4-(3-fluoro-4-(2-fluoro-3-(6-methoxy-5-((7-oxo-2,6-diazaspiro[3.4]octan-2-yl)methyl)pyridin-2-yl)phenyl)pyridin-2-yl)-2-methoxybenzyl)-2,6-diazaspiro[3.4]octan-7-one;
N-((6-(2-fluoro-3-(3-fluoro-2-(3-methoxy-4-(((tetrahydro-2H-pyran-4-yl)amino)methyl)phenyl)pyridin-4-yl)phenyl)-2-methoxypyridin-3-yl)methyl)tetrahydro-2H-pyran-4-amine;
(1r,4r)-4-(((6-(2-chloro-3-(3-chloro-2-(2-((((1r,4r)-4-hydroxycyclohexyl)amino)methyl)-8-methoxyimidazo[1,2-a]pyridin-6-yl)pyridin-4-yl)phenyl)-2-methoxypyridin-3-yl)methyl)amino)cyclohexan-1-ol;
(1r,4r)-4-(((6-(2-chloro-3-(3-chloro-2-(2-((((1r,4s)-4-hydroxycyclohexyl)amino)methyl)-8-methoxyimidazo[1,2-a]pyridin-6-yl)pyridin-4-yl)phenyl)-2-methoxypyridin-3-yl)methyl)amino)cyclohexan-1-ol;
(1r,4r)-4-(((6-(2-chloro-3-(3-chloro-2-(2-((((1s,4r)-4-hydroxycyclohexyl)amino)methyl)-8-methoxyimidazo[1,2-a]pyridin-6-yl)pyridin-4-yl)phenyl)-2-methoxypyridin-3-yl)methyl)amino)cyclohexan-1-ol;
(1r,4r)-4-(((6-(2-chloro-3-(3-chloro-2-(2-((((1s,4s)-4-hydroxycyclohexyl)amino)methyl)-8-methoxyimidazo[1,2-a]pyridin-6-yl)pyridin-4-yl)phenyl)-2-methoxypyridin-3-yl)methyl)amino)cyclohexan-1-ol;
(1r,4s)-4-(((6-(2-chloro-3-(3-chloro-2-(2-((((1r,4r)-4-hydroxycyclohexyl)amino)methyl)-8-methoxyimidazo[1,2-a]pyridin-6-yl)pyridin-4-yl)phenyl)-2-methoxypyridin-3-yl)methyl)amino)cyclohexan-1-ol;
(1r,4s)-4-(((6-(2-chloro-3-(3-chloro-2-(2-((((1r,4s)-4-hydroxycyclohexyl)amino)methyl)-8-methoxyimidazo[1,2-a]pyridin-6-yl)pyridin-4-yl)phenyl)-2-methoxypyridin-3-yl)methyl)amino)cyclohexan-1-ol;
(1r,4s)-4-(((6-(2-chloro-3-(3-chloro-2-(2-((((1s,4r)-4-hydroxycyclohexyl)amino)methyl)-8-methoxyimidazo[1,2-a]pyridin-6-yl)pyridin-4-yl)phenyl)-2-methoxypyridin-3-yl)methyl)amino)cyclohexan-1-ol;
(1r,4s)-4-(((6-(2-chloro-3-(3-chloro-2-(2-((((1s,4s)-4-hydroxycyclohexyl)amino)methyl)-8-methoxyimidazo[1,2-a]pyridin-6-yl)pyridin-4-yl)phenyl)-2-methoxypyridin-3-yl)methyl)amino)cyclohexan-1-ol;
(1s,4r)-4-(((6-(2-chloro-3-(3-chloro-2-(2-((((1r,4r)-4-hydroxycyclohexyl)amino)methyl)-8-methoxyimidazo[1,2-a]pyridin-6-yl)pyridin-4-yl)phenyl)-2-methoxypyridin-3-yl)methyl)amino)cyclohexan-1-ol;
(1s,4r)-4-(((6-(2-chloro-3-(3-chloro-2-(2-((((1r,4s)-4-hydroxycyclohexyl)amino)methyl)-8-methoxyimidazo[1,2-a]pyridin-6-yl)pyridin-4-yl)phenyl)-2-methoxypyridin-3-yl)methyl)amino)cyclohexan-1-ol;
(1s,4r)-4-(((6-(2-chloro-3-(3-chloro-2-(2-((((1s,4r)-4-hydroxycyclohexyl)amino)methyl)-8-methoxyimidazo[1,2-a]pyridin-6-yl)pyridin-4-yl)phenyl)-2-methoxypyridin-3-yl)methyl)amino)cyclohexan-1-ol;
(1s,4r)-4-(((6-(2-chloro-3-(3-chloro-2-(2-((((1s,4s)-4-hydroxycyclohexyl)amino)methyl)-8-methoxyimidazo[1,2-a]pyridin-6-yl)pyridin-4-yl)phenyl)-2-methoxypyridin-3-yl)methyl)amino)cyclohexan-1-ol;
(1s,4s)-4-(((6-(2-chloro-3-(3-chloro-2-(2-((((1r,4r)-4-hydroxycyclohexyl)amino)methyl)-8-methoxyimidazo[1,2-a]pyridin-6-yl)pyridin-4-yl)phenyl)-2-methoxypyridin-3-yl)methyl)amino)cyclohexan-1-ol;
(1s,4s)-4-(((6-(2-chloro-3-(3-chloro-2-(2-((((1r,4s)-4-hydroxycyclohexyl)amino)methyl)-8-methoxyimidazo[1,2-a]pyridin-6-yl)pyridin-4-yl)phenyl)-2-methoxypyridin-3-yl)methyl)amino)cyclohexan-1-ol;
(1s,4s)-4-(((6-(2-chloro-3-(3-chloro-2-(2-((((1s,4r)-4-hydroxycyclohexyl)amino)methyl)-8-methoxyimidazo[1,2-a]pyridin-6-yl)pyridin-4-yl)phenyl)-2-methoxypyridin-3-yl)methyl)amino)cyclohexan-1-ol;
(1s,4s)-4-(((6-(2-chloro-3-(3-chloro-2-(2-((((1s,4s)-4-hydroxycyclohexyl)amino)methyl)-
8-methoxyimidazo[1,2-a]pyridin-6-yl)pyridin-4-yl)phenyl)-2-methoxypyridin-3-yl)methyl)amino)cyclohexan-1-ol;
2-((6-(3-chloro-4-(2-chloro-3-(6-methoxy-5-((6-oxo-2,5-diazaspiro[3.4]octan-2-yl)methyl)pyridin-2-yl)phenyl)pyridin-2-yl)-8-methoxyimidazo[1,2-a]pyridin-2-yl)methyl)-2,5-diazaspiro[3.4]octan-6-one; 1-((6-(2-chloro-3-(3-chloro-2-(2-((3-hydroxy-3-methylazetidin-1-yl)methyl)-8-methoxyimidazo[1,2-a]pyridin-6-yl)pyridin-4-yl)phenyl)-2-methoxypyridin-3-yl)methyl)-3-methylazetidin-3-ol;
(4-(((6-(2-chloro-3-(3-chloro-2-(4-(((1-(cyclohexanecarbonyl)piperidin-4-yl)amino)methyl)-3-methoxyphenyl)pyridin-4-yl)phenyl)-2-methoxypyridin-3-yl)methyl)amino)piperidin-1-yl)(cyclohexyl)methanone;
4-((6-(2-chloro-3-(3-chloro-2-(3-methoxy-4-((3-oxopiperazin-1-yl)methyl)phenyl)pyridin-4-yl)phenyl)-2-methoxypyridin-3-yl)methyl)piperazin-2-one;
1-(4-(((6-(2-chloro-3-(3-chloro-2-(3-methoxy-4-(morpholinomethyl)phenyl)pyridin-4-yl)phenyl)-2-methoxypyridin-3-yl)methyl)amino)piperidin-1-yl)ethan-1-one;
1-(4-(((6-(2-chloro-3-(3-chloro-2-(4-((4-hydroxypiperidin-1-yl)methyl)-3-methoxyphenyl)pyridin-4-yl)phenyl)-2-methoxypyridin-3-yl)methyl)amino)piperidin-1-yl)ethan-1-one;
(S)-(2-(6-(3-chloro-4-(2-chloro-3-(6-methoxy-5-((((5-oxopyrrolidin-2-yl)methyl)amino)methyl)pyridin-2-yl)phenyl)pyridin-2-yl)-2-methoxy-4-oxopyrrolo[2,1-f][1,2,4]triazin-3(4H)-yl)ethyl)glycine;
(R)-(2-(6-(3-chloro-4-(2-chloro-3-(6-methoxy-5-((((5-oxopyrrolidin-2-yl)methyl)amino)methyl)pyridin-2-yl)phenyl)pyridin-2-yl)-2-methoxy-4-oxopyrrolo[2,1-f][1,2,4]triazin-3(4H)-yl)ethyl)glycine;
N-(1-((6-(3-(2-(4-((4-acetamidopiperidin-1-yl)methyl)-3-methoxyphenyl)-3-methylpyridin-4-yl)-2-methylphenyl)-2-methoxypyridin-3-yl)methyl)piperidin-4-yl)acetamide;
N-((6-(2-chloro-3-(2-(3-methoxy-4-(((tetrahydro-2H-pyran-4-yl)amino)methyl)phenyl)-3-methylpyridin-4-yl)phenyl)-2-methoxypyridin-3-yl)methyl)tetrahydro-2H-pyran-4-amine;
(1r,4r)-4-(((6-(2-chloro-3-(2-(4-((((1r,4r)-4-hydroxycyclohexyl)amino)methyl)-3-methoxyphenyl)-3-methylpyridin-4-yl)phenyl)-2-methoxypyridin-3-yl)methyl)amino)cyclohexan-1-ol;
(1r,4r)-4-(((6-(2-chloro-3-(2-(4-((((1r,4s)-4-hydroxycyclohexyl)amino)methyl)-3-methoxyphenyl)-3-methylpyridin-4-yl)phenyl)-2-methoxypyridin-3-yl)methyl)amino)cyclohexan-1-ol;
(1r,4r)-4-(((6-(2-chloro-3-(2-(4-((((1s,4r)-4-hydroxycyclohexyl)amino)methyl)-3-methoxyphenyl)-3-methylpyridin-4-yl)phenyl)-2-methoxypyridin-3-yl)methyl)amino)cyclohexan-1-ol;
(1r,4r)-4-(((6-(2-chloro-3-(2-(4-((((1s,4s)-4-hydroxycyclohexyl)amino)methyl)-3-methoxyphenyl)-3-methylpyridin-4-yl)phenyl)-2-methoxypyridin-3-yl)methyl)amino)cyclohexan-1-ol;
(1r,4s)-4-(((6-(2-chloro-3-(2-(4-((((1r,4r)-4-hydroxycyclohexyl)amino)methyl)-3-methoxyphenyl)-3-methylpyridin-4-yl)phenyl)-2-methoxypyridin-3-yl)methyl)amino)cyclohexan-1-ol;
(1r,4s)-4-(((6-(2-chloro-3-(2-(4-((((1r,4s)-4-hydroxycyclohexyl)amino)methyl)-3-methoxyphenyl)-3-methylpyridin-4-yl)phenyl)-2-methoxypyridin-3-yl)methyl)amino)cyclohexan-1-ol;
(1r,4s)-4-(((6-(2-chloro-3-(2-(4-((((1s,4r)-4-hydroxycyclohexyl)amino)methyl)-3-methoxyphenyl)-3-methylpyridin-4-yl)phenyl)-2-methoxypyridin-3-yl)methyl)amino)cyclohexan-1-ol;
(1r,4s)-4-(((6-(2-chloro-3-(2-(4-((((1s,4s)-4-hydroxycyclohexyl)amino)methyl)-3-methoxyphenyl)-3-methylpyridin-4-yl)phenyl)-2-methoxypyridin-3-yl)methyl)amino)cyclohexan-1-ol;
(1s,4r)-4-(((6-(2-chloro-3-(2-(4-((((1r,4r)-4-hydroxycyclohexyl)amino)methyl)-3-methoxyphenyl)-3-methylpyridin-4-yl)phenyl)-2-methoxypyridin-3-yl)methyl)amino)cyclohexan-1-ol;
(1s,4r)-4-(((6-(2-chloro-3-(2-(4-((((1r,4s)-4-hydroxycyclohexyl)amino)methyl)-3-methoxyphenyl)-3-methylpyridin-4-yl)phenyl)-2-methoxypyridin-3-yl)methyl)amino)cyclohexan-1-ol;
(1s,4r)-4-(((6-(2-chloro-3-(2-(4-((((1s,4r)-4-hydroxycyclohexyl)amino)methyl)-3-methoxyphenyl)-3-methylpyridin-4-yl)phenyl)-2-methoxypyridin-3-yl)methyl)amino)cyclohexan-1-ol;
(1s,4r)-4-(((6-(2-chloro-3-(2-(4-((((1s,4s)-4-hydroxycyclohexyl)amino)methyl)-3-methoxyphenyl)-3-methylpyridin-4-yl)phenyl)-2-methoxypyridin-3-yl)methyl)amino)cyclohexan-1-ol;
(1s,4s)-4-(((6-(2-chloro-3-(2-(4-((((1r,4r)-4-hydroxycyclohexyl)amino)methyl)-3-methoxyphenyl)-3-methylpyridin-4-yl)phenyl)-2-methoxypyridin-3-yl)methyl)amino)cyclohexan-1-ol;
(1s,4s)-4-(((6-(2-chloro-3-(2-(4-((((1r,4s)-4-hydroxycyclohexyl)amino)methyl)-3-methoxyphenyl)-3-methylpyridin-4-yl)phenyl)-2-methoxypyridin-3-yl)methyl)amino)cyclohexan-1-ol;
(1s,4s)-4-(((6-(2-chloro-3-(2-(4-((((1s,4r)-4-hydroxycyclohexyl)amino)methyl)-3-methoxyphenyl)-3-methylpyridin-4-yl)phenyl)-2-methoxypyridin-3-yl)methyl)amino)cyclohexan-1-ol;
(1s,4s)-4-(((6-(2-chloro-3-(2-(4-((((1s,4s)-4-hydroxycyclohexyl)amino)methyl)-3-methoxyphenyl)-3-methylpyridin-4-yl)phenyl)-2-methoxypyridin-3-yl)methyl)amino)cyclohexan-1-ol;
6-(4-(4-(3-(5-((2-oxa-6-azaspiro[3.3]heptan-6-yl)methyl)-6-methoxypyridin-2-yl)-2-chlorophenyl)-3-methylpyridin-2-yl)-2-methoxybenzyl)-2-oxa-6-azaspiro[3.3]heptane;
methyl 4-(((6-(2-chloro-3-(3-chloro-2-(3-methoxy-4-(((1-(methoxycarbonyl)piperidin-4-yl)amino)methyl)phenyl)pyridin-4-yl)phenyl)-2-methoxypyridin-3-yl)methyl)amino)piperidine-1-carboxylate;
N-(1-((6-(3-(2-(4-((4-acetamidopiperidin-1-yl)methyl)-3-methoxyphenyl)-3-methylpyridin-4-yl)-2-chlorophenyl)-2-methoxypyridin-3-yl)methyl)piperidin-4-yl)acetamide;
N-(1-((6-(3-(2-(4-((4-acetamidopiperidin-1-yl)methyl)-3-methoxyphenyl)-3-chloropyridin-4-yl)-2-methylphenyl)-2-methoxypyridin-3-yl)methyl)piperidin-4-yl)acetamide;
N-((6-(2-chloro-3-(3-chloro-2-(3-methoxy-4-(((tetrahydro-2H-pyran-4-yl)amino)methyl)phenyl)pyridin-4-yl)phenyl)-2-methoxypyridin-3-yl)methyl)-N-methyltetrahydro-2H-pyran-4-amine;
1-(4-(((6-(2-chloro-3-(3-chloro-2-(3-methoxy-4-(((tetrahydro-2H-pyran-4-yl)amino)methyl)phenyl)pyridin-4-yl)phenyl)-2-methoxypyridin-3-yl)methyl)(methyl)amino)piperidin-1-yl)ethan-1-one;
2-(4-(4-(3-(5-((7-oxa-2-azaspiro[3.5]nonan-2-yl)methyl)-6-methoxypyridin-2-yl)-2-chlorophenyl)-3-chloropyridin-2-yl)-2-methoxybenzyl)-7-oxa-2-azaspiro[3.5]nonane;
2-(1-((6-(2-chloro-3-(3-chloro-2-(4-((3-(2-hydroxypropan-2-yl)azetidin-1-yl)methyl)-3-methoxyphenyl)pyridin-4-yl)phenyl)-2-methoxypyridin-3-yl)methyl)azetidin-3-yl)propan-2-ol;
(S)-5-((((6-(2-chloro-3-(3-chloro-2-(3-methoxy-4-((methyl(((S)-5-oxopyrrolidin-2-yl)methyl)amino)methyl)phenyl)pyridin-4-yl)phenyl)-2-methoxypyridin-3-yl)methyl)(methyl)amino)methyl)pyrrolidin-2-one;
(S)-5-((((6-(2-chloro-3-(3-chloro-2-(3-methoxy-4-((methyl(((R)-5-oxopyrrolidin-2-yl)methyl)amino)methyl)phenyl)pyridin-4-yl)phenyl)-2-methoxypyridin-3-yl)methyl)(methyl)amino)methyl)pyrrolidin-2-one;
(R)-5-((((6-(2-chloro-3-(3-chloro-2-(3-methoxy-4-((methyl(((S)-5-oxopyrrolidin-2-yl)methyl)amino)methyl)phenyl)pyridin-4-yl)phenyl)-2-methoxypyridin-3-yl)methyl)(methyl)amino)methyl)pyrrolidin-2-one;
(R)-5-((((6-(2-chloro-3-(3-chloro-2-(3-methoxy-4-((methyl(((R)-5-oxopyrrolidin-2-yl)methyl)amino)methyl)phenyl)pyridin-4-yl)phenyl)-2-methoxypyridin-3-yl)methyl)(methyl)amino)methyl)pyrrolidin-2-one;
(R)-4-((((6-(2-chloro-3-(3-chloro-2-(3-methoxy-4-((methyl(((R)-5-oxopyrrolidin-3-yl)methyl)amino)methyl)phenyl)pyridin-4-yl)phenyl)-2-methoxypyridin-3-yl)methyl)(methyl)amino)methyl)pyrrolidin-2-one;
(S)-4-((((6-(2-chloro-3-(3-chloro-2-(3-methoxy-4-((methyl(((R)-5-oxopyrrolidin-3-yl)methyl)amino)methyl)phenyl)pyridin-4-yl)phenyl)-2-methoxypyridin-3-yl)methyl)(methyl)amino)methyl)pyrrolidin-2-one;
(R)-4-((((6-(2-chloro-3-(3-chloro-2-(3-methoxy-4-((methyl(((S)-5-oxopyrrolidin-3-yl)methyl)amino)methyl)phenyl)pyridin-4-yl)phenyl)-2-methoxypyridin-3-yl)methyl)(methyl)amino)methyl)pyrrolidin-2-one;
(S)-4-((((6-(2-chloro-3-(3-chloro-2-(3-methoxy-4-((methyl(((S)-5-oxopyrrolidin-3-yl)methyl)amino)methyl)phenyl)pyridin-4-yl)phenyl)-2-methoxypyridin-3-yl)methyl)(methyl)amino)methyl)pyrrolidin-2-one;
2-((6-(3-chloro-4-(2-chloro-3-(5-((6-hydroxy-2-azaspiro[3.3]heptan-2-yl)methyl)-6-methoxypyridin-2-yl)phenyl)pyridin-2-yl)-8-methoxyimidazo[1,2-a]pyridin-2-yl)methyl)-2-azaspiro[3.3]heptan-6-ol;
1-(4-(((6-(2-chloro-3-(3-chloro-2-(8-methoxy-2-(morpholinomethyl)imidazo[1,2-a]pyridin-6-yl)pyridin-4-yl)phenyl)-2-methoxypyridin-3-yl)methyl)amino)piperidin-1-yl)ethan-1-one;
1-(4-(((6-(2-chloro-3-(3-chloro-2-(2-((3,3-difluoropyrrolidin-1-yl)methyl)-8-methoxyimidazo[1,2-a]pyridin-6-yl)pyridin-4-yl)phenyl)-2-methoxypyridin-3-yl)methyl)amino)piperidin-1-yl)ethan-1-one;
1-((6-(4-(3-(5-(((1-acetylpiperidin-4-yl)amino)methyl)-6-methoxypyridin-2-yl)-2-chlorophenyl)-3-chloropyridin-2-yl)-8-methoxyimidazo[1,2-a]pyridin-2-yl)methyl)-3-methylazetidine-3-carbonitrile;
1-(4-(((6-(2-chloro-3-(3-chloro-2-(2-(((2,2-difluoroethyl)amino)methyl)-8-methoxyimidazo[1,2-a]pyridin-6-yl)pyridin-4-yl)phenyl)-2-methoxypyridin-3-yl)methyl)amino)piperidin-1-yl)ethan-1-one;
4-(2-((4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)amino)ethyl)piperazin-2-one;
2-(3-(6-(4-(3-(5-(((1-acetylpiperidin-4-yl)amino)methyl)-6-methoxypyridin-2-yl)-2-chlorophenyl)-3-chloropyridin-2-yl)-8-methoxy-3,4-dihydroisoquinolin-2(1H)-yl)-2-oxopyrrolidin-1-yl)acetic acid;
1-(4-(((6-(3-(2-(4-(((1-acetylpiperidin-4-yl)amino)methyl)-3-methoxyphenyl)-3-chloropyridin-4-yl)-2-chlorophenyl)-2-methoxypyridin-3-yl)methyl)amino)piperidin-1-yl)-2-methoxyethan-1-one;
1-(4-(((6-(3-(2-(4-((6-acetyl-2,6-diazaspiro[3.3]heptan-2-yl)methyl)-3-methoxyphenyl)-3-chloropyridin-4-yl)-2-chlorophenyl)-2-methoxypyridin-3-yl)methyl)amino)piperidin-1-yl)ethan-1-one;
1-(4-((4-(4-(3-(5-((6-acetyl-2,6-diazaspiro[3.3]heptan-2-yl)methyl)-6-methoxypyridin-2-yl)-2-chlorophenyl)-3-chloropyridin-2-yl)-2-methoxybenzyl)amino)piperidin-1-yl)ethan-1-one;
N-((6-(2-chloro-3-(3-chloro-2-(3-methoxy-4-(((tetrahydro-2H-thiopyran-4-yl)amino)methyl)phenyl)pyridin-4-yl)phenyl)-2-methoxypyridin-3-yl)methyl)tetrahydro-2H-thiopyran-4-amine;
1-(2-((6-(3-(2-(4-((7-acetyl-2,7-diazaspiro[3.5]nonan-2-yl)methyl)-3-methoxyphenyl)-3-chloropyridin-4-yl)-2-chlorophenyl)-2-methoxypyridin-3-yl)methyl)-2,7-diazaspiro[3.5]nonan-7-yl)ethan-1-one;
(S)-5-((((6-(((S)-4-(5-(((1-acetylpiperidin-4-yl)amino)methyl)-3'-chloro-6-methoxy-[2,4'-bipyridin]-2'-yl)-2,3-dihydro-1H-inden-1-yl)amino)-2-methoxy-5-(trifluoromethyl)pyridin-3-yl)methyl)amino)methyl)pyrrolidin-2-one;
(S)-5-((((6-(((R)-4-(5-(((1-acetylpiperidin-4-yl)amino)methyl)-3'-chloro-6-methoxy-[2,4'-bipyridin]-2'-yl)-2,3-dihydro-1H-inden-1-yl)amino)-2-methoxy-5-(trifluoromethyl)pyridin-3-yl)methyl)amino)methyl)pyrrolidin-2-one;
(R)-5-((((6-(((S)-4-(5-(((1-acetylpiperidin-4-yl)amino)methyl)-3'-chloro-6-methoxy-[2,4'-bipyridin]-2'-yl)-2,3-dihydro-1H-inden-1-yl)amino)-2-methoxy-5-(trifluoromethyl)pyridin-3-yl)methyl)amino)methyl)pyrrolidin-2-one;
(R)-5-((((6-(((R)-4-(5-(((1-acetylpiperidin-4-yl)amino)methyl)-3'-chloro-6-methoxy-[2,4'-bipyridin]-2'-yl)-2,3-dihydro-1H-inden-1-yl)amino)-2-methoxy-5-(trifluoromethyl)pyridin-3-yl)methyl)amino)methyl)pyrrolidin-2-one;
1-(4-((4-(3-chloro-4-(2-chloro-3-(5-((4-hydroxypiperidin-1-yl)methyl)-6-methoxypyridin-2-yl)phenyl)pyridin-2-yl)-2-methoxybenzyl)amino)piperidin-1-yl)ethan-1-one;
1-(4-((4-(3-chloro-4-(2-chloro-3-(6-methoxy-5-((((1r,4r)-4-methoxycyclohexyl)amino)methyl)pyridin-2-yl)phenyl)pyridin-2-yl)-2-methoxybenzyl)amino)piperidin-1-yl)ethan-1-one;
1-(4-((4-(3-chloro-4-(2-chloro-3-(6-methoxy-5-((((1s,4r)-4-methoxycyclohexyl)amino)methyl)pyridin-2-yl)phenyl)pyridin-2-yl)-2-methoxybenzyl)amino)piperidin-1-yl)ethan-1-one;
1-(4-((4-(3-chloro-4-(2-chloro-3-(6-methoxy-5-((((1r,4s)-4-methoxycyclohexyl)amino)methyl)pyridin-2-yl)phenyl)pyridin-2-yl)-2-methoxybenzyl)amino)piperidin-1-yl)ethan-1-one;
1-(4-((4-(3-chloro-4-(2-chloro-3-(6-methoxy-5-((((1s,4s)-4-methoxycyclohexyl)amino)methyl)pyridin-2-yl)phenyl)pyridin-2-yl)-2-methoxybenzyl)amino)piperidin-1-yl)ethan-1-one;
(R)-1-(4-(((6-(2-chloro-3-(3-chloro-2-(4-((3-hydroxypiperidin-1-yl)methyl)-3-methoxyphenyl)pyridin-4-yl)phenyl)-2-methoxypyridin-3-yl)methyl)amino)piperidin-1-yl)ethan-1-one;
(S)-1-(4-(((6-(2-chloro-3-(3-chloro-2-(4-((3-hydroxypiperidin-1-yl)methyl)-3-methoxyphenyl)pyridin-4-yl)phenyl)-2-methoxypyridin-3-yl)methyl)amino)piperidin-1-yl)ethan-1-one;
2-(4-(3-chloro-4-(2-chloro-3-(6-methoxy-5-(((1-(2-methoxyacetyl)piperidin-4-yl)amino)methyl)pyridin-2-yl)phenyl)pyridin-2-yl)-2-methoxybenzyl)-2,6-diazaspiro[3.4]octan-7-one;
1-(4-(((6-(3-(2-(4-((6-acetyl-2,6-diazaspiro[3.3]heptan-2-yl)methyl)-3-methoxyphenyl)-3-chloropyridin-4-yl)-2-chlorophenyl)-2-methoxypyridin-3-yl)methyl)amino)piperidin-1-yl)-2-methoxyethan-1-one;
1-(6-(4-(3-chloro-4-(2-chloro-3-(6-methoxy-5-(((tetrahydro-2H-pyran-4-yl)amino)methyl)pyridin-2-yl)phenyl)pyridin-2-yl)-2-methoxybenzyl)-2,6-diazaspiro[3.3]heptan-2-yl)ethan-1-one;
2-((6-(3-(2-(4-((6-acetyl-2,6-diazaspiro[3.3]heptan-2-yl)methyl)-3-methoxyphenyl)-3-chloropyridin-4-yl)-2-chlorophenyl)-2-methoxypyridin-3-yl)methyl)-2,6-diazaspiro[3.4]octan-7-one;
2-((6-(3-(2-(4-((2,6-diazaspiro[3.3]heptan-2-yl)methyl)-3-methoxyphenyl)-3-chloropyridin-4-yl)-2-chlorophenyl)-2-methoxypyridin-3-yl)methyl)-2,6-diazaspiro[3.4]octan-7-one;
2-((6-(2-chloro-3-(3-chloro-2-(3-methoxy-4-(((tetrahydro-2H-pyran-4-yl)amino)methyl)phenyl)pyridin-4-yl)phenyl)-2-methoxypyridin-3-yl)methyl)-2,6-diazaspiro[3.4]octan-7-one;
2-((6-(2-chloro-3-(3-chloro-2-(4-((((1r,3r)-3-hydroxy-3-methylcyclobutyl)amino)methyl)-3-methoxyphenyl)pyridin-4-yl)phenyl)-2-methoxypyridin-3-yl)methyl)-2,6-diazaspiro[3.4]octan-7-one;
2-((6-(2-chloro-3-(3-chloro-2-(4-((((1s,3r)-3-hydroxy-3-methylcyclobutyl)amino)methyl)-3-methoxyphenyl)pyridin-4-yl)phenyl)-2-methoxypyridin-3-yl)methyl)-2,6-diazaspiro[3.4]octan-7-one;
2-((6-(2-chloro-3-(3-chloro-2-(4-((((1r,3s)-3-hydroxy-3-methylcyclobutyl)amino)methyl)-3-methoxyphenyl)pyridin-4-yl)phenyl)-2-methoxypyridin-3-yl)methyl)-2,6-diazaspiro[3.4]octan-7-one;
2-((6-(2-chloro-3-(3-chloro-2-(4-((((1s,3s)-3-hydroxy-3-methylcyclobutyl)amino)methyl)-3-methoxyphenyl)pyridin-4-yl)phenyl)-2-methoxypyridin-3-yl)methyl)-2,6-diazaspiro[3.4]octan-7-one;
2-((6-(2-chloro-3-(3-chloro-2-(4-((6-hydroxy-2-azaspiro[3.3]heptan-2-yl)methyl)-3-methoxyphenyl)pyridin-4-yl)phenyl)-2-methoxypyridin-3-yl)methyl)-2,6-diazaspiro[3.4]octan-7-one;
2-((6-(2-chloro-3-(3-chloro-2-(4-((3-hydroxy-3-methylazetidin-1-yl)methyl)-3-methoxyphenyl)pyridin-4-yl)phenyl)-2-methoxypyridin-3-yl)methyl)-2,6-diazaspiro[3.4]octan-7-one;
1-(3-((((6-(3-(2-(4-((((1-acetylazetidin-3-yl)methyl)(methyl)amino)methyl)-3-methoxyphenyl)-3-chloropyridin-4-yl)-2-chlorophenyl)-2-methoxypyridin-3-yl)methyl)(methyl)amino)methyl)azetidin-1-yl)ethan-1-one;
1-(4-(((4-(4'-(((1-acetylpiperidin-4-yl)amino)methyl)-2-chloro-3'-methoxy-[1,1'-biphenyl]-3-yl)-3-chloro-5'-methoxy-[2,3'-bipyridin]-6'-yl)methyl)amino)piperidin-1-yl)ethan-1-one;
2-((2'-chloro-3'-(3-chloro-5'-methoxy-6'-((7-oxo-2,6-diazaspiro[3.4]octan-2-yl)methyl)-[2,3'-bipyridin]-4-yl)-3-methoxy-[1,1'-biphenyl]-4-yl)methyl)-2,6-diazaspiro[3.4]octan-7-one;
2-((6-(2-chloro-3-(3-chloro-2-(4-((((1r,4r)-4-hydroxycyclohexyl)amino)methyl)-3-methoxyphenyl)pyridin-4-yl)phenyl)-2-methoxypyridin-3-yl)methyl)-2,6-diazaspiro[3.4]octan-7-one;
2-((6-(2-chloro-3-(3-chloro-2-(4-((((1s,4r)-4-hydroxycyclohexyl)amino)methyl)-3-methoxyphenyl)pyridin-4-yl)phenyl)-2-methoxypyridin-3-yl)methyl)-2,6-diazaspiro[3.4]octan-7-one;
2-((6-(2-chloro-3-(3-chloro-2-(4-((((1r,4s)-4-hydroxycyclohexyl)amino)methyl)-3-methoxyphenyl)pyridin-4-yl)phenyl)-2-methoxypyridin-3-yl)methyl)-2,6-diazaspiro[3.4]octan-7-one;
2-((6-(2-chloro-3-(3-chloro-2-(4-((((1s,4s)-4-hydroxycyclohexyl)amino)methyl)-3-methoxyphenyl)pyridin-4-yl)phenyl)-2-methoxypyridin-3-yl)methyl)-2,6-diazaspiro[3.4]octan-7-one;
1-((((6-(2-chloro-3-(3-chloro-2-(4-((((1-hydroxycyclopropyl)methyl)amino)methyl)-3-methoxyphenyl)pyridin-4-yl)phenyl)-2-methoxypyridin-3-yl)methyl)amino)methyl)cyclopropan-1-ol;
N-((6-(2-chloro-3-(3-chloro-2-(4-(((2,2-difluoroethyl)amino)methyl)-3-methoxyphenyl)pyridin-4-yl)phenyl)-2-methoxypyridin-3-yl)methyl)-2,2-difluoroethan-1-amine;
2-(4-(3-chloro-4-(2-chloro-3-(6-methoxy-5-(((tetrahydro-2H-pyran-4-yl)amino)methyl)pyridin-2-yl)phenyl)pyridin-2-yl)-2-methoxybenzyl)-2,6-diazaspiro[3.4]octan-7-one;
(S)-1-(4-(((6-(2-chloro-3-(3-chloro-2-(4-((3-hydroxypiperidin-1-yl)methyl)-3-methoxyphenyl)pyridin-4-yl)phenyl)-2-methoxypyridin-3-yl)methyl)amino)piperidin-1-yl)ethan-1-one;
(R)-1-(4-(((6-(2-chloro-3-(3-chloro-2-(4-((3-hydroxypiperidin-1-yl)methyl)-3-methoxyphenyl)pyridin-4-yl)phenyl)-2-methoxypyridin-3-yl)methyl)amino)piperidin-1-yl)ethan-1-one;
1-(4-(((6-(2-chloro-3-(3-chloro-2-(4-((4-(hydroxymethyl)piperidin-1-yl)methyl)-3-methoxyphenyl)pyridin-4-yl)phenyl)-2-methoxypyridin-3-yl)methyl)amino)piperidin-1-yl)ethan-1-one;
1-(4-(((6-(2-chloro-3-(3-chloro-2-(3-methoxy-4-((4-(methoxymethyl)piperidin-1-yl)methyl)phenyl)pyridin-4-yl)phenyl)-2-methoxypyridin-3-yl)methyl)amino)piperidin-1-yl)ethan-1-one;
1-(4-(((6-(2-chloro-3-(3-chloro-2-(4-((4-hydroxypiperidin-1-yl)methyl)-3-methoxyphenyl)pyridin-4-yl)phenyl)-2-methoxypyridin-3-yl)methyl)amino)piperidin-1-yl)-2-methoxyethan-1-one;
1-(6-((6-(2-chloro-3-(3-chloro-2-(3-methoxy-4-(((tetrahydro-2H-pyran-4-yl)amino)methyl)phenyl)pyridin-4-yl)phenyl)-2-methoxypyridin-3-yl)methyl)-2,6-diazaspiro[3.3]heptan-2-yl)ethan-1-one;
1-(4-(((6-(2-chloro-3-(3-chloro-2-(4-((((1-hydroxycyclopropyl)methyl)amino)methyl)-3-methoxyphenyl)pyridin-4-yl)phenyl)-2-methoxypyridin-3-yl)methyl)amino)piperidin-1-yl)ethan-1-one;
1-(4-(((6-(2-chloro-3-(3-chloro-2-(4-(((2,2-difluoroethyl)amino)methyl)-3-methoxyphenyl)pyridin-4-yl)phenyl)-2-methoxypyridin-3-yl)methyl)amino)piperidin-1-yl)ethan-1-one;
N-((6-(2-chloro-3-(3-chloro-2-(4-((isopropylamino)methyl)-3-methoxyphenyl)pyridin-4-yl)phenyl)-2-methoxypyridin-3-yl)methyl)propan-2-amine;
2-(4-(3-chloro-4-(2-chloro-3-(5-((((1r,4r)-4-hydroxycyclohexyl)amino)methyl)-6-methoxypyridin-2-yl)phenyl)pyridin-2-yl)-2-methoxybenzyl)-2,6-diazaspiro[3.4]octan-7-one;
2-(4-(3-chloro-4-(2-chloro-3-(5-((((1s,4r)-4-hydroxycyclohexyl)amino)methyl)-6-methoxypyridin-2-yl)phenyl)pyridin-2-yl)-2-methoxybenzyl)-2,6-diazaspiro[3.4]octan-7-one;
2-(4-(3-chloro-4-(2-chloro-3-(5-((((1r,4s)-4-hydroxycyclohexyl)amino)methyl)-6-methoxypyridin-2-yl)phenyl)pyridin-2-yl)-2-methoxybenzyl)-2,6-diazaspiro[3.4]octan-7-one;
2-(4-(3-chloro-4-(2-chloro-3-(5-((((1s,4s)-4-hydroxycyclohexyl)amino)methyl)-6-methoxypyridin-2-yl)phenyl)pyridin-2-yl)-2-methoxybenzyl)-2,6-diazaspiro[3.4]octan-7-one;
2-(4-(3-chloro-4-(2-chloro-3-(5-((((1s,4s)-4-hydroxycyclohexyl)amino)methyl)-6-methoxypyridin-2-yl)phenyl)pyridin-2-yl)-2-methoxybenzyl)-2,6-diazaspiro[3.4]octan-7-one;
2-(4-(3-chloro-4-(2-chloro-3-(5-((((1s,4r)-4-hydroxycyclohexyl)amino)methyl)-6-methoxypyridin-2-yl)phenyl)pyridin-2-yl)-2-methoxybenzyl)-2,6-diazaspiro[3.4]octan-7-one;
2-(4-(3-chloro-4-(2-chloro-3-(5-((((1r,4s)-4-hydroxycyclohexyl)amino)methyl)-6-methoxypyridin-2-yl)phenyl)pyridin-2-yl)-2-methoxybenzyl)-2,6-diazaspiro[3.4]octan-7-one;
2-(4-(3-chloro-4-(2-chloro-3-(5-((((1r,4r)-4-hydroxycyclohexyl)amino)methyl)-6-methoxypyridin-2-yl)phenyl)pyridin-2-yl)-2-methoxybenzyl)-2,6-diazaspiro[3.4]octan-7-one;
2-(4-(3-chloro-4-(2-chloro-3-(5-((((1r,3r)-3-hydroxy-3-methylcyclobutyl)amino)methyl)-6-methoxypyridin-2-yl)phenyl)pyridin-2-yl)-2-methoxybenzyl)-2,6-diazaspiro[3.4]octan-7-one;
2-(4-(3-chloro-4-(2-chloro-3-(5-((((1s,3r)-3-hydroxy-3-methylcyclobutyl)amino)methyl)-6-methoxypyridin-2-yl)phenyl)pyridin-2-yl)-2-methoxybenzyl)-2,6-diazaspiro[3.4]octan-7-one;
2-(4-(3-chloro-4-(2-chloro-3-(5-((((1r,3s)-3-hydroxy-3-methylcyclobutyl)amino)methyl)-6-methoxypyridin-2-yl)phenyl)pyridin-2-yl)-2-methoxybenzyl)-2,6-diazaspiro[3.4]octan-7-one;
2-(4-(3-chloro-4-(2-chloro-3-(5-((((1s,3s)-3-hydroxy-3-methylcyclobutyl)amino)methyl)-6-methoxypyridin-2-yl)phenyl)pyridin-2-yl)-2-methoxybenzyl)-2,6-diazaspiro[3.4]octan-7-one;
2-(4-(3-chloro-4-(2-chloro-3-(5-((3-hydroxy-3-methylazetidin-1-yl)methyl)-6-methoxypyridin-2-yl)phenyl)pyridin-2-yl)-2-methoxybenzyl)-2,6-diazaspiro[3.4]octan-7-one;
2-(4-(4-(3-(5-((6-acetyl-2,6-diazaspiro[3.3]heptan-2-yl)methyl)-6-methoxypyridin-2-yl)-2-chlorophenyl)-3-chloropyridin-2-yl)-2-methoxybenzyl)-2,6-diazaspiro[3.4]octan-7-one;
2-(4-(4-(3-(5-((2,6-diazaspiro[3.3]heptan-2-yl)methyl)-6-methoxypyridin-2-yl)-2-chlorophenyl)-3-chloropyridin-2-yl)-2-methoxybenzyl)-2,6-diazaspiro[3.4]octan-7-one;
2-(4-(3-chloro-4-(2-chloro-3-(5-((6-hydroxy-2-azaspiro[3.3]heptan-2-yl)methyl)-6-methoxypyridin-2-yl)phenyl)pyridin-2-yl)-2-methoxybenzyl)-2,6-diazaspiro[3.4]octan-7-one;
1-(4-(((6-(3-(6-(4-(((1-acetylpiperidin-4-yl)amino)methyl)-3-methoxyphenyl)-5-methylpyrimidin-4-yl)-2-chlorophenyl)-2-methoxypyridin-3-yl)methyl)amino)piperidin-1-yl)ethan-1-one;
(S)-5-((((6-(2-chloro-3-(6-(3-methoxy-4-(((((S)-5-oxopyrrolidin-2-yl)methyl)amino)methyl)phenyl)-5-methylpyrimidin-4-yl)phenyl)-2-methoxypyridin-3-yl)methyl)amino)methyl)pyrrolidin-2-one;
(S)-5-((((6-(2-chloro-3-(6-(3-methoxy-4-(((((R)-5-oxopyrrolidin-2-yl)methyl)amino)methyl)phenyl)-5-methylpyrimidin-4-yl)phenyl)-2-methoxypyridin-3-yl)methyl)amino)methyl)pyrrolidin-2-one;
(R)-5-((((6-(2-chloro-3-(6-(3-methoxy-4-(((((S)-5-oxopyrrolidin-2-yl)methyl)amino)methyl)phenyl)-5-methylpyrimidin-4-yl)phenyl)-2-methoxypyridin-3-yl)methyl)amino)methyl)pyrrolidin-2-one;
(R)-5-((((6-(2-chloro-3-(6-(3-methoxy-4-(((((R)-5-oxopyrrolidin-2-yl)methyl)amino)methyl)phenyl)-5-methylpyrimidin-4-yl)phenyl)-2-methoxypyridin-3-yl)methyl)amino)methyl)pyrrolidin-2-one;
N-((6-(2-chloro-3-(3-chloro-2-(4-((cyclohexylamino)methyl)-3-methoxyphenyl)pyridin-4-yl)phenyl)-2-methoxypyridin-3-yl)methyl)cyclohexanamine;
2-((6-(3-(2-(4-(((1-acetylpiperidin-4-yl)amino)methyl)-3-methoxyphenyl)-3-chloropyridin-4-yl)-2-chlorophenyl)-2-methoxypyridin-3-yl)methyl)-2,6-diazaspiro[3.4]octan-5-one;
2-((6-(3-(2-(4-(((1-acetylpiperidin-4-yl)amino)methyl)-3-methoxyphenyl)-3-chloropyridin-4-yl)-2-chlorophenyl)-2-methoxypyridin-3-yl)methyl)-2,5,7-triazaspiro[3.4]octan-6-one;
(S)-5-((((2'-((S)-1-((5-(((1-acetylpiperidin-4-yl)amino)methyl)-6-methoxy-3-(trifluoromethyl)pyridin-2-yl)oxy)-2,3-dihydro-1H-inden-4-yl)-3'-chloro-6-methoxy-[2,4'-bipyridin]-5-yl)methyl)amino)methyl)pyrrolidin-2-one;
(S)-5-((((2'-((R)-1-((5-(((1-acetylpiperidin-4-yl)amino)methyl)-6-methoxy-3-(trifluoromethyl)pyridin-2-yl)oxy)-2,3-dihydro-1H-inden-4-yl)-3'-chloro-6-methoxy-[2,4'-bipyridin]-5-yl)methyl)amino)methyl)pyrrolidin-2-one;
(R)-5-((((2'-((S)-1-((5-(((1-acetylpiperidin-4-yl)amino)methyl)-6-methoxy-3-(trifluoromethyl)pyridin-2-yl)oxy)-2,3-dihydro-1H-inden-4-yl)-3'-chloro-6-methoxy-[2,4'-bipyridin]-5-yl)methyl)amino)methyl)pyrrolidin-2-one;
(R)-5-((((2'-((R)-1-((5-(((1-acetylpiperidin-4-yl)amino)methyl)-6-methoxy-3-(trifluoromethyl)pyridin-2-yl)oxy)-2,3-dihydro-1H-inden-4-yl)-3'-chloro-6-methoxy-[2,4'-bipyridin]-5-yl)methyl)amino)methyl)pyrrolidin-2-one;
1-(4-(((6-(3-(2-(4-(((1-acetylpiperidin-4-yl)amino)methyl)-3-methoxyphenyl)-3-chloropyridin-4-yl)-2-(trifluoromethyl)phenyl)-2-methoxypyridin-3-yl)methyl)amino)piperidin-1-yl)ethan-1-one;
N-((6-(3-(3-chloro-2-(3-methoxy-4-(((tetrahydro-2H-pyran-4-yl)amino)methyl)phenyl)pyridin-4-yl)-2-(trifluoromethyl)phenyl)-2-methoxypyridin-3-yl)methyl)tetrahydro-2H-pyran-4-amine;
N-((6-(3-(2-(4-((7-oxa-2-azaspiro[3.5]nonan-2-yl)methyl)-3-methoxyphenyl)-3-chloropyridin-4-yl)-2-chlorophenyl)-2-methoxypyridin-3-yl)methyl)tetrahydro-2H-pyran-4-amine;
N-(4-(4-(3-(5-((7-oxa-2-azaspiro[3.5]nonan-2-yl)methyl)-6-methoxypyridin-2-yl)-2-chlorophenyl)-3-chloropyridin-2-yl)-2-methoxybenzyl)tetrahydro-2H-pyran-4-amine;
(S)-5-((((6-(3-chloro-2-((S)-1-((6-methoxy-5-(((((S)-5-oxopyrrolidin-2-yl)methyl)amino)methyl)-3-(trifluoromethyl)pyrazin-2-yl)amino)-2,3-dihydro-1H-inden-4-yl)pyridin-4-yl)-1-methyl-1H-pyrrolo[2,3-b]pyridin-3-yl)methyl)amino)methyl)pyrrolidin-2-one;
(R)-5-((((6-(3-chloro-2-((R)-1-((6-methoxy-5-(((((R)-5-oxopyrrolidin-2-yl)methyl)amino)methyl)-3-(trifluoromethyl)pyrazin-2-yl)amino)-2,3-dihydro-1H-inden-4-yl)pyridin-4-yl)-1-methyl-1H-pyrrolo[2,3-b]pyridin-3-yl)methyl)amino)methyl)pyrrolidin-2-one;
(R)-5-((((6-(3-chloro-2-((R)-1-((6-methoxy-5-(((((S)-5-oxopyrrolidin-2-yl)methyl)amino)methyl)-3-(trifluoromethyl)pyrazin-2-yl)amino)-2,3-dihydro-1H-inden-4-yl)pyridin-4-yl)-1-methyl-1H-pyrrolo[2,3-b]pyridin-3-yl)methyl)amino)methyl)pyrrolidin-2-one;
(R)-5-((((6-(3-chloro-2-((S)-1-((6-methoxy-5-(((((R)-5-oxopyrrolidin-2-yl)methyl)amino)methyl)-3-(trifluoromethyl)pyrazin-2-yl)amino)-2,3-dihydro-1H-inden-4-yl)pyridin-4-yl)-1-methyl-1H-pyrrolo[2,3-b]pyridin-3-yl)methyl)amino)methyl)pyrrolidin-2-one;
(R)-5-((((6-(3-chloro-2-((S)-1-((6-methoxy-5-(((((S)-5-oxopyrrolidin-2-yl)methyl)amino)methyl)-3-(trifluoromethyl)pyrazin-2-yl)amino)-2,3-dihydro-1H-inden-4-yl)pyridin-4-yl)-1-methyl-1H-pyrrolo[2,3-b]pyridin-3-yl)methyl)amino)methyl)pyrrolidin-2-one;
(S)-5-((((6-(3-chloro-2-((R)-1-((6-methoxy-5-(((((R)-5-oxopyrrolidin-2-yl)methyl)amino)methyl)-3-(trifluoromethyl)pyrazin-2-yl)amino)-2,3-dihydro-1H-inden-4-yl)pyridin-4-yl)-1-methyl-1H-pyrrolo[2,3-b]pyridin-3-yl)methyl)amino)methyl)pyrrolidin-2-one;
(S)-5-((((6-(3-chloro-2-((R)-1-((6-methoxy-5-(((((S)-5-oxopyrrolidin-2-yl)methyl)amino)methyl)-3-(trifluoromethyl)pyrazin-2-yl)amino)-2,3-dihydro-1H-inden-4-yl)pyridin-4-yl)-1-methyl-1H-pyrrolo[2,3-b]pyridin-3-yl)methyl)amino)methyl)pyrrolidin-2-one;
(S)-5-((((6-(3-chloro-2-((S)-1-((6-methoxy-5-(((((R)-5-oxopyrrolidin-2-yl)methyl)amino)methyl)-3-(trifluoromethyl)pyrazin-2-yl)amino)-2,3-dihydro-1H-inden-4-yl)pyridin-4-yl)-1-methyl-1H-pyrrolo[2,3-b]pyridin-3-yl)methyl)amino)methyl)pyrrolidin-2-one;
1-(4-(((6-(3-(2-(4-(((1-acetylpiperidin-4-yl)amino)methyl)-3-methoxyphenyl)-3-chloropyridin-4-yl)-2-chlorophenyl)-2-methoxypyridin-3-yl)methyl)amino)piperidin-1-yl)-2-hydroxyethan-1-one;
N-((6-(2-chloro-3-(3-chloro-2-(4-(((3-fluoropropyl)amino)methyl)-3-methoxyphenyl)pyridin-4-yl)phenyl)-2-methoxypyridin-3-yl)methyl)-3-fluoropropan-1-amine;
2-(4-(6-(2-chloro-3-(6-methoxy-5-((7-oxo-2,6-diazaspiro[3.4]octan-2-yl)methyl)pyridin-2-yl)phenyl)-5-methylpyrimidin-4-yl)-2-methoxybenzyl)-2,6-diazaspiro[3.4]octan-7-one;
1-(1-((6-(3-(2-(4-((4-acetylpiperidin-1-yl)methyl)-3-methoxyphenyl)-3-chloropyridin-4-yl)-2-chlorophenyl)-2-methoxypyridin-3-yl)methyl)piperidin-4-yl)ethan-1-one;
1-(4-(((6-(2-chloro-3-(3-chloro-2-(4-(((3-fluoropropyl)amino)methyl)-3-methoxyphenyl)pyridin-4-yl)phenyl)-2-methoxypyridin-3-yl)methyl)amino)piperidin-1-yl)ethan-1-one;
1-(6-((6-(3-(6-(4-((6-acetyl-2,6-diazaspiro[3.3]heptan-2-yl)methyl)-3-methoxyphenyl)-5-methylpyrimidin-4-yl)-2-chlorophenyl)-2-methoxypyridin-3-yl)methyl)-2,6-diazaspiro[3.3]heptan-2-yl)ethan-1-one;
1-(4-(((6-(2-chloro-3-(3-chloro-2-(4-((((1r,4r)-4-hydroxycyclohexyl)amino)methyl)-3-methoxyphenyl)pyridin-4-yl)phenyl)-2-methoxypyridin-3-yl)methyl)amino)piperidin-1-yl)-2-hydroxyethan-1-one;
1-(4-(((6-(2-chloro-3-(3-chloro-2-(4-((((1s,4r)-4-hydroxycyclohexyl)amino)methyl)-3-methoxyphenyl)pyridin-4-yl)phenyl)-2-methoxypyridin-3-yl)methyl)amino)piperidin-1-yl)-2-hydroxyethan-1-one;
1-(4-(((6-(2-chloro-3-(3-chloro-2-(4-((((1r,4s)-4-hydroxycyclohexyl)amino)methyl)-3-methoxyphenyl)pyridin-4-yl)phenyl)-2-methoxypyridin-3-yl)methyl)amino)piperidin-1-yl)-2-hydroxyethan-1-one;
1-(4-(((6-(2-chloro-3-(3-chloro-2-(4-((((1s,4s)-4-hydroxycyclohexyl)amino)methyl)-3-methoxyphenyl)pyridin-4-yl)phenyl)-2-methoxypyridin-3-yl)methyl)amino)piperidin-1-yl)-2-hydroxyethan-1-one;
1-(4-(((6-(3-(6-(4-(((1-acetylpiperidin-4-yl)amino)methyl)-3-methoxyphenyl)-5-chloropyrimidin-4-yl)-2-chlorophenyl)-2-methoxypyridin-3-yl)methyl)amino)piperidin-1-yl)ethan-1-one;
(R)-5-(((6-(3-(2-(4-(((1-acetylpiperidin-4-yl)amino)methyl)-3-methoxyphenyl)-3-chloropyridin-4-yl)-2-chlorophenyl)-2-methoxypyridin-3-yl)methyl)amino)piperidin-2-one;
(S)-5-(((6-(3-(2-(4-(((1-acetylpiperidin-4-yl)amino)methyl)-3-methoxyphenyl)-3-chloropyridin-4-yl)-2-chlorophenyl)-2-methoxypyridin-3-yl)methyl)amino)piperidin-2-one;
(S)-5-((((6-(2-chloro-3-(5-chloro-6-(3-methoxy-4-(((((S)-5-oxopyrrolidin-2-yl)methyl)amino)methyl)phenyl)pyrimidin-4-yl)phenyl)-2-methoxypyridin-3-yl)methyl)amino)methyl)pyrrolidin-2-one;
(S)-5-((((6-(2-chloro-3-(5-chloro-6-(3-methoxy-4-(((((R)-5-oxopyrrolidin-2-yl)methyl)amino)methyl)phenyl)pyrimidin-4-yl)phenyl)-2-methoxypyridin-3-yl)methyl)amino)methyl)pyrrolidin-2-one;
(R)-5-((((6-(2-chloro-3-(5-chloro-6-(3-methoxy-4-(((((S)-5-oxopyrrolidin-2-yl)methyl)amino)methyl)phenyl)pyrimidin-4-yl)phenyl)-2-methoxypyridin-3-yl)methyl)amino)methyl)pyrrolidin-2-one;
(R)-5-((((6-(2-chloro-3-(5-chloro-6-(3-methoxy-4-(((((R)-5-oxopyrrolidin-2-yl)methyl)amino)methyl)phenyl)pyrimidin-4-yl)phenyl)-2-methoxypyridin-3-yl)methyl)amino)methyl)pyrrolidin-2-one;
(S)-5-((((6-(((S)-4-(5-(((1-acetylpiperidin-4-yl)amino)methyl)-3'-chloro-6-methoxy-[2,4'-bipyridin]-2'-yl)-2,3-dihydro-1H-inden- 1-yl)oxy)-2-methoxy-5-(trifluoromethyl)pyridin-3-yl)methyl)amino)methyl)pyrrolidin-2-one;
(S)-5-((((6-(((R)-4-(5-(((1-acetylpiperidin-4-yl)amino)methyl)-3'-chloro-6-methoxy-[2,4'-bipyridin]-2'-yl)-2,3-dihydro-1H-inden- 1-yl)oxy)-2-methoxy-5-(trifluoromethyl)pyridin-3-yl)methyl)amino)methyl)pyrrolidin-2-one;
(R)-5-((((6-(((S)-4-(5-(((1-acetylpiperidin-4-yl)amino)methyl)-3'-chloro-6-methoxy-[2,4'-bipyridin]-2'-yl)-2,3-dihydro-1H-inden- 1-yl)oxy)-2-methoxy-5-(trifluoromethyl)pyridin-3-yl)methyl)amino)methyl)pyrrolidin-2-one;
(R)-5-((((6-(((R)-4-(5-(((1-acetylpiperidin-4-yl)amino)methyl)-3'-chloro-6-methoxy-[2,4'-bipyridin]-2'-yl)-2,3-dihydro-1H-inden- 1-yl)oxy)-2-methoxy-5-(trifluoromethyl)pyridin-3-yl)methyl)amino)methyl)pyrrolidin-2-one;
(S)-5-((((6-(3-chloro-2-((S)-1-((6-methoxy-5-(((((S)-5-oxopyrrolidin-2-yl)methyl)amino)methyl)-3-(trifluoromethyl)pyridin-2-yl)oxy)-2,3-dihydro-1H-inden-4-yl)pyridin-4-yl)-1-methyl-1H-pyrrolo[2,3-b]pyridin-3-yl)methyl)amino)methyl)pyrrolidin-2-one;
(R)-5-((((6-(3-chloro-2-((R)-1-((6-methoxy-5-(((((R)-5-oxopyrrolidin-2-yl)methyl)amino)methyl)-3-(trifluoromethyl)pyridin-2-yl)oxy)-2,3-dihydro-1H-inden-4-yl)pyridin-4-yl)-1-methyl-1H-pyrrolo[2,3-b]pyridin-3-yl)methyl)amino)methyl)pyrrolidin-2-one;
(R)-5-((((6-(3-chloro-2-((R)-1-((6-methoxy-5-(((((S)-5-oxopyrrolidin-2-yl)methyl)amino)methyl)-3-(trifluoromethyl)pyridin-2-yl)oxy)-2,3-dihydro-1H-inden-4-yl)pyridin-4-yl)-1-methyl-1H-pyrrolo[2,3-b]pyridin-3-yl)methyl)amino)methyl)pyrrolidin-2-one;
(R)-5-((((6-(3-chloro-2-((S)-1-((6-methoxy-5-(((((R)-5-oxopyrrolidin-2-yl)methyl)amino)methyl)-3-(trifluoromethyl)pyridin-2-yl)oxy)-2,3-dihydro-1H-inden-4-yl)pyridin-4-yl)-1-methyl-1H-pyrrolo[2,3-b]pyridin-3-yl)methyl)amino)methyl)pyrrolidin-2-one;
(R)-5-((((6-(3-chloro-2-((S)-1-((6-methoxy-5-(((((S)-5-oxopyrrolidin-2-yl)methyl)amino)methyl)-3-(trifluoromethyl)pyridin-2-yl)oxy)-2,3-dihydro-1H-inden-4-yl)pyridin-4-yl)-1-methyl-1H-pyrrolo[2,3-b]pyridin-3-yl)methyl)amino)methyl)pyrrolidin-2-one;
(S)-5-((((6-(3-chloro-2-((R)-1-((6-methoxy-5-(((((R)-5-oxopyrrolidin-2-yl)methyl)amino)methyl)-3-(trifluoromethyl)pyridin-2-yl)oxy)-2,3-dihydro-1H-inden-4-yl)pyridin-4-yl)-1-methyl-1H-pyrrolo[2,3-b]pyridin-3-yl)methyl)amino)methyl)pyrrolidin-2-one;
(S)-5-((((6-(3-chloro-2-((R)-1-((6-methoxy-5-(((((S)-5-oxopyrrolidin-2-yl)methyl)amino)methyl)-3-(trifluoromethyl)pyridin-2-yl)oxy)-2,3-dihydro-1H-inden-4-yl)pyridin-4-yl)-1-methyl-1H-pyrrolo[2,3-b]pyridin-3-yl)methyl)amino)methyl)pyrrolidin-2-one;
(S)-5-((((6-(3-chloro-2-((S)-1-((6-methoxy-5-(((((R)-5-oxopyrrolidin-2-yl)methyl)amino)methyl)-3-(trifluoromethyl)pyridin-2-yl)oxy)-2,3-dihydro-1H-inden-4-yl)pyridin-4-yl)-1-methyl-1H-pyrrolo[2,3-b]pyridin-3-yl)methyl)amino)methyl)pyrrolidin-2-one;
1-(4-(((6-(2-chloro-3-(3-chloro-2-(3-methoxy-4-(((1-(3-methoxypropanoyl)piperidin-4-yl)amino)methyl)phenyl)pyridin-4-yl)phenyl)-2-methoxypyridin-3-yl)methyl)amino)piperidin-1-yl)-3-methoxypropan-1-one;
2-((6-(3-(6'-(((1-acetylpiperidin-4-yl)amino)methyl)-3-chloro-5'-methoxy-[2,3'-bipyridin]-4-yl)-2-chlorophenyl)-2-methoxypyridin-3-yl)methyl)-2,6-diazaspiro[3.4]octan-7-one;
2-((6-(2-chloro-3-(3-chloro-5'-methoxy-6'-(((tetrahydro-2H-pyran-4-yl)amino)methyl)-[2,3'-bipyridin]-4-yl)phenyl)-2-methoxypyridin-3-yl)methyl)-2,6-diazaspiro[3.4]octan-7-one;
2-((6-(2-chloro-3-(3-chloro-6'-((((1r,4r)-4-hydroxycyclohexyl)amino)methyl)-5'-methoxy-[2,3'-bipyridin]-4-yl)phenyl)-2-methoxypyridin-3-yl)methyl)-2,6-diazaspiro[3.4]octan-7-one;
2-((6-(2-chloro-3-(3-chloro-6'-((((1s,4r)-4-hydroxycyclohexyl)amino)methyl)-5'-methoxy-[2,3'-bipyridin]-4-yl)phenyl)-2-methoxypyridin-3-yl)methyl)-2,6-diazaspiro[3.4]octan-7-one;
2-((6-(2-chloro-3-(3-chloro-6'-((((1r,4s)-4-hydroxycyclohexyl)amino)methyl)-5'-methoxy-[2,3'-bipyridin]-4-yl)phenyl)-2-methoxypyridin-3-yl)methyl)-2,6-diazaspiro[3.4]octan-7-one;
2-((6-(2-chloro-3-(3-chloro-6'-((((1s,4s)-4-hydroxycyclohexyl)amino)methyl)-5'-methoxy-[2,3'-bipyridin]-4-yl)phenyl)-2-methoxypyridin-3-yl)methyl)-2,6-diazaspiro[3.4]octan-7-one;
(S)-2-((6-(2-chloro-3-(3-chloro-5'-methoxy-6'-((((5-oxopyrrolidin-2-yl)methyl)amino)methyl)-[2,3'-bipyridin]-4-yl)phenyl)-2-methoxypyridin-3-yl)methyl)-2,6-diazaspiro[3.4]octan-7-one;
(R)-2-((6-(2-chloro-3-(3-chloro-5'-methoxy-6'-((((5-oxopyrrolidin-2-yl)methyl)amino)methyl)-[2,3'-bipyridin]-4-yl)phenyl)-2-methoxypyridin-3-yl)methyl)-2,6-diazaspiro[3.4]octan-7-one;
(S)-2-((2'-(1-((5-(((1-acetylpiperidin-4-yl)amino)methyl)-6-methoxy-3-(trifluoromethyl)pyridin-2-yl)oxy)-2,3-dihydro-1H-inden-4-yl)-3'-chloro-6-methoxy-[2,4'-bipyridin]-5-yl)methyl)-2,6-diazaspiro[3.4]octan-7-one;
(R)-2-((2'-(1-((5-(((1-acetylpiperidin-4-yl)amino)methyl)-6-methoxy-3-(trifluoromethyl)pyridin-2-yl)oxy)-2,3-dihydro-1H-inden-4-yl)-3'-chloro-6-methoxy-[2,4'-bipyridin]-5-yl)methyl)-2,6-diazaspiro[3.4]octan-7-one;
(S)-2-((3'-chloro-6-methoxy-2'-(1-((6-methoxy-5-(((tetrahydro-2H-pyran-4-yl)amino)methyl)-3-(trifluoromethyl)pyridin-2-yl)oxy)-2,3-dihydro-1H-inden-4-yl)-[2,4'-bipyridin]-5-yl)methyl)-2,6-diazaspiro[3.4]octan-7-one;
(R)-2-((3'-chloro-6-methoxy-2'-(1-((6-methoxy-5-(((tetrahydro-2H-pyran-4-yl)amino)methyl)-3-(trifluoromethyl)pyridin-2-yl)oxy)-2,3-dihydro-1H-inden-4-yl)-[2,4'-bipyridin]-5-yl)methyl)-2,6-diazaspiro[3.4]octan-7-one;
2-((3'-chloro-2'-((S)-1-((5-((((S)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)-2,6-diazaspiro[3.4]octan-7-one;
2-((3'-chloro-2'-((S)-1-((5-((((R)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)-2,6-diazaspiro[3.4]octan-7-one;
2-((3'-chloro-2'-((R)-1-((5-((((S)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)-2,6-diazaspiro[3.4]octan-7-one;
2-((3'-chloro-2'-((R)-1-((5-((((R)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)-2,6-diazaspiro[3.4]octan-7-one;
2-(4-(5-chloro-6-(2-chloro-3-(6-methoxy-5-((7-oxo-2,6-diazaspiro[3.4]octan-2-yl)methyl)pyridin-2-yl)phenyl)pyrimidin-4-yl)-2-methoxybenzyl)-2,6-diazaspiro[3.4]octan-7-one;
(S)-6-(3-chloro-4-(2-chloro-3-(6-methoxy-5-((((5-oxopyrrolidin-2-yl)methyl)amino)methyl)pyridin-2-yl)phenyl)pyridin-2-yl)-2-methoxy-3-(2-(methylamino)ethyl)pyrrolo[2,1-f][1,2,4]triazin-4(3H)-one;
(R)-6-(3-chloro-4-(2-chloro-3-(6-methoxy-5-((((5-oxopyrrolidin-2-yl)methyl)amino)methyl)pyridin-2-yl)phenyl)pyridin-2-yl)-2-methoxy-3-(2-(methylamino)ethyl)pyrrolo[2,1-f][1,2,4]triazin-4(3H)-one;
1-(4-((4-(3-chloro-4-(2-chloro-3-(1-methyl-3-(((tetrahydro-2H-pyran-4-yl)amino)methyl)-1H-pyrrolo[2,3-b]pyridin-6-yl)phenyl)pyridin-2-yl)-2-methoxybenzyl)amino)piperidin-1-yl)ethan-1-one;
1-(4-((4-(3-chloro-4-(2-chloro-3-(1-methyl-3-((((tetrahydro-2H-pyran-4-yl)methyl)amino)methyl)-1H-pyrrolo[2,3-b]pyridin-6-yl)phenyl)pyridin-2-yl)-2-methoxybenzyl)amino)piperidin-1-yl)ethan-1-one;
1-(4-((4-(3-chloro-4-(2-chloro-3-(3-(((1,1-dioxidotetrahydro-2H-thiopyran-4-yl)amino)methyl)-1-methyl-1H-pyrrolo[2,3-b]pyridin-6-yl)phenyl)pyridin-2-yl)-2-methoxybenzyl)amino)piperidin-1-yl)ethan-1-one;
1-(4-((4-(3-chloro-4-(2-chloro-3-(3-(((2-hydroxyethyl)amino)methyl)-1-methyl-1H-pyrrolo[2,3-b]pyridin-6-yl)phenyl)pyridin-2-yl)-2-methoxybenzyl)amino)piperidin-1-yl)ethan-1-one;
1-(4-(((6-(2-chloro-3-(3-chloro-2-(4-(((2-hydroxyethyl)amino)methyl)-3-methoxyphenyl)pyridin-4-yl)phenyl)-1-methyl-1H-pyrrolo[2,3-b]pyridin-3-yl)methyl)amino)piperidin-1-yl)ethan-1-one;
(S)-5-((((6-(2-chloro-3-(3-chloro-2-(4-methyl-1,1-dioxido-2,3,4,5-tetrahydrobenzo[f][1,4]thiazepin-8-yl)pyridin-4-yl)phenyl)-2-methoxypyridin-3-yl)methyl)amino)methyl)pyrrolidin-2-one;
(R)-5-((((6-(2-chloro-3-(3-chloro-2-(4-methyl-1,1-dioxido-2,3,4,5-tetrahydrobenzo[f][1,4]thiazepin-8-yl)pyridin-4-yl)phenyl)-2-methoxypyridin-3-yl)methyl)amino)methyl)pyrrolidin-2-one;
1-(4-((6-(3-(6-(4-((4-acetylpiperazin-1-yl)methyl)-3-methoxyphenyl)-5-chloropyrimidin-4-yl)-2-chlorophenyl)-2-methoxypyridin-3-yl)methyl)piperazin-1-yl)ethan-1-one;
N-((6-(2-chloro-3-(3-chloro-2-(3-methoxy-4-(((tetrahydro-2H-pyran-4-yl)amino)methyl)phenyl)pyridin-4-yl)phenyl)-1-methyl-1H-pyrrolo[2,3-b]pyridin-3-yl)methyl)tetrahydro-2H-pyran-4-amine;
1-(4-(((6-(3-(2-(4-((6-acetyl-2,6-diazaspiro[3.3]heptan-2-yl)methyl)-3-methoxyphenyl)-3-chloropyridin-4-yl)-2-chlorophenyl)-1-methyl-1H-pyrrolo[2,3-b]pyridin-3-yl)methyl)amino)piperidin-1-yl)ethan-1-one;
1-(4-(((5-(3-(2-(4-(((1-acetylpiperidin-4-yl)amino)methyl)-3-methoxyphenyl)-3-chloropyridin-4-yl)-2-chlorophenyl)-3-methoxypyridin-2-yl)methyl)amino)piperidin-1-yl)ethan-1-one;
2-(4-(3-chloro-4-(2-chloro-3-(5-methoxy-6-((7-oxo-2,6-diazaspiro[3.4]octan-2-yl)methyl)pyridin-3-yl)phenyl)pyridin-2-yl)-2-methoxybenzyl)-2,6-diazaspiro[3.4]octan-7-one;
1-(4-(((5-(3-(6'-(((1-acetylpiperidin-4-yl)amino)methyl)-3-chloro-5'-methoxy-[2,3'-bipyridin]-4-yl)-2-chlorophenyl)-3-methoxypyridin-2-yl)methyl)amino)piperidin-1-yl)ethan-1-one;
2-(4-(5-chloro-6-(2-chloro-3-(5-((6-hydroxy-2-azaspiro[3.3]heptan-2-yl)methyl)-6-methoxypyridin-2-yl)phenyl)pyrimidin-4-yl)-2-methoxybenzyl)-2-azaspiro[3.3]heptan-6-ol;
2-((5-(2-chloro-3-(3-chloro-5'-methoxy-6'-((7-oxo-2,6-diazaspiro[3.4]octan-2-yl)methyl)-[2,3'-bipyridin]-4-yl)phenyl)-3-methoxypyridin-2-yl)methyl)-2,6-diazaspiro[3.4]octan-7-one;
N-((5-(2-chloro-3-(3-chloro-5'-methoxy-6'-(((tetrahydro-2H-pyran-4-yl)amino)methyl)-[2,3'-bipyridin]-4-yl)phenyl)-3-methoxypyridin-2-yl)methyl)tetrahydro-2H-pyran-4-amine;
2-(4-(3-chloro-4-(2-chloro-3-(6-methoxy-5-((6-methyl-7-oxo-2,6-diazaspiro[3.4]octan-2-yl)methyl)pyridin-2-yl)phenyl)pyridin-2-yl)-2-methoxybenzyl)-6-methyl-2,6-diazaspiro[3.4]octan-7-one;
1-(4-(((6-(3-(5-(((1-acetylpiperidin-4-yl)amino)methyl)-3'-chloro-6-methoxy-[2,4'-bipyridin]-2'-yl)-2-chlorophenyl)-2-methoxypyridin-3-yl)methyl)amino)piperidin-1-yl)ethan-1-one;
2-((6-(2-chloro-3-(3'-chloro-6-methoxy-5-((7-oxo-2,6-diazaspiro[3.4]octan-2-yl)methyl)-[2,4'-bipyridin]-2'-yl)phenyl)-2-methoxypyridin-3-yl)methyl)-2,6-diazaspiro[3.4]octan-7-one;
N-((3'-chloro-2'-(2-chloro-3-(6-methoxy-5-(((tetrahydro-2H-pyran-4-yl)amino)methyl)pyridin-2-yl)phenyl)-6-methoxy-[2,4'-bipyridin]-5-yl)methyl)tetrahydro-2H-pyran-4-amine;
(S)-5-((((3'-chloro-2'-(2-chloro-3-(6-methoxy-5-(((((S)-5-oxopyrrolidin-2-yl)methyl)amino)methyl)pyridin-2-yl)phenyl)-6-methoxy-[2,4'-bipyridin]-5-yl)methyl)amino)methyl)pyrrolidin-2-one;
(S)-5-((((3'-chloro-2'-(2-chloro-3-(6-methoxy-5-(((((R)-5-oxopyrrolidin-2-yl)methyl)amino)methyl)pyridin-2-yl)phenyl)-6-methoxy-[2,4'-bipyridin]-5-yl)methyl)amino)methyl)pyrrolidin-2-one;
(R)-5-((((3'-chloro-2'-(2-chloro-3-(6-methoxy-5-(((((S)-5-oxopyrrolidin-2-yl)methyl)amino)methyl)pyridin-2-yl)phenyl)-6-methoxy-[2,4'-bipyridin]-5-yl)methyl)amino)methyl)pyrrolidin-2-one;
(R)-5-((((3'-chloro-2'-(2-chloro-3-(6-methoxy-5-(((((R)-5-oxopyrrolidin-2-yl)methyl)amino)methyl)pyridin-2-yl)phenyl)-6-methoxy-[2,4'-bipyridin]-5-yl)methyl)amino)methyl)pyrrolidin-2-one;
2-(4-(3-chloro-4-(3-(6-methoxy-5-((7-oxo-2,6-diazaspiro[3.4]octan-2-yl)methyl)pyridin-2-yl)-2-(trifluoromethyl)phenyl)pyridin-2-yl)-2-methoxybenzyl)-2,6-diazaspiro[3.4]octan-7-one;
1-(6-((6-(3-(2-(4-((6-acetyl-2,6-diazaspiro[3.3]heptan-2-yl)methyl)-3-methoxyphenyl)-3-chloropyridin-4-yl)-2-(trifluoromethyl)phenyl)-2-methoxypyridin-3-yl)methyl)-2,6-diazaspiro[3.3]heptan-2-yl)ethan-1-one;
6-(4-(4-(3-(5-((2-oxa-6-azaspiro[3.3]heptan-6-yl)methyl)-6-methoxypyridin-2-yl)-2-(trifluoromethyl)phenyl)-3-chloropyridin-2-yl)-2-methoxybenzyl)-2-oxa-6-azaspiro[3.3]heptane;
2-(4-(4-(3-(3-(((1-acetylpiperidin-4-yl)amino)methyl)-1-methyl-1H-pyrrolo[2,3-b]pyridin-6-yl)-2-chlorophenyl)-3-chloropyridin-2-yl)-2-methoxybenzyl)-2,6-diazaspiro[3.4]octan-7-one;
(S)-1-(((6-(3-(3-chloro-2-(4-((((S)-2-hydroxypropyl)amino)methyl)-3-methoxyphenyl)pyridin-4-yl)-2-(trifluoromethyl)phenyl)-2-methoxypyridin-3-yl)methyl)amino)propan-2-ol;
(S)-1-(((6-(3-(3-chloro-2-(4-((((R)-2-hydroxypropyl)amino)methyl)-3-methoxyphenyl)pyridin-4-yl)-2-(trifluoromethyl)phenyl)-2-methoxypyridin-3-yl)methyl)amino)propan-2-ol;
(R)-1-(((6-(3-(3-chloro-2-(4-((((S)-2-hydroxypropyl)amino)methyl)-3-methoxyphenyl)pyridin-4-yl)-2-(trifluoromethyl)phenyl)-2-methoxypyridin-3-yl)methyl)amino)propan-2-ol;
(R)-1-(((6-(3-(3-chloro-2-(4-((((R)-2-hydroxypropyl)amino)methyl)-3-methoxyphenyl)pyridin-4-yl)-2-(trifluoromethyl)phenyl)-2-methoxypyridin-3-yl)methyl)amino)propan-2-ol;
2-(((6-(2-chloro-3-(5-chloro-6-(4-(((2-hydroxyethyl)amino)methyl)-3-methoxyphenyl)pyrimidin-4-yl)phenyl)-2-methoxypyridin-3-yl)methyl)amino)ethan-1-ol;
5-((((6-(2-chloro-3-(3-chloro-2-(1,1-dioxido-4-(((S)-5-oxopyrrolidin-2-yl)methyl)-2,3,4,5-tetrahydrobenzo[f][1,4]thiazepin-8-yl)pyridin-4-yl)phenyl)-2-methoxypyridin-3-yl)methyl)amino)methyl)pyrrolidin-2-one;
5-((((6-(2-chloro-3-(3-chloro-2-(1,1-dioxido-4-(((R)-5-oxopyrrolidin-2-yl)methyl)-2,3,4,5-tetrahydrobenzo[f][1,4]thiazepin-8-yl)pyridin-4-yl)phenyl)-2-methoxypyridin-3-yl)methyl)amino)methyl)pyrrolidin-2-one;
(S)-5-((((6-(2-chloro-3-(3-chloro-2-(1,1-dioxido-4-(((S)-5-oxopyrrolidin-2-yl)methyl)-2,3,4,5-tetrahydrobenzo[f][1,4]thiazepin-8-yl)pyridin-4-yl)phenyl)-2-methoxypyridin-3-yl)methyl)amino)methyl)pyrrolidin-2-one;
(S)-5-((((6-(2-chloro-3-(3-chloro-2-(1,1-dioxido-4-(((R)-5-oxopyrrolidin-2-yl)methyl)-2,3,4,5-tetrahydrobenzo[f][1,4]thiazepin-8-yl)pyridin-4-yl)phenyl)-2-methoxypyridin-3-yl)methyl)amino)methyl)pyrrolidin-2-one;
(R)-5-((((6-(2-chloro-3-(3-chloro-2-(1,1-dioxido-4-(((S)-5-oxopyrrolidin-2-yl)methyl)-2,3,4,5-tetrahydrobenzo[f][1,4]thiazepin-8-yl)pyridin-4-yl)phenyl)-2-methoxypyridin-3-yl)methyl)amino)methyl)pyrrolidin-2-one;
(R)-5-((((6-(2-chloro-3-(3-chloro-2-(1,1-dioxido-4-(((R)-5-oxopyrrolidin-2-yl)methyl)-2,3,4,5-tetrahydrobenzo[f][1,4]thiazepin-8-yl)pyridin-4-yl)phenyl)-2-methoxypyridin-3-yl)methyl)amino)methyl)pyrrolidin-2-one;
1-(4-(((6-(2-chloro-3-(3-chloro-2-(8-ethoxy-2-((((1r,4r)-4-hydroxycyclohexyl)amino)methyl)-[1,2,4]triazolo[1,5-a]pyridin-6-yl)pyridin-4-yl)phenyl)-2-methoxypyridin-3-yl)methyl)amino)piperidin-1-yl)ethan-1-one;
1-(4-(((6-(2-chloro-3-(3-chloro-2-(8-ethoxy-2-((((1s,4r)-4-hydroxycyclohexyl)amino)methyl)-[1,2,4]triazolo[1,5-a]pyridin-6-yl)pyridin-4-yl)phenyl)-2-methoxypyridin-3-yl)methyl)amino)piperidin-1-yl)ethan-1-one;
1-(4-(((6-(2-chloro-3-(3-chloro-2-(8-ethoxy-2-((((1r,4s)-4-hydroxycyclohexyl)amino)methyl)-[1,2,4]triazolo[1,5-a]pyridin-6-yl)pyridin-4-yl)phenyl)-2-methoxypyridin-3-yl)methyl)amino)piperidin-1-yl)ethan-1-one;
1-(4-(((6-(2-chloro-3-(3-chloro-2-(8-ethoxy-2-((((1s,4s)-4-hydroxycyclohexyl)amino)methyl)-[1,2,4]triazolo[1,5-a]pyridin-6-yl)pyridin-4-yl)phenyl)-2-methoxypyridin-3-yl)methyl)amino)piperidin-1-yl)ethan-1-one;
1-(4-(((6-(2-chloro-3-(3-chloro-2-(8-ethoxy-2-((((1r,3r)-3-hydroxy-3-methylcyclobutyl)amino)methyl)-[1,2,4]triazolo[1,5-a]pyridin-6-yl)pyridin-4-yl)phenyl)-2-methoxypyridin-3-yl)methyl)amino)piperidin-1-yl)ethan-1-one;
1-(4-(((6-(2-chloro-3-(3-chloro-2-(8-ethoxy-2-((((1s,3r)-3-hydroxy-3-methylcyclobutyl)amino)methyl)-[1,2,4]triazolo[1,5-a]pyridin-6-yl)pyridin-4-yl)phenyl)-2-methoxypyridin-3-yl)methyl)amino)piperidin-1-yl)ethan-1-one;
1-(4-(((6-(2-chloro-3-(3-chloro-2-(8-ethoxy-2-((((1r,3s)-3-hydroxy-3-methylcyclobutyl)amino)methyl)-[1,2,4]triazolo[1,5-a]pyridin-6-yl)pyridin-4-yl)phenyl)-2-methoxypyridin-3-yl)methyl)amino)piperidin-1-yl)ethan-1-one;
1-(4-(((6-(2-chloro-3-(3-chloro-2-(8-ethoxy-2-((((1s,3s)-3-hydroxy-3-methylcyclobutyl)amino)methyl)-[1,2,4]triazolo[1,5-a]pyridin-6-yl)pyridin-4-yl)phenyl)-2-methoxypyridin-3-yl)methyl)amino)piperidin-1-yl)ethan-1-one;
1-(4-(((6-(4-(3-(5-(((1-acetylpiperidin-4-yl)amino)methyl)-6-methoxypyridin-2-yl)-2-chlorophenyl)-3-chloropyridin-2-yl)-8-ethoxy-[1,2,4]triazolo[1,5-a]pyridin-2-yl)methyl)amino)piperidin-1-yl)ethan-1-one;
2-((6-(4-(3-(5-(((1-acetylpiperidin-4-yl)amino)methyl)-6-methoxypyridin-2-yl)-2-chlorophenyl)-3-chloropyridin-2-yl)-8-ethoxy-[1,2,4]triazolo[1,5-a]pyridin-2-yl)methyl)-2,6-diazaspiro[3.4]octan-7-one;
N-((6-(3-(3-chloro-2-(4-(((3-fluoropropyl)amino)methyl)-3-methoxyphenyl)pyridin-4-yl)-2-(trifluoromethyl)phenyl)-2-methoxypyridin-3-yl)methyl)-3-fluoropropan-1-amine;
(1r,4r)-4-(((6-(3-(3-chloro-2-(4-((((1r,4r)-4-hydroxycyclohexyl)amino)methyl)-3-methoxyphenyl)pyridin-4-yl)-2-(trifluoromethyl)phenyl)-2-methoxypyridin-3-yl)methyl)amino)cyclohexan-1-ol;
(1r,4r)-4-(((6-(3-(3-chloro-2-(4-((((1r,4s)-4-hydroxycyclohexyl)amino)methyl)-3-methoxyphenyl)pyridin-4-yl)-2-(trifluoromethyl)phenyl)-2-methoxypyridin-3-yl)methyl)amino)cyclohexan-1-ol;
(1r,4r)-4-(((6-(3-(3-chloro-2-(4-((((1s,4r)-4-hydroxycyclohexyl)amino)methyl)-3-methoxyphenyl)pyridin-4-yl)-2-(trifluoromethyl)phenyl)-2-methoxypyridin-3-yl)methyl)amino)cyclohexan-1-ol;
(1r,4r)-4-(((6-(3-(3-chloro-2-(4-((((1s,4s)-4-hydroxycyclohexyl)amino)methyl)-3-methoxyphenyl)pyridin-4-yl)-2-(trifluoromethyl)phenyl)-2-methoxypyridin-3-yl)methyl)amino)cyclohexan-1-ol;
(1r,4s)-4-(((6-(3-(3-chloro-2-(4-((((1r,4r)-4-hydroxycyclohexyl)amino)methyl)-3-methoxyphenyl)pyridin-4-yl)-2-(trifluoromethyl)phenyl)-2-methoxypyridin-3-yl)methyl)amino)cyclohexan-1-ol;
(1r,4s)-4-(((6-(3-(3-chloro-2-(4-((((1r,4s)-4-hydroxycyclohexyl)amino)methyl)-3-methoxyphenyl)pyridin-4-yl)-2-(trifluoromethyl)phenyl)-2-methoxypyridin-3-yl)methyl)amino)cyclohexan-1-ol;
(1r,4s)-4-(((6-(3-(3-chloro-2-(4-((((1s,4r)-4-hydroxycyclohexyl)amino)methyl)-3-methoxyphenyl)pyridin-4-yl)-2-(trifluoromethyl)phenyl)-2-methoxypyridin-3-yl)methyl)amino)cyclohexan-1-ol;
(1r,4s)-4-(((6-(3-(3-chloro-2-(4-((((1s,4s)-4-hydroxycyclohexyl)amino)methyl)-3-methoxyphenyl)pyridin-4-yl)-2-(trifluoromethyl)phenyl)-2-methoxypyridin-3-yl)methyl)amino)cyclohexan-1-ol;
(1s,4r)-4-(((6-(3-(3-chloro-2-(4-((((1r,4r)-4-hydroxycyclohexyl)amino)methyl)-3-methoxyphenyl)pyridin-4-yl)-2-(trifluoromethyl)phenyl)-2-methoxypyridin-3-yl)methyl)amino)cyclohexan-1-ol;
(1s,4r)-4-(((6-(3-(3-chloro-2-(4-((((1r,4s)-4-hydroxycyclohexyl)amino)methyl)-3-methoxyphenyl)pyridin-4-yl)-2-(trifluoromethyl)phenyl)-2-methoxypyridin-3-yl)methyl)amino)cyclohexan-1-ol;
(1s,4r)-4-(((6-(3-(3-chloro-2-(4-((((1s,4r)-4-hydroxycyclohexyl)amino)methyl)-3-methoxyphenyl)pyridin-4-yl)-2-(trifluoromethyl)phenyl)-2-methoxypyridin-3-yl)methyl)amino)cyclohexan-1-ol;
(1s,4r)-4-(((6-(3-(3-chloro-2-(4-((((1s,4s)-4-hydroxycyclohexyl)amino)methyl)-3-methoxyphenyl)pyridin-4-yl)-2-(trifluoromethyl)phenyl)-2-methoxypyridin-3-yl)methyl)amino)cyclohexan-1-ol;
(1s,4s)-4-(((6-(3-(3-chloro-2-(4-((((1r,4r)-4-hydroxycyclohexyl)amino)methyl)-3-methoxyphenyl)pyridin-4-yl)-2-(trifluoromethyl)phenyl)-2-methoxypyridin-3-yl)methyl)amino)cyclohexan-1-ol;
(1s,4s)-4-(((6-(3-(3-chloro-2-(4-((((1r,4s)-4-hydroxycyclohexyl)amino)methyl)-3-methoxyphenyl)pyridin-4-yl)-2-(trifluoromethyl)phenyl)-2-methoxypyridin-3-yl)methyl)amino)cyclohexan-1-ol;
(1s,4s)-4-(((6-(3-(3-chloro-2-(4-((((1s,4r)-4-hydroxycyclohexyl)amino)methyl)-3-methoxyphenyl)pyridin-4-yl)-2-(trifluoromethyl)phenyl)-2-methoxypyridin-3-yl)methyl)amino)cyclohexan-1-ol;
(1s,4s)-4-(((6-(3-(3-chloro-2-(4-((((1s,4s)-4-hydroxycyclohexyl)amino)methyl)-3-methoxyphenyl)pyridin-4-yl)-2-(trifluoromethyl)phenyl)-2-methoxypyridin-3-yl)methyl)amino)cyclohexan-1-ol;
1-(6-((6-(3-(6-(4-((6-acetyl-2,6-diazaspiro[3.3]heptan-2-yl)methyl)-3-methoxyphenyl)-5-chloropyrimidin-4-yl)-2-chlorophenyl)-2-methoxypyridin-3-yl)methyl)-2,6-diazaspiro[3.3]heptan-2-yl)ethan-1-one;
N-(3-(3-chloro-2-(3-methoxy-4-((7-oxo-2,6-diazaspiro[3.4]octan-2-yl)methyl)phenyl)pyridin-4-yl)-2-methylphenyl)-5-((7-oxo-2,6-diazaspiro[3.4]octan-2-yl)methyl)picolinamide;
(3-(((6-(2-chloro-3-(3-chloro-2-(4-(((3-(hydroxymethyl)bicyclo[1.1.1]pentan-1-yl)amino)methyl)-3-methoxyphenyl)pyridin-4-yl)phenyl)-2-methoxypyridin-3-yl)methyl)amino)bicyclo[1.1.1]pentan-1-yl)methanol;
(1s,3s)-3-(((6-(2-chloro-3-(5-chloro-6-(4-((((1s,3s)-3-hydroxycyclobutyl)amino)methyl)-3-methoxyphenyl)pyrimidin-4-yl)phenyl)-2-methoxypyridin-3-yl)methyl)amino)cyclobutan-1-ol;
(1r,3r)-3-(((6-(2-chloro-3-(5-chloro-6-(4-((((1r,3r)-3-hydroxycyclobutyl)amino)methyl)-3-methoxyphenyl)pyrimidin-4-yl)phenyl)-2-methoxypyridin-3-yl)methyl)amino)cyclobutan-1-ol;
(1r,3r)-3-(((6-(2-chloro-3-(5-chloro-6-(4-((((1r,3s)-3-hydroxycyclobutyl)amino)methyl)-3-methoxyphenyl)pyrimidin-4-yl)phenyl)-2-methoxypyridin-3-yl)methyl)amino)cyclobutan-1-ol;
(1r,3r)-3-(((6-(2-chloro-3-(5-chloro-6-(4-((((1s,3r)-3-hydroxycyclobutyl)amino)methyl)-3-methoxyphenyl)pyrimidin-4-yl)phenyl)-2-methoxypyridin-3-yl)methyl)amino)cyclobutan-1-ol;
(1r,3r)-3-(((6-(2-chloro-3-(5-chloro-6-(4-((((1s,3s)-3-hydroxycyclobutyl)amino)methyl)-3-methoxyphenyl)pyrimidin-4-yl)phenyl)-2-methoxypyridin-3-yl)methyl)amino)cyclobutan-1-ol;
(1r,3s)-3-(((6-(2-chloro-3-(5-chloro-6-(4-((((1r,3r)-3-hydroxycyclobutyl)amino)methyl)-3-methoxyphenyl)pyrimidin-4-yl)phenyl)-2-methoxypyridin-3-yl)methyl)amino)cyclobutan-1-ol;
(1r,3s)-3-(((6-(2-chloro-3-(5-chloro-6-(4-((((1r,3s)-3-hydroxycyclobutyl)amino)methyl)-3-methoxyphenyl)pyrimidin-4-yl)phenyl)-2-methoxypyridin-3-yl)methyl)amino)cyclobutan-1-ol;
(1r,3s)-3-(((6-(2-chloro-3-(5-chloro-6-(4-((((1s,3r)-3-hydroxycyclobutyl)amino)methyl)-3-methoxyphenyl)pyrimidin-4-yl)phenyl)-2-methoxypyridin-3-yl)methyl)amino)cyclobutan-1-ol;
(1r,3s)-3-(((6-(2-chloro-3-(5-chloro-6-(4-((((1s,3s)-3-hydroxycyclobutyl)amino)methyl)-3-methoxyphenyl)pyrimidin-4-yl)phenyl)-2-methoxypyridin-3-yl)methyl)amino)cyclobutan-1-ol;
(1s,3r)-3-(((6-(2-chloro-3-(5-chloro-6-(4-((((1r,3r)-3-hydroxycyclobutyl)amino)methyl)-3-methoxyphenyl)pyrimidin-4-yl)phenyl)-2-methoxypyridin-3-yl)methyl)amino)cyclobutan-1-ol;
(1s,3r)-3-(((6-(2-chloro-3-(5-chloro-6-(4-((((1r,3s)-3-hydroxycyclobutyl)amino)methyl)-3-methoxyphenyl)pyrimidin-4-yl)phenyl)-2-methoxypyridin-3-yl)methyl)amino)cyclobutan-1-ol;
(1s,3r)-3-(((6-(2-chloro-3-(5-chloro-6-(4-((((1s,3r)-3-hydroxycyclobutyl)amino)methyl)-3-methoxyphenyl)pyrimidin-4-yl)phenyl)-2-methoxypyridin-3-yl)methyl)amino)cyclobutan-1-ol;
(1s,3r)-3-(((6-(2-chloro-3-(5-chloro-6-(4-((((1s,3s)-3-hydroxycyclobutyl)amino)methyl)-3-methoxyphenyl)pyrimidin-4-yl)phenyl)-2-methoxypyridin-3-yl)methyl)amino)cyclobutan-1-ol;
(1s,3s)-3-(((6-(2-chloro-3-(5-chloro-6-(4-((((1r,3r)-3-hydroxycyclobutyl)amino)methyl)-3-methoxyphenyl)pyrimidin-4-yl)phenyl)-2-methoxypyridin-3-yl)methyl)amino)cyclobutan-1-ol;
(1s,3s)-3-(((6-(2-chloro-3-(5-chloro-6-(4-((((1r,3s)-3-hydroxycyclobutyl)amino)methyl)-3-methoxyphenyl)pyrimidin-4-yl)phenyl)-2-methoxypyridin-3-yl)methyl)amino)cyclobutan-1-ol;
(1s,3s)-3-(((6-(2-chloro-3-(5-chloro-6-(4-((((1s,3r)-3-hydroxycyclobutyl)amino)methyl)-3-methoxyphenyl)pyrimidin-4-yl)phenyl)-2-methoxypyridin-3-yl)methyl)amino)cyclobutan-1-ol;
1-(6-((6-(2-chloro-3-(3-chloro-2-(3-methoxy-4-((6-(2-methoxyacetyl)-2,6-diazaspiro[3.3]heptan-2-yl)methyl)phenyl)pyridin-4-yl)phenyl)-2-methoxypyridin-3-yl)methyl)-2,6-diazaspiro[3.3]heptan-2-yl)-2-methoxyethan-1-one;
1-(4-(((6-(2-chloro-3-(3-chloro-2-(2-((((1r,4r)-4-hydroxycyclohexyl)amino)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one;
1-(4-(((6-(2-chloro-3-(3-chloro-2-(2-((((1s,4r)-4-hydroxycyclohexyl)amino)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one;
1-(4-(((6-(2-chloro-3-(3-chloro-2-(2-((((1r,4s)-4-hydroxycyclohexyl)amino)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one;
1-(4-(((6-(2-chloro-3-(3-chloro-2-(2-((((1s,4s)-4-hydroxycyclohexyl)amino)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one;
1-(4-(((6-(2-chloro-3-(3-chloro-2-(2-((((1r,3r)-3-hydroxy-3-methylcyclobutyl)amino)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one;
1-(4-(((6-(2-chloro-3-(3-chloro-2-(2-((((1s,3r)-3-hydroxy-3-methylcyclobutyl)amino)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one;
1-(4-(((6-(2-chloro-3-(3-chloro-2-(2-((((1r,3s)-3-hydroxy-3-methylcyclobutyl)amino)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one;
1-(4-(((6-(2-chloro-3-(3-chloro-2-(2-((((1s,3s)-3-hydroxy-3-methylcyclobutyl)amino)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one;
2-((6-(2-chloro-3-(3-chloro-2-(3-(difluoromethoxy)-4-((7-oxo-2,6-diazaspiro[3.4]octan-2-yl)methyl)phenyl)pyridin-4-yl)phenyl)-2-methoxypyridin-3-yl)methyl)-2,6-diazaspiro[3.4]octan-7-one;
1-(6-((6-(3-(2-(4-((6-acetyl-2,6-diazaspiro[3.3]heptan-2-yl)methyl)-3-(difluoromethoxy)phenyl)-3-chloropyridin-4-yl)-2-chlorophenyl)-2-methoxypyridin-3-yl)methyl)-2,6-diazaspiro[3.3]heptan-2-yl)ethan-1-one;
1-(4-(((S)-1-(6-(3-(2-(4-((S)-1-((1-acetylpiperidin-4-yl)amino)ethyl)-3-methoxyphenyl)-3-chloropyridin-4-yl)-2-chlorophenyl)-2-methoxypyridin-3-yl)ethyl)amino)piperidin-1-yl)ethan-1-one;
1-(4-(((S)-1-(6-(3-(2-(4-((R)-1-((1-acetylpiperidin-4-yl)amino)ethyl)-3-methoxyphenyl)-3-chloropyridin-4-yl)-2-chlorophenyl)-2-methoxypyridin-3-yl)ethyl)amino)piperidin-1-yl)ethan-1-one;
1-(4-(((R)-1-(6-(3-(2-(4-((S)-1-((1-acetylpiperidin-4-yl)amino)ethyl)-3-methoxyphenyl)-3-chloropyridin-4-yl)-2-chlorophenyl)-2-methoxypyridin-3-yl)ethyl)amino)piperidin-1-yl)ethan-1-one;
1-(4-(((R)-1-(6-(3-(2-(4-((R)-1-((1-acetylpiperidin-4-yl)amino)ethyl)-3-methoxyphenyl)-3-chloropyridin-4-yl)-2-chlorophenyl)-2-methoxypyridin-3-yl)ethyl)amino)piperidin-1-yl)ethan-1-one;
1-(4-(((6-(2-chloro-3-(3-chloro-2-(3-methoxy-4-(((tetrahydro-2H-pyran-4-yl)amino)methyl)phenyl)pyridin-4-yl)phenyl)-1-methyl-1H-pyrrolo[2,3-b]pyridin-3-yl)methyl)(methyl)amino)piperidin-1-yl)ethan-1-one;
1-(4-(((6-(3-(2-(4-((6-acetyl-2,6-diazaspiro[3.3]heptan-2-yl)methyl)-3-methoxyphenyl)-3-chloropyridin-4-yl)-2-chlorophenyl)-1-methyl-1H-pyrrolo[2,3-b]pyridin-3-yl)methyl)(methyl)amino)piperidin-1-yl)ethan-1-one;
1-(4-(((6-(3-(3-chloro-2-(3-methoxy-4-(((1-(2-methoxyacetyl)piperidin-4-yl)amino)methyl)phenyl)pyridin-4-yl)-2-(trifluoromethyl)phenyl)-2-methoxypyridin-3-yl)methyl)amino)piperidin-1-yl)-2-methoxyethan-1-one;
2-(4-(4-(3-(3-(((1-acetylpiperidin-4-yl)(methyl)amino)methyl)-1-methyl-1H-pyrrolo[2,3-b]pyridin-6-yl)-2-chlorophenyl)-3-chloropyridin-2-yl)-2-methoxybenzyl)-2,6-diazaspiro[3.4]octan-7-one;
N-(4-(4-(3-(5-(((2-oxaspiro[3.3]heptan-6-yl)amino)methyl)-6-methoxypyridin-2-yl)-2-(trifluoromethyl)phenyl)-3-chloropyridin-2-yl)-2-methoxybenzyl)-2-oxaspiro[3.3]heptan-6-amine;
2-(4-(3-chloro-4-(2-chloro-3-(1-methyl-3-(((tetrahydro-2H-pyran-4-yl)amino)methyl)-1H-pyrrolo[2,3-b]pyridin-6-yl)phenyl)pyridin-2-yl)-2-methoxybenzyl)-2,6-diazaspiro[3.4]octan-7-one;
2-((4-(3-chloro-4-(2-chloro-3-(1-methyl-3-(((tetrahydro-2H-pyran-4-yl)amino)methyl)-1H-pyrrolo[2,3-b]pyridin-6-yl)phenyl)pyridin-2-yl)-2-methoxybenzyl)amino)ethan-1-ol;
1-(4-(((6-(3-(2-(4-(((1-acetylpiperidin-4-yl)amino)methyl)-3-methoxyphenyl)-3-chloropyridin-4-yl)-2-chlorophenyl)-2-methoxypyridin-3-yl)methyl)amino)piperidin-1-yl)-3-methoxypropan-1-one;
(S)-5-((((3'-chloro-6-methoxy-2'-((S)-1-((6-methoxy-5-(((tetrahydro-2H-pyran-4-yl)amino)methyl)-3-(trifluoromethyl)pyridin-2-yl)oxy)-2,3-dihydro-1H-inden-4-yl)-[2,4'-bipyridin]-5-yl)methyl)amino)methyl)pyrrolidin-2-one;
(S)-5-((((3'-chloro-6-methoxy-2'-((R)-1-((6-methoxy-5-(((tetrahydro-2H-pyran-4-yl)amino)methyl)-3-(trifluoromethyl)pyridin-2-yl)oxy)-2,3-dihydro-1H-inden-4-yl)-[2,4'-bipyridin]-5-yl)methyl)amino)methyl)pyrrolidin-2-one;
(R)-5-((((3'-chloro-6-methoxy-2'-((S)-1-((6-methoxy-5-(((tetrahydro-2H-pyran-4-yl)amino)methyl)-3-(trifluoromethyl)pyridin-2-yl)oxy)-2,3-dihydro-1H-inden-4-yl)-[2,4'-bipyridin]-5-yl)methyl)amino)methyl)pyrrolidin-2-one;
(R)-5-((((3'-chloro-6-methoxy-2'-((R)-1-((6-methoxy-5-(((tetrahydro-2H-pyran-4-yl)amino)methyl)-3-(trifluoromethyl)pyridin-2-yl)oxy)-2,3-dihydro-1H-inden-4-yl)-[2,4'-bipyridin]-5-yl)methyl)amino)methyl)pyrrolidin-2-one;
1-(6-(4-(3-chloro-4-(2-chloro-3-(1-methyl-3-(((tetrahydro-2H-pyran-4-yl)amino)methyl)-1H-pyrrolo[2,3-b]pyridin-6-yl)phenyl)pyridin-2-yl)-2-methoxybenzyl)-2,6-diazaspiro[3.3]heptan-2-yl)ethan-1-one;
(S)-5-((((6-(3-(3-chloro-2-(3-methoxy-4-(((((S)-5-oxopyrrolidin-2-yl)methyl)amino)methyl)phenyl)pyridin-4-yl)-2-(trifluoromethyl)phenyl)-2-methoxypyridin-3-yl)methyl)amino)methyl)pyrrolidin-2-one;
(S)-5-((((6-(3-(3-chloro-2-(3-methoxy-4-(((((R)-5-oxopyrrolidin-2-yl)methyl)amino)methyl)phenyl)pyridin-4-yl)-2-(trifluoromethyl)phenyl)-2-methoxypyridin-3-yl)methyl)amino)methyl)pyrrolidin-2-one;
(R)-5-((((6-(3-(3-chloro-2-(3-methoxy-4-(((((S)-5-oxopyrrolidin-2-yl)methyl)amino)methyl)phenyl)pyridin-4-yl)-2-(trifluoromethyl)phenyl)-2-methoxypyridin-3-yl)methyl)amino)methyl)pyrrolidin-2-one;
(R)-5-((((6-(3-(3-chloro-2-(3-methoxy-4-(((((R)-5-oxopyrrolidin-2-yl)methyl)amino)methyl)phenyl)pyridin-4-yl)-2-(trifluoromethyl)phenyl)-2-methoxypyridin-3-yl)methyl)amino)methyl)pyrrolidin-2-one;
(S)-5-((((6-(2-chloro-3-(3-chloro-2-(4-((S)-2-hydroxypropyl)-1,1-dioxido-2,3,4,5-tetrahydrobenzo[f][1,4]thiazepin-8-yl)pyridin-4-yl)phenyl)-2-methoxypyridin-3-yl)methyl)amino)methyl)pyrrolidin-2-one;
(S)-5-((((6-(2-chloro-3-(3-chloro-2-(4-((R)-2-hydroxypropyl)-1,1-dioxido-2,3,4,5-tetrahydrobenzo[f][1,4]thiazepin-8-yl)pyridin-4-yl)phenyl)-2-methoxypyridin-3-yl)methyl)amino)methyl)pyrrolidin-2-one;
(R)-5-((((6-(2-chloro-3-(3-chloro-2-(4-((S)-2-hydroxypropyl)-1,1-dioxido-2,3,4,5-tetrahydrobenzo[f][1,4]thiazepin-8-yl)pyridin-4-yl)phenyl)-2-methoxypyridin-3-yl)methyl)amino)methyl)pyrrolidin-2-one;
(R)-5-((((6-(2-chloro-3-(3-chloro-2-(4-((R)-2-hydroxypropyl)-1,1-dioxido-2,3,4,5-tetrahydrobenzo[f][1,4]thiazepin-8-yl)pyridin-4-yl)phenyl)-2-methoxypyridin-3-yl)methyl)amino)methyl)pyrrolidin-2-one;
2-(((6-(3-(3-chloro-2-(4-(((2-hydroxyethyl)amino)methyl)-3-methoxyphenyl)pyridin-4-yl)-2-(trifluoromethyl)phenyl)-2-methoxypyridin-3-yl)methyl)amino)ethan-1-ol;
1-(6-(3-(3-chloro-2-(3-methoxy-4-((methylamino)methyl)phenyl)pyridin-4-yl)-2-(trifluoromethyl)phenyl)-2-methoxypyridin-3-yl)-N-methylmethanamine;
N-((6-(3-(3-chloro-2-(3-methoxy-4-(((2-methoxyethyl)amino)methyl)phenyl)pyridin-4-yl)-2-(trifluoromethyl)phenyl)-2-methoxypyridin-3-yl)methyl)-2-methoxyethan-1-amine;
2-(2-(4-(((1-acetylpiperidin-4-yl)amino)methyl)-3-methoxyphenyl)-3-chloropyridin-4-yl)-6-(5-(((1-acetylpiperidin-4-yl)amino)methyl)-6-methoxypyridin-2-yl)benzonitrile;
1-(4-(((6-(2-chloro-3-(3-chloro-2-(4-((4-hydroxypiperidin-1-yl)methyl)-3-methoxyphenyl)pyridin-4-yl)phenyl)-2-methoxypyridin-3-yl)methyl)amino)piperidin-1-yl)-3-methoxypropan-1-one;
(S)-5-((((6-(2-chloro-3-(3-chloro-2-(3-(difluoromethoxy)-4-(((((S)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
(S)-5-((((6-(2-chloro-3-(3-chloro-2-(3-(difluoromethoxy)-4-(((((R)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
(R)-5-((((6-(2-chloro-3-(3-chloro-2-(3-(difluoromethoxy)-4-(((((S)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
(R)-5-((((6-(2-chloro-3-(3-chloro-2-(3-(difluoromethoxy)-4-(((((R)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
N-((6-(2-chloro-3-(3-chloro-2-(3-(difluoromethoxy)-4-(((tetrahydro-2H-pyran-4-yl)amino)methyl)phenyl)pyridin-4-yl)phenyl)-2-methoxypyridin-3-yl)methyl)tetrahydro-2H-pyran-4-amine;
4-(((6-(2-chloro-3-(3-chloro-2-(3-(difluoromethoxy)-4-(((1-methyl-2-oxopiperidin-4-yl)amino)methyl)phenyl)pyridin-4-yl)phenyl)-2-methoxypyridin-3-yl)methyl)amino)-1-methylpiperidin-2-one;
2-(4-(((6-(2-chloro-3-(3-chloro-2-(3-(difluoromethoxy)-4-(((1-(2-hydroxyethyl)piperidin-4-yl)amino)methyl)phenyl)pyridin-4-yl)phenyl)-2-methoxypyridin-3-yl)methyl)amino)piperidin-1-yl)ethan-1-ol;
1-(4-(((6-(3-(4-(4-(((1-acetylpiperidin-4-yl)amino)methyl)-3-methoxyphenyl)-3-chloropyridin-2-yl)-2-chlorophenyl)-2-methoxypyridin-3-yl)methyl)amino)piperidin-1-yl)ethan-1-one;
2-(4-(3-chloro-2-(2-chloro-3-(6-methoxy-5-((7-oxo-2,6-diazaspiro[3.4]octan-2-yl)methyl)pyridin-2-yl)phenyl)pyridin-4-yl)-2-methoxybenzyl)-2,6-diazaspiro[3.4]octan-7-one;
N-(4-(3-chloro-2-(2-chloro-3-(6-methoxy-5-(((tetrahydro-2H-pyran-4-yl)amino)methyl)pyridin-2-yl)phenyl)pyridin-4-yl)-2-methoxybenzyl)tetrahydro-2H-pyran-4-amine;
(S)-5-(((4-(3-chloro-2-(2-chloro-3-(6-methoxy-5-(((((S)-5-oxopyrrolidin-2-yl)methyl)amino)methyl)pyridin-2-yl)phenyl)pyridin-4-yl)-2-methoxybenzyl)amino)methyl)pyrrolidin-2-one;
(S)-5-(((4-(3-chloro-2-(2-chloro-3-(6-methoxy-5-(((((R)-5-oxopyrrolidin-2-yl)methyl)amino)methyl)pyridin-2-yl)phenyl)pyridin-4-yl)-2-methoxybenzyl)amino)methyl)pyrrolidin-2-one;
(R)-5-(((4-(3-chloro-2-(2-chloro-3-(6-methoxy-5-(((((S)-5-oxopyrrolidin-2-yl)methyl)amino)methyl)pyridin-2-yl)phenyl)pyridin-4-yl)-2-methoxybenzyl)amino)methyl)pyrrolidin-2-one;
(R)-5-(((4-(3-chloro-2-(2-chloro-3-(6-methoxy-5-(((((R)-5-oxopyrrolidin-2-yl)methyl)amino)methyl)pyridin-2-yl)phenyl)pyridin-4-yl)-2-methoxybenzyl)amino)methyl)pyrrolidin-2-one;
2-(4-(3-chloro-4-(2-chloro-3-(6-methoxy-5-(((1-(3-methoxypropanoyl)piperidin-4-yl)amino)methyl)pyridin-2-yl)phenyl)pyridin-2-yl)-2-methoxybenzyl)-2,6-diazaspiro[3.4]octan-7-one;
N-((6-(2-chloro-3-(5-chloro-6-(3-methoxy-4-(((tetrahydro-2H-pyran-4-yl)amino)methyl)phenyl)pyrimidin-4-yl)phenyl)-2-methoxypyridin-3-yl)methyl)tetrahydro-2H-pyran-4-amine;
1-(4-(((6-(3-(5'-(((1-acetylpiperidin-4-yl)amino)methyl)-3-chloro-6'-methoxy-[2,2'-bipyridin]-4-yl)-2-chlorophenyl)-2-methoxypyridin-3-yl)methyl)amino)piperidin-1-yl)ethan-1-one;
2-((6-(2-chloro-3-(3-chloro-6'-methoxy-5'-((7-oxo-2,6-diazaspiro[3.4]octan-2-yl)methyl)-[2,2'-bipyridin]-4-yl)phenyl)-2-methoxypyridin-3-yl)methyl)-2,6-diazaspiro[3.4]octan-7-one;
N-((6-(2-chloro-3-(3-chloro-6'-methoxy-5'-(((tetrahydro-2H-pyran-4-yl)amino)methyl)-[2,2'-bipyridin]-4-yl)phenyl)-2-methoxypyridin-3-yl)methyl)tetrahydro-2H-pyran-4-amine;
(S)-5-((((6-(2-chloro-3-(3-chloro-6'-methoxy-5'-(((((S)-5-oxopyrrolidin-2-yl)methyl)amino)methyl)-[2,2'-bipyridin]-4-yl)phenyl)-2-methoxypyridin-3-yl)methyl)amino)methyl)pyrrolidin-2-one;
(S)-5-((((6-(2-chloro-3-(3-chloro-6'-methoxy-5'-(((((R)-5-oxopyrrolidin-2-yl)methyl)amino)methyl)-[2,2'-bipyridin]-4-yl)phenyl)-2-methoxypyridin-3-yl)methyl)amino)methyl)pyrrolidin-2-one;
(R)-5-((((6-(2-chloro-3-(3-chloro-6'-methoxy-5'-(((((S)-5-oxopyrrolidin-2-yl)methyl)amino)methyl)-[2,2'-bipyridin]-4-yl)phenyl)-2-methoxypyridin-3-yl)methyl)amino)methyl)pyrrolidin-2-one;
(R)-5-((((6-(2-chloro-3-(3-chloro-6'-methoxy-5'-(((((R)-5-oxopyrrolidin-2-yl)methyl)amino)methyl)-[2,2'-bipyridin]-4-yl)phenyl)-2-methoxypyridin-3-yl)methyl)amino)methyl)pyrrolidin-2-one;
1-(4-(((6-(3-(2-(4-((6-acetyl-2,6-diazaspiro[3.3]heptan-2-yl)methyl)-3-methoxyphenyl)-3-chloropyridin-4-yl)-2-chlorophenyl)-2-methoxypyridin-3-yl)methyl)amino)piperidin-1-yl)-3-methoxypropan-1-one;
(S)-1-(4-(((6-(2-chloro-3-(3-chloro-2-(4-(((2-hydroxypropyl)amino)methyl)-3-methoxyphenyl)pyridin-4-yl)phenyl)-2-methoxypyridin-3-yl)methyl)amino)piperidin-1-yl)-3-methoxypropan-1-one;
(R)-1-(4-(((6-(2-chloro-3-(3-chloro-2-(4-(((2-hydroxypropyl)amino)methyl)-3-methoxyphenyl)pyridin-4-yl)phenyl)-2-methoxypyridin-3-yl)methyl)amino)piperidin-1-yl)-3-methoxypropan-1-one;
(S)-5-((((2'-((S)-1-((5-(((1-acetylpiperidin-4-yl)amino)methyl)-6-methoxy-3-(trifluoromethyl)pyridin-2-yl)amino)-2,3-dihydro-1H-inden-4-yl)-3'-chloro-6-methoxy-[2,4'-bipyridin]-5-yl)methyl)amino)methyl)pyrrolidin-2-one;
(S)-5-((((2'-((R)-1-((5-(((1-acetylpiperidin-4-yl)amino)methyl)-6-methoxy-3-(trifluoromethyl)pyridin-2-yl)amino)-2,3-dihydro-1H-inden-4-yl)-3'-chloro-6-methoxy-[2,4'-bipyridin]-5-yl)methyl)amino)methyl)pyrrolidin-2-one;
(R)-5-((((2'-((S)-1-((5-(((1-acetylpiperidin-4-yl)amino)methyl)-6-methoxy-3-(trifluoromethyl)pyridin-2-yl)amino)-2,3-dihydro-1H-inden-4-yl)-3'-chloro-6-methoxy-[2,4'-bipyridin]-5-yl)methyl)amino)methyl)pyrrolidin-2-one;
(R)-5-((((2'-((R)-1-((5-(((1-acetylpiperidin-4-yl)amino)methyl)-6-methoxy-3-(trifluoromethyl)pyridin-2-yl)amino)-2,3-dihydro-1H-inden-4-yl)-3'-chloro-6-methoxy-[2,4'-bipyridin]-5-yl)methyl)amino)methyl)pyrrolidin-2-one;
(S)-5-((((3'-chloro-6-methoxy-2'-((S)-1-((6-methoxy-5-(((tetrahydro-2H-pyran-4-yl)amino)methyl)-3-(trifluoromethyl)pyridin-2-yl)amino)-2,3-dihydro-1H-inden-4-yl)-[2,4'-bipyridin]-5-yl)methyl)amino)methyl)pyrrolidin-2-one;
(S)-5-((((3'-chloro-6-methoxy-2'-((R)-1-((6-methoxy-5-(((tetrahydro-2H-pyran-4-yl)amino)methyl)-3-(trifluoromethyl)pyridin-2-yl)amino)-2,3-dihydro-1H-inden-4-yl)-[2,4'-bipyridin]-5-yl)methyl)amino)methyl)pyrrolidin-2-one;
(R)-5-((((3'-chloro-6-methoxy-2'-((S)-1-((6-methoxy-5-(((tetrahydro-2H-pyran-4-yl)amino)methyl)-3-(trifluoromethyl)pyridin-2-yl)amino)-2,3-dihydro-1H-inden-4-yl)-[2,4'-bipyridin]-5-yl)methyl)amino)methyl)pyrrolidin-2-one;
(R)-5-((((3'-chloro-6-methoxy-2'-((R)-1-((6-methoxy-5-(((tetrahydro-2H-pyran-4-yl)amino)methyl)-3-(trifluoromethyl)pyridin-2-yl)amino)-2,3-dihydro-1H-inden-4-yl)-[2,4'-bipyridin]-5-yl)methyl)amino)methyl)pyrrolidin-2-one;
N-((6-(2-chloro-3-(3-chloro-2-(3-methoxy-4-(((tetrahydro-2H-pyran-4-yl)amino)methyl)phenyl)pyridin-4-yl)phenyl)-1-methyl-1H-pyrrolo[2,3-b]pyridin-3-yl)methyl)-N-methyltetrahydro-2H-pyran-4-amine;
2-(2-(4-((6-acetyl-2,6-diazaspiro[3.3]heptan-2-yl)methyl)-3-methoxyphenyl)-3-chloropyridin-4-yl)-6-(5-((6-acetyl-2,6-diazaspiro[3.3]heptan-2-yl)methyl)-6-methoxypyridin-2-yl)benzonitrile;
1-(4-(((6-(2-chloro-3-(3-chloro-2-(2-((3-hydroxy-3-methylazetidin-1-yl)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one;
(R)-1-(4-(((6-(2-chloro-3-(3-chloro-2-(2-(((2-hydroxypropyl)amino)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one;
(S)-1-(4-(((6-(2-chloro-3-(3-chloro-2-(2-(((2-hydroxypropyl)amino)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one;
1-(4-(((6-(3-(5-(((1-acetylpiperidin-4-yl)amino)methyl)-4'-chloro-6-methoxy-[2,3'-bipyridin]-5'-yl)-2-chlorophenyl)-2-methoxypyridin-3-yl)methyl)amino)piperidin-1-yl)ethan-1-one;
2-((6-(2-chloro-3-(4'-chloro-6-methoxy-5-((7-oxo-2,6-diazaspiro[3.4]octan-2-yl)methyl)-[2,3'-bipyridin]-5'-yl)phenyl)-2-methoxypyridin-3-yl)methyl)-2,6-diazaspiro[3.4]octan-7-one;
1-(4-(((6-(3-(5-(4-(((1-acetylpiperidin-4-yl)amino)methyl)-3-methoxyphenyl)-4-chloropyridin-3-yl)-2-chlorophenyl)-2-methoxypyridin-3-yl)methyl)amino)piperidin-1-yl)ethan-1-one;
2-(4-(4-chloro-5-(2-chloro-3-(6-methoxy-5-((7-oxo-2,6-diazaspiro[3.4]octan-2-yl)methyl)pyridin-2-yl)phenyl)pyridin-3-yl)-2-methoxybenzyl)-2,6-diazaspiro[3.4]octan-7-one;
1-(6-(2-chloro-3-(5-chloro-6-(3-methoxy-4-(((oxazol-5-ylmethyl)amino)methyl)phenyl)pyrimidin-4-yl)phenyl)-2-methoxypyridin-3-yl)-N-(oxazol-5-ylmethyl)methanamine;
1-(4-(((6-(2-chloro-3-(3-chloro-2-(2-((isopropylamino)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one;
5-(((1-acetylpiperidin-4-yl)amino)methyl)-N-(3-(2-(4-(((1-acetylpiperidin-4-yl)amino)methyl)-3-methoxyphenyl)-3-chloropyridin-4-yl)-2-methylphenyl)picolinamide;
N-(3-(3-chloro-2-(3-methoxy-4-(((tetrahydro-2H-pyran-4-yl)amino)methyl)phenyl)pyridin-4-yl)-2-methylphenyl)-5-(((tetrahydro-2H-pyran-4-yl)amino)methyl)picolinamide;
N-(3-(3-chloro-2-(3-methoxy-4-(((((S)-5-oxopyrrolidin-2-yl)methyl)amino)methyl)phenyl)pyridin-4-yl)-2-methylphenyl)-5-(((((S)-5-oxopyrrolidin-2-yl)methyl)amino)methyl)picolinamide;
N-(3-(3-chloro-2-(3-methoxy-4-(((((S)-5-oxopyrrolidin-2-yl)methyl)amino)methyl)phenyl)pyridin-4-yl)-2-methylphenyl)-5-(((((R)-5-oxopyrrolidin-2-yl)methyl)amino)methyl)picolinamide;
N-(3-(3-chloro-2-(3-methoxy-4-(((((R)-5-oxopyrrolidin-2-yl)methyl)amino)methyl)phenyl)pyridin-4-yl)-2-methylphenyl)-5-(((((S)-5-oxopyrrolidin-2-yl)methyl)amino)methyl)picolinamide;
N-(3-(3-chloro-2-(3-methoxy-4-(((((R)-5-oxopyrrolidin-2-yl)methyl)amino)methyl)phenyl)pyridin-4-yl)-2-methylphenyl)-5-(((((R)-5-oxopyrrolidin-2-yl)methyl)amino)methyl)picolinamide;
N-(3-(3-chloro-2-(4-(((2-hydroxyethyl)amino)methyl)-3-methoxyphenyl)pyridin-4-yl)-2-methylphenyl)-5-(((2-hydroxyethyl)amino)methyl)picolinamide;
5-((6-acetyl-2,6-diazaspiro[3.3]heptan-2-yl)methyl)-N-(3-(2-(4-((6-acetyl-2,6-diazaspiro[3.3]heptan-2-yl)methyl)-3-methoxyphenyl)-3-chloropyridin-4-yl)-2-methylphenyl)picolinamide;
N-(3-(3-chloro-2-(4-((((S)-2-hydroxypropyl)amino)methyl)-3-methoxyphenyl)pyridin-4-yl)-2-methylphenyl)-5-((((S)-2-hydroxypropyl)amino)methyl)picolinamide;
N-(3-(3-chloro-2-(4-((((S)-2-hydroxypropyl)amino)methyl)-3-methoxyphenyl)pyridin-4-yl)-2-methylphenyl)-5-((((R)-2-hydroxypropyl)amino)methyl)picolinamide;
N-(3-(3-chloro-2-(4-((((R)-2-hydroxypropyl)amino)methyl)-3-methoxyphenyl)pyridin-4-yl)-2-methylphenyl)-5-((((S)-2-hydroxypropyl)amino)methyl)picolinamide;
N-(3-(3-chloro-2-(4-((((R)-2-hydroxypropyl)amino)methyl)-3-methoxyphenyl)pyridin-4-yl)-2-methylphenyl)-5-((((R)-2-hydroxypropyl)amino)methyl)picolinamide;
1-(6-(((6-(3-(2-(4-(((2-acetyl-2-azaspiro[3.3]heptan-6-yl)amino)methyl)-3-methoxyphenyl)-3-chloropyridin-4-yl)-2-(trifluoromethyl)phenyl)-2-methoxypyridin-3-yl)methyl)amino)-2-azaspiro[3.3]heptan-2-yl)ethan-1-one;
2-(3-chloro-2-(3-methoxy-4-(((tetrahydro-2H-pyran-4-yl)amino)methyl)phenyl)pyridin-4-yl)-6-(6-methoxy-5-(((tetrahydro-2H-pyran-4-yl)amino)methyl)pyridin-2-yl)benzonitrile;
2-(3-chloro-2-(3-methoxy-4-((7-oxo-2,6-diazaspiro[3.4]octan-2-yl)methyl)phenyl)pyridin-4-yl)-6-(6-methoxy-5-((7-oxo-2,6-diazaspiro[3.4]octan-2-yl)methyl)pyridin-2-yl)benzonitrile;
1-(4-(((6-(2-chloro-3-(3-chloro-2-(2-(((2-hydroxyethyl)amino)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one;
1-(4-((6-(3-(2-(4-((4-acetylpiperazin-1-yl)methyl)-3-methoxyphenyl)-3-chloropyridin-4-yl)-2-chlorophenyl)-2-methoxypyridin-3-yl)methyl)piperazin-1-yl)ethan-1-one;
N-(3-(2-(4-(((1-acetylpiperidin-4-yl)amino)methyl)-3-methoxyphenyl)-3-chloropyridin-4-yl)-2-chlorophenyl)-1,3-dimethyl-2,4-dioxo-1,2,3,4-tetrahydropyrimidine-5-carboxamide;
(S)-N-(2-chloro-3-(3-chloro-2-(3-methoxy-4-((((5-oxopyrrolidin-2-yl)methyl)amino)methyl)phenyl)pyridin-4-yl)phenyl)-1,3-dimethyl-2,4-dioxo-1,2,3,4-tetrahydropyrimidine-5-carboxamide;
(R)-N-(2-chloro-3-(3-chloro-2-(3-methoxy-4-((((5-oxopyrrolidin-2-yl)methyl)amino)methyl)phenyl)pyridin-4-yl)phenyl)-1,3-dimethyl-2,4-dioxo-1,2,3,4-tetrahydropyrimidine-5-carboxamide;
N-(2-chloro-3-(3-chloro-2-(3-methoxy-4-((7-oxo-2,6-diazaspiro[3.4]octan-2-yl)methyl)phenyl)pyridin-4-yl)phenyl)-1,3-dimethyl-2,4-dioxo-1,2,3,4-tetrahydropyrimidine-5-carboxamide;
2-((6-(4-(3-(5-(((1-acetylpiperidin-4-yl)amino)methyl)-6-methoxypyridin-2-yl)-2-chlorophenyl)-3-chloropyridin-2-yl)-8-methoxy-[1,2,4]triazolo[1,5-a]pyridin-2-yl)methyl)-2,6-diazaspiro[3.4]octan-7-one;
1-(4-(((6-(2-chloro-3-(3-chloro-2-(2-((6-hydroxy-2-azaspiro[3.3]heptan-2-yl)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one;
1-(4-(((6-(2-chloro-3-(3-chloro-2-(2-(((2-hydroxyethyl)amino)methyl)-4-methoxypyrrolo[2,1-f][1,2,4]triazin-6-yl)pyridin-4-yl)phenyl)-2-methoxypyridin-3-yl)methyl)amino)piperidin-1-yl)ethan-1-one;
2-((6-(3-(2-(4-((6-acetyl-2,6-diazaspiro[3.3]heptan-2-yl)methyl)-3-(difluoromethoxy)phenyl)-3-chloropyridin-4-yl)-2-chlorophenyl)-2-methoxypyridin-3-yl)methyl)-2,6-diazaspiro[3.4]octan-7-one;
1-(6-(4-(3-chloro-4-(2-chloro-3-(6-methoxy-5-(((tetrahydro-2H-pyran-4-yl)amino)methyl)pyridin-2-yl)phenyl)pyridin-2-yl)-2-(difluoromethoxy)benzyl)-2,6-diazaspiro[3.3]heptan-2-yl)ethan-1-one;
1-(4-(((6-(3-(2-(4-((6-acetyl-2,6-diazaspiro[3.3]heptan-2-yl)methyl)-3-(difluoromethoxy)phenyl)-3-chloropyridin-4-yl)-2-chlorophenyl)-2-methoxypyridin-3-yl)methyl)amino)piperidin-1-yl)ethan-1-one;
2-((6-(3-(2-(4-(((1-acetylpiperidin-4-yl)amino)methyl)-3-(difluoromethoxy)phenyl)-3-chloropyridin-4-yl)-2-chlorophenyl)-2-methoxypyridin-3-yl)methyl)-2,6-diazaspiro[3.4]octan-7-one;
(S)-5-((((6-(((S)-4-(3'-chloro-6-methoxy-5-(((tetrahydro-2H-pyran-4-yl)amino)methyl)-[2,4'-bipyridin]-2'-yl)-2,3-dihydro-1H-inden-1-yl)amino)-2-methoxy-5-(trifluoromethyl)pyridin-3-yl)methyl)amino)methyl)pyrrolidin-2-one;
(S)-5-((((6-(((R)-4-(3'-chloro-6-methoxy-5-(((tetrahydro-2H-pyran-4-yl)amino)methyl)-[2,4'-bipyridin]-2'-yl)-2,3-dihydro-1H-inden-1-yl)amino)-2-methoxy-5-(trifluoromethyl)pyridin-3-yl)methyl)amino)methyl)pyrrolidin-2-one;
(R)-5-((((6-(((S)-4-(3'-chloro-6-methoxy-5-(((tetrahydro-2H-pyran-4-yl)amino)methyl)-[2,4'-bipyridin]-2'-yl)-2,3-dihydro-1H-inden-1-yl)amino)-2-methoxy-5-(trifluoromethyl)pyridin-3-yl)methyl)amino)methyl)pyrrolidin-2-one;
(R)-5-((((6-(((R)-4-(3'-chloro-6-methoxy-5-(((tetrahydro-2H-pyran-4-yl)amino)methyl)-[2,4'-bipyridin]-2'-yl)-2,3-dihydro-1H-inden-1-yl)amino)-2-methoxy-5-(trifluoromethyl)pyridin-3-yl)methyl)amino)methyl)pyrrolidin-2-one;
1-(6-((6-(2-chloro-3-(3-chloro-2-(4-((6-(2-hydroxyacetyl)-2,6-diazaspiro[3.3]heptan-2-yl)methyl)-3-methoxyphenyl)pyridin-4-yl)phenyl)-2-methoxypyridin-3-yl)methyl)-2,6-diazaspiro[3.3]heptan-2-yl)-2-hydroxyethan-1-one;
1-(4-((4-(5-(((1-acetylpiperidin-4-yl)amino)methyl)-3',3"-dichloro-6-methoxy-[2,4':2',4"-terpyridin]-2"-yl)-2-methoxybenzyl)amino)piperidin-1-yl)ethan-1-one;
2-(4-(3',3"-dichloro-6-methoxy-5-((7-oxo-2,6-diazaspiro[3.4]octan-2-yl)methyl)-[2,4':2',4"-terpyridin]-2"-yl)-2-methoxybenzyl)-2,6-diazaspiro[3.4]octan-7-one;
2-(2-((6-(2-chloro-3-(3-chloro-2-(4-((6-(2-hydroxyethyl)-2,6-diazaspiro[3.4]octan-2-yl)methyl)-3-methoxyphenyl)pyridin-4-yl)phenyl)-2-methoxypyridin-3-yl)methyl)-2,6-diazaspiro[3.4]octan-6-yl)ethan-1-ol;
1-(2-((6-(3-(2-(4-((6-acetyl-2,6-diazaspiro[3.4]octan-2-yl)methyl)-3-methoxyphenyl)-3-chloropyridin-4-yl)-2-chlorophenyl)-2-methoxypyridin-3-yl)methyl)-2,6-diazaspiro[3.4]octan-6-yl)ethan-1-one;
N-(2-chloro-3-(3-chloro-2-(3-methoxy-4-((7-oxo-2,6-diazaspiro[3.4]octan-2-yl)methyl)phenyl)pyridin-4-yl)phenyl)-5-((7-oxo-2,6-diazaspiro[3.4]octan-2-yl)methyl)picolinamide;
5-(((1-acetylpiperidin-4-yl)amino)methyl)-N-(3-(2-(4-(((1-acetylpiperidin-4-yl)amino)methyl)-3-methoxyphenyl)-3-chloropyridin-4-yl)-2-chlorophenyl)picolinamide;
N-(2-chloro-3-(3-chloro-2-(3-methoxy-4-(((tetrahydro-2H-pyran-4-yl)amino)methyl)phenyl)pyridin-4-yl)phenyl)-5-(((tetrahydro-2H-pyran-4-yl)amino)methyl)picolinamide;
N-(2-chloro-3-(3-chloro-2-(3-methoxy-4-(((((S)-5-oxopyrrolidin-2-yl)methyl)amino)methyl)phenyl)pyridin-4-yl)phenyl)-5-(((((S)-5-oxopyrrolidin-2-yl)methyl)amino)methyl)picolinamide;
N-(2-chloro-3-(3-chloro-2-(3-methoxy-4-(((((S)-5-oxopyrrolidin-2-yl)methyl)amino)methyl)phenyl)pyridin-4-yl)phenyl)-5-(((((R)-5-oxopyrrolidin-2-yl)methyl)amino)methyl)picolinamide;
N-(2-chloro-3-(3-chloro-2-(3-methoxy-4-(((((R)-5-oxopyrrolidin-2-yl)methyl)amino)methyl)phenyl)pyridin-4-yl)phenyl)-5-(((((S)-5-oxopyrrolidin-2-yl)methyl)amino)methyl)picolinamide;
N-(2-chloro-3-(3-chloro-2-(3-methoxy-4-(((((R)-5-oxopyrrolidin-2-yl)methyl)amino)methyl)phenyl)pyridin-4-yl)phenyl)-5-(((((R)-5-oxopyrrolidin-2-yl)methyl)amino)methyl)picolinamide;
N-(2-chloro-3-(3-chloro-2-(4-(((2-hydroxyethyl)amino)methyl)-3-methoxyphenyl)pyridin-4-yl)phenyl)-5-(((2-hydroxyethyl)amino)methyl)picolinamide;
5-((6-acetyl-2,6-diazaspiro[3.3]heptan-2-yl)methyl)-N-(3-(2-(4-((6-acetyl-2,6-diazaspiro[3.3]heptan-2-yl)methyl)-3-methoxyphenyl)-3-chloropyridin-4-yl)-2-chlorophenyl)picolinamide;
N-(2-chloro-3-(3-chloro-2-(4-((((S)-2-hydroxypropyl)amino)methyl)-3-methoxyphenyl)pyridin-4-yl)phenyl)-5-((((S)-2-hydroxypropyl)amino)methyl)picolinamide;
N-(2-chloro-3-(3-chloro-2-(4-((((S)-2-hydroxypropyl)amino)methyl)-3-methoxyphenyl)pyridin-4-yl)phenyl)-5-((((R)-2-hydroxypropyl)amino)methyl)picolinamide;
N-(2-chloro-3-(3-chloro-2-(4-((((R)-2-hydroxypropyl)amino)methyl)-3-methoxyphenyl)pyridin-4-yl)phenyl)-5-((((S)-2-hydroxypropyl)amino)methyl)picolinamide;
N-(2-chloro-3-(3-chloro-2-(4-((((R)-2-hydroxypropyl)amino)methyl)-3-methoxyphenyl)pyridin-4-yl)phenyl)-5-((((R)-2-hydroxypropyl)amino)methyl)picolinamide;
1-(6-((6-(2-chloro-3-(3-chloro-2-(3-methoxy-4-((6-(3-methoxypropanoyl)-2,6-diazaspiro[3.3]heptan-2-yl)methyl)phenyl)pyridin-4-yl)phenyl)-2-methoxypyridin-3-yl)methyl)-2,6-diazaspiro[3.3]heptan-2-yl)-3-methoxypropan-1-one;
2-((2'-(3-(5-(((1-acetylpiperidin-4-yl)amino)methyl)-6-methoxypyridin-2-yl)-2-chlorophenyl)-3'-chloro-6-methoxy-[2,4'-bipyridin]-5-yl)methyl)-2,6-diazaspiro[3.4]octan-7-one;
2-((3'-chloro-2'-(2-chloro-3-(5-((4-hydroxypiperidin-1-yl)methyl)-6-methoxypyridin-2-yl)phenyl)-6-methoxy-[2,4'-bipyridin]-5-yl)methyl)-2,6-diazaspiro[3.4]octan-7-one;
2-((6-(3-(5-(((1-acetylpiperidin-4-yl)amino)methyl)-3'-chloro-6-methoxy-[2,4'-bipyridin]-2'-yl)-2-chlorophenyl)-2-methoxypyridin-3-yl)methyl)-2,6-diazaspiro[3.4]octan-7-one;
2-(4-(3-chloro-4-(2-chloro-3-(6-methoxy-5-(((tetrahydro-2H-pyran-4-yl)amino)methyl)pyridin-2-yl)phenyl)pyridin-2-yl)-2-(difluoromethoxy)benzyl)-2,6-diazaspiro[3.4]octan-7-one;
2-(4-(4-(3-(5-(((1-acetylpiperidin-4-yl)amino)methyl)-6-methoxypyridin-2-yl)-2-chlorophenyl)-3-chloropyridin-2-yl)-2-(difluoromethoxy)benzyl)-2,6-diazaspiro[3.4]octan-7-one;
2-((6-(2-chloro-3-(3-chloro-2-(3-(difluoromethoxy)-4-(((tetrahydro-2H-pyran-4-yl)amino)methyl)phenyl)pyridin-4-yl)phenyl)-2-methoxypyridin-3-yl)methyl)-2,6-diazaspiro[3.4]octan-7-one;
(S)-5-((((6-(((S)-4-(3'-chloro-6-methoxy-5-(((tetrahydro-2H-pyran-4-yl)amino)methyl)-[2,4'-bipyridin]-2'-yl)-2,3-dihydro-1H-inden-1-yl)oxy)-2-methoxy-5-(trifluoromethyl)pyridin-3-yl)methyl)amino)methyl)pyrrolidin-2-one;
(R)-5-((((6-(((S)-4-(3'-chloro-6-methoxy-5-(((tetrahydro-2H-pyran-4-yl)amino)methyl)-[2,4'-bipyridin]-2'-yl)-2,3-dihydro-1H-inden-1-yl)oxy)-2-methoxy-5-(trifluoromethyl)pyridin-3-yl)methyl)amino)methyl)pyrrolidin-2-one;
(S)-5-((((6-(((R)-4-(3'-chloro-6-methoxy-5-(((tetrahydro-2H-pyran-4-yl)amino)methyl)-[2,4'-bipyridin]-2'-yl)-2,3-dihydro-1H-inden-1-yl)oxy)-2-methoxy-5-(trifluoromethyl)pyridin-3-yl)methyl)amino)methyl)pyrrolidin-2-one;
(R)-5-((((6-(((R)-4-(3'-chloro-6-methoxy-5-(((tetrahydro-2H-pyran-4-yl)amino)methyl)-[2,4'-bipyridin]-2'-yl)-2,3-dihydro-1H-inden-1-yl)oxy)-2-methoxy-5-(trifluoromethyl)pyridin-3-yl)methyl)amino)methyl)pyrrolidin-2-one;
2-(4-(3-chloro-4-(2-chloro-3-(6-methoxy-5-((6-(2-methoxyacetyl)-2,6-diazaspiro[3.3]heptan-2-yl)methyl)pyridin-2-yl)phenyl)pyridin-2-yl)-2-methoxybenzyl)-2,6-diazaspiro[3.4]octan-7-one;
2-(4-(4-(3-(5-((6-acetyl-2,6-diazaspiro[3.3]heptan-2-yl)methyl)-6-methoxypyridin-2-yl)-2-chlorophenyl)-3-chloropyridin-2-yl)-2-(difluoromethoxy)benzyl)-2,6-diazaspiro[3.4]octan-7-one;
1-(6-((6-(2-chloro-3-(3-chloro-2-(3-(difluoromethoxy)-4-(((tetrahydro-2H-pyran-4-yl)amino)methyl)phenyl)pyridin-4-yl)phenyl)-2-methoxypyridin-3-yl)methyl)-2,6-diazaspiro[3.3]heptan-2-yl)ethan-1-one;
1-(4-((4-(4-(3-(5-((6-acetyl-2,6-diazaspiro[3.3]heptan-2-yl)methyl)-6-methoxypyridin-2-yl)-2-chlorophenyl)-3-chloropyridin-2-yl)-2-(difluoromethoxy)benzyl)amino)piperidin-1-yl)ethan-1-one;
1-(6-((6-(3-(2-(4-((6-acetyl-2,6-diazaspiro[3.3]heptan-2-yl)methyl)-3-methoxyphenyl)-3-chloropyridin-4-yl)-2-chlorophenyl)-2-methoxypyridin-3-yl)methyl)-2,6-diazaspiro[3.3]heptan-2-yl)-2-methoxyethan-1-one;
1-(2-((6-(2-chloro-3-(3-chloro-2-(4-((4-hydroxypiperidin-1-yl)methyl)-3-methoxyphenyl)pyridin-4-yl)phenyl)-2-methoxypyridin-3-yl)methyl)-2,6-diazaspiro[3.4]octan-6-yl)ethan-1-one;
1-(2-(4-(3-chloro-4-(2-chloro-3-(5-((4-hydroxypiperidin-1-yl)methyl)-6-methoxypyridin-2-yl)phenyl)pyridin-2-yl)-2-methoxybenzyl)-2,6-diazaspiro[3.4]octan-6-yl)ethan-1-one;
N-(2-chloro-3-(3-chloro-2-(3-methoxy-4-((methylamino)methyl)phenyl)pyridin-4-yl)phenyl)-5-((methylamino)methyl)picolinamide;
5-(((2-oxaspiro[3.3]heptan-6-yl)amino)methyl)-N-(3-(2-(4-(((2-oxaspiro[3.3]heptan-6-yl)amino)methyl)-3-methoxyphenyl)-3-chloropyridin-4-yl)-2-chlorophenyl)picolinamide;
N-(3-(3-chloro-2-(3-methoxy-4-((methylamino)methyl)phenyl)pyridin-4-yl)-2-methylphenyl)-5-((methylamino)methyl)picolinamide;
(S)-5-((((6-(2-chloro-3-(3-chloro-2-(2-((3-hydroxy-3-methylazetidin-1-yl)methyl)-4-methoxypyrrolo[2,1-f][1,2,4]triazin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-((3-hydroxy-3-methylazetidin-1-yl)methyl)-4-methoxypyrrolo[2,1-f][1,2,4]triazin-6-yl)pyridin-4-yl)phenyl)-2-methoxypyridin-3-yl)methyl)amino)methyl)pyrrolidin-2-one;
1-(4-(((6-(3-(2-(2-((2-oxa-6-azaspiro[3.3]heptan-6-yl)methyl)-8-methoxy-[1,2,4]triazolo[1,5-a]pyridin-6-yl)-3-chloropyridin-4-yl)-2-chlorophenyl)-2-methoxypyridin-3-yl)methyl)amino)piperidin-1-yl)ethan-1-one;
1-(4-(((6-(2-chloro-3-(3-chloro-2-(2-((((1-hydroxycyclopropyl)methyl)amino)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one;
N-(3-(2-(4-(((1-acetylpiperidin-4-yl)amino)methyl)-3-methoxyphenyl)-3-chloropyridin-4-yl)-2-methylphenyl)-5-(((2-hydroxyethyl)amino)methyl)picolinamide;
5-((2-oxa-6-azaspiro[3.3]heptan-6-yl)methyl)-N-(3-(2-(4-((2-oxa-6-azaspiro[3.3]heptan-6-yl)methyl)-3-methoxyphenyl)-3-chloropyridin-4-yl)-2-chlorophenyl)picolinamide;
N-(2-chloro-3-(3-chloro-2-(4-(((3-fluoropropyl)amino)methyl)-3-methoxyphenyl)pyridin-4-yl)phenyl)-5-(((3-fluoropropyl)amino)methyl)picolinamide;
N-(3-(3-chloro-2-(3-methoxy-4-(((tetrahydro-2H-pyran-4-yl)amino)methyl)phenyl)pyridin-4-yl)-2-methylphenyl)-5-(((2-hydroxyethyl)amino)methyl)picolinamide;
1-(((6-(((S)-5-(3'-chloro-6-methoxy-5-(((((S)-5-oxopyrrolidin-2-yl)methyl)amino)methyl)-[2,4'-bipyridin]-2'-yl)-1,2,3,4-tetrahydronaphthalen-1-yl)amino)-2-methoxy-5-(trifluoromethyl)pyridin-3-yl)methyl)amino)cyclopropane-1-carboxylic acid;
1-(((6-(((R)-5-(3'-chloro-6-methoxy-5-(((((S)-5-oxopyrrolidin-2-yl)methyl)amino)methyl)-[2,4'-bipyridin]-2'-yl)-1,2,3,4-tetrahydronaphthalen-1-yl)amino)-2-methoxy-5-(trifluoromethyl)pyridin-3-yl)methyl)amino)cyclopropane-1-carboxylic acid;
1-(((6-(((S)-5-(3'-chloro-6-methoxy-5-(((((R)-5-oxopyrrolidin-2-yl)methyl)amino)methyl)-[2,4'-bipyridin]-2'-yl)-1,2,3,4-tetrahydronaphthalen-1-yl)amino)-2-methoxy-5-(trifluoromethyl)pyridin-3-yl)methyl)amino)cyclopropane-1-carboxylic acid;
1-(((6-(((R)-5-(3'-chloro-6-methoxy-5-(((((R)-5-oxopyrrolidin-2-yl)methyl)amino)methyl)-[2,4'-bipyridin]-2'-yl)-1,2,3,4-tetrahydronaphthalen-1-yl)amino)-2-methoxy-5-(trifluoromethyl)pyridin-3-yl)methyl)amino)cyclopropane-1-carboxylic acid;
1-(((6-(((S)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)cyclopropane-1-carboxylic acid;
1-(((6-(((R)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)cyclopropane-1-carboxylic acid;
1-(((6-(((S)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)cyclopropane-1-carboxylic acid;
1-(((6-(((R)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)cyclopropane-1-carboxylic acid;
5-(((2-acetyl-2-azaspiro[3.3]heptan-6-yl)amino)methyl)-N-(3-(2-(4-(((2-acetyl-2-azaspiro[3.3]heptan-6-yl)amino)methyl)-3-methoxyphenyl)-3-chloropyridin-4-yl)-2-chlorophenyl)picolinamide;
(S)-5-((((3'-chloro-2'-((S)-5-((5-((((S)-2-hydroxypropyl)amino)methyl)-6-methoxy-3-(trifluoromethyl)pyridin-2-yl)amino)-5,6,7,8-tetrahydronaphthalen-1-yl)-6-methoxy-[2,4'-bipyridin]-5-yl)methyl)amino)methyl)pyrrolidin-2-one;
(S)-5-((((3'-chloro-2'-((R)-5-((5-((((S)-2-hydroxypropyl)amino)methyl)-6-methoxy-3-(trifluoromethyl)pyridin-2-yl)amino)-5,6,7,8-tetrahydronaphthalen-1-yl)-6-methoxy-[2,4'-bipyridin]-5-yl)methyl)amino)methyl)pyrrolidin-2-one;
(S)-5-((((3'-chloro-2'-((S)-5-((5-((((R)-2-hydroxypropyl)amino)methyl)-6-methoxy-3-(trifluoromethyl)pyridin-2-yl)amino)-5,6,7,8-tetrahydronaphthalen-1-yl)-6-methoxy-[2,4'-bipyridin]-5-yl)methyl)amino)methyl)pyrrolidin-2-one;
(S)-5-((((3'-chloro-2'-((R)-5-((5-((((R)-2-hydroxypropyl)amino)methyl)-6-methoxy-3-(trifluoromethyl)pyridin-2-yl)amino)-5,6,7,8-tetrahydronaphthalen-1-yl)-6-methoxy-[2,4'-bipyridin]-5-yl)methyl)amino)methyl)pyrrolidin-2-one;
(R)-5-((((3'-chloro-2'-((S)-5-((5-((((S)-2-hydroxypropyl)amino)methyl)-6-methoxy-3-(trifluoromethyl)pyridin-2-yl)amino)-5,6,7,8-tetrahydronaphthalen-1-yl)-6-methoxy-[2,4'-bipyridin]-5-yl)methyl)amino)methyl)pyrrolidin-2-one;
(R)-5-((((3'-chloro-2'-((R)-5-((5-((((S)-2-hydroxypropyl)amino)methyl)-6-methoxy-3-(trifluoromethyl)pyridin-2-yl)amino)-5,6,7,8-tetrahydronaphthalen-1-yl)-6-methoxy-[2,4'-bipyridin]-5-yl)methyl)amino)methyl)pyrrolidin-2-one;
(R)-5-((((3'-chloro-2'-((S)-5-((5-((((R)-2-hydroxypropyl)amino)methyl)-6-methoxy-3-(trifluoromethyl)pyridin-2-yl)amino)-5,6,7,8-tetrahydronaphthalen-1-yl)-6-methoxy-[2,4'-bipyridin]-5-yl)methyl)amino)methyl)pyrrolidin-2-one;
(R)-5-((((3'-chloro-2'-((R)-5-((5-((((R)-2-hydroxypropyl)amino)methyl)-6-methoxy-3-(trifluoromethyl)pyridin-2-yl)amino)-5,6,7,8-tetrahydronaphthalen-1-yl)-6-methoxy-[2,4'-bipyridin]-5-yl)methyl)amino)methyl)pyrrolidin-2-one;
(S)-N-(2-chloro-3-(5-chloro-6-(3-methoxy-4-((((5-oxopyrrolidin-2-yl)methyl)amino)methyl)phenyl)pyrimidin-4-yl)phenyl)-1,3-dimethyl-2,4-dioxo-1,2,3,4-tetrahydropyrimidine-5-carboxamide;
(R)-N-(2-chloro-3-(5-chloro-6-(3-methoxy-4-((((5-oxopyrrolidin-2-yl)methyl)amino)methyl)phenyl)pyrimidin-4-yl)phenyl)-1,3-dimethyl-2,4-dioxo-1,2,3,4-tetrahydropyrimidine-5-carboxamide;
(S)-5-((((3'-chloro-6-methoxy-2'-((S)-5-((6-methoxy-5-(((((S)-5-oxopyrrolidin-2-yl)methyl)amino)methyl)-3-(trifluoromethyl)pyridin-2-yl)amino)-5,6,7,8-tetrahydronaphthalen-1-yl)-[2,4'-bipyridin]-5-yl)methyl)amino)methyl)pyrrolidin-2-one;
(S)-5-((((3'-chloro-6-methoxy-2'-((R)-5-((6-methoxy-5-(((((S)-5-oxopyrrolidin-2-yl)methyl)amino)methyl)-3-(trifluoromethyl)pyridin-2-yl)amino)-5,6,7,8-tetrahydronaphthalen-1-yl)-[2,4'-bipyridin]-5-yl)methyl)amino)methyl)pyrrolidin-2-one;
(S)-5-((((3'-chloro-6-methoxy-2'-((S)-5-((6-methoxy-5-(((((R)-5-oxopyrrolidin-2-yl)methyl)amino)methyl)-3-(trifluoromethyl)pyridin-2-yl)amino)-5,6,7,8-tetrahydronaphthalen-1-yl)-[2,4'-bipyridin]-5-yl)methyl)amino)methyl)pyrrolidin-2-one;
(S)-5-((((3'-chloro-6-methoxy-2'-((R)-5-((6-methoxy-5-(((((R)-5-oxopyrrolidin-2-yl)methyl)amino)methyl)-3-(trifluoromethyl)pyridin-2-yl)amino)-5,6,7,8-tetrahydronaphthalen-1-yl)-[2,4'-bipyridin]-5-yl)methyl)amino)methyl)pyrrolidin-2-one;
(R)-5-((((3'-chloro-6-methoxy-2'-((S)-5-((6-methoxy-5-(((((S)-5-oxopyrrolidin-2-yl)methyl)amino)methyl)-3-(trifluoromethyl)pyridin-2-yl)amino)-5,6,7,8-tetrahydronaphthalen-1-yl)-[2,4'-bipyridin]-5-yl)methyl)amino)methyl)pyrrolidin-2-one;
(R)-5-((((3'-chloro-6-methoxy-2'-((R)-5-((6-methoxy-5-(((((S)-5-oxopyrrolidin-2-yl)methyl)amino)methyl)-3-(trifluoromethyl)pyridin-2-yl)amino)-5,6,7,8-tetrahydronaphthalen-1-yl)-[2,4'-bipyridin]-5-yl)methyl)amino)methyl)pyrrolidin-2-one;
(R)-5-((((3'-chloro-6-methoxy-2'-((S)-5-((6-methoxy-5-(((((R)-5-oxopyrrolidin-2-yl)methyl)amino)methyl)-3-(trifluoromethyl)pyridin-2-yl)amino)-5,6,7,8-tetrahydronaphthalen-1-yl)-[2,4'-bipyridin]-5-yl)methyl)amino)methyl)pyrrolidin-2-one;
(R)-5-((((3'-chloro-6-methoxy-2'-((R)-5-((6-methoxy-5-(((((R)-5-oxopyrrolidin-2-yl)methyl)amino)methyl)-3-(trifluoromethyl)pyridin-2-yl)amino)-5,6,7,8-tetrahydronaphthalen-1-yl)-[2,4'-bipyridin]-5-yl)methyl)amino)methyl)pyrrolidin-2-one;
(S)-5-((((6-(((S)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)methyl)pyrrolidin-2-one;
(S)-5-((((6-(((R)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)methyl)pyrrolidin-2-one;
(S)-5-((((6-(((S)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)methyl)pyrrolidin-2-one;
(S)-5-((((6-(((R)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)methyl)pyrrolidin-2-one;
(R)-5-((((6-(((S)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)methyl)pyrrolidin-2-one;
(R)-5-((((6-(((R)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)methyl)pyrrolidin-2-one;
(R)-5-((((6-(((S)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)methyl)pyrrolidin-2-one;
(R)-5-((((6-(((R)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)methyl)pyrrolidin-2-one;
2-(4-(5-chloro-6-(2-chloro-3-(5-(((2-hydroxyethyl)amino)methyl)-6-methoxypyridin-2-yl)phenyl)pyrimidin-4-yl)-2-methoxybenzyl)-2,6-diazaspiro[3.4]octan-7-one;
(S)-N-(2-chloro-3-(4'-chloro-6-methoxy-5-((((5-oxopyrrolidin-2-yl)methyl)amino)methyl)-[2,3'-bipyridin]-5'-yl)phenyl)-1,3-dimethyl-2,4-dioxo-1,2,3,4-tetrahydropyrimidine-5-carboxamide;
(R)-N-(2-chloro-3-(4'-chloro-6-methoxy-5-((((5-oxopyrrolidin-2-yl)methyl)amino)methyl)-[2,3'-bipyridin]-5'-yl)phenyl)-1,3-dimethyl-2,4-dioxo-1,2,3,4-tetrahydropyrimidine-5-carboxamide;
1-(4-(((6-(2-chloro-3-(3-chloro-2-(2-(((3-fluoropropyl)amino)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one;
1-(4-(((6-(2-chloro-3-(3-chloro-2-(2-(((2-hydroxy-2-methylpropyl)amino)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one;
1-(4-(((6-(2-chloro-3-(3-chloro-2-(2-(((2-hydroxyethyl)(methyl)amino)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one;
(S)-5-((((6-(2-chloro-3-(5-methoxy-6-(3-methoxy-4-(((((S)-5-oxopyrrolidin-2-yl)methyl)amino)methyl)phenyl)pyrimidin-4-yl)phenyl)-2-methoxypyridin-3-yl)methyl)amino)methyl)pyrrolidin-2-one;
(S)-5-((((6-(2-chloro-3-(5-methoxy-6-(3-methoxy-4-(((((R)-5-oxopyrrolidin-2-yl)methyl)amino)methyl)phenyl)pyrimidin-4-yl)phenyl)-2-methoxypyridin-3-yl)methyl)amino)methyl)pyrrolidin-2-one;
(R)-5-((((6-(2-chloro-3-(5-methoxy-6-(3-methoxy-4-(((((S)-5-oxopyrrolidin-2-yl)methyl)amino)methyl)phenyl)pyrimidin-4-yl)phenyl)-2-methoxypyridin-3-yl)methyl)amino)methyl)pyrrolidin-2-one;
(R)-5-((((6-(2-chloro-3-(5-methoxy-6-(3-methoxy-4-(((((R)-5-oxopyrrolidin-2-yl)methyl)amino)methyl)phenyl)pyrimidin-4-yl)phenyl)-2-methoxypyridin-3-yl)methyl)amino)methyl)pyrrolidin-2-one;
1-(4-(((6-(3-(6-(4-(((1-acetylpiperidin-4-yl)amino)methyl)-3-methoxyphenyl)-5-methoxypyrimidin-4-yl)-2-chlorophenyl)-2-methoxypyridin-3-yl)methyl)amino)piperidin-1-yl)ethan-1-one;
(S)-N-(2-chloro-3-(3'-chloro-6-methoxy-5-((((5-oxopyrrolidin-2-yl)methyl)amino)methyl)-[2,4'-bipyridin]-2'-yl)phenyl)-1,3-dimethyl-2,4-dioxo-1,2,3,4-tetrahydropyrimidine-5-carboxamide;
(R)-N-(2-chloro-3-(3'-chloro-6-methoxy-5-((((5-oxopyrrolidin-2-yl)methyl)amino)methyl)-[2,4'-bipyridin]-2'-yl)phenyl)-1,3-dimethyl-2,4-dioxo-1,2,3,4-tetrahydropyrimidine-5-carboxamide;
N-(2-chloro-3-(3'-chloro-6-methoxy-5-((7-oxo-2,6-diazaspiro[3.4]octan-2-yl)methyl)-[2,4'-bipyridin]-2'-yl)phenyl)-1,3-dimethyl-2,4-dioxo-1,2,3,4-tetrahydropyrimidine-5-carboxamide;
2-((6-(((S)-5-(3'-chloro-6-methoxy-5-(((((S)-5-oxopyrrolidin-2-yl)methyl)amino)methyl)-[2,4'-bipyridin]-2'-yl)-1,2,3,4-tetrahydronaphthalen-1-yl)amino)-2-methoxy-5-(trifluoromethyl)pyridin-3-yl)methyl)-2,6-diazaspiro[3.4]octan-7-one;
2-((6-(((R)-5-(3'-chloro-6-methoxy-5-(((((S)-5-oxopyrrolidin-2-yl)methyl)amino)methyl)-[2,4'-bipyridin]-2'-yl)-1,2,3,4-tetrahydronaphthalen-1-yl)amino)-2-methoxy-5-(trifluoromethyl)pyridin-3-yl)methyl)-2,6-diazaspiro[3.4]octan-7-one;
2-((6-(((S)-5-(3'-chloro-6-methoxy-5-(((((R)-5-oxopyrrolidin-2-yl)methyl)amino)methyl)-[2,4'-bipyridin]-2'-yl)-1,2,3,4-tetrahydronaphthalen-1-yl)amino)-2-methoxy-5-(trifluoromethyl)pyridin-3-yl)methyl)-2,6-diazaspiro[3.4]octan-7-one;
2-((6-(((R)-5-(3'-chloro-6-methoxy-5-(((((R)-5-oxopyrrolidin-2-yl)methyl)amino)methyl)-[2,4'-bipyridin]-2'-yl)-1,2,3,4-tetrahydronaphthalen-1-yl)amino)-2-methoxy-5-(trifluoromethyl)pyridin-3-yl)methyl)-2,6-diazaspiro[3.4]octan-7-one;
N-(3-(5-(((1-acetylpiperidin-4-yl)amino)methyl)-3'-chloro-6-methoxy-[2,4'-bipyridin]-2'-yl)-2-chlorophenyl)-1,3-dimethyl-2,4-dioxo-1,2,3,4-tetrahydropyrimidine-5-carboxamide;
N-(2-chloro-3-(4'-chloro-6-methoxy-5-((7-oxo-2,6-diazaspiro[3.4]octan-2-yl)methyl)-[2,3'-bipyridin]-5'-yl)phenyl)-1,3-dimethyl-2,4-dioxo-1,2,3,4-tetrahydropyrimidine-5-carboxamide;
N-(3-(5-(((1-acetylpiperidin-4-yl)amino)methyl)-4'-chloro-6-methoxy-[2,3'-bipyridin]-5'-yl)-2-chlorophenyl)-1,3-dimethyl-2,4-dioxo-1,2,3,4-tetrahydropyrimidine-5-carboxamide;
2-((6-(((S)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)-2,6-diazaspiro[3.4]octan-7-one;
2-((6-(((R)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)-2,6-diazaspiro[3.4]octan-7-one;
2-((6-(((S)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)-2,6-diazaspiro[3.4]octan-7-one;
2-((6-(((R)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)-2,6-diazaspiro[3.4]octan-7-one;
N-(3-(3-chloro-2-(3-(difluoromethoxy)-4-(((((S)-5-oxopyrrolidin-2-yl)methyl)amino)methyl)phenyl)pyridin-4-yl)-2-methylphenyl)-5-(((((S)-5-oxopyrrolidin-2-yl)methyl)amino)methyl)picolinamide;
N-(3-(3-chloro-2-(3-(difluoromethoxy)-4-(((((R)-5-oxopyrrolidin-2-yl)methyl)amino)methyl)phenyl)pyridin-4-yl)-2-methylphenyl)-5-(((((S)-5-oxopyrrolidin-2-yl)methyl)amino)methyl)picolinamide;
N-(3-(3-chloro-2-(3-(difluoromethoxy)-4-(((((S)-5-oxopyrrolidin-2-yl)methyl)amino)methyl)phenyl)pyridin-4-yl)-2-methylphenyl)-5-(((((R)-5-oxopyrrolidin-2-yl)methyl)amino)methyl)picolinamide;
N-(3-(3-chloro-2-(3-(difluoromethoxy)-4-(((((R)-5-oxopyrrolidin-2-yl)methyl)amino)methyl)phenyl)pyridin-4-yl)-2-methylphenyl)-5-(((((R)-5-oxopyrrolidin-2-yl)methyl)amino)methyl)picolinamide;
N-(3-(3-chloro-2-(3-fluoro-5-methoxy-4-(((((S)-5-oxopyrrolidin-2-yl)methyl)amino)methyl)phenyl)pyridin-4-yl)-2-methylphenyl)-5-(((((S)-5-oxopyrrolidin-2-yl)methyl)amino)methyl)picolinamide;
N-(3-(3-chloro-2-(3-fluoro-5-methoxy-4-(((((R)-5-oxopyrrolidin-2-yl)methyl)amino)methyl)phenyl)pyridin-4-yl)-2-methylphenyl)-5-(((((S)-5-oxopyrrolidin-2-yl)methyl)amino)methyl)picolinamide;
N-(3-(3-chloro-2-(3-fluoro-5-methoxy-4-(((((S)-5-oxopyrrolidin-2-yl)methyl)amino)methyl)phenyl)pyridin-4-yl)-2-methylphenyl)-5-(((((R)-5-oxopyrrolidin-2-yl)methyl)amino)methyl)picolinamide;
N-(3-(3-chloro-2-(3-fluoro-5-methoxy-4-(((((R)-5-oxopyrrolidin-2-yl)methyl)amino)methyl)phenyl)pyridin-4-yl)-2-methylphenyl)-5-(((((R)-5-oxopyrrolidin-2-yl)methyl)amino)methyl)picolinamide;
2-(4-(6-(2-chloro-3-(6-methoxy-5-((7-oxo-2,6-diazaspiro[3.4]octan-2-yl)methyl)pyridin-2-yl)phenyl)-5-methoxypyrimidin-4-yl)-2-methoxybenzyl)-2,6-diazaspiro[3.4]octan-7-one;
2-((4-(6-(2-chloro-3-(5-(((2-hydroxyethyl)amino)methyl)-6-methoxypyridin-2-yl)phenyl)-5-methoxypyrimidin-4-yl)-2-methoxybenzyl)amino)ethan-1-ol;
(S)-N-(2-chloro-3-(4'-chloro-5-(((2-hydroxypropyl)amino)methyl)-6-methoxy-[2,3'-bipyridin]-5'-yl)phenyl)-1,3-dimethyl-2,4-dioxo-1,2,3,4-tetrahydropyrimidine-5-carboxamide;
(R)-N-(2-chloro-3-(4'-chloro-5-(((2-hydroxypropyl)amino)methyl)-6-methoxy-[2,3'-bipyridin]-5'-yl)phenyl)-1,3-dimethyl-2,4-dioxo-1,2,3,4-tetrahydropyrimidine-5-carboxamide;
N-(3-(5-((6-acetyl-2,6-diazaspiro[3.3]heptan-2-yl)methyl)-4'-chloro-6-methoxy-[2,3'-bipyridin]-5'-yl)-2-chlorophenyl)-1,3-dimethyl-2,4-dioxo-1,2,3,4-tetrahydropyrimidine-5-carboxamide;
N-(3-(5-((6-acetyl-2,6-diazaspiro[3.3]heptan-2-yl)methyl)-3'-chloro-6-methoxy-[2,4'-bipyridin]-2'-yl)-2-chlorophenyl)-1,3-dimethyl-2,4-dioxo-1,2,3,4-tetrahydropyrimidine-5-carboxamide;
(S)-N-(5-(3'-chloro-6-methoxy-5-((methylamino)methyl)-[2,4'-bipyridin]-2'-yl)-1,2,3,4-tetrahydronaphthalen-1-yl)-6-methoxy-5-((methylamino)methyl)-3-(trifluoromethyl)pyridin-2-amine;
(R)-N-(5-(3'-chloro-6-methoxy-5-((methylamino)methyl)-[2,4'-bipyridin]-2'-yl)-1,2,3,4-tetrahydronaphthalen-1-yl)-6-methoxy-5-((methylamino)methyl)-3-(trifluoromethyl)pyridin-2-amine;
N-(3-(2-(4-(((1-acetylpiperidin-4-yl)amino)methyl)-3-methoxyphenyl)-3-chloropyridin-4-yl)-2-chlorophenyl)-5-(((2-hydroxyethyl)amino)methyl)picolinamide;
N-((6-(2-chloro-3-(5-methoxy-6-(3-methoxy-4-(((tetrahydro-2H-pyran-4-yl)amino)methyl)phenyl)pyrimidin-4-yl)phenyl)-2-methoxypyridin-3-yl)methyl)tetrahydro-2H-pyran-4-amine;
(S)-N-(3-(3-chloro-2-(3-methoxy-4-((((5-oxopyrrolidin-2-yl)methyl)amino)methyl)phenyl)pyridin-4-yl)-2-methylphenyl)-5-(((2-hydroxyethyl)amino)methyl)picolinamide;
(R)-N-(3-(3-chloro-2-(3-methoxy-4-((((5-oxopyrrolidin-2-yl)methyl)amino)methyl)phenyl)pyridin-4-yl)-2-methylphenyl)-5-(((2-hydroxyethyl)amino)methyl)picolinamide;
(S)-5-(((4-(3-chloro-4-((S)-5-((5-((((S)-2-hydroxypropyl)amino)methyl)-6-methoxy-3-(trifluoromethyl)pyridin-2-yl)amino)-5,6,7,8-tetrahydronaphthalen-1-yl)pyridin-2-yl)-2-methoxybenzyl)amino)methyl)pyrrolidin-2-one;
(S)-5-(((4-(3-chloro-4-((R)-5-((5-((((S)-2-hydroxypropyl)amino)methyl)-6-methoxy-3-(trifluoromethyl)pyridin-2-yl)amino)-5,6,7,8-tetrahydronaphthalen-1-yl)pyridin-2-yl)-2-methoxybenzyl)amino)methyl)pyrrolidin-2-one;
(S)-5-(((4-(3-chloro-4-((S)-5-((5-((((R)-2-hydroxypropyl)amino)methyl)-6-methoxy-3-(trifluoromethyl)pyridin-2-yl)amino)-5,6,7,8-tetrahydronaphthalen-1-yl)pyridin-2-yl)-2-methoxybenzyl)amino)methyl)pyrrolidin-2-one;
(S)-5-(((4-(3-chloro-4-((R)-5-((5-((((R)-2-hydroxypropyl)amino)methyl)-6-methoxy-3-(trifluoromethyl)pyridin-2-yl)amino)-5,6,7,8-tetrahydronaphthalen-1-yl)pyridin-2-yl)-2-methoxybenzyl)amino)methyl)pyrrolidin-2-one;
(R)-5-(((4-(3-chloro-4-((S)-5-((5-((((S)-2-hydroxypropyl)amino)methyl)-6-methoxy-3-(trifluoromethyl)pyridin-2-yl)amino)-5,6,7,8-tetrahydronaphthalen-1-yl)pyridin-2-yl)-2-methoxybenzyl)amino)methyl)pyrrolidin-2-one;
(R)-5-(((4-(3-chloro-4-((R)-5-((5-((((S)-2-hydroxypropyl)amino)methyl)-6-methoxy-3-(trifluoromethyl)pyridin-2-yl)amino)-5,6,7,8-tetrahydronaphthalen-1-yl)pyridin-2-yl)-2-methoxybenzyl)amino)methyl)pyrrolidin-2-one;
(R)-5-(((4-(3-chloro-4-((S)-5-((5-((((R)-2-hydroxypropyl)amino)methyl)-6-methoxy-3-(trifluoromethyl)pyridin-2-yl)amino)-5,6,7,8-tetrahydronaphthalen-1-yl)pyridin-2-yl)-2-methoxybenzyl)amino)methyl)pyrrolidin-2-one;
(R)-5-(((4-(3-chloro-4-((R)-5-((5-((((R)-2-hydroxypropyl)amino)methyl)-6-methoxy-3-(trifluoromethyl)pyridin-2-yl)amino)-5,6,7,8-tetrahydronaphthalen-1-yl)pyridin-2-yl)-2-methoxybenzyl)amino)methyl)pyrrolidin-2-one;
2-(4-(5-chloro-6-(2-chloro-3-(5-((((1s,3s)-3-hydroxycyclobutyl)amino)methyl)-6-methoxypyridin-2-yl)phenyl)pyrimidin-4-yl)-2-methoxybenzyl)-2,6-diazaspiro[3.4]octan-7-one;
2-(4-(5-chloro-6-(2-chloro-3-(5-((((1s,3r)-3-hydroxycyclobutyl)amino)methyl)-6-methoxypyridin-2-yl)phenyl)pyrimidin-4-yl)-2-methoxybenzyl)-2,6-diazaspiro[3.4]octan-7-one;
2-(4-(5-chloro-6-(2-chloro-3-(5-((((1r,3s)-3-hydroxycyclobutyl)amino)methyl)-6-methoxypyridin-2-yl)phenyl)pyrimidin-4-yl)-2-methoxybenzyl)-2,6-diazaspiro[3.4]octan-7-one;
2-(4-(5-chloro-6-(2-chloro-3-(5-((((1r,3r)-3-hydroxycyclobutyl)amino)methyl)-6-methoxypyridin-2-yl)phenyl)pyrimidin-4-yl)-2-methoxybenzyl)-2,6-diazaspiro[3.4]octan-7-one;
(S)-N-(2-chloro-3-(3-chloro-6'-methoxy-5'-((((5-oxopyrrolidin-2-yl)methyl)amino)methyl)-[2,2'-bipyridin]-4-yl)phenyl)-1,3-dimethyl-2,4-dioxo-1,2,3,4-tetrahydropyrimidine-5-carboxamide;
(R)-N-(2-chloro-3-(3-chloro-6'-methoxy-5'-((((5-oxopyrrolidin-2-yl)methyl)amino)methyl)-[2,2'-bipyridin]-4-yl)phenyl)-1,3-dimethyl-2,4-dioxo-1,2,3,4-tetrahydropyrimidine-5-carboxamide;
(S)-N-(2-chloro-3-(3-chloro-5'-(((2-hydroxypropyl)amino)methyl)-6'-methoxy-[2,2'-bipyridin]-4-yl)phenyl)-1,3-dimethyl-2,4-dioxo-1,2,3,4-tetrahydropyrimidine-5-carboxamide;
(R)-N-(2-chloro-3-(3-chloro-5'-(((2-hydroxypropyl)amino)methyl)-6'-methoxy-[2,2'-bipyridin]-4-yl)phenyl)-1,3-dimethyl-2,4-dioxo-1,2,3,4-tetrahydropyrimidine-5-carboxamide;
(S)-N-(5-(3-chloro-2-(3-methoxy-4-((methylamino)methyl)phenyl)pyridin-4-yl)-1,2,3,4-tetrahydronaphthalen-1-yl)-6-methoxy-5-((methylamino)methyl)-3-(trifluoromethyl)pyridin-2-amine;
(R)-N-(5-(3-chloro-2-(3-methoxy-4-((methylamino)methyl)phenyl)pyridin-4-yl)-1,2,3,4-tetrahydronaphthalen-1-yl)-6-methoxy-5-((methylamino)methyl)-3-(trifluoromethyl)pyridin-2-amine;
(S)-2-(4-(5-chloro-6-(2-chloro-3-(6-methoxy-5-((((5-oxopyrrolidin-2-yl)methyl)amino)methyl)pyridin-2-yl)phenyl)pyrimidin-4-yl)-2-methoxybenzyl)-2,6-diazaspiro[3.4]octan-7-one;
(R)-2-(4-(5-chloro-6-(2-chloro-3-(6-methoxy-5-((((5-oxopyrrolidin-2-yl)methyl)amino)methyl)pyridin-2-yl)phenyl)pyrimidin-4-yl)-2-methoxybenzyl)-2,6-diazaspiro[3.4]octan-7-one;
2-(4-(6-(2-chloro-3-(6-methoxy-5-((6-oxo-2,5-diazaspiro[3.4]octan-2-yl)methyl)pyridin-2-yl)phenyl)-5-methoxypyrimidin-4-yl)-2-methoxybenzyl)-2,5-diazaspiro[3.4]octan-6-one;
N-(3-(3-chloro-2-(3-methoxy-4-((7-oxo-2,6-diazaspiro[3.4]octan-2-yl)methyl)phenyl)pyridin-4-yl)-2-methylphenyl)-5-(((2-hydroxyethyl)amino)methyl)picolinamide;
(S)-N-(2-chloro-3-(3'-chloro-5-(((2-hydroxypropyl)amino)methyl)-6-methoxy-[2,4'-bipyridin]-2'-yl)phenyl)-1,3-dimethyl-2,4-dioxo-1,2,3,4-tetrahydropyrimidine-5-carboxamide;
(R)-N-(2-chloro-3-(3'-chloro-5-(((2-hydroxypropyl)amino)methyl)-6-methoxy-[2,4'-bipyridin]-2'-yl)phenyl)-1,3-dimethyl-2,4-dioxo-1,2,3,4-tetrahydropyrimidine-5-carboxamide;
(S)-N-(2-chloro-3-(5-chloro-6-(4-(((2-hydroxypropyl)amino)methyl)-3-methoxyphenyl)pyrimidin-4-yl)phenyl)-1,3-dimethyl-2,4-dioxo-1,2,3,4-tetrahydropyrimidine-5-carboxamide;
(R)-N-(2-chloro-3-(5-chloro-6-(4-(((2-hydroxypropyl)amino)methyl)-3-methoxyphenyl)pyrimidin-4-yl)phenyl)-1,3-dimethyl-2,4-dioxo-1,2,3,4-tetrahydropyrimidine-5-carboxamide;
(S)-N-(3-(2-(4-(((1-acetylpiperidin-4-yl)amino)methyl)-3-methoxyphenyl)-3-chloropyridin-4-yl)-2-methylphenyl)-5-((((5-oxopyrrolidin-2-yl)methyl)amino)methyl)picolinamide;
(R)-N-(3-(2-(4-(((1-acetylpiperidin-4-yl)amino)methyl)-3-methoxyphenyl)-3-chloropyridin-4-yl)-2-methylphenyl)-5-((((5-oxopyrrolidin-2-yl)methyl)amino)methyl)picolinamide;
(S)-N-(3-(3-chloro-2-(3-methoxy-4-(((tetrahydro-2H-pyran-4-yl)amino)methyl)phenyl)pyridin-4-yl)-2-methylphenyl)-5-((((5-oxopyrrolidin-2-yl)methyl)amino)methyl)picolinamide;
(R)-N-(3-(3-chloro-2-(3-methoxy-4-(((tetrahydro-2H-pyran-4-yl)amino)methyl)phenyl)pyridin-4-yl)-2-methylphenyl)-5-((((5-oxopyrrolidin-2-yl)methyl)amino)methyl)picolinamide;
(S)-N-(3-(3-chloro-2-(4-(((3-fluoropropyl)amino)methyl)-3-methoxyphenyl)pyridin-4-yl)-2-methylphenyl)-5-((((5-oxopyrrolidin-2-yl)methyl)amino)methyl)picolinamide;
(R)-N-(3-(3-chloro-2-(4-(((3-fluoropropyl)amino)methyl)-3-methoxyphenyl)pyridin-4-yl)-2-methylphenyl)-5-((((5-oxopyrrolidin-2-yl)methyl)amino)methyl)picolinamide;
(S)-N-(3-(3-chloro-2-(4-(((2-hydroxyethyl)amino)methyl)-3-methoxyphenyl)pyridin-4-yl)-2-methylphenyl)-5-((((5-oxopyrrolidin-2-yl)methyl)amino)methyl)picolinamide;
(R)-N-(3-(3-chloro-2-(4-(((2-hydroxyethyl)amino)methyl)-3-methoxyphenyl)pyridin-4-yl)-2-methylphenyl)-5-((((5-oxopyrrolidin-2-yl)methyl)amino)methyl)picolinamide;
(S)-N-(2-chloro-3-(3-chloro-2-(3-methoxy-4-((((5-oxopyrrolidin-2-yl)methyl)amino)methyl)phenyl)pyridin-4-yl)phenyl)-5-((methylamino)methyl)picolinamide;
(R)-N-(2-chloro-3-(3-chloro-2-(3-methoxy-4-((((5-oxopyrrolidin-2-yl)methyl)amino)methyl)phenyl)pyridin-4-yl)phenyl)-5-((methylamino)methyl)picolinamide;
(S)-6-((2-chloro-3-(3-chloro-2-(3-methoxy-4-((((5-oxopyrrolidin-2-yl)methyl)amino)methyl)phenyl)pyridin-4-yl)phenyl)carbamoyl)nicotinic acid;
(R)-6-((2-chloro-3-(3-chloro-2-(3-methoxy-4-((((5-oxopyrrolidin-2-yl)methyl)amino)methyl)phenyl)pyridin-4-yl)phenyl)carbamoyl)nicotinic acid;
2-((6-(3-chloro-4-(2-chloro-3-(5-((isopropylamino)methyl)-6-methoxypyridin-2-yl)phenyl)pyridin-2-yl)-8-methoxy-[1,2,4]triazolo[1,5-a]pyridin-2-yl)methyl)-2-azaspiro[3.3]heptan-6-ol;
N-(2-chloro-3-(3-chloro-2-(3-methoxy-4-((7-oxo-2,6-diazaspiro[3.4]octan-2-yl)methyl)phenyl)pyridin-4-yl)phenyl)-5-(((2-hydroxyethyl)amino)methyl)picolinamide;
(S)-N-(2-chloro-3-(3-chloro-2-(3-methoxy-4-((((5-oxopyrrolidin-2-yl)methyl)amino)methyl)phenyl)pyridin-4-yl)phenyl)-5-(((2-hydroxyethyl)amino)methyl)picolinamide;
(R)-N-(2-chloro-3-(3-chloro-2-(3-methoxy-4-((((5-oxopyrrolidin-2-yl)methyl)amino)methyl)phenyl)pyridin-4-yl)phenyl)-5-(((2-hydroxyethyl)amino)methyl)picolinamide;
(S)-N-(3-(3-chloro-2-(3-methoxy-4-((((5-oxopyrrolidin-2-yl)methyl)amino)methyl)phenyl)pyridin-4-yl)-2-methylphenyl)-5-((methylamino)methyl)picolinamide;
(R)-N-(3-(3-chloro-2-(3-methoxy-4-((((5-oxopyrrolidin-2-yl)methyl)amino)methyl)phenyl)pyridin-4-yl)-2-methylphenyl)-5-((methylamino)methyl)picolinamide;
N-(3-(2-(4-((6-acetyl-2,6-diazaspiro[3.3]heptan-2-yl)methyl)-3-methoxyphenyl)-3-chloropyridin-4-yl)-2-chlorophenyl)-5-(((2-hydroxyethyl)amino)methyl)picolinamide;
(1r,4r)-4-(((6-(3-chloro-4-(2-chloro-3-(5-((isopropylamino)methyl)-6-methoxypyridin-2-yl)phenyl)pyridin-2-yl)-8-methoxy-[1,2,4]triazolo[1,5-a]pyridin-2-yl)methyl)amino)cyclohexan-1-ol;
(1s,4r)-4-(((6-(3-chloro-4-(2-chloro-3-(5-((isopropylamino)methyl)-6-methoxypyridin-2-yl)phenyl)pyridin-2-yl)-8-methoxy-[1,2,4]triazolo[1,5-a]pyridin-2-yl)methyl)amino)cyclohexan-1-ol;
(1r,4s)-4-(((6-(3-chloro-4-(2-chloro-3-(5-((isopropylamino)methyl)-6-methoxypyridin-2-yl)phenyl)pyridin-2-yl)-8-methoxy-[1,2,4]triazolo[1,5-a]pyridin-2-yl)methyl)amino)cyclohexan-1-ol;
(1s,4s)-4-(((6-(3-chloro-4-(2-chloro-3-(5-((isopropylamino)methyl)-6-methoxypyridin-2-yl)phenyl)pyridin-2-yl)-8-methoxy-[1,2,4]triazolo[1,5-a]pyridin-2-yl)methyl)amino)cyclohexan-1-ol;
1-((3'-chloro-2'-(2-chloro-3-(5-((4-hydroxypiperidin-1-yl)methyl)-6-methoxypyridin-2-yl)phenyl)-6-methoxy-[2,4'-bipyridin]-5-yl)methyl)piperidin-4-ol;
1-(2-((6-(3-(5-((6-acetyl-2,6-diazaspiro[3.4]octan-2-yl)methyl)-3'-chloro-6-methoxy-[2,4'-bipyridin]-2'-yl)-2-chlorophenyl)-2-methoxypyridin-3-yl)methyl)-2,6-diazaspiro[3.4]octan-6-yl)ethan-1-one;
1-(4-(((6-(2-chloro-3-(3'-chloro-5-((4-hydroxypiperidin-1-yl)methyl)-6-methoxy-[2,4'-bipyridin]-2'-yl)phenyl)-2-methoxypyridin-3-yl)methyl)amino)piperidin-1-yl)ethan-1-one;
N-(3-(3-chloro-2-(3-fluoro-4-(((2-hydroxyethyl)amino)methyl)-5-methoxyphenyl)pyridin-4-yl)-2-methylphenyl)-5-(((2-hydroxyethyl)amino)methyl)picolinamide;
N-((6-(2-chloro-3-(3-chloro-2-(2-((isopropylamino)methyl)-8-methoxy-[1,2,4]triazolo[1,5-a]pyridin-6-yl)pyridin-4-yl)phenyl)-2-methoxypyridin-3-yl)methyl)propan-2-amine;
(S)-5-((((6-(3-chloro-4-(2-chloro-3-(5-((isopropylamino)methyl)-6-methoxypyridin-2-yl)phenyl)pyridin-2-yl)-8-methoxy-[1,2,4]triazolo[1,5-a]pyridin-2-yl)methyl)amino)methyl)pyrrolidin-2-one;
(R)-5-((((6-(3-chloro-4-(2-chloro-3-(5-((isopropylamino)methyl)-6-methoxypyridin-2-yl)phenyl)pyridin-2-yl)-8-methoxy-[1,2,4]triazolo[1,5-a]pyridin-2-yl)methyl)amino)methyl)pyrrolidin-2-one;
1-(4-(((6-(3-chloro-4-(2-chloro-3-(5-((isopropylamino)methyl)-6-methoxypyridin-2-yl)phenyl)pyridin-2-yl)-8-methoxy-[1,2,4]triazolo[1,5-a]pyridin-2-yl)methyl)amino)piperidin-1-yl)ethan-1-one;
N-(3-(3'-chloro-6-methoxy-5-(((tetrahydro-2H-pyran-4-yl)amino)methyl)-[2,4'-bipyridin]-2'-yl)-2-methylphenyl)-5-(((2-hydroxyethyl)amino)methyl)picolinamide;
1-(6-((6-(3-chloro-4-(2-chloro-3-(5-((isopropylamino)methyl)-6-methoxypyridin-2-yl)phenyl)pyridin-2-yl)-8-methoxy-[1,2,4]triazolo[1,5-a]pyridin-2-yl)methyl)-2,6-diazaspiro[3.3]heptan-2-yl)ethan-1-one;
N-(2-chloro-3-(3-chloro-2-(3-methoxy-4-(((tetrahydro-2H-pyran-4-yl)amino)methyl)phenyl)pyridin-4-yl)phenyl)-5-(((2-hydroxyethyl)amino)methyl)picolinamide;
N-(3-(5-(((1-acetylpiperidin-4-yl)amino)methyl)-3'-chloro-6-methoxy-[2,4'-bipyridin]-2'-yl)-2-methylphenyl)-5-(((2-hydroxyethyl)amino)methyl)picolinamide;
(S)-N-(3-(3-chloro-2-(3-(difluoromethoxy)-4-((((5-oxopyrrolidin-2-yl)methyl)amino)methyl)phenyl)pyridin-4-yl)-2-methylphenyl)-5-(((2-hydroxyethyl)amino)methyl)picolinamide;
(R)-N-(3-(3-chloro-2-(3-(difluoromethoxy)-4-((((5-oxopyrrolidin-2-yl)methyl)amino)methyl)phenyl)pyridin-4-yl)-2-methylphenyl)-5-(((2-hydroxyethyl)amino)methyl)picolinamide;
(S)-N-(3-(3'-chloro-6-methoxy-5-((((5-oxopyrrolidin-2-yl)methyl)amino)methyl)-[2,4'-bipyridin]-2'-yl)-2-methylphenyl)-5-(((2-hydroxyethyl)amino)methyl)picolinamide;
(R)-N-(3-(3'-chloro-6-methoxy-5-((((5-oxopyrrolidin-2-yl)methyl)amino)methyl)-[2,4'-bipyridin]-2'-yl)-2-methylphenyl)-5-(((2-hydroxyethyl)amino)methyl)picolinamide;
(R)-1-((3'-chloro-2'-(2-chloro-3-(5-(((R)-3-hydroxypyrrolidin-1-yl)methyl)-6-methoxypyridin-2-yl)phenyl)-6-methoxy-[2,4'-bipyridin]-5-yl)methyl)pyrrolidin-3-ol;
(R)-1-((3'-chloro-2'-(2-chloro-3-(5-(((S)-3-hydroxypyrrolidin-1-yl)methyl)-6-methoxypyridin-2-yl)phenyl)-6-methoxy-[2,4'-bipyridin]-5-yl)methyl)pyrrolidin-3-ol;
(S)-1-((3'-chloro-2'-(2-chloro-3-(5-(((R)-3-hydroxypyrrolidin-1-yl)methyl)-6-methoxypyridin-2-yl)phenyl)-6-methoxy-[2,4'-bipyridin]-5-yl)methyl)pyrrolidin-3-ol;
(S)-1-((3'-chloro-2'-(2-chloro-3-(5-(((S)-3-hydroxypyrrolidin-1-yl)methyl)-6-methoxypyridin-2-yl)phenyl)-6-methoxy-[2,4'-bipyridin]-5-yl)methyl)pyrrolidin-3-ol; and
1-(4-(((3'-chloro-2'-(2-chloro-3-(5-((4-hydroxypiperidin-1-yl)methyl)-6-methoxypyridin-2-yl)phenyl)-6-methoxy-[2,4'-bipyridin]-5-yl)methyl)amino)piperidin-1-yl)ethan-1-one.

Embodiment 16 provides a pharmaceutical composition comprising at least one compound of any one of Embodiments 1-15 and at least one pharmaceutically acceptable carrier.

Embodiment 17 provides the pharmaceutical composition of Embodiment 16, further comprising at least one additional agent that treats, ameliorates, and/or prevents hepatitis virus infection.

Embodiment 18 provides the pharmaceutical composition of Embodiment 17, wherein the at least one additional agent comprises at least one selected from the group consisting of reverse transcriptase inhibitor; capsid inhibitor; cccDNA formation inhibitor; RNA destabilizer; oligomeric nucleotide targeted against the HBV genome; immunostimulatory; GalNAc-siRNA conjugate targeted against an HBV gene transcript; and therapeutic vaccine.

Embodiment 19 provides the pharmaceutical composition of Embodiment 18, wherein the immunostimulator is a checkpoint inhibitor.

Embodiment 20 provides the pharmaceutical composition of Embodiment 19, wherein the checkpoint inhibitor is a PD-L1 inhibitor.

Embodiment 21 provides the pharmaceutical composition of any one of Embodiments 16-20, wherein the hepatitis virus is at least one selected from the group consisting of hepatitis B virus (HBV) and hepatitis D virus (HDV).

Embodiment 22 provides a method of treating, ameliorating, and/or preventing hepatitis virus infection in a subject, the method comprising administering to the subject in need thereof a therapeutically effective amount of the compound of any one of Embodiments 1-15 and/or the pharmaceutical composition of any one of Embodiments 16-21, or a salt, solvate, prodrug, stereoisomer, tautomer, or any mixtures thereof.

Embodiment 23 provides the method of Embodiment 22, wherein the subject is infected with hepatitis B virus (HBV).

Embodiment 24 provides the method of any one of Embodiments 22-23, wherein the subject is further infected with hepatitis D virus (HDV).

Embodiment 25 provides the method of any one of Embodiments 22-24, wherein the subject is infected with HBV and HDV.

Embodiment 26 provides the method of any one of Embodiments 22-25, wherein the subject is further administered at least one additional agent useful for treating the hepatitis virus infection.

Embodiment 27 provides the method of Embodiment 26, wherein the at least one additional agent comprises at least one selected from the group consisting of reverse transcriptase inhibitor; capsid inhibitor; cccDNA formation inhibitor; RNA destabilizer; oligomeric nucleotide targeted against the HBV genome; immunostimulator; GalNAc-siRNA conjugate targeted against an HBV gene transcript; and therapeutic vaccine.

Embodiment 28 provides the method of Embodiment 27, wherein the immunostimulatory is a checkpoint inhibitor.

Embodiment 29 provides the method of Embodiment 28, wherein the checkpoint inhibitor is a PD-L1 inhibitor.

Embodiment 30 provides the method of any one of Embodiments 26-29, wherein the subject is co-administered the at least one compound and the at least one additional agent.

Embodiment 31 provides the method of any one of Embodiments 26-30, wherein the at least one compound and the at least one additional agent are coformulated.

Embodiment 32 provides the method of any one of Embodiments 22-31, wherein the subject is a mammal.

Embodiment 33 provides the method of Embodiment 32, wherein the mammal is a human.

Embodiment 34 provides a method of treating, ameliorating, and/or preventing cancer in a subject, the method comprising administering to the subject in need thereof a therapeutically effective amount of the compound of any one of Embodiments 1-15 and/or the pharmaceutical composition of any one of Embodiments 16-21, or a salt, solvate, prodrug, stereoisomer, tautomer, or any mixtures thereof.

Embodiment 35 provides the method of Embodiment 34, wherein the compound or composition is the only anticancer agent administered to the subject.

Embodiment 36 provides the method of any one of Embodiments 34-35, wherein the compound is administered to the subject in a pharmaceutically acceptable composition.

Embodiment 37 provides the method of Embodiment 34 or 36, wherein the subject is further administered at least one additional agent or therapy useful for treating, ameliorating, and/or preventing the cancer.

Embodiment 38 provides the method of Embodiment 37, wherein the additional anticancer agent or therapy comprises nivolumab, pembrolizumab, atezolizumab, ipilimumab, chemotherapy, radiation therapy, and/or resection therapy.

Embodiment 39 provides the method of Embodiment 37, wherein the additional anticancer agent or therapy comprises Rituxan, doxorubicin, gemcitabine, nivolumab, pembrolizumab, and/or pilimumab.

Embodiment 40 provides the method of any one of Embodiments 34 and 36-39, wherein the compound or composition is coformulated and/or co-administered with the at least one additional agent.

Embodiment 41 provides the method of any one of Embodiments 34-40, wherein the cancer is amenable to treatment by inhibiting PD-1, PD-L1, or the PD-1/PD-L1 interaction.

Embodiment 42 provides the method of any one of Embodiments 34-41, wherein the cancer is at least one of pancreatic cancer, bladder cancer, colorectal cancer, breast cancer, prostate cancer, renal cancer, hepatocellular cancer, lung cancer, ovarian cancer, cervical cancer, gastric cancer, esophageal cancer, head and neck cancer, melanoma, neuroendocrine cancer, CNS cancer, brain cancer, bone cancer, soft tissue sarcoma, non-small cell lung cancer, small-cell lung cancer, or colon cancer.

Embodiment 43 provides the method of any one of Embodiments 34-41, wherein the cancer is at least one of acute lymphocytic leukemia (ALL), acute myeloid leukemia (AML), chronic lymphocytic leukemia (CLL), small lymphocytic lymphoma (SLL), myelodysplastic syndrome (MDS), myeloproliferative disease (MPD), chronic myeloid leukemia (CML), multiple myeloma (MM), non-Hodgkin's lymphoma (NHL), mantle cell lymphoma (MCL), follicular lymphoma, Waldestrom's macroglobulinemia (WM), T-cell lymphoma, B-cell lymphoma and diffuse large B-cell lymphoma (DLBCL).

Embodiment 44 provides the method of any one of Embodiments 34-43, wherein the subject is a mammal.

Embodiment 45 provides the method of Embodiment 44, wherein the mammal is a human.

The disclosures of each and every patent, patent application, and publication cited herein are hereby incorporated herein by reference in their entirety. While this disclosure has been disclosed with reference to specific embodiments, it is apparent that other embodiments and variations of this disclosure may be devised by others skilled in the art without departing from the true spirit and scope of the disclosure. The appended claims are intended to be construed to include all such embodiments and equivalent variatiosns.

The invention is further embodied by the following items:
1. A compound of formula (I), or a salt, solvate, geometric isomer, stereoisomer, or tautomer thereof: 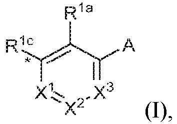 wherein:
   A is 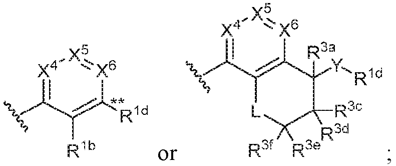
   Y is NR³⁶ or O;
   L is selected from the group consisting of -C(R^{3g})(R^{3h})-, -(C(R^{3g})(R^{3h}))(C(R³ⁱ)(R^{3j}))-, and a bond;
   X¹ is selected from the group consisting of CR^{'1} and N;
   X² is selected from the group consisting of CR^{'2} and N;
   X³ is selected from the group consisting of CR^{'3} and N;
   X⁴ is selected from the group consisting of CR^{"1} and N;
   X⁵ is selected from the group consisting of CR^{"2} and N;
   X⁶ is selected from the group consisting of CR^{"3} and N;
      wherein one to four of X¹, X², X³, X⁴, X⁵, and X⁶ is N;
   R^{'1}, R^{'2}, R^{'3}, R^{"1}, R^{"2}, and R^{"3} are each independently selected from the group consisting of H, halogen, C₁-C₃ alkyl, C₃-C₈ cycloalkyl, C₁-C₃ alkoxy, cyano, C₁-C₃ haloalkyl, and C₁-C₃ haloalkoxy;
   R^{1a} and R^{1b} are each independently selected from the group consisting of H, halogen, C₁-C₃ alkyl, C₃-C₈ cycloalkyl, C₁-C₃ alkoxy, cyano, C₁-C₃ haloalkyl, and C₁-C₃ haloalkoxy;
   R^{1c} and R^{1d} are each independently selected from the group consisting of: 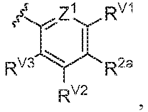 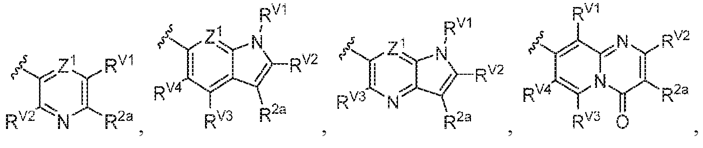 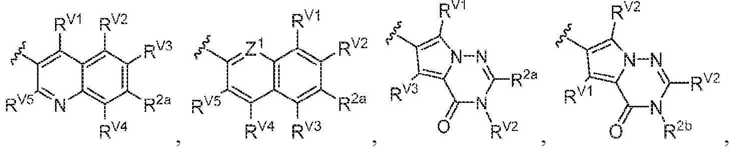 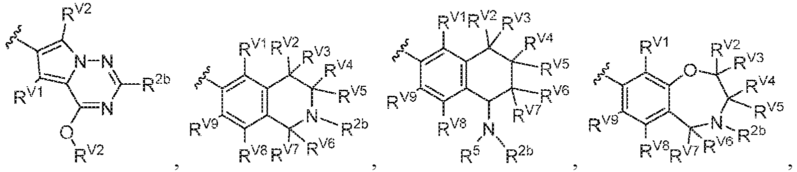 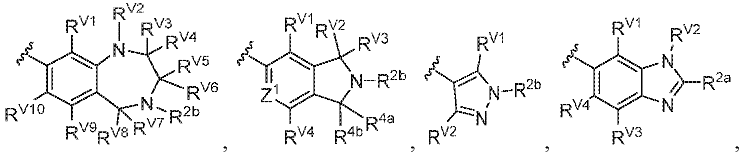 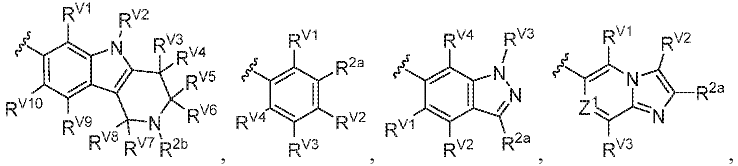 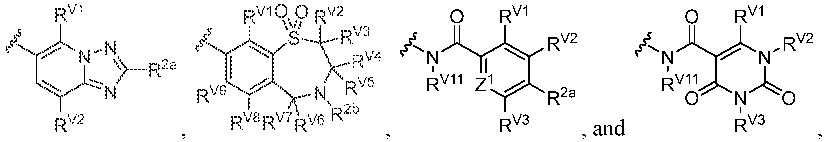 wherein each occurrence of Z¹ is CR^{V10} or N;
   R^{2a} is selected from the group consisting of -C(=O)NR⁶R⁶, C(=O)OR^{I}, optionally substituted heterocyclyl, -(CH₂)₁₋₃(optionally substituted heterocyclyl), optionally substituted C₁-C₆ alkoxy, optionally substituted C₁-C₆ aminoalkyl, and optionally substituted C₁-C₆ hydroxyalkyl,
      wherein each optional substituent in R^{2a} is independently selected from the group consisting of optionally substituted heterocyclyl, optionally substituted C₁-C₆ alkyl, optionally substituted C₁-C₆ hydroxyalkyl, optionally substituted cycloalkyl, C(=O)(optionally substituted C₁-C₆ alkyl), and optionally substituted -(CH₂)₁₋₂(heterocyclyl),
      wherein two optional substituents in R^{2a} may combine with the atoms to which they are bound to form an optionally substituted C₂-C₈ heterocyclyl or optionally substituted C₃-C₈ cycloalkyl,
      wherein each occurrence of R^{I} is independently H, C₁-C₆ alkyl, or C₃-C₈ cycloalkyl, wherein the alkyl or cycloalkyl is independently optionally substituted with at least one of halogen, -OH, C₁-C₆ alkoxy, -NH₂, -NH(C₁-C₆ alkyl), and -N(C₁-C₆ alkyl)(C₁-C₆ alkyl),
      wherein each occurrence of R⁶ is independently H, C₁-C₆ alkyl, or C₃-C₈ cycloalkyl, wherein the alkyl or cycloalkyl is independently optionally substituted with at least one of halogen, -OH, C₁-C₆ alkoxy, -NH₂, -NH(C₁-C₆ alkyl), and -N(C₁-C₆ alkyl)(C₁-C₆ alkyl),
   R^{2b} is selected from the group consisting of H, -(CH₂)₁₋₃C(=O)OR^{I}, -(CH₂)₁₋₃C(=O)NR^{I}R^{I}, optionally substituted heterocyclyl, -(CH₂)₁₋₂(optionally substituted heterocyclyl), optionally substituted C₁-C₆ alkyl, optionally substituted C₁-C₆ haloalkyl, optionally substituted C₁-C₆ alkoxy, optionally substituted C₁-C₆ aminoalkyl, and optionally substituted C₁-C₆ hydroxyalkyl,
      wherein each optional substituent in R^{2b} is independently selected from the group consisting of optionally substituted C₁-C₆ alkyl, optionally substituted heterocyclyl, optionally substituted cycloalkyl, and optionally substituted -(CH₂)₁₋₃(heterocyclyl);
   R^{3a}, R^{3b}, R^{3c}, R^{3d}, R^{3e}, R^{3f}, R^{3g}, R^{3h} R³ⁱ, and R^{3j} are each independently selected from the group consisting of H, halogen, C₁-C₃ alkyl, C₃-C₈ cycloalkyl, C₁-C₃ alkoxy, C₁-C₃ haloalkyl, and C₁-C₃ haloalkoxy;
   R⁵ is selected from the group consisting of H, C₁-C₃ alkyl, C₃-C₈ cycloalkyl, C₁-C₃ alkoxy, C₁-C₃ haloalkyl, and C₁-C₃ haloalkoxy,
      wherein R⁵ and R^{2b} may combine with the nitrogen atom to which they are bound to form a C₃-C₁₂ heterocyclyl;
   each occurrence of R^{4a} and R^{4b} is independently selected from the group consisting of H, C₁-C₃ alkyl, C₃-C₈ cycloalkyl, C₁-C₃ alkoxy, C₁-C₃ haloalkyl, and C₁-C₃ haloalkoxy,
      wherein R^{4a} and R^{4b} may combine with the carbon atom to which they are bound to form a carbonyl (C=O);
   each occurrence of R^{V1}, R^{V2}, R^{V3}, R^{V4}, R^{V5}, R^{V6}, R^{V7}, R^{V8}, R^{V9}, and R^{V10} is independently selected from the group consisting of H, C₁-C₃ alkyl, C₃-C₈ cycloalkyl, C₁-C₃ alkoxy, cyano, halogen, C₁-C₃ haloalkyl, C₁-C₃ haloalkoxy, and C(=O)OR^{I};
   R^{V11} is selected from the group consisting of H, C₁-C₃ alkyl, C₃-C₈ cycloalkyl, C₁-C₃ alkoxy, C₁-C₃ haloalkyl, and C₁-C₃ haloalkoxy;
   wherein if R^{1c} and R^{1d} are identical, then at least one of the following applies:
      (a) if X¹ is N, then Z¹ in R^{1c}, if present, is CR^{V10}, and;
      (b) if X⁶ is N, then Z¹ in R^{1d}, if present, is CR^{V10}.
2. The compound of item 1, which is selected from the group consisting of: 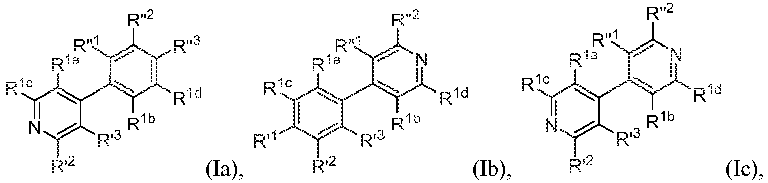 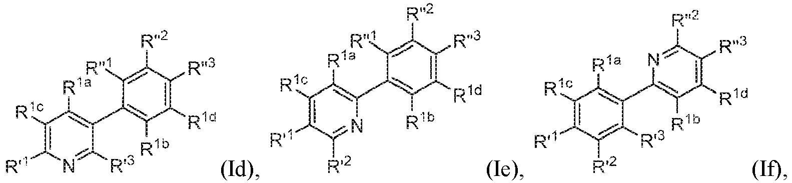 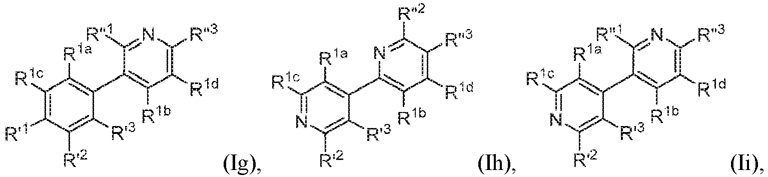 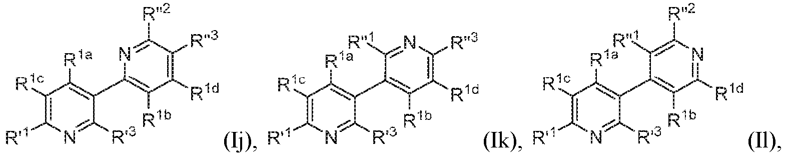 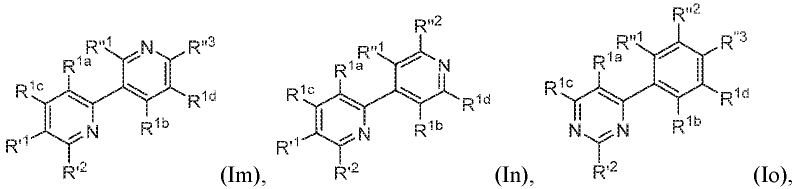 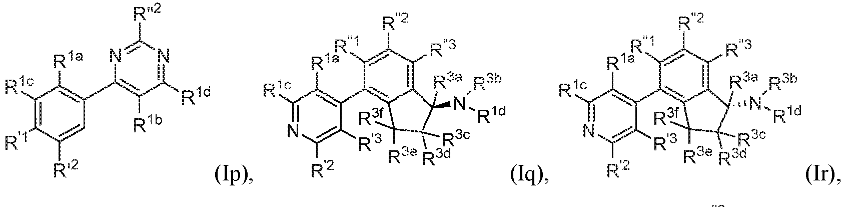 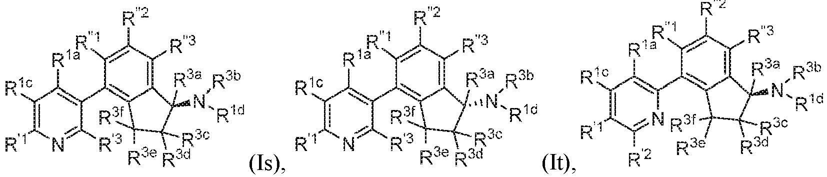 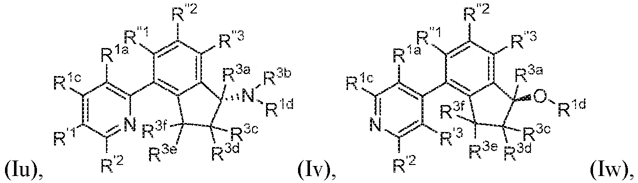 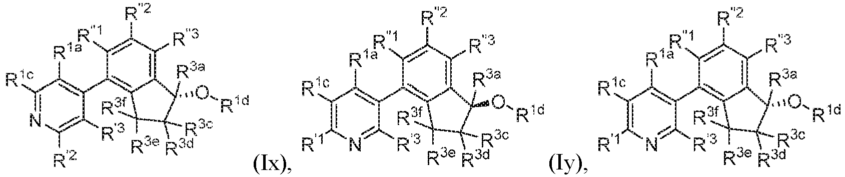 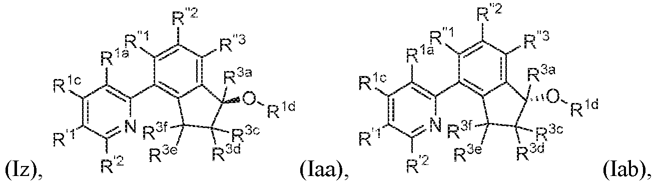 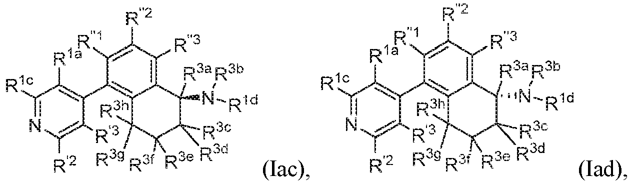 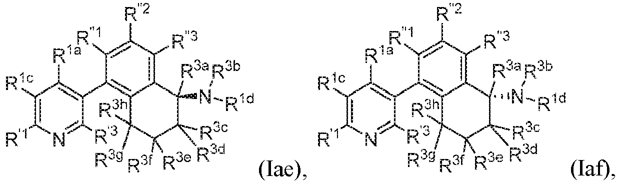 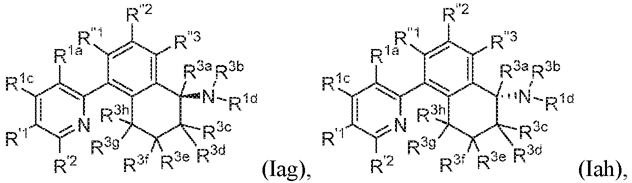 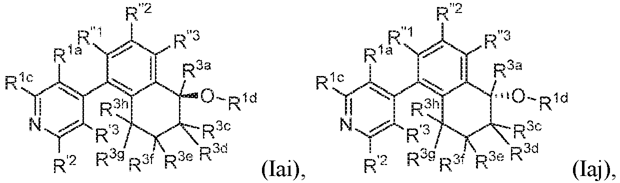 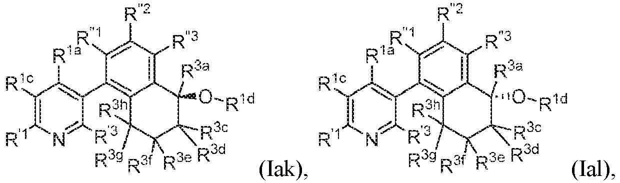 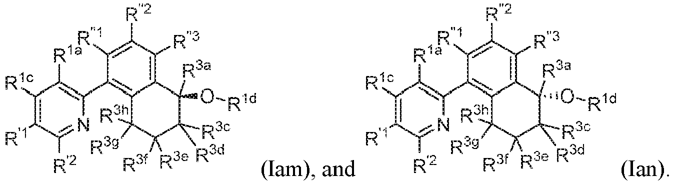
3. The compound of any one of items 1-2, wherein one of the following applies:
   (a) each of R^{'2}, R^{'3}, R^{"1}, R^{"2}, and R^{"3} are H, and X¹ is N;
   (b) each of R^{'1}, R^{'3} , R^{"1}, R^{"2}, and R^{"3} are H, and X² is N;
   (c) each of R^{'1}, R^{'2}, R^{"1}, R^{"2}, and R^{"3} are H, and X³ is N;
   (d) each of R^{'1}, R^{'2}, R^{'3}, R^{"2}, and R^{"3} are H, and X⁴ is N;
   (e) each of R^{'1}, R^{'2}, R^{'3}, R^{"1}, and R^{"3} are H, and X⁵ is N;
   (f) each of R^{'1}, R^{'2} R^{'3}, R^{"1}, and R^{"2} are H, and X⁶ is N;
   (g) each of R^{'2}, R^{'3}, R^{"2}, and R^{"3} are H, and X¹ and X⁴ are N;
   (h) each of R^{'2}, R^{'3}, R^{"1}, and R^{"3} are H, and X¹ and X⁵ are N;
   (i) each of R^{'2}, R^{'3}, R^{"1}, and R^{"2} are H, and X¹ and X⁶ are N;
   (j) each of R^{'1}, R^{'3}, R^{"2}, and R^{"3} are H, and X² and X⁴ are N;
   (k) each of R^{'1}, R^{'3}, R^{"1}, and R^{"3} are H, and X² and X⁵ are N;
   (l) each of R^{'1}, R^{'3}, R^{"1}, and R^{"2} are H, and X² and X⁶ are N;
   (m) each of R^{'1}, R^{'2}, R^{"1}, and R^{"3} are H, and X³ and X⁵ are N;
   (n) each of R^{'1}, R^{'2}, R^{"1}, and R^{"2} are H, and X³ and X⁶ are N;
   (o) each of R^{'2}, R^{'1}, R^{"2}, and R^{"3} are H, and X¹ and X³ are N; and
   (p) each of R^{'1}, R^{'2}, R^{'3}, and R^{"2} are H, and X⁴ and X⁶ are N.
4. The compound of any one of items 1-3, wherein one of the following applies:
   (a) L is a bond and each of R^{3a}, R^{3c}, R^{3a}, R^{3e}, and R^{3f} is H;
   (b) L is a bond, Y is NR^{3b}, and each of R^{3a}, R^{3b}, R^{3c}, R^{3d}, R^{3e}, and R^{3f} is H;
   (c) L is -C(R^{3g})(R^{3h})- and each of R^{3a}, R^{3c}, R^{3d}, R^{3e}, R^{3f}, R^{3g}, and R^{3h} is H;
   (d) L is -C(R^{3g})(R^{3h})-, Y is NR^{3b}, and each of R^{3a}, R^{3b}, R^{3c}, R^{3d}, R^{3e}, R^{3f}, R^{3g}, and R^{3h} is H;
   (e) L is -(C(R^{3g})(R^{3h}))(C(R³ⁱ)(R^{3j}))- and each of R^{3a}, R^{3c}, R^{3d}, R^{3e}, R^{3f}, R^{3g}, R^{3h}, R³ⁱ, and R^{3j} is H; and
   (f) L is -C(R^{3g})(R^{3h})-, Y is NR^{3b}, and each of R^{3a}, R^{3b}, R^{3c}, R^{3d}, R^{3e}, R^{3f}, R^{3g}, R^{3h}, R³ⁱ, and R^{3j} are H.
5. The compound of any one of items 1-4, wherein each of of R^{1a} and R^{1b} is independently selected from the group consisting of H, F, Cl, Me, CF₃, and CN.
6. The compound of any one of items 1-5, wherein R^{1a} and R^{1b} are identical.
7. The compound of any one of items 1-6, wherein R^{2a} is selected from the group consisting of -(CH₂)₀₋₂NH(CH₂)₀₋₂(optionally substituted 5-oxopyrrolidin-2-yl), -(CH₂)₀₋₂N(C₁-C₆ alkyl)(CH₂)₀₋₂(optionally substituted 5-oxopyrrolidin-2-yl), -(CH₂)₀₋₂NH(CH₂)₀₋₂(C₁-C₆ alkyl), -(CH₂)₀₋₂N(C₁-C₆ alkyl)₂, -(CH₂)₀₋₂NH(CH₂)₀₋₂(C₁-C₆ haloalkyl), -(CH₂)₀₋₂N(C₁-C₆ alkyl)(C₁-C₆ haloalkyl), -(CH₂)₀₋₂NH(CH₂)₀₋₂(C₁-C₆ hydroxyalkyl), -(CH₂)₀₋₂N(C₁-C₆ alkyl)(CH₂)₀₋₂(C₁-C₆ hydroxyalkyl), -(CH₂)₀₋₂NH(CH₂)₀₋₂C(=O)O(C₁-C₆ alkyl), -(CH₂)₀₋₂N(C₁-C₆ alkyl)(CH₂)₀₋₂C(=0)O(C₁-C₆ alkyl), -(CH₂)₀₋₂NH(CH₂)₀₋₂N(C₁-C₆ alkyl)C(=O)(C₁-C₆ alkyl), -(CH₂)₀₋₂NH(CH₂)₀₋₂NHC(=O)(C₁-C₆ alkyl), -(CH₂)₀₋₂N(C₁-C₆ alkyl)(CH₂)₀₋₂N(C₁-C₆ alkyl)C(=O)(C₁-C₆ alkyl), -(CH₂)₀₋₂N(C₁-C₆ alkyl)(CH₂)₀₋₂NHC(=O)(C₁-C₆ alkyl),-(CH₂)₀₋₂NH(CH₂)₀₋₂(optionally substituted oxetanyl), -(CH₂)₀₋₂N(C₁-C₆ alkyl)(CH₂)₀₋₂(optionally substituted oxetanyl), -(CH₂)₀₋₂NH(CH₂)₀₋₂(optionally substituted oxolanyl),-(CH₂)₀₋₂N(C₁-C₆ alkyl)(CH₂)₀₋₂(optionally substituted oxolanyl), -(CH₂)₀₋₂NH(CH₂)₀₋₂(optionally substituted oxanyl), -(CH₂)₀₋₂N(C₁-C₆ alkyl)(CH₂)₀₋₂(optionally substituted oxanyl), -(CH₂)₀₋₂NH(CH₂)₀₋₂(optionally substituted cyclobutyl), -(CH₂)₀₋₂N(C₁-C₆ alkyl)(CH₂)₀₋₂(optionally substituted cyclobutyl), -(CH₂)₀₋₂NH(CH₂)₀₋₂(optionally substituted cyclohexyl), -(CH₂)₀₋₂N(C₁-C₆ alkyl)(CH₂)₀₋₂(optionally substituted cyclohexyl), -(CH₂)₀₋₂NH(CH₂)₀₋₂(optionally substituted C₅-C₆ cycloalkylsulfonyl), -(CH₂)₀₋₂N(C₁-C₆ alkyl)(CH₂)₀₋₂(optionally substituted C₅-C₆ cycloalkylsulfonyl), -(CH₂)₀₋₂NH(CH₂)₀₋₂(optionally substituted azetidinyl), -(CH₂)₀₋₂N(C₁-C₆ alkyl)(CH₂)₀₋₂(optionally substituted azetidinyl), -(CH₂)₀₋₂NH(CH₂)₀₋₂(optionally substituted pyrrolidinyl), -(CH₂)₀₋₂N(C₁-C₆ alkyl)(CH₂)₀₋₂(optionally substituted pyrrolidinyl), -(CH₂)₀₋₂NH(CH₂)₀₋₂(optionally substituted piperidinyl), -(CH₂)₀₋₂N(C₁-C₆ alkyl)(CH₂)₀₋₂(optionally substituted piperidinyl),-(CH₂)₀₋₂(optionally substituted guanidinyl), -(CH₂)₀₋₂NH(C(CH₂)₂₋₅)C(=O)O(C₁-C₆ alkyl),-(CH₂)₀₋₂N(C₁-C₆ alkyl)(C(CH₂)₂₋₅)C(=O)O(C₁-C₆ alkyl), -(CH₂)₀₋₂NH(CH(C₁-C₃ alkyl))C(=O)O(C₁-C₆ alkyl), -(CH₂)₀₋₂N(C₁-C₆ alkyl)(CH(C₁-C₃ alkyl))C(=O)O(C₁-C₆ alkyl), -(CH₂)₀₋₂NH(CH₂)₀₋₁(C₅-C₉ bridged cycloalkyl)(CH₂)₀₋₁C(=O)O(C₁-C₆ alkyl), -(CH₂)₀₋₂N(C₁-C₆ alkyl)(CH₂)₀₋₁(C₅-C₉ bridged cycloalkyl)(CH₂)₀₋₁C(=O)O(C₁-C₆ alkyl), -(CH₂)₀₋₂NH(CH₂)₀₋₁(CHOH)(CH₂)₀₋₁C(=O)O(C₁-C₆ alkyl), -(CH₂)₀₋₂NH(CH₂)₀₋₁(CHO(C₁-C₆ alkyl))(CH₂)₀₋₁C(=O)O(C₁-C₆ alkyl), -(CH₂)₀₋₂N(C₁-C₆ alkyl)(CH₂)₀₋₁(CHOH)(CH₂)₀₋₁C(=O)O(C₁-C₆ alkyl), -(CH₂)₀₋₂N(C₁-C₆ alkyl)(CH₂)₀₋₁(CHO(C₁-C₆ alkyl))(CH₂)₀₋₁C(=O)O(C₁-C₆ alkyl), -(CH₂)₀₋₂(C₄-C₁₀ bridged heterocycloalkyl), -(CH₂)₀₋₂(optionally substituted azetidinyl), -(CH₂)₀₋₂(optionally substituted pyrrolidinyl), -(CH₂)₀₋₂(optionally substituted piperidinyl), -(CH₂)₀₋₂(optionally substituted morpholinyl), -(CH₂)₀₋₂(optionally substituted piperazinyl), -(CH₂)₀₋₂(optionally substituted piperazin-2-onyl), -C(CH₃)N(C₁-C₆ alkyl)₂, -(CH₂)₀₋₂N(C₁-C₆ alkyl)(CH₂)₀₋₂(CHF₂), -(CH₂)₀₋₂NH(CH₂)₀₋₂(CHF₂), -(CH₂)₀₋₂N(C₁-C₆ alkyl)(CH₂)₀₋₄(CH₂F), -(CH₂)₀₋₂NH(CH₂)₀₋₄(CH₂F), -(CH₂)₀₋₂NH(CH₂)₀₋₂N(C₁-C₆ alkyl)C(=O)CH₃, -(CH₂)₀₋₂NH(CH₂)₀₋₂NHC(=O)CH₃, -(CH₂)₀₋₂N(C₁-C₆ alkyl)(CH₂)₀₋₂N(C₁-C₆ alkyl)C(=O)CH₃, -(CH₂)₀₋₂N(C₁-C₆ alkyl)(CH₂)₀₋₂NHC(=O)CH₃, -(CH₂)₀₋₂N(C₁-C₆ alkyl)C((C(=O)OC₁-C₆ alkyl)((CH₂)₀₋₂O(C₁-C₆ alkyl))), -(CH₂)₀₋₂NHC((C(=O)OC₁-C₆ alkyl)((CH₂)₀₋₂O(C₁-C₆ alkyl))), -(CH₂)₀₋₂N(C₁-C₆ alkyl)C((C(=O)OH((CH₂)₀₋₂O(C₁-C₆ alkyl))), -(CH₂)₀₋₂N(C₁-C₆ alkyl)C((C(=O)OC₁-C₆ alkyl)((CH₂)₀₋₂OH)), -(CH₂)₀₋₂NHC((C(=O)OH)((CH₂)₀₋₂O(C₁-C₆ alkyl))), -(CH₂)₀₋₂N(C₁-C₆ alkyl)C((C(=O)OH((CH₂)₀₋₂OH)), -(CH₂)₀₋₂NHC((C(=O)OC₁-C₆ alkyl)((CH₂)₀₋₂OH)), -(CH₂)₀₋₂NHC((C(=O)OH((CH₂)₀₋₂OH)), -(CH₂)₀₋₂N(C₁-C₆ alkyl)(CH₂)₁₋₄N(C₁-C₆ alkyl)C(=O)Me, -(CH₂)₀₋₂N(C₁-C₆ alkyl)(CH₂)₁₋₄NHC(=O)Me, -(CH₂)₀₋₂NH(CH₂)₁₋₄N(C₁-C₆ alkyl)C(=O)Me, -(CH₂)₀₋₂NH(CH₂)₁₋₄NHC(=O)Me, -(CH₂)₀₋₂N(C₁-C₆ alkyl)(CH₂)₁₋₅C(=O)N(C₁-C₆ alkyl)(C₁-C₆ alkyl), -(CH₂)₀₋₂NH(CH₂)₁₋₅C(=O)N(C₁-C₆ alkyl)(C₁-C₆ alkyl), -(CH₂)₀₋₂N(C₁-C₆ alkyl)(CH₂)₁₋₅C(=O)NH(C₁-C₆ alkyl), -(CH₂)₀₋₂N(C₁-C₆ alkyl)(CH₂)₁₋₅C(=O)NH₂, -(CH₂)₀₋₂NH(CH₂)₁₋₅C(=O)NH(C₁-C₆ alkyl), -(CH₂)₀₋₂NH(CH₂)₁₋₅C(=O)NH₂, -(CH₂)₀₋₂N(C₁-C₆ alkyl)(CH₂)₀₋₂(optionally substituted cyclopropyl), -(CH₂)₀₋₂NH(CH₂)₀₋₂(optionally substituted cyclopropyl), -(CH₂)₀₋₂N(C₁-C₆ alkyl)(CH₂)₀₋₂(optionally substituted butanolid-2-yl), -(CH₂)₀₋₂NH(CH₂)₀₋₂(optionally substituted butanolide-2-yl), -(CH₂)₀₋₂N(C₁-C₆ alkyl)(CH₂)₀₋₂(optionally substituted tetrahydro-4-thiopyranyl), -(CH₂)₀₋₂NH(CH₂)₀₋₂(optionally substituted oxazolyl), -(CH₂)₀₋₂NH(CH₂)₀₋₂(optionally substituted tetrahydro-4-thiopyranyl), -CH(CH₃)NH(CH₂)₀₋₂(optionally substituted piperidinyl), -CH(CH₃)N(C₁-C₆ alkyl)(CH₂)₀₋₂(optionally substituted piperidinyl), -(CH₂)₀₋₂N(C₁-C₆ alkyl)(CH₂)₀₋₂(optionally substituted piperazinyl), and -(CH₂)₀₋₂NH(CH₂)₀₋₂(optionally substituted piperazinyl).
8. The compound of any one of items 1-7, wherein R^{2b} is selected from the group consisting of -(CH₂)₀₋₂(optionally substituted 5-oxopyrrolidin-2-yl), -(CH₂)₀₋₂(optionally substituted piperidinyl), -(CH₂)₀₋₂(optionally substituted azetidinyl), -(CH₂)₀₋₂(C₁-C₆ alkyl),-(CH₂)₀₋₂(C₁-C₆ haloalkyl), -(CH₂)₀₋₂(C₁-C₆ hydroxyalkyl), -(CH₂)₀₋₂(optionally substituted cyclopropyl), -(CH₂)₀₋₂(optionally substituted cyclobutyl), -(CH₂)₀₋₂C(=O)OH, -(CH₂)₀₋₂N(C₁-C₆ alkyl)CH₃; -(CH₂)₀₋₂NHCH₃, -(CH₂)₀₋₂N(C₁-C₆ alkyl)(CH₂)₀₋₂C(=O)O(C₁-C₆ alkyl), -(CH₂)₀₋₂NH(CH₂)₀₋₂C(=O)O(C₁-C₆ alkyl), -(CH₂)₀₋₂N(C₁-C₆ alkyl)(CH₂)₀₋₂C(=O)OH,-(CH₂)₀₋₂NH(CH₂)₀₋₂C(=O)OH, -(CH₂)₀₋₂N(C₁-C₆ alkyl)(CH₂)₀₋₂(optionally substituted 5-oxopyrrolidin-2-yl), -(CH₂)₀₋₂NH(CH₂)₀₋₂(optionally substituted 5-oxopyrrolidin-2-yl),-(CH₂)₀₋₂C(=O)N(C₁-C₆ alkyl)(C₁-C₆ alky), -(CH₂)₀₋₂C(=O)NH(C₁-C₆ alkyl), -(CH₂)₀₋₂C(=O)NH₂, -(CH₂)₀₋₂CH(OC₁-C₆ alkyl)CH₂F, -(CH₂)₀₋₂CH(OH)CH₂F, and -(CH₂)₀₋₂C(=O)O(C₁-C₆ alkyl).
9. The compound of any one of items 1-8, wherein R^{2a} is selected from the group consisting of:
   OR^{c}, 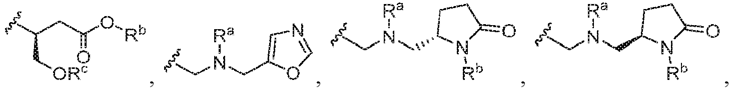 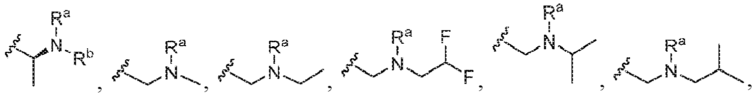 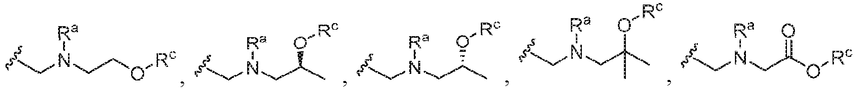 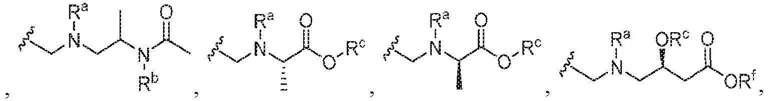 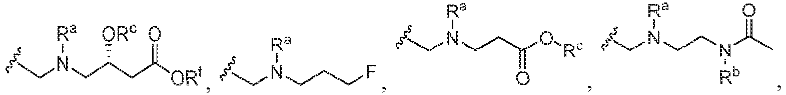 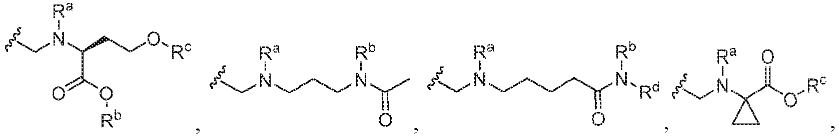 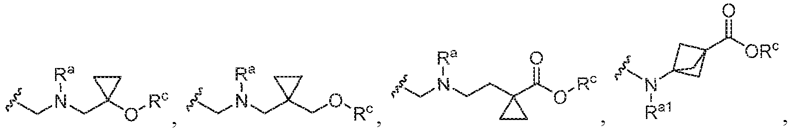 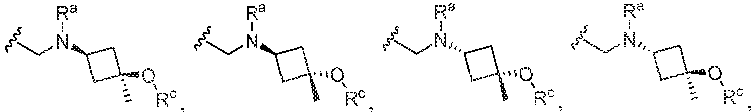 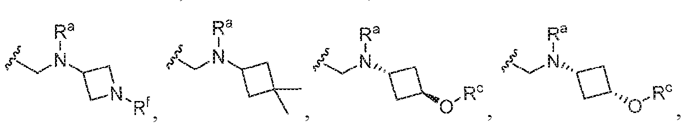 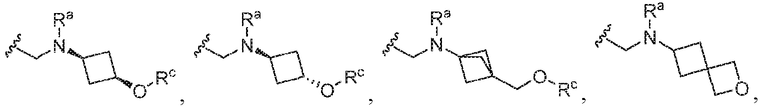 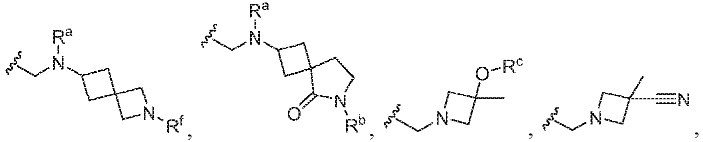 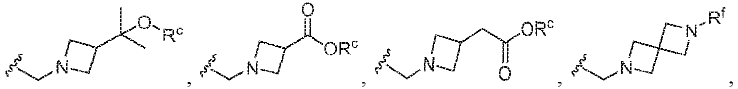 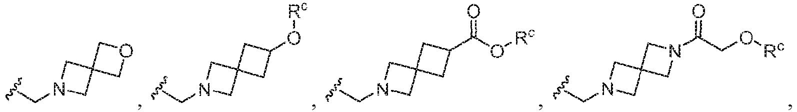 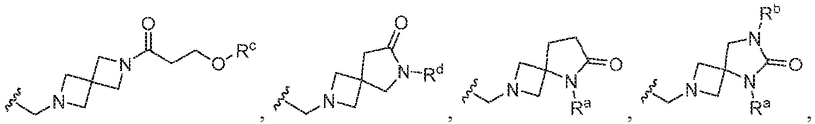 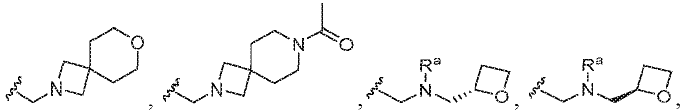 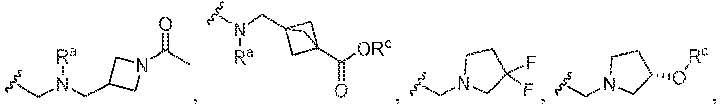 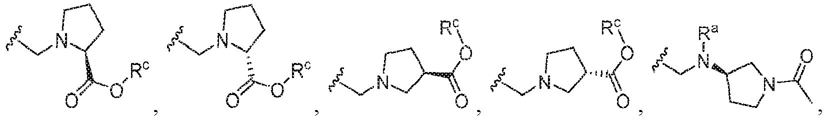 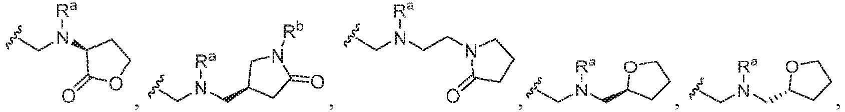 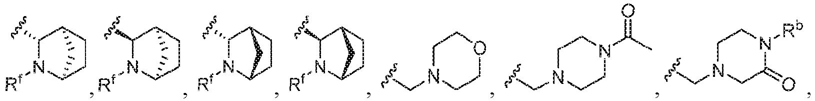 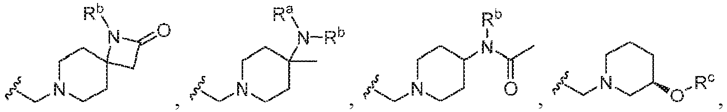 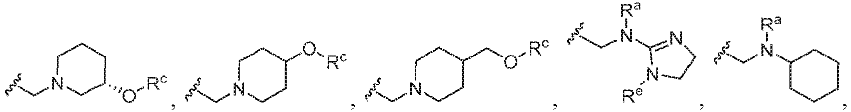 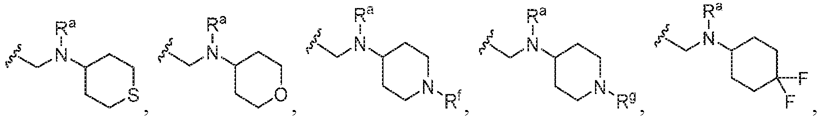 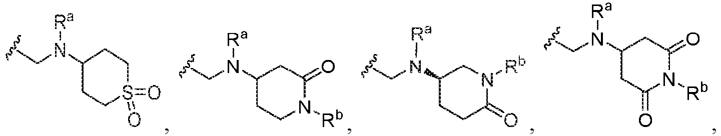 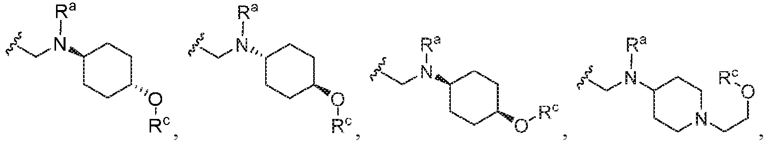 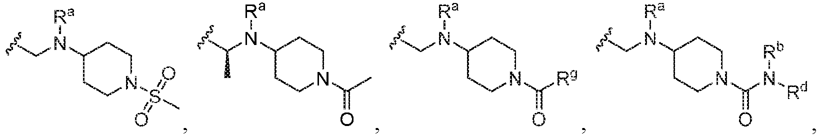 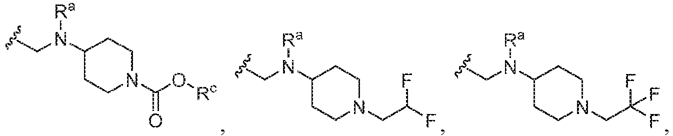 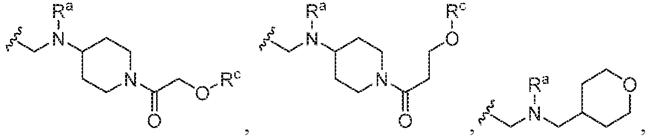 and 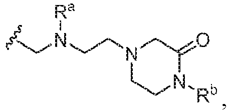
   wherein each of R^{a}, R^{b}, R^{c}, R^{d}, R^{e}, and R^{f} is independently selected from the group consisting of H, C₁-C₃ alkyl, C₃-C₈ cycloalkyl, C₁-C₃ alkoxy, C₁-C₃ haloalkyl, C₁-C₃ haloalkoxy, and -C(=O)C₁-C₃ alkyl; and
   R^{g} is selected from the group consisting of optionally substituted C₂-C₆ heteroaryl and optionally substituted phenyl.
10. The compound of any one of items 1-9, wherein at least one of R^{a}, R^{b}, R^{c}, R^{d}, R^{e}, and R^{f} is selected from the group consisting of methyl, isopropyl, and -C(=O)CH₃.
11. The compound of any one of items 1-10, wherein at least one of R^{a}, R^{b}, R^{c}, R^{d}, R^{e}, and R^{f}, if present, is H.
12. The compound of any one of items 1-11, wherein R^{2b} is selected from the group consisting of: 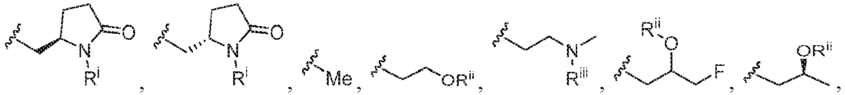 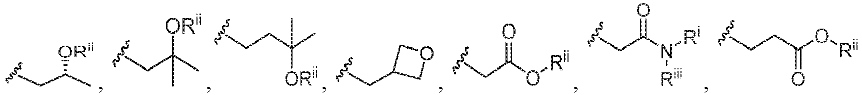 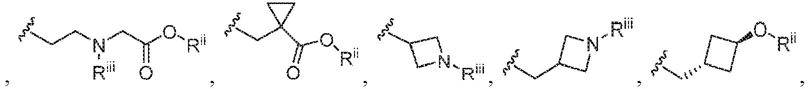 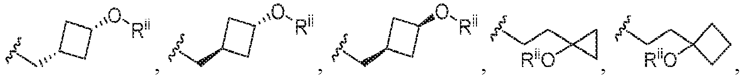 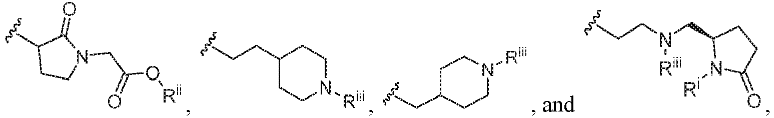 wherein each of Rⁱ, Rⁱⁱ, and Rⁱⁱⁱ is independently selected from the group consisting of H, C₁-C₃ alkyl, C₃-C₈ cycloalkyl, C₁-C₃ alkoxy, C₁-C₃ haloalkyl, C₁-C₃ haloalkoxy, and - C(=O)C₁-C₃ alkyl.
13. The compound of any one of items 1-12, wherein R^{1c} and R^{1d} are independently selected from the group consisting of: 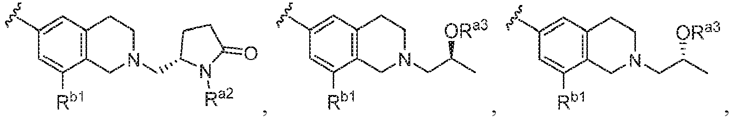 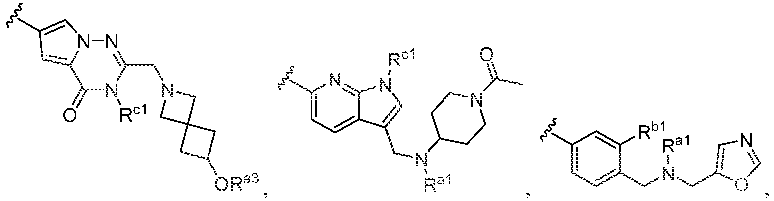 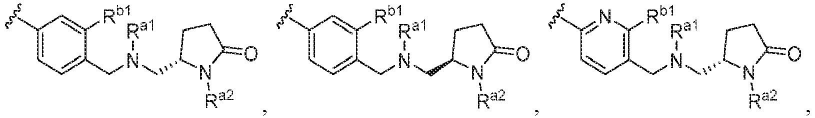 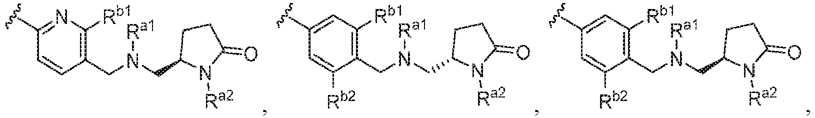 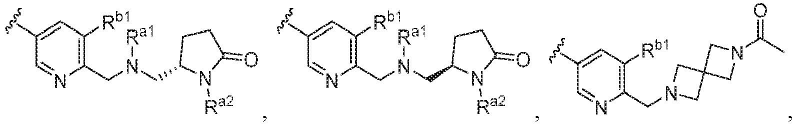 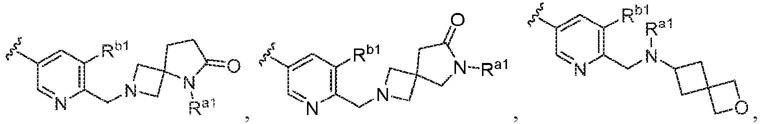 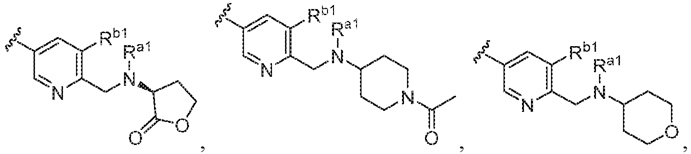 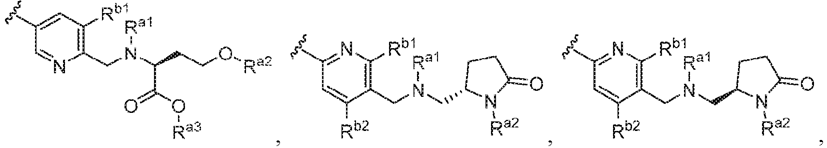 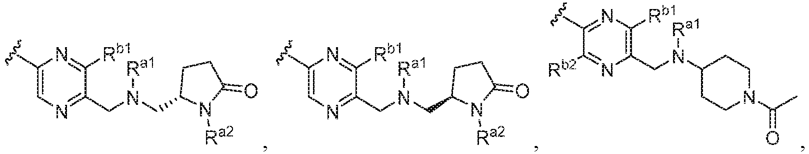 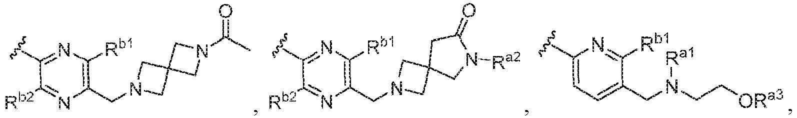 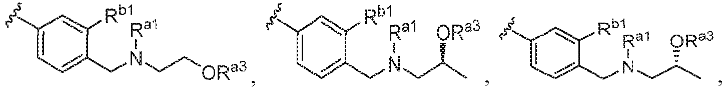 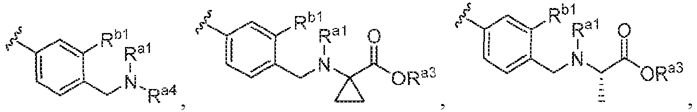 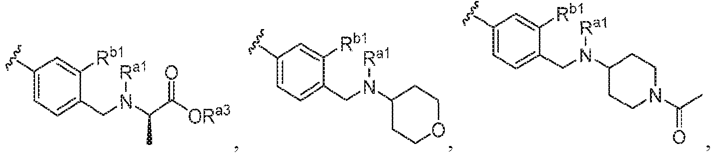 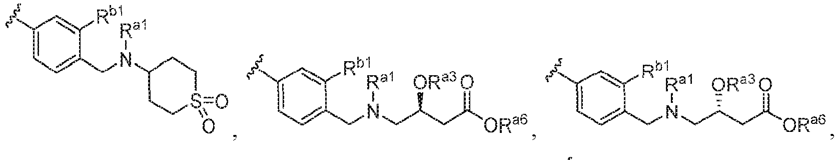 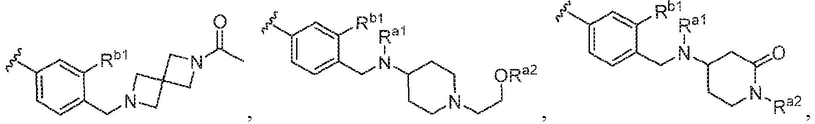 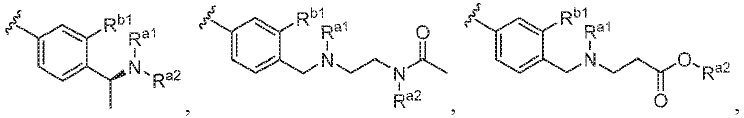 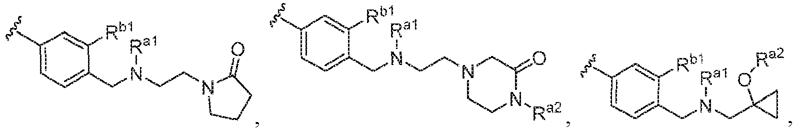 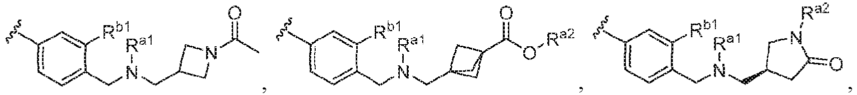 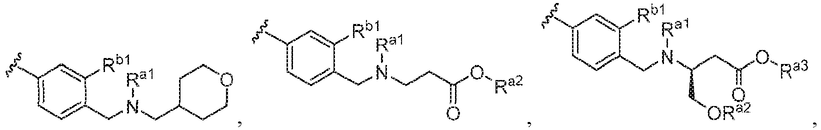 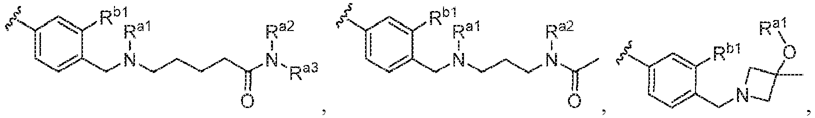 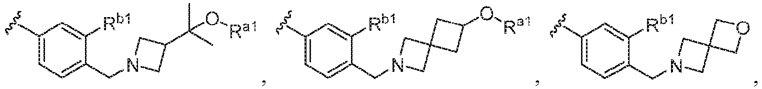 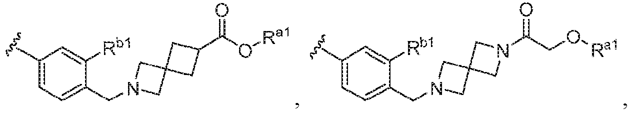 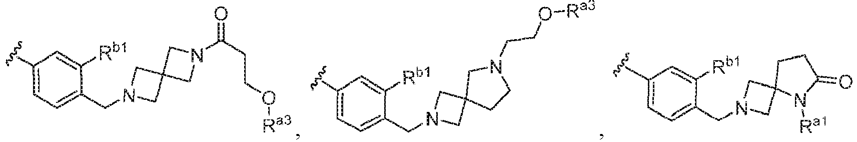 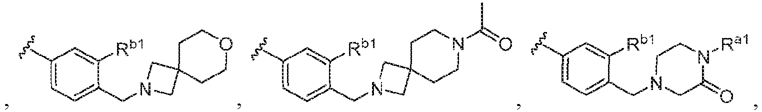 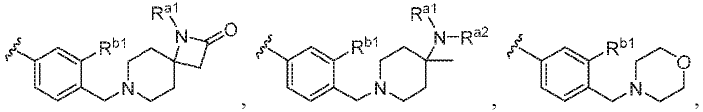 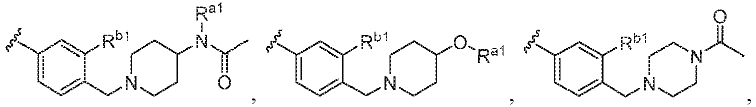 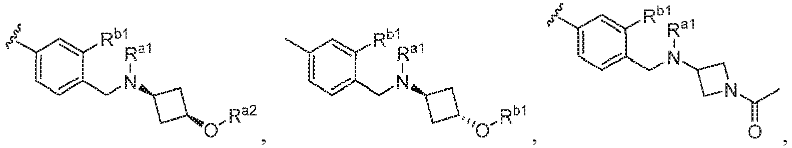 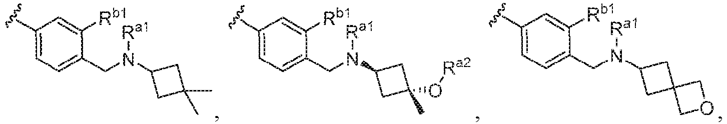 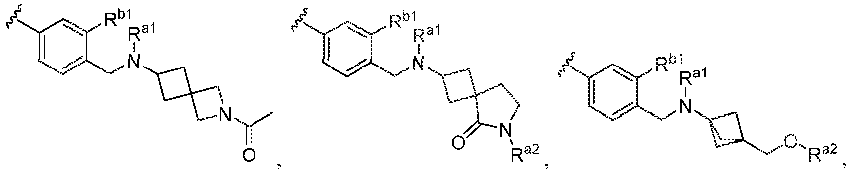 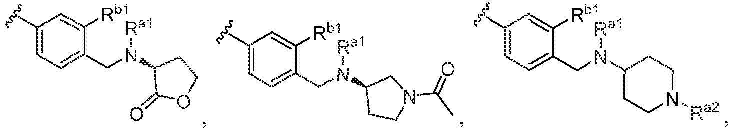 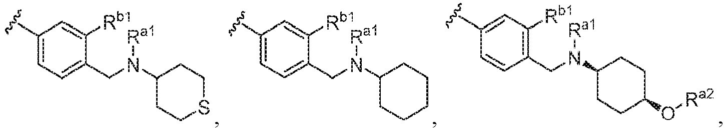 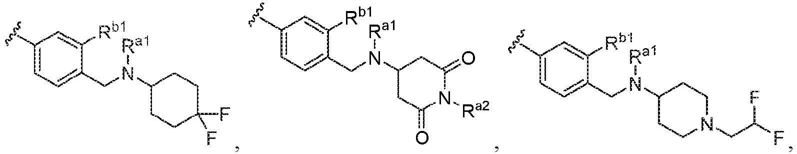 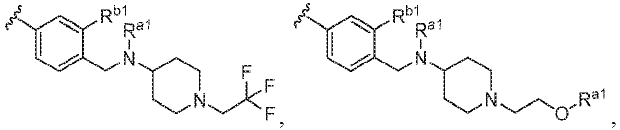 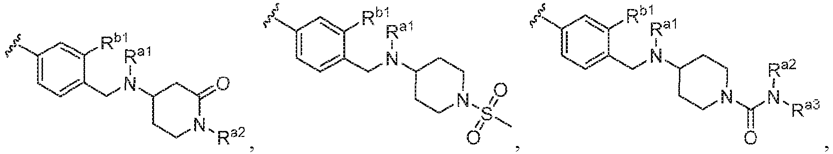 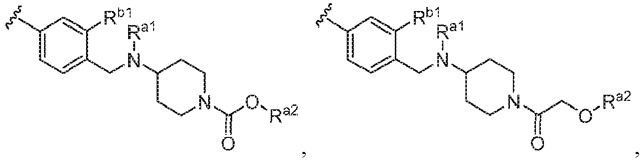 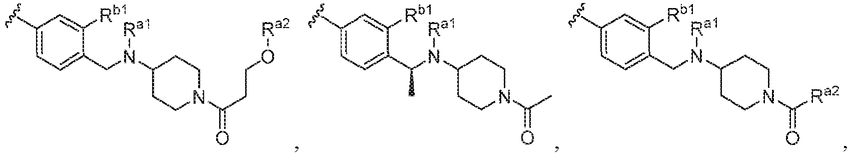 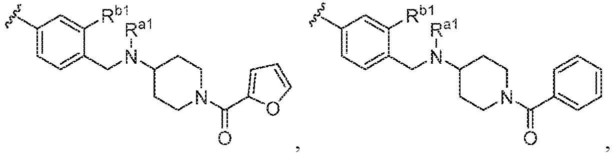 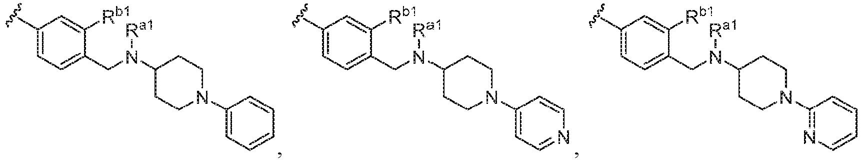 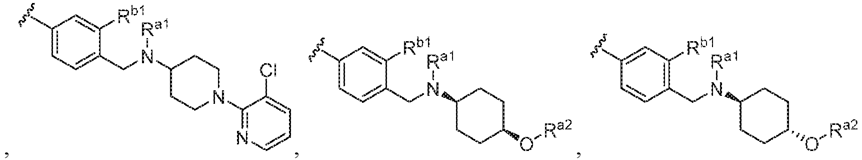 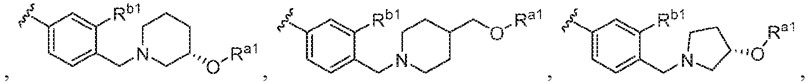 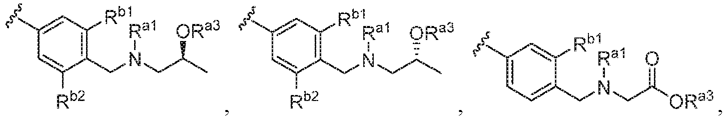 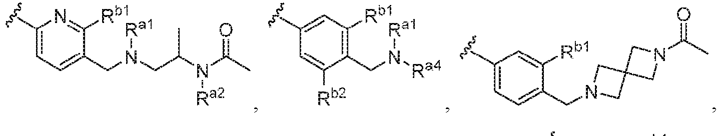 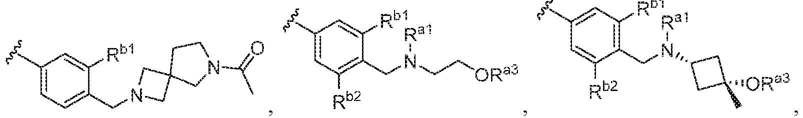 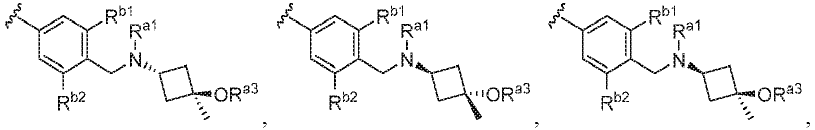 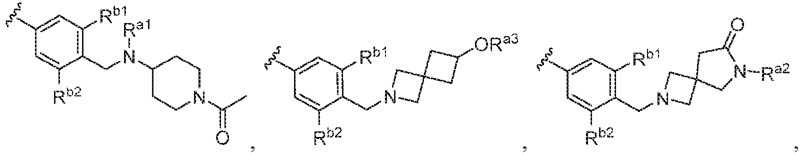 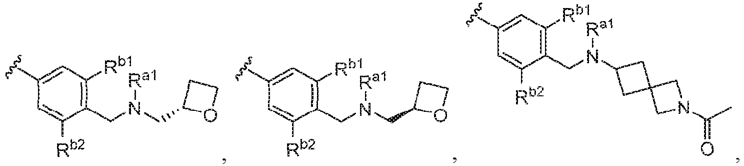 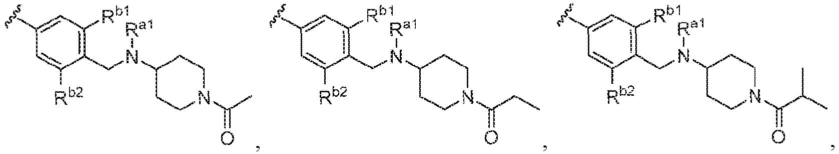 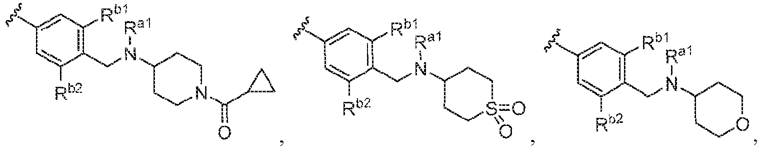 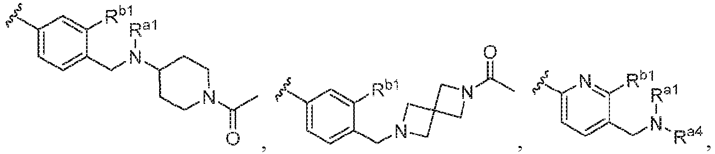 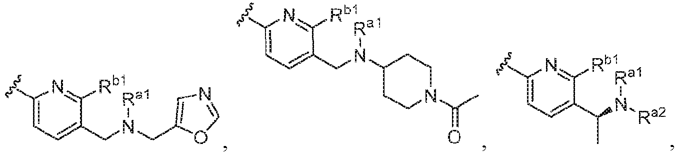 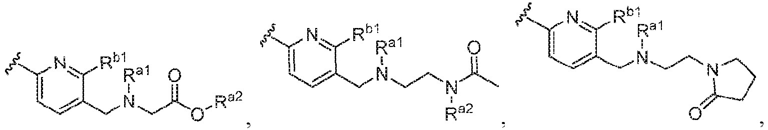 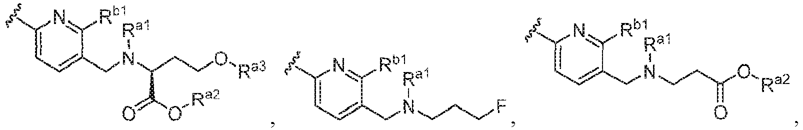 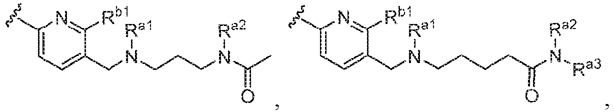 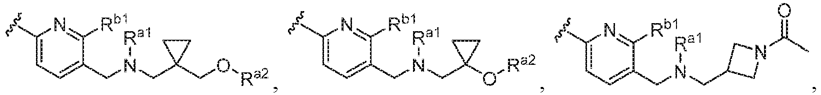 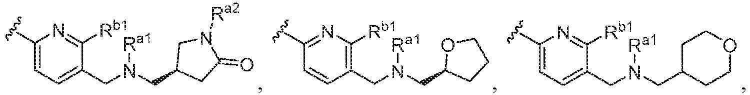 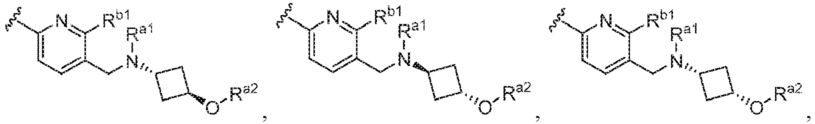 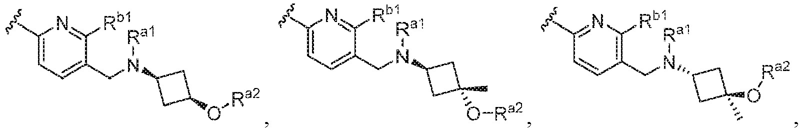 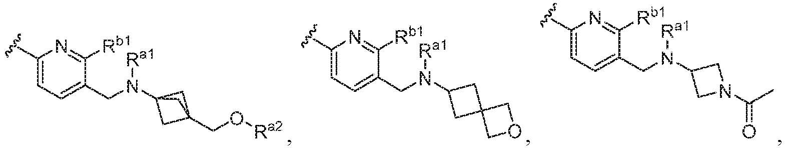 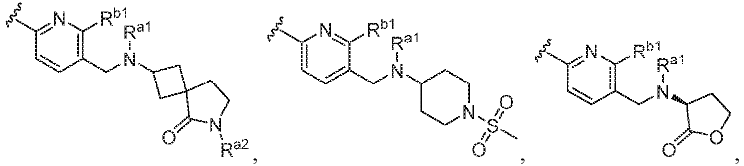 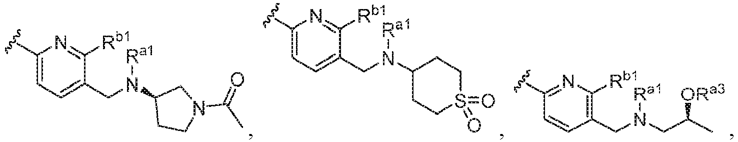 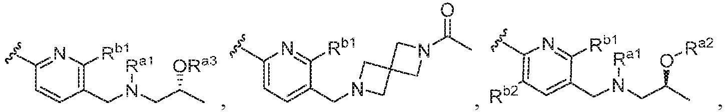 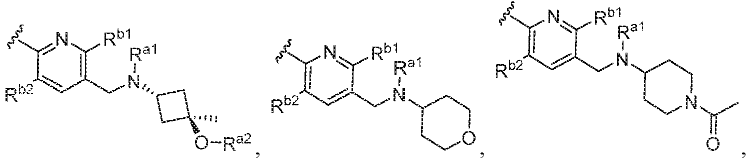 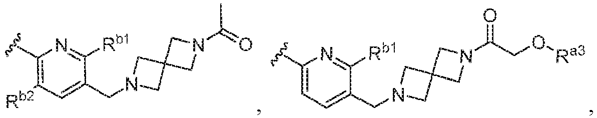 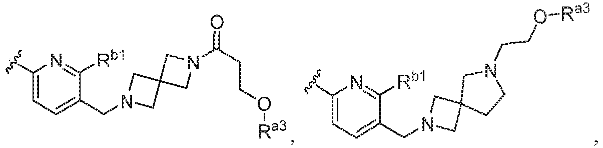 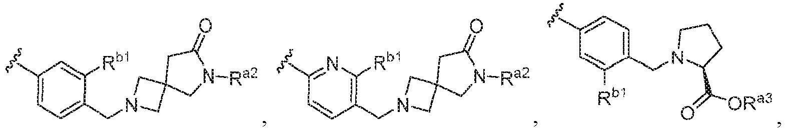 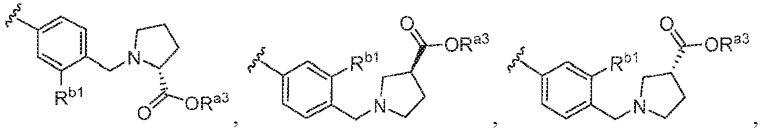 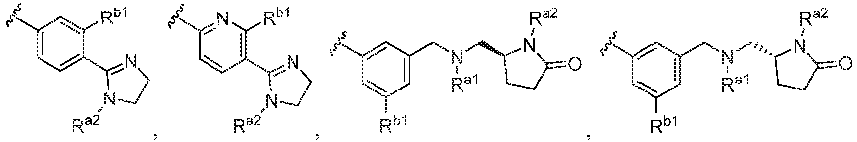 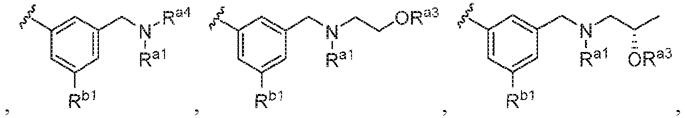 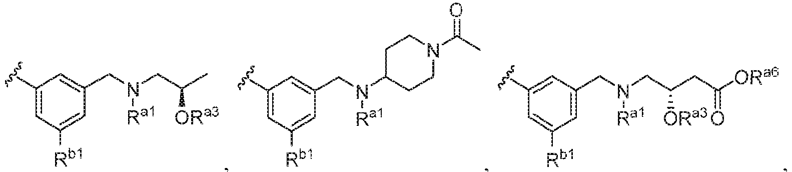 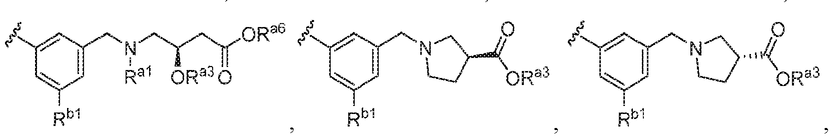 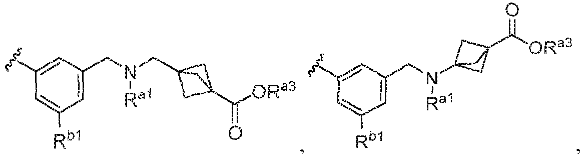 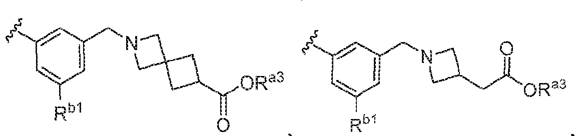 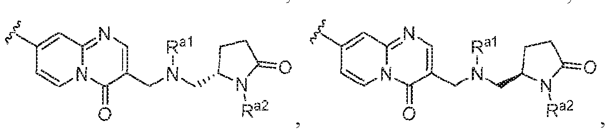 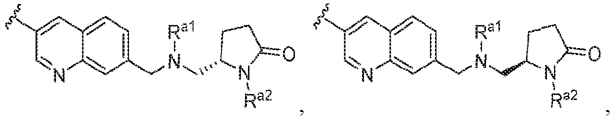 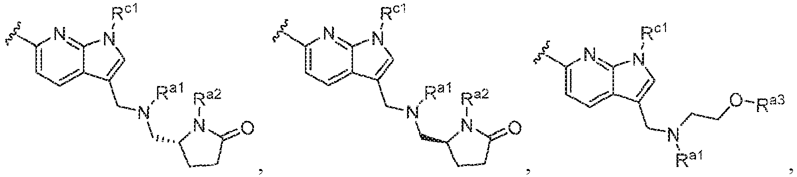 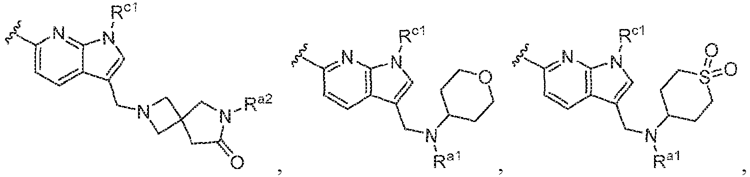 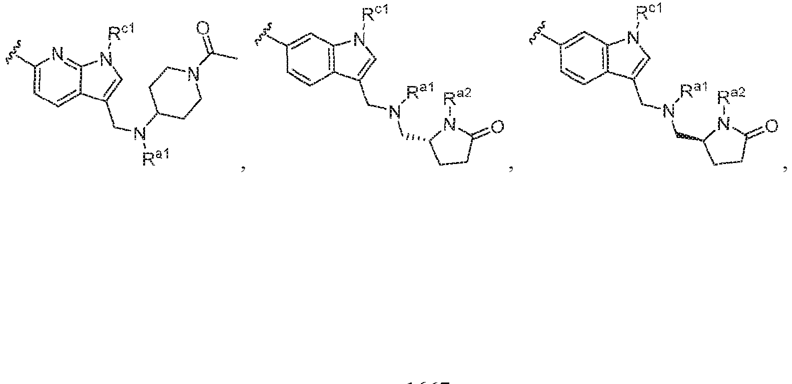 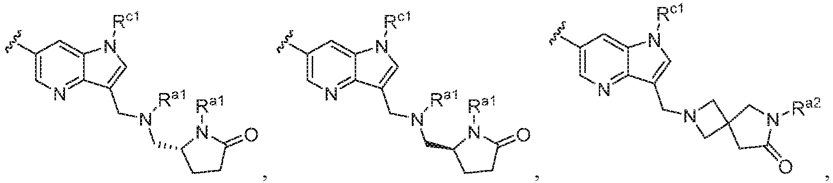 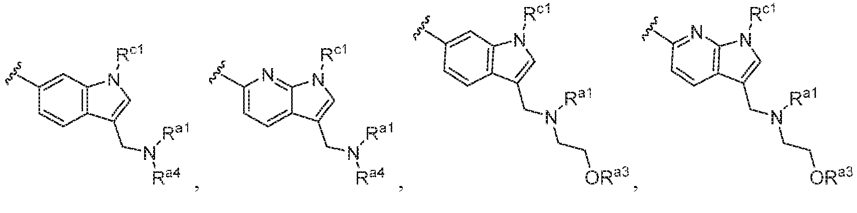 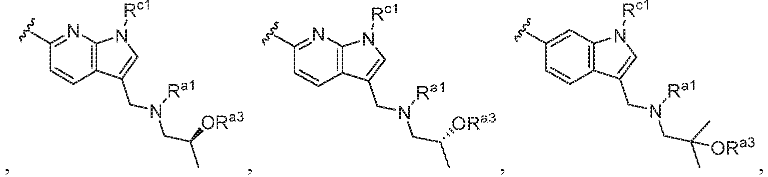 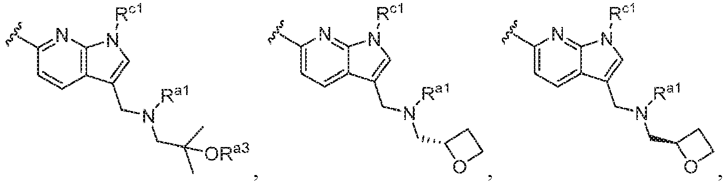 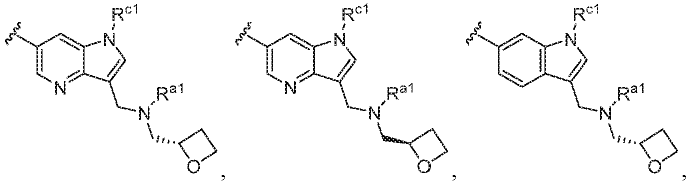 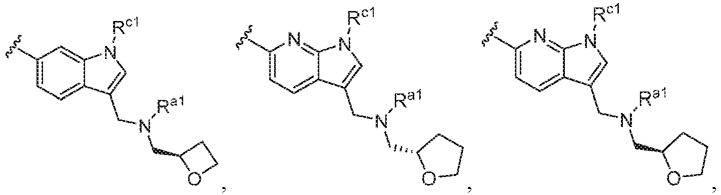 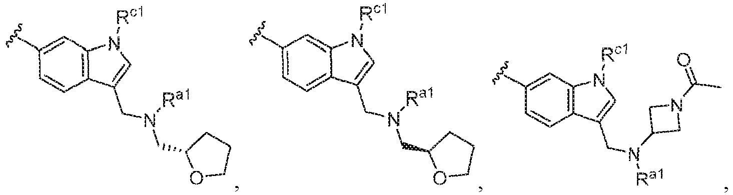 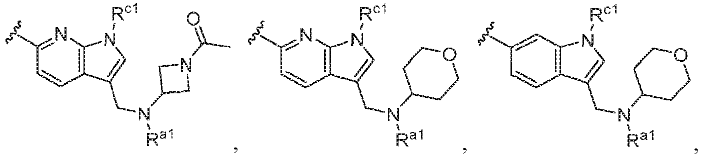 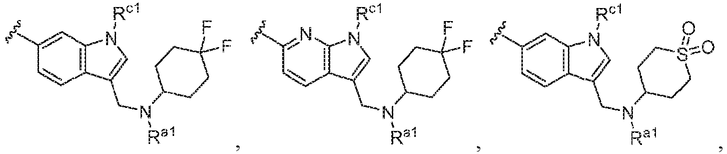 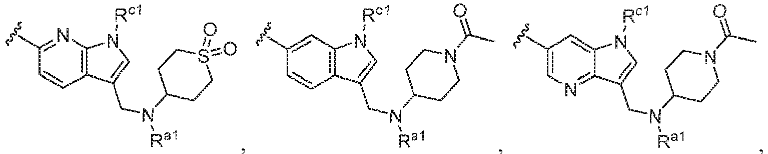 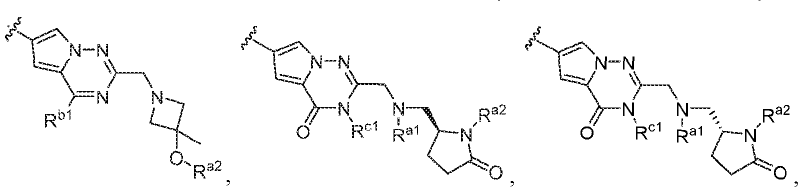 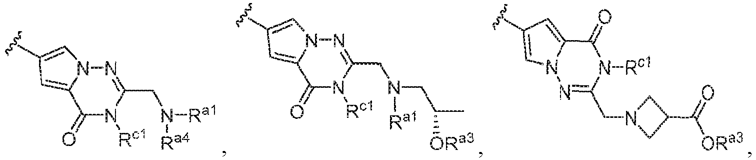 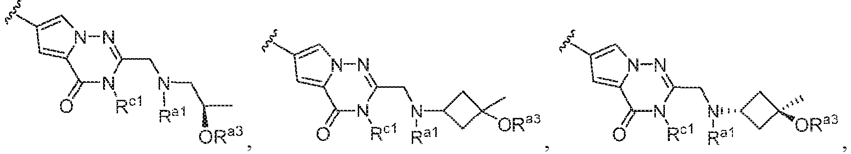 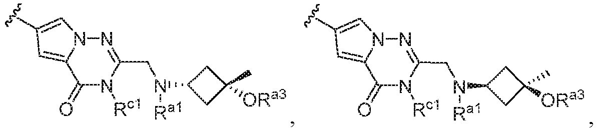 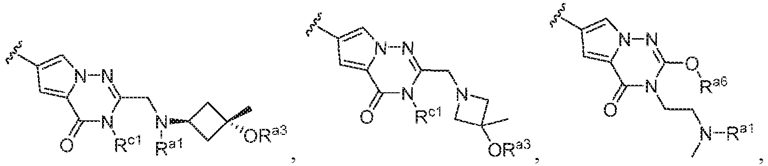 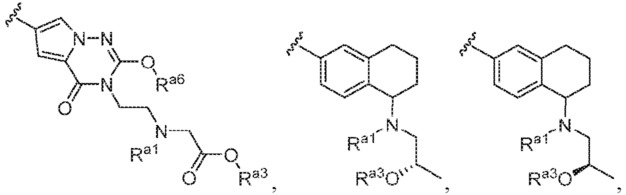 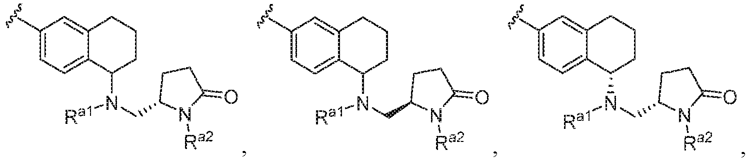 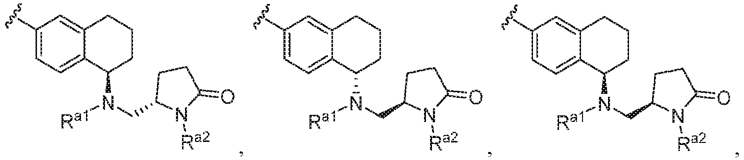 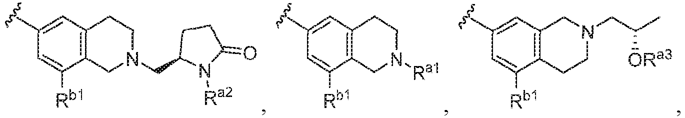 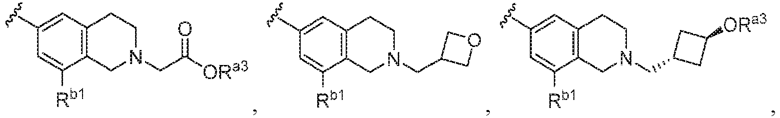 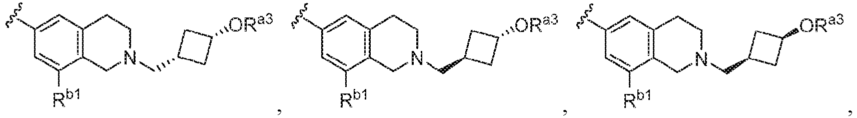 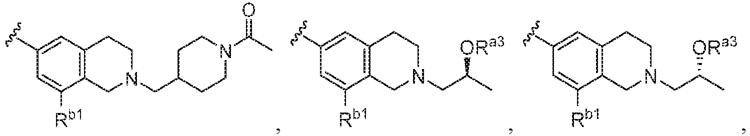 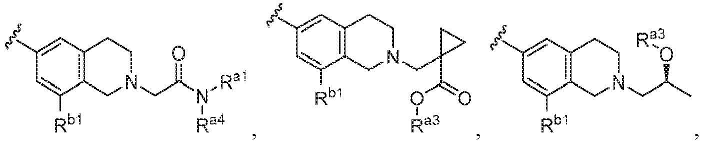 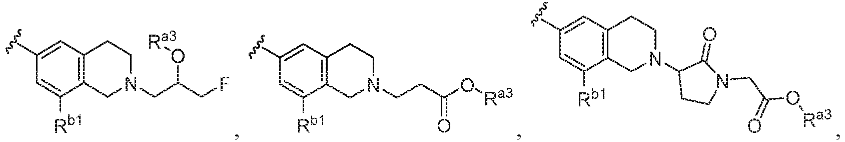 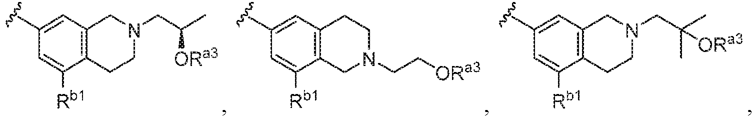 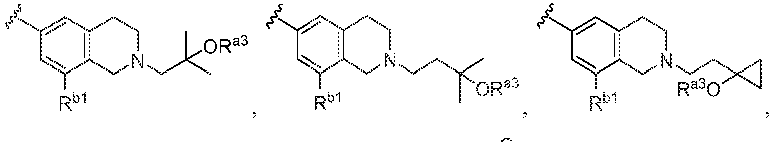 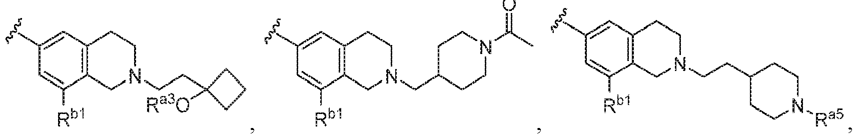 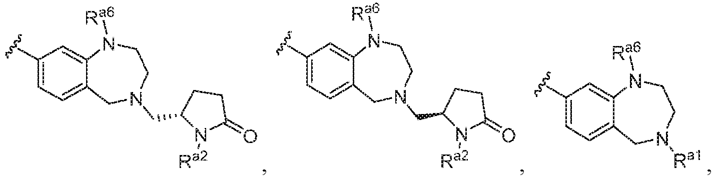 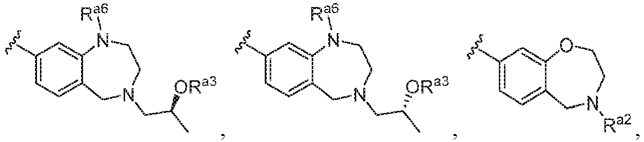 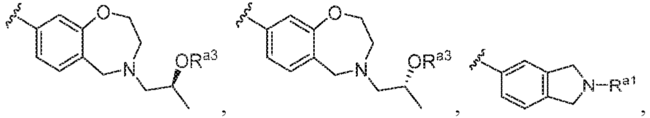 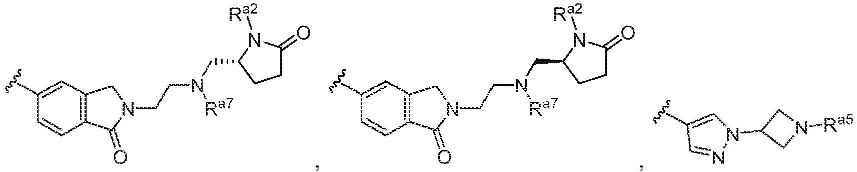 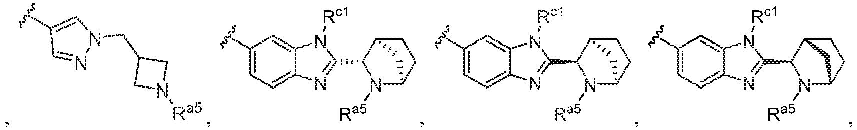 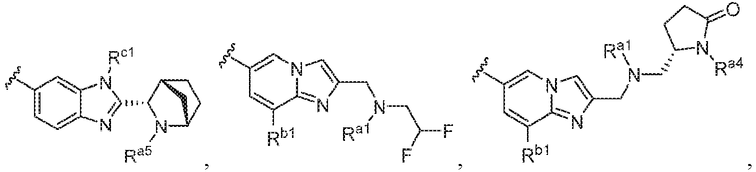 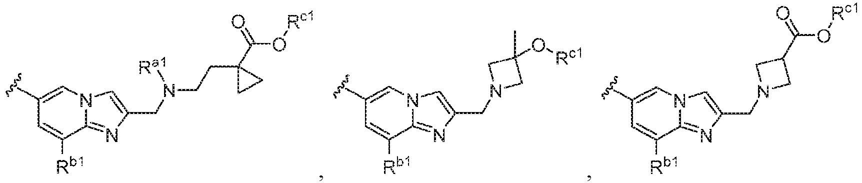 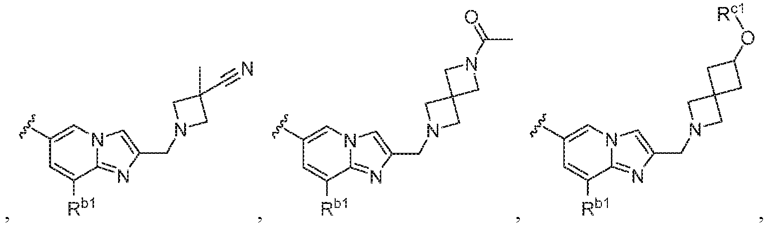 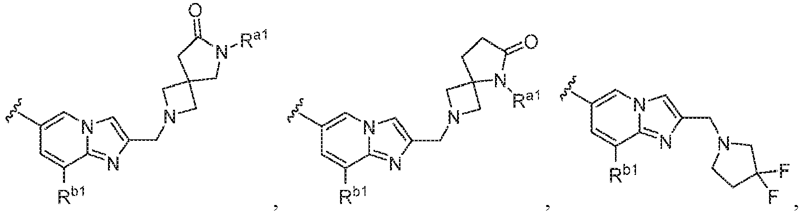 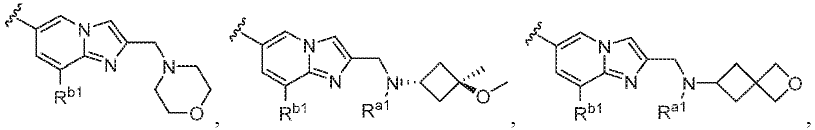 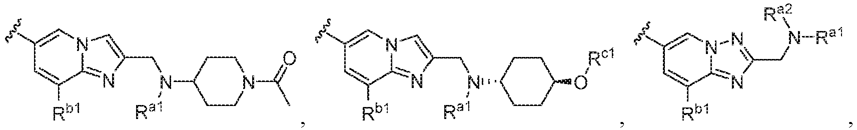 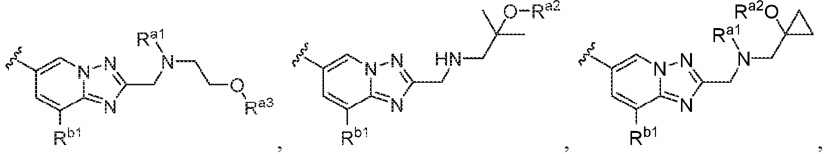 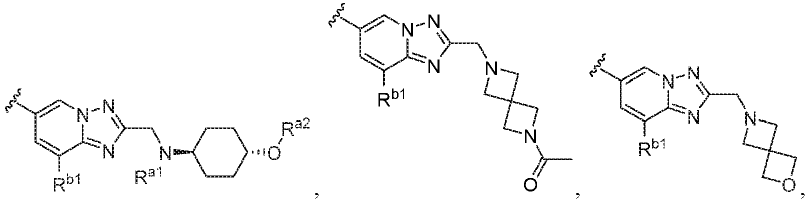 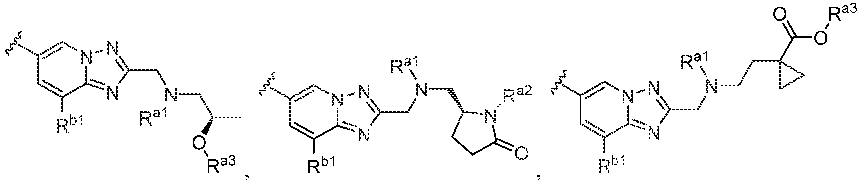 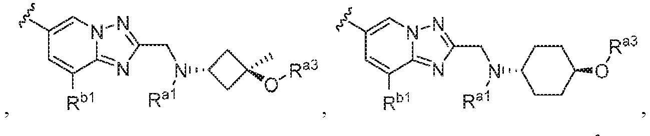 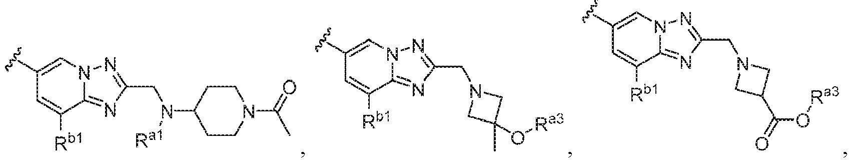 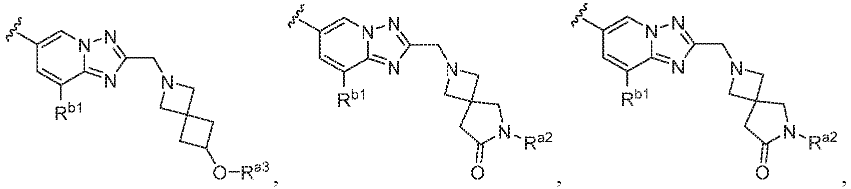 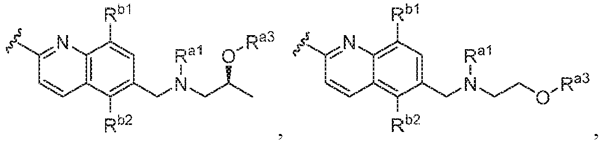 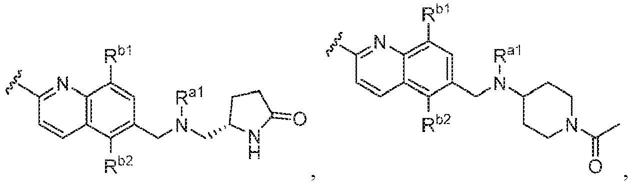 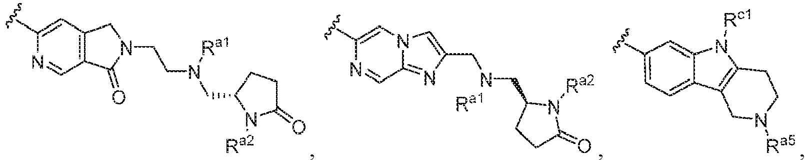 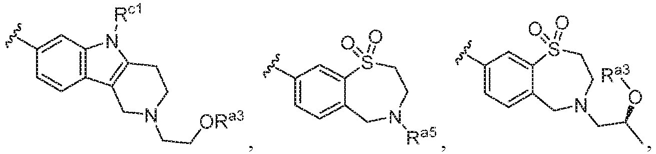 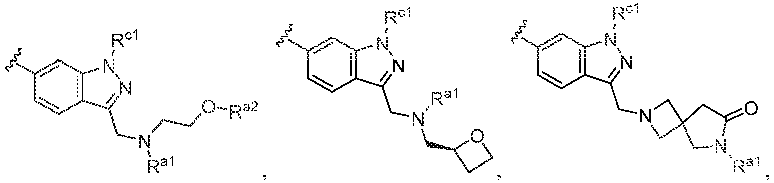 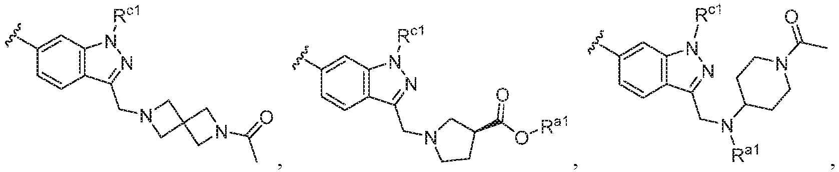 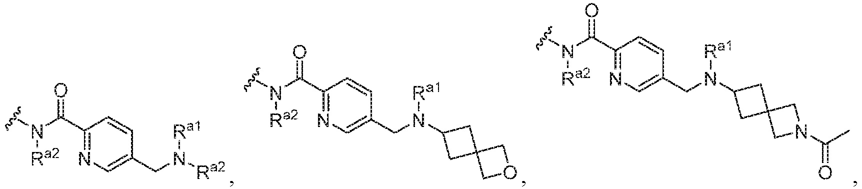 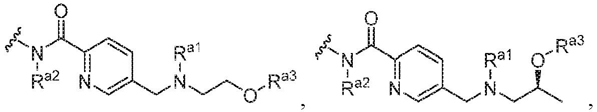 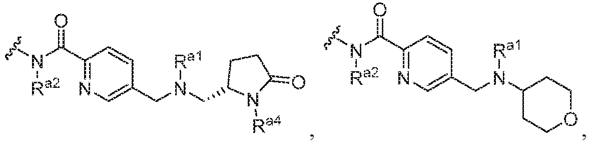 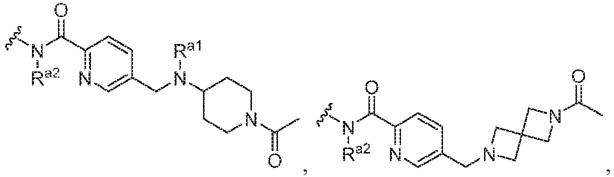 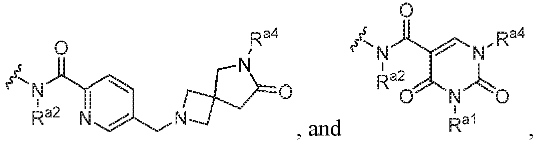 wherein:
   each of R^{a1}, R^{a2}, R^{a3}, R^{a4}, R^{a5}*,* R^{a6}, and R^{a7} is independently selected from the group consisting of H, C₁-C₆ alkyl, C₃-C₈ cycloalkyl, C₁-C₆ alkoxy, C₁-C₆ haloalkyl, and C₁-C₆ haloalkoxy;
   each of R^{b1} and R^{b2} is independently selected from the group consisting of H, C₁-C₃ alkyl, C₃-C₈ cycloalkyl, C₁-C₃ alkoxy, cyano, halogen, C₁-C₃ haloalkyl, and C₁-C₃ haloalkoxy; and
   R^{1c} is selected from the group consisting of H, C₁-C₃ alkyl, C₃-C₈ cycloalkyl, C₁-C₃ alkoxy, C₁-C₃ haloalkyl, C₁-C₃ haloalkoxy, and C(=O)OH.
14. The compound of any one of items 1-13, wherein R^{1c} and R^{1d} are independently selected from the group consisting of: 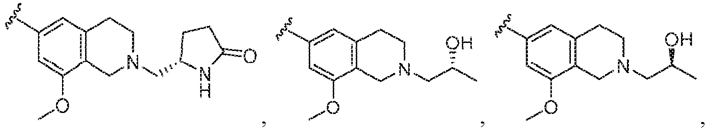 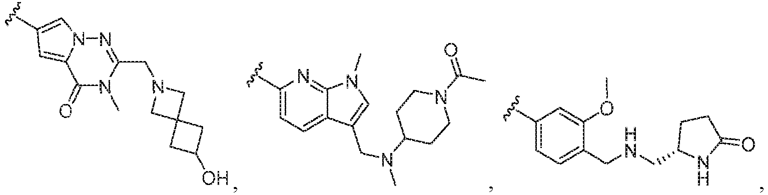 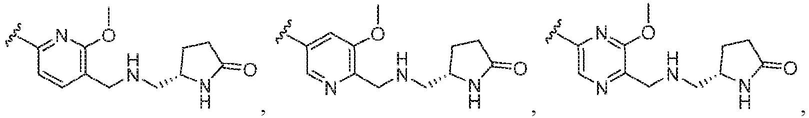 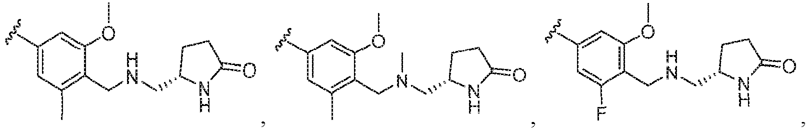 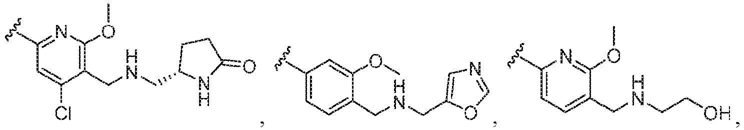 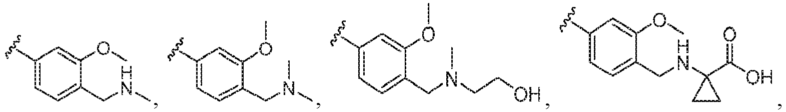 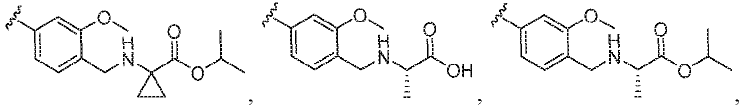 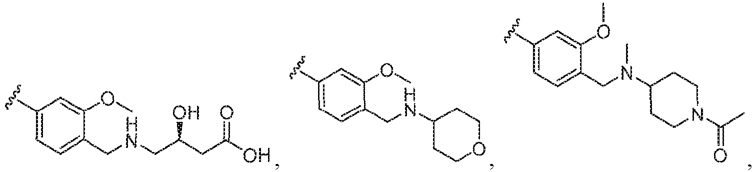 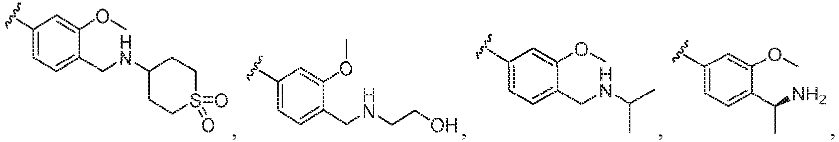 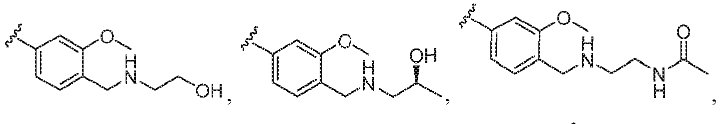 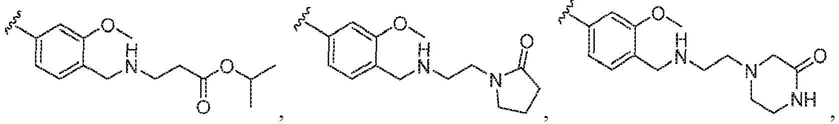 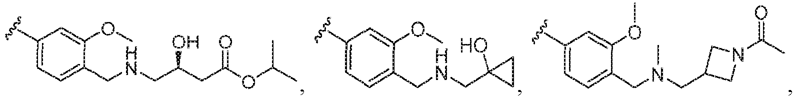 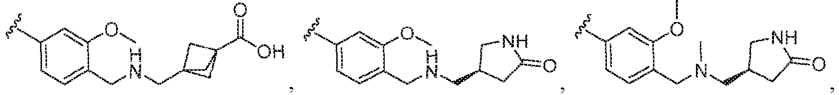 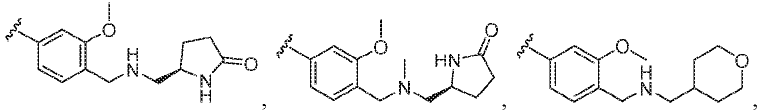 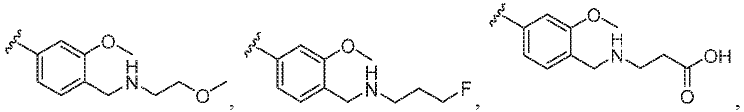 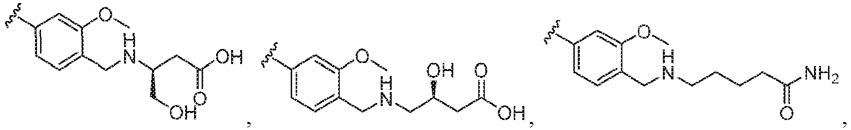 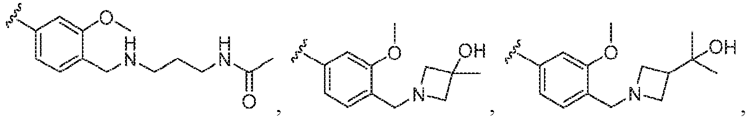 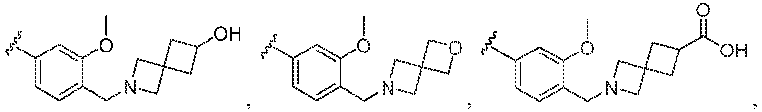 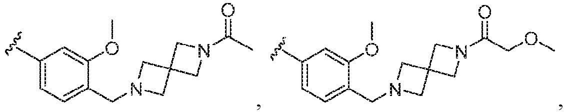 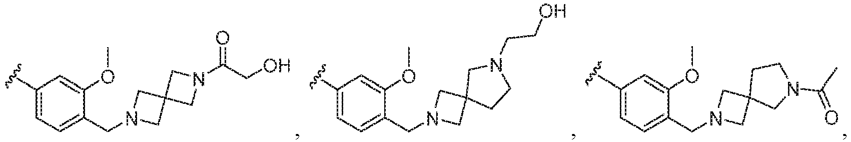 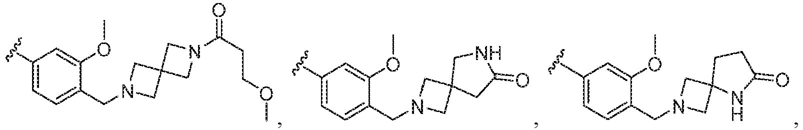 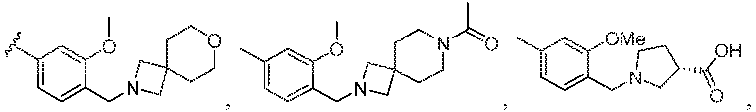 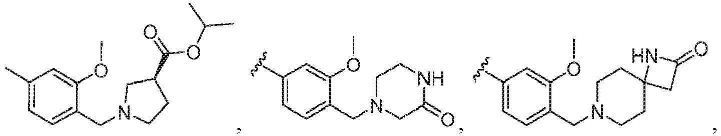 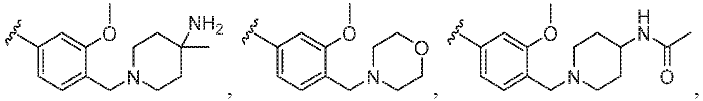 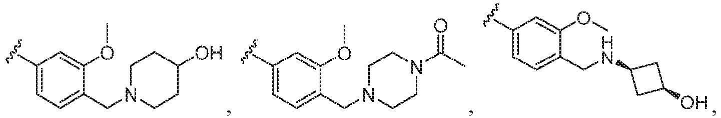 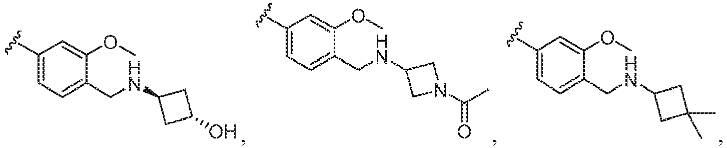 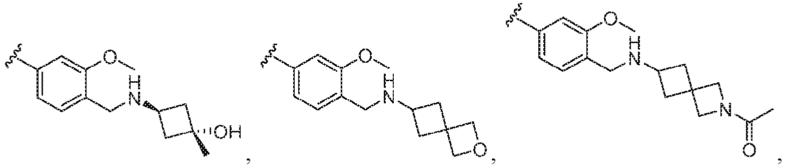 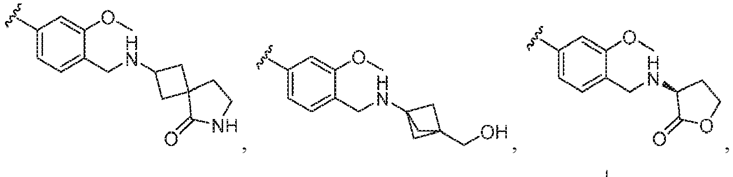 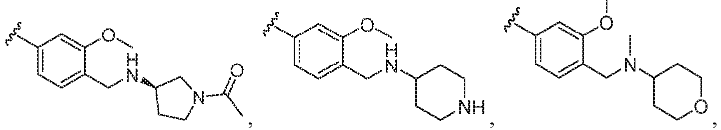 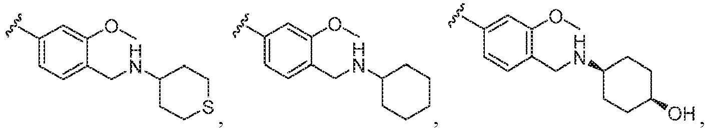 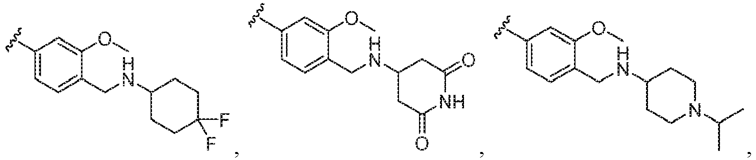 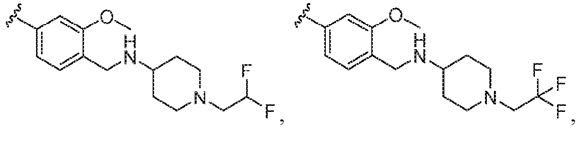 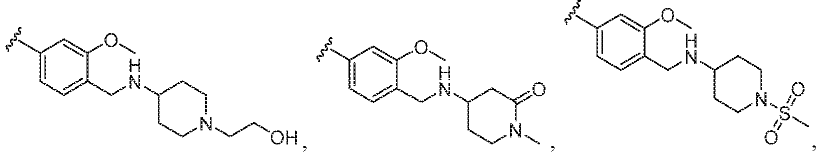 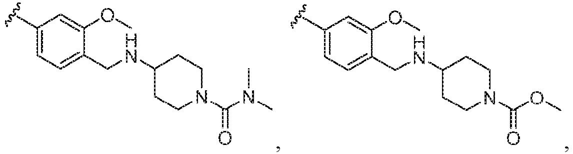 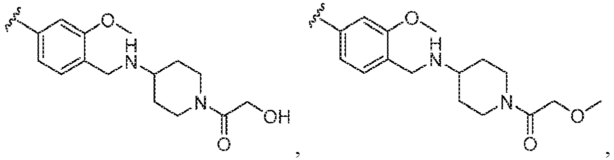 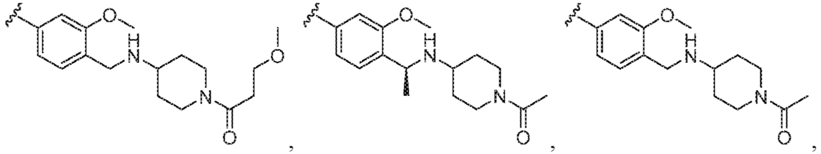 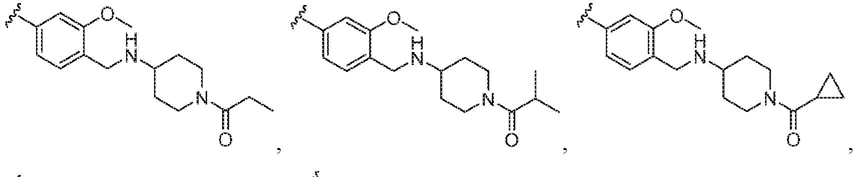 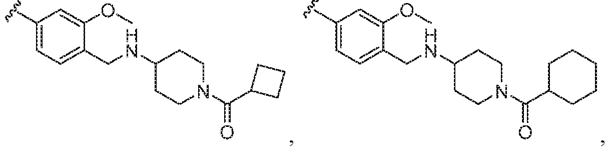 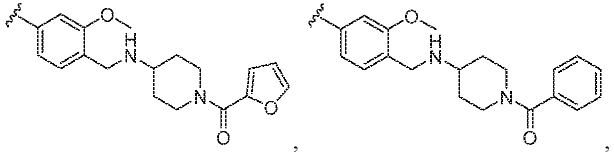 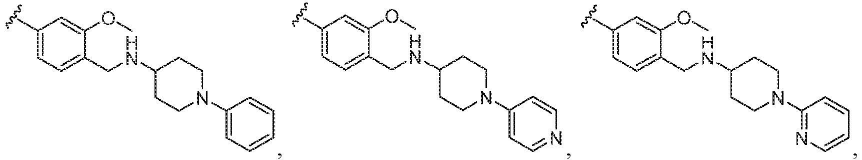 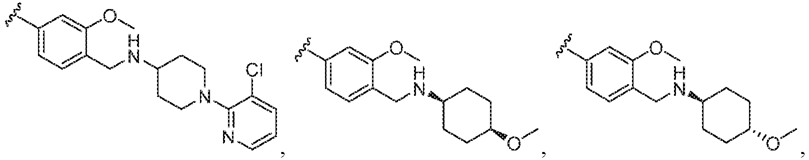 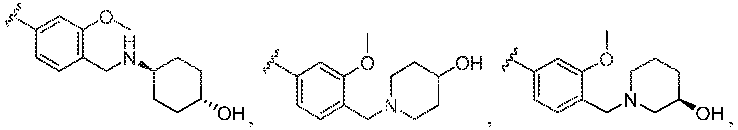 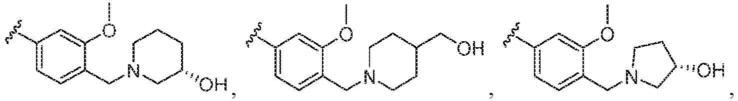 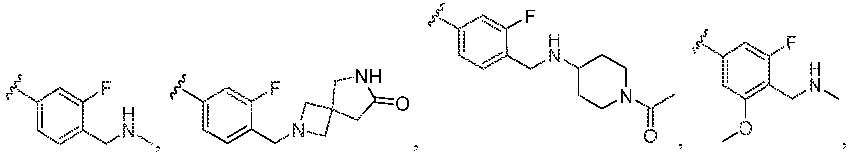 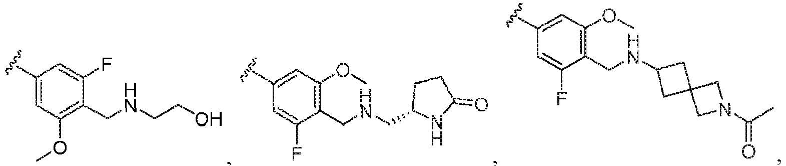 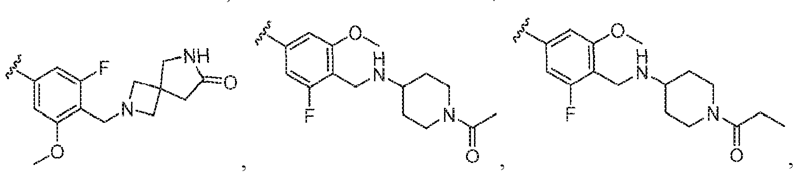 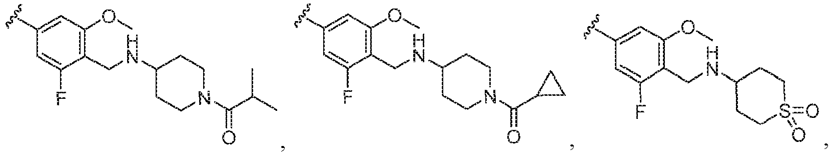 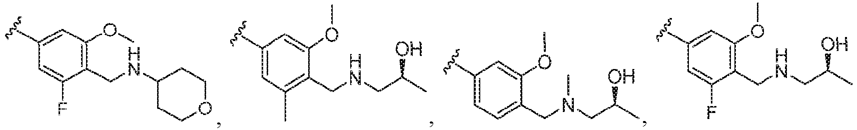 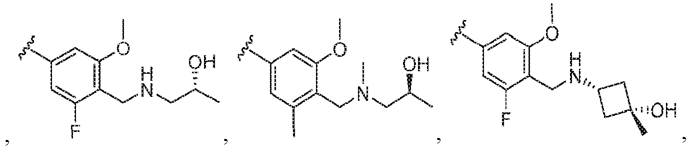 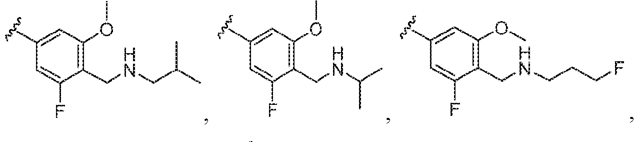 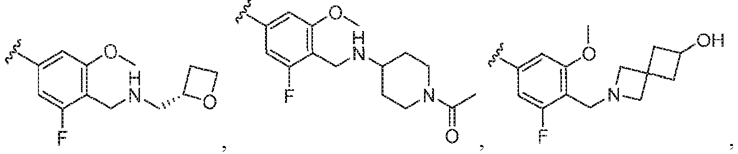 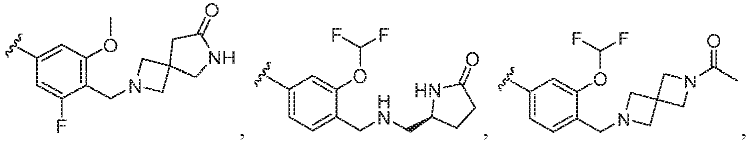 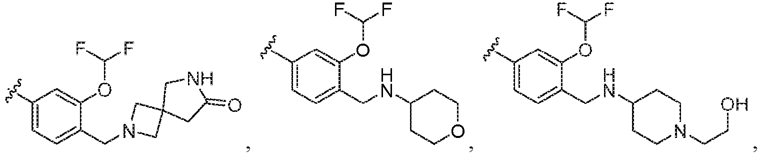 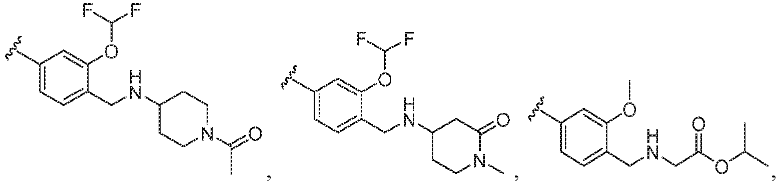 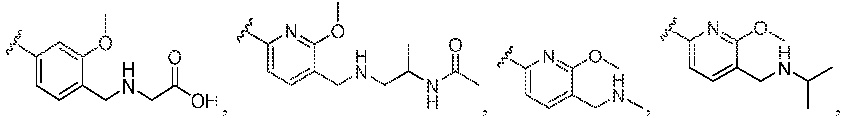 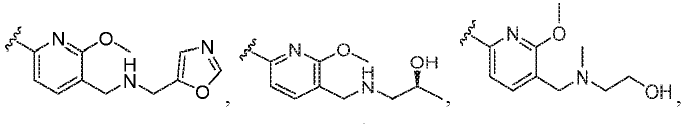 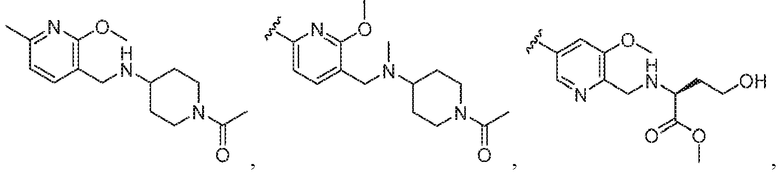 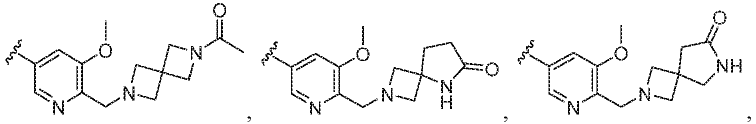 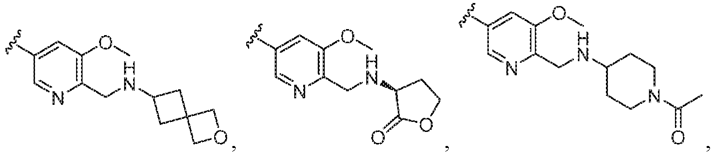 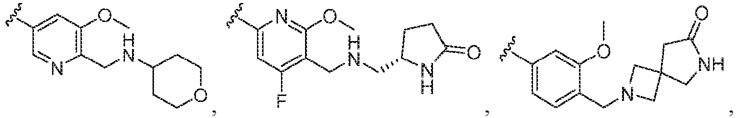 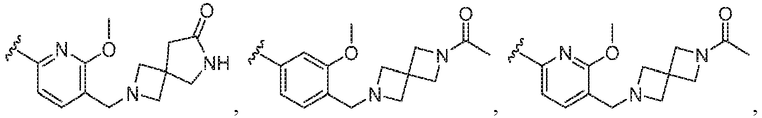 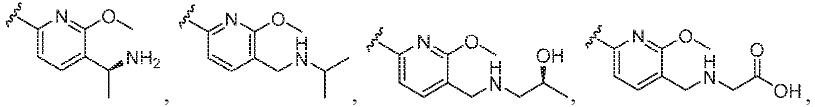 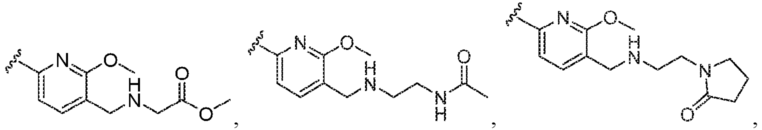 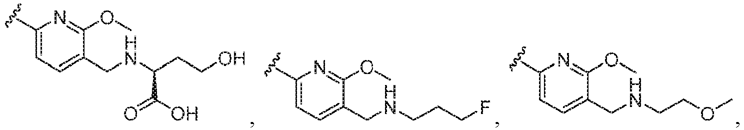 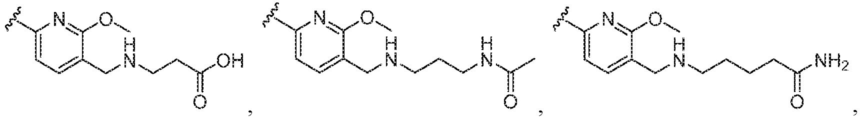 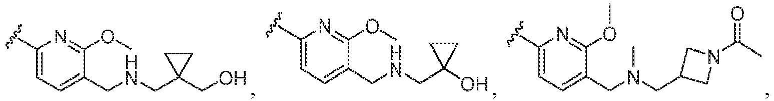 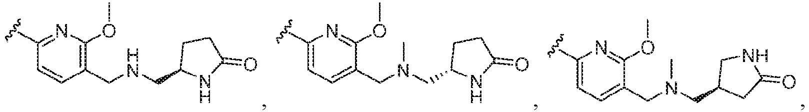 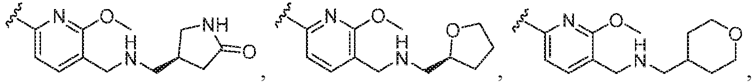 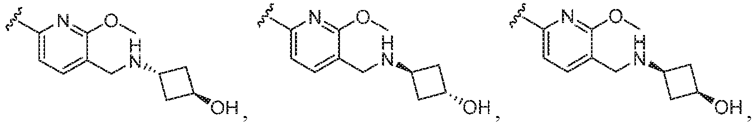 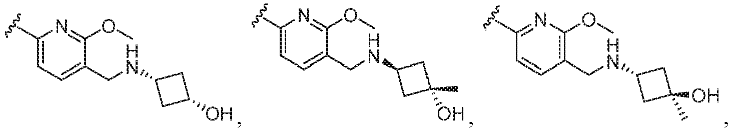 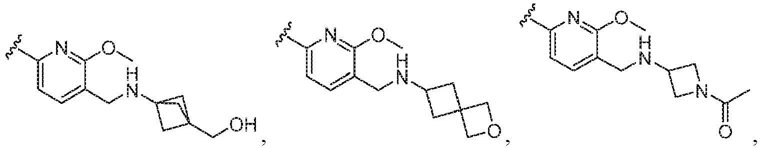 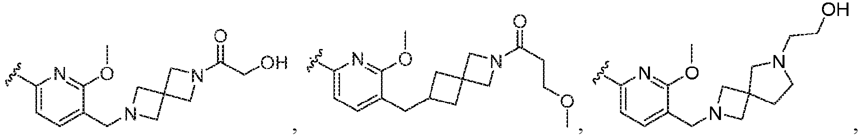 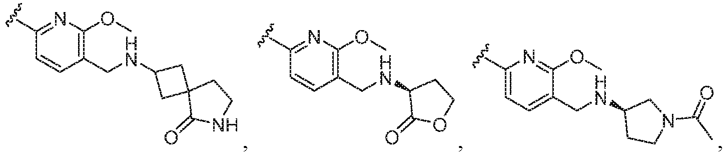 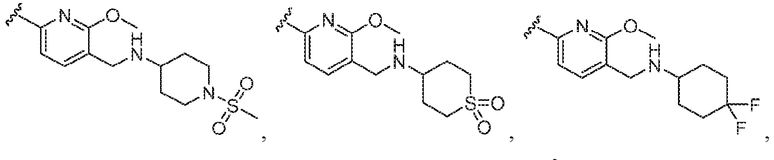 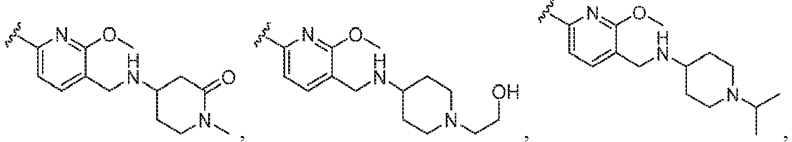 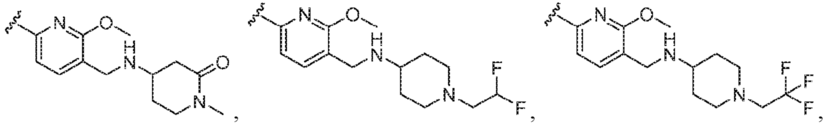 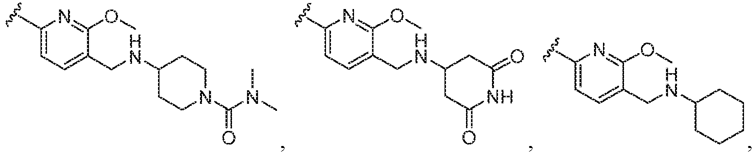 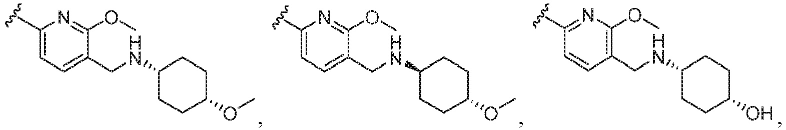 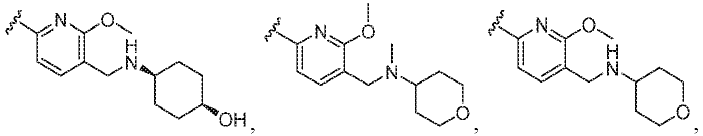 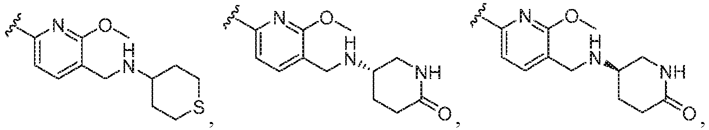 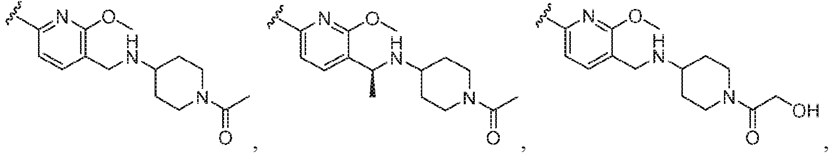 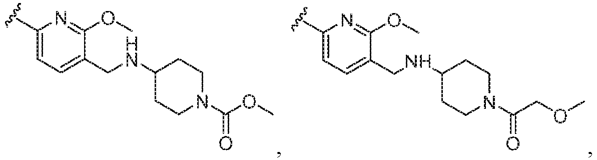 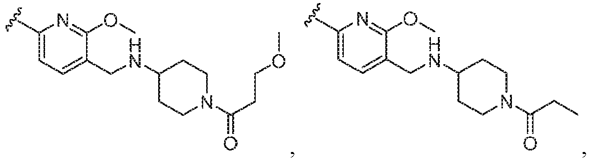 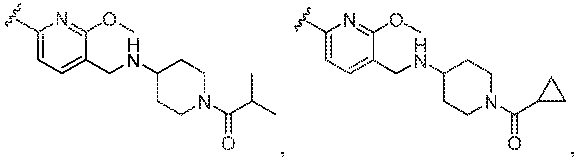 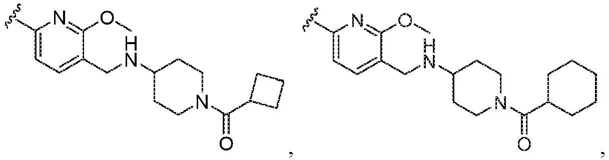 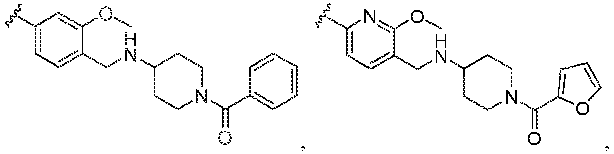 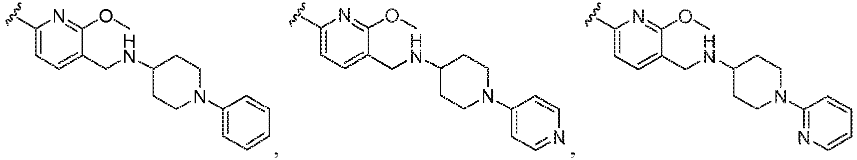 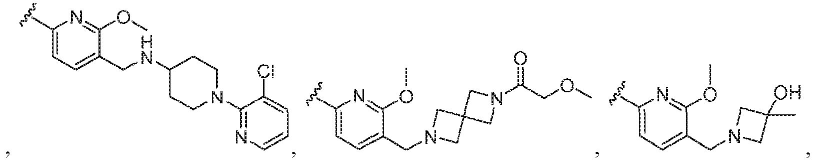 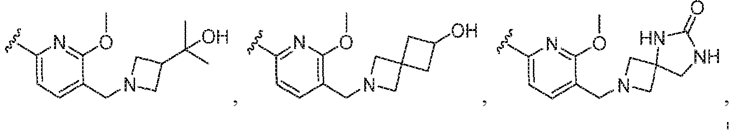 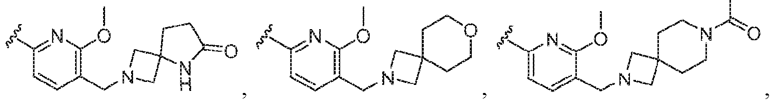 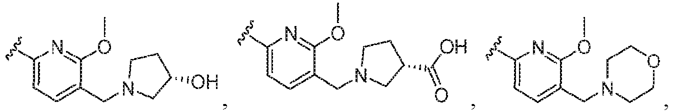 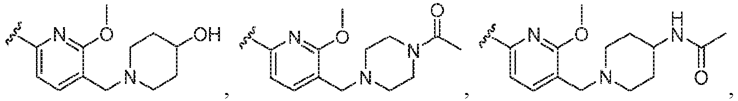 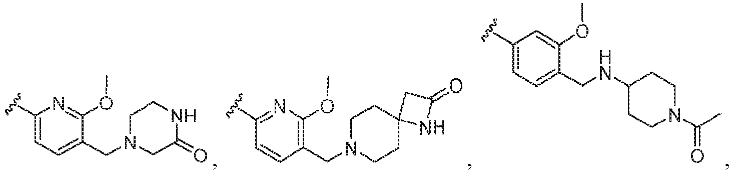 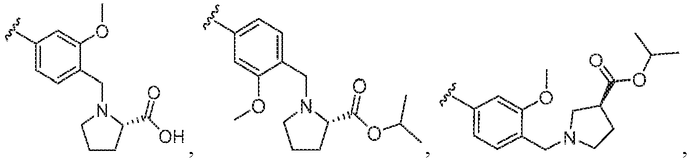 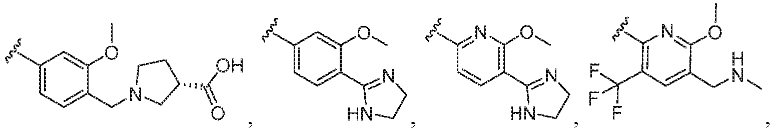 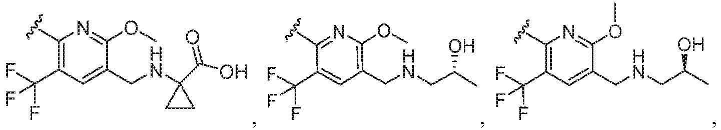 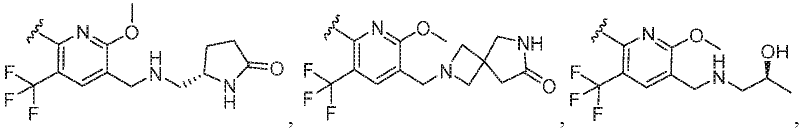 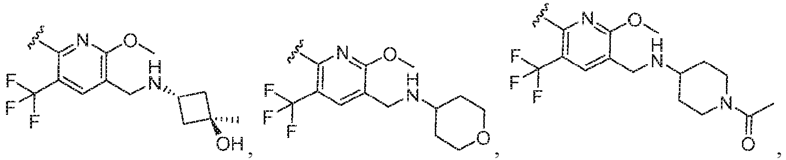 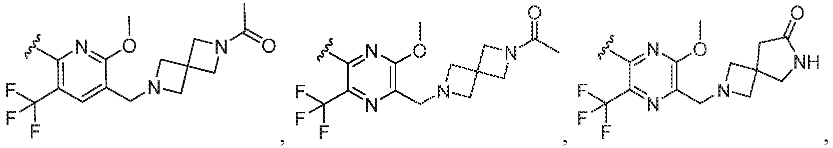 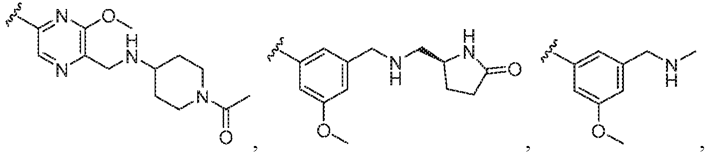 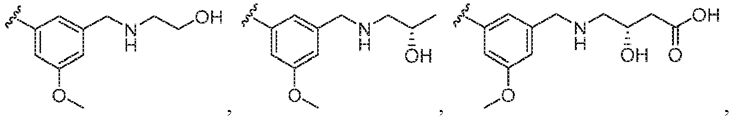 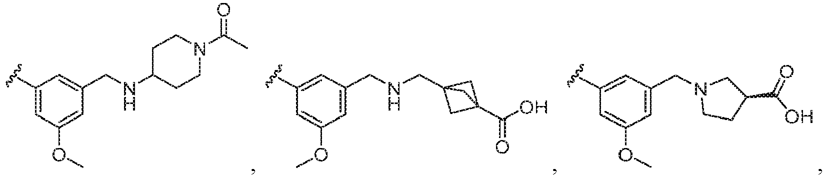 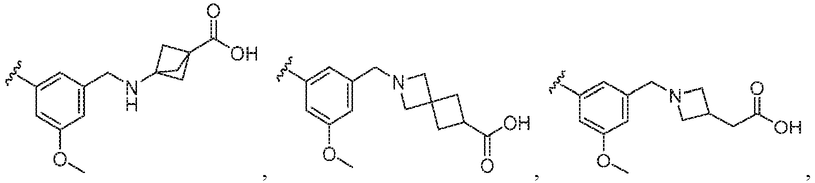 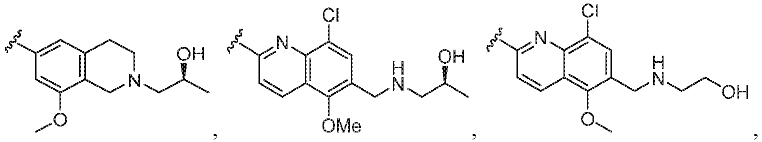 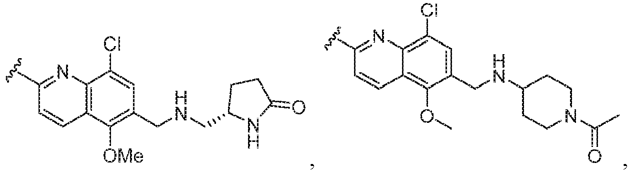 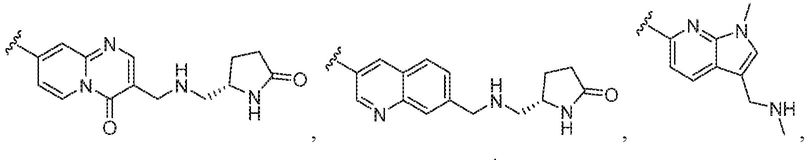 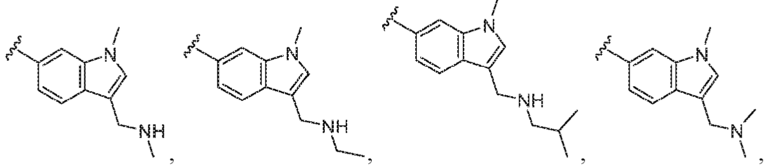 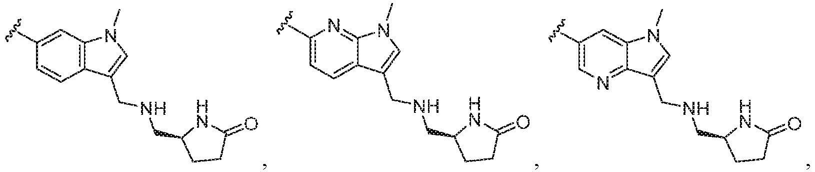 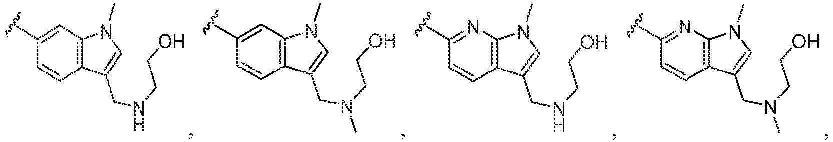 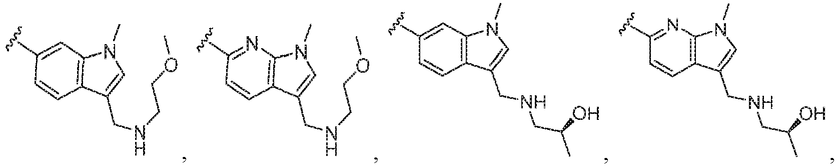 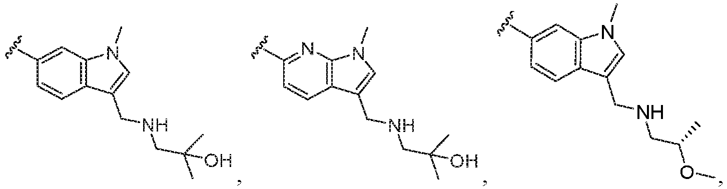 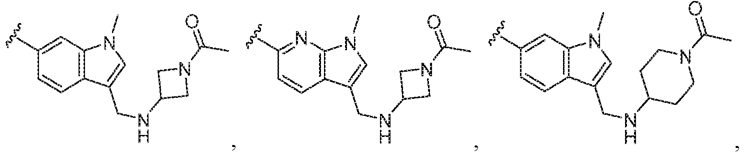 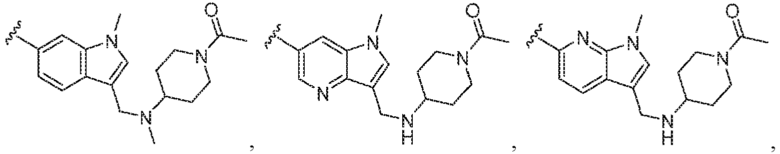 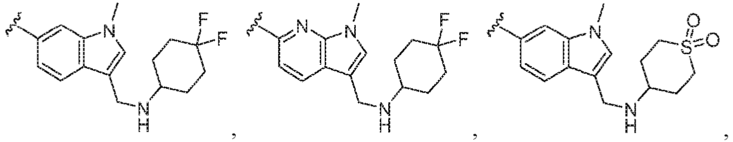 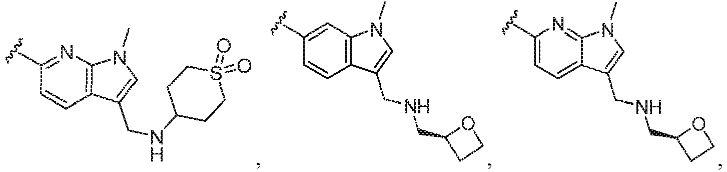 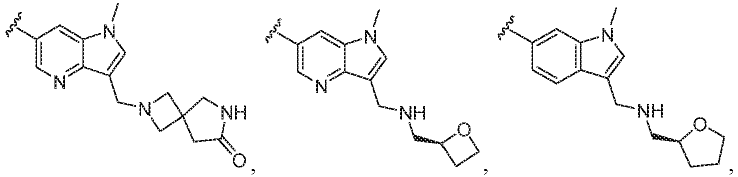 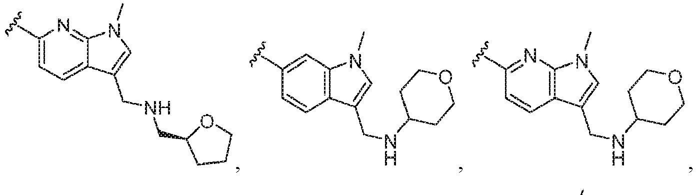 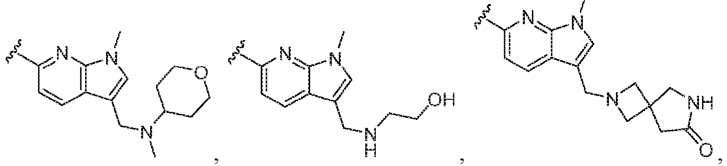 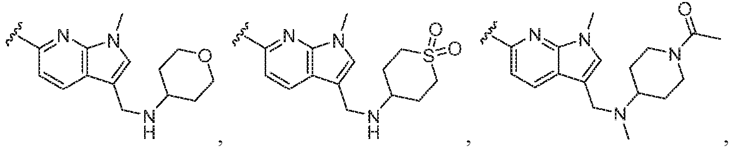 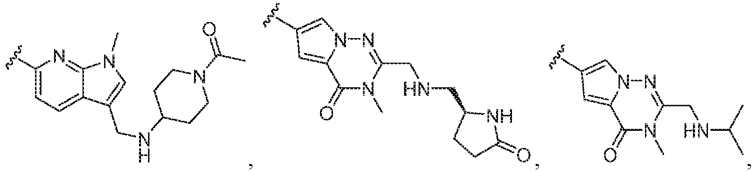 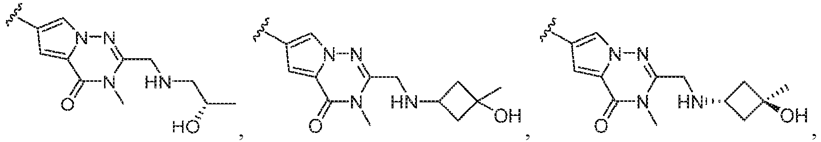 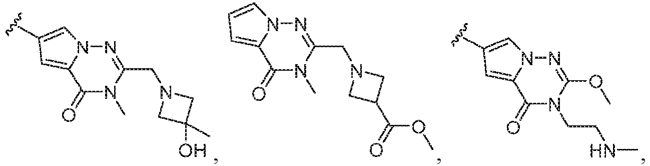 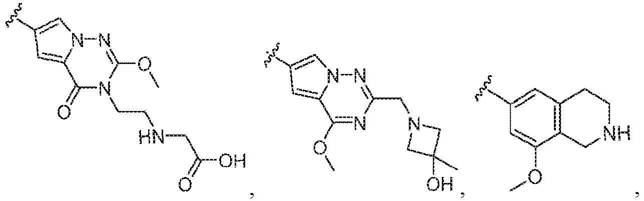 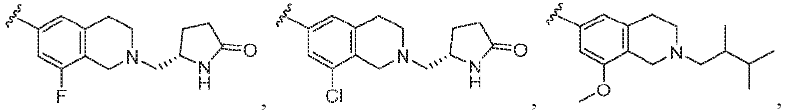 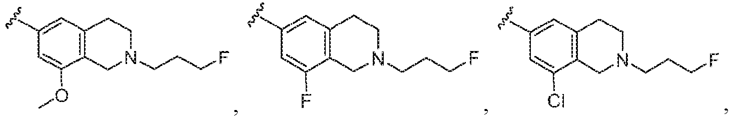 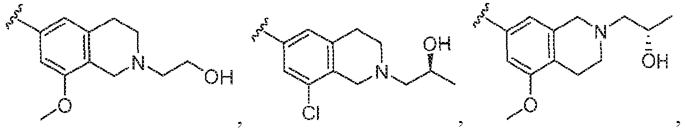 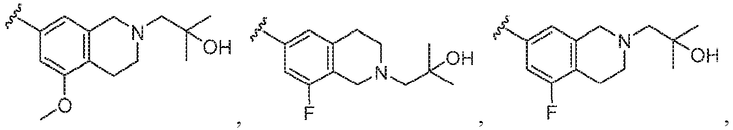 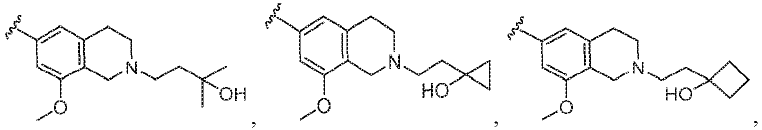 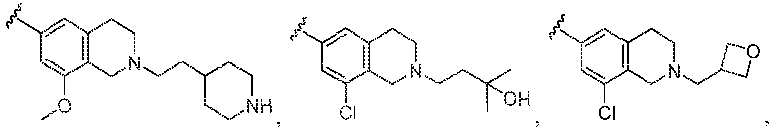 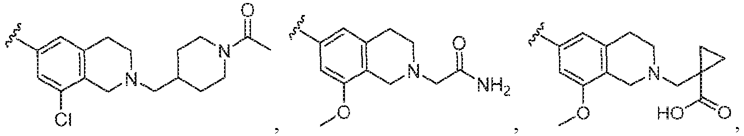 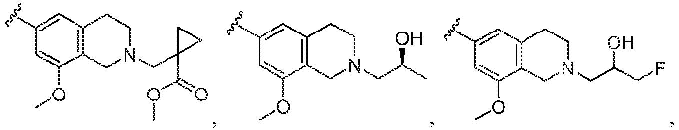 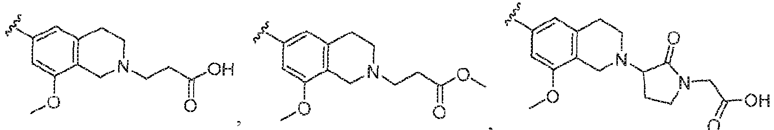 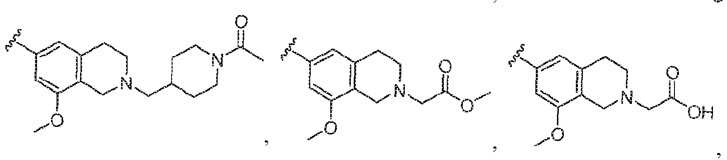 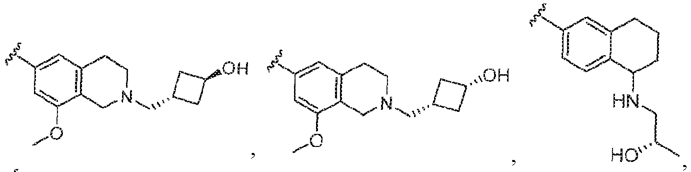 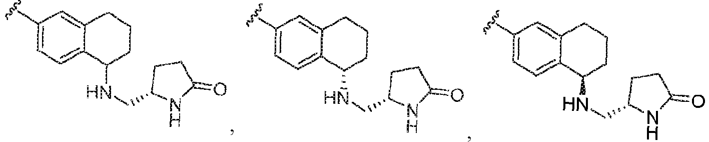 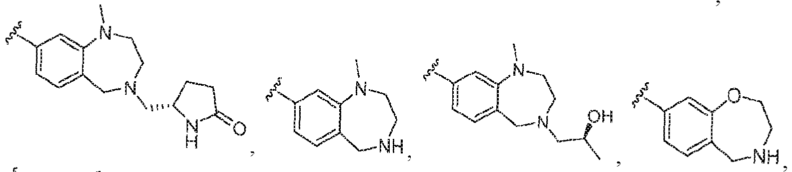 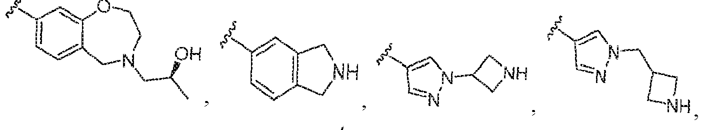 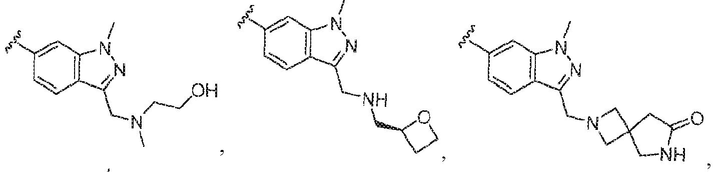 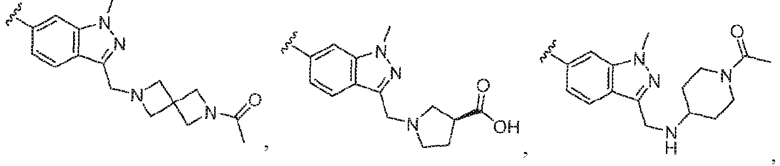 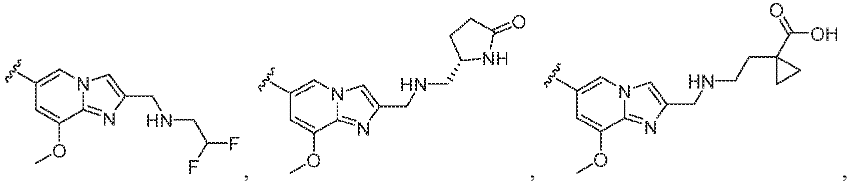 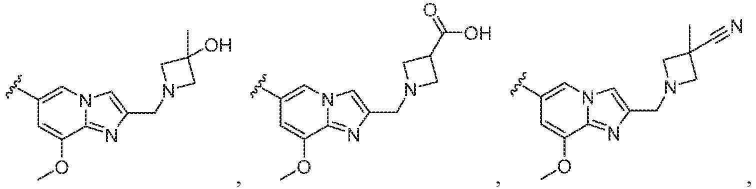 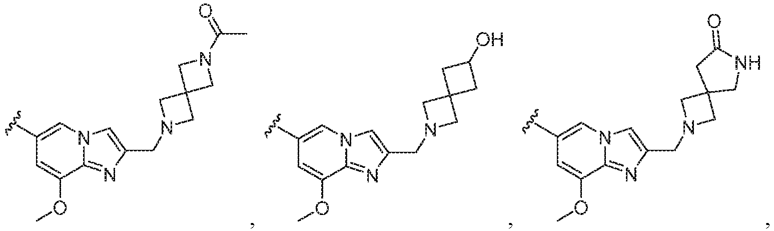 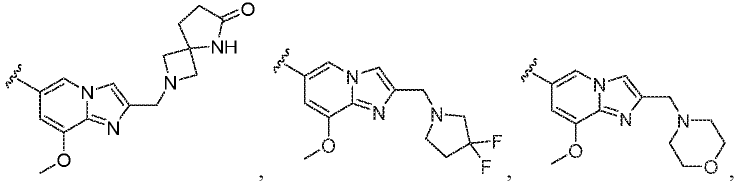 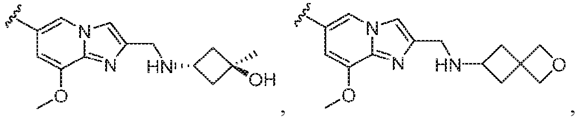 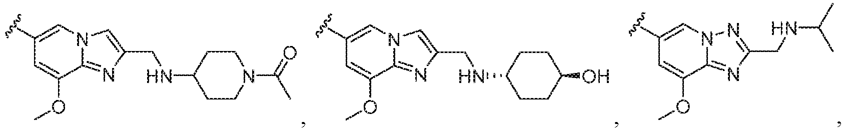 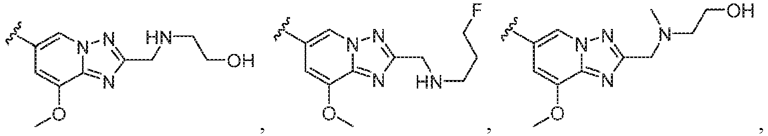 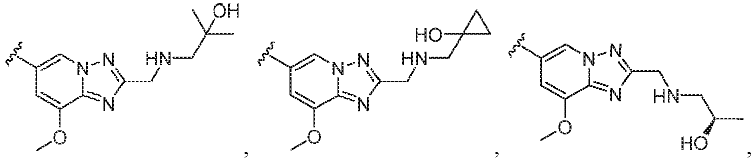 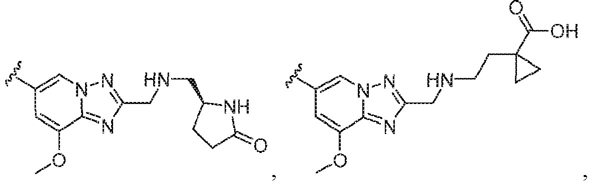 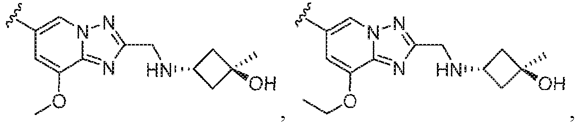 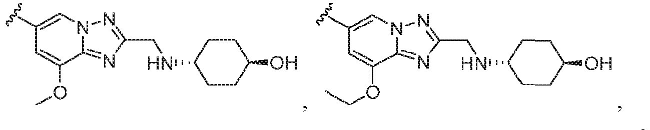 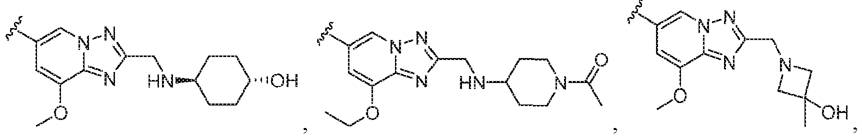 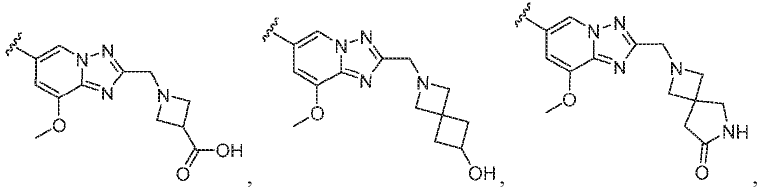 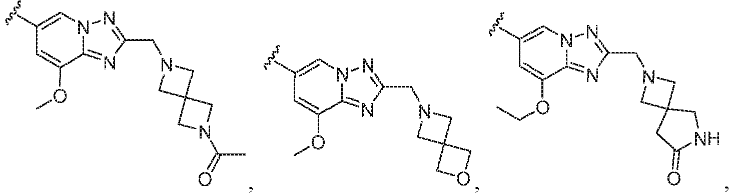 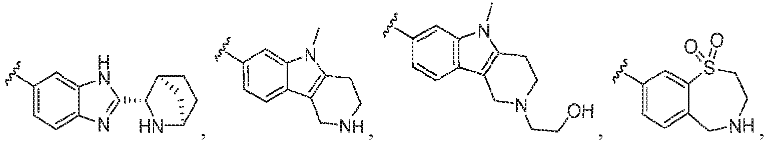 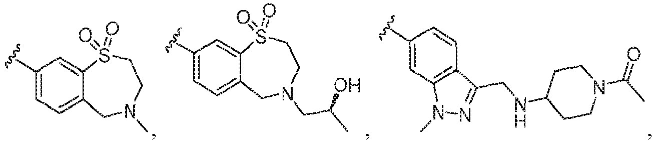 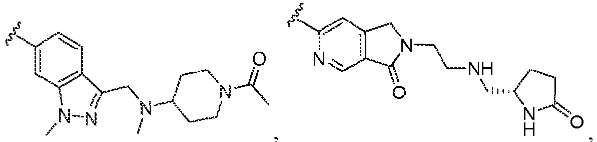 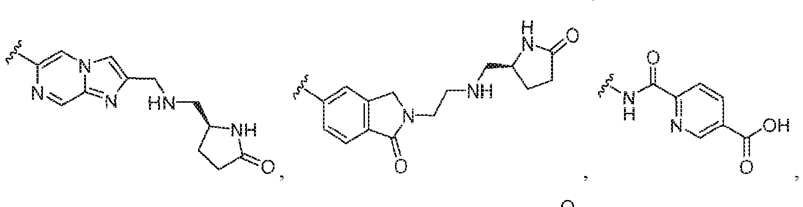 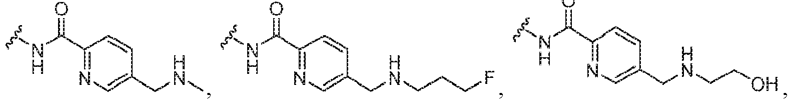 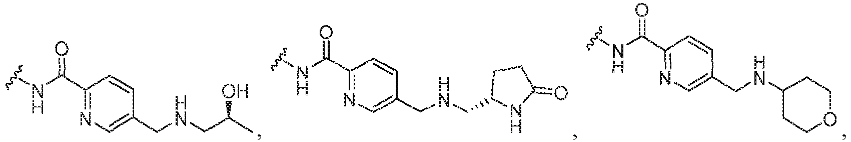 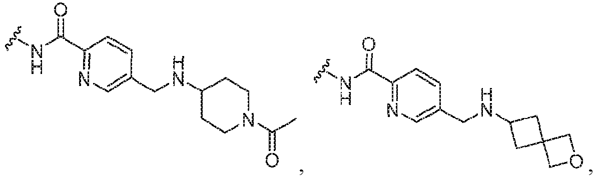 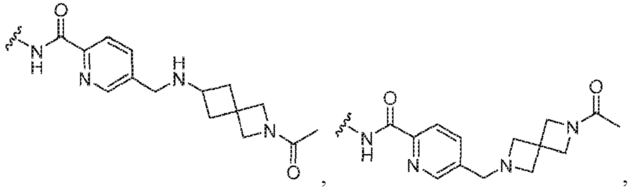 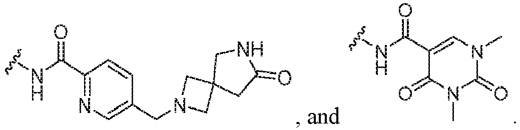
15. The compound of any one of items 1-14, which is selected from the group consisting of:
   8-(2-chloro-3-(3-chloro-2-(3-methoxy-4-(((((S)-5-oxopyrrolidin-2-yl)methyl)amino)methyl)phenyl)pyridin-4-yl)phenyl)-3-(((((S)-5-oxopyrrolidin-2-yl)methyl)amino)methyl)-4H-pyrido[1,2-a]pyrimidin-4-one;
   8-(2-chloro-3-(3-chloro-2-(3-methoxy-4-(((((S)-5-oxopyrrolidin-2-yl)methyl)amino)methyl)phenyl)pyridin-4-yl)phenyl)-3-(((((R)-5-oxopyrrolidin-2-yl)methyl)amino)methyl)-4H-pyrido[1,2-a]pyrimidin-4-one;
   8-(2-chloro-3-(3-chloro-2-(3-methoxy-4-(((((R)-5-oxopyrrolidin-2-yl)methyl)amino)methyl)phenyl)pyridin-4-yl)phenyl)-3-(((((S)-5-oxopyrrolidin-2-yl)methyl)amino)methyl)-4H-pyrido[1,2-a]pyrimidin-4-one;
   8-(2-chloro-3-(3-chloro-2-(3-methoxy-4-(((((R)-5-oxopyrrolidin-2-yl)methyl)amino)methyl)phenyl)pyridin-4-yl)phenyl)-3-(((((R)-5-oxopyrrolidin-2-yl)methyl)amino)methyl)-4H-pyrido[1,2-a]pyrimidin-4-one;
   8-(3-chloro-4-(2-chloro-3-(6-methoxy-5-(((((S)-5-oxopyrrolidin-2-yl)methyl)amino)methyl)pyridin-2-yl)phenyl)pyridin-2-yl)-3-(((((S)-5-oxopyrrolidin-2-yl)methyl)amino)methyl)-4H-pyrido[1,2-a]pyrimidin-4-one;
   8-(3-chloro-4-(2-chloro-3-(6-methoxy-5-(((((S)-5-oxopyrrolidin-2-yl)methyl)amino)methyl)pyridin-2-yl)phenyl)pyridin-2-yl)-3-(((((R)-5-oxopyrrolidin-2-yl)methyl)amino)methyl)-4H-pyrido[1,2-a]pyrimidin-4-one;
   8-(3-chloro-4-(2-chloro-3-(6-methoxy-5-(((((R)-5-oxopyrrolidin-2-yl)methyl)amino)methyl)pyridin-2-yl)phenyl)pyridin-2-yl)-3-(((((S)-5-oxopyrrolidin-2-yl)methyl)amino)methyl)-4H-pyrido[1,2-a]pyrimidin-4-one;
   8-(3-chloro-4-(2-chloro-3-(6-methoxy-5-(((((R)-5-oxopyrrolidin-2-yl)methyl)amino)methyl)pyridin-2-yl)phenyl)pyridin-2-yl)-3-(((((R)-5-oxopyrrolidin-2-yl)methyl)amino)methyl)-4H-pyrido[1,2-a]pyrimidin-4-one;
   (S)-5-((((6-(2-chloro-3-(3-chloro-2-(1-methyl-2,3,4,5-tetrahydro-1H-benzo[e][1,4]diazepin-8-yl)pyridin-4-yl)phenyl)-2-methoxypyridin-3-yl)methyl)amino)methyl)pyrrolidin-2-one;
   (R)-5-((((6-(2-chloro-3-(3-chloro-2-(1-methyl-2,3,4,5-tetrahydro-1H-benzo[e][1,4]diazepin-8-yl)pyridin-4-yl)phenyl)-2-methoxypyridin-3-yl)methyl)amino)methyl)pyrrolidin-2-one;
   (5S,5'S)-5,5'-(((((3,3'-dichloro-[4,4'-bipyridine]-2,2'-diyl)bis(2-methoxy-4,1-phenylene))bis(methylene))bis(azanediyl))bis(methylene))bis(pyrrolidin-2-one);
   (5S,5'R)-5,5'-(((((3,3'-dichloro-[4,4'-bipyridine]-2,2'-diyl)bis(2-methoxy-4,1-phenylene))bis(methylene))bis(azanediyl))bis(methylene))bis(pyrrolidin-2-one);
   (5R,5'S)-5,5'-(((((3,3'-dichloro-[4,4'-bipyridine]-2,2'-diyl)bis(2-methoxy-4,1-phenylene))bis(methylene))bis(azanediyl))bis(methylene))bis(pyrrolidin-2-one);
   (5R,5'R)-5,5'-(((((3,3'-dichloro-[4,4'-bipyridine]-2,2'-diyl)bis(2-methoxy-4,1-phenylene))bis(methylene))bis(azanediyl))bis(methylene))bis(pyrrolidin-2-one);
   N-(1-(((6-(2-chloro-3-(3-chloro-2-(1-methyl-2,3,4,5-tetrahydro-1H-benzo[e][1,4]diazepin-8-yl)pyridin-4-yl)phenyl)-2-methoxypyridin-3-yl)methyl)amino)propan-2-yl)acetamide;
   (S)-N-(1-(((6-(2-chloro-3-(3-chloro-2-(1-methyl-2,3,4,5-tetrahydro-1H-benzo[e][1,4]diazepin-8-yl)pyridin-4-yl)phenyl)-2-methoxypyridin-3-yl)methyl)amino)propan-2-yl)acetamide;
   (R)-N-(1-(((6-(2-chloro-3-(3-chloro-2-(1-methyl-2,3,4,5-tetrahydro-1H-benzo[e][1,4]diazepin-8-yl)pyridin-4-yl)phenyl)-2-methoxypyridin-3-yl)methyl)amino)propan-2-yl)acetamide;
   (S)-5-((((6-(2-chloro-3-(3-chloro-2-(8-methoxy-2-(((S)-5-oxopyrrolidin-2-yl)methyl)-1,2,3,4-tetrahydroisoquinolin-6-yl)pyridin-4-yl)phenyl)-2-methoxypyridin-3-yl)methyl)amino)methyl)pyrrolidin-2-one;
   (S)-5-((((6-(2-chloro-3-(3-chloro-2-(8-methoxy-2-(((R)-5-oxopyrrolidin-2-yl)methyl)-1,2,3,4-tetrahydroisoquinolin-6-yl)pyridin-4-yl)phenyl)-2-methoxypyridin-3-yl)methyl)amino)methyl)pyrrolidin-2-one;
   (R)-5-((((6-(2-chloro-3-(3-chloro-2-(8-methoxy-2-(((S)-5-oxopyrrolidin-2-yl)methyl)-1,2,3,4-tetrahydroisoquinolin-6-yl)pyridin-4-yl)phenyl)-2-methoxypyridin-3-yl)methyl)amino)methyl)pyrrolidin-2-one;
   (R)-5-((((6-(2-chloro-3-(3-chloro-2-(8-methoxy-2-(((R)-5-oxopyrrolidin-2-yl)methyl)-1,2,3,4-tetrahydroisoquinolin-6-yl)pyridin-4-yl)phenyl)-2-methoxypyridin-3-yl)methyl)amino)methyl)pyrrolidin-2-one;
   (S)-5-((((6-(2-chloro-3-(3-chloro-2-(8-methoxy-1,2,3,4-tetrahydroisoquinolin-6-yl)pyridin-4-yl)phenyl)-2-methoxypyridin-3-yl)methyl)amino)methyl)pyrrolidin-2-one;
   (R)-5-((((6-(2-chloro-3-(3-chloro-2-(8-methoxy-1,2,3,4-tetrahydroisoquinolin-6-yl)pyridin-4-yl)phenyl)-2-methoxypyridin-3-yl)methyl)amino)methyl)pyrrolidin-2-one;
   (S)-5-((((6-(2-chloro-3-(3-chloro-2-(3-methoxy-4-(((((S)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
   (S)-5-((((6-(2-chloro-3-(3-chloro-2-(3-methoxy-4-(((((R)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
   (R)-5-((((6-(2-chloro-3-(3-chloro-2-(3-methoxy-4-(((((S)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
   (R)-5-((((6-(2-chloro-3-(3-chloro-2-(3-methoxy-4-(((((R)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
   (S)-5-((((6-(2-chloro-3-(3-chloro-2-(1-methyl-2,3,4,5-tetrahydro-1H-benzo[e] [1,4]diazepin-8-yl)pyridin-4-yl)phenyl)-1-methyl-1H-pyrrolo[2,3-b]pyridin-3-yl)methyl)amino)methyl)pyrrolidin-2-one;
   (R)-5-((((6-(2-chloro-3-(3-chloro-2-(1-methyl-2,3,4,5-tetrahydro-1H-benzo[e] [1,4]diazepin-8-yl)pyridin-4-yl)phenyl)-1-methyl-1H-pyrrolo[2,3-b]pyridin-3-yl)methyl)amino)methyl)pyrrolidin-2-one;
   (S)-5-((((6-(2-chloro-3-(3-chloro-2-(3-fluoro-5-methoxy-4-(((((S)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
   (S)-5-((((6-(2-chloro-3-(3-chloro-2-(3-fluoro-5-methoxy-4-(((((R)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
   (R)-5-((((6-(2-chloro-3-(3-chloro-2-(3-fluoro-5-methoxy-4-(((((S)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
   (R)-5-((((6-(2-chloro-3-(3-chloro-2-(3-fluoro-5-methoxy-4-(((((R)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
   (S)-5-((((6-(2-chloro-3-(3-chloro-2-(1-methyl-3-(((((S)-5-oxopyrrolidin-2-yl)methyl)amino)methyl)-1H-indol-6-yl)pyridin-4-yl)phenyl)-2-methoxypyridin-3-yl)methyl)amino)methyl)pyrrolidin-2-one;
   (S)-5-((((6-(2-chloro-3-(3-chloro-2-(1-methyl-3-(((((R)-5-oxopyrrolidin-2-yl)methyl)amino)methyl)-1H-indol-6-yl)pyridin-4-yl)phenyl)-2-methoxypyridin-3-yl)methyl)amino)methyl)pyrrolidin-2-one;
   (R)-5-((((6-(2-chloro-3-(3-chloro-2-(1-methyl-3-(((((S)-5-oxopyrrolidin-2-yl)methyl)amino)methyl)-1H-indol-6-yl)pyridin-4-yl)phenyl)-2-methoxypyridin-3-yl)methyl)amino)methyl)pyrrolidin-2-one;
   (R)-5-((((6-(2-chloro-3-(3-chloro-2-(1-methyl-3-(((((R)-5-oxopyrrolidin-2-yl)methyl)amino)methyl)-1H-indol-6-yl)pyridin-4-yl)phenyl)-2-methoxypyridin-3-yl)methyl)amino)methyl)pyrrolidin-2-one;
   (5 S,5'S)-5,5'-(((((3,3'-dichloro-[4,4'-bipyridine]-2,2'-diyl)bis(2-fluoro-6-methoxy-4,1-phenylene))bis(methylene))bis(azanediyl))bis(methylene))bis(pyrrolidin-2-one);
   (5S,5'R)-5,5'-(((((3,3'-dichloro-[4,4'-bipyridine]-2,2'-diyl)bis(2-fluoro-6-methoxy-4,1-phenylene))bis(methylene))bis(azanediyl))bis(methylene))bis(pyrrolidin-2-one);
   (5R,5'S)-5,5'-(((((3,3'-dichloro-[4,4'-bipyridine]-2,2'-diyl)bis(2-fluoro-6-methoxy-4,1-phenylene))bis(methylene))bis(azanediyl))bis(methylene))bis(pyrrolidin-2-one);
   (5R,5'R)-5,5'-(((((3,3'-dichloro-[4,4'-bipyridine]-2,2'-diyl)bis(2-fluoro-6-methoxy-4,1-phenylene))bis(methylene))bis(azanediyl))bis(methylene))bis(pyrrolidin-2-one);
   (S)-5-((((6-(2-chloro-3-(3-chloro-2-(1-methyl-3-(((((S)-5-oxopyrrolidin-2-yl)methyl)amino)methyl)-1H-pyrrolo[3,2-b]pyridin-6-yl)pyridin-4-yl)phenyl)-2-methoxypyridin-3-yl)methyl)amino)methyl)pyrrolidin-2-one;
   (S)-5-((((6-(2-chloro-3-(3-chloro-2-(1-methyl-3-(((((R)-5-oxopyrrolidin-2-yl)methyl)amino)methyl)-1H-pyrrolo[3,2-b]pyridin-6-yl)pyridin-4-yl)phenyl)-2-methoxypyridin-3-yl)methyl)amino)methyl)pyrrolidin-2-one;
   (R)-5-((((6-(2-chloro-3-(3-chloro-2-(1-methyl-3-(((((S)-5-oxopyrrolidin-2-yl)methyl)amino)methyl)-1H-pyrrolo[3,2-b]pyridin-6-yl)pyridin-4-yl)phenyl)-2-methoxypyridin-3-yl)methyl)amino)methyl)pyrrolidin-2-one;
   (R)-5-((((6-(2-chloro-3-(3-chloro-2-(1-methyl-3-(((((R)-5-oxopyrrolidin-2-yl)methyl)amino)methyl)-1H-pyrrolo[3,2-b]pyridin-6-yl)pyridin-4-yl)phenyl)-2-methoxypyridin-3-yl)methyl)amino)methyl)pyrrolidin-2-one;
   (S)-5-(((4-(3,3'-dichloro-2'-(1-methyl-3-(((((S)-5-oxopyrrolidin-2-yl)methyl)amino)methyl)-1H-indol-6-yl)-[4,4'-bipyridin]-2-yl)-2-methoxybenzyl)amino)methyl)pyrrolidin-2-one;
   (S)-5-(((4-(3,3'-dichloro-2'-(1-methyl-3-(((((R)-5-oxopyrrolidin-2-yl)methyl)amino)methyl)-1H-indol-6-yl)-[4,4'-bipyridin]-2-yl)-2-methoxybenzyl)amino)methyl)pyrrolidin-2-one;
   (R)-5-(((4-(3,3'-dichloro-2'-(1-methyl-3-(((((S)-5-oxopyrrolidin-2-yl)methyl)amino)methyl)-1H-indol-6-yl)-[4,4'-bipyridin]-2-yl)-2-methoxybenzyl)amino)methyl)pyrrolidin-2-one;
   (R)-5-(((4-(3,3'-dichloro-2'-(1-methyl-3-(((((R)-5-oxopyrrolidin-2-yl)methyl)amino)methyl)-1H-indol-6-yl)-[4,4'-bipyridin]-2-yl)-2-methoxybenzyl)amino)methyl)pyrrolidin-2-one;
   (5S,5'S)-5,5'-(((((3,3'-dichloro-[4,4'-bipyridine]-2,2'-diyl)bis(1-methyl-1H-indole-6,3-diyl))bis(methylene))bis(azanediyl))bis(methylene))bis(pyrrolidin-2-one);
   (5S,5'R)-5,5'-(((((3,3'-dichloro-[4,4'-bipyridine]-2,2'-diyl)bis(1-methyl-1H-indole-6,3-diyl))bis(methylene))bis(azanediyl))bis(methylene))bis(pyrrolidin-2-one);
   (5R,5'S)-5,5'-(((((3,3'-dichloro-[4,4'-bipyridine]-2,2'-diyl)bis(1-methyl-1H-indole-6,3-diyl))bis(methylene))bis(azanediyl))bis(methylene))bis(pyrrolidin-2-one);
   (5R,5'R)-5,5'-(((((3,3'-dichloro-[4,4'-bipyridine]-2,2'-diyl)bis(1-methyl-1H-indole-6,3-diyl))bis(methylene))bis(azanediyl))bis(methylene))bis(pyrrolidin-2-one);
   (S)-5-(((4-(3,3'-dichloro-2'-(1-methyl-3-(((((S)-5-oxopyrrolidin-2-yl)methyl)amino)methyl)-1H-pyrrolo[3,2-b]pyridin-6-yl)-[4,4'-bipyridin]-2-yl)-2-methoxybenzyl)amino)methyl)pyrrolidin-2-one;
   (S)-5-(((4-(3,3'-dichloro-2'-(1-methyl-3-(((((R)-5-oxopyrrolidin-2-yl)methyl)amino)methyl)-1H-pyrrolo[3,2-b]pyridin-6-yl)-[4,4'-bipyridin]-2-yl)-2-methoxybenzyl)amino)methyl)pyrrolidin-2-one;
   (R)-5-(((4-(3,3'-dichloro-2'-(1-methyl-3-(((((S)-5-oxopyrrolidin-2-yl)methyl)amino)methyl)-1H-pyrrolo[3,2-b]pyridin-6-yl)-[4,4'-bipyridin]-2-yl)-2-methoxybenzyl)amino)methyl)pyrrolidin-2-one;
   (R)-5-(((4-(3,3'-dichloro-2'-(1-methyl-3-(((((R)-5-oxopyrrolidin-2-yl)methyl)amino)methyl)-1H-pyrrolo[3,2-b]pyridin-6-yl)-[4,4'-bipyridin]-2-yl)-2-methoxybenzyl)amino)methyl)pyrrolidin-2-one;
   (S)-5-((((6-(2-chloro-3-(3-chloro-2-(3-methoxy-4-(((((S)-5-oxopyrrolidin-2-yl)methyl)amino)methyl)phenyl)pyridin-4-yl)phenyl)-1-methyl-1H-pyrrolo[2,3-b]pyridin-3-yl)methyl)amino)methyl)pyrrolidin-2-one;
   (S)-5-((((6-(2-chloro-3-(3-chloro-2-(3-methoxy-4-(((((R)-5-oxopyrrolidin-2-yl)methyl)amino)methyl)phenyl)pyridin-4-yl)phenyl)-1-methyl-1H-pyrrolo[2,3-b]pyridin-3-yl)methyl)amino)methyl)pyrrolidin-2-one;
   (R)-5-((((6-(2-chloro-3-(3-chloro-2-(3-methoxy-4-(((((S)-5-oxopyrrolidin-2-yl)methyl)amino)methyl)phenyl)pyridin-4-yl)phenyl)-1-methyl-1H-pyrrolo[2,3-b]pyridin-3-yl)methyl)amino)methyl)pyrrolidin-2-one;
   (R)-5-((((6-(2-chloro-3-(3-chloro-2-(3-methoxy-4-(((((R)-5-oxopyrrolidin-2-yl)methyl)amino)methyl)phenyl)pyridin-4-yl)phenyl)-1-methyl-1H-pyrrolo[2,3-b]pyridin-3-yl)methyl)amino)methyl)pyrrolidin-2-one;
   (S)-5-((6-(2-chloro-3-(3-chloro-2-(3-methoxy-4-(((((S)-5-oxopyrrolidin-2-yl)methyl)amino)methyl)phenyl)pyridin-4-yl)phenyl)-8-methoxy-3,4-dihydroisoquinolin-2(1H)-yl)methyl)pyrrolidin-2-one;
   (S)-5-((6-(2-chloro-3-(3-chloro-2-(3-methoxy-4-(((((R)-5-oxopyrrolidin-2-yl)methyl)amino)methyl)phenyl)pyridin-4-yl)phenyl)-8-methoxy-3,4-dihydroisoquinolin-2(1H)-yl)methyl)pyrrolidin-2-one;
   (R)-5-((6-(2-chloro-3-(3-chloro-2-(3-methoxy-4-(((((S)-5-oxopyrrolidin-2-yl)methyl)amino)methyl)phenyl)pyridin-4-yl)phenyl)-8-methoxy-3,4-dihydroisoquinolin-2(1H)-yl)methyl)pyrrolidin-2-one;
   (R)-5-((6-(2-chloro-3-(3-chloro-2-(3-methoxy-4-(((((R)-5-oxopyrrolidin-2-yl)methyl)amino)methyl)phenyl)pyridin-4-yl)phenyl)-8-methoxy-3,4-dihydroisoquinolin-2(1H)-yl)methyl)pyrrolidin-2-one;
   (S)-5-((((6-(2-chloro-3-(3-chloro-2-(1-methyl-4-(((S)-5-oxopyrrolidin-2-yl)methyl)-2,3,4,5-tetrahydro-1H-benzo[e][1,4]diazepin-8-yl)pyridin-4-yl)phenyl)-2-methoxypyridin-3-yl)methyl)amino)methyl)pyrrolidin-2-one;
   (S)-5-((((6-(2-chloro-3-(3-chloro-2-(1-methyl-4-(((R)-5-oxopyrrolidin-2-yl)methyl)-2,3,4,5-tetrahydro-1H-benzo[e][1,4]diazepin-8-yl)pyridin-4-yl)phenyl)-2-methoxypyridin-3-yl)methyl)amino)methyl)pyrrolidin-2-one;
   (R)-5-((((6-(2-chloro-3-(3-chloro-2-(1-methyl-4-(((S)-5-oxopyrrolidin-2-yl)methyl)-2,3,4,5-tetrahydro-1H-benzo[e][1,4]diazepin-8-yl)pyridin-4-yl)phenyl)-2-methoxypyridin-3-yl)methyl)amino)methyl)pyrrolidin-2-one;
   (R)-5-((((6-(2-chloro-3-(3-chloro-2-(1-methyl-4-(((R)-5-oxopyrrolidin-2-yl)methyl)-2,3,4,5-tetrahydro-1H-benzo[e][1,4]diazepin-8-yl)pyridin-4-yl)phenyl)-2-methoxypyridin-3-yl)methyl)amino)methyl)pyrrolidin-2-one;
   (S)-5-((((5-(2-chloro-3-(3-chloro-2-(3-methoxy-4-(((((S)-5-oxopyrrolidin-2-yl)methyl)amino)methyl)phenyl)pyridin-4-yl)phenyl)-3-methoxypyrazin-2-yl)methyl)amino)methyl)pyrrolidin-2-one;
   (S)-5-((((5-(2-chloro-3-(3-chloro-2-(3-methoxy-4-(((((R)-5-oxopyrrolidin-2-yl)methyl)amino)methyl)phenyl)pyridin-4-yl)phenyl)-3-methoxypyrazin-2-yl)methyl)amino)methyl)pyrrolidin-2-one;
   (R)-5-((((5-(2-chloro-3-(3-chloro-2-(3-methoxy-4-(((((S)-5-oxopyrrolidin-2-yl)methyl)amino)methyl)phenyl)pyridin-4-yl)phenyl)-3-methoxypyrazin-2-yl)methyl)amino)methyl)pyrrolidin-2-one;
   (R)-5-((((5-(2-chloro-3-(3-chloro-2-(3-methoxy-4-(((((R)-5-oxopyrrolidin-2-yl)methyl)amino)methyl)phenyl)pyridin-4-yl)phenyl)-3-methoxypyrazin-2-yl)methyl)amino)methyl)pyrrolidin-2-one;
   (S)-5-((((6-(3-(2-(3-(((1-acetylpiperidin-4-yl)amino)methyl)-1-methyl-1H-pyrrolo[3,2-b]pyridin-6-yl)-3-chloropyridin-4-yl)-2-chlorophenyl)-2-methoxypyridin-3-yl)methyl)amino)methyl)pyrrolidin-2-one;
   (R)-5-((((6-(3-(2-(3-(((1-acetylpiperidin-4-yl)amino)methyl)-1-methyl-1H-pyrrolo[3,2-b]pyridin-6-yl)-3-chloropyridin-4-yl)-2-chlorophenyl)-2-methoxypyridin-3-yl)methyl)amino)methyl)pyrrolidin-2-one;
   (S)-5-((((6-(2-chloro-3-(3-chloro-2-(1-methyl-3-(((((S)-oxetan-2-yl)methyl)amino)methyl)-1H-pyrrolo[3,2-b]pyridin-6-yl)pyridin-4-yl)phenyl)-2-methoxypyridin-3-yl)methyl)amino)methyl)pyrrolidin-2-one;
   (S)-5-((((6-(2-chloro-3-(3-chloro-2-(1-methyl-3-(((((R)-oxetan-2-yl)methyl)amino)methyl)-1H-pyrrolo[3,2-b]pyridin-6-yl)pyridin-4-yl)phenyl)-2-methoxypyridin-3-yl)methyl)amino)methyl)pyrrolidin-2-one;
   (R)-5-((((6-(2-chloro-3-(3-chloro-2-(1-methyl-3-(((((S)-oxetan-2-yl)methyl)amino)methyl)-1H-pyrrolo[3,2-b]pyridin-6-yl)pyridin-4-yl)phenyl)-2-methoxypyridin-3-yl)methyl)amino)methyl)pyrrolidin-2-one;
   (R)-5-((((6-(2-chloro-3-(3-chloro-2-(1-methyl-3-(((((R)-oxetan-2-yl)methyl)amino)methyl)-1H-pyrrolo[3,2-b]pyridin-6-yl)pyridin-4-yl)phenyl)-2-methoxypyridin-3-yl)methyl)amino)methyl)pyrrolidin-2-one;
   (S)-5-((((6-(2-chloro-3-(3-chloro-5'-methoxy-6'-(((((S)-5-oxopyrrolidin-2-yl)methyl)amino)methyl)-[2,3'-bipyridin]-4-yl)phenyl)-2-methoxypyridin-3-yl)methyl)amino)methyl)pyrrolidin-2-one;
   (S)-5-((((6-(2-chloro-3-(3-chloro-5'-methoxy-6'-(((((R)-5-oxopyrrolidin-2-yl)methyl)amino)methyl)-[2,3'-bipyridin]-4-yl)phenyl)-2-methoxypyridin-3-yl)methyl)amino)methyl)pyrrolidin-2-one;
   (R)-5-((((6-(2-chloro-3-(3-chloro-5'-methoxy-6'-(((((S)-5-oxopyrrolidin-2-yl)methyl)amino)methyl)-[2,3'-bipyridin]-4-yl)phenyl)-2-methoxypyridin-3-yl)methyl)amino)methyl)pyrrolidin-2-one;
   (R)-5-((((6-(2-chloro-3-(3-chloro-5'-methoxy-6'-(((((R)-5-oxopyrrolidin-2-yl)methyl)amino)methyl)-[2,3'-bipyridin]-4-yl)phenyl)-2-methoxypyridin-3-yl)methyl)amino)methyl)pyrrolidin-2-one;
   (S)-5-((((6-(2-chloro-3-(3-chloro-2-(4-((S)-2-hydroxypropyl)-1-methyl-2,3,4,5-tetrahydro-1H-benzo[e][1,4]diazepin-8-yl)pyridin-4-yl)phenyl)-2-methoxypyridin-3-yl)methyl)amino)methyl)pyrrolidin-2-one;
   (S)-5-((((6-(2-chloro-3-(3-chloro-2-(4-((R)-2-hydroxypropyl)-1-methyl-2,3,4,5-tetrahydro-1H-benzo[e][1,4]diazepin-8-yl)pyridin-4-yl)phenyl)-2-methoxypyridin-3-yl)methyl)amino)methyl)pyrrolidin-2-one;
   (R)-5-((((6-(2-chloro-3-(3-chloro-2-(4-((S)-2-hydroxypropyl)-1-methyl-2,3,4,5-tetrahydro-1H-benzo[e][1,4]diazepin-8-yl)pyridin-4-yl)phenyl)-2-methoxypyridin-3-yl)methyl)amino)methyl)pyrrolidin-2-one;
   (R)-5-((((6-(2-chloro-3-(3-chloro-2-(4-((R)-2-hydroxypropyl)-1-methyl-2,3,4,5-tetrahydro-1H-benzo[e][1,4]diazepin-8-yl)pyridin-4-yl)phenyl)-2-methoxypyridin-3-yl)methyl)amino)methyl)pyrrolidin-2-one;
   (S)-5-((((6-(2-chloro-3-(3-chloro-2-(4-((S)-2-hydroxypropyl)-2,3,4,5-tetrahydrobenzo[f][1,4]oxazepin-8-yl)pyridin-4-yl)phenyl)-2-methoxypyridin-3-yl)methyl)amino)methyl)pyrrolidin-2-one;
   (S)-5-((((6-(2-chloro-3-(3-chloro-2-(4-((R)-2-hydroxypropyl)-2,3,4,5-tetrahydrobenzo[f][1,4]oxazepin-8-yl)pyridin-4-yl)phenyl)-2-methoxypyridin-3-yl)methyl)amino)methyl)pyrrolidin-2-one;
   (R)-5-((((6-(2-chloro-3-(3-chloro-2-(4-((S)-2-hydroxypropyl)-2,3,4,5-tetrahydrobenzo[f][1,4]oxazepin-8-yl)pyridin-4-yl)phenyl)-2-methoxypyridin-3-yl)methyl)amino)methyl)pyrrolidin-2-one;
   (R)-5-((((6-(2-chloro-3-(3-chloro-2-(4-((R)-2-hydroxypropyl)-2,3,4,5-tetrahydrobenzo[f][1,4]oxazepin-8-yl)pyridin-4-yl)phenyl)-2-methoxypyridin-3-yl)methyl)amino)methyl)pyrrolidin-2-one;
   (S)-5-((((6-(2-chloro-3-(3-chloro-2-(2,3,4,5-tetrahydrobenzo[f][1,4]oxazepin-8-yl)pyridin-4-yl)phenyl)-2-methoxypyridin-3-yl)methyl)amino)methyl)pyrrolidin-2-one;
   (R)-5-((((6-(2-chloro-3-(3-chloro-2-(2,3,4,5-tetrahydrobenzo[f][1,4]oxazepin-8-yl)pyridin-4-yl)phenyl)-2-methoxypyridin-3-yl)methyl)amino)methyl)pyrrolidin-2-one;
   (S)-5-((((6-(2-chloro-3-(3-chloro-2-(2-((R)-2-hydroxypropyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
   (S)-5-((((6-(2-chloro-3-(3-chloro-2-(2-((S)-2-hydroxypropyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
   (R)-5-((((6-(2-chloro-3-(3-chloro-2-(2-((R)-2-hydroxypropyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
   (R)-5-((((6-(2-chloro-3-(3-chloro-2-(2-((S)-2-hydroxypropyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
   (S)-5-((((6-(3-(2-(3-(((1-acetylpiperidin-4-yl)amino)methyl)-1-methyl-1H-indol-6-yl)-3-chloropyridin-4-yl)-2-chlorophenyl)-2-methoxypyridin-3-yl)methyl)amino)methyl)pyrrolidin-2-one;
   (R)-5-((((6-(3-(2-(3-(((1-acetylpiperidin-4-yl)amino)methyl)-1-methyl-1H-indol-6-yl)-3-chloropyridin-4-yl)-2-chlorophenyl)-2-methoxypyridin-3-yl)methyl)amino)methyl)pyrrolidin-2-one;
   (S)-5-((((6-(2-chloro-3-(3-chloro-2-(3-(((2-hydroxyethyl)amino)methyl)-1-methyl-1H-indol-6-yl)pyridin-4-yl)phenyl)-2-methoxypyridin-3-yl)methyl)amino)methyl)pyrrolidin-2-one;
   (R)-5-((((6-(2-chloro-3-(3-chloro-2-(3-(((2-hydroxyethyl)amino)methyl)-1-methyl-1H-indol-6-yl)pyridin-4-yl)phenyl)-2-methoxypyridin-3-yl)methyl)amino)methyl)pyrrolidin-2-one;
   (S)-5-((((6-(2-chloro-3-(3-chloro-2-(3-(((2-methoxyethyl)amino)methyl)-1-methyl-1H-indol-6-yl)pyridin-4-yl)phenyl)-2-methoxypyridin-3-yl)methyl)amino)methyl)pyrrolidin-2-one;
   (R)-5-((((6-(2-chloro-3-(3-chloro-2-(3-(((2-methoxyethyl)amino)methyl)-1-methyl-1H-indol-6-yl)pyridin-4-yl)phenyl)-2-methoxypyridin-3-yl)methyl)amino)methyl)pyrrolidin-2-one;
   (S)-5-((((6-(2-chloro-3-(3-chloro-2-(1-methyl-3-((methylamino)methyl)-1H-indol-6-yl)pyridin-4-yl)phenyl)-2-methoxypyridin-3-yl)methyl)amino)methyl)pyrrolidin-2-one;
   (R)-5-((((6-(2-chloro-3-(3-chloro-2-(1-methyl-3-((methylamino)methyl)-1H-indol-6-yl)pyridin-4-yl)phenyl)-2-methoxypyridin-3-yl)methyl)amino)methyl)pyrrolidin-2-one;
   (S)-5-((((6-(2-chloro-3-(3-chloro-2-(3-((ethylamino)methyl)-1-methyl-1H-indol-6-yl)pyridin-4-yl)phenyl)-2-methoxypyridin-3-yl)methyl)amino)methyl)pyrrolidin-2-one;
   (R)-5-((((6-(2-chloro-3-(3-chloro-2-(3-((ethylamino)methyl)-1-methyl-1H-indol-6-yl)pyridin-4-yl)phenyl)-2-methoxypyridin-3-yl)methyl)amino)methyl)pyrrolidin-2-one;
   (S)-5-((((6-(2-chloro-3-(3-chloro-2-(3-((dimethylamino)methyl)-1-methyl-1H-indol-6-yl)pyridin-4-yl)phenyl)-2-methoxypyridin-3-yl)methyl)amino)methyl)pyrrolidin-2-one;
   (R)-5-((((6-(2-chloro-3-(3-chloro-2-(3-((dimethylamino)methyl)-1-methyl-1H-indol-6-yl)pyridin-4-yl)phenyl)-2-methoxypyridin-3-yl)methyl)amino)methyl)pyrrolidin-2-one;
   (S)-5-((((6-(2-chloro-3-(3-chloro-2-(1-methyl-3-(((((S)-oxetan-2-yl)methyl)amino)methyl)-1H-indol-6-yl)pyridin-4-yl)phenyl)-2-methoxypyridin-3-yl)methyl)amino)methyl)pyrrolidin-2-one;
   (S)-5-((((6-(2-chloro-3-(3-chloro-2-(1-methyl-3-(((((R)-oxetan-2-yl)methyl)amino)methyl)-1H-indol-6-yl)pyridin-4-yl)phenyl)-2-methoxypyridin-3-yl)methyl)amino)methyl)pyrrolidin-2-one;
   (R)-5-((((6-(2-chloro-3-(3-chloro-2-(1-methyl-3-(((((S)-oxetan-2-yl)methyl)amino)methyl)-1H-indol-6-yl)pyridin-4-yl)phenyl)-2-methoxypyridin-3-yl)methyl)amino)methyl)pyrrolidin-2-one;
   (R)-5-((((6-(2-chloro-3-(3-chloro-2-(1-methyl-3-(((((R)-oxetan-2-yl)methyl)amino)methyl)-1H-indol-6-yl)pyridin-4-yl)phenyl)-2-methoxypyridin-3-yl)methyl)amino)methyl)pyrrolidin-2-one;
   (S)-5-((((6-(2-chloro-3-(3-chloro-2-(3-((((S)-2-hydroxypropyl)amino)methyl)-1-methyl-1H-indol-6-yl)pyridin-4-yl)phenyl)-2-methoxypyridin-3-yl)methyl)amino)methyl)pyrrolidin-2-one;
   (S)-5-((((6-(2-chloro-3-(3-chloro-2-(3-((((R)-2-hydroxypropyl)amino)methyl)-1-methyl-1H-indol-6-yl)pyridin-4-yl)phenyl)-2-methoxypyridin-3-yl)methyl)amino)methyl)pyrrolidin-2-one;
   (R)-5-((((6-(2-chloro-3-(3-chloro-2-(3-((((S)-2-hydroxypropyl)amino)methyl)-1-methyl-1H-indol-6-yl)pyridin-4-yl)phenyl)-2-methoxypyridin-3-yl)methyl)amino)methyl)pyrrolidin-2-one;
   (R)-5-((((6-(2-chloro-3-(3-chloro-2-(3-((((R)-2-hydroxypropyl)amino)methyl)-1-methyl-1H-indol-6-yl)pyridin-4-yl)phenyl)-2-methoxypyridin-3-yl)methyl)amino)methyl)pyrrolidin-2-one;
   (S)-5-((((6-(2-chloro-3-(3-chloro-2-(3-(((2-hydroxy-2-methylpropyl)amino)methyl)-1-methyl-1H-indol-6-yl)pyridin-4-yl)phenyl)-2-methoxypyridin-3-yl)methyl)amino)methyl)pyrrolidin-2-one;
   (R)-5-((((6-(2-chloro-3-(3-chloro-2-(3-(((2-hydroxy-2-methylpropyl)amino)methyl)-1-methyl-1H-indol-6-yl)pyridin-4-yl)phenyl)-2-methoxypyridin-3-yl)methyl)amino)methyl)pyrrolidin-2-one;
   (S)-5-((6-(3-chloro-4-(2-chloro-3-(4-chloro-6-methoxy-5-(((((S)-5-oxopyrrolidin-2-yl)methyl)amino)methyl)pyridin-2-yl)phenyl)pyridin-2-yl)-8-methoxy-3,4-dihydroisoquinolin-2(1H)-yl)methyl)pyrrolidin-2-one;
   (S)-5-((6-(3-chloro-4-(2-chloro-3-(4-chloro-6-methoxy-5-(((((R)-5-oxopyrrolidin-2-yl)methyl)amino)methyl)pyridin-2-yl)phenyl)pyridin-2-yl)-8-methoxy-3,4-dihydroisoquinolin-2(1H)-yl)methyl)pyrrolidin-2-one;
   (R)-5-((6-(3-chloro-4-(2-chloro-3-(4-chloro-6-methoxy-5-(((((S)-5-oxopyrrolidin-2-yl)methyl)amino)methyl)pyridin-2-yl)phenyl)pyridin-2-yl)-8-methoxy-3,4-dihydroisoquinolin-2(1H)-yl)methyl)pyrrolidin-2-one;
   (R)-5-((6-(3-chloro-4-(2-chloro-3-(4-chloro-6-methoxy-5-(((((R)-5-oxopyrrolidin-2-yl)methyl)amino)methyl)pyridin-2-yl)phenyl)pyridin-2-yl)-8-methoxy-3,4-dihydroisoquinolin-2(1H)-yl)methyl)pyrrolidin-2-one;
   (S)-5-((((6-(2-chloro-3-(3-chloro-2-(3-((isobutylamino)methyl)-1-methyl-1H-indol-6-yl)pyridin-4-yl)phenyl)-2-methoxypyridin-3-yl)methyl)amino)methyl)pyrrolidin-2-one;
   (R)-5-((((6-(2-chloro-3-(3-chloro-2-(3-((isobutylamino)methyl)-1-methyl-1H-indol-6-yl)pyridin-4-yl)phenyl)-2-methoxypyridin-3-yl)methyl)amino)methyl)pyrrolidin-2-one;
   (S)-5-((((6-(2-chloro-3-(3-chloro-2-(3-(((2-hydroxyethyl)(methyl)amino)methyl)-1-methyl-1H-indol-6-yl)pyridin-4-yl)phenyl)-2-methoxypyridin-3-yl)methyl)amino)methyl)pyrrolidin-2-one;
   (R)-5-((((6-(2-chloro-3-(3-chloro-2-(3-(((2-hydroxyethyl)(methyl)amino)methyl)-1-methyl-1H-indol-6-yl)pyridin-4-yl)phenyl)-2-methoxypyridin-3-yl)methyl)amino)methyl)pyrrolidin-2-one;
   (S)-5-((((6-(2-chloro-3-(3-chloro-2-(3-((((S)-2-methoxypropyl)amino)methyl)-1-methyl-1H-indol-6-yl)pyridin-4-yl)phenyl)-2-methoxypyridin-3-yl)methyl)amino)methyl)pyrrolidin-2-one;
   (S)-5-((((6-(2-chloro-3-(3-chloro-2-(3-((((R)-2-methoxypropyl)amino)methyl)-1-methyl-1H-indol-6-yl)pyridin-4-yl)phenyl)-2-methoxypyridin-3-yl)methyl)amino)methyl)pyrrolidin-2-one;
   (R)-5-((((6-(2-chloro-3-(3-chloro-2-(3-((((S)-2-methoxypropyl)amino)methyl)-1-methyl-1H-indol-6-yl)pyridin-4-yl)phenyl)-2-methoxypyridin-3-yl)methyl)amino)methyl)pyrrolidin-2-one;
   (R)-5-((((6-(2-chloro-3-(3-chloro-2-(3-((((R)-2-methoxypropyl)amino)methyl)-1-methyl-1H-indol-6-yl)pyridin-4-yl)phenyl)-2-methoxypyridin-3-yl)methyl)amino)methyl)pyrrolidin-2-one;
   (S)-5-((((6-(2-chloro-3-(3-chloro-2-(1-methyl-3-(((((S)-tetrahydrofuran-2-yl)methyl)amino)methyl)-1H-indol-6-yl)pyridin-4-yl)phenyl)-2-methoxypyridin-3-yl)methyl)amino)methyl)pyrrolidin-2-one;
   (S)-5-((((6-(2-chloro-3-(3-chloro-2-(1-methyl-3-(((((R)-tetrahydrofuran-2-yl)methyl)amino)methyl)-1H-indol-6-yl)pyridin-4-yl)phenyl)-2-methoxypyridin-3-yl)methyl)amino)methyl)pyrrolidin-2-one;
   (R)-5-((((6-(2-chloro-3-(3-chloro-2-(1-methyl-3-(((((S)-tetrahydrofuran-2-yl)methyl)amino)methyl)-1H-indol-6-yl)pyridin-4-yl)phenyl)-2-methoxypyridin-3-yl)methyl)amino)methyl)pyrrolidin-2-one;
   (R)-5-((((6-(2-chloro-3-(3-chloro-2-(1-methyl-3-(((((R)-tetrahydrofuran-2-yl)methyl)amino)methyl)-1H-indol-6-yl)pyridin-4-yl)phenyl)-2-methoxypyridin-3-yl)methyl)amino)methyl)pyrrolidin-2-one;
   (S)-5-((((6-(2-chloro-3-(3-chloro-2-(1-methyl-3-(((tetrahydro-2H-pyran-4-yl)amino)methyl)-1H-indol-6-yl)pyridin-4-yl)phenyl)-2-methoxypyridin-3-yl)methyl)amino)methyl)pyrrolidin-2-one;
   (R)-5-((((6-(2-chloro-3-(3-chloro-2-(1-methyl-3-(((tetrahydro-2H-pyran-4-yl)amino)methyl)-1H-indol-6-yl)pyridin-4-yl)phenyl)-2-methoxypyridin-3-yl)methyl)amino)methyl)pyrrolidin-2-one;
   (S)-5-((((6-(2-chloro-3-(3-chloro-2-(3-(((1,1-dioxidotetrahydro-2H-thiopyran-4-yl)amino)methyl)- 1-methyl-1H-indol-6-yl)pyridin-4-yl)phenyl)-2-methoxypyridin-3-yl)methyl)amino)methyl)pyrrolidin-2-one;
   (R)-5-((((6-(2-chloro-3-(3-chloro-2-(3-(((1,1-dioxidotetrahydro-2H-thiopyran-4-yl)amino)methyl)- 1-methyl-1H-indol-6-yl)pyridin-4-yl)phenyl)-2-methoxypyridin-3-yl)methyl)amino)methyl)pyrrolidin-2-one;
   (S)-5-((((6-(2-chloro-3-(3-chloro-2-(2-((S)-2-hydroxypropyl)-5-methoxy-1,2,3,4-tetrahydroisoquinolin-7-yl)pyridin-4-yl)phenyl)-2-methoxypyridin-3-yl)methyl)amino)methyl)pyrrolidin-2-one;
   (S)-5-((((6-(2-chloro-3-(3-chloro-2-(2-((R)-2-hydroxypropyl)-5-methoxy-1,2,3,4-tetrahydroisoquinolin-7-yl)pyridin-4-yl)phenyl)-2-methoxypyridin-3-yl)methyl)amino)methyl)pyrrolidin-2-one;
   (R)-5-((((6-(2-chloro-3-(3-chloro-2-(2-((S)-2-hydroxypropyl)-5-methoxy-1,2,3,4-tetrahydroisoquinolin-7-yl)pyridin-4-yl)phenyl)-2-methoxypyridin-3-yl)methyl)amino)methyl)pyrrolidin-2-one;
   (R)-5-((((6-(2-chloro-3-(3-chloro-2-(2-((R)-2-hydroxypropyl)-5-methoxy-1,2,3,4-tetrahydroisoquinolin-7-yl)pyridin-4-yl)phenyl)-2-methoxypyridin-3-yl)methyl)amino)methyl)pyrrolidin-2-one;
   (S)-5-((((6-(2-chloro-3-(3-chloro-2-(2-(2-hydroxy-2-methylpropyl)-5-methoxy-1,2,3,4-tetrahydroisoquinolin-7-yl)pyridin-4-yl)phenyl)-2-methoxypyridin-3-yl)methyl)amino)methyl)pyrrolidin-2-one;
   (R)-5-((((6-(2-chloro-3-(3-chloro-2-(2-(2-hydroxy-2-methylpropyl)-5-methoxy-1,2,3,4-tetrahydroisoquinolin-7-yl)pyridin-4-yl)phenyl)-2-methoxypyridin-3-yl)methyl)amino)methyl)pyrrolidin-2-one;
   (S)-5-((((6-(2-chloro-3-(3-chloro-2-(8-fluoro-2-(2-hydroxy-2-methylpropyl)-1,2,3,4-tetrahydroisoquinolin-6-yl)pyridin-4-yl)phenyl)-2-methoxypyridin-3-yl)methyl)amino)methyl)pyrrolidin-2-one;
   (R)-5-((((6-(2-chloro-3-(3-chloro-2-(8-fluoro-2-(2-hydroxy-2-methylpropyl)-1,2,3,4-tetrahydroisoquinolin-6-yl)pyridin-4-yl)phenyl)-2-methoxypyridin-3-yl)methyl)amino)methyl)pyrrolidin-2-one;
   (S)-5-((((6-(2-chloro-3-(3-chloro-2-(5-fluoro-2-(2-hydroxy-2-methylpropyl)-1,2,3,4-tetrahydroisoquinolin-7-yl)pyridin-4-yl)phenyl)-2-methoxypyridin-3-yl)methyl)amino)methyl)pyrrolidin-2-one;
   (R)-5-((((6-(2-chloro-3-(3-chloro-2-(5-fluoro-2-(2-hydroxy-2-methylpropyl)-1,2,3,4-tetrahydroisoquinolin-7-yl)pyridin-4-yl)phenyl)-2-methoxypyridin-3-yl)methyl)amino)methyl)pyrrolidin-2-one;
   (S)-6-(3-chloro-4-(2-chloro-3-(6-methoxy-5-((((5-oxopyrrolidin-2-yl)methyl)amino)methyl)pyridin-2-yl)phenyl)pyridin-2-yl)-2-((6-hydroxy-2-azaspiro[3.3]heptan-2-yl)methyl)-3-methylpyrrolo[2,1-f][1,2,4]triazin-4(3H)-one;
   (R)-6-(3-chloro-4-(2-chloro-3-(6-methoxy-5-((((5-oxopyrrolidin-2-yl)methyl)amino)methyl)pyridin-2-yl)phenyl)pyridin-2-yl)-2-((6-hydroxy-2-azaspiro[3.3]heptan-2-yl)methyl)-3-methylpyrrolo[2,1-f][1,2,4]triazin-4(3H)-one;
   (S)-5-((((6-(2-chloro-3-(3-chloro-2-(2-(3-hydroxy-3-methylbutyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
   (R)-5-((((6-(2-chloro-3-(3-chloro-2-(2-(3-hydroxy-3-methylbutyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
   (S)-5-((((6-(2-chloro-3-(3-chloro-2-(2-((S)-2-hydroxypropyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
   (S)-5-((((6-(2-chloro-3-(3-chloro-2-(2-((R)-2-hydroxypropyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
   (R)-5-((((6-(2-chloro-3-(3-chloro-2-(2-((S)-2-hydroxypropyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
   (R)-5-((((6-(2-chloro-3-(3-chloro-2-(2-((R)-2-hydroxypropyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
   (S)-5-((((6-(2-chloro-3-(3-chloro-2-(2-(2-(1-hydroxycyclobutyl)ethyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
   (R)-5-((((6-(2-chloro-3-(3-chloro-2-(2-(2-(1-hydroxycyclobutyl)ethyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
   (S)-5-((((6-(2-chloro-3-(3-chloro-2-(7-(((((S)-5-oxopyrrolidin-2-yl)methyl)amino)methyl)quinolin-3-yl)pyridin-4-yl)phenyl)-2-methoxypyridin-3-yl)methyl)amino)methyl)pyrrolidin-2-one;
   (S)-5-((((6-(2-chloro-3-(3-chloro-2-(7-(((((R)-5-oxopyrrolidin-2-yl)methyl)amino)methyl)quinolin-3-yl)pyridin-4-yl)phenyl)-2-methoxypyridin-3-yl)methyl)amino)methyl)pyrrolidin-2-one;
   (R)-5-((((6-(2-chloro-3-(3-chloro-2-(7-(((((S)-5-oxopyrrolidin-2-yl)methyl)amino)methyl)quinolin-3-yl)pyridin-4-yl)phenyl)-2-methoxypyridin-3-yl)methyl)amino)methyl)pyrrolidin-2-one;
   (R)-5-((((6-(2-chloro-3-(3-chloro-2-(7-(((((R)-5-oxopyrrolidin-2-yl)methyl)amino)methyl)quinolin-3-yl)pyridin-4-yl)phenyl)-2-methoxypyridin-3-yl)methyl)amino)methyl)pyrrolidin-2-one;
   (5S)-5-((((6-(2-chloro-3-(3-chloro-2-(2-(2-hydroxy-3-methylbutyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
   (SR)-5-((((6-(2-chloro-3-(3-chloro-2-(2-(2-hydroxy-3-methylbutyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
   (S)-5-((((6-(2-chloro-3-(3-chloro-2-(2-(2-hydroxyethyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
   (R)-5-((((6-(2-chloro-3-(3-chloro-2-(2-(2-hydroxyethyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
   (S)-5-((((6-(3-(2-(3-(((1-acetylazetidin-3-yl)amino)methyl)-1-methyl-1H-indol-6-yl)-3-chloropyridin-4-yl)-2-chlorophenyl)-2-methoxypyridin-3-yl)methyl)amino)methyl)pyrrolidin-2-one;
   (R)-5-((((6-(3-(2-(3-(((1-acetylazetidin-3-yl)amino)methyl)-1-methyl-1H-indol-6-yl)-3-chloropyridin-4-yl)-2-chlorophenyl)-2-methoxypyridin-3-yl)methyl)amino)methyl)pyrrolidin-2-one;
   (S)-5-((((6-(3-(2-(3-(((1-acetylpiperidin-4-yl)(methyl)amino)methyl)-1-methyl-1H-indol-6-yl)-3-chloropyridin-4-yl)-2-chlorophenyl)-2-methoxypyridin-3-yl)methyl)amino)methyl)pyrrolidin-2-one;
   (R)-5-((((6-(3-(2-(3-(((1-acetylpiperidin-4-yl)(methyl)amino)methyl)-1-methyl-1H-indol-6-yl)-3-chloropyridin-4-yl)-2-chlorophenyl)-2-methoxypyridin-3-yl)methyl)amino)methyl)pyrrolidin-2-one;
   (S)-5-(((4-(4-(3-(3-(((1-acetylpiperidin-4-yl)amino)methyl)-1-methyl-1H-pyrrolo[2,3-b]pyridin-6-yl)-2-chlorophenyl)-3-chloropyridin-2-yl)-2-methoxybenzyl)amino)methyl)pyrrolidin-2-one;
   (R)-5-(((4-(4-(3-(3-(((1-acetylpiperidin-4-yl)amino)methyl)-1-methyl-1H-pyrrolo[2,3-b]pyridin-6-yl)-2-chlorophenyl)-3-chloropyridin-2-yl)-2-methoxybenzyl)amino)methyl)pyrrolidin-2-one;
   (S)-5-(((4-(3-chloro-4-(2-chloro-3-(1-methyl-3-(((((S)-oxetan-2-yl)methyl)amino)methyl)-1H-pyrrolo[2,3-b]pyridin-6-yl)phenyl)pyridin-2-yl)-2-methoxybenzyl)amino)methyl)pyrrolidin-2-one;
   (S)-5-(((4-(3-chloro-4-(2-chloro-3-(1-methyl-3-(((((R)-oxetan-2-yl)methyl)amino)methyl)-1H-pyrrolo[2,3-b]pyridin-6-yl)phenyl)pyridin-2-yl)-2-methoxybenzyl)amino)methyl)pyrrolidin-2-one;
   (R)-5-(((4-(3-chloro-4-(2-chloro-3-(1-methyl-3-(((((S)-oxetan-2-yl)methyl)amino)methyl)-1H-pyrrolo[2,3-b]pyridin-6-yl)phenyl)pyridin-2-yl)-2-methoxybenzyl)amino)methyl)pyrrolidin-2-one;
   (R)-5-(((4-(3-chloro-4-(2-chloro-3-(1-methyl-3-(((((R)-oxetan-2-yl)methyl)amino)methyl)-1H-pyrrolo[2,3-b]pyridin-6-yl)phenyl)pyridin-2-yl)-2-methoxybenzyl)amino)methyl)pyrrolidin-2-one;
   (S)-5-(((4-(3-chloro-4-(2-chloro-3-(1-methyl-3-(((tetrahydro-2H-pyran-4-yl)amino)methyl)-1H-pyrrolo[2,3-b]pyridin-6-yl)phenyl)pyridin-2-yl)-2-methoxybenzyl)amino)methyl)pyrrolidin-2-one;
   (R)-5-(((4-(3-chloro-4-(2-chloro-3-(1-methyl-3-(((tetrahydro-2H-pyran-4-yl)amino)methyl)-1H-pyrrolo[2,3-b]pyridin-6-yl)phenyl)pyridin-2-yl)-2-methoxybenzyl)amino)methyl)pyrrolidin-2-one;
   (S)-5-(((4-(3-chloro-4-(2-chloro-3-(3-(((1,1-dioxidotetrahydro-2H-thiopyran-4-yl)amino)methyl)-1-methyl-1H-pyrrolo [2,3-b]pyridin-6-yl)phenyl)pyridin-2-yl)-2-methoxybenzyl)amino)methyl)pyrrolidin-2-one;
   (R)-5-(((4-(3-chloro-4-(2-chloro-3-(3-(((1,1-dioxidotetrahydro-2H-thiopyran-4-yl)amino)methyl)-1-methyl-1H-pyrrolo[2,3-b]pyridin-6-yl)phenyl)pyridin-2-yl)-2-methoxybenzyl)amino)methyl)pyrrolidin-2-one;
   (S)-5-((((6-(2-chloro-3-(3-chloro-2-(8-fluoro-2-(((S)-5-oxopyrrolidin-2-yl)methyl)-1,2,3,4-tetrahydroisoquinolin-6-yl)pyridin-4-yl)phenyl)-2-methoxypyridin-3-yl)methyl)amino)methyl)pyrrolidin-2-one;
   (S)-5-((((6-(2-chloro-3-(3-chloro-2-(8-fluoro-2-(((R)-5-oxopyrrolidin-2-yl)methyl)-1,2,3,4-tetrahydroisoquinolin-6-yl)pyridin-4-yl)phenyl)-2-methoxypyridin-3-yl)methyl)amino)methyl)pyrrolidin-2-one;
   (R)-5-((((6-(2-chloro-3-(3-chloro-2-(8-fluoro-2-(((S)-5-oxopyrrolidin-2-yl)methyl)-1,2,3,4-tetrahydroisoquinolin-6-yl)pyridin-4-yl)phenyl)-2-methoxypyridin-3-yl)methyl)amino)methyl)pyrrolidin-2-one;
   (R)-5-((((6-(2-chloro-3-(3-chloro-2-(8-fluoro-2-(((R)-5-oxopyrrolidin-2-yl)methyl)-1,2,3,4-tetrahydroisoquinolin-6-yl)pyridin-4-yl)phenyl)-2-methoxypyridin-3-yl)methyl)amino)methyl)pyrrolidin-2-one;
   (S)-5-((((6-(3-(2-(2-((1-acetylpiperidin-4-yl)methyl)-8-methoxy-1,2,3,4-tetrahydroisoquinolin-6-yl)-3-chloropyridin-4-yl)-2-chlorophenyl)-2-methoxypyridin-3-yl)methyl)amino)methyl)pyrrolidin-2-one;
   (R)-5-((((6-(3-(2-(2-((1-acetylpiperidin-4-yl)methyl)-8-methoxy-1,2,3,4-tetrahydroisoquinolin-6-yl)-3-chloropyridin-4-yl)-2-chlorophenyl)-2-methoxypyridin-3-yl)methyl)amino)methyl)pyrrolidin-2-one;
   (S)-5-((((6-(2-chloro-3-(3-chloro-2-(2-(3-fluoropropyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
   (R)-5-((((6-(2-chloro-3-(3-chloro-2-(2-(3-fluoropropyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
   (S)-5-((((6-(2-chloro-3-(3-chloro-2-(8-methoxy-2-(2-(piperidin-4-yl)ethyl)-1,2,3,4-tetrahydroisoquinolin-6-yl)pyridin-4-yl)phenyl)-2-methoxypyridin-3-yl)methyl)amino)methyl)pyrrolidin-2-one;
   (R)-5-((((6-(2-chloro-3-(3-chloro-2-(8-methoxy-2-(2-(piperidin-4-yl)ethyl)-1,2,3,4-tetrahydroisoquinolin-6-yl)pyridin-4-yl)phenyl)-2-methoxypyridin-3-yl)methyl)amino)methyl)pyrrolidin-2-one;
   (S)-5-((((6-(2-chloro-3-(3-chloro-2-(8-fluoro-2-(3-fluoropropyl)-1,2,3,4-tetrahydroisoquinolin-6-yl)pyridin-4-yl)phenyl)-2-methoxypyridin-3-yl)methyl)amino)methyl)pyrrolidin-2-one;
   (R)-5-((((6-(2-chloro-3-(3-chloro-2-(8-fluoro-2-(3-fluoropropyl)-1,2,3,4-tetrahydroisoquinolin-6-yl)pyridin-4-yl)phenyl)-2-methoxypyridin-3-yl)methyl)amino)methyl)pyrrolidin-2-one;
   5-(3-chloro-4-(2-chloro-3-(6-methoxy-5-(((((S)-5-oxopyrrolidin-2-yl)methyl)amino)methyl)pyridin-2-yl)phenyl)pyridin-2-yl)-2-(2-((((S)-5-oxopyrrolidin-2-yl)methyl)amino)ethyl)isoindolin-1-one;
   5-(3-chloro-4-(2-chloro-3-(6-methoxy-5-(((((S)-5-oxopyrrolidin-2-yl)methyl)amino)methyl)pyridin-2-yl)phenyl)pyridin-2-yl)-2-(2-((((R)-5-oxopyrrolidin-2-yl)methyl)amino)ethyl)isoindolin-1-one;
   5-(3-chloro-4-(2-chloro-3-(6-methoxy-5-(((((R)-5-oxopyrrolidin-2-yl)methyl)amino)methyl)pyridin-2-yl)phenyl)pyridin-2-yl)-2-(2-((((S)-5-oxopyrrolidin-2-yl)methyl)amino)ethyl)isoindolin-1-one;
   5-(3-chloro-4-(2-chloro-3-(6-methoxy-5-(((((R)-5-oxopyrrolidin-2-yl)methyl)amino)methyl)pyridin-2-yl)phenyl)pyridin-2-yl)-2-(2-((((R)-5-oxopyrrolidin-2-yl)methyl)amino)ethyl)isoindolin-1-one;
   (S)-5-((((6-(2-chloro-3-(3-chloro-2-(8-chloro-2-(((S)-5-oxopyrrolidin-2-yl)methyl)-1,2,3,4-tetrahydroisoquinolin-6-yl)pyridin-4-yl)phenyl)-2-methoxypyridin-3-yl)methyl)amino)methyl)pyrrolidin-2-one;
   (S)-5-((((6-(2-chloro-3-(3-chloro-2-(8-chloro-2-(((R)-5-oxopyrrolidin-2-yl)methyl)-1,2,3,4-tetrahydroisoquinolin-6-yl)pyridin-4-yl)phenyl)-2-methoxypyridin-3-yl)methyl)amino)methyl)pyrrolidin-2-one;
   (R)-5-((((6-(2-chloro-3-(3-chloro-2-(8-chloro-2-(((S)-5-oxopyrrolidin-2-yl)methyl)-1,2,3,4-tetrahydroisoquinolin-6-yl)pyridin-4-yl)phenyl)-2-methoxypyridin-3-yl)methyl)amino)methyl)pyrrolidin-2-one;
   (R)-5-((((6-(2-chloro-3-(3-chloro-2-(8-chloro-2-(((R)-5-oxopyrrolidin-2-yl)methyl)-1,2,3,4-tetrahydroisoquinolin-6-yl)pyridin-4-yl)phenyl)-2-methoxypyridin-3-yl)methyl)amino)methyl)pyrrolidin-2-one;
   isopropyl (4-(3-chloro-4-(2-chloro-3-(6-methoxy-5-(((((S)-5-oxopyrrolidin-2-yl)methyl)amino)methyl)pyridin-2-yl)phenyl)pyridin-2-yl)-2-methoxybenzyl)-L-prolinate;
   isopropyl (4-(3-chloro-4-(2-chloro-3-(6-methoxy-5-(((((S)-5-oxopyrrolidin-2-yl)methyl)amino)methyl)pyridin-2-yl)phenyl)pyridin-2-yl)-2-methoxybenzyl)-D-prolinate;
   isopropyl (4-(3-chloro-4-(2-chloro-3-(6-methoxy-5-(((((R)-5-oxopyrrolidin-2-yl)methyl)amino)methyl)pyridin-2-yl)phenyl)pyridin-2-yl)-2-methoxybenzyl)-L-prolinate;
   isopropyl (4-(3-chloro-4-(2-chloro-3-(6-methoxy-5-(((((R)-5-oxopyrrolidin-2-yl)methyl)amino)methyl)pyridin-2-yl)phenyl)pyridin-2-yl)-2-methoxybenzyl)-D-prolinate;
   (S)-5-((((6-(2-chloro-3-(3-chloro-2-(8-chloro-2-((S)-2-hydroxypropyl)-1,2,3,4-tetrahydroisoquinolin-6-yl)pyridin-4-yl)phenyl)-2-methoxypyridin-3-yl)methyl)amino)methyl)pyrrolidin-2-one;
   (S)-5-((((6-(2-chloro-3-(3-chloro-2-(8-chloro-2-((R)-2-hydroxypropyl)-1,2,3,4-tetrahydroisoquinolin-6-yl)pyridin-4-yl)phenyl)-2-methoxypyridin-3-yl)methyl)amino)methyl)pyrrolidin-2-one;
   (R)-5-((((6-(2-chloro-3-(3-chloro-2-(8-chloro-2-((S)-2-hydroxypropyl)-1,2,3,4-tetrahydroisoquinolin-6-yl)pyridin-4-yl)phenyl)-2-methoxypyridin-3-yl)methyl)amino)methyl)pyrrolidin-2-one;
   (R)-5-((((6-(2-chloro-3-(3-chloro-2-(8-chloro-2-((R)-2-hydroxypropyl)-1,2,3,4-tetrahydroisoquinolin-6-yl)pyridin-4-yl)phenyl)-2-methoxypyridin-3-yl)methyl)amino)methyl)pyrrolidin-2-one;
   (S)-5-((((6-(2-chloro-3-(3-chloro-2-(4-(((2-hydroxyethyl)amino)methyl)-3-methoxyphenyl)pyridin-4-yl)phenyl)-2-methoxypyridin-3-yl)methyl)amino)methyl)pyrrolidin-2-one;
   (R)-5-((((6-(2-chloro-3-(3-chloro-2-(4-(((2-hydroxyethyl)amino)methyl)-3-methoxyphenyl)pyridin-4-yl)phenyl)-2-methoxypyridin-3-yl)methyl)amino)methyl)pyrrolidin-2-one;
   (S)-5-((((4-chloro-6-(2-chloro-3-(3-chloro-2-(4-((((S)-2-hydroxypropyl)(methyl)amino)methyl)-3-methoxyphenyl)pyridin-4-yl)phenyl)-2-methoxypyridin-3-yl)methyl)amino)methyl)pyrrolidin-2-one;
   (S)-5-((((4-chloro-6-(2-chloro-3-(3-chloro-2-(4-((((R)-2-hydroxypropyl)(methyl)amino)methyl)-3-methoxyphenyl)pyridin-4-yl)phenyl)-2-methoxypyridin-3-yl)methyl)amino)methyl)pyrrolidin-2-one;
   (R)-5-((((4-chloro-6-(2-chloro-3-(3-chloro-2-(4-((((S)-2-hydroxypropyl)(methyl)amino)methyl)-3-methoxyphenyl)pyridin-4-yl)phenyl)-2-methoxypyridin-3-yl)methyl)amino)methyl)pyrrolidin-2-one;
   (R)-5-((((4-chloro-6-(2-chloro-3-(3-chloro-2-(4-((((R)-2-hydroxypropyl)(methyl)amino)methyl)-3-methoxyphenyl)pyridin-4-yl)phenyl)-2-methoxypyridin-3-yl)methyl)amino)methyl)pyrrolidin-2-one;
   (S)-5-((((6-(2-chloro-3-(3-chloro-2-(8-chloro-2-(3-fluoropropyl)-1,2,3,4-tetrahydroisoquinolin-6-yl)pyridin-4-yl)phenyl)-2-methoxypyridin-3-yl)methyl)amino)methyl)pyrrolidin-2-one;
   (R)-5-((((6-(2-chloro-3-(3-chloro-2-(8-chloro-2-(3-fluoropropyl)-1,2,3,4-tetrahydroisoquinolin-6-yl)pyridin-4-yl)phenyl)-2-methoxypyridin-3-yl)methyl)amino)methyl)pyrrolidin-2-one;
   (S)-5-((((6-(2-chloro-3-(3-chloro-2-(3-(((4,4-difluorocyclohexyl)amino)methyl)-1-methyl-1H-indol-6-yl)pyridin-4-yl)phenyl)-2-methoxypyridin-3-yl)methyl)amino)methyl)pyrrolidin-2-one;
   (R)-5-((((6-(2-chloro-3-(3-chloro-2-(3-(((4,4-difluorocyclohexyl)amino)methyl)-1-methyl-1H-indol-6-yl)pyridin-4-yl)phenyl)-2-methoxypyridin-3-yl)methyl)amino)methyl)pyrrolidin-2-one;
   (S)-5-((((6-(2-chloro-3-(3-chloro-2-(2-(2-(1-hydroxycyclopropyl)ethyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
   (R)-5-((((6-(2-chloro-3-(3-chloro-2-(2-(2-(1-hydroxycyclopropyl)ethyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
   (S)-5-((((6-(2-chloro-3-(3-chloro-2-(4-((((S)-2-hydroxypropyl)(methyl)amino)methyl)-3-methoxyphenyl)pyridin-4-yl)phenyl)-2-methoxypyridin-3-yl)methyl)amino)methyl)pyrrolidin-2-one;
   (S)-5-((((6-(2-chloro-3-(3-chloro-2-(4-((((R)-2-hydroxypropyl)(methyl)amino)methyl)-3-methoxyphenyl)pyridin-4-yl)phenyl)-2-methoxypyridin-3-yl)methyl)amino)methyl)pyrrolidin-2-one;
   (R)-5-((((6-(2-chloro-3-(3-chloro-2-(4-((((S)-2-hydroxypropyl)(methyl)amino)methyl)-3-methoxyphenyl)pyridin-4-yl)phenyl)-2-methoxypyridin-3-yl)methyl)amino)methyl)pyrrolidin-2-one;
   (R)-5-((((6-(2-chloro-3-(3-chloro-2-(4-((((R)-2-hydroxypropyl)(methyl)amino)methyl)-3-methoxyphenyl)pyridin-4-yl)phenyl)-2-methoxypyridin-3-yl)methyl)amino)methyl)pyrrolidin-2-one;
   isopropyl (S)-1-(4-(3-chloro-4-(2-chloro-3-(6-methoxy-5-(((((S)-5-oxopyrrolidin-2-yl)methyl)amino)methyl)pyridin-2-yl)phenyl)pyridin-2-yl)-2-methoxybenzyl)pyrrolidine-3-carboxylate;
   isopropyl (S)-1-(4-(3-chloro-4-(2-chloro-3-(6-methoxy-5-(((((R)-5-oxopyrrolidin-2-yl)methyl)amino)methyl)pyridin-2-yl)phenyl)pyridin-2-yl)-2-methoxybenzyl)pyrrolidine-3-carboxylate;
   isopropyl (R)-1-(4-(3-chloro-4-(2-chloro-3-(6-methoxy-5-(((((S)-5-oxopyrrolidin-2-yl)methyl)amino)methyl)pyridin-2-yl)phenyl)pyridin-2-yl)-2-methoxybenzyl)pyrrolidine-3-carboxylate;
   isopropyl (R)-1-(4-(3-chloro-4-(2-chloro-3-(6-methoxy-5-(((((R)-5-oxopyrrolidin-2-yl)methyl)amino)methyl)pyridin-2-yl)phenyl)pyridin-2-yl)-2-methoxybenzyl)pyrrolidine-3-carboxylate;
   (S)-1-(4-(3-chloro-4-(2-chloro-3-(6-methoxy-5-(((((S)-5-oxopyrrolidin-2-yl)methyl)amino)methyl)pyridin-2-yl)phenyl)pyridin-2-yl)-2-methoxybenzyl)pyrrolidine-3-carboxylic acid;
   (S)-1-(4-(3-chloro-4-(2-chloro-3-(6-methoxy-5-(((((R)-5-oxopyrrolidin-2-yl)methyl)amino)methyl)pyridin-2-yl)phenyl)pyridin-2-yl)-2-methoxybenzyl)pyrrolidine-3-carboxylic acid;
   (R)-1-(4-(3-chloro-4-(2-chloro-3-(6-methoxy-5-(((((S)-5-oxopyrrolidin-2-yl)methyl)amino)methyl)pyridin-2-yl)phenyl)pyridin-2-yl)-2-methoxybenzyl)pyrrolidine-3-carboxylic acid;
   (R)-1-(4-(3-chloro-4-(2-chloro-3-(6-methoxy-5-(((((R)-5-oxopyrrolidin-2-yl)methyl)amino)methyl)pyridin-2-yl)phenyl)pyridin-2-yl)-2-methoxybenzyl)pyrrolidine-3-carboxylic acid;
   (4-(3-chloro-4-(2-chloro-3-(6-methoxy-5-(((((S)-5-oxopyrrolidin-2-yl)methyl)amino)methyl)pyridin-2-yl)phenyl)pyridin-2-yl)-2-methoxybenzyl)-L-proline;
   (4-(3-chloro-4-(2-chloro-3-(6-methoxy-5-(((((S)-5-oxopyrrolidin-2-yl)methyl)amino)methyl)pyridin-2-yl)phenyl)pyridin-2-yl)-2-methoxybenzyl)-D-proline;
   (4-(3-chloro-4-(2-chloro-3-(6-methoxy-5-(((((R)-5-oxopyrrolidin-2-yl)methyl)amino)methyl)pyridin-2-yl)phenyl)pyridin-2-yl)-2-methoxybenzyl)-L-proline;
   (4-(3-chloro-4-(2-chloro-3-(6-methoxy-5-(((((R)-5-oxopyrrolidin-2-yl)methyl)amino)methyl)pyridin-2-yl)phenyl)pyridin-2-yl)-2-methoxybenzyl)-D-proline;
   (S)-5-((((6-(2-chloro-3-(3-chloro-2-(3-fluoro-4-((((1r,3r)-3-hydroxy-3-methylcyclobutyl)amino)methyl)-5-methoxyphenyl)pyridin-4-yl)phenyl)-2-methoxypyridin-3-yl)methyl)amino)methyl)pyrrolidin-2-one;
   (R)-5-((((6-(2-chloro-3-(3-chloro-2-(3-fluoro-4-((((1r,3r)-3-hydroxy-3-methylcyclobutyl)amino)methyl)-5-methoxyphenyl)pyridin-4-yl)phenyl)-2-methoxypyridin-3-yl)methyl)amino)methyl)pyrrolidin-2-one;
   (R)-5-((((6-(2-chloro-3-(3-chloro-2-(3-fluoro-4-((((1r,3s)-3-hydroxy-3-methylcyclobutyl)amino)methyl)-5-methoxyphenyl)pyridin-4-yl)phenyl)-2-methoxypyridin-3-yl)methyl)amino)methyl)pyrrolidin-2-one;
   (R)-5-((((6-(2-chloro-3-(3-chloro-2-(3-fluoro-4-((((1s,3r)-3-hydroxy-3-methylcyclobutyl)amino)methyl)-5-methoxyphenyl)pyridin-4-yl)phenyl)-2-methoxypyridin-3-yl)methyl)amino)methyl)pyrrolidin-2-one;
   (S)-5-((((6-(2-chloro-3-(3-chloro-2-(3-fluoro-4-((((1s,3r)-3-hydroxy-3-methylcyclobutyl)amino)methyl)-5-methoxyphenyl)pyridin-4-yl)phenyl)-2-methoxypyridin-3-yl)methyl)amino)methyl)pyrrolidin-2-one;
   (S)-5-((((6-(2-chloro-3-(3-chloro-2-(3-fluoro-4-((((1r,3s)-3-hydroxy-3-methylcyclobutyl)amino)methyl)-5-methoxyphenyl)pyridin-4-yl)phenyl)-2-methoxypyridin-3-yl)methyl)amino)methyl)pyrrolidin-2-one;
   (R)-5-((((6-(2-chloro-3-(3-chloro-2-(3-fluoro-4-((((1s,3s)-3-hydroxy-3-methylcyclobutyl)amino)methyl)-5-methoxyphenyl)pyridin-4-yl)phenyl)-2-methoxypyridin-3-yl)methyl)amino)methyl)pyrrolidin-2-one;
   (S)-5-((((6-(2-chloro-3-(3-chloro-2-(3-fluoro-4-((((1s,3s)-3-hydroxy-3-methylcyclobutyl)amino)methyl)-5-methoxyphenyl)pyridin-4-yl)phenyl)-2-methoxypyridin-3-yl)methyl)amino)methyl)pyrrolidin-2-one;
   (S)-5-((((6-(2-chloro-3-(3-chloro-2-(3-fluoro-4-((((S)-2-hydroxypropyl)amino)methyl)-5-methoxyphenyl)pyridin-4-yl)phenyl)-2-methoxypyridin-3-yl)methyl)amino)methyl)pyrrolidin-2-one;
   (R)-5-((((6-(2-chloro-3-(3-chloro-2-(3-fluoro-4-((((S)-2-hydroxypropyl)amino)methyl)-5-methoxyphenyl)pyridin-4-yl)phenyl)-2-methoxypyridin-3-yl)methyl)amino)methyl)pyrrolidin-2-one;
   (R)-5-((((6-(2-chloro-3-(3-chloro-2-(3-fluoro-4-((((R)-2-hydroxypropyl)amino)methyl)-5-methoxyphenyl)pyridin-4-yl)phenyl)-2-methoxypyridin-3-yl)methyl)amino)methyl)pyrrolidin-2-one;
   (S)-5-((((6-(2-chloro-3-(3-chloro-2-(3-fluoro-4-((((R)-2-hydroxypropyl)amino)methyl)-5-methoxyphenyl)pyridin-4-yl)phenyl)-2-methoxypyridin-3-yl)methyl)amino)methyl)pyrrolidin-2-one;
   (S)-5-((((6-(2-chloro-3-(3-chloro-2-(3-fluoro-4-((isopropylamino)methyl)-5-methoxyphenyl)pyridin-4-yl)phenyl)-2-methoxypyridin-3-yl)methyl)amino)methyl)pyrrolidin-2-one;
   (R)-5-((((6-(2-chloro-3-(3-chloro-2-(3-fluoro-4-((isopropylamino)methyl)-5-methoxyphenyl)pyridin-4-yl)phenyl)-2-methoxypyridin-3-yl)methyl)amino)methyl)pyrrolidin-2-one;
   (S)-5-((((6-(2-chloro-3-(3-chloro-2-(3-fluoro-4-((isobutylamino)methyl)-5-methoxyphenyl)pyridin-4-yl)phenyl)-2-methoxypyridin-3-yl)methyl)amino)methyl)pyrrolidin-2-one;
   (R)-5-((((6-(2-chloro-3-(3-chloro-2-(3-fluoro-4-((isobutylamino)methyl)-5-methoxyphenyl)pyridin-4-yl)phenyl)-2-methoxypyridin-3-yl)methyl)amino)methyl)pyrrolidin-2-one;
   2-(((6-(2-chloro-3-(3-chloro-2-(2-(3-fluoropropyl)-8-methoxy-1,2,3,4-tetrahydroisoquinolin-6-yl)pyridin-4-yl)phenyl)-2-methoxypyridin-3-yl)methyl)amino)ethan-1-ol;
   2-(((6-(2-chloro-3-(3-chloro-2-(8-methoxy-2-(2-(piperidin-4-yl)ethyl)-1,2,3,4-tetrahydroisoquinolin-6-yl)pyridin-4-yl)phenyl)-2-methoxypyridin-3-yl)methyl)amino)ethan-1-ol;
   (S)-5-((((4-chloro-6-(2-chloro-3-(3-chloro-2-(2-((S)-2-hydroxypropyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
   (S)-5-((((4-chloro-6-(2-chloro-3-(3-chloro-2-(2-((R)-2-hydroxypropyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
   (R)-5-((((4-chloro-6-(2-chloro-3-(3-chloro-2-(2-((S)-2-hydroxypropyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
   (R)-5-((((4-chloro-6-(2-chloro-3-(3-chloro-2-(2-((R)-2-hydroxypropyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
   6-(3-chloro-4-(2-chloro-3-(4-chloro-6-methoxy-5-(((((S)-5-oxopyrrolidin-2-yl)methyl)amino)methyl)pyridin-2-yl)phenyl)pyridin-2-yl)-2-((((1r,3r)-3-hydroxy-3-methylcyclobutyl)amino)methyl)-3-methylpyrrolo[2,1-f][1,2,4]triazin-4(3H)-one;
   6-(3-chloro-4-(2-chloro-3-(4-chloro-6-methoxy-5-(((((R)-5-oxopyrrolidin-2-yl)methyl)amino)methyl)pyridin-2-yl)phenyl)pyridin-2-yl)-2-((((1r,3s)-3-hydroxy-3-methylcyclobutyl)amino)methyl)-3-methylpyrrolo[2,1-f][1,2,4]triazin-4(3H)-one;
   6-(3-chloro-4-(2-chloro-3-(4-chloro-6-methoxy-5-(((((R)-5-oxopyrrolidin-2-yl)methyl)amino)methyl)pyridin-2-yl)phenyl)pyridin-2-yl)-2-((((1s,3r)-3-hydroxy-3-methylcyclobutyl)amino)methyl)-3-methylpyrrolo[2,1-f][1,2,4]triazin-4(3H)-one;
   6-(3-chloro-4-(2-chloro-3-(4-chloro-6-methoxy-5-(((((S)-5-oxopyrrolidin-2-yl)methyl)amino)methyl)pyridin-2-yl)phenyl)pyridin-2-yl)-2-((((1s,3r)-3-hydroxy-3-methylcyclobutyl)amino)methyl)-3-methylpyrrolo[2,1-f][1,2,4]triazin-4(3H)-one;
   6-(3-chloro-4-(2-chloro-3-(4-chloro-6-methoxy-5-(((((S)-5-oxopyrrolidin-2-yl)methyl)amino)methyl)pyridin-2-yl)phenyl)pyridin-2-yl)-2-((((1r,3s)-3-hydroxy-3-methylcyclobutyl)amino)methyl)-3-methylpyrrolo[2,1-f][1,2,4]triazin-4(3H)-one;
   6-(3-chloro-4-(2-chloro-3-(4-chloro-6-methoxy-5-(((((R)-5-oxopyrrolidin-2-yl)methyl)amino)methyl)pyridin-2-yl)phenyl)pyridin-2-yl)-2-((((1s,3s)-3-hydroxy-3-methylcyclobutyl)amino)methyl)-3-methylpyrrolo[2,1-f][1,2,4]triazin-4(3H)-one;
   6-(3-chloro-4-(2-chloro-3-(4-chloro-6-methoxy-5-(((((R)-5-oxopyrrolidin-2-yl)methyl)amino)methyl)pyridin-2-yl)phenyl)pyridin-2-yl)-2-((((1r,3r)-3-hydroxy-3-methylcyclobutyl)amino)methyl)-3-methylpyrrolo[2,1-f][1,2,4]triazin-4(3H)-one;
   6-(3-chloro-4-(2-chloro-3-(4-chloro-6-methoxy-5-(((((S)-5-oxopyrrolidin-2-yl)methyl)amino)methyl)pyridin-2-yl)phenyl)pyridin-2-yl)-2-((((1s,3s)-3-hydroxy-3-methylcyclobutyl)amino)methyl)-3-methylpyrrolo[2,1-f][1,2,4]triazin-4(3H)-one;
   (S)-2-(4-(3-chloro-4-(2-chloro-3-(6-methoxy-5-((((5-oxopyrrolidin-2-yl)methyl)amino)methyl)pyridin-2-yl)phenyl)pyridin-2-yl)-2-methoxybenzyl)-2,6-diazaspiro[3.4]octan-7-one;
   (R)-2-(4-(3-chloro-4-(2-chloro-3-(6-methoxy-5-((((5-oxopyrrolidin-2-yl)methyl)amino)methyl)pyridin-2-yl)phenyl)pyridin-2-yl)-2-methoxybenzyl)-2,6-diazaspiro[3.4]octan-7-one;
   2-(4-(3-chloro-4-(2-chloro-3-(6-methoxy-5-((7-oxo-2,6-diazaspiro[3.4]octan-2-yl)methyl)pyridin-2-yl)phenyl)pyridin-2-yl)-2-methoxybenzyl)-2,6-diazaspiro[3.4]octan-7-one;
   1-(6-((6-(3-(2-(4-((6-acetyl-2,6-diazaspiro[3.3]heptan-2-yl)methyl)-3-methoxyphenyl)-3-chloropyridin-4-yl)-2-chlorophenyl)-2-methoxypyridin-3-yl)methyl)-2,6-diazaspiro[3.3]heptan-2-yl)ethan-1-one;
   6-(3-chloro-4-(2-chloro-3-(6-methoxy-5-(((((S)-5-oxopyrrolidin-2-yl)methyl)amino)methyl)pyridin-2-yl)phenyl)pyridin-2-yl)-2-((((S)-2-hydroxypropyl)amino)methyl)-3-methylpyrrolo[2,1-f][1,2,4]triazin-4(3H)-one;
   6-(3-chloro-4-(2-chloro-3-(6-methoxy-5-(((((S)-5-oxopyrrolidin-2-yl)methyl)amino)methyl)pyridin-2-yl)phenyl)pyridin-2-yl)-2-((((R)-2-hydroxypropyl)amino)methyl)-3-methylpyrrolo[2,1-f][1,2,4]triazin-4(3H)-one;
   6-(3-chloro-4-(2-chloro-3-(6-methoxy-5-(((((R)-5-oxopyrrolidin-2-yl)methyl)amino)methyl)pyridin-2-yl)phenyl)pyridin-2-yl)-2-((((S)-2-hydroxypropyl)amino)methyl)-3-methylpyrrolo[2,1-f][1,2,4]triazin-4(3H)-one;
   6-(3-chloro-4-(2-chloro-3-(6-methoxy-5-(((((R)-5-oxopyrrolidin-2-yl)methyl)amino)methyl)pyridin-2-yl)phenyl)pyridin-2-yl)-2-((((R)-2-hydroxypropyl)amino)methyl)-3-methylpyrrolo[2,1-f][1,2,4]triazin-4(3H)-one;
   (S)-5-(((4-(4-(3-(3-(((1-acetylpiperidin-4-yl)(methyl)amino)methyl)-1-methyl-1H-pyrrolo[2,3-b]pyridin-6-yl)-2-chlorophenyl)-3-chloropyridin-2-yl)-2-methoxybenzyl)amino)methyl)pyrrolidin-2-one;
   (R)-5-(((4-(4-(3-(3-(((1-acetylpiperidin-4-yl)(methyl)amino)methyl)-1-methyl-1H-pyrrolo[2,3-b]pyridin-6-yl)-2-chlorophenyl)-3-chloropyridin-2-yl)-2-methoxybenzyl)amino)methyl)pyrrolidin-2-one;
   (S)-5-(((4-(3-chloro-4-(2-chloro-3-(1-methyl-3-(((((S)-tetrahydrofuran-2-yl)methyl)amino)methyl)-1H-pyrrolo[2,3-b]pyridin-6-yl)phenyl)pyridin-2-yl)-2-methoxybenzyl)amino)methyl)pyrrolidin-2-one;
   (S)-5-(((4-(3-chloro-4-(2-chloro-3-(1-methyl-3-(((((R)-tetrahydrofuran-2-yl)methyl)amino)methyl)-1H-pyrrolo[2,3-b]pyridin-6-yl)phenyl)pyridin-2-yl)-2-methoxybenzyl)amino)methyl)pyrrolidin-2-one;
   (R)-5-(((4-(3-chloro-4-(2-chloro-3-(1-methyl-3-(((((S)-tetrahydrofuran-2-yl)methyl)amino)methyl)-1H-pyrrolo[2,3-b]pyridin-6-yl)phenyl)pyridin-2-yl)-2-methoxybenzyl)amino)methyl)pyrrolidin-2-one;
   (R)-5-(((4-(3-chloro-4-(2-chloro-3-(1-methyl-3-(((((R)-tetrahydrofuran-2-yl)methyl)amino)methyl)-1H-pyrrolo[2,3-b]pyridin-6-yl)phenyl)pyridin-2-yl)-2-methoxybenzyl)amino)methyl)pyrrolidin-2-one;
   (S)-5-(((4-(3-chloro-4-(2-chloro-3-(1-methyl-3-((methylamino)methyl)-1H-pyrrolo[2,3-b]pyridin-6-yl)phenyl)pyridin-2-yl)-2-methoxybenzyl)amino)methyl)pyrrolidin-2-one;
   (R)-5-(((4-(3-chloro-4-(2-chloro-3-(1-methyl-3-((methylamino)methyl)-1H-pyrrolo[2,3-b]pyridin-6-yl)phenyl)pyridin-2-yl)-2-methoxybenzyl)amino)methyl)pyrrolidin-2-one;
   (S)-5-(((4-(4-(3-(3-(((1-acetylazetidin-3-yl)amino)methyl)-1-methyl-1H-pyrrolo[2,3-b]pyridin-6-yl)-2-chlorophenyl)-3-chloropyridin-2-yl)-2-methoxybenzyl)amino)methyl)pyrrolidin-2-one;
   (R)-5-(((4-(4-(3-(3-(((1-acetylazetidin-3-yl)amino)methyl)-1-methyl-1H-pyrrolo[2,3-b]pyridin-6-yl)-2-chlorophenyl)-3-chloropyridin-2-yl)-2-methoxybenzyl)amino)methyl)pyrrolidin-2-one;
   (S)-5-(((4-(3-chloro-4-(2-chloro-3-(3-(((4,4-difluorocyclohexyl)amino)methyl)-1-methyl-1H-pyrrolo[2,3-b]pyridin-6-yl)phenyl)pyridin-2-yl)-2-methoxybenzyl)amino)methyl)pyrrolidin-2-one;
   (R)-5-(((4-(3-chloro-4-(2-chloro-3-(3-(((4,4-difluorocyclohexyl)amino)methyl)-1-methyl-1H-pyrrolo[2,3-b]pyridin-6-yl)phenyl)pyridin-2-yl)-2-methoxybenzyl)amino)methyl)pyrrolidin-2-one;
   (S)-5-(((4-(3-chloro-4-(2-chloro-3-(3-(((2-hydroxyethyl)amino)methyl)-1-methyl-1H-pyrrolo[2,3-b]pyridin-6-yl)phenyl)pyridin-2-yl)-2-methoxybenzyl)amino)methyl)pyrrolidin-2-one;
   (R)-5-(((4-(3-chloro-4-(2-chloro-3-(3-(((2-hydroxyethyl)amino)methyl)-1-methyl-1H-pyrrolo[2,3-b]pyridin-6-yl)phenyl)pyridin-2-yl)-2-methoxybenzyl)amino)methyl)pyrrolidin-2-one;
   (S)-5-(((4-(3-chloro-4-(2-chloro-3-(3-((((S)-2-hydroxypropyl)amino)methyl)-1-methyl-1H-pyrrolo[2,3-b]pyridin-6-yl)phenyl)pyridin-2-yl)-2-methoxybenzyl)amino)methyl)pyrrolidin-2-one;
   (S)-5-(((4-(3-chloro-4-(2-chloro-3-(3-((((R)-2-hydroxypropyl)amino)methyl)-1-methyl-1H-pyrrolo[2,3-b]pyridin-6-yl)phenyl)pyridin-2-yl)-2-methoxybenzyl)amino)methyl)pyrrolidin-2-one;
   (R)-5-(((4-(3-chloro-4-(2-chloro-3-(3-((((S)-2-hydroxypropyl)amino)methyl)-1-methyl-1H-pyrrolo[2,3-b]pyridin-6-yl)phenyl)pyridin-2-yl)-2-methoxybenzyl)amino)methyl)pyrrolidin-2-one;
   (R)-5-(((4-(3-chloro-4-(2-chloro-3-(3-((((R)-2-hydroxypropyl)amino)methyl)-1-methyl-1H-pyrrolo[2,3-b]pyridin-6-yl)phenyl)pyridin-2-yl)-2-methoxybenzyl)amino)methyl)pyrrolidin-2-one;
   (S)-5-(((4-(3-chloro-4-(2-chloro-3-(3-(((2-hydroxy-2-methylpropyl)amino)methyl)-1-methyl-1H-pyrrolo[2,3-b]pyridin-6-yl)phenyl)pyridin-2-yl)-2-methoxybenzyl)amino)methyl)pyrrolidin-2-one;
   (R)-5-(((4-(3-chloro-4-(2-chloro-3-(3-(((2-hydroxy-2-methylpropyl)amino)methyl)-1-methyl-1H-pyrrolo[2,3-b]pyridin-6-yl)phenyl)pyridin-2-yl)-2-methoxybenzyl)amino)methyl)pyrrolidin-2-one;
   (S)-5-(((4-(3-chloro-4-(2-chloro-3-(3-(((2-methoxyethyl)amino)methyl)-1-methyl-1H-pyrrolo[2,3-b]pyridin-6-yl)phenyl)pyridin-2-yl)-2-methoxybenzyl)amino)methyl)pyrrolidin-2-one;
   (R)-5-(((4-(3-chloro-4-(2-chloro-3-(3-(((2-methoxyethyl)amino)methyl)-1-methyl-1H-pyrrolo[2,3-b]pyridin-6-yl)phenyl)pyridin-2-yl)-2-methoxybenzyl)amino)methyl)pyrrolidin-2-one;
   (S)-5-(((4-(3-chloro-4-(2-chloro-3-(3-(((2-hydroxyethyl)(methyl)amino)methyl)-1-methyl-1H-pyrrolo[2,3-b]pyridin-6-yl)phenyl)pyridin-2-yl)-2-methoxybenzyl)amino)methyl)pyrrolidin-2-one;
   (R)-5-(((4-(3-chloro-4-(2-chloro-3-(3-(((2-hydroxyethyl)(methyl)amino)methyl)-1-methyl-1H-pyrrolo[2,3-b]pyridin-6-yl)phenyl)pyridin-2-yl)-2-methoxybenzyl)amino)methyl)pyrrolidin-2-one;
   (S)-6-(3-chloro-4-(2-chloro-3-(6-methoxy-5-((((5-oxopyrrolidin-2-yl)methyl)amino)methyl)pyridin-2-yl)phenyl)pyridin-2-yl)-2-(((3-hydroxy-3-methylcyclobutyl)amino)methyl)-3-methylpyrrolo[2,1-f][1,2,4]triazin-4(3H)-one;
   (R)-6-(3-chloro-4-(2-chloro-3-(6-methoxy-5-((((5-oxopyrrolidin-2-yl)methyl)amino)methyl)pyridin-2-yl)phenyl)pyridin-2-yl)-2-(((3-hydroxy-3-methylcyclobutyl)amino)methyl)-3-methylpyrrolo[2,1-f][1,2,4]triazin-4(3H)-one;
   6-(3-chloro-4-(2-chloro-3-(6-methoxy-5-(((((S)-5-oxopyrrolidin-2-yl)methyl)amino)methyl)pyridin-2-yl)phenyl)pyridin-2-yl)-3-methyl-2-(((((S)-5-oxopyrrolidin-2-yl)methyl)amino)methyl)pyrrolo[2,1-f][1,2,4]triazin-4(3H)-one;
   6-(3-chloro-4-(2-chloro-3-(6-methoxy-5-(((((S)-5-oxopyrrolidin-2-yl)methyl)amino)methyl)pyridin-2-yl)phenyl)pyridin-2-yl)-3-methyl-2-(((((R)-5-oxopyrrolidin-2-yl)methyl)amino)methyl)pyrrolo[2,1-f][1,2,4]triazin-4(3H)-one;
   6-(3-chloro-4-(2-chloro-3-(6-methoxy-5-(((((R)-5-oxopyrrolidin-2-yl)methyl)amino)methyl)pyridin-2-yl)phenyl)pyridin-2-yl)-3-methyl-2-(((((S)-5-oxopyrrolidin-2-yl)methyl)amino)methyl)pyrrolo[2,1-f][1,2,4]triazin-4(3H)-one;
   6-(3-chloro-4-(2-chloro-3-(6-methoxy-5-(((((R)-5-oxopyrrolidin-2-yl)methyl)amino)methyl)pyridin-2-yl)phenyl)pyridin-2-yl)-3-methyl-2-(((((R)-5-oxopyrrolidin-2-yl)methyl)amino)methyl)pyrrolo[2,1-f][1,2,4]triazin-4(3H)-one;
   (S)-5-((((4-chloro-6-(2-chloro-3-(3-chloro-2-(2-((R)-2-hydroxypropyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
   (S)-5-((((4-chloro-6-(2-chloro-3-(3-chloro-2-(2-((S)-2-hydroxypropyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
   (R)-5-((((4-chloro-6-(2-chloro-3-(3-chloro-2-(2-((R)-2-hydroxypropyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
   (R)-5-((((4-chloro-6-(2-chloro-3-(3-chloro-2-(2-((S)-2-hydroxypropyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
   (S)-(4-(3-chloro-4-(2-chloro-3-(4-chloro-6-methoxy-5-((((5-oxopyrrolidin-2-yl)methyl)amino)methyl)pyridin-2-yl)phenyl)pyridin-2-yl)-2-methoxybenzyl)glycine;
   (R)-(4-(3-chloro-4-(2-chloro-3-(4-chloro-6-methoxy-5-((((5-oxopyrrolidin-2-yl)methyl)amino)methyl)pyridin-2-yl)phenyl)pyridin-2-yl)-2-methoxybenzyl)glycine;
   3-chloro-4-(2-chloro-3-(5-(4,5-dihydro-1H-imidazol-2-yl)-6-methoxypyridin-2-yl)phenyl)-2-(4-(4,5-dihydro-1H-imidazol-2-yl)-3-methoxyphenyl)pyridine;
   (S)-(4-(3-chloro-4-(2-chloro-3-(6-methoxy-5-((((5-oxopyrrolidin-2-yl)methyl)amino)methyl)pyridin-2-yl)phenyl)pyridin-2-yl)-2-methoxybenzyl)glycine;
   (R)-(4-(3-chloro-4-(2-chloro-3-(6-methoxy-5-((((5-oxopyrrolidin-2-yl)methyl)amino)methyl)pyridin-2-yl)phenyl)pyridin-2-yl)-2-methoxybenzyl)glycine;
   isopropyl (S)-(4-(3-chloro-4-(2-chloro-3-(6-methoxy-5-((((5-oxopyrrolidin-2-yl)methyl)amino)methyl)pyridin-2-yl)phenyl)pyridin-2-yl)-2-methoxybenzyl)glycinate;
   isopropyl (R)-(4-(3-chloro-4-(2-chloro-3-(6-methoxy-5-((((5-oxopyrrolidin-2-yl)methyl)amino)methyl)pyridin-2-yl)phenyl)pyridin-2-yl)-2-methoxybenzyl)glycinate;
   (S)-5-((((6-(3-(2-(4-(((1-acetylpiperidin-4-yl)amino)methyl)-3-methoxyphenyl)-3-chloropyridin-4-yl)-2-chlorophenyl)-2-methoxypyridin-3-yl)methyl)amino)methyl)pyrrolidin-2-one;
   (R)-5-((((6-(3-(2-(4-(((1-acetylpiperidin-4-yl)amino)methyl)-3-methoxyphenyl)-3-chloropyridin-4-yl)-2-chlorophenyl)-2-methoxypyridin-3-yl)methyl)amino)methyl)pyrrolidin-2-one;
   (S)-6-(3-chloro-4-(2-chloro-3-(6-methoxy-5-((((5-oxopyrrolidin-2-yl)methyl)amino)methyl)pyridin-2-yl)phenyl)pyridin-2-yl)-2-((3-hydroxy-3-methylazetidin-1-yl)methyl)-3-methylpyrrolo[2,1-f][1,2,4]triazin-4(3H)-one;
   (R)-6-(3-chloro-4-(2-chloro-3-(6-methoxy-5-((((5-oxopyrrolidin-2-yl)methyl)amino)methyl)pyridin-2-yl)phenyl)pyridin-2-yl)-2-((3-hydroxy-3-methylazetidin-1-yl)methyl)-3-methylpyrrolo[2,1-f][1,2,4]triazin-4(3H)-one;
   (S)-6-(3-chloro-4-(2-chloro-3-(6-methoxy-5-((((5-oxopyrrolidin-2-yl)methyl)amino)methyl)pyridin-2-yl)phenyl)pyridin-2-yl)-2-((isopropylamino)methyl)-3-methylpyrrolo[2,1-f][1,2,4]triazin-4(3H)-one;
   (R)-6-(3-chloro-4-(2-chloro-3-(6-methoxy-5-((((5-oxopyrrolidin-2-yl)methyl)amino)methyl)pyridin-2-yl)phenyl)pyridin-2-yl)-2-((isopropylamino)methyl)-3-methylpyrrolo[2,1-f][1,2,4]triazin-4(3H)-one;
   (5S)-5-((((6-(2-chloro-3-(3-chloro-2-(5-((((S)-5-oxopyrrolidin-2-yl)methyl)amino)-5,6,7,8-tetrahydronaphthalen-2-yl)pyridin-4-yl)phenyl)-2-methoxypyridin-3-yl)methyl)amino)methyl)pyrrolidin-2-one;
   (5S)-5-((((6-(2-chloro-3-(3-chloro-2-(5-((((R)-5-oxopyrrolidin-2-yl)methyl)amino)-5,6,7,8-tetrahydronaphthalen-2-yl)pyridin-4-yl)phenyl)-2-methoxypyridin-3-yl)methyl)amino)methyl)pyrrolidin-2-one;
   (5R)-5-((((6-(2-chloro-3-(3-chloro-2-(5-((((S)-5-oxopyrrolidin-2-yl)methyl)amino)-5,6,7,8-tetrahydronaphthalen-2-yl)pyridin-4-yl)phenyl)-2-methoxypyridin-3-yl)methyl)amino)methyl)pyrrolidin-2-one;
   (5R)-5-((((6-(2-chloro-3-(3-chloro-2-(5-((((R)-5-oxopyrrolidin-2-yl)methyl)amino)-5,6,7,8-tetrahydronaphthalen-2-yl)pyridin-4-yl)phenyl)-2-methoxypyridin-3-yl)methyl)amino)methyl)pyrrolidin-2-one;
   (5 S)-5-((((6-(2-chloro-3-(3-chloro-2-(5-(((S)-2-hydroxypropyl)amino)-5,6,7, 8-tetrahydronaphthalen-2-yl)pyridin-4-yl)phenyl)-2-methoxypyridin-3-yl)methyl)amino)methyl)pyrrolidin-2-one;
   (5 S)-5-((((6-(2-chloro-3-(3-chloro-2-(5-(((R)-2-hydroxypropyl)amino)-5,6,7, 8-tetrahydronaphthalen-2-yl)pyridin-4-yl)phenyl)-2-methoxypyridin-3-yl)methyl)amino)methyl)pyrrolidin-2-one;
   (5R)-5-((((6-(2-chloro-3-(3-chloro-2-(5-(((S)-2-hydroxypropyl)amino)-5,6,7,8-tetrahydronaphthalen-2-yl)pyridin-4-yl)phenyl)-2-methoxypyridin-3-yl)methyl)amino)methyl)pyrrolidin-2-one;
   (5R)-5-((((6-(2-chloro-3-(3-chloro-2-(5-(((R)-2-hydroxypropyl)amino)-5,6,7,8-tetrahydronaphthalen-2-yl)pyridin-4-yl)phenyl)-2-methoxypyridin-3-yl)methyl)amino)methyl)pyrrolidin-2-one;
   (S)-5-((((6-(2-chloro-3-(3-chloro-2-(4-((((S)-2-hydroxypropyl)amino)methyl)-3-methoxy-5-methylphenyl)pyridin-4-yl)phenyl)-2-methoxypyridin-3-yl)methyl)amino)methyl)pyrrolidin-2-one;
   (S)-5-((((6-(2-chloro-3-(3-chloro-2-(4-((((R)-2-hydroxypropyl)amino)methyl)-3-methoxy-5-methylphenyl)pyridin-4-yl)phenyl)-2-methoxypyridin-3-yl)methyl)amino)methyl)pyrrolidin-2-one;
   (R)-5-((((6-(2-chloro-3-(3-chloro-2-(4-((((S)-2-hydroxypropyl)amino)methyl)-3-methoxy-5-methylphenyl)pyridin-4-yl)phenyl)-2-methoxypyridin-3-yl)methyl)amino)methyl)pyrrolidin-2-one;
   (R)-5-((((6-(2-chloro-3-(3-chloro-2-(4-((((R)-2-hydroxypropyl)amino)methyl)-3-methoxy-5-methylphenyl)pyridin-4-yl)phenyl)-2-methoxypyridin-3-yl)methyl)amino)methyl)pyrrolidin-2-one;
   (S)-5-((((6-(2-chloro-3-(3-chloro-2-(3-methoxy-5-methyl-4-(((((S)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
   (S)-5-((((6-(2-chloro-3-(3-chloro-2-(3-methoxy-5-methyl-4-(((((R)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
   (R)-5-((((6-(2-chloro-3-(3-chloro-2-(3-methoxy-5-methyl-4-(((((S)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
   (R)-5-((((6-(2-chloro-3-(3-chloro-2-(3-methoxy-5-methyl-4-(((((R)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
   (S)-5-((((6-(2-chloro-3-(3-chloro-2-(isoindolin-5-yl)pyridin-4-yl)phenyl)-2-methoxypyridin-3-yl)methyl)amino)methyl)pyrrolidin-2-one;
   (R)-5-((((6-(2-chloro-3-(3-chloro-2-(isoindolin-5-yl)pyridin-4-yl)phenyl)-2-methoxypyridin-3-yl)methyl)amino)methyl)pyrrolidin-2-one;
   (S)-5-((((6-(2-chloro-3-(3-chloro-2-(3-methoxy-5-methyl-4-((methyl(((S)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
   (S)-5-((((6-(2-chloro-3-(3-chloro-2-(3-methoxy-5-methyl-4-((methyl(((R)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
   (R)-5-((((6-(2-chloro-3-(3-chloro-2-(3-methoxy-5-methyl-4-((methyl(((S)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
   (R)-5-((((6-(2-chloro-3-(3-chloro-2-(3-methoxy-5-methyl-4-((methyl(((R)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
   (S)-5-((((6-(2-chloro-3-(3-chloro-2-(4-((((S)-2-hydroxypropyl)(methyl)amino)methyl)-3-methoxy-5-methylphenyl)pyridin-4-yl)phenyl)-2-methoxypyridin-3-yl)methyl)amino)methyl)pyrrolidin-2-one;
   (S)-5-((((6-(2-chloro-3-(3-chloro-2-(4-((((R)-2-hydroxypropyl)(methyl)amino)methyl)-3-methoxy-5-methylphenyl)pyridin-4-yl)phenyl)-2-methoxypyridin-3-yl)methyl)amino)methyl)pyrrolidin-2-one;
   (R)-5-((((6-(2-chloro-3-(3-chloro-2-(4-((((S)-2-hydroxypropyl)(methyl)amino)methyl)-3-methoxy-5-methylphenyl)pyridin-4-yl)phenyl)-2-methoxypyridin-3-yl)methyl)amino)methyl)pyrrolidin-2-one;
   (R)-5-((((6-(2-chloro-3-(3-chloro-2-(4-((((R)-2-hydroxypropyl)(methyl)amino)methyl)-3-methoxy-5-methylphenyl)pyridin-4-yl)phenyl)-2-methoxypyridin-3-yl)methyl)amino)methyl)pyrrolidin-2-one;
   (S)-5-((((6-(3-(2-(1-(azetidin-3-yl)-1H-pyrazol-4-yl)-3-chloropyridin-4-yl)-2-chlorophenyl)-2-methoxypyridin-3-yl)methyl)amino)methyl)pyrrolidin-2-one;
   (R)-5-((((6-(3-(2-(1-(azetidin-3-yl)-1H-pyrazol-4-yl)-3-chloropyridin-4-yl)-2-chlorophenyl)-2-methoxypyridin-3-yl)methyl)amino)methyl)pyrrolidin-2-one;
   (S)-5-((((6-(3-(2-(1-(azetidin-3-ylmethyl)-1H-pyrazol-4-yl)-3-chloropyridin-4-yl)-2-chlorophenyl)-2-methoxypyridin-3-yl)methyl)amino)methyl)pyrrolidin-2-one;
   (R)-5-((((6-(3-(2-(1-(azetidin-3-ylmethyl)-1H-pyrazol-4-yl)-3-chloropyridin-4-yl)-2-chlorophenyl)-2-methoxypyridin-3-yl)methyl)amino)methyl)pyrrolidin-2-one;
   (S)-1-((4-(3-chloro-4-(2-chloro-3-(6-methoxy-5-((((5-oxopyrrolidin-2-yl)methyl)amino)methyl)pyridin-2-yl)phenyl)pyridin-2-yl)-2-methoxybenzyl)amino)cyclopropane-1-carboxylic acid;
   (R)-1-((4-(3-chloro-4-(2-chloro-3-(6-methoxy-5-((((5-oxopyrrolidin-2-yl)methyl)amino)methyl)pyridin-2-yl)phenyl)pyridin-2-yl)-2-methoxybenzyl)amino)cyclopropane-1-carboxylic acid;
   (S)-5-((((6-(2-chloro-3-(3-chloro-2-(3-methoxy-4-(((((S)-oxetan-2-yl)methyl)amino)methyl)phenyl)pyridin-4-yl)phenyl)-2-methoxypyridin-3-yl)methyl)amino)methyl)pyrrolidin-2-one;
   (S)-5-((((6-(2-chloro-3-(3-chloro-2-(3-methoxy-4-(((((R)-oxetan-2-yl)methyl)amino)methyl)phenyl)pyridin-4-yl)phenyl)-2-methoxypyridin-3-yl)methyl)amino)methyl)pyrrolidin-2-one;
   (R)-5-((((6-(2-chloro-3-(3-chloro-2-(3-methoxy-4-(((((S)-oxetan-2-yl)methyl)amino)methyl)phenyl)pyridin-4-yl)phenyl)-2-methoxypyridin-3-yl)methyl)amino)methyl)pyrrolidin-2-one;
   (R)-5-((((6-(2-chloro-3-(3-chloro-2-(3-methoxy-4-(((((R)-oxetan-2-yl)methyl)amino)methyl)phenyl)pyridin-4-yl)phenyl)-2-methoxypyridin-3-yl)methyl)amino)methyl)pyrrolidin-2-one;
   isopropyl (S)-(4-(3-chloro-4-(2-chloro-3-(4-chloro-6-methoxy-5-((((5-oxopyrrolidin-2-yl)methyl)amino)methyl)pyridin-2-yl)phenyl)pyridin-2-yl)-2-methoxybenzyl)glycinate;
   isopropyl (R)-(4-(3-chloro-4-(2-chloro-3-(4-chloro-6-methoxy-5-((((5-oxopyrrolidin-2-yl)methyl)amino)methyl)pyridin-2-yl)phenyl)pyridin-2-yl)-2-methoxybenzyl)glycinate;
   (S)-5-((((4-chloro-6-(2-chloro-3-(3-chloro-2-(2-(2-hydroxyethyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
   (R)-5-((((4-chloro-6-(2-chloro-3-(3-chloro-2-(2-(2-hydroxyethyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
   isopropyl (4-(3-chloro-4-(2-chloro-3-(6-methoxy-5-(((((S)-5-oxopyrrolidin-2-yl)methyl)amino)methyl)pyridin-2-yl)phenyl)pyridin-2-yl)-2-methoxybenzyl)-L-alaninate;
   isopropyl (4-(3-chloro-4-(2-chloro-3-(6-methoxy-5-(((((S)-5-oxopyrrolidin-2-yl)methyl)amino)methyl)pyridin-2-yl)phenyl)pyridin-2-yl)-2-methoxybenzyl)-D-alaninate;
   isopropyl (4-(3-chloro-4-(2-chloro-3-(6-methoxy-5-(((((R)-5-oxopyrrolidin-2-yl)methyl)amino)methyl)pyridin-2-yl)phenyl)pyridin-2-yl)-2-methoxybenzyl)-L-alaninate;
   isopropyl (4-(3-chloro-4-(2-chloro-3-(6-methoxy-5-(((((R)-5-oxopyrrolidin-2-yl)methyl)amino)methyl)pyridin-2-yl)phenyl)pyridin-2-yl)-2-methoxybenzyl)-D-alaninate;
   (S)-5-((((6-(2-chloro-3-(3-chloro-2-(4-(((1,1-dioxidotetrahydro-2H-thiopyran-4-yl)amino)methyl)-3-methoxyphenyl)pyridin-4-yl)phenyl)-2-methoxypyridin-3-yl)methyl)amino)methyl)pyrrolidin-2-one;
   (R)-5-((((6-(2-chloro-3-(3-chloro-2-(4-(((1,1-dioxidotetrahydro-2H-thiopyran-4-yl)amino)methyl)-3-methoxyphenyl)pyridin-4-yl)phenyl)-2-methoxypyridin-3-yl)methyl)amino)methyl)pyrrolidin-2-one;
   (S)-5-((((6-(2-chloro-3-(3-chloro-2-(3-methoxy-4-(((tetrahydro-2H-pyran-4-yl)amino)methyl)phenyl)pyridin-4-yl)phenyl)-2-methoxypyridin-3-yl)methyl)amino)methyl)pyrrolidin-2-one;
   (R)-5-((((6-(2-chloro-3-(3-chloro-2-(3-methoxy-4-(((tetrahydro-2H-pyran-4-yl)amino)methyl)phenyl)pyridin-4-yl)phenyl)-2-methoxypyridin-3-yl)methyl)amino)methyl)pyrrolidin-2-one;
   (4-(3-chloro-4-(2-chloro-3-(6-methoxy-5-(((((S)-5-oxopyrrolidin-2-yl)methyl)amino)methyl)pyridin-2-yl)phenyl)pyridin-2-yl)-2-methoxybenzyl)-L-alanine;
   (4-(3-chloro-4-(2-chloro-3-(6-methoxy-5-(((((S)-5-oxopyrrolidin-2-yl)methyl)amino)methyl)pyridin-2-yl)phenyl)pyridin-2-yl)-2-methoxybenzyl)-D-alanine;
   (4-(3-chloro-4-(2-chloro-3-(6-methoxy-5-(((((R)-5-oxopyrrolidin-2-yl)methyl)amino)methyl)pyridin-2-yl)phenyl)pyridin-2-yl)-2-methoxybenzyl)-L-alanine;
   (4-(3-chloro-4-(2-chloro-3-(6-methoxy-5-(((((R)-5-oxopyrrolidin-2-yl)methyl)amino)methyl)pyridin-2-yl)phenyl)pyridin-2-yl)-2-methoxybenzyl)-D-alanine;
   (S)-5-((((6-(2-chloro-3-(3-chloro-2-(2-((R)-2-hydroxypropyl)-8-methoxy-1,2,3,4-tetrahydroisoquinolin-6-yl)pyridin-4-yl)phenyl)-4-fluoro-2-methoxypyridin-3-yl)methyl)amino)methyl)pyrrolidin-2-one;
   (S)-5-((((6-(2-chloro-3-(3-chloro-2-(2-((S)-2-hydroxypropyl)-8-methoxy-1,2,3,4-tetrahydroisoquinolin-6-yl)pyridin-4-yl)phenyl)-4-fluoro-2-methoxypyridin-3-yl)methyl)amino)methyl)pyrrolidin-2-one;
   (R)-5-((((6-(2-chloro-3-(3-chloro-2-(2-((R)-2-hydroxypropyl)-8-methoxy-1,2,3,4-tetrahydroisoquinolin-6-yl)pyridin-4-yl)phenyl)-4-fluoro-2-methoxypyridin-3-yl)methyl)amino)methyl)pyrrolidin-2-one;
   (R)-5-((((6-(2-chloro-3-(3-chloro-2-(2-((S)-2-hydroxypropyl)-8-methoxy-1,2,3,4-tetrahydroisoquinolin-6-yl)pyridin-4-yl)phenyl)-4-fluoro-2-methoxypyridin-3-yl)methyl)amino)methyl)pyrrolidin-2-one;
   (S)-5-((((6-(2-chloro-3-(3-chloro-2-(2-(2-hydroxyethyl)-8-methoxy-1,2,3,4-tetrahydroisoquinolin-6-yl)pyridin-4-yl)phenyl)-4-fluoro-2-methoxypyridin-3-yl)methyl)amino)methyl)pyrrolidin-2-one;
   (S)-5-((((6-(2-chloro-3-(3-chloro-2-(2-(2-hydroxyethyl)-8-methoxy-1,2,3,4-tetrahydroisoquinolin-6-yl)pyridin-4-yl)phenyl)-4-fluoro-2-methoxypyridin-3-yl)methyl)amino)methyl)pyrrolidin-2-one;
   (S)-5-((((6-(2-chloro-3-(3-chloro-2-(2-(((1r,3r)-3-hydroxycyclobutyl)methyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
   (S)-5-((((6-(2-chloro-3-(3-chloro-2-(2-(((1s,3s)-3-hydroxycyclobutyl)methyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
   (S)-5-((((6-(2-chloro-3-(3-chloro-2-(2-(((1s,3r)-3-hydroxycyclobutyl)methyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
   (R)-5-((((6-(2-chloro-3-(3-chloro-2-(2-(((1s,3s)-3-hydroxycyclobutyl)methyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
   (S)-5-((((6-(2-chloro-3-(3-chloro-2-(2-(((1r,3r)-3-hydroxycyclobutyl)methyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
   (R)-5-((((6-(2-chloro-3-(3-chloro-2-(2-(((1s,3r)-3-hydroxycyclobutyl)methyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
   (R)-5-((((6-(2-chloro-3-(3-chloro-2-(2-(((1r,3s)-3-hydroxycyclobutyl)methyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
   (R)-5-((((6-(2-chloro-3-(3-chloro-2-(2-(((1r,3r)-3-hydroxycyclobutyl)methyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
   isopropyl (S)-1-((4-(3-chloro-4-(2-chloro-3-(6-methoxy-5-((((5-oxopyrrolidin-2-yl)methyl)amino)methyl)pyridin-2-yl)phenyl)pyridin-2-yl)-2-methoxybenzyl)amino)cyclopropane-1-carboxylate;
   isopropyl (R)-1-((4-(3-chloro-4-(2-chloro-3-(6-methoxy-5-((((5-oxopyrrolidin-2-yl)methyl)amino)methyl)pyridin-2-yl)phenyl)pyridin-2-yl)-2-methoxybenzyl)amino)cyclopropane-1-carboxylate;
   1-(6-(2-chloro-3-(3-chloro-2-(3-methoxy-4-((methylamino)methyl)phenyl)pyridin-4-yl)phenyl)-2-methoxypyridin-3-yl)-N-methylmethanamine;
   1-(6-(2-chloro-3-(3-chloro-2-(4-((dimethylamino)methyl)-3-methoxyphenyl)pyridin-4-yl)phenyl)-2-methoxypyridin-3-yl)-N,N-dimethylmethanamine;
   2-(((6-(2-chloro-3-(3-chloro-2-(4-(((2-hydroxyethyl)(methyl)amino)methyl)-3-methoxyphenyl)pyridin-4-yl)phenyl)-2-methoxypyridin-3-yl)methyl)(methyl)amino)ethan-1-ol;
   1-(4-(((6-(3-(2-(4-(((1-acetylpiperidin-4-yl)amino)methyl)-3-methoxyphenyl)-3-chloropyridin-4-yl)-2-chlorophenyl)-2-methoxypyridin-3-yl)methyl)amino)piperidin-1-yl)ethan-1-one;
   1-(4-(((6-(3-(2-(4-(((1-acetylpiperidin-4-yl)(methyl)amino)methyl)-3-methoxyphenyl)-3-chloropyridin-4-yl)-2-chlorophenyl)-2-methoxypyridin-3-yl)methyl)(methyl)amino)piperidin-1-yl)ethan-1-one;
   (S)-5-((((6-(2-chloro-3-(3-chloro-2-(5-methyl-2,3,4,5-tetrahydro-1H-pyrido[4,3-b]indol-7-yl)pyridin-4-yl)phenyl)-2-methoxypyridin-3-yl)methyl)amino)methyl)pyrrolidin-2-one;
   (S)-5-((((6-(2-chloro-3-(3-chloro-2-(5-methyl-2,3,4,5-tetrahydro-1H-pyrido[4,3-b]indol-7-yl)pyridin-4-yl)phenyl)-2-methoxypyridin-3-yl)methyl)amino)methyl)pyrrolidin-2-one;
   5-(3-chloro-4-(2-chloro-3-(5-((((S)-2-hydroxypropyl)amino)methyl)-6-methoxypyridin-2-yl)phenyl)pyridin-2-yl)-2-(2-((((S)-5-oxopyrrolidin-2-yl)methyl)amino)ethyl)isoindolin-1-one;
   5-(3-chloro-4-(2-chloro-3-(5-((((S)-2-hydroxypropyl)amino)methyl)-6-methoxypyridin-2-yl)phenyl)pyridin-2-yl)-2-(2-((((R)-5-oxopyrrolidin-2-yl)methyl)amino)ethyl)isoindolin-1-one;
   5-(3-chloro-4-(2-chloro-3-(5-((((R)-2-hydroxypropyl)amino)methyl)-6-methoxypyridin-2-yl)phenyl)pyridin-2-yl)-2-(2-((((S)-5-oxopyrrolidin-2-yl)methyl)amino)ethyl)isoindolin-1-one;
   5-(3-chloro-4-(2-chloro-3-(5-((((R)-2-hydroxypropyl)amino)methyl)-6-methoxypyridin-2-yl)phenyl)pyridin-2-yl)-2-(2-((((R)-5-oxopyrrolidin-2-yl)methyl)amino)ethyl)isoindolin-1-one;
   (S)-5-(4-(3-(5-(((1-acetylpiperidin-4-yl)amino)methyl)-6-methoxypyridin-2-yl)-2-chlorophenyl)-3-chloropyridin-2-yl)-2-(2-(((5-oxopyrrolidin-2-yl)methyl)amino)ethyl)isoindolin-1-one;
   (R)-5-(4-(3-(5-(((1-acetylpiperidin-4-yl)amino)methyl)-6-methoxypyridin-2-yl)-2-chlorophenyl)-3-chloropyridin-2-yl)-2-(2-(((5-oxopyrrolidin-2-yl)methyl)amino)ethyl)isoindolin-1-one;
   (S)-5-(4-(3-(5-((6-acetyl-2,6-diazaspiro[3.3]heptan-2-yl)methyl)-6-methoxypyridin-2-yl)-2-chlorophenyl)-3-chloropyridin-2-yl)-2-(2-(((5-oxopyrrolidin-2-yl)methyl)amino)ethyl)isoindolin-1-one;
   (R)-5-(4-(3-(5-((6-acetyl-2,6-diazaspiro[3.3]heptan-2-yl)methyl)-6-methoxypyridin-2-yl)-2-chlorophenyl)-3-chloropyridin-2-yl)-2-(2-(((5-oxopyrrolidin-2-yl)methyl)amino)ethyl)isoindolin-1-one;
   (S)-2-(4-(3-chloro-4-(2-chloro-3-(6-methoxy-5-((((5-oxopyrrolidin-2-yl)methyl)amino)methyl)pyridin-2-yl)phenyl)pyridin-2-yl)-2-fluoro-6-methoxybenzyl)-2,6-diazaspiro[3.4]octan-7-one;
   (R)-2-(4-(3-chloro-4-(2-chloro-3-(6-methoxy-5-((((5-oxopyrrolidin-2-yl)methyl)amino)methyl)pyridin-2-yl)phenyl)pyridin-2-yl)-2-fluoro-6-methoxybenzyl)-2,6-diazaspiro[3.4]octan-7-one;
   (S)-5-((((6-(2-chloro-3-(3-chloro-2-(3-fluoro-4-((6-hydroxy-2-azaspiro[3.3]heptan-2-yl)methyl)-5-methoxyphenyl)pyridin-4-yl)phenyl)-2-methoxypyridin-3-yl)methyl)amino)methyl)pyrrolidin-2-one;
   (R)-5-((((6-(2-chloro-3-(3-chloro-2-(3-fluoro-4-((6-hydroxy-2-azaspiro[3.3]heptan-2-yl)methyl)-5-methoxyphenyl)pyridin-4-yl)phenyl)-2-methoxypyridin-3-yl)methyl)amino)methyl)pyrrolidin-2-one;
   (S)-5-((((6-(2-chloro-3-(3-chloro-2-(3-fluoro-4-(((3-fluoropropyl)amino)methyl)-5-methoxyphenyl)pyridin-4-yl)phenyl)-2-methoxypyridin-3-yl)methyl)amino)methyl)pyrrolidin-2-one;
   (R)-5-((((6-(2-chloro-3-(3-chloro-2-(3-fluoro-4-(((3-fluoropropyl)amino)methyl)-5-methoxyphenyl)pyridin-4-yl)phenyl)-2-methoxypyridin-3-yl)methyl)amino)methyl)pyrrolidin-2-one;
   (S)-5-((((6-(3-(2-(4-(((1-acetylpiperidin-4-yl)amino)methyl)-3-fluoro-5-methoxyphenyl)-3-chloropyridin-4-yl)-2-chlorophenyl)-2-methoxypyridin-3-yl)methyl)amino)methyl)pyrrolidin-2-one;
   (R)-5-((((6-(3-(2-(4-(((1-acetylpiperidin-4-yl)amino)methyl)-3-fluoro-5-methoxyphenyl)-3-chloropyridin-4-yl)-2-chlorophenyl)-2-methoxypyridin-3-yl)methyl)amino)methyl)pyrrolidin-2-one;
   methyl (S)-2-(6-(3-chloro-4-(2-chloro-3-(6-methoxy-5-((((5-oxopyrrolidin-2-yl)methyl)amino)methyl)pyridin-2-yl)phenyl)pyridin-2-yl)-8-methoxy-3,4-dihydroisoquinolin-2(1H)-yl)acetate;
   methyl (R)-2-(6-(3-chloro-4-(2-chloro-3-(6-methoxy-5-((((5-oxopyrrolidin-2-yl)methyl)amino)methyl)pyridin-2-yl)phenyl)pyridin-2-yl)-8-methoxy-3,4-dihydroisoquinolin-2(1H)-yl)acetate;
   (S)-2-(6-(3-chloro-4-(2-chloro-3-(6-methoxy-5-((((5-oxopyrrolidin-2-yl)methyl)amino)methyl)pyridin-2-yl)phenyl)pyridin-2-yl)-8-methoxy-3,4-dihydroisoquinolin-2(1H)-yl)acetic acid;
   (R)-2-(6-(3-chloro-4-(2-chloro-3-(6-methoxy-5-((((5-oxopyrrolidin-2-yl)methyl)amino)methyl)pyridin-2-yl)phenyl)pyridin-2-yl)-8-methoxy-3,4-dihydroisoquinolin-2(1H)-yl)acetic acid;
   (S)-5-((((6-(3-(2-(2-((1R,3S,4S)-2-azabicyclo[2.2.1]heptan-3-yl)-1H-benzo[d]imidazol-6-yl)-3-chloropyridin-4-yl)-2-chlorophenyl)-2-methoxypyridin-3-yl)methyl)amino)methyl)pyrrolidin-2-one;
   (R)-5-((((6-(3-(2-(2-((1R,3S,4S)-2-azabicyclo[2.2.1]heptan-3-yl)-1H-benzo[d]imidazol-6-yl)-3-chloropyridin-4-yl)-2-chlorophenyl)-2-methoxypyridin-3-yl)methyl)amino)methyl)pyrrolidin-2-one;
   (S)-5-((((6-(3-(2-(2-((1R,3R,4S)-2-azabicyclo[2.2.1]heptan-3-yl)-1H-benzo[d]imidazol-6-yl)-3-chloropyridin-4-yl)-2-chlorophenyl)-2-methoxypyridin-3-yl)methyl)amino)methyl)pyrrolidin-2-one;
   (R)-5-((((6-(3-(2-(2-((1R,3R,4S)-2-azabicyclo[2.2.1]heptan-3-yl)-1H-benzo[d]imidazol-6-yl)-3-chloropyridin-4-yl)-2-chlorophenyl)-2-methoxypyridin-3-yl)methyl)amino)methyl)pyrrolidin-2-one;
   (S)-5-((((6-(3-(2-(2-((1S,3S,4R)-2-azabicyclo[2.2.1]heptan-3-yl)-1H-benzo[d]imidazol-6-yl)-3-chloropyridin-4-yl)-2-chlorophenyl)-2-methoxypyridin-3-yl)methyl)amino)methyl)pyrrolidin-2-one;
   (R)-5-((((6-(3-(2-(2-((1S,3S,4R)-2-azabicyclo[2.2.1]heptan-3-yl)-1H-benzo[d]imidazol-6-yl)-3-chloropyridin-4-yl)-2-chlorophenyl)-2-methoxypyridin-3-yl)methyl)amino)methyl)pyrrolidin-2-one;
   (S)-5-((((6-(3-(2-(2-((1S,3R,4R)-2-azabicyclo[2.2.1]heptan-3-yl)-1H-benzo[d]imidazol-6-yl)-3-chloropyridin-4-yl)-2-chlorophenyl)-2-methoxypyridin-3-yl)methyl)amino)methyl)pyrrolidin-2-one;
   (R)-5-((((6-(3-(2-(2-((1S,3R,4R)-2-azabicyclo[2.2.1]heptan-3-yl)-1H-benzo[d]imidazol-6-yl)-3-chloropyridin-4-yl)-2-chlorophenyl)-2-methoxypyridin-3-yl)methyl)amino)methyl)pyrrolidin-2-one;
   (S)-5-((((6-(2-chloro-3-(3-chloro-2-(3-methoxy-5-(((((S)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
   (S)-5-((((6-(2-chloro-3-(3-chloro-2-(3-methoxy-5-(((((R)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
   (R)-5-((((6-(2-chloro-3-(3-chloro-2-(3-methoxy-5-(((((S)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
   (R)-5-((((6-(2-chloro-3-(3-chloro-2-(3-methoxy-5-(((((R)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
   1-(4-(((6-(3-(2-(3-(((1-acetylpiperidin-4-yl)amino)methyl)-5-methoxyphenyl)-3-chloropyridin-4-yl)-2-chlorophenyl)-2-methoxypyridin-3-yl)methyl)amino)piperidin-1-yl)ethan-1-one;
   1-(6-(2-chloro-3-(3-chloro-2-(3-methoxy-5-((methylamino)methyl)phenyl)pyridin-4-yl)phenyl)-2-methoxypyridin-3-yl)-N-methylmethanamine;
   chloro-3-(3-chloro-2-(3-(((2-hydroxyethyl)amino)methyl)-5-methoxyphenyl)pyridin-4-yl)phenyl)-2-methoxypyridin-3-yl)methyl)amino)ethan-1-ol;
   (S)-1-(((6-(2-chloro-3-(3-chloro-2-(3-((((S)-2-hydroxypropyl)amino)methyl)-5-methoxyphenyl)pyridin-4-yl)phenyl)-2-methoxypyridin-3-yl)methyl)amino)propan-2-ol;
   (S)-1-(((6-(2-chloro-3-(3-chloro-2-(3-((((R)-2-hydroxypropyl)amino)methyl)-5-methoxyphenyl)pyridin-4-yl)phenyl)-2-methoxypyridin-3-yl)methyl)amino)propan-2-ol;
   (R)-1-(((6-(2-chloro-3-(3-chloro-2-(3-((((S)-2-hydroxypropyl)amino)methyl)-5-methoxyphenyl)pyridin-4-yl)phenyl)-2-methoxypyridin-3-yl)methyl)amino)propan-2-ol;
   (R)-1-(((6-(2-chloro-3-(3-chloro-2-(3-((((R)-2-hydroxypropyl)amino)methyl)-5-methoxyphenyl)pyridin-4-yl)phenyl)-2-methoxypyridin-3-yl)methyl)amino)propan-2-ol;
   (S)-5-((((3'-chloro-2'-((S)-1-((5-((((S)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)methyl)pyrrolidin-2-one;
   (S)-5-((((3'-chloro-2'-((S)-1-((5-((((R)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)methyl)pyrrolidin-2-one;
   (S)-5-((((3'-chloro-2'-((R)-1-((5-((((S)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)methyl)pyrrolidin-2-one;
   (R)-5-((((3'-chloro-2'-((S)-1-((5-((((S)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)methyl)pyrrolidin-2-one;
   (S)-5-((((3'-chloro-2'-((R)-1-((5-((((R)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)methyl)pyrrolidin-2-one;
   (R)-5-((((3'-chloro-2'-((S)-1-((5-((((R)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)methyl)pyrrolidin-2-one;
   (R)-5-((((3'-chloro-2'-((R)-1-((5-((((S)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)methyl)pyrrolidin-2-one;
   (R)-5-((((3'-chloro-2'-((R)-1-((5-((((R)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)methyl)pyrrolidin-2-one;
   1-(((6-(((S)-4-(3'-chloro-6-methoxy-5-(((((S)-5-oxopyrrolidin-2-yl)methyl)amino)methyl)-[2,4'-bipyridin]-2'-yl)-2,3-dihydro-1H-inden-1-yl)oxy)-2-methoxy-5-(trifluoromethyl)pyridin-3-yl)methyl)amino)cyclopropane-1-carboxylic acid;
   1-(((6-(((S)-4-(3'-chloro-6-methoxy-5-(((((R)-5-oxopyrrolidin-2-yl)methyl)amino)methyl)-[2,4'-bipyridin]-2'-yl)-2,3-dihydro-1H-inden-1-yl)oxy)-2-methoxy-5-(trifluoromethyl)pyridin-3-yl)methyl)amino)cyclopropane-1-carboxylic acid;
   1-(((6-(((R)-4-(3'-chloro-6-methoxy-5-(((((S)-5-oxopyrrolidin-2-yl)methyl)amino)methyl)-[2,4'-bipyridin]-2'-yl)-2,3-dihydro-1H-inden-1-yl)oxy)-2-methoxy-5-(trifluoromethyl)pyridin-3-yl)methyl)amino)cyclopropane-1-carboxylic acid;
   1-(((6-(((R)-4-(3'-chloro-6-methoxy-5-(((((R)-5-oxopyrrolidin-2-yl)methyl)amino)methyl)-[2,4'-bipyridin]-2'-yl)-2,3-dihydro-1H-inden-1-yl)oxy)-2-methoxy-5-(trifluoromethyl)pyridin-3-yl)methyl)amino)cyclopropane-1-carboxylic acid;
   methyl 1-(((6-(((S)-4-(3'-chloro-6-methoxy-5-(((((S)-5-oxopyrrolidin-2-yl)methyl)amino)methyl)-[2,4'-bipyridin]-2'-yl)-2,3-dihydro-1H-inden-1-yl)amino)-2-methoxy-5-(trifluoromethyl)pyridin-3-yl)methyl)amino)cyclopropane-1-carboxylate;
   methyl 1-(((6-(((S)-4-(3'-chloro-6-methoxy-5-(((((R)-5-oxopyrrolidin-2-yl)methyl)amino)methyl)-[2,4'-bipyridin]-2'-yl)-2,3-dihydro-1H-inden-1-yl)amino)-2-methoxy-5-(trifluoromethyl)pyridin-3-yl)methyl)amino)cyclopropane-1-carboxylate;
   methyl 1-(((6-(((R)-4-(3'-chloro-6-methoxy-5-(((((S)-5-oxopyrrolidin-2-yl)methyl)amino)methyl)-[2,4'-bipyridin]-2'-yl)-2,3-dihydro-1H-inden-1-yl)amino)-2-methoxy-5-(trifluoromethyl)pyridin-3-yl)methyl)amino)cyclopropane-1-carboxylate;
   methyl 1-(((6-(((R)-4-(3'-chloro-6-methoxy-5-(((((R)-5-oxopyrrolidin-2-yl)methyl)amino)methyl)-[2,4'-bipyridin]-2'-yl)-2,3-dihydro-1H-inden-1-yl)amino)-2-methoxy-5-(trifluoromethyl)pyridin-3-yl)methyl)amino)cyclopropane-1-carboxylate;
   (S)-5-((((6-(2-chloro-3-(3-chloro-2-(2-(2-hydroxyethyl)-5-methyl-2,3,4,5-tetrahydro-1H-pyrido[4,3-b]indol-7-yl)pyridin-4-yl)phenyl)-2-methoxypyridin-3-yl)methyl)amino)methyl)pyrrolidin-2-one;
   (R)-5-((((6-(2-chloro-3-(3-chloro-2-(2-(2-hydroxyethyl)-5-methyl-2,3,4,5-tetrahydro-1H-pyrido[4,3-b]indol-7-yl)pyridin-4-yl)phenyl)-2-methoxypyridin-3-yl)methyl)amino)methyl)pyrrolidin-2-one;
   1-(6-(2-chloro-3-(3-chloro-2-(isoindolin-5-yl)pyridin-4-yl)phenyl)-2-methoxypyridin-3-yl)-N-methylmethanamine;
   2-(((6-(2-chloro-3-(3-chloro-2-(isoindolin-5-yl)pyridin-4-yl)phenyl)-2-methoxypyridin-3-yl)methyl)amino)ethan-1-ol;
   (S)-1-(((6-(2-chloro-3-(3-chloro-2-(isoindolin-5-yl)pyridin-4-yl)phenyl)-2-methoxypyridin-3-yl)methyl)amino)propan-2-ol;
   (R)-1-(((6-(2-chloro-3-(3-chloro-2-(isoindolin-5-yl)pyridin-4-yl)phenyl)-2-methoxypyridin-3-yl)methyl)amino)propan-2-ol;
   1-(4-(((6-(2-chloro-3-(3-chloro-2-(isoindolin-5-yl)pyridin-4-yl)phenyl)-2-methoxypyridin-3-yl)methyl)amino)piperidin-1-yl)ethan-1-one;
   1-(4-(((6-(2-chloro-3-(3-chloro-2-(isoindolin-5-yl)pyridin-4-yl)phenyl)-2-methoxypyridin-3-yl)methyl)(methyl)amino)piperidin-1-yl)ethan-1-one;
   1-(((6-(((S)-4-(3'-chloro-6-methoxy-5-(((((S)-5-oxopyrrolidin-2-yl)methyl)amino)methyl)-[2,4'-bipyridin]-2'-yl)-2,3-dihydro-1H-inden-1-yl)amino)-2-methoxy-5-(trifluoromethyl)pyridin-3-yl)methyl)amino)cyclopropane-1-carboxylic acid;
   1-(((6-(((S)-4-(3'-chloro-6-methoxy-5-(((((R)-5-oxopyrrolidin-2-yl)methyl)amino)methyl)-[2,4'-bipyridin]-2'-yl)-2,3-dihydro-1H-inden-1-yl)amino)-2-methoxy-5-(trifluoromethyl)pyridin-3-yl)methyl)amino)cyclopropane-1-carboxylic acid;
   1-(((6-(((R)-4-(3'-chloro-6-methoxy-5-(((((S)-5-oxopyrrolidin-2-yl)methyl)amino)methyl)-[2,4'-bipyridin]-2'-yl)-2,3-dihydro-1H-inden-1-yl)amino)-2-methoxy-5-(trifluoromethyl)pyridin-3-yl)methyl)amino)cyclopropane-1-carboxylic acid;
   1-(((6-(((R)-4-(3'-chloro-6-methoxy-5-(((((R)-5-oxopyrrolidin-2-yl)methyl)amino)methyl)-[2,4'-bipyridin]-2'-yl)-2,3-dihydro-1H-inden-1-yl)amino)-2-methoxy-5-(trifluoromethyl)pyridin-3-yl)methyl)amino)cyclopropane-1-carboxylic acid;
   (S)-5-((((3'-chloro-2'-((S)-1-((5-((((R)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)methyl)pyrrolidin-2-one;
   (S)-5-((((3'-chloro-2'-((S)-1-((5-((((S)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)methyl)pyrrolidin-2-one;
   (S)-5-((((3'-chloro-2'-((R)-1-((5-((((S)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)methyl)pyrrolidin-2-one;
   (S)-5-((((3'-chloro-2'-((R)-1-((5-((((R)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)methyl)pyrrolidin-2-one;
   (R)-5-((((3'-chloro-2'-((R)-1-((5-((((R)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)methyl)pyrrolidin-2-one;
   (R)-5-((((3'-chloro-2'-((S)-1-((5-((((S)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)methyl)pyrrolidin-2-one;
   (R)-5-((((3'-chloro-2'-((S)-1-((5-((((R)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)methyl)pyrrolidin-2-one;
   (R)-5-((((3'-chloro-2'-((R)-1-((5-((((S)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)methyl)pyrrolidin-2-one;
   (S)-5-((((3'-chloro-6-methoxy-2'-((S)-1-((6-methoxy-5-(((((S)-5-oxopyrrolidin-2-yl)methyl)amino)methyl)-3-(trifluoromethyl)pyridin-2-yl)oxy)-2,3-dihydro-1H-inden-4-yl)-[2,4'-bipyridin]-5-yl)methyl)amino)methyl)pyrrolidin-2-one;
   (S)-5-((((3'-chloro-6-methoxy-2'-((S)-1-((6-methoxy-5-(((((R)-5-oxopyrrolidin-2-yl)methyl)amino)methyl)-3-(trifluoromethyl)pyridin-2-yl)oxy)-2,3-dihydro-1H-inden-4-yl)-[2,4'-bipyridin]-5-yl)methyl)amino)methyl)pyrrolidin-2-one;
   (S)-5-((((3'-chloro-6-methoxy-2'-((R)-1-((6-methoxy-5-(((((R)-5-oxopyrrolidin-2-yl)methyl)amino)methyl)-3-(trifluoromethyl)pyridin-2-yl)oxy)-2,3-dihydro-1H-inden-4-yl)-[2,4'-bipyridin]-5-yl)methyl)amino)methyl)pyrrolidin-2-one;
   (S)-5-((((3'-chloro-6-methoxy-2'-((R)-1-((6-methoxy-5-(((((R)-5-oxopyrrolidin-2-yl)methyl)amino)methyl)-3-(trifluoromethyl)pyridin-2-yl)oxy)-2,3-dihydro-1H-inden-4-yl)-[2,4'-bipyridin]-5-yl)methyl)amino)methyl)pyrrolidin-2-one;
   (R)-5-((((3'-chloro-6-methoxy-2'-((R)-1-((6-methoxy-5-(((((R)-5-oxopyrrolidin-2-yl)methyl)amino)methyl)-3-(trifluoromethyl)pyridin-2-yl)oxy)-2,3-dihydro-1H-inden-4-yl)-[2,4'-bipyridin]-5-yl)methyl)amino)methyl)pyrrolidin-2-one;
   (R)-5-((((3'-chloro-6-methoxy-2'-((S)-1-((6-methoxy-5-(((((S)-5-oxopyrrolidin-2-yl)methyl)amino)methyl)-3-(trifluoromethyl)pyridin-2-yl)oxy)-2,3-dihydro-1H-inden-4-yl)-[2,4'-bipyridin]-5-yl)methyl)amino)methyl)pyrrolidin-2-one;
   (R)-5-((((3'-chloro-6-methoxy-2'-((S)-1-((6-methoxy-5-(((((R)-5-oxopyrrolidin-2-yl)methyl)amino)methyl)-3-(trifluoromethyl)pyridin-2-yl)oxy)-2,3-dihydro-1H-inden-4-yl)-[2,4'-bipyridin]-5-yl)methyl)amino)methyl)pyrrolidin-2-one;
   (R)-5-((((3'-chloro-6-methoxy-2'-((R)-1-((6-methoxy-5-(((((S)-5-oxopyrrolidin-2-yl)methyl)amino)methyl)-3-(trifluoromethyl)pyridin-2-yl)oxy)-2,3-dihydro-1H-inden-4-yl)-[2,4'-bipyridin]-5-yl)methyl)amino)methyl)pyrrolidin-2-one;
   (S)-5-((((6-(3-(2-(3-(((1-acetylpiperidin-4-yl)amino)methyl)-1-methyl-1H-indazol-6-yl)-3-chloropyridin-4-yl)-2-chlorophenyl)-2-methoxypyridin-3-yl)methyl)amino)methyl)pyrrolidin-2-one;
   (R)-5-((((6-(3-(2-(3-(((1-acetylpiperidin-4-yl)amino)methyl)-1-methyl-1H-indazol-6-yl)-3-chloropyridin-4-yl)-2-chlorophenyl)-2-methoxypyridin-3-yl)methyl)amino)methyl)pyrrolidin-2-one;
   (S)-5-((((6-(3-(2-(3-(((1-acetylpiperidin-4-yl)(methyl)amino)methyl)-1-methyl-1H-indazol-6-yl)-3-chloropyridin-4-yl)-2-chlorophenyl)-2-methoxypyridin-3-yl)methyl)amino)methyl)pyrrolidin-2-one;
   (R)-5-((((6-(3-(2-(3-(((1-acetylpiperidin-4-yl)(methyl)amino)methyl)-1-methyl-1H-indazol-6-yl)-3-chloropyridin-4-yl)-2-chlorophenyl)-2-methoxypyridin-3-yl)methyl)amino)methyl)pyrrolidin-2-one;
   (S)-4-((3-(3-chloro-4-(2-chloro-3-(6-methoxy-5-((methylamino)methyl)pyridin-2-yl)phenyl)pyridin-2-yl)-5-methoxybenzyl)amino)-3-hydroxybutanoic acid;
   (R)-4-((3-(3-chloro-4-(2-chloro-3-(6-methoxy-5-((methylamino)methyl)pyridin-2-yl)phenyl)pyridin-2-yl)-5-methoxybenzyl)amino)-3-hydroxybutanoic acid;
   3-((3-(3-chloro-4-(2-chloro-3-(6-methoxy-5-((methylamino)methyl)pyridin-2-yl)phenyl)pyridin-2-yl)-5-methoxybenzyl)amino)propanoic acid;
   (S)-1-(3-(3-chloro-4-(2-chloro-3-(6-methoxy-5-((methylamino)methyl)pyridin-2-yl)phenyl)pyridin-2-yl)-5-methoxybenzyl)pyrrolidine-3-carboxylic acid;
   (R)-1-(3-(3-chloro-4-(2-chloro-3-(6-methoxy-5-((methylamino)methyl)pyridin-2-yl)phenyl)pyridin-2-yl)-5-methoxybenzyl)pyrrolidine-3-carboxylic acid;
   2-(1-(3-(3-chloro-4-(2-chloro-3-(6-methoxy-5-((methylamino)methyl)pyridin-2-yl)phenyl)pyridin-2-yl)-5-methoxybenzyl)azetidin-3-yl)acetic acid;
   2-(3-(3-chloro-4-(2-chloro-3-(6-methoxy-5-((methylamino)methyl)pyridin-2-yl)phenyl)pyridin-2-yl)-5-methoxybenzyl)-2-azaspiro[3.3]heptane-6-carboxylic acid;
   3-((3-(3-chloro-4-(2-chloro-3-(6-methoxy-5-((methylamino)methyl)pyridin-2-yl)phenyl)pyridin-2-yl)-5-methoxybenzyl)amino)bicyclo[1.1.1]pentane-1-carboxylic acid;
   3-(((3-(3-chloro-4-(2-chloro-3-(6-methoxy-5-((methylamino)methyl)pyridin-2-yl)phenyl)pyridin-2-yl)-5-methoxybenzyl)amino)methyl)bicyclo[1.1.1]pentane-1-carboxylic acid;
   (S)-5-((((6-(2-chloro-3-(3-chloro-2-(2-((S)-2-hydroxypropyl)-8-methoxy-1,2,3,4-tetrahydroisoquinolin-6-yl)pyridin-4-yl)phenyl)-4-fluoro-2-methoxypyridin-3-yl)methyl)amino)methyl)pyrrolidin-2-one;
   (S)-5-((((6-(2-chloro-3-(3-chloro-2-(2-((R)-2-hydroxypropyl)-8-methoxy-1,2,3,4-tetrahydroisoquinolin-6-yl)pyridin-4-yl)phenyl)-4-fluoro-2-methoxypyridin-3-yl)methyl)amino)methyl)pyrrolidin-2-one;
   (R)-5-((((6-(2-chloro-3-(3-chloro-2-(2-((S)-2-hydroxypropyl)-8-methoxy-1,2,3,4-tetrahydroisoquinolin-6-yl)pyridin-4-yl)phenyl)-4-fluoro-2-methoxypyridin-3-yl)methyl)amino)methyl)pyrrolidin-2-one;
   (R)-5-((((6-(2-chloro-3-(3-chloro-2-(2-((R)-2-hydroxypropyl)-8-methoxy-1,2,3,4-tetrahydroisoquinolin-6-yl)pyridin-4-yl)phenyl)-4-fluoro-2-methoxypyridin-3-yl)methyl)amino)methyl)pyrrolidin-2-one;
   (S)-5-((((6-(2-chloro-3-(3-chloro-2-(1,1-dioxido-2,3,4,5-tetrahydrobenzo[f][1,4]thiazepin-8-yl)pyridin-4-yl)phenyl)-2-methoxypyridin-3-yl)methyl)amino)methyl)pyrrolidin-2-one;
   (R)-5-((((6-(2-chloro-3-(3-chloro-2-(1,1-dioxido-2,3,4,5-tetrahydrobenzo[f][1,4]thiazepin-8-yl)pyridin-4-yl)phenyl)-2-methoxypyridin-3-yl)methyl)amino)methyl)pyrrolidin-2-one;
   5-(3-chloro-4-(2-chloro-3-(4-fluoro-6-methoxy-5-(((((S)-5-oxopyrrolidin-2-yl)methyl)amino)methyl)pyridin-2-yl)phenyl)pyridin-2-yl)-2-(2-((((S)-5-oxopyrrolidin-2-yl)methyl)amino)ethyl)isoindolin-1-one;
   5-(3-chloro-4-(2-chloro-3-(4-fluoro-6-methoxy-5-(((((S)-5-oxopyrrolidin-2-yl)methyl)amino)methyl)pyridin-2-yl)phenyl)pyridin-2-yl)-2-(2-((((R)-5-oxopyrrolidin-2-yl)methyl)amino)ethyl)isoindolin-1-one;
   5-(3-chloro-4-(2-chloro-3-(4-fluoro-6-methoxy-5-(((((R)-5-oxopyrrolidin-2-yl)methyl)amino)methyl)pyridin-2-yl)phenyl)pyridin-2-yl)-2-(2-((((S)-5-oxopyrrolidin-2-yl)methyl)amino)ethyl)isoindolin-1-one;
   5-(3-chloro-4-(2-chloro-3-(4-fluoro-6-methoxy-5-(((((R)-5-oxopyrrolidin-2-yl)methyl)amino)methyl)pyridin-2-yl)phenyl)pyridin-2-yl)-2-(2-((((R)-5-oxopyrrolidin-2-yl)methyl)amino)ethyl)isoindolin-1-one;
   (S)-5-((((6-(2-chloro-3-(3-chloro-2-(4-(4,5-dihydro-1H-imidazol-2-yl)-3-methoxyphenyl)pyridin-4-yl)phenyl)-2-methoxypyridin-3-yl)methyl)amino)methyl)pyrrolidin-2-one;
   (R)-5-((((6-(2-chloro-3-(3-chloro-2-(4-(4,5-dihydro-1H-imidazol-2-yl)-3-methoxyphenyl)pyridin-4-yl)phenyl)-2-methoxypyridin-3-yl)methyl)amino)methyl)pyrrolidin-2-one;
   (S)-4-((4-(3-chloro-4-(2-chloro-3-(5-(4,5-dihydro-1H-imidazol-2-yl)-6-methoxypyridin-2-yl)phenyl)pyridin-2-yl)-2-methoxybenzyl)amino)-3-hydroxybutanoic acid;
   (R)-4-((4-(3-chloro-4-(2-chloro-3-(5-(4,5-dihydro-1H-imidazol-2-yl)-6-methoxypyridin-2-yl)phenyl)pyridin-2-yl)-2-methoxybenzyl)amino)-3-hydroxybutanoic acid;
   (S)-5-((((6-(2-chloro-3-(3-chloro-2-(3-fluoro-5-methoxy-4-(((((S)-oxetan-2-yl)methyl)amino)methyl)phenyl)pyridin-4-yl)phenyl)-2-methoxypyridin-3-yl)methyl)amino)methyl)pyrrolidin-2-one;
   (S)-5-((((6-(2-chloro-3-(3-chloro-2-(3-fluoro-5-methoxy-4-(((((R)-oxetan-2-yl)methyl)amino)methyl)phenyl)pyridin-4-yl)phenyl)-2-methoxypyridin-3-yl)methyl)amino)methyl)pyrrolidin-2-one;
   (R)-5-((((6-(2-chloro-3-(3-chloro-2-(3-fluoro-5-methoxy-4-(((((S)-oxetan-2-yl)methyl)amino)methyl)phenyl)pyridin-4-yl)phenyl)-2-methoxypyridin-3-yl)methyl)amino)methyl)pyrrolidin-2-one;
   (S)-5-((((6-(2-chloro-3-(3-chloro-2-(3-fluoro-5-methoxy-4-(((((S)-oxetan-2-yl)methyl)amino)methyl)phenyl)pyridin-4-yl)phenyl)-2-methoxypyridin-3-yl)methyl)amino)methyl)pyrrolidin-2-one;
   methyl (S)-1-((6-(3-chloro-4-(2-chloro-3-(6-methoxy-5-((((5-oxopyrrolidin-2-yl)methyl)amino)methyl)pyridin-2-yl)phenyl)pyridin-2-yl)-3-methyl-4-oxo-3,4-dihydropyrrolo[2,1-f][1,2,4]triazin-2-yl)methyl)azetidine-3-carboxylate;
   methyl (R)-1-((6-(3-chloro-4-(2-chloro-3-(6-methoxy-5-((((5-oxopyrrolidin-2-yl)methyl)amino)methyl)pyridin-2-yl)phenyl)pyridin-2-yl)-3-methyl-4-oxo-3,4-dihydropyrrolo[2,1-f][1,2,4]triazin-2-yl)methyl)azetidine-3-carboxylate;
   (S)-5-((((6-(2-chloro-3-(3-chloro-2-(1-methyl-3-(((((S)-5-oxopyrrolidin-2-yl)methyl)amino)methyl)-1H-indazol-6-yl)pyridin-4-yl)phenyl)-2-methoxypyridin-3-yl)methyl)amino)methyl)pyrrolidin-2-one;
   (R)-5-((((6-(2-chloro-3-(3-chloro-2-(1-methyl-3-(((((S)-5-oxopyrrolidin-2-yl)methyl)amino)methyl)-1H-indazol-6-yl)pyridin-4-yl)phenyl)-2-methoxypyridin-3-yl)methyl)amino)methyl)pyrrolidin-2-one;
   (S)-5-((((6-(2-chloro-3-(3-chloro-2-((S)-5-((((S)-5-oxopyrrolidin-2-yl)methyl)amino)-5,6,7,8-tetrahydronaphthalen-2-yl)pyridin-4-yl)phenyl)-2-methoxypyridin-3-yl)methyl)amino)methyl)pyrrolidin-2-one;
   (S)-5-((((6-(2-chloro-3-(3-chloro-2-((S)-5-((((R)-5-oxopyrrolidin-2-yl)methyl)amino)-5,6,7,8-tetrahydronaphthalen-2-yl)pyridin-4-yl)phenyl)-2-methoxypyridin-3-yl)methyl)amino)methyl)pyrrolidin-2-one;
   (S)-5-((((6-(2-chloro-3-(3-chloro-2-((R)-5-((((S)-5-oxopyrrolidin-2-yl)methyl)amino)-5,6,7,8-tetrahydronaphthalen-2-yl)pyridin-4-yl)phenyl)-2-methoxypyridin-3-yl)methyl)amino)methyl)pyrrolidin-2-one;
   (S)-5-((((6-(2-chloro-3-(3-chloro-2-((R)-5-((((R)-5-oxopyrrolidin-2-yl)methyl)amino)-5,6,7,8-tetrahydronaphthalen-2-yl)pyridin-4-yl)phenyl)-2-methoxypyridin-3-yl)methyl)amino)methyl)pyrrolidin-2-one;
   (R)-5-((((6-(2-chloro-3-(3-chloro-2-((R)-5-((((R)-5-oxopyrrolidin-2-yl)methyl)amino)-5,6,7,8-tetrahydronaphthalen-2-yl)pyridin-4-yl)phenyl)-2-methoxypyridin-3-yl)methyl)amino)methyl)pyrrolidin-2-one;
   (R)-5-((((6-(2-chloro-3-(3-chloro-2-((R)-5-((((S)-5-oxopyrrolidin-2-yl)methyl)amino)-5,6,7,8-tetrahydronaphthalen-2-yl)pyridin-4-yl)phenyl)-2-methoxypyridin-3-yl)methyl)amino)methyl)pyrrolidin-2-one;
   (R)-5-((((6-(2-chloro-3-(3-chloro-2-((R)-5-((((R)-5-oxopyrrolidin-2-yl)methyl)amino)-5,6,7,8-tetrahydronaphthalen-2-yl)pyridin-4-yl)phenyl)-2-methoxypyridin-3-yl)methyl)amino)methyl)pyrrolidin-2-one;
   (R)-5-((((6-(2-chloro-3-(3-chloro-2-((S)-5-((((S)-5-oxopyrrolidin-2-yl)methyl)amino)-5,6,7,8-tetrahydronaphthalen-2-yl)pyridin-4-yl)phenyl)-2-methoxypyridin-3-yl)methyl)amino)methyl)pyrrolidin-2-one;
   1-(((6-(((S)-5-(3'-chloro-6-methoxy-5-(((((S)-5-oxopyrrolidin-2-yl)methyl)amino)methyl)-[2,4'-bipyridin]-2'-yl)-1,2,3,4-tetrahydronaphthalen-1-yl)amino)-2-methoxy-5-(trifluoromethyl)pyridin-3-yl)methyl)amino)cyclopropane-1-carboxylic acid;
   1-(((6-(((S)-5-(3'-chloro-6-methoxy-5-(((((R)-5-oxopyrrolidin-2-yl)methyl)amino)methyl)-[2,4'-bipyridin]-2'-yl)-1,2,3,4-tetrahydronaphthalen-1-yl)amino)-2-methoxy-5-(trifluoromethyl)pyridin-3-yl)methyl)amino)cyclopropane-1-carboxylic acid;
   1-(((6-(((R)-5-(3'-chloro-6-methoxy-5-(((((S)-5-oxopyrrolidin-2-yl)methyl)amino)methyl)-[2,4'-bipyridin]-2'-yl)-1,2,3,4-tetrahydronaphthalen-1-yl)amino)-2-methoxy-5-(trifluoromethyl)pyridin-3-yl)methyl)amino)cyclopropane-1-carboxylic acid;
   1-(((6-(((R)-5-(3'-chloro-6-methoxy-5-(((((R)-5-oxopyrrolidin-2-yl)methyl)amino)methyl)-[2,4'-bipyridin]-2'-yl)-1,2,3,4-tetrahydronaphthalen-1-yl)amino)-2-methoxy-5-(trifluoromethyl)pyridin-3-yl)methyl)amino)cyclopropane-1-carboxylic acid;
   (S)-5-((((3'-chloro-2'-((S)-5-((5-((((S)-2-hydroxypropyl)amino)methyl)-6-methoxy-3-(trifluoromethyl)pyridin-2-yl)amino)-5,6,7,8-tetrahydronaphthalen- 1-yl)-6-methoxy-[2,4'-bipyridin]-5-yl)methyl)amino)methyl)pyrrolidin-2-one;
   (S)-5-((((3'-chloro-2'-((S)-5-((5-((((R)-2-hydroxypropyl)amino)methyl)-6-methoxy-3-(trifluoromethyl)pyridin-2-yl)amino)-5,6,7,8-tetrahydronaphthalen- 1-yl)-6-methoxy-[2,4'-bipyridin]-5-yl)methyl)amino)methyl)pyrrolidin-2-one;
   (S)-5-((((3'-chloro-2'-((R)-5-((5-((((S)-2-hydroxypropyl)amino)methyl)-6-methoxy-3-(trifluoromethyl)pyridin-2-yl)amino)-5,6,7,8-tetrahydronaphthalen- 1-yl)-6-methoxy-[2,4'-bipyridin]-5-yl)methyl)amino)methyl)pyrrolidin-2-one;
   (S)-5-((((3'-chloro-2'-((R)-5-((5-((((R)-2-hydroxypropyl)amino)methyl)-6-methoxy-3-(trifluoromethyl)pyridin-2-yl)amino)-5,6,7,8-tetrahydronaphthalen- 1-yl)-6-methoxy-[2,4'-bipyridin]-5-yl)methyl)amino)methyl)pyrrolidin-2-one;
   (R)-5-((((3'-chloro-2'-((R)-5-((5-((((R)-2-hydroxypropyl)amino)methyl)-6-methoxy-3-(trifluoromethyl)pyridin-2-yl)amino)-5,6,7,8-tetrahydronaphthalen- 1-yl)-6-methoxy-[2,4'-bipyridin]-5-yl)methyl)amino)methyl)pyrrolidin-2-one;
   (R)-5-((((3'-chloro-2'-((R)-5-((5-((((S)-2-hydroxypropyl)amino)methyl)-6-methoxy-3-(trifluoromethyl)pyridin-2-yl)amino)-5,6,7,8-tetrahydronaphthalen- 1-yl)-6-methoxy-[2,4'-bipyridin]-5-yl)methyl)amino)methyl)pyrrolidin-2-one;
   (R)-5-((((3'-chloro-2'-((S)-5-((5-((((R)-2-hydroxypropyl)amino)methyl)-6-methoxy-3-(trifluoromethyl)pyridin-2-yl)amino)-5,6,7,8-tetrahydronaphthalen- 1-yl)-6-methoxy-[2,4'-bipyridin]-5-yl)methyl)amino)methyl)pyrrolidin-2-one;
   (R)-5-((((3'-chloro-2'-((S)-5-((5-((((S)-2-hydroxypropyl)amino)methyl)-6-methoxy-3-(trifluoromethyl)pyridin-2-yl)amino)-5,6,7,8-tetrahydronaphthalen- 1-yl)-6-methoxy-[2,4'-bipyridin]-5-yl)methyl)amino)methyl)pyrrolidin-2-one;
   (S)-5-((((3'-chloro-6-methoxy-2'-((S)-5-((6-methoxy-5-(((((S)-5-oxopyrrolidin-2-yl)methyl)amino)methyl)-3-(trifluoromethyl)pyridin-2-yl)amino)-5,6,7,8-tetrahydronaphthalen-1-yl)-[2,4'-bipyridin]-5-yl)methyl)amino)methyl)pyrrolidin-2-one;
   (S)-5-((((3'-chloro-6-methoxy-2'-((S)-5-((6-methoxy-5-(((((R)-5-oxopyrrolidin-2-yl)methyl)amino)methyl)-3-(trifluoromethyl)pyridin-2-yl)amino)-5,6,7,8-tetrahydronaphthalen-1-yl)-[2,4'-bipyridin]-5-yl)methyl)amino)methyl)pyrrolidin-2-one;
   (S)-5-((((3'-chloro-6-methoxy-2'-((R)-5-((6-methoxy-5-(((((S)-5-oxopyrrolidin-2-yl)methyl)amino)methyl)-3-(trifluoromethyl)pyridin-2-yl)amino)-5,6,7,8-tetrahydronaphthalen-1-yl)-[2,4'-bipyridin]-5-yl)methyl)amino)methyl)pyrrolidin-2-one;
   (S)-5-((((3'-chloro-6-methoxy-2'-((R)-5-((6-methoxy-5-(((((R)-5-oxopyrrolidin-2-yl)methyl)amino)methyl)-3-(trifluoromethyl)pyridin-2-yl)amino)-5,6,7,8-tetrahydronaphthalen-1-yl)-[2,4'-bipyridin]-5-yl)methyl)amino)methyl)pyrrolidin-2-one;
   (R)-5-((((3'-chloro-6-methoxy-2'-((R)-5-((6-methoxy-5-(((((R)-5-oxopyrrolidin-2-yl)methyl)amino)methyl)-3-(trifluoromethyl)pyridin-2-yl)amino)-5,6,7,8-tetrahydronaphthalen-1-yl)-[2,4'-bipyridin]-5-yl)methyl)amino)methyl)pyrrolidin-2-one;
   (R)-5-((((3'-chloro-6-methoxy-2'-((R)-5-((6-methoxy-5-(((((S)-5-oxopyrrolidin-2-yl)methyl)amino)methyl)-3-(trifluoromethyl)pyridin-2-yl)amino)-5,6,7,8-tetrahydronaphthalen-1-yl)-[2,4'-bipyridin]-5-yl)methyl)amino)methyl)pyrrolidin-2-one;
   (R)-5-((((3'-chloro-6-methoxy-2'-((S)-5-((6-methoxy-5-(((((R)-5-oxopyrrolidin-2-yl)methyl)amino)methyl)-3-(trifluoromethyl)pyridin-2-yl)amino)-5,6,7,8-tetrahydronaphthalen-1-yl)-[2,4'-bipyridin]-5-yl)methyl)amino)methyl)pyrrolidin-2-one;
   (R)-5-((((3'-chloro-6-methoxy-2'-((S)-5-((6-methoxy-5-(((((S)-5-oxopyrrolidin-2-yl)methyl)amino)methyl)-3-(trifluoromethyl)pyridin-2-yl)amino)-5,6,7,8-tetrahydronaphthalen-1-yl)-[2,4'-bipyridin]-5-yl)methyl)amino)methyl)pyrrolidin-2-one;
   2-((6-(((S)-5-(3'-chloro-6-methoxy-5-(((((S)-5-oxopyrrolidin-2-yl)methyl)amino)methyl)-[2,4'-bipyridin]-2'-yl)-1,2,3,4-tetrahydronaphthalen-1-yl)amino)-2-methoxy-5-(trifluoromethyl)pyridin-3-yl)methyl)-2,6-diazaspiro[3.4]octan-7-one;
   2-((6-(((S)-5-(3'-chloro-6-methoxy-5-(((((R)-5-oxopyrrolidin-2-yl)methyl)amino)methyl)-[2,4'-bipyridin]-2'-yl)-1,2,3,4-tetrahydronaphthalen-1-yl)amino)-2-methoxy-5-(trifluoromethyl)pyridin-3-yl)methyl)-2,6-diazaspiro[3.4]octan-7-one;
   2-((6-(((R)-5-(3'-chloro-6-methoxy-5-(((((S)-5-oxopyrrolidin-2-yl)methyl)amino)methyl)-[2,4'-bipyridin]-2'-yl)-1,2,3,4-tetrahydronaphthalen-1-yl)amino)-2-methoxy-5-(trifluoromethyl)pyridin-3-yl)methyl)-2,6-diazaspiro[3.4]octan-7-one;
   2-((6-(((R)-5-(3'-chloro-6-methoxy-5-(((((R)-5-oxopyrrolidin-2-yl)methyl)amino)methyl)-[2,4'-bipyridin]-2'-yl)-1,2,3,4-tetrahydronaphthalen-1-yl)amino)-2-methoxy-5-(trifluoromethyl)pyridin-3-yl)methyl)-2,6-diazaspiro[3.4]octan-7-one;
   (S)-N-(5-(3'-chloro-6-methoxy-5-((methylamino)methyl)-[2,4'-bipyridin]-2'-yl)-1,2,3,4-tetrahydronaphthalen-1-yl)-6-methoxy-5-((methylamino)methyl)-3-(trifluoromethyl)pyridin-2-amine;
   (R)-N-(5-(3'-chloro-6-methoxy-5-((methylamino)methyl)-[2,4'-bipyridin]-2'-yl)-1,2,3,4-tetrahydronaphthalen-1-yl)-6-methoxy-5-((methylamino)methyl)-3-(trifluoromethyl)pyridin-2-amine;
   1-(((6-(((S)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)cyclopropane-1-carboxylic acid;
   1-(((6-(((S)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)cyclopropane-1-carboxylic acid;
   1-(((6-(((R)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)cyclopropane-1-carboxylic acid;
   1-(((6-(((R)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)cyclopropane-1-carboxylic acid;
   (S)-5-(((4-(3-chloro-4-((S)-5-((5-((((S)-2-hydroxypropyl)amino)methyl)-6-methoxy-3-(trifluoromethyl)pyridin-2-yl)amino)-5,6,7,8-tetrahydronaphthalen-1-yl)pyridin-2-yl)-2-methoxybenzyl)amino)methyl)pyrrolidin-2-one;
   (S)-5-(((4-(3-chloro-4-((S)-5-((5-((((R)-2-hydroxypropyl)amino)methyl)-6-methoxy-3-(trifluoromethyl)pyridin-2-yl)amino)-5,6,7,8-tetrahydronaphthalen-1-yl)pyridin-2-yl)-2-methoxybenzyl)amino)methyl)pyrrolidin-2-one;
   (S)-5-(((4-(3-chloro-4-((R)-5-((5-((((S)-2-hydroxypropyl)amino)methyl)-6-methoxy-3-(trifluoromethyl)pyridin-2-yl)amino)-5,6,7,8-tetrahydronaphthalen-1-yl)pyridin-2-yl)-2-methoxybenzyl)amino)methyl)pyrrolidin-2-one;
   (S)-5-(((4-(3-chloro-4-((R)-5-((5-((((R)-2-hydroxypropyl)amino)methyl)-6-methoxy-3-(trifluoromethyl)pyridin-2-yl)amino)-5,6,7,8-tetrahydronaphthalen-1-yl)pyridin-2-yl)-2-methoxybenzyl)amino)methyl)pyrrolidin-2-one;
   (R)-5-(((4-(3-chloro-4-((R)-5-((5-((((R)-2-hydroxypropyl)amino)methyl)-6-methoxy-3-(trifluoromethyl)pyridin-2-yl)amino)-5,6,7,8-tetrahydronaphthalen-1-yl)pyridin-2-yl)-2-methoxybenzyl)amino)methyl)pyrrolidin-2-one;
   (R)-5-(((4-(3-chloro-4-((R)-5-((5-((((S)-2-hydroxypropyl)amino)methyl)-6-methoxy-3-(trifluoromethyl)pyridin-2-yl)amino)-5,6,7,8-tetrahydronaphthalen-1-yl)pyridin-2-yl)-2-methoxybenzyl)amino)methyl)pyrrolidin-2-one;
   (R)-5-(((4-(3-chloro-4-((S)-5-((5-((((R)-2-hydroxypropyl)amino)methyl)-6-methoxy-3-(trifluoromethyl)pyridin-2-yl)amino)-5,6,7,8-tetrahydronaphthalen-1-yl)pyridin-2-yl)-2-methoxybenzyl)amino)methyl)pyrrolidin-2-one;
   (R)-5-(((4-(3-chloro-4-((S)-5-((5-((((S)-2-hydroxypropyl)amino)methyl)-6-methoxy-3-(trifluoromethyl)pyridin-2-yl)amino)-5,6,7,8-tetrahydronaphthalen-1-yl)pyridin-2-yl)-2-methoxybenzyl)amino)methyl)pyrrolidin-2-one;
   (S)-5-((((6-(((S)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)methyl)pyrrolidin-2-one;
   (S)-5-((((6-(((S)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)methyl)pyrrolidin-2-one;
   (S)-5-((((6-(((R)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)methyl)pyrrolidin-2-one;
   (S)-5-((((6-(((R)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)methyl)pyrrolidin-2-one;
   (R)-5-((((6-(((R)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)methyl)pyrrolidin-2-one;
   (R)-5-((((6-(((R)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)methyl)pyrrolidin-2-one;
   (R)-5-((((6-(((S)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)methyl)pyrrolidin-2-one;
   (R)-5-((((6-(((S)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)methyl)pyrrolidin-2-one;
   2-((6-(((S)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)-2,6-diazaspiro[3.4]octan-7-one;
   2-((6-(((S)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)-2,6-diazaspiro[3.4]octan-7-one;
   2-((6-(((R)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)-2,6-diazaspiro[3.4]octan-7-one;
   2-((6-(((R)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)-2,6-diazaspiro[3.4]octan-7-one;
   (S)-N-(5-(3-chloro-2-(3-methoxy-4-((methylamino)methyl)phenyl)pyridin-4-yl)-1,2,3,4-tetrahydronaphthalen-1-yl)-6-methoxy-5-((methylamino)methyl)-3-(trifluoromethyl)pyridin-2-amine;
   (R)-N-(5-(3-chloro-2-(3-methoxy-4-((methylamino)methyl)phenyl)pyridin-4-yl)-1,2,3,4-tetrahydronaphthalen-1-yl)-6-methoxy-5-((methylamino)methyl)-3-(trifluoromethyl)pyridin-2-amine;
   1-(((6-(((S)-5-(3'-chloro-6-methoxy-5-(((((S)-5-oxopyrrolidin-2-yl)methyl)amino)methyl)-[2,4'-bipyridin]-2'-yl)-1,2,3,4-tetrahydronaphthalen-1-yl)oxy)-2-methoxy-5-(trifluoromethyl)pyridin-3-yl)methyl)amino)cyclopropane-1-carboxylic acid;
   1-(((6-(((S)-5-(3'-chloro-6-methoxy-5-(((((R)-5-oxopyrrolidin-2-yl)methyl)amino)methyl)-[2,4'-bipyridin]-2'-yl)-1,2,3,4-tetrahydronaphthalen-1-yl)oxy)-2-methoxy-5-(trifluoromethyl)pyridin-3-yl)methyl)amino)cyclopropane-1-carboxylic acid;
   1-(((6-(((R)-5-(3'-chloro-6-methoxy-5-(((((S)-5-oxopyrrolidin-2-yl)methyl)amino)methyl)-[2,4'-bipyridin]-2'-yl)-1,2,3,4-tetrahydronaphthalen-1-yl)oxy)-2-methoxy-5-(trifluoromethyl)pyridin-3-yl)methyl)amino)cyclopropane-1-carboxylic acid;
   1-(((6-(((R)-5-(3'-chloro-6-methoxy-5-(((((R)-5-oxopyrrolidin-2-yl)methyl)amino)methyl)-[2,4'-bipyridin]-2'-yl)-1,2,3,4-tetrahydronaphthalen-1-yl)oxy)-2-methoxy-5-(trifluoromethyl)pyridin-3-yl)methyl)amino)cyclopropane-1-carboxylic acid;
   (S)-5-((((3'-chloro-2'-((S)-5-((5-((((S)-2-hydroxypropyl)amino)methyl)-6-methoxy-3-(trifluoromethyl)pyridin-2-yl)oxy)-5,6,7,8-tetrahydronaphthalen-1-yl)-6-methoxy-[2,4'-bipyridin]-5-yl)methyl)amino)methyl)pyrrolidin-2-one;
   (S)-5-((((3'-chloro-2'-((S)-5-((5-((((R)-2-hydroxypropyl)amino)methyl)-6-methoxy-3-(trifluoromethyl)pyridin-2-yl)oxy)-5,6,7,8-tetrahydronaphthalen-1-yl)-6-methoxy-[2,4'-bipyridin]-5-yl)methyl)amino)methyl)pyrrolidin-2-one;
   (S)-5-((((3'-chloro-2'-((R)-5-((5-((((S)-2-hydroxypropyl)amino)methyl)-6-methoxy-3-(trifluoromethyl)pyridin-2-yl)oxy)-5,6,7,8-tetrahydronaphthalen-1-yl)-6-methoxy-[2,4'-bipyridin]-5-yl)methyl)amino)methyl)pyrrolidin-2-one;
   (S)-5-((((3'-chloro-2'-((R)-5-((5-((((R)-2-hydroxypropyl)amino)methyl)-6-methoxy-3-(trifluoromethyl)pyridin-2-yl)oxy)-5,6,7,8-tetrahydronaphthalen-1-yl)-6-methoxy-[2,4'-bipyridin]-5-yl)methyl)amino)methyl)pyrrolidin-2-one;
   (R)-5-((((3'-chloro-2'-((R)-5-((5-((((R)-2-hydroxypropyl)amino)methyl)-6-methoxy-3-(trifluoromethyl)pyridin-2-yl)oxy)-5,6,7,8-tetrahydronaphthalen-1-yl)-6-methoxy-[2,4'-bipyridin]-5-yl)methyl)amino)methyl)pyrrolidin-2-one;
   (R)-5-((((3'-chloro-2'-((R)-5-((5-((((S)-2-hydroxypropyl)amino)methyl)-6-methoxy-3-(trifluoromethyl)pyridin-2-yl)oxy)-5,6,7,8-tetrahydronaphthalen-1-yl)-6-methoxy-[2,4'-bipyridin]-5-yl)methyl)amino)methyl)pyrrolidin-2-one;
   (R)-5-((((3'-chloro-2'-((S)-5-((5-((((R)-2-hydroxypropyl)amino)methyl)-6-methoxy-3-(trifluoromethyl)pyridin-2-yl)oxy)-5,6,7,8-tetrahydronaphthalen-1-yl)-6-methoxy-[2,4'-bipyridin]-5-yl)methyl)amino)methyl)pyrrolidin-2-one;
   (R)-5-((((3'-chloro-2'-((S)-5-((5-((((S)-2-hydroxypropyl)amino)methyl)-6-methoxy-3-(trifluoromethyl)pyridin-2-yl)oxy)-5,6,7,8-tetrahydronaphthalen-1-yl)-6-methoxy-[2,4'-bipyridin]-5-yl)methyl)amino)methyl)pyrrolidin-2-one;
   (S)-5-((((3'-chloro-6-methoxy-2'-((S)-5-((6-methoxy-5-(((((S)-5-oxopyrrolidin-2-yl)methyl)amino)methyl)-3-(trifluoromethyl)pyridin-2-yl)oxy)-5,6,7,8-tetrahydronaphthalen-1-yl)-[2,4'-bipyridin]-5-yl)methyl)amino)methyl)pyrrolidin-2-one;
   (S)-5-((((3'-chloro-6-methoxy-2'-((S)-5-((6-methoxy-5-(((((R)-5-oxopyrrolidin-2-yl)methyl)amino)methyl)-3-(trifluoromethyl)pyridin-2-yl)oxy)-5,6,7,8-tetrahydronaphthalen-1-yl)-[2,4'-bipyridin]-5-yl)methyl)amino)methyl)pyrrolidin-2-one;
   (S)-5-((((3'-chloro-6-methoxy-2'-((R)-5-((6-methoxy-5-(((((S)-5-oxopyrrolidin-2-yl)methyl)amino)methyl)-3-(trifluoromethyl)pyridin-2-yl)oxy)-5,6,7,8-tetrahydronaphthalen-1-yl)-[2,4'-bipyridin]-5-yl)methyl)amino)methyl)pyrrolidin-2-one;
   (S)-5-((((3'-chloro-6-methoxy-2'-((R)-5-((6-methoxy-5-(((((R)-5-oxopyrrolidin-2-yl)methyl)amino)methyl)-3-(trifluoromethyl)pyridin-2-yl)oxy)-5,6,7,8-tetrahydronaphthalen-1-yl)-[2,4'-bipyridin]-5-yl)methyl)amino)methyl)pyrrolidin-2-one;
   (R)-5-((((3'-chloro-6-methoxy-2'-((R)-5-((6-methoxy-5-(((((R)-5-oxopyrrolidin-2-yl)methyl)amino)methyl)-3-(trifluoromethyl)pyridin-2-yl)oxy)-5,6,7,8-tetrahydronaphthalen-1-yl)-[2,4'-bipyridin]-5-yl)methyl)amino)methyl)pyrrolidin-2-one;
   (R)-5-((((3'-chloro-6-methoxy-2'-((R)-5-((6-methoxy-5-(((((S)-5-oxopyrrolidin-2-yl)methyl)amino)methyl)-3-(trifluoromethyl)pyridin-2-yl)oxy)-5,6,7,8-tetrahydronaphthalen-1-yl)-[2,4'-bipyridin]-5-yl)methyl)amino)methyl)pyrrolidin-2-one;
   (R)-5-((((3'-chloro-6-methoxy-2'-((S)-5-((6-methoxy-5-(((((R)-5-oxopyrrolidin-2-yl)methyl)amino)methyl)-3-(trifluoromethyl)pyridin-2-yl)oxy)-5,6,7,8-tetrahydronaphthalen-1-yl)-[2,4'-bipyridin]-5-yl)methyl)amino)methyl)pyrrolidin-2-one;
   (R)-5-((((3'-chloro-6-methoxy-2'-((S)-5-((6-methoxy-5-(((((S)-5-oxopyrrolidin-2-yl)methyl)amino)methyl)-3-(trifluoromethyl)pyridin-2-yl)oxy)-5,6,7,8-tetrahydronaphthalen-1-yl)-[2,4'-bipyridin]-5-yl)methyl)amino)methyl)pyrrolidin-2-one;
   2-((6-(((S)-5-(3'-chloro-6-methoxy-5-(((((S)-5-oxopyrrolidin-2-yl)methyl)amino)methyl)-[2,4'-bipyridin]-2'-yl)-1,2,3,4-tetrahydronaphthalen-1-yl)oxy)-2-methoxy-5-(trifluoromethyl)pyridin-3-yl)methyl)-2,6-diazaspiro[3.4]octan-7-one;
   2-((6-(((S)-5-(3'-chloro-6-methoxy-5-(((((R)-5-oxopyrrolidin-2-yl)methyl)amino)methyl)-[2,4'-bipyridin]-2'-yl)-1,2,3,4-tetrahydronaphthalen-1-yl)oxy)-2-methoxy-5-(trifluoromethyl)pyridin-3-yl)methyl)-2,6-diazaspiro[3.4]octan-7-one;
   2-((6-(((S)-5-(3'-chloro-6-methoxy-5-(((((S)-5-oxopyrrolidin-2-yl)methyl)amino)methyl)-[2,4'-bipyridin]-2'-yl)-1,2,3,4-tetrahydronaphthalen-1-yl)oxy)-2-methoxy-5-(trifluoromethyl)pyridin-3-yl)methyl)-2,6-diazaspiro[3.4]octan-7-one;
   2-((6-(((R)-5-(3'-chloro-6-methoxy-5-(((((R)-5-oxopyrrolidin-2-yl)methyl)amino)methyl)-[2,4'-bipyridin]-2'-yl)-1,2,3,4-tetrahydronaphthalen-1-yl)oxy)-2-methoxy-5-(trifluoromethyl)pyridin-3-yl)methyl)-2,6-diazaspiro[3.4]octan-7-one;
   (S)-1-(3'-chloro-6-methoxy-2'-(5-((6-methoxy-5-((methylamino)methyl)-3-(trifluoromethyl)pyridin-2-yl)oxy)-5,6,7,8-tetrahydronaphthalen-1-yl)-[2,4'-bipyridin]-5-yl)-N-methylmethanamine;
   (R)-1-(3'-chloro-6-methoxy-2'-(5-((6-methoxy-5-((methylamino)methyl)-3-(trifluoromethyl)pyridin-2-yl)oxy)-5,6,7,8-tetrahydronaphthalen-1-yl)-[2,4'-bipyridin]-5-yl)-N-methylmethanamine;
   1-(((6-(((S)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)oxy)-2-methoxy-5-(trifluoromethyl)pyridin-3-yl)methyl)amino)cyclopropane-1-carboxylic acid;
   1-(((6-(((S)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)oxy)-2-methoxy-5-(trifluoromethyl)pyridin-3-yl)methyl)amino)cyclopropane-1-carboxylic acid;
   1-(((6-(((R)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)oxy)-2-methoxy-5-(trifluoromethyl)pyridin-3-yl)methyl)amino)cyclopropane-1-carboxylic acid;
   1-(((6-(((R)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)oxy)-2-methoxy-5-(trifluoromethyl)pyridin-3-yl)methyl)amino)cyclopropane-1-carboxylic acid;
   (S)-5-(((4-(3-chloro-4-((S)-5-((5-((((S)-2-hydroxypropyl)amino)methyl)-6-methoxy-3-(trifluoromethyl)pyridin-2-yl)oxy)-5,6,7,8-tetrahydronaphthalen-1-yl)pyridin-2-yl)-2-methoxybenzyl)amino)methyl)pyrrolidin-2-one;
   (S)-5-(((4-(3-chloro-4-((S)-5-((5-((((R)-2-hydroxypropyl)amino)methyl)-6-methoxy-3-(trifluoromethyl)pyridin-2-yl)oxy)-5,6,7,8-tetrahydronaphthalen-1-yl)pyridin-2-yl)-2-methoxybenzyl)amino)methyl)pyrrolidin-2-one;
   (S)-5-(((4-(3-chloro-4-((R)-5-((5-((((S)-2-hydroxypropyl)amino)methyl)-6-methoxy-3-(trifluoromethyl)pyridin-2-yl)oxy)-5,6,7,8-tetrahydronaphthalen-1-yl)pyridin-2-yl)-2-methoxybenzyl)amino)methyl)pyrrolidin-2-one;
   (S)-5-(((4-(3-chloro-4-((R)-5-((5-((((R)-2-hydroxypropyl)amino)methyl)-6-methoxy-3-(trifluoromethyl)pyridin-2-yl)oxy)-5,6,7,8-tetrahydronaphthalen-1-yl)pyridin-2-yl)-2-methoxybenzyl)amino)methyl)pyrrolidin-2-one;
   (R)-5-(((4-(3-chloro-4-((R)-5-((5-((((R)-2-hydroxypropyl)amino)methyl)-6-methoxy-3-(trifluoromethyl)pyridin-2-yl)oxy)-5,6,7,8-tetrahydronaphthalen-1-yl)pyridin-2-yl)-2-methoxybenzyl)amino)methyl)pyrrolidin-2-one;
   (R)-5-(((4-(3-chloro-4-((R)-5-((5-((((S)-2-hydroxypropyl)amino)methyl)-6-methoxy-3-(trifluoromethyl)pyridin-2-yl)oxy)-5,6,7,8-tetrahydronaphthalen-1-yl)pyridin-2-yl)-2-methoxybenzyl)amino)methyl)pyrrolidin-2-one;
   (R)-5-(((4-(3-chloro-4-((S)-5-((5-((((R)-2-hydroxypropyl)amino)methyl)-6-methoxy-3-(trifluoromethyl)pyridin-2-yl)oxy)-5,6,7,8-tetrahydronaphthalen-1-yl)pyridin-2-yl)-2-methoxybenzyl)amino)methyl)pyrrolidin-2-one;
   (R)-5-(((4-(3-chloro-4-((S)-5-((5-((((S)-2-hydroxypropyl)amino)methyl)-6-methoxy-3-(trifluoromethyl)pyridin-2-yl)oxy)-5,6,7,8-tetrahydronaphthalen-1-yl)pyridin-2-yl)-2-methoxybenzyl)amino)methyl)pyrrolidin-2-one;
   (S)-5-((((6-(((S)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)oxy)-2-methoxy-5-(trifluoromethyl)pyridin-3-yl)methyl)amino)methyl)pyrrolidin-2-one;
   (S)-5-((((6-(((S)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)oxy)-2-methoxy-5-(trifluoromethyl)pyridin-3-yl)methyl)amino)methyl)pyrrolidin-2-one;
   (S)-5-((((6-(((R)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)oxy)-2-methoxy-5-(trifluoromethyl)pyridin-3-yl)methyl)amino)methyl)pyrrolidin-2-one;
   (S)-5-((((6-(((R)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)oxy)-2-methoxy-5-(trifluoromethyl)pyridin-3-yl)methyl)amino)methyl)pyrrolidin-2-one;
   (R)-5-((((6-(((R)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)oxy)-2-methoxy-5-(trifluoromethyl)pyridin-3-yl)methyl)amino)methyl)pyrrolidin-2-one;
   (R)-5-((((6-(((R)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)oxy)-2-methoxy-5-(trifluoromethyl)pyridin-3-yl)methyl)amino)methyl)pyrrolidin-2-one;
   (R)-5-((((6-(((S)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)oxy)-2-methoxy-5-(trifluoromethyl)pyridin-3-yl)methyl)amino)methyl)pyrrolidin-2-one;
   (R)-5-((((6-(((S)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)oxy)-2-methoxy-5-(trifluoromethyl)pyridin-3-yl)methyl)amino)methyl)pyrrolidin-2-one;
   2-((6-(((S)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)oxy)-2-methoxy-5-(trifluoromethyl)pyridin-3-yl)methyl)-2,6-diazaspiro[3.4]octan-7-one;
   2-((6-(((S)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)oxy)-2-methoxy-5-(trifluoromethyl)pyridin-3-yl)methyl)-2,6-diazaspiro[3.4]octan-7-one;
   2-((6-(((R)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)oxy)-2-methoxy-5-(trifluoromethyl)pyridin-3-yl)methyl)-2,6-diazaspiro[3.4]octan-7-one;
   2-((6-(((R)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)oxy)-2-methoxy-5-(trifluoromethyl)pyridin-3-yl)methyl)-2,6-diazaspiro[3.4]octan-7-one;
   (S)-1-(6-((5-(3-chloro-2-(3-methoxy-4-((methylamino)methyl)phenyl)pyridin-4-yl)-1,2,3,4-tetrahydronaphthalen-1-yl)oxy)-2-methoxy-5-(trifluoromethyl)pyridin-3-yl)-N-methylmethanamine;
   (R)-1-(6-((5-(3-chloro-2-(3-methoxy-4-((methylamino)methyl)phenyl)pyridin-4-yl)-1,2,3,4-tetrahydronaphthalen-1-yl)oxy)-2-methoxy-5-(trifluoromethyl)pyridin-3-yl)-N-methylmethanamine;
   (S)-5-(((4-(3-chloro-4-(2-chloro-3-(8-chloro-6-((((S)-2-hydroxypropyl)amino)methyl)-5-methoxyquinolin-2-yl)phenyl)pyridin-2-yl)-2-methoxybenzyl)amino)methyl)pyrrolidin-2-one;
   (S)-5-(((4-(3-chloro-4-(2-chloro-3-(8-chloro-6-((((R)-2-hydroxypropyl)amino)methyl)-5-methoxyquinolin-2-yl)phenyl)pyridin-2-yl)-2-methoxybenzyl)amino)methyl)pyrrolidin-2-one;
   (R)-5-(((4-(3-chloro-4-(2-chloro-3-(8-chloro-6-((((S)-2-hydroxypropyl)amino)methyl)-5-methoxyquinolin-2-yl)phenyl)pyridin-2-yl)-2-methoxybenzyl)amino)methyl)pyrrolidin-2-one;
   (R)-5-(((4-(3-chloro-4-(2-chloro-3-(8-chloro-6-((((R)-2-hydroxypropyl)amino)methyl)-5-methoxyquinolin-2-yl)phenyl)pyridin-2-yl)-2-methoxybenzyl)amino)methyl)pyrrolidin-2-one;
   (S)-5-((((8-chloro-2-(2-chloro-3-(3-chloro-2-(3-methoxy-4-(((((S)-5-oxopyrrolidin-2-yl)methyl)amino)methyl)phenyl)pyridin-4-yl)phenyl)-5-methoxyquinolin-6-yl)methyl)amino)methyl)pyrrolidin-2-one;
   (S)-5-((((8-chloro-2-(2-chloro-3-(3-chloro-2-(3-methoxy-4-(((((R)-5-oxopyrrolidin-2-yl)methyl)amino)methyl)phenyl)pyridin-4-yl)phenyl)-5-methoxyquinolin-6-yl)methyl)amino)methyl)pyrrolidin-2-one;
   (R)-5-((((8-chloro-2-(2-chloro-3-(3-chloro-2-(3-methoxy-4-(((((S)-5-oxopyrrolidin-2-yl)methyl)amino)methyl)phenyl)pyridin-4-yl)phenyl)-5-methoxyquinolin-6-yl)methyl)amino)methyl)pyrrolidin-2-one;
   (R)-5-((((8-chloro-2-(2-chloro-3-(3-chloro-2-(3-methoxy-4-(((((R)-5-oxopyrrolidin-2-yl)methyl)amino)methyl)phenyl)pyridin-4-yl)phenyl)-5-methoxyquinolin-6-yl)methyl)amino)methyl)pyrrolidin-2-one;
   (S)-5-((((6-(2-chloro-3-(3-chloro-2-(8-chloro-2-(3-hydroxy-3-methylbutyl)-1,2,3,4-tetrahydroisoquinolin-6-yl)pyridin-4-yl)phenyl)-2-methoxypyridin-3-yl)methyl)amino)methyl)pyrrolidin-2-one;
   (R)-5-((((6-(2-chloro-3-(3-chloro-2-(8-chloro-2-(3-hydroxy-3-methylbutyl)-1,2,3,4-tetrahydroisoquinolin-6-yl)pyridin-4-yl)phenyl)-2-methoxypyridin-3-yl)methyl)amino)methyl)pyrrolidin-2-one;
   isopropyl (S)-4-((4-(3-chloro-4-(2-chloro-3-(5-(4,5-dihydro-1H-imidazol-2-yl)-6-methoxypyridin-2-yl)phenyl)pyridin-2-yl)-2-methoxybenzyl)amino)-3-hydroxybutanoate;
   isopropyl (R)-4-((4-(3-chloro-4-(2-chloro-3-(5-(4,5-dihydro-1H-imidazol-2-yl)-6-methoxypyridin-2-yl)phenyl)pyridin-2-yl)-2-methoxybenzyl)amino)-3-hydroxybutanoate;
   (S)-5-((((6-(3-(2-(4-(((2-acetyl-2-azaspiro[3.3]heptan-6-yl)amino)methyl)-3-fluoro-5-methoxyphenyl)-3-chloropyridin-4-yl)-2-chlorophenyl)-2-methoxypyridin-3-yl)methyl)amino)methyl)pyrrolidin-2-one;
   (R)-5-((((6-(3-(2-(4-(((2-acetyl-2-azaspiro[3.3]heptan-6-yl)amino)methyl)-3-fluoro-5-methoxyphenyl)-3-chloropyridin-4-yl)-2-chlorophenyl)-2-methoxypyridin-3-yl)methyl)amino)methyl)pyrrolidin-2-one;
   N-(4-(3-chloro-4-(2-chloro-3-(6-methoxy-5-((methylamino)methyl)pyridin-2-yl)phenyl)pyridin-2-yl)-2-methoxybenzyl)propan-2-amine;
   1-(4-((4-(3-chloro-4-(2-chloro-3-(6-methoxy-5-((methylamino)methyl)pyridin-2-yl)phenyl)pyridin-2-yl)-2-methoxybenzyl)amino)piperidin-1-yl)ethan-1-one;
   2-(4-(3-chloro-4-(2-chloro-3-(6-methoxy-5-((methylamino)methyl)pyridin-2-yl)phenyl)pyridin-2-yl)-2-methoxybenzyl)-2,6-diazaspiro[3.4]octan-7-one;
   (S)-1-(((6-(2-chloro-3-(3-chloro-2-(2-((S)-2-hydroxypropyl)-8-methoxy-1,2,3,4-tetrahydroisoquinolin-6-yl)pyridin-4-yl)phenyl)-2-methoxypyridin-3-yl)methyl)amino)propan-2-ol;
   (S)-1-(((6-(2-chloro-3-(3-chloro-2-(2-((R)-2-hydroxypropyl)-8-methoxy-1,2,3,4-tetrahydroisoquinolin-6-yl)pyridin-4-yl)phenyl)-2-methoxypyridin-3-yl)methyl)amino)propan-2-ol;
   (R)-1-(((6-(2-chloro-3-(3-chloro-2-(2-((S)-2-hydroxypropyl)-8-methoxy-1,2,3,4-tetrahydroisoquinolin-6-yl)pyridin-4-yl)phenyl)-2-methoxypyridin-3-yl)methyl)amino)propan-2-ol;
   (R)-1-(((6-(2-chloro-3-(3-chloro-2-(2-((R)-2-hydroxypropyl)-8-methoxy-1,2,3,4-tetrahydroisoquinolin-6-yl)pyridin-4-yl)phenyl)-2-methoxypyridin-3-yl)methyl)amino)propan-2-ol;
   (1r,3r)-3-(((6-(2-chloro-3-(3-chloro-2-(2-((S)-2-hydroxypropyl)-8-methoxy-1,2,3,4-tetrahydroisoquinolin-6-yl)pyridin-4-yl)phenyl)-2-methoxypyridin-3-yl)methyl)amino)-1-methylcyclobutan-1-ol;
   (1r,3r)-3-(((6-(2-chloro-3-(3-chloro-2-(2-((R)-2-hydroxypropyl)-8-methoxy-1,2,3,4-tetrahydroisoquinolin-6-yl)pyridin-4-yl)phenyl)-2-methoxypyridin-3-yl)methyl)amino)-1-methylcyclobutan-1-ol;
   (1r,3s)-3-(((6-(2-chloro-3-(3-chloro-2-(2-((S)-2-hydroxypropyl)-8-methoxy-1,2,3,4-tetrahydroisoquinolin-6-yl)pyridin-4-yl)phenyl)-2-methoxypyridin-3-yl)methyl)amino)-1-methylcyclobutan-1-ol;
   (1s,3s)-3-(((6-(2-chloro-3-(3-chloro-2-(2-((S)-2-hydroxypropyl)-8-methoxy-1,2,3,4-tetrahydroisoquinolin-6-yl)pyridin-4-yl)phenyl)-2-methoxypyridin-3-yl)methyl)amino)-1-methylcyclobutan-1-ol;
   (1s,3s)-3-(((6-(2-chloro-3-(3-chloro-2-(2-((R)-2-hydroxypropyl)-8-methoxy-1,2,3,4-tetrahydroisoquinolin-6-yl)pyridin-4-yl)phenyl)-2-methoxypyridin-3-yl)methyl)amino)-1-methylcyclobutan-1-ol;
   (1s,3r)-3-(((6-(2-chloro-3-(3-chloro-2-(2-((R)-2-hydroxypropyl)-8-methoxy-1,2,3,4-tetrahydroisoquinolin-6-yl)pyridin-4-yl)phenyl)-2-methoxypyridin-3-yl)methyl)amino)-1-methylcyclobutan-1-ol;
   (1s,3r)-3-(((6-(2-chloro-3-(3-chloro-2-(2-((S)-2-hydroxypropyl)-8-methoxy-1,2,3,4-tetrahydroisoquinolin-6-yl)pyridin-4-yl)phenyl)-2-methoxypyridin-3-yl)methyl)amino)-1-methylcyclobutan-1-ol;
   (1r,3s)-3-(((6-(2-chloro-3-(3-chloro-2-(2-((R)-2-hydroxypropyl)-8-methoxy-1,2,3,4-tetrahydroisoquinolin-6-yl)pyridin-4-yl)phenyl)-2-methoxypyridin-3-yl)methyl)amino)-1-methylcyclobutan-1-ol;
   (S)-1-(6-(3-chloro-4-(2-chloro-3-(6-methoxy-5-(((((S)-tetrahydrofuran-2-yl)methyl)amino)methyl)pyridin-2-yl)phenyl)pyridin-2-yl)-8-methoxy-3,4-dihydroisoquinolin-2(1H)-yl)propan-2-ol;
   (S)-1-(6-(3-chloro-4-(2-chloro-3-(6-methoxy-5-(((((R)-tetrahydrofuran-2-yl)methyl)amino)methyl)pyridin-2-yl)phenyl)pyridin-2-yl)-8-methoxy-3,4-dihydroisoquinolin-2(1H)-yl)propan-2-ol;
   (R)-1-(6-(3-chloro-4-(2-chloro-3-(6-methoxy-5-(((((S)-tetrahydrofuran-2-yl)methyl)amino)methyl)pyridin-2-yl)phenyl)pyridin-2-yl)-8-methoxy-3,4-dihydroisoquinolin-2(1H)-yl)propan-2-ol;
   (R)-1-(6-(3-chloro-4-(2-chloro-3-(6-methoxy-5-(((((R)-tetrahydrofuran-2-yl)methyl)amino)methyl)pyridin-2-yl)phenyl)pyridin-2-yl)-8-methoxy-3,4-dihydroisoquinolin-2(1H)-yl)propan-2-ol;
   (S)-1-((6-(2-chloro-3-(3-chloro-2-(2-(2-hydroxypropyl)-8-methoxy-1,2,3,4-tetrahydroisoquinolin-6-yl)pyridin-4-yl)phenyl)-2-methoxypyridin-3-yl)methyl)-3-methylazetidin-3-ol;
   (R)-1-((6-(2-chloro-3-(3-chloro-2-(2-(2-hydroxypropyl)-8-methoxy-1,2,3,4-tetrahydroisoquinolin-6-yl)pyridin-4-yl)phenyl)-2-methoxypyridin-3-yl)methyl)-3-methylazetidin-3-ol;
   methyl (S)-((6-(2-chloro-3-(3-chloro-2-(2-(2-hydroxypropyl)-8-methoxy-1,2,3,4-tetrahydroisoquinolin-6-yl)pyridin-4-yl)phenyl)-2-methoxypyridin-3-yl)methyl)glycinate;
   methyl (R)-((6-(2-chloro-3-(3-chloro-2-(2-(2-hydroxypropyl)-8-methoxy-1,2,3,4-tetrahydroisoquinolin-6-yl)pyridin-4-yl)phenyl)-2-methoxypyridin-3-yl)methyl)glycinate;
   (S)-2-((6-(2-chloro-3-(3-chloro-2-(3-methoxy-4-((((5-oxopyrrolidin-2-yl)methyl)amino)methyl)phenyl)pyridin-4-yl)phenyl)-1-methyl-1H-pyrrolo[2,3-b]pyridin-3-yl)methyl)-2,6-diazaspiro[3.4]octan-7-one;
   (R)-2-((6-(2-chloro-3-(3-chloro-2-(3-methoxy-4-((((5-oxopyrrolidin-2-yl)methyl)amino)methyl)phenyl)pyridin-4-yl)phenyl)-1-methyl-1H-pyrrolo[2,3-b]pyridin-3-yl)methyl)-2,6-diazaspiro[3.4]octan-7-one;
   (S)-4-((4-(3-chloro-4-(2-chloro-3-(6-methoxy-5-((methylamino)methyl)pyridin-2-yl)phenyl)pyridin-2-yl)-2-methoxybenzyl)amino)-3-hydroxybutanoic acid;
   (R)-4-((4-(3-chloro-4-(2-chloro-3-(6-methoxy-5-((methylamino)methyl)pyridin-2-yl)phenyl)pyridin-2-yl)-2-methoxybenzyl)amino)-3-hydroxybutanoic acid;
   3-((4-(3-chloro-4-(2-chloro-3-(6-methoxy-5-((methylamino)methyl)pyridin-2-yl)phenyl)pyridin-2-yl)-2-methoxybenzyl)amino)propanoic acid;
   6-(3-chloro-4-(2-chloro-3-(5-((6-hydroxy-2-azaspiro[3.3]heptan-2-yl)methyl)-6-methoxypyridin-2-yl)phenyl)pyridin-2-yl)-2-((3-hydroxy-3-methylazetidin-1-yl)methyl)-3-methylpyrrolo[2,1-f][1,2,4]triazin-4(3H)-one;
   (S)-5-((((3'-chloro-6-methoxy-2'-((S)-1-((6-methoxy-5-(((((S)-5-oxopyrrolidin-2-yl)methyl)amino)methyl)-3-(trifluoromethyl)pyridin-2-yl)amino)-2,3-dihydro-1H-inden-4-yl)-[2,4'-bipyridin]-5-yl)methyl)amino)methyl)pyrrolidin-2-one;
   (R)-5-((((3'-chloro-6-methoxy-2'-((R)-1-((6-methoxy-5-(((((R)-5-oxopyrrolidin-2-yl)methyl)amino)methyl)-3-(trifluoromethyl)pyridin-2-yl)amino)-2,3-dihydro-1H-inden-4-yl)-[2,4'-bipyridin]-5-yl)methyl)amino)methyl)pyrrolidin-2-one;
   (R)-5-((((3'-chloro-6-methoxy-2'-((R)-1-((6-methoxy-5-(((((S)-5-oxopyrrolidin-2-yl)methyl)amino)methyl)-3-(trifluoromethyl)pyridin-2-yl)amino)-2,3-dihydro-1H-inden-4-yl)-[2,4'-bipyridin]-5-yl)methyl)amino)methyl)pyrrolidin-2-one;
   (R)-5-((((3'-chloro-6-methoxy-2'-((S)-1-((6-methoxy-5-(((((S)-5-oxopyrrolidin-2-yl)methyl)amino)methyl)-3-(trifluoromethyl)pyridin-2-yl)amino)-2,3-dihydro-1H-inden-4-yl)-[2,4'-bipyridin]-5-yl)methyl)amino)methyl)pyrrolidin-2-one;
   (R)-5-((((3'-chloro-6-methoxy-2'-((S)-1-((6-methoxy-5-(((((R)-5-oxopyrrolidin-2-yl)methyl)amino)methyl)-3-(trifluoromethyl)pyridin-2-yl)amino)-2,3-dihydro-1H-inden-4-yl)-[2,4'-bipyridin]-5-yl)methyl)amino)methyl)pyrrolidin-2-one;
   (S)-5-((((3'-chloro-6-methoxy-2'-((S)-1-((6-methoxy-5-(((((R)-5-oxopyrrolidin-2-yl)methyl)amino)methyl)-3-(trifluoromethyl)pyridin-2-yl)amino)-2,3-dihydro-1H-inden-4-yl)-[2,4'-bipyridin]-5-yl)methyl)amino)methyl)pyrrolidin-2-one;
   (S)-5-((((3'-chloro-6-methoxy-2'-((R)-1-((6-methoxy-5-(((((R)-5-oxopyrrolidin-2-yl)methyl)amino)methyl)-3-(trifluoromethyl)pyridin-2-yl)amino)-2,3-dihydro-1H-inden-4-yl)-[2,4'-bipyridin]-5-yl)methyl)amino)methyl)pyrrolidin-2-one;
   (S)-5-((((3'-chloro-6-methoxy-2'-((R)-1-((6-methoxy-5-(((((S)-5-oxopyrrolidin-2-yl)methyl)amino)methyl)-3-(trifluoromethyl)pyridin-2-yl)amino)-2,3-dihydro-1H-inden-4-yl)-[2,4'-bipyridin]-5-yl)methyl)amino)methyl)pyrrolidin-2-one;
   (S)-N-(4-(3'-chloro-6-methoxy-5-((methylamino)methyl)-[2,4'-bipyridin]-2'-yl)-2,3-dihydro-1H-inden-1-yl)-6-methoxy-5-((methylamino)methyl)-3-(trifluoromethyl)pyridin-2-amine;
   (R)-N-(4-(3'-chloro-6-methoxy-5-((methylamino)methyl)-[2,4'-bipyridin]-2'-yl)-2,3-dihydro-1H-inden-1-yl)-6-methoxy-5-((methylamino)methyl)-3-(trifluoromethyl)pyridin-2-amine;
   (1S,3r)-3-(((3'-chloro-2'-((S)-1-((5-((((1r,3S)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
   (1R,3r)-3-(((3'-chloro-2'-((R)-1-((5-((((1r,3R)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
   (1R,3r)-3-(((3'-chloro-2'-((R)-1-((5-((((1r,3S)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
   (1R,3r)-3-(((3'-chloro-2'-((R)-1-((5-((((1s,3R)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
   (1R,3r)-3-(((3'-chloro-2'-((R)-1-((5-((((1s,3S)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
   (1R,3r)-3-(((3'-chloro-2'-((S)-1-((5-((((1r,3R)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
   (1R,3r)-3-(((3'-chloro-2'-((S)-1-((5-((((1r,3S)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
   (1R,3r)-3-(((3'-chloro-2'-((S)-1-((5-((((1s,3R)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
   (1R,3r)-3-(((3'-chloro-2'-((S)-1-((5-((((1s,3S)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
   (1R,3s)-3-(((3'-chloro-2'-((R)-1-((5-((((1r,3R)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
   (1R,3s)-3-(((3'-chloro-2'-((R)-1-((5-((((1r,3S)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
   (1R,3s)-3-(((3'-chloro-2'-((R)-1-((5-((((1s,3R)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
   (1R,3s)-3-(((3'-chloro-2'-((R)-1-((5-((((1s,3S)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
   (1R,3r)-3-(((3'-chloro-2'-((R)-1-((5-((((1r,3R)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
   (1R,3s)-3-(((3'-chloro-2'-((S)-1-((5-((((1r,3S)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
   (1R,3s)-3-(((3'-chloro-2'-((S)-1-((5-((((1s,3R)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
   (1R,3s)-3-(((3'-chloro-2'-((S)-1-((5-((((1s,3S)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
   (1S,3r)-3-(((3'-chloro-2'-((R)-1-((5-((((1r,3R)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
   (1S,3r)-3-(((3'-chloro-2'-((R)-1-((5-((((1r,3S)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
   (1S,3r)-3-(((3'-chloro-2'-((R)-1-((5-((((1s,3R)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
   (1S,3r)-3-(((3'-chloro-2'-((R)-1-((5-((((1s,3S)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
   (1S,3r)-3-(((3'-chloro-2'-((S)-1-((5-((((1r,3R)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
   (1S,3r)-3-(((3'-chloro-2'-((S)-1-((5-((((1s,3R)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
   (1S,3r)-3-(((3'-chloro-2'-((S)-1-((5-((((1s,3S)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
   (1S,3s)-3-(((3'-chloro-2'-((R)-1-((5-((((1r,3R)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
   (1S,3s)-3-(((3'-chloro-2'-((R)-1-((5-((((1r,3S)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
   (1S,3s)-3-(((3'-chloro-2'-((R)-1-((5-((((1s,3R)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
   (1S,3s)-3-(((3'-chloro-2'-((R)-1-((5-((((1s,3S)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
   (1S,3r)-3-(((3'-chloro-2'-((R)-1-((5-((((1r,3R)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
   (1S,3s)-3-(((3'-chloro-2'-((S)-1-((5-((((1r,3S)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
   (1S,3s)-3-(((3'-chloro-2'-((S)-1-((5-((((1s,3R)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
   (1S,3s)-3-(((3'-chloro-2'-((S)-1-((5-((((1s,3S)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
   (S)-1-(4-(3-chloro-4-(2-chloro-3-(6-methoxy-5-((methylamino)methyl)pyridin-2-yl)phenyl)pyridin-2-yl)-2-methoxybenzyl)pyrrolidine-3-carboxylic acid;
   (R)-1-(4-(3-chloro-4-(2-chloro-3-(6-methoxy-5-((methylamino)methyl)pyridin-2-yl)phenyl)pyridin-2-yl)-2-methoxybenzyl)pyrrolidine-3-carboxylic acid;
   2-(4-(3-chloro-4-(2-chloro-3-(6-methoxy-5-((methylamino)methyl)pyridin-2-yl)phenyl)pyridin-2-yl)-2-methoxybenzyl)-2-azaspiro[3.3]heptane-6-carboxylic acid;
   3-(((4-(3-chloro-4-(2-chloro-3-(6-methoxy-5-((methylamino)methyl)pyridin-2-yl)phenyl)pyridin-2-yl)-2-methoxybenzyl)amino)methyl)bicyclo[1.1.1]pentane-1-carboxylic acid;
   (S)-((6-(2-chloro-3-(3-chloro-2-(2-(2-hydroxypropyl)-8-methoxy-1,2,3,4-tetrahydroisoquinolin-6-yl)pyridin-4-yl)phenyl)-2-methoxypyridin-3-yl)methyl)glycine;
   (R)-((6-(2-chloro-3-(3-chloro-2-(2-(2-hydroxypropyl)-8-methoxy-1,2,3,4-tetrahydroisoquinolin-6-yl)pyridin-4-yl)phenyl)-2-methoxypyridin-3-yl)methyl)glycine;
   2-((6-(2-chloro-3-(3-chloro-2-(isoindolin-5-yl)pyridin-4-yl)phenyl)-2-methoxypyridin-3-yl)methyl)-2,6-diazaspiro[3.4]octan-7-one;
   6-(3-chloro-4-(2-chloro-3-(5-((3-hydroxy-3-methylazetidin-1-yl)methyl)-6-methoxypyridin-2-yl)phenyl)pyridin-2-yl)-2-((3-hydroxy-3-methylazetidin-1-yl)methyl)-3-methylpyrrolo[2,1-f][1,2,4]triazin-4(3H)-one;
   6-(3-chloro-4-(2-chloro-3-(5-((((1r,3r)-3-hydroxy-3-methylcyclobutyl)amino)methyl)-6-methoxypyridin-2-yl)phenyl)pyridin-2-yl)-2-((3-hydroxy-3-methylazetidin-1-yl)methyl)-3-methylpyrrolo[2,1-f][1,2,4]triazin-4(3H)-one;
   6-(3-chloro-4-(2-chloro-3-(5-((((1r,3s)-3-hydroxy-3-methylcyclobutyl)amino)methyl)-6-methoxypyridin-2-yl)phenyl)pyridin-2-yl)-2-((3-hydroxy-3-methylazetidin-1-yl)methyl)-3-methylpyrrolo[2,1-f][1,2,4]triazin-4(3H)-one;
   6-(3-chloro-4-(2-chloro-3-(5-((((1s,3r)-3-hydroxy-3-methylcyclobutyl)amino)methyl)-6-methoxypyridin-2-yl)phenyl)pyridin-2-yl)-2-((3-hydroxy-3-methylazetidin-1-yl)methyl)-3-methylpyrrolo[2,1-f][1,2,4]triazin-4(3H)-one;
   6-(3-chloro-4-(2-chloro-3-(5-((((1s,3s)-3-hydroxy-3-methylcyclobutyl)amino)methyl)-6-methoxypyridin-2-yl)phenyl)pyridin-2-yl)-2-((3-hydroxy-3-methylazetidin-1-yl)methyl)-3-methylpyrrolo[2, I-f] [1,2,4]triazin-4(3H)-one;
   6-(3-chloro-4-(2-chloro-3-(5-((isopropylamino)methyl)-6-methoxypyridin-2-yl)phenyl)pyridin-2-yl)-2-((3-hydroxy-3-methylazetidin-1-yl)methyl)-3-methylpyrrolo[2,1-f][1,2,4]triazin-4(3H)-one;
   6-(3-chloro-4-(2-chloro-3-(5-((((1r,4r)-4-hydroxycyclohexyl)amino)methyl)-6-methoxypyridin-2-yl)phenyl)pyridin-2-yl)-2-((3-hydroxy-3-methylazetidin-1-yl)methyl)-3-methylpyrrolo[2,1-f][1,2,4]triazin-4(3H)-one;
   6-(3-chloro-4-(2-chloro-3-(5-((((1r,4s)-4-hydroxycyclohexyl)amino)methyl)-6-methoxypyridin-2-yl)phenyl)pyridin-2-yl)-2-((3-hydroxy-3-methylazetidin-1-yl)methyl)-3-methylpyrrolo[2,1-f][1,2,4]triazin-4(3H)-one;
   6-(3-chloro-4-(2-chloro-3-(5-((((1s,4r)-4-hydroxycyclohexyl)amino)methyl)-6-methoxypyridin-2-yl)phenyl)pyridin-2-yl)-2-((3-hydroxy-3-methylazetidin-1-yl)methyl)-3-methylpyrrolo[2, I-f] [1,2,4]triazin-4(3H)-one;
   6-(3-chloro-4-(2-chloro-3-(5-((((1s,4s)-4-hydroxycyclohexyl)amino)methyl)-6-methoxypyridin-2-yl)phenyl)pyridin-2-yl)-2-((3-hydroxy-3-methylazetidin-1-yl)methyl)-3-methylpyrrolo[2,1-f][1,2,4]triazin-4(3H)-one;
   (S)-5-((((6-(2-chloro-3-(3-chloro-2-(1-methyl-3-(((((S)-oxetan-2-yl)methyl)amino)methyl)-1H-indazol-6-yl)pyridin-4-yl)phenyl)-2-methoxypyridin-3-yl)methyl)amino)methyl)pyrrolidin-2-one;
   (S)-5-((((6-(2-chloro-3-(3-chloro-2-(1-methyl-3-(((((R)-oxetan-2-yl)methyl)amino)methyl)-1H-indazol-6-yl)pyridin-4-yl)phenyl)-2-methoxypyridin-3-yl)methyl)amino)methyl)pyrrolidin-2-one;
   (R)-5-((((6-(2-chloro-3-(3-chloro-2-(1-methyl-3-(((((S)-oxetan-2-yl)methyl)amino)methyl)-1H-indazol-6-yl)pyridin-4-yl)phenyl)-2-methoxypyridin-3-yl)methyl)amino)methyl)pyrrolidin-2-one;
   (R)-5-((((6-(2-chloro-3-(3-chloro-2-(1-methyl-3-(((((R)-oxetan-2-yl)methyl)amino)methyl)-1H-indazol-6-yl)pyridin-4-yl)phenyl)-2-methoxypyridin-3-yl)methyl)amino)methyl)pyrrolidin-2-one;
   (S)-5-((((8-chloro-2-(2-chloro-3-(3-chloro-2-(3-fluoro-5-methoxy-4-(((((S)-5-oxopyrrolidin-2-yl)methyl)amino)methyl)phenyl)pyridin-4-yl)phenyl)-5-methoxyquinolin-6-yl)methyl)amino)methyl)pyrrolidin-2-one;
   (S)-5-((((8-chloro-2-(2-chloro-3-(3-chloro-2-(3-fluoro-5-methoxy-4-(((((R)-5-oxopyrrolidin-2-yl)methyl)amino)methyl)phenyl)pyridin-4-yl)phenyl)-5-methoxyquinolin-6-yl)methyl)amino)methyl)pyrrolidin-2-one;
   (R)-5-((((8-chloro-2-(2-chloro-3-(3-chloro-2-(3-fluoro-5-methoxy-4-(((((S)-5-oxopyrrolidin-2-yl)methyl)amino)methyl)phenyl)pyridin-4-yl)phenyl)-5-methoxyquinolin-6-yl)methyl)amino)methyl)pyrrolidin-2-one;
   (R)-5-((((8-chloro-2-(2-chloro-3-(3-chloro-2-(3-fluoro-5-methoxy-4-(((((R)-5-oxopyrrolidin-2-yl)methyl)amino)methyl)phenyl)pyridin-4-yl)phenyl)-5-methoxyquinolin-6-yl)methyl)amino)methyl)pyrrolidin-2-one;
   (S)-5-((((6-(3-(2-(4-(((2-acetyl-2-azaspiro[3.3]heptan-6-yl)amino)methyl)-3-methoxyphenyl)-3-chloropyridin-4-yl)-2-chlorophenyl)-2-methoxypyridin-3-yl)methyl)amino)methyl)pyrrolidin-2-one;
   (R)-5-((((6-(3-(2-(4-(((2-acetyl-2-azaspiro[3.3]heptan-6-yl)amino)methyl)-3-methoxyphenyl)-3-chloropyridin-4-yl)-2-chlorophenyl)-2-methoxypyridin-3-yl)methyl)amino)methyl)pyrrolidin-2-one;
   (S)-5-(((4-(3-chloro-4-(2-chloro-3-(8-chloro-6-(((2-hydroxyethyl)amino)methyl)-5-methoxyquinolin-2-yl)phenyl)pyridin-2-yl)-2-methoxybenzyl)amino)methyl)pyrrolidin-2-one;
   (R)-5-(((4-(3-chloro-4-(2-chloro-3-(8-chloro-6-(((2-hydroxyethyl)amino)methyl)-5-methoxyquinolin-2-yl)phenyl)pyridin-2-yl)-2-methoxybenzyl)amino)methyl)pyrrolidin-2-one;
   (S)-1-(4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)pyrrolidine-3-carboxylic acid;
   (R)-1-(4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)pyrrolidine-3-carboxylic acid;
   1-(4-(((6-(2-chloro-3-(3-chloro-2-(3-methoxy-4-((methylamino)methyl)phenyl)pyridin-4-yl)phenyl)-2-methoxypyridin-3-yl)methyl)amino)piperidin-1-yl)ethan-1-one;
   (S)-5-(((4-(4-(3-(6-(((1-acetylpiperidin-4-yl)amino)methyl)-8-chloro-5-methoxyquinolin-2-yl)-2-chlorophenyl)-3-chloropyridin-2-yl)-2-methoxybenzyl)amino)methyl)pyrrolidin-2-one;
   (R)-5-(((4-(4-(3-(6-(((1-acetylpiperidin-4-yl)amino)methyl)-8-chloro-5-methoxyquinolin-2-yl)-2-chlorophenyl)-3-chloropyridin-2-yl)-2-methoxybenzyl)amino)methyl)pyrrolidin-2-one;
   5-(3-chloro-4-(2-chloro-3'-fluoro-5'-methoxy-4'-(((((S)-5-oxopyrrolidin-2-yl)methyl)amino)methyl)-[1,1'-biphenyl]-3-yl)pyridin-2-yl)-2-(2-((((S)-5-oxopyrrolidin-2-yl)methyl)amino)ethyl)isoindolin-1-one;
   5-(3-chloro-4-(2-chloro-3'-fluoro-5'-methoxy-4'-(((((S)-5-oxopyrrolidin-2-yl)methyl)amino)methyl)-[1,1'-biphenyl]-3-yl)pyridin-2-yl)-2-(2-((((R)-5-oxopyrrolidin-2-yl)methyl)amino)ethyl)isoindolin-1-one;
   5-(3-chloro-4-(2-chloro-3'-fluoro-5'-methoxy-4'-(((((R)-5-oxopyrrolidin-2-yl)methyl)amino)methyl)-[1,1'-biphenyl]-3-yl)pyridin-2-yl)-2-(2-((((S)-5-oxopyrrolidin-2-yl)methyl)amino)ethyl)isoindolin-1-one;
   5-(3-chloro-4-(2-chloro-3'-fluoro-5'-methoxy-4'-(((((R)-5-oxopyrrolidin-2-yl)methyl)amino)methyl)-[1,1'-biphenyl]-3-yl)pyridin-2-yl)-2-(2-((((R)-5-oxopyrrolidin-2-yl)methyl)amino)ethyl)isoindolin-1-one;
   2-(4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)-2-azaspiro[3.3]heptane-6-carboxylic acid;
   2-(4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)-2,6-diazaspiro[3.4]octan-7-one;
   isopropyl (S)-1-(4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)pyrrolidine-3-carboxylate;
   isopropyl (R)-1-(4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)pyrrolidine-3-carboxylate;
   1-(4-((4-(4-(3-(3-(((1-acetylpiperidin-4-yl)amino)methyl)-1-methyl-1H-pyrrolo[2,3-b]pyridin-6-yl)-2-chlorophenyl)-3-chloropyridin-2-yl)-2-methoxybenzyl)amino)piperidin-1-yl)ethan-1-one;
   (S)-5-((((6-(2-chloro-3-(3-chloro-2-(3-(((2-hydroxyethyl)(methyl)amino)methyl)-1-methyl-1H-indazol-6-yl)pyridin-4-yl)phenyl)-2-methoxypyridin-3-yl)methyl)amino)methyl)pyrrolidin-2-one;
   (R)-5-((((6-(2-chloro-3-(3-chloro-2-(3-(((2-hydroxyethyl)(methyl)amino)methyl)-1-methyl-1H-indazol-6-yl)pyridin-4-yl)phenyl)-2-methoxypyridin-3-yl)methyl)amino)methyl)pyrrolidin-2-one;
   (S)-1-(6-((6-((4-(5-((6-acetyl-2,6-diazaspiro[3.3]heptan-2-yl)methyl)-3'-chloro-6-methoxy-[2,4'-bipyridin]-2'-yl)-2,3-dihydro-1H-inden-1-yl)oxy)-2-methoxy-5-(trifluoromethyl)pyridin-3-yl)methyl)-2,6-diazaspiro[3.3]heptan-2-yl)ethan-1-one;
   (R)-1-(6-((6-((4-(5-((6-acetyl-2,6-diazaspiro[3.3]heptan-2-yl)methyl)-3'-chloro-6-methoxy-[2,4'-bipyridin]-2'-yl)-2,3-dihydro-1H-inden-1-yl)oxy)-2-methoxy-5-(trifluoromethyl)pyridin-3-yl)methyl)-2,6-diazaspiro[3.3]heptan-2-yl)ethan-1-one;
   1-(6-((6-(2-chloro-3-(3-chloro-2-(2-(3-fluoropropyl)-8-methoxy-1,2,3,4-tetrahydroisoquinolin-6-yl)pyridin-4-yl)phenyl)-2-methoxypyridin-3-yl)methyl)-2,6-diazaspiro[3.3]heptan-2-yl)ethan-1-one;
   2-((6-(2-chloro-3-(3-chloro-2-(2-(3-fluoropropyl)-8-methoxy-1,2,3,4-tetrahydroisoquinolin-6-yl)pyridin-4-yl)phenyl)-2-methoxypyridin-3-yl)methyl)-2,6-diazaspiro[3.4]octan-7-one;
   N-((6-(2-chloro-3-(3-chloro-2-(2-(3-fluoropropyl)-8-methoxy-1,2,3,4-tetrahydroisoquinolin-6-yl)pyridin-4-yl)phenyl)-2-methoxypyridin-3-yl)methyl)-2-oxaspiro[3.3]heptan-6-amine;
   (1-((((6-(2-chloro-3-(3-chloro-2-(2-(3-fluoropropyl)-8-methoxy-1,2,3,4-tetrahydroisoquinolin-6-yl)pyridin-4-yl)phenyl)-2-methoxypyridin-3-yl)methyl)amino)methyl)cyclopropyl)methanol;
   (S)-1-(((3'-chloro-2'-((S)-1-((5-((((S)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)propan-2-ol;
   (S)-1-(((3'-chloro-2'-((R)-1-((5-((((R)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)propan-2-ol;
   (S)-1-(((3'-chloro-2'-((R)-1-((5-((((S)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)propan-2-ol;
   (S)-1-(((3'-chloro-2'-((S)-1-((5-((((R)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)propan-2-ol;
   (R)-1-(((3'-chloro-2'-((R)-1-((5-((((R)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)propan-2-ol;
   (R)-1-(((3'-chloro-2'-((R)-1-((5-((((S)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)propan-2-ol;
   (R)-1-(((3'-chloro-2'-((S)-1-((5-((((R)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)propan-2-ol;
   (R)-1-(((3'-chloro-2'-((S)-1-((5-((((S)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)propan-2-ol;
   (S)-1-(4-(((6-((4-(5-(((1-acetylpiperidin-4-yl)amino)methyl)-3'-chloro-6-methoxy-[2,4'-bipyridin]-2'-yl)-2,3-dihydro-1H-inden-1-yl)oxy)-2-methoxy-5-(trifluoromethyl)pyridin-3-yl)methyl)amino)piperidin-1-yl)ethan-1-one;
   (R)-1-(4-(((6-((4-(5-(((1-acetylpiperidin-4-yl)amino)methyl)-3'-chloro-6-methoxy-[2,4'-bipyridin]-2'-yl)-2,3-dihydro-1H-inden-1-yl)oxy)-2-methoxy-5-(trifluoromethyl)pyridin-3-yl)methyl)amino)piperidin-1-yl)ethan-1-one;
   2-((6-(2-chloro-3-(3-chloro-2-((R)-5-((((S)-5-oxopyrrolidin-2-yl)methyl)amino)-5,6,7,8-tetrahydronaphthalen-2-yl)pyridin-4-yl)phenyl)-2-methoxypyridin-3-yl)methyl)-2,6-diazaspiro[3.4]octan-7-one;
   2-((6-(2-chloro-3-(3-chloro-2-((S)-5-((((S)-5-oxopyrrolidin-2-yl)methyl)amino)-5,6,7,8-tetrahydronaphthalen-2-yl)pyridin-4-yl)phenyl)-2-methoxypyridin-3-yl)methyl)-2,6-diazaspiro[3.4]octan-7-one;
   2-((6-(2-chloro-3-(3-chloro-2-((R)-5-((((R)-5-oxopyrrolidin-2-yl)methyl)amino)-5,6,7,8-tetrahydronaphthalen-2-yl)pyridin-4-yl)phenyl)-2-methoxypyridin-3-yl)methyl)-2,6-diazaspiro[3.4]octan-7-one;
   2-((6-(2-chloro-3-(3-chloro-2-((S)-5-((((R)-5-oxopyrrolidin-2-yl)methyl)amino)-5,6,7,8-tetrahydronaphthalen-2-yl)pyridin-4-yl)phenyl)-2-methoxypyridin-3-yl)methyl)-2,6-diazaspiro[3.4]octan-7-one;
   (S)-5-((((R)-6-(3-chloro-4-(2-chloro-3-(5-((((S)-2-hydroxypropyl)amino)methyl)-6-methoxypyridin-2-yl)phenyl)pyridin-2-yl)-1,2,3,4-tetrahydronaphthalen-1-yl)amino)methyl)pyrrolidin-2-one;
   (S)-5-((((R)-6-(3-chloro-4-(2-chloro-3-(5-((((R)-2-hydroxypropyl)amino)methyl)-6-methoxypyridin-2-yl)phenyl)pyridin-2-yl)-1,2,3,4-tetrahydronaphthalen-1-yl)amino)methyl)pyrrolidin-2-one;
   (S)-5-((((S)-6-(3-chloro-4-(2-chloro-3-(5-((((R)-2-hydroxypropyl)amino)methyl)-6-methoxypyridin-2-yl)phenyl)pyridin-2-yl)-1,2,3,4-tetrahydronaphthalen-1-yl)amino)methyl)pyrrolidin-2-one;
   (S)-5-((((S)-6-(3-chloro-4-(2-chloro-3-(5-((((S)-2-hydroxypropyl)amino)methyl)-6-methoxypyridin-2-yl)phenyl)pyridin-2-yl)-1,2,3,4-tetrahydronaphthalen-1-yl)amino)methyl)pyrrolidin-2-one;
   (R)-5-((((R)-6-(3-chloro-4-(2-chloro-3-(5-((((R)-2-hydroxypropyl)amino)methyl)-6-methoxypyridin-2-yl)phenyl)pyridin-2-yl)-1,2,3,4-tetrahydronaphthalen-1-yl)amino)methyl)pyrrolidin-2-one;
   (R)-5-((((R)-6-(3-chloro-4-(2-chloro-3-(5-((((S)-2-hydroxypropyl)amino)methyl)-6-methoxypyridin-2-yl)phenyl)pyridin-2-yl)-1,2,3,4-tetrahydronaphthalen-1-yl)amino)methyl)pyrrolidin-2-one;
   (R)-5-((((S)-6-(3-chloro-4-(2-chloro-3-(5-((((R)-2-hydroxypropyl)amino)methyl)-6-methoxypyridin-2-yl)phenyl)pyridin-2-yl)-1,2,3,4-tetrahydronaphthalen-1-yl)amino)methyl)pyrrolidin-2-one;
   (R)-5-((((S)-6-(3-chloro-4-(2-chloro-3-(5-((((S)-2-hydroxypropyl)amino)methyl)-6-methoxypyridin-2-yl)phenyl)pyridin-2-yl)-1,2,3,4-tetrahydronaphthalen-1-yl)amino)methyl)pyrrolidin-2-one;
   (S)-5-((((R)-6-(3-chloro-4-(2-chloro-3-(6-methoxy-5-((((S)-2-oxotetrahydrofuran-3-yl)amino)methyl)pyridin-2-yl)phenyl)pyridin-2-yl)-1,2,3,4-tetrahydronaphthalen-1-yl)amino)methyl)pyrrolidin-2-one;
   (S)-5-((((S)-6-(3-chloro-4-(2-chloro-3-(6-methoxy-5-((((S)-2-oxotetrahydrofuran-3-yl)amino)methyl)pyridin-2-yl)phenyl)pyridin-2-yl)-1,2,3,4-tetrahydronaphthalen-1-yl)amino)methyl)pyrrolidin-2-one;
   (S)-5-((((S)-6-(3-chloro-4-(2-chloro-3-(6-methoxy-5-((((R)-2-oxotetrahydrofuran-3-yl)amino)methyl)pyridin-2-yl)phenyl)pyridin-2-yl)-1,2,3,4-tetrahydronaphthalen-1-yl)amino)methyl)pyrrolidin-2-one;
   (S)-5-((((R)-6-(3-chloro-4-(2-chloro-3-(6-methoxy-5-((((R)-2-oxotetrahydrofuran-3-yl)amino)methyl)pyridin-2-yl)phenyl)pyridin-2-yl)-1,2,3,4-tetrahydronaphthalen-1-yl)amino)methyl)pyrrolidin-2-one;
   (R)-5-((((R)-6-(3-chloro-4-(2-chloro-3-(6-methoxy-5-((((S)-2-oxotetrahydrofuran-3-yl)amino)methyl)pyridin-2-yl)phenyl)pyridin-2-yl)-1,2,3,4-tetrahydronaphthalen-1-yl)amino)methyl)pyrrolidin-2-one;
   (R)-5-((((S)-6-(3-chloro-4-(2-chloro-3-(6-methoxy-5-((((S)-2-oxotetrahydrofuran-3-yl)amino)methyl)pyridin-2-yl)phenyl)pyridin-2-yl)-1,2,3,4-tetrahydronaphthalen-1-yl)amino)methyl)pyrrolidin-2-one;
   (R)-5-((((S)-6-(3-chloro-4-(2-chloro-3-(6-methoxy-5-((((R)-2-oxotetrahydrofuran-3-yl)amino)methyl)pyridin-2-yl)phenyl)pyridin-2-yl)-1,2,3,4-tetrahydronaphthalen-1-yl)amino)methyl)pyrrolidin-2-one;
   (S)-5-((((S)-6-(3-chloro-4-(2-chloro-3-(6-methoxy-5-((((S)-2-oxotetrahydrofuran-3-yl)amino)methyl)pyridin-2-yl)phenyl)pyridin-2-yl)-1,2,3,4-tetrahydronaphthalen-1-yl)amino)methyl)pyrrolidin-2-one;
   (S)-5-((((6-(2-chloro-3-(3-chloro-2-(8-methoxy-2-(((((S)-5-oxopyrrolidin-2-yl)methyl)amino)methyl)-[1,2,4]triazolo[1,5-a]pyridin-6-yl)pyridin-4-yl)phenyl)-2-methoxypyridin-3-yl)methyl)amino)methyl)pyrrolidin-2-one;
   (S)-5-((((6-(2-chloro-3-(3-chloro-2-(8-methoxy-2-(((((R)-5-oxopyrrolidin-2-yl)methyl)amino)methyl)-[1,2,4]triazolo[1,5-a]pyridin-6-yl)pyridin-4-yl)phenyl)-2-methoxypyridin-3-yl)methyl)amino)methyl)pyrrolidin-2-one;
   (R)-5-((((6-(2-chloro-3-(3-chloro-2-(8-methoxy-2-(((((S)-5-oxopyrrolidin-2-yl)methyl)amino)methyl)-[1,2,4]triazolo[1,5-a]pyridin-6-yl)pyridin-4-yl)phenyl)-2-methoxypyridin-3-yl)methyl)amino)methyl)pyrrolidin-2-one;
   (R)-5-((((6-(2-chloro-3-(3-chloro-2-(8-methoxy-2-(((((R)-5-oxopyrrolidin-2-yl)methyl)amino)methyl)-[1,2,4]triazolo[1,5-a]pyridin-6-yl)pyridin-4-yl)phenyl)-2-methoxypyridin-3-yl)methyl)amino)methyl)pyrrolidin-2-one;
   (S)-5-((((6-(2-chloro-3-(3-chloro-2-(2-((3-hydroxy-3-methylazetidin-1-yl)methyl)-8-methoxy-[1,2,4]triazolo[1,5-a]pyridin-6-yl)pyridin-4-yl)phenyl)-2-methoxypyridin-3-yl)methyl)amino)methyl)pyrrolidin-2-one;
   (R)-5-((((6-(2-chloro-3-(3-chloro-2-(2-((3-hydroxy-3-methylazetidin-1-yl)methyl)-8-methoxy-[1,2,4]triazolo[1,5-a]pyridin-6-yl)pyridin-4-yl)phenyl)-2-methoxypyridin-3-yl)methyl)amino)methyl)pyrrolidin-2-one;
   (S)-5-((((6-(2-chloro-3-(3-chloro-2-(2-((((1r,4r)-4-hydroxycyclohexyl)amino)methyl)-8-methoxy-[1,2,4]triazolo[1,5-a]pyridin-6-yl)pyridin-4-yl)phenyl)-2-methoxypyridin-3-yl)methyl)amino)methyl)pyrrolidin-2-one;
   (S)-5-((((6-(2-chloro-3-(3-chloro-2-(2-((((1s,4s)-4-hydroxycyclohexyl)amino)methyl)-8-methoxy-[1,2,4]triazolo[1,5-a]pyridin-6-yl)pyridin-4-yl)phenyl)-2-methoxypyridin-3-yl)methyl)amino)methyl)pyrrolidin-2-one;
   (S)-5-((((6-(2-chloro-3-(3-chloro-2-(2-((((1s,4r)-4-hydroxycyclohexyl)amino)methyl)-8-methoxy-[1,2,4]triazolo[1,5-a]pyridin-6-yl)pyridin-4-yl)phenyl)-2-methoxypyridin-3-yl)methyl)amino)methyl)pyrrolidin-2-one;
   (S)-5-((((6-(2-chloro-3-(3-chloro-2-(2-((((1r,4s)-4-hydroxycyclohexyl)amino)methyl)-8-methoxy-[1,2,4]triazolo[1,5-a]pyridin-6-yl)pyridin-4-yl)phenyl)-2-methoxypyridin-3-yl)methyl)amino)methyl)pyrrolidin-2-one;
   (R)-5-((((6-(2-chloro-3-(3-chloro-2-(2-((((1r,4s)-4-hydroxycyclohexyl)amino)methyl)-8-methoxy-[1,2,4]triazolo[1,5-a]pyridin-6-yl)pyridin-4-yl)phenyl)-2-methoxypyridin-3-yl)methyl)amino)methyl)pyrrolidin-2-one;
   (R)-5-((((6-(2-chloro-3-(3-chloro-2-(2-((((1s,4s)-4-hydroxycyclohexyl)amino)methyl)-8-methoxy-[1,2,4]triazolo[1,5-a]pyridin-6-yl)pyridin-4-yl)phenyl)-2-methoxypyridin-3-yl)methyl)amino)methyl)pyrrolidin-2-one;
   (R)-5-((((6-(2-chloro-3-(3-chloro-2-(2-((((1s,4r)-4-hydroxycyclohexyl)amino)methyl)-8-methoxy-[1,2,4]triazolo[1,5-a]pyridin-6-yl)pyridin-4-yl)phenyl)-2-methoxypyridin-3-yl)methyl)amino)methyl)pyrrolidin-2-one;
   (R)-5-((((6-(2-chloro-3-(3-chloro-2-(2-((((1r,4r)-4-hydroxycyclohexyl)amino)methyl)-8-methoxy-[1,2,4]triazolo[1,5-a]pyridin-6-yl)pyridin-4-yl)phenyl)-2-methoxypyridin-3-yl)methyl)amino)methyl)pyrrolidin-2-one;
   (S)-1-((6-(3-chloro-4-(2-chloro-3-(6-methoxy-5-((((5-oxopyrrolidin-2-yl)methyl)amino)methyl)pyridin-2-yl)phenyl)pyridin-2-yl)-8-methoxy-[1,2,4]triazolo[1,5-a]pyridin-2-yl)methyl)azetidine-3-carboxylic acid;
   (R)-1-((6-(3-chloro-4-(2-chloro-3-(6-methoxy-5-((((5-oxopyrrolidin-2-yl)methyl)amino)methyl)pyridin-2-yl)phenyl)pyridin-2-yl)-8-methoxy-[1,2,4]triazolo[1,5-a]pyridin-2-yl)methyl)azetidine-3-carboxylic acid;
   (S)-1-(2-(((6-(3-chloro-4-(2-chloro-3-(6-methoxy-5-((((5-oxopyrrolidin-2-yl)methyl)amino)methyl)pyridin-2-yl)phenyl)pyridin-2-yl)-8-methoxy-[1,2,4]triazolo[1,5-a]pyridin-2-yl)methyl)amino)ethyl)cyclopropane-1-carboxylic acid;
   (R)-1-(2-(((6-(3-chloro-4-(2-chloro-3-(6-methoxy-5-((((5-oxopyrrolidin-2-yl)methyl)amino)methyl)pyridin-2-yl)phenyl)pyridin-2-yl)-8-methoxy-[1,2,4]triazolo[1,5-a]pyridin-2-yl)methyl)amino)ethyl)cyclopropane-1-carboxylic acid;
   1-(4-(((6-(3-(2-(3-(((1-acetylpiperidin-4-yl)amino)methyl)-1-methyl-1H-indazol-6-yl)-3-chloropyridin-4-yl)-2-chlorophenyl)-2-methoxypyridin-3-yl)methyl)amino)piperidin-1-yl)ethan-1-one;
   1-(4-((4-(4-(3-(3-(((1-acetylpiperidin-4-yl)(methyl)amino)methyl)-1-methyl-1H-pyrrolo[2,3-b]pyridin-6-yl)-2-chlorophenyl)-3-chloropyridin-2-yl)-2-methoxybenzyl)amino)piperidin-1-yl)ethan-1-one;
   ((6-(2-chloro-3-(3-chloro-2-((R)-5-((((S)-5-oxopyrrolidin-2-yl)methyl)amino)-5,6,7, 8-tetrahydronaphthalen-2-yl)pyridin-4-yl)phenyl)-2-methoxypyridin-3-yl)methyl)-L-homoserine;
   ((6-(2-chloro-3-(3-chloro-2-((R)-5-((((R)-5-oxopyrrolidin-2-yl)methyl)amino)-5,6,7,8-tetrahydronaphthalen-2-yl)pyridin-4-yl)phenyl)-2-methoxypyridin-3-yl)methyl)-L-homoserine;
   ((6-(2-chloro-3-(3-chloro-2-((S)-5-((((S)-5-oxopyrrolidin-2-yl)methyl)amino)-5,6,7,8-tetrahydronaphthalen-2-yl)pyridin-4-yl)phenyl)-2-methoxypyridin-3-yl)methyl)-L-homoserine;
   ((6-(2-chloro-3-(3-chloro-2-((S)-5-((((R)-5-oxopyrrolidin-2-yl)methyl)amino)-5,6,7,8-tetrahydronaphthalen-2-yl)pyridin-4-yl)phenyl)-2-methoxypyridin-3-yl)methyl)-L-homoserine;
   ((6-(2-chloro-3-(3-chloro-2-((R)-5-((((R)-5-oxopyrrolidin-2-yl)methyl)amino)-5,6,7,8-tetrahydronaphthalen-2-yl)pyridin-4-yl)phenyl)-2-methoxypyridin-3-yl)methyl)-D-homoserine;
   ((6-(2-chloro-3-(3-chloro-2-((S)-5-((((S)-5-oxopyrrolidin-2-yl)methyl)amino)-5,6,7,8-tetrahydronaphthalen-2-yl)pyridin-4-yl)phenyl)-2-methoxypyridin-3-yl)methyl)-D-homoserine;
   ((6-(2-chloro-3-(3-chloro-2-((S)-5-((((R)-5-oxopyrrolidin-2-yl)methyl)amino)-5,6,7,8-tetrahydronaphthalen-2-yl)pyridin-4-yl)phenyl)-2-methoxypyridin-3-yl)methyl)-D-homoserine;
   ((6-(2-chloro-3-(3-chloro-2-((R)-5-((((S)-5-oxopyrrolidin-2-yl)methyl)amino)-5,6,7, 8-tetrahydronaphthalen-2-yl)pyridin-4-yl)phenyl)-2-methoxypyridin-3-yl)methyl)-D-homoserine;
   (5S,5'S)-5,5'-(((3,3'-dichloro-[4,4'-bipyridine]-2,2'-diyl)bis(8-methoxy-3,4-dihydroisoquinoline-6,2(1H)-diyl))bis(methylene))bis(pyrrolidin-2-one);
   (5S,5'R)-5,5'-(((3,3'-dichloro-[4,4'-bipyridine]-2,2'-diyl)bis(8-methoxy-3,4-dihydroisoquinoline-6,2(1H)-diyl))bis(methylene))bis(pyrrolidin-2-one);
   (5R,5'S)-5,5'-(((3,3'-dichloro-[4,4'-bipyridine]-2,2'-diyl)bis(8-methoxy-3,4-dihydroisoquinoline-6,2(1H)-diyl))bis(methylene))bis(pyrrolidin-2-one);
   (5R,5'R)-5,5'-(((3,3'-dichloro-[4,4'-bipyridine]-2,2'-diyl)bis(8-methoxy-3,4-dihydroisoquinoline-6,2(1H)-diyl))bis(methylene))bis(pyrrolidin-2-one);
   methyl ((3-chloro-4-(2-chloro-3-(6-methoxy-5-(((((S)-5-oxopyrrolidin-2-yl)methyl)amino)methyl)pyridin-2-yl)phenyl)-5'-methoxy-[2,3'-bipyridin]-6'-yl)methyl)-L-homoserinate;
   methyl ((3-chloro-4-(2-chloro-3-(6-methoxy-5-(((((R)-5-oxopyrrolidin-2-yl)methyl)amino)methyl)pyridin-2-yl)phenyl)-5'-methoxy-[2,3'-bipyridin]-6'-yl)methyl)-L-homoserinate;
   methyl ((3-chloro-4-(2-chloro-3-(6-methoxy-5-(((((R)-5-oxopyrrolidin-2-yl)methyl)amino)methyl)pyridin-2-yl)phenyl)-5'-methoxy-[2,3'-bipyridin]-6'-yl)methyl)-D-homoserinate;
   methyl ((3-chloro-4-(2-chloro-3-(6-methoxy-5-(((((S)-5-oxopyrrolidin-2-yl)methyl)amino)methyl)pyridin-2-yl)phenyl)-5'-methoxy-[2,3'-bipyridin]-6'-yl)methyl)-D-homoserinate;
   (S)-5-((((6-(2-chloro-3-(3-chloro-5'-methoxy-6'-((((S)-2-oxotetrahydrofuran-3-yl)amino)methyl)-[2,3'-bipyridin]-4-yl)phenyl)-2-methoxypyridin-3-yl)methyl)amino)methyl)pyrrolidin-2-one;
   (S)-5-((((6-(2-chloro-3-(3-chloro-5'-methoxy-6'-((((R)-2-oxotetrahydrofuran-3-yl)amino)methyl)-[2,3'-bipyridin]-4-yl)phenyl)-2-methoxypyridin-3-yl)methyl)amino)methyl)pyrrolidin-2-one;
   (R)-5-((((6-(2-chloro-3-(3-chloro-5'-methoxy-6'-((((S)-2-oxotetrahydrofuran-3-yl)amino)methyl)-[2,3'-bipyridin]-4-yl)phenyl)-2-methoxypyridin-3-yl)methyl)amino)methyl)pyrrolidin-2-one;
   (R)-5-((((6-(2-chloro-3-(3-chloro-5'-methoxy-6'-((((R)-2-oxotetrahydrofuran-3-yl)amino)methyl)-[2,3'-bipyridin]-4-yl)phenyl)-2-methoxypyridin-3-yl)methyl)amino)methyl)pyrrolidin-2-one;
   (S)-5-((((6-(((S)-4-(3-chloro-2-(3-methoxy-4-(((((S)-5-oxopyrrolidin-2-yl)methyl)amino)methyl)phenyl)pyridin-4-yl)-2,3-dihydro-1H-inden-1-yl)oxy)-2-methoxy-5-(trifluoromethyl)pyridin-3-yl)methyl)amino)methyl)pyrrolidin-2-one;
   (S)-5-((((6-(((S)-4-(3-chloro-2-(3-methoxy-4-(((((R)-5-oxopyrrolidin-2-yl)methyl)amino)methyl)phenyl)pyridin-4-yl)-2,3-dihydro-1H-inden-1-yl)oxy)-2-methoxy-5-(trifluoromethyl)pyridin-3-yl)methyl)amino)methyl)pyrrolidin-2-one;
   (S)-5-((((6-(((R)-4-(3-chloro-2-(3-methoxy-4-(((((S)-5-oxopyrrolidin-2-yl)methyl)amino)methyl)phenyl)pyridin-4-yl)-2,3-dihydro-1H-inden-1-yl)oxy)-2-methoxy-5-(trifluoromethyl)pyridin-3-yl)methyl)amino)methyl)pyrrolidin-2-one;
   (S)-5-((((6-(((S)-4-(3-chloro-2-(3-methoxy-4-(((((R)-5-oxopyrrolidin-2-yl)methyl)amino)methyl)phenyl)pyridin-4-yl)-2,3-dihydro-1H-inden-1-yl)oxy)-2-methoxy-5-(trifluoromethyl)pyridin-3-yl)methyl)amino)methyl)pyrrolidin-2-one;
   (R)-5-((((6-(((S)-4-(3-chloro-2-(3-methoxy-4-(((((S)-5-oxopyrrolidin-2-yl)methyl)amino)methyl)phenyl)pyridin-4-yl)-2,3-dihydro-1H-inden-1-yl)oxy)-2-methoxy-5-(trifluoromethyl)pyridin-3-yl)methyl)amino)methyl)pyrrolidin-2-one;
   (R)-5-((((6-(((S)-4-(3-chloro-2-(3-methoxy-4-(((((R)-5-oxopyrrolidin-2-yl)methyl)amino)methyl)phenyl)pyridin-4-yl)-2,3-dihydro-1H-inden-1-yl)oxy)-2-methoxy-5-(trifluoromethyl)pyridin-3-yl)methyl)amino)methyl)pyrrolidin-2-one;
   (R)-5-((((6-(((R)-4-(3-chloro-2-(3-methoxy-4-(((((S)-5-oxopyrrolidin-2-yl)methyl)amino)methyl)phenyl)pyridin-4-yl)-2,3-dihydro-1H-inden-1-yl)oxy)-2-methoxy-5-(trifluoromethyl)pyridin-3-yl)methyl)amino)methyl)pyrrolidin-2-one;
   (R)-5-((((6-(((S)-4-(3-chloro-2-(3-methoxy-4-(((((R)-5-oxopyrrolidin-2-yl)methyl)amino)methyl)phenyl)pyridin-4-yl)-2,3-dihydro-1H-inden-1-yl)oxy)-2-methoxy-5-(trifluoromethyl)pyridin-3-yl)methyl)amino)methyl)pyrrolidin-2-one;
   (S)-1-(((6-(((S)-4-(3-chloro-2-(4-((((S)-2-hydroxypropyl)amino)methyl)-3-methoxyphenyl)pyridin-4-yl)-2,3-dihydro-1H-inden-1-yl)oxy)-2-methoxy-5-(trifluoromethyl)pyridin-3-yl)methyl)amino)propan-2-ol;
   (S)-1-(((6-(((S)-4-(3-chloro-2-(4-((((R)-2-hydroxypropyl)amino)methyl)-3-methoxyphenyl)pyridin-4-yl)-2,3-dihydro-1H-inden-1-yl)oxy)-2-methoxy-5-(trifluoromethyl)pyridin-3-yl)methyl)amino)propan-2-ol;
   (S)-1-(((6-(((R)-4-(3-chloro-2-(4-((((S)-2-hydroxypropyl)amino)methyl)-3-methoxyphenyl)pyridin-4-yl)-2,3-dihydro-1H-inden-1-yl)oxy)-2-methoxy-5-(trifluoromethyl)pyridin-3-yl)methyl)amino)propan-2-ol;
   (S)-1-(((6-(((R)-4-(3-chloro-2-(4-((((R)-2-hydroxypropyl)amino)methyl)-3-methoxyphenyl)pyridin-4-yl)-2,3-dihydro-1H-inden-1-yl)oxy)-2-methoxy-5-(trifluoromethyl)pyridin-3-yl)methyl)amino)propan-2-ol;
   (R)-1-(((6-(((S)-4-(3-chloro-2-(4-((((S)-2-hydroxypropyl)amino)methyl)-3-methoxyphenyl)pyridin-4-yl)-2,3-dihydro-1H-inden-1-yl)oxy)-2-methoxy-5-(trifluoromethyl)pyridin-3-yl)methyl)amino)propan-2-ol;
   (R)-1-(((6-(((S)-4-(3-chloro-2-(4-((((R)-2-hydroxypropyl)amino)methyl)-3-methoxyphenyl)pyridin-4-yl)-2,3-dihydro-1H-inden-1-yl)oxy)-2-methoxy-5-(trifluoromethyl)pyridin-3-yl)methyl)amino)propan-2-ol;
   (R)-1-(((6-(((R)-4-(3-chloro-2-(4-((((S)-2-hydroxypropyl)amino)methyl)-3-methoxyphenyl)pyridin-4-yl)-2,3-dihydro-1H-inden-1-yl)oxy)-2-methoxy-5-(trifluoromethyl)pyridin-3-yl)methyl)amino)propan-2-ol;
   (R)-1-(((6-(((R)-4-(3-chloro-2-(4-((((R)-2-hydroxypropyl)amino)methyl)-3-methoxyphenyl)pyridin-4-yl)-2,3-dihydro-1H-inden-1-yl)oxy)-2-methoxy-5-(trifluoromethyl)pyridin-3-yl)methyl)amino)propan-2-ol;
   1-(4-(((6-(3-(2-(4-(((1-acetylpiperidin-4-yl)amino)methyl)-3-methoxyphenyl)-3-methylpyridin-4-yl)-2-chlorophenyl)-2-methoxypyridin-3-yl)methyl)amino)piperidin-1-yl)ethan-1-one;
   methyl (S)-3-(6-(3-chloro-4-(2-chloro-3-(5-(((2-hydroxypropyl)amino)methyl)-6-methoxypyridin-2-yl)phenyl)pyridin-2-yl)-8-methoxy-3,4-dihydroisoquinolin-2(1H)-yl)propanoate;
   methyl (R)-3-(6-(3-chloro-4-(2-chloro-3-(5-(((2-hydroxypropyl)amino)methyl)-6-methoxypyridin-2-yl)phenyl)pyridin-2-yl)-8-methoxy-3,4-dihydroisoquinolin-2(1H)-yl)propanoate;
   methyl 3-(6-(3-chloro-4-(2-chloro-3-(6-methoxy-5-((7-oxo-2,6-diazaspiro[3.4]octan-2-yl)methyl)pyridin-2-yl)phenyl)pyridin-2-yl)-8-methoxy-3,4-dihydroisoquinolin-2(1H)-yl)propanoate;
   (S)-1-(((6-(2-chloro-3-(2-(4-((((S)-2-hydroxypropyl)amino)methyl)-3-methoxyphenyl)-3-methylpyridin-4-yl)phenyl)-2-methoxypyridin-3-yl)methyl)amino)propan-2-ol;
   (S)-1-(((6-(2-chloro-3-(2-(4-((((R)-2-hydroxypropyl)amino)methyl)-3-methoxyphenyl)-3-methylpyridin-4-yl)phenyl)-2-methoxypyridin-3-yl)methyl)amino)propan-2-ol;
   (R)-1-(((6-(2-chloro-3-(2-(4-((((S)-2-hydroxypropyl)amino)methyl)-3-methoxyphenyl)-3-methylpyridin-4-yl)phenyl)-2-methoxypyridin-3-yl)methyl)amino)propan-2-ol;
   (R)-1-(((6-(2-chloro-3-(2-(4-((((R)-2-hydroxypropyl)amino)methyl)-3-methoxyphenyl)-3-methylpyridin-4-yl)phenyl)-2-methoxypyridin-3-yl)methyl)amino)propan-2-ol;
   (S)-2-(6-(3-chloro-4-(2-chloro-3-(5-(((2-hydroxypropyl)amino)methyl)-6-methoxypyridin-2-yl)phenyl)pyridin-2-yl)-8-methoxy-3,4-dihydroisoquinolin-2(1H)-yl)acetamide;
   (R)-2-(6-(3-chloro-4-(2-chloro-3-(5-(((2-hydroxypropyl)amino)methyl)-6-methoxypyridin-2-yl)phenyl)pyridin-2-yl)-8-methoxy-3,4-dihydroisoquinolin-2(1H)-yl)acetamide;
   1-(6-(2-chloro-3-(3-chloro-2-(1-methyl-3-((methylamino)methyl)-1H-indol-6-yl)pyridin-4-yl)phenyl)-2-methoxypyridin-3-yl)-N-methylmethanamine;
   1-(4-(((6-(3-(2-(3-(((1-acetylpiperidin-4-yl)amino)methyl)-1-methyl-1H-indol-6-yl)-3-chloropyridin-4-yl)-2-chlorophenyl)-2-methoxypyridin-3-yl)methyl)amino)piperidin-1-yl)ethan-1-one;
   isopropyl (S)-1-(4-(3-chloro-4-(2-chloro-3-(6-methoxy-5-((methylamino)methyl)pyridin-2-yl)phenyl)pyridin-2-yl)-2-methoxybenzyl)pyrrolidine-3-carboxylate;
   isopropyl (R)-1-(4-(3-chloro-4-(2-chloro-3-(6-methoxy-5-((methylamino)methyl)pyridin-2-yl)phenyl)pyridin-2-yl)-2-methoxybenzyl)pyrrolidine-3-carboxylate;
   isopropyl 3-((4-(3-chloro-4-(2-chloro-3-(6-methoxy-5-((methylamino)methyl)pyridin-2-yl)phenyl)pyridin-2-yl)-2-methoxybenzyl)amino)propanoate;
   isopropyl (S)-4-((4-(3-chloro-4-(2-chloro-3-(6-methoxy-5-((methylamino)methyl)pyridin-2-yl)phenyl)pyridin-2-yl)-2-methoxybenzyl)amino)-3-hydroxybutanoate;
   isopropyl (R)-4-((4-(3-chloro-4-(2-chloro-3-(6-methoxy-5-((methylamino)methyl)pyridin-2-yl)phenyl)pyridin-2-yl)-2-methoxybenzyl)amino)-3-hydroxybutanoate;
   isopropyl 2-(4-(3-chloro-4-(2-chloro-3-(6-methoxy-5-((methylamino)methyl)pyridin-2-yl)phenyl)pyridin-2-yl)-2-methoxybenzyl)-2-azaspiro[3.3]heptane-6-carboxylate;
   1-(4-(((6-(3-(2-(4-(((1-acetylpiperidin-4-yl)amino)methyl)-3-fluorophenyl)-3-chloropyridin-4-yl)-2-chlorophenyl)-2-methoxypyridin-3-yl)methyl)amino)piperidin-1-yl)ethan-1-one;
   1-(6-(2-chloro-3-(3-chloro-2-(3-fluoro-4-((methylamino)methyl)phenyl)pyridin-4-yl)phenyl)-2-methoxypyridin-3-yl)-N-methylmethanamine;
   2-((6-(2-chloro-3-(3-chloro-2-(3-fluoro-4-((7-oxo-2,6-diazaspiro[3.4]octan-2-yl)methyl)phenyl)pyridin-4-yl)phenyl)-2-methoxypyridin-3-yl)methyl)-2,6-diazaspiro[3.4]octan-7-one;
   1-(4-(((6-(3-(2-(4-(((1-acetylpiperidin-4-yl)amino)methyl)-3-fluoro-5-methoxyphenyl)-3-chloropyridin-4-yl)-2-chlorophenyl)-2-methoxypyridin-3-yl)methyl)amino)piperidin-1-yl)ethan-1-one;
   1-(6-(2-chloro-3-(3-chloro-2-(3-fluoro-5-methoxy-4-((methylamino)methyl)phenyl)pyridin-4-yl)phenyl)-2-methoxypyridin-3-yl)-N-methylmethanamine;
   2-(4-(3-chloro-4-(2-chloro-3-(6-methoxy-5-((7-oxo-2,6-diazaspiro[3.4]octan-2-yl)methyl)pyridin-2-yl)phenyl)pyridin-2-yl)-2-fluoro-6-methoxybenzyl)-2,6-diazaspiro[3.4]octan-7-one;
   (S)-2-((6-(3-chloro-4-(2-chloro-3-(6-methoxy-5-((((5-oxopyrrolidin-2-yl)methyl)amino)methyl)pyridin-2-yl)phenyl)pyridin-2-yl)-1-methyl-1H-indazol-3-yl)methyl)-2,6-diazaspiro[3.4]octan-7-one;
   (R)-2-((6-(3-chloro-4-(2-chloro-3-(6-methoxy-5-((((5-oxopyrrolidin-2-yl)methyl)amino)methyl)pyridin-2-yl)phenyl)pyridin-2-yl)-1-methyl-1H-indazol-3-yl)methyl)-2,6-diazaspiro[3.4]octan-7-one;
   (S)-5-((((6-(3-(2-(2-((1-acetylpiperidin-4-yl)methyl)-8-chloro-1,2,3,4-tetrahydroisoquinolin-6-yl)-3-chloropyridin-4-yl)-2-chlorophenyl)-2-methoxypyridin-3-yl)methyl)amino)methyl)pyrrolidin-2-one;
   (R)-5-((((6-(3-(2-(2-((1-acetylpiperidin-4-yl)methyl)-8-chloro-1,2,3,4-tetrahydroisoquinolin-6-yl)-3-chloropyridin-4-yl)-2-chlorophenyl)-2-methoxypyridin-3-yl)methyl)amino)methyl)pyrrolidin-2-one;
   6-(2-chloro-3-(3-chloro-2-(3-methoxy-4-(((((S)-5-oxopyrrolidin-2-yl)methyl)amino)methyl)phenyl)pyridin-4-yl)phenyl)-2-(2-((((S)-5-oxopyrrolidin-2-yl)methyl)amino)ethyl)-1,2-dihydro-3H-pyrrolo[3,4-c]pyridin-3-one;
   6-(2-chloro-3-(3-chloro-2-(3-methoxy-4-(((((S)-5-oxopyrrolidin-2-yl)methyl)amino)methyl)phenyl)pyridin-4-yl)phenyl)-2-(2-((((R)-5-oxopyrrolidin-2-yl)methyl)amino)ethyl)-1,2-dihydro-3H-pyrrolo[3,4-c]pyridin-3-one;
   6-(2-chloro-3-(3-chloro-2-(3-methoxy-4-(((((R)-5-oxopyrrolidin-2-yl)methyl)amino)methyl)phenyl)pyridin-4-yl)phenyl)-2-(2-((((S)-5-oxopyrrolidin-2-yl)methyl)amino)ethyl)-1,2-dihydro-3H-pyrrolo[3,4-c]pyridin-3-one;
   6-(2-chloro-3-(3-chloro-2-(3-methoxy-4-(((((R)-5-oxopyrrolidin-2-yl)methyl)amino)methyl)phenyl)pyridin-4-yl)phenyl)-2-(2-((((R)-5-oxopyrrolidin-2-yl)methyl)amino)ethyl)-1,2-dihydro-3H-pyrrolo[3,4-c]pyridin-3-one;
   (S)-5-((((6-(2-chloro-3-(3-chloro-2-(3-methoxy-4-(((((S)-5-oxopyrrolidin-2-yl)methyl)amino)methyl)phenyl)pyridin-4-yl)phenyl)imidazo[1,2-a]pyrazin-2-yl)methyl)amino)methyl)pyrrolidin-2-one;
   (S)-5-((((6-(2-chloro-3-(3-chloro-2-(3-methoxy-4-(((((R)-5-oxopyrrolidin-2-yl)methyl)amino)methyl)phenyl)pyridin-4-yl)phenyl)imidazo[1,2-a]pyrazin-2-yl)methyl)amino)methyl)pyrrolidin-2-one;
   (R)-5-((((6-(2-chloro-3-(3-chloro-2-(3-methoxy-4-(((((S)-5-oxopyrrolidin-2-yl)methyl)amino)methyl)phenyl)pyridin-4-yl)phenyl)imidazo[1,2-a]pyrazin-2-yl)methyl)amino)methyl)pyrrolidin-2-one;
   (R)-5-((((6-(2-chloro-3-(3-chloro-2-(3-methoxy-4-(((((R)-5-oxopyrrolidin-2-yl)methyl)amino)methyl)phenyl)pyridin-4-yl)phenyl)imidazo[1,2-a]pyrazin-2-yl)methyl)amino)methyl)pyrrolidin-2-one;
   (S)-2-(4-(3-chloro-4-(1-((6-methoxy-5-((7-oxo-2,6-diazaspiro[3.4]octan-2-yl)methyl)-3-(trifluoromethyl)pyridin-2-yl)oxy)-2,3-dihydro-1H-inden-4-yl)pyridin-2-yl)-2-methoxybenzyl)-2,6-diazaspiro[3.4]octan-7-one;
   (R)-2-(4-(3-chloro-4-(1-((6-methoxy-5-((7-oxo-2,6-diazaspiro[3.4]octan-2-yl)methyl)-3-(trifluoromethyl)pyridin-2-yl)oxy)-2,3-dihydro-1H-inden-4-yl)pyridin-2-yl)-2-methoxybenzyl)-2,6-diazaspiro[3.4]octan-7-one;
   (S)-3-(6-(3-chloro-4-(2-chloro-3-(5-(((2-hydroxypropyl)amino)methyl)-6-methoxypyridin-2-yl)phenyl)pyridin-2-yl)-8-methoxy-3,4-dihydroisoquinolin-2(1H)-yl)propanoic acid;
   (R)-3-(6-(3-chloro-4-(2-chloro-3-(5-(((2-hydroxypropyl)amino)methyl)-6-methoxypyridin-2-yl)phenyl)pyridin-2-yl)-8-methoxy-3,4-dihydroisoquinolin-2(1H)-yl)propanoic acid;
   3-(6-(3-chloro-4-(2-chloro-3-(6-methoxy-5-((7-oxo-2,6-diazaspiro[3.4]octan-2-yl)methyl)pyridin-2-yl)phenyl)pyridin-2-yl)-8-methoxy-3,4-dihydroisoquinolin-2(1H)-yl)propanoic acid;
   methyl 1-((6-(3-chloro-4-(2-chloro-3-(6-methoxy-5-((7-oxo-2,6-diazaspiro[3.4]octan-2-yl)methyl)pyridin-2-yl)phenyl)pyridin-2-yl)-8-methoxy-3,4-dihydroisoquinolin-2(1H)-yl)methyl)cyclopropane-1-carboxylate;
   2-((6-(2-chloro-3-(3-chloro-2-(2-(3-fluoro-2-hydroxypropyl)-8-methoxy-1,2,3,4-tetrahydroisoquinolin-6-yl)pyridin-4-yl)phenyl)-2-methoxypyridin-3-yl)methyl)-2,6-diazaspiro[3.4]octan-7-one;
   (S)-2-((6-(2-chloro-3-(3-chloro-2-(2-(3-fluoro-2-hydroxypropyl)-8-methoxy-1,2,3,4-tetrahydroisoquinolin-6-yl)pyridin-4-yl)phenyl)-2-methoxypyridin-3-yl)methyl)-2,6-diazaspiro[3.4]octan-7-one;
   (R)-2-((6-(2-chloro-3-(3-chloro-2-(2-(3-fluoro-2-hydroxypropyl)-8-methoxy-1,2,3,4-tetrahydroisoquinolin-6-yl)pyridin-4-yl)phenyl)-2-methoxypyridin-3-yl)methyl)-2,6-diazaspiro[3.4]octan-7-one;
   1-((6-(3-chloro-4-(2-chloro-3-(6-methoxy-5-((7-oxo-2,6-diazaspiro[3.4]octan-2-yl)methyl)pyridin-2-yl)phenyl)pyridin-2-yl)-8-methoxy-3,4-dihydroisoquinolin-2(1H)-yl)methyl)cyclopropane-1-carboxylic acid;
   N-((6-(2-chloro-3-(3-chloro-2-(3-methoxy-4-(((tetrahydro-2H-pyran-4-yl)amino)methyl)phenyl)pyridin-4-yl)phenyl)-2-methoxypyridin-3-yl)methyl)tetrahydro-2H-pyran-4-amine;
   4-(((6-(2-chloro-3-(3-chloro-2-(4-(((1,1-dioxidotetrahydro-2H-thiopyran-4-yl)amino)methyl)-3-methoxyphenyl)pyridin-4-yl)phenyl)-2-methoxypyridin-3-yl)methyl)amino)tetrahydro-2H-thiopyran 1,1-dioxide;
   1-(3-(((6-(3-(2-(4-(((1-acetylazetidin-3-yl)amino)methyl)-3-methoxyphenyl)-3-chloropyridin-4-yl)-2-chlorophenyl)-2-methoxypyridin-3-yl)methyl)amino)azetidin-1-yl)ethan-1-one;
   (S)-1-(((6-(2-chloro-3-(3-chloro-2-(4-((((S)-2-hydroxypropyl)amino)methyl)-3-methoxyphenyl)pyridin-4-yl)phenyl)-2-methoxypyridin-3-yl)methyl)amino)propan-2-ol;
   (R)-1-(((6-(2-chloro-3-(3-chloro-2-(4-((((S)-2-hydroxypropyl)amino)methyl)-3-methoxyphenyl)pyridin-4-yl)phenyl)-2-methoxypyridin-3-yl)methyl)amino)propan-2-ol;
   (S)-1-(((6-(2-chloro-3-(3-chloro-2-(4-((((R)-2-hydroxypropyl)amino)methyl)-3-methoxyphenyl)pyridin-4-yl)phenyl)-2-methoxypyridin-3-yl)methyl)amino)propan-2-ol;
   (R)-1-(((6-(2-chloro-3-(3-chloro-2-(4-((((R)-2-hydroxypropyl)amino)methyl)-3-methoxyphenyl)pyridin-4-yl)phenyl)-2-methoxypyridin-3-yl)methyl)amino)propan-2-ol;
   2-((4-(3-chloro-4-(2-chloro-3-(6-methoxy-5-(((5-oxo-6-azaspiro[3.4]octan-2-yl)amino)methyl)pyridin-2-yl)phenyl)pyridin-2-yl)-2-methoxybenzyl)amino)-6-azaspiro[3.4]octan-5-one;
   1-(2-(((6-(2-chloro-3-(3-chloro-2-(3-methoxy-4-(((2-(2-oxopyrrolidin-1-yl)ethyl)amino)methyl)phenyl)pyridin-4-yl)phenyl)-2-methoxypyridin-3-yl)methyl)amino)ethyl)pyrrolidin-2-one;
   (S)-2-((3'-chloro-6-methoxy-2'-(1-((6-methoxy-5-((7-oxo-2,6-diazaspiro[3.4]octan-2-yl)methyl)-3-(trifluoromethyl)pyrazin-2-yl)amino)-2,3-dihydro-1H-inden-4-yl)-[2,4'-bipyridin]-5-yl)methyl)-2,6-diazaspiro[3.4]octan-7-one;
   (R)-2-((3'-chloro-6-methoxy-2'-(1-((6-methoxy-5-((7-oxo-2,6-diazaspiro[3.4]octan-2-yl)methyl)-3-(trifluoromethyl)pyrazin-2-yl)amino)-2,3-dihydro-1H-inden-4-yl)-[2,4'-bipyridin]-5-yl)methyl)-2,6-diazaspiro[3.4]octan-7-one;
   (S)-1-(6-((5-((4-(5-((6-acetyl-2,6-diazaspiro[3.3]heptan-2-yl)methyl)-3'-chloro-6-methoxy-[2,4'-bipyridin]-2'-yl)-2,3-dihydro-1H-inden-1-yl)amino)-3-methoxy-6-(trifluoromethyl)pyrazin-2-yl)methyl)-2,6-diazaspiro[3.3]heptan-2-yl)ethan-1-one;
   (R)-1-(6-((5-((4-(5-((6-acetyl-2,6-diazaspiro[3.3]heptan-2-yl)methyl)-3'-chloro-6-methoxy-[2,4'-bipyridin]-2'-yl)-2,3-dihydro-1H-inden-1-yl)amino)-3-methoxy-6-(trifluoromethyl)pyrazin-2-yl)methyl)-2,6-diazaspiro[3.3]heptan-2-yl)ethan-1-one;
   (S)-2-(4-(3-chloro-4-(1-((6-methoxy-5-((7-oxo-2,6-diazaspiro[3.4]octan-2-yl)methyl)-3-(trifluoromethyl)pyridin-2-yl)oxy)-2,3-dihydro-1H-inden-4-yl)pyridin-2-yl)-2-fluoro-6-methoxybenzyl)-2,6-diazaspiro[3.4]octan-7-one;
   (R)-2-(4-(3-chloro-4-(1-((6-methoxy-5-((7-oxo-2,6-diazaspiro[3.4]octan-2-yl)methyl)-3-(trifluoromethyl)pyridin-2-yl)oxy)-2,3-dihydro-1H-inden-4-yl)pyridin-2-yl)-2-fluoro-6-methoxybenzyl)-2,6-diazaspiro[3.4]octan-7-one;
   (S)-5-((((6-(2-chloro-3-(3-chloro-2-(8-chloro-2-(oxetan-3-ylmethyl)-1,2,3,4-tetrahydroisoquinolin-6-yl)pyridin-4-yl)phenyl)-2-methoxypyridin-3-yl)methyl)amino)methyl)pyrrolidin-2-one;
   (R)-5-((((6-(2-chloro-3-(3-chloro-2-(8-chloro-2-(oxetan-3-ylmethyl)-1,2,3,4-tetrahydroisoquinolin-6-yl)pyridin-4-yl)phenyl)-2-methoxypyridin-3-yl)methyl)amino)methyl)pyrrolidin-2-one;
   (S)-5-((((6-(3-(2-(3-((6-acetyl-2,6-diazaspiro[3.3]heptan-2-yl)methyl)-1-methyl-1H-indazol-6-yl)-3-chloropyridin-4-yl)-2-chlorophenyl)-2-methoxypyridin-3-yl)methyl)amino)methyl)pyrrolidin-2-one;
   (R)-5-((((6-(3-(2-(3-((6-acetyl-2,6-diazaspiro[3.3]heptan-2-yl)methyl)-1-methyl-1H-indazol-6-yl)-3-chloropyridin-4-yl)-2-chlorophenyl)-2-methoxypyridin-3-yl)methyl)amino)methyl)pyrrolidin-2-one;
   (S)-1-((6-(3-chloro-4-(2-chloro-3-(6-methoxy-5-(((((S)-5-oxopyrrolidin-2-yl)methyl)amino)methyl)pyridin-2-yl)phenyl)pyridin-2-yl)-1-methyl-1H-indazol-3-yl)methyl)pyrrolidine-3-carboxylic acid;
   (S)-1-((6-(3-chloro-4-(2-chloro-3-(6-methoxy-5-(((((R)-5-oxopyrrolidin-2-yl)methyl)amino)methyl)pyridin-2-yl)phenyl)pyridin-2-yl)-1-methyl-1H-indazol-3-yl)methyl)pyrrolidine-3-carboxylic acid;
   (R)-1-((6-(3-chloro-4-(2-chloro-3-(6-methoxy-5-(((((S)-5-oxopyrrolidin-2-yl)methyl)amino)methyl)pyridin-2-yl)phenyl)pyridin-2-yl)-1-methyl-1H-indazol-3-yl)methyl)pyrrolidine-3-carboxylic acid;
   (R)-1-((6-(3-chloro-4-(2-chloro-3-(6-methoxy-5-(((((R)-5-oxopyrrolidin-2-yl)methyl)amino)methyl)pyridin-2-yl)phenyl)pyridin-2-yl)-1-methyl-1H-indazol-3-yl)methyl)pyrrolidine-3-carboxylic acid;
   (S)-5-((((6-(2-chloro-3-(3-chloro-2-(8-methoxy-2-(((((S)-5-oxopyrrolidin-2-yl)methyl)amino)methyl)imidazo[1,2-a]pyridin-6-yl)pyridin-4-yl)phenyl)-2-methoxypyridin-3-yl)methyl)amino)methyl)pyrrolidin-2-one;
   (S)-5-((((6-(2-chloro-3-(3-chloro-2-(8-methoxy-2-(((((R)-5-oxopyrrolidin-2-yl)methyl)amino)methyl)imidazo[1,2-a]pyridin-6-yl)pyridin-4-yl)phenyl)-2-methoxypyridin-3-yl)methyl)amino)methyl)pyrrolidin-2-one;
   (R)-5-((((6-(2-chloro-3-(3-chloro-2-(8-methoxy-2-(((((S)-5-oxopyrrolidin-2-yl)methyl)amino)methyl)imidazo[1,2-a]pyridin-6-yl)pyridin-4-yl)phenyl)-2-methoxypyridin-3-yl)methyl)amino)methyl)pyrrolidin-2-one;
   (R)-5-((((6-(2-chloro-3-(3-chloro-2-(8-methoxy-2-(((((R)-5-oxopyrrolidin-2-yl)methyl)amino)methyl)imidazo[1,2-a]pyridin-6-yl)pyridin-4-yl)phenyl)-2-methoxypyridin-3-yl)methyl)amino)methyl)pyrrolidin-2-one;
   1-(4-(((5-(3-(2-(4-(((1-acetylpiperidin-4-yl)amino)methyl)-3-methoxyphenyl)-3-chloropyridin-4-yl)-2-chlorophenyl)-3-methoxypyrazin-2-yl)methyl)amino)piperidin-1-yl)ethan-1-one;
   (S)-5-((((6-(2-chloro-3-(3-chloro-2-(2-((((1r,3r)-3-hydroxy-3-methylcyclobutyl)amino)methyl)-8-methoxyimidazo[1,2-a]pyridin-6-yl)pyridin-4-yl)phenyl)-2-methoxypyridin-3-yl)methyl)amino)methyl)pyrrolidin-2-one;
   (S)-5-((((6-(2-chloro-3-(3-chloro-2-(2-((((1s,3s)-3-hydroxy-3-methylcyclobutyl)amino)methyl)-8-methoxyimidazo[1,2-a]pyridin-6-yl)pyridin-4-yl)phenyl)-2-methoxypyridin-3-yl)methyl)amino)methyl)pyrrolidin-2-one;
   (S)-5-((((6-(2-chloro-3-(3-chloro-2-(2-((((1s,3r)-3-hydroxy-3-methylcyclobutyl)amino)methyl)-8-methoxyimidazo[1,2-a]pyridin-6-yl)pyridin-4-yl)phenyl)-2-methoxypyridin-3-yl)methyl)amino)methyl)pyrrolidin-2-one;
   (S)-5-((((6-(2-chloro-3-(3-chloro-2-(2-((((1r,3s)-3-hydroxy-3-methylcyclobutyl)amino)methyl)-8-methoxyimidazo[1,2-a]pyridin-6-yl)pyridin-4-yl)phenyl)-2-methoxypyridin-3-yl)methyl)amino)methyl)pyrrolidin-2-one;
   (R)-5-((((6-(2-chloro-3-(3-chloro-2-(2-((((1r,3s)-3-hydroxy-3-methylcyclobutyl)amino)methyl)-8-methoxyimidazo[1,2-a]pyridin-6-yl)pyridin-4-yl)phenyl)-2-methoxypyridin-3-yl)methyl)amino)methyl)pyrrolidin-2-one;
   (R)-5-((((6-(2-chloro-3-(3-chloro-2-(2-((((1s,3s)-3-hydroxy-3-methylcyclobutyl)amino)methyl)-8-methoxyimidazo[1,2-a]pyridin-6-yl)pyridin-4-yl)phenyl)-2-methoxypyridin-3-yl)methyl)amino)methyl)pyrrolidin-2-one;
   (R)-5-((((6-(2-chloro-3-(3-chloro-2-(2-((((1s,3r)-3-hydroxy-3-methylcyclobutyl)amino)methyl)-8-methoxyimidazo[1,2-a]pyridin-6-yl)pyridin-4-yl)phenyl)-2-methoxypyridin-3-yl)methyl)amino)methyl)pyrrolidin-2-one;
   (R)-5-((((6-(2-chloro-3-(3-chloro-2-(2-((((1r,3r)-3-hydroxy-3-methylcyclobutyl)amino)methyl)-8-methoxyimidazo[1,2-a]pyridin-6-yl)pyridin-4-yl)phenyl)-2-methoxypyridin-3-yl)methyl)amino)methyl)pyrrolidin-2-one;
   1,1'-(((((3,3'-dichloro-[4,4'-bipyridine]-2,2'-diyl)bis(2-fluoro-6-methoxy-4,1-phenylene))bis(methylene))bis(azanediyl))bis(piperidine-4,1-diyl))bis(ethan-1-one);
   (S)-2-((6-(3-chloro-4-(2-chloro-3-(6-methoxy-5-((((5-oxopyrrolidin-2-yl)methyl)amino)methyl)pyridin-2-yl)phenyl)pyridin-2-yl)-8-methoxyimidazo[1,2-a]pyridin-2-yl)methyl)-2,6-diazaspiro[3.4]octan-7-one;
   (R)-2-((6-(3-chloro-4-(2-chloro-3-(6-methoxy-5-((((5-oxopyrrolidin-2-yl)methyl)amino)methyl)pyridin-2-yl)phenyl)pyridin-2-yl)-8-methoxyimidazo[1,2-a]pyridin-2-yl)methyl)-2,6-diazaspiro[3.4]octan-7-one;
   1-(4-(((6-(3-(2-(4-(((1-acetylpiperidin-4-yl)(methyl)amino)methyl)-3-methoxyphenyl)-3-chloropyridin-4-yl)-2-chlorophenyl)-2-methoxypyridin-3-yl)methyl)amino)piperidin-1-yl)ethan-1-one;
   (4-(((6-(2-chloro-3-(3-chloro-2-(4-(((1-(cyclopropanecarbonyl)piperidin-4-yl)amino)methyl)-3-fluoro-5-methoxyphenyl)pyridin-4-yl)phenyl)-2-methoxypyridin-3-yl)methyl)amino)piperidin-1-yl)(cyclopropyl)methanone;
   (S)-5-((((6-(2-chloro-3-(3-chloro-2-(2-((3-hydroxy-3-methylazetidin-1-yl)methyl)-8-methoxyimidazo[1,2-a]pyridin-6-yl)pyridin-4-yl)phenyl)-2-methoxypyridin-3-yl)methyl)amino)methyl)pyrrolidin-2-one;
   (R)-5-((((6-(2-chloro-3-(3-chloro-2-(2-((3-hydroxy-3-methylazetidin-1-yl)methyl)-8-methoxyimidazo[1,2-a]pyridin-6-yl)pyridin-4-yl)phenyl)-2-methoxypyridin-3-yl)methyl)amino)methyl)pyrrolidin-2-one;
   (S)-5-((((6-(3-(2-(2-(((1-acetylpiperidin-4-yl)amino)methyl)-8-methoxyimidazo[1,2-a]pyridin-6-yl)-3-chloropyridin-4-yl)-2-chlorophenyl)-2-methoxypyridin-3-yl)methyl)amino)methyl)pyrrolidin-2-one;
   (R)-5-((((6-(3-(2-(2-(((1-acetylpiperidin-4-yl)amino)methyl)-8-methoxyimidazo[1,2-a]pyridin-6-yl)-3-chloropyridin-4-yl)-2-chlorophenyl)-2-methoxypyridin-3-yl)methyl)amino)methyl)pyrrolidin-2-one;
   (S)-5-((((6-(3-(2-(2-((6-acetyl-2,6-diazaspiro[3.3]heptan-2-yl)methyl)-8-methoxyimidazo[1,2-a]pyridin-6-yl)-3-chloropyridin-4-yl)-2-chlorophenyl)-2-methoxypyridin-3-yl)methyl)amino)methyl)pyrrolidin-2-one;
   (R)-5-((((6-(3-(2-(2-((6-acetyl-2,6-diazaspiro[3.3]heptan-2-yl)methyl)-8-methoxyimidazo[1,2-a]pyridin-6-yl)-3-chloropyridin-4-yl)-2-chlorophenyl)-2-methoxypyridin-3-yl)methyl)amino)methyl)pyrrolidin-2-one;
   (S)-2-((6-(3-chloro-4-(2-chloro-3-(6-methoxy-5-((((5-oxopyrrolidin-2-yl)methyl)amino)methyl)pyridin-2-yl)phenyl)pyridin-2-yl)-8-methoxyimidazo[1,2-a]pyridin-2-yl)methyl)-2,5-diazaspiro[3.4]octan-6-one;
   (R)-2-((6-(3-chloro-4-(2-chloro-3-(6-methoxy-5-((((5-oxopyrrolidin-2-yl)methyl)amino)methyl)pyridin-2-yl)phenyl)pyridin-2-yl)-8-methoxyimidazo[1,2-a]pyridin-2-yl)methyl)-2,5-diazaspiro[3.4]octan-6-one;
   (S)-5-((((6-(3-(2-(2-(((2-oxaspiro[3.3]heptan-6-yl)amino)methyl)-8-methoxyimidazo[1,2-a]pyridin-6-yl)-3-chloropyridin-4-yl)-2-chlorophenyl)-2-methoxypyridin-3-yl)methyl)amino)methyl)pyrrolidin-2-one;
   (R)-5-((((6-(3-(2-(2-(((2-oxaspiro[3.3]heptan-6-yl)amino)methyl)-8-methoxyimidazo[1,2-a]pyridin-6-yl)-3-chloropyridin-4-yl)-2-chlorophenyl)-2-methoxypyridin-3-yl)methyl)amino)methyl)pyrrolidin-2-one;
   N-(1-((6-(3-(2-(4-((4-acetamidopiperidin-1-yl)methyl)-3-methoxyphenyl)-3-chloropyridin-4-yl)-2-chlorophenyl)-2-methoxypyridin-3-yl)methyl)piperidin-4-yl)acetamide;
   2,2'-(((3,3'-dichloro-[4,4'-bipyridine]-2,2'-diyl)bis(2-fluoro-6-methoxy-4,1-phenylene))bis(methylene))bis(2,6-diazaspiro[3.4]octan-7-one);
   2-(4-(4-(2-chloro-3-(6-methoxy-5-((7-oxo-2,6-diazaspiro[3.4]octan-2-yl)methyl)pyridin-2-yl)phenyl)-3-fluoropyridin-2-yl)-2-methoxybenzyl)-2,6-diazaspiro[3.4]octan-7-one;
   N-(1-(4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)piperidin-4-yl)acetamide;
   N-((6-(2-chloro-3-(3-chloro-2-(4-(((1-isopropylpiperidin-4-yl)amino)methyl)-3-methoxyphenyl)pyridin-4-yl)phenyl)-2-methoxypyridin-3-yl)methyl)-1-isopropylpiperidin-4-amine;
   N-((6-(2-chloro-3-(3-chloro-2-(4-(((4,4-difluorocyclohexyl)amino)methyl)-3-methoxyphenyl)pyridin-4-yl)phenyl)-2-methoxypyridin-3-yl)methyl)-4,4-difluorocyclohexan-1-amine;
   2-(4-(((6-(2-chloro-3-(3-chloro-2-(4-(((1-(2-hydroxyethyl)piperidin-4-yl)amino)methyl)-3-methoxyphenyl)pyridin-4-yl)phenyl)-2-methoxypyridin-3-yl)methyl)amino)piperidin-1-yl)ethan-1-ol;
   N-(2-(((6-(3-(2-(4-(((2-acetamidoethyl)amino)methyl)-3-methoxyphenyl)-3-chloropyridin-4-yl)-2-chlorophenyl)-2-methoxypyridin-3-yl)methyl)amino)ethyl)acetamide;
   1-(6-(2-chloro-3-(3-chloro-2-(3-methoxy-4-((((tetrahydro-2H-pyran-4-yl)methyl)amino)methyl)phenyl)pyridin-4-yl)phenyl)-2-methoxypyridin-3-yl)-N-((tetrahydro-2H-pyran-4-yl)methyl)methanamine;
   1-(4-(((6-(2-chloro-3-(3-chloro-2-(3-fluoro-5-methoxy-4-(((1-propionylpiperidin-4-yl)amino)methyl)phenyl)pyridin-4-yl)phenyl)-2-methoxypyridin-3-yl)methyl)amino)piperidin-1-yl)propan-1-one;
   2-((6-(3-(2-(4-(((1-acetylpiperidin-4-yl)amino)methyl)-3-methoxyphenyl)-3-chloropyridin-4-yl)-2-chlorophenyl)-2-methoxypyridin-3-yl)methyl)-2,6-diazaspiro[3.4]octan-7-one;
   1-(4-((4-(4-(3-(5-(((1-acetylpiperidin-4-yl)(methyl)amino)methyl)-6-methoxypyridin-2-yl)-2-chlorophenyl)-3-chloropyridin-2-yl)-2-methoxybenzyl)amino)piperidin-1-yl)ethan-1-one;
   1-(6-(2-chloro-3-(2-(3-methoxy-4-((methylamino)methyl)phenyl)-3-methylpyridin-4-yl)phenyl)-2-methoxypyridin-3-yl)-N-methylmethanamine;
   (S)-5-((((6-(3-(2-(4-((S)-1-aminoethyl)-3-methoxyphenyl)-3-chloropyridin-4-yl)-2-chlorophenyl)-2-methoxypyridin-3-yl)methyl)amino)methyl)pyrrolidin-2-one;
   (R)-5-((((6-(3-(2-(4-((S)-1-aminoethyl)-3-methoxyphenyl)-3-chloropyridin-4-yl)-2-chlorophenyl)-2-methoxypyridin-3-yl)methyl)amino)methyl)pyrrolidin-2-one;
   (S)-5-((((6-(3-(2-(4-((R)-1-aminoethyl)-3-methoxyphenyl)-3-chloropyridin-4-yl)-2-chlorophenyl)-2-methoxypyridin-3-yl)methyl)amino)methyl)pyrrolidin-2-one;
   (R)-5-((((6-(3-(2-(4-((R)-1-aminoethyl)-3-methoxyphenyl)-3-chloropyridin-4-yl)-2-chlorophenyl)-2-methoxypyridin-3-yl)methyl)amino)methyl)pyrrolidin-2-one;
   (S)-2-((3'-chloro-6-methoxy-2'-(1-((6-methoxy-5-((7-oxo-2,6-diazaspiro[3.4]octan-2-yl)methyl)-3-(trifluoromethyl)pyridin-2-yl)oxy)-2,3-dihydro-1H-inden-4-yl)-[2,4'-bipyridin]-5-yl)methyl)-2,6-diazaspiro[3.4]octan-7-one;
   (R)-2-((3'-chloro-6-methoxy-2'-(1-((6-methoxy-5-((7-oxo-2,6-diazaspiro[3.4]octan-2-yl)methyl)-3-(trifluoromethyl)pyridin-2-yl)oxy)-2,3-dihydro-1H-inden-4-yl)-[2,4'-bipyridin]-5-yl)methyl)-2,6-diazaspiro[3.4]octan-7-one;
   1-(4-(((6-(3-(2-(4-(((1-acetylpiperidin-4-yl)amino)methyl)-3-methoxyphenyl)-3-fluoropyridin-4-yl)-2-chlorophenyl)-2-methoxypyridin-3-yl)methyl)amino)piperidin-1-yl)ethan-1-one;
   (S)-4-((4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)amino)dihydrofuran-2(3H)-one;
   (R)-4-((4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)amino)dihydrofuran-2(3H)-one;
   methyl (S)-3-((4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)amino)-4-hydroxybutanoate;
   methyl (R)-3-((4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)amino)-4-hydroxybutanoate;
   (S)-3-((4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)amino)dihydrofuran-2(3H)-one;
   (R)-3-((4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)amino)dihydrofuran-2(3H)-one;
   N-(1-((6-(3-(2-(4-(((1-acetylpiperidin-4-yl)amino)methyl)-3-methoxyphenyl)-3-chloropyridin-4-yl)-2-chlorophenyl)-2-methoxypyridin-3-yl)methyl)piperidin-4-yl)acetamide;
   (S)-3-((4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)amino)-4-hydroxybutanoic acid;
   (R)-3-((4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)amino)-4-hydroxybutanoic acid;
   (4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)-L-homoserine;
   (4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)-D-homoserine;
   1-(4-(((6-(2-chloro-3-(3-chloro-2-(3-methoxy-4-(((1-propionylpiperidin-4-yl)amino)methyl)phenyl)pyridin-4-yl)phenyl)-2-methoxypyridin-3-yl)methyl)amino)piperidin-1-yl)propan-1-one;
   (4-(((6-(2-chloro-3-(3-chloro-2-(4-(((1-(cyclopropanecarbonyl)piperidin-4-yl)amino)methyl)-3-methoxyphenyl)pyridin-4-yl)phenyl)-2-methoxypyridin-3-yl)methyl)amino)piperidin-1-yl)(cyclopropyl)methanone;
   1-(4-(((6-(2-chloro-3-(3-chloro-2-(4-(((1-isobutyrylpiperidin-4-yl)amino)methyl)-3-methoxyphenyl)pyridin-4-yl)phenyl)-2-methoxypyridin-3-yl)methyl)amino)piperidin-1-yl)-2-methylpropan-1-one;
   1-(4-(((6-(2-chloro-3-(3-chloro-2-(3-fluoro-4-(((1-isobutyrylpiperidin-4-yl)amino)methyl)-5-methoxyphenyl)pyridin-4-yl)phenyl)-2-methoxypyridin-3-yl)methyl)amino)piperidin-1-yl)-2-methylpropan-1-one;
   N-((6-(2-chloro-3-(3-chloro-2-(3-fluoro-5-methoxy-4-(((tetrahydro-2H-pyran-4-yl)amino)methyl)phenyl)pyridin-4-yl)phenyl)-2-methoxypyridin-3-yl)methyl)tetrahydro-2H-pyran-4-amine;
   4-(((6-(2-chloro-3-(3-chloro-2-(4-(((1,1-dioxidotetrahydro-2H-thiopyran-4-yl)amino)methyl)-3-fluoro-5-methoxyphenyl)pyridin-4-yl)phenyl)-2-methoxypyridin-3-yl)methyl)amino)tetrahydro-2H-thiopyran 1,1-dioxide;
   (S)-1-((6-(3-chloro-4-(2-chloro-3-(6-methoxy-5-((((5-oxopyrrolidin-2-yl)methyl)amino)methyl)pyridin-2-yl)phenyl)pyridin-2-yl)-8-methoxyimidazo[1,2-a]pyridin-2-yl)methyl)azetidine-3-carboxylic acid;
   (R)-1-((6-(3-chloro-4-(2-chloro-3-(6-methoxy-5-((((5-oxopyrrolidin-2-yl)methyl)amino)methyl)pyridin-2-yl)phenyl)pyridin-2-yl)-8-methoxyimidazo[1,2-a]pyridin-2-yl)methyl)azetidine-3-carboxylic acid;
   (S)-1-(2-(((6-(3-chloro-4-(2-chloro-3-(6-methoxy-5-((((5-oxopyrrolidin-2-yl)methyl)amino)methyl)pyridin-2-yl)phenyl)pyridin-2-yl)-8-methoxyimidazo[1,2-a]pyridin-2-yl)methyl)amino)ethyl)cyclopropane-1-carboxylic acid;
   (R)-1-(2-(((6-(3-chloro-4-(2-chloro-3-(6-methoxy-5-((((5-oxopyrrolidin-2-yl)methyl)amino)methyl)pyridin-2-yl)phenyl)pyridin-2-yl)-8-methoxyimidazo[1,2-a]pyridin-2-yl)methyl)amino)ethyl)cyclopropane-1-carboxylic acid;
   (4-(((6-(2-chloro-3-(3-chloro-2-(4-(((1-(cyclobutanecarbonyl)piperidin-4-yl)amino)methyl)-3-methoxyphenyl)pyridin-4-yl)phenyl)-2-methoxypyridin-3-yl)methyl)amino)piperidin-1-yl)(cyclobutyl)methanone;
   7-(4-(3-chloro-4-(2-chloro-3-(6-methoxy-5-((2-oxo-1,7-diazaspiro[3.5]nonan-7-yl)methyl)pyridin-2-yl)phenyl)pyridin-2-yl)-2-methoxybenzyl)-1,7-diazaspiro[3.5]nonan-2-one;
   (R)-5-((((6-(2-chloro-3-(3-chloro-2-(3-methoxy-4-(((((R)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
   (R)-5-((((6-(2-chloro-3-(3-chloro-2-(3-methoxy-4-(((((S)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
   (S)-5-((((6-(2-chloro-3-(3-chloro-2-(3-methoxy-4-(((((R)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
   (S)-5-((((6-(2-chloro-3-(3-chloro-2-(3-methoxy-4-(((((S)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
   N-(1-((6-(2-chloro-3-(3-chloro-2-(3-methoxy-4-((7-oxo-2,6-diazaspiro[3.4]octan-2-yl)methyl)phenyl)pyridin-4-yl)phenyl)-2-methoxypyridin-3-yl)methyl)piperidin-4-yl)acetamide;
   (R)-N-(1-((6-(2-chloro-3-(3-chloro-2-(3-methoxy-4-((((5-oxopyrrolidin-2-yl)methyl)amino)methyl)phenyl)pyridin-4-yl)phenyl)-2-methoxypyridin-3-yl)methyl)piperidin-4-yl)acetamide;
   (S)-N-(1-((6-(2-chloro-3-(3-chloro-2-(3-methoxy-4-((((5-oxopyrrolidin-2-yl)methyl)amino)methyl)phenyl)pyridin-4-yl)phenyl)-2-methoxypyridin-3-yl)methyl)piperidin-4-yl)acetamide;
   (S)-N-(1-((6-(2-chloro-3-(3-chloro-2-(3-methoxy-4-((((5-oxopyrrolidin-2-yl)methyl)amino)methyl)phenyl)pyridin-4-yl)phenyl)-2-methoxypyridin-3-yl)methyl)piperidin-4-yl)acetamide;
   (R)-N-(1-((6-(2-chloro-3-(3-chloro-2-(3-methoxy-4-((((5-oxopyrrolidin-2-yl)methyl)amino)methyl)phenyl)pyridin-4-yl)phenyl)-2-methoxypyridin-3-yl)methyl)piperidin-4-yl)acetamide;
   N-(3-(((6-(3-(2-(4-(((3-acetamidopropyl)amino)methyl)-3-methoxyphenyl)-3-chloropyridin-4-yl)-2-chlorophenyl)-2-methoxypyridin-3-yl)methyl)amino)propyl)acetamide;
   4-(((6-(2-chloro-3-(3-chloro-2-(4-(((2,6-dioxopiperidin-4-yl)amino)methyl)-3-methoxyphenyl)pyridin-4-yl)phenyl)-2-methoxypyridin-3-yl)methyl)amino)piperidine-2,6-dione;
   1-(4-(((6-(2-chloro-3-(3-chloro-2-(4-((((1r,4r)-4-hydroxycyclohexyl)amino)methyl)-3-methoxyphenyl)pyridin-4-yl)phenyl)-2-methoxypyridin-3-yl)methyl)amino)piperidin-1-yl)ethan-1-one;
   1-(4-(((6-(2-chloro-3-(3-chloro-2-(4-((((1r,4s)-4-hydroxycyclohexyl)amino)methyl)-3-methoxyphenyl)pyridin-4-yl)phenyl)-2-methoxypyridin-3-yl)methyl)amino)piperidin-1-yl)ethan-1-one;
   1-(4-(((6-(2-chloro-3-(3-chloro-2-(4-((((1s,4r)-4-hydroxycyclohexyl)amino)methyl)-3-methoxyphenyl)pyridin-4-yl)phenyl)-2-methoxypyridin-3-yl)methyl)amino)piperidin-1-yl)ethan-1-one;
   1-(4-(((6-(2-chloro-3-(3-chloro-2-(4-((((1s,4s)-4-hydroxycyclohexyl)amino)methyl)-3-methoxyphenyl)pyridin-4-yl)phenyl)-2-methoxypyridin-3-yl)methyl)amino)piperidin-1-yl)ethan-1-one;
   1-(4-(((6-(2-chloro-3-(3-chloro-2-(4-((6-hydroxy-2-azaspiro[3.3]heptan-2-yl)methyl)-3-methoxyphenyl)pyridin-4-yl)phenyl)-2-methoxypyridin-3-yl)methyl)amino)piperidin-1-yl)ethan-1-one;
   1-(2-methoxy-4-(4-(3-(6-methoxy-5-((methylamino)methyl)pyridin-2-yl)-2-methylphenyl)-3-methylpyridin-2-yl)phenyl)-N-methylmethanamine;
   1-(4-(((6-(3-(2-(4-(((1-acetylpiperidin-4-yl)amino)methyl)-3-methoxyphenyl)-3-methylpyridin-4-yl)-2-methylphenyl)-2-methoxypyridin-3-yl)methyl)amino)piperidin-1-yl)ethan-1-one;
   (1r,4r)-4-(((6-(2-chloro-3-(3-chloro-2-(4-((((1r,4r)-4-hydroxycyclohexyl)amino)methyl)-3-methoxyphenyl)pyridin-4-yl)phenyl)-2-methoxypyridin-3-yl)methyl)amino)cyclohexan-1-ol;
   (1r,4r)-4-(((6-(2-chloro-3-(3-chloro-2-(4-((((1r,4s)-4-hydroxycyclohexyl)amino)methyl)-3-methoxyphenyl)pyridin-4-yl)phenyl)-2-methoxypyridin-3-yl)methyl)amino)cyclohexan-1-ol;
   (1r,4r)-4-(((6-(2-chloro-3-(3-chloro-2-(4-((((1s,4r)-4-hydroxycyclohexyl)amino)methyl)-3-methoxyphenyl)pyridin-4-yl)phenyl)-2-methoxypyridin-3-yl)methyl)amino)cyclohexan-1-ol;
   (1r,4r)-4-(((6-(2-chloro-3-(3-chloro-2-(4-((((1s,4s)-4-hydroxycyclohexyl)amino)methyl)-3-methoxyphenyl)pyridin-4-yl)phenyl)-2-methoxypyridin-3-yl)methyl)amino)cyclohexan-1-ol;
   (1r,4s)-4-(((6-(2-chloro-3-(3-chloro-2-(4-((((1r,4r)-4-hydroxycyclohexyl)amino)methyl)-3-methoxyphenyl)pyridin-4-yl)phenyl)-2-methoxypyridin-3-yl)methyl)amino)cyclohexan-1-ol;
   (1r,4s)-4-(((6-(2-chloro-3-(3-chloro-2-(4-((((1r,4s)-4-hydroxycyclohexyl)amino)methyl)-3-methoxyphenyl)pyridin-4-yl)phenyl)-2-methoxypyridin-3-yl)methyl)amino)cyclohexan-1-ol;
   (1r,4s)-4-(((6-(2-chloro-3-(3-chloro-2-(4-((((1s,4r)-4-hydroxycyclohexyl)amino)methyl)-3-methoxyphenyl)pyridin-4-yl)phenyl)-2-methoxypyridin-3-yl)methyl)amino)cyclohexan-1-ol;
   (1r,4s)-4-(((6-(2-chloro-3-(3-chloro-2-(4-((((1s,4s)-4-hydroxycyclohexyl)amino)methyl)-3-methoxyphenyl)pyridin-4-yl)phenyl)-2-methoxypyridin-3-yl)methyl)amino)cyclohexan-1-ol;
   (1s,4r)-4-(((6-(2-chloro-3-(3-chloro-2-(4-((((1r,4r)-4-hydroxycyclohexyl)amino)methyl)-3-methoxyphenyl)pyridin-4-yl)phenyl)-2-methoxypyridin-3-yl)methyl)amino)cyclohexan-1-ol;
   (1s,4r)-4-(((6-(2-chloro-3-(3-chloro-2-(4-((((1r,4s)-4-hydroxycyclohexyl)amino)methyl)-3-methoxyphenyl)pyridin-4-yl)phenyl)-2-methoxypyridin-3-yl)methyl)amino)cyclohexan-1-ol;
   (1s,4r)-4-(((6-(2-chloro-3-(3-chloro-2-(4-((((1s,4r)-4-hydroxycyclohexyl)amino)methyl)-3-methoxyphenyl)pyridin-4-yl)phenyl)-2-methoxypyridin-3-yl)methyl)amino)cyclohexan-1-ol;
   (1s,4r)-4-(((6-(2-chloro-3-(3-chloro-2-(4-((((1s,4s)-4-hydroxycyclohexyl)amino)methyl)-3-methoxyphenyl)pyridin-4-yl)phenyl)-2-methoxypyridin-3-yl)methyl)amino)cyclohexan-1-ol;
   (1s,4s)-4-(((6-(2-chloro-3-(3-chloro-2-(4-((((1r,4r)-4-hydroxycyclohexyl)amino)methyl)-3-methoxyphenyl)pyridin-4-yl)phenyl)-2-methoxypyridin-3-yl)methyl)amino)cyclohexan-1-ol;
   (1s,4s)-4-(((6-(2-chloro-3-(3-chloro-2-(4-((((1r,4s)-4-hydroxycyclohexyl)amino)methyl)-3-methoxyphenyl)pyridin-4-yl)phenyl)-2-methoxypyridin-3-yl)methyl)amino)cyclohexan-1-ol;
   (1s,4s)-4-(((6-(2-chloro-3-(3-chloro-2-(4-((((1s,4r)-4-hydroxycyclohexyl)amino)methyl)-3-methoxyphenyl)pyridin-4-yl)phenyl)-2-methoxypyridin-3-yl)methyl)amino)cyclohexan-1-ol;
   (1s,4s)-4-(((6-(2-chloro-3-(3-chloro-2-(4-((((1s,4s)-4-hydroxycyclohexyl)amino)methyl)-3-methoxyphenyl)pyridin-4-yl)phenyl)-2-methoxypyridin-3-yl)methyl)amino)cyclohexan-1-ol;
   N-(4-(4-(3-(5-(((2-oxaspiro[3.3]heptan-6-yl)amino)methyl)-6-methoxypyridin-2-yl)-2-chlorophenyl)-3-chloropyridin-2-yl)-2-methoxybenzyl)-2-oxaspiro[3.3]heptan-6-amine;
   (S)-2-(4-(3-chloro-4-(1-((6-methoxy-5-((7-oxo-2,6-diazaspiro[3.4]octan-2-yl)methyl)-3-(trifluoromethyl)pyrazin-2-yl)amino)-2,3-dihydro-1H-inden-4-yl)pyridin-2-yl)-2-methoxybenzyl)-2,6-diazaspiro[3.4]octan-7-one;
   (R)-2-(4-(3-chloro-4-(1-((6-methoxy-5-((7-oxo-2,6-diazaspiro[3.4]octan-2-yl)methyl)-3-(trifluoromethyl)pyrazin-2-yl)amino)-2,3-dihydro-1H-inden-4-yl)pyridin-2-yl)-2-methoxybenzyl)-2,6-diazaspiro[3.4]octan-7-one;
   (S)-1-(6-((5-((4-(2-(4-((6-acetyl-2,6-diazaspiro[3.3]heptan-2-yl)methyl)-3-methoxyphenyl)-3-chloropyridin-4-yl)-2,3-dihydro-1H-inden-1-yl)amino)-3-methoxy-6-(trifluoromethyl)pyrazin-2-yl)methyl)-2,6-diazaspiro[3.3]heptan-2-yl)ethan-1-one;
   (R)-1-(6-((5-((4-(2-(4-((6-acetyl-2,6-diazaspiro[3.3]heptan-2-yl)methyl)-3-methoxyphenyl)-3-chloropyridin-4-yl)-2,3-dihydro-1H-inden-1-yl)amino)-3-methoxy-6-(trifluoromethyl)pyrazin-2-yl)methyl)-2,6-diazaspiro[3.3]heptan-2-yl)ethan-1-one;
   2-(4-(4-(2-chloro-3-(6-methoxy-5-((7-oxo-2,6-diazaspiro[3.4]octan-2-yl)methyl)pyridin-2-yl)phenyl)-3-methylpyridin-2-yl)-2-methoxybenzyl)-2,6-diazaspiro[3.4]octan-7-one;
   1-(6-((6-(3-(2-(4-((6-acetyl-2,6-diazaspiro[3.3]heptan-2-yl)methyl)-3-methoxyphenyl)-3-methylpyridin-4-yl)-2-chlorophenyl)-2-methoxypyridin-3-yl)methyl)-2,6-diazaspiro[3.3]heptan-2-yl)ethan-1-one;
   (S)-5-((((6-(2-chloro-3-(3-chloro-2-(2-((((1r,3r)-3-hydroxy-3-methylcyclobutyl)amino)methyl)-8-methoxy-[1,2,4]triazolo[1,5-a]pyridin-6-yl)pyridin-4-yl)phenyl)-2-methoxypyridin-3-yl)methyl)amino)methyl)pyrrolidin-2-one;
   (R)-5-((((6-(2-chloro-3-(3-chloro-2-(2-((((1r,3r)-3-hydroxy-3-methylcyclobutyl)amino)methyl)-8-methoxy-[1,2,4]triazolo[1,5-a]pyridin-6-yl)pyridin-4-yl)phenyl)-2-methoxypyridin-3-yl)methyl)amino)methyl)pyrrolidin-2-one;
   (R)-5-((((6-(2-chloro-3-(3-chloro-2-(2-((((1r,3s)-3-hydroxy-3-methylcyclobutyl)amino)methyl)-8-methoxy-[1,2,4]triazolo[1,5-a]pyridin-6-yl)pyridin-4-yl)phenyl)-2-methoxypyridin-3-yl)methyl)amino)methyl)pyrrolidin-2-one;
   (R)-5-((((6-(2-chloro-3-(3-chloro-2-(2-((((1s,3r)-3-hydroxy-3-methylcyclobutyl)amino)methyl)-8-methoxy-[1,2,4]triazolo[1,5-a]pyridin-6-yl)pyridin-4-yl)phenyl)-2-methoxypyridin-3-yl)methyl)amino)methyl)pyrrolidin-2-one;
   (R)-5-((((6-(2-chloro-3-(3-chloro-2-(2-((((1s,3s)-3-hydroxy-3-methylcyclobutyl)amino)methyl)-8-methoxy-[1,2,4]triazolo[1,5-a]pyridin-6-yl)pyridin-4-yl)phenyl)-2-methoxypyridin-3-yl)methyl)amino)methyl)pyrrolidin-2-one;
   (S)-5-((((6-(2-chloro-3-(3-chloro-2-(2-((((1r,3s)-3-hydroxy-3-methylcyclobutyl)amino)methyl)-8-methoxy-[1,2,4]triazolo[1,5-a]pyridin-6-yl)pyridin-4-yl)phenyl)-2-methoxypyridin-3-yl)methyl)amino)methyl)pyrrolidin-2-one;
   (S)-5-((((6-(2-chloro-3-(3-chloro-2-(2-((((1s,3r)-3-hydroxy-3-methylcyclobutyl)amino)methyl)-8-methoxy-[1,2,4]triazolo[1,5-a]pyridin-6-yl)pyridin-4-yl)phenyl)-2-methoxypyridin-3-yl)methyl)amino)methyl)pyrrolidin-2-one;
   (S)-5-((((6-(2-chloro-3-(3-chloro-2-(2-((((1s,3s)-3-hydroxy-3-methylcyclobutyl)amino)methyl)-8-methoxy-[1,2,4]triazolo[1,5-a]pyridin-6-yl)pyridin-4-yl)phenyl)-2-methoxypyridin-3-yl)methyl)amino)methyl)pyrrolidin-2-one;
   1-(4-((4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)amino)piperidin-1-yl)-2-methylpropan-1-one;
   N-((6-(2-chloro-3-(3-chloro-2-(3-methoxy-4-(((1-(methylsulfonyl)piperidin-4-yl)amino)methyl)phenyl)pyridin-4-yl)phenyl)-2-methoxypyridin-3-yl)methyl)-1-(methylsulfonyl)piperidin-4-amine;
   5-(((6-(3-(2-(4-(((5-amino-5-oxopentyl)amino)methyl)-3-methoxyphenyl)-3-chloropyridin-4-yl)-2-chlorophenyl)-2-methoxypyridin-3-yl)methyl)amino)pentanamide;
   1-((R)-3-(((6-(3-(2-(4-((((R)-1-acetylpyrrolidin-3-yl)amino)methyl)-3-methoxyphenyl)-3-chloropyridin-4-yl)-2-chlorophenyl)-2-methoxypyridin-3-yl)methyl)amino)pyrrolidin-1-yl)ethan-1-one;
   1-((S)-3-(((6-(3-(2-(4-((((R)-1-acetylpyrrolidin-3-yl)amino)methyl)-3-methoxyphenyl)-3-chloropyridin-4-yl)-2-chlorophenyl)-2-methoxypyridin-3-yl)methyl)amino)pyrrolidin-1-yl)ethan-1-one;
   1-((R)-3-(((6-(3-(2-(4-((((S)-1-acetylpyrrolidin-3-yl)amino)methyl)-3-methoxyphenyl)-3-chloropyridin-4-yl)-2-chlorophenyl)-2-methoxypyridin-3-yl)methyl)amino)pyrrolidin-1-yl)ethan-1-one;
   1-((S)-3-(((6-(3-(2-(4-((((S)-1-acetylpyrrolidin-3-yl)amino)methyl)-3-methoxyphenyl)-3-chloropyridin-4-yl)-2-chlorophenyl)-2-methoxypyridin-3-yl)methyl)amino)pyrrolidin-1-yl)ethan-1-one;
   1-(4-(((6-(3-(6'-(((1-acetylpiperidin-4-yl)amino)methyl)-3-chloro-5'-methoxy-[2,3'-bipyridin]-4-yl)-2-chlorophenyl)-2-methoxypyridin-3-yl)methyl)amino)piperidin-1-yl)ethan-1-one;
   2-((3-chloro-4-(2-chloro-3-(6-methoxy-5-((7-oxo-2,6-diazaspiro[3.4]octan-2-yl)methyl)pyridin-2-yl)phenyl)-5'-methoxy-[2,3'-bipyridin]-6'-yl)methyl)-2,6-diazaspiro[3.4]octan-7-one;
   (S)-N-(1-((6-(3-chloro-4-(2-chloro-3-(6-methoxy-5-((((5-oxopyrrolidin-2-yl)methyl)amino)methyl)pyridin-2-yl)phenyl)pyridin-2-yl)-1-methyl-1H-pyrrolo[3,2-b]pyridin-3-yl)methyl)piperidin-4-yl)acetamide;
   (R)-N-(1-((6-(3-chloro-4-(2-chloro-3-(6-methoxy-5-((((5-oxopyrrolidin-2-yl)methyl)amino)methyl)pyridin-2-yl)phenyl)pyridin-2-yl)-1-methyl-1H-pyrrolo[3,2-b]pyridin-3-yl)methyl)piperidin-4-yl)acetamide;
   (S)-2-((6-(3-chloro-4-(2-chloro-3-(6-methoxy-5-((((5-oxopyrrolidin-2-yl)methyl)amino)methyl)pyridin-2-yl)phenyl)pyridin-2-yl)-1-methyl-1H-pyrrolo[3,2-b]pyridin-3-yl)methyl)-2,6-diazaspiro[3.4]octan-7-one;
   (R)-2-((6-(3-chloro-4-(2-chloro-3-(6-methoxy-5-((((5-oxopyrrolidin-2-yl)methyl)amino)methyl)pyridin-2-yl)phenyl)pyridin-2-yl)-1-methyl-1H-pyrrolo[3,2-b]pyridin-3-yl)methyl)-2,6-diazaspiro[3.4]octan-7-one;
   1-(4-(((6-(3-(2-(4-(((2-oxaspiro[3.3]heptan-6-yl)amino)methyl)-3-methoxyphenyl)-3-chloropyridin-4-yl)-2-chlorophenyl)-2-methoxypyridin-3-yl)methyl)amino)piperidin-1-yl)ethan-1-one;
   1-(4-(((6-(3-(2-(4-((2-oxa-6-azaspiro[3.3]heptan-6-yl)methyl)-3-methoxyphenyl)-3-chloropyridin-4-yl)-2-chlorophenyl)-2-methoxypyridin-3-yl)methyl)amino)piperidin-1-yl)ethan-1-one;
   1-(4-(((6-(2-chloro-3-(3-chloro-2-(4-(((3,3-dimethylcyclobutyl)amino)methyl)-3-methoxyphenyl)pyridin-4-yl)phenyl)-2-methoxypyridin-3-yl)methyl)amino)piperidin-1-yl)ethan-1-one;
   1-(4-(((6-(3-(2-(4-(((1-acetylpiperidin-4-yl)amino)methyl)-3-methoxyphenyl)-3-chloropyridin-4-yl)-2-chlorophenyl)-2-methoxypyridin-3-yl)methyl)amino)piperidin-1-yl)-2-methylpropan-1-one;
   1-(4-(((6-(2-chloro-3-(3-chloro-2-(3-methoxy-4-(((1-(methylsulfonyl)piperidin-4-yl)amino)methyl)phenyl)pyridin-4-yl)phenyl)-2-methoxypyridin-3-yl)methyl)amino)piperidin-1-yl)ethan-1-one;
   (S)-5-((((6-(2-chloro-3-(3-chloro-2-(2-((((1r,4r)-4-hydroxycyclohexyl)amino)methyl)-8-methoxyimidazo[1,2-a]pyridin-6-yl)pyridin-4-yl)phenyl)-2-methoxypyridin-3-yl)methyl)amino)methyl)pyrrolidin-2-one;
   (R)-5-((((6-(2-chloro-3-(3-chloro-2-(2-((((Ir,4r)-4-hydroxycyclohexyl)amino)methyl)-8-methoxyimidazo[1,2-a]pyridin-6-yl)pyridin-4-yl)phenyl)-2-methoxypyridin-3-yl)methyl)amino)methyl)pyrrolidin-2-one;
   (R)-5-((((6-(2-chloro-3-(3-chloro-2-(2-((((1r,4s)-4-hydroxycyclohexyl)amino)methyl)-8-methoxyimidazo[1,2-a]pyridin-6-yl)pyridin-4-yl)phenyl)-2-methoxypyridin-3-yl)methyl)amino)methyl)pyrrolidin-2-one;
   (R)-5-((((6-(2-chloro-3-(3-chloro-2-(2-((((1s,4r)-4-hydroxycyclohexyl)amino)methyl)-8-methoxyimidazo[1,2-a]pyridin-6-yl)pyridin-4-yl)phenyl)-2-methoxypyridin-3-yl)methyl)amino)methyl)pyrrolidin-2-one;
   (R)-5-((((6-(2-chloro-3-(3-chloro-2-(2-((((1s,4s)-4-hydroxycyclohexyl)amino)methyl)-8-methoxyimidazo[1,2-a]pyridin-6-yl)pyridin-4-yl)phenyl)-2-methoxypyridin-3-yl)methyl)amino)methyl)pyrrolidin-2-one;
   (S)-5-((((6-(2-chloro-3-(3-chloro-2-(2-((((1r,4s)-4-hydroxycyclohexyl)amino)methyl)-8-methoxyimidazo[1,2-a]pyridin-6-yl)pyridin-4-yl)phenyl)-2-methoxypyridin-3-yl)methyl)amino)methyl)pyrrolidin-2-one;
   (S)-5-((((6-(2-chloro-3-(3-chloro-2-(2-((((1s,4r)-4-hydroxycyclohexyl)amino)methyl)-8-methoxyimidazo[1,2-a]pyridin-6-yl)pyridin-4-yl)phenyl)-2-methoxypyridin-3-yl)methyl)amino)methyl)pyrrolidin-2-one;
   (S)-5-((((6-(2-chloro-3-(3-chloro-2-(2-((((1s,4s)-4-hydroxycyclohexyl)amino)methyl)-8-methoxyimidazo[1,2-a]pyridin-6-yl)pyridin-4-yl)phenyl)-2-methoxypyridin-3-yl)methyl)amino)methyl)pyrrolidin-2-one;
   (1r,3r)-3-(((6-(2-chloro-3-(3-chloro-2-(4-((((1r,3r)-3-hydroxy-3-methylcyclobutyl)amino)methyl)-3-methoxyphenyl)pyridin-4-yl)phenyl)-2-methoxypyridin-3-yl)methyl)amino)-1-methylcyclobutan-1-ol;
   (1r,3r)-3-(((6-(2-chloro-3-(3-chloro-2-(4-((((1r,3s)-3-hydroxy-3-methylcyclobutyl)amino)methyl)-3-methoxyphenyl)pyridin-4-yl)phenyl)-2-methoxypyridin-3-yl)methyl)amino)-1-methylcyclobutan-1-ol;
   (1r,3r)-3-(((6-(2-chloro-3-(3-chloro-2-(4-((((1s,3r)-3-hydroxy-3-methylcyclobutyl)amino)methyl)-3-methoxyphenyl)pyridin-4-yl)phenyl)-2-methoxypyridin-3-yl)methyl)amino)-1-methylcyclobutan-1-ol;
   (1r,3r)-3-(((6-(2-chloro-3-(3-chloro-2-(4-((((1s,3s)-3-hydroxy-3-methylcyclobutyl)amino)methyl)-3-methoxyphenyl)pyridin-4-yl)phenyl)-2-methoxypyridin-3-yl)methyl)amino)-1-methylcyclobutan-1-ol;
   (1r,3s)-3-(((6-(2-chloro-3-(3-chloro-2-(4-((((1r,3r)-3-hydroxy-3-methylcyclobutyl)amino)methyl)-3-methoxyphenyl)pyridin-4-yl)phenyl)-2-methoxypyridin-3-yl)methyl)amino)-1-methylcyclobutan-1-ol;
   (1r,3s)-3-(((6-(2-chloro-3-(3-chloro-2-(4-((((1r,3s)-3-hydroxy-3-methylcyclobutyl)amino)methyl)-3-methoxyphenyl)pyridin-4-yl)phenyl)-2-methoxypyridin-3-yl)methyl)amino)-1-methylcyclobutan-1-ol;
   (1r,3s)-3-(((6-(2-chloro-3-(3-chloro-2-(4-((((1s,3r)-3-hydroxy-3-methylcyclobutyl)amino)methyl)-3-methoxyphenyl)pyridin-4-yl)phenyl)-2-methoxypyridin-3-yl)methyl)amino)-1-methylcyclobutan-1-ol;
   (1r,3s)-3-(((6-(2-chloro-3-(3-chloro-2-(4-((((1s,3s)-3-hydroxy-3-methylcyclobutyl)amino)methyl)-3-methoxyphenyl)pyridin-4-yl)phenyl)-2-methoxypyridin-3-yl)methyl)amino)-1-methylcyclobutan-1-ol;
   (1s,3r)-3-(((6-(2-chloro-3-(3-chloro-2-(4-((((1r,3r)-3-hydroxy-3-methylcyclobutyl)amino)methyl)-3-methoxyphenyl)pyridin-4-yl)phenyl)-2-methoxypyridin-3-yl)methyl)amino)-1-methylcyclobutan-1-ol;
   (1s,3r)-3-(((6-(2-chloro-3-(3-chloro-2-(4-((((1r,3s)-3-hydroxy-3-methylcyclobutyl)amino)methyl)-3-methoxyphenyl)pyridin-4-yl)phenyl)-2-methoxypyridin-3-yl)methyl)amino)-1-methylcyclobutan-1-ol;
   (1s,3r)-3-(((6-(2-chloro-3-(3-chloro-2-(4-((((1s,3r)-3-hydroxy-3-methylcyclobutyl)amino)methyl)-3-methoxyphenyl)pyridin-4-yl)phenyl)-2-methoxypyridin-3-yl)methyl)amino)-1-methylcyclobutan-1-ol;
   (1s,3r)-3-(((6-(2-chloro-3-(3-chloro-2-(4-((((1s,3s)-3-hydroxy-3-methylcyclobutyl)amino)methyl)-3-methoxyphenyl)pyridin-4-yl)phenyl)-2-methoxypyridin-3-yl)methyl)amino)-1-methylcyclobutan-1-ol;
   (1s,3s)-3-(((6-(2-chloro-3-(3-chloro-2-(4-((((1r,3r)-3-hydroxy-3-methylcyclobutyl)amino)methyl)-3-methoxyphenyl)pyridin-4-yl)phenyl)-2-methoxypyridin-3-yl)methyl)amino)-1-methylcyclobutan-1-ol;
   (1s,3s)-3-(((6-(2-chloro-3-(3-chloro-2-(4-((((1r,3s)-3-hydroxy-3-methylcyclobutyl)amino)methyl)-3-methoxyphenyl)pyridin-4-yl)phenyl)-2-methoxypyridin-3-yl)methyl)amino)-1-methylcyclobutan-1-ol;
   (1s,3s)-3-(((6-(2-chloro-3-(3-chloro-2-(4-((((1s,3r)-3-hydroxy-3-methylcyclobutyl)amino)methyl)-3-methoxyphenyl)pyridin-4-yl)phenyl)-2-methoxypyridin-3-yl)methyl)amino)-1-methylcyclobutan-1-ol;
   (1s,3s)-3-(((6-(2-chloro-3-(3-chloro-2-(4-((((1s,3s)-3-hydroxy-3-methylcyclobutyl)amino)methyl)-3-methoxyphenyl)pyridin-4-yl)phenyl)-2-methoxypyridin-3-yl)methyl)amino)-1-methylcyclobutan-1-ol;
   2-(4-(3-chloro-4-(2-chloro-3-(5-((6-hydroxy-2-azaspiro[3.3]heptan-2-yl)methyl)-6-methoxypyridin-2-yl)phenyl)pyridin-2-yl)-2-methoxybenzyl)-2-azaspiro[3.3]heptan-6-ol;
   6-(4-(4-(3-(5-((2-oxa-6-azaspiro[3.3]heptan-6-yl)methyl)-6-methoxypyridin-2-yl)-2-chlorophenyl)-3-chloropyridin-2-yl)-2-methoxybenzyl)-2-oxa-6-azaspiro[3.3]heptane;
   (S)-1-((6-(3-(2-(4-(((1-acetylpiperidin-4-yl)amino)methyl)-3-methoxyphenyl)-3-chloropyridin-4-yl)-2-chlorophenyl)-2-methoxypyridin-3-yl)methyl)pyrrolidine-3-carboxylic acid;
   (R)-1-((6-(3-(2-(4-(((1-acetylpiperidin-4-yl)amino)methyl)-3-methoxyphenyl)-3-chloropyridin-4-yl)-2-chlorophenyl)-2-methoxypyridin-3-yl)methyl)pyrrolidine-3-carboxylic acid;
   3-(((6-(3-(2-(4-(((1-acetylpiperidin-4-yl)amino)methyl)-3-methoxyphenyl)-3-chloropyridin-4-yl)-2-chlorophenyl)-2-methoxypyridin-3-yl)methyl)amino)propanoic acid;
   3-((4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)amino)propanoic acid;
   1-(4-((4-(3-chloro-4-(2-chloro-3-(6-methoxy-5-(((tetrahydro-2H-pyran-4-yl)amino)methyl)pyridin-2-yl)phenyl)pyridin-2-yl)-2-methoxybenzyl)amino)piperidin-1-yl)ethan-1-one;
   1-(4-(((6-(2-chloro-3-(3-chloro-2-(3-methoxy-4-(((tetrahydro-2H-pyran-4-yl)amino)methyl)phenyl)pyridin-4-yl)phenyl)-2-methoxypyridin-3-yl)methyl)amino)piperidin-1-yl)ethan-1-one;
   (S)-1-(6-(3-(2-(4-((S)-1-aminoethyl)-3-methoxyphenyl)-3-chloropyridin-4-yl)-2-chlorophenyl)-2-methoxypyridin-3-yl)ethan-1-amine;
   (R)-1-(6-(3-(2-(4-((S)-1-aminoethyl)-3-methoxyphenyl)-3-chloropyridin-4-yl)-2-chlorophenyl)-2-methoxypyridin-3-yl)ethan-1-amine;
   (S)-1-(6-(3-(2-(4-((R)-1-aminoethyl)-3-methoxyphenyl)-3-chloropyridin-4-yl)-2-chlorophenyl)-2-methoxypyridin-3-yl)ethan-1-amine;
   (R)-1-(6-(3-(2-(4-((R)-1-aminoethyl)-3-methoxyphenyl)-3-chloropyridin-4-yl)-2-chlorophenyl)-2-methoxypyridin-3-yl)ethan-1-amine;
   1-(4-(((6-(3-(2-(4-(((1-acetylpiperidin-4-yl)amino)methyl)-3-(difluoromethoxy)phenyl)-3-chloropyridin-4-yl)-2-chlorophenyl)-2-methoxypyridin-3-yl)methyl)amino)piperidin-1-yl)ethan-1-one;
   1-(6-(3-(3-chloro-2-(3-methoxy-4-((methylamino)methyl)phenyl)pyridin-4-yl)-2-methylphenyl)-2-methoxypyridin-3-yl)-N-methylmethanamine;
   1-((6-(2-chloro-3-(3-chloro-2-(4-((3-hydroxy-3-methylazetidin-1-yl)methyl)-3-methoxyphenyl)pyridin-4-yl)phenyl)-2-methoxypyridin-3-yl)methyl)-3-methylazetidin-3-ol;
   1-(4-(((6-(2-chloro-3-(3-chloro-2-(3-methoxy-4-(((1-(2-methoxyacetyl)piperidin-4-yl)amino)methyl)phenyl)pyridin-4-yl)phenyl)-2-methoxypyridin-3-yl)methyl)amino)piperidin-1-yl)-2-methoxyethan-1-one;
   2-(4-(3-chloro-4-(2-chloro-3-(6-methoxy-5-((6-oxo-2,5-diazaspiro[3.4]octan-2-yl)methyl)pyridin-2-yl)phenyl)pyridin-2-yl)-2-methoxybenzyl)-2,5-diazaspiro[3.4]octan-6-one;
   1-(4-(((6-(3-(2-(4-(((1-acetylpiperidin-4-yl)amino)methyl)-3-methoxyphenyl)-3-chloropyridin-4-yl)-2-methylphenyl)-2-methoxypyridin-3-yl)methyl)amino)piperidin-1-yl)ethan-1-one;
   N-(1-(4-(3-chloro-4-(2-chloro-3-(6-methoxy-5-((7-oxo-2,6-diazaspiro[3.4]octan-2-yl)methyl)pyridin-2-yl)phenyl)pyridin-2-yl)-2-methoxybenzyl)piperidin-4-yl)acetamide;
   N-(1-(4-(3-chloro-4-(2-chloro-3-(6-methoxy-5-((6-oxo-2,5-diazaspiro[3.4]octan-2-yl)methyl)pyridin-2-yl)phenyl)pyridin-2-yl)-2-methoxybenzyl)piperidin-4-yl)acetamide;
   N-(1-(4-(4-(3-(5-((6-acetyl-2,6-diazaspiro[3.3]heptan-2-yl)methyl)-6-methoxypyridin-2-yl)-2-chlorophenyl)-3-chloropyridin-2-yl)-2-methoxybenzyl)piperidin-4-yl)acetamide;
   (S)-N-(1-(4-(3-chloro-4-(2-chloro-3-(6-methoxy-5-((((5-oxopyrrolidin-2-yl)methyl)amino)methyl)pyridin-2-yl)phenyl)pyridin-2-yl)-2-methoxybenzyl)piperidin-4-yl)acetamide;
   (R)-N-(1-(4-(3-chloro-4-(2-chloro-3-(6-methoxy-5-((((5-oxopyrrolidin-2-yl)methyl)amino)methyl)pyridin-2-yl)phenyl)pyridin-2-yl)-2-methoxybenzyl)piperidin-4-yl)acetamide;
   2-(4-(((1-acetylpiperidin-4-yl)amino)methyl)-3-methoxyphenyl)-4-(3-(5-(((1-acetylpiperidin-4-yl)amino)methyl)-6-methoxypyridin-2-yl)-2-chlorophenyl)nicotinonitrile;
   (S)-4-((((6-(2-chloro-3-(3-chloro-2-(3-methoxy-4-(((((S)-5-oxopyrrolidin-3-yl)methyl)amino)methyl)phenyl)pyridin-4-yl)phenyl)-2-methoxypyridin-3-yl)methyl)amino)methyl)pyrrolidin-2-one;
   (S)-4-((((6-(2-chloro-3-(3-chloro-2-(3-methoxy-4-(((((R)-5-oxopyrrolidin-3-yl)methyl)amino)methyl)phenyl)pyridin-4-yl)phenyl)-2-methoxypyridin-3-yl)methyl)amino)methyl)pyrrolidin-2-one;
   (R)-4-((((6-(2-chloro-3-(3-chloro-2-(3-methoxy-4-(((((S)-5-oxopyrrolidin-3-yl)methyl)amino)methyl)phenyl)pyridin-4-yl)phenyl)-2-methoxypyridin-3-yl)methyl)amino)methyl)pyrrolidin-2-one;
   (R)-4-((((6-(2-chloro-3-(3-chloro-2-(3-methoxy-4-(((((R)-5-oxopyrrolidin-3-yl)methyl)amino)methyl)phenyl)pyridin-4-yl)phenyl)-2-methoxypyridin-3-yl)methyl)amino)methyl)pyrrolidin-2-one;
   1-(4-(((6-(3-(2-(4-((4-amino-4-methylpiperidin-1-yl)methyl)-3-methoxyphenyl)-3-chloropyridin-4-yl)-2-chlorophenyl)-2-methoxypyridin-3-yl)methyl)amino)piperidin-1-yl)ethan-1-one;
   (S)-4-(((4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)amino)methyl)pyrrolidin-2-one;
   (R)-4-(((4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)amino)methyl)pyrrolidin-2-one;
   2-(4-(3-chloro-4-(3-(6-methoxy-5-((7-oxo-2,6-diazaspiro[3.4]octan-2-yl)methyl)pyridin-2-yl)-2-methylphenyl)pyridin-2-yl)-2-methoxybenzyl)-2,6-diazaspiro[3.4]octan-7-one;
   1-(6-((6-(3-(2-(4-((6-acetyl-2,6-diazaspiro[3.3]heptan-2-yl)methyl)-3-methoxyphenyl)-3-chloropyridin-4-yl)-2-methylphenyl)-2-methoxypyridin-3-yl)methyl)-2,6-diazaspiro[3.3]heptan-2-yl)ethan-1-one;
   (1s,4s)-4-(((6-(2-chloro-3-(3-chloro-2-(4-((((1s,4s)-4-hydroxycyclohexyl)amino)methyl)-3-methoxyphenyl)pyridin-4-yl)phenyl)-2-methoxypyridin-3-yl)methyl)amino)cyclohexan-1-ol;
   (1r,4r)-4-(((6-(2-chloro-3-(3-chloro-2-(4-((((1r,4r)-4-hydroxycyclohexyl)amino)methyl)-3-methoxyphenyl)pyridin-4-yl)phenyl)-2-methoxypyridin-3-yl)methyl)amino)cyclohexan-1-ol;
   (1r,4r)-4-(((6-(2-chloro-3-(3-chloro-2-(4-((((1r,4s)-4-hydroxycyclohexyl)amino)methyl)-3-methoxyphenyl)pyridin-4-yl)phenyl)-2-methoxypyridin-3-yl)methyl)amino)cyclohexan-1-ol;
   (1r,4r)-4-(((6-(2-chloro-3-(3-chloro-2-(4-((((1s,4r)-4-hydroxycyclohexyl)amino)methyl)-3-methoxyphenyl)pyridin-4-yl)phenyl)-2-methoxypyridin-3-yl)methyl)amino)cyclohexan-1-ol;
   (1r,4r)-4-(((6-(2-chloro-3-(3-chloro-2-(4-((((1s,4s)-4-hydroxycyclohexyl)amino)methyl)-3-methoxyphenyl)pyridin-4-yl)phenyl)-2-methoxypyridin-3-yl)methyl)amino)cyclohexan-1-ol;
   (1r,4s)-4-(((6-(2-chloro-3-(3-chloro-2-(4-((((1r,4r)-4-hydroxycyclohexyl)amino)methyl)-3-methoxyphenyl)pyridin-4-yl)phenyl)-2-methoxypyridin-3-yl)methyl)amino)cyclohexan-1-ol;
   (1r,4s)-4-(((6-(2-chloro-3-(3-chloro-2-(4-((((1r,4s)-4-hydroxycyclohexyl)amino)methyl)-3-methoxyphenyl)pyridin-4-yl)phenyl)-2-methoxypyridin-3-yl)methyl)amino)cyclohexan-1-ol;
   (1r,4s)-4-(((6-(2-chloro-3-(3-chloro-2-(4-((((1s,4r)-4-hydroxycyclohexyl)amino)methyl)-3-methoxyphenyl)pyridin-4-yl)phenyl)-2-methoxypyridin-3-yl)methyl)amino)cyclohexan-1-ol;
   (1r,4s)-4-(((6-(2-chloro-3-(3-chloro-2-(4-((((1s,4s)-4-hydroxycyclohexyl)amino)methyl)-3-methoxyphenyl)pyridin-4-yl)phenyl)-2-methoxypyridin-3-yl)methyl)amino)cyclohexan-1-ol;
   (1s,4r)-4-(((6-(2-chloro-3-(3-chloro-2-(4-((((1r,4r)-4-hydroxycyclohexyl)amino)methyl)-3-methoxyphenyl)pyridin-4-yl)phenyl)-2-methoxypyridin-3-yl)methyl)amino)cyclohexan-1-ol;
   (1s,4r)-4-(((6-(2-chloro-3-(3-chloro-2-(4-((((1r,4s)-4-hydroxycyclohexyl)amino)methyl)-3-methoxyphenyl)pyridin-4-yl)phenyl)-2-methoxypyridin-3-yl)methyl)amino)cyclohexan-1-ol;
   (1s,4r)-4-(((6-(2-chloro-3-(3-chloro-2-(4-((((1s,4r)-4-hydroxycyclohexyl)amino)methyl)-3-methoxyphenyl)pyridin-4-yl)phenyl)-2-methoxypyridin-3-yl)methyl)amino)cyclohexan-1-ol;
   (1s,4r)-4-(((6-(2-chloro-3-(3-chloro-2-(4-((((1s,4s)-4-hydroxycyclohexyl)amino)methyl)-3-methoxyphenyl)pyridin-4-yl)phenyl)-2-methoxypyridin-3-yl)methyl)amino)cyclohexan-1-ol;
   (1s,4s)-4-(((6-(2-chloro-3-(3-chloro-2-(4-((((1r,4r)-4-hydroxycyclohexyl)amino)methyl)-3-methoxyphenyl)pyridin-4-yl)phenyl)-2-methoxypyridin-3-yl)methyl)amino)cyclohexan-1-ol;
   (1s,4s)-4-(((6-(2-chloro-3-(3-chloro-2-(4-((((1r,4s)-4-hydroxycyclohexyl)amino)methyl)-3-methoxyphenyl)pyridin-4-yl)phenyl)-2-methoxypyridin-3-yl)methyl)amino)cyclohexan-1-ol;
   (1s,4s)-4-(((6-(2-chloro-3-(3-chloro-2-(4-((((1s,4r)-4-hydroxycyclohexyl)amino)methyl)-3-methoxyphenyl)pyridin-4-yl)phenyl)-2-methoxypyridin-3-yl)methyl)amino)cyclohexan-1-ol;
   4-((6-(2-chloro-3-(3-chloro-2-(3-methoxy-4-(morpholinomethyl)phenyl)pyridin-4-yl)phenyl)-2-methoxypyridin-3-yl)methyl)morpholine;
   1-((6-(2-chloro-3-(3-chloro-2-(4-((4-hydroxypiperidin-1-yl)methyl)-3-methoxyphenyl)pyridin-4-yl)phenyl)-2-methoxypyridin-3-yl)methyl)piperidin-4-ol;
   1-(4-((4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)amino)piperidin-1-yl)-2-methoxyethan-1-one;
   (S)-5-((((6-(2-chloro-3-(2-(3-methoxy-4-(((((S)-5-oxopyrrolidin-2-yl)methyl)amino)methyl)phenyl)-3-methylpyridin-4-yl)phenyl)-2-methoxypyridin-3-yl)methyl)amino)methyl)pyrrolidin-2-one;
   (S)-5-((((6-(2-chloro-3-(2-(3-methoxy-4-(((((R)-5-oxopyrrolidin-2-yl)methyl)amino)methyl)phenyl)-3-methylpyridin-4-yl)phenyl)-2-methoxypyridin-3-yl)methyl)amino)methyl)pyrrolidin-2-one;
   (R)-5-((((6-(2-chloro-3-(2-(3-methoxy-4-(((((S)-5-oxopyrrolidin-2-yl)methyl)amino)methyl)phenyl)-3-methylpyridin-4-yl)phenyl)-2-methoxypyridin-3-yl)methyl)amino)methyl)pyrrolidin-2-one;
   (R)-5-((((6-(2-chloro-3-(2-(3-methoxy-4-(((((R)-5-oxopyrrolidin-2-yl)methyl)amino)methyl)phenyl)-3-methylpyridin-4-yl)phenyl)-2-methoxypyridin-3-yl)methyl)amino)methyl)pyrrolidin-2-one;
   2-(2-methoxy-4-(4-(3-(6-methoxy-5-((7-oxo-2,6-diazaspiro[3.4]octan-2-yl)methyl)pyridin-2-yl)-2-methylphenyl)-3-methylpyridin-2-yl)benzyl)-2,6-diazaspiro[3.4]octan-7-one;
   1-(6-((6-(3-(2-(4-((6-acetyl-2,6-diazaspiro[3.3]heptan-2-yl)methyl)-3-methoxyphenyl)-3-methylpyridin-4-yl)-2-methylphenyl)-2-methoxypyridin-3-yl)methyl)-2,6-diazaspiro[3.3]heptan-2-yl)ethan-1-one;
   1-(4-(((6-(2-chloro-3-(3-chloro-2-(3-methoxy-4-((piperidin-4-ylamino)methyl)phenyl)pyridin-4-yl)phenyl)-2-methoxypyridin-3-yl)methyl)amino)piperidin-1-yl)ethan-1-one;
   4-(((6-(2-chloro-3-(3-chloro-2-(3-methoxy-4-(((1-methyl-2-oxopiperidin-4-yl)amino)methyl)phenyl)pyridin-4-yl)phenyl)-2-methoxypyridin-3-yl)methyl)amino)-1-methylpiperidin-2-one;
   4-(((6-(2-chloro-3-(3-chloro-2-(4-(((1-(dimethylcarbamoyl)piperidin-4-yl)amino)methyl)-3-methoxyphenyl)pyridin-4-yl)phenyl)-2-methoxypyridin-3-yl)methyl)amino)-N,N-dimethylpiperidine-1-carboxamide;
   N-((6-(2-chloro-3-(3-chloro-2-(4-(((1-(2,2-difluoroethyl)piperidin-4-yl)amino)methyl)-3-methoxyphenyl)pyridin-4-yl)phenyl)-2-methoxypyridin-3-yl)methyl)-1-(2,2-difluoroethyl)piperidin-4-amine;
   (4-(((6-(2-chloro-3-(3-chloro-2-(4-(((1-(furan-2-carbonyl)piperidin-4-yl)amino)methyl)-3-methoxyphenyl)pyridin-4-yl)phenyl)-2-methoxypyridin-3-yl)methyl)amino)piperidin-1-yl)(furan-2-yl)methanone;
   (4-(((6-(3-(2-(4-(((1-benzoylpiperidin-4-yl)amino)methyl)-3-methoxyphenyl)-3-chloropyridin-4-yl)-2-chlorophenyl)-2-methoxypyridin-3-yl)methyl)amino)piperidin-1-yl)(phenyl)methanone;
   N-((6-(2-chloro-3-(3-chloro-2-(3-methoxy-4-(((1-phenylpiperidin-4-yl)amino)methyl)phenyl)pyridin-4-yl)phenyl)-2-methoxypyridin-3-yl)methyl)-1-phenylpiperidin-4-amine;
   N-((6-(2-chloro-3-(3-chloro-2-(3-methoxy-4-(((1-(pyridin-4-yl)piperidin-4-yl)amino)methyl)phenyl)pyridin-4-yl)phenyl)-2-methoxypyridin-3-yl)methyl)-1-(pyridin-4-yl)piperidin-4-amine;
   N-(2-methoxy-4-(4-(3-(6-methoxy-5-(((tetrahydro-2H-pyran-4-yl)amino)methyl)pyridin-2-yl)-2-methylphenyl)-3-methylpyridin-2-yl)benzyl)tetrahydro-2H-pyran-4-amine;
   N-((6-(3-(3-chloro-2-(3-methoxy-4-(((tetrahydro-2H-pyran-4-yl)amino)methyl)phenyl)pyridin-4-yl)-2-methylphenyl)-2-methoxypyridin-3-yl)methyl)tetrahydro-2H-pyran-4-amine;
   1-(4-(((6-(2-chloro-3-(3-chloro-2-(3-methoxy-4-((methyl(tetrahydro-2H-pyran-4-yl)amino)methyl)phenyl)pyridin-4-yl)phenyl)-2-methoxypyridin-3-yl)methyl)amino)piperidin-1-yl)ethan-1-one;
   1-(6-((6-(3-(6'-((6-acetyl-2,6-diazaspiro[3.3]heptan-2-yl)methyl)-3-chloro-5'-methoxy-[2,3'-bipyridin]-4-yl)-2-chlorophenyl)-2-methoxypyridin-3-yl)methyl)-2,6-diazaspiro[3.3]heptan-2-yl)ethan-1-one;
   N-((4-(3-(5-(((2-oxaspiro[3.3]heptan-6-yl)amino)methyl)-6-methoxypyridin-2-yl)-2-chlorophenyl)-3-chloro-5'-methoxy-[2,3'-bipyridin]-6'-yl)methyl)-2-oxaspiro[3.3]heptan-6-amine;
   2-((3-chloro-4-(2-chloro-3-(6-methoxy-5-((6-oxo-2,5-diazaspiro[3.4]octan-2-yl)methyl)pyridin-2-yl)phenyl)-5'-methoxy-[2,3'-bipyridin]-6'-yl)methyl)-2,5-diazaspiro[3.4]octan-6-one;
   N-((6-(2-chloro-3-(3-chloro-5'-methoxy-6'-(((tetrahydro-2H-pyran-4-yl)amino)methyl)-[2,3'-bipyridin]-4-yl)phenyl)-2-methoxypyridin-3-yl)methyl)tetrahydro-2H-pyran-4-amine;
   N-(1-((6-(2-chloro-3-(3-chloro-2-(3-methoxy-4-(((tetrahydro-2H-pyran-4-yl)amino)methyl)phenyl)pyridin-4-yl)phenyl)-2-methoxypyridin-3-yl)methyl)piperidin-4-yl)acetamide;
   4-(2-chloro-3-(6-methoxy-5-((7-oxo-2,6-diazaspiro[3.4]octan-2-yl)methyl)pyridin-2-yl)phenyl)-2-(3-methoxy-4-((7-oxo-2,6-diazaspiro[3.4]octan-2-yl)methyl)phenyl)nicotinonitrile;
   4-(2-chloro-3-(6-methoxy-5-(((tetrahydro-2H-pyran-4-yl)amino)methyl)pyridin-2-yl)phenyl)-2-(3-methoxy-4-(((tetrahydro-2H-pyran-4-yl)amino)methyl)phenyl)nicotinonitrile;
   (S)-1-((6-(2-chloro-3-(3-chloro-2-(4-(((S)-3-hydroxypyrrolidin-1-yl)methyl)-3-methoxyphenyl)pyridin-4-yl)phenyl)-2-methoxypyridin-3-yl)methyl)pyrrolidin-3-ol;
   (S)-1-((6-(2-chloro-3-(3-chloro-2-(4-(((R)-3-hydroxypyrrolidin-1-yl)methyl)-3-methoxyphenyl)pyridin-4-yl)phenyl)-2-methoxypyridin-3-yl)methyl)pyrrolidin-3-ol;
   (R)-1-((6-(2-chloro-3-(3-chloro-2-(4-(((S)-3-hydroxypyrrolidin-1-yl)methyl)-3-methoxyphenyl)pyridin-4-yl)phenyl)-2-methoxypyridin-3-yl)methyl)pyrrolidin-3-ol;
   (R)-1-((6-(2-chloro-3-(3-chloro-2-(4-(((R)-3-hydroxypyrrolidin-1-yl)methyl)-3-methoxyphenyl)pyridin-4-yl)phenyl)-2-methoxypyridin-3-yl)methyl)pyrrolidin-3-ol;
   1-(4-((4-(3-chloro-4-(2-chloro-3-(6-methoxy-5-((methyl(tetrahydro-2H-pyran-4-yl)amino)methyl)pyridin-2-yl)phenyl)pyridin-2-yl)-2-methoxybenzyl)amino)piperidin-1-yl)ethan-1-one;
   N-((6-(2-chloro-3-(3-chloro-2-(3-methoxy-4-(((1-(2,2,2-trifluoroethyl)piperidin-4-yl)amino)methyl)phenyl)pyridin-4-yl)phenyl)-2-methoxypyridin-3-yl)methyl)-1-(2,2,2-trifluoroethyl)piperidin-4-amine;
   2-(4-(3-chloro-4-(2-fluoro-3-(6-methoxy-5-((7-oxo-2,6-diazaspiro[3.4]octan-2-yl)methyl)pyridin-2-yl)phenyl)pyridin-2-yl)-2-methoxybenzyl)-2,6-diazaspiro[3.4]octan-7-one;
   1-(4-(((6-(3-(2-(4-(((1-acetylpiperidin-4-yl)amino)methyl)-3-methoxyphenyl)-3-chloropyridin-4-yl)-2-fluorophenyl)-2-methoxypyridin-3-yl)methyl)amino)piperidin-1-yl)ethan-1-one;
   N-((6-(3-(3-chloro-2-(3-methoxy-4-(((tetrahydro-2H-pyran-4-yl)amino)methyl)phenyl)pyridin-4-yl)-2-fluorophenyl)-2-methoxypyridin-3-yl)methyl)tetrahydro-2H-pyran-4-amine;
   N-(4-(3-chloro-4-(2-chloro-3-(6-methoxy-5-(((tetrahydro-2H-pyran-4-yl)amino)methyl)pyridin-2-yl)phenyl)pyridin-2-yl)-2-methoxybenzyl)-N-methyltetrahydro-2H-pyran-4-amine;
   1-(4-((4-(3-chloro-4-(2-chloro-3-(6-methoxy-5-(((tetrahydro-2H-pyran-4-yl)amino)methyl)pyridin-2-yl)phenyl)pyridin-2-yl)-2-methoxybenzyl)(methyl)amino)piperidin-1-yl)ethan-1-one;
   1-(4-(((6-(2-chloro-3-(3-chloro-2-(4-(((1-(2-hydroxyacetyl)piperidin-4-yl)amino)methyl)-3-methoxyphenyl)pyridin-4-yl)phenyl)-2-methoxypyridin-3-yl)methyl)amino)piperidin-1-yl)-2-hydroxyethan-1-one;
   N-((6-(2-chloro-3-(3-chloro-2-(3-methoxy-4-(((1-(pyridin-2-yl)piperidin-4-yl)amino)methyl)phenyl)pyridin-4-yl)phenyl)-2-methoxypyridin-3-yl)methyl)-1-(pyridin-2-yl)piperidin-4-amine;
   N-((6-(2-chloro-3-(3-chloro-2-(4-(((1-(3-chloropyridin-2-yl)piperidin-4-yl)amino)methyl)-3-methoxyphenyl)pyridin-4-yl)phenyl)-2-methoxypyridin-3-yl)methyl)-1-(3-chloropyridin-2-yl)piperidin-4-amine;
   N-((6-(2-chloro-3-(3-chloro-2-(3-methoxy-4-(((2-methoxyethyl)amino)methyl)phenyl)pyridin-4-yl)phenyl)-2-methoxypyridin-3-yl)methyl)-2-methoxyethan-1-amine;
   3-chloro-4-(2-chloro-3-(6-methoxy-5-((4-methoxypiperidin-1-yl)methyl)pyridin-2-yl)phenyl)-2-(3-methoxy-4-((4-methoxypiperidin-1-yl)methyl)phenyl)pyridine;
   (1s,4s)-N-((6-(2-chloro-3-(3-chloro-2-(3-methoxy-4-((((1s,4s)-4-methoxycyclohexyl)amino)methyl)phenyl)pyridin-4-yl)phenyl)-2-methoxypyridin-3-yl)methyl)-4-methoxycyclohexan-1-amine;
   (1r,4r)-N-((6-(2-chloro-3-(3-chloro-2-(3-methoxy-4-((((1r,4r)-4-methoxycyclohexyl)amino)methyl)phenyl)pyridin-4-yl)phenyl)-2-methoxypyridin-3-yl)methyl)-4-methoxycyclohexan-1-amine;
   (1r,4r)-N-((6-(2-chloro-3-(3-chloro-2-(3-methoxy-4-((((1r,4s)-4-methoxycyclohexyl)amino)methyl)phenyl)pyridin-4-yl)phenyl)-2-methoxypyridin-3-yl)methyl)-4-methoxycyclohexan-1-amine;
   (1r,4r)-N-((6-(2-chloro-3-(3-chloro-2-(3-methoxy-4-((((1s,4r)-4-methoxycyclohexyl)amino)methyl)phenyl)pyridin-4-yl)phenyl)-2-methoxypyridin-3-yl)methyl)-4-methoxycyclohexan-1-amine;
   (1r,4r)-N-((6-(2-chloro-3-(3-chloro-2-(3-methoxy-4-((((1s,4s)-4-methoxycyclohexyl)amino)methyl)phenyl)pyridin-4-yl)phenyl)-2-methoxypyridin-3-yl)methyl)-4-methoxycyclohexan-1-amine;
   (1r,4s)-N-((6-(2-chloro-3-(3-chloro-2-(3-methoxy-4-((((1r,4r)-4-methoxycyclohexyl)amino)methyl)phenyl)pyridin-4-yl)phenyl)-2-methoxypyridin-3-yl)methyl)-4-methoxycyclohexan-1-amine;
   (1r,4s)-N-((6-(2-chloro-3-(3-chloro-2-(3-methoxy-4-((((1r,4s)-4-methoxycyclohexyl)amino)methyl)phenyl)pyridin-4-yl)phenyl)-2-methoxypyridin-3-yl)methyl)-4-methoxycyclohexan-1-amine;
   (1r,4s)-N-((6-(2-chloro-3-(3-chloro-2-(3-methoxy-4-((((1s,4r)-4-methoxycyclohexyl)amino)methyl)phenyl)pyridin-4-yl)phenyl)-2-methoxypyridin-3-yl)methyl)-4-methoxycyclohexan-1-amine;
   (1r,4s)-N-((6-(2-chloro-3-(3-chloro-2-(3-methoxy-4-((((1s,4s)-4-methoxycyclohexyl)amino)methyl)phenyl)pyridin-4-yl)phenyl)-2-methoxypyridin-3-yl)methyl)-4-methoxycyclohexan-1-amine;
   (1s,4r)-N-((6-(2-chloro-3-(3-chloro-2-(3-methoxy-4-((((1r,4r)-4-methoxycyclohexyl)amino)methyl)phenyl)pyridin-4-yl)phenyl)-2-methoxypyridin-3-yl)methyl)-4-methoxycyclohexan-1-amine;
   (1s,4r)-N-((6-(2-chloro-3-(3-chloro-2-(3-methoxy-4-((((1r,4s)-4-methoxycyclohexyl)amino)methyl)phenyl)pyridin-4-yl)phenyl)-2-methoxypyridin-3-yl)methyl)-4-methoxycyclohexan-1-amine;
   (1s,4r)-N-((6-(2-chloro-3-(3-chloro-2-(3-methoxy-4-((((1s,4r)-4-methoxycyclohexyl)amino)methyl)phenyl)pyridin-4-yl)phenyl)-2-methoxypyridin-3-yl)methyl)-4-methoxycyclohexan-1-amine;
   (1s,4r)-N-((6-(2-chloro-3-(3-chloro-2-(3-methoxy-4-((((1s,4s)-4-methoxycyclohexyl)amino)methyl)phenyl)pyridin-4-yl)phenyl)-2-methoxypyridin-3-yl)methyl)-4-methoxycyclohexan-1-amine;
   (1s,4s)-N-((6-(2-chloro-3-(3-chloro-2-(3-methoxy-4-((((1r,4r)-4-methoxycyclohexyl)amino)methyl)phenyl)pyridin-4-yl)phenyl)-2-methoxypyridin-3-yl)methyl)-4-methoxycyclohexan-1-amine;
   (1s,4s)-N-((6-(2-chloro-3-(3-chloro-2-(3-methoxy-4-((((1r,4s)-4-methoxycyclohexyl)amino)methyl)phenyl)pyridin-4-yl)phenyl)-2-methoxypyridin-3-yl)methyl)-4-methoxycyclohexan-1-amine;
   (1s,4s)-N-((6-(2-chloro-3-(3-chloro-2-(3-methoxy-4-((((1s,4r)-4-methoxycyclohexyl)amino)methyl)phenyl)pyridin-4-yl)phenyl)-2-methoxypyridin-3-yl)methyl)-4-methoxycyclohexan-1-amine;
   1-(4-(((6-(3-(2-(4-(((1-acetylpiperidin-4-yl)amino)methyl)-3-methoxyphenyl)-3-fluoropyridin-4-yl)-2-fluorophenyl)-2-methoxypyridin-3-yl)methyl)amino)piperidin-1-yl)ethan-1-one;
   (1r,4r)-N-((6-(2-chloro-3-(3-chloro-2-(3-methoxy-4-((((1r,4r)-4-methoxycyclohexyl)amino)methyl)phenyl)pyridin-4-yl)phenyl)-2-methoxypyridin-3-yl)methyl)-4-methoxycyclohexan-1-amine;
   (1r,4r)-N-((6-(2-chloro-3-(3-chloro-2-(3-methoxy-4-((((1r,4s)-4-methoxycyclohexyl)amino)methyl)phenyl)pyridin-4-yl)phenyl)-2-methoxypyridin-3-yl)methyl)-4-methoxycyclohexan-1-amine;
   (1r,4r)-N-((6-(2-chloro-3-(3-chloro-2-(3-methoxy-4-((((1s,4r)-4-methoxycyclohexyl)amino)methyl)phenyl)pyridin-4-yl)phenyl)-2-methoxypyridin-3-yl)methyl)-4-methoxycyclohexan-1-amine;
   (1r,4r)-N-((6-(2-chloro-3-(3-chloro-2-(3-methoxy-4-((((1s,4s)-4-methoxycyclohexyl)amino)methyl)phenyl)pyridin-4-yl)phenyl)-2-methoxypyridin-3-yl)methyl)-4-methoxycyclohexan-1-amine;
   (1r,4s)-N-((6-(2-chloro-3-(3-chloro-2-(3-methoxy-4-((((1r,4r)-4-methoxycyclohexyl)amino)methyl)phenyl)pyridin-4-yl)phenyl)-2-methoxypyridin-3-yl)methyl)-4-methoxycyclohexan-1-amine;
   (1r,4s)-N-((6-(2-chloro-3-(3-chloro-2-(3-methoxy-4-((((1r,4s)-4-methoxycyclohexyl)amino)methyl)phenyl)pyridin-4-yl)phenyl)-2-methoxypyridin-3-yl)methyl)-4-methoxycyclohexan-1-amine;
   (1r,4s)-N-((6-(2-chloro-3-(3-chloro-2-(3-methoxy-4-((((1s,4r)-4-methoxycyclohexyl)amino)methyl)phenyl)pyridin-4-yl)phenyl)-2-methoxypyridin-3-yl)methyl)-4-methoxycyclohexan-1-amine;
   (1r,4s)-N-((6-(2-chloro-3-(3-chloro-2-(3-methoxy-4-((((1s,4s)-4-methoxycyclohexyl)amino)methyl)phenyl)pyridin-4-yl)phenyl)-2-methoxypyridin-3-yl)methyl)-4-methoxycyclohexan-1-amine;
   (1s,4r)-N-((6-(2-chloro-3-(3-chloro-2-(3-methoxy-4-((((1r,4r)-4-methoxycyclohexyl)amino)methyl)phenyl)pyridin-4-yl)phenyl)-2-methoxypyridin-3-yl)methyl)-4-methoxycyclohexan-1-amine;
   (1s,4r)-N-((6-(2-chloro-3-(3-chloro-2-(3-methoxy-4-((((1r,4s)-4-methoxycyclohexyl)amino)methyl)phenyl)pyridin-4-yl)phenyl)-2-methoxypyridin-3-yl)methyl)-4-methoxycyclohexan-1-amine;
   (1s,4r)-N-((6-(2-chloro-3-(3-chloro-2-(3-methoxy-4-((((1s,4r)-4-methoxycyclohexyl)amino)methyl)phenyl)pyridin-4-yl)phenyl)-2-methoxypyridin-3-yl)methyl)-4-methoxycyclohexan-1-amine;
   (1s,4r)-N-((6-(2-chloro-3-(3-chloro-2-(3-methoxy-4-((((1s,4s)-4-methoxycyclohexyl)amino)methyl)phenyl)pyridin-4-yl)phenyl)-2-methoxypyridin-3-yl)methyl)-4-methoxycyclohexan-1-amine;
   (1s,4s)-N-((6-(2-chloro-3-(3-chloro-2-(3-methoxy-4-((((1r,4r)-4-methoxycyclohexyl)amino)methyl)phenyl)pyridin-4-yl)phenyl)-2-methoxypyridin-3-yl)methyl)-4-methoxycyclohexan-1-amine;
   (1s,4s)-N-((6-(2-chloro-3-(3-chloro-2-(3-methoxy-4-((((1r,4s)-4-methoxycyclohexyl)amino)methyl)phenyl)pyridin-4-yl)phenyl)-2-methoxypyridin-3-yl)methyl)-4-methoxycyclohexan-1-amine;
   (1s,4s)-N-((6-(2-chloro-3-(3-chloro-2-(3-methoxy-4-((((1s,4r)-4-methoxycyclohexyl)amino)methyl)phenyl)pyridin-4-yl)phenyl)-2-methoxypyridin-3-yl)methyl)-4-methoxycyclohexan-1-amine;
   (1s,4s)-N-((6-(2-chloro-3-(3-chloro-2-(3-methoxy-4-((((1s,4s)-4-methoxycyclohexyl)amino)methyl)phenyl)pyridin-4-yl)phenyl)-2-methoxypyridin-3-yl)methyl)-4-methoxycyclohexan-1-amine;
   2-(4-(3-fluoro-4-(2-fluoro-3-(6-methoxy-5-((7-oxo-2,6-diazaspiro[3.4]octan-2-yl)methyl)pyridin-2-yl)phenyl)pyridin-2-yl)-2-methoxybenzyl)-2,6-diazaspiro[3.4]octan-7-one;
   N-((6-(2-fluoro-3-(3-fluoro-2-(3-methoxy-4-(((tetrahydro-2H-pyran-4-yl)amino)methyl)phenyl)pyridin-4-yl)phenyl)-2-methoxypyridin-3-yl)methyl)tetrahydro-2H-pyran-4-amine;
   (1r,4r)-4-(((6-(2-chloro-3-(3-chloro-2-(2-((((1r,4r)-4-hydroxycyclohexyl)amino)methyl)-8-methoxyimidazo[1,2-a]pyridin-6-yl)pyridin-4-yl)phenyl)-2-methoxypyridin-3-yl)methyl)amino)cyclohexan-1-ol;
   (1r,4r)-4-(((6-(2-chloro-3-(3-chloro-2-(2-((((1r,4s)-4-hydroxycyclohexyl)amino)methyl)-8-methoxyimidazo[1,2-a]pyridin-6-yl)pyridin-4-yl)phenyl)-2-methoxypyridin-3-yl)methyl)amino)cyclohexan-1-ol;
   (1r,4r)-4-(((6-(2-chloro-3-(3-chloro-2-(2-((((1s,4r)-4-hydroxycyclohexyl)amino)methyl)-8-methoxyimidazo[1,2-a]pyridin-6-yl)pyridin-4-yl)phenyl)-2-methoxypyridin-3-yl)methyl)amino)cyclohexan-1-ol;
   (1r,4r)-4-(((6-(2-chloro-3-(3-chloro-2-(2-((((1s,4s)-4-hydroxycyclohexyl)amino)methyl)-8-methoxyimidazo[1,2-a]pyridin-6-yl)pyridin-4-yl)phenyl)-2-methoxypyridin-3-yl)methyl)amino)cyclohexan-1-ol;
   (1r,4s)-4-(((6-(2-chloro-3-(3-chloro-2-(2-((((1r,4r)-4-hydroxycyclohexyl)amino)methyl)-8-methoxyimidazo[1,2-a]pyridin-6-yl)pyridin-4-yl)phenyl)-2-methoxypyridin-3-yl)methyl)amino)cyclohexan-1-ol;
   (1r,4s)-4-(((6-(2-chloro-3-(3-chloro-2-(2-((((1r,4s)-4-hydroxycyclohexyl)amino)methyl)-8-methoxyimidazo[1,2-a]pyridin-6-yl)pyridin-4-yl)phenyl)-2-methoxypyridin-3-yl)methyl)amino)cyclohexan-1-ol;
   (1r,4s)-4-(((6-(2-chloro-3-(3-chloro-2-(2-((((1s,4r)-4-hydroxycyclohexyl)amino)methyl)-8-methoxyimidazo[1,2-a]pyridin-6-yl)pyridin-4-yl)phenyl)-2-methoxypyridin-3-yl)methyl)amino)cyclohexan-1-ol;
   (1r,4s)-4-(((6-(2-chloro-3-(3-chloro-2-(2-((((1s,4s)-4-hydroxycyclohexyl)amino)methyl)-8-methoxyimidazo[1,2-a]pyridin-6-yl)pyridin-4-yl)phenyl)-2-methoxypyridin-3-yl)methyl)amino)cyclohexan-1-ol;
   (1s,4r)-4-(((6-(2-chloro-3-(3-chloro-2-(2-((((1r,4r)-4-hydroxycyclohexyl)amino)methyl)-8-methoxyimidazo[1,2-a]pyridin-6-yl)pyridin-4-yl)phenyl)-2-methoxypyridin-3-yl)methyl)amino)cyclohexan-1-ol;
   (1s,4r)-4-(((6-(2-chloro-3-(3-chloro-2-(2-((((1r,4s)-4-hydroxycyclohexyl)amino)methyl)-8-methoxyimidazo[1,2-a]pyridin-6-yl)pyridin-4-yl)phenyl)-2-methoxypyridin-3-yl)methyl)amino)cyclohexan-1-ol;
   (1s,4r)-4-(((6-(2-chloro-3-(3-chloro-2-(2-((((1s,4r)-4-hydroxycyclohexyl)amino)methyl)-8-methoxyimidazo[1,2-a]pyridin-6-yl)pyridin-4-yl)phenyl)-2-methoxypyridin-3-yl)methyl)amino)cyclohexan-1-ol;
   (1s,4r)-4-(((6-(2-chloro-3-(3-chloro-2-(2-((((1s,4s)-4-hydroxycyclohexyl)amino)methyl)-8-methoxyimidazo[1,2-a]pyridin-6-yl)pyridin-4-yl)phenyl)-2-methoxypyridin-3-yl)methyl)amino)cyclohexan-1-ol;
   (1s,4s)-4-(((6-(2-chloro-3-(3-chloro-2-(2-((((1r,4r)-4-hydroxycyclohexyl)amino)methyl)-8-methoxyimidazo[1,2-a]pyridin-6-yl)pyridin-4-yl)phenyl)-2-methoxypyridin-3-yl)methyl)amino)cyclohexan-1-ol;
   (1s,4s)-4-(((6-(2-chloro-3-(3-chloro-2-(2-((((1r,4s)-4-hydroxycyclohexyl)amino)methyl)-8-methoxyimidazo[1,2-a]pyridin-6-yl)pyridin-4-yl)phenyl)-2-methoxypyridin-3-yl)methyl)amino)cyclohexan-1-ol;
   (1s,4s)-4-(((6-(2-chloro-3-(3-chloro-2-(2-((((1s,4r)-4-hydroxycyclohexyl)amino)methyl)-8-methoxyimidazo[1,2-a]pyridin-6-yl)pyridin-4-yl)phenyl)-2-methoxypyridin-3-yl)methyl)amino)cyclohexan-1-ol;
   (1s,4s)-4-(((6-(2-chloro-3-(3-chloro-2-(2-((((1s,4s)-4-hydroxycyclohexyl)amino)methyl)-8-methoxyimidazo[1,2-a]pyridin-6-yl)pyridin-4-yl)phenyl)-2-methoxypyridin-3-yl)methyl)amino)cyclohexan-1-ol;
   2-((6-(3-chloro-4-(2-chloro-3-(6-methoxy-5-((6-oxo-2,5-diazaspiro[3.4]octan-2-yl)methyl)pyridin-2-yl)phenyl)pyridin-2-yl)-8-methoxyimidazo[1,2-a]pyridin-2-yl)methyl)-2,5-diazaspiro[3.4]octan-6-one;
   1-((6-(2-chloro-3-(3-chloro-2-(2-((3-hydroxy-3-methylazetidin-1-yl)methyl)-8-methoxyimidazo[1,2-a]pyridin-6-yl)pyridin-4-yl)phenyl)-2-methoxypyridin-3-yl)methyl)-3-methylazetidin-3-ol;
   (4-(((6-(2-chloro-3-(3-chloro-2-(4-(((1-(cyclohexanecarbonyl)piperidin-4-yl)amino)methyl)-3-methoxyphenyl)pyridin-4-yl)phenyl)-2-methoxypyridin-3-yl)methyl)amino)piperidin-1-yl)(cyclohexyl)methanone;
   4-((6-(2-chloro-3-(3-chloro-2-(3-methoxy-4-((3-oxopiperazin-1-yl)methyl)phenyl)pyridin-4-yl)phenyl)-2-methoxypyridin-3-yl)methyl)piperazin-2-one;
   1-(4-(((6-(2-chloro-3-(3-chloro-2-(3-methoxy-4-(morpholinomethyl)phenyl)pyridin-4-yl)phenyl)-2-methoxypyridin-3-yl)methyl)amino)piperidin-1-yl)ethan-1-one;
   1-(4-(((6-(2-chloro-3-(3-chloro-2-(4-((4-hydroxypiperidin-1-yl)methyl)-3-methoxyphenyl)pyridin-4-yl)phenyl)-2-methoxypyridin-3-yl)methyl)amino)piperidin-1-yl)ethan-1-one;
   (S)-(2-(6-(3-chloro-4-(2-chloro-3-(6-methoxy-5-((((5-oxopyrrolidin-2-yl)methyl)amino)methyl)pyridin-2-yl)phenyl)pyridin-2-yl)-2-methoxy-4-oxopyrrolo[2,1-f][1,2,4]triazin-3(4H)-yl)ethyl)glycine;
   (R)-(-(6-(3-chloro-4-(2-chloro-3-(6-methoxy-5-((((5-oxopyrrolidin-2-yl)methyl)amino)methyl)pyridin-2-yl)phenyl)pyridin-2-yl)-2-methoxy-4-oxopyrrolo[2,1-f][1,2,4]triazin-3(4H)-yl)ethyl)glycine;
   N-(1-((6-(3-(2-(4-((4-acetamidopiperidin-1-yl)methyl)-3-methoxyphenyl)-3-methylpyridin-4-yl)-2-methylphenyl)-2-methoxypyridin-3-yl)methyl)piperidin-4-yl)acetamide;
   N-((6-(2-chloro-3-(2-(3-methoxy-4-(((tetrahydro-2H-pyran-4-yl)amino)methyl)phenyl)-3-methylpyridin-4-yl)phenyl)-2-methoxypyridin-3-yl)methyl)tetrahydro-2H-pyran-4-amine;
   (1r,4r)-4-(((6-(2-chloro-3-(2-(4-((((1r,4r)-4-hydroxycyclohexyl)amino)methyl)-3-methoxyphenyl)-3-methylpyridin-4-yl)phenyl)-2-methoxypyridin-3-yl)methyl)amino)cyclohexan-1-ol;
   (1r,4r)-4-(((6-(2-chloro-3-(2-(4-((((1r,4s)-4-hydroxycyclohexyl)amino)methyl)-3-methoxyphenyl)-3-methylpyridin-4-yl)phenyl)-2-methoxypyridin-3-yl)methyl)amino)cyclohexan-1-ol;
   (1r,4r)-4-(((6-(2-chloro-3-(2-(4-((((1s,4r)-4-hydroxycyclohexyl)amino)methyl)-3-methoxyphenyl)-3-methylpyridin-4-yl)phenyl)-2-methoxypyridin-3-yl)methyl)amino)cyclohexan-1-ol;
   (1r,4r)-4-(((6-(2-chloro-3-(2-(4-((((1s,4s)-4-hydroxycyclohexyl)amino)methyl)-3-methoxyphenyl)-3-methylpyridin-4-yl)phenyl)-2-methoxypyridin-3-yl)methyl)amino)cyclohexan-1-ol;
   (1r,4s)-4-(((6-(2-chloro-3-(2-(4-((((1r,4r)-4-hydroxycyclohexyl)amino)methyl)-3-methoxyphenyl)-3-methylpyridin-4-yl)phenyl)-2-methoxypyridin-3-yl)methyl)amino)cyclohexan-1-ol;
   (1r,4s)-4-(((6-(2-chloro-3-(2-(4-((((1r,4s)-4-hydroxycyclohexyl)amino)methyl)-3-methoxyphenyl)-3-methylpyridin-4-yl)phenyl)-2-methoxypyridin-3-yl)methyl)amino)cyclohexan-1-ol;
   (1r,4s)-4-(((6-(2-chloro-3-(2-(4-((((1s,4r)-4-hydroxycyclohexyl)amino)methyl)-3-methoxyphenyl)-3-methylpyridin-4-yl)phenyl)-2-methoxypyridin-3-yl)methyl)amino)cyclohexan-1-ol;
   (1r,4s)-4-(((6-(2-chloro-3-(2-(4-((((1s,4s)-4-hydroxycyclohexyl)amino)methyl)-3-methoxyphenyl)-3-methylpyridin-4-yl)phenyl)-2-methoxypyridin-3-yl)methyl)amino)cyclohexan-1-ol;
   (1s,4r)-4-(((6-(2-chloro-3-(2-(4-((((1r,4r)-4-hydroxycyclohexyl)amino)methyl)-3-methoxyphenyl)-3-methylpyridin-4-yl)phenyl)-2-methoxypyridin-3-yl)methyl)amino)cyclohexan-1-ol;
   (1s,4r)-4-(((6-(2-chloro-3-(2-(4-((((1r,4s)-4-hydroxycyclohexyl)amino)methyl)-3-methoxyphenyl)-3-methylpyridin-4-yl)phenyl)-2-methoxypyridin-3-yl)methyl)amino)cyclohexan-1-ol;
   (1s,4r)-4-(((6-(2-chloro-3-(2-(4-((((1s,4r)-4-hydroxycyclohexyl)amino)methyl)-3-methoxyphenyl)-3-methylpyridin-4-yl)phenyl)-2-methoxypyridin-3-yl)methyl)amino)cyclohexan-1-ol;
   (1s,4r)-4-(((6-(2-chloro-3-(2-(4-((((1s,4s)-4-hydroxycyclohexyl)amino)methyl)-3-methoxyphenyl)-3-methylpyridin-4-yl)phenyl)-2-methoxypyridin-3-yl)methyl)amino)cyclohexan-1-ol;
   (1s,4s)-4-(((6-(2-chloro-3-(2-(4-((((1r,4r)-4-hydroxycyclohexyl)amino)methyl)-3-methoxyphenyl)-3-methylpyridin-4-yl)phenyl)-2-methoxypyridin-3-yl)methyl)amino)cyclohexan-1-ol;
   (1s,4s)-4-(((6-(2-chloro-3-(2-(4-((((1r,4s)-4-hydroxycyclohexyl)amino)methyl)-3-methoxyphenyl)-3-methylpyridin-4-yl)phenyl)-2-methoxypyridin-3-yl)methyl)amino)cyclohexan-1-ol;
   (1s,4s)-4-(((6-(2-chloro-3-(2-(4-((((1s,4r)-4-hydroxycyclohexyl)amino)methyl)-3-methoxyphenyl)-3-methylpyridin-4-yl)phenyl)-2-methoxypyridin-3-yl)methyl)amino)cyclohexan-1-ol;
   (1s,4s)-4-(((6-(2-chloro-3-(2-(4-((((1s,4s)-4-hydroxycyclohexyl)amino)methyl)-3-methoxyphenyl)-3-methylpyridin-4-yl)phenyl)-2-methoxypyridin-3-yl)methyl)amino)cyclohexan-1-ol;
   6-(4-(4-(3-(5-((2-oxa-6-azaspiro[3.3]heptan-6-yl)methyl)-6-methoxypyridin-2-yl)-2-chlorophenyl)-3-methylpyridin-2-yl)-2-methoxybenzy1)-2-oxa-6-azaspiro[3.3]heptane;
   methyl 4-(((6-(2-chloro-3-(3-chloro-2-(3-methoxy-4-(((1-(methoxycarbonyl)piperidin-4-yl)amino)methyl)phenyl)pyridin-4-yl)phenyl)-2-methoxypyridin-3-yl)methyl)amino)piperidine-1-carboxylate;
   N-(1-((6-(3-(2-(4-((4-acetamidopiperidin-1-yl)methyl)-3-methoxyphenyl)-3-methylpyridin-4-yl)-2-chlorophenyl)-2-methoxypyridin-3-yl)methyl)piperidin-4-yl)acetamide;
   N-(1-((6-(3-(2-(4-((4-acetamidopiperidin-1-yl)methyl)-3-methoxyphenyl)-3-chloropyridin-4-yl)-2-methylphenyl)-2-methoxypyridin-3-yl)methyl)piperidin-4-yl)acetamide;
   N-((6-(2-chloro-3-(3-chloro-2-(3-methoxy-4-(((tetrahydro-2H-pyran-4-yl)amino)methyl)phenyl)pyridin-4-yl)phenyl)-2-methoxypyridin-3-yl)methyl)-N-methyltetrahydro-2H-pyran-4-amine;
   1-(4-(((6-(2-chloro-3-(3-chloro-2-(3-methoxy-4-(((tetrahydro-2H-pyran-4-yl)amino)methyl)phenyl)pyridin-4-yl)phenyl)-2-methoxypyridin-3-yl)methyl)(methyl)amino)piperidin-1-yl)ethan-1-one;
   2-(4-(4-(3-(5-((7-oxa-2-azaspiro[3.5]nonan-2-yl)methyl)-6-methoxypyridin-2-yl)-2-chlorophenyl)-3-chloropyridin-2-yl)-2-methoxybenzyl)-7-oxa-2-azaspiro[3.5]nonane;
   2-(1-((6-(2-chloro-3-(3-chloro-2-(4-((3-(2-hydroxypropan-2-yl)azetidin-1-yl)methyl)-3-methoxyphenyl)pyridin-4-yl)phenyl)-2-methoxypyridin-3-yl)methyl)azetidin-3-yl)propan-2-ol;
   (S)-5-((((6-(2-chloro-3-(3-chloro-2-(3-methoxy-4-((methyl(((S)-5-oxopyrrolidin-2-yl)methyl)amino)methyl)phenyl)pyridin-4-yl)phenyl)-2-methoxypyridin-3-yl)methyl)(methyl)amino)methyl)pyrrolidin-2-one;
   (S)-5-((((6-(2-chloro-3-(3-chloro-2-(3-methoxy-4-((methyl(((R)-5-oxopyrrolidin-2-yl)methyl)amino)methyl)phenyl)pyridin-4-yl)phenyl)-2-methoxypyridin-3-yl)methyl)(methyl)amino)methyl)pyrrolidin-2-one;
   (R)-5-((((6-(2-chloro-3-(3-chloro-2-(3-methoxy-4-((methyl(((S)-5-oxopyrrolidin-2-yl)methyl)amino)methyl)phenyl)pyridin-4-yl)phenyl)-2-methoxypyridin-3-yl)methyl)(methyl)amino)methyl)pyrrolidin-2-one;
   (R)-5-((((6-(2-chloro-3-(3-chloro-2-(3-methoxy-4-((methyl(((R)-5-oxopyrrolidin-2-yl)methyl)amino)methyl)phenyl)pyridin-4-yl)phenyl)-2-methoxypyridin-3-yl)methyl)(methyl)amino)methyl)pyrrolidin-2-one;
   (R)-4-((((6-(2-chloro-3-(3-chloro-2-(3-methoxy-4-((methyl(((R)-5-oxopyrrolidin-3-yl)methyl)amino)methyl)phenyl)pyridin-4-yl)phenyl)-2-methoxypyridin-3-yl)methyl)(methyl)amino)methyl)pyrrolidin-2-one;
   (S)-4-((((6-(2-chloro-3-(3-chloro-2-(3-methoxy-4-((methyl(((R)-5-oxopyrrolidin-3-yl)methyl)amino)methyl)phenyl)pyridin-4-yl)phenyl)-2-methoxypyridin-3-yl)methyl)(methyl)amino)methyl)pyrrolidin-2-one;
   (R)-4-((((6-(2-chloro-3-(3-chloro-2-(3-methoxy-4-((methyl(((S)-5-oxopyrrolidin-3-yl)methyl)amino)methyl)phenyl)pyridin-4-yl)phenyl)-2-methoxypyridin-3-yl)methyl)(methyl)amino)methyl)pyrrolidin-2-one;
   (S)-4-((((6-(2-chloro-3-(3-chloro-2-(3-methoxy-4-((methyl(((S)-5-oxopyrrolidin-3-yl)methyl)amino)methyl)phenyl)pyridin-4-yl)phenyl)-2-methoxypyridin-3-yl)methyl)(methyl)amino)methyl)pyrrolidin-2-one;
   2-((6-(3-chloro-4-(2-chloro-3-(5-((6-hydroxy-2-azaspiro[3.3]heptan-2-yl)methyl)-6-methoxypyridin-2-yl)phenyl)pyridin-2-yl)-8-methoxyimidazo[1,2-a]pyridin-2-yl)methyl)-2-azaspiro[3.3]heptan-6-ol;
   1-(4-(((6-(2-chloro-3-(3-chloro-2-(8-methoxy-2-(morpholinomethyl)imidazo[1,2-a]pyridin-6-yl)pyridin-4-yl)phenyl)-2-methoxypyridin-3-yl)methyl)amino)piperidin-1-yl)ethan-1-one;
   1-(4-(((6-(2-chloro-3-(3-chloro-2-(2-((3,3-difluoropyrrolidin-1-yl)methyl)-8-methoxyimidazo[1,2-a]pyridin-6-yl)pyridin-4-yl)phenyl)-2-methoxypyridin-3-yl)methyl)amino)piperidin-1-yl)ethan-1-one;
   1-((6-(4-(3-(5-(((1-acetylpiperidin-4-yl)amino)methyl)-6-methoxypyridin-2-yl)-2-chlorophenyl)-3-chloropyridin-2-yl)-8-methoxyimidazo[1,2-a]pyridin-2-yl)methyl)-3-methylazetidine-3-carbonitrile;
   1-(4-(((6-(2-chloro-3-(3-chloro-2-(2-(((2,2-difluoroethyl)amino)methyl)-8-methoxyimidazo[1,2-a]pyridin-6-yl)pyridin-4-yl)phenyl)-2-methoxypyridin-3-yl)methyl)amino)piperidin-1-yl)ethan-1-one;
   4-(2-((4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)amino)ethyl)piperazin-2-one;
   2-(3-(6-(4-(3-(5-(((1-acetylpiperidin-4-yl)amino)methyl)-6-methoxypyridin-2-yl)-2-chlorophenyl)-3-chloropyridin-2-yl)-8-methoxy-3,4-dihydroisoquinolin-2(1H)-yl)-2-oxopyrrolidin-1-yl)acetic acid;
   1-(4-(((6-(3-(2-(4-(((1-acetylpiperidin-4-yl)amino)methyl)-3-methoxyphenyl)-3-chloropyridin-4-yl)-2-chlorophenyl)-2-methoxypyridin-3-yl)methyl)amino)piperidin-1-yl)-2-methoxyethan-1-one;
   1-(4-(((6-(3-(2-(4-((6-acetyl-2,6-diazaspiro[3.3]heptan-2-yl)methyl)-3-methoxyphenyl)-3-chloropyridin-4-yl)-2-chlorophenyl)-2-methoxypyridin-3-yl)methyl)amino)piperidin-1-yl)ethan-1-one;
   1-(4-((4-(4-(3-(5-((6-acetyl-2,6-diazaspiro[3.3]heptan-2-yl)methyl)-6-methoxypyridin-2-yl)-2-chlorophenyl)-3-chloropyridin-2-yl)-2-methoxybenzyl)amino)piperidin-1-yl)ethan-1-one;
   N-((6-(2-chloro-3-(3-chloro-2-(3-methoxy-4-(((tetrahydro-2H-thiopyran-4-yl)amino)methyl)phenyl)pyridin-4-yl)phenyl)-2-methoxypyridin-3-yl)methyl)tetrahydro-2H-thiopyran-4-amine;
   1-(2-((6-(3-(2-(4-((7-acetyl-2,7-diazaspiro[3.5]nonan-2-yl)methyl)-3-methoxyphenyl)-3-chloropyridin-4-yl)-2-chlorophenyl)-2-methoxypyridin-3-yl)methyl)-2,7-diazaspiro[3.5]nonan-7-yl)ethan-1-one;
   (S)-5-((((6-(((S)-4-(5-(((1-acetylpiperidin-4-yl)amino)methyl)-3'-chloro-6-methoxy-[2,4'-bipyridin]-2'-yl)-2,3-dihydro-1H-inden-1-yl)amino)-2-methoxy-5-(trifluoromethyl)pyridin-3-yl)methyl)amino)methyl)pyrrolidin-2-one;
   (S)-5-((((6-(((R)-4-(5-(((1-acetylpiperidin-4-yl)amino)methyl)-3'-chloro-6-methoxy-[2,4'-bipyridin]-2'-yl)-2,3-dihydro-1H-inden-1-yl)amino)-2-methoxy-5-(trifluoromethyl)pyridin-3-yl)methyl)amino)methyl)pyrrolidin-2-one;
   (R)-5-((((6-(((S)-4-(5-(((1-acetylpiperidin-4-yl)amino)methyl)-3'-chloro-6-methoxy-[2,4'-bipyridin]-2'-yl)-2,3-dihydro-1H-inden-1-yl)amino)-2-methoxy-5-(trifluoromethyl)pyridin-3-yl)methyl)amino)methyl)pyrrolidin-2-one;
   (R)-5-((((6-(((R)-4-(5-(((1-acetylpiperidin-4-yl)amino)methyl)-3'-chloro-6-methoxy-[2,4'-bipyridin]-2'-yl)-2,3-dihydro-1H-inden-1-yl)amino)-2-methoxy-5-(trifluoromethyl)pyridin-3-yl)methyl)amino)methyl)pyrrolidin-2-one;
   1-(4-((4-(3-chloro-4-(2-chloro-3-(5-((4-hydroxypiperidin-1-yl)methyl)-6-methoxypyridin-2-yl)phenyl)pyridin-2-yl)-2-methoxybenzyl)amino)piperidin-1-yl)ethan-1-one;
   1-(4-((4-(3-chloro-4-(2-chloro-3-(6-methoxy-5-((((1r,4r)-4-methoxycyclohexyl)amino)methyl)pyridin-2-yl)phenyl)pyridin-2-yl)-2-methoxybenzyl)amino)piperidin-1-yl)ethan-1-one;
   1-(4-((4-(3-chloro-4-(2-chloro-3-(6-methoxy-5-((((1s,4r)-4-methoxycyclohexyl)amino)methyl)pyridin-2-yl)phenyl)pyridin-2-yl)-2-methoxybenzyl)amino)piperidin-1-yl)ethan-1-one;
   1-(4-((4-(3-chloro-4-(2-chloro-3-(6-methoxy-5-((((1r,4s)-4-methoxycyclohexyl)amino)methyl)pyridin-2-yl)phenyl)pyridin-2-yl)-2-methoxybenzyl)amino)piperidin-1-yl)ethan-1-one;
   1-(4-((4-(3-chloro-4-(2-chloro-3-(6-methoxy-5-((((1s,4s)-4-methoxycyclohexyl)amino)methyl)pyridin-2-yl)phenyl)pyridin-2-yl)-2-methoxybenzyl)amino)piperidin-1-yl)ethan-1-one;
   (R)-1-(4-(((6-(2-chloro-3-(3-chloro-2-(4-((3-hydroxypiperidin-1-yl)methyl)-3-methoxyphenyl)pyridin-4-yl)phenyl)-2-methoxypyridin-3-yl)methyl)amino)piperidin-1-yl)ethan-1-one;
   (S)-1-(4-(((6-(2-chloro-3-(3-chloro-2-(4-((3-hydroxypiperidin-1-yl)methyl)-3-methoxyphenyl)pyridin-4-yl)phenyl)-2-methoxypyridin-3-yl)methyl)amino)piperidin-1-yl)ethan-1-one;
   2-(4-(3-chloro-4-(2-chloro-3-(6-methoxy-5-(((1-(2-methoxyacetyl)piperidin-4-yl)amino)methyl)pyridin-2-yl)phenyl)pyridin-2-yl)-2-methoxybenzyl)-2,6-diazaspiro[3.4]octan-7-one;
   1-(4-(((6-(3-(2-(4-((6-acetyl-2,6-diazaspiro[3.3]heptan-2-yl)methyl)-3-methoxyphenyl)-3-chloropyridin-4-yl)-2-chlorophenyl)-2-methoxypyridin-3-yl)methyl)amino)piperidin-1-yl)-2-methoxyethan-1-one;
   1-(6-(4-(3-chloro-4-(2-chloro-3-(6-methoxy-5-(((tetrahydro-2H-pyran-4-yl)amino)methyl)pyridin-2-yl)phenyl)pyridin-2-yl)-2-methoxybenzyl)-2,6-diazaspiro[3.3]heptan-2-yl)ethan-1-one;
   2-((6-(3-(2-(4-((6-acetyl-2,6-diazaspiro[3.3]heptan-2-yl)methyl)-3-methoxyphenyl)-3-chloropyridin-4-yl)-2-chlorophenyl)-2-methoxypyridin-3-yl)methyl)-2,6-diazaspiro[3.4]octan-7-one;
   2-((6-(3-(2-(4-((2,6-diazaspiro[3 .3]heptan-2-yl)methyl)-3-methoxyphenyl)-3-chloropyridin-4-yl)-2-chlorophenyl)-2-methoxypyridin-3-yl)methyl)-2,6-diazaspiro[3.4]octan-7-one;
   2-((6-(2-chloro-3-(3-chloro-2-(3-methoxy-4-(((tetrahydro-2H-pyran-4-yl)amino)methyl)phenyl)pyridin-4-yl)phenyl)-2-methoxypyridin-3-yl)methyl)-2,6-diazaspiro[3.4]octan-7-one;
   2-((6-(2-chloro-3-(3-chloro-2-(4-((((1r,3r)-3-hydroxy-3-methylcyclobutyl)amino)methyl)-3-methoxyphenyl)pyridin-4-yl)phenyl)-2-methoxypyridin-3-yl)methyl)-2,6-diazaspiro[3.4]octan-7-one;
   2-((6-(2-chloro-3-(3-chloro-2-(4-((((1s,3r)-3-hydroxy-3-methylcyclobutyl)amino)methyl)-3-methoxyphenyl)pyridin-4-yl)phenyl)-2-methoxypyridin-3-yl)methyl)-2,6-diazaspiro[3.4]octan-7-one;
   2-((6-(2-chloro-3-(3-chloro-2-(4-((((1r,3s)-3-hydroxy-3-methylcyclobutyl)amino)methyl)-3-methoxyphenyl)pyridin-4-yl)phenyl)-2-methoxypyridin-3-yl)methyl)-2,6-diazaspiro[3.4]octan-7-one;
   2-((6-(2-chloro-3-(3-chloro-2-(4-((((1s,3s)-3-hydroxy-3-methylcyclobutyl)amino)methyl)-3-methoxyphenyl)pyridin-4-yl)phenyl)-2-methoxypyridin-3-yl)methyl)-2,6-diazaspiro[3.4]octan-7-one;
   2-((6-(2-chloro-3-(3-chloro-2-(4-((6-hydroxy-2-azaspiro[3.3]heptan-2-yl)methyl)-3-methoxyphenyl)pyridin-4-yl)phenyl)-2-methoxypyridin-3-yl)methyl)-2,6-diazaspiro[3.4]octan-7-one;
   2-((6-(2-chloro-3-(3-chloro-2-(4-((3-hydroxy-3-methylazetidin-1-yl)methyl)-3-methoxyphenyl)pyridin-4-yl)phenyl)-2-methoxypyridin-3-yl)methyl)-2,6-diazaspiro[3.4]octan-7-one;
   1-(3-((((6-(3-(2-(4-((((1-acetylazetidin-3-yl)methyl)(methyl)amino)methyl)-3-methoxyphenyl)-3-chloropyridin-4-yl)-2-chlorophenyl)-2-methoxypyridin-3-yl)methyl)(methyl)amino)methyl)azetidin-1-yl)ethan-1-one;
   1-(4-(((4-(4'-(((1-acetylpiperidin-4-yl)amino)methyl)-2-chloro-3'-methoxy-[1,1'-biphenyl]-3-yl)-3-chloro-5'-methoxy-[2,3'-bipyridin]-6'-yl)methyl)amino)piperidin-1-yl)ethan-1-one;
   2-((2'-chloro-3'-(3-chloro-5'-methoxy-6'-((7-oxo-2,6-diazaspiro[3.4]octan-2-yl)methyl)-[2,3'-bipyridin]-4-yl)-3-methoxy-[1,1'-biphenyl]-4-yl)methyl)-2,6-diazaspiro[3.4]octan-7-one;
   2-((6-(2-chloro-3-(3-chloro-2-(4-((((1r,4r)-4-hydroxycyclohexyl)amino)methyl)-3-methoxyphenyl)pyridin-4-yl)phenyl)-2-methoxypyridin-3-yl)methyl)-2,6-diazaspiro[3.4]octan-7-one;
   2-((6-(2-chloro-3-(3-chloro-2-(4-((((1s,4r)-4-hydroxycyclohexyl)amino)methyl)-3-methoxyphenyl)pyridin-4-yl)phenyl)-2-methoxypyridin-3-yl)methyl)-2,6-diazaspiro[3.4]octan-7-one;
   2-((6-(2-chloro-3-(3-chloro-2-(4-((((1r,4s)-4-hydroxycyclohexyl)amino)methyl)-3-methoxyphenyl)pyridin-4-yl)phenyl)-2-methoxypyridin-3-yl)methyl)-2,6-diazaspiro[3.4]octan-7-one;
   2-((6-(2-chloro-3-(3-chloro-2-(4-((((1s,4s)-4-hydroxycyclohexyl)amino)methyl)-3-methoxyphenyl)pyridin-4-yl)phenyl)-2-methoxypyridin-3-yl)methyl)-2,6-diazaspiro[3.4]octan-7-one;
   1-((((6-(2-chloro-3-(3-chloro-2-(4-((((1-hydroxycyclopropyl)methyl)amino)methyl)-3-methoxyphenyl)pyridin-4-yl)phenyl)-2-methoxypyridin-3-yl)methyl)amino)methyl)cyclopropan-1-ol;
   N-((6-(2-chloro-3-(3-chloro-2-(4-(((2,2-difluoroethyl)amino)methyl)-3-methoxyphenyl)pyridin-4-yl)phenyl)-2-methoxypyridin-3-yl)methyl)-2,2-difluoroethan-1-amine;
   2-(4-(3-chloro-4-(2-chloro-3-(6-methoxy-5-(((tetrahydro-2H-pyran-4-yl)amino)methyl)pyridin-2-yl)phenyl)pyridin-2-yl)-2-methoxybenzyl)-2,6-diazaspiro[3.4]octan-7-one;
   (S)-1-(4-(((6-(2-chloro-3-(3-chloro-2-(4-((3-hydroxypiperidin-1-yl)methyl)-3-methoxyphenyl)pyridin-4-yl)phenyl)-2-methoxypyridin-3-yl)methyl)amino)piperidin-1-yl)ethan-1-one;
   (R)-1-(4-(((6-(2-chloro-3-(3-chloro-2-(4-((3-hydroxypiperidin-1-yl)methyl)-3-methoxyphenyl)pyridin-4-yl)phenyl)-2-methoxypyridin-3-yl)methyl)amino)piperidin-1-yl)ethan-1-one;
   1-(4-(((6-(2-chloro-3-(3-chloro-2-(4-((4-(hydroxymethyl)piperidin-1-yl)methyl)-3-methoxyphenyl)pyridin-4-yl)phenyl)-2-methoxypyridin-3-yl)methyl)amino)piperidin-1-yl)ethan-1-one;
   1-(4-(((6-(2-chloro-3-(3-chloro-2-(3-methoxy-4-((4-(methoxymethyl)piperidin-1-yl)methyl)phenyl)pyridin-4-yl)phenyl)-2-methoxypyridin-3-yl)methyl)amino)piperidin-1-yl)ethan-1-one;
   1-(4-(((6-(2-chloro-3-(3-chloro-2-(4-((4-hydroxypiperidin-1-yl)methyl)-3-methoxyphenyl)pyridin-4-yl)phenyl)-2-methoxypyridin-3-yl)methyl)amino)piperidin-1-yl)-2-methoxyethan-1-one;
   1-(6-((6-(2-chloro-3-(3-chloro-2-(3-methoxy-4-(((tetrahydro-2H-pyran-4-yl)amino)methyl)phenyl)pyridin-4-yl)phenyl)-2-methoxypyridin-3-yl)methyl)-2,6-diazaspiro[3.3]heptan-2-yl)ethan-1-one;
   1-(4-(((6-(2-chloro-3-(3-chloro-2-(4-((((1-hydroxycyclopropyl)methyl)amino)methyl)-3-methoxyphenyl)pyridin-4-yl)phenyl)-2-methoxypyridin-3-yl)methyl)amino)piperidin-1-yl)ethan-1-one;
   1-(4-(((6-(2-chloro-3-(3-chloro-2-(4-(((2,2-difluoroethyl)amino)methyl)-3-methoxyphenyl)pyridin-4-yl)phenyl)-2-methoxypyridin-3-yl)methyl)amino)piperidin-1-yl)ethan-1-one;
   N-((6-(2-chloro-3-(3-chloro-2-(4-((isopropylamino)methyl)-3-methoxyphenyl)pyridin-4-yl)phenyl)-2-methoxypyridin-3-yl)methyl)propan-2-amine;
   2-(4-(3-chloro-4-(2-chloro-3-(5-((((1r,4r)-4-hydroxycyclohexyl)amino)methyl)-6-methoxypyridin-2-yl)phenyl)pyridin-2-yl)-2-methoxybenzyl)-2,6-diazaspiro[3.4]octan-7-one;
   2-(4-(3-chloro-4-(2-chloro-3-(5-((((1s,4r)-4-hydroxycyclohexyl)amino)methyl)-6-methoxypyridin-2-yl)phenyl)pyridin-2-yl)-2-methoxybenzyl)-2,6-diazaspiro[3.4]octan-7-one;
   2-(4-(3-chloro-4-(2-chloro-3-(5-((((1r,4s)-4-hydroxycyclohexyl)amino)methyl)-6-methoxypyridin-2-yl)phenyl)pyridin-2-yl)-2-methoxybenzyl)-2,6-diazaspiro[3.4]octan-7-one;
   2-(4-(3-chloro-4-(2-chloro-3-(5-((((1s,4s)-4-hydroxycyclohexyl)amino)methyl)-6-methoxypyridin-2-yl)phenyl)pyridin-2-yl)-2-methoxybenzyl)-2,6-diazaspiro[3.4]octan-7-one;
   2-(4-(3-chloro-4-(2-chloro-3-(5-((((1s,4s)-4-hydroxycyclohexyl)amino)methyl)-6-methoxypyridin-2-yl)phenyl)pyridin-2-yl)-2-methoxybenzyl)-2,6-diazaspiro[3.4]octan-7-one;
   2-(4-(3-chloro-4-(2-chloro-3-(5-((((1s,4r)-4-hydroxycyclohexyl)amino)methyl)-6-methoxypyridin-2-yl)phenyl)pyridin-2-yl)-2-methoxybenzyl)-2,6-diazaspiro[3.4]octan-7-one;
   2-(4-(3-chloro-4-(2-chloro-3-(5-((((1r,4s)-4-hydroxycyclohexyl)amino)methyl)-6-methoxypyridin-2-yl)phenyl)pyridin-2-yl)-2-methoxybenzyl)-2,6-diazaspiro[3.4]octan-7-one;
   2-(4-(3-chloro-4-(2-chloro-3-(5-((((1r,4r)-4-hydroxycyclohexyl)amino)methyl)-6-methoxypyridin-2-yl)phenyl)pyridin-2-yl)-2-methoxybenzyl)-2,6-diazaspiro[3.4]octan-7-one;
   2-(4-(3-chloro-4-(2-chloro-3-(5-((((1r,3r)-3-hydroxy-3-methylcyclobutyl)amino)methyl)-6-methoxypyridin-2-yl)phenyl)pyridin-2-yl)-2-methoxybenzyl)-2,6-diazaspiro[3.4]octan-7-one;
   2-(4-(3-chloro-4-(2-chloro-3-(5-((((1s,3r)-3-hydroxy-3-methylcyclobutyl)amino)methyl)-6-methoxypyridin-2-yl)phenyl)pyridin-2-yl)-2-methoxybenzyl)-2,6-diazaspiro[3.4]octan-7-one;
   2-(4-(3-chloro-4-(2-chloro-3-(5-((((1r,3s)-3-hydroxy-3-methylcyclobutyl)amino)methyl)-6-methoxypyridin-2-yl)phenyl)pyridin-2-yl)-2-methoxybenzyl)-2,6-diazaspiro[3.4]octan-7-one;
   2-(4-(3-chloro-4-(2-chloro-3-(5-((((1s,3s)-3-hydroxy-3-methylcyclobutyl)amino)methyl)-6-methoxypyridin-2-yl)phenyl)pyridin-2-yl)-2-methoxybenzyl)-2,6-diazaspiro[3.4]octan-7-one;
   2-(4-(3-chloro-4-(2-chloro-3-(5-((3-hydroxy-3-methylazetidin-1-yl)methyl)-6-methoxypyridin-2-yl)phenyl)pyridin-2-yl)-2-methoxybenzyl)-2,6-diazaspiro[3.4]octan-7-one;
   2-(4-(4-(3-(5-((6-acetyl-2,6-diazaspiro[3.3]heptan-2-yl)methyl)-6-methoxypyridin-2-yl)-2-chlorophenyl)-3-chloropyridin-2-yl)-2-methoxybenzyl)-2,6-diazaspiro[3.4]octan-7-one;
   2-(4-(4-(3-(5-((2,6-diazaspiro[3.3]heptan-2-yl)methyl)-6-methoxypyridin-2-yl)-2-chlorophenyl)-3-chloropyridin-2-yl)-2-methoxybenzyl)-2,6-diazaspiro[3.4]octan-7-one;
   2-(4-(3-chloro-4-(2-chloro-3-(5-((6-hydroxy-2-azaspiro[3.3]heptan-2-yl)methyl)-6-methoxypyridin-2-yl)phenyl)pyridin-2-yl)-2-methoxybenzyl)-2,6-diazaspiro[3.4]octan-7-one;
   1-(4-(((6-(3-(6-(4-(((1-acetylpiperidin-4-yl)amino)methyl)-3-methoxyphenyl)-5-methylpyrimidin-4-yl)-2-chlorophenyl)-2-methoxypyridin-3-yl)methyl)amino)piperidin-1-yl)ethan-1-one;
   (S)-5-((((6-(2-chloro-3-(6-(3-methoxy-4-(((((S)-5-oxopyrrolidin-2-yl)methyl)amino)methyl)phenyl)-5-methylpyrimidin-4-yl)phenyl)-2-methoxypyridin-3-yl)methyl)amino)methyl)pyrrolidin-2-one;
   (S)-5-((((6-(2-chloro-3-(6-(3-methoxy-4-(((((R)-5-oxopyrrolidin-2-yl)methyl)amino)methyl)phenyl)-5-methylpyrimidin-4-yl)phenyl)-2-methoxypyridin-3-yl)methyl)amino)methyl)pyrrolidin-2-one;
   (R)-5-((((6-(2-chloro-3-(6-(3-methoxy-4-(((((S)-5-oxopyrrolidin-2-yl)methyl)amino)methyl)phenyl)-5-methylpyrimidin-4-yl)phenyl)-2-methoxypyridin-3-yl)methyl)amino)methyl)pyrrolidin-2-one;
   (R)-5-((((6-(2-chloro-3-(6-(3-methoxy-4-(((((R)-5-oxopyrrolidin-2-yl)methyl)amino)methyl)phenyl)-5-methylpyrimidin-4-yl)phenyl)-2-methoxypyridin-3-yl)methyl)amino)methyl)pyrrolidin-2-one;
   N-((6-(2-chloro-3-(3-chloro-2-(4-((cyclohexylamino)methyl)-3-methoxyphenyl)pyridin-4-yl)phenyl)-2-methoxypyridin-3-yl)methyl)cyclohexanamine;
   2-((6-(3-(2-(4-(((1-acetylpiperidin-4-yl)amino)methyl)-3-methoxyphenyl)-3-chloropyridin-4-yl)-2-chlorophenyl)-2-methoxypyridin-3-yl)methyl)-2,6-diazaspiro[3.4]octan-5-one;
   2-((6-(3-(2-(4-(((1-acetylpiperidin-4-yl)amino)methyl)-3-methoxyphenyl)-3-chloropyridin-4-yl)-2-chlorophenyl)-2-methoxypyridin-3-yl)methyl)-2,5,7-triazaspiro[3.4]octan-6-one;
   (S)-5-((((2'-((S)-1-((5-(((1-acetylpiperidin-4-yl)amino)methyl)-6-methoxy-3-(trifluoromethyl)pyridin-2-yl)oxy)-2,3-dihydro-1H-inden-4-yl)-3'-chloro-6-methoxy-[2,4'-bipyridin]-5-yl)methyl)amino)methyl)pyrrolidin-2-one;
   (S)-5-((((2'-((R)-1-((5-(((1-acetylpiperidin-4-yl)amino)methyl)-6-methoxy-3-(trifluoromethyl)pyridin-2-yl)oxy)-2,3-dihydro-1H-inden-4-yl)-3'-chloro-6-methoxy-[2,4'-bipyridin]-5-yl)methyl)amino)methyl)pyrrolidin-2-one;
   (R)-5-((((2'-((S)-1-((5-(((1-acetylpiperidin-4-yl)amino)methyl)-6-methoxy-3-(trifluoromethyl)pyridin-2-yl)oxy)-2,3-dihydro-1H-inden-4-yl)-3'-chloro-6-methoxy-[2,4'-bipyridin]-5-yl)methyl)amino)methyl)pyrrolidin-2-one;
   (R)-5-((((2'-((R)-1-((5-(((1-acetylpiperidin-4-yl)amino)methyl)-6-methoxy-3-(trifluoromethyl)pyridin-2-yl)oxy)-2,3-dihydro-1H-inden-4-yl)-3'-chloro-6-methoxy-[2,4'-bipyridin]-5-yl)methyl)amino)methyl)pyrrolidin-2-one;
   1-(4-(((6-(3-(2-(4-(((1-acetylpiperidin-4-yl)amino)methyl)-3-methoxyphenyl)-3-chloropyridin-4-yl)-2-(trifluoromethyl)phenyl)-2-methoxypyridin-3-yl)methyl)amino)piperidin-1-yl)ethan-1-one;
   N-((6-(3-(3-chloro-2-(3-methoxy-4-(((tetrahydro-2H-pyran-4-yl)amino)methyl)phenyl)pyridin-4-yl)-2-(trifluoromethyl)phenyl)-2-methoxypyridin-3-yl)methyl)tetrahydro-2H-pyran-4-amine;
   N-((6-(3-(2-(4-((7-oxa-2-azaspiro[3.5]nonan-2-yl)methyl)-3-methoxyphenyl)-3-chloropyridin-4-yl)-2-chlorophenyl)-2-methoxypyridin-3-yl)methyl)tetrahydro-2H-pyran-4-amine;
   N-(4-(4-(3-(5-((7-oxa-2-azaspiro[3.5]nonan-2-yl)methyl)-6-methoxypyridin-2-yl)-2-chlorophenyl)-3-chloropyridin-2-yl)-2-methoxybenzyl)tetrahydro-2H-pyran-4-amine;
   (S)-5-((((6-(3-chloro-2-((S)-1-((6-methoxy-5-(((((S)-5-oxopyrrolidin-2-yl)methyl)amino)methyl)-3-(trifluoromethyl)pyrazin-2-yl)amino)-2,3-dihydro-1H-inden-4-yl)pyridin-4-yl)-1-methyl-1H-pyrrolo[2,3-b]pyridin-3-yl)methyl)amino)methyl)pyrrolidin-2-one;
   (R)-5-((((6-(3-chloro-2-((R)-1-((6-methoxy-5-(((((R)-5-oxopyrrolidin-2-yl)methyl)amino)methyl)-3-(trifluoromethyl)pyrazin-2-yl)amino)-2,3-dihydro-1H-inden-4-yl)pyridin-4-yl)-1-methyl-1H-pyrrolo[2,3-b]pyridin-3-yl)methyl)amino)methyl)pyrrolidin-2-one;
   (R)-5-((((6-(3-chloro-2-((R)-1-((6-methoxy-5-(((((S)-5-oxopyrrolidin-2-yl)methyl)amino)methyl)-3-(trifluoromethyl)pyrazin-2-yl)amino)-2,3-dihydro-1H-inden-4-yl)pyridin-4-yl)-1-methyl-1H-pyrrolo[2,3-b]pyridin-3-yl)methyl)amino)methyl)pyrrolidin-2-one;
   (R)-5-((((6-(3-chloro-2-((S)-1-((6-methoxy-5-(((((R)-5-oxopyrrolidin-2-yl)methyl)amino)methyl)-3-(trifluoromethyl)pyrazin-2-yl)amino)-2,3-dihydro-1H-inden-4-yl)pyridin-4-yl)-1-methyl-1H-pyrrolo[2,3-b]pyridin-3-yl)methyl)amino)methyl)pyrrolidin-2-one;
   (R)-5-((((6-(3-chloro-2-((S)-1-((6-methoxy-5-(((((S)-5-oxopyrrolidin-2-yl)methyl)amino)methyl)-3-(trifluoromethyl)pyrazin-2-yl)amino)-2,3-dihydro-1H-inden-4-yl)pyridin-4-yl)-1-methyl-1H-pyrrolo[2,3-b]pyridin-3-yl)methyl)amino)methyl)pyrrolidin-2-one;
   (S)-5-((((6-(3-chloro-2-((R)-1-((6-methoxy-5-(((((R)-5-oxopyrrolidin-2-yl)methyl)amino)methyl)-3-(trifluoromethyl)pyrazin-2-yl)amino)-2,3-dihydro-1H-inden-4-yl)pyridin-4-yl)-1-methyl-1H-pyrrolo[2,3-b]pyridin-3-yl)methyl)amino)methyl)pyrrolidin-2-one;
   (S)-5-((((6-(3-chloro-2-((R)-1-((6-methoxy-5-(((((S)-5-oxopyrrolidin-2-yl)methyl)amino)methyl)-3-(trifluoromethyl)pyrazin-2-yl)amino)-2,3-dihydro-1H-inden-4-yl)pyridin-4-yl)-1-methyl-1H-pyrrolo[2,3-b]pyridin-3-yl)methyl)amino)methyl)pyrrolidin-2-one;
   (S)-5-((((6-(3-chloro-2-((S)-1-((6-methoxy-5-(((((R)-5-oxopyrrolidin-2-yl)methyl)amino)methyl)-3-(trifluoromethyl)pyrazin-2-yl)amino)-2,3-dihydro-1H-inden-4-yl)pyridin-4-yl)-1-methyl-1H-pyrrolo[2,3-b]pyridin-3-yl)methyl)amino)methyl)pyrrolidin-2-one;
   1-(4-(((6-(3-(2-(4-(((1-acetylpiperidin-4-yl)amino)methyl)-3-methoxyphenyl)-3-chloropyridin-4-yl)-2-chlorophenyl)-2-methoxypyridin-3-yl)methyl)amino)piperidin-1-yl)-2-hydroxyethan-1-one;
   N-((6-(2-chloro-3-(3-chloro-2-(4-(((3-fluoropropyl)amino)methyl)-3-methoxyphenyl)pyridin-4-yl)phenyl)-2-methoxypyridin-3-yl)methyl)-3-fluoropropan-1-amine;
   2-(4-(6-(2-chloro-3-(6-methoxy-5-((7-oxo-2,6-diazaspiro[3.4]octan-2-yl)methyl)pyridin-2-yl)phenyl)-5-methylpyrimidin-4-yl)-2-methoxybenzyl)-2,6-diazaspiro[3.4]octan-7-one;
   1-(1-((6-(3-(2-(4-((4-acetylpiperidin-1-yl)methyl)-3-methoxyphenyl)-3-chloropyridin-4-yl)-2-chlorophenyl)-2-methoxypyridin-3-yl)methyl)piperidin-4-yl)ethan-1-one;
   1-(4-(((6-(2-chloro-3-(3-chloro-2-(4-(((3-fluoropropyl)amino)methyl)-3-methoxyphenyl)pyridin-4-yl)phenyl)-2-methoxypyridin-3-yl)methyl)amino)piperidin-1-yl)ethan-1-one;
   1-(6-((6-(3-(6-(4-((6-acetyl-2,6-diazaspiro[3.3]heptan-2-yl)methyl)-3-methoxyphenyl)-5-methylpyrimidin-4-yl)-2-chlorophenyl)-2-methoxypyridin-3-yl)methyl)-2,6-diazaspiro[3.3]heptan-2-yl)ethan-1-one;
   1-(4-(((6-(2-chloro-3-(3-chloro-2-(4-((((1r,4r)-4-hydroxycyclohexyl)amino)methyl)-3-methoxyphenyl)pyridin-4-yl)phenyl)-2-methoxypyridin-3-yl)methyl)amino)piperidin-1-yl)-2-hydroxyethan-1-one;
   1-(4-(((6-(2-chloro-3-(3-chloro-2-(4-((((1s,4r)-4-hydroxycyclohexyl)amino)methyl)-3-methoxyphenyl)pyridin-4-yl)phenyl)-2-methoxypyridin-3-yl)methyl)amino)piperidin-1-yl)-2-hydroxyethan-1-one;
   1-(4-(((6-(2-chloro-3-(3-chloro-2-(4-((((1r,4s)-4-hydroxycyclohexyl)amino)methyl)-3-methoxyphenyl)pyridin-4-yl)phenyl)-2-methoxypyridin-3-yl)methyl)amino)piperidin-1-yl)-2-hydroxyethan-1-one;
   1-(4-(((6-(2-chloro-3-(3-chloro-2-(4-((((1s,4s)-4-hydroxycyclohexyl)amino)methyl)-3-methoxyphenyl)pyridin-4-yl)phenyl)-2-methoxypyridin-3-yl)methyl)amino)piperidin-1-yl)-2-hydroxyethan-1-one;
   1-(4-(((6-(3-(6-(4-(((1-acetylpiperidin-4-yl)amino)methyl)-3-methoxyphenyl)-5-chloropyrimidin-4-yl)-2-chlorophenyl)-2-methoxypyridin-3-yl)methyl)amino)piperidin-1-yl)ethan-1-one;
   (R)-5-(((6-(3-(2-(4-(((1-acetylpiperidin-4-yl)amino)methyl)-3-methoxyphenyl)-3-chloropyridin-4-yl)-2-chlorophenyl)-2-methoxypyridin-3-yl)methyl)amino)piperidin-2-one;
   (S)-5-(((6-(3-(2-(4-(((1-acetylpiperidin-4-yl)amino)methyl)-3-methoxyphenyl)-3-chloropyridin-4-yl)-2-chlorophenyl)-2-methoxypyridin-3-yl)methyl)amino)piperidin-2-one;
   (S)-5-((((6-(2-chloro-3-(5-chloro-6-(3-methoxy-4-(((((S)-5-oxopyrrolidin-2-yl)methyl)amino)methyl)phenyl)pyrimidin-4-yl)phenyl)-2-methoxypyridin-3-yl)methyl)amino)methyl)pyrrolidin-2-one;
   (S)-5-((((6-(2-chloro-3-(5-chloro-6-(3-methoxy-4-(((((R)-5-oxopyrrolidin-2-yl)methyl)amino)methyl)phenyl)pyrimidin-4-yl)phenyl)-2-methoxypyridin-3-yl)methyl)amino)methyl)pyrrolidin-2-one;
   (R)-5-((((6-(2-chloro-3-(5-chloro-6-(3-methoxy-4-(((((S)-5-oxopyrrolidin-2-yl)methyl)amino)methyl)phenyl)pyrimidin-4-yl)phenyl)-2-methoxypyridin-3-yl)methyl)amino)methyl)pyrrolidin-2-one;
   (R)-5-((((6-(2-chloro-3-(5-chloro-6-(3-methoxy-4-(((((R)-5-oxopyrrolidin-2-yl)methyl)amino)methyl)phenyl)pyrimidin-4-yl)phenyl)-2-methoxypyridin-3-yl)methyl)amino)methyl)pyrrolidin-2-one;
   (S)-5-((((6-(((S)-4-(5-(((1-acetylpiperidin-4-yl)amino)methyl)-3'-chloro-6-methoxy-[2,4'-bipyridin]-2'-yl)-2,3-dihydro-1H-inden-1-yl)oxy)-2-methoxy-5-(trifluoromethyl)pyridin-3-yl)methyl)amino)methyl)pyrrolidin-2-one;
   (S)-5-((((6-(((R)-4-(5-(((1-acetylpiperidin-4-yl)amino)methyl)-3'-chloro-6-methoxy-[2,4'-bipyridin]-2'-yl)-2,3-dihydro-1H-inden-1-yl)oxy)-2-methoxy-5-(trifluoromethyl)pyridin-3-yl)methyl)amino)methyl)pyrrolidin-2-one;
   (R)-5-((((6-(((S)-4-(5-(((1-acetylpiperidin-4-yl)amino)methyl)-3'-chloro-6-methoxy-[2,4'-bipyridin]-2'-yl)-2,3-dihydro-1H-inden- 1-yl)oxy)-2-methoxy-5-(trifluoromethyl)pyridin-3-yl)methyl)amino)methyl)pyrrolidin-2-one;
   (R)-5-((((6-(((R)-4-(5-(((1-acetylpiperidin-4-yl)amino)methyl)-3'-chloro-6-methoxy-[2,4'-bipyridin]-2'-yl)-2,3-dihydro-1H-inden- 1-yl)oxy)-2-methoxy-5-(trifluoromethyl)pyridin-3-yl)methyl)amino)methyl)pyrrolidin-2-one;
   (S)-5-((((6-(3-chloro-2-((S)-1-((6-methoxy-5-(((((S)-5-oxopyrrolidin-2-yl)methyl)amino)methyl)-3-(trifluoromethyl)pyridin-2-yl)oxy)-2,3-dihydro-1H-inden-4-yl)pyridin-4-yl)-1-methyl-1H-pyrrolo[2,3-b]pyridin-3-yl)methyl)amino)methyl)pyrrolidin-2-one;
   (R)-5-((((6-(3-chloro-2-((R)-1-((6-methoxy-5-(((((R)-5-oxopyrrolidin-2-yl)methyl)amino)methyl)-3-(trifluoromethyl)pyridin-2-yl)oxy)-2,3-dihydro-1H-inden-4-yl)pyridin-4-yl)-1-methyl-1H-pyrrolo[2,3-b]pyridin-3-yl)methyl)amino)methyl)pyrrolidin-2-one;
   (R)-5-((((6-(3-chloro-2-((R)-1-((6-methoxy-5-(((((S)-5-oxopyrrolidin-2-yl)methyl)amino)methyl)-3-(trifluoromethyl)pyridin-2-yl)oxy)-2,3-dihydro-1H-inden-4-yl)pyridin-4-yl)-1-methyl-1H-pyrrolo[2,3-b]pyridin-3-yl)methyl)amino)methyl)pyrrolidin-2-one;
   (R)-5-((((6-(3-chloro-2-((S)-1-((6-methoxy-5-(((((R)-5-oxopyrrolidin-2-yl)methyl)amino)methyl)-3-(trifluoromethyl)pyridin-2-yl)oxy)-2,3-dihydro-1H-inden-4-yl)pyridin-4-yl)-1-methyl-1H-pyrrolo[2,3-b]pyridin-3-yl)methyl)amino)methyl)pyrrolidin-2-one;
   (R)-5-((((6-(3-chloro-2-((S)-1-((6-methoxy-5-(((((S)-5-oxopyrrolidin-2-yl)methyl)amino)methyl)-3-(trifluoromethyl)pyridin-2-yl)oxy)-2,3-dihydro-1H-inden-4-yl)pyridin-4-yl)-1-methyl-1H-pyrrolo[2,3-b]pyridin-3-yl)methyl)amino)methyl)pyrrolidin-2-one;
   (S)-5-((((6-(3-chloro-2-((R)-1-((6-methoxy-5-(((((R)-5-oxopyrrolidin-2-yl)methyl)amino)methyl)-3-(trifluoromethyl)pyridin-2-yl)oxy)-2,3-dihydro-1H-inden-4-yl)pyridin-4-yl)-1-methyl-1H-pyrrolo[2,3-b]pyridin-3-yl)methyl)amino)methyl)pyrrolidin-2-one;
   (S)-5-((((6-(3-chloro-2-((R)-1-((6-methoxy-5-(((((S)-5-oxopyrrolidin-2-yl)methyl)amino)methyl)-3-(trifluoromethyl)pyridin-2-yl)oxy)-2,3-dihydro-1H-inden-4-yl)pyridin-4-yl)-1-methyl-1H-pyrrolo[2,3-b]pyridin-3-yl)methyl)amino)methyl)pyrrolidin-2-one;
   (S)-5-((((6-(3-chloro-2-((S)-1-((6-methoxy-5-(((((R)-5-oxopyrrolidin-2-yl)methyl)amino)methyl)-3-(trifluoromethyl)pyridin-2-yl)oxy)-2,3-dihydro-1H-inden-4-yl)pyridin-4-yl)-1-methyl-1H-pyrrolo[2,3-b]pyridin-3-yl)methyl)amino)methyl)pyrrolidin-2-one;
   1-(4-(((6-(2-chloro-3-(3-chloro-2-(3-methoxy-4-(((1-(3-methoxypropanoyl)piperidin-4-yl)amino)methyl)phenyl)pyridin-4-yl)phenyl)-2-methoxypyridin-3-yl)methyl)amino)piperidin-1-yl)-3-methoxypropan-1-one;
   2-((6-(3-(6'-(((1-acetylpiperidin-4-yl)amino)methyl)-3-chloro-5'-methoxy-[2,3'-bipyridin]-4-yl)-2-chlorophenyl)-2-methoxypyridin-3-yl)methyl)-2,6-diazaspiro[3.4]octan-7-one;
   2-((6-(2-chloro-3-(3-chloro-5'-methoxy-6'-(((tetrahydro-2H-pyran-4-yl)amino)methyl)-[2,3'-bipyridin]-4-yl)phenyl)-2-methoxypyridin-3-yl)methyl)-2,6-diazaspiro[3.4]octan-7-one;
   2-((6-(2-chloro-3-(3-chloro-6'-((((1r,4r)-4-hydroxycyclohexyl)amino)methyl)-5'-methoxy-[2,3'-bipyridin]-4-yl)phenyl)-2-methoxypyridin-3-yl)methyl)-2,6-diazaspiro[3.4]octan-7-one;
   2-((6-(2-chloro-3-(3-chloro-6'-((((1s,4r)-4-hydroxycyclohexyl)amino)methyl)-5'-methoxy-[2,3'-bipyridin]-4-yl)phenyl)-2-methoxypyridin-3-yl)methyl)-2,6-diazaspiro[3.4]octan-7-one;
   2-((6-(2-chloro-3-(3-chloro-6'-((((1r,4s)-4-hydroxycyclohexyl)amino)methyl)-5'-methoxy-[2,3'-bipyridin]-4-yl)phenyl)-2-methoxypyridin-3-yl)methyl)-2,6-diazaspiro[3.4]octan-7-one;
   2-((6-(2-chloro-3-(3-chloro-6'-((((1s,4s)-4-hydroxycyclohexyl)amino)methyl)-5'-methoxy-[2,3'-bipyridin]-4-yl)phenyl)-2-methoxypyridin-3-yl)methyl)-2,6-diazaspiro[3.4]octan-7-one;
   (S)-2-((6-(2-chloro-3-(3-chloro-5'-methoxy-6'-((((5-oxopyrrolidin-2-yl)methyl)amino)methyl)-[2,3'-bipyridin]-4-yl)phenyl)-2-methoxypyridin-3-yl)methyl)-2,6-diazaspiro[3.4]octan-7-one;
   (R)-2-((6-(2-chloro-3-(3-chloro-5'-methoxy-6'-((((5-oxopyrrolidin-2-yl)methyl)amino)methyl)-[2,3'-bipyridin]-4-yl)phenyl)-2-methoxypyridin-3-yl)methyl)-2,6-diazaspiro[3.4]octan-7-one;
   (S)-2-((2'-(1-((5-(((1-acetylpiperidin-4-yl)amino)methyl)-6-methoxy-3-(trifluoromethyl)pyridin-2-yl)oxy)-2,3-dihydro-1H-inden-4-yl)-3'-chloro-6-methoxy-[2,4'-bipyridin]-5-yl)methyl)-2,6-diazaspiro[3.4]octan-7-one;
   (R)-2-((2'-(1-((5-(((1-acetylpiperidin-4-yl)amino)methyl)-6-methoxy-3-(trifluoromethyl)pyridin-2-yl)oxy)-2,3-dihydro-1H-inden-4-yl)-3'-chloro-6-methoxy-[2,4'-bipyridin]-5-yl)methyl)-2,6-diazaspiro[3.4]octan-7-one;
   (S)-2-((3'-chloro-6-methoxy-2'-(1-((6-methoxy-5-(((tetrahydro-2H-pyran-4-yl)amino)methyl)-3-(trifluoromethyl)pyridin-2-yl)oxy)-2,3-dihydro-1H-inden-4-yl)-[2,4'-bipyridin]-5-yl)methyl)-2,6-diazaspiro[3.4]octan-7-one;
   (R)-2-((3'-chloro-6-methoxy-2'-(1-((6-methoxy-5-(((tetrahydro-2H-pyran-4-yl)amino)methyl)-3-(trifluoromethyl)pyridin-2-yl)oxy)-2,3-dihydro-1H-inden-4-yl)-[2,4'-bipyridin]-5-yl)methyl)-2,6-diazaspiro[3.4]octan-7-one;
   2-((3'-chloro-2'-((S)-1-((5-((((S)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)-2,6-diazaspiro[3.4]octan-7-one;
   2-((3'-chloro-2'-((S)-1-((5-((((R)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)-2,6-diazaspiro[3.4]octan-7-one;
   2-((3'-chloro-2'-((R)-1-((5-((((S)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)-2,6-diazaspiro[3.4]octan-7-one;
   2-((3'-chloro-2'-((R)-1-((5-((((R)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)-2,6-diazaspiro[3.4]octan-7-one;
   2-(4-(5-chloro-6-(2-chloro-3-(6-methoxy-5-((7-oxo-2,6-diazaspiro[3.4]octan-2-yl)methyl)pyridin-2-yl)phenyl)pyrimidin-4-yl)-2-methoxybenzyl)-2,6-diazaspiro[3.4]octan-7-one;
   (S)-6-(3-chloro-4-(2-chloro-3-(6-methoxy-5-((((5-oxopyrrolidin-2-yl)methyl)amino)methyl)pyridin-2-yl)phenyl)pyridin-2-yl)-2-methoxy-3-(2-(methylamino)ethyl)pyrrolo[2,1-f][1,2,4]triazin-4(3H)-one;
   (R)-6-(3-chloro-4-(2-chloro-3-(6-methoxy-5-((((5-oxopyrrolidin-2-yl)methyl)amino)methyl)pyridin-2-yl)phenyl)pyridin-2-yl)-2-methoxy-3-(2-(methylamino)ethyl)pyrrolo[2,1-f][1,2,4]triazin-4(3H)-one;
   1-(4-((4-(3-chloro-4-(2-chloro-3-(1-methyl-3-(((tetrahydro-2H-pyran-4-yl)amino)methyl)-1H-pyrrolo[2,3-b]pyridin-6-yl)phenyl)pyridin-2-yl)-2-methoxybenzyl)amino)piperidin-1-yl)ethan-1-one;
   1-(4-((4-(3-chloro-4-(2-chloro-3-(1-methyl-3-((((tetrahydro-2H-pyran-4-yl)methyl)amino)methyl)-1H-pyrrolo[2,3-b]pyridin-6-yl)phenyl)pyridin-2-yl)-2-methoxybenzyl)amino)piperidin-1-yl)ethan-1-one;
   1-(4-((4-(3-chloro-4-(2-chloro-3-(3-(((1,1-dioxidotetrahydro-2H-thiopyran-4-yl)amino)methyl)-1-methyl-1H-pyrrolo[2,3-b]pyridin-6-yl)phenyl)pyridin-2-yl)-2-methoxybenzyl)amino)piperidin-1-yl)ethan-1-one;
   1-(4-((4-(3-chloro-4-(2-chloro-3-(3-(((2-hydroxyethyl)amino)methyl)-1-methyl-1H-pyrrolo[2,3-b]pyridin-6-yl)phenyl)pyridin-2-yl)-2-methoxybenzyl)amino)piperidin-1-yl)ethan-1-one;
   1-(4-(((6-(2-chloro-3-(3-chloro-2-(4-(((2-hydroxyethyl)amino)methyl)-3-methoxyphenyl)pyridin-4-yl)phenyl)-1-methyl-1H-pyrrolo[2,3-b]pyridin-3-yl)methyl)amino)piperidin-1-yl)ethan-1-one;
   (S)-5-((((6-(2-chloro-3-(3-chloro-2-(4-methyl-1,1-dioxido-2,3,4,5-tetrahydrobenzo[f][1,4]thiazepin-8-yl)pyridin-4-yl)phenyl)-2-methoxypyridin-3-yl)methyl)amino)methyl)pyrrolidin-2-one;
   (R)-5-((((6-(2-chloro-3-(3-chloro-2-(4-methyl-1,1-dioxido-2,3,4,5-tetrahydrobenzo[f][1,4]thiazepin-8-yl)pyridin-4-yl)phenyl)-2-methoxypyridin-3-yl)methyl)amino)methyl)pyrrolidin-2-one;
   1-(4-((6-(3-(6-(4-((4-acetylpiperazin-1-yl)methyl)-3-methoxyphenyl)-5-chloropyrimidin-4-yl)-2-chlorophenyl)-2-methoxypyridin-3-yl)methyl)piperazin-1-yl)ethan-1-one;
   N-((6-(2-chloro-3-(3-chloro-2-(3-methoxy-4-(((tetrahydro-2H-pyran-4-yl)amino)methyl)phenyl)pyridin-4-yl)phenyl)-1-methyl-1H-pyrrolo[2,3-b]pyridin-3-yl)methyl)tetrahydro-2H-pyran-4-amine;
   1-(4-(((6-(3-(2-(4-((6-acetyl-2,6-diazaspiro[3.3]heptan-2-yl)methyl)-3-methoxyphenyl)-3-chloropyridin-4-yl)-2-chlorophenyl)-1-methyl-1H-pyrrolo[2,3-b]pyridin-3-yl)methyl)amino)piperidin-1-yl)ethan-1-one;
   1-(4-(((5-(3-(2-(4-(((1-acetylpiperidin-4-yl)amino)methyl)-3-methoxyphenyl)-3-chloropyridin-4-yl)-2-chlorophenyl)-3-methoxypyridin-2-yl)methyl)amino)piperidin-1-yl)ethan-1-one;
   2-(4-(3-chloro-4-(2-chloro-3-(5-methoxy-6-((7-oxo-2,6-diazaspiro[3.4]octan-2-yl)methyl)pyridin-3-yl)phenyl)pyridin-2-yl)-2-methoxybenzyl)-2,6-diazaspiro[3.4]octan-7-one;
   1-(4-(((5-(3-(6'-(((1-acetylpiperidin-4-yl)amino)methyl)-3-chloro-5'-methoxy-[2,3'-bipyridin]-4-yl)-2-chlorophenyl)-3-methoxypyridin-2-yl)methyl)amino)piperidin-1-yl)ethan-1-one;
   2-(4-(5-chloro-6-(2-chloro-3-(5-((6-hydroxy-2-azaspiro[3.3]heptan-2-yl)methyl)-6-methoxypyridin-2-yl)phenyl)pyrimidin-4-yl)-2-methoxybenzyl)-2-azaspiro[3.3]heptan-6-ol;
   2-((5-(2-chloro-3-(3-chloro-5'-methoxy-6'-((7-oxo-2,6-diazaspiro[3.4]octan-2-yl)methyl)-[2,3'-bipyridin]-4-yl)phenyl)-3-methoxypyridin-2-yl)methyl)-2,6-diazaspiro[3.4]octan-7-one;
   N-((5-(2-chloro-3-(3-chloro-5'-methoxy-6'-(((tetrahydro-2H-pyran-4-yl)amino)methyl)-[2,3'-bipyridin]-4-yl)phenyl)-3-methoxypyridin-2-yl)methyl)tetrahydro-2H-pyran-4-amine;
   2-(4-(3-chloro-4-(2-chloro-3-(6-methoxy-5-((6-methyl-7-oxo-2,6-diazaspiro[3.4]octan-2-yl)methyl)pyridin-2-yl)phenyl)pyridin-2-yl)-2-methoxybenzyl)-6-methyl-2,6-diazaspiro[3.4]octan-7-one;
   1-(4-(((6-(3-(5-(((1-acetylpiperidin-4-yl)amino)methyl)-3'-chloro-6-methoxy-[2,4'-bipyridin]-2'-yl)-2-chlorophenyl)-2-methoxypyridin-3-yl)methyl)amino)piperidin-1-yl)ethan-1-one;
   2-((6-(2-chloro-3-(3'-chloro-6-methoxy-5-((7-oxo-2,6-diazaspiro[3.4]octan-2-yl)methyl)-[2,4'-bipyridin]-2'-yl)phenyl)-2-methoxypyridin-3-yl)methyl)-2,6-diazaspiro[3.4]octan-7-one;
   N-((3'-chloro-2'-(2-chloro-3-(6-methoxy-5-(((tetrahydro-2H-pyran-4-yl)amino)methyl)pyridin-2-yl)phenyl)-6-methoxy-[2,4'-bipyridin]-5-yl)methyl)tetrahydro-2H-pyran-4-amine;
   (S)-5-((((3'-chloro-2'-(2-chloro-3-(6-methoxy-5-(((((S)-5-oxopyrrolidin-2-yl)methyl)amino)methyl)pyridin-2-yl)phenyl)-6-methoxy-[2,4'-bipyridin]-5-yl)methyl)amino)methyl)pyrrolidin-2-one;
   (S)-5-((((3'-chloro-2'-(2-chloro-3-(6-methoxy-5-(((((R)-5-oxopyrrolidin-2-yl)methyl)amino)methyl)pyridin-2-yl)phenyl)-6-methoxy-[2,4'-bipyridin]-5-yl)methyl)amino)methyl)pyrrolidin-2-one;
   (R)-5-((((3'-chloro-2'-(2-chloro-3-(6-methoxy-5-(((((S)-5-oxopyrrolidin-2-yl)methyl)amino)methyl)pyridin-2-yl)phenyl)-6-methoxy-[2,4'-bipyridin]-5-yl)methyl)amino)methyl)pyrrolidin-2-one;
   (R)-5-((((3'-chloro-2'-(2-chloro-3-(6-methoxy-5-(((((R)-5-oxopyrrolidin-2-yl)methyl)amino)methyl)pyridin-2-yl)phenyl)-6-methoxy-[2,4'-bipyridin]-5-yl)methyl)amino)methyl)pyrrolidin-2-one;
   2-(4-(3-chloro-4-(3-(6-methoxy-5-((7-oxo-2,6-diazaspiro[3.4]octan-2-yl)methyl)pyridin-2-yl)-2-(trifluoromethyl)phenyl)pyridin-2-yl)-2-methoxybenzyl)-2,6-diazaspiro[3.4]octan-7-one;
   1-(6-((6-(3-(2-(4-((6-acetyl-2,6-diazaspiro[3.3]heptan-2-yl)methyl)-3-methoxyphenyl)-3-chloropyridin-4-yl)-2-(trifluoromethyl)phenyl)-2-methoxypyridin-3-yl)methyl)-2,6-diazaspiro[3.3]heptan-2-yl)ethan-1-one;
   6-(4-(4-(3-(5-((2-oxa-6-azaspiro[3.3]heptan-6-yl)methyl)-6-methoxypyridin-2-yl)-2-(trifluoromethyl)phenyl)-3-chloropyridin-2-yl)-2-methoxybenzyl)-2-oxa-6-azaspiro[3.3]heptane;
   2-(4-(4-(3-(3-(((1-acetylpiperidin-4-yl)amino)methyl)-1-methyl-1H-pyrrolo[2,3-b]pyridin-6-yl)-2-chlorophenyl)-3-chloropyridin-2-yl)-2-methoxybenzyl)-2,6-diazaspiro[3.4]octan-7-one;
   (S)-1-(((6-(3-(3-chloro-2-(4-((((S)-2-hydroxypropyl)amino)methyl)-3-methoxyphenyl)pyridin-4-yl)-2-(trifluoromethyl)phenyl)-2-methoxypyridin-3-yl)methyl)amino)propan-2-ol;
   (S)-1-(((6-(3-(3-chloro-2-(4-((((R)-2-hydroxypropyl)amino)methyl)-3-methoxyphenyl)pyridin-4-yl)-2-(trifluoromethyl)phenyl)-2-methoxypyridin-3-yl)methyl)amino)propan-2-ol;
   (R)-1-(((6-(3-(3-chloro-2-(4-((((S)-2-hydroxypropyl)amino)methyl)-3-methoxyphenyl)pyridin-4-yl)-2-(trifluoromethyl)phenyl)-2-methoxypyridin-3-yl)methyl)amino)propan-2-ol;
   (R)-1-(((6-(3-(3-chloro-2-(4-((((R)-2-hydroxypropyl)amino)methyl)-3-methoxyphenyl)pyridin-4-yl)-2-(trifluoromethyl)phenyl)-2-methoxypyridin-3-yl)methyl)amino)propan-2-ol;
   2-(((6-(2-chloro-3-(5-chloro-6-(4-(((2-hydroxyethyl)amino)methyl)-3-methoxyphenyl)pyrimidin-4-yl)phenyl)-2-methoxypyridin-3-yl)methyl)amino)ethan-1-ol;
   5-((((6-(2-chloro-3-(3-chloro-2-(1,1-dioxido-4-(((S)-5-oxopyrrolidin-2-yl)methyl)-2,3,4,5-tetrahydrobenzo[f][1,4]thiazepin-8-yl)pyridin-4-yl)phenyl)-2-methoxypyridin-3-yl)methyl)amino)methyl)pyrrolidin-2-one;
   5-((((6-(2-chloro-3-(3-chloro-2-(1,1-dioxido-4-(((R)-5-oxopyrrolidin-2-yl)methyl)-2,3,4,5-tetrahydrobenzo[f][1,4]thiazepin-8-yl)pyridin-4-yl)phenyl)-2-methoxypyridin-3-yl)methyl)amino)methyl)pyrrolidin-2-one;
   (S)-5-((((6-(2-chloro-3-(3-chloro-2-(1,1-dioxido-4-(((S)-5-oxopyrrolidin-2-yl)methyl)-2,3,4,5-tetrahydrobenzo[f][1,4]thiazepin-8-yl)pyridin-4-yl)phenyl)-2-methoxypyridin-3-yl)methyl)amino)methyl)pyrrolidin-2-one;
   (S)-5-((((6-(2-chloro-3-(3-chloro-2-(1,1-dioxido-4-(((R)-5-oxopyrrolidin-2-yl)methyl)-2,3,4,5-tetrahydrobenzo[f][1,4]thiazepin-8-yl)pyridin-4-yl)phenyl)-2-methoxypyridin-3-yl)methyl)amino)methyl)pyrrolidin-2-one;
   (R)-5-((((6-(2-chloro-3-(3-chloro-2-(1,1-dioxido-4-(((S)-5-oxopyrrolidin-2-yl)methyl)-2,3,4,5-tetrahydrobenzo[f][1,4]thiazepin-8-yl)pyridin-4-yl)phenyl)-2-methoxypyridin-3-yl)methyl)amino)methyl)pyrrolidin-2-one;
   (R)-5-((((6-(2-chloro-3-(3-chloro-2-(1,1-dioxido-4-(((R)-5-oxopyrrolidin-2-yl)methyl)-2,3,4,5-tetrahydrobenzo[f][1,4]thiazepin-8-yl)pyridin-4-yl)phenyl)-2-methoxypyridin-3-yl)methyl)amino)methyl)pyrrolidin-2-one;
   1-(4-(((6-(2-chloro-3-(3-chloro-2-(8-ethoxy-2-((((1r,4r)-4-hydroxycyclohexyl)amino)methyl)-[1,2,4]triazolo[1,5-a]pyridin-6-yl)pyridin-4-yl)phenyl)-2-methoxypyridin-3-yl)methyl)amino)piperidin-1-yl)ethan-1-one;
   1-(4-(((6-(2-chloro-3-(3-chloro-2-(8-ethoxy-2-((((1s,4r)-4-hydroxycyclohexyl)amino)methyl)-[1,2,4]triazolo[1,5-a]pyridin-6-yl)pyridin-4-yl)phenyl)-2-methoxypyridin-3-yl)methyl)amino)piperidin-1-yl)ethan-1-one;
   1-(4-(((6-(2-chloro-3-(3-chloro-2-(8-ethoxy-2-((((1r,4s)-4-hydroxycyclohexyl)amino)methyl)-[1,2,4]triazolo[1,5-a]pyridin-6-yl)pyridin-4-yl)phenyl)-2-methoxypyridin-3-yl)methyl)amino)piperidin-1-yl)ethan-1-one;
   1-(4-(((6-(2-chloro-3-(3-chloro-2-(8-ethoxy-2-((((1s,4s)-4-hydroxycyclohexyl)amino)methyl)-[1,2,4]triazolo[1,5-a]pyridin-6-yl)pyridin-4-yl)phenyl)-2-methoxypyridin-3-yl)methyl)amino)piperidin-1-yl)ethan-1-one;
   1-(4-(((6-(2-chloro-3-(3-chloro-2-(8-ethoxy-2-((((1r,3r)-3-hydroxy-3-methylcyclobutyl)amino)methyl)-[1,2,4]triazolo[1,5-a]pyridin-6-yl)pyridin-4-yl)phenyl)-2-methoxypyridin-3-yl)methyl)amino)piperidin-1-yl)ethan-1-one;
   1-(4-(((6-(2-chloro-3-(3-chloro-2-(8-ethoxy-2-((((1s,3r)-3-hydroxy-3-methylcyclobutyl)amino)methyl)-[1,2,4]triazolo[1,5-a]pyridin-6-yl)pyridin-4-yl)phenyl)-2-methoxypyridin-3-yl)methyl)amino)piperidin-1-yl)ethan-1-one;
   1-(4-(((6-(2-chloro-3-(3-chloro-2-(8-ethoxy-2-((((1r,3s)-3-hydroxy-3-methylcyclobutyl)amino)methyl)-[1,2,4]triazolo[1,5-a]pyridin-6-yl)pyridin-4-yl)phenyl)-2-methoxypyridin-3-yl)methyl)amino)piperidin-1-yl)ethan-1-one;
   1-(4-(((6-(2-chloro-3-(3-chloro-2-(8-ethoxy-2-((((1s,3s)-3-hydroxy-3-methylcyclobutyl)amino)methyl)-[1,2,4]triazolo[1,5-a]pyridin-6-yl)pyridin-4-yl)phenyl)-2-methoxypyridin-3-yl)methyl)amino)piperidin-1-yl)ethan-1-one;
   1-(4-(((6-(4-(3-(5-(((1-acetylpiperidin-4-yl)amino)methyl)-6-methoxypyridin-2-yl)-2-chlorophenyl)-3-chloropyridin-2-yl)-8-ethoxy-[1,2,4]triazolo[1,5-a]pyridin-2-yl)methyl)amino)piperidin-1-yl)ethan-1-one;
   2-((6-(4-(3-(5-(((1-acetylpiperidin-4-yl)amino)methyl)-6-methoxypyridin-2-yl)-2-chlorophenyl)-3-chloropyridin-2-yl)-8-ethoxy-[1,2,4]triazolo[1,5-a]pyridin-2-yl)methyl)-2,6-diazaspiro[3.4]octan-7-one;
   N-((6-(3-(3-chloro-2-(4-(((3-fluoropropyl)amino)methyl)-3-methoxyphenyl)pyridin-4-yl)-2-(trifluoromethyl)phenyl)-2-methoxypyridin-3-yl)methyl)-3-fluoropropan-1-amine;
   (1r,4r)-4-(((6-(3-(3-chloro-2-(4-((((1r,4r)-4-hydroxycyclohexyl)amino)methyl)-3-methoxyphenyl)pyridin-4-yl)-2-(trifluoromethyl)phenyl)-2-methoxypyridin-3-yl)methyl)amino)cyclohexan-1-ol;
   (1r,4r)-4-(((6-(3-(3-chloro-2-(4-((((1r,4s)-4-hydroxycyclohexyl)amino)methyl)-3-methoxyphenyl)pyridin-4-yl)-2-(trifluoromethyl)phenyl)-2-methoxypyridin-3-yl)methyl)amino)cyclohexan-1-ol;
   (1r,4r)-4-(((6-(3-(3-chloro-2-(4-((((1s,4r)-4-hydroxycyclohexyl)amino)methyl)-3-methoxyphenyl)pyridin-4-yl)-2-(trifluoromethyl)phenyl)-2-methoxypyridin-3-yl)methyl)amino)cyclohexan-1-ol;
   (1r,4r)-4-(((6-(3-(3-chloro-2-(4-((((1s,4s)-4-hydroxycyclohexyl)amino)methyl)-3-methoxyphenyl)pyridin-4-yl)-2-(trifluoromethyl)phenyl)-2-methoxypyridin-3-yl)methyl)amino)cyclohexan-1-ol;
   (1r,4s)-4-(((6-(3-(3-chloro-2-(4-((((1r,4r)-4-hydroxycyclohexyl)amino)methyl)-3-methoxyphenyl)pyridin-4-yl)-2-(trifluoromethyl)phenyl)-2-methoxypyridin-3-yl)methyl)amino)cyclohexan-1-ol;
   (1r,4s)-4-(((6-(3-(3-chloro-2-(4-((((1r,4s)-4-hydroxycyclohexyl)amino)methyl)-3-methoxyphenyl)pyridin-4-yl)-2-(trifluoromethyl)phenyl)-2-methoxypyridin-3-yl)methyl)amino)cyclohexan-1-ol;
   (1r,4s)-4-(((6-(3-(3-chloro-2-(4-((((1s,4r)-4-hydroxycyclohexyl)amino)methyl)-3-methoxyphenyl)pyridin-4-yl)-2-(trifluoromethyl)phenyl)-2-methoxypyridin-3-yl)methyl)amino)cyclohexan-1-ol;
   (1r,4s)-4-(((6-(3-(3-chloro-2-(4-((((1s,4s)-4-hydroxycyclohexyl)amino)methyl)-3-methoxyphenyl)pyridin-4-yl)-2-(trifluoromethyl)phenyl)-2-methoxypyridin-3-yl)methyl)amino)cyclohexan-1-ol;
   (1s,4r)-4-(((6-(3-(3-chloro-2-(4-((((1r,4r)-4-hydroxycyclohexyl)amino)methyl)-3-methoxyphenyl)pyridin-4-yl)-2-(trifluoromethyl)phenyl)-2-methoxypyridin-3-yl)methyl)amino)cyclohexan-1-ol;
   (1s,4r)-4-(((6-(3-(3-chloro-2-(4-((((1r,4s)-4-hydroxycyclohexyl)amino)methyl)-3-methoxyphenyl)pyridin-4-yl)-2-(trifluoromethyl)phenyl)-2-methoxypyridin-3-yl)methyl)amino)cyclohexan-1-ol;
   (1s,4r)-4-(((6-(3-(3-chloro-2-(4-((((1s,4r)-4-hydroxycyclohexyl)amino)methyl)-3-methoxyphenyl)pyridin-4-yl)-2-(trifluoromethyl)phenyl)-2-methoxypyridin-3-yl)methyl)amino)cyclohexan-1-ol;
   (1s,4r)-4-(((6-(3-(3-chloro-2-(4-((((1s,4s)-4-hydroxycyclohexyl)amino)methyl)-3-methoxyphenyl)pyridin-4-yl)-2-(trifluoromethyl)phenyl)-2-methoxypyridin-3-yl)methyl)amino)cyclohexan-1-ol;
   (1s,4s)-4-(((6-(3-(3-chloro-2-(4-((((1r,4r)-4-hydroxycyclohexyl)amino)methyl)-3-methoxyphenyl)pyridin-4-yl)-2-(trifluoromethyl)phenyl)-2-methoxypyridin-3-yl)methyl)amino)cyclohexan-1-ol;
   (1s,4s)-4-(((6-(3-(3-chloro-2-(4-((((1r,4s)-4-hydroxycyclohexyl)amino)methyl)-3-methoxyphenyl)pyridin-4-yl)-2-(trifluoromethyl)phenyl)-2-methoxypyridin-3-yl)methyl)amino)cyclohexan-1-ol;
   (1s,4s)-4-(((6-(3-(3-chloro-2-(4-((((1s,4r)-4-hydroxycyclohexyl)amino)methyl)-3-methoxyphenyl)pyridin-4-yl)-2-(trifluoromethyl)phenyl)-2-methoxypyridin-3-yl)methyl)amino)cyclohexan-1-ol;
   (1s,4s)-4-(((6-(3-(3-chloro-2-(4-((((1s,4s)-4-hydroxycyclohexyl)amino)methyl)-3-methoxyphenyl)pyridin-4-yl)-2-(trifluoromethyl)phenyl)-2-methoxypyridin-3-yl)methyl)amino)cyclohexan-1-ol;
   1-(6-((6-(3-(6-(4-((6-acetyl-2,6-diazaspiro[3.3]heptan-2-yl)methyl)-3-methoxyphenyl)-5-chloropyrimidin-4-yl)-2-chlorophenyl)-2-methoxypyridin-3-yl)methyl)-2,6-diazaspiro[3.3]heptan-2-yl)ethan-1-one;
   N-(3-(3-chloro-2-(3-methoxy-4-((7-oxo-2,6-diazaspiro[3.4]octan-2-yl)methyl)phenyl)pyridin-4-yl)-2-methylphenyl)-5-((7-oxo-2,6-diazaspiro[3.4]octan-2-yl)methyl)picolinamide;
   (3-(((6-(2-chloro-3-(3-chloro-2-(4-(((3-(hydroxymethyl)bicyclo[1.1.1]pentan-1-yl)amino)methyl)-3-methoxyphenyl)pyridin-4-yl)phenyl)-2-methoxypyridin-3-yl)methyl)amino)bicyclo[1.1.1]pentan-1-yl)methanol;
   (1s,3s)-3-(((6-(2-chloro-3-(5-chloro-6-(4-((((1s,3s)-3-hydroxycyclobutyl)amino)methyl)-3-methoxyphenyl)pyrimidin-4-yl)phenyl)-2-methoxypyridin-3-yl)methyl)amino)cyclobutan-1-ol;
   (1r,3r)-3-(((6-(2-chloro-3-(5-chloro-6-(4-((((1r,3r)-3-hydroxycyclobutyl)amino)methyl)-3-methoxyphenyl)pyrimidin-4-yl)phenyl)-2-methoxypyridin-3-yl)methyl)amino)cyclobutan-1-ol;
   (1r,3r)-3-(((6-(2-chloro-3-(5-chloro-6-(4-((((1r,3s)-3-hydroxycyclobutyl)amino)methyl)-3-methoxyphenyl)pyrimidin-4-yl)phenyl)-2-methoxypyridin-3-yl)methyl)amino)cyclobutan-1-ol;
   (1r,3r)-3-(((6-(2-chloro-3-(5-chloro-6-(4-((((1s,3r)-3-hydroxycyclobutyl)amino)methyl)-3-methoxyphenyl)pyrimidin-4-yl)phenyl)-2-methoxypyridin-3-yl)methyl)amino)cyclobutan-1-ol;
   (1r,3r)-3-(((6-(2-chloro-3-(5-chloro-6-(4-((((1s,3s)-3-hydroxycyclobutyl)amino)methyl)-3-methoxyphenyl)pyrimidin-4-yl)phenyl)-2-methoxypyridin-3-yl)methyl)amino)cyclobutan-1-ol;
   (1r,3s)-3-(((6-(2-chloro-3-(5-chloro-6-(4-((((1r,3r)-3-hydroxycyclobutyl)amino)methyl)-3-methoxyphenyl)pyrimidin-4-yl)phenyl)-2-methoxypyridin-3-yl)methyl)amino)cyclobutan-1-ol;
   (1r,3s)-3-(((6-(2-chloro-3-(5-chloro-6-(4-((((1r,3s)-3-hydroxycyclobutyl)amino)methyl)-3-methoxyphenyl)pyrimidin-4-yl)phenyl)-2-methoxypyridin-3-yl)methyl)amino)cyclobutan-1-ol;
   (1r,3s)-3-(((6-(2-chloro-3-(5-chloro-6-(4-((((1s,3r)-3-hydroxycyclobutyl)amino)methyl)-3-methoxyphenyl)pyrimidin-4-yl)phenyl)-2-methoxypyridin-3-yl)methyl)amino)cyclobutan-1-ol;
   (1r,3s)-3-(((6-(2-chloro-3-(5-chloro-6-(4-((((1s,3s)-3-hydroxycyclobutyl)amino)methyl)-3-methoxyphenyl)pyrimidin-4-yl)phenyl)-2-methoxypyridin-3-yl)methyl)amino)cyclobutan-1-ol;
   (1s,3r)-3-(((6-(2-chloro-3-(5-chloro-6-(4-((((1r,3r)-3-hydroxycyclobutyl)amino)methyl)-3-methoxyphenyl)pyrimidin-4-yl)phenyl)-2-methoxypyridin-3-yl)methyl)amino)cyclobutan-1-ol;
   (1s,3r)-3-(((6-(2-chloro-3-(5-chloro-6-(4-((((1r,3s)-3-hydroxycyclobutyl)amino)methyl)-3-methoxyphenyl)pyrimidin-4-yl)phenyl)-2-methoxypyridin-3-yl)methyl)amino)cyclobutan-1-ol;
   (1s,3r)-3-(((6-(2-chloro-3-(5-chloro-6-(4-((((1s,3r)-3-hydroxycyclobutyl)amino)methyl)-3-methoxyphenyl)pyrimidin-4-yl)phenyl)-2-methoxypyridin-3-yl)methyl)amino)cyclobutan-1-ol;
   (1s,3r)-3-(((6-(2-chloro-3-(5-chloro-6-(4-((((1s,3s)-3-hydroxycyclobutyl)amino)methyl)-3-methoxyphenyl)pyrimidin-4-yl)phenyl)-2-methoxypyridin-3-yl)methyl)amino)cyclobutan-1-ol;
   (1s,3s)-3-(((6-(2-chloro-3-(5-chloro-6-(4-((((1r,3r)-3-hydroxycyclobutyl)amino)methyl)-3-methoxyphenyl)pyrimidin-4-yl)phenyl)-2-methoxypyridin-3-yl)methyl)amino)cyclobutan-1-ol;
   (1s,3s)-3-(((6-(2-chloro-3-(5-chloro-6-(4-((((1r,3s)-3-hydroxycyclobutyl)amino)methyl)-3-methoxyphenyl)pyrimidin-4-yl)phenyl)-2-methoxypyridin-3-yl)methyl)amino)cyclobutan-1-ol;
   (1s,3s)-3-(((6-(2-chloro-3-(5-chloro-6-(4-((((1s,3r)-3-hydroxycyclobutyl)amino)methyl)-3-methoxyphenyl)pyrimidin-4-yl)phenyl)-2-methoxypyridin-3-yl)methyl)amino)cyclobutan-1-ol;
   1-(6-((6-(2-chloro-3-(3-chloro-2-(3-methoxy-4-((6-(2-methoxyacetyl)-2,6-diazaspiro[3.3]heptan-2-yl)methyl)phenyl)pyridin-4-yl)phenyl)-2-methoxypyridin-3-yl)methyl)-2,6-diazaspiro[3.3]heptan-2-yl)-2-methoxyethan-1-one;
   1-(4-(((6-(2-chloro-3-(3-chloro-2-(2-((((1r,4r)-4-hydroxycyclohexyl)amino)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one;
   1-(4-(((6-(2-chloro-3-(3-chloro-2-(2-((((1s,4r)-4-hydroxycyclohexyl)amino)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one;
   1-(4-(((6-(2-chloro-3-(3-chloro-2-(2-((((1r,4s)-4-hydroxycyclohexyl)amino)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one;
   1-(4-(((6-(2-chloro-3-(3-chloro-2-(2-((((1s,4s)-4-hydroxycyclohexyl)amino)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one;
   1-(4-(((6-(2-chloro-3-(3-chloro-2-(2-((((1r,3r)-3-hydroxy-3-methylcyclobutyl)amino)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one;
   1-(4-(((6-(2-chloro-3-(3-chloro-2-(2-((((1s,3r)-3-hydroxy-3-methylcyclobutyl)amino)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one;
   1-(4-(((6-(2-chloro-3-(3-chloro-2-(2-((((1r,3s)-3-hydroxy-3-methylcyclobutyl)amino)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one;
   1-(4-(((6-(2-chloro-3-(3-chloro-2-(2-((((1s,3s)-3-hydroxy-3-methylcyclobutyl)amino)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one;
   2-((6-(2-chloro-3-(3-chloro-2-(3-(difluoromethoxy)-4-((7-oxo-2,6-diazaspiro[3.4]octan-2-yl)methyl)phenyl)pyridin-4-yl)phenyl)-2-methoxypyridin-3-yl)methyl)-2,6-diazaspiro[3.4]octan-7-one;
   1-(6-((6-(3-(2-(4-((6-acetyl-2,6-diazaspiro[3.3]heptan-2-yl)methyl)-3-(difluoromethoxy)phenyl)-3-chloropyridin-4-yl)-2-chlorophenyl)-2-methoxypyridin-3-yl)methyl)-2,6-diazaspiro[3.3]heptan-2-yl)ethan-1-one;
   1-(4-(((S)-1-(6-(3-(2-(4-((S)-1-((1-acetylpiperidin-4-yl)amino)ethyl)-3-methoxyphenyl)-3-chloropyridin-4-yl)-2-chlorophenyl)-2-methoxypyridin-3-yl)ethyl)amino)piperidin-1-yl)ethan-1-one;
   1-(4-(((S)-1-(6-(3-(2-(4-((R)-1-((1-acetylpiperidin-4-yl)amino)ethyl)-3-methoxyphenyl)-3-chloropyridin-4-yl)-2-chlorophenyl)-2-methoxypyridin-3-yl)ethyl)amino)piperidin-1-yl)ethan-1-one;
   1-(4-(((R)-1-(6-(3-(2-(4-((S)-1-((1-acetylpiperidin-4-yl)amino)ethyl)-3-methoxyphenyl)-3-chloropyridin-4-yl)-2-chlorophenyl)-2-methoxypyridin-3-yl)ethyl)amino)piperidin-1-yl)ethan-1-one;
   1-(4-(((R)-1-(6-(3-(2-(4-((R)-1-((1-acetylpiperidin-4-yl)amino)ethyl)-3-methoxyphenyl)-3-chloropyridin-4-yl)-2-chlorophenyl)-2-methoxypyridin-3-yl)ethyl)amino)piperidin-1-yl)ethan-1-one;
   1-(4-(((6-(2-chloro-3-(3-chloro-2-(3-methoxy-4-(((tetrahydro-2H-pyran-4-yl)amino)methyl)phenyl)pyridin-4-yl)phenyl)-1-methyl-1H-pyrrolo[2,3-b]pyridin-3-yl)methyl)(methyl)amino)piperidin-1-yl)ethan-1-one;
   1-(4-(((6-(3-(2-(4-((6-acetyl-2,6-diazaspiro[3.3]heptan-2-yl)methyl)-3-methoxyphenyl)-3-chloropyridin-4-yl)-2-chlorophenyl)-1-methyl-1H-pyrrolo[2,3-b]pyridin-3-yl)methyl)(methyl)amino)piperidin-1-yl)ethan-1-one;
   1-(4-(((6-(3-(3-chloro-2-(3-methoxy-4-(((1-(2-methoxyacetyl)piperidin-4-yl)amino)methyl)phenyl)pyridin-4-yl)-2-(trifluoromethyl)phenyl)-2-methoxypyridin-3-yl)methyl)amino)piperidin-1-yl)-2-methoxyethan-1-one;
   2-(4-(4-(3-(3-(((1-acetylpiperidin-4-yl)(methyl)amino)methyl)-1-methyl-1H-pyrrolo[2,3-b]pyridin-6-yl)-2-chlorophenyl)-3-chloropyridin-2-yl)-2-methoxybenzyl)-2,6-diazaspiro[3.4]octan-7-one;
   N-(4-(4-(3-(5-(((2-oxaspiro[3.3]heptan-6-yl)amino)methyl)-6-methoxypyridin-2-yl)-2-(trifluoromethyl)phenyl)-3-chloropyridin-2-yl)-2-methoxybenzyl)-2-oxaspiro[3.3]heptan-6-amine;
   2-(4-(3-chloro-4-(2-chloro-3-(1-methyl-3-(((tetrahydro-2H-pyran-4-yl)amino)methyl)-1H-pyrrolo[2,3-b]pyridin-6-yl)phenyl)pyridin-2-yl)-2-methoxybenzyl)-2,6-diazaspiro[3.4]octan-7-one;
   2-((4-(3-chloro-4-(2-chloro-3-(1-methyl-3-(((tetrahydro-2H-pyran-4-yl)amino)methyl)-1H-pyrrolo[2,3-b]pyridin-6-yl)phenyl)pyridin-2-yl)-2-methoxybenzyl)amino)ethan-1-ol;
   1-(4-(((6-(3-(2-(4-(((1-acetylpiperidin-4-yl)amino)methyl)-3-methoxyphenyl)-3-chloropyridin-4-yl)-2-chlorophenyl)-2-methoxypyridin-3-yl)methyl)amino)piperidin-1-yl)-3-methoxypropan-1-one;
   (S)-5-((((3'-chloro-6-methoxy-2'-((S)-1-((6-methoxy-5-(((tetrahydro-2H-pyran-4-yl)amino)methyl)-3-(trifluoromethyl)pyridin-2-yl)oxy)-2,3-dihydro-1H-inden-4-yl)-[2,4'-bipyridin]-5-yl)methyl)amino)methyl)pyrrolidin-2-one;
   (S)-5-((((3'-chloro-6-methoxy-2'-((R)-1-((6-methoxy-5-(((tetrahydro-2H-pyran-4-yl)amino)methyl)-3-(trifluoromethyl)pyridin-2-yl)oxy)-2,3-dihydro-1H-inden-4-yl)-[2,4'-bipyridin]-5-yl)methyl)amino)methyl)pyrrolidin-2-one;
   (R)-5-((((3'-chloro-6-methoxy-2'-((S)-1-((6-methoxy-5-(((tetrahydro-2H-pyran-4-yl)amino)methyl)-3-(trifluoromethyl)pyridin-2-yl)oxy)-2,3-dihydro-1H-inden-4-yl)-[2,4'-bipyridin]-5-yl)methyl)amino)methyl)pyrrolidin-2-one;
   (R)-5-((((3'-chloro-6-methoxy-2'-((R)-1-((6-methoxy-5-(((tetrahydro-2H-pyran-4-yl)amino)methyl)-3-(trifluoromethyl)pyridin-2-yl)oxy)-2,3-dihydro-1H-inden-4-yl)-[2,4'-bipyridin]-5-yl)methyl)amino)methyl)pyrrolidin-2-one;
   1-(6-(4-(3-chloro-4-(2-chloro-3-(1-methyl-3-(((tetrahydro-2H-pyran-4-yl)amino)methyl)-1H-pyrrolo[2,3-b]pyridin-6-yl)phenyl)pyridin-2-yl)-2-methoxybenzyl)-2,6-diazaspiro[3.3]heptan-2-yl)ethan-1-one;
   (S)-5-((((6-(3-(3-chloro-2-(3-methoxy-4-(((((S)-5-oxopyrrolidin-2-yl)methyl)amino)methyl)phenyl)pyridin-4-yl)-2-(trifluoromethyl)phenyl)-2-methoxypyridin-3-yl)methyl)amino)methyl)pyrrolidin-2-one;
   (S)-5-((((6-(3-(3-chloro-2-(3-methoxy-4-(((((R)-5-oxopyrrolidin-2-yl)methyl)amino)methyl)phenyl)pyridin-4-yl)-2-(trifluoromethyl)phenyl)-2-methoxypyridin-3-yl)methyl)amino)methyl)pyrrolidin-2-one;
   (R)-5-((((6-(3-(3-chloro-2-(3-methoxy-4-(((((S)-5-oxopyrrolidin-2-yl)methyl)amino)methyl)phenyl)pyridin-4-yl)-2-(trifluoromethyl)phenyl)-2-methoxypyridin-3-yl)methyl)amino)methyl)pyrrolidin-2-one;
   (R)-5-((((6-(3-(3-chloro-2-(3-methoxy-4-(((((R)-5-oxopyrrolidin-2-yl)methyl)amino)methyl)phenyl)pyridin-4-yl)-2-(trifluoromethyl)phenyl)-2-methoxypyridin-3-yl)methyl)amino)methyl)pyrrolidin-2-one;
   (S)-5-((((6-(2-chloro-3-(3-chloro-2-(4-((S)-2-hydroxypropyl)-1,1-dioxido-2,3,4,5-tetrahydrobenzo[f][1,4]thiazepin-8-yl)pyridin-4-yl)phenyl)-2-methoxypyridin-3-yl)methyl)amino)methyl)pyrrolidin-2-one;
   (S)-5-((((6-(2-chloro-3-(3-chloro-2-(4-((R)-2-hydroxypropyl)-1,1-dioxido-2,3,4,5-tetrahydrobenzo[f][1,4]thiazepin-8-yl)pyridin-4-yl)phenyl)-2-methoxypyridin-3-yl)methyl)amino)methyl)pyrrolidin-2-one;
   (R)-5-((((6-(2-chloro-3-(3-chloro-2-(4-((S)-2-hydroxypropyl)-1,1-dioxido-2,3,4,5-tetrahydrobenzo[f][1,4]thiazepin-8-yl)pyridin-4-yl)phenyl)-2-methoxypyridin-3-yl)methyl)amino)methyl)pyrrolidin-2-one;
   (R)-5-((((6-(2-chloro-3-(3-chloro-2-(4-((R)-2-hydroxypropyl)-1,1-dioxido-2,3,4,5-tetrahydrobenzo[f][1,4]thiazepin-8-yl)pyridin-4-yl)phenyl)-2-methoxypyridin-3-yl)methyl)amino)methyl)pyrrolidin-2-one;
   2-(((6-(3-(3-chloro-2-(4-(((2-hydroxyethyl)amino)methyl)-3-methoxyphenyl)pyridin-4-yl)-2-(trifluoromethyl)phenyl)-2-methoxypyridin-3-yl)methyl)amino)ethan-1-ol;
   1-(6-(3-(3-chloro-2-(3-methoxy-4-((methylamino)methyl)phenyl)pyridin-4-yl)-2-(trifluoromethyl)phenyl)-2-methoxypyridin-3-yl)-N-methylmethanamine;
   N-((6-(3-(3-chloro-2-(3-methoxy-4-(((2-methoxyethyl)amino)methyl)phenyl)pyridin-4-yl)-2-(trifluoromethyl)phenyl)-2-methoxypyridin-3-yl)methyl)-2-methoxyethan-1-amine;
   2-(2-(4-(((1-acetylpiperidin-4-yl)amino)methyl)-3-methoxyphenyl)-3-chloropyridin-4-yl)-6-(5-(((1-acetylpiperidin-4-yl)amino)methyl)-6-methoxypyridin-2-yl)benzonitrile;
   1-(4-(((6-(2-chloro-3-(3-chloro-2-(4-((4-hydroxypiperidin-1-yl)methyl)-3-methoxyphenyl)pyridin-4-yl)phenyl)-2-methoxypyridin-3-yl)methyl)amino)piperidin-1-yl)-3-methoxypropan-1-one;
   (S)-5-((((6-(2-chloro-3-(3-chloro-2-(3-(difluoromethoxy)-4-(((((S)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
   (S)-5-((((6-(2-chloro-3-(3-chloro-2-(3-(difluoromethoxy)-4-(((((R)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
   (R)-5-((((6-(2-chloro-3-(3-chloro-2-(3-(difluoromethoxy)-4-(((((S)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
   (R)-5-((((6-(2-chloro-3-(3-chloro-2-(3-(difluoromethoxy)-4-(((((R)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
   N-((6-(2-chloro-3-(3-chloro-2-(3-(difluoromethoxy)-4-(((tetrahydro-2H-pyran-4-yl)amino)methyl)phenyl)pyridin-4-yl)phenyl)-2-methoxypyridin-3-yl)methyl)tetrahydro-2H-pyran-4-amine;
   4-(((6-(2-chloro-3-(3-chloro-2-(3-(difluoromethoxy)-4-(((1-methyl-2-oxopiperidin-4-yl)amino)methyl)phenyl)pyridin-4-yl)phenyl)-2-methoxypyridin-3-yl)methyl)amino)-1-methylpiperidin-2-one;
   2-(4-(((6-(2-chloro-3-(3-chloro-2-(3-(difluoromethoxy)-4-(((1-(2-hydroxyethyl)piperidin-4-yl)amino)methyl)phenyl)pyridin-4-yl)phenyl)-2-methoxypyridin-3-yl)methyl)amino)piperidin-1-yl)ethan-1-ol;
   1-(4-(((6-(3-(4-(4-(((1-acetylpiperidin-4-yl)amino)methyl)-3-methoxyphenyl)-3-chloropyridin-2-yl)-2-chlorophenyl)-2-methoxypyridin-3-yl)methyl)amino)piperidin-1-yl)ethan-1-one;
   2-(4-(3-chloro-2-(2-chloro-3-(6-methoxy-5-((7-oxo-2,6-diazaspiro[3.4]octan-2-yl)methyl)pyridin-2-yl)phenyl)pyridin-4-yl)-2-methoxybenzyl)-2,6-diazaspiro[3.4]octan-7-one;
   N-(4-(3-chloro-2-(2-chloro-3-(6-methoxy-5-(((tetrahydro-2H-pyran-4-yl)amino)methyl)pyridin-2-yl)phenyl)pyridin-4-yl)-2-methoxybenzyl)tetrahydro-2H-pyran-4-amine;
   (S)-5-(((4-(3-chloro-2-(2-chloro-3-(6-methoxy-5-(((((S)-5-oxopyrrolidin-2-yl)methyl)amino)methyl)pyridin-2-yl)phenyl)pyridin-4-yl)-2-methoxybenzyl)amino)methyl)pyrrolidin-2-one;
   (S)-5-(((4-(3-chloro-2-(2-chloro-3-(6-methoxy-5-(((((R)-5-oxopyrrolidin-2-yl)methyl)amino)methyl)pyridin-2-yl)phenyl)pyridin-4-yl)-2-methoxybenzyl)amino)methyl)pyrrolidin-2-one;
   (R)-5-(((4-(3-chloro-2-(2-chloro-3-(6-methoxy-5-(((((S)-5-oxopyrrolidin-2-yl)methyl)amino)methyl)pyridin-2-yl)phenyl)pyridin-4-yl)-2-methoxybenzyl)amino)methyl)pyrrolidin-2-one;
   (R)-5-(((4-(3-chloro-2-(2-chloro-3-(6-methoxy-5-(((((R)-5-oxopyrrolidin-2-yl)methyl)amino)methyl)pyridin-2-yl)phenyl)pyridin-4-yl)-2-methoxybenzyl)amino)methyl)pyrrolidin-2-one;
   2-(4-(3-chloro-4-(2-chloro-3-(6-methoxy-5-(((1-(3-methoxypropanoyl)piperidin-4-yl)amino)methyl)pyridin-2-yl)phenyl)pyridin-2-yl)-2-methoxybenzyl)-2,6-diazaspiro[3.4]octan-7-one;
   N-((6-(2-chloro-3-(5-chloro-6-(3-methoxy-4-(((tetrahydro-2H-pyran-4-yl)amino)methyl)phenyl)pyrimidin-4-yl)phenyl)-2-methoxypyridin-3-yl)methyl)tetrahydro-2H-pyran-4-amine;
   1-(4-(((6-(3-(5'-(((1-acetylpiperidin-4-yl)amino)methyl)-3-chloro-6'-methoxy-[2,2'-bipyridin]-4-yl)-2-chlorophenyl)-2-methoxypyridin-3-yl)methyl)amino)piperidin-1-yl)ethan-1-one;
   2-((6-(2-chloro-3-(3-chloro-6'-methoxy-5'-((7-oxo-2,6-diazaspiro[3.4]octan-2-yl)methyl)-[2,2'-bipyridin]-4-yl)phenyl)-2-methoxypyridin-3-yl)methyl)-2,6-diazaspiro[3.4]octan-7-one;
   N-((6-(2-chloro-3-(3-chloro-6'-methoxy-5'-(((tetrahydro-2H-pyran-4-yl)amino)methyl)-[2,2'-bipyridin]-4-yl)phenyl)-2-methoxypyridin-3-yl)methyl)tetrahydro-2H-pyran-4-amine;
   (S)-5-((((6-(2-chloro-3-(3-chloro-6'-methoxy-5'-(((((S)-5-oxopyrrolidin-2-yl)methyl)amino)methyl)-[2,2'-bipyridin]-4-yl)phenyl)-2-methoxypyridin-3-yl)methyl)amino)methyl)pyrrolidin-2-one;
   (S)-5-((((6-(2-chloro-3-(3-chloro-6'-methoxy-5'-(((((R)-5-oxopyrrolidin-2-yl)methyl)amino)methyl)-[2,2'-bipyridin]-4-yl)phenyl)-2-methoxypyridin-3-yl)methyl)amino)methyl)pyrrolidin-2-one;
   (R)-5-((((6-(2-chloro-3-(3-chloro-6'-methoxy-5'-(((((S)-5-oxopyrrolidin-2-yl)methyl)amino)methyl)-[2,2'-bipyridin]-4-yl)phenyl)-2-methoxypyridin-3-yl)methyl)amino)methyl)pyrrolidin-2-one;
   (R)-5-((((6-(2-chloro-3-(3-chloro-6'-methoxy-5'-(((((R)-5-oxopyrrolidin-2-yl)methyl)amino)methyl)-[2,2'-bipyridin]-4-yl)phenyl)-2-methoxypyridin-3-yl)methyl)amino)methyl)pyrrolidin-2-one;
   1-(4-(((6-(3-(2-(4-((6-acetyl-2,6-diazaspiro[3.3]heptan-2-yl)methyl)-3-methoxyphenyl)-3-chloropyridin-4-yl)-2-chlorophenyl)-2-methoxypyridin-3-yl)methyl)amino)piperidin-1-yl)-3-methoxypropan-1-one;
   (S)-1-(4-(((6-(2-chloro-3-(3-chloro-2-(4-(((2-hydroxypropyl)amino)methyl)-3-methoxyphenyl)pyridin-4-yl)phenyl)-2-methoxypyridin-3-yl)methyl)amino)piperidin-1-yl)-3-methoxypropan-1-one;
   (R)-1-(4-(((6-(2-chloro-3-(3-chloro-2-(4-(((2-hydroxypropyl)amino)methyl)-3-methoxyphenyl)pyridin-4-yl)phenyl)-2-methoxypyridin-3-yl)methyl)amino)piperidin-1-yl)-3-methoxypropan-1-one;
   (S)-5-((((2'-((S)-1-((5-(((1-acetylpiperidin-4-yl)amino)methyl)-6-methoxy-3-(trifluoromethyl)pyridin-2-yl)amino)-2,3-dihydro-1H-inden-4-yl)-3'-chloro-6-methoxy-[2,4'-bipyridin]-5-yl)methyl)amino)methyl)pyrrolidin-2-one;
   (S)-5-((((2'-((R)-1-((5-(((1-acetylpiperidin-4-yl)amino)methyl)-6-methoxy-3-(trifluoromethyl)pyridin-2-yl)amino)-2,3-dihydro-1H-inden-4-yl)-3'-chloro-6-methoxy-[2,4'-bipyridin]-5-yl)methyl)amino)methyl)pyrrolidin-2-one;
   (R)-5-((((2'-((S)-1-((5-(((1-acetylpiperidin-4-yl)amino)methyl)-6-methoxy-3-(trifluoromethyl)pyridin-2-yl)amino)-2,3-dihydro-1H-inden-4-yl)-3'-chloro-6-methoxy-[2,4'-bipyridin]-5-yl)methyl)amino)methyl)pyrrolidin-2-one;
   (R)-5-((((2'-((R)-1-((5-(((1-acetylpiperidin-4-yl)amino)methyl)-6-methoxy-3-(trifluoromethyl)pyridin-2-yl)amino)-2,3-dihydro-1H-inden-4-yl)-3'-chloro-6-methoxy-[2,4'-bipyridin]-5-yl)methyl)amino)methyl)pyrrolidin-2-one;
   (S)-5-((((3'-chloro-6-methoxy-2'-((S)-1-((6-methoxy-5-(((tetrahydro-2H-pyran-4-yl)amino)methyl)-3-(trifluoromethyl)pyridin-2-yl)amino)-2,3-dihydro-1H-inden-4-yl)-[2,4'-bipyridin]-5-yl)methyl)amino)methyl)pyrrolidin-2-one;
   (S)-5-((((3'-chloro-6-methoxy-2'-((R)-1-((6-methoxy-5-(((tetrahydro-2H-pyran-4-yl)amino)methyl)-3-(trifluoromethyl)pyridin-2-yl)amino)-2,3-dihydro-1H-inden-4-yl)-[2,4'-bipyridin]-5-yl)methyl)amino)methyl)pyrrolidin-2-one;
   (R)-5-((((3'-chloro-6-methoxy-2'-((S)-1-((6-methoxy-5-(((tetrahydro-2H-pyran-4-yl)amino)methyl)-3-(trifluoromethyl)pyridin-2-yl)amino)-2,3-dihydro-1H-inden-4-yl)-[2,4'-bipyridin]-5-yl)methyl)amino)methyl)pyrrolidin-2-one;
   (R)-5-((((3'-chloro-6-methoxy-2'-((R)-1-((6-methoxy-5-(((tetrahydro-2H-pyran-4-yl)amino)methyl)-3-(trifluoromethyl)pyridin-2-yl)amino)-2,3-dihydro-1H-inden-4-yl)-[2,4'-bipyridin]-5-yl)methyl)amino)methyl)pyrrolidin-2-one;
   N-((6-(2-chloro-3-(3-chloro-2-(3-methoxy-4-(((tetrahydro-2H-pyran-4-yl)amino)methyl)phenyl)pyridin-4-yl)phenyl)-1-methyl-1H-pyrrolo[2,3-b]pyridin-3-yl)methyl)-N-methyltetrahydro-2H-pyran-4-amine;
   2-(2-(4-((6-acetyl-2,6-diazaspiro[3.3]heptan-2-yl)methyl)-3-methoxyphenyl)-3-chloropyridin-4-yl)-6-(5-((6-acetyl-2,6-diazaspiro[3.3]heptan-2-yl)methyl)-6-methoxypyridin-2-yl)benzonitrile;
   1-(4-(((6-(2-chloro-3-(3-chloro-2-(2-((3-hydroxy-3-methylazetidin-1-yl)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one;
   (R)-1-(4-(((6-(2-chloro-3-(3-chloro-2-(2-(((2-hydroxypropyl)amino)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one;
   (S)-1-(4-(((6-(2-chloro-3-(3-chloro-2-(2-(((2-hydroxypropyl)amino)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one;
   1-(4-(((6-(3-(5-(((1-acetylpiperidin-4-yl)amino)methyl)-4'-chloro-6-methoxy-[2,3'-bipyridin]-5'-yl)-2-chlorophenyl)-2-methoxypyridin-3-yl)methyl)amino)piperidin-1-yl)ethan-1-one;
   2-((6-(2-chloro-3-(4'-chloro-6-methoxy-5-((7-oxo-2,6-diazaspiro[3.4]octan-2-yl)methyl)-[2,3'-bipyridin]-5'-yl)phenyl)-2-methoxypyridin-3-yl)methyl)-2,6-diazaspiro[3.4]octan-7-one;
   1-(4-(((6-(3-(5-(4-(((1-acetylpiperidin-4-yl)amino)methyl)-3-methoxyphenyl)-4-chloropyridin-3-yl)-2-chlorophenyl)-2-methoxypyridin-3-yl)methyl)amino)piperidin-1-yl)ethan-1-one;
   2-(4-(4-chloro-5-(2-chloro-3-(6-methoxy-5-((7-oxo-2,6-diazaspiro[3.4]octan-2-yl)methyl)pyridin-2-yl)phenyl)pyridin-3-yl)-2-methoxybenzyl)-2,6-diazaspiro[3.4]octan-7-one;
   1-(6-(2-chloro-3-(5-chloro-6-(3-methoxy-4-(((oxazol-5-ylmethyl)amino)methyl)phenyl)pyrimidin-4-yl)phenyl)-2-methoxypyridin-3-yl)-N-(oxazol-5-ylmethyl)methanamine;
   1-(4-(((6-(2-chloro-3-(3-chloro-2-(2-((isopropylamino)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one;
   5-(((1-acetylpiperidin-4-yl)amino)methyl)-N-(3-(2-(4-(((1-acetylpiperidin-4-yl)amino)methyl)-3-methoxyphenyl)-3-chloropyridin-4-yl)-2-methylphenyl)picolinamide;
   N-(3-(3-chloro-2-(3-methoxy-4-(((tetrahydro-2H-pyran-4-yl)amino)methyl)phenyl)pyridin-4-yl)-2-methylphenyl)-5-(((tetrahydro-2H-pyran-4-yl)amino)methyl)picolinamide;
   N-(3-(3-chloro-2-(3-methoxy-4-(((((S)-5-oxopyrrolidin-2-yl)methyl)amino)methyl)phenyl)pyridin-4-yl)-2-methylphenyl)-5-(((((S)-5-oxopyrrolidin-2-yl)methyl)amino)methyl)picolinamide;
   N-(3-(3-chloro-2-(3-methoxy-4-(((((S)-5-oxopyrrolidin-2-yl)methyl)amino)methyl)phenyl)pyridin-4-yl)-2-methylphenyl)-5-(((((R)-5-oxopyrrolidin-2-yl)methyl)amino)methyl)picolinamide;
   N-(3-(3-chloro-2-(3-methoxy-4-(((((R)-5-oxopyrrolidin-2-yl)methyl)amino)methyl)phenyl)pyridin-4-yl)-2-methylphenyl)-5-(((((S)-5-oxopyrrolidin-2-yl)methyl)amino)methyl)picolinamide;
   N-(3-(3-chloro-2-(3-methoxy-4-(((((R)-5-oxopyrrolidin-2-yl)methyl)amino)methyl)phenyl)pyridin-4-yl)-2-methylphenyl)-5-(((((R)-5-oxopyrrolidin-2-yl)methyl)amino)methyl)picolinamide;
   N-(3-(3-chloro-2-(4-(((2-hydroxyethyl)amino)methyl)-3-methoxyphenyl)pyridin-4-yl)-2-methylphenyl)-5-(((2-hydroxyethyl)amino)methyl)picolinamide;
   5-((6-acetyl-2,6-diazaspiro[3.3]heptan-2-yl)methyl)-N-(3-(2-(4-((6-acetyl-2,6-diazaspiro[3.3]heptan-2-yl)methyl)-3-methoxyphenyl)-3-chloropyridin-4-yl)-2-methylphenyl)picolinamide;
   N-(3-(3-chloro-2-(4-((((S)-2-hydroxypropyl)amino)methyl)-3-methoxyphenyl)pyridin-4-yl)-2-methylphenyl)-5-((((S)-2-hydroxypropyl)amino)methyl)picolinamide;
   N-(3-(3-chloro-2-(4-((((S)-2-hydroxypropyl)amino)methyl)-3-methoxyphenyl)pyridin-4-yl)-2-methylphenyl)-5-((((R)-2-hydroxypropyl)amino)methyl)picolinamide;
   N-(3-(3-chloro-2-(4-((((R)-2-hydroxypropyl)amino)methyl)-3-methoxyphenyl)pyridin-4-yl)-2-methylphenyl)-5-((((S)-2-hydroxypropyl)amino)methyl)picolinamide;
   N-(3-(3-chloro-2-(4-((((R)-2-hydroxypropyl)amino)methyl)-3-methoxyphenyl)pyridin-4-yl)-2-methylphenyl)-5-((((R)-2-hydroxypropyl)amino)methyl)picolinamide;
   1-(6-(((6-(3-(2-(4-(((2-acetyl-2-azaspiro[3.3]heptan-6-yl)amino)methyl)-3-methoxyphenyl)-3-chloropyridin-4-yl)-2-(trifluoromethyl)phenyl)-2-methoxypyridin-3-yl)methyl)amino)-2-azaspiro[3.3]heptan-2-yl)ethan-1-one;
   2-(3-chloro-2-(3-methoxy-4-(((tetrahydro-2H-pyran-4-yl)amino)methyl)phenyl)pyridin-4-yl)-6-(6-methoxy-5-(((tetrahydro-2H-pyran-4-yl)amino)methyl)pyridin-2-yl)benzonitrile;
   2-(3-chloro-2-(3-methoxy-4-((7-oxo-2,6-diazaspiro[3.4]octan-2-yl)methyl)phenyl)pyridin-4-yl)-6-(6-methoxy-5-((7-oxo-2,6-diazaspiro[3.4]octan-2-yl)methyl)pyridin-2-yl)benzonitrile;
   1-(4-(((6-(2-chloro-3-(3-chloro-2-(2-(((2-hydroxyethyl)amino)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one;
   1-(4-((6-(3-(2-(4-((4-acetylpiperazin-1-yl)methyl)-3-methoxyphenyl)-3-chloropyridin-4-yl)-2-chlorophenyl)-2-methoxypyridin-3-yl)methyl)piperazin-1-yl)ethan-1-one;
   N-(3-(2-(4-(((1-acetylpiperidin-4-yl)amino)methyl)-3-methoxyphenyl)-3-chloropyridin-4-yl)-2-chlorophenyl)-1,3-dimethyl-2,4-dioxo-1,2,3,4-tetrahydropyrimidine-5-carboxamide;
   (S)-N-(2-chloro-3-(3-chloro-2-(3-methoxy-4-((((5-oxopyrrolidin-2-yl)methyl)amino)methyl)phenyl)pyridin-4-yl)phenyl)-1,3-dimethyl-2,4-dioxo-1,2,3,4-tetrahydropyrimidine-5-carboxamide;
   (R)-N-(2-chloro-3-(3-chloro-2-(3-methoxy-4-((((5-oxopyrrolidin-2-yl)methyl)amino)methyl)phenyl)pyridin-4-yl)phenyl)-1,3-dimethyl-2,4-dioxo-1,2,3,4-tetrahydropyrimidine-5-carboxamide;
   N-(2-chloro-3-(3-chloro-2-(3-methoxy-4-((7-oxo-2,6-diazaspiro[3.4]octan-2-yl)methyl)phenyl)pyridin-4-yl)phenyl)-1,3-dimethyl-2,4-dioxo-1,2,3,4-tetrahydropyrimidine-5-carboxamide;
   2-((6-(4-(3-(5-(((1-acetylpiperidin-4-yl)amino)methyl)-6-methoxypyridin-2-yl)-2-chlorophenyl)-3-chloropyridin-2-yl)-8-methoxy-[1,2,4]triazolo[1,5-a]pyridin-2-yl)methyl)-2,6-diazaspiro[3.4]octan-7-one;
   1-(4-(((6-(2-chloro-3-(3-chloro-2-(2-((6-hydroxy-2-azaspiro[3.3]heptan-2-yl)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one;
   1-(4-(((6-(2-chloro-3-(3-chloro-2-(2-(((2-hydroxyethyl)amino)methyl)-4-methoxypyrrolo[2,1-f][1,2,4]triazin-6-yl)pyridin-4-yl)phenyl)-2-methoxypyridin-3-yl)methyl)amino)piperidin-1-yl)ethan-1-one;
   2-((6-(3-(2-(4-((6-acetyl-2,6-diazaspiro[3.3]heptan-2-yl)methyl)-3-(difluoromethoxy)phenyl)-3-chloropyridin-4-yl)-2-chlorophenyl)-2-methoxypyridin-3-yl)methyl)-2,6-diazaspiro[3.4]octan-7-one;
   1-(6-(4-(3-chloro-4-(2-chloro-3-(6-methoxy-5-(((tetrahydro-2H-pyran-4-yl)amino)methyl)pyridin-2-yl)phenyl)pyridin-2-yl)-2-(difluoromethoxy)benzyl)-2,6-diazaspiro[3.3]heptan-2-yl)ethan-1-one;
   1-(4-(((6-(3-(2-(4-((6-acetyl-2,6-diazaspiro[3.3]heptan-2-yl)methyl)-3-(difluoromethoxy)phenyl)-3-chloropyridin-4-yl)-2-chlorophenyl)-2-methoxypyridin-3-yl)methyl)amino)piperidin-1-yl)ethan-1-one;
   2-((6-(3-(2-(4-(((1-acetylpiperidin-4-yl)amino)methyl)-3-(difluoromethoxy)phenyl)-3-chloropyridin-4-yl)-2-chlorophenyl)-2-methoxypyridin-3-yl)methyl)-2,6-diazaspiro[3.4]octan-7-one;
   (S)-5-((((6-(((S)-4-(3'-chloro-6-methoxy-5-(((tetrahydro-2H-pyran-4-yl)amino)methyl)-[2,4'-bipyridin]-2'-yl)-2,3-dihydro-1H-inden-1-yl)amino)-2-methoxy-5-(trifluoromethyl)pyridin-3-yl)methyl)amino)methyl)pyrrolidin-2-one;
   (S)-5-((((6-(((R)-4-(3'-chloro-6-methoxy-5-(((tetrahydro-2H-pyran-4-yl)amino)methyl)-[2,4'-bipyridin]-2'-yl)-2,3-dihydro-1H-inden-1-yl)amino)-2-methoxy-5-(trifluoromethyl)pyridin-3-yl)methyl)amino)methyl)pyrrolidin-2-one;
   (R)-5-((((6-(((S)-4-(3'-chloro-6-methoxy-5-(((tetrahydro-2H-pyran-4-yl)amino)methyl)-[2,4'-bipyridin]-2'-yl)-2,3-dihydro-1H-inden-1-yl)amino)-2-methoxy-5-(trifluoromethyl)pyridin-3-yl)methyl)amino)methyl)pyrrolidin-2-one;
   (R)-5-((((6-(((R)-4-(3'-chloro-6-methoxy-5-(((tetrahydro-2H-pyran-4-yl)amino)methyl)-[2,4'-bipyridin]-2'-yl)-2,3-dihydro-1H-inden-1-yl)amino)-2-methoxy-5-(trifluoromethyl)pyridin-3-yl)methyl)amino)methyl)pyrrolidin-2-one;
   1-(6-((6-(2-chloro-3-(3-chloro-2-(4-((6-(2-hydroxyacetyl)-2,6-diazaspiro[3.3]heptan-2-yl)methyl)-3-methoxyphenyl)pyridin-4-yl)phenyl)-2-methoxypyridin-3-yl)methyl)-2,6-diazaspiro[3.3]heptan-2-yl)-2-hydroxyethan-1-one;
   1-(4-((4-(5-(((1-acetylpiperidin-4-yl)amino)methyl)-3',3"-dichloro-6-methoxy-[2,4':2',4"-terpyridin]-2"-yl)-2-methoxybenzyl)amino)piperidin-1-yl)ethan-1-one;
   2-(4-(3',3"-dichloro-6-methoxy-5-((7-oxo-2,6-diazaspiro[3.4]octan-2-yl)methyl)-[2,4':2',4"-terpyridin]-2"-yl)-2-methoxybenzyl)-2,6-diazaspiro[3.4]octan-7-one;
   2-(2-((6-(2-chloro-3-(3-chloro-2-(4-((6-(2-hydroxyethyl)-2,6-diazaspiro[3.4]octan-2-yl)methyl)-3-methoxyphenyl)pyridin-4-yl)phenyl)-2-methoxypyridin-3-yl)methyl)-2,6-diazaspiro[3.4]octan-6-yl)ethan-1-ol;
   1-(2-((6-(3-(2-(4-((6-acetyl-2,6-diazaspiro[3.4]octan-2-yl)methyl)-3-methoxyphenyl)-3-chloropyridin-4-yl)-2-chlorophenyl)-2-methoxypyridin-3-yl)methyl)-2,6-diazaspiro[3.4]octan-6-yl)ethan-1-one;
   N-(2-chloro-3-(3-chloro-2-(3-methoxy-4-((7-oxo-2,6-diazaspiro[3.4]octan-2-yl)methyl)phenyl)pyridin-4-yl)phenyl)-5-((7-oxo-2,6-diazaspiro[3.4]octan-2-yl)methyl)picolinamide;
   5-(((1-acetylpiperidin-4-yl)amino)methyl)-N-(3-(2-(4-(((1-acetylpiperidin-4-yl)amino)methyl)-3-methoxyphenyl)-3-chloropyridin-4-yl)-2-chlorophenyl)picolinamide;
   N-(2-chloro-3-(3-chloro-2-(3-methoxy-4-(((tetrahydro-2H-pyran-4-yl)amino)methyl)phenyl)pyridin-4-yl)phenyl)-5-(((tetrahydro-2H-pyran-4-yl)amino)methyl)picolinamide;
   N-(2-chloro-3-(3-chloro-2-(3-methoxy-4-(((((S)-5-oxopyrrolidin-2-yl)methyl)amino)methyl)phenyl)pyridin-4-yl)phenyl)-5-(((((S)-5-oxopyrrolidin-2-yl)methyl)amino)methyl)picolinamide;
   N-(2-chloro-3-(3-chloro-2-(3-methoxy-4-(((((S)-5-oxopyrrolidin-2-yl)methyl)amino)methyl)phenyl)pyridin-4-yl)phenyl)-5-(((((R)-5-oxopyrrolidin-2-yl)methyl)amino)methyl)picolinamide;
   N-(2-chloro-3-(3-chloro-2-(3-methoxy-4-(((((R)-5-oxopyrrolidin-2-yl)methyl)amino)methyl)phenyl)pyridin-4-yl)phenyl)-5-(((((S)-5-oxopyrrolidin-2-yl)methyl)amino)methyl)picolinamide;
   N-(2-chloro-3-(3-chloro-2-(3-methoxy-4-(((((R)-5-oxopyrrolidin-2-yl)methyl)amino)methyl)phenyl)pyridin-4-yl)phenyl)-5-(((((R)-5-oxopyrrolidin-2-yl)methyl)amino)methyl)picolinamide;
   N-(2-chloro-3-(3-chloro-2-(4-(((2-hydroxyethyl)amino)methyl)-3-methoxyphenyl)pyridin-4-yl)phenyl)-5-(((2-hydroxyethyl)amino)methyl)picolinamide;
   5-((6-acetyl-2,6-diazaspiro[3.3]heptan-2-yl)methyl)-N-(3-(2-(4-((6-acetyl-2,6-diazaspiro[3.3]heptan-2-yl)methyl)-3-methoxyphenyl)-3-chloropyridin-4-yl)-2-chlorophenyl)picolinamide;
   N-(2-chloro-3-(3-chloro-2-(4-((((S)-2-hydroxypropyl)amino)methyl)-3-methoxyphenyl)pyridin-4-yl)phenyl)-5-((((S)-2-hydroxypropyl)amino)methyl)picolinamide;
   N-(2-chloro-3-(3-chloro-2-(4-((((S)-2-hydroxypropyl)amino)methyl)-3-methoxyphenyl)pyridin-4-yl)phenyl)-5-((((R)-2-hydroxypropyl)amino)methyl)picolinamide;
   N-(2-chloro-3-(3-chloro-2-(4-((((R)-2-hydroxypropyl)amino)methyl)-3-methoxyphenyl)pyridin-4-yl)phenyl)-5-((((S)-2-hydroxypropyl)amino)methyl)picolinamide;
   N-(2-chloro-3-(3-chloro-2-(4-((((R)-2-hydroxypropyl)amino)methyl)-3-methoxyphenyl)pyridin-4-yl)phenyl)-5-((((R)-2-hydroxypropyl)amino)methyl)picolinamide;
   1-(6-((6-(2-chloro-3-(3-chloro-2-(3-methoxy-4-((6-(3-methoxypropanoyl)-2,6-diazaspiro[3.3]heptan-2-yl)methyl)phenyl)pyridin-4-yl)phenyl)-2-methoxypyridin-3-yl)methyl)-2,6-diazaspiro[3.3]heptan-2-yl)-3-methoxypropan-1-one;
   2-((2'-(3-(5-(((1-acetylpiperidin-4-yl)amino)methyl)-6-methoxypyridin-2-yl)-2-chlorophenyl)-3'-chloro-6-methoxy-[2,4'-bipyridin]-5-yl)methyl)-2,6-diazaspiro[3.4]octan-7-one;
   2-((3'-chloro-2'-(2-chloro-3-(5-((4-hydroxypiperidin-1-yl)methyl)-6-methoxypyridin-2-yl)phenyl)-6-methoxy-[2,4'-bipyridin]-5-yl)methyl)-2,6-diazaspiro[3.4]octan-7-one;
   2-((6-(3-(5-(((1-acetylpiperidin-4-yl)amino)methyl)-3'-chloro-6-methoxy-[2,4'-bipyridin]-2'-yl)-2-chlorophenyl)-2-methoxypyridin-3-yl)methyl)-2,6-diazaspiro[3.4]octan-7-one;
   2-(4-(3-chloro-4-(2-chloro-3-(6-methoxy-5-(((tetrahydro-2H-pyran-4-yl)amino)methyl)pyridin-2-yl)phenyl)pyridin-2-yl)-2-(difluoromethoxy)benzyl)-2,6-diazaspiro[3.4]octan-7-one;
   2-(4-(4-(3-(5-(((1-acetylpiperidin-4-yl)amino)methyl)-6-methoxypyridin-2-yl)-2-chlorophenyl)-3-chloropyridin-2-yl)-2-(difluoromethoxy)benzyl)-2,6-diazaspiro[3.4]octan-7-one;
   2-((6-(2-chloro-3-(3-chloro-2-(3-(difluoromethoxy)-4-(((tetrahydro-2H-pyran-4-yl)amino)methyl)phenyl)pyridin-4-yl)phenyl)-2-methoxypyridin-3-yl)methyl)-2,6-diazaspiro[3.4]octan-7-one;
   (S)-5-((((6-(((S)-4-(3'-chloro-6-methoxy-5-(((tetrahydro-2H-pyran-4-yl)amino)methyl)-[2,4'-bipyridin]-2'-yl)-2,3-dihydro-1H-inden-1-yl)oxy)-2-methoxy-5-(trifluoromethyl)pyridin-3-yl)methyl)amino)methyl)pyrrolidin-2-one;
   (R)-5-((((6-(((S)-4-(3'-chloro-6-methoxy-5-(((tetrahydro-2H-pyran-4-yl)amino)methyl)-[2,4'-bipyridin]-2'-yl)-2,3-dihydro-1H-inden-1-yl)oxy)-2-methoxy-5-(trifluoromethyl)pyridin-3-yl)methyl)amino)methyl)pyrrolidin-2-one;
   (S)-5-((((6-(((R)-4-(3'-chloro-6-methoxy-5-(((tetrahydro-2H-pyran-4-yl)amino)methyl)-[2,4'-bipyridin]-2'-yl)-2,3-dihydro-1H-inden-1-yl)oxy)-2-methoxy-5-(trifluoromethyl)pyridin-3-yl)methyl)amino)methyl)pyrrolidin-2-one;
   (R)-5-((((6-(((R)-4-(3'-chloro-6-methoxy-5-(((tetrahydro-2H-pyran-4-yl)amino)methyl)-[2,4'-bipyridin]-2'-yl)-2,3-dihydro-1H-inden-1-yl)oxy)-2-methoxy-5-(trifluoromethyl)pyridin-3-yl)methyl)amino)methyl)pyrrolidin-2-one;
   2-(4-(3-chloro-4-(2-chloro-3-(6-methoxy-5-((6-(2-methoxyacetyl)-2,6-diazaspiro[3.3]heptan-2-yl)methyl)pyridin-2-yl)phenyl)pyridin-2-yl)-2-methoxybenzyl)-2,6-diazaspiro[3.4]octan-7-one;
   2-(4-(4-(3-(5-((6-acetyl-2,6-diazaspiro[3.3]heptan-2-yl)methyl)-6-methoxypyridin-2-yl)-2-chlorophenyl)-3-chloropyridin-2-yl)-2-(difluoromethoxy)benzyl)-2,6-diazaspiro[3.4]octan-7-one;
   1-(6-((6-(2-chloro-3-(3-chloro-2-(3-(difluoromethoxy)-4-(((tetrahydro-2H-pyran-4-yl)amino)methyl)phenyl)pyridin-4-yl)phenyl)-2-methoxypyridin-3-yl)methyl)-2,6-diazaspiro[3.3]heptan-2-yl)ethan-1-one;
   1-(4-((4-(4-(3-(5-((6-acetyl-2,6-diazaspiro[3.3]heptan-2-yl)methyl)-6-methoxypyridin-2-yl)-2-chlorophenyl)-3-chloropyridin-2-yl)-2-(difluoromethoxy)benzyl)amino)piperidin-1-yl)ethan-1-one;
   1-(6-((6-(3-(2-(4-((6-acetyl-2,6-diazaspiro[3.3]heptan-2-yl)methyl)-3-methoxyphenyl)-3-chloropyridin-4-yl)-2-chlorophenyl)-2-methoxypyridin-3-yl)methyl)-2,6-diazaspiro[3.3]heptan-2-yl)-2-methoxyethan-1-one;
   1-(2-((6-(2-chloro-3-(3-chloro-2-(4-((4-hydroxypiperidin-1-yl)methyl)-3-methoxyphenyl)pyridin-4-yl)phenyl)-2-methoxypyridin-3-yl)methyl)-2,6-diazaspiro[3.4]octan-6-yl)ethan-1-one;
   1-(2-(4-(3-chloro-4-(2-chloro-3-(5-((4-hydroxypiperidin-1-yl)methyl)-6-methoxypyridin-2-yl)phenyl)pyridin-2-yl)-2-methoxybenzyl)-2,6-diazaspiro[3.4]octan-6-yl)ethan-1-one;
   N-(2-chloro-3-(3-chloro-2-(3-methoxy-4-((methylamino)methyl)phenyl)pyridin-4-yl)phenyl)-5-((methylamino)methyl)picolinamide;
   5-(((2-oxaspiro[3.3]heptan-6-yl)amino)methyl)-N-(3-(2-(4-(((2-oxaspiro[3.3]heptan-6-yl)amino)methyl)-3-methoxyphenyl)-3-chloropyridin-4-yl)-2-chlorophenyl)picolinamide;
   N-(3-(3-chloro-2-(3-methoxy-4-((methylamino)methyl)phenyl)pyridin-4-yl)-2-methylphenyl)-5-((methylamino)methyl)picolinamide;
   (S)-5-((((6-(2-chloro-3-(3-chloro-2-(2-((3-hydroxy-3-methylazetidin-1-yl)methyl)-4-methoxypyrrolo[2,1-f][1,2,4]triazin-6-yl)pyridin-4-yl)phenyl)-2-methoxypyridin-3-yl)methyl)amino)methyl)pyrrolidin-2-one;
   (R)-5-((((6-(2-chloro-3-(3-chloro-2-(2-((3-hydroxy-3-methylazetidin-1-yl)methyl)-4-methoxypyrrolo[2,1-f][1,2,4]triazin-6-yl)pyridin-4-yl)phenyl)-2-methoxypyridin-3-yl)methyl)amino)methyl)pyrrolidin-2-one;
   1-(4-(((6-(3-(2-(2-((2-oxa-6-azaspiro[3.3]heptan-6-yl)methyl)-8-methoxy-[1,2,4]triazolo[1,5-a]pyridin-6-yl)-3-chloropyridin-4-yl)-2-chlorophenyl)-2-methoxypyridin-3-yl)methyl)amino)piperidin-1-yl)ethan-1-one;
   1-(4-(((6-(2-chloro-3-(3-chloro-2-(2-((((1-hydroxycyclopropyl)methyl)amino)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one;
   N-(3-(2-(4-(((1-acetylpiperidin-4-yl)amino)methyl)-3-methoxyphenyl)-3-chloropyridin-4-yl)-2-methylphenyl)-5-(((2-hydroxyethyl)amino)methyl)picolinamide;
   5-((2-oxa-6-azaspiro[3.3]heptan-6-yl)methyl)-N-(3-(2-(4-((2-oxa-6-azaspiro[3.3]heptan-6-yl)methyl)-3-methoxyphenyl)-3-chloropyridin-4-yl)-2-chlorophenyl)picolinamide;
   N-(2-chloro-3-(3-chloro-2-(4-(((3-fluoropropyl)amino)methyl)-3-methoxyphenyl)pyridin-4-yl)phenyl)-5-(((3-fluoropropyl)amino)methyl)picolinamide;
   N-(3-(3-chloro-2-(3-methoxy-4-(((tetrahydro-2H-pyran-4-yl)amino)methyl)phenyl)pyridin-4-yl)-2-methylphenyl)-5-(((2-hydroxyethyl)amino)methyl)picolinamide;
   1-(((6-(((S)-5-(3'-chloro-6-methoxy-5-(((((S)-5-oxopyrrolidin-2-yl)methyl)amino)methyl)-[2,4'-bipyridin]-2'-yl)-1,2,3,4-tetrahydronaphthalen-1-yl)amino)-2-methoxy-5-(trifluoromethyl)pyridin-3-yl)methyl)amino)cyclopropane-1-carboxylic acid;
   1-(((6-(((R)-5-(3'-chloro-6-methoxy-5-(((((S)-5-oxopyrrolidin-2-yl)methyl)amino)methyl)-[2,4'-bipyridin]-2'-yl)-1,2,3,4-tetrahydronaphthalen-1-yl)amino)-2-methoxy-5-(trifluoromethyl)pyridin-3-yl)methyl)amino)cyclopropane-1-carboxylic acid;
   1-(((6-(((S)-5-(3'-chloro-6-methoxy-5-(((((R)-5-oxopyrrolidin-2-yl)methyl)amino)methyl)-[2,4'-bipyridin]-2'-yl)-1,2,3,4-tetrahydronaphthalen-1-yl)amino)-2-methoxy-5-(trifluoromethyl)pyridin-3-yl)methyl)amino)cyclopropane-1-carboxylic acid;
   1-(((6-(((R)-5-(3'-chloro-6-methoxy-5-(((((R)-5-oxopyrrolidin-2-yl)methyl)amino)methyl)-[2,4'-bipyridin]-2'-yl)-1,2,3,4-tetrahydronaphthalen-1-yl)amino)-2-methoxy-5-(trifluoromethyl)pyridin-3-yl)methyl)amino)cyclopropane-1-carboxylic acid;
   1-(((6-(((S)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)cyclopropane-1-carboxylic acid;
   1-(((6-(((R)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)cyclopropane-1-carboxylic acid;
   1-(((6-(((S)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)cyclopropane-1-carboxylic acid;
   1-(((6-(((R)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)cyclopropane-1-carboxylic acid;
   5-(((2-acetyl-2-azaspiro[3.3]heptan-6-yl)amino)methyl)-N-(3-(2-(4-(((2-acetyl-2-azaspiro[3.3]heptan-6-yl)amino)methyl)-3-methoxyphenyl)-3-chloropyridin-4-yl)-2-chlorophenyl)picolinamide;
   (S)-5-((((3'-chloro-2'-((S)-5-((5-((((S)-2-hydroxypropyl)amino)methyl)-6-methoxy-3-(trifluoromethyl)pyridin-2-yl)amino)-5,6,7,8-tetrahydronaphthalen-1-yl)-6-methoxy-[2,4'-bipyridin]-5-yl)methyl)amino)methyl)pyrrolidin-2-one;
   (S)-5-((((3'-chloro-2'-((R)-5-((5-((((S)-2-hydroxypropyl)amino)methyl)-6-methoxy-3-(trifluoromethyl)pyridin-2-yl)amino)-5,6,7,8-tetrahydronaphthalen- 1-yl)-6-methoxy-[2,4'-bipyridin]-5-yl)methyl)amino)methyl)pyrrolidin-2-one;
   (S)-5-((((3'-chloro-2'-((S)-5-((5-((((R)-2-hydroxypropyl)amino)methyl)-6-methoxy-3-(trifluoromethyl)pyridin-2-yl)amino)-5,6,7,8-tetrahydronaphthalen-1-yl)-6-methoxy-[2,4'-bipyridin]-5-yl)methyl)amino)methyl)pyrrolidin-2-one;
   (S)-5-((((3'-chloro-2'-((R)-5-((5-((((R)-2-hydroxypropyl)amino)methyl)-6-methoxy-3-(trifluoromethyl)pyridin-2-yl)amino)-5,6,7,8-tetrahydronaphthalen-1-yl)-6-methoxy-[2,4'-bipyridin]-5-yl)methyl)amino)methyl)pyrrolidin-2-one;
   (R)-5-((((3'-chloro-2'-((S)-5-((5-((((S)-2-hydroxypropyl)amino)methyl)-6-methoxy-3-(trifluoromethyl)pyridin-2-yl)amino)-5,6,7,8-tetrahydronaphthalen-1-yl)-6-methoxy-[2,4'-bipyridin]-5-yl)methyl)amino)methyl)pyrrolidin-2-one;
   (R)-5-((((3'-chloro-2'-((R)-5-((5-((((S)-2-hydroxypropyl)amino)methyl)-6-methoxy-3-(trifluoromethyl)pyridin-2-yl)amino)-5,6,7,8-tetrahydronaphthalen- 1-yl)-6-methoxy-[2,4'-bipyridin]-5-yl)methyl)amino)methyl)pyrrolidin-2-one;
   (R)-5-((((3'-chloro-2'-((S)-5-((5-((((R)-2-hydroxypropyl)amino)methyl)-6-methoxy-3-(trifluoromethyl)pyridin-2-yl)amino)-5,6,7,8-tetrahydronaphthalen- 1-yl)-6-methoxy-[2,4'-bipyridin]-5-yl)methyl)amino)methyl)pyrrolidin-2-one;
   (R)-5-((((3'-chloro-2'-((R)-5-((5-((((R)-2-hydroxypropyl)amino)methyl)-6-methoxy-3-(trifluoromethyl)pyridin-2-yl)amino)-5,6,7,8-tetrahydronaphthalen-1-yl)-6-methoxy-[2,4'-bipyridin]-5-yl)methyl)amino)methyl)pyrrolidin-2-one;
   (S)-N-(2-chloro-3-(5-chloro-6-(3-methoxy-4-((((5-oxopyrrolidin-2-yl)methyl)amino)methyl)phenyl)pyrimidin-4-yl)phenyl)-1,3-dimethyl-2,4-dioxo-1,2,3,4-tetrahydropyrimidine-5-carboxamide;
   (R)-N-(2-chloro-3-(5-chloro-6-(3-methoxy-4-((((5-oxopyrrolidin-2-yl)methyl)amino)methyl)phenyl)pyrimidin-4-yl)phenyl)-1,3-dimethyl-2,4-dioxo-1,2,3,4-tetrahydropyrimidine-5-carboxamide;
   (S)-5-((((3'-chloro-6-methoxy-2'-((S)-5-((6-methoxy-5-(((((S)-5-oxopyrrolidin-2-yl)methyl)amino)methyl)-3-(trifluoromethyl)pyridin-2-yl)amino)-5,6,7,8-tetrahydronaphthalen-1-yl)-[2,4'-bipyridin]-5-yl)methyl)amino)methyl)pyrrolidin-2-one;
   (S)-5-((((3'-chloro-6-methoxy-2'-((R)-5-((6-methoxy-5-(((((S)-5-oxopyrrolidin-2-yl)methyl)amino)methyl)-3-(trifluoromethyl)pyridin-2-yl)amino)-5,6,7,8-tetrahydronaphthalen-1-yl)-[2,4'-bipyridin]-5-yl)methyl)amino)methyl)pyrrolidin-2-one;
   (S)-5-((((3'-chloro-6-methoxy-2'-((S)-5-((6-methoxy-5-(((((R)-5-oxopyrrolidin-2-yl)methyl)amino)methyl)-3-(trifluoromethyl)pyridin-2-yl)amino)-5,6,7,8-tetrahydronaphthalen-1-yl)-[2,4'-bipyridin]-5-yl)methyl)amino)methyl)pyrrolidin-2-one;
   (S)-5-((((3'-chloro-6-methoxy-2'-((R)-5-((6-methoxy-5-(((((R)-5-oxopyrrolidin-2-yl)methyl)amino)methyl)-3-(trifluoromethyl)pyridin-2-yl)amino)-5,6,7,8-tetrahydronaphthalen-1-yl)-[2,4'-bipyridin]-5-yl)methyl)amino)methyl)pyrrolidin-2-one;
   (R)-5-((((3'-chloro-6-methoxy-2'-((S)-5-((6-methoxy-5-(((((S)-5-oxopyrrolidin-2-yl)methyl)amino)methyl)-3-(trifluoromethyl)pyridin-2-yl)amino)-5,6,7,8-tetrahydronaphthalen-1-yl)-[2,4'-bipyridin]-5-yl)methyl)amino)methyl)pyrrolidin-2-one;
   (R)-5-((((3'-chloro-6-methoxy-2'-((R)-5-((6-methoxy-5-(((((S)-5-oxopyrrolidin-2-yl)methyl)amino)methyl)-3-(trifluoromethyl)pyridin-2-yl)amino)-5,6,7,8-tetrahydronaphthalen-1-yl)-[2,4'-bipyridin]-5-yl)methyl)amino)methyl)pyrrolidin-2-one;
   (R)-5-((((3'-chloro-6-methoxy-2'-((S)-5-((6-methoxy-5-(((((R)-5-oxopyrrolidin-2-yl)methyl)amino)methyl)-3-(trifluoromethyl)pyridin-2-yl)amino)-5,6,7,8-tetrahydronaphthalen-1-yl)-[2,4'-bipyridin]-5-yl)methyl)amino)methyl)pyrrolidin-2-one;
   (R)-5-((((3'-chloro-6-methoxy-2'-((R)-5-((6-methoxy-5-(((((R)-5-oxopyrrolidin-2-yl)methyl)amino)methyl)-3-(trifluoromethyl)pyridin-2-yl)amino)-5,6,7,8-tetrahydronaphthalen-1-yl)-[2,4'-bipyridin]-5-yl)methyl)amino)methyl)pyrrolidin-2-one;
   (S)-5-((((6-(((S)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)methyl)pyrrolidin-2-one;
   (S)-5-((((6-(((R)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)methyl)pyrrolidin-2-one;
   (S)-5-((((6-(((S)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)methyl)pyrrolidin-2-one;
   (S)-5-((((6-(((R)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)methyl)pyrrolidin-2-one;
   (R)-5-((((6-(((S)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)methyl)pyrrolidin-2-one;
   (R)-5-((((6-(((R)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)methyl)pyrrolidin-2-one;
   (R)-5-((((6-(((S)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)methyl)pyrrolidin-2-one;
   (R)-5-((((6-(((R)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)methyl)pyrrolidin-2-one;
   2-(4-(5-chloro-6-(2-chloro-3-(5-(((2-hydroxyethyl)amino)methyl)-6-methoxypyridin-2-yl)phenyl)pyrimidin-4-yl)-2-methoxybenzyl)-2,6-diazaspiro[3.4]octan-7-one;
   (S)-N-(2-chloro-3-(4'-chloro-6-methoxy-5-((((5-oxopyrrolidin-2-yl)methyl)amino)methyl)-[2,3'-bipyridin]-5'-yl)phenyl)-1,3-dimethyl-2,4-dioxo-1,2,3,4-tetrahydropyrimidine-5-carboxamide;
   (R)-N-(2-chloro-3-(4'-chloro-6-methoxy-5-((((5-oxopyrrolidin-2-yl)methyl)amino)methyl)-[2,3'-bipyridin]-5'-yl)phenyl)-1,3-dimethyl-2,4-dioxo-1,2,3,4-tetrahydropyrimidine-5-carboxamide;
   1-(4-(((6-(2-chloro-3-(3-chloro-2-(2-(((3-fluoropropyl)amino)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one;
   1-(4-(((6-(2-chloro-3-(3-chloro-2-(2-(((2-hydroxy-2-methylpropyl)amino)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one;
   1-(4-(((6-(2-chloro-3-(3-chloro-2-(2-(((2-hydroxyethyl)(methyl)amino)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one;
   (S)-5-((((6-(2-chloro-3-(5-methoxy-6-(3-methoxy-4-(((((S)-5-oxopyrrolidin-2-yl)methyl)amino)methyl)phenyl)pyrimidin-4-yl)phenyl)-2-methoxypyridin-3-yl)methyl)amino)methyl)pyrrolidin-2-one;
   (S)-5-((((6-(2-chloro-3-(5-methoxy-6-(3-methoxy-4-(((((R)-5-oxopyrrolidin-2-yl)methyl)amino)methyl)phenyl)pyrimidin-4-yl)phenyl)-2-methoxypyridin-3-yl)methyl)amino)methyl)pyrrolidin-2-one;
   (R)-5-((((6-(2-chloro-3-(5-methoxy-6-(3-methoxy-4-(((((S)-5-oxopyrrolidin-2-yl)methyl)amino)methyl)phenyl)pyrimidin-4-yl)phenyl)-2-methoxypyridin-3-yl)methyl)amino)methyl)pyrrolidin-2-one;
   (R)-5-((((6-(2-chloro-3-(5-methoxy-6-(3-methoxy-4-(((((R)-5-oxopyrrolidin-2-yl)methyl)amino)methyl)phenyl)pyrimidin-4-yl)phenyl)-2-methoxypyridin-3-yl)methyl)amino)methyl)pyrrolidin-2-one;
   1-(4-(((6-(3-(6-(4-(((1-acetylpiperidin-4-yl)amino)methyl)-3-methoxyphenyl)-5-methoxypyrimidin-4-yl)-2-chlorophenyl)-2-methoxypyridin-3-yl)methyl)amino)piperidin-1-yl)ethan-1-one;
   (S)-N-(2-chloro-3-(3'-chloro-6-methoxy-5-((((5-oxopyrrolidin-2-yl)methyl)amino)methyl)-[2,4'-bipyridin]-2'-yl)phenyl)-1,3-dimethyl-2,4-dioxo-1,2,3,4-tetrahydropyrimidine-5-carboxamide;
   (R)-N-(2-chloro-3-(3'-chloro-6-methoxy-5-((((5-oxopyrrolidin-2-yl)methyl)amino)methyl)-[2,4'-bipyridin]-2'-yl)phenyl)-1,3-dimethyl-2,4-dioxo-1,2,3,4-tetrahydropyrimidine-5-carboxamide;
   N-(2-chloro-3-(3'-chloro-6-methoxy-5-((7-oxo-2,6-diazaspiro[3.4]octan-2-yl)methyl)-[2,4'-bipyridin]-2'-yl)phenyl)-1,3-dimethyl-2,4-dioxo-1,2,3,4-tetrahydropyrimidine-5-carboxamide;
   2-((6-(((S)-5-(3'-chloro-6-methoxy-5-(((((S)-5-oxopyrrolidin-2-yl)methyl)amino)methyl)-[2,4'-bipyridin]-2'-yl)-1,2,3,4-tetrahydronaphthalen-1-yl)amino)-2-methoxy-5-(trifluoromethyl)pyridin-3-yl)methyl)-2,6-diazaspiro[3.4]octan-7-one;
   2-((6-(((R)-5-(3'-chloro-6-methoxy-5-(((((S)-5-oxopyrrolidin-2-yl)methyl)amino)methyl)-[2,4'-bipyridin]-2'-yl)-1,2,3,4-tetrahydronaphthalen-1-yl)amino)-2-methoxy-5-(trifluoromethyl)pyridin-3-yl)methyl)-2,6-diazaspiro[3.4]octan-7-one;
   2-((6-(((S)-5-(3'-chloro-6-methoxy-5-(((((R)-5-oxopyrrolidin-2-yl)methyl)amino)methyl)-[2,4'-bipyridin]-2'-yl)-1,2,3,4-tetrahydronaphthalen-1-yl)amino)-2-methoxy-5-(trifluoromethyl)pyridin-3-yl)methyl)-2,6-diazaspiro[3.4]octan-7-one;
   2-((6-(((R)-5-(3'-chloro-6-methoxy-5-(((((R)-5-oxopyrrolidin-2-yl)methyl)amino)methyl)-[2,4'-bipyridin]-2'-yl)-1,2,3,4-tetrahydronaphthalen-1-yl)amino)-2-methoxy-5-(trifluoromethyl)pyridin-3-yl)methyl)-2,6-diazaspiro[3.4]octan-7-one;
   N-(3-(5-(((1-acetylpiperidin-4-yl)amino)methyl)-3'-chloro-6-methoxy-[2,4'-bipyridin]-2'-yl)-2-chlorophenyl)-1,3-dimethyl-2,4-dioxo-1,2,3,4-tetrahydropyrimidine-5-carboxamide;
   N-(2-chloro-3-(4'-chloro-6-methoxy-5-((7-oxo-2,6-diazaspiro[3.4]octan-2-yl)methyl)-[2,3'-bipyridin]-5'-yl)phenyl)-1,3-dimethyl-2,4-dioxo-1,2,3,4-tetrahydropyrimidine-5-carboxamide;
   N-(3-(5-(((1-acetylpiperidin-4-yl)amino)methyl)-4'-chloro-6-methoxy-[2,3'-bipyridin]-5'-yl)-2-chlorophenyl)-1,3-dimethyl-2,4-dioxo-1,2,3,4-tetrahydropyrimidine-5-carboxamide;
   2-((6-(((S)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)-2,6-diazaspiro[3.4]octan-7-one;
   2-((6-(((R)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)-2,6-diazaspiro[3.4]octan-7-one;
   2-((6-(((S)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)-2,6-diazaspiro[3.4]octan-7-one;
   2-((6-(((R)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)-2,6-diazaspiro[3.4]octan-7-one;
   N-(3-(3-chloro-2-(3-(difluoromethoxy)-4-(((((S)-5-oxopyrrolidin-2-yl)methyl)amino)methyl)phenyl)pyridin-4-yl)-2-methylphenyl)-5-(((((S)-5-oxopyrrolidin-2-yl)methyl)amino)methyl)picolinamide;
   N-(3-(3-chloro-2-(3-(difluoromethoxy)-4-(((((R)-5-oxopyrrolidin-2-yl)methyl)amino)methyl)phenyl)pyridin-4-yl)-2-methylphenyl)-5-(((((S)-5-oxopyrrolidin-2-yl)methyl)amino)methyl)picolinamide;
   N-(3-(3-chloro-2-(3-(difluoromethoxy)-4-(((((S)-5-oxopyrrolidin-2-yl)methyl)amino)methyl)phenyl)pyridin-4-yl)-2-methylphenyl)-5-(((((R)-5-oxopyrrolidin-2-yl)methyl)amino)methyl)picolinamide;
   N-(3-(3-chloro-2-(3-(difluoromethoxy)-4-(((((R)-5-oxopyrrolidin-2-yl)methyl)amino)methyl)phenyl)pyridin-4-yl)-2-methylphenyl)-5-(((((R)-5-oxopyrrolidin-2-yl)methyl)amino)methyl)picolinamide;
   N-(3-(3-chloro-2-(3-fluoro-5-methoxy-4-(((((S)-5-oxopyrrolidin-2-yl)methyl)amino)methyl)phenyl)pyridin-4-yl)-2-methylphenyl)-5-(((((S)-5-oxopyrrolidin-2-yl)methyl)amino)methyl)picolinamide;
   N-(3-(3-chloro-2-(3-fluoro-5-methoxy-4-(((((R)-5-oxopyrrolidin-2-yl)methyl)amino)methyl)phenyl)pyridin-4-yl)-2-methylphenyl)-5-(((((S)-5-oxopyrrolidin-2-yl)methyl)amino)methyl)picolinamide;
   N-(3-(3-chloro-2-(3-fluoro-5-methoxy-4-(((((S)-5-oxopyrrolidin-2-yl)methyl)amino)methyl)phenyl)pyridin-4-yl)-2-methylphenyl)-5-(((((R)-5-oxopyrrolidin-2-yl)methyl)amino)methyl)picolinamide;
   N-(3-(3-chloro-2-(3-fluoro-5-methoxy-4-(((((R)-5-oxopyrrolidin-2-yl)methyl)amino)methyl)phenyl)pyridin-4-yl)-2-methylphenyl)-5-(((((R)-5-oxopyrrolidin-2-yl)methyl)amino)methyl)picolinamide;
   2-(4-(6-(2-chloro-3-(6-methoxy-5-((7-oxo-2,6-diazaspiro[3.4]octan-2-yl)methyl)pyridin-2-yl)phenyl)-5-methoxypyrimidin-4-yl)-2-methoxybenzyl)-2,6-diazaspiro[3.4]octan-7-one;
   2-((4-(6-(2-chloro-3-(5-(((2-hydroxyethyl)amino)methyl)-6-methoxypyridin-2-yl)phenyl)-5-methoxypyrimidin-4-yl)-2-methoxybenzyl)amino)ethan-1-ol;
   (S)-N-(2-chloro-3-(4'-chloro-5-(((2-hydroxypropyl)amino)methyl)-6-methoxy-[2,3'-bipyridin]-5'-yl)phenyl)-1,3-dimethyl-2,4-dioxo-1,2,3,4-tetrahydropyrimidine-5-carboxamide;
   (R)-N-(2-chloro-3-(4'-chloro-5-(((2-hydroxypropyl)amino)methyl)-6-methoxy-[2,3'-bipyridin]-5'-yl)phenyl)-1,3-dimethyl-2,4-dioxo-1,2,3,4-tetrahydropyrimidine-5-carboxamide;
   N-(3-(5-((6-acetyl-2,6-diazaspiro[3.3]heptan-2-yl)methyl)-4'-chloro-6-methoxy-[2,3'-bipyridin]-5'-yl)-2-chlorophenyl)-1,3-dimethyl-2,4-dioxo-1,2,3,4-tetrahydropyrimidine-5-carboxamide;
   N-(3-(5-((6-acetyl-2,6-diazaspiro[3.3]heptan-2-yl)methyl)-3'-chloro-6-methoxy-[2,4'-bipyridin]-2'-yl)-2-chlorophenyl)-1,3-dimethyl-2,4-dioxo-1,2,3,4-tetrahydropyrimidine-5-carboxamide;
   (S)-N-(5-(3'-chloro-6-methoxy-5-((methylamino)methyl)-[2,4'-bipyridin]-2'-yl)-1,2,3,4-tetrahydronaphthalen-1-yl)-6-methoxy-5-((methylamino)methyl)-3-(trifluoromethyl)pyridin-2-amine;
   (R)-N-(5-(3'-chloro-6-methoxy-5-((methylamino)methyl)-[2,4'-bipyridin]-2'-yl)-1,2,3,4-tetrahydronaphthalen-1-yl)-6-methoxy-5-((methylamino)methyl)-3-(trifluoromethyl)pyridin-2-amine;
   N-(3-(2-(4-(((1-acetylpiperidin-4-yl)amino)methyl)-3-methoxyphenyl)-3-chloropyridin-4-yl)-2-chlorophenyl)-5-(((2-hydroxyethyl)amino)methyl)picolinamide;
   N-((6-(2-chloro-3-(5-methoxy-6-(3-methoxy-4-(((tetrahydro-2H-pyran-4-yl)amino)methyl)phenyl)pyrimidin-4-yl)phenyl)-2-methoxypyridin-3-yl)methyl)tetrahydro-2H-pyran-4-amine;
   (S)-N-(3-(3-chloro-2-(3-methoxy-4-((((5-oxopyrrolidin-2-yl)methyl)amino)methyl)phenyl)pyridin-4-yl)-2-methylphenyl)-5-(((2-hydroxyethyl)amino)methyl)picolinamide;
   (R)-N-(3-(3-chloro-2-(3-methoxy-4-((((5-oxopyrrolidin-2-yl)methyl)amino)methyl)phenyl)pyridin-4-yl)-2-methylphenyl)-5-(((2-hydroxyethyl)amino)methyl)picolinamide;
   (S)-5-(((4-(3-chloro-4-((S)-5-((5-((((S)-2-hydroxypropyl)amino)methyl)-6-methoxy-3-(trifluoromethyl)pyridin-2-yl)amino)-5,6,7,8-tetrahydronaphthalen-1-yl)pyridin-2-yl)-2-methoxybenzyl)amino)methyl)pyrrolidin-2-one;
   (S)-5-(((4-(3-chloro-4-((R)-5-((5-((((S)-2-hydroxypropyl)amino)methyl)-6-methoxy-3-(trifluoromethyl)pyridin-2-yl)amino)-5,6,7,8-tetrahydronaphthalen-1-yl)pyridin-2-yl)-2-methoxybenzyl)amino)methyl)pyrrolidin-2-one;
   (S)-5-(((4-(3-chloro-4-((S)-5-((5-((((R)-2-hydroxypropyl)amino)methyl)-6-methoxy-3-(trifluoromethyl)pyridin-2-yl)amino)-5,6,7,8-tetrahydronaphthalen-1-yl)pyridin-2-yl)-2-methoxybenzyl)amino)methyl)pyrrolidin-2-one;
   (S)-5-(((4-(3-chloro-4-((R)-5-((5-((((R)-2-hydroxypropyl)amino)methyl)-6-methoxy-3-(trifluoromethyl)pyridin-2-yl)amino)-5,6,7,8-tetrahydronaphthalen-1-yl)pyridin-2-yl)-2-methoxybenzyl)amino)methyl)pyrrolidin-2-one;
   (R)-5-(((4-(3-chloro-4-((S)-5-((5-((((S)-2-hydroxypropyl)amino)methyl)-6-methoxy-3-(trifluoromethyl)pyridin-2-yl)amino)-5,6,7,8-tetrahydronaphthalen-1-yl)pyridin-2-yl)-2-methoxybenzyl)amino)methyl)pyrrolidin-2-one;
   (R)-5-(((4-(3-chloro-4-((R)-5-((5-((((S)-2-hydroxypropyl)amino)methyl)-6-methoxy-3-(trifluoromethyl)pyridin-2-yl)amino)-5,6,7,8-tetrahydronaphthalen-1-yl)pyridin-2-yl)-2-methoxybenzyl)amino)methyl)pyrrolidin-2-one;
   (R)-5-(((4-(3-chloro-4-((S)-5-((5-((((R)-2-hydroxypropyl)amino)methyl)-6-methoxy-3-(trifluoromethyl)pyridin-2-yl)amino)-5,6,7,8-tetrahydronaphthalen-1-yl)pyridin-2-yl)-2-methoxybenzyl)amino)methyl)pyrrolidin-2-one;
   (R)-5-(((4-(3-chloro-4-((R)-5-((5-((((R)-2-hydroxypropyl)amino)methyl)-6-methoxy-3-(trifluoromethyl)pyridin-2-yl)amino)-5,6,7,8-tetrahydronaphthalen-1-yl)pyridin-2-yl)-2-methoxybenzyl)amino)methyl)pyrrolidin-2-one;
   2-(4-(5-chloro-6-(2-chloro-3-(5-((((1s,3s)-3-hydroxycyclobutyl)amino)methyl)-6-methoxypyridin-2-yl)phenyl)pyrimidin-4-yl)-2-methoxybenzyl)-2,6-diazaspiro[3.4]octan-7-one;
   2-(4-(5-chloro-6-(2-chloro-3-(5-((((1s,3r)-3-hydroxycyclobutyl)amino)methyl)-6-methoxypyridin-2-yl)phenyl)pyrimidin-4-yl)-2-methoxybenzyl)-2,6-diazaspiro[3.4]octan-7-one;
   2-(4-(5-chloro-6-(2-chloro-3-(5-((((1r,3s)-3-hydroxycyclobutyl)amino)methyl)-6-methoxypyridin-2-yl)phenyl)pyrimidin-4-yl)-2-methoxybenzyl)-2,6-diazaspiro[3.4]octan-7-one;
   2-(4-(5-chloro-6-(2-chloro-3-(5-((((1r,3r)-3-hydroxycyclobutyl)amino)methyl)-6-methoxypyridin-2-yl)phenyl)pyrimidin-4-yl)-2-methoxybenzyl)-2,6-diazaspiro[3.4]octan-7-one;
   (S)-N-(2-chloro-3-(3-chloro-6'-methoxy-5'-((((5-oxopyrrolidin-2-yl)methyl)amino)methyl)-[2,2'-bipyridin]-4-yl)phenyl)-1,3-dimethyl-2,4-dioxo-1,2,3,4-tetrahydropyrimidine-5-carboxamide;
   (R)-N-(2-chloro-3-(3-chloro-6'-methoxy-5'-((((5-oxopyrrolidin-2-yl)methyl)amino)methyl)-[2,2'-bipyridin]-4-yl)phenyl)-1,3-dimethyl-2,4-dioxo-1,2,3,4-tetrahydropyrimidine-5-carboxamide;
   (S)-N-(2-chloro-3-(3-chloro-5'-(((2-hydroxypropyl)amino)methyl)-6'-methoxy-[2,2'-bipyridin]-4-yl)phenyl)-1,3-dimethyl-2,4-dioxo-1,2,3,4-tetrahydropyrimidine-5-carboxamide;
   (R)-N-(2-chloro-3-(3-chloro-5'-(((2-hydroxypropyl)amino)methyl)-6'-methoxy-[2,2'-bipyridin]-4-yl)phenyl)-1,3-dimethyl-2,4-dioxo-1,2,3,4-tetrahydropyrimidine-5-carboxamide;
   (S)-N-(5-(3-chloro-2-(3-methoxy-4-((methylamino)methyl)phenyl)pyridin-4-yl)-1,2,3,4-tetrahydronaphthalen-1-yl)-6-methoxy-5-((methylamino)methyl)-3-(trifluoromethyl)pyridin-2-amine;
   (R)-N-(5-(3-chloro-2-(3-methoxy-4-((methylamino)methyl)phenyl)pyridin-4-yl)-1,2,3,4-tetrahydronaphthalen-1-yl)-6-methoxy-5-((methylamino)methyl)-3-(trifluoromethyl)pyridin-2-amine;
   (S)-2-(4-(5-chloro-6-(2-chloro-3-(6-methoxy-5-((((5-oxopyrrolidin-2-yl)methyl)amino)methyl)pyridin-2-yl)phenyl)pyrimidin-4-yl)-2-methoxybenzyl)-2,6-diazaspiro[3.4]octan-7-one;
   (R)-2-(4-(5-chloro-6-(2-chloro-3-(6-methoxy-5-((((5-oxopyrrolidin-2-yl)methyl)amino)methyl)pyridin-2-yl)phenyl)pyrimidin-4-yl)-2-methoxybenzyl)-2,6-diazaspiro[3.4]octan-7-one;
   2-(4-(6-(2-chloro-3-(6-methoxy-5-((6-oxo-2,5-diazaspiro[3.4]octan-2-yl)methyl)pyridin-2-yl)phenyl)-5-methoxypyrimidin-4-yl)-2-methoxybenzyl)-2,5-diazaspiro[3.4]octan-6-one;
   N-(3-(3-chloro-2-(3-methoxy-4-((7-oxo-2,6-diazaspiro[3.4]octan-2-yl)methyl)phenyl)pyridin-4-yl)-2-methylphenyl)-5-(((2-hydroxyethyl)amino)methyl)picolinamide;
   (S)-N-(2-chloro-3-(3'-chloro-5-(((2-hydroxypropyl)amino)methyl)-6-methoxy-[2,4'-bipyridin]-2'-yl)phenyl)-1,3-dimethyl-2,4-dioxo-1,2,3,4-tetrahydropyrimidine-5-carboxamide;
   (R)-N-(2-chloro-3-(3'-chloro-5-(((2-hydroxypropyl)amino)methyl)-6-methoxy-[2,4'-bipyridin]-2'-yl)phenyl)-1,3-dimethyl-2,4-dioxo-1,2,3,4-tetrahydropyrimidine-5-carboxamide;
   (S)-N-(2-chloro-3-(5-chloro-6-(4-(((2-hydroxypropyl)amino)methyl)-3-methoxyphenyl)pyrimidin-4-yl)phenyl)-1,3-dimethyl-2,4-dioxo-1,2,3,4-tetrahydropyrimidine-5-carboxamide;
   (R)-N-(2-chloro-3-(5-chloro-6-(4-(((2-hydroxypropyl)amino)methyl)-3-methoxyphenyl)pyrimidin-4-yl)phenyl)-1,3-dimethyl-2,4-dioxo-1,2,3,4-tetrahydropyrimidine-5-carboxamide;
   (S)-N-(3-(2-(4-(((1-acetylpiperidin-4-yl)amino)methyl)-3-methoxyphenyl)-3-chloropyridin-4-yl)-2-methylphenyl)-5-((((5-oxopyrrolidin-2-yl)methyl)amino)methyl)picolinamide;
   (R)-N-(3-(2-(4-(((1-acetylpiperidin-4-yl)amino)methyl)-3-methoxyphenyl)-3-chloropyridin-4-yl)-2-methylphenyl)-5-((((5-oxopyrrolidin-2-yl)methyl)amino)methyl)picolinamide;
   (S)-N-(3-(3-chloro-2-(3-methoxy-4-(((tetrahydro-2H-pyran-4-yl)amino)methyl)phenyl)pyridin-4-yl)-2-methylphenyl)-5-((((5-oxopyrrolidin-2-yl)methyl)amino)methyl)picolinamide;
   (R)-N-(3-(3-chloro-2-(3-methoxy-4-(((tetrahydro-2H-pyran-4-yl)amino)methyl)phenyl)pyridin-4-yl)-2-methylphenyl)-5-((((5-oxopyrrolidin-2-yl)methyl)amino)methyl)picolinamide;
   (S)-N-(3-(3-chloro-2-(4-(((3-fluoropropyl)amino)methyl)-3-methoxyphenyl)pyridin-4-yl)-2-methylphenyl)-5-((((5-oxopyrrolidin-2-yl)methyl)amino)methyl)picolinamide;
   (R)-N-(3-(3-chloro-2-(4-(((3-fluoropropyl)amino)methyl)-3-methoxyphenyl)pyridin-4-yl)-2-methylphenyl)-5-((((5-oxopyrrolidin-2-yl)methyl)amino)methyl)picolinamide;
   (S)-N-(3-(3-chloro-2-(4-(((2-hydroxyethyl)amino)methyl)-3-methoxyphenyl)pyridin-4-yl)-2-methylphenyl)-5-((((5-oxopyrrolidin-2-yl)methyl)amino)methyl)picolinamide;
   (R)-N-(3-(3-chloro-2-(4-(((2-hydroxyethyl)amino)methyl)-3-methoxyphenyl)pyridin-4-yl)-2-methylphenyl)-5-((((5-oxopyrrolidin-2-yl)methyl)amino)methyl)picolinamide;
   (S)-N-(2-chloro-3-(3-chloro-2-(3-methoxy-4-((((5-oxopyrrolidin-2-yl)methyl)amino)methyl)phenyl)pyridin-4-yl)phenyl)-5-((methylamino)methyl)picolinamide;
   (R)-N-(2-chloro-3-(3-chloro-2-(3-methoxy-4-((((5-oxopyrrolidin-2-yl)methyl)amino)methyl)phenyl)pyridin-4-yl)phenyl)-5-((methylamino)methyl)picolinamide;
   (S)-6-((2-chloro-3-(3-chloro-2-(3-methoxy-4-((((5-oxopyrrolidin-2-yl)methyl)amino)methyl)phenyl)pyridin-4-yl)phenyl)carbamoyl)nicotinic acid;
   (R)-6-((2-chloro-3-(3-chloro-2-(3-methoxy-4-((((5-oxopyrrolidin-2-yl)methyl)amino)methyl)phenyl)pyridin-4-yl)phenyl)carbamoyl)nicotinic acid;
   2-((6-(3-chloro-4-(2-chloro-3-(5-((isopropylamino)methyl)-6-methoxypyridin-2-yl)phenyl)pyridin-2-yl)-8-methoxy-[1,2,4]triazolo[1,5-a]pyridin-2-yl)methyl)-2-azaspiro[3.3]heptan-6-ol;
   N-(2-chloro-3-(3-chloro-2-(3-methoxy-4-((7-oxo-2,6-diazaspiro[3.4]octan-2-yl)methyl)phenyl)pyridin-4-yl)phenyl)-5-(((2-hydroxyethyl)amino)methyl)picolinamide;
   (S)-N-(2-chloro-3-(3-chloro-2-(3-methoxy-4-((((5-oxopyrrolidin-2-yl)methyl)amino)methyl)phenyl)pyridin-4-yl)phenyl)-5-(((2-hydroxyethyl)amino)methyl)picolinamide;
   (R)-N-(2-chloro-3-(3-chloro-2-(3-methoxy-4-((((5-oxopyrrolidin-2-yl)methyl)amino)methyl)phenyl)pyridin-4-yl)phenyl)-5-(((2-hydroxyethyl)amino)methyl)picolinamide;
   (S)-N-(3-(3-chloro-2-(3-methoxy-4-((((5-oxopyrrolidin-2-yl)methyl)amino)methyl)phenyl)pyridin-4-yl)-2-methylphenyl)-5-((methylamino)methyl)picolinamide;
   (R)-N-(3-(3-chloro-2-(3-methoxy-4-((((5-oxopyrrolidin-2-yl)methyl)amino)methyl)phenyl)pyridin-4-yl)-2-methylphenyl)-5-((methylamino)methyl)picolinamide;
   N-(3-(2-(4-((6-acetyl-2,6-diazaspiro[3.3]heptan-2-yl)methyl)-3-methoxyphenyl)-3-chloropyridin-4-yl)-2-chlorophenyl)-5-(((2-hydroxyethyl)amino)methyl)picolinamide;
   (1r,4r)-4-(((6-(3-chloro-4-(2-chloro-3-(5-((isopropylamino)methyl)-6-methoxypyridin-2-yl)phenyl)pyridin-2-yl)-8-methoxy-[1,2,4]triazolo[1,5-a]pyridin-2-yl)methyl)amino)cyclohexan-1-ol;
   (1s,4r)-4-(((6-(3-chloro-4-(2-chloro-3-(5-((isopropylamino)methyl)-6-methoxypyridin-2-yl)phenyl)pyridin-2-yl)-8-methoxy-[1,2,4]triazolo[1,5-a]pyridin-2-yl)methyl)amino)cyclohexan-1-ol;
   (1r,4s)-4-(((6-(3-chloro-4-(2-chloro-3-(5-((isopropylamino)methyl)-6-methoxypyridin-2-yl)phenyl)pyridin-2-yl)-8-methoxy-[1,2,4]triazolo[1,5-a]pyridin-2-yl)methyl)amino)cyclohexan-1-ol;
   (1s,4s)-4-(((6-(3-chloro-4-(2-chloro-3-(5-((isopropylamino)methyl)-6-methoxypyridin-2-yl)phenyl)pyridin-2-yl)-8-methoxy-[1,2,4]triazolo[1,5-a]pyridin-2-yl)methyl)amino)cyclohexan-1-ol;
   1-((3'-chloro-2'-(2-chloro-3-(5-((4-hydroxypiperidin-1-yl)methyl)-6-methoxypyridin-2-yl)phenyl)-6-methoxy-[2,4'-bipyridin]-5-yl)methyl)piperidin-4-ol;
   1-(2-((6-(3-(5-((6-acetyl-2,6-diazaspiro[3.4]octan-2-yl)methyl)-3'-chloro-6-methoxy-[2,4'-bipyridin]-2'-yl)-2-chlorophenyl)-2-methoxypyridin-3-yl)methyl)-2,6-diazaspiro[3.4]octan-6-yl)ethan-1-one;
   1-(4-(((6-(2-chloro-3-(3'-chloro-5-((4-hydroxypiperidin-1-yl)methyl)-6-methoxy-[2,4'-bipyridin]-2'-yl)phenyl)-2-methoxypyridin-3-yl)methyl)amino)piperidin-1-yl)ethan-1-one;
   N-(3-(3-chloro-2-(3-fluoro-4-(((2-hydroxyethyl)amino)methyl)-5-methoxyphenyl)pyridin-4-yl)-2-methylphenyl)-5-(((2-hydroxyethyl)amino)methyl)picolinamide;
   N-((6-(2-chloro-3-(3-chloro-2-(2-((isopropylamino)methyl)-8-methoxy-[1,2,4]triazolo[1,5-a]pyridin-6-yl)pyridin-4-yl)phenyl)-2-methoxypyridin-3-yl)methyl)propan-2-amine;
   (S)-5-((((6-(3-chloro-4-(2-chloro-3-(5-((isopropylamino)methyl)-6-methoxypyridin-2-yl)phenyl)pyridin-2-yl)-8-methoxy-[1,2,4]triazolo[1,5-a]pyridin-2-yl)methyl)amino)methyl)pyrrolidin-2-one;
   (R)-5-((((6-(3-chloro-4-(2-chloro-3-(5-((isopropylamino)methyl)-6-methoxypyridin-2-yl)phenyl)pyridin-2-yl)-8-methoxy-[1,2,4]triazolo[1,5-a]pyridin-2-yl)methyl)amino)methyl)pyrrolidin-2-one;
   1-(4-(((6-(3-chloro-4-(2-chloro-3-(5-((isopropylamino)methyl)-6-methoxypyridin-2-yl)phenyl)pyridin-2-yl)-8-methoxy-[1,2,4]triazolo[1,5-a]pyridin-2-yl)methyl)amino)piperidin-1-yl)ethan-1-one;
   N-(3-(3'-chloro-6-methoxy-5-(((tetrahydro-2H-pyran-4-yl)amino)methyl)-[2,4'-bipyridin]-2'-yl)-2-methylphenyl)-5-(((2-hydroxyethyl)amino)methyl)picolinamide;
   1-(6-((6-(3-chloro-4-(2-chloro-3-(5-((isopropylamino)methyl)-6-methoxypyridin-2-yl)phenyl)pyridin-2-yl)-8-methoxy-[1,2,4]triazolo[1,5-a]pyridin-2-yl)methyl)-2,6-diazaspiro[3.3]heptan-2-yl)ethan-1-one;
   N-(2-chloro-3-(3-chloro-2-(3-methoxy-4-(((tetrahydro-2H-pyran-4-yl)amino)methyl)phenyl)pyridin-4-yl)phenyl)-5-(((2-hydroxyethyl)amino)methyl)picolinamide;
   N-(3-(5-(((1-acetylpiperidin-4-yl)amino)methyl)-3'-chloro-6-methoxy-[2,4'-bipyridin]-2'-yl)-2-methylphenyl)-5-(((2-hydroxyethyl)amino)methyl)picolinamide;
   (S)-N-(3-(3-chloro-2-(3-(difluoromethoxy)-4-((((5-oxopyrrolidin-2-yl)methyl)amino)methyl)phenyl)pyridin-4-yl)-2-methylphenyl)-5-(((2-hydroxyethyl)amino)methyl)picolinamide;
   (R)-N-(3-(3-chloro-2-(3-(difluoromethoxy)-4-((((5-oxopyrrolidin-2-yl)methyl)amino)methyl)phenyl)pyridin-4-yl)-2-methylphenyl)-5-(((2-hydroxyethyl)amino)methyl)picolinamide;
   (S)-N-(3-(3'-chloro-6-methoxy-5-((((5-oxopyrrolidin-2-yl)methyl)amino)methyl)-[2,4'-bipyridin]-2'-yl)-2-methylphenyl)-5-(((2-hydroxyethyl)amino)methyl)picolinamide;
   (R)-N-(3-(3'-chloro-6-methoxy-5-((((5-oxopyrrolidin-2-yl)methyl)amino)methyl)-[2,4'-bipyridin]-2'-yl)-2-methylphenyl)-5-(((2-hydroxyethyl)amino)methyl)picolinamide;
   (R)-1-((3'-chloro-2'-(2-chloro-3-(5-(((R)-3-hydroxypyrrolidin-1-yl)methyl)-6-methoxypyridin-2-yl)phenyl)-6-methoxy-[2,4'-bipyridin]-5-yl)methyl)pyrrolidin-3-ol;
   (R)-1-((3'-chloro-2'-(2-chloro-3-(5-(((S)-3-hydroxypyrrolidin-1-yl)methyl)-6-methoxypyridin-2-yl)phenyl)-6-methoxy-[2,4'-bipyridin]-5-yl)methyl)pyrrolidin-3-ol;
   (S)-1-((3'-chloro-2'-(2-chloro-3-(5-(((R)-3-hydroxypyrrolidin-1-yl)methyl)-6-methoxypyridin-2-yl)phenyl)-6-methoxy-[2,4'-bipyridin]-5-yl)methyl)pyrrolidin-3-ol;
   (S)-1-((3'-chloro-2'-(2-chloro-3-(5-(((S)-3-hydroxypyrrolidin-1-yl)methyl)-6-methoxypyridin-2-yl)phenyl)-6-methoxy-[2,4'-bipyridin]-5-yl)methyl)pyrrolidin-3-ol; and
   1-(4-(((3'-chloro-2'-(2-chloro-3-(5-((4-hydroxypiperidin-1-yl)methyl)-6-methoxypyridin-2-yl)phenyl)-6-methoxy-[2,4'-bipyridin]-5-yl)methyl)amino)piperidin-1-yl)ethan-1-one.
16. A pharmaceutical composition comprising at least one compound of any one of items 1-15 and at least one pharmaceutically acceptable carrier.
17. The pharmaceutical composition of item 16, further comprising at least one additional agent that treats, ameliorates, and/or prevents hepatitis virus infection.
18. The pharmaceutical composition of item 17, wherein the at least one additional agent comprises at least one selected from the group consisting of reverse transcriptase inhibitor; capsid inhibitor; cccDNA formation inhibitor; RNA destabilizer; oligomeric nucleotide targeted against the HBV genome; immunostimulatory; GalNAc-siRNA conjugate targeted against an HBV gene transcript; and therapeutic vaccine.
19. The pharmaceutical composition of item 18, wherein the immunostimulator is a checkpoint inhibitor.
20. The pharmaceutical composition of item 19, wherein the checkpoint inhibitor is a PD-L1 inhibitor.
21. The pharmaceutical composition of any one of items 16-20, wherein the hepatitis virus is at least one selected from the group consisting of hepatitis B virus (HBV) and hepatitis D virus (HDV).
22. A method of treating, ameliorating, and/or preventing hepatitis virus infection in a subject, the method comprising administering to the subject in need thereof a therapeutically effective amount of the compound of any one of items 1-15 and/or the pharmaceutical composition of any one of items 16-21, or a salt, solvate, prodrug, stereoisomer, tautomer, or any mixtures thereof.
23. The method of item 22, wherein the subject is infected with hepatitis B virus (HBV).
24. The method of any one of items 22-23, wherein the subject is further infected with hepatitis D virus (HDV).
25. The method of any one of items 22-24, wherein the subject is infected with HBV and HDV.
26. The method of any one of items 22-25, wherein the subject is further administered at least one additional agent useful for treating the hepatitis virus infection.
27. The method of item 26, wherein the at least one additional agent comprises at least one selected from the group consisting of reverse transcriptase inhibitor; capsid inhibitor; cccDNA formation inhibitor; RNA destabilizer; oligomeric nucleotide targeted against the HBV genome; immunostimulator; GalNAc-siRNA conjugate targeted against an HBV gene transcript; and therapeutic vaccine.
28. The method of item 27, wherein the immunostimulatory is a checkpoint inhibitor.
29. The method of item 28, wherein the checkpoint inhibitor is a PD-L1 inhibitor.
30. The method of any one of items 26-29, wherein the subject is co-administered the at least one compound and the at least one additional agent.
31. The method of any one of items 26-30, wherein the at least one compound and the at least one additional agent are coformulated.
32. The method of any one of items 22-31, wherein the subject is a mammal.
33. The method of item 32, wherein the mammal is a human.
34. A method of treating, ameliorating, and/or preventing cancer in a subject, the method comprising administering to the subject in need thereof a therapeutically effective amount of the compound of any one of items 1-15 and/or the pharmaceutical composition of any one of items 16-21, or a salt, solvate, prodrug, stereoisomer, tautomer, or any mixtures thereof.
35. The method of item 34, wherein the compound or composition is the only anticancer agent administered to the subject.
36. The method of any one of items 34-35, wherein the compound is administered to the subject in a pharmaceutically acceptable composition.
37. The method of item 34 or 36, wherein the subject is further administered at least one additional agent or therapy useful for treating, ameliorating, and/or preventing the cancer.
38. The method of item 37, wherein the additional anticancer agent or therapy comprises nivolumab, pembrolizumab, atezolizumab, ipilimumab, chemotherapy, radiation therapy, and/or resection therapy.
39. The method of item 37, wherein the additional anticancer agent or therapy comprises Rituxan, doxorubicin, gemcitabine, nivolumab, pembrolizumab, and/or pilimumab.
40. The method of any one of items 34 and 36-39, wherein the compound or composition is coformulated and/or co-administered with the at least one additional agent.
41. The method of any one of items 34-40, wherein the cancer is amenable to treatment by inhibiting PD-1, PD-L1, or the PD-1/PD-L1 interaction.
42. The method of any one of items 34-41, wherein the cancer is at least one of pancreatic cancer, bladder cancer, colorectal cancer, breast cancer, prostate cancer, renal cancer, hepatocellular cancer, lung cancer, ovarian cancer, cervical cancer, gastric cancer, esophageal cancer, head and neck cancer, melanoma, neuroendocrine cancer, CNS cancer, brain cancer, bone cancer, soft tissue sarcoma, non-small cell lung cancer, small-cell lung cancer, or colon cancer.
43. The method of any one of items 34-41, wherein the cancer is at least one of acute lymphocytic leukemia (ALL), acute myeloid leukemia (AML), chronic lymphocytic leukemia (CLL), small lymphocytic lymphoma (SLL), myelodysplastic syndrome (MDS), myeloproliferative disease (MPD), chronic myeloid leukemia (CML), multiple myeloma (MM), non-Hodgkin's lymphoma (NHL), mantle cell lymphoma (MCL), follicular lymphoma, Waldestrom's macroglobulinemia (WM), T-cell lymphoma, B-cell lymphoma and diffuse large B-cell lymphoma (DLBCL).
44. The method of any one of items 34-43, wherein the subject is a mammal.
45. The method of item 44, wherein the mammal is a human.

## Claims

1. A compound of formula (I), or a salt, solvate, geometric isomer, stereoisomer, or tautomer thereof: 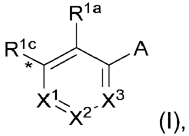 wherein:
A is 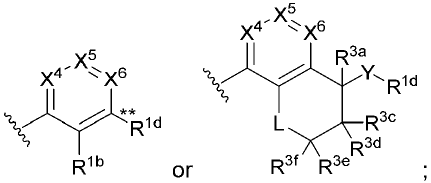
Y is NR^{3b} or O;
L is selected from the group consisting of -C(R^{3g})(R^{3h})-, -(C(R^{3g})(R^{3h}))(C(R³ⁱ)(R^{3j}))-, and a bond;
X¹ is selected from the group consisting of CR^{'1} and N;
X² is selected from the group consisting of CR^{'2} and N;
X³ is selected from the group consisting of CR^{'3} and N;
X⁴ is selected from the group consisting of CR^{"1} and N;
X⁵ is selected from the group consisting of CR^{"2} and N;
X⁶ is selected from the group consisting of CR^{"3} and N;
wherein one to four of X¹, X², X³, X⁴, X⁵, and X⁶ is N;
R^{'1}, R^{'2}, R^{'3}, R^{"1}, R^{"2}, and R^{"3} are each independently selected from the group consisting of H, halogen, C₁-C₃ alkyl, C₃-C₈ cycloalkyl, C₁-C₃ alkoxy, cyano, C₁-C₃ haloalkyl, and C₁-C₃ haloalkoxy;
R^{1a} and R^{1b} are each independently selected from the group consisting of H, halogen, C₁-C₃ alkyl, C₃-C₈ cycloalkyl, C₁-C₃ alkoxy, cyano, C₁-C₃ haloalkyl, and C₁-C₃ haloalkoxy;
R^{1c} and R^{1d} are each independently selected from the group consisting of: 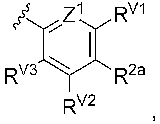 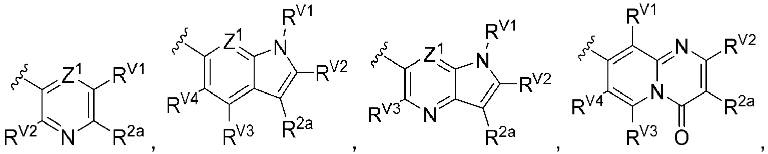 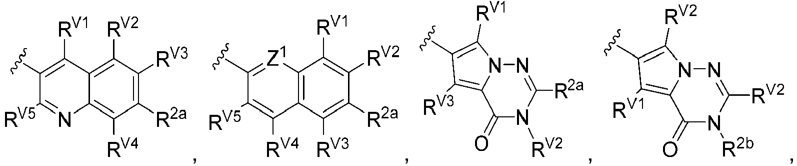 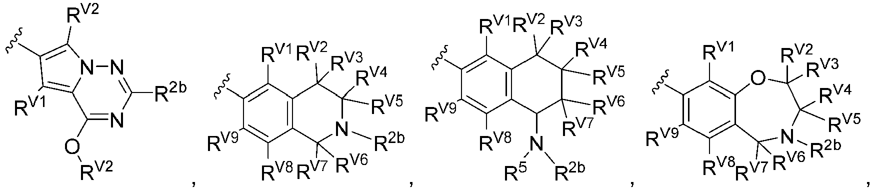 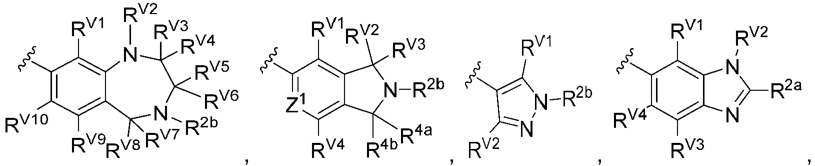 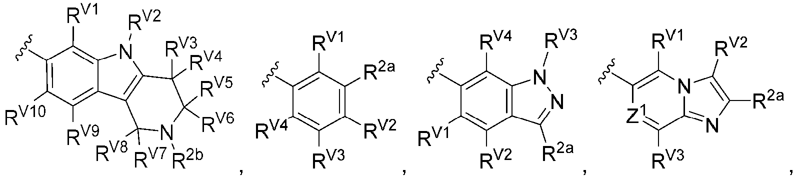 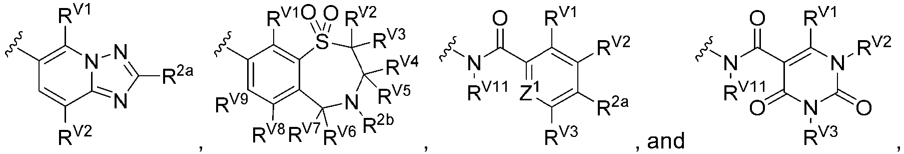 wherein each occurrence of Z¹ is CR^{V10} or N;
R^{2a} is selected from the group consisting of -C(=O)NR⁶R⁶, C(=O)OR^{I}, optionally substituted C₁-C₆ alkoxy, optionally substituted C₁-C₆ hydroxyalkyl, -(CH₂)₀₋₂NH(CH₂)₀₋₂(optionally substituted 5-oxopyrrolidin-2-yl), -(CH₂)₀₋₂N(C₁-C₆ alkyl)(CH₂)₀₋₂(optionally substituted 5-oxopyrrolidin-2-yl), -(CH₂)₀₋₂NH(CH₂)₀₋₂(C₁-C₆ alkyl), -(CH₂)₀₋₂N(C₁-C₆ alkyl)₂, - (CH₂)₀₋₂NH(CH₂)₀₋₂(C₁-C₆ haloalkyl), -(CH₂)₀₋₂N(C₁-C₆ alkyl)(C₁-C₆ haloalkyl), -(CH₂)₀₋₂NH(CH₂)₀₋₂(C₁-C₆ hydroxyalkyl), -(CH₂)₀₋₂N(C₁-C₆ alkyl)(CH₂)₀₋₂(C₁-C₆ hydroxyalkyl), -(CH₂)₀₋₂NH(CH₂)₀₋₂C(=O)O(C₁-C₆ alkyl), -(CH₂)₀₋₂N(C₁-C₆ alkyl)(CH₂)₀₋₂C(=O)O(C₁-C₆ alkyl), -(CH₂)₀₋₂NH(CH₂)₀₋₂N(C₁-C₆ alkyl)C(=O)(C₁-C₆ alkyl), -(CH₂)₀₋₂NH(CH₂)₀₋₂NHC(=O)(C₁-C₆ alkyl), - (CH₂)₀₋₂N(C₁-C₆ alkyl)(CH₂)₀₋₂N(C₁-C₆ alkyl)C(=O)(C₁-C₆ alkyl), -(CH₂)₀₋₂N(C₁-C₆ alkyl)(CH₂)₀₋₂NHC(=O)(C₁-C₆ alkyl), -(CH₂)₀₋₂NH(CH₂)₀₋₂(optionally substituted oxetanyl), -(CH₂)₀₋₂N(C₁-C₆ alkyl)(CH₂)₀₋₂(optionally substituted oxetanyl), -(CH₂)₀₋₂NH(CH₂)₀₋₂(optionally substituted oxolanyl), -(CH₂)₀₋₂N(C₁-C₆ alkyl)(CH₂)₀₋₂(optionally substituted oxolanyl), -(CH₂)₀₋₂NH(CH₂)₀₋₂(optionally substituted oxanyl), -(CH₂)₀₋₂N(C₁-C₆ alkyl)(CH₂)₀₋₂(optionally substituted oxanyl), -(CH₂)₀₋₂NH(CH₂)₀₋₂(optionally substituted cyclobutyl), -(CH₂)₀₋₂N(C₁-C₆ alkyl)(CH₂)₀₋₂(optionally substituted cyclobutyl), -(CH₂)₀₋₂NH(CH₂)₀₋₂(optionally substituted cyclohexyl), - (CH₂)₀₋₂N(C₁-C₆ alkyl)(CH₂)₀₋₂(optionally substituted cyclohexyl), -(CH₂)₀₋₂NH(CH₂)₀₋₂(optionally substituted C₅-C₆ cycloalkylsulfonyl), -(CH₂)₀₋₂N(C₁-C₆ alkyl)(CH₂)₀₋₂(optionally substituted C₅-C₆ cycloalkylsulfonyl), -(CH₂)₀₋₂NH(CH₂)₀₋₂(optionally substituted azetidinyl), - (CH₂)₀₋₂N(C₁-C₆ alkyl)(CH₂)₀₋₂(optionally substituted azetidinyl), -(CH₂)₀₋₂NH(CH₂)₀₋₂(optionally substituted pyrrolidinyl), -(CH₂)₀₋₂N(C₁-C₆ alkyl)(CH₂)₀₋₂(optionally substituted pyrrolidinyl), -(CH₂)₀₋₂NH(CH₂)₀₋₂(optionally substituted piperidinyl), -(CH₂)₀₋₂N(C₁-C₆ alkyl)(CH₂)₀₋₂(optionally substituted piperidinyl), -(CH₂)₀₋₂(optionally substituted guanidinyl), - (CH₂)₀₋₂NH(C(CH₂)₂₋₅)C(=O)O(C₁-C₆ alkyl), -(CH₂)₀₋₂N(C₁-C₆ alkyl)(C(CH₂)₂₋₅)C(=O)O(C₁-C₆ alkyl), -(CH₂)₀₋₂NH(CH(C₁-C₃ alkyl))C(=O)O(C₁-C₆ alkyl), -(CH₂)₀₋₂N(C₁-C₆ alkyl)(CH(C₁-C₃ alkyl))C(=O)O(Cₗ-C₆ alkyl), -(CH₂)₀₋₂NH(CH₂)₀₋₁(C₅-C₉ bridged cycloalkyl)(CH₂)₀₋₁C(=O)O(C₁-C₆ alkyl), -(CH₂)₀₋₂N(C₁-C₆ alkyl)(CH₂)₀₋₁(C₅-C₉ bridged cycloalkyl)(CH₂)₀₋₁C(=O)O(C₁-C₆ alkyl), -(CH₂)₀₋₂NH(CH₂)₀₋₁(CHOH)(CH₂)₀₋₁C(=O)O(C₁-C₆ alkyl), -(CH₂)₀₋₂NH(CH₂)₀₋₁(CHO(C₁-C₆ alkyl))(CH₂)₀₋₁C(=O)O(C₁-C₆ alkyl), -(CH₂)₀₋₂N(C₁-C₆ alkyl)(CH₂)₀₋₁(CHOH)(CH₂)₀₋₁C(=O)O(C₁-C₆ alkyl), -(CH₂)₀₋₂N(C₁-C₆ alkyl)(CH₂)₀₋₁(CHO(C₁-C₆ alkyl))(CH₂)₀₋₁C(=O)O(C₁-C₆ alkyl), -(CH₂)₀₋₂(C₄-C₁₀ bridged heterocycloalkyl), -(CH₂)₀₋₂(optionally substituted azetidinyl), -(CH₂)₀₋₂(optionally substituted pyrrolidinyl), -(CH₂)₀₋₂(optionally substituted piperidinyl), -(CH₂)₀₋₂(optionally substituted morpholinyl), -(CH₂)₀₋₂(optionally substituted piperazinyl), -(CH₂)₀₋₂(optionally substituted piperazin-2-onyl), -C(CH₃)N(C₁-C₆ alkyl)₂, - (CH₂)₀₋₂N(C₁-C₆ alkyl)(CH₂)₀₋₂(CHF₂), -(CH₂)₀₋₂NH(CH₂)₀₋₂(CHF₂), -(CH₂)₀₋₂N(C₁-C₆ alkyl)(CH₂)₀₋₄(CH₂F), -(CH₂)₀₋₂NH(CH₂)₀₋₄(CH₂F), -(CH₂)₀₋₂NH(CH₂)₀₋₂N(C₁-C₆ alkyl)C(=O)CH₃, -(CH₂)₀₋₂NH(CH₂)₀₋₂NHC(=O)CH₃, -(CH₂)₀₋₂N(C₁-C₆ alkyl)(CH₂)₀₋₂N(C₁-C₆ alkyl)C(=O)CH₃, -(CH₂)₀₋₂N(C₁-C₆ alkyl)(CH₂)₀₋₂NHC(=O)CH₃, -(CH₂)₀₋₂N(C₁-C₆ alkyl)C((C(=O)OC₁-C₆ alkyl)((CH₂)₀₋₂O(C₁-C₆ alkyl))), -(CH₂)₀₋₂NHC((C(=O)OC₁-C₆ alkyl)((CH₂)₀₋₂O(C₁-C₆ alkyl))), -(CH₂)₀₋₂N(C₁-C₆ alkyl)C((C(=O)OH((CH₂)₀₋₂O(C₁-C₆ alkyl))), - (CH₂)₀₋₂N(C₁-C₆ alkyl)C((C(=O)OC₁-C₆ alkyl)((CH₂)₀₋₂OH)), -(CH₂)₀₋₂NHC((C(=O)OH)((CH₂)₀₋₂O(C₁-C₆ alkyl))), -(CH₂)₀₋₂N(C₁-C₆ alkyl)C((C(=O)OH((CH₂)₀₋₂OH)), -(CH₂)₀₋₂NHC((C(=O)OC₁-C₆ alkyl)((CH₂)₀₋₂OH)), -(CH₂)₀₋₂NHC((C(=O)OH((CH₂)₀₋₂OH)), -(CH₂)₀₋₂N(C₁-C₆ alkyl)(CH₂)₁₋₄N(C₁-C₆ alkyl)C(=O)Me, -(CH₂)₀₋₂N(C₁-C₆ alkyl)(CH₂)₁₋₄NHC(=O)Me, - (CH₂)₀₋₂NH(CH₂)₁₋₄N(C₁-C₆ alkyl)C(=O)Me, -(CH₂)₀₋₂NH(CH₂)₁₋₄NHC(=O)Me, -(CH₂)₀₋₂N(C₁-C₆ alkyl)(CH₂)₁₋₅C(=O)N(C₁-C₆ alkyl)(C₁-C₆ alkyl), -(CH₂)₀₋₂NH(CH₂)₁₋₅C(=O)N(C₁-C₆ alkyl)(C₁-C₆ alkyl), -(CH₂)₀₋₂N(C₁-C₆ alkyl)(CH₂)₁₋₅C(=O)NH(C₁-C₆ alkyl), -(CH₂)₀₋₂N(C₁-C₆ alkyl)(CH₂)₁₋₅C(=O)NH₂, -(CH₂)₀₋₂NH(CH₂)₁₋₅C(=O)NH(C₁-C₆ alkyl), -(CH₂)₀₋₂NH(CH₂)₁₋₅C(=O)NH₂, - (CH₂)₀₋₂N(C₁-C₆ alkyl)(CH₂)₀₋₂(optionally substituted cyclopropyl), -(CH₂)₀₋₂NH(CH₂)₀₋₂(optionally substituted cyclopropyl), -(CH₂)₀₋₂N(C₁-C₆ alkyl)(CH₂)₀₋₂(optionally substituted butanolid-2-yl), -(CH₂)₀₋₂NH(CH₂)₀₋₂(optionally substituted butanolide-2-yl), -(CH₂)₀₋₂N(C₁-C₆ alkyl)(CH₂)₀₋₂(optionally substituted tetrahydro-4-thiopyranyl), -(CH₂)₀₋₂NH(CH₂)₀₋₂(optionally substituted oxazolyl), -(CH₂)₀₋₂NH(CH₂)₀₋₂(optionally substituted tetrahydro-4-thiopyranyl), - CH(CH₃)NH(CH₂)₀₋₂(optionally substituted piperidinyl), -CH(CH₃)N(C₁-C₆ alkyl)(CH₂)₀₋₂(optionally substituted piperidinyl), -(CH₂)₀₋₂N(C₁-C₆ alkyl)(CH₂)₀₋₂(optionally substituted piperazinyl), and -(CH₂)₀₋₂NH(CH₂)₀₋₂(optionally substituted piperazinyl),
wherein each optional substituent in R^{2a} is independently selected from the group consisting of halogen, OH, NH₂, C(=O)OH, C(=O)O(C₁-C₆ alkyl), C(=O)NH₂, C(=O)NH(C₁-C₆ alkyl), C(=O)N(C₁-C₆ alkyl)₂, optionally substituted heterocyclyl, optionally substituted C₁-C₆ alkyl, optionally substituted C₁-C₆ hydroxyalkyl, optionally substituted cycloalkyl, C(=O)(optionally substituted C₁-C₆ alkyl), and optionally substituted -(CH₂)₁₋₂(heterocyclyl),
wherein two optional substituents in R^{2a} may combine with the atoms to which they are bound to form an optionally substituted C₂-C₈ heterocyclyl or optionally substituted C₃-C₈ cycloalkyl,
wherein each occurrence of R^{I} is independently H, C₁-C₆ alkyl, or C₃-C₈ cycloalkyl, wherein the alkyl or cycloalkyl is independently optionally substituted with at least one of halogen, -OH, C₁-C₆ alkoxy, -NH₂, -NH(C₁-C₆ alkyl), and -N(C₁-C₆ alkyl)(C₁-C₆ alkyl),
wherein each occurrence of R⁶ is independently H, C₁-C₆ alkyl, or C₃-C₈ cycloalkyl, wherein the alkyl or cycloalkyl is independently optionally substituted with at least one of halogen, -OH, C₁-C₆ alkoxy, -NH₂, -NH(C₁-C₆ alkyl), and -N(C₁-C₆ alkyl)(C₁-C₆ alkyl),
R^{2b} is selected from the group consisting of H, -(CH₂)₁₋₃C(=O)OR', -(CH₂)₁₋₃C(=O)NR^{I}R^{I}, optionally substituted C₁-C₆ alkyl, optionally substituted C₁-C₆ alkoxy, optionally substituted C₁-C₆ hydroxyalkyl, -(CH₂)₀₋₂(C₁-C₆ hydroxyalkyl), -(CH₂)₀₋₂CH(OC₁-C₆ alkyl)CH₂F, -(CH₂)₀₋₂CH(OH)CH₂F, -(CH₂)₀₋₂(optionally substituted 5-oxopyrrolidin-2-yl), -(CH₂)₀₋₂(optionally substituted piperidinyl), -(CH₂)₀₋₂(optionally substituted azetidinyl), -(CH₂)₀₋₂(optionally substituted cyclopropyl), -(CH₂)₀₋₂(optionally substituted cyclobutyl), -(CH₂)₀₋₂N(C₁-C₆ alkyl)CH₃; -(CH₂)₀₋₂NHCH₃, -(CH₂)₀₋₂N(C₁-C₆ alkyl)(CH₂)₀₋₂C(=O)O(C₁-C₆ alkyl), -(CH₂)₀₋₂NH(CH₂)₀₋₂C(=O)O(C₁-C₆ alkyl), -(CH₂)₀₋₂N(C₁-C₆ alkyl)(CH₂)₀₋₂C(=O)OH, -(CH₂)₀₋₂NH(CH₂)₀₋₂C(=O)OH, -(CH₂)₀₋₂N(C₁-C₆ alkyl)(CH₂)₀₋₂(optionally substituted 5-oxopyrrolidin-2-yl), and - (CH₂)₀₋₂NH(CH₂)₀₋₂(optionally substituted 5-oxopyrrolidin-2-yl),
wherein each optional substituent in R^{2b} is independently selected from the group consisting of optionally substituted C₁-C₆ alkyl, optionally substituted heterocyclyl, optionally substituted cycloalkyl, and optionally substituted -(CH₂)₁₋₃(heterocyclyl);
R^{3a}, R^{3b}, R^{3c}, R^{3d}, R^{3e}, R^{3f}, R^{3g}, R^{3h}, R³ⁱ, and R^{3j} are each independently selected from the group consisting of H, halogen, C₁-C₃ alkyl, C₃-C₈ cycloalkyl, C₁-C₃ alkoxy, C₁-C₃ haloalkyl, and C₁-C₃ haloalkoxy;
R⁵ is selected from the group consisting of H, C₁-C₃ alkyl, C₃-C₈ cycloalkyl, C₁-C₃ alkoxy, C₁-C₃ haloalkyl, and C₁-C₃ haloalkoxy,
wherein R⁵ and R^{2b} may combine with the nitrogen atom to which they are bound to form a C₃-C₁₂ heterocyclyl;
each occurrence of R^{4a} and R^{4b} is independently selected from the group consisting of H, C₁-C₃ alkyl, C₃-C₈ cycloalkyl, C₁-C₃ alkoxy, C₁-C₃ haloalkyl, and C₁-C₃ haloalkoxy,
wherein R^{4a} and R^{4b} may combine with the carbon atom to which they are bound to form a carbonyl (C=O);
each occurrence of R^{V1}, R^{V2}, R^{V3}, R^{V4}, R^{V5,} R^{V6}, R^{V7}, R^{V8}, R^{V9}, and R^{V10} is independently selected from the group consisting of H, C₁-C₃ alkyl, C₃-C₈ cycloalkyl, C₁-C₃ alkoxy, cyano, halogen, C₁-C₃ haloalkyl, C₁-C₃ haloalkoxy, and C(=O)OR^{I};
R^{V11} is selected from the group consisting of H, C₁-C₃ alkyl, C₃-C₈ cycloalkyl, C₁-C₃ alkoxy, C₁-C₃ haloalkyl, and C₁-C₃ haloalkoxy;
wherein if R^{1c} and R^{1d} are identical, then at least one of the following applies:
(a) if X¹ is N, then Z¹ in R^{1c}, if present, is CR^{V10}, and;
(b) if X⁶ is N, then Z¹ in R^{1d}, if present, is CR^{V10}.

2. The compound of claim 1, which is selected from the group consisting of:

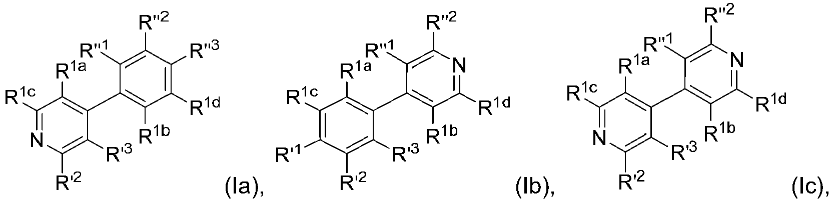

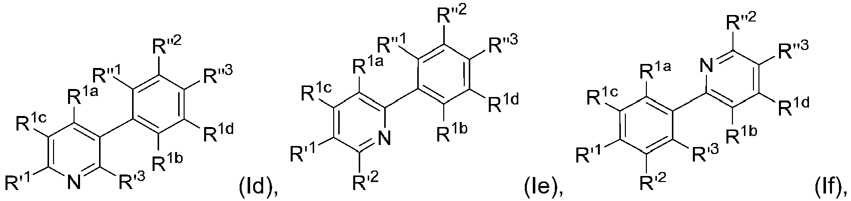

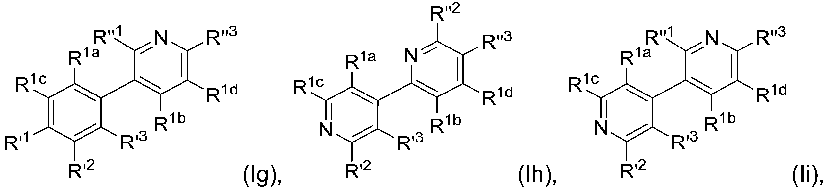

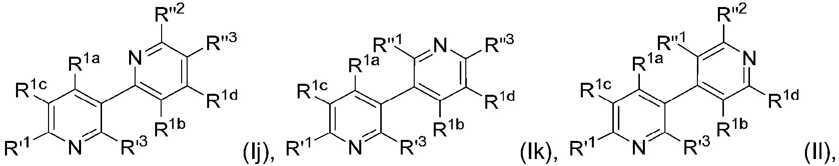

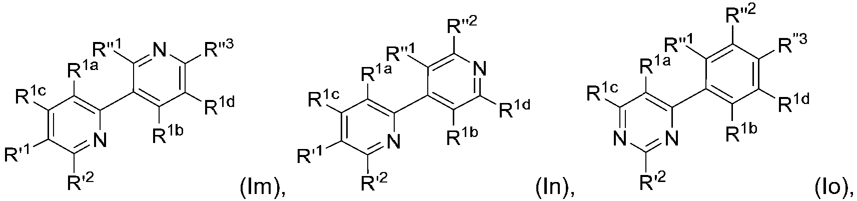

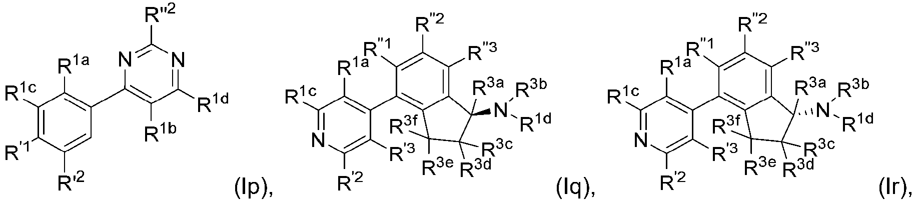

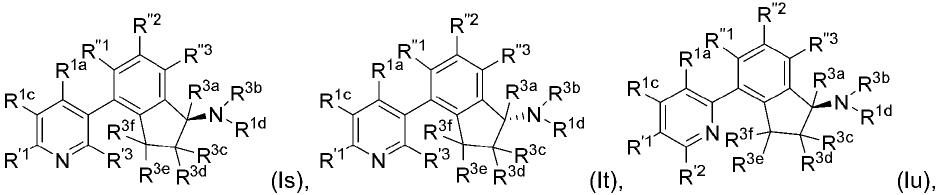

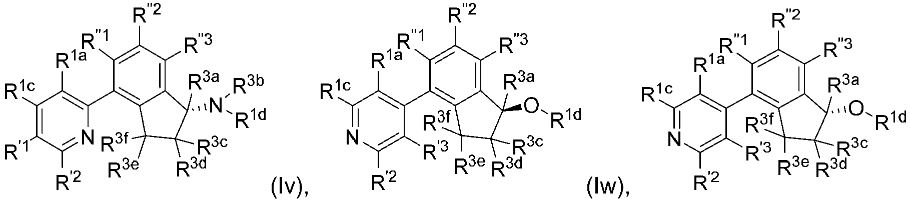

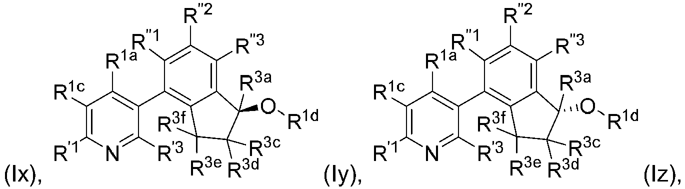

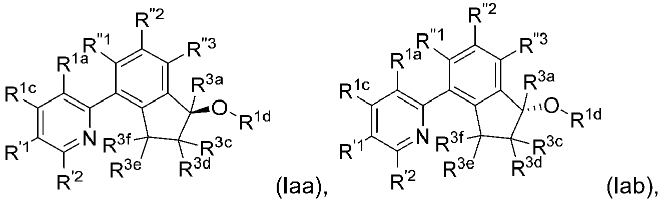

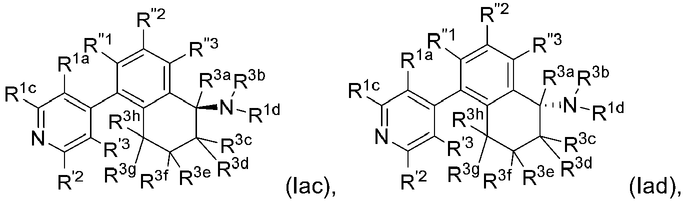

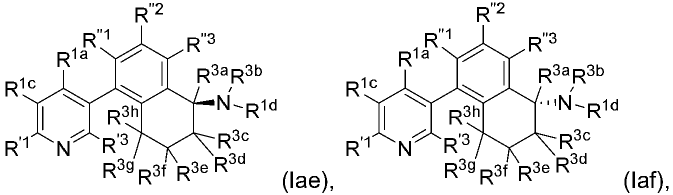

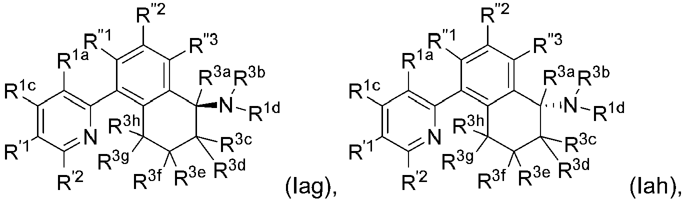

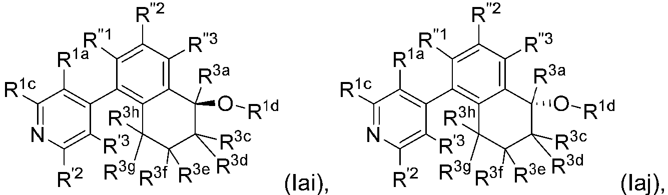

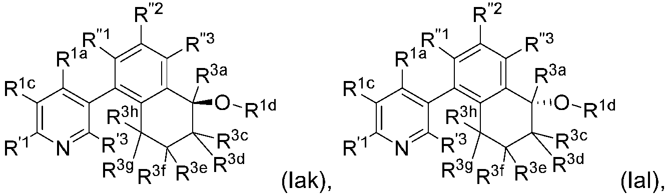

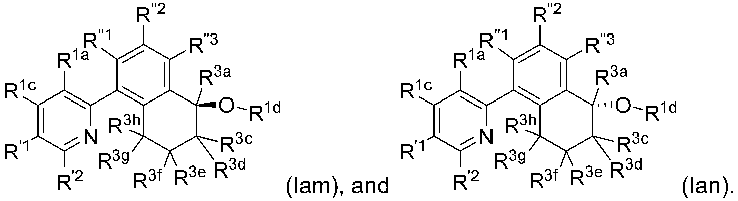

3. The compound of any one of claims 1-2, wherein one of the following applies:
(a) each of R^{'2}, R^{'3}, R^{"1}, R^{"2}, and R^{"3} are H, and X¹ is N;
(b) each of R^{'1}, R^{'3}, R^{"1}, R^{"2}, and R^{"3} are H, and X² is N;
(c) each of R^{'1}, R^{'2}, R^{"1}, R^{"2}, and R^{"3} are H, and X³ is N;
(d) each of R^{'1}, R^{'2}, R^{'3}, R^{"2}, and R^{"3} are H, and X⁴ is N;
(e) each of R^{'1}, R^{'2}, R^{'3}, R^{"1}, and R^{"3} are H, and X⁵ is N;
(f) each of R^{'1}, R^{'2}, R^{'3}, R^{"1}, and R^{"2} are H, and X⁶ is N;
(g) each of R^{'2}, R^{'3}, R^{"2}, and R^{"3} are H, and X¹ and X⁴ are N;
(h) each of R^{'2}, R^{'3}, R^{"1}, and R^{"3} are H, and X¹ and X⁵ are N;
(i) each of R^{'2}, R^{'3}, R^{"1}, and R^{"2} are H, and X¹ and X⁶ are N;
(j) each of R^{'1}, R^{'3}, R^{"2}, and R^{"3} are H, and X² and X⁴ are N;
(k) each of R^{'1}, R^{'3}, R^{"1}, and R^{"3} are H, and X² and X⁵ are N;
(l) each of R^{'1}, R^{'3}, R^{"1}, and R^{"2} are H, and X² and X⁶ are N;
(m) each of R^{'1}, R^{'2}, R^{"1}, and R^{"3} are H, and X³ and X⁵ are N;
(n) each of R^{'1}, R^{'2}, R^{"1}, and R^{"2} are H, and X³ and X⁶ are N;
(o) each of R^{'2}, R^{"1}, R^{"2}, and R^{"3} are H, and X¹ and X³ are N; and
(p) each of R^{'1}, R^{'2}, R^{'3}, and R^{"2} are H, and X⁴ and X⁶ are N.

4. The compound of any one of claims 1-3, wherein one of the following applies:
(a) L is a bond and each of R^{3a}, R^{3c}, R^{3d}, R^{3e}, and R^{3f} is H;
(b) L is a bond, Y is NR^{3b}, and each of R^{3a}, R^{3b}, R^{3c}, R^{3d}, R^{3e}, and R^{3f} is H;
(c) L is -C(R^{3g})(R^{3h})- and each of R^{3a}, R^{3c}, R^{3d}, R^{3e}, R^{3f}, R^{3g}, and R^{3h} is H;
(d) L is -C(R^{3g})(R^{3h})-, Y is NR^{3b}, and each of R^{3a}, R^{3b}, R^{3c}, R^{3d}, R^{3e}, R^{3f}, R^{3g}, and R^{3h} is H;
(e) L is -(C(R^{3g})(R^{3h}))(C(R³ⁱ)(R^{3j}))- and each of R^{3a}, R^{3c}, R^{3d}, R^{3e}, R^{3f}, R^{3g}, R^{3h}, R³ⁱ, and R^{3j} is H; and
(f) L is -C(R^{3g})(R^{3h})-, Y is NR^{3b}, and each of R^{3a}, R^{3b}, R^{3c}, R^{3d}, R^{3e}, R^{3f}, R^{3g}, R^{3h}, R³ⁱ, and R^{3j} are H.

5. The compound of any one of claims 1-4, wherein each of of R^{1a} and R^{1b} is independently selected from the group consisting of H, F, Cl, Me, CF₃, and CN.

6. The compound of any one of claims 1-5, wherein R^{1a} and R^{1b} are identical.

7. The compound of any one of claims 1-6, wherein R^{1c} and R^{1d} are independently selected from the group consisting of:

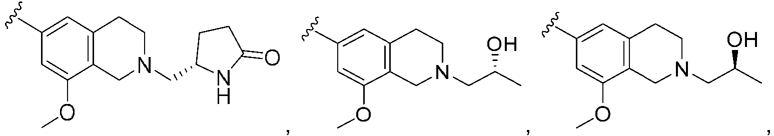

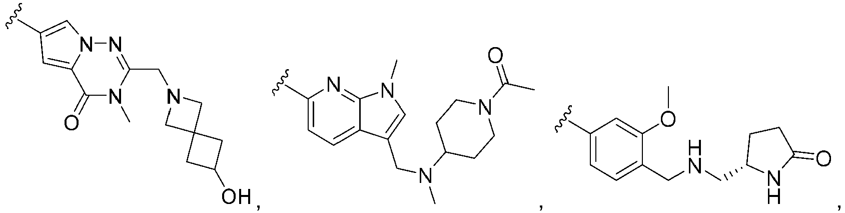

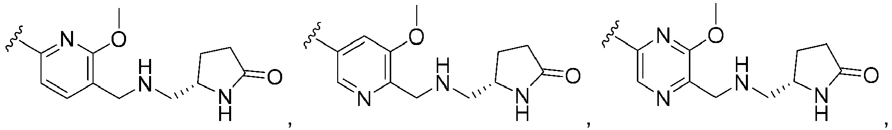

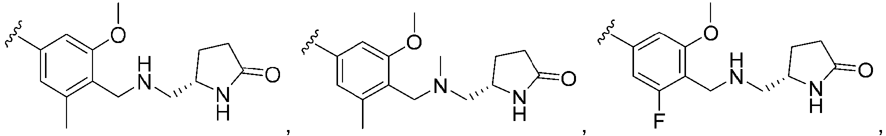

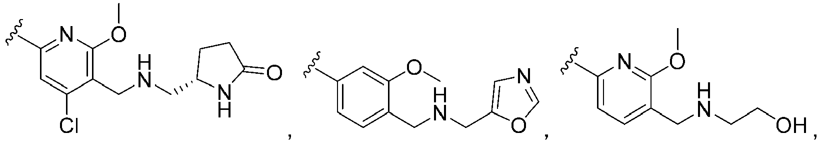

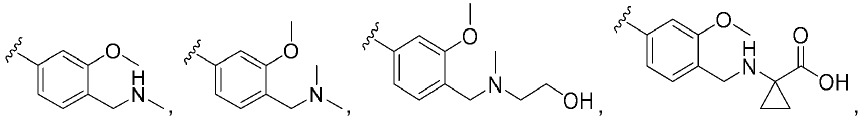

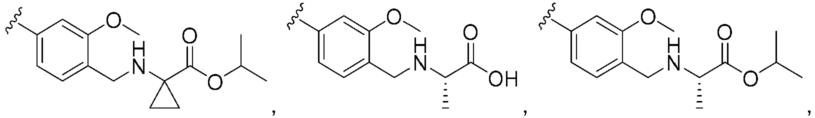

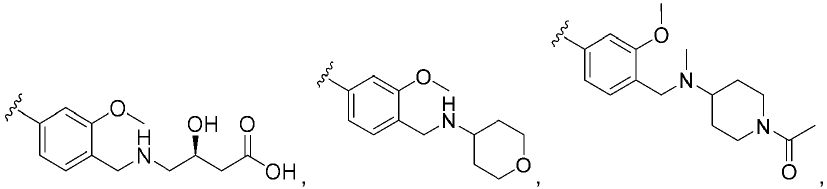

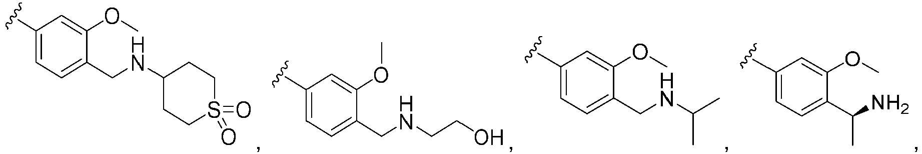

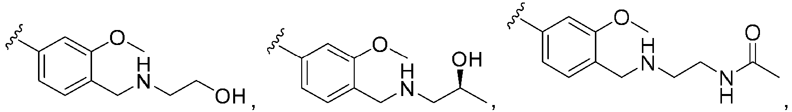

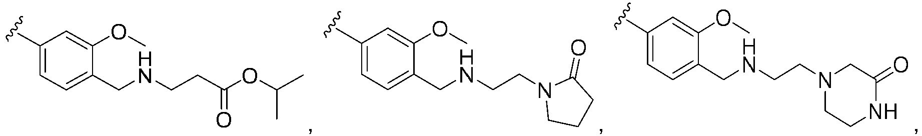

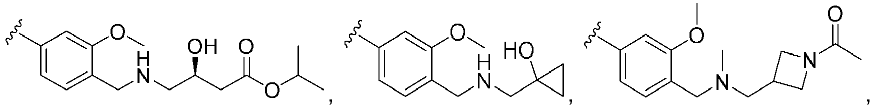

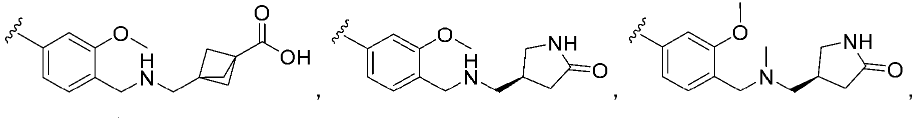

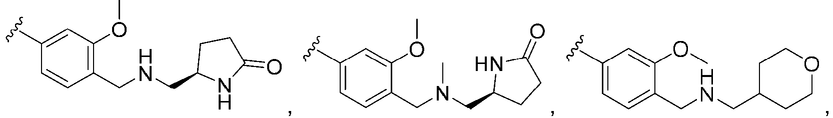

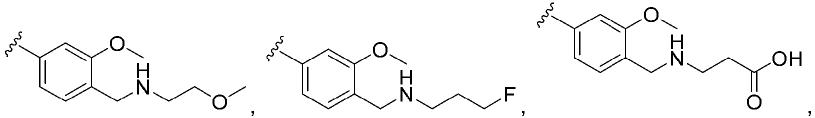

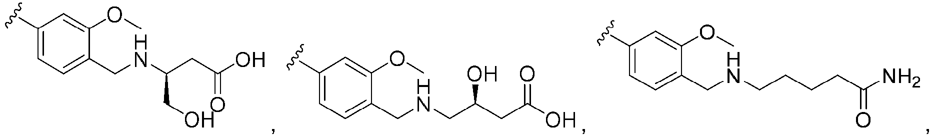

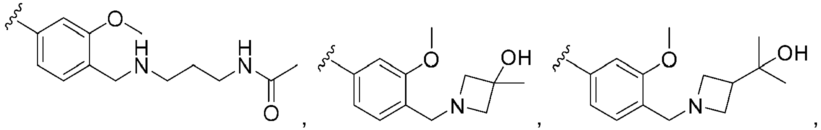

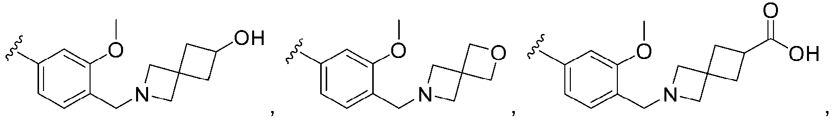

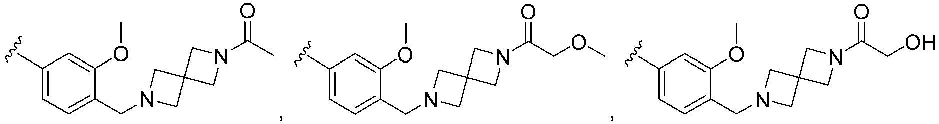

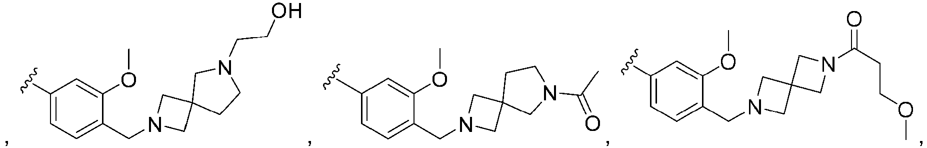

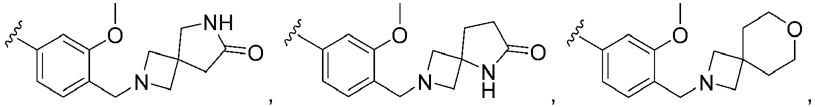

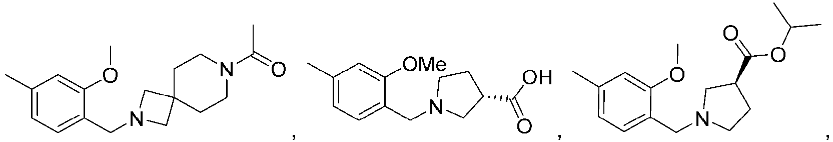

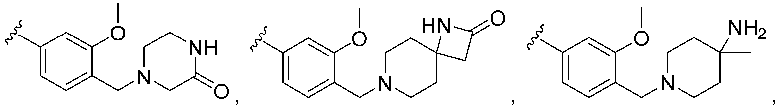

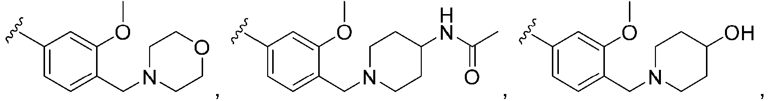

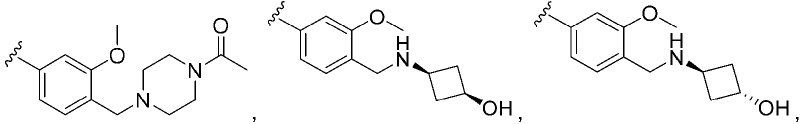

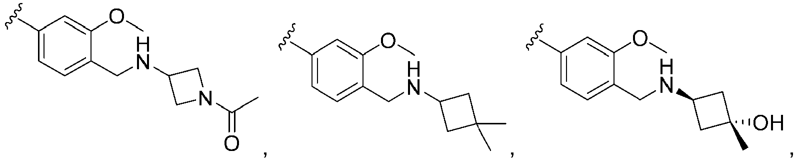

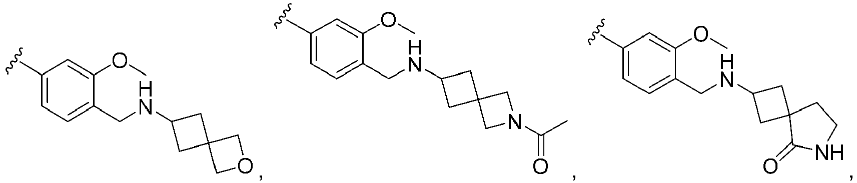

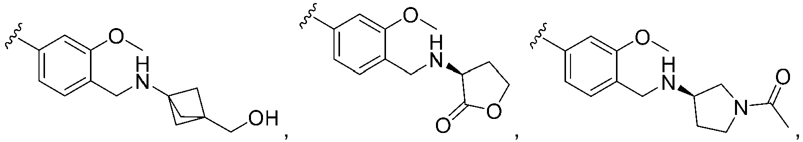

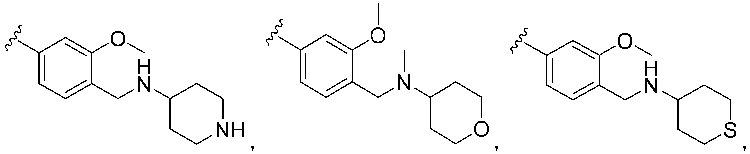

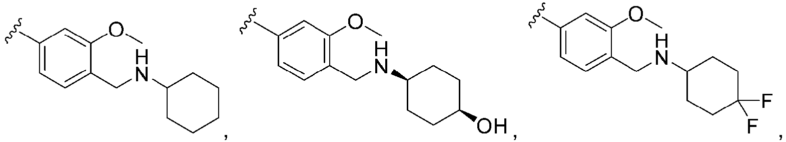

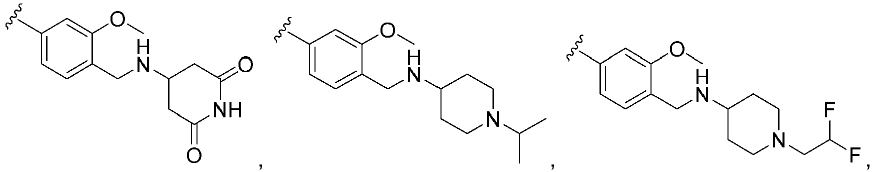

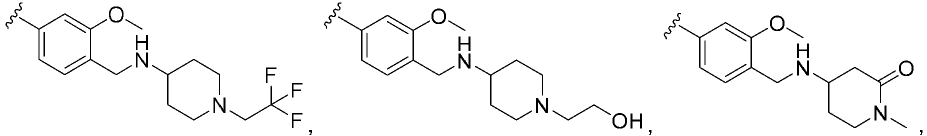

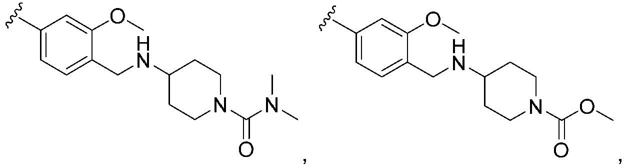

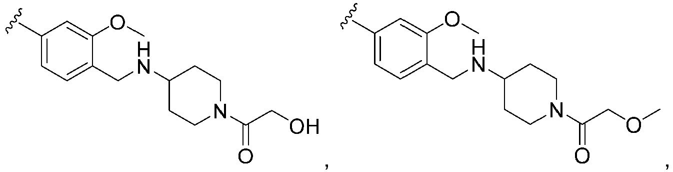

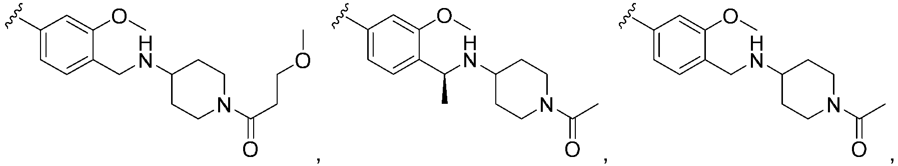

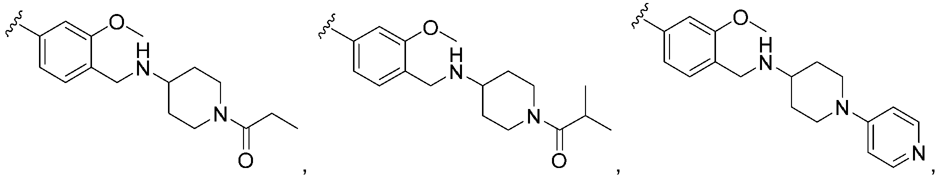

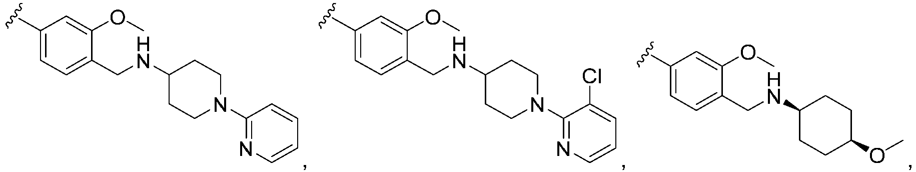

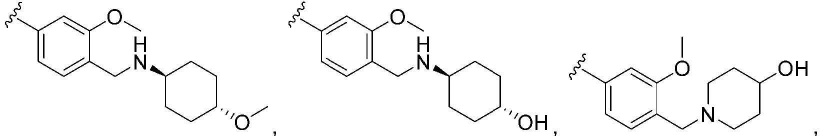

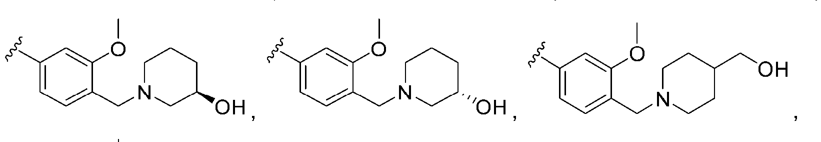

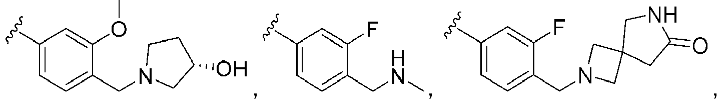

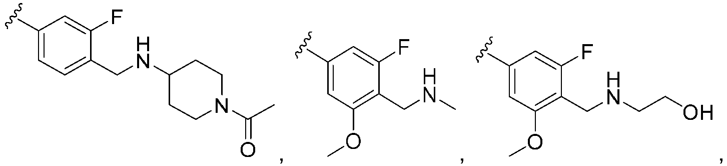

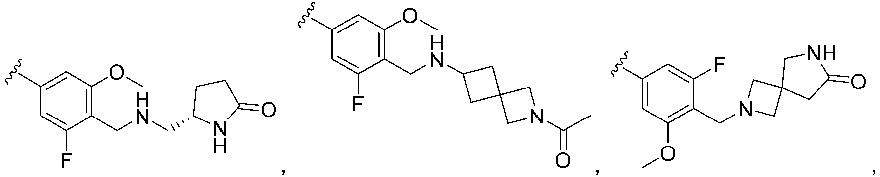

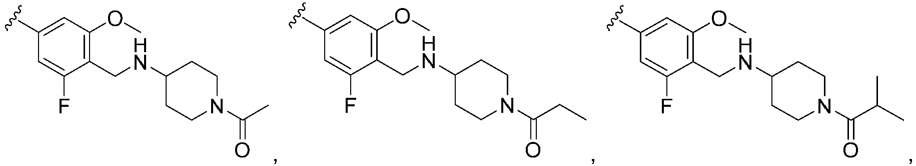

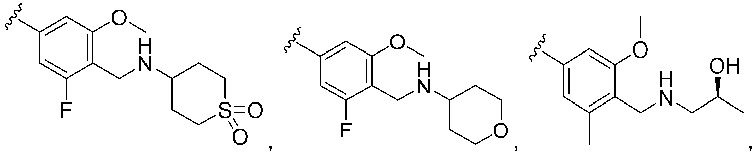

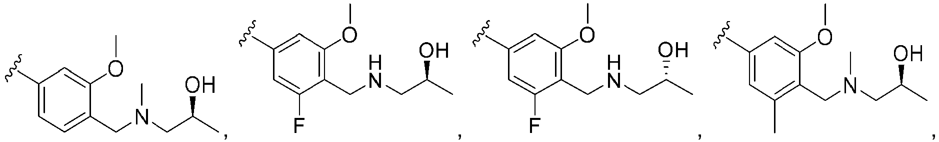

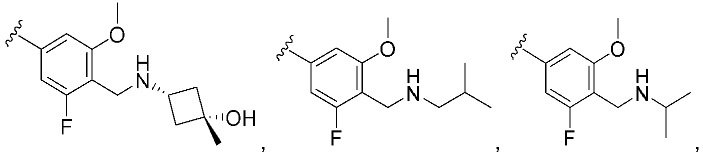

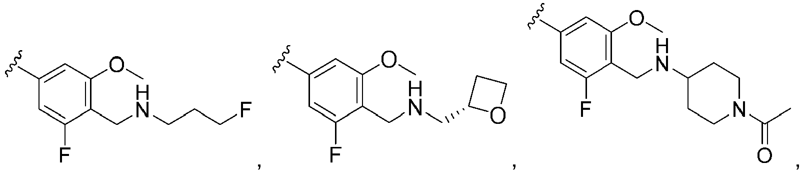

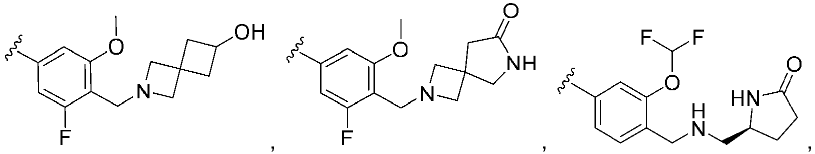

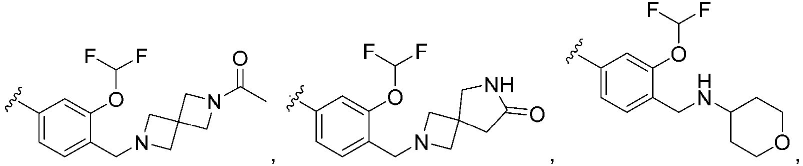

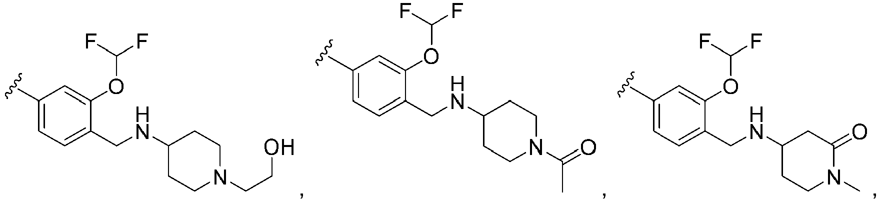

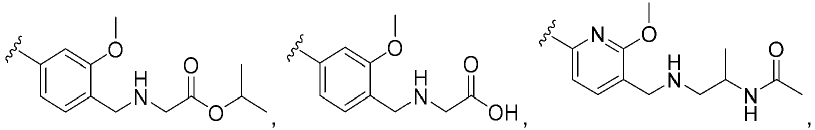

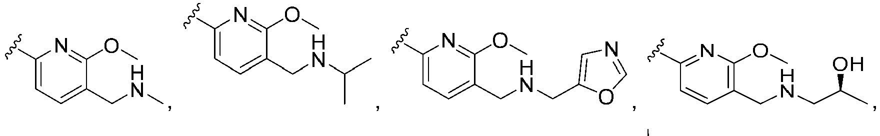

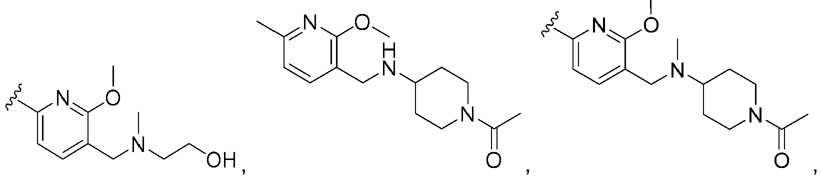

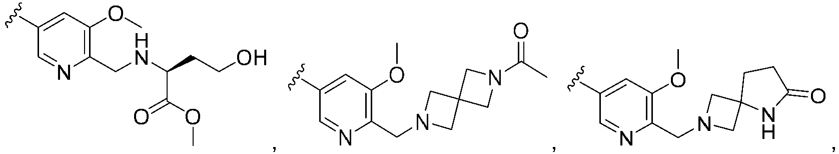

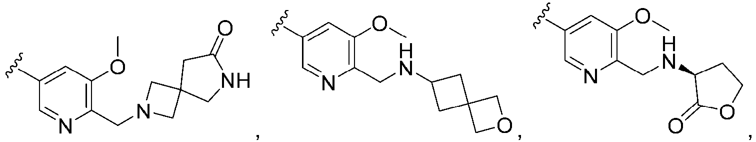

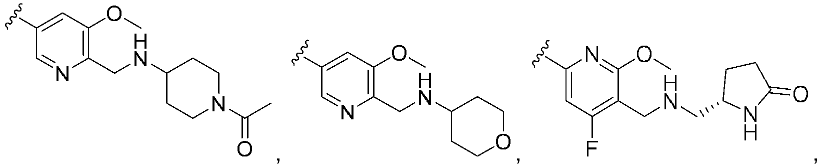

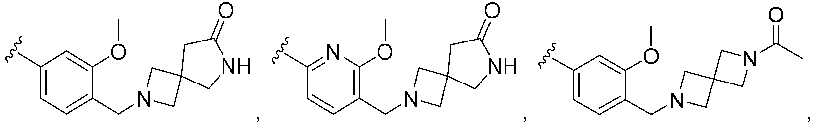

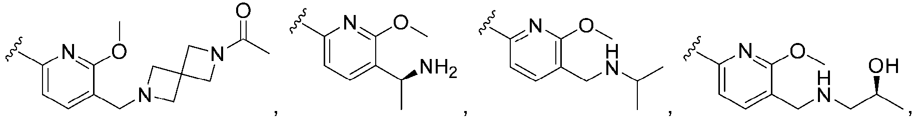

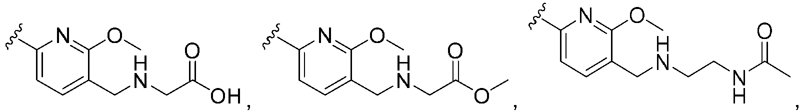

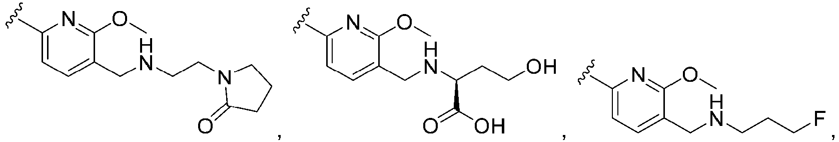

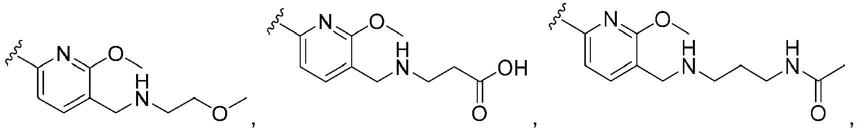

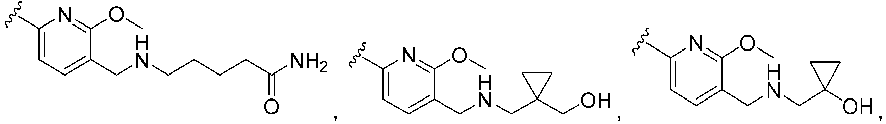

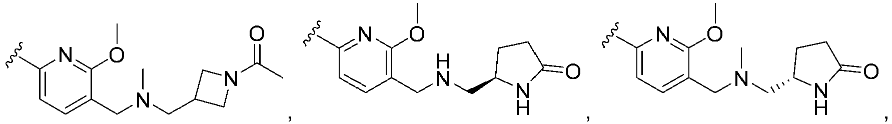

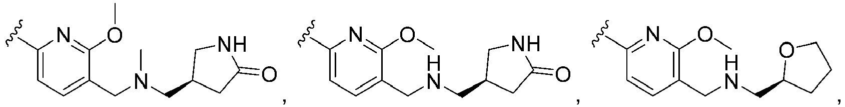

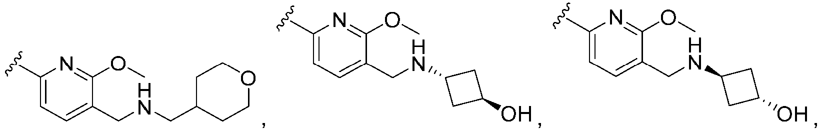

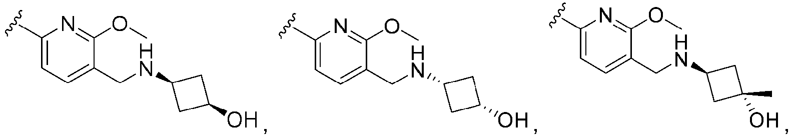

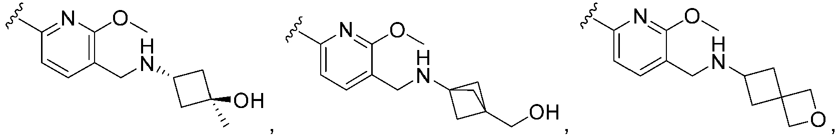

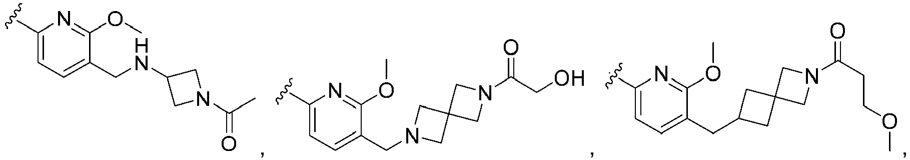

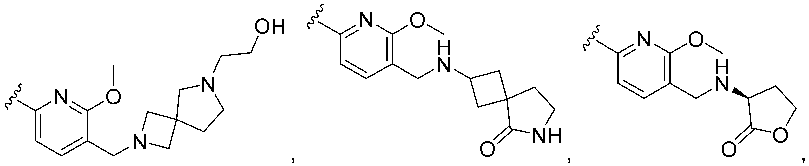

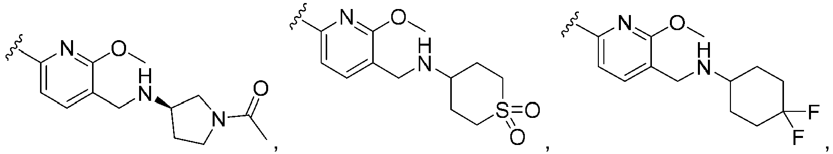

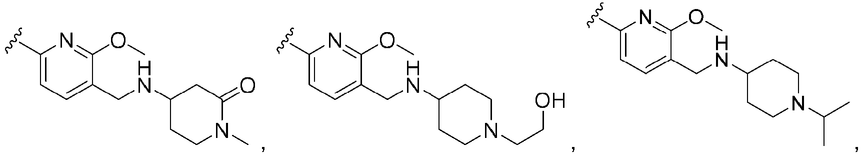

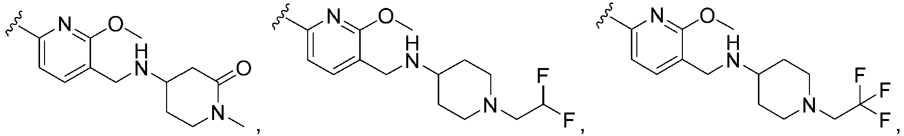

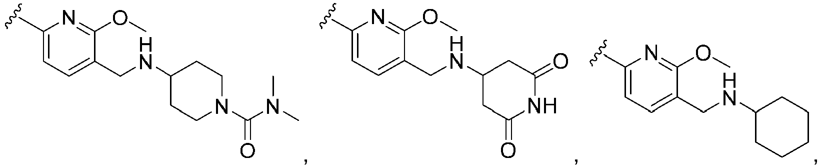

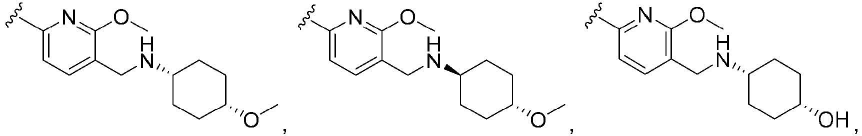

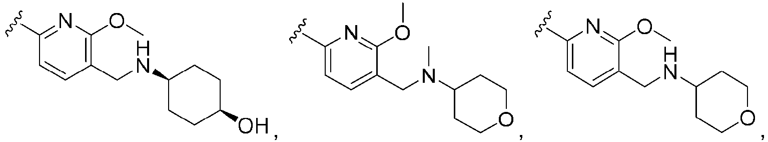

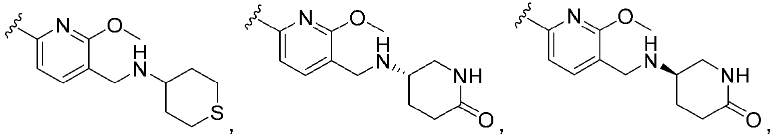

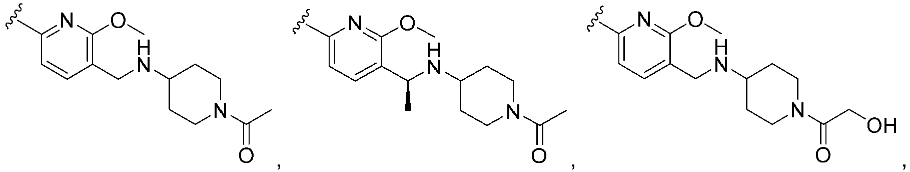

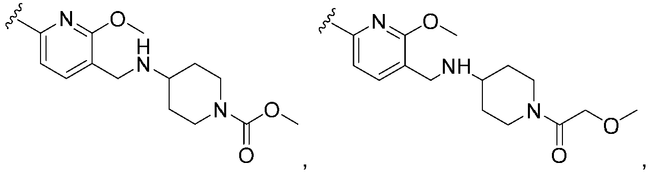

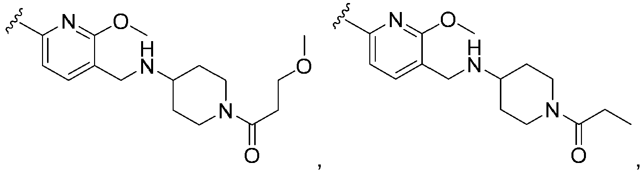

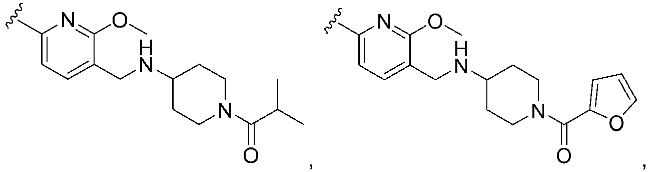

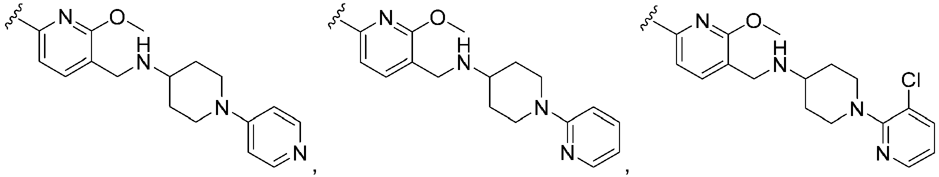

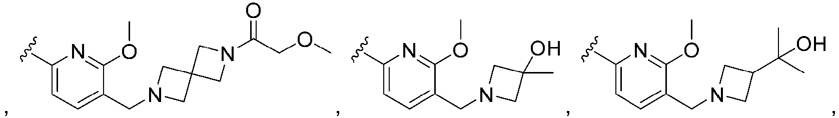

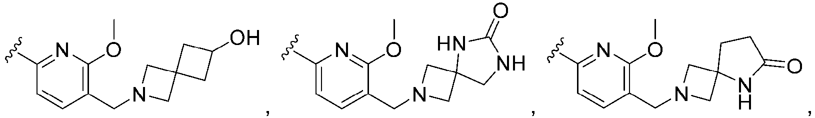

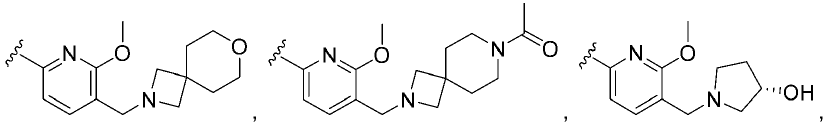

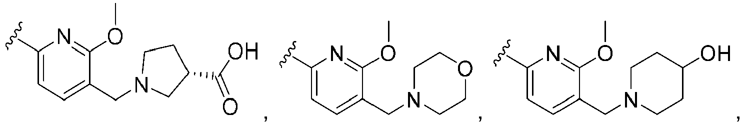

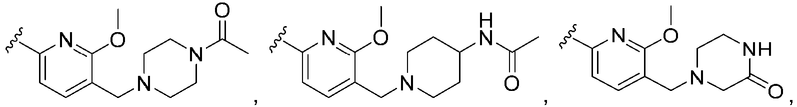

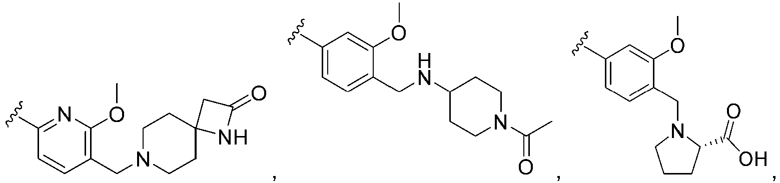

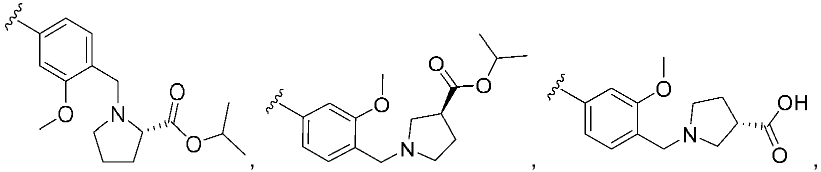

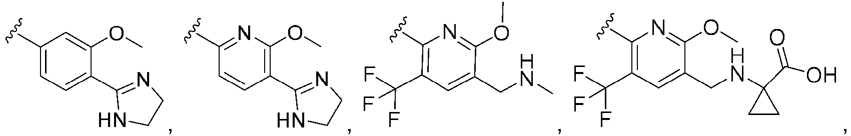

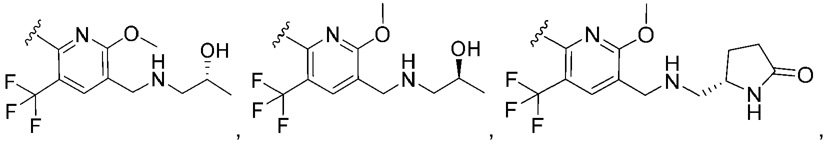

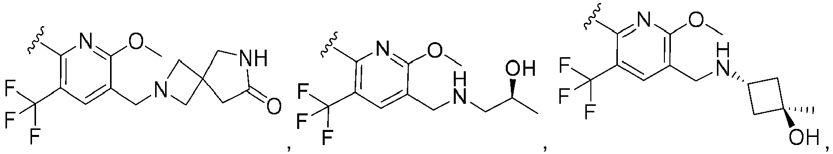

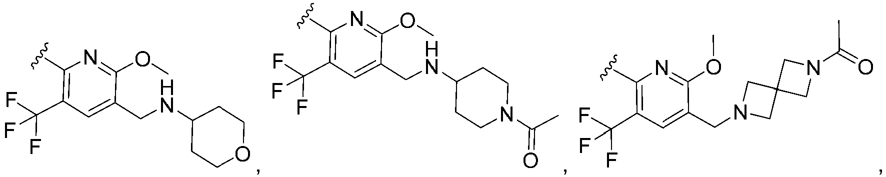

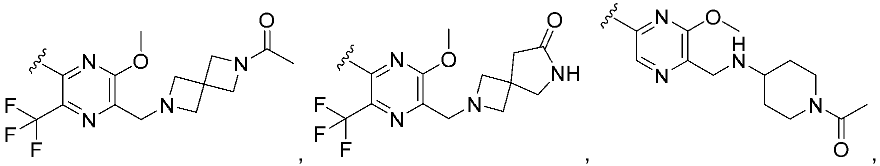

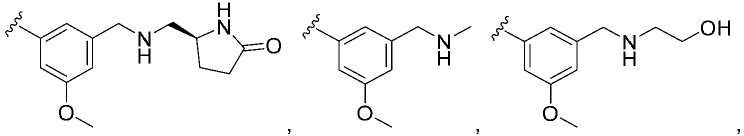

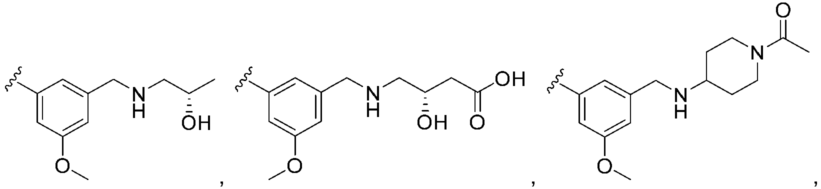

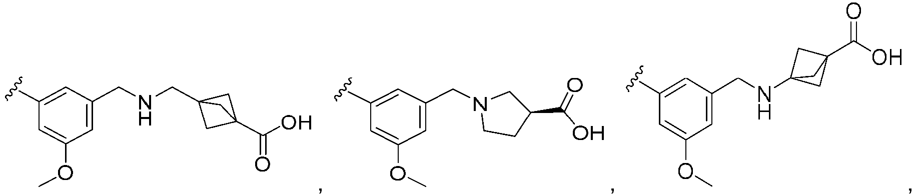

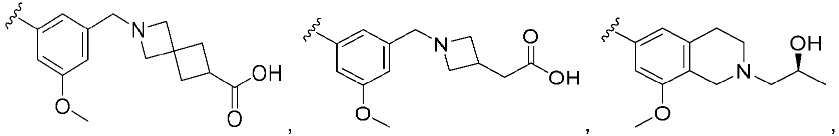

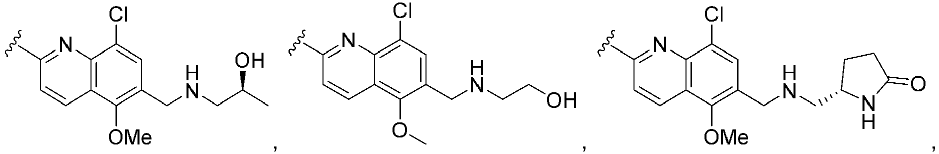

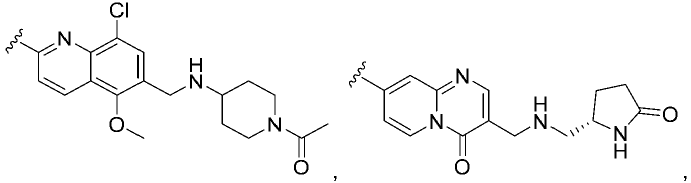

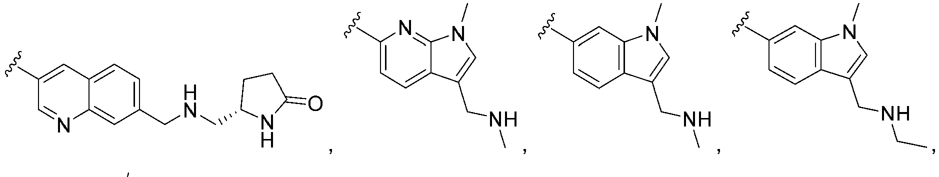

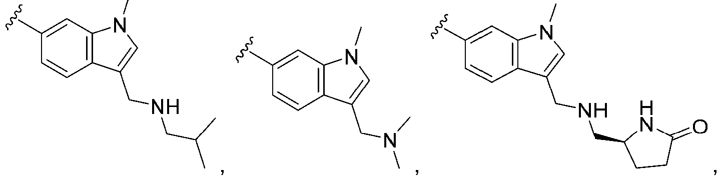

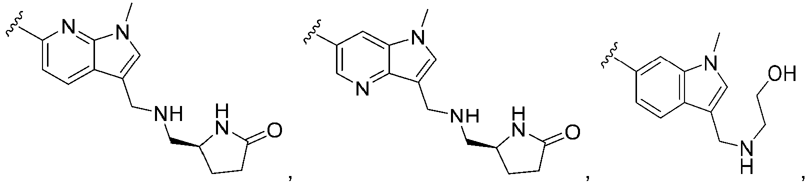

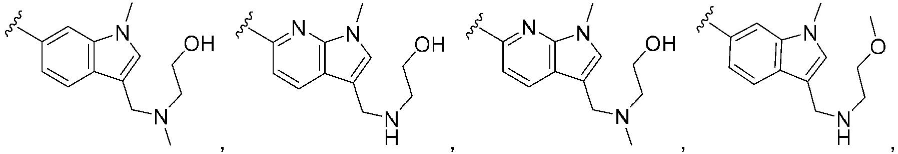

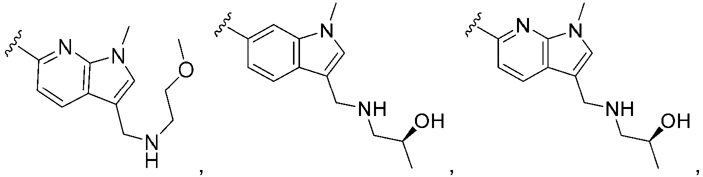

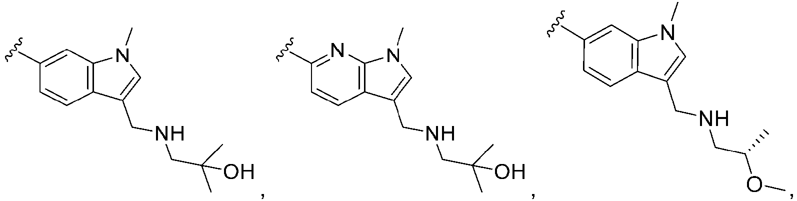

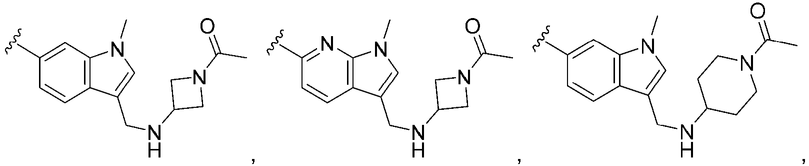

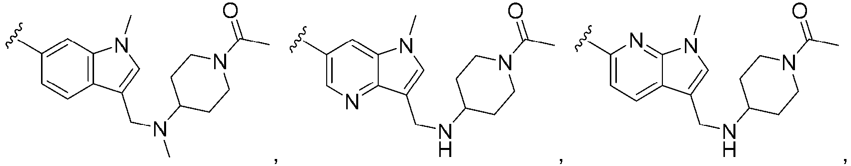

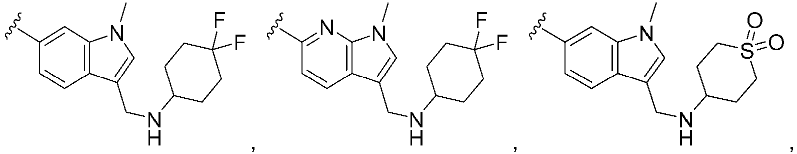

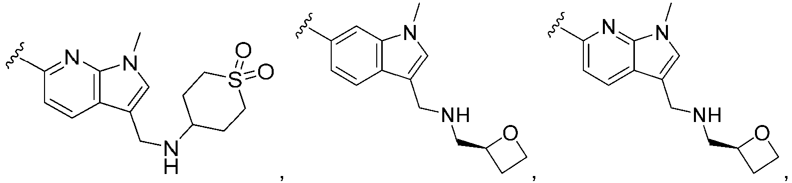

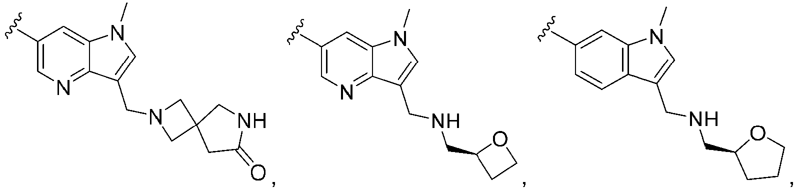

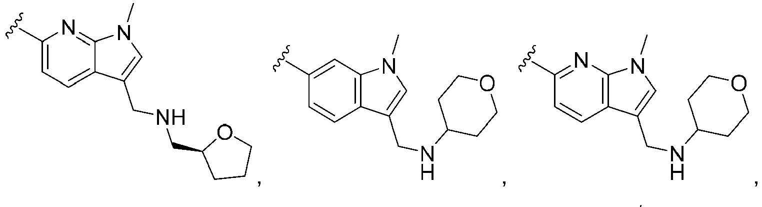

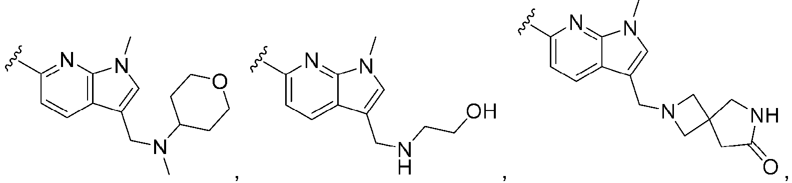

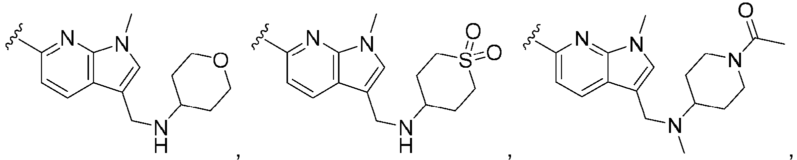

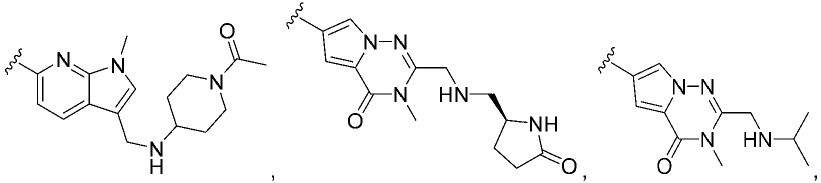

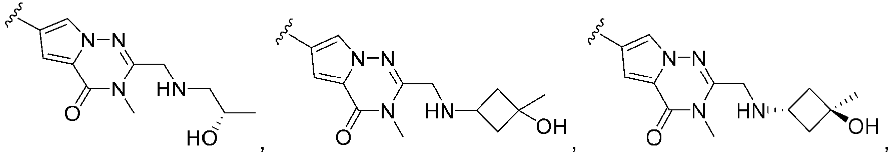

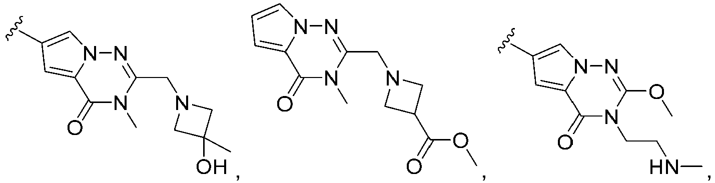

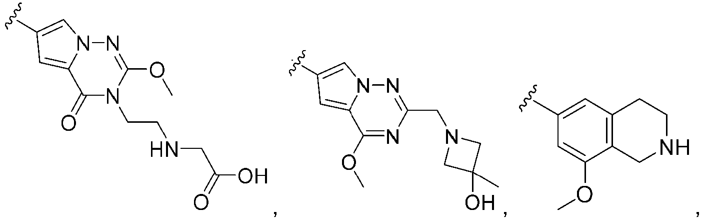

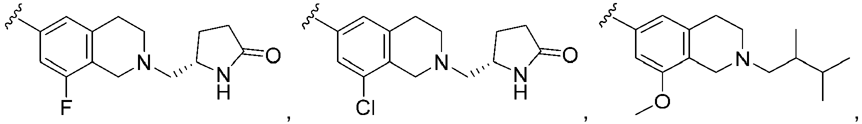

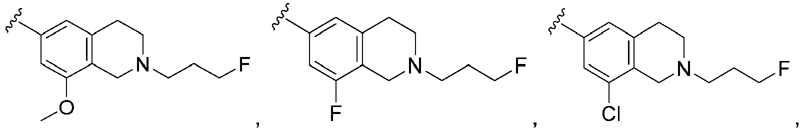

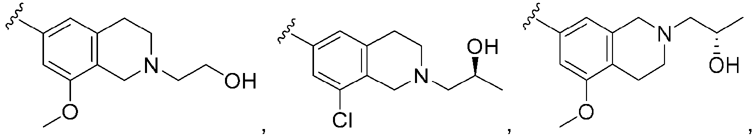

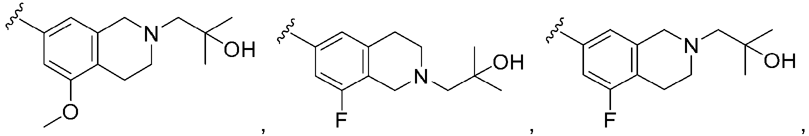

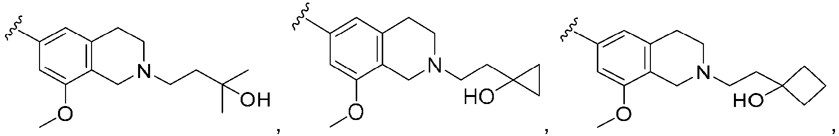

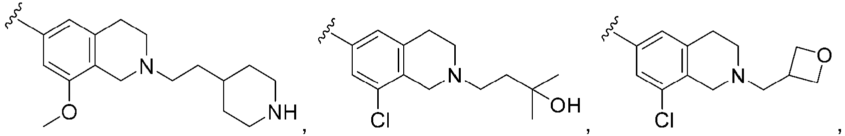

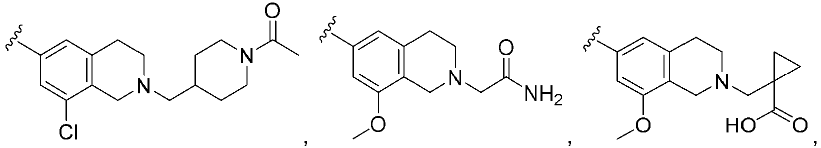

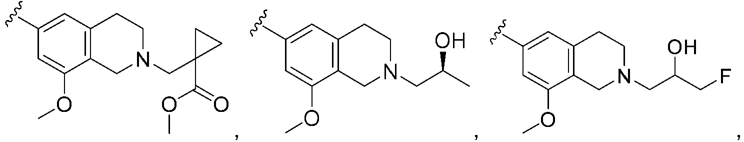

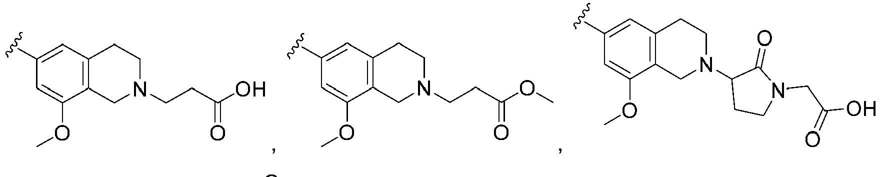

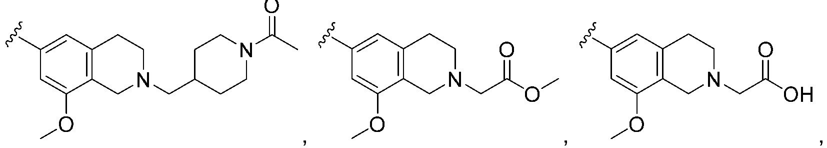

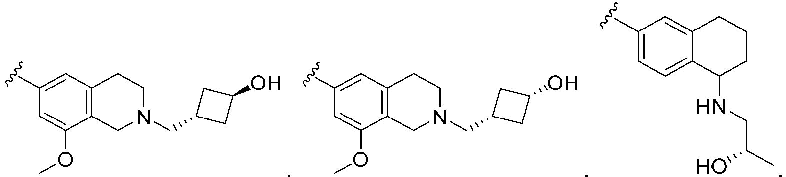

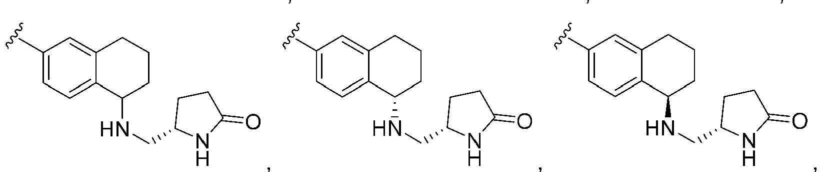

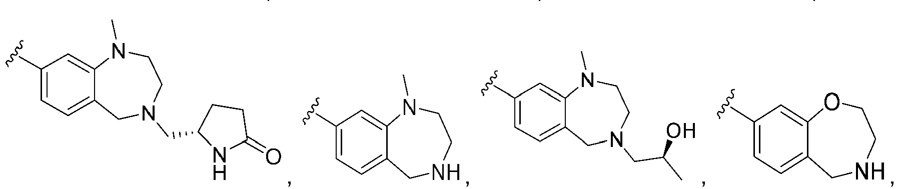

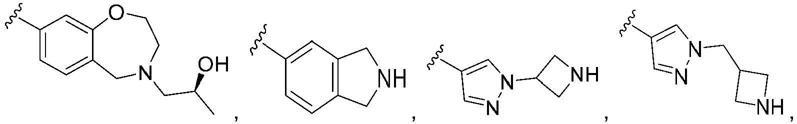

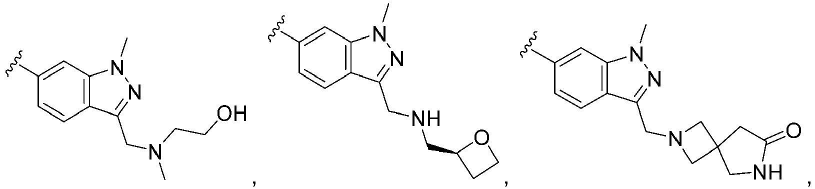

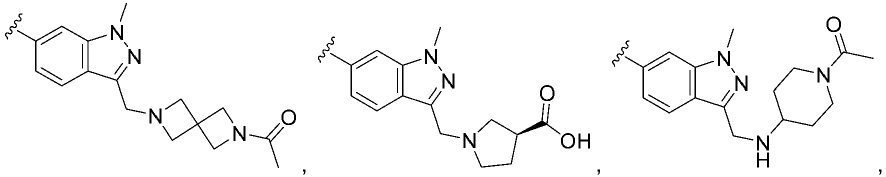

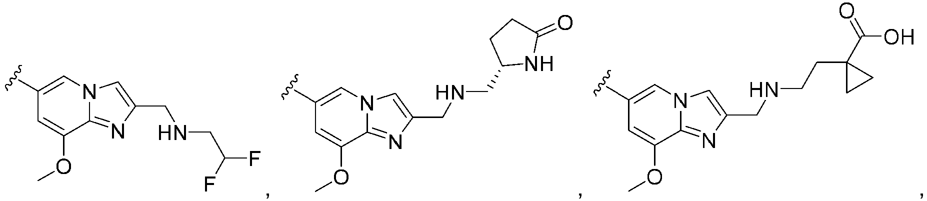

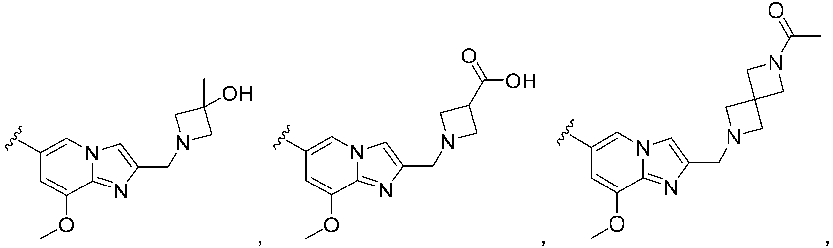

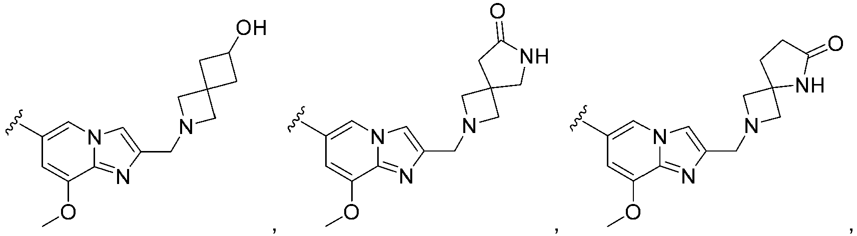

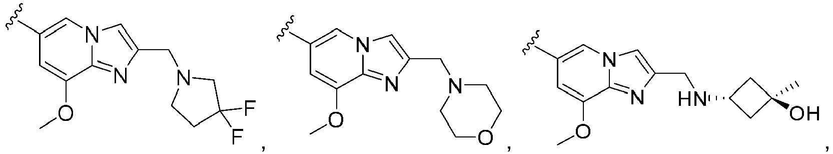

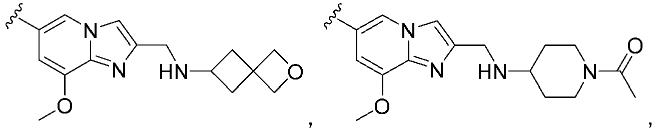

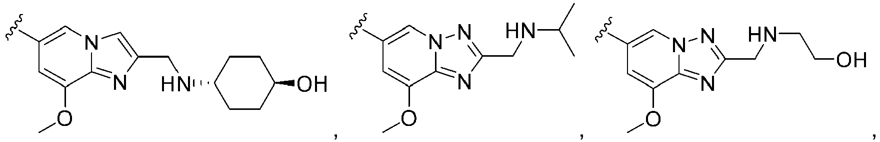

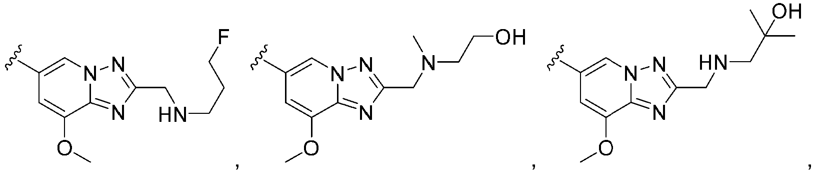

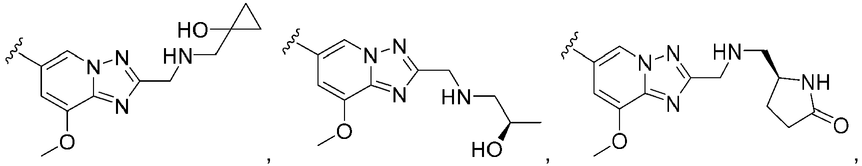

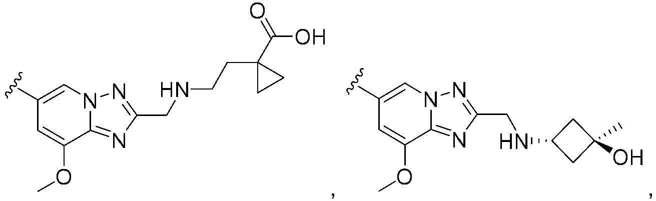

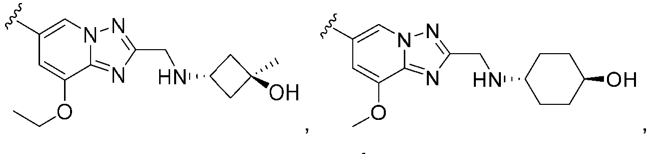

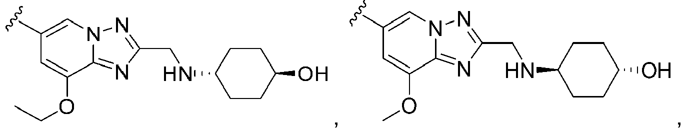

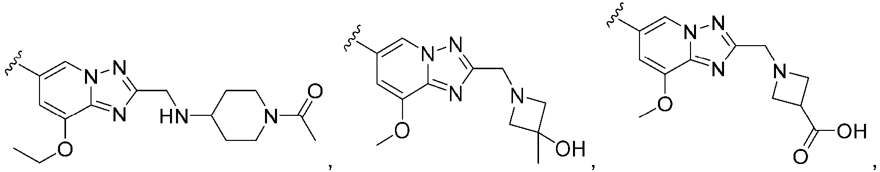

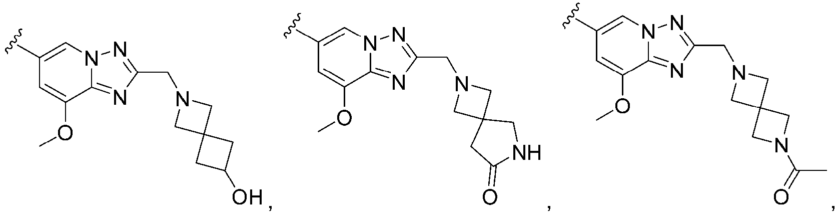

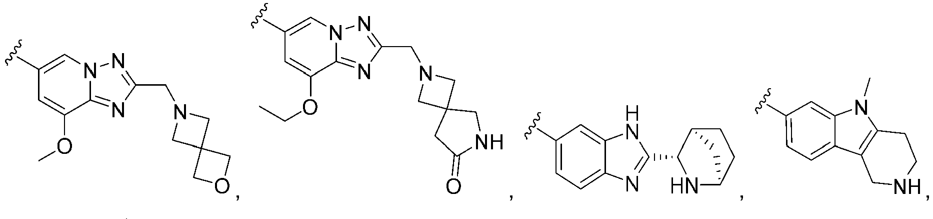

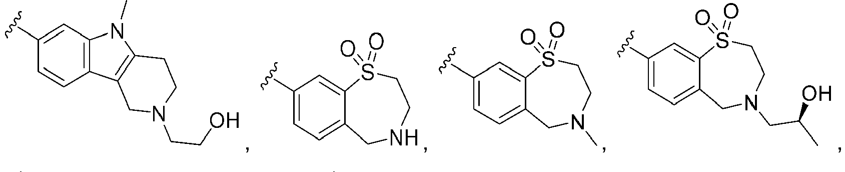

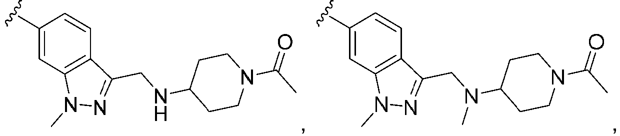

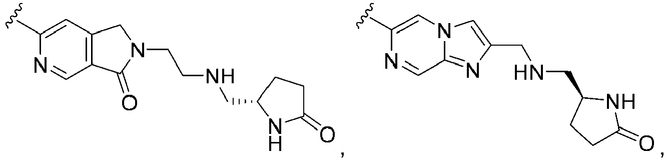

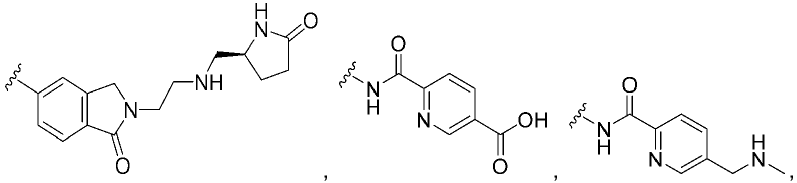

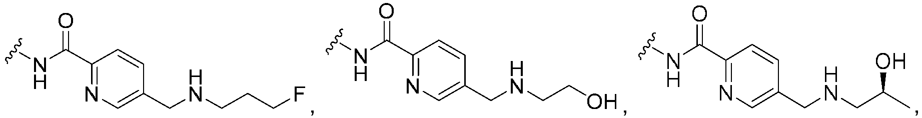

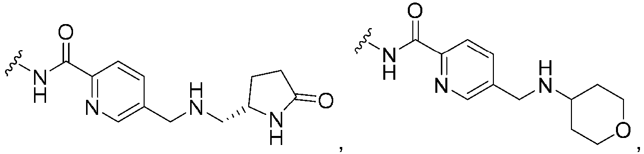

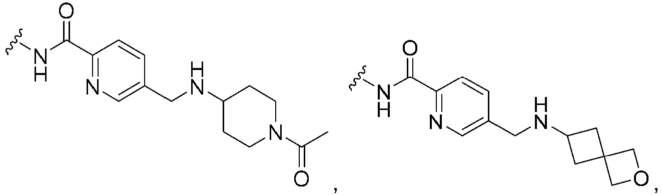

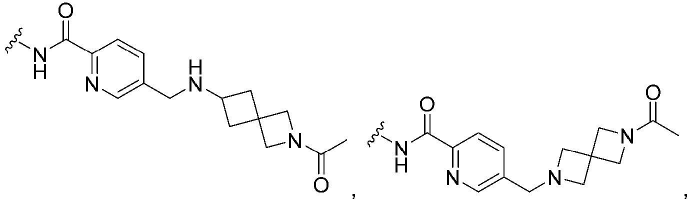

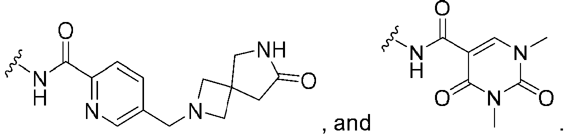

8. The compound of any one of claims 1-7, which is selected from the group consisting of:
8-(2-chloro-3-(3-chloro-2-(3-methoxy-4-(((((S)-5-oxopyrrolidin-2-yl)methyl)amino)methyl)phenyl)pyridin-4-yl)phenyl)-3-(((((S)-5-oxopyrrolidin-2-yl)methyl)amino)methyl)-4H-pyrido[1,2-a]pyrimidin-4-one;
8-(2-chloro-3-(3-chloro-2-(3-methoxy-4-(((((S)-5-oxopyrrolidin-2-yl)methyl)amino)methyl)phenyl)pyridin-4-yl)phenyl)-3-(((((R)-5-oxopyrrolidin-2-yl)methyl)amino)methyl)-4H-pyrido[1,2-a]pyrimidin-4-one;
8-(2-chloro-3-(3-chloro-2-(3-methoxy-4-(((((R)-5-oxopyrrolidin-2-yl)methyl)amino)methyl)phenyl)pyridin-4-yl)phenyl)-3-(((((S)-5-oxopyrrolidin-2-yl)methyl)amino)methyl)-4H-pyrido[1,2-a]pyrimidin-4-one;
8-(2-chloro-3-(3-chloro-2-(3-methoxy-4-(((((R)-5-oxopyrrolidin-2-yl)methyl)amino)methyl)phenyl)pyridin-4-yl)phenyl)-3-(((((R)-5-oxopyrrolidin-2-yl)methyl)amino)methyl)-4H-pyrido[1,2-a]pyrimidin-4-one;
8-(3-chloro-4-(2-chloro-3-(6-methoxy-5-(((((S)-5-oxopyrrolidin-2-yl)methyl)amino)methyl)pyridin-2-yl)phenyl)pyridin-2-yl)-3-(((((S)-5-oxopyrrolidin-2-yl)methyl)amino)methyl)-4H-pyrido[1,2-a]pyrimidin-4-one;
8-(3-chloro-4-(2-chloro-3-(6-methoxy-5-(((((S)-5-oxopyrrolidin-2-yl)methyl)amino)methyl)pyridin-2-yl)phenyl)pyridin-2-yl)-3-(((((R)-5-oxopyrrolidin-2-yl)methyl)amino)methyl)-4H-pyrido[1,2-a]pyrimidin-4-one;
8-(3-chloro-4-(2-chloro-3-(6-methoxy-5-(((((R)-5-oxopyrrolidin-2-yl)methyl)amino)methyl)pyridin-2-yl)phenyl)pyridin-2-yl)-3-(((((S)-5-oxopyrrolidin-2-yl)methyl)amino)methyl)-4H-pyrido[1,2-a]pyrimidin-4-one;
8-(3-chloro-4-(2-chloro-3-(6-methoxy-5-(((((R)-5-oxopyrrolidin-2-yl)methyl)amino)methyl)pyridin-2-yl)phenyl)pyridin-2-yl)-3-(((((R)-5-oxopyrrolidin-2-yl)methyl)amino)methyl)-4H-pyrido[1,2-a]pyrimidin-4-one;
(S)-5-((((6-(2-chloro-3-(3-chloro-2-(1-methyl-2,3,4,5-tetrahydro-1H-benzo[e][1,4]diazepin-8-yl)pyridin-4-yl)phenyl)-2-methoxypyridin-3-yl)methyl)amino)methyl)pyrrolidin-2-one;
(R)-5-((((6-(2-chloro-3-(3-chloro-2-(1-methyl-2,3,4,5-tetrahydro-1H-benzo[e][1,4]diazepin-8-yl)pyridin-4-yl)phenyl)-2-methoxypyridin-3-yl)methyl)amino)methyl)pyrrolidin-2-one;
(5S,5'S)-5,5'-(((((3,3'-dichloro-[4,4'-bipyridine]-2,2'-diyl)bis(2-methoxy-4,1-phenylene))bis(methylene))bis(azanediyl))bis(methylene))bis(pyrrolidin-2-one);
(5S,5'R)-5,5'-(((((3,3'-dichloro-[4,4'-bipyridine]-2,2'-diyl)bis(2-methoxy-4,1-phenylene))bis(methylene))bis(azanediyl))bis(methylene))bis(pyrrolidin-2-one);
(5R,5'S)-5,5'-(((((3,3'-dichloro-[4,4'-bipyridine]-2,2'-diyl)bis(2-methoxy-4,1-phenylene))bis(methylene))bis(azanediyl))bis(methylene))bis(pyrrolidin-2-one);
(5R,5'R)-5,5'-(((((3,3'-dichloro-[4,4'-bipyridine]-2,2'-diyl)bis(2-methoxy-4,1-phenylene))bis(methylene))bis(azanediyl))bis(methylene))bis(pyrrolidin-2-one);
N-(1-(((6-(2-chloro-3-(3-chloro-2-(1-methyl-2,3,4,5-tetrahydro-1H-benzo[e][1,4]diazepin-8-yl)pyridin-4-yl)phenyl)-2-methoxypyridin-3-yl)methyl)amino)propan-2-yl)acetamide;
(S)-N-(1-(((6-(2-chloro-3-(3-chloro-2-(1-methyl-2,3,4,5-tetrahydro-1H-benzo[e][1,4]diazepin-8-yl)pyridin-4-yl)phenyl)-2-methoxypyridin-3-yl)methyl)amino)propan-2-yl)acetamide;
(R)-N-(1-(((6-(2-chloro-3-(3-chloro-2-(1-methyl-2,3,4,5-tetrahydro-1H-benzo[e][1,4]diazepin-8-yl)pyridin-4-yl)phenyl)-2-methoxypyridin-3-yl)methyl)amino)propan-2-yl)acetamide;
(S)-5-((((6-(2-chloro-3-(3-chloro-2-(8-methoxy-2-(((S)-5-oxopyrrolidin-2-yl)methyl)-1,2,3,4-tetrahydroisoquinolin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(8-methoxy-2-(((R)-5-oxopyrrolidin-2-yl)methyl)-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(8-methoxy-2-(((S)-5-oxopyrrolidin-2-yl)methyl)-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(8-methoxy-2-(((R)-5-oxopyrrolidin-2-yl)methyl)-1,2,3,4-tetrahydroisoquinolin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(8-methoxy-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(8-methoxy-1,2,3,4-tetrahydroisoquinolin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(3-methoxy-4-(((((S)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
(S)-5-((((6-(2-chloro-3-(3-chloro-2-(3-methoxy-4-(((((R)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
(R)-5-((((6-(2-chloro-3-(3-chloro-2-(3-methoxy-4-(((((S)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
(R)-5-((((6-(2-chloro-3-(3-chloro-2-(3-methoxy-4-(((((R)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
(S)-5-((((6-(2-chloro-3-(3-chloro-2-(1-methyl-2,3,4,5-tetrahydro-1H-benzo[e][1,4]diazepin-8-yl)pyridin-4-yl)phenyl)-1-methyl-1H-pyrrolo[2,3-b]pyridin-3-yl)methyl)amino)methyl)pyrrolidin-2-one;
(R)-5-((((6-(2-chloro-3-(3-chloro-2-(1-methyl-2,3,4,5-tetrahydro-1H-benzo[e][1,4]diazepin-8-yl)pyridin-4-yl)phenyl)-1-methyl-1H-pyrrolo[2,3-b]pyridin-3-yl)methyl)amino)methyl)pyrrolidin-2-one;
(S)-5-((((6-(2-chloro-3-(3-chloro-2-(3-fluoro-5-methoxy-4-(((((S)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
(S)-5-((((6-(2-chloro-3-(3-chloro-2-(3-fluoro-5-methoxy-4-(((((R)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
(R)-5-((((6-(2-chloro-3-(3-chloro-2-(3-fluoro-5-methoxy-4-(((((S)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
(R)-5-((((6-(2-chloro-3-(3-chloro-2-(3-fluoro-5-methoxy-4-(((((R)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
(S)-5-((((6-(2-chloro-3-(3-chloro-2-(1-methyl-3-(((((S)-5-oxopyrrolidin-2-yl)methyl)amino)methyl)-1H-indol-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(1-methyl-3-(((((R)-5-oxopyrrolidin-2-yl)methyl)amino)methyl)-1H-indol-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(1-methyl-3-(((((S)-5-oxopyrrolidin-2-yl)methyl)amino)methyl)-1H-indol-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(1-methyl-3-(((((R)-5-oxopyrrolidin-2-yl)methyl)amino)methyl)-1H-indol-6-yl)pyridin-4-yl)phenyl)-2-methoxypyridin-3-yl)methyl)amino)methyl)pyrrolidin-2-one;
(5S,5'S)-5,5'-(((((3,3'-dichloro-[4,4'-bipyridine]-2,2'-diyl)bis(2-fluoro-6-methoxy-4,1-phenylene))bis(methylene))bis(azanediyl))bis(methylene))bis(pyrrolidin-2-one);
(5S,5'R)-5,5'-(((((3,3'-dichloro-[4,4'-bipyridine]-2,2'-diyl)bis(2-fluoro-6-methoxy-4,1-phenylene))bis(methylene))bis(azanediyl))bis(methylene))bis(pyrrolidin-2-one);
(5R,5'S)-5,5'-(((((3,3'-dichloro-[4,4'-bipyridine]-2,2'-diyl)bis(2-fluoro-6-methoxy-4,1-phenylene))bis(methylene))bis(azanediyl))bis(methylene))bis(pyrrolidin-2-one);
(5R,5'R)-5,5'-(((((3,3'-dichloro-[4,4'-bipyridine]-2,2'-diyl)bis(2-fluoro-6-methoxy-4,1-phenylene))bis(methylene))bis(azanediyl))bis(methylene))bis(pyrrolidin-2-one);
(S)-5-((((6-(2-chloro-3-(3-chloro-2-(1-methyl-3-(((((S)-5-oxopyrrolidin-2-yl)methyl)amino)methyl)-1H-pyrrolo[3,2-b]pyridin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(1-methyl-3-(((((R)-5-oxopyrrolidin-2-yl)methyl)amino)methyl)-1H-pyrrolo[3,2-b]pyridin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(1-methyl-3-(((((S)-5-oxopyrrolidin-2-yl)methyl)amino)methyl)-1H-pyrrolo[3,2-b]pyridin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(1-methyl-3-(((((R)-5-oxopyrrolidin-2-yl)methyl)amino)methyl)-1H-pyrrolo[3,2-b]pyridin-6-yl)pyridin-4-yl)phenyl)-2-methoxypyridin-3-yl)methyl)amino)methyl)pyrrolidin-2-one;
(S)-5-(((4-(3,3'-dichloro-2'-(1-methyl-3-(((((S)-5-oxopyrrolidin-2-yl)methyl)amino)methyl)-1H-indol-6-yl)-[4,4'-bipyridin]-2-yl)-2-methoxybenzyl)amino)methyl)pyrrolidin-2-one;
(S)-5-(((4-(3,3'-dichloro-2'-(1-methyl-3-(((((R)-5-oxopyrrolidin-2-yl)methyl)amino)methyl)-1H-indol-6-yl)-[4,4'-bipyridin]-2-yl)-2-methoxybenzyl)amino)methyl)pyrrolidin-2-one;
(R)-5-(((4-(3,3'-dichloro-2'-(1-methyl-3-(((((S)-5-oxopyrrolidin-2-yl)methyl)amino)methyl)-1H-indol-6-yl)-[4,4'-bipyridin]-2-yl)-2-methoxybenzyl)amino)methyl)pyrrolidin-2-one;
(R)-5-(((4-(3,3'-dichloro-2'-(1-methyl-3-(((((R)-5-oxopyrrolidin-2-yl)methyl)amino)methyl)-1H-indol-6-yl)-[4,4'-bipyridin]-2-yl)-2-methoxybenzyl)amino)methyl)pyrrolidin-2-one;
(5S,5'S)-5,5'-(((((3,3'-dichloro-[4,4'-bipyridine]-2,2'-diyl)bis(1-methyl-1H-indole-6,3-diyl))bis(methylene))bis(azanediyl))bis(methylene))bis(pyrrolidin-2-one);
(5S,5'R)-5,5'-(((((3,3'-dichloro-[4,4'-bipyridine]-2,2'-diyl)bis(1-methyl-1H-indole-6,3-diyl))bis(methylene))bis(azanediyl))bis(methylene))bis(pyrrolidin-2-one);
(5R,5'S)-5,5'-(((((3,3'-dichloro-[4,4'-bipyridine]-2,2'-diyl)bis(1-methyl-1H-indole-6,3-diyl))bis(methylene))bis(azanediyl))bis(methylene))bis(pyrrolidin-2-one);
(5R,5'R)-5,5'-(((((3,3'-dichloro-[4,4'-bipyridine]-2,2'-diyl)bis(1-methyl-1H-indole-6,3-diyl))bis(methylene))bis(azanediyl))bis(methylene))bis(pyrrolidin-2-one);
(S)-5-(((4-(3,3'-dichloro-2'-(1-methyl-3-(((((S)-5-oxopyrrolidin-2-yl)methyl)amino)methyl)-1H-pyrrolo[3,2-b]pyridin-6-yl)-[4,4'-bipyridin]-2-yl)-2-methoxybenzyl)amino)methyl)pyrrolidin-2-one;
(S)-5-(((4-(3,3'-dichloro-2'-(1-methyl-3-(((((R)-5-oxopyrrolidin-2-yl)methyl)amino)methyl)-1H-pyrrolo[3,2-b]pyridin-6-yl)-[4,4'-bipyridin]-2-yl)-2-methoxybenzyl)amino)methyl)pyrrolidin-2-one;
(R)-5-(((4-(3,3'-dichloro-2'-(1-methyl-3-(((((S)-5-oxopyrrolidin-2-yl)methyl)amino)methyl)-1H-pyrrolo[3,2-b]pyridin-6-yl)-[4,4'-bipyridin]-2-yl)-2-methoxybenzyl)amino)methyl)pyrrolidin-2-one;
(R)-5-(((4-(3,3'-dichloro-2'-(1-methyl-3-(((((R)-5-oxopyrrolidin-2-yl)methyl)amino)methyl)-1H-pyrrolo[3,2-b]pyridin-6-yl)-[4,4'-bipyridin]-2-yl)-2-methoxybenzyl)amino)methyl)pyrrolidin-2-one;
(S)-5-((((6-(2-chloro-3-(3-chloro-2-(3-methoxy-4-(((((S)-5-oxopyrrolidin-2-yl)methyl)amino)methyl)phenyl)pyridin-4-yl)phenyl)-1-methyl-1H-pyrrolo[2,3-b]pyridin-3-yl)methyl)amino)methyl)pyrrolidin-2-one;
(S)-5-((((6-(2-chloro-3-(3-chloro-2-(3-methoxy-4-(((((R)-5-oxopyrrolidin-2-yl)methyl)amino)methyl)phenyl)pyridin-4-yl)phenyl)-1-methyl-1H-pyrrolo[2,3-b]pyridin-3-yl)methyl)amino)methyl)pyrrolidin-2-one;
(R)-5-((((6-(2-chloro-3-(3-chloro-2-(3-methoxy-4-(((((S)-5-oxopyrrolidin-2-yl)methyl)amino)methyl)phenyl)pyridin-4-yl)phenyl)-1-methyl-1H-pyrrolo[2,3-b]pyridin-3-yl)methyl)amino)methyl)pyrrolidin-2-one;
(R)-5-((((6-(2-chloro-3-(3-chloro-2-(3-methoxy-4-(((((R)-5-oxopyrrolidin-2-yl)methyl)amino)methyl)phenyl)pyridin-4-yl)phenyl)-1-methyl-1H-pyrrolo[2,3-b]pyridin-3-yl)methyl)amino)methyl)pyrrolidin-2-one;
(S)-5-((6-(2-chloro-3-(3-chloro-2-(3-methoxy-4-(((((S)-5-oxopyrrolidin-2-yl)methyl)amino)methyl)phenyl)pyridin-4-yl)phenyl)-8-methoxy-3,4-dihydroisoquinolin-2(1H)-yl)methyl)pyrrolidin-2-one;
(S)-5-((6-(2-chloro-3-(3-chloro-2-(3-methoxy-4-(((((R)-5-oxopyrrolidin-2-yl)methyl)amino)methyl)phenyl)pyridin-4-yl)phenyl)-8-methoxy-3,4-dihydroisoquinolin-2(1H)-yl)methyl)pyrrolidin-2-one;
(R)-5-((6-(2-chloro-3-(3-chloro-2-(3-methoxy-4-(((((S)-5-oxopyrrolidin-2-yl)methyl)amino)methyl)phenyl)pyridin-4-yl)phenyl)-8-methoxy-3,4-dihydroisoquinolin-2(1H)-yl)methyl)pyrrolidin-2-one;
(R)-5-((6-(2-chloro-3-(3-chloro-2-(3-methoxy-4-(((((R)-5-oxopyrrolidin-2-yl)methyl)amino)methyl)phenyl)pyridin-4-yl)phenyl)-8-methoxy-3,4-dihydroisoquinolin-2(1H)-yl)methyl)pyrrolidin-2-one;
(S)-5-((((6-(2-chloro-3-(3-chloro-2-(1-methyl-4-(((S)-5-oxopyrrolidin-2-yl)methyl)-2,3,4,5-tetrahydro-1H-benzo[e][1,4]diazepin-8-yl)pyridin-4-yl)phenyl)-2-methoxypyridin-3-yl)methyl)amino)methyl)pyrrolidin-2-one;
(S)-5-((((6-(2-chloro-3-(3-chloro-2-(1-methyl-4-(((R)-5-oxopyrrolidin-2-yl)methyl)-2,3,4,5-tetrahydro-1H-benzo[e][1,4]diazepin-8-yl)pyridin-4-yl)phenyl)-2-methoxypyridin-3-yl)methyl)amino)methyl)pyrrolidin-2-one;
(R)-5-((((6-(2-chloro-3-(3-chloro-2-(1-methyl-4-(((S)-5-oxopyrrolidin-2-yl)methyl)-2,3,4,5-tetrahydro-1H-benzo[e][1,4]diazepin-8-yl)pyridin-4-yl)phenyl)-2-methoxypyridin-3-yl)methyl)amino)methyl)pyrrolidin-2-one;
(R)-5-((((6-(2-chloro-3-(3-chloro-2-(1-methyl-4-(((R)-5-oxopyrrolidin-2-yl)methyl)-2,3,4,5-tetrahydro-1H-benzo[e][1,4]diazepin-8-yl)pyridin-4-yl)phenyl)-2-methoxypyridin-3-yl)methyl)amino)methyl)pyrrolidin-2-one;
(S)-5-((((5-(2-chloro-3-(3-chloro-2-(3-methoxy-4-(((((S)-5-oxopyrrolidin-2-yl)methyl)amino)methyl)phenyl)pyridin-4-yl)phenyl)-3-methoxypyrazin-2-yl)methyl)amino)methyl)pyrrolidin-2-one;
(S)-5-((((5-(2-chloro-3-(3-chloro-2-(3-methoxy-4-(((((R)-5-oxopyrrolidin-2-yl)methyl)amino)methyl)phenyl)pyridin-4-yl)phenyl)-3-methoxypyrazin-2-yl)methyl)amino)methyl)pyrrolidin-2-one;
(R)-5-((((5-(2-chloro-3-(3-chloro-2-(3-methoxy-4-(((((S)-5-oxopyrrolidin-2-yl)methyl)amino)methyl)phenyl)pyridin-4-yl)phenyl)-3-methoxypyrazin-2-yl)methyl)amino)methyl)pyrrolidin-2-one;
(R)-5-((((5-(2-chloro-3-(3-chloro-2-(3-methoxy-4-(((((R)-5-oxopyrrolidin-2-yl)methyl)amino)methyl)phenyl)pyridin-4-yl)phenyl)-3-methoxypyrazin-2-yl)methyl)amino)methyl)pyrrolidin-2-one;
(S)-5-((((6-(3-(2-(3-(((1-acetylpiperidin-4-yl)amino)methyl)-1-methyl-1H-pyrrolo[3,2-b]pyridin-6-yl)-3-chloropyridin-4-yl)-2-chlorophenyl)-2-methoxypyridin-3-yl)methyl)amino)methyl)pyrrolidin-2-one;
(R)-5-((((6-(3-(2-(3-(((1-acetylpiperidin-4-yl)amino)methyl)-1-methyl-1H-pyrrolo[3,2-b]pyridin-6-yl)-3-chloropyridin-4-yl)-2-chlorophenyl)-2-methoxypyridin-3-yl)methyl)amino)methyl)pyrrolidin-2-one;
(S)-5-((((6-(2-chloro-3-(3-chloro-2-(1-methyl-3-(((((S)-oxetan-2-yl)methyl)amino)methyl)-1H-pyrrolo[3,2-b]pyridin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(1-methyl-3-(((((R)-oxetan-2-yl)methyl)amino)methyl)-1H-pyrrolo[3,2-b]pyridin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(1-methyl-3-(((((S)-oxetan-2-yl)methyl)amino)methyl)-1H-pyrrolo[3,2-b]pyridin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(1-methyl-3-(((((R)-oxetan-2-yl)methyl)amino)methyl)-1H-pyrrolo[3,2-b]pyridin-6-yl)pyridin-4-yl)phenyl)-2-methoxypyridin-3-yl)methyl)amino)methyl)pyrrolidin-2-one;
(S)-5-((((6-(2-chloro-3-(3-chloro-5'-methoxy-6'-(((((S)-5-oxopyrrolidin-2-yl)methyl)amino)methyl)-[2,3'-bipyridin]-4-yl)phenyl)-2-methoxypyridin-3-yl)methyl)amino)methyl)pyrrolidin-2-one;
(S)-5-((((6-(2-chloro-3-(3-chloro-5'-methoxy-6'-(((((R)-5-oxopyrrolidin-2-yl)methyl)amino)methyl)-[2,3'-bipyridin]-4-yl)phenyl)-2-methoxypyridin-3-yl)methyl)amino)methyl)pyrrolidin-2-one;
(R)-5-((((6-(2-chloro-3-(3-chloro-5'-methoxy-6'-(((((S)-5-oxopyrrolidin-2-yl)methyl)amino)methyl)-[2,3'-bipyridin]-4-yl)phenyl)-2-methoxypyridin-3-yl)methyl)amino)methyl)pyrrolidin-2-one;
(R)-5-((((6-(2-chloro-3-(3-chloro-5'-methoxy-6'-(((((R)-5-oxopyrrolidin-2-yl)methyl)amino)methyl)-[2,3'-bipyridin]-4-yl)phenyl)-2-methoxypyridin-3-yl)methyl)amino)methyl)pyrrolidin-2-one;
(S)-5-((((6-(2-chloro-3-(3-chloro-2-(4-((S)-2-hydroxypropyl)-1-methyl-2,3,4,5-tetrahydro-1H-benzo[e][1,4]diazepin-8-yl)pyridin-4-yl)phenyl)-2-methoxypyridin-3-yl)methyl)amino)methyl)pyrrolidin-2-one;
(S)-5-((((6-(2-chloro-3-(3-chloro-2-(4-((R)-2-hydroxypropyl)-1-methyl-2,3,4,5-tetrahydro-1H-benzo[e][1,4]diazepin-8-yl)pyridin-4-yl)phenyl)-2-methoxypyridin-3-yl)methyl)amino)methyl)pyrrolidin-2-one;
(R)-5-((((6-(2-chloro-3-(3-chloro-2-(4-((S)-2-hydroxypropyl)-1-methyl-2,3,4,5-tetrahydro-1H-benzo[e][1,4]diazepin-8-yl)pyridin-4-yl)phenyl)-2-methoxypyridin-3-yl)methyl)amino)methyl)pyrrolidin-2-one;
(R)-5-((((6-(2-chloro-3-(3-chloro-2-(4-((R)-2-hydroxypropyl)-1-methyl-2,3,4,5-tetrahydro-1H-benzo[e][1,4]diazepin-8-yl)pyridin-4-yl)phenyl)-2-methoxypyridin-3-yl)methyl)amino)methyl)pyrrolidin-2-one;
(S)-5-((((6-(2-chloro-3-(3-chloro-2-(4-((S)-2-hydroxypropyl)-2,3,4,5-tetrahydrobenzo[f][1,4]oxazepin-8-yl)pyridin-4-yl)phenyl)-2-methoxypyridin-3-yl)methyl)amino)methyl)pyrrolidin-2-one;
(S)-5-((((6-(2-chloro-3-(3-chloro-2-(4-((R)-2-hydroxypropyl)-2,3,4,5-tetrahydrobenzo[f][1,4]oxazepin-8-yl)pyridin-4-yl)phenyl)-2-methoxypyridin-3-yl)methyl)amino)methyl)pyrrolidin-2-one;
(R)-5-((((6-(2-chloro-3-(3-chloro-2-(4-((S)-2-hydroxypropyl)-2,3,4,5-tetrahydrobenzo[f][1,4]oxazepin-8-yl)pyridin-4-yl)phenyl)-2-methoxypyridin-3-yl)methyl)amino)methyl)pyrrolidin-2-one;
(R)-5-((((6-(2-chloro-3-(3-chloro-2-(4-((R)-2-hydroxypropyl)-2,3,4,5-tetrahydrobenzo[f][1,4]oxazepin-8-yl)pyridin-4-yl)phenyl)-2-methoxypyridin-3-yl)methyl)amino)methyl)pyrrolidin-2-one;
(S)-5-((((6-(2-chloro-3-(3-chloro-2-(2,3,4,5-tetrahydrobenzo[f][1,4]oxazepin-8-yl)pyridin-4-yl)phenyl)-2-methoxypyridin-3-yl)methyl)amino)methyl)pyrrolidin-2-one;
(R)-5-((((6-(2-chloro-3-(3-chloro-2-(2,3,4,5-tetrahydrobenzo[f][1,4]oxazepin-8-yl)pyridin-4-yl)phenyl)-2-methoxypyridin-3-yl)methyl)amino)methyl)pyrrolidin-2-one;
(S)-5-((((6-(2-chloro-3-(3-chloro-2-(2-((R)-2-hydroxypropyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(2-((S)-2-hydroxypropyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-((R)-2-hydroxypropyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-((S)-2-hydroxypropyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(S)-5-((((6-(3-(2-(3-(((1-acetylpiperidin-4-yl)amino)methyl)-1-methyl-1H-indol-6-yl)-3-chloropyridin-4-yl)-2-chlorophenyl)-2-methoxypyridin-3-yl)methyl)amino)methyl)pyrrolidin-2-one;
(R)-5-((((6-(3-(2-(3-(((1-acetylpiperidin-4-yl)amino)methyl)-1-methyl-1H-indol-6-yl)-3-chloropyridin-4-yl)-2-chlorophenyl)-2-methoxypyridin-3-yl)methyl)amino)methyl)pyrrolidin-2-one;
(S)-5-((((6-(2-chloro-3-(3-chloro-2-(3-(((2-hydroxyethyl)amino)methyl)-1-methyl-1H-indol-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(3-(((2-hydroxyethyl)amino)methyl)-1-methyl-1H-indol-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(3-(((2-methoxyethyl)amino)methyl)-1-methyl-1H-indol-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(3-(((2-methoxyethyl)amino)methyl)-1-methyl-1H-indol-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(1-methyl-3-((methylamino)methyl)-1H-indol-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(1-methyl-3-((methylamino)methyl)-1H-indol-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(3-((ethylamino)methyl)-1-methyl-1H-indol-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(3-((ethylamino)methyl)-1-methyl-1H-indol-6-yl)}pyridin-4-yl)phenyl)-2-methoxypyridin-3-yl)methyl)amino)methyl)pyrrolidin-2-one;
(S)-5-((((6-(2-chloro-3-(3-chloro-2-(3-((dimethylamino)methyl)-1-methyl-1H-indol-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(3-((dimethylamino)methyl)-1-methyl-1H-indol-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(1-methyl-3-(((((S)-oxetan-2-yl)methyl)amino)methyl)-1H-indol-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(1-methyl-3-(((((R)-oxetan-2-yl)methyl)amino)methyl)-1H-indol-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(1-methyl-3-(((((S)-oxetan-2-yl)methyl)amino)methyl)-1H-indol-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(1-methyl-3-(((((R)-oxetan-2-yl)methyl)amino)methyl)-1H-indol-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(3-((((S)-2-hydroxypropyl)amino)methyl)-1-methyl-1H-indol-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(3-((((R)-2-hydroxypropyl)amino)methyl)-1-methyl-1H-indol-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(3-((((S)-2-hydroxypropyl)amino)methyl)-1-methyl-1H-indol-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(3-((((R)-2-hydroxypropyl)amino)methyl)-1-methyl-1H-indol-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(3-(((2-hydroxy-2-methylpropyl)amino)methyl)-1-methyl-1H-indol-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(3-(((2-hydroxy-2-methylpropyl)amino)methyl)-1-methyl-1H-indol-6-yl)pyridin-4-yl)phenyl)-2-methoxypyridin-3-yl)methyl)amino)methyl)pyrrolidin-2-one;
(S)-5-((6-(3-chloro-4-(2-chloro-3-(4-chloro-6-methoxy-5-(((((S)-5-oxopyrrolidin-2-yl)methyl)amino)methyl)pyridin-2-yl)phenyl)pyridin-2-yl)-8-methoxy-3,4-dihydroisoquinolin-2(1H)-yl)methyl)pyrrolidin-2-one;
(S)-5-((6-(3-chloro-4-(2-chloro-3-(4-chloro-6-methoxy-5-(((((R)-5-oxopyrrolidin-2-yl)methyl)amino)methyl)pyridin-2-yl)phenyl)pyridin-2-yl)-8-methoxy-3,4-dihydroisoquinolin-2(1H)-yl)methyl)pyrrolidin-2-one;
(R)-5-((6-(3-chloro-4-(2-chloro-3-(4-chloro-6-methoxy-5-(((((S)-5-oxopyrrolidin-2-yl)methyl)amino)methyl)pyridin-2-yl)phenyl)pyridin-2-yl)-8-methoxy-3,4-dihydroisoquinolin-2(1H)-yl)methyl)pyrrolidin-2-one;
(R)-5-((6-(3-chloro-4-(2-chloro-3-(4-chloro-6-methoxy-5-(((((R)-5-oxopyrrolidin-2-yl)methyl)amino)methyl)pyridin-2-yl)phenyl)pyridin-2-yl)-8-methoxy-3,4-dihydroisoquinolin-2(1H)-yl)methyl)pyrrolidin-2-one;
(S)-5-((((6-(2-chloro-3-(3-chloro-2-(3-((isobutylamino)methyl)-1-methyl-1H-indol-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(3-((isobutylamino)methyl)-1-methyl-1H-indol-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(3-(((2-hydroxyethyl)(methyl)amino)methyl)-1-methyl-1H-indol-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(3-(((2-hydroxyethyl)(methyl)amino)methyl)-1-methyl-1H-indol-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(3-((((S)-2-methoxypropyl)amino)methyl)-1-methyl-1H-indol-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(3-((((R)-2-methoxypropyl)amino)methyl)-1-methyl-1H-indol-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(3-((((S)-2-methoxypropyl)amino)methyl)-1-methyl-1H-indol-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(3-((((R)-2-methoxypropyl)amino)methyl)-1-methyl-1H-indol-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(1-methyl-3-(((((S)-tetrahydrofuran-2-yl)methyl)amino)methyl)-1H-indol-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(1-methyl-3-(((((R)-tetrahydrofuran-2-yl)methyl)amino)methyl)-1H-indol-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(1-methyl-3-(((((S)-tetrahydrofuran-2-yl)methyl)amino)methyl)-1H-indol-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(1-methyl-3-(((((R)-tetrahydrofuran-2-yl)methyl)amino)methyl)-1H-indol-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(1-methyl-3-(((tetrahydro-2H-pyran-4-yl)amino)methyl)-1H-indol-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(1-methyl-3-(((tetrahydro-2H-pyran-4-yl)amino)methyl)-1H-indol-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(3-(((1,1-dioxidotetrahydro-2H-thiopyran-4-yl)amino)methyl)-1-methyl-1H-indol-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(3-(((1,1-dioxidotetrahydro-2H-thiopyran-4-yl)amino)methyl)-1-methyl-1H-indol-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(2-((S)-2-hydroxypropyl)-5-methoxy-1,2,3,4-tetrahydroisoquinolin-7-yl)pyridin-4-yl)phenyl)-2-methoxypyridin-3-yl)methyl)amino)methyl)pyrrolidin-2-one;
(S)-5-((((6-(2-chloro-3-(3-chloro-2-(2-((R)-2-hydroxypropyl)-5-methoxy-1,2,3,4-tetrahydroisoquinolin-7-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-((S)-2-hydroxypropyl)-5-methoxy-1,2,3,4-tetrahydroisoquinolin-7-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-((R)-2-hydroxypropyl)-5-methoxy-1,2,3,4-tetrahydroisoquinolin-7-yl)pyridin-4-yl)phenyl)-2-methoxypyridin-3-yl)methyl)amino)methyl)pyrrolidin-2-one;
(S)-5-((((6-(2-chloro-3-(3-chloro-2-(2-(2-hydroxy-2-methylpropyl)-5-methoxy-1,2,3,4-tetrahydroisoquinolin-7-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-(2-hydroxy-2-methylpropyl)-5-methoxy-1,2,3,4-tetrahydroisoquinolin-7-yl)pyridin-4-yl)phenyl)-2-methoxypyridin-3-yl)methyl)amino)methyl)pyrrolidin-2-one;
(S)-5-((((6-(2-chloro-3-(3-chloro-2-(8-fluoro-2-(2-hydroxy-2-methylpropyl)-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(8-fluoro-2-(2-hydroxy-2-methylpropyl)-1,2,3,4-tetrahydroisoquinolin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(5-fluoro-2-(2-hydroxy-2-methylpropyl)-1,2,3,4-tetrahydroisoquinolin-7-yl)pyridin-4-yl)phenyl)-2-methoxypyridin-3-yl)methyl)amino)methyl)pyrrolidin-2-one;
(R)-5-((((6-(2-chloro-3-(3-chloro-2-(5-fluoro-2-(2-hydroxy-2-methylpropyl)-1,2,3,4-tetrahydroisoquinolin-7-yl)pyridin-4-yl)phenyl)-2-methoxypyridin-3-yl)methyl)amino)methyl)pyrrolidin-2-one;
(S)-6-(3-chloro-4-(2-chloro-3-(6-methoxy-5-((((5-oxopyrrolidin-2-yl)methyl)amino)methyl)pyridin-2-yl)phenyl)pyridin-2-yl)-2-((6-hydroxy-2-azaspiro[3.3]heptan-2-yl)methyl)-3-methylpyrrolo[2,1-f][1,2,4]triazin-4(3H)-one;
(R)-6-(3-chloro-4-(2-chloro-3-(6-methoxy-5-((((5-oxopyrrolidin-2-yl)methyl)amino)methyl)pyridin-2-yl)phenyl)pyridin-2-yl)-2-((6-hydroxy-2-azaspiro[3.3]heptan-2-yl)methyl)-3-methylpyrrolo[2,1-f][1,2,4]triazin-4(3H)-one;
(S)-5-((((6-(2-chloro-3-(3-chloro-2-(2-(3-hydroxy-3-methylbutyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-(3-hydroxy-3-methylbutyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(2-((S)-2-hydroxypropyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(2-((R)-2-hydroxypropyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-((S)-2-hydroxypropyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-((R)-2-hydroxypropyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(2-(2-(1-hydroxycyclobutyl)ethyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-(2-(1-hydroxycyclobutyl)ethyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(7-(((((S)-5-oxopyrrolidin-2-yl)methyl)amino)methyl)quinolin-3-yl)pyridin-4-yl)phenyl)-2-methoxypyridin-3-yl)methyl)amino)methyl)pyrrolidin-2-one;
(S)-5-((((6-(2-chloro-3-(3-chloro-2-(7-(((((R)-5-oxopyrrolidin-2-yl)methyl)amino)methyl)quinolin-3-yl)pyridin-4-yl)phenyl)-2-methoxypyridin-3-yl)methyl)amino)methyl)pyrrolidin-2-one;
(R)-5-((((6-(2-chloro-3-(3-chloro-2-(7-(((((S)-5-oxopyrrolidin-2-yl)methyl)amino)methyl)quinolin-3-yl)pyridin-4-yl)phenyl)-2-methoxypyridin-3-yl)methyl)amino)methyl)pyrrolidin-2-one;
(R)-5-((((6-(2-chloro-3-(3-chloro-2-(7-(((((R)-5-oxopyrrolidin-2-yl)methyl)amino)methyl)quinolin-3-yl)pyridin-4-yl)phenyl)-2-methoxypyridin-3-yl)methyl)amino)methyl)pyrrolidin-2-one;
(5S)-5-((((6-(2-chloro-3-(3-chloro-2-(2-(2-hydroxy-3-methylbutyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(5R)-5-((((6-(2-chloro-3-(3-chloro-2-(2-(2-hydroxy-3-methylbutyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(2-(2-hydroxyethyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-(2-hydroxyethyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(S)-5-((((6-(3-(2-(3-(((1-acetylazetidin-3-yl)amino)methyl)-1-methyl-1H-indol-6-yl)-3-chloropyridin-4-yl)-2-chlorophenyl)-2-methoxypyridin-3-yl)methyl)amino)methyl)pyrrolidin-2-one;
(R)-5-((((6-(3-(2-(3-(((1-acetylazetidin-3-yl)amino)methyl)-1-methyl-1H-indol-6-yl)-3-chloropyridin-4-yl)-2-chlorophenyl)-2-methoxypyridin-3-yl)methyl)amino)methyl)pyrrolidin-2-one;
(S)-5-((((6-(3-(2-(3-(((1-acetylpiperidin-4-yl)(methyl)amino)methyl)-1-methyl-1H-indol-6-yl)-3-chloropyridin-4-yl)-2-chlorophenyl)-2-methoxypyridin-3-yl)methyl)amino)methyl)pyrrolidin-2-one;
(R)-5-((((6-(3-(2-(3-(((1-acetylpiperidin-4-yl)(methyl)amino)methyl)-1-methyl-1H-indol-6-yl)-3-chloropyridin-4-yl)-2-chlorophenyl)-2-methoxypyridin-3-yl)methyl)amino)methyl)pyrrolidin-2-one;
(S)-5-(((4-(4-(3-(3-(((1-acetylpiperidin-4-yl)amino)methyl)-1-methyl-1H-pyrrolo[2,3-b]pyridin-6-yl)-2-chlorophenyl)-3-chloropyridin-2-yl)-2-methoxybenzyl)amino)methyl)pyrrolidin-2-one;
(R)-5-(((4-(4-(3-(3-(((1-acetylpiperidin-4-yl)amino)methyl)-1-methyl-1H-pyrrolo[2,3-b]pyridin-6-yl)-2-chlorophenyl)-3-chloropyridin-2-yl)-2-methoxybenzyl)amino)methyl)pyrrolidin-2-one;
(S)-5-(((4-(3-chloro-4-(2-chloro-3-(1-methyl-3-(((((S)-oxetan-2-yl)methyl)amino)methyl)-1H-pyrrolo[2,3-b]pyridin-6-yl)phenyl)pyridin-2-yl)-2-methoxybenzyl)amino)methyl)pyrrolidin-2-one;
(S)-5-(((4-(3-chloro-4-(2-chloro-3-(1-methyl-3-(((((R)-oxetan-2-yl)methyl)amino)methyl)-1H-pyrrolo[2,3-b]pyridin-6-yl)phenyl)pyridin-2-yl)-2-methoxybenzyl)amino)methyl)pyrrolidin-2-one;
(R)-5-(((4-(3-chloro-4-(2-chloro-3-(1-methyl-3-(((((S)-oxetan-2-yl)methyl)amino)methyl)-1H-pyrrolo[2,3-b]pyridin-6-yl)phenyl)pyridin-2-yl)-2-methoxybenzyl)amino)methyl)pyrrolidin-2-one;
(R)-5-(((4-(3-chloro-4-(2-chloro-3-(1-methyl-3-(((((R)-oxetan-2-yl)methyl)amino)methyl)-1H-pyrrolo[2,3-b]pyridin-6-yl)phenyl)pyridin-2-yl)-2-methoxybenzyl)amino)methyl)pyrrolidin-2-one;
(S)-5-(((4-(3-chloro-4-(2-chloro-3-(1-methyl-3-(((tetrahydro-2H-pyran-4-yl)amino)methyl)-1H-pyrrolo[2,3-b]pyridin-6-yl)phenyl)pyridin-2-yl)-2-methoxybenzyl)amino)methyl)pyrrolidin-2-one;
(R)-5-(((4-(3-chloro-4-(2-chloro-3-(1-methyl-3-(((tetrahydro-2H-pyran-4-yl)amino)methyl)-1H-pyrrolo[2,3-b]pyridin-6-yl)phenyl)pyridin-2-yl)-2-methoxybenzyl)amino)methyl)pyrrolidin-2-one;
(S)-5-(((4-(3-chloro-4-(2-chloro-3-(3-(((1,1-dioxidotetrahydro-2H-thiopyran-4-yl)amino)methyl)-1-methyl-1H-pyrrolo[2,3-b]pyridin-6-yl)phenyl)pyridin-2-yl)-2-methoxybenzyl)amino)methyl)pyrrolidin-2-one;
(R)-5-(((4-(3-chloro-4-(2-chloro-3-(3-(((1,1-dioxidotetrahydro-2H-thiopyran-4-yl)amino)methyl)-1-methyl-1H-pyrrolo[2,3-b]pyridin-6-yl)phenyl)pyridin-2-yl)-2-methoxybenzyl)amino)methyl)pyrrolidin-2-one;
(S)-5-((((6-(2-chloro-3-(3-chloro-2-(8-fluoro-2-(((S)-5-oxopyrrolidin-2-yl)methyl)-1,2,3,4-tetrahydroisoquinolin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(8-fluoro-2-(((R)-5-oxopyrrolidin-2-yl)methyl)-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(8-fluoro-2-(((S)-5-oxopyrrolidin-2-yl)methyl)-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(8-fluoro-2-(((R)-5-oxopyrrolidin-2-yl)methyl)-1,2,3,4-tetrahydroisoquinolin-6-yl)pyridin-4-yl)phenyl)-2-methoxypyridin-3-yl)methyl)amino)methyl)pyrrolidin-2-one;
(S)-5-((((6-(3-(2-(2-((1-acetylpiperidin-4-yl)methyl)-8-methoxy-1,2,3,4-tetrahydroisoquinolin-6-yl)-3-chloropyridin-4-yl)-2-chlorophenyl)-2-methoxypyridin-3-yl)methyl)amino)methyl)pyrrolidin-2-one;
(R)-5-((((6-(3-(2-(2-((1-acetylpiperidin-4-yl)methyl)-8-methoxy-1,2,3,4-tetrahydroisoquinolin-6-yl)-3-chloropyridin-4-yl)-2-chlorophenyl)-2-methoxypyridin-3-yl)methyl)amino)methyl)pyrrolidin-2-one;
(S)-5-((((6-(2-chloro-3-(3-chloro-2-(2-(3-fluoropropyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-(3-fluoropropyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(8-methoxy-2-(2-(piperidin-4-yl)ethyl)-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(8-methoxy-2-(2-(piperidin-4-yl)ethyl)-1,2,3,4-tetrahydroisoquinolin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(8-fluoro-2-(3-fluoropropyl)-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(8-fluoro-2-(3-fluoropropyl)-1,2,3,4-tetrahydroisoquinolin-6-yl)pyridin-4-yl)phenyl)-2-methoxypyridin-3-yl)methyl)amino)methyl)pyrrolidin-2-one;
5-(3-chloro-4-(2-chloro-3-(6-methoxy-5-(((((S)-5-oxopyrrolidin-2-yl)methyl)amino)methyl)pyridin-2-yl)phenyl)pyridin-2-yl)-2-(2-((((S)-5-oxopyrrolidin-2-yl)methyl)amino)ethyl)isoindolin-1-one;
5-(3-chloro-4-(2-chloro-3-(6-methoxy-5-(((((S)-5-oxopyrrolidin-2-yl)methyl)amino)methyl)pyridin-2-yl)phenyl)pyridin-2-yl)-2-(2-((((R)-5-oxopyrrolidin-2-yl)methyl)amino)ethyl)isoindolin-1-one;
5-(3-chloro-4-(2-chloro-3-(6-methoxy-5-(((((R)-5-oxopyrrolidin-2-yl)methyl)amino)methyl)pyridin-2-yl)phenyl)pyridin-2-yl)-2-(2-((((S)-5-oxopyrrolidin-2-yl)methyl)amino)ethyl)isoindolin-1-one;
5-(3-chloro-4-(2-chloro-3-(6-methoxy-5-(((((R)-5-oxopyrrolidin-2-yl)methyl)amino)methyl)pyridin-2-yl)phenyl)pyridin-2-yl)-2-(2-((((R)-5-oxopyrrolidin-2-yl)methyl)amino)ethyl)isoindolin-1-one;
(S)-5-((((6-(2-chloro-3-(3-chloro-2-(8-chloro-2-(((S)-5-oxopyrrolidin-2-yl)methyl)-1,2,3,4-tetrahydroisoquinolin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(8-chloro-2-(((R)-5-oxopyrrolidin-2-yl)methyl)-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(8-chloro-2-(((S)-5-oxopyrrolidin-2-yl)methyl)-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(8-chloro-2-(((R)-5-oxopyrrolidin-2-yl)methyl)-1,2,3,4-tetrahydroisoquinolin-6-yl)pyridin-4-yl)phenyl)-2-methoxypyridin-3-yl)methyl)amino)methyl)pyrrolidin-2-one;
isopropyl (4-(3-chloro-4-(2-chloro-3-(6-methoxy-5-(((((S)-5-oxopyrrolidin-2-yl)methyl)amino)methyl)pyridin-2-yl)phenyl)pyridin-2-yl)-2-methoxybenzyl)-L-prolinate;
isopropyl (4-(3-chloro-4-(2-chloro-3-(6-methoxy-5-(((((S)-5-oxopyrrolidin-2-yl)methyl)amino)methyl)pyridin-2-yl)phenyl)pyridin-2-yl)-2-methoxybenzyl)-D-prolinate;
isopropyl (4-(3-chloro-4-(2-chloro-3-(6-methoxy-5-(((((R)-5-oxopyrrolidin-2-yl)methyl)amino)methyl)pyridin-2-yl)phenyl)pyridin-2-yl)-2-methoxybenzyl)-L-prolinate;
isopropyl (4-(3-chloro-4-(2-chloro-3-(6-methoxy-5-(((((R)-5-oxopyrrolidin-2-yl)methyl)amino)methyl)pyridin-2-yl)phenyl)pyridin-2-yl)-2-methoxybenzyl)-D-prolinate;
(S)-5-((((6-(2-chloro-3-(3-chloro-2-(8-chloro-2-((S)-2-hydroxypropyl)-1,2,3,4-tetrahydroisoquinolin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(8-chloro-2-((R)-2-hydroxypropyl)-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(8-chloro-2-((S)-2-hydroxypropyl)-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(8-chloro-2-((R)-2-hydroxypropyl)-1,2,3,4-tetrahydroisoquinolin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(4-(((2-hydroxyethyl)amino)methyl)-3-methoxyphenyl)pyridin-4-yl)phenyl)-2-methoxypyridin-3-yl)methyl)amino)methyl)pyrrolidin-2-one;
(R)-5-((((6-(2-chloro-3-(3-chloro-2-(4-(((2-hydroxyethyl)amino)methyl)-3-methoxyphenyl)pyridin-4-yl)phenyl)-2-methoxypyridin-3-yl)methyl)amino)methyl)pyrrolidin-2-one;
(S)-5-((((4-chloro-6-(2-chloro-3-(3-chloro-2-(4-((((S)-2-hydroxypropyl)(methyl)amino)methyl)-3-methoxyphenyl)pyridin-4-yl)phenyl)-2-methoxypyridin-3-yl)methyl)amino)methyl)pyrrolidin-2-one;
(S)-5-((((4-chloro-6-(2-chloro-3-(3-chloro-2-(4-((((R)-2-hydroxypropyl)(methyl)amino)methyl)-3-methoxyphenyl)pyridin-4-yl)phenyl)-2-methoxypyridin-3-yl)methyl)amino)methyl)pyrrolidin-2-one;
(R)-5-((((4-chloro-6-(2-chloro-3-(3-chloro-2-(4-((((S)-2-hydroxypropyl)(methyl)amino)methyl)-3-methoxyphenyl)pyridin-4-yl)phenyl)-2-methoxypyridin-3-yl)methyl)amino)methyl)pyrrolidin-2-one;
(R)-5-((((4-chloro-6-(2-chloro-3-(3-chloro-2-(4-((((R)-2-hydroxypropyl)(methyl)amino)methyl)-3-methoxyphenyl)pyridin-4-yl)phenyl)-2-methoxypyridin-3-yl)methyl)amino)methyl)pyrrolidin-2-one;
(S)-5-((((6-(2-chloro-3-(3-chloro-2-(8-chloro-2-(3-fluoropropyl)-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(8-chloro-2-(3-fluoropropyl)-1,2,3,4-tetrahydroisoquinolin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(3-(((4,4-difluorocyclohexyl)amino)methyl)-1-methyl-1H-indol-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(3-(((4,4-difluorocyclohexyl)amino)methyl)-1-methyl-1H-indol-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(2-(2-(1-hydroxycyclopropyl)ethyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-(2-(1-hydroxycyclopropyl)ethyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(4-((((S)-2-hydroxypropyl)(methyl)amino)methyl)-3-methoxyphenyl)pyridin-4-yl)phenyl)-2-methoxypyridin-3-yl)methyl)amino)methyl)pyrrolidin-2-one;
(S)-5-((((6-(2-chloro-3-(3-chloro-2-(4-((((R)-2-hydroxypropyl)(methyl)amino)methyl)-3-methoxyphenyl)pyridin-4-yl)phenyl)-2-methoxypyridin-3-yl)methyl)amino)methyl)pyrrolidin-2-one;
(R)-5-((((6-(2-chloro-3-(3-chloro-2-(4-((((S)-2-hydroxypropyl)(methyl)amino)methyl)-3-methoxyphenyl)pyridin-4-yl)phenyl)-2-methoxypyridin-3-yl)methyl)amino)methyl)pyrrolidin-2-one;
(R)-5-((((6-(2-chloro-3-(3-chloro-2-(4-((((R)-2-hydroxypropyl)(methyl)amino)methyl)-3-methoxyphenyl)pyridin-4-yl)phenyl)-2-methoxypyridin-3-yl)methyl)amino)methyl)pyrrolidin-2-one;
isopropyl (S)-1-(4-(3-chloro-4-(2-chloro-3-(6-methoxy-5-(((((S)-5-oxopyrrolidin-2-yl)methyl)amino)methyl)pyridin-2-yl)phenyl)pyridin-2-yl)-2-methoxybenzyl)pyrrolidine-3-carboxylate;
isopropyl (S)-1-(4-(3-chloro-4-(2-chloro-3-(6-methoxy-5-(((((R)-5-oxopyrrolidin-2-yl)methyl)amino)methyl)pyridin-2-yl)phenyl)pyridin-2-yl)-2-methoxybenzyl)pyrrolidine-3-carboxylate;
isopropyl (R)-1-(4-(3-chloro-4-(2-chloro-3-(6-methoxy-5-(((((S)-5-oxopyrrolidin-2-yl)methyl)amino)methyl)pyridin-2-yl)phenyl)pyridin-2-yl)-2-methoxybenzyl)pyrrolidine-3-carboxylate;
isopropyl (R)-1-(4-(3-chloro-4-(2-chloro-3-(6-methoxy-5-(((((R)-5-oxopyrrolidin-2-yl)methyl)amino)methyl)pyridin-2-yl)phenyl)pyridin-2-yl)-2-methoxybenzyl)pyrrolidine-3-carboxylate;
(S)-1-(4-(3-chloro-4-(2-chloro-3-(6-methoxy-5-(((((S)-5-oxopyrrolidin-2-yl)methyl)amino)methyl)pyridin-2-yl)phenyl)pyridin-2-yl)-2-methoxybenzyl)pyrrolidine-3-carboxylic acid;
(S)-1-(4-(3-chloro-4-(2-chloro-3-(6-methoxy-5-(((((R)-5-oxopyrrolidin-2-yl)methyl)amino)methyl)pyridin-2-yl)phenyl)pyridin-2-yl)-2-methoxybenzyl)pyrrolidine-3-carboxylic acid;
(R)-1-(4-(3-chloro-4-(2-chloro-3-(6-methoxy-5-(((((S)-5-oxopyrrolidin-2-yl)methyl)amino)methyl)pyridin-2-yl)phenyl)pyridin-2-yl)-2-methoxybenzyl)pyrrolidine-3-carboxylic acid;
(R)-1-(4-(3-chloro-4-(2-chloro-3-(6-methoxy-5-(((((R)-5-oxopyrrolidin-2-yl)methyl)amino)methyl)pyridin-2-yl)phenyl)pyridin-2-yl)-2-methoxybenzyl)pyrrolidine-3-carboxylic acid;
(4-(3-chloro-4-(2-chloro-3-(6-methoxy-5-(((((S)-5-oxopyrrolidin-2-yl)methyl)amino)methyl)pyridin-2-yl)phenyl)pyridin-2-yl)-2-methoxybenzyl)-L-proline;
(4-(3-chloro-4-(2-chloro-3-(6-methoxy-5-(((((S)-5-oxopyrrolidin-2-yl)methyl)amino)methyl)pyridin-2-yl)phenyl)pyridin-2-yl)-2-methoxybenzyl)-D-proline;
(4-(3-chloro-4-(2-chloro-3-(6-methoxy-5-(((((R)-5-oxopyrrolidin-2-yl)methyl)amino)methyl)pyridin-2-yl)phenyl)pyridin-2-yl)-2-methoxybenzyl)-L-proline;
(4-(3-chloro-4-(2-chloro-3-(6-methoxy-5-(((((R)-5-oxopyrrolidin-2-yl)methyl)amino)methyl)pyridin-2-yl)phenyl)pyridin-2-yl)-2-methoxybenzyl)-D-proline;
(S)-5-((((6-(2-chloro-3-(3-chloro-2-(3-fluoro-4-((((1r,3r)-3-hydroxy-3-methylcyclobutyl)amino)methyl)-5-methoxyphenyl)pyridin-4-yl)phenyl)-2-methoxypyridin-3-yl)methyl)amino)methyl)pyrrolidin-2-one;
(R)-5-((((6-(2-chloro-3-(3-chloro-2-(3-fluoro-4-((((1r,3r)-3-hydroxy-3-methylcyclobutyl)amino)methyl)-5-methoxyphenyl)pyridin-4-yl)phenyl)-2-methoxypyridin-3-yl)methyl)amino)methyl)pyrrolidin-2-one;
(R)-5-((((6-(2-chloro-3-(3-chloro-2-(3-fluoro-4-((((1r,3s)-3-hydroxy-3-methylcyclobutyl)amino)methyl)-5-methoxyphenyl)pyridin-4-yl)phenyl)-2-methoxypyridin-3-yl)methyl)amino)methyl)pyrrolidin-2-one;
(R)-5-((((6-(2-chloro-3-(3-chloro-2-(3-fluoro-4-((((1s,3r)-3-hydroxy-3-methylcyclobutyl)amino)methyl)-5-methoxyphenyl)pyridin-4-yl)phenyl)-2-methoxypyridin-3-yl)methyl)amino)methyl)pyrrolidin-2-one;
(S)-5-((((6-(2-chloro-3-(3-chloro-2-(3-fluoro-4-((((1s,3r)-3-hydroxy-3-methylcyclobutyl)amino)methyl)-5-methoxyphenyl)pyridin-4-yl)phenyl)-2-methoxypyridin-3-yl)methyl)amino)methyl)pyrrolidin-2-one;
(S)-5-((((6-(2-chloro-3-(3-chloro-2-(3-fluoro-4-((((1r,3s)-3-hydroxy-3-methylcyclobutyl)amino)methyl)-5-methoxyphenyl)pyridin-4-yl)phenyl)-2-methoxypyridin-3-yl)methyl)amino)methyl)pyrrolidin-2-one;
(R)-5-((((6-(2-chloro-3-(3-chloro-2-(3-fluoro-4-((((1s,3s)-3-hydroxy-3-methylcyclobutyl)amino)methyl)-5-methoxyphenyl)pyridin-4-yl)phenyl)-2-methoxypyridin-3-yl)methyl)amino)methyl)pyrrolidin-2-one;
(S)-5-((((6-(2-chloro-3-(3-chloro-2-(3-fluoro-4-((((1s,3s)-3-hydroxy-3-methylcyclobutyl)amino)methyl)-5-methoxyphenyl)pyridin-4-yl)phenyl)-2-methoxypyridin-3-yl)methyl)amino)methyl)pyrrolidin-2-one;
(S)-5-((((6-(2-chloro-3-(3-chloro-2-(3-fluoro-4-((((S)-2-hydroxypropyl)amino)methyl)-5-methoxyphenyl)pyridin-4-yl)phenyl)-2-methoxypyridin-3-yl)methyl)amino)methyl)pyrrolidin-2-one;
(R)-5-((((6-(2-chloro-3-(3-chloro-2-(3-fluoro-4-((((S)-2-hydroxypropyl)amino)methyl)-5-methoxyphenyl)pyridin-4-yl)phenyl)-2-methoxypyridin-3-yl)methyl)amino)methyl)pyrrolidin-2-one;
(R)-5-((((6-(2-chloro-3-(3-chloro-2-(3-fluoro-4-((((R)-2-hydroxypropyl)amino)methyl)-5-methoxyphenyl)pyridin-4-yl)phenyl)-2-methoxypyridin-3-yl)methyl)amino)methyl)pyrrolidin-2-one;
(S)-5-((((6-(2-chloro-3-(3-chloro-2-(3-fluoro-4-((((R)-2-hydroxypropyl)amino)methyl)-5-methoxyphenyl)pyridin-4-yl)phenyl)-2-methoxypyridin-3-yl)methyl)amino)methyl)pyrrolidin-2-one;
(S)-5-((((6-(2-chloro-3-(3-chloro-2-(3-fluoro-4-((isopropylamino)methyl)-5-methoxyphenyl)pyridin-4-yl)phenyl)-2-methoxypyridin-3-yl)methyl)amino)methyl)pyrrolidin-2-one;
(R)-5-((((6-(2-chloro-3-(3-chloro-2-(3-fluoro-4-((isopropylamino)methyl)-5-methoxyphenyl)pyridin-4-yl)phenyl)-2-methoxypyridin-3-yl)methyl)amino)methyl)pyrrolidin-2-one;
(S)-5-((((6-(2-chloro-3-(3-chloro-2-(3-fluoro-4-((isobutylamino)methyl)-5-methoxyphenyl)pyridin-4-yl)phenyl)-2-methoxypyridin-3-yl)methyl)amino)methyl)pyrrolidin-2-one;
(R)-5-((((6-(2-chloro-3-(3-chloro-2-(3-fluoro-4-((isobutylamino)methyl)-5-methoxyphenyl)pyridin-4-yl)phenyl)-2-methoxypyridin-3-yl)methyl)amino)methyl)pyrrolidin-2-one;
2-(((6-(2-chloro-3-(3-chloro-2-(2-(3-fluoropropyl)-8-methoxy-1,2,3,4-tetrahydroisoquinolin-6-yl)pyridin-4-yl)phenyl)-2-methoxypyridin-3-yl)methyl)amino)ethan-1-ol;
2-(((6-(2-chloro-3-(3-chloro-2-(8-methoxy-2-(2-(piperidin-4-yl)ethyl)-1,2,3,4-tetrahydroisoquinolin-6-yl)pyridin-4-yl)phenyl)-2-methoxypyridin-3-yl)methyl)amino)ethan-1-ol;
(S)-5-((((4-chloro-6-(2-chloro-3-(3-chloro-2-(2-((S)-2-hydroxypropyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(S)-5-((((4-chloro-6-(2-chloro-3-(3-chloro-2-(2-((R)-2-hydroxypropyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((4-chloro-6-(2-chloro-3-(3-chloro-2-(2-((S)-2-hydroxypropyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((4-chloro-6-(2-chloro-3-(3-chloro-2-(2-((R)-2-hydroxypropyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
6-(3-chloro-4-(2-chloro-3-(4-chloro-6-methoxy-5-(((((S)-5-oxopyrrolidin-2-yl)methyl)amino)methyl)pyridin-2-yl)phenyl)pyridin-2-yl)-2-((((1r,3r)-3-hydroxy-3-methylcyclobutyl)amino)methyl)-3-methylpyrrolo[2,1-f][1,2,4]triazin-4(3H)-one;
6-(3-chloro-4-(2-chloro-3-(4-chloro-6-methoxy-5-(((((R)-5-oxopyrrolidin-2-yl)methyl)amino)methyl)pyridin-2-yl)phenyl)pyridin-2-yl)-2-((((1r,3s)-3-hydroxy-3-methylcyclobutyl)amino)methyl)-3-methylpyrrolo[2,1-f][1,2,4]triazin-4(3H)-one;
6-(3-chloro-4-(2-chloro-3-(4-chloro-6-methoxy-5-(((((R)-5-oxopyrrolidin-2-yl)methyl)amino)methyl)pyridin-2-yl)phenyl)pyridin-2-yl)-2-((((1s,3r)-3-hydroxy-3-methylcyclobutyl)amino)methyl)-3-methylpyrrolo[2,1-f][1,2,4]triazin-4(3H)-one;
6-(3-chloro-4-(2-chloro-3-(4-chloro-6-methoxy-5-(((((S)-5-oxopyrrolidin-2-yl)methyl)amino)methyl)pyridin-2-yl)phenyl)pyridin-2-yl)-2-((((1s,3r)-3-hydroxy-3-methylcyclobutyl)amino)methyl)-3-methylpyrrolo[2,1-f][1,2,4]triazin-4(3H)-one;
6-(3-chloro-4-(2-chloro-3-(4-chloro-6-methoxy-5-(((((S)-5-oxopyrrolidin-2-yl)methyl)amino)methyl)pyridin-2-yl)phenyl)pyridin-2-yl)-2-((((1r,3s)-3-hydroxy-3-methylcyclobutyl)amino)methyl)-3-methylpyrrolo[2,1-f][1,2,4]triazin-4(3H)-one;
6-(3-chloro-4-(2-chloro-3-(4-chloro-6-methoxy-5-(((((R)-5-oxopyrrolidin-2-yl)methyl)amino)methyl)pyridin-2-yl)phenyl)pyridin-2-yl)-2-((((1s,3s)-3-hydroxy-3-methylcyclobutyl)amino)methyl)-3-methylpyrrolo[2,1-f][1,2,4]triazin-4(3H)-one;
6-(3-chloro-4-(2-chloro-3-(4-chloro-6-methoxy-5-(((((R)-5-oxopyrrolidin-2-yl)methyl)amino)methyl)pyridin-2-yl)phenyl)pyridin-2-yl)-2-((((1r,3r)-3-hydroxy-3-methylcyclobutyl)amino)methyl)-3-methylpyrrolo[2,1-f][1,2,4]triazin-4(3H)-one;
6-(3-chloro-4-(2-chloro-3-(4-chloro-6-methoxy-5-(((((S)-5-oxopyrrolidin-2-yl)methyl)amino)methyl)pyridin-2-yl)phenyl)pyridin-2-yl)-2-((((1s,3s)-3-hydroxy-3-methylcyclobutyl)amino)methyl)-3-methylpyrrolo[2,1-f][1,2,4]triazin-4(3H)-one;
(S)-2-(4-(3-chloro-4-(2-chloro-3-(6-methoxy-5-((((5-oxopyrrolidin-2-yl)methyl)amino)methyl)pyridin-2-yl)phenyl)pyridin-2-yl)-2-methoxybenzyl)-2,6-diazaspiro[3.4]octan-7-one;
(R)-2-(4-(3-chloro-4-(2-chloro-3-(6-methoxy-5-((((5-oxopyrrolidin-2-yl)methyl)amino)methyl)pyridin-2-yl)phenyl)pyridin-2-yl)-2-methoxybenzyl)-2,6-diazaspiro[3.4]octan-7-one;
2-(4-(3-chloro-4-(2-chloro-3-(6-methoxy-5-((7-oxo-2,6-diazaspiro[3.4]octan-2-yl)methyl)pyridin-2-yl)phenyl)pyridin-2-yl)-2-methoxybenzyl)-2,6-diazaspiro[3.4]octan-7-one;
1-(6-((6-(3-(2-(4-((6-acetyl-2,6-diazaspiro[3.3]heptan-2-yl)methyl)-3-methoxyphenyl)-3-chloropyridin-4-yl)-2-chlorophenyl)-2-methoxypyridin-3-yl)methyl)-2,6-diazaspiro[3.3]heptan-2-yl)ethan-1-one;
6-(3-chloro-4-(2-chloro-3-(6-methoxy-5-(((((S)-5-oxopyrrolidin-2-yl)methyl)amino)methyl)pyridin-2-yl)phenyl)pyridin-2-yl)-2-((((S)-2-hydroxypropyl)amino)methyl)-3-methylpyrrolo[2,1-f][1,2,4]triazin-4(3H)-one;
6-(3-chloro-4-(2-chloro-3-(6-methoxy-5-(((((S)-5-oxopyrrolidin-2-yl)methyl)amino)methyl)pyridin-2-yl)phenyl)pyridin-2-yl)-2-((((R)-2-hydroxypropyl)amino)methyl)-3-methylpyrrolo[2,1-f][1,2,4]triazin-4(3H)-one;
6-(3-chloro-4-(2-chloro-3-(6-methoxy-5-(((((R)-5-oxopyrrolidin-2-yl)methyl)amino)methyl)pyridin-2-yl)phenyl)pyridin-2-yl)-2-((((S)-2-hydroxypropyl)amino)methyl)-3-methylpyrrolo[2,1-f][1,2,4]triazin-4(3H)-one;
6-(3-chloro-4-(2-chloro-3-(6-methoxy-5-(((((R)-5-oxopyrrolidin-2-yl)methyl)amino)methyl)pyridin-2-yl)phenyl)pyridin-2-yl)-2-((((R)-2-hydroxypropyl)amino)methyl)-3-methylpyrrolo[2,1-f][1,2,4]triazin-4(3H)-one;
(S)-5-(((4-(4-(3-(3-(((1-acetylpiperidin-4-yl)(methyl)amino)methyl)-1-methyl-1H-pyrrolo[2,3-b]pyridin-6-yl)-2-chlorophenyl)-3-chloropyridin-2-yl)-2-methoxybenzyl)amino)methyl)pyrrolidin-2-one;
(R)-5-(((4-(4-(3-(3-(((1-acetylpiperidin-4-yl)(methyl)amino)methyl)-1-methyl-1H-pyrrolo[2,3-b]pyridin-6-yl)-2-chlorophenyl)-3-chloropyridin-2-yl)-2-methoxybenzyl)amino)methyl)pyrrolidin-2-one;
(S)-5-(((4-(3-chloro-4-(2-chloro-3-(1-methyl-3-(((((S)-tetrahydrofuran-2-yl)methyl)amino)methyl)-1H-pyrrolo[2,3-b]pyridin-6-yl)phenyl)pyridin-2-yl)-2-methoxybenzyl)amino)methyl)pyrrolidin-2-one;
(S)-5-(((4-(3-chloro-4-(2-chloro-3-(1-methyl-3-(((((R)-tetrahydrofuran-2-yl)methyl)amino)methyl)-1H-pyrrolo[2,3-b]pyridin-6-yl)phenyl)pyridin-2-yl)-2-methoxybenzyl)amino)methyl)pyrrolidin-2-one;
(R)-5-(((4-(3-chloro-4-(2-chloro-3-(1-methyl-3-(((((S)-tetrahydrofuran-2-yl)methyl)amino)methyl)-1H-pyrrolo[2,3-b]pyridin-6-yl)phenyl)pyridin-2-yl)-2-methoxybenzyl)amino)methyl)pyrrolidin-2-one;
(R)-5-(((4-(3-chloro-4-(2-chloro-3-(1-methyl-3-(((((R)-tetrahydrofuran-2-yl)methyl)amino)methyl)-1H-pyrrolo[2,3-b]pyridin-6-yl)phenyl)pyridin-2-yl)-2-methoxybenzyl)amino)methyl)pyrrolidin-2-one;
(S)-5-(((4-(3-chloro-4-(2-chloro-3-(1-methyl-3-((methylamino)methyl)-1H-pyrrolo[2,3-b]pyridin-6-yl)phenyl)pyridin-2-yl)-2-methoxybenzyl)amino)methyl)pyrrolidin-2-one;
(R)-5-(((4-(3-chloro-4-(2-chloro-3-(1-methyl-3-((methylamino)methyl)-1H-pyrrolo[2,3-b]pyridin-6-yl)phenyl)pyridin-2-yl)-2-methoxybenzyl)amino)methyl)pyrrolidin-2-one;
(S)-5-(((4-(4-(3-(3-(((1-acetylazetidin-3-yl)amino)methyl)-1-methyl-1H-pyrrolo[2,3-b]pyridin-6-yl)-2-chlorophenyl)-3-chloropyridin-2-yl)-2-methoxybenzyl)amino)methyl)pyrrolidin-2-one;
(R)-5-(((4-(4-(3-(3-(((1-acetylazetidin-3-yl)amino)methyl)-1-methyl-1H-pyrrolo[2,3-b]pyridin-6-yl)-2-chlorophenyl)-3-chloropyridin-2-yl)-2-methoxybenzyl)amino)methyl)pyrrolidin-2-one;
(S)-5-(((4-(3-chloro-4-(2-chloro-3-(3-(((4,4-difluorocyclohexyl)amino)methyl)-1-methyl-1H-pyrrolo[2,3-b]pyridin-6-yl)phenyl)pyridin-2-yl)-2-methoxybenzyl)amino)methyl)pyrrolidin-2-one;
(R)-5-(((4-(3-chloro-4-(2-chloro-3-(3-(((4,4-difluorocyclohexyl)amino)methyl)-1-methyl-1H-pyrrolo[2,3-b]pyridin-6-yl)phenyl)pyridin-2-yl)-2-methoxybenzyl)amino)methyl)pyrrolidin-2-one;
(S)-5-(((4-(3-chloro-4-(2-chloro-3-(3-(((2-hydroxyethyl)amino)methyl)-1-methyl-1H-pyrrolo[2,3-b]pyridin-6-yl)phenyl)pyridin-2-yl)-2-methoxybenzyl)amino)methyl)pyrrolidin-2-one;
(R)-5-(((4-(3-chloro-4-(2-chloro-3-(3-(((2-hydroxyethyl)amino)methyl)-1-methyl-1H-pyrrolo[2,3-b]pyridin-6-yl)phenyl)pyridin-2-yl)-2-methoxybenzyl)amino)methyl)pyrrolidin-2-one;
(S)-5-(((4-(3-chloro-4-(2-chloro-3-(3-((((S)-2-hydroxypropyl)amino)methyl)-1-methyl-1H-pyrrolo[2,3-b]pyridin-6-yl)phenyl)pyridin-2-yl)-2-methoxybenzyl)amino)methyl)pyrrolidin-2-one;
(S)-5-(((4-(3-chloro-4-(2-chloro-3-(3-((((R)-2-hydroxypropyl)amino)methyl)-1-methyl-1H-pyrrolo[2,3-b]pyridin-6-yl)phenyl)pyridin-2-yl)-2-methoxybenzyl)amino)methyl)pyrrolidin-2-one;
(R)-5-(((4-(3-chloro-4-(2-chloro-3-(3-((((S)-2-hydroxypropyl)amino)methyl)-1-methyl-1H-pyrrolo[2,3-b]pyridin-6-yl)phenyl)pyridin-2-yl)-2-methoxybenzyl)amino)methyl)pyrrolidin-2-one;
(R)-5-(((4-(3-chloro-4-(2-chloro-3-(3-((((R)-2-hydroxypropyl)amino)methyl)-1-methyl-1H-pyrrolo[2,3-b]pyridin-6-yl)phenyl)pyridin-2-yl)-2-methoxybenzyl)amino)methyl)pyrrolidin-2-one;
(S)-5-(((4-(3-chloro-4-(2-chloro-3-(3-(((2-hydroxy-2-methylpropyl)amino)methyl)-1-methyl-1H-pyrrolo[2,3-b]pyridin-6-yl)phenyl)pyridin-2-yl)-2-methoxybenzyl)amino)methyl)pyrrolidin-2-one;
(R)-5-(((4-(3-chloro-4-(2-chloro-3-(3-(((2-hydroxy-2-methylpropyl)amino)methyl)-1-methyl-1H-pyrrolo[2,3-b]pyridin-6-yl)phenyl)pyridin-2-yl)-2-methoxybenzyl)amino)methyl)pyrrolidin-2-one;
(S)-5-(((4-(3-chloro-4-(2-chloro-3-(3-(((2-methoxyethyl)amino)methyl)-1-methyl-1H-pyrrolo[2,3-b]pyridin-6-yl)phenyl)pyridin-2-yl)-2-methoxybenzyl)amino)methyl)pyrrolidin-2-one;
(R)-5-(((4-(3-chloro-4-(2-chloro-3-(3-(((2-methoxyethyl)amino)methyl)-1-methyl-1H-pyrrolo[2,3-b]pyridin-6-yl)phenyl)pyridin-2-yl)-2-methoxybenzyl)amino)methyl)pyrrolidin-2-one;
(S)-5-(((4-(3-chloro-4-(2-chloro-3-(3-(((2-hydroxyethyl)(methyl)amino)methyl)-1-methyl-1H-pyrrolo[2,3-b]pyridin-6-yl)phenyl)pyridin-2-yl)-2-methoxybenzyl)amino)methyl)pyrrolidin-2-one;
(R)-5-(((4-(3-chloro-4-(2-chloro-3-(3-(((2-hydroxyethyl)(methyl)amino)methyl)-1-methyl-1H-pyrrolo[2,3-b]pyridin-6-yl)phenyl)pyridin-2-yl)-2-methoxybenzyl)amino)methyl)pyrrolidin-2-one;
(S)-6-(3-chloro-4-(2-chloro-3-(6-methoxy-5-((((5-oxopyrrolidin-2-yl)methyl)amino)methyl)pyridin-2-yl)phenyl)pyridin-2-yl)-2-(((3-hydroxy-3-methylcyclobutyl)amino)methyl)-3-methylpyrrolo[2,1-f][1,2,4]triazin-4(3H)-one;
(R)-6-(3-chloro-4-(2-chloro-3-(6-methoxy-5-((((5-oxopyrrolidin-2-yl)methyl)amino)methyl)pyridin-2-yl)phenyl)pyridin-2-yl)-2-(((3-hydroxy-3-methylcyclobutyl)amino)methyl)-3-methylpyrrolo[2,1-f][1,2,4]triazin-4(3H)-one;
6-(3-chloro-4-(2-chloro-3-(6-methoxy-5-(((((S)-5-oxopyrrolidin-2-yl)methyl)amino)methyl)pyridin-2-yl)phenyl)pyridin-2-yl)-3-methyl-2-(((((S)-5-oxopyrrolidin-2-yl)methyl)amino)methyl)pyrrolo[2,1-f][1,2,4]triazin-4(3H)-one;
6-(3-chloro-4-(2-chloro-3-(6-methoxy-5-(((((S)-5-oxopyrrolidin-2-yl)methyl)amino)methyl)pyridin-2-yl)phenyl)pyridin-2-yl)-3-methyl-2-(((((R)-5-oxopyrrolidin-2-yl)methyl)amino)methyl)pyrrolo[2,1-f][1,2,4]triazin-4(3H)-one;
6-(3-chloro-4-(2-chloro-3-(6-methoxy-5-(((((R)-5-oxopyrrolidin-2-yl)methyl)amino)methyl)pyridin-2-yl)phenyl)pyridin-2-yl)-3-methyl-2-(((((S)-5-oxopyrrolidin-2-yl)methyl)amino)methyl)pyrrolo[2,1-f][1,2,4]triazin-4(3H)-one;
6-(3-chloro-4-(2-chloro-3-(6-methoxy-5-(((((R)-5-oxopyrrolidin-2-yl)methyl)amino)methyl)pyridin-2-yl)phenyl)pyridin-2-yl)-3-methyl-2-(((((R)-5-oxopyrrolidin-2-yl)methyl)amino)methyl)pyrrolo[2,1-f][1,2,4]triazin-4(3H)-one;
(S)-5-((((4-chloro-6-(2-chloro-3-(3-chloro-2-(2-((R)-2-hydroxypropyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(S)-5-((((4-chloro-6-(2-chloro-3-(3-chloro-2-(2-((S)-2-hydroxypropyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((4-chloro-6-(2-chloro-3-(3-chloro-2-(2-((R)-2-hydroxypropyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((4-chloro-6-(2-chloro-3-(3-chloro-2-(2-((S)-2-hydroxypropyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(S)-(4-(3-chloro-4-(2-chloro-3-(4-chloro-6-methoxy-5-((((5-oxopyrrolidin-2-yl)methyl)amino)methyl)pyridin-2-yl)phenyl)pyridin-2-yl)-2-methoxybenzyl)glycine;
(R)-(4-(3-chloro-4-(2-chloro-3-(4-chloro-6-methoxy-5-((((5-oxopyrrolidin-2-yl)methyl)amino)methyl)pyridin-2-yl)phenyl)pyridin-2-yl)-2-methoxybenzyl)glycine;
3-chloro-4-(2-chloro-3-(5-(4,5-dihydro-1H-imidazol-2-yl)-6-methoxypyridin-2-yl)phenyl)-2-(4-(4,5-dihydro-1H-imidazol-2-yl)-3-methoxyphenyl)pyridine;
(S)-(4-(3-chloro-4-(2-chloro-3-(6-methoxy-5-((((5-oxopyrrolidin-2-yl)methyl)amino)methyl)pyridin-2-yl)phenyl)pyridin-2-yl)-2-methoxybenzyl)glycine;
(R)-(4-(3-chloro-4-(2-chloro-3-(6-methoxy-5-((((5-oxopyrrolidin-2-yl)methyl)amino)methyl)pyridin-2-yl)phenyl)pyridin-2-yl)-2-methoxybenzyl)glycine;
isopropyl (S)-(4-(3-chloro-4-(2-chloro-3-(6-methoxy-5-((((5-oxopyrrolidin-2-yl)methyl)amino)methyl)pyridin-2-yl)phenyl)pyridin-2-yl)-2-methoxybenzyl)glycinate;
isopropyl (R)-(4-(3-chloro-4-(2-chloro-3-(6-methoxy-5-((((5-oxopyrrolidin-2-yl)methyl)amino)methyl)pyridin-2-yl)phenyl)pyridin-2-yl)-2-methoxybenzyl)glycinate;
(S)-5-((((6-(3-(2-(4-(((1-acetylpiperidin-4-yl)amino)methyl)-3-methoxyphenyl)-3-chloropyridin-4-yl)-2-chlorophenyl)-2-methoxypyridin-3-yl)methyl)amino)methyl)pyrrolidin-2-one;
(R)-5-((((6-(3-(2-(4-(((1-acetylpiperidin-4-yl)amino)methyl)-3-methoxyphenyl)-3-chloropyridin-4-yl)-2-chlorophenyl)-2-methoxypyridin-3-yl)methyl)amino)methyl)pyrrolidin-2-one;
(S)-6-(3-chloro-4-(2-chloro-3-(6-methoxy-5-((((5-oxopyrrolidin-2-yl)methyl)amino)methyl)pyridin-2-yl)phenyl)pyridin-2-yl)-2-((3-hydroxy-3-methylazetidin-1-yl)methyl)-3-methylpyrrolo[2,1-f][1,2,4]triazin-4(3H)-one;
(R)-6-(3-chloro-4-(2-chloro-3-(6-methoxy-5-((((5-oxopyrrolidin-2-yl)methyl)amino)methyl)pyridin-2-yl)phenyl)pyridin-2-yl)-2-((3-hydroxy-3-methylazetidin-1-yl)methyl)-3-methylpyrrolo[2,1-f][1,2,4]triazin-4(3H)-one;
(S)-6-(3-chloro-4-(2-chloro-3-(6-methoxy-5-((((5-oxopyrrolidin-2-yl)methyl)amino)methyl)pyridin-2-yl)phenyl)pyridin-2-yl)-2-((isopropylamino)methyl)-3-methylpyrrolo[2,1-f][1,2,4]triazin-4(3H)-one;
(R)-6-(3-chloro-4-(2-chloro-3-(6-methoxy-5-((((5-oxopyrrolidin-2-yl)methyl)amino)methyl)pyridin-2-yl)phenyl)pyridin-2-yl)-2-((isopropylamino)methyl)-3-methylpyrrolo[2,1-f][1,2,4]triazin-4(3H)-one;
(5S)-5-((((6-(2-chloro-3-(3-chloro-2-(5-((((S)-5-oxopyrrolidin-2-yl)methyl)amino)-5,6,7,8-tetrahydronaphthalen-2-yl)pyridin-4-yl)phenyl)-2-methoxypyridin-3-yl)methyl)amino)methyl)pyrrolidin-2-one;
(5S)-5-((((6-(2-chloro-3-(3-chloro-2-(5-((((R)-5-oxopyrrolidin-2-yl)methyl)amino)-5,6,7,8-tetrahydronaphthalen-2-yl)pyridin-4-yl)phenyl)-2-methoxypyridin-3-yl)methyl)amino)methyl)pyrrolidin-2-one;
(5R)-5-((((6-(2-chloro-3-(3-chloro-2-(5-((((S)-5-oxopyrrolidin-2-yl)methyl)amino)-5,6,7,8-tetrahydronaphthalen-2-yl)pyridin-4-yl)phenyl)-2-methoxypyridin-3-yl)methyl)amino)methyl)pyrrolidin-2-one;
(5R)-5-((((6-(2-chloro-3-(3-chloro-2-(5-((((R)-5-oxopyrrolidin-2-yl)methyl)amino)-5,6,7,8-tetrahydronaphthalen-2-yl)pyridin-4-yl)phenyl)-2-methoxypyridin-3-yl)methyl)amino)methyl)pyrrolidin-2-one;
(5S)-5-((((6-(2-chloro-3-(3-chloro-2-(5-(((S)-2-hydroxypropyl)amino)-5,6,7,8-tetrahydronaphthalen-2-yl)pyridin-4-yl)phenyl)-2-methoxypyridin-3-yl)methyl)amino)methyl)pyrrolidin-2-one;
(5S)-5-((((6-(2-chloro-3-(3-chloro-2-(5-(((R)-2-hydroxypropyl)amino)-5,6,7,8-tetrahydronaphthalen-2-yl)pyridin-4-yl)phenyl)-2-methoxypyridin-3-yl)methyl)amino)methyl)pyrrolidin-2-one;
(5R)-5-((((6-(2-chloro-3-(3-chloro-2-(5-(((S)-2-hydroxypropyl)amino)-5,6,7,8-tetrahydronaphthalen-2-yl)pyridin-4-yl)phenyl)-2-methoxypyridin-3-yl)methyl)amino)methyl)pyrrolidin-2-one;
(5R)-5-((((6-(2-chloro-3-(3-chloro-2-(5-(((R)-2-hydroxypropyl)amino)-5,6,7,8-tetrahydronaphthalen-2-yl)pyridin-4-yl)phenyl)-2-methoxypyridin-3-yl)methyl)amino)methyl)pyrrolidin-2-one;
(S)-5-((((6-(2-chloro-3-(3-chloro-2-(4-((((S)-2-hydroxypropyl)amino)methyl)-3-methoxy-5-methylphenyl)pyridin-4-yl)phenyl)-2-methoxypyridin-3-yl)methyl)amino)methyl)pyrrolidin-2-one;
(S)-5-((((6-(2-chloro-3-(3-chloro-2-(4-((((R)-2-hydroxypropyl)amino)methyl)-3-methoxy-5-methylphenyl)pyridin-4-yl)phenyl)-2-methoxypyridin-3-yl)methyl)amino)methyl)pyrrolidin-2-one;
(R)-5-((((6-(2-chloro-3-(3-chloro-2-(4-((((S)-2-hydroxypropyl)amino)methyl)-3-methoxy-5-methylphenyl)pyridin-4-yl)phenyl)-2-methoxypyridin-3-yl)methyl)amino)methyl)pyrrolidin-2-one;
(R)-5-((((6-(2-chloro-3-(3-chloro-2-(4-((((R)-2-hydroxypropyl)amino)methyl)-3-methoxy-5-methylphenyl)pyridin-4-yl)phenyl)-2-methoxypyridin-3-yl)methyl)amino)methyl)pyrrolidin-2-one;
(S)-5-((((6-(2-chloro-3-(3-chloro-2-(3-methoxy-5-methyl-4-(((((S)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
(S)-5-((((6-(2-chloro-3-(3-chloro-2-(3-methoxy-5-methyl-4-(((((R)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
(R)-5-((((6-(2-chloro-3-(3-chloro-2-(3-methoxy-5-methyl-4-(((((S)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
(R)-5-((((6-(2-chloro-3-(3-chloro-2-(3-methoxy-5-methyl-4-(((((R)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
(S)-5-((((6-(2-chloro-3-(3-chloro-2-(isoindolin-5-yl)pyridin-4-yl)phenyl)-2-methoxypyridin-3-yl)methyl)amino)methyl)pyrrolidin-2-one;
(R)-5-((((6-(2-chloro-3-(3-chloro-2-(isoindolin-5-yl)pyridin-4-yl)phenyl)-2-methoxypyridin-3-yl)methyl)amino)methyl)pyrrolidin-2-one;
(S)-5-((((6-(2-chloro-3-(3-chloro-2-(3-methoxy-5-methyl-4-((methyl(((S)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
(S)-5-((((6-(2-chloro-3-(3-chloro-2-(3-methoxy-5-methyl-4-((methyl(((R)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
(R)-5-((((6-(2-chloro-3-(3-chloro-2-(3-methoxy-5-methyl-4-((methyl(((S)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
(R)-5-((((6-(2-chloro-3-(3-chloro-2-(3-methoxy-5-methyl-4-((methyl(((R)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
(S)-5-((((6-(2-chloro-3-(3-chloro-2-(4-((((S)-2-hydroxypropyl)(methyl)amino)methyl)-3-methoxy-5-methylphenyl)pyridin-4-yl)phenyl)-2-methoxypyridin-3-yl)methyl)amino)methyl)pyrrolidin-2-one;
(S)-5-((((6-(2-chloro-3-(3-chloro-2-(4-((((R)-2-hydroxypropyl)(methyl)amino)methyl)-3-methoxy-5-methylphenyl)pyridin-4-yl)phenyl)-2-methoxypyridin-3-yl)methyl)amino)methyl)pyrrolidin-2-one;
(R)-5-((((6-(2-chloro-3-(3-chloro-2-(4-((((S)-2-hydroxypropyl)(methyl)amino)methyl)-3-methoxy-5-methylphenyl)pyridin-4-yl)phenyl)-2-methoxypyridin-3-yl)methyl)amino)methyl)pyrrolidin-2-one;
(R)-5-((((6-(2-chloro-3-(3-chloro-2-(4-((((R)-2-hydroxypropyl)(methyl)amino)methyl)-3-methoxy-5-methylphenyl)pyridin-4-yl)phenyl)-2-methoxypyridin-3-yl)methyl)amino)methyl)pyrrolidin-2-one;
(S)-5-((((6-(3-(2-(1-(azetidin-3-yl)-1H-pyrazol-4-yl)-3-chloropyridin-4-yl)-2-chlorophenyl)-2-methoxypyridin-3-yl)methyl)amino)methyl)pyrrolidin-2-one;
(R)-5-((((6-(3-(2-(1-(azetidin-3-yl)-1H-pyrazol-4-yl)-3-chloropyridin-4-yl)-2-chlorophenyl)-2-methoxypyridin-3-yl)methyl)amino)methyl)pyrrolidin-2-one;
(S)-5-((((6-(3-(2-(1-(azetidin-3-ylmethyl)-1H-pyrazol-4-yl)-3-chloropyridin-4-yl)-2-chlorophenyl)-2-methoxypyridin-3-yl)methyl)amino)methyl)pyrrolidin-2-one;
(R)-5-((((6-(3-(2-(1-(azetidin-3-ylmethyl)-1H-pyrazol-4-yl)-3-chloropyridin-4-yl)-2-chlorophenyl)-2-methoxypyridin-3-yl)methyl)amino)methyl)pyrrolidin-2-one;
(S)-1-((4-(3-chloro-4-(2-chloro-3-(6-methoxy-5-((((5-oxopyrrolidin-2-yl)methyl)amino)methyl)pyridin-2-yl)phenyl)pyridin-2-yl)-2-methoxybenzyl)amino)cyclopropane-1-carboxylic acid;
(R)-1-((4-(3-chloro-4-(2-chloro-3-(6-methoxy-5-((((5-oxopyrrolidin-2-yl)methyl)amino)methyl)pyridin-2-yl)phenyl)pyridin-2-yl)-2-methoxybenzyl)amino)cyclopropane-1-carboxylic acid;
(S)-5-((((6-(2-chloro-3-(3-chloro-2-(3-methoxy-4-(((((S)-oxetan-2-yl)methyl)amino)methyl)phenyl)pyridin-4-yl)phenyl)-2-methoxypyridin-3-yl)methyl)amino)methyl)pyrrolidin-2-one;
(S)-5-((((6-(2-chloro-3-(3-chloro-2-(3-methoxy-4-(((((R)-oxetan-2-yl)methyl)amino)methyl)phenyl)pyridin-4-yl)phenyl)-2-methoxypyridin-3-yl)methyl)amino)methyl)pyrrolidin-2-one;
(R)-5-((((6-(2-chloro-3-(3-chloro-2-(3-methoxy-4-(((((S)-oxetan-2-yl)methyl)amino)methyl)phenyl)pyridin-4-yl)phenyl)-2-methoxypyridin-3-yl)methyl)amino)methyl)pyrrolidin-2-one;
(R)-5-((((6-(2-chloro-3-(3-chloro-2-(3-methoxy-4-(((((R)-oxetan-2-yl)methyl)amino)methyl)phenyl)pyridin-4-yl)phenyl)-2-methoxypyridin-3-yl)methyl)amino)methyl)pyrrolidin-2-one;
isopropyl (S)-(4-(3-chloro-4-(2-chloro-3-(4-chloro-6-methoxy-5-((((5-oxopyrrolidin-2-yl)methyl)amino)methyl)pyridin-2-yl)phenyl)pyridin-2-yl)-2-methoxybenzyl)glycinate;
isopropyl (R)-(4-(3-chloro-4-(2-chloro-3-(4-chloro-6-methoxy-5-((((5-oxopyrrolidin-2-yl)methyl)amino)methyl)pyridin-2-yl)phenyl)pyridin-2-yl)-2-methoxybenzyl)glycinate;
(S)-5-((((4-chloro-6-(2-chloro-3-(3-chloro-2-(2-(2-hydroxyethyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((4-chloro-6-(2-chloro-3-(3-chloro-2-(2-(2-hydroxyethyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
isopropyl (4-(3-chloro-4-(2-chloro-3-(6-methoxy-5-(((((S)-5-oxopyrrolidin-2-yl)methyl)amino)methyl)pyridin-2-yl)phenyl)pyridin-2-yl)-2-methoxybenzyl)-L-alaninate;
isopropyl (4-(3-chloro-4-(2-chloro-3-(6-methoxy-5-(((((S)-5-oxopyrrolidin-2-yl)methyl)amino)methyl)pyridin-2-yl)phenyl)pyridin-2-yl)-2-methoxybenzyl)-D-alaninate;
isopropyl (4-(3-chloro-4-(2-chloro-3-(6-methoxy-5-(((((R)-5-oxopyrrolidin-2-yl)methyl)amino)methyl)pyridin-2-yl)phenyl)pyridin-2-yl)-2-methoxybenzyl)-L-alaninate;
isopropyl (4-(3-chloro-4-(2-chloro-3-(6-methoxy-5-(((((R)-5-oxopyrrolidin-2-yl)methyl)amino)methyl)pyridin-2-yl)phenyl)pyridin-2-yl)-2-methoxybenzyl)-D-alaninate;
(S)-5-((((6-(2-chloro-3-(3-chloro-2-(4-(((1,1-dioxidotetrahydro-2H-thiopyran-4-yl)amino)methyl)-3-methoxyphenyl)pyridin-4-yl)phenyl)-2-methoxypyridin-3-yl)methyl)amino)methyl)pyrrolidin-2-one;
(R)-5-((((6-(2-chloro-3-(3-chloro-2-(4-(((1,1-dioxidotetrahydro-2H-thiopyran-4-yl)amino)methyl)-3-methoxyphenyl)pyridin-4-yl)phenyl)-2-methoxypyridin-3-yl)methyl)amino)methyl)pyrrolidin-2-one;
(S)-5-((((6-(2-chloro-3-(3-chloro-2-(3-methoxy-4-(((tetrahydro-2H-pyran-4-yl)amino)methyl)phenyl)pyridin-4-yl)phenyl)-2-methoxypyridin-3-yl)methyl)amino)methyl)pyrrolidin-2-one;
(R)-5-((((6-(2-chloro-3-(3-chloro-2-(3-methoxy-4-(((tetrahydro-2H-pyran-4-yl)amino)methyl)phenyl)pyridin-4-yl)phenyl)-2-methoxypyridin-3-yl)methyl)amino)methyl)pyrrolidin-2-one;
(4-(3-chloro-4-(2-chloro-3-(6-methoxy-5-(((((S)-5-oxopyrrolidin-2-yl)methyl)amino)methyl)pyridin-2-yl)phenyl)pyridin-2-yl)-2-methoxybenzyl)-L-alanine;
(4-(3-chloro-4-(2-chloro-3-(6-methoxy-5-(((((S)-5-oxopyrrolidin-2-yl)methyl)amino)methyl)pyridin-2-yl)phenyl)pyridin-2-yl)-2-methoxybenzyl)-D-alanine;
(4-(3-chloro-4-(2-chloro-3-(6-methoxy-5-(((((R)-5-oxopyrrolidin-2-yl)methyl)amino)methyl)pyridin-2-yl)phenyl)pyridin-2-yl)-2-methoxybenzyl)-L-alanine;
(4-(3-chloro-4-(2-chloro-3-(6-methoxy-5-(((((R)-5-oxopyrrolidin-2-yl)methyl)amino)methyl)pyridin-2-yl)phenyl)pyridin-2-yl)-2-methoxybenzyl)-D-alanine;
(S)-5-((((6-(2-chloro-3-(3-chloro-2-(2-((R)-2-hydroxypropyl)-8-methoxy-1,2,3,4-tetrahydroisoquinolin-6-yl)pyridin-4-yl)phenyl)-4-fluoro-2-methoxypyridin-3-yl)methyl)amino)methyl)pyrrolidin-2-one;
(S)-5-((((6-(2-chloro-3-(3-chloro-2-(2-((S)-2-hydroxypropyl)-8-methoxy-1,2,3,4-tetrahydroisoquinolin-6-yl)pyridin-4-yl)phenyl)-4-fluoro-2-methoxypyridin-3-yl)methyl)amino)methyl)pyrrolidin-2-one;
(R)-5-((((6-(2-chloro-3-(3-chloro-2-(2-((R)-2-hydroxypropyl)-8-methoxy-1,2,3,4-tetrahydroisoquinolin-6-yl)pyridin-4-yl)phenyl)-4-fluoro-2-methoxypyridin-3-yl)methyl)amino)methyl)pyrrolidin-2-one;
(R)-5-((((6-(2-chloro-3-(3-chloro-2-(2-((S)-2-hydroxypropyl)-8-methoxy-1,2,3,4-tetrahydroisoquinolin-6-yl)pyridin-4-yl)phenyl)-4-fluoro-2-methoxypyridin-3-yl)methyl)amino)methyl)pyrrolidin-2-one;
(S)-5-((((6-(2-chloro-3-(3-chloro-2-(2-(2-hydroxyethyl)-8-methoxy-1,2,3,4-tetrahydroisoquinolin-6-yl)pyridin-4-yl)phenyl)-4-fluoro-2-methoxypyridin-3-yl)methyl)amino)methyl)pyrrolidin-2-one;
(S)-5-((((6-(2-chloro-3-(3-chloro-2-(2-(2-hydroxyethyl)-8-methoxy-1,2,3,4-tetrahydroisoquinolin-6-yl)pyridin-4-yl)phenyl)-4-fluoro-2-methoxypyridin-3-yl)methyl)amino)methyl)pyrrolidin-2-one;
(S)-5-((((6-(2-chloro-3-(3-chloro-2-(2-(((1r,3r)-3-hydroxycyclobutyl)methyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(2-(((1s,3s)-3-hydroxycyclobutyl)methyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(2-(((1s,3r)-3-hydroxycyclobutyl)methyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-(((1s,3s)-3-hydroxycyclobutyl)methyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(2-(((1r,3r)-3-hydroxycyclobutyl)methyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-(((1s,3r)-3-hydroxycyclobutyl)methyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-(((1r,3s)-3-hydroxycyclobutyl)methyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-(((1r,3r)-3-hydroxycyclobutyl)methyl)-8-methoxy-1,2,3,4-tetrahydroisoquinolin-6-yl)pyridin-4-yl)phenyl)-2-methoxypyridin-3-yl)methyl)amino)methyl)pyrrolidin-2-one;
isopropyl (S)-1-((4-(3-chloro-4-(2-chloro-3-(6-methoxy-5-((((5-oxopyrrolidin-2-yl)methyl)amino)methyl)pyridin-2-yl)phenyl)pyridin-2-yl)-2-methoxybenzyl)amino)cyclopropane-1-carboxylate;
isopropyl (R)-1-((4-(3-chloro-4-(2-chloro-3-(6-methoxy-5-((((5-oxopyrrolidin-2-yl)methyl)amino)methyl)pyridin-2-yl)phenyl)pyridin-2-yl)-2-methoxybenzyl)amino)cyclopropane-1-carboxylate;
1-(6-(2-chloro-3-(3-chloro-2-(3-methoxy-4-((methylamino)methyl)phenyl)pyridin-4-yl)phenyl)-2-methoxypyridin-3-yl)-N-methylmethanamine;
1-(6-(2-chloro-3-(3-chloro-2-(4-((dimethylamino)methyl)-3-methoxyphenyl)pyridin-4-yl)phenyl)-2-methoxypyridin-3-yl)-N,N-dimethylmethanamine;
2-(((6-(2-chloro-3-(3-chloro-2-(4-(((2-hydroxyethyl)(methyl)amino)methyl)-3-methoxyphenyl)pyridin-4-yl)phenyl)-2-methoxypyridin-3-yl)methyl)(methyl)amino)ethan-1-ol;
1-(4-(((6-(3-(2-(4-(((1-acetylpiperidin-4-yl)amino)methyl)-3-methoxyphenyl)-3-chloropyridin-4-yl)-2-chlorophenyl)-2-methoxypyridin-3-yl)methyl)amino)piperidin-1-yl)ethan-1-one;
1-(4-(((6-(3-(2-(4-(((1-acetylpiperidin-4-yl)(methyl)amino)methyl)-3-methoxyphenyl)-3-chloropyridin-4-yl)-2-chlorophenyl)-2-methoxypyridin-3-yl)methyl)(methyl)amino)piperidin-1-yl)ethan-1-one;
(S)-5-((((6-(2-chloro-3-(3-chloro-2-(5-methyl-2,3,4,5-tetrahydro-1H-pyrido[4,3-b]indol-7-yl)pyridin-4-yl)phenyl)-2-methoxypyridin-3-yl)methyl)amino)methyl)pyrrolidin-2-one;
(S)-5-((((6-(2-chloro-3-(3-chloro-2-(5-methyl-2,3,4,5-tetrahydro-1H-pyrido[4,3-b]indol-7-yl)pyridin-4-yl)phenyl)-2-methoxypyridin-3-yl)methyl)amino)methyl)pyrrolidin-2-one;
5-(3-chloro-4-(2-chloro-3-(5-((((S)-2-hydroxypropyl)amino)methyl)-6-methoxypyridin-2-yl)phenyl)pyridin-2-yl)-2-(2-((((S)-5-oxopyrrolidin-2-yl)methyl)amino)ethyl)isoindolin-1-one;
5-(3-chloro-4-(2-chloro-3-(5-((((S)-2-hydroxypropyl)amino)methyl)-6-methoxypyridin-2-yl)phenyl)pyridin-2-yl)-2-(2-((((R)-5-oxopyrrolidin-2-yl)methyl)amino)ethyl)isoindolin-1-one;
5-(3-chloro-4-(2-chloro-3-(5-((((R)-2-hydroxypropyl)amino)methyl)-6-methoxypyridin-2-yl)phenyl)pyridin-2-yl)-2-(2-((((S)-5-oxopyrrolidin-2-yl)methyl)amino)ethyl)isoindolin-1-one;
5-(3-chloro-4-(2-chloro-3-(5-((((R)-2-hydroxypropyl)amino)methyl)-6-methoxypyridin-2-yl)phenyl)pyridin-2-yl)-2-(2-((((R)-5-oxopyrrolidin-2-yl)methyl)amino)ethyl)isoindolin-1-one;
(S)-5-(4-(3-(5-(((1-acetylpiperidin-4-yl)amino)methyl)-6-methoxypyridin-2-yl)-2-chlorophenyl)-3-chloropyridin-2-yl)-2-(2-(((5-oxopyrrolidin-2-yl)methyl)amino)ethyl)isoindolin-1-one;
(R)-5-(4-(3-(5-(((1-acetylpiperidin-4-yl)amino)methyl)-6-methoxypyridin-2-yl)-2-chlorophenyl)-3-chloropyridin-2-yl)-2-(2-(((5-oxopyrrolidin-2-yl)methyl)amino)ethyl)isoindolin-1-one;
(S)-5-(4-(3-(5-((6-acetyl-2,6-diazaspiro[3.3]heptan-2-yl)methyl)-6-methoxypyridin-2-yl)-2-chlorophenyl)-3-chloropyridin-2-yl)-2-(2-(((5-oxopyrrolidin-2-yl)methyl)amino)ethyl)isoindolin-1-one;
(R)-5-(4-(3-(5-((6-acetyl-2,6-diazaspiro[3.3]heptan-2-yl)methyl)-6-methoxypyridin-2-yl)-2-chlorophenyl)-3-chloropyridin-2-yl)-2-(2-(((5-oxopyrrolidin-2-yl)methyl)amino)ethyl)isoindolin-1-one;
(S)-2-(4-(3-chloro-4-(2-chloro-3-(6-methoxy-5-((((5-oxopyrrolidin-2-yl)methyl)amino)methyl)pyridin-2-yl)phenyl)pyridin-2-yl)-2-fluoro-6-methoxybenzyl)-2,6-diazaspiro[3.4]octan-7-one;
(R)-2-(4-(3-chloro-4-(2-chloro-3-(6-methoxy-5-((((5-oxopyrrolidin-2-yl)methyl)amino)methyl)pyridin-2-yl)phenyl)pyridin-2-yl)-2-fluoro-6-methoxybenzyl)-2,6-diazaspiro[3.4]octan-7-one;
(S)-5-((((6-(2-chloro-3-(3-chloro-2-(3-fluoro-4-((6-hydroxy-2-azaspiro[3.3]heptan-2-yl)methyl)-5-methoxyphenyl)pyridin-4-yl)phenyl)-2-methoxypyridin-3-yl)methyl)amino)methyl)pyrrolidin-2-one;
(R)-5-((((6-(2-chloro-3-(3-chloro-2-(3-fluoro-4-((6-hydroxy-2-azaspiro[3.3]heptan-2-yl)methyl)-5-methoxyphenyl)pyridin-4-yl)phenyl)-2-methoxypyridin-3-yl)methyl)amino)methyl)pyrrolidin-2-one;
(S)-5-((((6-(2-chloro-3-(3-chloro-2-(3-fluoro-4-(((3-fluoropropyl)amino)methyl)-5-methoxyphenyl)pyridin-4-yl)phenyl)-2-methoxypyridin-3-yl)methyl)amino)methyl)pyrrolidin-2-one;
(R)-5-((((6-(2-chloro-3-(3-chloro-2-(3-fluoro-4-(((3-fluoropropyl)amino)methyl)-5-methoxyphenyl)pyridin-4-yl)phenyl)-2-methoxypyridin-3-yl)methyl)amino)methyl)pyrrolidin-2-one;
(S)-5-((((6-(3-(2-(4-(((1-acetylpiperidin-4-yl)amino)methyl)-3-fluoro-5-methoxyphenyl)-3-chloropyridin-4-yl)-2-chlorophenyl)-2-methoxypyridin-3-yl)methyl)amino)methyl)pyrrolidin-2-one;
(R)-5-((((6-(3-(2-(4-(((1-acetylpiperidin-4-yl)amino)methyl)-3-fluoro-5-methoxyphenyl)-3-chloropyridin-4-yl)-2-chlorophenyl)-2-methoxypyridin-3-yl)methyl)amino)methyl)pyrrolidin-2-one;
methyl (S)-2-(6-(3-chloro-4-(2-chloro-3-(6-methoxy-5-((((5-oxopyrrolidin-2-yl)methyl)amino)methyl)pyridin-2-yl)phenyl)pyridin-2-yl)-8-methoxy-3,4-dihydroisoquinolin-2(1H)-yl)acetate;
methyl (R)-2-(6-(3-chloro-4-(2-chloro-3-(6-methoxy-5-((((5-oxopyrrolidin-2-yl)methyl)amino)methyl)pyridin-2-yl)phenyl)pyridin-2-yl)-8-methoxy-3,4-dihydroisoquinolin-2(1H)-yl)acetate;
(S)-2-(6-(3-chloro-4-(2-chloro-3-(6-methoxy-5-((((5-oxopyrrolidin-2-yl)methyl)amino)methyl)pyridin-2-yl)phenyl)pyridin-2-yl)-8-methoxy-3,4-dihydroisoquinolin-2(1H)-yl)acetic acid;
(R)-2-(6-(3-chloro-4-(2-chloro-3-(6-methoxy-5-((((5-oxopyrrolidin-2-yl)methyl)amino)methyl)pyridin-2-yl)phenyl)pyridin-2-yl)-8-methoxy-3,4-dihydroisoquinolin-2(1H)-yl)acetic acid;
(S)-5-((((6-(3-(2-(2-((1R,3S,4S)-2-azabicyclo[2.2.1]heptan-3-yl)-1H-benzo[d]imidazol-6-yl)-3-chloropyridin-4-yl)-2-chlorophenyl)-2-methoxypyridin-3-yl)methyl)amino)methyl)pyrrolidin-2-one;
(R)-5-((((6-(3-(2-(2-((1R,3S,4S)-2-azabicyclo[2.2.1]heptan-3-yl)-1H-benzo[d]imidazol-6-yl)-3-chloropyridin-4-yl)-2-chlorophenyl)-2-methoxypyridin-3-yl)methyl)amino)methyl)pyrrolidin-2-one;
(S)-5-((((6-(3-(2-(2-((1R,3R,4S)-2-azabicyclo[2.2.1]heptan-3-yl)-1H-benzo[d]imidazol-6-yl)-3-chloropyridin-4-yl)-2-chlorophenyl)-2-methoxypyridin-3-yl)methyl)amino)methyl)pyrrolidin-2-one;
(R)-5-((((6-(3-(2-(2-((1R,3R,4S)-2-azabicyclo[2.2.1]heptan-3-yl)-1H-benzo[d]imidazol-6-yl)-3-chloropyridin-4-yl)-2-chlorophenyl)-2-methoxypyridin-3-yl)methyl)amino)methyl)pyrrolidin-2-one;
(S)-5-((((6-(3-(2-(2-((1S,3S,4R)-2-azabicyclo[2.2.1]heptan-3-yl)-1H-benzo[d]imidazol-6-yl)-3-chloropyridin-4-yl)-2-chlorophenyl)-2-methoxypyridin-3-yl)methyl)amino)methyl)pyrrolidin-2-one;
(R)-5-((((6-(3-(2-(2-((1S,3S,4R)-2-azabicyclo[2.2.1]heptan-3-yl)-1H-benzo[d]imidazol-6-yl)-3-chloropyridin-4-yl)-2-chlorophenyl)-2-methoxypyridin-3-yl)methyl)amino)methyl)pyrrolidin-2-one;
(S)-5-((((6-(3-(2-(2-((1S,3R,4R)-2-azabicyclo[2.2.1]heptan-3-yl)-1H-benzo[d]imidazol-6-yl)-3-chloropyridin-4-yl)-2-chlorophenyl)-2-methoxypyridin-3-yl)methyl)amino)methyl)pyrrolidin-2-one;
(R)-5-((((6-(3-(2-(2-((1S,3R,4R)-2-azabicyclo[2.2.1]heptan-3-yl)-1 H-benzo[d]imidazol-6-yl)-3-chloropyridin-4-yl)-2-chlorophenyl)-2-methoxypyridin-3-yl)methyl)amino)methyl)pyrrolidin-2-one;
(S)-5-((((6-(2-chloro-3-(3-chloro-2-(3-methoxy-5-(((((S)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
(S)-5-((((6-(2-chloro-3-(3-chloro-2-(3-methoxy-5-(((((R)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
(R)-5-((((6-(2-chloro-3-(3-chloro-2-(3-methoxy-5-(((((S)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
(R)-5-((((6-(2-chloro-3-(3-chloro-2-(3-methoxy-5-(((((R)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
1-(4-(((6-(3-(2-(3-(((1-acetylpiperidin-4-yl)amino)methyl)-5-methoxyphenyl)-3-chloropyridin-4-yl)-2-chlorophenyl)-2-methoxypyridin-3-yl)methyl)amino)piperidin-1-yl)ethan-1-one;
1-(6-(2-chloro-3-(3-chloro-2-(3-methoxy-5-((methylamino)methyl)phenyl)pyridin-4-yl)phenyl)-2-methoxypyridin-3-yl)-N-methylmethanamine;
chloro-3-(3-chloro-2-(3-(((2-hydroxyethyl)amino)methyl)-5-methoxyphenyl)pyridin-4-yl)phenyl)-2-methoxypyridin-3-yl)methyl)amino)ethan-1-ol;
(S)-1-(((6-(2-chloro-3-(3-chloro-2-(3-((((S)-2-hydroxypropyl)amino)methyl)-5-methoxyphenyl)pyridin-4-yl)phenyl)-2-methoxypyridin-3-yl)methyl)amino)propan-2-ol;
(S)-1-(((6-(2-chloro-3-(3-chloro-2-(3-((((R)-2-hydroxypropyl)amino)methyl)-5-methoxyphenyl)pyridin-4-yl)phenyl)-2-methoxypyridin-3-yl)methyl)amino)propan-2-ol;
(R)-1-(((6-(2-chloro-3-(3-chloro-2-(3-((((S)-2-hydroxypropyl)amino)methyl)-5-methoxyphenyl)pyridin-4-yl)phenyl)-2-methoxypyridin-3-yl)methyl)amino)propan-2-ol;
(R)-1-(((6-(2-chloro-3-(3-chloro-2-(3-((((R)-2-hydroxypropyl)amino)methyl)-5-methoxyphenyl)pyridin-4-yl)phenyl)-2-methoxypyridin-3-yl)methyl)amino)propan-2-ol;
(S)-5-((((3'-chloro-2'-((S)-1-((5-((((S)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)methyl)pyrrolidin-2-one;
(S)-5-((((3'-chloro-2'-((S)-1-((5-((((R)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)methyl)pyrrolidin-2-one;
(S)-5-((((3'-chloro-2'-((R)-1-((5-((((S)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)methyl)pyrrolidin-2-one;
(R)-5-((((3'-chloro-2'-((S)-1-((5-((((S)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)methyl)pyrrolidin-2-one;
(S)-5-((((3'-chloro-2'-((R)-1-((5-((((R)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)methyl)pyrrolidin-2-one;
(R)-5-((((3'-chloro-2'-((S)-1-((5-((((R)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)methyl)pyrrolidin-2-one;
(R)-5-((((3'-chloro-2'-((R)-1-((5-((((S)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)methyl)pyrrolidin-2-one;
(R)-5-((((3'-chloro-2'-((R)-1-((5-((((R)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)methyl)pyrrolidin-2-one;
1-(((6-(((S)-4-(3'-chloro-6-methoxy-5-(((((S)-5-oxopyrrolidin-2-yl)methyl)amino)methyl)-[2,4'-bipyridin]-2'-yl)-2,3-dihydro-1H-inden-1-yl)oxy)-2-methoxy-5-(trifluoromethyl)pyridin-3-yl)methyl)amino)cyclopropane-1-carboxylic acid;
1-(((6-(((S)-4-(3'-chloro-6-methoxy-5-(((((R)-5-oxopyrrolidin-2-yl)methyl)amino)methyl)-[2,4'-bipyridin]-2'-yl)-2,3-dihydro-1H-inden-1-yl)oxy)-2-methoxy-5-(trifluoromethyl)pyridin-3-yl)methyl)amino)cyclopropane-1-carboxylic acid;
1-(((6-(((R)-4-(3'-chloro-6-methoxy-5-(((((S)-5-oxopyrrolidin-2-yl)methyl)amino)methyl)-[2,4'-bipyridin]-2'-yl)-2,3-dihydro-1H-inden-1-yl)oxy)-2-methoxy-5-(trifluoromethyl)pyridin-3-yl)methyl)amino)cyclopropane-1-carboxylic acid;
1-(((6-(((R)-4-(3'-chloro-6-methoxy-5-(((((R)-5-oxopyrrolidin-2-yl)methyl)amino)methyl)-[2,4'-bipyridin]-2'-yl)-2,3-dihydro-1H-inden-1-yl)oxy)-2-methoxy-5-(trifluoromethyl)pyridin-3-yl)methyl)amino)cyclopropane-1-carboxylic acid;
methyl 1-(((6-(((S)-4-(3'-chloro-6-methoxy-5-(((((S)-5-oxopyrrolidin-2-yl)methyl)amino)methyl)-[2,4'-bipyridin]-2'-yl)-2,3-dihydro-1H-inden-1-yl)amino)-2-methoxy-5-(trifluoromethyl)pyridin-3-yl)methyl)amino)cyclopropane-1-carboxylate;
methyl 1-(((6-(((S)-4-(3'-chloro-6-methoxy-5-(((((R)-5-oxopyrrolidin-2-yl)methyl)amino)methyl)-[2,4'-bipyridin]-2'-yl)-2,3-dihydro-1H-inden-1-yl)amino)-2-methoxy-5-(trifluoromethyl)pyridin-3-yl)methyl)amino)cyclopropane-1-carboxylate;
methyl 1-(((6-(((R)-4-(3'-chloro-6-methoxy-5-(((((S)-5-oxopyrrolidin-2-yl)methyl)amino)methyl)-[2,4'-bipyridin]-2'-yl)-2,3-dihydro-1H-inden-1-yl)amino)-2-methoxy-5-(trifluoromethyl)pyridin-3-yl)methyl)amino)cyclopropane-1-carboxylate;
methyl 1-(((6-(((R)-4-(3'-chloro-6-methoxy-5-(((((R)-5-oxopyrrolidin-2-yl)methyl)amino)methyl)-[2,4'-bipyridin]-2'-yl)-2,3-dihydro-1H-inden-1-yl)amino)-2-methoxy-5-(trifluoromethyl)pyridin-3-yl)methyl)amino)cyclopropane-1-carboxylate;
(S)-5-((((6-(2-chloro-3-(3-chloro-2-(2-(2-hydroxyethyl)-5-methyl-2,3,4,5-tetrahydro-1H-pyrido[4,3-b]indol-7-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-(2-hydroxyethyl)-5-methyl-2,3,4,5-tetrahydro-1H-pyrido[4,3-b]indol-7-yl)pyridin-4-yl)phenyl)-2-methoxypyridin-3-yl)methyl)amino)methyl)pyrrolidin-2-one;
1-(6-(2-chloro-3-(3-chloro-2-(isoindolin-5-yl)pyridin-4-yl)phenyl)-2-methoxypyridin-3-yl)-N-methylmethanamine;
2-(((6-(2-chloro-3-(3-chloro-2-(isoindolin-5-yl)pyridin-4-yl)phenyl)-2-methoxypyridin-3-yl)methyl)amino)ethan-1-ol;
(S)-1-(((6-(2-chloro-3-(3-chloro-2-(isoindolin-5-yl)pyridin-4-yl)phenyl)-2-methoxypyridin-3-yl)methyl)amino)propan-2-ol;
(R)-1-(((6-(2-chloro-3-(3-chloro-2-(isoindolin-5-yl)pyridin-4-yl)phenyl)-2-methoxypyridin-3-yl)methyl)amino)propan-2-ol;
1-(4-(((6-(2-chloro-3-(3-chloro-2-(isoindolin-5-yl)pyridin-4-yl)phenyl)-2-methoxypyridin-3-yl)methyl)amino)piperidin-1-yl)ethan-1-one;
1-(4-(((6-(2-chloro-3-(3-chloro-2-(isoindolin-5-yl)pyridin-4-yl)phenyl)-2-methoxypyridin-3-yl)methyl)(methyl)amino)piperidin-1-yl)ethan-1-one;
1-(((6-(((S)-4-(3'-chloro-6-methoxy-5-(((((S)-5-oxopyrrolidin-2-yl)methyl)amino)methyl)-[2,4'-bipyridin]-2'-yl)-2,3-dihydro-1H-inden-1-yl)amino)-2-methoxy-5-(trifluoromethyl)pyridin-3-yl)methyl)amino)cyclopropane-1-carboxylic acid;
1-(((6-(((S)-4-(3'-chloro-6-methoxy-5-(((((R)-5-oxopyrrolidin-2-yl)methyl)amino)methyl)-[2,4'-bipyridin]-2'-yl)-2,3-dihydro-1H-inden-1-yl)amino)-2-methoxy-5-(trifluoromethyl)pyridin-3-yl)methyl)amino)cyclopropane-1-carboxylic acid;
1-(((6-(((R)-4-(3'-chloro-6-methoxy-5-(((((S)-5-oxopyrrolidin-2-yl)methyl)amino)methyl)-[2,4'-bipyridin]-2'-yl)-2,3-dihydro-1H-inden-1-yl)amino)-2-methoxy-5-(trifluoromethyl)pyridin-3-yl)methyl)amino)cyclopropane-1-carboxylic acid;
1-(((6-(((R)-4-(3'-chloro-6-methoxy-5-(((((R)-5-oxopyrrolidin-2-yl)methyl)amino)methyl)-[2,4'-bipyridin]-2'-yl)-2,3-dihydro-1H-inden-1-yl)amino)-2-methoxy-5-(trifluoromethyl)pyridin-3-yl)methyl)amino)cyclopropane-1-carboxylic acid;
(S)-5-((((3'-chloro-2'-((S)-1-((5-((((R)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)methyl)pyrrolidin-2-one;
(S)-5-((((3'-chloro-2'-((S)-1-((5-((((S)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)methyl)pyrrolidin-2-one;
(S)-5-((((3'-chloro-2'-((R)-1-((5-((((S)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)methyl)pyrrolidin-2-one;
(S)-5-((((3'-chloro-2'-((R)-1-((5-((((R)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)methyl)pyrrolidin-2-one;
(R)-5-((((3'-chloro-2'-((R)-1-((5-((((R)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)methyl)pyrrolidin-2-one;
(R)-5-((((3'-chloro-2'-((S)-1-((5-((((S)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)methyl)pyrrolidin-2-one;
(R)-5-((((3'-chloro-2'-((S)-1-((5-((((R)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)methyl)pyrrolidin-2-one;
(R)-5-((((3'-chloro-2'-((R)-1-((5-((((S)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)methyl)pyrrolidin-2-one;
(S)-5-((((3'-chloro-6-methoxy-2'-((S)-1-((6-methoxy-5-(((((S)-5-oxopyrrolidin-2-yl)methyl)amino)methyl)-3-(trifluoromethyl)pyridin-2-yl)oxy)-2,3-dihydro-1H-inden-4-yl)-[2,4'-bipyridin]-5-yl)methyl)amino)methyl)pyrrolidin-2-one;
(S)-5-((((3'-chloro-6-methoxy-2'-((S)-1-((6-methoxy-5-(((((R)-5-oxopyrrolidin-2-yl)methyl)amino)methyl)-3-(trifluoromethyl)pyridin-2-yl)oxy)-2,3-dihydro-1H-inden-4-yl)-[2,4'-bipyridin]-5-yl)methyl)amino)methyl)pyrrolidin-2-one;
(S)-5-((((3'-chloro-6-methoxy-2'-((R)-1-((6-methoxy-5-(((((R)-5-oxopyrrolidin-2-yl)methyl)amino)methyl)-3-(trifluoromethyl)pyridin-2-yl)oxy)-2,3-dihydro-1H-inden-4-yl)-[2,4'-bipyridin]-5-yl)methyl)amino)methyl)pyrrolidin-2-one;
(S)-5-((((3'-chloro-6-methoxy-2'-((R)-1-((6-methoxy-5-(((((R)-5-oxopyrrolidin-2-yl)methyl)amino)methyl)-3-(trifluoromethyl)pyridin-2-yl)oxy)-2,3-dihydro-1H-inden-4-yl)-[2,4'-bipyridin]-5-yl)methyl)amino)methyl)pyrrolidin-2-one;
(R)-5-((((3'-chloro-6-methoxy-2'-((R)-1-((6-methoxy-5-(((((R)-5-oxopyrrolidin-2-yl)methyl)amino)methyl)-3-(trifluoromethyl)pyridin-2-yl)oxy)-2,3-dihydro-1H-inden-4-yl)-[2,4'-bipyridin]-5-yl)methyl)amino)methyl)pyrrolidin-2-one;
(R)-5-((((3'-chloro-6-methoxy-2'-((S)-1-((6-methoxy-5-(((((S)-5-oxopyrrolidin-2-yl)methyl)amino)methyl)-3-(trifluoromethyl)pyridin-2-yl)oxy)-2,3-dihydro-1H-inden-4-yl)-[2,4'-bipyridin]-5-yl)methyl)amino)methyl)pyrrolidin-2-one;
(R)-5-((((3'-chloro-6-methoxy-2'-((S)-1-((6-methoxy-5-(((((R)-5-oxopyrrolidin-2-yl)methyl)amino)methyl)-3-(trifluoromethyl)pyridin-2-yl)oxy)-2,3-dihydro-1H-inden-4-yl)-[2,4'-bipyridin]-5-yl)methyl)amino)methyl)pyrrolidin-2-one;
(R)-5-((((3'-chloro-6-methoxy-2'-((R)-1-((6-methoxy-5-(((((S)-5-oxopyrrolidin-2-yl)methyl)amino)methyl)-3-(trifluoromethyl)pyridin-2-yl)oxy)-2,3-dihydro-1H-inden-4-yl)-[2,4'-bipyridin]-5-yl)methyl)amino)methyl)pyrrolidin-2-one;
(S)-5-((((6-(3-(2-(3-(((1-acetylpiperidin-4-yl)amino)methyl)-1-methyl-1H-indazol-6-yl)-3-chloropyridin-4-yl)-2-chlorophenyl)-2-methoxypyridin-3-yl)methyl)amino)methyl)pyrrolidin-2-one;
(R)-5-((((6-(3-(2-(3-(((1-acetylpiperidin-4-yl)amino)methyl)-1-methyl-1H-indazol-6-yl)-3-chloropyridin-4-yl)-2-chlorophenyl)-2-methoxypyridin-3-yl)methyl)amino)methyl)pyrrolidin-2-one;
(S)-5-((((6-(3-(2-(3-(((1-acetylpiperidin-4-yl)(methyl)amino)methyl)-1-methyl-1H-indazol-6-yl)-3-chloropyridin-4-yl)-2-chlorophenyl)-2-methoxypyridin-3-yl)methyl)amino)methyl)pyrrolidin-2-one;
(R)-5-((((6-(3-(2-(3-(((1-acetylpiperidin-4-yl)(methyl)amino)methyl)-1-methyl-1H-indazol-6-yl)-3-chloropyridin-4-yl)-2-chlorophenyl)-2-methoxypyridin-3-yl)methyl)amino)methyl)pyrrolidin-2-one;
(S)-4-((3-(3-chloro-4-(2-chloro-3-(6-methoxy-5-((methylamino)methyl)pyridin-2-yl)phenyl)pyridin-2-yl)-5-methoxybenzyl)amino)-3-hydroxybutanoic acid;
(R)-4-((3-(3-chloro-4-(2-chloro-3-(6-methoxy-5-((methylamino)methyl)pyridin-2-yl)phenyl)pyridin-2-yl)-5-methoxybenzyl)amino)-3-hydroxybutanoic acid;
3-((3-(3-chloro-4-(2-chloro-3-(6-methoxy-5-((methylamino)methyl)pyridin-2-yl)phenyl)pyridin-2-yl)-5-methoxybenzyl)amino)propanoic acid;
(S)-1-(3-(3-chloro-4-(2-chloro-3-(6-methoxy-5-((methylamino)methyl)pyridin-2-yl)phenyl)pyridin-2-yl)-5-methoxybenzyl)pyrrolidine-3-carboxylic acid;
(R)-1-(3-(3-chloro-4-(2-chloro-3-(6-methoxy-5-((methylamino)methyl)pyridin-2-yl)phenyl)pyridin-2-yl)-5-methoxybenzyl)pyrrolidine-3-carboxylic acid;
2-(1-(3-(3-chloro-4-(2-chloro-3-(6-methoxy-5-((methylamino)methyl)pyridin-2-yl)phenyl)pyridin-2-yl)-5-methoxybenzyl)azetidin-3-yl)acetic acid;
2-(3-(3-chloro-4-(2-chloro-3-(6-methoxy-5-((methylamino)methyl)pyridin-2-yl)phenyl)pyridin-2-yl)-5-methoxybenzyl)-2-azaspiro[3.3]heptane-6-carboxylic acid;
3-((3-(3-chloro-4-(2-chloro-3-(6-methoxy-5-((methylamino)methyl)pyridin-2-yl)phenyl)pyridin-2-yl)-5-methoxybenzyl)amino)bicyclo[1.1.1]pentane-1-carboxylic acid;
3-(((3-(3-chloro-4-(2-chloro-3-(6-methoxy-5-((methylamino)methyl)pyridin-2-yl)phenyl)pyridin-2-yl)-5-methoxybenzyl)amino)methyl)bicyclo[1.1.1]pentane-1-carboxylic acid;
(S)-5-((((6-(2-chloro-3-(3-chloro-2-(2-((S)-2-hydroxypropyl)-8-methoxy-1,2,3,4-tetrahydroisoquinolin-6-yl)pyridin-4-yl)phenyl)-4-fluoro-2-methoxypyridin-3-yl)methyl)amino)methyl)pyrrolidin-2-one;
(S)-5-((((6-(2-chloro-3-(3-chloro-2-(2-((R)-2-hydroxypropyl)-8-methoxy-1,2,3,4-tetrahydroisoquinolin-6-yl)pyridin-4-yl)phenyl)-4-fluoro-2-methoxypyridin-3-yl)methyl)amino)methyl)pyrrolidin-2-one;
(R)-5-((((6-(2-chloro-3-(3-chloro-2-(2-((S)-2-hydroxypropyl)-8-methoxy-1,2,3,4-tetrahydroisoquinolin-6-yl)pyridin-4-yl)phenyl)-4-fluoro-2-methoxypyridin-3-yl)methyl)amino)methyl)pyrrolidin-2-one;
(R)-5-((((6-(2-chloro-3-(3-chloro-2-(2-((R)-2-hydroxypropyl)-8-methoxy-1,2,3,4-tetrahydroisoquinolin-6-yl)pyridin-4-yl)phenyl)-4-fluoro-2-methoxypyridin-3-yl)methyl)amino)methyl)pyrrolidin-2-one;
(S)-5-((((6-(2-chloro-3-(3-chloro-2-(1,1-dioxido-2,3,4,5-tetrahydrobenzo[f][1,4]thiazepin-8-yl)pyridin-4-yl)phenyl)-2-methoxypyridin-3-yl)methyl)amino)methyl)pyrrolidin-2-one;
(R)-5-((((6-(2-chloro-3-(3-chloro-2-(1,1-dioxido-2,3,4,5-tetrahydrobenzo[f][1,4]thiazepin-8-yl)pyridin-4-yl)phenyl)-2-methoxypyridin-3-yl)methyl)amino)methyl)pyrrolidin-2-one;
5-(3-chloro-4-(2-chloro-3-(4-fluoro-6-methoxy-5-(((((S)-5-oxopyrrolidin-2-yl)methyl)amino)methyl)pyridin-2-yl)phenyl)pyridin-2-yl)-2-(2-((((S)-5-oxopyrrolidin-2-yl)methyl)amino)ethyl)isoindolin-1-one;
5-(3-chloro-4-(2-chloro-3-(4-fluoro-6-methoxy-5-(((((S)-5-oxopyrrolidin-2-yl)methyl)amino)methyl)pyridin-2-yl)phenyl)pyridin-2-yl)-2-(2-((((R)-5-oxopyrrolidin-2-yl)methyl)amino)ethyl)isoindolin-1-one;
5-(3-chloro-4-(2-chloro-3-(4-fluoro-6-methoxy-5-(((((R)-5-oxopyrrolidin-2-yl)methyl)amino)methyl)pyridin-2-yl)phenyl)pyridin-2-yl)-2-(2-((((S)-5-oxopyrrolidin-2-yl)methyl)amino)ethyl)isoindolin-1-one;
5-(3-chloro-4-(2-chloro-3-(4-fluoro-6-methoxy-5-(((((R)-5-oxopyrrolidin-2-yl)methyl)amino)methyl)pyridin-2-yl)phenyl)pyridin-2-yl)-2-(2-((((R)-5-oxopyrrolidin-2-yl)methyl)amino)ethyl)isoindolin-1-one;
(S)-5-((((6-(2-chloro-3-(3-chloro-2-(4-(4,5-dihydro-1H-imidazol-2-yl)-3-methoxyphenyl)pyridin-4-yl)phenyl)-2-methoxypyridin-3-yl)methyl)amino)methyl)pyrrolidin-2-one;
(R)-5-((((6-(2-chloro-3-(3-chloro-2-(4-(4,5-dihydro-1H-imidazol-2-yl)-3-methoxyphenyl)pyridin-4-yl)phenyl)-2-methoxypyridin-3-yl)methyl)amino)methyl)pyrrolidin-2-one;
(S)-4-((4-(3-chloro-4-(2-chloro-3-(5-(4,5-dihydro-1H-imidazol-2-yl)-6-methoxypyridin-2-yl)phenyl)pyridin-2-yl)-2-methoxybenzyl)amino)-3-hydroxybutanoic acid;
(R)-4-((4-(3-chloro-4-(2-chloro-3-(5-(4,5-dihydro-1 H-imidazol-2-yl)-6-methoxypyridin-2-yl)phenyl)pyridin-2-yl)-2-methoxybenzyl)amino)-3-hydroxybutanoic acid;
(S)-5-((((6-(2-chloro-3-(3-chloro-2-(3-fluoro-5-methoxy-4-(((((S)-oxetan-2-yl)methyl)amino)methyl)phenyl)pyridin-4-yl)phenyl)-2-methoxypyridin-3-yl)methyl)amino)methyl)pyrrolidin-2-one;
(S)-5-((((6-(2-chloro-3-(3-chloro-2-(3-fluoro-5-methoxy-4-(((((R)-oxetan-2-yl)methyl)amino)methyl)phenyl)pyridin-4-yl)phenyl)-2-methoxypyridin-3-yl)methyl)amino)methyl)pyrrolidin-2-one;
(R)-5-((((6-(2-chloro-3-(3-chloro-2-(3-fluoro-5-methoxy-4-(((((S)-oxetan-2-yl)methyl)amino)methyl)phenyl)pyridin-4-yl)phenyl)-2-methoxypyridin-3-yl)methyl)amino)methyl)pyrrolidin-2-one;
(S)-5-((((6-(2-chloro-3-(3-chloro-2-(3-fluoro-5-methoxy-4-(((((S)-oxetan-2-yl)methyl)amino)methyl)phenyl)pyridin-4-yl)phenyl)-2-methoxypyridin-3-yl)methyl)amino)methyl)pyrrolidin-2-one;
methyl (S)-1-((6-(3-chloro-4-(2-chloro-3-(6-methoxy-5-((((5-oxopyrrolidin-2-yl)methyl)amino)methyl)pyridin-2-yl)phenyl)pyridin-2-yl)-3-methyl-4-oxo-3,4-dihydropyrrolo[2,1-f][1,2,4]triazin-2-yl)methyl)azetidine-3-carboxylate;
methyl (R)-1-((6-(3-chloro-4-(2-chloro-3-(6-methoxy-5-((((5-oxopyrrolidin-2-yl)methyl)amino)methyl)pyridin-2-yl)phenyl)pyridin-2-yl)-3-methyl-4-oxo-3,4-dihydropyrrolo[2,1-f][1,2,4]triazin-2-yl)methyl)azetidine-3-carboxylate;
(S)-5-((((6-(2-chloro-3-(3-chloro-2-(1-methyl-3-(((((S)-5-oxopyrrolidin-2-yl)methyl)amino)methyl)-1H-indazol-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(1-methyl-3-(((((S)-5-oxopyrrolidin-2-yl)methyl)amino)methyl)-1H-indazol-6-yl)pyridin-4-yl)phenyl)-2-methoxypyridin-3-yl)methyl)amino)methyl)pyrrolidin-2-one;
(S)-5-((((6-(2-chloro-3-(3-chloro-2-((S)-5-((((S)-5-oxopyrrolidin-2-yl)methyl)amino)-5,6,7,8-tetrahydronaphthalen-2-yl)pyridin-4-yl)phenyl)-2-methoxypyridin-3-yl)methyl)amino)methyl)pyrrolidin-2-one;
(S)-5-((((6-(2-chloro-3-(3-chloro-2-((S)-5-((((R)-5-oxopyrrolidin-2-yl)methyl)amino)-5,6,7,8-tetrahydronaphthalen-2-yl)pyridin-4-yl)phenyl)-2-methoxypyridin-3-yl)methyl)amino)methyl)pyrrolidin-2-one;
(S)-5-((((6-(2-chloro-3-(3-chloro-2-((R)-5-((((S)-5-oxopyrrolidin-2-yl)methyl)amino)-5,6,7,8-tetrahydronaphthalen-2-yl)pyridin-4-yl)phenyl)-2-methoxypyridin-3-yl)methyl)amino)methyl)pyrrolidin-2-one;
(S)-5-((((6-(2-chloro-3-(3-chloro-2-((R)-5-((((R)-5-oxopyrrolidin-2-yl)methyl)amino)-5,6,7,8-tetrahydronaphthalen-2-yl)pyridin-4-yl)phenyl)-2-methoxypyridin-3-yl)methyl)amino)methyl)pyrrolidin-2-one;
(R)-5-((((6-(2-chloro-3-(3-chloro-2-((R)-5-((((R)-5-oxopyrrolidin-2-yl)methyl)amino)-5,6,7,8-tetrahydronaphthalen-2-yl)pyridin-4-yl)phenyl)-2-methoxypyridin-3-yl)methyl)amino)methyl)pyrrolidin-2-one;
(R)-5-((((6-(2-chloro-3-(3-chloro-2-((R)-5-((((S)-5-oxopyrrolidin-2-yl)methyl)amino)-5,6,7,8-tetrahydronaphthalen-2-yl)pyridin-4-yl)phenyl)-2-methoxypyridin-3-yl)methyl)amino)methyl)pyrrolidin-2-one;
(R)-5-((((6-(2-chloro-3-(3-chloro-2-((R)-5-((((R)-5-oxopyrrolidin-2-yl)methyl)amino)-5,6,7,8-tetrahydronaphthalen-2-yl)pyridin-4-yl)phenyl)-2-methoxypyridin-3-yl)methyl)amino)methyl)pyrrolidin-2-one;
(R)-5-((((6-(2-chloro-3-(3-chloro-2-((S)-5-((((S)-5-oxopyrrolidin-2-yl)methyl)amino)-5,6,7,8-tetrahydronaphthalen-2-yl)pyridin-4-yl)phenyl)-2-methoxypyridin-3-yl)methyl)amino)methyl)pyrrolidin-2-one;
1-(((6-(((S)-5-(3'-chloro-6-methoxy-5-(((((S)-5-oxopyrrolidin-2-yl)methyl)amino)methyl)-[2,4'-bipyridin]-2'-yl)-1,2,3,4-tetrahydronaphthalen-1-yl)amino)-2-methoxy-5-(trifluoromethyl)pyridin-3-yl)methyl)amino)cyclopropane-1-carboxylic acid;
1-(((6-(((S)-5-(3'-chloro-6-methoxy-5-(((((R)-5-oxopyrrolidin-2-yl)methyl)amino)methyl)-[2,4'-bipyridin]-2'-yl)-1,2,3,4-tetrahydronaphthalen-1-yl)amino)-2-methoxy-5-(trifluoromethyl)pyridin-3-yl)methyl)amino)cyclopropane-1-carboxylic acid;
1-(((6-(((R)-5-(3'-chloro-6-methoxy-5-(((((S)-5-oxopyrrolidin-2-yl)methyl)amino)methyl)-[2,4'-bipyridin]-2'-yl)-1,2,3,4-tetrahydronaphthalen-1-yl)amino)-2-methoxy-5-(trifluoromethyl)pyridin-3-yl)methyl)amino)cyclopropane-1-carboxylic acid;
1-(((6-(((R)-5-(3'-chloro-6-methoxy-5-(((((R)-5-oxopyrrolidin-2-yl)methyl)amino)methyl)-[2,4'-bipyridin]-2'-yl)-1,2,3,4-tetrahydronaphthalen-1-yl)amino)-2-methoxy-5-(trifluoromethyl)pyridin-3-yl)methyl)amino)cyclopropane-1-carboxylic acid;
(S)-5-((((3'-chloro-2'-((S)-5-((5-((((S)-2-hydroxypropyl)amino)methyl)-6-methoxy-3-(trifluoromethyl)pyridin-2-yl)amino)-5,6,7,8-tetrahydronaphthalen-1-yl)-6-methoxy-[2,4'-bipyridin]-5-yl)methyl)amino)methyl)pyrrolidin-2-one;
(S)-5-((((3'-chloro-2'-((S)-5-((5-((((R)-2-hydroxypropyl)amino)methyl)-6-methoxy-3-(trifluoromethyl)pyridin-2-yl)amino)-5,6,7,8-tetrahydronaphthalen-1-yl)-6-methoxy-[2,4'-bipyridin]-5-yl)methyl)amino)methyl)pyrrolidin-2-one;
(S)-5-((((3'-chloro-2'-((R)-5-((5-((((S)-2-hydroxypropyl)amino)methyl)-6-methoxy-3-(trifluoromethyl)pyridin-2-yl)amino)-5,6,7,8-tetrahydronaphthalen-1-yl)-6-methoxy-[2,4'-bipyridin]-5-yl)methyl)amino)methyl)pyrrolidin-2-one;
(S)-5-((((3'-chloro-2'-((R)-5-((5-((((R)-2-hydroxypropyl)amino)methyl)-6-methoxy-3-(trifluoromethyl)pyridin-2-yl)amino)-5,6,7,8-tetrahydronaphthalen-1-yl)-6-methoxy-[2,4'-bipyridin]-5-yl)methyl)amino)methyl)pyrrolidin-2-one;
(R)-5-((((3'-chloro-2'-((R)-5-((5-((((R)-2-hydroxypropyl)amino)methyl)-6-methoxy-3-(trifluoromethyl)pyridin-2-yl)amino)-5,6,7,8-tetrahydronaphthalen-1-yl)-6-methoxy-[2,4'-bipyridin]-5-yl)methyl)amino)methyl)pyrrolidin-2-one;
(R)-5-((((3'-chloro-2'-((R)-5-((5-((((S)-2-hydroxypropyl)amino)methyl)-6-methoxy-3-(trifluoromethyl)pyridin-2-yl)amino)-5,6,7,8-tetrahydronaphthalen-1-yl)-6-methoxy-[2,4'-bipyridin]-5-yl)methyl)amino)methyl)pyrrolidin-2-one;
(R)-5-((((3'-chloro-2'-((S)-5-((5-((((R)-2-hydroxypropyl)amino)methyl)-6-methoxy-3-(trifluoromethyl)pyridin-2-yl)amino)-5,6,7,8-tetrahydronaphthalen-1-yl)-6-methoxy-[2,4'-bipyridin]-5-yl)methyl)amino)methyl)pyrrolidin-2-one;
(R)-5-((((3'-chloro-2'-((S)-5-((5-((((S)-2-hydroxypropyl)amino)methyl)-6-methoxy-3-(trifluoromethyl)pyridin-2-yl)amino)-5,6,7,8-tetrahydronaphthalen-1-yl)-6-methoxy-[2,4'-bipyridin]-5-yl)methyl)amino)methyl)pyrrolidin-2-one;
(S)-5-((((3'-chloro-6-methoxy-2'-((S)-5-((6-methoxy-5-(((((S)-5-oxopyrrolidin-2-yl)methyl)amino)methyl)-3-(trifluoromethyl)pyridin-2-yl)amino)-5,6,7,8-tetrahydronaphthalen-1-yl)-[2,4'-bipyridin]-5-yl)methyl)amino)methyl)pyrrolidin-2-one;
(S)-5-((((3'-chloro-6-methoxy-2'-((S)-5-((6-methoxy-5-(((((R)-5-oxopyrrolidin-2-yl)methyl)amino)methyl)-3-(trifluoromethyl)pyridin-2-yl)amino)-5,6,7,8-tetrahydronaphthalen-1-yl)-[2,4'-bipyridin]-5-yl)methyl)amino)methyl)pyrrolidin-2-one;
(S)-5-((((3'-chloro-6-methoxy-2'-((R)-5-((6-methoxy-5-(((((S)-5-oxopyrrolidin-2-yl)methyl)amino)methyl)-3-(trifluoromethyl)pyridin-2-yl)amino)-5,6,7,8-tetrahydronaphthalen-1-yl)-[2,4'-bipyridin]-5-yl)methyl)amino)methyl)pyrrolidin-2-one;
(S)-5-((((3'-chloro-6-methoxy-2'-((R)-5-((6-methoxy-5-(((((R)-5-oxopyrrolidin-2-yl)methyl)amino)methyl)-3-(trifluoromethyl)pyridin-2-yl)amino)-5,6,7,8-tetrahydronaphthalen-1-yl)-[2,4'-bipyridin]-5-yl)methyl)amino)methyl)pyrrolidin-2-one;
(R)-5-((((3'-chloro-6-methoxy-2'-((R)-5-((6-methoxy-5-(((((R)-5-oxopyrrolidin-2-yl)methyl)amino)methyl)-3-(trifluoromethyl)pyridin-2-yl)amino)-5,6,7,8-tetrahydronaphthalen-1-yl)-[2,4'-bipyridin]-5-yl)methyl)amino)methyl)pyrrolidin-2-one;
(R)-5-((((3'-chloro-6-methoxy-2'-((R)-5-((6-methoxy-5-(((((S)-5-oxopyrrolidin-2-yl)methyl)amino)methyl)-3-(trifluoromethyl)pyridin-2-yl)amino)-5,6,7,8-tetrahydronaphthalen-1-yl)-[2,4'-bipyridin]-5-yl)methyl)amino)methyl)pyrrolidin-2-one;
(R)-5-((((3'-chloro-6-methoxy-2'-((S)-5-((6-methoxy-5-(((((R)-5-oxopyrrolidin-2-yl)methyl)amino)methyl)-3-(trifluoromethyl)pyridin-2-yl)amino)-5,6,7,8-tetrahydronaphthalen-1-yl)-[2,4'-bipyridin]-5-yl)methyl)amino)methyl)pyrrolidin-2-one;
(R)-5-((((3'-chloro-6-methoxy-2'-((S)-5-((6-methoxy-5-(((((S)-5-oxopyrrolidin-2-yl)methyl)amino)methyl)-3-(trifluoromethyl)pyridin-2-yl)amino)-5,6,7,8-tetrahydronaphthalen-1-yl)-[2,4'-bipyridin]-5-yl)methyl)amino)methyl)pyrrolidin-2-one;
2-((6-(((S)-5-(3'-chloro-6-methoxy-5-(((((S)-5-oxopyrrolidin-2-yl)methyl)amino)methyl)-[2,4'-bipyridin]-2'-yl)-1,2,3,4-tetrahydronaphthalen-1-yl)amino)-2-methoxy-5-(trifluoromethyl)pyridin-3-yl)methyl)-2,6-diazaspiro[3.4]octan-7-one;
2-((6-(((S)-5-(3'-chloro-6-methoxy-5-(((((R)-5-oxopyrrolidin-2-yl)methyl)amino)methyl)-[2,4'-bipyridin]-2'-yl)-1,2,3,4-tetrahydronaphthalen-1-yl)amino)-2-methoxy-5-(trifluoromethyl)pyridin-3-yl)methyl)-2,6-diazaspiro[3.4]octan-7-one;
2-((6-(((R)-5-(3'-chloro-6-methoxy-5-(((((S)-5-oxopyrrolidin-2-yl)methyl)amino)methyl)-[2,4'-bipyridin]-2'-yl)-1,2,3,4-tetrahydronaphthalen-1-yl)amino)-2-methoxy-5-(trifluoromethyl)pyridin-3-yl)methyl)-2,6-diazaspiro[3.4]octan-7-one;
2-((6-(((R)-5-(3'-chloro-6-methoxy-5-(((((R)-5-oxopyrrolidin-2-yl)methyl)amino)methyl)-[2,4'-bipyridin]-2'-yl)-1,2,3,4-tetrahydronaphthalen-1-yl)amino)-2-methoxy-5-(trifluoromethyl)pyridin-3-yl)methyl)-2,6-diazaspiro[3.4]octan-7-one;
(S)-N-(5-(3'-chloro-6-methoxy-5-((methylamino)methyl)-[2,4'-bipyridin]-2'-yl)-1,2,3,4-tetrahydronaphthalen-1-yl)-6-methoxy-5-((methylamino)methyl)-3-(trifluoromethyl)pyridin-2-amine;
(R)-N-(5-(3'-chloro-6-methoxy-5-((methylamino)methyl)-[2,4'-bipyridin]-2'-yl)-1,2,3,4-tetrahydronaphthalen-1-yl)-6-methoxy-5-((methylamino)methyl)-3-(trifluoromethyl)pyridin-2-amine;
1-(((6-(((S)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)cyclopropane-1-carboxylic acid;
1-(((6-(((S)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)cyclopropane-1-carboxylic acid;
1-(((6-(((R)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)cyclopropane-1-carboxylic acid;
1-(((6-(((R)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)cyclopropane-1-carboxylic acid;
(S)-5-(((4-(3-chloro-4-((S)-5-((5-((((S)-2-hydroxypropyl)amino)methyl)-6-methoxy-3-(trifluoromethyl)pyridin-2-yl)amino)-5,6,7,8-tetrahydronaphthalen-1-yl)pyridin-2-yl)-2-methoxybenzyl)amino)methyl)pyrrolidin-2-one;
(S)-5-(((4-(3-chloro-4-((S)-5-((5-((((R)-2-hydroxypropyl)amino)methyl)-6-methoxy-3-(trifluoromethyl)pyridin-2-yl)amino)-5,6,7,8-tetrahydronaphthalen-1-yl)pyridin-2-yl)-2-methoxybenzyl)amino)methyl)pyrrolidin-2-one;
(S)-5-(((4-(3-chloro-4-((R)-5-((5-((((S)-2-hydroxypropyl)amino)methyl)-6-methoxy-3-(trifluoromethyl)pyridin-2-yl)amino)-5,6,7,8-tetrahydronaphthalen-1-yl)pyridin-2-yl)-2-methoxybenzyl)amino)methyl)pyrrolidin-2-one;
(S)-5-(((4-(3-chloro-4-((R)-5-((5-((((R)-2-hydroxypropyl)amino)methyl)-6-methoxy-3-(trifluoromethyl)pyridin-2-yl)amino)-5,6,7,8-tetrahydronaphthalen-1-yl)pyridin-2-yl)-2-methoxybenzyl)amino)methyl)pyrrolidin-2-one;
(R)-5-(((4-(3-chloro-4-((R)-5-((5-((((R)-2-hydroxypropyl)amino)methyl)-6-methoxy-3-(trifluoromethyl)pyridin-2-yl)amino)-5,6,7,8-tetrahydronaphthalen-1-yl)pyridin-2-yl)-2-methoxybenzyl)amino)methyl)pyrrolidin-2-one;
(R)-5-(((4-(3-chloro-4-((R)-5-((5-((((S)-2-hydroxypropyl)amino)methyl)-6-methoxy-3-(trifluoromethyl)pyridin-2-yl)amino)-5,6,7,8-tetrahydronaphthalen-1-yl)pyridin-2-yl)-2-methoxybenzyl)amino)methyl)pyrrolidin-2-one;
(R)-5-(((4-(3-chloro-4-((S)-5-((5-((((R)-2-hydroxypropyl)amino)methyl)-6-methoxy-3-(trifluoromethyl)pyridin-2-yl)amino)-5,6,7,8-tetrahydronaphthalen-1-yl)pyridin-2-yl)-2-methoxybenzyl)amino)methyl)pyrrolidin-2-one;
(R)-5-(((4-(3-chloro-4-((S)-5-((5-((((S)-2-hydroxypropyl)amino)methyl)-6-methoxy-3-(trifluoromethyl)pyridin-2-yl)amino)-5,6,7,8-tetrahydronaphthalen-1-yl)pyridin-2-yl)-2-methoxybenzyl)amino)methyl)pyrrolidin-2-one;
(S)-5-((((6-(((S)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)methyl)pyrrolidin-2-one;
(S)-5-((((6-(((S)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)methyl)pyrrolidin-2-one;
(S)-5-((((6-(((R)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)methyl)pyrrolidin-2-one;
(S)-5-((((6-(((R)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)methyl)pyrrolidin-2-one;
(R)-5-((((6-(((R)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)methyl)pyrrolidin-2-one;
(R)-5-((((6-(((R)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)methyl)pyrrolidin-2-one;
(R)-5-((((6-(((S)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)methyl)pyrrolidin-2-one;
(R)-5-((((6-(((S)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)methyl)pyrrolidin-2-one;
2-((6-(((S)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)-2,6-diazaspiro[3.4]octan-7-one;
2-((6-(((S)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)-2,6-diazaspiro[3.4]octan-7-one;
2-((6-(((R)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)-2,6-diazaspiro[3.4]octan-7-one;
2-((6-(((R)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)-2,6-diazaspiro[3.4]octan-7-one;
(S)-N-(5-(3-chloro-2-(3-methoxy-4-((methylamino)methyl)phenyl)pyridin-4-yl)-1,2,3,4-tetrahydronaphthalen-1-yl)-6-methoxy-5-((methylamino)methyl)-3-(trifluoromethyl)pyridin-2-amine;
(R)-N-(5-(3-chloro-2-(3-methoxy-4-((methylamino)methyl)phenyl)pyridin-4-yl)-1,2,3,4-tetrahydronaphthalen-1-yl)-6-methoxy-5-((methylamino)methyl)-3-(trifluoromethyl)pyridin-2-amine;
1-(((6-(((S)-5-(3'-chloro-6-methoxy-5-(((((S)-5-oxopyrrolidin-2-yl)methyl)amino)methyl)-[2,4'-bipyridin]-2'-yl)-1,2,3,4-tetrahydronaphthalen-1-yl)oxy)-2-methoxy-5-(trifluoromethyl)pyridin-3-yl)methyl)amino)cyclopropane-1-carboxylic acid;
1-(((6-(((S)-5-(3'-chloro-6-methoxy-5-(((((R)-5-oxopyrrolidin-2-yl)methyl)amino)methyl)-[2,4'-bipyridin]-2'-yl)-1,2,3,4-tetrahydronaphthalen-1-yl)oxy)-2-methoxy-5-(trifluoromethyl)pyridin-3-yl)methyl)amino)cyclopropane-1-carboxylic acid;
1-(((6-(((R)-5-(3'-chloro-6-methoxy-5-(((((S)-5-oxopyrrolidin-2-yl)methyl)amino)methyl)-[2,4'-bipyridin]-2'-yl)-1,2,3,4-tetrahydronaphthalen-1-yl)oxy)-2-methoxy-5-(trifluoromethyl)pyridin-3-yl)methyl)amino)cyclopropane-1-carboxylic acid;
1-(((6-(((R)-5-(3'-chloro-6-methoxy-5-(((((R)-5-oxopyrrolidin-2-yl)methyl)amino)methyl)-[2,4'-bipyridin]-2'-yl)-1,2,3,4-tetrahydronaphthalen-1-yl)oxy)-2-methoxy-5-(trifluoromethyl)pyridin-3-yl)methyl)amino)cyclopropane-1-carboxylic acid;
(S)-5-((((3'-chloro-2'-((S)-5-((5-((((S)-2-hydroxypropyl)amino)methyl)-6-methoxy-3-(trifluoromethyl)pyridin-2-yl)oxy)-5,6,7,8-tetrahydronaphthalen-1-yl)-6-methoxy-[2,4'-bipyridin]-5-yl)methyl)amino)methyl)pyrrolidin-2-one;
(S)-5-((((3'-chloro-2'-((S)-5-((5-((((R)-2-hydroxypropyl)amino)methyl)-6-methoxy-3-(trifluoromethyl)pyridin-2-yl)oxy)-5,6,7,8-tetrahydronaphthalen-1-yl)-6-methoxy-[2,4'-bipyridin]-5-yl)methyl)amino)methyl)pyrrolidin-2-one;
(S)-5-((((3'-chloro-2'-((R)-5-((5-((((S)-2-hydroxypropyl)amino)methyl)-6-methoxy-3-(trifluoromethyl)pyridin-2-yl)oxy)-5,6,7,8-tetrahydronaphthalen-1-yl)-6-methoxy-[2,4'-bipyridin]-5-yl)methyl)amino)methyl)pyrrolidin-2-one;
(S)-5-((((3'-chloro-2'-((R)-5-((5-((((R)-2-hydroxypropyl)amino)methyl)-6-methoxy-3-(trifluoromethyl)pyridin-2-yl)oxy)-5,6,7,8-tetrahydronaphthalen-1-yl)-6-methoxy-[2,4'-bipyridin]-5-yl)methyl)amino)methyl)pyrrolidin-2-one;
(R)-5-((((3'-chloro-2'-((R)-5-((5-((((R)-2-hydroxypropyl)amino)methyl)-6-methoxy-3-(trifluoromethyl)pyridin-2-yl)oxy)-5,6,7,8-tetrahydronaphthalen-1-yl)-6-methoxy-[2,4'-bipyridin]-5-yl)methyl)amino)methyl)pyrrolidin-2-one;
(R)-5-((((3'-chloro-2'-((R)-5-((5-((((S)-2-hydroxypropyl)amino)methyl)-6-methoxy-3-(trifluoromethyl)pyridin-2-yl)oxy)-5,6,7,8-tetrahydronaphthalen-1-yl)-6-methoxy-[2,4'-bipyridin]-5-yl)methyl)amino)methyl)pyrrolidin-2-one;
(R)-5-((((3'-chloro-2'-((S)-5-((5-((((R)-2-hydroxypropyl)amino)methyl)-6-methoxy-3-(trifluoromethyl)pyridin-2-yl)oxy)-5,6,7,8-tetrahydronaphthalen-1-yl)-6-methoxy-[2,4'-bipyridin]-5-yl)methyl)amino)methyl)pyrrolidin-2-one;
(R)-5-((((3'-chloro-2'-((S)-5-((5-((((S)-2-hydroxypropyl)amino)methyl)-6-methoxy-3-(trifluoromethyl)pyridin-2-yl)oxy)-5,6,7,8-tetrahydronaphthalen-1-yl)-6-methoxy-[2,4'-bipyridin]-5-yl)methyl)amino)methyl)pyrrolidin-2-one;
(S)-5-((((3'-chloro-6-methoxy-2'-((S)-5-((6-methoxy-5-(((((S)-5-oxopyrrolidin-2-yl)methyl)amino)methyl)-3-(trifluoromethyl)pyridin-2-yl)oxy)-5,6,7,8-tetrahydronaphthalen-1-yl)-[2,4'-bipyridin]-5-yl)methyl)amino)methyl)pyrrolidin-2-one;
(S)-5-((((3'-chloro-6-methoxy-2'-((S)-5-((6-methoxy-5-(((((R)-5-oxopyrrolidin-2-yl)methyl)amino)methyl)-3-(trifluoromethyl)pyridin-2-yl)oxy)-5,6,7,8-tetrahydronaphthalen-1-yl)-[2,4'-bipyridin]-5-yl)methyl)amino)methyl)pyrrolidin-2-one;
(S)-5-((((3'-chloro-6-methoxy-2'-((R)-5-((6-methoxy-5-(((((S)-5-oxopyrrolidin-2-yl)methyl)amino)methyl)-3-(trifluoromethyl)pyridin-2-yl)oxy)-5,6,7,8-tetrahydronaphthalen-1-yl)-[2,4'-bipyridin]-5-yl)methyl)amino)methyl)pyrrolidin-2-one;
(S)-5-((((3'-chloro-6-methoxy-2'-((R)-5-((6-methoxy-5-(((((R)-5-oxopyrrolidin-2-yl)methyl)amino)methyl)-3-(trifluoromethyl)pyridin-2-yl)oxy)-5,6,7,8-tetrahydronaphthalen-1-yl)-[2,4'-bipyridin]-5-yl)methyl)amino)methyl)pyrrolidin-2-one;
(R)-5-((((3'-chloro-6-methoxy-2'-((R)-5-((6-methoxy-5-(((((R)-5-oxopyrrolidin-2-yl)methyl)amino)methyl)-3-(trifluoromethyl)pyridin-2-yl)oxy)-5,6,7,8-tetrahydronaphthalen-1-yl)-[2,4'-bipyridin]-5-yl)methyl)amino)methyl)pyrrolidin-2-one;
(R)-5-((((3'-chloro-6-methoxy-2'-((R)-5-((6-methoxy-5-(((((S)-5-oxopyrrolidin-2-yl)methyl)amino)methyl)-3-(trifluoromethyl)pyridin-2-yl)oxy)-5,6,7,8-tetrahydronaphthalen-1-yl)-[2,4'-bipyridin]-5-yl)methyl)amino)methyl)pyrrolidin-2-one;
(R)-5-((((3'-chloro-6-methoxy-2'-((S)-5-((6-methoxy-5-(((((R)-5-oxopyrrolidin-2-yl)methyl)amino)methyl)-3-(trifluoromethyl)pyridin-2-yl)oxy)-5,6,7,8-tetrahydronaphthalen-1-yl)-[2,4'-bipyridin]-5-yl)methyl)amino)methyl)pyrrolidin-2-one;
(R)-5-((((3'-chloro-6-methoxy-2'-((S)-5-((6-methoxy-5-(((((S)-5-oxopyrrolidin-2-yl)methyl)amino)methyl)-3-(trifluoromethyl)pyridin-2-yl)oxy)-5,6,7,8-tetrahydronaphthalen-1-yl)-[2,4'-bipyridin]-5-yl)methyl)amino)methyl)pyrrolidin-2-one;
2-((6-(((S)-5-(3'-chloro-6-methoxy-5-(((((S)-5-oxopyrrolidin-2-yl)methyl)amino)methyl)-[2,4'-bipyridin]-2'-yl)-1,2,3,4-tetrahydronaphthalen-1-yl)oxy)-2-methoxy-5-(trifluoromethyl)pyridin-3-yl)methyl)-2,6-diazaspiro[3.4]octan-7-one;
2-((6-(((S)-5-(3'-chloro-6-methoxy-5-(((((R)-5-oxopyrrolidin-2-yl)methyl)amino)methyl)-[2,4'-bipyridin]-2'-yl)-1,2,3,4-tetrahydronaphthalen-1-yl)oxy)-2-methoxy-5-(trifluoromethyl)pyridin-3-yl)methyl)-2,6-diazaspiro[3.4]octan-7-one;
2-((6-(((S)-5-(3'-chloro-6-methoxy-5-(((((S)-5-oxopyrrolidin-2-yl)methyl)amino)methyl)-[2,4'-bipyridin]-2'-yl)-1,2,3,4-tetrahydronaphthalen-1-yl)oxy)-2-methoxy-5-(trifluoromethyl)pyridin-3-yl)methyl)-2,6-diazaspiro[3.4]octan-7-one;
2-((6-(((R)-5-(3'-chloro-6-methoxy-5-(((((R)-5-oxopyrrolidin-2-yl)methyl)amino)methyl)-[2,4'-bipyridin]-2'-yl)-1,2,3,4-tetrahydronaphthalen-1-yl)oxy)-2-methoxy-5-(trifluoromethyl)pyridin-3-yl)methyl)-2,6-diazaspiro[3.4]octan-7-one;
(S)-1-(3'-chloro-6-methoxy-2'-(5-((6-methoxy-5-((methylamino)methyl)-3-(trifluoromethyl)pyridin-2-yl)oxy)-5,6,7,8-tetrahydronaphthalen-1-yl)-[2,4'-bipyridin]-5-yl)-N-methylmethanamine;
(R)-1-(3'-chloro-6-methoxy-2'-(5-((6-methoxy-5-((methylamino)methyl)-3-(trifluoromethyl)pyridin-2-yl)oxy)-5,6,7,8-tetrahydronaphthalen-1-yl)-[2,4'-bipyridin]-5-yl)-N-methylmethanamine;
1-(((6-(((S)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)oxy)-2-methoxy-5-(trifluoromethyl)pyridin-3-yl)methyl)amino)cyclopropane-1-carboxylic acid;
1-(((6-(((S)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)oxy)-2-methoxy-5-(trifluoromethyl)pyridin-3-yl)methyl)amino)cyclopropane-1-carboxylic acid;
1-(((6-(((R)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)oxy)-2-methoxy-5-(trifluoromethyl)pyridin-3-yl)methyl)amino)cyclopropane-1-carboxylic acid;
1-(((6-(((R)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)oxy)-2-methoxy-5-(trifluoromethyl)pyridin-3-yl)methyl)amino)cyclopropane-1-carboxylic acid;
(S)-5-(((4-(3-chloro-4-((S)-5-((5-((((S)-2-hydroxypropyl)amino)methyl)-6-methoxy-3-(trifluoromethyl)pyridin-2-yl)oxy)-5,6,7,8-tetrahydronaphthalen-1-yl)pyridin-2-yl)-2-methoxybenzyl)amino)methyl)pyrrolidin-2-one;
(S)-5-(((4-(3-chloro-4-((S)-5-((5-((((R)-2-hydroxypropyl)amino)methyl)-6-methoxy-3-(trifluoromethyl)pyridin-2-yl)oxy)-5,6,7,8-tetrahydronaphthalen-1-yl)pyridin-2-yl)-2-methoxybenzyl)amino)methyl)pyrrolidin-2-one;
(S)-5-(((4-(3-chloro-4-((R)-5-((5-((((S)-2-hydroxypropyl)amino)methyl)-6-methoxy-3-(trifluoromethyl)pyridin-2-yl)oxy)-5,6,7,8-tetrahydronaphthalen-1-yl)pyridin-2-yl)-2-methoxybenzyl)amino)methyl)pyrrolidin-2-one;
(S)-5-(((4-(3-chloro-4-((R)-5-((5-((((R)-2-hydroxypropyl)amino)methyl)-6-methoxy-3-(trifluoromethyl)pyridin-2-yl)oxy)-5,6,7,8-tetrahydronaphthalen-1-yl)pyridin-2-yl)-2-methoxybenzyl)amino)methyl)pyrrolidin-2-one;
(R)-5-(((4-(3-chloro-4-((R)-5-((5-((((R)-2-hydroxypropyl)amino)methyl)-6-methoxy-3-(trifluoromethyl)pyridin-2-yl)oxy)-5,6,7,8-tetrahydronaphthalen-1-yl)pyridin-2-yl)-2-methoxybenzyl)amino)methyl)pyrrolidin-2-one;
(R)-5-(((4-(3-chloro-4-((R)-5-((5-((((S)-2-hydroxypropyl)amino)methyl)-6-methoxy-3-(trifluoromethyl)pyridin-2-yl)oxy)-5,6,7,8-tetrahydronaphthalen-1-yl)pyridin-2-yl)-2-methoxybenzyl)amino)methyl)pyrrolidin-2-one;
(R)-5-(((4-(3-chloro-4-((S)-5-((5-((((R)-2-hydroxypropyl)amino)methyl)-6-methoxy-3-(trifluoromethyl)pyridin-2-yl)oxy)-5,6,7,8-tetrahydronaphthalen-1-yl)pyridin-2-yl)-2-methoxybenzyl)amino)methyl)pyrrolidin-2-one;
(R)-5-(((4-(3-chloro-4-((S)-5-((5-((((S)-2-hydroxypropyl)amino)methyl)-6-methoxy-3-(trifluoromethyl)pyridin-2-yl)oxy)-5,6,7,8-tetrahydronaphthalen-1-yl)pyridin-2-yl)-2-methoxybenzyl)amino)methyl)pyrrolidin-2-one;
(S)-5-((((6-(((S)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)oxy)-2-methoxy-5-(trifluoromethyl)pyridin-3-yl)methyl)amino)methyl)pyrrolidin-2-one;
(S)-5-((((6-(((S)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)oxy)-2-methoxy-5-(trifluoromethyl)pyridin-3-yl)methyl)amino)methyl)pyrrolidin-2-one;
(S)-5-((((6-(((R)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)oxy)-2-methoxy-5-(trifluoromethyl)pyridin-3-yl)methyl)amino)methyl)pyrrolidin-2-one;
(S)-5-((((6-(((R)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)oxy)-2-methoxy-5-(trifluoromethyl)pyridin-3-yl)methyl)amino)methyl)pyrrolidin-2-one;
(R)-5-((((6-(((R)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)oxy)-2-methoxy-5-(trifluoromethyl)pyridin-3-yl)methyl)amino)methyl)pyrrolidin-2-one;
(R)-5-((((6-(((R)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)oxy)-2-methoxy-5-(trifluoromethyl)pyridin-3-yl)methyl)amino)methyl)pyrrolidin-2-one;
(R)-5-((((6-(((S)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)oxy)-2-methoxy-5-(trifluoromethyl)pyridin-3-yl)methyl)amino)methyl)pyrrolidin-2-one;
(R)-5-((((6-(((S)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)oxy)-2-methoxy-5-(trifluoromethyl)pyridin-3-yl)methyl)amino)methyl)pyrrolidin-2-one;
2-((6-(((S)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)oxy)-2-methoxy-5-(trifluoromethyl)pyridin-3-yl)methyl)-2,6-diazaspiro[3.4]octan-7-one;
2-((6-(((S)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)oxy)-2-methoxy-5-(trifluoromethyl)pyridin-3-yl)methyl)-2,6-diazaspiro[3.4]octan-7-one;
2-((6-(((R)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)oxy)-2-methoxy-5-(trifluoromethyl)pyridin-3-yl)methyl)-2,6-diazaspiro[3.4]octan-7-one;
2-((6-(((R)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)oxy)-2-methoxy-5-(trifluoromethyl)pyridin-3-yl)methyl)-2,6-diazaspiro[3.4]octan-7-one;
(S)-1-(6-((5-(3-chloro-2-(3-methoxy-4-((methylamino)methyl)phenyl)pyridin-4-yl)-1,2,3,4-tetrahydronaphthalen-1-yl)oxy)-2-methoxy-5-(trifluoromethyl)pyridin-3-yl)-N-methylmethanamine;
(R)-1-(6-((5-(3-chloro-2-(3-methoxy-4-((methylamino)methyl)phenyl)pyridin-4-yl)-1,2,3,4-tetrahydronaphthalen-1-yl)oxy)-2-methoxy-5-(trifluoromethyl)pyridin-3-yl)-N-methylmethanamine;
(S)-5-(((4-(3-chloro-4-(2-chloro-3-(8-chloro-6-((((S)-2-hydroxypropyl)amino)methyl)-5-methoxyquinolin-2-yl)phenyl)pyridin-2-yl)-2-methoxybenzyl)amino)methyl)pyrrolidin-2-one;
(S)-5-(((4-(3-chloro-4-(2-chloro-3-(8-chloro-6-((((R)-2-hydroxypropyl)amino)methyl)-5-methoxyquinolin-2-yl)phenyl)pyridin-2-yl)-2-methoxybenzyl)amino)methyl)pyrrolidin-2-one;
(R)-5-(((4-(3-chloro-4-(2-chloro-3-(8-chloro-6-((((S)-2-hydroxypropyl)amino)methyl)-5-methoxyquinolin-2-yl)phenyl)pyridin-2-yl)-2-methoxybenzyl)amino)methyl)pyrrolidin-2-one;
(R)-5-(((4-(3-chloro-4-(2-chloro-3-(8-chloro-6-((((R)-2-hydroxypropyl)amino)methyl)-5-methoxyquinolin-2-yl)phenyl)pyridin-2-yl)-2-methoxybenzyl)amino)methyl)pyrrolidin-2-one;
(S)-5-((((8-chloro-2-(2-chloro-3-(3-chloro-2-(3-methoxy-4-(((((S)-5-oxopyrrolidin-2-yl)methyl)amino)methyl)phenyl)pyridin-4-yl)phenyl)-5-methoxyquinolin-6-yl)methyl)amino)methyl)pyrrolidin-2-one;
(S)-5-((((8-chloro-2-(2-chloro-3-(3-chloro-2-(3-methoxy-4-(((((R)-5-oxopyrrolidin-2-yl)methyl)amino)methyl)phenyl)pyridin-4-yl)phenyl)-5-methoxyquinolin-6-yl)methyl)amino)methyl)pyrrolidin-2-one;
(R)-5-((((8-chloro-2-(2-chloro-3-(3-chloro-2-(3-methoxy-4-(((((S)-5-oxopyrrolidin-2-yl)methyl)amino)methyl)phenyl)pyridin-4-yl)phenyl)-5-methoxyquinolin-6-yl)methyl)amino)methyl)pyrrolidin-2-one;
(R)-5-((((8-chloro-2-(2-chloro-3-(3-chloro-2-(3-methoxy-4-(((((R)-5-oxopyrrolidin-2-yl)methyl)amino)methyl)phenyl)pyridin-4-yl)phenyl)-5-methoxyquinolin-6-yl)methyl)amino)methyl)pyrrolidin-2-one;
(S)-5-((((6-(2-chloro-3-(3-chloro-2-(8-chloro-2-(3-hydroxy-3-methylbutyl)-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(8-chloro-2-(3-hydroxy-3-methylbutyl)-1,2,3,4-tetrahydroisoquinolin-6-yl)pyridin-4-yl)phenyl)-2-methoxypyridin-3-yl)methyl)amino)methyl)pyrrolidin-2-one;
isopropyl (S)-4-((4-(3-chloro-4-(2-chloro-3-(5-(4,5-dihydro-1H-imidazol-2-yl)-6-methoxypyridin-2-yl)phenyl)pyridin-2-yl)-2-methoxybenzyl)amino)-3-hydroxybutanoate;
isopropyl (R)-4-((4-(3-chloro-4-(2-chloro-3-(5-(4,5-dihydro-1H-imidazol-2-yl)-6-methoxypyridin-2-yl)phenyl)pyridin-2-yl)-2-methoxybenzyl)amino)-3-hydroxybutanoate;
(S)-5-((((6-(3-(2-(4-(((2-acetyl-2-azaspiro[3.3]heptan-6-yl)amino)methyl)-3-fluoro-5-methoxyphenyl)-3-chloropyridin-4-yl)-2-chlorophenyl)-2-methoxypyridin-3-yl)methyl)amino)methyl)pyrrolidin-2-one;
(R)-5-((((6-(3-(2-(4-(((2-acetyl-2-azaspiro[3.3]heptan-6-yl)amino)methyl)-3-fluoro-5-methoxyphenyl)-3-chloropyridin-4-yl)-2-chlorophenyl)-2-methoxypyridin-3-yl)methyl)amino)methyl)pyrrolidin-2-one;
N-(4-(3-chloro-4-(2-chloro-3-(6-methoxy-5-((methylamino)methyl)pyridin-2-yl)phenyl)pyridin-2-yl)-2-methoxybenzyl)propan-2-amine;
1-(4-((4-(3-chloro-4-(2-chloro-3-(6-methoxy-5-((methylamino)methyl)pyridin-2-yl)phenyl)pyridin-2-yl)-2-methoxybenzyl)amino)piperidin-1-yl)ethan-1-one;
2-(4-(3-chloro-4-(2-chloro-3-(6-methoxy-5-((methylamino)methyl)pyridin-2-yl)phenyl)pyridin-2-yl)-2-methoxybenzyl)-2,6-diazaspiro[3.4]octan-7-one;
(S)-1-(((6-(2-chloro-3-(3-chloro-2-(2-((S)-2-hydroxypropyl)-8-methoxy-1,2,3,4-tetrahydroisoquinolin-6-yl)pyridin-4-yl)phenyl)-2-methoxypyridin-3-yl)methyl)amino)propan-2-ol;
(S)-1-(((6-(2-chloro-3-(3-chloro-2-(2-((R)-2-hydroxypropyl)-8-methoxy-1,2,3,4-tetrahydroisoquinolin-6-yl)pyridin-4-yl)phenyl)-2-methoxypyridin-3-yl)methyl)amino)propan-2-ol;
(R)-1-(((6-(2-chloro-3-(3-chloro-2-(2-((S)-2-hydroxypropyl)-8-methoxy-1,2,3,4-tetrahydroisoquinolin-6-yl)pyridin-4-yl)phenyl)-2-methoxypyridin-3-yl)methyl)amino)propan-2-ol;
(R)-1-(((6-(2-chloro-3-(3-chloro-2-(2-((R)-2-hydroxypropyl)-8-methoxy-1,2,3,4-tetrahydroisoquinolin-6-yl)pyridin-4-yl)phenyl)-2-methoxypyridin-3-yl)methyl)amino)propan-2-ol;
(1r,3r)-3-(((6-(2-chloro-3-(3-chloro-2-(2-((S)-2-hydroxypropyl)-8-methoxy-1,2,3,4-tetrahydroisoquinolin-6-yl)pyridin-4-yl)phenyl)-2-methoxypyridin-3-yl)methyl)amino)-1-methylcyclobutan-1-ol;
(1r,3r)-3-(((6-(2-chloro-3-(3-chloro-2-(2-((R)-2-hydroxypropyl)-8-methoxy-1,2,3,4-tetrahydroisoquinolin-6-yl)pyridin-4-yl)phenyl)-2-methoxypyridin-3-yl)methyl)amino)-1-methylcyclobutan-1-ol;
(1r,3s)-3-(((6-(2-chloro-3-(3-chloro-2-(2-((S)-2-hydroxypropyl)-8-methoxy-1,2,3,4-tetrahydroisoquinolin-6-yl)pyridin-4-yl)phenyl)-2-methoxypyridin-3-yl)methyl)amino)-1-methylcyclobutan-1-ol;
(1s,3s)-3-(((6-(2-chloro-3-(3-chloro-2-(2-((S)-2-hydroxypropyl)-8-methoxy-1,2,3,4-tetrahydroisoquinolin-6-yl)pyridin-4-yl)phenyl)-2-methoxypyridin-3-yl)methyl)amino)-1-methylcyclobutan-1-ol;
(1s,3s)-3-(((6-(2-chloro-3-(3-chloro-2-(2-((R)-2-hydroxypropyl)-8-methoxy-1,2,3,4-tetrahydroisoquinolin-6-yl)pyridin-4-yl)phenyl)-2-methoxypyridin-3-yl)methyl)amino)-1-methylcyclobutan-1-ol;
(1s,3r)-3-(((6-(2-chloro-3-(3-chloro-2-(2-((R)-2-hydroxypropyl)-8-methoxy-1,2,3,4-tetrahydroisoquinolin-6-yl)pyridin-4-yl)phenyl)-2-methoxypyridin-3-yl)methyl)amino)-1-methylcyclobutan-1-ol;
(1s,3r)-3-(((6-(2-chloro-3-(3-chloro-2-(2-((S)-2-hydroxypropyl)-8-methoxy-1,2,3,4-tetrahydroisoquinolin-6-yl)pyridin-4-yl)phenyl)-2-methoxypyridin-3-yl)methyl)amino)-1-methylcyclobutan-1-ol;
(1r,3s)-3-(((6-(2-chloro-3-(3-chloro-2-(2-((R)-2-hydroxypropyl)-8-methoxy-1,2,3,4-tetrahydroisoquinolin-6-yl)pyridin-4-yl)phenyl)-2-methoxypyridin-3-yl)methyl)amino)-1-methylcyclobutan-1-ol;
(S)-1-(6-(3-chloro-4-(2-chloro-3-(6-methoxy-5-(((((S)-tetrahydrofuran-2-yl)methyl)amino)methyl)pyridin-2-yl)phenyl)pyridin-2-yl)-8-methoxy-3,4-dihydroisoquinolin-2(1H)-yl)propan-2-ol;
(S)-1-(6-(3-chloro-4-(2-chloro-3-(6-methoxy-5-(((((R)-tetrahydrofuran-2-yl)methyl)amino)methyl)pyridin-2-yl)phenyl)pyridin-2-yl)-8-methoxy-3,4-dihydroisoquinolin-2(1H)-yl)propan-2-ol;
(R)-1-(6-(3-chloro-4-(2-chloro-3-(6-methoxy-5-(((((S)-tetrahydrofuran-2-yl)methyl)amino)methyl)pyridin-2-yl)phenyl)pyridin-2-yl)-8-methoxy-3,4-dihydroisoquinolin-2(1H)-yl)propan-2-ol;
(R)-1-(6-(3-chloro-4-(2-chloro-3-(6-methoxy-5-(((((R)-tetrahydrofuran-2-yl)methyl)amino)methyl)pyridin-2-yl)phenyl)pyridin-2-yl)-8-methoxy-3,4-dihydroisoquinolin-2(1H)-yl)propan-2-ol;
(S)-1-((6-(2-chloro-3-(3-chloro-2-(2-(2-hydroxypropyl)-8-methoxy-1,2,3,4-tetrahydroisoquinolin-6-yl)pyridin-4-yl)phenyl)-2-methoxypyridin-3-yl)methyl)-3-methylazetidin-3-ol;
(R)-1-((6-(2-chloro-3-(3-chloro-2-(2-(2-hydroxypropyl)-8-methoxy-1,2,3,4-tetrahydroisoquinolin-6-yl)pyridin-4-yl)phenyl)-2-methoxypyridin-3-yl)methyl)-3-methylazetidin-3-ol;
methyl (S)-((6-(2-chloro-3-(3-chloro-2-(2-(2-hydroxypropyl)-8-methoxy-1,2,3,4-tetrahydroisoquinolin-6-yl)pyridin-4-yl)phenyl)-2-methoxypyridin-3-yl)methyl)glycinate;
methyl (R)-((6-(2-chloro-3-(3-chloro-2-(2-(2-hydroxypropyl)-8-methoxy-1,2,3,4-tetrahydroisoquinolin-6-yl)pyridin-4-yl)phenyl)-2-methoxypyridin-3-yl)methyl)glycinate;
(S)-2-((6-(2-chloro-3-(3-chloro-2-(3-methoxy-4-((((5-oxopyrrolidin-2-yl)methyl)amino)methyl)phenyl)pyridin-4-yl)phenyl)-1-methyl-1H-pyrrolo[2,3-b]pyridin-3-yl)methyl)-2,6-diazaspiro[3.4]octan-7-one;
(R)-2-((6-(2-chloro-3-(3-chloro-2-(3-methoxy-4-((((5-oxopyrrolidin-2-yl)methyl)amino)methyl)phenyl)pyridin-4-yl)phenyl)-1-methyl-1H-pyrrolo[2,3-b]pyridin-3-yl)methyl)-2,6-diazaspiro[3.4]octan-7-one;
(S)-4-((4-(3-chloro-4-(2-chloro-3-(6-methoxy-5-((methylamino)methyl)pyridin-2-yl)phenyl)pyridin-2-yl)-2-methoxybenzyl)amino)-3-hydroxybutanoic acid;
(R)-4-((4-(3-chloro-4-(2-chloro-3-(6-methoxy-5-((methylamino)methyl)pyridin-2-yl)phenyl)pyridin-2-yl)-2-methoxybenzyl)amino)-3-hydroxybutanoic acid;
3-((4-(3-chloro-4-(2-chloro-3-(6-methoxy-5-((methylamino)methyl)pyridin-2-yl)phenyl)pyridin-2-yl)-2-methoxybenzyl)amino)propanoic acid;
6-(3-chloro-4-(2-chloro-3-(5-((6-hydroxy-2-azaspiro[3.3]heptan-2-yl)methyl)-6-methoxypyridin-2-yl)phenyl)pyridin-2-yl)-2-((3-hydroxy-3-methylazetidin-1-yl)methyl)-3-methylpyrrolo[2,1-f][1,2,4]triazin-4(3H)-one;
(S)-5-((((3'-chloro-6-methoxy-2'-((S)-1-((6-methoxy-5-(((((S)-5-oxopyrrolidin-2-yl)methyl)amino)methyl)-3-(trifluoromethyl)pyridin-2-yl)amino)-2,3-dihydro-1H-inden-4-yl)-[2,4'-bipyridin]-5-yl)methyl)amino)methyl)pyrrolidin-2-one;
(R)-5-((((3'-chloro-6-methoxy-2'-((R)-1-((6-methoxy-5-(((((R)-5-oxopyrrolidin-2-yl)methyl)amino)methyl)-3-(trifluoromethyl)pyridin-2-yl)amino)-2,3-dihydro-1H-inden-4-yl)-[2,4'-bipyridin]-5-yl)methyl)amino)methyl)pyrrolidin-2-one;
(R)-5-((((3'-chloro-6-methoxy-2'-((R)-1-((6-methoxy-5-(((((S)-5-oxopyrrolidin-2-yl)methyl)amino)methyl)-3-(trifluoromethyl)pyridin-2-yl)amino)-2,3-dihydro-1H-inden-4-yl)-[2,4'-bipyridin]-5-yl)methyl)amino)methyl)pyrrolidin-2-one;
(R)-5-((((3'-chloro-6-methoxy-2'-((S)-1-((6-methoxy-5-(((((S)-5-oxopyrrolidin-2-yl)methyl)amino)methyl)-3-(trifluoromethyl)pyridin-2-yl)amino)-2,3-dihydro-1H-inden-4-yl)-[2,4'-bipyridin]-5-yl)methyl)amino)methyl)pyrrolidin-2-one;
(R)-5-((((3'-chloro-6-methoxy-2'-((S)-1-((6-methoxy-5-(((((R)-5-oxopyrrolidin-2-yl)methyl)amino)methyl)-3-(trifluoromethyl)pyridin-2-yl)amino)-2,3-dihydro-1H-inden-4-yl)-[2,4'-bipyridin]-5-yl)methyl)amino)methyl)pyrrolidin-2-one;
(S)-5-((((3'-chloro-6-methoxy-2'-((S)-1-((6-methoxy-5-(((((R)-5-oxopyrrolidin-2-yl)methyl)amino)methyl)-3-(trifluoromethyl)pyridin-2-yl)amino)-2,3-dihydro-1H-inden-4-yl)-[2,4'-bipyridin]-5-yl)methyl)amino)methyl)pyrrolidin-2-one;
(S)-5-((((3'-chloro-6-methoxy-2'-((R)-1-((6-methoxy-5-(((((R)-5-oxopyrrolidin-2-yl)methyl)amino)methyl)-3-(trifluoromethyl)pyridin-2-yl)amino)-2,3-dihydro-1H-inden-4-yl)-[2,4'-bipyridin]-5-yl)methyl)amino)methyl)pyrrolidin-2-one;
(S)-5-((((3'-chloro-6-methoxy-2'-((R)-1-((6-methoxy-5-(((((S)-5-oxopyrrolidin-2-yl)methyl)amino)methyl)-3-(trifluoromethyl)pyridin-2-yl)amino)-2,3-dihydro-1H-inden-4-yl)-[2,4'-bipyridin]-5-yl)methyl)amino)methyl)pyrrolidin-2-one;
(S)-N-(4-(3'-chloro-6-methoxy-5-((methylamino)methyl)-[2,4'-bipyridin]-2'-yl)-2,3-dihydro-1H-inden-1-yl)-6-methoxy-5-((methylamino)methyl)-3-(trifluoromethyl)pyridin-2-amine;
(R)-N-(4-(3'-chloro-6-methoxy-5-((methylamino)methyl)-[2,4'-bipyridin]-2'-yl)-2,3-dihydro-1H-inden-1-yl)-6-methoxy-5-((methylamino)methyl)-3-(trifluoromethyl)pyridin-2-amine;
(1S,3r)-3-(((3'-chloro-2'-((S)-1-((5-((((1r,3S)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1R,3r)-3-(((3'-chloro-2'-((R)-1-((5-((((1r,3R)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1R,3r)-3-(((3'-chloro-2'-((R)-1-((5-((((1r,3S)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1R,3r)-3-(((3'-chloro-2'-((R)-1-((5-((((1s,3R)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1R,3r)-3-(((3'-chloro-2'-((R)-1-((5-((((1s,3S)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1R,3r)-3-(((3'-chloro-2'-((S)-1-((5-((((1r,3R)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1R,3r)-3-(((3'-chloro-2'-((S)-1-((5-((((1r,3S)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1R,3r)-3-(((3'-chloro-2'-((S)-1-((5-((((1s,3R)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1R,3r)-3-(((3'-chloro-2'-((S)-1-((5-((((1s,3S)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1R,3s)-3-(((3'-chloro-2'-((R)-1-((5-((((1r,3R)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1R,3s)-3-(((3'-chloro-2'-((R)-1-((5-((((1r,3S)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1R,3s)-3-(((3'-chloro-2'-((R)-1-((5-((((1s,3R)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1R,3s)-3-(((3'-chloro-2'-((R)-1-((5-((((1s,3S)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1R,3r)-3-(((3'-chloro-2'-((R)-1-((5-((((1r,3R)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1R,3s)-3-(((3'-chloro-2'-((S)-1-((5-((((1r,3S)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1R,3s)-3-(((3'-chloro-2'-((S)-1-((5-((((1s,3R)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1R,3s)-3-(((3'-chloro-2'-((S)-1-((5-((((1s,3S)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1S,3r)-3-(((3'-chloro-2'-((R)-1-((5-((((1r,3R)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1S,3r)-3-(((3'-chloro-2'-((R)-1-((5-((((1r,3S)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1S,3r)-3-(((3'-chloro-2'-((R)-1-((5-((((1s,3R)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1S,3r)-3-(((3'-chloro-2'-((R)-1-((5-((((1s,3S)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1S,3r)-3-(((3'-chloro-2'-((S)-1-((5-((((1r,3R)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1S,3r)-3-(((3'-chloro-2'-((S)-1-((5-((((1s,3R)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1S,3r)-3-(((3'-chloro-2'-((S)-1-((5-((((1s,3S)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1S,3s)-3-(((3'-chloro-2'-((R)-1-((5-((((1r,3R)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1S,3s)-3-(((3'-chloro-2'-((R)-1-((5-((((1r,3S)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1S,3s)-3-(((3'-chloro-2'-((R)-1-((5-((((1s,3R)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1S,3s)-3-(((3'-chloro-2'-((R)-1-((5-((((1s,3S)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1S,3r)-3-(((3'-chloro-2'-((R)-1-((5-((((1r,3R)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1S,3s)-3-(((3'-chloro-2'-((S)-1-((5-((((1r,3S)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1S,3s)-3-(((3'-chloro-2'-((S)-1-((5-((((1s,3R)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(1S,3s)-3-(((3'-chloro-2'-((S)-1-((5-((((1s,3S)-3-hydroxy-3-methylcyclobutyl)amino)methyl)-6-methoxy-3-(trifluoromethyl)pyridin-2-yl)amino)-2,3-dihydro-1H-inden-4-yl)-6-methoxy-[2,4'-bipyridin]-5-yl)methyl)amino)-1-methylcyclobutan-1-ol;
(S)-1-(4-(3-chloro-4-(2-chloro-3-(6-methoxy-5-((methylamino)methyl)pyridin-2-yl)phenyl)pyridin-2-yl)-2-methoxybenzyl)pyrrolidine-3-carboxylic acid;
(R)-1-(4-(3-chloro-4-(2-chloro-3-(6-methoxy-5-((methylamino)methyl)pyridin-2-yl)phenyl)pyridin-2-yl)-2-methoxybenzyl)pyrrolidine-3-carboxylic acid;
2-(4-(3-chloro-4-(2-chloro-3-(6-methoxy-5-((methylamino)methyl)pyridin-2-yl)phenyl)pyridin-2-yl)-2-methoxybenzyl)-2-azaspiro[3.3]heptane-6-carboxylic acid;
3-(((4-(3-chloro-4-(2-chloro-3-(6-methoxy-5-((methylamino)methyl)pyridin-2-yl)phenyl)pyridin-2-yl)-2-methoxybenzyl)amino)methyl)bicyclo[1.1.1]pentane-1-carboxylic acid;
(S)-((6-(2-chloro-3-(3-chloro-2-(2-(2-hydroxypropyl)-8-methoxy-1,2,3,4-tetrahydroisoquinolin-6-yl)pyridin-4-yl)phenyl)-2-methoxypyridin-3-yl)methyl)glycine;
(R)-((6-(2-chloro-3-(3-chloro-2-(2-(2-hydroxypropyl)-8-methoxy-1,2,3,4-tetrahydroisoquinolin-6-yl)pyridin-4-yl)phenyl)-2-methoxypyridin-3-yl)methyl)glycine;
2-((6-(2-chloro-3-(3-chloro-2-(isoindolin-5-yl)pyridin-4-yl)phenyl)-2-methoxypyridin-3-yl)methyl)-2,6-diazaspiro[3.4]octan-7-one;
6-(3-chloro-4-(2-chloro-3-(5-((3-hydroxy-3-methylazetidin-1-yl)methyl)-6-methoxypyridin-2-yl)phenyl)pyridin-2-yl)-2-((3-hydroxy-3-methylazetidin-1-yl)methyl)-3-methylpyrrolo[2,1-f][1,2,4]triazin-4(3H)-one;
6-(3-chloro-4-(2-chloro-3-(5-((((1r,3r)-3-hydroxy-3-methylcyclobutyl)amino)methyl)-6-methoxypyridin-2-yl)phenyl)pyridin-2-yl)-2-((3-hydroxy-3-methylazetidin-1-yl)methyl)-3-methylpyrrolo[2,1-f][1,2,4]triazin-4(3H)-one;
6-(3-chloro-4-(2-chloro-3-(5-((((1r,3s)-3-hydroxy-3-methylcyclobutyl)amino)methyl)-6-methoxypyridin-2-yl)phenyl)pyridin-2-yl)-2-((3-hydroxy-3-methylazetidin-1-yl)methyl)-3-methylpyrrolo[2,1-f][1,2,4]triazin-4(3H)-one;
6-(3-chloro-4-(2-chloro-3-(5-((((1s,3r)-3-hydroxy-3-methylcyclobutyl)amino)methyl)-6-methoxypyridin-2-yl)phenyl)pyridin-2-yl)-2-((3-hydroxy-3-methylazetidin-1-yl)methyl)-3-methylpyrrolo[2,1-f][1,2,4]triazin-4(3H)-one;
6-(3-chloro-4-(2-chloro-3-(5-((((1s,3s)-3-hydroxy-3-methylcyclobutyl)amino)methyl)-6-methoxypyridin-2-yl)phenyl)pyridin-2-yl)-2-((3-hydroxy-3-methylazetidin-1-yl)methyl)-3-methylpyrrolo[2,1-f][1,2,4]triazin-4(3H)-one;
6-(3-chloro-4-(2-chloro-3-(5-((isopropylamino)methyl)-6-methoxypyridin-2-yl)phenyl)pyridin-2-yl)-2-((3-hydroxy-3-methylazetidin-1-yl)methyl)-3-methylpyrrolo[2,1-f][1,2,4]triazin-4(3H)-one;
6-(3-chloro-4-(2-chloro-3-(5-((((1r,4r)-4-hydroxycyclohexyl)amino)methyl)-6-methoxypyridin-2-yl)phenyl)pyridin-2-yl)-2-((3-hydroxy-3-methylazetidin-1-yl)methyl)-3-methylpyrrolo[2,1-f][1,2,4]triazin-4(3H)-one;
6-(3-chloro-4-(2-chloro-3-(5-((((1r,4s)-4-hydroxycyclohexyl)amino)methyl)-6-methoxypyridin-2-yl)phenyl)pyridin-2-yl)-2-((3-hydroxy-3-methylazetidin-1-yl)methyl)-3-methylpyrrolo[2,1-f][1,2,4]triazin-4(3H)-one;
6-(3-chloro-4-(2-chloro-3-(5-((((1s,4r)-4-hydroxycyclohexyl)amino)methyl)-6-methoxypyridin-2-yl)phenyl)pyridin-2-yl)-2-((3-hydroxy-3-methylazetidin-1-yl)methyl)-3-methylpyrrolo[2,1-f][1,2,4]triazin-4(3H)-one;
6-(3-chloro-4-(2-chloro-3-(5-((((1s,4s)-4-hydroxycyclohexyl)amino)methyl)-6-methoxypyridin-2-yl)phenyl)pyridin-2-yl)-2-((3-hydroxy-3-methylazetidin-1-yl)methyl)-3-methylpyrrolo[2,1-f][1,2,4]triazin-4(3H)-one;
(S)-5-((((6-(2-chloro-3-(3-chloro-2-(1-methyl-3-(((((S)-oxetan-2-yl)methyl)amino)methyl)-1H-indazol-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(1-methyl-3-(((((R)-oxetan-2-yl)methyl)amino)methyl)-1H-indazol-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(1-methyl-3-(((((S)-oxetan-2-yl)methyl)amino)methyl)-1H-indazol-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(1-methyl-3-(((((R)-oxetan-2-yl)methyl)amino)methyl)-1 H-indazol-6-yl)pyridin-4-yl)phenyl)-2-methoxypyridin-3-yl)methyl)amino)methyl)pyrrolidin-2-one;
(S)-5-((((8-chloro-2-(2-chloro-3-(3-chloro-2-(3-fluoro-5-methoxy-4-(((((S)-5-oxopyrrolidin-2-yl)methyl)amino)methyl)phenyl)pyridin-4-yl)phenyl)-5-methoxyquinolin-6-yl)methyl)amino)methyl)pyrrolidin-2-one;
(S)-5-((((8-chloro-2-(2-chloro-3-(3-chloro-2-(3-fluoro-5-methoxy-4-(((((R)-5-oxopyrrolidin-2-yl)methyl)amino)methyl)phenyl)pyridin-4-yl)phenyl)-5-methoxyquinolin-6-yl)methyl)amino)methyl)pyrrolidin-2-one;
(R)-5-((((8-chloro-2-(2-chloro-3-(3-chloro-2-(3-fluoro-5-methoxy-4-(((((S)-5-oxopyrrolidin-2-yl)methyl)amino)methyl)phenyl)pyridin-4-yl)phenyl)-5-methoxyquinolin-6-yl)methyl)amino)methyl)pyrrolidin-2-one;
(R)-5-((((8-chloro-2-(2-chloro-3-(3-chloro-2-(3-fluoro-5-methoxy-4-(((((R)-5-oxopyrrolidin-2-yl)methyl)amino)methyl)phenyl)pyridin-4-yl)phenyl)-5-methoxyquinolin-6-yl)methyl)amino)methyl)pyrrolidin-2-one;
(S)-5-((((6-(3-(2-(4-(((2-acetyl-2-azaspiro[3.3]heptan-6-yl)amino)methyl)-3-methoxyphenyl)-3-chloropyridin-4-yl)-2-chlorophenyl)-2-methoxypyridin-3-yl)methyl)amino)methyl)pyrrolidin-2-one;
(R)-5-((((6-(3-(2-(4-(((2-acetyl-2-azaspiro[3.3]heptan-6-yl)amino)methyl)-3-methoxyphenyl)-3-chloropyridin-4-yl)-2-chlorophenyl)-2-methoxypyridin-3-yl)methyl)amino)methyl)pyrrolidin-2-one;
(S)-5-(((4-(3-chloro-4-(2-chloro-3-(8-chloro-6-(((2-hydroxyethyl)amino)methyl)-5-methoxyquinolin-2-yl)phenyl)pyridin-2-yl)-2-methoxybenzyl)amino)methyl)pyrrolidin-2-one;
(R)-5-(((4-(3-chloro-4-(2-chloro-3-(8-chloro-6-(((2-hydroxyethyl)amino)methyl)-5-methoxyquinolin-2-yl)phenyl)pyridin-2-yl)-2-methoxybenzyl)amino)methyl)pyrrolidin-2-one;
(S)-1-(4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)pyrrolidine-3-carboxylic acid;
(R)-1-(4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)pyrrolidine-3-carboxylic acid;
1-(4-(((6-(2-chloro-3-(3-chloro-2-(3-methoxy-4-((methylamino)methyl)phenyl)pyridin-4-yl)phenyl)-2-methoxypyridin-3-yl)methyl)amino)piperidin-1-yl)ethan-1-one;
(S)-5-(((4-(4-(3-(6-(((1-acetylpiperidin-4-yl)amino)methyl)-8-chloro-5-methoxyquinolin-2-yl)-2-chlorophenyl)-3-chloropyridin-2-yl)-2-methoxybenzyl)amino)methyl)pyrrolidin-2-one;
(R)-5-(((4-(4-(3-(6-(((1-acetylpiperidin-4-yl)amino)methyl)-8-chloro-5-methoxyquinolin-2-yl)-2-chlorophenyl)-3-chloropyridin-2-yl)-2-methoxybenzyl)amino)methyl)pyrrolidin-2-one;
5-(3-chloro-4-(2-chloro-3'-fluoro-5'-methoxy-4'-(((((S)-5-oxopyrrolidin-2-yl)methyl)amino)methyl)-[1,1'-biphenyl]-3-yl)pyridin-2-yl)-2-(2-((((S)-5-oxopyrrolidin-2-yl)methyl)amino)ethyl)isoindolin-1-one;
5-(3-chloro-4-(2-chloro-3'-fluoro-5'-methoxy-4'-(((((S)-5-oxopyrrolidin-2-yl)methyl)amino)methyl)-[1,1'-biphenyl]-3-yl)pyridin-2-yl)-2-(2-((((R)-5-oxopyrrolidin-2-yl)methyl)amino)ethyl)isoindolin-1-one;
5-(3-chloro-4-(2-chloro-3'-fluoro-5'-methoxy-4'-(((((R)-5-oxopyrrolidin-2-yl)methyl)amino)methyl)-[1,1'-biphenyl]-3-yl)pyridin-2-yl)-2-(2-((((S)-5-oxopyrrolidin-2-yl)methyl)amino)ethyl)isoindolin-1-one;
5-(3-chloro-4-(2-chloro-3'-fluoro-5'-methoxy-4'-(((((R)-5-oxopyrrolidin-2-yl)methyl)amino)methyl)-[1,1'-biphenyl]-3-yl)pyridin-2-yl)-2-(2-((((R)-5-oxopyrrolidin-2-yl)methyl)amino)ethyl)isoindolin-1-one;
2-(4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)-2-azaspiro[3.3]heptane-6-carboxylic acid;
2-(4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)-2,6-diazaspiro[3.4]octan-7-one;
isopropyl (S)-1-(4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)pyrrolidine-3-carboxylate;
isopropyl (R)-1-(4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)pyrrolidine-3-carboxylate;
1-(4-((4-(4-(3-(3-(((1-acetylpiperidin-4-yl)amino)methyl)-1-methyl-1H-pyrrolo[2,3-b]pyridin-6-yl)-2-chlorophenyl)-3-chloropyridin-2-yl)-2-methoxybenzyl)amino)piperidin-1-yl)ethan-1-one;
(S)-5-((((6-(2-chloro-3-(3-chloro-2-(3-(((2-hydroxyethyl)(methyl)amino)methyl)-1-methyl-1H-indazol-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(3-(((2-hydroxyethyl)(methyl)amino)methyl)-1-methyl-1H-indazol-6-yl)pyridin-4-yl)phenyl)-2-methoxypyridin-3-yl)methyl)amino)methyl)pyrrolidin-2-one;
(S)-1-(6-((6-((4-(5-((6-acetyl-2,6-diazaspiro[3.3]heptan-2-yl)methyl)-3'-chloro-6-methoxy-[2,4'-bipyridin]-2'-yl)-2,3-dihydro-1H-inden-1-yl)oxy)-2-methoxy-5-(trifluoromethyl)pyridin-3-yl)methyl)-2,6-diazaspiro[3.3]heptan-2-yl)ethan-1-one;
(R)-1-(6-((6-((4-(5-((6-acetyl-2,6-diazaspiro[3.3]heptan-2-yl)methyl)-3'-chloro-6-methoxy-[2,4'-bipyridin]-2'-yl)-2,3-dihydro-1H-inden-1-yl)oxy)-2-methoxy-5-(trifluoromethyl)pyridin-3-yl)methyl)-2,6-diazaspiro[3.3]heptan-2-yl)ethan-1-one;
1-(6-((6-(2-chloro-3-(3-chloro-2-(2-(3-fluoropropyl)-8-methoxy-1,2,3,4-tetrahydroisoquinolin-6-yl)pyridin-4-yl)phenyl)-2-methoxypyridin-3-yl)methyl)-2,6-diazaspiro[3.3]heptan-2-yl)ethan-1-one;
2-((6-(2-chloro-3-(3-chloro-2-(2-(3-fluoropropyl)-8-methoxy-1,2,3,4-tetrahydroisoquinolin-6-yl)pyridin-4-yl)phenyl)-2-methoxypyridin-3-yl)methyl)-2,6-diazaspiro[3.4]octan-7-one;
N-((6-(2-chloro-3-(3-chloro-2-(2-(3-fluoropropyl)-8-methoxy-1,2,3,4-tetrahydroisoquinolin-6-yl)pyridin-4-yl)phenyl)-2-methoxypyridin-3-yl)methyl)-2-oxaspiro[3.3]heptan-6-amine;
(1-((((6-(2-chloro-3-(3-chloro-2-(2-(3-fluoropropyl)-8-methoxy-1,2,3,4-tetrahydroisoquinolin-6-yl)pyridin-4-yl)phenyl)-2-methoxypyridin-3-yl)methyl)amino)methyl)cyclopropyl)methanol;
(S)-1-(((3'-chloro-2'-((S)-1-((5-((((S)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)propan-2-ol;
(S)-1-(((3'-chloro-2'-((R)-1-((5-((((R)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)propan-2-ol;
(S)-1-(((3'-chloro-2'-((R)-1-((5-((((S)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)propan-2-ol;
(S)-1-(((3'-chloro-2'-((S)-1-((5-((((R)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)propan-2-ol;
(R)-1-(((3'-chloro-2'-((R)-1-((5-((((R)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)propan-2-ol;
(R)-1-(((3'-chloro-2'-((R)-1-((5-((((S)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)propan-2-ol;
(R)-1-(((3'-chloro-2'-((S)-1-((5-((((R)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)propan-2-ol;
(R)-1-(((3'-chloro-2'-((S)-1-((5-((((S)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)amino)propan-2-ol;
(S)-1-(4-(((6-((4-(5-(((1-acetylpiperidin-4-yl)amino)methyl)-3'-chloro-6-methoxy-[2,4'-bipyridin]-2'-yl)-2,3-dihydro-1H-inden-1-yl)oxy)-2-methoxy-5-(trifluoromethyl)pyridin-3-yl)methyl)amino)piperidin-1-yl)ethan-1-one;
(R)-1-(4-(((6-((4-(5-(((1-acetylpiperidin-4-yl)amino)methyl)-3'-chloro-6-methoxy-[2,4'-bipyridin]-2'-yl)-2,3-dihydro-1H-inden-1-yl)oxy)-2-methoxy-5-(trifluoromethyl)pyridin-3-yl)methyl)amino)piperidin-1-yl)ethan-1-one;
2-((6-(2-chloro-3-(3-chloro-2-((R)-5-((((S)-5-oxopyrrolidin-2-yl)methyl)amino)-5,6,7,8-tetrahydronaphthalen-2-yl)pyridin-4-yl)phenyl)-2-methoxypyridin-3-yl)methyl)-2,6-diazaspiro[3.4]octan-7-one;
2-((6-(2-chloro-3-(3-chloro-2-((S)-5-((((S)-5-oxopyrrolidin-2-yl)methyl)amino)-5,6,7,8-tetrahydronaphthalen-2-yl)pyridin-4-yl)phenyl)-2-methoxypyridin-3-yl)methyl)-2,6-diazaspiro[3.4]octan-7-one;
2-((6-(2-chloro-3-(3-chloro-2-((R)-5-((((R)-5-oxopyrrolidin-2-yl)methyl)amino)-5,6,7,8-tetrahydronaphthalen-2-yl)pyridin-4-yl)phenyl)-2-methoxypyridin-3-yl)methyl)-2,6-diazaspiro[3.4]octan-7-one;
2-((6-(2-chloro-3-(3-chloro-2-((S)-5-((((R)-5-oxopyrrolidin-2-yl)methyl)amino)-5,6,7,8-tetrahydronaphthalen-2-yl)pyridin-4-yl)phenyl)-2-methoxypyridin-3-yl)methyl)-2,6-diazaspiro[3.4]octan-7-one;
(S)-5-((((R)-6-(3-chloro-4-(2-chloro-3-(5-((((S)-2-hydroxypropyl)amino)methyl)-6-methoxypyridin-2-yl)phenyl)pyridin-2-yl)-1,2,3,4-tetrahydronaphthalen-1-yl)amino)methyl)pyrrolidin-2-one;
(S)-5-((((R)-6-(3-chloro-4-(2-chloro-3-(5-((((R)-2-hydroxypropyl)amino)methyl)-6-methoxypyridin-2-yl)phenyl)pyridin-2-yl)-1,2,3,4-tetrahydronaphthalen-1-yl)amino)methyl)pyrrolidin-2-one;
(S)-5-((((S)-6-(3-chloro-4-(2-chloro-3-(5-((((R)-2-hydroxypropyl)amino)methyl)-6-methoxypyridin-2-yl)phenyl)pyridin-2-yl)-1,2,3,4-tetrahydronaphthalen-1-yl)amino)methyl)pyrrolidin-2-one;
(S)-5-((((S)-6-(3-chloro-4-(2-chloro-3-(5-((((S)-2-hydroxypropyl)amino)methyl)-6-methoxypyridin-2-yl)phenyl)pyridin-2-yl)-1,2,3,4-tetrahydronaphthalen-1-yl)amino)methyl)pyrrolidin-2-one;
(R)-5-((((R)-6-(3-chloro-4-(2-chloro-3-(5-((((R)-2-hydroxypropyl)amino)methyl)-6-methoxypyridin-2-yl)phenyl)pyridin-2-yl)-1,2,3,4-tetrahydronaphthalen-1-yl)amino)methyl)pyrrolidin-2-one;
(R)-5-((((R)-6-(3-chloro-4-(2-chloro-3-(5-((((S)-2-hydroxypropyl)amino)methyl)-6-methoxypyridin-2-yl)phenyl)pyridin-2-yl)-1,2,3,4-tetrahydronaphthalen-1-yl)amino)methyl)pyrrolidin-2-one;
(R)-5-((((S)-6-(3-chloro-4-(2-chloro-3-(5-((((R)-2-hydroxypropyl)amino)methyl)-6-methoxypyridin-2-yl)phenyl)pyridin-2-yl)-1,2,3,4-tetrahydronaphthalen-1-yl)amino)methyl)pyrrolidin-2-one;
(R)-5-((((S)-6-(3-chloro-4-(2-chloro-3-(5-((((S)-2-hydroxypropyl)amino)methyl)-6-methoxypyridin-2-yl)phenyl)pyridin-2-yl)-1,2,3,4-tetrahydronaphthalen-1-yl)amino)methyl)pyrrolidin-2-one;
(S)-5-((((R)-6-(3-chloro-4-(2-chloro-3-(6-methoxy-5-((((S)-2-oxotetrahydrofuran-3-yl)amino)methyl)pyridin-2-yl)phenyl)pyridin-2-yl)-1,2,3,4-tetrahydronaphthalen-1-yl)amino)methyl)pyrrolidin-2-one;
(S)-5-((((S)-6-(3-chloro-4-(2-chloro-3-(6-methoxy-5-((((S)-2-oxotetrahydrofuran-3-yl)amino)methyl)pyridin-2-yl)phenyl)pyridin-2-yl)-1,2,3,4-tetrahydronaphthalen-1-yl)amino)methyl)pyrrolidin-2-one;
(S)-5-((((S)-6-(3-chloro-4-(2-chloro-3-(6-methoxy-5-((((R)-2-oxotetrahydrofuran-3-yl)amino)methyl)pyridin-2-yl)phenyl)pyridin-2-yl)-1,2,3,4-tetrahydronaphthalen-1-yl)amino)methyl)pyrrolidin-2-one;
(S)-5-((((R)-6-(3-chloro-4-(2-chloro-3-(6-methoxy-5-((((R)-2-oxotetrahydrofuran-3-yl)amino)methyl)pyridin-2-yl)phenyl)pyridin-2-yl)-1,2,3,4-tetrahydronaphthalen-1-yl)amino)methyl)pyrrolidin-2-one;
(R)-5-((((R)-6-(3-chloro-4-(2-chloro-3-(6-methoxy-5-((((S)-2-oxotetrahydrofuran-3-yl)amino)methyl)pyridin-2-yl)phenyl)pyridin-2-yl)-1,2,3,4-tetrahydronaphthalen-1-yl)amino)methyl)pyrrolidin-2-one;
(R)-5-((((S)-6-(3-chloro-4-(2-chloro-3-(6-methoxy-5-((((S)-2-oxotetrahydrofuran-3-yl)amino)methyl)pyridin-2-yl)phenyl)pyridin-2-yl)-1,2,3,4-tetrahydronaphthalen-1-yl)amino)methyl)pyrrolidin-2-one;
(R)-5-((((S)-6-(3-chloro-4-(2-chloro-3-(6-methoxy-5-((((R)-2-oxotetrahydrofuran-3-yl)amino)methyl)pyridin-2-yl)phenyl)pyridin-2-yl)-1,2,3,4-tetrahydronaphthalen-1-yl)amino)methyl)pyrrolidin-2-one;
(S)-5-((((S)-6-(3-chloro-4-(2-chloro-3-(6-methoxy-5-((((S)-2-oxotetrahydrofuran-3-yl)amino)methyl)pyridin-2-yl)phenyl)pyridin-2-yl)-1,2,3,4-tetrahydronaphthalen-1-yl)amino)methyl)pyrrolidin-2-one;
(S)-5-((((6-(2-chloro-3-(3-chloro-2-(8-methoxy-2-(((((S)-5-oxopyrrolidin-2-yl)methyl)amino)methyl)-[1,2,4]triazolo[1,5-a]pyridin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(8-methoxy-2-(((((R)-5-oxopyrrolidin-2-yl)methyl)amino)methyl)-[1,2,4]triazolo[1,5-a]pyridin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(8-methoxy-2-(((((S)-5-oxopyrrolidin-2-yl)methyl)amino)methyl)-[1,2,4]triazolo[1,5-a]pyridin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(8-methoxy-2-(((((R)-5-oxopyrrolidin-2-yl)methyl)amino)methyl)-[1,2,4]triazolo[1,5-a]pyridin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(2-((3-hydroxy-3-methylazetidin-1-yl)methyl)-8-methoxy-[1,2,4]triazolo[1,5-a]pyridin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-((3-hydroxy-3-methylazetidin-1-yl)methyl)-8-methoxy-[1,2,4]triazolo[1,5-a]pyridin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(2-((((1r,4r)-4-hydroxycyclohexyl)amino)methyl)-8-methoxy-[1,2,4]triazolo[1,5-a]pyridin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(2-((((1s,4s)-4-hydroxycyclohexyl)amino)methyl)-8-methoxy-[1,2,4]triazolo[1,5-a]pyridin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(2-((((1s,4r)-4-hydroxycyclohexyl)amino)methyl)-8-methoxy-[1,2,4]triazolo[1,5-a]pyridin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(2-((((1r,4s)-4-hydroxycyclohexyl)amino)methyl)-8-methoxy-[1,2,4]triazolo[1,5-a]pyridin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-((((1r,4s)-4-hydroxycyclohexyl)amino)methyl)-8-methoxy-[1,2,4]triazolo[1,5-a]pyridin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-((((1s,4s)-4-hydroxycyclohexyl)amino)methyl)-8-methoxy-[1,2,4]triazolo[1,5-a]pyridin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-((((1s,4r)-4-hydroxycyclohexyl)amino)methyl)-8-methoxy-[1,2,4]triazolo[1,5-a]pyridin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-((((1r,4r)-4-hydroxycyclohexyl)amino)methyl)-8-methoxy-[1,2,4]triazolo[1,5-a]pyridin-6-yl)pyridin-4-yl)phenyl)-2-methoxypyridin-3-yl)methyl)amino)methyl)pyrrolidin-2-one;
(S)-1-((6-(3-chloro-4-(2-chloro-3-(6-methoxy-5-((((5-oxopyrrolidin-2-yl)methyl)amino)methyl)pyridin-2-yl)phenyl)pyridin-2-yl)-8-methoxy-[1,2,4]triazolo[1,5-a]pyridin-2-yl)methyl)azetidine-3-carboxylic acid;
(R)-1-((6-(3-chloro-4-(2-chloro-3-(6-methoxy-5-((((5-oxopyrrolidin-2-yl)methyl)amino)methyl)pyridin-2-yl)phenyl)pyridin-2-yl)-8-methoxy-[1,2,4]triazolo[1,5-a]pyridin-2-yl)methyl)azetidine-3-carboxylic acid;
(S)-1-(2-(((6-(3-chloro-4-(2-chloro-3-(6-methoxy-5-((((5-oxopyrrolidin-2-yl)methyl)amino)methyl)pyridin-2-yl)phenyl)pyridin-2-yl)-8-methoxy-[1,2,4]triazolo[1,5-a]pyridin-2-yl)methyl)amino)ethyl)cyclopropane-1-carboxylic acid;
(R)-1-(2-(((6-(3-chloro-4-(2-chloro-3-(6-methoxy-5-((((5-oxopyrrolidin-2-yl)methyl)amino)methyl)pyridin-2-yl)phenyl)pyridin-2-yl)-8-methoxy-[1,2,4]triazolo[1,5-a]pyridin-2-yl)methyl)amino)ethyl)cyclopropane-1-carboxylic acid;
1-(4-(((6-(3-(2-(3-(((1-acetylpiperidin-4-yl)amino)methyl)-1-methyl-1H-indazol-6-yl)-3-chloropyridin-4-yl)-2-chlorophenyl)-2-methoxypyridin-3-yl)methyl)amino)piperidin-1-yl)ethan-1-one;
1-(4-((4-(4-(3-(3-(((1-acetylpiperidin-4-yl)(methyl)amino)methyl)-1-methyl-1H-pyrrolo[2,3-b]pyridin-6-yl)-2-chlorophenyl)-3-chloropyridin-2-yl)-2-methoxybenzyl)amino)piperidin-1-yl)ethan-1-one;
((6-(2-chloro-3-(3-chloro-2-((R)-5-((((S)-5-oxopyrrolidin-2-yl)methyl)amino)-5,6,7,8-tetrahydronaphthalen-2-yl)pyridin-4-yl)phenyl)-2-methoxypyridin-3-yl)methyl)-L-homoserine;
((6-(2-chloro-3-(3-chloro-2-((R)-5-((((R)-5-oxopyrrolidin-2-yl)methyl)amino)-5,6,7,8-tetrahydronaphthalen-2-yl)pyridin-4-yl)phenyl)-2-methoxypyridin-3-yl)methyl)-L-homoserine;
((6-(2-chloro-3-(3-chloro-2-((S)-5-((((S)-5-oxopyrrolidin-2-yl)methyl)amino)-5,6,7,8-tetrahydronaphthalen-2-yl)pyridin-4-yl)phenyl)-2-methoxypyridin-3-yl)methyl)-L-homoserine;
((6-(2-chloro-3-(3-chloro-2-((S)-5-((((R)-5-oxopyrrolidin-2-yl)methyl)amino)-5,6,7,8-tetrahydronaphthalen-2-yl)pyridin-4-yl)phenyl)-2-methoxypyridin-3-yl)methyl)-L-homoserine;
((6-(2-chloro-3-(3-chloro-2-((R)-5-((((R)-5-oxopyrrolidin-2-yl)methyl)amino)-5,6,7,8-tetrahydronaphthalen-2-yl)pyridin-4-yl)phenyl)-2-methoxypyridin-3-yl)methyl)-D-homoserine;
((6-(2-chloro-3-(3-chloro-2-((S)-5-((((S)-5-oxopyrrolidin-2-yl)methyl)amino)-5,6,7,8-tetrahydronaphthalen-2-yl)pyridin-4-yl)phenyl)-2-methoxypyridin-3-yl)methyl)-D-homoserine;
((6-(2-chloro-3-(3-chloro-2-((S)-5-((((R)-5-oxopyrrolidin-2-yl)methyl)amino)-5,6,7,8-tetrahydronaphthalen-2-yl)pyridin-4-yl)phenyl)-2-methoxypyridin-3-yl)methyl)-D-homoserine;
((6-(2-chloro-3-(3-chloro-2-((R)-5-((((S)-5-oxopyrrolidin-2-yl)methyl)amino)-5,6,7,8-tetrahydronaphthalen-2-yl)pyridin-4-yl)phenyl)-2-methoxypyridin-3-yl)methyl)-D-homoserine;
(5S,5'S)-5,5'-(((3,3'-dichloro-[4,4'-bipyridine]-2,2'-diyl)bis(8-methoxy-3,4-dihydroisoquinoline-6,2(1H)-diyl))bis(methylene))bis(pyrrolidin-2-one);
(5S,5'R)-5,5'-(((3,3'-dichloro-[4,4'-bipyridine]-2,2'-diyl)bis(8-methoxy-3,4-dihydroisoquinoline-6,2(1H)-diyl))bis(methylene))bis(pyrrolidin-2-one);
(5R,5'S)-5,5'-(((3,3'-dichloro-[4,4'-bipyridine]-2,2'-diyl)bis(8-methoxy-3,4-dihydroisoquinoline-6,2(1H)-diyl))bis(methylene))bis(pyrrolidin-2-one);
(5R,5'R)-5,5'-(((3,3'-dichloro-[4,4'-bipyridine]-2,2'-diyl)bis(8-methoxy-3,4-dihydroisoquinoline-6,2(1H)-diyl))bis(methylene))bis(pyrrolidin-2-one);
methyl ((3-chloro-4-(2-chloro-3-(6-methoxy-5-(((((S)-5-oxopyrrolidin-2-yl)methyl)amino)methyl)pyridin-2-yl)phenyl)-5'-methoxy-[2,3'-bipyridin]-6'-yl)methyl)-L-homoserinate;
methyl ((3-chloro-4-(2-chloro-3-(6-methoxy-5-(((((R)-5-oxopyrrolidin-2-yl)methyl)amino)methyl)pyridin-2-yl)phenyl)-5'-methoxy-[2,3'-bipyridin]-6'-yl)methyl)-L-homoserinate;
methyl ((3-chloro-4-(2-chloro-3-(6-methoxy-5-(((((R)-5-oxopyrrolidin-2-yl)methyl)amino)methyl)pyridin-2-yl)phenyl)-5'-methoxy-[2,3'-bipyridin]-6'-yl)methyl)-D-homoserinate;
methyl ((3-chloro-4-(2-chloro-3-(6-methoxy-5-(((((S)-5-oxopyrrolidin-2-yl)methyl)amino)methyl)pyridin-2-yl)phenyl)-5'-methoxy-[2,3'-bipyridin]-6'-yl)methyl)-D-homoserinate;
(S)-5-((((6-(2-chloro-3-(3-chloro-5'-methoxy-6'-((((S)-2-oxotetrahydrofuran-3-yl)amino)methyl)-[2,3'-bipyridin]-4-yl)phenyl)-2-methoxypyridin-3-yl)methyl)amino)methyl)pyrrolidin-2-one;
(S)-5-((((6-(2-chloro-3-(3-chloro-5'-methoxy-6'-((((R)-2-oxotetrahydrofuran-3-yl)amino)methyl)-[2,3'-bipyridin]-4-yl)phenyl)-2-methoxypyridin-3-yl)methyl)amino)methyl)pyrrolidin-2-one;
(R)-5-((((6-(2-chloro-3-(3-chloro-5'-methoxy-6'-((((S)-2-oxotetrahydrofuran-3-yl)amino)methyl)-[2,3'-bipyridin]-4-yl)phenyl)-2-methoxypyridin-3-yl)methyl)amino)methyl)pyrrolidin-2-one;
(R)-5-((((6-(2-chloro-3-(3-chloro-5'-methoxy-6'-((((R)-2-oxotetrahydrofuran-3-yl)amino)methyl)-[2,3'-bipyridin]-4-yl)phenyl)-2-methoxypyridin-3-yl)methyl)amino)methyl)pyrrolidin-2-one;
(S)-5-((((6-(((S)-4-(3-chloro-2-(3-methoxy-4-(((((S)-5-oxopyrrolidin-2-yl)methyl)amino)methyl)phenyl)pyridin-4-yl)-2,3-dihydro-1H-inden-1-yl)oxy)-2-methoxy-5-(trifluoromethyl)pyridin-3-yl)methyl)amino)methyl)pyrrolidin-2-one;
(S)-5-((((6-(((S)-4-(3-chloro-2-(3-methoxy-4-(((((R)-5-oxopyrrolidin-2-yl)methyl)amino)methyl)phenyl)pyridin-4-yl)-2,3-dihydro-1H-inden-1-yl)oxy)-2-methoxy-5-(trifluoromethyl)pyridin-3-yl)methyl)amino)methyl)pyrrolidin-2-one;
(S)-5-((((6-(((R)-4-(3-chloro-2-(3-methoxy-4-(((((S)-5-oxopyrrolidin-2-yl)methyl)amino)methyl)phenyl)pyridin-4-yl)-2,3-dihydro-1H-inden-1-yl)oxy)-2-methoxy-5-(trifluoromethyl)pyridin-3-yl)methyl)amino)methyl)pyrrolidin-2-one;
(S)-5-((((6-(((S)-4-(3-chloro-2-(3-methoxy-4-(((((R)-5-oxopyrrolidin-2-yl)methyl)amino)methyl)phenyl)pyridin-4-yl)-2,3-dihydro-1H-inden-1-yl)oxy)-2-methoxy-5-(trifluoromethyl)pyridin-3-yl)methyl)amino)methyl)pyrrolidin-2-one;
(R)-5-((((6-(((S)-4-(3-chloro-2-(3-methoxy-4-(((((S)-5-oxopyrrolidin-2-yl)methyl)amino)methyl)phenyl)pyridin-4-yl)-2,3-dihydro-1H-inden-1-yl)oxy)-2-methoxy-5-(trifluoromethyl)pyridin-3-yl)methyl)amino)methyl)pyrrolidin-2-one;
(R)-5-((((6-(((S)-4-(3-chloro-2-(3-methoxy-4-(((((R)-5-oxopyrrolidin-2-yl)methyl)amino)methyl)phenyl)pyridin-4-yl)-2,3-dihydro-1H-inden-1-yl)oxy)-2-methoxy-5-(trifluoromethyl)pyridin-3-yl)methyl)amino)methyl)pyrrolidin-2-one;
(R)-5-((((6-(((R)-4-(3-chloro-2-(3-methoxy-4-(((((S)-5-oxopyrrolidin-2-yl)methyl)amino)methyl)phenyl)pyridin-4-yl)-2,3-dihydro-1H-inden-1-yl)oxy)-2-methoxy-5-(trifluoromethyl)pyridin-3-yl)methyl)amino)methyl)pyrrolidin-2-one;
(R)-5-((((6-(((S)-4-(3-chloro-2-(3-methoxy-4-(((((R)-5-oxopyrrolidin-2-yl)methyl)amino)methyl)phenyl)pyridin-4-yl)-2,3-dihydro-1H-inden-1-yl)oxy)-2-methoxy-5-(trifluoromethyl)pyridin-3-yl)methyl)amino)methyl)pyrrolidin-2-one;
(S)-1-(((6-(((S)-4-(3-chloro-2-(4-((((S)-2-hydroxypropyl)amino)methyl)-3-methoxyphenyl)pyridin-4-yl)-2,3-dihydro-1H-inden-1-yl)oxy)-2-methoxy-5-(trifluoromethyl)pyridin-3-yl)methyl)amino)propan-2-ol;
(S)-1-(((6-(((S)-4-(3-chloro-2-(4-((((R)-2-hydroxypropyl)amino)methyl)-3-methoxyphenyl)pyridin-4-yl)-2,3-dihydro-1H-inden-1-yl)oxy)-2-methoxy-5-(trifluoromethyl)pyridin-3-yl)methyl)amino)propan-2-ol;
(S)-1-(((6-(((R)-4-(3-chloro-2-(4-((((S)-2-hydroxypropyl)amino)methyl)-3-methoxyphenyl)pyridin-4-yl)-2,3-dihydro-1H-inden-1-yl)oxy)-2-methoxy-5-(trifluoromethyl)pyridin-3-yl)methyl)amino)propan-2-ol;
(S)-1-(((6-(((R)-4-(3-chloro-2-(4-((((R)-2-hydroxypropyl)amino)methyl)-3-methoxyphenyl)pyridin-4-yl)-2,3-dihydro-1H-inden-1-yl)oxy)-2-methoxy-5-(trifluoromethyl)pyridin-3-yl)methyl)amino)propan-2-ol;
(R)-1-(((6-(((S)-4-(3-chloro-2-(4-((((S)-2-hydroxypropyl)amino)methyl)-3-methoxyphenyl)pyridin-4-yl)-2,3-dihydro-1H-inden-1-yl)oxy)-2-methoxy-5-(trifluoromethyl)pyridin-3-yl)methyl)amino)propan-2-ol;
(R)-1-(((6-(((S)-4-(3-chloro-2-(4-((((R)-2-hydroxypropyl)amino)methyl)-3-methoxyphenyl)pyridin-4-yl)-2,3-dihydro-1H-inden-1-yl)oxy)-2-methoxy-5-(trifluoromethyl)pyridin-3-yl)methyl)amino)propan-2-ol;
(R)-1-(((6-(((R)-4-(3-chloro-2-(4-((((S)-2-hydroxypropyl)amino)methyl)-3-methoxyphenyl)pyridin-4-yl)-2,3-dihydro-1H-inden-1-yl)oxy)-2-methoxy-5-(trifluoromethyl)pyridin-3-yl)methyl)amino)propan-2-ol;
(R)-1-(((6-(((R)-4-(3-chloro-2-(4-((((R)-2-hydroxypropyl)amino)methyl)-3-methoxyphenyl)pyridin-4-yl)-2,3-dihydro-1H-inden-1-yl)oxy)-2-methoxy-5-(trifluoromethyl)pyridin-3-yl)methyl)amino)propan-2-ol;
1-(4-(((6-(3-(2-(4-(((1-acetylpiperidin-4-yl)amino)methyl)-3-methoxyphenyl)-3-methylpyridin-4-yl)-2-chlorophenyl)-2-methoxypyridin-3-yl)methyl)amino)piperidin-1-yl)ethan-1-one;
methyl (S)-3-(6-(3-chloro-4-(2-chloro-3-(5-(((2-hydroxypropyl)amino)methyl)-6-methoxypyridin-2-yl)phenyl)pyridin-2-yl)-8-methoxy-3,4-dihydroisoquinolin-2(1H)-yl)propanoate;
methyl (R)-3-(6-(3-chloro-4-(2-chloro-3-(5-(((2-hydroxypropyl)amino)methyl)-6-methoxypyridin-2-yl)phenyl)pyridin-2-yl)-8-methoxy-3,4-dihydroisoquinolin-2(1H)-yl)propanoate;
methyl 3-(6-(3-chloro-4-(2-chloro-3-(6-methoxy-5-((7-oxo-2,6-diazaspiro[3.4]octan-2-yl)methyl)pyridin-2-yl)phenyl)pyridin-2-yl)-8-methoxy-3,4-dihydroisoquinolin-2(1H)-yl)propanoate;
(S)-1-(((6-(2-chloro-3-(2-(4-((((S)-2-hydroxypropyl)amino)methyl)-3-methoxyphenyl)-3-methylpyridin-4-yl)phenyl)-2-methoxypyridin-3-yl)methyl)amino)propan-2-ol;
(S)-1-(((6-(2-chloro-3-(2-(4-((((R)-2-hydroxypropyl)amino)methyl)-3-methoxyphenyl)-3-methylpyridin-4-yl)phenyl)-2-methoxypyridin-3-yl)methyl)amino)propan-2-ol;
(R)-1-(((6-(2-chloro-3-(2-(4-((((S)-2-hydroxypropyl)amino)methyl)-3-methoxyphenyl)-3-methylpyridin-4-yl)phenyl)-2-methoxypyridin-3-yl)methyl)amino)propan-2-ol;
(R)-1-(((6-(2-chloro-3-(2-(4-((((R)-2-hydroxypropyl)amino)methyl)-3-methoxyphenyl)-3-methylpyridin-4-yl)phenyl)-2-methoxypyridin-3-yl)methyl)amino)propan-2-ol;
(S)-2-(6-(3-chloro-4-(2-chloro-3-(5-(((2-hydroxypropyl)amino)methyl)-6-methoxypyridin-2-yl)phenyl)pyridin-2-yl)-8-methoxy-3,4-dihydroisoquinolin-2(1H)-yl)acetamide;
(R)-2-(6-(3-chloro-4-(2-chloro-3-(5-(((2-hydroxypropyl)amino)methyl)-6-methoxypyridin-2-yl)phenyl)pyridin-2-yl)-8-methoxy-3,4-dihydroisoquinolin-2(1H)-yl)acetamide;
1-(6-(2-chloro-3-(3-chloro-2-(1-methyl-3-((methylamino)methyl)-1H-indol-6-yl)pyridin-4-yl)phenyl)-2-methoxypyridin-3-yl)-N-methylmethanamine;
1-(4-(((6-(3-(2-(3-(((1-acetylpiperidin-4-yl)amino)methyl)-1-methyl-1H-indol-6-yl)-3-chloropyridin-4-yl)-2-chlorophenyl)-2-methoxypyridin-3-yl)methyl)amino)piperidin-1-yl)ethan-1-one;
isopropyl (S)-1-(4-(3-chloro-4-(2-chloro-3-(6-methoxy-5-((methylamino)methyl)pyridin-2-yl)phenyl)pyridin-2-yl)-2-methoxybenzyl)pyrrolidine-3-carboxylate;
isopropyl (R)-1-(4-(3-chloro-4-(2-chloro-3-(6-methoxy-5-((methylamino)methyl)pyridin-2-yl)phenyl)pyridin-2-yl)-2-methoxybenzyl)pyrrolidine-3-carboxylate;
isopropyl 3-((4-(3-chloro-4-(2-chloro-3-(6-methoxy-5-((methylamino)methyl)pyridin-2-yl)phenyl)pyridin-2-yl)-2-methoxybenzyl)amino)propanoate;
isopropyl (S)-4-((4-(3-chloro-4-(2-chloro-3-(6-methoxy-5-((methylamino)methyl)pyridin-2-yl)phenyl)pyridin-2-yl)-2-methoxybenzyl)amino)-3-hydroxybutanoate;
isopropyl (R)-4-((4-(3-chloro-4-(2-chloro-3-(6-methoxy-5-((methylamino)methyl)pyridin-2-yl)phenyl)pyridin-2-yl)-2-methoxybenzyl)amino)-3-hydroxybutanoate;
isopropyl 2-(4-(3-chloro-4-(2-chloro-3-(6-methoxy-5-((methylamino)methyl)pyridin-2-yl)phenyl)pyridin-2-yl)-2-methoxybenzyl)-2-azaspiro[3.3]heptane-6-carboxylate;
1-(4-(((6-(3-(2-(4-(((1-acetylpiperidin-4-yl)amino)methyl)-3-fluorophenyl)-3-chloropyridin-4-yl)-2-chlorophenyl)-2-methoxypyridin-3-yl)methyl)amino)piperidin-1-yl)ethan-1-one;
1-(6-(2-chloro-3-(3-chloro-2-(3-fluoro-4-((methylamino)methyl)phenyl)pyridin-4-yl)phenyl)-2-methoxypyridin-3-yl)-N-methylmethanamine;
2-((6-(2-chloro-3-(3-chloro-2-(3-fluoro-4-((7-oxo-2,6-diazaspiro[3.4]octan-2-yl)methyl)phenyl)pyridin-4-yl)phenyl)-2-methoxypyridin-3-yl)methyl)-2,6-diazaspiro[3.4]octan-7-one;
1-(4-(((6-(3-(2-(4-(((1-acetylpiperidin-4-yl)amino)methyl)-3-fluoro-5-methoxyphenyl)-3-chloropyridin-4-yl)-2-chlorophenyl)-2-methoxypyridin-3-yl)methyl)amino)piperidin-1-yl)ethan-1-one;
1-(6-(2-chloro-3-(3-chloro-2-(3-fluoro-5-methoxy-4-((methylamino)methyl)phenyl)pyridin-4-yl)phenyl)-2-methoxypyridin-3-yl)-N-methylmethanamine;
2-(4-(3-chloro-4-(2-chloro-3-(6-methoxy-5-((7-oxo-2,6-diazaspiro[3.4]octan-2-yl)methyl)pyridin-2-yl)phenyl)pyridin-2-yl)-2-fluoro-6-methoxybenzyl)-2,6-diazaspiro[3.4]octan-7-one;
(S)-2-((6-(3-chloro-4-(2-chloro-3-(6-methoxy-5-((((5-oxopyrrolidin-2-yl)methyl)amino)methyl)pyridin-2-yl)phenyl)pyridin-2-yl)-1-methyl-1H-indazol-3-yl)methyl)-2,6-diazaspiro[3.4]octan-7-one;
(R)-2-((6-(3-chloro-4-(2-chloro-3-(6-methoxy-5-((((5-oxopyrrolidin-2-yl)methyl)amino)methyl)pyridin-2-yl)phenyl)pyridin-2-yl)-1-methyl-1H-indazol-3-yl)methyl)-2,6-diazaspiro[3.4]octan-7-one;
(S)-5-((((6-(3-(2-(2-((1-acetylpiperidin-4-yl)methyl)-8-chloro-1,2,3,4-tetrahydroisoquinolin-6-yl)-3-chloropyridin-4-yl)-2-chlorophenyl)-2-methoxypyridin-3-yl)methyl)amino)methyl)pyrrolidin-2-one;
(R)-5-((((6-(3-(2-(2-((1-acetylpiperidin-4-yl)methyl)-8-chloro-1,2,3,4-tetrahydroisoquinolin-6-yl)-3-chloropyridin-4-yl)-2-chlorophenyl)-2-methoxypyridin-3-yl)methyl)amino)methyl)pyrrolidin-2-one;
6-(2-chloro-3-(3-chloro-2-(3-methoxy-4-(((((S)-5-oxopyrrolidin-2-yl)methyl)amino)methyl)phenyl)pyridin-4-yl)phenyl)-2-(2-((((S)-5-oxopyrrolidin-2-yl)methyl)amino)ethyl)-1,2-dihydro-3H-pyrrolo[3,4-c]pyridin-3-one;
6-(2-chloro-3-(3-chloro-2-(3-methoxy-4-(((((S)-5-oxopyrrolidin-2-yl)methyl)amino)methyl)phenyl)pyridin-4-yl)phenyl)-2-(2-((((R)-5-oxopyrrolidin-2-yl)methyl)amino)ethyl)-1,2-dihydro-3H-pyrrolo[3,4-c]pyridin-3-one;
6-(2-chloro-3-(3-chloro-2-(3-methoxy-4-(((((R)-5-oxopyrrolidin-2-yl)methyl)amino)methyl)phenyl)pyridin-4-yl)phenyl)-2-(2-((((S)-5-oxopyrrolidin-2-yl)methyl)amino)ethyl)-1,2-dihydro-3H-pyrrolo[3,4-c]pyridin-3-one;
6-(2-chloro-3-(3-chloro-2-(3-methoxy-4-(((((R)-5-oxopyrrolidin-2-yl)methyl)amino)methyl)phenyl)pyridin-4-yl)phenyl)-2-(2-((((R)-5-oxopyrrolidin-2-yl)methyl)amino)ethyl)-1,2-dihydro-3H-pyrrolo[3,4-c]pyridin-3-one;
(S)-5-((((6-(2-chloro-3-(3-chloro-2-(3-methoxy-4-(((((S)-5-oxopyrrolidin-2-yl)methyl)amino)methyl)phenyl)pyridin-4-yl)phenyl)imidazo[1,2-a]pyrazin-2-yl)methyl)amino)methyl)pyrrolidin-2-one;
(S)-5-((((6-(2-chloro-3-(3-chloro-2-(3-methoxy-4-(((((R)-5-oxopyrrolidin-2-yl)methyl)amino)methyl)phenyl)pyridin-4-yl)phenyl)imidazo[1,2-a]pyrazin-2-yl)methyl)amino)methyl)pyrrolidin-2-one;
(R)-5-((((6-(2-chloro-3-(3-chloro-2-(3-methoxy-4-(((((S)-5-oxopyrrolidin-2-yl)methyl)amino)methyl)phenyl)pyridin-4-yl)phenyl)imidazo[1,2-a]pyrazin-2-yl)methyl)amino)methyl)pyrrolidin-2-one;
(R)-5-((((6-(2-chloro-3-(3-chloro-2-(3-methoxy-4-(((((R)-5-oxopyrrolidin-2-yl)methyl)amino)methyl)phenyl)pyridin-4-yl)phenyl)imidazo[1,2-a]pyrazin-2-yl)methyl)amino)methyl)pyrrolidin-2-one;
(S)-2-(4-(3-chloro-4-(1-((6-methoxy-5-((7-oxo-2,6-diazaspiro[3.4]octan-2-yl)methyl)-3-(trifluoromethyl)pyridin-2-yl)oxy)-2,3-dihydro-1H-inden-4-yl)pyridin-2-yl)-2-methoxybenzyl)-2,6-diazaspiro[3.4]octan-7-one;
(R)-2-(4-(3-chloro-4-(1-((6-methoxy-5-((7-oxo-2,6-diazaspiro[3.4]octan-2-yl)methyl)-3-(trifluoromethyl)pyridin-2-yl)oxy)-2,3-dihydro-1H-inden-4-yl)pyridin-2-yl)-2-methoxybenzyl)-2,6-diazaspiro[3.4]octan-7-one;
(S)-3-(6-(3-chloro-4-(2-chloro-3-(5-(((2-hydroxypropyl)amino)methyl)-6-methoxypyridin-2-yl)phenyl)pyridin-2-yl)-8-methoxy-3,4-dihydroisoquinolin-2(1H)-yl)propanoic acid;
(R)-3-(6-(3-chloro-4-(2-chloro-3-(5-(((2-hydroxypropyl)amino)methyl)-6-methoxypyridin-2-yl)phenyl)pyridin-2-yl)-8-methoxy-3,4-dihydroisoquinolin-2(1H)-yl)propanoic acid;
3-(6-(3-chloro-4-(2-chloro-3-(6-methoxy-5-((7-oxo-2,6-diazaspiro[3.4]octan-2-yl)methyl)pyridin-2-yl)phenyl)pyridin-2-yl)-8-methoxy-3,4-dihydroisoquinolin-2(1H)-yl)propanoic acid;
methyl 1-((6-(3-chloro-4-(2-chloro-3-(6-methoxy-5-((7-oxo-2,6-diazaspiro[3.4]octan-2-yl)methyl)pyridin-2-yl)phenyl)pyridin-2-yl)-8-methoxy-3,4-dihydroisoquinolin-2(1H)-yl)methyl)cyclopropane-1-carboxylate;
2-((6-(2-chloro-3-(3-chloro-2-(2-(3-fluoro-2-hydroxypropyl)-8-methoxy-1,2,3,4-tetrahydroisoquinolin-6-yl)pyridin-4-yl)phenyl)-2-methoxypyridin-3-yl)methyl)-2,6-diazaspiro[3.4]octan-7-one;
(S)-2-((6-(2-chloro-3-(3-chloro-2-(2-(3-fluoro-2-hydroxypropyl)-8-methoxy-1,2,3,4-tetrahydroisoquinolin-6-yl)pyridin-4-yl)phenyl)-2-methoxypyridin-3-yl)methyl)-2,6-diazaspiro[3.4]octan-7-one;
(R)-2-((6-(2-chloro-3-(3-chloro-2-(2-(3-fluoro-2-hydroxypropyl)-8-methoxy-1,2,3,4-tetrahydroisoquinolin-6-yl)pyridin-4-yl)phenyl)-2-methoxypyridin-3-yl)methyl)-2,6-diazaspiro[3.4]octan-7-one;
1-((6-(3-chloro-4-(2-chloro-3-(6-methoxy-5-((7-oxo-2,6-diazaspiro[3.4]octan-2-yl)methyl)pyridin-2-yl)phenyl)pyridin-2-yl)-8-methoxy-3,4-dihydroisoquinolin-2(1H)-yl)methyl)cyclopropane-1-carboxylic acid;
N-((6-(2-chloro-3-(3-chloro-2-(3-methoxy-4-(((tetrahydro-2H-pyran-4-yl)amino)methyl)phenyl)pyridin-4-yl)phenyl)-2-methoxypyridin-3-yl)methyl)tetrahydro-2H-pyran-4-amine;
4-(((6-(2-chloro-3-(3-chloro-2-(4-(((1,1-dioxidotetrahydro-2H-thiopyran-4-yl)amino)methyl)-3-methoxyphenyl)pyridin-4-yl)phenyl)-2-methoxypyridin-3-yl)methyl)amino)tetrahydro-2H-thiopyran 1,1-dioxide;
1-(3-(((6-(3-(2-(4-(((1-acetylazetidin-3-yl)amino)methyl)-3-methoxyphenyl)-3-chloropyridin-4-yl)-2-chlorophenyl)-2-methoxypyridin-3-yl)methyl)amino)azetidin-1-yl)ethan-1-one;
(S)-1-(((6-(2-chloro-3-(3-chloro-2-(4-((((S)-2-hydroxypropyl)amino)methyl)-3-methoxyphenyl)pyridin-4-yl)phenyl)-2-methoxypyridin-3-yl)methyl)amino)propan-2-ol;
(R)-1-(((6-(2-chloro-3-(3-chloro-2-(4-((((S)-2-hydroxypropyl)amino)methyl)-3-methoxyphenyl)pyridin-4-yl)phenyl)-2-methoxypyridin-3-yl)methyl)amino)propan-2-ol;
(S)-1-(((6-(2-chloro-3-(3-chloro-2-(4-((((R)-2-hydroxypropyl)amino)methyl)-3-methoxyphenyl)pyridin-4-yl)phenyl)-2-methoxypyridin-3-yl)methyl)amino)propan-2-ol;
(R)-1-(((6-(2-chloro-3-(3-chloro-2-(4-((((R)-2-hydroxypropyl)amino)methyl)-3-methoxyphenyl)pyridin-4-yl)phenyl)-2-methoxypyridin-3-yl)methyl)amino)propan-2-ol;
2-((4-(3-chloro-4-(2-chloro-3-(6-methoxy-5-(((5-oxo-6-azaspiro[3.4]octan-2-yl)amino)methyl)pyridin-2-yl)phenyl)pyridin-2-yl)-2-methoxybenzyl)amino)-6-azaspiro[3.4]octan-5-one;
1-(2-(((6-(2-chloro-3-(3-chloro-2-(3-methoxy-4-(((2-(2-oxopyrrolidin-1-yl)ethyl)amino)methyl)phenyl)pyridin-4-yl)phenyl)-2-methoxypyridin-3-yl)methyl)amino)ethyl)pyrrolidin-2-one;
(S)-2-((3'-chloro-6-methoxy-2'-(1-((6-methoxy-5-((7-oxo-2,6-diazaspiro[3.4]octan-2-yl)methyl)-3-(trifluoromethyl)pyrazin-2-yl)amino)-2,3-dihydro-1H-inden-4-yl)-[2,4'-bipyridin]-5-yl)methyl)-2,6-diazaspiro[3.4]octan-7-one;
(R)-2-((3'-chloro-6-methoxy-2'-(1-((6-methoxy-5-((7-oxo-2,6-diazaspiro[3.4]octan-2-yl)methyl)-3-(trifluoromethyl)pyrazin-2-yl)amino)-2,3-dihydro-1H-inden-4-yl)-[2,4'-bipyridin]-5-yl)methyl)-2,6-diazaspiro[3.4]octan-7-one;
(S)-1-(6-((5-((4-(5-((6-acetyl-2,6-diazaspiro[3.3]heptan-2-yl)methyl)-3'-chloro-6-methoxy-[2,4'-bipyridin]-2'-yl)-2,3-dihydro-1H-inden-1-yl)amino)-3-methoxy-6-(trifluoromethyl)pyrazin-2-yl)methyl)-2,6-diazaspiro[3.3]heptan-2-yl)ethan-1-one;
(R)-1-(6-((5-((4-(5-((6-acetyl-2,6-diazaspiro[3.3]heptan-2-yl)methyl)-3'-chloro-6-methoxy-[2,4'-bipyridin]-2'-yl)-2,3-dihydro-1H-inden-1-yl)amino)-3-methoxy-6-(trifluoromethyl)pyrazin-2-yl)methyl)-2,6-diazaspiro[3.3]heptan-2-yl)ethan-1-one;
(S)-2-(4-(3-chloro-4-(1-((6-methoxy-5-((7-oxo-2,6-diazaspiro[3.4]octan-2-yl)methyl)-3-(trifluoromethyl)pyridin-2-yl)oxy)-2,3-dihydro-1H-inden-4-yl)pyridin-2-yl)-2-fluoro-6-methoxybenzyl)-2,6-diazaspiro[3.4]octan-7-one;
(R)-2-(4-(3-chloro-4-(1-((6-methoxy-5-((7-oxo-2,6-diazaspiro[3.4]octan-2-yl)methyl)-3-(trifluoromethyl)pyridin-2-yl)oxy)-2,3-dihydro-1H-inden-4-yl)pyridin-2-yl)-2-fluoro-6-methoxybenzyl)-2,6-diazaspiro[3.4]octan-7-one;
(S)-5-((((6-(2-chloro-3-(3-chloro-2-(8-chloro-2-(oxetan-3-ylmethyl)-1,2,3,4-tetrahydroisoquinolin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(8-chloro-2-(oxetan-3-ylmethyl)-1,2,3,4-tetrahydroisoquinolin-6-yl)pyridin-4-yl)phenyl)-2-methoxypyridin-3-yl)methyl)amino)methyl)pyrrolidin-2-one;
(S)-5-((((6-(3-(2-(3-((6-acetyl-2,6-diazaspiro[3.3]heptan-2-yl)methyl)-1-methyl-1H-indazol-6-yl)-3-chloropyridin-4-yl)-2-chlorophenyl)-2-methoxypyridin-3-yl)methyl)amino)methyl)pyrrolidin-2-one;
(R)-5-((((6-(3-(2-(3-((6-acetyl-2,6-diazaspiro[3.3]heptan-2-yl)methyl)-1-methyl-1H-indazol-6-yl)-3-chloropyridin-4-yl)-2-chlorophenyl)-2-methoxypyridin-3-yl)methyl)amino)methyl)pyrrolidin-2-one;
(S)-1-((6-(3-chloro-4-(2-chloro-3-(6-methoxy-5-(((((S)-5-oxopyrrolidin-2-yl)methyl)amino)methyl)pyridin-2-yl)phenyl)pyridin-2-yl)-1-methyl-1H-indazol-3-yl)methyl)pyrrolidine-3-carboxylic acid;
(S)-1-((6-(3-chloro-4-(2-chloro-3-(6-methoxy-5-(((((R)-5-oxopyrrolidin-2-yl)methyl)amino)methyl)pyridin-2-yl)phenyl)pyridin-2-yl)-1-methyl-1H-indazol-3-yl)methyl)pyrrolidine-3-carboxylic acid;
(R)-1-((6-(3-chloro-4-(2-chloro-3-(6-methoxy-5-(((((S)-5-oxopyrrolidin-2-yl)methyl)amino)methyl)pyridin-2-yl)phenyl)pyridin-2-yl)-1-methyl-1H-indazol-3-yl)methyl)pyrrolidine-3-carboxylic acid;
(R)-1-((6-(3-chloro-4-(2-chloro-3-(6-methoxy-5-(((((R)-5-oxopyrrolidin-2-yl)methyl)amino)methyl)pyridin-2-yl)phenyl)pyridin-2-yl)-1-methyl-1H-indazol-3-yl)methyl)pyrrolidine-3-carboxylic acid;
(S)-5-((((6-(2-chloro-3-(3-chloro-2-(8-methoxy-2-(((((S)-5-oxopyrrolidin-2-yl)methyl)amino)methyl)imidazo[1,2-a]pyridin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(8-methoxy-2-(((((R)-5-oxopyrrolidin-2-yl)methyl)amino)methyl)imidazo[1,2-a]pyridin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(8-methoxy-2-(((((S)-5-oxopyrrolidin-2-yl)methyl)amino)methyl)imidazo[1,2-a]pyridin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(8-methoxy-2-(((((R)-5-oxopyrrolidin-2-yl)methyl)amino)methyl)imidazo[1,2-a]pyridin-6-yl)pyridin-4-yl)phenyl)-2-methoxypyridin-3-yl)methyl)amino)methyl)pyrrolidin-2-one;
1-(4-(((5-(3-(2-(4-(((1-acetylpiperidin-4-yl)amino)methyl)-3-methoxyphenyl)-3-chloropyridin-4-yl)-2-chlorophenyl)-3-methoxypyrazin-2-yl)methyl)amino)piperidin-1-yl)ethan-1-one;
(S)-5-((((6-(2-chloro-3-(3-chloro-2-(2-((((1r,3r)-3-hydroxy-3-methylcyclobutyl)amino)methyl)-8-methoxyimidazo[1,2-a]pyridin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(2-((((1s,3s)-3-hydroxy-3-methylcyclobutyl)amino)methyl)-8-methoxyimidazo[1,2-a]pyridin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(2-((((1s,3r)-3-hydroxy-3-methylcyclobutyl)amino)methyl)-8-methoxyimidazo[1,2-a]pyridin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(2-((((1r,3s)-3-hydroxy-3-methylcyclobutyl)amino)methyl)-8-methoxyimidazo[1,2-a]pyridin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-((((1r,3s)-3-hydroxy-3-methylcyclobutyl)amino)methyl)-8-methoxyimidazo[1,2-a]pyridin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-((((1s,3s)-3-hydroxy-3-methylcyclobutyl)amino)methyl)-8-methoxyimidazo[1,2-a]pyridin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-((((1s,3r)-3-hydroxy-3-methylcyclobutyl)amino)methyl)-8-methoxyimidazo[1,2-a]pyridin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-((((1r,3r)-3-hydroxy-3-methylcyclobutyl)amino)methyl)-8-methoxyimidazo[1,2-a]pyridin-6-yl)pyridin-4-yl)phenyl)-2-methoxypyridin-3-yl)methyl)amino)methyl)pyrrolidin-2-one;
1,1'-(((((3,3'-dichloro-[4,4'-bipyridine]-2,2'-diyl)bis(2-fluoro-6-methoxy-4,1-phenylene))bis(methylene))bis(azanediyl))bis(piperidine-4,1-diyl))bis(ethan-1-one);
(S)-2-((6-(3-chloro-4-(2-chloro-3-(6-methoxy-5-((((5-oxopyrrolidin-2-yl)methyl)amino)methyl)pyridin-2-yl)phenyl)pyridin-2-yl)-8-methoxyimidazo[1,2-a]pyridin-2-yl)methyl)-2,6-diazaspiro[3.4]octan-7-one;
(R)-2-((6-(3-chloro-4-(2-chloro-3-(6-methoxy-5-((((5-oxopyrrolidin-2-yl)methyl)amino)methyl)pyridin-2-yl)phenyl)pyridin-2-yl)-8-methoxyimidazo[1,2-a]pyridin-2-yl)methyl)-2,6-diazaspiro[3.4]octan-7-one;
1-(4-(((6-(3-(2-(4-(((1-acetylpiperidin-4-yl)(methyl)amino)methyl)-3-methoxyphenyl)-3-chloropyridin-4-yl)-2-chlorophenyl)-2-methoxypyridin-3-yl)methyl)amino)piperidin-1-yl)ethan-1-one;
(S)-5-((((6-(2-chloro-3-(3-chloro-2-(2-((3-hydroxy-3-methylazetidin-1-yl)methyl)-8-methoxyimidazo[1,2-a]pyridin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-((3-hydroxy-3-methylazetidin-1-yl)methyl)-8-methoxyimidazo[1,2-a]pyridin-6-yl)pyridin-4-yl)phenyl)-2-methoxypyridin-3-yl)methyl)amino)methyl)pyrrolidin-2-one;
(S)-5-((((6-(3-(2-(2-(((1-acetylpiperidin-4-yl)amino)methyl)-8-methoxyimidazo[1,2-a]pyridin-6-yl)-3-chloropyridin-4-yl)-2-chlorophenyl)-2-methoxypyridin-3-yl)methyl)amino)methyl)pyrrolidin-2-one;
(R)-5-((((6-(3-(2-(2-(((1-acetylpiperidin-4-yl)amino)methyl)-8-methoxyimidazo[1,2-a]pyridin-6-yl)-3-chloropyridin-4-yl)-2-chlorophenyl)-2-methoxypyridin-3-yl)methyl)amino)methyl)pyrrolidin-2-one;
(S)-5-((((6-(3-(2-(2-((6-acetyl-2,6-diazaspiro[3.3]heptan-2-yl)methyl)-8-methoxyimidazo[1,2-a]pyridin-6-yl)-3-chloropyridin-4-yl)-2-chlorophenyl)-2-methoxypyridin-3-yl)methyl)amino)methyl)pyrrolidin-2-one;
(R)-5-((((6-(3-(2-(2-((6-acetyl-2,6-diazaspiro[3.3]heptan-2-yl)methyl)-8-methoxyimidazo[1,2-a]pyridin-6-yl)-3-chloropyridin-4-yl)-2-chlorophenyl)-2-methoxypyridin-3-yl)methyl)amino)methyl)pyrrolidin-2-one;
(S)-2-((6-(3-chloro-4-(2-chloro-3-(6-methoxy-5-((((5-oxopyrrolidin-2-yl)methyl)amino)methyl)pyridin-2-yl)phenyl)pyridin-2-yl)-8-methoxyimidazo[1,2-a]pyridin-2-yl)methyl)-2,5-diazaspiro[3.4]octan-6-one;
(R)-2-((6-(3-chloro-4-(2-chloro-3-(6-methoxy-5-((((5-oxopyrrolidin-2-yl)methyl)amino)methyl)pyridin-2-yl)phenyl)pyridin-2-yl)-8-methoxyimidazo[1,2-a]pyridin-2-yl)methyl)-2,5-diazaspiro[3.4]octan-6-one;
(S)-5-((((6-(3-(2-(2-(((2-oxaspiro[3.3]heptan-6-yl)amino)methyl)-8-methoxyimidazo[1,2-a]pyridin-6-yl)-3-chloropyridin-4-yl)-2-chlorophenyl)-2-methoxypyridin-3-yl)methyl)amino)methyl)pyrrolidin-2-one;
(R)-5-((((6-(3-(2-(2-(((2-oxaspiro[3.3]heptan-6-yl)amino)methyl)-8-methoxyimidazo[1,2-a]pyridin-6-yl)-3-chloropyridin-4-yl)-2-chlorophenyl)-2-methoxypyridin-3-yl)methyl)amino)methyl)pyrrolidin-2-one;
N-(1-((6-(3-(2-(4-((4-acetamidopiperidin-1-yl)methyl)-3-methoxyphenyl)-3-chloropyridin-4-yl)-2-chlorophenyl)-2-methoxypyridin-3-yl)methyl)piperidin-4-yl)acetamide;
2,2'-(((3,3'-dichloro-[4,4'-bipyridine]-2,2'-diyl)bis(2-fluoro-6-methoxy-4,1-phenylene))bis(methylene))bis(2,6-diazaspiro[3.4]octan-7-one);
2-(4-(4-(2-chloro-3-(6-methoxy-5-((7-oxo-2,6-diazaspiro[3.4]octan-2-yl)methyl)pyridin-2-yl)phenyl)-3-fluoropyridin-2-yl)-2-methoxybenzyl)-2,6-diazaspiro[3.4]octan-7-one;
N-(1-(4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)piperidin-4-yl)acetamide;
N-((6-(2-chloro-3-(3-chloro-2-(4-(((1-isopropylpiperidin-4-yl)amino)methyl)-3-methoxyphenyl)pyridin-4-yl)phenyl)-2-methoxypyridin-3-yl)methyl)-1-isopropylpiperidin-4-amine;
N-((6-(2-chloro-3-(3-chloro-2-(4-(((4,4-difluorocyclohexyl)amino)methyl)-3-methoxyphenyl)pyridin-4-yl)phenyl)-2-methoxypyridin-3-yl)methyl)-4,4-difluorocyclohexan-1-amine;
2-(4-(((6-(2-chloro-3-(3-chloro-2-(4-(((1-(2-hydroxyethyl)piperidin-4-yl)amino)methyl)-3-methoxyphenyl)pyridin-4-yl)phenyl)-2-methoxypyridin-3-yl)methyl)amino)piperidin-1-yl)ethan-1-ol;
N-(2-(((6-(3-(2-(4-(((2-acetamidoethyl)amino)methyl)-3-methoxyphenyl)-3-chloropyridin-4-yl)-2-chlorophenyl)-2-methoxypyridin-3-yl)methyl)amino)ethyl)acetamide;
1-(6-(2-chloro-3-(3-chloro-2-(3-methoxy-4-((((tetrahydro-2H-pyran-4-yl)methyl)amino)methyl)phenyl)pyridin-4-yl)phenyl)-2-methoxypyridin-3-yl)-N-((tetrahydro-2H-pyran-4-yl)methyl)methanamine;
1-(4-(((6-(2-chloro-3-(3-chloro-2-(3-fluoro-5-methoxy-4-(((1-propionylpiperidin-4-yl)amino)methyl)phenyl)pyridin-4-yl)phenyl)-2-methoxypyridin-3-yl)methyl)amino)piperidin-1-yl)propan-1-one;
2-((6-(3-(2-(4-(((1-acetylpiperidin-4-yl)amino)methyl)-3-methoxyphenyl)-3-chloropyridin-4-yl)-2-chlorophenyl)-2-methoxypyridin-3-yl)methyl)-2,6-diazaspiro[3.4]octan-7-one;
1-(4-((4-(4-(3-(5-(((1-acetylpiperidin-4-yl)(methyl)amino)methyl)-6-methoxypyridin-2-yl)-2-chlorophenyl)-3-chloropyridin-2-yl)-2-methoxybenzyl)amino)piperidin-1-yl)ethan-1-one;
1-(6-(2-chloro-3-(2-(3-methoxy-4-((methylamino)methyl)phenyl)-3-methylpyridin-4-yl)phenyl)-2-methoxypyridin-3-yl)-N-methylmethanamine;
(S)-5-((((6-(3-(2-(4-((S)-1-aminoethyl)-3-methoxyphenyl)-3-chloropyridin-4-yl)-2-chlorophenyl)-2-methoxypyridin-3-yl)methyl)amino)methyl)pyrrolidin-2-one;
(R)-5-((((6-(3-(2-(4-((S)-1-aminoethyl)-3-methoxyphenyl)-3-chloropyridin-4-yl)-2-chlorophenyl)-2-methoxypyridin-3-yl)methyl)amino)methyl)pyrrolidin-2-one;
(S)-5-((((6-(3-(2-(4-((R)-1-aminoethyl)-3-methoxyphenyl)-3-chloropyridin-4-yl)-2-chlorophenyl)-2-methoxypyridin-3-yl)methyl)amino)methyl)pyrrolidin-2-one;
(R)-5-((((6-(3-(2-(4-((R)-1-aminoethyl)-3-methoxyphenyl)-3-chloropyridin-4-yl)-2-chlorophenyl)-2-methoxypyridin-3-yl)methyl)amino)methyl)pyrrolidin-2-one;
(S)-2-((3'-chloro-6-methoxy-2'-(1-((6-methoxy-5-((7-oxo-2,6-diazaspiro[3.4]octan-2-yl)methyl)-3-(trifluoromethyl)pyridin-2-yl)oxy)-2,3-dihydro-1H-inden-4-yl)-[2,4'-bipyridin]-5-yl)methyl)-2,6-diazaspiro[3.4]octan-7-one;
(R)-2-((3'-chloro-6-methoxy-2'-(1-((6-methoxy-5-((7-oxo-2,6-diazaspiro[3.4]octan-2-yl)methyl)-3-(trifluoromethyl)pyridin-2-yl)oxy)-2,3-dihydro-1H-inden-4-yl)-[2,4'-bipyridin]-5-yl)methyl)-2,6-diazaspiro[3.4]octan-7-one;
1-(4-(((6-(3-(2-(4-(((1-acetylpiperidin-4-yl)amino)methyl)-3-methoxyphenyl)-3-fluoropyridin-4-yl)-2-chlorophenyl)-2-methoxypyridin-3-yl)methyl)amino)piperidin-1-yl)ethan-1-one;
(S)-4-((4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)amino)dihydrofuran-2(3H)-one;
(R)-4-((4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)amino)dihydrofuran-2(3H)-one;
methyl (S)-3-((4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)amino)-4-hydroxybutanoate;
methyl (R)-3-((4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)amino)-4-hydroxybutanoate;
(S)-3-((4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)amino)dihydrofuran-2(3H)-one;
(R)-3-((4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)amino)dihydrofuran-2(3H)-one;
N-(1-((6-(3-(2-(4-(((1-acetylpiperidin-4-yl)amino)methyl)-3-methoxyphenyl)-3-chloropyridin-4-yl)-2-chlorophenyl)-2-methoxypyridin-3-yl)methyl)piperidin-4-yl)acetamide;
(S)-3-((4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)amino)-4-hydroxybutanoic acid;
(R)-3-((4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)amino)-4-hydroxybutanoic acid;
(4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)-L-homoserine;
(4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)-D-homoserine;
1-(4-(((6-(2-chloro-3-(3-chloro-2-(3-methoxy-4-(((1-propionylpiperidin-4-yl)amino)methyl)phenyl)pyridin-4-yl)phenyl)-2-methoxypyridin-3-yl)methyl)amino)piperidin-1-yl)propan-1-one;
1-(4-(((6-(2-chloro-3-(3-chloro-2-(4-(((1-isobutyrylpiperidin-4-yl)amino)methyl)-3-methoxyphenyl)pyridin-4-yl)phenyl)-2-methoxypyridin-3-yl)methyl)amino)piperidin-1-yl)-2-methylpropan-1-one;
1-(4-(((6-(2-chloro-3-(3-chloro-2-(3-fluoro-4-(((1-isobutyrylpiperidin-4-yl)amino)methyl)-5-methoxyphenyl)pyridin-4-yl)phenyl)-2-methoxypyridin-3-yl)methyl)amino)piperidin-1-yl)-2-methylpropan-1-one;
N-((6-(2-chloro-3-(3-chloro-2-(3-fluoro-5-methoxy-4-(((tetrahydro-2H-pyran-4-yl)amino)methyl)phenyl)pyridin-4-yl)phenyl)-2-methoxypyridin-3-yl)methyl)tetrahydro-2H-pyran-4-amine;
4-(((6-(2-chloro-3-(3-chloro-2-(4-(((1,1-dioxidotetrahydro-2H-thiopyran-4-yl)amino)methyl)-3-fluoro-5-methoxyphenyl)pyridin-4-yl)phenyl)-2-methoxypyridin-3-yl)methyl)amino)tetrahydro-2H-thiopyran 1,1-dioxide;
(S)-1-((6-(3-chloro-4-(2-chloro-3-(6-methoxy-5-((((5-oxopyrrolidin-2-yl)methyl)amino)methyl)pyridin-2-yl)phenyl)pyridin-2-yl)-8-methoxyimidazo[1,2-a]pyridin-2-yl)methyl)azetidine-3-carboxylic acid;
(R)-1-((6-(3-chloro-4-(2-chloro-3-(6-methoxy-5-((((5-oxopyrrolidin-2-yl)methyl)amino)methyl)pyridin-2-yl)phenyl)pyridin-2-yl)-8-methoxyimidazo[1,2-a]pyridin-2-yl)methyl)azetidine-3-carboxylic acid;
(S)-1-(2-(((6-(3-chloro-4-(2-chloro-3-(6-methoxy-5-((((5-oxopyrrolidin-2-yl)methyl)amino)methyl)pyridin-2-yl)phenyl)pyridin-2-yl)-8-methoxyimidazo[1,2-a]pyridin-2-yl)methyl)amino)ethyl)cyclopropane-1-carboxylic acid;
(R)-1-(2-(((6-(3-chloro-4-(2-chloro-3-(6-methoxy-5-((((5-oxopyrrolidin-2-yl)methyl)amino)methyl)pyridin-2-yl)phenyl)pyridin-2-yl)-8-methoxyimidazo[1,2-a]pyridin-2-yl)methyl)amino)ethyl)cyclopropane-1-carboxylic acid;
7-(4-(3-chloro-4-(2-chloro-3-(6-methoxy-5-((2-oxo-1,7-diazaspiro[3.5]nonan-7-yl)methyl)pyridin-2-yl)phenyl)pyridin-2-yl)-2-methoxybenzyl)-1,7-diazaspiro[3.5]nonan-2-one;
(R)-5-((((6-(2-chloro-3-(3-chloro-2-(3-methoxy-4-(((((R)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
(R)-5-((((6-(2-chloro-3-(3-chloro-2-(3-methoxy-4-(((((S)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
(S)-5-((((6-(2-chloro-3-(3-chloro-2-(3-methoxy-4-(((((R)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
(S)-5-((((6-(2-chloro-3-(3-chloro-2-(3-methoxy-4-(((((S)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
N-(1-((6-(2-chloro-3-(3-chloro-2-(3-methoxy-4-((7-oxo-2,6-diazaspiro[3.4]octan-2-yl)methyl)phenyl)pyridin-4-yl)phenyl)-2-methoxypyridin-3-yl)methyl)piperidin-4-yl)acetamide;
(R)-N-(1-((6-(2-chloro-3-(3-chloro-2-(3-methoxy-4-((((5-oxopyrrolidin-2-yl)methyl)amino)methyl)phenyl)pyridin-4-yl)phenyl)-2-methoxypyridin-3-yl)methyl)piperidin-4-yl)acetamide;
(S)-N-(1-((6-(2-chloro-3-(3-chloro-2-(3-methoxy-4-((((5-oxopyrrolidin-2-yl)methyl)amino)methyl)phenyl)pyridin-4-yl)phenyl)-2-methoxypyridin-3-yl)methyl)piperidin-4-yl)acetamide;
(S)-N-(1-((6-(2-chloro-3-(3-chloro-2-(3-methoxy-4-((((5-oxopyrrolidin-2-yl)methyl)amino)methyl)phenyl)pyridin-4-yl)phenyl)-2-methoxypyridin-3-yl)methyl)piperidin-4-yl)acetamide;
(R)-N-(1-((6-(2-chloro-3-(3-chloro-2-(3-methoxy-4-((((5-oxopyrrolidin-2-yl)methyl)amino)methyl)phenyl)pyridin-4-yl)phenyl)-2-methoxypyridin-3-yl)methyl)piperidin-4-yl)acetamide;
N-(3-(((6-(3-(2-(4-(((3-acetamidopropyl)amino)methyl)-3-methoxyphenyl)-3-chloropyridin-4-yl)-2-chlorophenyl)-2-methoxypyridin-3-yl)methyl)amino)propyl)acetamide;
4-(((6-(2-chloro-3-(3-chloro-2-(4-(((2,6-dioxopiperidin-4-yl)amino)methyl)-3-methoxyphenyl)pyridin-4-yl)phenyl)-2-methoxypyridin-3-yl)methyl)amino)piperidine-2,6-dione;
1-(4-(((6-(2-chloro-3-(3-chloro-2-(4-((((1r,4r)-4-hydroxycyclohexyl)amino)methyl)-3-methoxyphenyl)pyridin-4-yl)phenyl)-2-methoxypyridin-3-yl)methyl)amino)piperidin-1-yl)ethan-1-one;
1-(4-(((6-(2-chloro-3-(3-chloro-2-(4-((((1r,4s)-4-hydroxycyclohexyl)amino)methyl)-3-methoxyphenyl)pyridin-4-yl)phenyl)-2-methoxypyridin-3-yl)methyl)amino)piperidin-1-yl)ethan-1-one;
1-(4-(((6-(2-chloro-3-(3-chloro-2-(4-((((1s,4r)-4-hydroxycyclohexyl)amino)methyl)-3-methoxyphenyl)pyridin-4-yl)phenyl)-2-methoxypyridin-3-yl)methyl)amino)piperidin-1-yl)ethan-1-one;
1-(4-(((6-(2-chloro-3-(3-chloro-2-(4-((((1s,4s)-4-hydroxycyclohexyl)amino)methyl)-3-methoxyphenyl)pyridin-4-yl)phenyl)-2-methoxypyridin-3-yl)methyl)amino)piperidin-1-yl)ethan-1-one;
1-(4-(((6-(2-chloro-3-(3-chloro-2-(4-((6-hydroxy-2-azaspiro[3.3]heptan-2-yl)methyl)-3-methoxyphenyl)pyridin-4-yl)phenyl)-2-methoxypyridin-3-yl)methyl)amino)piperidin-1-yl)ethan-1-one;
1-(2-methoxy-4-(4-(3-(6-methoxy-5-((methylamino)methyl)pyridin-2-yl)-2-methylphenyl)-3-methylpyridin-2-yl)phenyl)-N-methylmethanamine;
1-(4-(((6-(3-(2-(4-(((1-acetylpiperidin-4-yl)amino)methyl)-3-methoxyphenyl)-3-methylpyridin-4-yl)-2-methylphenyl)-2-methoxypyridin-3-yl)methyl)amino)piperidin-1-yl)ethan-1-one;
(1r,4r)-4-(((6-(2-chloro-3-(3-chloro-2-(4-((((1r,4r)-4-hydroxycyclohexyl)amino)methyl)-3-methoxyphenyl)pyridin-4-yl)phenyl)-2-methoxypyridin-3-yl)methyl)amino)cyclohexan-1-ol;
(1r,4r)-4-(((6-(2-chloro-3-(3-chloro-2-(4-((((1r,4s)-4-hydroxycyclohexyl)amino)methyl)-3-methoxyphenyl)pyridin-4-yl)phenyl)-2-methoxypyridin-3-yl)methyl)amino)cyclohexan-1-ol;
(1r,4r)-4-(((6-(2-chloro-3-(3-chloro-2-(4-((((1s,4r)-4-hydroxycyclohexyl)amino)methyl)-3-methoxyphenyl)pyridin-4-yl)phenyl)-2-methoxypyridin-3-yl)methyl)amino)cyclohexan-1-ol;
(1r,4r)-4-(((6-(2-chloro-3-(3-chloro-2-(4-((((1s,4s)-4-hydroxycyclohexyl)amino)methyl)-3-methoxyphenyl)pyridin-4-yl)phenyl)-2-methoxypyridin-3-yl)methyl)amino)cyclohexan-1-ol;
(1r,4s)-4-(((6-(2-chloro-3-(3-chloro-2-(4-((((1r,4r)-4-hydroxycyclohexyl)amino)methyl)-3-methoxyphenyl)pyridin-4-yl)phenyl)-2-methoxypyridin-3-yl)methyl)amino)cyclohexan-1-ol;
(1r,4s)-4-(((6-(2-chloro-3-(3-chloro-2-(4-((((1r,4s)-4-hydroxycyclohexyl)amino)methyl)-3-methoxyphenyl)pyridin-4-yl)phenyl)-2-methoxypyridin-3-yl)methyl)amino)cyclohexan-1-ol;
(1r,4s)-4-(((6-(2-chloro-3-(3-chloro-2-(4-((((1s,4r)-4-hydroxycyclohexyl)amino)methyl)-3-methoxyphenyl)pyridin-4-yl)phenyl)-2-methoxypyridin-3-yl)methyl)amino)cyclohexan-1-ol;
(1r,4s)-4-(((6-(2-chloro-3-(3-chloro-2-(4-((((1s,4s)-4-hydroxycyclohexyl)amino)methyl)-3-methoxyphenyl)pyridin-4-yl)phenyl)-2-methoxypyridin-3-yl)methyl)amino)cyclohexan-1-ol;
(1s,4r)-4-(((6-(2-chloro-3-(3-chloro-2-(4-((((1r,4r)-4-hydroxycyclohexyl)amino)methyl)-3-methoxyphenyl)pyridin-4-yl)phenyl)-2-methoxypyridin-3-yl)methyl)amino)cyclohexan-1-ol;
(1s,4r)-4-(((6-(2-chloro-3-(3-chloro-2-(4-((((1r,4s)-4-hydroxycyclohexyl)amino)methyl)-3-methoxyphenyl)pyridin-4-yl)phenyl)-2-methoxypyridin-3-yl)methyl)amino)cyclohexan-1-ol;
(1s,4r)-4-(((6-(2-chloro-3-(3-chloro-2-(4-((((1s,4r)-4-hydroxycyclohexyl)amino)methyl)-3-methoxyphenyl)pyridin-4-yl)phenyl)-2-methoxypyridin-3-yl)methyl)amino)cyclohexan-1-ol;
(1s,4r)-4-(((6-(2-chloro-3-(3-chloro-2-(4-((((1s,4s)-4-hydroxycyclohexyl)amino)methyl)-3-methoxyphenyl)pyridin-4-yl)phenyl)-2-methoxypyridin-3-yl)methyl)amino)cyclohexan-1-ol;
(1s,4s)-4-(((6-(2-chloro-3-(3-chloro-2-(4-((((1r,4r)-4-hydroxycyclohexyl)amino)methyl)-3-methoxyphenyl)pyridin-4-yl)phenyl)-2-methoxypyridin-3-yl)methyl)amino)cyclohexan-1-ol;
(1s,4s)-4-(((6-(2-chloro-3-(3-chloro-2-(4-((((1r,4s)-4-hydroxycyclohexyl)amino)methyl)-3-methoxyphenyl)pyridin-4-yl)phenyl)-2-methoxypyridin-3-yl)methyl)amino)cyclohexan-1-ol;
(1s,4s)-4-(((6-(2-chloro-3-(3-chloro-2-(4-((((1s,4r)-4-hydroxycyclohexyl)amino)methyl)-3-methoxyphenyl)pyridin-4-yl)phenyl)-2-methoxypyridin-3-yl)methyl)amino)cyclohexan-1-ol;
(1s,4s)-4-(((6-(2-chloro-3-(3-chloro-2-(4-((((1s,4s)-4-hydroxycyclohexyl)amino)methyl)-3-methoxyphenyl)pyridin-4-yl)phenyl)-2-methoxypyridin-3-yl)methyl)amino)cyclohexan-1-ol;
N-(4-(4-(3-(5-(((2-oxaspiro[3.3]heptan-6-yl)amino)methyl)-6-methoxypyridin-2-yl)-2-chlorophenyl)-3-chloropyridin-2-yl)-2-methoxybenzyl)-2-oxaspiro[3.3]heptan-6-amine;
(S)-2-(4-(3-chloro-4-(1-((6-methoxy-5-((7-oxo-2,6-diazaspiro[3.4]octan-2-yl)methyl)-3-(trifluoromethyl)pyrazin-2-yl)amino)-2,3-dihydro-1H-inden-4-yl)pyridin-2-yl)-2-methoxybenzyl)-2,6-diazaspiro[3.4]octan-7-one;
(R)-2-(4-(3-chloro-4-(1-((6-methoxy-5-((7-oxo-2,6-diazaspiro[3.4]octan-2-yl)methyl)-3-(trifluoromethyl)pyrazin-2-yl)amino)-2,3-dihydro-1H-inden-4-yl)pyridin-2-yl)-2-methoxybenzyl)-2,6-diazaspiro[3.4]octan-7-one;
(S)-1-(6-((5-((4-(2-(4-((6-acetyl-2,6-diazaspiro[3.3]heptan-2-yl)methyl)-3-methoxyphenyl)-3-chloropyridin-4-yl)-2,3-dihydro-1H-inden-1-yl)amino)-3-methoxy-6-(trifluoromethyl)pyrazin-2-yl)methyl)-2,6-diazaspiro[3.3]heptan-2-yl)ethan-1-one;
(R)-1-(6-((5-((4-(2-(4-((6-acetyl-2,6-diazaspiro[3.3]heptan-2-yl)methyl)-3-methoxyphenyl)-3-chloropyridin-4-yl)-2,3-dihydro-1 H-inden-1-yl)amino)-3-methoxy-6-(trifluoromethyl)pyrazin-2-yl)methyl)-2,6-diazaspiro[3.3]heptan-2-yl)ethan-1-one;
2-(4-(4-(2-chloro-3-(6-methoxy-5-((7-oxo-2,6-diazaspiro[3.4]octan-2-yl)methyl)pyridin-2-yl)phenyl)-3-methylpyridin-2-yl)-2-methoxybenzyl)-2,6-diazaspiro[3.4]octan-7-one;
1-(6-((6-(3-(2-(4-((6-acetyl-2,6-diazaspiro[3.3]heptan-2-yl)methyl)-3-methoxyphenyl)-3-methylpyridin-4-yl)-2-chlorophenyl)-2-methoxypyridin-3-yl)methyl)-2,6-diazaspiro[3.3]heptan-2-yl)ethan-1-one;
(S)-5-((((6-(2-chloro-3-(3-chloro-2-(2-((((1r,3r)-3-hydroxy-3-methylcyclobutyl)amino)methyl)-8-methoxy-[1,2,4]triazolo[1,5-a]pyridin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-((((1r,3r)-3-hydroxy-3-methylcyclobutyl)amino)methyl)-8-methoxy-[1,2,4]triazolo[1,5-a]pyridin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-((((1r,3s)-3-hydroxy-3-methylcyclobutyl)amino)methyl)-8-methoxy-[1,2,4]triazolo[1,5-a]pyridin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-((((1s,3r)-3-hydroxy-3-methylcyclobutyl)amino)methyl)-8-methoxy-[1,2,4]triazolo[1,5-a]pyridin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-((((1s,3s)-3-hydroxy-3-methylcyclobutyl)amino)methyl)-8-methoxy-[1,2,4]triazolo[1,5-a]pyridin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(2-((((1r,3s)-3-hydroxy-3-methylcyclobutyl)amino)methyl)-8-methoxy-[1,2,4]triazolo[1,5-a]pyridin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(2-((((1s,3r)-3-hydroxy-3-methylcyclobutyl)amino)methyl)-8-methoxy-[1,2,4]triazolo[1,5-a]pyridin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(2-((((1s,3s)-3-hydroxy-3-methylcyclobutyl)amino)methyl)-8-methoxy-[1,2,4]triazolo[1,5-a]pyridin-6-yl)pyridin-4-yl)phenyl)-2-methoxypyridin-3-yl)methyl)amino)methyl)pyrrolidin-2-one;
1-(4-((4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)amino)piperidin-1-yl)-2-methylpropan-1-one;
5-(((6-(3-(2-(4-(((5-amino-5-oxopentyl)amino)methyl)-3-methoxyphenyl)-3-chloropyridin-4-yl)-2-chlorophenyl)-2-methoxypyridin-3-yl)methyl)amino)pentanamide;
1-((R)-3-(((6-(3-(2-(4-((((R)-1-acetylpyrrolidin-3-yl)amino)methyl)-3-methoxyphenyl)-3-chloropyridin-4-yl)-2-chlorophenyl)-2-methoxypyridin-3-yl)methyl)amino)pyrrolidin-1-yl)ethan-1-one;
1-((S)-3-(((6-(3-(2-(4-((((R)-1-acetylpyrrolidin-3-yl)amino)methyl)-3-methoxyphenyl)-3-chloropyridin-4-yl)-2-chlorophenyl)-2-methoxypyridin-3-yl)methyl)amino)pyrrolidin-1-yl)ethan-1-one;
1-((R)-3-(((6-(3-(2-(4-((((S)-1-acetylpyrrolidin-3-yl)amino)methyl)-3-methoxyphenyl)-3-chloropyridin-4-yl)-2-chlorophenyl)-2-methoxypyridin-3-yl)methyl)amino)pyrrolidin-1-yl)ethan-1-one;
1-((S)-3-(((6-(3-(2-(4-((((S)-1-acetylpyrrolidin-3-yl)amino)methyl)-3-methoxyphenyl)-3-chloropyridin-4-yl)-2-chlorophenyl)-2-methoxypyridin-3-yl)methyl)amino)pyrrolidin-1-yl)ethan-1-one;
1-(4-(((6-(3-(6'-(((1-acetylpiperidin-4-yl)amino)methyl)-3-chloro-5'-methoxy-[2,3'-bipyridin]-4-yl)-2-chlorophenyl)-2-methoxypyridin-3-yl)methyl)amino)piperidin-1-yl)ethan-1-one;
2-((3-chloro-4-(2-chloro-3-(6-methoxy-5-((7-oxo-2,6-diazaspiro[3.4]octan-2-yl)methyl)pyridin-2-yl)phenyl)-5'-methoxy-[2,3'-bipyridin]-6'-yl)methyl)-2,6-diazaspiro[3.4]octan-7-one;
(S)-N-(1-((6-(3-chloro-4-(2-chloro-3-(6-methoxy-5-((((5-oxopyrrolidin-2-yl)methyl)amino)methyl)pyridin-2-yl)phenyl)pyridin-2-yl)-1-methyl-1H-pyrrolo[3,2-b]pyridin-3-yl)methyl)piperidin-4-yl)acetamide;
(R)-N-(1-((6-(3-chloro-4-(2-chloro-3-(6-methoxy-5-((((5-oxopyrrolidin-2-yl)methyl)amino)methyl)pyridin-2-yl)phenyl)pyridin-2-yl)-1-methyl-1H-pyrrolo[3,2-b]pyridin-3-yl)methyl)piperidin-4-yl)acetamide;
(S)-2-((6-(3-chloro-4-(2-chloro-3-(6-methoxy-5-((((5-oxopyrrolidin-2-yl)methyl)amino)methyl)pyridin-2-yl)phenyl)pyridin-2-yl)-1-methyl-1H-pyrrolo[3,2-b]pyridin-3-yl)methyl)-2,6-diazaspiro[3.4]octan-7-one;
(R)-2-((6-(3-chloro-4-(2-chloro-3-(6-methoxy-5-((((5-oxopyrrolidin-2-yl)methyl)amino)methyl)pyridin-2-yl)phenyl)pyridin-2-yl)-1-methyl-1H-pyrrolo[3,2-b]pyridin-3-yl)methyl)-2,6-diazaspiro[3.4]octan-7-one;
1-(4-(((6-(3-(2-(4-(((2-oxaspiro[3.3]heptan-6-yl)amino)methyl)-3-methoxyphenyl)-3-chloropyridin-4-yl)-2-chlorophenyl)-2-methoxypyridin-3-yl)methyl)amino)piperidin-1-yl)ethan-1-one;
1-(4-(((6-(3-(2-(4-((2-oxa-6-azaspiro[3.3]heptan-6-yl)methyl)-3-methoxyphenyl)-3-chloropyridin-4-yl)-2-chlorophenyl)-2-methoxypyridin-3-yl)methyl)amino)piperidin-1-yl)ethan-1-one;
1-(4-(((6-(2-chloro-3-(3-chloro-2-(4-(((3,3-dimethylcyclobutyl)amino)methyl)-3-methoxyphenyl)pyridin-4-yl)phenyl)-2-methoxypyridin-3-yl)methyl)amino)piperidin-1-yl)ethan-1-one;
1-(4-(((6-(3-(2-(4-(((1-acetylpiperidin-4-yl)amino)methyl)-3-methoxyphenyl)-3-chloropyridin-4-yl)-2-chlorophenyl)-2-methoxypyridin-3-yl)methyl)amino)piperidin-1-yl)-2-methylpropan-1-one;
(S)-5-((((6-(2-chloro-3-(3-chloro-2-(2-((((1r,4r)-4-hydroxycyclohexyl)amino)methyl)-8-methoxyimidazo[1,2-a]pyridin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-((((1r,4r)-4-hydroxycyclohexyl)amino)methyl)-8-methoxyimidazo[1,2-a]pyridin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-((((1r,4s)-4-hydroxycyclohexyl)amino)methyl)-8-methoxyimidazo[1,2-a]pyridin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-((((1s,4r)-4-hydroxycyclohexyl)amino)methyl)-8-methoxyimidazo[1,2-a]pyridin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-((((1s,4s)-4-hydroxycyclohexyl)amino)methyl)-8-methoxyimidazo[1,2-a]pyridin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(2-((((1r,4s)-4-hydroxycyclohexyl)amino)methyl)-8-methoxyimidazo[1,2-a]pyridin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(2-((((1s,4r)-4-hydroxycyclohexyl)amino)methyl)-8-methoxyimidazo[1,2-a]pyridin-6-yl)pyridin-4-yl)phenyl)-2-methoxypyridin-3-yl)methyl)amino)methyl)pyrrolidin-2-one;
(S)-5-((((6-(2-chloro-3-(3-chloro-2-(2-((((1s,4s)-4-hydroxycyclohexyl)amino)methyl)-8-methoxyimidazo[1,2-a]pyridin-6-yl)pyridin-4-yl)phenyl)-2-methoxypyridin-3-yl)methyl)amino)methyl)pyrrolidin-2-one;
(1r,3r)-3-(((6-(2-chloro-3-(3-chloro-2-(4-((((1r,3r)-3-hydroxy-3-methylcyclobutyl)amino)methyl)-3-methoxyphenyl)pyridin-4-yl)phenyl)-2-methoxypyridin-3-yl)methyl)amino)-1-methylcyclobutan-1-ol;
(1r,3r)-3-(((6-(2-chloro-3-(3-chloro-2-(4-((((1r,3s)-3-hydroxy-3-methylcyclobutyl)amino)methyl)-3-methoxyphenyl)pyridin-4-yl)phenyl)-2-methoxypyridin-3-yl)methyl)amino)-1-methylcyclobutan-1-ol;
(1r,3r)-3-(((6-(2-chloro-3-(3-chloro-2-(4-((((1s,3r)-3-hydroxy-3-methylcyclobutyl)amino)methyl)-3-methoxyphenyl)pyridin-4-yl)phenyl)-2-methoxypyridin-3-yl)methyl)amino)-1-methylcyclobutan-1-ol;
(1r,3r)-3-(((6-(2-chloro-3-(3-chloro-2-(4-((((1s,3s)-3-hydroxy-3-methylcyclobutyl)amino)methyl)-3-methoxyphenyl)pyridin-4-yl)phenyl)-2-methoxypyridin-3-yl)methyl)amino)-1-methylcyclobutan-1-ol;
(1r,3s)-3-(((6-(2-chloro-3-(3-chloro-2-(4-((((1r,3r)-3-hydroxy-3-methylcyclobutyl)amino)methyl)-3-methoxyphenyl)pyridin-4-yl)phenyl)-2-methoxypyridin-3-yl)methyl)amino)-1-methylcyclobutan-1-ol;
(1r,3s)-3-(((6-(2-chloro-3-(3-chloro-2-(4-((((1r,3s)-3-hydroxy-3-methylcyclobutyl)amino)methyl)-3-methoxyphenyl)pyridin-4-yl)phenyl)-2-methoxypyridin-3-yl)methyl)amino)-1-methylcyclobutan-1-ol;
(1r,3s)-3-(((6-(2-chloro-3-(3-chloro-2-(4-((((1s,3r)-3-hydroxy-3-methylcyclobutyl)amino)methyl)-3-methoxyphenyl)pyridin-4-yl)phenyl)-2-methoxypyridin-3-yl)methyl)amino)-1-methylcyclobutan-1-ol;
(1r,3s)-3-(((6-(2-chloro-3-(3-chloro-2-(4-((((1s,3s)-3-hydroxy-3-methylcyclobutyl)amino)methyl)-3-methoxyphenyl)pyridin-4-yl)phenyl)-2-methoxypyridin-3-yl)methyl)amino)-1-methylcyclobutan-1-ol;
(1s,3r)-3-(((6-(2-chloro-3-(3-chloro-2-(4-((((1r,3r)-3-hydroxy-3-methylcyclobutyl)amino)methyl)-3-methoxyphenyl)pyridin-4-yl)phenyl)-2-methoxypyridin-3-yl)methyl)amino)-1-methylcyclobutan-1-ol;
(1s,3r)-3-(((6-(2-chloro-3-(3-chloro-2-(4-((((1r,3s)-3-hydroxy-3-methylcyclobutyl)amino)methyl)-3-methoxyphenyl)pyridin-4-yl)phenyl)-2-methoxypyridin-3-yl)methyl)amino)-1-methylcyclobutan-1-ol;
(1s,3r)-3-(((6-(2-chloro-3-(3-chloro-2-(4-((((1s,3r)-3-hydroxy-3-methylcyclobutyl)amino)methyl)-3-methoxyphenyl)pyridin-4-yl)phenyl)-2-methoxypyridin-3-yl)methyl)amino)-1-methylcyclobutan-1-ol;
(1s,3r)-3-(((6-(2-chloro-3-(3-chloro-2-(4-((((1s,3s)-3-hydroxy-3-methylcyclobutyl)amino)methyl)-3-methoxyphenyl)pyridin-4-yl)phenyl)-2-methoxypyridin-3-yl)methyl)amino)-1-methylcyclobutan-1-ol;
(1s,3s)-3-(((6-(2-chloro-3-(3-chloro-2-(4-((((1r,3r)-3-hydroxy-3-methylcyclobutyl)amino)methyl)-3-methoxyphenyl)pyridin-4-yl)phenyl)-2-methoxypyridin-3-yl)methyl)amino)-1-methylcyclobutan-1-ol;
(1s,3s)-3-(((6-(2-chloro-3-(3-chloro-2-(4-((((1r,3s)-3-hydroxy-3-methylcyclobutyl)amino)methyl)-3-methoxyphenyl)pyridin-4-yl)phenyl)-2-methoxypyridin-3-yl)methyl)amino)-1-methylcyclobutan-1-ol;
(1s,3s)-3-(((6-(2-chloro-3-(3-chloro-2-(4-((((1s,3r)-3-hydroxy-3-methylcyclobutyl)amino)methyl)-3-methoxyphenyl)pyridin-4-yl)phenyl)-2-methoxypyridin-3-yl)methyl)amino)-1-methylcyclobutan-1-ol;
(1s,3s)-3-(((6-(2-chloro-3-(3-chloro-2-(4-((((1s,3s)-3-hydroxy-3-methylcyclobutyl)amino)methyl)-3-methoxyphenyl)pyridin-4-yl)phenyl)-2-methoxypyridin-3-yl)methyl)amino)-1-methylcyclobutan-1-ol;
2-(4-(3-chloro-4-(2-chloro-3-(5-((6-hydroxy-2-azaspiro[3.3]heptan-2-yl)methyl)-6-methoxypyridin-2-yl)phenyl)pyridin-2-yl)-2-methoxybenzyl)-2-azaspiro[3.3]heptan-6-ol;
6-(4-(4-(3-(5-((2-oxa-6-azaspiro[3.3]heptan-6-yl)methyl)-6-methoxypyridin-2-yl)-2-chlorophenyl)-3-chloropyridin-2-yl)-2-methoxybenzyl)-2-oxa-6-azaspiro[3.3]heptane;
(S)-1-((6-(3-(2-(4-(((1-acetylpiperidin-4-yl)amino)methyl)-3-methoxyphenyl)-3-chloropyridin-4-yl)-2-chlorophenyl)-2-methoxypyridin-3-yl)methyl)pyrrolidine-3-carboxylic acid;
(R)-1-((6-(3-(2-(4-(((1-acetylpiperidin-4-yl)amino)methyl)-3-methoxyphenyl)-3-chloropyridin-4-yl)-2-chlorophenyl)-2-methoxypyridin-3-yl)methyl)pyrrolidine-3-carboxylic acid;
3-(((6-(3-(2-(4-(((1-acetylpiperidin-4-yl)amino)methyl)-3-methoxyphenyl)-3-chloropyridin-4-yl)-2-chlorophenyl)-2-methoxypyridin-3-yl)methyl)amino)propanoic acid;
3-((4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)amino)propanoic acid;
1-(4-((4-(3-chloro-4-(2-chloro-3-(6-methoxy-5-(((tetrahydro-2H-pyran-4-yl)amino)methyl)pyridin-2-yl)phenyl)pyridin-2-yl)-2-methoxybenzyl)amino)piperidin-1-yl)ethan-1-one;
1-(4-(((6-(2-chloro-3-(3-chloro-2-(3-methoxy-4-(((tetrahydro-2H-pyran-4-yl)amino)methyl)phenyl)pyridin-4-yl)phenyl)-2-methoxypyridin-3-yl)methyl)amino)piperidin-1-yl)ethan-1-one;
(S)-1-(6-(3-(2-(4-((S)-1-aminoethyl)-3-methoxyphenyl)-3-chloropyridin-4-yl)-2-chlorophenyl)-2-methoxypyridin-3-yl)ethan-1-amine;
(R)-1-(6-(3-(2-(4-((S)-1-aminoethyl)-3-methoxyphenyl)-3-chloropyridin-4-yl)-2-chlorophenyl)-2-methoxypyridin-3-yl)ethan-1-amine;
(S)-1-(6-(3-(2-(4-((R)-1-aminoethyl)-3-methoxyphenyl)-3-chloropyridin-4-yl)-2-chlorophenyl)-2-methoxypyridin-3-yl)ethan-1-amine;
(R)-1-(6-(3-(2-(4-((R)-1-aminoethyl)-3-methoxyphenyl)-3-chloropyridin-4-yl)-2-chlorophenyl)-2-methoxypyridin-3-yl)ethan-1-amine;
1-(4-(((6-(3-(2-(4-(((1-acetylpiperidin-4-yl)amino)methyl)-3-(difluoromethoxy)phenyl)-3-chloropyridin-4-yl)-2-chlorophenyl)-2-methoxypyridin-3-yl)methyl)amino)piperidin-1-yl)ethan-1-one;
1-(6-(3-(3-chloro-2-(3-methoxy-4-((methylamino)methyl)phenyl)pyridin-4-yl)-2-methylphenyl)-2-methoxypyridin-3-yl)-N-methylmethanamine;
1-((6-(2-chloro-3-(3-chloro-2-(4-((3-hydroxy-3-methylazetidin-1-yl)methyl)-3-methoxyphenyl)pyridin-4-yl)phenyl)-2-methoxypyridin-3-yl)methyl)-3-methylazetidin-3-ol;
1-(4-(((6-(2-chloro-3-(3-chloro-2-(3-methoxy-4-(((1-(2-methoxyacetyl)piperidin-4-yl)amino)methyl)phenyl)pyridin-4-yl)phenyl)-2-methoxypyridin-3-yl)methyl)amino)piperidin-1-yl)-2-methoxyethan-1-one;
2-(4-(3-chloro-4-(2-chloro-3-(6-methoxy-5-((6-oxo-2,5-diazaspiro[3.4]octan-2-yl)methyl)pyridin-2-yl)phenyl)pyridin-2-yl)-2-methoxybenzyl)-2,5-diazaspiro[3.4]octan-6-one;
1-(4-(((6-(3-(2-(4-(((1-acetylpiperidin-4-yl)amino)methyl)-3-methoxyphenyl)-3-chloropyridin-4-yl)-2-methylphenyl)-2-methoxypyridin-3-yl)methyl)amino)piperidin-1-yl)ethan-1-one;
N-(1-(4-(3-chloro-4-(2-chloro-3-(6-methoxy-5-((7-oxo-2,6-diazaspiro[3.4]octan-2-yl)methyl)pyridin-2-yl)phenyl)pyridin-2-yl)-2-methoxybenzyl)piperidin-4-yl)acetamide;
N-(1-(4-(3-chloro-4-(2-chloro-3-(6-methoxy-5-((6-oxo-2,5-diazaspiro[3.4]octan-2-yl)methyl)pyridin-2-yl)phenyl)pyridin-2-yl)-2-methoxybenzyl)piperidin-4-yl)acetamide;
N-(1-(4-(4-(3-(5-((6-acetyl-2,6-diazaspiro[3.3]heptan-2-yl)methyl)-6-methoxypyridin-2-yl)-2-chlorophenyl)-3-chloropyridin-2-yl)-2-methoxybenzyl)piperidin-4-yl)acetamide;
(S)-N-(1-(4-(3-chloro-4-(2-chloro-3-(6-methoxy-5-((((5-oxopyrrolidin-2-yl)methyl)amino)methyl)pyridin-2-yl)phenyl)pyridin-2-yl)-2-methoxybenzyl)piperidin-4-yl)acetamide;
(R)-N-(1-(4-(3-chloro-4-(2-chloro-3-(6-methoxy-5-((((5-oxopyrrolidin-2-yl)methyl)amino)methyl)pyridin-2-yl)phenyl)pyridin-2-yl)-2-methoxybenzyl)piperidin-4-yl)acetamide;
2-(4-(((1-acetylpiperidin-4-yl)amino)methyl)-3-methoxyphenyl)-4-(3-(5-(((1-acetylpiperidin-4-yl)amino)methyl)-6-methoxypyridin-2-yl)-2-chlorophenyl)nicotinonitrile;
(S)-4-((((6-(2-chloro-3-(3-chloro-2-(3-methoxy-4-(((((S)-5-oxopyrrolidin-3-yl)methyl)amino)methyl)phenyl)pyridin-4-yl)phenyl)-2-methoxypyridin-3-yl)methyl)amino)methyl)pyrrolidin-2-one;
(S)-4-((((6-(2-chloro-3-(3-chloro-2-(3-methoxy-4-(((((R)-5-oxopyrrolidin-3-yl)methyl)amino)methyl)phenyl)pyridin-4-yl)phenyl)-2-methoxypyridin-3-yl)methyl)amino)methyl)pyrrolidin-2-one;
(R)-4-((((6-(2-chloro-3-(3-chloro-2-(3-methoxy-4-(((((S)-5-oxopyrrolidin-3-yl)methyl)amino)methyl)phenyl)pyridin-4-yl)phenyl)-2-methoxypyridin-3-yl)methyl)amino)methyl)pyrrolidin-2-one;
(R)-4-((((6-(2-chloro-3-(3-chloro-2-(3-methoxy-4-(((((R)-5-oxopyrrolidin-3-yl)methyl)amino)methyl)phenyl)pyridin-4-yl)phenyl)-2-methoxypyridin-3-yl)methyl)amino)methyl)pyrrolidin-2-one;
1-(4-(((6-(3-(2-(4-((4-amino-4-methylpiperidin-1-yl)methyl)-3-methoxyphenyl)-3-chloropyridin-4-yl)-2-chlorophenyl)-2-methoxypyridin-3-yl)methyl)amino)piperidin-1-yl)ethan-1-one;
(S)-4-(((4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)amino)methyl)pyrrolidin-2-one;
(R)-4-(((4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)amino)methyl)pyrrolidin-2-one;
2-(4-(3-chloro-4-(3-(6-methoxy-5-((7-oxo-2,6-diazaspiro[3.4]octan-2-yl)methyl)pyridin-2-yl)-2-methylphenyl)pyridin-2-yl)-2-methoxybenzyl)-2,6-diazaspiro[3.4]octan-7-one;
1-(6-((6-(3-(2-(4-((6-acetyl-2,6-diazaspiro[3.3]heptan-2-yl)methyl)-3-methoxyphenyl)-3-chloropyridin-4-yl)-2-methylphenyl)-2-methoxypyridin-3-yl)methyl)-2,6-diazaspiro[3.3]heptan-2-yl)ethan-1-one;
(1s,4s)-4-(((6-(2-chloro-3-(3-chloro-2-(4-((((1s,4s)-4-hydroxycyclohexyl)amino)methyl)-3-methoxyphenyl)pyridin-4-yl)phenyl)-2-methoxypyridin-3-yl)methyl)amino)cyclohexan-1-ol;
(1r,4r)-4-(((6-(2-chloro-3-(3-chloro-2-(4-((((1r,4r)-4-hydroxycyclohexyl)amino)methyl)-3-methoxyphenyl)pyridin-4-yl)phenyl)-2-methoxypyridin-3-yl)methyl)amino)cyclohexan-1-ol;
(1r,4r)-4-(((6-(2-chloro-3-(3-chloro-2-(4-((((1r,4s)-4-hydroxycyclohexyl)amino)methyl)-3-methoxyphenyl)pyridin-4-yl)phenyl)-2-methoxypyridin-3-yl)methyl)amino)cyclohexan-1-ol;
(1r,4r)-4-(((6-(2-chloro-3-(3-chloro-2-(4-((((1s,4r)-4-hydroxycyclohexyl)amino)methyl)-3-methoxyphenyl)pyridin-4-yl)phenyl)-2-methoxypyridin-3-yl)methyl)amino)cyclohexan-1-ol;
(1r,4r)-4-(((6-(2-chloro-3-(3-chloro-2-(4-((((1s,4s)-4-hydroxycyclohexyl)amino)methyl)-3-methoxyphenyl)pyridin-4-yl)phenyl)-2-methoxypyridin-3-yl)methyl)amino)cyclohexan-1-ol;
(1r,4s)-4-(((6-(2-chloro-3-(3-chloro-2-(4-((((1r,4r)-4-hydroxycyclohexyl)amino)methyl)-3-methoxyphenyl)pyridin-4-yl)phenyl)-2-methoxypyridin-3-yl)methyl)amino)cyclohexan-1-ol;
(1r,4s)-4-(((6-(2-chloro-3-(3-chloro-2-(4-((((1r,4s)-4-hydroxycyclohexyl)amino)methyl)-3-methoxyphenyl)pyridin-4-yl)phenyl)-2-methoxypyridin-3-yl)methyl)amino)cyclohexan-1-ol;
(1r,4s)-4-(((6-(2-chloro-3-(3-chloro-2-(4-((((1s,4r)-4-hydroxycyclohexyl)amino)methyl)-3-methoxyphenyl)pyridin-4-yl)phenyl)-2-methoxypyridin-3-yl)methyl)amino)cyclohexan-1-ol;
(1r,4s)-4-(((6-(2-chloro-3-(3-chloro-2-(4-((((1s,4s)-4-hydroxycyclohexyl)amino)methyl)-3-methoxyphenyl)pyridin-4-yl)phenyl)-2-methoxypyridin-3-yl)methyl)amino)cyclohexan-1-ol;
(1s,4r)-4-(((6-(2-chloro-3-(3-chloro-2-(4-((((1r,4r)-4-hydroxycyclohexyl)amino)methyl)-3-methoxyphenyl)pyridin-4-yl)phenyl)-2-methoxypyridin-3-yl)methyl)amino)cyclohexan-1-ol;
(1s,4r)-4-(((6-(2-chloro-3-(3-chloro-2-(4-((((1r,4s)-4-hydroxycyclohexyl)amino)methyl)-3-methoxyphenyl)pyridin-4-yl)phenyl)-2-methoxypyridin-3-yl)methyl)amino)cyclohexan-1-ol;
(1s,4r)-4-(((6-(2-chloro-3-(3-chloro-2-(4-((((1s,4r)-4-hydroxycyclohexyl)amino)methyl)-3-methoxyphenyl)pyridin-4-yl)phenyl)-2-methoxypyridin-3-yl)methyl)amino)cyclohexan-1-ol;
(1s,4r)-4-(((6-(2-chloro-3-(3-chloro-2-(4-((((1s,4s)-4-hydroxycyclohexyl)amino)methyl)-3-methoxyphenyl)pyridin-4-yl)phenyl)-2-methoxypyridin-3-yl)methyl)amino)cyclohexan-1-ol;
(1s,4s)-4-(((6-(2-chloro-3-(3-chloro-2-(4-((((1r,4r)-4-hydroxycyclohexyl)amino)methyl)-3-methoxyphenyl)pyridin-4-yl)phenyl)-2-methoxypyridin-3-yl)methyl)amino)cyclohexan-1-ol;
(1s,4s)-4-(((6-(2-chloro-3-(3-chloro-2-(4-((((1r,4s)-4-hydroxycyclohexyl)amino)methyl)-3-methoxyphenyl)pyridin-4-yl)phenyl)-2-methoxypyridin-3-yl)methyl)amino)cyclohexan-1-ol;
(1s,4s)-4-(((6-(2-chloro-3-(3-chloro-2-(4-((((1s,4r)-4-hydroxycyclohexyl)amino)methyl)-3-methoxyphenyl)pyridin-4-yl)phenyl)-2-methoxypyridin-3-yl)methyl)amino)cyclohexan-1-ol;
4-((6-(2-chloro-3-(3-chloro-2-(3-methoxy-4-(morpholinomethyl)phenyl)pyridin-4-yl)phenyl)-2-methoxypyridin-3-yl)methyl)morpholine;
1-((6-(2-chloro-3-(3-chloro-2-(4-((4-hydroxypiperidin-1-yl)methyl)-3-methoxyphenyl)pyridin-4-yl)phenyl)-2-methoxypyridin-3-yl)methyl)piperidin-4-ol;
1-(4-((4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)amino)piperidin-1-yl)-2-methoxyethan-1-one;
(S)-5-((((6-(2-chloro-3-(2-(3-methoxy-4-(((((S)-5-oxopyrrolidin-2-yl)methyl)amino)methyl)phenyl)-3-methylpyridin-4-yl)phenyl)-2-methoxypyridin-3-yl)methyl)amino)methyl)pyrrolidin-2-one;
(S)-5-((((6-(2-chloro-3-(2-(3-methoxy-4-(((((R)-5-oxopyrrolidin-2-yl)methyl)amino)methyl)phenyl)-3-methylpyridin-4-yl)phenyl)-2-methoxypyridin-3-yl)methyl)amino)methyl)pyrrolidin-2-one;
(R)-5-((((6-(2-chloro-3-(2-(3-methoxy-4-(((((S)-5-oxopyrrolidin-2-yl)methyl)amino)methyl)phenyl)-3-methylpyridin-4-yl)phenyl)-2-methoxypyridin-3-yl)methyl)amino)methyl)pyrrolidin-2-one;
(R)-5-((((6-(2-chloro-3-(2-(3-methoxy-4-(((((R)-5-oxopyrrolidin-2-yl)methyl)amino)methyl)phenyl)-3-methylpyridin-4-yl)phenyl)-2-methoxypyridin-3-yl)methyl)amino)methyl)pyrrolidin-2-one;
2-(2-methoxy-4-(4-(3-(6-methoxy-5-((7-oxo-2,6-diazaspiro[3.4]octan-2-yl)methyl)pyridin-2-yl)-2-methylphenyl)-3-methylpyridin-2-yl)benzyl)-2,6-diazaspiro[3.4]octan-7-one;
1-(6-((6-(3-(2-(4-((6-acetyl-2,6-diazaspiro[3.3]heptan-2-yl)methyl)-3-methoxyphenyl)-3-methylpyridin-4-yl)-2-methylphenyl)-2-methoxypyridin-3-yl)methyl)-2,6-diazaspiro[3.3]heptan-2-yl)ethan-1-one;
1-(4-(((6-(2-chloro-3-(3-chloro-2-(3-methoxy-4-((piperidin-4-ylamino)methyl)phenyl)pyridin-4-yl)phenyl)-2-methoxypyridin-3-yl)methyl)amino)piperidin-1-yl)ethan-1-one;
4-(((6-(2-chloro-3-(3-chloro-2-(3-methoxy-4-(((1-methyl-2-oxopiperidin-4-yl)amino)methyl)phenyl)pyridin-4-yl)phenyl)-2-methoxypyridin-3-yl)methyl)amino)-1-methylpiperidin-2-one;
4-(((6-(2-chloro-3-(3-chloro-2-(4-(((1-(dimethylcarbamoyl)piperidin-4-yl)amino)methyl)-3-methoxyphenyl)pyridin-4-yl)phenyl)-2-methoxypyridin-3-yl)methyl)amino)-N,N-dimethylpiperidine-1-carboxamide;
N-((6-(2-chloro-3-(3-chloro-2-(4-(((1-(2,2-difluoroethyl)piperidin-4-yl)amino)methyl)-3-methoxyphenyl)pyridin-4-yl)phenyl)-2-methoxypyridin-3-yl)methyl)-1-(2,2-difluoroethyl)piperidin-4-amine;
N-((6-(2-chloro-3-(3-chloro-2-(3-methoxy-4-(((1-phenylpiperidin-4-yl)amino)methyl)phenyl)pyridin-4-yl)phenyl)-2-methoxypyridin-3-yl)methyl)-1-phenylpiperidin-4-amine;
N-(2-methoxy-4-(4-(3-(6-methoxy-5-(((tetrahydro-2H-pyran-4-yl)amino)methyl)pyridin-2-yl)-2-methylphenyl)-3-methylpyridin-2-yl)benzyl)tetrahydro-2H-pyran-4-amine;
N-((6-(3-(3-chloro-2-(3-methoxy-4-(((tetrahydro-2H-pyran-4-yl)amino)methyl)phenyl)pyridin-4-yl)-2-methylphenyl)-2-methoxypyridin-3-yl)methyl)tetrahydro-2H-pyran-4-amine;
1-(4-(((6-(2-chloro-3-(3-chloro-2-(3-methoxy-4-((methyl(tetrahydro-2H-pyran-4-yl)amino)methyl)phenyl)pyridin-4-yl)phenyl)-2-methoxypyridin-3-yl)methyl)amino)piperidin-1-yl)ethan-1-one;
1-(6-((6-(3-(6'-((6-acetyl-2,6-diazaspiro[3.3]heptan-2-yl)methyl)-3-chloro-5'-methoxy-[2,3'-bipyridin]-4-yl)-2-chlorophenyl)-2-methoxypyridin-3-yl)methyl)-2,6-diazaspiro[3.3]heptan-2-yl)ethan-1-one;
N-((4-(3-(5-(((2-oxaspiro[3.3]heptan-6-yl)amino)methyl)-6-methoxypyridin-2-yl)-2-chlorophenyl)-3-chloro-5'-methoxy-[2,3'-bipyridin]-6'-yl)methyl)-2-oxaspiro[3.3]heptan-6-amine;
2-((3-chloro-4-(2-chloro-3-(6-methoxy-5-((6-oxo-2,5-diazaspiro[3.4]octan-2-yl)methyl)pyridin-2-yl)phenyl)-5'-methoxy-[2,3'-bipyridin]-6'-yl)methyl)-2,5-diazaspiro[3.4]octan-6-one;
N-((6-(2-chloro-3-(3-chloro-5'-methoxy-6'-(((tetrahydro-2H-pyran-4-yl)amino)methyl)-[2,3'-bipyridin]-4-yl)phenyl)-2-methoxypyridin-3-yl)methyl)tetrahydro-2H-pyran-4-amine;
N-(1-((6-(2-chloro-3-(3-chloro-2-(3-methoxy-4-(((tetrahydro-2H-pyran-4-yl)amino)methyl)phenyl)pyridin-4-yl)phenyl)-2-methoxypyridin-3-yl)methyl)piperidin-4-yl)acetamide;
4-(2-chloro-3-(6-methoxy-5-((7-oxo-2,6-diazaspiro[3.4]octan-2-yl)methyl)pyridin-2-yl)phenyl)-2-(3-methoxy-4-((7-oxo-2,6-diazaspiro[3.4]octan-2-yl)methyl)phenyl)nicotinonitrile;
4-(2-chloro-3-(6-methoxy-5-(((tetrahydro-2H-pyran-4-yl)amino)methyl)pyridin-2-yl)phenyl)-2-(3-methoxy-4-(((tetrahydro-2H-pyran-4-yl)amino)methyl)phenyl)nicotinonitrile;
(S)-1-((6-(2-chloro-3-(3-chloro-2-(4-(((S)-3-hydroxypyrrolidin-1-yl)methyl)-3-methoxyphenyl)pyridin-4-yl)phenyl)-2-methoxypyridin-3-yl)methyl)pyrrolidin-3-ol;
(S)-1-((6-(2-chloro-3-(3-chloro-2-(4-(((R)-3-hydroxypyrrolidin-1-yl)methyl)-3-methoxyphenyl)pyridin-4-yl)phenyl)-2-methoxypyridin-3-yl)methyl)pyrrolidin-3-ol;
(R)-1-((6-(2-chloro-3-(3-chloro-2-(4-(((S)-3-hydroxypyrrolidin-1-yl)methyl)-3-methoxyphenyl)pyridin-4-yl)phenyl)-2-methoxypyridin-3-yl)methyl)pyrrolidin-3-ol;
(R)-1-((6-(2-chloro-3-(3-chloro-2-(4-(((R)-3-hydroxypyrrolidin-1-yl)methyl)-3-methoxyphenyl)pyridin-4-yl)phenyl)-2-methoxypyridin-3-yl)methyl)pyrrolidin-3-ol;
1-(4-((4-(3-chloro-4-(2-chloro-3-(6-methoxy-5-((methyl(tetrahydro-2H-pyran-4-yl)amino)methyl)pyridin-2-yl)phenyl)pyridin-2-yl)-2-methoxybenzyl)amino)piperidin-1-yl)ethan-1-one;
N-((6-(2-chloro-3-(3-chloro-2-(3-methoxy-4-(((1-(2,2,2-trifluoroethyl)piperidin-4-yl)amino)methyl)phenyl)pyridin-4-yl)phenyl)-2-methoxypyridin-3-yl)methyl)-1-(2,2,2-trifluoroethyl)piperidin-4-amine;
2-(4-(3-chloro-4-(2-fluoro-3-(6-methoxy-5-((7-oxo-2,6-diazaspiro[3.4]octan-2-yl)methyl)pyridin-2-yl)phenyl)pyridin-2-yl)-2-methoxybenzyl)-2,6-diazaspiro[3.4]octan-7-one;
1-(4-(((6-(3-(2-(4-(((1-acetylpiperidin-4-yl)amino)methyl)-3-methoxyphenyl)-3-chloropyridin-4-yl)-2-fluorophenyl)-2-methoxypyridin-3-yl)methyl)amino)piperidin-1-yl)ethan-1-one;
N-((6-(3-(3-chloro-2-(3-methoxy-4-(((tetrahydro-2H-pyran-4-yl)amino)methyl)phenyl)pyridin-4-yl)-2-fluorophenyl)-2-methoxypyridin-3-yl)methyl)tetrahydro-2H-pyran-4-amine;
N-(4-(3-chloro-4-(2-chloro-3-(6-methoxy-5-(((tetrahydro-2H-pyran-4-yl)amino)methyl)pyridin-2-yl)phenyl)pyridin-2-yl)-2-methoxybenzyl)-N-methyltetrahydro-2H-pyran-4-amine;
1-(4-((4-(3-chloro-4-(2-chloro-3-(6-methoxy-5-(((tetrahydro-2H-pyran-4-yl)amino)methyl)pyridin-2-yl)phenyl)pyridin-2-yl)-2-methoxybenzyl)(methyl)amino)piperidin-1-yl)ethan-1-one;
1-(4-(((6-(2-chloro-3-(3-chloro-2-(4-(((1-(2-hydroxyacetyl)piperidin-4-yl)amino)methyl)-3-methoxyphenyl)pyridin-4-yl)phenyl)-2-methoxypyridin-3-yl)methyl)amino)piperidin-1-yl)-2-hydroxyethan-1-one;
N-((6-(2-chloro-3-(3-chloro-2-(3-methoxy-4-(((1-(pyridin-2-yl)piperidin-4-yl)amino)methyl)phenyl)pyridin-4-yl)phenyl)-2-methoxypyridin-3-yl)methyl)-1-(pyridin-2-yl)piperidin-4-amine;
N-((6-(2-chloro-3-(3-chloro-2-(4-(((1-(3-chloropyridin-2-yl)piperidin-4-yl)amino)methyl)-3-methoxyphenyl)pyridin-4-yl)phenyl)-2-methoxypyridin-3-yl)methyl)-1-(3-chloropyridin-2-yl)piperidin-4-amine;
N-((6-(2-chloro-3-(3-chloro-2-(3-methoxy-4-(((2-methoxyethyl)amino)methyl)phenyl)pyridin-4-yl)phenyl)-2-methoxypyridin-3-yl)methyl)-2-methoxyethan-1-amine;
3-chloro-4-(2-chloro-3-(6-methoxy-5-((4-methoxypiperidin-1-yl)methyl)pyridin-2-yl)phenyl)-2-(3-methoxy-4-((4-methoxypiperidin-1-yl)methyl)phenyl)pyridine;
(1s,4s)-N-((6-(2-chloro-3-(3-chloro-2-(3-methoxy-4-((((1s,4s)-4-methoxycyclohexyl)amino)methyl)phenyl)pyridin-4-yl)phenyl)-2-methoxypyridin-3-yl)methyl)-4-methoxycyclohexan-1-amine;
(1r,4r)-N-((6-(2-chloro-3-(3-chloro-2-(3-methoxy-4-((((1r,4r)-4-methoxycyclohexyl)amino)methyl)phenyl)pyridin-4-yl)phenyl)-2-methoxypyridin-3-yl)methyl)-4-methoxycyclohexan-1-amine;
(1r,4r)-N-((6-(2-chloro-3-(3-chloro-2-(3-methoxy-4-((((1r,4s)-4-methoxycyclohexyl)amino)methyl)phenyl)pyridin-4-yl)phenyl)-2-methoxypyridin-3-yl)methyl)-4-methoxycyclohexan-1-amine;
(1r,4r)-N-((6-(2-chloro-3-(3-chloro-2-(3-methoxy-4-((((1s,4r)-4-methoxycyclohexyl)amino)methyl)phenyl)pyridin-4-yl)phenyl)-2-methoxypyridin-3-yl)methyl)-4-methoxycyclohexan-1-amine;
(1r,4r)-N-((6-(2-chloro-3-(3-chloro-2-(3-methoxy-4-((((1s,4s)-4-methoxycyclohexyl)amino)methyl)phenyl)pyridin-4-yl)phenyl)-2-methoxypyridin-3-yl)methyl)-4-methoxycyclohexan-1-amine;
(1r,4s)-N-((6-(2-chloro-3-(3-chloro-2-(3-methoxy-4-((((1r,4r)-4-methoxycyclohexyl)amino)methyl)phenyl)pyridin-4-yl)phenyl)-2-methoxypyridin-3-yl)methyl)-4-methoxycyclohexan-1-amine;
(1r,4s)-N-((6-(2-chloro-3-(3-chloro-2-(3-methoxy-4-((((1r,4s)-4-methoxycyclohexyl)amino)methyl)phenyl)pyridin-4-yl)phenyl)-2-methoxypyridin-3-yl)methyl)-4-methoxycyclohexan-1-amine;
(1r,4s)-N-((6-(2-chloro-3-(3-chloro-2-(3-methoxy-4-((((1s,4r)-4-methoxycyclohexyl)amino)methyl)phenyl)pyridin-4-yl)phenyl)-2-methoxypyridin-3-yl)methyl)-4-methoxycyclohexan-1-amine;
(1r,4s)-N-((6-(2-chloro-3-(3-chloro-2-(3-methoxy-4-((((1s,4s)-4-methoxycyclohexyl)amino)methyl)phenyl)pyridin-4-yl)phenyl)-2-methoxypyridin-3-yl)methyl)-4-methoxycyclohexan-1-amine;
(1s,4r)-N-((6-(2-chloro-3-(3-chloro-2-(3-methoxy-4-((((1r,4r)-4-methoxycyclohexyl)amino)methyl)phenyl)pyridin-4-yl)phenyl)-2-methoxypyridin-3-yl)methyl)-4-methoxycyclohexan-1-amine;
(1s,4r)-N-((6-(2-chloro-3-(3-chloro-2-(3-methoxy-4-((((1r,4s)-4-methoxycyclohexyl)amino)methyl)phenyl)pyridin-4-yl)phenyl)-2-methoxypyridin-3-yl)methyl)-4-methoxycyclohexan-1-amine;
(1s,4r)-N-((6-(2-chloro-3-(3-chloro-2-(3-methoxy-4-((((1s,4r)-4-methoxycyclohexyl)amino)methyl)phenyl)pyridin-4-yl)phenyl)-2-methoxypyridin-3-yl)methyl)-4-methoxycyclohexan-1-amine;
(1s,4r)-N-((6-(2-chloro-3-(3-chloro-2-(3-methoxy-4-((((1s,4s)-4-methoxycyclohexyl)amino)methyl)phenyl)pyridin-4-yl)phenyl)-2-methoxypyridin-3-yl)methyl)-4-methoxycyclohexan-1-amine;
(1s,4s)-N-((6-(2-chloro-3-(3-chloro-2-(3-methoxy-4-((((1r,4r)-4-methoxycyclohexyl)amino)methyl)phenyl)pyridin-4-yl)phenyl)-2-methoxypyridin-3-yl)methyl)-4-methoxycyclohexan-1-amine;
(1s,4s)-N-((6-(2-chloro-3-(3-chloro-2-(3-methoxy-4-((((1r,4s)-4-methoxycyclohexyl)amino)methyl)phenyl)pyridin-4-yl)phenyl)-2-methoxypyridin-3-yl)methyl)-4-methoxycyclohexan-1-amine;
(1s,4s)-N-((6-(2-chloro-3-(3-chloro-2-(3-methoxy-4-((((1s,4r)-4-methoxycyclohexyl)amino)methyl)phenyl)pyridin-4-yl)phenyl)-2-methoxypyridin-3-yl)methyl)-4-methoxycyclohexan-1-amine;
1-(4-(((6-(3-(2-(4-(((1-acetylpiperidin-4-yl)amino)methyl)-3-methoxyphenyl)-3-fluoropyridin-4-yl)-2-fluorophenyl)-2-methoxypyridin-3-yl)methyl)amino)piperidin-1-yl)ethan-1-one;
(1r,4r)-N-((6-(2-chloro-3-(3-chloro-2-(3-methoxy-4-((((1r,4r)-4-methoxycyclohexyl)amino)methyl)phenyl)pyridin-4-yl)phenyl)-2-methoxypyridin-3-yl)methyl)-4-methoxycyclohexan-1-amine;
(1r,4r)-N-((6-(2-chloro-3-(3-chloro-2-(3-methoxy-4-((((1r,4s)-4-methoxycyclohexyl)amino)methyl)phenyl)pyridin-4-yl)phenyl)-2-methoxypyridin-3-yl)methyl)-4-methoxycyclohexan-1-amine;
(1r,4r)-N-((6-(2-chloro-3-(3-chloro-2-(3-methoxy-4-((((1s,4r)-4-methoxycyclohexyl)amino)methyl)phenyl)pyridin-4-yl)phenyl)-2-methoxypyridin-3-yl)methyl)-4-methoxycyclohexan-1-amine;
(1r,4r)-N-((6-(2-chloro-3-(3-chloro-2-(3-methoxy-4-((((1s,4s)-4-methoxycyclohexyl)amino)methyl)phenyl)pyridin-4-yl)phenyl)-2-methoxypyridin-3-yl)methyl)-4-methoxycyclohexan-1-amine;
(1r,4s)-N-((6-(2-chloro-3-(3-chloro-2-(3-methoxy-4-((((1r,4r)-4-methoxycyclohexyl)amino)methyl)phenyl)pyridin-4-yl)phenyl)-2-methoxypyridin-3-yl)methyl)-4-methoxycyclohexan-1-amine;
(1r,4s)-N-((6-(2-chloro-3-(3-chloro-2-(3-methoxy-4-((((1r,4s)-4-methoxycyclohexyl)amino)methyl)phenyl)pyridin-4-yl)phenyl)-2-methoxypyridin-3-yl)methyl)-4-methoxycyclohexan-1-amine;
(1r,4s)-N-((6-(2-chloro-3-(3-chloro-2-(3-methoxy-4-((((1s,4r)-4-methoxycyclohexyl)amino)methyl)phenyl)pyridin-4-yl)phenyl)-2-methoxypyridin-3-yl)methyl)-4-methoxycyclohexan-1-amine;
(1r,4s)-N-((6-(2-chloro-3-(3-chloro-2-(3-methoxy-4-((((1s,4s)-4-methoxycyclohexyl)amino)methyl)phenyl)pyridin-4-yl)phenyl)-2-methoxypyridin-3-yl)methyl)-4-methoxycyclohexan-1-amine;
(1s,4r)-N-((6-(2-chloro-3-(3-chloro-2-(3-methoxy-4-((((1r,4r)-4-methoxycyclohexyl)amino)methyl)phenyl)pyridin-4-yl)phenyl)-2-methoxypyridin-3-yl)methyl)-4-methoxycyclohexan-1-amine;
(1s,4r)-N-((6-(2-chloro-3-(3-chloro-2-(3-methoxy-4-((((1r,4s)-4-methoxycyclohexyl)amino)methyl)phenyl)pyridin-4-yl)phenyl)-2-methoxypyridin-3-yl)methyl)-4-methoxycyclohexan-1-amine;
(1s,4r)-N-((6-(2-chloro-3-(3-chloro-2-(3-methoxy-4-((((1s,4r)-4-methoxycyclohexyl)amino)methyl)phenyl)pyridin-4-yl)phenyl)-2-methoxypyridin-3-yl)methyl)-4-methoxycyclohexan-1-amine;
(1s,4r)-N-((6-(2-chloro-3-(3-chloro-2-(3-methoxy-4-((((1s,4s)-4-methoxycyclohexyl)amino)methyl)phenyl)pyridin-4-yl)phenyl)-2-methoxypyridin-3-yl)methyl)-4-methoxycyclohexan-1-amine;
(1s,4s)-N-((6-(2-chloro-3-(3-chloro-2-(3-methoxy-4-((((1r,4r)-4-methoxycyclohexyl)amino)methyl)phenyl)pyridin-4-yl)phenyl)-2-methoxypyridin-3-yl)methyl)-4-methoxycyclohexan-1-amine;
(1s,4s)-N-((6-(2-chloro-3-(3-chloro-2-(3-methoxy-4-((((1r,4s)-4-methoxycyclohexyl)amino)methyl)phenyl)pyridin-4-yl)phenyl)-2-methoxypyridin-3-yl)methyl)-4-methoxycyclohexan-1-amine;
(1s,4s)-N-((6-(2-chloro-3-(3-chloro-2-(3-methoxy-4-((((1s,4r)-4-methoxycyclohexyl)amino)methyl)phenyl)pyridin-4-yl)phenyl)-2-methoxypyridin-3-yl)methyl)-4-methoxycyclohexan-1-amine;
(1s,4s)-N-((6-(2-chloro-3-(3-chloro-2-(3-methoxy-4-((((1s,4s)-4-methoxycyclohexyl)amino)methyl)phenyl)pyridin-4-yl)phenyl)-2-methoxypyridin-3-yl)methyl)-4-methoxycyclohexan-1-amine;
2-(4-(3-fluoro-4-(2-fluoro-3-(6-methoxy-5-((7-oxo-2,6-diazaspiro[3.4]octan-2-yl)methyl)pyridin-2-yl)phenyl)pyridin-2-yl)-2-methoxybenzyl)-2,6-diazaspiro[3.4]octan-7-one;
N-((6-(2-fluoro-3-(3-fluoro-2-(3-methoxy-4-(((tetrahydro-2H-pyran-4-yl)amino)methyl)phenyl)pyridin-4-yl)phenyl)-2-methoxypyridin-3-yl)methyl)tetrahydro-2H-pyran-4-amine;
(1r,4r)-4-(((6-(2-chloro-3-(3-chloro-2-(2-((((1r,4r)-4-hydroxycyclohexyl)amino)methyl)-8-methoxyimidazo[1,2-a]pyridin-6-yl)pyridin-4-yl)phenyl)-2-methoxypyridin-3-yl)methyl)amino)cyclohexan-1-ol;
(1r,4r)-4-(((6-(2-chloro-3-(3-chloro-2-(2-((((1r,4s)-4-hydroxycyclohexyl)amino)methyl)-8-methoxyimidazo[1,2-a]pyridin-6-yl)pyridin-4-yl)phenyl)-2-methoxypyridin-3-yl)methyl)amino)cyclohexan-1-ol;
(1r,4r)-4-(((6-(2-chloro-3-(3-chloro-2-(2-((((1s,4r)-4-hydroxycyclohexyl)amino)methyl)-8-methoxyimidazo[1,2-a]pyridin-6-yl)pyridin-4-yl)phenyl)-2-methoxypyridin-3-yl)methyl)amino)cyclohexan-1-ol;
(1r,4r)-4-(((6-(2-chloro-3-(3-chloro-2-(2-((((1s,4s)-4-hydroxycyclohexyl)amino)methyl)-8-methoxyimidazo[1,2-a]pyridin-6-yl)pyridin-4-yl)phenyl)-2-methoxypyridin-3-yl)methyl)amino)cyclohexan-1-ol;
(1r,4s)-4-(((6-(2-chloro-3-(3-chloro-2-(2-((((1r,4r)-4-hydroxycyclohexyl)amino)methyl)-8-methoxyimidazo[1,2-a]pyridin-6-yl)pyridin-4-yl)phenyl)-2-methoxypyridin-3-yl)methyl)amino)cyclohexan-1-ol;
(1r,4s)-4-(((6-(2-chloro-3-(3-chloro-2-(2-((((1r,4s)-4-hydroxycyclohexyl)amino)methyl)-8-methoxyimidazo[1,2-a]pyridin-6-yl)pyridin-4-yl)phenyl)-2-methoxypyridin-3-yl)methyl)amino)cyclohexan-1-ol;
(1r,4s)-4-(((6-(2-chloro-3-(3-chloro-2-(2-((((1s,4r)-4-hydroxycyclohexyl)amino)methyl)-8-methoxyimidazo[1,2-a]pyridin-6-yl)pyridin-4-yl)phenyl)-2-methoxypyridin-3-yl)methyl)amino)cyclohexan-1-ol;
(1r,4s)-4-(((6-(2-chloro-3-(3-chloro-2-(2-((((1s,4s)-4-hydroxycyclohexyl)amino)methyl)-8-methoxyimidazo[1,2-a]pyridin-6-yl)pyridin-4-yl)phenyl)-2-methoxypyridin-3-yl)methyl)amino)cyclohexan-1-ol;
(1s,4r)-4-(((6-(2-chloro-3-(3-chloro-2-(2-((((1r,4r)-4-hydroxycyclohexyl)amino)methyl)-8-methoxyimidazo[1,2-a]pyridin-6-yl)pyridin-4-yl)phenyl)-2-methoxypyridin-3-yl)methyl)amino)cyclohexan-1-ol;
(1s,4r)-4-(((6-(2-chloro-3-(3-chloro-2-(2-((((1r,4s)-4-hydroxycyclohexyl)amino)methyl)-8-methoxyimidazo[1,2-a]pyridin-6-yl)pyridin-4-yl)phenyl)-2-methoxypyridin-3-yl)methyl)amino)cyclohexan-1-ol;
(1s,4r)-4-(((6-(2-chloro-3-(3-chloro-2-(2-((((1s,4r)-4-hydroxycyclohexyl)amino)methyl)-8-methoxyimidazo[1,2-a]pyridin-6-yl)pyridin-4-yl)phenyl)-2-methoxypyridin-3-yl)methyl)amino)cyclohexan-1-ol;
(1s,4r)-4-(((6-(2-chloro-3-(3-chloro-2-(2-((((1s,4s)-4-hydroxycyclohexyl)amino)methyl)-8-methoxyimidazo[1,2-a]pyridin-6-yl)pyridin-4-yl)phenyl)-2-methoxypyridin-3-yl)methyl)amino)cyclohexan-1-ol;
(1s,4s)-4-(((6-(2-chloro-3-(3-chloro-2-(2-((((1r,4r)-4-hydroxycyclohexyl)amino)methyl)-8-methoxyimidazo[1,2-a]pyridin-6-yl)pyridin-4-yl)phenyl)-2-methoxypyridin-3-yl)methyl)amino)cyclohexan-1-ol;
(1s,4s)-4-(((6-(2-chloro-3-(3-chloro-2-(2-((((1r,4s)-4-hydroxycyclohexyl)amino)methyl)-8-methoxyimidazo[1,2-a]pyridin-6-yl)pyridin-4-yl)phenyl)-2-methoxypyridin-3-yl)methyl)amino)cyclohexan-1-ol;
(1s,4s)-4-(((6-(2-chloro-3-(3-chloro-2-(2-((((1s,4r)-4-hydroxycyclohexyl)amino)methyl)-8-methoxyimidazo[1,2-a]pyridin-6-yl)pyridin-4-yl)phenyl)-2-methoxypyridin-3-yl)methyl)amino)cyclohexan-1-ol;
(1s,4s)-4-(((6-(2-chloro-3-(3-chloro-2-(2-((((1s,4s)-4-hydroxycyclohexyl)amino)methyl)-8-methoxyimidazo[1,2-a]pyridin-6-yl)pyridin-4-yl)phenyl)-2-methoxypyridin-3-yl)methyl)amino)cyclohexan-1-ol;
2-((6-(3-chloro-4-(2-chloro-3-(6-methoxy-5-((6-oxo-2,5-diazaspiro[3.4]octan-2-yl)methyl)pyridin-2-yl)phenyl)pyridin-2-yl)-8-methoxyimidazo[1,2-a]pyridin-2-yl)methyl)-2,5-diazaspiro[3.4]octan-6-one;
1-((6-(2-chloro-3-(3-chloro-2-(2-((3-hydroxy-3-methylazetidin-1-yl)methyl)-8-methoxyimidazo[1,2-a]pyridin-6-yl)pyridin-4-yl)phenyl)-2-methoxypyridin-3-yl)methyl)-3-methylazetidin-3-ol;
4-((6-(2-chloro-3-(3-chloro-2-(3-methoxy-4-((3-oxopiperazin-1-yl)methyl)phenyl)pyridin-4-yl)phenyl)-2-methoxypyridin-3-yl)methyl)piperazin-2-one;
1-(4-(((6-(2-chloro-3-(3-chloro-2-(3-methoxy-4-(morpholinomethyl)phenyl)pyridin-4-yl)phenyl)-2-methoxypyridin-3-yl)methyl)amino)piperidin-1-yl)ethan-1-one;
1-(4-(((6-(2-chloro-3-(3-chloro-2-(4-((4-hydroxypiperidin-1-yl)methyl)-3-methoxyphenyl)pyridin-4-yl)phenyl)-2-methoxypyridin-3-yl)methyl)amino)piperidin-1-yl)ethan-1-one;
(S)-(2-(6-(3-chloro-4-(2-chloro-3-(6-methoxy-5-((((5-oxopyrrolidin-2-yl)methyl)amino)methyl)pyridin-2-yl)phenyl)pyridin-2-yl)-2-methoxy-4-oxopyrrolo[2,1-f][1,2,4]triazin-3(4H)-yl)ethyl)glycine;
(R)-(2-(6-(3-chloro-4-(2-chloro-3-(6-methoxy-5-((((5-oxopyrrolidin-2-yl)methyl)amino)methyl)pyridin-2-yl)phenyl)pyridin-2-yl)-2-methoxy-4-oxopyrrolo[2,1-f][1,2,4]triazin-3(4H)-yl)ethyl)glycine;
N-(1-((6-(3-(2-(4-((4-acetamidopiperidin-1-yl)methyl)-3-methoxyphenyl)-3-methylpyridin-4-yl)-2-methylphenyl)-2-methoxypyridin-3-yl)methyl)piperidin-4-yl)acetamide;
N-((6-(2-chloro-3-(2-(3-methoxy-4-(((tetrahydro-2H-pyran-4-yl)amino)methyl)phenyl)-3-methylpyridin-4-yl)phenyl)-2-methoxypyridin-3-yl)methyl)tetrahydro-2H-pyran-4-amine;
(1r,4r)-4-(((6-(2-chloro-3-(2-(4-((((1r,4r)-4-hydroxycyclohexyl)amino)methyl)-3-methoxyphenyl)-3-methylpyridin-4-yl)phenyl)-2-methoxypyridin-3-yl)methyl)amino)cyclohexan-1-ol;
(1r,4r)-4-(((6-(2-chloro-3-(2-(4-((((1r,4s)-4-hydroxycyclohexyl)amino)methyl)-3-methoxyphenyl)-3-methylpyridin-4-yl)phenyl)-2-methoxypyridin-3-yl)methyl)amino)cyclohexan-1-ol;
(1r,4r)-4-(((6-(2-chloro-3-(2-(4-((((1s,4r)-4-hydroxycyclohexyl)amino)methyl)-3-methoxyphenyl)-3-methylpyridin-4-yl)phenyl)-2-methoxypyridin-3-yl)methyl)amino)cyclohexan-1-ol;
(1r,4r)-4-(((6-(2-chloro-3-(2-(4-((((1s,4s)-4-hydroxycyclohexyl)amino)methyl)-3-methoxyphenyl)-3-methylpyridin-4-yl)phenyl)-2-methoxypyridin-3-yl)methyl)amino)cyclohexan-1-ol;
(1r,4s)-4-(((6-(2-chloro-3-(2-(4-((((1r,4r)-4-hydroxycyclohexyl)amino)methyl)-3-methoxyphenyl)-3-methylpyridin-4-yl)phenyl)-2-methoxypyridin-3-yl)methyl)amino)cyclohexan-1-ol;
(1r,4s)-4-(((6-(2-chloro-3-(2-(4-((((1r,4s)-4-hydroxycyclohexyl)amino)methyl)-3-methoxyphenyl)-3-methylpyridin-4-yl)phenyl)-2-methoxypyridin-3-yl)methyl)amino)cyclohexan-1-ol;
(1r,4s)-4-(((6-(2-chloro-3-(2-(4-((((1s,4r)-4-hydroxycyclohexyl)amino)methyl)-3-methoxyphenyl)-3-methylpyridin-4-yl)phenyl)-2-methoxypyridin-3-yl)methyl)amino)cyclohexan-1-ol;
(1r,4s)-4-(((6-(2-chloro-3-(2-(4-((((1s,4s)-4-hydroxycyclohexyl)amino)methyl)-3-methoxyphenyl)-3-methylpyridin-4-yl)phenyl)-2-methoxypyridin-3-yl)methyl)amino)cyclohexan-1-ol;
(1s,4r)-4-(((6-(2-chloro-3-(2-(4-((((1r,4r)-4-hydroxycyclohexyl)amino)methyl)-3-methoxyphenyl)-3-methylpyridin-4-yl)phenyl)-2-methoxypyridin-3-yl)methyl)amino)cyclohexan-1-ol;
(1s,4r)-4-(((6-(2-chloro-3-(2-(4-((((1r,4s)-4-hydroxycyclohexyl)amino)methyl)-3-methoxyphenyl)-3-methylpyridin-4-yl)phenyl)-2-methoxypyridin-3-yl)methyl)amino)cyclohexan-1-ol;
(1s,4r)-4-(((6-(2-chloro-3-(2-(4-((((1s,4r)-4-hydroxycyclohexyl)amino)methyl)-3-methoxyphenyl)-3-methylpyridin-4-yl)phenyl)-2-methoxypyridin-3-yl)methyl)amino)cyclohexan-1-ol;
(1s,4r)-4-(((6-(2-chloro-3-(2-(4-((((1s,4s)-4-hydroxycyclohexyl)amino)methyl)-3-methoxyphenyl)-3-methylpyridin-4-yl)phenyl)-2-methoxypyridin-3-yl)methyl)amino)cyclohexan-1-ol;
(1s,4s)-4-(((6-(2-chloro-3-(2-(4-((((1r,4r)-4-hydroxycyclohexyl)amino)methyl)-3-methoxyphenyl)-3-methylpyridin-4-yl)phenyl)-2-methoxypyridin-3-yl)methyl)amino)cyclohexan-1-ol;
(1s,4s)-4-(((6-(2-chloro-3-(2-(4-((((1r,4s)-4-hydroxycyclohexyl)amino)methyl)-3-methoxyphenyl)-3-methylpyridin-4-yl)phenyl)-2-methoxypyridin-3-yl)methyl)amino)cyclohexan-1-ol;
(1s,4s)-4-(((6-(2-chloro-3-(2-(4-((((1s,4r)-4-hydroxycyclohexyl)amino)methyl)-3-methoxyphenyl)-3-methylpyridin-4-yl)phenyl)-2-methoxypyridin-3-yl)methyl)amino)cyclohexan-1-ol;
(1s,4s)-4-(((6-(2-chloro-3-(2-(4-((((1s,4s)-4-hydroxycyclohexyl)amino)methyl)-3-methoxyphenyl)-3-methylpyridin-4-yl)phenyl)-2-methoxypyridin-3-yl)methyl)amino)cyclohexan-1-ol;
6-(4-(4-(3-(5-((2-oxa-6-azaspiro[3.3]heptan-6-yl)methyl)-6-methoxypyridin-2-yl)-2-chlorophenyl)-3-methylpyridin-2-yl)-2-methoxybenzyl)-2-oxa-6-azaspiro[3.3]heptane;
methyl 4-(((6-(2-chloro-3-(3-chloro-2-(3-methoxy-4-(((1-(methoxycarbonyl)piperidin-4-yl)amino)methyl)phenyl)pyridin-4-yl)phenyl)-2-methoxypyridin-3-yl)methyl)amino)piperidine-1-carboxylate;
N-(1-((6-(3-(2-(4-((4-acetamidopiperidin-1-yl)methyl)-3-methoxyphenyl)-3-methylpyridin-4-yl)-2-chlorophenyl)-2-methoxypyridin-3-yl)methyl)piperidin-4-yl)acetamide;
N-(1-((6-(3-(2-(4-((4-acetamidopiperidin-1-yl)methyl)-3-methoxyphenyl)-3-chloropyridin-4-yl)-2-methylphenyl)-2-methoxypyridin-3-yl)methyl)piperidin-4-yl)acetamide;
N-((6-(2-chloro-3-(3-chloro-2-(3-methoxy-4-(((tetrahydro-2H-pyran-4-yl)amino)methyl)phenyl)pyridin-4-yl)phenyl)-2-methoxypyridin-3-yl)methyl)-N-methyltetrahydro-2H-pyran-4-amine;
1-(4-(((6-(2-chloro-3-(3-chloro-2-(3-methoxy-4-(((tetrahydro-2H-pyran-4-yl)amino)methyl)phenyl)pyridin-4-yl)phenyl)-2-methoxypyridin-3-yl)methyl)(methyl)amino)piperidin-1-yl)ethan-1-one;
2-(4-(4-(3-(5-((7-oxa-2-azaspiro[3.5]nonan-2-yl)methyl)-6-methoxypyridin-2-yl)-2-chlorophenyl)-3-chloropyridin-2-yl)-2-methoxybenzyl)-7-oxa-2-azaspiro[3.5]nonane;
2-(1-((6-(2-chloro-3-(3-chloro-2-(4-((3-(2-hydroxypropan-2-yl)azetidin-1-yl)methyl)-3-methoxyphenyl)pyridin-4-yl)phenyl)-2-methoxypyridin-3-yl)methyl)azetidin-3-yl)propan-2-ol;
(S)-5-((((6-(2-chloro-3-(3-chloro-2-(3-methoxy-4-((methyl(((S)-5-oxopyrrolidin-2-yl)methyl)amino)methyl)phenyl)pyridin-4-yl)phenyl)-2-methoxypyridin-3-yl)methyl)(methyl)amino)methyl)pyrrolidin-2-one;
(S)-5-((((6-(2-chloro-3-(3-chloro-2-(3-methoxy-4-((methyl(((R)-5-oxopyrrolidin-2-yl)methyl)amino)methyl)phenyl)pyridin-4-yl)phenyl)-2-methoxypyridin-3-yl)methyl)(methyl)amino)methyl)pyrrolidin-2-one;
(R)-5-((((6-(2-chloro-3-(3-chloro-2-(3-methoxy-4-((methyl(((S)-5-oxopyrrolidin-2-yl)methyl)amino)methyl)phenyl)pyridin-4-yl)phenyl)-2-methoxypyridin-3-yl)methyl)(methyl)amino)methyl)pyrrolidin-2-one;
(R)-5-((((6-(2-chloro-3-(3-chloro-2-(3-methoxy-4-((methyl(((R)-5-oxopyrrolidin-2-yl)methyl)amino)methyl)phenyl)pyridin-4-yl)phenyl)-2-methoxypyridin-3-yl)methyl)(methyl)amino)methyl)pyrrolidin-2-one;
(R)-4-((((6-(2-chloro-3-(3-chloro-2-(3-methoxy-4-((methyl(((R)-5-oxopyrrolidin-3-yl)methyl)amino)methyl)phenyl)pyridin-4-yl)phenyl)-2-methoxypyridin-3-yl)methyl)(methyl)amino)methyl)pyrrolidin-2-one;
(S)-4-((((6-(2-chloro-3-(3-chloro-2-(3-methoxy-4-((methyl(((R)-5-oxopyrrolidin-3-yl)methyl)amino)methyl)phenyl)pyridin-4-yl)phenyl)-2-methoxypyridin-3-yl)methyl)(methyl)amino)methyl)pyrrolidin-2-one;
(R)-4-((((6-(2-chloro-3-(3-chloro-2-(3-methoxy-4-((methyl(((S)-5-oxopyrrolidin-3-yl)methyl)amino)methyl)phenyl)pyridin-4-yl)phenyl)-2-methoxypyridin-3-yl)methyl)(methyl)amino)methyl)pyrrolidin-2-one;
(S)-4-((((6-(2-chloro-3-(3-chloro-2-(3-methoxy-4-((methyl(((S)-5-oxopyrrolidin-3-yl)methyl)amino)methyl)phenyl)pyridin-4-yl)phenyl)-2-methoxypyridin-3-yl)methyl)(methyl)amino)methyl)pyrrolidin-2-one;
2-((6-(3-chloro-4-(2-chloro-3-(5-((6-hydroxy-2-azaspiro[3.3]heptan-2-yl)methyl)-6-methoxypyridin-2-yl)phenyl)pyridin-2-yl)-8-methoxyimidazo[1,2-a]pyridin-2-yl)methyl)-2-azaspiro[3.3]heptan-6-ol;
1-(4-(((6-(2-chloro-3-(3-chloro-2-(8-methoxy-2-(morpholinomethyl)imidazo[1,2-a]pyridin-6-yl)pyridin-4-yl)phenyl)-2-methoxypyridin-3-yl)methyl)amino)piperidin-1-yl)ethan-1-one;
1-(4-(((6-(2-chloro-3-(3-chloro-2-(2-((3,3-difluoropyrrolidin-1-yl)methyl)-8-methoxyimidazo[1,2-a]pyridin-6-yl)pyridin-4-yl)phenyl)-2-methoxypyridin-3-yl)methyl)amino)piperidin-1-yl)ethan-1-one;
1-(4-(((6-(2-chloro-3-(3-chloro-2-(2-(((2,2-difluoroethyl)amino)methyl)-8-methoxyimidazo[1,2-a]pyridin-6-yl)pyridin-4-yl)phenyl)-2-methoxypyridin-3-yl)methyl)amino)piperidin-1-yl)ethan-1-one;
4-(2-((4-(4-(3-(5-(((1-acetylpiperidin-4-yl)amino)methyl)-6-methoxypyridin-2-yl)-2-chlorophenyl)-3-chloropyridin-2-yl)-2-methoxybenzyl)amino)ethyl)piperazin-2-one;
2-(3-(6-(4-(3-(5-(((1-acetylpiperidin-4-yl)amino)methyl)-6-methoxypyridin-2-yl)-2-chlorophenyl)-3-chloropyridin-2-yl)-8-methoxy-3,4-dihydroisoquinolin-2(1H)-yl)-2-oxopyrrolidin-1-yl)acetic acid;
1-(4-(((6-(3-(2-(4-(((1-acetylpiperidin-4-yl)amino)methyl)-3-methoxyphenyl)-3-chloropyridin-4-yl)-2-chlorophenyl)-2-methoxypyridin-3-yl)methyl)amino)piperidin-1-yl)-2-methoxyethan-1-one;
1-(4-(((6-(3-(2-(4-((6-acetyl-2,6-diazaspiro[3.3]heptan-2-yl)methyl)-3-methoxyphenyl)-3-chloropyridin-4-yl)-2-chlorophenyl)-2-methoxypyridin-3-yl)methyl)amino)piperidin-1-yl)ethan-1-one;
1-(4-((4-(4-(3-(5-((6-acetyl-2,6-diazaspiro[3.3]heptan-2-yl)methyl)-6-methoxypyridin-2-yl)-2-chlorophenyl)-3-chloropyridin-2-yl)-2-methoxybenzyl)amino)piperidin-1-yl)ethan-1-one;
N-((6-(2-chloro-3-(3-chloro-2-(3-methoxy-4-(((tetrahydro-2H-thiopyran-4-yl)amino)methyl)phenyl)pyridin-4-yl)phenyl)-2-methoxypyridin-3-yl)methyl)tetrahydro-2H-thiopyran-4-amine;
1-(2-((6-(3-(2-(4-((7-acetyl-2,7-diazaspiro[3.5]nonan-2-yl)methyl)-3-methoxyphenyl)-3-chloropyridin-4-yl)-2-chlorophenyl)-2-methoxypyridin-3-yl)methyl)-2,7-diazaspiro[3.5]nonan-7-yl)ethan-1-one;
(S)-5-((((6-(((S)-4-(5-(((1-acetylpiperidin-4-yl)amino)methyl)-3'-chloro-6-methoxy-[2,4'-bipyridin]-2'-yl)-2,3-dihydro-1H-inden-1-yl)amino)-2-methoxy-5-(trifluoromethyl)pyridin-3-yl)methyl)amino)methyl)pyrrolidin-2-one;
(S)-5-((((6-(((R)-4-(5-(((1-acetylpiperidin-4-yl)amino)methyl)-3'-chloro-6-methoxy-[2,4'-bipyridin]-2'-yl)-2,3-dihydro-1H-inden-1-yl)amino)-2-methoxy-5-(trifluoromethyl)pyridin-3-yl)methyl)amino)methyl)pyrrolidin-2-one;
(R)-5-((((6-(((S)-4-(5-(((1-acetylpiperidin-4-yl)amino)methyl)-3'-chloro-6-methoxy-[2,4'-bipyridin]-2'-yl)-2,3-dihydro-1H-inden-1-yl)amino)-2-methoxy-5-(trifluoromethyl)pyridin-3-yl)methyl)amino)methyl)pyrrolidin-2-one;
(R)-5-((((6-(((R)-4-(5-(((1-acetylpiperidin-4-yl)amino)methyl)-3'-chloro-6-methoxy-[2,4'-bipyridin]-2'-yl)-2,3-dihydro-1H-inden-1-yl)amino)-2-methoxy-5-(trifluoromethyl)pyridin-3-yl)methyl)amino)methyl)pyrrolidin-2-one;
1-(4-((4-(3-chloro-4-(2-chloro-3-(5-((4-hydroxypiperidin-1-yl)methyl)-6-methoxypyridin-2-yl)phenyl)pyridin-2-yl)-2-methoxybenzyl)amino)piperidin-1-yl)ethan-1-one;
1-(4-((4-(3-chloro-4-(2-chloro-3-(6-methoxy-5-((((1r,4r)-4-methoxycyclohexyl)amino)methyl)pyridin-2-yl)phenyl)pyridin-2-yl)-2-methoxybenzyl)amino)piperidin-1-yl)ethan-1-one;
1-(4-((4-(3-chloro-4-(2-chloro-3-(6-methoxy-5-((((1s,4r)-4-methoxycyclohexyl)amino)methyl)pyridin-2-yl)phenyl)pyridin-2-yl)-2-methoxybenzyl)amino)piperidin-1-yl)ethan-1-one;
1-(4-((4-(3-chloro-4-(2-chloro-3-(6-methoxy-5-((((1r,4s)-4-methoxycyclohexyl)amino)methyl)pyridin-2-yl)phenyl)pyridin-2-yl)-2-methoxybenzyl)amino)piperidin-1-yl)ethan-1-one;
1-(4-((4-(3-chloro-4-(2-chloro-3-(6-methoxy-5-((((1s,4s)-4-methoxycyclohexyl)amino)methyl)pyridin-2-yl)phenyl)pyridin-2-yl)-2-methoxybenzyl)amino)piperidin-1-yl)ethan-1-one;
(R)-1-(4-(((6-(2-chloro-3-(3-chloro-2-(4-((3-hydroxypiperidin-1-yl)methyl)-3-methoxyphenyl)pyridin-4-yl)phenyl)-2-methoxypyridin-3-yl)methyl)amino)piperidin-1-yl)ethan-1-one;
(S)-1-(4-(((6-(2-chloro-3-(3-chloro-2-(4-((3-hydroxypiperidin-1-yl)methyl)-3-methoxyphenyl)pyridin-4-yl)phenyl)-2-methoxypyridin-3-yl)methyl)amino)piperidin-1-yl)ethan-1-one;
2-(4-(3-chloro-4-(2-chloro-3-(6-methoxy-5-(((1-(2-methoxyacetyl)piperidin-4-yl)amino)methyl)pyridin-2-yl)phenyl)pyridin-2-yl)-2-methoxybenzyl)-2,6-diazaspiro[3.4]octan-7-one;
1-(4-(((6-(3-(2-(4-((6-acetyl-2,6-diazaspiro[3.3]heptan-2-yl)methyl)-3-methoxyphenyl)-3-chloropyridin-4-yl)-2-chlorophenyl)-2-methoxypyridin-3-yl)methyl)amino)piperidin-1-yl)-2-methoxyethan-1-one;
1-(6-(4-(3-chloro-4-(2-chloro-3-(6-methoxy-5-(((tetrahydro-2H-pyran-4-yl)amino)methyl)pyridin-2-yl)phenyl)pyridin-2-yl)-2-methoxybenzyl)-2,6-diazaspiro[3.3]heptan-2-yl)ethan-1-one;
2-((6-(3-(2-(4-((6-acetyl-2,6-diazaspiro[3.3]heptan-2-yl)methyl)-3-methoxyphenyl)-3-chloropyridin-4-yl)-2-chlorophenyl)-2-methoxypyridin-3-yl)methyl)-2,6-diazaspiro[3.4]octan-7-one;
2-((6-(3-(2-(4-((2,6-diazaspiro[3.3]heptan-2-yl)methyl)-3-methoxyphenyl)-3-chloropyridin-4-yl)-2-chlorophenyl)-2-methoxypyridin-3-yl)methyl)-2,6-diazaspiro[3.4]octan-7-one;
2-((6-(2-chloro-3-(3-chloro-2-(3-methoxy-4-(((tetrahydro-2H-pyran-4-yl)amino)methyl)phenyl)pyridin-4-yl)phenyl)-2-methoxypyridin-3-yl)methyl)-2,6-diazaspiro[3.4]octan-7-one;
2-((6-(2-chloro-3-(3-chloro-2-(4-((((1r,3r)-3-hydroxy-3-methylcyclobutyl)amino)methyl)-3-methoxyphenyl)pyridin-4-yl)phenyl)-2-methoxypyridin-3-yl)methyl)-2,6-diazaspiro[3.4]octan-7-one;
2-((6-(2-chloro-3-(3-chloro-2-(4-((((1s,3r)-3-hydroxy-3-methylcyclobutyl)amino)methyl)-3-methoxyphenyl)pyridin-4-yl)phenyl)-2-methoxypyridin-3-yl)methyl)-2,6-diazaspiro[3.4]octan-7-one;
2-((6-(2-chloro-3-(3-chloro-2-(4-((((1r,3s)-3-hydroxy-3-methylcyclobutyl)amino)methyl)-3-methoxyphenyl)pyridin-4-yl)phenyl)-2-methoxypyridin-3-yl)methyl)-2,6-diazaspiro[3.4]octan-7-one;
2-((6-(2-chloro-3-(3-chloro-2-(4-((((1s,3s)-3-hydroxy-3-methylcyclobutyl)amino)methyl)-3-methoxyphenyl)pyridin-4-yl)phenyl)-2-methoxypyridin-3-yl)methyl)-2,6-diazaspiro[3.4]octan-7-one;
2-((6-(2-chloro-3-(3-chloro-2-(4-((6-hydroxy-2-azaspiro[3.3]heptan-2-yl)methyl)-3-methoxyphenyl)pyridin-4-yl)phenyl)-2-methoxypyridin-3-yl)methyl)-2,6-diazaspiro[3.4]octan-7-one;
2-((6-(2-chloro-3-(3-chloro-2-(4-((3-hydroxy-3-methylazetidin-1-yl)methyl)-3-methoxyphenyl)pyridin-4-yl)phenyl)-2-methoxypyridin-3-yl)methyl)-2,6-diazaspiro[3.4]octan-7-one;
1-(3-((((6-(3-(2-(4-((((1-acetylazetidin-3-yl)methyl)(methyl)amino)methyl)-3-methoxyphenyl)-3-chloropyridin-4-yl)-2-chlorophenyl)-2-methoxypyridin-3-yl)methyl)(methyl)amino)methyl)azetidin-1-yl)ethan-1-one;
1-(4-(((4-(4'-(((1-acetylpiperidin-4-yl)amino)methyl)-2-chloro-3'-methoxy-[1,1'-biphenyl]-3-yl)-3-chloro-5'-methoxy-[2,3'-bipyridin]-6'-yl)methyl)amino)piperidin-1-yl)ethan-1-one;
2-((2'-chloro-3'-(3-chloro-5'-methoxy-6'-((7-oxo-2,6-diazaspiro[3.4]octan-2-yl)methyl)-[2,3'-bipyridin]-4-yl)-3-methoxy-[1,1'-biphenyl]-4-yl)methyl)-2,6-diazaspiro[3.4]octan-7-one;
2-((6-(2-chloro-3-(3-chloro-2-(4-((((1r,4r)-4-hydroxycyclohexyl)amino)methyl)-3-methoxyphenyl)pyridin-4-yl)phenyl)-2-methoxypyridin-3-yl)methyl)-2,6-diazaspiro[3.4]octan-7-one;
2-((6-(2-chloro-3-(3-chloro-2-(4-((((1s,4r)-4-hydroxycyclohexyl)amino)methyl)-3-methoxyphenyl)pyridin-4-yl)phenyl)-2-methoxypyridin-3-yl)methyl)-2,6-diazaspiro[3.4]octan-7-one;
2-((6-(2-chloro-3-(3-chloro-2-(4-((((1r,4s)-4-hydroxycyclohexyl)amino)methyl)-3-methoxyphenyl)pyridin-4-yl)phenyl)-2-methoxypyridin-3-yl)methyl)-2,6-diazaspiro[3.4]octan-7-one;
2-((6-(2-chloro-3-(3-chloro-2-(4-((((1s,4s)-4-hydroxycyclohexyl)amino)methyl)-3-methoxyphenyl)pyridin-4-yl)phenyl)-2-methoxypyridin-3-yl)methyl)-2,6-diazaspiro[3.4]octan-7-one;
1-((((6-(2-chloro-3-(3-chloro-2-(4-((((1-hydroxycyclopropyl)methyl)amino)methyl)-3-methoxyphenyl)pyridin-4-yl)phenyl)-2-methoxypyridin-3-yl)methyl)amino)methyl)cyclopropan-1-ol;
N-((6-(2-chloro-3-(3-chloro-2-(4-(((2,2-difluoroethyl)amino)methyl)-3-methoxyphenyl)pyridin-4-yl)phenyl)-2-methoxypyridin-3-yl)methyl)-2,2-difluoroethan-1-amine;
2-(4-(3-chloro-4-(2-chloro-3-(6-methoxy-5-(((tetrahydro-2H-pyran-4-yl)amino)methyl)pyridin-2-yl)phenyl)pyridin-2-yl)-2-methoxybenzyl)-2,6-diazaspiro[3.4]octan-7-one;
(S)-1-(4-(((6-(2-chloro-3-(3-chloro-2-(4-((3-hydroxypiperidin-1-yl)methyl)-3-methoxyphenyl)pyridin-4-yl)phenyl)-2-methoxypyridin-3-yl)methyl)amino)piperidin-1-yl)ethan-1-one;
(R)-1-(4-(((6-(2-chloro-3-(3-chloro-2-(4-((3-hydroxypiperidin-1-yl)methyl)-3-methoxyphenyl)pyridin-4-yl)phenyl)-2-methoxypyridin-3-yl)methyl)amino)piperidin-1-yl)ethan-1-one;
1-(4-(((6-(2-chloro-3-(3-chloro-2-(4-((4-(hydroxymethyl)piperidin-1-yl)methyl)-3-methoxyphenyl)pyridin-4-yl)phenyl)-2-methoxypyridin-3-yl)methyl)amino)piperidin-1-yl)ethan-1-one;
1-(4-(((6-(2-chloro-3-(3-chloro-2-(3-methoxy-4-((4-(methoxymethyl)piperidin-1-yl)methyl)phenyl)pyridin-4-yl)phenyl)-2-methoxypyridin-3-yl)methyl)amino)piperidin-1-yl)ethan-1-one;
1-(4-(((6-(2-chloro-3-(3-chloro-2-(4-((4-hydroxypiperidin-1-yl)methyl)-3-methoxyphenyl)pyridin-4-yl)phenyl)-2-methoxypyridin-3-yl)methyl)amino)piperidin-1-yl)-2-methoxyethan-1-one;
1-(6-((6-(2-chloro-3-(3-chloro-2-(3-methoxy-4-(((tetrahydro-2H-pyran-4-yl)amino)methyl)phenyl)pyridin-4-yl)phenyl)-2-methoxypyridin-3-yl)methyl)-2,6-diazaspiro[3.3]heptan-2-yl)ethan-1-one;
1-(4-(((6-(2-chloro-3-(3-chloro-2-(4-((((1-hydroxycyclopropyl)methyl)amino)methyl)-3-methoxyphenyl)pyridin-4-yl)phenyl)-2-methoxypyridin-3-yl)methyl)amino)piperidin-1-yl)ethan-1-one;
1-(4-(((6-(2-chloro-3-(3-chloro-2-(4-(((2,2-difluoroethyl)amino)methyl)-3-methoxyphenyl)pyridin-4-yl)phenyl)-2-methoxypyridin-3-yl)methyl)amino)piperidin-1-yl)ethan-1-one;
N-((6-(2-chloro-3-(3-chloro-2-(4-((isopropylamino)methyl)-3-methoxyphenyl)pyridin-4-yl)phenyl)-2-methoxypyridin-3-yl)methyl)propan-2-amine;
2-(4-(3-chloro-4-(2-chloro-3-(5-((((1r,4r)-4-hydroxycyclohexyl)amino)methyl)-6-methoxypyridin-2-yl)phenyl)pyridin-2-yl)-2-methoxybenzyl)-2,6-diazaspiro[3.4]octan-7-one;
2-(4-(3-chloro-4-(2-chloro-3-(5-((((1s,4r)-4-hydroxycyclohexyl)amino)methyl)-6-methoxypyridin-2-yl)phenyl)pyridin-2-yl)-2-methoxybenzyl)-2,6-diazaspiro[3.4]octan-7-one;
2-(4-(3-chloro-4-(2-chloro-3-(5-((((1r,4s)-4-hydroxycyclohexyl)amino)methyl)-6-methoxypyridin-2-yl)phenyl)pyridin-2-yl)-2-methoxybenzyl)-2,6-diazaspiro[3.4]octan-7-one;
2-(4-(3-chloro-4-(2-chloro-3-(5-((((1s,4s)-4-hydroxycyclohexyl)amino)methyl)-6-methoxypyridin-2-yl)phenyl)pyridin-2-yl)-2-methoxybenzyl)-2,6-diazaspiro[3.4]octan-7-one;
2-(4-(3-chloro-4-(2-chloro-3-(5-((((1s,4s)-4-hydroxycyclohexyl)amino)methyl)-6-methoxypyridin-2-yl)phenyl)pyridin-2-yl)-2-methoxybenzyl)-2,6-diazaspiro[3.4]octan-7-one;
2-(4-(3-chloro-4-(2-chloro-3-(5-((((1s,4r)-4-hydroxycyclohexyl)amino)methyl)-6-methoxypyridin-2-yl)phenyl)pyridin-2-yl)-2-methoxybenzyl)-2,6-diazaspiro[3.4]octan-7-one;
2-(4-(3-chloro-4-(2-chloro-3-(5-((((1r,4s)-4-hydroxycyclohexyl)amino)methyl)-6-methoxypyridin-2-yl)phenyl)pyridin-2-yl)-2-methoxybenzyl)-2,6-diazaspiro[3.4]octan-7-one;
2-(4-(3-chloro-4-(2-chloro-3-(5-((((1r,4r)-4-hydroxycyclohexyl)amino)methyl)-6-methoxypyridin-2-yl)phenyl)pyridin-2-yl)-2-methoxybenzyl)-2,6-diazaspiro[3.4]octan-7-one;
2-(4-(3-chloro-4-(2-chloro-3-(5-((((1r,3r)-3-hydroxy-3-methylcyclobutyl)amino)methyl)-6-methoxypyridin-2-yl)phenyl)pyridin-2-yl)-2-methoxybenzyl)-2,6-diazaspiro[3.4]octan-7-one;
2-(4-(3-chloro-4-(2-chloro-3-(5-((((1s,3r)-3-hydroxy-3-methylcyclobutyl)amino)methyl)-6-methoxypyridin-2-yl)phenyl)pyridin-2-yl)-2-methoxybenzyl)-2,6-diazaspiro[3.4]octan-7-one;
2-(4-(3-chloro-4-(2-chloro-3-(5-((((1r,3s)-3-hydroxy-3-methylcyclobutyl)amino)methyl)-6-methoxypyridin-2-yl)phenyl)pyridin-2-yl)-2-methoxybenzyl)-2,6-diazaspiro[3.4]octan-7-one;
2-(4-(3-chloro-4-(2-chloro-3-(5-((((1s,3s)-3-hydroxy-3-methylcyclobutyl)amino)methyl)-6-methoxypyridin-2-yl)phenyl)pyridin-2-yl)-2-methoxybenzyl)-2,6-diazaspiro[3.4]octan-7-one;
2-(4-(3-chloro-4-(2-chloro-3-(5-((3-hydroxy-3-methylazetidin-1-yl)methyl)-6-methoxypyridin-2-yl)phenyl)pyridin-2-yl)-2-methoxybenzyl)-2,6-diazaspiro[3.4]octan-7-one;
2-(4-(4-(3-(5-((6-acetyl-2,6-diazaspiro[3.3]heptan-2-yl)methyl)-6-methoxypyridin-2-yl)-2-chlorophenyl)-3-chloropyridin-2-yl)-2-methoxybenzyl)-2,6-diazaspiro[3.4]octan-7-one;
2-(4-(4-(3-(5-((2,6-diazaspiro[3.3]heptan-2-yl)methyl)-6-methoxypyridin-2-yl)-2-chlorophenyl)-3-chloropyridin-2-yl)-2-methoxybenzyl)-2,6-diazaspiro[3.4]octan-7-one;
2-(4-(3-chloro-4-(2-chloro-3-(5-((6-hydroxy-2-azaspiro[3.3]heptan-2-yl)methyl)-6-methoxypyridin-2-yl)phenyl)pyridin-2-yl)-2-methoxybenzyl)-2,6-diazaspiro[3.4]octan-7-one;
1-(4-(((6-(3-(6-(4-(((1-acetylpiperidin-4-yl)amino)methyl)-3-methoxyphenyl)-5-methylpyrimidin-4-yl)-2-chlorophenyl)-2-methoxypyridin-3-yl)methyl)amino)piperidin-1-yl)ethan-1-one;
(S)-5-((((6-(2-chloro-3-(6-(3-methoxy-4-(((((S)-5-oxopyrrolidin-2-yl)methyl)amino)methyl)phenyl)-5-methylpyrimidin-4-yl)phenyl)-2-methoxypyridin-3-yl)methyl)amino)methyl)pyrrolidin-2-one;
(S)-5-((((6-(2-chloro-3-(6-(3-methoxy-4-(((((R)-5-oxopyrrolidin-2-yl)methyl)amino)methyl)phenyl)-5-methylpyrimidin-4-yl)phenyl)-2-methoxypyridin-3-yl)methyl)amino)methyl)pyrrolidin-2-one;
(R)-5-((((6-(2-chloro-3-(6-(3-methoxy-4-(((((S)-5-oxopyrrolidin-2-yl)methyl)amino)methyl)phenyl)-5-methylpyrimidin-4-yl)phenyl)-2-methoxypyridin-3-yl)methyl)amino)methyl)pyrrolidin-2-one;
(R)-5-((((6-(2-chloro-3-(6-(3-methoxy-4-(((((R)-5-oxopyrrolidin-2-yl)methyl)amino)methyl)phenyl)-5-methylpyrimidin-4-yl)phenyl)-2-methoxypyridin-3-yl)methyl)amino)methyl)pyrrolidin-2-one;
N-((6-(2-chloro-3-(3-chloro-2-(4-((cyclohexylamino)methyl)-3-methoxyphenyl)pyridin-4-yl)phenyl)-2-methoxypyridin-3-yl)methyl)cyclohexanamine;
2-((6-(3-(2-(4-(((1-acetylpiperidin-4-yl)amino)methyl)-3-methoxyphenyl)-3-chloropyridin-4-yl)-2-chlorophenyl)-2-methoxypyridin-3-yl)methyl)-2,6-diazaspiro[3.4]octan-5-one;
2-((6-(3-(2-(4-(((1-acetylpiperidin-4-yl)amino)methyl)-3-methoxyphenyl)-3-chloropyridin-4-yl)-2-chlorophenyl)-2-methoxypyridin-3-yl)methyl)-2,5,7-triazaspiro[3.4]octan-6-one;
(S)-5-((((2'-((S)-1-((5-(((1-acetylpiperidin-4-yl)amino)methyl)-6-methoxy-3-(trifluoromethyl)pyridin-2-yl)oxy)-2,3-dihydro-1H-inden-4-yl)-3'-chloro-6-methoxy-[2,4'-bipyridin]-5-yl)methyl)amino)methyl)pyrrolidin-2-one;
(S)-5-((((2'-((R)-1-((5-(((1-acetylpiperidin-4-yl)amino)methyl)-6-methoxy-3-(trifluoromethyl)pyridin-2-yl)oxy)-2,3-dihydro-1H-inden-4-yl)-3'-chloro-6-methoxy-[2,4'-bipyridin]-5-yl)methyl)amino)methyl)pyrrolidin-2-one;
(R)-5-((((2'-((S)-1-((5-(((1-acetylpiperidin-4-yl)amino)methyl)-6-methoxy-3-(trifluoromethyl)pyridin-2-yl)oxy)-2,3-dihydro-1H-inden-4-yl)-3'-chloro-6-methoxy-[2,4'-bipyridin]-5-yl)methyl)amino)methyl)pyrrolidin-2-one;
(R)-5-((((2'-((R)-1-((5-(((1-acetylpiperidin-4-yl)amino)methyl)-6-methoxy-3-(trifluoromethyl)pyridin-2-yl)oxy)-2,3-dihydro-1H-inden-4-yl)-3'-chloro-6-methoxy-[2,4'-bipyridin]-5-yl)methyl)amino)methyl)pyrrolidin-2-one;
1-(4-(((6-(3-(2-(4-(((1-acetylpiperidin-4-yl)amino)methyl)-3-methoxyphenyl)-3-chloropyridin-4-yl)-2-(trifluoromethyl)phenyl)-2-methoxypyridin-3-yl)methyl)amino)piperidin-1-yl)ethan-1-one;
N-((6-(3-(3-chloro-2-(3-methoxy-4-(((tetrahydro-2H-pyran-4-yl)amino)methyl)phenyl)pyridin-4-yl)-2-(trifluoromethyl)phenyl)-2-methoxypyridin-3-yl)methyl)tetrahydro-2H-pyran-4-amine;
N-((6-(3-(2-(4-((7-oxa-2-azaspiro[3.5]nonan-2-yl)methyl)-3-methoxyphenyl)-3-chloropyridin-4-yl)-2-chlorophenyl)-2-methoxypyridin-3-yl)methyl)tetrahydro-2H-pyran-4-amine;
N-(4-(4-(3-(5-((7-oxa-2-azaspiro[3.5]nonan-2-yl)methyl)-6-methoxypyridin-2-yl)-2-chlorophenyl)-3-chloropyridin-2-yl)-2-methoxybenzyl)tetrahydro-2H-pyran-4-amine;
(S)-5-((((6-(3-chloro-2-((S)-1-((6-methoxy-5-(((((S)-5-oxopyrrolidin-2-yl)methyl)amino)methyl)-3-(trifluoromethyl)pyrazin-2-yl)amino)-2,3-dihydro-1H-inden-4-yl)pyridin-4-yl)-1-methyl-1H-pyrrolo[2,3-b]pyridin-3-yl)methyl)amino)methyl)pyrrolidin-2-one;
(R)-5-((((6-(3-chloro-2-((R)-1-((6-methoxy-5-(((((R)-5-oxopyrrolidin-2-yl)methyl)amino)methyl)-3-(trifluoromethyl)pyrazin-2-yl)amino)-2,3-dihydro-1H-inden-4-yl)pyridin-4-yl)-1-methyl-1H-pyrrolo[2,3-b]pyridin-3-yl)methyl)amino)methyl)pyrrolidin-2-one;
(R)-5-((((6-(3-chloro-2-((R)-1-((6-methoxy-5-(((((S)-5-oxopyrrolidin-2-yl)methyl)amino)methyl)-3-(trifluoromethyl)pyrazin-2-yl)amino)-2,3-dihydro-1H-inden-4-yl)pyridin-4-yl)-1-methyl-1H-pyrrolo[2,3-b]pyridin-3-yl)methyl)amino)methyl)pyrrolidin-2-one;
(R)-5-((((6-(3-chloro-2-((S)-1-((6-methoxy-5-(((((R)-5-oxopyrrolidin-2-yl)methyl)amino)methyl)-3-(trifluoromethyl)pyrazin-2-yl)amino)-2,3-dihydro-1H-inden-4-yl)pyridin-4-yl)-1-methyl-1H-pyrrolo[2,3-b]pyridin-3-yl)methyl)amino)methyl)pyrrolidin-2-one;
(R)-5-((((6-(3-chloro-2-((S)-1-((6-methoxy-5-(((((S)-5-oxopyrrolidin-2-yl)methyl)amino)methyl)-3-(trifluoromethyl)pyrazin-2-yl)amino)-2,3-dihydro-1H-inden-4-yl)pyridin-4-yl)-1-methyl-1H-pyrrolo[2,3-b]pyridin-3-yl)methyl)amino)methyl)pyrrolidin-2-one;
(S)-5-((((6-(3-chloro-2-((R)-1-((6-methoxy-5-(((((R)-5-oxopyrrolidin-2-yl)methyl)amino)methyl)-3-(trifluoromethyl)pyrazin-2-yl)amino)-2,3-dihydro-1H-inden-4-yl)pyridin-4-yl)-1-methyl-1H-pyrrolo[2,3-b]pyridin-3-yl)methyl)amino)methyl)pyrrolidin-2-one;
(S)-5-((((6-(3-chloro-2-((R)-1-((6-methoxy-5-(((((S)-5-oxopyrrolidin-2-yl)methyl)amino)methyl)-3-(trifluoromethyl)pyrazin-2-yl)amino)-2,3-dihydro-1H-inden-4-yl)pyridin-4-yl)-1-methyl-1H-pyrrolo[2,3-b]pyridin-3-yl)methyl)amino)methyl)pyrrolidin-2-one;
(S)-5-((((6-(3-chloro-2-((S)-1-((6-methoxy-5-(((((R)-5-oxopyrrolidin-2-yl)methyl)amino)methyl)-3-(trifluoromethyl)pyrazin-2-yl)amino)-2,3-dihydro-1H-inden-4-yl)pyridin-4-yl)-1-methyl-1H-pyrrolo[2,3-b]pyridin-3-yl)methyl)amino)methyl)pyrrolidin-2-one;
1-(4-(((6-(3-(2-(4-(((1-acetylpiperidin-4-yl)amino)methyl)-3-methoxyphenyl)-3-chloropyridin-4-yl)-2-chlorophenyl)-2-methoxypyridin-3-yl)methyl)amino)piperidin-1-yl)-2-hydroxyethan-1-one;
N-((6-(2-chloro-3-(3-chloro-2-(4-(((3-fluoropropyl)amino)methyl)-3-methoxyphenyl)pyridin-4-yl)phenyl)-2-methoxypyridin-3-yl)methyl)-3-fluoropropan-1-amine;
2-(4-(6-(2-chloro-3-(6-methoxy-5-((7-oxo-2,6-diazaspiro[3.4]octan-2-yl)methyl)pyridin-2-yl)phenyl)-5-methylpyrimidin-4-yl)-2-methoxybenzyl)-2,6-diazaspiro[3.4]octan-7-one;
1-(1-((6-(3-(2-(4-((4-acetylpiperidin-1-yl)methyl)-3-methoxyphenyl)-3-chloropyridin-4-yl)-2-chlorophenyl)-2-methoxypyridin-3-yl)methyl)piperidin-4-yl)ethan-1-one;
1-(4-(((6-(2-chloro-3-(3-chloro-2-(4-(((3-fluoropropyl)amino)methyl)-3-methoxyphenyl)pyridin-4-yl)phenyl)-2-methoxypyridin-3-yl)methyl)amino)piperidin-1-yl)ethan-1-one;
1-(6-((6-(3-(6-(4-((6-acetyl-2,6-diazaspiro[3.3]heptan-2-yl)methyl)-3-methoxyphenyl)-5-methylpyrimidin-4-yl)-2-chlorophenyl)-2-methoxypyridin-3-yl)methyl)-2,6-diazaspiro[3.3]heptan-2-yl)ethan-1-one;
1-(4-(((6-(2-chloro-3-(3-chloro-2-(4-((((1r,4r)-4-hydroxycyclohexyl)amino)methyl)-3-methoxyphenyl)pyridin-4-yl)phenyl)-2-methoxypyridin-3-yl)methyl)amino)piperidin-1-yl)-2-hydroxyethan-1-one;
1-(4-(((6-(2-chloro-3-(3-chloro-2-(4-((((1s,4r)-4-hydroxycyclohexyl)amino)methyl)-3-methoxyphenyl)pyridin-4-yl)phenyl)-2-methoxypyridin-3-yl)methyl)amino)piperidin-1-yl)-2-hydroxyethan-1-one;
1-(4-(((6-(2-chloro-3-(3-chloro-2-(4-((((1r,4s)-4-hydroxycyclohexyl)amino)methyl)-3-methoxyphenyl)pyridin-4-yl)phenyl)-2-methoxypyridin-3-yl)methyl)amino)piperidin-1-yl)-2-hydroxyethan-1-one;
1-(4-(((6-(2-chloro-3-(3-chloro-2-(4-((((1s,4s)-4-hydroxycyclohexyl)amino)methyl)-3-methoxyphenyl)pyridin-4-yl)phenyl)-2-methoxypyridin-3-yl)methyl)amino)piperidin-1-yl)-2-hydroxyethan-1-one;
1-(4-(((6-(3-(6-(4-(((1-acetylpiperidin-4-yl)amino)methyl)-3-methoxyphenyl)-5-chloropyrimidin-4-yl)-2-chlorophenyl)-2-methoxypyridin-3-yl)methyl)amino)piperidin-1-yl)ethan-1-one;
(R)-5-(((6-(3-(2-(4-(((1-acetylpiperidin-4-yl)amino)methyl)-3-methoxyphenyl)-3-chloropyridin-4-yl)-2-chlorophenyl)-2-methoxypyridin-3-yl)methyl)amino)piperidin-2-one;
(S)-5-(((6-(3-(2-(4-(((1-acetylpiperidin-4-yl)amino)methyl)-3-methoxyphenyl)-3-chloropyridin-4-yl)-2-chlorophenyl)-2-methoxypyridin-3-yl)methyl)amino)piperidin-2-one;
(S)-5-((((6-(2-chloro-3-(5-chloro-6-(3-methoxy-4-(((((S)-5-oxopyrrolidin-2-yl)methyl)amino)methyl)phenyl)pyrimidin-4-yl)phenyl)-2-methoxypyridin-3-yl)methyl)amino)methyl)pyrrolidin-2-one;
(S)-5-((((6-(2-chloro-3-(5-chloro-6-(3-methoxy-4-(((((R)-5-oxopyrrolidin-2-yl)methyl)amino)methyl)phenyl)pyrimidin-4-yl)phenyl)-2-methoxypyridin-3-yl)methyl)amino)methyl)pyrrolidin-2-one;
(R)-5-((((6-(2-chloro-3-(5-chloro-6-(3-methoxy-4-(((((S)-5-oxopyrrolidin-2-yl)methyl)amino)methyl)phenyl)pyrimidin-4-yl)phenyl)-2-methoxypyridin-3-yl)methyl)amino)methyl)pyrrolidin-2-one;
(R)-5-((((6-(2-chloro-3-(5-chloro-6-(3-methoxy-4-(((((R)-5-oxopyrrolidin-2-yl)methyl)amino)methyl)phenyl)pyrimidin-4-yl)phenyl)-2-methoxypyridin-3-yl)methyl)amino)methyl)pyrrolidin-2-one;
(S)-5-((((6-(((S)-4-(5-(((1-acetylpiperidin-4-yl)amino)methyl)-3'-chloro-6-methoxy-[2,4'-bipyridin]-2'-yl)-2,3-dihydro-1H-inden-1-yl)oxy)-2-methoxy-5-(trifluoromethyl)pyridin-3-yl)methyl)amino)methyl)pyrrolidin-2-one;
(S)-5-((((6-(((R)-4-(5-(((1-acetylpiperidin-4-yl)amino)methyl)-3'-chloro-6-methoxy-[2,4'-bipyridin]-2'-yl)-2,3-dihydro-1H-inden-1-yl)oxy)-2-methoxy-5-(trifluoromethyl)pyridin-3-yl)methyl)amino)methyl)pyrrolidin-2-one;
(R)-5-((((6-(((S)-4-(5-(((1-acetylpiperidin-4-yl)amino)methyl)-3'-chloro-6-methoxy-[2,4'-bipyridin]-2'-yl)-2,3-dihydro-1H-inden-1-yl)oxy)-2-methoxy-5-(trifluoromethyl)pyridin-3-yl)methyl)amino)methyl)pyrrolidin-2-one;
(R)-5-((((6-(((R)-4-(5-(((1-acetylpiperidin-4-yl)amino)methyl)-3'-chloro-6-methoxy-[2,4'-bipyridin]-2'-yl)-2,3-dihydro-1H-inden-1-yl)oxy)-2-methoxy-5-(trifluoromethyl)pyridin-3-yl)methyl)amino)methyl)pyrrolidin-2-one;
(S)-5-((((6-(3-chloro-2-((S)-1-((6-methoxy-5-(((((S)-5-oxopyrrolidin-2-yl)methyl)amino)methyl)-3-(trifluoromethyl)pyridin-2-yl)oxy)-2,3-dihydro-1H-inden-4-yl)pyridin-4-yl)-1-methyl-1H-pyrrolo[2,3-b]pyridin-3-yl)methyl)amino)methyl)pyrrolidin-2-one;
(R)-5-((((6-(3-chloro-2-((R)-1-((6-methoxy-5-(((((R)-5-oxopyrrolidin-2-yl)methyl)amino)methyl)-3-(trifluoromethyl)pyridin-2-yl)oxy)-2,3-dihydro-1H-inden-4-yl)pyridin-4-yl)-1-methyl-1H-pyrrolo[2,3-b]pyridin-3-yl)methyl)amino)methyl)pyrrolidin-2-one;
(R)-5-((((6-(3-chloro-2-((R)-1-((6-methoxy-5-(((((S)-5-oxopyrrolidin-2-yl)methyl)amino)methyl)-3-(trifluoromethyl)pyridin-2-yl)oxy)-2,3-dihydro-1H-inden-4-yl)pyridin-4-yl)-1-methyl-1H-pyrrolo[2,3-b]pyridin-3-yl)methyl)amino)methyl)pyrrolidin-2-one;
(R)-5-((((6-(3-chloro-2-((S)-1-((6-methoxy-5-(((((R)-5-oxopyrrolidin-2-yl)methyl)amino)methyl)-3-(trifluoromethyl)pyridin-2-yl)oxy)-2,3-dihydro-1H-inden-4-yl)pyridin-4-yl)-1-methyl-1H-pyrrolo[2,3-b]pyridin-3-yl)methyl)amino)methyl)pyrrolidin-2-one;
(R)-5-((((6-(3-chloro-2-((S)-1-((6-methoxy-5-(((((S)-5-oxopyrrolidin-2-yl)methyl)amino)methyl)-3-(trifluoromethyl)pyridin-2-yl)oxy)-2,3-dihydro-1H-inden-4-yl)pyridin-4-yl)-1-methyl-1H-pyrrolo[2,3-b]pyridin-3-yl)methyl)amino)methyl)pyrrolidin-2-one;
(S)-5-((((6-(3-chloro-2-((R)-1-((6-methoxy-5-(((((R)-5-oxopyrrolidin-2-yl)methyl)amino)methyl)-3-(trifluoromethyl)pyridin-2-yl)oxy)-2,3-dihydro-1H-inden-4-yl)pyridin-4-yl)-1-methyl-1H-pyrrolo[2,3-b]pyridin-3-yl)methyl)amino)methyl)pyrrolidin-2-one;
(S)-5-((((6-(3-chloro-2-((R)-1-((6-methoxy-5-(((((S)-5-oxopyrrolidin-2-yl)methyl)amino)methyl)-3-(trifluoromethyl)pyridin-2-yl)oxy)-2,3-dihydro-1H-inden-4-yl)pyridin-4-yl)-1-methyl-1H-pyrrolo[2,3-b]pyridin-3-yl)methyl)amino)methyl)pyrrolidin-2-one;
(S)-5-((((6-(3-chloro-2-((S)-1-((6-methoxy-5-(((((R)-5-oxopyrrolidin-2-yl)methyl)amino)methyl)-3-(trifluoromethyl)pyridin-2-yl)oxy)-2,3-dihydro-1H-inden-4-yl)pyridin-4-yl)-1-methyl-1H-pyrrolo[2,3-b]pyridin-3-yl)methyl)amino)methyl)pyrrolidin-2-one;
1-(4-(((6-(2-chloro-3-(3-chloro-2-(3-methoxy-4-(((1-(3-methoxypropanoyl)piperidin-4-yl)amino)methyl)phenyl)pyridin-4-yl)phenyl)-2-methoxypyridin-3-yl)methyl)amino)piperidin-1-yl)-3-methoxypropan-1-one;
2-((6-(3-(6'-(((1-acetylpiperidin-4-yl)amino)methyl)-3-chloro-5'-methoxy-[2,3'-bipyridin]-4-yl)-2-chlorophenyl)-2-methoxypyridin-3-yl)methyl)-2,6-diazaspiro[3.4]octan-7-one;
2-((6-(2-chloro-3-(3-chloro-5'-methoxy-6'-(((tetrahydro-2H-pyran-4-yl)amino)methyl)-[2,3'-bipyridin]-4-yl)phenyl)-2-methoxypyridin-3-yl)methyl)-2,6-diazaspiro[3.4]octan-7-one;
2-((6-(2-chloro-3-(3-chloro-6'-((((1r,4r)-4-hydroxycyclohexyl)amino)methyl)-5'-methoxy-[2,3'-bipyridin]-4-yl)phenyl)-2-methoxypyridin-3-yl)methyl)-2,6-diazaspiro[3.4]octan-7-one;
2-((6-(2-chloro-3-(3-chloro-6'-((((1s,4r)-4-hydroxycyclohexyl)amino)methyl)-5'-methoxy-[2,3'-bipyridin]-4-yl)phenyl)-2-methoxypyridin-3-yl)methyl)-2,6-diazaspiro[3.4]octan-7-one;
2-((6-(2-chloro-3-(3-chloro-6'-((((1r,4s)-4-hydroxycyclohexyl)amino)methyl)-5'-methoxy-[2,3'-bipyridin]-4-yl)phenyl)-2-methoxypyridin-3-yl)methyl)-2,6-diazaspiro[3.4]octan-7-one;
2-((6-(2-chloro-3-(3-chloro-6'-((((1s,4s)-4-hydroxycyclohexyl)amino)methyl)-5'-methoxy-[2,3'-bipyridin]-4-yl)phenyl)-2-methoxypyridin-3-yl)methyl)-2,6-diazaspiro[3.4]octan-7-one;
(S)-2-((6-(2-chloro-3-(3-chloro-5'-methoxy-6'-((((5-oxopyrrolidin-2-yl)methyl)amino)methyl)-[2,3'-bipyridin]-4-yl)phenyl)-2-methoxypyridin-3-yl)methyl)-2,6-diazaspiro[3.4]octan-7-one;
(R)-2-((6-(2-chloro-3-(3-chloro-5'-methoxy-6'-((((5-oxopyrrolidin-2-yl)methyl)amino)methyl)-[2,3'-bipyridin]-4-yl)phenyl)-2-methoxypyridin-3-yl)methyl)-2,6-diazaspiro[3.4]octan-7-one;
(S)-2-((2'-(1-((5-(((1-acetylpiperidin-4-yl)amino)methyl)-6-methoxy-3-(trifluoromethyl)pyridin-2-yl)oxy)-2,3-dihydro-1H-inden-4-yl)-3'-chloro-6-methoxy-[2,4'-bipyridin]-5-yl)methyl)-2,6-diazaspiro[3.4]octan-7-one;
(R)-2-((2'-(1-((5-(((1-acetylpiperidin-4-yl)amino)methyl)-6-methoxy-3-(trifluoromethyl)pyridin-2-yl)oxy)-2,3-dihydro-1H-inden-4-yl)-3'-chloro-6-methoxy-[2,4'-bipyridin]-5-yl)methyl)-2,6-diazaspiro[3.4]octan-7-one;
(S)-2-((3'-chloro-6-methoxy-2'-(1-((6-methoxy-5-(((tetrahydro-2H-pyran-4-yl)amino)methyl)-3-(trifluoromethyl)pyridin-2-yl)oxy)-2,3-dihydro-1H-inden-4-yl)-[2,4'-bipyridin]-5-yl)methyl)-2,6-diazaspiro[3.4]octan-7-one;
(R)-2-((3'-chloro-6-methoxy-2'-(1-((6-methoxy-5-(((tetrahydro-2H-pyran-4-yl)amino)methyl)-3-(trifluoromethyl)pyridin-2-yl)oxy)-2,3-dihydro-1H-inden-4-yl)-[2,4'-bipyridin]-5-yl)methyl)-2,6-diazaspiro[3.4]octan-7-one;
2-((3'-chloro-2'-((S)-1-((5-((((S)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)-2,6-diazaspiro[3.4]octan-7-one;
2-((3'-chloro-2'-((S)-1-((5-((((R)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)-2,6-diazaspiro[3.4]octan-7-one;
2-((3'-chloro-2'-((R)-1-((5-((((S)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)-2,6-diazaspiro[3.4]octan-7-one;
2-((3'-chloro-2'-((R)-1-((5-((((R)-2-hydroxypropyl)amino)methyl)-6-methoxy-3-(trifluoromethyl)pyridin-2-yl)oxy)-2,3-dihydro-1H-inden-4-yl)-6-methoxy-[2,4'-bipyridin]-5-yl)methyl)-2,6-diazaspiro[3.4]octan-7-one;
2-(4-(5-chloro-6-(2-chloro-3-(6-methoxy-5-((7-oxo-2,6-diazaspiro[3.4]octan-2-yl)methyl)pyridin-2-yl)phenyl)pyrimidin-4-yl)-2-methoxybenzyl)-2,6-diazaspiro[3.4]octan-7-one;
(S)-6-(3-chloro-4-(2-chloro-3-(6-methoxy-5-((((5-oxopyrrolidin-2-yl)methyl)amino)methyl)pyridin-2-yl)phenyl)pyridin-2-yl)-2-methoxy-3-(2-(methylamino)ethyl)pyrrolo[2,1-f][1,2,4]triazin-4(3H)-one;
(R)-6-(3-chloro-4-(2-chloro-3-(6-methoxy-5-((((5-oxopyrrolidin-2-yl)methyl)amino)methyl)pyridin-2-yl)phenyl)pyridin-2-yl)-2-methoxy-3-(2-(methylamino)ethyl)pyrrolo[2,1-f][1,2,4]triazin-4(3H)-one;
1-(4-((4-(3-chloro-4-(2-chloro-3-(1-methyl-3-(((tetrahydro-2H-pyran-4-yl)amino)methyl)-1H-pyrrolo[2,3-b]pyridin-6-yl)phenyl)pyridin-2-yl)-2-methoxybenzyl)amino)piperidin-1-yl)ethan-1-one;
1-(4-((4-(3-chloro-4-(2-chloro-3-(1-methyl-3-((((tetrahydro-2H-pyran-4-yl)methyl)amino)methyl)-1H-pyrrolo[2,3-b]pyridin-6-yl)phenyl)pyridin-2-yl)-2-methoxybenzyl)amino)piperidin-1-yl)ethan-1-one;
1-(4-((4-(3-chloro-4-(2-chloro-3-(3-(((1,1-dioxidotetrahydro-2H-thiopyran-4-yl)amino)methyl)-1-methyl-1H-pyrrolo[2,3-b]pyridin-6-yl)phenyl)pyridin-2-yl)-2-methoxybenzyl)amino)piperidin-1-yl)ethan-1-one;
1-(4-((4-(3-chloro-4-(2-chloro-3-(3-(((2-hydroxyethyl)amino)methyl)-1-methyl-1H-pyrrolo[2,3-b]pyridin-6-yl)phenyl)pyridin-2-yl)-2-methoxybenzyl)amino)piperidin-1-yl)ethan-1-one;
1-(4-(((6-(2-chloro-3-(3-chloro-2-(4-(((2-hydroxyethyl)amino)methyl)-3-methoxyphenyl)pyridin-4-yl)phenyl)-1-methyl-1H-pyrrolo[2,3-b]pyridin-3-yl)methyl)amino)piperidin-1-yl)ethan-1-one;
(S)-5-((((6-(2-chloro-3-(3-chloro-2-(4-methyl-1,1-dioxido-2,3,4,5-tetrahydrobenzo[f][1,4]thiazepin-8-yl)pyridin-4-yl)phenyl)-2-methoxypyridin-3-yl)methyl)amino)methyl)pyrrolidin-2-one;
(R)-5-((((6-(2-chloro-3-(3-chloro-2-(4-methyl-1,1-dioxido-2,3,4,5-tetrahydrobenzo[f][1,4]thiazepin-8-yl)pyridin-4-yl)phenyl)-2-methoxypyridin-3-yl)methyl)amino)methyl)pyrrolidin-2-one;
1-(4-((6-(3-(6-(4-((4-acetylpiperazin-1-yl)methyl)-3-methoxyphenyl)-5-chloropyrimidin-4-yl)-2-chlorophenyl)-2-methoxypyridin-3-yl)methyl)piperazin-1-yl)ethan-1-one;
N-((6-(2-chloro-3-(3-chloro-2-(3-methoxy-4-(((tetrahydro-2H-pyran-4-yl)amino)methyl)phenyl)pyridin-4-yl)phenyl)-1-methyl-1H-pyrrolo[2,3-b]pyridin-3-yl)methyl)tetrahydro-2H-pyran-4-amine;
1-(4-(((6-(3-(2-(4-((6-acetyl-2,6-diazaspiro[3.3]heptan-2-yl)methyl)-3-methoxyphenyl)-3-chloropyridin-4-yl)-2-chlorophenyl)-1-methyl-1H-pyrrolo[2,3-b]pyridin-3-yl)methyl)amino)piperidin-1-yl)ethan-1-one;
1-(4-(((5-(3-(2-(4-(((1-acetylpiperidin-4-yl)amino)methyl)-3-methoxyphenyl)-3-chloropyridin-4-yl)-2-chlorophenyl)-3-methoxypyridin-2-yl)methyl)amino)piperidin-1-yl)ethan-1-one;
2-(4-(3-chloro-4-(2-chloro-3-(5-methoxy-6-((7-oxo-2,6-diazaspiro[3.4]octan-2-yl)methyl)pyridin-3-yl)phenyl)pyridin-2-yl)-2-methoxybenzyl)-2,6-diazaspiro[3.4]octan-7-one;
1-(4-(((5-(3-(6'-(((1-acetylpiperidin-4-yl)amino)methyl)-3-chloro-5'-methoxy-[2,3'-bipyridin]-4-yl)-2-chlorophenyl)-3-methoxypyridin-2-yl)methyl)amino)piperidin-1-yl)ethan-1-one;
2-(4-(5-chloro-6-(2-chloro-3-(5-((6-hydroxy-2-azaspiro[3.3]heptan-2-yl)methyl)-6-methoxypyridin-2-yl)phenyl)pyrimidin-4-yl)-2-methoxybenzyl)-2-azaspiro[3.3]heptan-6-ol;
2-((5-(2-chloro-3-(3-chloro-5'-methoxy-6'-((7-oxo-2,6-diazaspiro[3.4]octan-2-yl)methyl)-[2,3'-bipyridin]-4-yl)phenyl)-3-methoxypyridin-2-yl)methyl)-2,6-diazaspiro[3.4]octan-7-one;
N-((5-(2-chloro-3-(3-chloro-5'-methoxy-6'-(((tetrahydro-2H-pyran-4-yl)amino)methyl)-[2,3'-bipyridin]-4-yl)phenyl)-3-methoxypyridin-2-yl)methyl)tetrahydro-2H-pyran-4-amine;
2-(4-(3-chloro-4-(2-chloro-3-(6-methoxy-5-((6-methyl-7-oxo-2,6-diazaspiro[3.4]octan-2-yl)methyl)pyridin-2-yl)phenyl)pyridin-2-yl)-2-methoxybenzyl)-6-methyl-2,6-diazaspiro[3.4]octan-7-one;
1-(4-(((6-(3-(5-(((1-acetylpiperidin-4-yl)amino)methyl)-3'-chloro-6-methoxy-[2,4'-bipyridin]-2'-yl)-2-chlorophenyl)-2-methoxypyridin-3-yl)methyl)amino)piperidin-1-yl)ethan-1-one;
2-((6-(2-chloro-3-(3'-chloro-6-methoxy-5-((7-oxo-2,6-diazaspiro[3.4]octan-2-yl)methyl)-[2,4'-bipyridin]-2'-yl)phenyl)-2-methoxypyridin-3-yl)methyl)-2,6-diazaspiro[3.4]octan-7-one;
N-((3'-chloro-2'-(2-chloro-3-(6-methoxy-5-(((tetrahydro-2H-pyran-4-yl)amino)methyl)pyridin-2-yl)phenyl)-6-methoxy-[2,4'-bipyridin]-5-yl)methyl)tetrahydro-2H-pyran-4-amine;
(S)-5-((((3'-chloro-2'-(2-chloro-3-(6-methoxy-5-(((((S)-5-oxopyrrolidin-2-yl)methyl)amino)methyl)pyridin-2-yl)phenyl)-6-methoxy-[2,4'-bipyridin]-5-yl)methyl)amino)methyl)pyrrolidin-2-one;
(S)-5-((((3'-chloro-2'-(2-chloro-3-(6-methoxy-5-(((((R)-5-oxopyrrolidin-2-yl)methyl)amino)methyl)pyridin-2-yl)phenyl)-6-methoxy-[2,4'-bipyridin]-5-yl)methyl)amino)methyl)pyrrolidin-2-one;
(R)-5-((((3'-chloro-2'-(2-chloro-3-(6-methoxy-5-(((((S)-5-oxopyrrolidin-2-yl)methyl)amino)methyl)pyridin-2-yl)phenyl)-6-methoxy-[2,4'-bipyridin]-5-yl)methyl)amino)methyl)pyrrolidin-2-one;
(R)-5-((((3'-chloro-2'-(2-chloro-3-(6-methoxy-5-(((((R)-5-oxopyrrolidin-2-yl)methyl)amino)methyl)pyridin-2-yl)phenyl)-6-methoxy-[2,4'-bipyridin]-5-yl)methyl)amino)methyl)pyrrolidin-2-one;
2-(4-(3-chloro-4-(3-(6-methoxy-5-((7-oxo-2,6-diazaspiro[3.4]octan-2-yl)methyl)pyridin-2-yl)-2-(trifluoromethyl)phenyl)pyridin-2-yl)-2-methoxybenzyl)-2,6-diazaspiro[3.4]octan-7-one;
1-(6-((6-(3-(2-(4-((6-acetyl-2,6-diazaspiro[3.3]heptan-2-yl)methyl)-3-methoxyphenyl)-3-chloropyridin-4-yl)-2-(trifluoromethyl)phenyl)-2-methoxypyridin-3-yl)methyl)-2,6-diazaspiro[3.3]heptan-2-yl)ethan-1-one;
6-(4-(4-(3-(5-((2-oxa-6-azaspiro[3.3]heptan-6-yl)methyl)-6-methoxypyridin-2-yl)-2-(trifluoromethyl)phenyl)-3-chloropyridin-2-yl)-2-methoxybenzyl)-2-oxa-6-azaspiro[3.3]heptane;
2-(4-(4-(3-(3-(((1-acetylpiperidin-4-yl)amino)methyl)-1-methyl-1H-pyrrolo[2,3-b]pyridin-6-yl)-2-chlorophenyl)-3-chloropyridin-2-yl)-2-methoxybenzyl)-2,6-diazaspiro[3.4]octan-7-one;
(S)-1-(((6-(3-(3-chloro-2-(4-((((S)-2-hydroxypropyl)amino)methyl)-3-methoxyphenyl)pyridin-4-yl)-2-(trifluoromethyl)phenyl)-2-methoxypyridin-3-yl)methyl)amino)propan-2-ol;
(S)-1-(((6-(3-(3-chloro-2-(4-((((R)-2-hydroxypropyl)amino)methyl)-3-methoxyphenyl)pyridin-4-yl)-2-(trifluoromethyl)phenyl)-2-methoxypyridin-3-yl)methyl)amino)propan-2-ol;
(R)-1-(((6-(3-(3-chloro-2-(4-((((S)-2-hydroxypropyl)amino)methyl)-3-methoxyphenyl)pyridin-4-yl)-2-(trifluoromethyl)phenyl)-2-methoxypyridin-3-yl)methyl)amino)propan-2-ol;
(R)-1-(((6-(3-(3-chloro-2-(4-((((R)-2-hydroxypropyl)amino)methyl)-3-methoxyphenyl)pyridin-4-yl)-2-(trifluoromethyl)phenyl)-2-methoxypyridin-3-yl)methyl)amino)propan-2-ol;
2-(((6-(2-chloro-3-(5-chloro-6-(4-(((2-hydroxyethyl)amino)methyl)-3-methoxyphenyl)pyrimidin-4-yl)phenyl)-2-methoxypyridin-3-yl)methyl)amino)ethan-1-ol;
5-((((6-(2-chloro-3-(3-chloro-2-(1,1-dioxido-4-(((S)-5-oxopyrrolidin-2-yl)methyl)-2,3,4,5-tetrahydrobenzo[f][1,4]thiazepin-8-yl)pyridin-4-yl)phenyl)-2-methoxypyridin-3-yl)methyl)amino)methyl)pyrrolidin-2-one;
5-((((6-(2-chloro-3-(3-chloro-2-(1,1-dioxido-4-(((R)-5-oxopyrrolidin-2-yl)methyl)-2,3,4,5-tetrahydrobenzo[f][1,4]thiazepin-8-yl)pyridin-4-yl)phenyl)-2-methoxypyridin-3-yl)methyl)amino)methyl)pyrrolidin-2-one;
(S)-5-((((6-(2-chloro-3-(3-chloro-2-(1,1-dioxido-4-(((S)-5-oxopyrrolidin-2-yl)methyl)-2,3,4,5-tetrahydrobenzo[f][1,4]thiazepin-8-yl)pyridin-4-yl)phenyl)-2-methoxypyridin-3-yl)methyl)amino)methyl)pyrrolidin-2-one;
(S)-5-((((6-(2-chloro-3-(3-chloro-2-(1,1-dioxido-4-(((R)-5-oxopyrrolidin-2-yl)methyl)-2,3,4,5-tetrahydrobenzo[f][1,4]thiazepin-8-yl)pyridin-4-yl)phenyl)-2-methoxypyridin-3-yl)methyl)amino)methyl)pyrrolidin-2-one;
(R)-5-((((6-(2-chloro-3-(3-chloro-2-(1,1-dioxido-4-(((S)-5-oxopyrrolidin-2-yl)methyl)-2,3,4,5-tetrahydrobenzo[f][1,4]thiazepin-8-yl)pyridin-4-yl)phenyl)-2-methoxypyridin-3-yl)methyl)amino)methyl)pyrrolidin-2-one;
(R)-5-((((6-(2-chloro-3-(3-chloro-2-(1,1-dioxido-4-(((R)-5-oxopyrrolidin-2-yl)methyl)-2,3,4,5-tetrahydrobenzo[f][1,4]thiazepin-8-yl)pyridin-4-yl)phenyl)-2-methoxypyridin-3-yl)methyl)amino)methyl)pyrrolidin-2-one;
1-(4-(((6-(2-chloro-3-(3-chloro-2-(8-ethoxy-2-((((1r,4r)-4-hydroxycyclohexyl)amino)methyl)-[1,2,4]triazolo[1,5-a]pyridin-6-yl)pyridin-4-yl)phenyl)-2-methoxypyridin-3-yl)methyl)amino)piperidin-1-yl)ethan-1-one;
1-(4-(((6-(2-chloro-3-(3-chloro-2-(8-ethoxy-2-((((1s,4r)-4-hydroxycyclohexyl)amino)methyl)-[1,2,4]triazolo[1,5-a]pyridin-6-yl)pyridin-4-yl)phenyl)-2-methoxypyridin-3-yl)methyl)amino)piperidin-1-yl)ethan-1-one;
1-(4-(((6-(2-chloro-3-(3-chloro-2-(8-ethoxy-2-((((1r,4s)-4-hydroxycyclohexyl)amino)methyl)-[1,2,4]triazolo[1,5-a]pyridin-6-yl)pyridin-4-yl)phenyl)-2-methoxypyridin-3-yl)methyl)amino)piperidin-1-yl)ethan-1-one;
1-(4-(((6-(2-chloro-3-(3-chloro-2-(8-ethoxy-2-((((1s,4s)-4-hydroxycyclohexyl)amino)methyl)-[1,2,4]triazolo[1,5-a]pyridin-6-yl)pyridin-4-yl)phenyl)-2-methoxypyridin-3-yl)methyl)amino)piperidin-1-yl)ethan-1-one;
1-(4-(((6-(2-chloro-3-(3-chloro-2-(8-ethoxy-2-((((1r,3r)-3-hydroxy-3-methylcyclobutyl)amino)methyl)-[1,2,4]triazolo[1,5-a]pyridin-6-yl)pyridin-4-yl)phenyl)-2-methoxypyridin-3-yl)methyl)amino)piperidin-1-yl)ethan-1-one;
1-(4-(((6-(2-chloro-3-(3-chloro-2-(8-ethoxy-2-((((1s,3r)-3-hydroxy-3-methylcyclobutyl)amino)methyl)-[1,2,4]triazolo[1,5-a]pyridin-6-yl)pyridin-4-yl)phenyl)-2-methoxypyridin-3-yl)methyl)amino)piperidin-1-yl)ethan-1-one;
1-(4-(((6-(2-chloro-3-(3-chloro-2-(8-ethoxy-2-((((1r,3s)-3-hydroxy-3-methylcyclobutyl)amino)methyl)-[1,2,4]triazolo[1,5-a]pyridin-6-yl)pyridin-4-yl)phenyl)-2-methoxypyridin-3-yl)methyl)amino)piperidin-1-yl)ethan-1-one;
1-(4-(((6-(2-chloro-3-(3-chloro-2-(8-ethoxy-2-((((1s,3s)-3-hydroxy-3-methylcyclobutyl)amino)methyl)-[1,2,4]triazolo[1,5-a]pyridin-6-yl)pyridin-4-yl)phenyl)-2-methoxypyridin-3-yl)methyl)amino)piperidin-1-yl)ethan-1-one;
1-(4-(((6-(4-(3-(5-(((1-acetylpiperidin-4-yl)amino)methyl)-6-methoxypyridin-2-yl)-2-chlorophenyl)-3-chloropyridin-2-yl)-8-ethoxy-[1,2,4]triazolo[1,5-a]pyridin-2-yl)methyl)amino)piperidin-1-yl)ethan-1-one;
2-((6-(4-(3-(5-(((1-acetylpiperidin-4-yl)amino)methyl)-6-methoxypyridin-2-yl)-2-chlorophenyl)-3-chloropyridin-2-yl)-8-ethoxy-[1,2,4]triazolo[1,5-a]pyridin-2-yl)methyl)-2,6-diazaspiro[3.4]octan-7-one;
N-((6-(3-(3-chloro-2-(4-(((3-fluoropropyl)amino)methyl)-3-methoxyphenyl)pyridin-4-yl)-2-(trifluoromethyl)phenyl)-2-methoxypyridin-3-yl)methyl)-3-fluoropropan-1-amine;
(1r,4r)-4-(((6-(3-(3-chloro-2-(4-((((1r,4r)-4-hydroxycyclohexyl)amino)methyl)-3-methoxyphenyl)pyridin-4-yl)-2-(trifluoromethyl)phenyl)-2-methoxypyridin-3-yl)methyl)amino)cyclohexan-1-ol;
(1r,4r)-4-(((6-(3-(3-chloro-2-(4-((((1r,4s)-4-hydroxycyclohexyl)amino)methyl)-3-methoxyphenyl)pyridin-4-yl)-2-(trifluoromethyl)phenyl)-2-methoxypyridin-3-yl)methyl)amino)cyclohexan-1-ol;
(1r,4r)-4-(((6-(3-(3-chloro-2-(4-((((1s,4r)-4-hydroxycyclohexyl)amino)methyl)-3-methoxyphenyl)pyridin-4-yl)-2-(trifluoromethyl)phenyl)-2-methoxypyridin-3-yl)methyl)amino)cyclohexan-1-ol;
(1r,4r)-4-(((6-(3-(3-chloro-2-(4-((((1s,4s)-4-hydroxycyclohexyl)amino)methyl)-3-methoxyphenyl)pyridin-4-yl)-2-(trifluoromethyl)phenyl)-2-methoxypyridin-3-yl)methyl)amino)cyclohexan-1-ol;
(1r,4s)-4-(((6-(3-(3-chloro-2-(4-((((1r,4r)-4-hydroxycyclohexyl)amino)methyl)-3-methoxyphenyl)pyridin-4-yl)-2-(trifluoromethyl)phenyl)-2-methoxypyridin-3-yl)methyl)amino)cyclohexan-1-ol;
(1r,4s)-4-(((6-(3-(3-chloro-2-(4-((((1r,4s)-4-hydroxycyclohexyl)amino)methyl)-3-methoxyphenyl)pyridin-4-yl)-2-(trifluoromethyl)phenyl)-2-methoxypyridin-3-yl)methyl)amino)cyclohexan-1-ol;
(1r,4s)-4-(((6-(3-(3-chloro-2-(4-((((1s,4r)-4-hydroxycyclohexyl)amino)methyl)-3-methoxyphenyl)pyridin-4-yl)-2-(trifluoromethyl)phenyl)-2-methoxypyridin-3-yl)methyl)amino)cyclohexan-1-ol;
(1r,4s)-4-(((6-(3-(3-chloro-2-(4-((((1s,4s)-4-hydroxycyclohexyl)amino)methyl)-3-methoxyphenyl)pyridin-4-yl)-2-(trifluoromethyl)phenyl)-2-methoxypyridin-3-yl)methyl)amino)cyclohexan-1-ol;
(1s,4r)-4-(((6-(3-(3-chloro-2-(4-((((1r,4r)-4-hydroxycyclohexyl)amino)methyl)-3-methoxyphenyl)pyridin-4-yl)-2-(trifluoromethyl)phenyl)-2-methoxypyridin-3-yl)methyl)amino)cyclohexan-1-ol;
(1s,4r)-4-(((6-(3-(3-chloro-2-(4-((((1r,4s)-4-hydroxycyclohexyl)amino)methyl)-3-methoxyphenyl)pyridin-4-yl)-2-(trifluoromethyl)phenyl)-2-methoxypyridin-3-yl)methyl)amino)cyclohexan-1-ol;
(1s,4r)-4-(((6-(3-(3-chloro-2-(4-((((1s,4r)-4-hydroxycyclohexyl)amino)methyl)-3-methoxyphenyl)pyridin-4-yl)-2-(trifluoromethyl)phenyl)-2-methoxypyridin-3-yl)methyl)amino)cyclohexan-1-ol;
(1s,4r)-4-(((6-(3-(3-chloro-2-(4-((((1s,4s)-4-hydroxycyclohexyl)amino)methyl)-3-methoxyphenyl)pyridin-4-yl)-2-(trifluoromethyl)phenyl)-2-methoxypyridin-3-yl)methyl)amino)cyclohexan-1-ol;
(1s,4s)-4-(((6-(3-(3-chloro-2-(4-((((1r,4r)-4-hydroxycyclohexyl)amino)methyl)-3-methoxyphenyl)pyridin-4-yl)-2-(trifluoromethyl)phenyl)-2-methoxypyridin-3-yl)methyl)amino)cyclohexan-1-ol;
(1s,4s)-4-(((6-(3-(3-chloro-2-(4-((((1r,4s)-4-hydroxycyclohexyl)amino)methyl)-3-methoxyphenyl)pyridin-4-yl)-2-(trifluoromethyl)phenyl)-2-methoxypyridin-3-yl)methyl)amino)cyclohexan-1-ol;
(1s,4s)-4-(((6-(3-(3-chloro-2-(4-((((1s,4r)-4-hydroxycyclohexyl)amino)methyl)-3-methoxyphenyl)pyridin-4-yl)-2-(trifluoromethyl)phenyl)-2-methoxypyridin-3-yl)methyl)amino)cyclohexan-1-ol;
(1s,4s)-4-(((6-(3-(3-chloro-2-(4-((((1s,4s)-4-hydroxycyclohexyl)amino)methyl)-3-methoxyphenyl)pyridin-4-yl)-2-(trifluoromethyl)phenyl)-2-methoxypyridin-3-yl)methyl)amino)cyclohexan-1-ol;
1-(6-((6-(3-(6-(4-((6-acetyl-2,6-diazaspiro[3.3]heptan-2-yl)methyl)-3-methoxyphenyl)-5-chloropyrimidin-4-yl)-2-chlorophenyl)-2-methoxypyridin-3-yl)methyl)-2,6-diazaspiro[3.3]heptan-2-yl)ethan-1-one;
N-(3-(3-chloro-2-(3-methoxy-4-((7-oxo-2,6-diazaspiro[3.4]octan-2-yl)methyl)phenyl)pyridin-4-yl)-2-methylphenyl)-5-((7-oxo-2,6-diazaspiro[3.4]octan-2-yl)methyl)picolinamide;
(3-(((6-(2-chloro-3-(3-chloro-2-(4-(((3-(hydroxymethyl)bicyclo[1.1.1]pentan-1-yl)amino)methyl)-3-methoxyphenyl)pyridin-4-yl)phenyl)-2-methoxypyridin-3-yl)methyl)amino)bicyclo[1.1.1]pentan-1-yl)methanol;
(1s,3s)-3-(((6-(2-chloro-3-(5-chloro-6-(4-((((1s,3s)-3-hydroxycyclobutyl)amino)methyl)-3-methoxyphenyl)pyrimidin-4-yl)phenyl)-2-methoxypyridin-3-yl)methyl)amino)cyclobutan-1-ol;
(1r,3r)-3-(((6-(2-chloro-3-(5-chloro-6-(4-((((1r,3r)-3-hydroxycyclobutyl)amino)methyl)-3-methoxyphenyl)pyrimidin-4-yl)phenyl)-2-methoxypyridin-3-yl)methyl)amino)cyclobutan-1-ol;
(1r,3r)-3-(((6-(2-chloro-3-(5-chloro-6-(4-((((1r,3s)-3-hydroxycyclobutyl)amino)methyl)-3-methoxyphenyl)pyrimidin-4-yl)phenyl)-2-methoxypyridin-3-yl)methyl)amino)cyclobutan-1-ol;
(1r,3r)-3-(((6-(2-chloro-3-(5-chloro-6-(4-((((1s,3r)-3-hydroxycyclobutyl)amino)methyl)-3-methoxyphenyl)pyrimidin-4-yl)phenyl)-2-methoxypyridin-3-yl)methyl)amino)cyclobutan-1-ol;
(1r,3r)-3-(((6-(2-chloro-3-(5-chloro-6-(4-((((1s,3s)-3-hydroxycyclobutyl)amino)methyl)-3-methoxyphenyl)pyrimidin-4-yl)phenyl)-2-methoxypyridin-3-yl)methyl)amino)cyclobutan-1-ol;
(1r,3s)-3-(((6-(2-chloro-3-(5-chloro-6-(4-((((1r,3r)-3-hydroxycyclobutyl)amino)methyl)-3-methoxyphenyl)pyrimidin-4-yl)phenyl)-2-methoxypyridin-3-yl)methyl)amino)cyclobutan-1-ol;
(1r,3s)-3-(((6-(2-chloro-3-(5-chloro-6-(4-((((1r,3s)-3-hydroxycyclobutyl)amino)methyl)-3-methoxyphenyl)pyrimidin-4-yl)phenyl)-2-methoxypyridin-3-yl)methyl)amino)cyclobutan-1-ol;
(1r,3s)-3-(((6-(2-chloro-3-(5-chloro-6-(4-((((1s,3r)-3-hydroxycyclobutyl)amino)methyl)-3-methoxyphenyl)pyrimidin-4-yl)phenyl)-2-methoxypyridin-3-yl)methyl)amino)cyclobutan-1-ol;
(1r,3s)-3-(((6-(2-chloro-3-(5-chloro-6-(4-((((1s,3s)-3-hydroxycyclobutyl)amino)methyl)-3-methoxyphenyl)pyrimidin-4-yl)phenyl)-2-methoxypyridin-3-yl)methyl)amino)cyclobutan-1-ol;
(1s,3r)-3-(((6-(2-chloro-3-(5-chloro-6-(4-((((1r,3r)-3-hydroxycyclobutyl)amino)methyl)-3-methoxyphenyl)pyrimidin-4-yl)phenyl)-2-methoxypyridin-3-yl)methyl)amino)cyclobutan-1-ol;
(1s,3r)-3-(((6-(2-chloro-3-(5-chloro-6-(4-((((1r,3s)-3-hydroxycyclobutyl)amino)methyl)-3-methoxyphenyl)pyrimidin-4-yl)phenyl)-2-methoxypyridin-3-yl)methyl)amino)cyclobutan-1-ol;
(1s,3r)-3-(((6-(2-chloro-3-(5-chloro-6-(4-((((1s,3r)-3-hydroxycyclobutyl)amino)methyl)-3-methoxyphenyl)pyrimidin-4-yl)phenyl)-2-methoxypyridin-3-yl)methyl)amino)cyclobutan-1-ol;
(1s,3r)-3-(((6-(2-chloro-3-(5-chloro-6-(4-((((1s,3s)-3-hydroxycyclobutyl)amino)methyl)-3-methoxyphenyl)pyrimidin-4-yl)phenyl)-2-methoxypyridin-3-yl)methyl)amino)cyclobutan-1-ol;
(1s,3s)-3-(((6-(2-chloro-3-(5-chloro-6-(4-((((1r,3r)-3-hydroxycyclobutyl)amino)methyl)-3-methoxyphenyl)pyrimidin-4-yl)phenyl)-2-methoxypyridin-3-yl)methyl)amino)cyclobutan-1-ol;
(1s,3s)-3-(((6-(2-chloro-3-(5-chloro-6-(4-((((1r,3s)-3-hydroxycyclobutyl)amino)methyl)-3-methoxyphenyl)pyrimidin-4-yl)phenyl)-2-methoxypyridin-3-yl)methyl)amino)cyclobutan-1-ol;
(1s,3s)-3-(((6-(2-chloro-3-(5-chloro-6-(4-((((1s,3r)-3-hydroxycyclobutyl)amino)methyl)-3-methoxyphenyl)pyrimidin-4-yl)phenyl)-2-methoxypyridin-3-yl)methyl)amino)cyclobutan-1-ol;
1-(6-((6-(2-chloro-3-(3-chloro-2-(3-methoxy-4-((6-(2-methoxyacetyl)-2,6-diazaspiro[3.3]heptan-2-yl)methyl)phenyl)pyridin-4-yl)phenyl)-2-methoxypyridin-3-yl)methyl)-2,6-diazaspiro[3.3]heptan-2-yl)-2-methoxyethan-1-one;
1-(4-(((6-(2-chloro-3-(3-chloro-2-(2-((((1r,4r)-4-hydroxycyclohexyl)amino)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one;
1-(4-(((6-(2-chloro-3-(3-chloro-2-(2-((((1s,4r)-4-hydroxycyclohexyl)amino)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one;
1-(4-(((6-(2-chloro-3-(3-chloro-2-(2-((((1r,4s)-4-hydroxycyclohexyl)amino)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one;
1-(4-(((6-(2-chloro-3-(3-chloro-2-(2-((((1s,4s)-4-hydroxycyclohexyl)amino)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one;
1-(4-(((6-(2-chloro-3-(3-chloro-2-(2-((((1r,3r)-3-hydroxy-3-methylcyclobutyl)amino)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one;
1-(4-(((6-(2-chloro-3-(3-chloro-2-(2-((((1s,3r)-3-hydroxy-3-methylcyclobutyl)amino)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one;
1-(4-(((6-(2-chloro-3-(3-chloro-2-(2-((((1r,3s)-3-hydroxy-3-methylcyclobutyl)amino)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one;
1-(4-(((6-(2-chloro-3-(3-chloro-2-(2-((((1s,3s)-3-hydroxy-3-methylcyclobutyl)amino)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one;
2-((6-(2-chloro-3-(3-chloro-2-(3-(difluoromethoxy)-4-((7-oxo-2,6-diazaspiro[3.4]octan-2-yl)methyl)phenyl)pyridin-4-yl)phenyl)-2-methoxypyridin-3-yl)methyl)-2,6-diazaspiro[3.4]octan-7-one;
1-(6-((6-(3-(2-(4-((6-acetyl-2,6-diazaspiro[3.3]heptan-2-yl)methyl)-3-(difluoromethoxy)phenyl)-3-chloropyridin-4-yl)-2-chlorophenyl)-2-methoxypyridin-3-yl)methyl)-2,6-diazaspiro[3.3]heptan-2-yl)ethan-1-one;
1-(4-(((S)-1-(6-(3-(2-(4-((S)-1-((1-acetylpiperidin-4-yl)amino)ethyl)-3-methoxyphenyl)-3-chloropyridin-4-yl)-2-chlorophenyl)-2-methoxypyridin-3-yl)ethyl)amino)piperidin-1-yl)ethan-1-one;
1-(4-(((S)-1-(6-(3-(2-(4-((R)-1-((1-acetylpiperidin-4-yl)amino)ethyl)-3-methoxyphenyl)-3-chloropyridin-4-yl)-2-chlorophenyl)-2-methoxypyridin-3-yl)ethyl)amino)piperidin-1-yl)ethan-1-one;
1-(4-(((R)-1-(6-(3-(2-(4-((S)-1-((1-acetylpiperidin-4-yl)amino)ethyl)-3-methoxyphenyl)-3-chloropyridin-4-yl)-2-chlorophenyl)-2-methoxypyridin-3-yl)ethyl)amino)piperidin-1-yl)ethan-1-one;
1-(4-(((R)-1-(6-(3-(2-(4-((R)-1-((1-acetylpiperidin-4-yl)amino)ethyl)-3-methoxyphenyl)-3-chloropyridin-4-yl)-2-chlorophenyl)-2-methoxypyridin-3-yl)ethyl)amino)piperidin-1-yl)ethan-1-one;
1-(4-(((6-(2-chloro-3-(3-chloro-2-(3-methoxy-4-(((tetrahydro-2H-pyran-4-yl)amino)methyl)phenyl)pyridin-4-yl)phenyl)-1-methyl-1H-pyrrolo[2,3-b]pyridin-3-yl)methyl)(methyl)amino)piperidin-1-yl)ethan-1-one;
1-(4-(((6-(3-(2-(4-((6-acetyl-2,6-diazaspiro[3.3]heptan-2-yl)methyl)-3-methoxyphenyl)-3-chloropyridin-4-yl)-2-chlorophenyl)-1-methyl-1H-pyrrolo[2,3-b]pyridin-3-yl)methyl)(methyl)amino)piperidin-1-yl)ethan-1-one;
1-(4-(((6-(3-(3-chloro-2-(3-methoxy-4-(((1-(2-methoxyacetyl)piperidin-4-yl)amino)methyl)phenyl)pyridin-4-yl)-2-(trifluoromethyl)phenyl)-2-methoxypyridin-3-yl)methyl)amino)piperidin-1-yl)-2-methoxyethan-1-one;
2-(4-(4-(3-(3-(((1-acetylpiperidin-4-yl)(methyl)amino)methyl)-1-methyl-1H-pyrrolo[2,3-b]pyridin-6-yl)-2-chlorophenyl)-3-chloropyridin-2-yl)-2-methoxybenzyl)-2,6-diazaspiro[3.4]octan-7-one;
N-(4-(4-(3-(5-(((2-oxaspiro[3.3]heptan-6-yl)amino)methyl)-6-methoxypyridin-2-yl)-2-(trifluoromethyl)phenyl)-3-chloropyridin-2-yl)-2-methoxybenzyl)-2-oxaspiro[3.3]heptan-6-amine;
2-(4-(3-chloro-4-(2-chloro-3-(1-methyl-3-(((tetrahydro-2H-pyran-4-yl)amino)methyl)-1H-pyrrolo[2,3-b]pyridin-6-yl)phenyl)pyridin-2-yl)-2-methoxybenzyl)-2,6-diazaspiro[3.4]octan-7-one;
2-((4-(3-chloro-4-(2-chloro-3-(1-methyl-3-(((tetrahydro-2H-pyran-4-yl)amino)methyl)-1H-pyrrolo[2,3-b]pyridin-6-yl)phenyl)pyridin-2-yl)-2-methoxybenzyl)amino)ethan-1-ol;
1-(4-(((6-(3-(2-(4-(((1-acetylpiperidin-4-yl)amino)methyl)-3-methoxyphenyl)-3-chloropyridin-4-yl)-2-chlorophenyl)-2-methoxypyridin-3-yl)methyl)amino)piperidin-1-yl)-3-methoxypropan-1-one;
(S)-5-((((3'-chloro-6-methoxy-2'-((S)-1-((6-methoxy-5-(((tetrahydro-2H-pyran-4-yl)amino)methyl)-3-(trifluoromethyl)pyridin-2-yl)oxy)-2,3-dihydro-1H-inden-4-yl)-[2,4'-bipyridin]-5-yl)methyl)amino)methyl)pyrrolidin-2-one;
(S)-5-((((3'-chloro-6-methoxy-2'-((R)-1-((6-methoxy-5-(((tetrahydro-2H-pyran-4-yl)amino)methyl)-3-(trifluoromethyl)pyridin-2-yl)oxy)-2,3-dihydro-1H-inden-4-yl)-[2,4'-bipyridin]-5-yl)methyl)amino)methyl)pyrrolidin-2-one;
(R)-5-((((3'-chloro-6-methoxy-2'-((S)-1-((6-methoxy-5-(((tetrahydro-2H-pyran-4-yl)amino)methyl)-3-(trifluoromethyl)pyridin-2-yl)oxy)-2,3-dihydro-1H-inden-4-yl)-[2,4'-bipyridin]-5-yl)methyl)amino)methyl)pyrrolidin-2-one;
(R)-5-((((3'-chloro-6-methoxy-2'-((R)-1-((6-methoxy-5-(((tetrahydro-2H-pyran-4-yl)amino)methyl)-3-(trifluoromethyl)pyridin-2-yl)oxy)-2,3-dihydro-1H-inden-4-yl)-[2,4'-bipyridin]-5-yl)methyl)amino)methyl)pyrrolidin-2-one;
1-(6-(4-(3-chloro-4-(2-chloro-3-(1-methyl-3-(((tetrahydro-2H-pyran-4-yl)amino)methyl)-1H-pyrrolo[2,3-b]pyridin-6-yl)phenyl)pyridin-2-yl)-2-methoxybenzyl)-2,6-diazaspiro[3.3]heptan-2-yl)ethan-1-one;
(S)-5-((((6-(3-(3-chloro-2-(3-methoxy-4-(((((S)-5-oxopyrrolidin-2-yl)methyl)amino)methyl)phenyl)pyridin-4-yl)-2-(trifluoromethyl)phenyl)-2-methoxypyridin-3-yl)methyl)amino)methyl)pyrrolidin-2-one;
(S)-5-((((6-(3-(3-chloro-2-(3-methoxy-4-(((((R)-5-oxopyrrolidin-2-yl)methyl)amino)methyl)phenyl)pyridin-4-yl)-2-(trifluoromethyl)phenyl)-2-methoxypyridin-3-yl)methyl)amino)methyl)pyrrolidin-2-one;
(R)-5-((((6-(3-(3-chloro-2-(3-methoxy-4-(((((S)-5-oxopyrrolidin-2-yl)methyl)amino)methyl)phenyl)pyridin-4-yl)-2-(trifluoromethyl)phenyl)-2-methoxypyridin-3-yl)methyl)amino)methyl)pyrrolidin-2-one;
(R)-5-((((6-(3-(3-chloro-2-(3-methoxy-4-(((((R)-5-oxopyrrolidin-2-yl)methyl)amino)methyl)phenyl)pyridin-4-yl)-2-(trifluoromethyl)phenyl)-2-methoxypyridin-3-yl)methyl)amino)methyl)pyrrolidin-2-one;
(S)-5-((((6-(2-chloro-3-(3-chloro-2-(4-((S)-2-hydroxypropyl)-1,1-dioxido-2,3,4,5-tetrahydrobenzo[f][1,4]thiazepin-8-yl)pyridin-4-yl)phenyl)-2-methoxypyridin-3-yl)methyl)amino)methyl)pyrrolidin-2-one;
(S)-5-((((6-(2-chloro-3-(3-chloro-2-(4-((R)-2-hydroxypropyl)-1,1-dioxido-2,3,4,5-tetrahydrobenzo[f][1,4]thiazepin-8-yl)pyridin-4-yl)phenyl)-2-methoxypyridin-3-yl)methyl)amino)methyl)pyrrolidin-2-one;
(R)-5-((((6-(2-chloro-3-(3-chloro-2-(4-((S)-2-hydroxypropyl)-1,1-dioxido-2,3,4,5-tetrahydrobenzo[f][1,4]thiazepin-8-yl)pyridin-4-yl)phenyl)-2-methoxypyridin-3-yl)methyl)amino)methyl)pyrrolidin-2-one;
(R)-5-((((6-(2-chloro-3-(3-chloro-2-(4-((R)-2-hydroxypropyl)-1,1-dioxido-2,3,4,5-tetrahydrobenzo[f][1,4]thiazepin-8-yl)pyridin-4-yl)phenyl)-2-methoxypyridin-3-yl)methyl)amino)methyl)pyrrolidin-2-one;
2-(((6-(3-(3-chloro-2-(4-(((2-hydroxyethyl)amino)methyl)-3-methoxyphenyl)pyridin-4-yl)-2-(trifluoromethyl)phenyl)-2-methoxypyridin-3-yl)methyl)amino)ethan-1-ol;
1-(6-(3-(3-chloro-2-(3-methoxy-4-((methylamino)methyl)phenyl)pyridin-4-yl)-2-(trifluoromethyl)phenyl)-2-methoxypyridin-3-yl)-N-methylmethanamine;
N-((6-(3-(3-chloro-2-(3-methoxy-4-(((2-methoxyethyl)amino)methyl)phenyl)pyridin-4-yl)-2-(trifluoromethyl)phenyl)-2-methoxypyridin-3-yl)methyl)-2-methoxyethan-1-amine;
2-(2-(4-(((1-acetylpiperidin-4-yl)amino)methyl)-3-methoxyphenyl)-3-chloropyridin-4-yl)-6-(5-(((1-acetylpiperidin-4-yl)amino)methyl)-6-methoxypyridin-2-yl)benzonitrile;
1-(4-(((6-(2-chloro-3-(3-chloro-2-(4-((4-hydroxypiperidin-1-yl)methyl)-3-methoxyphenyl)pyridin-4-yl)phenyl)-2-methoxypyridin-3-yl)methyl)amino)piperidin-1-yl)-3-methoxypropan-1-one;
(S)-5-((((6-(2-chloro-3-(3-chloro-2-(3-(difluoromethoxy)-4-(((((S)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
(S)-5-((((6-(2-chloro-3-(3-chloro-2-(3-(difluoromethoxy)-4-(((((R)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
(R)-5-((((6-(2-chloro-3-(3-chloro-2-(3-(difluoromethoxy)-4-(((((S)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
(R)-5-((((6-(2-chloro-3-(3-chloro-2-(3-(difluoromethoxy)-4-(((((R)-5-oxopyrrolidin-2-yl)methyl)amino)methyl)phenyl)pyridin-4-yl)phenyl)-2-methoxypyridin-3-yl)methyl)amino)methyl)pyrrolidin-2-one;
N-((6-(2-chloro-3-(3-chloro-2-(3-(difluoromethoxy)-4-(((tetrahydro-2H-pyran-4-yl)amino)methyl)phenyl)pyridin-4-yl)phenyl)-2-methoxypyridin-3-yl)methyl)tetrahydro-2H-pyran-4-amine;
4-(((6-(2-chloro-3-(3-chloro-2-(3-(difluoromethoxy)-4-(((1-methyl-2-oxopiperidin-4-yl)amino)methyl)phenyl)pyridin-4-yl)phenyl)-2-methoxypyridin-3-yl)methyl)amino)-1-methylpiperidin-2-one;
2-(4-(((6-(2-chloro-3-(3-chloro-2-(3-(difluoromethoxy)-4-(((1-(2-hydroxyethyl)piperidin-4-yl)amino)methyl)phenyl)pyridin-4-yl)phenyl)-2-methoxypyridin-3-yl)methyl)amino)piperidin-1-yl)ethan-1-ol;
1-(4-(((6-(3-(4-(4-(((1-acetylpiperidin-4-yl)amino)methyl)-3-methoxyphenyl)-3-chloropyridin-2-yl)-2-chlorophenyl)-2-methoxypyridin-3-yl)methyl)amino)piperidin-1-yl)ethan-1-one;
2-(4-(3-chloro-2-(2-chloro-3-(6-methoxy-5-((7-oxo-2,6-diazaspiro[3.4]octan-2-yl)methyl)pyridin-2-yl)phenyl)pyridin-4-yl)-2-methoxybenzyl)-2,6-diazaspiro[3.4]octan-7-one;
N-(4-(3-chloro-2-(2-chloro-3-(6-methoxy-5-(((tetrahydro-2H-pyran-4-yl)amino)methyl)pyridin-2-yl)phenyl)pyridin-4-yl)-2-methoxybenzyl)tetrahydro-2H-pyran-4-amine;
(S)-5-(((4-(3-chloro-2-(2-chloro-3-(6-methoxy-5-(((((S)-5-oxopyrrolidin-2-yl)methyl)amino)methyl)pyrid in-2-yl)phenyl)pyrid in-4-yl)-2-methoxybenzyl)amino)methyl)pyrrolidin-2-one;
(S)-5-(((4-(3-chloro-2-(2-chloro-3-(6-methoxy-5-(((((R)-5-oxopyrrolidin-2-yl)methyl)amino)methyl)pyridin-2-yl)phenyl)pyridin-4-yl)-2-methoxybenzyl)amino)methyl)pyrrolidin-2-one;
(R)-5-(((4-(3-chloro-2-(2-chloro-3-(6-methoxy-5-(((((S)-5-oxopyrrolidin-2-yl)methyl)amino)methyl)pyridin-2-yl)phenyl)pyridin-4-yl)-2-methoxybenzyl)amino)methyl)pyrrolidin-2-one;
(R)-5-(((4-(3-chloro-2-(2-chloro-3-(6-methoxy-5-(((((R)-5-oxopyrrolidin-2-yl)methyl)amino)methyl)pyridin-2-yl)phenyl)pyridin-4-yl)-2-methoxybenzyl)amino)methyl)pyrrolidin-2-one;
2-(4-(3-chloro-4-(2-chloro-3-(6-methoxy-5-(((1-(3-methoxypropanoyl)piperidin-4-yl)amino)methyl)pyridin-2-yl)phenyl)pyridin-2-yl)-2-methoxybenzyl)-2,6-diazaspiro[3.4]octan-7-one;
N-((6-(2-chloro-3-(5-chloro-6-(3-methoxy-4-(((tetrahydro-2H-pyran-4-yl)amino)methyl)phenyl)pyrimidin-4-yl)phenyl)-2-methoxypyridin-3-yl)methyl)tetrahydro-2H-pyran-4-amine;
1-(4-(((6-(3-(5'-(((1-acetylpiperidin-4-yl)amino)methyl)-3-chloro-6'-methoxy-[2,2'-bipyridin]-4-yl)-2-chlorophenyl)-2-methoxypyridin-3-yl)methyl)amino)piperidin-1-yl)ethan-1-one;
2-((6-(2-chloro-3-(3-chloro-6'-methoxy-5'-((7-oxo-2,6-diazaspiro[3.4]octan-2-yl)methyl)-[2,2'-bipyridin]-4-yl)phenyl)-2-methoxypyridin-3-yl)methyl)-2,6-diazaspiro[3.4]octan-7-one;
N-((6-(2-chloro-3-(3-chloro-6'-methoxy-5'-(((tetrahydro-2H-pyran-4-yl)amino)methyl)-[2,2'-bipyridin]-4-yl)phenyl)-2-methoxypyridin-3-yl)methyl)tetrahydro-2H-pyran-4-amine;
(S)-5-((((6-(2-chloro-3-(3-chloro-6'-methoxy-5'-(((((S)-5-oxopyrrolidin-2-yl)methyl)amino)methyl)-[2,2'-bipyridin]-4-yl)phenyl)-2-methoxypyridin-3-yl)methyl)amino)methyl)pyrrolidin-2-one;
(S)-5-((((6-(2-chloro-3-(3-chloro-6'-methoxy-5'-(((((R)-5-oxopyrrolidin-2-yl)methyl)amino)methyl)-[2,2'-bipyridin]-4-yl)phenyl)-2-methoxypyridin-3-yl)methyl)amino)methyl)pyrrolidin-2-one;
(R)-5-((((6-(2-chloro-3-(3-chloro-6'-methoxy-5'-(((((S)-5-oxopyrrolidin-2-yl)methyl)amino)methyl)-[2,2'-bipyridin]-4-yl)phenyl)-2-methoxypyridin-3-yl)methyl)amino)methyl)pyrrolidin-2-one;
(R)-5-((((6-(2-chloro-3-(3-chloro-6'-methoxy-5'-(((((R)-5-oxopyrrolidin-2-yl)methyl)amino)methyl)-[2,2'-bipyridin]-4-yl)phenyl)-2-methoxypyridin-3-yl)methyl)amino)methyl)pyrrolidin-2-one;
1-(4-(((6-(3-(2-(4-((6-acetyl-2,6-diazaspiro[3.3]heptan-2-yl)methyl)-3-methoxyphenyl)-3-chloropyridin-4-yl)-2-chlorophenyl)-2-methoxypyridin-3-yl)methyl)amino)piperidin-1-yl)-3-methoxypropan-1-one;
(S)-1-(4-(((6-(2-chloro-3-(3-chloro-2-(4-(((2-hydroxypropyl)amino)methyl)-3-methoxyphenyl)pyridin-4-yl)phenyl)-2-methoxypyridin-3-yl)methyl)amino)piperidin-1-yl)-3-methoxypropan-1-one;
(R)-1-(4-(((6-(2-chloro-3-(3-chloro-2-(4-(((2-hydroxypropyl)amino)methyl)-3-methoxyphenyl)pyridin-4-yl)phenyl)-2-methoxypyridin-3-yl)methyl)amino)piperidin-1-yl)-3-methoxypropan-1-one;
(S)-5-((((2'-((S)-1-((5-(((1-acetylpiperidin-4-yl)amino)methyl)-6-methoxy-3-(trifluoromethyl)pyridin-2-yl)amino)-2,3-dihydro-1H-inden-4-yl)-3'-chloro-6-methoxy-[2,4'-bipyridin]-5-yl)methyl)amino)methyl)pyrrolidin-2-one;
(S)-5-((((2'-((R)-1-((5-(((1-acetylpiperidin-4-yl)amino)methyl)-6-methoxy-3-(trifluoromethyl)pyridin-2-yl)amino)-2,3-dihydro-1H-inden-4-yl)-3'-chloro-6-methoxy-[2,4'-bipyridin]-5-yl)methyl)amino)methyl)pyrrolidin-2-one;
(R)-5-((((2'-((S)-1-((5-(((1-acetylpiperidin-4-yl)amino)methyl)-6-methoxy-3-(trifluoromethyl)pyridin-2-yl)amino)-2,3-dihydro-1H-inden-4-yl)-3'-chloro-6-methoxy-[2,4'-bipyridin]-5-yl)methyl)amino)methyl)pyrrolidin-2-one;
(R)-5-((((2'-((R)-1-((5-(((1-acetylpiperidin-4-yl)amino)methyl)-6-methoxy-3-(trifluoromethyl)pyridin-2-yl)amino)-2,3-dihydro-1H-inden-4-yl)-3'-chloro-6-methoxy-[2,4'-bipyridin]-5-yl)methyl)amino)methyl)pyrrolidin-2-one;
(S)-5-((((3'-chloro-6-methoxy-2'-((S)-1-((6-methoxy-5-(((tetrahydro-2H-pyran-4-yl)amino)methyl)-3-(trifluoromethyl)pyridin-2-yl)amino)-2,3-dihydro-1H-inden-4-yl)-[2,4'-bipyridin]-5-yl)methyl)amino)methyl)pyrrolidin-2-one;
(S)-5-((((3'-chloro-6-methoxy-2'-((R)-1-((6-methoxy-5-(((tetrahydro-2H-pyran-4-yl)amino)methyl)-3-(trifluoromethyl)pyridin-2-yl)amino)-2,3-dihydro-1H-inden-4-yl)-[2,4'-bipyridin]-5-yl)methyl)amino)methyl)pyrrolidin-2-one;
(R)-5-((((3'-chloro-6-methoxy-2'-((S)-1-((6-methoxy-5-(((tetrahydro-2H-pyran-4-yl)amino)methyl)-3-(trifluoromethyl)pyridin-2-yl)amino)-2,3-dihydro-1H-inden-4-yl)-[2,4'-bipyridin]-5-yl)methyl)amino)methyl)pyrrolidin-2-one;
(R)-5-((((3'-chloro-6-methoxy-2'-((R)-1-((6-methoxy-5-(((tetrahydro-2H-pyran-4-yl)amino)methyl)-3-(trifluoromethyl)pyridin-2-yl)amino)-2,3-dihydro-1H-inden-4-yl)-[2,4'-bipyridin]-5-yl)methyl)amino)methyl)pyrrolidin-2-one;
N-((6-(2-chloro-3-(3-chloro-2-(3-methoxy-4-(((tetrahydro-2H-pyran-4-yl)amino)methyl)phenyl)pyridin-4-yl)phenyl)-1-methyl-1H-pyrrolo[2,3-b]pyridin-3-yl)methyl)-N-methyltetrahydro-2H-pyran-4-amine;
2-(2-(4-((6-acetyl-2,6-diazaspiro[3.3]heptan-2-yl)methyl)-3-methoxyphenyl)-3-chloropyridin-4-yl)-6-(5-((6-acetyl-2,6-diazaspiro[3.3]heptan-2-yl)methyl)-6-methoxypyridin-2-yl)benzonitrile;
1-(4-(((6-(2-chloro-3-(3-chloro-2-(2-((3-hydroxy-3-methylazetidin-1-yl)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one;
(R)-1-(4-(((6-(2-chloro-3-(3-chloro-2-(2-(((2-hydroxypropyl)amino)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one;
(S)-1-(4-(((6-(2-chloro-3-(3-chloro-2-(2-(((2-hydroxypropyl)amino)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one;
1-(4-(((6-(3-(5-(((1-acetylpiperidin-4-yl)amino)methyl)-4'-chloro-6-methoxy-[2,3'-bipyridin]-5'-yl)-2-chlorophenyl)-2-methoxypyridin-3-yl)methyl)amino)piperidin-1-yl)ethan-1-one;
2-((6-(2-chloro-3-(4'-chloro-6-methoxy-5-((7-oxo-2,6-diazaspiro[3.4]octan-2-yl)methyl)-[2,3'-bipyridin]-5'-yl)phenyl)-2-methoxypyridin-3-yl)methyl)-2,6-diazaspiro[3.4]octan-7-one;
1-(4-(((6-(3-(5-(4-(((1-acetylpiperidin-4-yl)amino)methyl)-3-methoxyphenyl)-4-chloropyridin-3-yl)-2-chlorophenyl)-2-methoxypyridin-3-yl)methyl)amino)piperidin-1-yl)ethan-1-one;
2-(4-(4-chloro-5-(2-chloro-3-(6-methoxy-5-((7-oxo-2,6-diazaspiro[3.4]octan-2-yl)methyl)pyridin-2-yl)phenyl)pyridin-3-yl)-2-methoxybenzyl)-2,6-diazaspiro[3.4]octan-7-one;
1-(6-(2-chloro-3-(5-chloro-6-(3-methoxy-4-(((oxazol-5-ylmethyl)amino)methyl)phenyl)pyrimidin-4-yl)phenyl)-2-methoxypyridin-3-yl)-N-(oxazol-5-ylmethyl)methanamine;
1-(4-(((6-(2-chloro-3-(3-chloro-2-(2-((isopropylamino)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one;
5-(((1-acetylpiperidin-4-yl)amino)methyl)-N-(3-(2-(4-(((1-acetylpiperidin-4-yl)amino)methyl)-3-methoxyphenyl)-3-chloropyridin-4-yl)-2-methylphenyl)picolinamide;
N-(3-(3-chloro-2-(3-methoxy-4-(((tetrahydro-2H-pyran-4-yl)amino)methyl)phenyl)pyridin-4-yl)-2-methylphenyl)-5-(((tetrahydro-2H-pyran-4-yl)amino)methyl)picolinamide;
N-(3-(3-chloro-2-(3-methoxy-4-(((((S)-5-oxopyrrolidin-2-yl)methyl)amino)methyl)phenyl)pyridin-4-yl)-2-methylphenyl)-5-(((((S)-5-oxopyrrolidin-2-yl)methyl)amino)methyl)picolinamide;
N-(3-(3-chloro-2-(3-methoxy-4-(((((S)-5-oxopyrrolidin-2-yl)methyl)amino)methyl)phenyl)pyridin-4-yl)-2-methylphenyl)-5-(((((R)-5-oxopyrrolidin-2-yl)methyl)amino)methyl)picolinamide;
N-(3-(3-chloro-2-(3-methoxy-4-(((((R)-5-oxopyrrolidin-2-yl)methyl)amino)methyl)phenyl)pyridin-4-yl)-2-methylphenyl)-5-(((((S)-5-oxopyrrolidin-2-yl)methyl)amino)methyl)picolinamide;
N-(3-(3-chloro-2-(3-methoxy-4-(((((R)-5-oxopyrrolidin-2-yl)methyl)amino)methyl)phenyl)pyridin-4-yl)-2-methylphenyl)-5-(((((R)-5-oxopyrrolidin-2-yl)methyl)amino)methyl)picolinamide;
N-(3-(3-chloro-2-(4-(((2-hydroxyethyl)amino)methyl)-3-methoxyphenyl)pyridin-4-yl)-2-methylphenyl)-5-(((2-hydroxyethyl)amino)methyl)picolinamide;
5-((6-acetyl-2,6-diazaspiro[3.3]heptan-2-yl)methyl)-N-(3-(2-(4-((6-acetyl-2,6-diazaspiro[3.3]heptan-2-yl)methyl)-3-methoxyphenyl)-3-chloropyridin-4-yl)-2-methylphenyl)picolinamide;
N-(3-(3-chloro-2-(4-((((S)-2-hydroxypropyl)amino)methyl)-3-methoxyphenyl)pyridin-4-yl)-2-methylphenyl)-5-((((S)-2-hydroxypropyl)amino)methyl)picolinamide;
N-(3-(3-chloro-2-(4-((((S)-2-hydroxypropyl)amino)methyl)-3-methoxyphenyl)pyridin-4-yl)-2-methylphenyl)-5-((((R)-2-hydroxypropyl)amino)methyl)picolinamide;
N-(3-(3-chloro-2-(4-((((R)-2-hydroxypropyl)amino)methyl)-3-methoxyphenyl)pyridin-4-yl)-2-methylphenyl)-5-((((S)-2-hydroxypropyl)amino)methyl)picolinamide;
N-(3-(3-chloro-2-(4-((((R)-2-hydroxypropyl)amino)methyl)-3-methoxyphenyl)pyridin-4-yl)-2-methylphenyl)-5-((((R)-2-hydroxypropyl)amino)methyl)picolinamide;
1-(6-(((6-(3-(2-(4-(((2-acetyl-2-azaspiro[3.3]heptan-6-yl)amino)methyl)-3-methoxyphenyl)-3-chloropyridin-4-yl)-2-(trifluoromethyl)phenyl)-2-methoxypyridin-3-yl)methyl)amino)-2-azaspiro[3.3]heptan-2-yl)ethan-1-one;
2-(3-chloro-2-(3-methoxy-4-(((tetrahydro-2H-pyran-4-yl)amino)methyl)phenyl)pyridin-4-yl)-6-(6-methoxy-5-(((tetrahydro-2H-pyran-4-yl)amino)methyl)pyridin-2-yl)benzonitrile;
2-(3-chloro-2-(3-methoxy-4-((7-oxo-2,6-diazaspiro[3.4]octan-2-yl)methyl)phenyl)pyridin-4-yl)-6-(6-methoxy-5-((7-oxo-2,6-diazaspiro[3.4]octan-2-yl)methyl)pyridin-2-yl)benzonitrile;
1-(4-(((6-(2-chloro-3-(3-chloro-2-(2-(((2-hydroxyethyl)amino)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one;
1-(4-((6-(3-(2-(4-((4-acetylpiperazin-1-yl)methyl)-3-methoxyphenyl)-3-chloropyridin-4-yl)-2-chlorophenyl)-2-methoxypyridin-3-yl)methyl)piperazin-1-yl)ethan-1-one;
N-(3-(2-(4-(((1-acetylpiperidin-4-yl)amino)methyl)-3-methoxyphenyl)-3-chloropyridin-4-yl)-2-chlorophenyl)-1,3-dimethyl-2,4-dioxo-1,2,3,4-tetrahydropyrimidine-5-carboxamide;
(S)-N-(2-chloro-3-(3-chloro-2-(3-methoxy-4-((((5-oxopyrrolidin-2-yl)methyl)amino)methyl)phenyl)pyridin-4-yl)phenyl)-1,3-dimethyl-2,4-dioxo-1,2,3,4-tetrahydropyrimidine-5-carboxamide;
(R)-N-(2-chloro-3-(3-chloro-2-(3-methoxy-4-((((5-oxopyrrolidin-2-yl)methyl)amino)methyl)phenyl)pyridin-4-yl)phenyl)-1,3-dimethyl-2,4-dioxo-1,2,3,4-tetrahydropyrimidine-5-carboxamide;
N-(2-chloro-3-(3-chloro-2-(3-methoxy-4-((7-oxo-2,6-diazaspiro[3.4]octan-2-yl)methyl)phenyl)pyridin-4-yl)phenyl)-1,3-dimethyl-2,4-dioxo-1,2,3,4-tetrahydropyrimidine-5-carboxamide;
2-((6-(4-(3-(5-(((1-acetylpiperidin-4-yl)amino)methyl)-6-methoxypyridin-2-yl)-2-chlorophenyl)-3-chloropyridin-2-yl)-8-methoxy-[1,2,4]triazolo[1,5-a]pyridin-2-yl)methyl)-2,6-diazaspiro[3.4]octan-7-one;
1-(4-(((6-(2-chloro-3-(3-chloro-2-(2-((6-hydroxy-2-azaspiro[3.3]heptan-2-yl)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one;
1-(4-(((6-(2-chloro-3-(3-chloro-2-(2-(((2-hydroxyethyl)amino)methyl)-4-methoxypyrrolo[2,1-f][1,2,4]triazin-6-yl)pyridin-4-yl)phenyl)-2-methoxypyridin-3-yl)methyl)amino)piperidin-1-yl)ethan-1-one;
2-((6-(3-(2-(4-((6-acetyl-2,6-diazaspiro[3.3]heptan-2-yl)methyl)-3-(difluoromethoxy)phenyl)-3-chloropyridin-4-yl)-2-chlorophenyl)-2-methoxypyridin-3-yl)methyl)-2,6-diazaspiro[3.4]octan-7-one;
1-(6-(4-(3-chloro-4-(2-chloro-3-(6-methoxy-5-(((tetrahydro-2H-pyran-4-yl)amino)methyl)pyridin-2-yl)phenyl)pyridin-2-yl)-2-(difluoromethoxy)benzyl)-2,6-diazaspiro[3.3]heptan-2-yl)ethan-1-one;
1-(4-(((6-(3-(2-(4-((6-acetyl-2,6-diazaspiro[3.3]heptan-2-yl)methyl)-3-(difluoromethoxy)phenyl)-3-chloropyridin-4-yl)-2-chlorophenyl)-2-methoxypyridin-3-yl)methyl)amino)piperidin-1-yl)ethan-1-one;
2-((6-(3-(2-(4-(((1-acetylpiperidin-4-yl)amino)methyl)-3-(difluoromethoxy)phenyl)-3-chloropyridin-4-yl)-2-chlorophenyl)-2-methoxypyridin-3-yl)methyl)-2,6-diazaspiro[3.4]octan-7-one;
(S)-5-((((6-(((S)-4-(3'-chloro-6-methoxy-5-(((tetrahydro-2H-pyran-4-yl)amino)methyl)-[2,4'-bipyridin]-2'-yl)-2,3-dihydro-1H-inden-1-yl)amino)-2-methoxy-5-(trifluoromethyl)pyridin-3-yl)methyl)amino)methyl)pyrrolidin-2-one;
(S)-5-((((6-(((R)-4-(3'-chloro-6-methoxy-5-(((tetrahydro-2H-pyran-4-yl)amino)methyl)-[2,4'-bipyridin]-2'-yl)-2,3-dihydro-1H-inden-1-yl)amino)-2-methoxy-5-(trifluoromethyl)pyridin-3-yl)methyl)amino)methyl)pyrrolidin-2-one;
(R)-5-((((6-(((S)-4-(3'-chloro-6-methoxy-5-(((tetrahydro-2H-pyran-4-yl)amino)methyl)-[2,4'-bipyridin]-2'-yl)-2,3-dihydro-1H-inden-1-yl)amino)-2-methoxy-5-(trifluoromethyl)pyridin-3-yl)methyl)amino)methyl)pyrrolidin-2-one;
(R)-5-((((6-(((R)-4-(3'-chloro-6-methoxy-5-(((tetrahydro-2H-pyran-4-yl)amino)methyl)-[2,4'-bipyridin]-2'-yl)-2,3-dihydro-1H-inden-1-yl)amino)-2-methoxy-5-(trifluoromethyl)pyridin-3-yl)methyl)amino)methyl)pyrrolidin-2-one;
1-(6-((6-(2-chloro-3-(3-chloro-2-(4-((6-(2-hydroxyacetyl)-2,6-diazaspiro[3.3]heptan-2-yl)methyl)-3-methoxyphenyl)pyridin-4-yl)phenyl)-2-methoxypyridin-3-yl)methyl)-2,6-diazaspiro[3.3]heptan-2-yl)-2-hydroxyethan-1-one;
1-(4-((4-(5-(((1-acetylpiperidin-4-yl)amino)methyl)-3',3"-dichloro-6-methoxy-[2,4':2',4"-terpyridin]-2"-yl)-2-methoxybenzyl)amino)piperidin-1-yl)ethan-1-one;
2-(4-(3',3"-dichloro-6-methoxy-5-((7-oxo-2,6-diazaspiro[3.4]octan-2-yl)methyl)-[2,4':2',4"-terpyridin]-2"-yl)-2-methoxybenzyl)-2,6-diazaspiro[3.4]octan-7-one;
2-(2-((6-(2-chloro-3-(3-chloro-2-(4-((6-(2-hydroxyethyl)-2,6-diazaspiro[3.4]octan-2-yl)methyl)-3-methoxyphenyl)pyridin-4-yl)phenyl)-2-methoxypyridin-3-yl)methyl)-2,6-diazaspiro[3.4]octan-6-yl)ethan-1-ol;
1-(2-((6-(3-(2-(4-((6-acetyl-2,6-diazaspiro[3.4]octan-2-yl)methyl)-3-methoxyphenyl)-3-chloropyridin-4-yl)-2-chlorophenyl)-2-methoxypyridin-3-yl)methyl)-2,6-diazaspiro[3.4]octan-6-yl)ethan-1-one;
N-(2-chloro-3-(3-chloro-2-(3-methoxy-4-((7-oxo-2,6-diazaspiro[3.4]octan-2-yl)methyl)phenyl)pyridin-4-yl)phenyl)-5-((7-oxo-2,6-diazaspiro[3.4]octan-2-yl)methyl)picolinamide;
5-(((1-acetylpiperidin-4-yl)amino)methyl)-N-(3-(2-(4-(((1-acetylpiperidin-4-yl)amino)methyl)-3-methoxyphenyl)-3-chloropyridin-4-yl)-2-chlorophenyl)picolinamide;
N-(2-chloro-3-(3-chloro-2-(3-methoxy-4-(((tetrahydro-2H-pyran-4-yl)amino)methyl)phenyl)pyridin-4-yl)phenyl)-5-(((tetrahydro-2H-pyran-4-yl)amino)methyl)picolinamide;
N-(2-chloro-3-(3-chloro-2-(3-methoxy-4-(((((S)-5-oxopyrrolidin-2-yl)methyl)amino)methyl)phenyl)pyridin-4-yl)phenyl)-5-(((((S)-5-oxopyrrolidin-2-yl)methyl)amino)methyl)picolinamide;
N-(2-chloro-3-(3-chloro-2-(3-methoxy-4-(((((S)-5-oxopyrrolidin-2-yl)methyl)amino)methyl)phenyl)pyridin-4-yl)phenyl)-5-(((((R)-5-oxopyrrolidin-2-yl)methyl)amino)methyl)picolinamide;
N-(2-chloro-3-(3-chloro-2-(3-methoxy-4-(((((R)-5-oxopyrrolidin-2-yl)methyl)amino)methyl)phenyl)pyridin-4-yl)phenyl)-5-(((((S)-5-oxopyrrolidin-2-yl)methyl)amino)methyl)picolinamide;
N-(2-chloro-3-(3-chloro-2-(3-methoxy-4-(((((R)-5-oxopyrrolidin-2-yl)methyl)amino)methyl)phenyl)pyridin-4-yl)phenyl)-5-(((((R)-5-oxopyrrolidin-2-yl)methyl)amino)methyl)picolinamide;
N-(2-chloro-3-(3-chloro-2-(4-(((2-hydroxyethyl)amino)methyl)-3-methoxyphenyl)pyridin-4-yl)phenyl)-5-(((2-hydroxyethyl)amino)methyl)picolinamide;
5-((6-acetyl-2,6-diazaspiro[3.3]heptan-2-yl)methyl)-N-(3-(2-(4-((6-acetyl-2,6-diazaspiro[3.3]heptan-2-yl)methyl)-3-methoxyphenyl)-3-chloropyridin-4-yl)-2-chlorophenyl)picolinamide;
N-(2-chloro-3-(3-chloro-2-(4-((((S)-2-hydroxypropyl)amino)methyl)-3-methoxyphenyl)pyridin-4-yl)phenyl)-5-((((S)-2-hydroxypropyl)amino)methyl)picolinamide;
N-(2-chloro-3-(3-chloro-2-(4-((((S)-2-hydroxypropyl)amino)methyl)-3-methoxyphenyl)pyridin-4-yl)phenyl)-5-((((R)-2-hydroxypropyl)amino)methyl)picolinamide;
N-(2-chloro-3-(3-chloro-2-(4-((((R)-2-hydroxypropyl)amino)methyl)-3-methoxyphenyl)pyridin-4-yl)phenyl)-5-((((S)-2-hydroxypropyl)amino)methyl)picolinamide;
N-(2-chloro-3-(3-chloro-2-(4-((((R)-2-hydroxypropyl)amino)methyl)-3-methoxyphenyl)pyridin-4-yl)phenyl)-5-((((R)-2-hydroxypropyl)amino)methyl)picolinamide;
1-(6-((6-(2-chloro-3-(3-chloro-2-(3-methoxy-4-((6-(3-methoxypropanoyl)-2,6-diazaspiro[3.3]heptan-2-yl)methyl)phenyl)pyridin-4-yl)phenyl)-2-methoxypyridin-3-yl)methyl)-2,6-diazaspiro[3.3]heptan-2-yl)-3-methoxypropan-1-one;
2-((2'-(3-(5-(((1-acetylpiperidin-4-yl)amino)methyl)-6-methoxypyridin-2-yl)-2-chlorophenyl)-3'-chloro-6-methoxy-[2,4'-bipyridin]-5-yl)methyl)-2,6-diazaspiro[3.4]octan-7-one;
2-((3'-chloro-2'-(2-chloro-3-(5-((4-hydroxypiperidin-1-yl)methyl)-6-methoxypyridin-2-yl)phenyl)-6-methoxy-[2,4'-bipyridin]-5-yl)methyl)-2,6-diazaspiro[3.4]octan-7-one;
2-((6-(3-(5-(((1-acetylpiperidin-4-yl)amino)methyl)-3'-chloro-6-methoxy-[2,4'-bipyridin]-2'-yl)-2-chlorophenyl)-2-methoxypyridin-3-yl)methyl)-2,6-diazaspiro[3.4]octan-7-one;
2-(4-(3-chloro-4-(2-chloro-3-(6-methoxy-5-(((tetrahydro-2H-pyran-4-yl)amino)methyl)pyridin-2-yl)phenyl)pyridin-2-yl)-2-(difluoromethoxy)benzyl)-2,6-diazaspiro[3.4]octan-7-one;
2-(4-(4-(3-(5-(((1-acetylpiperidin-4-yl)amino)methyl)-6-methoxypyridin-2-yl)-2-chlorophenyl)-3-chloropyridin-2-yl)-2-(difluoromethoxy)benzyl)-2,6-diazaspiro[3.4]octan-7-one;
2-((6-(2-chloro-3-(3-chloro-2-(3-(difluoromethoxy)-4-(((tetrahydro-2H-pyran-4-yl)amino)methyl)phenyl)pyridin-4-yl)phenyl)-2-methoxypyridin-3-yl)methyl)-2,6-diazaspiro[3.4]octan-7-one;
(S)-5-((((6-(((S)-4-(3'-chloro-6-methoxy-5-(((tetrahydro-2H-pyran-4-yl)amino)methyl)-[2,4'-bipyridin]-2'-yl)-2,3-dihydro-1H-inden-1-yl)oxy)-2-methoxy-5-(trifluoromethyl)pyridin-3-yl)methyl)amino)methyl)pyrrolidin-2-one;
(R)-5-((((6-(((S)-4-(3'-chloro-6-methoxy-5-(((tetrahydro-2H-pyran-4-yl)amino)methyl)-[2,4'-bipyridin]-2'-yl)-2,3-dihydro-1H-inden-1-yl)oxy)-2-methoxy-5-(trifluoromethyl)pyridin-3-yl)methyl)amino)methyl)pyrrolidin-2-one;
(S)-5-((((6-(((R)-4-(3'-chloro-6-methoxy-5-(((tetrahydro-2H-pyran-4-yl)amino)methyl)-[2,4'-bipyridin]-2'-yl)-2,3-dihydro-1H-inden-1-yl)oxy)-2-methoxy-5-(trifluoromethyl)pyridin-3-yl)methyl)amino)methyl)pyrrolidin-2-one;
(R)-5-((((6-(((R)-4-(3'-chloro-6-methoxy-5-(((tetrahydro-2H-pyran-4-yl)amino)methyl)-[2,4'-bipyridin]-2'-yl)-2,3-dihydro-1H-inden-1-yl)oxy)-2-methoxy-5-(trifluoromethyl)pyridin-3-yl)methyl)amino)methyl)pyrrolidin-2-one;
2-(4-(3-chloro-4-(2-chloro-3-(6-methoxy-5-((6-(2-methoxyacetyl)-2,6-diazaspiro[3.3]heptan-2-yl)methyl)pyridin-2-yl)phenyl)pyridin-2-yl)-2-methoxybenzyl)-2,6-diazaspiro[3.4]octan-7-one;
2-(4-(4-(3-(5-((6-acetyl-2,6-diazaspiro[3.3]heptan-2-yl)methyl)-6-methoxypyridin-2-yl)-2-chlorophenyl)-3-chloropyridin-2-yl)-2-(difluoromethoxy)benzyl)-2,6-diazaspiro[3.4]octan-7-one;
1-(6-((6-(2-chloro-3-(3-chloro-2-(3-(difluoromethoxy)-4-(((tetrahydro-2H-pyran-4-yl)amino)methyl)phenyl)pyridin-4-yl)phenyl)-2-methoxypyridin-3-yl)methyl)-2,6-diazaspiro[3.3]heptan-2-yl)ethan-1-one;
1-(4-((4-(4-(3-(5-((6-acetyl-2,6-diazaspiro[3.3]heptan-2-yl)methyl)-6-methoxypyridin-2-yl)-2-chlorophenyl)-3-chloropyridin-2-yl)-2-(difluoromethoxy)benzyl)amino)piperidin-1-yl)ethan-1-one;
1-(6-((6-(3-(2-(4-((6-acetyl-2,6-diazaspiro[3.3]heptan-2-yl)methyl)-3-methoxyphenyl)-3-chloropyridin-4-yl)-2-chlorophenyl)-2-methoxypyridin-3-yl)methyl)-2,6-diazaspiro[3.3]heptan-2-yl)-2-methoxyethan-1-one;
1-(2-((6-(2-chloro-3-(3-chloro-2-(4-((4-hydroxypiperidin-1-yl)methyl)-3-methoxyphenyl)pyridin-4-yl)phenyl)-2-methoxypyridin-3-yl)methyl)-2,6-diazaspiro[3.4]octan-6-yl)ethan-1-one;
1-(2-(4-(3-chloro-4-(2-chloro-3-(5-((4-hydroxypiperidin-1-yl)methyl)-6-methoxypyridin-2-yl)phenyl)pyridin-2-yl)-2-methoxybenzyl)-2,6-diazaspiro[3.4]octan-6-yl)ethan-1-one;
N-(2-chloro-3-(3-chloro-2-(3-methoxy-4-((methylamino)methyl)phenyl)pyridin-4-yl)phenyl)-5-((methylamino)methyl)picolinamide;
5-(((2-oxaspiro[3.3]heptan-6-yl)amino)methyl)-N-(3-(2-(4-(((2-oxaspiro[3.3]heptan-6-yl)amino)methyl)-3-methoxyphenyl)-3-chloropyridin-4-yl)-2-chlorophenyl)picolinamide;
N-(3-(3-chloro-2-(3-methoxy-4-((methylamino)methyl)phenyl)pyridin-4-yl)-2-methylphenyl)-5-((methylamino)methyl)picolinamide;
(S)-5-((((6-(2-chloro-3-(3-chloro-2-(2-((3-hydroxy-3-methylazetidin-1-yl)methyl)-4-methoxypyrrolo[2,1-f][1,2,4]triazin-6-yl)pyridin-4-yl)phenyl)-2-methoxypyridin-3-yl)methyl)amino)methyl)pyrrolidin-2-one;
(R)-5-((((6-(2-chloro-3-(3-chloro-2-(2-((3-hydroxy-3-methylazetidin-1-yl)methyl)-4-methoxypyrrolo[2,1-f][1,2,4]triazin-6-yl)pyridin-4-yl)phenyl)-2-methoxypyridin-3-yl)methyl)amino)methyl)pyrrolidin-2-one;
1-(4-(((6-(3-(2-(2-((2-oxa-6-azaspiro[3.3]heptan-6-yl)methyl)-8-methoxy-[1,2,4]triazolo[1,5-a]pyridin-6-yl)-3-chloropyridin-4-yl)-2-chlorophenyl)-2-methoxypyridin-3-yl)methyl)amino)piperidin-1-yl)ethan-1-one;
1-(4-(((6-(2-chloro-3-(3-chloro-2-(2-((((1-hydroxycyclopropyl)methyl)amino)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one;
N-(3-(2-(4-(((1-acetylpiperidin-4-yl)amino)methyl)-3-methoxyphenyl)-3-chloropyridin-4-yl)-2-methylphenyl)-5-(((2-hydroxyethyl)amino)methyl)picolinamide;
5-((2-oxa-6-azaspiro[3.3]heptan-6-yl)methyl)-N-(3-(2-(4-((2-oxa-6-azaspiro[3.3]heptan-6-yl)methyl)-3-methoxyphenyl)-3-chloropyridin-4-yl)-2-chlorophenyl)picolinamide;
N-(2-chloro-3-(3-chloro-2-(4-(((3-fluoropropyl)amino)methyl)-3-methoxyphenyl)pyridin-4-yl)phenyl)-5-(((3-fluoropropyl)amino)methyl)picolinamide;
N-(3-(3-chloro-2-(3-methoxy-4-(((tetrahydro-2H-pyran-4-yl)amino)methyl)phenyl)pyridin-4-yl)-2-methylphenyl)-5-(((2-hydroxyethyl)amino)methyl)picolinamide;
1-(((6-(((S)-5-(3'-chloro-6-methoxy-5-(((((S)-5-oxopyrrolidin-2-yl)methyl)amino)methyl)-[2,4'-bipyridin]-2'-yl)-1,2,3,4-tetrahydronaphthalen-1-yl)amino)-2-methoxy-5-(trifluoromethyl)pyridin-3-yl)methyl)amino)cyclopropane-1-carboxylic acid;
1-(((6-(((R)-5-(3'-chloro-6-methoxy-5-(((((S)-5-oxopyrrolidin-2-yl)methyl)amino)methyl)-[2,4'-bipyridin]-2'-yl)-1,2,3,4-tetrahydronaphthalen-1-yl)amino)-2-methoxy-5-(trifluoromethyl)pyridin-3-yl)methyl)amino)cyclopropane-1-carboxylic acid;
1-(((6-(((S)-5-(3'-chloro-6-methoxy-5-(((((R)-5-oxopyrrolidin-2-yl)methyl)amino)methyl)-[2,4'-bipyridin]-2'-yl)-1,2,3,4-tetrahydronaphthalen-1-yl)amino)-2-methoxy-5-(trifluoromethyl)pyridin-3-yl)methyl)amino)cyclopropane-1-carboxylic acid;
1-(((6-(((R)-5-(3'-chloro-6-methoxy-5-(((((R)-5-oxopyrrolidin-2-yl)methyl)amino)methyl)-[2,4'-bipyridin]-2'-yl)-1,2,3,4-tetrahydronaphthalen-1-yl)amino)-2-methoxy-5-(trifluoromethyl)pyridin-3-yl)methyl)amino)cyclopropane-1-carboxylic acid;
1-(((6-(((S)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)cyclopropane-1-carboxylic acid;
1-(((6-(((R)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)cyclopropane-1-carboxylic acid;
1-(((6-(((S)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)cyclopropane-1-carboxylic acid;
1-(((6-(((R)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)cyclopropane-1-carboxylic acid;
5-(((2-acetyl-2-azaspiro[3.3]heptan-6-yl)amino)methyl)-N-(3-(2-(4-(((2-acetyl-2-azaspiro[3.3]heptan-6-yl)amino)methyl)-3-methoxyphenyl)-3-chloropyridin-4-yl)-2-chlorophenyl)picolinamide;
(S)-5-((((3'-chloro-2'-((S)-5-((5-((((S)-2-hydroxypropyl)amino)methyl)-6-methoxy-3-(trifluoromethyl)pyridin-2-yl)amino)-5,6,7,8-tetrahydronaphthalen-1-yl)-6-methoxy-[2,4'-bipyridin]-5-yl)methyl)amino)methyl)pyrrolidin-2-one;
(S)-5-((((3'-chloro-2'-((R)-5-((5-((((S)-2-hydroxypropyl)amino)methyl)-6-methoxy-3-(trifluoromethyl)pyridin-2-yl)amino)-5,6,7,8-tetrahydronaphthalen-1-yl)-6-methoxy-[2,4'-bipyridin]-5-yl)methyl)amino)methyl)pyrrolidin-2-one;
(S)-5-((((3'-chloro-2'-((S)-5-((5-((((R)-2-hydroxypropyl)amino)methyl)-6-methoxy-3-(trifluoromethyl)pyridin-2-yl)amino)-5,6,7,8-tetrahydronaphthalen-1-yl)-6-methoxy-[2,4'-bipyridin]-5-yl)methyl)amino)methyl)pyrrolidin-2-one;
(S)-5-((((3'-chloro-2'-((R)-5-((5-((((R)-2-hydroxypropyl)amino)methyl)-6-methoxy-3-(trifluoromethyl)pyridin-2-yl)amino)-5,6,7,8-tetrahydronaphthalen-1-yl)-6-methoxy-[2,4'-bipyridin]-5-yl)methyl)amino)methyl)pyrrolidin-2-one;
(R)-5-((((3'-chloro-2'-((S)-5-((5-((((S)-2-hydroxypropyl)amino)methyl)-6-methoxy-3-(trifluoromethyl)pyridin-2-yl)amino)-5,6,7,8-tetrahydronaphthalen-1-yl)-6-methoxy-[2,4'-bipyridin]-5-yl)methyl)amino)methyl)pyrrolidin-2-one;
(R)-5-((((3'-chloro-2'-((R)-5-((5-((((S)-2-hydroxypropyl)amino)methyl)-6-methoxy-3-(trifluoromethyl)pyridin-2-yl)amino)-5,6,7,8-tetrahydronaphthalen-1-yl)-6-methoxy-[2,4'-bipyridin]-5-yl)methyl)amino)methyl)pyrrolidin-2-one;
(R)-5-((((3'-chloro-2'-((S)-5-((5-((((R)-2-hydroxypropyl)amino)methyl)-6-methoxy-3-(trifluoromethyl)pyridin-2-yl)amino)-5,6,7,8-tetrahydronaphthalen-1-yl)-6-methoxy-[2,4'-bipyridin]-5-yl)methyl)amino)methyl)pyrrolidin-2-one;
(R)-5-((((3'-chloro-2'-((R)-5-((5-((((R)-2-hydroxypropyl)amino)methyl)-6-methoxy-3-(trifluoromethyl)pyridin-2-yl)amino)-5,6,7,8-tetrahydronaphthalen-1-yl)-6-methoxy-[2,4'-bipyridin]-5-yl)methyl)amino)methyl)pyrrolidin-2-one;
(S)-N-(2-chloro-3-(5-chloro-6-(3-methoxy-4-((((5-oxopyrrolidin-2-yl)methyl)amino)methyl)phenyl)pyrimidin-4-yl)phenyl)-1,3-dimethyl-2,4-dioxo-1,2,3,4-tetrahydropyrimidine-5-carboxamide;
(R)-N-(2-chloro-3-(5-chloro-6-(3-methoxy-4-((((5-oxopyrrolidin-2-yl)methyl)amino)methyl)phenyl)pyrimidin-4-yl)phenyl)-1,3-dimethyl-2,4-dioxo-1,2,3,4-tetrahydropyrimidine-5-carboxamide;
(S)-5-((((3'-chloro-6-methoxy-2'-((S)-5-((6-methoxy-5-(((((S)-5-oxopyrrolidin-2-yl)methyl)amino)methyl)-3-(trifluoromethyl)pyridin-2-yl)amino)-5,6,7,8-tetrahydronaphthalen-1-yl)-[2,4'-bipyridin]-5-yl)methyl)amino)methyl)pyrrolidin-2-one;
(S)-5-((((3'-chloro-6-methoxy-2'-((R)-5-((6-methoxy-5-(((((S)-5-oxopyrrolidin-2-yl)methyl)amino)methyl)-3-(trifluoromethyl)pyridin-2-yl)amino)-5,6,7,8-tetrahydronaphthalen-1-yl)-[2,4'-bipyridin]-5-yl)methyl)amino)methyl)pyrrolidin-2-one;
(S)-5-((((3'-chloro-6-methoxy-2'-((S)-5-((6-methoxy-5-(((((R)-5-oxopyrrolidin-2-yl)methyl)amino)methyl)-3-(trifluoromethyl)pyridin-2-yl)amino)-5,6,7,8-tetrahydronaphthalen-1-yl)-[2,4'-bipyridin]-5-yl)methyl)amino)methyl)pyrrolidin-2-one;
(S)-5-((((3'-chloro-6-methoxy-2'-((R)-5-((6-methoxy-5-(((((R)-5-oxopyrrolidin-2-yl)methyl)amino)methyl)-3-(trifluoromethyl)pyridin-2-yl)amino)-5,6,7,8-tetrahydronaphthalen-1-yl)-[2,4'-bipyridin]-5-yl)methyl)amino)methyl)pyrrolidin-2-one;
(R)-5-((((3'-chloro-6-methoxy-2'-((S)-5-((6-methoxy-5-(((((S)-5-oxopyrrolidin-2-yl)methyl)amino)methyl)-3-(trifluoromethyl)pyridin-2-yl)amino)-5,6,7,8-tetrahydronaphthalen-1-yl)-[2,4'-bipyridin]-5-yl)methyl)amino)methyl)pyrrolidin-2-one;
(R)-5-((((3'-chloro-6-methoxy-2'-((R)-5-((6-methoxy-5-(((((S)-5-oxopyrrolidin-2-yl)methyl)amino)methyl)-3-(trifluoromethyl)pyridin-2-yl)amino)-5,6,7,8-tetrahydronaphthalen-1-yl)-[2,4'-bipyridin]-5-yl)methyl)amino)methyl)pyrrolidin-2-one;
(R)-5-((((3'-chloro-6-methoxy-2'-((S)-5-((6-methoxy-5-(((((R)-5-oxopyrrolidin-2-yl)methyl)amino)methyl)-3-(trifluoromethyl)pyridin-2-yl)amino)-5,6,7,8-tetrahydronaphthalen-1-yl)-[2,4'-bipyridin]-5-yl)methyl)amino)methyl)pyrrolidin-2-one;
(R)-5-((((3'-chloro-6-methoxy-2'-((R)-5-((6-methoxy-5-(((((R)-5-oxopyrrolidin-2-yl)methyl)amino)methyl)-3-(trifluoromethyl)pyridin-2-yl)amino)-5,6,7,8-tetrahydronaphthalen-1-yl)-[2,4'-bipyridin]-5-yl)methyl)amino)methyl)pyrrolidin-2-one;
(S)-5-((((6-(((S)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)methyl)pyrrolidin-2-one;
(S)-5-((((6-(((R)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)methyl)pyrrolidin-2-one;
(S)-5-((((6-(((S)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)methyl)pyrrolidin-2-one;
(S)-5-((((6-(((R)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)methyl)pyrrolidin-2-one;
(R)-5-((((6-(((S)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)methyl)pyrrolidin-2-one;
(R)-5-((((6-(((R)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)methyl)pyrrolidin-2-one;
(R)-5-((((6-(((S)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)methyl)pyrrolidin-2-one;
(R)-5-((((6-(((R)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)amino)methyl)pyrrolidin-2-one;
2-(4-(5-chloro-6-(2-chloro-3-(5-(((2-hydroxyethyl)amino)methyl)-6-methoxypyridin-2-yl)phenyl)pyrimidin-4-yl)-2-methoxybenzyl)-2,6-diazaspiro[3.4]octan-7-one;
(S)-N-(2-chloro-3-(4'-chloro-6-methoxy-5-((((5-oxopyrrolidin-2-yl)methyl)amino)methyl)-[2,3'-bipyridin]-5'-yl)phenyl)-1,3-dimethyl-2,4-dioxo-1,2,3,4-tetrahydropyrimidine-5-carboxamide;
(R)-N-(2-chloro-3-(4'-chloro-6-methoxy-5-((((5-oxopyrrolidin-2-yl)methyl)amino)methyl)-[2,3'-bipyridin]-5'-yl)phenyl)-1,3-dimethyl-2,4-dioxo-1,2,3,4-tetrahydropyrimidine-5-carboxamide;
1-(4-(((6-(2-chloro-3-(3-chloro-2-(2-(((3-fluoropropyl)amino)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one;
1-(4-(((6-(2-chloro-3-(3-chloro-2-(2-(((2-hydroxy-2-methylpropyl)amino)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one;
1-(4-(((6-(2-chloro-3-(3-chloro-2-(2-(((2-hydroxyethyl)(methyl)amino)methyl)-8-methoxy-[1,2,4]triazolo[1,5-a]pyridin-6-yl)pyridin-4-yl)phenyl)-2-methoxypyridin-3-yl)methyl)amino)piperidin-1-yl)ethan-1-one;
(S)-5-((((6-(2-chloro-3-(5-methoxy-6-(3-methoxy-4-(((((S)-5-oxopyrrolidin-2-yl)methyl)amino)methyl)phenyl)pyrimidin-4-yl)phenyl)-2-methoxypyridin-3-yl)methyl)amino)methyl)pyrrolidin-2-one;
(S)-5-((((6-(2-chloro-3-(5-methoxy-6-(3-methoxy-4-(((((R)-5-oxopyrrolidin-2-yl)methyl)amino)methyl)phenyl)pyrimidin-4-yl)phenyl)-2-methoxypyridin-3-yl)methyl)amino)methyl)pyrrolidin-2-one;
(R)-5-((((6-(2-chloro-3-(5-methoxy-6-(3-methoxy-4-(((((S)-5-oxopyrrolidin-2-yl)methyl)amino)methyl)phenyl)pyrimidin-4-yl)phenyl)-2-methoxypyridin-3-yl)methyl)amino)methyl)pyrrolidin-2-one;
(R)-5-((((6-(2-chloro-3-(5-methoxy-6-(3-methoxy-4-(((((R)-5-oxopyrrolidin-2-yl)methyl)amino)methyl)phenyl)pyrimidin-4-yl)phenyl)-2-methoxypyridin-3-yl)methyl)amino)methyl)pyrrolidin-2-one;
1-(4-(((6-(3-(6-(4-(((1-acetylpiperidin-4-yl)amino)methyl)-3-methoxyphenyl)-5-methoxypyrimidin-4-yl)-2-chlorophenyl)-2-methoxypyridin-3-yl)methyl)amino)piperidin-1-yl)ethan-1-one;
(S)-N-(2-chloro-3-(3'-chloro-6-methoxy-5-((((5-oxopyrrolidin-2-yl)methyl)amino)methyl)-[2,4'-bipyridin]-2'-yl)phenyl)-1,3-dimethyl-2,4-dioxo-1,2,3,4-tetrahydropyrimidine-5-carboxamide;
(R)-N-(2-chloro-3-(3'-chloro-6-methoxy-5-((((5-oxopyrrolidin-2-yl)methyl)amino)methyl)-[2,4'-bipyridin]-2'-yl)phenyl)-1,3-dimethyl-2,4-dioxo-1,2,3,4-tetrahydropyrimidine-5-carboxamide;
N-(2-chloro-3-(3'-chloro-6-methoxy-5-((7-oxo-2,6-diazaspiro[3.4]octan-2-yl)methyl)-[2,4'-bipyridin]-2'-yl)phenyl)-1,3-dimethyl-2,4-dioxo-1,2,3,4-tetrahydropyrimidine-5-carboxamide;
2-((6-(((S)-5-(3'-chloro-6-methoxy-5-(((((S)-5-oxopyrrolidin-2-yl)methyl)amino)methyl)-[2,4'-bipyridin]-2'-yl)-1,2,3,4-tetrahydronaphthalen-1-yl)amino)-2-methoxy-5-(trifluoromethyl)pyridin-3-yl)methyl)-2,6-diazaspiro[3.4]octan-7-one;
2-((6-(((R)-5-(3'-chloro-6-methoxy-5-(((((S)-5-oxopyrrolidin-2-yl)methyl)amino)methyl)-[2,4'-bipyridin]-2'-yl)-1,2,3,4-tetrahydronaphthalen-1-yl)amino)-2-methoxy-5-(trifluoromethyl)pyridin-3-yl)methyl)-2,6-diazaspiro[3.4]octan-7-one;
2-((6-(((S)-5-(3'-chloro-6-methoxy-5-(((((R)-5-oxopyrrolidin-2-yl)methyl)amino)methyl)-[2,4'-bipyridin]-2'-yl)-1,2,3,4-tetrahydronaphthalen-1-yl)amino)-2-methoxy-5-(trifluoromethyl)pyridin-3-yl)methyl)-2,6-diazaspiro[3.4]octan-7-one;
2-((6-(((R)-5-(3'-chloro-6-methoxy-5-(((((R)-5-oxopyrrolidin-2-yl)methyl)amino)methyl)-[2,4'-bipyridin]-2'-yl)-1,2,3,4-tetrahydronaphthalen-1-yl)amino)-2-methoxy-5-(trifluoromethyl)pyridin-3-yl)methyl)-2,6-diazaspiro[3.4]octan-7-one;
N-(3-(5-(((1-acetylpiperidin-4-yl)amino)methyl)-3'-chloro-6-methoxy-[2,4'-bipyridin]-2'-yl)-2-chlorophenyl)-1,3-dimethyl-2,4-dioxo-1,2,3,4-tetrahydropyrimidine-5-carboxamide;
N-(2-chloro-3-(4'-chloro-6-methoxy-5-((7-oxo-2,6-diazaspiro[3.4]octan-2-yl)methyl)-[2,3'-bipyridin]-5'-yl)phenyl)-1,3-dimethyl-2,4-dioxo-1,2,3,4-tetrahydropyrimidine-5-carboxamide;
N-(3-(5-(((1-acetylpiperidin-4-yl)amino)methyl)-4'-chloro-6-methoxy-[2,3'-bipyridin]-5'-yl)-2-chlorophenyl)-1,3-dimethyl-2,4-dioxo-1,2,3,4-tetrahydropyrimidine-5-carboxamide;
2-((6-(((S)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)-2,6-diazaspiro[3.4]octan-7-one;
2-((6-(((R)-5-(3-chloro-2-(3-methoxy-4-(((((S)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)-2,6-diazaspiro[3.4]octan-7-one;
2-((6-(((S)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)-2,6-diazaspiro[3.4]octan-7-one;
2-((6-(((R)-5-(3-chloro-2-(3-methoxy-4-(((((R)-5-oxopyrrolidin-2-yl)methyl)amino)methyl)phenyl)pyridin-4-yl)-1,2,3,4-tetrahydronaphthalen-1-yl)amino)-2-methoxy-5-(trifluoromethyl)pyridin-3-yl)methyl)-2,6-diazaspiro[3.4]octan-7-one;
N-(3-(3-chloro-2-(3-(difluoromethoxy)-4-(((((S)-5-oxopyrrolidin-2-yl)methyl)amino)methyl)phenyl)pyridin-4-yl)-2-methylphenyl)-5-(((((S)-5-oxopyrrolidin-2-yl)methyl)amino)methyl)picolinamide;
N-(3-(3-chloro-2-(3-(difluoromethoxy)-4-(((((R)-5-oxopyrrolidin-2-yl)methyl)amino)methyl)phenyl)pyridin-4-yl)-2-methylphenyl)-5-(((((S)-5-oxopyrrolidin-2-yl)methyl)amino)methyl)picolinamide;
N-(3-(3-chloro-2-(3-(difluoromethoxy)-4-(((((S)-5-oxopyrrolidin-2-yl)methyl)amino)methyl)phenyl)pyridin-4-yl)-2-methylphenyl)-5-(((((R)-5-oxopyrrolidin-2-yl)methyl)amino)methyl)picolinamide;
N-(3-(3-chloro-2-(3-(difluoromethoxy)-4-(((((R)-5-oxopyrrolidin-2-yl)methyl)amino)methyl)phenyl)pyridin-4-yl)-2-methylphenyl)-5-(((((R)-5-oxopyrrolidin-2-yl)methyl)amino)methyl)picolinamide;
N-(3-(3-chloro-2-(3-fluoro-5-methoxy-4-(((((S)-5-oxopyrrolidin-2-yl)methyl)amino)methyl)phenyl)pyridin-4-yl)-2-methylphenyl)-5-(((((S)-5-oxopyrrolidin-2-yl)methyl)amino)methyl)picolinamide;
N-(3-(3-chloro-2-(3-fluoro-5-methoxy-4-(((((R)-5-oxopyrrolidin-2-yl)methyl)amino)methyl)phenyl)pyridin-4-yl)-2-methylphenyl)-5-(((((S)-5-oxopyrrolidin-2-yl)methyl)amino)methyl)picolinamide;
N-(3-(3-chloro-2-(3-fluoro-5-methoxy-4-(((((S)-5-oxopyrrolidin-2-yl)methyl)amino)methyl)phenyl)pyridin-4-yl)-2-methylphenyl)-5-(((((R)-5-oxopyrrolidin-2-yl)methyl)amino)methyl)picolinamide;
N-(3-(3-chloro-2-(3-fluoro-5-methoxy-4-(((((R)-5-oxopyrrolidin-2-yl)methyl)amino)methyl)phenyl)pyridin-4-yl)-2-methylphenyl)-5-(((((R)-5-oxopyrrolidin-2-yl)methyl)amino)methyl)picolinamide;
2-(4-(6-(2-chloro-3-(6-methoxy-5-((7-oxo-2,6-diazaspiro[3.4]octan-2-yl)methyl)pyridin-2-yl)phenyl)-5-methoxypyrimidin-4-yl)-2-methoxybenzyl)-2,6-diazaspiro[3.4]octan-7-one;
2-((4-(6-(2-chloro-3-(5-(((2-hydroxyethyl)amino)methyl)-6-methoxypyridin-2-yl)phenyl)-5-methoxypyrimidin-4-yl)-2-methoxybenzyl)amino)ethan-1-ol;
(S)-N-(2-chloro-3-(4'-chloro-5-(((2-hydroxypropyl)amino)methyl)-6-methoxy-[2,3'-bipyridin]-5'-yl)phenyl)-1,3-dimethyl-2,4-dioxo-1,2,3,4-tetrahydropyrimidine-5-carboxamide;
(R)-N-(2-chloro-3-(4'-chloro-5-(((2-hydroxypropyl)amino)methyl)-6-methoxy-[2,3'-bipyridin]-5'-yl)phenyl)-1,3-dimethyl-2,4-dioxo-1,2,3,4-tetrahydropyrimidine-5-carboxamide;
N-(3-(5-((6-acetyl-2,6-diazaspiro[3.3]heptan-2-yl)methyl)-4'-chloro-6-methoxy-[2,3'-bipyridin]-5'-yl)-2-chlorophenyl)-1,3-dimethyl-2,4-dioxo-1,2,3,4-tetrahydropyrimidine-5-carboxamide;
N-(3-(5-((6-acetyl-2,6-diazaspiro[3.3]heptan-2-yl)methyl)-3'-chloro-6-methoxy-[2,4'-bipyridin]-2'-yl)-2-chlorophenyl)-1,3-dimethyl-2,4-dioxo-1,2,3,4-tetrahydropyrimidine-5-carboxamide;
(S)-N-(5-(3'-chloro-6-methoxy-5-((methylamino)methyl)-[2,4'-bipyridin]-2'-yl)-1,2,3,4-tetrahydronaphthalen-1-yl)-6-methoxy-5-((methylamino)methyl)-3-(trifluoromethyl)pyridin-2-amine;
(R)-N-(5-(3'-chloro-6-methoxy-5-((methylamino)methyl)-[2,4'-bipyridin]-2'-yl)-1,2,3,4-tetrahydronaphthalen-1-yl)-6-methoxy-5-((methylamino)methyl)-3-(trifluoromethyl)pyridin-2-amine;
N-(3-(2-(4-(((1-acetylpiperidin-4-yl)amino)methyl)-3-methoxyphenyl)-3-chloropyridin-4-yl)-2-chlorophenyl)-5-(((2-hydroxyethyl)amino)methyl)picolinamide;
N-((6-(2-chloro-3-(5-methoxy-6-(3-methoxy-4-(((tetrahydro-2H-pyran-4-yl)amino)methyl)phenyl)pyrimidin-4-yl)phenyl)-2-methoxypyridin-3-yl)methyl)tetrahydro-2H-pyran-4-amine;
(S)-N-(3-(3-chloro-2-(3-methoxy-4-((((5-oxopyrrolidin-2-yl)methyl)amino)methyl)phenyl)pyridin-4-yl)-2-methylphenyl)-5-(((2-hydroxyethyl)amino)methyl)picolinamide;
(R)-N-(3-(3-chloro-2-(3-methoxy-4-((((5-oxopyrrolidin-2-yl)methyl)amino)methyl)phenyl)pyridin-4-yl)-2-methylphenyl)-5-(((2-hydroxyethyl)amino)methyl)picolinamide;
(S)-5-(((4-(3-chloro-4-((S)-5-((5-((((S)-2-hydroxypropyl)amino)methyl)-6-methoxy-3-(trifluoromethyl)pyridin-2-yl)amino)-5,6,7,8-tetrahydronaphthalen-1-yl)pyridin-2-yl)-2-methoxybenzyl)amino)methyl)pyrrolidin-2-one;
(S)-5-(((4-(3-chloro-4-((R)-5-((5-((((S)-2-hydroxypropyl)amino)methyl)-6-methoxy-3-(trifluoromethyl)pyridin-2-yl)amino)-5,6,7,8-tetrahydronaphthalen-1-yl)pyridin-2-yl)-2-methoxybenzyl)amino)methyl)pyrrolidin-2-one;
(S)-5-(((4-(3-chloro-4-((S)-5-((5-((((R)-2-hydroxypropyl)amino)methyl)-6-methoxy-3-(trifluoromethyl)pyridin-2-yl)amino)-5,6,7,8-tetrahydronaphthalen-1-yl)pyridin-2-yl)-2-methoxybenzyl)amino)methyl)pyrrolidin-2-one;
(S)-5-(((4-(3-chloro-4-((R)-5-((5-((((R)-2-hydroxypropyl)amino)methyl)-6-methoxy-3-(trifluoromethyl)pyridin-2-yl)amino)-5,6,7,8-tetrahydronaphthalen-1-yl)pyridin-2-yl)-2-methoxybenzyl)amino)methyl)pyrrolidin-2-one;
(R)-5-(((4-(3-chloro-4-((S)-5-((5-((((S)-2-hydroxypropyl)amino)methyl)-6-methoxy-3-(trifluoromethyl)pyridin-2-yl)amino)-5,6,7,8-tetrahydronaphthalen-1-yl)pyridin-2-yl)-2-methoxybenzyl)amino)methyl)pyrrolidin-2-one;
(R)-5-(((4-(3-chloro-4-((R)-5-((5-((((S)-2-hydroxypropyl)amino)methyl)-6-methoxy-3-(trifluoromethyl)pyridin-2-yl)amino)-5,6,7,8-tetrahydronaphthalen-1-yl)pyridin-2-yl)-2-methoxybenzyl)amino)methyl)pyrrolidin-2-one;
(R)-5-(((4-(3-chloro-4-((S)-5-((5-((((R)-2-hydroxypropyl)amino)methyl)-6-methoxy-3-(trifluoromethyl)pyridin-2-yl)amino)-5,6,7,8-tetrahydronaphthalen-1-yl)pyridin-2-yl)-2-methoxybenzyl)amino)methyl)pyrrolidin-2-one;
(R)-5-(((4-(3-chloro-4-((R)-5-((5-((((R)-2-hydroxypropyl)amino)methyl)-6-methoxy-3-(trifluoromethyl)pyridin-2-yl)amino)-5,6,7,8-tetrahydronaphthalen-1-yl)pyridin-2-yl)-2-methoxybenzyl)amino)methyl)pyrrolidin-2-one;
2-(4-(5-chloro-6-(2-chloro-3-(5-((((1s,3s)-3-hydroxycyclobutyl)amino)methyl)-6-methoxypyridin-2-yl)phenyl)pyrimidin-4-yl)-2-methoxybenzyl)-2,6-diazaspiro[3.4]octan-7-one;
2-(4-(5-chloro-6-(2-chloro-3-(5-((((1s,3r)-3-hydroxycyclobutyl)amino)methyl)-6-methoxypyridin-2-yl)phenyl)pyrimidin-4-yl)-2-methoxybenzyl)-2,6-diazaspiro[3.4]octan-7-one;
2-(4-(5-chloro-6-(2-chloro-3-(5-((((1r,3s)-3-hydroxycyclobutyl)amino)methyl)-6-methoxypyridin-2-yl)phenyl)pyrimidin-4-yl)-2-methoxybenzyl)-2,6-diazaspiro[3.4]octan-7-one;
2-(4-(5-chloro-6-(2-chloro-3-(5-((((1r,3r)-3-hydroxycyclobutyl)amino)methyl)-6-methoxypyridin-2-yl)phenyl)pyrimidin-4-yl)-2-methoxybenzyl)-2,6-diazaspiro[3.4]octan-7-one;
(S)-N-(2-chloro-3-(3-chloro-6'-methoxy-5'-((((5-oxopyrrolidin-2-yl)methyl)amino)methyl)-[2,2'-bipyridin]-4-yl)phenyl)-1,3-dimethyl-2,4-dioxo-1,2,3,4-tetrahydropyrimidine-5-carboxamide;
(R)-N-(2-chloro-3-(3-chloro-6'-methoxy-5'-((((5-oxopyrrolidin-2-yl)methyl)amino)methyl)-[2,2'-bipyridin]-4-yl)phenyl)-1,3-dimethyl-2,4-dioxo-1,2,3,4-tetrahydropyrimidine-5-carboxamide;
(S)-N-(2-chloro-3-(3-chloro-5'-(((2-hydroxypropyl)amino)methyl)-6'-methoxy-[2,2'-bipyridin]-4-yl)phenyl)-1,3-dimethyl-2,4-dioxo-1,2,3,4-tetrahydropyrimidine-5-carboxamide;
(R)-N-(2-chloro-3-(3-chloro-5'-(((2-hydroxypropyl)amino)methyl)-6'-methoxy-[2,2'-bipyridin]-4-yl)phenyl)-1,3-dimethyl-2,4-dioxo-1,2,3,4-tetrahydropyrimidine-5-carboxamide;
(S)-N-(5-(3-chloro-2-(3-methoxy-4-((methylamino)methyl)phenyl)pyridin-4-yl)-1,2,3,4-tetrahydronaphthalen-1-yl)-6-methoxy-5-((methylamino)methyl)-3-(trifluoromethyl)pyridin-2-amine;
(R)-N-(5-(3-chloro-2-(3-methoxy-4-((methylamino)methyl)phenyl)pyridin-4-yl)-1,2,3,4-tetrahydronaphthalen-1-yl)-6-methoxy-5-((methylamino)methyl)-3-(trifluoromethyl)pyridin-2-amine;
(S)-2-(4-(5-chloro-6-(2-chloro-3-(6-methoxy-5-((((5-oxopyrrolidin-2-yl)methyl)amino)methyl)pyridin-2-yl)phenyl)pyrimidin-4-yl)-2-methoxybenzyl)-2,6-diazaspiro[3.4]octan-7-one;
(R)-2-(4-(5-chloro-6-(2-chloro-3-(6-methoxy-5-((((5-oxopyrrolidin-2-yl)methyl)amino)methyl)pyridin-2-yl)phenyl)pyrimidin-4-yl)-2-methoxybenzyl)-2,6-diazaspiro[3.4]octan-7-one;
2-(4-(6-(2-chloro-3-(6-methoxy-5-((6-oxo-2,5-diazaspiro[3.4]octan-2-yl)methyl)pyridin-2-yl)phenyl)-5-methoxypyrimidin-4-yl)-2-methoxybenzyl)-2,5-diazaspiro[3.4]octan-6-one;
N-(3-(3-chloro-2-(3-methoxy-4-((7-oxo-2,6-diazaspiro[3.4]octan-2-yl)methyl)phenyl)pyridin-4-yl)-2-methylphenyl)-5-(((2-hydroxyethyl)amino)methyl)picolinamide;
(S)-N-(2-chloro-3-(3'-chloro-5-(((2-hydroxypropyl)amino)methyl)-6-methoxy-[2,4'-bipyridin]-2'-yl)phenyl)-1,3-dimethyl-2,4-dioxo-1,2,3,4-tetrahydropyrimidine-5-carboxamide;
(R)-N-(2-chloro-3-(3'-chloro-5-(((2-hydroxypropyl)amino)methyl)-6-methoxy-[2,4'-bipyridin]-2'-yl)phenyl)-1,3-dimethyl-2,4-dioxo-1,2,3,4-tetrahydropyrimidine-5-carboxamide;
(S)-N-(2-chloro-3-(5-chloro-6-(4-(((2-hydroxypropyl)amino)methyl)-3-methoxyphenyl)pyrimidin-4-yl)phenyl)-1,3-dimethyl-2,4-dioxo-1,2,3,4-tetrahydropyrimidine-5-carboxamide;
(R)-N-(2-chloro-3-(5-chloro-6-(4-(((2-hydroxypropyl)amino)methyl)-3-methoxyphenyl)pyrimidin-4-yl)phenyl)-1,3-dimethyl-2,4-dioxo-1,2,3,4-tetrahydropyrimidine-5-carboxamide;
(S)-N-(3-(2-(4-(((1-acetylpiperidin-4-yl)amino)methyl)-3-methoxyphenyl)-3-chloropyridin-4-yl)-2-methylphenyl)-5-((((5-oxopyrrolidin-2-yl)methyl)amino)methyl)picolinamide;
(R)-N-(3-(2-(4-(((1-acetylpiperidin-4-yl)amino)methyl)-3-methoxyphenyl)-3-chloropyridin-4-yl)-2-methylphenyl)-5-((((5-oxopyrrolidin-2-yl)methyl)amino)methyl)picolinamide;
(S)-N-(3-(3-chloro-2-(3-methoxy-4-(((tetrahydro-2H-pyran-4-yl)amino)methyl)phenyl)pyridin-4-yl)-2-methylphenyl)-5-((((5-oxopyrrolidin-2-yl)methyl)amino)methyl)picolinamide;
(R)-N-(3-(3-chloro-2-(3-methoxy-4-(((tetrahydro-2H-pyran-4-yl)amino)methyl)phenyl)pyridin-4-yl)-2-methylphenyl)-5-((((5-oxopyrrolidin-2-yl)methyl)amino)methyl)picolinamide;
(S)-N-(3-(3-chloro-2-(4-(((3-fluoropropyl)amino)methyl)-3-methoxyphenyl)pyridin-4-yl)-2-methylphenyl)-5-((((5-oxopyrrolidin-2-yl)methyl)amino)methyl)picolinamide;
(R)-N-(3-(3-chloro-2-(4-(((3-fluoropropyl)amino)methyl)-3-methoxyphenyl)pyridin-4-yl)-2-methylphenyl)-5-((((5-oxopyrrolidin-2-yl)methyl)amino)methyl)picolinamide;
(S)-N-(3-(3-chloro-2-(4-(((2-hydroxyethyl)amino)methyl)-3-methoxyphenyl)pyridin-4-yl)-2-methylphenyl)-5-((((5-oxopyrrolidin-2-yl)methyl)amino)methyl)picolinamide;
(R)-N-(3-(3-chloro-2-(4-(((2-hydroxyethyl)amino)methyl)-3-methoxyphenyl)pyridin-4-yl)-2-methylphenyl)-5-((((5-oxopyrrolidin-2-yl)methyl)amino)methyl)picolinamide;
(S)-N-(2-chloro-3-(3-chloro-2-(3-methoxy-4-((((5-oxopyrrolidin-2-yl)methyl)amino)methyl)phenyl)pyridin-4-yl)phenyl)-5-((methylamino)methyl)picolinamide;
(R)-N-(2-chloro-3-(3-chloro-2-(3-methoxy-4-((((5-oxopyrrolidin-2-yl)methyl)amino)methyl)phenyl)pyridin-4-yl)phenyl)-5-((methylamino)methyl)picolinamide;
(S)-6-((2-chloro-3-(3-chloro-2-(3-methoxy-4-((((5-oxopyrrolidin-2-yl)methyl)amino)methyl)phenyl)pyridin-4-yl)phenyl)carbamoyl)nicotinic acid;
(R)-6-((2-chloro-3-(3-chloro-2-(3-methoxy-4-((((5-oxopyrrolidin-2-yl)methyl)amino)methyl)phenyl)pyridin-4-yl)phenyl)carbamoyl)nicotinic acid;
2-((6-(3-chloro-4-(2-chloro-3-(5-((isopropylamino)methyl)-6-methoxypyridin-2-yl)phenyl)pyridin-2-yl)-8-methoxy-[1,2,4]triazolo[1,5-a]pyridin-2-yl)methyl)-2-azaspiro[3.3]heptan-6-ol;
N-(2-chloro-3-(3-chloro-2-(3-methoxy-4-((7-oxo-2,6-diazaspiro[3.4]octan-2-yl)methyl)phenyl)pyridin-4-yl)phenyl)-5-(((2-hydroxyethyl)amino)methyl)picolinamide;
(S)-N-(2-chloro-3-(3-chloro-2-(3-methoxy-4-((((5-oxopyrrolidin-2-yl)methyl)amino)methyl)phenyl)pyridin-4-yl)phenyl)-5-(((2-hydroxyethyl)amino)methyl)picolinamide;
(R)-N-(2-chloro-3-(3-chloro-2-(3-methoxy-4-((((5-oxopyrrolidin-2-yl)methyl)amino)methyl)phenyl)pyridin-4-yl)phenyl)-5-(((2-hydroxyethyl)amino)methyl)picolinamide;
(S)-N-(3-(3-chloro-2-(3-methoxy-4-((((5-oxopyrrolidin-2-yl)methyl)amino)methyl)phenyl)pyridin-4-yl)-2-methylphenyl)-5-((methylamino)methyl)picolinamide;
(R)-N-(3-(3-chloro-2-(3-methoxy-4-((((5-oxopyrrolidin-2-yl)methyl)amino)methyl)phenyl)pyridin-4-yl)-2-methylphenyl)-5-((methylamino)methyl)picolinamide;
N-(3-(2-(4-((6-acetyl-2,6-diazaspiro[3.3]heptan-2-yl)methyl)-3-methoxyphenyl)-3-chloropyridin-4-yl)-2-chlorophenyl)-5-(((2-hydroxyethyl)amino)methyl)picolinamide;
(1r,4r)-4-(((6-(3-chloro-4-(2-chloro-3-(5-((isopropylamino)methyl)-6-methoxypyridin-2-yl)phenyl)pyridin-2-yl)-8-methoxy-[1,2,4]triazolo[1,5-a]pyridin-2-yl)methyl)amino)cyclohexan-1-ol;
(1s,4r)-4-(((6-(3-chloro-4-(2-chloro-3-(5-((isopropylamino)methyl)-6-methoxypyridin-2-yl)phenyl)pyridin-2-yl)-8-methoxy-[1,2,4]triazolo[1,5-a]pyridin-2-yl)methyl)amino)cyclohexan-1-ol;
(1r,4s)-4-(((6-(3-chloro-4-(2-chloro-3-(5-((isopropylamino)methyl)-6-methoxypyridin-2-yl)phenyl)pyridin-2-yl)-8-methoxy-[1,2,4]triazolo[1,5-a]pyridin-2-yl)methyl)amino)cyclohexan-1-ol;
(1s,4s)-4-(((6-(3-chloro-4-(2-chloro-3-(5-((isopropylamino)methyl)-6-methoxypyridin-2-yl)phenyl)pyridin-2-yl)-8-methoxy-[1,2,4]triazolo[1,5-a]pyridin-2-yl)methyl)amino)cyclohexan-1-ol;
1-((3'-chloro-2'-(2-chloro-3-(5-((4-hydroxypiperidin-1-yl)methyl)-6-methoxypyridin-2-yl)phenyl)-6-methoxy-[2,4'-bipyridin]-5-yl)methyl)piperidin-4-ol;
1-(2-((6-(3-(5-((6-acetyl-2,6-diazaspiro[3.4]octan-2-yl)methyl)-3'-chloro-6-methoxy-[2,4'-bipyridin]-2'-yl)-2-chlorophenyl)-2-methoxypyridin-3-yl)methyl)-2,6-diazaspiro[3.4]octan-6-yl)ethan-1-one;
1-(4-(((6-(2-chloro-3-(3'-chloro-5-((4-hydroxypiperidin-1-yl)methyl)-6-methoxy-[2,4'-bipyridin]-2'-yl)phenyl)-2-methoxypyridin-3-yl)methyl)amino)piperidin-1-yl)ethan-1-one;
N-(3-(3-chloro-2-(3-fluoro-4-(((2-hydroxyethyl)amino)methyl)-5-methoxyphenyl)pyridin-4-yl)-2-methylphenyl)-5-(((2-hydroxyethyl)amino)methyl)picolinamide;
N-((6-(2-chloro-3-(3-chloro-2-(2-((isopropylamino)methyl)-8-methoxy-[1,2,4]triazolo[1,5-a]pyridin-6-yl)pyridin-4-yl)phenyl)-2-methoxypyridin-3-yl)methyl)propan-2-amine;
(S)-5-((((6-(3-chloro-4-(2-chloro-3-(5-((isopropylamino)methyl)-6-methoxypyridin-2-yl)phenyl)pyridin-2-yl)-8-methoxy-[1,2,4]triazolo[1,5-a]pyridin-2-yl)methyl)amino)methyl)pyrrolidin-2-one;
(R)-5-((((6-(3-chloro-4-(2-chloro-3-(5-((isopropylamino)methyl)-6-methoxypyridin-2-yl)phenyl)pyridin-2-yl)-8-methoxy-[1,2,4]triazolo[1,5-a]pyridin-2-yl)methyl)amino)methyl)pyrrolidin-2-one;
1-(4-(((6-(3-chloro-4-(2-chloro-3-(5-((isopropylamino)methyl)-6-methoxypyridin-2-yl)phenyl)pyridin-2-yl)-8-methoxy-[1,2,4]triazolo[1,5-a]pyridin-2-yl)methyl)amino)piperidin-1-yl)ethan-1-one;
N-(3-(3'-chloro-6-methoxy-5-(((tetrahydro-2H-pyran-4-yl)amino)methyl)-[2,4'-bipyridin]-2'-yl)-2-methylphenyl)-5-(((2-hydroxyethyl)amino)methyl)picolinamide;
1-(6-((6-(3-chloro-4-(2-chloro-3-(5-((isopropylamino)methyl)-6-methoxypyridin-2-yl)phenyl)pyridin-2-yl)-8-methoxy-[1,2,4]triazolo[1,5-a]pyridin-2-yl)methyl)-2,6-diazaspiro[3.3]heptan-2-yl)ethan-1-one;
N-(2-chloro-3-(3-chloro-2-(3-methoxy-4-(((tetrahydro-2H-pyran-4-yl)amino)methyl)phenyl)pyridin-4-yl)phenyl)-5-(((2-hydroxyethyl)amino)methyl)picolinamide;
N-(3-(5-(((1-acetylpiperidin-4-yl)amino)methyl)-3'-chloro-6-methoxy-[2,4'-bipyridin]-2'-yl)-2-methylphenyl)-5-(((2-hydroxyethyl)amino)methyl)picolinamide;
(S)-N-(3-(3-chloro-2-(3-(difluoromethoxy)-4-((((5-oxopyrrolidin-2-yl)methyl)amino)methyl)phenyl)pyridin-4-yl)-2-methylphenyl)-5-(((2-hydroxyethyl)amino)methyl)picolinamide;
(R)-N-(3-(3-chloro-2-(3-(difluoromethoxy)-4-((((5-oxopyrrolidin-2-yl)methyl)amino)methyl)phenyl)pyridin-4-yl)-2-methylphenyl)-5-(((2-hydroxyethyl)amino)methyl)picolinamide;
(S)-N-(3-(3'-chloro-6-methoxy-5-((((5-oxopyrrolidin-2-yl)methyl)amino)methyl)-[2,4'-bipyridin]-2'-yl)-2-methylphenyl)-5-(((2-hydroxyethyl)amino)methyl)picolinamide;
(R)-N-(3-(3'-chloro-6-methoxy-5-((((5-oxopyrrolidin-2-yl)methyl)amino)methyl)-[2,4'-bipyridin]-2'-yl)-2-methylphenyl)-5-(((2-hydroxyethyl)amino)methyl)picolinamide;
(R)-1-((3'-chloro-2'-(2-chloro-3-(5-(((R)-3-hydroxypyrrolidin-1-yl)methyl)-6-methoxypyridin-2-yl)phenyl)-6-methoxy-[2,4'-bipyridin]-5-yl)methyl)pyrrolidin-3-ol;
(R)-1-((3'-chloro-2'-(2-chloro-3-(5-(((S)-3-hydroxypyrrolidin-1-yl)methyl)-6-methoxypyridin-2-yl)phenyl)-6-methoxy-[2,4'-bipyridin]-5-yl)methyl)pyrrolidin-3-ol;
(S)-1-((3'-chloro-2'-(2-chloro-3-(5-(((R)-3-hydroxypyrrolidin-1-yl)methyl)-6-methoxypyridin-2-yl)phenyl)-6-methoxy-[2,4'-bipyridin]-5-yl)methyl)pyrrolidin-3-ol;
(S)-1-((3'-chloro-2'-(2-chloro-3-(5-(((S)-3-hydroxypyrrolidin-1-yl)methyl)-6-methoxypyridin-2-yl)phenyl)-6-methoxy-[2,4'-bipyridin]-5-yl)methyl)pyrrolidin-3-ol; and
1-(4-(((3'-chloro-2'-(2-chloro-3-(5-((4-hydroxypiperidin-1-yl)methyl)-6-methoxypyridin-2-yl)phenyl)-6-methoxy-[2,4'-bipyridin]-5-yl)methyl)amino)piperidin-1-yl)ethan-1-one.

9. A pharmaceutical composition comprising at least one compound of any one of claims 1-8 and at least one pharmaceutically acceptable carrier,
preferably further comprising at least one additional agent that treats, ameliorates, and/or prevents hepatitis virus infection,
more preferably wherein the at least one additional agent comprises at least one selected from the group consisting of reverse transcriptase inhibitor; capsid inhibitor; cccDNA formation inhibitor; RNA destabilizer; oligomeric nucleotide targeted against the HBV genome; immunostimulatory; GalNAc-siRNA conjugate targeted against an HBV gene transcript; and therapeutic vaccine.

10. The pharmaceutical composition of claim 9, wherein the immunostimulator is a checkpoint inhibitor, preferably wherein the checkpoint inhibitor is a PD-L1 inhibitor.

11. The pharmaceutical composition of any claim 9 or 10, wherein the hepatitis virus is at least one selected from the group consisting of hepatitis B virus (HBV) and hepatitis D virus (HDV).

12. The compound of any one of claims 1-8 and/or the pharmaceutical composition of claim 10 or 11, or a salt, solvate, stereoisomer, tautomer, or any mixtures thereof for use in treating, ameliorating, and/or preventing hepatitis B virus (HBV) infection in a subject, preferably wherein the subject is a mammal, more preferably wherein the mammal is a human, and most preferably wherein the subject is further infected with hepatitis D virus (HDV).

13. The compound for use according to claim 12, wherein the subject is further administered at least one additional agent useful for treating the hepatitis virus infection, preferably wherein the at least one additional agent comprises at least one selected from the group consisting of reverse transcriptase inhibitor; capsid inhibitor; cccDNA formation inhibitor; RNA destabilizer; oligomeric nucleotide targeted against the HBV genome; immunostimulator; GalNAc-siRNA conjugate targeted against an HBV gene transcript; and therapeutic vaccine, more preferably wherein the immunostimulatory is a checkpoint inhibitor, even more preferably wherein the checkpoint inhibitor is a PD-L1 inhibitor, and most preferably wherein the subject is co-administered the at least one compound and the at least one additional agent, and wherein the at least one compound and the at least one additional agent are optionally coformulated.

14. The compound of any one of claims 1-8 and/or the pharmaceutical composition of claim 10 or 11, or a salt, solvate, stereoisomer, tautomer, or any mixtures thereof for use in treating, ameliorating, and/or preventing cancer in a subject,
preferably wherein the subject is a mammal, more preferably wherein the mammal is a human,
prefererably wherein the cancer is amenable to treatment by inhibiting PD-1, PD-L1, or the PD-1/PD-L1 interaction,
preferably wherein the cancer is at least one of pancreatic cancer, bladder cancer, colorectal cancer, breast cancer, prostate cancer, renal cancer, hepatocellular cancer, lung cancer, ovarian cancer, cervical cancer, gastric cancer, esophageal cancer, head and neck cancer, melanoma, neuroendocrine cancer, CNS cancer, brain cancer, bone cancer, soft tissue sarcoma, non-small cell lung cancer, small-cell lung cancer, or colon cancer, and most preferably wherein the cancer is at least one of acute lymphocytic leukemia (ALL), acute myeloid leukemia (AML), chronic lymphocytic leukemia (CLL), small lymphocytic lymphoma (SLL), myelodysplastic syndrome (MDS), myeloproliferative disease (MPD), chronic myeloid leukemia (CML), multiple myeloma (MM), non-Hodgkin's lymphoma (NHL), mantle cell lymphoma (MCL), follicular lymphoma, Waldestrom's macroglobulinemia (WM), T-cell lymphoma, B-cell lymphoma and diffuse large B-cell lymphoma (DLBCL).

15. The compound for use according to claim 14, wherein
(a) the compound or composition is the only anticancer agent administered to the subject, or
(b) the subject is further administered at least one additional agent or therapy useful for treating, ameliorating, and/or preventing the cancer,
preferably wherein the additional anticancer agent or therapy comprises nivolumab, pembrolizumab, atezolizumab, ipilimumab, chemotherapy, radiation therapy, and/or resection therapy,
preferably wherein the additional anticancer agent or therapy comprises Rituxan, doxorubicin, gemcitabine, nivolumab, pembrolizumab, and/or pilimumab,
preferably wherein the compound or composition is coformulated and/or co-administered with the at least one additional agent.
